# EUROPEAN PATENT APPLICATION

(11) **EP 1 074 617 A2**
(43) Date of publication of application: **07.02.2001**
(21) Application number: 00116126.4
(22) Date of filing: 28.07.2000
(51) Int. Cl.: C12N 15/12, C12N 15/11, C07K 14/47, C07K 16/18, C12Q 1/68

(54) **Primers for synthesising full-length cDNA and their use**

(30) Priority: 29.07.1999 JP 24803699; 27.08.1999 JP 30025399; 11.01.2000 JP 2000118776; 02.05.2000 JP 2000183767; 09.06.2000 JP 2000241899
(71) Applicant: Helix Research Institute, Kisarazu-shi, Chiba 292-0812 (JP)
(72) Inventor: Ota, Toshio, Fujisawa-shi, Kanagawa 251-0042 (JP); Isogai, Takao, Inashiki-gun, Ibaraki 300-0303 (JP); Nishikawa, Tetsuo, Itabashi-ku, Tokyo 173-0013 (JP); Hayashi, Kohji, Ichihara-shi, Chiba 292-0056 (JP); Saito, Kaoru, Kisarazu-shi, Chiba 292-0056 (JP); Yamamoto, Junichi, Kisarazu-shi, Chiba 292-0041 (JP); Ishii, Shizuko, Kisarazu-shi, Chiba 292-0812 (JP); Sugiyama, Tomoyasu, Kisarazu-shi, Chiba 292-0045 (JP); Wakamatsu, Ai, Kisarazu-shi, Chiba 292-0014 (JP); Nagai, Keiichi, Higashiyamato-shi, Tokyo 207-0022 (JP); Otsuki, Tetsuji, Kisarazu-shi, Chiba 292-0045 (JP)
(74) Representative: VOSSIUS & PARTNER

(57) **Abstract**

Primers for synthesizing full-length cDNAs and their use are provided.

5602 cDNA encoding a human protein has been isolated and nucleotide sequences of 5'-, and 3' -ends of the cDNA have been determined. Furthermore, primers for synthesizing the full-length cDNA have been provided to clarify the function of the protein encoded by the cDNA. The full-length cDNA of the present invention containing the translation start site provides information useful for analyzing the functions of the protein.

## Description

### FIELD OF THE INVENTION

The present invention relates to a polynucleotide encoding a novel protein, a protein encoded by the polynucleotide, and new uses of these.

### BACKGROUND OF THE INVENTION

Currently, the sequencing projects, the determination and analysis of the genomic DNA of various living organisms have been in progress all over the world. The whole genomic sequences of more than 10 species of prokaryotes, a lower eukaryote, yeast, and a multicellular eukaryote, C. elegans are already determined. As to human genome, which is supposed to be composed of three thousand million base pairs, the world wide cooperative projects have been under way to analyze it, and the whole structure is predicted to be determined by the years 2002-2003. The aim of the determination of genomic sequence is to reveal the functions of all genes and their regulation and to understand living organisms as a network of interactions between genes, proteins, cells or individuals through deducing the information in a genome, which is a blueprint of the highly complicated living organisms. To understand living organisms by utilizing the genomic information from various species is not only important as an academie subject, but also socially significant from the viewpoint of industrial application.

However, determination of genomic sequences itself cannot identify the functions of all genes. For example, as for yeast, only the function of approximately half of the 6000 genes, which is predicted based on the genomic sequence, was able to be deduced. As for human, the number of the genes is predicted to be approximately one hundred thousand. Therefore, it is desirable to establish "a high throughput analysis system of the gene functions" which allows us to identify rapidly and efficiently the functions of vast amounts of the genes obtained by the genomic sequencing.

Many genes in the eukaryotic genome are split by introns into multiple exons. Thus, it is difficult to predict correctly the structure of encoded protein solely based on genomic information. In contrast, cDNA, which is produced from mRNA that lacks introns, encodes a protein as a single continuous amino acid sequence and allows us to identify the primary structure of the protein easily. In human cDNA research, to date, more than one million ESTs (Expression Sequence Tags) are publicly available, and the ESTs presumably cover not less than 80% of all human genes.

The information of ESTs is utilized for analyzing the structure of human genome, or for predicting the exon-regions of genomic sequences or their expression profile. However, many human ESTs have been derived from proximal regions to the 3'-end of cDNA, and information around the 5'-end of mRNA is extremely little. Among these human cDNAs, the number of the corresponding mRNAs whose encoding protein sequences are deduced is approximately 7000, and further, the number of full-length therein is only 5500. Thus, even including cDNA registered as EST, the percentage of human cDNA obtained so far is estimated to be 10-15% of all the genes.

It is possible to identify the transcription start site of mRNA on the genomic sequence based on the 5'-end sequence of a full-length cDNA, and to analyze factors involved in the stability of mRNA that is contained in the cDNA, or in its regulation of expression at the translation stage. Also, since a full-length cDNA contains ATG, the translation start site, in the 5'-region, it can be translated into a protein in a correct frame. Therefore, it is possible to produce a large amount of the protein encoded by the cDNA or to analyze biological activity of the expressed protein by utilizing an appropriate expression system. Thus, analysis of a full-length cDNA provides valuable information which complements the information from genome sequencing. Also, full-length cDNA clones that can be expressed are extremely valuable in empirical analysis of gene function and in industrial application.

Therefore, if a novel human full-length cDNA is isolated, it can be used for developing medicines for diseases in which the gene is involved. The protein encoded by the gene can be used as a drug by itself. Thus, it has great significance to obtain a full-length cDNA encoding a novel human protein.

In particular, human secretory proteins or membrane proteins would be useful by itself as a medicine like tissue plasminogen activator (TPA), or as a target of medicines like membrane receptors. In addition, genes for signal transduction-associated proteins (protein kinases, etc.), glycoprotein-associated proteins, transcription-associated proteins, etc. are genes whose relationships to human diseases have been elucidated. Moreover, genes for disease-associated proteins form a gene group rich in genes whose relationships to human diseases have been elucidated.

Therefore, it has great significance to isolate novel full-length cDNA clones of human, only few of which has been isolated. Especially, isolation of a novel cDNA clone encoding a secretory protein or membrane protein is desired since the protein itself would be useful as a medicine, and also the clones potentially include a gene associated with diseases. In addition, genes encoding proteins that are associated with signal transduction, glycoprotein, transcription, or diseases are expected to be useful as target molecules for therapy, or as medicines themselves. These genes form a gene group predicted to be strongly associated with diseases. Thus, identification of the full-length cDNA clones encoding those proteins has great significance.

### SUMMARY OF THE INVENTION

An objective of the present invention is to provide a polynucleotide encoding a novel protein, a protein encoded by said polynucleotide, and novel usages of these.

The inventors have developed a method for efficiently cloning a human full-length cDNA that is predicted by the ATGpr etc. to be a full-length cDNA clone, from a full-length-enriched cDNA library that is synthesized by the oligo-capping method. Then, the inventors determined the nucleotide sequence of the obtained cDNA clones from both 5'- and 3'- ends.

Furthermore, the inventors analyzed the obtained clones by the BLAST search of the databases, SwissProt (http://www.ebi.ac.uk/ebi_docsSwissProt_db/swisshome.html), GenBank (http://www.ncbi.nlm.nih.gov/web/GenBank), and UniGene (Human) (http://www.ncbi.nlm.nih.gov/UniGene).

The full-length cDNA clones of the present invention have high fullness ratio since these were obtained by the combination of (1) construction of a full-length-enriched cDNA library that is synthesized by the oligo-capping method, and (2) a system in which the full-length ratio is evaluated from the nucleotide sequence of the 5'-end (selection based on the ATGpr, previously removed complete sequences to ESTs). However, the primer of the present invention enables to obtain full-length cDNA easily without any specialized methods as in the described method.
Homology analysis in which the analysis is carried out against a not-full-length cDNA fragment to postulate the function of a protein encoded by said fragment, is being commonly performed.
However, since such analysis is based on the information of the fragment, it is not clear as to whether this fragment corresponds to a part that is functionally important in the protein. In other words, the reliability of the homology analysis based on the information of a fragment is doubtful, as information related to the structure of the whole protein is not available. However, the homology analysis of the present invention is conducted based on the information of a full-length cDNA comprising the whole coding region of the cDNA, and therefore, the homology of various portions of the protein can be analyzed. Hence, the reliability of the homology analysis has been dramatically improved in the present invention.

The inventors completed the invention by finding that it is possible to synthesize a novel full-length cDNA by using the combination of a primer that is designed based on the nucleotide sequence of the 5'-ends of the selected full-length cDNA clones and any of an oligo-dT primer or a 3'-primer that is designed based on the nucleotide sequence of the 3'-ends of the selected clones.

Thus, the present invention relates to primers described below, a method for synthesizing a polynucleotide using the primers, and polynucleotides obtained by the method.

First, the present invention relates to
(1) use of an oligonucleotide as a primer for synthesizing the polynucleotide comprising the nucleotide sequence set forth in any one of SEQ ID NOs: 1-5547 and SEQ ID NOs: 16111-16164, or the complementary strand thereof, wherein said oligonucleotide is complementary to said polynucleotide or the complementary strand thereof and comprises at least 15 nucleotides;
(2) a primer set for synthesizing polynucleotides, the primer set comprising an oligo-dT primer and an oligonucleotide complementary to the complementary strand of the polynucleotide comprising the nucleotide sequence set forth in any one of SEQ ID NOs: 1-5547 and SEQ ID NOs: 16111-16164, wherein said oligonucleotide comprises at least 15 nucleotides; and
(3) a primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence / 3'-end nucleotide sequence is selected from the combinations of 5'-end nucleotide sequence / 3'-end nucleotide sequence set forth in the SEQ ID NOs in Tables 1 and 2.

Tables 1 and 2 shows names of clones obtained in the examples described later, comprising the polynucleotide of the present invention (Table 1; 5547 clones, Table 2; 54 clones), names of nucleotide sequences at the 5'-end and 3'-end of the full-length cDNA, and their corresponding SEQ ID NOs. A blank indicates that the 3'-end sequence corresponding to the 5'-end sequence has not been determined for the same clone.

The SEQ ID NO of a 5'-end sequence is shown on the right side of the name of the 5'-end sequence, and the SEQ ID NO of a 3'-end sequence is shown on the right side of the name of the 3'-end sequence.

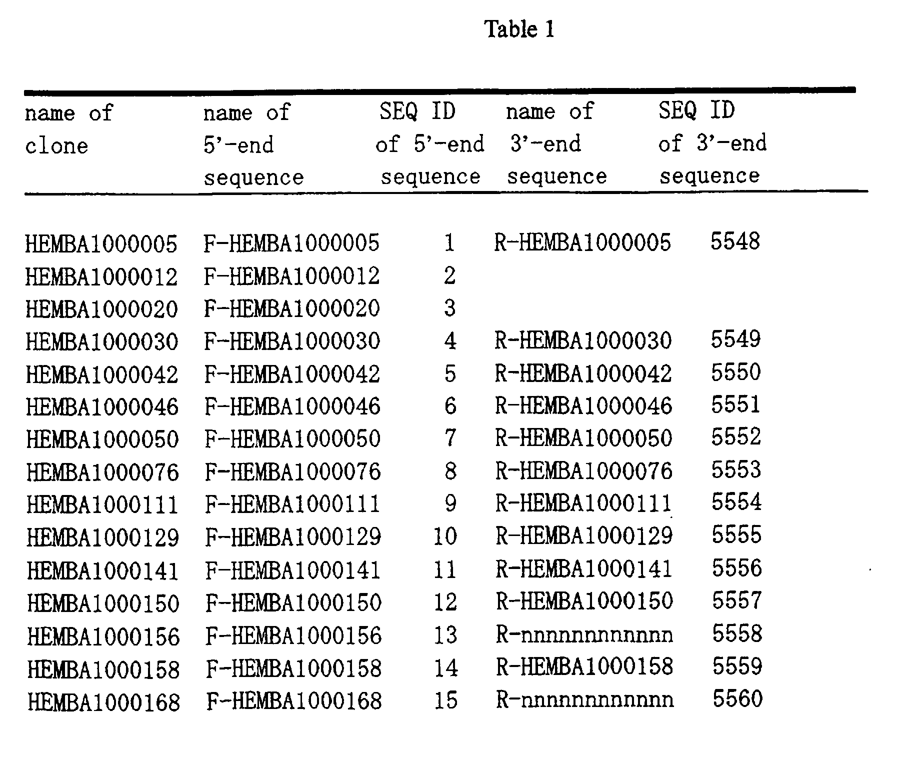

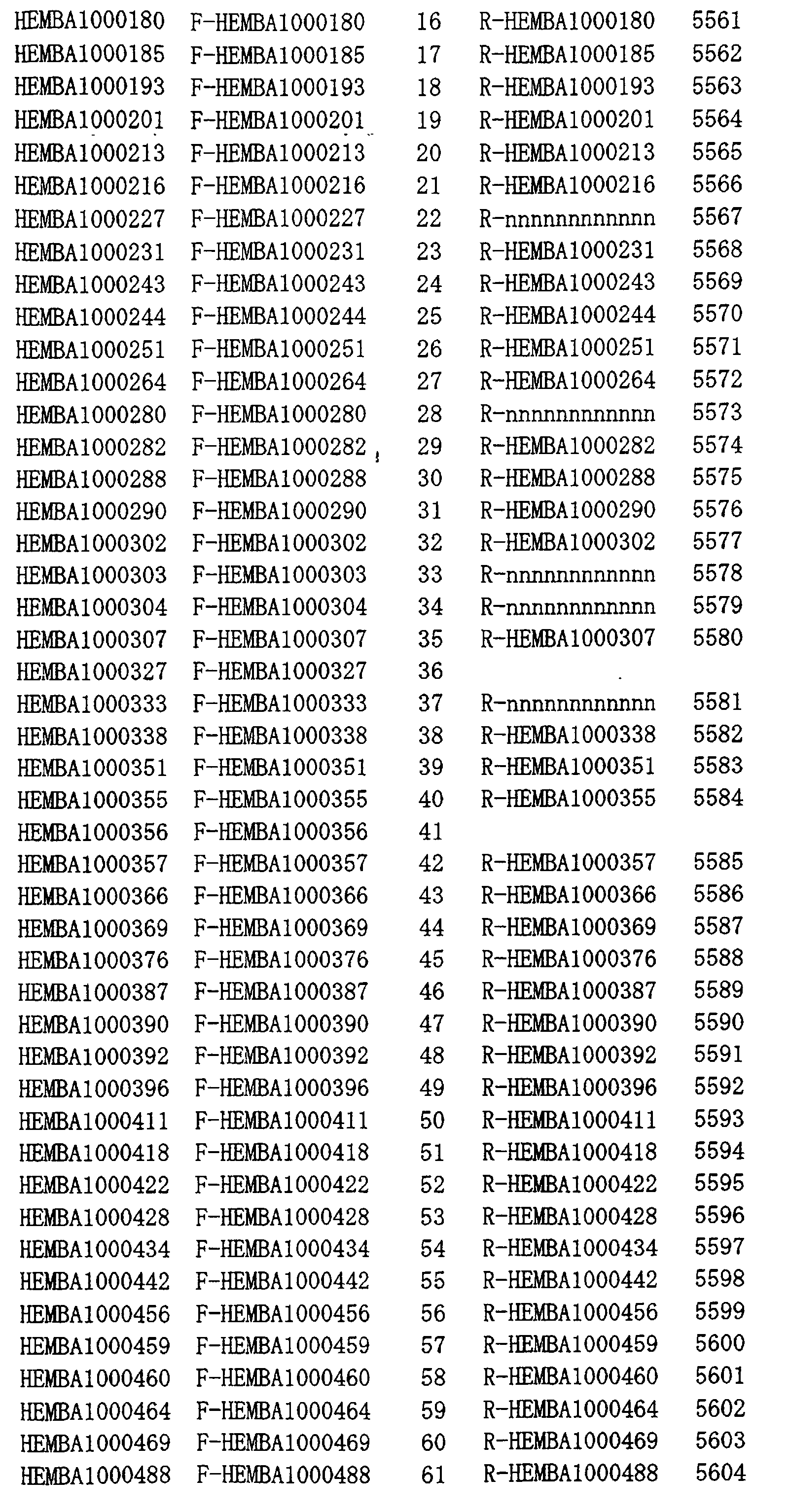

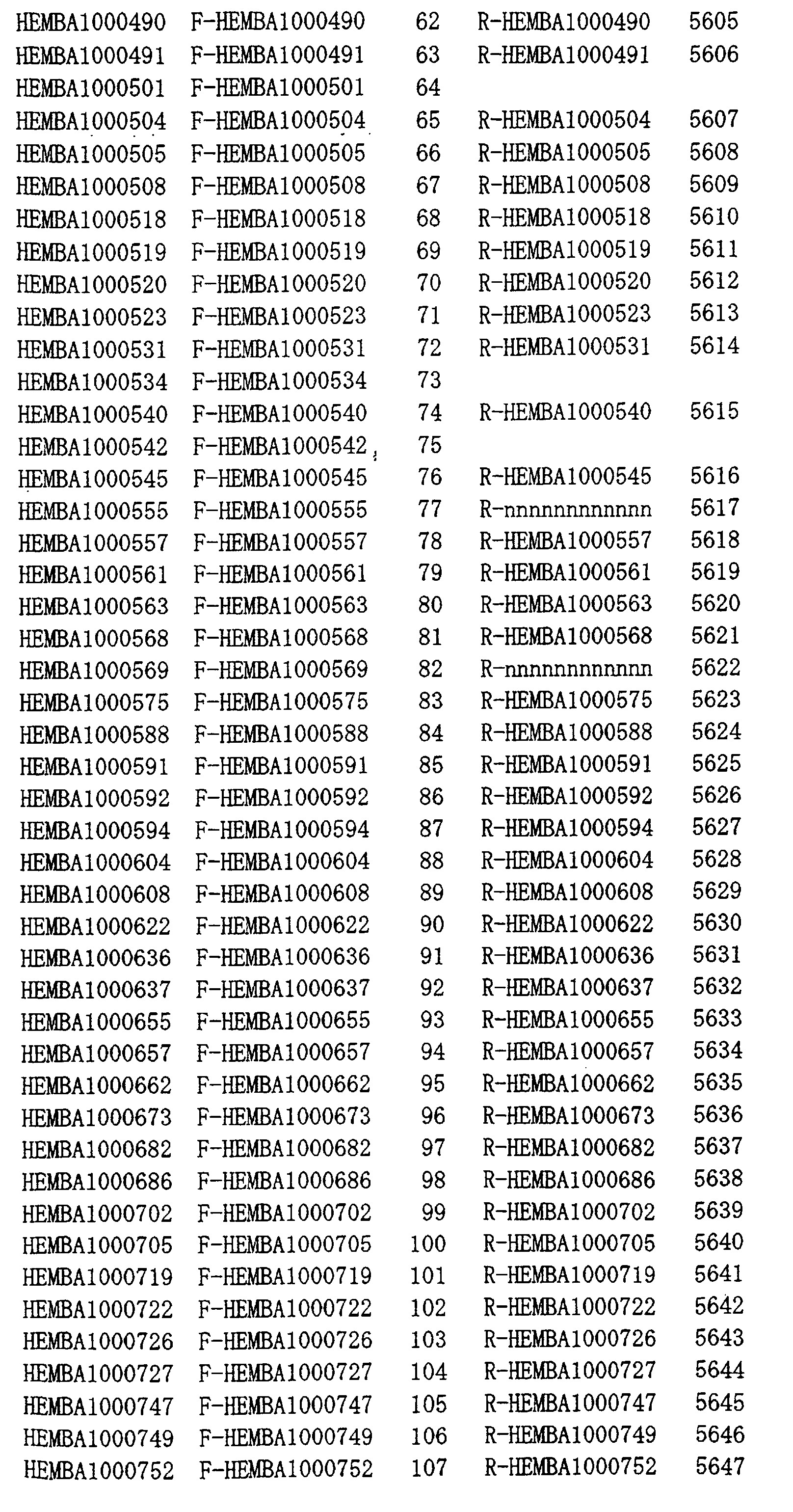

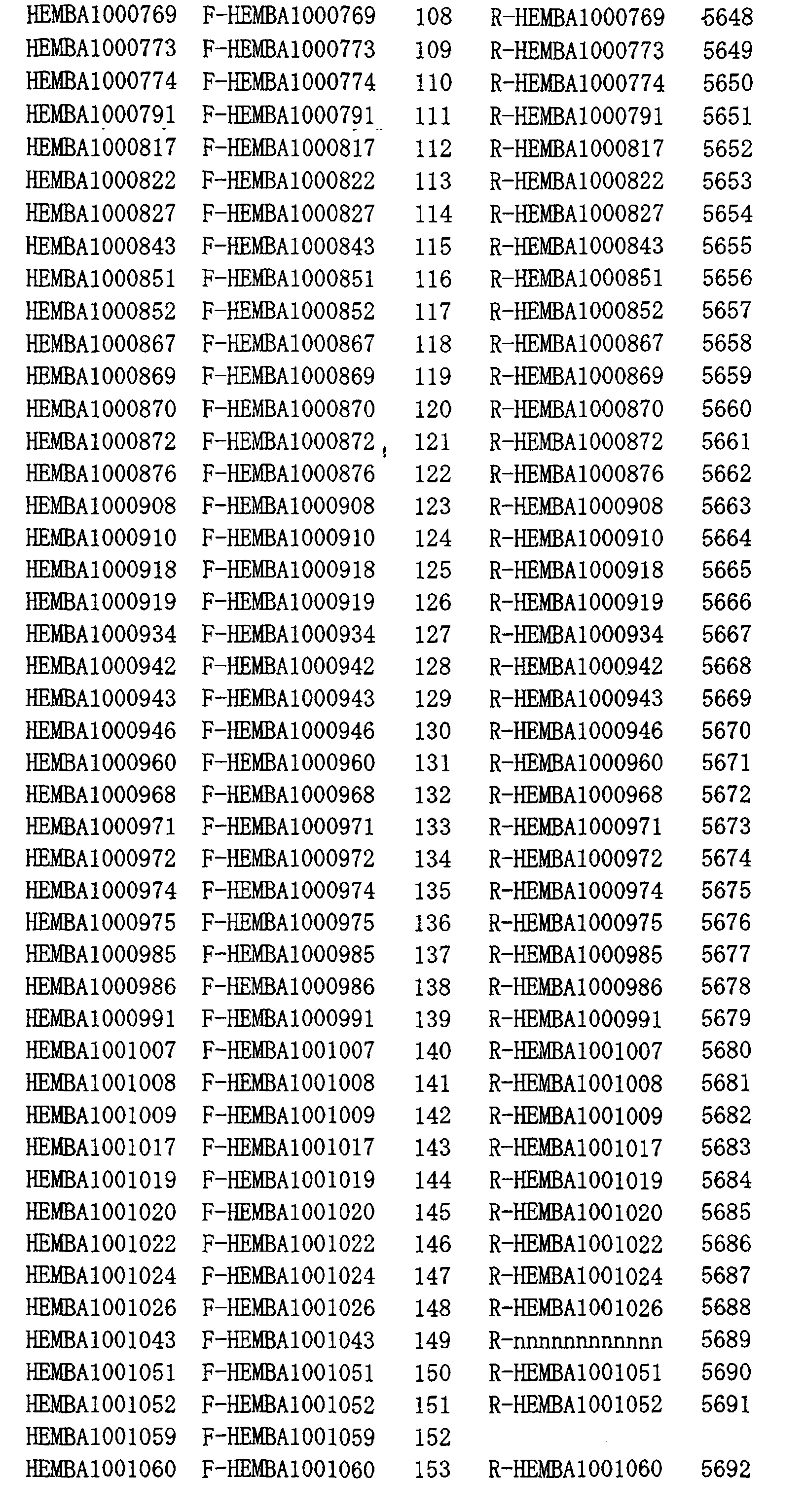

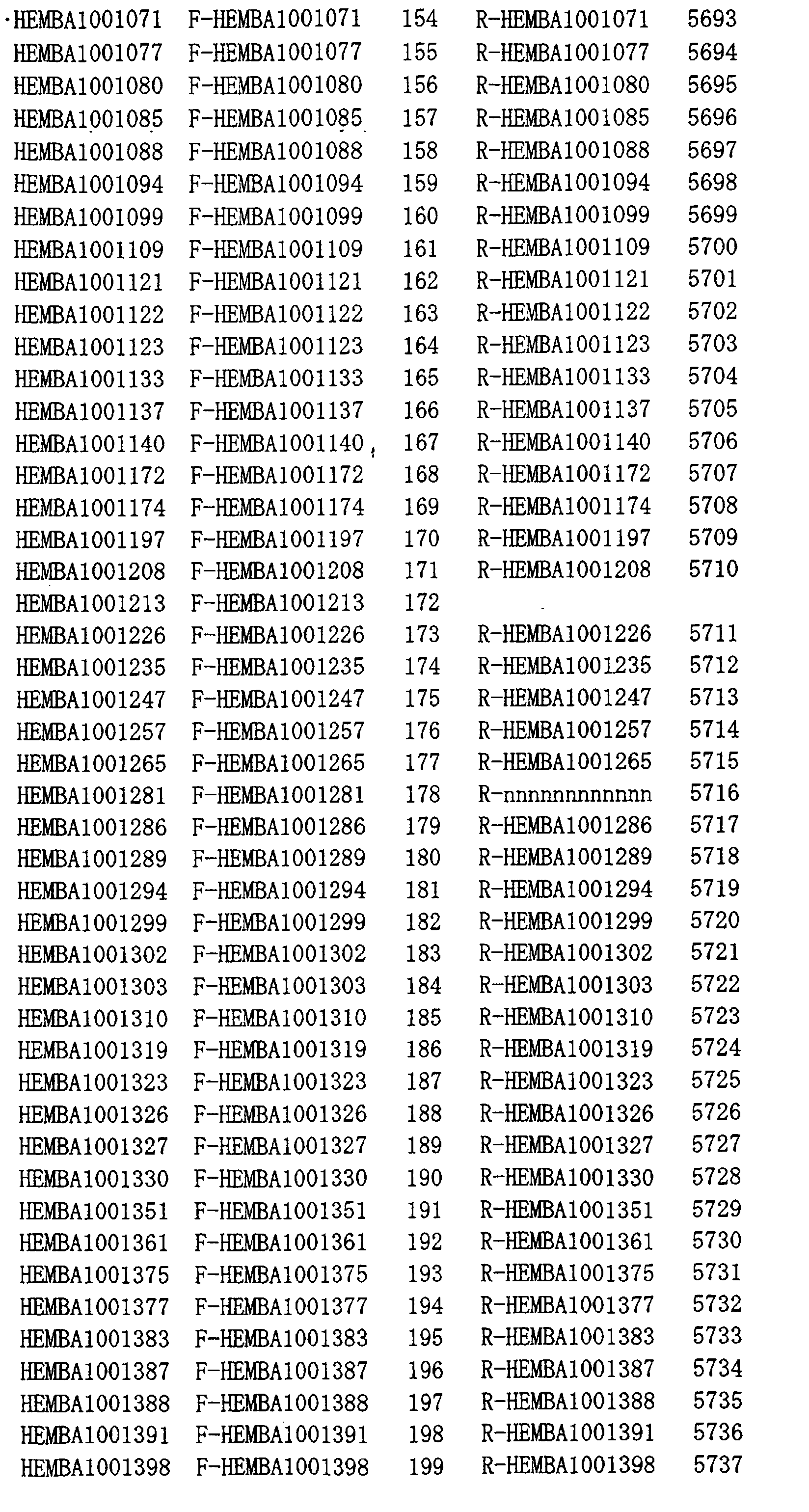

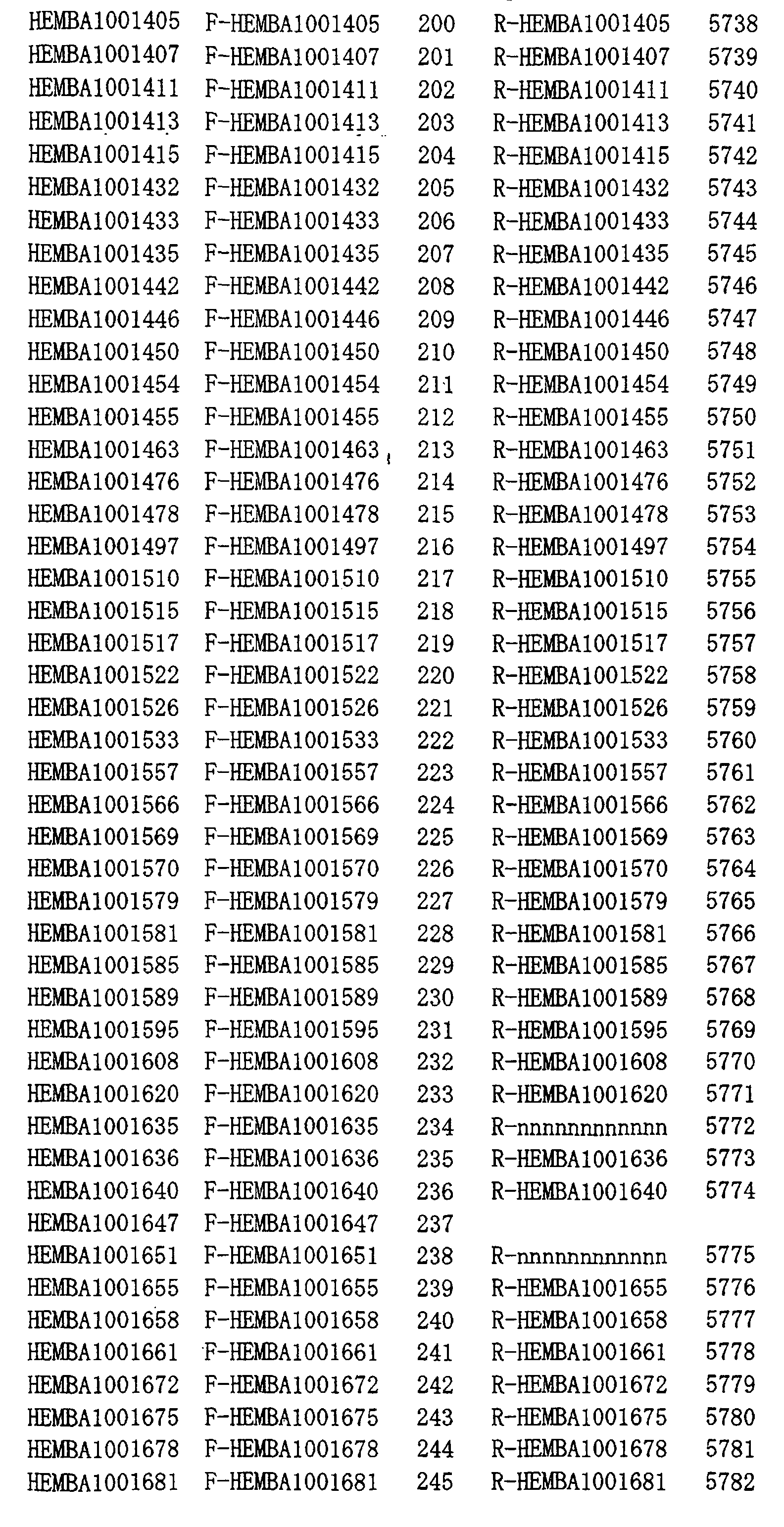

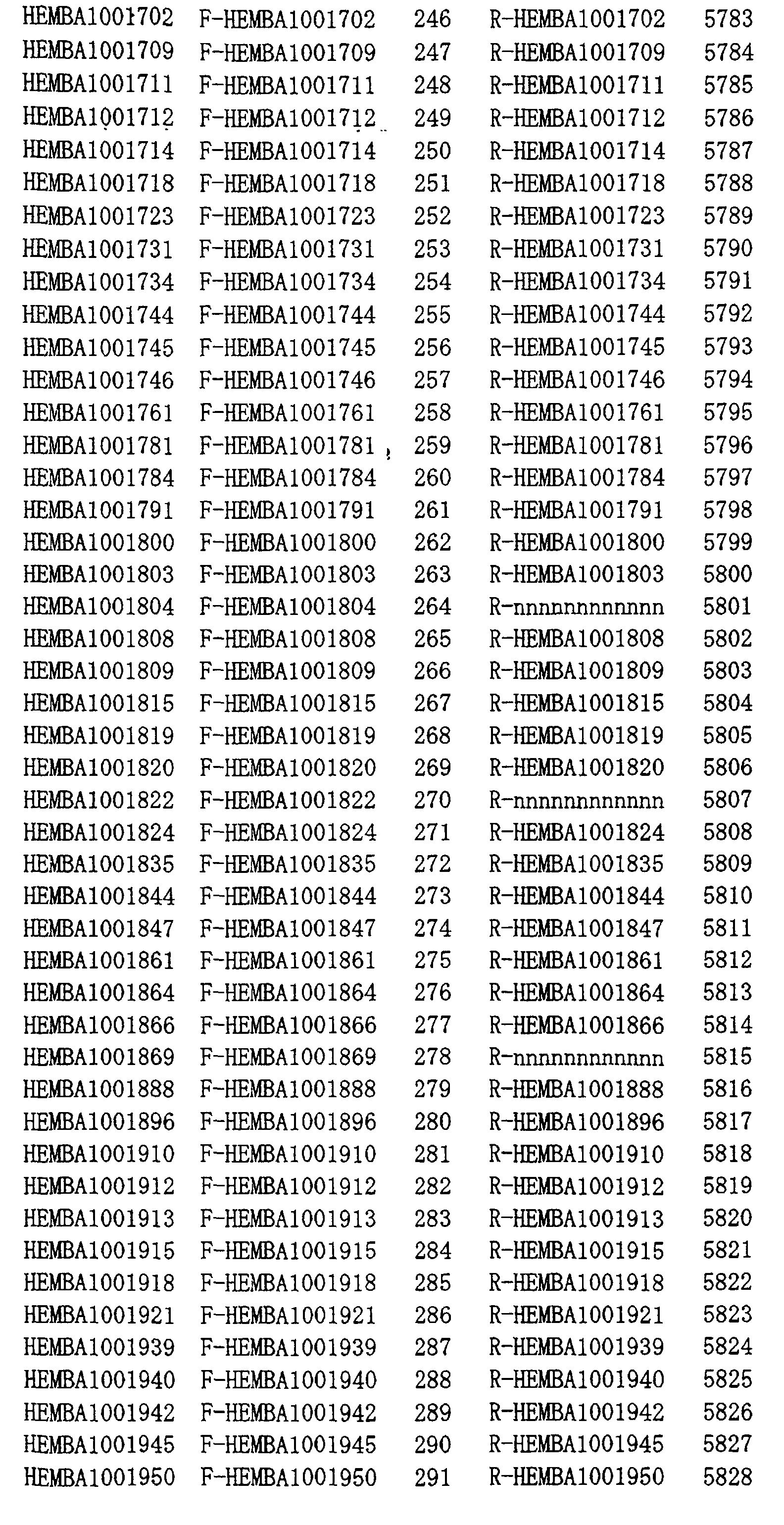

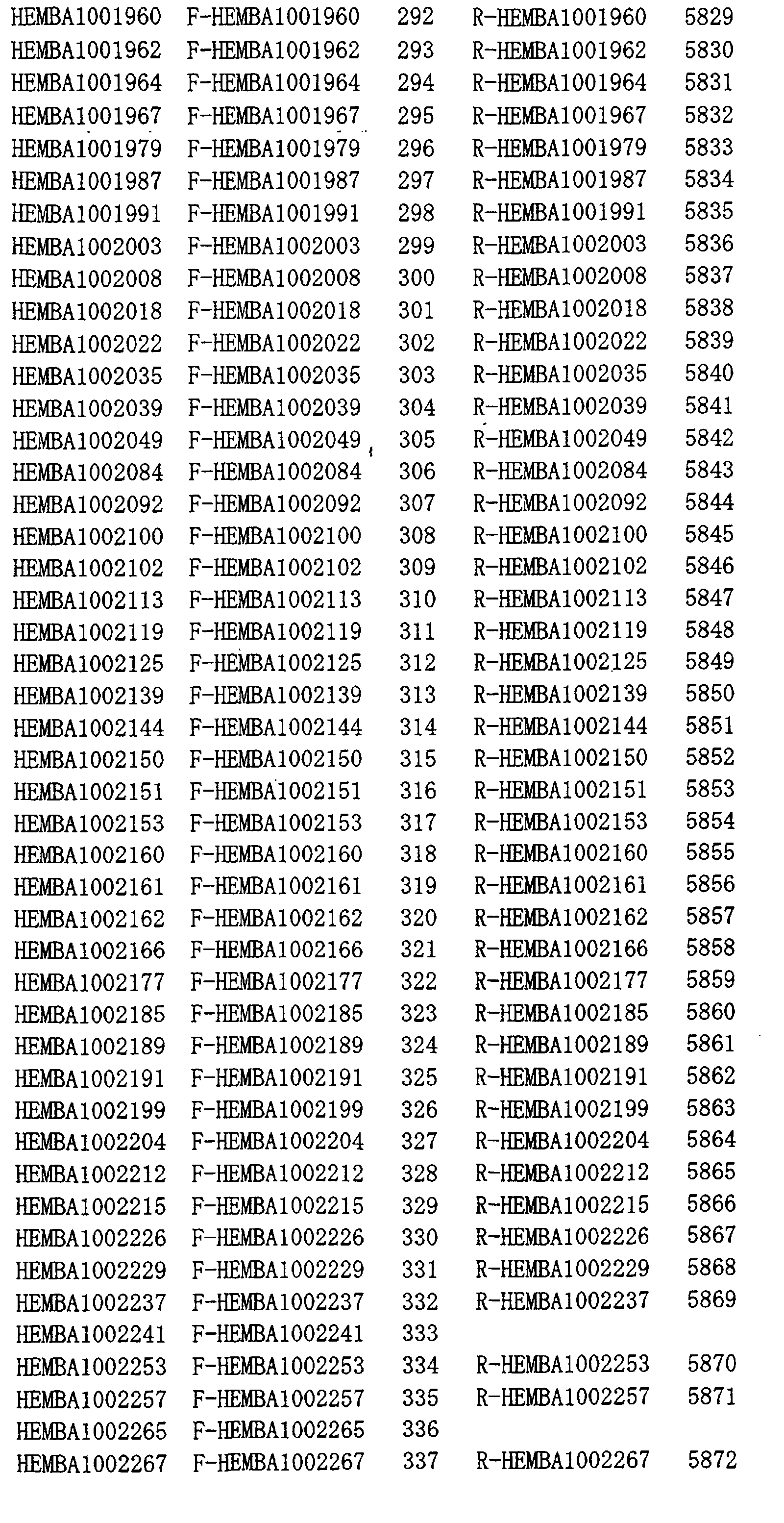

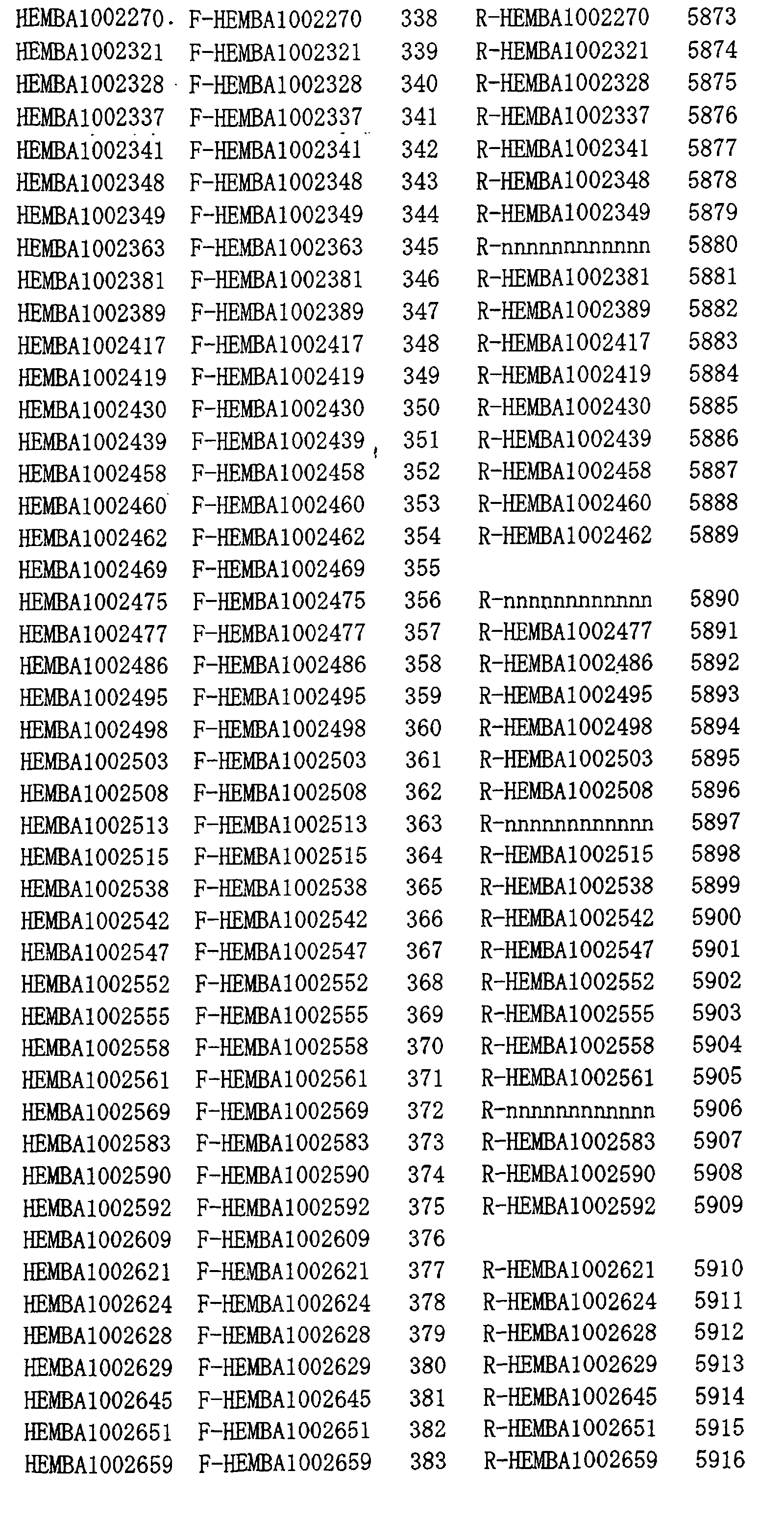

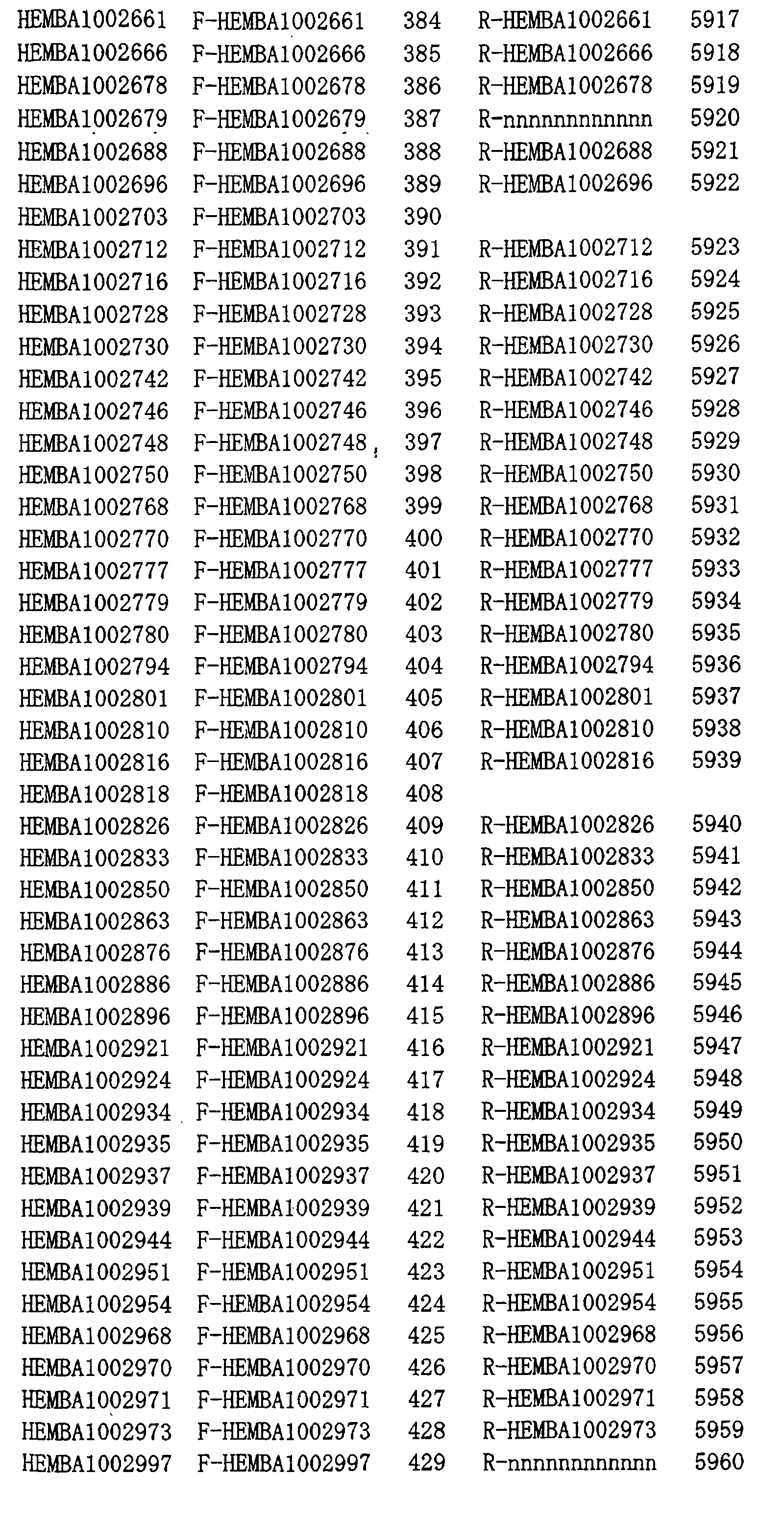

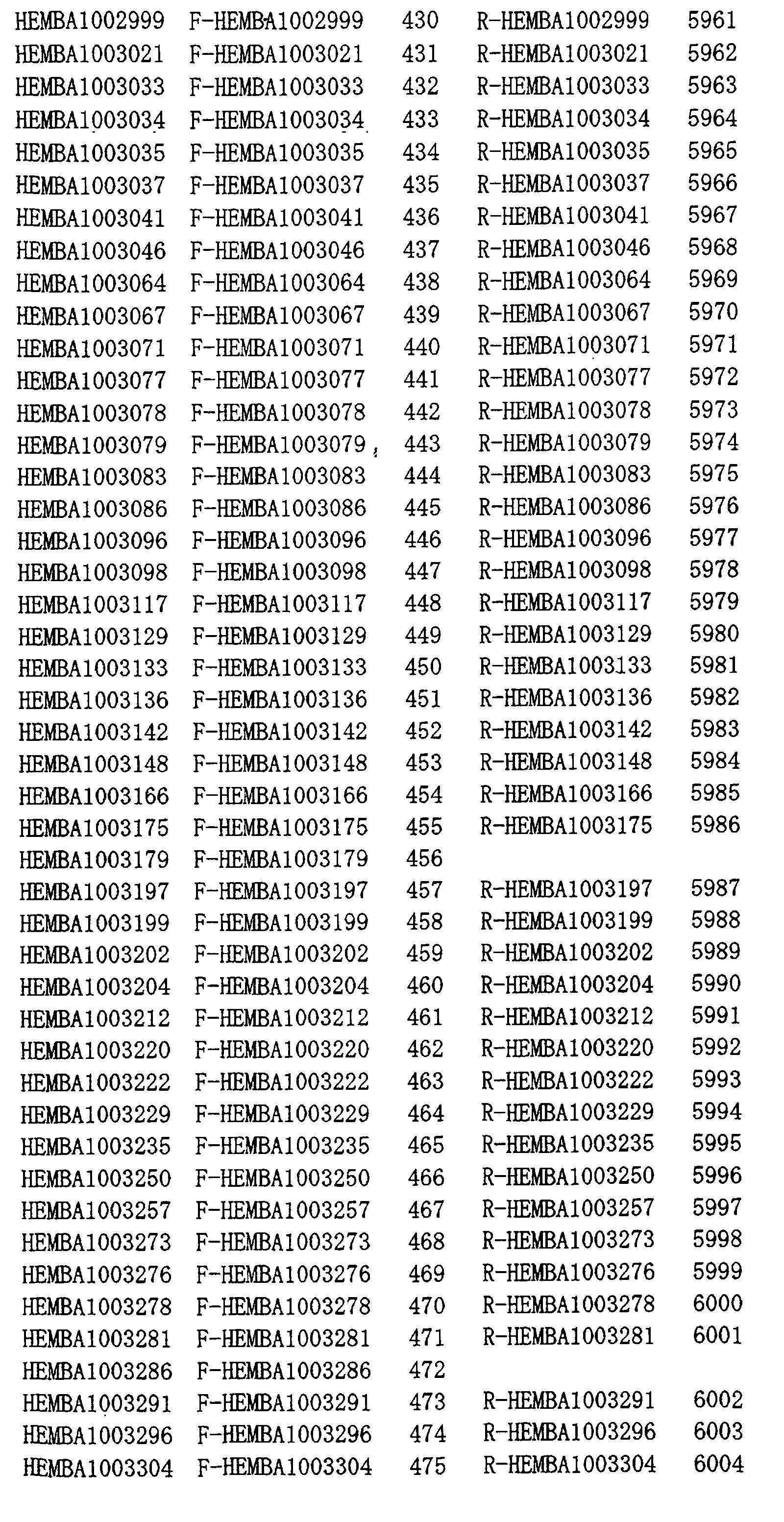

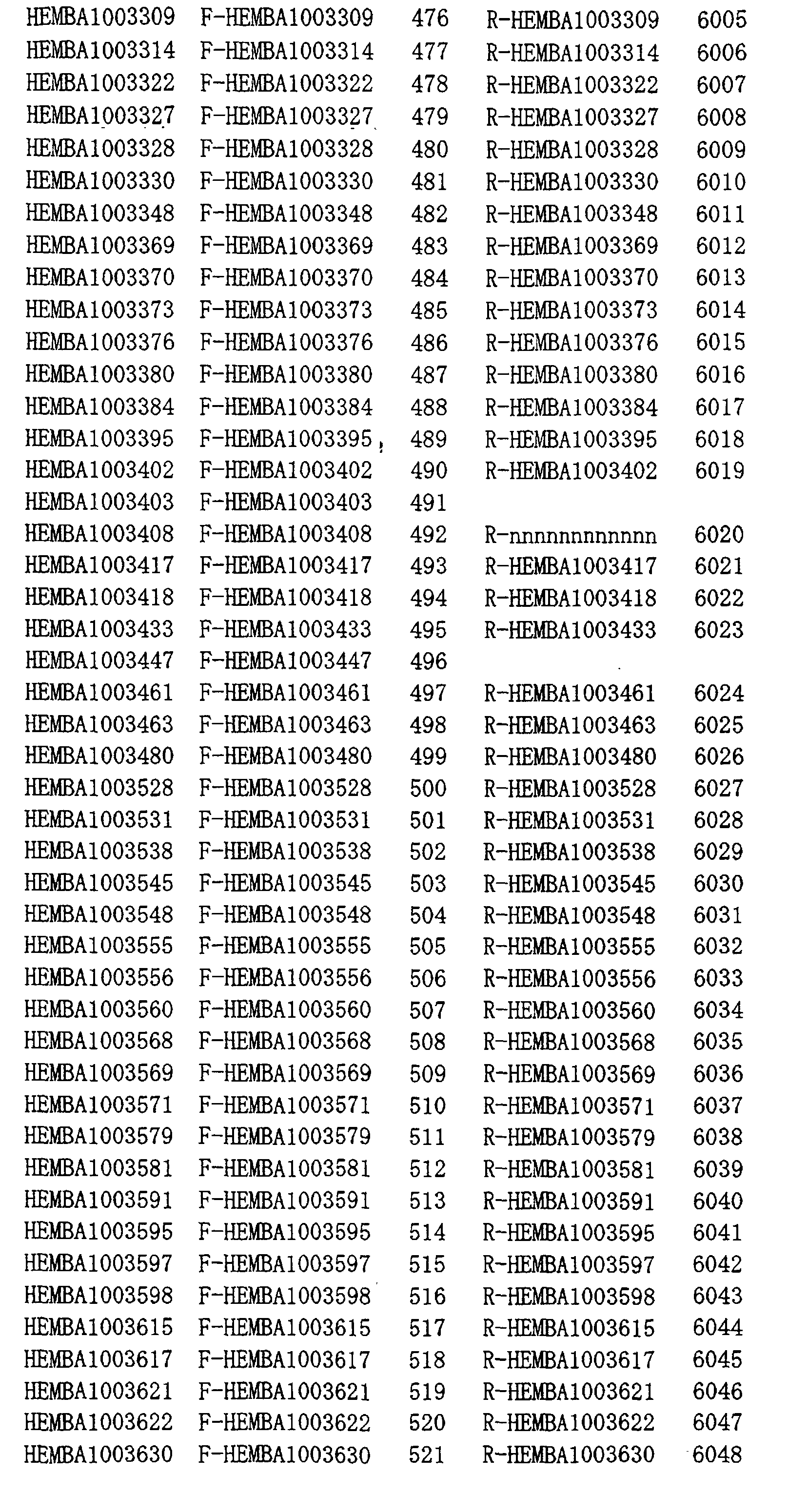

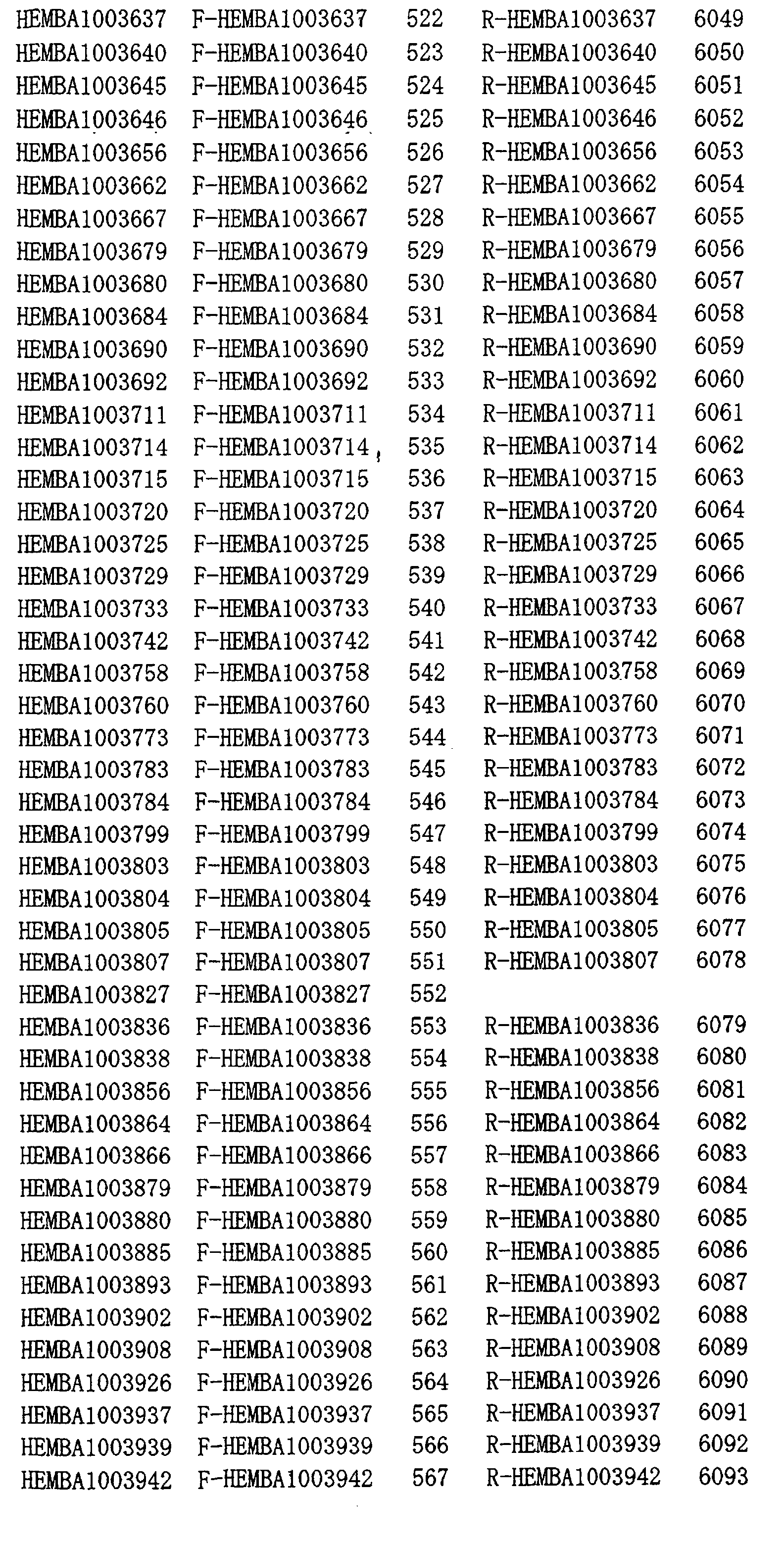

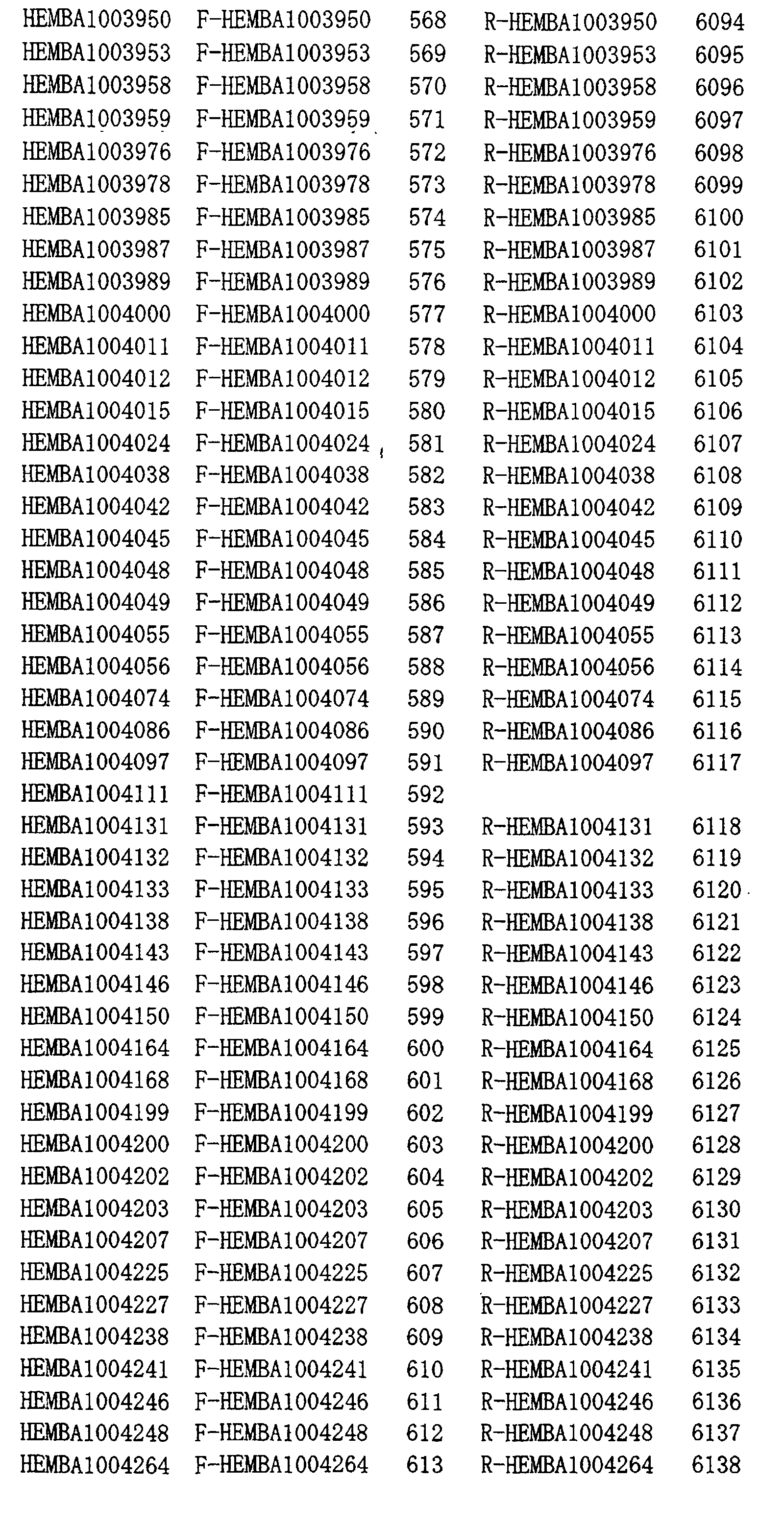

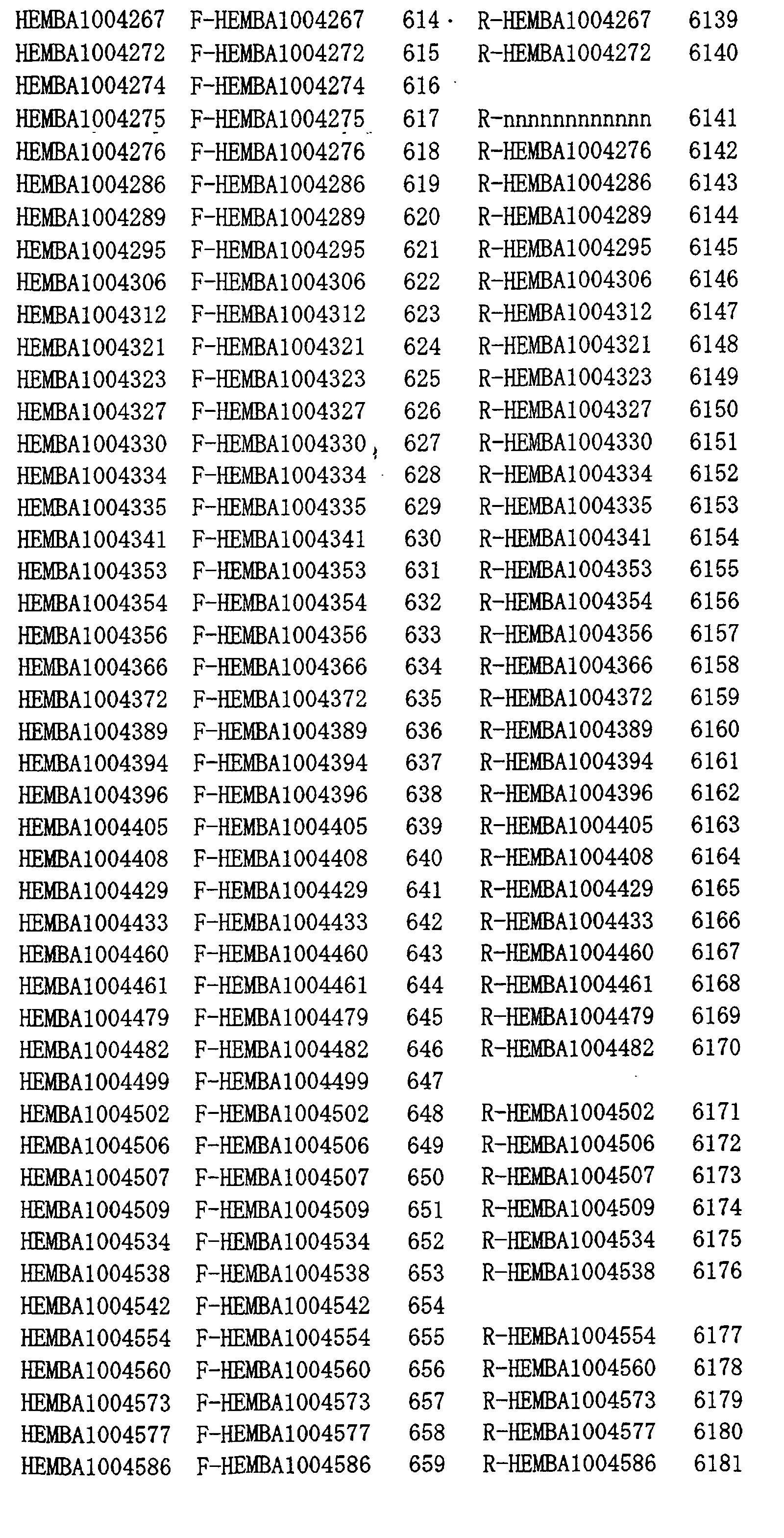

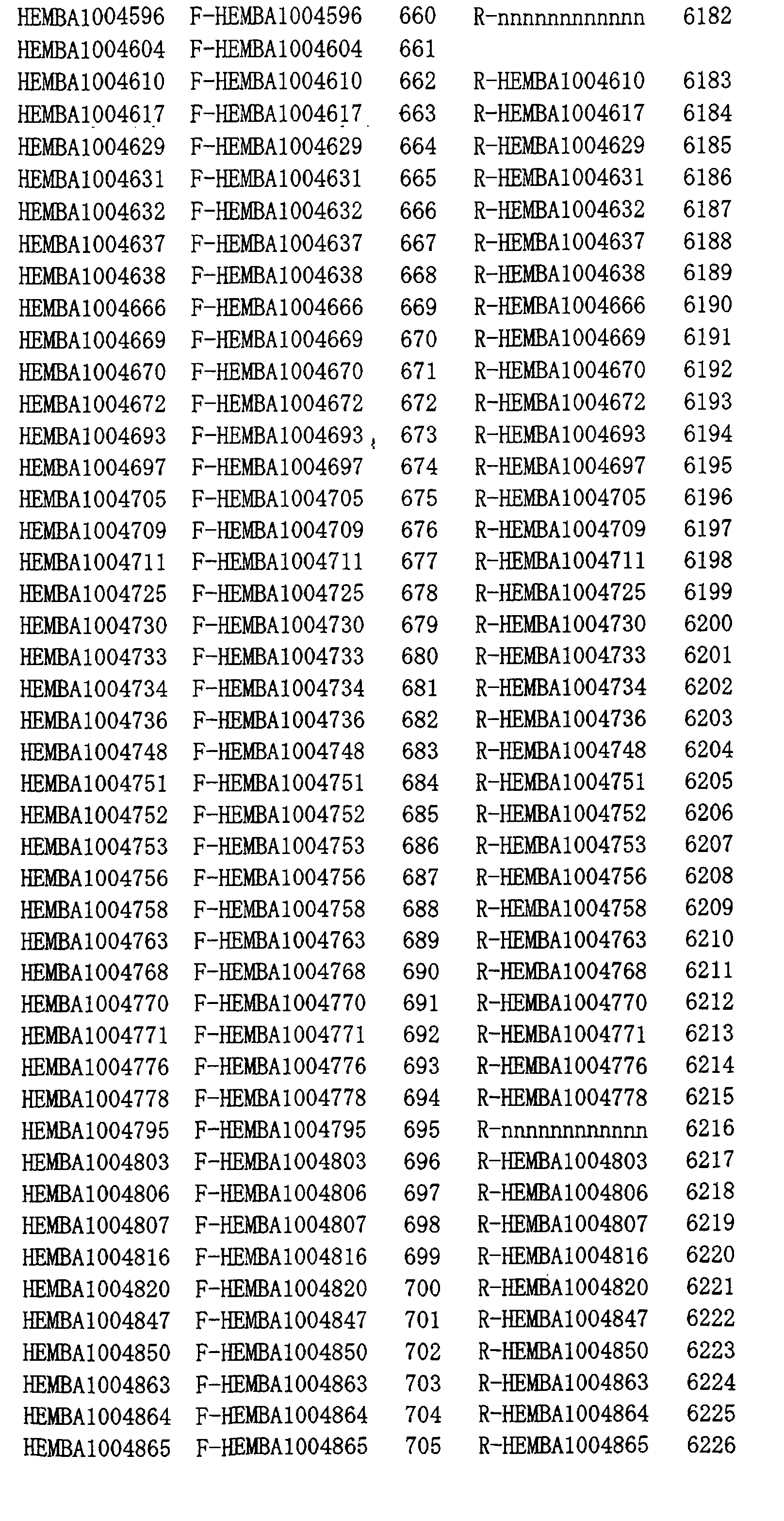

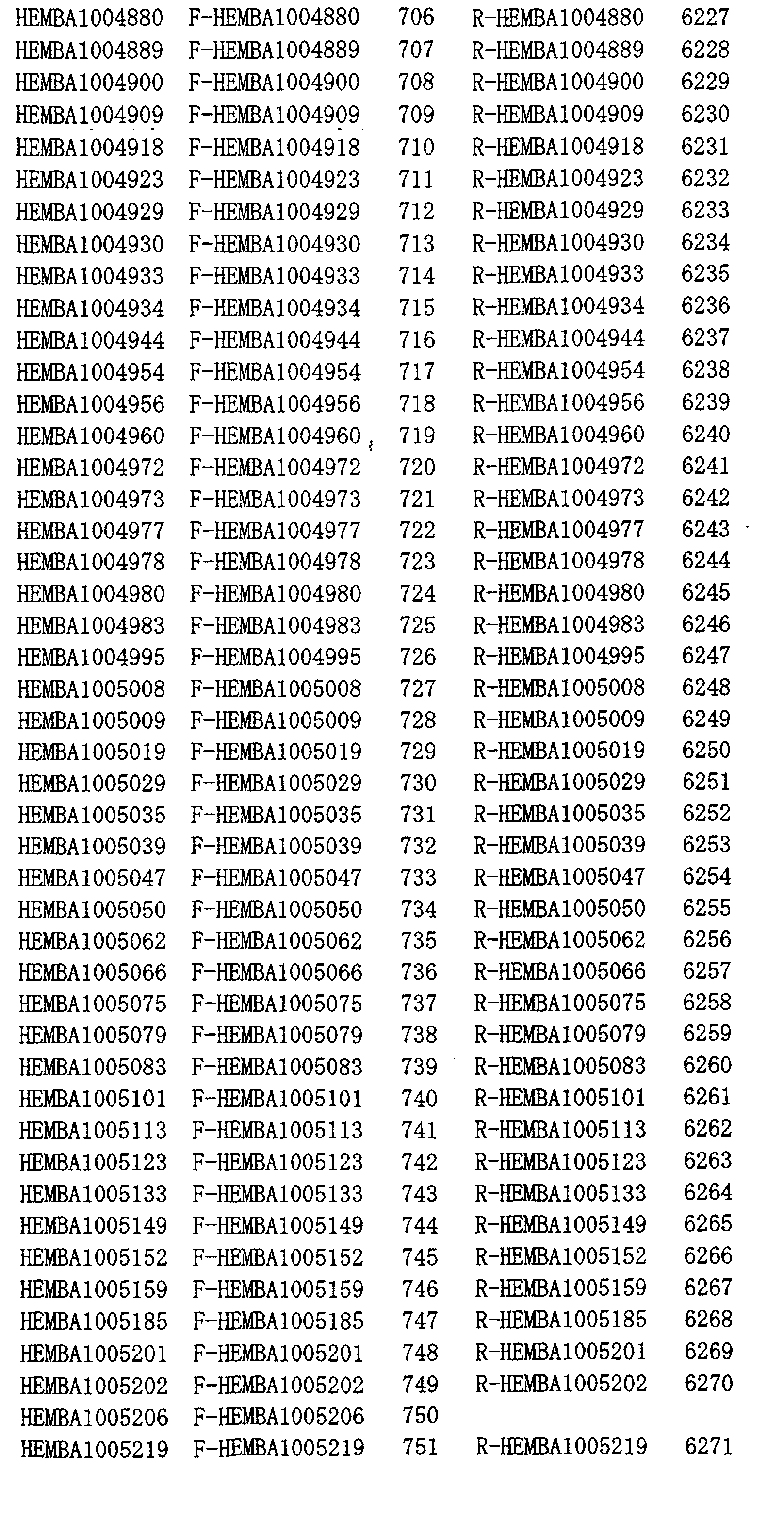

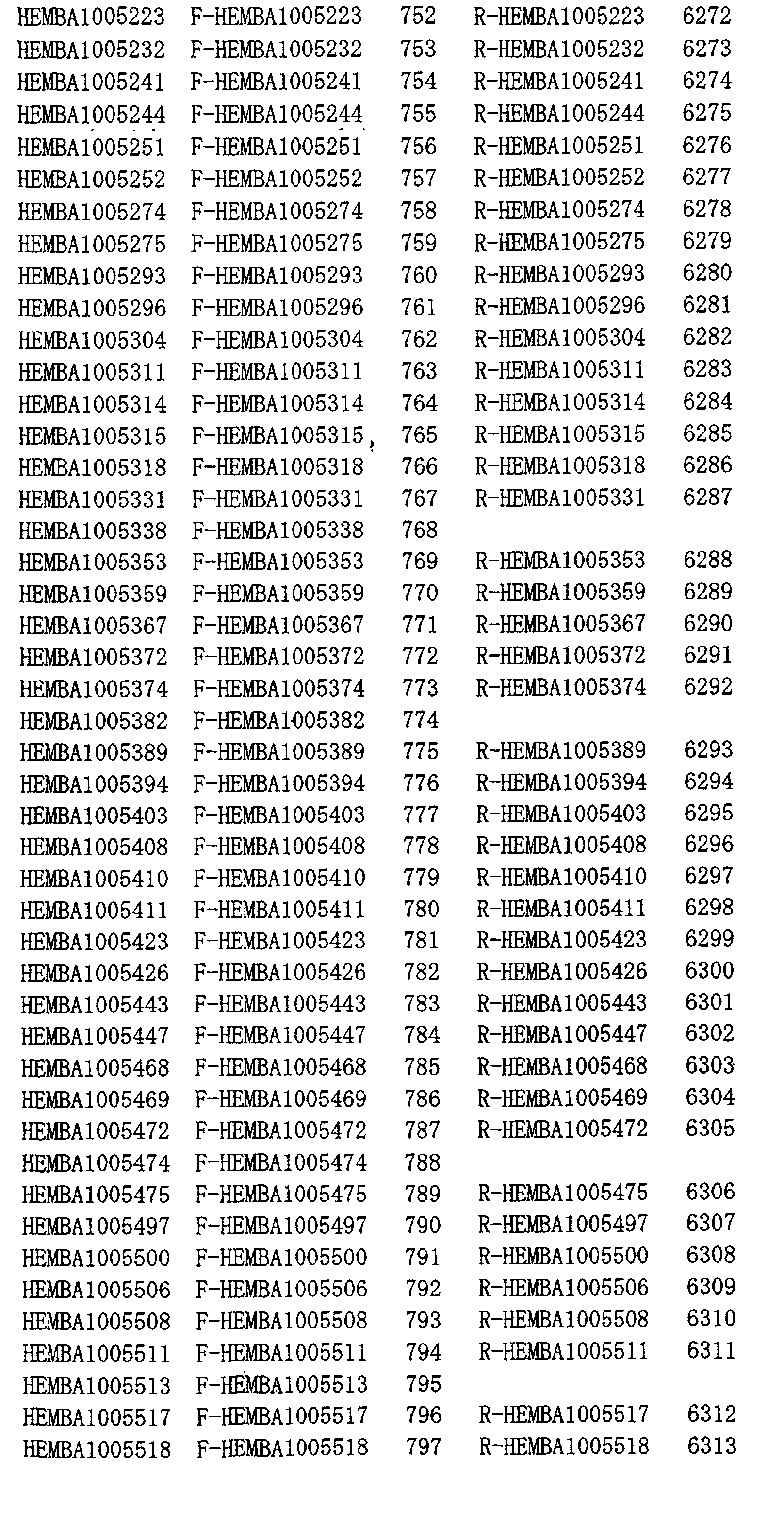

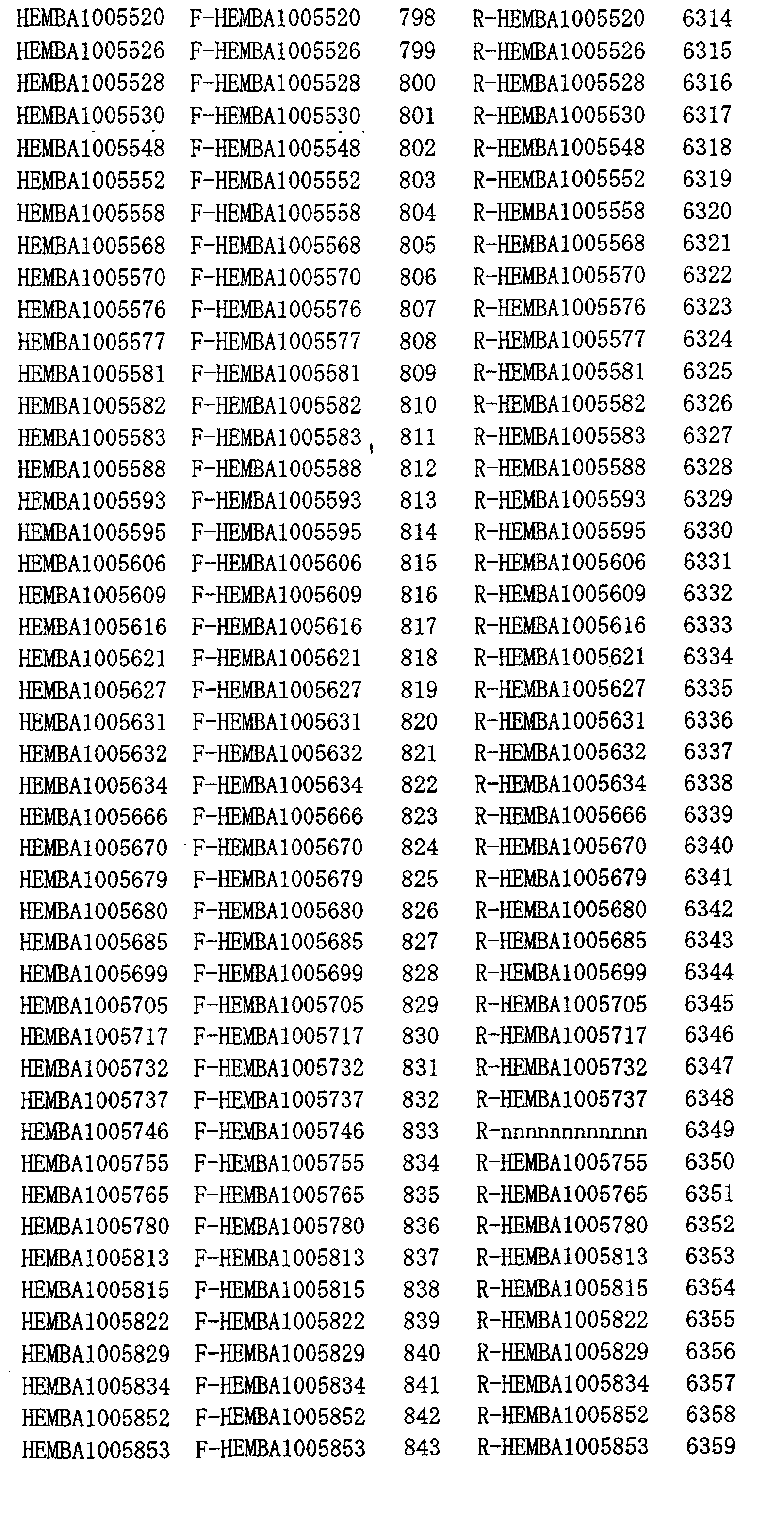

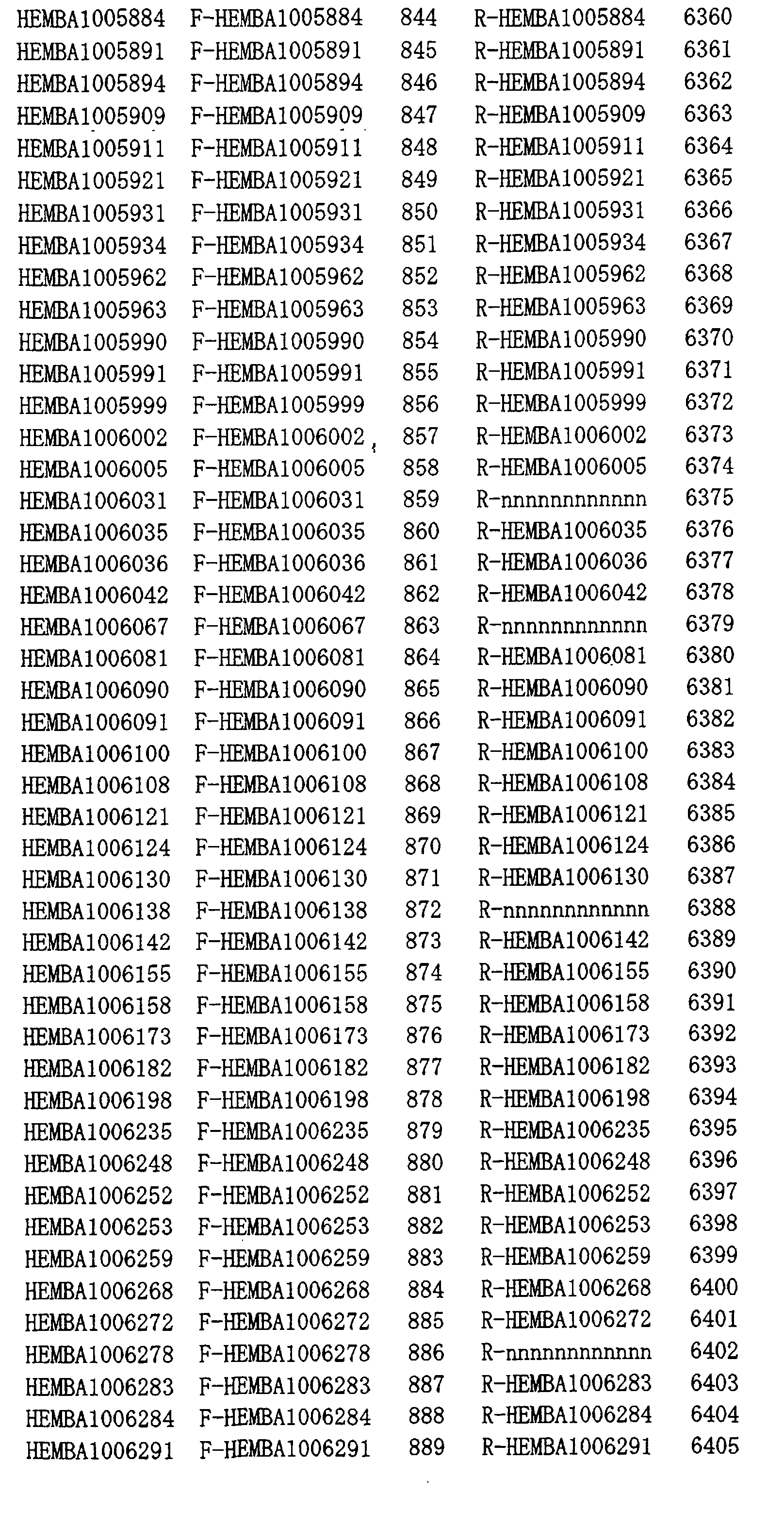

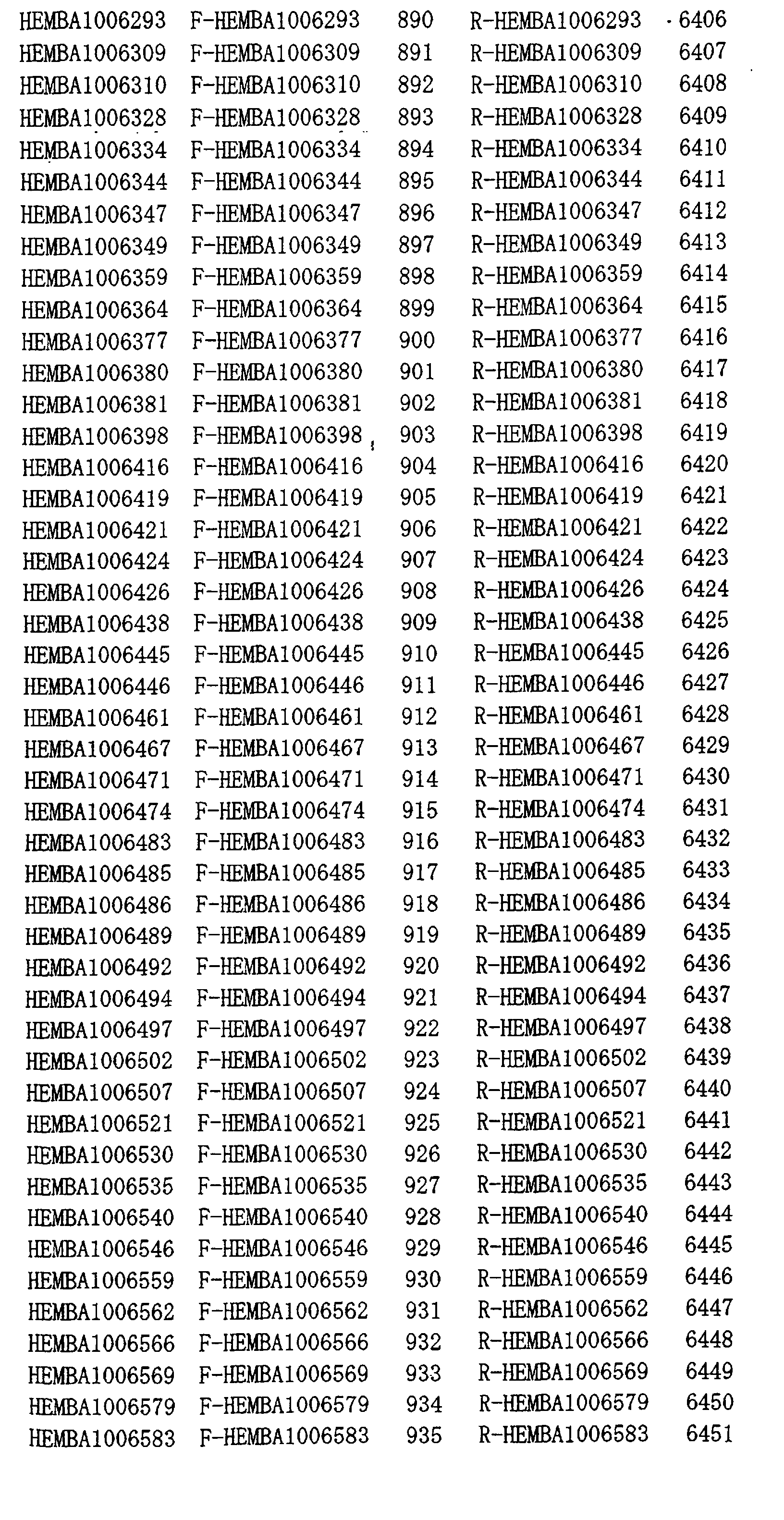

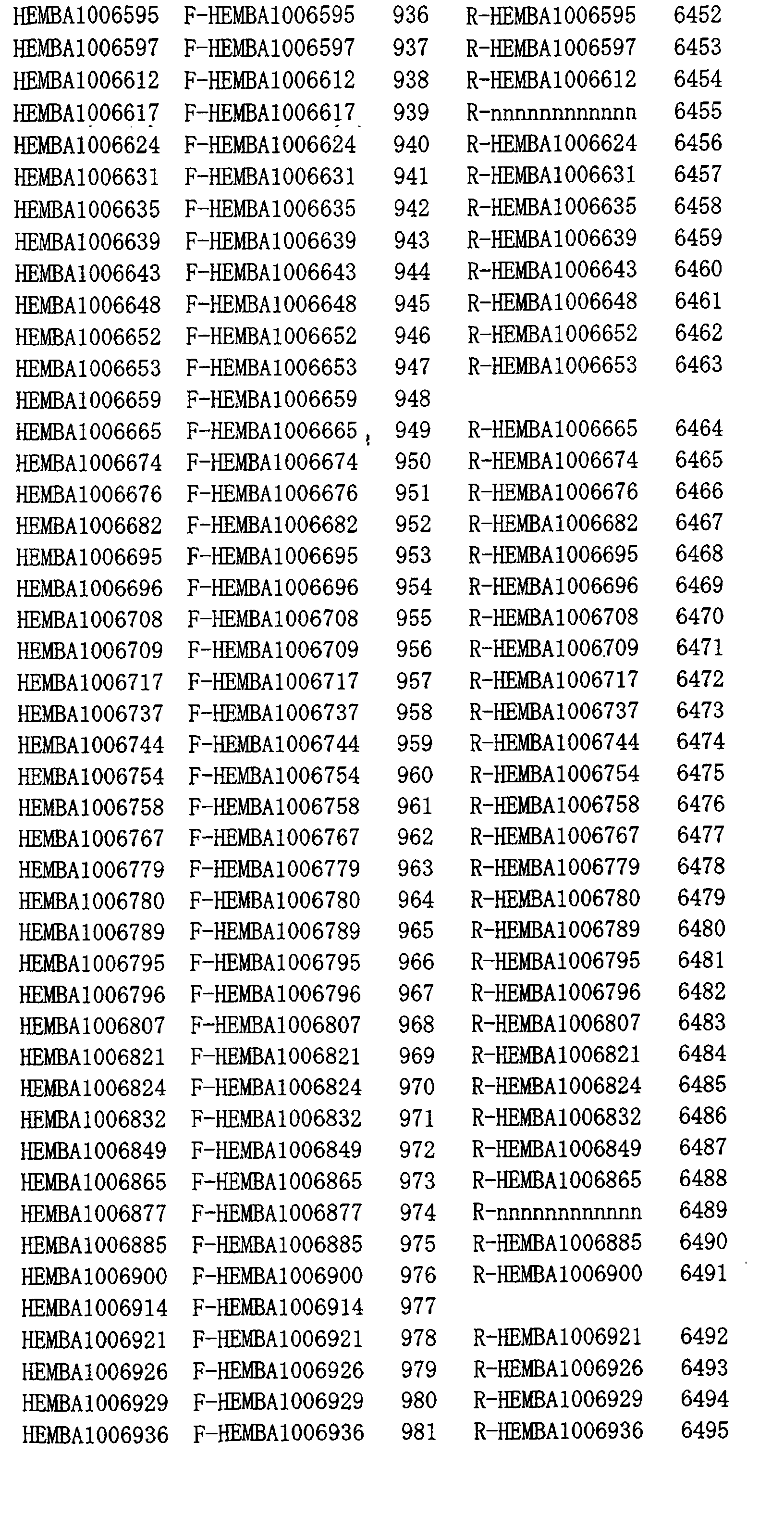

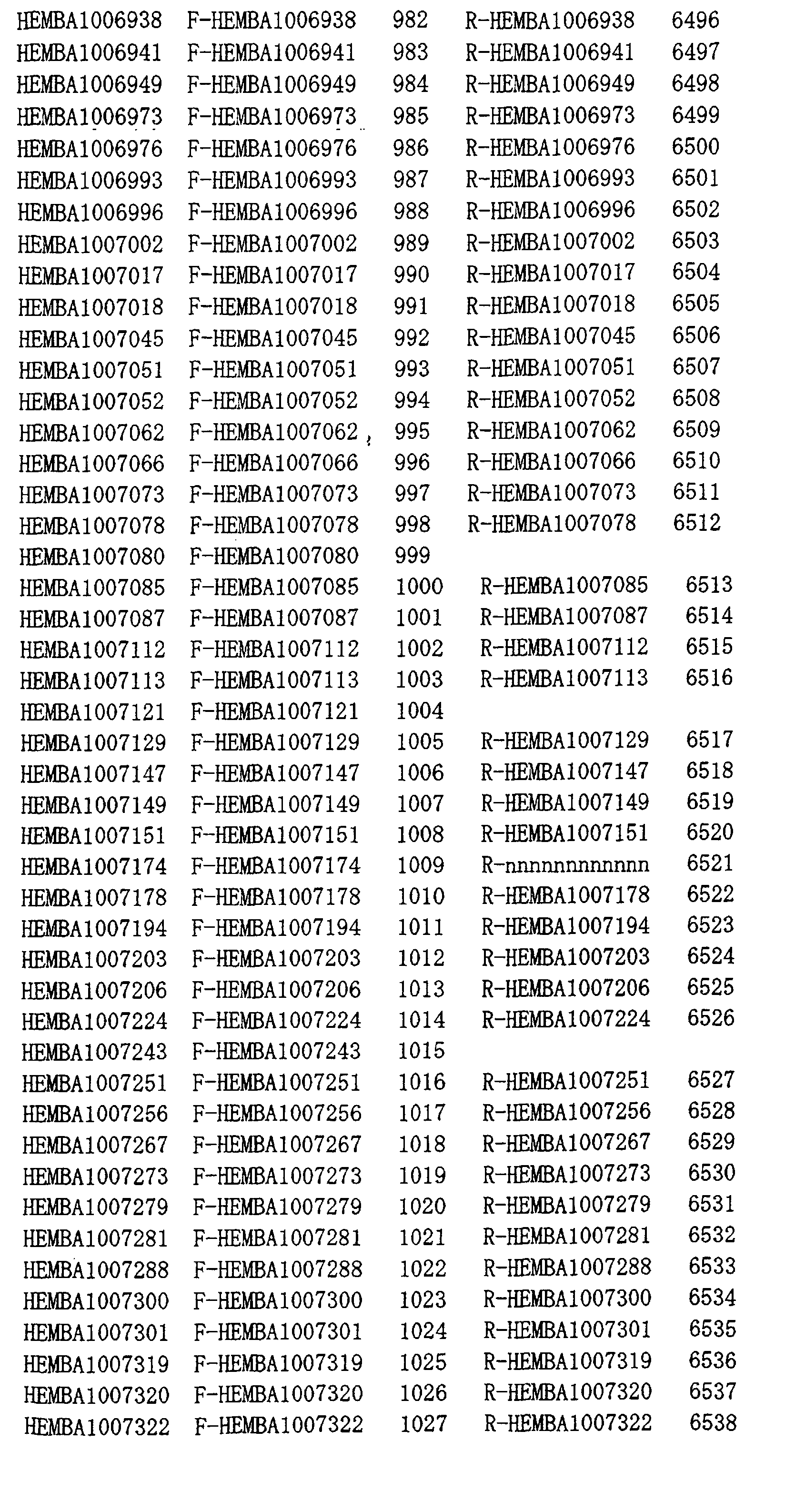

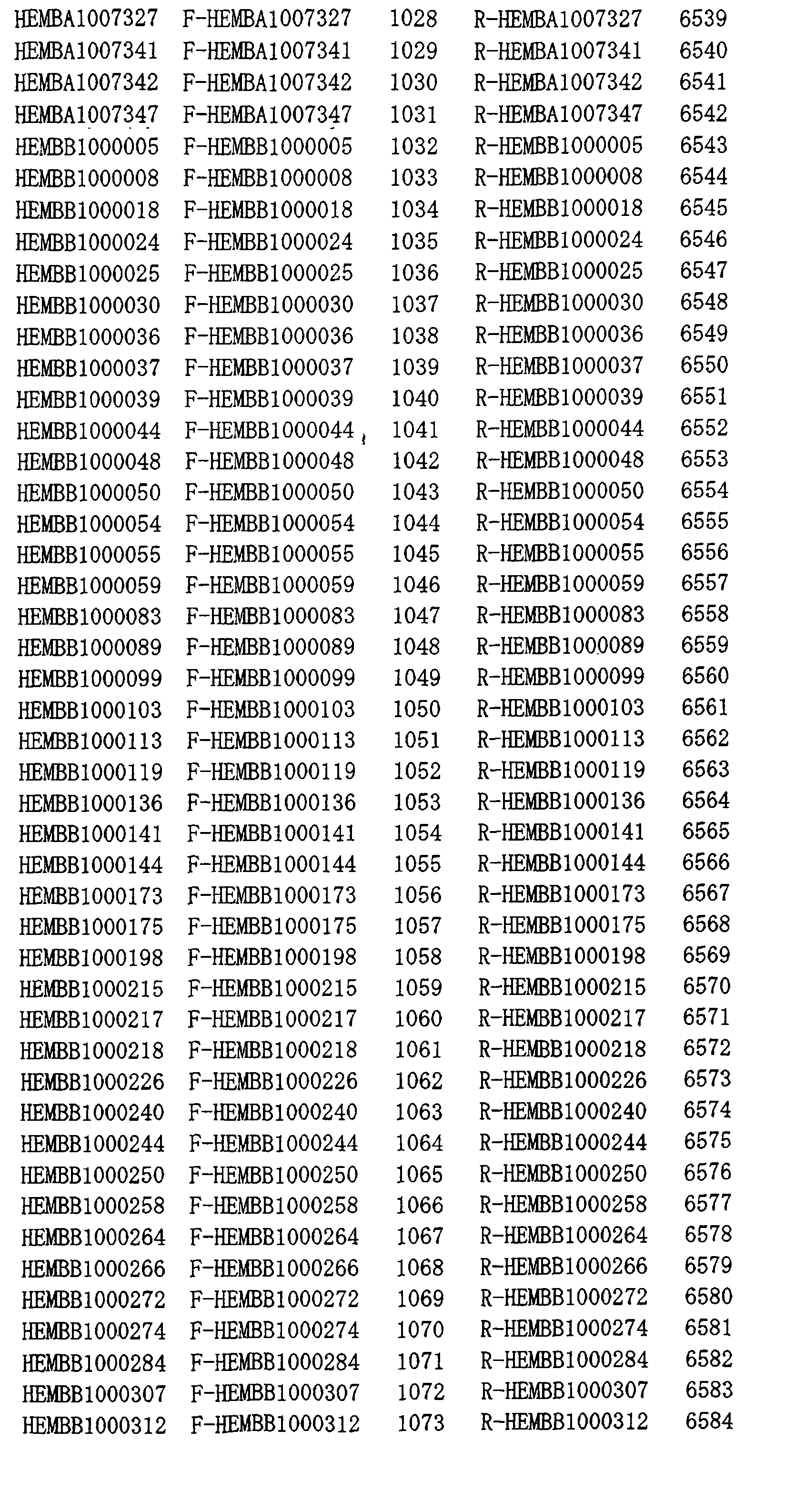

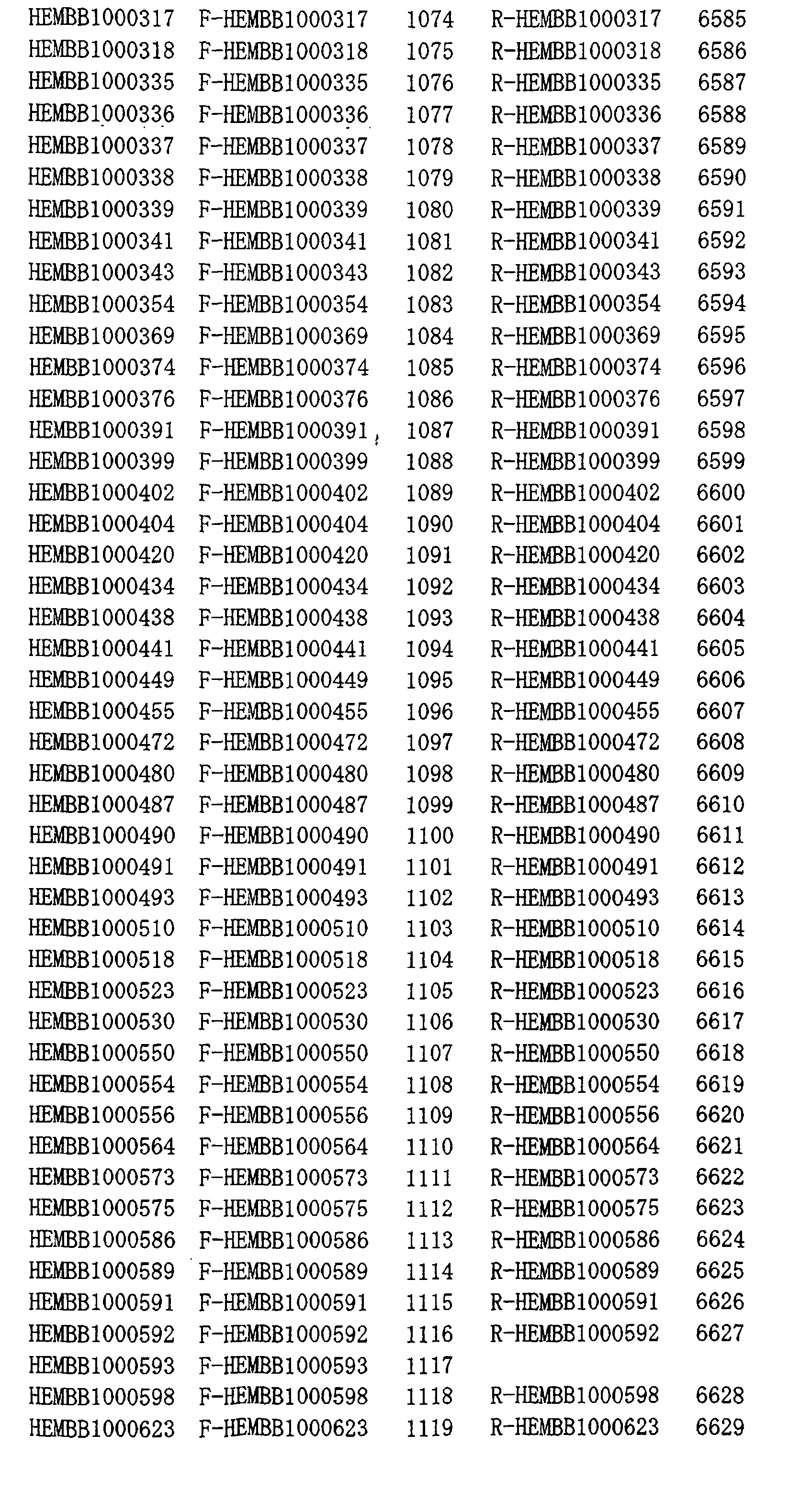

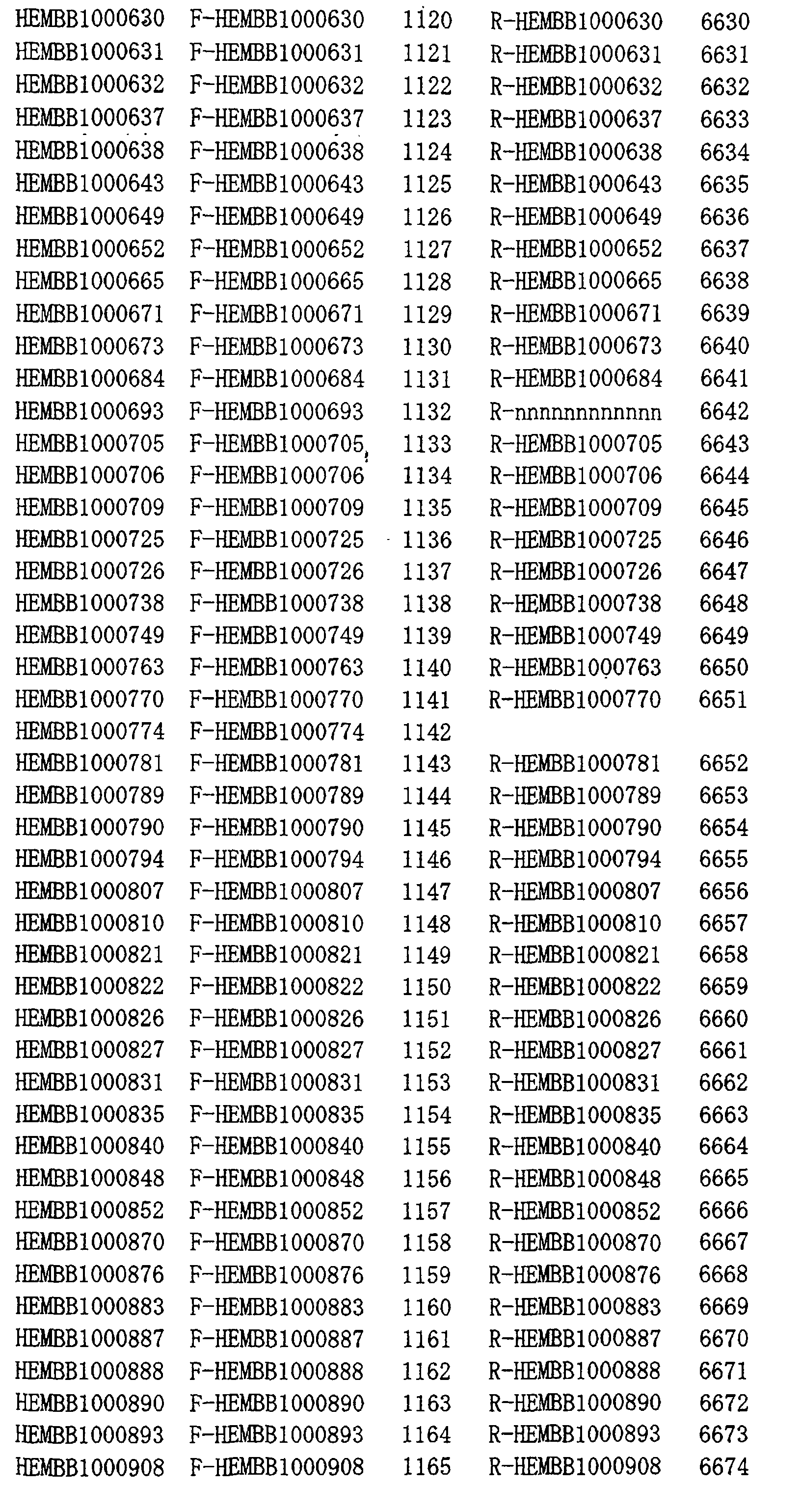

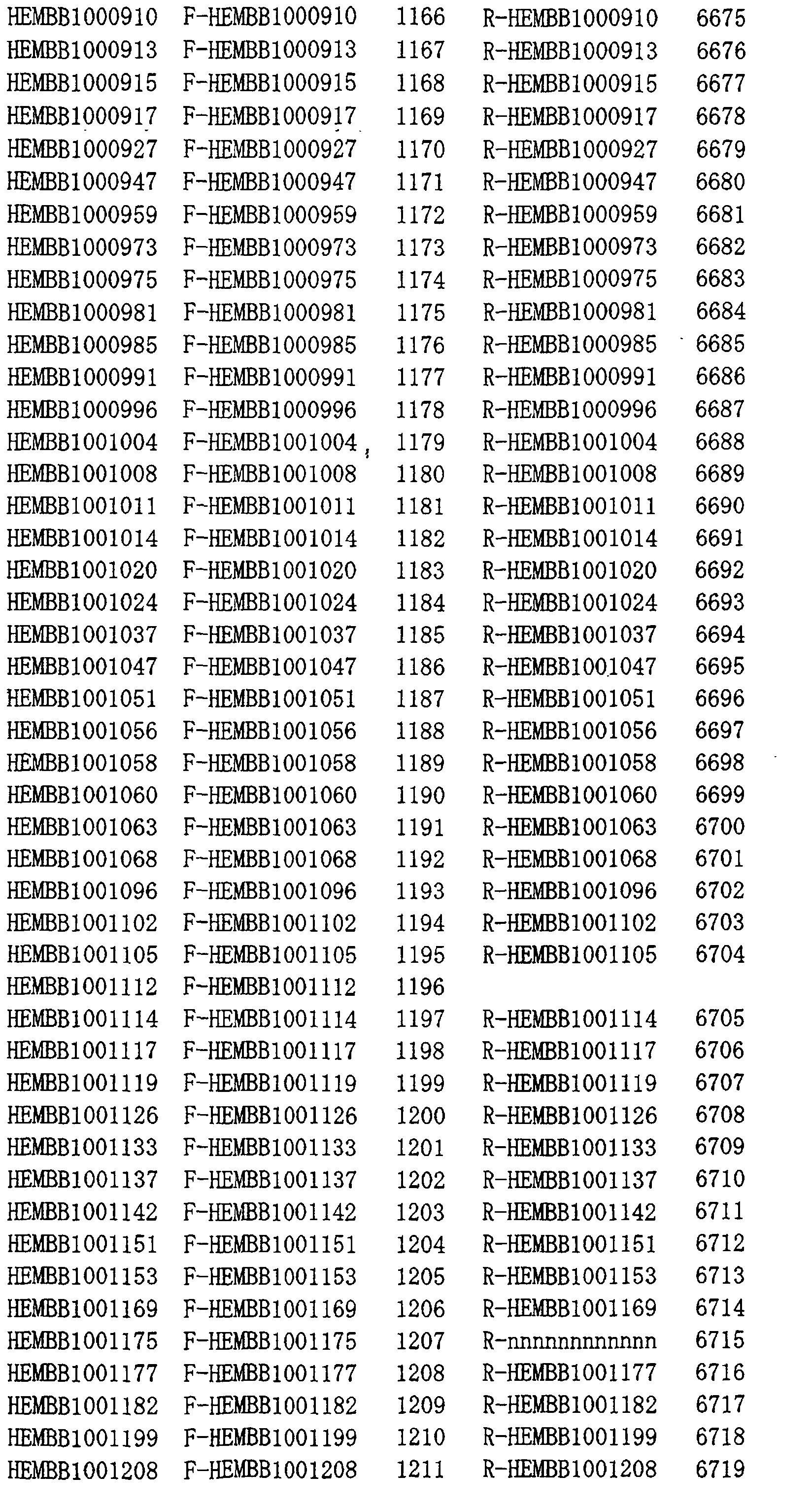

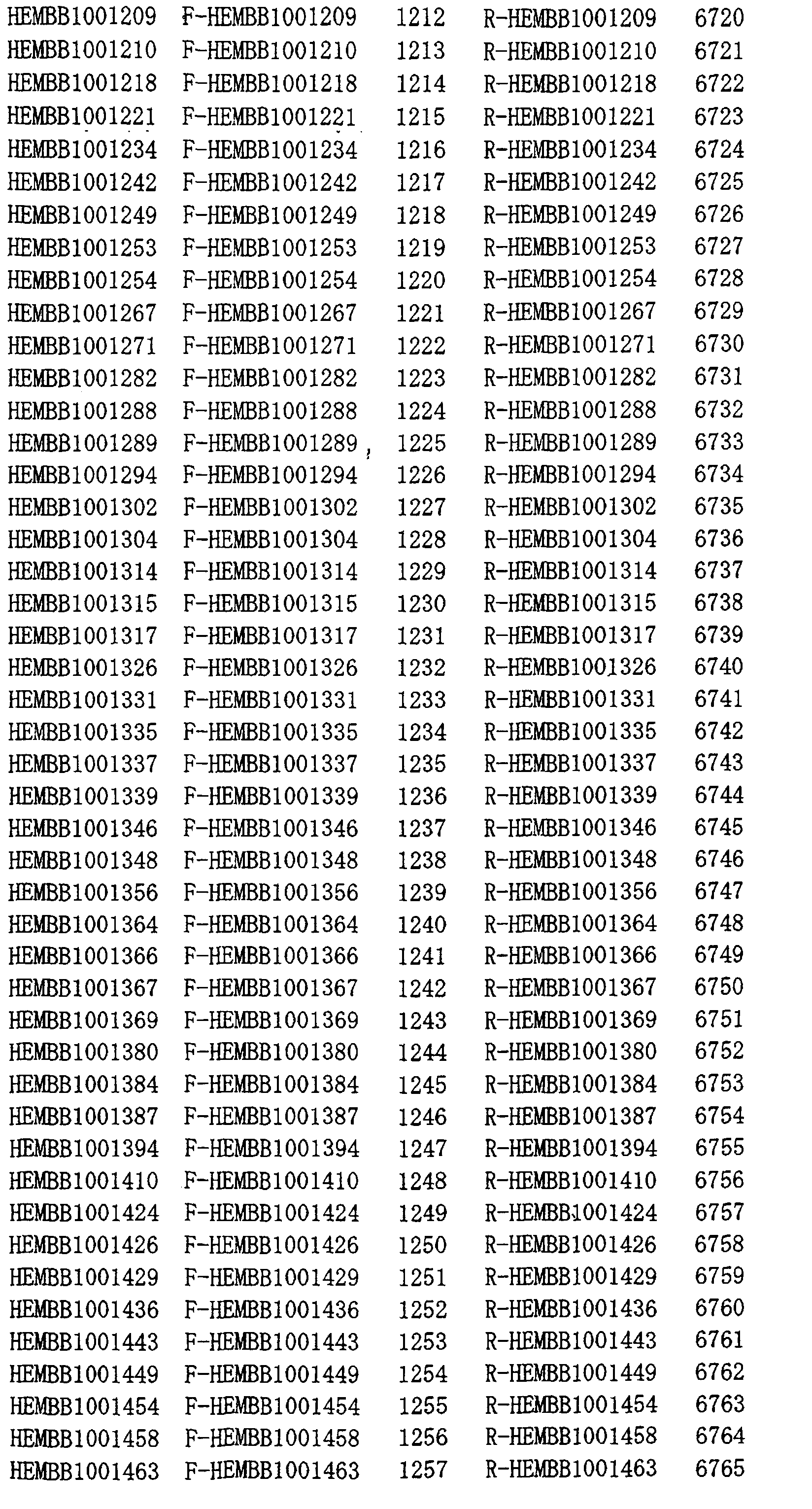

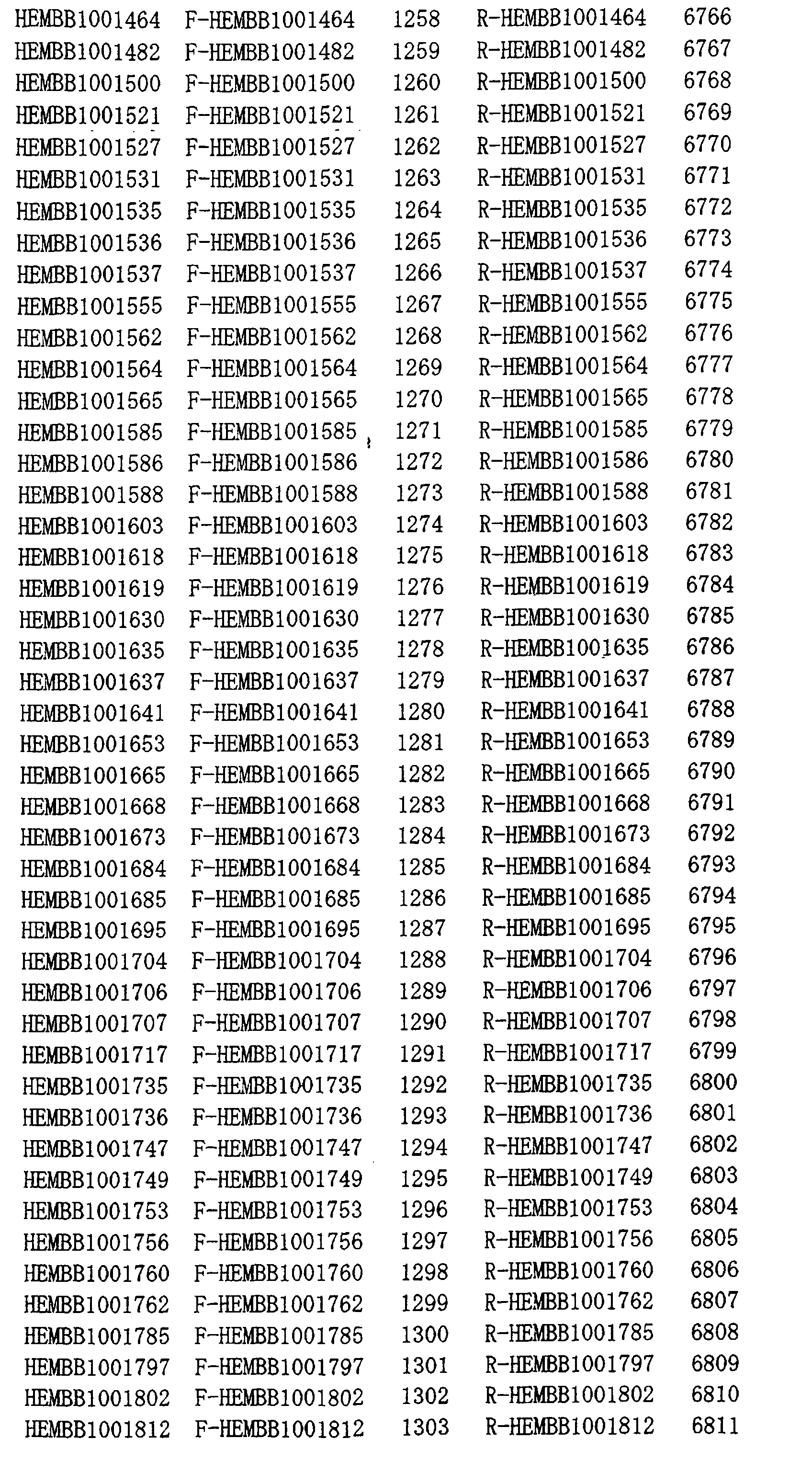

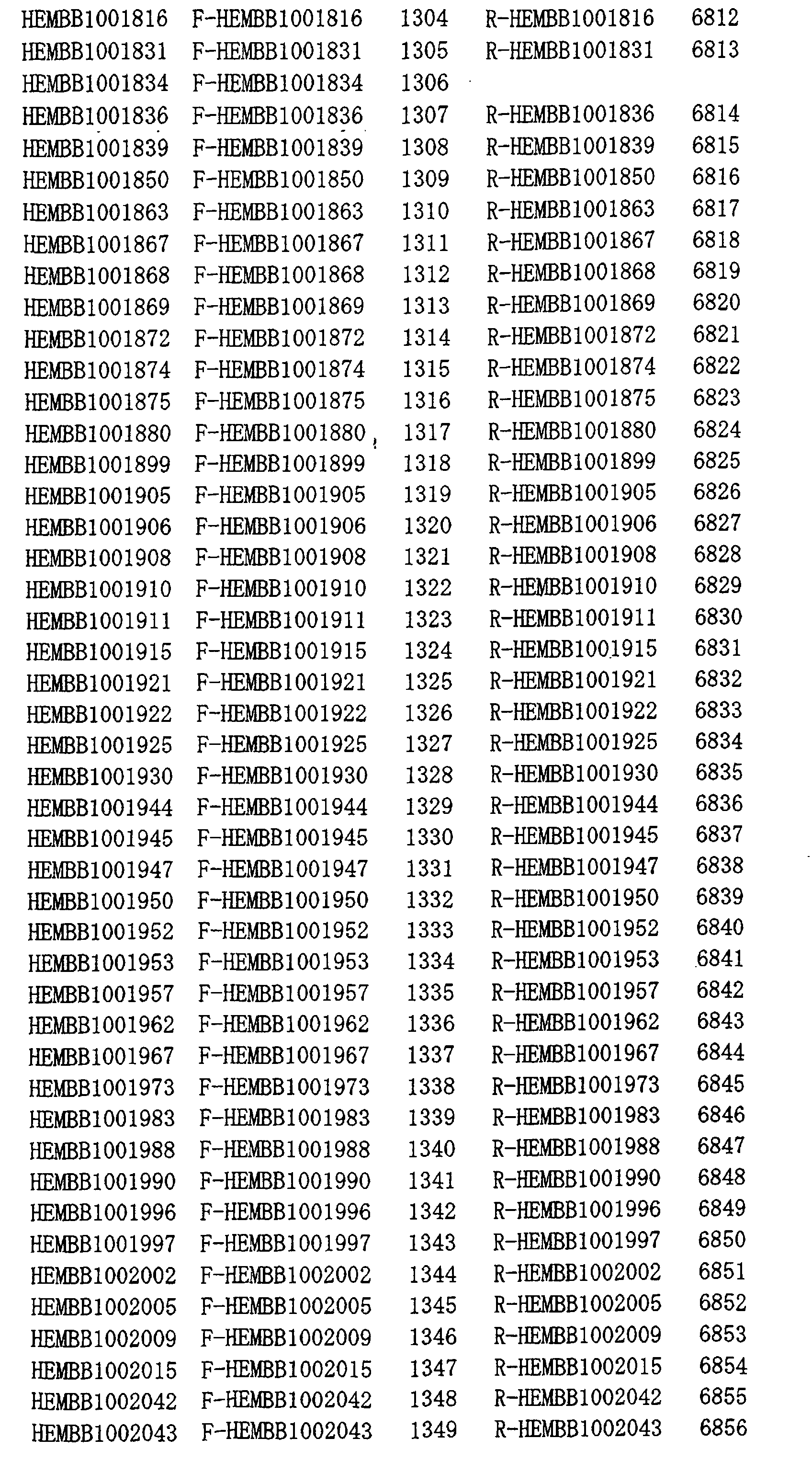

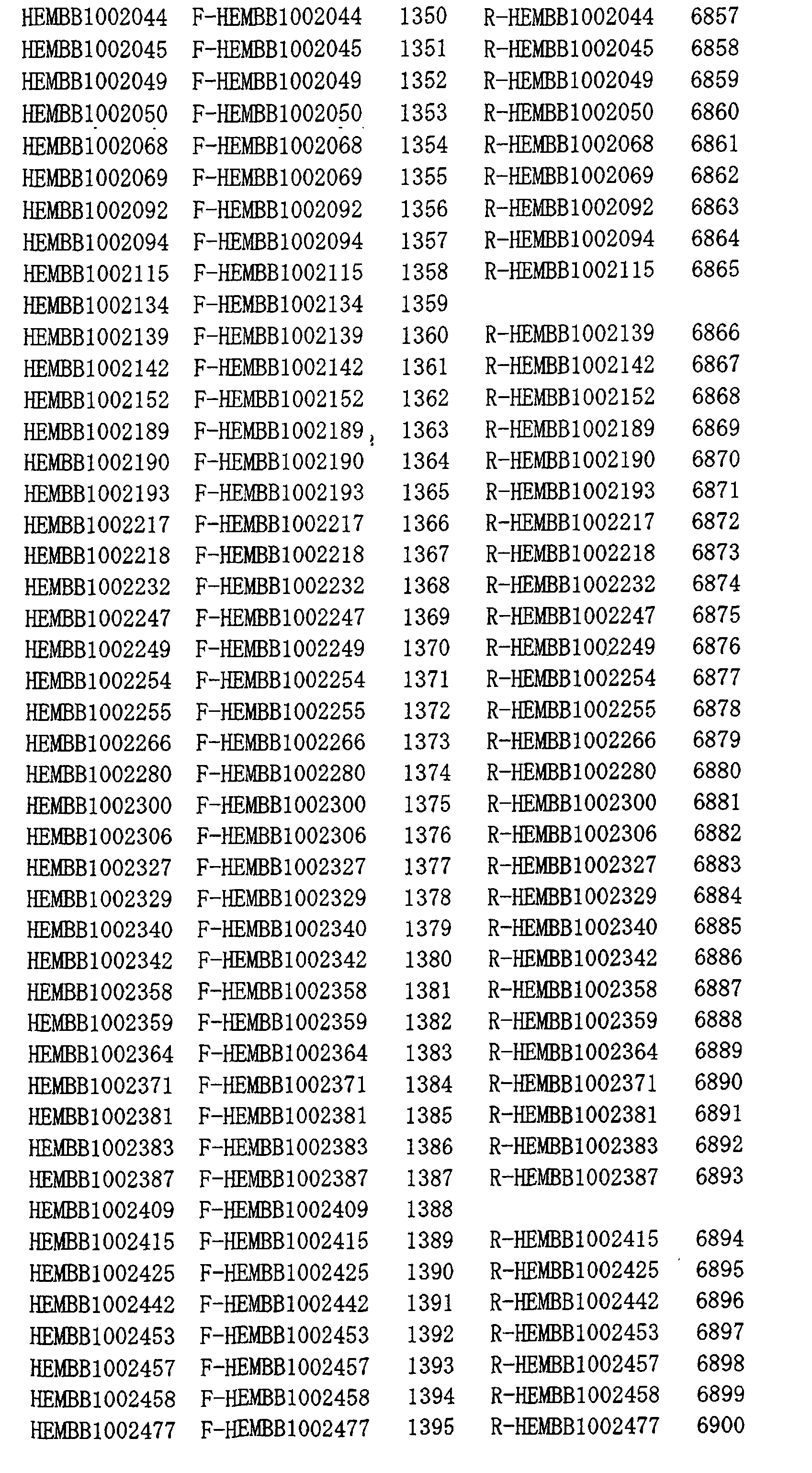

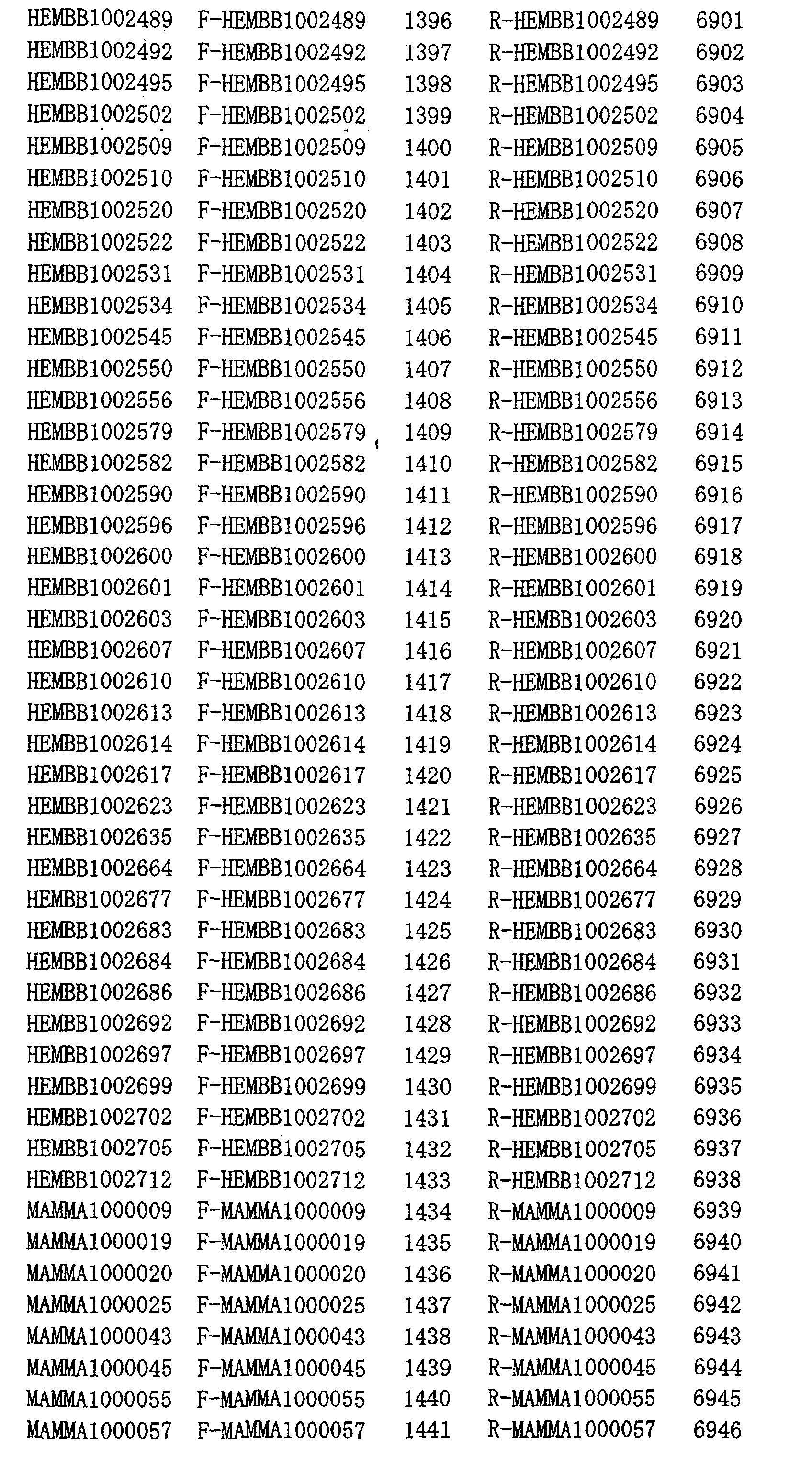

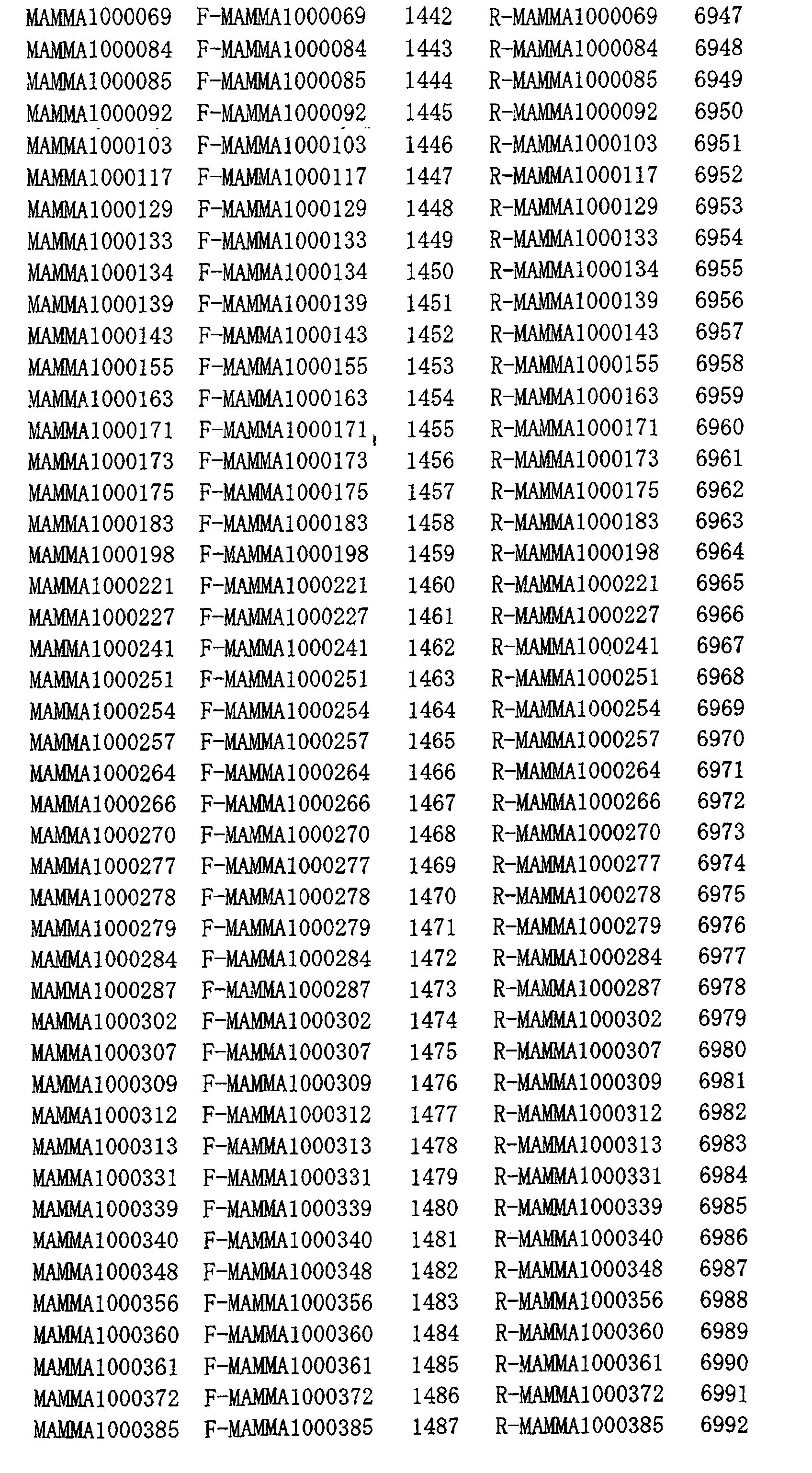

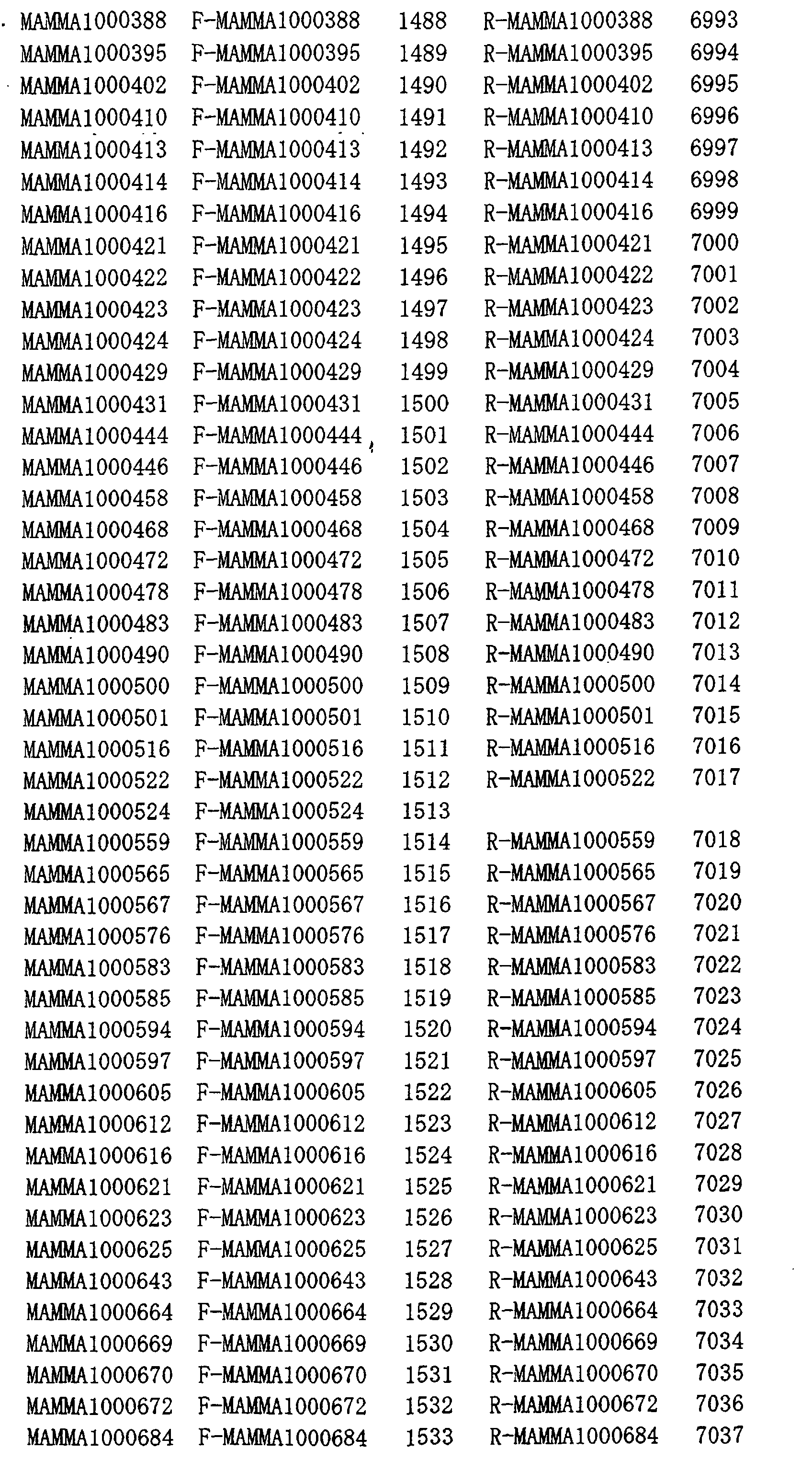

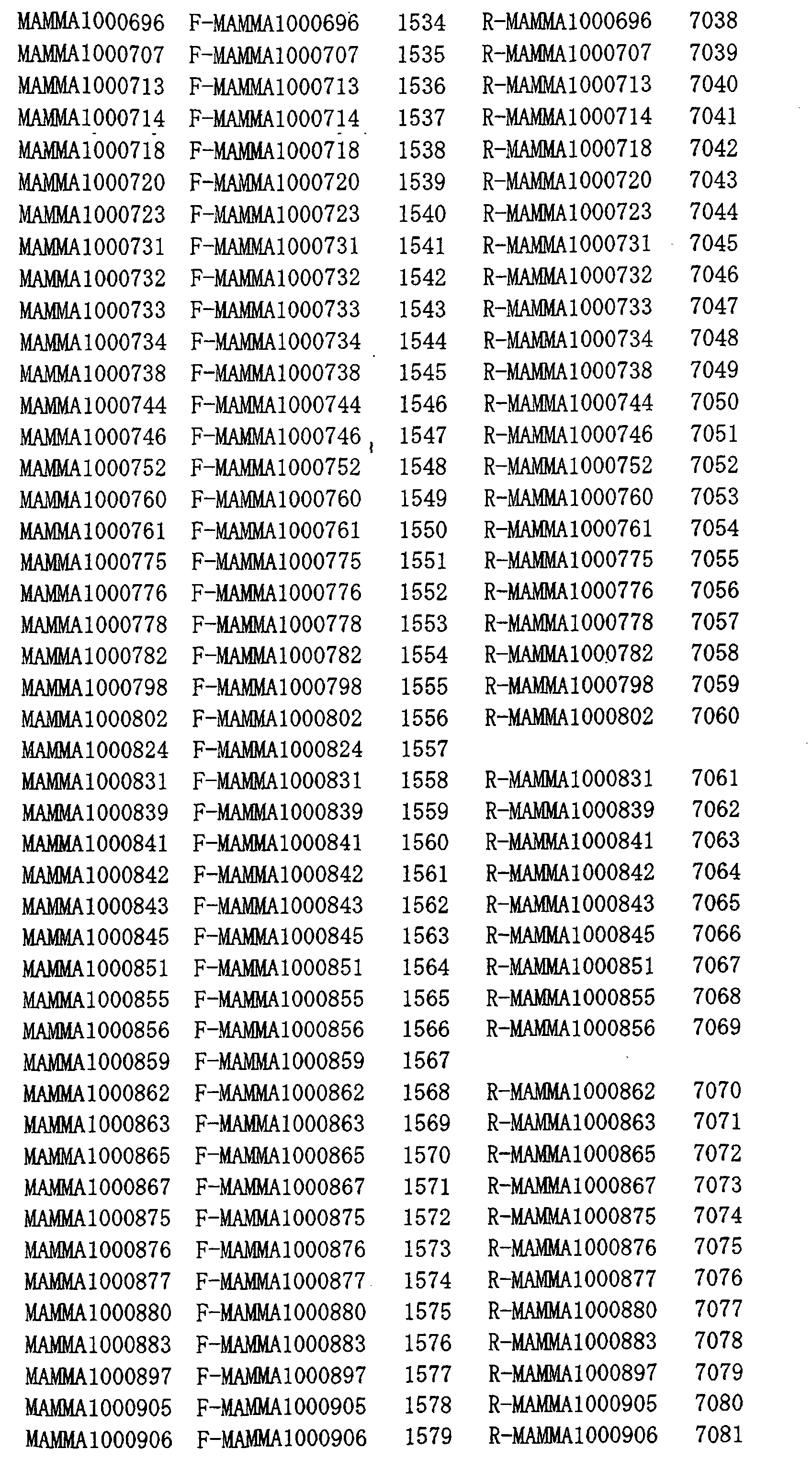

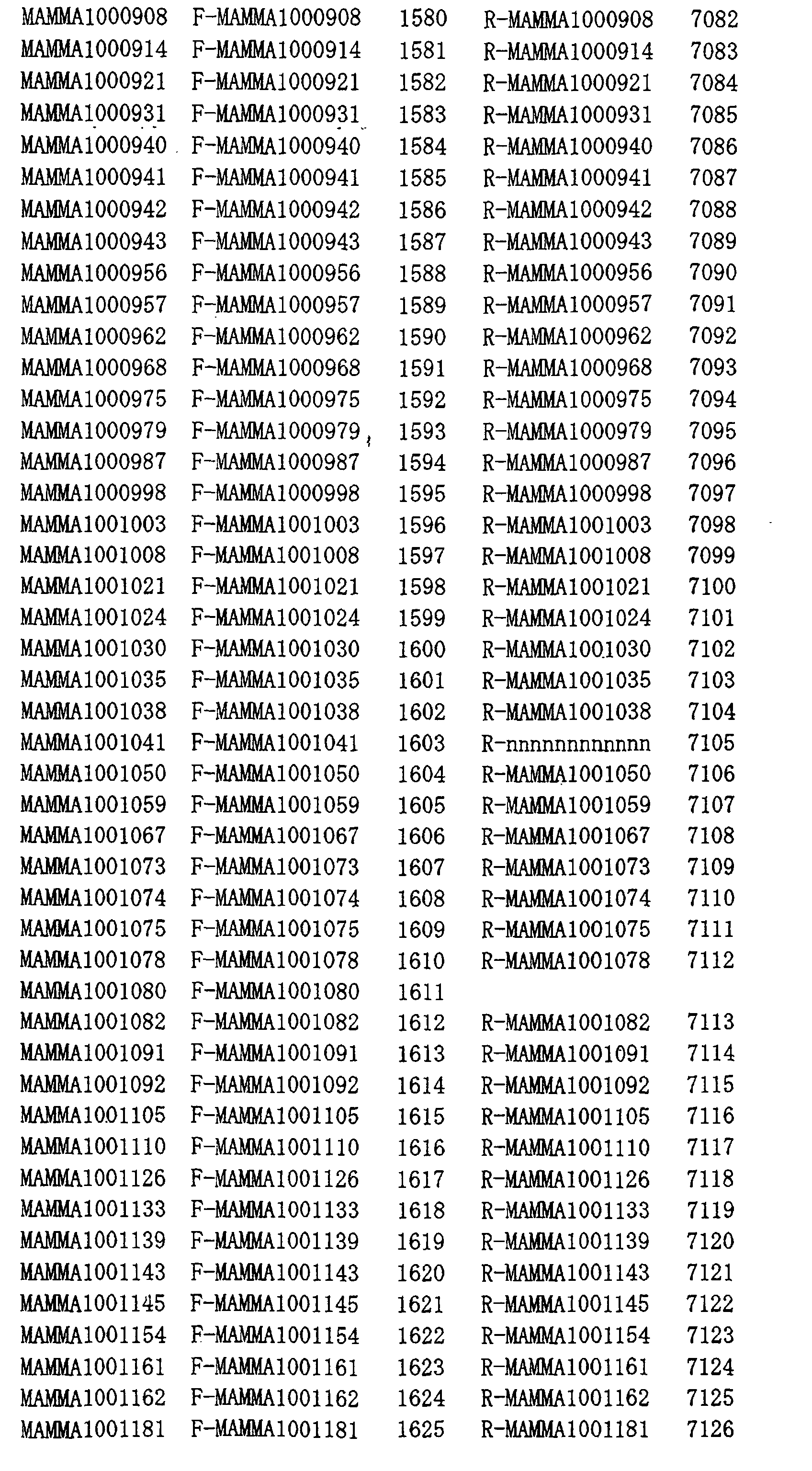

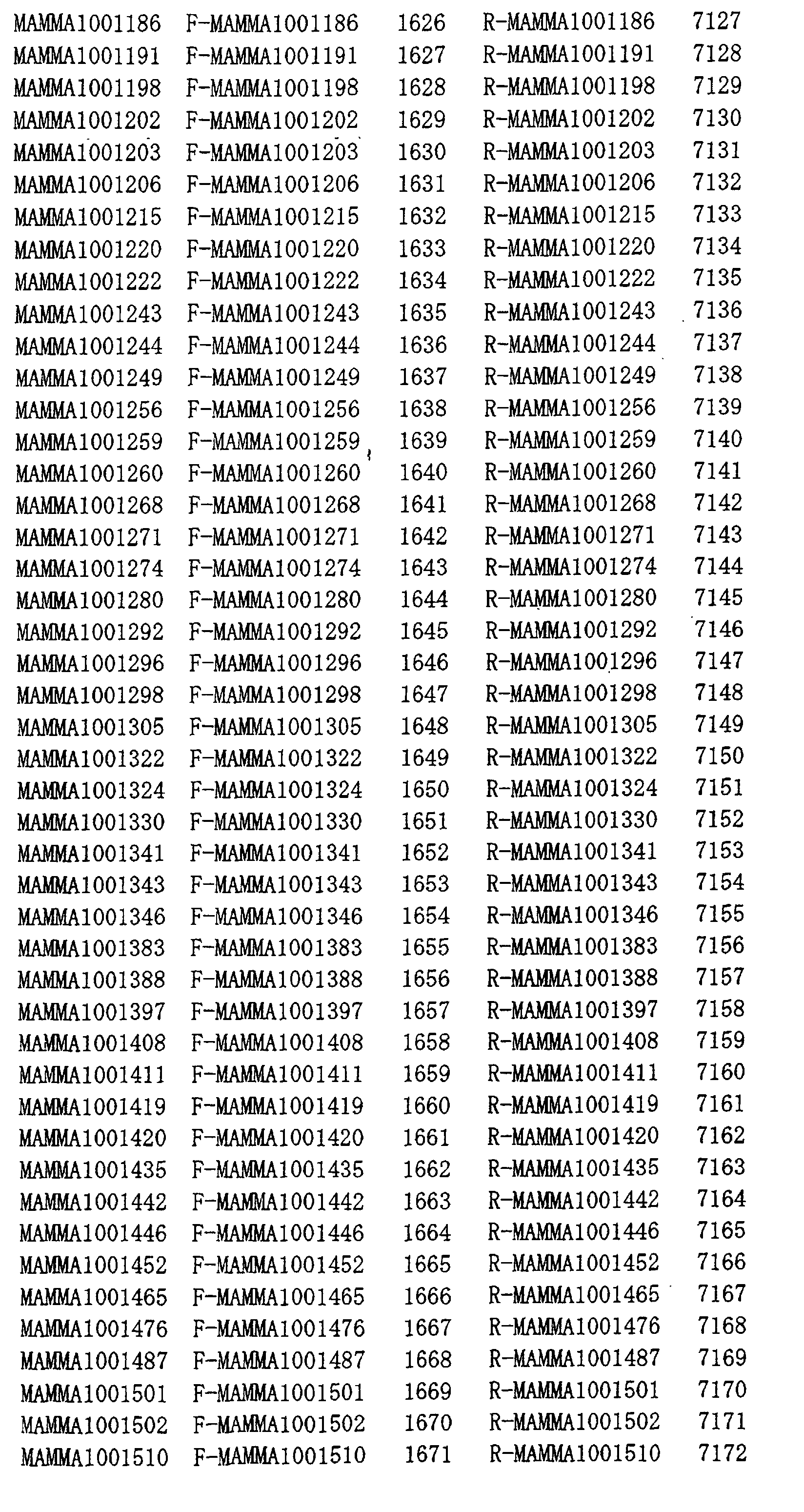

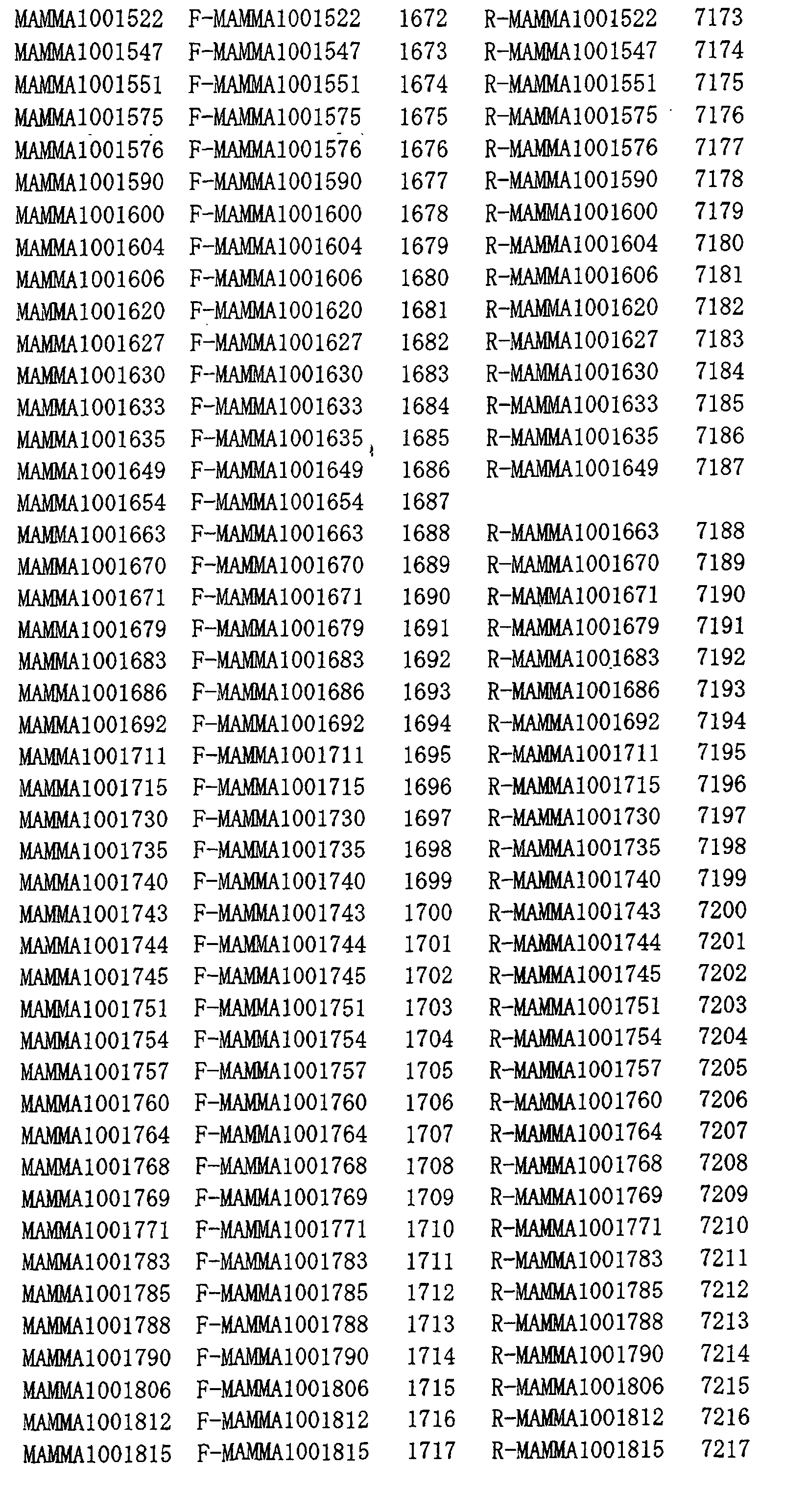

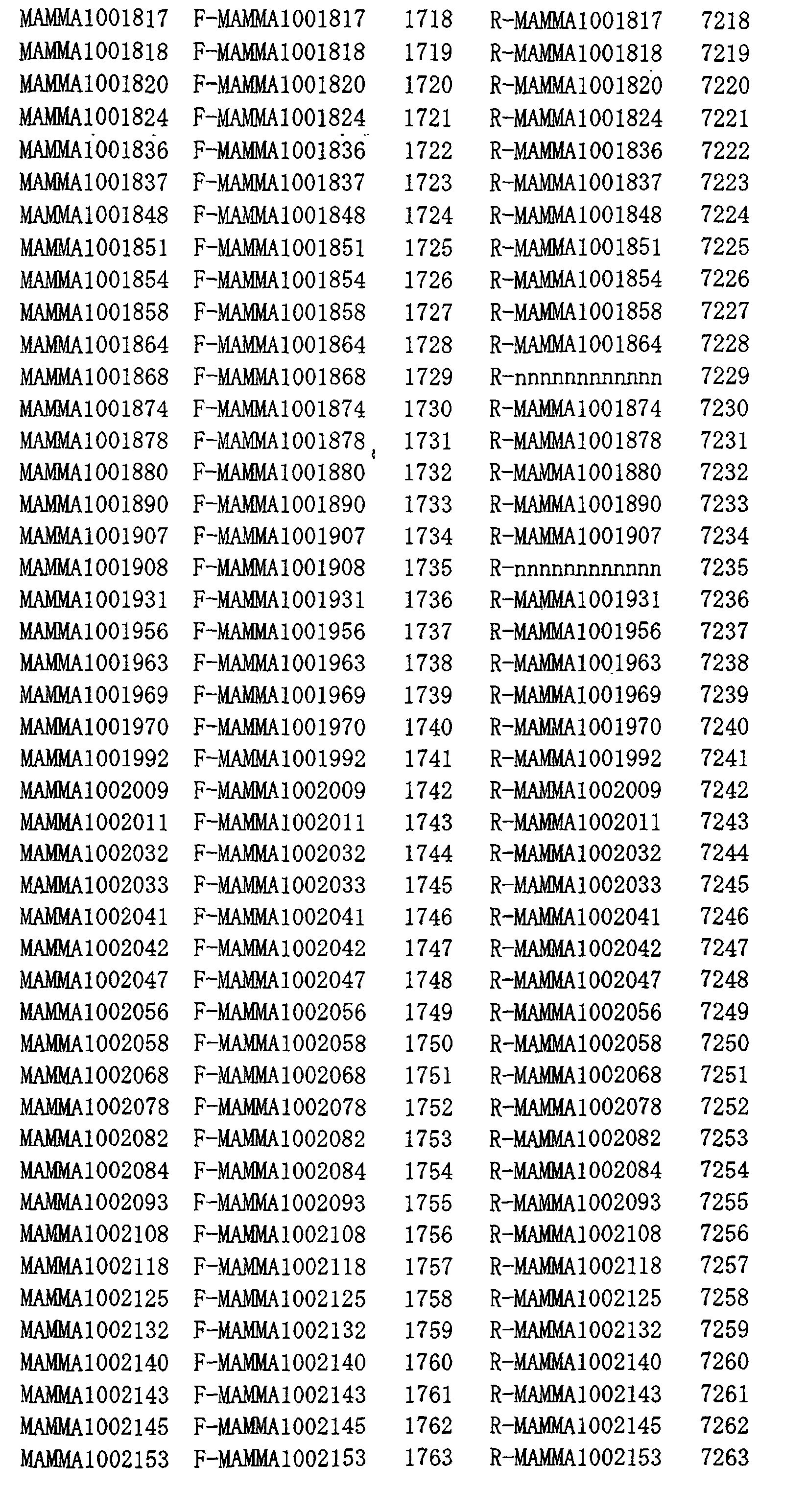

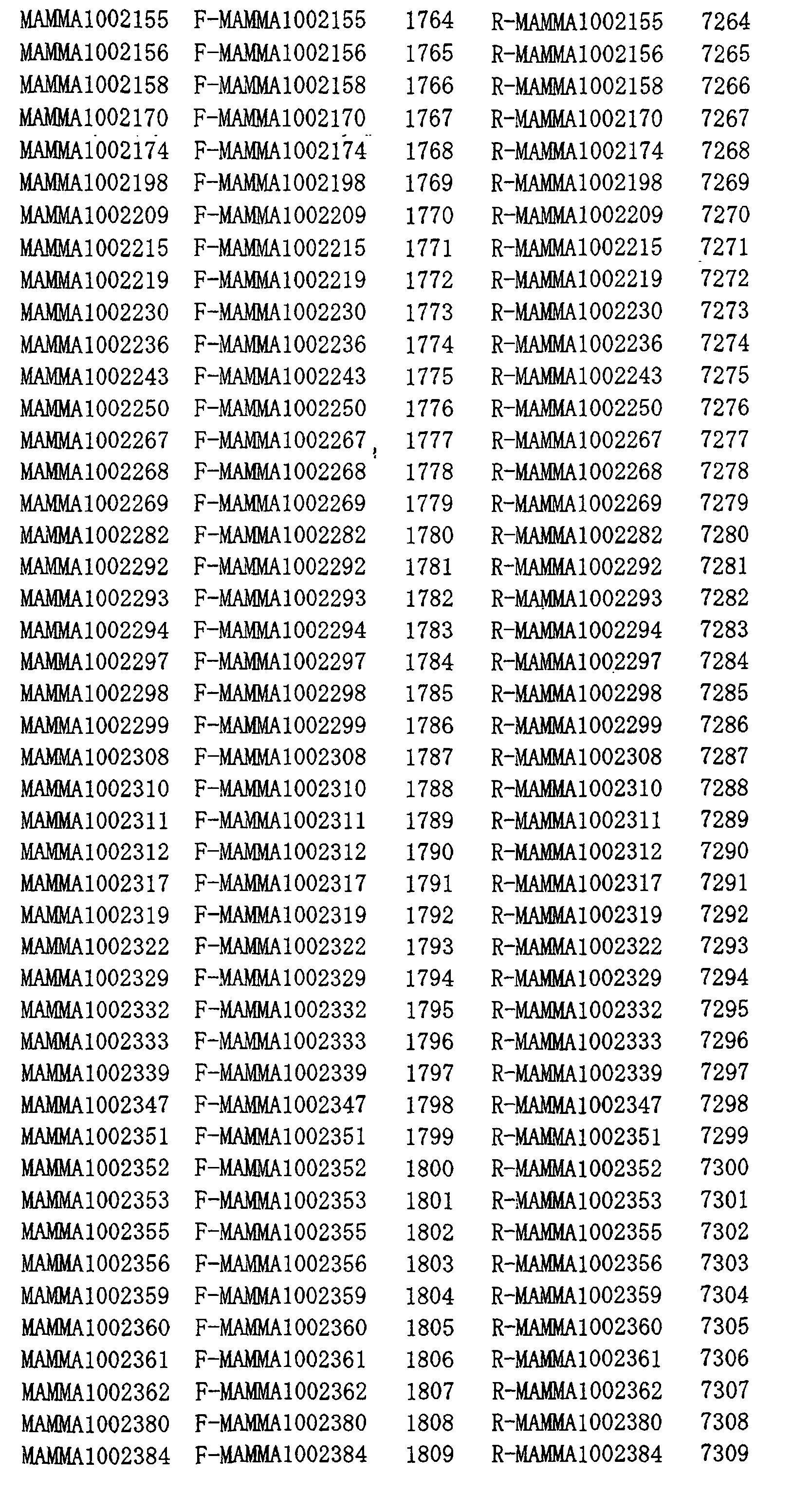

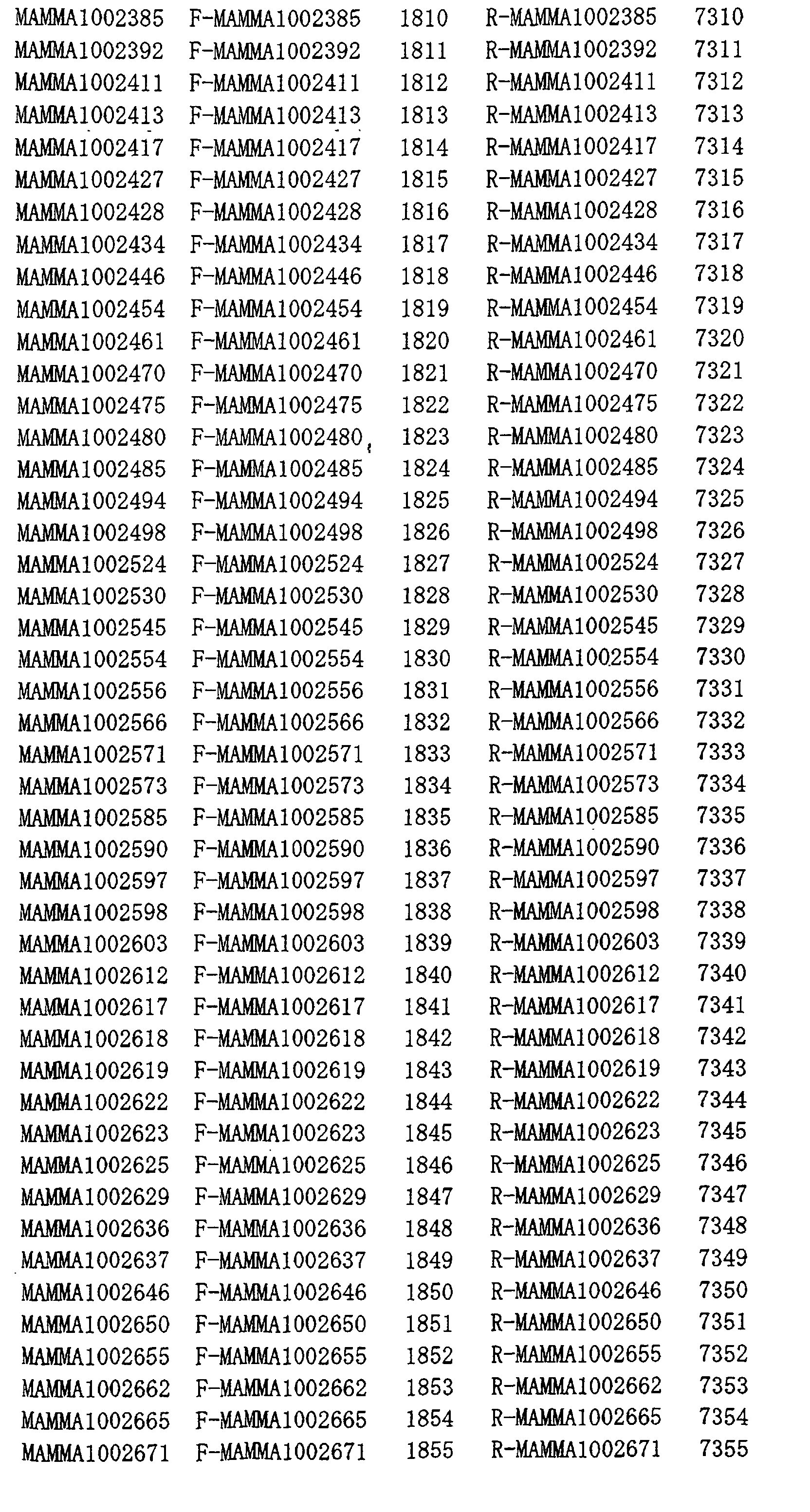

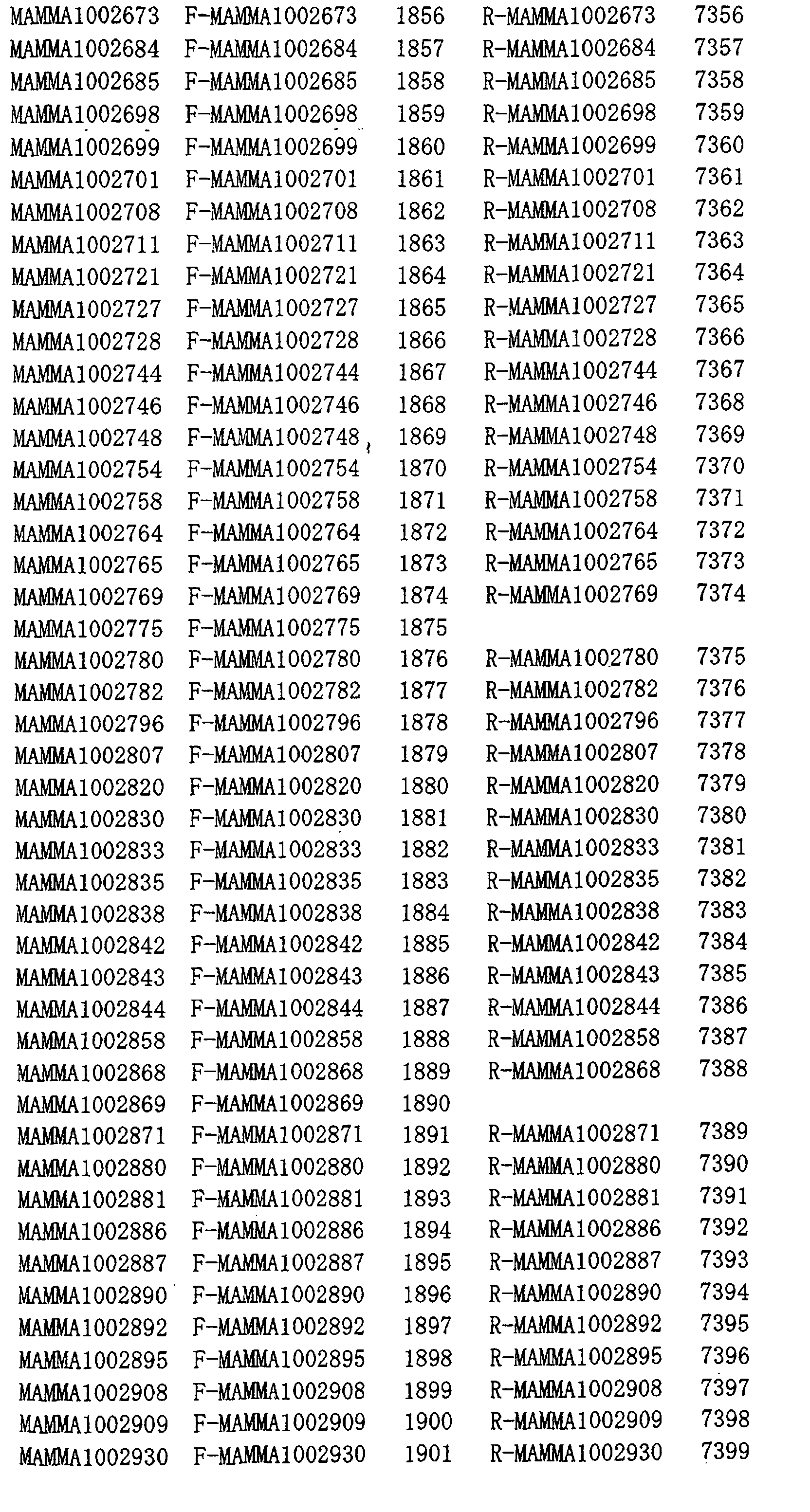

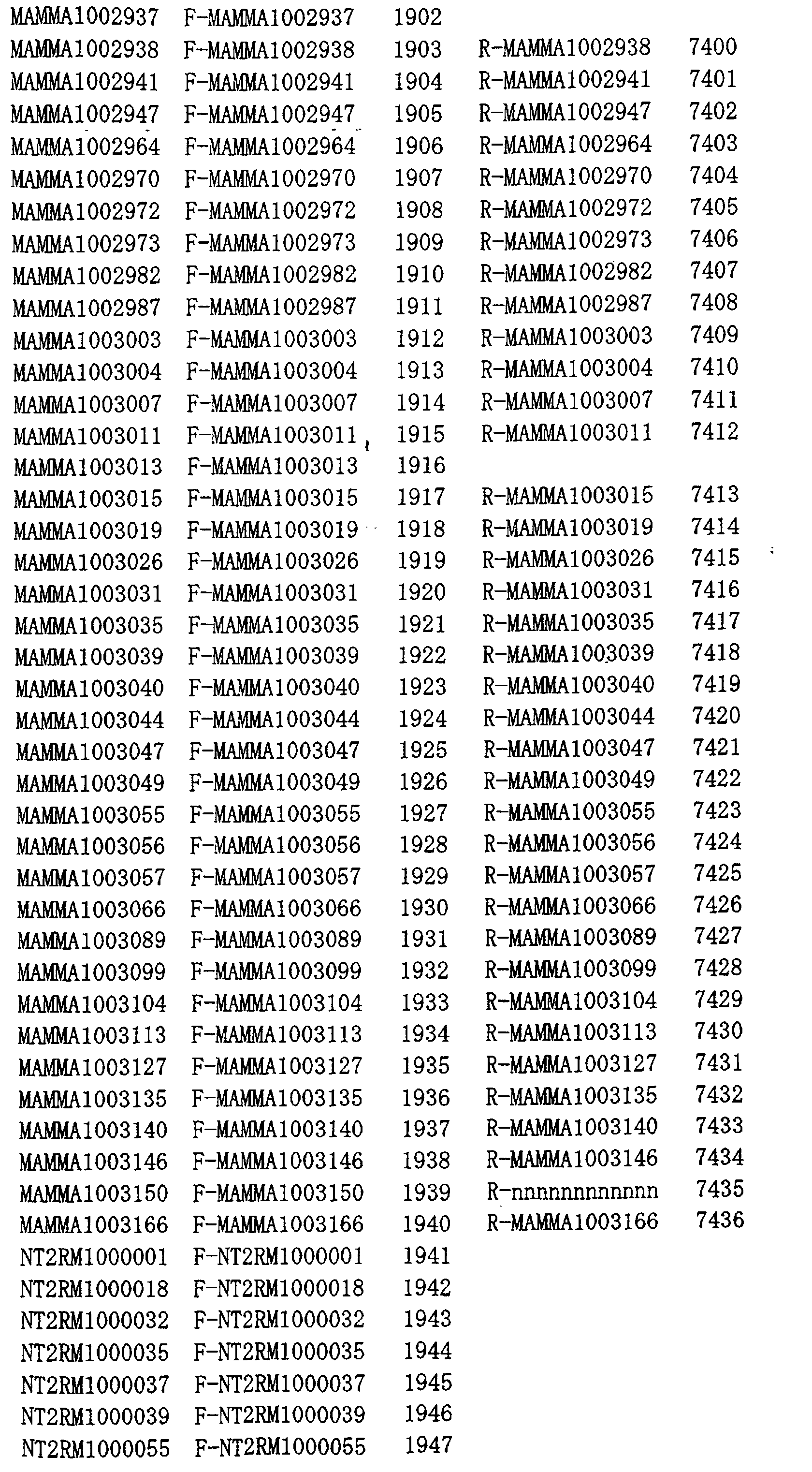

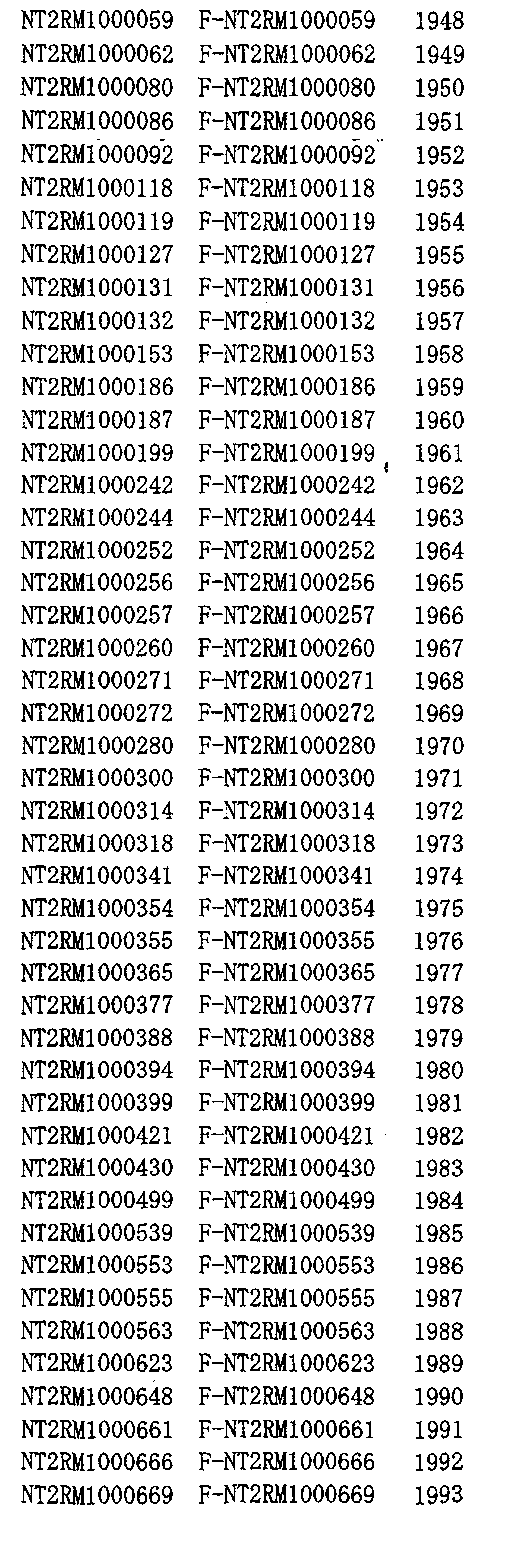

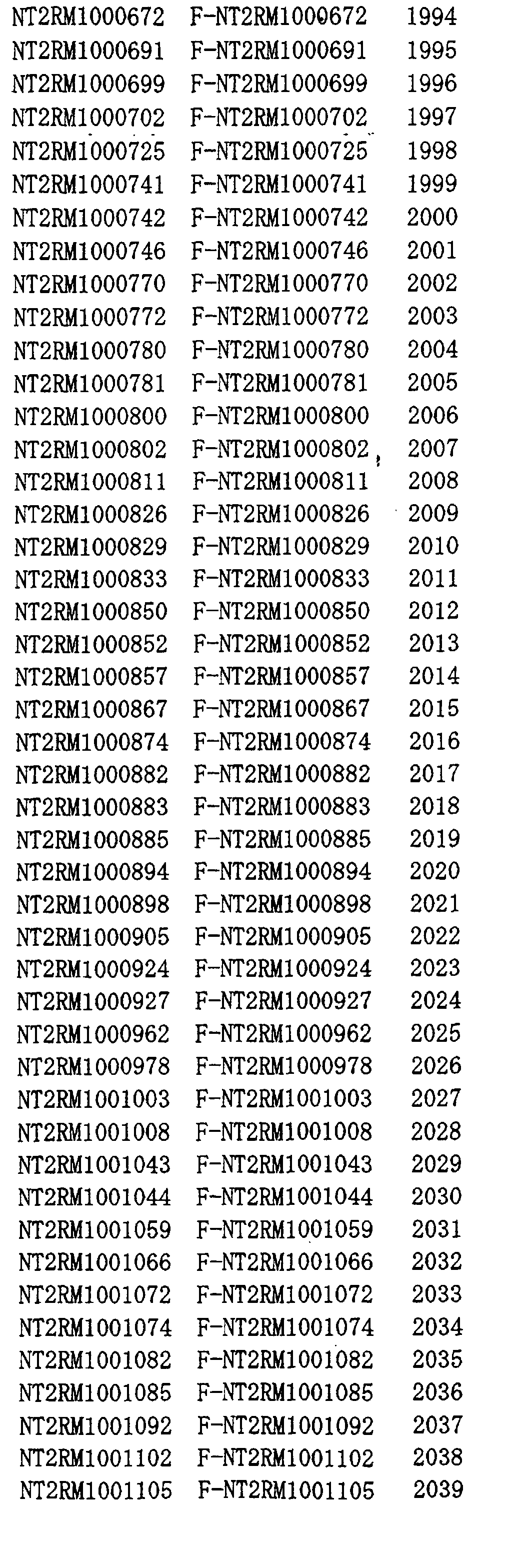

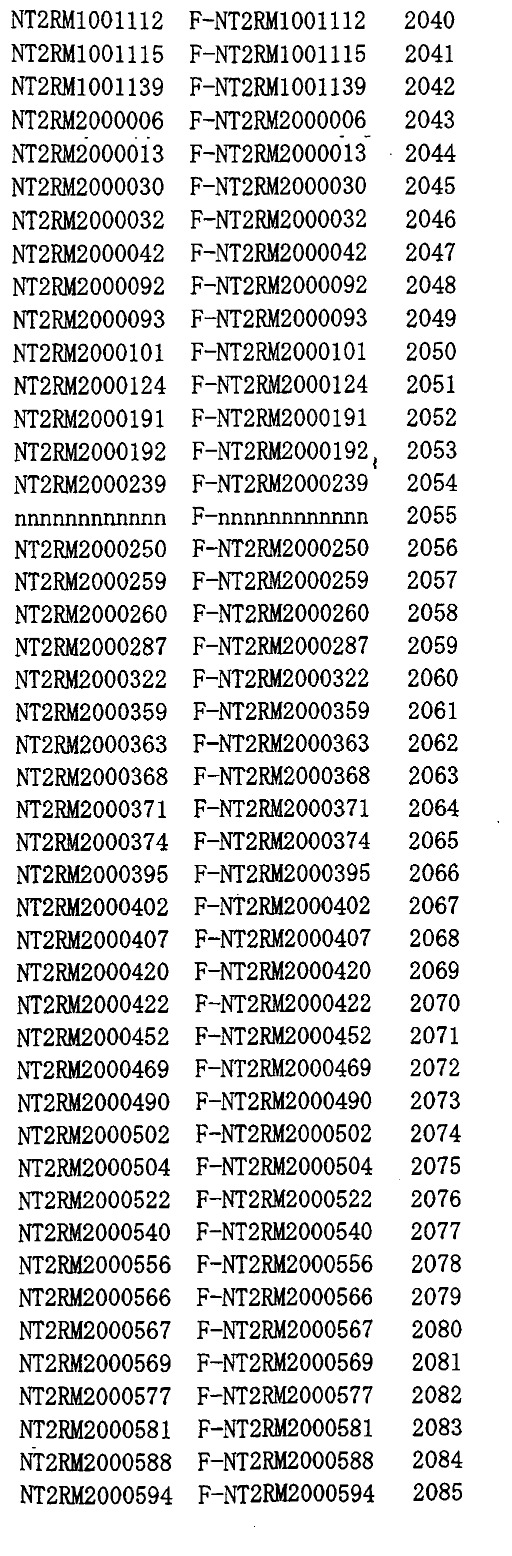

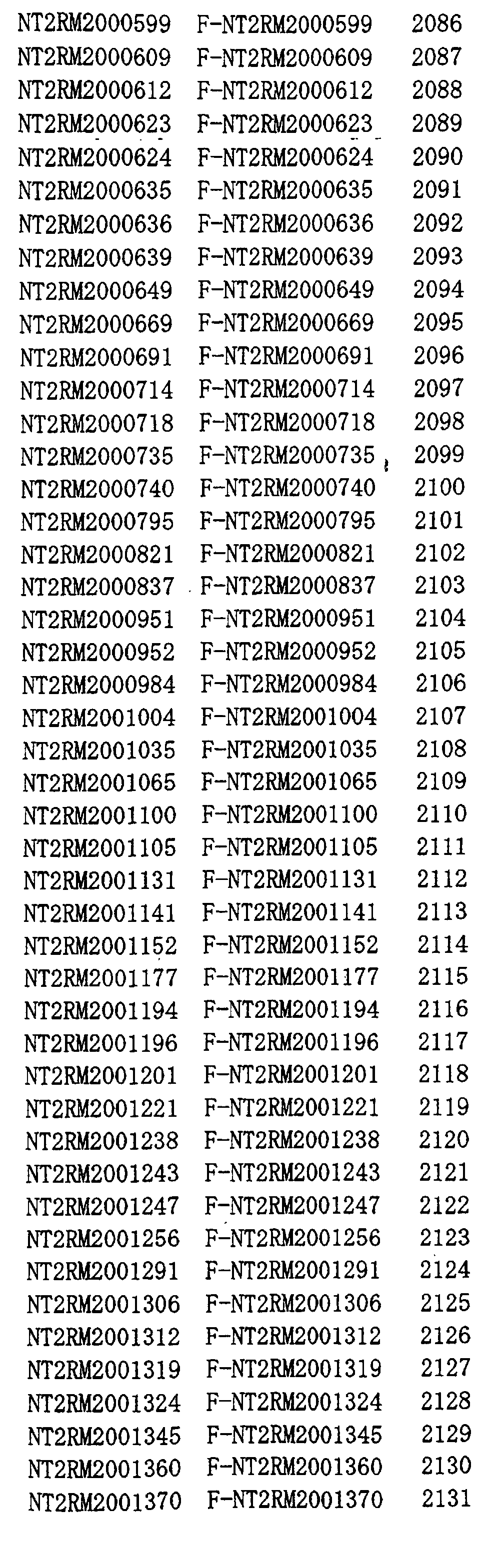

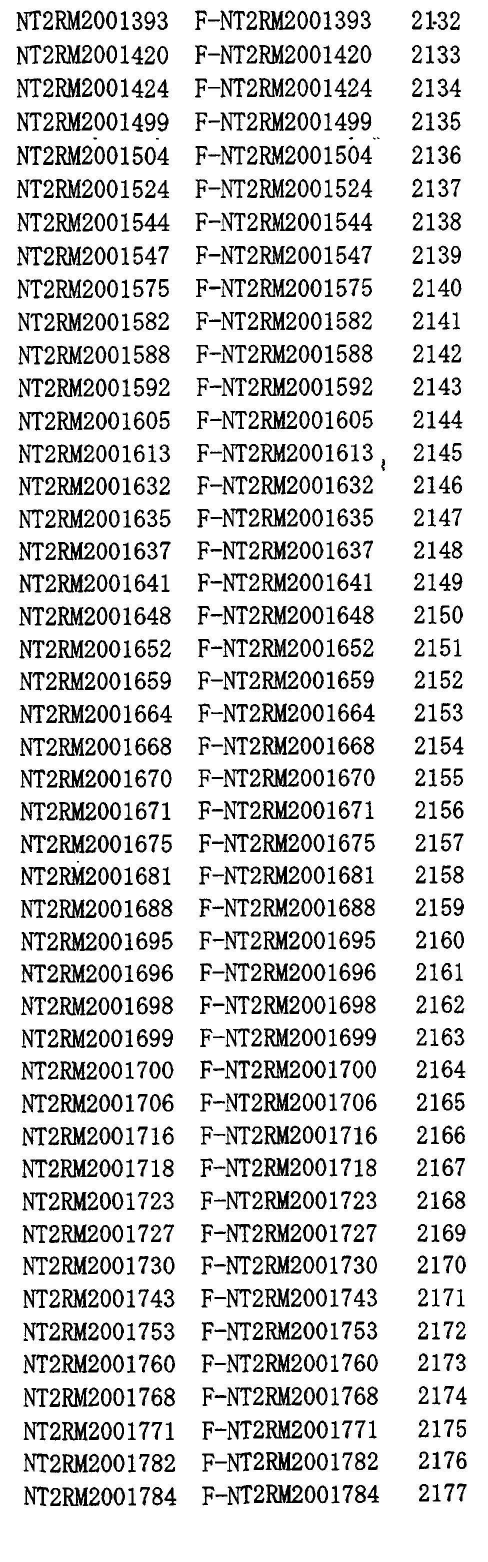

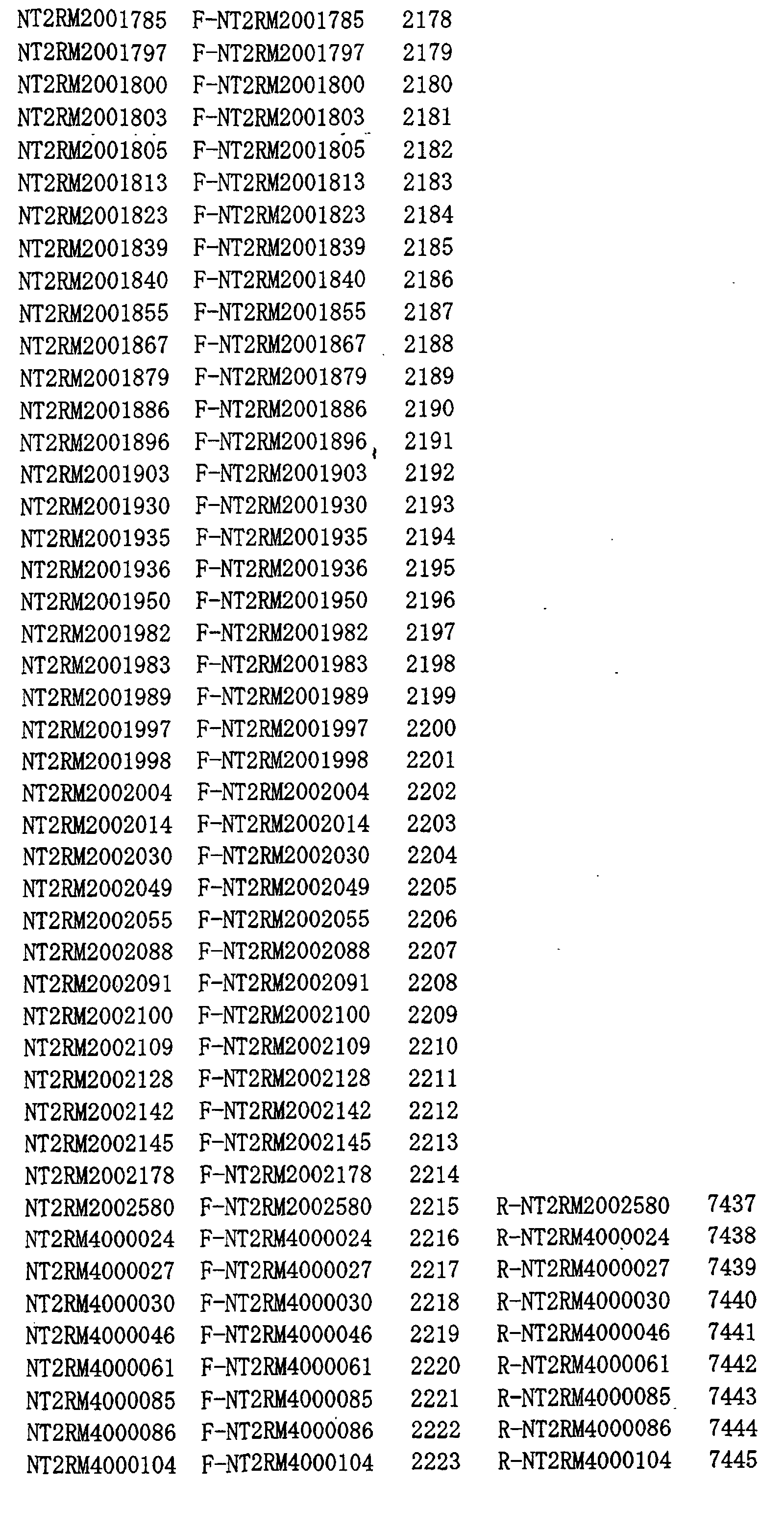

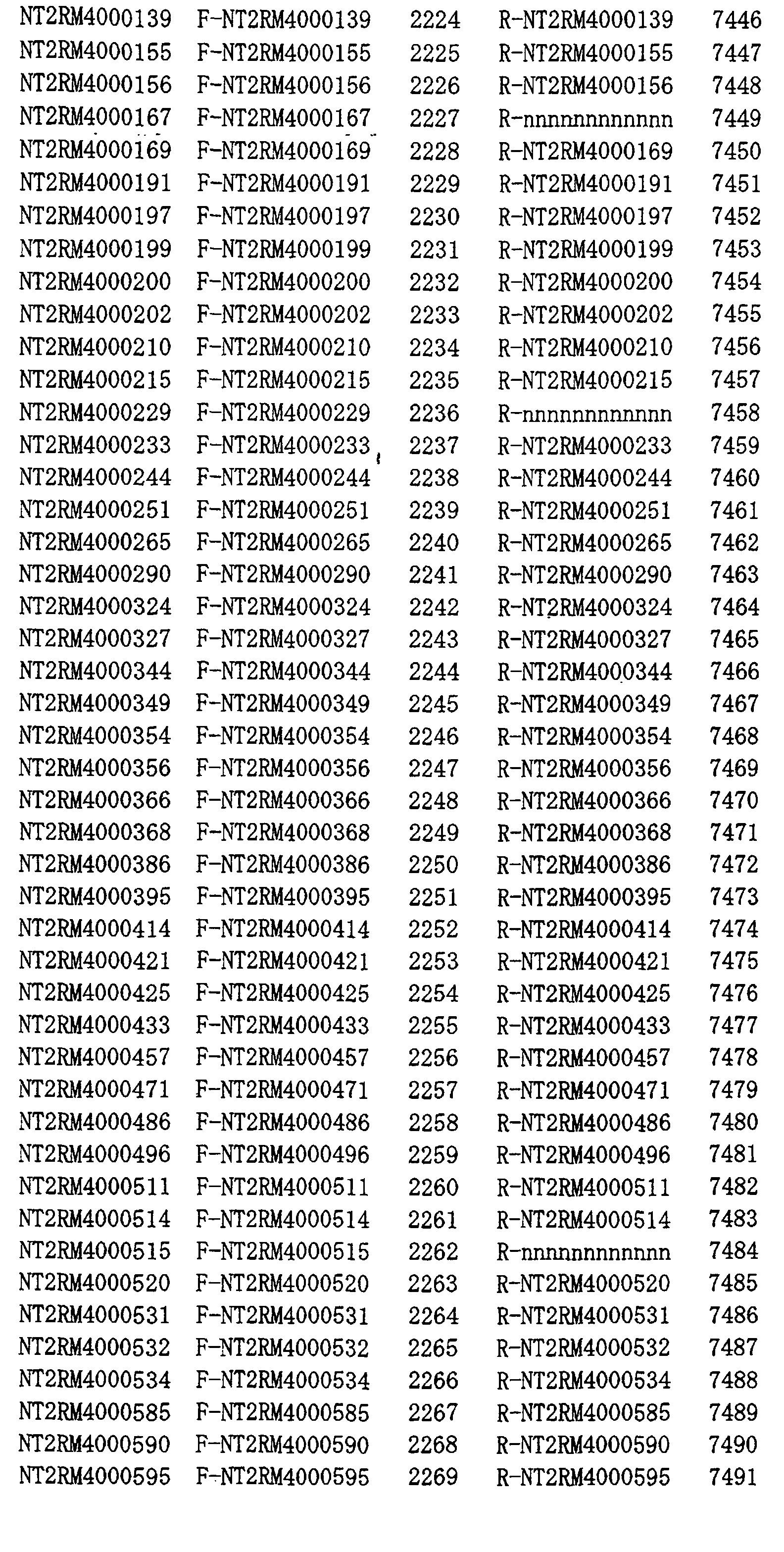

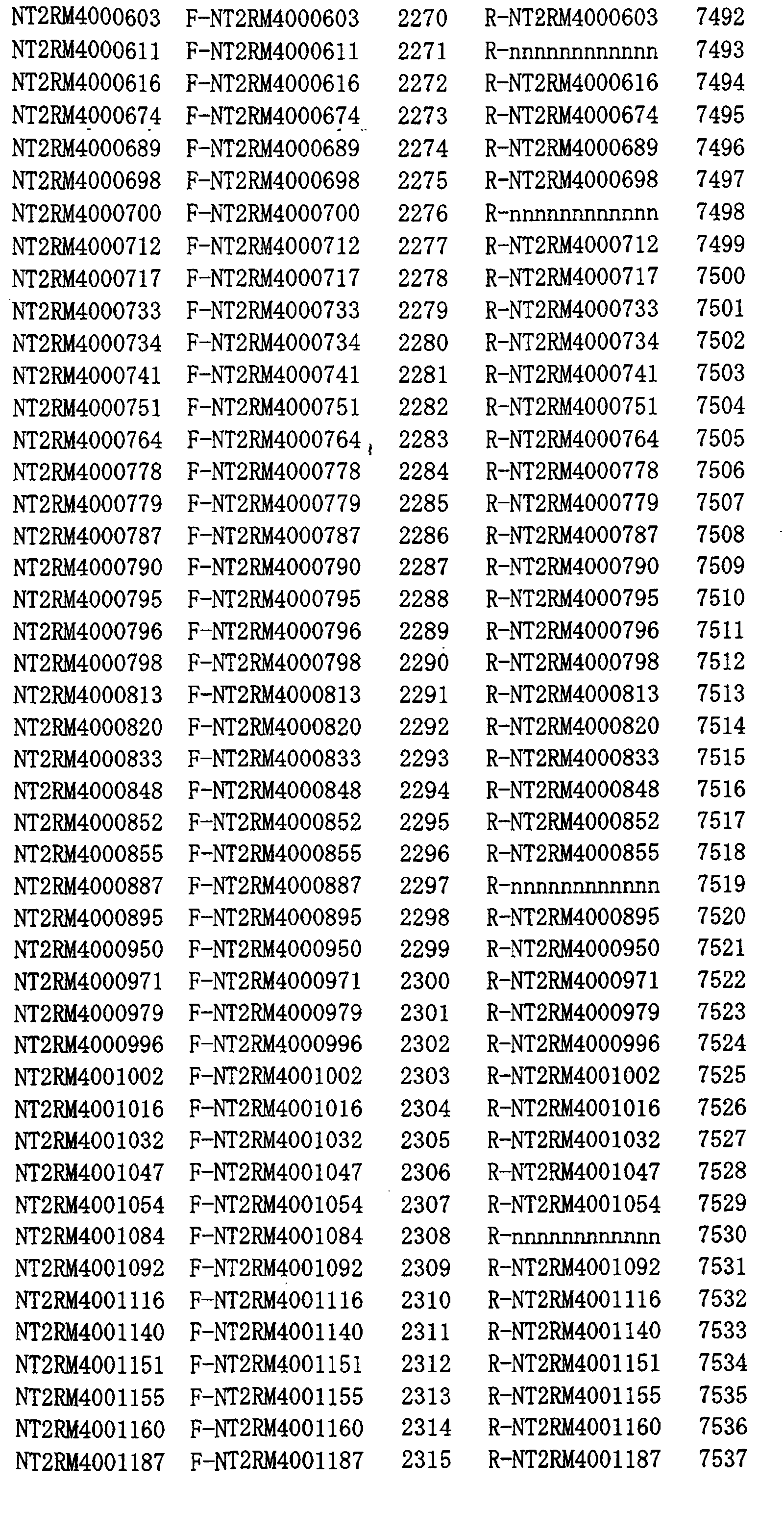

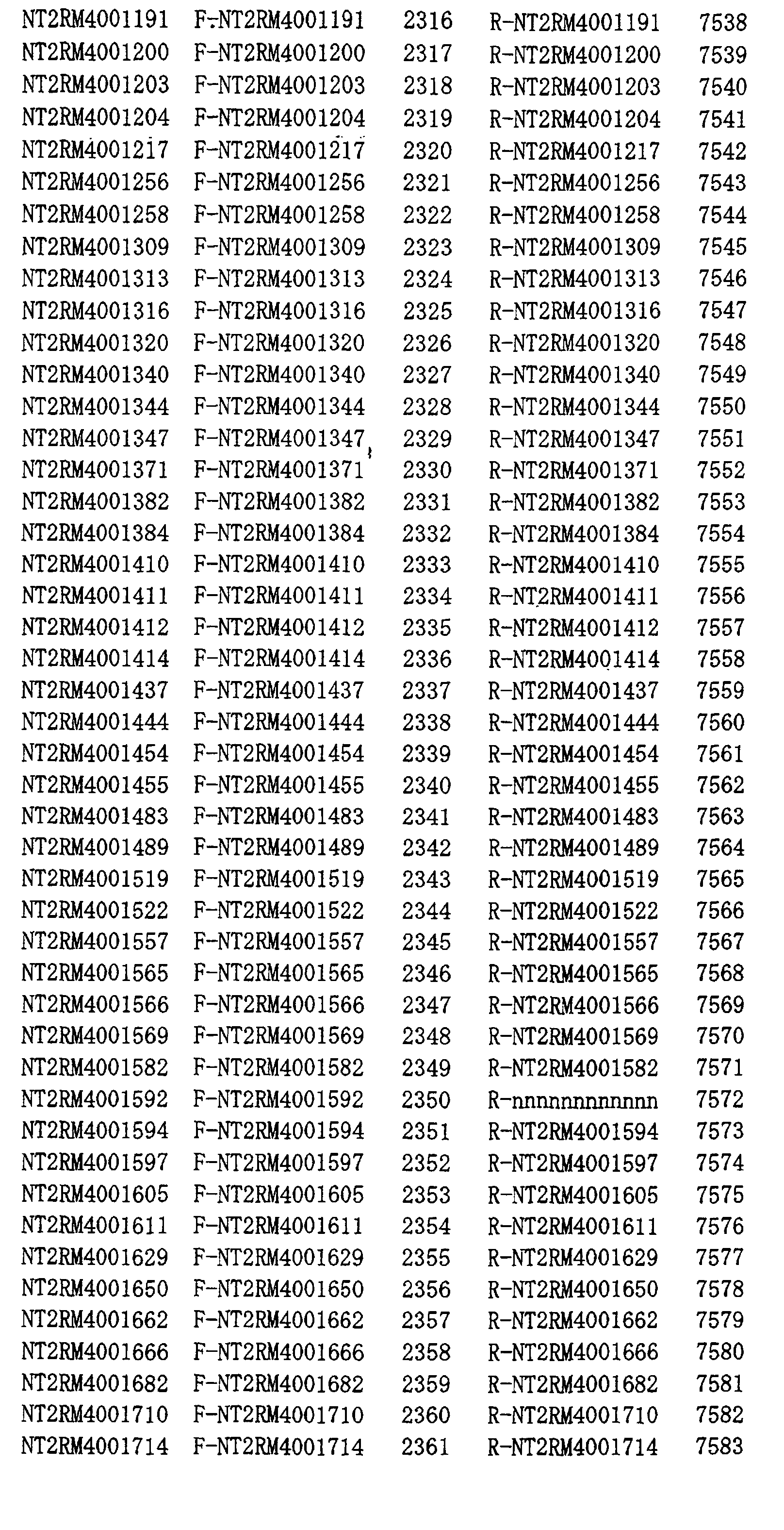

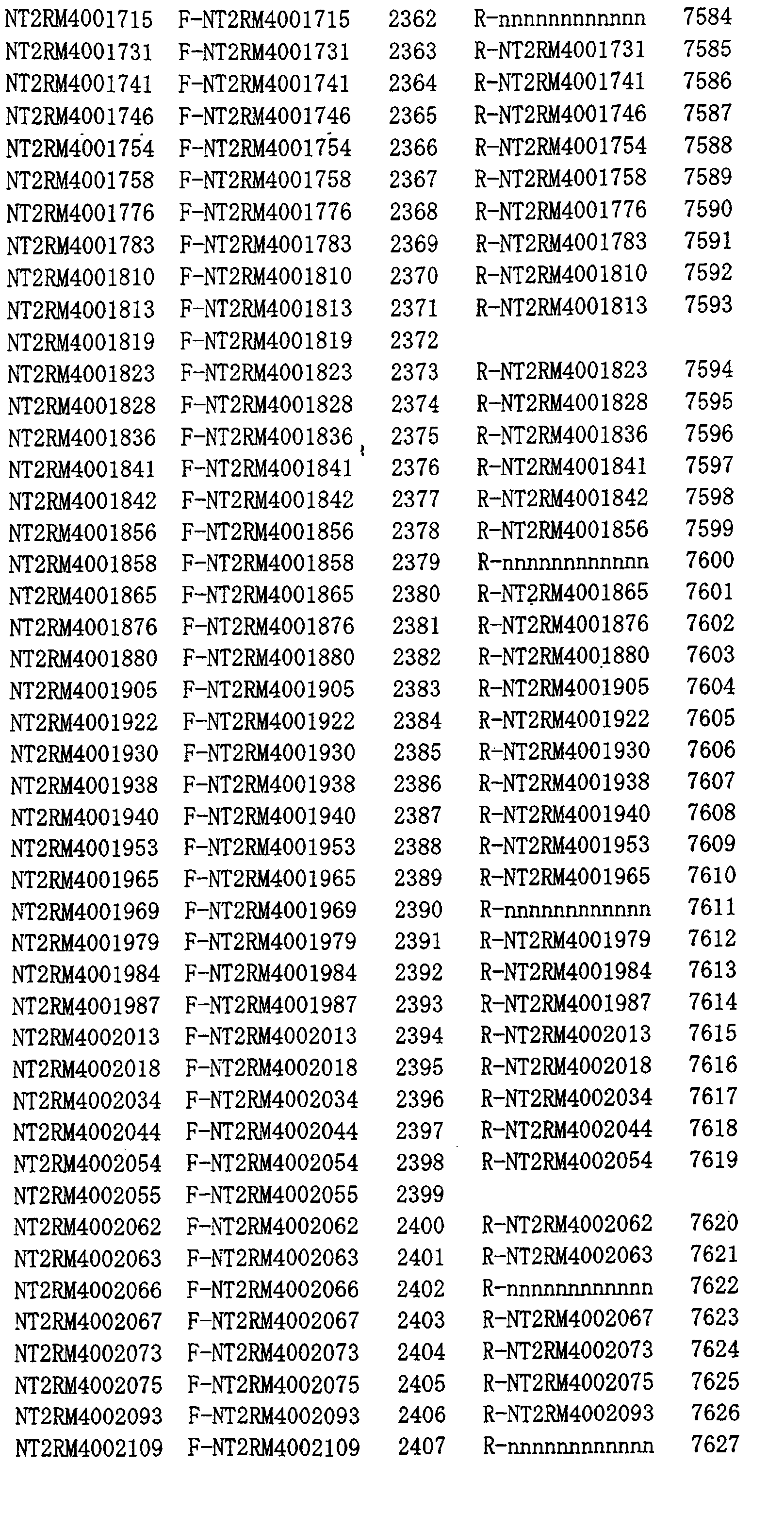

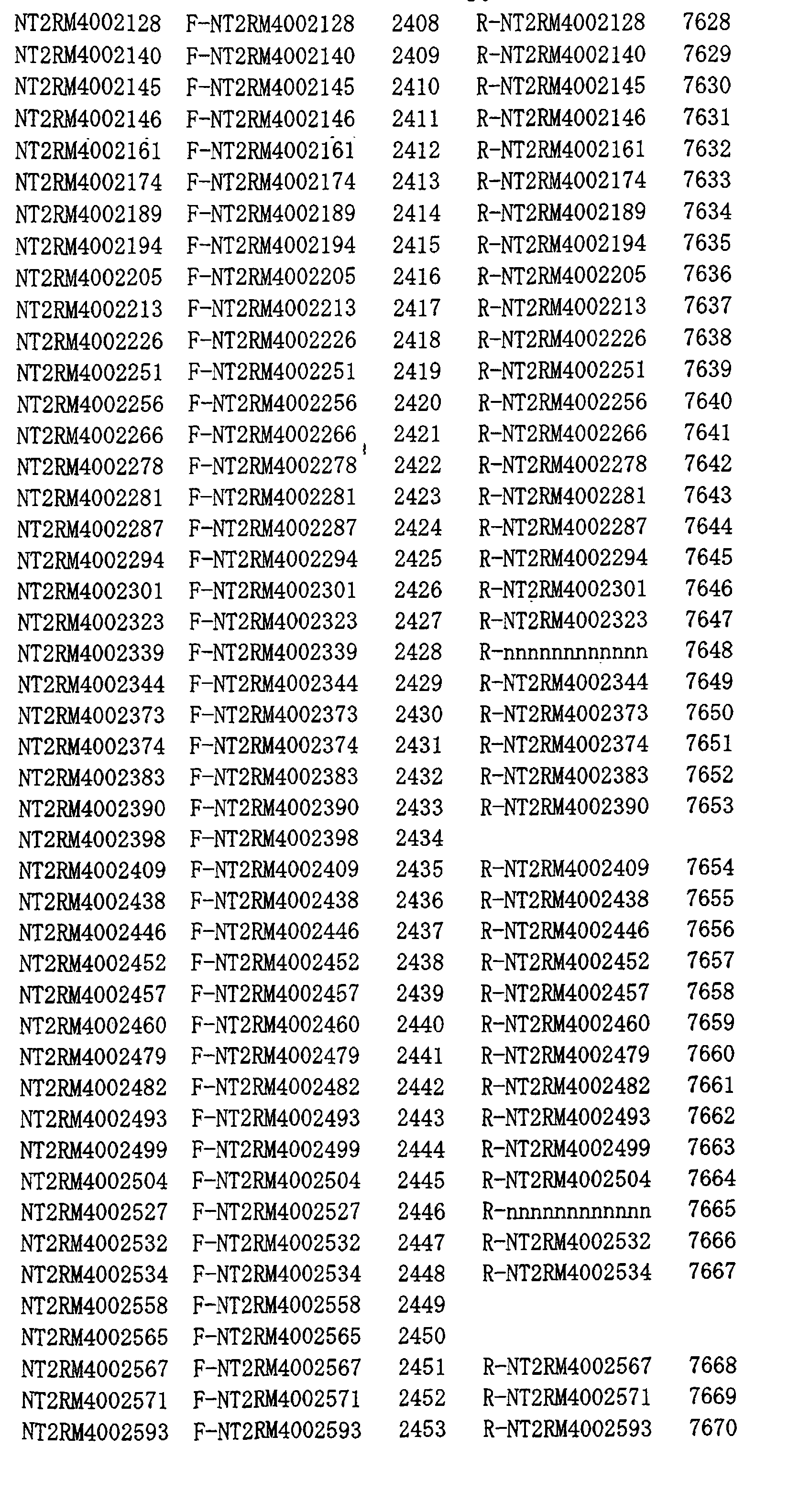

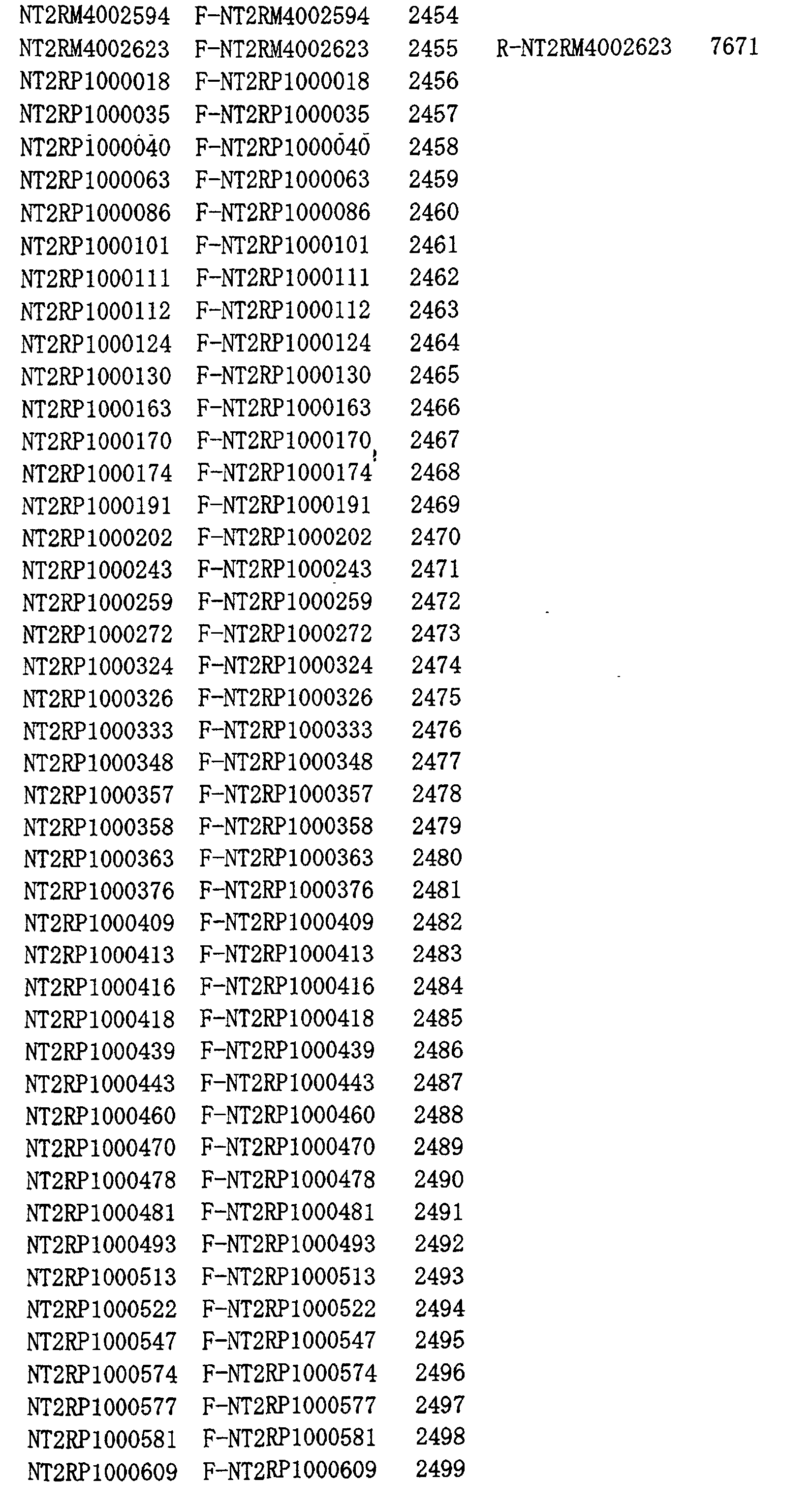

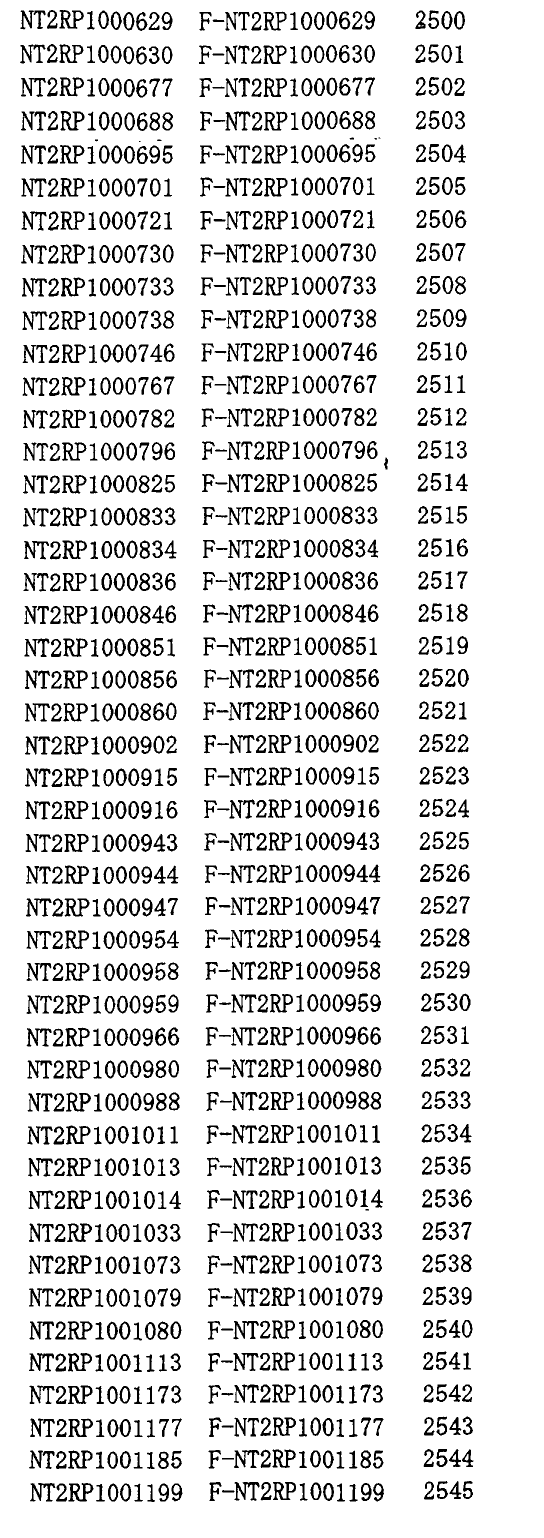

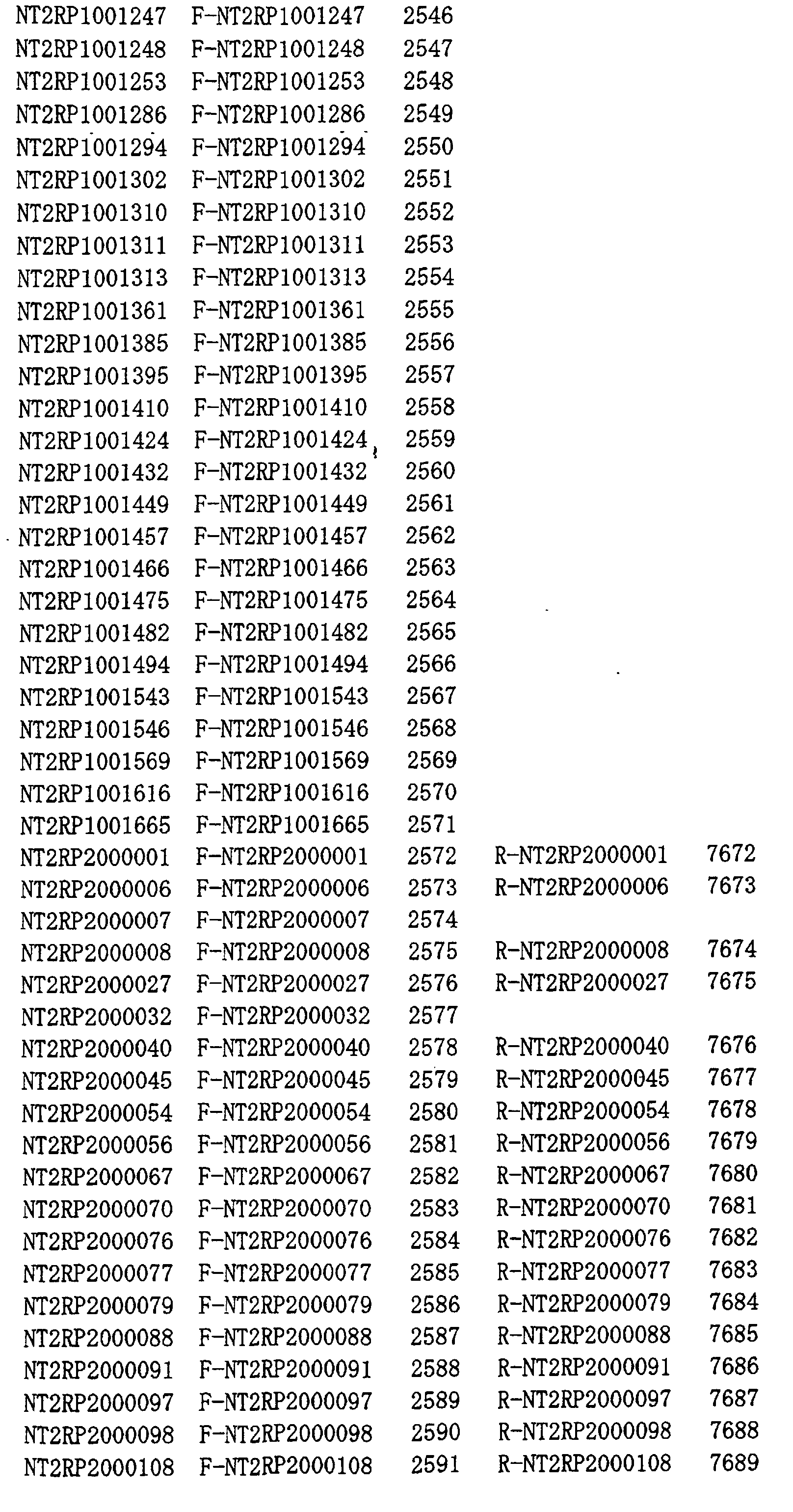

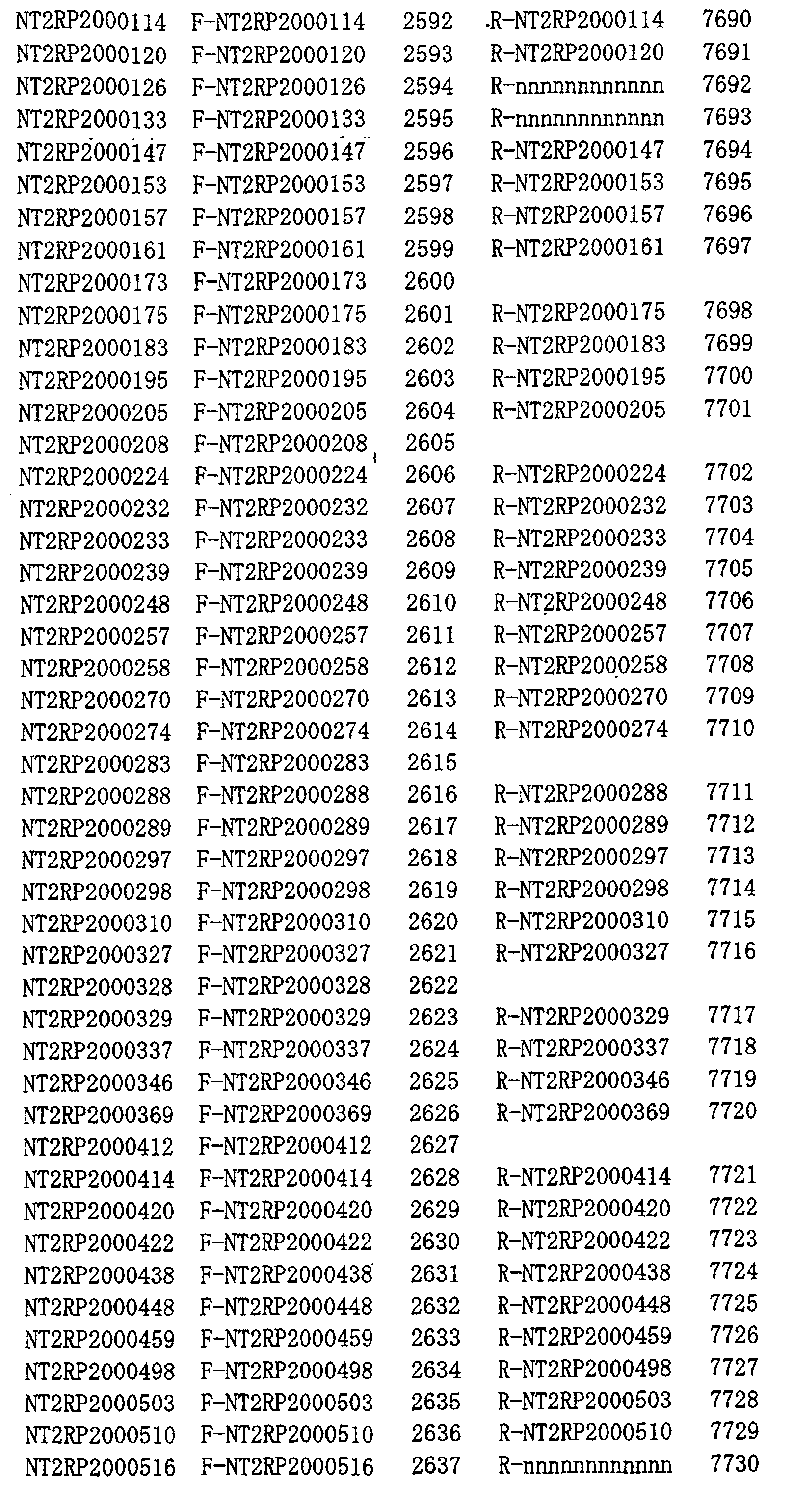

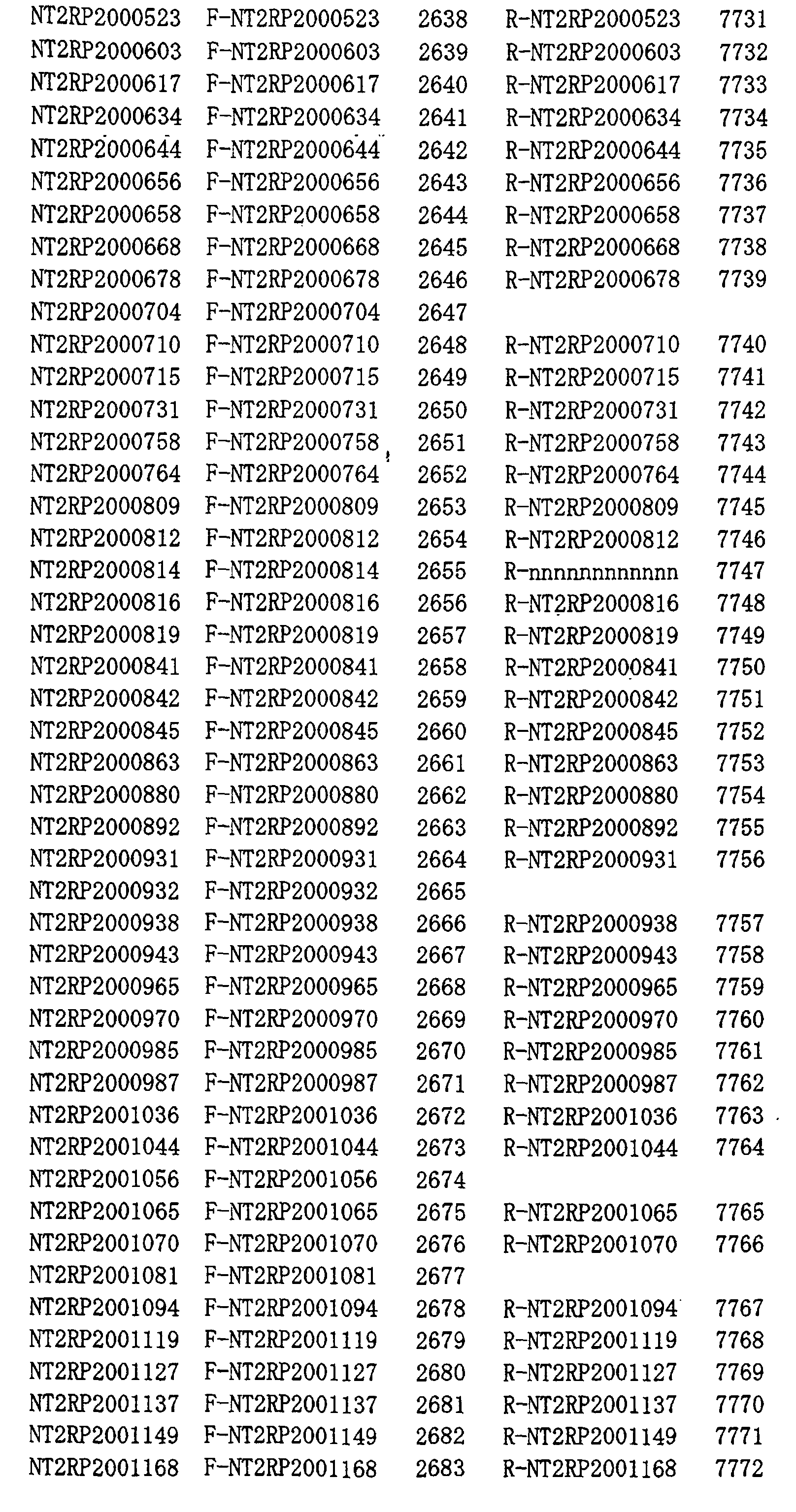

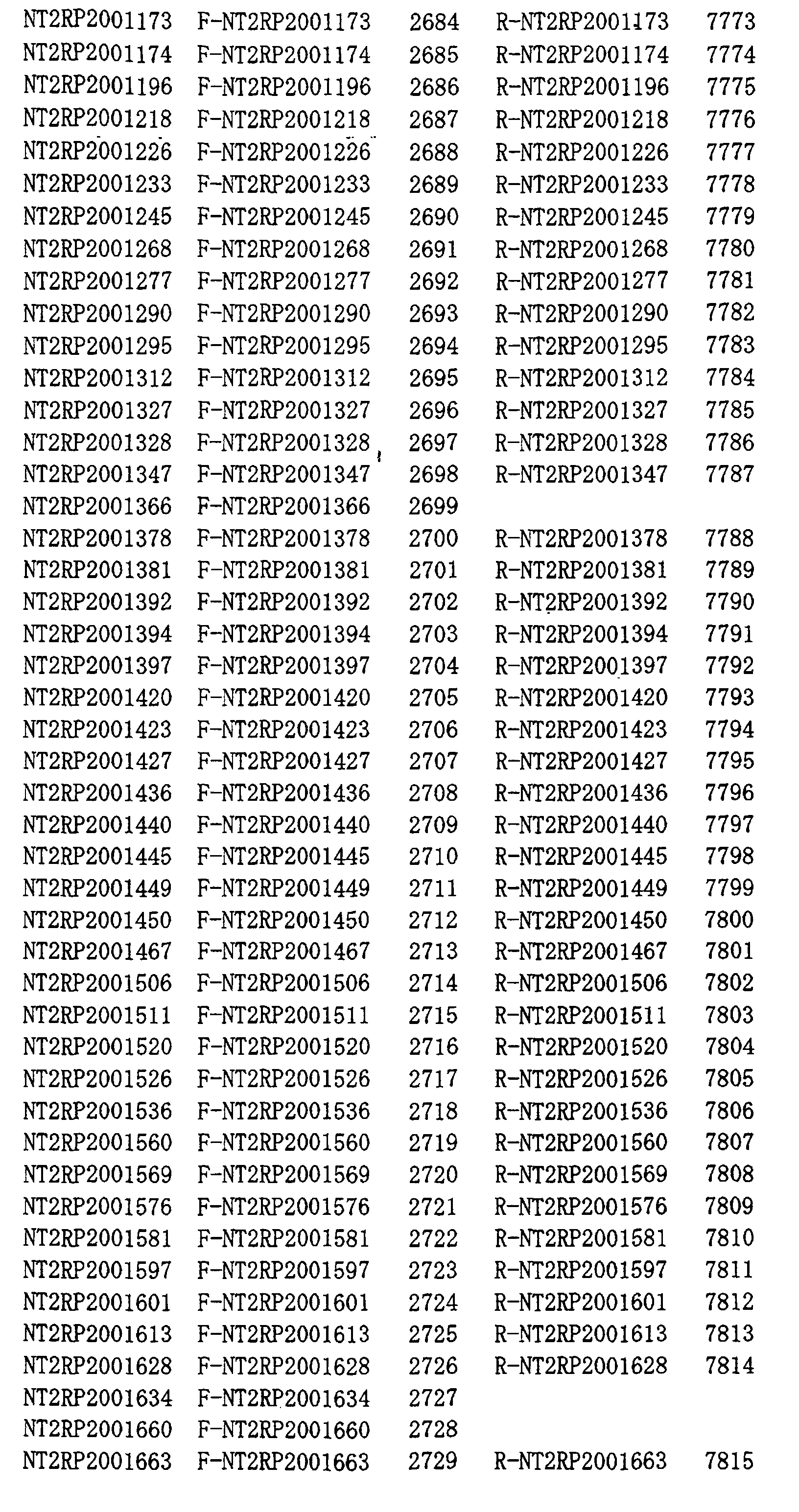

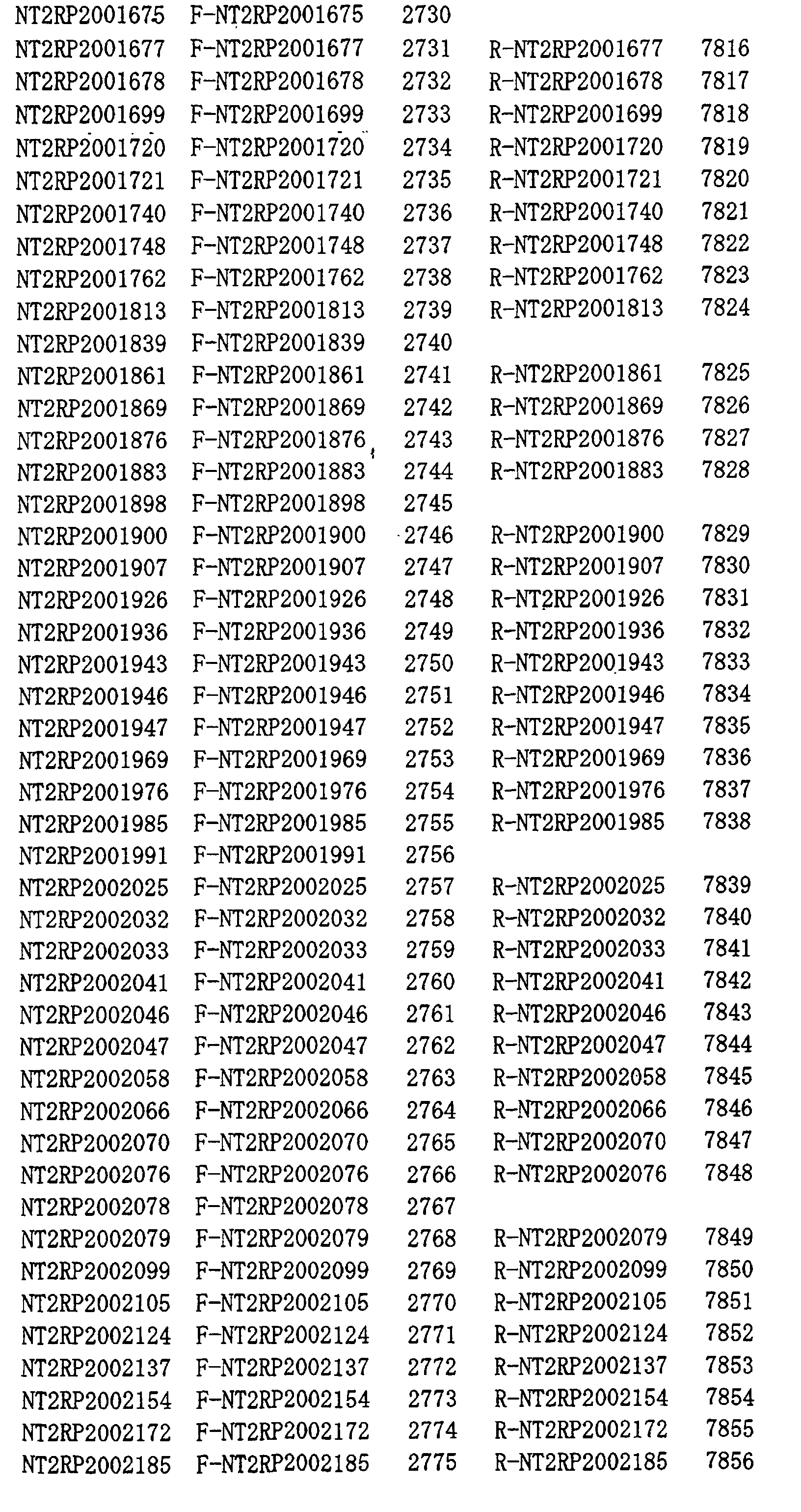

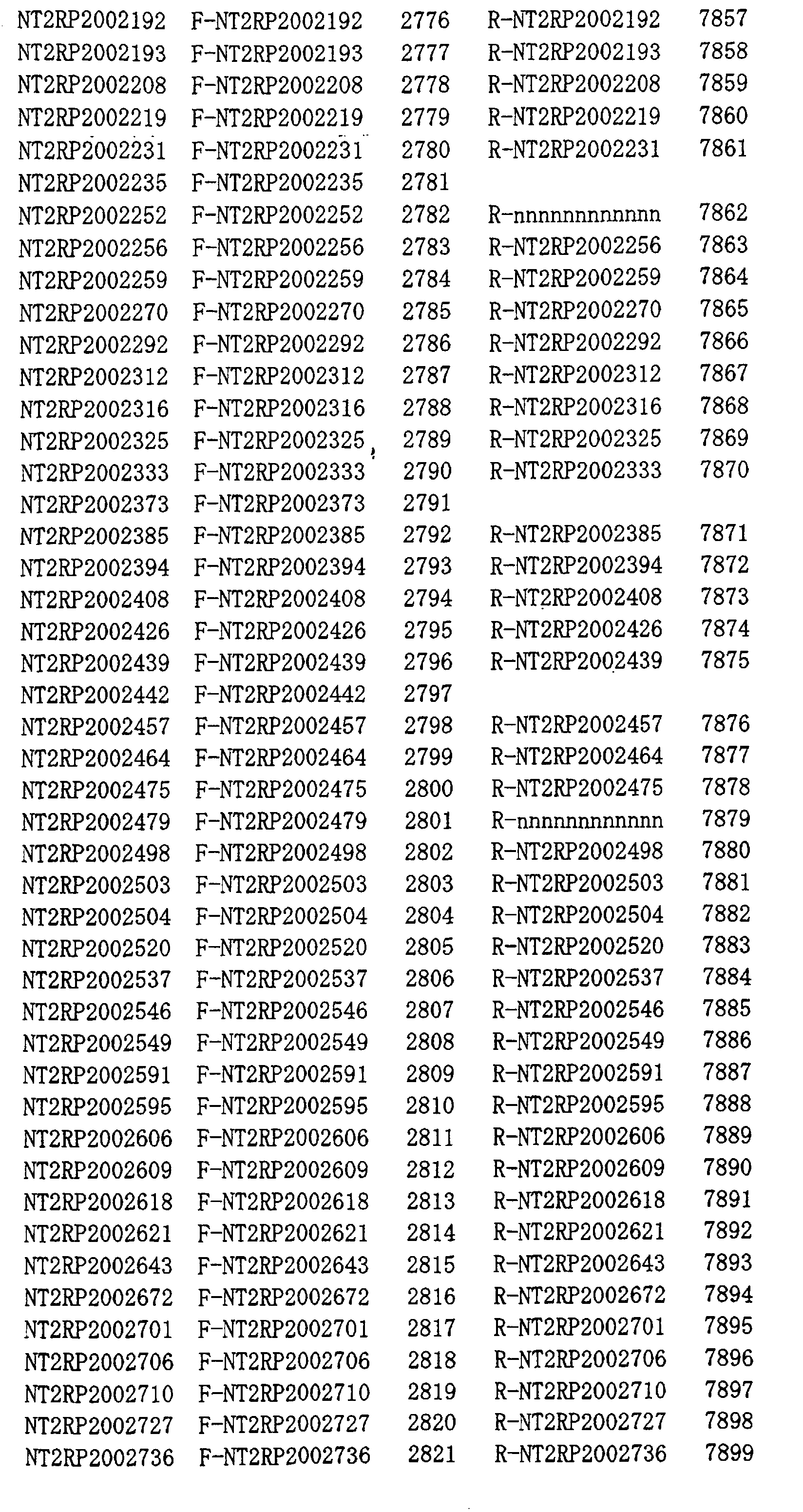

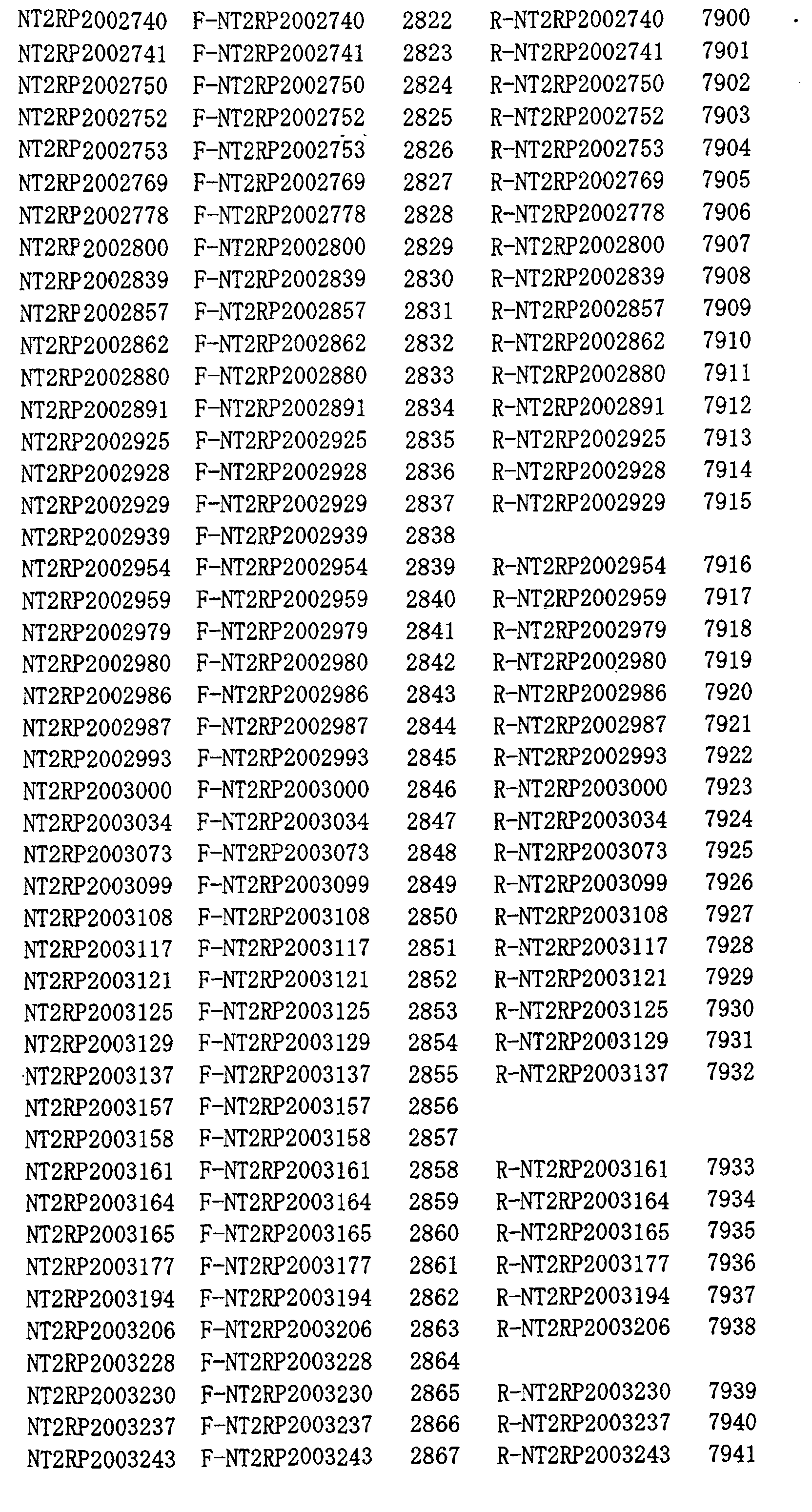

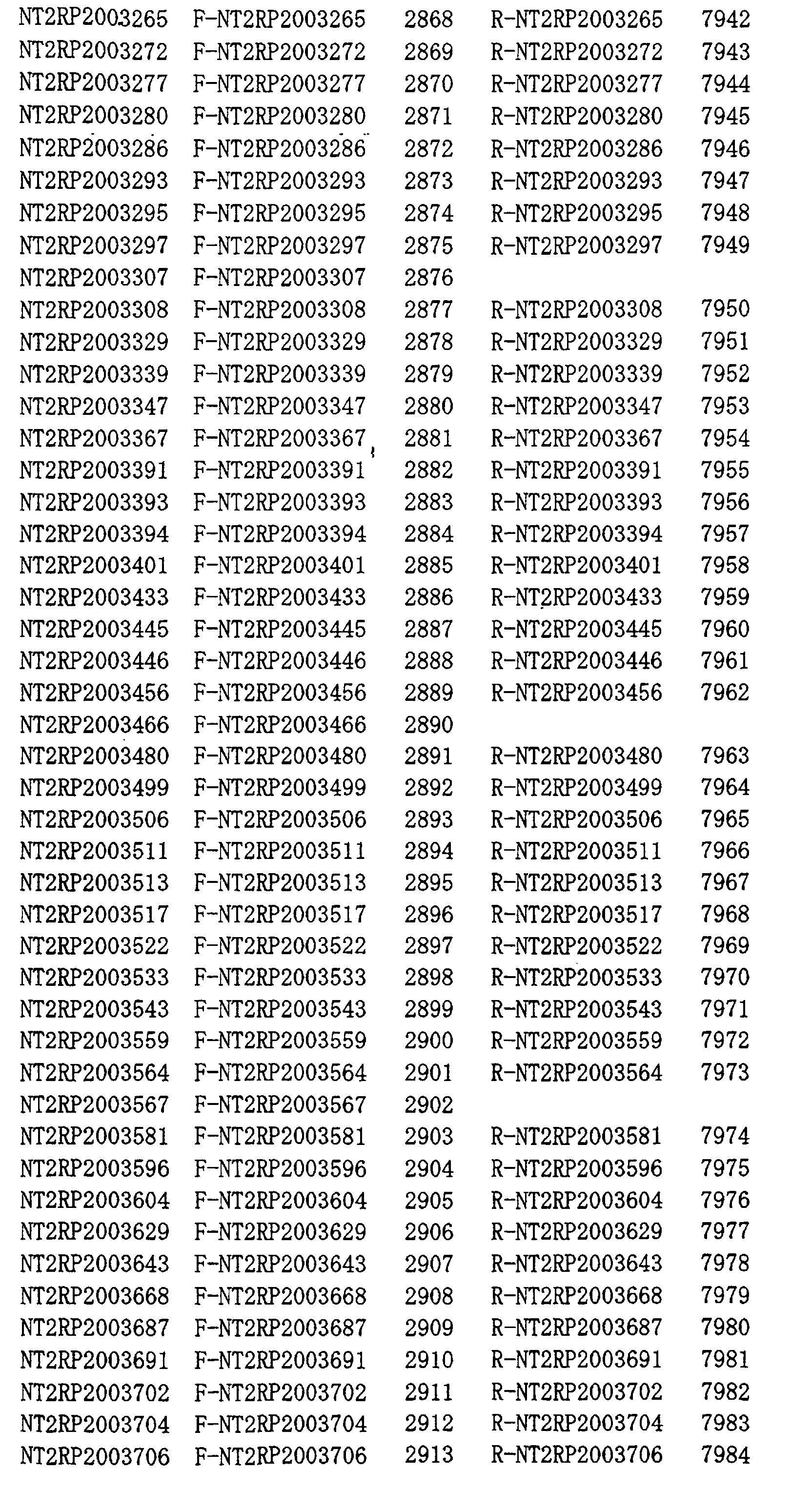

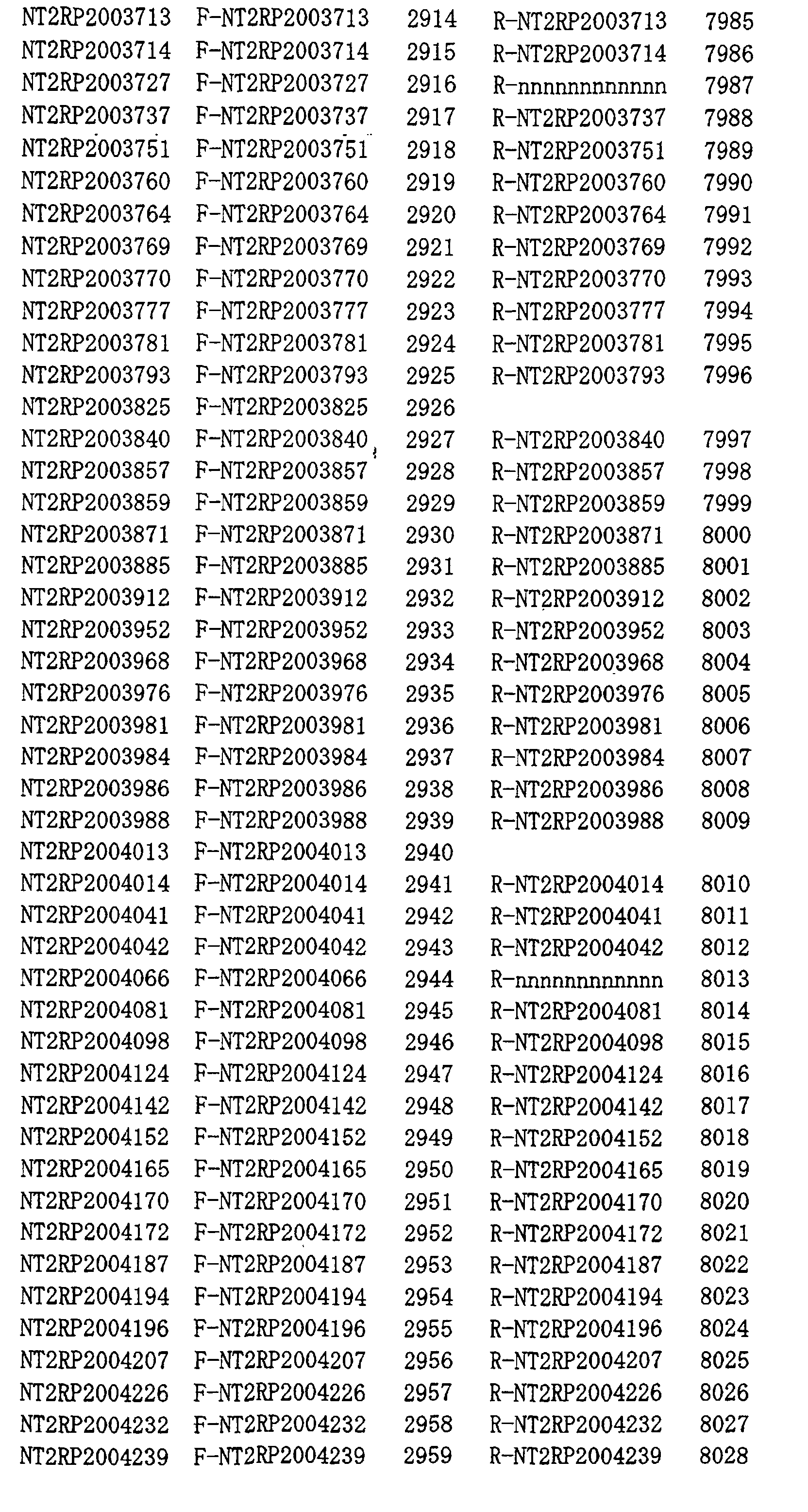

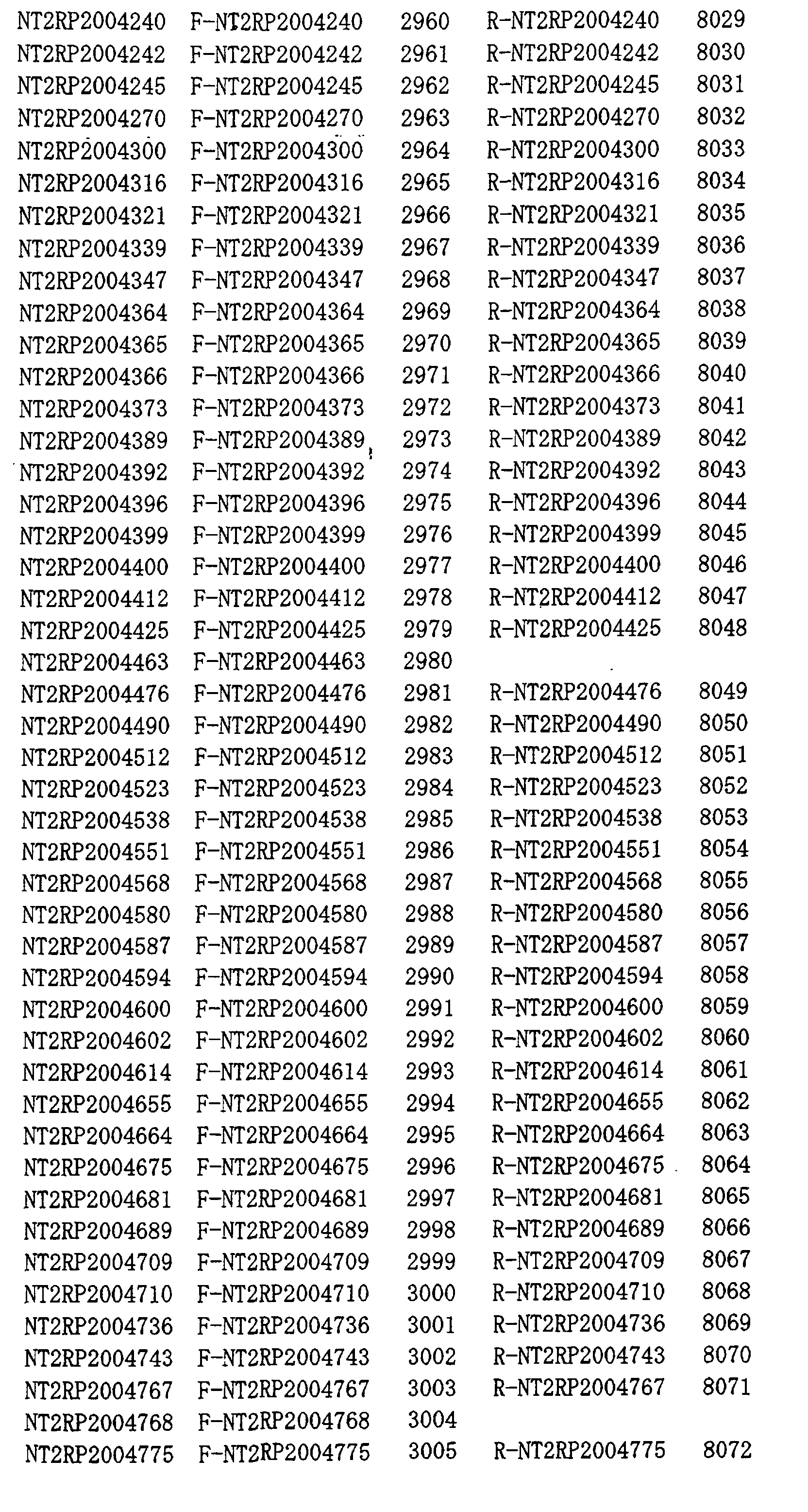

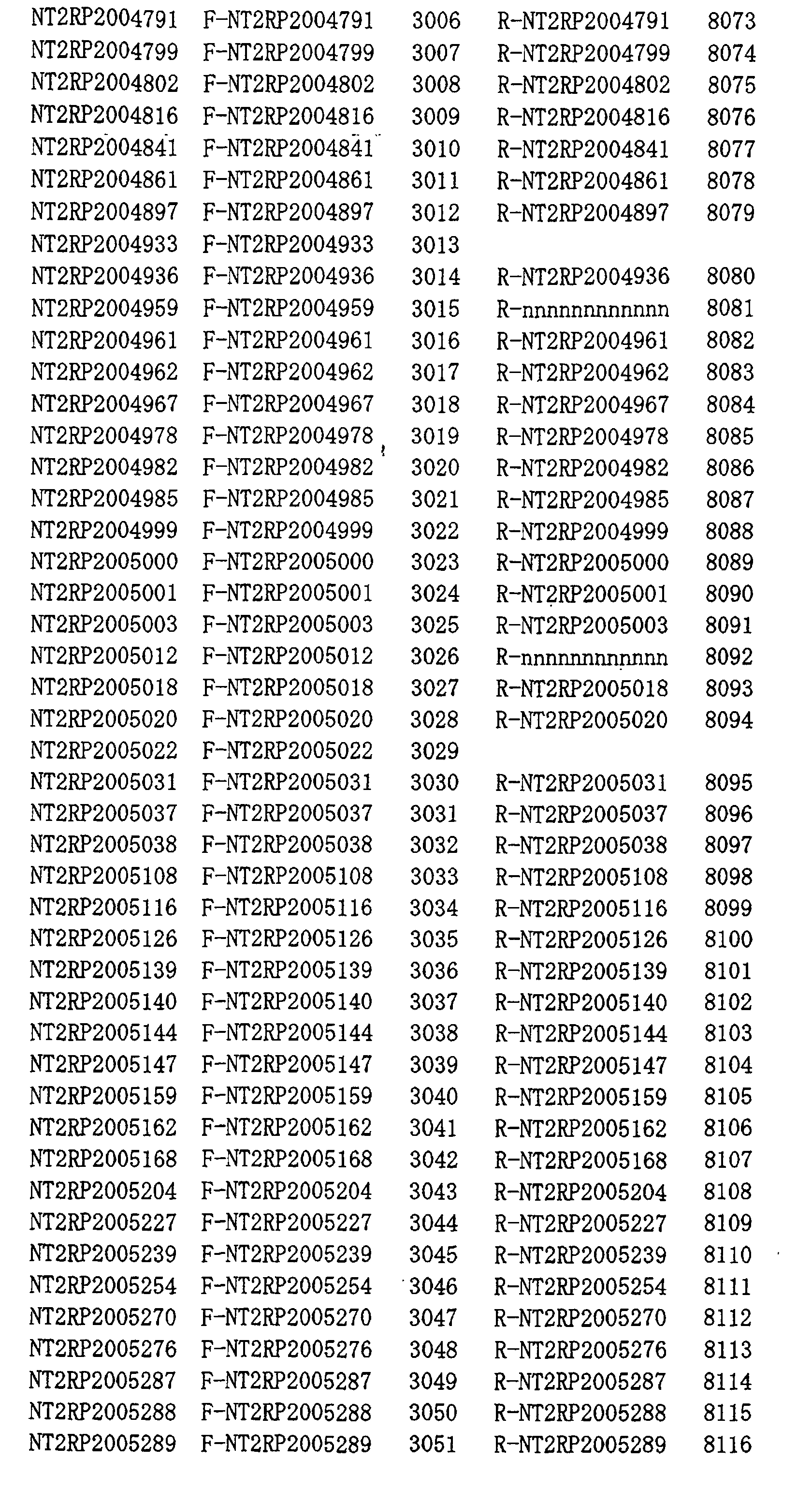

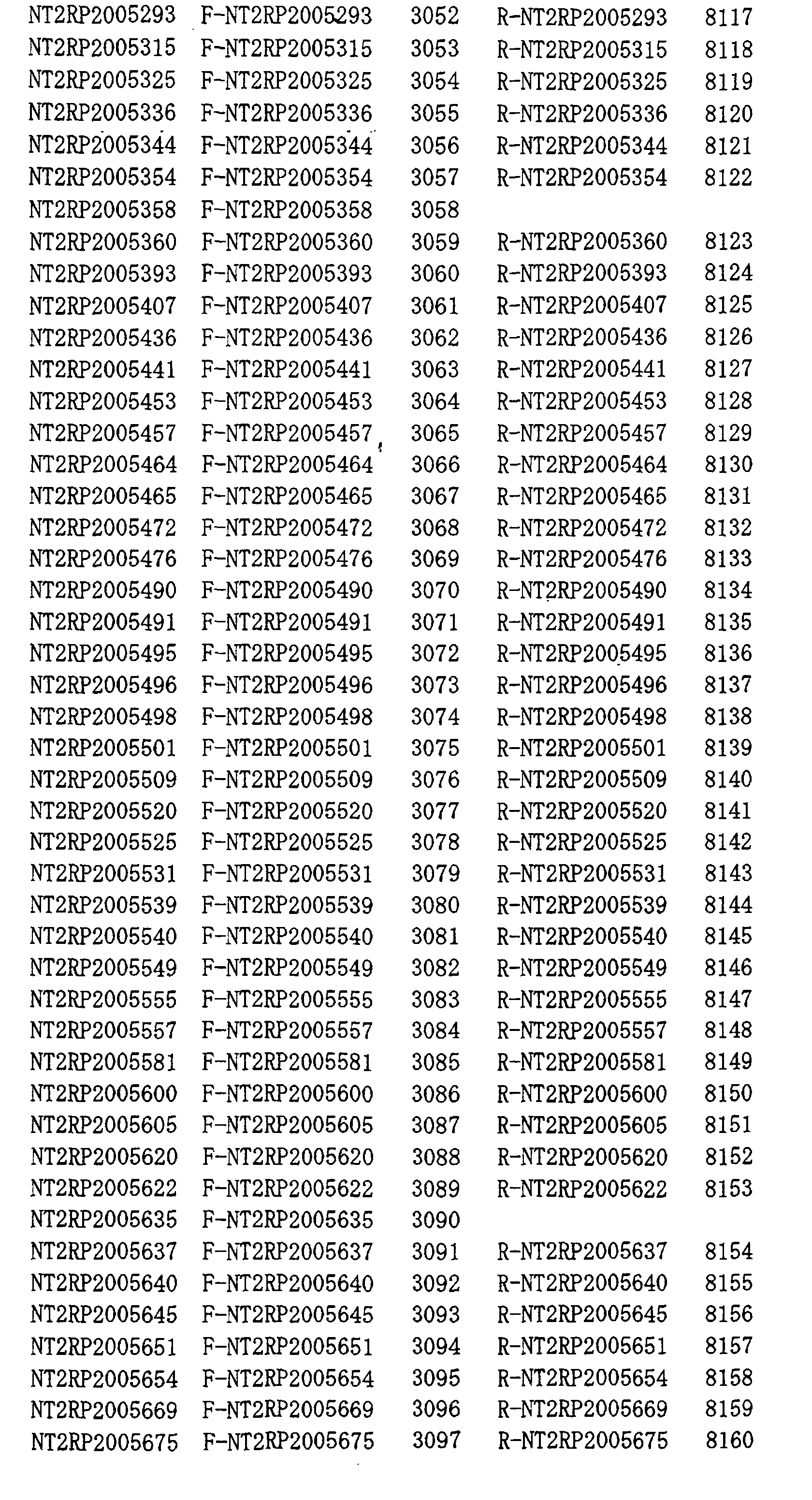

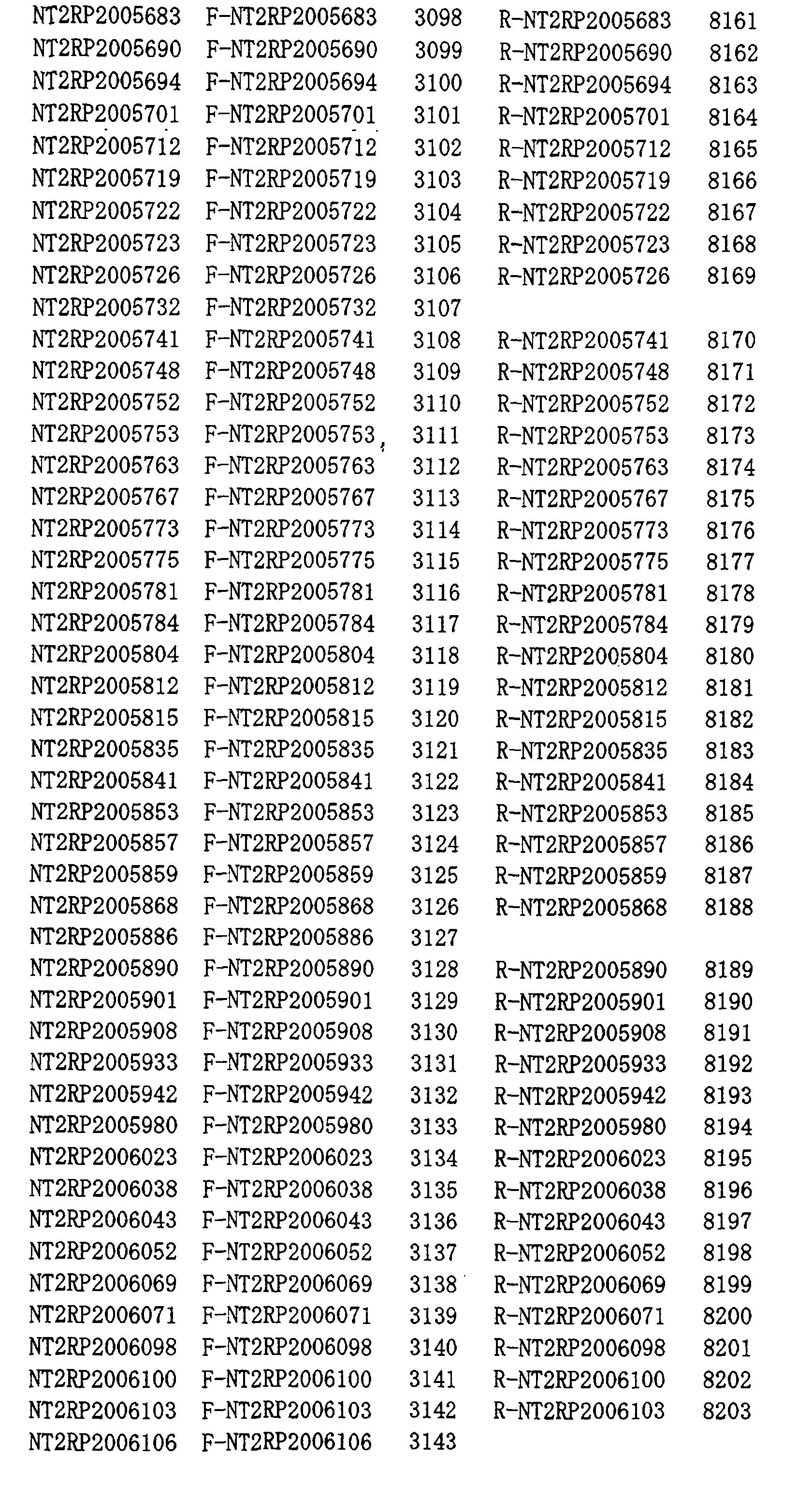

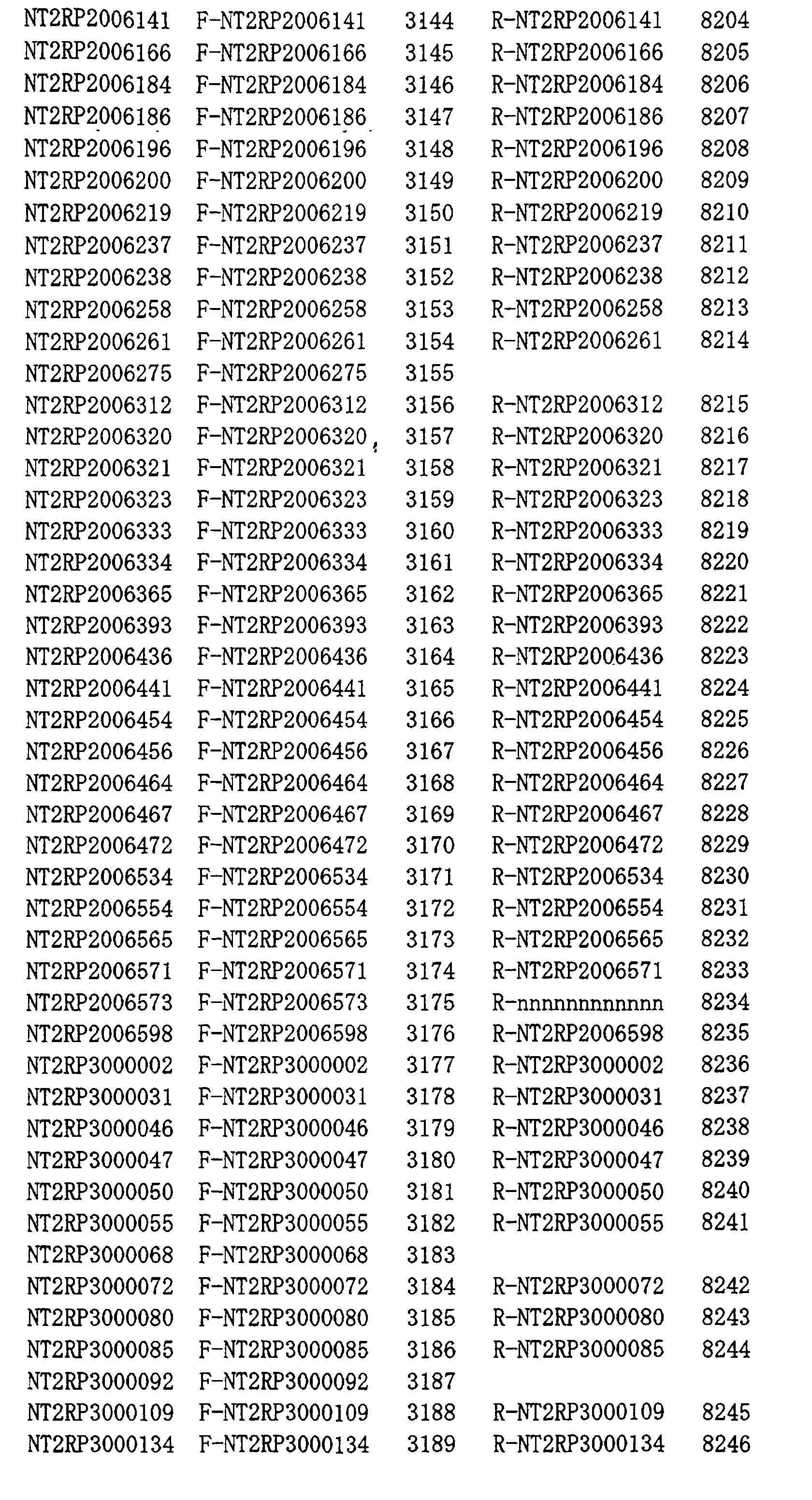

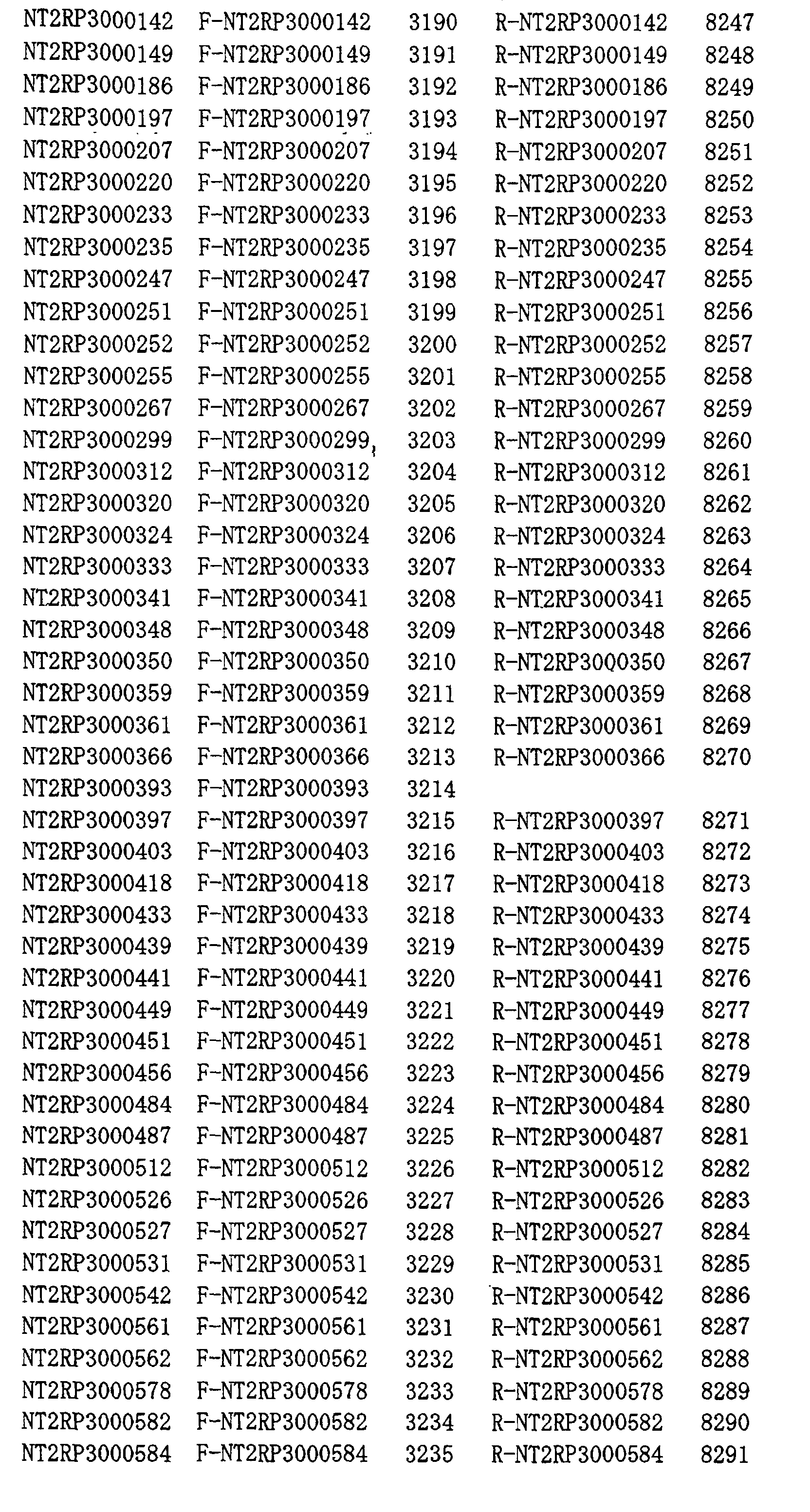

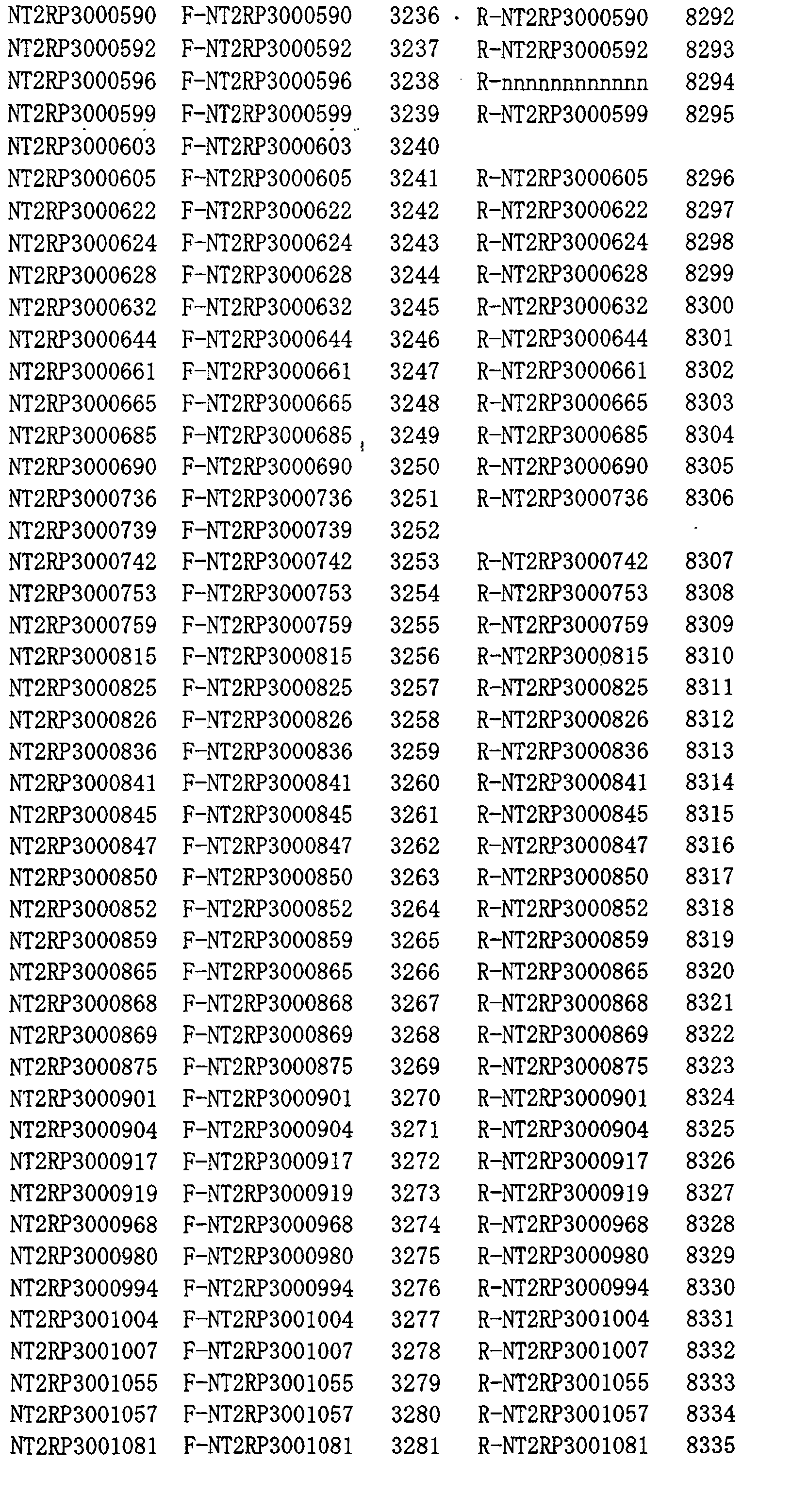

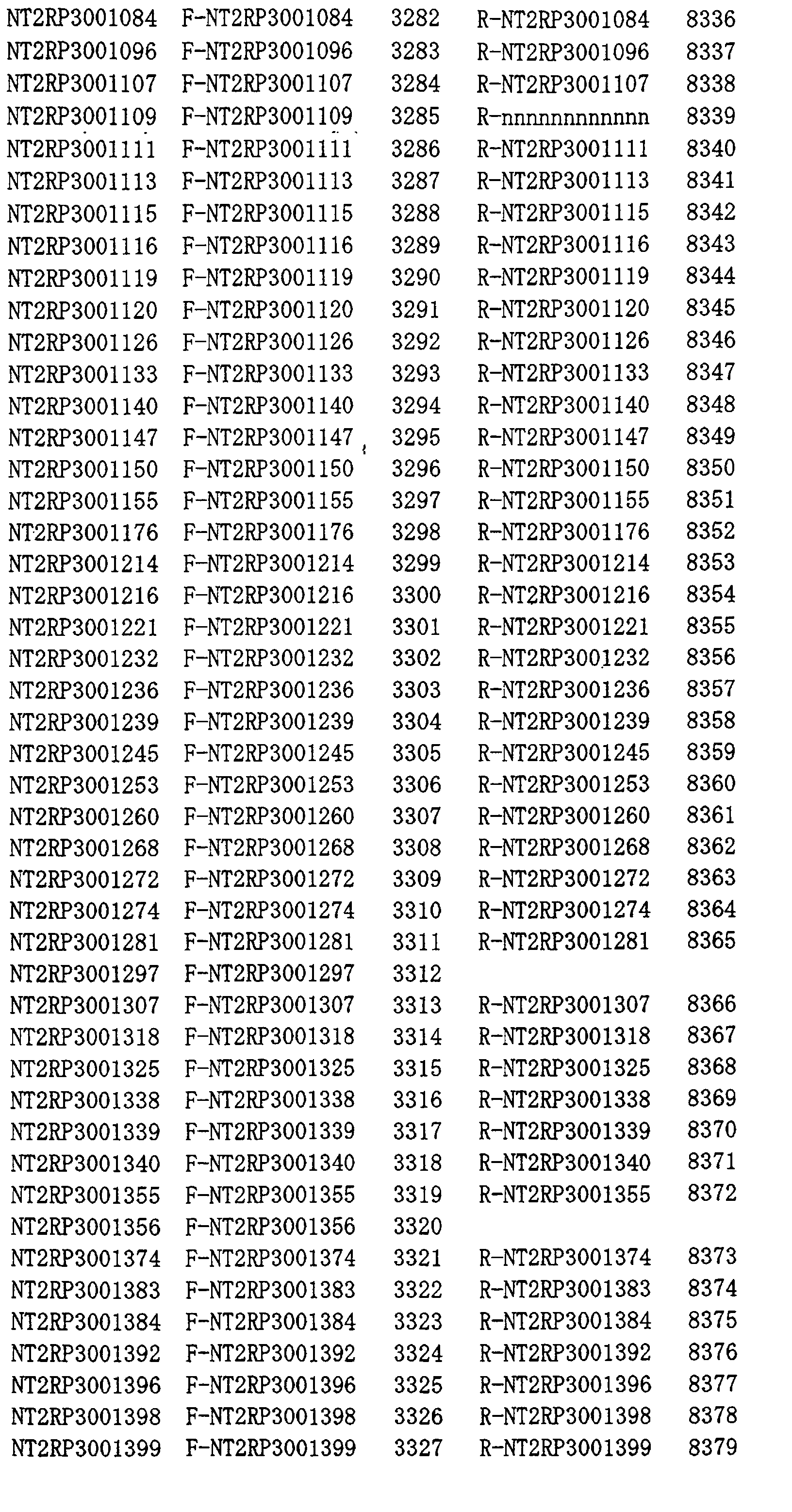

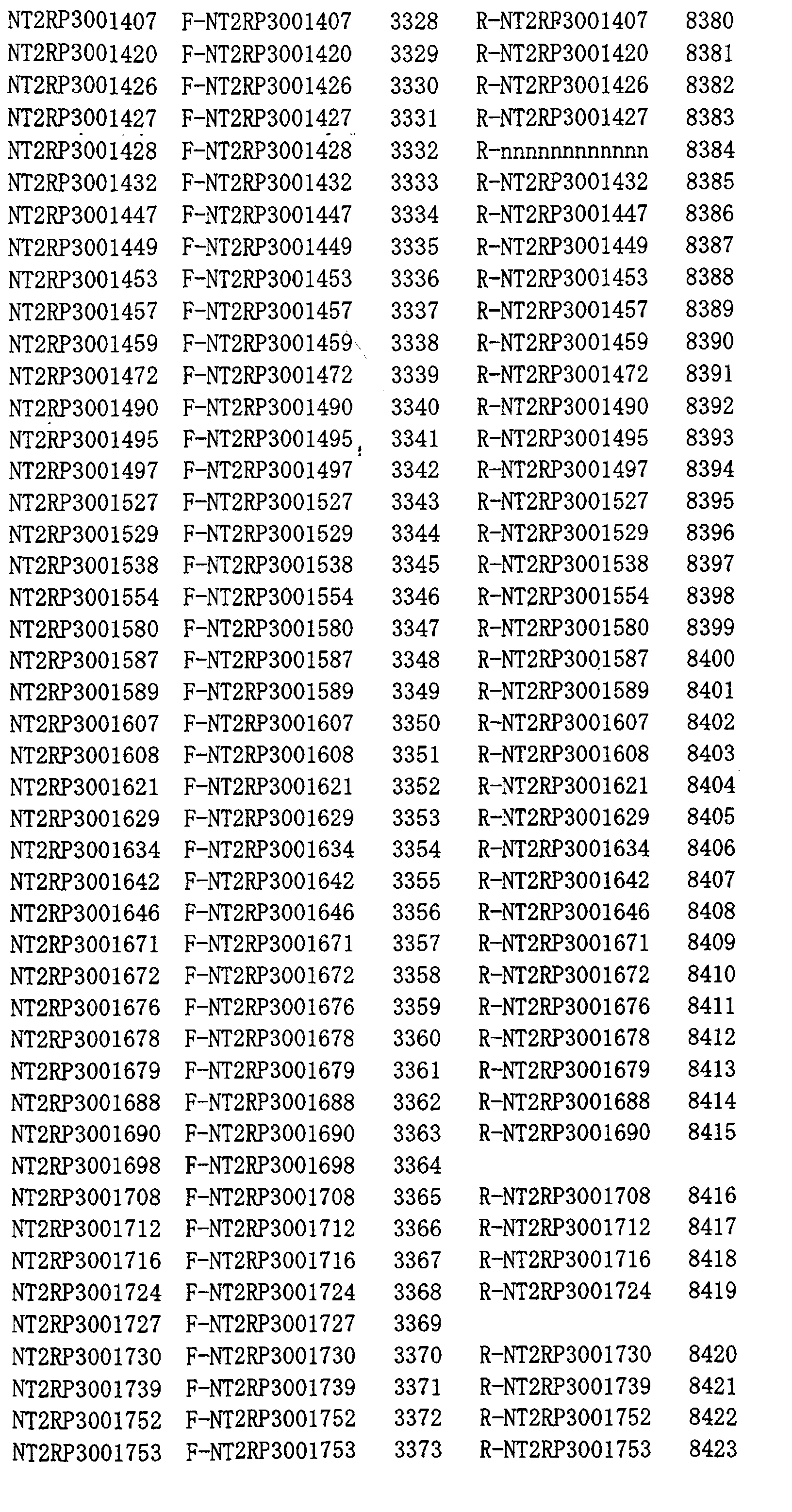

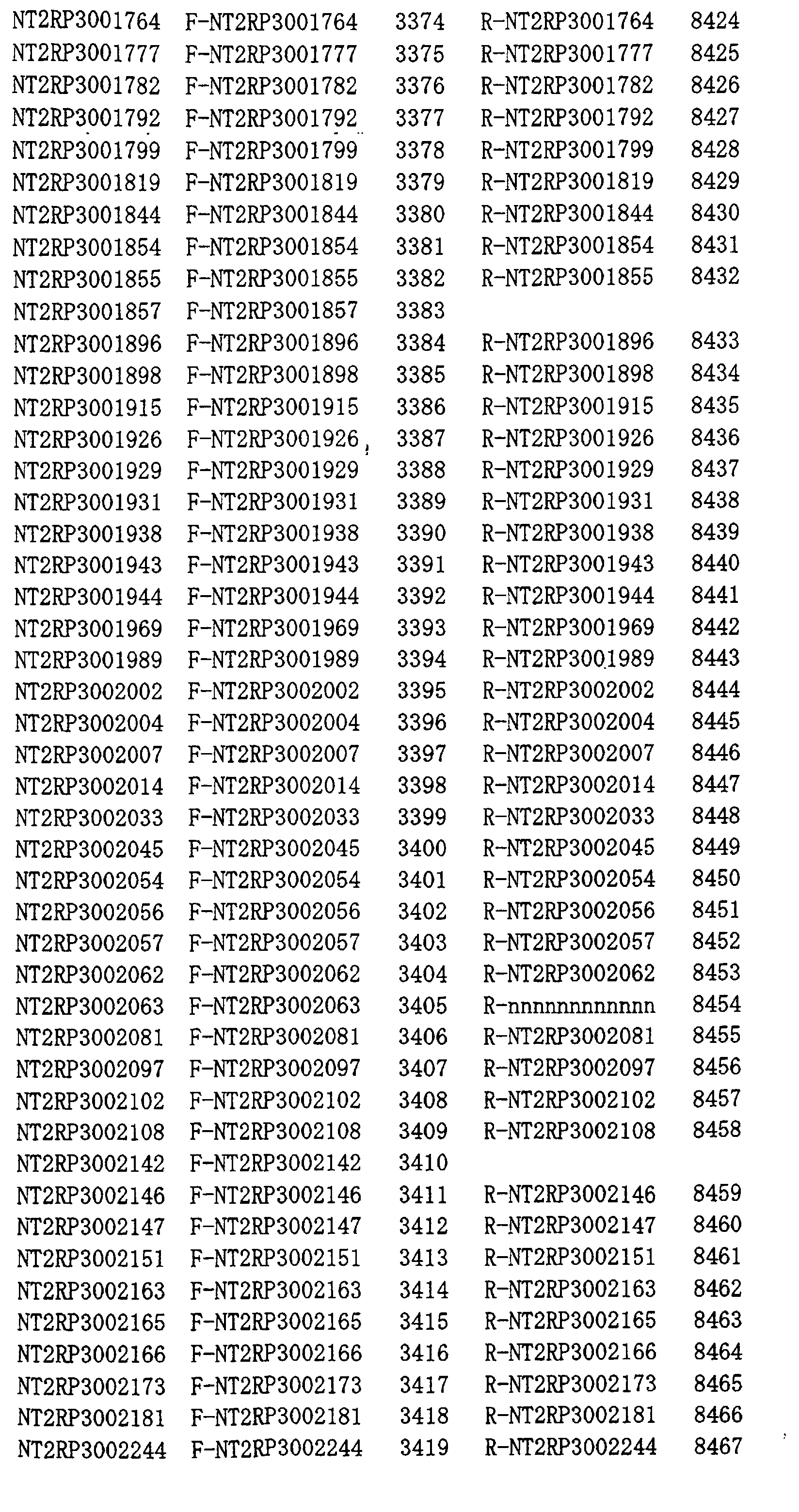

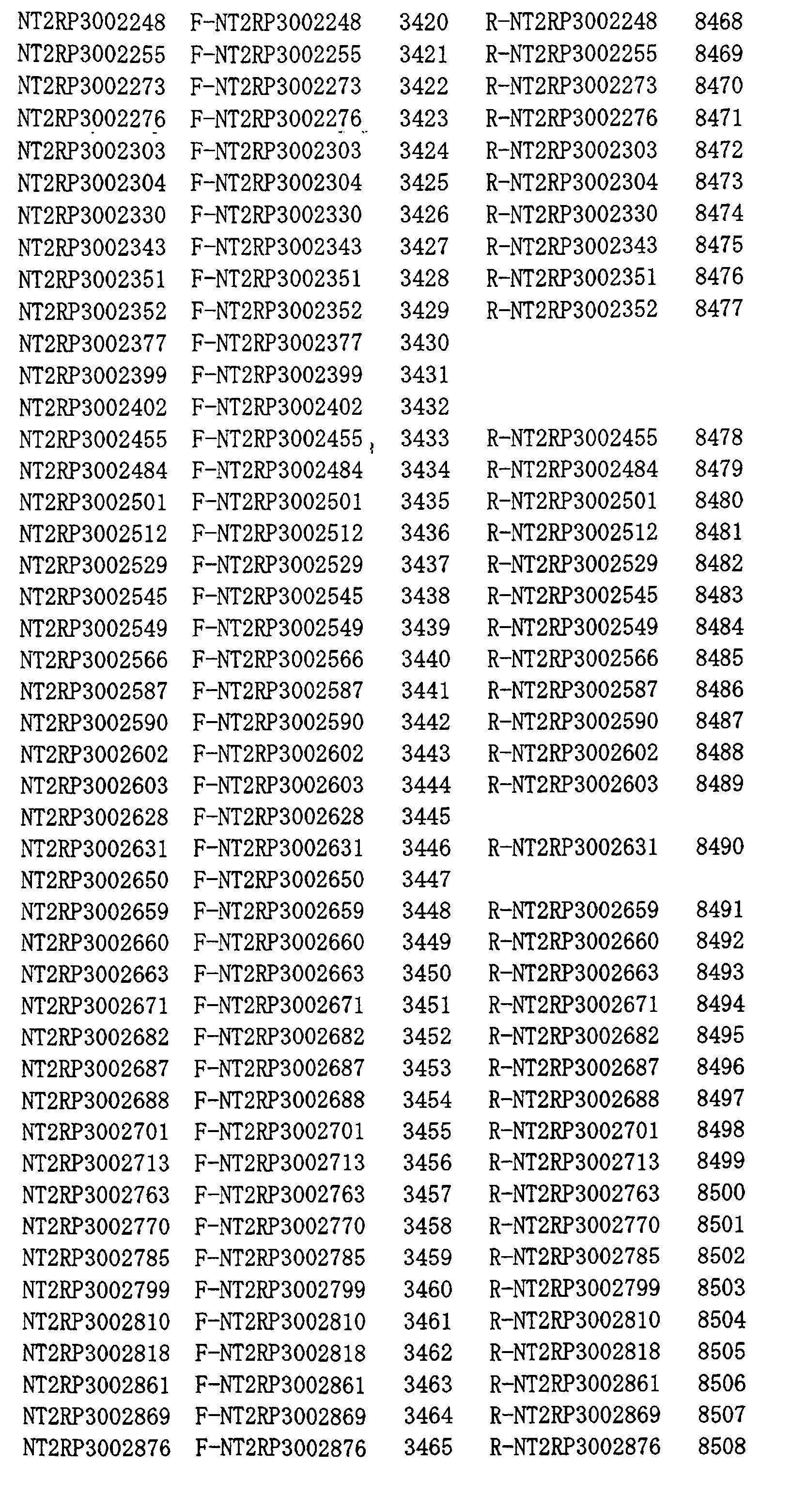

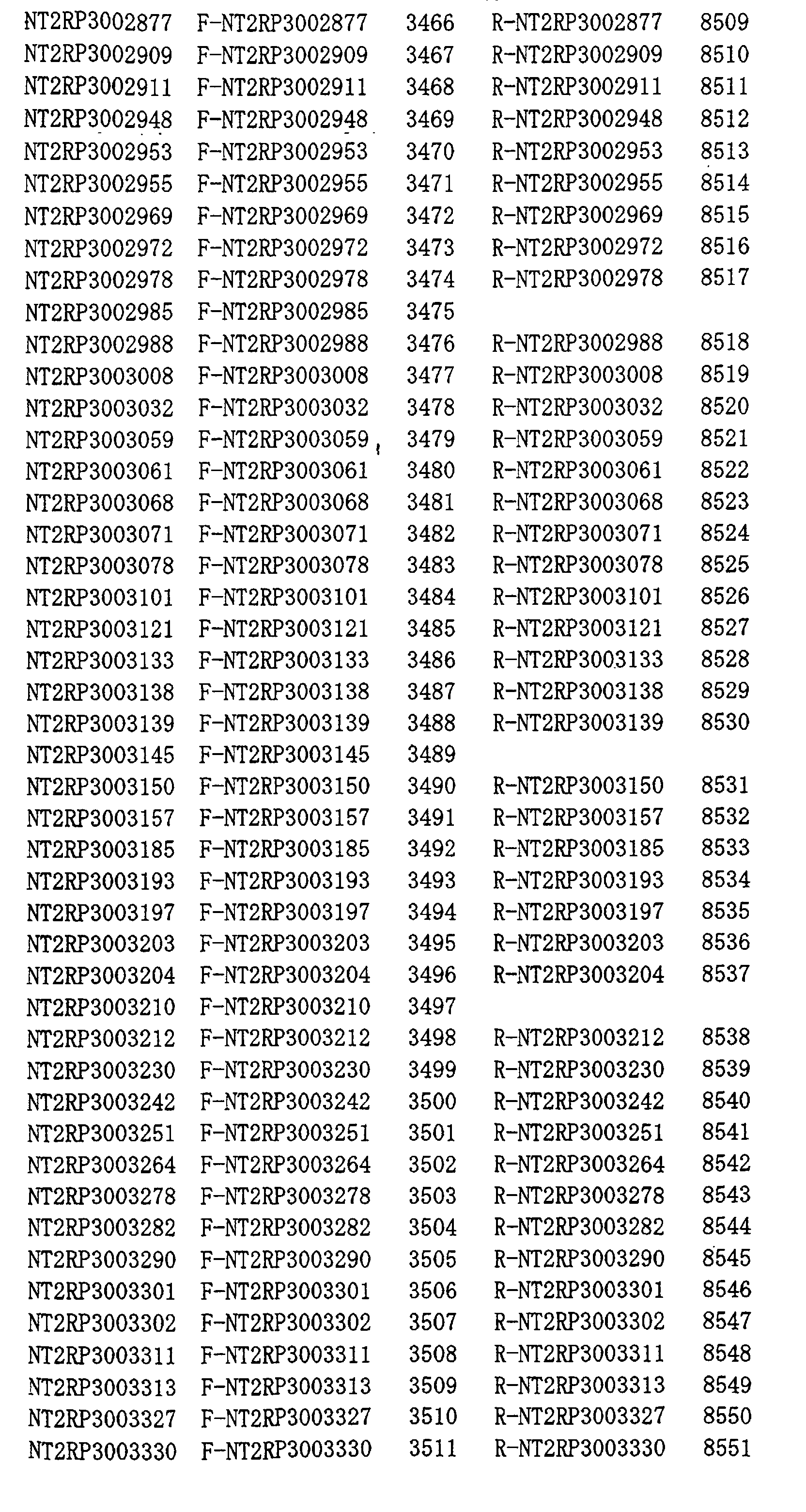

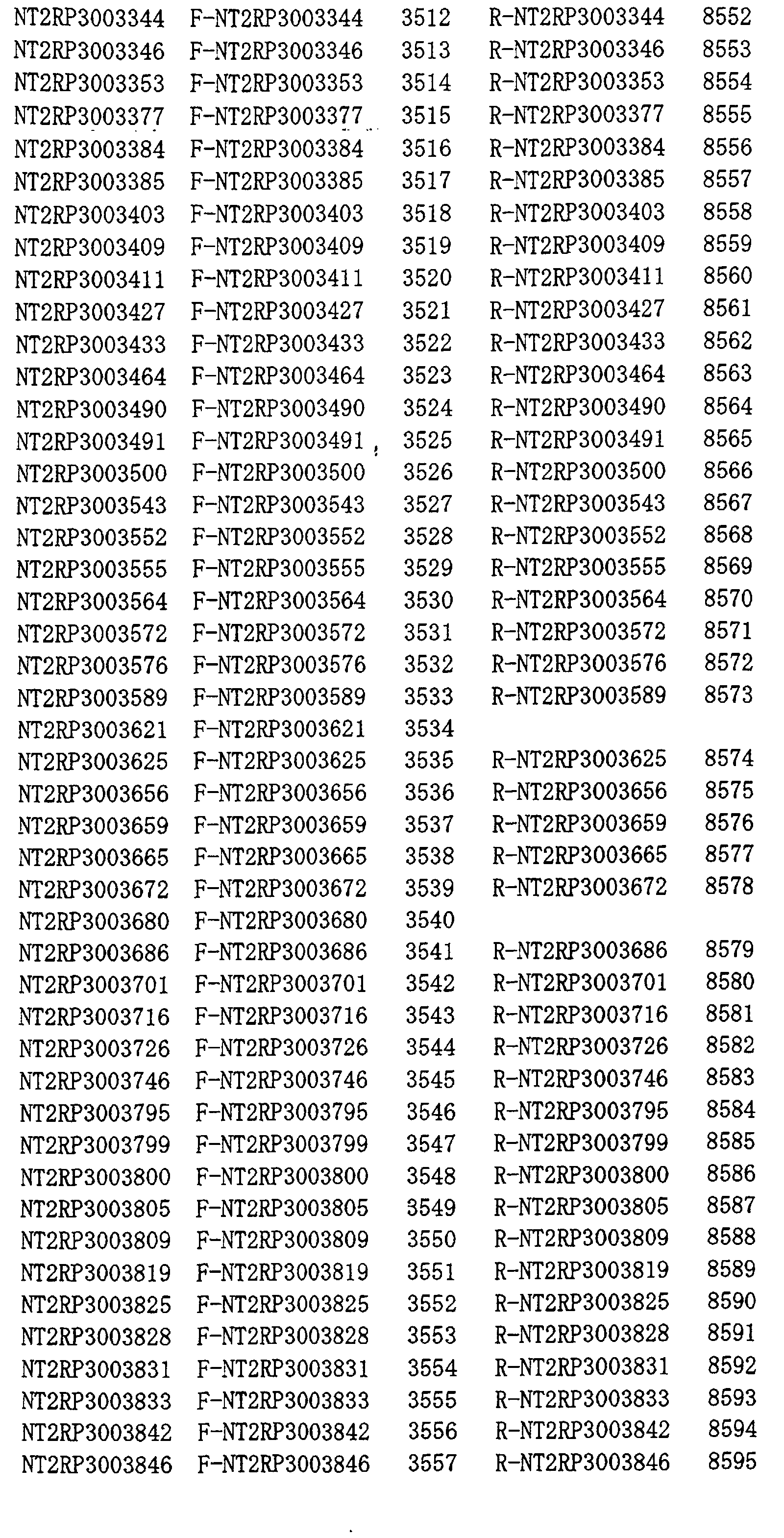

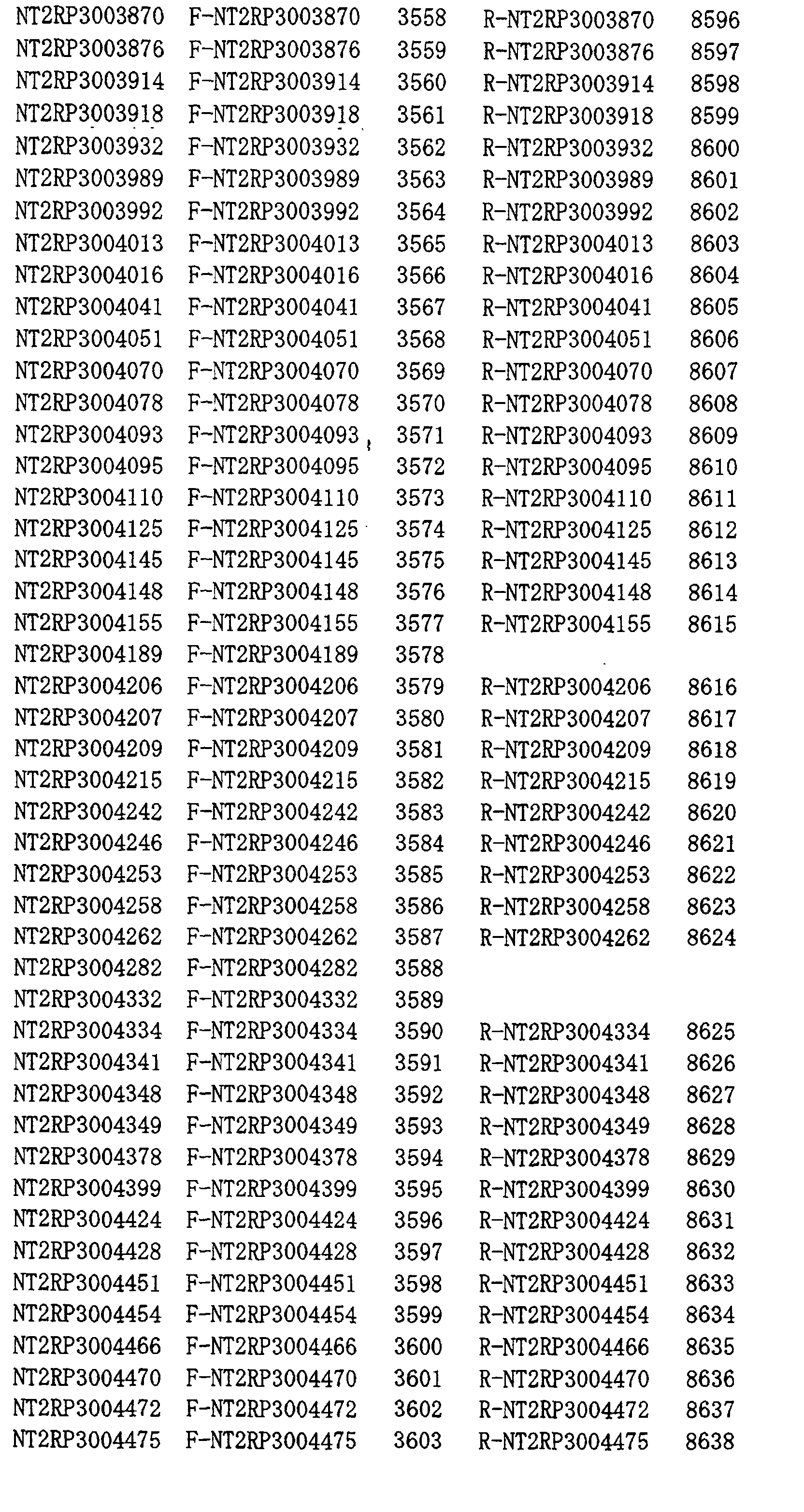

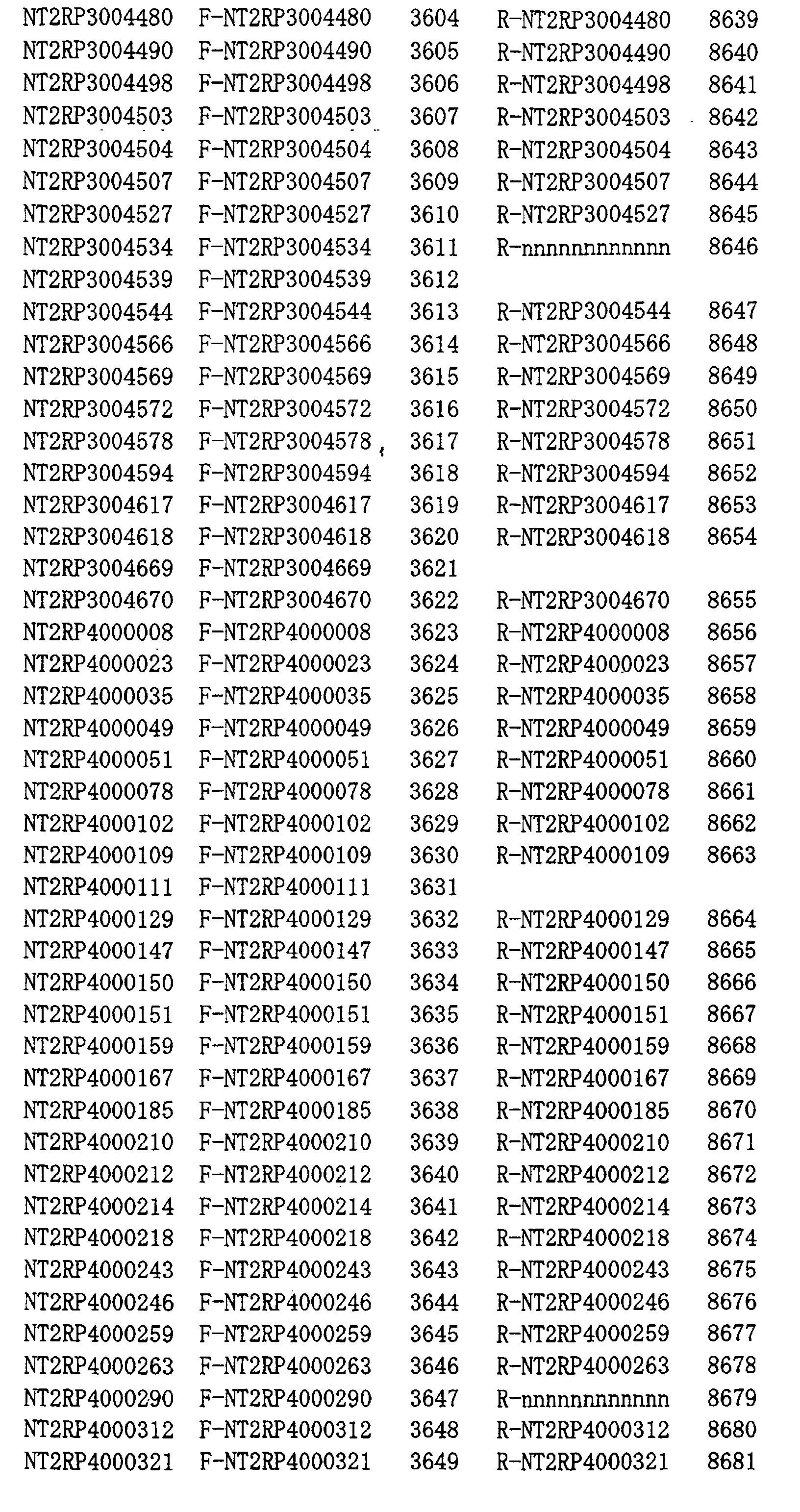

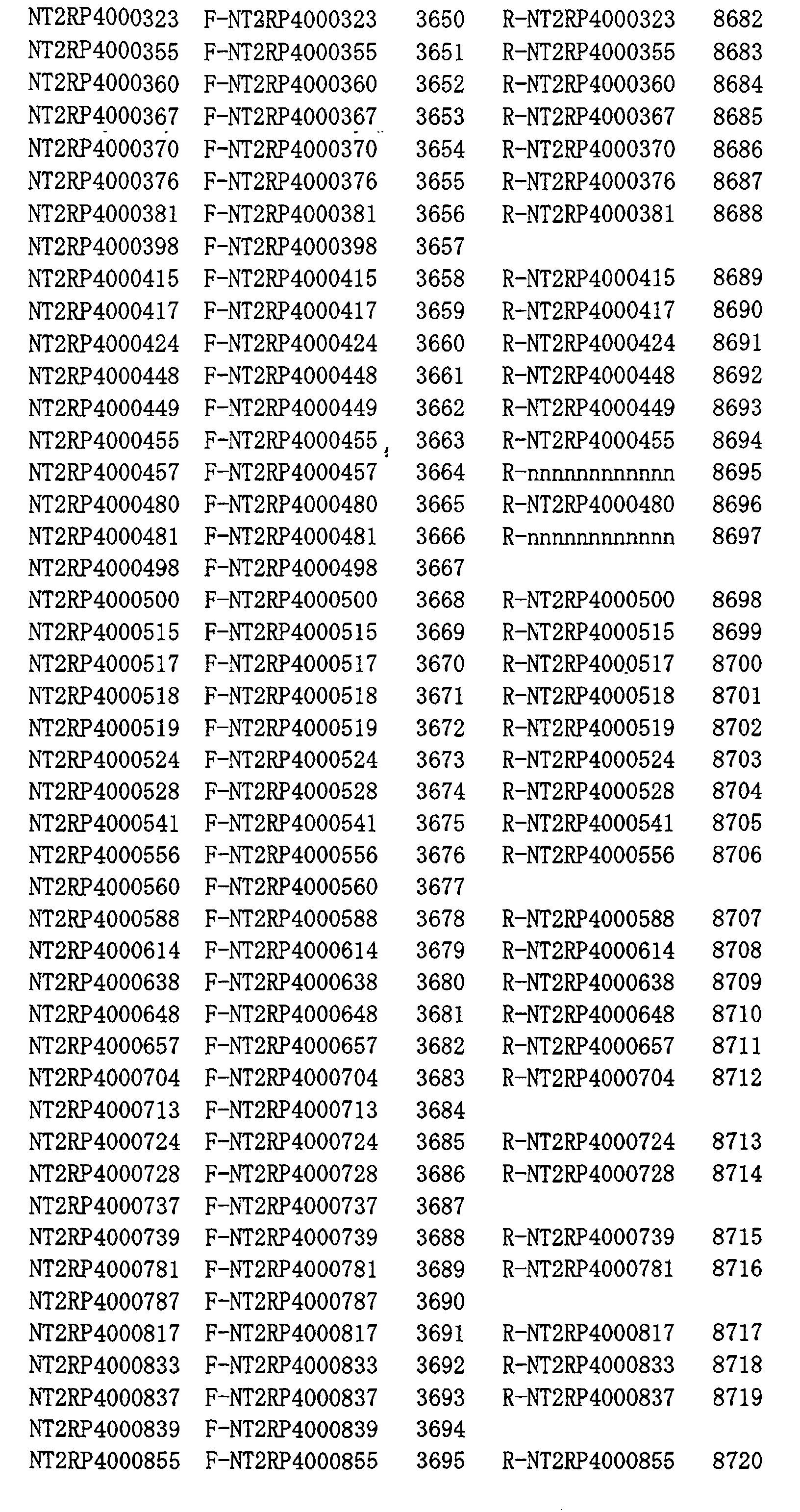

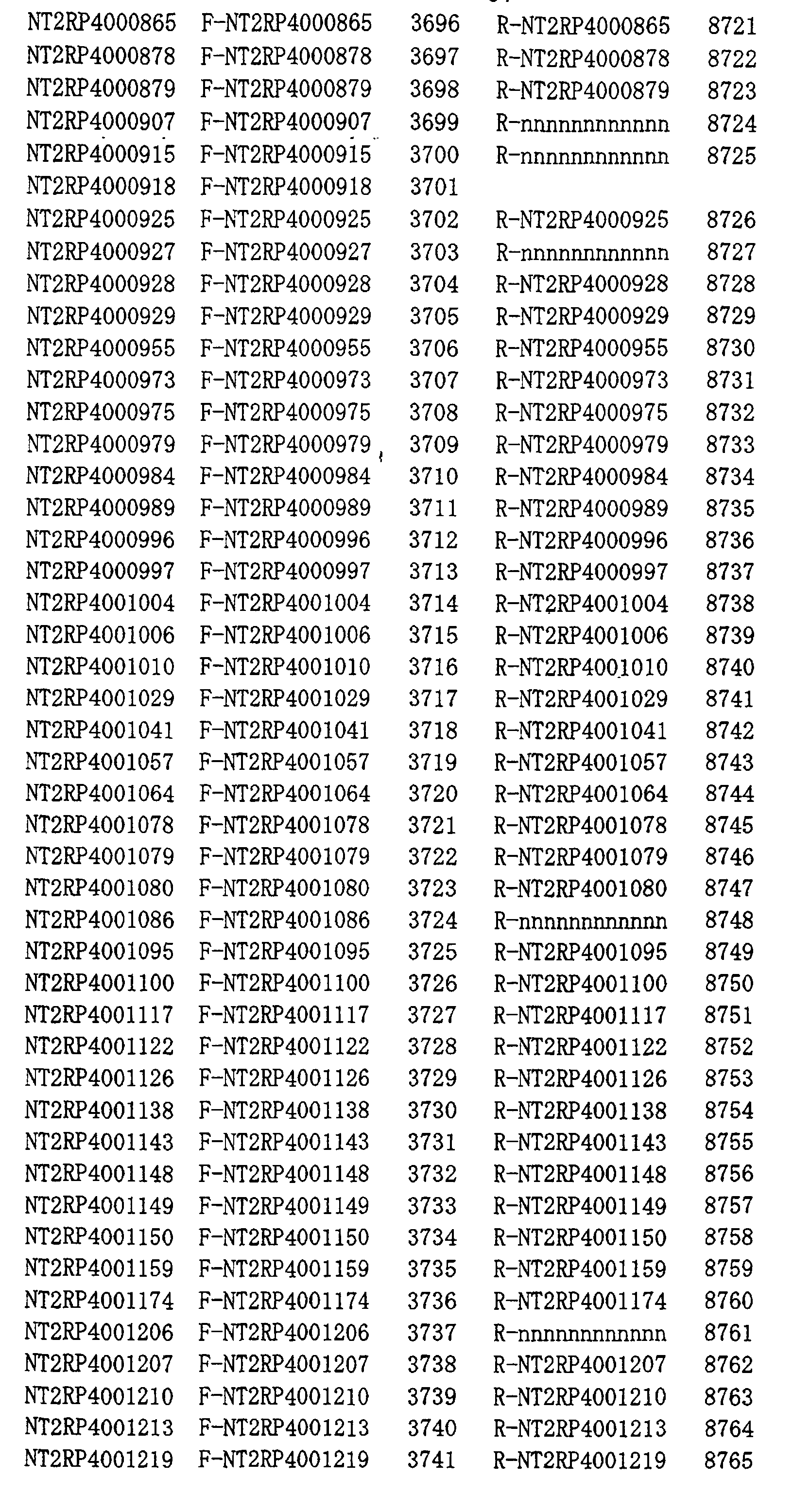

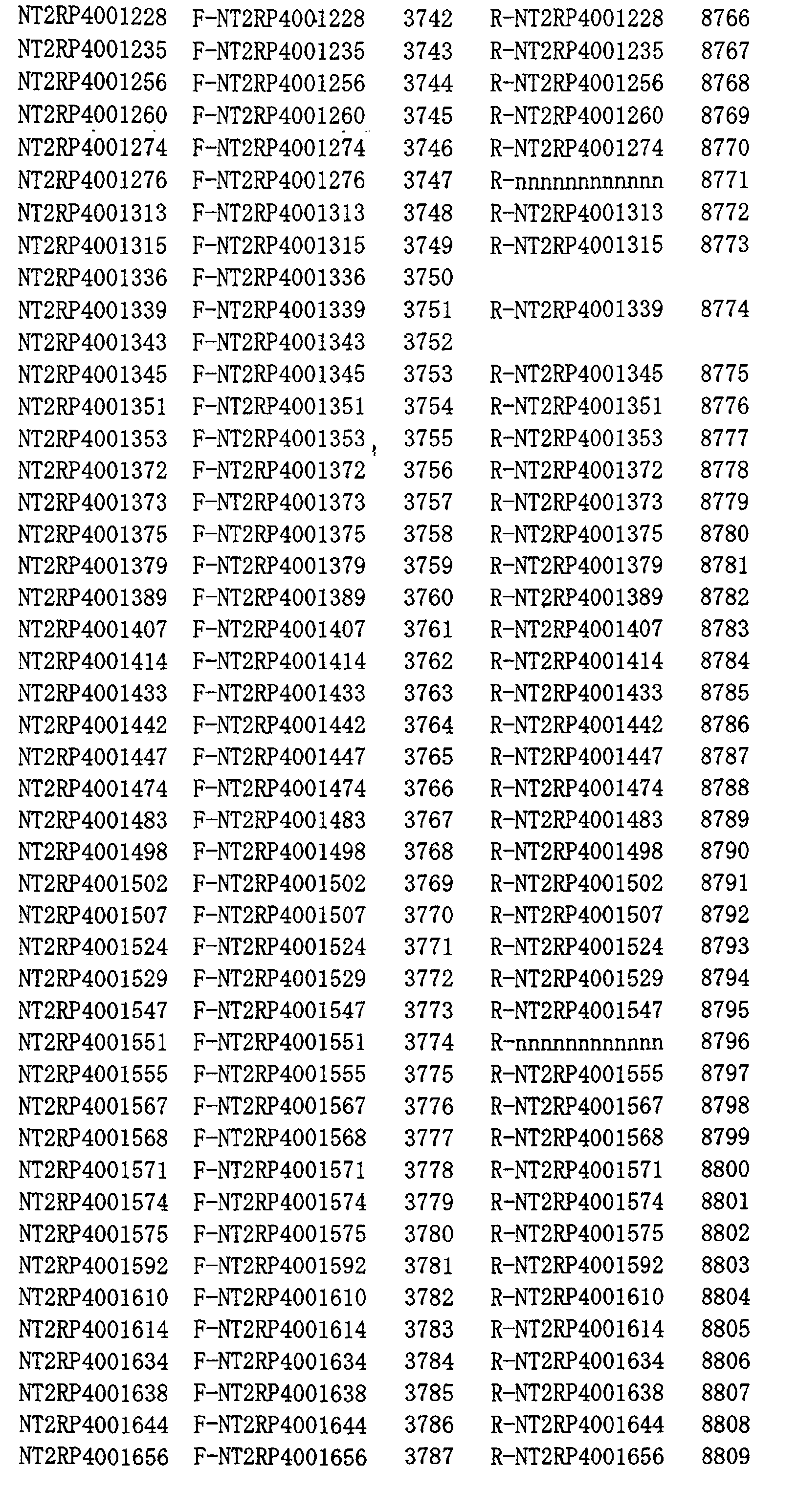

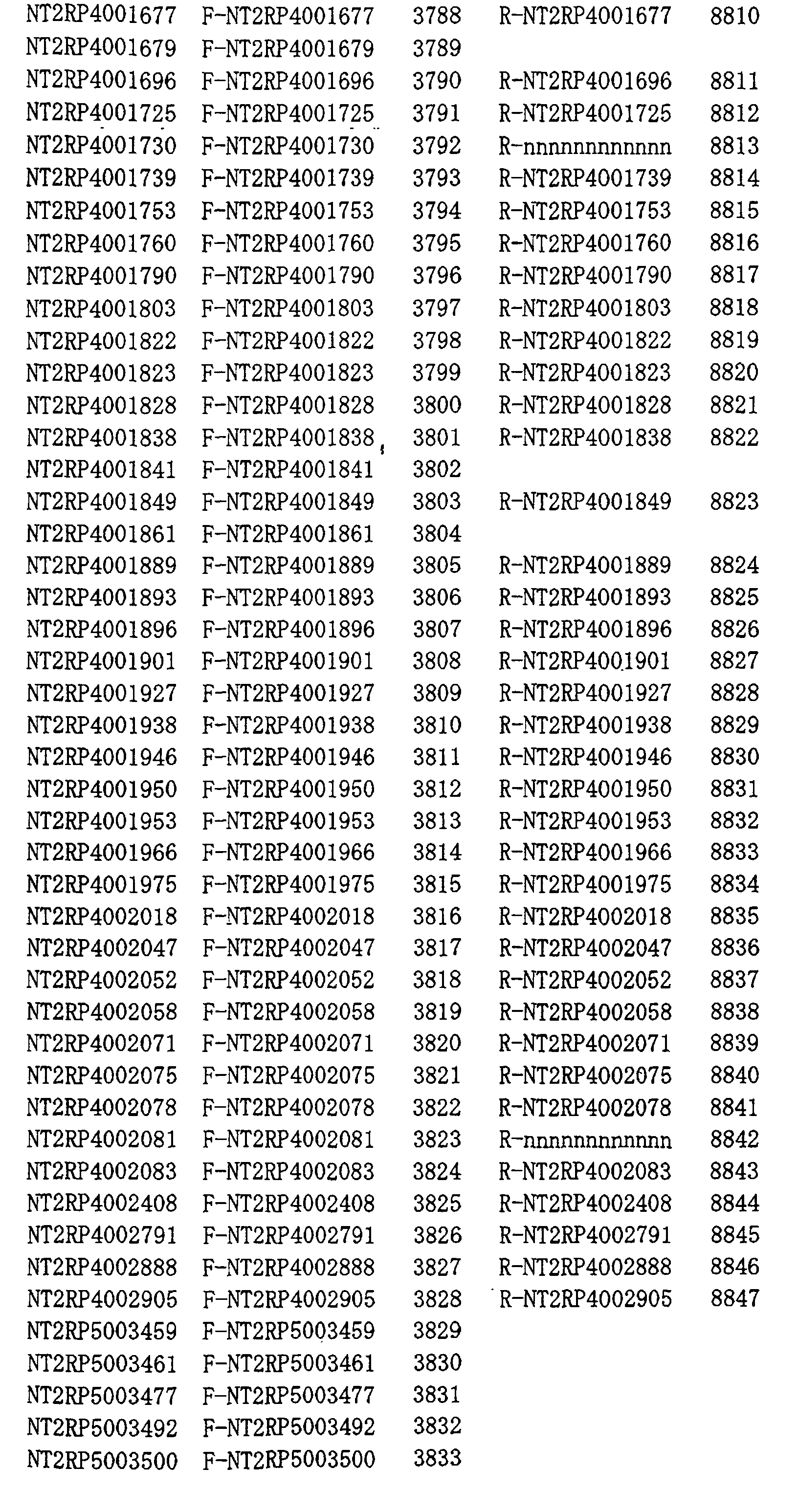

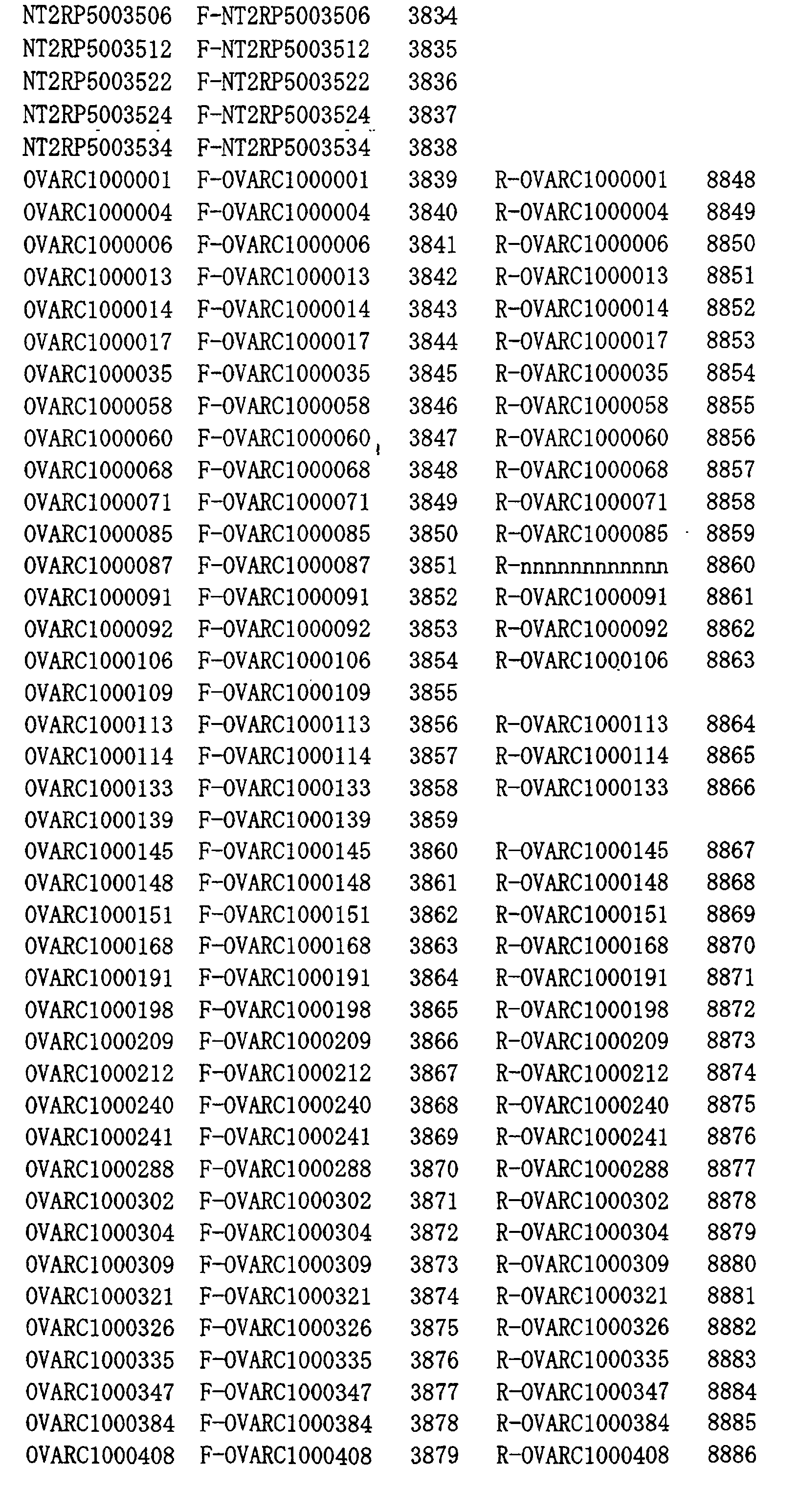

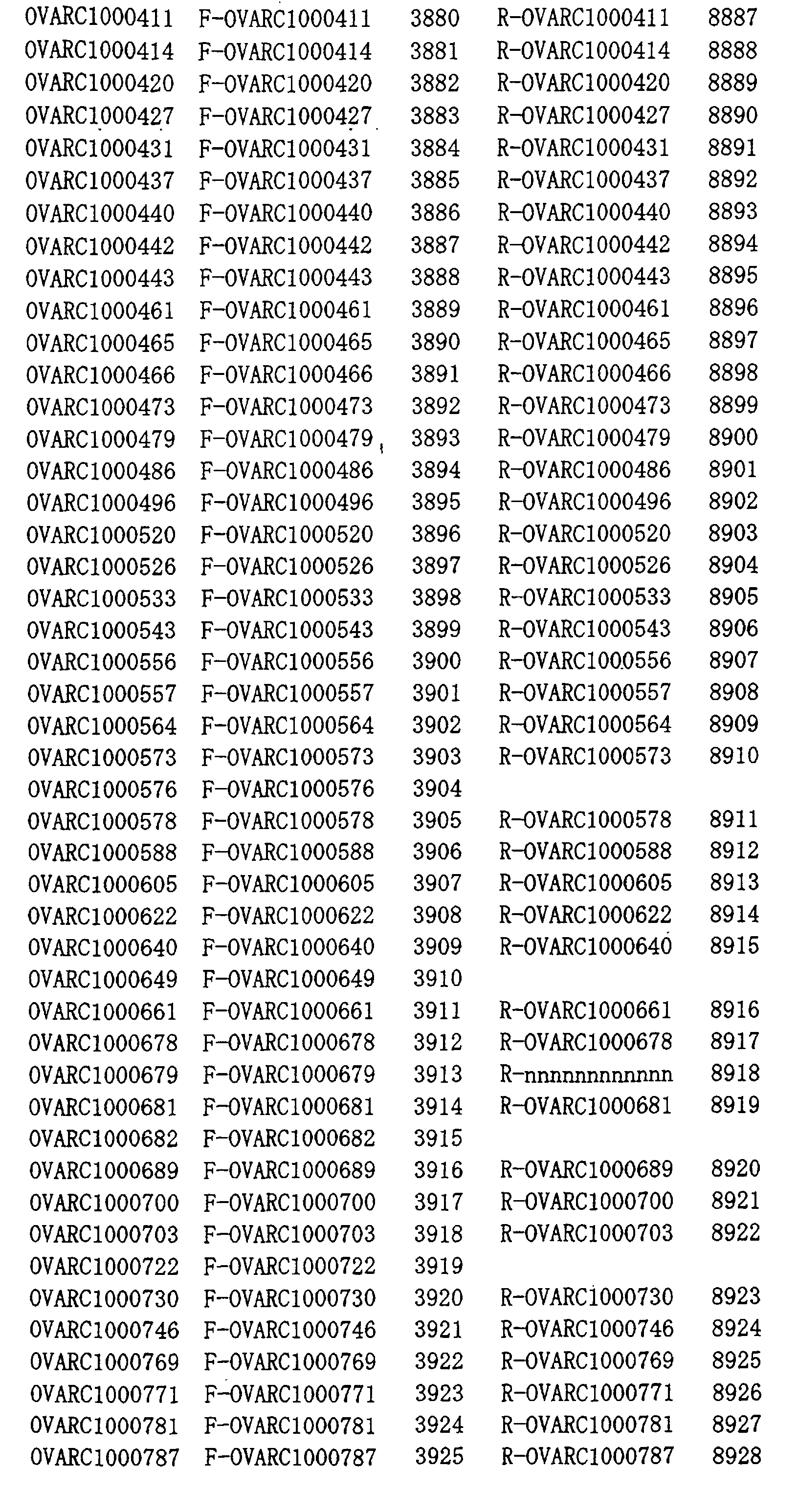

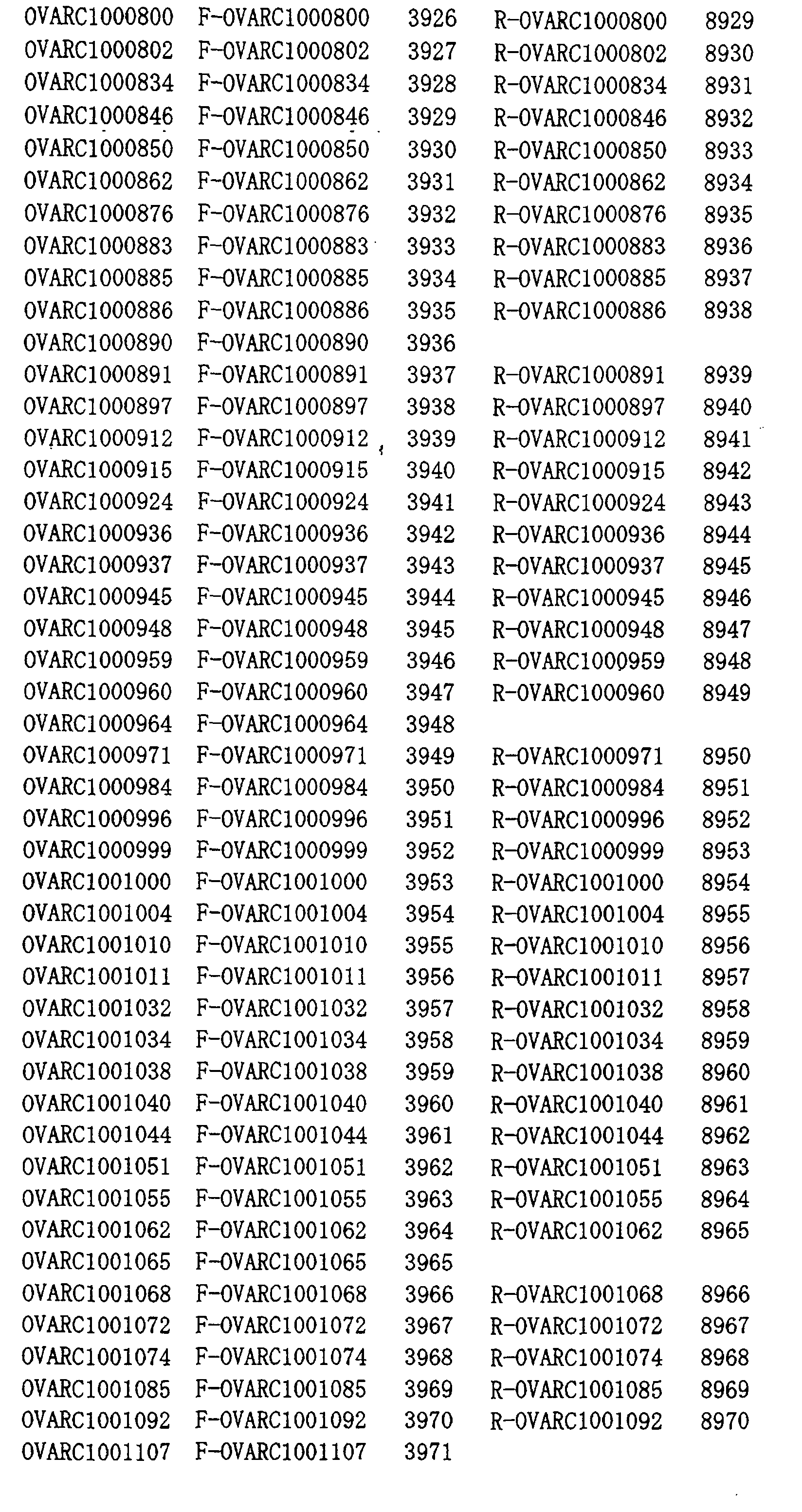

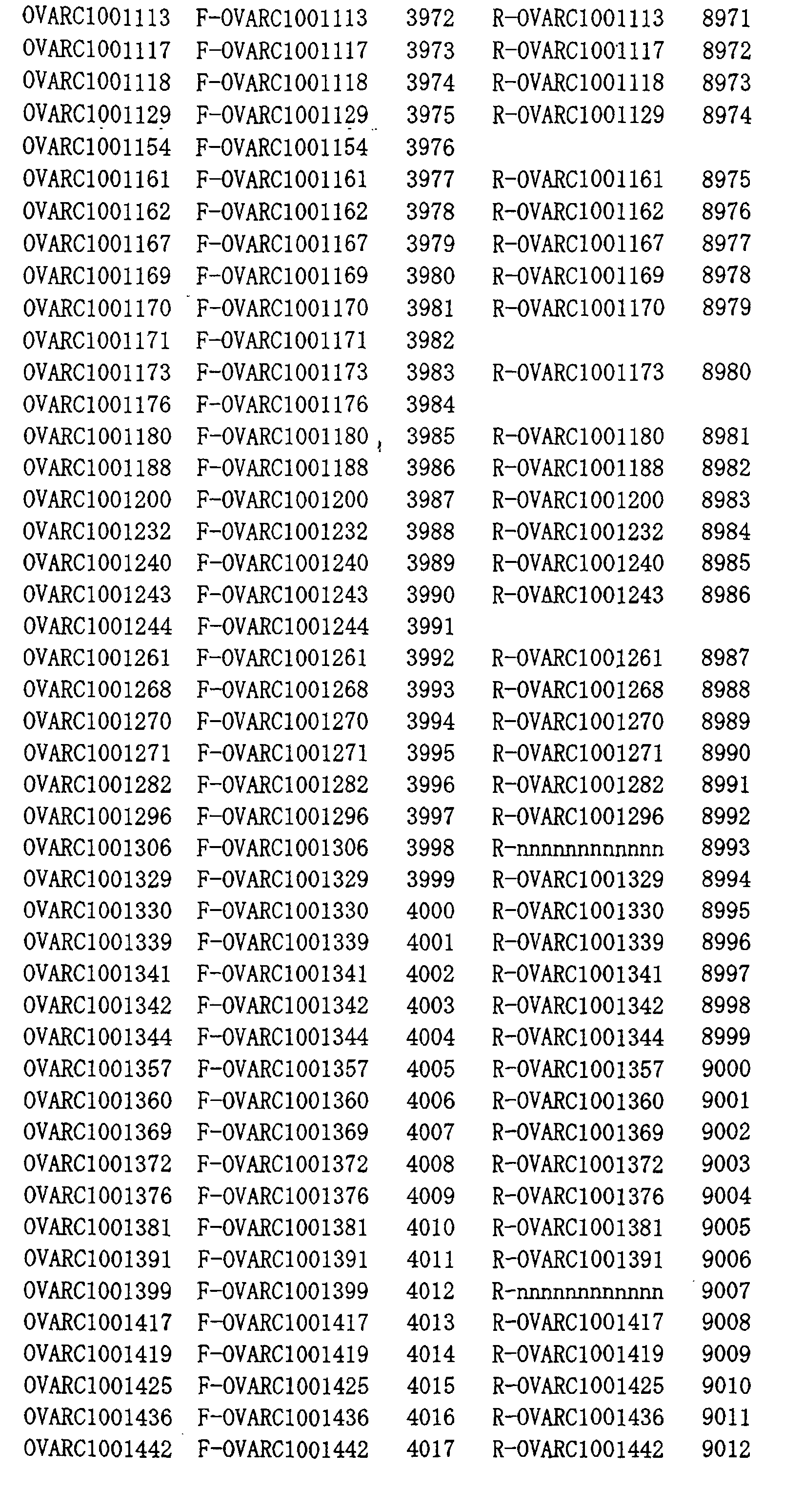

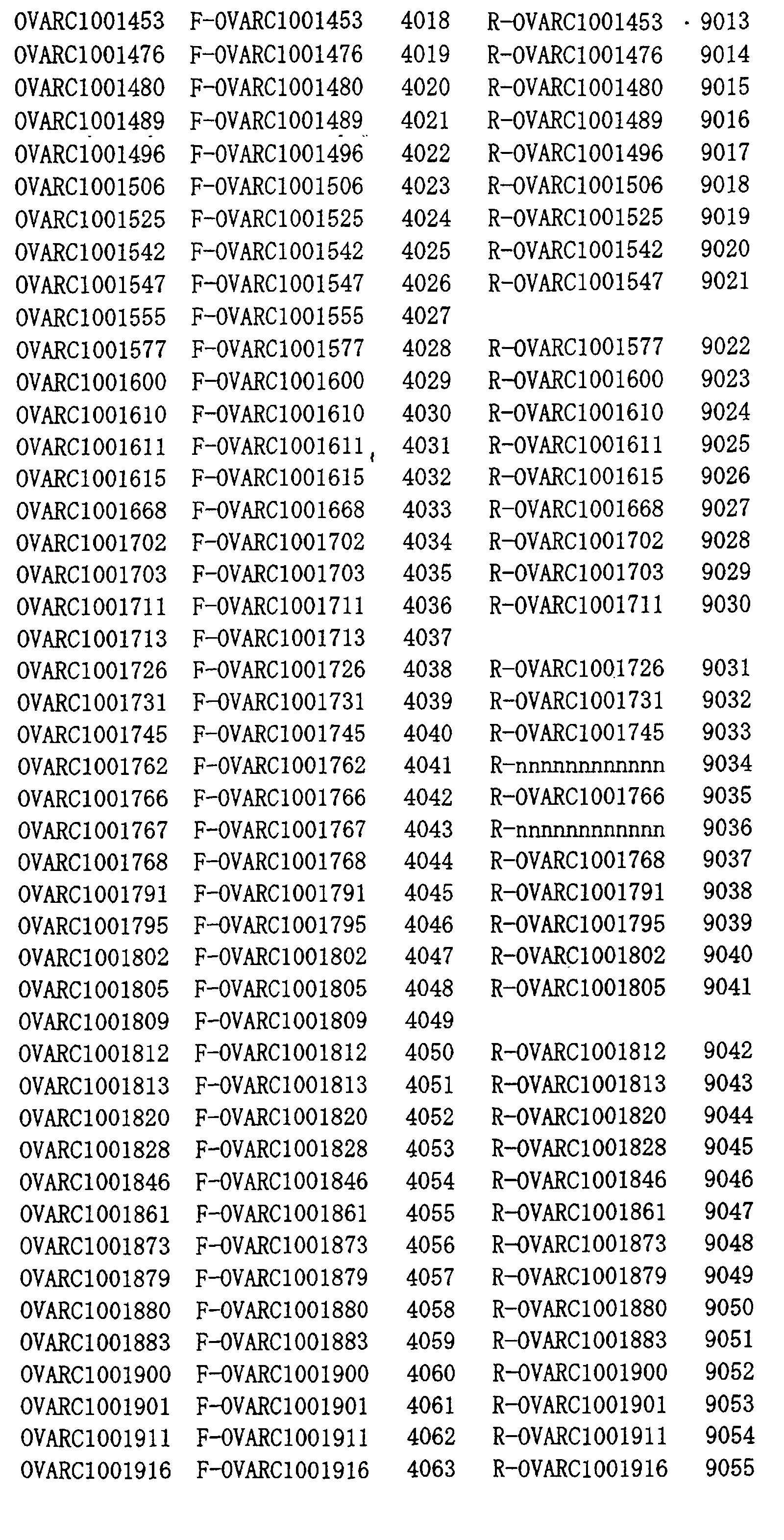

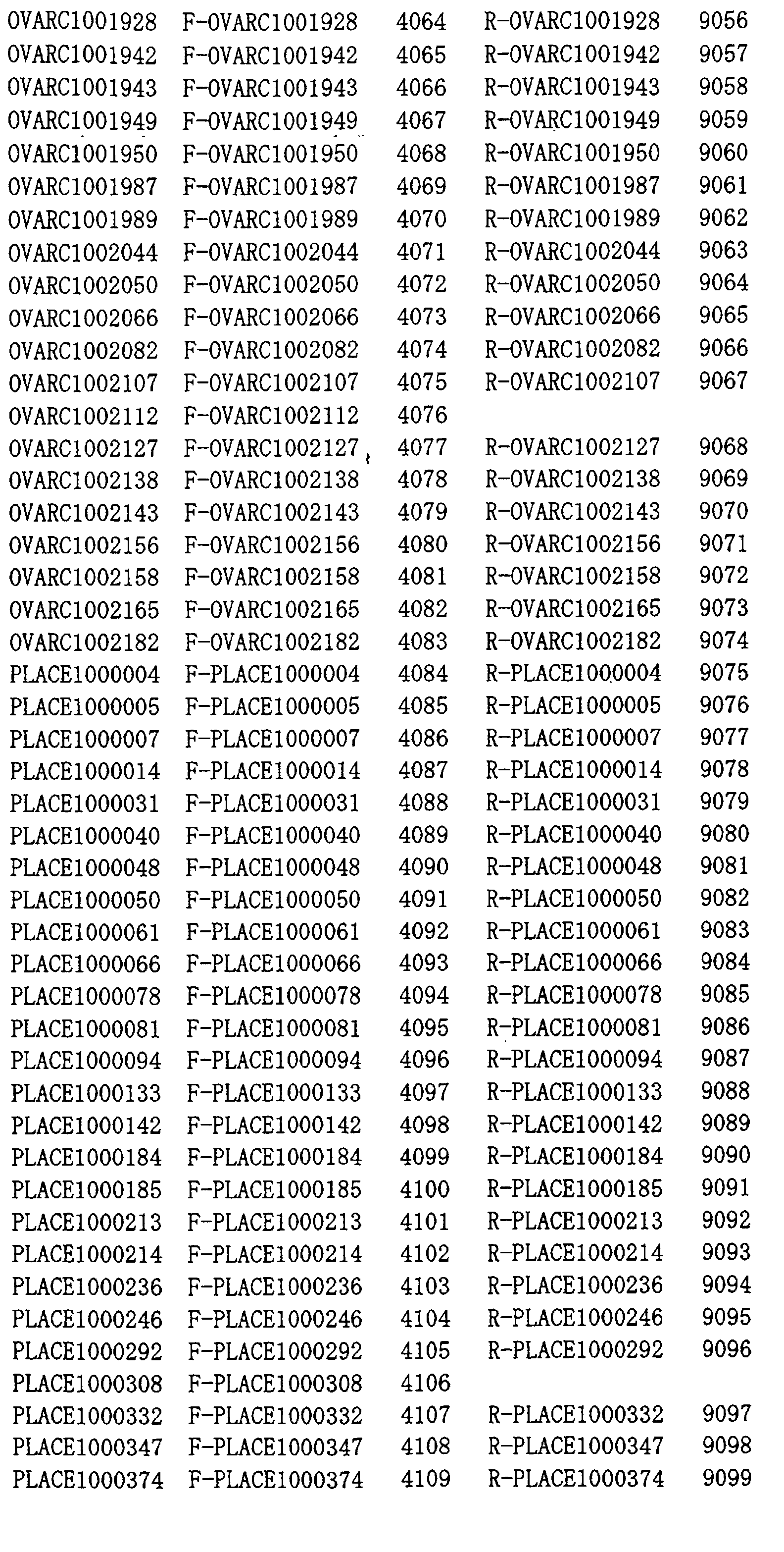

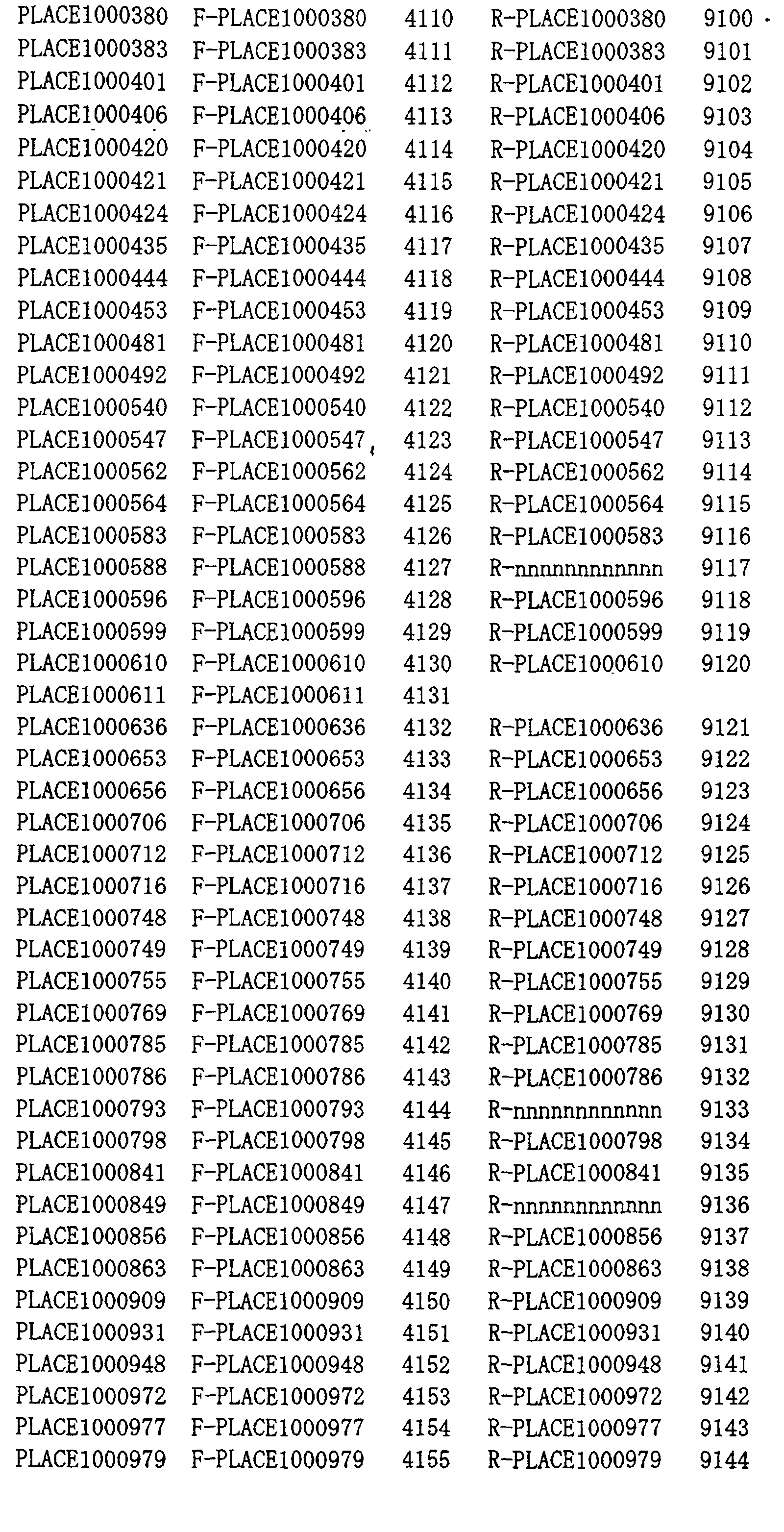

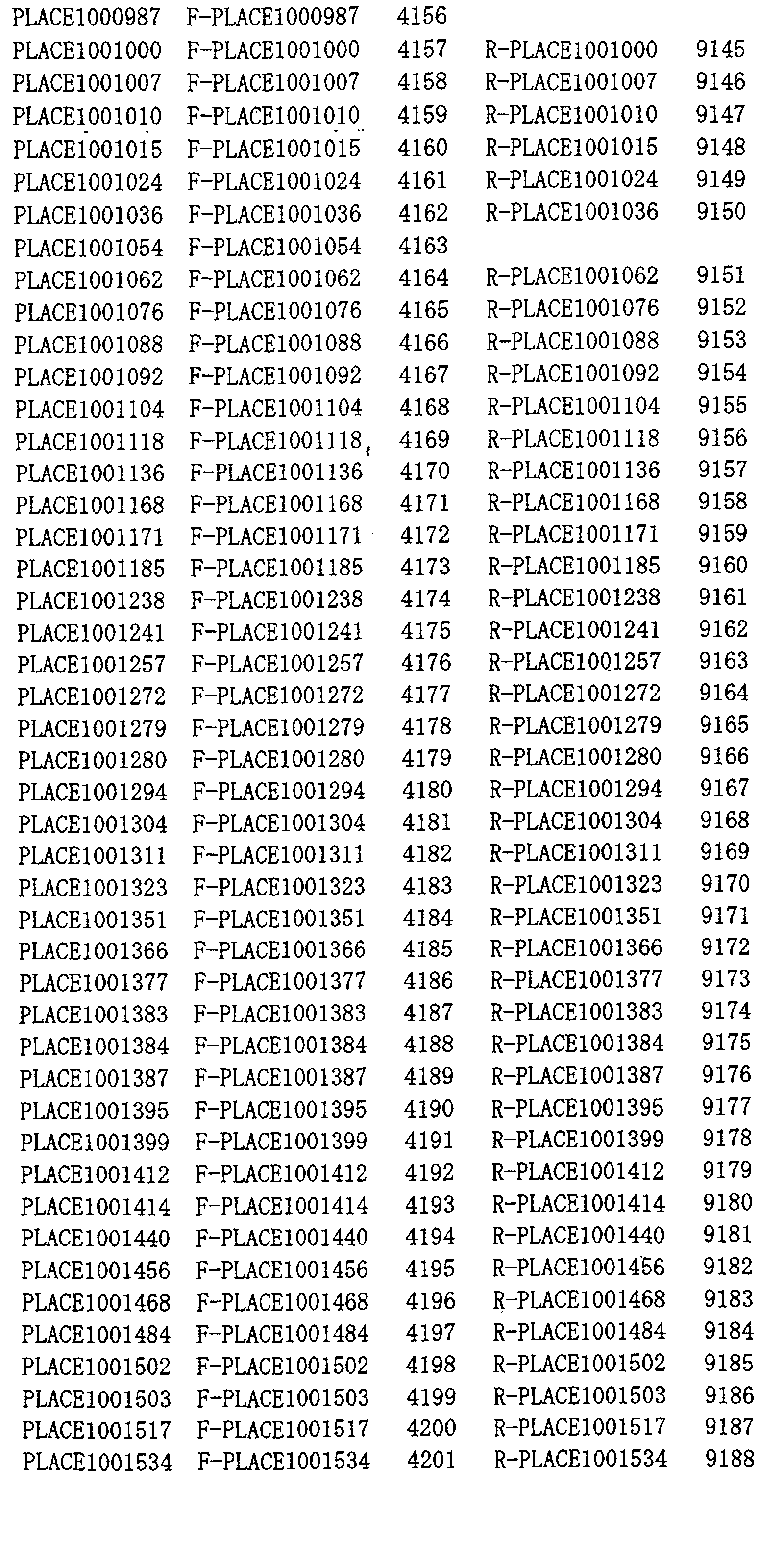

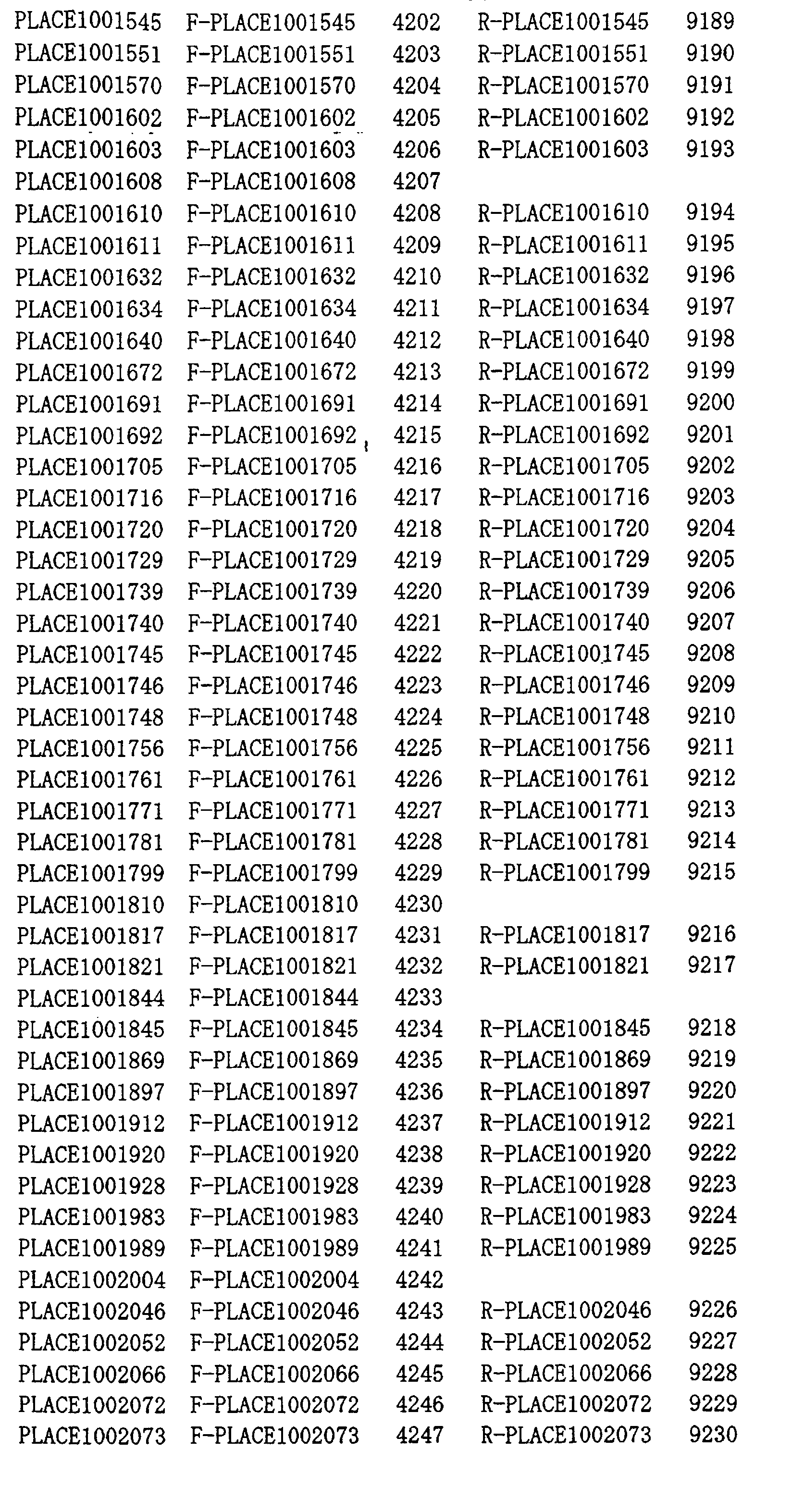

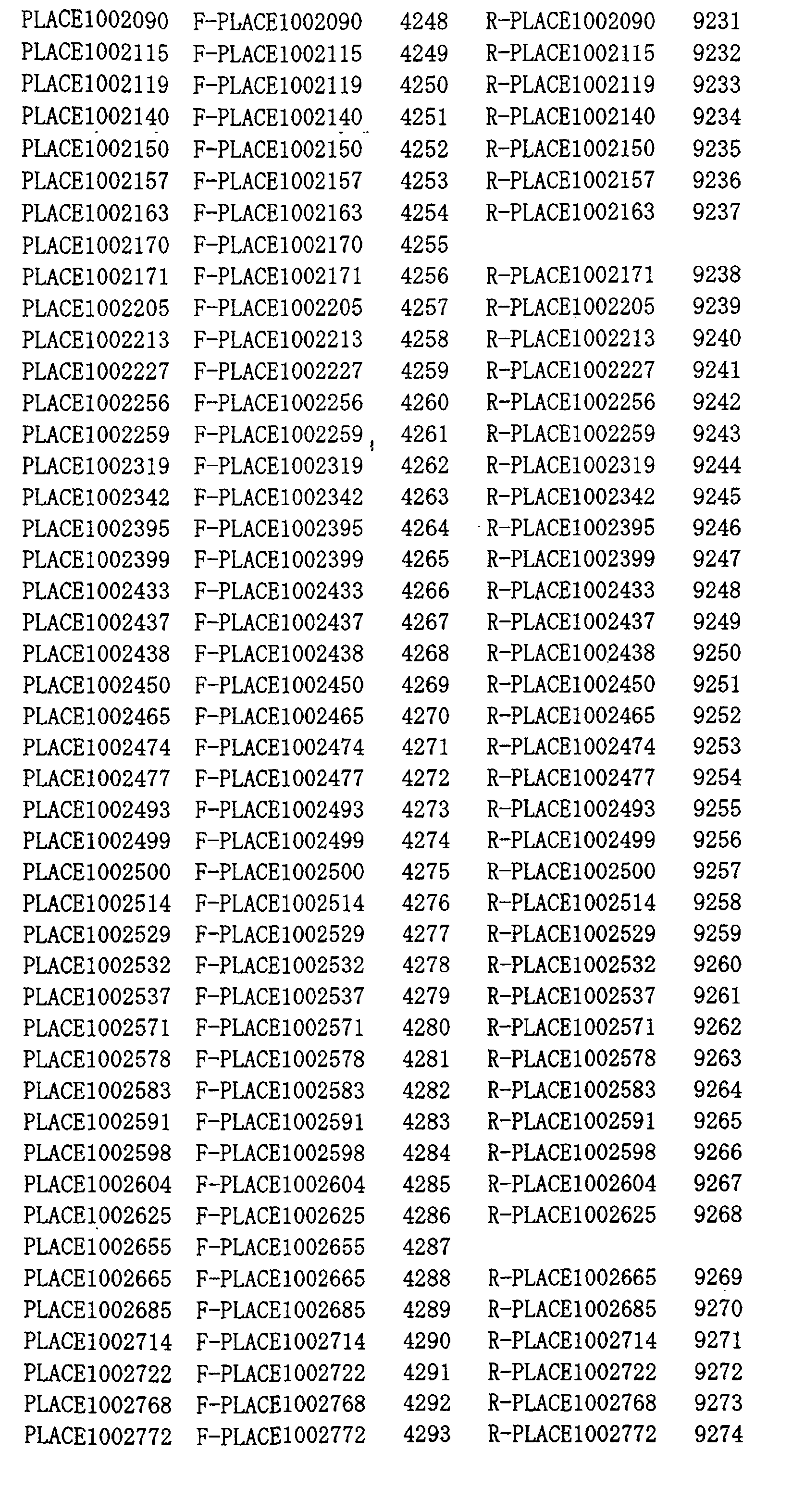

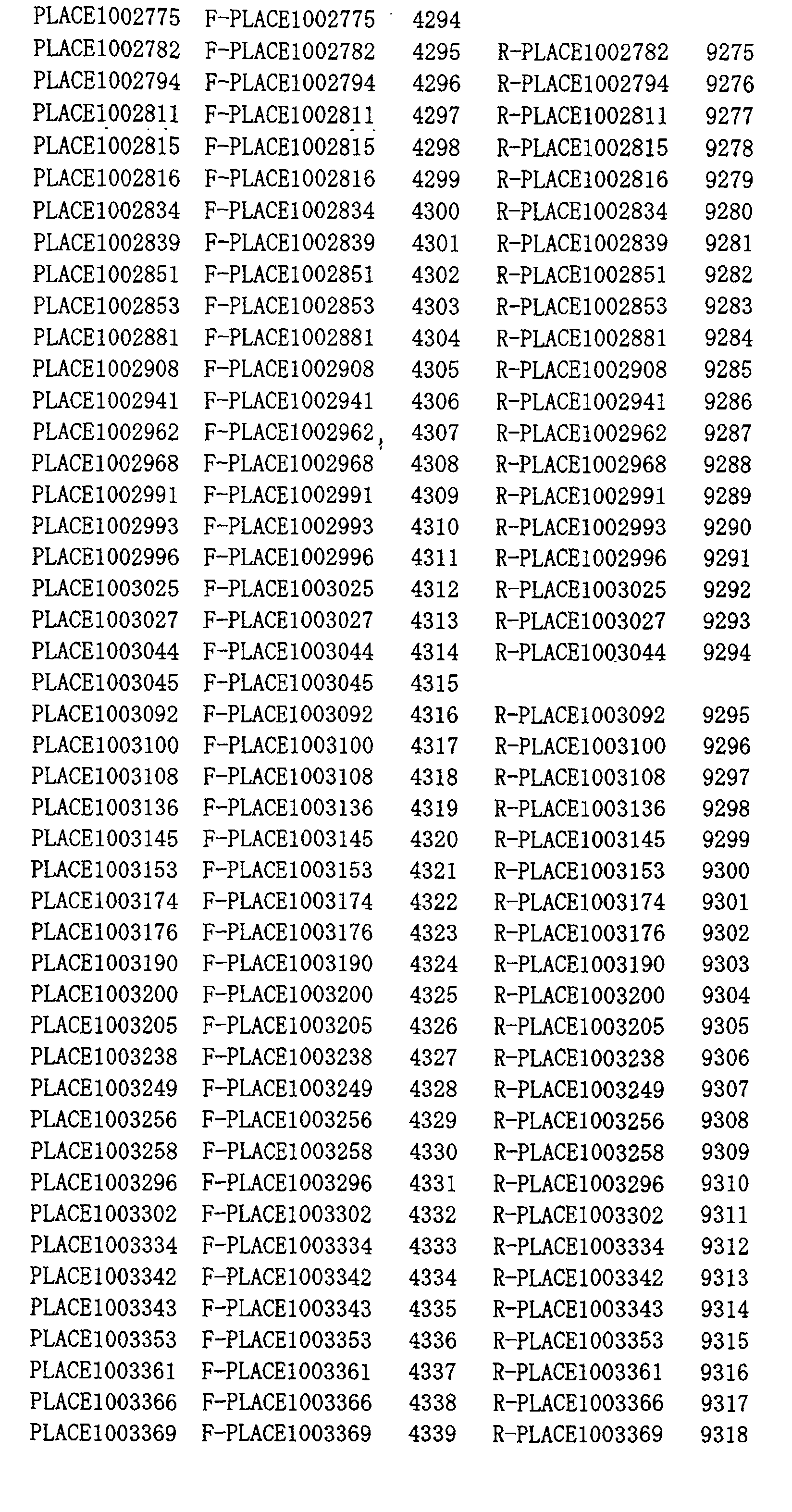

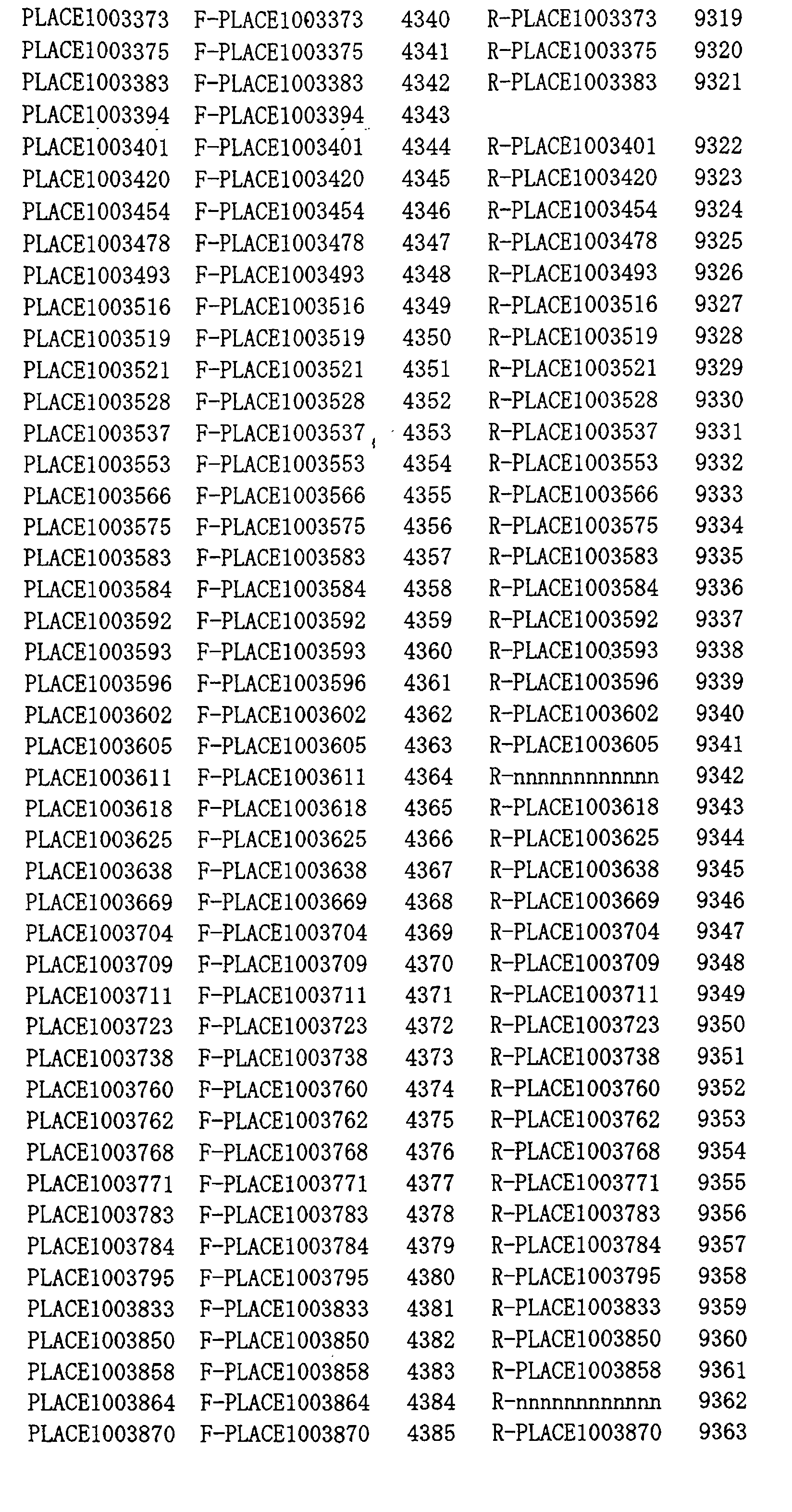

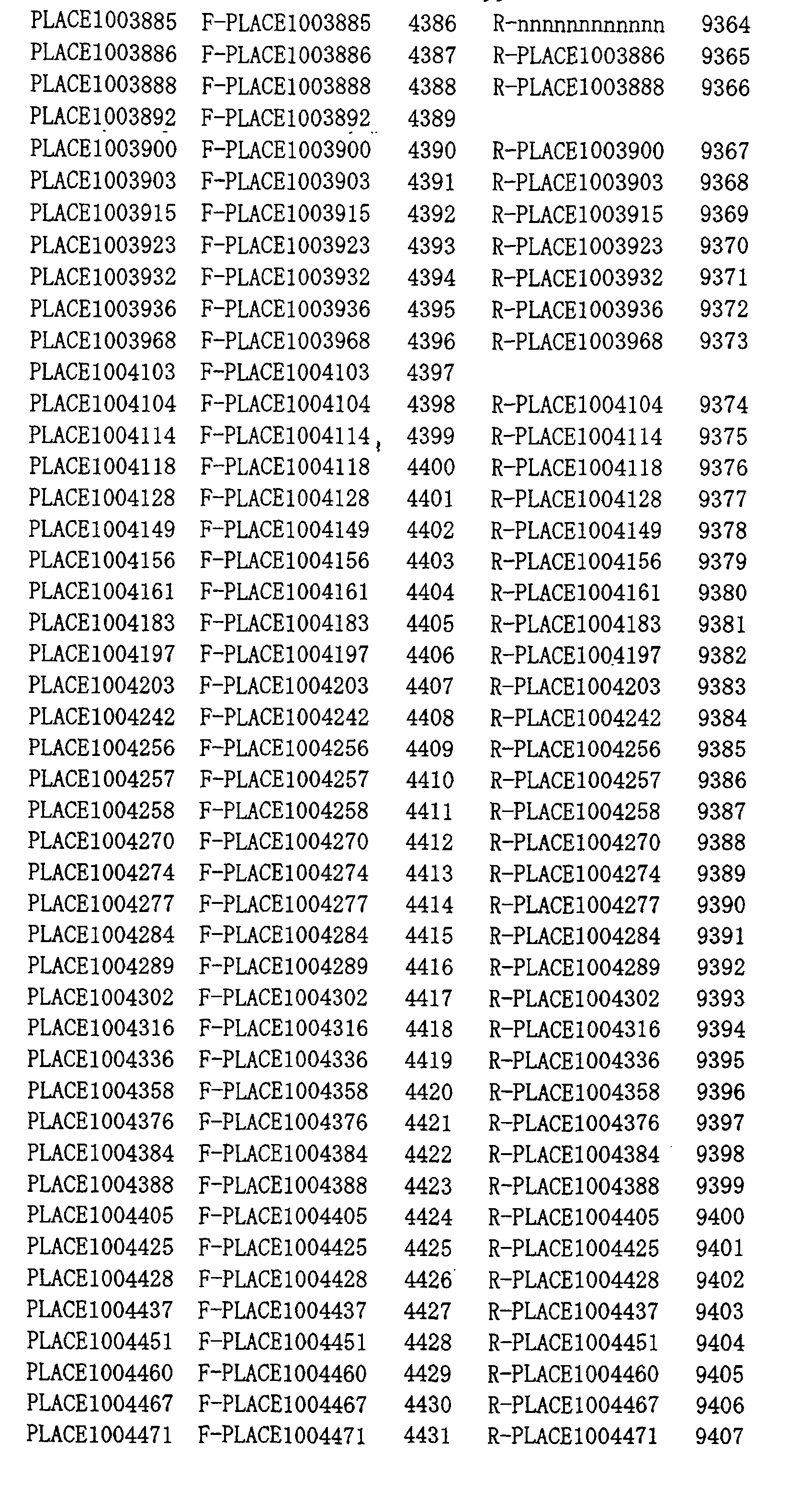

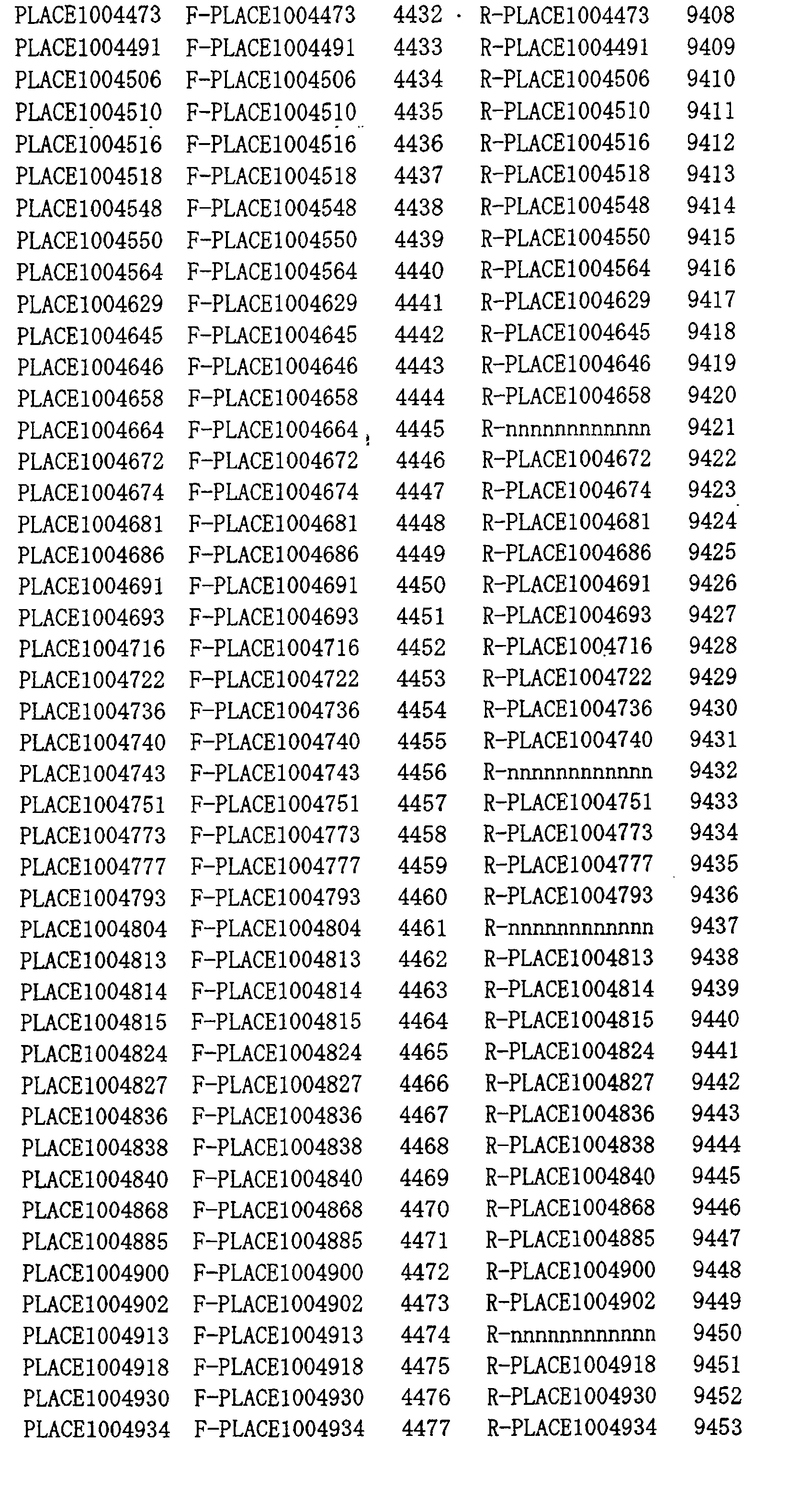

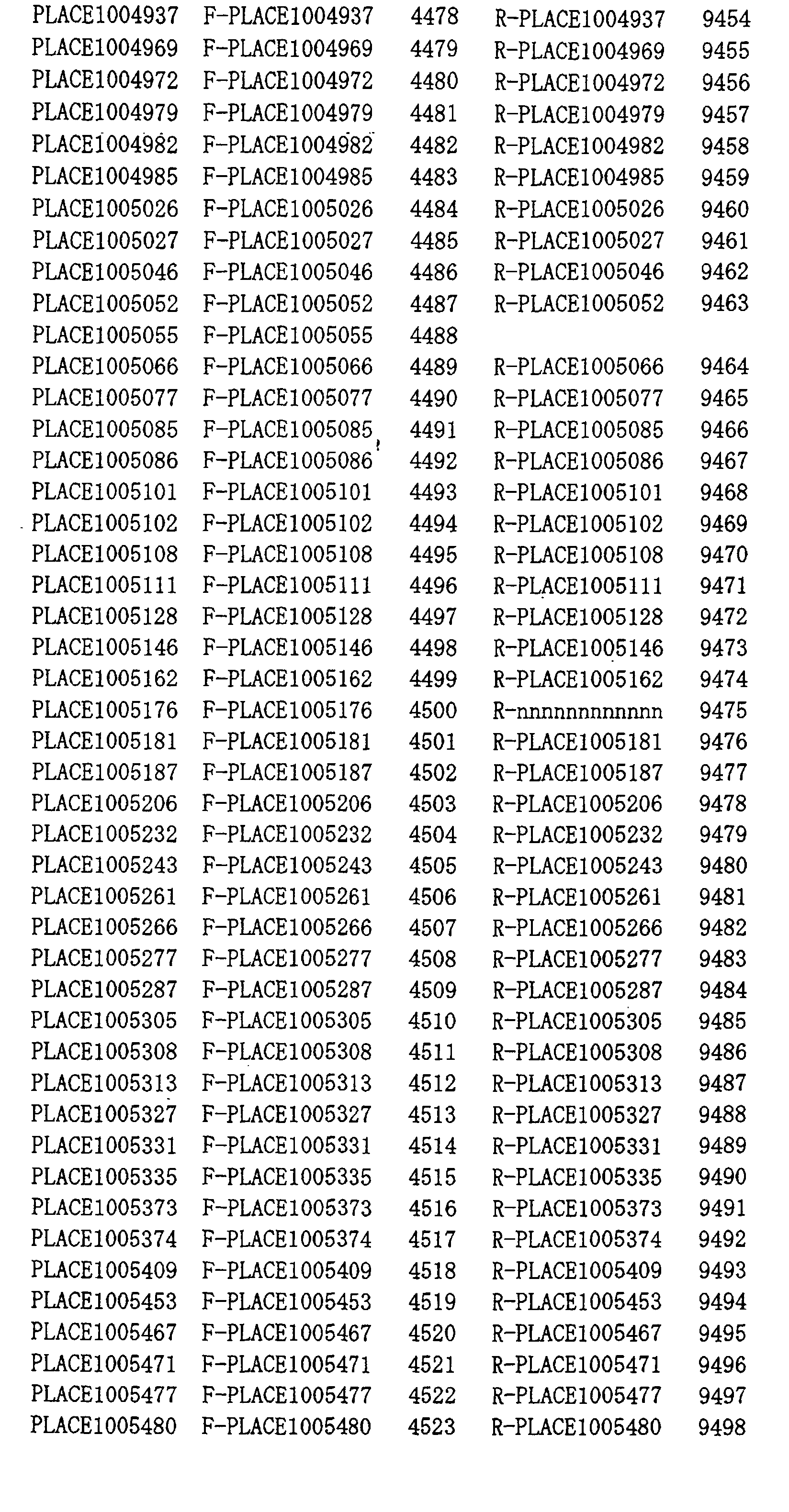

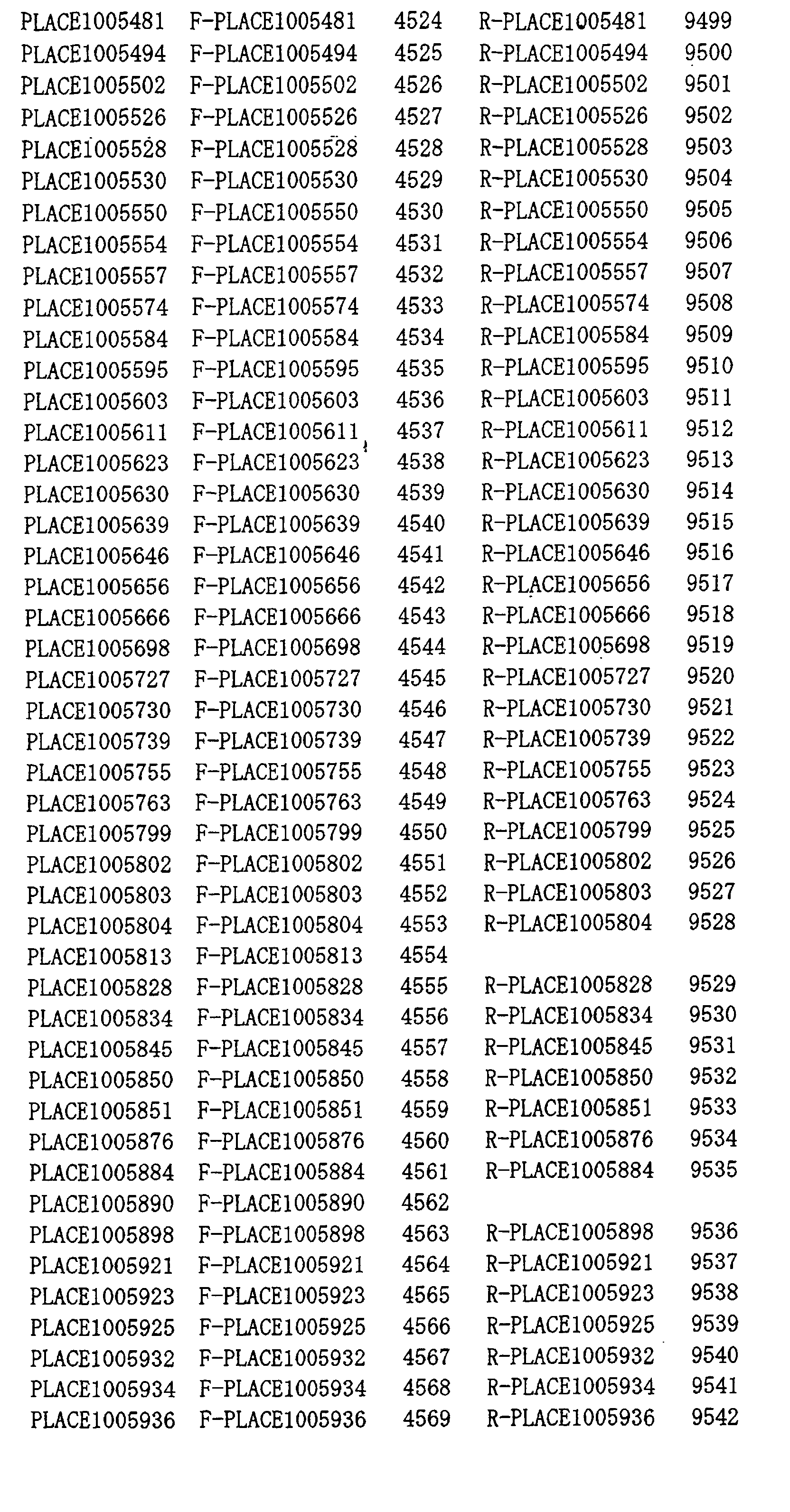

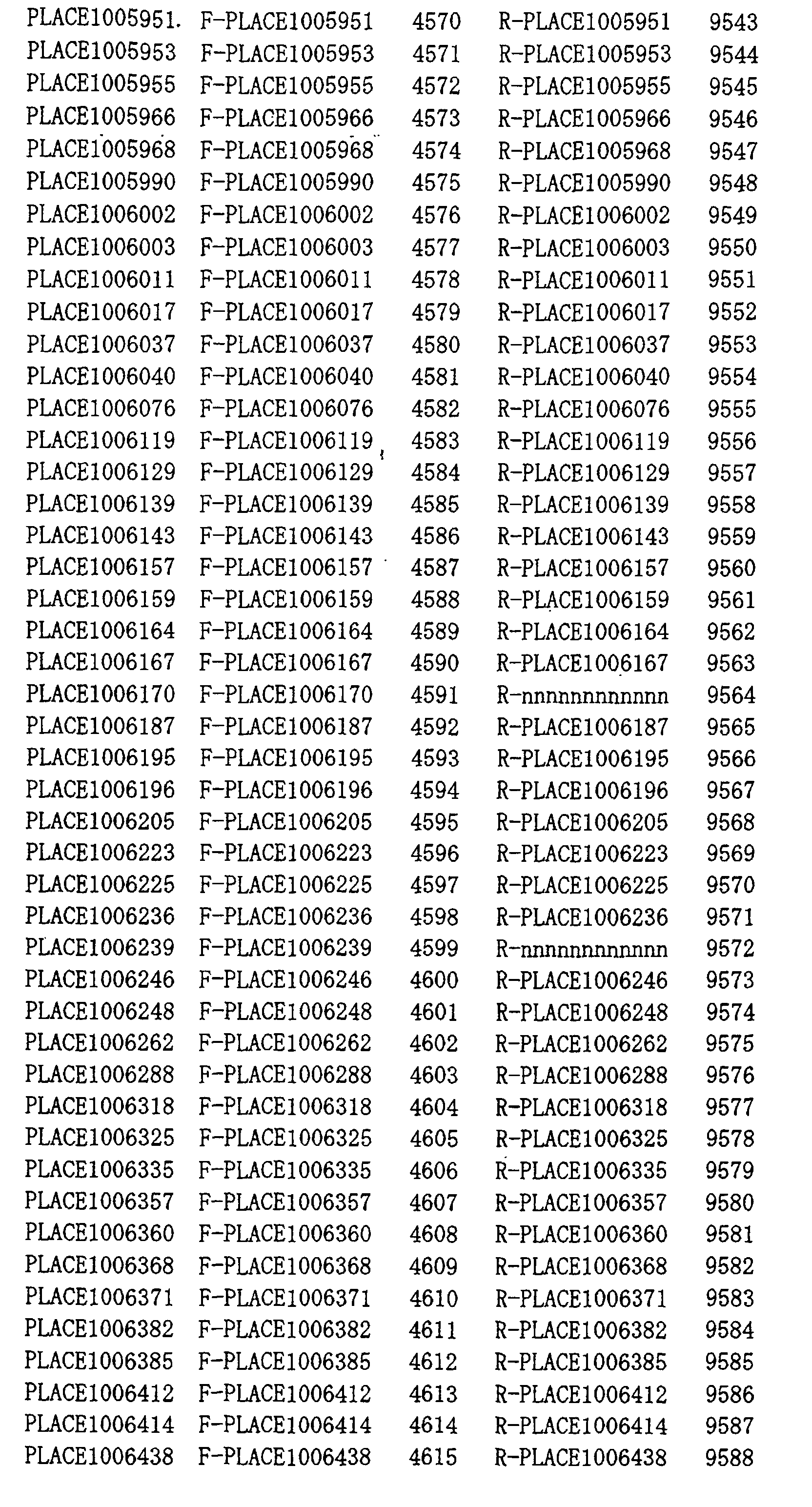

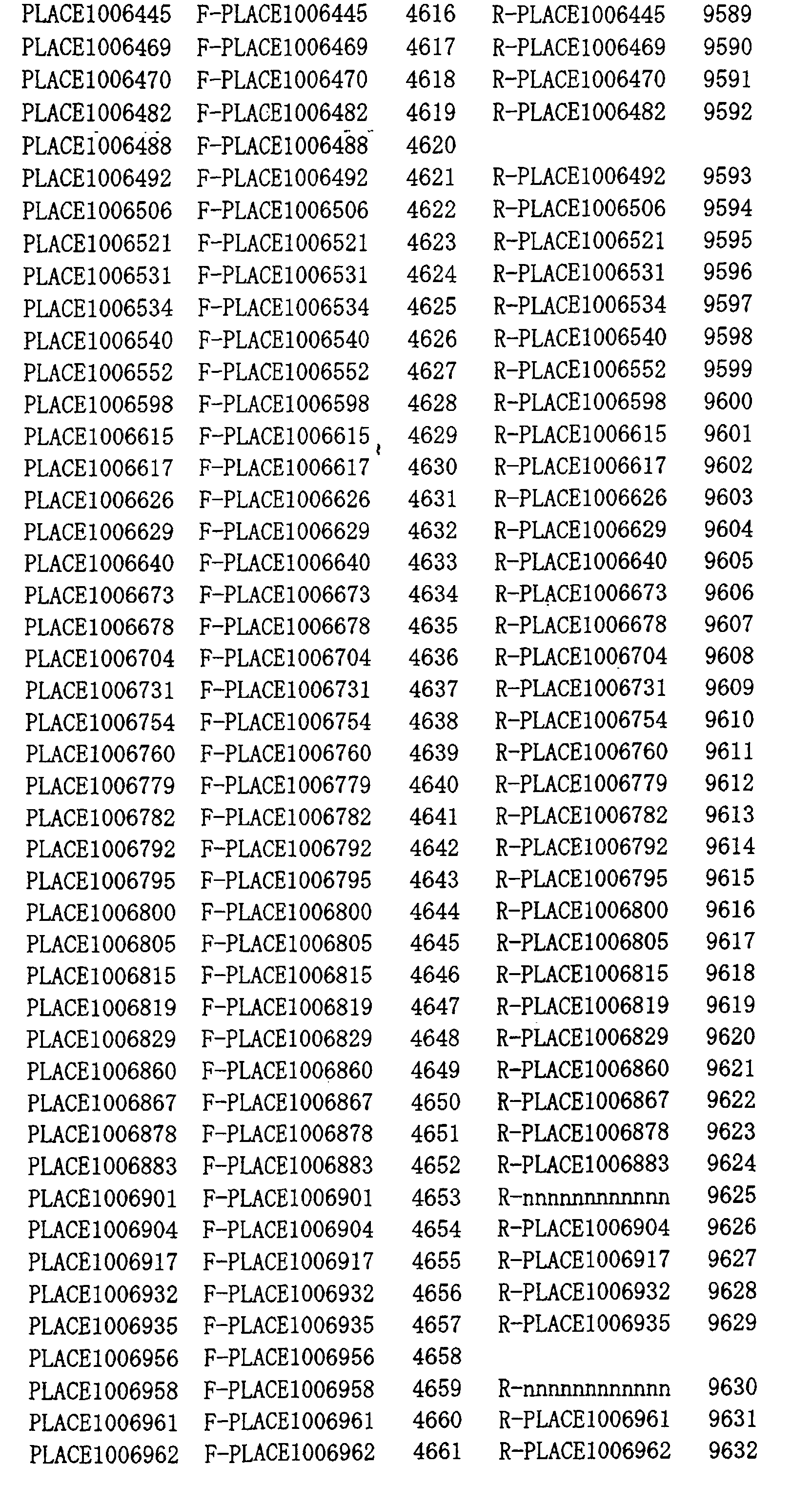

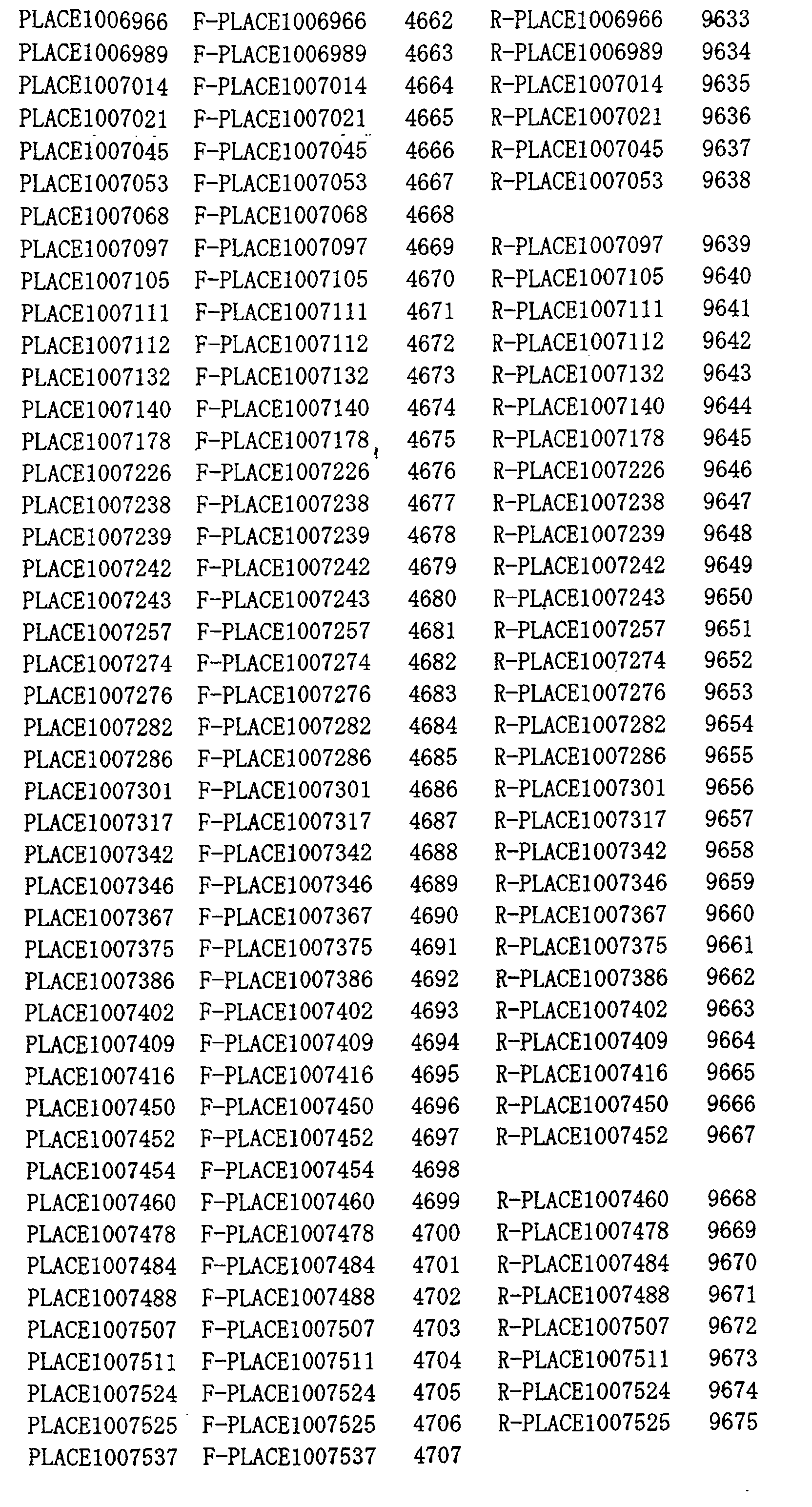

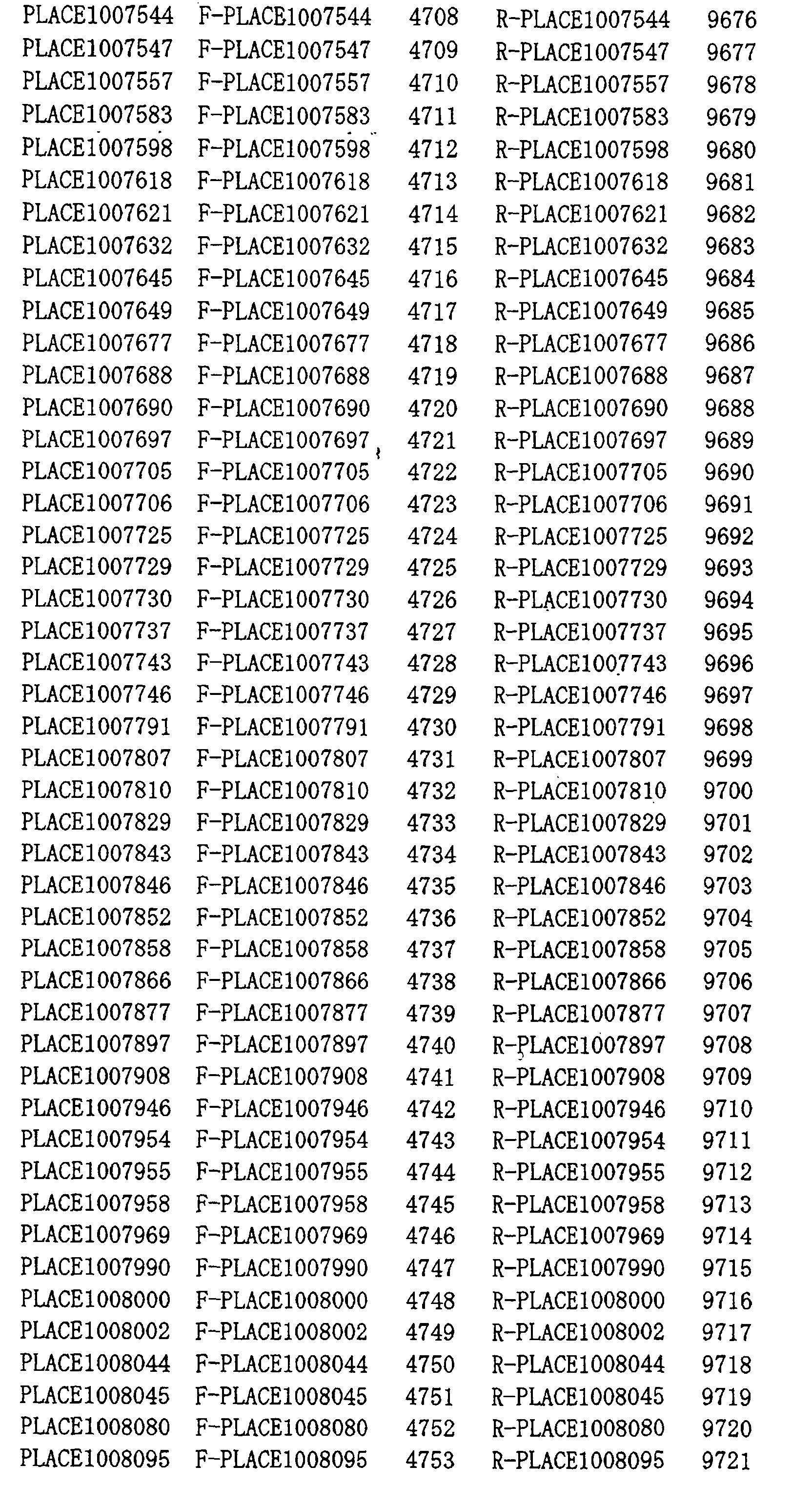

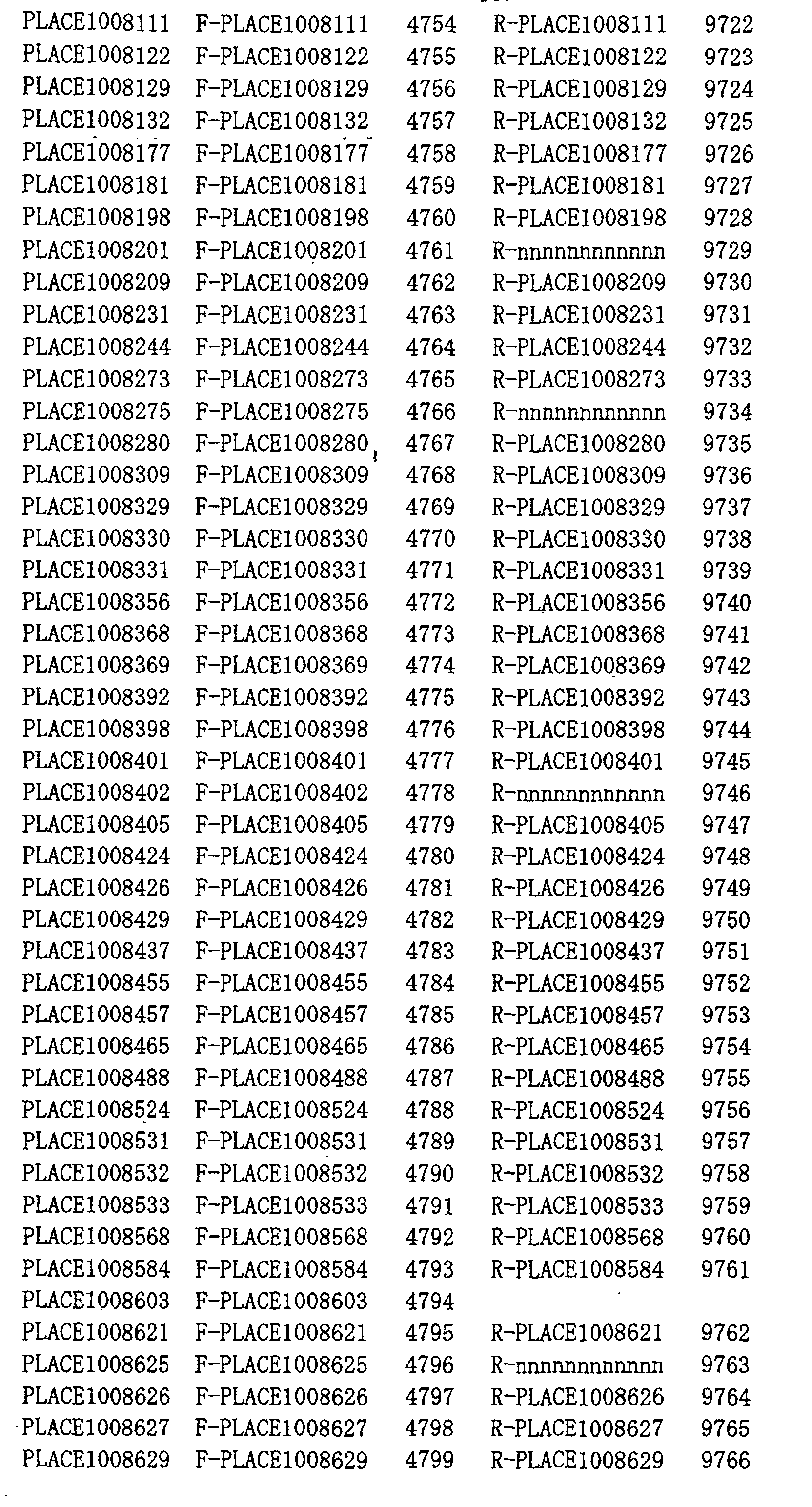

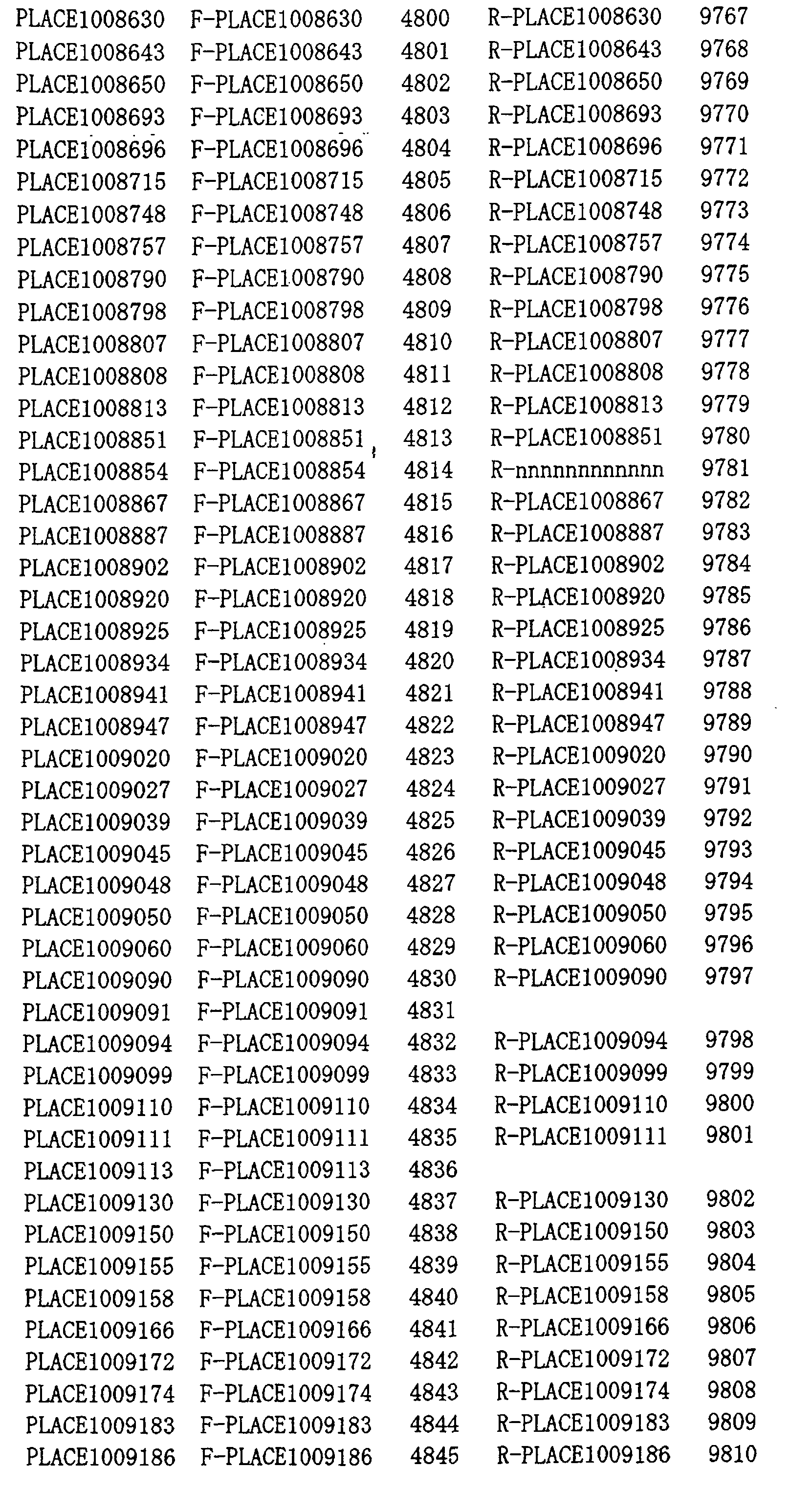

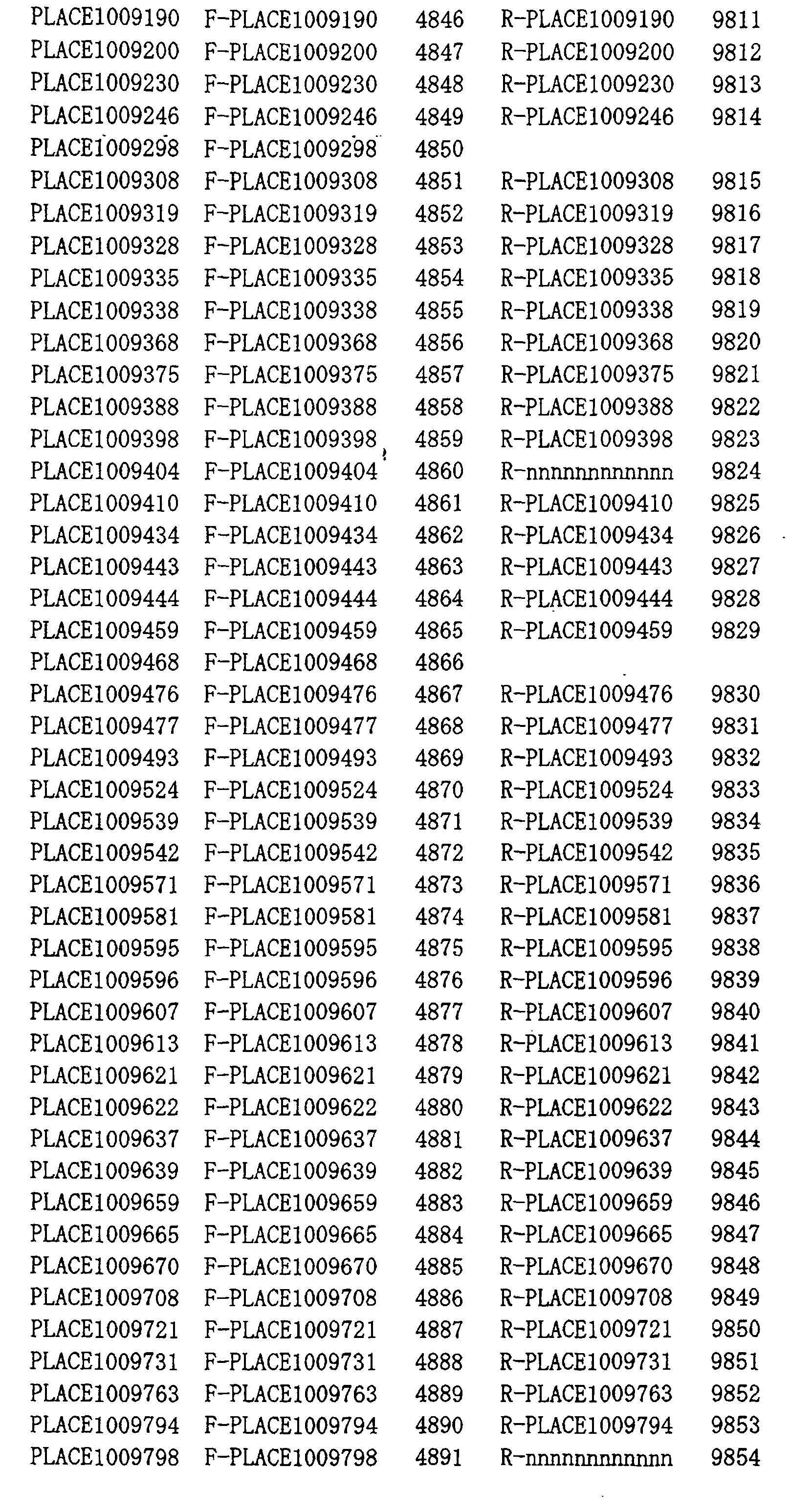

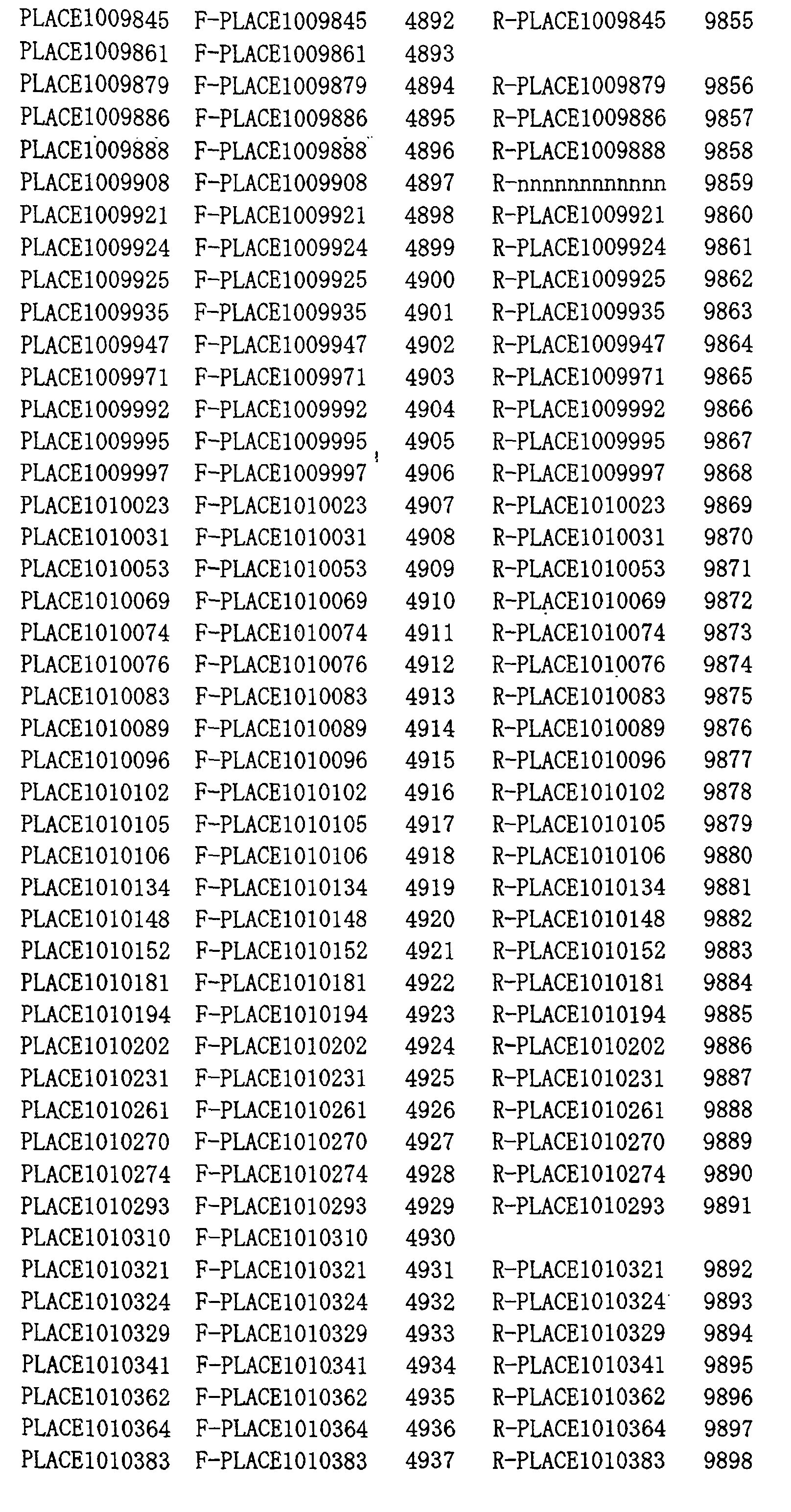

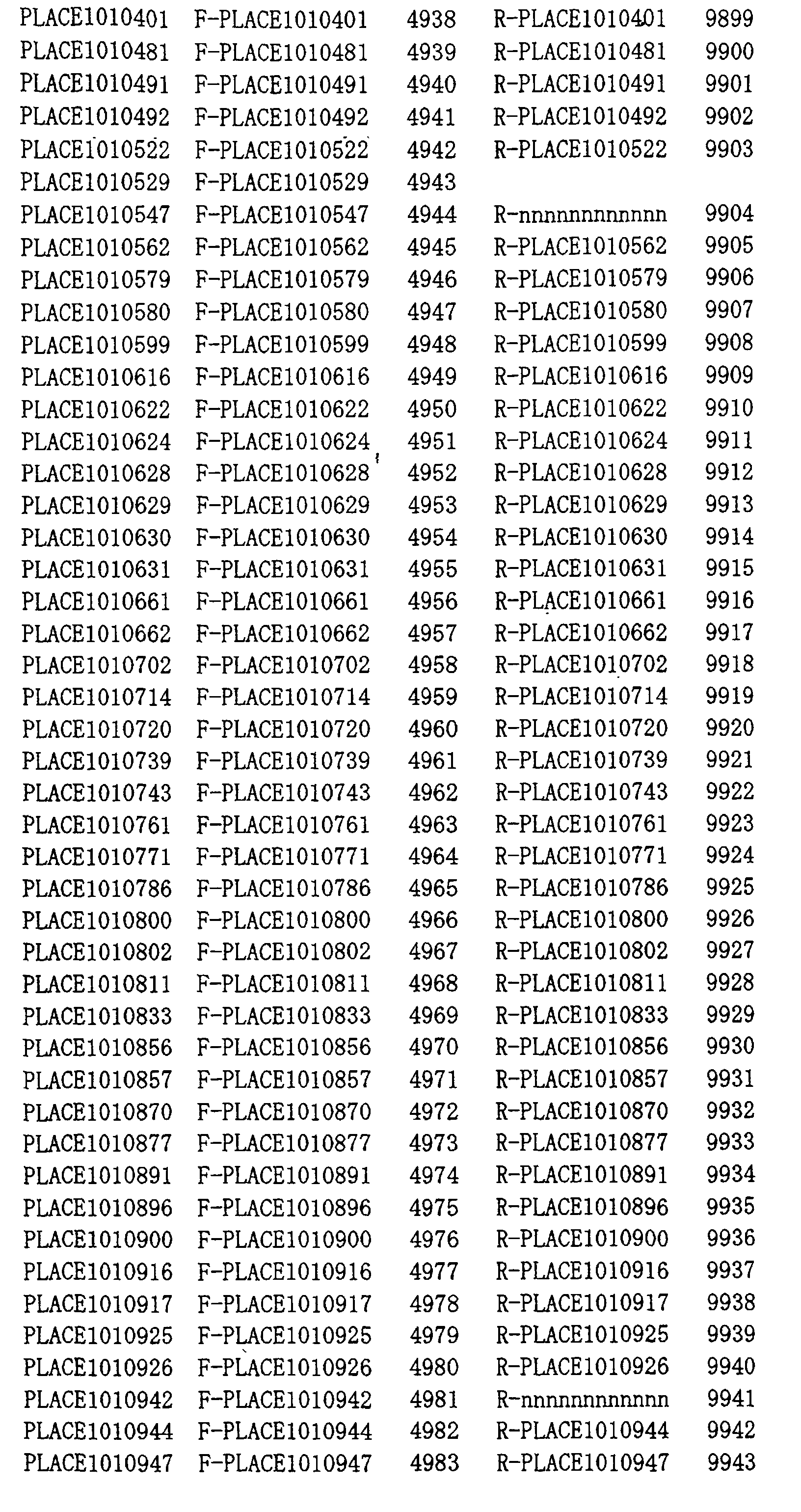

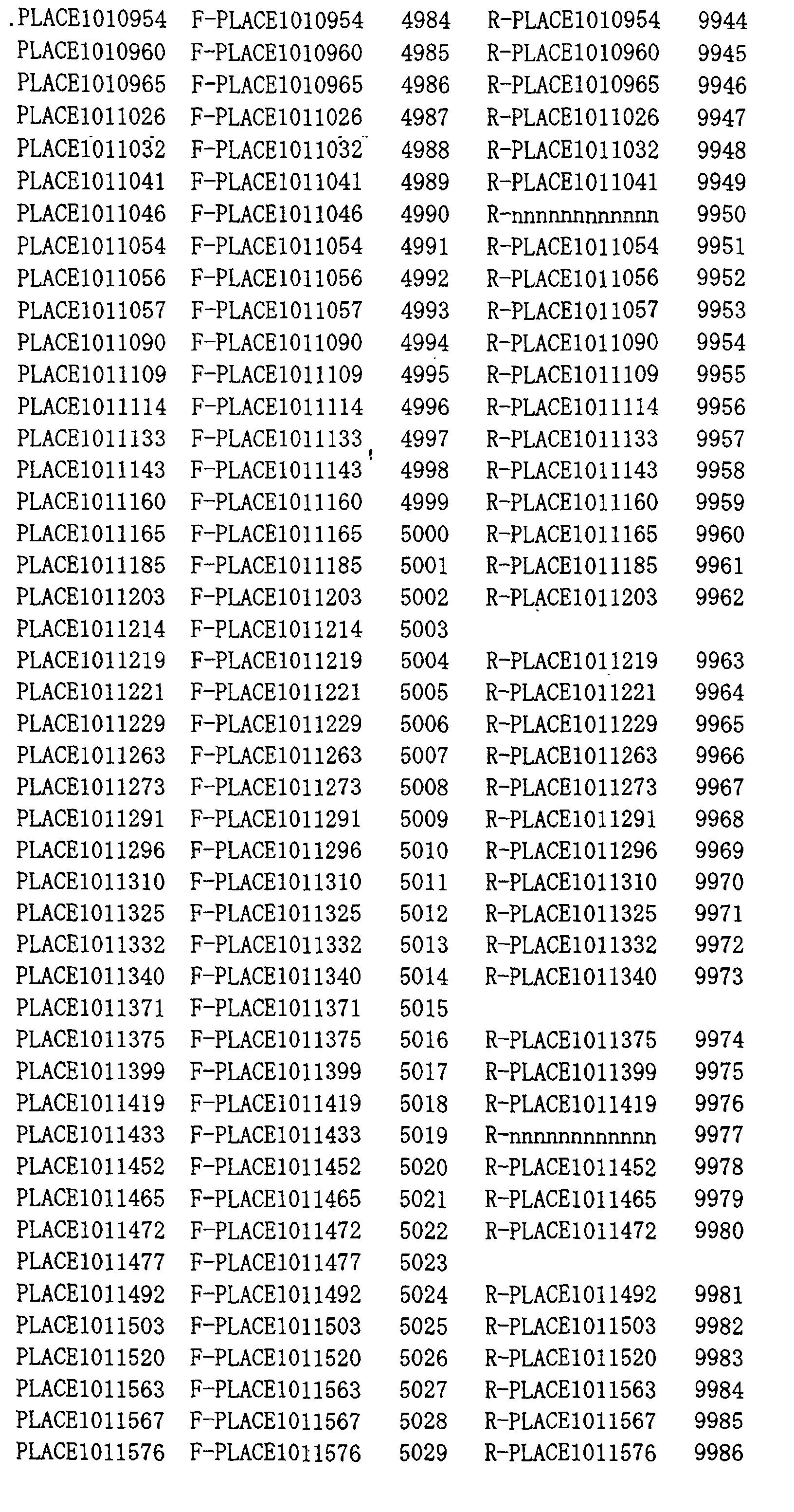

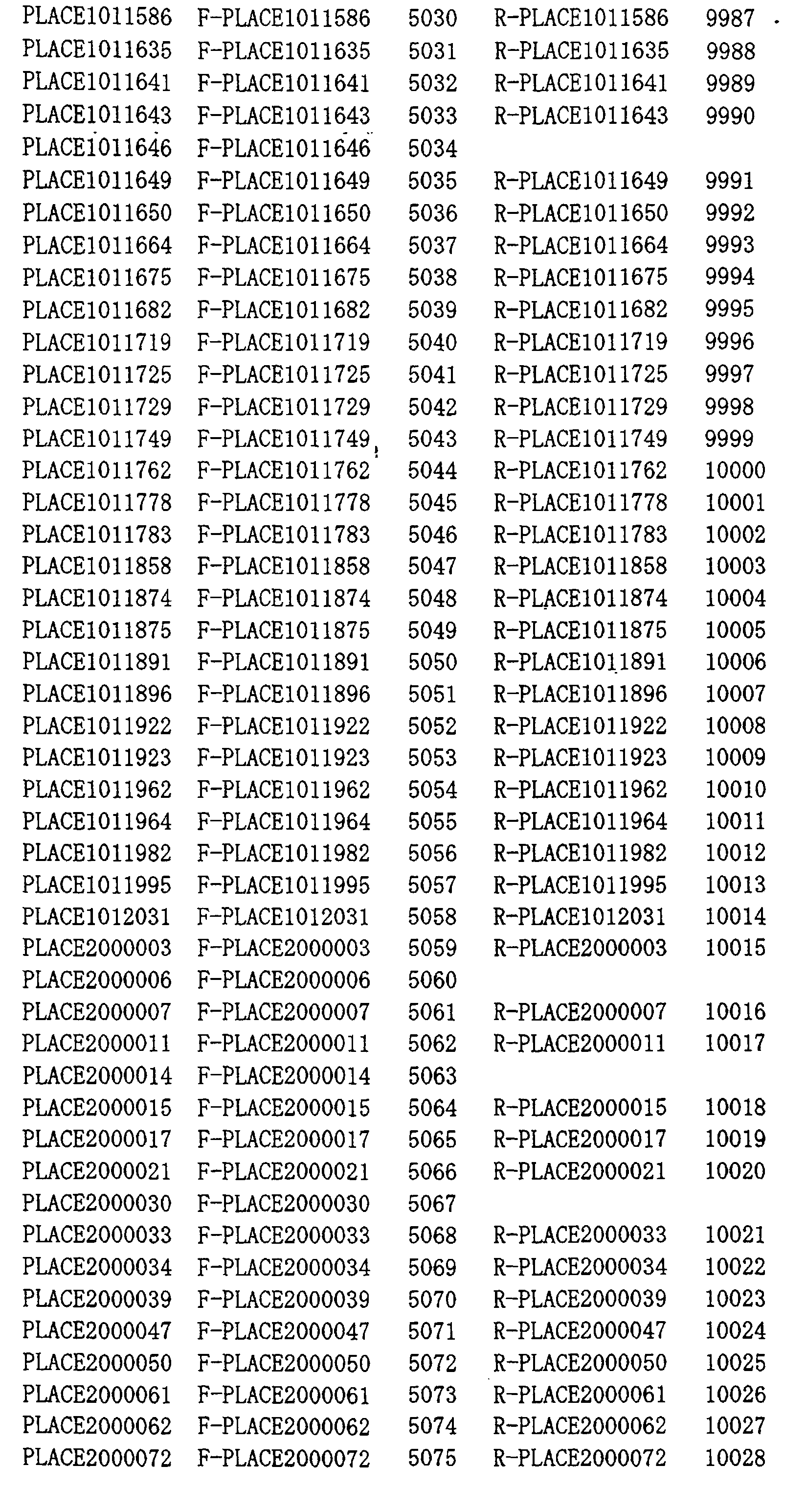

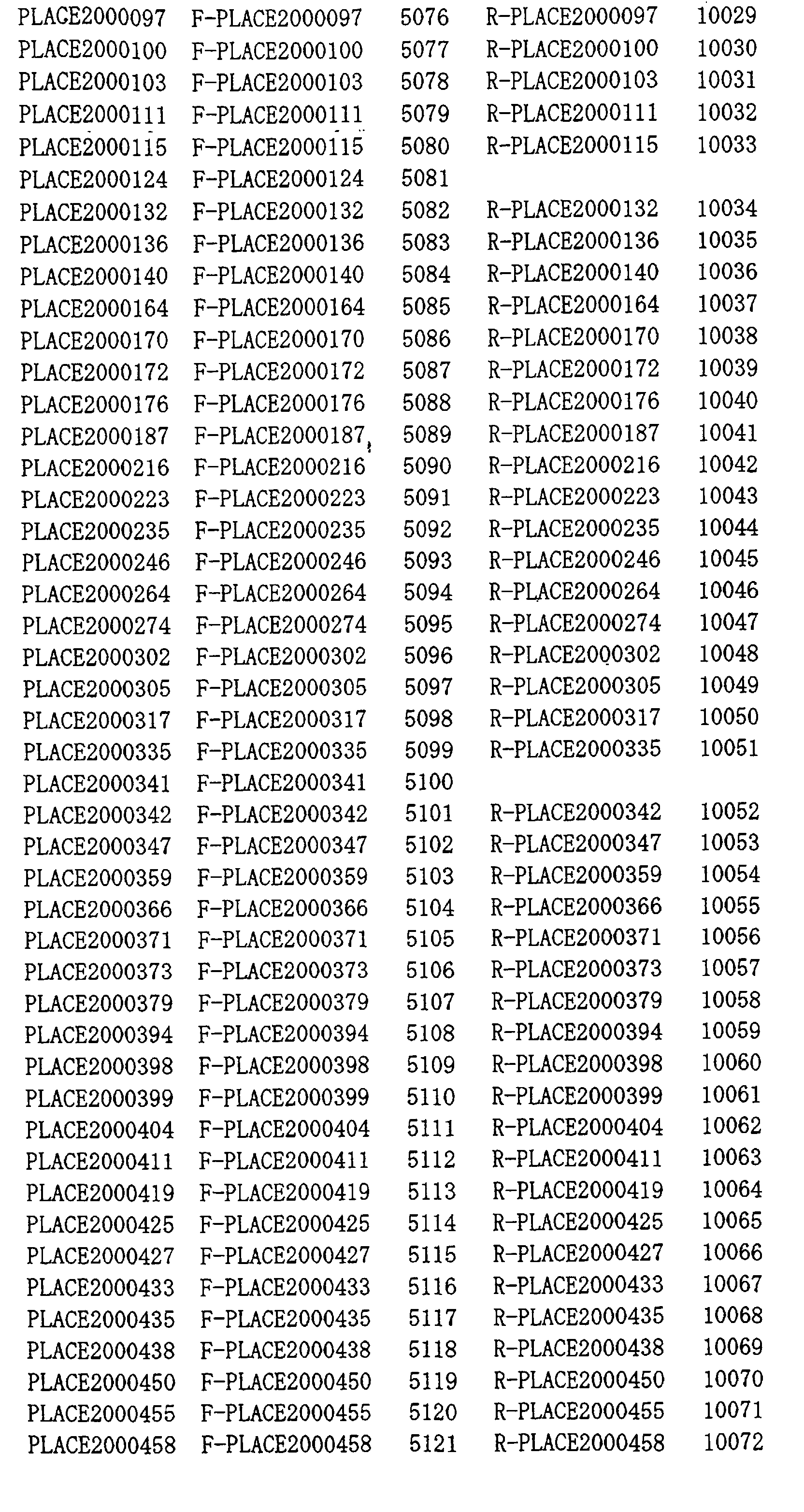

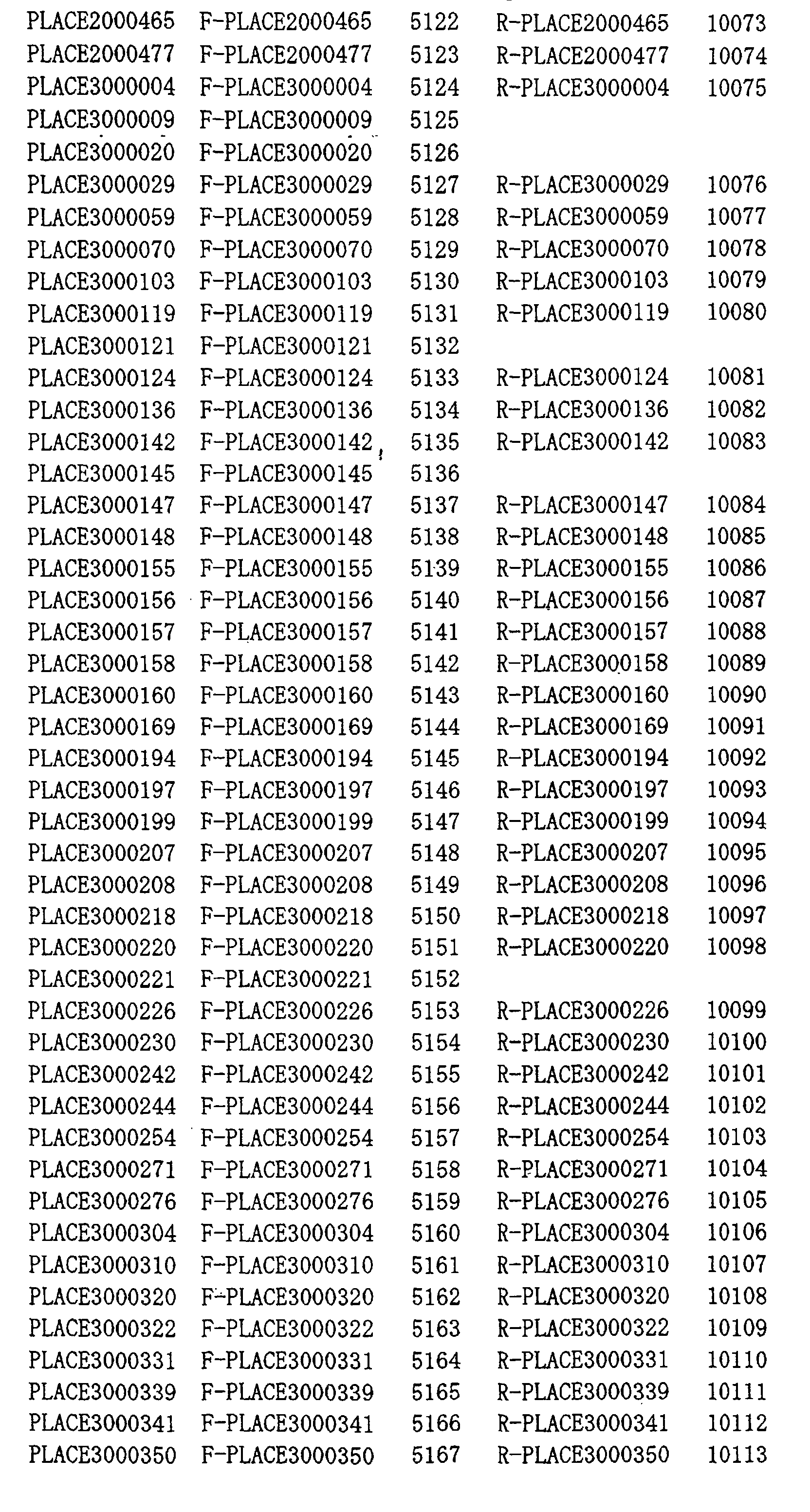

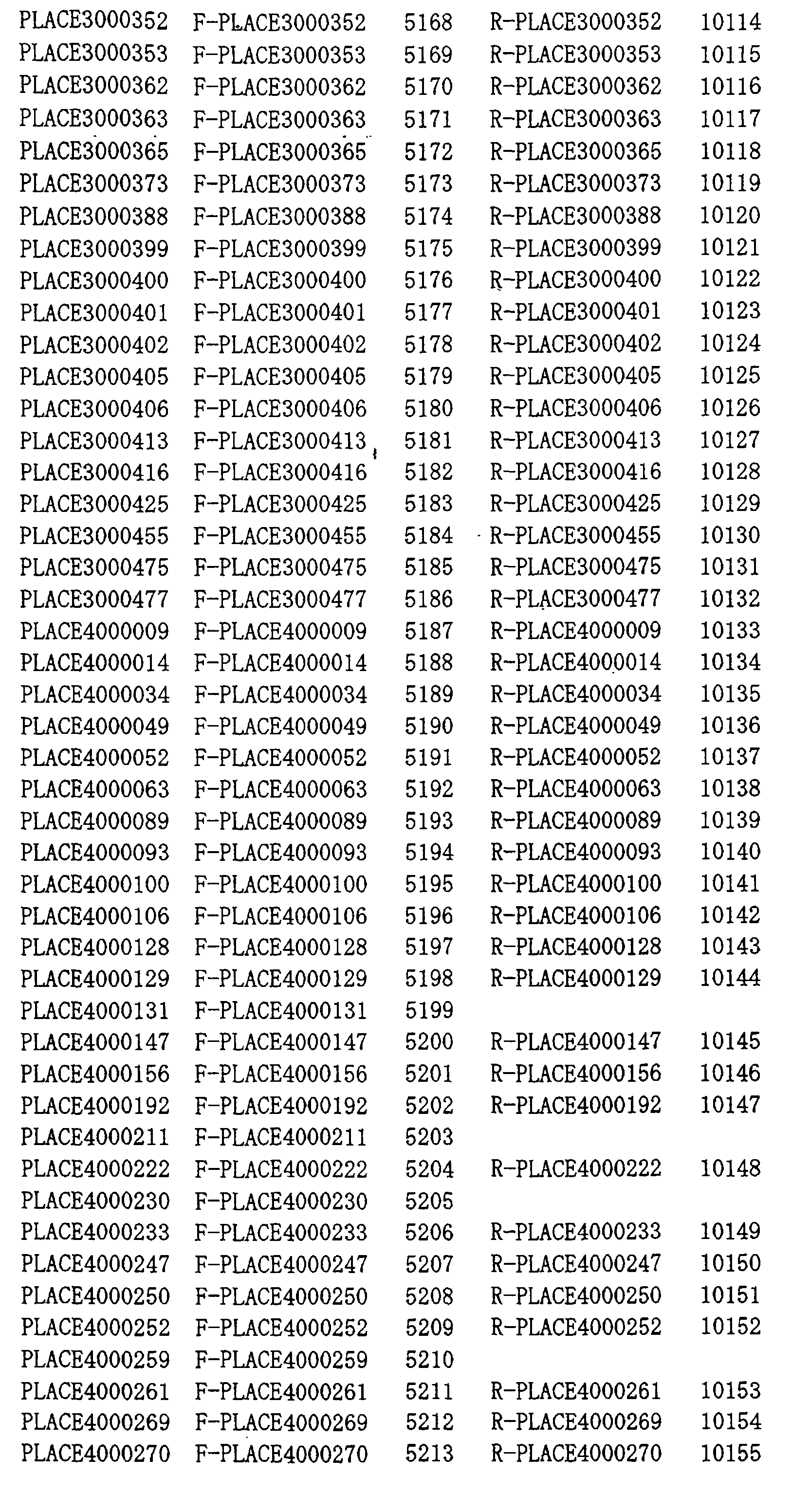

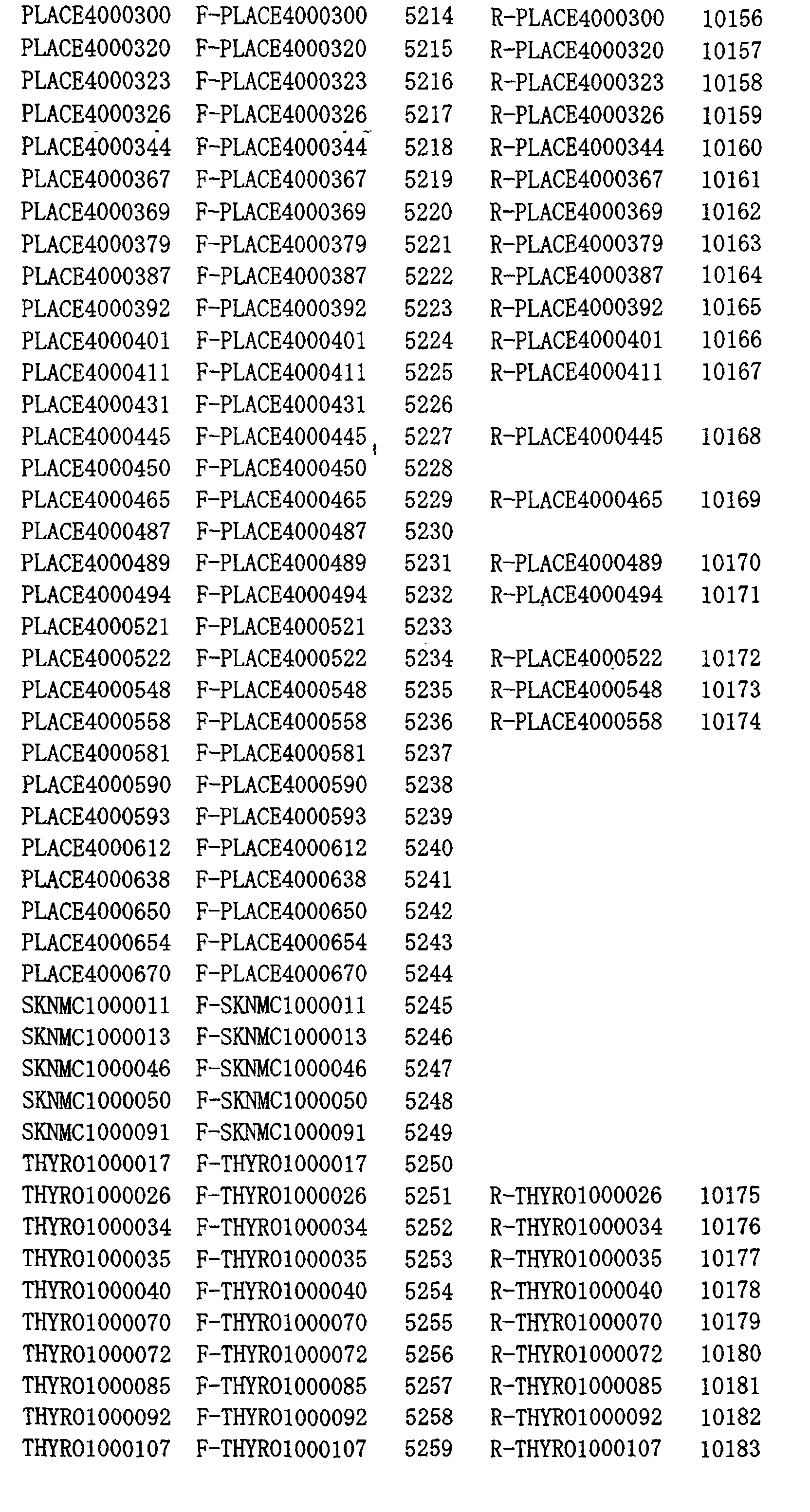

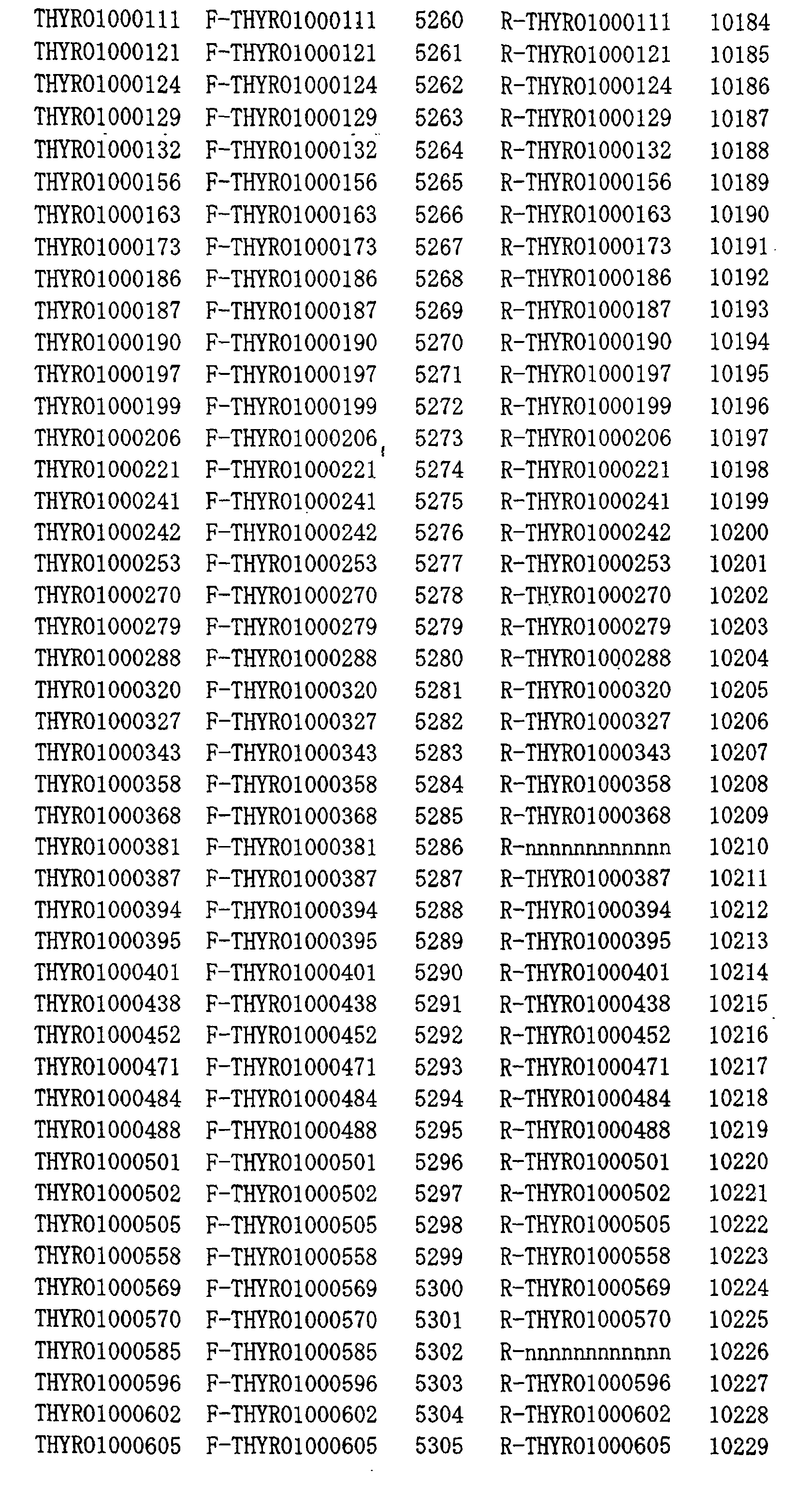

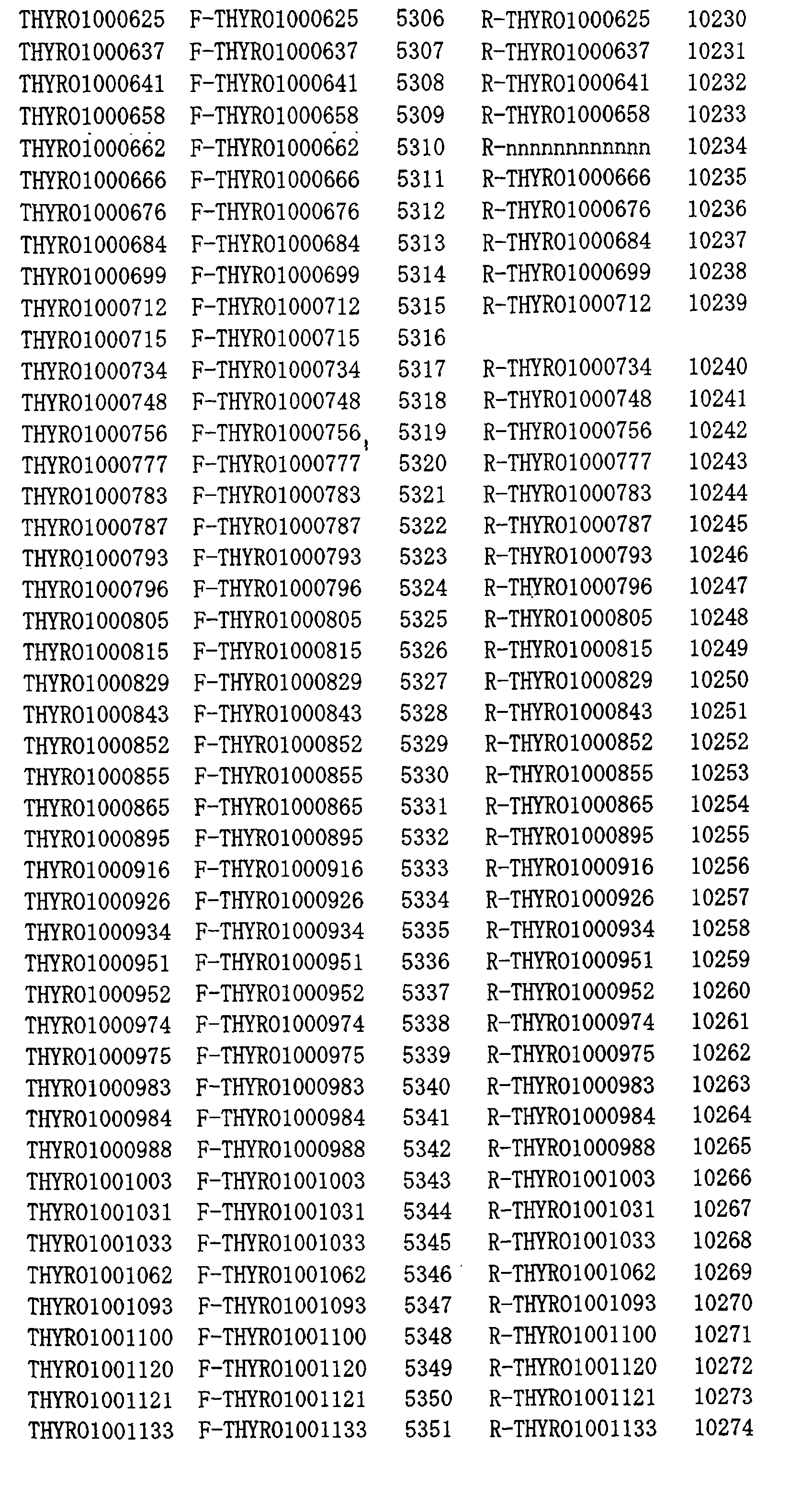

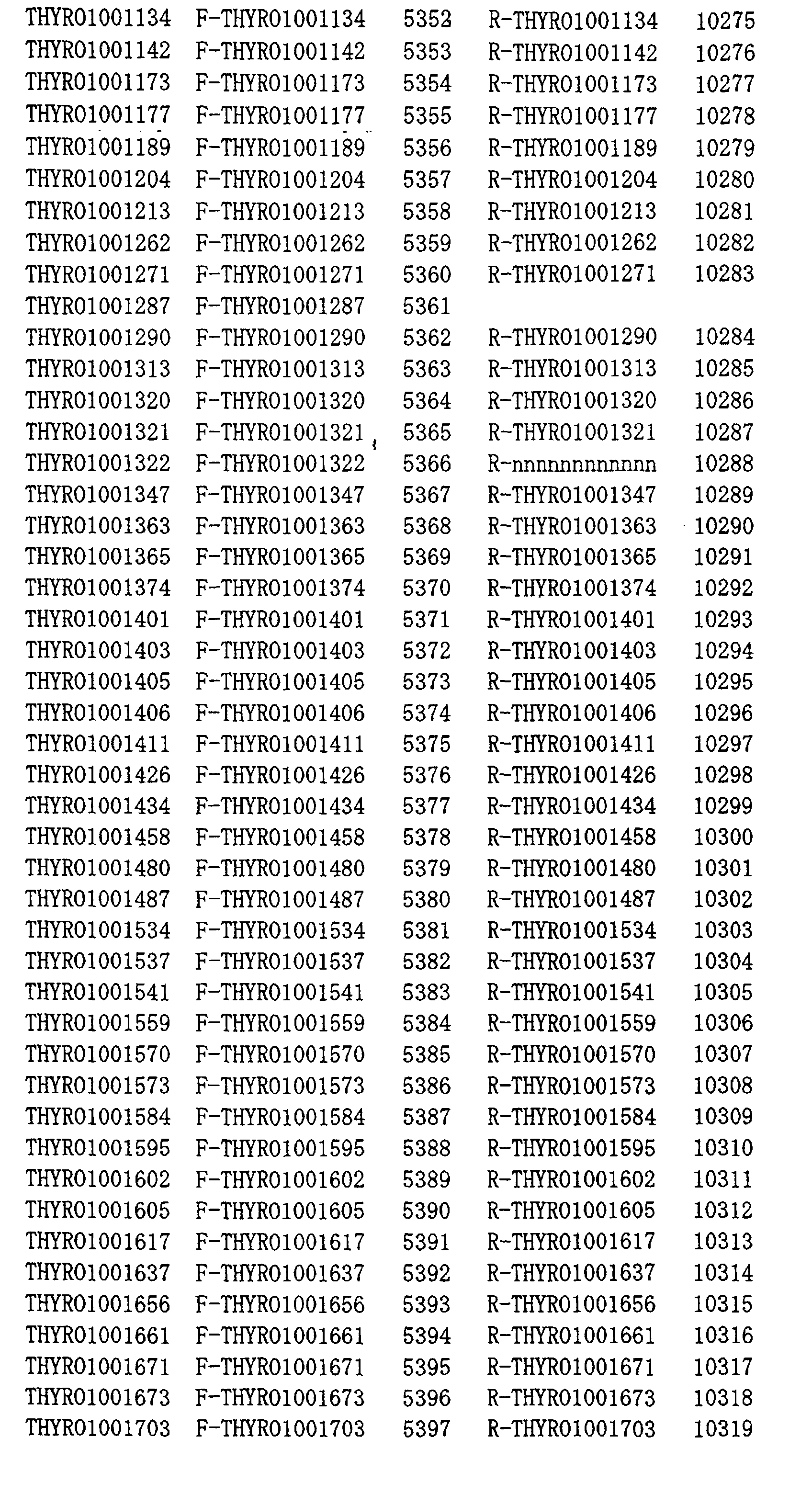

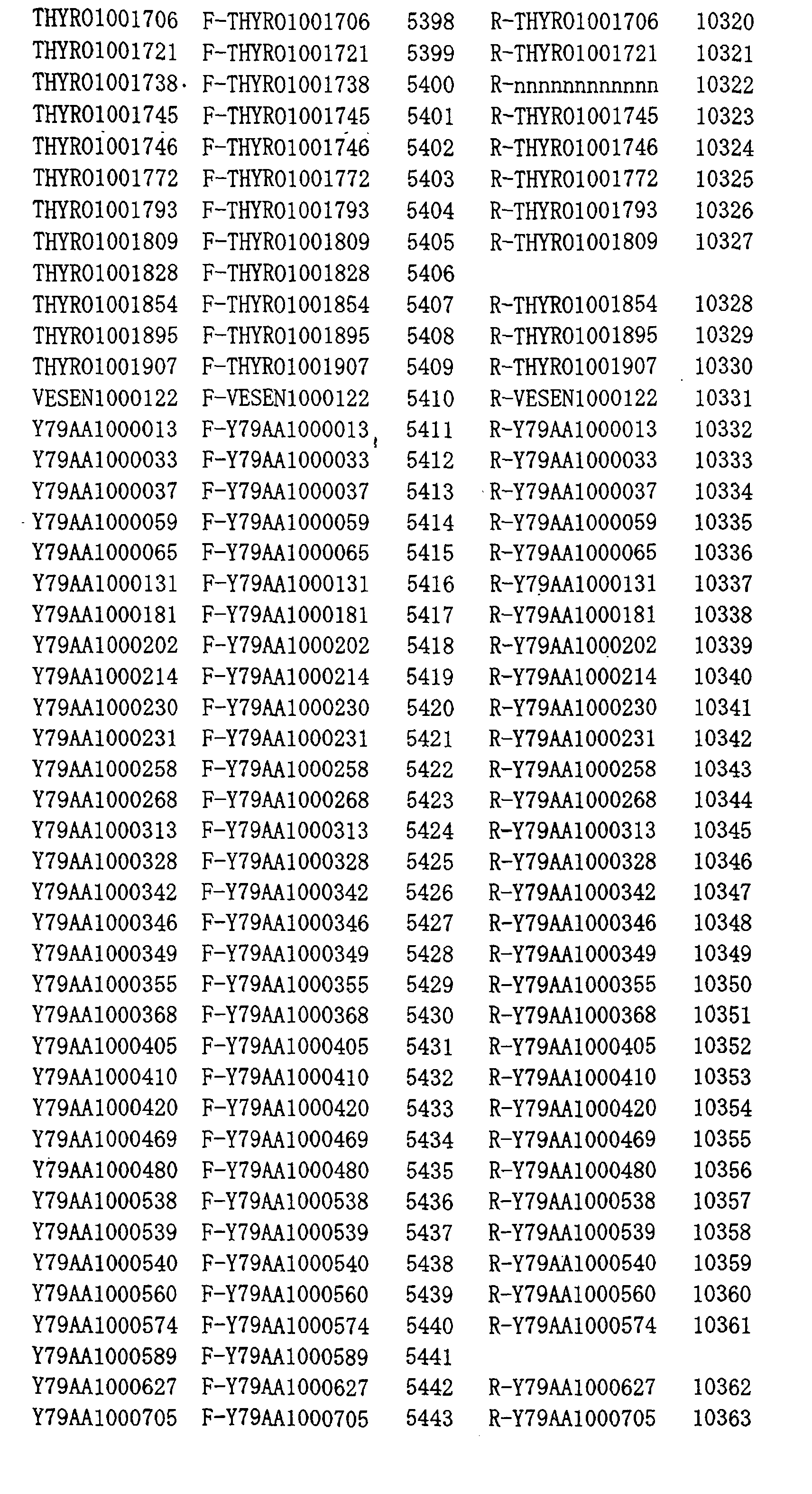

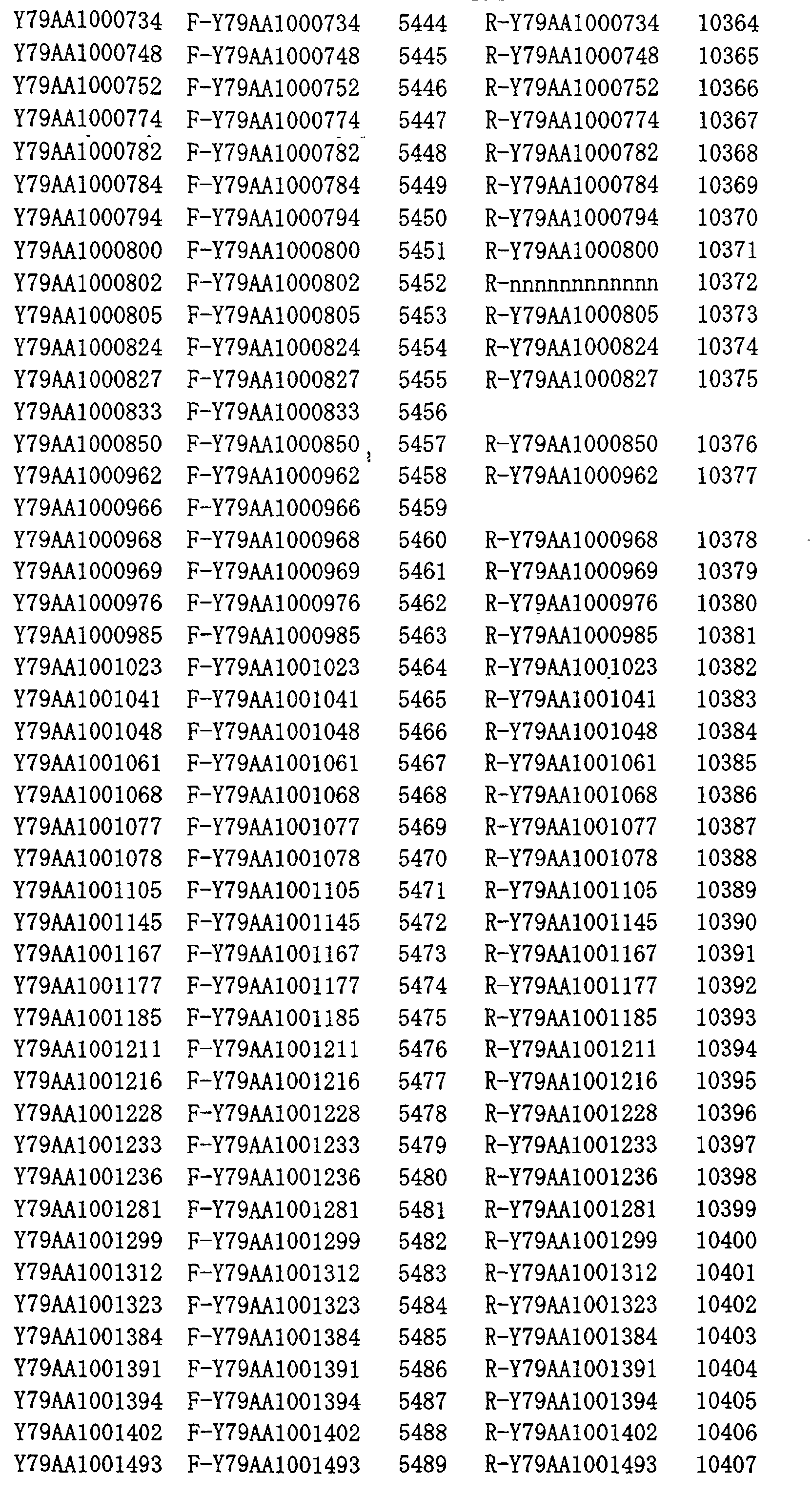

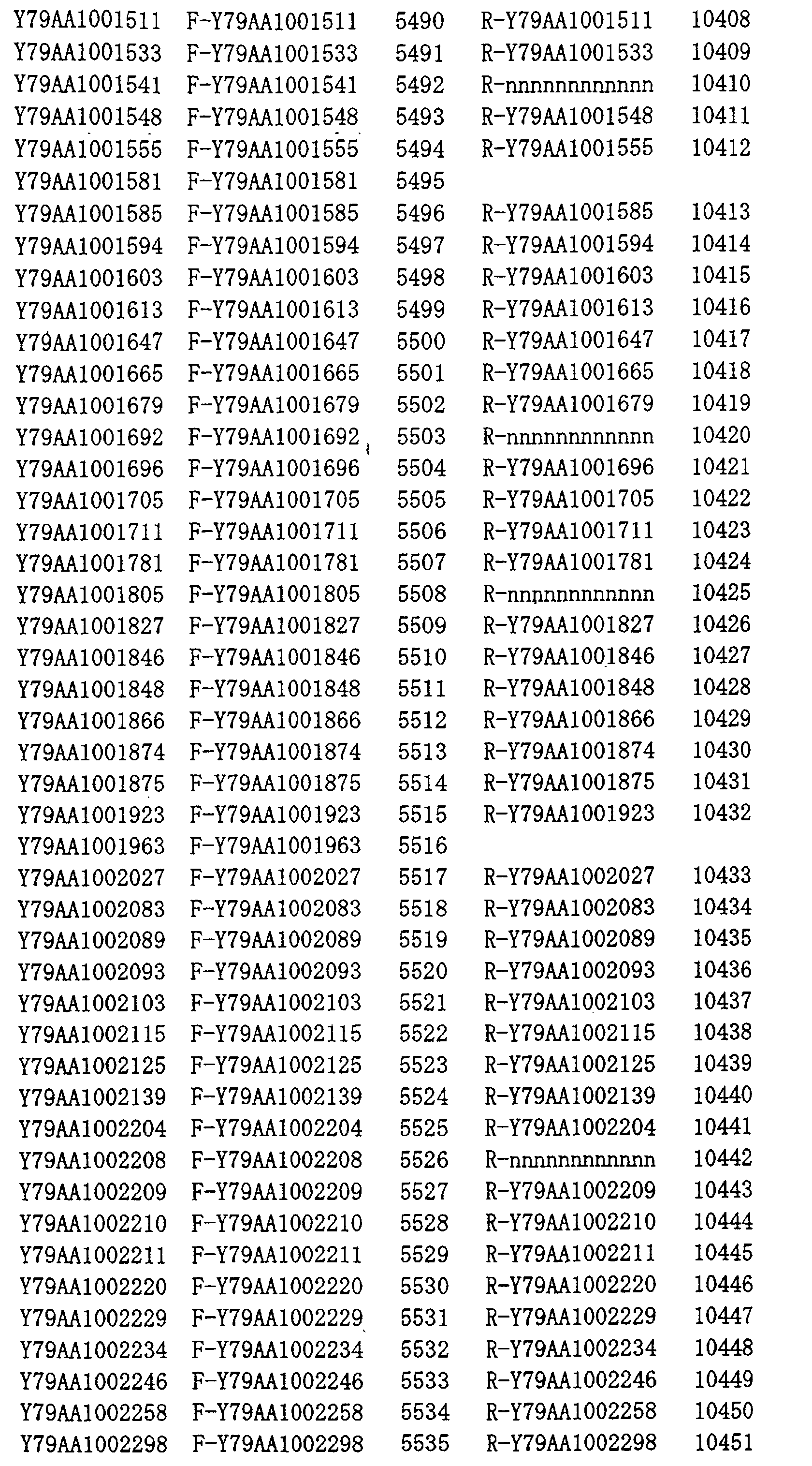

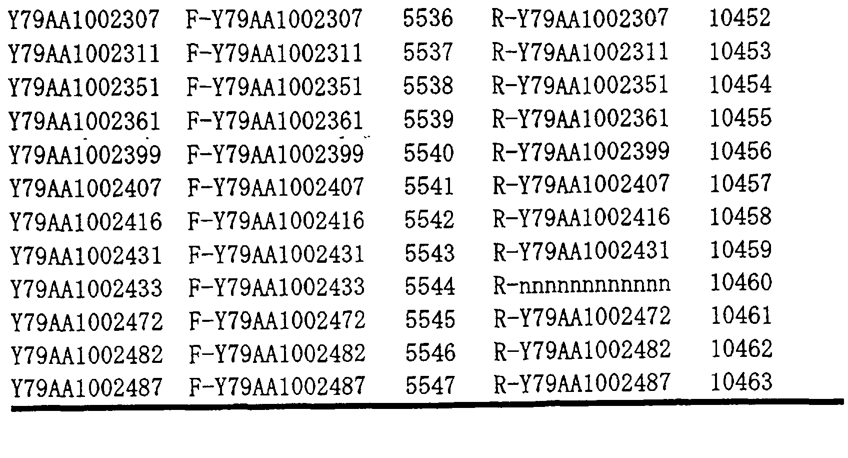

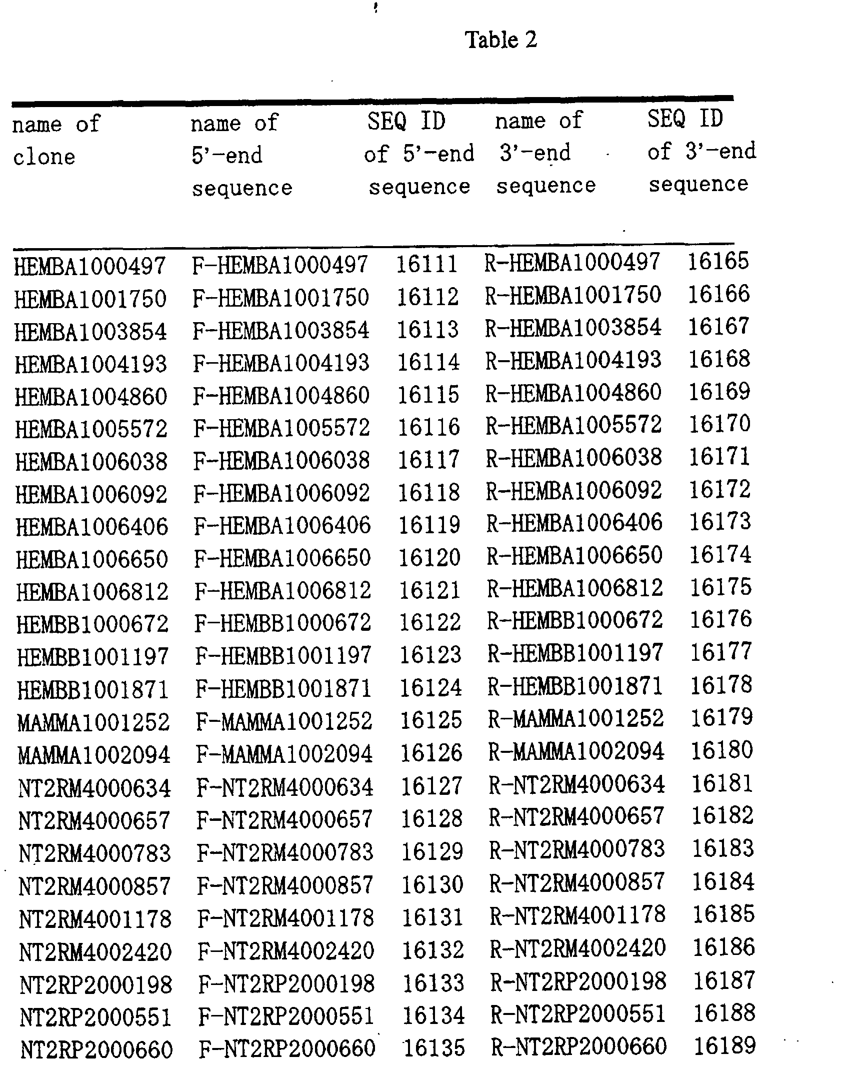

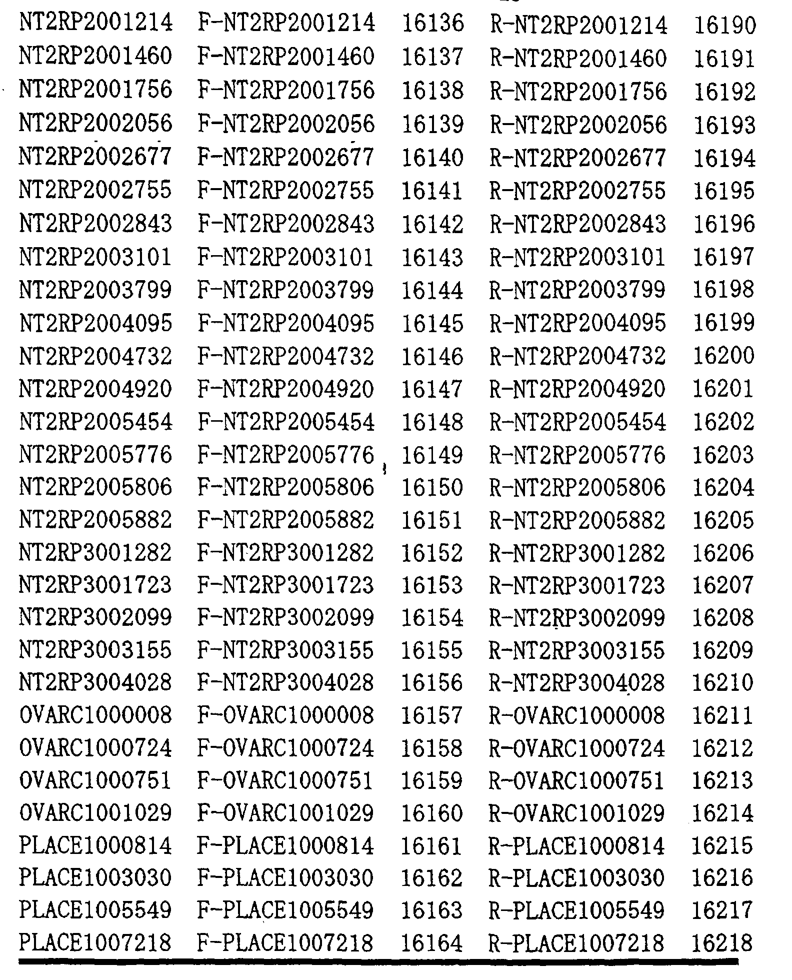

Furthermore, the present invention relates to the use of the above primers, as described below.
(4) A polynucleotide which can be synthesized with the primer set of (2) or (3).
(5) A polynucleotide comprising a coding region in the polynucleotide of (4).
(6) A substantially pure protein encoded by polynucleotide of (4).
(7) A partial peptide of the protein of (6).

In addition, the present invention comprises a polynucleotide described below and a protein encoded by the polynucleotide.
(8) An isolated polynucleotide selected from the group consisting of
   (a) a polynucleotide comprising a coding region of the nucleotide sequence set forth in any one of the SEQ ID NOs in Tables 350 and 351;
   (b) a polynucleotide comprising a nucleotide sequence encoding a protein comprising the amino acid sequence set forth in any one of the SEQ ID NOs in Tables 350 and 351;
   (c) a polynucleotide comprising a nucleotide sequence encoding a protein comprising an amino acid sequence selected from the amino acid sequences set forth in the SEQ ID NOs in Tables 350 and 351, in which one or more amino acids are substituted, deleted, inserted, and/or added, wherein said protein is functionally equivalent to the protein comprising said amino acid sequence selected from the amino acid sequences set forth in the SEQ ID NOs in Tables 350 and 351;
   (d) a polynucleotide that hybridizes with a polynucleotide comprising a nucleotide sequence selected from the nucleotide sequences set forth in the SEQ ID NOs in Tables 350 and 351, and that comprises a nucleotide sequence encoding a protein functionally equivalent to the protein encoded by the nucleotide sequence selected from the nucleotide sequences set forth in the SEQ ID NOs in Tables 350 and 351;
   (e) a polynucleotide comprising a nucleotide sequence encoding a partial amino acid sequence of a protein encoded by the polynucleotide of (a) to (d);
   (f) a polynucleotide comprising a nucleotide sequence with at least 70% identity to the nucleotide sequence set forth in any one of the SEQ ID NOs in Tables 350 and 351.
(9). A substantially pure protein encoded by the polynucleotide of (8).
(10) An antibody against the protein or peptide of any one of (6), (7), and (9).
(11) A vector comprising the polynucleotide of (5) or (8).
(12) A transformant carrying the polynucleotide of (5) or (8), or the vector of (11).
(13) A transformant expressively carrying the polynucleotide of (5) or (8), or the vector of (11).
(14) A method for producing the protein or peptide of any one of (6), (7), and (9), comprising culturing the transformant of (13) and recovering the expression product.
(15) An oligonucleotide comprising the nucleotide sequence set forth in any one of the SEQ ID NOs in Tables 350 and 351 or the nucleotide sequence complementary to the complementary strand thereof, wherein said oligonucleotide comprises 15 nucleotides or more.
(16) Use of the oligonucleotide of (15) as a primer for synthesizing a polynucleotide.
(17) Use of the oligonucleotide of (15) as a probe for detecting a gene.
(18) An antisense polynucleotide against the polynucleotide of (8), or the portion thereof.
(19) A method for synthesizing a polynucleotide, the method comprising:
   a) synthesizing a complementary strand using a cDNA library as a template, and using the primer set of (2) or (3), or the primer of (16); and
   b) recovering the synthesized product.
(20) The method of (19), wherein the cDNA library is obtainable by oligo-capping method.
(21) The method of (19), wherein the complementary strand is obtainable by PCR.
(22) A method for detecting the polynucleotide of (8), the method comprising:
   a) incubating a target polynucleotide with the oligonucleotide of (15) under the conditions where hybridization occurs, and
   b) detecting the hybridization of the target polynucleotide with the oligonucleotide of (15).
(23) A database of polynucleotides and/or proteins, the database comprising information on at least one sequence selected from the nucleotide sequences set forth in the SEQ ID NOs in Tables 350 and 351 and/or the amino acid sequences set forth in the SEQ ID NOs in Tables 350 and 351, or a medium on which the database is stored.

Any patents, patent applications, and publications cited herein are incorporated by reference.

### BRIEF DESCRIPTION OF THE DRAWINGS

Figure 1 shows the restriction maps of vectors pME18SFL3 and pUC19FL3.

Figure 2 shows the reproducibility of gene expression analysis. The respective intensities of gene expression observed in independent set of experiments are plotted in the vertical axis as well as in the horizontal axis.

Figure 3 shows the detection limit in gene expression analysis. The intensity of expression is shown in the vertical axis and the concentration (µg/ml) of probe used is shown in the horizontal axis.

### DETAILED DESCRIPTION OF THE INVENTION

Herein, "polynucleotide" is defined as a molecule in which multiple nucleotides are polymerized. There are no limitations in the number of the polymerized nucleotides. In case that the polymer contains relatively low number of nucleotides, it is also described as an "oligonucleotide". The polynucleotide or the oligonucleotide of the present invention can be a natural or chemically synthesized product. Alternatively, it can be synthesized using a template DNA by an enzymatic reaction such as PCR.

All the cDNA provided by the invention are full-length cDNA. Herein, a "full-length cDNA" is defined as a cDNA which contains both ATG codon (the translation start site) and the stop codon. Accordingly, the untranslated regions, which are originally found in the upstream or downstream of the protein coding region in natural mRNA, may or may not be contained.

An "isolated polynucleotide" is a polynucleotide the structure of which is not identical to that of any naturally occurring nucleic acid or to that of any fragment of a naturally occurring genomic nucleic acid spanning more than three separate genes. The term therefore covers, for example,
(a) a DNA which has the sequence of part of a naturally occurring genomic DNA molecule but is not flanked by both of the coding sequences that flank that part of the molecule in the genome of the organism in which it naturally occurs;
(b) a nucleic acid incorporated into a vector or into the genomic DNA of a prokaryote or eukaryote in a manner such that the resulting molecule is not identical to any naturally occurring vector or genomic DNA;
(c) a separate molecule such as a cDNA, a genomic fragment, a fragment produced by polymerase chain reaction (PCR), or a restriction fragment; and
(d) a recombinant nucleotide sequence that is part of a hybrid gene, i.e., a gene encoding a fusion protein. Specifically excluded from this definition are nucleic acids present in mixtures of different (i) DNA molecules, (ii) transfected cells, or (iii) cell clones: e.g., as these occur in a DNA library such as a cDNA or genomic DNA library.

The term "substantially pure" as used herein in reference to a given polypeptide means that the protein or polypeptide is substantially free from other biological macromolecules. The substantially pure protein or polypeptide is at least 75% (e.g., at least 80, 85, 95, or 99%) pure by dry weight. Purity can be measured by any appropriate standard method, for example, by column chromatography, polyacrylamide gel electrophoresis, or HPLC analysis.

All the clones (5602 clones) of the present invention are novel and encode the full-length proteins. All the clones were prepared by oligo capping method, which can achieve cDNA cloning with high fullness ratio. The cDNA clones were selected by using ATGprl score as an index of the fullness ratio at the 5'-end, based on the sequence features of the 5'-end sequences. Selection was further carried out by searching GenBank database for EST sequences homologous to 5'-end sequence of each clone by BLAST [S.F. Altschul, W. Gish, W. Miller, E.W. Myers & D.L. Lipman J. Mol. Biol., 215:403-410 (1990); W. Gish, & D.J. States, Nature Genet., 3:266-272 (1993)] and by considering the number of matching (identical) EST sequences or the number of continuous amino acids in the 5'-end sequence initiated from the initiation codon.

Moreover, the clones were turn out to be not identical to any of the known human mRNA (namely novel) by homology search using the 5'-end sequence.

The primers of the present invention, which are used for synthesizing full-length cDNA, are selected from the group comprising SEQ ID NO: 1-5547 (5'-primer), or SEQ ID NO: 5548-10463 (3'-primer). Further, the primers of the present invention, which are used for synthesizing full-length cDNA, are selected from SEQ ID NO: 16111-16164 (5'-primer), or SEQ ID NO: 16165-16218 (3'-primer). Some of the nucleotides include a known EST as its part. However, the primers of the present invention are novel in terms that the primers enable to synthesize full-length cDNA. Because the known ESTs lack important information on what part of cDNA the ESTs correspond to, it is impossible to design primers on the basis of the ESTs.

All the full-length cDNA of the present invention can be synthesized using a primer set comprising the nucleotide sequences selected from both the 5'-and 3'-end sequences, or a set comprising a primer based on the 5'-end sequence and an oligo-dT primer, by a method such as PCR (Current protocols in Molecular Biology (1987) Ausubel et al. edit, John Wiley & Sons, Section 6.1-6.4).

Specifically, PCR can be performed using an oligonucleotide that has 15 nucleotides longer, and specifically hybridizes with the complementary strand of the polynucleotide that contains the nucleotide sequence selected from the 5'-end sequences shown in Table 1 and 2 (SEQ ID NO: 1-5547, or SEQ ID NO: 16111-16164), and an oligo-dT primer as a 5'-, and 3'-primer, respectively. The length of the primers is usually 15-100 bp, and favorably between 15-35 bp. In case of LA PCR, which is described below, the primer length of 25-35 bp may provide a good result.

A method to design a primer that enables a specific amplification based on the given nucleotide sequence is known to those skilled in the art (Current Protocols in Molecular Biology, Ausubel et al. edit, (1987) John Wiley & Sons, Section 6.1-6.4). In designing a primer based on the 5'-end sequence, the primer is designed so as that, in principle, the amplification products will include the translation start site. Accordingly, in case that a given 5'-end nucleotide sequence is the 5'- untranslated region (5'UTR), any part of the sequence can be used as a 5'-primer as far as the specificity toward the target cDNA is insured. The translation start site can be predicted using a known method such as the ATGpr as described below.

When synthesizing a polynucleotide, the target nucleotide sequence to be amplified can extend to several thousand bp in some cDNA. However, it is possible to amplify such a long nucleotides by using such as LA PCR (Long and Accurate PCR). It is advantageous to use LA PCR when synthesizing long DNA. In LA PCR, in which a special DNA polymerase having 3' →5' exonuclease activity is used, misincorporated nucleotides can be removed. Accordingly, accurate synthesis of the complementary strand can be achieved even with a long nucleotide sequence. By using LA PCR, it is reported that amplification of a nucleotide with 20 kb longer can be achieved under desirable condition (Takeshi Hayashi (1996) Jikken-Igaku Bessatsu, "Advanced Technologies in PCR" Youdo-sha).

A template DNA for synthesizing the cDNA of the present invention can be obtained by using cDNA libraries that are prepared by various methods. The full-length cDNA clones obtained here are those with high fullness ratio, which were obtained using a combination of (1) a method to prepare a full-length-enriched cDNA library using the oligo-capping method, and (2) an estimation system for fullness using the 5'-end sequence (selection based on the estimation by the ATGpr after removing clones that are not-full-length compared to the ESTs). However, it is possible to easily obtain a full-length cDNA by using the primers that are provided by the present invention, not by the above described specialized method.
The problem with the cDNA libraries prepared by the known methods or commercially available is that mRNA contained in the libraries has very low fullness ratio. Thus, it is difficult to screen full-length cDNA clone directly from the library using ordinary cloning methods. The present invention has revealed a primer that is capable of synthesizing a full-length cDNA. If provided with primers, it is possible to synthesize a target full-length cDNA by using enzymatic reactions such as PCR. In particular, a full-length-enriched cDNA library, synthesized by methods such as oligo-capping, is desirable to synthesize a full-length cDNA with more reliability.

The 5'-end sequence of the full-length cDNA clones of the invention can be used to isolate the regulatory element of transcription including the promoter on the genome. By the spring of the year 2000, a rough draft of the human genome (analysis of human genomic sequence with lower accuracy), which covers 90% of the genome, is planned to be accomplished, and by the year 2003, analysis of the entire human genomic sequence is going to be finished. However, it is hard to analyze with software the transcription start sites on the human genome, in which long introns exist. By contrast, it is easy to specify the transcription start site on the genomic sequence using the 5'-end sequence of the full-length cDNA clone, thus it is easy to obtain the genomic region involved in transcription regulation, which includes the promoter that is contained in the upstream of the transcription start site.

The full-length cDNAs cloned in the present invention are classified into 13 groups, based on the data such as ATGpr1 score, by which the fullness ratio can be evaluated. Specifically, the 13 groups consist of; the below-mentioned groups (1)-(3), containing 3690 clones (Table 9), and the group (12), containing 3 clones, wherein ATGpr1 (score defined in the ATGpr program) is higher than 0.3; and the below-mentioned groups (4)-(11), containing 1857 clones (Table 10), and the group (13), containing 52 clones, wherein, although ATGpr1 is 0.3 or less, the clones are judged to be full-length from various viewpoints. Names of the clones belonging to the groups (1)-(13) are as indicated in Examples or below.
(1) 1516 clones
   Among the 3690 clones that have the maximal ATGpr1 score higher than 0.3, 1516 clones are novel full-length clones, in which at least either of the sequences of the 5'- and 3'-ends, or both are not identical to those of any human EST.
(2)377 clones
   Among the 3690, 377 clones are novel full-length clones, in which the number of human EST having identical sequence at both 5'- and 3'-ends is 1 to 5.
(3) 1797 clones
   Among the 3690, 1797 clones are novel full-length clones, in which the number of human EST having identical sequence at the 5'-end is not more than 20 (except the clones described above).
(4) 453 clones
   Among the 1857 clones in which the maximal ATGpr1 score is 0.3 or less, the following 453 clones are estimated to be novel full-length clones since the clones have the maximal score 0.3 or more in the ATGpr2, and at least either of the sequences of their 5'- and 3'-ends, or both are not identical to those of any human EST. The ATGpr2 score is determined by using the ATGpr program with neglecting the information of the frequency of the six nucleotides contained within the sequence between the ATG codon and the stop codon (the maximal length is 300 nucleotides from the ATG codon) (Salamo A.A., Nishikawa T., and Swindells M.B. (1998) Bioinformatics, 14: 384-390; http://www.hri.co.jp/atgpr/). The ATGpr program for calculating the ATGpr2 score is described as the ATGpr2 program in the followings.
(5) 24 clones
   Among the 1857 clones, 24 clones are estimated to be full-length since their maximal ATGpr2 scores are higher than 0.3, and also novel, though they have low scores in ATGpr1 program, in which the number of the human EST having identical sequence at both 5'- and 3'-ends is 1 to 5.
(6) 65 clones
   Among the 1857 clones, 65 clones are estimated to be full-length since, though they have low scores in both programs, ATGpr1 and ATGpr2, the scores are the maximum in comparison to those of the other clones in the same cluster (at least two clones). The clones are also novel, if at least either of the sequences of the 5'- and 3'-ends, or both are not identical to those of any human EST.
(7) 32 clones
   Among the 1857 clones, 32 clones are estimated to be full-length since, though they have low scores in both programs, ATGpr1 and ATGpr2, the scores are the maximum in comparison to those of the other clones in the same cluster (at least two clones). The clones are also novel, if the number of the human EST having identical sequence at both 5'- and 3'-ends is 1 to 5.
(8) 36 clones
   Among the 1857 clones, 36 clones are full-length, which were selected by assembling the sequences of the other clones or human EST, although they have low scores in both programs, ATGpr1 and ATGpr2. The clones are also novel, if at least either of the sequences of the 5'- and 3'-ends, or both are not identical to those of any human EST.
(9) 81 clones
   Among the 1857 clones, 81 clones are full-length, which were selected by assembling the sequences of the other clones or human EST, although they have low scores in both programs, ATGpr1 and ATGpr2. The clones are also novel, if the number of the human EST having identical sequence at the 5'-end is not more than 20 (other than the clones in which at least either of the sequences of the 5'- and 3'-ends, or both are not identical to those of any human EST).
(10) 938 clones
   Among the 1857 clones, 938 clones are estimated to be full-length according to the fullness ratio shown in Table 4, although they have low scores in both programs, ATGpr1 and ATGpr2. The clones are also novel, if at least the sequence of the 5'-end is not identical to those of any human EST.
(11) 228 clones
   Among the 1857 clones, 228 clones are estimated to be full-length according to the fullness ratio shown in Table 7, although they have low scores in both programs, ATGpr1 and ATGpr2. The clones are also novel, if at least the sequence of the 3'-end is not identical to those of any human EST.
(12) 3 clones
   Three clones, HEMBA1006812, HEMBB1001871, and NT2RP3001282, whose maximal ATGpr1 values are higher than 0.3, are full-length and novel clones whose 5'-end sequences presumably contain a coding region which is initiated with ATG codon and which encodes 100 amino acids or more.
(13) 52 clones
   The following 52 clones, which have maximal ATGpr1 values of 0.3 or less, are full-length with the fullness ratios shown in Table 4 although the fullness ratios are low:
   HEMBA1000497, HEMBA1001750, HEMBA1003854, HEMBA1004193, HEMBA1004860, HEMBA1005572, HEMBA1006038, HEMBA1006092, HEMBA1006406, HEMBA1006650, HEMBB1000672, HEMBB1001197, MAMMA1001252, MAMMA1002094, NT2RM4000634, NT2RM4000657, NT2RM4000783, NT2RM4000857, NT2RM4001178, NT2RM4002420, NT2RP2000198, NT2RP2000551, NT2RP2000660, NT2RP2001214, NT2RP2001460, NT2RP2001756, NT2RP2002056, NT2RP2002677, NT2RP2002755, NT2RP2002843, NT2RP2003101, NT2RP2003799, NT2RP2004095, NT2RP2004732, NT2RP2004920, NT2RP2005454, NT2RP2005776, NT2RP2005806, NT2RP2005882, NT2RP3001723, NT2RP3002099, NT2RP3003155, NT2RP3004028, OVARC1000008, OVARC1000724, OVARC1000751, OVARC1001029, PLACE1000814, PLACE1003030, PLACE1005549, PLACE1007218, NT2RP4002298.
   Moreover, the clones are novel clones whose 5' -end sequences presumably contain a coding region which is initiated with ATG codon and which encodes 50 amino acids or more. Among them, the following 20 clones is predicted to contain a coding region with 100 amino acids or more and should encode proteins:
   HEMBA1000497, HEMBA1003854, HEMBA1004193, NT2RM4000657, NT2RM4001178, NT2RP2001756, NT2RP2002677, NT2RP2002755, NT2RP2002843, NT2RP2004095, NT2RP2004920, NT2RP2005806, NT2RP3002099, NT2RP3003155, OVARC1000724, OVARC1001029, PLACE1000814, PLACE1003030, PLACE1005549, PLACE1007218.

The protein encoded by the polynucleotide of the invention can be prepared as a recombinant protein or as a natural protein. For example, the recombinant protein can be prepared by inserting the polynucleotide encoding the protein of the invention into a vector, introducing the vector into an appropriate host cell and purifying the protein expressed within the transformed host cell, as described below. In contrast, the natural protein can be prepared, for example, by utilizing an affinity column to which an antibody against the protein of the invention (Current Protocols in Molecular Biology (1987) Ausubel et al. edit, John Wily & Sons, Section 16.1-16.19) is attached. The antibody used for affinity purification may be either a polyclonal antibody, or a monoclonal antibody. Alternatively, in vitro translation (See, for example, "On the fidelity of mRNA translation in the nuclease-treated rabbit reticulocyte lysate system." Dasso M.C., and Jackson R.J. (1989) Nucleic Acids Res. 17: 3129-3144) may be used for preparing the protein of the invention.

Proteins functionally equivalent to the proteins of the present invention can be prepared based on the activities, which were clarified in the above-mentioned manner, of the proteins of the present invention. Using the biological activity possessed by the protein of the invention as an index, it is possible to verify whether or not a particular protein is functionally equivalent to the protein of the invention by examining whether or not the protein has said activity.

Proteins functionally equivalent to the proteins of the present invention can be prepared by those skilled in the art, for example, by using a method for introducing mutations into an amino acid sequence of a protein (for example, site-directed mutagenesis (Current Protocols in Molecular Biology, edit, Ausubel et al., (1987) John Wiley & Sons, Section 8.1-8.5). Besides, such proteins can be generated by spontaneous mutations. The present invention comprises the proteins having one or more amino acids substitutions, deletions, insertions and/or additions in the amino acid sequences of the proteins of the present invention (Tables 350 and 351), as far as the proteins have the equivalent functions to those of the proteins identified in the present Examples described later.

There are no limitations in the number and sites of amino acid mutations, as far as the proteins maintain the functions thereof. The number of mutations is typically 30% or less, or 20% or less, or 10% or less, preferably within 5% or less, or 3% or less of the total amino acids, more preferably within 2% or less or 1 % or less of the total amino acids. From the viewpoint of maintaining the protein function, it is preferable that a substituted amino has a similar property to that of the original amino acid. For example, Ala, Val, Leu, Ile, Pro, Met, Phe and Trp are assumed to have similar properties to one another because they are all classified into a group of non-polar amino acids. Similarly, substitution can be performed among non-charged amino acid such as Gly, Ser, Thr, Cys, Tyr, Asn, and Gln, acidic amino acids such as Asp and Glu, and basic amino acids such as Lys, Arg, and His.

In addition, proteins functionally equivalent to the proteins of the present invention can be isolated by using techniques of hybridization or gene amplification known to those skilled in the art. Specifically, using the hybridization technique (Current Protocols in Molecular Biology, edit, Ausubel et al., (1987) John Wiley & Sons, Section 6.3-6.4)), those skilled in the art can usually isolate a DNA highly homologous to the DNA encoding the protein identified in the present Example based on the identified nucleotide sequence (Tables 350 and 351) or a portion thereof and obtain the functionally equivalent protein from the isolated DNA. The present invention include proteins encoded by the DNAs hybridizing with the DNAs encoding the proteins identified in the present Example, as far as the proteins are functionally equivalent to the proteins identified in the present Example. Organisms from which the functionally equivalent proteins are isolated are illustrated by vertebrates such as human, mouse, rat, rabbit, pig and bovine, but are not limited to these animals.

Washing conditions of hybridization for the isolation of DNAs encoding the functionally equivalent proteins are usually "1 × SSC, 0.1% SDS, 37°C"; more stringent conditions are "0.5 × SSC, 0.1% SDS, 42°C"; and still more stringent conditions are "0.1 × SSC, 0.1% SDS, 65°C". Alternatively, the following conditions can be given as hybridization conditions of the present invention. Namely, conditions in which the hybridization is done at "6 × SSC, 40% Formamide, 25°C", and the washing at "1 × SSC, 55°C" can be given. More preferable conditions are those in which the hybridization is done at "6 × SSC, 40% Formamide, 37°C", and the washing at "0.2 × SSC, 55°C". Even more preferable are those in which the hybridization is done at "6 × SSC, 50% Formamide, 37°C", and the washing at "0.1 × SSC, 62°C". The more stringent the conditions of hybridization are, the more frequently the DNAs highly homologous to the probe sequence are isolated. Therefore, it is preferable to conduct hybridization under stringent conditions. Examples of stringent conditions in the present invention are, washing conditions of "0.5 × SSC, 0.1% SDS, 42°C", or alternatively, hybridization conditions of "6 × SSC, 40% Formamide, 37°C", and the washing at "0.2 × SSC, 55°C". However, the above-mentioned combinations of SSC, SDS and temperature conditions are indicated just as examples. Those skilled in the art can select the hybridization conditions with similar stringency to those mentioned above by properly combining the above-mentioned or other factors (for example, probe concentration, probe length and duration of hybridization reaction) that determines the stringency of hybridization.

The amino acid sequences of proteins isolated by using the hybridization techniques usually exhibit high homology to those of the proteins of the present invention, which are shown in Tables 350 and 351. The present invention encompasses a polynucleotide comprising a nucleotide sequence that has a high identity to the nucleotide sequence of claim 8 (a).
Furthermore, the present invention encompasses a peptide, or protein comprising an amino acid sequence that has a high identity to the amino acid sequence encoded by the polynucleotide of claim 8 (b). The term "high identity" indicates sequence identity of at least 40% or more;
preferably 60% or more; and more preferably 70% or more. Alternatively, more preferable is identity of 90% or more, or 93% or more, or 95% or more, furthermore, 97% or more, or 99% or more. The identity can be determined by using the BLAST search algorithm.

With the gene amplification technique (PCR) (Current Protocols in Molecular Biology, edit, Ausubel et al., (1987) John Wiley & Sons, Section 6.3-6.4)) using primers designed based on the nucleotide sequence (Tables 350 and 351) or a portion thereof identified in the present Example, it is possible to isolate a DNA fragment highly homologous to the polynucleotide sequence or a portion thereof and to obtain functionally equivalent protein to a particular protein identified in the present Example based on the isolated DNA fragment.

The "percent identity" of two amino acid sequences or of two nucleic acids is determined using the algorithm of Karlin and Altschul (Proc. Natl. Acad. Sei. USA 87:2264-2268, 1990), modified as in Karlin and Altschul (Proc. Natl. Acad. Sei. USA 90:5873-5877, 1993). Such an algorithm is incorporated into the BLASTN and BLASTX programs of Altschul et al. (J. Mol. Biol.215:403-410, 1990). BLAST nucleotide searches are performed with the BLASTN program, score = 100, wordlength = 12. BLAST protein searches are performed with the BLASTX program, score = 50, wordlength = 3. When gaps exist between two sequences, Gapped BLAST is utilized as described in Altschul et al. (Nucleic Acids Res.25:3389-3402,1997). When utilizing BLAST and Gapped BLAST programs, the default parameters of the respective programs (e.g., BLASTX and BLASTN) are used. See http://www.ncbi.nlm.nih.gov.

The present invention also includes a partial peptide of the proteins of the invention. The partial peptide comprises a protein generated as a result that a signal peptide has been removed from a secretory protein. If the protein of the present invention has an activity as a receptor or a ligand, the partial peptide may function as a competitive inhibitor of the protein and may bind to the receptor (or ligand). In addition, the present invention comprises an antigen peptide for raising antibodies. For the peptides to be specific for the protein of the invention, the peptides comprise at least 7 amino acids, preferably 8 amino acids or more, more preferably 9 amino acids or more, and even more preferably 10 amino acids or more. The peptide can be used for preparing antibodies against the protein of the invention, or competitive inhibitors of them, and also screening for a receptor that binds to the protein of the invention. The partial peptides of the invention can be produced, for example, by genetic engineering methods, known methods for synthesizing peptides, or digesting the protein of the invention with an appropriate peptidase.

The present invention also relates to a vector into which the DNA of the invention is inserted. The vector of the invention is not limited as long as it contains the inserted DNA stably. For example, if *E. coli* is used as a host, vectors such as pBluescript vector (Stratagene) are preferable as a cloning vector. To produce the protein of the invention, expression vectors are especially useful. Any expression vector can be used as far as it is capable of expressing the protein *in vitro,* in *E. coli,* in cultured cells, or *in vivo.* For example, pBEST vector (Promega) is preferable for *in vitro* expression, pET vector (Invitrogen) for *E. coli,* pME18S-FL3 vector (GenBank Accession No. AB009864) for cultured cells, and pME18S vector (Mol. Cell. Biol. (1988) 8: 466-472) for *in vivo* expression. To insert the DNA of the invention, ligation utilizing restriction sites can be performed according to the standard method (Current Protocols in Molecular Biology (1987) Ausubel et al. edit, John Wily & Sons, Section 11.4-11.11).

The present invention also relates to a transformant carrying the vector of the invention. Any cell can be used as a host into which the vector of the invention is inserted, and various kinds of host cells can be used depending on the purposes. For strong expression of the protein in eukaryotic cells, COS cells or CHO cells can be used, for example.

Introduction of the vector into host cells can be performed, for example, by calcium phosphate precipitation method, electroporation method (Current Protocols in Molecular Biology (1987) Ausubel et al. edit, John Wily & Sons, Section 9.1-9.9), lipofectamine method (GIBCO-BRL), or microinjection method, etc.

The primer of the present invention can be used for synthesizing full-length cDNA, and also for the detection and/or diagnosis of the abnormality of the protein of the invention encoded by the full-length cDNA. For example, by utilizing polymerase chain reaction (genomic DNA-PCR, or RT-PCR) using the primer of the invention, DNA encoding the protein of the invention can be amplified. It is also possible to obtain the regulatory region of expression in the 5'-upstream by using PCR or hybridization since the transcription start site within the genomic sequence can be easily specified based on the 5'-end sequence of the full-length cDNA. The obtained genomic region can be used for detection and/or diagnosis of the abnormality of the sequence by RFLP analysis, SSCP, or direct sequencing.

Furthermore, the "polynucleotide having a length of at least 15 nucleotides, comprising a nucleotide sequence that is complementary to a polynucleotide comprising the nucleotide sequence set forth in any one of SEQ ID NOs in Tables 350 and 351, or its complementary strand" includes an antisense polynucleotide for suppressing the expression of the protein of the invention. To exert the antisense effect, the antisense polynucleotide has a length of at least 15 bp or more, for example, 50 bp or more, preferably 100 bp or more, and more preferably 500 bp or more, and has a length of usually 3000 bp or less and preferably 2000 bp or less. The antisense DNA can be used in the gene therapy of the diseases that are caused by the abnormality of the protein of the invention (abnormal function or abnormal expression). Said antisense DNA can be prepared, for example, by the phosphorothioate method ("Physicochemical properties of phosphorothioate oligodeoxynucleotides." Stein (1988) Nucleic Acids Res. 16: 3209-3221) based on the nucleotide sequence of the DNA encoding the protein (for example, the DNA set forth in any one of SEQ ID NOs in Tables 350 and 351).

The polynucleotide or antisense DNA of the present invention can be used in gene therapy, for example, by administrating it into a patient by the in vivo or ex vivo method with virus vectors such as retrovirus vectors, adenovirus vectors, and adeno-associated virus vectors, or non-virus vectors such as liposome.

The present invention also relates to antibodies that bind to the protein of the invention.
There are no limitations in the form of the antibodies of the invention. They include polyclonal antibodies, monoclonal antibodies, or their portions that can bind to the protein of the invention. They also include antibodies of all classes. Furthermore, special antibodies such as humanized antibodies are also included.

The polyclonal antibody of the invention can be obtained according to the standard method by synthesizing an oligopeptide corresponding to the amino acid sequence and immunizing rabbits with the peptides (Current Protocols in Molecular Biology (1987) Ausubel et al. edit, John Wily & Sons, Section 11.12-11.13). The monoclonal antibody of the invention can be obtained according to the standard method by purifying the protein expressed in *E. coli,* immunizing mice with the protein, and producing a hybridoma cell by fusing the spleen cells and myeloma cells (Current Protocols in Molecular Biology (1987) Ausubel et al. edit, John Wily & Sons, Section 11.4-11.11).

The antibody binding to the protein of the present invention can be used for purification of the protein of the invention, and also for detection and/or diagnosis of the abnormalities of the expression and structure of the protein. Specifically, proteins can be extracted, for example, from tissues, blood, or cells, and the protein of the invention is detected by Western blotting, immunoprecipitation, or ELISA, etc. for the above purpose.

Furthermore, the antibody binding to the protein of the present invention can be utilized for treating the diseases that associates with the protein of the invention. If the antibodies are used for treating patients, human antibodies or humanized antibodies are preferable in terms of their low antigenicity. The human antibodies can be prepared by immunizing a mouse whose immune system is replaced with that of human ("Functional transplant of megabase human immunoglobulin loci recapitulates human antibody response in mice" Mendez M.J. et al. (1997) Nat. Genet. 15: 146-156). The humanized antibodies can be prepared by recombination of the hypervariable region of a monoclonal antibody (Methods in Enzymology (1991) 203: 99-121).

The cDNA of the present invention encodes the amino acid sequence of a protein which is predicted to have the function(s) described below based on the homology search of the GenBank and SwissProt. Specifically, for instance, as shown in EXAMPLES, searching a known gene or protein that is homologous to the partial sequence of the full-length cDNA of the invention (5602 clone) and referring the function of the gene and of the protein encoded by the gene make it possible to predict the function of the protein encoded by the cDNA of the invention. In this way, each of 1437 clones out of the 5602 full-length cDNA clones of the invention was predicted to encode a protein that was classified into one or more of the following categories.
Secretory or membrane protein (261 clones)
Glycoprotein-associated protein (113 clones)
Signal transduction-associated protein (148 clones)
Transcription-associated protein (233 clones)
Disease-associated protein (437 clones)
Enzyme or metabolism-associated protein (301 clones)
Cell division- or cell proliferation-associated protein (74 clones)
Cytoskeleton-associated protein (92 clones)
RNA synthesis-associated protein (280 clones)
Nuclear protein (352 clones)
Protein synthesis- or transport-associated protein (112 clones)
Cellular defense-associated protein (23 clones)
Development- or growth-associated protein (23 clones)

It is also possible to predict the protein function by looking into the amino acid sequence for the motifs such as the signal sequence, transmembrane region, nuclear translocation signal, glycosylation signal, phosphorylation site, Zinc finger motif, and SH3 domain. The programs, PSORT (Nakai K., and Kanehisa M. (1992) Genomics 14: 897-911), SOSUI (Hirokawa T. et al. (1998) Bioinformatics 14: 378-379) (Mitsui Information Developing Inc.), and MEMSAT (Jones D.T., Taylor W.R., and Thornton J.M. (1994) Biochemistry 33: 3038-3049) can be used to predict the existence of the signal sequence or transmembrane region. Alternatively, a partial amino acid sequence of the protein is fused with another protein such as GFP, the fusion protein is transfected into cultured cells, and the localization is analyzed to predict the function of the original protein.

Based on the determined nucleotide sequences of the full-length cDNAs obtained in the present invention, it is possible to predict more detailed functions of the proteins encoded by the cDNA clones, for example, by searching the databases such as GenBank, Swiss-Prot and UniGene for homologies of the cDNAs; or by searching the amino acid sequences deduced from the full-length cDNAs for signal sequences by using software programs such as PSORT, for transmembrane regions by using software programs such as SOSUI or for motifs by using software programs such as Pfam (http://www.sanger.ac.uk/Software/Pfam/index.shtm1) and PROSITE (http://www.expasy.ch/prosite/). As a matter of course, the functions are often predictable by using partial sequence information (preferably 300 nucleotides or more) instead of the full-length nucleotide sequences. However, the result of the prediction by using partial nucleotide sequence does not always agree with the result obtained by using full-length nucleotide sequence, and thus, it is needless to say that the prediction of function is preferably performed based on the full-length nucleotide sequences.
GenBank, Swiss-Prot and UniGene databases were searched for homologies of the full-length nucleotide sequences of the 4997 clones (see Example 18). The amino acid sequences deduced from the full-length nucleotide sequences were searched for functional domains by PSORT, SOSUI and Pfam. Prediction of functions of proteins encoded by the clones and the categorization thereof were performed based on these results obtained.
The following 798 clones were categorized into secretory and/or membrane proteins.
HEMBA1000356, HEMBA1000518, HEMBA1000531, HEMBA1000637, HEMBA1000719, HEMBA1000817, HEMBA1000822, HEMBA1000852, HEMBA1000870, HEMBA1000991, HEMBA1001052, HEMBA1001071, HEMBA1001085, HEMBA1001286, HEMBA1001351, HEMBA1001407, HEMBA1001446, HEMBA1001515, HEMBA1001557, HEMBA1001569, HEMBA1001661, HEMBA1001734, HEMBA1001746, HEMBA1001866, HEMBA1002125, HEMBA1002150, HEMBA1002166, HEMBA1002417, HEMBA1002462, HEMBA1002475, HEMBA1002477, HEMBA1002486, HEMBA1002609, HEMBA1002659, HEMBA1002661, HEMBA1002780, HEMBA1002818, HEMBA1002876, HEMBA1002921, HEMBA1003071, HEMBA1003077, HEMBA1003079, HEMBA1003086, HEMBA1003096, HEMBA1003281, HEMBA1003286, HEMBA1003538, HEMBA1003711, HEMBA1003742, HEMBA1003803, HEMBA1004055, HEMBA1004143, HEMBA1004146, HEMBA1004207, HEMBA1004341, HEMBA1004461, HEMBA1004577, HEMBA1004637, HEMBA1004752, HEMBA1004756, HEMBA1004850, HEMBA1004889, HEMBA1004923, HEMBA1004930, HEMBA1005029, HEMBA1005035, HEMBA1005050, HEMBA1005552, HEMBA1005576, HEMBA1005581, HEMBA1005588, HEMBA1005616, HEMBA1005699, HEMBA1005991, HEMBA1006036, HEMBA1006038, HEMBA1006067, HEMBA1006173, HEMBA1006198, HEMBA1006293, HEMBA1006310, HEMBA1006492, HEMBA1006502, HEMBA1006583, HEMBA1006659, HEMBA1006758, HEMBA1006789, HEMBA1006921, HEMBA1006926, HEMBA1006976, HEMBA1007203, HEMBA1007301, HEMBB1000037, HEMBB1000050, HEMBB1000054, HEMBB1000175, HEMBB1000317, HEMBB1000556, HEMBB1000593, HEMBB1000631, HEMBB1000763, HEMBB1000827, HEMBB1000915, HEMBB1000975, HEMBB1001112, HEMBB1001151, HEMBB1001177, HEMBB1001302, HEMBB1001348, HEMBB1001564, HEMBB1001630, HEMBB1001871, HEMBB1001872, HEMBB1001925, HEMBB1001962, HEMBB1002042, HEMBB1002044, HEMBB1002142, HEMBB1002190, HEMBB1002193, HEMBB1002247, HEMBB1002383, HEMBB1002387, HEMBB1002550, HEMBB1002600, HEMBB1002692, MAMMA1000045, MAMMA1000129, MAMMA1000133, MAMMA1000277, MAMMA1000278, MAMMA1000410, MAMMA1000416, MAMMA1000472, MAMMA1000672, MAMMA1000684, MAMMA1000714, MAMMA1000734, MAMMA1000778, MAMMA1000798, MAMMA1000842, MAMMA1000859, MAMMA1000897, MAMMA1000956, MAMMA1001008, MAMMA1001030, MAMMA1001041, MAMMA1001073, MAMMA1001080, MAMMA1001139, MAMMA1001154, MAMMA1001322, MAMMA1001388, MAMMA1001411, MAMMA1001487, MAMMA1001751, MAMMA1001754, MAMMA1001771, MAMMA1002009, MAMMA1002427, MAMMA1002428, MAMMA1002461, MAMMA1002524, MAMMA1002573, MAMMA1002598, MAMMA1002655, MAMMA1002684, MAMMA1002769, MAMMA1002844, MAMMA1002881, MAMMA1002890, MAMMA1002938, MAMMA1002947, MAMMA1003035, MAMMA1003089, MAMMA1003146, MAMMA1003150, NT2RM1000035, NT2RM1000037, NT2RM1000062, NT2RM1000080, NT2RM1000092, NT2RM1000131, NT2RM1000199, NT2RM1000257, NT2RM1000260, NT2RM1000355, NT2RM1000430, NT2RM1000563, NT2RM1000648, NT2RM1000742, NT2RM1000770, NT2RM1000800, NT2RM1000811, NT2RM1000833, NT2RM1000857, NT2RM1000867, NT2RM1000882, NT2RM1000905, NT2RM1001008,
NT2RM1001115, NT2RM1001139, NT2RM2000259, NT2RM2000260, NT2RM2000287, NT2RM2000395, NT2RM2000402, NT2RM2000407, NT2RM2000422, NT2RM2000490, NT2RM2000522, NT2RM2000566, NT2RM2000581, NT2RM2000609, NT2RM2000821, NT2RM2001370, NT2RM2001393, NT2RM2001499, NT2RM2001547, NT2RM2001613, NT2RM2001648, NT2RM2001659, NT2RM2001671, NT2RM2001688, NT2RM2001698, NT2RM2001718, NT2RM2001753, NT2RM2001760, NT2RM2001785, NT2RM2001930, NT2RM2001950, NT2RM2001997, NT2RM2001998, NT2RM2002049, NT2RM2002145, NT2RM4000233, NT2RM4000433, NT2RM4000457, NT2RM4000486, NT2RM4000496, NT2RM4000520, NT2RM4000634, NT2RM4000674, NT2RM4000700, NT2RM4000764, NT2RM4000778, NT2RM4000795, NT2RM4000820, NT2RM4000857, NT2RM4001032, NT2RM4001054, NT2RM4001116, NT2RM4001455, NT2RM4001666, NT2RM4001810, NT2RM4001813, NT2RM4001930, NT2RM4001987, NT2RM4002054, NT2RM4002073, NT2RM4002145, NT2RM4002146, NT2RM4002189, NT2RM4002194, NT2RM4002251, NT2RM4002339, NT2RM4002438, NT2RM4002446, NT2RM4002452, NT2RM4002460, NT2RM4002493, NT2RM4002558, NT2RM4002565, NT2RM4002571, NT2RM4002594, NT2RP1000130, NT2RP1000191, NT2RP1000326, NT2RP1000358, NT2RP1000413, NT2RP1000418, NT2RP1000547, NT2RP1000609, NT2RP1000677, NT2RP1000767, NT2RP1000782, NT2RP1000856, NT2RP1001113, NT2RP1001247, NT2RP1001286, NT2RP1001310, NT2RP1001311, NT2RP1001313, NT2RP1001385, NT2RP1001449, NT2RP1001546, NT2RP1001569, NT2RP2000032, NT2RP2000040, NT2RP2000056, NT2RP2000070, NT2RP2000091, NT2RP2000114, NT2RP2000120, NT2RP2000173, NT2RP2000175, NT2RP2000195, NT2RP2000257, NT2RP2000270, NT2RP2000283, NT2RP2000288, NT2RP2000289, NT2RP2000459, NT2RP2000516, NT2RP2000660, NT2RP2000842, NT2RP2000892, NT2RP2001081, NT2RP2001268, NT2RP2001295, NT2RP2001366, NT2RP2001378, NT2RP2001576, NT2RP2001581, NT2RP2001597, NT2RP2001613, NT2RP2001947, NT2RP2001991, NT2RP2002025, NT2RP2002066, NT2RP2002078, NT2RP2002105, NT2RP2002312, NT2RP2002325, NT2RP2002385, NT2RP2002479, NT2RP2002537, NT2RP2002643, NT2RP2002701, NT2RP2002740, NT2RP2002857, NT2RP2003125, NT2RP2003297, NT2RP2003433, NT2RP2003446, NT2RP2003466, NT2RP2003506, NT2RP2003513, NT2RP2003629, NT2RP2003668, NT2RP2003760, NT2RP2003777, NT2RP2003781, NT2RP2004041, NT2RP2004142, NT2RP2004194, NT2RP2004270, NT2RP2004300, NT2RP2004392, NT2RP2004655, NT2RP2004681, NT2RP2004775, NT2RP2004799, NT2RP2004936, NT2RP2004959, NT2RP2005012, NT2RP2005159, NT2RP2005227, NT2RP2005270, NT2RP2005344, NT2RP2005465, NT2RP2005509, NT2RP2005752, NT2RP2005781, NT2RP2005784, NT2RP2005812, NT2RP2006069, NT2RP2006100, NT2RP2006141, NT2RP2006184, NT2RP2006261, NT2RP2006565, NT2RP2006571, NT2RP2006573, NT2RP3000092, NT2RP3000109, NT2RP3000134, NT2RP3000207, NT2RP3000333, NT2RP3000341, NT2RP3000393, NT2RP3000439, NT2RP3000441, NT2RP3000531, NT2RP3000685, NT2RP3000825, NT2RP3000826, NT2RP3000852, NT2RP3000919, NT2RP3001084, NT2RP3001096, NT2RP3001126, NT2RP3001140, NT2RP3001176, NT2RP3001260, NT2RP3001282, NT2RP3001355, NT2RP3001383, NT2RP3001426, NT2RP3001453, NT2RP3001497, NT2RP3001538, NT2RP3001589, NT2RP3001642, NT2RP3001708, NT2RP3001716, NT2RP3001727, NT2RP3001739, NT2RP3001799, NT2RP3001943, NT2RP3001944, NT2RP3002002, NT2RP3002007, NT2RP3002014, NT2RP3002054, NT2RP3002108, NT2RP3002163, NT2RP3002351, NT2RP3002455, NT2RP3002549, NT2RP3002602, NT2RP3002628, NT2RP3002650, NT2RP3002687, NT2RP3002701, NT2RP3002810, NT2RP3002869, NT2RP3002969, NT2RP3002985, NT2RP3003008, NT2RP3003059, NT2RP3003071, NT2RP3003101, NT2RP3003145, NT2RP3003197, NT2RP3003203, NT2RP3003242, NT2RP3003302, NT2RP3003353, NT2RP3003409, NT2RP3003576, NT2RP3003621, NT2RP3003665, NT2RP3003672, NT2RP3003701, NT2RP3003716, NT2RP3003799, NT2RP3003828, NT2RP3003914, NT2RP3003918, NT2RP3003992, NT2RP3004051, NT2RP3004148, NT2RP3004155, NT2RP3004207, NT2RP3004282, NT2RP3004454, NT2RP3004480, NT2RP3004503, NT2RP4000008, NT2RP4000051, NT2RP4000151, NT2RP4000212, NT2RP4000243, NT2RP4000259, NT2RP4000323, NT2RP4000417, NT2RP4000500, NT2RP4000524, NT2RP4000556, NT2RP4000560, NT2RP4000588, NT2RP4000713, NT2RP4000724, NT2RP4000817, NT2RP4000833, NT2RP4000878, NT2RP4000907, NT2RP4000925, NT2RP4000928, NT2RP4000973, NT2RP4000989, NT2RP4001057, NT2RP4001064, NT2RP4001079, NT2RP4001117, NT2RP4001138, NT2RP4001149, NT2RP4001150, NT2RP4001174, NT2RP4001219, NT2RP4001274, NT2RP4001313, NT2RP4001345, NT2RP4001372, NT2RP4001373, NT2RP4001379, NT2RP4001498, NT2RP4001547, NT2RP4001571, NT2RP4001574, NT2RP4001644, NT2RP4001656, NT2RP4001677, NT2RP4001730, NT2RP4001739, NT2RP4001803, NT2RP4001822, NT2RP4001823, NT2RP4001950, NT2RP4001975, NT2RP4002052, NT2RP4002075, NT2RP5003500, NT2RP5003506, NT2RP5003522, NT2RP5003534, OVARC1000060, OVARC1000335, OVARC1000682, OVARC1000689, OVARC1000700, OVARC1000722, OVARC1000751, OVARC1000850, OVARC1000890, OVARC1000924, OVARC1000936, OVARC1000959, OVARC1000984, OVARC1000999, OVARC1001034, OVARC1001055, OVARC1001117, OVARC1001129, OVARC1001154, OVARC1001329, OVARC1001381, OVARC1001391, OVARC1001453, OVARC1001476, OVARC1001506, OVARC1001610, OVARC1001702, OVARC1001703, OVARC1001713, OVARC1001745, OVARC1001767, OVARC1002127, OVARC1002138, OVARC1002158, OVARC1002165, PLACE1000014, PLACE1000213, PLACE1000401, PLACE1000562, PLACE1000611, PLACE1000656, PLACE1000712, PLACE1000793, PLACE1000909, PLACE1000948, PLACE1000977, PLACE1001241, PLACE1001257, PLACE1001377, PLACE1001517, PLACE1001610, PLACE1001761, PLACE1001771, PLACE1001817, PLACE1001983, PLACE1002046, PLACE1002140, PLACE1002213, PLACE1002395, PLACE1002437, PLACE1002500, PLACE1002583, PLACE1002714, PLACE1002722, PLACE1002782, PLACE1002794, PLACE1002851, PLACE1002908, PLACE1003030, PLACE1003044, PLACE1003045, PLACE1003238, PLACE1003296, PLACE1003369, PLACE1003420, PLACE1003493, PLACE1003537, PLACE1003553, PLACE1003596, PLACE1003760, PLACE1003768, PLACE1003771, PLACE1003903, PLACE1004149, PLACE1004197, PLACE1004203, PLACE1004258, PLACE1004270, PLACE1004277, PLACE1004289, PLACE1004473, PLACE1004629, PLACE1004646, PLACE1004743, PLACE1004751, PLACE1004793, PLACE1004840, PLACE1004969, PLACE1005086, PLACE1005162, PLACE1005206, PLACE1005313, PLACE1005467, PLACE1005530, PLACE1005595, PLACE1005611, PLACE1005623, PLACE1005763, PLACE1005884, PLACE1005890, PLACE1005898, PLACE1005934, PLACE1005953, PLACE1006157, PLACE1006225, PLACE1006239, PLACE1006288, PLACE1006492, PLACE1006534, PLACE1006678, PLACE1006754, PLACE1006901, PLACE1006935, PLACE1006956, PLACE1007111, PLACE1007243, PLACE1007274, PLACE1007282, PLACE1007317, PLACE1007375, PLACE1007386, PLACE1007409, PLACE1007416, PLACE1007484, PLACE1007583, PLACE1007632, PLACE1007645, PLACE1007649, PLACE1007852, PLACE1007877, PLACE1007954, PLACE1008273, PLACE1008309, PLACE1008331, PLACE1008402, PLACE1008424, PLACE1008429, PLACE1008531, PLACE1008532, PLACE1008533, PLACE1008568, PLACE1008643, PLACE1008693, PLACE1008715, PLACE1009045, PLACE1009094, PLACE1009298, PLACE1009319, PLACE1009338, PLACE1009368, PLACE1009493, PLACE1009639, PLACE1009659, PLACE1009708, PLACE1009731, PLACE1009845, PLACE1009861, PLACE1009935, PLACE1009992, PLACE1010089, PLACE1010231, PLACE1010321, PLACE1010362, PLACE1010599, PLACE1010622, PLACE1010662, PLACE1010811, PLACE1010917, PLACE1010942, PLACE1010954, PLACE1011090, PLACE1011214, PLACE1011221, PLACE1011371, PLACE1011399, PLACE1011492, PLACE1011646, PLACE1011749, PLACE1011896, PLACE2000034, PLACE2000062, PLACE2000111, PLACE2000132, PLACE2000176, PLACE2000187, PLACE2000216, PLACE2000335, PLACE2000341, PLACE2000373, PLACE2000379, PLACE2000398, PLACE2000399, PLACE2000425, PLACE2000438, PLACE2000458, PLACE2000477, PLACE3000020, PLACE3000218, PLACE3000226, PLACE3000242, PLACE3000244, PLACE3000339, PLACE3000373, PLACE3000399, PLACE3000406, PLACE3000413, PLACE3000455, PLACE4000052, PLACE4000063, PLACE4000129, PLACE4000247, PLACE4000250, PLACE4000259, PLACE4000300, PLACE4000387, PLACE4000431, PLACE4000487, PLACE4000494, PLACE4000522, PLACE4000548, PLACE4000581, PLACE4000593, PLACE4000650, THYRO1000156, THYRO1000327, THYRO1000394, THYRO1000395, THYRO1000570, THYRO1000748, THYRO1000756, THYRO1000783, THYRO1001134, THYRO1001271, THYRO1001287, THYRO1001320, THYRO1001401, THYRO1001534, THYRO1001537, THYRO1001541, THYRO1001828, Y79AA1000258, Y79AA1000420, Y79AA1000469, Y79AA1000734, Y79AA1000800, Y79AA1000976, Y79AA1001023, Y79AA1001177, Y79AA1001384, Y79AA1001394, Y79AA1001603, Y79AA1001647, Y79AA1001846, Y79AA1001874, Y79AA1002139, Y79AA1002246, Y79AA1002351, Y79AA1002399, Y79AA1002416, MAMMA1002498, NT2RM4002287

The following 142 clones were categorized into glycoprotein-associated proteins.
HEMBA1000156, HEMBA1000518, HEMBA1000852, HEMBA1001071, HEMBA1001286, HEMBA1001661, HEMBA1001734, HEMBA1001866, HEMBA1003071, HEMBA1003077, HEMBA1003281, HEMBA1003538, HEMBA1003679, HEMBA1003866, HEMBA1005576, HEMBA1005581, HEMBA1005699, HEMBA1006038, HEMBA1006976, HEMBA1007301, HEMBB1000317, HEMBB1000915, HEMBB1001871, HEMBB1001872, HEMBB1002193, MAMMA1000672, MAMMA1000897, MAMMA1001030, MAMMA1001388, MAMMA1002329, MAMMA1002428, MAMMA1002573, MAMMA1003150, NT2RM1000648, NT2RM1001115, NT2RM2000260, NT2RM2000407, NT2RM2000422, NT2RM2000490, NT2RM2001499, NT2RM2001659, NT2RM2001930, NT2RM4000820, NT2RM4000857, NT2RM4001810, NT2RM4001813, NT2RM4001987, NT2RM4002145, NT2RM4002189, NT2RM4002251, NT2RM4002460, NT2RM4002558, NT2RP1000677, NT2RP1000782, NT2RP1000856, NT2RP1001546, NT2RP2000056, NT2RP2000070, NT2RP2001295, NT2RP2001378, NT2RP2001597, NT2RP2001991, NT2RP2002025, NT2RP2002078, NT2RP2002385, NT2RP2004587, NT2RP2004732, NT2RP2005531, NT2RP3000207, NT2RP3000531, NT2RP3000825, NT2RP3001140, NT2RP3002810, NT2RP3003672, NT2RP3003701, NT2RP3003716, NT2RP3003914, NT2RP3004148,
NT2RP4000212, NT2RP4000417, NT2RP4000724, NT2RP4000817, NT2RP4000925, NT2RP4001150, NT2RP4001372, NT2RP4001730, NT2RP4001822, NT2RP4001823, NT2RP5003522, OVARC1000091, OVARC1000288, OVARC1000682, OVARC1001055, OVARC1001506, OVARC1001713, OVARC1002127, PLACE1000213, PLACE1000401, PLACE1002437, PLACE1002583,
PLACE1002722, PLACE1003045, PLACE1003238, PLACE1003258, PLACE1003493, PLACE1004197, PLACE1004793, PLACE1005953, PLACE1005955, PLACE1006157, PLACE1006239, PLACE1006368, PLACE1006534, PLACE1006754, PLACE1006956, PLACE1007416, PLACE1007632, PLACE1007649, PLACE1008643, PLACE1009094,
PLACE1009992, PLACE1010231, PLACE1010662, PLACE1011371, PLACE2000034, PLACE2000373, PLACE2000398, PLACE2000399, PLACE2000438, PLACE2000458, PLACE3000339, PLACE4000063, PLACE4000230, PLACE4000522, PLACE4000548, PLACE4000581, THYRO1000327, THYRO1000756, THYRO1001287, Y79AA1001603, Y79AA1001874, MAMMA1002498

The following 140 clones were categorized into signal transduction-associated proteins.
HEMBA1000303, HEMBA1000369, HEMBA1000608, HEMBA1000657, HEMBA1000919, HEMBA1001019, HEMBA1001174, HEMBA1001822, HEMBA1001921, HEMBA1002139, HEMBA1002212, HEMBA1002341, HEMBA1002417, HEMBA1002768, HEMBA1003250, HEMBA1003291, HEMBA1003645, HEMBA1004286, HEMBA1005737, HEMBA1006130, HEMBA1006708, HEMBB1000083, HEMBB1000266, HEMBB1000632, HEMBB1000781, HEMBB1000831, HEMBB1002193, MAMMA1000173, MAMMA1001038,
MAMMA1001198, MAMMA1002842, MAMMA1003057, NT2RM1000702, NT2RM1000772, NT2RM1001072, NT2RM2000030, NT2RM2000469, NT2RM2000612, NT2RM2001221, NT2RM2001345, NT2RM2002128, NT2RM4000229, NT2RM4000354, NT2RM4000611, NT2RM4000798, NT2RM4001411, NT2RM4001412, NT2RM4001629, NT2RM4001758, NT2RM4002013, NT2RM4002527, NT2RP1000018, NT2RP1000701, NT2RP1001294, NT2RP1001302, NT2RP2000668, NT2RP2001440, NT2RP2001560, NT2RP2002058, NT2RP2002193, NT2RP2002408, NT2RP2002710, NT2RP2002929, NT2RP2003164, NT2RP2003912, NT2RP2004232, NT2RP2004768, NT2RP2006071, NT2RP2006534, NT2RP3000759, NT2RP3000845, NT2RP3001646, NT2RP3001857, NT2RP3001938, NT2RP3002004, NT2RP3002785, NT2RP3002909, NT2RP3002988, NT2RP3003800, NT2RP3004189, NT2RP3004544, NT2RP4000147, NT2RP4000839, NT2RP4001122, NT2RP4001148, NT2RP4001336, NT2RP4001375, NT2RP4001644, NT2RP4001725, NT2RP4001849, NT2RP4001896, NT2RP4001927, NT2RP4002408, NT2RP5003477, OVARC1000013, OVARC1000437, OVARC1000556, OVARC1000649, OVARC1000945, OVARC1001200,
OVARC1002182, PLACE1000977, PLACE1001387, PLACE1002493, PLACE1002591, PLACE1003190, PLACE1003353, PLACE1004128, PLACE1004302, PLACE1004937, PLACE1005243, PLACE1008000, PLACE1008244, PLACE1008650, PLACE1009468, PLACE1009596, PLACE1009708, PLACE1009845, PLACE1010926, PLACE1011041, PLACE2000164, PLACE2000371, PLACE3000145, PLACE3000350, THYRO 1000072, THYRO1000748, THYRO1001120, Y79AA1000328, Y79AA1002431, HEMBA1001247, NT2RM2001813, NT2RM4001454, NT2RP2005140, NT2RP2005293, NT2RP3000487, NT2RP3003311, PLACE1000972, PLACE1003723, PLACE1005327, PLACE3000124,

The following 321 clones were categorized into transcription -associated proteins.
HEMBA1000158, HEMBA1000201, HEMBA1000216, HEMBA1000555, HEMBA1000561, HEMBA1000851, HEMBA1001077, HEMBA1001137, HEMBA1001405, HEMBA1001510, HEMBA1001635, HEMBA1001804, HEMBA1001809, HEMBA1001819, HEMBA1001847, HEMBA1001869, HEMBA1002035, HEMBA1002092, HEMBA1002177, HEMBA1002770, HEMBA1002935, HEMBA1003408, HEMBA1003545, HEMBA1003568, HEMBA1003662, HEMBA1003684, HEMBA1003760, HEMBA1003953, HEMBA1004097, HEMBA1004321, HEMBA1004353, HEMBA1004389, HEMBA1004479, HEMBA1004758, HEMBA1004973, HEMBA1005219, HEMBA1005359, HEMBA1005513, HEMBA1005528, HEMBA1005548, HEMBA1005558, HEMBA1005931, HEMBA1006158, HEMBA1006248, HEMBA1006278, HEMBA1006283, HEMBA1006347, HEMBA1006359, HEMBA1006559, HEMBA1006941, HEMBB1000789, HEMBB1001011, HEMBB1001314, HEMBB1001482, HEMBB1001673, HEMBB1001749, HEMBB1001839, HEMBB1001908, HEMBB1002134, HEMBB1002217, HEMBB1002342, HEMBB1002607, MAMMA1000183, MAMMA1000388, MAMMA1001105, MAMMA1001222, MAMMA1001260, MAMMA1001627, MAMMA1001633, MAMMA1001743, MAMMA1001820, MAMMA1001837, MAMMA1002617, MAMMA1002650, MAMMA1002937, NT2RM1000055, NT2RM1000086, NT2RM1000746, NT2RM1000885, NT2RM1000894, NT2RM1001092, NT2RM2000013, NT2RM2000452, NT2RM2000735, NT2RM2000740, NT2RM2001035, NT2RM2001105, NT2RM2001575, NT2RM2001670, NT2RM2001716, NT2RM2001771, NT2RM2002091, NT2RM4000024, NT2RM4000046, NT2RM4000104, NT2RM4000202, NT2RM4000531, NT2RM4000595, NT2RM4000733, NT2RM4000734,
NT2RM4000741, NT2RM4000751, NT2RM4000996, NT2RM4001092, NT2RM4001140, NT2RM4001200, NT2RM4001483, NT2RM4001592, NT2RM4001783, NT2RM4001823, NT2RM4001828, NT2RM4001858, NT2RM4001979, NT2RM4002066, NT2RP1000086, NT2RP1000111, NT2RP1000574, NT2RP1000902, NT2RP1001013, NT2RP2000008, NT2RP2000126, NT2RP2000297, NT2RP2000420, NT2RP2001174, NT2RP2001233, NT2RP2001756, NT2RP2001869, NT2RP2002046, NT2RP2002252, NT2RP2002270, NT2RP2002464, NT2RP2002503, NT2RP2002520, NT2RP2002591, NT2RP2002880, NT2RP2002939, NT2RP2002993, NT2RP2003243, NT2RP2003329, NT2RP2003347, NT2RP2003480, NT2RP2003522, NT2RP2003564, NT2RP2003714, NT2RP2004013, NT2RP2004066, NT2RP2004187, NT2RP2004920, NT2RP2004961, NT2RP2005003, NT2RP2005139, NT2RP2005325, NT2RP2005496, NT2RP2005701, NT2RP2005722, NT2RP2005776, NT2RP2005942, NT2RP2006238, NT2RP2006436, NT2RP3000050, NT2RP3000320, NT2RP3000512, NT2RP3000527, NT2RP3000590, NT2RP3000603, NT2RP3000605, NT2RP3000632, NT2RP3001057, NT2RP3001107, NT2RP3001111, NT2RP3001120, NT2RP3001150, NT2RP3001268, NT2RP3001338, NT2RP3001398, NT2RP3001527, NT2RP3001688, NT2RP3001855, NT2RP3002165, NT2RP3002399, NT2RP3002876, NT2RP3003133, NT2RP3003193, NT2RP3003251, NT2RP3003313, NT2RP3003327, NT2RP3003555, NT2RP3004016, NT2RP3004125, NT2RP3004242, NT2RP3004428, NT2RP3004498, NT2RP3004566, NT2RP3004617, NT2RP4000210, NT2RP4000398, NT2RP4000455, NT2RP4000648, NT2RP4000837, NT2RP4000865, NT2RP4000997, NT2RP4001029, NT2RP4001080, NT2RP4001213, NT2RP4001433, NT2RP4001529, NT2RP4001551, NT2RP4001568, NT2RP4001638, NT2RP4001753, NT2RP4001760, NT2RP4001790, NT2RP4001838, NT2RP4001938, NT2RP4002078, NT2RP4002081, NT2RP5003461, OVARC1000151, OVARC1000241, OVARC1000479, OVARC1001271, OVARC1001417, OVARC1001436, PLACE1000133, PLACE1000583, PLACE1000706, PLACE1000786, PLACE1000979, PLACE1001118, PLACE1001238, PLACE1001294, PLACE1001304, PLACE1001383, PLACE1001602, PLACE1001632, PLACE1002171, PLACE1002438, PLACE1002450, PLACE1002532, PLACE1002775, PLACE1002834, PLACE1003302, PLACE1003605, PLACE1003738, PLACE1003885, PLACE1004471, PLACE1005584, PLACE1005803, PLACE1005966, PLACE1006167, PLACE1006318, PLACE1006438, PLACE1006482, PLACE1007239, PLACE1007346, PLACE1007488, PLACE1007547, PLACE1007598, PLACE1007955, PLACE1008132, PLACE1008201, PLACE1009099, PLACE1009246, PLACE1009308, PLACE1009398, PLACE1009798, PLACE1010134, PLACE1010702, PLACE1010771, PLACE1010870, PLACE1011160, PLACE1011433, PLACE1011576, PLACE3000009, PLACE3000169, PLACE3000254, PLACE4000128, PLACE4000156, PLACE4000192, PLACE4000211, PLACE4000261, PLACE4000450, PLACE4000489, THYRO1000085, THYRO1000121, THYRO 1000242, THYRO 1000488, THYRO 1000501, THYRO 1000569, THYRO1001100, THYRO1001189, THYRO1001809, Y79AA1000013, Y79AA1000033, Y79AA1000037, Y79AA1000342, Y79AA1000627, Y79AA1000705, Y79AA1001299, Y79AA1001312, Y79AA1001391, Y79AA1001533, Y79AA1001613, Y79AA1001866, Y79AA1002103, Y79AA1002229, Y79AA1002433, Y79AA1002472, Y79AA1002482, HEMBA1003257, NT2RM2000101, NT2RM2001797, NT2RP1000101, NT2RP2002208, NT2RP3001214, NT2RP3003278, NT2RP4001235, PLACE1000050, PLACE1001716, PLACE1002499, PLACE1007544,

The following 392 clones were categorized into disease-associated proteins.
HEMBA1000020, HEMBA1000216, HEMBA1000304, HEMBA1000561, HEMBA1000569, HEMBA1000910, HEMBA1001043, HEMBA1001059, HEMBA1001071, HEMBA1001088, HEMBA1001569, HEMBA1001661, HEMBA1001672, HEMBA1001819, HEMBA1001921, HEMBA1002267, HEMBA1002419, HEMBA1002469, HEMBA1002547, HEMBA1002555, HEMBA1002810, HEMBA1002939, HEMBA1002997, HEMBA1003148, HEMBA1003369, HEMBA1003417, HEMBA1003418, HEMBA1003433, HEMBA1003538, HEMBA1003555, HEMBA1003568, HEMBA1003569, HEMBA1003581, HEMBA1004168, HEMBA1004202, HEMBA1004248, HEMBA1004275, HEMBA1004321, HEMBA1004353, HEMBA1004356, HEMBA1004479, HEMBA1004509, HEMBA1004669, HEMBA1005009, HEMBA1005338, HEMBA1005367, HEMBA1005423, HEMBA1005528, HEMBA1005581, HEMBA1005621, HEMBA1005699, HEMBA1006507, HEMBA1006650, HEMBA1006652, HEMBA1006737, HEMBA1006807, HEMBA1006877, HEMBA1007121, HEMBA1007243, HEMBB1000119, HEMBB1000693, HEMBB1000927, HEMBB1000985, HEMBB1001068, HEMBB1001282, HEMBB1001339, HEMBB1001482, HEMBB1001564, HEMBB1001802, HEMBB1001905, HEMBB1001908, HEMBB1002217, HEMBB1002477, MAMMA1000388, MAMMA1000731, MAMMA1001305, MAMMA1001633, MAMMA1001868, MAMMA1002170, MAMMA1002198, MAMMA1002268, MAMMA1002485, MAMMA1002530, MAMMA1002858, MAMMA1002869, MAMMA1002881, MAMMA1003047, MAMMA1003146, MAMMA1003166, NT2RM1000001, NT2RM1000153, NT2RM1000252, NT2RM1000555, NT2RM1000770, NT2RM1000826, NT2RM1000850, NT2RM1001003, NT2RM1001092, NT2RM1001102, NT2RM2000191,
NT2RM2000363, NT2RM2000594, NT2RM2000624, NT2RM2000714, NT2RM2000821, NT2RM2001035, NT2RM2001575, NT2RM2001652, NT2RM2001664, NT2RM2001668, NT2RM2001698, NT2RM2001803, NT2RM2001839, NT2RM4000155, NT2RM4000471, NT2RM4000486, NT2RM4000657, NT2RM4000751, NT2RM4000996, NT2RM4001629, NT2RM4001810, NT2RM4001819, NT2RM4001865, NT2RM4001876, NT2RM4001940, NT2RM4002066, NT2RM4002093, NT2RM4002146, NT2RM4002161, NT2RM4002323, NT2RM4002558, NT2RM4002571, NT2RP1000086, NT2RP1000574, NT2RP1000738, NT2RP1000825, NT2RP1000833, NT2RP1000959, NT2RP1000966, NT2RP1001013, NT2RP1001185, NT2RP1001482, NT2RP1001665, NT2RP2000070, NT2RP2000147, NT2RP2000224, NT2RP2000248, NT2RP2000297, NT2RP2000310, NT2RP2000414, NT2RP2000420, NT2RP2000523, NT2RP2000809, NT2RP2000812, NT2RP2001233, NT2RP2001327, NT2RP2001378, NT2RP2001394, NT2RP2001397, NT2RP2001460, NT2RP2001520, NT2RP2001536, NT2RP2001876, NT2RP2001898, NT2RP2002025, NT2RP2002058, NT2RP2002124, NT2RP2002325, NT2RP2002503, NT2RP2002959, NT2RP2003000, NT2RP2003157, NT2RP2003164, NT2RP2003228, NT2RP2003295, NT2RP2003517, NT2RP2003564, NT2RP2003604, NT2RP2003714, NT2RP2003737, NT2RP2003952, NT2RP2004013, NT2RP2004170, NT2RP2004587, NT2RP2004732, NT2RP2004933, NT2RP2005003, NT2RP2005144, NT2RP2005239, NT2RP2005276, NT2RP2005288, NT2RP2005315, NT2RP2005325, NT2RP2005336, NT2RP2005358, NT2RP2005407, NT2RP2005436, NT2RP2005476, NT2RP2005525, NT2RP2005694, NT2RP2005719, NT2RP2006043, NT2RP2006071, NT2RP2006219, NT2RP2006312, NT2RP2006456, NT2RP3000050, NT2RP3000068, NT2RP3000085, NT2RP3000299, NT2RP3000403, NT2RP3000596, NT2RP3000739, NT2RP3000753, NT2RP3000875, NT2RP3001057, NT2RP3001081, NT2RP3001216, NT2RP3001307, NT2RP3001338, NT2RP3001427, NT2RP3001428, NT2RP3001679, NT2RP3001723, NT2RP3001855, NT2RP3001898, NT2RP3001969, NT2RP3002056, NT2RP3002062, NT2RP3002151, NT2RP3002351, NT2RP3002399, NT2RP3002953, NT2RP3002988, NT2RP3003078, NT2RP3003251, NT2RP3003282, NT2RP3003313, NT2RP3003327, NT2RP3003409, NT2RP3003672, NT2RP3003831, NT2RP3004016, NT2RP3004078, NT2RP3004209, NT2RP3004258, NT2RP3004490, NT2RP3004534, NT2RP3004569, NT2RP3004572, NT2RP4000109, NT2RP4000367, NT2RP4000376, NT2RP4000449, NT2RP4000855, NT2RP4000879, NT2RP4000925, NT2RP4001086, NT2RP4001126, NT2RP4001150, NT2RP4001213, NT2RP4001276, NT2RP4001407, NT2RP4001433, NT2RP4001483, NT2RP4001575, NT2RP4001760, NT2RP4001861, NT2RP4002078, NT2RP4002791, OVARC1000014, OVARC1000139, OVARC1000520, OVARC1000722, OVARC1000771, OVARC1000834, OVARC1001051, OVARC1001113, OVARC1001244, OVARC1001372, OVARC1001417, OVARC1001496, OVARC1001506, OVARC1001577, OVARC1001726, OVARC1001766, OVARC1001809, OVARC1002165, PLACE1000133, PLACE1000383, PLACE1000420, PLACE1000583, PLACE1000588, PLACE1001171, PLACE1001387, PLACE1001602, PLACE1002046, PLACE1002140, PLACE1002437, PLACE1002474, PLACE1002685, PLACE1002782, PLACE1002834, PLACE1002908, PLACE1003045, PLACE1003302, PLACE1003353, PLACE1003366, PLACE1003493, PLACE1003669, PLACE1003704, PLACE1003903, PLACE1003968, PLACE1004183, PLACE1004197, PLACE1004277, PLACE1004316, PLACE1004358, PLACE1004471, PLACE1004506, PLACE1004510, PLACE1004674, PLACE1004777, PLACE1004814, PLACE1005494, PLACE1006040, PLACE1006170, PLACE1006438, PLACE1006615, PLACE1007140, PLACE1007239, PLACE1007257, PLACE1007511, PLACE1007598, PLACE1008177, PLACE1008356, PLACE1008402, PLACE1008696, PLACE1009027, PLACE1009113, PLACE1009158, PLACE1009444, PLACE1009524, PLACE1010529, PLACE1010870, PLACE1010896, PLACE1011635, PLACE1011858, PLACE1011922, PLACE2000015, PLACE2000072, PLACE2000216, PLACE2000399, PLACE2000438, PLACE2000458, PLACE3000242, PLACE4000009, PLACE4000014, PLACE4000156, PLACE4000369, SKNMC1000046, SKNMC1000050, THYRO1000034, THYRO1000327, THYRO1000343, THYRO1000358, THYRO1000501, THYRO1000662, THYRO1000684, THYRO1000748, THYRO1000934, THYRO1001120, THYRO1001189, THYRO1001204, THYRO1001458, THYRO1001617, THYRO1001671, Y79AA1000346, Y79AA1000469, Y79AA1000560, Y79AA1000734, Y79AA1000782, Y79AA1001391, Y79AA1001548, Y79AA1001594, Y79AA1001711, Y79AA1001874, Y79AA1002204, Y79AA1002210, Y79AA1002258, Y79AA1002472, Y79AA1002482,

The following 427 clones presumably belong to enzymes and/or metabolism-associated proteins. HEMBA1000012, HEMBA1000129, HEMBA1000141, HEMBA1000150, HEMBA1000542, HEMBA1000852, HEMBA1001019, HEMBA1001257, HEMBA1001526, HEMBA1001620, HEMBA1001866, HEMBA1001896, HEMBA1002212, HEMBA1002513, HEMBA1002746, HEMBA1002973, HEMBA1003046, HEMBA1003136, HEMBA1003179, HEMBA1003250, HEMBA1003291, HEMBA1003408, HEMBA1003538, HEMBA1003679, HEMBA1003680, HEMBA1004199, HEMBA1004227, HEMBA1004408, HEMBA1004509, HEMBA1004734, HEMBA1004768, HEMBA1005394, HEMBA1005513, HEMBA1005737, HEMBA1005815, HEMBA1006031, HEMBA1006272, HEMBA1006278, HEMBA1006291, HEMBA1006309, HEMBA1006347, HEMBA1006485, HEMBA1006521, HEMBA1006624, HEMBA1006885, HEMBA1006976, HEMBA1007121, HEMBA1007224, HEMBA1007243, HEMBA1007300, HEMBB1000083, HEMBB1000217, HEMBB1000915, HEMBB1000947, HEMBB1001137, HEMBB1001346, HEMBB1001429, HEMBB1001443, HEMBB1001915, HEMBB1001950, HEMBB1002042, MAMMA1000020, MAMMA1000085, MAMMA1000672, MAMMA1000713, MAMMA1000841, MAMMA1000897, MAMMA1001008, MAMMA1001038, MAMMA1001059, MAMMA1001476, MAMMA1001501, MAMMA1002268, MAMMA1002470, MAMMA1002530, MAMMA1002573, MAMMA1002619, MAMMA1002655, MAMMA1002671, MAMMA1003013, MAMMA1003035, NT2RM1000039, NT2RM1000132, NT2RM1000153, NT2RM1000256, NT2RM1000280, NT2RM1000377, NT2RM1000553, NT2RM1000648, NT2RM1000702, NT2RM1000894, NT2RM1001072, NT2RM1001115, NT2RM2000013, NT2RM2000092, NT2RM2000322, NT2RM2000368, NT2RM2000371, NT2RM2000469, NT2RM2000504, NT2RM2000577, NT2RM2000594, NT2RM2000951, NT2RM2001238, NT2RM2001547, NT2RM2001632, NT2RM2001664, NT2RM2001698, NT2RM2001700, NT2RM2001730, NT2RM2001782, NT2RM2001803, NT2RM2001886, NT2RM2001935, NT2RM2001997, NT2RM2002030, NT2RM2002128, NT2RM4000024, NT2RM4000155, NT2RM4000344, NT2RM4000471, NT2RM4000616, NT2RM4000657, NT2RM4000712, NT2RM4000820, NT2RM4001313, NT2RM4001316, NT2RM4001444, NT2RM4001592, NT2RM4001758, NT2RM4001819, NT2RM4001880, NT2RM4002062, NT2RM4002063, NT2RM4002189, NT2RM4002213, NT2RM4002251, NT2RM4002409, NT2RM4002532, NT2RM4002623, NT2RP1000376, NT2RP1000443, NT2RP1000522, NT2RP1000834, NT2RP1000947, NT2RP1001079, NT2RP1001185, NT2RP1001253, NT2RP1001361, NT2RP1001543, NT2RP2000056, NT2RP2000114, NT2RP2000183, NT2RP2000248, NT2RP2000329, NT2RP2000422, NT2RP2000448, NT2RP2000668, NT2RP2000710, NT2RP2000816, NT2RP2001070, NT2RP2001392, NT2RP2001601, NT2RP2001663, NT2RP2001740, NT2RP2001748, NT2RP2001898, NT2RP2002124, NT2RP2002256, NT2RP2002609, NT2RP2002618, NT2RP2002959, NT2RP2002993, NT2RP2003230, NT2RP2003286, NT2RP2003401, NT2RP2003506, NT2RP2003543, NT2RP2003643, NT2RP2003702, NT2RP2003704, NT2RP2003713, NT2RP2003737, NT2RP2003840, NT2RP2003912, NT2RP2003952, NT2RP2004098, NT2RP2004239, NT2RP2004245, NT2RP2004768, NT2RP2004791, NT2RP2004799, NT2RP2004933, NT2RP2005038, NT2RP2005139, NT2RP2005162, NT2RP2005204, NT2RP2005239, NT2RP2005276, NT2RP2005344, NT2RP2005360, NT2RP2005457, NT2RP2005498, NT2RP2005549, NT2RP2005557, NT2RP2005605, NT2RP2005635, NT2RP2005723, NT2RP2005773, NT2RP2005775, NT2RP2005776, NT2RP2005784, NT2RP2005835, NT2RP2005942, NT2RP2006534, NT2RP2006571, NT2RP2006573, NT2RP3000031, NT2RP3000085, NT2RP3000207, NT2RP3000359, NT2RP3000578, NT2RP3000742, NT2RP3000845, NT2RP3000875, NT2RP3000917, NT2RP3001055, NT2RP3001221, NT2RP3001495, NT2RP3001898, NT2RP3001938, NT2RP3002303, NT2RP3002351, NT2RP3002501, NT2RP3002602, NT2RP3002628, NT2RP3002663, NT2RP3003301, NT2RP3003385, NT2RP3003490, NT2RP3003659, NT2RP3003825, NT2RP3003831, NT2RP3003846, NT2RP3003914, NT2RP3004148, NT2RP3004209, NT2RP3004378, NT2RP3004669, NT2RP3004670, NT2RP4000259, NT2RP4000312, NT2RP4000367, NT2RP4000417, NT2RP4000457, NT2RP4000657, NT2RP4000817, NT2RP4000855, NT2RP4000879, NT2RP4000927, NT2RP4000973, NT2RP4000997, NT2RP4001041, NT2RP4001079, NT2RP4001095, NT2RP4001143, NT2RP4001219, NT2RP4001375, NT2RP4001389, NT2RP4001483, NT2RP4001555, NT2RP4001592, NT2RP4001644, NT2RP4001730, NT2RP4001946, NT2RP4002408, NT2RP5003500, NT2RP5003522, OVARC1000013, OVARC1000060, OVARC1000139, OVARC1000288, OVARC1000309, OVARC1000473, OVARC1000556, OVARC1000682, OVARC1000722, OVARC1000751, OVARC1000885, OVARC1000915, OVARC1001107, OVARC1001713, OVARC1001762, OVARC1001809, OVARC1001942, OVARC1002156, OVARC1002165, PLACE1000007, PLACE1000142, PLACE1000185, PLACE1000213, PLACE1000383, PLACE1000420, PLACE1000547, PLACE1000653, PLACE1000755, PLACE1001054, PLACE1001062, PLACE1001672, PLACE1001692, PLACE1001748, PLACE1001781, PLACE1001817, PLACE1001869, PLACE1001989, PLACE1002073, PLACE1002598, PLACE1002908, PLACE1002991, PLACE1003174, PLACE1003176, PLACE1003709, PLACE1003885, PLACE1003888, PLACE1003903, PLACE1003915, PLACE1004270, PLACE1004428, PLACE1004437, PLACE1004751, PLACE1004804, PLACE1004918, PLACE1005243, PLACE1005305, PLACE1005373, PLACE1005656, PLACE1005763, PLACE1005804, PLACE1005953, PLACE1005955, PLACE1006011, PLACE1006469, PLACE1006534, PLACE1006626, PLACE1006731, PLACE1006819, PLACE1006829, PLACE1006878, PLACE1007226, PLACE1007416, PLACE1007649, PLACE1007706, PLACE1007729, PLACE1007954, PLACE1007958, PLACE1008111, PLACE1008275, PLACE1008330, PLACE1008643, PLACE1009094, PLACE1009130, PLACE1009444, PLACE1009763, PLACE1009861, PLACE1009992, PLACE1009997, PLACE1010096, PLACE1010362, PLACE1010481, PLACE1010662, PLACE1011046, PLACE1011219, PLACE1011229, PLACE1011332, PLACE1011635, PLACE1011923, PLACE2000021, PLACE2000034, PLACE2000398, PLACE2000404, PLACE2000438, PLACE3000009, PLACE3000020, PLACE3000059, PLACE3000147, PLACE3000339, PLACE3000350, PLACE4000063, PLACE4000100, PLACE4000401, PLACE4000548, PLACE4000654, SKNMC1000050, THYRO1000072, THYRO1000197, THYRO1000288, THYRO1000605, THYRO1000662, THYRO1000756, THYRO1000852, THYRO1000926, THYRO1000934, THYRO1000951, THYRO1000983, THYRO1001003, THYRO1001287, THYRO1001374, THYRO1001406, THYRO1001617, THYRO1001671, THYRO1001738, Y79AA1000782, Y79AA1001048, Y79AA1001233, Y79AA1001394, Y79AA1001493, Y79AA1001548, Y79AA1001581, Y79AA1001603, Y79AA1001827, Y79AA1002027, Y79AA1002209, Y79AA1002211, Y79AA1002361, Y79AA1002416, HEMBA1005732, MAMMA1000402,

The following 217 clones presumably belong to ATP- and/or GTP-binding proteins.
HEMBA1000012, HEMBA1000129, HEMBA1000185, HEMBA1000491, HEMBA1000531, HEMBA1001019, HEMBA1001174, HEMBA1001387, HEMBA1001595, HEMBA1001723, HEMBA1001913, HEMBA1002161, HEMBA1002212, HEMBA1002876, HEMBA1002997, HEMBA1003250, HEMBA1003291, HEMBA1003369, HEMBA1003555, HEMBA1003560, HEMBA1004131, HEMBA1004199, HEMBA1004202, HEMBA1004354, HEMBA1004697, HEMBA1005047, HEMBA1005595, HEMBA1007018, HEMBA1007151, HEMBB1000083, HEMBB1000226, HEMBB1000264, HEMBB1000632, HEMBB1000725, HEMBB1001294, HEMBB1002193, MAMMA1000085, MAMMA1000612, MAMMA1000731, MAMMA1000738, MAMMA1001038, MAMMA1001735, MAMMA1001768, MAMMA1003127, NT2RM1000187, NT2RM1000388, NT2RM1000702, NT2RM1000772, NT2RM1000924, NT2RM2000469, NT2RM2000577, NT2RM2000740, NT2RM2001100, NT2RM2001201, NT2RM2001345, NT2RM2001823, NT2RM2002128, NT2RM4000155, NT2RM4000191, NT2RM4000356, NT2RM4000496, NT2RM4000611, NT2RM4000733, NT2RM4000820, NT2RM4001084, NT2RM4001178, NT2RM4001344, NT2RM4001444, NT2RM4001592, NT2RM4001714, NT2RM4001758, NT2RM4001880, NT2RM4002062, NT2RM4002174, NT2RM4002205, NT2RM4002527, NT2RM4002594, NT2RM4002623, NT2RP1000470, NT2RP1000478, NT2RP1000915, NT2RP1000958, NT2RP1001080, NT2RP1001410, NT2RP1001569, NT2RP2000126, NT2RP2000258, NT2RP2000329, NT2RP2000660, NT2RP2000668, NT2RP2000710, NT2RP2000812, NT2RP2000880, NT2RP2001245, NT2RP2001392, NT2RP2002606, NT2RP2003277, NT2RP2003912, NT2RP2004538, NT2RP2004568,
NT2RP2004689, NT2RP2004768, NT2RP2004791, NT2RP2004920, NT2RP2005344, NT2RP2005393, NT2RP2005763, NT2RP2006534, NT2RP3000046, NT2RP3000252, NT2RP3000350, NT2RP3000359, NT2RP3000366, NT2RP3000397, NT2RP3000759, NT2RP3000845, NT2RP3000875, NT2RP3001150, NT2RP3001427, NT2RP3001453, NT2RP3001529, NT2RP3001730, NT2RP3001799, NT2RP3001857, NT2RP3001938, NT2RP3002007, NT2RP3002151, NT2RP3002330, NT2RP3002399, NT2RP3002671, NT2RP3003301, NT2RP3003353, NT2RP3003589, NT2RP3003809, NT2RP3003876, NT2RP3004189, NT2RP3004428, NT2RP3004578, NT2RP4000290, NT2RP4000481, NT2RP4000518, NT2RP4000781, NT2RP4000839, NT2RP4000929, NT2RP4001041, NT2RP4001079, NT2RP4001375, NT2RP4001414, NT2RP4001592, NT2RP4001634, NT2RP4001644, NT2RP4001656, NT2RP4001896, NT2RP4002047, NT2RP4002058, NT2RP4002408, NT2RP5003477, OVARC1000013, OVARC1000304, OVARC1000556, OVARC1000771, OVARC1000800, OVARC1001068, OVARC1002138, PLACE1000040, PLACE1000588, PLACE1001104, PLACE1001739, PLACE1002433, PLACE1002437, PLACE1002714, PLACE1003394, PLACE1003521, PLACE1003915, PLACE1004902, PLACE1005243, PLACE1005305, PLACE1005549, PLACE1005739, PLACE1005921, PLACE1006119, PLACE1006196, PLACE1006552, PLACE1006956, PLACE1007409, PLACE1007697, PLACE1007946, PLACE1008244, PLACE1009404, PLACE1009476, PLACE1009596, PLACE1009908, PLACE1010134, PLACE1010720, PLACE1010896, PLACE1011109, PLACE1011114, PLACE1011310, PLACE1011922, PLACE2000014, PLACE2000039, PLACE2000274, PLACE2000404, PLACE2000427, PLACE3000350, PLACE4000009, PLACE4000014, PLACE4000326, SKNMC1000013, THYRO1000072, THYRO1001458, Y79AA1000833, Y79AA1000962, Y79AA1001394, Y79AA1001875, Y79AA1001963, Y79AA1002209,

The following 320 clones presumably belong to nuclear proteins.
HEMBA1000005, HEMBA1000158, HEMBA1000216, HEMBA1000561, HEMBA1000591, HEMBA1001088, HEMBA1001137, HEMBA1001405, HEMBA1001510, HEMBA1001579, HEMBA1001809, HEMBA1001819, HEMBA1001824, HEMBA1001847, HEMBA1001869, HEMBA1002177, HEMBA1002241, HEMBA1002495, HEMBA1002569, HEMBA1002935, HEMBA1002951, HEMBA1002999, HEMBA1003408, HEMBA1003545, HEMBA1003662, HEMBA1003684, HEMBA1003690, HEMBA1003760, HEMBA1004203, HEMBA1004321, HEMBA1004353, HEMBA1004479, HEMBA1004973, HEMBA1005219, HEMBA1005359, HEMBA1005558, HEMBA1005931, HEMBA1006278, HEMBA1006283, HEMBA1006359, HEMBA1006485, HEMBA1007087, HEMBB1000226, HEMBB1000789, HEMBB1001011, HEMBB1001056, HEMBB1001242, HEMBB1001482, HEMBB1001915, HEMBB1002134, HEMBB1002217, MAMMA1000183, MAMMA1000731, MAMMA1001105, MAMMA1001222, MAMMA1001260, MAMMA1001633, MAMMA1001743, MAMMA1001837, MAMMA1002617, MAMMA1002869, MAMMA1002937, MAMMA1003011, NT2RM1000086, NT2RM1000187, NT2RM1000666, NT2RM1000885, NT2RM1000894, NT2RM1001059, NT2RM1001092, NT2RM2000013, NT2RM2000588, NT2RM2000624, NT2RM2000735, NT2RM2000740, NT2RM2001105, NT2RM2001635, NT2RM2001670, NT2RM2001771, NT2RM2001823, NT2RM2001936, NT2RM2001989, NT2RM2002004, NT2RM2002088, NT2RM2002091, NT2RM4000024, NT2RM4000046, NT2RM4000104, NT2RM4000202, NT2RM4000215, NT2RM4000290, NT2RM4000531, NT2RM4000751, NT2RM4000996, NT2RM4001092, NT2RM4001140, NT2RM4001200, NT2RM4001483, NT2RM4001566, NT2RM4001592,
NT2RM4001597, NT2RM4001783, NT2RM4001823, NT2RM4001828, NT2RM4001858, NT2RM4001979, NT2RP1000035, NT2RP1000111, NT2RP1000493, NT2RP1000574, NT2RP1000630, NT2RP1000902, NT2RP1000915, NT2RP1000958, NT2RP1000966, NT2RP1001013, NT2RP1001177, NT2RP2000008, NT2RP2000076, NT2RP2000126, NT2RP2000153, NT2RP2000161, NT2RP2000248, NT2RP2000258, NT2RP2000297, NT2RP2000420, NT2RP2000931, NT2RP2001233, NT2RP2001420, NT2RP2001756, NT2RP2001869, NT2RP2002079, NT2RP2002270, NT2RP2002503, NT2RP2002591, NT2RP2002880, NT2RP2002939, NT2RP2002993, NT2RP2003137, NT2RP2003157, NT2RP2003277, NT2RP2003286, NT2RP2003308, NT2RP2003347, NT2RP2003714, NT2RP2003912, NT2RP2004013, NT2RP2004187, NT2RP2004689, NT2RP2004920, NT2RP2005393, NT2RP2005436, NT2RP2005496, NT2RP2005539, NT2RP2005701, NT2RP2005767, NT2RP2005776, NT2RP2005933, NT2RP2005942, NT2RP2006043, NT2RP2006436, NT2RP3000031, NT2RP3000050, NT2RP3000397, NT2RP3000512, NT2RP3000527, NT2RP3000590, NT2RP3000603, NT2RP3000632, NT2RP3000917, NT2RP3001057, NT2RP3001107, NT2RP3001120, NT2RP3001253, NT2RP3001338, NT2RP3001384, NT2RP3001398, NT2RP3001427, NT2RP3001428, NT2RP3001472, NT2RP3001646, NT2RP3001671, NT2RP3001792, NT2RP3001855, NT2RP3002056, NT2RP3002165, NT2RP3002399, NT2RP3002876, NT2RP3003193, NT2RP3003212, NT2RP3003555, NT2RP3004016, NT2RP3004206, NT2RP3004424, NT2RP3004428, NT2RP3004566, NT2RP3004617, NT2RP4000078, NT2RP4000111, NT2RP4000210, NT2RP4000398, NT2RP4000481, NT2RP4000518, NT2RP4000997, NT2RP4001148, NT2RP4001206, NT2RP4001213, NT2RP4001433, NT2RP4001568, NT2RP4001638, NT2RP4001696, NT2RP4001753, NT2RP4001938, NT2RP4002058, NT2RP4002078, NT2RP4002081, NT2RP4002791, OVARC1000006, OVARC1000087, OVARC1000091, OVARC1000241, OVARC1000326, OVARC1000556, OVARC1000846, OVARC1001038, OVARC1001180, OVARC1001232, OVARC1001271, OVARC1001306, OVARC1001436, OVARC1002112, PLACE1000133, PLACE1000184, PLACE1000406, PLACE1000583, PLACE1000596, PLACE1000979, PLACE1001118, PLACE1001383, PLACE1001632, PLACE1002171, PLACE1002433, PLACE1002438, PLACE1002532, PLACE1002775, PLACE1002816, PLACE1002834, PLACE1003100, PLACE1003190, PLACE1003302, PLACE1003519, PLACE1003521, PLACE1003605, PLACE1003704, PLACE1003738, PLACE1003885, PLACE1003923, PLACE1004302, PLACE1004471, PLACE1004564, PLACE1004814, PLACE1004902, PLACE1005287, PLACE1005876, PLACE1005966, PLACE1006167, PLACE1006438, PLACE1006482, PLACE1006829, PLACE1006878, PLACE1006917, PLACE1007014, PLACE1007547, PLACE1007598, PLACE1007688, PLACE1007969, PLACE1008044, PLACE1008132, PLACE1008603, PLACE1009099, PLACE1009130, PLACE1009308, PLACE1009398, PLACE1010134, PLACE1010194, PLACE1010702, PLACE1010720, PLACE1010870, PLACE1011056, PLACE1011433, PLACE1011664, PLACE2000014, PLACE2000427, PLACE3000009, PLACE3000169, PLACE4000014, PLACE4000156, PLACE4000192, PLACE4000261, PLACE4000326, PLACE4000489, SKNMC1000011, THYRO1000085, THYRO1000242, THYRO1000585, THYRO1001100, THYRO1001189, THYRO1001809, Y79AA1000037, Y79AA1000214, Y79AA1000231, Y79AA1000589, Y79AA1000752, Y79AA1001391, Y79AA1001613, Y79AA1001705, Y79AA1001963, Y79AA1002431, Y79AA1002472, Y79AA1002482,

The following 296 clones presumably belong to DNA- and/or RNA-binding proteins. HEMBA1000158, HEMBA1000216, HEMBA1000561, HEMBA1000591, HEMBA1000851, HEMBA1001088, HEMBA1001137, HEMBA1001405, HEMBA1001510, HEMBA1001804, HEMBA1001809, HEMBA1001819, HEMBA1001847, HEMBA1001869, HEMBA1002177, HEMBA1002935, HEMBA1003408, HEMBA1003545, HEMBA1003568, HEMBA1003591, HEMBA1003662, HEMBA1003684, HEMBA1003760, HEMBA1003783, HEMBA1003805, HEMBA1003953, HEMBA1004321, HEMBA1004354, HEMBA1004389, HEMBA1004479, HEMBA1004669, HEMBA1004847, HEMBA1004973, HEMBA1005202, HEMBA1005359, HEMBA1005931, HEMBA1006248, HEMBA1006278, HEMBA1006283, HEMBA1006359, HEMBA1006652, HEMBA1007087, HEMBA1007194, HEMBB1000264, HEMBB1000789, HEMBB1001011, HEMBB1001482, HEMBB1001736, HEMBB1001749, HEMBB1001839, HEMBB1002217, MAMMA1000183, MAMMA1000284, MAMMA1000731, MAMMA1001105, MAMMA1001222, MAMMA1001260, MAMMA1001743, MAMMA1001837, MAMMA1002385, MAMMA1002617, MAMMA1002869, MAMMA1002937, MAMMA1003011, NT2RM1000086, NT2RM1000539, NT2RM1000555, NT2RM1000666, NT2RM1000691, NT2RM1000826, NT2RM1000885, NT2RM1001059, NT2RM1001092, NT2RM2000371, NT2RM2000624, NT2RM2000735, NT2RM2001105, NT2RM2001424, NT2RM2001575, NT2RM2001605, NT2RM2001670, NT2RM2001771, NT2RM2001823, NT2RM2001989, NT2RM2002004, NT2RM2002014, NT2RM2002088, NT2RM2002091, NT2RM4000046, NT2RM4000104, NT2RM4000167, NT2RM4000191, NT2RM4000202, NT2RM4000531, NT2RM4000595, NT2RM4000733, NT2RM4000751, NT2RM4000996, NT2RM4001092, NT2RM4001140,
NT2RM4001178, NT2RM4001200, NT2RM4001483, NT2RM4001592, NT2RM4001783, NT2RM4001823, NT2RM4001828, NT2RM4001858, NT2RM4001880, NT2RM4001979, NT2RM4002093, NT2RM4002109, NT2RP1000470, NT2RP1000493, NT2RP1000574, NT2RP1000902, NT2RP1000966, NT2RP1001013, NT2RP1001073, NT2RP1001080, NT2RP2000008, NT2RP2000153, NT2RP2000258, NT2RP2000297, NT2RP2001127, NT2RP2001174, NT2RP2001233, NT2RP2001511, NT2RP2001756, NT2RP2001869, NT2RP2002079, NT2RP2002099, NT2RP2002503, NT2RP2002591, NT2RP2002939, NT2RP2003157, NT2RP2003329, NT2RP2003347, NT2RP2003480, NT2RP2003522, NT2RP2003564, NT2RP2003714, NT2RP2004187, NT2RP2004568, NT2RP2004920, NT2RP2005003, NT2RP2005139, NT2RP2005168, NT2RP2005436, NT2RP2005496, NT2RP2005701, NT2RP2005763, NT2RP2005776, NT2RP2005942, NT2RP2006043, NT2RP2006436, NT2RP2006464, NT2RP3000050, NT2RP3000512, NT2RP3000527, NT2RP3000562, NT2RP3000590, NT2RP3000603, NT2RP3000624, NT2RP3000632, NT2RP3000994, NT2RP3001057, NT2RP3001107, NT2RP3001120, NT2RP3001150, NT2RP3001155, NT2RP3001338, NT2RP3001398, NT2RP3001472, NT2RP3001672, NT2RP3001688, NT2RP3001724, NT2RP3001792, NT2RP3001855, NT2RP3002165, NT2RP3002399, NT2RP3002876, NT2RP3003138, NT2RP3003193, NT2RP3003251, NT2RP3003327, NT2RP3003555, NT2RP3004013, NT2RP3004078, NT2RP3004428, NT2RP3004490, NT2RP3004566, NT2RP3004594, NT2RP3004617, NT2RP3004618, NT2RP4000111, NT2RP4000398, NT2RP4000455, NT2RP4000518, NT2RP4000648, NT2RP4000865, NT2RP4000929, NT2RP4001080, NT2RP4001095, NT2RP4001213, NT2RP4001433, NT2RP4001568, NT2RP4001696, NT2RP4001753, NT2RP4001838, NT2RP4001938, NT2RP4002078, OVARC1000006, OVARC1000087, OVARC1000241, OVARC1000746, OVARC1000846, OVARC1001232, OVARC1001271, OVARC1001306, OVARC1001987, OVARC1002112, PLACE1000406, PLACE1000583, PLACE1000979, PLACE1001118, PLACE1001632, PLACE1001739, PLACE1002438, PLACE1002532, PLACE1002775, PLACE1002834, PLACE1003302, PLACE1003519, PLACE1003605, PLACE1003704, PLACE1003738, PLACE1003885, PLACE1004471, PLACE1004564, PLACE1004814, PLACE1005584, PLACE1005876, PLACE1005951, PLACE1006196, PLACE1006482, PLACE1006488, PLACE1006531, PLACE1006917, PLACE1007346, PLACE1007547, PLACE1007598, PLACE1007688, PLACE1007969, PLACE1008132, PLACE1009099, PLACE1009246, PLACE1009398, PLACE1009476, PLACE1009622, PLACE1010053, PLACE1010194, PLACE1010702, PLACE1010870, PLACE1011056, PLACE1011114, PLACE1011433, PLACE2000427, PLACE3000009, PLACE3000169, PLACE4000014, PLACE4000156, PLACE4000192, PLACE4000261, PLACE4000489, SKNMC1000091, THYRO 1000085, THYRO1000242, THYRO1000501, THYRO1001100, THYRO1001189, THYRO1001809, Y79AA1000037, Y79AA1000349, Y79AA1000752, Y79AA1001211, Y79AA1001312, Y79AA1001391, Y79AA1001613, Y79AA1002103, Y79AA1002472, Y79AA1002482, HEMBA1004596, OVARC1000148, PLACE1003334, THYRO1001661,

The following 66 clones presumably belong to the category of RNA synthesis-associated proteins.
HEMBA1000591, HEMBA1001579, HEMBA1003179, HEMBA1003591, HEMBA1006278, HEMBB1000226, NT2RM1000187, NT2RM1000852, NT2RM2000624, NT2RM2001989, NT2RM2002100, NT2RM4000191, NT2RM4001178, NT2RM4002093, NT2RP1000035, NT2RP1000272, NT2RP1000470, NT2RP1001080, NT2RP2000153, NT2RP2002928, NT2RP2003157, NT2RP2004568, NT2RP2005126, NT2RP2005436, NT2RP2005539, NT2RP2005605, NT2RP2005776, NT2RP2005942, NT2RP2006043, NT2RP2006238, NT2RP3000361, NT2RP3000397, NT2RP3001671, NT2RP3004504, NT2RP4000078, NT2RP4000111, NT2RP4000481, NT2RP4000518, NT2RP4000614, NT2RP4000929, NT2RP4001696, NT2RP4002058, OVARC1001232, OVARC1001577, PLACE1000406, PLACE1000596, PLACE1000755, PLACE1001739, PLACE1003704, PLACE1003885, PLACE1004564, PLACE1004814, PLACE1004902, PLACE1005373, PLACE1005646, PLACE1005876, PLACE1006196, PLACE1006626, PLACE1006878, PLACE1006917, PLACE1009476, PLACE1009925, PLACE1010194, PLACE1011114, THYRO1000121, Y79AA1001963,

The following 184 clones presumably belong to protein synthesis- and/or protein transport-associated proteins.
HEMBA1000012, HEMBA1000141, HEMBA1000592, HEMBA1003617, HEMBA1003773, HEMBA1004202, HEMBA1004276, HEMBA1004734, HEMBA1004847, HEMBA1004929, HEMBA1004930, HEMBA1005047, HEMBA1005202, HEMBA1006031, HEMBA1006272, HEMBA1006474, HEMBA1006652, HEMBA1006914, HEMBA1006973, HEMBA1007224, HEMBB1000915, HEMBB1001112, HEMBB1001137, HEMBB1001736, HEMBB1001831, HEMBB1001915, MAMMA1000085, MAMMA1000734, MAMMA1001008, MAMMA1002170, MAMMA1002219, MAMMA1002236, MAMMA1002619, NT2RM1000661, NT2RM1000833, NT2RM2000092, NT2RM2000504, NT2RM2000577, NT2RM2000821, NT2RM2001201, NT2RM2001592, NT2RM2001613, NT2RM2001648, NT2RM2001730, NT2RM2001760, NT2RM2002055, NT2RM4000155, NT2RM4000169, NT2RM4000344, NT2RM4000356, NT2RM4000421, NT2RM4000712, NT2RM4001054, NT2RM4001203, NT2RM4001382, NT2RM4001444, NT2RM4002062, NT2RM4002205, NT2RM4002623, NT2RP1000326, NT2RP1000522, NT2RP1000547, NT2RP1000746, NT2RP1000947, NT2RP1001569, NT2RP2000147, NT2RP2000710, NT2RP2000880, NT2RP2000943, NT2RP2001290, NT2RP2001392, NT2RP2001601, NT2RP2001613, NT2RP2001660, NT2RP2001740, NT2RP2002124, NT2RP2002606, NT2RP2002862, NT2RP2002959, NT2RP2002980, NT2RP2003137, NT2RP2003158, NT2RP2003391, NT2RP2003394, NT2RP2003401, NT2RP2003433, NT2RP2003704, NT2RP2003713, NT2RP2003737, NT2RP2003760, NT2RP2003981, NT2RP2004366, NT2RP2004389, NT2RP2004791, NT2RP2005012, NT2RP2005116, NT2RP2005360, NT2RP2005763, NT2RP2005784, NT2RP3000366,
NT2RP3000759, NT2RP3000968, NT2RP3001113, NT2RP3001690, NT2RP3002045, NT2RP3002151, NT2RP3002529, NT2RP3002671, NT2RP3003301, NT2RP3003846, NT2RP3003876, NT2RP3004209, NT2RP4000370, NT2RP4000457, NT2RP4000879, NT2RP4000927, NT2RP4001041, NT2RP4001117, NT2RP4001313, NT2RP4001315, NT2RP4001574, NT2RP4001592, OVARC1000013, OVARC1000071, OVARC1000085, OVARC1000465, OVARC1000564, OVARC1000771, OVARC1000862, OVARC1001171, OVARC1001180, OVARC1001342, PLACE1000007, PLACE1000061, PLACE1000081, PLACE1000492, PLACE1000863, PLACE1001092, PLACE1001748, PLACE1002090, PLACE1003174, PLACE1003915, PLACE1004104, PLACE1004270, PLACE1004743, PLACE1005557, PLACE1005813, PLACE1006170, PLACE1006488, PLACE1006829, PLACE1007706, PLACE1007729, PLACE1008273, PLACE1008402, PLACE1008790, PLACE1008813, PLACE1009094, PLACE1009130, PLACE1009477, PLACE1009721, PLACE1009845, PLACE1010074, PLACE1010547, PLACE1011109, PLACE1011229, PLACE1011477, PLACE1012031, PLACE2000404, PLACE3000059, PLACE3000121, PLACE4000269, PLACE4000654, SKNMC1000011, THYRO1000983, THYRO1001003, THYRO1001313, Y79AA1000560, Y79AA1000784, Y79AA1000968, Y79AA1001493, Y79AA1001875, Y79AA1002027, Y79AA1002209, HEMBA1006284,

The following 130 clones presumably belong to cytoskeleton-associated proteins.
HEMBA1000156, HEMBA1000168, HEMBA1000411, HEMBA1000588, HEMBA1001043, HEMBA1001651, HEMBA1001661, HEMBA1002102, HEMBA1002161, HEMBA1002939, HEMBA1003235, HEMBA1003581, HEMBA1004499, HEMBA1004534, HEMBA1004697, HEMBA1004929, HEMBA1004972, HEMBA1005582, HEMBA1005595, HEMBA1006344, HEMBA1006737, HEMBB1001175, HEMBB1001282, HEMBB1001562, HEMBB1001802, MAMMA1000824, MAMMA1001041, MAMMA1001576, MAMMA1001679, MAMMA1001735, MAMMA1002297, MAMMA1002351, MAMMA1002622, MAMMA1002637, MAMMA1003127, NT2RM1000850, NT2RM1000898, NT2RM2000030, NT2RM2000260, NT2RM2000691, NT2RM2001324, NT2RM4000169, NT2RM4000229, NT2RM4000515, NT2RM4001217, NT2RP1000202, NT2RP1000348, NT2RP1000460,
NT2RP1000478, NT2RP1001033, NT2RP1001294, NT2RP1001302, NT2RP2000070, NT2RP2000812, NT2RP2000814, NT2RP2001168, NT2RP2001245, NT2RP2001634, NT2RP2001900, NT2RP2003307, NT2RP2003394, NT2RP2004041, NT2RP2004242, NT2RP2004538, NT2RP2004587, NT2RP2004681, NT2RP2004732, NT2RP2004978, NT2RP2005491, NT2RP2005531, NT2RP2005712, NT2RP2006275, NT2RP3000753, NT2RP3001113, NT2RP3001216, NT2RP3001239, NT2RP3001272, NT2RP3001554, NT2RP3001690, NT2RP3001799, NT2RP3002688, NT2RP3003061, NT2RP3003185, NT2RP3003230, NT2RP3004569, NT2RP3004578, NT2RP4001004, NT2RP4001086, NT2RP4001256, NT2RP4001567, NT2RP4001927, OVARC1000001, OVARC1000106, OVARC1000437, OVARC1000520, OVARC1000679, OVARC1001731, OVARC1002050, PLACE1001104, PLACE1002571,
PLACE1002591, PLACE1002655, PLACE1002714, PLACE1003625, PLACE1005287, PLACE1006552, PLACE1007946, PLACE1008426, PLACE1010148, PLACE1010547, PLACE1010743, PLACE1010896, PLACE1010960, PLACE1011310, PLACE1011922, PLACE2000216, PLACE2000274, PLACE2000371, PLACE2000458, PLACE3000145, PLACE3000416, PLACE4000009, THYRO1000132, THYRO1001405, THYRO1001458, Y79AA1000368, Y79AA1000794, Y79AA1000833, Y79AA1000962, Y79AA1002208,

The following 54 clones presumably belong to cell division-associated and/or cell proliferation-associated proteins.
HEMBA1001019, HEMBA1001595, HEMBA1002363, HEMBA1002997, HEMBA1003136, HEMBA1003369, HEMBA1004131, HEMBA1004354, HEMBA1005621, HEMBB1000037, HEMBB1000264, MAMMA1001768, MAMMA1002769, NT2RM1000354, NT2RM1000430, NT2RM1000874, NT2RM2001256, NT2RM2001743, NT2RM2001896, NT2RM2002145, NT2RM4000215, NT2RM4001714, NT2RP1000163, NT2RP1000333, NT2RP1000439, NT2RP2000346, NT2RP2001397, NT2RP2002595, NT2RP2003177, NT2RP2003596, NT2RP2003912, NT2RP2004396, NT2RP2005037, NT2RP2005520, NT2RP2005669, NT2RP2005835, NT2RP3001730, NT2RP3002081, NT2RP4000210, NT2RP4000415, NT2RP4001414, NT2RP4001634, OVARC1000013, OVARC1000937, PLACE1001383, PLACE1002433, PLACE1004316, PLACE1005287, PLACE1008808, PLACE1010720, PLACE1010833, Y79AA1000748, Y79AA1001236, Y79AA1001394

The following 36 clones presumably belong to the category of embryogenesis- and/or development-associated proteins:
HEMBA1000518, HEMBA1001847, HEMBA1001869, HEMBA1003545, HEMBA1004973, HEMBB1002442, MAMMA1001837, NT2RM2001670, NT2RM4000046, NT2RM4000531, NT2RM4001140, NT2RM4001858, NT2RP2002078, NT2RP2004187, NT2RP2006436, NT2RP3000603, NT2RP3000994, NT2RP3001580, NT2RP3001708, NT2RP3003071, NT2RP3004472, NT2RP3004617, NT2RP4000246, NT2RP4001567, OVARC1000304, OVARC1000746, PLACE1000793, PLACE1002532, PLACE1003258, PLACE1003625, PLACE1004460, PLACE1009622, PLACE4000558, THYRO1000085, Y79AA1001391, Y79AA1001692

The following 30 clones presumably belong to cellular defense-associated proteins.
HEMBA1000005, HEMBA1000531, HEMBA1003417, HEMBA1006253, NT2RM4000354, NT2RM4001880, NT2RP1000333, NT2RP1000493, NT2RP2000006, NT2RP2000045, NT2RP2000809, NT2RP2001536, NT2RP2002464, NT2RP2004920, NT2RP2005037, NT2RP3000590, NT2RP3001426, NT2RP3002062, NT2RP3002785, NT2RP3004262, NT2RP4001555, NT2RP4001638, PLACE1006958, PLACE1008275, PLACE1009113, PLACE1011858, PLACE4000014, THYRO1000684, Y79AA1002139, Y79AA1002229

Although it is unclear whether or not 261 clones out of clones other than the above-mentioned clones belong to any of the above-described categories, these clones are predicted to have some functions, based on the homology search using their full-length sequences.
HEMBA1000030, HEMBA1000307, HEMBA1000333, HEMBA1000488, HEMBA1000523, HEMBA1001197, HEMBA1001302, HEMBA1001455, HEMBA1001675, HEMBA1001714, HEMBA1001744, HEMBA1001967, HEMBA1002151, HEMBA1002215, HEMBA1002458, HEMBA1002777, HEMBA1003098, HEMBA1003199, HEMBA1003615, HEMBA1003836, HEMBA1004295, HEMBA1004573, HEMBA1004604, HEMBA1004795, HEMBA1005101, HEMBA1005201, HEMBA1005206, HEMBA1005530, HEMBA1005666, HEMBA1005990, HEMBA1006268, HEMBA1006398, HEMBA1006445, HEMBA1007174, HEMBA1007251, HEMBB1000036, HEMBB1000144, HEMBB1000973, HEMBB1001058, HEMBB1001234, HEMBB1001288, HEMBB1001331, HEMBB1001384, HEMBB1002266, HEMBB1002510, HEMBB1002705, MAMMA1000055, MAMMA1000625, MAMMA1001075, MAMMA1001181, MAMMA1001259, MAMMA1001730, MAMMA1002143, MAMMA1002699, MAMMA1002972, MAMMA1003113, NT2RM1000118, NT2RM1000186, NT2RM1000244, NT2RM1000421, NT2RM1000499, NT2RM1000623, NT2RM1000883, NT2RM2000502, NT2RM2000599, NT2RM2000718, NT2RM2001065, NT2RM2001196, NT2RM2001983, NT2RM2002109, NT2RM2002142, NT2RM4000030, NT2RM4000139, NT2RM4000156, NT2RM4000386, NT2RM4000590, NT2RM4001047, NT2RM4001155, NT2RM4001256, NT2RM4001320, NT2RM4001340, NT2RM4001347, NT2RM4001371, NT2RM4001582, NT2RM4001611, NT2RM4001731, NT2RM4001969, NT2RM4002034, NT2RM4002075, NT2RM4002226, NT2RP1000040, NT2RP1000363, NT2RP1000481, NT2RP1000513, NT2RP1000733, NT2RP1000860, NT2RP1000954, NT2RP1001011, NT2RP1001395, NT2RP1001457,
NT2RP1001494, NT2RP2000054, NT2RP2000067, NT2RP2000133, NT2RP2000157, NT2RP2000764, NT2RP2000965, NT2RP2001839, NT2RP2001883, NT2RP2001976, NT2RP2001985, NT2RP2002185, NT2RP2002442, NT2RP2002727, NT2RP2002741, NT2RP2002986, NT2RP2003121, NT2RP2003265, NT2RP2003272, NT2RP2003857, NT2RP2003871, NT2RP2004425, NT2RP2004476, NT2RP2004710, NT2RP2004816, NT2RP2005441, NT2RP2005490, NT2RP2005620, NT2RP2005654, NT2RP2005675, NT2RP2005753, NT2RP2005841, NT2RP2006598, NT2RP3000047, NT2RP3000233, NT2RP3000868, NT2RP3000869, NT2RP3001399, NT2RP3001407, NT2RP3001457, NT2RP3001587, NT2RP3001712, NT2RP3001819, NT2RP3001854, NT2RP3001931, NT2RP3002273, NT2RP3002631, NT2RP3002682, NT2RP3002770, NT2RP3002818, NT2RP3002948, NT2RP3002972, NT2RP3003032, NT2RP3003290, NT2RP3003411, NT2RP3003491, NT2RP3003500, NT2RP3003726, NT2RP3004348, NT2RP3004507, NT2RP4000129, NT2RP4000498, NT2RP4000528, NT2RP4000737, NT2RP4000979, NT2RP4001010, NT2RP4001207, NT2RP4001228, NT2RP4001260, NT2RP4001339, NT2RP4001351, NT2RP4001474, NT2RP4001966, NT2RP4002018, OVARC1000209, OVARC1000876, OVARC1001065, OVARC1001092, OVARC1001419, OVARC1001555, OVARC1001711, OVARC1001943, PLACE1000004, PLACE1000066, PLACE1000610, PLACE1000636, PLACE1000769, PLACE1000987, PLACE1001036, PLACE1001845, PLACE1001920, PLACE1002665, PLACE1003602, PLACE1003611, PLACE1004256, PLACE1004550, PLACE1004868, PLACE1004930, PLACE1005052, PLACE1005102, PLACE1005176, PLACE1005187, PLACE1005331, PLACE1005727, PLACE1006003, PLACE1006335, PLACE1006385, PLACE1006506, PLACE1007105, PLACE1007537, PLACE1007705, PLACE1007791, PLACE1007897, PLACE1008080, PLACE1008368, PLACE1008398, PLACE1008465, PLACE1008627, PLACE1009020, PLACE1009060, PLACE1009186, PLACE1009443, PLACE1009571, PLACE1009670, PLACE1010105, PLACE1010261, PLACE1010310, PLACE1010522, PLACE1010579, PLACE1010628, PLACE1010661, PLACE1010761, PLACE1011185, PLACE1011340, PLACE1011586, PLACE2000246, PLACE2000411, PLACE3000477, THYRO1000173, THYRO1000401, THYRO1000666, THYRO1001033, THYRO1001347, THYRO1001656, THYRO1001703, THYRO1001721, Y79AA1000059, Y79AA1000181, Y79AA1000268, Y79AA1000313, Y79AA1000540, Y79AA1000966, Y79AA1000985, Y79AA1001323, Y79AA1001402, Y79AA1001679, Y79AA1001923, Y79AA1002083, Y79AA1002307, Y79AA1002311, Y79AA1002487,

In some cases, the predicted functions based on the partial sequences are different from those based on the full-length sequences. The reason is that a protein does not always belong solely to a single category of the above-described functional categories, and therefore, a protein may belong to two or more of the predicted functional categories. Besides, additional functions can be found for the clones classified into these functional categories by further analyses.

Since the protein encoded by clones of the invention contains full-length amino acid sequence, it is possible to analyze its biological activity, and its effect on cellular conditions such as cell proliferation and differentiation by expressing the protein as a recombinant protein using an appropriate expression system, injecting the recombinant into the cell, or raising a specific antibody against the protein.

If the protein is a secretory protein, membrane protein, or protein associated with glycoprotein, signal transduction, or transcription, its biological activity can be analyzed by the methods in "Gene Transcription" (Hames B.D., and Higgins S.J. edit, (1993)), "Glycobiology" (Fukuda M., and Kobata A. edit, (1993)), "Growth Factors" (McKay I., and Leigh I. edit, (1993)), "Extracellular Matrix" (Haralson M.A., and Hassell J.R. edit, (1995), "Transcription Factors" (Latchman D.S. edit, (1993)), "Signal Transduction" (Milligans G. edit, (1992)), "Protein Phosphorylation" (Hardies G.D. edit, (1993), and "Ion Channels" (Ashley R.H. edit, (1995) featured in "The Practical Approach Series" (IRL PRESS), or "Signal Transduction Protocols" (Kendall D.A., and Hill S.J. edit, (1995), "Glycoprotein Analysis in Biomedicine" (Hounsell E.F. edit, (1993)), featured in "Method in Molecular Biology" (Humana Press).

As to a protein associated with a disease, it is possible to perform a functional analysis as described above, but also possible to analyze correlation between the expression or the activity of the protein and a certain disease by using a specific antibody that is obtained by using expressed protein. Alternatively, it is possible to utilize the database Online Mendelian Inheritance in Man (OMIM) (http://www.ncbi.nlm.nih.gov/Omim/), which is a database of human genes and diseases, to analyze the protein.
New information is constantly being deposited in the OMIM database. Therefore, it is possible for one skilled in the art to find a new relationship between a particular disease and a gene of the present invention in the most up-to-date database.

Also, as for a secretory protein, membrane protein, signal transduction-associated protein, glycoprotein-associated protein, or transcription-associated protein, etc., search of the OMIM with the following keywords resulted in the finding that the proteins are associated with many diseases (the result of the OMIM search for secrete and membrane proteins is shown below). Also, association between proteins associated to signal transduction or transcription and diseases is reported in "Transcription Factor Research-1999" (Fujii, Tamura, Kageyama, and Satake edit, (1999) Jikken-Igaku Zoukan, Vol.17, No.3), and "Gene Medicine" ((1999) Vol.3, No.2). For example, in tumors, many proteins have been shown to play a role, including secretory proteins, membrane proteins, and proteins associated with signal transduction, glycoprotein, and transcription, and also proteins associated with metabolism, cytoskeleton, and cell cycle, as described in "Tumor Biology" (Matsubara S. (1992) Syoukabou Life Science series). Thus, besides the proteins associated with diseases, many proteins described above are also potentially associated with diseases, and thus useful as a target in the medicinal industry.

The result of the OMIM search for secretory and membrane proteins is shown below, in which the keywords,
(1) secretion protein,
(2) membrane protein,
(3) channel, and
(4) extracellular matrix were used.

Shown in the search result are only the accession numbers in the OMIM. Using the number, data showing the relationship between a disease and a gene or protein can be seen. The OMIM data has been renewed everyday.
1) Secretion protein
   268 entries found, searching for "secretion protein"
   104760, 176860, 160900, 107400, 118910, 139320, 603850, 147572,176880, 600946, 603215, 157147, 600174, 151675, 170280, 179512, 179513,138120, 179509, 246700, 179510, 600626, 179511, 600998, 109270, 601489, 154545, 179490, 185860, 603216, 122559, 601746,147290, 602672, 146770, 603062, 179508, 131230, 601591, 602421, 139250, 167805, 167770, 600041, 600564, 118825, 601146, 300090, 600753, 601652, 600759, 600768, 602434, 182590, 603166, 308230, 602534, 603489, 107470, 150390, 104610, 173120, 158106, 143890, 306900, 308700, 134797, 137350, 227500, 176300, 107730, 600760, 138079, 120180, 120160, 120150, 124092, 138160, 101000, 227600, 600509, 601199, 142410, 104311, 193400, 201910, 107300, 122560, 272800, 217000, 590050, 147670, 133170, 176730, 300300, 134370, 274600, 120140,162151, 158070, 152790, 120120, 106100, 300200, 192340, 190160, 138040, 147470, 147620, 173350, 147380, 152200, 152760, 157145, 153450, 264080, 113811, 600937, 600840, 188545, 202110, 600514, 186590, 603372, 136435, 137241, 252800, 214500, 207750, 138850, 139191, 142640, 138130, 189907, 603692, 600633, 603355, 107270, 600377, 147892, 232200, 600281, 232800, 602358, 137035, 601771, 601769, 253200, 601933, 118444, 600270, 120700, 600945, 603732, 147660, 600761, 172400, 600823, 600877, 130080, 171060, 107740, 307800, 602843, 130660, 152780, 124020, 601124, 601340, 601604, 601610, 171050, 312060, 232700, 300159, 142703, 600734, 125255, 168450, 123812, 188540, 147940, 188450, 600839, 182452, 188400, 182280, 176760, 263200, 600264, 188826, 252650, 601185, 162641, 137216, 601398, 601538, 118888, 118445, 601745, 190180, 601922, 182098, 602008, 147440, 602384, 600031, 109160, 602663, 151670, 602682, 602730, 602779, 146880, 603061, 142704, 603140, 106150, 600732, 153620, 603318, 139392, 600042, 102200, 603493, 182100, 264300, 603795, 184600
2) Membrane protein
   1017 entries found, searching for "membrane protein"
   130500, 305360, 153330, 173610, 170995, 109270, 170993, 309060, 120920, 602333, 133740, 133710, 602690, 133730, 159430, 600897, 133090, 601178, 602413, 602003, 109280, 603237, 602173, 107776, 602334, 125305, 602335, 182879, 154045, 309845, 600594, 603718, 603241, 603214, 603657, 603177, 600182, 601476, 602879, 136950, 600723, 601114, 185880, 185881, 300096, 602257, 160900, 177070, 603062, 603344, 602977, 310200, 600959, 300100, 186945, 600039, 600267, 128240, 182900, 601097, 136430, 600946, 602534, 601047, 143450, 603141, 603700, 600579, 256540, 159440, 602414, 600403, 602048, 188860, 137290, 158343, 184756, 602910, 603179, 600279, 108733, 107770, 173335, 602625, 154050, 219800, 603850, 601028, 600447, 104225, 186946, 601767, 603143, 121015, 603215, 227400, 603735, 600179, 602421, 180721,
   176801, 176860, 600753, 603142, 176790, 600266, 601239, 115501, 143890, 121014, 121011, 125950, 603534, 304040, 601134, 600754, 601510, 601595, 190315, 300172, 602216, 602261, 602262, 602461, 131560, 179514, 179512, 176981, 142461, 139310, 312080, 176640, 128239, 185470, 310300, 601403, 601757, 273800, 151460, 176943, 104311, 168468, 120130, 602887, 600164, 601531, 601832,104775, 600040, 603583, 176894, 602631, 166945, 182180, 120620, 141180, 601014, 139150, 182860, 177061, 600174, 180069, 191275, 104760, 601693, 300017, 603518, 601009, 134651, 601107, 603868, 600168, 136425, 603531, 603291, 600917, 603216, 102720, 300118, 179590, 135630, 602285, 107450, 602296, 303630, 176878, 120090, 600322, 138160, 601212, 603293, 131230, 112205, 600763, 600718, 300187, 170715, 601966, 300051, 602474,
   120070, 600691, 600855, 182309, 602101, 602857, 194355, 162230, 600874, 113730, 155550, 602701, 306400, 601789, 231200, 107271, 175100, 182870, 305100, 301000, 601313, 157147, 147670, 139200, 603593,157655, 600934, 155970, 602049, 155960, 155760, 118990, 135620, 308230, 602694, 162060, 300023, 160993, 153619, 153432, 120131, 603823, 603167, 601023, 600816, 165040, 601681,166490, 300112, 120190, 300145, 163970, 600772, 602926, 602933, 602202, 400015, 151510, 600759, 602672, 602654, 603821, 116952, 151430, 602632, 155975, 602217, 150370, 600752, 601179, 600932, 603048, 603234, 601805, 603822, 603869, 601717, 601181, 313440, 139130, 107777, 109190, 603452, 191163, 191164, 602370, 176877, 103195, 600523, 191328, 601275, 204200, 602426, 603820, 600551, 600695, 600552, 600553, 602306, 601523,
   602507, 602299, 600583, 114070, 600632, 603498, 185430, 600587, 235200, 173470, 603199, 601633, 602500, 208900, 180297, 156225, 516020, 190195, 141900, 102680, 193300, 101000, 193400, 300011, 107400, 257220,107741, 180380, 203200, 111700, 600024, 304800, 600065, 110750, 179605, 113705, 601638, 222900, 120120, 602509, 602469, 600930, 601383, 176261, 602574, 602997, 311770, 131550, 603616, 308700, 603372, 256100, 224100, 276903, 305900, 516000, 131195, 314555, 601567, 603866, 306900, 103390, 186720, 173850, 601050, 602505, 186590, 246530, 602689, 194380, 300041, 162643, 152790,120150, 600682, 600106, 272750, 188040, 602382, 601497, 113811, 182138, 212138, 601309, 109690, 114760, 176805, 601253, 123900, 602581, 189980,191190,110700, 600163, 137167, 600580, 601610, 190000, 123825, 603491,
   600135, 186591, 173910, 138140, 107266, 120950, 601081, 603690, 244400, 312700, 171060, 601199, 601758, 170500, 277900, 601997, 314850, 601880, 603009, 120220, 603126, 164920, 602934, 164730, 163890, 603434, 107269, 602909, 600877, 256550, 164761, 602872, 120110, 126150, 158070, 266200, 223360, 250800, 269920, 252650, 603355, 154582, 138190, 300035, 602640, 227650, 158120, 153700, 182380, 155740, 204500, 603401, 601975, 300135, 136350, 602924, 300167, 185050, 176100, 300189, 151525, 300200, 165180, 230800, 602158, 602676, 603411, 193245, 120325, 601848, 192500, 603102, 147795, 245900, 137060, 147557,120650, 602377, 307800, 120930, 308100, 142800, 191092, 232300, 173510, 602225, 180470, 190930, 186357, 134638, 600544, 601373, 600509, 600359, 603784, 600395, 600653, 603754, 601597, 601066,
   600185, 601295, 600978, 205400, 603274, 600418, 600839, 516050, 601691, 601007, 600650, 600308, 603261, 601193, 600004, 600017, 516040, 253800, 276901, 600019, 257200,108780, 300037, 300104, 300126, 255125, 203300, 300191, 426000, 302060, 304700, 201475, 252010, 193210, 311030, 306250, 248600, 191740, 108360, 131244, 600423, 232200, 191305, 231680, 103320, 190180, 600493, 111200, 226200, 312600, 600170, 111680, 186910, 203100, 600536, 600238,186830, 186760, 186745, 186711, 106180, 112203, 103180, 182530, 182160, 600644, 307030, 192321, 600667, 125647, 179080, 114207, 114860, 176000, 116930, 600748, 173515, 173325, 600377, 171760, 171050, 118425, 170260, 191315, 600798, 600821, 600823, 600444, 600840, 159465, 600857, 158380, 600867, 154360, 152427, 150330, 110900, 147840,147360, 147280,
   146880, 312610, 120940, 142871, 142790, 600937, 142600,134390,111250, 600979, 600997, 142460, 186845, 134635, 601017, 139191, 139090, 138850, 601040, 138720, 122561, 131100, 123610, 217070, 100500, 603377, 602354, 603302, 603207, 603086, 602188, 602095, 603867, 603842, 603798, 602602, 601194, 602607, 603713, 603681, 601252, 603648, 603646, 603644, 601282, 601284, 603667, 603712, 603594, 601872, 603425, 601843, 603263, 603208, 601411, 603201, 603189, 601463, 603164, 603152, 603087, 602874, 601492, 602893, 602057, 602859, 602746, 603879, 603510, 602458, 603380, 601581, 603765, 603283, 601599, 601733, 601852, 602316, 601615, 601617, 602184, 602894, 603005, 603030, 603861, 602835, 602136, 600153, 600074, 600046, 600023, 601625, 516006, 600018, 600016, 516002, 601590, 313475, 313470, 600244,
   600528, 601611, 600282, 600327, 601568, 600368, 601730, 601535, 601745, 601929, 300169, 300150, 300132, 601533, 600385, 600464, 600424, 600429, 601756, 601488, 516005, 251100, 516004, 600918, 516003, 602192, 516001, 240500, 600465, 602241, 602243, 230200, 601485, 601478, 601416, 602297, 601459, 601839, 602314, 193065, 193001, 191306, 600504, 601020, 191191, 602372, 190181, 600534, 188380, 186854, 186360, 600530, 185250, 182331, 600535, 182305, 601296, 600582, 600732, 600734, 600742, 600782, 176802, 176266, 600769, 601883, 600864, 601901, 176260, 173490, 600910, 601905, 171890, 600916, 601987, 602679, 162651, 161555, 160994, 602714, 602715, 602724, 602736, 300007, 602783, 275630, 602836, 270200, 602871, 159460, 602876, 154540, 153900, 602890, 601153, 602190, 602905, 153634, 153337, 602914,
   152310, 151690, 151625, 602935, 602974, 150325, 602992, 150320, 250790, 603006, 603007, 603008, 150292, 233690, 603046, 150210, 603061, 147940, 603063, 221770, 223100, 603097, 147880, 603118, 147730, 146928, 146630, 142622, 603149, 603150, 603151, 600923, 138981, 138590, 138330, 216950, 603192, 138297, 603202, 601002, 602343, 138230, 136131, 603217, 603220, 134660, 131390, 131235, 603242, 603243, 130130, 602345, 126455, 601123, 126064, 125240, 602359, 603312, 602380, 603318, 123890, 123836, 603356, 603361, 603366, 123830, 179610, 188060, 123620, 120980, 186355, 118510, 114835, 114217, 113810, 603499, 182310, 111740, 109610, 603548, 603564, 108740, 603598, 603613, 107273, 603626, 602518, 179410, 603647, 602515, 603652, 106195, 602573, 178990, 105210, 104615, 167055, 603717, 104614, 603728,
   104210, 603749, 603750, 103850, 602608, 603787, 603788, 603796, 173445, 103220, 102910, 102681, 102670, 102642, 603833, 173391, 102576,102575, 171833, 102573, 101800, 603875, 601108
3) Channel (member of membrane protein)
   272 entries found, searching for "channel"
   176266, 600724, 170500, 182390, 123825, 114208, 114205, 601784, 114206, 600937, 114204, 603415, 600053, 114209, 114207, 600760, 118425, 601011, 192500, 176261, 600761, 176260, 600359, 600228, 600877, 602235, 300008, 182389, 182391, 601328, 601534, 600504, 602323, 601958, 602780, 602781, 601327, 601012, 600734, 603208, 182392, 603220, 603219, 603888, 600054, 602232, 601745, 603537, 602604, 603796, 302910, 602866, 601013, 602905, 602906, 600163, 152427, 180901, 600702, 600308, 602754, 107776, 602024, 314555, 601949, 600235, 602023, 176263, 600681, 176265, 193245, 603305, 176258, 602983, 601219, 601141, 176267, 602343, 602726, 138253, 176262, 600003, 600397, 602872, 138249, 600843, 600935, 600580, 600845, 602158, 602106, 176264, 300110, 176257, 602717, 603493, 176268, 600932, 602727, 138254,
   603652, 300138, 602420, 600570, 600150, 603583, 602345, 603749, 601142, 176256, 600846, 138252, 602982, 603787, 602836, 603788, 602566, 603651, 602421, 100690, 107777, 100725, 100710, 600509, 603061, 154275, 304040, 154276, 180902, 121014, 602368, 139311, 601383, 108745, 601313, 601042, 600131, 186360, 600109, 600229, 600170, 603319, 601485, 118503, 180903, 602076, 124030, 601059, 601212, 601218, 147450, 600855, 600919, 601154, 601157, 171060, 600968, 182139, 131230, 121015, 600421, 113730, 249210, 310500, 600637, 125950, 118800, 156490, 602974, 104610, 121011, 602522, 118504, 300041, 160900, 601382, 602103, 600465, 602014, 600442, 601109, 602481, 277900, 254210, 138247, 164920, 170280, 171050, 128100, 173910, 600884, 123885, 602887, 600232, 180297, 137192, 600304, 138251, 603053, 300103,
   603152, 603199, 118511, 118508, 138079, 600983, 182307, 603324, 305990, 603418, 114080, 232200, 600046, 600040, 602403, 603750, 603785, 104210, 600019, 600300, 182860, 603852, 603853, 603855, 516060
4) Extracellular matrix
   167 entries found, searching for "extracellular matrix"
   603479, 602201, 601418, 601548, 154870, 115437, 602285, 602262, 602261,134797, 600754, 120361, 116935, 602263, 603320, 601807, 603321, 185250, 185261, 253700, 128239, 120324, 193300, 276901, 308700, 600514, 600261, 602109, 120140, 120150, 147557, 193400, 600536, 188826, 120180, 118661, 120320, 152200, 135821, 112260, 230740, 602090, 155760, 192975, 190182, 602108, 601463, 186745, 600900, 600985, 600758, 602369,179590, 601211, 600065, 602178, 600262, 182888, 182889, 151510, 182120, 150325, 190181, 150370, 186355, 193065, 165070, 154705, 147559, 146650, 146640, 153619, 175100, 187380, 231050, 188060, 135820, 156790, 130660, 301870, 128240, 600076, 600119, 193210, 600215, 600245, 121010, 150240, 600309, 600491, 222600, 120328, 600564, 600596, 600616, 600700, 600742, 120325, 138297, 600930,
   156225, 601028, 601050, 601105, 253800, 601284, 601313, 120280, 310200, 601492, 120250, 601587, 601636, 601652, 601692, 601728, 120220, 601915, 602048, 155120, 310300, 120210, 120165, 120120, 118940, 116930, 602264, 116806, 602366, 120470, 602415, 602428, 602453, 602505, 602574, 603005, 603196, 603221, 603319, 107269, 216550, 103320, 603489, 603551, 603767, 603799, 603842

There are several methods for analyzing the expression levels of genes associated with diseases. Differences in gene expression levels between diseased and normal tissues are studied by the analytical methods, for example, Northern hybridization and differential display. Other examples include a method with high-density cDNA filter, a method with DNA microarray and methods with PCR amplification (Experimental Medicine, Vol.17, No. 8, 980-1056 (1999); Cell Engineering (additional volume) DNA Microarray and Advanced PCR Methods, Muramatsu & Naba (eds.), Shujunsya). The varying levels of gene expression between diseased tussues and normal tissues can be studied by any of these analytical methods. When explicit difference in the expression level is observed for a gene, it can be conclude that the gene is closely associated with a disease or disorder. Instead of diseased tissues, cultured cells can be used for the assessment. Similarly, when gene expression is explicitly different between normal cells and cells reproducing disease-associated specific features, it can be concluded that the gene is closely associated with a disease or disorder. When the expression levels of genes are evidently varied during major cellular events (such as differentiation and apoptosis), the genes are involved in the cellular events and accordingly are candidates for disease- and/or disorder-associated genes. Further, genes exhibiting tissue-specific expression are genes playing important parts in the tissue functions and, therefore, can be candidates for genes associated with diseases and/or disorders affecting the tissues.

For example, non-enzymic protein glycation reaction is believed to be a cause for a variety of chronic diabetic complications. Accordingly, genes of which expression levels are elevated or decreased in a glycated protein-dependent manner in the endothelial cells, are associated with diabetic complications caused by glycated proteins (Diabetes 1996, 45 (Suppl. 3), S67-S72; Diabetes, 1997, 46 (Suppl. 2), S19-S25).
The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center, http://www.rheuma-net.or.jp/). Recent studies have also revealed that tissue necrosis factor (TNF)-α participates in the onset (Current opinion in immunology 1999, 11, 657-662). When the expression of a gene exhibits responsiveness to the action of TNF on synovial cells, the gene is considered to be involved in rheumatoid arthritis. Many genes acting at the downstream of TNF-α and IL-1β among inflammation-associated cytokines have been previously identified. The respective stimulations are transduced through independent pathways of signaling cascade. There exists another signaling cascade for both stimulations, wherein NF-κ B is a common transducing molecule shared by the two stimulations (J. Leukoc. Biol., 1994, 56(5): 542-547). It has also been revealed that many inflammation-associated genes, including IL-2, IL-6 and G-CSF, are varied in the expression levels thereof in response to the signal through the common pathway (Trend Genet. 1999, 15(6): 229-235). It is assumed that genes of which expression levels are varied in response to the stimulation of TNF-α or IL-1β also participate in inflammation.

Ultraviolet radiation damage has been recognized as a risk factor for skin cancers, etc. (United States Environmental Protection Agency: Ozone Depletion Home Page, http://www.epa.gov/ozone/). Genes of which expression levels are varied in skin epidermal cells exposed to ultraviolet rays are considered to be associated with ultraviolet radiation damage of skin. In addition, genes associated with neural differentiation can be candidates for genes responsible for neurological diseases as well as candidates for genes usable for treating the diseases.

Clones exhibiting differences in the expression levels thereof can be selected by using gene expression analysis. The selection comprises, for example; analyzing cDNA clones by using high-density cDNA filter; and statistically treating the multiple signal values (signal values of radioisotope in the labeled probes or values obtained by measuring fluorescence intensities emitted from the fluorescent labels) for the respective clones by two-sample t-test, where the signal values are determined by multiple experiments of hybridization. The clones of interest are selectable based on the statistically significant differences in the signal distribution at p<0.05. However, selectable clones with significant difference in the expression levels thereof may be changed depending on the partial modification of statistical treatment. For example, the clones may be selected by conducting statistical treatment with two-sample t-test at p<0.01; or genes exhibiting more explicit differences in the expression levels thereof can be selected by performing statistical treatment with a pre-determined cut-off value for the significant signal difference. An alternative method is that the expression levels are simply compared with each other, and then, the clones of interest are selected based on the ratio of the expression levels thereof.

Clones that vary in their expression levels can also be selected by comparing the expression levels by PCR analysis, for example, by using the method of determining the band intensities representing the amounts of PCR products with ethidium bromide staining; the method of determining the values of radioisotope signals or fluorescence intensities of the PCR products when radiolabeled or fluorescent dye-labeled primers, respectively, are used in PCR amplification; or the method of determining the values of radioisotope signals or fluorescence intensities of the probes hybridized to the PCR products when radiolabeled or fluorescent dye-labeled probes, respectively, are used in the hybridization. If the expression level ratios obtained in multiple PCR experiments are constantly at least 2-fold, such a clone can be judged to vary in its expression level. When the ratios are several-fold or not less than 10-fold, the clone can be selected as a gene exhibiting the explicit difference in its expression level.

A survey of genes of which expression levels are varied specifically to the glycated protein in the endothelial cells has revealed genes with elevated expression levels, HEMBA1003958, HEMBA1004850, MAMMA1001256, MAMMA1002132, PLACE2000411 and PLACE3000119. On the other hand, a gene of which expression level is decreased specifically to the glycated protein is MAMMA1001783. These clones are genes associated with diabetes.

A survey of genes of which expression levels are varied in response to TNF-α (Tumor Necrosis Factor-alpha) in the primary cell culture of synovial tissue has revealed the following clones with elevated expression levels in the presence of TNF-α:
HEMBA1000005, HEMBA1000012, HEMBA1000020, HEMBA1000046, HEMBA1000076, HEMBA1000111, HEMBA1000168, HEMBA1000185, HEMBA1000201, HEMBA1000231, HEMBA1000243, HEMBA1000280, HEMBA1000282, HEMBA1000304, HEMBA1000307, HEMBA1000327, HEMBA1000356, HEMBA1000376, HEMBA1000387, HEMBA1000390, HEMBA1000418, HEMBA1000460, HEMBA1000491, HEMBA1000501, HEMBA1000518, HEMBA1000519, HEMBA1000520, HEMBA1000531, HEMBA1000534, HEMBA1000542, HEMBA1000545, HEMBA1000591, HEMBA1000592, HEMBA1000594, HEMBA1000636, HEMBA1000655, HEMBA1000657, HEMBA1000673, HEMBA1000682, HEMBA1000686, HEMBA1000722, HEMBA1000726, HEMBA1000827, HEMBA1000870, HEMBA1000918, HEMBA1000971, HEMBA1000974, HEMBA1000986, HEMBA1001019, HEMBA1001043, HEMBA1001051, HEMBA1001059, HEMBA1001060, HEMBA1001071, HEMBA1001080, HEMBA1001109, HEMBA1001140, HEMBA1001172, HEMBA1001196, HEMBA1001213, HEMBA1001226, HEMBA1001281, HEMBA1001299, HEMBA1001302, HEMBA1001303, HEMBA1001323, HEMBA1001326, HEMBA1001327, HEMBA1001330, HEMBA1001351, HEMBA1001407, HEMBA1001411, HEMBA1001446, HEMBA1001454, HEMBA1001569, HEMBA1001647, HEMBA1001714, HEMBA1001800, HEMBA1001804, HEMBA1001809, HEMBA1001888, HEMBA1001912, HEMBA1001921, HEMBA1001967, HEMBA1002084, HEMBA1002161, HEMBA1002166, HEMBA1002241, HEMBA1002337, HEMBA1002363, HEMBA1002389, HEMBA1002458, HEMBA1002460, HEMBA1002469, HEMBA1002538, HEMBA1002542, HEMBA1002547, HEMBA1002609, HEMBA1002624,
HEMBA1002659, HEMBA1002750, HEMBA1002770, HEMBA1002779, HEMBA1002810, HEMBA1002816, HEMBA1002818, HEMBA1002850, HEMBA1002863, HEMBA1003021, HEMBA1003033, HEMBA1003078, HEMBA1003166, HEMBA1003202, HEMBA1003204, HEMBA1003229, HEMBA1003235, HEMBA1003276, HEMBA1003286, HEMBA1003296, HEMBA1003370, HEMBA1003376, HEMBA1003403, HEMBA1003418, HEMBA1003433, HEMBA1003447, HEMBA1003560, HEMBA1003569, HEMBA1003571, HEMBA1003591, HEMBA1003597, HEMBA1003598, HEMBA1003621, HEMBA1003656, HEMBA1003662, HEMBA1003680, HEMBA1003715, HEMBA1003725, HEMBA1003729, HEMBA1003733, HEMBA1003742, HEMBA1003773, HEMBA1003783, HEMBA1003950, HEMBA1004012, HEMBA1004015, HEMBA1004048, HEMBA1004074, HEMBA1004086, HEMBA1004111, HEMBA1004131, HEMBA1004202, HEMBA1004203, HEMBA1004207, HEMBA1004248, HEMBA1004274, HEMBA1004321, HEMBA1004330, HEMBA1004356, HEMBA1004366, HEMBA1004405, HEMBA1004408, HEMBA1004429, HEMBA1004499, HEMBA1004507, HEMBA1004509, HEMBA1004542, HEMBA1004596, HEMBA1004604, HEMBA1004776, HEMBA1004889, HEMBA1004934, HEMBA1004978, HEMBA1005019, HEMBA1005047, HEMBA1005206, HEMBA1005219, HEMBA1005274, HEMBA1005331, HEMBA1005338, HEMBA1005394, HEMBA1005423, HEMBA1005576, HEMBA1005732, HEMBA1005746, HEMBA1006091, HEMBA1006142, HEMBA1006173, HEMBA1006198, HEMBA1006253, HEMBA1006268, HEMBA1006309, HEMBA1006377, HEMBA1006474, HEMBA1006486, HEMBA1006492, HEMBA1006502, HEMBA1006535, HEMBA1006579, HEMBA1006648, HEMBA1006659, HEMBA1006885, HEMBA1006929, HEMBA1006941, HEMBA1007078, HEMBA1007080, HEMBA1007121, HEMBA1007194, HEMBA1007300, HEMBA1007301, HEMBA1007322, HEMBB1000036, HEMBB1000044, HEMBB1000089, HEMBB1000215, HEMBB1000217, HEMBB1000272, HEMBB1000420, HEMBB1000591, HEMBB1000593, HEMBB1000631, HEMBB1000835, HEMBB1000887, HEMBB1000908, HEMBB1000975, HEMBB1000985, HEMBB1001011, HEMBB1001014, HEMBB1001112, HEMBB1001133, HEMBB1001331, HEMBB1001337, HEMBB1001366, HEMBB1001367, HEMBB1001384, HEMBB1001394, HEMBB1001429, HEMBB1001463, HEMBB1001619, HEMBB1001684, HEMBB1001706, HEMBB1001753, HEMBB1001797, HEMBB1001802, HEMBB1001812, HEMBB1001874, HEMBB1001910, HEMBB1001915, HEMBB1001973, HEMBB1001983, HEMBB1001990, HEMBB1002190, HEMBB1002193, HEMBB1002249, HEMBB1002329, HEMBB1002342, HEMBB1002371, HEMBB1002409, HEMBB1002442, HEMBB1002489, HEMBB1002492, HEMBB1002520, HEMBB1002534, HEMBB1002596, HEMBB1002664, HEMBB1002692, HEMBB1002697, HEMBB1002705, MAMMA1000092, MAMMA1000155, MAMMA1000163, MAMMA1000173, MAMMA1000175, MAMMA1000227, MAMMA1000241, MAMMA1000257, MAMMA1000264, MAMMA1000266, MAMMA1000270, MAMMA1000307, MAMMA1000410, MAMMA1000413, MAMMA1000416, MAMMA1000421, MAMMA1000472, MAMMA1000501, MAMMA1000605, MAMMA1000643, MAMMA1000670, MAMMA1000684, MAMMA1000696, MAMMA1000732, MAMMA1000752, MAMMA1000802, MAMMA1000824, MAMMA1000905, MAMMA1000921, MAMMA1000931, MAMMA1000957, MAMMA1000962,
MAMMA1000998, MAMMA1001008, MAMMA1001050, MAMMA1001074, MAMMA1001078, MAMMA1001292, MAMMA1001397, MAMMA1001476, MAMMA1001743, MAMMA1001744, MAMMA1001754, MAMMA1001760, MAMMA1001785, MAMMA1001858, MAMMA1001908, MAMMA1002236, MAMMA1002267, MAMMA1002292, MAMMA1002311, MAMMA1002322, MAMMA1002359, MAMMA1002362, MAMMA1002485, MAMMA1002494, MAMMA1002597, MAMMA1002598, MAMMA1002665, MAMMA1002671, MAMMA1002684, MAMMA1002748, MAMMA1002775, MAMMA1002830, MAMMA1002858, MAMMA1002868, MAMMA1002886, MAMMA1002887, MAMMA1002892, MAMMA1002909, MAMMA1002937, MAMMA1002947, MAMMA1002964, MAMMA1002970, MAMMA1003013, MAMMA1003150, NT2RM1000039, NT2RM1000062, NT2RM1000080, NT2RM1000086, NT2RM1000127, NT2RM1000132, NT2RM1000187, NT2RM1000199, NT2RM1000244, NT2RM1000256, NT2RM1000272, NT2RM1000318, NT2RM1000354, NT2RM1000377, NT2RM1000430, NT2RM1000499, NT2RM1000539, NT2RM1000553, NT2RM1000563, NT2RM1000699, NT2RM1000742, NT2RM1000826, NT2RM1000829, NT2RM1000833, NT2RM1000882, NT2RM1000898, NT2RM1000905, NT2RM1001092, NT2RM2000013, NT2RM2000032, NT2RM2000042, NT2RM2000101, NT2RM2000124, NT2RM2000192, NT2RM2000259, NT2RM2000260, NT2RM2000363, NT2RM2000368, NT2RM2000402, NT2RM2000452, NT2RM2000952, NT2RM2001221, NT2RM2002014, NT2RM2002030, NT2RM4000156, NT2RM4000349, NT2RM4000395, NT2RM4000457, NT2RM4000511, NT2RM4000514, NT2RM4000698, NT2RM4000764, NT2RM4001016, NT2RM4001084, NT2RM4001594, NT2RM4001629,
NT2RM4001662, NT2RM4001841, NT2RM4002093, NT2RM4002109, NT2RM4002145, NT2RM4002189, NT2RM4002194, NT2RM4002226, NT2RP1000170, NT2RP1000439, NT2RP1000478, NT2RP1000513, NT2RP1000701, NT2RP1000856, NT2RP1001361, NT2RP2000097, NT2RP2000239, NT2RP2000288, NT2RP2000328, NT2RP2000329, NT2RP2000369, NT2RP2000422, NT2RP2000842, NT2RP2000965, NT2RP2001245, NT2RP2001440, NT2RP2001560, NT2RP2001634, NT2RP2001663, NT2RP2001677, NT2RP2001762, NT2RP2002270, NT2RP2002312, NT2RP2002316, NT2RP2002333, NT2RP2002706, NT2RP2002925, NT2RP2002959, NT2RP2002987, NT2RP2003125, NT2RP2003137, NT2RP2003237, NT2RP2003272, NT2RP2003596, NT2RP2003604, NT2RP2003643, NT2RP2003968, NT2RP2003976, NT2RP2004194, NT2RP2004321, NT2RP2005037, NT2RP2005140, NT2RP2005204, NT2RP2005293, NT2RP2005457, NT2RP2005555, NT2RP2005600, NT2RP2005701, NT2RP2005719, NT2RP2005722, NT2RP2005773, NT2RP2005890, NT2RP2006023, NT2RP2006071, NT2RP3000186, NT2RP3000341, NT2RP3000599, NT2RP3000632, NT2RP3000644, NT2RP3000852, NT2RP3000968, NT2RP3001096, NT2RP3001109, NT2RP3001126, NT2RP3001147, NT2RP3001449, NT2RP3001529, NT2RP3001753, NT2RP3001854, NT2RP3001915, NT2RP3001969, NT2RP3002081, NT2RP3002142, NT2RP3002399, NT2RP3002590, NT2RP3002603, NT2RP3002810, NT2RP3002876, NT2RP3003311, NT2RP3003330, NT2RP3003672, NT2RP3004209, NT2RP3004378, NT2RP4000078, NT2RP4000541, NT2RP4000588, NT2RP4001219, NT2RP4001228, NT2RP4001276, NT2RP4001507, NT2RP4002047, NT2RP5003459, NT2RP5003492, OVARC1000085, OVARC1000087, OVARC1000106, OVARC1000151, OVARC1000198, OVARC1000431, OVARC1000440, OVARC1000564, OVARC1000605, OVARC1000679, OVARC1000883, OVARC1000912, OVARC1000960, OVARC1000971, OVARC1001038, OVARC1001055, OVARC1001085, OVARC1001129, OVARC1001167, OVARC1001339, OVARC1001425, OVARC1001745, OVARC1001762, OVARC1001766, OVARC1001942, OVARC1002044, OVARC1002138, PLACE1000004, PLACE1000005, PLACE1000420, PLACE1000547, PLACE1000562, PLACE1000653, PLACE1001168, PLACE1001311, PLACE1001377, PLACE1001920, PLACE1001983, PLACE1002066, PLACE1002072, PLACE1002140, PLACE1002171, PLACE1002319, PLACE1002474, PLACE1002499, PLACE1002532, PLACE1002665, PLACE1003025, PLACE1003145, PLACE1003361, PLACE1003605, PLACE1003704, PLACE1003783, PLACE1003885, PLACE1004405, PLACE1004629, PLACE1004686, PLACE1004930, PLACE1005066, PLACE1005077, PLACE1005630, PLACE1005876, PLACE1006143, PLACE1006325, PLACE1006488, PLACE1006805, PLACE1006829, PLACE1007286, PLACE1007858, PLACE1008201, PLACE1009045, PLACE1009113, PLACE1009621, PLACE1010106, PLACE1010310, PLACE1010622, PLACE1010944, PLACE1010965, PLACE1011185, PLACE1011332, PLACE1011635, PLACE1011646, PLACE1011725, PLACE2000014, PLACE2000264, PLACE2000394, PLACE2000419, PLACE3000160, PLACE3000220, PLACE3000254, PLACE3000271, PLACE3000339, PLACE3000341, PLACE3000350, PLACE3000353, PLACE3000401, PLACE4000300, SKNMC1000091, THYRO1000855, THYRO1001559, Y79AA1000065, Y79AA1000202, Y79AA1000214, Y79AA1000346, Y79AA1000784, Y79AA1000833, Y79AA1000968, Y79AA1001555, Y79AA1002220

On the other hand, clones with decreased expression levels in the presence of TNFα are:
HEMBA1002150, HEMBB1000240, NT2RM2000469, NT2RM2000984, NT2RM2001688, NT2RM4000290, NT2RM4000496, NT2RM4000590, NT2RM4001047, NT2RM4001582, NT2RM4001611, NT2RM4001650, NT2RM4002075, NT2RM4002128, NT2RP1000174, NT2RP1000243, NT2RP1000581, NT2RP1000688, NT2RP1000767, NT2RP1000825, NT2RP1001185; NT2RP1001286, NT2RP1001432, NT2RP1001457, NT2RP2000001, NT2RP2000248, NT2RP2000841, NT2RP2001813, NT2RP2002137, NT2RP2002928, NT2RP2003517, NT2RP2003559, NT2RP2003564, NT2RP2004933, NT2RP2005038, NT2RP2006365, NT2RP3000072, NT2RP3000320, NT2RP3000484, NT2RP3000980, NT2RP3001111, NT2RP3001420, NT2RP3001495, NT2RP3002056, NT2RP3002057, NT2RP3002545, NT2RP3002713, NT2RP3002799, NT2RP3002869, NT2RP3002953, NT2RP3002955, NT2RP3003282, NT2RP3003290, NT2RP3003384, NT2RP3003385, NT2RP3003870, NT2RP3004207, NT2RP3004262, NT2RP3004527, NT2RP4000500, NT2RP4000524, NT2RP4000787, NT2RP4000927, NT2RP4000955, NT2RP4000989, NT2RP4001442, NT2RP4001638, NT2RP4001950, NT2RP4002888, NT2RP5003524, OVARC1001270, PLACE1000246, PLACE1002816.

These are rheumatoid arthritis-associated clones.

A survey of genes of which expression levels are varied in primary cultured skin fibroblast cells exposed to ultraviolet light has revealed the following clones with elevated expression levels by ultraviolet radiation: HEMBA1000542, HEMBA1001808, HEMBA1002177, HEMBA1003314, MAMMA1001874, NT2RM2001100, NT2RP2005732, NT2RP3000592, NT2RP4000657, OVARC 1000004, OVARC1001092, OVARC1001342, PLACE1002816, NT2RM4001002, NT2RM4001813, NT2RM4002266, NT2RP2001174, NT2RP2001196, NT2RP2005358, NT2RP3000690, NT2RP3001216, NT2RP3003464, PLACE1006382, THYRO1000070, THYRO1001100, Y79AA1000342

On the other hand, the expression levels of the following clones were decreased by ultraviolet radiation:
HEMBA1000005, HEMBA1000150, HEMBA1000156, HEMBA1000158, HEMBA1000168, HEMBA1000231, HEMBA1000304, HEMBA1000307, HEMBA1000333, HEMBA1000366, HEMBA1000369, HEMBA1000390, HEMBA1000396, HEMBA1000418, HEMBA1000434, HEMBA1000464, HEMBA1000469, HEMBA1000490, HEMBA1000504, HEMBA1000505, HEMBA1000557, HEMBA1000657, HEMBA1000673, HEMBA1000682, HEMBA1000686, HEMBA1000727, HEMBA1000752, HEMBA1000851, HEMBA1000852, HEMBA1000870, HEMBA1000872, HEMBA1001085, HEMBA1001121, HEMBA1001133, HEMBA1001235, HEMBA1001265, HEMBA1001281, HEMBA1001289, HEMBA1001299, HEMBA1001303, HEMBA1001310, HEMBA1001323, HEMBA1001595, HEMBA1001620, HEMBA1001640, HEMBA1001678, HEMBA1001712, HEMBA1001835, HEMBA1001950, HEMBA1001987, HEMBA1002253, HEMBA1002321, HEMBA1002341, HEMBA1002419, HEMBA1002679, HEMBA1002728, HEMBA1002818, HEMBA1002935, HEMBA1002999, HEMBA1003034, HEMBA1003071, HEMBA1003098, HEMBA1003142, HEMBA1003175, HEMBA1003202, HEMBA1003212, HEMBA1003220, HEMBA1003276, HEMBA1003373, HEMBA1003417, HEMBA1003447, HEMBA1003528, HEMBA1003684, HEMBA1003799, HEMBA1003885, HEMBA1003989, HEMBA1004011, HEMBA1004055, HEMBA1004133, HEMBA1004225, HEMBA1004272, HEMBA1004353, HEMBA1004631, HEMBA1004669, HEMBA1004705, HEMBA1004753, HEMBA1004776, HEMBA1004803, HEMBA1004816, HEMBA1004900, HEMBA1005047, HEMBA1005079, HEMBA1005101, HEMBA1005149, HEMBA1005152, HEMBA1005202, HEMBA1005314, HEMBA1005372, HEMBA1005511, HEMBA1005513, HEMBA1005518, HEMBA1005570, HEMBA1005577, HEMBA1005581, HEMBA1005588, HEMBA1005609, HEMBA1005632, HEMBA1005853, HEMBA1006031, HEMBA1006035, HEMBA1006485, HEMBA1006486, HEMBA1006502, HEMBA1006696, HEMBA1006789, HEMBA1006796, HEMBA1007085, HEMBA1007224, HEMBA1007301, HEMBA1007319, HEMBA1007341, HEMBA1007342, HEMBB1000036, HEMBB1000037, HEMBB1000217, HEMBB1000266, HEMBB1000317, HEMBB1000336, HEMBB1000354, HEMBB1000369, HEMBB1000399, HEMBB1000434, HEMBB1000438, HEMBB1000592, HEMBB1000673, HEMBB1000789, HEMBB1000810, HEMBB1000883, HEMBB1000887, HEMBB1001105, HEMBB1001182, HEMBB1001242, HEMBB1001267, HEMBB1001424, HEMBB1001464, HEMBB1001531, HEMBB1001618, HEMBB1001996, HEMBB1002092, HEMBB1002139, HEMBB1002142, HEMBB1002190, HEMBB1002453, HEMBB1002520, HEMBB1002550, HEMBB1002556, HEMBB1002600, HEMBB1002664, MAMMA1000009, MAMMA1000055, MAMMA1000069, MAMMA1000133, MAMMA1000171, MAMMA1000173, MAMMA1000287, MAMMA1000416, MAMMA1000585, MAMMA1000713, MAMMA1000760, MAMMA1000798, MAMMA1000831, MAMMA1000875, MAMMA1000876, MAMMA1000877, MAMMA1000906, MAMMA1000931, MAMMA1000962, MAMMA1001133, MAMMA1001139, MAMMA1001243, MAMMA1001271, MAMMA1001274, MAMMA1001298, MAMMA1001606, MAMMA1001630, MAMMA1001670, MAMMA1001743, MAMMA1001751, MAMMA1002140, MAMMA1002145, MAMMA1002158, MAMMA1002170, MAMMA1002236, MAMMA1002311, MAMMA1002498, MAMMA1002754, MAMMA1002780, MAMMA1002820, MAMMA1002843, MAMMA1002844, MAMMA1002871, MAMMA1003047, NT2RM1000037, NT2RM1000039, NT2RM1000080, NT2RM1000086, NT2RM1000341, NT2RM1000499, NT2RM1000669, NT2RM1000746, NT2RM1000781, NT2RM1000885, NT2RM1000905, NT2RM1000962, NT2RM2000239, NT2RM2000260, NT2RM2000371, NT2RM2000639, NT2RM2000649, NT2RM2000735, NT2RM2000821, NT2RM2000984, NT2RM2001035, NT2RM2001065, NT2RM2001105, NT2RM2001177, NT2RM2001194, NT2RM2001196, NT2RM2001243, NT2RM2001256, NT2RM2001424, NT2RM2001588, NT2RM2001635, NT2RM2001648, NT2RM2001652, NT2RM2001668, NT2RM2001706, NT2RM2001727, NT2RM2001730, NT2RM2001743, NT2RM2001753, NT2RM2001760, NT2RM2001771, NT2RM2001785, NT2RM2001800, NT2RM2001855, NT2RM2001896, NT2RM2001997, NT2RM2002030, NT2RM2002049, NT2RM2002091, NT2RM2002142, NT2RM2002145, NT2RM2002178, NT2RM2002580, NT2RM4000215, NT2RM4000344, NT2RM4000368, NT2RM4000421, NT2RM4000425, NT2RM4000457, NT2RM4000496, NT2RM4000515, NT2RM4000712, NT2RM4000787, NT2RM4000813, NT2RM4000820, NT2RM4000852, NT2RM4000950, NT2RM4000996, NT2RM4001016, NT2RM4001047, NT2RM4001054, NT2RM4001140, NT2RM4001151, NT2RM4001187, NT2RM4001204, NT2RM4001258, NT2RM4001437, NT2RM4001454, NT2RM4001489, NT2RM4001605, NT2RM4001611, NT2RM4001666, NT2RM4001710, NT2RM4001714, NT2RM4001715, NT2RM4001731, NT2RM4001741, NT2RM4001746, NT2RM4001856, NT2RM4001938, NT2RM4001940, NT2RM4001984, NT2RM4001987, NT2RM4002013, NT2RM4002054, NT2RM4002055, NT2RM4002073, NT2RM4002145, NT2RM4002146, NT2RM4002161, NT2RM4002174, NT2RM4002194, NT2RM4002205, NT2RM4002213, NT2RM4002266, NT2RM4002278, NT2RM4002558, NT2RM4002565, NT2RM4002593, NT2RM4002594, NT2RP1000063, NT2RP1000111, NT2RP1000124, NT2RP1000174, NT2RP1000348, NT2RP1000443, NT2RP1000522, NT2RP1000677, NT2RP1000695, NT2RP1000730, NT2RP1000767, NT2RP1000834, NT2RP1000856, NT2RP1000944, NT2RP1001014, NT2RP1001073, NT2RP1001079, NT2RP1001177, NT2RP1001185, NT2RP1001199, NT2RP1001253, NT2RP1001310, NT2RP1001313, NT2RP1001385, NT2RP1001410, NT2RP1001449, NT2RP1001546, NT2RP2000120, NT2RP2000126, NT2RP2000205, NT2RP2000224, NT2RP2000274, NT2RP2000298, NT2RP2000310, NT2RP2000327, NT2RP2000328, NT2RP2000329, NT2RP2000337, NT2RP2000369, NT2RP2000414, NT2RP2000498, NT2RP2000510, NT2RP2000715, NT2RP2000819, NT2RP2000931, NT2RP2000938, NT2RP2001044, NT2RP2001065, NT2RP2001081, NT2RP2001137, NT2RP2001312, NT2RP2001327, NT2RP2001366, NT2RP2001381, NT2RP2001420, NT2RP2001427, NT2RP2001449, NT2RP2001511, NT2RP2001526, NT2RP2001560, NT2RP2001576, NT2RP2001581, NT2RP2001601, NT2RP2001613, NT2RP2001628, NT2RP2001663, NT2RP2001675, NT2RP2001677, NT2RP2001678, NT2RP2001699, NT2RP2001721, NT2RP2001740, NT2RP2001813, NT2RP2001861, NT2RP2001876, NT2RP2001907, NT2RP2001926, NT2RP2001946, NT2RP2001985, NT2RP2002032, NT2RP2002046, NT2RP2002076, NT2RP2002099, NT2RP2002105, NT2RP2002137, NT2RP2002172, NT2RP2002208, NT2RP2002219, NT2RP2002231, NT2RP2002256, NT2RP2002270, NT2RP2002292, NT2RP2002316, NT2RP2002333, NT2RP2002520, NT2RP2002549, NT2RP2002621, NT2RP2002706, NT2RP2002710, NT2RP2002750, NT2RP2002800, NT2RP2002862, NT2RP2002880, NT2RP2002925, NT2RP2002929, NT2RP2002939, NT2RP2002959, NT2RP2002993, NT2RP2003000, NT2RP2003158, NT2RP2003243, NT2RP2003265, NT2RP2003277, NT2RP2003280, NT2RP2003295, NT2RP2003297, NT2RP2003433, NT2RP2003445, NT2RP2003456, NT2RP2003506, NT2RP2003543, NT2RP2003559, NT2RP2003567, NT2RP2003604, NT2RP2003706, NT2RP2003777, NT2RP2003781, NT2RP2003871, NT2RP2003952, NT2RP2003976, NT2RP2004013, NT2RP2004014, NT2RP2004081, NT2RP2004142, NT2RP2004165, NT2RP2004170, NT2RP2004194, NT2RP2004196, NT2RP2004239, NT2RP2004240,NT2RP2004321, NT2RP2004364, NT2RP2004365, NT2RP2004373, NT2RP2004389, NT2RP2004392, NT2RP2004399, NT2RP2004538, NT2RP2004587, NT2RP2004600, NT2RP2004602, NT2RP2004614, NT2RP2004664, NT2RP2004675, NT2RP2004681, NT2RP2004710, NT2RP2004743, NT2RP2004767, NT2RP2004799, NT2RP2004816, NT2RP2004959, NT2RP2004962, NT2RP2005000, NT2RP2005003, NT2RP2005012, NT2RP2005031, NT2RP2005037, NT2RP2005108, NT2RP2005126, NT2RP2005144, NT2RP2005147, NT2RP2005289, NT2RP2005293, NT2RP2005325, NT2RP2005354, NT2RP2005441, NT2RP2005464, NT2RP2005491, NT2RP2005495, NT2RP2005498, NT2RP2005501, NT2RP2005555, NT2RP2005557, NT2RP2005605, NT2RP2005622, NT2RP2005645, NT2RP2005651, NT2RP2005654, NT2RP2005669, NT2RP2005694, NT2RP2005701, NT2RP2005752, NT2RP2005773, NT2RP2005804, NT2RP2005835, NT2RP2005841, NT2RP2005857, NT2RP2005890, NT2RP2005901, NT2RP2005942, NT2RP2006023, NT2RP2006052, NT2RP2006069, NT2RP2006166, NT2RP2006184, NT2RP2006186, NT2RP2006196, NT2RP2006238, NT2RP2006258, NT2RP2006312, NT2RP2006320, NT2RP2006565, NT2RP2006571, NT2RP3000050, NT2RP3000055, NT2RP3000072, NT2RP3000080, NT2RP3000109, NT2RP3000134, NT2RP3000142, NT2RP3000149, NT2RP3000186, NT2RP3000197, NT2RP3000207, NT2RP3000220, NT2RP3000247, NT2RP3000251, NT2RP3000361, NT2RP3000366, NT2RP3000418, NT2RP3000449, NT2RP3000451, NT2RP3000484, NT2RP3000487, NT2RP3000512, NT2RP3000527, NT2RP3000542, NT2RP3000561, NT2RP3000562, NT2RP3000578, NT2RP3000596, NT2RP3000605, NT2RP3000624, NT2RP3000644, NT2RP3000661, NT2RP3000665, NT2RP3000739, NT2RP3000850, NT2RP3000868, NT2RP3000980, NT2RP3001084, NT2RP3001096, NT2RP3001109, NT2RP3001111, NT2RP3001115, NT2RP3001119, NT2RP3001120, NT2RP3001126, NT2RP3001140, NT2RP3001155, NT2RP3001176, NT2RP3001272, NT2RP3001297, NT2RP3001325, NT2RP3001339, NT2RP3001398, NT2RP3001447, NT2RP3001457, NT2RP3001459, NT2RP3001495, NT2RP3001554, NT2RP3001580, NT2RP3001587, NT2RP3001608, NT2RP3001672, NT2RP3001676, NT2RP3001712, NT2RP3001730, NT2RP3001782, NT2RP3001799, NT2RP3001929, NT2RP3001943, NT2RP3002002, NT2RP3002004, NT2RP3002045, NT2RP3002054, NT2RP3002057, NT2RP3002142, NT2RP3002163, NT2RP3002165, NT2RP3002173, NT2RP3002273, NT2RP3002351, NT2RP3002631, NT2RP3002659, NT2RP3002671, NT2RP3002713, NT2RP3002955, NT2RP3003071, NT2RP3003078, NT2RP3003139, NT2RP3003242, NT2RP3003301, NT2RP3003302, NT2RP3003313, NT2RP3003330, NT2RP3003377, NT2RP3003384, NT2RP3003411, NT2RP3003701, NT2RP3003831, NT2RP3003870, NT2RP3003992, NT2RP3004013, NT2RP3004070, NT2RP3004148, NT2RP3004206, NT2RP3004207, NT2RP3004215, NT2RP3004332, NT2RP3004341, NT2RP3004349, NT2RP3004399, NT2RP4000023, NT2RP4000051, NT2RP4000129, NT2RP4000147, NT2RP4000212, NT2RP4000214, NT2RP4000323, NT2RP4000449, NT2RP4000515, NT2RP4000519, NT2RP4000528, NT2RP4000556, NT2RP4000588, NT2RP4000638, NT2RP4000648, NT2RP4000973, NT2RP4000996, NT2RP4001006, NT2RP4001029, NT2RP4001041, NT2RP4001100, NT2RP4001117, NT2RP4001174, NT2RP4001235, NT2RP4001274, NT2RP4001276, NT2RP4001315, NT2RP4001373, NT2RP4001389, NT2RP4001433, NT2RP4001502, NT2RP4001524, NT2RP4001567, NT2RP4001574, NT2RP4001677, NT2RP4001753, NT2RP4001927, NT2RP4002052, NT2RP4002058, NT2RP4002071, NT2RP4002078, NT2RP4002083, NT2RP5003461, NT2RP5003512, NT2RP5003522, OVARC1000085, OVARC1000091, OVARC1000106, OVARC1000109, OVARC1000114, OVARC1000148, OVARC1000168, OVARC1000198, OVARC1000240, OVARC1000241, OVARC1000288, OVARC1000302, OVARC1000309, OVARC1000326, OVARC1000335, OVARC1000347, OVARC1000384, OVARC1000411, OVARC1000414, OVARC1000420, OVARC1000431, OVARC1000437, OVARC1000440, OVARC1000442, OVARC1000465, OVARC1000473, OVARC1000479, OVARC1000486, OVARC1000526, OVARC1000556, OVARC1000557, OVARC1000564, OVARC1000573, OVARC1000578, OVARC1000588, OVARC1000605, OVARC1000622, OVARC1000678, OVARC1000679, OVARC1000681, OVARC1000703, OVARC1000730, OVARC1000746, OVARC1000769, OVARC1000781, OVARC1000787, OVARC1000800, OVARC1000834, OVARC1000846, OVARC1000850, OVARC1000862, OVARC1000876, OVARC1000883, OVARC1000891, OVARC1000897, OVARC1000912, OVARC1000915, OVARC1000924, OVARC1000937, OVARC1000945, OVARC1000948, OVARC1000959, OVARC1000960, OVARC1000984, OVARC1000996, OVARC1000999, OVARC1001000, OVARC1001010, OVARC1001011, OVARC1001032, OVARC1001034, OVARC1001038, OVARC1001040, OVARC1001044, OVARC1001055, OVARC1001062, OVARC1001085, OVARC1001117, OVARC1001118, OVARC1001162, OVARC1001167, OVARC1001170, OVARC1001173, OVARC1001180, OVARC1001232, OVARC1001240, OVARC1001261, OVARC1001268, OVARC1001270, OVARC1001271, OVARC1001282, OVARC1001296, OVARC1001306, OVARC1001329, OVARC1001330, OVARC1001339, OVARC1001341, OVARC1001344, OVARC1001360, OVARC1001369, OVARC1001376, OVARC1001381, OVARC1001399, OVARC1001417, OVARC1001419, OVARC1001436, OVARC1001496, OVARC1001506, OVARC1001525, OVARC1001542, OVARC1001600, OVARC1001668, OVARC1001702, OVARC1001731, OVARC1001745, OVARC1001762, OVARC1001791, OVARC1001802, OVARC1001812, OVARC1001813, OVARC1001820, OVARC1001861, OVARC1001873, OVARC1001879, OVARC1001880, OVARC1001883, OVARC1001900, OVARC1001911, OVARC1001916, OVARC1001928, OVARC1001942, OVARC1001949, OVARC1001950, OVARC1001987, OVARC1001989, OVARC1002044, OVARC1002050, OVARC1002066, OVARC1002107, OVARC1002112, OVARC1002127, OVARC1002165, PLACE1000004, PLACE1000078, PLACE1000081, PLACE1000142, PLACE1000184, PLACE1000185, PLACE1000246, PLACE1000292, PLACE1000332, PLACE1000383, PLACE1000401, PLACE1000406, PLACE1000420, PLACE1000421, PLACE1000435, PLACE1000453, PLACE1000481, PLACE1000562, PLACE1000583, PLACE1000610, PLACE1000656, PLACE1000785, PLACE1000798, PLACE1000909, PLACE1000948, PLACE1001010, PLACE1001076, PLACE1001092, PLACE1001104, PLACE1001171, PLACE1001185, PLACE1001279, PLACE1001304, PLACE1001311, PLACE1001323, PLACE1001351, PLACE1001366, PLACE1001502, PLACE1001534, PLACE1001602, PLACE1001603, PLACE1001608, PLACE1001610, PLACE1001632, PLACE1001634, PLACE1001720, PLACE1001729, PLACE1001739, PLACE1001745, PLACE1001746, PLACE1001748, PLACE1001781, PLACE1001799, PLACE1001821, PLACE1001912, PLACE1001928, PLACE1001989, PLACE1002072, PLACE1002090, PLACE1002115, PLACE1002150, PLACE1002170, PLACE1002319, PLACE1002342, PLACE1002395, PLACE1002399, PLACE1002433, PLACE1002437, PLACE1002438, PLACE1002450, PLACE1002499, PLACE1002514, PLACE1002583, PLACE1002685, PLACE1002768, PLACE1002772, PLACE1002782, PLACE1002794, PLACE1002811, PLACE1002815, PLACE1002834, PLACE1002839, PLACE1002853, PLACE1002941, PLACE1002968, PLACE1003044, PLACE1003092, PLACE1003100, PLACE1003145, PLACE1003153, PLACE1003176, PLACE1003190, PLACE1003200, PLACE1003205, PLACE1003238, PLACE1003249, PLACE1003302, PLACE1003353, PLACE1003373, PLACE1003383, PLACE1003516, PLACE1003521, PLACE1003592, PLACE1003611, PLACE1003618, PLACE1003704, PLACE1003709, PLACE1003768, PLACE1003784, PLACE1003833, PLACE1003870, PLACE1003886, PLACE1003888, PLACE1003903, PLACE1003936, PLACE1003968, PLACE1004103, PLACE1004114, PLACE1004118, PLACE1004242, PLACE1004256, PLACE1004284, PLACE1004289, PLACE1004316, PLACE1004336, PLACE1004384, PLACE1004388, PLACE1004405, PLACE1004425, PLACE1004428, PLACE1004451, PLACE1004467, PLACE1004491, PLACE1004510, PLACE1004548, PLACE1004658, PLACE1004672, PLACE1004693, PLACE1004716, PLACE1004777, PLACE1004813, PLACE1004824, PLACE1004827, PLACE1004836, PLACE1004840, PLACE1004885, PLACE1004913, PLACE1004918, PLACE1004930, PLACE1004934, PLACE1004972, PLACE1004982, PLACE1004985, PLACE1005026, PLACE1005101, PLACE1005102, PLACE1005128, PLACE1005176, PLACE1005181, PLACE1005187, PLACE1005232, PLACE1005261, PLACE1005277, PLACE1005287, PLACE1005305, PLACE1005327, PLACE1005331, PLACE1005373, PLACE1005374, PLACE1005409, PLACE1005477, PLACE1005480, PLACE1005481, PLACE1005494, PLACE1005502, PLACE1005526, PLACE1005528, PLACE1005530, PLACE1005550, PLACE1005554, PLACE1005557, PLACE1005595, PLACE1005603, PLACE1005611, PLACE1005630, PLACE1005639, PLACE1005646, PLACE1005656, PLACE1005666, PLACE1005730, PLACE1005755, PLACE1005763, PLACE1005799, PLACE1005803, PLACE1005804, PLACE1005834, PLACE1005845, PLACE1005850, PLACE1005876, PLACE1005884, PLACE1005890, PLACE1005898, PLACE1005921, PLACE1005923, PLACE1005932, PLACE1005934, PLACE1005936, PLACE1005951, PLACE1005966, PLACE1005968, PLACE1005990, PLACE1006002, PLACE1006003, PLACE1006011, PLACE1006017, PLACE1006037, PLACE1006040, PLACE1006119, PLACE1006129, PLACE1006139, PLACE1006143, PLACE1006157, PLACE1006159, PLACE1006164, PLACE1006167, PLACE1006170, PLACE1006187, PLACE1006195, PLACE1006205, PLACE1006223, PLACE1006225, PLACE1006236, PLACE1006239, PLACE1006288, PLACE1006318, PLACE1006357, PLACE1006368, PLACE1006371, PLACE1006438, PLACE1006469, PLACE1006482, PLACE1006488, PLACE1006492, PLACE1006506, PLACE1006531, PLACE1006540, PLACE1006552, PLACE1006617, PLACE1006626, PLACE1006673, PLACE1006704, PLACE1006731, PLACE1006754, PLACE1006779, PLACE1006795, PLACE1006800, PLACE1006805, PLACE1006819, PLACE1006829, PLACE1006860, PLACE1006867, PLACE1006878, PLACE1006883, PLACE1006956, PLACE1006958, PLACE1007014, PLACE1007111, PLACE1007140, PLACE1007178, PLACE1007238, PLACE1007239, PLACE1007242, PLACE1007257, PLACE1007274, PLACE1007276, PLACE1007282, PLACE1007286, PLACE1007301, PLACE1007317, PLACE1007346, PLACE1007367, PLACE1007375, PLACE1007386, PLACE1007402, PLACE1007409, PLACE1007416, PLACE1007460, PLACE1007478, PLACE1007484, PLACE1007488, PLACE1007507, PLACE1007511, PLACE1007524, PLACE1007537, PLACE1007544, PLACE1007557, PLACE1007677, PLACE1007688, PLACE1007690, PLACE1007737, PLACE1007743, PLACE1007829, PLACE1007846, PLACE1007852, PLACE1007858, PLACE1007897, PLACE1007908, PLACE1007946, PLACE1007955, PLACE1007958, PLACE1008122, PLACE1008177, PLACE1008181, PLACE1008198, PLACE1008209, PLACE1008244, PLACE1008330, PLACE1008392, PLACE1008405, PLACE1008424, PLACE1008437, PLACE1008455, PLACE1008457, PLACE1008524, PLACE1008531, PLACE1008532, PLACE1008533, PLACE1008568, PLACE1008625, PLACE1008626, PLACE1008627, PLACE1008630, PLACE1008693, PLACE1008715, PLACE1008748, PLACE1008808, PLACE1008813, PLACE1008887, PLACE1008947, PLACE1009045, PLACE1009060, PLACE1009091, PLACE1009099, PLACE1009150, PLACE1009155, PLACE1009183, PLACE1009186, PLACE1009200, PLACE1009308, PLACE1009328, PLACE1009368, PLACE1009398, PLACE1009410, PLACE1009443, PLACE1009444, PLACE1009459, PLACE1009468, PLACE1009476, PLACE1009493, PLACE1009524, PLACE1009581, PLACE1009607, PLACE1009621, PLACE1009721, PLACE1009798, PLACE1009861, PLACE1009879, PLACE1009886, PLACE1009888, PLACE1009947, PLACE1009995, PLACE1010076, PLACE1010083, PLACE1010089, PLACE1010102, PLACE1010105, PLACE1010106, PLACE1010134, PLACE1010194, PLACE1010261, PLACE1010293, PLACE1010310, PLACE1010324, PLACE1010341, PLACE1010364, PLACE1010481, PLACE1010491, PLACE1010547, PLACE1010579, PLACE1010580, PLACE1010599, PLACE1010628, PLACE1010629, PLACE1010662, PLACE1010714, PLACE1010720, PLACE1010739, PLACE1010743, PLACE1010800, PLACE1010811, PLACE1010833, PLACE1010856, PLACE1010870, PLACE1010877, PLACE1010891, PLACE1010896, PLACE1010900, PLACE1010916, PLACE1010917, PLACE1010925, PLACE1010926, PLACE1010942, PLACE1010947, PLACE1010954, PLACE1010960, PLACE1010965, PLACE1011026, PLACE1011041, PLACE1011046, PLACE1011054, PLACE1011090, PLACE1011165, PLACE1011214, PLACE1011229, PLACE1011273, PLACE1011296, PLACE1011310, PLACE1011371, PLACE1011375, PLACE1011399, PLACE1011419, PLACE1011452, PLACE1011465, PLACE1011472, PLACE1011503, PLACE1011563, PLACE1011567, PLACE1011586, PLACE1011641, PLACE1011643, PLACE1011646, PLACE1011650, PLACE1011664, PLACE1011675, PLACE1011725, PLACE1011729, PLACE1011749, PLACE1011762, PLACE1011778, PLACE1011783, PLACE1011858, PLACE1011891, PLACE1011923, PLACE1011962, PLACE1011982, PLACE1011995, PLACE2000006, PLACE2000007, PLACE2000014, PLACE2000017, PLACE2000030, PLACE2000033, PLACE2000039, PLACE2000050, PLACE2000061, PLACE2000100, PLACE2000111, PLACE2000124, PLACE2000132, PLACE2000140, PLACE2000164, PLACE2000170, PLACE2000172, PLACE2000176, PLACE2000187, PLACE2000216, PLACE2000223, PLACE2000264, PLACE2000274, PLACE2000335, PLACE2000341, PLACE2000342, PLACE2000347, PLACE2000366, PLACE2000373, PLACE2000398, PLACE2000419, PLACE2000433, PLACE2000435, PLACE2000455, PLACE2000458, PLACE2000465, PLACE3000020, PLACE3000029, PLACE3000059, PLACE3000103, PLACE3000119, PLACE3000136, PLACE3000156, PLACE3000157, PLACE3000158, PLACE3000160, PLACE3000197, PLACE3000199, PLACE3000207, PLACE3000218, PLACE3000220, PLACE3000221, PLACE3000226, PLACE3000242, PLACE3000244, PLACE3000254, PLACE3000271, PLACE3000276, PLACE3000304, PLACE3000310, PLACE3000320, PLACE3000350, PLACE3000353, PLACE3000363, PLACE3000365, PLACE3000373, PLACE3000388, PLACE3000399, PLACE3000400, PLACE3000455, PLACE4000052, PLACE4000093, PLACE4000128, PLACE4000129, PLACE4000131, PLACE4000147, PLACE4000156, PLACE4000211, PLACE4000222, PLACE4000230, PLACE4000233, PLACE4000261, PLACE4000269, PLACE4000270, PLACE4000320, PLACE4000323, PLACE4000326, PLACE4000369, PLACE4000379, PLACE4000387, PLACE4000392, PLACE4000411, PLACE4000431, PLACE4000487, PLACE4000494, PLACE4000521, PLACE4000522, PLACE4000548, PLACE4000558, PLACE4000581, PLACE4000590, PLACE4000612, SKNMC1000050, SKNMC1000091, THYRO 1000040, THYRO1000085, THYRO1000092, THYRO1000111, THYRO1000121, THYRO1000156, THYRO1000163, THYRO1000186, THYRO1000187, THYRO1000206, THYRO1000241, THYRO1000320, THYRO1000343, THYRO1000358, THYRO1000368, THYRO1000381, THYRO1000387, THYRO1000395, THYRO1000401, THYRO1000452, THYRO1000484, THYRO1000501, THYRO1000502, THYRO1000505, THYRO1000662, THYRO1000666, THYRO1000684, THYRO1000748, THYRO1000787, THYRO1000793, THYRO1000815, THYRO1000829, THYRO1000852, THYRO1000855, THYRO1000895, THYRO1000926, THYRO1000951, THYRO1000952, THYRO1000984, THYRO1000988, THYRO1001031, THYRO1001062, THYRO1001093, THYRO1001120, THYRO1001121, THYRO1001133, THYRO1001134, THYRO1001142, THYRO1001173, THYRO1001177, THYRO1001204, THYRO1001213, THYRO1001290, THYRO1001313, THYRO1001321, THYRO1001322, THYRO1001363, THYRO1001374, THYRO1001401, THYRO1001406, THYRO1001458, THYRO1001617, THYRO1001671, THYRO1001673, THYRO1001703, THYRO1001706, THYRO1001721, THYRO1001738, THYRO1001745, THYRO1001746, THYRO1001809, Y79AA1000013, Y79AA1000033, Y79AA1000059, Y79AA1000202, Y79AA1000230, Y79AA1000268, Y79AA1000346, Y79AA1000420, Y79AA1000480, Y79AA1000539, Y79AA1000540, Y79AA1000574, Y79AA1000627, Y79AA1000705, Y79AA1000734, Y79AA1000748, Y79AA1000752, Y79AA1000774, Y79AA1000784, Y79AA1000794, Y79AA1000800, Y79AA1000802, Y79AA1000805, Y79AA1000833, Y79AA1000850, Y79AA1000962, Y79AA1000966, Y79AA1001041, Y79AA1001048, Y79AA1001061, Y79AA1001078, Y79AA1001105, Y79AA1001177, Y79AA1001185, Y79AA1001211, Y79AA1001216, Y79AA1001281, Y79AA1001299, Y79AA1001312, Y79AA1001323, Y79AA1001384, Y79AA1001402, Y79AA1001533, Y79AA1001548, Y79AA1001581, Y79AA1001594, Y79AA1001665, Y79AA1001679, Y79AA1001692, Y79AA1001696, Y79AA1001711, Y79AA1001805, Y79AA1001827, Y79AA1001846, Y79AA1001848, Y79AA1001866, Y79AA1001875, Y79AA1002027, Y79AA1002083, Y79AA1002089, Y79AA1002093, Y79AA1002103, Y79AA1002115, Y79AA1002139, Y79AA1002208, Y79AA1002209, Y79AA1002220, Y79AA1002234, Y79AA1002246, Y79AA1002307, Y79AA1002311, Y79AA1002351, Y79AA1002361, Y79AA1002399, Y79AA1002433, Y79AA1002472, Y79AA1002482, HEMBA1000542, NT2RM2001100, HEMBA1000012, HEMBA1000201, HEMBA1000216, HEMBA1000244, HEMBA1000251, HEMBA1000303, HEMBA1000355, HEMBA1000356, HEMBA1000456, HEMBA1000488, HEMBA1000519, HEMBA1000591, HEMBA1000592, HEMBA1000636, HEMBA1000637, HEMBA1000722, HEMBA1000817, HEMBA1000934, HEMBA1000971, HEMBA1001052, HEMBA1001059, HEMBA1001122, HEMBA1001123, HEMBA1001213, HEMBA1001226, HEMBA1001257, HEMBA1001302, HEMBA1001326, HEMBA1001327, HEMBA1001570, HEMBA1001651, HEMBA1001745, HEMBA1001847, HEMBA1001912, HEMBA1001921, HEMBA1001942, HEMBA1001979, HEMBA1002039, HEMBA1002185, HEMBA1002199, HEMBA1002229, HEMBA1002257, HEMBA1002265, HEMBA1002337, HEMBA1002475, HEMBA1002513, HEMBA1002515, HEMBA1002624, HEMBA1002628, HEMBA1002712, HEMBA1002794, HEMBA1002816, HEMBA1002876, HEMBA1002939, HEMBA1002968, HEMBA1002971, HEMBA1003064, HEMBA1003077, HEMBA1003086, HEMBA1003304, HEMBA1003328, HEMBA1003376, HEMBA1003402, HEMBA1003556, HEMBA1003560, HEMBA1003568, HEMBA1003597, HEMBA1003598, HEMBA1003656, HEMBA1003715, HEMBA1003733, HEMBA1003760, HEMBA1003773, HEMBA1003784, HEMBA1003803, HEMBA1003838, HEMBA1003864, HEMBA1003950, HEMBA1004012, HEMBA1004138, HEMBA1004143, HEMBA1004241, HEMBA1004246, HEMBA1004264, HEMBA1004276, HEMBA1004354, HEMBA1004502, HEMBA1004538, HEMBA1004670, HEMBA1004752, HEMBA1004771, HEMBA1004880, HEMBA1004923, HEMBA1004972, HEMBA1004995, HEMBA1005029, HEMBA1005039, HEMBA1005159, HEMBA1005315, HEMBA1005338, HEMBA1005410, HEMBA1005447, HEMBA1005500, HEMBA1005506, HEMBA1005583, HEMBA1005606, HEMBA1005755, HEMBA1005813, HEMBA1005909, HEMBA1005931, HEMBA1005934, HEMBA1005991, HEMBA1006002, HEMBA1006036, HEMBA1006067, HEMBA1006091, HEMBA1006142, HEMBA1006235, HEMBA1006283, HEMBA1006291, HEMBA1006293, HEMBA1006344, HEMBA1006492, HEMBA1006535, HEMBA1006569, HEMBA1006612, HEMBA1006676, HEMBA1006767, HEMBA1006877, HEMBA1006885, HEMBA1006926, HEMBA1006996, HEMBA1007018, HEMBA1007066, HEMBA1007080, HEMBA1007112, HEMBA1007178, HEMBA1007203, HEMBA1007243, HEMBB1000226, HEMBB1000240, HEMBB1000272, HEMBB1000638, HEMBB1000706, HEMBB1000826, HEMBB1001014, HEMBB1001112, HEMBB1001119, HEMBB1001317, HEMBB1002342, HEMBB1002371, HEMBB1002381, HEMBB1002387, HEMBB1002409, HEMBB1002457, HEMBB1002477, HEMBB1002489, HEMBB1002502, HEMBB1002510, HEMBB1002582, HEMBB1002596, MAMMA1000103, MAMMA1000155, MAMMA1000183, MAMMA1000198, MAMMA1000221, MAMMA1000241, MAMMA1000270,
MAMMA1000284, MAMMA1000309, MAMMA1000339, MAMMA1000348, MAMMA1000410, MAMMA1000421, MAMMA1000422, MAMMA1000429, MAMMA1000444, MAMMA1000478, MAMMA1000524, MAMMA1000605, MAMMA1000738, MAMMA1000761, MAMMA1000776, MAMMA1000778, MAMMA1000782, MAMMA1000802, MAMMA1000839, MAMMA1000843,

MAMMA1000908, MAMMA1000943, MAMMA1000998, MAMMA1001080, MAMMA1001105, MAMMA1001260, MAMMA1001324, MAMMA1001446, MAMMA1001627, MAMMA1001908, MAMMA1002084, MAMMA1002174, MAMMA1002360, MAMMA1002384, MAMMA1002650, MAMMA1002685, MAMMA1002796, MAMMA1002973, MAMMA1003004, MAMMA1003166, NT2RM1000018, NT2RM1000187, NT2RM1000244, NT2RM1000256, NT2RM1000260, NT2RM1000377, NT2RM1000388, NT2RM1000555, NT2RM1000661, NT2RM1000699, NT2RM1000770, NT2RM1000780, NT2RM1000800, NT2RM1000802, NT2RM1000894, NT2RM1000898, NT2RM1001003, NT2RM1001043, NT2RM1001044, NT2RM1001074, NT2RM1001139, NT2RM2000101, NT2RM2000368, NT2RM2000402, NT2RM2000407, NT2RM2000422, NT2RM2000577, NT2RM2000581, NT2RM2000612, NT2RM2000691, NT2RM2000714, NT2RM2000718, NT2RM2000837, NT2RM2001131, NT2RM2001152, NT2RM2001221, NT2RM2001238, NT2RM2001306, NT2RM2001420, NT2RM2001524, NT2RM2001582, NT2RM2001592, NT2RM2001632, NT2RM2001659, NT2RM2001671, NT2RM2001675, NT2RM2001696, NT2RM2001716, NT2RM2001803, NT2RM2001805, NT2RM2001903, NT2RM2001983, NT2RM4000085, NT2RM4000155, NT2RM4000229, NT2RM4000290, NT2RM4000324, NT2RM4000395, NT2RM4000433, NT2RM4000471, NT2RM4000531, NT2RM4000532, NT2RM4000616, NT2RM4000700, NT2RM4000741, NT2RM4000751, NT2RM4000778, NT2RM4000779, NT2RM4000848, NT2RM4000855, NT2RM4000887, NT2RM4000895, NT2RM4001032, NT2RM4001203, NT2RM4001316, NT2RM4001414, NT2RM4001483, NT2RM4001519, NT2RM4001522, NT2RM4001557, NT2RM4001565, NT2RM4001582, NT2RM4001629, NT2RM4001682, NT2RM4001776, NT2RM4001783, NT2RM4002075, NT2RM4002093, NT2RM4002109, NT2RM4002189, NT2RM4002256, NT2RM4002294, NT2RM4002373, NT2RM4002504, NT2RP1000035, NT2RP1000040, NT2RP1000086, NT2RP1000112, NT2RP1000163, NT2RP1000358, NT2RP1000409, NT2RP1000478, NT2RP1000481, NT2RP1000574, NT2RP1000577, NT2RP1000629, NT2RP1000630, NT2RP1000688, NT2RP1000733, NT2RP1000796, NT2RP1000916, NT2RP1000959, NT2RP1000966, NT2RP1001011, NT2RP1001033, NT2RP1001286, NT2RP1001302, NT2RP1001361, NT2RP1001457, NT2RP1001482, NT2RP2000054, NT2RP2000079, NT2RP2000091, NT2RP2000097, NT2RP2000161, NT2RP2000195, NT2RP2000232, NT2RP2000233, NT2RP2000248, NT2RP2000283, NT2RP2000422, NT2RP2000438, NT2RP2000448, NT2RP2000459, NT2RP2000731, NT2RP2000758, NT2RP2000764, NT2RP2000892, NT2RP2000943, NT2RP2001036, NT2RP2001070, NT2RP2001218, NT2RP2001467, NT2RP2001506, NT2RP2001520, NT2RP2001597, NT2RP2001634, NT2RP2001660, NT2RP2001762, NT2RP2001947, NT2RP2001991, NT2RP2002033, NT2RP2002041, NT2RP2002047, NT2RP2002066, NT2RP2002070, NT2RP2002193, NT2RP2002235, NT2RP2002252, NT2RP2002394, NT2RP2002537, NT2RP2002727, NT2RP2002736, NT2RP2002752, NT2RP2002753, NT2RP2002769, NT2RP2002778, NT2RP2002857, NT2RP2002928, NT2RP2002954, NT2RP2002980, NT2RP2002986, NT2RP2003121, NT2RP2003161, NT2RP2003272, NT2RP2003329, NT2RP2003339, NT2RP2003391, NT2RP2003393, NT2RP2003394, NT2RP2003401, NT2RP2003466, NT2RP2003513, NT2RP2003564, NT2RP2003581, NT2RP2003629, NT2RP2003727, NT2RP2003764, NT2RP2003793, NT2RP2003840, NT2RP2003885, NT2RP2004066, NT2RP2004187, NT2RP2004226, NT2RP2004242, NT2RP2004245, NT2RP2004316, NT2RP2004366, NT2RP2004396, NT2RP2004463, NT2RP2004512, NT2RP2004655, NT2RP2004736, NT2RP2004775, NT2RP2004791, NT2RP2004802, NT2RP2004841, NT2RP2004897, NT2RP2004933, NT2RP2004936, NT2RP2004961, NT2RP2004967, NT2RP2004982, NT2RP2005018, NT2RP2005038, NT2RP2005116, NT2RP2005140, NT2RP2005159, NT2RP2005162, NT2RP2005276, NT2RP2005288, NT2RP2005315, NT2RP2005344, NT2RP2005407, NT2RP2005453, NT2RP2005457, NT2RP2005476, NT2RP2005490, NT2RP2005525, NT2RP2005540, NT2RP2005549, NT2RP2005600, NT2RP2005675, NT2RP2005690, NT2RP2005712, NT2RP2005722, NT2RP2005723, NT2RP2005763, NT2RP2005812, NT2RP2005853, NT2RP2005859, NT2RP2005933, NT2RP2005980, NT2RP2006038, NT2RP2006043, NT2RP2006071, NT2RP2006098, NT2RP2006100, NT2RP2006103, NT2RP2006106, NT2RP2006261, NT2RP2006334, NT2RP2006464, NT2RP2006573, NT2RP3000255, NT2RP3000267, NT2RP3000341, NT2RP3000403, NT2RP3000526, NT2RP3000531, NT2RP3000584, NT2RP3000590, NT2RP3000685, NT2RP3000759, NT2RP3000847, NT2RP3000865, NT2RP3000875, NT2RP3000968, NT2RP3000994, NT2RP3001107, NT2RP3001116, NT2RP3001147, NT2RP3001150, NT2RP3001236, NT2RP3001307, NT2RP3001318, NT2RP3001399, NT2RP3001420, NT2RP3001426, NT2RP3001472, NT2RP3001497, NT2RP3001527, NT2RP3001607, NT2RP3001671, NT2RP3001690, NT2RP3001727, NT2RP3001915, NT2RP3001931, NT2RP3001944, NT2RP3001989, NT2RP3002147, NT2RP3002244, NT2RP3002330, NT2RP3002343, NT2RP3002501, NT2RP3002587, NT2RP3002602, NT2RP3002682, NT2RP3002687, NT2RP3002770, NT2RP3002785, NT2RP3002869, NT2RP3002877, NT2RP3002969, NT2RP3003145, NT2RP3003157, NT2RP3003212, NT2RP3003264, NT2RP3003278, NT2RP3003282, NT2RP3003290, NT2RP3003327, NT2RP3003344, NT2RP3003353, NT2RP3003491, NT2RP3003543, NT2RP3003665, NT2RP3003918, NT2RP3003932, NT2RP3004110, NT2RP3004155, NT2RP3004262, NT2RP3004334, NT2RP3004428, NT2RP3004466, NT2RP3004475, NT2RP3004504, NT2RP3004507, NT2RP3004527, NT2RP3004544, NT2RP3004572, NT2RP3004578, NT2RP4000109, NT2RP4000246, NT2RP4000360, NT2RP4000376, NT2RP4000398, NT2RP4000455, NT2RP4000518, NT2RP4000524, NT2RP4000878, NT2RP4000879, NT2RP4000955, NT2RP4000989, NT2RP4000997, NT2RP4001004, NT2RP4001010, NT2RP4001078, NT2RP4001122, NT2RP4001126, NT2RP4001149, NT2RP4001206, NT2RP4001219, NT2RP4001260, NT2RP4001339, NT2RP4001353, NT2RP4001414, NT2RP4001442, NT2RP4001447, NT2RP4001474, NT2RP4001551, NT2RP4001803, NT2RP4001893, NT2RP4001896, NT2RP4001946, NT2RP4001950, NT2RP4001966, NT2RP4002081, NT2RP4002408, NT2RP4002791, NT2RP4002888, NT2RP5003524, NT2RP5003534, OVARC1000013, OVARC1000035, OVARC1000087, OVARC1000113, OVARC1000139, OVARC1000145, OVARC1000209, OVARC1000212, OVARC1000408, OVARC1000427, OVARC1000466, OVARC1000496, OVARC1000533, OVARC1000576, OVARC1000640, OVARC1000649, OVARC1000682, OVARC1000689, OVARC1000771, OVARC1000885, OVARC1000890, OVARC1000936, OVARC1000964, OVARC1001065, OVARC1001068, OVARC1001072, OVARC1001107, OVARC1001113, OVARC1001154, OVARC1001169, OVARC1001176, OVARC1001372, OVARC1001391, OVARC1001453, OVARC1001476, OVARC1001480, OVARC1001547, OVARC1001555, OVARC1001610, OVARC1001711, OVARC1001713, OVARC1001726, OVARC1001766, OVARC1001767, OVARC1001768, OVARC1001805, OVARC1001809, OVARC1001828, OVARC1001846, OVARC1001901, OVARC1001943, OVARC1002138, OVARC1002156, PLACE1000005, PLACE1000007, PLACE1000040, PLACE1000048, PLACE1000133, PLACE1000214, PLACE1000347, PLACE1000374, PLACE1000424, PLACE1000492, PLACE1000547, PLACE1000564, PLACE1000599, PLACE1000653, PLACE1000706, PLACE1000748, PLACE1000749, PLACE1000755, PLACE1000841, PLACE1000849, PLACE1000856, PLACE1000931, PLACE1000987, PLACE1001015, PLACE1001024, PLACE1001054, PLACE1001062, PLACE1001118, PLACE1001168, PLACE1001238, PLACE1001377, PLACE1001383, PLACE1001384, PLACE1001399, PLACE1001440, PLACE1001503, PLACE1001545, PLACE1001611, PLACE1001640, PLACE1001691, PLACE1001705, PLACE1001740, PLACE1001817, PLACE1001844, PLACE1001845, PLACE1001869, PLACE1001897, PLACE1002004, PLACE1002073, PLACE1002140, PLACE1002157, PLACE1002171, PLACE1002205, PLACE1002213, PLACE1002474, PLACE1002500, PLACE1002529, PLACE1002537, PLACE1002598, PLACE1002655, PLACE1002908, PLACE1003045, PLACE1003136, PLACE1003174, PLACE1003258, PLACE1003296, PLACE1003334, PLACE1003342, PLACE1003361, PLACE1003366, PLACE1003369, PLACE1003420, PLACE1003454, PLACE1003553, PLACE1003566, PLACE1003583, PLACE1003669, PLACE1003711, PLACE1003723, PLACE1003738, PLACE1003760, PLACE1003762, PLACE1003771, PLACE1003783, PLACE1003850, PLACE1003858, PLACE1003915, PLACE1004128, PLACE1004161, PLACE1004183, PLACE1004197, PLACE1004302, PLACE1004358, PLACE1004437, PLACE1004460, PLACE1004471, PLACE1004506, PLACE1004518, PLACE1004646, PLACE1004722, PLACE1004793, PLACE1004804, PLACE1004838, PLACE1004868, PLACE1004900, PLACE1004902, PLACE1004969, PLACE1004979, PLACE1005086, PLACE1005162, PLACE1005243, PLACE1005266, PLACE1005313, PLACE1005584, PLACE1005698, PLACE1005727, PLACE1005739, PLACE1005851, PLACE1005925, PLACE1006196, PLACE1006248, PLACE1006262, PLACE1006335, PLACE1006360, PLACE1006385, PLACE1006412, PLACE1006414, PLACE1006445, PLACE1006598, PLACE1006629, PLACE1006640, PLACE1006792, PLACE1006815, PLACE1006901, PLACE1006917, PLACE1006962, PLACE1007021, PLACE1007045, PLACE1007112, PLACE1007226, PLACE1007243, PLACE1007454, PLACE1007547, PLACE1007598, PLACE1007618, PLACE1007645, PLACE1007649, PLACE1007706, PLACE1007725, PLACE1007746, PLACE1007843, PLACE1007877, PLACE1007954, PLACE1007969, PLACE1008002, PLACE1008095, PLACE1008111, PLACE1008132, PLACE1008273, PLACE1008275, PLACE1008331, PLACE1008356, PLACE1008368, PLACE1008402, PLACE1008603, PLACE1008650, PLACE1008696, PLACE1008757, PLACE1008807, PLACE1008867, PLACE1008941, PLACE1009020, PLACE1009039, PLACE1009158, PLACE1009172, PLACE1009190, PLACE1009230, PLACE1009319, PLACE1009338, PLACE1009375, PLACE1009434, PLACE1009613, PLACE1009637, PLACE1009659, PLACE1009763, PLACE1009845, PLACE1009921, PLACE1009971, PLACE1009992, PLACE1010023, PLACE1010053, PLACE1010069, PLACE1010074, PLACE1010181, PLACE1010202, PLACE1010231, PLACE1010270, PLACE1010274, PLACE1010321, PLACE1010329, PLACE1010492, PLACE1010522, PLACE1010616, PLACE1010624, PLACE1010631, PLACE1010786, PLACE1011032, PLACE1011114, PLACE1011221, PLACE1011325, PLACE1011520, PLACE1011635, PLACE1011649, PLACE1011682, PLACE1011875, PLACE1011896, PLACE1011964, PLACE1012031, PLACE2000015, PLACE2000021, PLACE2000047, PLACE2000072, PLACE2000097, PLACE2000136, PLACE2000246, PLACE2000302, PLACE2000379, PLACE2000394, PLACE2000425, PLACE2000427, PLACE2000477, PLACE3000009, PLACE3000070, PLACE3000142, PLACE3000145, PLACE3000148, PLACE3000155, PLACE3000169, PLACE3000208, PLACE3000230, PLACE3000322, PLACE3000331, PLACE3000352, PLACE3000401, PLACE3000413, PLACE3000425, PLACE3000477, PLACE4000009, PLACE4000049, PLACE4000089, PLACE4000100, PLACE4000247, PLACE4000250, PLACE4000252, PLACE4000300, PLACE4000344, PLACE4000367, PLACE4000465, PLACE4000489, PLACE4000638, SKNMC1000013, THYRO1000017, THYRO1000026, THYRO1000034, THYRO1000072, THYRO1000132, THYRO1000173, THYRO1000190, THYRO1000197, THYRO1000221, THYRO1000253, THYRO1000270, THYRO1000279, THYRO1000327, THYRO1000394, THYRO1000438, THYRO1000558, THYRO1000569, THYRO1000585, THYRO1000596, THYRO1000625, THYRO1000637, THYRO1000676, THYRO1000734, THYRO1000777, THYRO1000783, THYRO1000805, THYRO1000843, THYRO1000934, THYRO1001033, THYRO1001347, THYRO1001405, THYRO1001411, THYRO1001534, THYRO1001573, THYRO1001584, THYRO1001602, THYRO1001605, THYRO1001772, THYRO1001854, VESEN1000122, Y79AA1000037, Y79AA1000065, Y79AA1000181, Y79AA1000231, Y79AA1000349, Y79AA1000355, Y79AA1000368, Y79AA1000538, Y79AA1000782, Y79AA1001023, Y79AA1001145, Y79AA1001391, Y79AA1001541, Y79AA1001585, Y79AA1001705, Y79AA1001781, Y79AA1001923, Y79AA1001963, Y79AA1002125, Y79AA1002229, Y79AA1002407, Y79AA1002487

These clones are associated with ultraviolet radiation damage.

A survey of genes of which expression levels are varied in response to the stimulation for inducing cell differentiation (stimulation using retinoic acid (RA) or using RA/inhibitor (inhibitor for cell division)) in culture cells of neural strain, NT2, revealed the following clones with elevated expression levels in the presence of RA:
HEMBA1000005, HEMBA1000042, HEMBA1000046, HEMBA1000076, HEMBA1000111, HEMBA1000141, HEMBA1000150, HEMBA1000185, HEMBA1000282, HEMBA1000304, HEMBA1000307, HEMBA1000338, HEMBA1000357, HEMBA1000376, HEMBA1000387, HEMBA1000392, HEMBA1000428, HEMBA1000456, HEMBA1000459, HEMBA1000469, HEMBA1000504, HEMBA1000508, HEMBA1000519, HEMBA1000540, HEMBA1000545, HEMBA1000557, HEMBA1000563, HEMBA1000568, HEMBA1000575, HEMBA1000588, HEMBA1000592, HEMBA1000604, HEMBA1000622, HEMBA1000655, HEMBA1000673, HEMBA1000682, HEMBA1000726, HEMBA1000727, HEMBA1000749, HEMBA1000769, HEMBA1000774, HEMBA1000791, HEMBA1000822, HEMBA1000872, HEMBA1000876, HEMBA1000910, HEMBA1000942, HEMBA1000943, HEMBA1000960, HEMBA1000972, HEMBA1000974, HEMBA1000991, HEMBA1001008, HEMBA1001020, HEMBA1001043, HEMBA1001051, HEMBA1001060, HEMBA1001071, HEMBA1001077, HEMBA1001085, HEMBA1001094, HEMBA1001109, HEMBA1001121, HEMBA1001122, HEMBA1001140, HEMBA1001172, HEMBA1001226, HEMBA1001235, HEMBA1001265, HEMBA1001281, HEMBA1001294, HEMBA1001299, HEMBA1001319, HEMBA1001323, HEMBA1001330, HEMBA1001351, HEMBA1001361, HEMBA1001377, HEMBA1001388, HEMBA1001391, HEMBA1001398, HEMBA1001432, HEMBA1001435, HEMBA1001442, HEMBA1001454, HEMBA1001455, HEMBA1001497, HEMBA1001517, HEMBA1001569, HEMBA1001570, HEMBA1001581, HEMBA1001585, HEMBA1001620, HEMBA1001711, HEMBA1001718, HEMBA1001723, HEMBA1001761, HEMBA1001815, HEMBA1001819, HEMBA1001861, HEMBA1001864, HEMBA1001869, HEMBA1001888, HEMBA1001915, HEMBA1001918, HEMBA1001940, HEMBA1001964, HEMBA1001967, HEMBA1001979, HEMBA1001987, HEMBA1001991, HEMBA1002008, HEMBA1002022, HEMBA1002039, HEMBA1002049, HEMBA1002084, HEMBA1002102, HEMBA1002113, HEMBA1002144, HEMBA1002160, HEMBA1002162, HEMBA1002185, HEMBA1002212, HEMBA1002226, HEMBA1002229, HEMBA1002267, HEMBA1002270, HEMBA1002337, HEMBA1002381, HEMBA1002458, HEMBA1002477, HEMBA1002508, HEMBA1002558, HEMBA1002561, HEMBA1002583, HEMBA1002590, HEMBA1002628, HEMBA1002645, HEMBA1002661, HEMBA1002678, HEMBA1002712, HEMBA1002728, HEMBA1002780, HEMBA1002850, HEMBA1002886, HEMBA1002934, HEMBA1002935, HEMBA1002939, HEMBA1002951, HEMBA1002968, HEMBA1002970, HEMBA1002973, HEMBA1002999, HEMBA1003021, HEMBA1003033, HEMBA1003034, HEMBA1003064, HEMBA1003067, HEMBA1003078, HEMBA1003086, HEMBA1003096, HEMBA1003129, HEMBA1003142, HEMBA1003148, HEMBA1003166, HEMBA1003175, HEMBA1003197, HEMBA1003199, HEMBA1003202, HEMBA1003204, HEMBA1003212, HEMBA1003235, HEMBA1003250, HEMBA1003273, HEMBA1003276, HEMBA1003278, HEMBA1003291, HEMBA1003309, HEMBA1003322, HEMBA1003328, HEMBA1003348, HEMBA1003369, HEMBA1003376, HEMBA1003384, HEMBA1003395, HEMBA1003463, HEMBA1003480, HEMBA1003531, HEMBA1003548, HEMBA1003591, HEMBA1003595, HEMBA1003597, HEMBA1003617, HEMBA1003621, HEMBA1003622, HEMBA1003637, HEMBA1003640, HEMBA1003645, HEMBA1003646, HEMBA1003656, HEMBA1003679, HEMBA1003692, HEMBA1003715, HEMBA1003720, HEMBA1003725, HEMBA1003729, HEMBA1003758, HEMBA1003803, HEMBA1003805, HEMBA1003836, HEMBA1003838, HEMBA1003879, HEMBA1003885, HEMBA1003893, HEMBA1003908, HEMBA1003937, HEMBA1003942, HEMBA1003953, HEMBA1003958, HEMBA1003959, HEMBA1003978, HEMBA1003987, HEMBA1003989, HEMBA1004000, HEMBA1004011, HEMBA1004012, HEMBA1004015, HEMBA1004024, HEMBA1004049, HEMBA1004056, HEMBA1004111, HEMBA1004132, HEMBA1004143, HEMBA1004164, HEMBA1004199, HEMBA1004200, HEMBA1004207, HEMBA1004225, HEMBA1004246, HEMBA1004248, HEMBA1004267, HEMBA1004289, HEMBA1004312, HEMBA1004323, HEMBA1004335, HEMBA1004353, HEMBA1004354, HEMBA1004356, HEMBA1004366, HEMBA1004396, HEMBA1004405, HEMBA1004429, HEMBA1004433, HEMBA1004460, HEMBA1004499, HEMBA1004502, HEMBA1004506, HEMBA1004534, HEMBA1004538, HEMBA1004573, HEMBA1004577, HEMBA1004586, HEMBA1004610, HEMBA1004629, HEMBA1004666, HEMBA1004669, HEMBA1004672, HEMBA1004709, HEMBA1004733, HEMBA1004736, HEMBA1004748, HEMBA1004751, HEMBA1004758, HEMBA1004763, HEMBA1004768, HEMBA1004770, HEMBA1004778, HEMBA1004803, HEMBA1004820, HEMBA1004847, HEMBA1004863, HEMBA1004880, HEMBA1004909, HEMBA1004918, HEMBA1004923, HEMBA1004930, HEMBA1004934, HEMBA1004944, HEMBA1004954, HEMBA1004980, HEMBA1005035, HEMBA1005039, HEMBA1005075, HEMBA1005079, HEMBA1005113, HEMBA1005123, HEMBA1005133, HEMBA1005149, HEMBA1005152, HEMBA1005219, HEMBA1005223, HEMBA1005232, HEMBA1005251, HEMBA1005274, HEMBA1005275, HEMBA1005304, HEMBA1005311, HEMBA1005314, HEMBA1005359, HEMBA1005367, HEMBA1005374, HEMBA1005410, HEMBA1005411, HEMBA1005423, HEMBA1005426, HEMBA1005443, HEMBA1005447, HEMBA1005474, HEMBA1005508, HEMBA1005511, HEMBA1005520, HEMBA1005526, HEMBA1005548, HEMBA1005552, HEMBA1005576, HEMBA1005581, HEMBA1005582, HEMBA1005583, HEMBA1005588, HEMBA1005595, HEMBA1005609, HEMBA1005616, HEMBA1005627, HEMBA1005631, HEMBA1005666, HEMBA1005670, HEMBA1005679, HEMBA1005699, HEMBA1005705, HEMBA1005765, HEMBA1005780, HEMBA1005822, HEMBA1005829, HEMBA1005834, HEMBA1005853, HEMBA1005891, HEMBA1005894, HEMBA1005911, HEMBA1005921, HEMBA1005991, HEMBA1005999, HEMBA1006036, HEMBA1006042, HEMBA1006100, HEMBA1006138, HEMBA1006142, HEMBA1006268, HEMBA1006278, HEMBA1006328, HEMBA1006344, HEMBA1006359, HEMBA1006398, HEMBA1006416, HEMBA1006419, HEMBA1006421, HEMBA1006426, HEMBA1006438, HEMBA1006483, HEMBA1006485, HEMBA1006502, HEMBA1006507, HEMBA1006546, HEMBA1006559, HEMBA1006562, HEMBA1006579, HEMBA1006597, HEMBA1006612, HEMBA1006617, HEMBA1006631, HEMBA1006635, HEMBA1006652, HEMBA1006695, HEMBA1006744, HEMBA1006754, HEMBA1006779, HEMBA1006780, HEMBA1006795, HEMBA1006821, HEMBA1006865, HEMBA1006926, HEMBA1006973, HEMBA1007017, HEMBA1007052, HEMBA1007080, HEMBA1007085, HEMBA1007113, HEMBA1007121, HEMBA1007147, HEMBA1007149, HEMBA1007206, HEMBA1007224, HEMBA1007256, HEMBA1007267, HEMBA1007288, HEMBA1007327, HEMBA1007341, HEMBA1007347,
HEMBB1000005, HEMBB1000008, HEMBB1000018, HEMBB1000024, HEMBB1000030, HEMBB1000039, HEMBB1000048, HEMBB1000054, HEMBB1000059, HEMBB1000083, HEMBB1000089, HEMBB1000099, HEMBB1000113, HEMBB1000141, HEMBB1000144, HEMBB1000173, HEMBB1000175, HEMBB1000215, HEMBB1000218, HEMBB1000258, HEMBB1000264, HEMBB1000272, HEMBB1000307, HEMBB1000338, HEMBB1000339, HEMBB1000343, HEMBB1000354, HEMBB1000374, HEMBB1000376, HEMBB1000420, HEMBB1000434, HEMBB1000441, HEMBB1000449, HEMBB1000490, HEMBB1000491, HEMBB1000523, HEMBB1000530, HEMBB1000550, HEMBB1000554, HEMBB1000573, HEMBB1000575, HEMBB1000593, HEMBB1000598, HEMBB1000638, HEMBB1000643, HEMBB1000649, HEMBB1000671, HEMBB1000684, HEMBB1000709, HEMBB1000726, HEMBB1000770, HEMBB1000774, HEMBB1000790, HEMBB1000835, HEMBB1000840, HEMBB1000848, HEMBB1000870, HEMBB1000883, HEMBB1000887, HEMBB1000890, HEMBB1000910, HEMBB1000917, HEMBB1000959, HEMBB1000985, HEMBB1000996, HEMBB1001004, HEMBB1001020, HEMBB1001024, HEMBB1001037, HEMBB1001060, HEMBB1001096, HEMBB1001114, HEMBB1001117, HEMBB1001142, HEMBB1001153, HEMBB1001177, HEMBB1001210, HEMBB1001218, HEMBB1001242, HEMBB1001267, HEMBB1001289, HEMBB1001346, HEMBB1001348, HEMBB1001364, HEMBB1001366, HEMBB1001369, HEMBB1001380, HEMBB1001387, HEMBB1001394, HEMBB1001426, HEMBB1001436, HEMBB1001454, HEMBB1001463, HEMBB1001521, HEMBB1001535, HEMBB1001537, HEMBB1001555, HEMBB1001585, HEMBB1001603, HEMBB1001619, HEMBB1001668, HEMBB1001685, HEMBB1001695, HEMBB1001704, HEMBB1001706, HEMBB1001707, HEMBB1001735, HEMBB1001747, HEMBB1001749, HEMBB1001760, HEMBB1001812, HEMBB1001816, HEMBB1001831, HEMBB1001836, HEMBB1001850, HEMBB1001863, HEMBB1001867, HEMBB1001908, HEMBB1001910, HEMBB1001911, HEMBB1001915, HEMBB1001921, HEMBB1001930, HEMBB1001944, HEMBB1001952, HEMBB1001957, HEMBB1001967, HEMBB1001988, HEMBB1001997, HEMBB1002005, HEMBB1002042, HEMBB1002043, HEMBB1002045, HEMBB1002049, HEMBB1002069, HEMBB1002094, HEMBB1002139, HEMBB1002152, HEMBB1002189, HEMBB1002217, HEMBB1002232, HEMBB1002249, HEMBB1002254, HEMBB1002266, HEMBB1002280, HEMBB1002306, HEMBB1002364, HEMBB1002371, HEMBB1002409, HEMBB1002425, HEMBB1002453, HEMBB1002492, HEMBB1002520, HEMBB1002556, HEMBB1002582, HEMBB1002590, HEMBB1002607, HEMBB1002614, HEMBB1002617, HEMBB1002623, HEMBB1002635, HEMBB1002677, HEMBB1002683, HEMBB1002684, HEMBB1002697, HEMBB1002699, HEMBB1002705,
MAMMA1000009, MAMMA1000043, MAMMA1000045, MAMMA1000084, MAMMA1000103, MAMMA1000134, MAMMA1000139, MAMMA1000143, MAMMA1000171, MAMMA1000241, MAMMA1000251, MAMMA1000254, MAMMA1000257, MAMMA1000264, MAMMA1000266, MAMMA1000270, MAMMA1000279, MAMMA1000287, MAMMA1000340, MAMMA1000348, MAMMA1000360, MAMMA1000361, MAMMA1000372, MAMMA1000385, MAMMA1000402, MAMMA1000424, MAMMA1000478, MAMMA1000500, MAMMA1000522, MAMMA1000524, MAMMA1000565, MAMMA1000567, MAMMA1000576, MAMMA1000585, MAMMA1000594, MAMMA1000597, MAMMA1000605, MAMMA1000616, MAMMA1000643, MAMMA1000664, MAMMA1000669, MAMMA1000696, MAMMA1000718, MAMMA1000723, MAMMA1000731, MAMMA1000744, MAMMA1000746, MAMMA1000752, MAMMA1000760, MAMMA1000761, MAMMA1000776, MAMMA1000802, MAMMA1000824, MAMMA1000839, MAMMA1000880, MAMMA1000905, MAMMA1000931, MAMMA1000940, MAMMA1000941, MAMMA1000943, MAMMA1000957, MAMMA1000962, MAMMA1000968, MAMMA1000987, MAMMA1000998, MAMMA1001003, MAMMA1001035, MAMMA1001038, MAMMA1001067, MAMMA1001078, MAMMA1001092, MAMMA1001126, MAMMA1001133, MAMMA1001139, MAMMA1001161, MAMMA1001186, MAMMA1001202, MAMMA1001206, MAMMA1001220, MAMMA1001243, MAMMA1001268, MAMMA1001274, MAMMA1001296, MAMMA1001298, MAMMA1001305, MAMMA1001343, MAMMA1001383, MAMMA1001397, MAMMA1001419, MAMMA1001420, MAMMA1001435, MAMMA1001442, MAMMA1001663, MAMMA1001670, MAMMA1001683, MAMMA1001692, MAMMA1001740, MAMMA1001745, MAMMA1001754, MAMMA1001768, MAMMA1001769, MAMMA1001783, MAMMA1001785, MAMMA1001806, MAMMA1001890, MAMMA1001907, MAMMA1001969, MAMMA1002009, MAMMA1002032, MAMMA1002056, MAMMA1002058, MAMMA1002125, MAMMA1002132, MAMMA1002140, MAMMA1002155, MAMMA1002158, MAMMA1002174, MAMMA1002198, MAMMA1002230, MAMMA1002293, MAMMA1002310, MAMMA1002311, MAMMA1002322, MAMMA1002339, MAMMA1002359, MAMMA1002384, MAMMA1002428, MAMMA1002434, MAMMA1002475, MAMMA1002494, MAMMA1002545, MAMMA1002573, MAMMA1002603, MAMMA1002619, MAMMA1002622, MAMMA1002623, MAMMA1002625, MAMMA1002662, MAMMA1002698, MAMMA1002701, MAMMA1002708, MAMMA1002721, MAMMA1002744, MAMMA1002764, MAMMA1002769, MAMMA1002820, MAMMA1002830, MAMMA1002833, MAMMA1002868, MAMMA1002871, MAMMA1002892, MAMMA1002895, MAMMA1002908, MAMMA1002909, MAMMA1002930, MAMMA1002941, MAMMA1002964, MAMMA1002970, MAMMA1002973, MAMMA1002987, MAMMA1003004, MAMMA1003040, MAMMA1003066, MAMMA1003089, NT2RM1000039, NT2RM1000672, NT2RM1000691, NT2RM1000725, NT2RM1000781, NT2RM1000829, NT2RM1000882, NT2RM1001105, NT2RM2000013, NT2RM2000374, NT2RM2000420, NT2RM2000452, NT2RM2000504, NT2RM2000609, NT2RM2000635, NT2RM2000735, NT2RM2000795, NT2RM2000821, NT2RM2001035, NT2RM2001105, NT2RM2001141, NT2RM2001177, NT2RM2001196, NT2RM2001306, NT2RM2001524, NT2RM2001582, NT2RM2001588, NT2RM2001592, NT2RM2001605, NT2RM2001632, NT2RM2001637, NT2RM2001648, NT2RM2001668, NT2RM2001671, NT2RM2001675, NT2RM2001681, NT2RM2001695, NT2RM2001699, NT2RM2001706, NT2RM2001723, NT2RM2001753, NT2RM2001771, NT2RM2001797, NT2RM2001840, NT2RM2001879, NT2RM2002049, NT2RM2002091, NT2RM4000265, NT2RM4000290, NT2RM4000324, NT2RM4000327, NT2RM4000368, NT2RM4000421, NT2RM4000425, NT2RM4000471, NT2RM4000486, NT2RM4000531, NT2RM4000532, NT2RM4000712, NT2RM4000751, NT2RM4000787, NT2RM4000790, NT2RM4000820, NT2RM4000852, NT2RM4000855, NT2RM4000895, NT2RM4000996, NT2RM4001002, NT2RM4001032, NT2RM4001047, NT2RM4001116, NT2RM4001151, NT2RM4001200, NT2RM4001313, NT2RM4001371, NT2RM4001410, NT2RM4001411, NT2RM4001437, NT2RM4001454, NT2RM4001483, NT2RM4001489, NT2RM4001522, NT2RM4001557, NT2RM4001566, NT2RM4001582, NT2RM4001650, NT2RM4001682, NT2RM4001746, NT2RM4001828, NT2RM4001842, NT2RM4001865, NT2RM4001922, NT2RM4001953, NT2RM4001979, NT2RM4002013, NT2RM4002067, NT2RM4002075, NT2RM4002093, NT2RM4002128, NT2RM4002140, NT2RM4002174, NT2RM4002205, NT2RM4002301, NT2RM4002323, NT2RM4002383, NT2RM4002409, NT2RM4002457, NT2RM4002499, NT2RM4002504, NT2RM4002558, NT2RM4002565, NT2RM4002594,
NT2RP1000018, NT2RP1000112, NT2RP1000124, NT2RP1000130, NT2RP1000170, NT2RP1000202, NT2RP1000259, NT2RP1000418, NT2RP1000493, NT2RP1000547, NT2RP1000574, NT2RP1000577, NT2RP1000630, NT2RP1000730, NT2RP1000782, NT2RP1000846, NT2RP1000856, NT2RP1000902, NT2RP1000943, NT2RP1000944, NT2RP1000988, NT2RP1001011, NT2RP1001013, NT2RP1001033, NT2RP1001173, NT2RP1001177, NT2RP1001185, NT2RP1001199, NT2RP1001286, NT2RP1001449, NT2RP1001475, NT2RP1001546, NT2RP2000032, NT2RP2000079, NT2RP2000091, NT2RP2000097, NT2RP2000120, NT2RP2000157, NT2RP2000195, NT2RP2000208, NT2RP2000224, NT2RP2000248, NT2RP2000257, NT2RP2000270, NT2RP2000274, NT2RP2000283, NT2RP2000288, NT2RP2000297, NT2RP2000298, NT2RP2000329, NT2RP2000369, NT2RP2000498, NT2RP2000634, NT2RP2000644, NT2RP2000656, NT2RP2000678, NT2RP2000715, NT2RP2000809, NT2RP2000932, NT2RP2000970, NT2RP2000987, NT2RP2001036, NT2RP2001081, NT2RP2001119, NT2RP2001127, NT2RP2001173, NT2RP2001268, NT2RP2001277, NT2RP2001290, NT2RP2001328, NT2RP2001366, NT2RP2001394, NT2RP2001420, NT2RP2001423, NT2RP2001427, NT2RP2001436, NT2RP2001440, NT2RP2001467, NT2RP2001506, NT2RP2001526, NT2RP2001601, NT2RP2001660, NT2RP2001678, NT2RP2001720, NT2RP2001861, NT2RP2001869, NT2RP2001876, NT2RP2001907, NT2RP2001926, NT2RP2001985, NT2RP2001991, NT2RP2002033, NT2RP2002041, NT2RP2002046, NT2RP2002078, NT2RP2002124, NT2RP2002193, NT2RP2002208, NT2RP2002256, NT2RP2002312, NT2RP2002316, NT2RP2002325, NT2RP2002333, NT2RP2002426, NT2RP2002457, NT2RP2002475, NT2RP2002520, NT2RP2002537, NT2RP2002595, NT2RP2002621, NT2RP2002672, NT2RP2002701, NT2RP2002706, NT2RP2002741, NT2RP2002750, NT2RP2002752, NT2RP2002769, NT2RP2002800, NT2RP2002925, NT2RP2002928, NT2RP2002959, NT2RP2002980, NT2RP2002987, NT2RP2003000, NT2RP2003034, NT2RP2003073, NT2RP2003099, NT2RP2003117, NT2RP2003129, NT2RP2003137, NT2RP2003157, NT2RP2003161, NT2RP2003165, NT2RP2003206, NT2RP2003230, NT2RP2003237, NT2RP2003243, NT2RP2003265, NT2RP2003272, NT2RP2003280, NT2RP2003293, NT2RP2003297, NT2RP2003339, NT2RP2003393, NT2RP2003394, NT2RP2003445, NT2RP2003456, NT2RP2003533, NT2RP2003559, NT2RP2003564, NT2RP2003596, NT2RP2003629, NT2RP2003691, NT2RP2003702, NT2RP2003760, NT2RP2003825, NT2RP2003859, NT2RP2003871, NT2RP2003976, NT2RP2003986, NT2RP2003988, NT2RP2004014, NT2RP2004081, NT2RP2004152, NT2RP2004187, NT2RP2004232, NT2RP2004239, NT2RP2004240, NT2RP2004321, NT2RP2004339, NT2RP2004400, NT2RP2004476, NT2RP2004538, NT2RP2004551, NT2RP2004580, NT2RP2004602, NT2RP2004675, NT2RP2004681, NT2RP2004689, NT2RP2004709, NT2RP2004710, NT2RP2004736, NT2RP2004743, NT2RP2004767, NT2RP2004775, NT2RP2004802, NT2RP2004861, NT2RP2004933, NT2RP2004961, NT2RP2004962, NT2RP2004967, NT2RP2004982, NT2RP2004985, NT2RP2005003, NT2RP2005038, NT2RP2005227, NT2RP2005276, NT2RP2005287, NT2RP2005289, NT2RP2005315, NT2RP2005436, NT2RP2005441, NT2RP2005453, NT2RP2005465, NT2RP2005476, NT2RP2005496, NT2RP2005509, NT2RP2005525, NT2RP2005540, NT2RP2005555, NT2RP2005581, NT2RP2005669, NT2RP2005719, NT2RP2005722, NT2RP2005767, NT2RP2005773, NT2RP2005853, NT2RP2005886, NT2RP2005901, NT2RP2005942, NT2RP2005980, NT2RP2006023, NT2RP2006043, NT2RP2006071, NT2RP2006166, NT2RP2006196, NT2RP2006237, NT2RP2006312, NT2RP2006320, NT2RP2006365, NT2RP2006393, NT2RP2006436, NT2RP2006441, NT2RP2006467, NT2RP2006554, NT2RP2006565, NT2RP2006598,
NT2RP3000002, NT2RP3000050, NT2RP3000072, NT2RP3000109, NT2RP3000134, NT2RP3000197, NT2RP3000341, NT2RP3000359, NT2RP3000366, NT2RP3000403, NT2RP3000418, NT2RP3000433, NT2RP3000441, NT2RP3000526, NT2RP3000531, NT2RP3000542, NT2RP3000561, NT2RP3000562, NT2RP3000603, NT2RP3000605, NT2RP3000644, NT2RP3000759, NT2RP3000815, NT2RP3000825, NT2RP3000836, NT2RP3000847, NT2RP3000850, NT2RP3000865, NT2RP3000901, NT2RP3000968, NT2RP3001007, NT2RP3001057, NT2RP3001120, NT2RP3001126, NT2RP3001140, NT2RP3001214, NT2RP3001216, NT2RP3001236, NT2RP3001245, NT2RP3001253, NT2RP3001260, NT2RP3001268, NT2RP3001274, NT2RP3001281, NT2RP3001297, NT2RP3001355, NT2RP3001356, NT2RP3001383, NT2RP3001396, NT2RP3001407, NT2RP3001420, NT2RP3001428, NT2RP3001449, NT2RP3001453, NT2RP3001472, NT2RP3001490, NT2RP3001495, NT2RP3001497, NT2RP3001527, NT2RP3001554, NT2RP3001589, NT2RP3001642, NT2RP3001679, NT2RP3001688, NT2RP3001712, NT2RP3001752, NT2RP3001782, NT2RP3001799, NT2RP3001844, NT2RP3001926, NT2RP3001989, NT2RP3002002, NT2RP3002004, NT2RP3002033, NT2RP3002056, NT2RP3002062, NT2RP3002102, NT2RP3002142, NT2RP3002173, NT2RP3002248, NT2RP3002304, NT2RP3002343, NT2RP3002484, NT2RP3002529, NT2RP3002545, NT2RP3002549, NT2RP3002682, NT2RP3002687, NT2RP3002713, NT2RP3002876, NT2RP3002877, NT2RP3002972, NT2RP3003032, NT2RP3003133, NT2RP3003139, NT2RP3003145, NT2RP3003157, NT2RP3003193, NT2RP3003204, NT2RP3003210, NT2RP3003212, NT2RP3003251, NT2RP3003264, NT2RP3003282, NT2RP3003290, NT2RP3003301, NT2RP3003313, NT2RP3003346, NT2RP3003403, NT2RP3003500, NT2RP3003543, NT2RP3003576, NT2RP3003672, NT2RP3003828, NT2RP3003831, NT2RP3003992, NT2RP3004041, NT2RP3004093, NT2RP3004155, NT2RP3004209, NT2RP3004215, NT2RP3004246, NT2RP3004332, NT2RP3004348, NT2RP3004349, NT2RP3004470, NT2RP3004472, NT2RP3004480, NT2RP3004490, NT2RP3004503, NT2RP3004566, NT2RP3004569, NT2RP3004572, NT2RP4000035, NT2RP4000147, NT2RP4000167, NT2RP4000212, NT2RP4000214, NT2RP4000259, NT2RP4000360, NT2RP4000381, NT2RP4000398, NT2RP4000424, NT2RP4000448, NT2RP4000455, NT2RP4000517, NT2RP4000519, NT2RP4000524, NT2RP4000614, NT2RP4000728, NT2RP4000737, NT2RP4000817, NT2RP4000865, NT2RP4001079, NT2RP4001122, NT2RP4001150, NT2RP4001174, NT2RP4001274, NT2RP4001353, NT2RP4001447, NT2RP4001507, NT2RP4001547, NT2RP4001677, NT2RP4001679, NT2RP4001730, NT2RP4001803, NT2RP4001889, NT2RP4001946, NT2RP4001953, NT2RP4002018, NT2RP4002071, NT2RP4002791, NT2RP5003461,
OVARC1000058, OVARC1000091, OVARC1000092, OVARC1000113, OVARC1000114, OVARC1000145, OVARC1000168, OVARC1000198, OVARC1000321, OVARC1000335, OVARC1000384, OVARC1000408, OVARC1000414, OVARC1000442, OVARC1000443, OVARC1000486, OVARC1000520, OVARC1000526, OVARC1000556, OVARC1000557, OVARC1000573, OVARC1000578, OVARC1000588, OVARC1000622, OVARC1000679, OVARC1000682, OVARC1000700, OVARC1000703, OVARC1000746, OVARC1000769, OVARC1000800, OVARC1000846, OVARC1000862, OVARC1000885, OVARC1000924, OVARC1000936, OVARC 1000959, OVARC1000960, OVARC1000971, OVARC1000984, OVARC1000996, OVARC1000999, OVARC1001000, OVARC1001004, OVARC1001032, OVARC1001034, OVARC1001040, OVARC1001044, OVARC1001092, OVARC1001117, OVARC1001118, OVARC1001154, OVARC1001161, OVARC1001170, OVARC1001173, OVARC1001200, OVARC1001240, OVARC1001268, OVARC1001271, OVARC1001329, OVARC1001341, OVARC1001344, OVARC1001376, OVARC1001381, OVARC1001436, OVARC1001476, OVARC1001489, OVARC1001525, OVARC1001542, OVARC1001547, OVARC1001668, OVARC1001745, OVARC1001767, OVARC1001802, OVARC1001812, OVARC1001820, OVARC1001873, OVARC1001883, OVARC1001949, OVARC1001987, OVARC1001989, OVARC1002044, OVARC1002082, OVARC1002107, OVARC1002138, OVARC1002165,
PLACE1000014, PLACE1000031, PLACE1000040, PLACE1000048, PLACE1000078, PLACE1000184, PLACE1000246, PLACE1000292, PLACE1000332, PLACE1000347, PLACE1000424, PLACE1000435, PLACE1000444, PLACE1000540, PLACE1000547, PLACE1000562, PLACE1000583, PLACE1000599, PLACE1000706, PLACE1000712, PLACE1000755, PLACE1000793, PLACE1000798, PLACE1000979, PLACE1001000, PLACE1001010, PLACE1001062, PLACE1001088, PLACE1001118, PLACE1001136, PLACE1001238, PLACE1001257, PLACE1001280, PLACE1001294, PLACE1001304, PLACE1001311, PLACE1001323, PLACE1001366, PLACE1001384, PLACE1001395, PLACE1001399, PLACE1001456, PLACE1001484, PLACE1001503, PLACE1001570, PLACE1001603, PLACE1001608, PLACE1001610, PLACE1001632, PLACE1001634, PLACE1001691, PLACE1001720, PLACE1001746, PLACE1001760, PLACE1001821, PLACE1001844, PLACE1001845, PLACE1001897, PLACE1001912, PLACE1001989, PLACE1002004, PLACE1002066, PLACE1002072, PLACE1002119, PLACE1002150, PLACE1002157, PLACE1002205, PLACE1002256, PLACE1002259, PLACE1002342, PLACE1002438, PLACE1002450, PLACE1002474, PLACE1002477, PLACE1002499, PLACE1002500, PLACE1002537, PLACE1002578, PLACE1002655, PLACE1002665, PLACE1002722, PLACE1002815, PLACE1002834, PLACE1002851, PLACE1002881, PLACE1002968, PLACE1002993, PLACE1003027, PLACE1003108, PLACE1003174, PLACE1003200, PLACE1003205, PLACE1003249, PLACE1003256, PLACE1003302, PLACE1003334, PLACE1003343, PLACE1003361, PLACE1003373, PLACE1003394, PLACE1003420, PLACE1003516, PLACE1003519, PLACE1003575, PLACE1003584, PLACE1003592, PLACE1003593, PLACE1003638, PLACE1003669, PLACE1003704, PLACE1003723, PLACE1003760, PLACE1003762, PLACE1003768, PLACE1003771, PLACE1003795, PLACE1003833, PLACE1003870, PLACE1003892, PLACE1003915, PLACE1003968, PLACE1004103, PLACE1004149, PLACE1004156, PLACE1004256, PLACE1004277, PLACE1004284, PLACE1004384, PLACE1004425, PLACE1004467, PLACE1004471, PLACE1004473, PLACE1004510, PLACE1004629, PLACE1004658, PLACE1004672, PLACE1004686, PLACE1004751, PLACE1004777, PLACE1004814, PLACE1004815, PLACE1004824, PLACE1004827, PLACE1004836, PLACE1004840, PLACE1004885, PLACE1004972, PLACE1004979,
PLACE1005027, PLACE1005046, PLACE1005055, PLACE1005085, PLACE1005102, PLACE1005108, PLACE1005128, PLACE1005146, PLACE1005266, PLACE1005305, PLACE1005374, PLACE1005409, PLACE1005453, PLACE1005477, PLACE1005481, PLACE1005528, PLACE1005574, PLACE1005666, PLACE1005763, PLACE1005804, PLACE1005828, PLACE1005834, PLACE1005850, PLACE1005851, PLACE1005934, PLACE1006002, PLACE1006076, PLACE1006119, PLACE1006143, PLACE1006159, PLACE1006164, PLACE1006170, PLACE1006187, PLACE1006223, PLACE1006239, PLACE1006248, PLACE1006412, PLACE1006445, PLACE1006482, PLACE1006492, PLACE1006521, PLACE1006540, PLACE1006617, PLACE1006629, PLACE1006673, PLACE1006704, PLACE1006760, PLACE1006792, PLACE1006795, PLACE1006800, PLACE1006860, PLACE1006904, PLACE1006961, PLACE1006962, PLACE1007045, PLACE1007274, PLACE1007346, PLACE1007367, PLACE1007375, PLACE1007386, PLACE1007416, PLACE1007450, PLACE1007454, PLACE1007478, PLACE1007484, PLACE1007544, PLACE1007547, PLACE1007557, PLACE1007598, PLACE1007645, PLACE1007677, PLACE1007743, PLACE1007807, PLACE1007829, PLACE1007858, PLACE1008002, PLACE1008129, PLACE1008132, PLACE1008201, PLACE1008209, PLACE1008273, PLACE1008368, PLACE1008532, PLACE1008568, PLACE1008696, PLACE1008867, PLACE1008887, PLACE1008941, PLACE1009027, PLACE1009039, PLACE1009050, PLACE1009099, PLACE1009155, PLACE1009172, PLACE1009174, PLACE1009298, PLACE1009328, PLACE1009335, PLACE1009338, PLACE1009388, PLACE1009444, PLACE1009595, PLACE1009596, PLACE1009607, PLACE1009621, PLACE1009637, PLACE1009665, PLACE1009708, PLACE1009798, PLACE1009861, PLACE1009886, PLACE1009971, PLACE1009995, PLACE1010089, PLACE1010102, PLACE1010106, PLACE1010152, PLACE1010383, PLACE1010491, PLACE1010616, PLACE1010630, PLACE1010631, PLACE1010702, PLACE1010739, PLACE1010761, PLACE1010833, PLACE1010870, PLACE1010896, PLACE1010916, PLACE1010925, PLACE1010942, PLACE1010954, PLACE1010965, PLACE1011041, PLACE1011046, PLACE1011054, PLACE1011057, PLACE1011090, PLACE1011109, PLACE1011203, PLACE1011214, PLACE1011296, PLACE1011340, PLACE1011433, PLACE1011452, PLACE1011567, PLACE1011576, PLACE1011643, PLACE1011675, PLACE1011719, PLACE1011729, PLACE1011749, PLACE1011762, PLACE1011783, PLACE1011874, PLACE1011995,
PLACE2000017, PLACE2000021, PLACE2000033, PLACE2000039, PLACE2000047, PLACE2000062, PLACE2000103, PLACE2000124, PLACE2000170, PLACE2000216, PLACE2000235, PLACE2000264, PLACE2000302, PLACE2000305, PLACE2000335, PLACE2000342, PLACE2000347, PLACE2000379, PLACE2000394, PLACE2000433, PLACE2000450, PLACE2000465, PLACE3000103, PLACE3000119, PLACE3000121, PLACE3000124, PLACE3000155, PLACE3000158, PLACE3000207, PLACE3000220, PLACE3000242, PLACE3000271, PLACE3000304, PLACE3000322, PLACE3000331, PLACE3000341, PLACE3000362, PLACE3000365, PLACE3000388, PLACE3000399, PLACE3000400, PLACE3000401, PLACE3000402, PLACE3000425, PLACE3000455, PLACE4000034, PLACE4000049, PLACE4000089, PLACE4000128, PLACE4000156, PLACE4000222, PLACE4000250, PLACE4000320, PLACE4000379, PLACE4000431, PLACE4000445, PLACE4000465, PLACE4000487, PLACE4000494, PLACE4000522, PLACE4000558, PLACE4000581, PLACE4000650, PLACE4000654,
THYRO1000034, THYRO1000085, THYRO1000092, THYRO1000111, THYRO1000156, THYRO1000163, THYRO1000173, THYRO1000190, THYRO1000197, THYRO1000221, THYRO1000241, THYRO1000327, THYRO1000381, THYRO1000387, THYRO1000394, THYRO1000488, THYRO1000585, THYRO1000625, THYRO1000637, THYRO1000658, THYRO1000666, THYRO1000676, THYRO1000684, THYRO1000712, THYRO1000734, THYRO1000793, THYRO1000796, THYRO1000805, THYRO1000815, THYRO1000865, THYRO1000916, THYRO1000934, THYRO1000974, THYRO1000975, THYRO1001031, THYRO1001062, THYRO1001093, THYRO1001133, THYRO1001173, THYRO1001177, THYRO1001189, THYRO1001204, THYRO1001213, THYRO1001262, THYRO1001290, THYRO1001320, THYRO1001322, THYRO1001401, THYRO1001406, THYRO1001426, THYRO1001480, THYRO1001487, THYRO1001537, THYRO1001595, THYRO1001617, THYRO1001637, THYRO1001706, THYRO1001772, THYRO1001828, THYRO1001854, Y79AA1000059, Y79AA1000214, Y79AA1000355, Y79AA1000410, Y79AA1000538, Y79AA1000539, Y79AA1000705, Y79AA1000800, Y79AA1000850, Y79AA1000962, Y79AA1000976, Y79AA1001061, Y79AA1001068, Y79AA1001493, Y79AA1001548, Y79AA1001585, Y79AA1001594, Y79AA1001696, Y79AA1001711, Y79AA1002103, Y79AA1002115, Y79AA1002258, Y79AA1002361, Y79AA1002407, Y79AA1002472, Y79AA1002482

On the other hand, clones of which expression levels decreased by RA are as follows:
HEMBA1000946, HEMBA1003569, HEMBA1005570, HEMBB1000915, NT2RM1000666, NT2RM2000092, NT2RM2000594, NT2RM2001256, NT2RM4001754, NT2RM4001905, NT2RP2001675, NT2RP2002047, NT2RP2005491, NT2RP3000980, NT2RP3002081, NT2RP3004594, NT2RP4001950, NT2RP4002408, OVARC1000431, OVARC1001942, OVARC1001943, PLACE1003190, PLACE1004868, PLACE1005923, PLACE1007257, PLACE1010624, Y79AA1000346

Clones of which expression levels increase by RA/inhibitor are as follows:
HEMBA1000046, HEMBA1000307, HEMBA1000434, HEMBA1000504, HEMBA1000588, HEMBA1000682, HEMBA1000726, HEMBA1000943, HEMBA1001071, HEMBA1001094, HEMBA1001122, HEMBA1001323, HEMBA1001361, HEMBA1001455, HEMBA1001709, HEMBA1001746, HEMBA1001869, HEMBA1002084, HEMBA1002583, HEMBA1002628, HEMBA1002801, HEMBA1002937, HEMBA1003096, HEMBA1003142, HEMBA1003229, HEMBA1003276, HEMBA1003309, HEMBA1003463, HEMBA1003597, HEMBA1003617, HEMBA1003725, HEMBA1003803, HEMBA1003879, HEMBA1003989, HEMBA1004000, HEMBA1004015, HEMBA1004024, HEMBA1004049, HEMBA1004056, HEMBA1004199, HEMBA1004248, HEMBA1004356, HEMBA1004554, HEMBA1004666, HEMBA1004725, HEMBA1004770, HEMBA1004803, HEMBA1004923, HEMBA1004934, HEMBA1004954, HEMBA1005039, HEMBA1005075, HEMBA1005113, HEMBA1005219, HEMBA1005232, HEMBA1005251, HEMBA1005304, HEMBA1005367, HEMBA1005372, HEMBA1005403, HEMBA1005410, HEMBA1005411, HEMBA1005548, HEMBA1005581, HEMBA1005631, HEMBA1005666, HEMBA1005755, HEMBA1005780, HEMBA1006067, HEMBA1006130, HEMBA1006364, HEMBA1006485, HEMBA1006559, HEMBA1006579, HEMBA1006754, HEMBB1000059, HEMBB1000575, HEMBB1000709, HEMBB1000822, HEMBB1000848, HEMBB1000852, HEMBB1000913, HEMBB1000985, HEMBB1001117, HEMBB1001210, HEMBB1001317, HEMBB1001394, HEMBB1001443, HEMBB1001668, HEMBB1001695, HEMBB1002049, HEMBB1002254, HEMBB1002266, HEMBB1002371, HEMBB1002502, HEMBB1002614, HEMBB1002617, HEMBB1002692, HEMBB1002697, MAMMA1000241, MAMMA1000424, MAMMA1000616, MAMMA1000731, MAMMA1000824, MAMMA1000908, MAMMA1000956, MAMMA1001038, MAMMA1001091, MAMMA1001243, MAMMA1001815, MAMMA1001820, MAMMA1002267, MAMMA1002769, MAMMA1002871, MAMMA1002941,
NT2RM1000355, NT2RM1000725, NT2RM1000829, NT2RM1000850, NT2RM1000898, NT2RM2000504, NT2RM2000635, NT2RM2000718, NT2RM2000821, NT2RM2001370, NT2RM2001582, NT2RM2001592, NT2RM2001613, NT2RM2001632, NT2RM2001635, NT2RM2001648, NT2RM2001659, NT2RM2001671, NT2RM2001695, NT2RM2001760, NT2RM2001782, NT2RM2001839, NT2RM2001879, NT2RM2001983, NT2RM4000104, NT2RM4000290, NT2RM4000425, NT2RM4000433, NT2RM4000471, NT2RM4000531, NT2RM4000852, NT2RM4001047, NT2RM4001347, NT2RM4001454, NT2RM4001557, NT2RM4001566, NT2RM4001582, NT2RM4001938, NT2RM4001953, NT2RM4002018, NT2RM4002409, NT2RM4002558, NT2RM4002594, NT2RP1000259, NT2RP1000418, NT2RP1000574, NT2RP1000629, NT2RP1000782, NT2RP1000856, NT2RP1000943, NT2RP1000988, NT2RP1001013, NT2RP1001173, NT2RP1001546, NT2RP2000091, NT2RP2000208, NT2RP2000274, NT2RP2000329, NT2RP2000369, NT2RP2000634, NT2RP2000842, NT2RP2000943, NT2RP2000987, NT2RP2001094, NT2RP2001277, NT2RP2001290, NT2RP2001366, NT2RP2001423, NT2RP2001436, NT2RP2001467, NT2RP2001506, NT2RP2001601, NT2RP2001663, NT2RP2001926, NT2RP2001985, NT2RP2002032, NT2RP2002041, NT2RP2002046, NT2RP2002078, NT2RP2002124, NT2RP2002185, NT2RP2002193, NT2RP2002312, NT2RP2002316, NT2RP2002426, NT2RP2002457, NT2RP2002475, NT2RP2002520, NT2RP2002595, NT2RP2002643, NT2RP2002672, NT2RP2002701, NT2RP2002710, NT2RP2002727, NT2RP2003099, NT2RP2003121, NT2RP2003137, NT2RP2003158, NT2RP2003206, NT2RP2003230, NT2RP2003272, NT2RP2003280, NT2RP2003347, NT2RP2003393, NT2RP2003401, NT2RP2003445, NT2RP2003456, NT2RP2003511, NT2RP2003517, NT2RP2003543, NT2RP2003596, NT2RP2003706, NT2RP2003871, NT2RP2004681, NT2RP2004743, NT2RP2004775, NT2RP2004933, NT2RP2004967, NT2RP2005003, NT2RP2005270, NT2RP2005289, NT2RP2005344, NT2RP2005453, NT2RP2005555, NT2RP2005767, NT2RP2005853, NT2RP2006043, NT2RP2006393, NT2RP2006436, NT2RP2006441, NT2RP2006467, NT2RP2006534, NT2RP2006565, NT2RP3000348, NT2RP3000359, NT2RP3000366, NT2RP3000403, NT2RP3000418, NT2RP3000441, NT2RP3000561, NT2RP3000759, NT2RP3000826, NT2RP3001007, NT2RP3001096, NT2RP3001126, NT2RP3001355, NT2RP3001396, NT2RP3001449, NT2RP3001490, NT2RP3001679, NT2RP3001727, NT2RP3001752, NT2RP3001777, NT2RP3001782, NT2RP3001799, NT2RP3001854, NT2RP3001989, NT2RP3002142, NT2RP3002248, NT2RP3002343, NT2RP3002484, NT2RP3002529, NT2RP3002549, NT2RP3002628, NT2RP3002687, NT2RP3002688, NT2RP3002810, NT2RP3003032, NT2RP3003139, NT2RP3003193, NT2RP3003203, NT2RP3003204, NT2RP3003210, NT2RP3003212, NT2RP3003264, NT2RP3003282, NT2RP3003500, NT2RP3004041, NT2RP3004215, NT2RP4000147, NT2RP4000259, NT2RP4000360, NT2RP4000448, NT2RP4000524, NT2RP4000588, NT2RP4000879, NT2RP4000907, NT2RP4000989, NT2RP4001079, NT2RP4001150, NT2RP4001219, NT2RP4001260, NT2RP4001274, NT2RP4001353, NT2RP4001547, NT2RP4001677, NT2RP4002052, OVARC1000006, OVARC1000092, OVARC1000321, OVARC1000384, OVARC1000408, OVARC1000414, OVARC1000520, OVARC1000526, OVARC1000588, OVARC1000679, OVARC1000682, OVARC1000769, OVARC1000850, OVARC1000862, OVARC1000886, OVARC1000984, OVARC1001000, OVARC1001004, OVARC1001154, OVARC1001170, OVARC1001173, OVARC1001200, OVARC1001268, OVARC1001376, OVARC1001419, OVARC1001425, OVARC1001476, OVARC1001480, OVARC1001542, OVARC1001873, OVARC1001928, OVARC1001987, OVARC1002066, OVARC1002082, OVARC1002112, OVARC1002127,
PLACE1000014, PLACE1000048, PLACE1000184, PLACE1000185, PLACE1000246, PLACE1000292, PLACE1000332, PLACE1000347, PLACE1000564, PLACE1000656, PLACE1000712, PLACE1001000, PLACE1001168, PLACE1001185, PLACE1001241, PLACE1001294, PLACE1001311, PLACE1001395, PLACE1001570, PLACE1001608, PLACE1001610, PLACE1001716, PLACE1001746, PLACE1001817, PLACE1001821, PLACE1001844, PLACE1001897, PLACE1002066, PLACE1002119, PLACE1002157, PLACE1002205, PLACE1002256, PLACE1002259, PLACE1002399, PLACE1002438, PLACE1002474, PLACE1002477, PLACE1002500, PLACE1002514, PLACE1002578, PLACE1002815, PLACE1002851, PLACE1002968, PLACE1003108, PLACE1003174, PLACE1003200, PLACE1003238, PLACE1003256, PLACE1003334, PLACE1003342, PLACE1003516, PLACE1003521, PLACE1003537, PLACE1003592, PLACE1003596, PLACE1003723, PLACE1003760, PLACE1003771, PLACE1003783, PLACE1003795, PLACE1003892, PLACE1003968, PLACE1004103, PLACE1004256, PLACE1004405, PLACE1004460, PLACE1004506, PLACE1004629, PLACE1004674, PLACE1004813, PLACE1004979, PLACE1005066, PLACE1005101, PLACE1005102, PLACE1005128, PLACE1005181, PLACE1005287, PLACE1005305, PLACE1005327, PLACE1005477, PLACE1005595, PLACE1005603, PLACE1005666, PLACE1005804, PLACE1005884, PLACE1005934, PLACE1006076, PLACE1006119, PLACE1006159, PLACE1006164, PLACE1006170, PLACE1006382, PLACE1006492, PLACE1006629, PLACE1006704, PLACE1006731, PLACE1006760, PLACE1006779, PLACE1006795, PLACE1006805, PLACE1006962, PLACE1007045, PLACE1007111, PLACE1007282, PLACE1007386, PLACE1007416, PLACE1007484, PLACE1007544, PLACE1007645, PLACE1007743, PLACE1007746, PLACE1007807, PLACE1007858, PLACE1008002, PLACE1008181, PLACE1008273, PLACE1008368, PLACE1008405, PLACE1008532, PLACE1008568, PLACE1008625, PLACE1008696, PLACE1008867, PLACE1009027, PLACE1009039, PLACE1009045, PLACE1009110, PLACE1009298, PLACE1009328, PLACE1009581, PLACE1009621, PLACE1009622, PLACE1009637, PLACE1009925, PLACE1009935, PLACE1010089, PLACE1010106, PLACE1010152, PLACE1010274, PLACE1010491, PLACE1010629, PLACE1010630, PLACE1010714, PLACE1010739, PLACE1010891, PLACE1010896, PLACE1010925, PLACE1010965, PLACE1011026, PLACE1011046, PLACE1011214, PLACE1011399, PLACE1011433, PLACE1011492, PLACE1011641, PLACE1011649, PLACE1011719, PLACE1011762, PLACE1011858, PLACE1011923, PLACE2000014, PLACE2000039, PLACE2000216, PLACE2000302, PLACE2000317, PLACE2000342, PLACE2000347, PLACE2000379, PLACE3000121, PLACE3000124, PLACE3000160, PLACE3000242, PLACE3000271, PLACE3000353, PLACE3000362, PLACE3000365, PLACE3000400, PLACE3000401, PLACE4000034, PLACE4000089, PLACE4000522, PLACE4000558,
SKNMC1000050, THYRO1000040, THYRO1000197, THYRO1000241, THYRO1000327, THYRO1000394, THYRO1000488, THYRO1000501, THYRO1000585, THYRO1000596, THYRO1000625, THYRO1000805, THYRO1000934, THYRO1001133, THYRO1001134, THYRO1001173, THYRO1001213, THYRO1001262, THYRO1001290, THYRO1001721, Y79AA1000037, Y79AA1000800, Y79AA1000976, Y79AA1001078, Y79AA1001228, Y79AA1001299, Y79AA1001402, Y79AA1001585, Y79AA1001696, Y79AA1001711, Y79AA1001827, Y79AA1001875, Y79AA1002027, Y79AA1002211, Y79AA1002234, Y79AA1002258

On the other hand, clones of which expression levels decrease by RA/inhibitor are as follows:
HEMBA1000012, HEMBA1000501, HEMBA1000946, HEMBA1003220, HEMBA1003403, HEMBA1003569, HEMBA1003591, HEMBA1003926, HEMBA1004168, HEMBA1004507, HEMBA1005009, HEMBA1005296, HEMBA1005528, HEMBA1005570, HEMBA1006467, HEMBA1006486, HEMBA1006492, HEMBA1007322, HEMBB1000055, HEMBB1000244, HEMBB1001665, MAMMA1000684, MAMMA1001139, MAMMA1001743, NT2RM1000257 NT2RM1000318, NT2RM1000539, NT2RM1000666, NT2RM2000092, NT2RM2000192, NT2RM2000371, NT2RM2000594, NT2RM4000511, NT2RM4001140, NT2RM4001754, NT2RM4001905, NT2RM4001940, NT2RM4002593, NT2RP1000086, NT2RP1000439, NT2RP1001073, NT2RP2000098, NT2RP2000965, NT2RP2001397, NT2RP2002047, NT2RP2004226, NT2RP2004396, NT2RP2004655, NT2RP2005126, NT2RP2005464, NT2RP2005712, NT2RP2005859, NT2RP2005890, NT2RP3000980, NT2RP3001383, NT2RP3001621, NT2RP3002081, NT2RP3002181, NT2RP3002244, NT2RP3002590, NT2RP3003059, NT2RP3004258, NT2RP3004378, NT2RP3004527, NT2RP3004594, NT2RP4001760, NT2RP4001950, NT2RP4002047, NT2RP4002408, NT2RP5003459, OVARC1000004, OVARC1000035, OVARC1000431, OVARC1001051, OVARC1001129, OVARC1001176, OVARC1001261, OVARC1001342, OVARC1001942, OVARC1001943, PLACE1002171, PLACE1002465, PLACE1003190, PLACE1003375, PLACE1004128, PLACE1005026, PLACE1005876, PLACE1005923, PLACE1007257, PLACE1007375, PLACE1007507, PLACE1008941, PLACE1010624, PLACE1011090, PLACE1011219, THYRO1000270, Y79AA1000346, Y79AA1001541

These clones are also associated with neural differentiation and, therefore, are candidates for genes associated with neurological diseases.

For example, if the protein encoded by the cDNA of the present invention is a regulatory factor of cellular conditions such as growth and differentiation, it can be used for developing medicines as follows. The protein or antibody provided by the invention is injected into a certain kind of cells by microinjection. Then, using the cells, it is possible to screen low molecular weight compounds by measuring the change in the cellular conditions, or the activation or inhibition of a particular gene. The screening can be performed as follows. First, the protein is expressed and purified as recombinant. The purified protein is microinjected into cells such as various cell lines, or primary culture cells, and the cellular change such as growth and differentiation can be examined. Alternatively, the induction of genes whose expression is known to be associated with a particular change of cellular conditions may be detected by the amount of mRNA or protein. Or, the amount of intracellular molecules (low molecular weight compounds, etc.) that is changed by the function of the gene product (protein) which is known to be associated with a particular change of cellular conditions may be detected. The compounds to be screened (both low and high molecular compounds are acceptable) can be added to the culture media and assessed for their activity by measuring the change of the cellular conditions. Instead of microinjection, cell lines introduced with the gene obtained in the invention can be used for the screening. If the gene product is turn out to be associated with a particular change in the cellular conditions, the change of the product can be used as a measurement for screening. Once a compound is screened out which can activate or inhibit the function of the protein of the invention, it can be applied for developing medicines.

If the protein encoded by the cDNA of the present invention is a secretory protein, membrane protein, or protein associated with signal transduction, glycoprotein, transcription, or diseases, it can be used in functional assays for developing medicines.

In case of a membrane protein, it is most likely to be a protein that functions as a receptor or ligand on the cell surface. Therefore, it is possible to reveal a new relationship between a ligand and receptor by screening the membrane protein of the invention based on the binding activity with the known ligand or receptor. Screening can be performed according to the known methods.

For example, a ligand against the protein of the invention can be screened in the following manner. Namely, a ligand that binds to a specific protein can be screened by a method comprising the steps of: (a) contacting a test sample with the protein of the invention or a partial peptide thereof, or cells expressing these, and (b) selecting a test sample that binds to said protein, said partial peptide, or said cells.

On the other hand, for example, screening using cells expressing the protein of the present invention that is a receptor protein can also be performed as follows. It is possible to screen receptors that is capable of binding to a specific protein by using procedures (a) attaching the sample cells to the protein of the invention or its partial peptide, and (b) selecting cells that can bind to the said protein or its partial peptide.

In a following screening as an example, first the protein of the invention is expressed, and the recombinant protein is purified. Next, the purified protein is labeled, binding assay is performed using a various cell lines or primary cultured cells, and cells that are expressing a receptor are selected (Growth and differentiation factors and their receptors, Shin-Seikagaku Jikken Kouza Vol.7 (1991) Honjyo, Arai, Taniguchi, and Muramatsu edit, p203-236, Tokyo-Kagaku-Doujin). A protein of the invention can be labeled with RI such as ¹²⁵I, and enzyme (alkaline phosphatase etc.). Alternatively, a protein of the invention may be used without labeling and then detected by using a labeled antibody against the protein. The cells that are selected by the above screening methods, which express a receptor of the protein of the invention, can be used for the further screening of an agonists or antagonists of the said receptor.

Once the ligand binding to the protein of the invention, the receptor of the protein of the invention or the cells expressing the receptor are obtained by screening, it is possible to screen a compound that binds to the ligand and receptor. Also it is possible to screen a compound that can inhibit both bindings (agonists or antagonists of the receptor, for example) by utilizing the binding activities.

When the protein of the invention is a receptor, the screening method comprises the steps of (a) contacting the protein of the invention or cells expressing the protein of the invention with the ligand, in the presence of a test sample, (b) detecting the binding activity between said protein or cells expressing said protein and the ligand, and (c) selecting a compound that reduces said binding activity when compared to the activity in the absence of the test sample. Furthermore, when the protein of the invention is a ligand, the screening method comprises the steps of (a) contacting the protein of the invention with its receptor or cells expressing the receptor in the presence of samples, (b) detecting the binding activity between the protein and its receptor or the cells expressing the receptor, and (c) selecting a compound that can potentially reduce the binding activity compared to the activity in the absence of the sample.

Samples to screen include cell extracts, expressed products from a gene library, synthesized low molecular compound, synthesized peptide, and natural compounds, for example, but are not construed to be listed here. A compound that is isolated by the above screening using a binding activity of the protein of the invention can also be used as a sample.

A compound isolated by the screening may be a candidate to be an agonist or an antagonist of the receptor of the protein. By utilizing an assay that monitors a change in the intracellular signaling such as phosphorylation which results from reduction of the binding between the protein and its receptor, it is possible to identify whether the obtained compound is an agonist or antagonist of the receptor. Also, the compound may be a candidate of a molecule that can inhibit the interaction between the protein and its associated proteins (including a receptor) in vivo. Such compounds can be used for developing drugs for precaution or cures of a disease with which the protein is associated.

Secretory proteins may regulate cellular conditions such as growth and differentiation. It is possible to find out a novel factor that regulates cellular conditions by adding the secretory protein of the invention to a certain kind of cell, and performing a screening by utilizing the cellular changes in growth or differentiation, or activation of a particular gene.

The screening can be performed, for example, as follows. First, the protein of the invention is expressed and purified in a recombinant form. Then, the purified protein is added to a various kind of cell lines or primary cultured cells, and the change in the cell growth and differentiation is monitored. The induction of a particular gene that is known to be involved in a certain cellular change is detected by the amounts of mRNA and protein. Alternatively, the amount of an intracellular molecule (low-molecular-weight compounds, etc.) that is changed by the function of a gene product (protein) that is known to function in a certain cellular change is used for the detection.

Once the screening reveals that the protein of the invention can regulate cellular conditions or the functions, it is possible to apply the protein as a pharmaceutical and diagnostic medicine for associated diseases by itself or by altering a part of it into an appropriate composition.

As is above described for membrane proteins, the secretory protein provided by the invention may be used to explore a novel ligand-receptor interaction using a screening based on the binding activity to a known ligand or receptor. A similar method can be used to identify an agonist or antagonist. The resulting compounds obtained by the methods can be a candidate of a compound that can inhibit the interaction between the protein of the invention and an interacting molecule (including a receptor). The compounds may be able to use as a preventive, therapeutic, and diagnostic medicine for the diseases, in which the protein may play a certain role.

Proteins associated with signal transduction or transcription may be a factor that affects a certain protein or gene in response to intracellular/extracellular stimuli. It is possible to find out a novel factor that can affect a protein or gene by expressing the protein provided by the invention in a certain types of cells, and performing a screening utilizing the activation of a certain intracellular protein or gene.

The screening may be performed as follows. First, a transformed cell line expressing the protein is obtained. Then, the transformed cell line and the untransformed original cell line are compared for the changes in the expression of a certain gene by detecting the amount of its mRNA or protein. Alternatively, the amount of an intracellular molecule (low molecular weight compounds) that is changed by the function of a certain gene product (protein) may be used for the detection. Furthermore, the change of the expression of a certain gene can be detected by introducing a fusion gene that comprises a regulatory region of the gene and a marker gene (luciferase, beta-galactosidase, etc.) into a cell, expressing the protein provided by the invention into the cell, and estimating the activity of a marker gene product (protein).

If the protein or gene of the invention is associated with diseases, it is possible to screen a gene or compound that can regulate its expression and/or activity either directly or indirectly by utilizing the protein of the present invention.

For example, the protein of the invention is expressed and purified as a recombinant protein. Then, the protein or gene that interacts with the protein of the invention is purified, and screened based on the binding. Alternatively, the screening can be performed by adding with a compound of a candidate of the inhibitor added in advance and monitoring the change of binding activity. In another method, a transcription regulatory region locating in the 5'-upstream of the gene encoding the protein of the invention that is capable of regulating the expression of other genes is obtained, and fused with a marker gene. The fusion is introduced into a cell, and the cell is added with compounds to explore a regulatory factor of the expression of the said gene.

The compound obtained by the screening can be used for developing pharmaceutical and diagnostic medicines for the diseases with which the protein of the present invention is associated. Similarly, if the regulatory factor obtained in the screening is turn out to be a protein, compounds that can newly affect the expression or activity of the protein may be used as a medicine for the diseases with which the protein of the invention is associated.

If the protein of the invention has an enzymatic activity, regardless as to whether it is a secretory protein, membrane protein, or proteins associated with signal transduction, glycoprotein, transcription, or diseases, a screening may be performed by adding a compound to the protein of the invention and monitoring the change of the compound. The enzymatic activity may also be utilized to screen a compound that can inhibit the activity of the protein.

In a screening given as an example, the protein of the invention is expressed and the recombinant protein is purified. Then, compounds are contacted with the purified protein, and the amount of the compound and the reaction products is examined. Alternatively, compounds that are candidates of an inhibitor are pretreated, then a compound (substrate) that can react with the purified protein is added, and the amount of the substrate and the reaction products is examined.

The compounds obtained in the screening may be used as a medicine for diseases with which the protein of the invention is associated. Also they can be applied for tests that examine whether the protein of the invention functions normally *in vivo.*

Whether the secretory protein, membrane protein, signal transduction-associated protein, glycoprotein-associated protein, or transcription-associated protein of the present invention is a novel protein associated with diseases or not is determined in another method than described above, by obtaining a specific antibody against the protein of the invention, and examining the relationship between the expression or activity of the protein and a certain disease. In an alternative way, it may be analyzed referred to the methods in "Molecular Diagnosis of Genetic Diseases" (Elles R. edit, (1996) in the series of "Method in Molecular Biology" (Humana Press).

Proteins associated with diseases are targets of screening as mentioned, and thus are very useful in developing drugs which regulate their expression and activity. Also, the proteins are useful in the medicinal industry as a diagnostic marker of the associated disease or a target of gene therapy.

Compounds isolated as mentioned above can be administered patients as it is, or after formulated into a pharmaceutical composition according to the known methods. For example, a pharmaceutically acceptable carrier or vehicle, specifically sterilized water, saline, plant oil, emulsifier, or suspending agent can be mixed with the compounds appropriately. The pharmaceutical compositions can be administered to patients by a method known to those skilled in the art, such as intraarterial, intravenous, or subcutaneous injections. The dosage may vary depending on the weight or age of a patient, or the method of administration, but those skilled in the art can choose an appropriate dosage properly. If the compound is encoded by DNA, the DNA can be cloned into a vector for gene therapy, and used for gene therapy. The dosage of the DNA and the method of its administration may vary depending on the weight or age of a patient, or the symptoms, but those skilled in the art can choose properly.

The present invention further relates to databases comprising at least a sequence of polynucleotide and/or protein, or a medium recorded in such databases, selected from the sequence data of the nucleotide and/or the amino acids indicated in Table 350 and Table 351.
The term "database" means a set of accumulated information as machine-searchable and readable information of nucleotide sequence. The databases of the present invention comprise at least one of the novel nucleotide sequences of polynucleotides provided by the present invention. The databases of the present invention can consist of only the sequence data of the novel polynucleotides provided by the present invention or can comprise other information on nucleotide sequences of known full-length cDNAs or ESTs. The databases of the present invention can be comprised of not only the information on the nucleotide sequences but also the information on the gene functions revealed by the present invention. Additional information such as names of DNA clones carrying the full-length cDNAs can be recorded or linked together with the sequence data in the databases.

The database of the present invention is useful for gaining complete gene sequence information from partial sequence information of a gene of interest. The database of the present invention comprises nucleotide sequence information of full-length cDNAs. Consequently, by comparing the information in this database with the nucleotide sequence of a partial gene fragment yielded by differential display method or subtraction method, the information on the full-length nucleotide sequence of interest can be gained from the sequence of the partial fragment as a starting clue.

The sequence information of the full-length cDNAs constituting the database of the present invention contains not only the information on the complete sequences but also extra information on expression frequency of the genes as well as homology of the genes to known genes and known proteins. Thus the extra information facilitates rapid functional analyses of partial gene fragments. Further, the information on human genes is accumulated in the database of the present invention, and therefore, the database is useful for isolating a human homologue of a gene originating from other species. The human homologue can be isolated based on the nucleotide sequence of the gene from the original species.

At present, information on a wide variety of gene fragments can be obtained by differential display method and subtraction method. In general, these gene fragments are utilized as tools for isolating the full-length sequences thereof. When the gene fragment corresponds to an already-known gene, the full-length sequence is easily obtained by comparing the partial sequence with the information in known databases. However, when there exists no information corresponding to the partial sequence of interest in the known databases, cDNA cloning should be carried out for the full-length cDNA. It is often difficult to obtain the full-length nucleotide sequence using the partial sequence information as an initial clue. If the full-length of the gene is not available, the amino acid sequence of the protein encoded by the gene remains unidentified. Thus the database of the present invention can contribute to the identification of full-length cDNAs corresponding to gene fragments, which cannot be revealed by using databases of known genes.

The present invention has provided 5602 novel full-length cDNA clones, and primers for synthesizing the cDNA. As has not yet proceeded the isolation of full-length cDNA within the human, the invention has great significance. The full-length cDNA clones contain the translation initiation site, and thus provide a useful information for analysis of protein functions.

The cDNA clones are assumed to encode proteins such as secretory proteins, membrane proteins, signal transduction-associated protein, glycoprotein-associated protein, or transcription-associated protein, etc., which have important functions in vivo, and also predicted to be associated with many diseases. The genes and proteins associated with diseases are useful for developing a diagnostic marker or medicines for regulation of their expression and activity, or as a target of gene therapy.

The invention is illustrated more specifically with reference to the following examples, but is not to be construed as being limited thereto.

### EXAMPLE 1

### Construction of a cDNA library by the oligo-capping method.

The NT-2 neuron progenitor cells (Stratagene), a teratocarcinoma cell line from human embryo testis, which can differentiate into neurons by the treatment with retinoic acid were used.
The NT-2 cells were cultured according to the manufacturer's instructions as follows.
(1) NT-2 cells were cultured without induction by retinoic acid treatment (NT2RM1, NT2RM2, NT2RM4).
(2) After cultured, NT-2 cells were induced by adding retinoic acid, and then were cultured for 48 hours (NT2RP1).
(3) After cultured, NT-2 cells were induced by adding retinoic acid, and then were cultured for 2 weeks (NT2RP2, NT2RP3, NT2RP4, NT2RP5).

Also, the human neuroblastoma cell line SK-N-MC (ATCC HTB-10) (SKNMC1), and human retinoblastoma cell line Y79 (ATCC HTB-18) (Y79AA1) were cultured according to the culture conditions described in the ATCC catalogue (http://www.atcc.org/). The cells were harvested separately, and mRNA was extracted from each cell by the method described in the literature (Molecular Cloning 2nd edition. (1989) Sambrook J., Fritsch, E.F., and Maniatis T., Cold Spring Harbor Laboratory Press). Furthermore, poly(A)⁺RNA was purified from the mRNA using oligo-dT cellulose.

Similarly, human placenta (PLACE1, PLACE2, PLACE3, PLACE4), human ovary cancer tissue (OVARC1), tissues from human embryo at 10 weeks, which is enriched with head (HEMBA1), or body (HEMBB1), human mammary gland (MAMMA1), human thyroid gland (THYRO1), and primary cultured cells of human blood vessel endothelium (VESEN1) were used to extract mRNA by the method described in the literature (Molecular Cloning 2nd edition. (1989) Sambrook J., Fritsch, E.F., and Maniatis T., Cold Spring Harbor Laboratory Press). Furthermore, poly(A)⁺RNA was purified from the mRNA using oligo-dT cellulose.

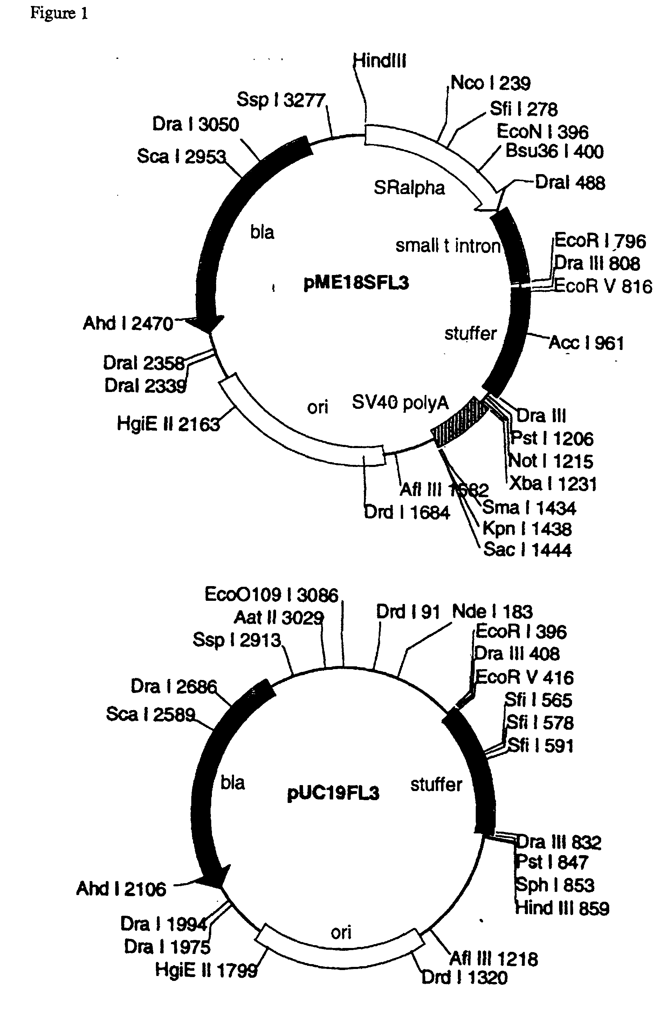

Each poly(A)⁺RNA was used to construct a cDNA library by the oligo-capping method (Maruyama M. and Sugano S. (1994) Gene, 138: 171-174). Using the Oligo-cap linker (SEQ ID NO: 10464) and the Oligo-dT primer (SEQ ID NO: 10465), bacterial alkaline phosphatase (BAP) treatment, tobacco acid phosphatase (TAP) treatment, RNA ligation, the first strand cDNA synthesis, and removal of RNA were performed as described in the reference (Suzuki and Kanno (1996) Protein Nucleic acid and Enzyme, 41: 197-201; Suzuki Y. et al. (1997) Gene, 200: 149-156). Next, 5'- and 3'-PCR primers (SEQ ID NO: 10466, and 10467, respectively) were used for performing PCR to convert the cDNA into double stranded cDNA, which was then digested with SfiI. Then, the DraIII-cleaved pUC19FL3 vector (Figure 1; for NT2RM1, and NT2RP1), or the DraIII-cleaved pME18SFL3 (Figure 1) (GenBank AB009864, expression vector; for NT2RM2, NT2RM4, NT2RP2, NT2RP3, NT2RP4, NT2RP5, SKNMC1, Y79AA1, PLACE1, PLACE2, PLACE3, PLACE4, OVARC1, HEMBA1, HEMBB1, MAMMA1, THYRO1, and VESEN1) was used for cloning the cDNA in a unidirectional manner, and cDNA libraries were obtained. The nucleotide sequence of the 5'- and 3'- ends of the cDNA clones was analyzed with a DNA sequencer (ABI PRISM 377, PE Biosystems) after sequencing reactions were performed with the DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, PE Biosystems), according to the instructions. The data were compiled into a database.

The full-length-enriched cDNA libraries except those for NT2RM1 and NT2RP1 were constructed using eukaryotic expression vector pME18SFL3. The vector contains SRα promoter and SV40 small t intron in the upstream of the cloning site, and SV40 polyA added signal sequence site in the downstream. As the cloning site of pME18SFL3 has asymmetrical DraIII sites, and the ends of cDNA fragments contain SfiI sites complementary to the DraIII sites, the cloned cDNA fragments can be inserted into the downstream of the SRα promoter unidirectionally. Therefore, clones containing full-length cDNA can be expressed transiently by introducing the obtained plasmid directly into COS cells. Thus, the clones can be analyzed very easily in terms of the proteins that are the gene products of the clones, or in terms of the biological activities of the proteins.

Herein, the cDNA libraries and the name of each clone are related as shown in Table 3. Therein, "xxxxxx" represents the clone number of six digits. Thus, the sequences are named by the library name, the clone number plus F- for the 5'-end, or R- for the 3'-end.

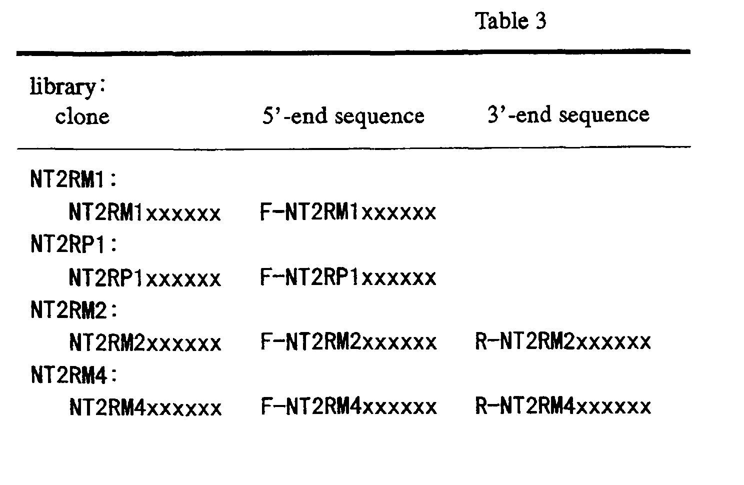

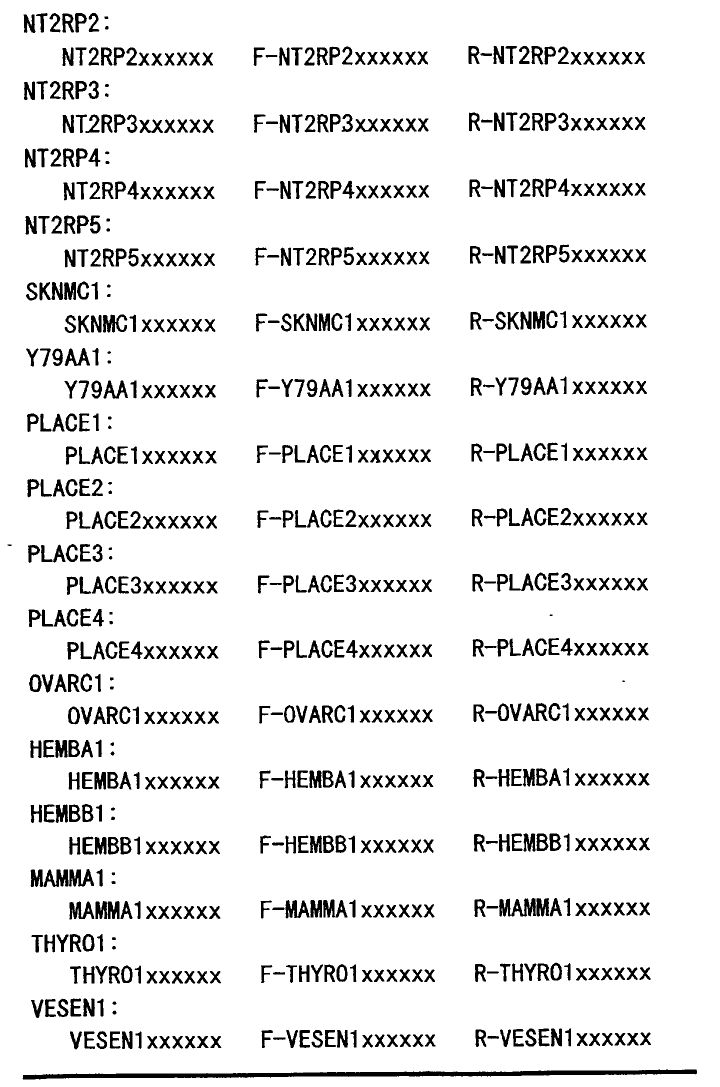

### EXAMPLE 2

### Estimation of the fullness ratio at the 5'-ends of the clones contained in the cDNA libraries constructed by the oligo-capping method.

The fullness ratio at the 5'-end sequence of the 59,823 clones in the human cDNA libraries constructed by the oligo-capping method was determined as follows. Of all the clones whose 5'-end sequences were found in those of known human mRNA in the public database, a clone was judged to be "full-length", if it had a longer 5'-end sequence than that of the known human mRNA, or, even though the 5'-end sequence was shorter, if it contained the translation initiation codon. A clone which did not contain the translation initiation codon was judged to be "not-full-length". The fullness ratio ((the number of full-length clones)/(the number of full-length and not-full-length clones)) at the 5'-end of the cDNA clones from each library was determined by comparing with the known human mRNA. As a result, the fullness ratio of the 5'-ends was 63.5%. The result indicates that the fullness ratio at the 5'-end sequence was extremely high.

### EXAMPLE 3

### Assessment of the fullness ratio of the 5'-end of the cDNA by the ATGpr and the ESTiMateFL.

The ATGpr, developed by Salamov A.A., Nishikawa T., and Swindells M.B. in the Helix Research Institute, is a program for prediction of the translation initiation codon based on the characteristics of the sequences in the vicinity of the ATG codon. The results are shown with expectations (also described as ATGpr1 below) that an ATG is a true initiation codon (0.05-0.94) (can be described as ATGpr1). When the program was applied to the 5'-end sequences of the clones from the cDNA library that was obtained by the oligo-capping method and that had 65% fullness ratio, the sensitivity and specificity of estimation of a full-length clone (clone containing the N-terminal end of ORF) were improved to 82-83% by selecting only clones having the ATGpr1 score 0.6 or higher.

Furthermore, the program was used to assess the fullness of 18,959 clones in the human libraries obtained here, which have 5'-ends matched to a known human mRNA.

Briefly, the maximal ATGpr1 score of the clones was determined, and then their 5'-end sequence was compared with the known human mRNA to estimate whether the clone is full-length or not. The result is shown in Table 4.

Based on the knowledge that known mRNAs, in general, are highly expressed in the cell, similar estimation was performed with genes having a low number in the EST hit, which represent relatively low abundant mRNAs, and the result is shown in Table 5.

In the table, the number of full-length clones indicate that of clones containing the N-terminal end of ORF, and so does the number of not-full-length clones that of clones without the N-terminal end of ORF. The fullness ratio represents (the number of full-length clones)/(the number of full-length clones plus the number of not-full-length clones).

**Table 4**

| The maximal ATGpr1 score and the fullness ratio of the 5'-end sequences of clones obtained from human cDNA libraries constructed by the oligo-capping method; clones having a matched 5'-end with that of a known mRNA. | | | |
|---|---|---|---|
| maximal ATGpr1 score | number of (full-length clones plus not-full-length clones) | number of full-length clones | fullness ratio |
| >=0.70 | 11,193 | 9,346 | 83.5% |
| >=0.50 | 13,369 | 10,549 | 78.9% |
| >=0.30 | 15,489 | 11,340 | 73.2% |
| >=0.15 | 17,394 | 11,811 | 67.9% |
| >=0.00 | 18,959 | 12,046 | 63.5% |

**Table 5**

| The maximal ATGpr1 score and the fullness ratio of the 5'-end sequences of the clones obtained from human cDNA libraries constructed by the oligo-capping method; clones having 5 EST hits or less among the clones having a matched 5'-end with that of a known mRNA. | | | |
|---|---|---|---|
| maximal ATGpr1 score | number of (full-length clones plus not-full-length clones) | number of full-length clones | fullness ratio |
| >=0.70 | 2,801 | 1,934 | 69.0% |
| >=0.50 | 3,683 | 2,393 | 65.0% |
| >=0.30 | 4,683 | 2,707 | 57.8% |
| >=0.15 | 5,559 | 2,890 | 52.0% |
| >=0.00 | 6,113 | 3,013 | 49.8% |

Next, the ESTiMateFL was used for the estimation. The ESTiMateFL, developed by Nishikawa and Ota in the Helix Research Institute, is a method for the selection of a clone with high fullness ratio by comparing with the 5'-end or 3'-end sequences of ESTs in the public database.

By the method, a cDNA clone is judged to be most likely not to be full-length if there exist any ESTs which have longer 5'-end or 3'-end sequences than the clone. The method is systematized for high throughput analysis. A clone is judged to be full-length if the clone has a longer 5'-end sequence than ESTs in the public database. Even if a clone has a shorter 5'-end, the clone is judged to be full-length if the difference in length is within 50 bases, and otherwise judged not to be full-length, for convenience.

In case of the clones whose 5'-end sequence is matching with the known mRNA, 80% of the clones judged to be full-length by comparing with ESTs was also judged to be full-length by comparing with the known mRNA. Also, 80% of the clones judged to be not full-length by comparing with ESTs was also judged to be not full-length by comparing with the known mRNA.

The precision of the estimation by comparing with ESTs is improved with increasing number of ESTs to be compared. However, in case that a limited number of ESTs are available, the reliability becomes low. Thus, the method is effective in excluding clones with high probability of being not-full-length, from the cDNA clones that is synthesized by the oligo-capping method and that have the 5'-end sequences with about 60 % fullness ratio. In particular, the ESTiMateFL is efficiently used to estimate the fullness ratio at the 3'-end sequence of cDNA of a human unknown mRNA which has a significant number of EST deposits in the public database.

The 18,959 clones isolated from human cDNA libraries constructed by the oligo-capping method, which have the 5'-end sequence matched with a known human mRNA, were estimated by using the ATGpr and ESTiMateFL. Briefly, the 5'-end sequence of the respective clone was analyzed to obtain the maximal ATGpr1 score, and compared with the ORF of the known human mRNA that matches with it to determine whether the clone is full-length or not. Then, the 5'-end sequence of the respective clone was analyzed by the ESTiMateFL to judge whether the clone is full-length or not. Specifically, the 5'-end sequences of the 18,959 clones were compared with those of ESTs by the ESTiMateFL and the clones other than those that are not full-length were selected. Then, the selected clones were used to analyze the relationship between the ATGpr and the fullness ratio. The result was summarized in Table 6. Also, among the selected, the clones in which the number of the EST hit is not more than 5 were selected and analyzed. The result was summarized in Table 7, which represents the result of the analysis of mRNA with relatively low abundance.

Therein, the number of being full-length, the number of being not full-length, and the fullness ratio indicate the number of the clones that contain the N-terminus of the ORF, the number of the clones that do not contain the N-terminus of the ORF, and (the number of being full-length)/(the number of being full-length) plus (the number of being not full-length), respectively.

**Table 6**

| The maximal ATGpr1 score and the fullness ratio of the 5'-end sequence in the clones isolated from human cDNA libraries constructed by the oligo-capping method, which have the 5'-end sequence matched with a known human mRNA, and also other than those being not full-length according to the comparison with ESTs. | | | |
|---|---|---|---|
| maximal ATGpr1 score | number of (full-length clones plus not-full-length clones) | number of full-length clones | fullness ratio |
| >=0.70 | 9,068 | 8,349 | 92.1% |
| >=0.50 | 10,345 | 9,318 | 90.1% |
| >=0.30 | 11,425 | 9,964 | 87.2% |
| >=0.15 | 12,254 | 10,335 | 84.3% |
| >=0.00 | 12,785 | 10,484 | 82.0% |

**Table 7**

| Maximal ATGpr1 score and fullness ratio of the 5'-end sequence of the clones, which were isolated from the human cDNA libraries constructed by the oligo-capping method, whose 5'-end sequence is identical to a known human mRNA, in which the number of the EST hit is not more than 5. | | | |
|---|---|---|---|
| maximal ATGpr1 score | number of (full-length clones plus not-full-length clones) | number of full-length clones | fullness ratio |
| >=0.70 | 1,959 | 1,510 | 77.1% |
| >=0.50 | 2,469 | 1,821 | 73.8% |
| >=0.30 | 2,975 | 2,046 | 68.8% |
| >=0.15 | 3,368 | 2,164 | 64.3% |
| >=0.00 | 3,661 | 2,226 | 60.8% |

The 19,226 clones, isolated from the human cDNA libraries constructed by the oligo-capping method, whose 5'-end sequence is identical to that of a known human mRNA were estimated by the ATGpr2, and the correlation between the score and the fullness ratio was estimated. Specifically, the maximal ATGpr2 score of the clones identical to a known human mRNA was determined, and then their fullness ratio was estimated by comparing the 5'-ends with ORF of known human mRNA. The result was shown in Table 8.

**Table 8**

| Maximal ATGpr2 score and fullness ratio of the 5'-end sequence of the clones, which are isolated from the human cDNA libraries constructed by the oligo-capping method, whose 5'-end sequence is identical to a known human mRNA. | | | |
|---|---|---|---|
| maximal ATGpr2 score | number of (full-length clones plus not-full-length clones) | number of full-length clones | fullness ratio |
| >=0.30 | 10,748 | 8,031 | 74.7% |
| >=0.15 | 16,383 | 11,226 | 68.5% |
| >=0.00 | 19,226 | 12,285 | 63.9% |

According to the above results, it was found that, in case of using clones isolated from human cDNA libraries constructed by the oligo-capping method, the fullness ratio of the clones that have low score in the ATGpr can be improved by estimating their 5'-end sequence using the combination of the ATGpr and the ESTiMateFL. Therefore, the method was applied to select a cDNA clone with high fullness ratio.

### EXAMPLE 4

### Clustering of the 5'-end and 3'-end sequences of cDNA clones.

The 5'-end and 3'-end sequences of cDNA clones were obtained, and clustered separately. The single pass data of the nucleotide sequence of the 5'-end and 3'-end was subject to the BLAST search between the sequence data of all the clones synthesized in example 1, and the clones considered to be originating from the same gene were put together into a group. If the 5'-end of a clone contains the consensus sequence of 300 bases or more with identity 95% or more, or the 3'-end contains the consensus sequence of 200 bases or more and having identity 90% or more, the clones were put in the same group.

The groups of the 5'-end sequence and the 3'-end sequence were further clustered so as that the groups from the same clone can be in the same group (cluster).

### EXAMPLE 5

### Characterization of the cloned sequence.

The data of the 5'-end sequence of the cloned sequence was characterized by the following way:
(1) examining whether it is identical to those of mRNA from human and other species (including authorized sequences) and human EST by the BLAST homology search of the GenBank,
(2) examining whether it has longer 5'-end than those of human mRNA and human EST,
(3) determining the scores in the ATGpr1 and ATGpr2 programs of all the initiation codons in the 5'-end sequence, and
(4) determining the number of the human EST clone(s) that is judged to be identical by the BLAST homology search of the GenBank.

The data of the 3'-end sequence of the cloned sequence was characterized by the above (1) and (4).

These characterized data were used for the final selection of the clones.

### EXAMPLE 6

### Identity to the human mRNA and human EST, and comparison of the length of the 5'-end.

The 5'-end and 3'-end sequences of the cloned sequence was judged to be identical to those of mRNA from human or other species when the sequence to compare has the length of 200 bases or longer, and the obtained homology is 94% or more. The 5'-end and 3'-end sequences of the cloned sequence was judged to be identical to those of human EST when the sequence to compare has the length of 200 bases or longer, and the obtained homology is 90% or more.

The sequence of the clone was judged to be full-length in comparison with human mRNA when the sequence has longer 5'-end, or it contains the translation initiation site. The sequence of the clone was judged to be full-length in comparison with human EST in the database when the sequence has longer 5'-end, or while it has shorter end, the difference in length between the two sequences is 50 bases or less. The other clones were judged to be not full-length.

### EXAMPLE 7

### Prediction of the fullness ratio by the ATGpr.

The score in the ATGpr1 is the expectation to be full-length based on calculations, and the higher score reflects the higher probability to be full-length as shown in Example 3. The maximal ATGpr1 score and the maximal ATGpr2 score represent the score obtained with all the initiation codons contained in the 5'-end sequence of the cloned sequence, and were used for the characterization.

### EXAMPLE 8

### Prediction of the novelty using the number of the identical ESTs by the homology search.

For both the 5'-end and 3'-end sequences of the clones, the number of the identical ESTs was determined by the homology search on the GenBank. Human ESTs were judged to be identical when the EST has a sequence of 200 nucleotides or more with 90% or more matching with the 5'-end sequence. The number of the identical ESTs were used for characterization and as an index of novelty. The clone having not identical sequence at the 5'-end and 3'-end sequences to those of mRNA as well as those of ESTs is a gene encoding a novel protein. Similarly, a clone having either the 5'-end or the 3'-end sequences, which has low number of the identical ESTs, is judged to be a gene encoding a novel protein.

### EXAMPLE 9

### Characterization of clusters.

The clusters of the groups of the 5'-end and 3'-end sequences were characterized according to the following criteria.
(1) Whether it is identical to the mRNA sequences from human or other species (including authorized sequences), or human ESTs by the BLAST search of the GenBank.
   A cluster containing at least one sequence of all the 5'-end and 3'-end sequences, which is identical to one of the mRNA sequences, was regarded to be the same cluster of the mRNA sequence.
(2) Whether it has longer 5'-end than human mRNA sequence and human ESTs.
   When all the 5'-end sequences contained in a cluster are judged to be not full-length compared with the mRNA sequences and human ESTs, the cluster was regarded as being not full-length.
(3) The scores in the ATGpr1 and ATGpr2 using all the initiation codons contained in the 5'-end sequences.
   The maximal ATGpr1 score among those of all the 5'-end sequences in a cluster was determined as the ATGpr1 score of the cluster. The ATGpr2 score of the cluster was also determined in the same way.
(4) The number of the identical human ESTs determined by the BLAST search of the GenBank.

The maximum number was determined in the numbers of ESTs identical to each of 5'-end sequences contained in a cluster. The number of the ESTs identical to the 5'-end sequences in the cluster was defined as the maximum number. The number of the ESTs identical to the 3'-end sequences in a cluster was determined in the same way.

### EXAMPLE 10

### Methods for selection of the clusters by the characteristics.

Data obtained by the characterization described above was used to discard the clusters that are identical to any mRNA sequence from human and other species (including authorized sequences), or those clusters that are not full-length. From the rest of the clusters, the clusters that fulfill any of the following conditions were selected.
(a) A cluster in which the number of the identical ESTs for the 5'-end sequence is 20 or less, and the ATGpr1 score exceeds 0.3.
(b) A cluster having the ATGpr1 score not more than 0.3, in which the number of the identical ESTs for both the 5'-end sequence and the 3'-end sequence is 5 or less, and multiple clones are contained.
(c) A cluster having the ATGpr1 score not more than 0.3, in which the number of the identical ESTs for the 5'-end sequence is 0, and the number of the identical ESTs for the 3'-end sequence is not less than 1.
(d) A cluster having the ATGpr1 score not more than 0.3, in which the number of the identical ESTs for the 5'-end sequence is not less than 1 and not more than 5, and the number of the identical ESTs for the 3'-end sequence is 0.

The clusters selected by (a) contain at least one clone that is novel and having high fullness ratio. The clusters selected by (b), (c), and (d) contain at least one clone that is novel and having low fullness ratio, but is still full-length.

### EXAMPLE 11

### Methods for selection of clones from clusters.

In the clusters comprising a single clone, the clone was selected.

In the clusters comprising multiple clones, in which multiple clones have the ATGpr1 score higher than 0.3, a clone with the highest score was selected.

In the clusters comprising multiple clones, in which multiple clones have the ATGpr1 score not more than 0.3, a clone with the highest ATGpr2 score was selected, if the score was higher than 0.3.

In the clusters comprising multiple clones, in which the clones have the scores not more than 0.3 in both the ATGpr1 and the ATGpr2, a clone with the highest scores in both the ATGpr1 and ATGpr2 was selected.

In the clusters comprising multiple clones, in which the above selection by the ATGpr score was not applicable, selected was a clone having longer 5'-end by assembling the 5'-end sequence, 3'-end sequence, and human ESTs. For assembling, the Sequencher™ (Hitachi Soft Engineering ) was used. When even the selection by assembling failed, all the clones were judged to be full-length.

As a result, 3690 clones were the clones that have the maximal ATGpr1 score higher than 0.3. On the other hand, 477 clones were the clones that have the maximal ATGpr1 score not more than 0.3, and the maximal ATGpr2 score higher than 0.3. The number of the clones having the highest scores in both the ATGpr1 and ATGpr2, while the scores were not more than 0.3, were 97. The number of the clones which were not selected by the ATGpr scores, but were selected by assembling the 5'-end sequence, 3'-end sequence, and human ESTs, were 117. The clones that have the score in both the ATGpr1 and ATGpr2 not more than 0.3, but were selected because the cluster comprises a single clone, were 1166. In the clones, at least either of the 5'-end or 3'-end sequence was not identical to any of human ESTs. Some clones were selected because the cluster comprises a single clone, or by assembling, in which there is no ATG codon (9 clones: HEMBA1001960, HEMBA106569, HEMBB1001454, NT2PR2002839, NT2RP2005325, NT2RP2006323, PLACE1004506, PLACE1005526, and THYRO1001177). The sequences that do not contain the ATG codon were considered to be corresponding to the 5'-UTR. Although the clones do not have the scores in the ATGpr1 and ATGpr2, the clones were yet judged to be full-length according to the fullness ratio, as shown in Table 4, 5, 6, 7, and 8. The above clones that were finally judged to be full-length were classified into 11 groups according to the following criteria.
Group (1): 1516 clones
   Among the 3690 clones having the maximal ATGpr1 score higher than 0.3, the following 1516 clones were having high fullness ratio and a novel clone, in which at least either of the 5'-end or 3'-end sequence, or both of them were not identical to any of human ESTs.
   HEMBA1000046, HEMBA1000050, HEMBA1000129, HEMBA1000150, HEMBA1000158, HEMBA1000193 , HEMBA1000201, HEMBA1000216, HEMBA1000227, HEMBA1000288, HEMBA1000290, HEMBA100030 3, HEMBA1000304, HEMBA1000369, HEMBA1000392, HEMBA1000396, HEMBA1000488, HEMBA10005 05, HEMBA1000508, HEMBA1000534, HEMBA1000542, HEMBA1000594, HEMBA1000637, HEMBA1000 657, HEMBA1000752, HEMBA1000867, HEMBA1000869, HEMBA1000872, HEMBA1000910, HEMBA100 0918, HEMBA1000919, HEMBA1000942, HEMBA1000968, HEMBA1000975, HEMBA1000986, HEMBA10 01022, HEMBA1001043, HEMBA1001052, HEMBA1001080, HEMBA1001085, HEMBA1001109, HEMBA1 001140, HEMBA1001174, HEMBA1001235, HEMBA1001286, HEMBA1001302, HEMBA1001398, HEMBA 1001407, HEMBA1001415, HEMBA1001446, HEMBA1001476, HEMBA1001497, HEMBA1001510, HEMB A1001533, HEMBA1001570, HEMBA1001581, HEMBA1001635, HEMBA1001640, HEMBA1001647, HEM BA1001661, HEMBA1001731, HEMBA1001744, HEMBA1001746, HEMBA1001800, HEMBA1001815, HE MBA1001822, HEMBA1001866, HEMBA1001896, HEMBA1001910, HEMBA1001987,
   HEMBA1002018, HEMBA1002035, HEMBA1002049, HEMBA1002092, HEMBA1002119, HEMBA1002125 , HEMBA1002161, HEMBA1002177, HEMBA1002189, HEMBA1002191, HEMBA1002199, HEMBA100222 9, HEMBA1002237, HEMBA1002265, HEMBA1002363, HEMBA1002417, HEMBA1002419, HEMBA10024 30, HEMBA1002439, HEMBA1002477, HEMBA1002503, HEMBA1002508, HEMBA1002515, HEMBA1002 547, HEMBA1002688, HEMBA1002703, HEMBA1002746, HEMBA1002750, HEMBA1002850, HEMBA100 2973, HEMBA1003021, HEMBA1003067, HEMBA1003077, HEMBA1003078, HEMBA1003079, HEMBA10 03117, HEMBA1003129, HEMBA1003175, HEMBA1003199, HEMBA1003235, HEMBA1003250, HEMBA1 003257, HEMBA1003291, HEMBA1003322, HEMBA1003327, HEMBA1003370, HEMBA1003380, HEMBA 1003395, HEMBA1003402, HEMBA1003461, HEMBA1003480, HEMBA1003538, HEMBA1003545, HEMB A1003556, HEMBA1003581, HEMBA1003621, HEMBA1003645, HEMBA1003667, HEMBA1003720, HEM BA1003760, HEMBA1003799, HEMBA1003807, HEMBA1003827, HEMBA1003836, HEMBA1003866, HE MBA1003879, HEMBA1003880, HEMBA1003985, HEMBA1003989,
   HEMBA1004011, HEMBA1004048, HEMBA1004056, HEMBA1004074, HEMBA1004133, HEMBA1004146 , HEMBA1004150, HEMBA1004199, HEMBA1004200, HEMBA1004238, HEMBA1004246, HEMBA100427 5, HEMBA1004286, HEMBA1004289, HEMBA1004321, HEMBA1004327, HEMBA1004335, HEMBA10043 41, HEMBA1004372, HEMBA1004389, HEMBA1004479, HEMBA1004499, HEMBA1004507, HEMBA1004 542, HEMBA1004554, HEMBA1004573, HEMBA1004596, HEMBA1004632, HEMBA1004697, HEMBA100 4705, HEMBA1004709, HEMBA1004711, HEMBA1004736, HEMBA1004751, HEMBA1004752, HEMBA10 04753, HEMBA1004756, HEMBA1004758, HEMBA1004768, HEMBA1004771, HEMBA1004795, HEMBA1 004806, HEMBA1004850, HEMBA1004863, HEMBA1004889, HEMBA1004923, HEMBA1004929, HEMBA 1004930, HEMBA1004944, HEMBA1004972, HEMBA1004980, HEMBA1005019, HEMBA1005035, HEMB A1005050, HEMBA1005066, HEMBA1005075, HEMBA1005079, HEMBA1005083, HEMBA1005113, HEM BA1005133, HEMBA1005149, HEMBA1005219, HEMBA1005331, HEMBA1005338, HEMBA1005367, HE MBA1005411, HEMBA1005468, HEMBA1005469, HEMBA1005517, HEMBA1005518, HEMBA1005526, H EMBA1005548, HEMBA1005576, HEMBA1005583, HEMBA1005595, HEMBA1005609, HEMBA1005685, HEMBA1005732, HEMBA1005755, HEMBA1005813, HEMBA1005815, HEMBA1005834, HEMBA1005884 , HEMBA1005963, HEMBA1005991,
   HEMBA1006031, HEMBA1006100, HEMBA1006121, HEMBA1006138, HEMBA1006173, HEMBA1006182 , HEMBA1006198, HEMBA1006252, HEMBA1006272, HEMBA1006278, HEMBA1006291, HEMBA100629 3, HEMBA1006344, HEMBA1006349, HEMBA1006377, HEMBA1006381, HEMBA1006398, HEMBA10064 24, HEMBA1006467, HEMBA1006474, HEMBA1006483, HEMBA1006492, HEMBA1006494, HEMBA1006 497, HEMBA1006502, HEMBA1006530, HEMBA1006579, HEMBA1006583, HEMBA1006643, HEMBA100 6674, HEMBA1006682, HEMBA1006709, HEMBA1006717, HEMBA1006737, HEMBA1006754, HEMBA10 06758, HEMBA1006767, HEMBA1006795, HEMBA1006796, HEMBA1006807, HEMBA1006832, HEMBA1 006900, HEMBA1006973, HEMBA1006976, HEMBA1006993, HEMBA1006996, HEMBA1007002, HEMBA 1007052, HEMBA1007062, HEMBA1007066, HEMBA1007151, HEMBA1007203, HEMBA1007281, HEMB A1007300, HEMBA1007320, HEMBA1007342,
   HEMBB1000008, HEMBB1000024, HEMBB1000025, HEMBB1000083, HEMBB1000103, HEMBB1000173 , HEMBB1000175, HEMBB1000198, HEMBB1000240, HEMBB1000244, HEMBB1000338, HEMBB100033 9, HEMBB1000391, HEMBB1000438, HEMBB1000510, HEMBB1000550, HEMBB1000556, HEMBB10005 89, HEMBB1000591, HEMBB1000593, HEMBB1000632, HEMBB1000671, HEMBB1000673, HEMBB1000 693, HEMBB1000706, HEMBB1000725, HEMBB1000781, HEMBB1000810, HEMBB1000826, HEMBB100 0835, HEMBB1000848, HEMBB1000852, HEMBB1000870, HEMBB1000887, HEMBB1000908, HEMBB10 00927, HEMBB1000947, HEMBB1000973, HEMBB1000991, HEMBB1001011, HEMBB1001014, HEMBB1 001020, HEMBB1001024, HEMBB1001058, HEMBB1001096, HEMBB1001105, HEMBB1001126, HEMBB 1001169, HEMBB1001199, HEMBB1001314, HEMBB1001339, HEMBB1001346, HEMBB1001364, HEMB B1001369, HEMBB1001394, HEMBB1001410, HEMBB1001426, HEMBB1001449, HEMBB1001482, HEM BB1001531, HEMBB1001562, HEMBB1001564, HEMBB1001585, HEMBB1001635, HEMBB1001653, HE MBB1001665, HEMBB1001668, HEMBB1001685, HEMBB1001707, HEMBB1001760, HEMBB1001785, H EMBB1001812, HEMBB1001816, HEMBB1001831, HEMBB1001834, HEMBB1001839, HEMBB1001872, HEMBB1001874, HEMBB1001908, HEMBB1001910, HEMBB1001915, HEMBB1001950, HEMBB1001957 , HEMBB1001962,
   HEMBB1002044, HEMBB1002050, HEMBB1002068, HEMBB1002142, HEMBB1002152, HEMBB1002193 , HEMBB1002217, HEMBB1002218, HEMBB1002249, HEMBB1002327, HEMBB1002340, HEMBB100235 8, HEMBB1002359, HEMBB1002415, HEMBB1002442, HEMBB1002457, HEMBB1002492, HEMBB10024 95, HEMBB1002502, HEMBB1002545, HEMBB1002614, HEMBB1002684, HEMBB1002692, MAMMA1000019, MAMMA1000020, MAMMA1000025, MAMMA1000057, MAMMA1000069, MAMMA1000084 , MAMMA1000139, MAMMA1000143, MAMMA1000171, MAMMA1000183, MAMMA1000251, MAMMA100027 7, MAMMA1000279, MAMMA1000309, MAMMA1000312, MAMMA1000313, MAMMA1000339, MAMMA10003 61, MAMMA1000372, MAMMA1000388, MAMMA1000410, MAMMA1000421, MAMMA1000458, MAMMA1000 472, MAMMA1000524, MAMMA1000567, MAMMA1000583, MAMMA1000623, MAMMA1000664, MAMMA100 0672, MAMMA1000684, MAMMA1000713, MAMMA1000731, MAMMA1000746, MAMMA1000760, MAMMA10 00776, MAMMA1000842, MAMMA1000843, MAMMA1000856, MAMMA1000865, MAMMA1000875, MAMMA1 000897, MAMMA1000906, MAMMA1000908, MAMMA1000914, MAMMA1000921, MAMMA1000956, MAMMA 1000968, MAMMA1000979, MAMMA1001078, MAMMA1001080, MAMMA1001091, MAMMA1001110, MAMM A1001126, MAMMA1001143, MAMMA1001154, MAMMA1001181, MAMMA1001215, MAMMA1001222, MAM MA1001244, MAMMA1001260, MAMMA1001296, MAMMA1001305, MAMMA1001343, MAMMA1001346, MA MMA1001388, MAMMA1001411, MAMMA1001419, MAMMA1001465, MAMMA1001487, MAMMA1001510, M AMMA1001522, MAMMA1001551, MAMMA1001600, MAMMA1001604, MAMMA1001620, MAMMA1001627, MAMMA1001630, MAMMA1001633, MAMMA1001635, MAMMA1001692, MAMMA1001743, MAMMA1001751 , MAMMA1001757, MAMMA1001764, MAMMA1001768, MAMMA1001783, MAMMA1001790, MAMMA100181 7, MAMMA1001824, MAMMA1001848, MAMMA1001851, MAMMA1001858, MAMMA1001864, MAMMA10018 68, MAMMA1001874, MAMMA1001956, MAMMA1001969, MAMMA1001970,
   MAMMA1002009, MAMMA1002033, MAMMA1002042, MAMMA1002047, MAMMA1002068, MAMMA1002153 , MAMMA1002156, MAMMA1002170, MAMMA1002174, MAMMA1002243, MAMMA1002268, MAMMA100226 9, MAMMA1002294, MAMMA1002308, MAMMA1002312, MAMMA1002317, MAMMA1002319, MAMMA10023 29, MAMMA1002333, MAMMA1002353, MAMMA1002355, MAMMA1002356, MAMMA1002362, MAMMA1002 380, MAMMA1002384, MAMMA1002413, MAMMA1002427, MAMMA1002454, MAMMA1002524, MAMMA100 2554, MAMMA1002590, MAMMA1002617, MAMMA1002655, MAMMA1002665, MAMMA1002671, MAMMA10 02673, MAMMA1002685, MAMMA1002699, MAMMA1002711, MAMMA1002782, MAMMA1002807, MAMMA1 002842, MAMMA1002843, MAMMA1002868, MAMMA1002881, MAMMA1002886, MAMMA1002895, MAMMA 1002938, MAMMA1002964, MAMMA1003013, MAMMA1003015, MAMMA1003044, MAMMA1003049, MAMM A1003056, MAMMA1003066, MAMMA1003099, MAMMA1003104, MAMMA1003127,
   NT2RM1000018, NT2RM1000059, NT2RM1000118, NT2RM1000131, NT2RM1000186, NT2RM1000242 , NT2RM1000252, NT2RM1000260, NT2RM1000271, NT2RM1000314, NT2RM1000318, NT2RM100035 4, NT2RM1000399, NT2RM1000421, NT2RM1000563, NT2RM1000623, NT2RM1000666, NT2RM10007 25, NT2RM1000742, NT2RM1000772, NT2RM1000802, NT2RM1000811, NT2RM1000850, NT2RM1000 885, NT2RM1000894, NT2RM1000978, NT2RM1001059, NT2RM1001066, NT2RM1001072, NT2RM100 1082, NT2RM1001085, NT2RM1001092, NT2RM1001115,
   NT2RM2000042, NT2RM2000124, NT2RM2000250, NT2RM2000259, NT2RM2000363, NT2RM2000374 , NT2RM2000469, NT2RM2000556, NT2RM2000566, NT2RM2000567, NT2RM2000588, NT2RM200059 4, NT2RM2000599, NT2RM2000623, NT2RM2000635, NT2RM2000636, NT2RM2000718, NT2RM20007 95, NT2RM2000951, NT2RM2001004, NT2RM2001105, NT2RM2001141, NT2RM2001247, NT2RM2001 312, NT2RM2001324, NT2RM2001499, NT2RM2001544, NT2RM2001547, NT2RM2001575, NT2RM200 1588, NT2RM2001641, NT2RM2001652, NT2RM2001659, NT2RM2001670, NT2RM2001698, NT2RM20 01699, NT2RM2001727, NT2RM2001753, NT2RM2001771, NT2RM2001797, NT2RM2001805, NT2RM2 001930, NT2RM2001982, NT2RM2001983, NT2RM2001989, NT2RM2002014,
   NT2RM4000167, NT2RM4000199, NT2RM4000202, NT2RM4000215, NT2RM4000251, NT2RM4000386 , NT2RM4000433, NT2RM4000486, NT2RM4000496, NT2RM4000534, NT2RM4000585, NT2RM400059 0, NT2RM4000595, NT2RM4000616, NT2RM4000700, NT2RM4000712, NT2RM4000733, NT2RM40007 34, NT2RM4000787, NT2RM4000795, NT2RM4000796, NT2RM4000798, NT2RM4000820, NT2RM4000 848, NT2RM4000887, NT2RM4000971, NT2RM4000996, NT2RM4001054, NT2RM4001191, NT2RM400 1203, NT2RM4001217, NT2RM4001320, NT2RM4001344, NT2RM4001454, NT2RM4001455, NT2RM40 01522, NT2RM4001592, NT2RM4001594, NT2RM4001629, NT2RM4001650, NT2RM4001662, NT2RM4 001666, NT2RM4001710, NT2RM4001746, NT2RM4001813, NT2RM4001842, NT2RM4001969, NT2RM 4001987, NT2RM4002054, NT2RM4002063, NT2RM4002066, NT2RM4002073, NT2RM4002075, NT2R M4002093, NT2RM4002109, NT2RM4002174, NT2RM4002194, NT2RM4002205, NT2RM4002266, NT2 RM4002301, NT2RM4002339, NT2RM4002373, NT2RM4002374, NT2RM4002409, NT2RM4002446, NT 2RM4002457, NT2RM4002460, NT2RM4002482, NT2RM4002493, NT2RM4002527, NT2RM4002558, NT2RP1000357, NT2RP1000376, NT2RP1000409, NT2RP1000460, NT2RP1000581, NT2RP1000677 NT2RP1000721, NT2RP1000733, NT2RP1000796, NT2RP1000836, NT2RP1000846, NT2RP100091 6, NT2RP1001079, NT2RP1001185, NT2RP1001247, NT2RP1001494, NT2RP2000067, NT2RP20000 70, NT2RP2000133, NT2RP2000173, NT2RP2000175, NT2RP2000195, NT2RP2000208, NT2RP2000 232, NT2RP2000233, NT2RP2000283, NT2RP2000297, NT2RP2000310, NT2RP2000412, NT2RP200 0422, NT2RP2000503, NT2RP2000516, NT2RP2000656, NT2RP2000658, NT2RP2000704, NT2RP20 00814, NT2RP2000816, NT2RP2000841, NT2RP2000938, NT2RP2001036, NT2RP2001081, NT2RP2 001094, NT2RP2001119, NT2RP2001137, NT2RP2001173, NT2RP2001174, NT2RP2001392, NT2RP 2001427, NT2RP2001440, NT2RP2001450, NT2RP2001511, NT2RP2001536, NT2RP2001560, NT2R P2001581, NT2RP2001601, NT2RP2001613, NT2RP2001748, NT2RP2001861, NT2RP2001900, NT2 RP2001946, NT2RP2001976, NT2RP2001985, NT2RP2001991,
   NT2RP2002025, NT2RP2002079, NT2RP2002154, NT2RP2002231, NT2RP2002252, NT2RP2002333, NT2RP2002373, NT2RP2002464, NT2RP2002609, NT2RP2002621, NT2RP2002672, NT2RP200270 6, NT2RP2002769, NT2RP2002800, NT2RP2002891, NT2RP2002959, NT2RP2003099, NT2RP20031 17, NT2RP2003121, NT2RP2003165, NT2RP2003307, NT2RP2003308, NT2RP2003367, NT2RP2003 401, NT2RP2003446, NT2RP2003513, NT2RP2003533, NT2RP2003564, NT2RP2003581, NT2RP200 3687, NT2RP2003691, NT2RP2003751, NT2RP2003760, NT2RP2003764, NT2RP2003769, NT2RP20 03857, NT2RP2003952, NT2RP2003986,
   NT2RP2004041, NT2RP2004239, NT2RP2004240, NT2RP2004366, NT2RP2004373, NT2RP2004538 , NT2RP2004551, NT2RP2004568, NT2RP2004600, NT2RP2004743, NT2RP2004841, NT2RP200486 1, NT2RP2004959, NT2RP2004978, NT2RP2005000, NT2RP2005018, NT2RP2005022, NT2RP20050 38, NT2RP2005116, NT2RP2005144, NT2RP2005147, NT2RP2005239, NT2RP2005472, NT2RP2005 496, NT2RP2005555, NT2RP2005557, NT2RP2005600, NT2RP2005605, NT2RP2005622, NT2RP200 5635, NT2RP2005637, NT2RP2005654, NT2RP2005683, NT2RP2005690, NT2RP2005723, NT2RP20 05767, NT2RP2005775, NT2RP2005781, NT2RP2005804, NT2RP2005812, NT2RP2005853, NT2RP2 005868, NT2RP2006038, NT2RP2006052, NT2RP2006069, NT2RP2006200, NT2RP2006275, NT2RP 2006333, NT2RP2006334, NT2RP2006436, NT2RP2006464, NT2RP2006571, NT2RP2006573, NT2RP3000002, NT2RP3000046, NT2RP3000050, NT2RP3000068, NT2RP3000072, NT2RP3000080 , NT2RP3000092, NT2RP3000134, NT2RP3000207, NT2RP3000220, NT2RP3000251, NT2RP300025 5, NT2RP3000299, NT2RP3000324, NT2RP3000348, NT2RP3000397, NT2RP3000439, NT2RP30004 84, NT2RP3000531, NT2RP3000592, NT2RP3000603, NT2RP3000632, NT2RP3000644, NT2RP3000 661, NT2RP3000665, NT2RP3000690, NT2RP3000753, NT2RP3000825, NT2RP3000841, NT2RP300 0845, NT2RP3000852, NT2RP3000868, NT2RP3000869, NT2RP3001096, NT2RP3001107, NT2RP30 01109, NT2RP3001113, NT2RP3001116, NT2RP3001120, NT2RP3001133, NT2RP3001155, NT2RP3 001176, NT2RP3001236, NT2RP3001239, NT2RP3001272, NT2RP3001307, NT2RP3001338, NT2RP 3001398, NT2RP3001420, NT2RP3001428, NT2RP3001447, NT2RP3001453, NT2RP3001495, NT2R P3001554, NT2RP3001608, NT2RP3001629, NT2RP3001678, NT2RP3001688, NT2RP3001708, NT2 RP3001739, NT2RP3001764, NT2RP3001799, NT2RP3001819, NT2RP3001844, NT2RP3001855, NT 2RP3001915, NT2RP3001931, NT2RP3001944,
   NT2RP3002007, NT2RP3002063, NT2RP3002081, NT2RP3002108, NT2RP3002163, NT2RP3002304, NT2RP3002351, NT2RP3002587, NT2RP3002603, NT2RP3002650, NT2RP3002659, NT2RP300266 3, NT2RP3002671, NT2RP3002701, NT2RP3002785, NT2RP3002818, NT2RP3002869, NT2RP30028 76, NT2RP3002877, NT2RP3002911, NT2RP3002953, NT2RP3002985, NT2RP3002988, NT2RP3003 139, NT2RP3003157, NT2RP3003193, NT2RP3003282, NT2RP3003302, NT2RP3003344, NT2RP300 3353, NT2RP3003377, NT2RP3003385, NT2RP3003409, NT2RP3003621, NT2RP3003656, NT2RP30 03701, NT2RP3003726, NT2RP3003795, NT2RP3003846, NT2RP3004078, NT2RP3004155, NT2RP3 004206, NT2RP3004242, NT2RP3004341, NT2RP3004428, NT2RP3004451, NT2RP3004454, NT2RP 3004490, NT2RP3004534, NT2RP3004544, NT2RP3004569, NT2RP3004572, NT2RP3004594, NT2R P3004617, NT2RP3004618, NT2RP3004669, NT2RP3004670,
   NT2RP4000051, NT2RP4000111, NT2RP4000159, NT2RP4000210, NT2RP4000290, NT2RP4000323 , NT2RP4000355, NT2RP4000360, NT2RP4000398, NT2RP4000448, NT2RP4000457, NT2RP400048 0, NT2RP4000481, NT2RP4000541, NT2RP4000560, NT2RP4000638, NT2RP4000728, NT2RP40007 37, NT2RP4000739, NT2RP4000817, NT2RP4000837, NT2RP4000865, NT2RP4000925, NT2RP4000 927, NT2RP4000929, NT2RP4000955, NT2RP4000973, NT2RP4001004, NT2RP4001029, NT2RP400 1057, NT2RP4001064, NT2RP4001078, NT2RP4001080, NT2RP4001086, NT2RP4001138, NT2RP40 01150, NT2RP4001313, NT2RP4001345, NT2RP4001351, NT2RP4001353, NT2RP4001372, NT2RP4 001373, NT2RP4001379, NT2RP4001433, NT2RP4001483, NT2RP4001551, NT2RP4001614, NT2RP 4001634, NT2RP4001656, NT2RP4001725, NT2RP4001790, NT2RP4001896, NT2RP4001938, NT2R P4001966, NT2RP4002047, NT2RP4002052, NT2RP4002058, NT2RP4002081, NT2RP5003459, NT2 RP5003500, NT2RP5003506, NT2RP5003524,
   OVARC1000014, OVARC1000035, OVARC1000087, OVARC1000109, OVARC1000114, OVARC1000148 , OVARC1000151, OVARC1000241, OVARC1000326, OVARC1000347, OVARC1000408, OVARC100042 0, OVARC1000437, OVARC1000442, OVARC1000473, OVARC1000520, OVARC1000556, OVARC10005 57, OVARC1000588, OVARC1000605, OVARC1000622, OVARC1000640, OVARC1000649, OVARC1000 661, OVARC1000681, OVARC1000689, OVARC1000787, OVARC1000800, OVARC1000850, OVARC100 0890, OVARC1000912, OVARC1000945, OVARC1000959, OVARC1000996, OVARC1000999, OVARC10 01004, OVARC1001010, OVARC1001032, OVARC1001044, OVARC1001074, OVARC1001162, OVARC1 001173, OVARC1001180, OVARC1001200, OVARC1001243, OVARC1001306, OVARC1001344, OVARC 1001369, OVARC1001372, OVARC1001376, OVARC1001391, OVARC1001399, OVARC1001417, OVAR C1001425, OVARC1001436, OVARC1001453, OVARC1001489, OVARC1001506, OVARC1001525, OVA RC1001611, OVARC1001703, OVARC1001731, OVARC1001762, OVARC1001802, OVARC1001828, OV ARC1001861, OVARC1001942, OVARC1001949, OVARC1001950, OVARC1002082, OVARC1002107, O VARC1002158, OVARC1002165,
   PLACE1000004, PLACE1000005, PLACE1000061, PLACE1000081, PLACE1000236, PLACE1000401 , PLACE1000435, PLACE1000444, PLACE1000547, PLACE1000564, PLACE1000716, PLACE100094 8, PLACE1000972, PLACE1001010, PLACE1001024, PLACE1001036, PLACE1001062, PiACE10011 04, PLACE1001171, PLACE1001257, PLACE1001280, PLACE1001311, PLACE1001366, PLACE1001 456, PLACE1001484, PLACE1001545, PLACE1001634, PLACE1001729, PLACE1001739, PLACE100 1740, PLACE1001771, PLACE1001810, PLACE1001912, PLACE1001928, PLACE1001983, PLACE1002004, PLACE1002073, PLACE1002119, PLACE1002163, PLACE1002170, PLACE1002213 , PLACE1002433, PLACE1002437, PLACE1002438, PLACE1002450, PLACE1002474, PLACE100252 9, PLACE1002625, PLACE1002655, PLACE1002665, PLACE1002685, PLACE1002714, PLACE10027 75, PLACE1002839, PLACE1002941, PLACE1003044, PLACE1003045, PLACE1003108, PLACE1003 145, PLACE1003174, PLACE1003190, PLACE1003249, PLACE1003256, PLACE1003334, PLACE100 3366, PLACE1003369, PLACE1003383, PLACE1003493, PLACE1003611, PLACE1003709, PLACE10 03711, PLACE1003771, PLACE1003784, PLACE1003795, PLACE1003870, PLACE1003885, PLACE1 003888, PLACE1003936,
   PLACE1004128, PLACE1004156, PLACE1004161, PLACE1004203, PLACE1004270, PLACE1004277 , PLACE1004289, PLACE1004302, PLACE1004388, PLACE1004428, PLACE1004451, PLACE100446 0, PLACE1004516, PLACE1004548, PLACE1004564, PLACE1004646, PLACE1004664, PLACE10046 86, PLACE1004691, PLACE1004722, PLACE1004740, PLACE1004743, PLACE1004751, PLACE1004 804, PLACE1004824, PLACE1004885, PLACE1004918, PLACE1004937, PLACE1005026, PLACE100 5046, PLACE1005206, PLACE1005232, PLACE1005243, PLACE1005266, PLACE1005277, PLACE10 05331, PLACE1005480, PLACE1005481, PLACE1005494, PLACE1005530, PLACE1005554, PLACE1 005646, PLACE1005656, PLACE1005755, PLACE1005763, PLACE1005799, PLACE1005804, PLACE 1005828, PLACE1005876, PLACE1005890, PLACE1005923, PLACE1005925, PLACE1005934, PLAC E1005951, PLACE1005953, PLACE1005990,
   PLACE1006011, PLACE1006037, PLACE1006170, PLACE1006195, PLACE1006357, PLACE1006385 , PLACE1006412, PLACE1006414, PLACE1006438, PLACE1006482, PLACE1006552, PLACE100659 8, PLACE1006673, PLACE1006704, PLACE1006782, PLACE1006829, PLACE1006932, PLACE10069 35, PLACE1006956, PLACE1006958, PLACE1006962, PLACE1007014, PLACE1007068, PLACE1007 112, PLACE1007226, PLACE1007242, PLACE1007257, PLACE1007274, PLACE1007301, PLACE100 7342, PLACE1007346, PLACE1007450, PLACE1007452, PLACE1007484, PLACE1007488, PLACE10 07524, PLACE1007537, PLACE1007618, PLACE1007729, PLACE1007810, PLACE1007946, PLACE1008000, PLACE1008002, PLACE1008095, PLACE1008177, PLACE1008273, PLACE1008275 , PLACE1008280, PLACE1008329, PLACE1008457, PLACE1008465, PLACE1008488, PLACE100853 3, PLACE1008626, PLACE1008693, PLACE1008696, PLACE1008790, PLACE1008813, PLACE10088 54, PLACE1008867, PLACE1008887, PLACE1008925, PLACE1009090, PLACE1009094, PLACE1009 110, PLACE1009111, PLACE1009113, PLACE1009158, PLACE1009174, PLACE1009190, PLACE100 9230, PLACE1009328, PLACE1009335, PLACE1009368, PLACE1009375, PLACE1009388, PLACE10 09404, PLACE1009434, PLACE1009443, PLACE1009459, PLACE1009524, PLACE1009542, PLACE1 009607, PLACE1009798, PLACE1009861, PLACE1009971, PLACE1009992, PLACE1009997, PLACE 1010148, PLACE1010261, PLACE1010293, PLACE1010310, PLACE1010321, PLACE1010324, PLAC E1010329, PLACE1010401, PLACE1010481, PLACE1010547, PLACE1010616, PLACE1010629, PLA CE1010714, PLACE1010743, PLACE1010900, PLACE1010926, PLACE1010954, PLACE1011046, PL ACE1011054, PLACE1011056, PLACE1011114, PLACE1011229, PLACE1011273, PLACE1011399, P LACE1011635, PLACE1011725, PLACE1011783, PLACE1011875, PLACE1011891, PLACE1011896, PLACE1011922, PLACE1012031,
   PLACE2000006, PLACE2000014, PLACE2000015, PLACE2000033, PLACE2000034, PLACE2000039 , PLACE2000047, PLACE2000050, PLACE2000062, PLACE2000072, PLACE2000097, PLACE200010 0, PLACE2000111, PLACE2000124, PLACE2000170, PLACE2000223, PLACE2000235, PLACE20002 46, PLACE2000274, PLACE2000359, PLACE2000371, PLACE2000373, PLACE2000411, PLACE2000 433, PLACE2000438, PLACE2000458,
   PLACE3000020, PLACE3000029, PLACE3000059, PLACE3000070, PLACE3000103, PLACE3000142 , PLACE3000145, PLACE3000147, PLACE3000155, PLACE3000157, PLACE3000194, PLACE300022 1, PLACE3000244, PLACE3000254, PLACE3000304, PLACE3000310, PLACE3000320, PLACE30003 22, PLACE3000331, PLACE3000350, PLACE3000352, PLACE3000363, PLACE3000405, PLACE3000 416, PLACE3000425, PLACE3000455,
   PLACE4000014, PLACE4000052, PLACE4000063, PLACE4000128, PLACE4000129, PLACE4000131 , PLACE4000147, PLACE4000156, PLACE4000192, PLACE4000211, PLACE4000222, PLACE400023 0, PLACE4000261, PLACE4000270, PLACE4000323, PLACE4000387, PLACE4000392, PLACE40004 50, PLACE4000465, PLACE4000487, PLACE4000489, PLACE4000521, PLACE4000522, PLACE4000 590, PLACE4000612, PLACE4000638, PLACE4000654,
   SKNMC1000011, SKNMC1000013, THYRO1000034, THYRO1000070, THYRO1000072, THYRO1000107 , THYRO1000124, THYRO1000163, THYRO1000253, THYRO1000343, THYRO1000381, THYRO100038 7, THYRO1000395, THYRO1000501, THYRO1000505, THYRO1000637, THYRO1000666, THYRO10006 76, THYRO1000684, THYRO1000734, THYRO1000787, THYRO1000916, THYRO1000934, THYRO1000 951, THYRO1001033, THYRO1001100, THYRO1001142, THYRO1001189, THYRO1001213, THYRO100 1320, THYRO1001374, THYRO1001403, THYRO1001458, THYRO1001487, THYRO1001602, THYRO10 01605, THYRO1001637, THYRO1001656, THYRO1001738, THYRO1001809, THYRO1001828, Y79AA1 000131, Y79AA1000268, Y79AA1000349, Y79AA1000355, Y79AA1000627, Y79AA1000824, Y79AA 1000827, Y79AA1000850, Y79AA1000962, Y79AA1000976, Y79AA1001068, Y79AA1001145, Y79A A1001185, Y79AA1001233, Y79AA1001299, Y79AA1001391, Y79AA1001541, Y79AA1001555, Y79 AA1001581, Y79AA1001613, Y79AA1001846, Y79AA1001866, Y79AA1001874, Y79AA1002115, Y7 9AA1002139, Y79AA1002229, Y79AA1002246, Y79AA1002298, Y79AA1002361, Y79AA1002399, Y 79AA1002431, Y79AA1002433, Y79AA1002472, Y79AA1002482,
Group (2): 377 clones
   Among the 3690 clones, the following 377 clones were full-length, and a novel clone, in which the number of the identical human ESTs for both the 5'-end and 3'-end sequences is 1 or higher and not more than 5.
   HEMBA1000156, HEMBA1000231, HEMBA1000244, HEMBA1000302, HEMBA1000523, HEMBA1000531 , HEMBA1000555, HEMBA1000568, HEMBA1000971, HEMBA1001009, HEMBA1001133, HEMBA100113 7, HEMBA1001257, HEMBA1001281, HEMBA1001289, HEMBA1001310, HEMBA1001377, HEMBA10013 88, HEMBA1001413, HEMBA1001455, HEMBA1001579, HEMBA1001702, HEMBA1001781, HEMBA1001 804, HEMBA1001819, HEMBA1001864, HEMBA1001869, HEMBA1001912, HEMBA1001915, HEMBA100 2084, HEMBA1002102, HEMBA1002212, HEMBA1002495, HEMBA1002513, HEMBA1002863, HEMBA10 02935, HEMBA1002970, HEMBA1002971, HEMBA1002997, HEMBA1002999, HEMBA1003035, HEMBA1 003041, HEMBA1003433, HEMBA1003568, HEMBA1003680, HEMBA1003692, HEMBA1003804, HEMBA 1003893, HEMBA1003953, HEMBA1004097, HEMBA1004207, HEMBA1004502, HEMBA1004560, HEMB A1004638, HEMBA1004693, HEMBA1004973, HEMBA1004977, HEMBA1005223, HEMBA1005244, HEM BA1005314, HEMBA1005680, HEMBA1005737, HEMBA1005746, HEMBA1005822, HEMBA1005891, HE MBA1006445, HEMBA1006535, HEMBA1006566, HEMBA1006877, HEMBA1006936, HEMBA1007178, H EMBA1007251, HEMBA1007267,
   HEMBB1000030, HEMBB1000215, HEMBB1000226, HEMBB1000449, HEMBB1000789, HEMBB1000975, HEMBB1001153, HEMBB1001175, HEMBB1001234, HEMBB1001387, HEMBB1001905, HEMBB100190 6, HEMBB1002300, HEMBB1002383, HEMBB1002489, HEMBB1002607, HEMBB1002705, MAMMA1000257, MAMMA1000416, MAMMA1000831, MAMMA1000841, MAMMA1001021, MAMMA1001041 , MAMMA1001105, MAMMA1001139, MAMMA1001198, MAMMA1001730, MAMMA1001837, MAMMA100219 8, MAMMA1002209, MAMMA1002219, MAMMA1002292, MAMMA1002297, MAMMA1002637, MAMMA10026 50, MAMMA1002796, MAMMA1002987, MAMMA1003135, MAMMA1003150,
   NT2RM1001008, NT2RM2001896, NT2RM4000024, NT2RM4000061, NT2RM4000155, NT2RM4000169, NT2RM4000229, NT2RM4000290, NT2RM4000354, NT2RM4000895, NT2RM4001032, NT2RM400104 7, NT2RM4001116, NT2RM4001140, NT2RM4001200, NT2RM4001384, NT2RM4001412, NT2RM40014 83, NT2RM4001715, NT2RM4001783, NT2RM4001979, NT2RM4002034, NT2RM4002044, NT2RM4002 145, NT2RM4002344, NT2RM4002452,
   NT2RP2000006, NT2RP2000008, NT2RP2000054, NT2RP2000079, NT2RP2000097, NT2RP2000126 NT2RP2000224, NT2RP2000274, NT2RP2000764, NT2RP2000812, NT2RP2000819, NT2RP200084 5, NT2RP2000863, NT2RP2000880, NT2RP2001127, NT2RP2001218, NT2RP2001233, NT2RP20013 94, NT2RP2001420, NT2RP2001576, NT2RP2001597, NT2RP2001721, NT2RP2001907, NT2RP2002 046, NT2RP2002312, NT2RP2002325, NT2RP2002503, NT2RP2002537, NT2RP2002862, NT2RP200 2925, NT2RP2002939, NT2RP2002954, NT2RP2002986, NT2RP2003034, NT2RP2003125, NT2RP20 03286, NT2RP2003293, NT2RP2003347, NT2RP2003433, NT2RP2003445, NT2RP2003543, NT2RP2 003781, NT2RP2004066, NT2RP2004316, NT2RP2004392, NT2RP2004897, NT2RP2004999, NT2RP 2005003, NT2RP2005393, NT2RP2005520, NT2RP2005722, NT2RP2005752, NT2RP2005763, NT2R P2006023, NT2RP2006186, NT2RP2006565,
   NT2RP3000527, NT2RP3000562, NT2RP3000590, NT2RP3000596, NT2RP3000599, NT2RP3001004 , NT2RP3001126, NT2RP3001150, NT2RP3001392, NT2RP3001407, NT2RP3001457, NT2RP300175 3, NT2RP3001929, NT2RP3001969, NT2RP3002151, NT2RP3002303, NT2RP3002484, NT2RP30025 90, NT2RP3002660, NT2RP3003078, NT2RP3003204, NT2RP3003251, NT2RP3003411, NT2RP3003 831, NT2RP3003870, NT2RP3003876, NT2RP3004093, NT2RP3004246, NT2RP3004253, NT2RP300 4348, NT2RP3004504, NT2RP3004507, NT2RP4000312, NT2RP4000518, NT2RP4000781, NT2RP40 00975, NT2RP4000997, NT2RP4002408, NT2RP5003512,
   OVARC1000060, OVARC1000411, OVARC1000440, OVARC1000461, OVARC1000703, OVARC1000846 , OVARC1000876, OVARC1000915, OVARC1001040, OVARC1001113, OVARC1001167, OVARC100117 0, OVARC1001188, OVARC1001271, OVARC1001329, OVARC1001610, OVARC1001767, OVARC10018 20, OVARC1001879, OVARC1002138,
   PLACE1000014, PLACE1000048, PLACE1000420, PLACE1000588, PLACE1000596, PLACE1000636 , PLACE1000755, PLACE1000786, PLACE1000849, PLACE1000909, PLACE1001000, PLACE100124 1, PLACE1001387, PLACE1001503, PLACE1001551, PLACE1001570, PLACE1001602, PLACE10016 10, PLACE1001632, PLACE1001989, PLACE1002115, PLACE1002465, PLACE1002532, PLACE1002 851, PLACE1002991, PLACE1002993, PLACE1003025, PLACE1003342, PLACE1003553, PLACE100 3605, PLACE1003669, PLACE1003723, PLACE1003738, PLACE1003762, PLACE1003783, PLACE10 04104, PLACE1004114, PLACE1004149, PLACE1004197, PLACE1004473, PLACE1004510, PLACE1 004645, PLACE1004672, PLACE1004868,
   PLACE1005313, PLACE1005374, PLACE1005834, PLACE1005921, PLACE1005936, PLACE1005966 , PLACE1006157, PLACE1006225, PLACE1006239, PLACE1006248, PLACE1006445, PLACE100653 1, PLACE1006626, PLACE1006731, PLACE1006901, PLACE1006961, PLACE1006966, PLACE10073 17, PLACE1007375, PLACE1007460, PLACE1007544, PLACE1007583, PLACE1007791, PLACE1007 843, PLACE1007897, PLACE1008129, PLACE1008309, PLACE1008401, PLACE1008402, PLACE100 8532, PLACE1008627, PLACE1008808, PLACE1008902, PLACE1008934, PLACE1009099, PLACE10 09130, PLACE1009166, PLACE1009659, PLACE1009665, PLACE1009708, PLACE1009794, PLACE1 009886, PLACE1009908, PLACE1010202, PLACE1010580, PLACE1010661, PLACE1010811, PLACE 1010917, PLACE1010942, PLACE1010965, PLACE1011026, PLACE1011325, PLACE1011472, PLAC E1011563, PLACE1011664, PLACE1011682, PLACE1011982, PLACE4000049, PLACE4000300, PLA CE4000558,
   THYRO1000368, THYRO1000662, THYRO1000712, THYRO1000783, THYRO1000952, THYRO1000988 THYRO1001661, THYRO1001854, Y79AA1000037, Y79AA1000059, Y79AA1000985, Y79AA100110, 5, Y79AA1001177, Y79AA1001211, Y79AA1001281, Y79AA1001533, Y79AA1001923, Y79AA10020 83, Y79AA1002204, Y79AA1002407,
Group (3): 1797 clones
   Among the 3690 clones, the following 1797 clones were full-length, and novel clones, in which the number of the identical human ESTs for the 5'-end sequence is not more than 20 (except clones in which at least either of the 5'-end or 3'-end sequence, or both of them are not identical to any of human ESTs, and clones in which the number of the identical human ESTs for both the 5'-end and 3'-end sequences is 1 or higher and not more than 5).
   HEMBA1000005, HEMBA1000012, HEMBA1000020, HEMBA1000030, HEMBA1000076, HEMBA1000141, HEMBA1000168, HEMBA1000185, HEMBA1000307, HEMBA1000327, HEMBA1000356, HEMBA100038 7, HEMBA1000456, HEMBA1000460, HEMBA1000490, HEMBA1000491, HEMBA1000501, HEMBA10005 20, HEMBA1000561, HEMBA1000588, HEMBA1000591, HEMBA1000592, HEMBA1000608, HEMBA1000 636, HEMBA1000682, HEMBA1000686, HEMBA1000719, HEMBA1000727, HEMBA1000817, HEMBA100 0851, HEMBA1000946, HEMBA1001008, HEMBA1001059, HEMBA1001088, HEMBA1001122, HEMBA10 01197, HEMBA1001213, HEMBA1001247, HEMBA1001303, HEMBA1001326, HEMBA1001351, HEMBA1 001361, HEMBA1001387, HEMBA1001405, HEMBA1001450, HEMBA1001526, HEMBA1001569, HEMBA 1001595, HEMBA1001620, HEMBA1001655, HEMBA1001672, HEMBA1001711, HEMBA1001714, HEMB A1001723, HEMBA1001809, HEMBA1001824, HEMBA1001847, HEMBA1001913, HEMBA1001921, HEM BA1001939, HEMBA1001950, HEMBA1001967,
   HEMBA1002150, HEMBA1002151, HEMBA1002215, HEMBA1002241, HEMBA1002267, HEMBA1002341, HEMBA1002458, HEMBA1002460, HEMBA1002462, HEMBA1002469, HEMBA1002475, HEMBA100254 2, HEMBA1002569, HEMBA1002583, HEMBA1002609, HEMBA1002624, HEMBA1002696, HEMBA10027 68, HEMBA1002770, HEMBA1002777, HEMBA1002779, HEMBA1002794, HEMBA1002810, HEMBA1002 816, HEMBA1002818, HEMBA1002876, HEMBA1002937, HEMBA1002939, HEMBA1002951, HEMBA100 2954, HEMBA1003033, HEMBA1003046, HEMBA1003096, HEMBA1003136, HEMBA1003148, HEMBA10 03179, HEMBA1003222, HEMBA1003281, HEMBA1003286, HEMBA1003369, HEMBA1003403, HEMBA1 003408, HEMBA1003417, HEMBA1003418, HEMBA1003447, HEMBA1003463, HEMBA1003528, HEMBA 1003555, HEMBA1003560, HEMBA1003569, HEMBA1003591, HEMBA1003615, HEMBA1003617, HEMB A1003646, HEMBA1003662, HEMBA1003679, HEMBA1003684, HEMBA1003690, HEMBA1003711, HEM BA1003729, HEMBA1003742, HEMBA1003783, HEMBA1003803, HEMBA1003805, HEMBA1003838, HE MBA1003864, HEMBA1003959, HEMBA1003978,
   HEMBA1004055, HEMBA1004086, HEMBA1004111, HEMBA1004131, HEMBA1004143, HEMBA1004168 , HEMBA1004202, HEMBA1004203, HEMBA1004227, HEMBA1004248, HEMBA1004264, HEMBA100427 4, HEMBA1004276, HEMBA1004330, HEMBA1004353, HEMBA1004408, HEMBA1004509, HEMBA10045 34, HEMBA1004604, HEMBA1004610, HEMBA1004637, HEMBA1004669, HEMBA1004725, HEMBA1004 734, HEMBA1004763, HEMBA1004776, HEMBA1004847, HEMBA1004864, HEMBA1004933, HEMBA100 4934, HEMBA1004954, HEMBA1005009, HEMBA1005029, HEMBA1005047, HEMBA1005101, HEMBA10 05185, HEMBA1005201, HEMBA1005202, HEMBA1005206, HEMBA1005252, HEMBA1005296, HEMBA1 005382, HEMBA1005394, HEMBA1005403, HEMBA1005423, HEMBA1005472, HEMBA1005474, HEMBA 1005475, HEMBA1005513, HEMBA1005528, HEMBA1005530, HEMBA1005558, HEMBA1005581, HEMB A1005582, HEMBA1005621, HEMBA1005666, HEMBA1005852, HEMBA1005990,
   HEMBA1006035, HEMBA1006081, HEMBA1006091, HEMBA1006108, HEMBA1006130, HEMBA1006155, HEMBA1006235, HEMBA1006248, HEMBA1006283, HEMBA1006284, HEMBA1006309, HEMBA100631 0, HEMBA1006347, HEMBA1006485, HEMBA1006507, HEMBA1006521, HEMBA1006559, HEMBA10066 12, HEMBA1006624, HEMBA1006631, HEMBA1006652, HEMBA1006659, HEMBA1006708, HEMBA1006 789, HEMBA1006885, HEMBA1006914, HEMBA1006926, HEMBA1006941, HEMBA1007018, HEMBA100 7080, HEMBA1007085, HEMBA1007087, HEMBA1007112, HEMBA1007121, HEMBA1007149, HEMBA10 07174, HEMBA1007194, HEMBA1007224, HEMBA1007243, HEMBA1007301, HEMBA1007319, HEMBB1000018, HEMBB1000036, HEMBB1000037, HEMBB1000048, HEMBB1000119, HEMBB1000136 , HEMBB1000217, HEMBB1000266, HEMBB1000592, HEMBB1000623, HEMBB1000630, HEMBB100063 1, HEMBB1000705, HEMBB1000763, HEMBB1000774, HEMBB1000807, HEMBB1000985, HEMBB10010 56, HEMBB1001068, HEMBB1001112, HEMBB1001117, HEMBB1001137, HEMBB1001151, HEMBB1001 182, HEMBB1001210, HEMBB1001242, HEMBB1001288, HEMBB1001289, HEMBB1001294, HEMBB100 1331, HEMBB1001384, HEMBB1001429, HEMBB1001603, HEMBB1001673, HEMBB1001684, HEMBB10 01695, HEMBB1001736, HEMBB1001869, HEMBB1001945, HEMBB1001947, HEMBB1001990, HEMBB1 002134, HEMBB1002329, HEMBB1002342, HEMBB1002409, HEMBB1002510, HEMBB1002550, HEMBB 1002600, HEMBB1002697,
   MAMMA1000055, MAMMA1000085, MAMMA1000163, MAMMA1000173, MAMMA1000278, MAMMA1000284 , MAMMA1000395, MAMMA1000422, MAMMA1000429, MAMMA1000468, MAMMA1000490, MAMMA100061 2, MAMMA1000625, MAMMA1000670, MAMMA1000734, MAMMA1000738, MAMMA1000824, MAMMA10008 59, MAMMA1001008, MAMMA1001059, MAMMA1001073, MAMMA1001075, MAMMA1001202, MAMMA1001 259, MAMMA1001271, MAMMA1001292, MAMMA1001476, MAMMA1001576, MAMMA1001649, MAMMA100 1654, MAMMA1001735, MAMMA1001744, MAMMA1001754, MAMMA1001771, MAMMA1001785, MAMMA10 01812, MAMMA1002011, MAMMA1002041, MAMMA1002143, MAMMA1002236, MAMMA1002351, MAMMA1 002385, MAMMA1002434, MAMMA1002461, MAMMA1002470, MAMMA1002485, MAMMA1002530, MAMMA 1002585, MAMMA1002598, MAMMA1002619, MAMMA1002684, MAMMA1002769, MAMMA1002775, MAMM A1002869, MAMMA1002890, MAMMA1002937, MAMMA1002947, MAMMA1003011, MAMMA1003035, MAM MA1003047, MAMMA1003057, MAMMA1003113, MAMMA1003146, MAMMA1003166,
   NT2RM1000001, NT2RM1000032, NT2RM1000035, NT2RM1000037, NT2RM1000039, NT2RM1000055 , NT2RM1000062, NT2RM1000080, NT2RM1000086, NT2RM1000092, NT2RM1000119, NT2RM100012 7, NT2RM1000132, NT2RM1000153, NT2RM1000187, NT2RM1000199, NT2RM1000244, NT2RM10002 56, NT2RM1000257, NT2RM1000272, NT2RM1000280, NT2RM1000300, NT2RM1000341, NT2RM1000 355, NT2RM1000365, NT2RM1000377, NT2RM1000388, NT2RM1000394, NT2RM1000430, NT2RM100 0499, NT2RM1000539, NT2RM1000553, NT2RM1000555, NT2RM1000648, NT2RM1000661, NT2RM10 00669, NT2RM1000672, NT2RM1000691, NT2RM1000699, NT2RM1000702, NT2RM1000741, NT2RM1 000746, NT2RM1000770, NT2RM1000780, NT2RM1000781, NT2RM1000800, NT2RM1000826, NT2RM 1000829, NT2RM1000833, NT2RM1000852, NT2RM1000857, NT2RM1000867, NT2RM1000874, NT2R M1000882, NT2RM1000883, NT2RM1000898, NT2RM1000905, NT2RM1000924, NT2RM1000927, NT2 RM1000962, NT2RM1001003, NT2RM1001043, NT2RM1001044, NT2RM1001074, NT2RM1001102, NT 2RM1001105, NT2RM1001112, NT2RM1001139,
   NT2RM2000006, NT2RM2000013, NT2RM2000030, NT2RM2000032, NT2RM2000092, NT2RM2000093 , NT2RM2000101, NT2RM2000191, NT2RM2000192, NT2RM2000239, NNNNNNNNNNNN, NT2RM200026 0, NT2RM2000287, NT2RM2000322, NT2RM2000359, NT2RM2000368, NT2RM2000371, NT2RM20003 95, NT2RM2000402, NT2RM2000407, NT2RM2000420, NT2RM2000422, NT2RM2000452, NT2RM2000 490, NT2RM2000502, NT2RM2000504, NT2RM2000522, NT2RM2000540, NT2RM2000569, NT2RM200 0577, NT2RM2000581, NT2RM2000612, NT2RM2000624, NT2RM2000639, NT2RM2000649, NT2RM20 00669, NT2RM2000691, NT2RM2000714, NT2RM2000735, NT2RM2000740, NT2RM2000821, NT2RM2 000837, NT2RM2000952, NT2RM2000984, NT2RM2001035, NT2RM2001065, NT2RM2001100, NT2RM 2001131, NT2RM2001152, NT2RM2001177, NT2RM2001194, NT2RM2001196, NT2RM2001201, NT2R M2001221, NT2RM2001238, NT2RM2001243, NT2RM2001256, NT2RM2001291, NT2RM2001306, NT2 RM2001319, NT2RM2001345, NT2RM2001360, NT2RM2001370, NT2RM2001393, NT2RM2001420, NT 2RM2001424,
   NT2RM2001504, NT2RM2001524, NT2RM2001582, NT2RM2001592, NT2RM2001605, NT2RM2001613, NT2RM2001632, NT2RM2001635, NT2RM2001637, NT2RM2001648, NT2RM2001664, NT2RM200166 8, NT2RM2001671, NT2RM2001675, NT2RM2001681, NT2RM2001688, NT2RM2001695, NT2RM20016 96, NT2RM2001700, NT2RM2001706, NT2RM2001716, NT2RM2001718, NT2RM2001723, NT2RM2001 730, NT2RM2001743, NT2RM2001760, NT2RM2001768, NT2RM2001782, NT2RM2001784, NT2RM200 1785, NT2RM2001800, NT2RM2001803, NT2RM2001813, NT2RM2001823, NT2RM2001839, NT2RM20 01840, NT2RM2001855, NT2RM2001867, NT2RM2001879, NT2RM2001886, NT2RM2001903, NT2RM2 001935, NT2RM2001936, NT2RM2001950, NT2RM2001997, NT2RM2001998, NT2RM2002004, NT2RM 2002030, NT2RM2002049, NT2RM2002055, NT2RM2002088, NT2RM2002091, NT2RM2002100, NT2R M2002109, NT2RM2002128, NT2RM2002142, NT2RM2002145, NT2RM2002178, NT2RM2002580, NT2RM4000027, NT2RM4000030, NT2RM4000104, NT2RM4000139, NT2RM4000156, NT2RM4000191 , NT2RM4000197, NT2RM4000210, NT2RM4000233, NT2RM4000324, NT2RM4000344, NT2RM400034 9, NT2RM4000356, NT2RM4000395, NT2RM4000421, NT2RM4000457, NT2RM4000471, NT2RM40005 11, NT2RM4000514, NT2RM4000515, NT2RM4000520, NT2RM4000531, NT2RM4000603, NT2RM4000 611, NT2RM4000674, NT2RM4000689, NT2RM4000698, NT2RM4000717, NT2RM4000741, NT2RM400 0751, NT2RM4000764, NT2RM4000778, NT2RM4000790, NT2RM4000813, NT2RM4000833, NT2RM40 00852, NT2RM4000950, NT2RM4001002, NT2RM4001084, NT2RM4001092, NT2RM4001151, NT2RM4 001155, NT2RM4001187, NT2RM4001204, NT2RM4001256, NT2RM4001258, NT2RM4001309, NT2RM 4001316, NT2RM4001340, NT2RM4001347, NT2RM4001371, NT2RM4001382, NT2RM4001410, NT2R M4001411, NT2RM4001444, NT2RM4001489,
   NT2RM4001565, NT2RM4001566, NT2RM4001569, NT2RM4001582, NT2RM4001597, NT2RM4001611, NT2RM4001682, NT2RM4001714, NT2RM4001731, NT2RM4001741, NT2RM4001758, NT2RM400181 9, NT2RM4001823, NT2RM4001828, NT2RM4001836, NT2RM4001841, NT2RM4001865, NT2RM40018 76, NT2RM4001880, NT2RM4001905, NT2RM4001922, NT2RM4001938, NT2RM4001940, NT2RM4001 965, NT2RM4002013, NT2RM4002018, NT2RM4002055, NT2RM4002062, NT2RM4002128, NT2RM400 2140, NT2RM4002146, NT2RM4002161, NT2RM4002189, NT2RM4002213, NT2RM4002226, NT2RM40 02251, NT2RM4002256, NT2RM4002294, NT2RM4002323, NT2RM4002398, NT2RM4002534, NT2RM4 002565, NT2RM4002571, NT2RM4002593, NT2RM4002594, NT2RM4002623,
   NT2RP1000018, NT2RP1000035, NT2RP1000040, NT2RP1000063, NT2RP1000086, NT2RP1000101 , NT2RP1000111, NT2RP1000112, NT2RP1000124, NT2RP1000130, NT2RP1000163, NT2RP100017 0, NT2RP1000174, NT2RP1000191, NT2RP1000202, NT2RP1000243, NT2RP1000259, NT2RP10002 72, NT2RP1000324, NT2RP1000326, NT2RP1000333, NT2RP1000348, NT2RP1000358, NT2RP1000 363, NT2RP1000413, NT2RP1000416, NT2RP1000418, NT2RP1000439, NT2RP1000443, NT2RP100 0470, NT2RP1000478, NT2RP1000481, NT2RP1000493, NT2RP1000513, NT2RP1000522, NT2RP10 00547, NT2RP1000574, NT2RP1000577, NT2RP1000609, NT2RP1000629, NT2RP1000630, NT2RP1 000688, NT2RP1000695, NT2RP1000701, NT2RP1000730, NT2RP1000738, NT2RP1000746, NT2RP 1000767, NT2RP1000782, NT2RP1000825, NT2RP1000833, NT2RP1000834, NT2RP1000851, NT2R P1000856, NT2RP1000860, NT2RP1000902, NT2RP1000915, NT2RP1000943, NT2RP1000944, NT2 RP1000947, NT2RP1000954, NT2RP1000958, NT2RP1000959, NT2RP1000966, NT2RP1000980, NT 2RP1000988, NT2RP1001011, NT2RP1001013, NT2RP1001014, NT2RP1001033, NT2RP1001073, N T2RP1001080, NT2RP1001113, NT2RP1001173, NT2RP1001177, NT2RP1001199, NT2RP1001248,
   NT2RP1001253, NT2RP1001286, NT2RP1001294, NT2RP1001302, NT2RP1001310, NT2RP1001311 , NT2RP1001313, NT2RP1001361, NT2RP1001385, NT2RP1001395, NT2RP1001410, NT2RP100142 4, NT2RP1001432, NT2RP1001449, NT2RP1001457, NT2RP1001466, NT2RP1001475, NT2RP10014 82, NT2RP1001543, NT2RP1001546, NT2RP1001569, NT2RP1001616, NT2RP1001665,
   NT2RP2000001, NT2RP2000007, NT2RP2000032, NT2RP2000040, NT2RP2000045, NT2RP2000056, NT2RP2000088, NT2RP2000091, NT2RP2000114, NT2RP2000120, NT2RP2000147, NT2RP200015 3, NT2RP2000157, NT2RP2000161, NT2RP2000205, NT2RP2000239, NT2RP2000248, NT2RP20002 88, NT2RP2000298, NT2RP2000328, NT2RP2000329, NT2RP2000346, NT2RP2000369, NT2RP2000 414, NT2RP2000438, NT2RP2000448, NT2RP2000510, NT2RP2000617, NT2RP2000634, NT2RP200 0668, NT2RP2000710, NT2RP2000809, NT2RP2000892, NT2RP2000931, NT2RP2000932, NT2RP20 00943, NT2RP2000965, NT2RP2000985, NT2RP2001044, NT2RP2001056, NT2RP2001065, NT2RP2 001070, NT2RP2001168, NT2RP2001245, NT2RP2001268, NT2RP2001327, NT2RP2001328, NT2RP 2001366, NT2RP2001378, NT2RP2001381, NT2RP2001397, NT2RP2001436, NT2RP2001520, NT2R P2001628, NT2RP2001634, NT2RP2001660, NT2RP2001675, NT2RP2001839, NT2RP2001876, NT2 RP2001883, NT2RP2001898, NT2RP2001947, NT2RP2001969,
   NT2RP2002033, NT2RP2002058, NT2RP2002076, NT2RP2002078, NT2RP2002099, NT2RP2002185 , NT2RP2002193, NT2RP2002208, NT2RP2002235, NT2RP2002270, NT2RP2002292, NT2RP200238 5, NT2RP2002408, NT2RP2002426, NT2RP2002442, NT2RP2002479, NT2RP2002498, NT2RP20025 20, NT2RP2002549, NT2RP2002595, NT2RP2002618, NT2RP2002701, NT2RP2002710, NT2RP2002 880, NT2RP2002928, NT2RP2002929, NT2RP2002979, NT2RP2002980, NT2RP2002993, NT2RP200 3108, NT2RP2003137, NT2RP2003157, NT2RP2003158, NT2RP2003177, NT2RP2003194, NT2RP20 03228, NT2RP2003243, NT2RP2003265, NT2RP2003272, NT2RP2003277, NT2RP2003280, NT2RP2 003295, NT2RP2003297, NT2RP2003329, NT2RP2003391, NT2RP2003393, NT2RP2003466, NT2RP 2003480, NT2RP2003506, NT2RP2003511, NT2RP2003567, NT2RP2003596, NT2RP2003604, NT2R P2003629, NT2RP2003643, NT2RP2003702, NT2RP2003713, NT2RP2003714, NT2RP2003737, NT2 RP2003777, NT2RP2003793, NT2RP2003825, NT2RP2003840, NT2RP2003976, NT2RP2003981, NT 2RP2003984,
   NT2RP2004013, NT2RP2004042, NT2RP2004081, NT2RP2004098, NT2RP2004124, NT2RP2004152, NT2RP2004165, NT2RP2004187, NT2RP2004194, NT2RP2004196, NT2RP2004226, NT2RP200424 2, NT2RP2004245, NT2RP2004364, NT2RP2004365, NT2RP2004389, NT2RP2004399, NT2RP20044 63, NT2RP2004476, NT2RP2004594, NT2RP2004602, NT2RP2004614, NT2RP2004655, NT2RP2004 664, NT2RP2004689, NT2RP2004710, NT2RP2004768, NT2RP2004791, NT2RP2004799, NT2RP200 4802, NT2RP2004816, NT2RP2004933, NT2RP2004936, NT2RP2004985, NT2RP2005001, NT2RP20 05012, NT2RP2005037, NT2RP2005126, NT2RP2005139, NT2RP2005140, NT2RP2005159, NT2RP2 005162, NT2RP2005168, NT2RP2005204, NT2RP2005227, NT2RP2005270, NT2RP2005276, NT2RP 2005287, NT2RP2005288, NT2RP2005315, NT2RP2005358, NT2RP2005436, NT2RP2005441, NT2R P2005453, NT2RP2005457, NT2RP2005464, NT2RP2005465, NT2RP2005490, NT2RP2005495, NT2 RP2005498, NT2RP2005509, NT2RP2005525, NT2RP2005539, NT2RP2005540, NT2RP2005549, NT 2RP2005620, NT2RP2005640, NT2RP2005669, NT2RP2005675, NT2RP2005712, NT2RP2005732, N T2RP2005748, NT2RP2005773, NT2RP2005784, NT2RP2005835, NT2RP2005859, NT2RP2005886,
   NT2RP2005890, NT2RP2005901, NT2RP2005933, NT2RP2006043, NT2RP2006071, NT2RP2006100 NT2RP2006106, NT2RP2006141, NT2RP2006196, NT2RP2006219, NT2RP2006237, NT2RP200623, 8, NT2RP2006312, NT2RP2006365, NT2RP2006441, NT2RP2006456, NT2RP2006472,
   NT2RP3000031, NT2RP3000047, NT2RP3000085, NT2RP3000142, NT2RP3000149, NT2RP3000197 , NT2RP3000252, NT2RP3000267, NT2RP3000312, NT2RP3000320, NT2RP3000333, NT2RP300035 0, NT2RP3000359, NT2RP3000361, NT2RP3000366, NT2RP3000393, NT2RP3000403, NT2RP30004 56, NT2RP3000487, NT2RP3000526, NT2RP3000605, NT2RP3000624, NT2RP3000739, NT2RP3000 759, NT2RP3000826, NT2RP3000836, NT2RP3000847, NT2RP3000850, NT2RP3000859, NT2RP300 0901, NT2RP3000917, NT2RP3000919, NT2RP3000968, NT2RP3000980, NT2RP3000994, NT2RP30 01055, NT2RP3001057, NT2RP3001081, NT2RP3001084, NT2RP3001111, NT2RP3001119, NT2RP3 001140, NT2RP3001147, NT2RP3001214, NT2RP3001216, NT2RP3001221, NT2RP3001253, NT2RP 3001260, NT2RP3001268, NT2RP3001274, NT2RP3001297, NT2RP3001325, NT2RP3001355, NT2R P3001356, NT2RP3001384, NT2RP3001396, NT2RP3001399, NT2RP3001426, NT2RP3001427, NT2 RP3001449, NT2RP3001472, NT2RP3001490, NT2RP3001497, NT2RP3001529, NT2RP3001538, NT 2RP3001587, NT2RP3001621, NT2RP3001642, NT2RP3001646, NT2RP3001671, NT2RP3001672, N T2RP3001676, NT2RP3001679, NT2RP3001690, NT2RP3001698, NT2RP3001712, NT2RP3001716, NT2RP3001724, NT2RP3001727, NT2RP3001730, NT2RP3001777, NT2RP3001792, NT2RP3001854 , NT2RP3001857, NT2RP3001896, NT2RP3001938, NT2RP3001943,
   NT2RP3002014, NT2RP3002033, NT2RP3002045, NT2RP3002054, NT2RP3002062, NT2RP3002097, NT2RP3002102, NT2RP3002142, NT2RP3002146, NT2RP3002147, NT2RP3002165, NT2RP300218 1, NT2RP3002244, NT2RP3002248, NT2RP3002255, NT2RP3002273, NT2RP3002276, NT2RP30023 30, NT2RP3002343, NT2RP3002377, NT2RP3002399, NT2RP3002402, NT2RP3002455, NT2RP3002 501, NT2RP3002512, NT2RP3002529, NT2RP3002545, NT2RP3002549, NT2RP3002566, NT2RP300 2602, NT2RP3002628, NT2RP3002631, NT2RP3002682, NT2RP3002688, NT2RP3002763, NT2RP30 02861, NT2RP3002909, NT2RP3002948, NT2RP3002969, NT2RP3002972, NT2RP3003008, NT2RP3 003061, NT2RP3003068, NT2RP3003071, NT2RP3003101, NT2RP3003145, NT2RP3003150, NT2RP 3003185, NT2RP3003197, NT2RP3003203, NT2RP3003210, NT2RP3003212, NT2RP3003230, NT2R P3003242, NT2RP3003278, NT2RP3003290, NT2RP3003301, NT2RP3003311, NT2RP3003313, NT2 RP3003327, NT2RP3003330, NT2RP3003427, NT2RP3003490, NT2RP3003491, NT2RP3003543, NT 2RP3003552, NT2RP3003555, NT2RP3003564, NT2RP3003589, NT2RP3003659, NT2RP3003672, N T2RP3003680, NT2RP3003686, NT2RP3003716, NT2RP3003805, NT2RP3003809, NT2RP3003825, NT2RP3003833, NT2RP3003914, NT2RP3003918, NT2RP3003932, NT2RP3003992, NT2RP3004013 , NT2RP3004041, NT2RP3004051, NT2RP3004125, NT2RP3004189, NT2RP3004207, NT2RP300420 9, NT2RP3004258, NT2RP3004262, NT2RP3004282, NT2RP3004332, NT2RP3004378, NT2RP30044 24, NT2RP3004466, NT2RP3004472, NT2RP3004480, NT2RP3004498, NT2RP3004539, NT2RP3004 566, NT2RP3004578,
   NT2RP4000008, NT2RP4000109, NT2RP4000129, NT2RP4000147, NT2RP4000150, NT2RP4000151, NT2RP4000185, NT2RP4000212, NT2RP4000243, NT2RP4000246, NT2RP4000259, NT2RP400026 3, NT2RP4000367, NT2RP4000370, NT2RP4000376, NT2RP4000381, NT2RP4000415, NT2RP40004 17, NT2RP4000449, NT2RP4000455, NT2RP4000498, NT2RP4000500, NT2RP4000524, NT2RP4000 528, NT2RP4000556, NT2RP4000588, NT2RP4000614, NT2RP4000648, NT2RP4000657, NT2RP400 0704, NT2RP4000713, NT2RP4000724, NT2RP4000787, NT2RP4000833, NT2RP4000839, NT2RP40 00855, NT2RP4000878, NT2RP4000879, NT2RP4000907, NT2RP4000918, NT2RP4000928, NT2RP4 000979, NT2RP4000984, NT2RP4000989, NT2RP4001006, NT2RP4001010, NT2RP4001041, NT2RP 4001079, NT2RP4001095, NT2RP4001100, NT2RP4001117, NT2RP4001122, NT2RP4001126, NT2R P4001143, NT2RP4001148, NT2RP4001149, NT2RP4001159, NT2RP4001174, NT2RP4001206, NT2 RP4001207, NT2RP4001210, NT2RP4001213, NT2RP4001219, NT2RP4001228, NT2RP4001235, NT 2RP4001256, NT2RP4001260, NT2RP4001274, NT2RP4001276, NT2RP4001315, NT2RP4001336, N T2RP4001339, NT2RP4001343, NT2RP4001375, NT2RP4001389, NT2RP4001414, NT2RP4001442, NT2RP4001447, NT2RP4001474, NT2RP4001498,
   NT2RP4001502, NT2RP4001507, NT2RP4001524, NT2RP4001529, NT2RP4001547, NT2RP4001555 , NT2RP4001567, NT2RP4001568, NT2RP4001571, NT2RP4001574, NT2RP4001575, NT2RP400159 2, NT2RP4001610, NT2RP4001638, NT2RP4001644, NT2RP4001677, NT2RP4001679, NT2RP40016 96, NT2RP4001730, NT2RP4001739, NT2RP4001753, NT2RP4001760, NT2RP4001803, NT2RP4001 822, NT2RP4001823, NT2RP4001828, NT2RP4001838, NT2RP4001841, NT2RP4001849, NT2RP400 1861, NT2RP4001893, NT2RP4001901, NT2RP4001927, NT2RP4001946, NT2RP4001950, NT2RP40 01953, NT2RP4001975, NT2RP4002018, NT2RP4002071, NT2RP4002078, NT2RP4002083, NT2RP4 002791, NT2RP4002888, NT2RP5003461, NT2RP5003477, NT2RP5003492, NT2RP5003522, NT2RP 5003534,
   OVARC1000001, OVARC1000004, OVARC1000006, OVARC1000013, OVARC1000091, OVARC1000106 , OVARC1000113, OVARC1000139, OVARC1000168, OVARC1000209, OVARC1000212, OVARC100028 8, OVARC1000304, OVARC1000309, OVARC1000321, OVARC1000335, OVARC1000384, OVARC10004 43, OVARC1000465, OVARC1000466, OVARC1000479, OVARC1000564, OVARC1000576, OVARC1000 682, OVARC1000722, OVARC1000730, OVARC1000746, OVARC1000771, OVARC1000781, OVARC100 0834, OVARC1000862, OVARC1000883, OVARC1000885, OVARC1000886, OVARC1000924, OVARC10 00964, OVARC1000984, OVARC1001034, OVARC1001038, OVARC1001055, OVARC1001065, OVARC1 001068, OVARC1001092, OVARC1001107, OVARC1001154, OVARC1001161, OVARC1001171, OVARC 1001176, OVARC1001232, OVARC1001244, OVARC1001270, OVARC1001296, OVARC1001341, OVAR C1001360, OVARC1001381, OVARC1001419, OVARC1001476, OVARC1001496, OVARC1001555, OVA RC1001577, OVARC1001702, OVARC1001711, OVARC1001713, OVARC1001766, OVARC1001768, OV ARC1001791, OVARC1001809, OVARC1001873, OVARC1001880, OVARC1001916, OVARC1001943, O VARC1001987, OVARC1002050, OVARC1002112, OVARC1002127, OVARC1002156, OVARC1002182, PLACE1000007, PLACE1000050, PLACE1000066, PLACE1000133, PLACE1000142, PLACE1000184 , PLACE1000185, PLACE1000213, PLACE1000246, PLACE1000308, PLACE1000347, PLACE100037 4, PLACE1000380, PLACE1000383, PLACE1000406, PLACE1000453, PLACE1000492, PLACE10005 62, PLACE1000610, PLACE1000611, PLACE1000653, PLACE1000656, PLACE1000706, PLACE1000 712, PLACE1000748, PLACE1000769, PLACE1000785, PLACE1000793, PLACE1000798, PLACE100 0856, PLACE1000863, PLACE1000977, PLACE1000987, PLACE1001054, PLACE1001092, PLACE10 01168, PLACE1001185, PLACE1001238, PLACE1001294, PLACE1001304, PLACE1001351, PLACE1 001383, PLACE1001399, PLACE1001412, PLACE1001608, PLACE1001692, PLACE1001761, PLACE 1001781, PLACE1001817, PLACE1001844, PLACE1001869, PLACE1001920,
   PLACE1002046, PLACE1002072, PLACE1002090, PLACE1002140, PLACE1002171, PLACE1002342 , PLACE1002395, PLACE1002499, PLACE1002571, PLACE1002722, PLACE1002794, PLACE100281 1, PLACE1002815, PLACE1002816, PLACE1002834, PLACE1002908, PLACE1002996, PLACE10030 27, PLACE1003092, PLACE1003100, PLACE1003136, PLACE1003176, PLACE1003200, PLACE1003 296, PLACE1003302, PLACE1003353, PLACE1003394, PLACE1003454, PLACE1003537, PLACE100 3592, PLACE1003596, PLACE1003602, PLACE1003625, PLACE1003704, PLACE1003886, PLACE10 03892, PLACE1003903, PLACE1003915, PLACE1003923, PLACE1003968,
   PLACE1004103, PLACE1004183, PLACE1004258, PLACE1004316, PLACE1004358, PLACE1004376 , PLACE1004405, PLACE1004437, PLACE1004550, PLACE1004629, PLACE1004674, PLACE100473 6, PLACE1004777, PLACE1004814, PLACE1004902, PLACE1004930, PLACE1004969, PLACE10049 82, PLACE1005027, PLACE1005055, PLACE1005066, PLACE1005101, PLACE1005102, PLACE1005 181, PLACE1005187, PLACE1005261, PLACE1005287, PLACE1005305, PLACE1005308, PLACE100 5327, PLACE1005335, PLACE1005373, PLACE1005550, PLACE1005557, PLACE1005595, PLACE10 05603, PLACE1005623, PLACE1005630, PLACE1005698, PLACE1005727, PLACE1005739, PLACE1 005803, PLACE1005813, PLACE1005851, PLACE1005955,
   PLACE1006040, PLACE1006119, PLACE1006139, PLACE1006159, PLACE1006167, PLACE1006196, PLACE1006236, PLACE1006246, PLACE1006325, PLACE1006335, PLACE1006469, PLACE100648 8, PLACE1006492, PLACE1006615, PLACE1006678, PLACE1006819, PLACE1006883, PLACE10069 17, PLACE1006989, PLACE1007021, PLACE1007053, PLACE1007105, PLACE1007178, PLACE1007 238, PLACE1007239, PLACE1007243, PLACE1007282, PLACE1007367, PLACE1007386, PLACE100 7402, PLACE1007409, PLACE1007416, PLACE1007454, PLACE1007507, PLACE1007511, PLACE10 07547, PLACE1007598, PLACE1007621, PLACE1007632, PLACE1007645, PLACE1007649, PLACE1 007688, PLACE1007690, PLACE1007697, PLACE1007705, PLACE1007706, PLACE1007725, PLACE 1007730, PLACE1007746, PLACE1007846, PLACE1007858, PLACE1007908, PLACE1007954, PLAC E1007955, PLACE1007958, PLACE1007969, PLACE1007990,
   PLACE1008044, PLACE1008122, PLACE1008132, PLACE1008198, PLACE1008209, PLACE1008356 , PLACE1008368, PLACE1008398, PLACE1008429, PLACE1008524, PLACE1008531, PLACE100856 8, PLACE1008603, PLACE1008629, PLACE1008650, PLACE1009027, PLACE1009045, PLACE10090 60, PLACE1009091, PLACE1009186, PLACE1009298, PLACE1009319, PLACE1009338, PLACE1009 444, PLACE1009468, PLACE1009476, PLACE1009571, PLACE1009581, PLACE1009596, PLACE100 9622, PLACE1009670, PLACE1009721, PLACE1009731, PLACE1009763, PLACE1009845, PLACE10 09921, PLACE1009995,
   PLACE1010023, PLACE1010031, PLACE1010053, PLACE1010074, PLACE1010076, PLACE1010096 , PLACE1010102, PLACE1010105, PLACE1010106, PLACE1010134, PLACE1010152, PLACE101019 4, PLACE1010274, PLACE1010362, PLACE1010364, PLACE1010383, PLACE1010491, PLACE10104 92, PLACE1010522, PLACE1010529, PLACE1010599, PLACE1010622, PLACE1010630, PLACE1010 720, PLACE1010761, PLACE1010771, PLACE1010786, PLACE1010800, PLACE1010833, PLACE101 0856, PLACE1010857, PLACE1010870, PLACE1010877, PLACE1010925, PLACE1010944, PLACE10 10960, PLACE1011041, PLACE1011090, PLACE1011160, PLACE1011214, PLACE1011219, PLACE1 011221, PLACE1011263, PLACE1011291, PLACE1011310, PLACE1011332, PLACE1011371, PLACE 1011433, PLACE1011477, PLACE1011586, PLACE1011646, PLACE1011675, PLACE1011858, PLAC E1011923,
   PLACE2000007, PLACE2000021, PLACE2000030, PLACE2000164, PLACE2000172, PLACE2000302 , PLACE2000341, PLACE2000399, PLACE2000404, PLACE2000427, PLACE3000009, PLACE300012 1, PLACE3000148, PLACE3000156, PLACE3000160, PLACE3000197, PLACE3000226, PLACE30002 42, PLACE3000339, PLACE3000353, PLACE3000413, PLACE3000477, PLACE4000009, PLACE4000 034, PLACE4000089, PLACE4000106, PLACE4000259, PLACE4000269, PLACE4000326, PLACE400 0369, PLACE4000431, PLACE4000445, PLACE4000581, PLACE4000593, PLACE4000650, PLACE40 00670,
   SKNMC1000046, SKNMC1000050, SKNMC1000091, THYRO1000017, THYRO1000040, THYRO1000121 , THYRO1000173, THYRO1000197, THYRO1000199, THYRO1000206, THYRO1000242, THYRO100027 0, THYRO1000288, THYRO1000320, THYRO1000327, THYRO1000358, THYRO1000394, THYRO10004 01, THYRO1000488, THYRO1000502, THYRO1000569, THYRO1000570, THYRO1000585, THYRO1000 605, THYRO1000715, THYRO1000756, THYRO1000777, THYRO1000829, THYRO1000855, THYRO100 0926, THYRO1000983, THYRO1000984, THYRO1001120, THYRO1001134, THYRO1001173, THYRO10 01204, THYRO1001271, THYRO1001287, THYRO1001313, THYRO1001347, THYRO1001363, THYRO1 001405, THYRO1001406, THYRO1001537, THYRO1001584, THYRO1001671, THYRO1001703, THYRO 1001721, THYRO1001793,
   VESEN1000122, Y79AA1000013, Y79AA1000181, Y79AA1000202, Y79AA1000214, Y79AA1000230 , Y79AA1000231, Y79AA1000258, Y79AA1000313, Y79AA1000328, Y79AA1000342, Y79AA100036 8, Y79AA1000420, Y79AA1000469, Y79AA1000480, Y79AA1000539, Y79AA1000540, Y79AA10005 60, Y79AA1000574, Y79AA1000589, Y79AA1000705, Y79AA1000734, Y79AA1000748, Y79AA1000 752, Y79AA1000774, Y79AA1000782, Y79AA1000784, Y79AA1000794, Y79AA1000800, Y79AA100 0833, Y79AA1000966, Y79AA1000968, Y79AA1001023, Y79AA1001041, Y79AA1001048, Y79AA10 01077, Y79AA1001078, Y79AA1001216, Y79AA1001228, Y79AA1001236, Y79AA1001312, Y79AA1 001323, Y79AA1001394, Y79AA1001402, Y79AA1001493, Y79AA1001511, Y79AA1001548, Y79AA 1001585, Y79AA1001603, Y79AA1001647, Y79AA1001665, Y79AA1001679, Y79AA1001696, Y79A A1001705, Y79AA1001711, Y79AA1001781, Y79AA1001805, Y79AA1001827, Y79AA1001963, Y79 AA1002027, Y79AA1002089, Y79AA1002093, Y79AA1002125, Y79AA1002208, Y79AA1002209, Y7 9AA1002210, Y79AA1002234, Y79AA1002258, Y79AA1002307, Y79AA1002311, Y79AA1002351, Y 79AA1002416, Y79AA1002487,
Group (4): 453 clones
   Among the 1857 clones having the maximal ATGpr1 score not more than 0.3 (including the 9 clones whose 5'-end sequence does not contain the ATG codon), the following 453 clones were judged to be full-length since their ATGpr2 score was 0.3 or higher (Table 11). The clones were novel clones, in which at least either of the 5'-end or 3'-end sequence is not identical to any of human ESTs.
   HEMBA1000180, HEMBA1000280, HEMBA1000282, HEMBA1000333, HEMBA1000351, HEMBA1000357 , HEMBA1000376, HEMBA1000469, HEMBA1000504, HEMBA1000519, HEMBA1000545, HEMBA100055 7, HEMBA1000575, HEMBA1000726, HEMBA1000822, HEMBA1000934, HEMBA1000943, HEMBA10009 60, HEMBA1000972, HEMBA1001020, HEMBA1001051, HEMBA1001060, HEMBA1001071, HEMBA1001 208, HEMBA1001319, HEMBA1001383, HEMBA1001411, HEMBA1001433, HEMBA1001435, HEMBA100 1478, HEMBA1001522, HEMBA1001636, HEMBA1001651, HEMBA1001658, HEMBA1001709, HEMBA10 01745, HEMBA1001791, HEMBA1001835, HEMBA1001844, HEMBA1001888, HEMBA1001940, HEMBA1 001962, HEMBA1002022, HEMBA1002185, HEMBA1002348, HEMBA1002621, HEMBA1002645, HEMBA 1002661, HEMBA1002728, HEMBA1002921, HEMBA1002924, HEMBA1002934, HEMBA1002968, HEMBA1003037, HEMBA1003071, HEMBA1003166, HEMBA1003202, HEMBA1003204, HEMBA1003220, HEMBA1003229, HEMBA1003276, HEMBA1003296, HEMBA1003373, HEMBA1003531, HEMBA100364 0, HEMBA1003856, HEMBA1003926, HEMBA1003942, HEMBA1003987, HEMBA1004024, HEMBA10042 67, HEMBA1004323, HEMBA1004433, HEMBA1004577, HEMBA1004730, HEMBA1004778, HEMBA1004 803, HEMBA1004807, HEMBA1004880, HEMBA1004900, HEMBA1004983, HEMBA1005123, HEMBA100 5241, HEMBA1005311, HEMBA1005318, HEMBA1005353, HEMBA1005374, HEMBA1005447, HEMBA10 05588, HEMBA1005593, HEMBA1005606, HEMBA1005679, HEMBA1005894, HEMBA1005911, HEMBA1 006036, HEMBA1006124, HEMBA1006253, HEMBA1006259, HEMBA1006364, HEMBA1006380, HEMBA 1006426, HEMBA1006562, HEMBA1006597, HEMBA1006639, HEMBA1006653, HEMBA1006696, HEMB A1006744, HEMBA1006824, HEMBA1006949, HEMBA1007078, HEMBA1007129, HEMBA1007147, HEM BA1007206, HEMBA1007279, HEMBA1007327,
   HEMBB1000005, HEMBB1000055, HEMBB1000144, HEMBB1000258, HEMBB1000318, HEMBB1000335, HEMBB1000354, HEMBB1000374, HEMBB1000402, HEMBB1000404, HEMBB1000480, HEMBB100049 3, HEMBB1000554, HEMBB1000573, HEMBB1000649, HEMBB1000652, HEMBB1000709, HEMBB10007 49, HEMBB1000790, HEMBB1000827, HEMBB1000831, HEMBB1000893, HEMBB1001004, HEMBB1001 008, HEMBB1001047, HEMBB1001315, HEMBB1001317, HEMBB1001326, HEMBB1001367, HEMBB100 1424, HEMBB1001436, HEMBB1001458, HEMBB1001535, HEMBB1001565, HEMBB1001747, HEMBB10 01749, HEMBB1001797, HEMBB1001836, HEMBB1001863, HEMBB1001875, HEMBB1001911, HEMBB1 001922, HEMBB1001925, HEMBB1001944, HEMBB1001983, HEMBB1001996, HEMBB1001997, HEMBB 1002092, HEMBB1002247, HEMBB1002266, HEMBB1002387, HEMBB1002425, HEMBB1002458, HEMB B1002522, HEMBB1002534, HEMBB1002582, HEMBB1002596, HEMBB1002617, HEMBB1002702, MAMMA1000043, MAMMA1000092, MAMMA1000129, MAMMA1000198, MAMMA1000221, MAMMA1000307 , MAMMA1000331, MAMMA1000360, MAMMA1000402, MAMMA1000414, MAMMA1000500, MAMMA100052 2, MAMMA1000576, MAMMA1000594, MAMMA1000597, MAMMA1000720, MAMMA1000775, MAMMA10007 78, MAMMA1000798, MAMMA1000862, MAMMA1000876, MAMMA1000931, MAMMA1000940, MAMMA1000 941, MAMMA1000975, MAMMA1001038, MAMMA1001186, MAMMA1001220, MAMMA1001256, MAMMA100 1274, MAMMA1001341, MAMMA1001397, MAMMA1001420, MAMMA1001547, MAMMA1001670, MAMMA10 01679, MAMMA1001711, MAMMA1001745, MAMMA1001760, MAMMA1001769, MAMMA1001815, MAMMA1 001907, MAMMA1002056, MAMMA1002078, MAMMA1002093, MAMMA1002125, MAMMA1002132, MAMMA 1002145, MAMMA1002250, MAMMA1002311, MAMMA1002411, MAMMA1002498, MAMMA1002571, MAMM A1002701, MAMMA1002727, MAMMA1002728, MAMMA1002746, MAMMA1002764, MAMMA1002765, MAM MA1002820, MAMMA1002830, MAMMA1002909, MAMMA1002941, MAMMA1002973, MAMMA1003004, MA MMA1003007, MAMMA1003039, MAMMA1003089,
   NT2RM4000086, NT2RM4000265, NT2RM4000414, NT2RM4000779, NT2RM4000855, NT2RM4001160 , NT2RM4001313, NT2RM4001437, NT2RM4001754, NT2RM4001953, NT2RM4001984, NT2RP200007 7, NT2RP2000183, NT2RP2000420, NT2RP2000678, NT2RP2000715, NT2RP2000842, NT2RP20009 70, NT2RP2001149, NT2RP2001226, NT2RP2001295, NT2RP2001347, NT2RP2001569, NT2RP2001 663, NT2RP2001936, NT2RP2002041, NT2RP2002172, NT2RP2002219, NT2RP2002316, NT2RP200 2546, NT2RP2002591, NT2RP2002643, NT2RP2002741, NT2RP2002750, NT2RP2002778, NT2RP20 02857, NT2RP2003000, NT2RP2003073, NT2RP2003237, NT2RP2003394, NT2RP2003517, NT2RP2 003668, NT2RP2003988, NT2RP2004232, NT2RP2004523, NT2RP2004736, NT2RP2004767, NT2RP 2004775, NT2RP2004961, NT2RP2004962, NT2RP2004982, NT2RP2005407, NT2RP2005726, NT2R P2006258, NT2RP2006261, NT2RP2006454, NT2RP3000055, NT2RP3000233, NT2RP3000341, NT2 RP3000418, NT2RP3000451, NT2RP3000561, NT2RP3000582, NT2RP3001281, NT2RP3001339, NT 2RP3001340, NT2RP3001383, NT2RP3001432, NT2RP3001580, NT2RP3001589, NT2RP3002004, N T2RP3002173, NT2RP3003133, NT2RP3003346, NT2RP3003403, NT2RP3003576, NT2RP3003625, NT2RP3003665, NT2RP3003800, NT2RP3003828, NT2RP3004070, NT2RP3004470, NT2RP4000023 , NT2RP4000035, NT2RP4000102, NT2RP4000167, NT2RP4000214, NT2RP4000218, NT2RP400042 4, NT2RP4000915, NT2RP4002075,
   OVARC1000085, OVARC1000092, OVARC1000145, OVARC1000414, OVARC1000496, OVARC1000526 , OVARC1000948, OVARC1001011, OVARC1001600, OVARC1001805, OVARC1001813, OVARC100184 6,
   PLACE1000540, PLACE1000599, PLACE1001088, PLACE1001377, PLACE1001440, PLACE1001517 , PLACE1001672, PLACE1001756, PLACE1002157, PLACE1002205, PLACE1002259, PLACE100239 9, PLACE1002477, PLACE1002583, PLACE1002968, PLACE1003238, PLACE1003566, PLACE10035 93, PLACE1003618, PLACE1004274, PLACE1004716, PLACE1004773, PLACE1004815, PLACE1004 979, PLACE1005052, PLACE1005086, PLACE1005128, PLACE1005176, PLACE1005467, PLACE100 5639, PLACE1005850, PLACE1006003, PLACE1006017, PLACE1006288, PLACE1006371, PLACE10 06629, PLACE1007478, PLACE1008330, PLACE1008584, PLACE1008851, PLACE1008941, PLACE1 009039, PLACE1009493, PLACE1009539, PLACE1009637, PLACE1009947, PLACE1010231, PLACE 1010562, PLACE1010579, PLACE1010739, PLACE1010802, PLACE1010896, PLACE1011032, PLAC E1011185, PLACE1011452, PLACE1011465, PLACE1011520, PLACE1011567, PLACE1011719, PLA CE2000011, PLACE2000017, PLACE2000061, PLACE2000187, PLACE2000216, PLACE2000335, PL ACE2000347, PLACE2000366, PLACE2000394, PLACE2000398, PLACE2000425, PLACE2000450, P LACE2000477, PLACE3000119, PLACE3000207, PLACE3000230, PLACE3000271, PLACE3000373, PLACE3000399, PLACE3000401, PLACE3000406, PLACE4000247, PLACE4000320, PLACE4000367 , PLACE4000401,
   THYRO1000111, THYRO1000187, THYRO1000484, THYRO1000596, THYRO1000625, THYRO1000815 , THYRO1000865, THYRO1001003, THYRO1001031, THYRO1001133, THYRO1001401, THYRO100142 6, THYRO1001434, THYRO1001559, THYRO1001570, THYRO1001706, THYRO1001746, THYRO10017 72, THYRO1001907, Y79AA1000033, Y79AA1000346, Y79AA1000805, Y79AA1001692, Y79AA1002 220,
Group (5): 24 clones
   The following 24 clones having low score in the ATGpr1 were judged to be full-length since their ATGpr2 score was 0.3 or higher (Table 11). The clones were novel clones, in which the number of the identical human ESTs for both the 5'-end and 3'-end sequences is 1 or higher and not more than 5.
   HEMBA1000622, HEMBA1000749, HEMBA1000876, HEMBA1001226, HEMBA1001391, HEMBA1002742 , HEMBA1003908, HEMBA1004000, HEMBB1000336, MAMMA1000356, MAMMA1000883, MAMMA100159 0, NT2RP2000289, NT2RP2001467, PLACE1002853, PLACE1003420, PLACE1004836, PLACE10049 13, PLACE1006795, PLACE1008181, PLACE1008715, PLACE4000344, THYRO1000129, THYRO1001 321,
Group (6): 65 clones
   The following 65 clones having low scores in both the ATGpr1 and ATGpr2 (Table 11) were judged to be full-length, since both scores were still the maximum in a cluster compared with those of the other clones(at least 2 clones or more) in the same cluster. The clones were novel clones, in which at least either of the 5'-end or 3'-end sequence is not identical to any of human ESTs.
   HEMBA1000604, HEMBA1000673, HEMBA1001024, HEMBA1001026, HEMBA1001734, HEMBA1001784 , HEMBA1001808, HEMBA1003902, HEMBA1004164, HEMBA1004909, HEMBA1005232, HEMBA100557 7, HEMBA1006461, HEMBA1006695, HEMBB1001119, HEMBB1001337, HEMBB1001536, HEMBB10018 68, HEMBB1002045, HEMBB1002579, MAMMA1000444, MAMMA1000761, MAMMA1000943, MAMMA1001 820, MAMMA1002360,
   NT2RM4000366, NT2RM4001856, NT2RM4002390, NT2RP2000108, NT2RP2000257, NT2RP2001506, NT2RP2002047, NT2RP2002066, NT2RP2002475, NT2RP2004400, NT2RP2004587, NT2RP200528 9, NT2RP2005694, NT2RP3001898, NT2RP3003264, NT2RP3003433, NT2RP3003842, OVARC10012 40, PLACE1001323, PLACE1002227, PLACE1002500, PLACE1002604, PLACE1002772, PLACE1003 478, PLACE1004681, PLACE1005108, PLACE1005932, PLACE1006318, PLACE1006368, PLACE100 6506, PLACE1006904, PLACE1007557, PLACE1007877, PLACE1009048, PLACE1011109, PLACE10 11643, PLACE4000548, THYRO1000279, Y79AA1000410, Y79AA1002103,
Group (7): 32 clones
   The following 32 clones having low scores in both the ATGpr1 and ATGpr2 (Table 11) were judged to be full-length, since both scores were still the maximum in a cluster compared with those of the other clones(at least 2 clones or more) in the same cluster. The clones were novel clones, in which the number of the identical human ESTs for both the 5'-end and 3'-end sequences is 1 or higher and not more than 5.
   HEMBA1000251, HEMBA1001803, HEMBA1001918, HEMBA1002257, HEMBA1003064, HEMBA1003714 , HEMBA1004405, HEMBA1005508, HEMBB1000054, HEMBB1001142, MAMMA1000175, MAMMA100116 2, MAMMA1002972, NT2RM4000425, NT2RP2004512, NT2RP2005531, NT2RP2005942, NT2RP20065 54, NT2RP3001007, NT2RP3001318, OVARC1000017, OVARC1000068, OVARC1000486, PLACE1001 705, PLACE1002319, PLACE1007743, PLACE1007829, PLACE1008630, PLACE1009925, PLACE101 1492, PLACE1011749, THYRO1000793,
   The following 117 clones, selected by assembling the sequence of the other clones in the same cluster and human ESTs, have high fullness ratio. The clones were classified into the following group (8) and (9).
   HEMBA1001323, HEMBA1001330, HEMBA1001712, HEMBA1001820, HEMBA1002204, HEMBA1002349, HEMBA1002538, HEMBA1003309, HEMBA1003939, HEMBA1004015, HEMBA1004295, HEMBA100467 2, HEMBA1004865, HEMBA1005251, HEMBA1006158, HEMBA1006676, HEMBA1006779, HEMBA10072 88, HEMBB1000218, HEMBB1000272, HEMBB1000399, HEMBB1000491, HEMBB1000996, HEMBB1001 114, HEMBB1001850, HEMBB1002015, MAMMA1000287, MAMMA1001683, MAMMA1001686, MAMMA100 2612,
   NT2RM2000609, NT2RM4002438, NT2RM4002567, NT2RP2000270, NT2RP2000758, NT2RP2001290, NT2RP2001526, NT2RP2002124, NT2RP2002736, NT2RP2002753, NT2RP2003456, NT2RP200372 7, NT2RP2003871, NT2RP2003968, NT2RP2004321, NT2RP2004412, NT2RP2004580, NT2RP20052 93, NT2RP2005476, NT2RP2005753, NT2RP2005815, NT2RP2005841, NT2RP2005857, NT2RP2006 393, NT2RP2006467, NT2RP3000109, NT2RP3000449, NT2RP3001245, NT2RP3001634, NT2RP300 2056, NT2RP3002810, NT2RP3002955, NT2RP3003032, NT2RP3003138, NT2RP3003500, NT2RP30 03819, NT2RP4000078, NT2RP4000515, NT2RP4000517, NT2RP4001407, NT2RP4001889, NT2RP4 002905,
   OVARC1000071, OVARC1001883, PLACE1000292, PLACE1001007, PLACE1001395, PLACE1001691 , PLACE1001746, PLACE1001748, PLACE1001845, PLACE1002066, PLACE1003373, PLACE100390 0, PLACE1004118, PLACE1004256, PLACE1004284, PLACE1004336, PLACE1004506, PLACE10049 34, PLACE1005077, PLACE1005111, PLACE1005409, PLACE1005730, PLACE1006076, PLACE1006 360, PLACE1006470, PLACE1006760, PLACE1006867, PLACE1007045, PLACE1007111, PLACE100 7807, PLACE1008080, PLACE1008244, PLACE1008369, PLACE1008405, PLACE1008426, PLACE10 08621, PLACE1009020, PLACE1009621, PLACE1010089, PLACE1010270, PLACE3000276, THYRO1 000805, THYRO1001365, THYRO1001673, Y79AA1001848,
Group (8): 36 clones
   Among the 117 clones described above, the following 36 clones were novel clones, in which at least either of the 5'-end or 3'-end sequence, or both of them were not identical to any of human ESTs.
   HEMBA1001330, HEMBA1001712, HEMBA1001820, HEMBA1002204, HEMBA1002349, HEMBA1003939 , HEMBA1004295, HEMBA1004865, HEMBA1006779, HEMBB1000218, HEMBB1000491, HEMBB100099 6, HEMBB1001114, MAMMA1000287, MAMMA1001686, MAMMA1002612, NT2RP2002736, NT2RP20037 27, NT2RP2004580, NT2RP2005841, NT2RP2006393, PLACE1002066, PLACE1003373, PLACE1003 900, PLACE1004118, PLACE1004336, PLACE1004506, PLACE1004934, PLACE1005409, PLACE100 5730, PLACE1006470, PLACE1008080, PLACE3000276, THYRO1000805, THYRO1001365, THYRO10 01673,
Group (9): 81 clones
   Among the 117 clones described above, the following 81 clones were the clones in which the number of the identical human ESTs for the 5'-end sequence is not more than 20 (except clones in which at least either of the 5'-end or 3'-end sequence, or both of them are not identical to any of human ESTs, and clones in which the number of the identical human ESTs for both the 5'-end and 3'-end sequences is 1 or higher and not more than 5).
   HEMBA1001323, HEMBA1002538, HEMBA1003309, HEMBA1004015, HEMBA1004672, HEMBA1005251 , HEMBA1006158, HEMBA1006676, HEMBA1007288, HEMBB1000272, HEMBB1000399, HEMBB100185 0, HEMBB1002015, MAMMA1001683, NT2RM2000609, NT2RM4002438, NT2RM4002567, NT2RP20002 70, NT2RP2000758, NT2RP2001290, NT2RP2001526, NT2RP2002124, NT2RP2002753, NT2RP2003 456, NT2RP2003871, NT2RP2003968, NT2RP2004321, NT2RP2004412, NT2RP2005293, NT2RP200 5476, NT2RP2005753, NT2RP2005815, NT2RP2005857, NT2RP2006467, NT2RP3000109, NT2RP30 00449, NT2RP3001245, NT2RP3001634, NT2RP3002056, NT2RP3002810, NT2RP3002955, NT2RP3 003032, NT2RP3003138, NT2RP3003500, NT2RP3003819, NT2RP4000078, NT2RP4000515, NT2RP 4000517, NT2RP4001407, NT2RP4001889, NT2RP4002905,
   OVARC1000071, OVARC1001883, PLACE1000292, PLACE1001007, PLACE1001395, PLACE1001691 , PLACE1001746, PLACE1001748, PLACE1001845, PLACE1004256, PLACE1004284, PLACE100507 7, PLACE1005111, PLACE1006076, PLACE1006360, PLACE1006760, PLACE1006867, PLACE10070 45, PLACE1007111, PLACE1007807, PLACE1008244, PLACE1008369, PLACE1008405, PLACE1008 426, PLACE1008621, PLACE1009020, PLACE1009621, PLACE1010089, PLACE1010270, Y79AA100 1848,
Group (10): 938 clones
   The following 938 clones having low scores in both the ATGpr1 and ATGpr2 were judged to be full-length according to the fullness ratio shown in Table 4. The clones were novel clones, in which at least the 5'-end sequence was not identical to any of human ESTs.
   HEMBA1000042, HEMBA1000213, HEMBA1000264, HEMBA1000355, HEMBA1000366, HEMBA1000411 , HEMBA1000422, HEMBA1000428, HEMBA1000434, HEMBA1000442, HEMBA1000464, HEMBA100054 0, HEMBA1000563, HEMBA1000569, HEMBA1000655, HEMBA1000662, HEMBA1000702, HEMBA10007 05, HEMBA1000722, HEMBA1000747, HEMBA1000769, HEMBA1000773, HEMBA1000774, HEMBA1000 827, HEMBA1000843, HEMBA1000852, HEMBA1000870, HEMBA1000908, HEMBA1000985, HEMBA100 0991, HEMBA1001017, HEMBA1001019, HEMBA1001094, HEMBA1001121, HEMBA1001172, HEMBA10 01265, HEMBA1001327, HEMBA1001375, HEMBA1001432, HEMBA1001454, HEMBA1001463, HEMBA1 001515, HEMBA1001517, HEMBA1001557, HEMBA1001566, HEMBA1001585, HEMBA1001589, HEMBA 1001608, HEMBA1001675, HEMBA1001678, HEMBA1001681, HEMBA1001718, HEMBA1001761, HEMB A1001945, HEMBA1001960, HEMBA1001964, HEMBA1001991,
   HEMBA1002003, HEMBA1002008, HEMBA1002039, HEMBA1002100, HEMBA1002113, HEMBA1002139 , HEMBA1002144, HEMBA1002153, HEMBA1002226, HEMBA1002253, HEMBA1002270, HEMBA100232 1, HEMBA1002328, HEMBA1002337, HEMBA1002381, HEMBA1002486, HEMBA1002552, HEMBA10025 55, HEMBA1002558, HEMBA1002561, HEMBA1002590, HEMBA1002628, HEMBA1002629, HEMBA1002 651, HEMBA1002659, HEMBA1002666, HEMBA1002678, HEMBA1002712, HEMBA1002716, HEMBA100 2730, HEMBA1002748, HEMBA1002780, HEMBA1002801, HEMBA1002826, HEMBA1002833, HEMBA10 02886, HEMBA1002896, HEMBA1003083, HEMBA1003086, HEMBA1003098, HEMBA1003133, HEMBA1 003197, HEMBA1003212, HEMBA1003273, HEMBA1003278, HEMBA1003304, HEMBA1003314, HEMBA 1003328, HEMBA1003330, HEMBA1003348, HEMBA1003376, HEMBA1003384, HEMBA1003595, HEMB A1003597, HEMBA1003622, HEMBA1003637, HEMBA1003656, HEMBA1003715, HEMBA1003725, HEM BA1003733, HEMBA1003758, HEMBA1003773, HEMBA1003784, HEMBA1003885, HEMBA1003937, HE MBA1003950, HEMBA1003976,
   HEMBA1004012, HEMBA1004045, HEMBA1004138, HEMBA1004241, HEMBA1004272, HEMBA1004306 , HEMBA1004312, HEMBA1004334, HEMBA1004354, HEMBA1004356, HEMBA1004366, HEMBA100439 4, HEMBA1004396, HEMBA1004429, HEMBA1004460, HEMBA1004461, HEMBA1004482, HEMBA10045 06, HEMBA1004538, HEMBA1004586, HEMBA1004617, HEMBA1004629, HEMBA1004631, HEMBA1004 666, HEMBA1004670, HEMBA1004733, HEMBA1004816, HEMBA1004820, HEMBA1004918, HEMBA100 4956, HEMBA1004960, HEMBA1004978, HEMBA1004995, HEMBA1005008, HEMBA1005062, HEMBA10 05274, HEMBA1005275, HEMBA1005293, HEMBA1005304, HEMBA1005315, HEMBA1005372, HEMBA1 005389, HEMBA1005426, HEMBA1005500, HEMBA1005506, HEMBA1005511, HEMBA1005520, HEMBA 1005568, HEMBA1005570, HEMBA1005616, HEMBA1005627, HEMBA1005631, HEMBA1005632, HEMB A1005670, HEMBA1005699, HEMBA1005705, HEMBA1005765, HEMBA1005780, HEMBA1005853, HEM BA1005909, HEMBA1005931, HEMBA1005934, HEMBA1005962, HEMBA1005999,
   HEMBA1006002, HEMBA1006005, HEMBA1006042, HEMBA1006067, HEMBA1006090, HEMBA1006142, HEMBA1006268, HEMBA1006334, HEMBA1006359, HEMBA1006416, HEMBA1006419, HEMBA100642 1, HEMBA1006446, HEMBA1006471, HEMBA1006486, HEMBA1006489, HEMBA1006546, HEMBA10065 69, HEMBA1006595, HEMBA1006617, HEMBA1006635, HEMBA1006648, HEMBA1006665, HEMBA1006 821, HEMBA1006849, HEMBA1006865, HEMBA1006929, HEMBA1007045, HEMBA1007051, HEMBA100 7073, HEMBA1007113, HEMBA1007256, HEMBA1007273, HEMBA1007322, HEMBA1007347, HEMBB1000039, HEMBB1000044, HEMBB1000059, HEMBB1000089, HEMBB1000099, HEMBB1000113 , HEMBB1000141, HEMBB1000250, HEMBB1000264, HEMBB1000274, HEMBB1000284, HEMBB100030 7, HEMBB1000312, HEMBB1000317, HEMBB1000337, HEMBB1000341, HEMBB1000343, HEMBB10003 69, HEMBB1000376, HEMBB1000434, HEMBB1000441, HEMBB1000455, HEMBB1000472, HEMBB1000 518, HEMBB1000530, HEMBB1000564, HEMBB1000575, HEMBB1000586, HEMBB1000598, HEMBB100 0637, HEMBB1000638, HEMBB1000643, HEMBB1000665, HEMBB1000726, HEMBB1000738, HEMBB10 00770, HEMBB1000794, HEMBB1000821, HEMBB1000822, HEMBB1000883, HEMBB1000888, HEMBB1 000890, HEMBB1000910, HEMBB1000959, HEMBB1000981, HEMBB1001037, HEMBB1001051, HEMBB 1001060, HEMBB1001133, HEMBB1001208, HEMBB1001209, HEMBB1001218, HEMBB1001221, HEMB B1001249, HEMBB1001254, HEMBB1001267, HEMBB1001282, HEMBB1001302, HEMBB1001304, HEM BB1001335, HEMBB1001348, HEMBB1001356, HEMBB1001366, HEMBB1001380, HEMBB1001443, HE MBB1001463, HEMBB1001464,
   HEMBB1001521, HEMBB1001527, HEMBB1001537, HEMBB1001555, HEMBB1001586, HEMBB1001619 , HEMBB1001630, HEMBB1001637, HEMBB1001641, HEMBB1001706, HEMBB1001735, HEMBB100175 3, HEMBB1001762, HEMBB1001802, HEMBB1001880, HEMBB1001899, HEMBB1001921, HEMBB10019 30, HEMBB1001952, HEMBB1001953, HEMBB1001973, HEMBB1001988, HEMBB1002002, HEMBB1002 005, HEMBB1002009, HEMBB1002042, HEMBB1002049, HEMBB1002069, HEMBB1002115, HEMBB100 2139, HEMBB1002189, HEMBB1002232, HEMBB1002254, HEMBB1002255, HEMBB1002280, HEMBB10 02306, HEMBB1002364, HEMBB1002371, HEMBB1002381, HEMBB1002453, HEMBB1002477, HEMBB1 002509, HEMBB1002531, HEMBB1002601, HEMBB1002603, HEMBB1002610, HEMBB1002613, HEMBB 1002623, HEMBB1002635, HEMBB1002664, HEMBB1002677, HEMBB1002683, HEMBB1002686, HEMB B1002699, HEMBB1002712,
   MAMMA1000009, MAMMA1000045, MAMMA1000103, MAMMA1000133, MAMMA1000134, MAMMA1000155 , MAMMA1000241, MAMMA1000254, MAMMA1000264, MAMMA1000266, MAMMA1000340, MAMMA100038 5, MAMMA1000423, MAMMA1000424, MAMMA1000446, MAMMA1000478, MAMMA1000501, MAMMA10005 16, MAMMA1000565, MAMMA1000585, MAMMA1000605, MAMMA1000616, MAMMA1000621, MAMMA1000 643, MAMMA1000669, MAMMA1000696, MAMMA1000707, MAMMA1000714, MAMMA1000718, MAMMA100 0732, MAMMA1000733, MAMMA1000839, MAMMA1000845, MAMMA1000855, MAMMA1000863, MAMMA10 00867, MAMMA1000880, MAMMA1000905, MAMMA1000942, MAMMA1000957, MAMMA1000987, MAMMA1 000998, MAMMA1001003, MAMMA1001024, MAMMA1001030, MAMMA1001035, MAMMA1001050, MAMMA 1001067, MAMMA1001082, MAMMA1001092, MAMMA1001145, MAMMA1001161, MAMMA1001203, MAMM A1001243, MAMMA1001268, MAMMA1001280, MAMMA1001298, MAMMA1001322, MAMMA1001324, MAM MA1001383, MAMMA1001408, MAMMA1001435, MAMMA1001442, MAMMA1001446,
   MAMMA1001501, MAMMA1001575, MAMMA1001606, MAMMA1001671, MAMMA1001715, MAMMA1001740 , MAMMA1001788, MAMMA1001818, MAMMA1001854, MAMMA1001878, MAMMA1001931, MAMMA100199 2, MAMMA1002032, MAMMA1002082, MAMMA1002084, MAMMA1002108, MAMMA1002118, MAMMA10021 40, MAMMA1002215, MAMMA1002267, MAMMA1002298, MAMMA1002299, MAMMA1002310, MAMMA1002 322, MAMMA1002339, MAMMA1002347, MAMMA1002352, MAMMA1002361, MAMMA1002392, MAMMA100 2417, MAMMA1002428, MAMMA1002446, MAMMA1002475, MAMMA1002480, MAMMA1002545, MAMMA10 02556, MAMMA1002566, MAMMA1002597, MAMMA1002603, MAMMA1002623, MAMMA1002625, MAMMA1 002629, MAMMA1002636, MAMMA1002662, MAMMA1002698, MAMMA1002708, MAMMA1002721, MAMMA 1002744, MAMMA1002754, MAMMA1002758, MAMMA1002780, MAMMA1002833, MAMMA1002835, MAMM A1002838, MAMMA1002844, MAMMA1002858, MAMMA1002880, MAMMA1002887, MAMMA1002908, MAM MA1002930, MAMMA1002970, MAMMA1002982, MAMMA1003003, MAMMA1003019, MAMMA1003026, MA MMA1003031, MAMMA1003040, MAMMA1003055,
   NT2RM4000046, NT2RM4000085, NT2RM4000200, NT2RM4000244, NT2RM4000368, NT2RM4000979 , NT2RM4001016, NT2RM4001519, NT2RM4001557, NT2RM4001605, NT2RM4001810, NT2RM400185 8, NT2RM4001930, NT2RM4002067, NT2RM4002278, NT2RM4002281, NT2RM4002287, NT2RM40023 83, NT2RM4002479, NT2RM4002504, NT2RM4002532, NT2RP2000027, NT2RP2000098, NT2RP2000 258, NT2RP2000327, NT2RP2000337, NT2RP2000459, NT2RP2000498, NT2RP2000987, NT2RP200 1277, NT2RP2001312, NT2RP2001423, NT2RP2001445, NT2RP2001449, NT2RP2001677, NT2RP20 01699, NT2RP2001720, NT2RP2001740, NT2RP2001762, NT2RP2001813, NT2RP2001869, NT2RP2 001943, NT2RP2002032, NT2RP2002070, NT2RP2002105, NT2RP2002137, NT2RP2002192, NT2RP 2002256, NT2RP2002259, NT2RP2002394, NT2RP2002439, NT2RP2002457, NT2RP2002504, NT2R P2002606, NT2RP2002727, NT2RP2002740, NT2RP2002752, NT2RP2002839, NT2RP2002987, NT2RP2003129, NT2RP2003161, NT2RP2003164, NT2RP2003230, NT2RP2003339, NT2RP2003499, NT2RP2003522, NT2RP2003559, NT2RP2003706, NT2RP2003770, NT2RP2003859, NT2RP200388 5, NT2RP2004014, NT2RP2004142, NT2RP2004170, NT2RP2004172, NT2RP2004207, NT2RP20042 70, NT2RP2004300, NT2RP2004339, NT2RP2004347, NT2RP2004425, NT2RP2004490, NT2RP2004 675, NT2RP2004709, NT2RP2004967, NT2RP2005020, NT2RP2005031, NT2RP2005108, NT2RP200 5254, NT2RP2005325, NT2RP2005336, NT2RP2005344, NT2RP2005354, NT2RP2005360, NT2RP20 05491, NT2RP2005501, NT2RP2005581, NT2RP2005645, NT2RP2005651, NT2RP2005701, NT2RP2 005719, NT2RP2005741, NT2RP2005908, NT2RP2005980, NT2RP2006098, NT2RP2006103, NT2RP 2006166, NT2RP2006184, NT2RP2006320, NT2RP2006321, NT2RP2006323, NT2RP2006534, NT2R P2006598,
   NT2RP3000186, NT2RP3000235, NT2RP3000247, NT2RP3000433, NT2RP3000441, NT2RP3000578, NT2RP3000584, NT2RP3000622, NT2RP3000628, NT2RP3000685, NT2RP3000742, NT2RP300081 5, NT2RP3000875, NT2RP3000904, NT2RP3001115, NT2RP3001232, NT2RP3001374, NT2RP30014 59, NT2RP3001527, NT2RP3001607, NT2RP3001752, NT2RP3001782, NT2RP3001926, NT2RP3001 989, NT2RP3002002, NT2RP3002166, NT2RP3002352, NT2RP3002687, NT2RP3002713, NT2RP300 2770, NT2RP3002799, NT2RP3002978, NT2RP3003059, NT2RP3003121, NT2RP3003464, NT2RP30 03572, NT2RP3003746, NT2RP3003799, NT2RP3003989, NT2RP3004016, NT2RP3004095, NT2RP3 004110, NT2RP3004145, NT2RP3004148, NT2RP3004215, NT2RP3004399, NT2RP3004475, NT2RP 3004503, NT2RP4000321, NT2RP4000519, NT2RP4000996,
   OVARC1000133, OVARC1000198, OVARC1000240, OVARC1000427, OVARC1000431, OVARC1000533 , OVARC1000543, OVARC1000573, OVARC1000578, OVARC1000678, OVARC1000679, OVARC100070 0, OVARC1000769, OVARC1000802, OVARC1000891, OVARC1000897, OVARC1000936, OVARC10009 37, OVARC1000960, OVARC1000971, OVARC1001000, OVARC1001062, OVARC1001072, OVARC1001 085, OVARC1001118, OVARC1001129, OVARC1001169, OVARC1001261, OVARC1001268, OVARC100 1282, OVARC1001330, OVARC1001339, OVARC1001342, OVARC1001357, OVARC1001442, OVARC10 01480, OVARC1001542, OVARC1001547, OVARC1001615, OVARC1001726, OVARC1001795, OVARC1 001900, OVARC1001901, OVARC1001911, OVARC1001989, OVARC1002044, OVARC1002066, OVARC 1002143,
   PLACE1000031, PLACE1000040, PLACE1000078, PLACE1000094, PLACE1000214, PLACE1000332 , PLACE1000421, PLACE1000424, PLACE1000481, PLACE1000749, PLACE1000841, PLACE100093 1, PLACE1000979, PLACE1001015, PLACE1001076, PLACE1001118, PLACE1001136, PLACE10012 79, PLACE1001384, PLACE1001468, PLACE1001502, PLACE1001534, PLACE1001603, PLACE1001 611, PLACE1001640, PLACE1001716, PLACE1001720, PLACE1001745, PLACE1001799, PLACE100 1821, PLACE1001897, PLACE1002052, PLACE1002150, PLACE1002256, PLACE1002493, PLACE10 02514, PLACE1002578, PLACE1002591, PLACE1002598, PLACE1002768, PLACE1002782, PLACE1 002881, PLACE1002962, PLACE1003153, PLACE1003258, PLACE1003343, PLACE1003375, PLACE 1003401, PLACE1003519, PLACE1003521, PLACE1003528, PLACE1003575, PLACE1003583, PLAC E1003584, PLACE1003638, PLACE1003760, PLACE1003768, PLACE1003833, PLACE1003850, PLA CE1003858, PLACE1003932,
   PLACE1004242, PLACE1004257, PLACE1004425, PLACE1004467, PLACE1004471, PLACE1004518 , PLACE1004658, PLACE1004693, PLACE1004813, PLACE1004827, PLACE1004838, PLACE100484 0, PLACE1004900, PLACE1004972, PLACE1005146, PLACE1005162, PLACE1005453, PLACE10054 71, PLACE1005477, PLACE1005502, PLACE1005526, PLACE1005528, PLACE1005574, PLACE1005 584, PLACE1005611, PLACE1005802, PLACE1005884, PLACE1005898, PLACE1005968, PLACE100 6002, PLACE1006129, PLACE1006143, PLACE1006187, PLACE1006205, PLACE1006223, PLACE10 06382, PLACE1006521, PLACE1006534, PLACE1006540, PLACE1006617, PLACE1006640, PLACE1 006779, PLACE1006792, PLACE1006800, PLACE1006805, PLACE1006815, PLACE1006860, PLACE 1006878, PLACE1007097, PLACE1007132, PLACE1007140, PLACE1007276, PLACE1007286, PLAC E1007525, PLACE1007677, PLACE1007737, PLACE1007866,
   PLACE1008045, PLACE1008111, PLACE1008201, PLACE1008231, PLACE1008392, PLACE1008424, PLACE1008437, PLACE1008455, PLACE1008625, PLACE1008748, PLACE1008757, PLACE100879 8, PLACE1008807, PLACE1008920, PLACE1008947, PLACE1009150, PLACE1009246, PLACE10093 08, PLACE1009410, PLACE1009477, PLACE1009613, PLACE1009639, PLACE1009879, PLACE1009 888, PLACE1009924, PLACE1009935, PLACE1010069, PLACE1010083, PLACE1010181, PLACE101 0341, PLACE1010624, PLACE1010628, PLACE1010631, PLACE1010662, PLACE1010916, PLACE10 10947, PLACE1011057, PLACE1011133, PLACE1011143, PLACE1011296, PLACE1011340, PLACE1 011419, PLACE1011503, PLACE1011641, PLACE1011649, PLACE1011650, PLACE1011729, PLACE 1011778, PLACE1011874, PLACE1011962, PLACE1011995,
   PLACE2000003, PLACE2000103, PLACE2000115, PLACE2000132, PLACE2000140, PLACE2000176 , PLACE2000317, PLACE2000342, PLACE2000419, PLACE2000435, PLACE2000455, PLACE200046 5, PLACE3000004, PLACE3000124, PLACE3000136, PLACE3000158, PLACE3000169, PLACE30001 99, PLACE3000208, PLACE3000218, PLACE3000341, PLACE3000362, PLACE3000365, PLACE3000 388, PLACE3000400, PLACE3000402, PLACE3000475, PLACE4000093, PLACE4000100, PLACE400 0233, PLACE4000252, PLACE4000379, PLACE4000411, PLACE4000494,
   THYRO1000026, THYRO1000035, THYRO1000085, THYRO1000132, THYRO1000156, THYRO1000186 , THYRO1000190, THYRO1000221, THYRO1000241, THYRO1000438, THYRO1000452, THYRO100055
   8, THYRO1000602, THYRO1000641, THYRO1000658, THYRO1000699, THYRO1000748, THYRO10007 96, THYRO1000843, THYRO1000895, THRO1000974, THYRO1000975, THYRO1001062, THYRO1001 093, THYRO1001121, THYRO1001177, THYRO1001262, THYRO1001290, THYRO1001322, THYRO100 1411, THYRO1001480, THYRO1001541, THYRO1001573, THYRO1001617, THYRO1001895, Y79AA10 00065, Y79AA1000405, Y79AA1000538, Y79AA1000802, Y79AA1000969, Y79AA1001061, Y79AA1 001167, Y79AA1001384, Y79AA1001594, Y79AA1001875, Y79AA1002211,
Group (11): 228 clones
   The following 228 clones having low scores in both the ATGpr1 and ATGpr2 were judged to be full-length according to the fullness ratio shown in Table 7. The clones were a novel clone, in which at least the 3'-end sequence was not identical to any of human ESTs.
   HEMBA1000111, HEMBA1000243, HEMBA1000338, HEMBA1000390, HEMBA1000418, HEMBA1000459 , HEMBA1000518, HEMBA1000791, HEMBA1000974, HEMBA1001007, HEMBA1001077, HEMBA100109 9, HEMBA1001123, HEMBA1001294, HEMBA1001299, HEMBA1001442, HEMBA1001861, HEMBA10019 42, HEMBA1001979, HEMBA1002160, HEMBA1002162, HEMBA1002166, HEMBA1002389, HEMBA1002 498, HEMBA1002592, HEMBA1002679, HEMBA1002944, HEMBA1003034, HEMBA1003142, HEMBA100 3548, HEMBA1003571, HEMBA1003579, HEMBA1003598, HEMBA1003630, HEMBA1003958, HEMBA10 04038, HEMBA1004042, HEMBA1004049, HEMBA1004132, HEMBA1004225, HEMBA1004748, HEMBA1 004770, HEMBA1005039, HEMBA1005152, HEMBA1005159, HEMBA1005359, HEMBA1005408, HEMBA 1005410, HEMBA1005443, HEMBA1005497, HEMBA1005552, HEMBA1005634, HEMBA1005717, HEMB A1005829, HEMBA1005921, HEMBA1006328, HEMBA1006438, HEMBA1006540, HEMBA1006780, HEM BA1006921, HEMBA1006938, HEMBA1007017, HEMBA1007341,
   HEMBB1000050, HEMBB1000420, HEMBB1000487, HEMBB1000490, HEMBB1000523, HEMBB1000684 , HEMBB1000840, HEMBB1000876, HEMBB1000913, HEMBB1000915, HEMBB1000917, HEMBB100106 3, HEMBB1001102, HEMBB1001177, HEMBB1001253, HEMBB1001271, HEMBB1001454, HEMBB10015 00, HEMBB1001588, HEMBB1001618, HEMBB1001704, HEMBB1001717, HEMBB1001756, HEMBB1001 867, HEMBB1001967, HEMBB1002043, HEMBB1002094, HEMBB1002190, HEMBB1002520, HEMBB100 2556, HEMBB1002590,
   MAMMA1000117, MAMMA1000227, MAMMA1000270, MAMMA1000302, MAMMA1000348, MAMMA1000413 , MAMMA1000431, MAMMA1000483, MAMMA1000559, MAMMA1000723, MAMMA1000744, MAMMA100075 2, MAMMA1000782, MAMMA1000802, MAMMA1000851, MAMMA1000877, MAMMA1000962, MAMMA10010 74, MAMMA1001133, MAMMA1001191, MAMMA1001206, MAMMA1001249, MAMMA1001330, MAMMA1001 452, MAMMA1001502, MAMMA1001663, MAMMA1001806, MAMMA1001836, MAMMA1001880, MAMMA100 1890, MAMMA1001908, MAMMA1001963, MAMMA1002058, MAMMA1002155, MAMMA1002158, MAMMA10 02230, MAMMA1002282, MAMMA1002293, MAMMA1002332, MAMMA1002359, MAMMA1002494, MAMMA1 002573, MAMMA1002618, MAMMA1002622, MAMMA1002646, MAMMA1002748, MAMMA1002871, MAMMA 1002892, MAMMA1003140,
   NT2RM4000327, NT2RM4000532, NT2RM4001414, NT2RM4001776, NT2RM4002499, NT2RP2000076 , NT2RP2000523, NT2RP2000603, NT2RP2000644, NT2RP2000731, NT2RP2001196, NT2RP200167 8, NT2RP2001926, NT2RP2003206, NT2RP2003704, NT2RP2003912, NT2RP2004396, NT2RP20046 81, NT2RP3000512, NT2RP3000542, NT2RP3000736, NT2RP3000865, NT2RP3002057, NT2RP3003 384, NT2RP3004334, NT2RP3004349, NT2RP3004527, NT2RP4000049, OVARC1000058, OVARC100 0191, OVARC1000302, OVARC1001051, OVARC1001117, OVARC1001668, OVARC1001745, OVARC10 01812, OVARC1001928,
   PLACE1000583, PLACE1001272, PLACE1001414, PLACE1002537, PLACE1003205, PLACE1003361 , PLACE1003516, PLACE1003864, PLACE1004384, PLACE1004491, PLACE1004793, PLACE100498 5, PLACE1005085, PLACE1005666, PLACE1005845, PLACE1006164, PLACE1006262, PLACE10067 54, PLACE1007852, PLACE1008331, PLACE1008643, PLACE1009050, PLACE1009155, PLACE1009 172, PLACE1009183, PLACE1009200, PLACE1009398, PLACE1009595, PLACE1010702, PLACE101 0891, PLACE1011165, PLACE1011203, PLACE1011375, PLACE1011576, PLACE1011762, PLACE10 11964, PLACE2000136, PLACE2000264, PLACE2000305, PLACE2000379, PLACE3000220, PLACE4 000250, THYRO1000092,THYRO1000471, THYRO1000852, THYRO1001534, THYRO1001595, THYRO 1001745.

### EXAMPLE 12

### Homology search using the 5'-end and 3'-end sequences of the selected clones.

The 5' -end sequences of the selected 5547 clones were used for the homology search of the SwissProt, and both the 5' -end 3' -end sequences were used for the search of the GenBank and UniGene (ref. the result of the search of the SwissProt, GenBank (except ESTs and STSs), and UniGene (Human) was attached).

Each search result is shown in the last part of this SPECIFICATION by arranging each item in the following format.

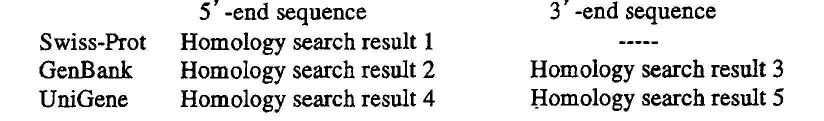

According to the top hit data, at least 1430 clones were predicted to encode a protein belonging to any of the categories, secretory or membrane protein, glycoprotein, protein associated with signal transduction, protein associated with transcription, protein associated with diseases, enzyme or protein associated with metabolism, protein associated with cell division or cell proliferation, protein associated with cytoskeleton, protein associated with RNA synthesis, nuclear protein, protein associated with protein synthesis or transport, protein associated with cellular defense, or protein associated with development or growth. Among the clones predicted belonging to any of the categories, 1001 clones were estimated to have a relatively high homology with the known proteins or genes in the same category. In addition, 429 clones were estimated to have a relatively low homology with the known proteins in the same category.

Herein, the term "relatively high homology" is defined as having 60% or more identity and the P-value 10⁻¹⁰ or less in comparison with known sequences in the SwissProt database, or 64% or more identity and the P-value 10⁻¹⁵ or less in comparison with those in the GenBank and UniGene databases (see the attached list). Also, the term "relatively low homology" is defined as not fulfilling the requirements to be "relatively high homology", but still having the scores, 25% or more identity and the P-value 10⁻⁶ or less, using the sequence having 55 nucleotides or more, in comparison with known sequences in the SwissProt database (see the attached list). The P-value is a score obtained statistically by taking into account the probability of occurrence of the similarity between two sequences. In general, the smaller P-value reflects the higher similarity (Altschul S.F., Gish W., Miller W., Myers E.W., and Lipman D.J. (1990) "Basic local alignment search tool" J.Mol. Biol., 215: 403-410; Gish W., and States D.J. (1993) "Identification of protein coding regions by database similarity search" Nature Genet. 3: 266-272).

The clones predicted to encode a protein in the category of secretory protein or membrane protein have the keywords, "signal", "transmembrane", "membrane", "extracellular matrix", "receptor", "G-protein coupled receptor", "ionic channel", "voltage-gated channel", "calcium channel", "cell adhesion", "collagen", or "connective tissue", or descriptions from which the clone can be predicted to be a secretory or membrane protein, in the top hit data of the SwissProt using the 5'-end sequence, or in the top hit data of the GenBank or UniGene using the 5'-end sequence and 3'-end sequence.

The clones predicted to encode a protein in the category of glycoprotein have the keywords, "glycoprotein", or descriptions from which the clone can be predicted to be a glycoprotein, in the top hit data of the SwissProt using the 5'-end sequence, or in the top hit data of the GenBank or UniGene using the 5'-end sequence and 3'-end sequence.

The clones predicted to encode a protein in the category of proteins associated with signal transduction have the keywords, "serine/threonine-protein kinase", "tyrosine-protein kinase", "SH3 domain", or "WD repeat", or descriptions from which the clone can be predicted to be a protein associated with signal transduction (such as "ADP-ribosylation factor"), in the top hit data of the SwissProt using the 5'-end sequence, or in the top hit data of the GenBank or UniGene using the 5'-end sequence and 3'-end sequence.

The clones predicted to encode a protein in the category of proteins associated with transcription have the keywords, "transcription regulation", "zinc finger", or "homeobox", or descriptions from which the clone can be predicted to be a protein associated with transcription, in the top hit data of the SwissProt using the 5'-end sequence, or in the top hit data of the GenBank or UniGene using the 5'-end sequence and 3'-end sequence.

The clones predicted to encode a protein in the category of proteins associated with diseases are the clones in which the top hit data of the SwissProt using the 5'-end sequence, or the top hit data of the GenBank or UniGene using the 5'-end sequence and 3'-end sequence is a gene or protein that is deposited in the Online Mendelian Inheritance in Man (OMIM) database, which is a database of human genes and diseases, or the top hit data has descriptions from which the clone can be predicted to be a protein associated with diseases.

The clones predicted to encode a protein in the category of enzyme or proteins associated with metabolism are the clones in which the top hit data of the SwissProt using the 5'-end sequence, or the top hit data of the GenBank or UniGene using the 5'-end sequence and 3'-end sequence is a gene or protein with E.C.No. (Enzyme commission number), or the top hit data has descriptions from which the clone can be predicted to be an enzyme or protein associated with metabolism (such as "metabolism", "oxidoreductase", or "lipid").

The clones predicted to encode a protein in the category of proteins associated with cell division or cell proliferation have the keywords, "cell division", "cell cycle", "mitosis", or "chromosomal protein", or descriptions from which the clone can be predicted to be a protein associated with cell division or cell proliferation (such as "histone", "cell growth", or "apoptosis"), in the top hit data of the SwissProt using the 5'-end sequence, or in the top hit data of the GenBank or UniGene using the 5'-end sequence and 3'-end sequence.

The clones predicted to encode a protein in the category of proteins associated with cytoskeleton have the keywords, "structural protein", "cytoskeleton", "actin-binding", or "microtubules", or descriptions from which the clone can be predicted to be a protein associated with cytoskeleton, in the top hit data of the SwissProt using the 5'-end sequence, or in the top hit data of the GenBank or UniGene using the 5'-end sequence and 3'-end sequence.

The clones predicted to encode a protein in the category of proteins associated with RNA synthesis include the above clones predicted to be a protein associated with transcription, and also the clones which have the keywords, "RNA splicing", or "RNA processing", or descriptions from which the clone can be predicted to be a protein associated with RNA synthesis (such as "RNA helicase", "polyadenylation", or "RNA transport"), in the top hit data of the SwissProt using the 5'-end sequence, or in the top hit data of the GenBank or UniGene using the 5'-end sequence and 3'-end sequence.

The clones predicted to encode a protein in the category of nuclear protein include the above clones predicted to be a protein associated with transcription, and also the clones which have the keyword, "nuclear protein", or descriptions from which the clone can be predicted to be a nuclear protein, in the top hit data of the SwissProt using the 5'-end sequence, or in the top hit data of the GenBank or UniGene using the 5'-end sequence and 3'-end sequence.

The clones predicted to encode a protein in the category of proteins associated with protein synthesis or transport have the keywords, "translation regulation", "protein biosynthesis", "amino-acid biosynthesis", "ribosomal protein", or "protein transport", or descriptions from which the clone can be predicted to be a protein associated with protein synthesis or transport (such as "signal recognition particle", "ubiquitin", "proteosome", or "protease"), in the top hit data of the SwissProt using the 5'-end sequence, or in the top hit data of the GenBank or UniGene using the 5'-end sequence and 3'-end sequence.

The clones predicted to encode a protein in the category of proteins associated with cellular defense have the keywords, "heat shock", "chaperone", "DNA repair", or "DNA damage", or descriptions from which the clone can be predicted to be a protein associated with cellular defense, in the top hit data of the SwissProt using the 5'-end sequence, or in the top hit data of the GenBank or UniGene using the 5'-end sequence and 3'-end sequence.

The clones predicted to encode a protein in the category of proteins associated with development or growth have the keyword, "developmental protein", or descriptions from which the clone can be predicted to be a protein associated with development or growth, in the top hit data of the SwissProt using the 5'-end sequence, or in the top hit data of the GenBank or UniGene using the 5'-end sequence and 3'-end sequence.

The following 1430 clones were predicted to encode a protein belonging to any of the categories, secretory or membrane protein, glycoprotein, protein associated with signal transduction, protein associated with transcription, protein associated with diseases, enzyme or protein associated with metabolism, protein associated with cell division or cell proliferation, protein associated with cytoskeleton, protein associated with RNA synthesis, nuclear protein, protein associated with protein synthesis or transport, protein associated with cellular defense, or protein associated with development or growth.
HEMBA1000005, HEMBA1000012, HEMBA1000020, HEMBA1000030, HEMBA1000158, HEMBA1000185 , HEMBA1000201, HEMBA1000216, HEMBA1000303, HEMBA1000459, HEMBA1000488, HEMBA100049 1, HEMBA1000523, HEMBA1000531, HEMBA1000542, HEMBA1000561, HEMBA1000569, HEMBA10005 88, HEMBA1000591, HEMBA1000657, HEMBA1000673, HEMBA1000752, HEMBA1000827, HEMBA1000 851, HEMBA1000852, HEMBA1000972, HEMBA1000991, HEMBA1001017, HEMBA1001019, HEMBA100 1059, HEMBA1001071, HEMBA1001088, HEMBA1001123, HEMBA1001137, HEMBA1001174, HEMBA10 01257, HEMBA1001302, HEMBA1001351, HEMBA1001387, HEMBA1001407, HEMBA1001476, HEMBA1 001510, HEMBA1001569, HEMBA1001570, HEMBA1001579, HEMBA1001595, HEMBA1001620, HEMBA 1001672, HEMBA1001678, HEMBA1001714, HEMBA1001744, HEMBA1001800, HEMBA1001804, HEMB A1001809, HEMBA1001819, HEMBA1001822, HEMBA1001847, HEMBA1001896, HEMBA1001913, HEM BA1001921, HEMBA1002003, HEMBA1002035, HEMBA1002092, HEMBA1002102, HEMBA1002150, HE MBA1002160, HEMBA1002161, HEMBA1002162, HEMBA1002212, HEMBA1002229, HEMBA1002257, H EMBA1002341, HEMBA1002363, HEMBA1002417, HEMBA1002495, HEMBA1002513, HEMBA1002547, HEMBA1002555, HEMBA1002569, HEMBA1002609, HEMBA1002688, HEMBA1002716, HEMBA1002810 , HEMBA1002896, HEMBA1002939, HEMBA1002973, HEMBA1002999, HEMBA1003046, HEMBA100307 1, HEMBA1003077, HEMBA1003136, HEMBA1003148, HEMBA1003179, HEMBA1003235, HEMBA10032 86, HEMBA1003291, HEMBA1003314, HEMBA1003418, HEMBA1003433, HEMBA1003538,
HEMBA1003545, HEMBA1003555, HEMBA1003560, HEMBA1003568, HEMBA1003569, HEMBA1003581 , HEMBA1003598, HEMBA1003615, HEMBA1003621, HEMBA1003680, HEMBA1003684, HEMBA100369 0, HEMBA1003720, HEMBA1003760, HEMBA1003773, HEMBA1003783, HEMBA1003805, HEMBA10038 36, HEMBA1003866, HEMBA1003953, HEMBA1004097, HEMBA1004131, HEMBA1004168, HEMBA1004 202, HEMBA1004207, HEMBA1004227, HEMBA1004248, HEMBA1004276, HEMBA1004286, HEMBA100 4321, HEMBA1004353, HEMBA1004354, HEMBA1004356, HEMBA1004389, HEMBA1004408, HEMBA10 04479, HEMBA1004534, HEMBA1004604, HEMBA1004669, HEMBA1004734, HEMBA1004752, HEMBA1 004753, HEMBA1004756, HEMBA1004758, HEMBA1004795, HEMBA1004807, HEMBA1004847, HEMBA 1005009, HEMBA1005047, HEMBA1005202, HEMBA1005241, HEMBA1005293, HEMBA1005331, HEMB A1005338, HEMBA1005359, HEMBA1005367, HEMBA1005423, HEMBA1005443, HEMBA1005475, HEM BA1005513, HEMBA1005528, HEMBA1005548, HEMBA1005576, HEMBA1005581, HEMBA1005595, HE MBA1005679, HEMBA1005699, HEMBA1005732, HEMBA1005737, HEMBA1005815, HEMBA1005931, H EMBA1005963, HEMBA1005990, HEMBA1006173, HEMBA1006253, HEMBA1006272, HEMBA1006278, HEMBA1006310, HEMBA1006344, HEMBA1006347, HEMBA1006359, HEMBA1006474, HEMBA1006521, HEMBA1006540, HEMBA1006559, HEMBA1006562, HEMBA1006639, HEMBA1006648, HEMBA100665 2, HEMBA1006737, HEMBA1006807, HEMBA1006877, HEMBA1006914, HEMBA1006941, HEMBA10069 73, HEMBA1006976, HEMBA1007018, HEMBA1007045, HEMBA1007078, HEMBA1007121,
HEMBA1007174, HEMBA1007243, HEMBA1007300, HEMBA1007301, HEMBB1000037, HEMBB1000119 , HEMBB1000217, HEMBB1000250, HEMBB1000264, HEMBB1000317, HEMBB1000399, HEMBB100040 4, HEMBB1000530, HEMBB1000632, HEMBB1000637, HEMBB1000684, HEMBB1000693, HEMBB10007 25, HEMBB1000781, HEMBB1000789, HEMBB1000915, HEMBB1000927, HEMBB1001011, HEMBB1001 051, HEMBB1001068, HEMBB1001102, HEMBB1001112, HEMBB1001119, HEMBB1001133, HEMBB100 1151, HEMBB1001175, HEMBB1001177, HEMBB1001234, HEMBB1001242, HEMBB1001282, HEMBB10 01288, HEMBB1001294, HEMBB1001314, HEMBB1001331, HEMBB1001346, HEMBB1001384, HEMBB1 001429, HEMBB1001443, HEMBB1001482, HEMBB1001802, HEMBB1001831, HEMBB1001908, HEMBB 1001915, HEMBB1001922, HEMBB1001967, HEMBB1002042, HEMBB1002050, HEMBB1002092, HEMB B1002134, HEMBB1002193, HEMBB1002217, HEMBB1002342, HEMBB1002358, HEMBB1002442, HEM BB1002477, HEMBB1002600, HEMBB1002635, HEMBB1002683, MAMMA1000009, MAMMA1000020, MA MMA1000085, MAMMA1000092, MAMMA1000103, MAMMA1000155, MAMMA1000173, MAMMA1000183, M AMMA1000388, MAMMA1000424, MAMMA1000429, MAMMA1000672, MAMMA1000731, MAMMA1000734, MAMMA1000841, MAMMA1000897, MAMMA1000956, MAMMA1001008, MAMMA1001021, MAMMA1001030 , MAMMA1001041, MAMMA1001059, MAMMA1001075, MAMMA1001080, MAMMA1001105, MAMMA100113 9, MAMMA1001198, MAMMA1001305, MAMMA1001388, MAMMA1001476, MAMMA1001501, MAMMA10015 76, MAMMA1001633, MAMMA1001735, MAMMA1001751, MAMMA1001754, MAMMA1001771,
MAMMA1001820, MAMMA1001837, MAMMA1002140, MAMMA1002143, MAMMA1002170, MAMMA1002198 , MAMMA1002219, MAMMA1002236, MAMMA1002268, MAMMA1002352, MAMMA1002392, MAMMA100248 5, MAMMA1002530, MAMMA1002573, MAMMA1002598, MAMMA1002619, MAMMA1002622, MAMMA10026 36, MAMMA1002637, MAMMA1002650, MAMMA1002671, MAMMA1002699, MAMMA1002842, MAMMA1002 858, MAMMA1002869, MAMMA1002881, MAMMA1002937, MAMMA1002938, MAMMA1003011, MAMMA100 3047, MAMMA1003057, MAMMA1003099, MAMMA1003104, MAMMA1003113, MAMMA1003127, MAMMA10 03146, MAMMA1003166, NT2RM1000035, NT2RM1000055, NT2RM1000118, NT2RM1000132, NT2RM1 000153, NT2RM1000186, NT2RM1000187, NT2RM1000199, NT2RM1000256, NT2RM1000257, NT2RM 1000280, NT2RM1000318, NT2RM1000355, NT2RM1000377, NT2RM1000394, NT2RM1000430, NT2R M1000555, NT2RM1000648, NT2RM1000661, NT2RM1000666, NT2RM1000691, NT2RM1000725, NT2 RM1000742, NT2RM1000770, NT2RM1000772, NT2RM1000811, NT2RM1000826, NT2RM1000833, NT 2RM1000850, NT2RM1000852, NT2RM1000867, NT2RM1000882, NT2RM1000883, NT2RM1000894, N T2RM1000898, NT2RM1001003, NT2RM1001059, NT2RM1001072, NT2RM1001082, NT2RM1001092, NT2RM1001139, NT2RM2000013, NT2RM2000124, NT2RM2000191, NT2RM2000363, NT2RM2000368, , NT2RM2000371, NT2RM2000374, NT2RM2000402, NT2RM2000407, NT2RM2000422, NT2RM200050 4, NT2RM2000566, NT2RM2000577, NT2RM2000594, NT2RM2000599, NT2RM2000612, NT2RM20006 23, NT2RM2000691, NT2RM2000714, NT2RM2000735, NT2RM2000740, NT2RM2000821,
NT2RM2000951, NT2RM2001035, NT2RM2001065, NT2RM2001131, NT2RM2001221, NT2RM2001238 NT2RM2001256, NT2RM2001324, NT2RM2001345, NT2RM2001424, NT2RM2001499, NT2RM200157 5, NT2RM2001588, NT2RM2001592, NT2RM2001605, NT2RM2001613, NT2RM2001635, NT2RM20016 48, NT2RM2001652, NT2RM2001664, NT2RM2001670, NT2RM2001700, NT2RM2001730, NT2RM2001 760, NT2RM2001771, NT2RM2001785, NT2RM2001797, NT2RM2001803, NT2RM2001823, NT2RM200 1839, NT2RM2001930, NT2RM2001936, NT2RM2001983, NT2RM2001989, NT2RM2002030, NT2RM20 02100, NT2RM2002109, NT2RM2002128, NT2RM2002142, NT2RM2002145, NT2RM2002580, NT2RM4 000024, NT2RM4000030, NT2RM4000085, NT2RM4000104, NT2RM4000139, NT2RM4000155, NT2RM 4000156, NT2RM4000167, NT2RM4000169, NT2RM4000191, NT2RM4000215, NT2RM4000233, NT2R M4000290, NT2RM4000327, NT2RM4000344, NT2RM4000354, NT2RM4000386, NT2RM4000421, NT2 RM4000433, NT2RM4000471, NT2RM4000496, NT2RM4000531, NT2RM4000616, NT2RM4000689, NT 2RM4000712, NT2RM4000734, NT2RM4000751, NT2RM4000795, NT2RM4000798, NT2RM4000895, N T2RM4000996, NT2RM4001032, NT2RM4001047, NT2RM4001054, NT2RM4001140, NT2RM4001200, NT2RM4001203, NT2RM4001217, NT2RM4001313, NT2RM4001316, NT2RM4001320, NT2RM4001340 , NT2RM4001382, NT2RM4001410, NT2RM4001411, NT2RM4001412, NT2RM4001444, NT2RM400148 3, NT2RM4001566, NT2RM4001582, NT2RM4001592, NT2RM4001605, NT2RM4001611, NT2RM40016 29, NT2RM4001714, NT2RM4001741, NT2RM4001746, NT2RM4001758, NT2RM4001776,
NT2RM4001783, NT2RM4001819, NT2RM4001823, NT2RM4001828, NT2RM4001858, NT2RM4001865, NT2RM4001930, NT2RM4001940, NT2RM4001979, NT2RM4001987, NT2RM4002062, NT2RM400206 3, NT2RM4002073, NT2RM4002093, NT2RM4002109, NT2RM4002145, NT2RM4002146, NT2RM40021 61, NT2RM4002189, NT2RM4002194, NT2RM4002205, NT2RM4002226, NT2RM4002409, NT2RM4002 457, NT2RM4002479, NT2RM4002527, NT2RM4002558, NT2RM4002565, NT2RM4002571, NT2RM400 2594, NT2RM4002623, NT2RP1000018, NT2RP1000035, NT2RP1000086, NT2RP1000111, NT2RP10 00112, NT2RP1000130, NT2RP1000272, NT2RP1000326, NT2RP1000333, NT2RP1000348, NT2RP1 000363, NT2RP1000376, NT2RP1000413, NT2RP1000460, NT2RP1000470, NT2RP1000478, NT2RP 1000493, NT2RP1000547, NT2RP1000574, NT2RP1000581, NT2RP1000609, NT2RP1000629, NT2R P1000701, NT2RP1000721, NT2RP1000733, NT2RP1000738, NT2RP1000782, NT2RP1000825, NT2 RP1000833, NT2RP1000834, NT2RP1000856, NT2RP1000860, NT2RP1000947, NT2RP1000966, NT 2RP1001033, NT2RP1001079, NT2RP1001080, NT2RP1001177, NT2RP1001247, NT2RP1001253, N T2RP1001313, NT2RP1001361, NT2RP1001395, NT2RP1001410, NT2RP1001457, NT2RP1001482, NT2RP1001543, NT2RP1001546, NT2RP1001569, NT2RP2000006, NT2RP2000008, NT2RP2000045, NT2RP2000056, NT2RP2000077, NT2RP2000114, NT2RP2000126, NT2RP2000133, NT2RP200014 7, NT2RP2000161, NT2RP2000183, NT2RP2000233, NT2RP2000257, NT2RP2000297, NT2RP20003 10, NT2RP2000329, NT2RP2000346, NT2RP2000414, NT2RP2000422, NT2RP2000448,
NT2RP2000459, NT2RP2000603, NT2RP2000710, NT2RP2000764, NT2RP2000812, NT2RP2000842, NT2RP2000880, NT2RP2000931, NT2RP2000932, NT2RP2001070, NT2RP2001081, NT2RP200112 7, NT2RP2001174, NT2RP2001233, NT2RP2001290, NT2RP2001327, NT2RP2001397, NT2RP20014 20, NT2RP2001440, NT2RP2001449, NT2RP2001511, NT2RP2001520, NT2RP2001536, NT2RP2001 634, NT2RP2001660, NT2RP2001663, NT2RP2001748, NT2RP2001762, NT2RP2001839, NT2RP200 1876, NT2RP2001898, NT2RP2001900, NT2RP2001991, NT2RP2002025, NT2RP2002058, NT2RP20 02066, NT2RP2002099, NT2RP2002185, NT2RP2002193, NT2RP2002252, NT2RP2002256, NT2RP2 002259, NT2RP2002312, NT2RP2002325, NT2RP2002385, NT2RP2002442, NT2RP2002479, NT2RP 2002503, NT2RP2002504, NT2RP2002591, NT2RP2002595, NT2RP2002606, NT2RP2002618, NT2R P2002710, NT2RP2002727, NT2RP2002928, NT2RP2002959, NT2RP2002980, NT2RP2002993, NT2 RP2003125, NT2RP2003158, NT2RP2003228, NT2RP2003230, NT2RP2003277, NT2RP2003286, NT 2RP2003293, NT2RP2003295, NT2RP2003307, NT2RP2003308, NT2RP2003433, NT2RP2003506, N T2RP2003517, NT2RP2003564, NT2RP2003604, NT2RP2003643, NT2RP2003702, NT2RP2003706, NT2RP2003714, NT2RP2003737, NT2RP2003760, NT2RP2003781, NT2RP2003912, NT2RP2003968, NT2RP2004013, NT2RP2004098, NT2RP2004187, NT2RP2004194, NT2RP2004232, NT2RP200423 9, NT2RP2004316, NT2RP2004523, NT2RP2004538, NT2RP2004568, NT2RP2004655, NT2RP20047 68, NT2RP2004791, NT2RP2004799, NT2RP2004816, NT2RP2004933, NT2RP2004961,
NT2RP2005003, NT2RP2005012, NT2RP2005020, NT2RP2005022, NT2RP2005037, NT2RP2005038 , NT2RP2005116, NT2RP2005126, NT2RP2005144, NT2RP2005168, NT2RP2005204, NT2RP200523 9, NT2RP2005276, NT2RP2005287, NT2RP2005288, NT2RP2005289, NT2RP2005325, NT2RP20053 44, NT2RP2005358, NT2RP2005457, NT2RP2005465, NT2RP2005496, NT2RP2005498, NT2RP2005 520, NT2RP2005531, NT2RP2005539, NT2RP2005557, NT2RP2005620, NT2RP2005675, NT2RP200 5690, NT2RP2005722, NT2RP2005752, NT2RP2005753, NT2RP2005763, NT2RP2005767, NT2RP20 05773, NT2RP2005775, NT2RP2005835, NT2RP2005857, NT2RP2005890, NT2RP2005933, NT2RP2 005942, NT2RP2006219, NT2RP2006238, NT2RP2006275, NT2RP2006464, NT2RP2006565, NT2RP 2006571, NT2RP2006598, NT2RP3000031, NT2RP3000046, NT2RP3000047, NT2RP3000050, NT2R P3000080, NT2RP3000085, NT2RP3000299, NT2RP3000324, NT2RP3000359, NT2RP3000361, NT2 RP3000366, NT2RP3000397, NT2RP3000403, NT2RP3000418, NT2RP3000512, NT2RP3000527, NT 2RP3000590, NT2RP3000603, NT2RP3000632, NT2RP3000739, NT2RP3000742, NT2RP3000759, N T2RP3000845, NT2RP3000868, NT2RP3000875, NT2RP3000917, NT2RP3000919, NT2RP3000968, NT2RP3000994, NT2RP3001057, NT2RP3001081, NT2RP3001120, NT2RP3001140, NT2RP3001155 , NT2RP3001216, NT2RP3001268, NT2RP3001272, NT2RP3001274, NT2RP3001340, NT2RP300135 5, NT2RP3001398, NT2RP3001399, NT2RP3001407, NT2RP3001426, NT2RP3001427, NT2RP30014 28, NT2RP3001495, NT2RP3001497, NT2RP3001554, NT2RP3001587, NT2RP3001671,
NT2RP3001672, NT2RP3001676, NT2RP3001688, NT2RP3001708, NT2RP3001712, NT2RP3001724 , NT2RP3001727, NT2RP3001730, NT2RP3001764, NT2RP3001792, NT2RP3001799, NT2RP300185 5, NT2RP3002004, NT2RP3002045, NT2RP3002056, NT2RP3002151, NT2RP3002165, NT2RP30022 73, NT2RP3002351, NT2RP3002352, NT2RP3002377, NT2RP3002399, NT2RP3002402, NT2RP3002 529, NT2RP3002659, NT2RP3002663, NT2RP3002688, NT2RP3002785, NT2RP3002818, NT2RP300 2876, NT2RP3002909, NT2RP3002969, NT2RP3002972, NT2RP3003059, NT2RP3003061, NT2RP30 03101, NT2RP3003138, NT2RP3003157, NT2RP3003193, NT2RP3003203, NT2RP3003212, NT2RP3 003230, NT2RP3003242, NT2RP3003251, NT2RP3003282, NT2RP3003301, NT2RP3003327, NT2RP 3003346, NT2RP3003385, NT2RP3003411, NT2RP3003464, NT2RP3003500, NT2RP3003589, NT2R P3003672, NT2RP3003701, NT2RP3003716, NT2RP3003800, NT2RP3003825, NT2RP3003831, NT2 RP3003876, NT2RP3003914, NT2RP3003918, NT2RP3004013, NT2RP3004078, NT2RP3004155, NT 2RP3004206, NT2RP3004207, NT2RP3004209, NT2RP3004253, NT2RP3004258, NT2RP3004262, N T2RP3004399, NT2RP3004424, NT2RP3004454, NT2RP3004472, NT2RP3004475, NT2RP3004480, NT2RP3004490, NT2RP3004504, NT2RP3004507, NT2RP3004527, NT2RP3004534, NT2RP3004566, NT2RP3004569, NT2RP3004572, NT2RP3004578, NT2RP3004594, NT2RP3004617, NT2RP300461 8, NT2RP3004669, NT2RP3004670, NT2RP4000008, NT2RP4000049, NT2RP4000078, NT2RP40001 09, NT2RP4000111, NT2RP4000147, NT2RP4000150, NT2RP4000185, NT2RP4000210,
NT2RP4000212, NT2RP4000243, NT2RP4000246, NT2RP4000259, NT2RP4000312, NT2RP4000367 , NT2RP4000370, NT2RP4000376, NT2RP4000398, NT2RP4000417, NT2RP4000449, NT2RP400045 7, NT2RP4000498, NT2RP4000518, NT2RP4000524, NT2RP4000528, NT2RP4000614, NT2RP40007 24, NT2RP4000787, NT2RP4000855, NT2RP4000865, NT2RP4000878, NT2RP4000879, NT2RP4000 907, NT2RP4000915, NT2RP4000925, NT2RP4000928, NT2RP4000997, NT2RP4001006, NT2RP400 1029, NT2RP4001041, NT2RP4001079, NT2RP4001080, NT2RP4001095, NT2RP4001117, NT2RP40 01122, NT2RP4001126, NT2RP4001148, NT2RP4001149, NT2RP4001150, NT2RP4001174, NT2RP4 001207, NT2RP4001213, NT2RP4001219, NT2RP4001313, NT2RP4001315, NT2RP4001345, NT2RP 4001372, NT2RP4001375, NT2RP4001414, NT2RP4001433, NT2RP4001483, NT2RP4001498, NT2R P4001529, NT2RP4001551, NT2RP4001574, NT2RP4001592, NT2RP4001638, NT2RP4001644, NT2 RP4001725, NT2RP4001753, NT2RP4001760, NT2RP4001790, NT2RP4001823, NT2RP4001838, NT 2RP4001849, NT2RP4001893, NT2RP4001927, NT2RP4001938, NT2RP4001946, NT2RP4001966, N T2RP4002047, NT2RP4002078, NT2RP4002408, NT2RP5003477, NT2RP5003500, NT2RP5003522, NT2RP5003524, OVARC1000004, OVARC1000006, OVARC1000014, OVARC1000060, OVARC1000087 , OVARC1000113, OVARC1000114, OVARC1000139, OVARC1000241, OVARC1000304, OVARC100030 9, OVARC1000326, OVARC1000437, OVARC1000440, OVARC1000465, OVARC1000479, OVARC10005 20, OVARC1000543, OVARC1000649, OVARC1000679, OVARC1000682, OVARC1000722,
OVARC1000746, OVARC1000771, OVARC1000834, OVARC1000846, OVARC1000850, OVARC1000862 , OVARC1000876, OVARC1000883, OVARC1000885, OVARC1000912, OVARC1000945, OVARC100099 6, OVARC1001055, OVARC1001068, OVARC1001107, OVARC1001113, OVARC1001154, OVARC10011 80, OVARC1001244, OVARC1001261, OVARC1001296, OVARC1001329, OVARC1001342, OVARC1001 357, OVARC1001372, OVARC1001381, OVARC1001417, OVARC1001419, OVARC1001476, OVARC100 1496, OVARC1001506, OVARC1001542, OVARC1001555, OVARC1001577, OVARC1001610, OVARC10 01668, OVARC1001702, OVARC1001713, OVARC1001731, OVARC1001762, OVARC1001766, OVARC1 001802, OVARC1001809, OVARC1001861, OVARC1001880, OVARC1001900, OVARC1001901, OVARC 1001942, OVARC1001943, OVARC1001949, OVARC1002050, OVARC1002112, OVARC1002138, PLAC E1000007, PLACE1000061, PLACE1000066, PLACE1000133, PLACE1000142, PLACE1000184, PLA CE1000383, PLACE1000406, PLACE1000420, PLACE1000492, PLACE1000547, PLACE1000583, PL ACE1000588, PLACE1000596, PLACE1000611, PLACE1000653, PLACE1000706, PLACE1000863, P LACE1001062, PLACE1001092, PLACE1001118, PLACE1001136, PLACE1001238, PLACE1001294, PLACE1001304, PLACE1001377, PLACE1001384, PLACE1001387, PLACE1001517, PLACE1001602 , PLACE1001603, PLACE1001611, PLACE1001632, PLACE1001634, PLACE1001691, PLACE100169 2, PLACE1001748, PLACE1001771, PLACE1001817, PLACE1001989, PLACE1002046, PLACE10020 90, PLACE1002437, PLACE1002450, PLACE1002474, PLACE1002493, PLACE1002500,
PLACE1002532, PLACE1002571, PLACE1002591, PLACE1002655, PLACE1002665, PLACE1002685 , PLACE1002722, PLACE1002775, PLACE1002782, PLACE1002811, PLACE1002816, PLACE100283 4, PLACE1002991, PLACE1003100, PLACE1003190, PLACE1003258, PLACE1003302, PLACE10033 53, PLACE1003394, PLACE1003420, PLACE1003493, PLACE1003596, PLACE1003605, PLACE1003 669, PLACE1003704, PLACE1003709, PLACE1003738, PLACE1003885, PLACE1003888, PLACE100 3903, PLACE1003915, PLACE1003968, PLACE1004104, PLACE1004128, PLACE1004149, PLACE10 04197, PLACE1004203, PLACE1004277, PLACE1004316, PLACE1004358, PLACE1004428, PLACE1 004437, PLACE1004471, PLACE1004510, PLACE1004564, PLACE1004629, PLACE1004674, PLACE 1004681, PLACE1004743, PLACE1004751, PLACE1004773, PLACE1004777, PLACE1004814, PLAC E1004902, PLACE1004918, PLACE1005101, PLACE1005287, PLACE1005305, PLACE1005477, PLA CE1005494, PLACE1005502, PLACE1005557, PLACE1005611, PLACE1005646, PLACE1005656, FL ACE1005698, PLACE1005739, PLACE1005763, PLACE1005804, PLACE1005813, PLACE1005876, P LACE1005890, PLACE1005968, PLACE1006011, PLACE1006037, PLACE1006040, PLACE1006119, PLACE1006157, PLACE1006170, PLACE1006187, PLACE1006196, PLACE1006288, PLACE1006368 , PLACE1006385, PLACE1006414, PLACE1006438, PLACE1006469, PLACE1006482, PLACE100648 8, PLACE1006531, PLACE1006615, PLACE1006731, PLACE1006754, PLACE1006958, PLACE10069 62, PLACE1007105, PLACE1007238, PLACE1007239, PLACE1007257, PLACE1007346,
PLACE1007375, PLACE1007409, PLACE1007488, PLACE1007511, PLACE1007544, PLACE1007598 , PLACE1007706, PLACE1007729, PLACE1007737, PLACE1007852, PLACE1007955, PLACE100795 8, PLACE1007969, PLACE1008000, PLACE1008044, PLACE1008177, PLACE1008244, PLACE10082 73, PLACE1008275, PLACE1008309, PLACE1008356, PLACE1008402, PLACE1008603, PLACE1008 627, PLACE1008643, PLACE1008650, PLACE1008696, PLACE1008790, PLACE1008808, PLACE100 8813, PLACE1008941, PLACE1008947, PLACE1009027, PLACE1009060, PLACE1009094, PLACE10 09099, PLACE1009113, PLACE1009200, PLACE1009230, PLACE1009246, PLACE1009298, PLACE1 009319, PLACE1009398, PLACE1009444, PLACE1009468, PLACE1009524, PLACE1009622, PLACE 1009659, PLACE1009670, PLACE1009721, PLACE1009763, PLACE1009845, PLACE1009861, PLAC E1009997, PLACE1010053, PLACE1010074, PLACE1010083, PLACE1010089, PLACE1010096, PLA CE1010106, PLACE1010152, PLACE1010194, PLACE1010261, PLACE1010310, PLACE1010481, PL ACE1010491, PLACE1010580, PLACE1010599, PLACE1010702, PLACE1010720, PLACE1010743, P LACE1010761, PLACE1010771, PLACE1010833, PLACE1010856, PLACE1010870, PLACE1010942, PLACE1010960, PLACE1011041, PLACE1011046, PLACE1011054, PLACE1011056, PLACE1011057 , PLACE1011109, PLACE1011165, PLACE1011203, PLACE1011219, PLACE1011229, PLACE101126 3, PLACE1011332, PLACE1011371, PLACE1011477, PLACE1011492, PLACE1011576, PLACE10116 64, PLACE1011896, PLACE1011923, PLACE2000021, PLACE2000034, PLACE2000039,
PLACE2000062, PLACE2000072, PLACE2000140, PLACE2000164, PLACE2000216, PLACE2000274 , PLACE2000341, PLACE2000359, PLACE2000399, PLACE2000404, PLACE2000411, PLACE200043 8, PLACE2000458, PLACE3000004, PLACE3000009, PLACE3000020, PLACE3000059, PLACE30001 21, PLACE3000145, PLACE3000148, PLACE3000156, PLACE3000242, PLACE3000244, PLACE3000 322, PLACE3000350, PLACE3000352, PLACE3000353, PLACE3000373, PLACE3000455, PLACE300 0475, PLACE4000009, PLACE4000014, PLACE4000052, PLACE4000128, PLACE4000156, PLACE40 00230, PLACE4000259, PLACE4000261, PLACE4000269, PLACE4000320, PLACE4000431, PLACE4 000522, PLACE4000558, PLACE4000581, PLACE4000590, PLACE4000612, PLACE4000654, SKNMC 1000011, SKNMC1000050, THYRO1000017, THYRO1000026, THYRO1000072, THYRO1000121, THYR 01000173, THYRO1000186, THYRO1000197, THYRO1000242, THYRO1000288, THYRO1000320, THY R01000327, THYRO1000358, THYRO1000387, THYRO1000471, THYRO1000501, THYRO1000585, TH YR01000666, THYRO1000783, THYRO1000852, THYRO1000926, THYRO1000934, THYRO1000951, T HYR01000974, THYRO1001100, THYRO1001121, THYRO1001189, THYRO1001406, THYRO1001458, THYRO1001595, THYRO1001605, THYRO1001617, THYRO1001671, Y79AA1000037, Y79AA1000059 , Y79AA1000214, Y79AA1000258, Y79AA1000346, Y79AA1000349, Y79AA1000368, Y79AA100046 9, Y79AA1000539, Y79AA1000560, Y79AA1000589, Y79AA1000627, Y79AA1000705, Y79AA10007 34, Y79AA1000752, Y79AA1000784, Y79AA1000833, Y79AA1000962, Y79AA1000966,
Y79AA1000968, Y79AA1000985, Y79AA1001048, Y79AA1001105, Y79AA1001233, Y79AA1001236 , Y79AA1001299, Y79AA1001391, Y79AA1001394, Y79AA1001533, Y79AA1001548, Y79AA100158 1, Y79AA1001613, Y79AA1001679, Y79AA1001692, Y79AA1001827, Y79AA1001848, Y79AA10018 66, Y79AA1001874, Y79AA1001963, Y79AA1002027, Y79AA1002093, Y79AA1002139, Y79AA1002 208, Y79AA1002209, Y79AA1002258, Y79AA1002361, Y79AA1002416, Y79AA1002472, Y79AA100 2482,

Among the clones, 1001 clones were estimated to have a relatively high homology with the known proteins or genes in the same category.
HEMBA1000005, HEMBA1000020, HEMBA1000030, HEMBA1000158, HEMBA1000201, HEMBA1000216, HEMBA1000303, HEMBA1000459, HEMBA1000523, HEMBA1000531, HEMBA1000542, HEMBA1000561, HEMBA1000588, HEMBA1000591, HEMBA1000657, HEMBA1000673, HEMBA1000827, HEMBA1000851, HEMBA1000852, HEMBA1000972, HEMBA1000991, HEMBA1001017, HEMBA1001019, HEMBA1001059, HEMBA1001071, HEMBA1001088, HEMBA1001123, HEMBA1001174, HEMBA1001257, HEMBA1001302, HEMBA1001351, HEMBA1001387, HEMBA1001510, HEMBA1001569, HEMBA1001570, HEMBA1001579, HEMBA1001595, HEMBA1001620, HEMBA1001672, HEMBA1001678, HEMBA1001714, HEMBA1001819, HEMBA1001822, HEMBA1001896, HEMBA1001913, HEMBA1001921, HEMBA1002003, HEMBA1002092, HEMBA1002160, HEMBA1002161, HEMBA1002162, HEMBA1002229, HEMBA1002257, HEMBA1002341, HEMBA1002363, HEMBA1002417, HEMBA1002513, HEMBA1002547, HEMBA1002569, HEMBA1002716, HEMBA1002810, HEMBA1002896, HEMBA1002973, HEMBA1002999, HEMBA1003046, HEMBA1003148, HEMBA1003286, HEMBA1003314, HEMBA1003433, HEMBA1003538, HEMBA1003545, HEMBA1003555, HEMBA1003560, HEMBA1003569, HEMBA1003581, HEMBA1003615, HEMBA1003621, HEMBA1003690, HEMBA1003720, HEMBA1003760, HEMBA1003773, HEMBA1003783, HEMBA1003805, HEMBA1003866, HEMBA1004097, HEMBA1004131, HEMBA1004168, HEMBA1004202, HEMBA1004207, HEMBA1004227, HEMBA1004248, HEMBA1004286, HEMBA1004321, HEMBA1004353, HEMBA1004354, HEMBA1004356, HEMBA1004389, HEMBA1004479, HEMBA1004534, HEMBA1004604,
HEMBA1004752, HEMBA1004753, HEMBA1004756, HEMBA1004758, HEMBA1004795, HEMBA1004807, HEMBA1004847, HEMBA1005009, HEMBA1005047, HEMBA1005202, HEMBA1005241, HEMBA1005293, HEMBA1005331, HEMBA1005338, HEMBA1005359, HEMBA1005367, HEMBA1005423, HEMBA1005443, HEMBA1005513, HEMBA1005528, HEMBA1005548, HEMBA1005576, HEMBA1005581, HEMBA1005679, HEMBA1005699, HEMBA1005732, HEMBA1005963, HEMBA1005990, HEMBA1006173, HEMBA1006253, HEMBA1006272, HEMBA1006278, HEMBA1006310, HEMBA1006344, HEMBA1006359, HEMBA1006540, HEMBA1006559, HEMBA1006639, HEMBA1006648, HEMBA1006807, HEMBA1006877, HEMBA1006941, HEMBA1006973, HEMBA1006976, HEMBA1007018, HEMBA1007078, HEMBA1007174, HEMBA1007243, HEMBB1000037, HEMBB1000250, HEMBB1000264, HEMBB1000399, HEMBB1000404, HEMBB1000530, HEMBB1000637, HEMBB1000684, HEMBB1000693, HEMBB1000725, HEMBB1000781, HEMBB1000915, HEMBB1000927, HEMBB1001051, HEMBB1001068, HEMBB1001102, HEMBB1001112, HEMBB1001119, HEMBB1001133, HEMBB1001151, HEMBB1001175, HEMBB1001242, HEMBB1001294, HEMBB1001314, HEMBB1001331, HEMBB1001346, HEMBB1001384, HEMBB1001429, HEMBB1001443, HEMBB1001482, HEMBB1001802, HEMBB1001831, HEMBB1001908, HEMBB1001915, HEMBB1001922, HEMBB1001967, HEMBB1002042, HEMBB1002134, HEMBB1002193, HEMBB1002217, HEMBB1002342, HEMBB1002358, HEMBB1002442, HEMBB1002477, HEMBB1002600, HEMBB1002635, HEMBB1002683, MAMMA1000009, MAMMA1000020, MAMMA1000092, MAMMA1000103, MAMMA1000155,
MAMMA1000173, MAMMA1000183, MAMMA1000388, MAMMA1000424, MAMMA1000429, MAMMA1000731, MAMMA1000734, MAMMA1000956, MAMMA1001021, MAMMA1001059, MAMMA1001075, MAMMA1001080, MAMMA1001105, MAMMA1001139, MAMMA1001198, MAMMA1001305, MAMMA1001388, MAMMA1001476, MAMMA1001501, MAMMA1001576, MAMMA1001633, MAMMA1001735, MAMMA1001751, MAMM1001754, MAMMA1001771, MAMMA1001820, MAMMA1001837, MAMMA1002140, MAMMA1002170, MAMM1002198, MAMMA1002219, MAMMA1002236, MAYM1002268, MAMMA1002352, MAMMA1002392, MAMMA1002485, MAMMA1002530, MAMMA1002598, MAMMA1002622, MAMMA1002637, MAMMA1002699, MAMMA1002842, MAMMA1002858, MAMMA1002869, MAMMA1002938, MAMMA1003011, MAMMA1003047, MAMMA1003057, MAMMA1003099, MAMMA1003104, MAMMA1003113, MAMMA1003127, MAMMA1003146, MAMMA1003166, NT2RM1000035, NT2RM1000055, NT2RM1000132, NT2RM1000199, NT2RM1000256, NT2RM1000257, NT2RM1000280, NT2RM1000318, NT2RM1000355, NT2RM1000394, NT2RM1000430, NT2RM1000555, NT2RM1000661, NT2RM1000725, NT2RM1000742, NT2RM1000811, NT2RM1000826, NT2RM1000833, NT2RM1000852, NT2RM1000867, NT2RM1000883, NT2RM1000894, NT2RM1001003, NT2RM1001082, NT2RM1001092, NT2RM2000013, NT2RM2000124, NT2RM2000191, NT2RM2000368, NT2RM2000374, NT2RM2000407, NT2RM2000422, NT2RM2000504, NT2RM2000566, NT2RM2000594, NT2RM2000612, NT2RM2000691, NT2RM2000735, NT2RM2000740, NT2RM2000821, NT2RM2000951, NT2RM2001035, NT2RM2001065, NT2RM2001238, NT2RM2001256, NT2RM2001424,
NT2RM2001499, NT2RM2001575, NT2RM2001592, NT2RM2001605, NT2RM2001613, NT2RM2001648, NT2RM2001652, NT2RM2001664, NT2RM2001760, NT2RM2001771, NT2RM2001803, NT2RM2001839, NT2RM2001930, NT2RM2001983, NT2RM2002030, NT2RM2002100, NT2RM2002109, NT2RM2002128, NT2RM2002142, NT2RM2002145, NT2RM4000024, NT2RM4000104, NT2RM4000139, NT2RM4000167, NT2RM4000233, NT2RM4000290, NT2RM4000327, NT2RM4000344, NT2RM4000386, NT2RM4000433, NT2RM4000471, NT2RM4000531, NT2RM4000616, NT2RM4000689, NT2RM4000712, NT2RM4000795, NT2RM4000798, NT2RM4000895, NT2RM4000996, NT2RM4001047, NT2RM4001054, NT2RM4001200, NT2RM4001203, NT2RM4001217, NT2RM4001313, NT2RM4001320, NT2RM4001382, NT2RM4001411, NT2RM4001412, NT2RM4001483, NT2RM4001566, NT2RM4001582, NT2RM4001592, NT2RM4001605, NT2RM4001714, NT2RM4001741, NT2RM4001758, NT2RM4001819, NT2RM4001823, NT2RM4001828, NT2RM4001858, NT2RM4001865, NT2RM4001940, NT2RM4002063, NT2RM4002073, NT2RM4002093, NT2RM4002109, NT2RM4002146, NT2RM4002161, NT2RM4002194, NT2RM4002205, NT2RM4002457, NT2RM4002479, NT2RM4002558, NT2RM4002565, NT2RM4002594, NT2RP1000018, NT2RP1000035, NT2RP1000086, NT2RP1000112, NT2RP1000272, NT2RP1000326, NT2RP1000376, NT2RP1000413, NT2RP1000478, NT2RP1000574, NT2RP1000581, NT2RP1000629, NT2RP1000701, NT2RP1000721, NT2RP1000733, NT2RP1000738, NT2RP1000782, NT2RP1000825, NT2RP1000833, NT2RP1000834, NT2RP1000856, NT2RP1000860, NT2RP1000947, NT2RP1000966,
NT2RP1001079, NT2RP1001177, NT2RP1001247, NT2RP1001253, NT2RP1001361, NT2RP1001395, NT2RP1001410, NT2RP1001457, NT2RP1001482, NT2RP1001546, NT2RP1001569, NT2RP2000008, NT2RP2000045, NT2RP2000056, NT2RP2000077, NT2RP2000114, NT2RP2000126, NT2RP2000147, NT2RP2000233, NT2RP2000297, NT2RP2000310, NT2RP2000329, NT2RP2000346, NT2RP2000414, NT2RP2000422, NT2RP2000459, NT2RP2000603, NT2RP2000842, NT2RP2000880, NT2RP2000931, NT2RP2001070, NT2RP2001081, NT2RP2001233, NT2RP2001290, NT2RP2001327, NT2RP2001397, NT2RP2001420, NT2RP2001440, NT2RP2001449, NT2RP2001511, NT2RP2001520, NT2RP2001536, NT2RP2001634, NT2RP2001660, NT2RP2001663, NT2RP2001748, NT2RP2001762, NT2RP2001876, NT2RP2001898, NT2RP2001991, NT2RP2002066, NT2RP2002099, NT2RP2002193, NT2RP2002252, NT2RP2002256, NT2RP2002259, NT2RP2002312, NT2RP2002325, NT2RP2002385, NT2RP2002479, NT2RP2002503, NT2RP2002504, NT2RP2002591, NT2RP2002606, NT2RP2002727, NT2RP2002928, NT2RP2002959, NT2RP2002993, NT2RP2003158, NT2RP2003228, NT2RP2003230, NT2RP2003295, NT2RP2003307, NT2RP2003308, NT2RP2003433, NT2RP2003517, NT2RP2003604, NT2RP2003643, NT2RP2003702, NT2RP2003714, NT2RP2003737, NT2RP2003760, NT2RP2003781, NT2RP2003912, NT2RP2003968, NT2RP2004013, NT2RP2004194, NT2RP2004232, NT2RP2004239, NT2RP2004316, NT2RP2004523, NT2RP2004538, NT2RP2004655, NT2RP2004768, NT2RP2004799, NT2RP2004816, NT2RP2004933, NT2RP2004961, NT2RP2005003, NT2RP2005012,
NT2RP2005020, NT2RP2005116, NT2RP2005126, NT2RP2005144, NT2RP2005168, NT2RP2005239, NT2RP2005276, NT2RP2005287, NT2RP2005288, NT2RP2005289, NT2RP2005325, NT2RP2005358, NT2RP2005457, NT2RP2005498, NT2RP2005520, NT2RP2005539, NT2RP2005557, NT2RP2005620, NT2RP2005675, NT2RP2005722, NT2RP2005752, NT2RP2005753, NT2RP2005773, NT2RP2005775, NT2RP2005835, NT2RP2005857, NT2RP2005890, NT2RP2005933, NT2RP2005942, NT2RP2006219, NT2RP2006238, NT2RP2006464, NT2RP2006565, NT2RP2006571, NT2RP2006598, NT2RP3000031, NT2RP3000050, NT2RP3000080, NT2RP3000085, NT2RP3000299, NT2RP3000324, NT2RP3000359, NT2RP3000366, NT2RP3000403, NT2RP3000512, NT2RP3000632, NT2RP3000739, NT2RP3000742, NT2RP3000845, NT2RP3000875, NT2RP3000917, NT2RP3000919, NT2RP3000968, NT2RP3001057, NT2RP3001081, NT2RP3001120, NT2RP3001140, NT2RP3001155, NT2RP3001268, NT2RP3001272, NT2RP3001340, NT2RP3001398, NT2RP3001428, NT2RP3001495, NT2RP3001497, NT2RP3001554, NT2RP3001671, NT2RP3001672, NT2RP3001676, NT2RP3001688, NT2RP3001712, NT2RP3001724, NT2RP3001727, NT2RP3001730, NT2RP3001764, NT2RP3001792, NT2RP3001799, NT2RP3001855, NT2RP3002004, NT2RP3002045, NT2RP3002151, NT2RP3002165, NT2RP3002351, NT2RP3002352, NT2RP3002402, NT2RP3002529, NT2RP3002659, NT2RP3002688, NT2RP3002818, NT2RP3002876, NT2RP3002969, NT2RP3002972, NT2RP3003059, NT2RP3003101, NT2RP3003138, NT2RP3003157, NT2RP3003193, NT2RP3003203, NT2RP3003212, NT2RP3003230,
NT2RP3003242, NT2RP3003251, NT2RP3003282, NT2RP3003301, NT2RP3003327, NT2RP3003346, NT2RP3003385, NT2RP3003411, NT2RP3003464, NT2RP3003589, NT2RP3003800, NT2RP3003876, NT2RP3003914, NT2RP3003918, NT2RP3004013, NT2RP3004078, NT2RP3004155, NT2RP3004206, NT2RP3004207, NT2RP3004258, NT2RP3004262, NT2RP3004399, NT2RP3004424, NT2RP3004480, NT2RP3004490, NT2RP3004504, NT2RP3004527, NT2RP3004534, NT2RP3004566, NT2RP3004572, NT2RP3004594, NT2RP3004618, NT2RP3004670, NT2RP4000008, NT2RP4000049, NT2RP4000078, NT2RP4000109, NT2RP4000111, NT2RP4000147, NT2RP4000150, NT2RP4000185, NT2RP4000243, NT2RP4000246, NT2RP4000367, NT2RP4000376, NT2RP4000398, NT2RP4000518, NT2RP4000524, NT2RP4000614, NT2RP4000724, NT2RP4000787, NT2RP4000855, NT2RP4000865, NT2RP4000878, NT2RP4000907, NT2RP4000915, NT2RP4000928, NT2RP4000997, NT2RP4001006, NT2RP4001029, NT2RP4001079, NT2RP4001080, NT2RP4001117, NT2RP4001213, NT2RP4001313, NT2RP4001315, NT2RP4001345, NT2RP4001414, NT2RP4001433, NT2RP4001483, NT2RP4001498, NT2RP4001529, NT2RP4001574, NT2RP4001644, NT2RP4001753, NT2RP4001760, NT2RP4001938, NT2RP4001966, NT2RP4002047, NT2RP4002078, NT2RP5003500, OVARC1000006, OVARC1000014, OVARC1000060, OVARC1000113, OVARC1000114, OVARC1000241, OVARC1000304, OVARC1000326, OVARC1000437, OVARC1000440, OVARC1000465, OVARC1000479, OVARC1000520, OVARC1000649, OVARC1000679, OVARC1000682, OVARC1000722, OVARC1000771, OVARC1000834,
OVARC1000850, OVARC1000862, OVARC1000883, OVARC1000945, OVARC1000996, OVARC1001055, OVARC1001068, OVARC1001107, OVARC1001113, OVARC1001154, OVARC1001244, OVARC1001261, OVARC1001296, OVARC1001342, OVARC1001357, OVARC1001372, OVARC1001381, OVARC1001417, OVARC1001419, OVARC1001496, OVARC1001506, OVARC1001542, OVARC1001577, OVARC1001668, OVARC1001702, OVARC1001713, OVARC1001731, OVARC1001766, OVARC1001802, OVARC1001809, OVARC1001861, OVARC1001900, OVARC1001901, OVARC1001943, OVARC1001949, OVARC1002112, PLACE1000007, PLACE1000061, PLACE1000133, PLACE1000184, PLACE1000383, PLACE1000406, PLACE1000492, PLACE1000588, PLACE1000596, PLACE1000611, PLACE1000653, PLACE1000706, PLACE1001062, PLACE1001092, PLACE1001118, PLACE1001136, PLACE1001238, PLACE1001294, PLACE1001304, PLACE1001377, PLACE1001384, PLACE1001517, PLACE1001602, PLACE1001603, PLACE1001611, PLACE1001634, PLACE1001691, PLACE1001748, PLACE1001771, PLACE1001817, PLACE1002046, PLACE1002090, PLACE1002437, PLACE1002474, PLACE1002493, PLACE1002500, PLACE1002532, PLACE1002591, PLACE1002655, PLACE1002665, PLACE1002685, PLACE1002782, PLACE1002816, PLACE1002834, PLACE1003100, PLACE1003302, PLACE1003353, PLACE1003394, PLACE1003596, PLACE1003709, PLACE1003738, PLACE1003885, PLACE1003888, PLACE1003903, PLACE1003968, PLACE1004104, PLACE1004128, PLACE1004149, PLACE1004203, PLACE1004277, PLACE1004316, PLACE1004358, PLACE1004437, PLACE1004510,
PLACE1004564, PLACE1004674, PLACE1004681, PLACE1004743, PLACE1004773, PLACE1004777, PLACE1004814, PLACE1004918, PLACE1005101, PLACE1005305, PLACE1005477, PLACE1005494, PLACE1005502, PLACE1005646, PLACE1005656, PLACE1005698, PLACE1005739, PLACE1005804, PLACE1005813, PLACE1005876, PLACE1005968, PLACE1006011, PLACE1006037, PLACE1006040, PLACE1006119, PLACE1006170, PLACE1006187, PLACE1006196, PLACE1006288, PLACE1006368, PLACE1006385, PLACE1006414, PLACE1006438, PLACE1006482, PLACE1006488, PLACE1006531, PLACE1006615, PLACE1006754, PLACE1006958, PLACE1006962, PLACE1007105, PLACE1007239, PLACE1007257, PLACE1007346, PLACE1007409, PLACE1007511, PLACE1007706, PLACE1007737, PLACE1007852, PLACE1007955, PLACE1007958, PLACE1007969, PLACE1008000, PLACE1008044, PLACE1008177, PLACE1008273, PLACE1008309, PLACE1008356, PLACE1008402, PLACE1008603, PLACE1008627, PLACE1008643, PLACE1008650, PLACE1008696, PLACE1008790, PLACE1008808, PLACE1008813, PLACE1008941, PLACE1009027, PLACE1009060, PLACE1009099, PLACE1009113, PLACE1009200, PLACE1009230, PLACE1009246, PLACE1009298, PLACE1009398, PLACE1009444, PLACE1009468, PLACE1009524, PLACE1009659, PLACE1009670, PLACE1009763, PLACE1009997, PLACE1010053, PLACE1010074, PLACE1010096, PLACE1010261, PLACE1010481, PLACE1010491, PLACE1010580, PLACE1010599, PLACE1010702, PLACE1010720, PLACE1010743, PLACE1010761, PLACE1010771, PLACE1010856, PLACE1010870, PLACE1010942,
PLACE1011041, PLACE1011046, PLACE1011054, PLACE1011057, PLACE1011109, PLACE1011165, PLACE1011203, PLACE1011229, PLACE1011332, PLACE1011477, PLACE1011576, PLACE1011664, PLACE1011896, PLACE1011923, PLACE2000021, PLACE2000039, PLACE2000062, PLACE2000072, PLACE2000140, PLACE2000216, PLACE2000341, PLACE2000359, PLACE2000404, PLACE2000438, PLACE3000004, PLACE3000009, PLACE3000020, PLACE3000059, PLACE3000121, PLACE3000145, PLACE3000244, PLACE3000350, PLACE3000352, PLACE3000455, PLACE3000475, PLACE4000052, PLACE4000128, PLACE4000156, PLACE4000230, PLACE4000261, PLACE4000269, PLACE4000320, PLACE4000654, SKNMC1000050, THYRO1000017, THYRO1000026, THYRO1000121, THYRO1000173, THYRO1000186, THYRO1000197, THYRO1000242, THYRO1000288, THYRO1000320, THYRO1000327, THYRO1000358, THYRO1000387, THYRO1000471, THYRO1000501, THYRO1000585, THYRO1000666, THYRO1000783, THYRO1000926, THYRO1000974, THYRO1001100, THYRO1001121, THYRO1001189, THYRO1001406, THYRO1001458, THYRO1001595, THYRO1001605, THYRO1001617, THYRO1001671, Y79AA1000037, Y79AA1000059, Y79AA1000214, Y79AA1000346, Y79AA1000349, Y79AA1000469, Y79AA1000560, Y79AA1000627, Y79AA1000705, Y79AA1000734, Y79AA1000752, Y79AA1000784, Y79AA1000833, Y79AA1000966, Y79AA1000968, Y79AA1000985, Y79AA1001105, Y79AA1001236, Y79AA1001299, Y79AA1001391, Y79AA1001533, Y79AA1001548, Y79AA1001679, Y79AA1001827, Y79AA1001866, Y79AA1002093, Y79AA1002258, Y79AA1002361, Y79AA1002416,

The following 429 clones were estimated to have a relatively low homology with the known proteins in the same category.

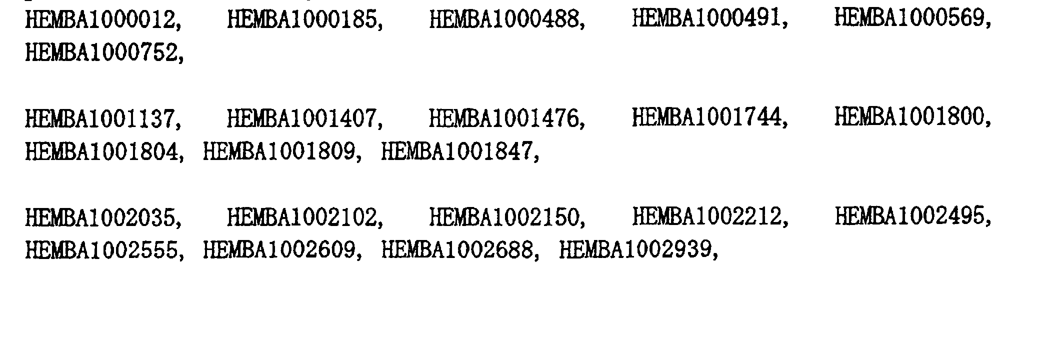

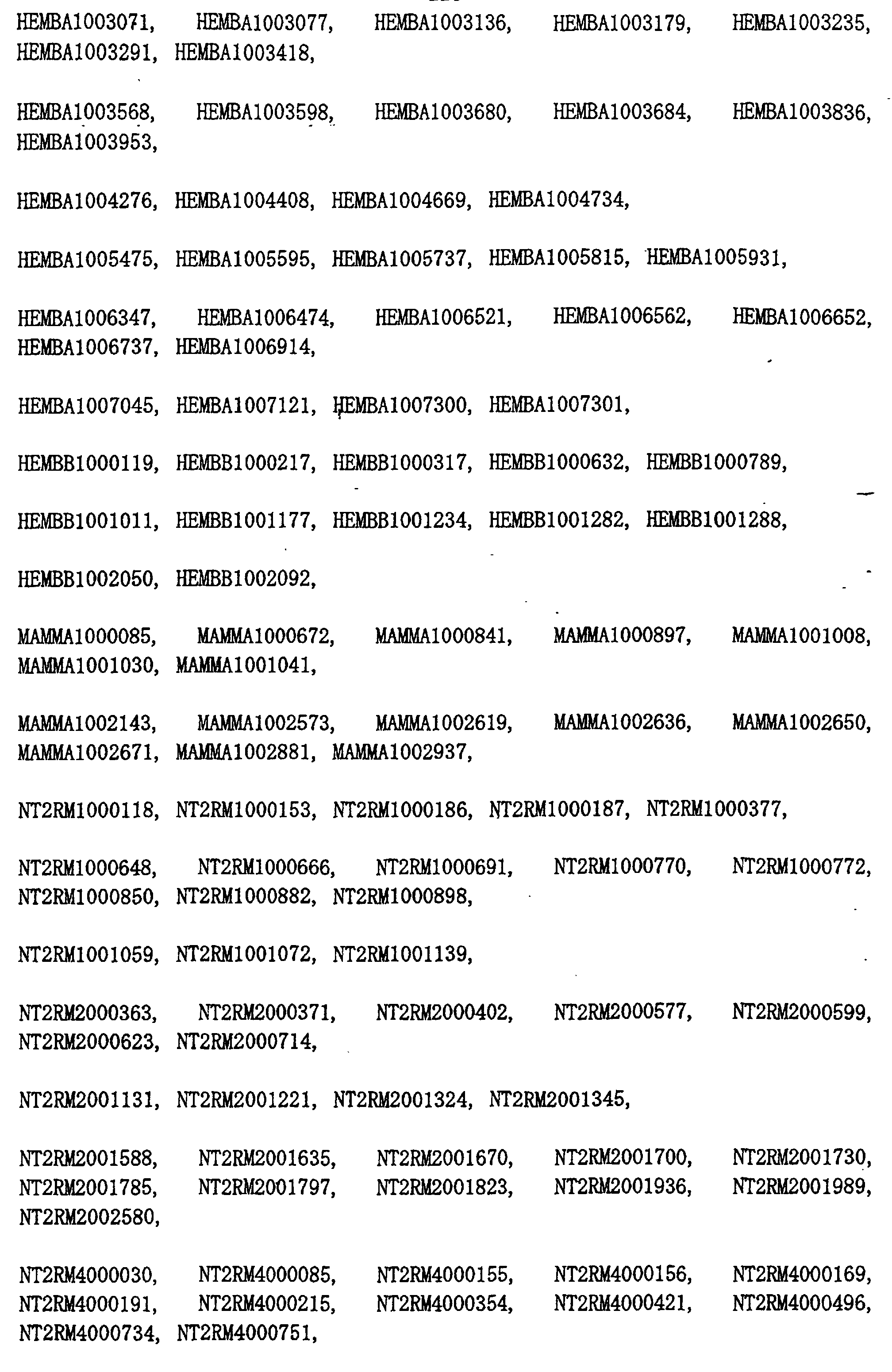

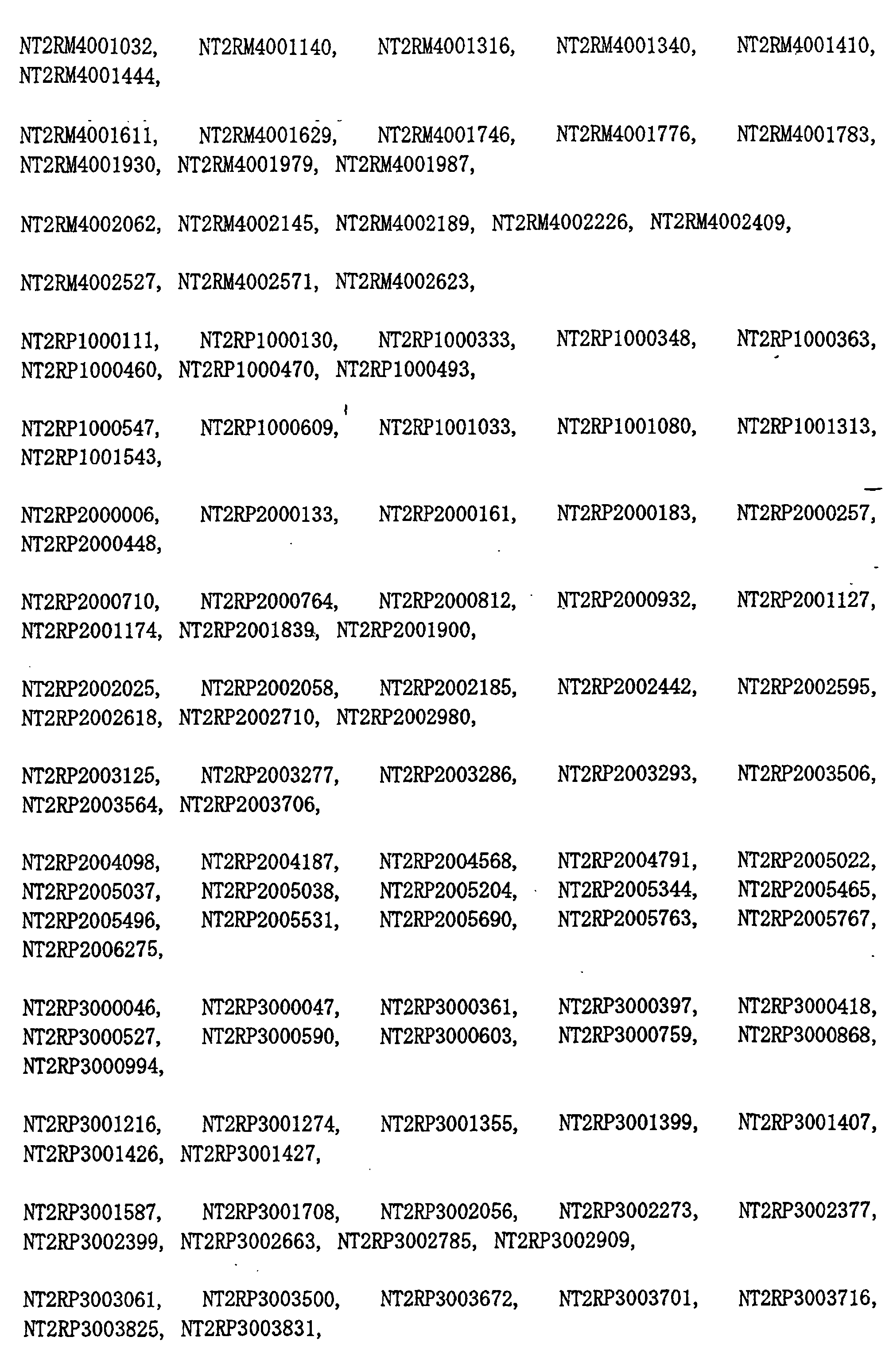

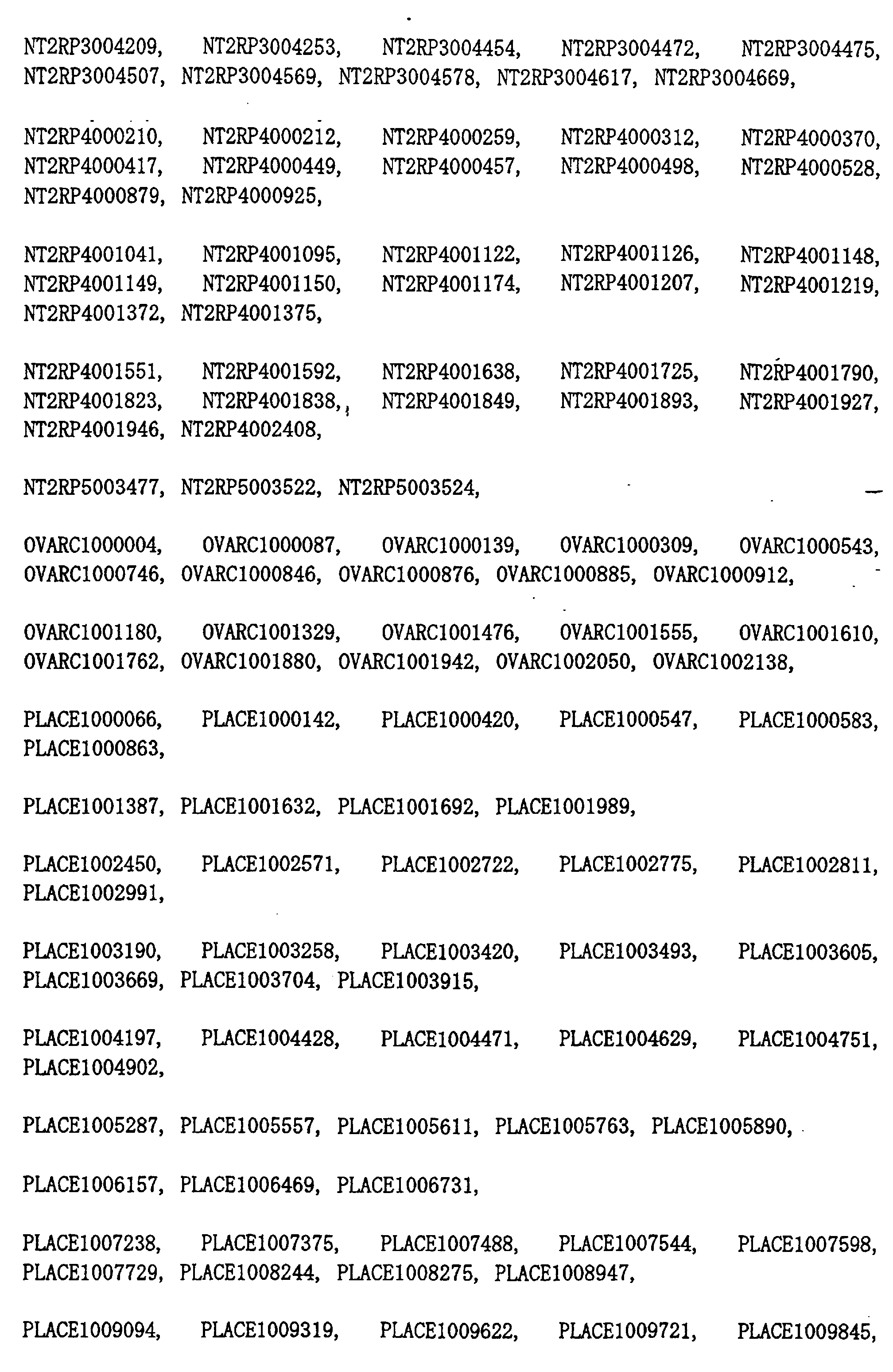

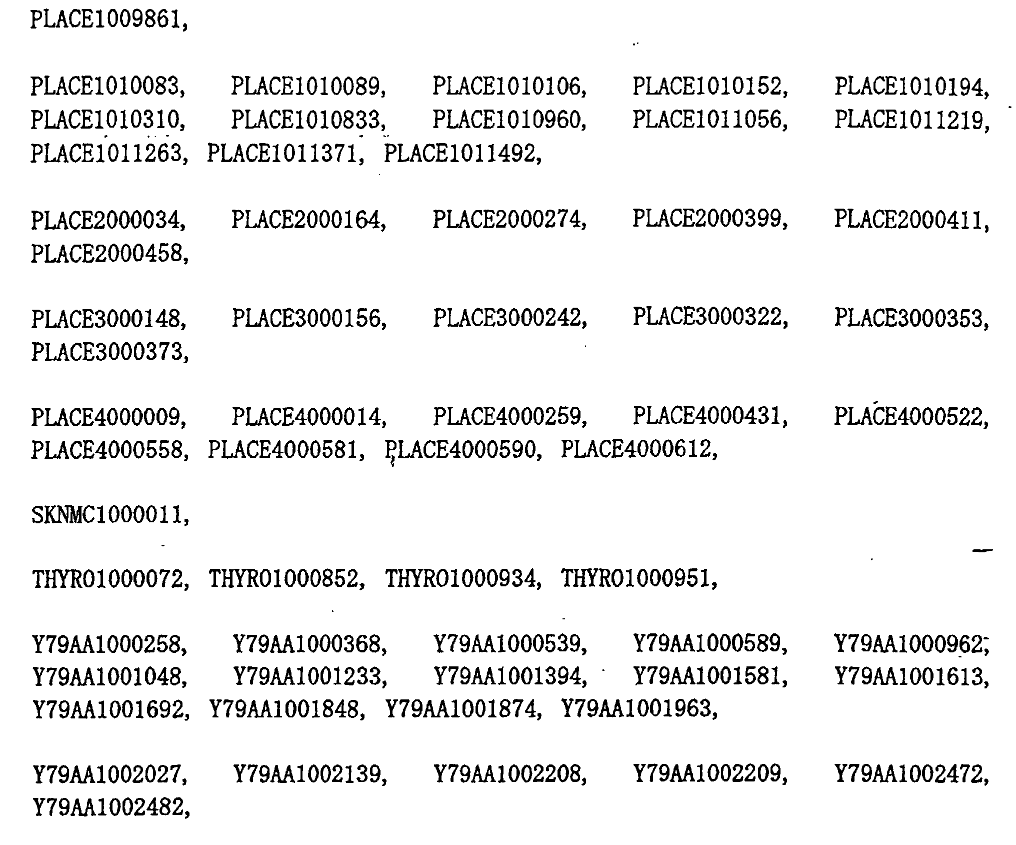

(1) Among the 1430 clones, the following 259 clones were predicted to encode a protein in the category of secretory protein or membrane protein (including the clones belonging to plural categories).
   HEMBA1000005, HEMBA1001017, HEMBA1001059, HEMBA1001071, HEMBA1001351, HEMBA1001407 , HEMBA1001476, HEMBA1001569, HEMBA1001678, HEMBA1001804, HEMBA1002150, HEMBA100254 7, HEMBA1002555, HEMBA1002688, HEMBA1002716, HEMBA1002999, HEMBA1003071, HEMBA10030 77, HEMBA1003538, HEMBA1003598, HEMBA1003690, HEMBA1004207, HEMBA1004356, HEMBA1004 389, HEMBA1004756, HEMBA1005338, HEMBA1005367, HEMBA1005576, HEMBA1005699, HEMBA100 5963, HEMBA1005990, HEMBA1006310, HEMBA1006344, HEMBA1006562, HEMBA1006976, HEMBA10 07045, HEMBA1007301, HEMBB1000037, HEMBB1000119, HEMBB1000317, HEMBB1000530, HEMBB1 000915, HEMBB1001112, HEMBB1001119, HEMBB1001151, HEMBB1001282, HEMBB1001331, HEMBB 1002042, HEMBB1002092, HEMBB1002193, HEMBB1002600, MAMMA1000103, MAMMA1000424, MAMM A1000672, MAMMA1000734, MAMMA1000897, MAMMA1001008, MAMMA1001030, MAMMA1001041, MAM MA1001075, MAMMA1001080, MAMMA1001751, MAMMA1001754, MAMMA1002143, MAMMA1002219, MA MMA1002485, MAMMA1002636, MAMMA1002938, MAMMA1003104, NT2RM1000199, NT2RM1000355, N T2RM1000430, NT2RM1000648, NT2RM1000742, NT2RM1000770, NT2RM1000811, NT2RM1000833, NT2RM1000867, NT2RM1000882, NT2RM1000883, NT2RM1001139, NT2RM2000374, NT2RM2000402 , NT2RM2000422, NT2RM2000566, NT2RM2000623, NT2RR2001324, NT2RM2001499, NT2RM200158 8, NT2RM2001592, NT2RM2001613, NT2RM2001635, NT2RM2001648, NT2RM2001760, NT2RM20021 09, NT2RM2002145, NT2RM4000156, NT2RM4000169, NT2RM4000386, NT2RM4000433, NT2RM4000795, NT2RM4001032, NT2RM4001054, NT2RM4001410, NT2RM4001605; NT2RM4001930, NT2RM4001987, NT2RM4002073, NT2RM4002145, NT2RM4002189, NT2RM4002558, NT2RM400256 5, NT2RM4002571, NT2RM4002594, NT2RP1000130, NT2RP1000326, NT2RP1000363, NT2RP10004 13, NT2RP1000547, NT2RP1000782, NT2RP1000856, NT2RP1001247, NT2RP1001313, NT2RP1001 546, NT2RP1001569, NT2RP2000056, NT2RP2000257, NT2RP2000842, NT2RP2001081, NT2RP200 1991, NT2RP2002025, NT2RP2002193, NT2RP2002256, NT2RP2002312, NT2RP2002479, NT2RP20 02504, NT2RP2003433, NT2RP2003706, NT2RP2003760, NT2RP2004194, NT2RP2004655, NT2RP2 005020, NT2RP2005344, NT2RP2005465, NT2RP2005752, NT2RP2005753, NT2RP2005835, NT2RP 2005933, NT2RP2006275, NT2RP2006565, NT2RP2006571, NT2RP3000324, NT2RP3000739, NT2R P3000919, NT2RP3001140, NT2RP3001355, NT2RP3001676, NT2RP3001708, NT2RP3001727, NT2 RP3002659, NT2RP3003059, NT2RP3003101, NT2RP3003203, NT2RP3003212, NT2RP3003242, NT 2RP3003672, NT2RP3003701, NT2RP3003716, NT2RP3003918, NT2RP3004207, NT2RP3004253, N T2RP4000008, NT2RP4000212, NT2RP4000243, NT2RP4000417, NT2RP4000878, NT2RP4000907, NT2RP4000925, NT2RP4000928, NT2RP4001079, NT2RP4001117, NT2RP4001150, NT2RP4001174 , NT2RP4001313, NT2RP4001315, NT2RP4001345, NT2RP4001372, NT2RP4001574, NT2RP400182 3, NT2RP4001966, NT2RP5003524, OVARC1000060, OVARC1000326, OVARC1000543, OVARC10006 82, OVARC1000722, OVARC1000912, OVARC1001055, OVARC1001154, OVARC1001261,
   OVARC1001329, OVARC1001419, OVARC1001506, OVARC1001542, OVARC1001610, OVARC1001713 , OVARC1001802, OVARC1001943, PLACE1000611, PLACE1001092, PLACE1001136, PLACE100129 4, PLACE1002437, PLACE1002500, PLACE1002722, PLACE1002782, PLACE1002816, PLACE10034 20, PLACE1003493, PLACE1003596, PLACE1004104, PLACE1004197, PLACE1004203, PLACE1004 277, PLACE1004629, PLACE1004751, PLACE1005494, PLACE1005804, PLACE1005890, PLACE100 6157, PLACE1007409, PLACE1007852, PLACE1008044, PLACE1008273, PLACE1008309, PLACE10 08603, PLACE1008643, PLACE1009659, PLACE1009670, PLACE1009845, PLACE1009861, PLACE1 011371, PLACE1011492, PLACE1011896, PLACE2000034, PLACE2000341, PLACE2000399, PLACE 2000458, PLACE3000020, PLACE3000322, PLACE3000353, PLACE4000052, PLACE4000269, PLAC E4000522, PLACE4000581, THYRO1000783, Y79AA1000258, Y79AA1000346, Y79AA1001874,
(2) The following 111 clones were predicted to encode a protein in the category of glycoprotein (including the clones belonging to plural categories).
   HEMBA1000752, HEMBA1001017, HEMBA1001059, HEMBA1001071, HEMBA1001302, HEMBA1002150 , HEMBA1002547, HEMBA1002555, HEMBA1003077, HEMBA1003538, HEMBA1003598, HEMBA100386 6, HEMBA1004356, HEMBA1005338, HEMBA1005576, HEMBA1005699, HEMBA1006562, HEMBA10069 76, HEMBA1007301, HEMBB1000317, HEMBB1000530, HEMBB1001119, HEMBB1002092, HEMBB1002 193, MAMMA1000672, MAMMA1000897, MAMMA1001030, MAMMA1001388, MAMMA1001771, MAMMA100 2485, MAMMA1002636, MAMMA1002938, NT2RM1000648, NT2RM2000374, NT2RM2000407, NT2RM20 00422, NT2RM2000623, NT2RM2001499, NT2RM2001588, NT2RM2001839, NT2RM2001930, NT2RM4 000156, NT2RM4000233, NT2RM4001410, NT2RM4001987, NT2RM4002145, NT2RM4002189, NT2RM 4002194, NT2RM4002558, NT2RM4002571, NT2RP1000856, NT2RP1001247, NT2RP2000056, NT2R P2001991, NT2RP2002025, NT2RP2002325, NT2RP2002385, NT2RP2003706, NT2RP2005933, NT2 RP3001140, NT2RP3003242, NT2RP3003672, NT2RP3003701, NT2RP3003716, NT2RP3003914, NT 2RP3004253, NT2RP4000212, NT2RP4000417, NT2RP4000925, NT2RP4001149, NT2RP4001150, N T2RP4001219, NT2RP4001345, NT2RP4001372, NT2RP5003522, NT2RP5003524, OVARC1000543, OVARC1000682, OVARC1000722, OVARC1001055, OVARC1001154, OVARC1001506, OVARC1001713 PLACE1002437, PLACE1002722, PLACE1003258, PLACE1003493, PLACE1004197, PLACE100475 1, PLACE1005698, PLACE1005804, PLACE1006157, PLACE1006754, PLACE1008643, PLACE10090 94, PLACE1009861, PLACE1011371, PLACE2000034, PLACE2000062, PLACE2000399, PLACE2000458, PLACE3000009, PLACE3000020, PLACE3000353, PLACE3000373, PLACE4000052 , PLACE4000230, PLACE4000522, PLACE4000581, THYRO1000852, Y79AA1001874,
(3) The following 147 clones were predicted to encode a protein in the category of proteins associated with signal transduction (including the clones belonging to plural categories).
   HEMBA1000030, HEMBA1000185, HEMBA1000303, HEMBA1000459, HEMBA1000491, HEMBA1000657, HEMBA1000991, HEMBA1001174, HEMBA1001387, HEMBA1001822, HEMBA1001921, HEMBA100221 2, HEMBA1002341, HEMBA1002417, HEMBA1002810, HEMBA1002896, HEMBA1003291, HEMBA10033 14, HEMBA1003560, HEMBA1003615, HEMBA1003621, HEMBA1003805, HEMBA1004286, HEMBA1004 604, HEMBA1006173, HEMBA1006540, HEMBA1006973, HEMBA1007174, HEMBB1000632, HEMBB100 0781, HEMBB1001051, HEMBB1001177, HEMBB1001234, HEMBB1001294, HEMBB1001384, HEMBB10 02193, HEMBB1002477, HEMBB1002635, MAMMA1001305, MAMMA1002699, MAMMA1002842, MAMMA1 003057, MAMMA1003113, NT2RM1000118, NT2RM1000186, NT2RM1000772, NT2RM1000850, NT2RM 1001072, NT2RM2000124, NT2RM2000612, NT2RM2001065, NT2RM2001221, NT2RM2001345, NT2R M2001652, NT2RM2001983, NT2RM2002109, NT2RM2002128, NT2RM4000327, NT2RM4000354, NT2 RM4000798, NT2RM4001203, NT2RM4001411, NT2RM4001412, NT2RM4001582, NT2RM4001629, NT 2RM4001758, NT2RM4002226, NT2RP1000018, NT2RP1000112, NT2RP1000701, NT2RP1000825, N T2RP1001395, NT2RP1001410, NT2RP1001457, NT2RP1001482, NT2RP2002058, NT2RP2002710, NT2RP2003702, NT2RP2003912, NT2RP2004232, NT2RP2004768, NT2RP2005022, NT2RP2005620 , NT2RP2005890, NT2RP3000299, NT2RP3000403, NT2RP3000742, NT2RP3000845, NT2RP300176 4, NT2RP3002004, NT2RP3002785, NT2RP3002909, NT2RP3003230, NT2RP3003411, NT2RP30034 64, NT2RP3003800, NT2RP3004475, NT2RP3004534, NT2RP4000147, NT2RP4000376,
   NT2RP4001122, NT2RP4001375, NT2RP4001644, NT2RP4001725, NT2RP4001760, NT2RP4001849, NT2RP4001927, NT2RP4002408, NT2RP5003477, OVARC1000437, OVARC1000465, OVARC100064 9, OVARC1000945, OVARC1001476, OVARC1001861, PLACE1001377, PLACE1001384, PLACE10013 87, PLACE1002493, PLACE1002591, PLACE1002685, PLACE1003353, PLACE1004128, PLACE1004 918, PLACE1005502, PLACE1005739, PLACE1005968, PLACE1006385, PLACE1007238, PLACE100 7375, PLACE1007488, PLACE1008244, PLACE1008650, PLACE1009319, PLACE1009468, PLACE10 10083, PLACE1010942, PLACE1011041, PLACE1011046, PLACE1011923, PLACE2000164, PLACE3 000145, PLACE3000350, PLACE3000475, THYRO1001595, Y79AA1000059, Y79AA1000966,
(4) The following 230 clones were predicted to encode a protein in the category of proteins associated with transcription (including the clones belonging to plural categories).
   HEMBA1000201, HEMBA1000216, HEMBA1000488, HEMBA1000561, HEMBA1000851, HEMBA1001137 , HEMBA1001510, HEMBA1001579, HEMBA1001800, HEMBA1001809, HEMBA1001819, HEMBA100184 7, HEMBA1002035, HEMBA1002092, HEMBA1002569, HEMBA1002609, HEMBA1003545, HEMBA10035 68, HEMBA1003684, HEMBA1003760, HEMBA1003953, HEMBA1004097, HEMBA1004321, HEMBA1004 353, HEMBA1004479, HEMBA1004758, HEMBA1005359, HEMBA1005528, HEMBA1005548, HEMBA100 5931, HEMBA1006253, HEMBA1006278, HEMBA1006359, HEMBA1006559, HEMBB1000250, HEMBB10 00789, HEMBB1001011, HEMBB1001314, HEMBB1001482, HEMBB1001908, HEMBB1002134, HEMBB1 002217, MAMMA1000155, MAMMA1000183, MAMMA1000388, MAMMA1001633, MAMMA1001820, MAMMA 1001837, MAMMA1002650, MAMMA1002937, NT2RM1000055, NT2RM1000666, NT2RM1000691, NT2R M1000894, NT2RM1001092, NT2RM2000013, NT2RM2000735, NT2RM2001035, NT2RM2001575, NT2 RM2001605, NT2RM2001670, NT2RM2001771, NT2RM2001797, NT2RM2002580, NT2RM4000024, NT 2RM4000104, NT2RM4000191, NT2RM4000496, NT2RM4000531, NT2RM4000734, NT2RM4000751, N T2RM4000996, NT2RM4001140, NT2RM4001200, NT2RM4001483, NT2RM4001592, NT2RM4001746, NT2RM4001783, NT2RM4001823, NT2RM4001828, NT2RM4001858, NT2RM4001979, NT2RP1000111 , NT2RP1000333, NT2RP1000574, NT2RP1000721, NT2RP1000860, NT2RP2000008, NT2RP200013 3, NT2RP2000297, NT2RP2000931, NT2RP2001174, NT2RP2001233, NT2RP2002252, NT2RP20025 03, NT2RP2002591, NT2RP2003125, NT2RP2003228, NT2RP2003293, NT2RP2003564,
   NT2RP2003714, NT2RP2004013, NT2RP2004187, NT2RP2004961, NT2RP2005003, NT2RP2005037 , NT2RP2005287, NT2RP2005496, NT2RP2005539, NT2RP2005722, NT2RP2006238, NT2RP200646 4, NT2RP3000050, NT2RP3000397, NT2RP3000512, NT2RP3000527, NT2RP3000590, NT2RP30006 32, NT2RP3001057, NT2RP3001120, NT2RP3001155, NT2RP3001268, NT2RP3001398, NT2RP3001 399, NT2RP3001671, NT2RP3001672, NT2RP3001688, NT2RP3001712, NT2RP3001792, NT2RP300 1855, NT2RP3002165, NT2RP3002377, NT2RP3002399, NT2RP3003157, NT2RP3003193, NT2RP30 03251, NT2RP3003327, NT2RP3004258, NT2RP3004566, NT2RP3004572, NT2RP3004594, NT2RP3 004617, NT2RP4000078, NT2RP4000210, NT2RP4000398, NT2RP4000449, NT2RP4000787, NT2RP 4000865, NT2RP4000997, NT2RP4001029, NT2RP4001213, NT2RP4001433, NT2RP4001529, NT2R P4001551, NT2RP4001638, NT2RP4001753, NT2RP4001790, NT2RP4001938, NT2RP4002078, OVA RC1000479, OVARC1000846, OVARC1001381, OVARC1001555, OVARC1001577, OVARC1001949, PL ACE1000066, PLACE1000133, PLACE1000583, PLACE1000596, PLACE1000706, PLACE1001118, P LACE1001238, PLACE1001304, PLACE1001602, PLACE1001632, PLACE1002450, PLACE1002532, PLACE1002834, PLACE1003302, PLACE1003605, PLACE1003738, PLACE1003885, PLACE1004471 , PLACE1004510, PLACE1004681, PLACE1006414, PLACE1006438, PLACE1006482, PLACE100723 9, PLACE1007346, PLACE1007544, PLACE1007598, PLACE1007955, PLACE1007969, PLACE10086 27, PLACE1008941, PLACE1009099, PLACE1009246, PLACE1009398, PLACE1010261,
   PLACE1010491, PLACE1010702, PLACE1010771, PLACE1010870, PLACE1011576, PLACE2000072 , PLACE2000359, PLACE4000128, PLACE4000156, PLACE4000259, PLACE4000431, THYRO100012 1, THYRO1000242, THYRO1000501, THYRO1000585, THYRO1001100, THYRO1001189, Y79AA10000 37, Y79AA1000627, Y79AA1000705, Y79AA1001105, Y79AA1001299, Y79AA1001391, Y79AA1001 533, Y79AA1001613, Y79AA1001848, Y79AA1001866, Y79AA1002093, Y79AA1002472, Y79AA100 2482,
(5) The following 436 clones were predicted to encode a protein in the category of proteins associated with diseases (including the clones belonging to plural categories).
   HEMBA1000158, HEMBA1000216, HEMBA1000523, HEMBA1000561, HEMBA1000569, HEMBA1000673 , HEMBA1000972, HEMBA1001017, HEMBA1001019, HEMBA1001059, HEMBA1001071, HEMBA100108 8, HEMBA1001123, HEMBA1001407, HEMBA1001569, HEMBA1001570, HEMBA1001672, HEMBA10020 35, HEMBA1002160, HEMBA1002162, HEMBA1002547, HEMBA1002939, HEMBA1003077, HEMBA1003 418, HEMBA1003433, HEMBA1003538, HEMBA1003555, HEMBA1003568, HEMBA1003569, HEMBA100 3581, HEMBA1003720, HEMBA1003760, HEMBA1004168, HEMBA1004248, HEMBA1004276, HEMBA10 04354, HEMBA1004356, HEMBA1004479, HEMBA1004534, HEMBA1004669, HEMBA1004752, HEMBA1 004753, HEMBA1004807, HEMBA1005293, HEMBA1005331, HEMBA1005359, HEMBA1005367, HEMBA 1005423, HEMBA1005443, HEMBA1005475, HEMBA1005528, HEMBA1005576, HEMBA1005581, HEMB A1005679, HEMBA1005699, HEMBA1005732, HEMBA1005931, HEMBA1006173, HEMBA1006474, HEM BA1006562, HEMBA1006648, HEMBA1006652, HEMBA1006737, HEMBA1006807, HEMBA1006976, HE MBA1007078, HEMBA1007121, HEMBA1007243, HEMBA1007301, HEMBB1000264, HEMBB1000637, H EMBB1000684, HEMBB1000693, HEMBB1000915, HEMBB1000927, HEMBB1001011, HEMBB1001051, HEMBB1001068, HEMBB1001102, HEMBB1001119, HEMBB1001133, HEMBB1001177, HEMBB1001802, HEMBB1001908, HEMBB1001922, HEMBB1001967, HEMBB1002050, HEMBB1002193, HEMBB100221 7, HEMBB1002358, HEMBB1002635, HEMBB1002683, MAMMA1000009, MAMMA1000092, MAMMA10001 03, MAMMA1000173, MAMMA1000731, MAMMA1000897, MAMMA1000956, MAMMA1001021,
   MAMMA1001041, MAMMA1001105, MAMMA1001198, MAMMA1001305, MAMMA1001576, MAMMA1001633 , MAMMA1002140, MAMMA1002170, MAMMA1002198, MAMMA1002268, MAMMA1002352, MAMMA100239 2, MAMMA1002485, MAMMA1002530, MAMMA1002573, MAMMA1002636, MAMMA1002858, MAMMA10028 69, MAMMA1002881, MAMMA1002937, MAMMA1003047, MAMMA1003099, MAMMA1003146, MAMMA1003 166, NT2RM1000035, NT2RM1000153, NT2RM1000257, NT2RM1000318, NT2RM1000377, NT2RM100 0394, NT2RM1000555, NT2RM1000691, NT2RM1000725, NT2RM1000770, NT2RM1000826, NT2RM10 00850, NT2RM1001059, NT2RM1001072, NT2RM1001082, NT2RM1001092, NT2RM2000363, NT2RM2 000374, NT2RM2000594, NT2RM2000599, NT2RM2000714, NT2RM2000740, NT2RM2000821, NT2RM 2001035, NT2RM2001499, NT2RM2001575, NT2RM2001605, NT2RM2001664, NT2RM2001670, NT2R M2001803, NT2RM2001823, NT2RM2001839, NT2RM4000085, NT2RM4000139, NT2RM4000155, NT2 RM4000290, NT2RM4000689, NT2RM4000895, NT2RM4001320, NT2RM4001566, NT2RM4001629, NT 2RM4001741, NT2RM4001819, NT2RM4001823, NT2RM4001865, NT2RM4001940, NT2RM4002093, N T2RM4002146, NT2RM4002161, NT2RM4002189, NT2RM4002457, NT2RM4002558, NT2RM4002571, NT2RP1000086, NT2RP1000376, NT2RP1000574, NT2RP1000581, NT2RP1000629, NT2RP1000721 , NT2RP1000733, NT2RP1000738, NT2RP1000825, NT2RP1000833, NT2RP1000860, NT2RP100096 6, NT2RP1001033, NT2RP1001247, NT2RP2000126, NT2RP2000147, NT2RP2000233, NT2RP20002 97, NT2RP2000310, NT2RP2000414, NT2RP2000448, NT2RP2000459, NT2RP2000603,
   NT2RP2000812, NT2RP2001127, NT2RP2001327, NT2RP2001440, NT2RP2001520, NT2RP2001663, NT2RP2001748, NT2RP2001876, NT2RP2001898, NT2RP2002025, NT2RP2002066, NT2RP200225 9, NT2RP2002312, NT2RP2002503, NT2RP2002618, NT2RP2003228, NT2RP2003293, NT2RP20032 95, NT2RP2003517, NT2RP2003564, NT2RP2003706, NT2RP2003737, NT2RP2003968, NT2RP2004 013, NT2RP2004098, NT2RP2004187, NT2RP2004316, NT2RP2004523, NT2RP2004538, NT2RP200 4933, NT2RP2005144, NT2RP2005288, NT2RP2005289, NT2RP2005325, NT2RP2005358, NT2RP20 05531, NT2RP2005773, NT2RP2006219, NT2RP3000050, NT2RP3000080, NT2RP3000299, NT2RP3 000397, NT2RP3000512, NT2RP3000527, NT2RP3001081, NT2RP3001216, NT2RP3001268, NT2RP 3001355, NT2RP3001427, NT2RP3001428, NT2RP3001497, NT2RP3001554, NT2RP3001724, NT2R P3001764, NT2RP3001799, NT2RP3001855, NT2RP3002045, NT2RP3002151, NT2RP3002351, NT2 RP3002352, NT2RP3002663, NT2RP3002818, NT2RP3002876, NT2RP3002909, NT2RP3002972, NT 2RP3003061, NT2RP3003157, NT2RP3003242, NT2RP3003282, NT2RP3003672, NT2RP3004078, N T2RP3004209, NT2RP3004399, NT2RP3004424, NT2RP3004475, NT2RP3004480, NT2RP3004490, NT2RP3004527, NT2RP3004578, NT2RP4000049, NT2RP4000109, NT2RP4000185, NT2RP4000312 , NT2RP4000367, NT2RP4000398, NT2RP4000457, NT2RP4000855, NT2RP4000879, NT2RP400091 5, NT2RP4000928, NT2RP4001095, NT2RP4001150, NT2RP4001213, NT2RP4001483, NT2RP40014 98, NT2RP4001753, NT2RP4001838, OVARC1000014, OVARC1000114, OVARC1000139,
   OVARC1000241, OVARC1000440, OVARC1000543, OVARC1000722, OVARC1000771, OVARC1000834 , OVARC1000850, OVARC1001113, OVARC1001244, OVARC1001296, OVARC1001357, OVARC100137 2, OVARC1001417, OVARC1001496, OVARC1001506, OVARC1001577, OVARC1001668, OVARC10017 13, OVARC1001809, OVARC1001880, OVARC1001901, OVARC1001949, OVARC1002050, PLACE1000 133, PLACE1000142, PLACE1000184, PLACE1000383, PLACE1000406, PLACE1000420, PLACE100 0583, PLACE1000588, PLACE1000706, PLACE1001384, PLACE1001387, PLACE1001517, PLACE10 01602, PLACE1001634, PLACE1001771, PLACE1002046, PLACE1002437, PLACE1002474, PLACE1 002665, PLACE1002834, PLACE1003302, PLACE1003493, PLACE1003704, PLACE1003888, PLACE 1003903, PLACE1003968, PLACE1004197, PLACE1004358, PLACE1004471, PLACE1004681, PLAC E1004751, PLACE1004773, PLACE1005101, PLACE1006040, PLACE1006119, PLACE1006170, PLA CE1006187, PLACE1006288, PLACE1006414, PLACE1006438, PLACE1006962, PLACE1007239, PL ACE1007257, PLACE1007488, PLACE1007511, PLACE1007737, PLACE1008000, PLACE1008402, P LACE1008643, PLACE1008696, PLACE1008941, PLACE1009027, PLACE1009200, PLACE1009230, PLACE1009298, PLACE1009319, PLACE1009444, PLACE1009524, PLACE1010083, PLACE1010089 , PLACE1010599, PLACE1010833, PLACE1010856, PLACE1011054, PLACE1011057, PLACE101116 5, PLACE1011229, PLACE1011263, PLACE1011371, PLACE2000072, PLACE2000399, PLACE20004 38, PLACE2000458, PLACE3000004, PLACE3000242, PLACE3000352, PLACE3000353,
   PLACE3000455, PLACE3000475, PLACE4000009, PLACE4000014, PLACE4000052, PLACE4000320 , PLACE4000581, SKNMC1000050, THYRO1000026, THYRO1000173, THYRO1000186, THYRO100032 0, THYRO1000327, THYRO1000387, THYRO1000471, THYRO1000934, THYRO1001458, THYRO10015 95, THYRO1001605, THYRO1001617, THYRO1001671, Y79AA1000037, Y79AA1000469, Y79AA1000 539, Y79AA1000560, Y79AA1000734, Y79AA1000985, Y79AA1001105, Y79AA1001391, Y79AA100 1394, Y79AA1001548, Y79AA1001613, Y79AA1001874, Y79AA1002258, Y79AA1002416, Y79AA10 02482,
(6) The following 301 clones were predicted to encode a protein in the category of enzyme or proteins associated with metabolism (including the clones belonging to plural categories).
   HEMBA1000012, HEMBA1000459, HEMBA1000852, HEMBA1001059, HEMBA1001257, HEMBA1001620 , HEMBA1001714, HEMBA1001744, HEMBA1001896, HEMBA1002003, HEMBA1002212, HEMBA100225 7, HEMBA1002513, HEMBA1002973, HEMBA1003046, HEMBA1003136, HEMBA1003179, HEMBA10032 86, HEMBA1003291, HEMBA1003538, HEMBA1003680, HEMBA1004227, HEMBA1004408, HEMBA1004 734, HEMBA1004795, HEMBA1005513, HEMBA1005737, HEMBA1005815, HEMBA1006278, HEMBA100 6347, HEMBA1006521, HEMBA1006648, HEMBA1006877, HEMBA1006941, HEMBA1006976, HEMBA10 07121, HEMBA1007243, HEMBA1007300, HEMBB1000781, HEMBB1000915, HEMBB1001288, HEMBB1 001429, HEMBB1001443, HEMBB1001915, HEMBB1002042, HEMBB1002342, HEMBB1002358, HEMBB 1002635, HEMBB1002683, MAMMA1000020, MAMMA1000085, MAMMA1000672, MAMMA1000841, MAMM A1001008, MAMMA1001139, MAMMA1001476, MAMMA1001501, MAMMA1002268, MAMMA1002530, MAM MA1002619, MAMMA1002671, MAMMA1002938, NT2RM1000132, NT2RM1000153, NT2RM1000256, NT 2RM1000280, NT2RM1000377, NT2RM1000648, NT2RM1000850, NT2RM1000867, NT2RM1000894, N T2RM1001072, NT2RM2000013, NT2RM2000124, NT2RM2000191, NT2RM2000368, NT2RM2000371, NT2RM2000577, NT2RM2000594, NT2RM2000599, NT2RM2000951, NT2RM2001238, NT2RM2001664 , NT2RM2001700, NT2RM2001730, NT2RM2001785, NT2RM2001803, NT2RM2002030, NT2RM400002 4, NT2RM4000155, NT2RM4000344, NT2RM4000496, NT2RM4000616, NT2RM4000712, NT2RM40008 95, NT2RM4001313, NT2RM4001316, NT2RM4001340, NT2RM4001444, NT2RM4001758,
   NT2RM4001819, NT2RM4001930, NT2RM4002062, NT2RM4002063, NT2RM4002409, NT2RM4002558 , NT2RM4002571, NT2RM4002623, NT2RP1000018, NT2RP1000112, NT2RP1000376, NT2RP100060 9, NT2RP1000833, NT2RP1000834, NT2RP1001079, NT2RP1001253, NT2RP1001361, NT2RP10015 43, NT2RP2000056, NT2RP2000114, NT2RP2000183, NT2RP2000329, NT2RP2000422, NT2RP2000 710, NT2RP2000764, NT2RP2000932, NT2RP2001070, NT2RP2001663, NT2RP2001839, NT2RP200 1898, NT2RP2002256, NT2RP2002312, NT2RP2002442, NT2RP2002595, NT2RP2002618, NT2RP20 02993, NT2RP2003230, NT2RP2003286, NT2RP2003506, NT2RP2003643, NT2RP2003706, NT2RP2 003737, NT2RP2003781, NT2RP2003912, NT2RP2004232, NT2RP2004239, NT2RP2004768, NT2RP 2004791, NT2RP2004799, NT2RP2005038, NT2RP2005276, NT2RP2005344, NT2RP2005457, NT2R P2005498, NT2RP2005531, NT2RP2005557, NT2RP2005690, NT2RP2005773, NT2RP2005775, NT2 RP2005942, NT2RP2006571, NT2RP3000046, NT2RP3000085, NT2RP3000359, NT2RP3000418, NT 2RP3000742, NT2RP3000845, NT2RP3000875, NT2RP3001274, NT2RP3001407, NT2RP3001495, N T2RP3002351, NT2RP3002402, NT2RP3002969, NT2RP3003301, NT2RP3003346, NT2RP3003500, NT2RP3003800, NT2RP3003825, NT2RP3003831, NT2RP3003914, NT2RP3004155, NT2RP3004209 NT2RP3004669, NT2RP3004670, NT2RP4000259, NT2RP4000417, NT2RP4000457, NT2RP400072 4, NT2RP4000855, NT2RP4000928, NT2RP4000997, NT2RP4001041, NT2RP4001079, NT2RP40010 95, NT2RP4001219, NT2RP4001345, NT2RP4001375, NT2RP4001483, NT2RP4001592,
   NT2RP4001644, NT2RP4001893, NT2RP4001946, NT2RP4002408, NT2RP5003500, NT2RP5003522 , OVARC1000060, OVARC1000139, OVARC1000309, OVARC1000543, OVARC1000682, OVARC100072 2, OVARC1000885, OVARC1001261, OVARC1001610, OVARC1001713, OVARC1001762, OVARC10018 09, OVARC1001942, PLACE1000007, PLACE1000142, PLACE1000420, PLACE1000547, PLACE1000 653, PLACE1001062, PLACE1001603, PLACE1001692, PLACE1001817, PLACE1001989, PLACE100 2991, PLACE1003100, PLACE1003709, PLACE1003885, PLACE1003888, PLACE1003903, PLACE10 03915, PLACE1003968, PLACE1004428, PLACE1004437, PLACE1004751, PLACE1005305, PLACE1 005477, PLACE1005656, PLACE1005763, PLACE1005804, PLACE1006011, PLACE1016469, PLACE 1006731, PLACE1007729, PLACE1007958, PLACE1008275, PLACE1008356, PLACE1008696, PLAC E1009444, PLACE1009861, PLACE1009997, PLACE1010089, PLACE1010096, PLACE1010106, PLA CE1010152, PLACE1010481, PLACE1011046, PLACE1011203, PLACE1011219, PLACE1011229, PL ACE1011332, PLACE1011923, PLACE2000021, PLACE2000140, PLACE2000404, PLACE2000411, P LACE3000020, PLACE3000148, PLACE3000156, PLACE3000350, PLACE3000353, PLACE4000259, PLACE4000431, PLACE4000558, PLACE4000590, SKNMC1000050, THYRO1000017, THYRO1000197 , THYRO1000320, THYRO1000358, THYRO1000471, THYRO1000926, THYRO1000934, THYRO100095 1, THYRO1001406, THYRO1001617, THYRO1001671, Y79AA1001048, Y79AA1001233, Y79AA10015 81, Y79AA1001679, Y79AA1001827, Y79AA1002027, Y79AA1002209, Y79AA1002361, Y79AA1002 416,
(7) The following 74 clones were predicted to encode a protein in the category of proteins associated with cell division or cell proliferation (including the clones belonging to plural categories).
   HEMBA1001019, HEMBA1001595, HEMBA1002229, HEMBA1002363, HEMBA1003136, HEMBA1003836 , HEMBA1004131, HEMBA1005241, HEMBB1000399, HEMBB1001175, HEMBB1001242, MAMMA100182 0, MAMMA1003011, NT2RM1000394, NT2RM2001256, NT2RM4000215, NT2RM4001611, NT2RM40017 14, NT2RP1000733, NT2RP1001177, NT2RP2000077, NT2RP2000346, NT2RP2001397, NT2RP2003 912, NT2RP2005520, NT2RP2005675, NT2RP2005767, NT2RP2005857, NT2RP2006464, NT2RP300 0031, NT2RP3001155, NT2RP3001730, NT2RP3002151, NT2RP3002273, NT2RP3002399, NT2RP30 04507, NT2RP3004578, NT2RP3004594, NT2RP4000210, NT2RP4000498, NT2RP4001207, NT2RP4 001414, NT2RP4001551, OVARC1000006, OVARC1000087, OVARC1000113, OVARC1000846, OVARC 1000876, OVARC1001068, OVARC1001107, OVARC1001113, OVARC1002112, PLACE1000547, PLAC E1001611, PLACE1001691, PLACE1002775, PLACE1002811, PLACE1003709, PLACE1004316, PLA CE1004674, PLACE1004814, PLACE1005287, PLACE1006187, PLACE1006368, PLACE1008808, PL ACE1008947, PLACE1009060, PLACE1010720, PLACE1010761, PLACE1010833, PLACE1011056, P LACE3000244, Y79AA1000214, Y79AA1001236,
(8) The following 92 clones were predicted to encode a protein in the category of proteins associated with cytoskeleton (including the clones belonging to plural categories).
   HEMBA1000020, HEMBA1000588, HEMBA1001804, HEMBA1002102, HEMBA1002161, HEMBA1002939 , HEMBA1003235, HEMBA1003581, HEMBA1004534, HEMBA1005009, HEMBA1005595, HEMBA100627 2, HEMBA1006737, HEMBA1007018, HEMBB1000404, HEMBB1001282, MAMMA1000173, MAMMA10004 29, MAMMA1001041, MAMMA1001576, MAMMA1001735, MAMMA1002622, MAMMA1002637, MAMMA1003 099, MAMMA1003127, NT2RM1000898, NT2RM1001003, NT2RM1001139, NT2RM2000691, NT2RM400 0030, NT2RM4000167, NT2RM4000689, NT2RM4001741, NT2RM4001776, NT2RM4002109, NT2RP10 00348, NT2RP1000460, NT2RP1000478, NT2RP1001033, NT2RP2000812, NT2RP2001634, NT2RP2 001900, NT2RP2002727, NT2RP2003307, NT2RP2003604, NT2RP2004538, NT2RP2005531, NT2RP 3000868, NT2RP3001216, NT2RP3001272, NT2RP3002688, NT2RP3003061, NT2RP3003138, NT2R P3003230, NT2RP3003282, NT2RP3004454, NT2RP3004569, NT2RP4001126, NT2RP4001927, OVA RC1000437, OVARC1000520, OVARC1000679, OVARC1001731, OVARC1002050, PLACE1002571, PL ACE1002591, PLACE1002655, PLACE1003669, PLACE1004777, PLACE1005287, PLACE1006368, P LACE1007105, PLACE1010310, PLACE1010743, PLACE1010960, PLACE1011263, PLACE2000039, PLACE2000216, PLACE2000274, PLACE3000145, PLACE3000322, PLACE4000009, PLACE4000612 , THYRO1000072, THYRO1000666, THYRO1001121, THYRO1001458, Y79AA1000368, Y79AA100083 3, Y79AA1000962, Y79AA1000985, Y79AA1002208,
(9) The following 280 clones were predicted to encode a protein in the category of proteins associated with RNA synthesis (including the clones belonging to plural categories).
   HEMBA1000201, HEMBA1000216, HEMBA1000488, HEMBA1000561, HEMBA1000591, HEMBA1000851 , HEMBA1001137, HEMBA1001510, HEMBA1001579, HEMBA1001800, HEMBA1001809, HEMBA100181 9, HEMBA1001847, HEMBA1002035, HEMBA1002092, HEMBA1002569, HEMBA1002609, HEMBA10034 18, HEMBA1003545, HEMBA1003568, HEMBA1003684, HEMBA1003760, HEMBA1003953, HEMBA1004 097, HEMBA1004321, HEMBA1004353, HEMBA1004479, HEMBA1004669, HEMBA1004758, HEMBA100 5359, HEMBA1005528, HEMBA1005548, HEMBA1005931, HEMBA1006253, HEMBA1006278, HEMBA10 06359, HEMBA1006559, HEMBA1006639, HEMBB1000250, HEMBB1000789, HEMBB1001011, HEMBB1 001314, HEMBB1001482, HEMBB1001908, HEMBB1002134, HEMBB1002217, MAMMA1000155, MAMMA 1000183, MAMMA1000388, MAMMA1001059, MAMMA1001633, MAMMA1001820, MAMMA1001837, MAMM A1002650, MAMMA1002937, NT2RM1000055, NT2RM1000187, NT2RM1000666, NT2RM1000691, NT2 RM1000852, NT2RM1000894, NT2RM1001092, NT2RM2000013, NT2RM2000735, NT2RM2001035, NT 2RM2001424, NT2RM2001575, NT2RM2001605, NT2RM2001670, NT2RM2001771, NT2RM2001797, N T2RM2001989, NT2RM2002100, NT2RM2002580, NT2RM4000024, NT2RM4000104, NT2RM4000191, NT2RM4000421, NT2RM4000471, NT2RM4000496, NT2RM4000531, NT2RM4000734, NT2RM4000751 , NT2RM4000996, NT2RM4001140, NT2RM4001200, NT2RM4001483, NT2RM4001592, NT2RM400174 6, NT2RM4001783, NT2RM4001823, NT2RM4001828, NT2RM4001858, NT2RM4001979, NT2RM40020 93, NT2RM4002479, NT2RP1000111, NT2RP1000272, NT2RP1000333, NT2RP1000470,
   NT2RP1000574, NT2RP1000721, NT2RP1000860, NT2RP1001080, NT2RP2000008, NT2RP2000133 , NT2RP2000161, NT2RP2000297, NT2RP2000931, NT2RP2001174, NT2RP2001233, NT2RP200144 9, NT2RP2002099, NT2RP2002252, NT2RP2002503, NT2RP2002591, NT2RP2002928, NT2RP20031 25, NT2RP2003228, NT2RP2003293, NT2RP2003564, NT2RP2003714, NT2RP2004013, NT2RP2004 187, NT2RP2004568, NT2RP2004961, NT2RP2005003, NT2RP2005037, NT2RP2005126, NT2RP200 5168, NT2RP2005239, NT2RP2005287, NT2RP2005496, NT2RP2005539, NT2RP2005722, NT2RP20 05763, NT2RP2005942, NT2RP2006238, NT2RP2006464, NT2RP3000050, NT2RP3000361, NT2RP3 000397, NT2RP3000512, NT2RP3000527, NT2RP3000590, NT2RP3000632, NT2RP3000917, NT2RP 3001057, NT2RP3001120, NT2RP3001155, NT2RP3001268, NT2RP3001398, NT2RP3001399, NT2R P3001671, NT2RP3001672, NT2RP3001688, NT2RP3001712, NT2RP3001792, NT2RP3001855, NT2 RP3002165, NT2RP3002377, NT2RP3002399, NT2RP3003157, NT2RP3003193, NT2RP3003251, NT 2RP3003327, NT2RP3004013, NT2RP3004258, NT2RP3004504, NT2RP3004566, NT2RP3004572, N T2RP3004594, NT2RP3004617, NT2RP4000078, NT2RP4000111, NT2RP4000210, NT2RP4000398, NT2RP4000449, NT2RP4000518, NT2RP4000614, NT2RP4000787, NT2RP4000865, NT2RP4000997 , NT2RP4001029, NT2RP4001080, NT2RP4001148, NT2RP4001213, NT2RP4001433, NT2RP400152 9, NT2RP4001551, NT2RP4001638, NT2RP4001753, NT2RP4001790, NT2RP4001938, NT2RP40020 78, OVARC1000479, OVARC1000846, OVARC1001381, OVARC1001555, OVARC1001577,
   OVARC1001949, PLACE1000066, PLACE1000133, PLACE1000583, PLACE1000596, PLACE1000706 , PLACE1001118, PLACE1001238, PLACE1001304, PLACE1001602, PLACE1001632, PLACE100245 0, PLACE1002532, PLACE1002834, PLACE1003190, PLACE1003302, PLACE1003605, PLACE10037 04, PLACE1003738, PLACE1003885, PLACE1004471, PLACE1004510, PLACE1004564, PLACE1004 681, PLACE1004902, PLACE1005646, PLACE1005876, PLACE1006196, PLACE1006414, PLACE100 6438, PLACE1006482, PLACE1007239, PLACE1007346, PLACE1007544, PLACE1007598, PLACE10 07955, PLACE1007969, PLACE1008627, PLACE1008941, PLACE1009099, PLACE1009246, PLACE1 009398, PLACE1010053, PLACE1010194, PLACE1010261, PLACE1010491, PLACE1010580, PLACE 1010702, PLACE1010771, PLACE1010870, PLACE1011576, PLACE2000072, PLACE2000359, PLAC E4000128, PLACE4000156, PLACE4000259, PLACE4000431, THYRO1000121, THYRO1000242, THY RO1000501, THYRO1000585, THYRO1001100, THYRO1001189, Y79AA1000037, Y79AA1000349, Y7 9AA1000539, Y79AA1000627, Y79AA1000705, Y79AA1000752, Y79AA1001105, Y79AA1001299, Y 79AA1001391, Y79AA1001533, Y79AA1001613, Y79AA1001848, Y79AA1001866, Y79AA1001963, Y79AA1002093, Y79AA1002472, Y79AA1002482,
(10) The following 352 clones were predicted to encode a protein in the category of nuclear protein (including the clones belonging to plural categories).
   HEMBA1000005, HEMBA1000201, HEMBA1000216, HEMBA1000488, HEMBA1000561, HEMBA1000827 , HEMBA1000851, HEMBA1001088, HEMBA1001137, HEMBA1001476, HEMBA1001510, HEMBA100157 9, HEMBA1001800, HEMBA1001809, HEMBA1001819, HEMBA1001847, HEMBA1002035, HEMBA10020 92, HEMBA1002363, HEMBA1002495, HEMBA1002569, HEMBA1002609, HEMBA1002999, HEMBA1003 148, HEMBA1003418, HEMBA1003545, HEMBA1003568, HEMBA1003684, HEMBA1003760, HEMBA100 3783, HEMBA1003953, HEMBA1004097, HEMBA1004321, HEMBA1004353, HEMBA1004479, HEMBA10 04669, HEMBA1004758, HEMBA1005359, HEMBA1005475, HEMBA1005528, HEMBA1005548, HEMBA1 005931, HEMBA1006253, HEMBA1006278, HEMBA1006359, HEMBA1006474, HEMBA1006559, HEMBA 1006914, HEMBB1000250, HEMBB1000399, HEMBB1000789, HEMBB1001011, HEMBB1001314, HEMB B1001482, HEMBB1001908, HEMBB1001915, HEMBB1002134, HEMBB1002217, MAMMA1000155, MAM MA1000183, MAMMA1000388, MAMMA1000731, MAMMA1001633, MAMMA1001820, MAMMA1001837, MA MMA1002650, MAMMA1002869, MAMMA1002937, MAMMA1003011, NT2RM1000055, NT2RM1000187, N T2RM1000257, NT2RM1000394, NT2RM1000666, NT2RM1000691, NT2RM1000852, NT2RM1000894, NT2RM1001092, NT2RM2000013, NT2RM2000735, NT2RM2001035, NT2RM2001575, NT2RM2001605 , NT2RM2001635, NT2RM2001670, NT2RM2001771, NT2RM2001797, NT2RM2001823, NT2RM200193 6, NT2RM2001989, NT2RM2002100, NT2RM2002580, NT2RM4000024, NT2RM4000085, NT2RM40001 04, NT2RM4000191, NT2RM4000215, NT2RM4000290, NT2RM4000421, NT2RM4000496,
   NT2RM4000531, NT2RM4000734, NT2RM4000751, NT2RM4000996, NT2RM4001140, NT2RM4001200 , NT2RM4001217, NT2RM4001382, NT2RM4001483, NT2RM4001592, NT2RM4001605, NT2RM400161 1, NT2RM4001746, NT2RM4001783, NT2RM4001823, NT2RM4001828, NT2RM4001858, NT2RM40019 79, NT2RM4002093, NT2RM4002146, NT2RM4002527, NT2RP1000035, NT2RP1000111, NT2RP1000 333, NT2RP1000493, NT2RP1000574, NT2RP1000721, NT2RP1000860, NT2RP1000966, NT2RP100 1177, NT2RP2000008, NT2RP2000126, NT2RP2000133, NT2RP2000161, NT2RP2000297, NT2RP20 00414, NT2RP2000931, NT2RP2001174, NT2RP2001233, NT2RP2001420, NT2RP2001449, NT2RP2 001536, NT2RP2001762, NT2RP2002185, NT2RP2002252, NT2RP2002259, NT2RP2002503, NT2RP 2002504, NT2RP2002591, NT2RP2002993, NT2RP2003125, NT2RP2003228, NT2RP2003277, NT2R P2003286, NT2RP2003293, NT2RP2003308, NT2RP2003564, NT2RP2003714, NT2RP2004013, NT2 RP2004187, NT2RP2004961, NT2RP2005003, NT2RP2005037, NT2RP2005287, NT2RP2005496, NT 2RP2005520, NT2RP2005539, NT2RP2005722, NT2RP2005767, NT2RP2005857, NT2RP2005942, N T2RP2006238, NT2RP2006464, NT2RP3000031, NT2RP3000050, NT2RP3000361, NT2RP3000397, NT2RP3000512, NT2RP3000527, NT2RP1000590, NT2RP3000603, NT2RP3000632, NT2RP3000917 , NT2RP3001057, NT2RP3001120, NT2RP3001155, NT2RP3001268, NT2RP3001274, NT2RP300139 8, NT2RP3001399, NT2RP3001427, NT2RP3001428, NT2RP3001587, NT2RP3001671, NT2RP30016 72, NT2RP3001688, NT2RP3001712, NT2RP3001724, NT2RP3001792, NT2RP3001855,
   NT2RP3002056, NT2RP3002165, NT2RP3002377, NT2RP3002399, NT2RP3003157, NT2RP3003193 , NT2RP3003212, NT2RP3003251, NT2RP3003327, NT2RP3004206, NT2RP3004209, NT2RP300425 8, NT2RP3004566, NT2RP3004572, NT2RP3004594, NT2RP3004617, NT2RP4000078, NT2RP40001 11, NT2RP4000150, NT2RP4000210, NT2RP4000398, NT2RP4000449, NT2RP4000457, NT2RP4000 518, NT2RP4000787, NT2RP4000865, NT2RP4000997, NT2RP4001029, NT2RP4001080, NT2RP400 1148, NT2RP4001207, NT2RP4001213, NT2RP4001433, NT2RP4001529, NT2RP4001551, NT2RP40 01638, NT2RP4001753, NT2RP4001790, NT2RP4001938, NT2RP4002078, OVARC1000006, OVARC1 000087, OVARC1000139, OVARC1000326, OVARC1000440, OVARC1000479, OVARC1000846, OVARC 1000883, OVARC1001180, OVARC1001244, OVARC1001381, OVARC1001555, OVARC1001577, OVAR C1001702, OVARC1001949, OVARC1002112, PLACE1000066, PLACE1000133, PLACE1000406, PLA CE1000583, PLACE1000596, PLACE1000706, PLACE1001118, PLACE1001238, PLACE1001304, PL ACE1001602, PLACE1001611, PLACE1001632, PLACE1002450, PLACE1002532, PLACE1002834, P LACE1003100, PLACE1003190, PLACE1003302, PLACE1003605, PLACE1003704, PLACE1003738, PLACE1003885, PLACE1004471, PLACE1004510, PLACE1004564, PLACE1004681, PLACE1004902 , PLACE1005287, PLACE1005876, PLACE1006011, PLACE1006119, PLACE1006414, PLACE100643 8, PLACE1006482, PLACE1007239, PLACE1007346, PLACE1007544, PLACE1007598, PLACE10079 55, PLACE1007969, PLACE1008044, PLACE1008177, PLACE1008603, PLACE1008627,
   PLACE1008941, PLACE1008947, PLACE1009099, PLACE1009113, PLACE1009246, PLACE1009398 , PLACE1010152, PLACE1010194, PLACE1010261, PLACE1010491, PLACE1010702, PLACE101072 0, PLACE1010771, PLACE1010870, PLACE1011056, PLACE1011229, PLACE1011576, PLACE10116 64, PLACE2000072, PLACE2000359, PLACE2000411, PLACE4000014, PLACE4000128, PLACE4000 156, PLACE4000259, PLACE4000261, PLACE4000431, THYRO1000121, THYRO1000242, THYRO100 0501, THYRO1000585, THYRO1001100, THYRO1001189, Y79AA1000037, Y79AA1000214, Y79AA10 00539, Y79AA1000589, Y79AA1000627, Y79AA1000705, Y79AA1000752, Y79AA1000784, Y79AA1 001105, Y79AA1001299, Y79AA1001391, Y79AA1001533, Y79AA1001613, Y79AA1001848, Y79AA 1001866, Y79AA1001963, Y79AA1002093, Y79AA1002472, Y79AA1002482,
(11) The following 111 clones were predicted to encode a protein in the category of proteins associated with protein synthesis or transport (including the clones belonging to plural categories).
   HEMBA1000012, HEMBA1000542, HEMBA1001913, HEMBA1003773, HEMBA1004202, HEMBA1004356 , HEMBA1004847, HEMBA1005047,HEMBA1005202, HEMBA1005963, HEMBA1006310, HEMBA100665 2, HEMBA1006914, HEMBB1000217, HEMBB1000725, HEMBB1001346, HEMBB1001831, MAMMA10000 85, MAMMA1000734, MAMMA1002170, MAMMA1002236, MAMMA1002598, NT2RM1000661, NT2RM2000 504, NT2RM2000577, NT2RM2001131, NT2RM4000155, NT2RM4001444, NT2RM4002062, NT2RM400 2205, NT2RM4002623, NT2RP1000326, NT2RP1000947, NT2RP1001569, NT2RP2000710, NT2RP20 00880, NT2RP2001290, NT2RP2001511, NT2RP2001660, NT2RP2002185, NT2RP2002606, NT2RP2 002959, NT2RP2002980, NT2RP2003158, NT2RP2003760, NT2RP2004194, NT2RP2004791, NT2RP 2005012, NT2RP2005116, NT2RP2005204, NT2RP2006565, NT2RP2006598, NT2RP3000047, NT2R P3000366, NT2RP3000759, NT2RP3000968, NT2RP3001587, NT2RP3002529, NT2RP3003385, NT2 RP3003589, NT2RP3003876, NT2RP3004618, NT2RP4000370, NT2RP4000524, NT2RP4000528, NT 2RP4001041, NT2RP4001574, NT2RP4001592, NT2RP4002047, OVARC1000004, OVARC1000771, O VARC1000862, OVARC1001180, OVARC1001342, OVARC1001766, OVARC1002138, PLACE1000061, PLACE1000492, PLACE1000863, PLACE1001748, PLACE1002090, PLACE1003394, PLACE1003915 , PLACE1004149, PLACE1004743, PLACE1005557, PLACE1005813, PLACE1006488, PLACE100653 1, PLACE1006615, PLACE1007706, PLACE1008273, PLACE1008790, PLACE1008813, PLACE10097 21, PLACE1009763, PLACE1009845, PLACE1010074, PLACE1011109, PLACE1011477,
   PLACE2000404, PLACE3000059, PLACE3000121, PLACE4000558, PLACE4000654, SKNMC1000011 , THYRO1000288, THYRO1000974, Y79AA1000346, Y79AA1000968, Y79AA1002209,
(12) The following 23 clones were predicted to encode a protein in the category of proteins associated with cellular defense (including the clones belonging to plural categories).
   HEMBA1000005, HEMBA1000531, HEMBB1000217, HEMBB1000399, MAMMA1000734, NT2RP1000493 , NT2RP1000493, NT2RP2000006, NT2RP2000045, NT2RP2001536, NT2RP2005204, NT2RP300059 0, NT2RP3001426, NT2RP3002056, NT2RP3004262, NT2RP4001638, OVARC1001900, PLACE10056 11, PLACE1006958, PLACE1008275, PLACE1009113, PLACE4000014, Y79AA1002139,
(13) The following 23 clones were predicted to encode a protein in the category of proteins associated with development or growth (including the clones belonging to plural categories).
   HEMBB1001802, HEMBB1002442, NT2RM2002142, NT2RM4001047, NT2RP2003308, NT2RP2004816 , NT2RP3000994, NT2RP3001340, NT2RP3004206, NT2RP3004472, NT2RP4000246, NT2RP400100 6, OVARC1000304, OVARC1000746, OVARC1000996, PLACE1006037, PLACE1009622, PLACE10116 64, Y79AA1001692,

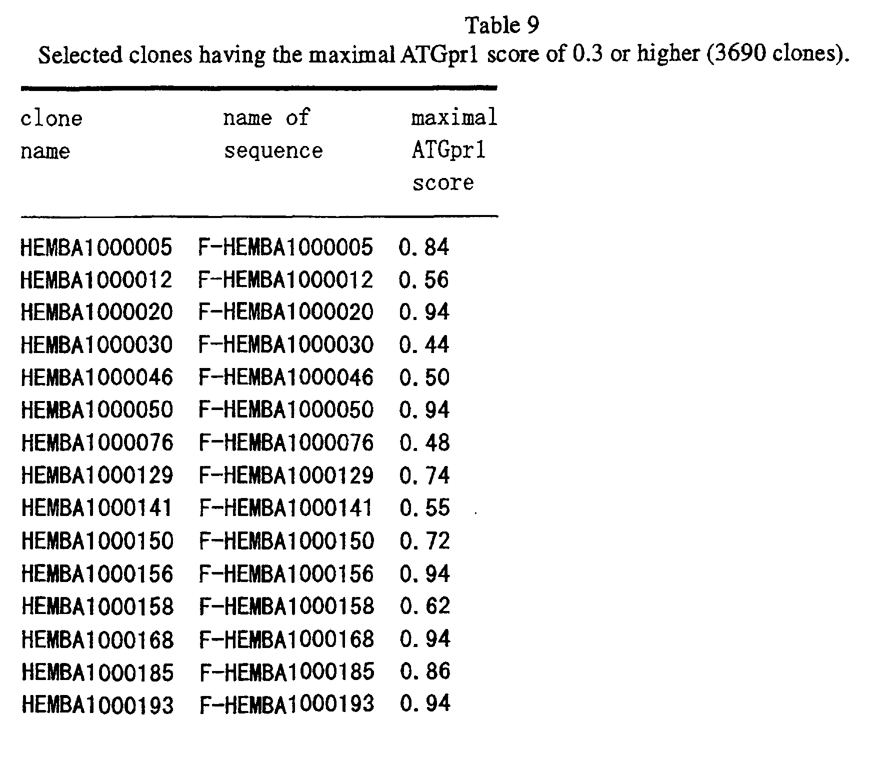

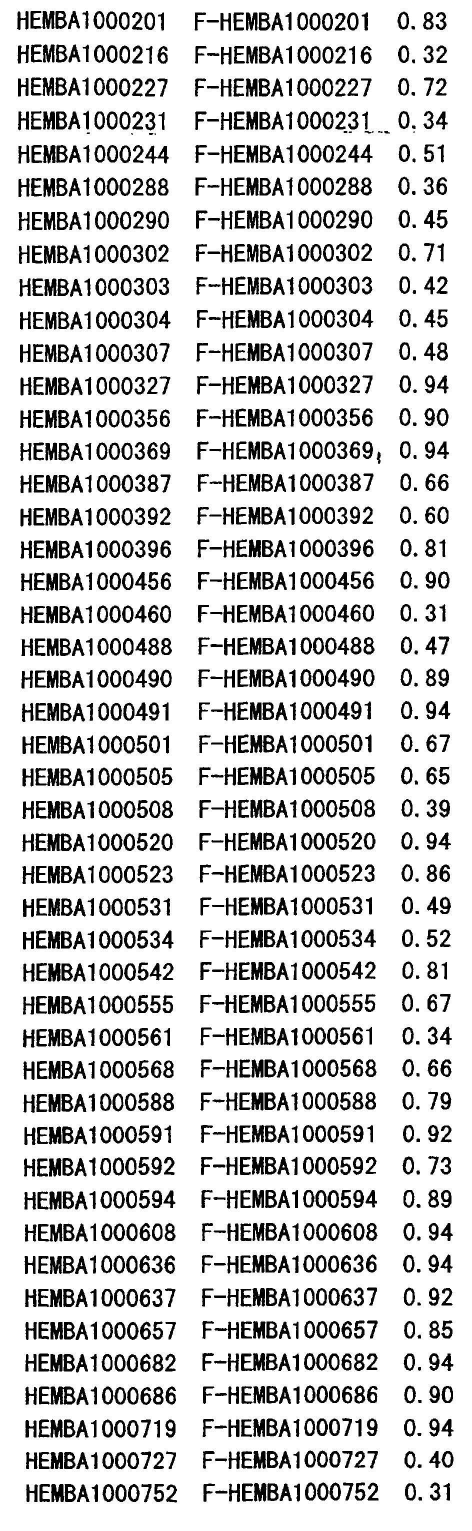

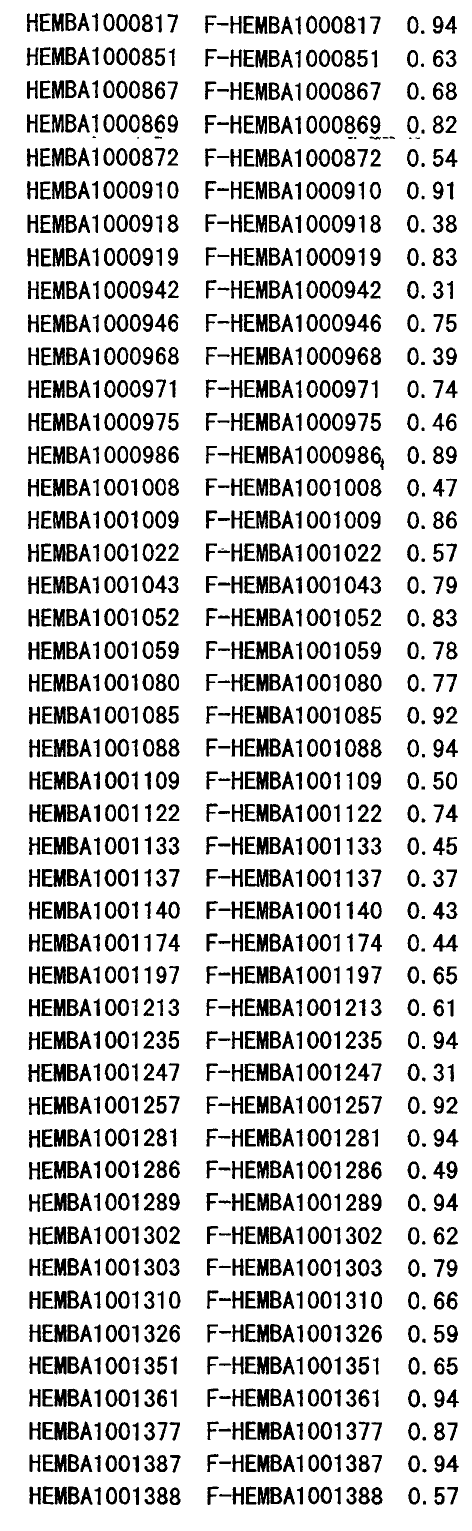

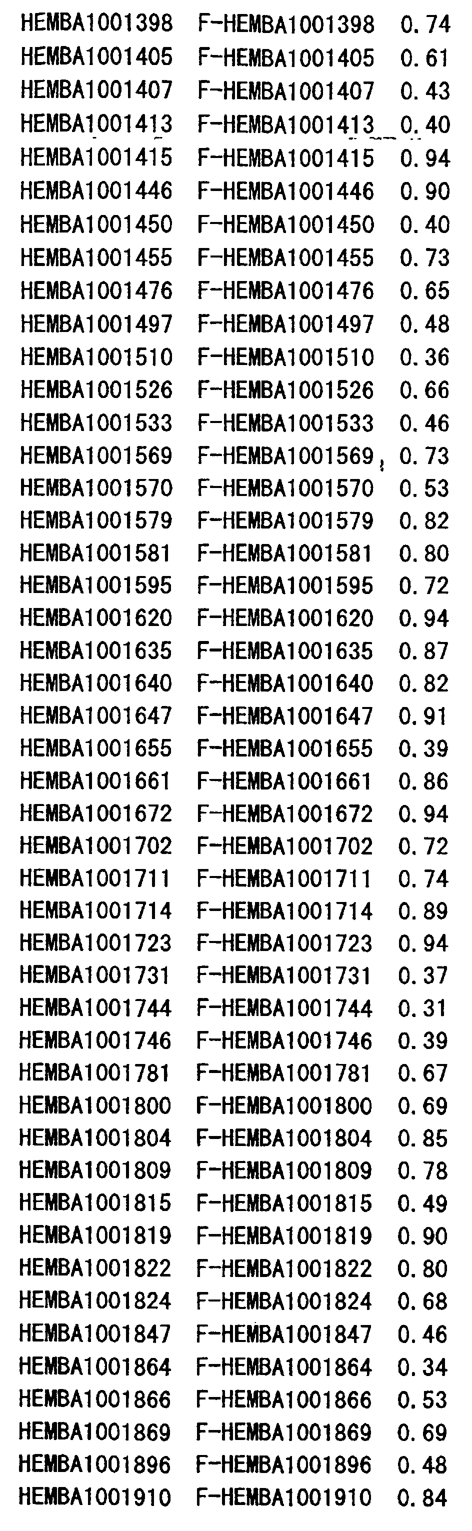

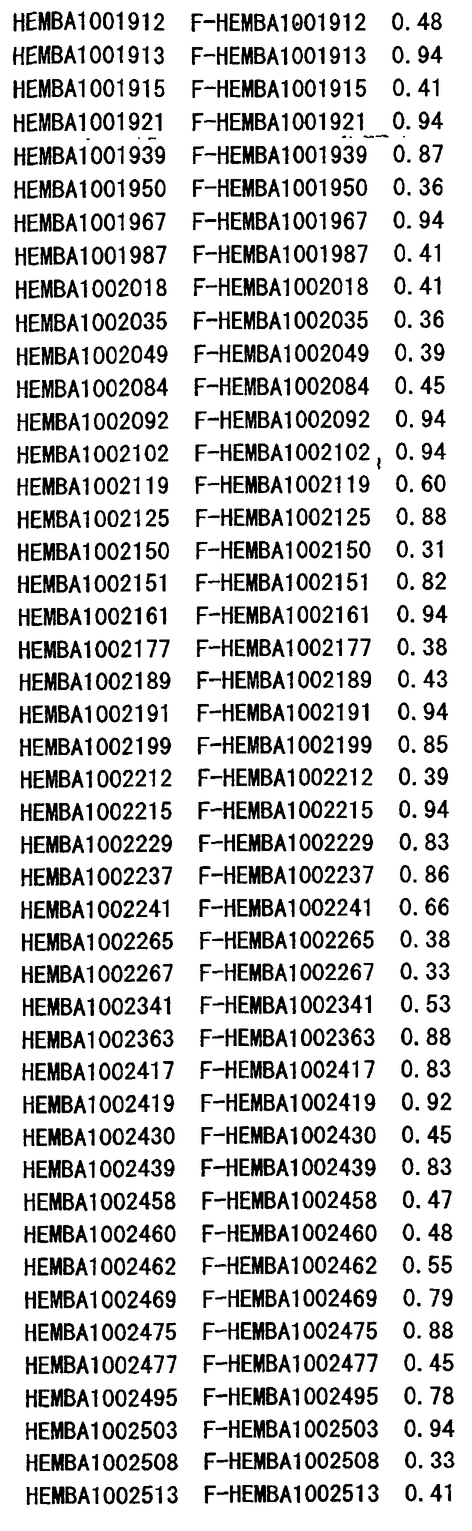

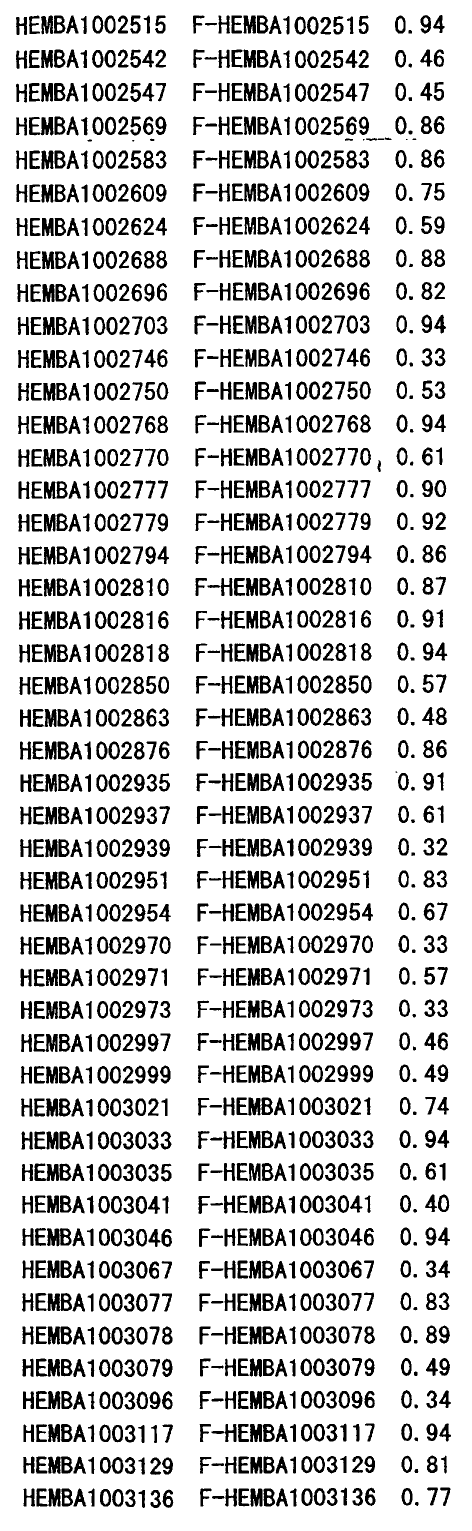

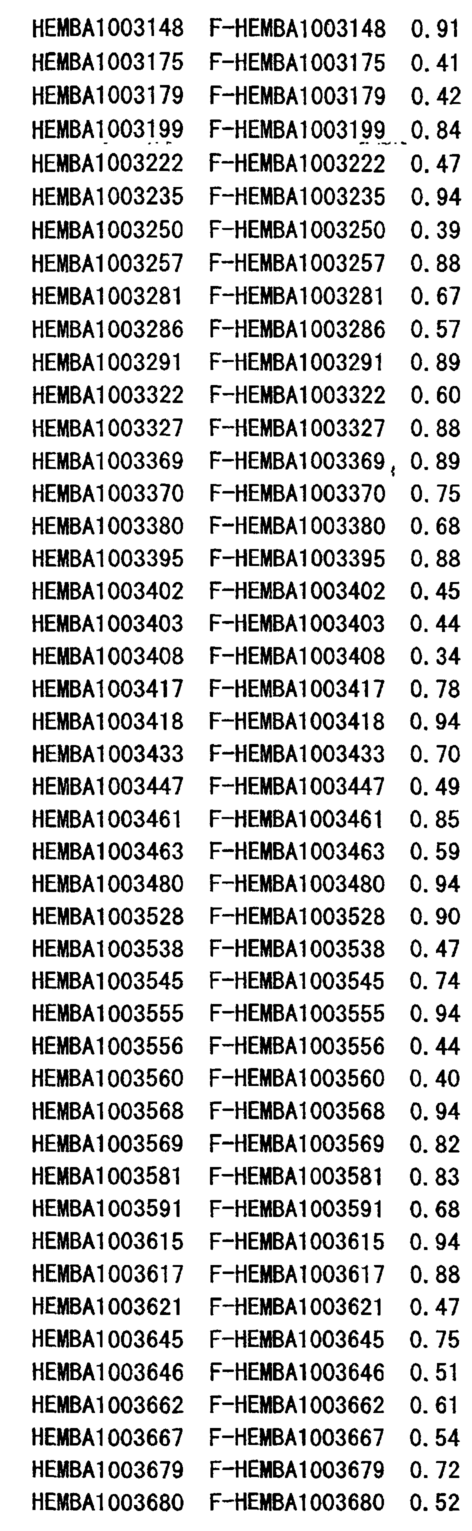

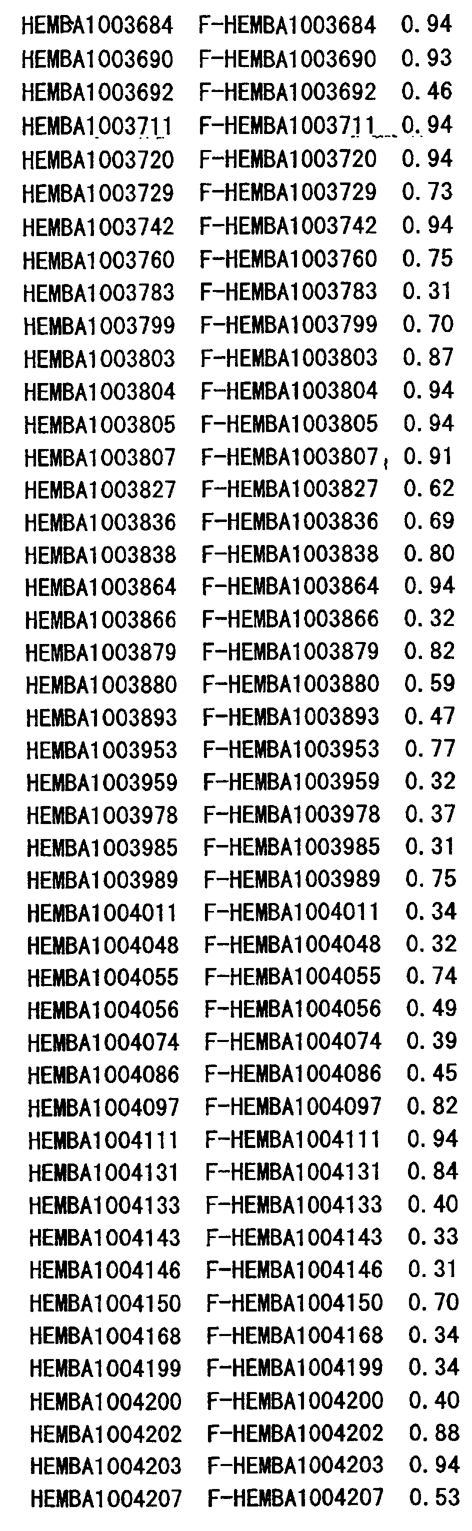

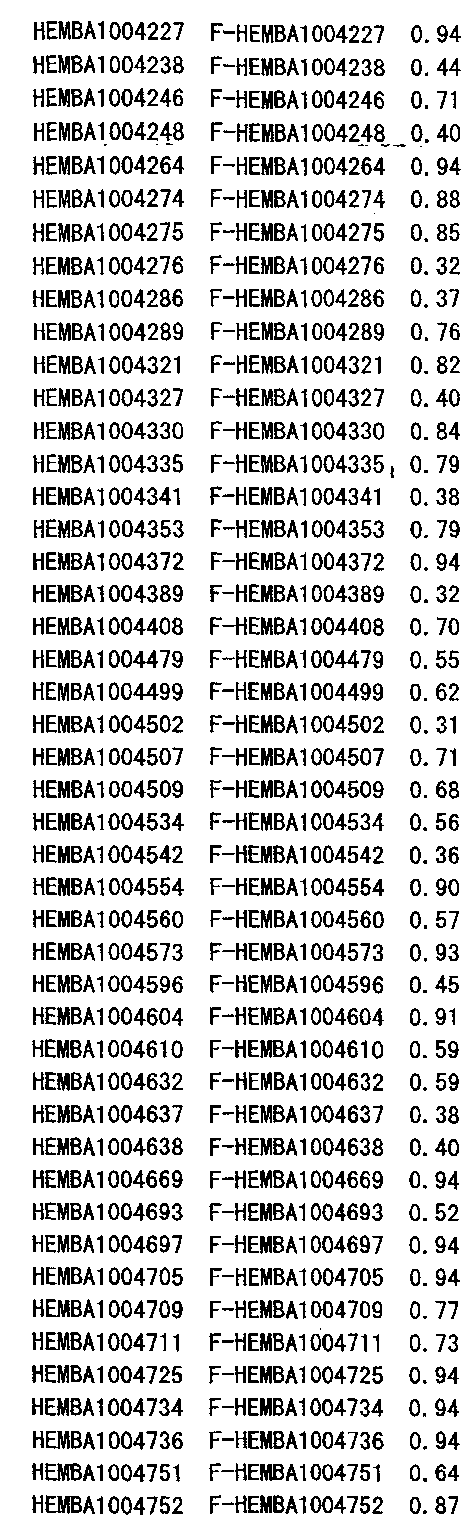

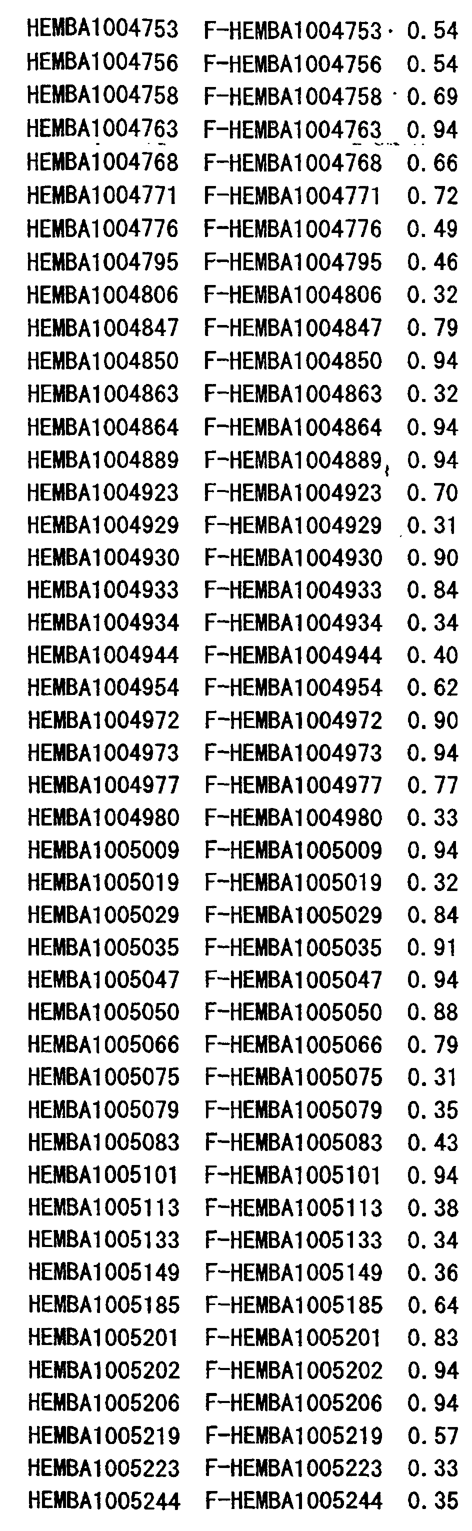

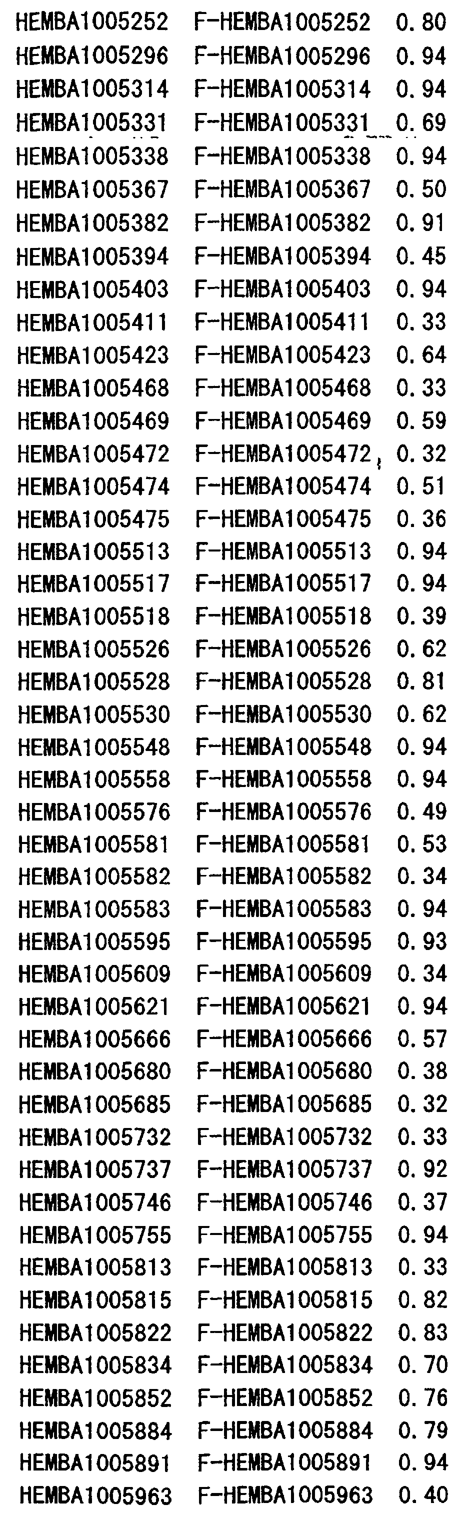

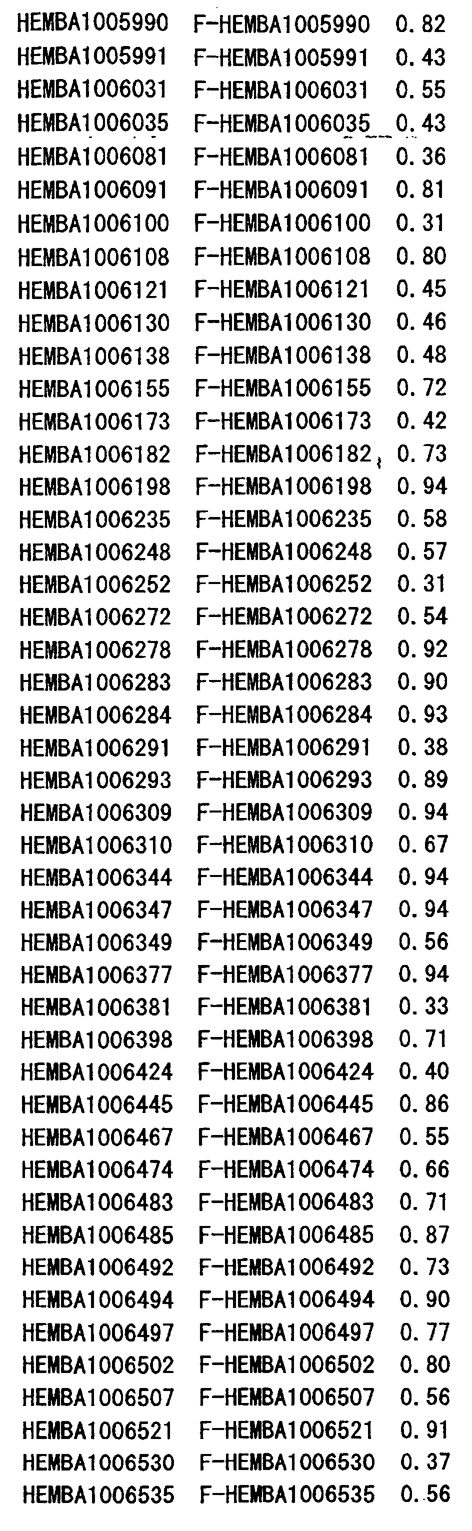

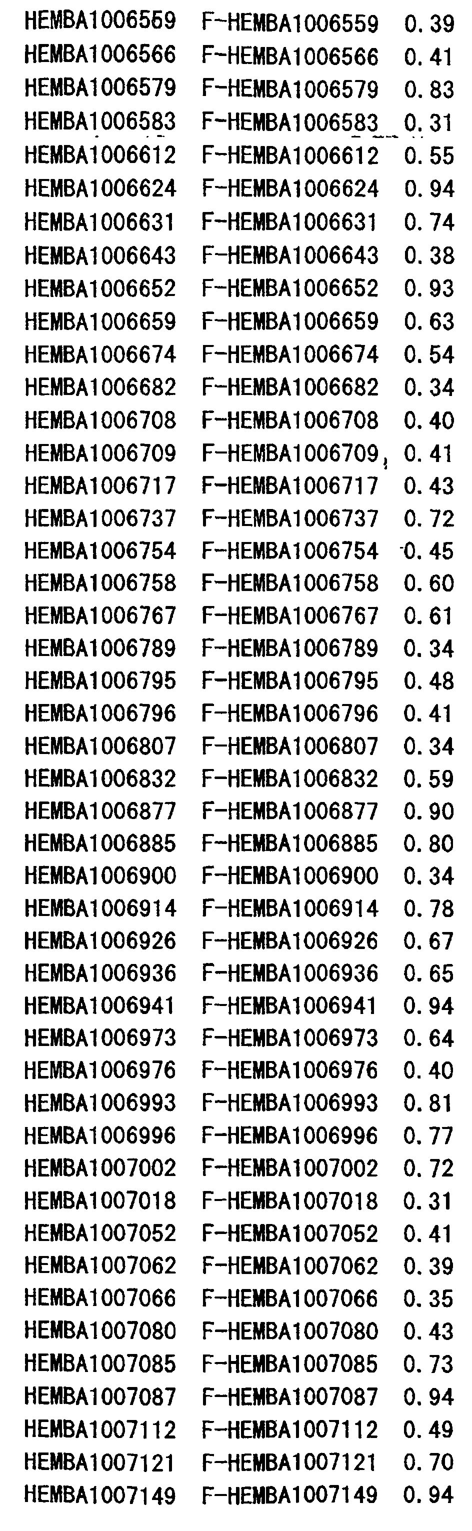

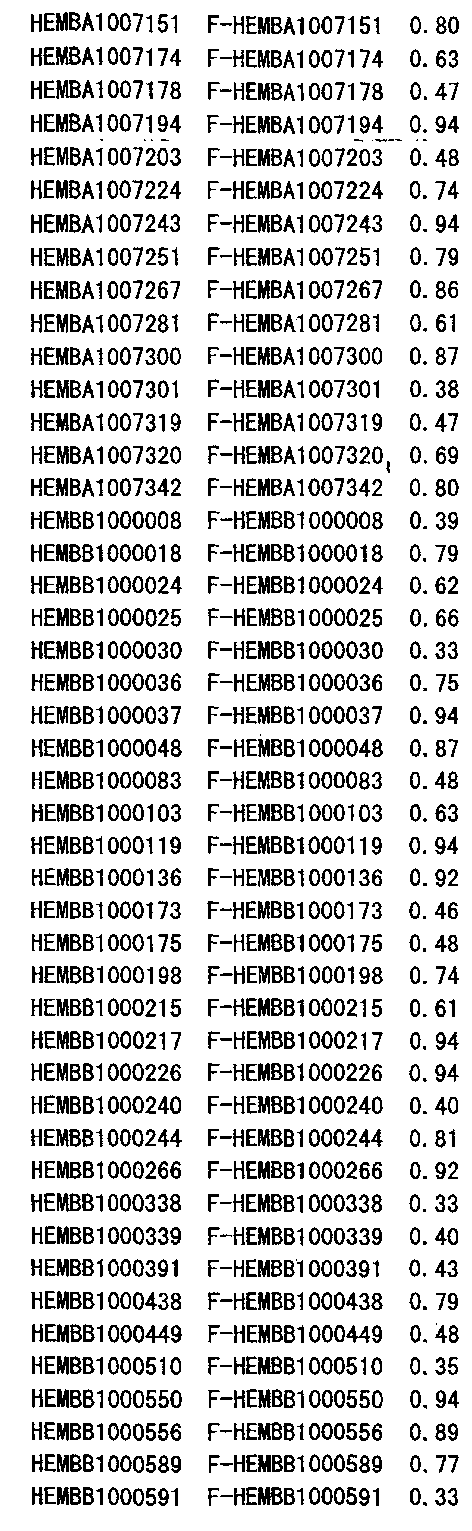

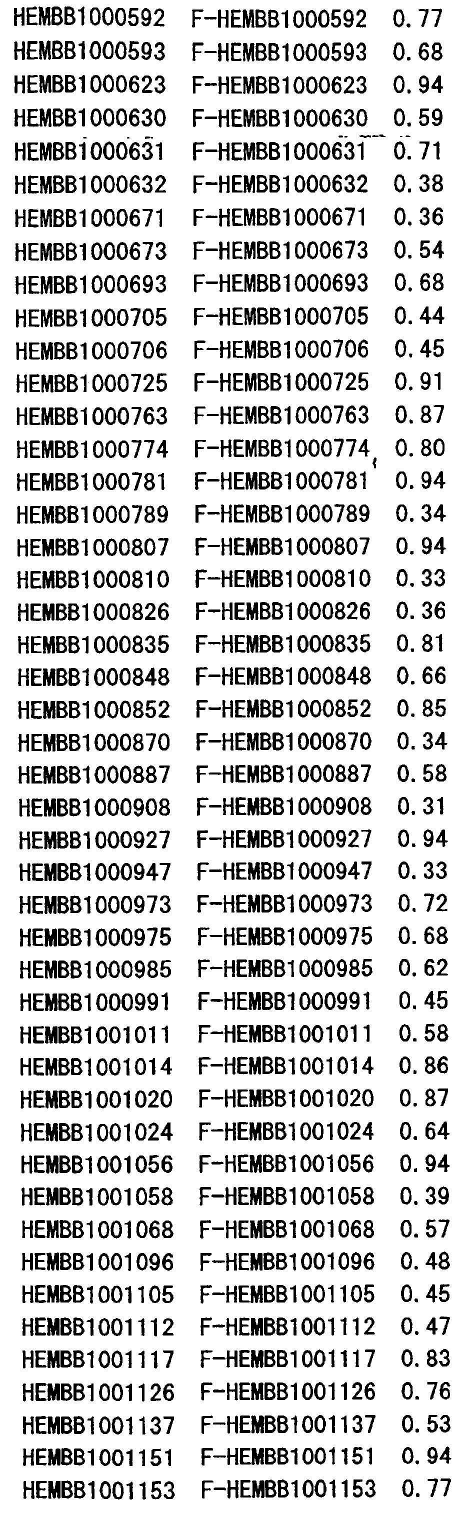

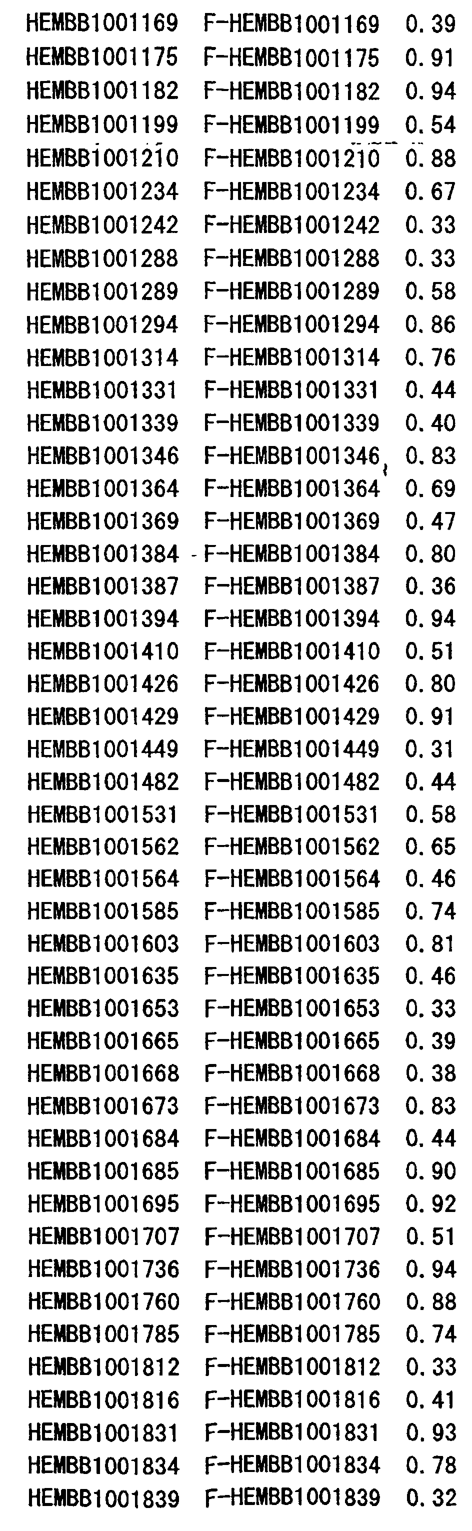

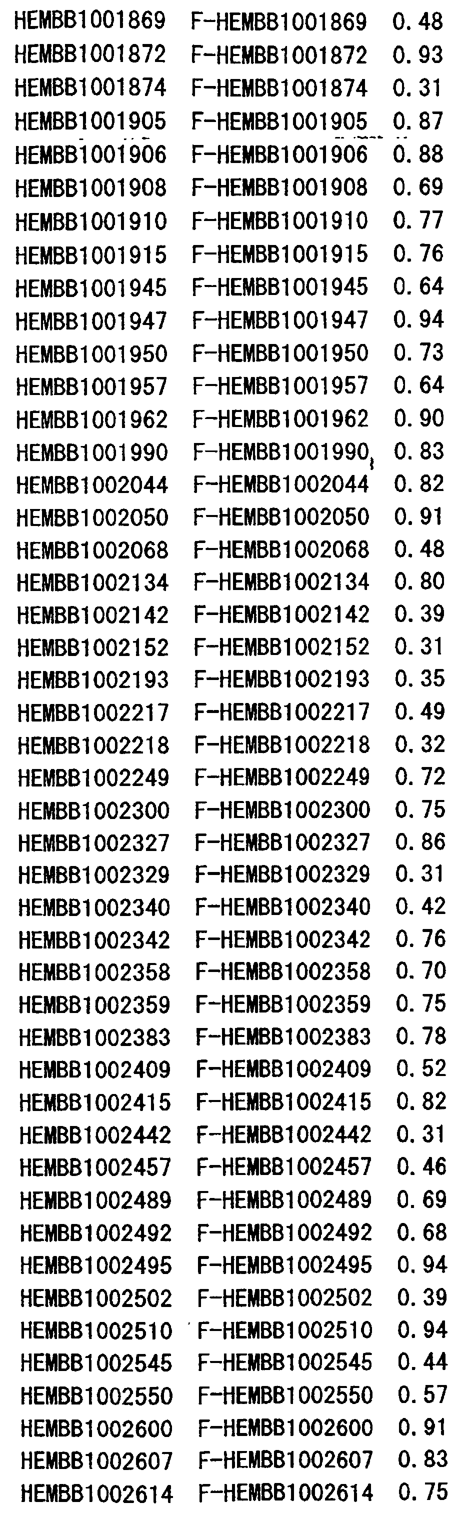

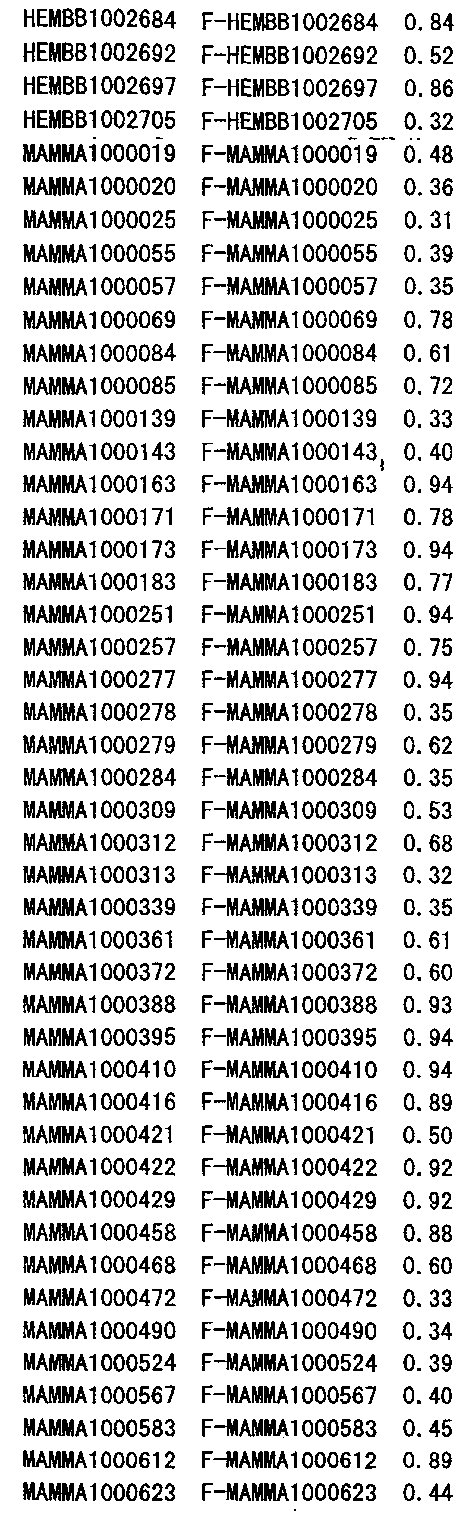

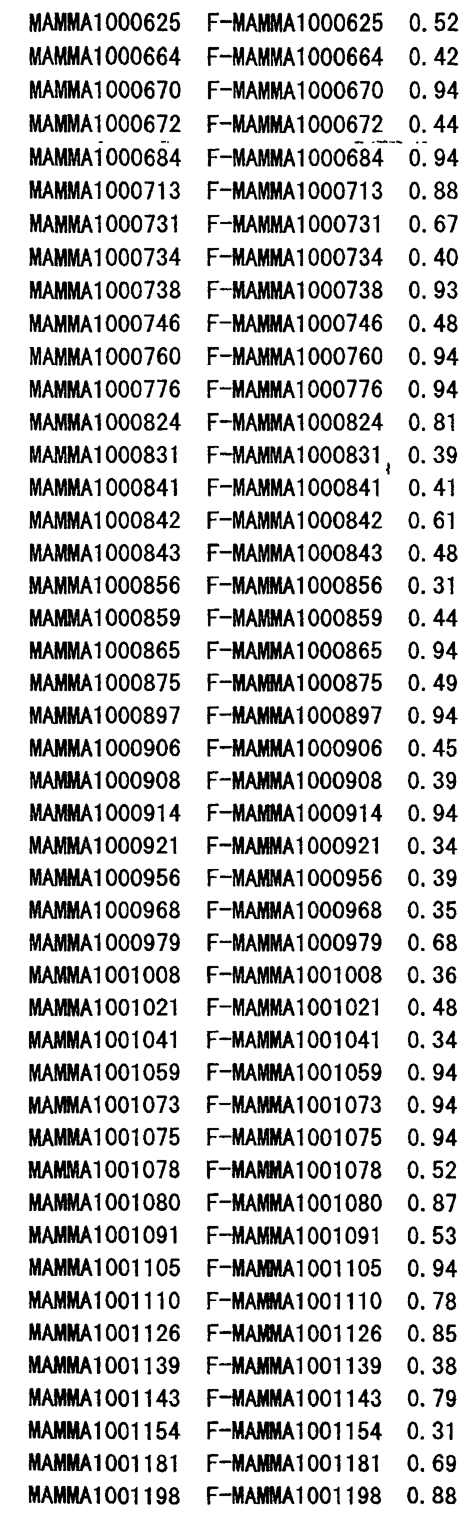

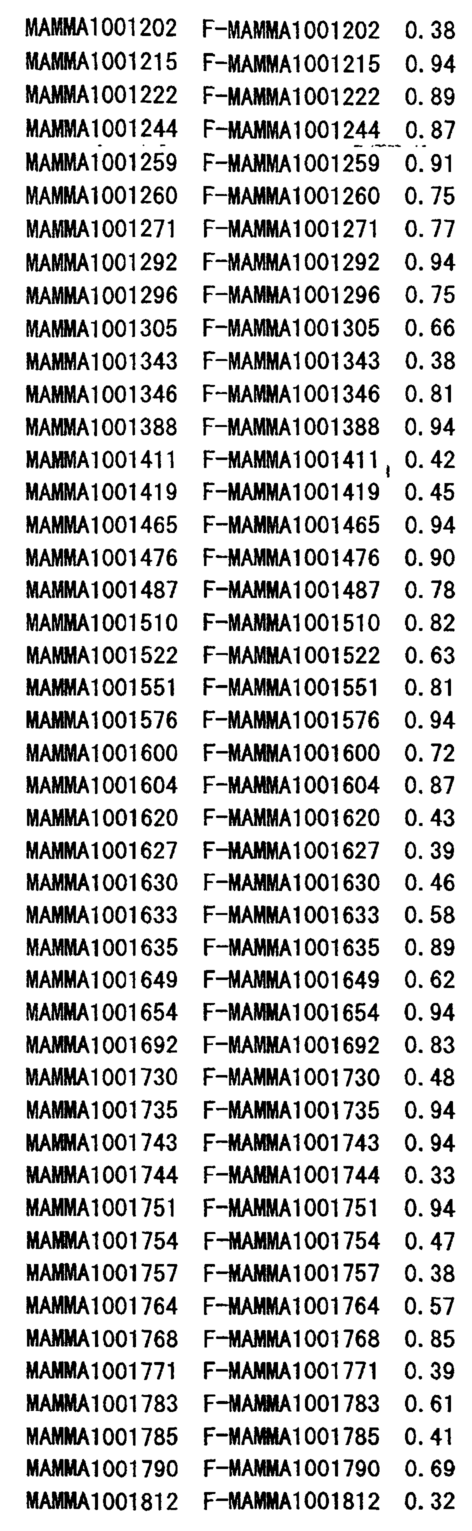

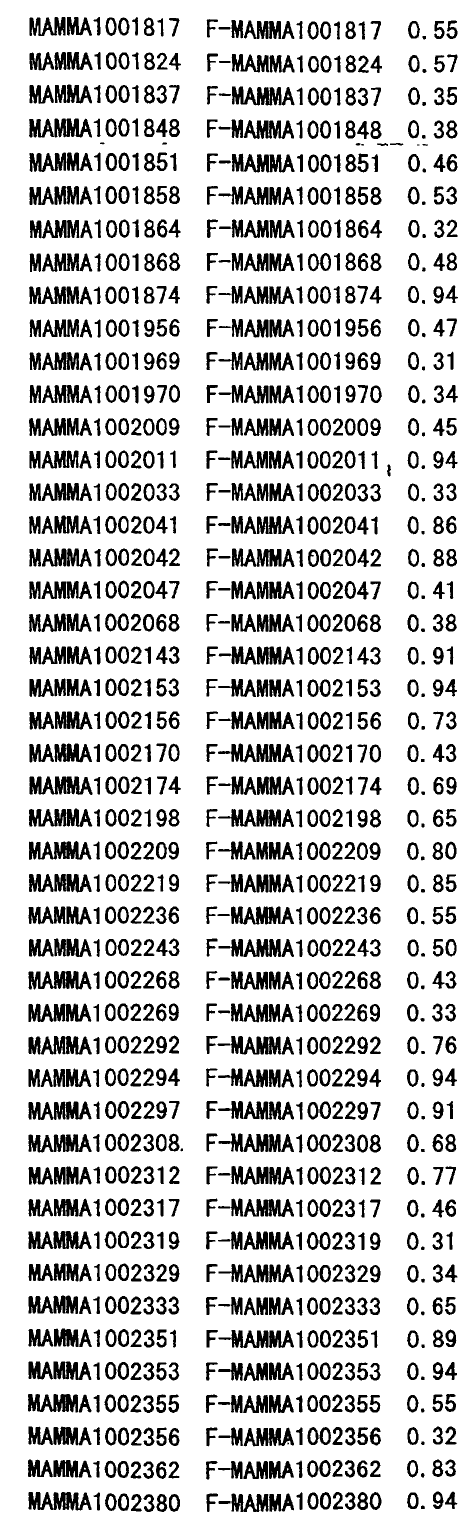

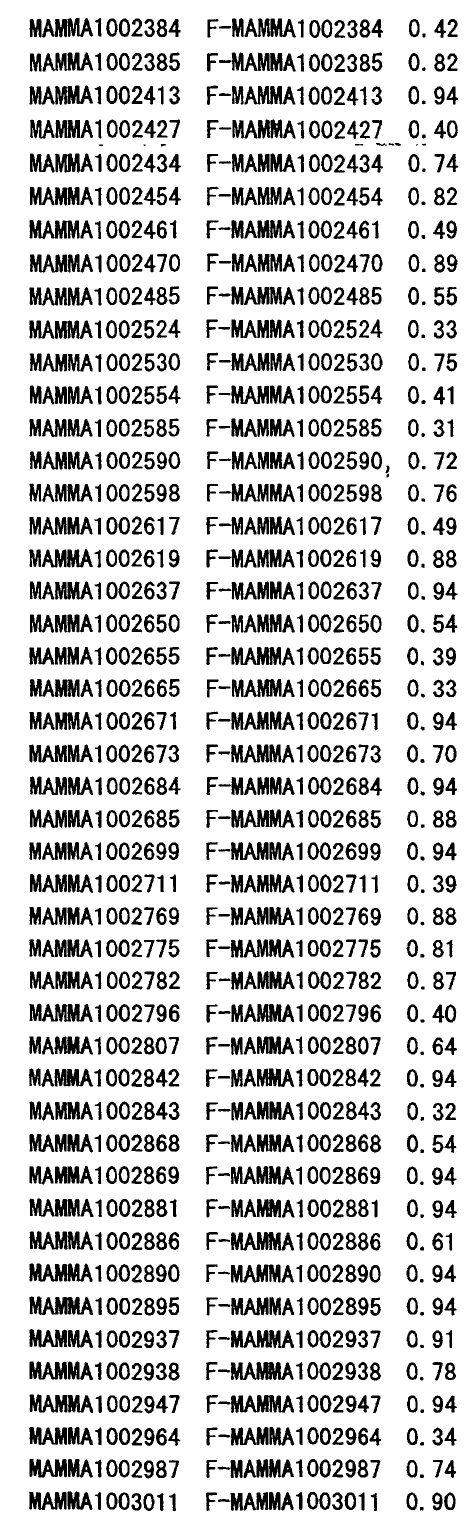

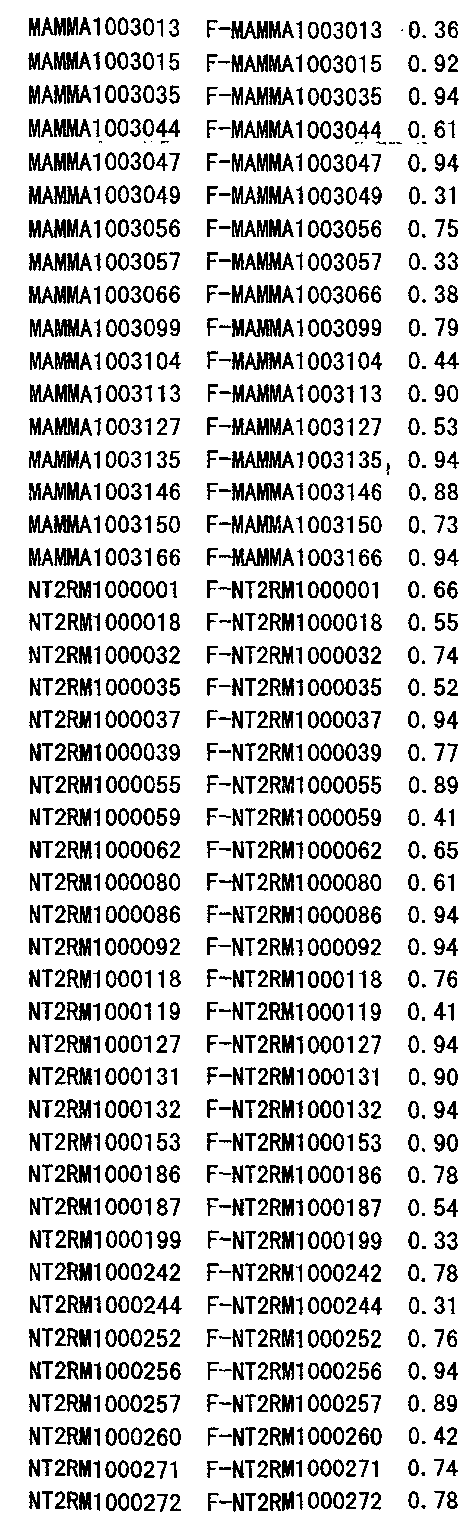

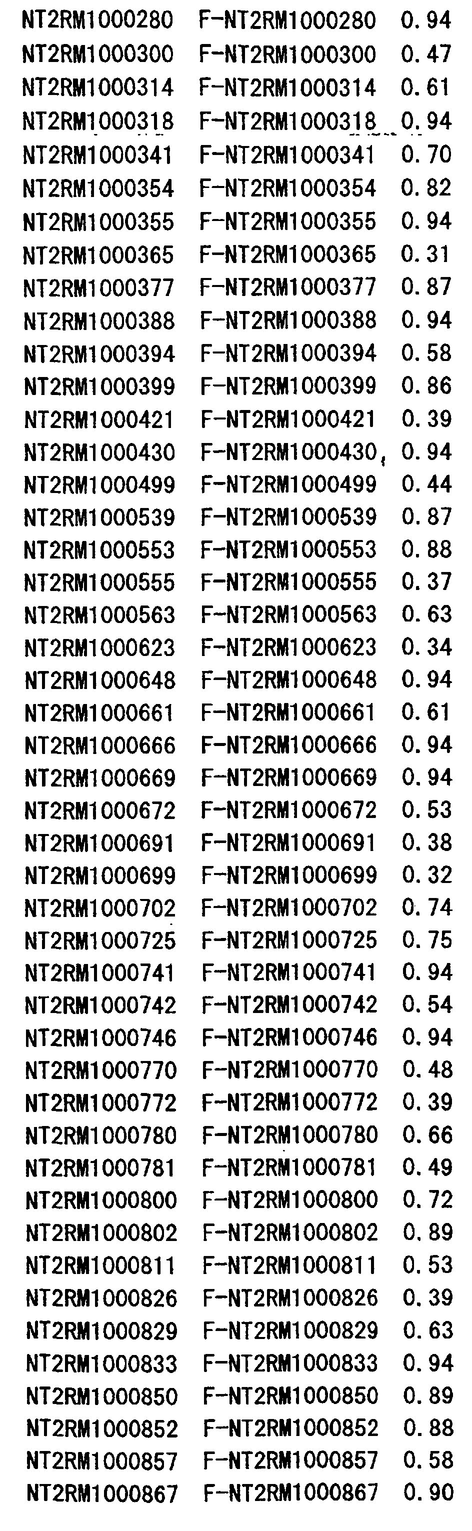

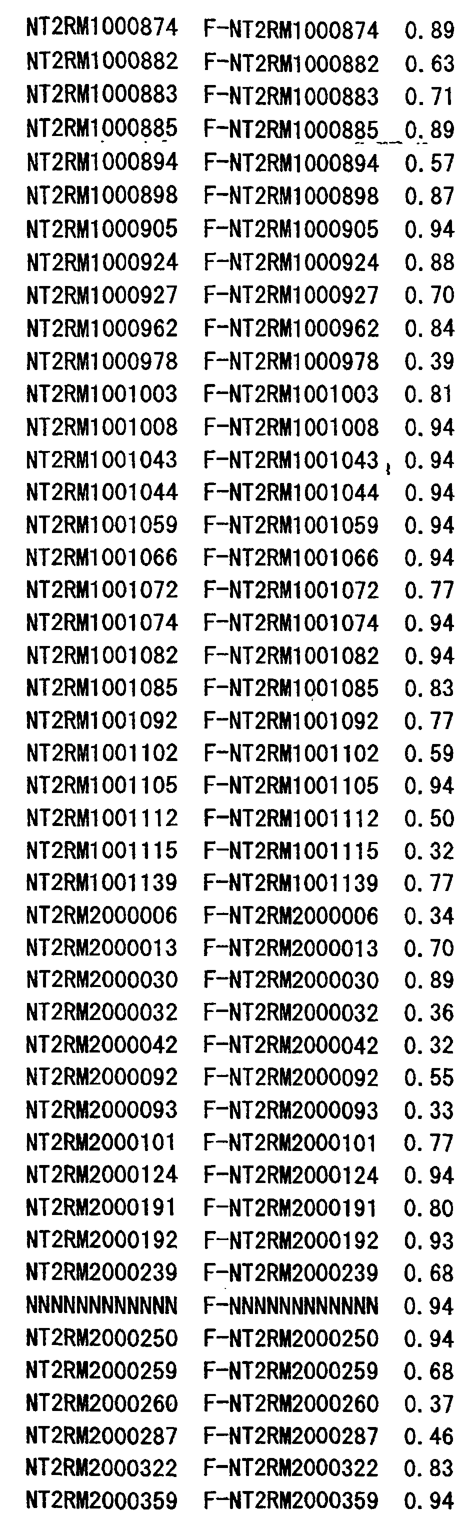

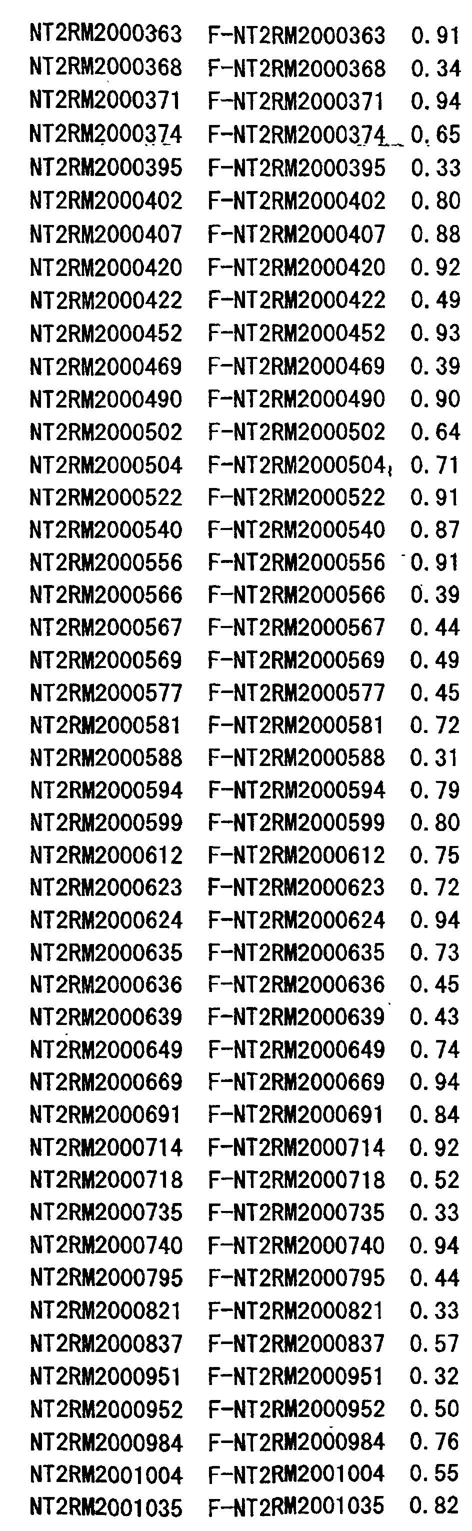

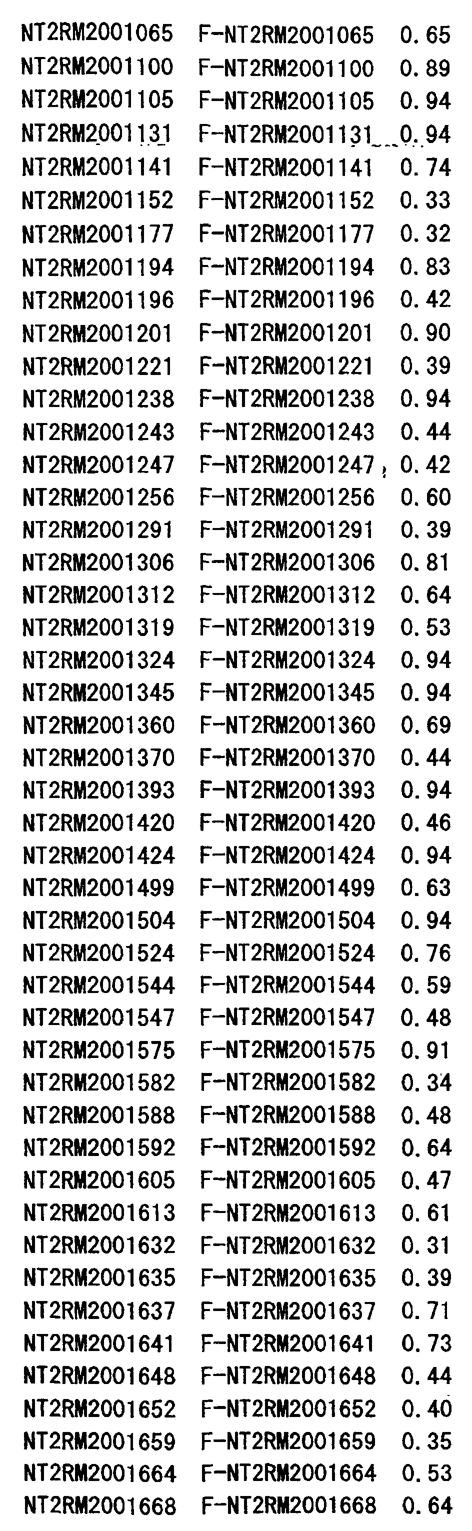

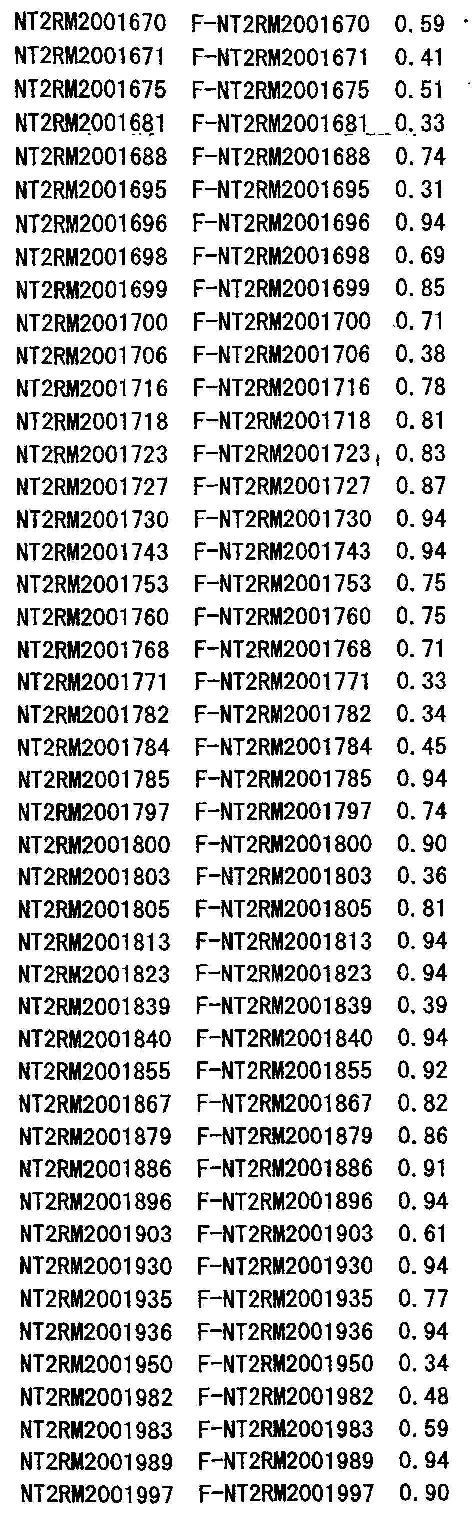

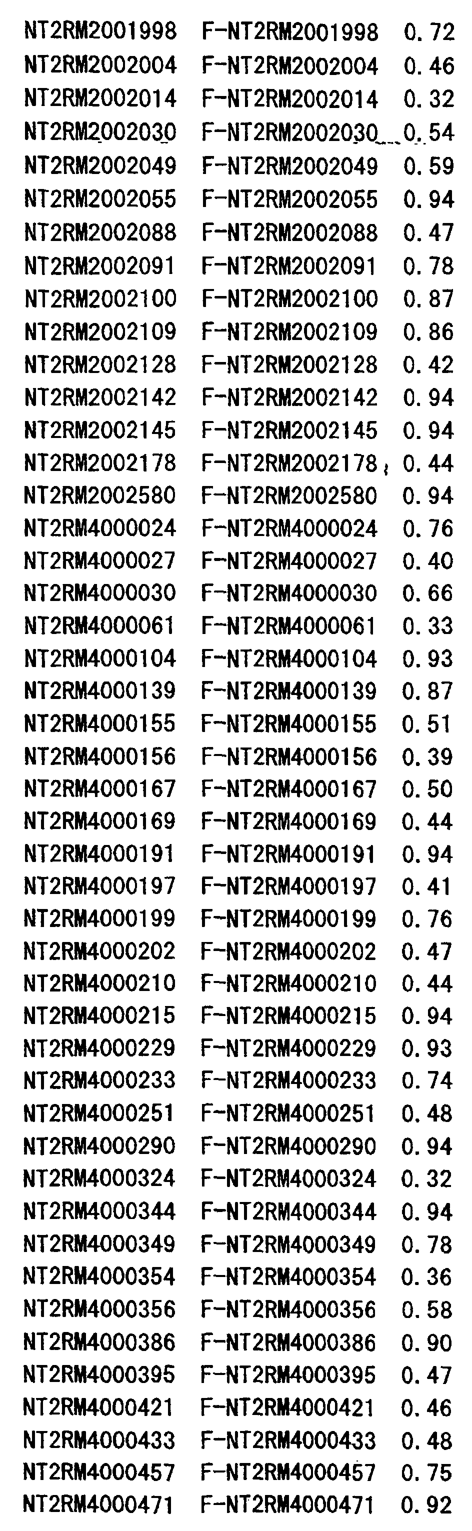

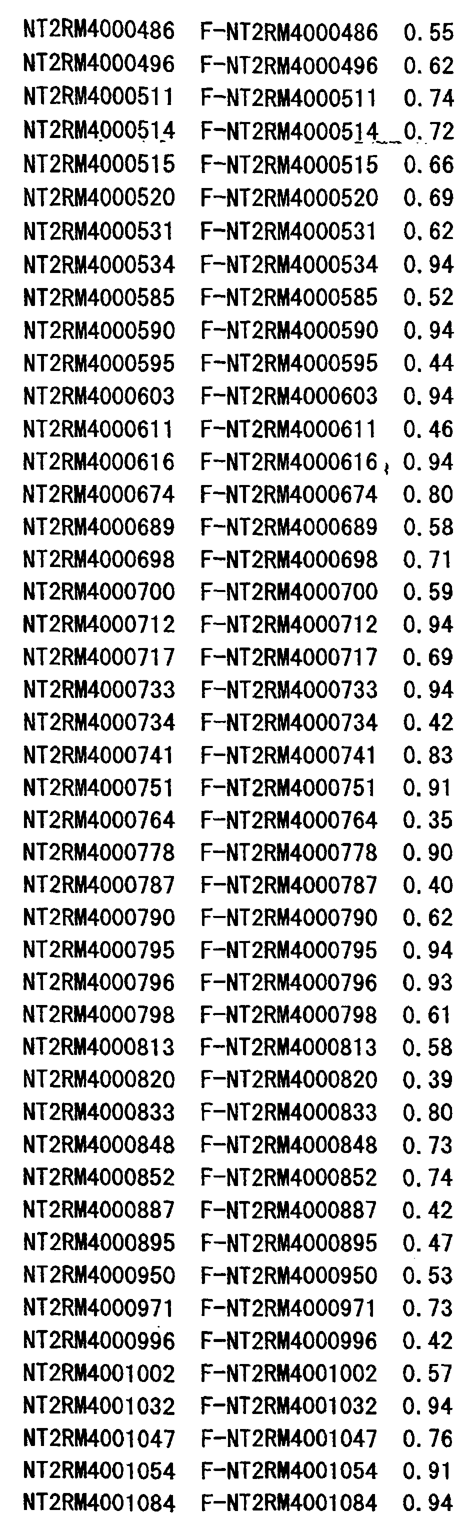

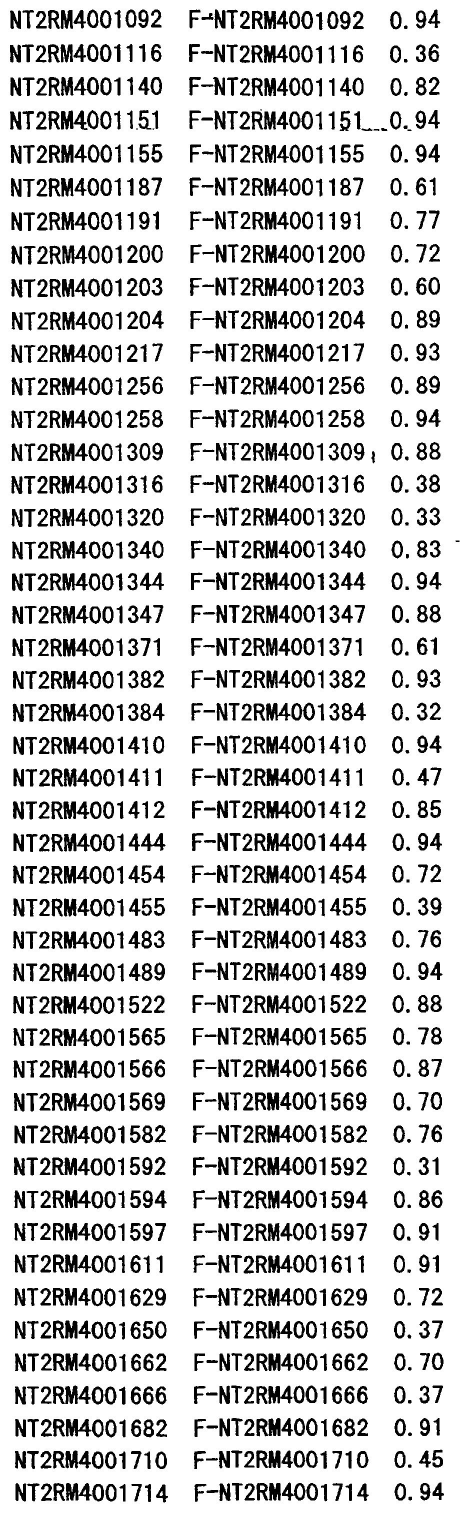

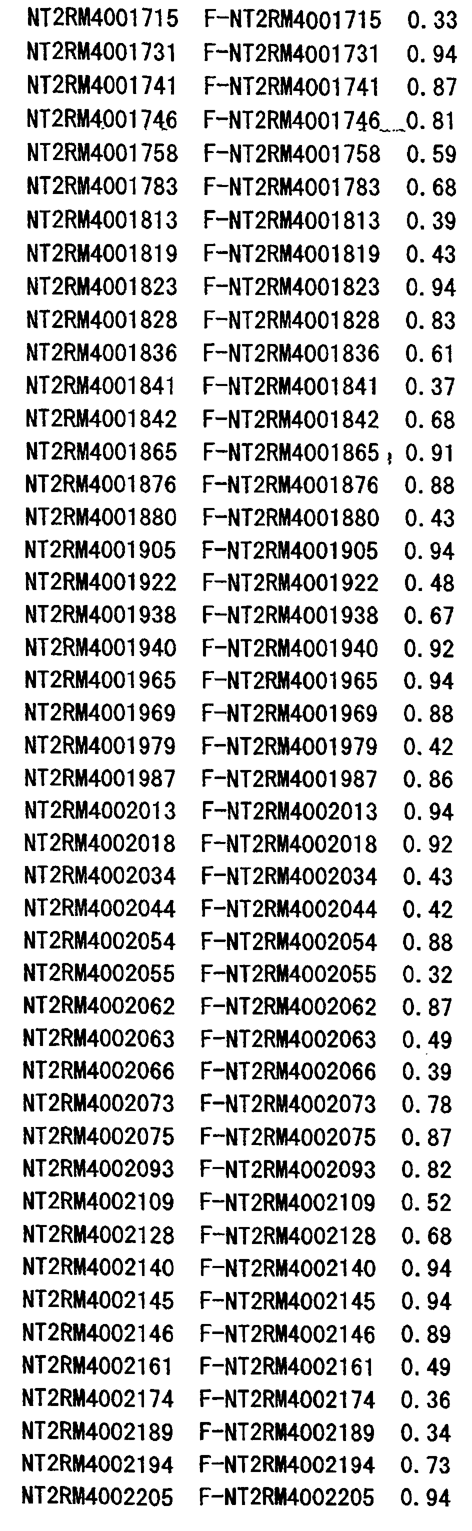

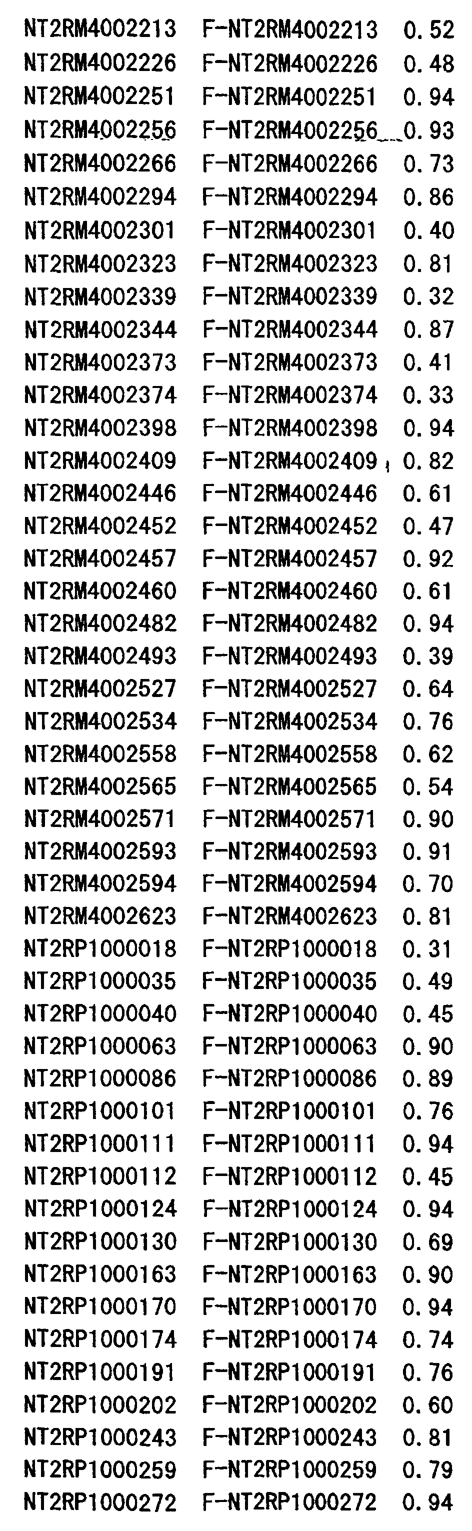

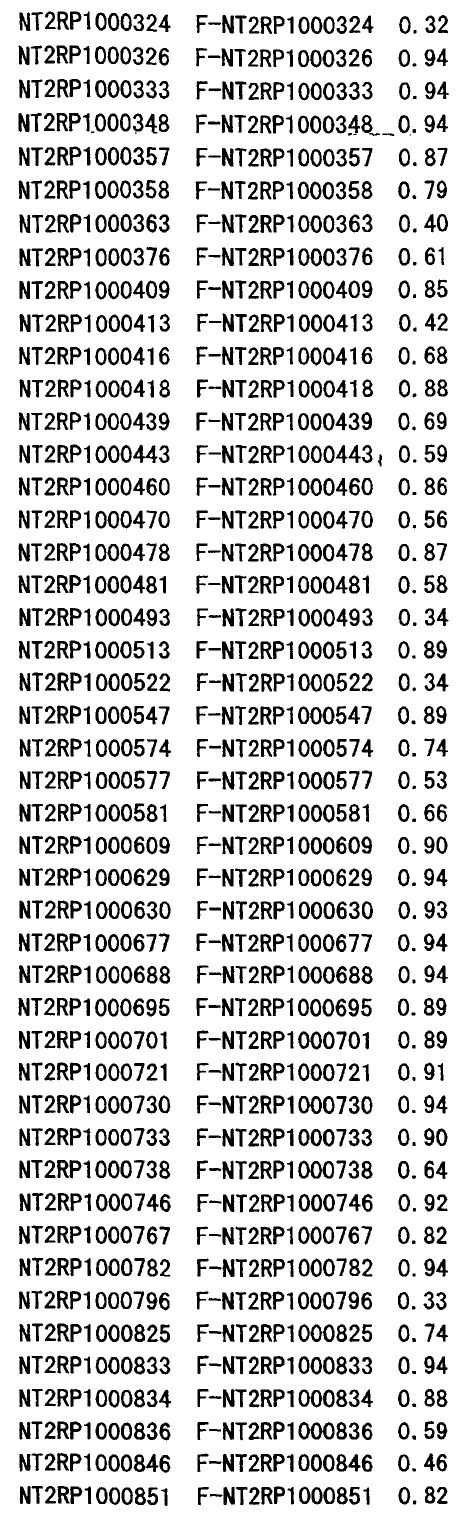

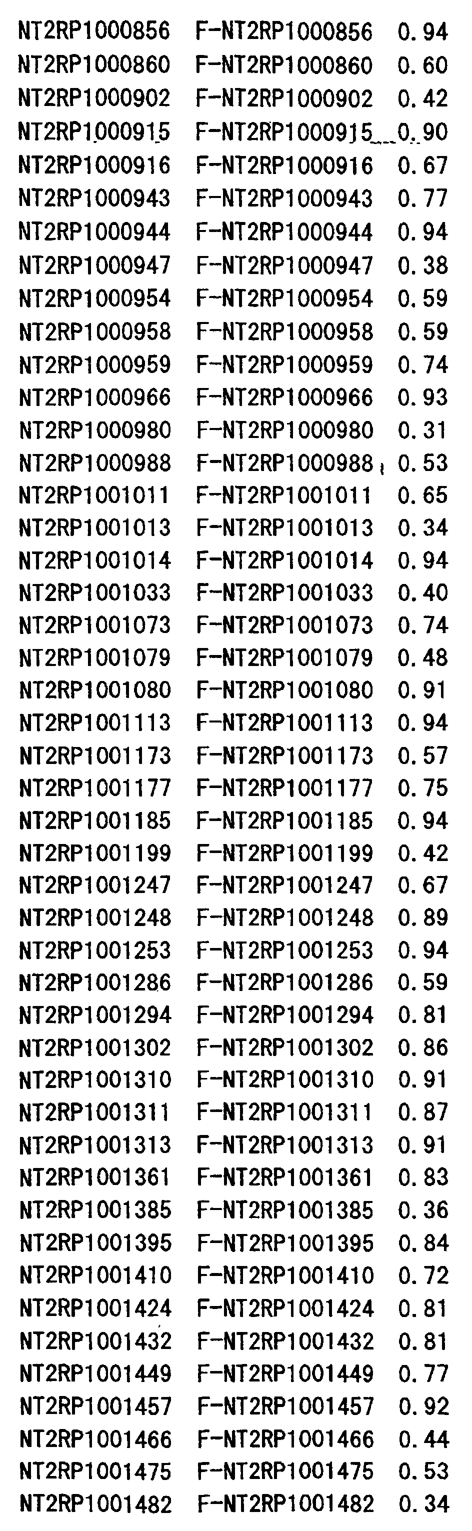

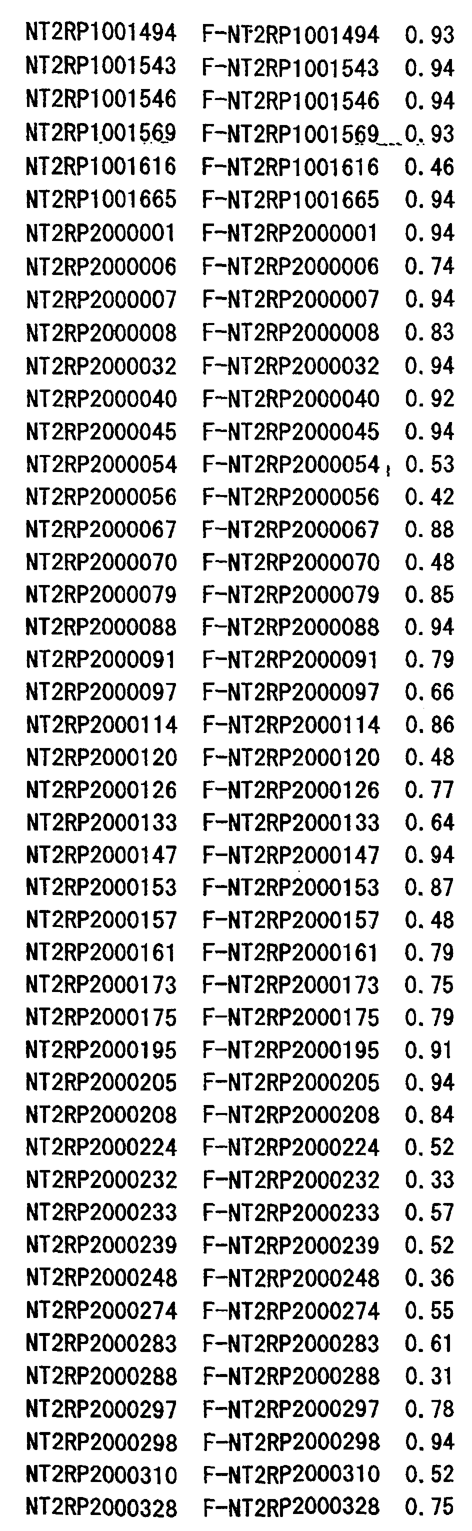

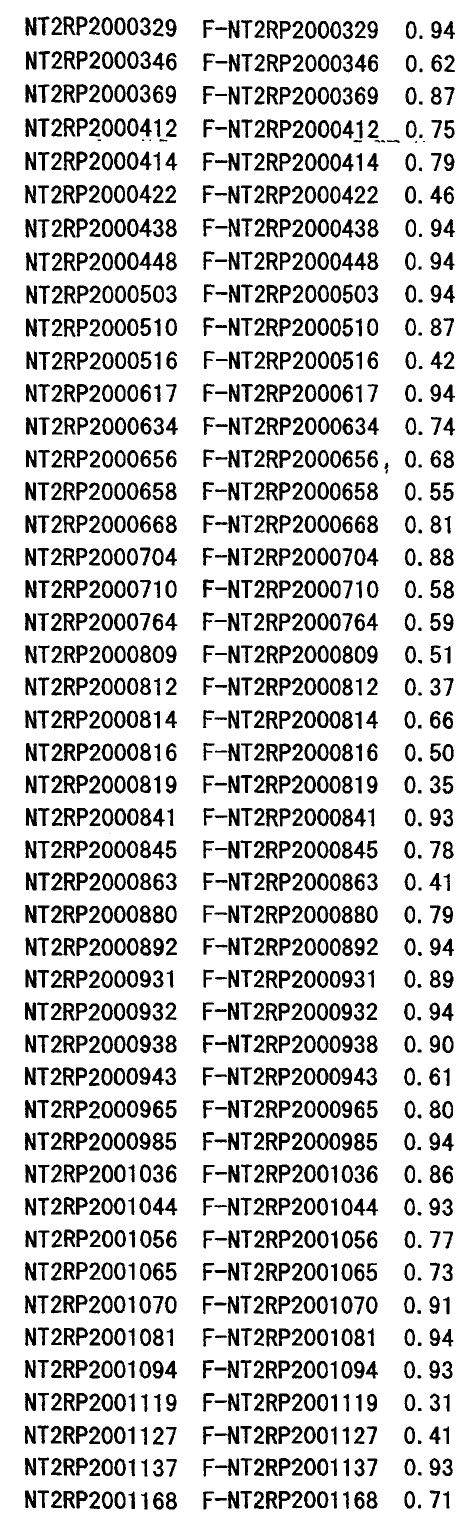

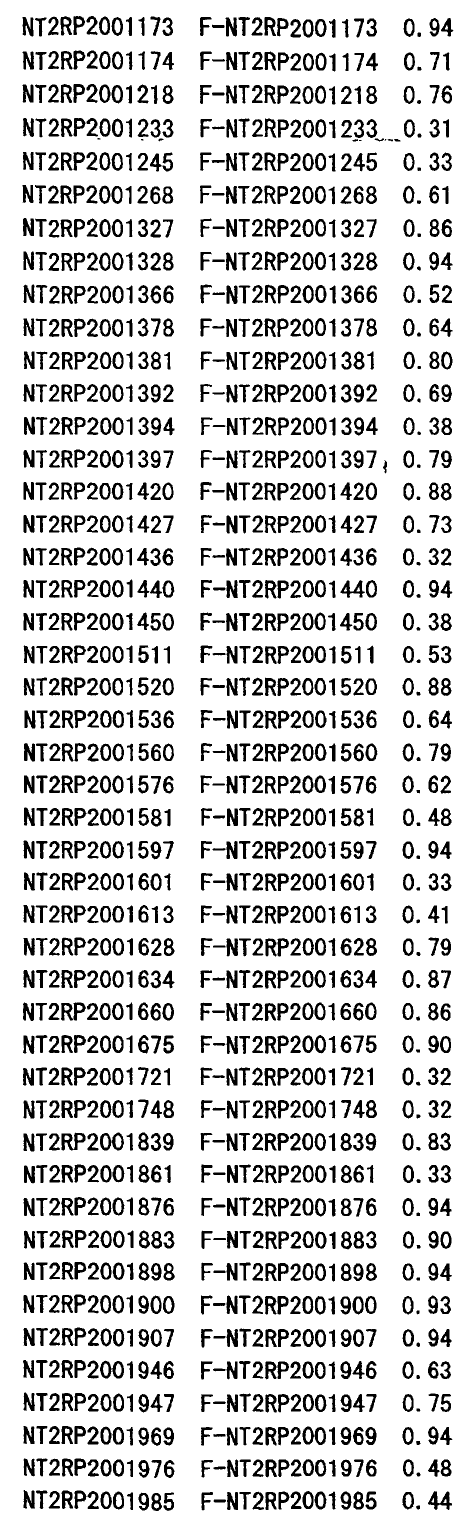

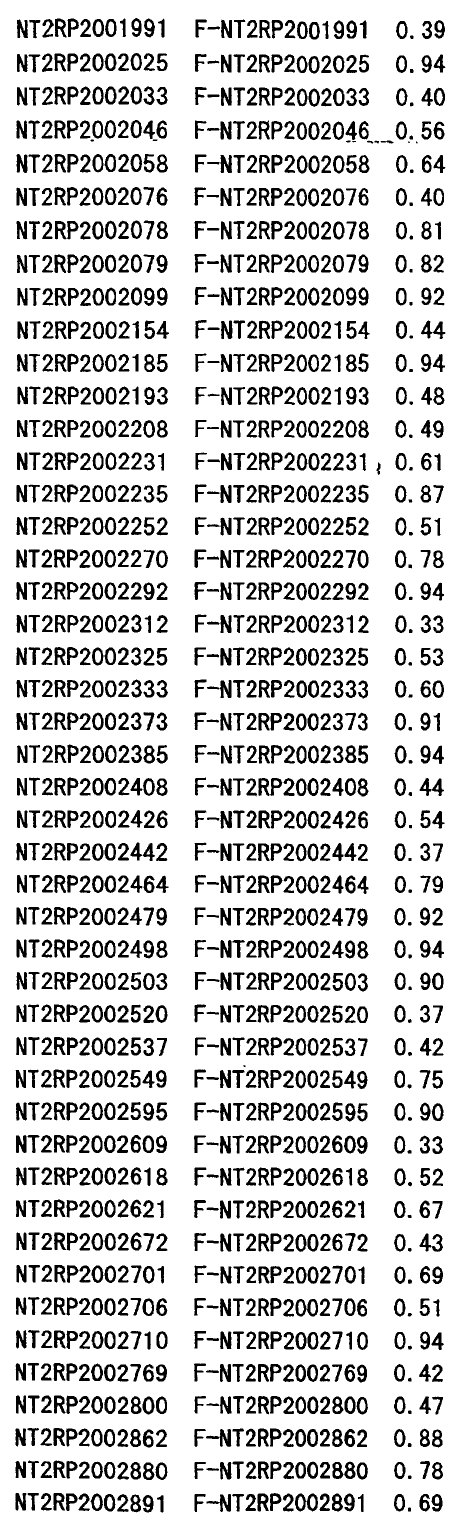

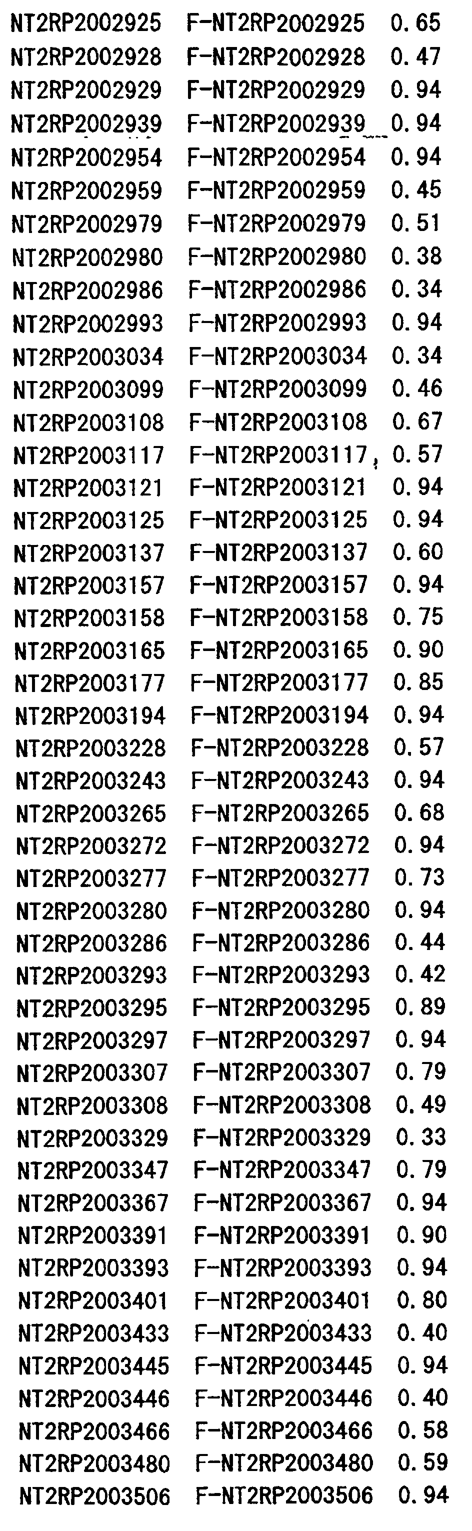

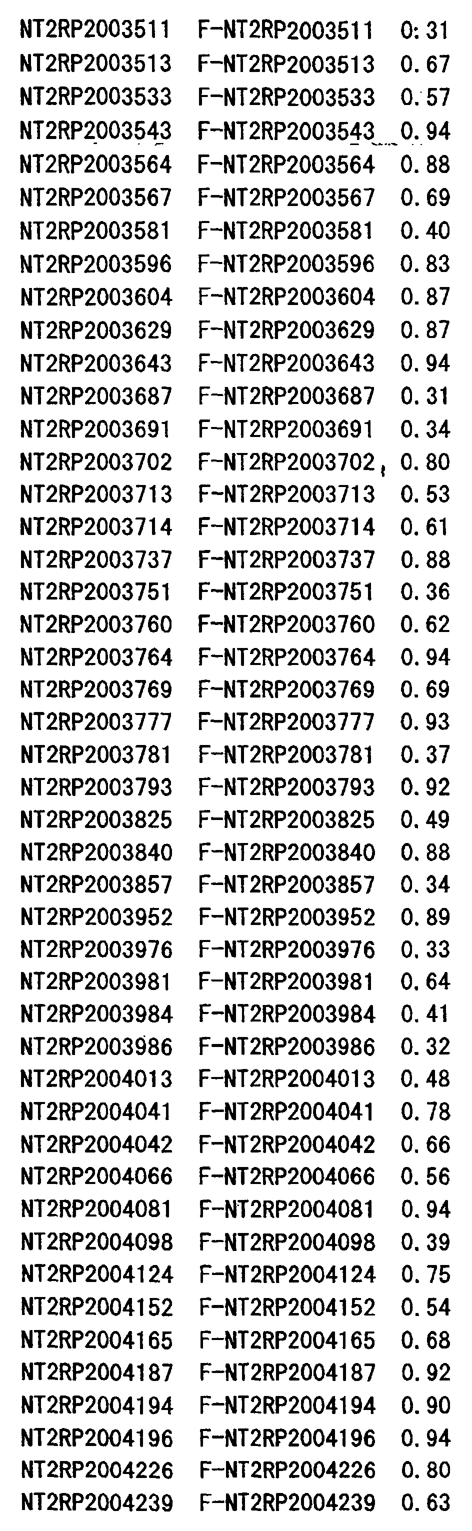

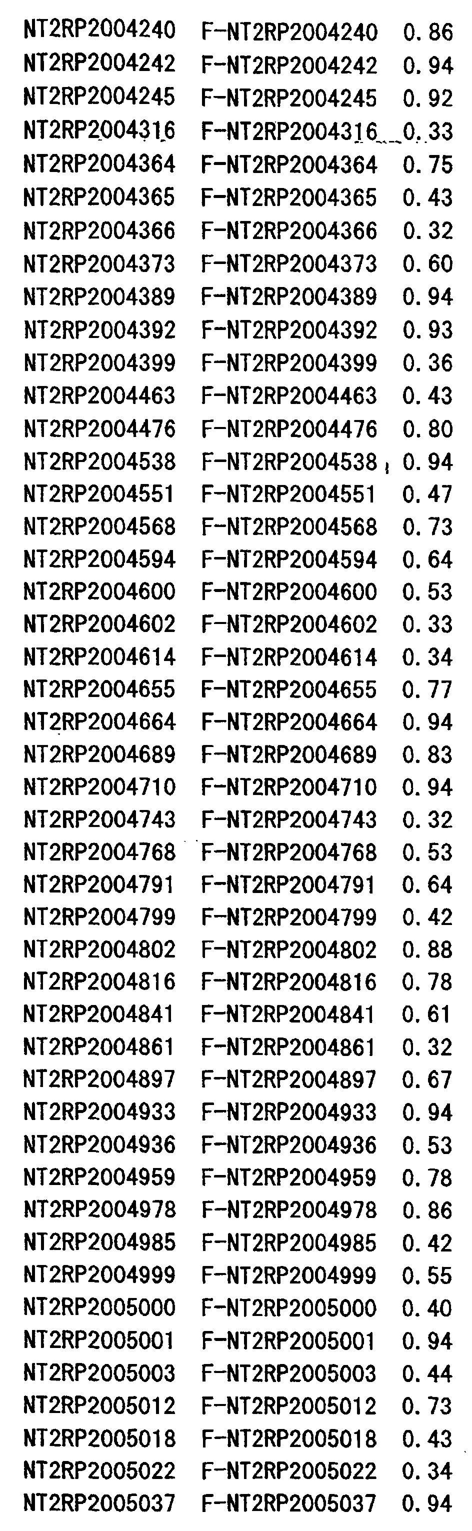

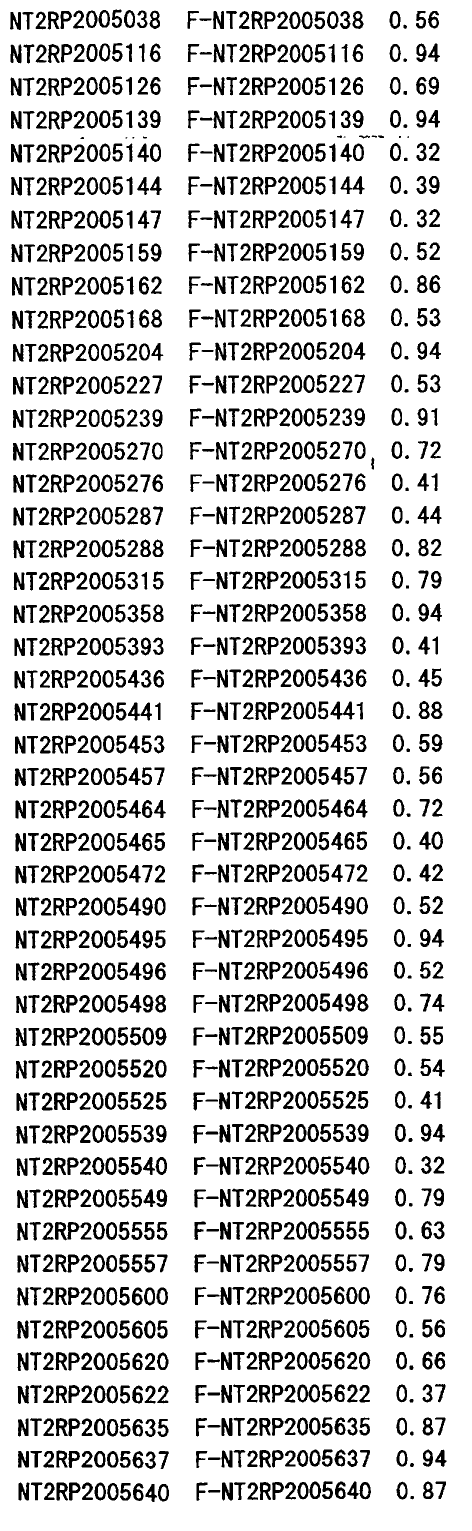

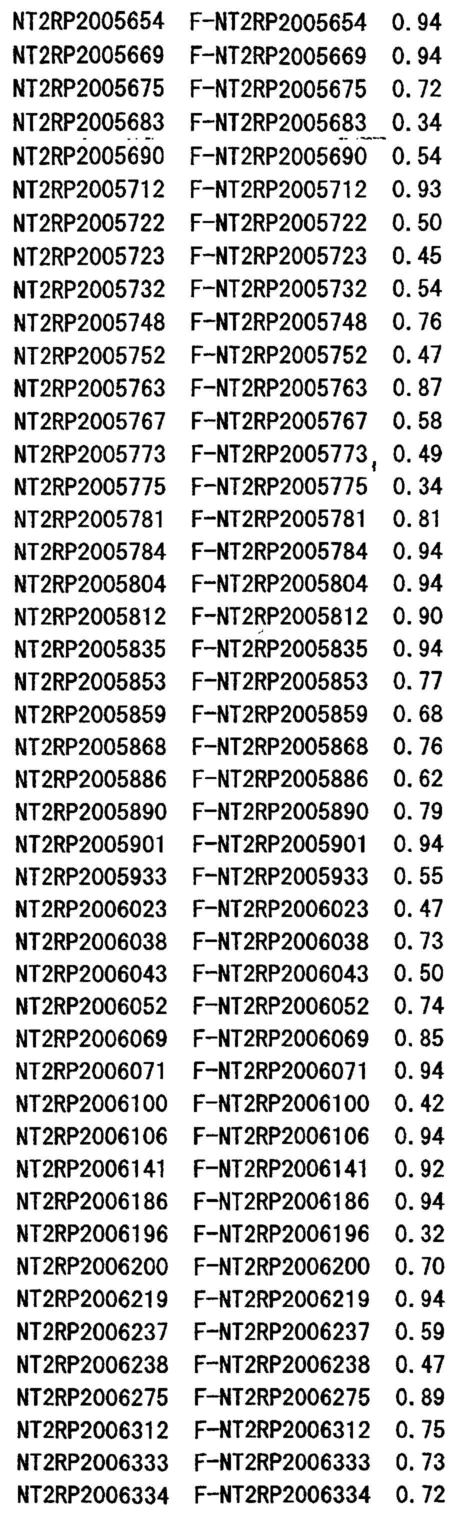

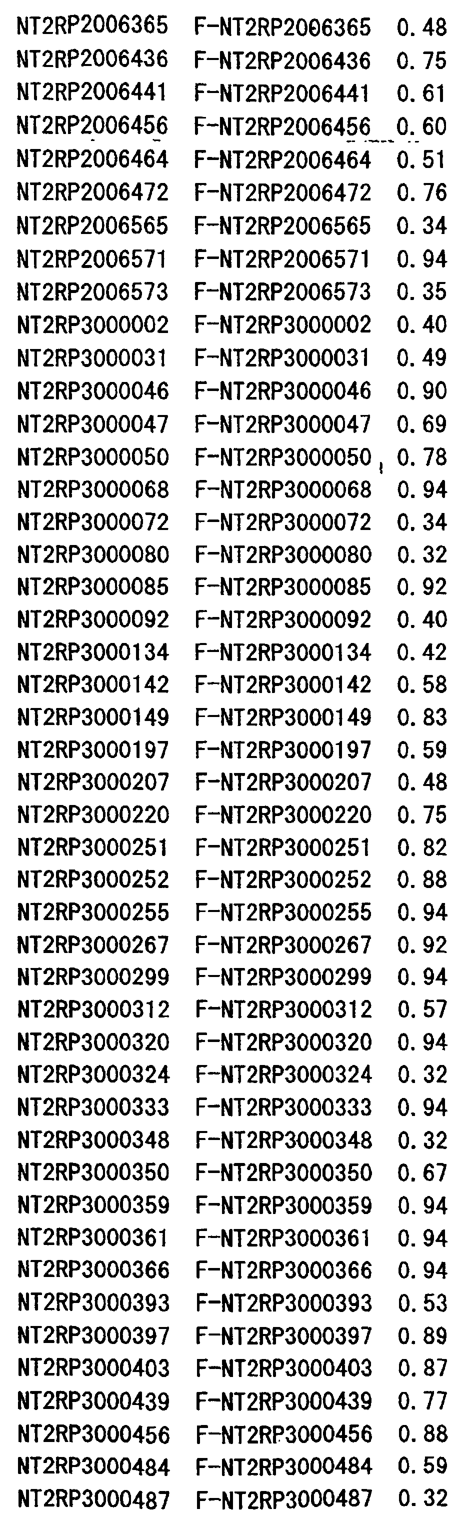

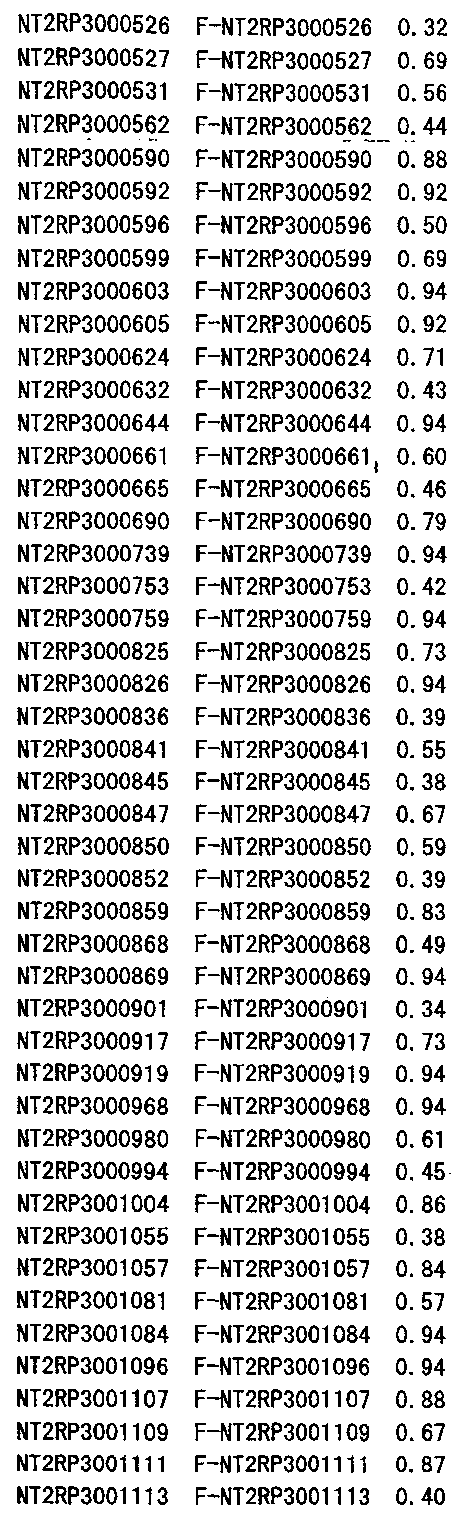

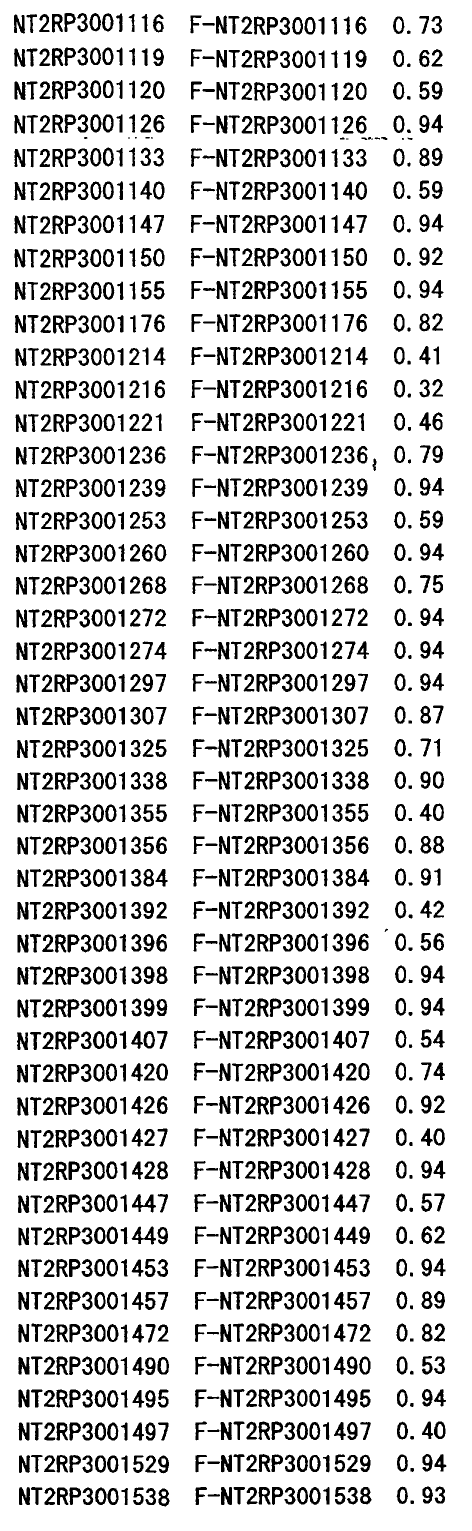

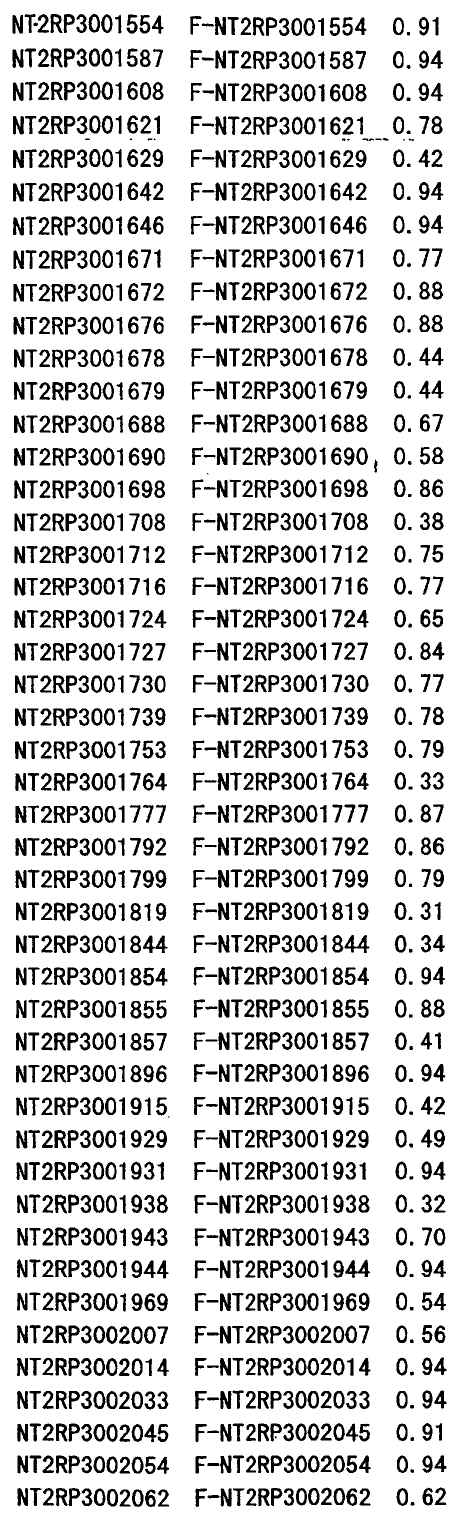

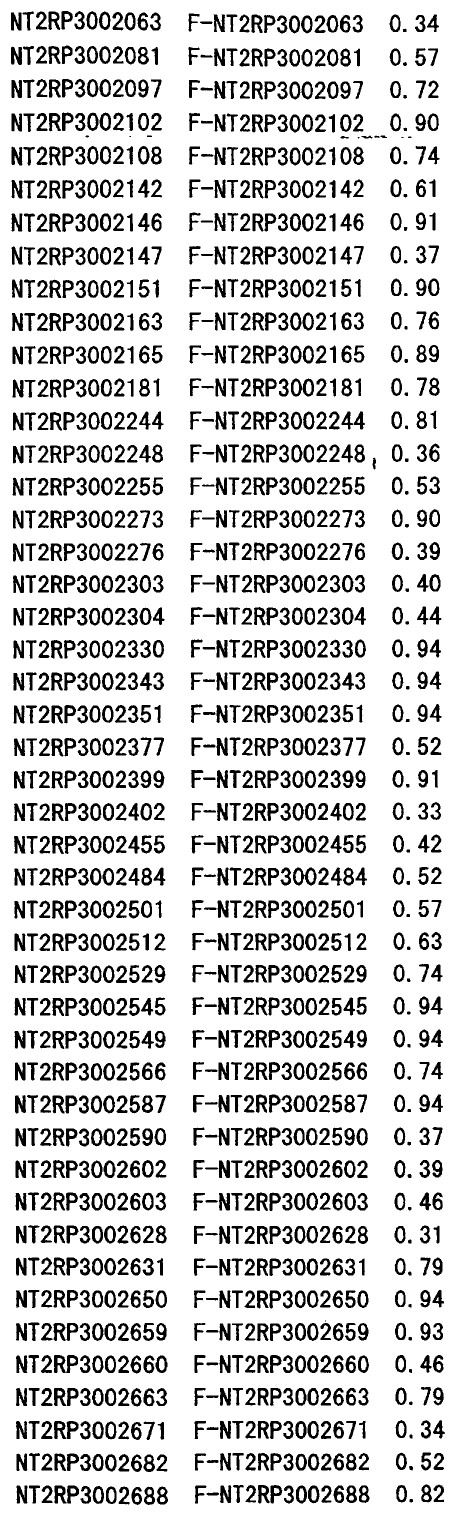

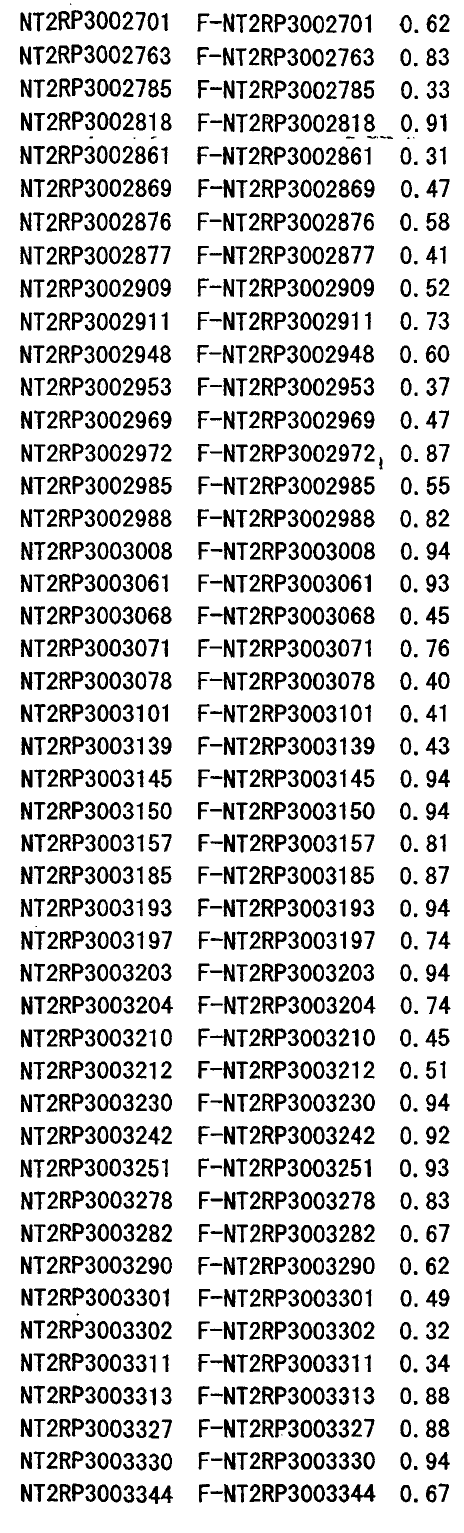

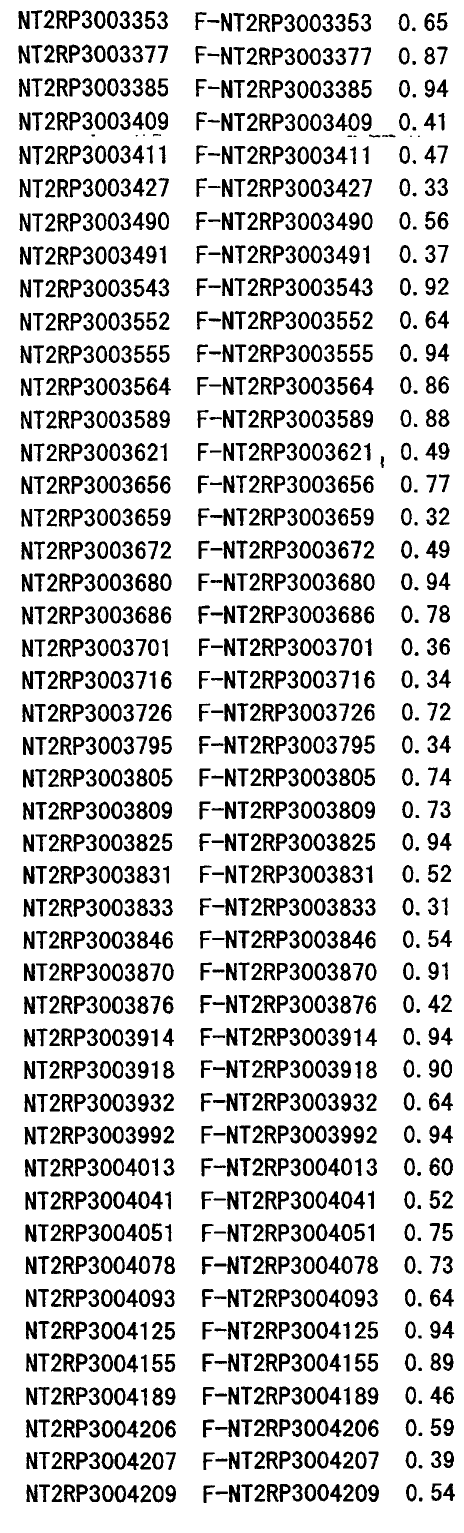

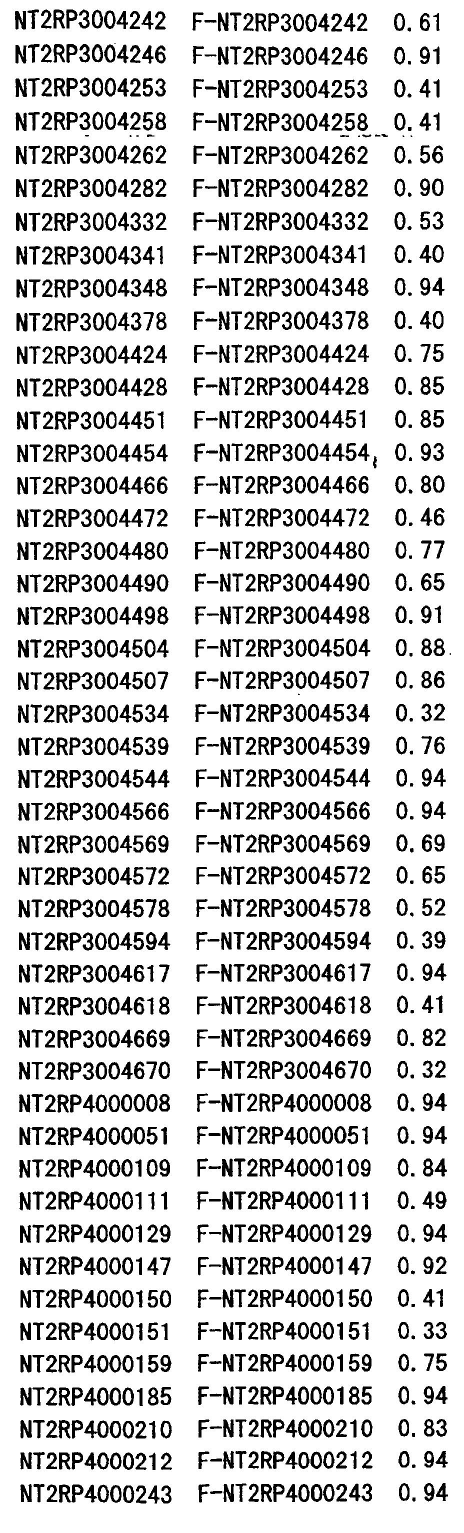

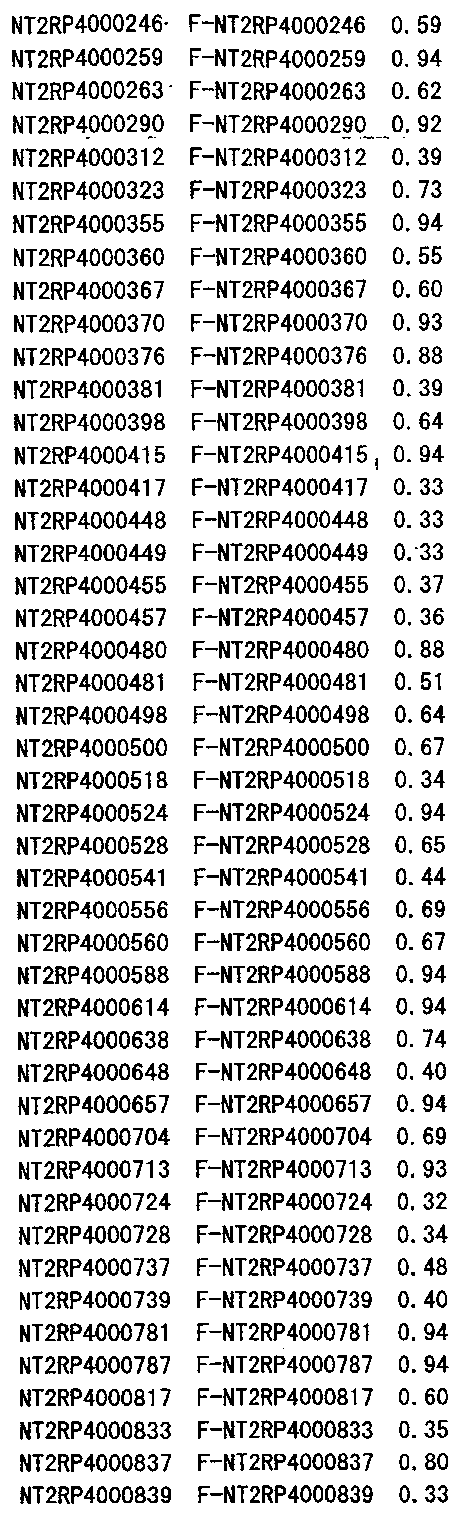

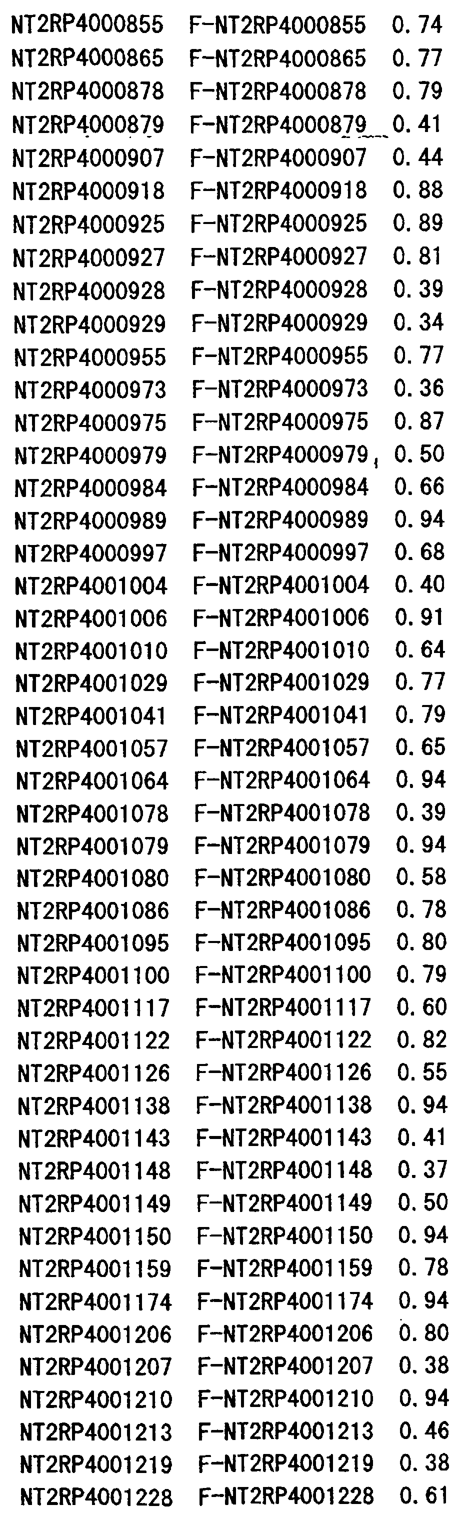

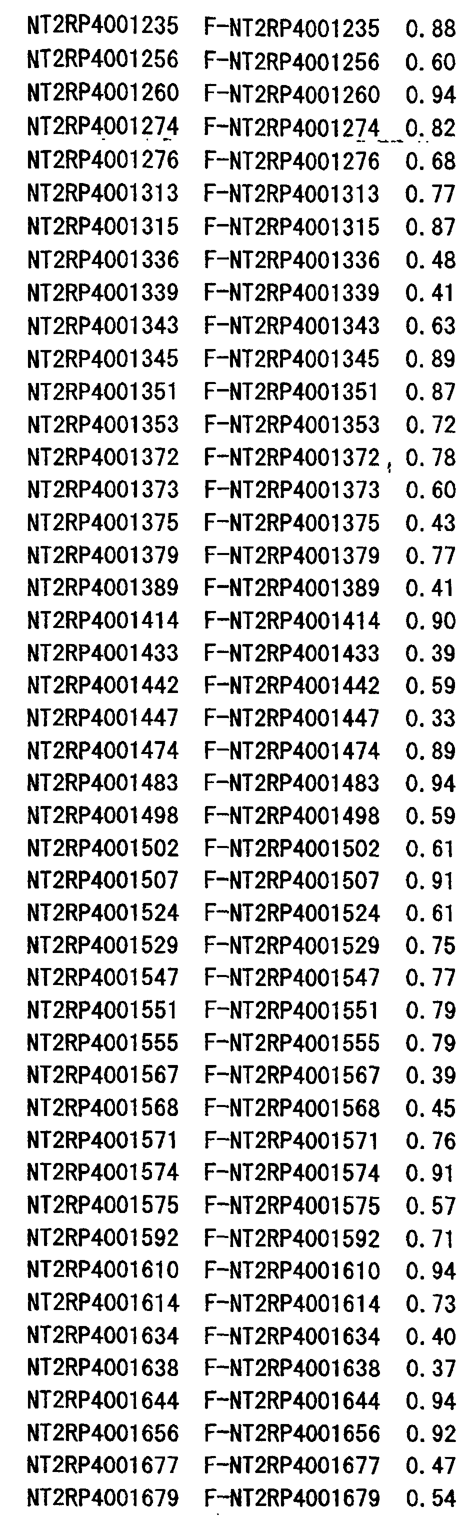

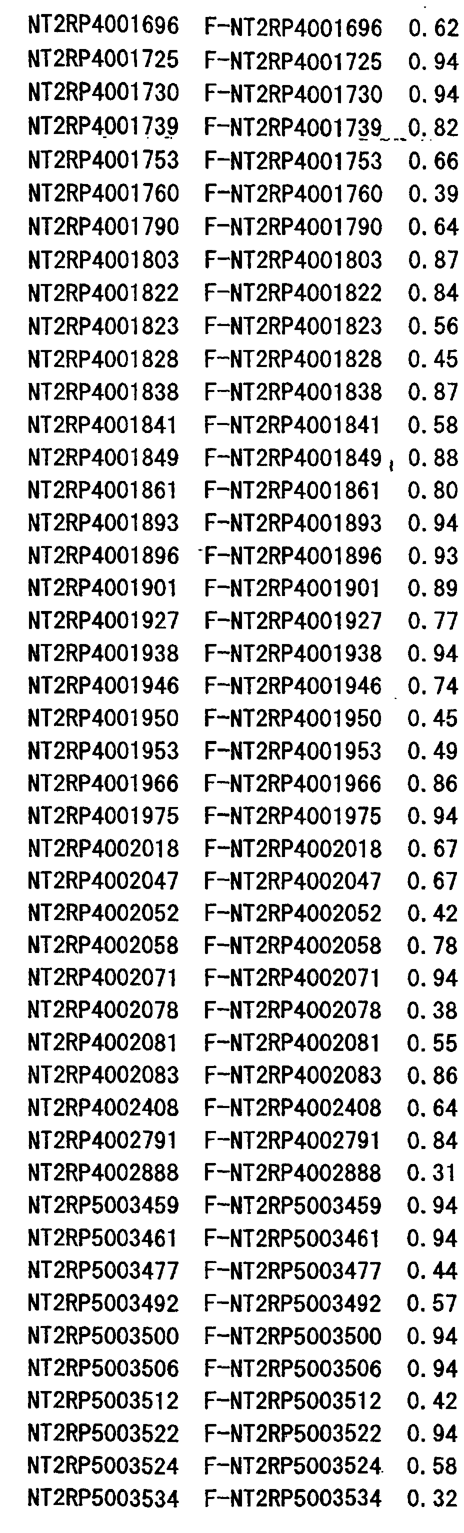

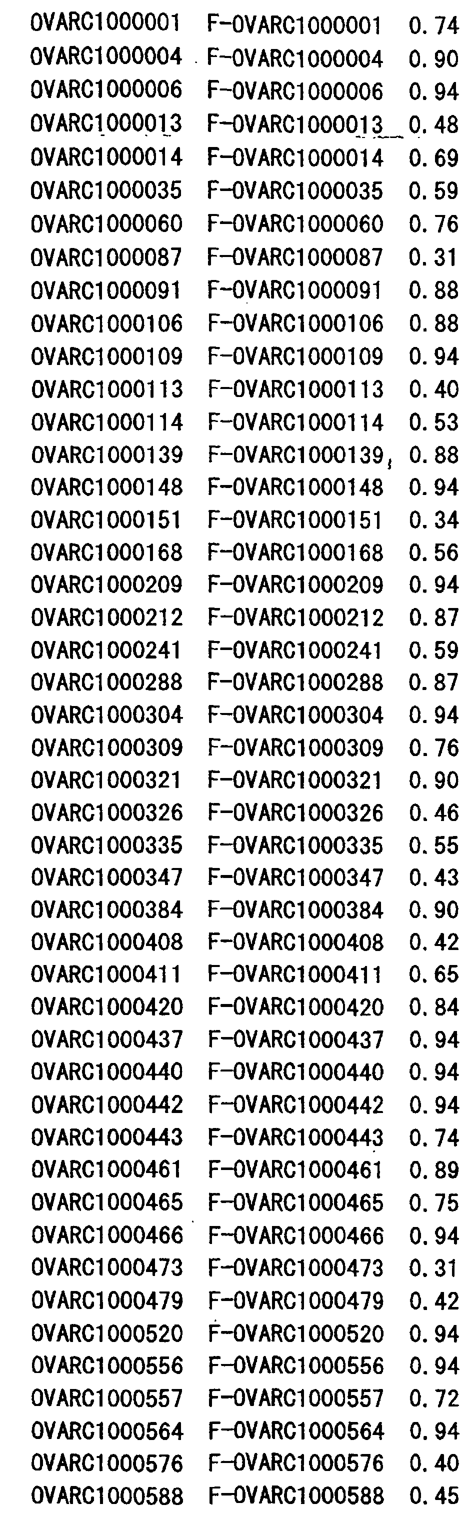

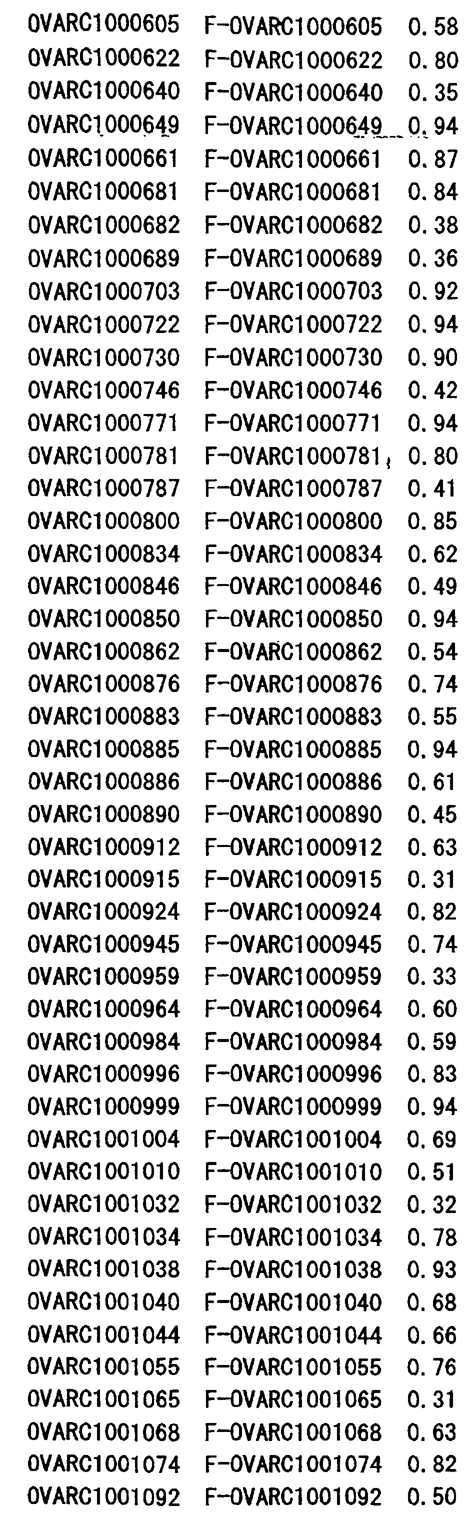

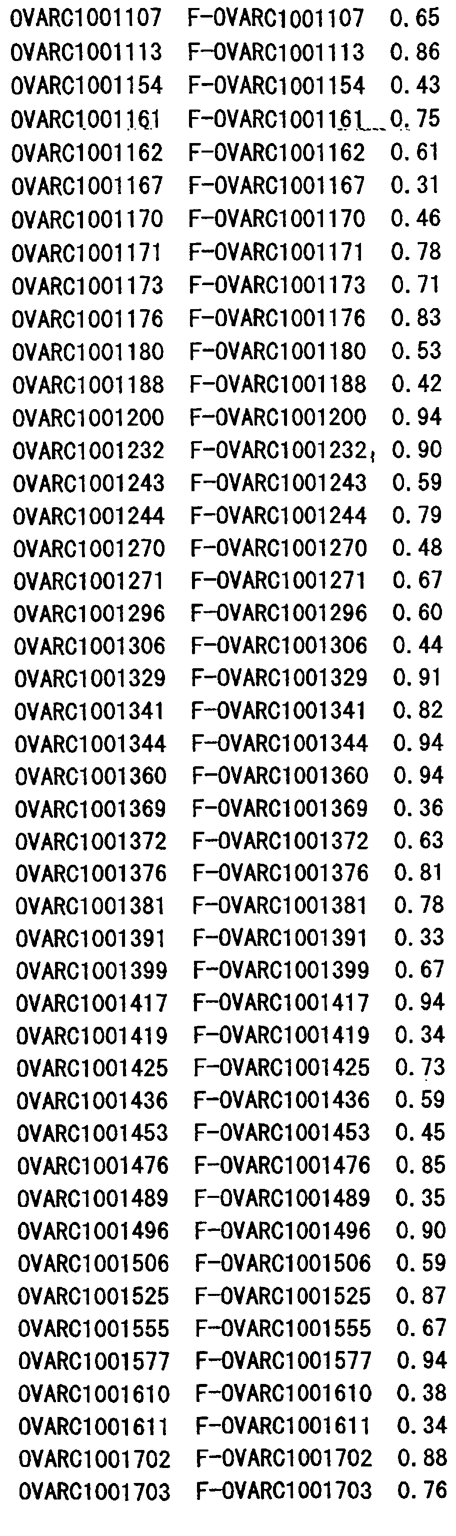

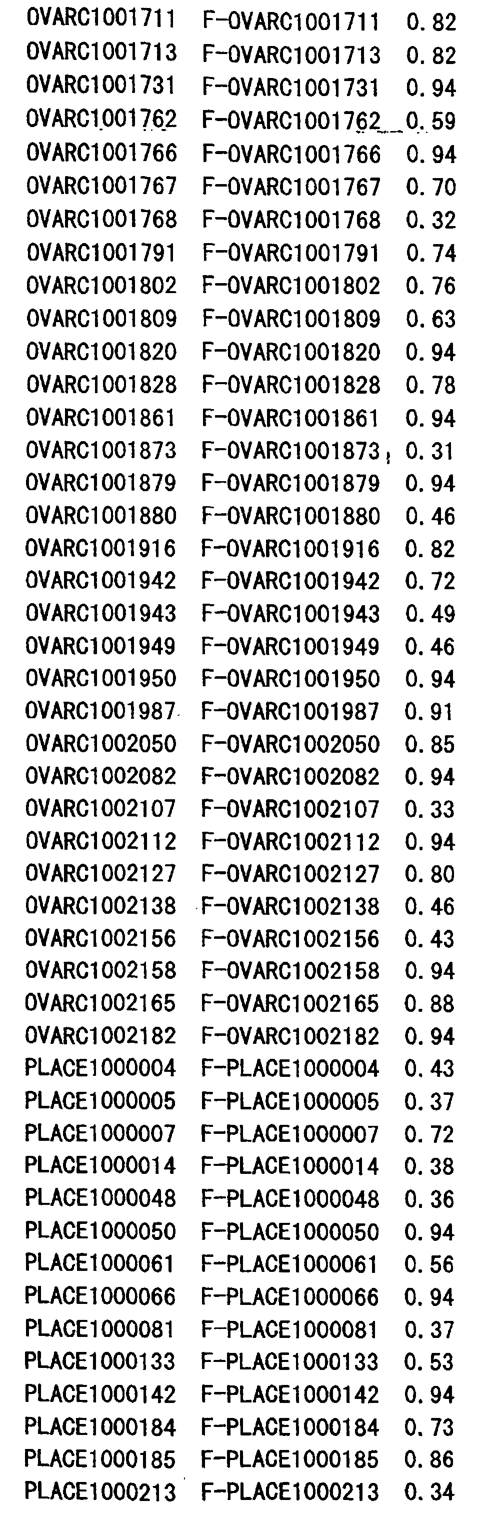

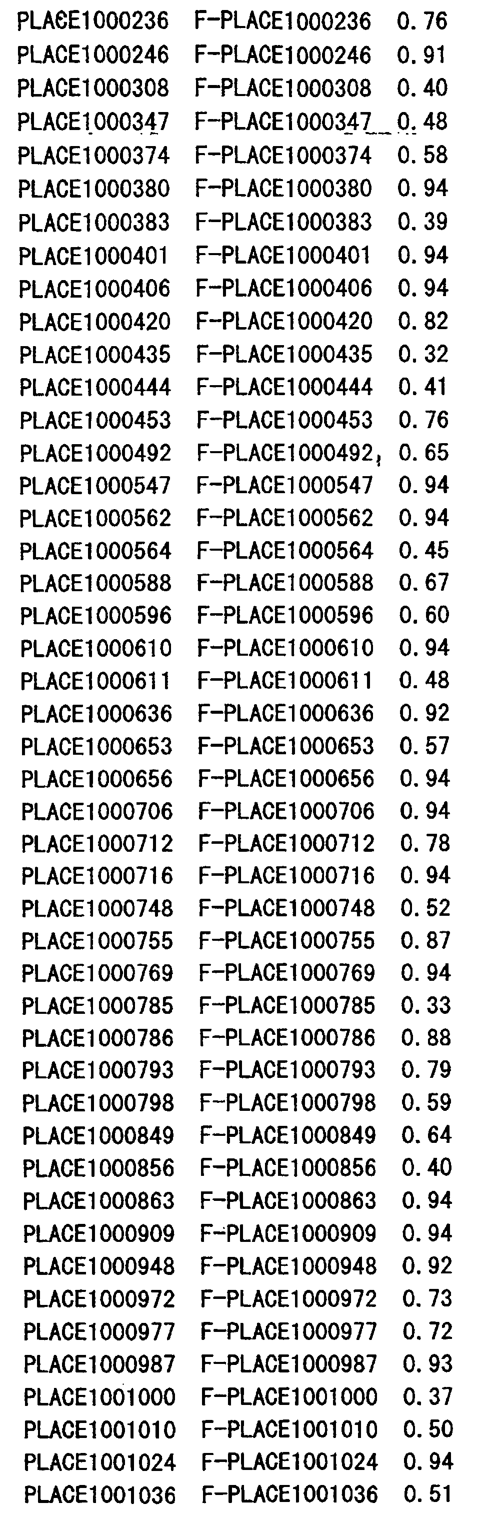

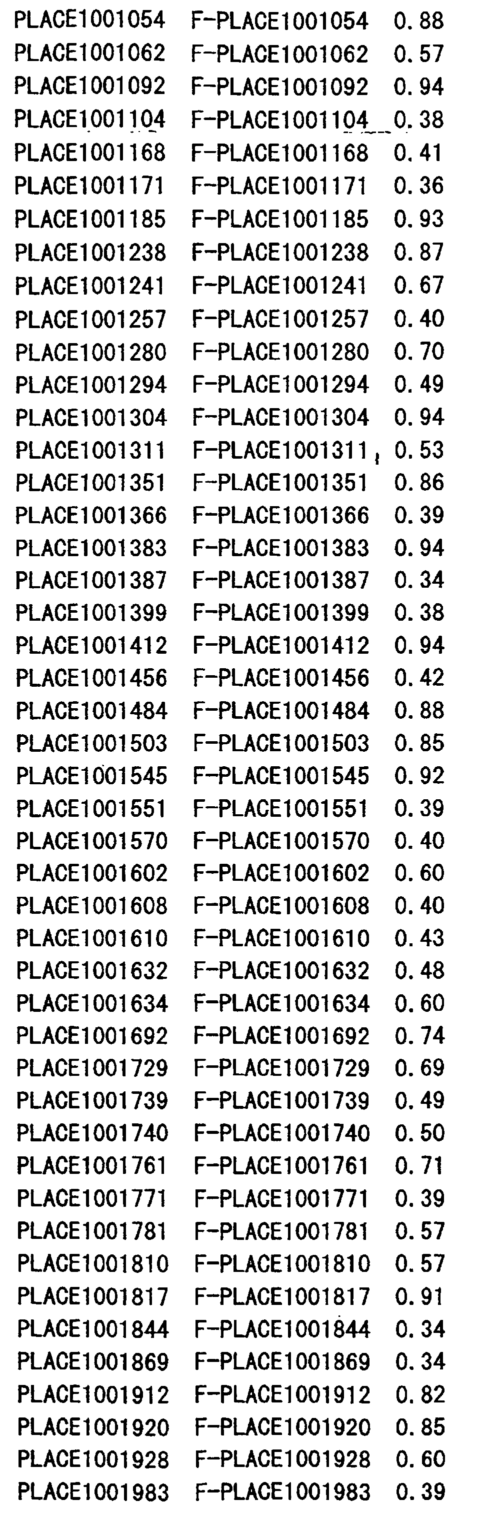

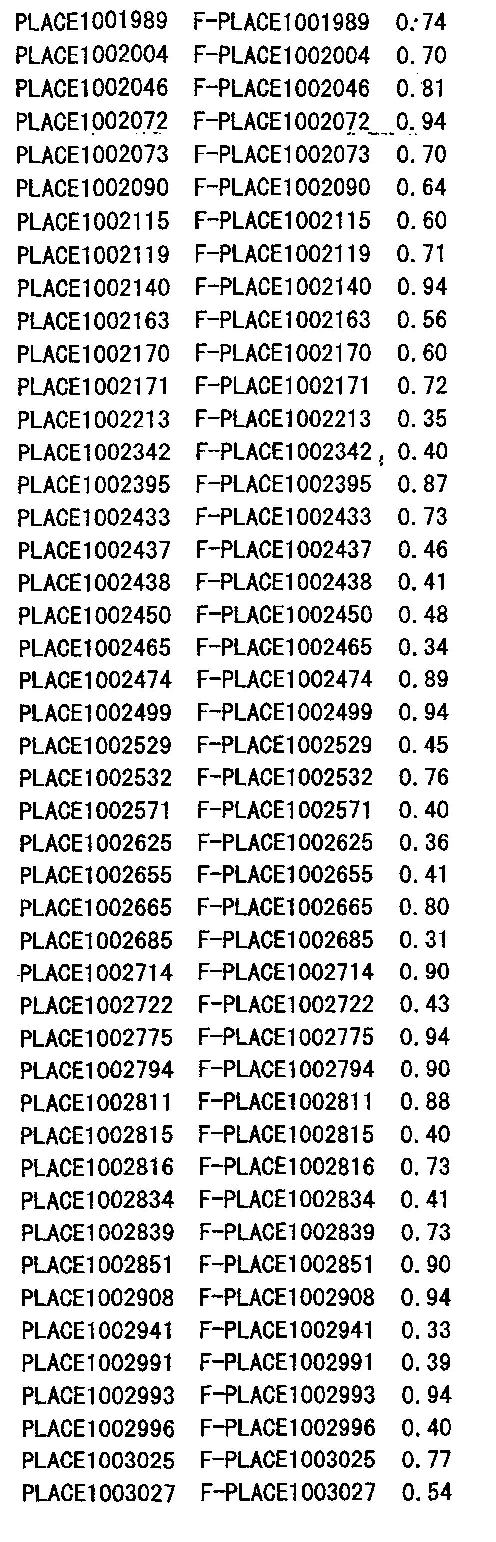

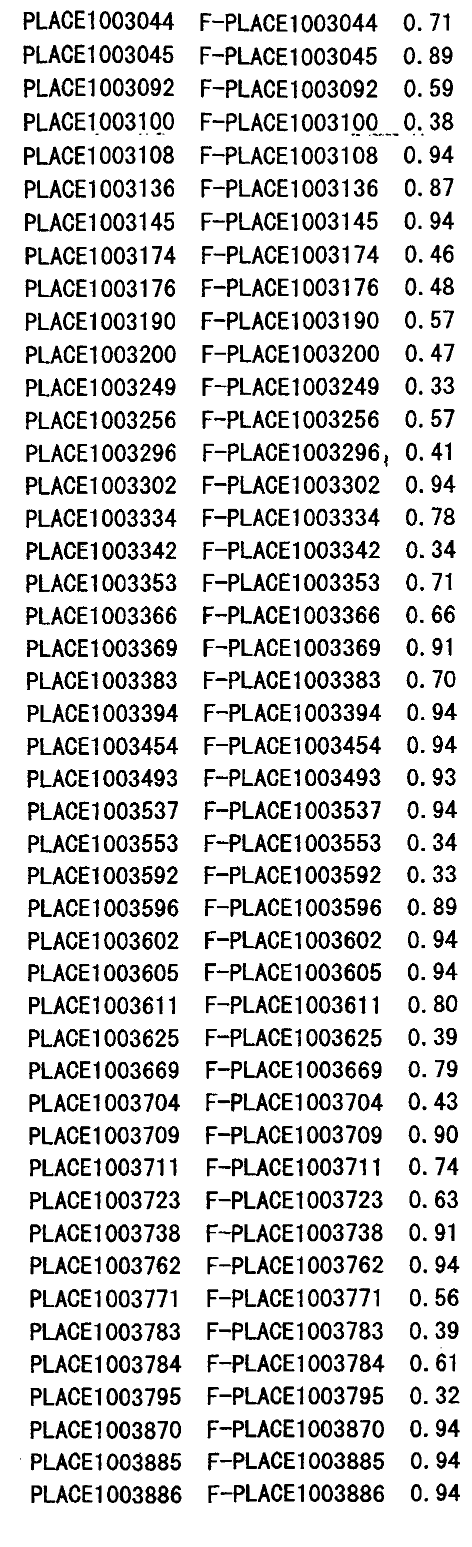

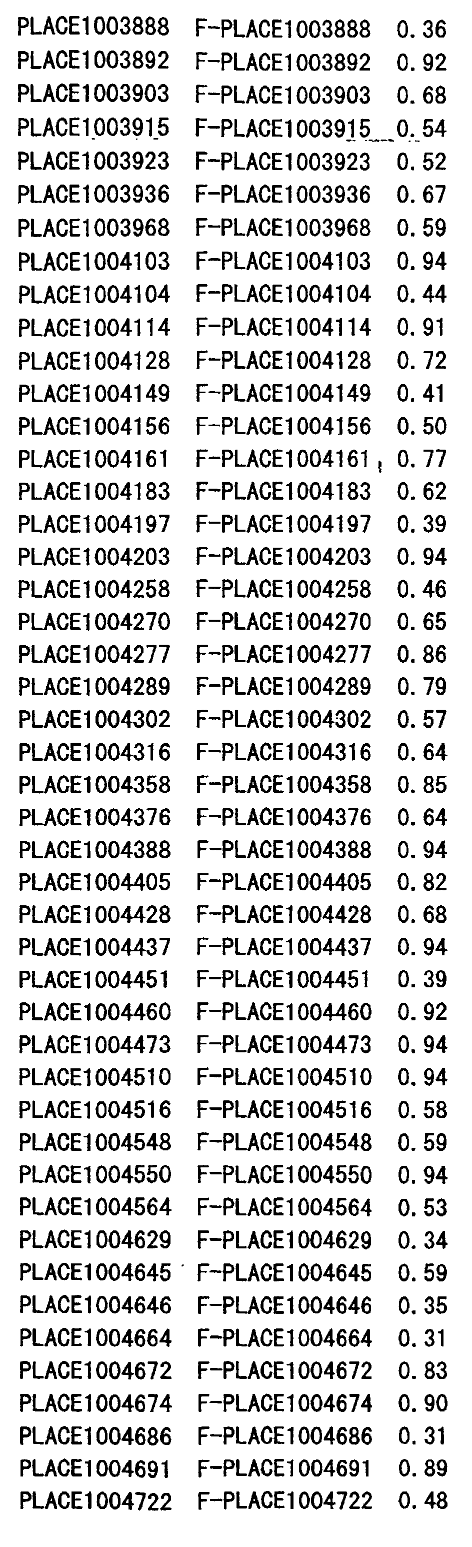

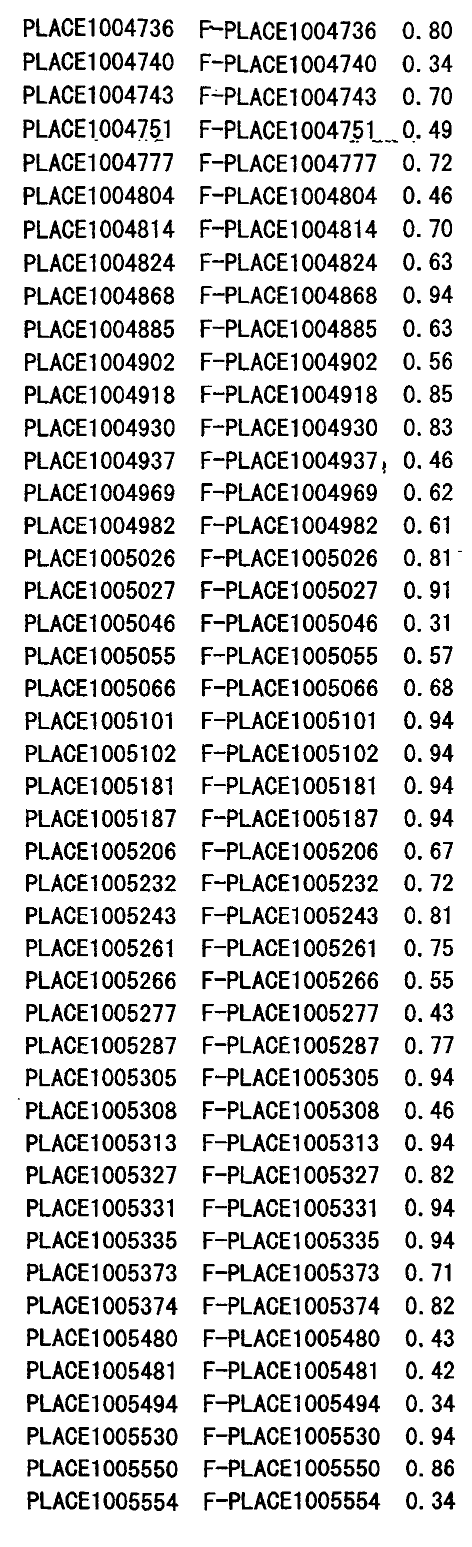

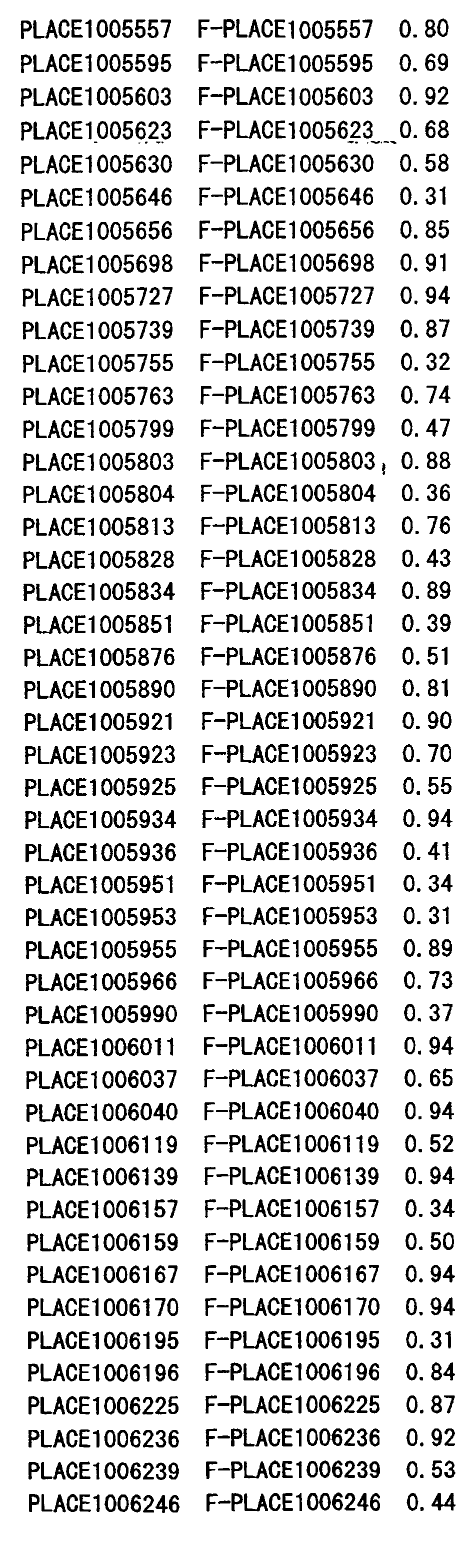

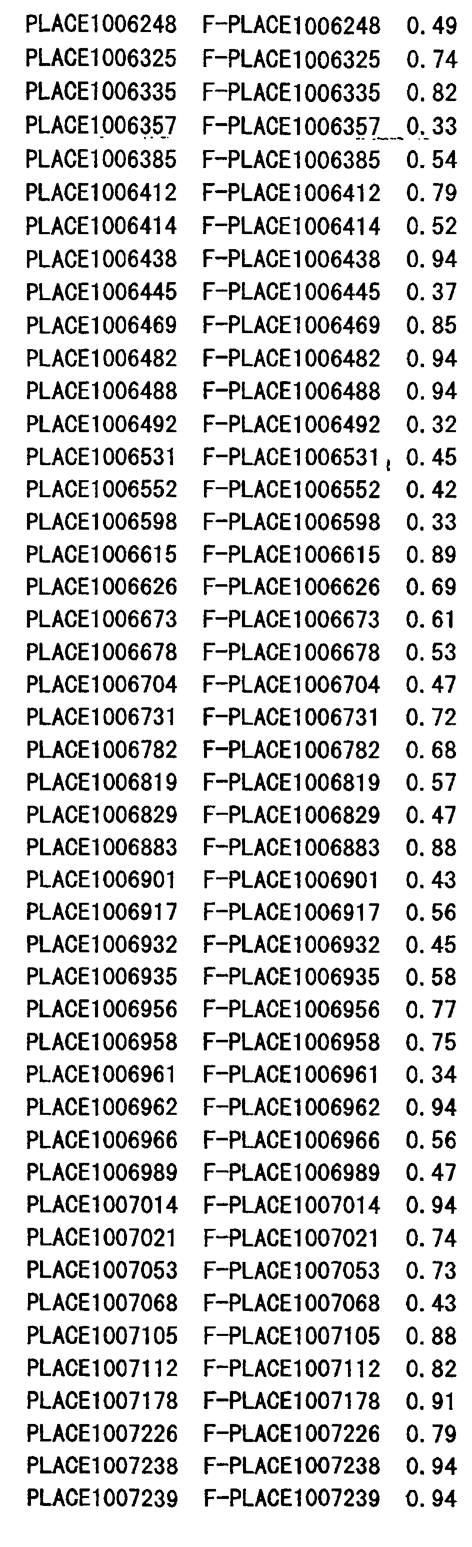

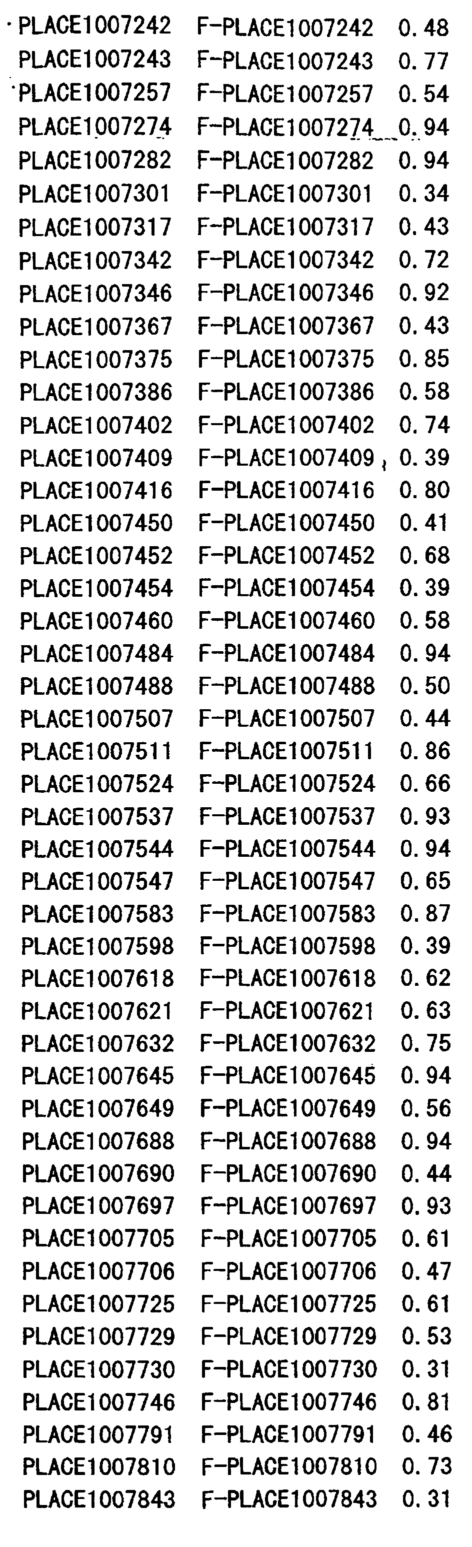

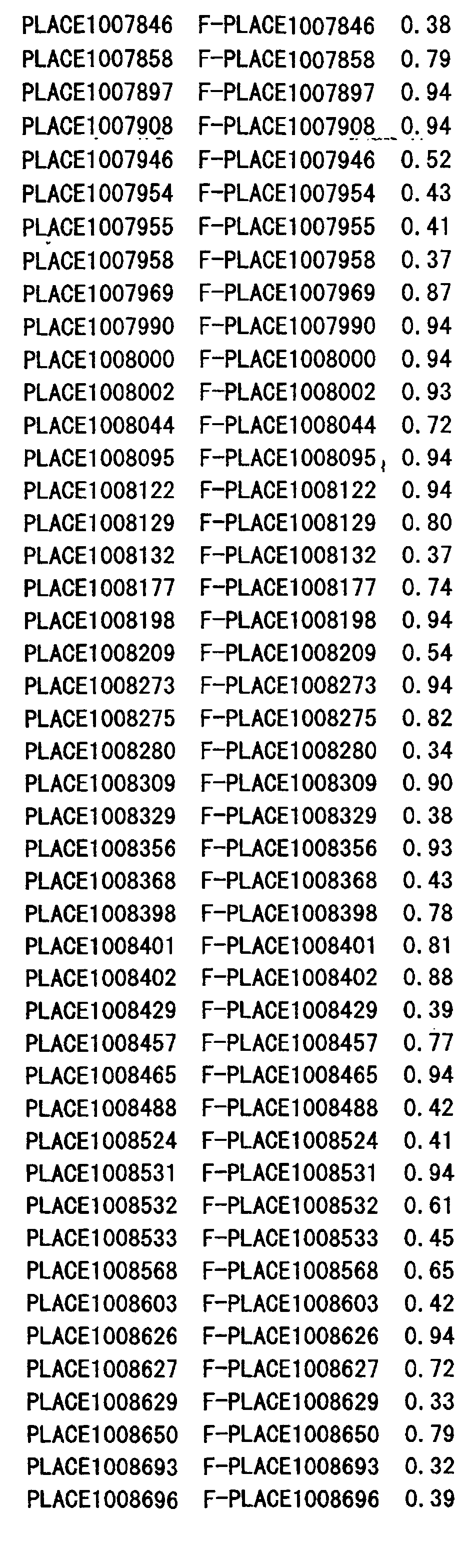

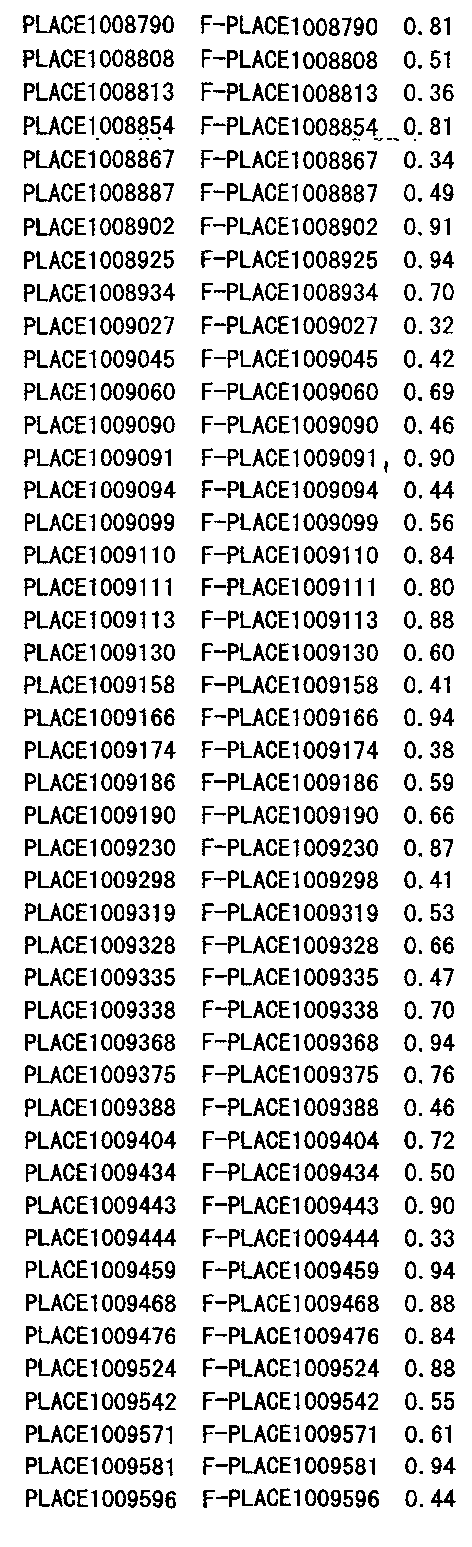

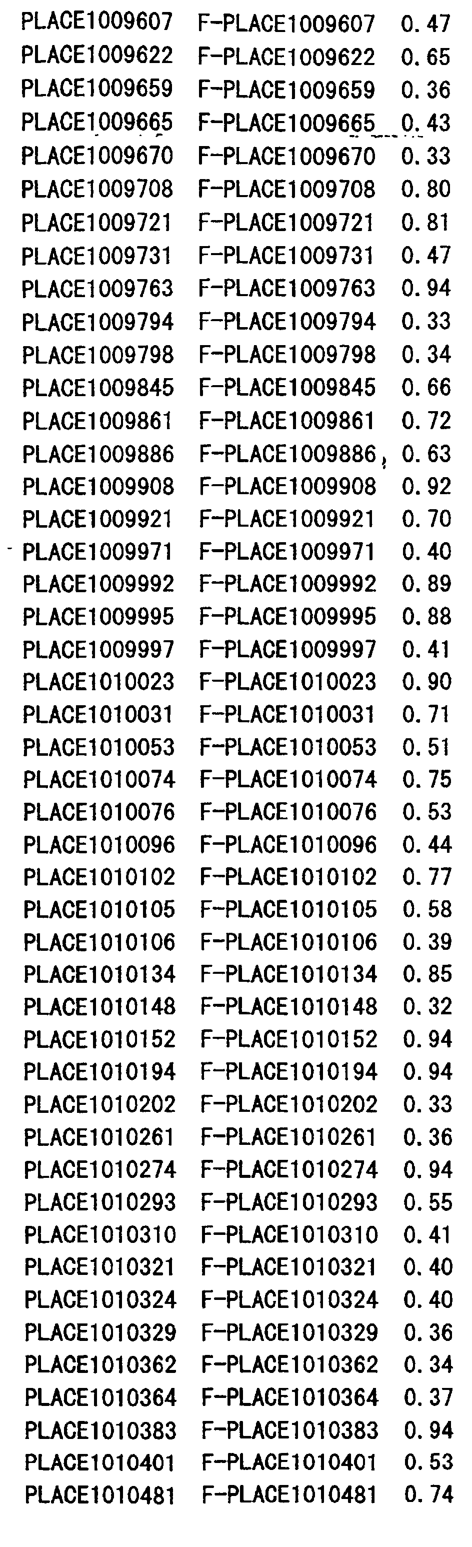

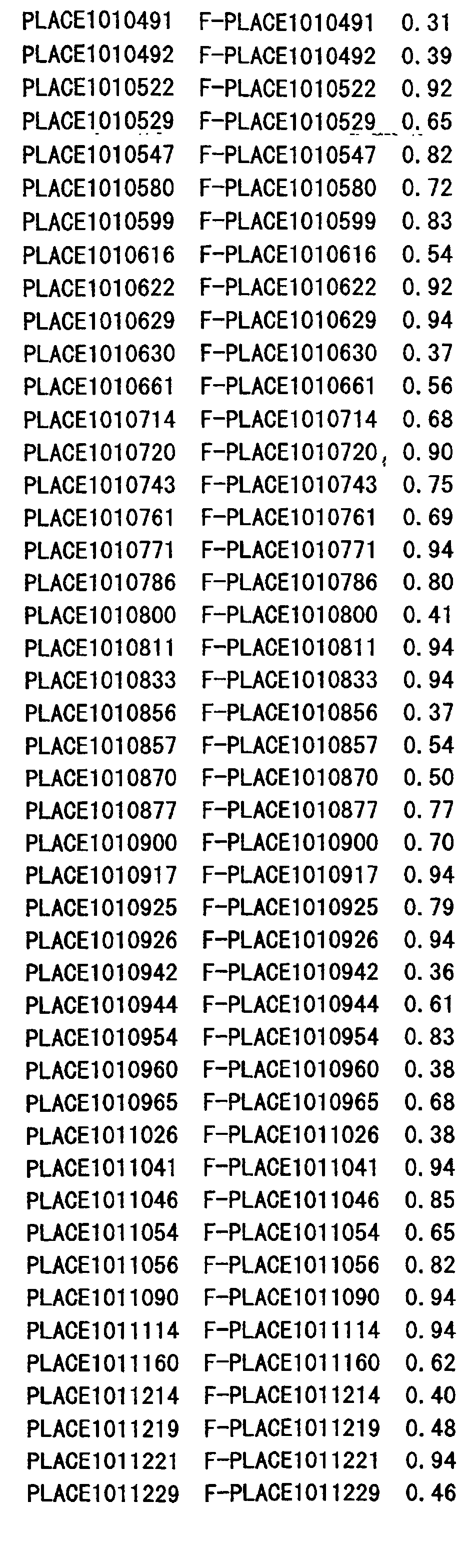

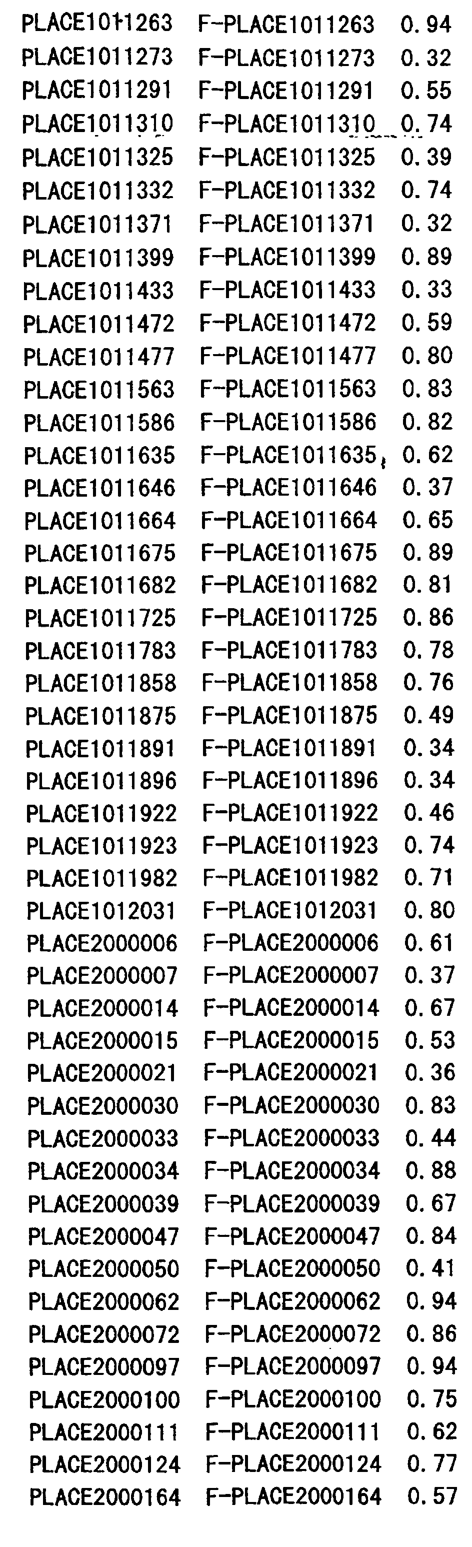

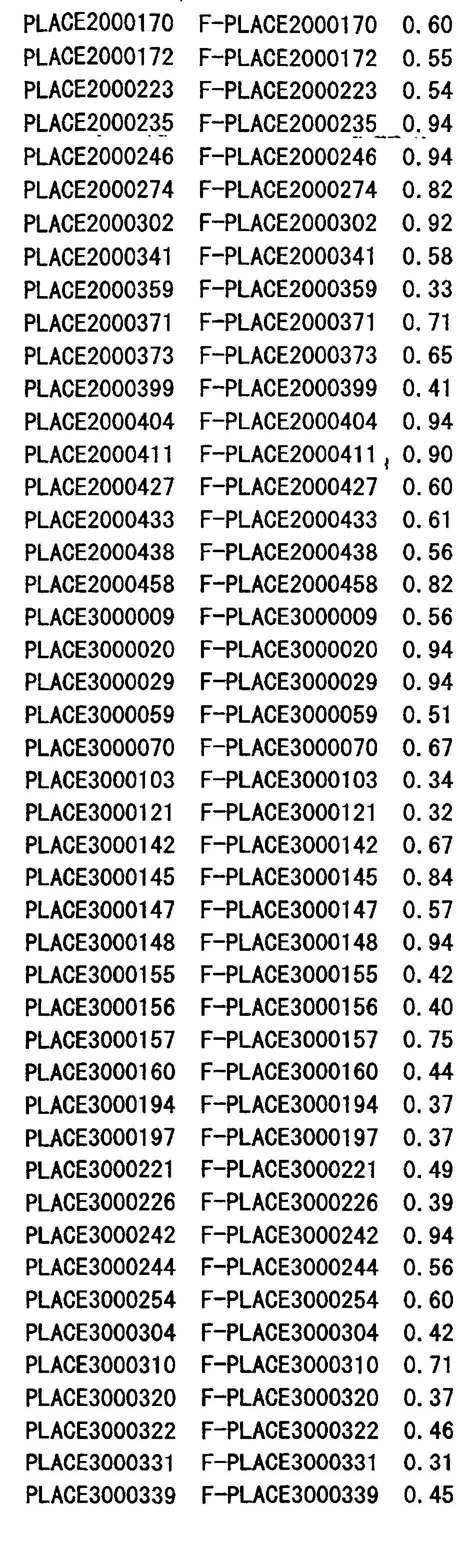

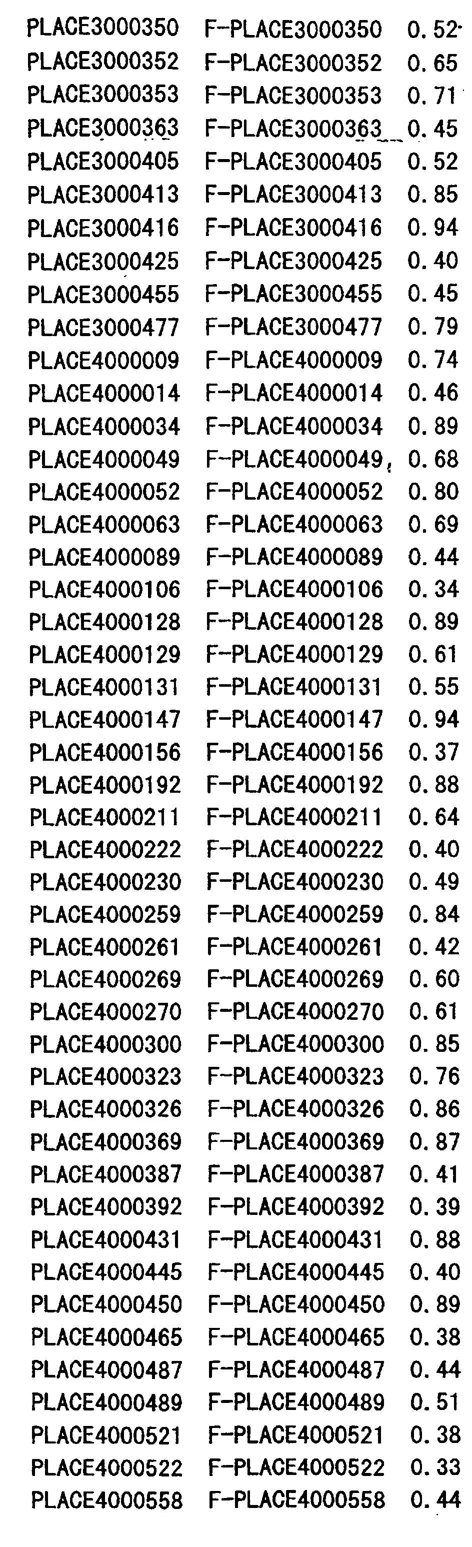

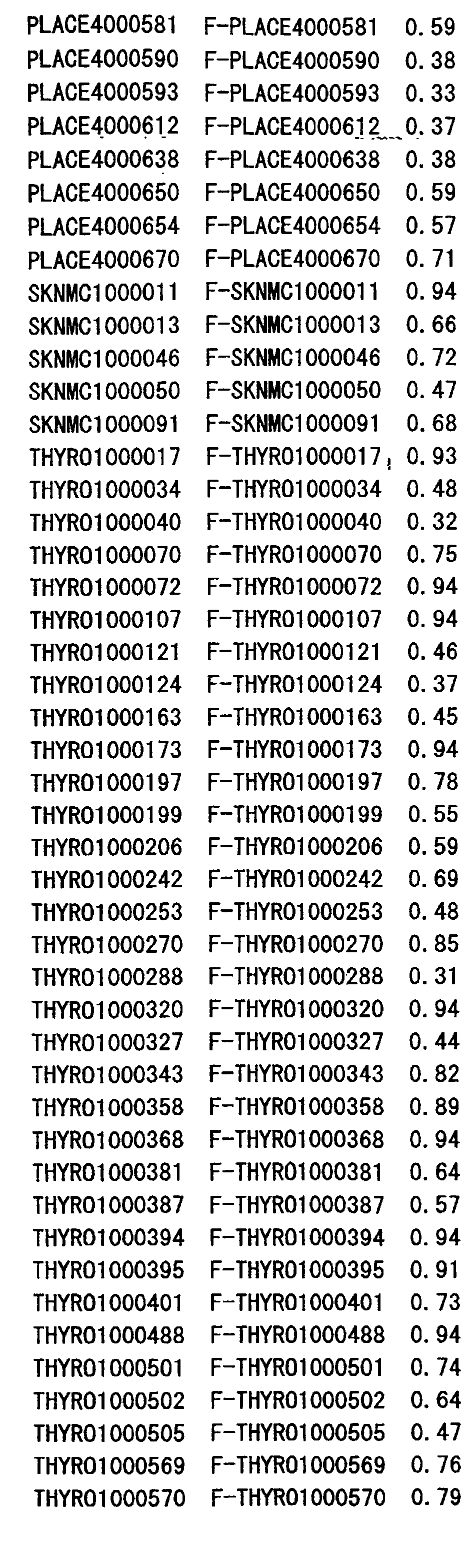

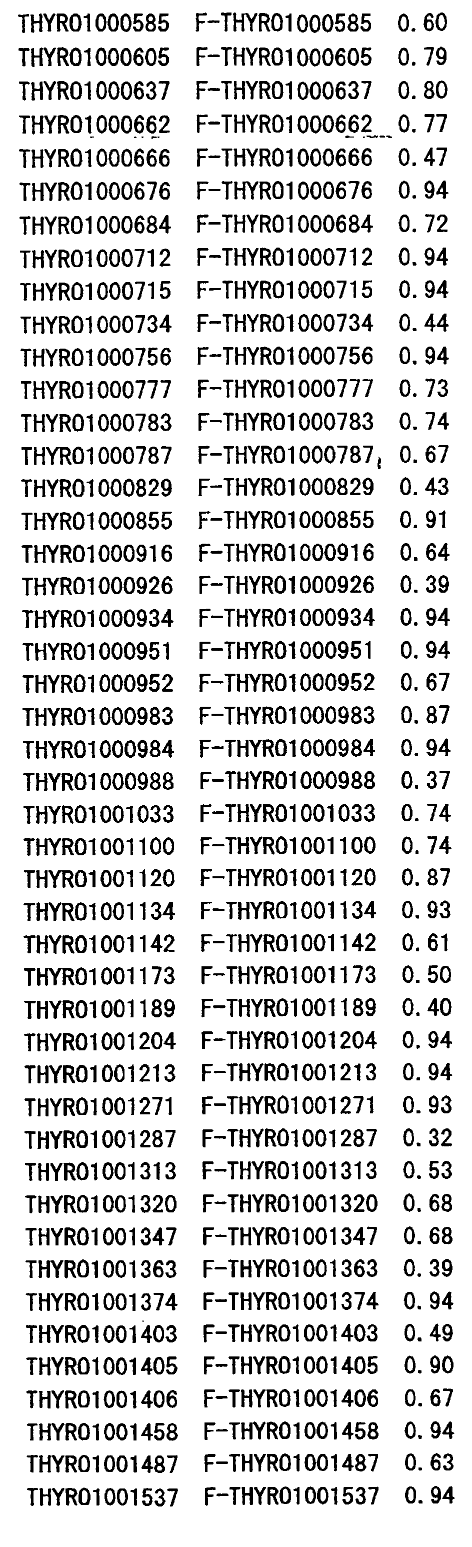

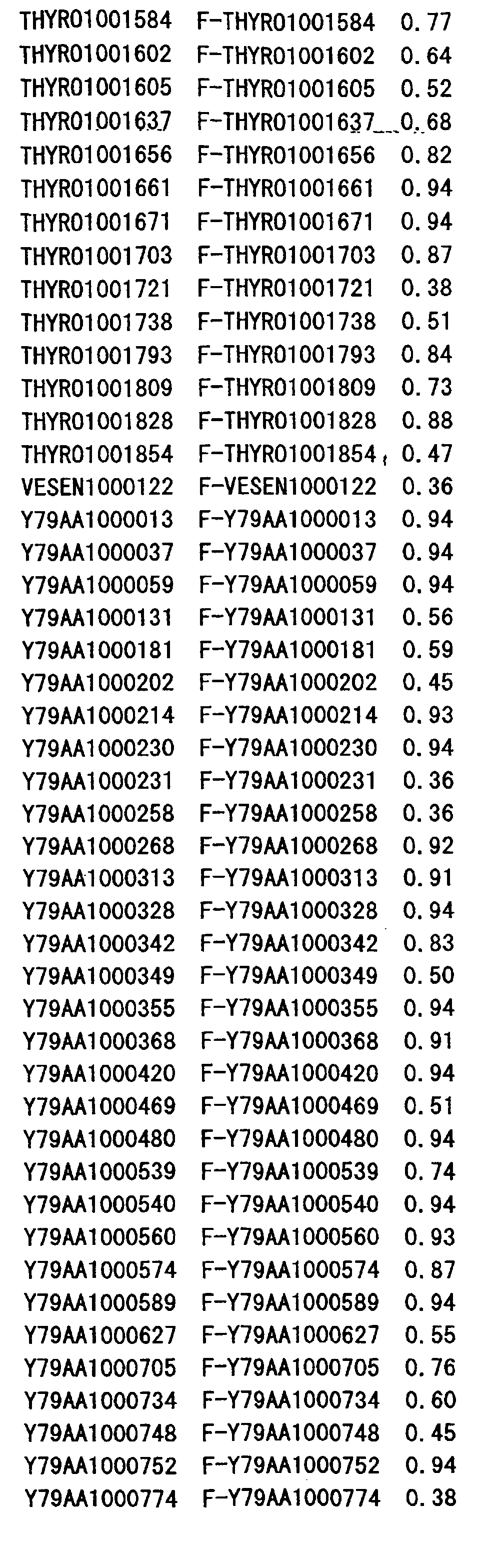

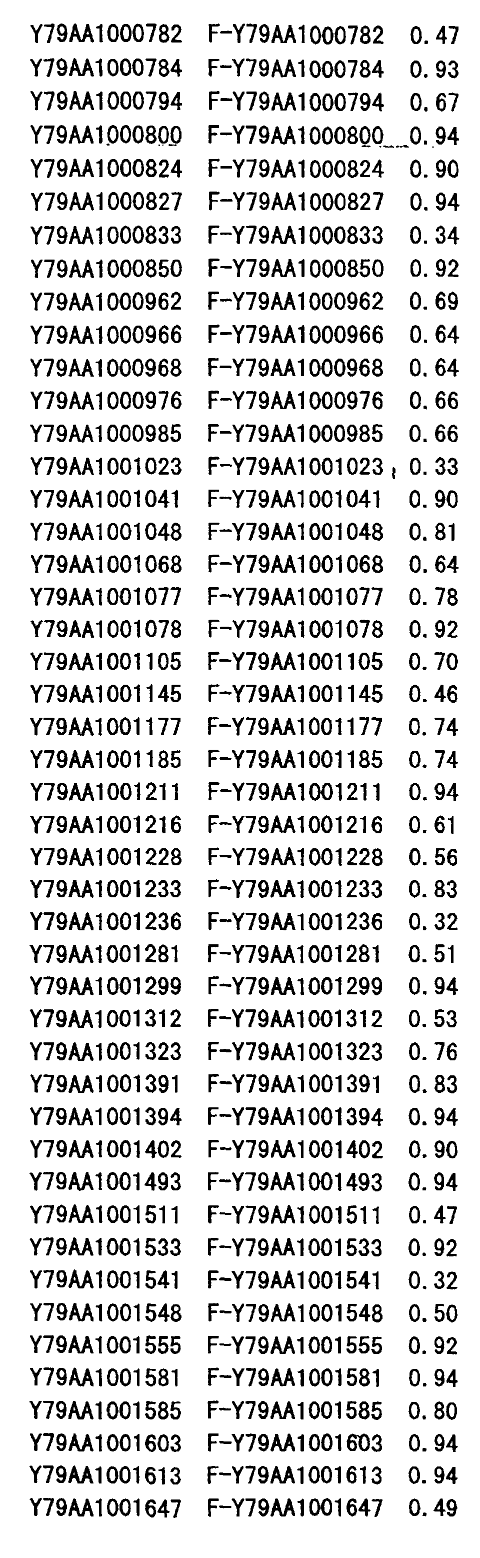

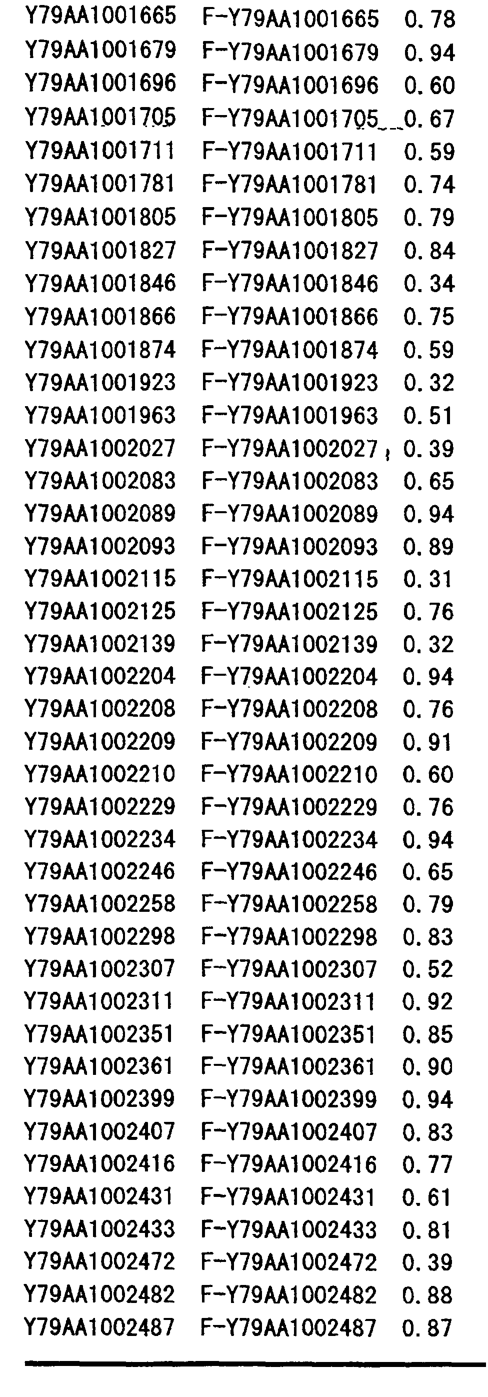

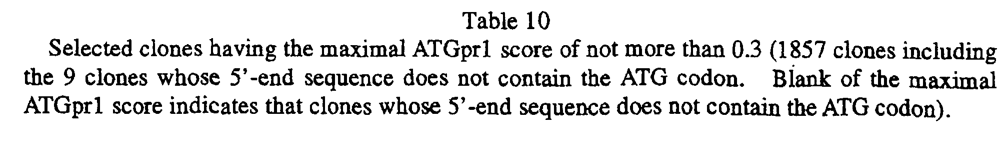

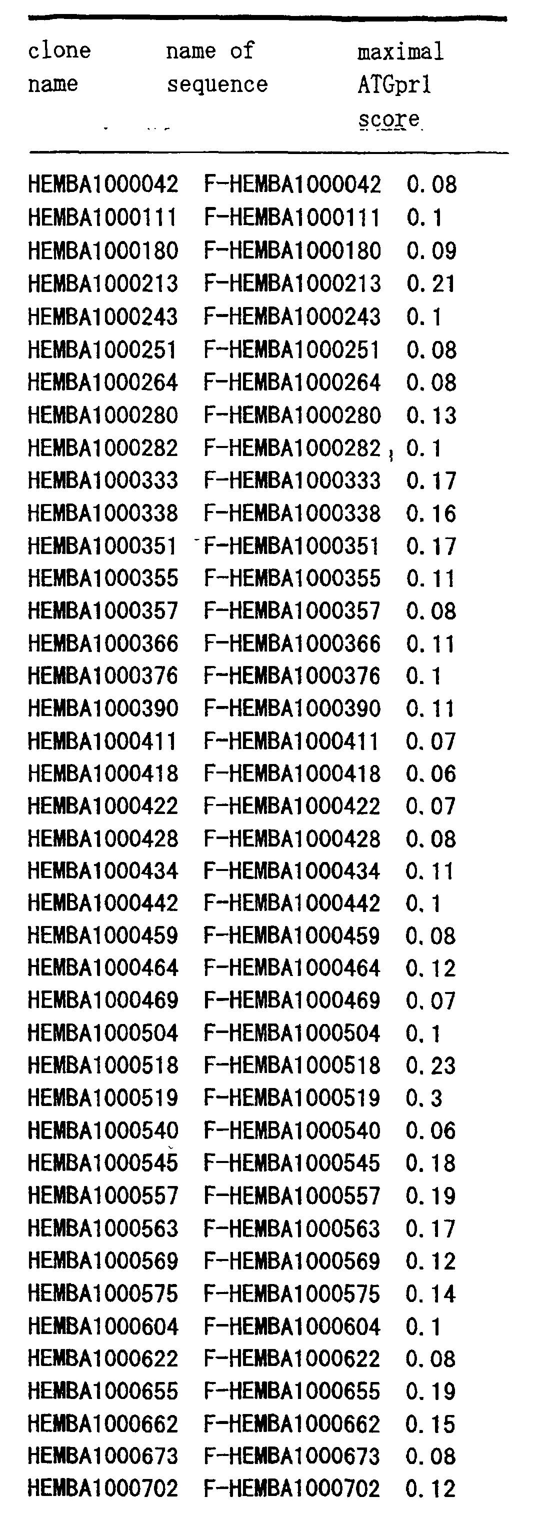

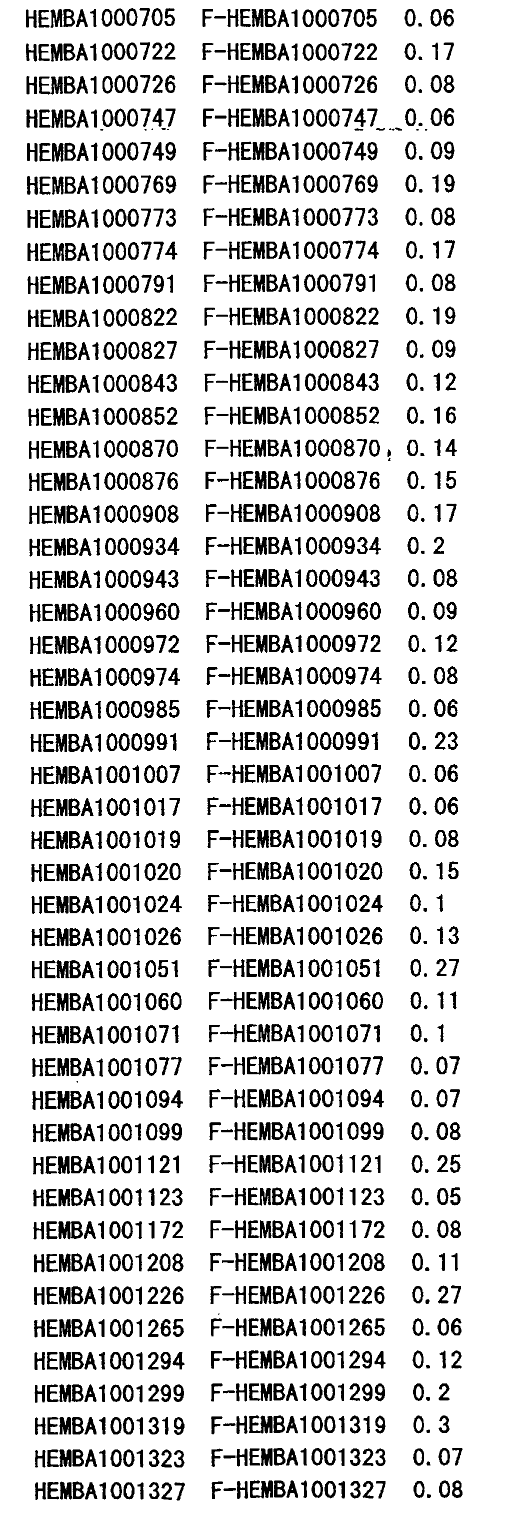

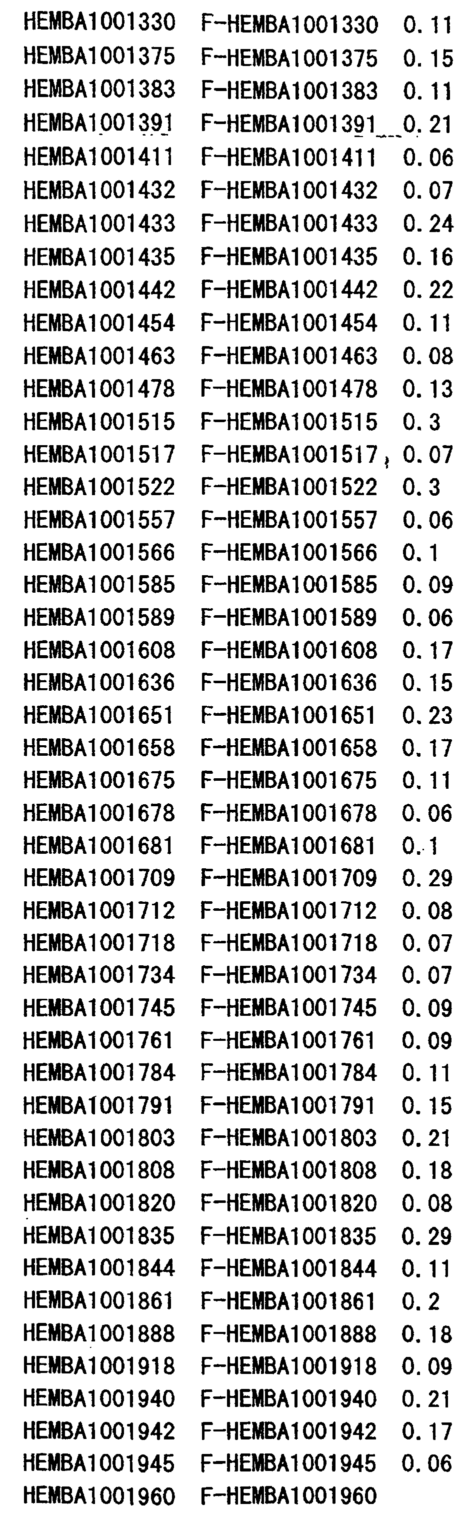

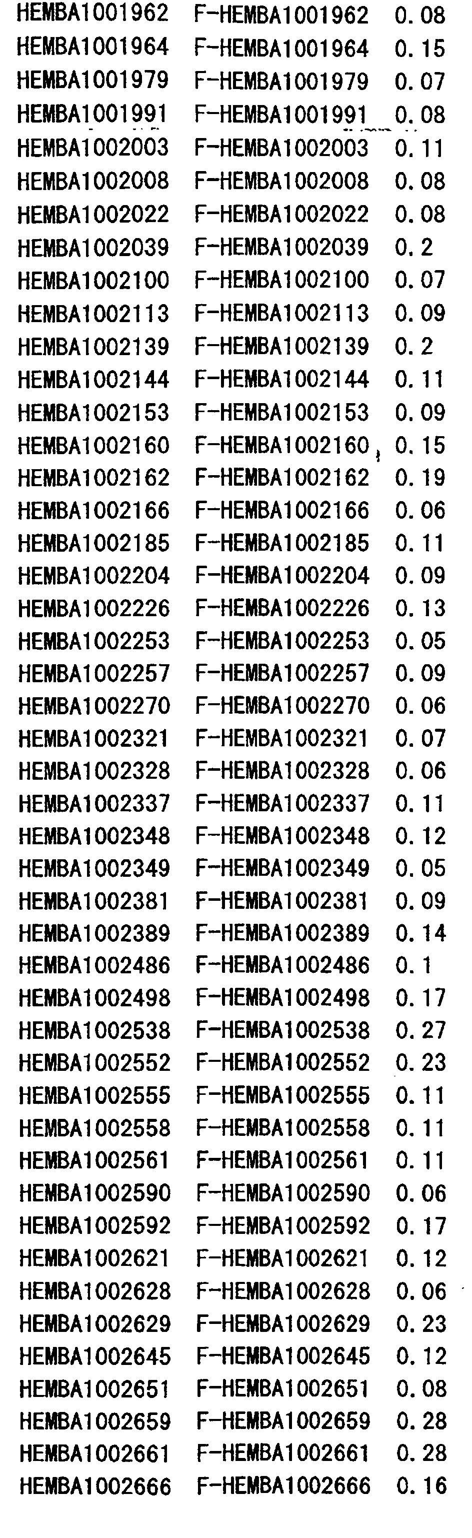

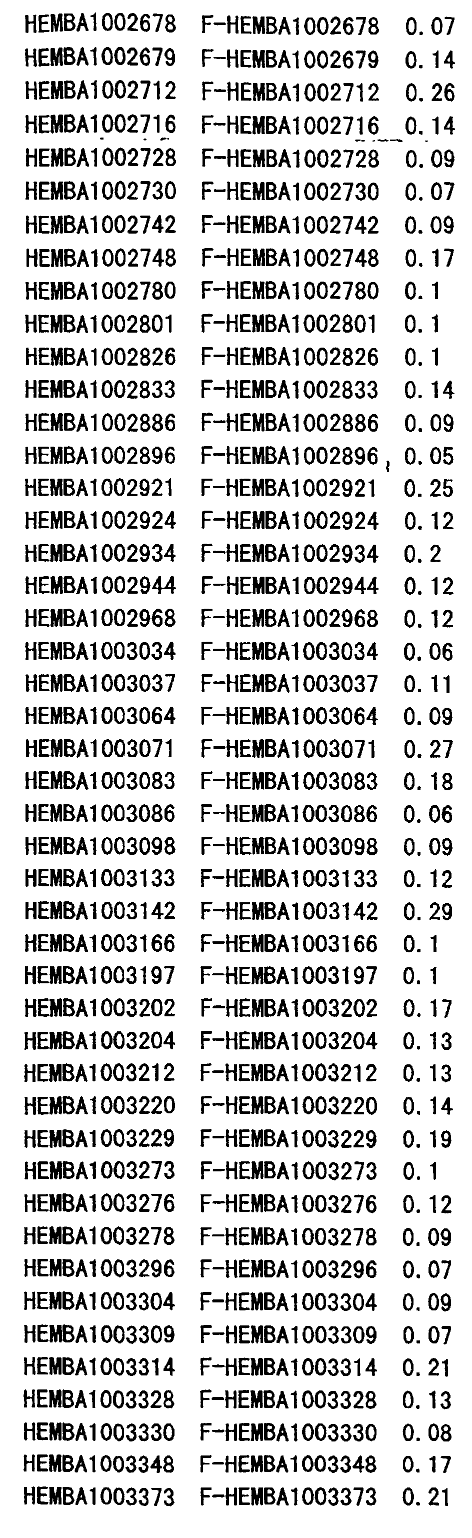

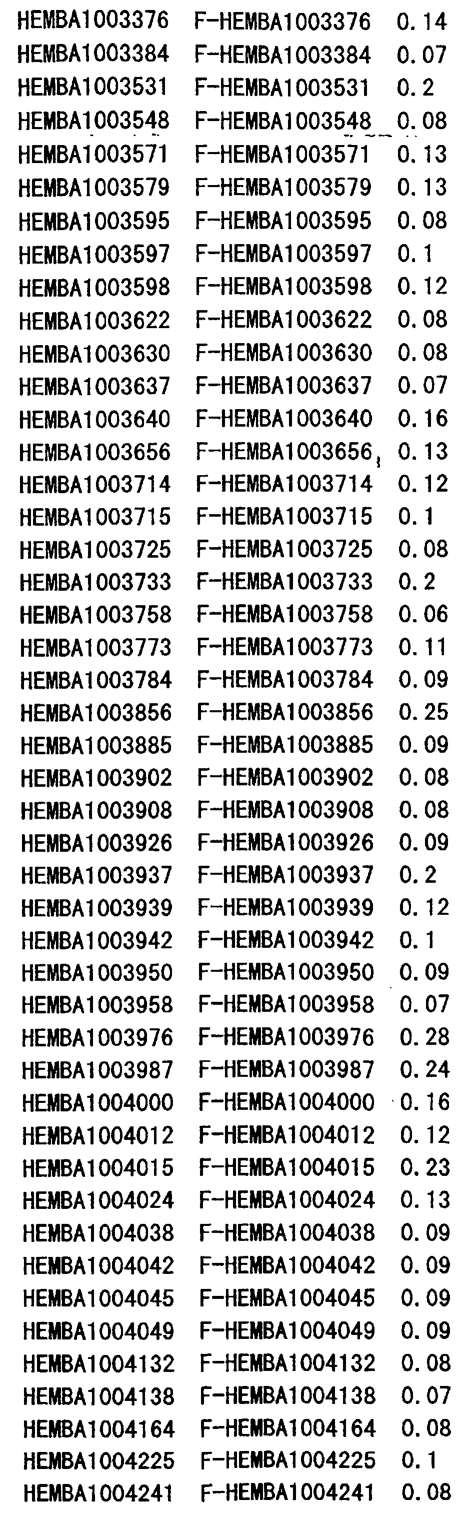

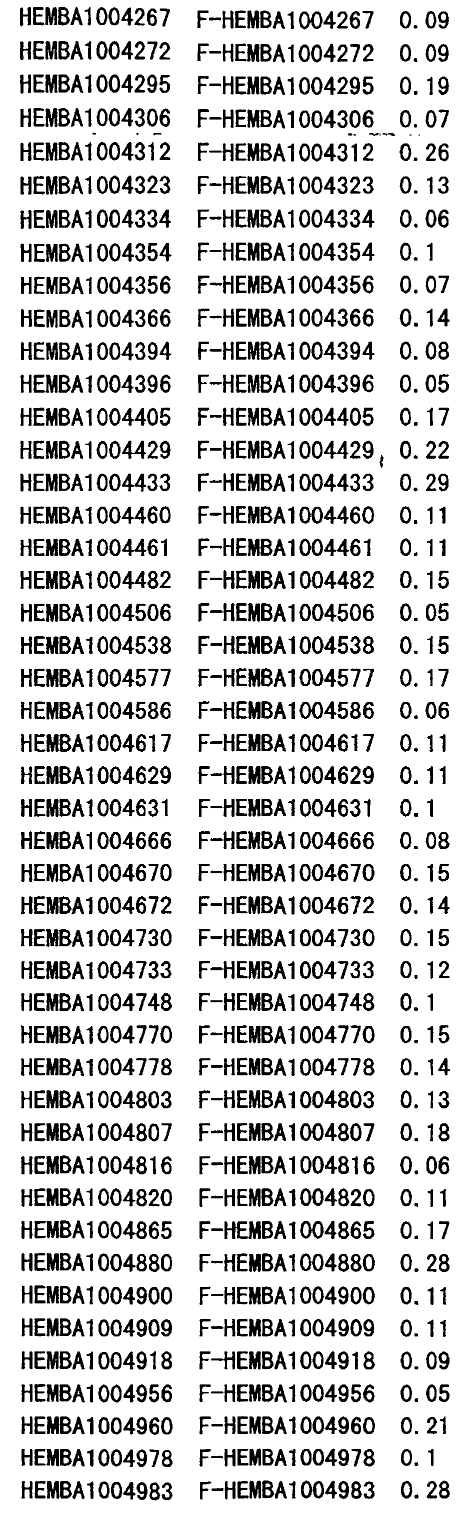

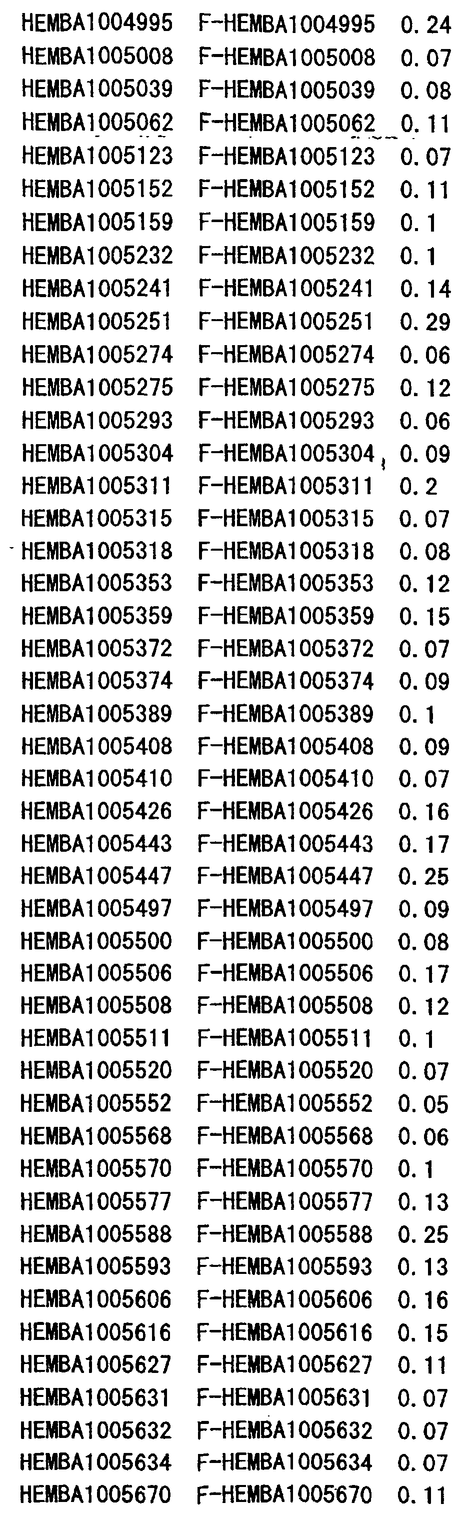

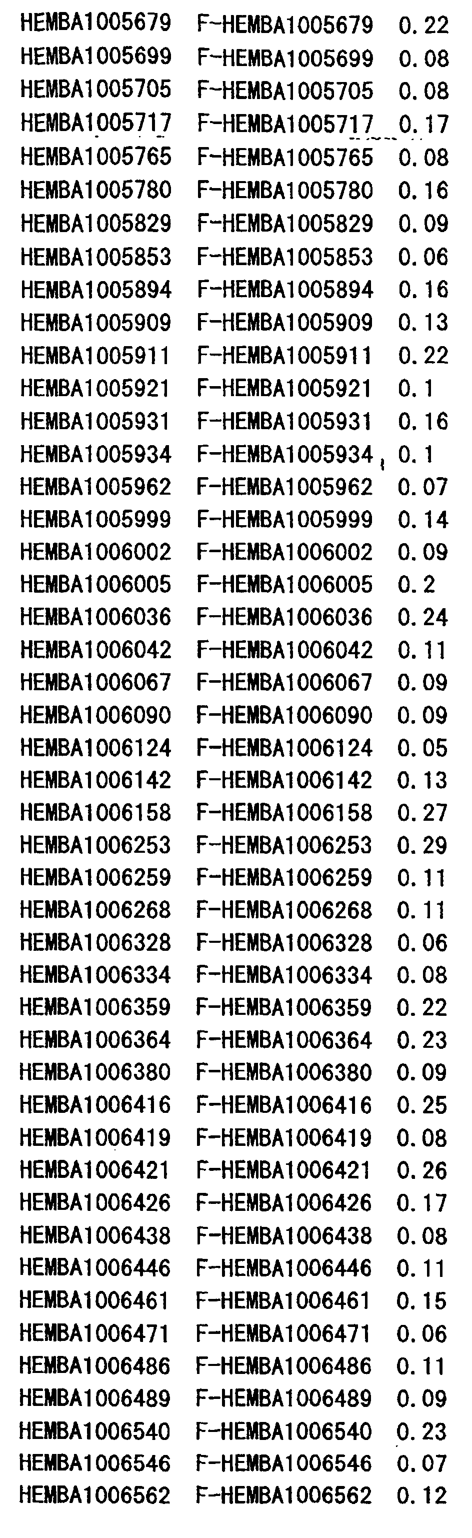

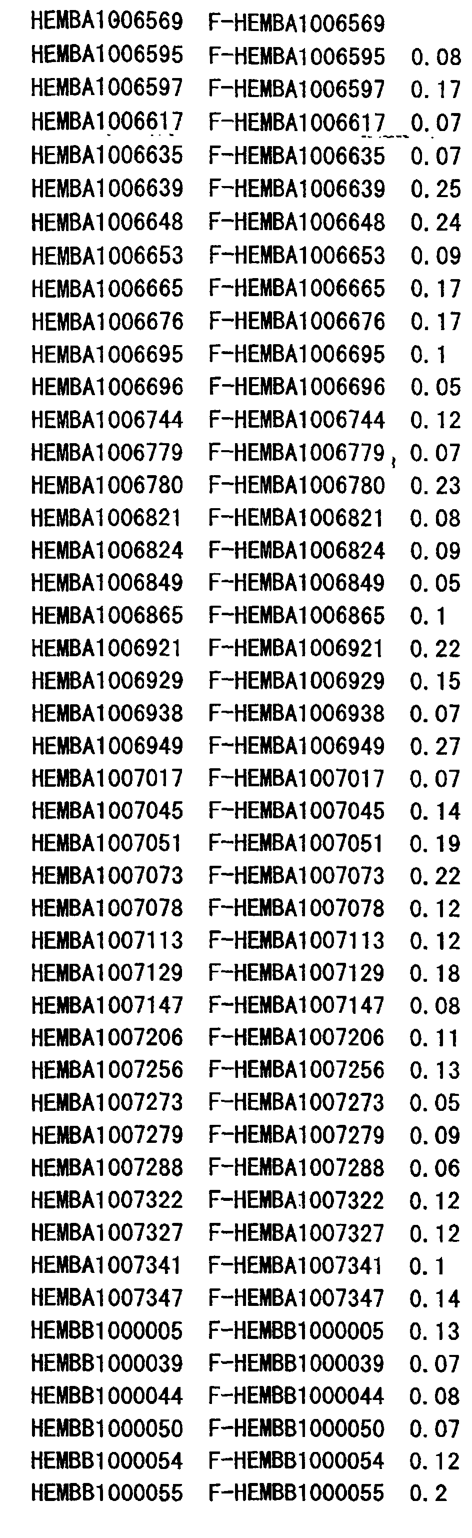

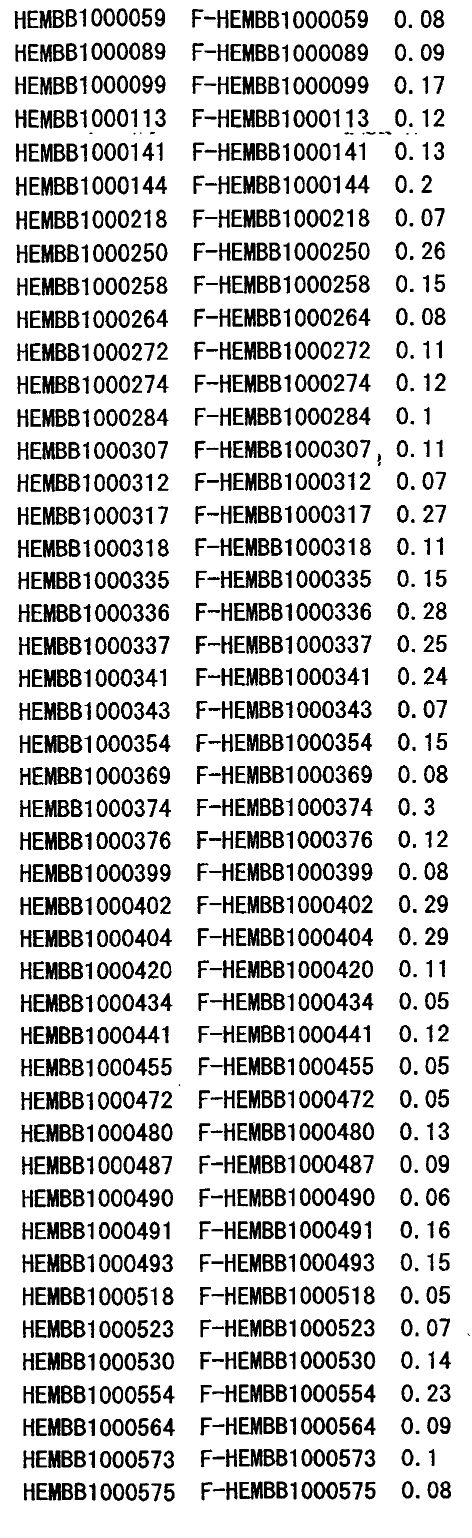

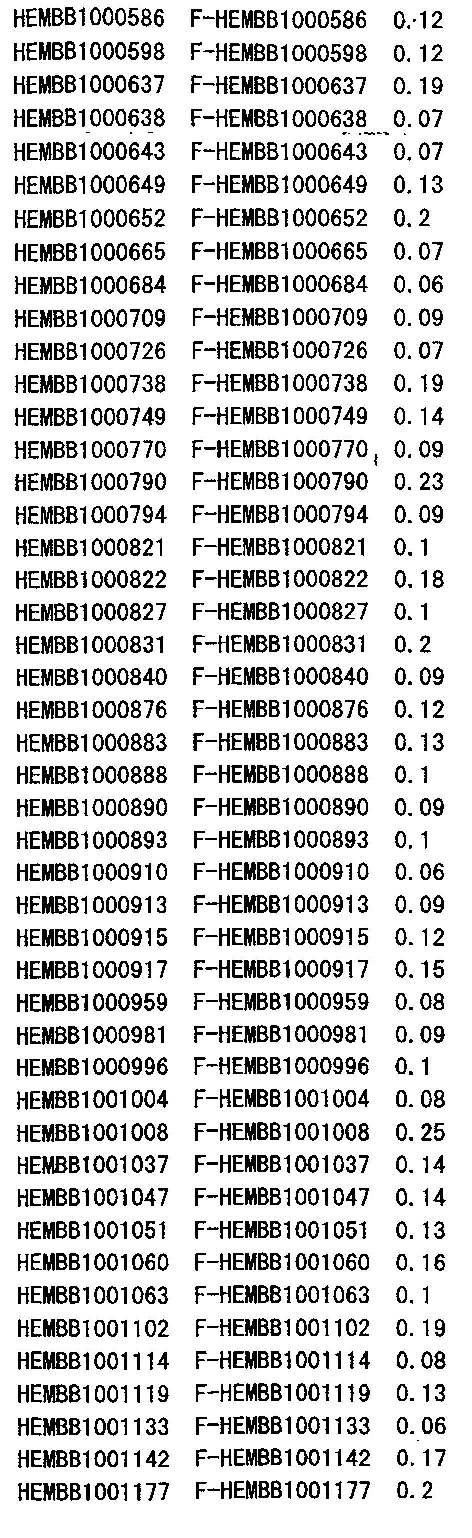

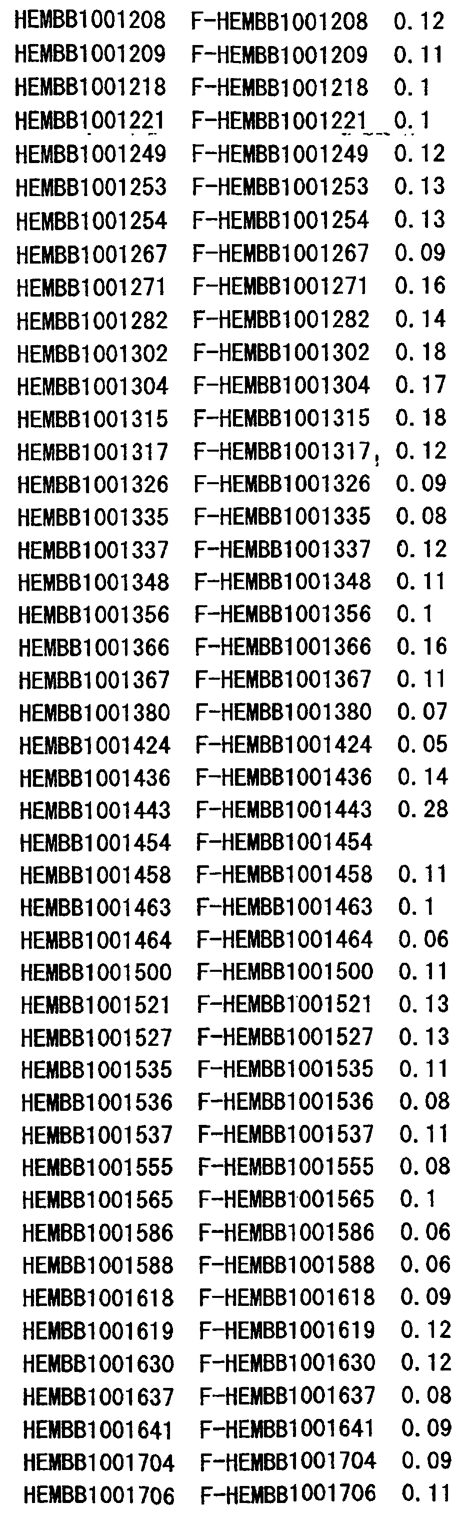

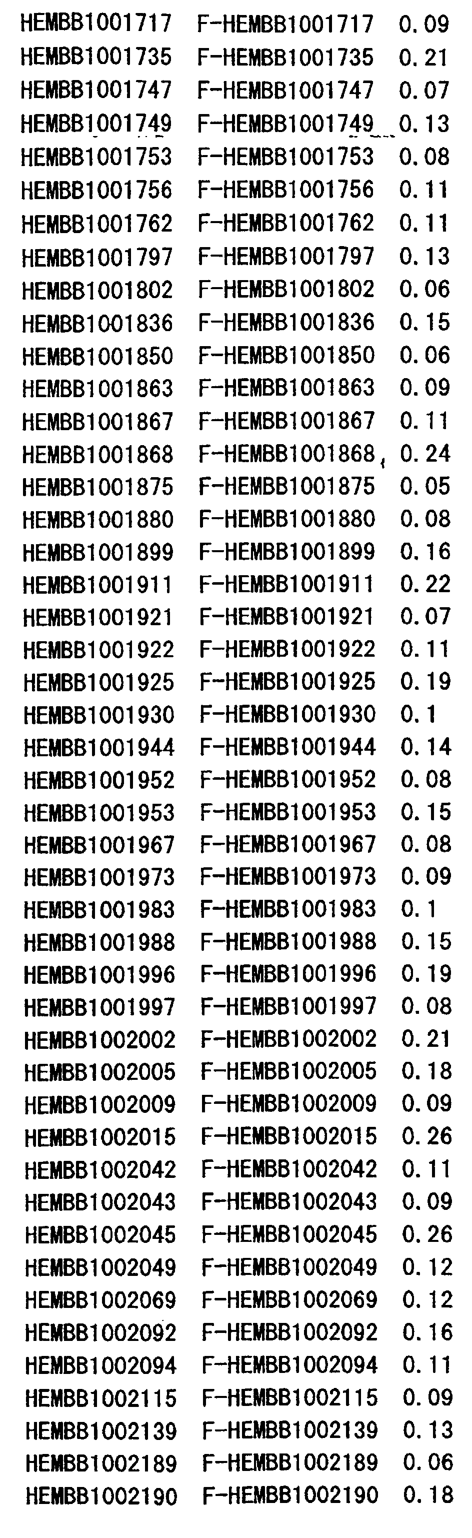

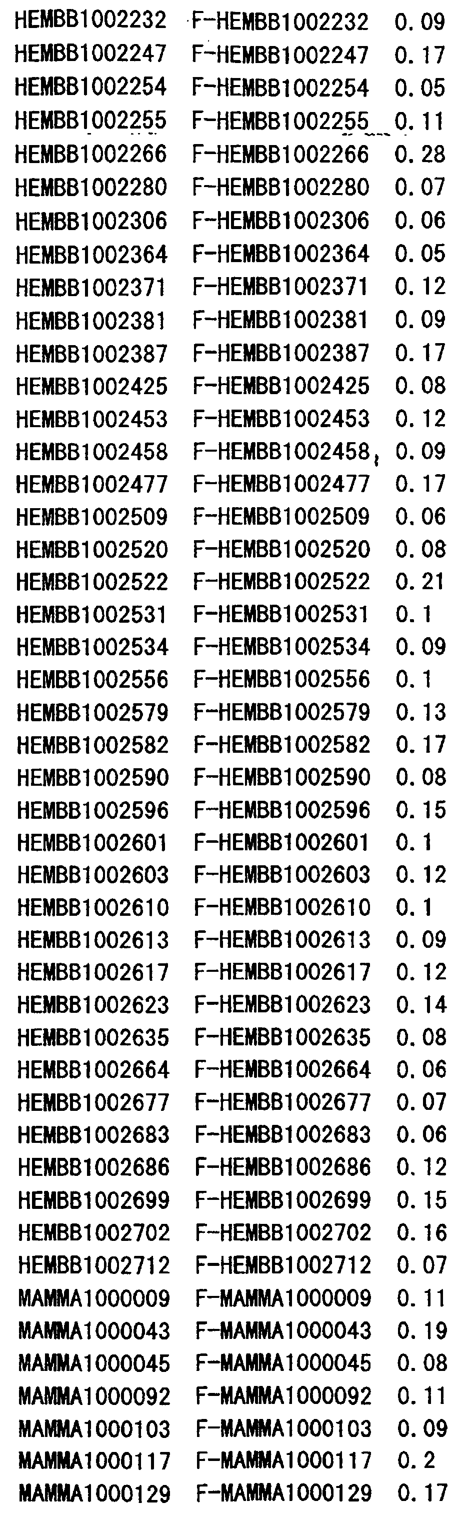

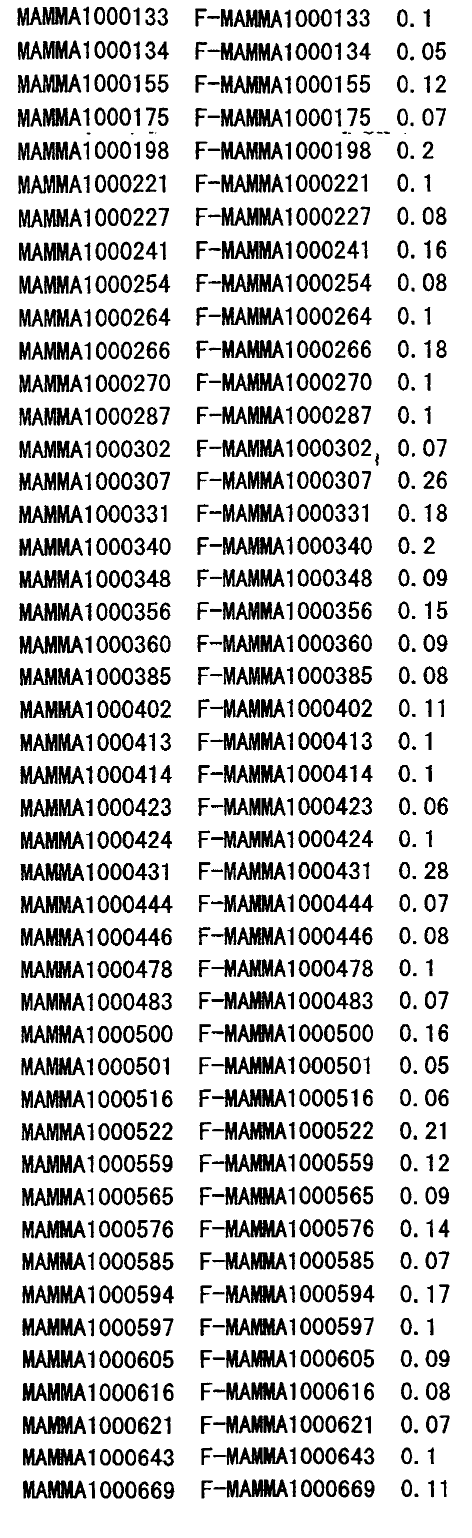

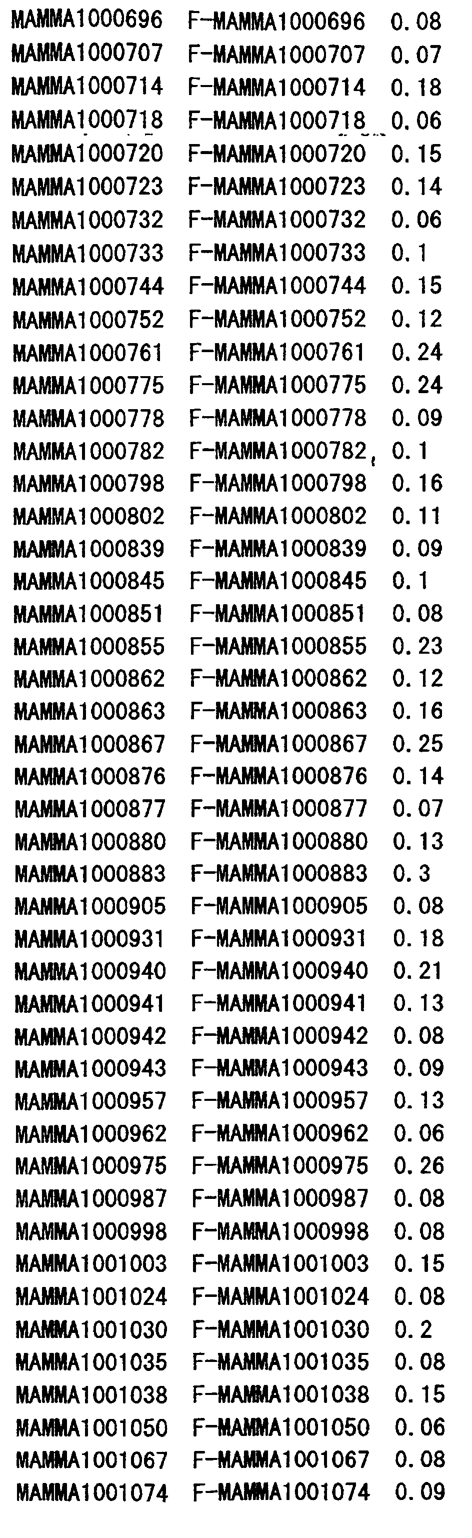

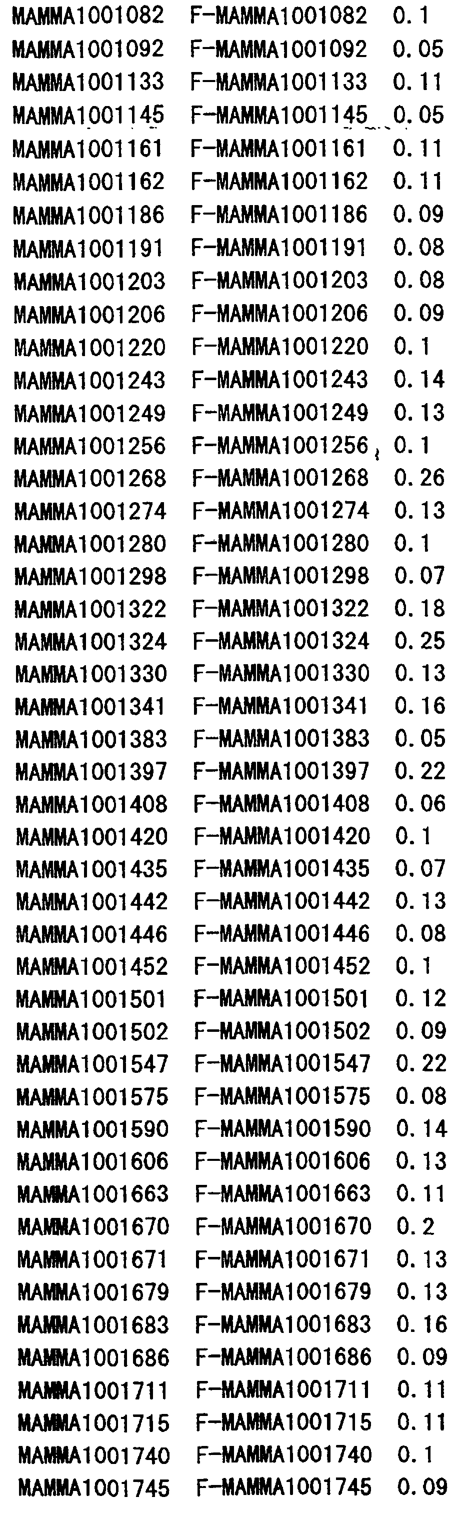

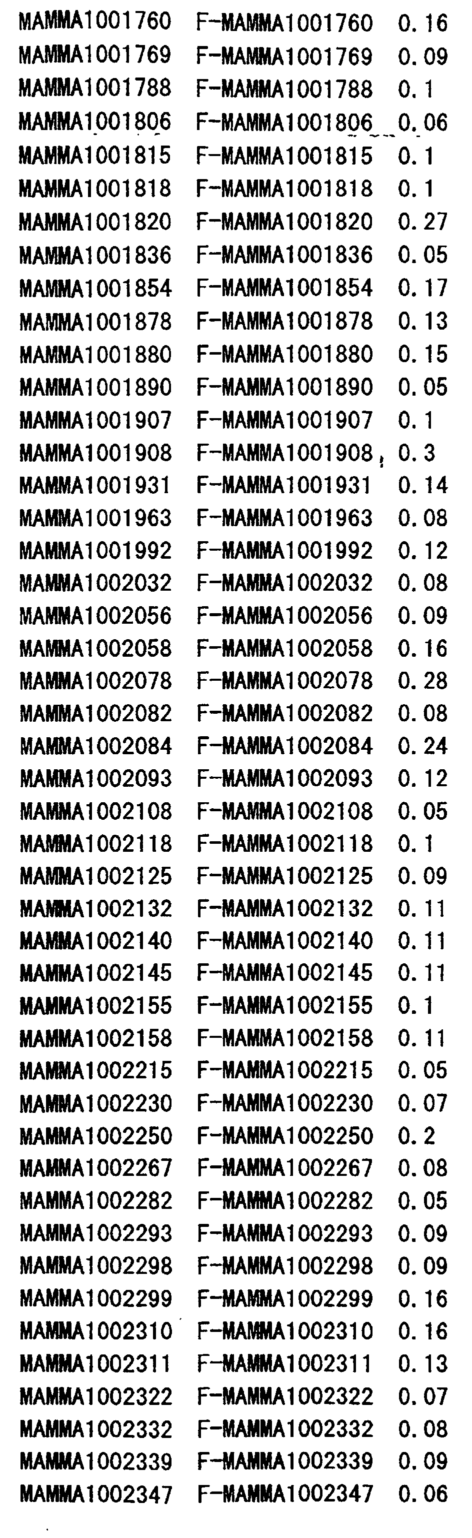

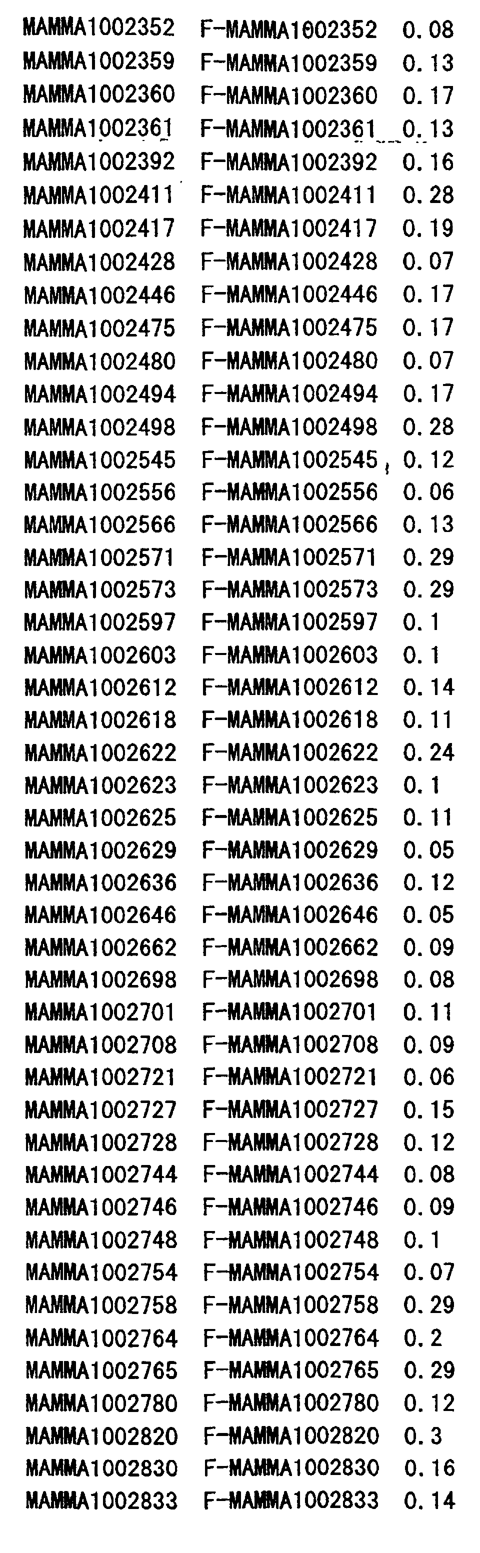

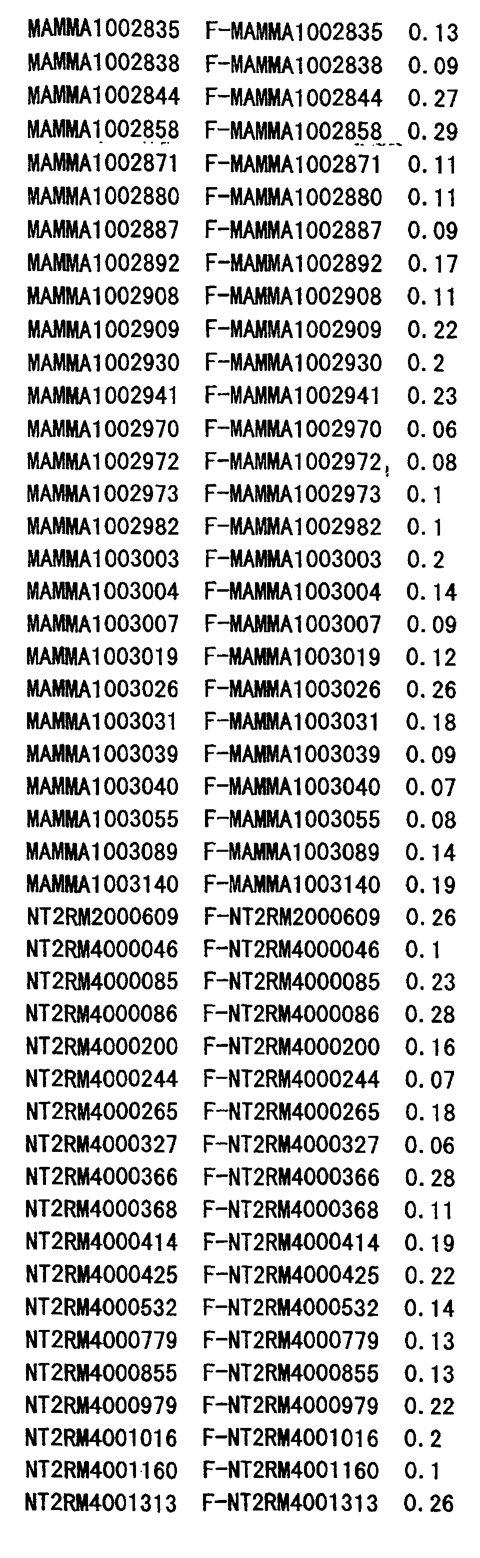

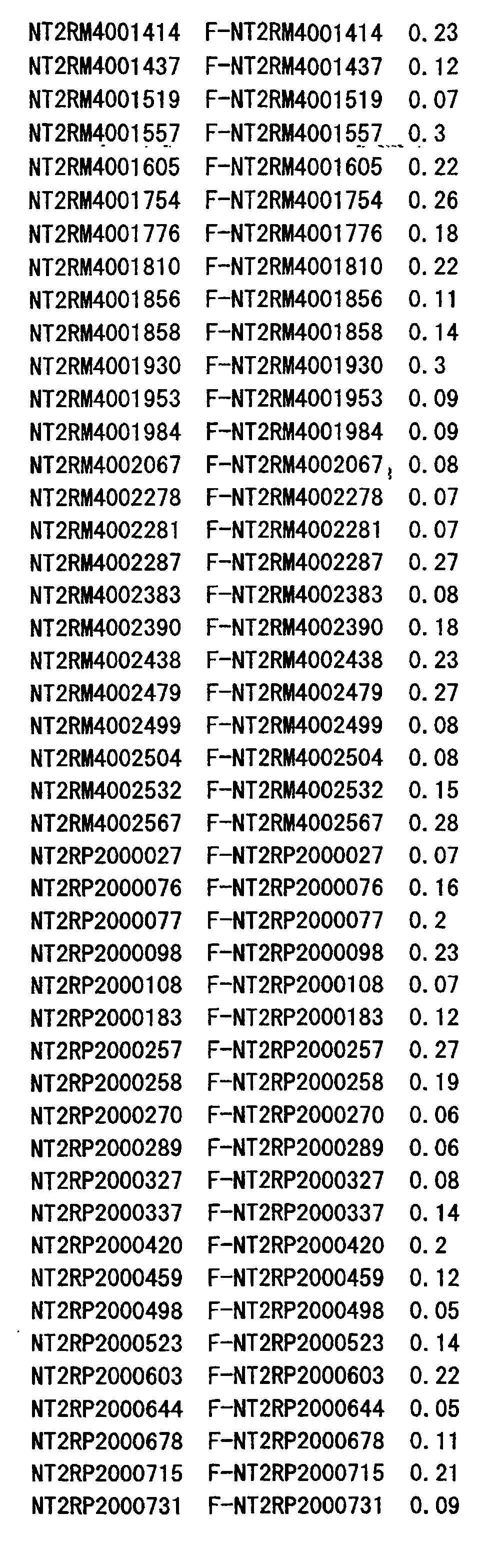

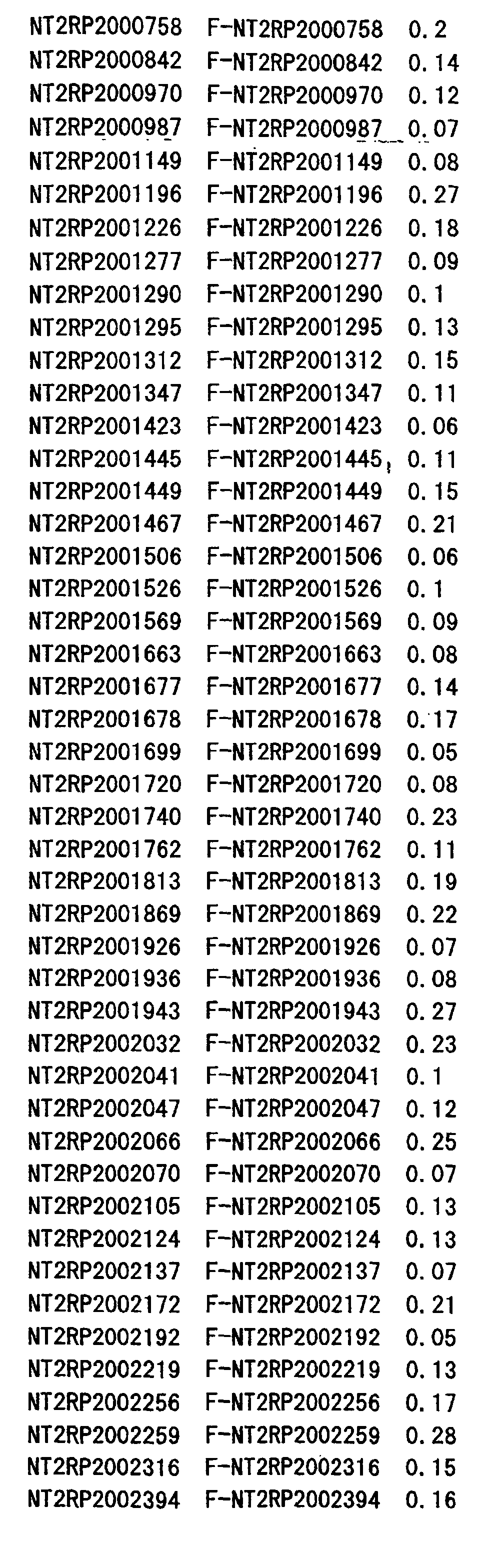

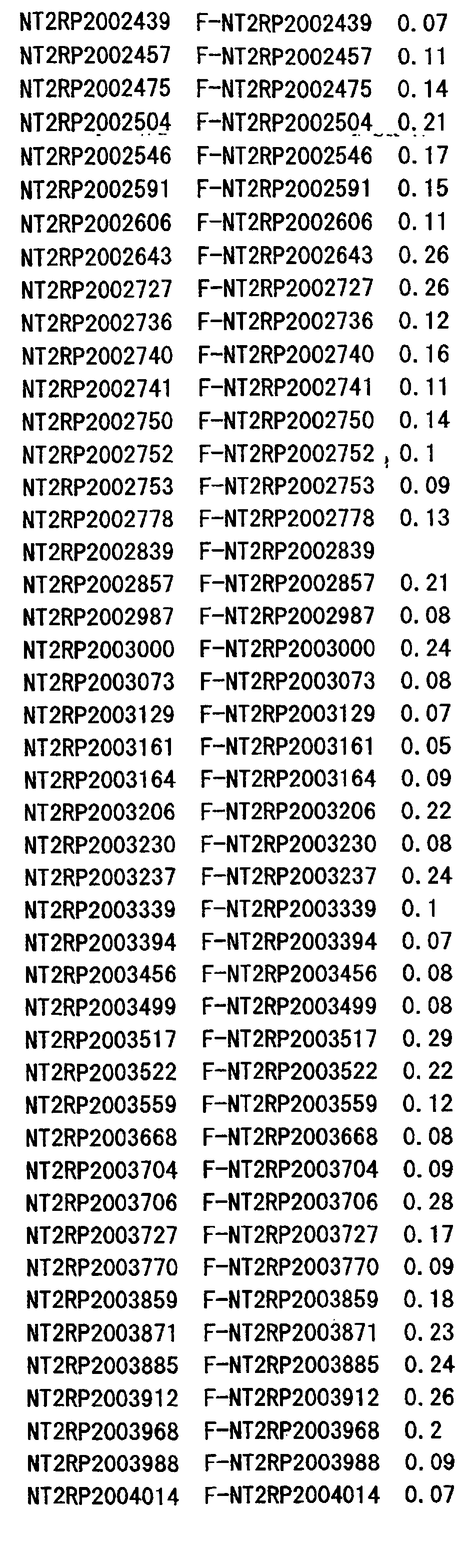

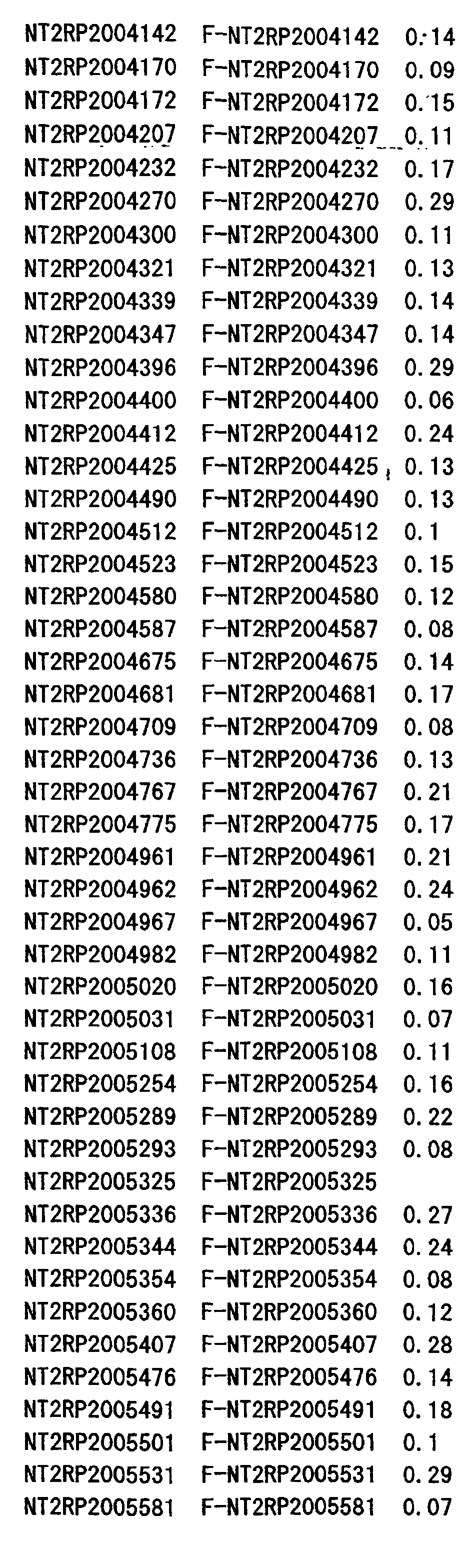

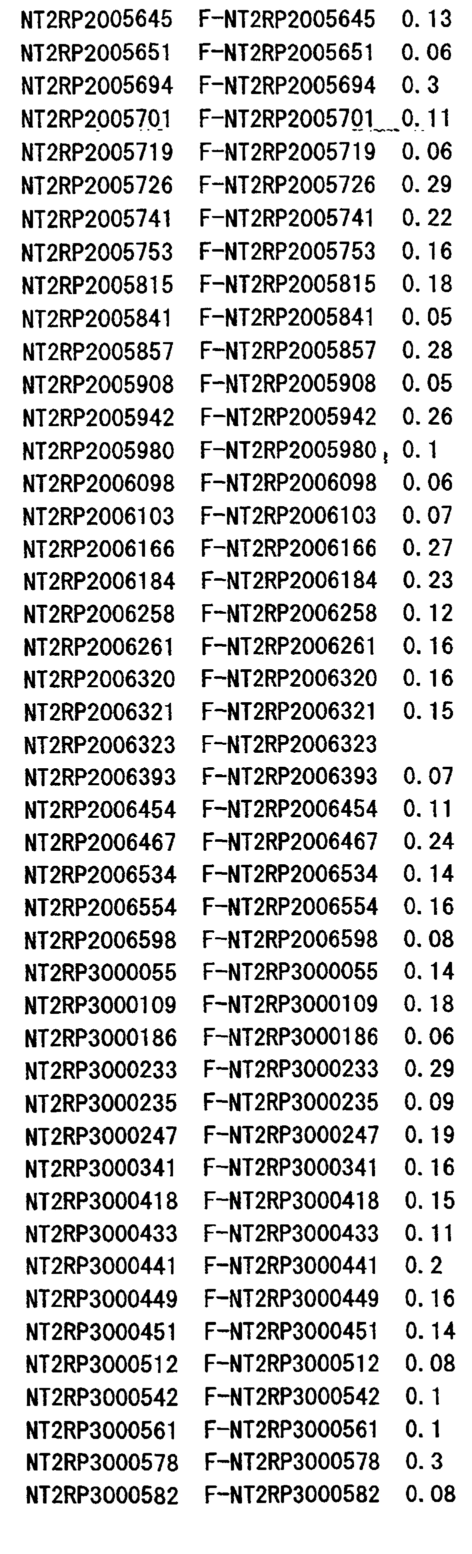

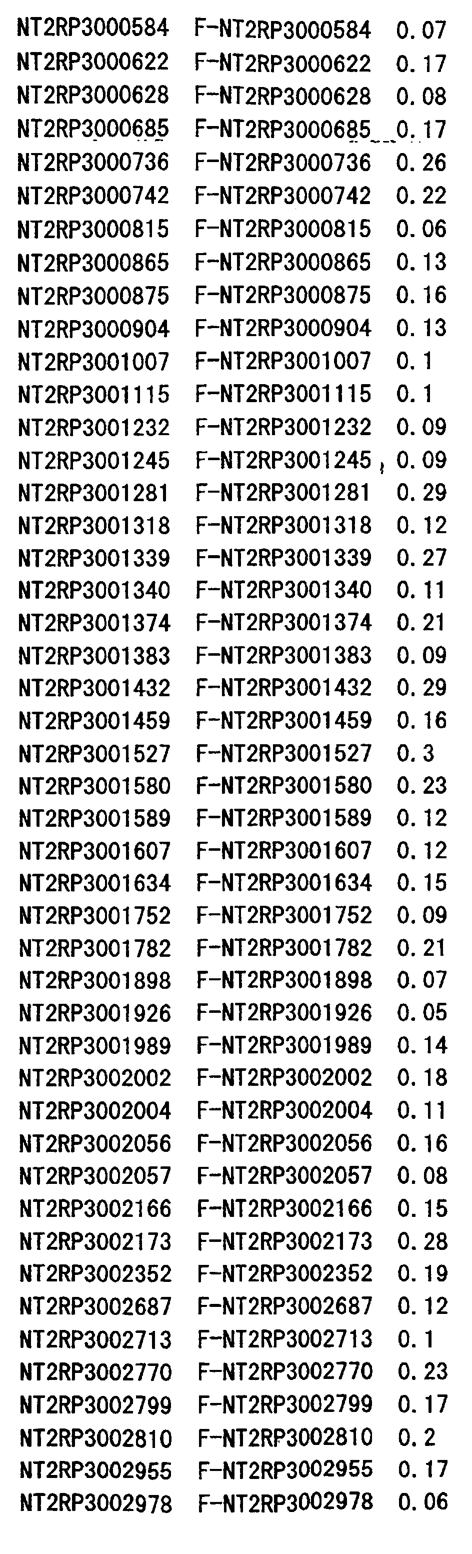

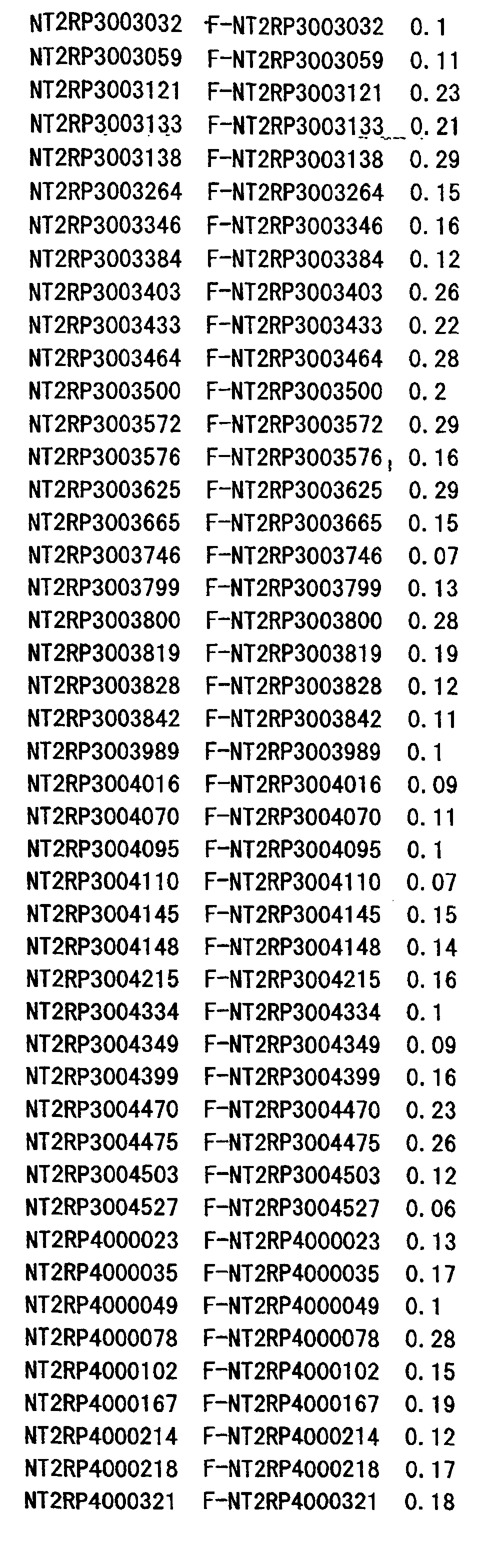

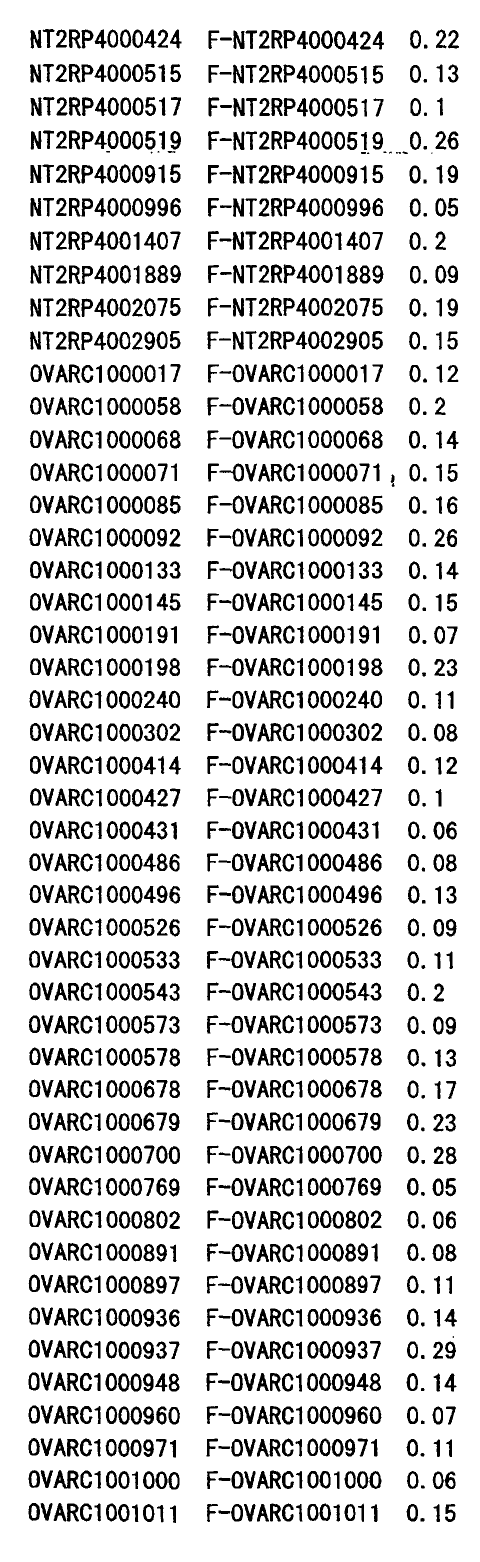

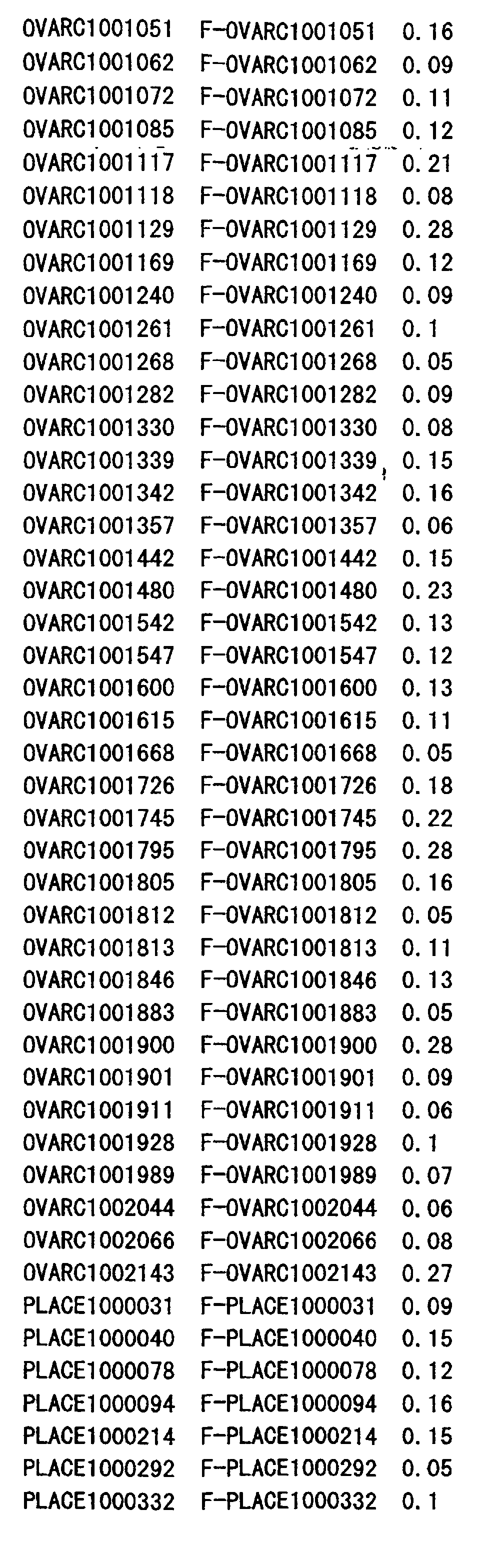

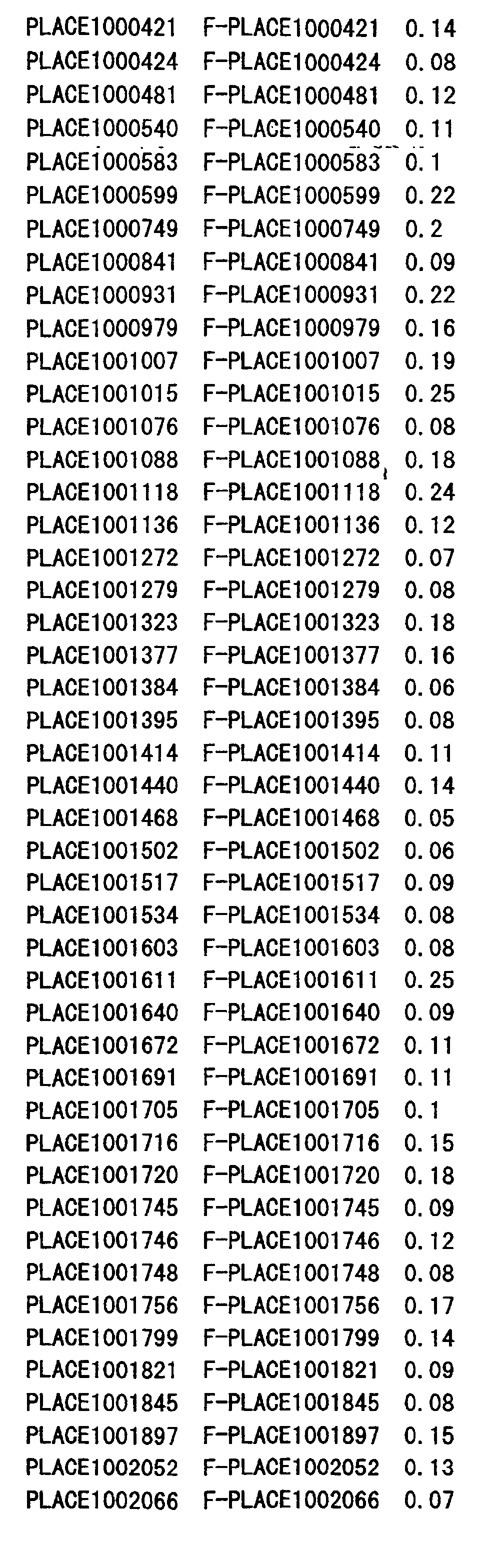

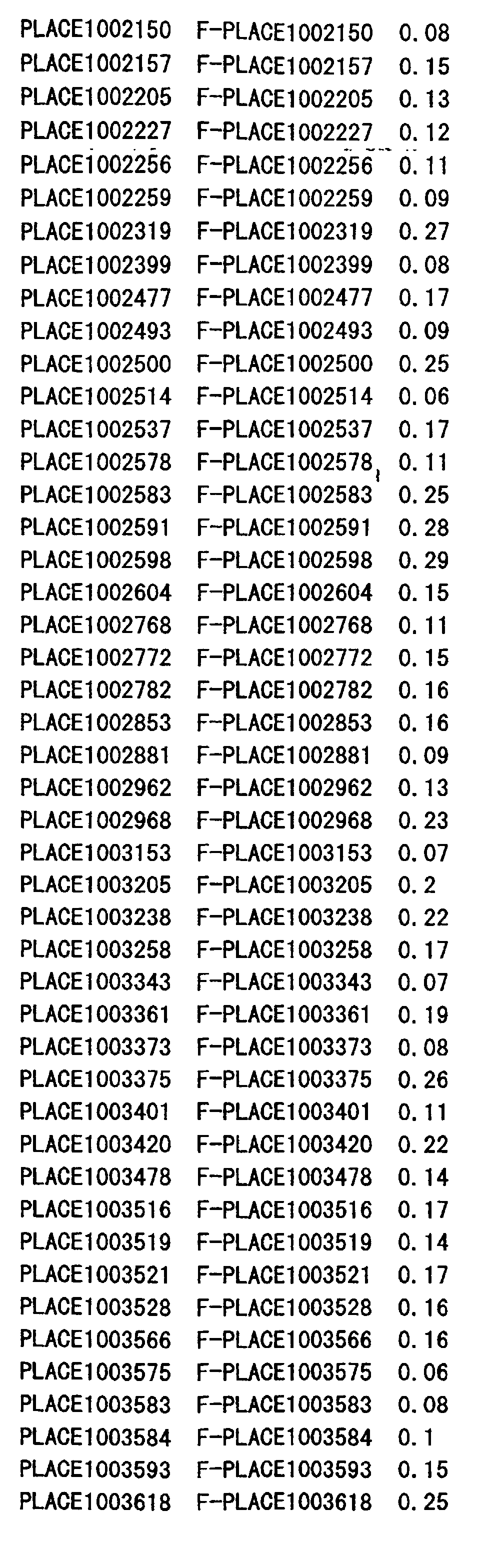

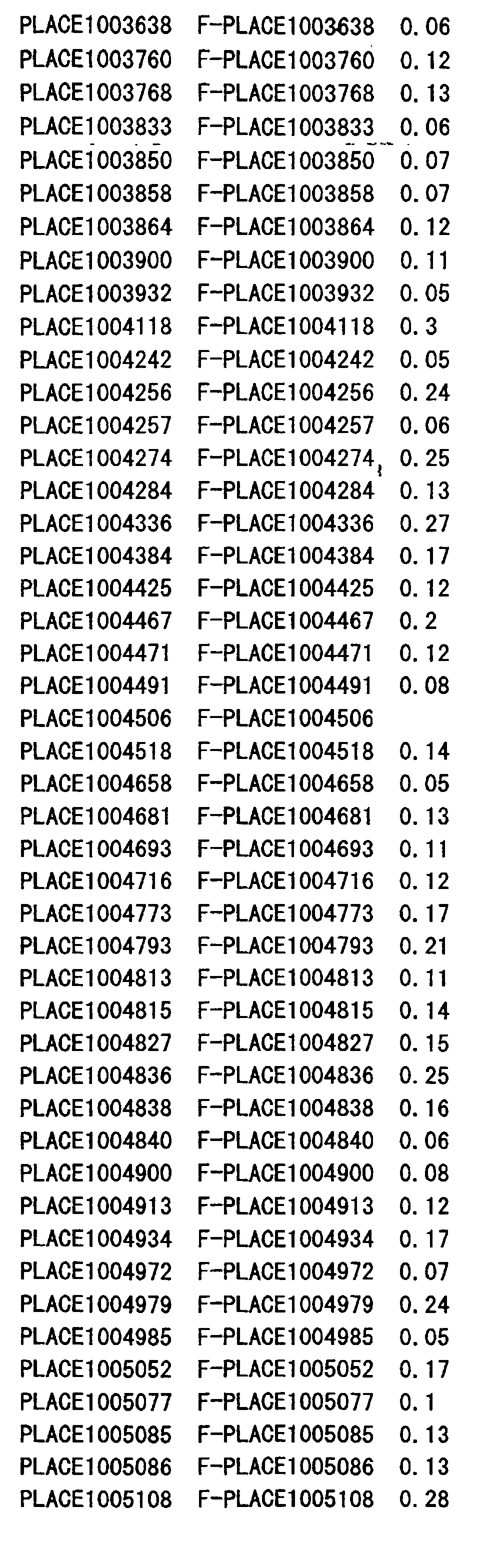

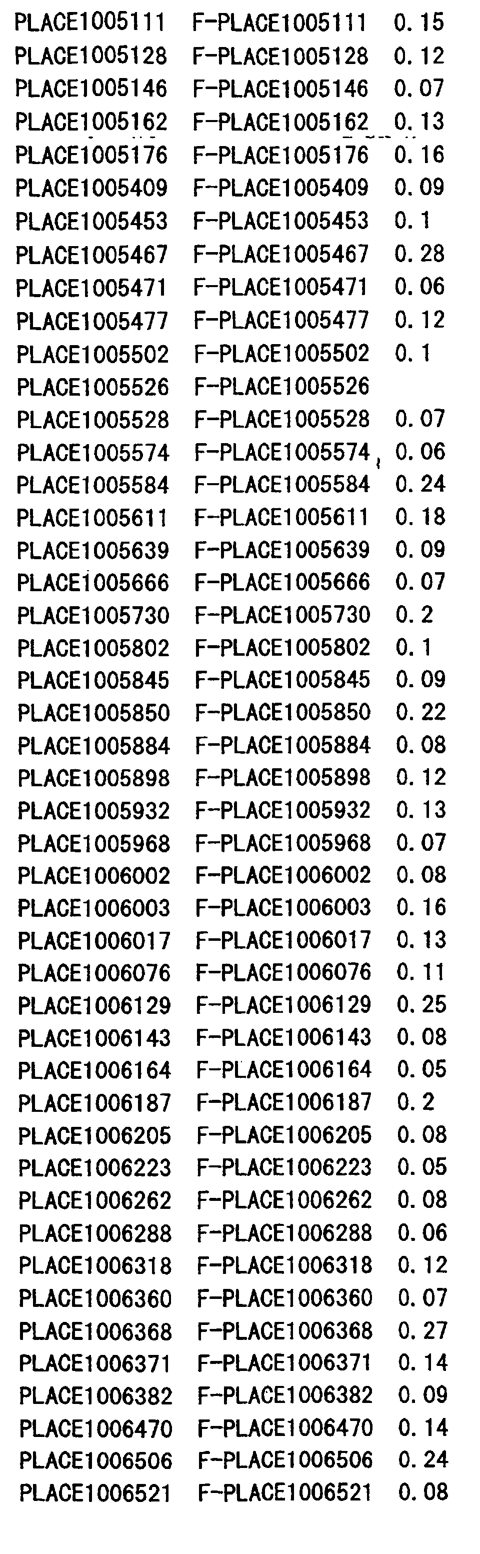

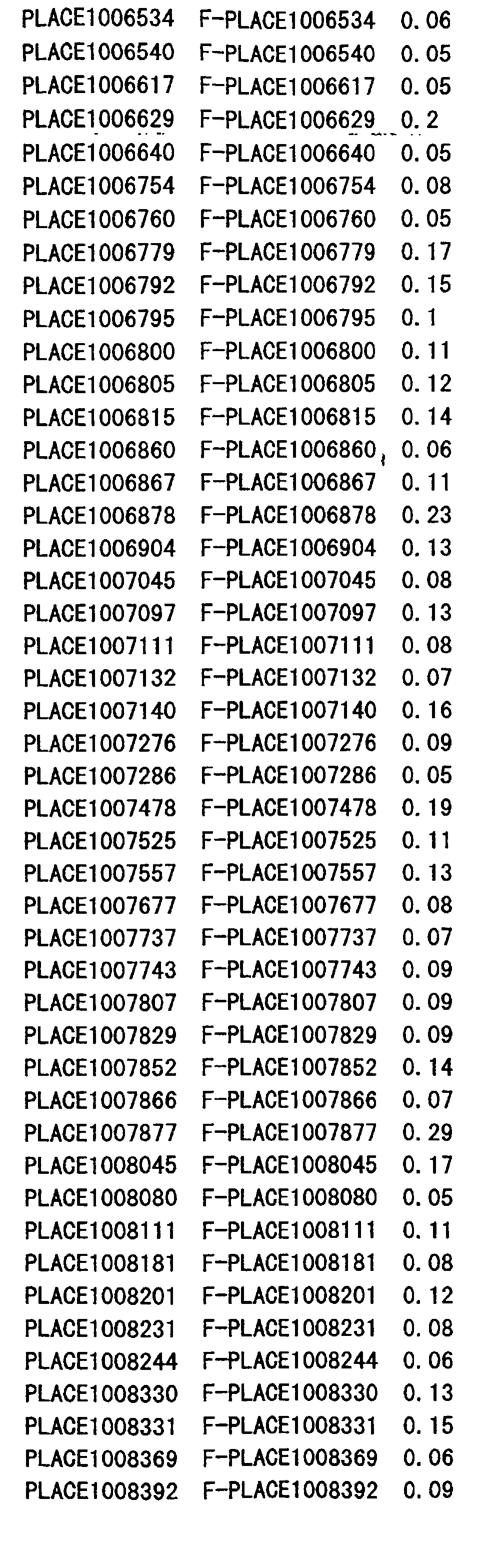

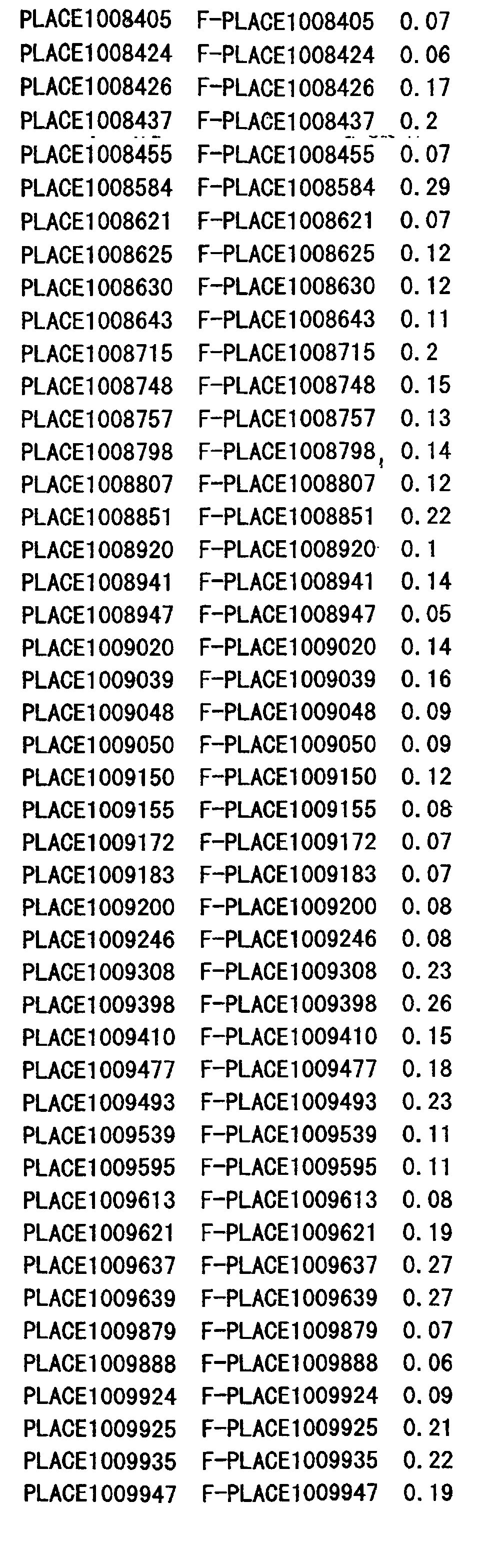

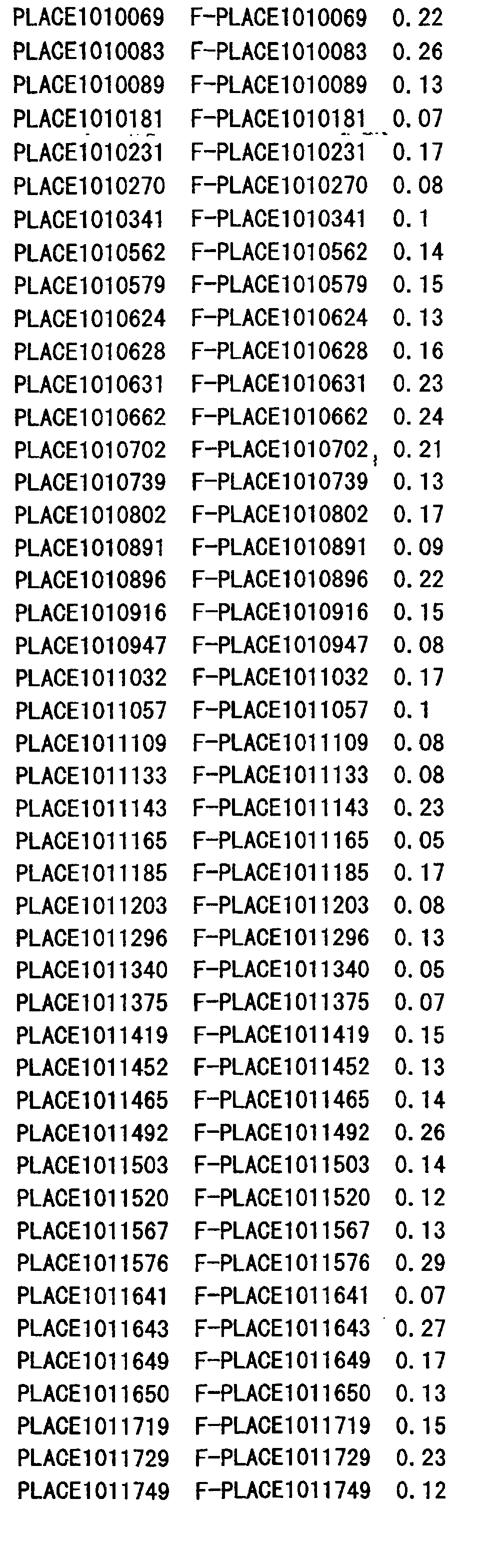

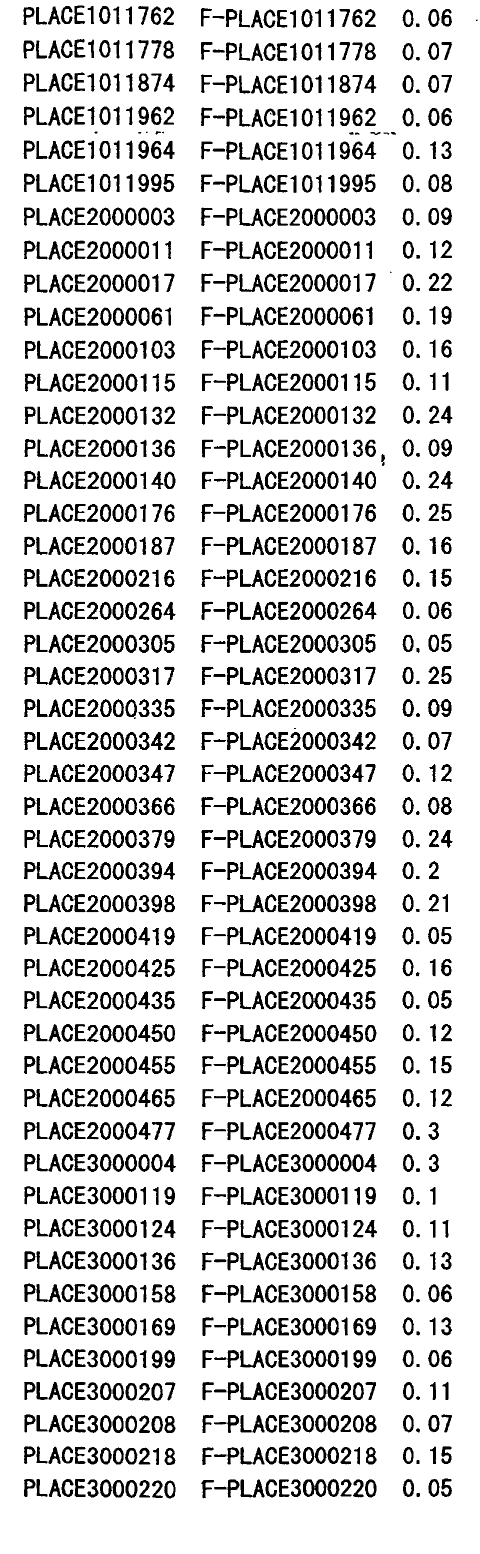

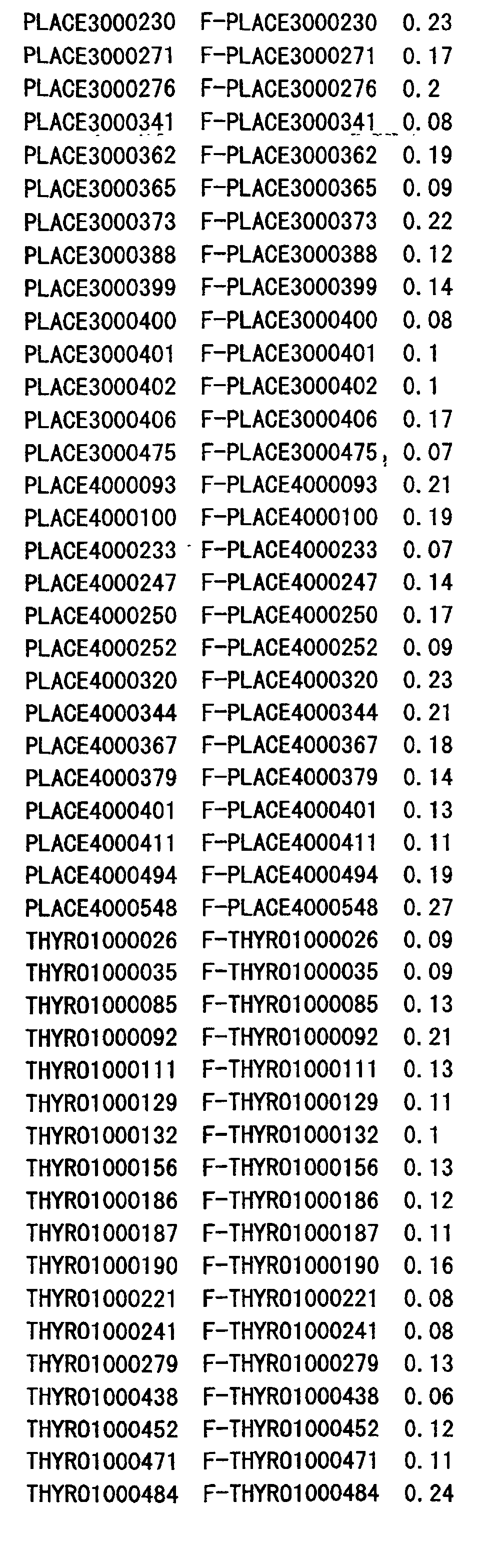

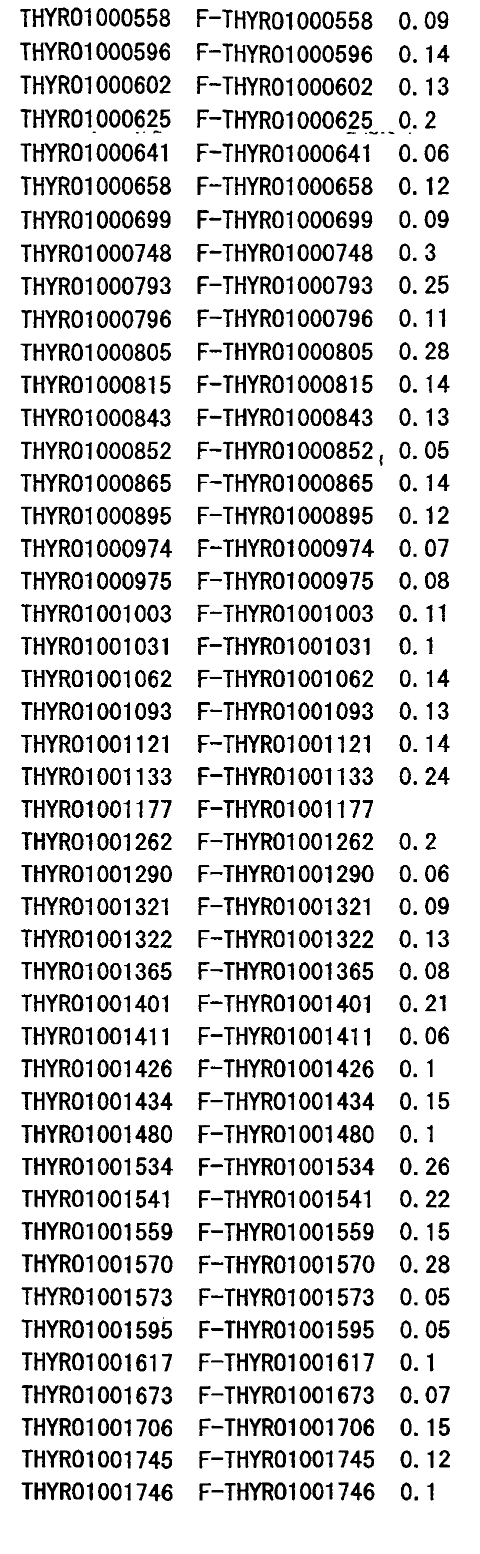

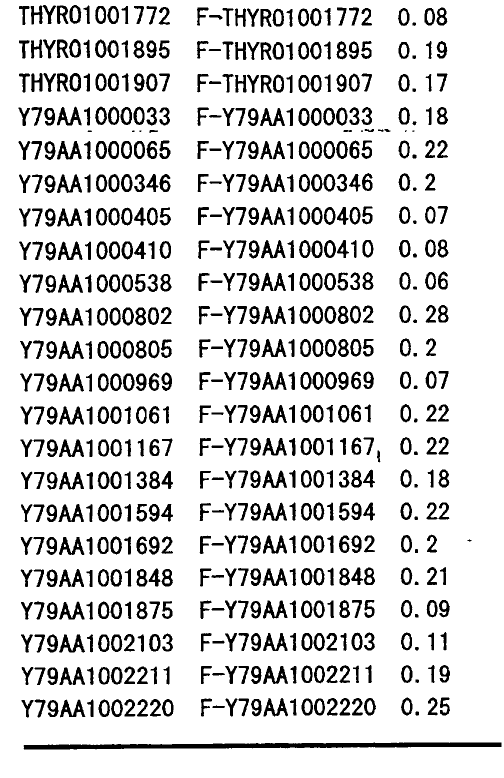

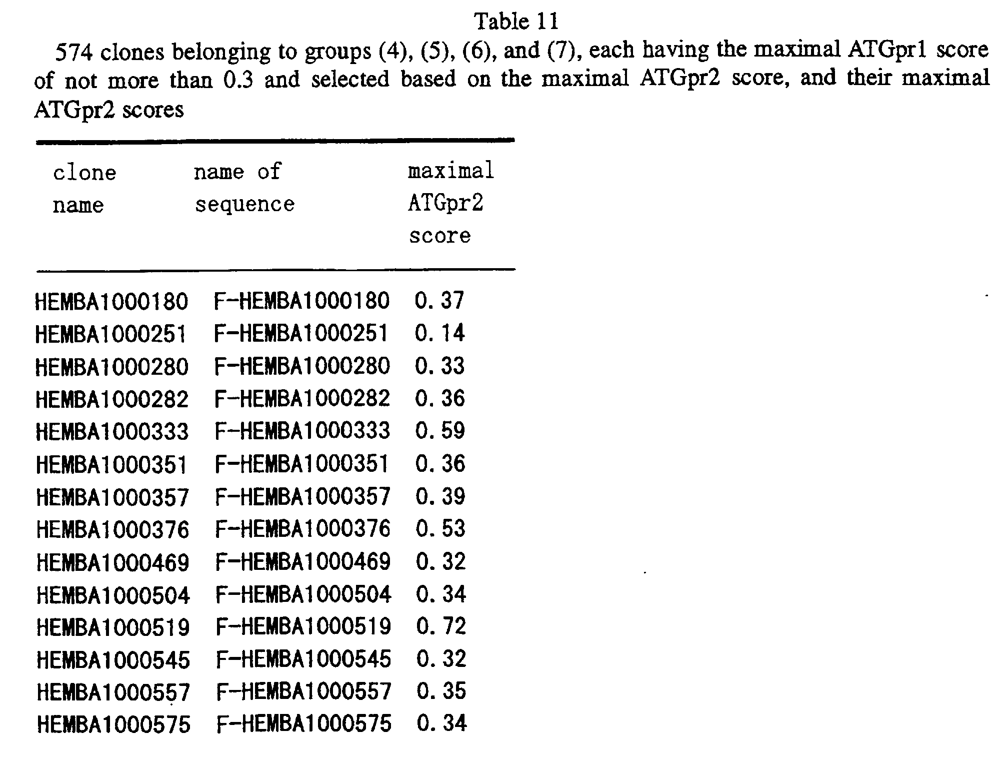

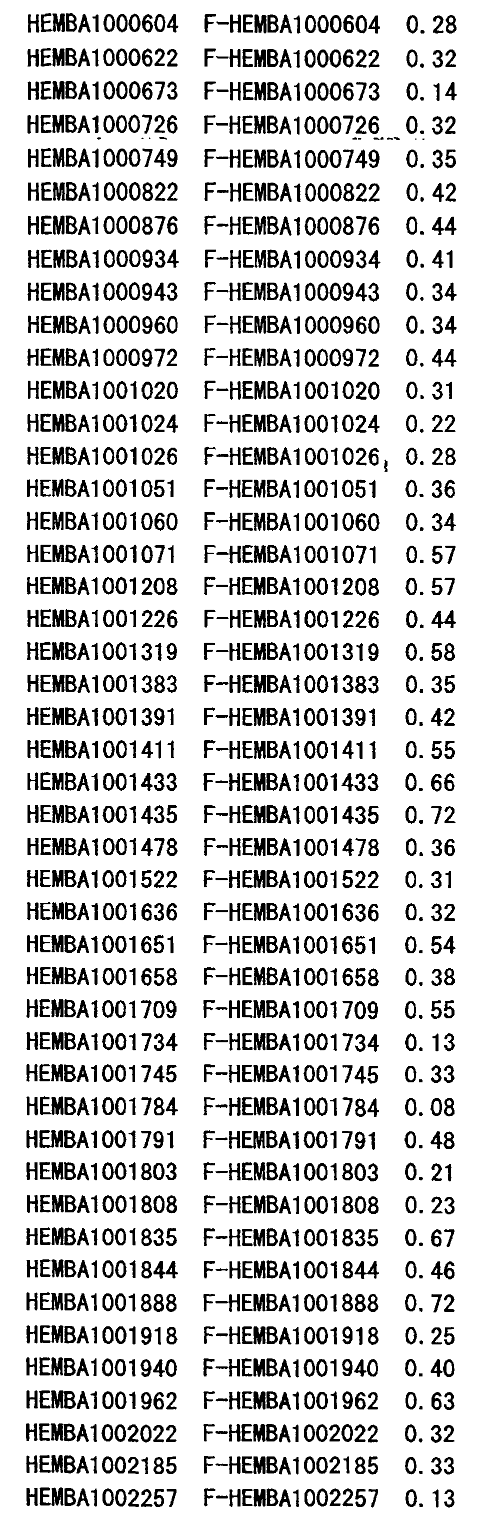

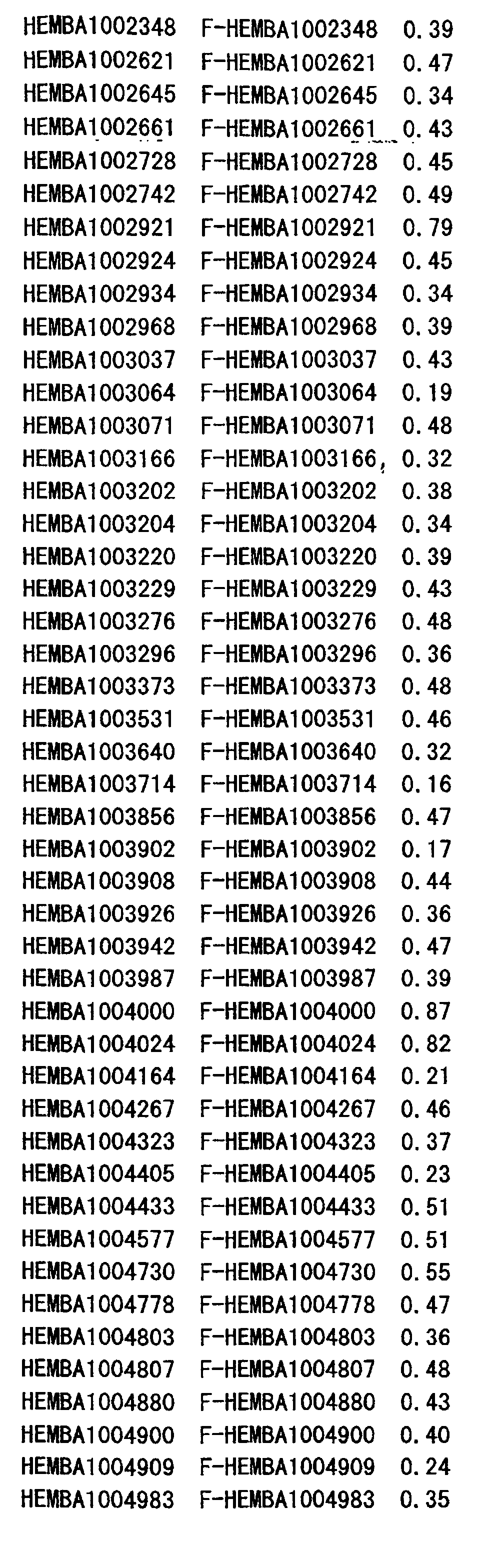

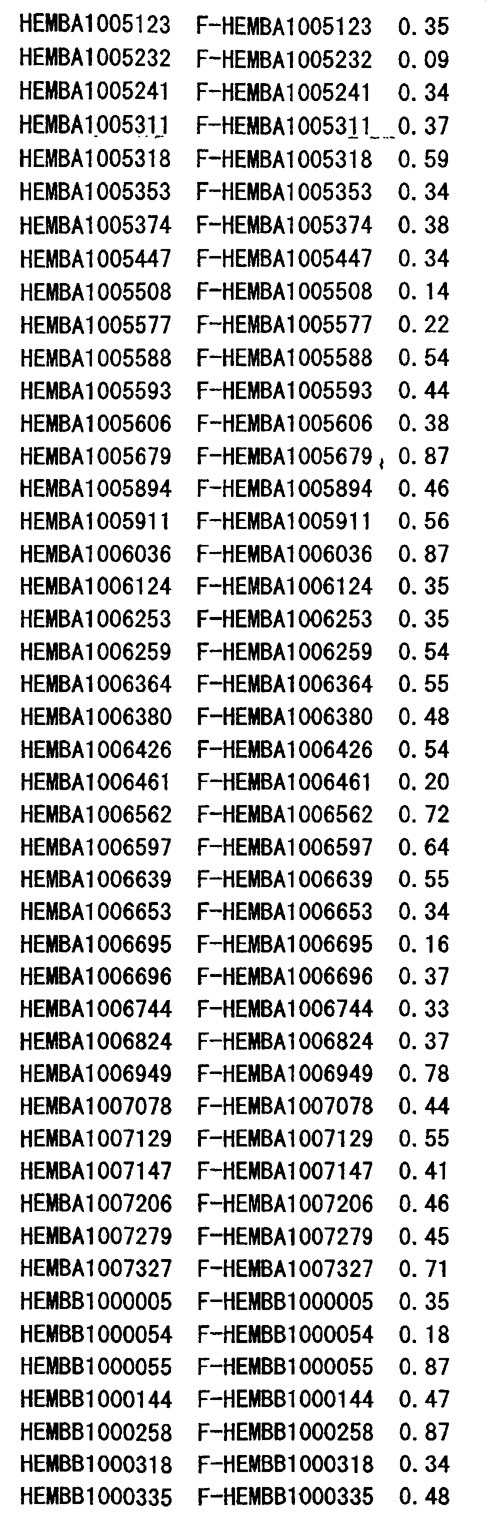

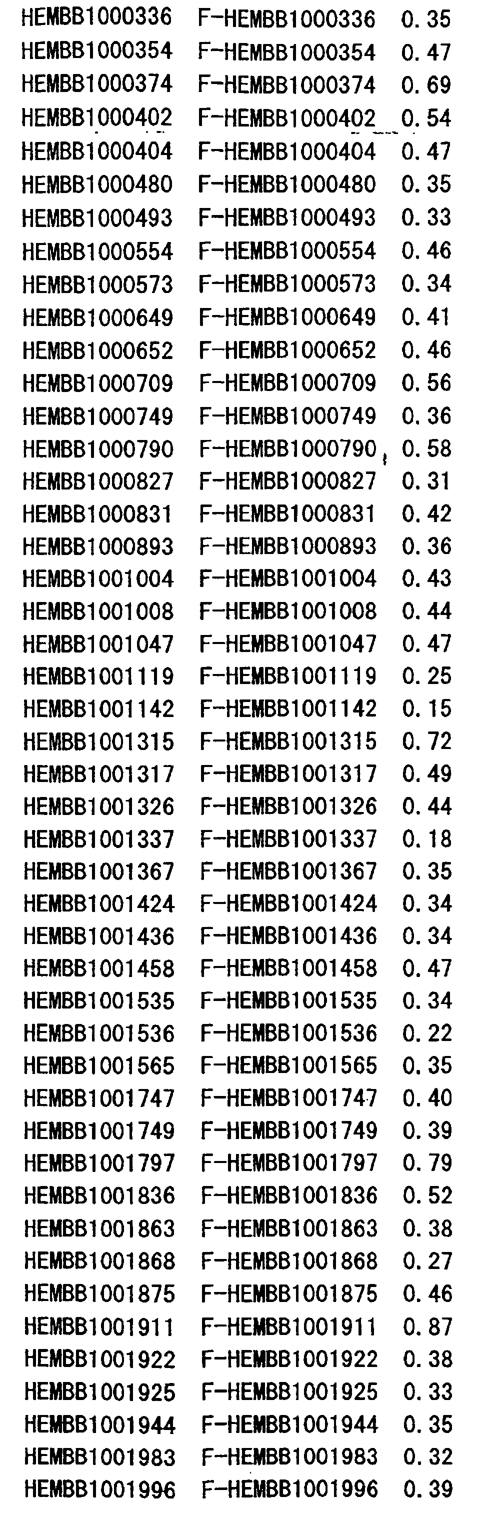

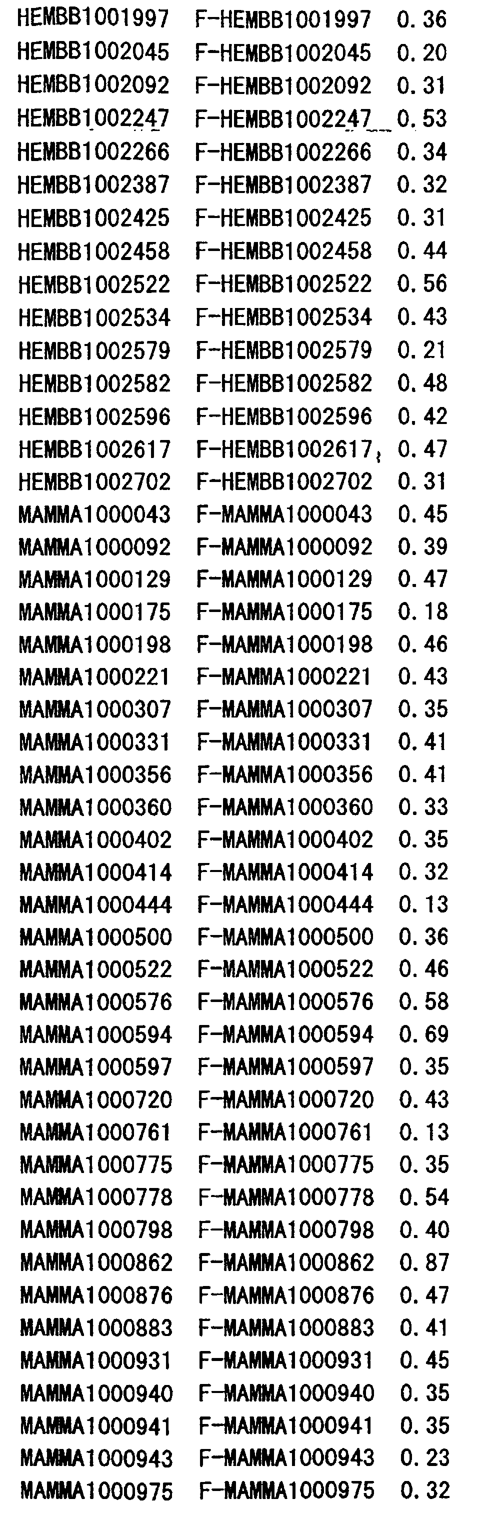

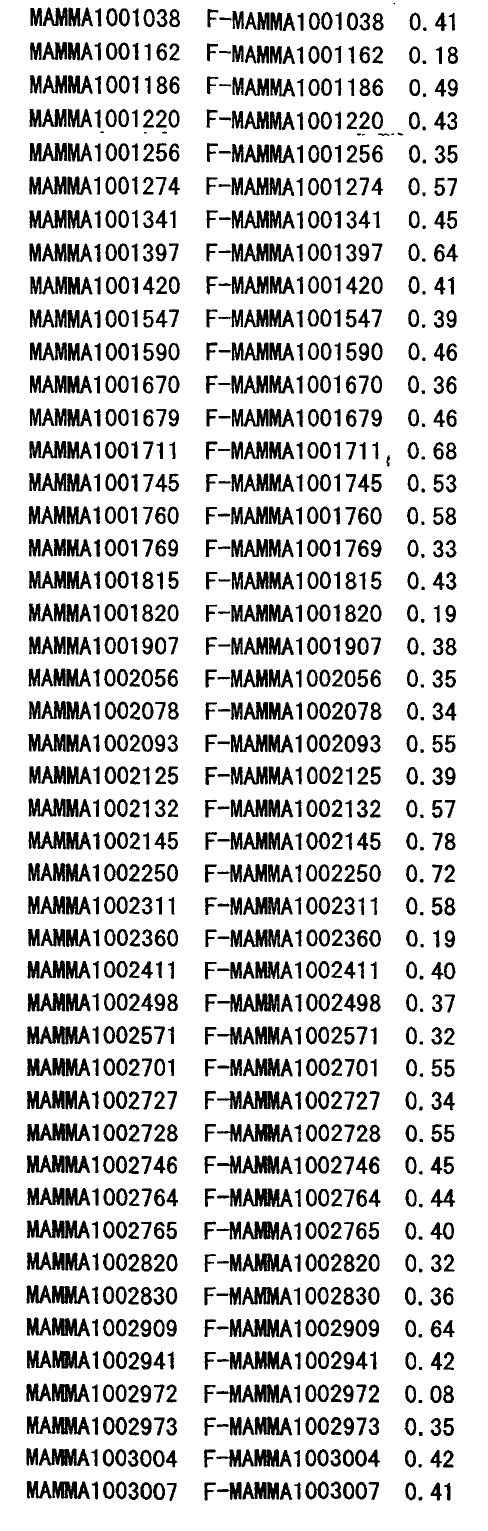

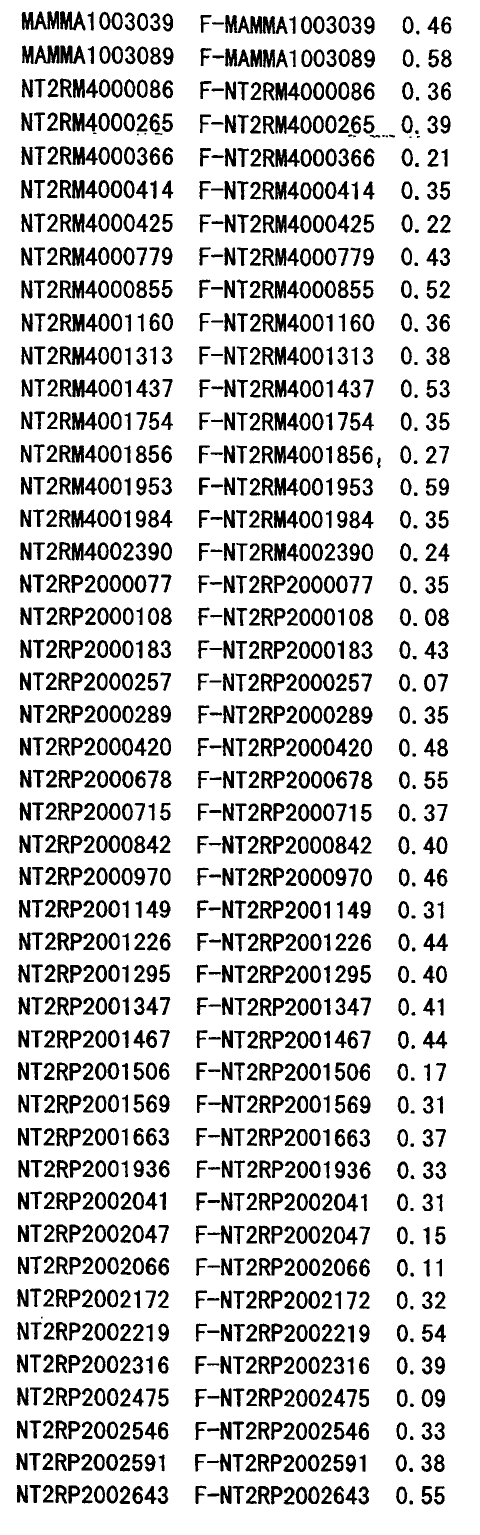

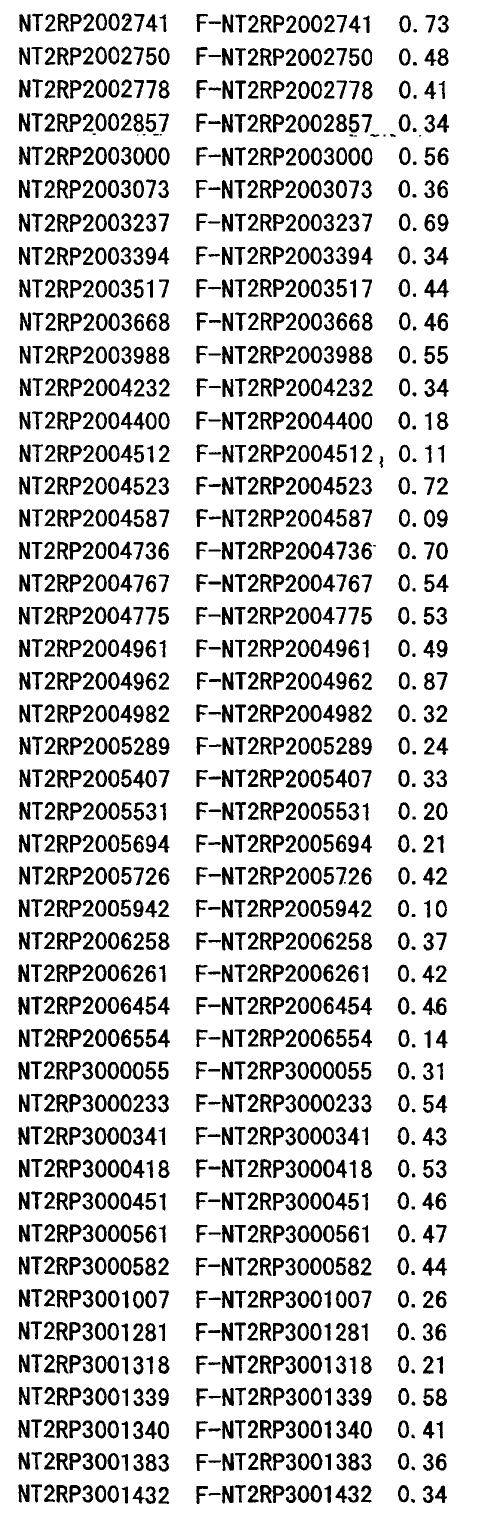

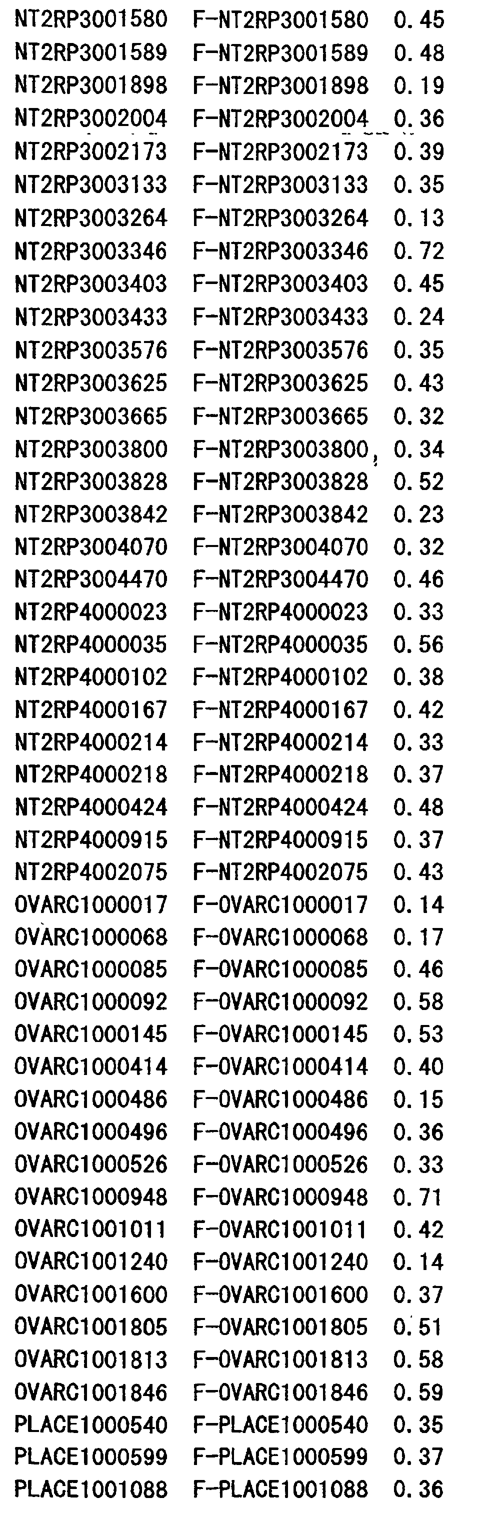

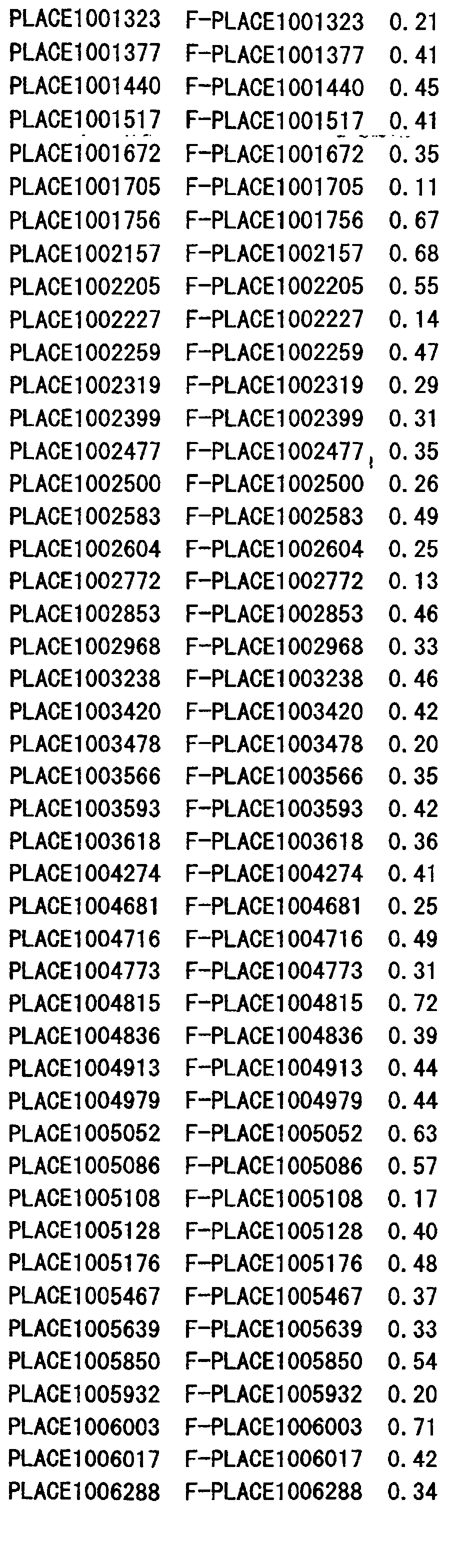

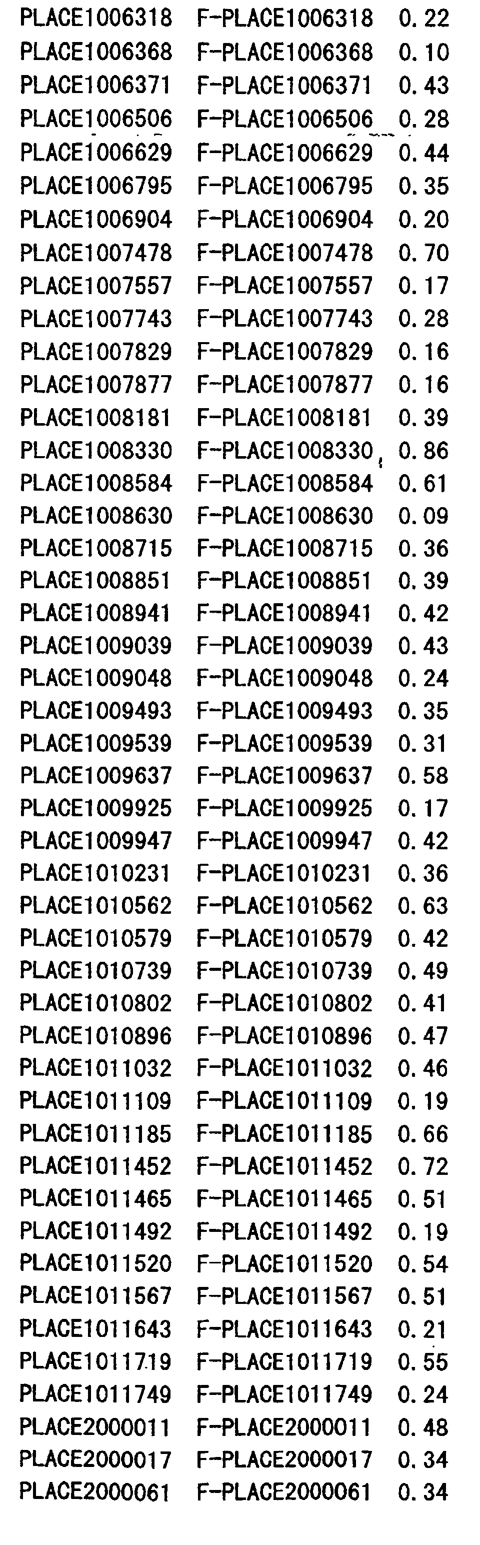

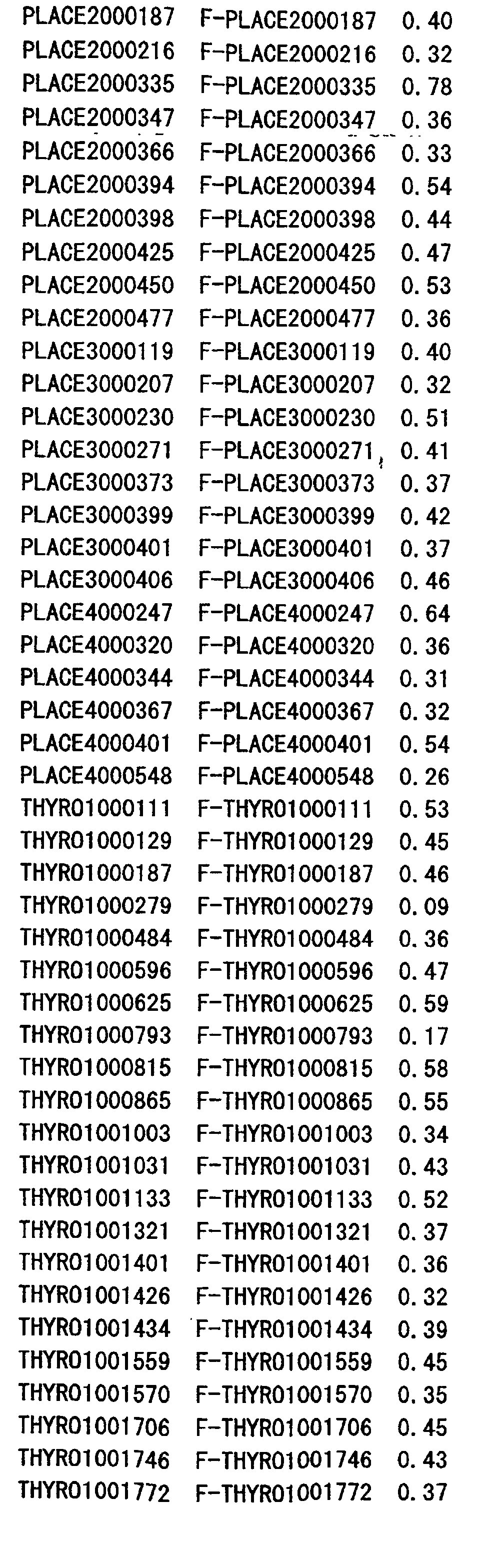

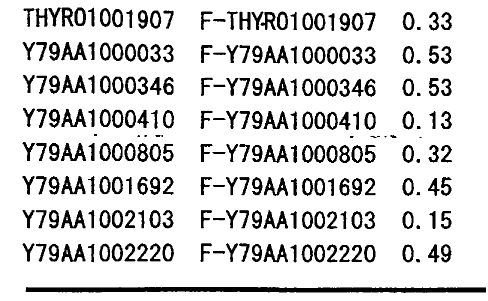

### EXAMPLE 13

### Full-length sequence analysis and homology search

Full-length sequence was determined for each selected cDNA clones. The nucleotide sequence determination was performed mainly by the dye-terminator method using custom synthesized DNA primers according to the primer walking procedure (custom synthesized DNA primers were used for sequencing; sequencing reaction was performed with DNA sequencing reagent supplied by PE Biosystems according to the supplier' s manual; and the samples were analyzed in an automatic sequencer made by the same supplier). Sequence determination of some clones was carried out in the same manner but using a Licor DNA sequencer. Overlapping partial nucleotide sequences, which were obtained by the above-described method, were assembled together to determine a full-length nucleotide sequence. Amino acid sequences were then deduced from the determined full-length nucleotide sequences. However, amino acid sequence is not shown for a clone of which coding region was hard to be deduced or of which amino acid sequence has less than 100 amino acid residues. SEQ ID NOs corresponding to the respective clones are indicated in Table 350 and Table 351.

GenBank, Swiss-Prot and UniGene were searched for the determined nucleotide sequences by BLAST analysis. Matching data of cDNA clone which exhibits higher homology and of which functions are easily predicted based on the nucleotide sequences and the deduced amino acid sequences are selected from the BLAST analysis matching data with P value of 10⁻⁴ or less. The matching data selected are listed herein. The results of homology search 6, 12, 13, and 14 are indicated in the last part of this specification. However, there are some clones that did not match the criteria for judgment and such matching data of BLAST analysis are not shown herein.

### EXAMPLE 14

### Novel full-length cDNA clone obtained from a cDNA library prepared by oligo-capping method

A cDNA clone, NT2RP4002298, was obtained from a cDNA library, NT2RP4 (see Example 1), prepared by oligo-capping method. Analysis of the entire nucleotide sequence of the clone has revealed that the clone encodes a novel protein consisting of 775 amino acids. The ATGpr1 score at the initiation codon of the amino acid sequence is 0.16, and therefore, the fullness ratio is low. However, the sequence can be full-length.

The full-length nucleotide sequence of NT2RP4002298 is shown in SEQ ID NO: 12370, and the deduced amino acid sequence encoded by the clone NT2RP4002298 is shown in SEQ ID NO: 12371.

### EXAMPLE 15

### Gene expression analysis with hybridization using high density DNA filter

Nylon membrane for DNA spotting was prepared according to the following procedure. E. coli was cultured in each well of a 96-well plate (in a LB medium at 37°C for 16 hours). A small aliquot of each culture was suspended in 10 µl of sterile water in a well of a 96-well plate. The plate was heated at 100°C for 10 minutes. Then the boiled samples were analyzed by PCR reaction. PCR was performed in a 20 µl solution by using TaKaRa PCR Amplification Kit (Takara) according to the supplier' s protocol. Primers used for the amplification of an insert cDNA in a plasmid were a pair of sequencing primers, ME761FW (5' tacggaagtgttacttctgc 3' / SEQ ID NO: 13290) and ME1250RV (5' tgtgggaggttttttctcta 3' / SEQ ID NO: 13291), or a pair of primers, M13M4 (5' gttttcccagtcacgac 3' / SEQ ID NO: 13292) and M13RV (5' caggaaacagctatgac 3' / SEQ ID NO: 13293). PCR reaction was performed in a thermal cycler, GeneAmp System 9600 (PE Biosystems). The cycling profile consisted of pre-heat at 95°C for 5 mimutes; 10 cycles of denaturation at 95°C for 10 seconds, and annealing/extension at 68°C for 1 minute; 20 cycles of denaturation at 98°C for 20 seconds and annealing/extension at 60°C for 3 minutes; and final extension at 72°C for 10 minutes. After the PCR reaction, the 20 µl reaction solution was loaded onto a 1 % agarose gel and fractionated by electrophoresis. DNA on the gel was stained with ethidium bromide to confirm the amplification of cDNA. When cDNAs were barely amplified by PCR, plasmids containing the corresponding insert cDNAs were prepared by the alkali-extraction method (J. Sambrook, E.F., Fritsh, & T. Maniatis, "Molecular Cloning, A laboratory manual/ 2nd edition, Cold Spring Harbor Laboratory Press, 1989).

Preparation of DNA array was carried out by the following procedure. An Aliquot of a DNA solution was added in each well of a 384-well plate. DNA was spotted onto a nylon membrane (Boehringer) by using a 384-pin tool of Biomek 2000 Laboratory Automation Sysytem (Beckman-Coulter). Specifically, the 384-well plate containing the DNA was placed under the 384-pin tool. The independent 384 needles were simultaneously dipped into the DNA solution for DNA deposition. The needles were gently pressed onto a nylon membrane and the DNA deposited at the tips of needles was spotted onto the membrane. Denaturation of the spotted DNA and immobilization of the DNA on the nylon membrane were carried out according to usual manners (J. Sambrook, E.F., Fritsh, & T. Maniatis, "Molecular Cloning, A laboratory manual/ 2nd edition, Cold Spring Harbor Laboratory Press, 1989).

Hybridization probe used was radioisotope-labeled 1st strand cDNA. The 1st strand cDNA synthesis was performed by using Thermoscript^{(TM)} RT-PCR System (GIBCO). Specifically, the 1st strand cDNA was synthesized by using 1.5 µg mRNAs from various human tissues (Clontech), 1 µl aliquots of 50 µM Oligo(dT)20 and 50 µCi [α ³³P]dATP according to an attached protocol. Probe purification was carried out by using ProbeQuant^{(TM)} G-50 micro column (Amersham-Pharmacia Biotech) according to an attached protocol. In the next step, 2 units of E. coli RNasH were added to the reaction mixture. The mixture was incubated at room temperature for 10 minutes and then 100 µg of human COT-1 DNA (GIBCO) was added thereto. The mixture was incubated at 97°C for 10 minutes and then was allowed to stand on ice to give hybridization probe.

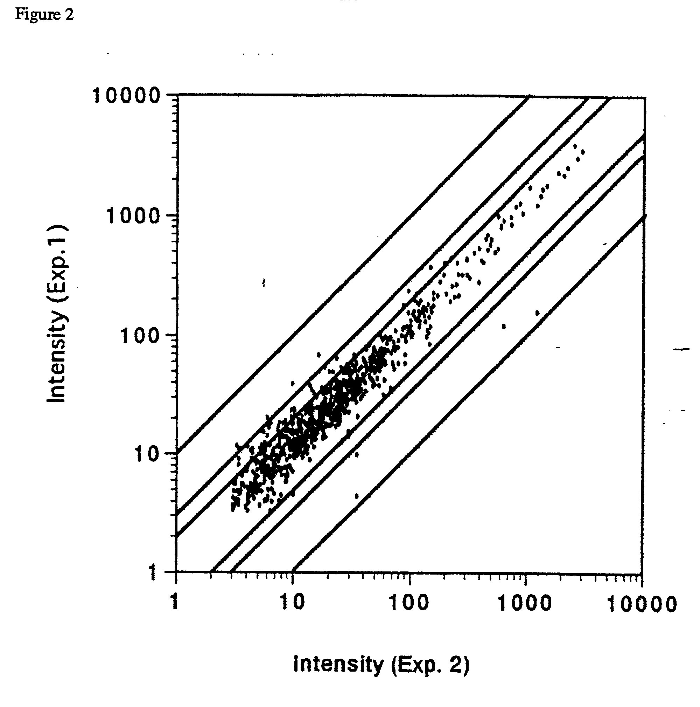

Hybridization of the radioisotope-labeled probe to the DNA array was performed in a usual manner (J. Sambrook, E.F., Fritsh, & T. Maniatis, Molecular Cloning, A laboratory manual/ 2nd edition, Cold Spring Harbor Laboratory Press, 1989). The membrane was washed as follows: the nylon membrane was incubated in Washing solution 1 (2 × SSC, 1% SDS) at room temperature (about 26°C) for 20 minutes and this washing was repeated 3 times; then the membrane was washed 3 times by incubating it in Washing solution 2 (0.1 × SSC, 1% SDS) at 65°C for 20 minutes. Autoradiography was performed by using an image plate for BAS2000 (Fuji Photo Film Co., Ltd.). Specifically, the nylon membrane with probe hybridized thereon was wrapped with a piece of Saran Wrap and brought into tight contact with the image plate on the light-sensitive surface. The membrane with the image plate was placed in an imaging cassette for radioisotope and allowed to stand in dark place for 4 hours. The radioactivity recorded on the image plate was analyzed by using BAS2000 (Fuji Photo Film Co., Ltd.). The activity was subjected to electronic conversion and recorded as an image file of autoradiogram. The signal intensity of each DNA spot was analyzed by using Visage High Density Grid Analysis Systems (Genomic Solutions Inc.). The signal intensity was converted into numerical data. The data were taken by duplicated measurements. The reproducibility was assessed by comparing the signal intensities of the corresponding spots on the duplicated DNA filters that were hybridized to a single DNA probe (Figure 2). The ratio between the corresponding spots falls within a range of 2 or less in 95% of entire spots and the correlation coefficient is r=0.97. Thus the reproducibility is assumed to be satisfactory.

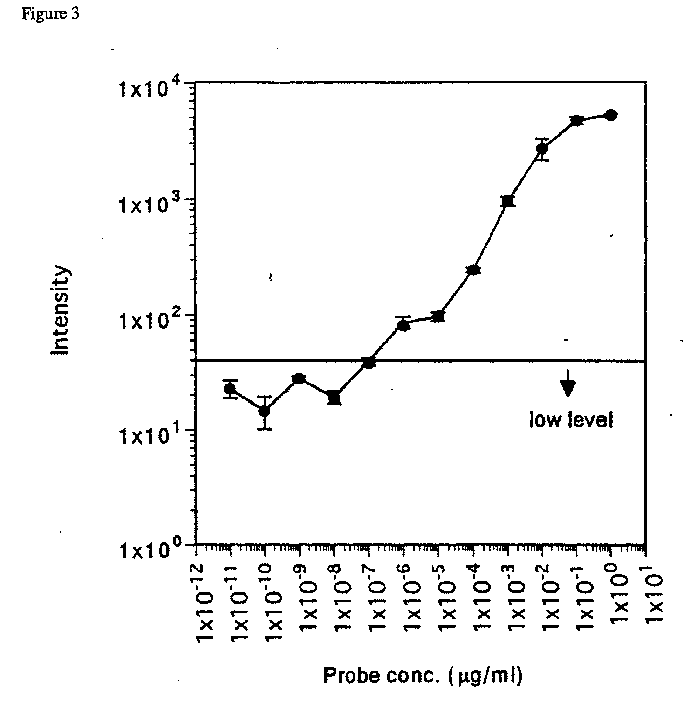

The detection sensitivity in gene expression analysis was estimated by examining increases in the signal intensity of probe concentration-dependent spot in hybridization using a probe complementary to the DNA spotted on the nylon membrane. DNA used was PLACE1008092 (same as DNA deposited in GenBank under an Accession No. AF107253). The DNA array with DNA of PLACE1008092 was prepared according to the above-mentioned method. The probe used was prepared as follows: mRNA was synthesized in vitro from the clone, PLACE1008092. By using this mRNA as a template, radioisotope-labeled 1st strand cDNA was synthesized in the same manner as described above, and the cDNA was used as the probe. The cDNA PLACE1008092 was inserted into pBluescript SK(-), of which T7 promoter was ligated to the 5' end of the cDNA, to give a plasmid for in vitro synthesis of the mRNA from PLACE1008092. Specifically, the PLACE1008092 insert was cut out from pME18SFL3 carrying the cDNA at a DraIII site thereof by XhoI digestion. The resulting PLACE1008092 fragment was ligated to XhoI-predigested pBluescript SK(-) by using DNA ligation kit ver.2 (Takara). The in-vitro mRNA synthesis from PLACE1008092 inserted in pBluescript SK(-) was carried out by using Ampliscribe^{(TM)} T7 high yield transcription kit (Epicentre technologies). Hybridization and the analysis of signal intensity of each DNA spot were conducted by the same methods as described above. When the probe concentration is 1 × 10⁷ µg/ml or less, there was no increase of signal intensity proportional to the probe concentration. Therefore it was assumed to be difficult to compare the signals with one another in the concentration range. Thus the spots with the intensity of 40 or less were indiscriminately taken as low-level signals (Figure 3). Within a concentration of the probe ranging from 1 × 10⁷ µg/ml to 0.1 µg/ml, the signal was found to increase in a probe concentration-dependent manner. The detection limit was then assumed to be 1:100,000 in a ratio of mRNA expression level in a sample.

Tables 12-168 (also containing clones with no description in Examples) show the expression of each cDNA in human normal tissues (heart, lung, pituitary gland, thymus, brain, kidney, liver and spleen). The expression levels are indicated by numerical values of 0-10,000. Genes that were expressed in at least a single tissue are indicated below by the corresponding clone names:

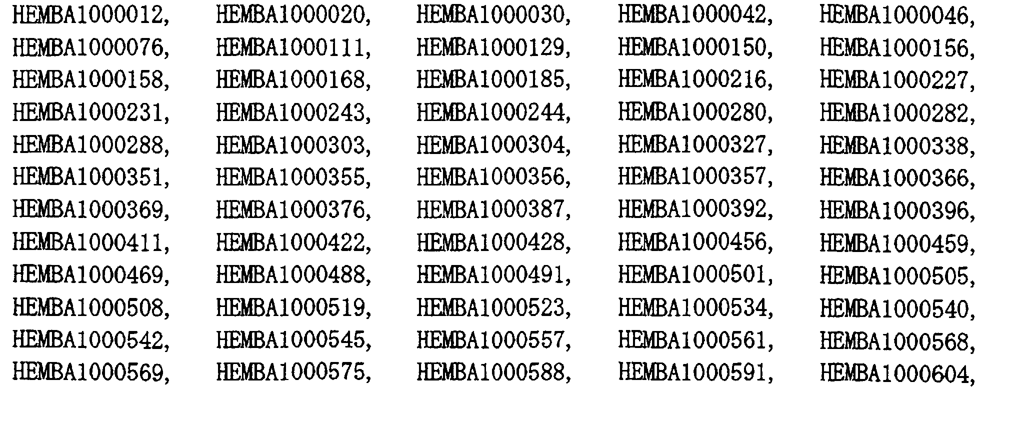

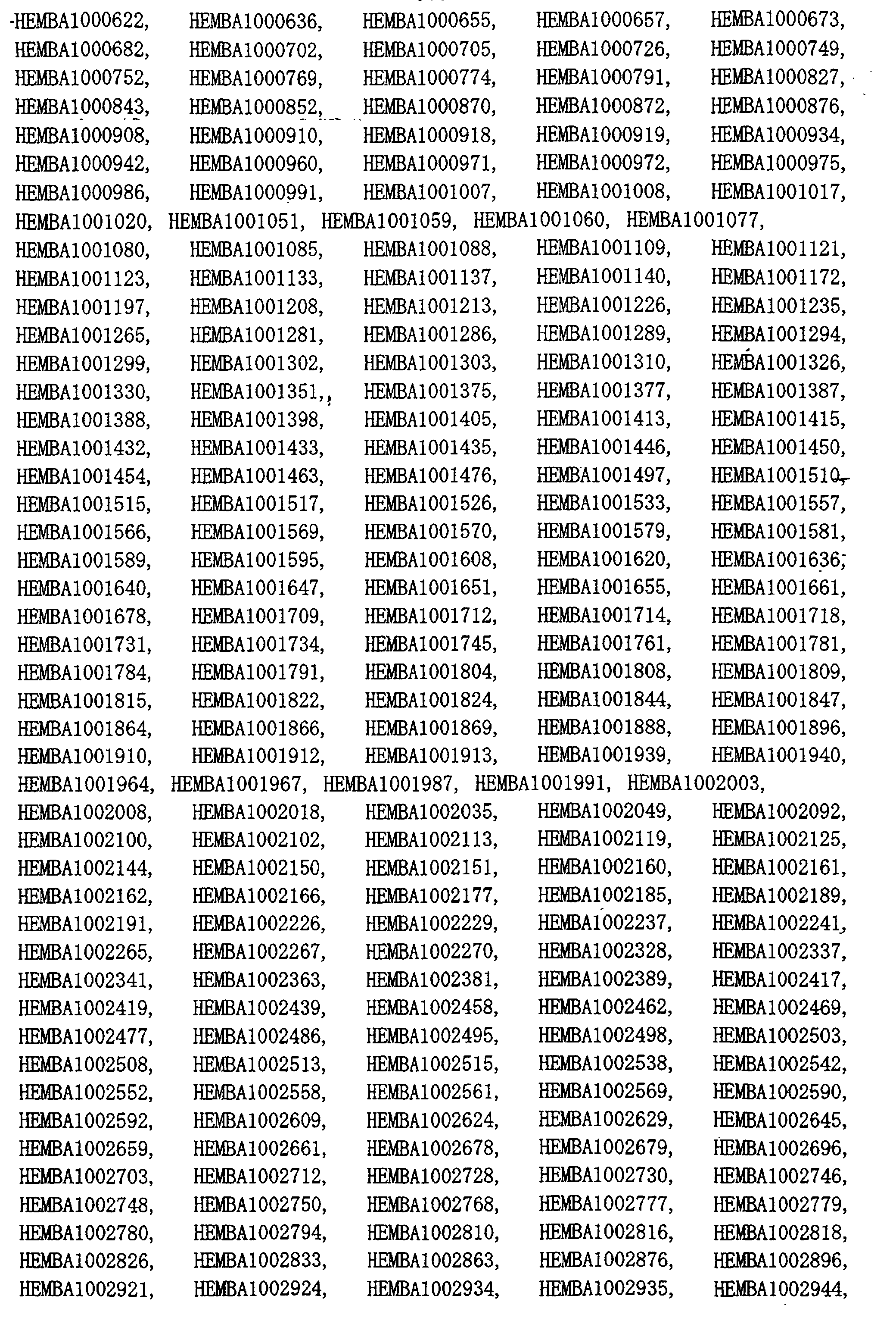

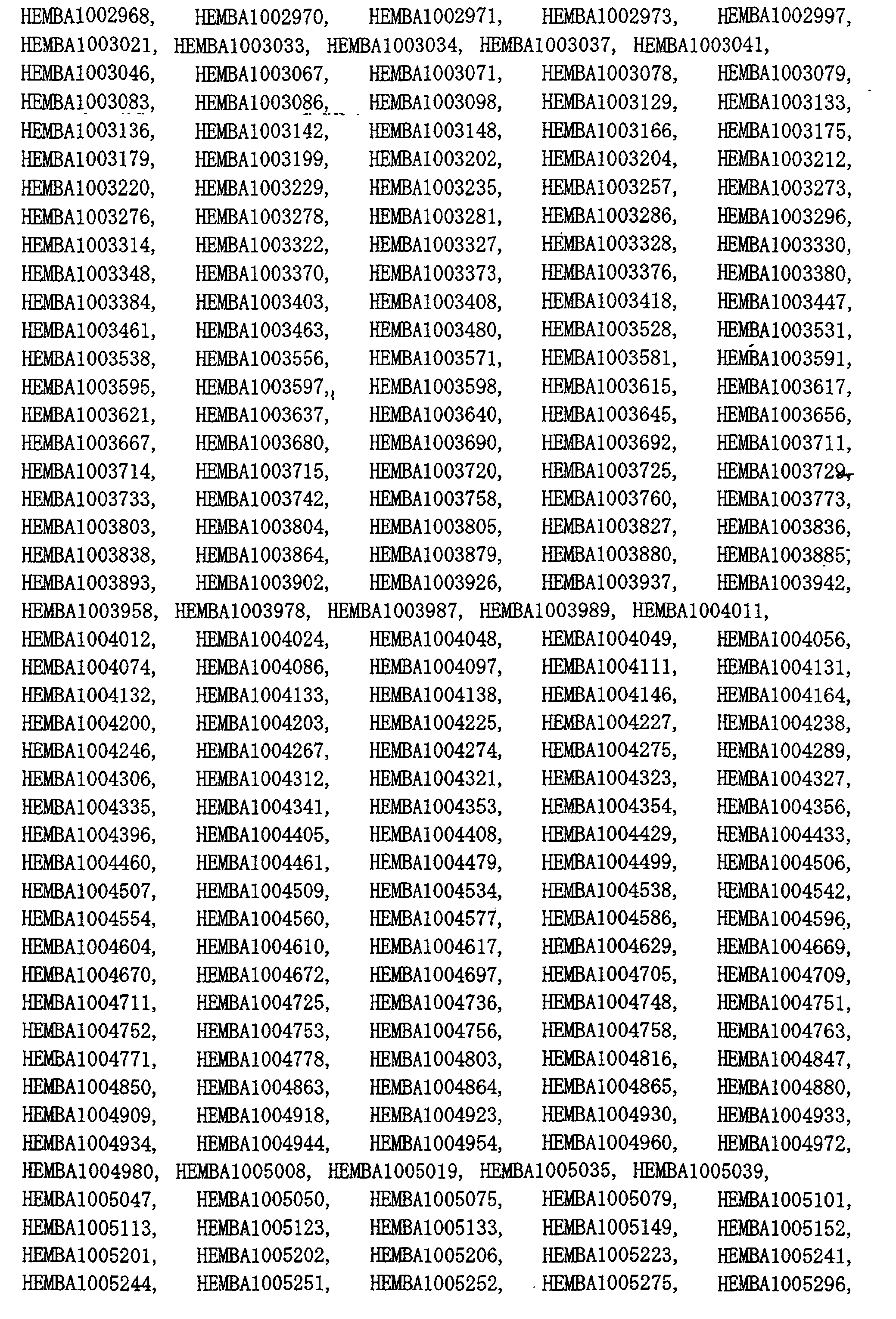

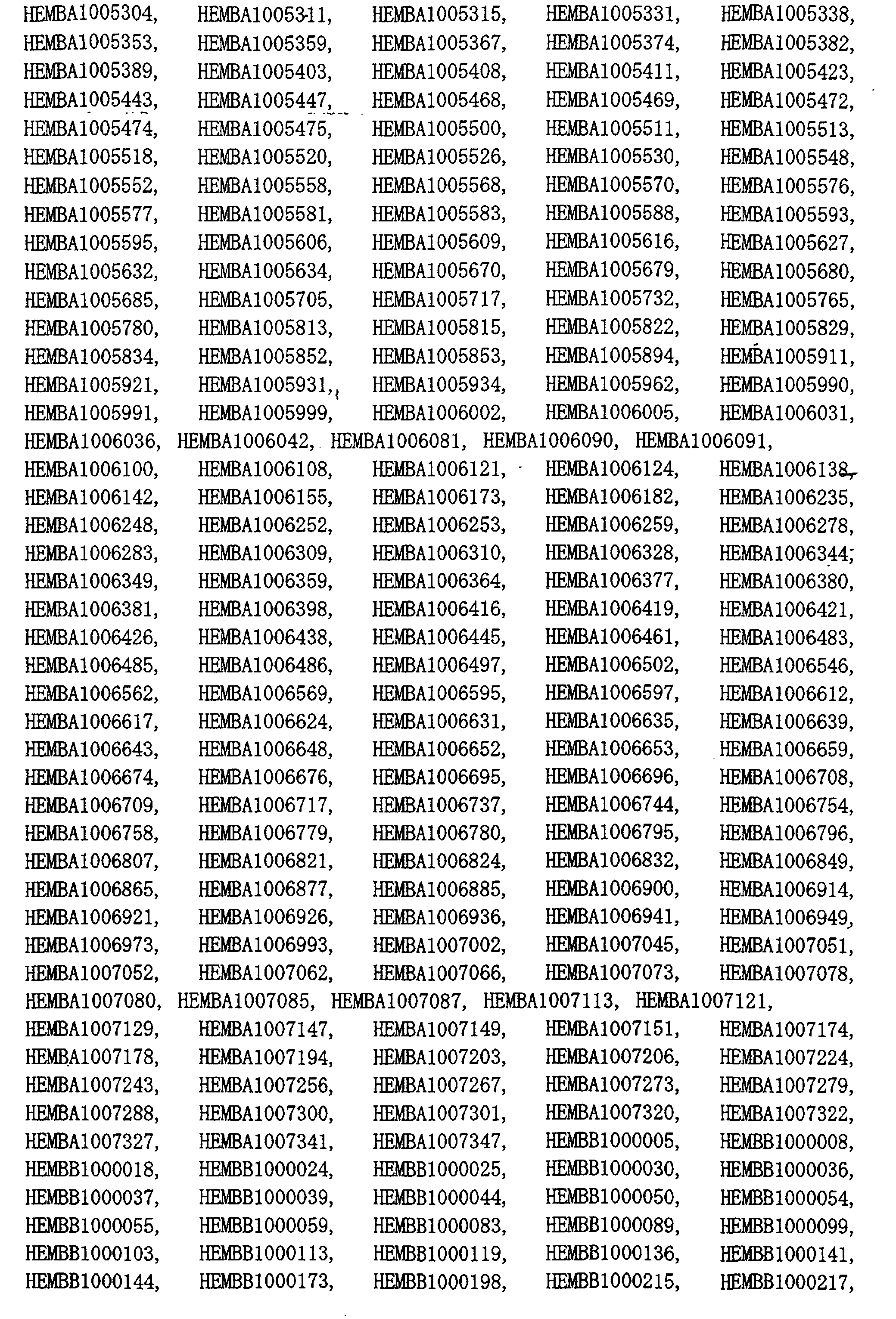

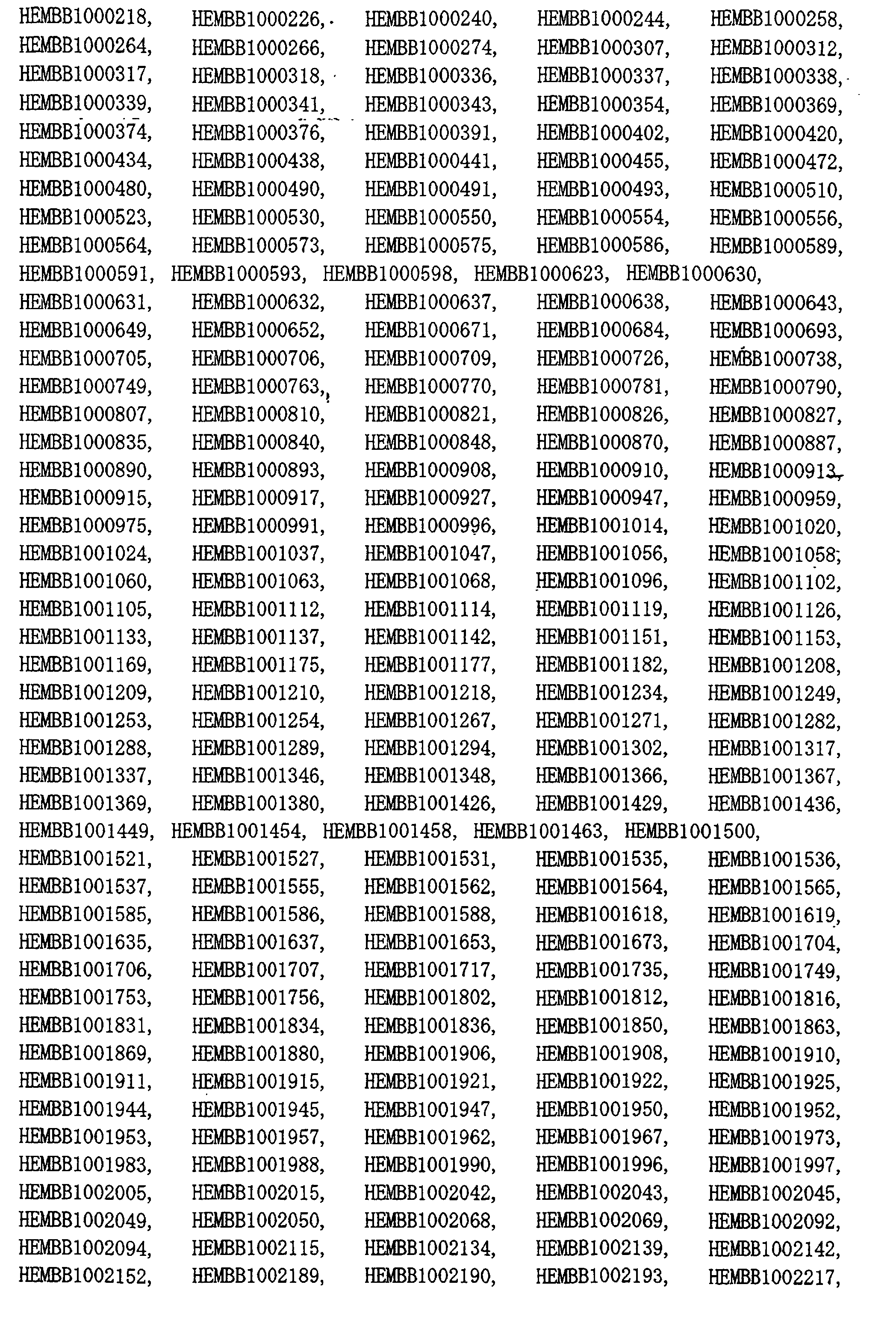

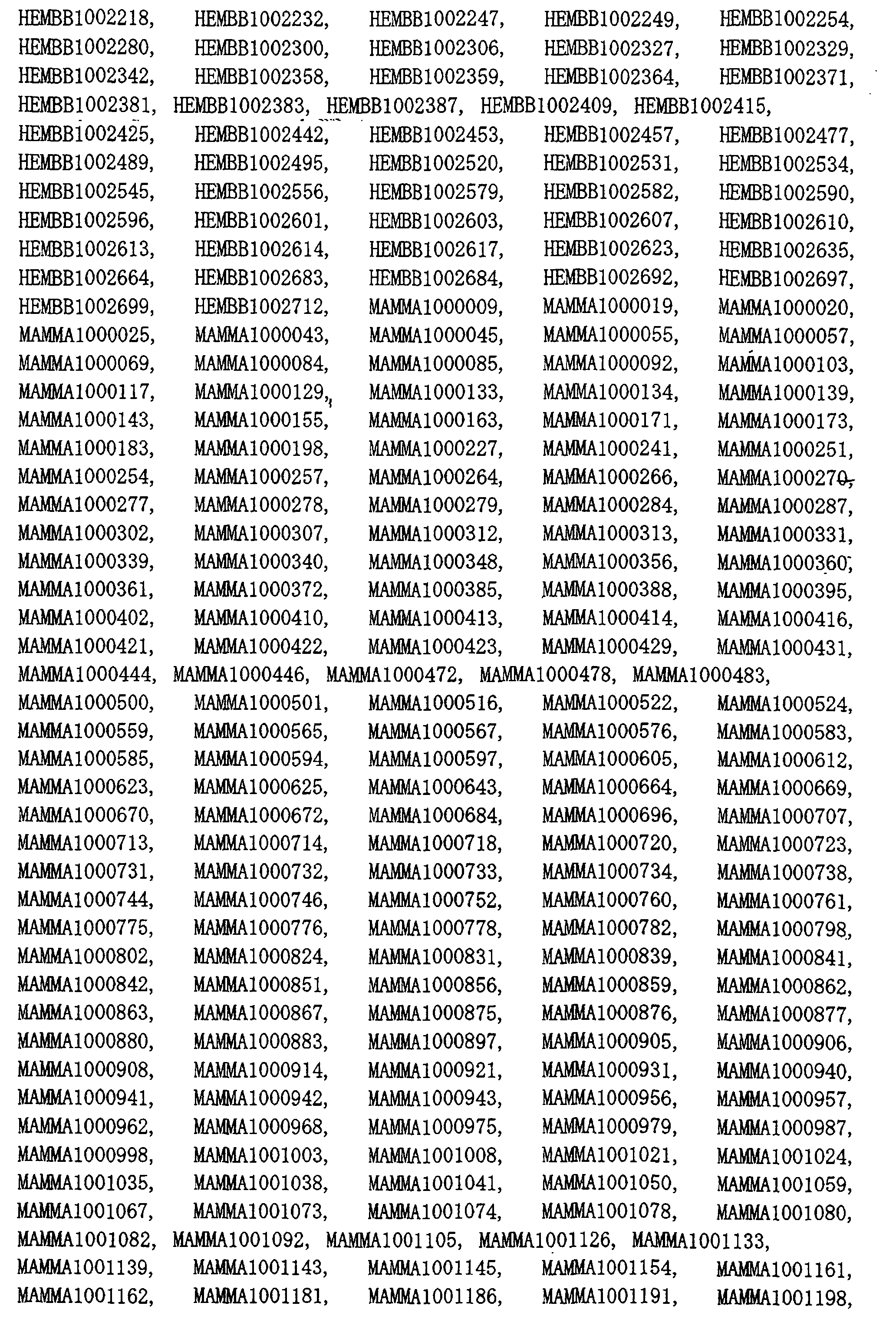

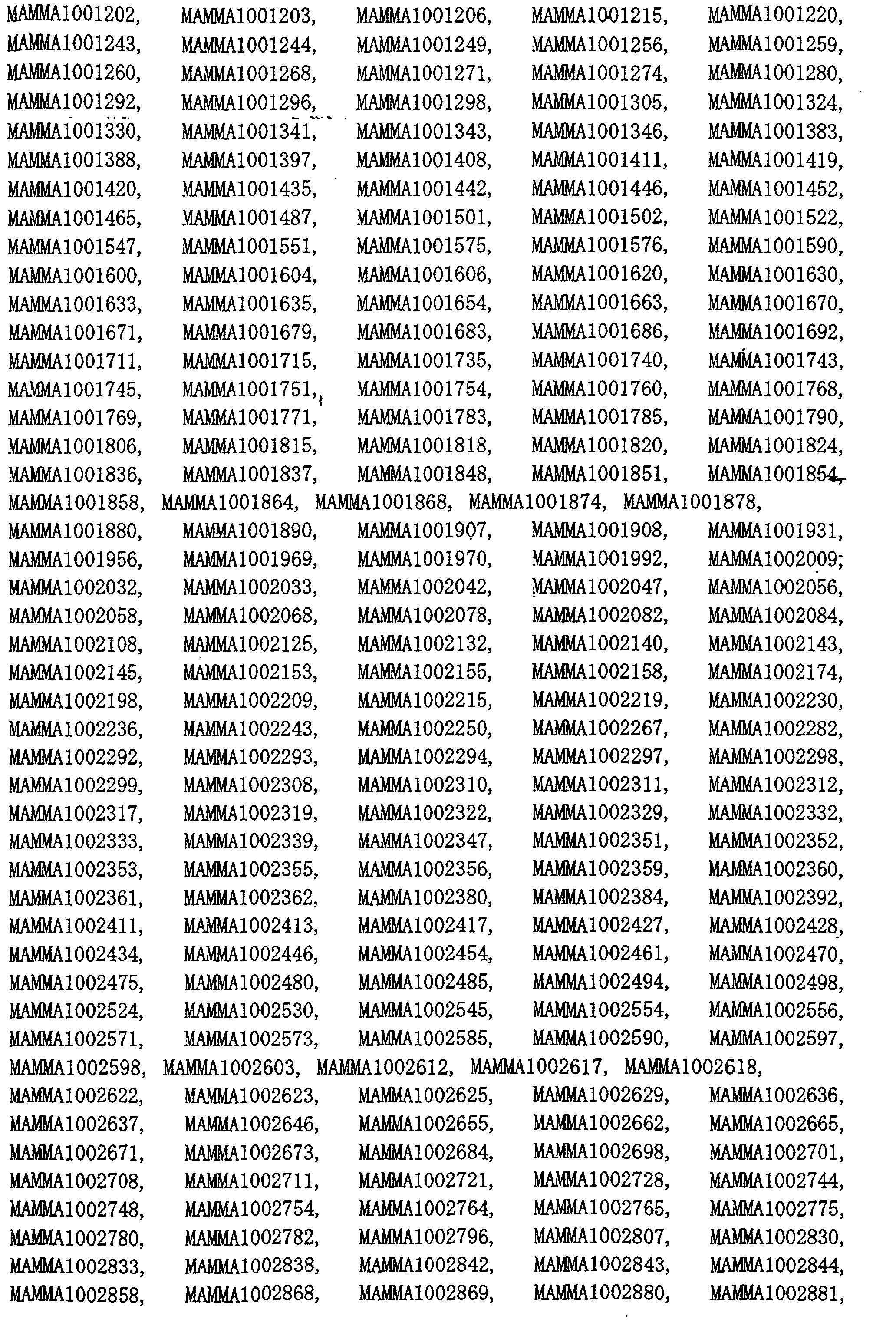

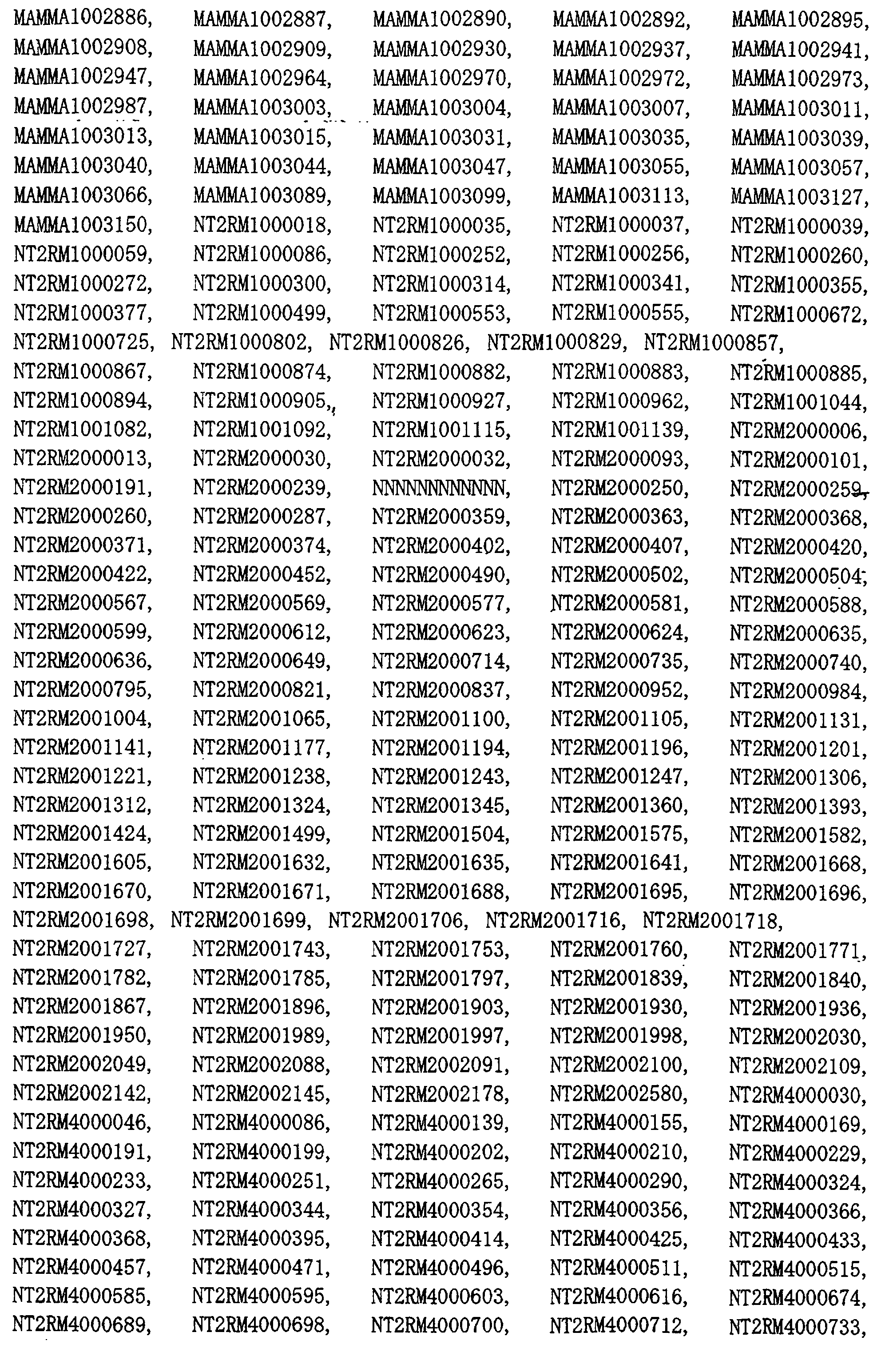

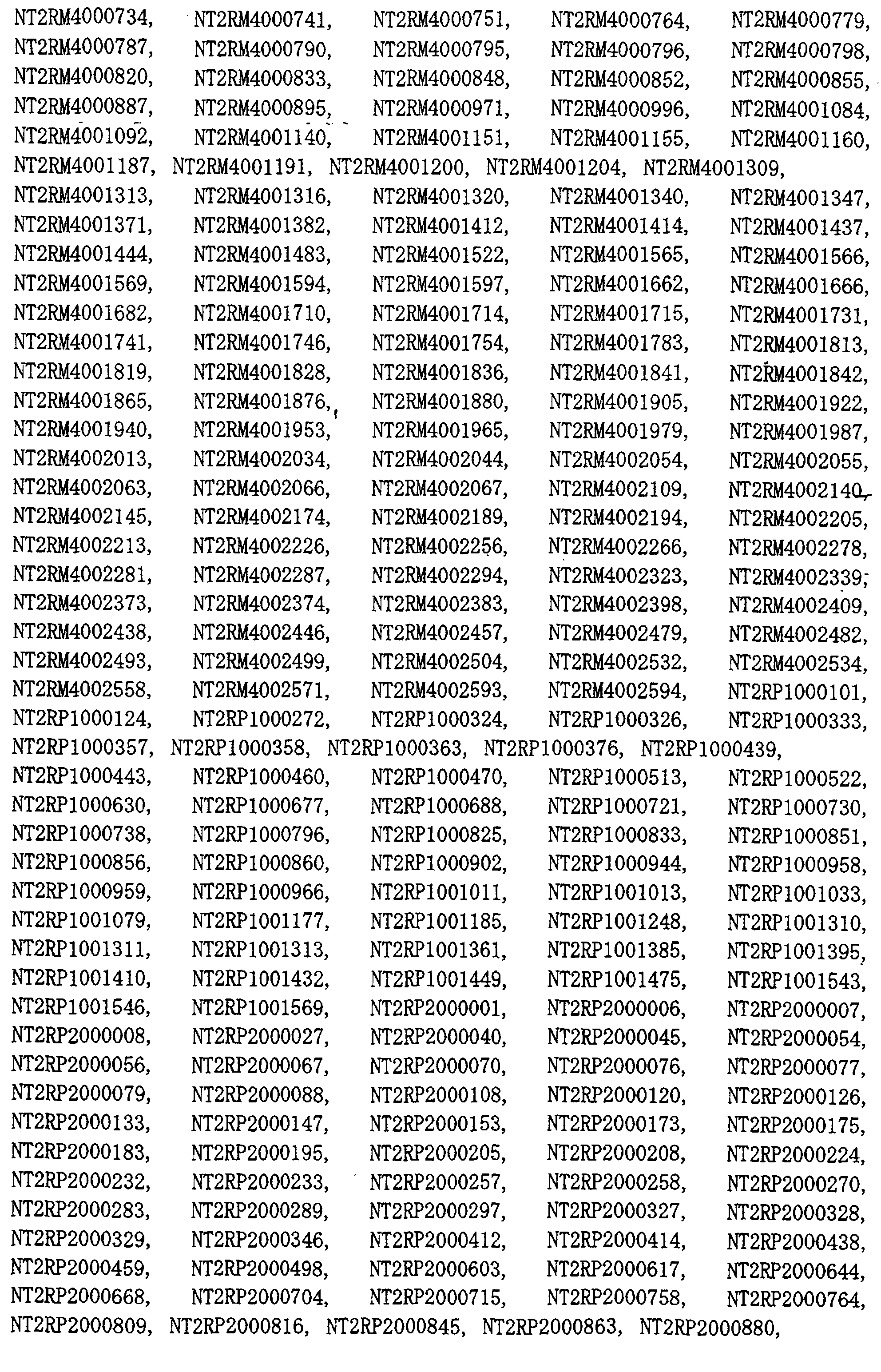

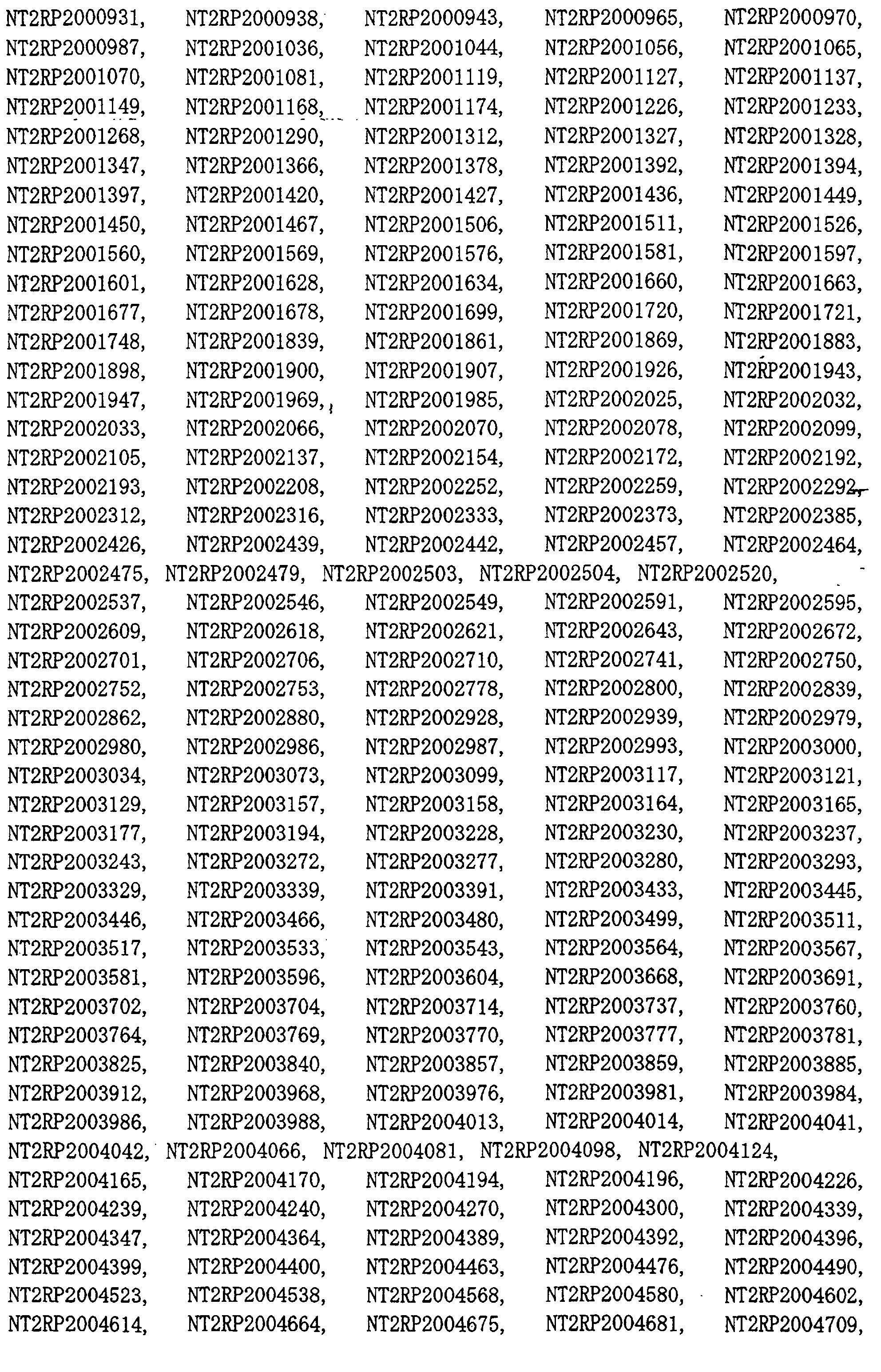

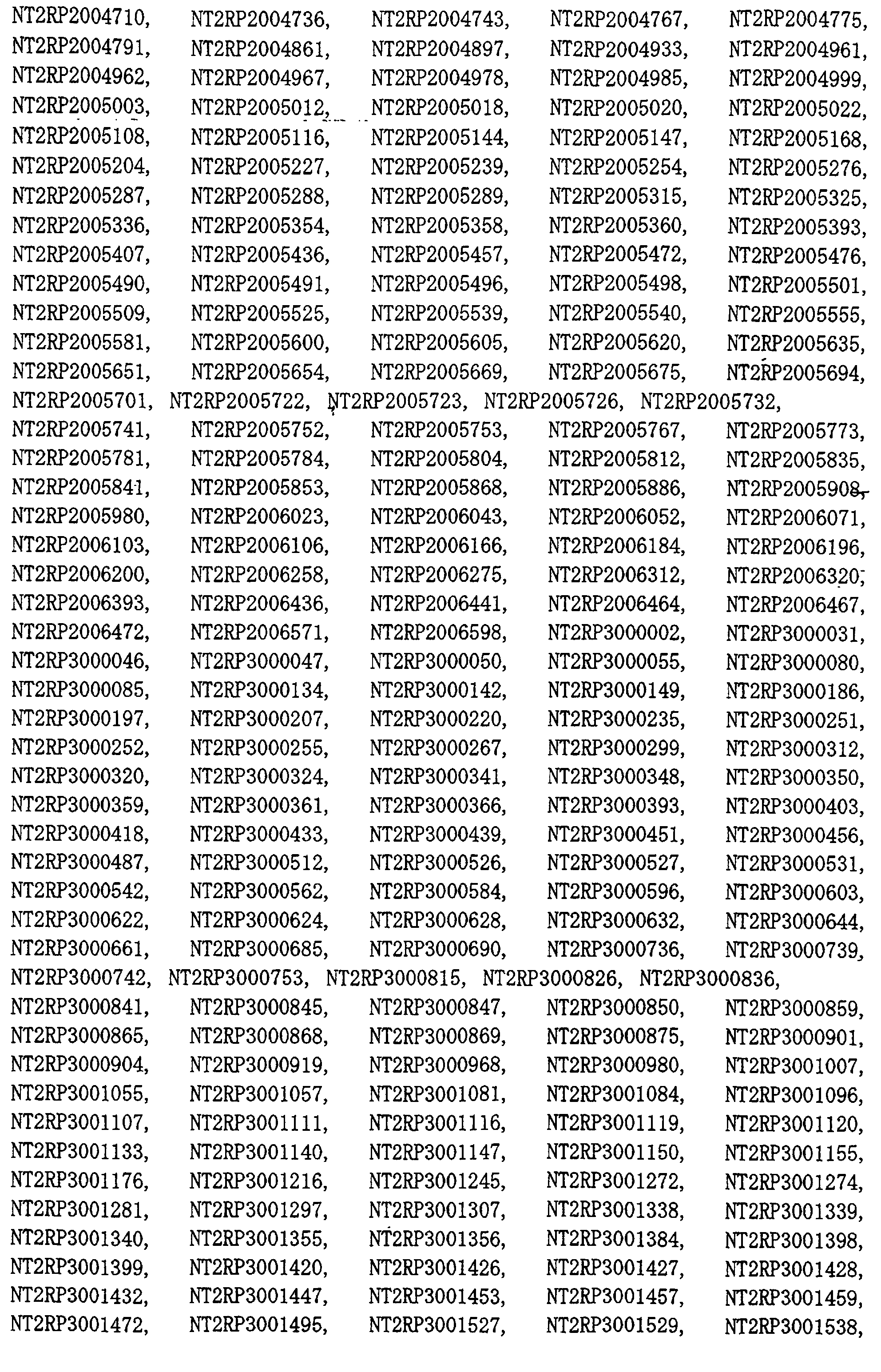

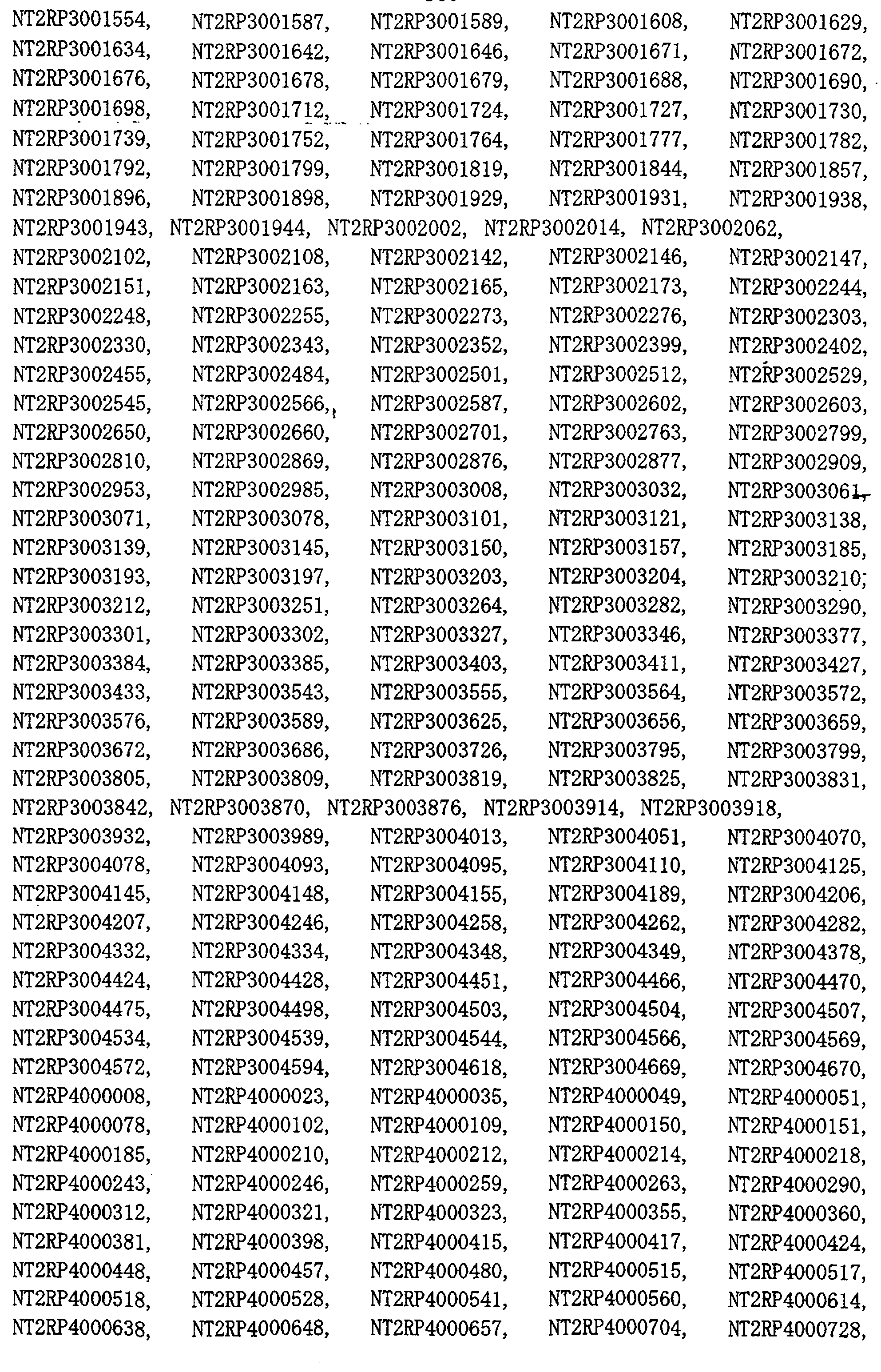

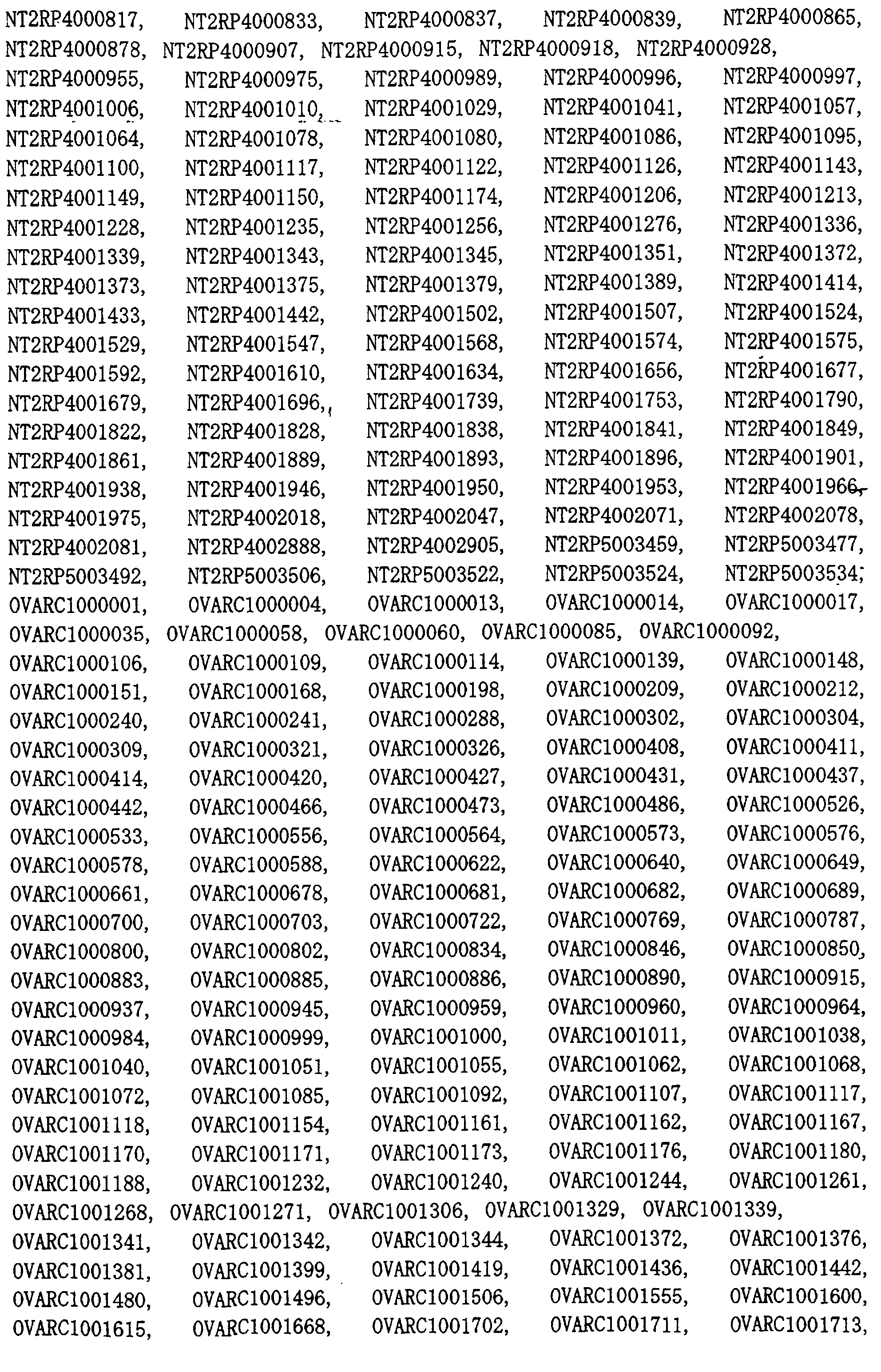

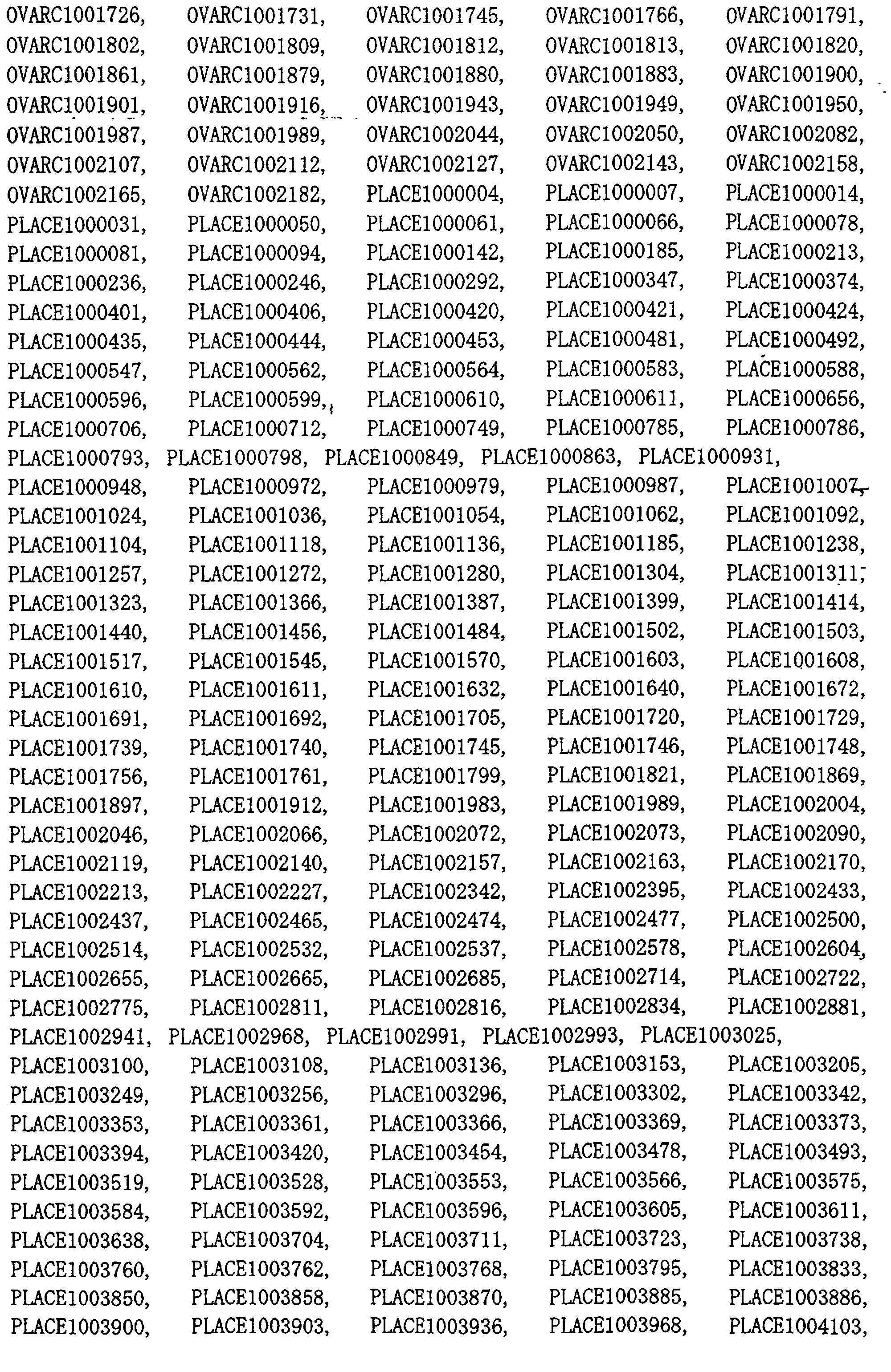

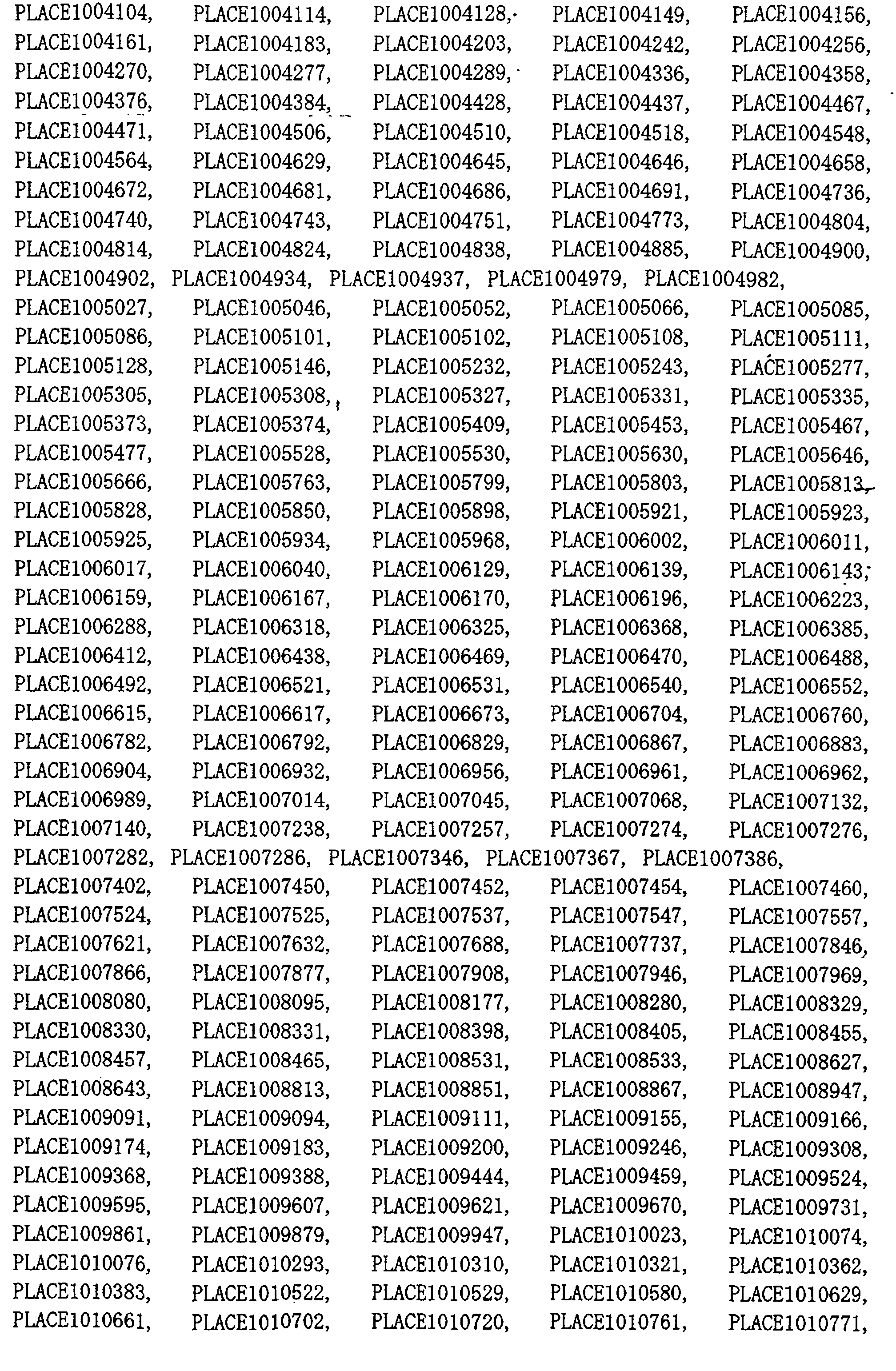

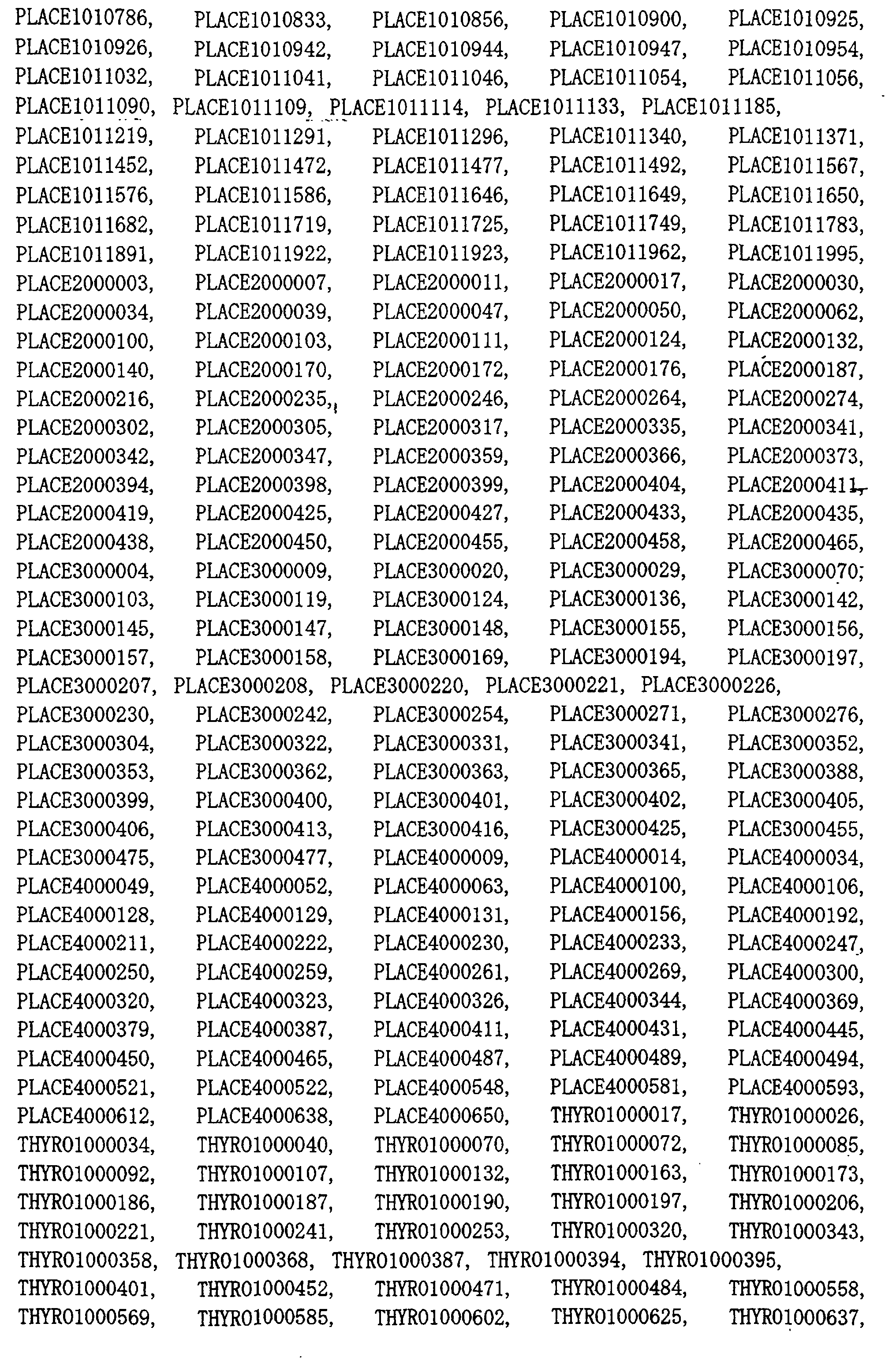

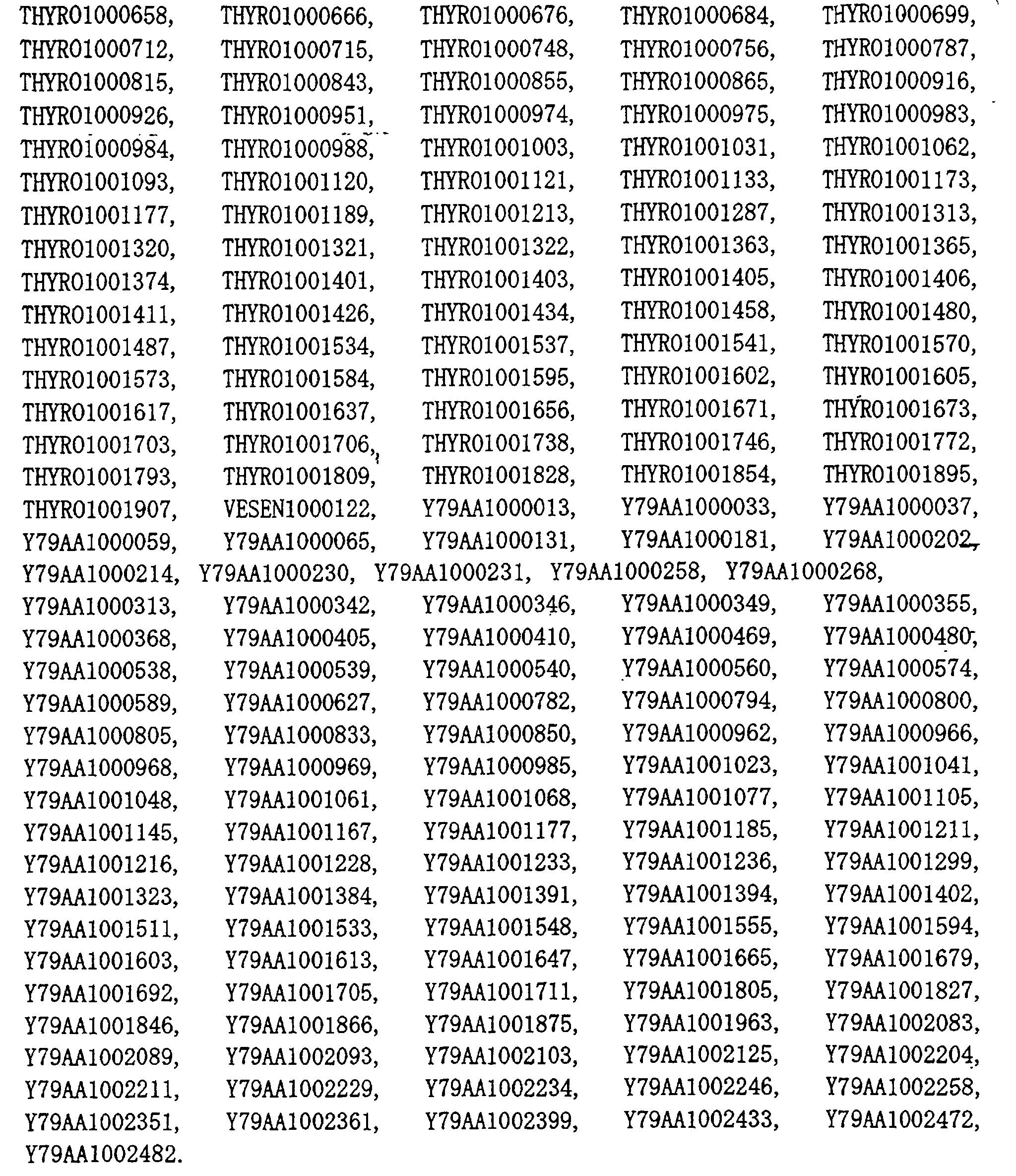

Genes that were expressed in all the tissues tested are indicated below by the corresponding clone names:

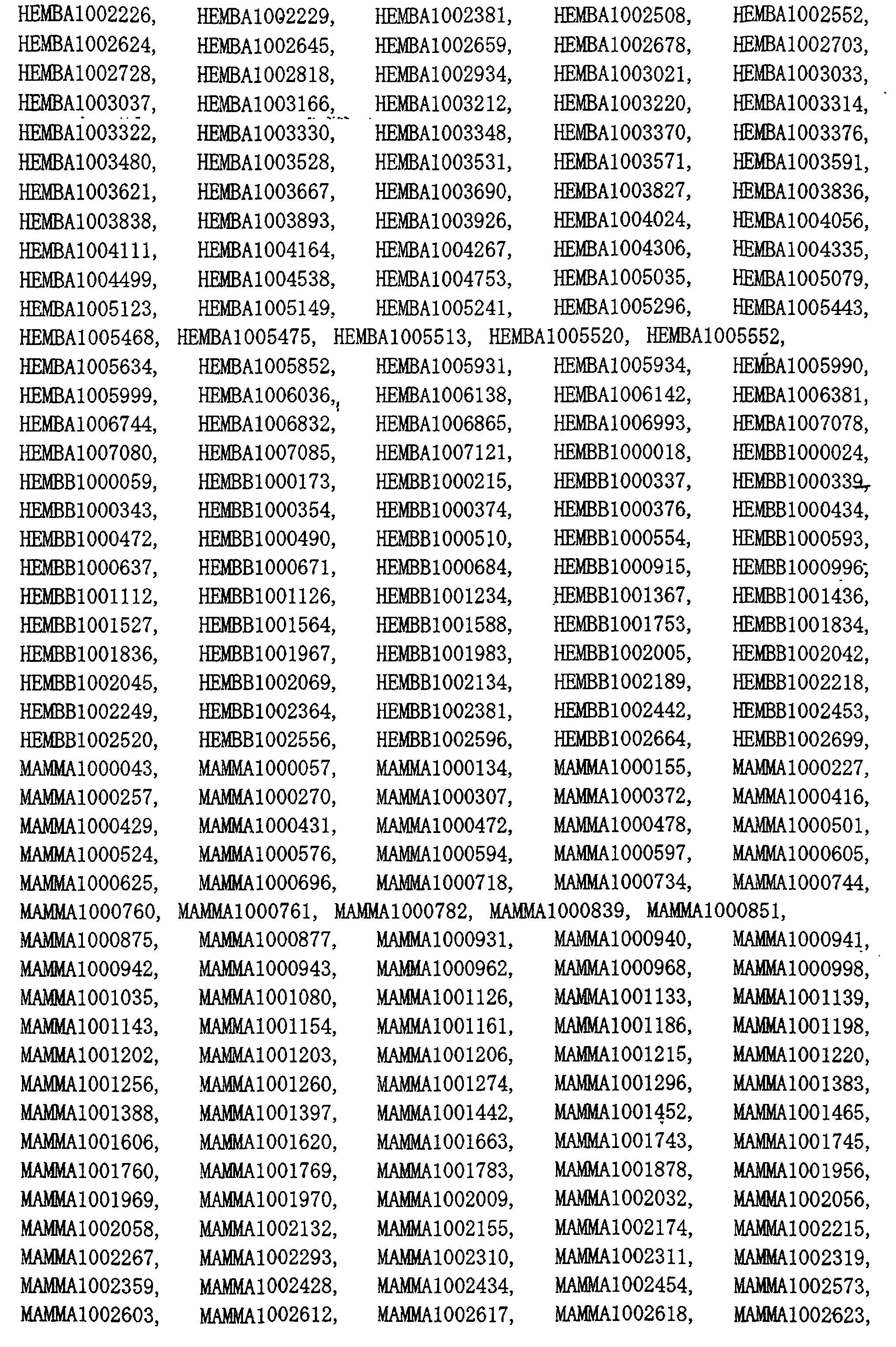

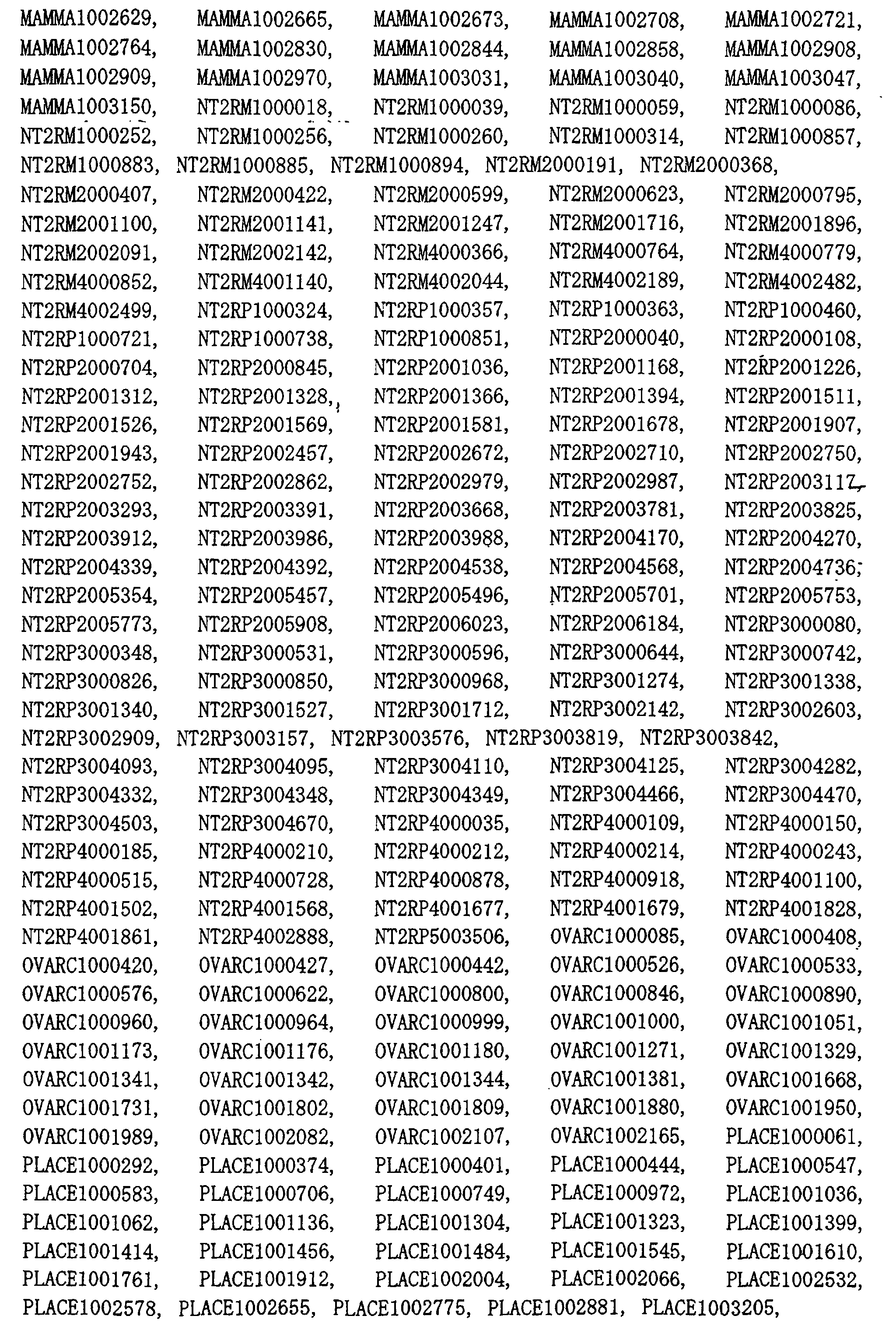

HEMBA1000020, HEMBA1000150, HEMBA1000282, HEMBA1000376, HEMBA1000387,
HEMBA1000508, HEMBA1000519, HEMBA1000575, HEMBA1000655, HEMBA1000682,
HEMBA1000702, HEMBA1000726, HEMBA1000774, HEMBA1000960, HEMBA1000986,
HEMBA1001051, HEMBA1001085, HEMBA1001109, HEMBA1001197, HEMBA1001226,
HEMBA1001286, HEMBA1001299, HEMBA1001377, HEMBA1001435, HEMBA1001476,
HEMBA1001510, HEMBA1001557, HEMBA1001570, HEMBA1001581, HEMBA1001651,
HEMBA1001678, HEMBA1001791, HEMBA1001824, HEMBA1001991, HEMBA1002100,
HEMBA1002102, HEMBA1002113, HEMBA1002150, HEMBA1002160, HEMBA1002166,

PLACE1003256, PLACE1003361, PLACE1003493, PLACE1003519, PLACE1003528,
PLACE1003566, PLACE1003592, PLACE1003870, PLACE1003968, PLACE1004103,
PLACE1004149, PLACE1004156, PLACE1004161, PLACE1004242, PLACE1004336,
PLACE1004358, PLACE1004672, PLACE1004736, PLACE1004824, PLACE1004900,
PLACE1004979, PLACE1005086, PLACE1005101, PLACE1005102, PLACE1005128,
PLACE1005232, PLACE1005528, PLACE1006002, PLACE1006139, PLACE1006412,
PLACE1006540, PLACE1007132, PLACE1007367, PLACE1008405, PLACE1008457,
PLACE1010310, PLACE1011056, PLACE1011340, PLACE1011646, PLACE1011783,
PLACE2000003, PLACE2000030, PLACE2000039, PLACE2000047, PLACE2000124,
PLACE2000235, PLACE2000305, PLACE2000335, PLACE2000347, PLACE2000411,
PLACE2000419, PLACE2000435, PLACE2000450, PLACE2000465, PLACE3000004,
PLACE3000009, PLACE3000070, PLACE3000124, PLACE3000136, PLACE3000145,
PLACE3000155, PLACE3000158, PLACE3000207, PLACE3000254, PLACE3000271,
PLACE3000304, PLACE3000331, PLACE3000399, PLACE3000401, PLACE3000455,
PLACE3000475, PLACE4000009, PLACE4000049, PLACE4000128, PLACE4000131,
PLACE4000192, PLACE4000211, PLACE4000250, PLACE4000323, PLACE4000445,
PLACE4000450, PLACE4000465, PLACE4000612, THYRO1000085, THYRO1000132,
THYRO1000186, THYRO1000484, THYRO1000569, THYRO1000699, THYRO1000712,
THYRO1000815, THYRO1001173, THYRO1001189, THYRO1001401, THYRO1001406,
THYRO1001411, THYRO1001426, THYRO1001480, THYRO1001487, THYRO1001537,
THYRO1001637, THYRO1001772, THYRO1001793, THYRO1001828, THYRO1001854,
Y79AA1000059, Y79AA1000131, Y79AA1000202, Y79AA1000214, Y79AA1000231,
Y79AA1000313, Y79AA1000342, Y79AA1000349, Y79AA1000410, Y79AA1000539,
Y79AA1000560, Y79AA1000589, Y79AA1000833, Y79AA1000985, Y79AA1001077,
Y79AA1001145, Y79AA1001216, Y79AA1001228, Y79AA1001299, Y79AA1001402,
Y79AA1001548, Y79AA1001603, Y79AA1001613, Y79AA1001805, Y79AA1002472,
Y79AA1002482.

Genes that were expressed at low levels in any of the tissues tested are indicated below by the corresponding clone names:

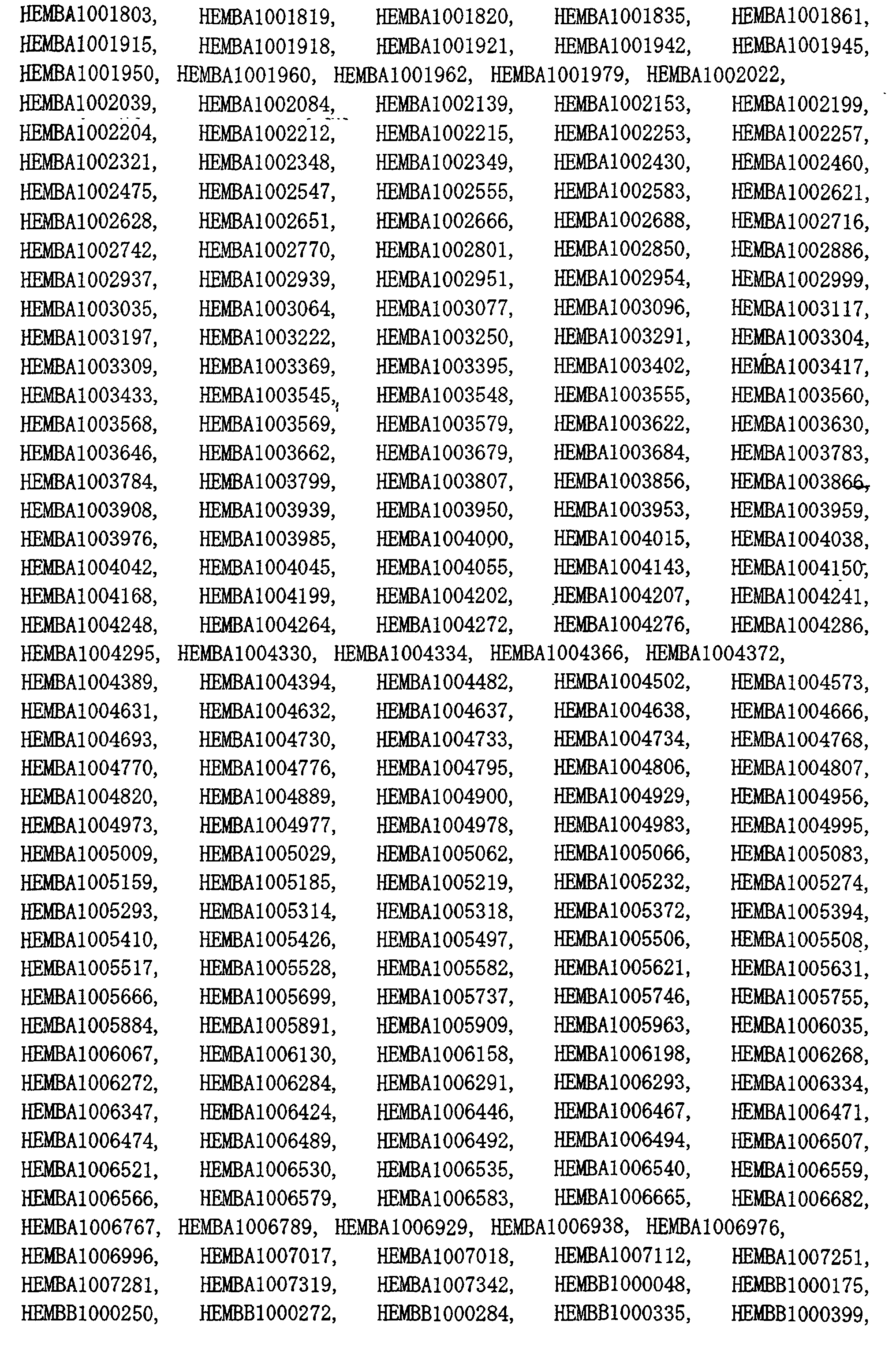

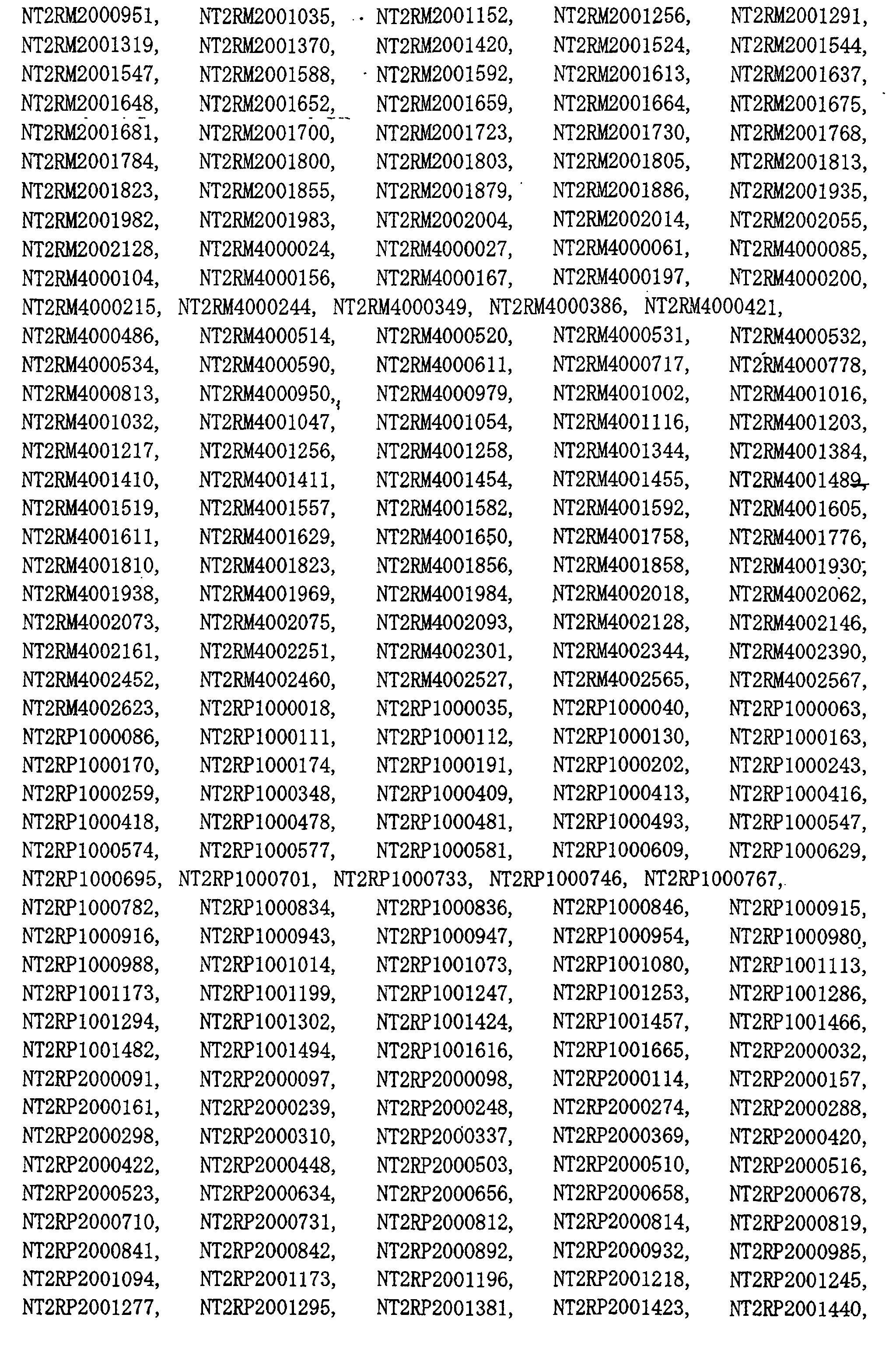

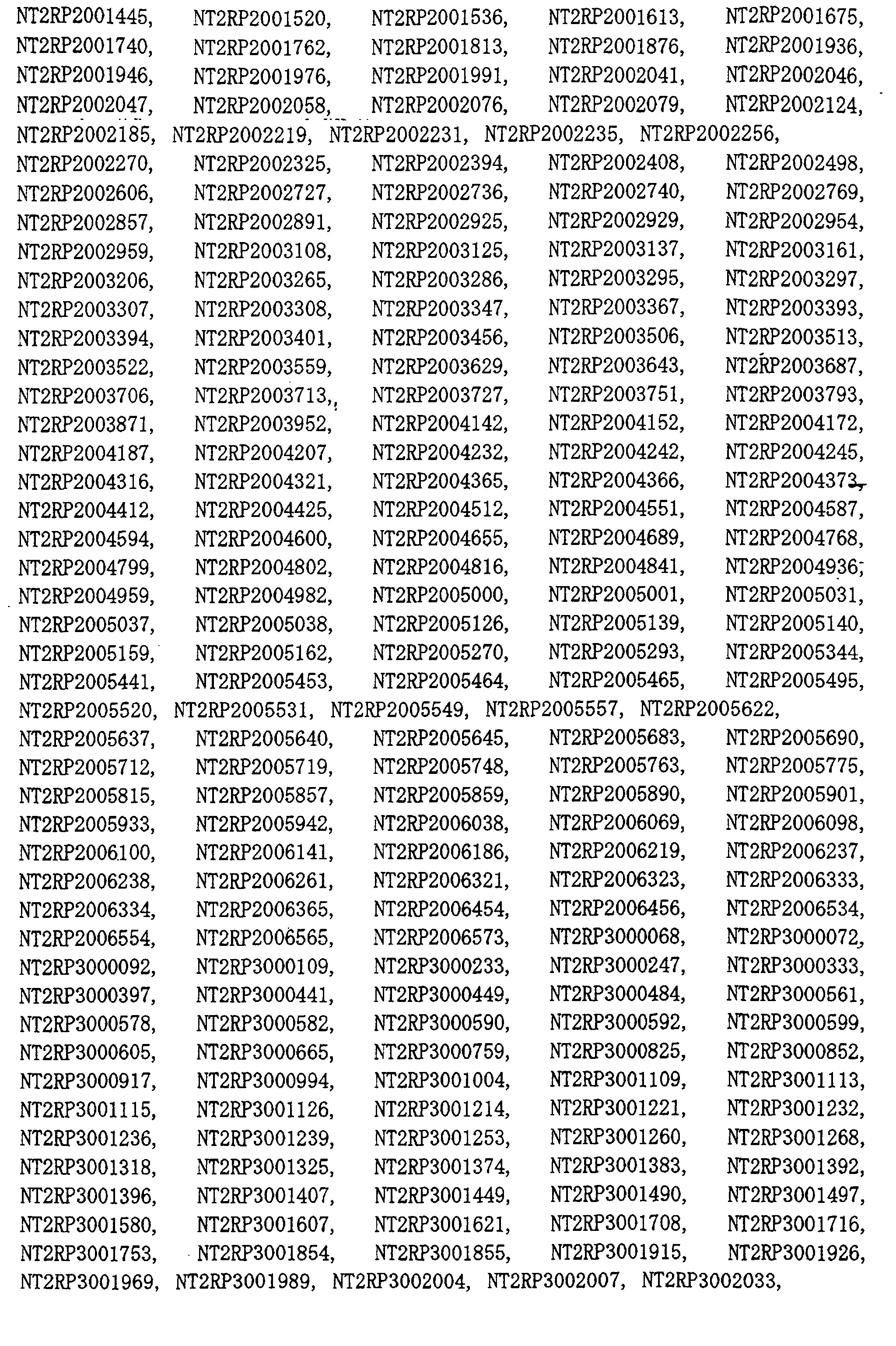

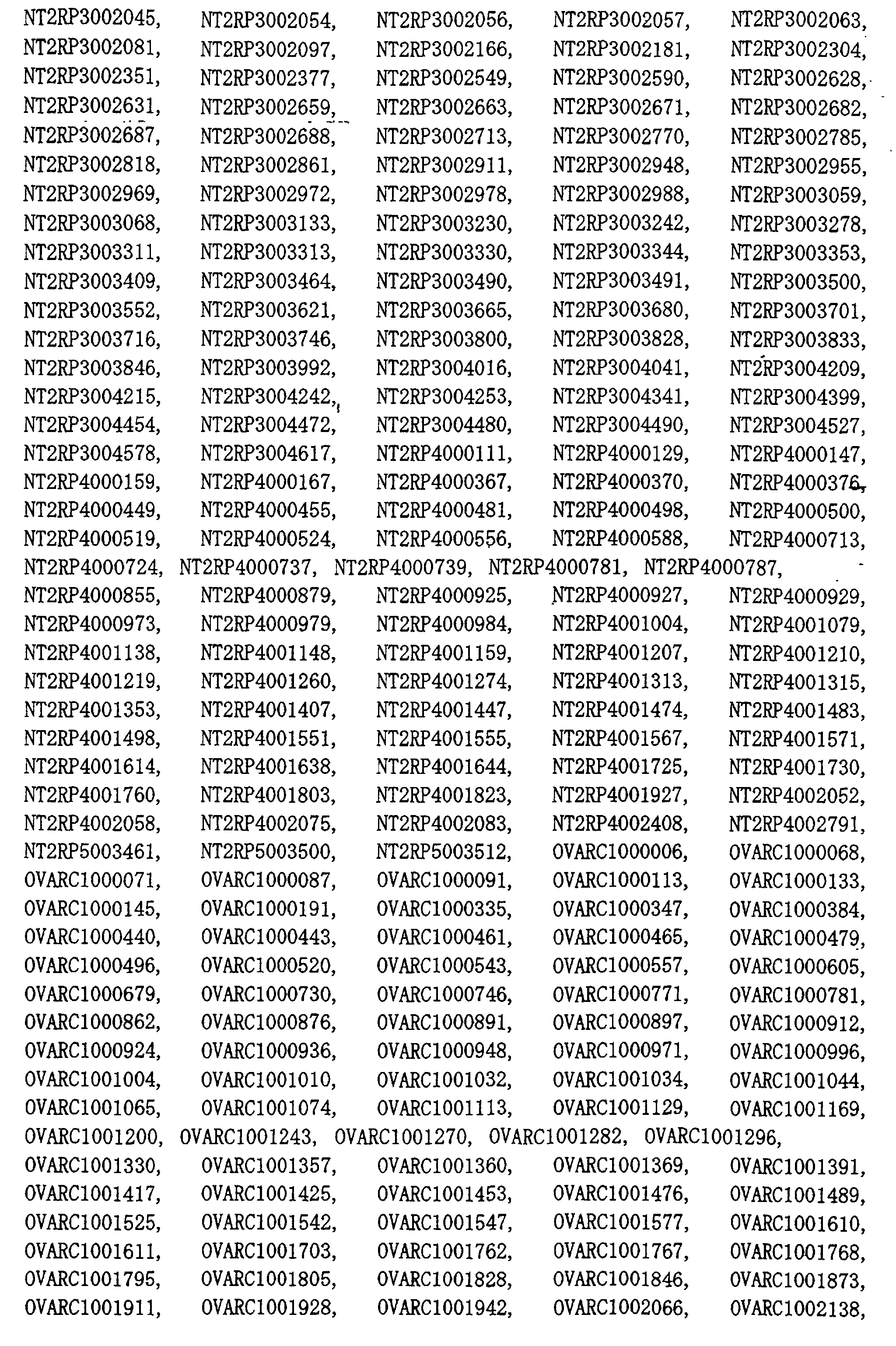

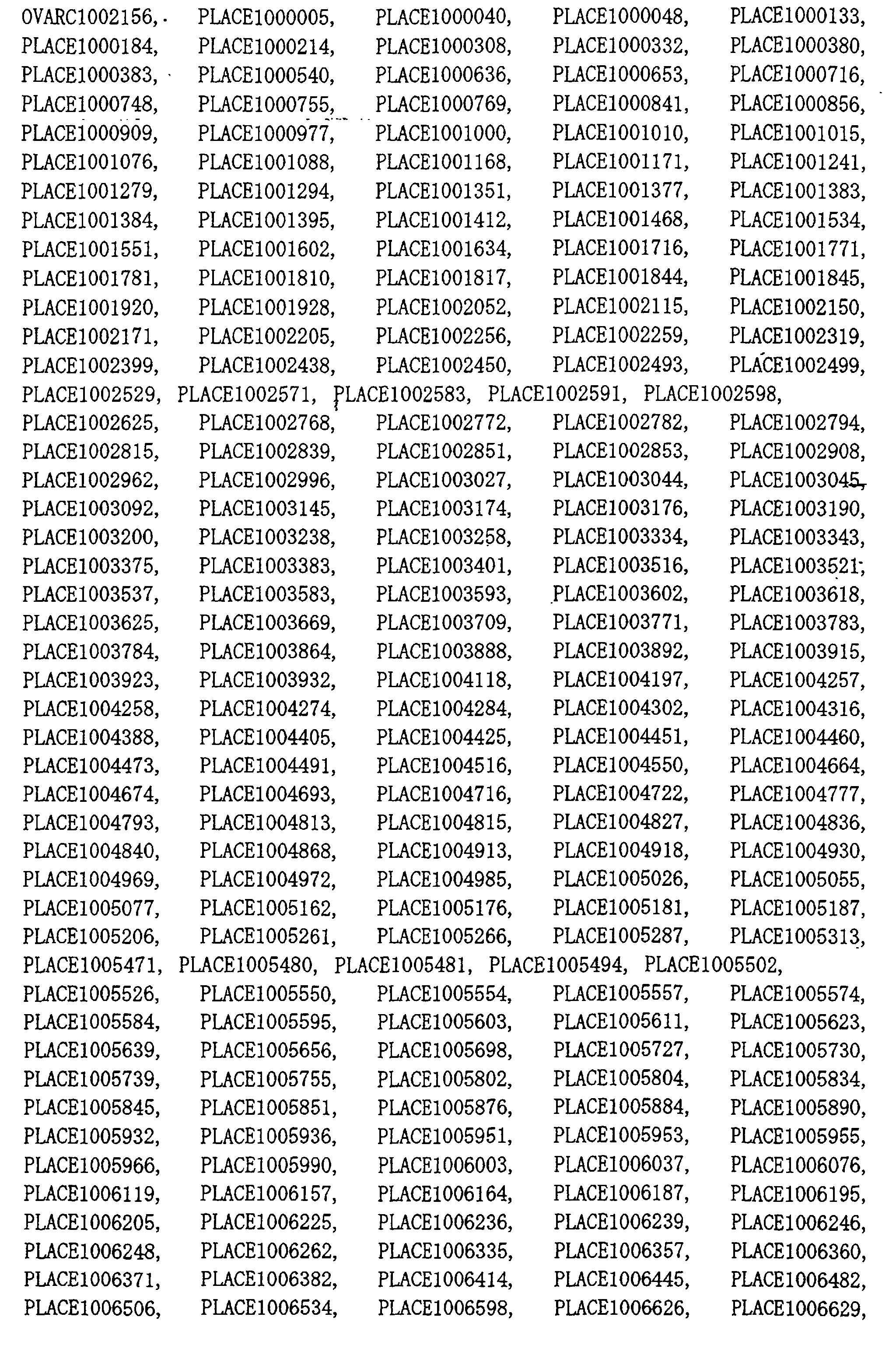

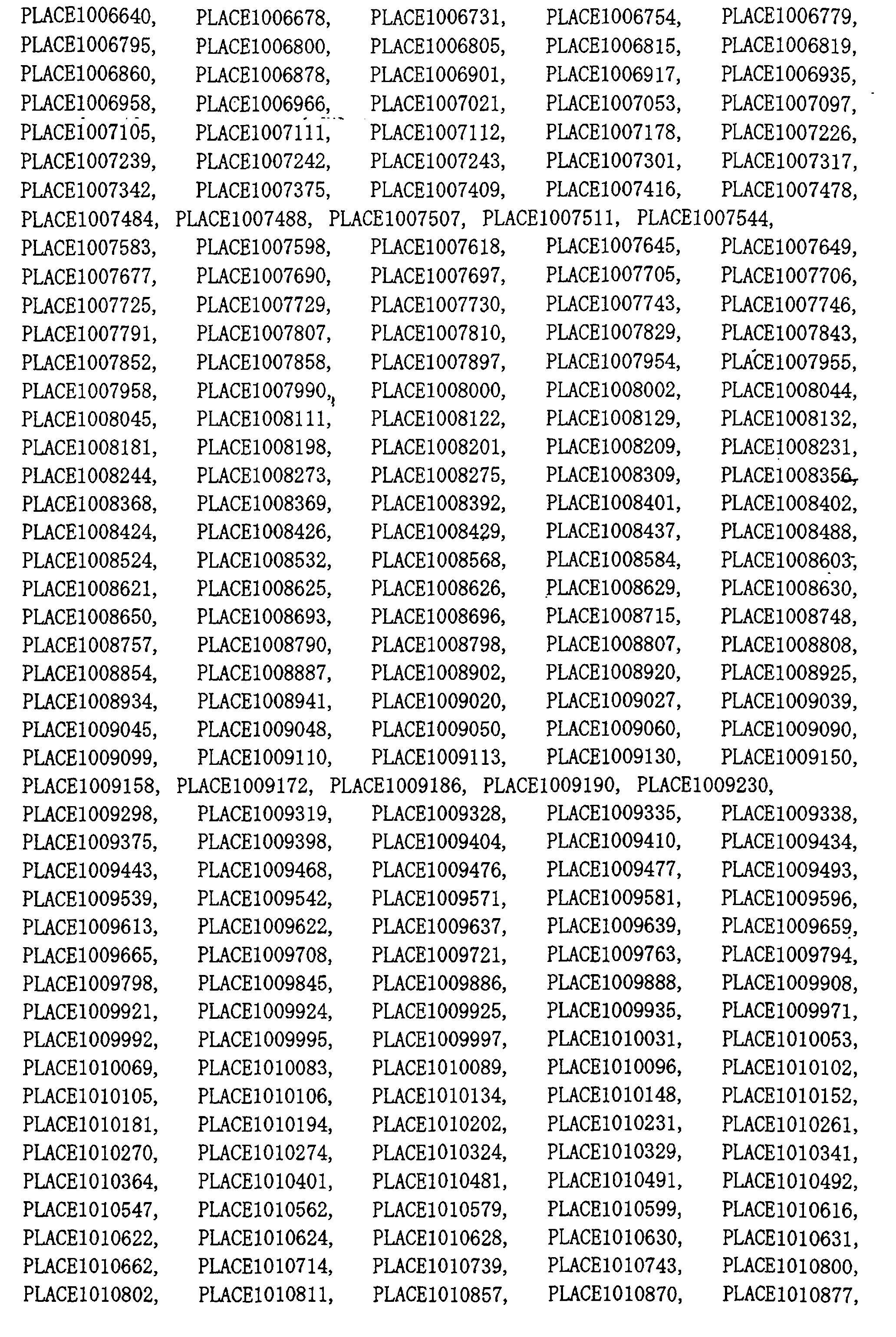

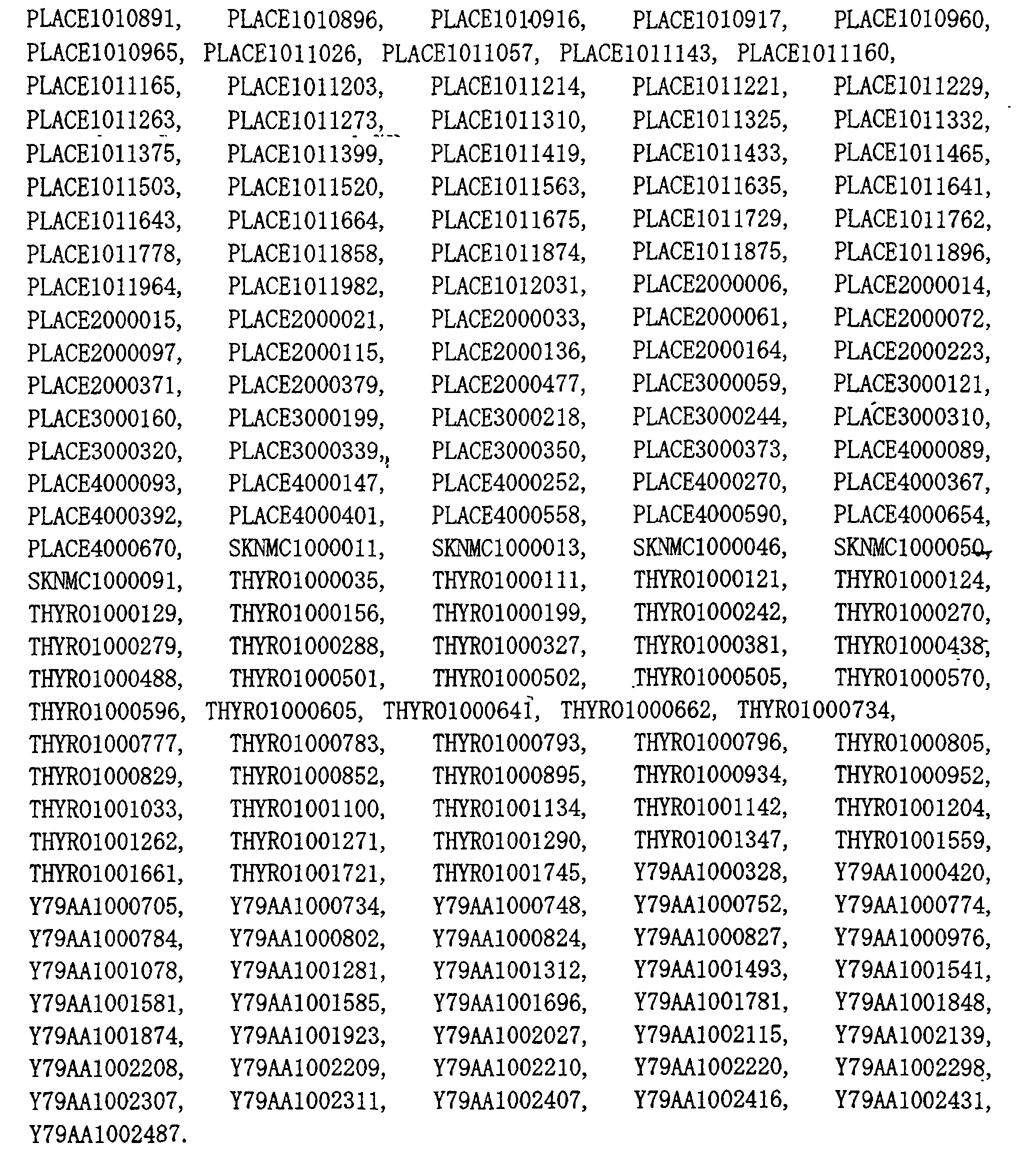

HEMBA1000005, HEMBA1000050, HEMBA1000141, HEMBA1000180, HEMBA1000193,
HEMBA1000201, HEMBA1000213, HEMBA1000251, HEMBA1000264, HEMBA1000290,
HEMBA1000302, HEMBA1000307, HEMBA1000333, HEMBA1000390, HEMBA1000418,
HEMBA1000434, HEMBA1000442, HEMBA1000460, HEMBA1000464, HEMBA1000490,
HEMBA1000504, HEMBA1000518, HEMBA1000520, HEMBA1000531, HEMBA1000555,
HEMBA1000563, HEMBA1000592, HEMBA1000594, HEMBA1000608, HEMBA1000637,
HEMBA1000662, HEMBA1000686, HEMBA1000719, HEMBA1000722, HEMBA1000727,
HEMBA1000747, HEMBA1000773, HEMBA1000817, HEMBA1000822, HEMBA1000851,
HEMBA1000867, HEMBA1000869, HEMBA1000943, HEMBA1000946, HEMBA1000968,
HEMBA1000974, HEMBA1000985, HEMBA1001009, HEMBA1001019, HEMBA1001022,
HEMBA1001024, HEMBA1001026, HEMBA1001043, HEMBA1001052, HEMBA1001071,
HEMBA1001094, HEMBA1001099, HEMBA1001122, HEMBA1001174, HEMBA1001247,
HEMBA1001257, HEMBA1001319, HEMBA1001323, HEMBA1001327, HEMBA1001361,
HEMBA1001383, HEMBA1001391, HEMBA1001407, HEMBA1001411, HEMBA1001442,
HEMBA1001455, HEMBA1001478, HEMBA1001522, HEMBA1001585, HEMBA1001635,
HEMBA1001658, HEMBA1001672, HEMBA1001675, HEMBA1001681, HEMBA1001702,
HEMBA1001711, HEMBA1001723, HEMBA1001744, HEMBA1001746, HEMBA1001800,

HEMBB1000404, HEMBB1000449, HEMBB1000487, HEMBB1000518, HEMBB1000592,
HEMBB1000665, HEMBB1000673, HEMBB1000725, HEMBB1000774, HEMBB1000789,
HEMBB1000794, HEMBB1000822, HEMBB1000831, HEMBB1000852, HEMBB1000876,
HEMBB1000883, HEMBB1000888, HEMBB1000973, HEMBB1000981, HEMBB1000985,
HEMBB1001004, HEMBB1001008, HEMBB1001011, HEMBB1001051, HEMBB1001117,
HEMBB1001199, HEMBB1001221, HEMBB1001242, HEMBB1001304, HEMBB1001314,
HEMBB1001315, HEMBB1001326, HEMBB1001331, HEMBB1001335, HEMBB1001339,
HEMBB1001356, HEMBB1001364, HEMBB1001384, HEMBB1001387, HEMBB1001394,
HEMBB1001410, HEMBB1001424, HEMBB1001443, HEMBB1001464, HEMBB1001482,
HEMBB1001603, HEMBB1001630, HEMBB1001641, HEMBB1001665, HEMBB1001668,
HEMBB1001684, HEMBB1001685, HEMBB1001695, HEMBB1001736, HEMBB1001747,
HEMBB1001760, HEMBB1001762, HEMBB1001785, HEMBB1001797, HEMBB1001839,
HEMBB1001867, HEMBB1001868, HEMBB1001872, HEMBB1001874, HEMBB1001875,
HEMBB1001899, HEMBB1001905, HEMBB1001930, HEMBB1002002, HEMBB1002009,
HEMBB1002044, HEMBB1002255, HEMBB1002266, HEMBB1002340, HEMBB1002458,
HEMBB1002492, HEMBB1002502, HEMBB1002509, HEMBB1002510, HEMBB1002522,
HEMBB1002550, HEMBB1002600, HEMBB1002677, HEMBB1002686, HEMBB1002702,
HEMBB1002705, MAMMA1000175, MAMMA1000221, MAMMA1000309, MAMMA1000424,
MAMMA1000458, MAMMA1000468, MAMMA1000490, MAMMA1000616, MAMMA1000621,
MAMMA1000843, MAMMA1000845, MAMMA1000855, MAMMA1000865, MAMMA1001030;
MAMMA1001075, MAMMA1001091, MAMMA1001110, MAMMA1001222, MAMMA1001322,
MAMMA1001476, MAMMA1001510, MAMMA1001627, MAMMA1001649, MAMMA1001730,
MAMMA1001744, MAMMA1001757, MAMMA1001764, MAMMA1001788, MAMMA1001812,
MAMMA1001817, MAMMA1001963, MAMMA1002011, MAMMA1002041, MAMMA1002093,
MAMMA1002118, MAMMA1002156, MAMMA1002170, MAMMA1002268, MAMMA1002269,
MAMMA1002385, MAMMA1002566, MAMMA1002619, MAMMA1002650, MAMMA1002685,
MAMMA1002699, MAMMA1002727, MAMMA1002746, MAMMA1002758, MAMMA1002769,
MAMMA1002820, MAMMA1002835, MAMMA1002871, MAMMA1002938, MAMMA1002982,
MAMMA1003019, MAMMA1003026, MAMMA1003049, MAMMA1003056, MAMMA1003104,
MAMMA1003135, MAMMA1003140, MAMMA1003146, MAMMA1003166, NT2RM1000001,
NT2RM1000032, NT2RM1000055, NT2RM1000062, NT2RM1000080, NT2RM1000092,
NT2RM1000118, NT2RM1000119, NT2RM1000127, NT2RM1000131, NT2RM1000132,
NT2RM1000153, NT2RM1000186, NT2RM1000187, NT2RM1000199, NT2RM1000242,
NT2RM1000244, NT2RM1000257, NT2RM1000271, NT2RM1000280, NT2RM1000318,
NT2RM1000354, NT2RM1000365, NT2RM1000388, NT2RM1000394, NT2RM1000399,
NT2RM1000421, NT2RM1000430, NT2RM1000539, NT2RM1000563, NT2RM1000623,
NT2RM1000648, NT2RM1000661, NT2RM1000666, NT2RM1000669, NT2RM1000691,
NT2RM1000699, NT2RM1000702, NT2RM1000741, NT2RM1000742, NT2RM1000746,
NT2RM1000770, NT2RM1000772, NT2RM1000780, NT2RM1000781, NT2RM1000800,
NT2RM1000811, NT2RM1000833, NT2RM1000850, NT2RM1000852, NT2RM1000898,
NT2RM1000924, NT2RM1000978, NT2RM1001003, NT2RM1001008, NT2RM1001043,
NT2RM1001059, NT2RM1001066, NT2RM1001072, NT2RM1001074, NT2RM1001085,
NT2RM1001102, NT2RM1001105, NT2RM1001112, NT2RM2000042, NT2RM2000092,
NT2RM2000124, NT2RM2000192, NT2RM2000322, NT2RM2000395, NT2RM2000469,
NT2RM2000522, NT2RM2000540, NT2RM2000556, NT2RM2000566, NT2RM2000594,
NT2RM2000609, NT2RM2000639, NT2RM2000669, NT2RM2000691, NT2RM2000718,

Genes exhibiting characteristic features in the expression thereof were selected by statistical analysis of these data. Two examples are shown below to describe the selection of genes of which expression is varied greatly among tissues. The β' actin gene is used frequently as a control in gene expression analysis. Genes of which expression is varied greatly among tissues as compared that of the β-actin gene were determined as follows. Specifically, sum of squared deviation was calculated in the signal intensity of β-actin observed in each tissue, which was divided by 7 degrees of freedom to determine a variance Sₐ². Next, sum of squared deviation was calculated in the signal intensity of a gene to be compared observed in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names:
HEMBA1002113, HEMBA1005296, HEMBA1007121, HEMBB1000637, HEMBB1000915, MAMMA1000597, MAMMA1000605, MAMMA1000962, MAMMA1001139, MAMMA1001198, MAMMA1002858, NT2RM2001896, NT2RP2002710, NT2RP2004339, NT2RP2004538, NT2RP3000348, NT2RP3003121, PLACE3000009, PLACE3000254, THYRO1000569, Y79AA1000131.

Gene of OVARC1000037 {heterogeneous nuclear ribonucleoprotein (hnRNP)} which expression is varied little. Genes of which expression is varied greatly among tissues as compared that of the OVARC1000037 gene were determined as follows. Specifically, sum of squared deviation was calculated in the signal intensity of β-actin observed in each tissue, which was divided by 7 degrees of freedom to determine a variance Sₐ². Next, sum of squared deviation was calculated in the signal intensity of a gene to be compared observed in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below,by the corresponding clone names:

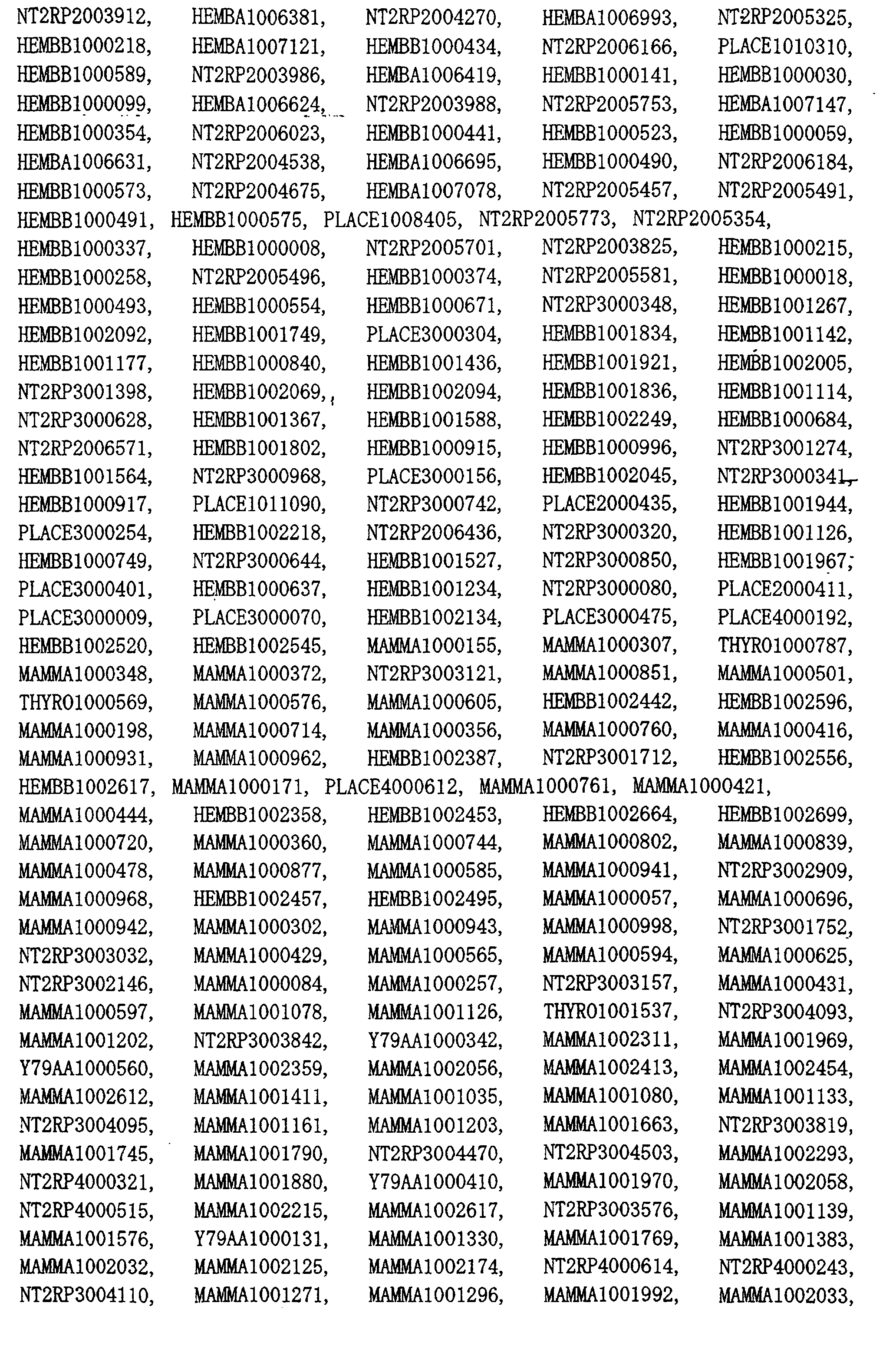

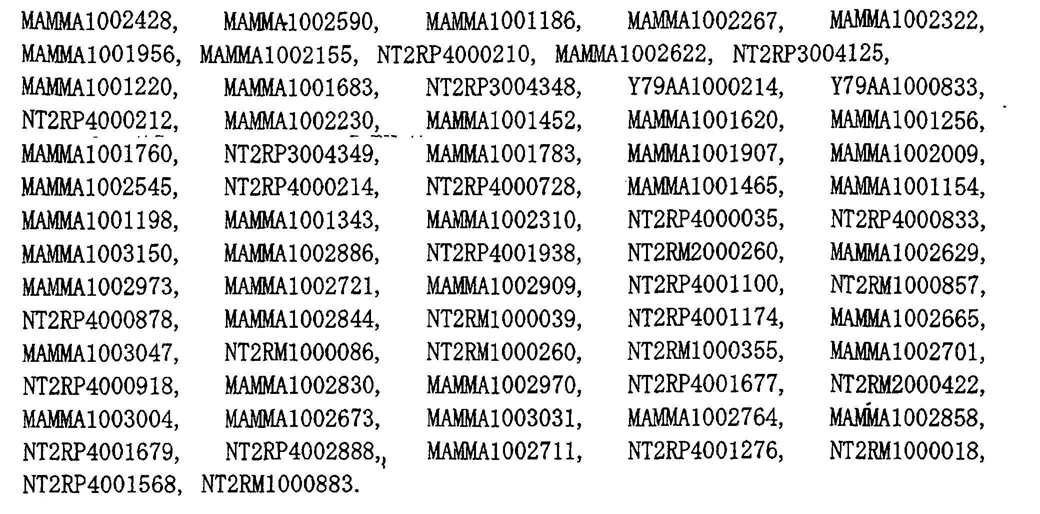

HEMBA1000304, NT2RM2001716, NT2RM2001840, HEMBA1001051, HEMBA1001109,
OVARC1001731, HEMBA1000726, HEMBA1001286, HEMBA1000387, HEMBA1000519,
NT2RM2001896, HEMBA1000042, HEMBA1001085, HEMBA1001330, OVARC1000576,
HEMBA1000575, NT2RM2000599, NT2RM2000714, HEMBA1000469, NT2RM4000366,
HEMBA1001377, HEMBA1000769, HEMBA1000338, NT2RM2000795, HEMBA1001299,
HEMBA1000508, HEMBA1000150, HEMBA1000774, HEMBA1001226, HEMBA1000960,
NT2RM4000795, HEMBA1002162, NT2RM4001876, NT2RM4002482, HEMBA1001678,
HEMBA1002113, NT2RM4002383, HEMBA1002229, HEMBA1002818, HEMBA1001454,
NT2RM4000764, HEMBA1001510, HEMBA1001714, HEMBA1002150, NT2RM4002044,
HEMBA1002728, NT2RM4002189, HEMBA1001991, HEMBA1002166, NT2RM4002499,
NT2RM4001140, NT2RM4002504, HEMBA1002590, HEMBA1001435, PLACE1000706,
HEMBA1002160, HEMBA1001824, HEMBA1001463, HEMBA1001533, HEMBA1001570,
PLACE1001036, HEMBA1001651, HEMBA1002381, HEMBA1002934, HEMBA1003370,
HEMBA1003021, HEMBA1003166, NT2RP1000738, NT2RP2000040, HEMBA1004164,
HEMBA1003836, HEMBA1004267, NT2RP2000845, HEMBA1003041, HEMBA1003571,
HEMBA1003758, NT2RP2000108, HEMBA1003838, NT2RP1000357, HEMBA1003376,
PLACE1003528, HEMBA1003528, NT2RP1001475, HEMBA1004049, HEMBA1003212,
HEMBA1003667, PLACE1004149, HEMBA1003926, HEMBA1004306, HEMBA1004024,
NT2RP1000363, HEMBA1003033, HEMBA1004335, HEMBA1003348, HEMBA1003034,
NT2RP2001081, HEMBA1004056, HEMBA1003314, HEMBA1003827, HEMBA1003893,
NT2RP2001036, NT2RP2001168, NT2RP2001328, HEMBA1005035, NT2RP2001569,
NT2RP2002439, HEMBA1005511, HEMBA1005999, NT2RP2002862, NT2RP2002979,
NT2RP2001394, HEMBA1004753, NT2RP2002621, HEMBA1005853, HEMBA1005443,
NT2RP2002980, NT2RP2001347, HEMBA1005241, NT2RP2002750, NT2RP2003533,
HEMBA1005634, NT2RP2003034, HEMBA1006138, NT2RP2003117, NT2RP2001366,
HEMBA1005079, NT2RP2003293, NT2RP2002710, HEMBA1005911, NT2RP2002752,
HEMBA1006036, NT2RP2002987, HEMBA1006100, HEMBA1004460, HEMBA1004538,
NT2RP2001943, NT2RP2002033, HEMBA1005296, HEMBA1005829, HEMBA1005520,
HEMBA1005123, HEMBA1005552, HEMBA1004930, NT2RP2001312, HEMBA1005304,
HEMBA1005834, HEMBA1005990, HEMBA1005526, NT2RP2003073, HEMBA1005331,
HEMBA1006744, HEMBA1006780, NT2RP2004339, HEMBB1000173, HEMBA1007113,
NT2RP2005908, HEMBB1000376, HEMBB1000024, HEMBB1000510, NT2RP2004580,

Thus, characteristic features in the expression of a gene are illustrated by comparing and _ statistically analyzing the expression of many genes.

### Analysis of disease-associated genes

Non-enzymic protein glycation reaction is believed to be a cause of a variety of chronic diabetic complications. Accordingly, genes of which expression is elevated or decreased in a glycated protein-specific manner in the endothelial cells are associated with diabetic complications caused by glycated proteins. Vascular endothelial cells are affected with glycated proteins present in blood. Reaction products of non-enzymic protein glycation include amadori compound (glycated protein) as a mildly glycated protein and advanced glycation endproduct as a heavily glycated protein. Hence, a survey was carried out for genes of which expression levels are varied depending on the presence of these glycated proteins in endothelial cells. The mRNAs were extracted from endothelial cells that were cultured in the presence or absence of glycated protein. The mRNAs were converted into radiolabeled first strand cDNAs for preparing probes. The probes were hybridized to the above-mentioned DNA array. Signal of each DNA spot was detected by BAS2000 and analyzed by ArrayGauge (Fuji Photo Film Co., Ltd.).

Advanced glycation endproduct of bovine serum albumin was prepared as follows: bovine serum albumin (BSA; Sigma) was incubated in a phosphate buffer solution containing 50 mM glucose at 37°C for 8 weeks; and the resulting brownish BSA was dialyzed against a phosphate buffer solution.

Human normal pulmonary arterial endothelial cells (Cell Applications) were cultured in an Endothelial Cell Growth Medium (Cell Applications). The culture dish (Farcon) with the cells were incubated in a CO₂ incubator (37°C, 5% CO₂, in a humid atmosphere). When the cells were grown to be confluent in the dish, 250 µg/ml of bovine serum albumin (sigma), glycated bovine serum albumin (Sigma) or advanced glycation endproduct of bovine serum albumin was added thereto and the cells were incubated for 33 hours. The mRNA was extracted from the cells by using a FastTack^{(TM)} 2.0 kit (Invitrogen). The labeling of hybridization probe was carried out by using the mRNA according to the same procedure as described above.

Table 169 shows the expression level of each cDNA in human pulmonary arterial endothelial cells cultured in a medium containing bovine serum albumin (sigma), glycated bovine serum albumin (Sigma) or advanced glycation endproduct of bovine serum albumin. Genes of which expression was detected in the endothelial cell are as follows:

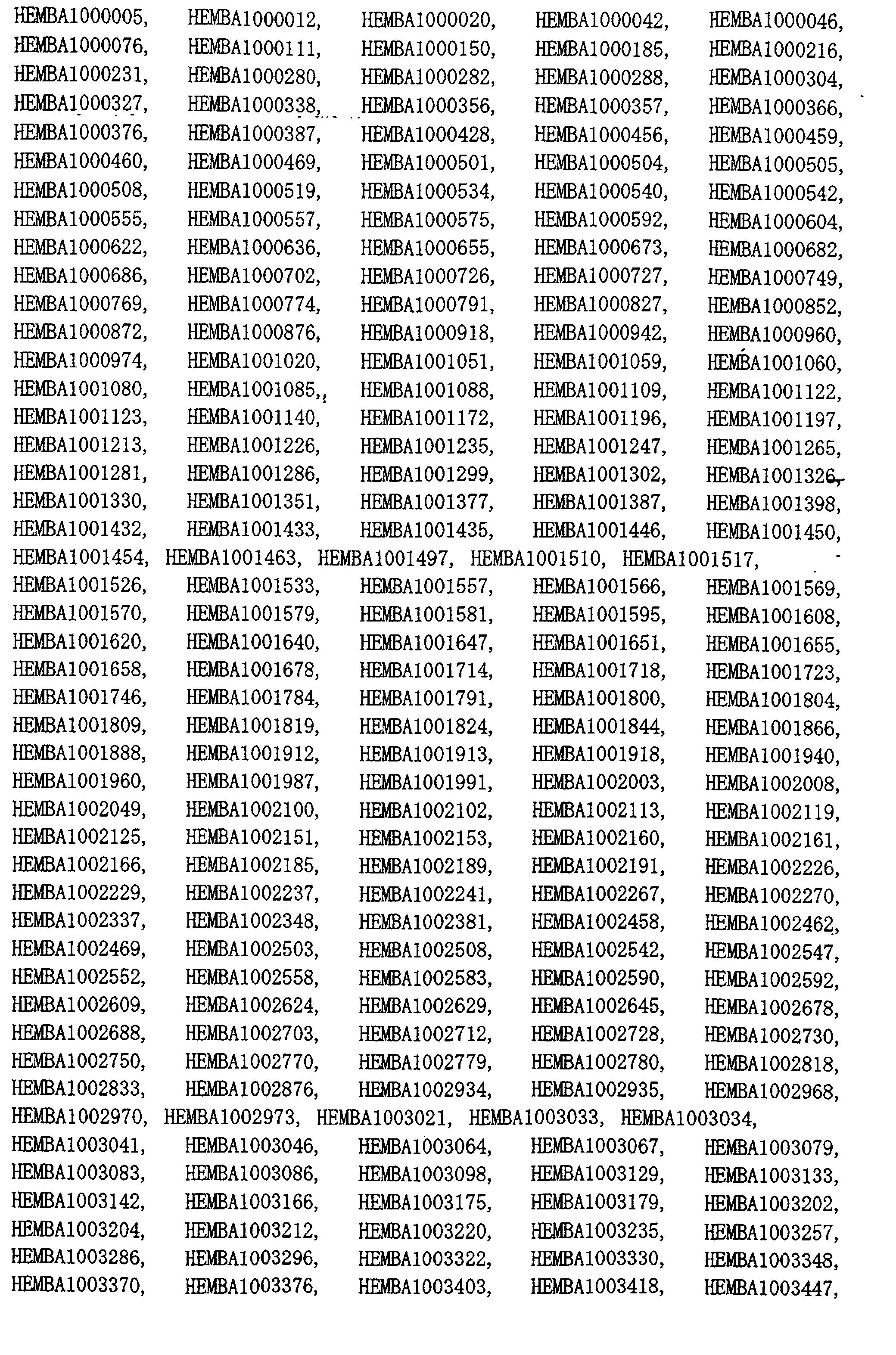

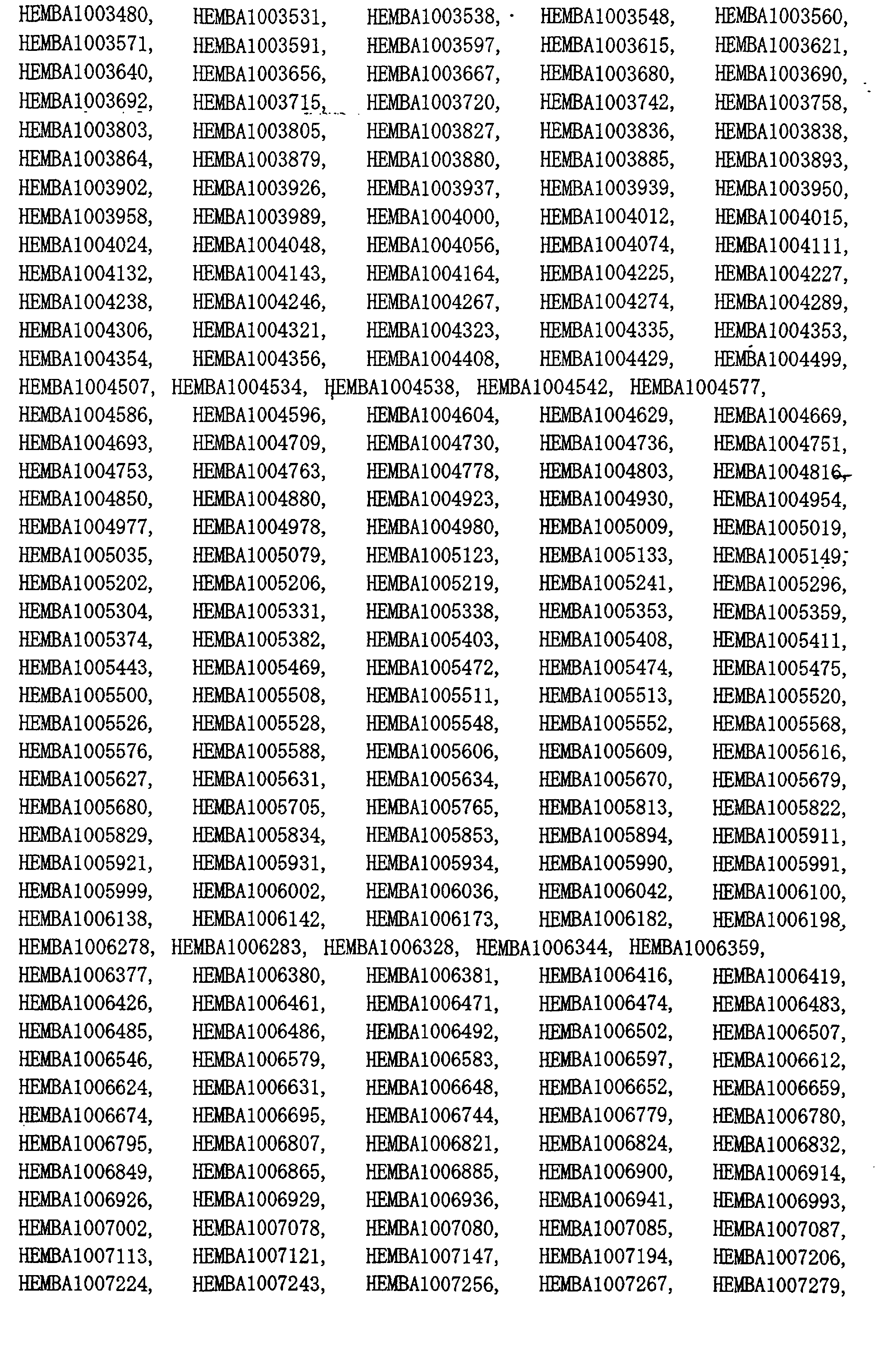

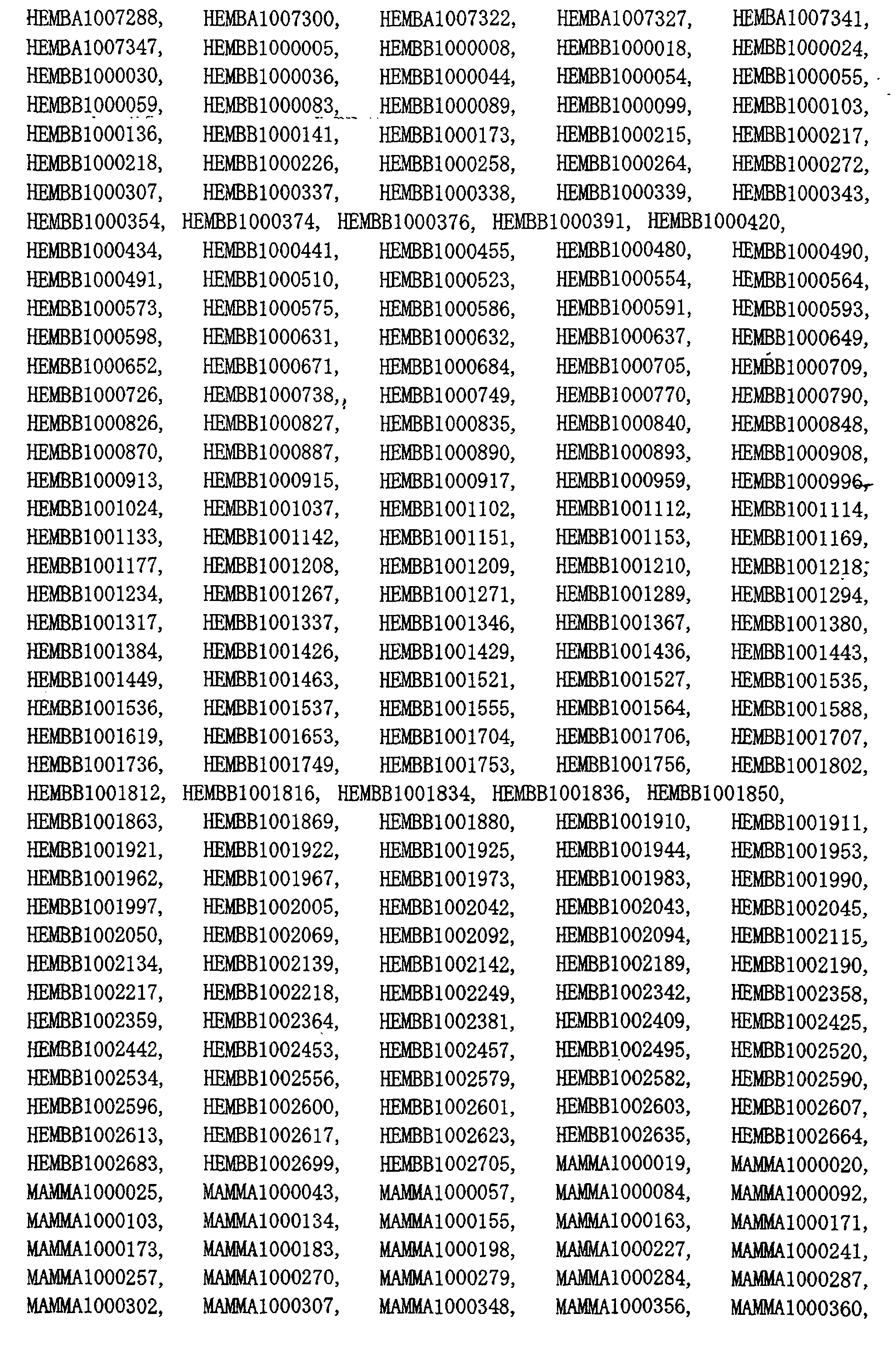

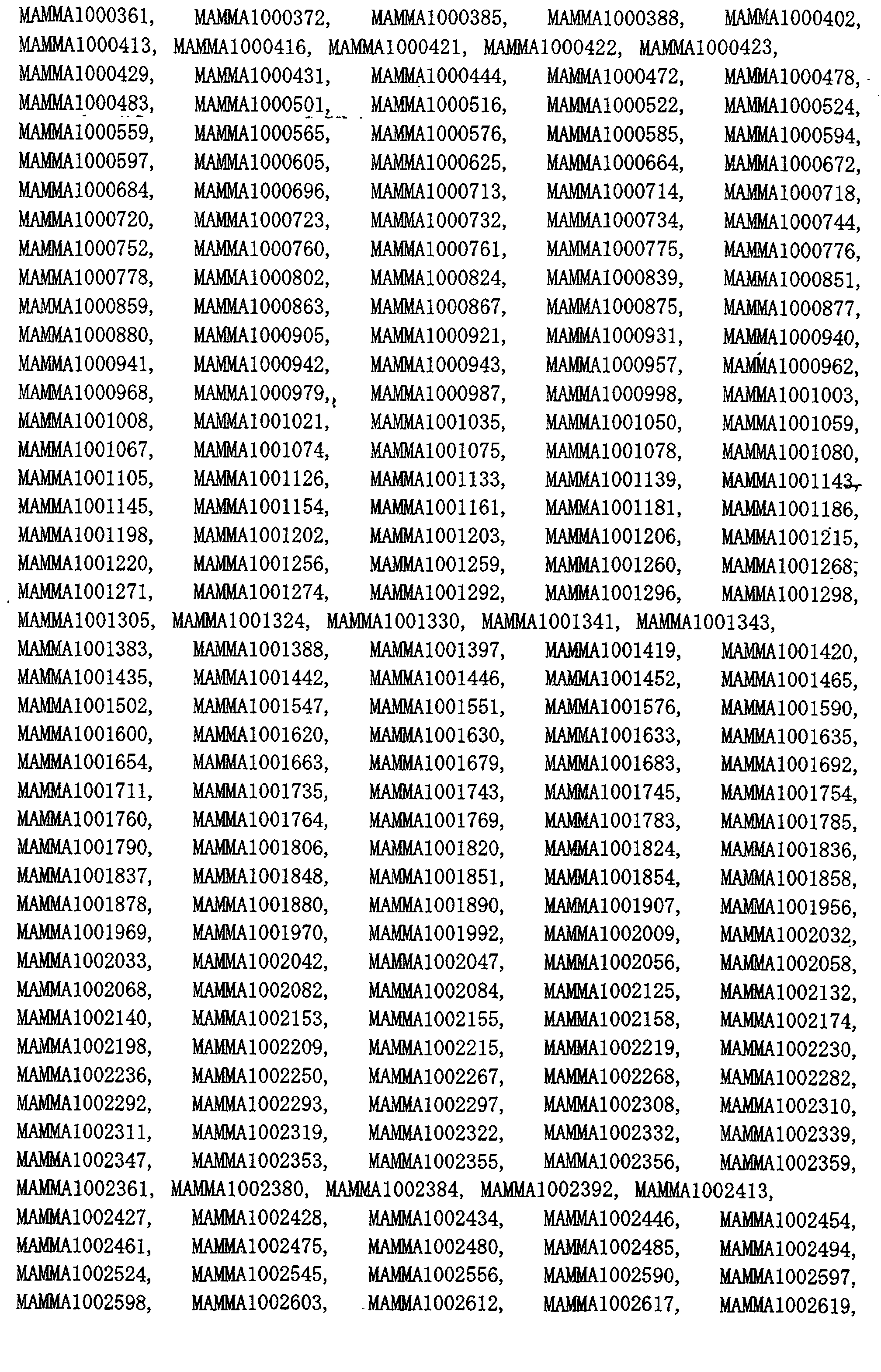

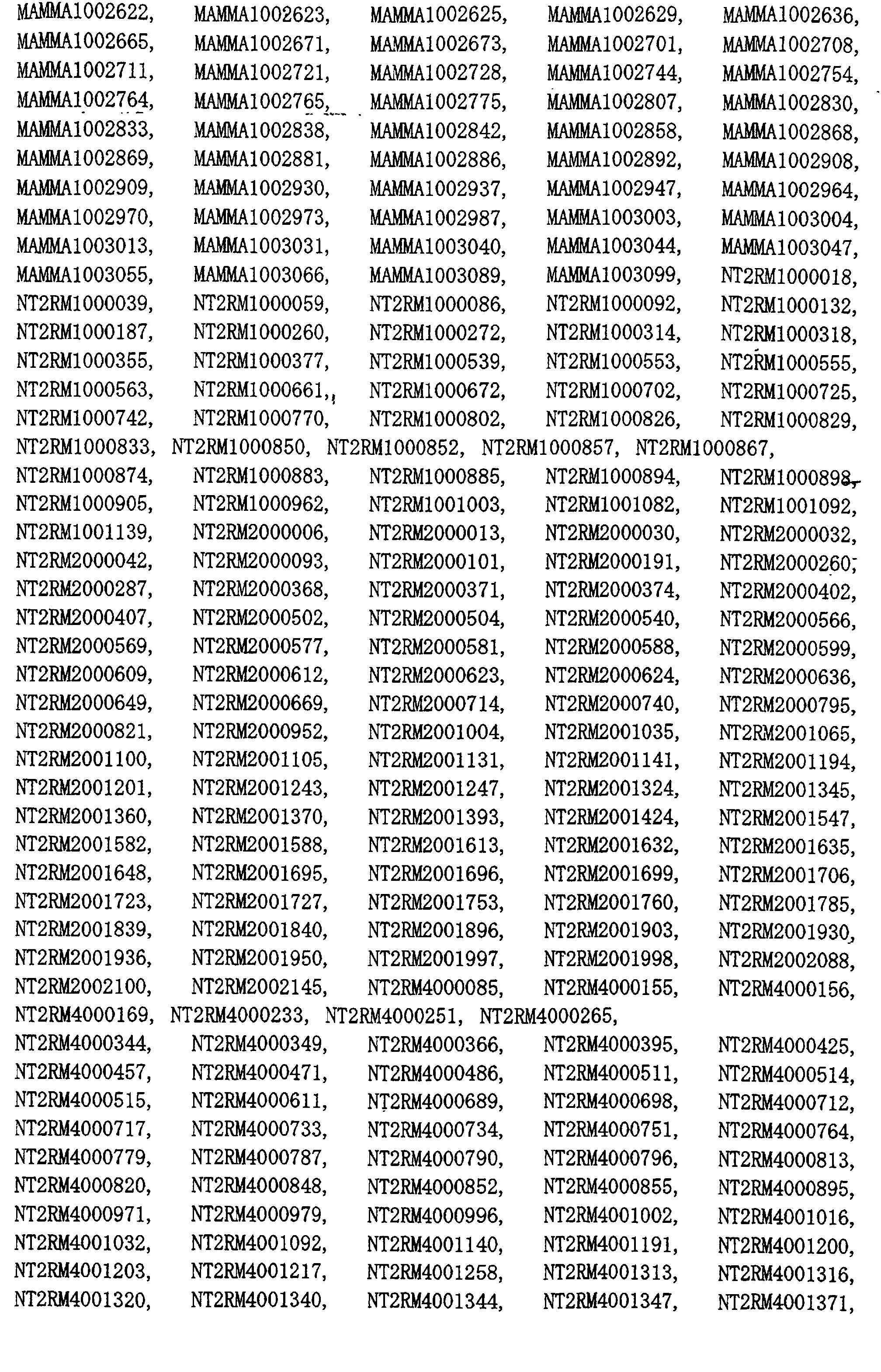

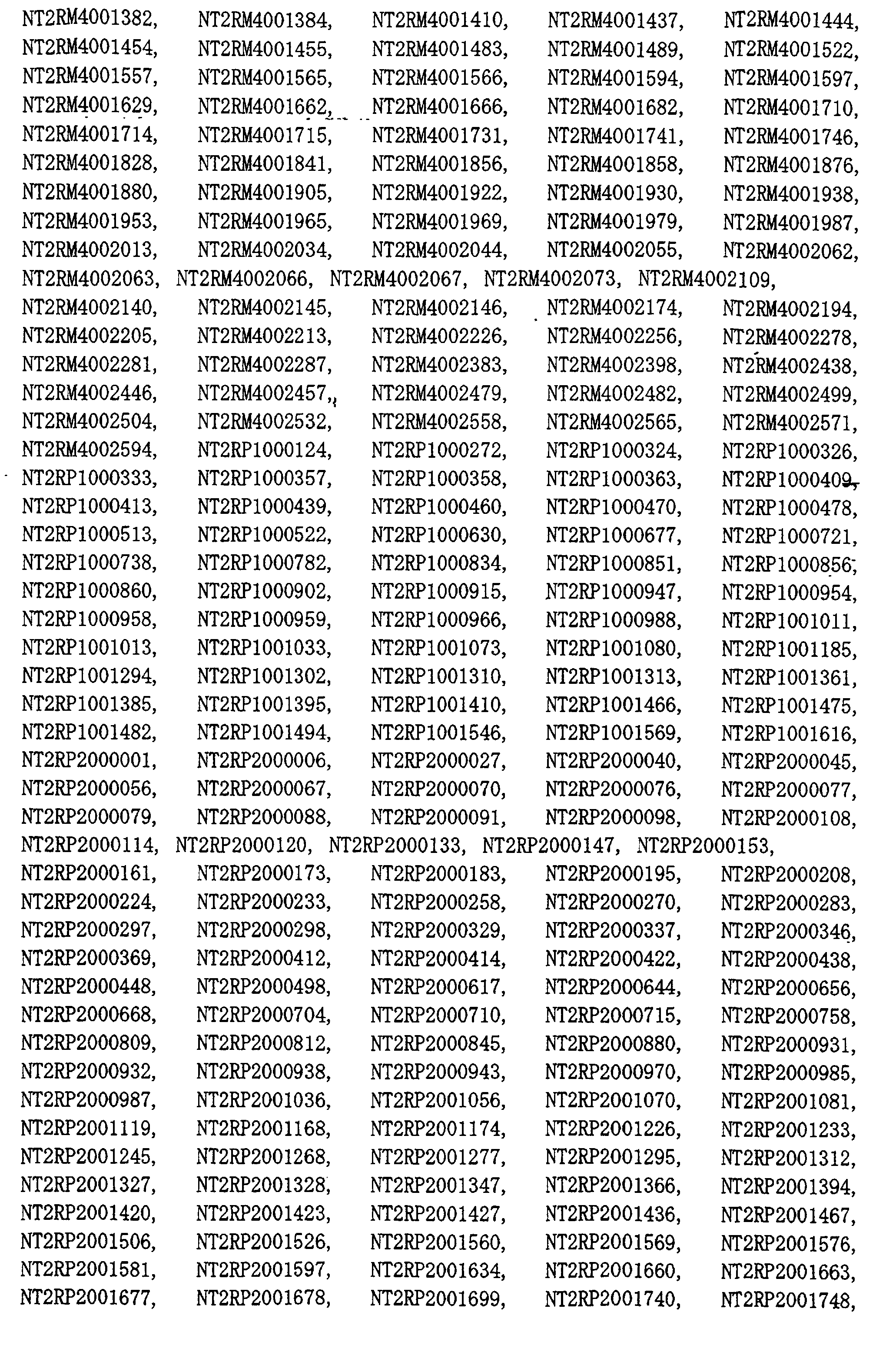

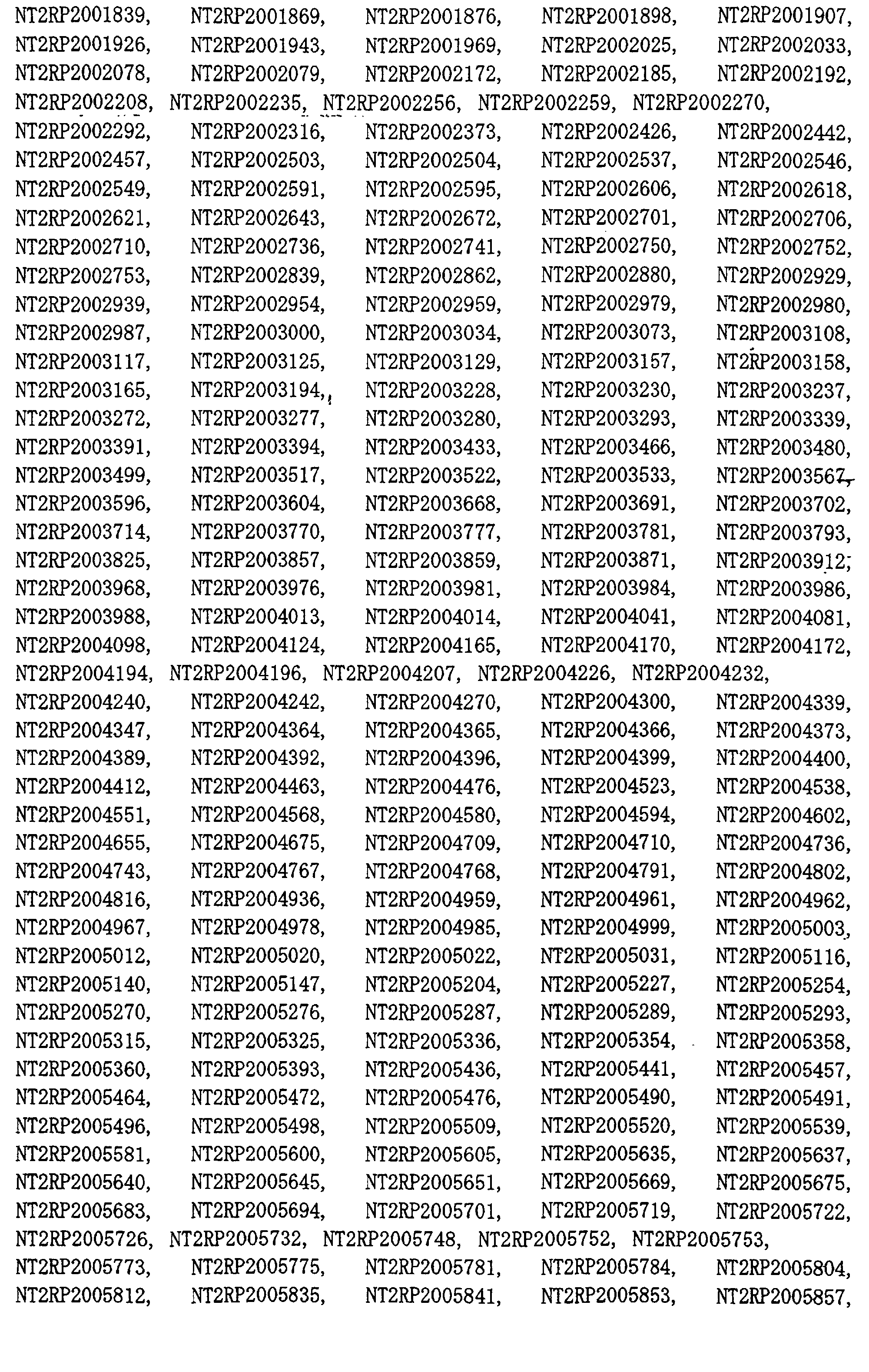

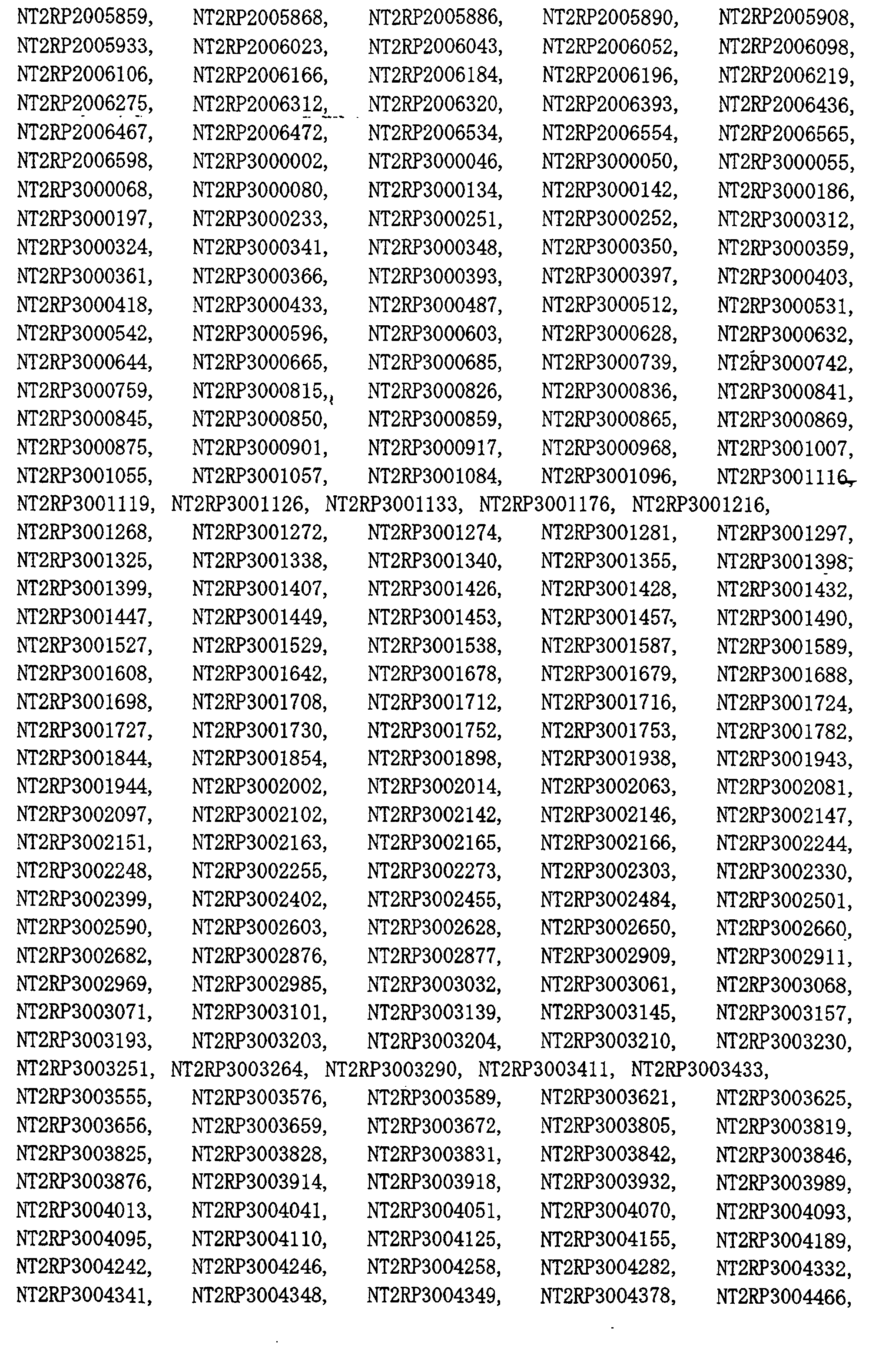

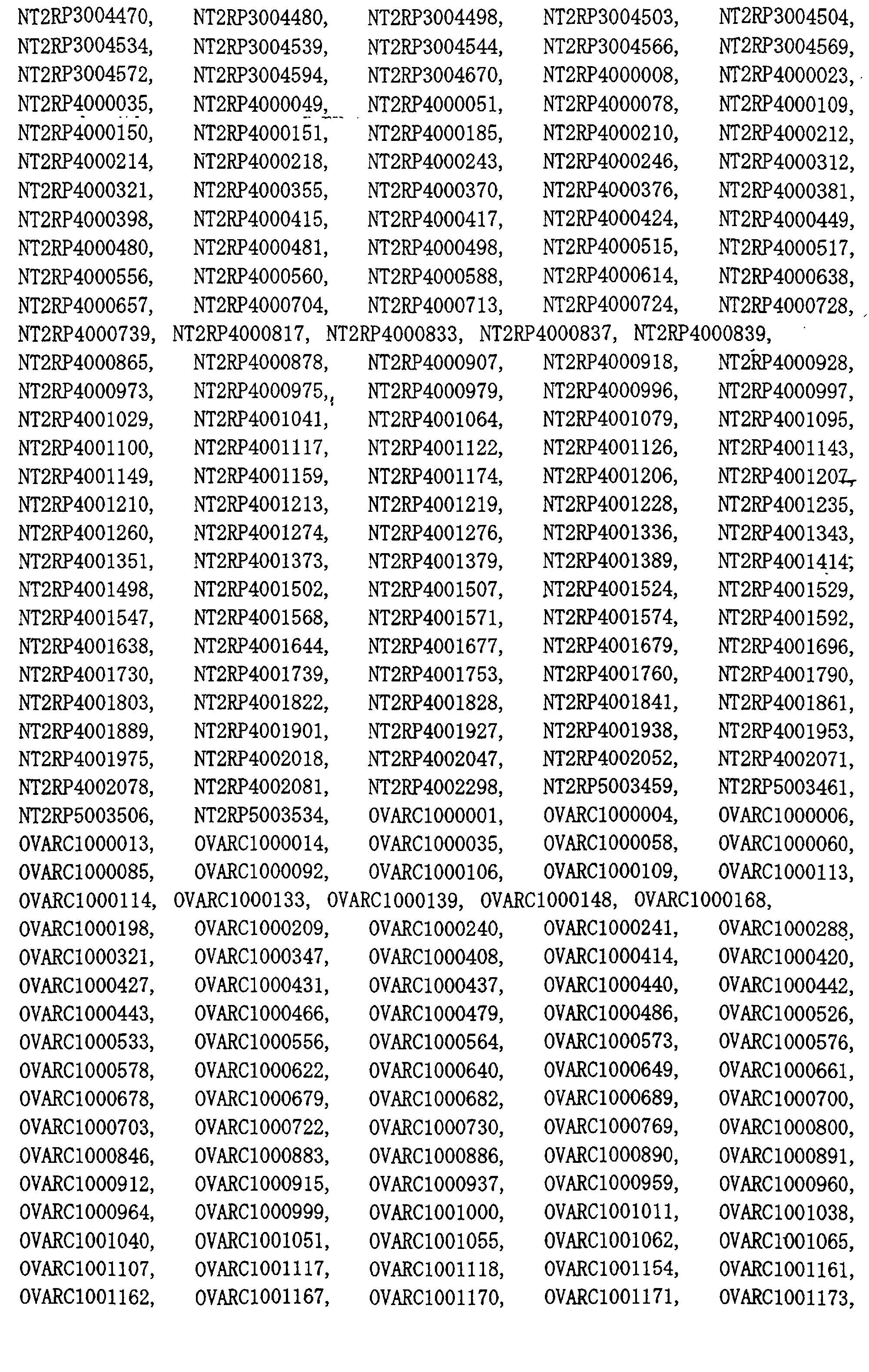

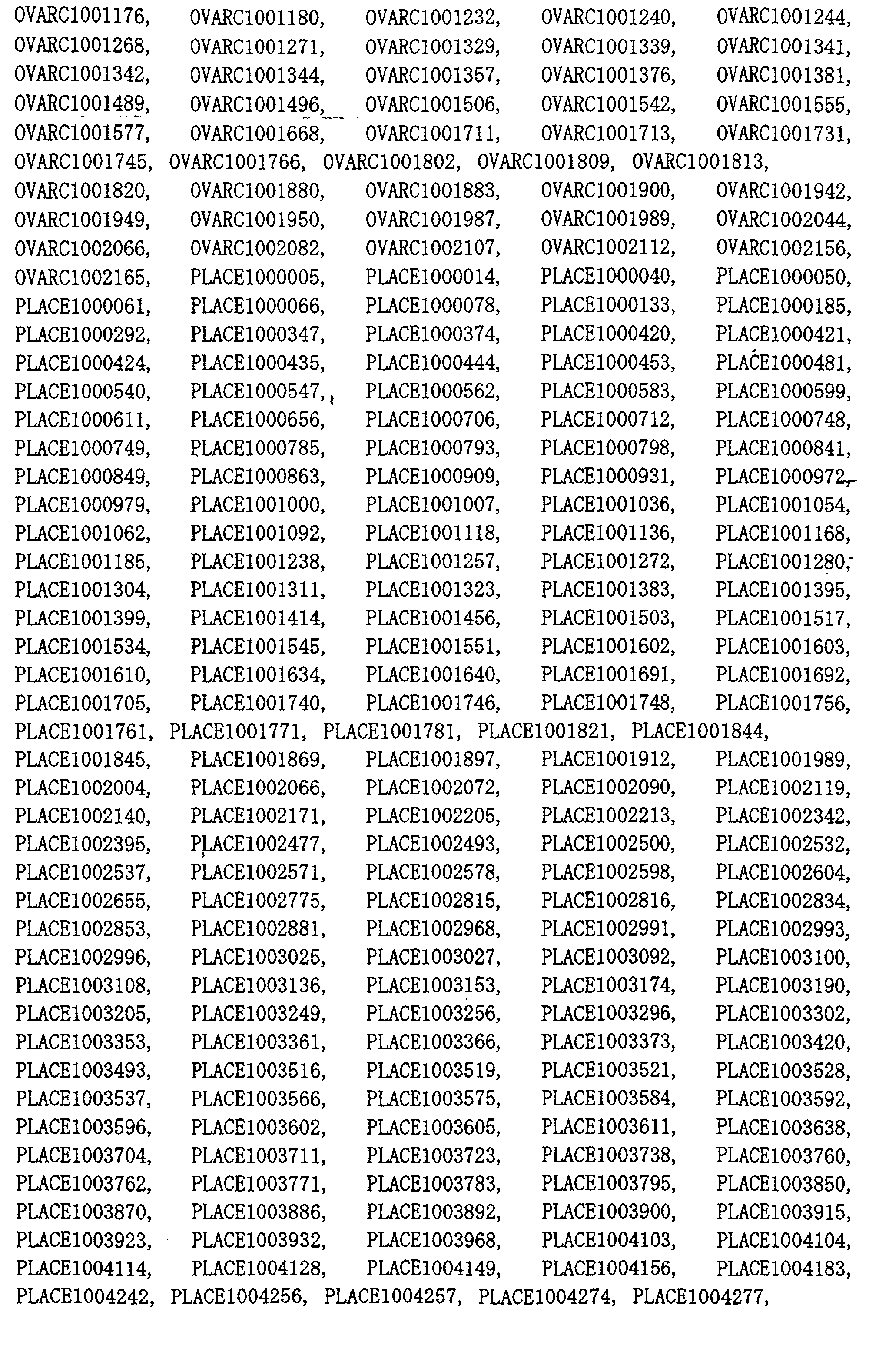

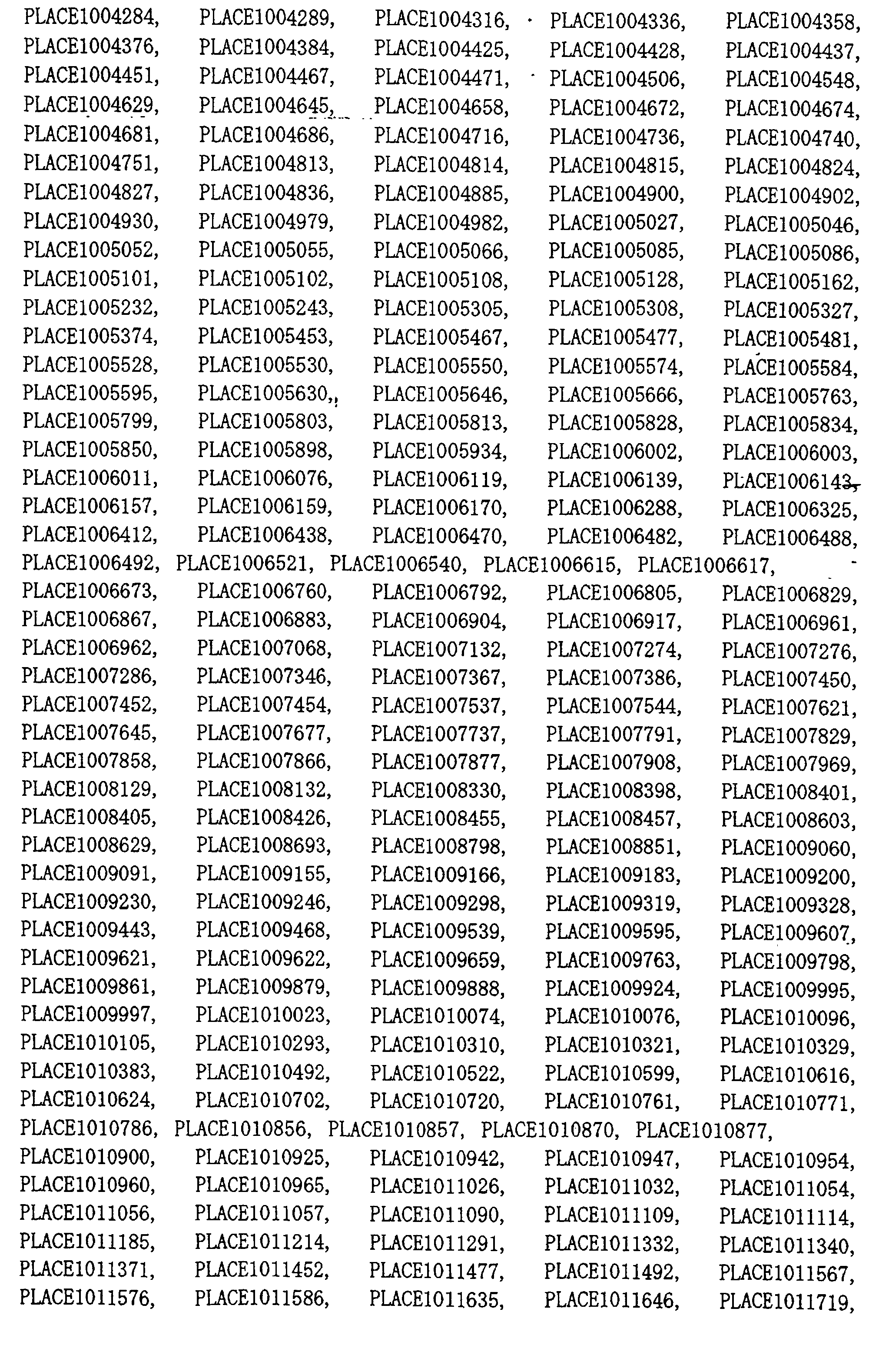

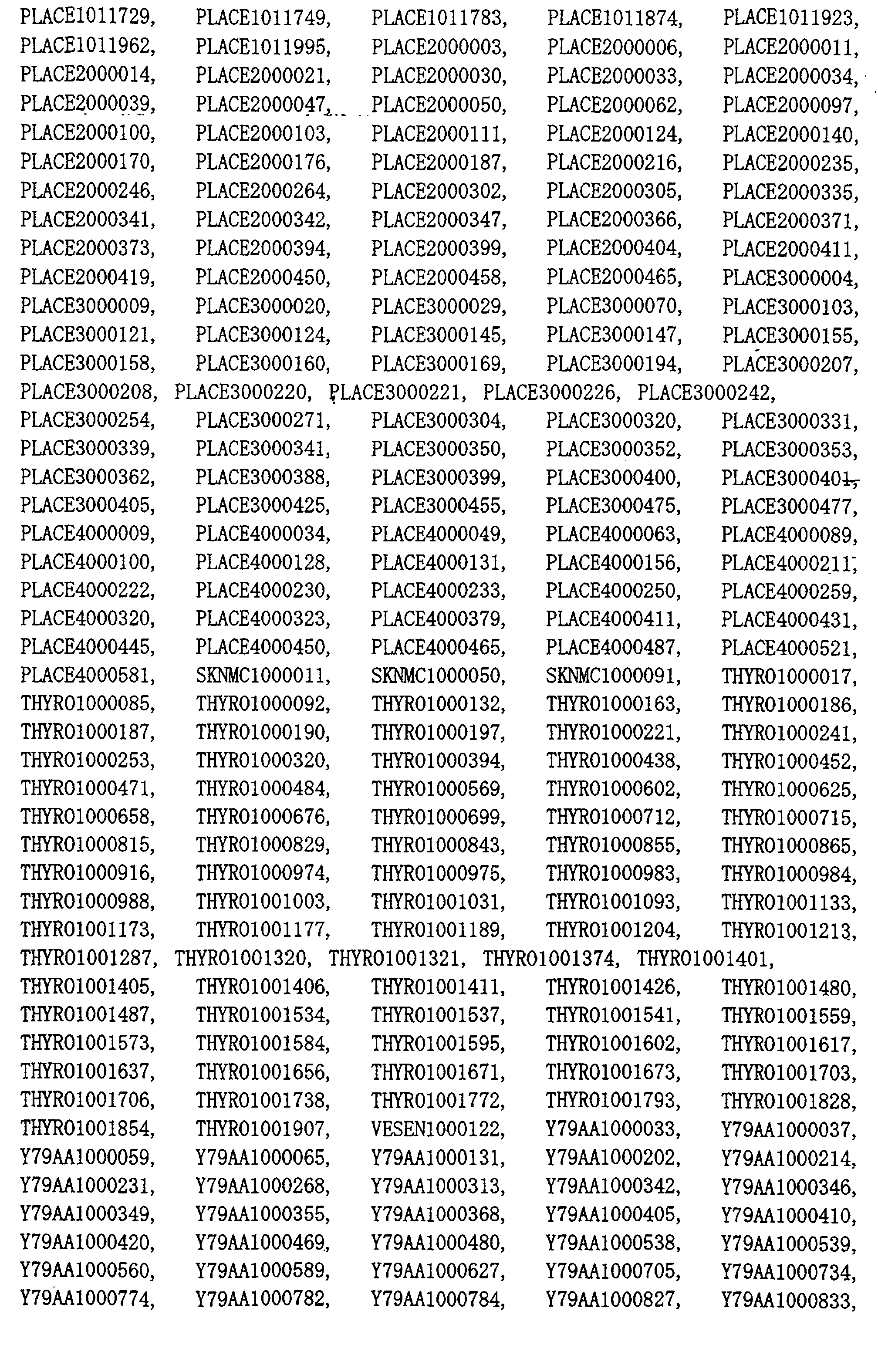

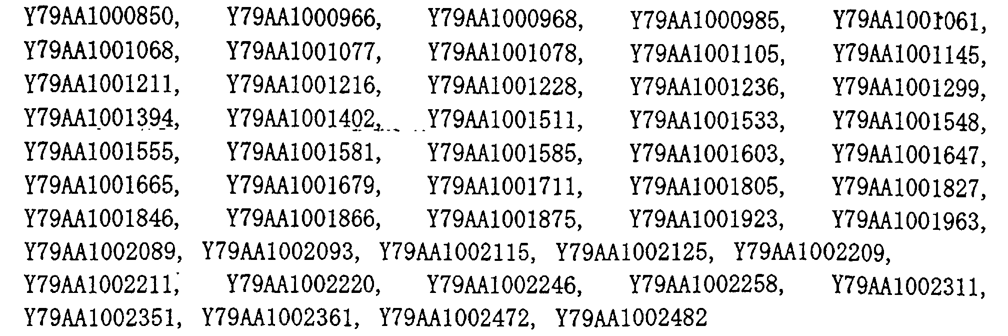

Signal ratios of EC_AGE_BSA to EC_BSA and of EC_glycated_BSA to EC_BSA were calculated for each gene. Genes with high signal ratios were selected. In the case of calculating the ratio of signal value of 40 or less to that of more than 40, such signal values were, for convenience, taken as 40 instead of the real values. When the ratio EC_AGE_BSA/EC_BSA is 2 or more, expression of the genes exhibiting such ratio is expected to be elevated due to advanced glycation endproduct of bovine serum albumin. The higher the value is, the higher the gene expression level is. When the ratio EC_AGE_BSA/EC_BSA ranges from 0.5 to 2, expression of the genes exhibiting such ratio is expected to be unaffected due to advanced glycation endproduct of bovine serum albumin. When the ratio EC_AGE_BSA/EC_BSA is less than 0.5, expression of the genes exhibiting such ratio value is expected to be decreased due to advanced glycation endproduct of bovine serum albumin. The lower the value is, the lower the gene expression level is.

Clone with EC_AGE_BSA/EC_BSA ratio of 2 or higher are as follows: HEMBA1003958, MAMMA1001256, PLACE2000411.

Clone with EC_AGE_BSA/EC_BSA ratio of 0.5 or less is as follows: MAMMA1001783.
These cDNAs are associated with diabetes.

When the ratio EC_glycated_BSA/EC_BSA is 2 or more, the expression level of the gene exhibiting such ratio is expected to be elevated due to glycated bovine serum albumin. The higher the value is, the higher the gene expression level is. When the ratio EC_glycated_BSA/EC_BSA ranges from 0.5 to 2, the expression level of the gene exhibiting such ratio is expected to be unaffected with glycated bovine serum albumin. When the ratio EC_glycated_BSA/EC_BSA is less than 0.5, the expression level of a gene exhibiting such ratio is expected to be decreased due to glycated bovine serum albumin. The lower the value is, the lower the gene expression level is.

Clones with EC_glycated_BSA/EC_BSA ratio of 2 or more are as follows: HEMBA1004850, MAMMA1001256, MAMMA1002132 and PLACE3000119.

A clone with EC_glycated_BSA/EC_BSA ratio of 0.5 or less is as follows:
MAMMA1001783.

These cDNAs are also associated with diabetes.

### Analysis of genes associated with neural cell differentiation

Genes involved in neural cell differentiation are useful for treating neurological diseases. It is possible that genes with varying expression levels in response to induction of cellular differentiation in neural cells are associated with neurological diseases.

A survey was performed for genes of which expression levels are varied in response to induction of differentiation (stimulation by retinoic acid (RA)) in cultured cells of a neural strain, NT2.

The NT2 cells were treated basically according to supplier' s instruction manual. "Undifferentiated NT2 cells" means NT2 cells successively cultured in an Opti-MEM I (GIBCO-BRL; catalog No. 31985) containing 10%(v/v) fetal bovine serum and 1%(v/v) penicillin-streptomycin (GIBCO BRL). "NT2 cells cultured in the presence of retinoic acid" means the cells resulted from transferring undifferentiated NT2 cells into a retinoic acid-containing medium, which consists of D-MEM (GIBCO BRL; catalog No. 11965), 10%(v/v) fetal bovine serum, 1%(v/v) penicillin-streptomycin and 10 µM retinoic acid (GIBCO-BRL), and the subsequent successive culture therein for 5 weeks. "NT2 cells that were cultured in the presence of retinoic acid and then further cultured in the presence of cell-division inhibitor added" means NT2 cells resulted from transferring NT2 cells cultured in the presence of retinoic acid for 5 weeks into a cell-division inhibitor-containing medium, which consisted of D-MEM(GIBCO BRL; catalog No.11965), 10%(v/v) fetal bovine serum, 1%(v/v) penicillin-streptomycin, 10µM retinoic acid, 10 µM FudR (5-fluoro-2' -deoxyuridine: GIBCO BRL), 10 µM Urd (Uridine: GIBCO BRL) and 1 µM araC (Cytosine β-D-Arabinofuranoside: GIBCO BRL), and the subsequence successive culture for 2 weeks. Each of the cells were treated with trypsin and then harvested. Total RNAs were extracted from the cells by using S.N.A.P.^{(TM)} Total RNA Isolation kit (Invitrogen^{(r)}). The labeling of probe used for hybridization was carried out by using 10 µg of the total RNA according to the same methods as described above. The data were obtained in triplicate (n=3). The data of signal value representing gene expression level in the cells in the presence of stimulation for inducing differentiation were compared with those in the absence of the stimulation. The comparison was performed by statistical treatment-of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Tables 170-349 show the expression level of each cDNA in undifferentiated NT2 cells, NT2 cells cultured in the presence of RA, and NT2 cells that were cultured in the presence of RA and that were further cultured in the presence of cell-division inhibitor added.

Averaged signal values (M₁, M₂) and sample variances (s₁², s₂²) were calculated for each gene in each of the cells, and then, the pooled sample variances s² were obtained from the sample variances of the two types of cells to be compared. The t values were determined according to the following formula: t=(M1-M2)/s/(1/3+1/3)^{1/2}. When the determined t-value was greater than a t-value at P, which means the probability of significance level, of 0.05 or 0.01 in the t-distribution table with 4 degrees of freeedom, the difference was judged to be found in the expression level of the gene between the two types of cells at p<0.05 or p<0.01, respectively. The tables also include the information on an increase (+) or decrease (-) in the expression level of a gene in the treated cells when the level is compared with that of untreated undifferentiated cells.

Clones of which expression levels increased by RA are as follows:
HEMBA1000005, HEMBA1000042, HEMBA1000046, HEMBA1000076, HEMBA1000111, HEMBA1000141, HEMBA1000150, HEMBA1000185, HEMBA1000282, HEMBA1000304, HEMBA1000307, HEMBA1000338, HEMBA1000357, HEMBA1000376, HEMBA1000387, HEMBA1000392, HEMBA1000428, HEMBA1000456, HEMBA1000459, HEMBA1000469, HEMBA1000504, HEMBA1000508, HEMBA1000519, HEMBA1000540, HEMBA1000545, HEMBA1000557, HEMBA1000563, HEMBA1000568, HEMBA1000575, HEMBA1000588, HEMBA1000592, HEMBA1000604, HEMBA1000622, HEMBA1000655, HEMBA1000673, HEMBA1000682, HEMBA1000726, HEMBA1000727, HEMBA1000749, HEMBA1000769, HEMBA1000774, HEMBA1000791, HEMBA1000822, HEMBA1000872, HEMBA1000876, HEMBA1000910, HEMBA1000942, HEMBA1000943, HEMBA1000960, HEMBA1000972, HEMBA1000974, HEMBA1000991, HEMBA1001008, HEMBA1001020, HEMBA1001043, HEMBA1001051, HEMBA1001060, HEMBA1001071, HEMBA1001077, HEMBA1001085, HEMBA1001094, HEMBA1001109, HEMBA1001121, HEMBA1001122, HEMBA1001140, HEMBA1001172, HEMBA1001226, HEMBA1001235, HEMBA1001265, HEMBA1001281, HEMBA1001294, HEMBA1001299, HEMBA1001319, HEMBA1001323, HEMBA1001330, HEMBA1001351, HEMBA1001361, HEMBA1001377, HEMBA1001388, HEMBA1001391, HEMBA1001398, HEMBA1001432, HEMBA1001435, HEMBA1001442, HEMBA1001454, HEMBA1001455, HEMBA1001497, HEMBA1001517, HEMBA1001569, HEMBA1001570, HEMBA1001581, HEMBA1001585, HEMBA1001620, HEMBA1001711, HEMBA1001718, HEMBA1001723, HEMBA1001761, HEMBA1001815, HEMBA1001819, HEMBA1001861, HEMBA1001864, HEMBA1001869, HEMBA1001888, HEMBA1001915, HEMBA1001918, HEMBA1001940, HEMBA1001964, HEMBA1001967, HEMBA1001979, HEMBA1001987, HEMBA1001991, HEMBA1002008, HEMBA1002022, HEMBA1002039, HEMBA1002049, HEMBA1002084, HEMBA1002102, HEMBA1002113, HEMBA1002144, HEMBA1002160, HEMBA1002162, HEMBA1002185, HEMBA1002212, HEMBA1002226, HEMBA1002229, HEMBA1002267, HEMBA1002270, HEMBA1002337, HEMBA1002381, HEMBA1002458, HEMBA1002477, HEMBA1002508, HEMBA1002558, HEMBA1002561, HEMBA1002583, HEMBA1002590, HEMBA1002628, HEMBA1002645, HEMBA1002661, HEMBA1002678, HEMBA1002712, HEMBA1002728, HEMBA1002780, HEMBA1002850, HEMBA1002886, HEMBA1002934, HEMBA1002935, HEMBA1002939, HEMBA1002951, HEMBA1002968, HEMBA1002970, HEMBA1002973, HEMBA1002999, HEMBA1003021, HEMBA1003033, HEMBA1003034, HEMBA1003064, HEMBA1003067, HEMBA1003078, HEMBA1003086, HEMBA1003096, HEMBA1003129, HEMBA1003142, HEMBA1003148, HEMBA1003166, HEMBA1003175, HEMBA1003197, HEMBA1003199, HEMBA1003202, HEMBA1003204, HEMBA1003212, HEMBA1003235, HEMBA1003250, HEMBA1003273, HEMBA1003276, HEMBA1003278, HEMBA1003291, HEMBA1003309, HEMBA1003322, HEMBA1003328, HEMBA1003348, HEMBA1003369, HEMBA1003376, HEMBA1003384, HEMBA1003395, HEMBA1003463, HEMBA1003480, HEMBA1003531, HEMBA1003548, HEMBA1003591, HEMBA1003595, HEMBA1003597, HEMBA1003617, HEMBA1003621, HEMBA1003622, HEMBA1003637, HEMBA1003640, HEMBA1003645, HEMBA1003646, HEMBA1003656, HEMBA1003679, HEMBA1003692, HEMBA1003715, HEMBA1003720, HEMBA1003725, HEMBA1003729, HEMBA1003758, HEMBA1003803, HEMBA1003805, HEMBA1003836, HEMBA1003838, HEMBA1003879, HEMBA1003885, HEMBA1003893, HEMBA1003908, HEMBA1003937, HEMBA1003942, HEMBA1003953, HEMBA1003958, HEMBA1003959, HEMBA1003978, HEMBA1003987, HEMBA1003989, HEMBA1004000, HEMBA1004011, HEMBA1004012, HEMBA1004015, HEMBA1004024, HEMBA1004049, HEMBA1004056, HEMBA1004111, HEMBA1004132, HEMBA1004143, HEMBA1004164, HEMBA1004199, HEMBA1004200, HEMBA1004207, HEMBA1004225, HEMBA1004246, HEMBA1004248, HEMBA1004267, HEMBA1004289, HEMBA1004312, HEMBA1004323, HEMBA1004335, HEMBA1004353, HEMBA1004354, HEMBA1004356, HEMBA1004366, HEMBA1004396, HEMBA1004405, HEMBA1004429, HEMBA1004433, HEMBA1004460, HEMBA1004499, HEMBA1004502, HEMBA1004506, HEMBA1004534, HEMBA1004538, HEMBA1004573, HEMBA1004577, HEMBA1004586, HEMBA1004610, HEMBA1004629, HEMBA1004666, HEMBA1004669, HEMBA1004672, HEMBA1004709, HEMBA1004733, HEMBA1004736, HEMBA1004748, HEMBA1004751, HEMBA1004758, HEMBA1004763, HEMBA1004768, HEMBA1004770, HEMBA1004778, HEMBA1004803, HEMBA1004820, HEMBA1004847, HEMBA1004863, HEMBA1004880, HEMBA1004909, HEMBA1004918, HEMBA1004923, HEMBA1004930, HEMBA1004934, HEMBA1004944, HEMBA1004954, HEMBA1004980, HEMBA1005035, HEMBA1005039, HEMBA1005075, HEMBA1005079, HEMBA1005113, HEMBA1005123, HEMBA1005133, HEMBA1005149, HEMBA1005152, HEMBA1005219, HEMBA1005223, HEMBA1005232, HEMBA1005251, HEMBA1005274, HEMBA1005275, HEMBA1005304, HEMBA1005311, HEMBA1005314, HEMBA1005359, HEMBA1005367, HEMBA1005374, HEMBA1005410, HEMBA1005411, HEMBA1005423, HEMBA1005426, HEMBA1005443, HEMBA1005447, HEMBA1005474, HEMBA1005508, HEMBA1005511, HEMBA1005520, HEMBA1005526, HEMBA1005548, HEMBA1005552, HEMBA1005576, HEMBA1005581, HEMBA1005582, HEMBA1005583, HEMBA1005588, HEMBA1005595, HEMBA1005609, HEMBA1005616, HEMBA1005627, HEMBA1005631, HEMBA1005666, HEMBA1005670, HEMBA1005679, HEMBA1005699, HEMBA1005705, HEMBA1005765, HEMBA1005780, HEMBA1005822, HEMBA1005829, HEMBA1005834, HEMBA1005853, HEMBA1005891, HEMBA1005894, HEMBA1005911, HEMBA1005921, HEMBA1005991, HEMBA1005999, HEMBA1006036, HEMBA1006042, HEMBA1006100, HEMBA1006138, HEMBA1006142, HEMBA1006268, HEMBA1006278, HEMBA1006328, HEMBA1006344, HEMBA1006359, HEMBA1006398, HEMBA1006416, HEMBA1006419, HEMBA1006421, HEMBA1006426, HEMBA1006438, HEMBA1006483, HEMBA1006485, HEMBA1006502, HEMBA1006507, HEMBA1006546, HEMBA1006559, HEMBA1006562, HEMBA1006579, HEMBA1006597, HEMBA1006612, HEMBA1006617, HEMBA1006631, HEMBA1006635, HEMBA1006652, HEMBA1006695, HEMBA1006744, HEMBA1006754, HEMBA1006779, HEMBA1006780, HEMBA1006795, HEMBA1006821, HEMBA1006865, HEMBA1006926, HEMBA1006973, HEMBA1007017, HEMBA1007052, HEMBA1007080, HEMBA1007085, HEMBA1007113, HEMBA1007121, HEMBA1007147, HEMBA1007149, HEMBA1007206, HEMBA1007224, HEMBA1007256, HEMBA1007267, HEMBA1007288, HEMBA1007327, HEMBA1007341, HEMBA1007347,
HEMBB1000005, HEMBB1000008, HEMBB1000018, HEMBB1000024, HEMBB1000030, HEMBB1000039, HEMBB1000048, HEMBB1000054, HEMBB1000059, HEMBB1000083, HEMBB1000089, HEMBB1000099, HEMBB1000113, HEMBB1000141, HEMBB1000144, HEMBB1000173, HEMBB1000175, HEMBB1000215, HEMBB1000218, HEMBB1000258, HEMBB1000264, HEMBB1000272, HEMBB1000307, HEMBB1000338, HEMBB1000339, HEMBB1000343, HEMBB1000354, HEMBB1000374, HEMBB1000376, HEMBB1000420, HEMBB1000434, HEMBB1000441, HEMBB1000449, HEMBB1000490, HEMBB1000491, HEMBB1000523, HEMBB1000530, HEMBB1000550, HEMBB1000554, HEMBB1000573, HEMBB1000575, HEMBB1000593, HEMBB1000598, HEMBB1000638, HEMBB1000643, HEMBB1000649, HEMBB1000671, HEMBB1000684, HEMBB1000709, HEMBB1000726, HEMBB1000770, HEMBB1000774, HEMBB1000790, HEMBB1000835, HEMBB1000840, HEMBB1000848, HEMBB1000870, HEMBB1000883, HEMBB1000887, HEMBB1000890, HEMBB1000910, HEMBB1000917, HEMBB1000959, HEMBB1000985, HEMBB1000996, HEMBB1001004, HEMBB1001020, HEMBB1001024, HEMBB1001037, HEMBB1001060, HEMBB1001096, HEMBB1001114, HEMBB1001117, HEMBB1001142, HEMBB1001153, HEMBB1001177, HEMBB1001210, HEMBB1001218, HEMBB1001242, HEMBB1001267, HEMBB1001289, HEMBB1001346, HEMBB1001348, HEMBB1001364, HEMBB1001366, HEMBB1001369, HEMBB1001380, HEMBB1001387, HEMBB1001394, HEMBB1001426, HEMBB1001436, HEMBB1001454, HEMBB1001463, HEMBB1001521, HEMBB1001535, HEMBB1001537, HEMBB1001555, HEMBB1001585, HEMBB1001603, HEMBB1001619, HEMBB1001668, HEMBB1001685, HEMBB1001695, HEMBB1001704, HEMBB1001706, HEMBB1001707, HEMBB1001735, HEMBB1001747, HEMBB1001749, HEMBB1001760, HEMBB1001812, HEMBB1001816, HEMBB1001831, HEMBB1001836, HEMBB1001850, HEMBB1001863, HEMBB1001867, HEMBB1001908, HEMBB1001910, HEMBB1001911, HEMBB1001915, HEMBB1001921, HEMBB1001930, HEMBB1001944, HEMBB1001952, HEMBB1001957, HEMBB1001967, HEMBB1001988, HEMBB1001997, HEMBB1002005, HEMBB1002042, HEMBB1002043, HEMBB1002045, HEMBB1002049, HEMBB1002069, HEMBB1002094, HEMBB1002139, HEMBB1002152, HEMBB1002189, HEMBB1002217, HEMBB1002232, HEMBB1002249, HEMBB1002254, HEMBB1002266, HEMBB1002280, HEMBB1002306, HEMBB1002364, HEMBB1002371, HEMBB1002409, HEMBB1002425, HEMBB1002453, HEMBB1002492, HEMBB1002520, HEMBB1002556, HEMBB1002582, HEMBB1002590, HEMBB1002607, HEMBB1002614, HEMBB1002617, HEMBB1002623, HEMBB1002635, HEMBB1002677, HEMBB1002683, HEMBB1002684, HEMBB1002697, HEMBB1002699, HEMBB1002705,
MAMMA1000009, MAMMA1000043, MAMMA1000045, MAMMA1000084, MAMMA1000103, MAMMA1000134, MAMMA1000139, MAMMA1000143, MAMMA1000171, MAMMA1000241, MAMMA1000251, MAMMA1000254, MAMMA1000257, MAMMA1000264, MAMMA1000266, MAMMA1000270, MAMMA1000279, MAMMA1000287, MAMMA1000340, MAMMA1000348, MAMMA1000360, MAMMA1000361, MAMMA1000372, MAMMA1000385, MAMMA1000402, MAMMA1000424, MAMMA1000478, MAMMA1000500, MAMMA1000522, MAMMA1000524, MAMMA1000565, MAMMA1000567, MAMMA1000576, MAMMA1000585, MAMMA1000594, MAMMA1000597, MAMMA1000605, MAMMA1000616, MAMMA1000643, MAMMA1000664, MAMMA1000669, MAMMA1000696, MAMMA1000718, MAMMA1000723, MAMMA1000731, MAMMA1000744, MAMMA1000746, MAMMA1000752, MAMMA1000760, MAMMA1000761, MAMMA1000776, MAMMA1000802, MAMMA1000824, MAMMA1000839, MAMMA1000880, MAMMA1000905, MAMMA1000931, MAMMA1000940, MAMMA1000941, MAMMA1000943, MAMMA1000957, MAMMA1000962, MAMMA1000968, MAMMA1000987, MAMMA1000998, MAMMA1001003, MAMMA1001035, MAMMA1001038, MAMMA1001067, MAMMA1001078, MAMMA1001092, MAMMA1001126, MAMMA1001133, MAMMA1001139, MAMMA1001161, MAMMA1001186, MAMMA1001202, MAMMA1001206, MAMMA1001220, MAMMA1001243, MAMMA1001268, MAMMA1001274, MAMMA1001296, MAMMA1001298, MAMMA1001305, MAMMA1001343, MAMMA1001383, MAMMA1001397, MAMMA1001419, MAMMA1001420, MAMMA1001435, MAMMA1001442, MAMMA1001663, MAMMA1001670, MAMMA1001683, MAMMA1001692, MAMMA1001740, MAMMA1001745, MAMMA1001754, MAMMA1001768, MAMMA1001769, MAMMA1001783, MAMMA1001785, MAMMA1001806, MAMMA1001890, MAMMA1001907, MAMMA1001969, MAMMA1002009, MAMMA1002032, MAMMA1002056, MAMMA1002058, MAMMA1002125, MAMMA1002132, MAMMA1002140, MAMMA1002155, MAMMA1002158, MAMMA1002174, MAMMA1002198, MAMMA1002230, MAMMA1002293, MAMMA1002310, MAMMA1002311, MAMMA1002322, MAMMA1002339, MAMMA1002359, MAMMA1002384, MAMMA1002428, MAMMA1002434, MAMMA1002475, MAMMA1002494, MAMMA1002545, MAMMA1002573, MAMMA1002603, MAMMA1002619, MAMMA1002622, MAMMA1002623, MAMMA1002625, MAMMA1002662, MAMMA1002698, MAMMA1002701, MAMMA1002708, MAMMA1002721, MAMMA1002744, MAMMA1002764, MAMMA1002769, MAMMA1002820, MAMMA1002830, MAMMA1002833, MAMMA1002868, MAMMA1002871, MAMMA1002892, MAMMA1002895, MAMMA1002908, MAMMA1002909, MAMMA1002930, MAMMA1002941, MAMMA1002964, MAMMA1002970, MAMMA1002973, MAMMA1002987, MAMMA1003004, MAMMA1003040, MAMMA1003066, MAMMA1003089,
NT2RM1000039, NT2RM1000672, NT2RM1000691, NT2RM1000725, NT2RM1000781, NT2RM1000829, NT2RM1000882, NT2RM1001105, NT2RM2000013, NT2RM2000374, NT2RM2000420, NT2RM2000452, NT2RM2000504, NT2RM2000609, NT2RM2000635, NT2RM2000735, NT2RM2000795, NT2RM2000821, NT2RM2001035, NT2RM2001105, NT2RM2001141, NT2RM2001177, NT2RM2001196, NT2RM2001306, NT2RM2001524, NT2RM2001582, NT2RM2001588, NT2RM2001592, NT2RM2001605, NT2RM2001632, NT2RM2001637, NT2RM2001648, NT2RM2001668, NT2RM2001671, NT2RM2001675, NT2RM2001681, NT2RM2001695, NT2RM2001699, NT2RM2001706, NT2RM2001723, NT2RM2001753, NT2RM2001771, NT2RM2001797, NT2RM2001840, NT2RM2001879, NT2RM2002049, NT2RM2002091, NT2RM4000265, NT2RM4000290, NT2RM4000324, NT2RM4000327, NT2RM4000368, NT2RM4000421, NT2RM4000425, NT2RM4000471, NT2RM4000486, NT2RM4000531, NT2RM4000532, NT2RM4000712, NT2RM4000751, NT2RM4000787, NT2RM4000790, NT2RM4000820, NT2RM4000852, NT2RM4000855, NT2RM4000895, NT2RM4000996, NT2RM4001002, NT2RM4001032, NT2RM4001047, NT2RM4001116, NT2RM4001151, NT2RM4001200, NT2RM4001313, NT2RM4001371, NT2RM4001410, NT2RM4001411, NT2RM4001437, NT2RM4001454, NT2RM4001483, NT2RM4001489, NT2RM4001522, NT2RM4001557, NT2RM4001566, NT2RM4001582, NT2RM4001650, NT2RM4001682, NT2RM4001746, NT2RM4001828, NT2RM4001842, NT2RM4001865, NT2RM4001922, NT2RM4001953, NT2RM4001979, NT2RM4002013, NT2RM4002067, NT2RM4002075, NT2RM4002093, NT2RM4002128, NT2RM4002140, NT2RM4002174, NT2RM4002205, NT2RM4002301, NT2RM4002323, NT2RM4002383, NT2RM4002409, NT2RM4002457, NT2RM4002499, NT2RM4002504, NT2RM4002558, NT2RM4002565, NT2RM4002594,
NT2RP1000018, NT2RP1000112, NT2RP1000124, NT2RP1000130, NT2RP1000170, NT2RP1000202, NT2RP1000259, NT2RP1000418, NT2RP1000493, NT2RP1000547, NT2RP1000574, NT2RP1000577, NT2RP1000630, NT2RP1000730, NT2RP1000782, NT2RP1000846, NT2RP1000856, NT2RP1000902, NT2RP1000943, NT2RP1000944, NT2RP1000988, NT2RP1001011, NT2RP1001013, NT2RP1001033, NT2RP1001173, NT2RP1001177, NT2RP1001185, NT2RP1001199, NT2RP1001286, NT2RP1001449, NT2RP1001475, NT2RP1001546, NT2RP2000032, NT2RP2000079, NT2RP2000091, NT2RP2000097, NT2RP2000120, NT2RP2000157, NT2RP2000195, NT2RP2000208, NT2RP2000224, NT2RP2000248, NT2RP2000257, NT2RP2000270, NT2RP2000274, NT2RP2000283, NT2RP2000288, NT2RP2000297, NT2RP2000298, NT2RP2000329, NT2RP2000369, NT2RP2000498, NT2RP2000634, NT2RP2000644, NT2RP2000656, NT2RP2000678, NT2RP2000715, NT2RP2000809, NT2RP2000932, NT2RP2000970, NT2RP2000987, NT2RP2001036, NT2RP2001081, NT2RP2001119, NT2RP2001127, NT2RP2001173, NT2RP2001268, NT2RP2001277, NT2RP2001290, NT2RP2001328, NT2RP2001366, NT2RP2001394, NT2RP2001420, NT2RP2001423, NT2RP2001427, NT2RP2001436, NT2RP2001440, NT2RP2001467, NT2RP2001506, NT2RP2001526, NT2RP2001601, NT2RP2001660, NT2RP2001678, NT2RP2001720, NT2RP2001861, NT2RP2001869, NT2RP2001876, NT2RP2001907, NT2RP2001926, NT2RP2001985, NT2RP2001991, NT2RP2002033, NT2RP2002041, NT2RP2002046, NT2RP2002078, NT2RP2002124, NT2RP2002193, NT2RP2002208, NT2RP2002256, NT2RP2002312, NT2RP2002316, NT2RP2002325, NT2RP2002333, NT2RP2002426, NT2RP2002457, NT2RP2002475, NT2RP2002520, NT2RP2002537, NT2RP2002595, NT2RP2002621, NT2RP2002672, NT2RP2002701, NT2RP2002706, NT2RP2002741, NT2RP2002750, NT2RP2002752, NT2RP2002769, NT2RP2002800, NT2RP2002925, NT2RP2002928, NT2RP2002959, NT2RP2002980, NT2RP2002987, NT2RP2003000, NT2RP2003034, NT2RP2003073, NT2RP2003099, NT2RP2003117, NT2RP2003129, NT2RP2003137, NT2RP2003157, NT2RP2003161, NT2RP2003165, NT2RP2003206, NT2RP2003230, NT2RP2003237, NT2RP2003243, NT2RP2003265, NT2RP2003272, NT2RP2003280, NT2RP2003293, NT2RP2003297, NT2RP2003339, NT2RP2003393, NT2RP2003394, NT2RP2003445, NT2RP2003456, NT2RP2003533, NT2RP2003559, NT2RP2003564, NT2RP2003596, NT2RP2003629, NT2RP2003691, NT2RP2003702, NT2RP2003760, NT2RP2003825, NT2RP2003859, NT2RP2003871, NT2RP2003976, NT2RP2003986, NT2RP2003988, NT2RP2004014, NT2RP2004081, NT2RP2004152, NT2RP2004187, NT2RP2004232, NT2RP2004239, NT2RP2004240, NT2RP2004321, NT2RP2004339, NT2RP2004400, NT2RP2004476, NT2RP2004538, NT2RP2004551, NT2RP2004580, NT2RP2004602, NT2RP2004675, NT2RP2004681, NT2RP2004689, NT2RP2004709, NT2RP2004710, NT2RP2004736, NT2RP2004743, NT2RP2004767, NT2RP2004775, NT2RP2004802, NT2RP2004861, NT2RP2004933, NT2RP2004961, NT2RP2004962, NT2RP2004967, NT2RP2004982, NT2RP2004985, NT2RP2005003, NT2RP2005038, NT2RP2005227, NT2RP2005276, NT2RP2005287, NT2RP2005289, NT2RP2005315, NT2RP2005436, NT2RP2005441, NT2RP2005453, NT2RP2005465, NT2RP2005476, NT2RP2005496, NT2RP2005509, NT2RP2005525, NT2RP2005540, NT2RP2005555, NT2RP2005581, NT2RP2005669, NT2RP2005719, NT2RP2005722, NT2RP2005767, NT2RP2005773, NT2RP2005853, NT2RP2005886, NT2RP2005901, NT2RP2005942, NT2RP2005980, NT2RP2006023, NT2RP2006043, NT2RP2006071, NT2RP2006166, NT2RP2006196, NT2RP2006237, NT2RP2006312, NT2RP2006320, NT2RP2006365, NT2RP2006393, NT2RP2006436, NT2RP2006441, NT2RP2006467, NT2RP2006554, NT2RP2006565, NT2RP2006598,
NT2RP3000002, NT2RP3000050, NT2RP3000072, NT2RP3000109, NT2RP3000134, NT2RP3000197, NT2RP3000341, NT2RP3000359, NT2RP3000366, NT2RP3000403, NT2RP3000418, NT2RP3000433, NT2RP3000441, NT2RP3000526, NT2RP3000531, NT2RP3000542, NT2RP3000561, NT2RP3000562, NT2RP3000603, NT2RP3000605, NT2RP3000644, NT2RP3000759, NT2RP3000815, NT2RP3000825, NT2RP3000836, NT2RP3000847, NT2RP3000850, NT2RP3000865, NT2RP3000901, NT2RP3000968, NT2RP3001007, NT2RP3001057, NT2RP3001120, NT2RP3001126, NT2RP3001140, NT2RP3001214, NT2RP3001216, NT2RP3001236, NT2RP3001245, NT2RP3001253, NT2RP3001260, NT2RP3001268, NT2RP3001274, NT2RP3001281, NT2RP3001297, NT2RP3001355, NT2RP3001356, NT2RP3001383, NT2RP3001396, NT2RP3001407, NT2RP3001420, NT2RP3001428, NT2RP3001449, NT2RP3001453, NT2RP3001472, NT2RP3001490, NT2RP3001495, NT2RP3001497, NT2RP3001527, NT2RP3001554, NT2RP3001589, NT2RP3001642, NT2RP3001679, NT2RP3001688, NT2RP3001712, NT2RP3001752, NT2RP3001782, NT2RP3001799, NT2RP3001844, NT2RP3001926, NT2RP3001989, NT2RP3002002, NT2RP3002004, NT2RP3002033, NT2RP3002056, NT2RP3002062, NT2RP3002102, NT2RP3002142, NT2RP3002173, NT2RP3002248, NT2RP3002304, NT2RP3002343, NT2RP3002484, NT2RP3002529, NT2RP3002545, NT2RP3002549, NT2RP3002682, NT2RP3002687, NT2RP3002713, NT2RP3002876, NT2RP3002877, NT2RP3002972, NT2RP3003032, NT2RP3003133, NT2RP3003139, NT2RP3003145, NT2RP3003157, NT2RP3003193, NT2RP3003204, NT2RP3003210, NT2RP3003212, NT2RP3003251, NT2RP3003264, NT2RP3003282, NT2RP3003290, NT2RP3003301, NT2RP3003313, NT2RP3003346, NT2RP3003403, NT2RP3003500, NT2RP3003543, NT2RP3003576, NT2RP3003672, NT2RP3003828, NT2RP3003831, NT2RP3003992, NT2RP3004041, NT2RP3004093, NT2RP3004155, NT2RP3004209, NT2RP3004215, NT2RP3004246, NT2RP3004332, NT2RP3004348, NT2RP3004349, NT2RP3004470, NT2RP3004472, NT2RP3004480, NT2RP3004490, NT2RP3004503, NT2RP3004566, NT2RP3004569, NT2RP3004572, NT2RP4000035, NT2RP4000147, NT2RP4000167, NT2RP4000212, NT2RP4000214, NT2RP4000259, NT2RP4000360, NT2RP4000381, NT2RP4000398, NT2RP4000424, NT2RP4000448, NT2RP4000455, NT2RP4000517, NT2RP4000519, NT2RP4000524, NT2RP4000614, NT2RP4000728, NT2RP4000737, NT2RP4000817, NT2RP4000865, NT2RP4001079, NT2RP4001122, NT2RP4001150, NT2RP4001174, NT2RP4001274, NT2RP4001353, NT2RP4001447, NT2RP4001507, NT2RP4001547, NT2RP4001677, NT2RP4001679, NT2RP4001730, NT2RP4001803, NT2RP4001889, NT2RP4001946, NT2RP4001953, NT2RP4002018, NT2RP4002071, NT2RP4002791, NT2RP5003461,
OVARC1000058, OVARC1000091, OVARC1000092, OVARC1000113, OVARC1000114, OVARC1000145, OVARC1000168, OVARC1000198, OVARC1000321, OVARC1000335, OVARC1000384, OVARC1000408, OVARC1000414, OVARC1000442, OVARC1000443, OVARC1000486, OVARC1000520, OVARC1000526, OVARC1000556, OVARC1000557, OVARC1000573, OVARC1000578, OVARC1000588, OVARC1000622, OVARC1000679, OVARC1000682, OVARC1000700, OVARC1000703, OVARC1000746, OVARC1000769, OVARC1000800, OVARC1000846, OVARC1000862, OVARC1000885, OVARC1000924, OVARC1000936, OVARC1000959, OVARC1000960, OVARC1000971, OVARC1000984, OVARC1000996, OVARC1000999, OVARC1001000, OVARC1001004, OVARC1001032, OVARC1001034, OVARC1001040, OVARC1001044, OVARC1001092, OVARC1001117, OVARC1001118, OVARC1001154, OVARC1001161, OVARC1001170, OVARC1001173, OVARC1001200, OVARC1001240, OVARC1001268, OVARC1001271, OVARC1001329, OVARC1001341, OVARC1001344, OVARC1001376, OVARC1001381, OVARC1001436, OVARC1001476, OVARC1001489, OVARC1001525, OVARC1001542, OVARC1001547, OVARC1001668, OVARC1001745, OVARC1001767, OVARC1001802, OVARC1001812, OVARC1001820, OVARC1001873, OVARC1001883, OVARC1001949, OVARC1001987, OVARC1001989, OVARC1002044, OVARC1002082, OVARC1002107, OVARC1002138, OVARC1002165,
PLACE1000014, PLACE1000031, PLACE1000040, PLACE1000048, PLACE1000078, PLACE1000184, PLACE1000246, PLACE1000292, PLACE1000332, PLACE1000347, PLACE1000424, PLACE1000435, PLACE1000444, PLACE1000540, PLACE1000547, PLACE1000562, PLACE1000583, PLACE1000599, PLACE1000706, PLACE1000712, PLACE1000755, PLACE1000793, PLACE1000798, PLACE1000979, PLACE1001000, PLACE1001010, PLACE1001062, PLACE1001088, PLACE1001118, PLACE1001136, PLACE1001238, PLACE1001257, PLACE1001280, PLACE1001294, PLACE1001304, PLACE1001311, PLACE1001323, PLACE1001366, PLACE1001384, PLACE1001395, PLACE1001399, PLACE1001456, PLACE1001484, PLACE1001503, PLACE1001570, PLACE1001603, PLACE1001608, PLACE1001610, PLACE1001632, PLACE1001634, PLACE1001691, PLACE1001720, PLACE1001746, PLACE1001760, PLACE1001821, PLACE1001844, PLACE1001845, PLACE1001897, PLACE1001912, PLACE1001989, PLACE1002004, PLACE1002066, PLACE1002072, PLACE1002119, PLACE1002150, PLACE1002157, PLACE1002205, PLACE1002256, PLACE1002259, PLACE1002342, PLACE1002438, PLACE1002450, PLACE1002474, PLACE1002477, PLACE1002499, PLACE1002500, PLACE1002537, PLACE1002578, PLACE1002655, PLACE1002665, PLACE1002722, PLACE1002815, PLACE1002834, PLACE1002851, PLACE1002881, PLACE1002968, PLACE1002993, PLACE1003027, PLACE1003108, PLACE1003174, PLACE1003200, PLACE1003205, PLACE1003249, PLACE1003256, PLACE1003302, PLACE1003334, PLACE1003343, PLACE1003361, PLACE1003373, PLACE1003394, PLACE1003420, PLACE1003516, PLACE1003519, PLACE1003575, PLACE1003584, PLACE1003592, PLACE1003593, PLACE1003638, PLACE1003669, PLACE1003704, PLACE1003723, PLACE1003760, PLACE1003762, PLACE1003768, PLACE1003771, PLACE1003795, PLACE1003833, PLACE1003870, PLACE1003892, PLACE1003915, PLACE1003968, PLACE1004103, PLACE1004149, PLACE1004156, PLACE1004256, PLACE1004277, PLACE1004284, PLACE1004384, PLACE1004425, PLACE1004467, PLACE1004471, PLACE1004473, PLACE1004510, PLACE1004629, PLACE1004658, PLACE1004672, PLACE1004686, PLACE1004751, PLACE1004777, PLACE1004814, PLACE1004815, PLACE1004824, PLACE1004827, PLACE1004836, PLACE1004840, PLACE1004885, PLACE1004972, PLACE1004979,
PLACE1005027, PLACE1005046, PLACE1005055, PLACE1005085, PLACE1005102, PLACE1005108, PLACE1005128, PLACE1005146, PLACE1005266, PLACE1005305, PLACE1005374, PLACE1005409, PLACE1005453, PLACE1005477, PLACE1005481, PLACE1005528, PLACE1005574, PLACE1005666, PLACE1005763, PLACE1005804, PLACE1005828, PLACE1005834, PLACE1005850, PLACE1005851, PLACE1005934, PLACE1006002, PLACE1006076, PLACE1006119, PLACE1006143, PLACE1006159, PLACE1006164, PLACE1006170, PLACE1006187, PLACE1006223, PLACE1006239, PLACE1006248, PLACE1006412, PLACE1006445, PLACE1006482, PLACE1006492, PLACE1006521, PLACE1006540, PLACE1006617, PLACE1006629, PLACE1006673, PLACE1006704, PLACE1006760, PLACE1006792, PLACE1006795, PLACE1006800, PLACE1006860, PLACE1006904, PLACE1006961, PLACE1006962, PLACE1007045, PLACE1007274, PLACE1007346, PLACE1007367, PLACE1007375, PLACE1007386, PLACE1007416, PLACE1007450, PLACE1007454, PLACE1007478, PLACE1007484, PLACE1007544, PLACE1007547, PLACE1007557, PLACE1007598, PLACE1007645, PLACE1007677, PLACE1007743, PLACE1007807, PLACE1007829, PLACE1007858, PLACE1008002, PLACE1008129, PLACE1008132, PLACE1008201, PLACE1008209, PLACE1008273, PLACE1008368, PLACE1008532, PLACE1008568, PLACE1008696, PLACE1008867, PLACE1008887, PLACE1008941, PLACE1009027, PLACE1009039, PLACE1009050, PLACE1009099, PLACE1009155, PLACE1009172, PLACE1009174, PLACE1009298, PLACE1009328, PLACE1009335, PLACE1009338, PLACE1009388, PLACE1009444, PLACE1009595, PLACE1009596, PLACE1009607, PLACE1009621, PLACE1009637, PLACE1009665, PLACE1009708, PLACE1009798, PLACE1009861, PLACE1009886, PLACE1009971, PLACE1009995, PLACE1010089, PLACE1010102, PLACE1010106, PLACE1010152, PLACE1010383, PLACE1010491, PLACE1010616, PLACE1010630, PLACE1010631, PLACE1010702, PLACE1010739, PLACE1010761, PLACE1010833, PLACE1010870, PLACE1010896, PLACE1010916, PLACE1010925, PLACE1010942, PLACE1010954, PLACE1010965, PLACE1011041, PLACE1011046, PLACE1011054, PLACE1011057, PLACE1011090, PLACE1011109, PLACE1011203, PLACE1011214, PLACE1011296, PLACE1011340, PLACE1011433, PLACE1011452, PLACE1011567, PLACE1011576, PLACE1011643, PLACE1011675, PLACE1011719, PLACE1011729, PLACE1011749, PLACE1011762, PLACE1011783, PLACE1011874, PLACE1011995,
PLACE2000017, PLACE2000021, PLACE2000033, PLACE2000039, PLACE2000047, PLACE2000062, PLACE2000103, PLACE2000124, PLACE2000170, PLACE2000216, PLACE2000235, PLACE2000264, PLACE2000302, PLACE2000305, PLACE2000335, PLACE2000342, PLACE2000347, PLACE2000379, PLACE2000394, PLACE2000433, PLACE2000450, PLACE2000465, PLACE3000103, PLACE3000119, PLACE3000121, PLACE3000124, PLACE3000155, PLACE3000158, PLACE3000207, PLACE3000220, PLACE3000242, PLACE3000271, PLACE3000304, PLACE3000322, PLACE3000331, PLACE3000341, PLACE3000362, PLACE3000365, PLACE3000388, PLACE3000399, PLACE3000400, PLACE3000401, PLACE3000402, PLACE3000425, PLACE3000455, PLACE4000034, PLACE4000049, PLACE4000089, PLACE4000128, PLACE4000156, PLACE4000222, PLACE4000250, PLACE4000320, PLACE4000379, PLACE4000431, PLACE4000445, PLACE4000465, PLACE4000487, PLACE4000494, PLACE4000522, PLACE4000558, PLACE4000581, PLACE4000650, PLACE4000654,
THYRO1000034, THYRO1000085, THYRO1000092, THYRO1000111, THYRO1000156, THYRO1000163, THYRO1000173, THYRO1000190, THYRO1000197, THYRO1000221, THYRO1000241, THYRO1000327, THYRO1000381, THYRO1000387, THYRO1000394, THYRO1000488, THYRO1000585, THYRO1000625, THYRO1000637, THYRO1000658, THYRO1000666, THYRO1000676, THYRO1000684, THYRO1000712, THYRO1000734, THYRO1000793, THYRO1000796, THYRO1000805, THYRO1000815, THYRO1000865, THYRO1000916, THYRO1000934, THYRO1000974, THYRO1000975, THYRO1001031, THYRO1001062, THYRO1001093, THYRO1001133, THYRO1001173, THYRO1001177,
THYRO1001189, THYRO1001204, THYRO1001213, THYRO1001262, THYRO1001290, THYRO1001320, THYRO1001322, THYRO1001401, THYRO1001406, THYRO1001426, THYRO1001480, THYRO1001487, THYRO1001537, THYRO1001595, THYRO1001617, THYRO1001637, THYRO1001706, THYRO1001772, THYRO1001828, THYRO1001854, Y79AA1000059, Y79AA1000214, Y79AA1000355, Y79AA1000410, Y79AA1000538, Y79AA1000539, Y79AA1000705, Y79AA1000800, Y79AA1000850, Y79AA1000962, Y79AA1000976, Y79AA1001061, Y79AA1001068, Y79AA1001493, Y79AA1001548, Y79AA1001585, Y79AA1001594, Y79AA1001696, Y79AA1001711, Y79AA1002103, Y79AA1002115, Y79AA1002258, Y79AA1002361, Y79AA1002407, Y79AA1002472, Y79AA1002482.

Clones of which expression levels decreased by RA are as follows:
HEMBA1000946, HEMBA1003569, HEMBA1005570, HEMBB1000915, NT2RM1000666, NT2RM2000092, NT2RM2000594, NT2RM2001256, NT2RM4001754, NT2RM4001905, NT2RP2001675, NT2RP2002047, NT2RP2005491, NT2RP3000980, NT2RP3002081, NT2RP3004594, NT2RP4001950, NT2RP4002408, OVARC1000431, OVARC1001942, OVARC1001943, PLACE1003190, PLACE1004868, PLACE1005923, PLACE1007257, PLACE1010624, Y79AA1000346.

Clones of which expression levels increase by RA/inhibitor are as follows:
HEMBA1000046, HEMBA1000307, HEMBA1000434, HEMBA1000504, HEMBA1000588, HEMBA1000682, HEMBA1000726, HEMBA1000943, HEMBA1001071, HEMBA1001094, HEMBA1001122, HEMBA1001323, HEMBA1001361, HEMBA1001455, HEMBA1001709, HEMBA1001746, HEMBA1001869, HEMBA1002084, HEMBA1002583, HEMBA1002628, HEMBA1002801, HEMBA1002937, HEMBA1003096, HEMBA1003142, HEMBA1003229, HEMBA1003276, HEMBA1003309, HEMBA1003463, HEMBA1003597, HEMBA1003617, HEMBA1003725, HEMBA1003803, HEMBA1003879, HEMBA1003989, HEMBA1004000, HEMBA1004015, HEMBA1004024, HEMBA1004049, HEMBA1004056, HEMBA1004199, HEMBA1004248, HEMBA1004356, HEMBA1004554, HEMBA1004666, HEMBA1004725, HEMBA1004770, HEMBA1004803, HEMBA1004923, HEMBA1004934, HEMBA1004954, HEMBA1005039, HEMBA1005075, HEMBA1005113, HEMBA1005219, HEMBA1005232, HEMBA1005251, HEMBA1005304, HEMBA1005367, HEMBA1005372, HEMBA1005403, HEMBA1005410, HEMBA1005411, HEMBA1005548, HEMBA1005581, HEMBA1005631, HEMBA1005666, HEMBA1005755, HEMBA1005780, HEMBA1006067, HEMBA1006130, HEMBA1006364, HEMBA1006485, HEMBA1006559, HEMBA1006579, HEMBA1006754, HEMBB1000059, HEMBB1000575, HEMBB1000709, HEMBB1000822, HEMBB1000848, HEMBB1000852, HEMBB1000913, HEMBB1000985, HEMBB1001117, HEMBB1001210, HEMBB1001317, HEMBB1001394, HEMBB1001443, HEMBB1001668, HEMBB1001695, HEMBB1002049, HEMBB1002254, HEMBB1002266, HEMBB1002371, HEMBB1002502, HEMBB1002614, HEMBB1002617, HEMBB1002692, HEMBB1002697, MAMMA1000241, MAMMA1000424, MAMMA1000616, MAMMA1000731, MAMMA1000824, MAMMA1000908, MAMMA1000956, MAMMA1001038, MAMMA1001091, MAMMA1001243, MAMMA1001815, MAMMA1001820, MAMMA1002267, MAMMA1002769, MAMMA1002871, MAMMA1002941, NT2RM1000355, NT2RM1000725, NT2RM1000829, NT2RM1000850, NT2RM1000898, NT2RM2000504, NT2RM2000635, NT2RM2000718, NT2RM2000821, NT2RM2001370, NT2RM2001582, NT2RM2001592, NT2RM2001613, NT2RM2001632, NT2RM2001635, NT2RM2001648, NT2RM2001659, NT2RM2001671, NT2RM2001695, NT2RM2001760, NT2RM2001782, NT2RM2001839, NT2RM2001879, NT2RM2001983, NT2RM4000104, NT2RM4000290, NT2RM4000425, NT2RM4000433, NT2RM4000471, NT2RM4000531, NT2RM4000852, NT2RM4001047, NT2RM4001347, NT2RM4001454, NT2RM4001557, NT2RM4001566, NT2RM4001582, NT2RM4001938, NT2RM4001953, NT2RM4002018, NT2RM4002409, NT2RM4002558, NT2RM4002594, NT2RP1000259, NT2RP1000418, NT2RP1000574, NT2RP1000629, NT2RP1000782, NT2RP1000856, NT2RP1000943, NT2RP1000988, NT2RP1001013, NT2RP1001173, NT2RP1001546, NT2RP2000091, NT2RP2000208, NT2RP2000274, NT2RP2000329, NT2RP2000369, NT2RP2000634, NT2RP2000842, NT2RP2000943, NT2RP2000987, NT2RP2001094, NT2RP2001277, NT2RP2001290, NT2RP2001366, NT2RP2001423, NT2RP2001436, NT2RP2001467, NT2RP2001506, NT2RP2001601, NT2RP2001663, NT2RP2001926, NT2RP2001985, NT2RP2002032, NT2RP2002041, NT2RP2002046, NT2RP2002078, NT2RP2002124, NT2RP2002185, NT2RP2002193, NT2RP2002312, NT2RP2002316, NT2RP2002426, NT2RP2002457, NT2RP2002475, NT2RP2002520, NT2RP2002595, NT2RP2002643, NT2RP2002672, NT2RP2002701, NT2RP2002710, NT2RP2002727, NT2RP2003099, NT2RP2003121, NT2RP2003137, NT2RP2003158, NT2RP2003206, NT2RP2003230, NT2RP2003272, NT2RP2003280, NT2RP2003347, NT2RP2003393, NT2RP2003401, NT2RP2003445, NT2RP2003456, NT2RP2003511, NT2RP2003517, NT2RP2003543, NT2RP2003596, NT2RP2003706, NT2RP2003871, NT2RP2004681, NT2RP2004743, NT2RP2004775, NT2RP2004933, NT2RP2004967, NT2RP2005003, NT2RP2005270, NT2RP2005289, NT2RP2005344, NT2RP2005453, NT2RP2005555, NT2RP2005767, NT2RP2005853, NT2RP2006043; NT2RP2006393, NT2RP2006436, NT2RP2006441, NT2RP2006467, NT2RP2006534, NT2RP2006565, NT2RP3000348, NT2RP3000359, NT2RP3000366, NT2RP3000403, NT2RP3000418, NT2RP3000441, NT2RP3000561, NT2RP3000759, NT2RP3000826, NT2RP3001007, NT2RP3001096, NT2RP3001126, NT2RP3001355, NT2RP3001396, NT2RP3001449, NT2RP3001490, NT2RP3001679, NT2RP3001727, NT2RP3001752, NT2RP3001777, NT2RP3001782, NT2RP3001799, NT2RP3001854, NT2RP3001989, NT2RP3002142, NT2RP3002248, NT2RP3002343, NT2RP3002484, NT2RP3002529, NT2RP3002549, NT2RP3002628, NT2RP3002687, NT2RP3002688, NT2RP3002810, NT2RP3003032, NT2RP3003139, NT2RP3003193, NT2RP3003203, NT2RP3003204, NT2RP3003210, NT2RP3003212, NT2RP3003264, NT2RP3003282, NT2RP3003500, NT2RP3004041, NT2RP3004215, NT2RP4000147, NT2RP4000259, NT2RP4000360, NT2RP4000448, NT2RP4000524, NT2RP4000588, NT2RP4000879, NT2RP4000907, NT2RP4000989, NT2RP4001079, NT2RP4001150, NT2RP4001219, NT2RP4001260, NT2RP4001274, NT2RP4001353, NT2RP4001547, NT2RP4001677, NT2RP4002052, OVARC1000006, OVARC1000092, OVARC1000321, OVARC1000384, OVARC1000408, OVARC1000414, OVARC1000520, OVARC1000526, OVARC1000588, OVARC1000679, OVARC1000682, OVARC1000769, OVARC1000850, OVARC1000862, OVARC1000886, OVARC1000984, OVARC1001000, OVARC1001004, OVARC1001154, OVARC1001170, OVARC1001173, OVARC1001200, OVARC1001268, OVARC1001376, OVARC1001419, OVARC1001425, OVARC1001476, OVARC1001480, OVARC1001542, OVARC1001873, OVARC1001928, OVARC1001987, OVARC1002066, OVARC1002082, OVARC1002112, OVARC1002127,
PLACE1000014, PLACE1000048, PLACE1000184, PLACE1000185, PLACE1000246, PLACE1000292, PLACE1000332, PLACE1000347, PLACE1000564, PLACE1000656, PLACE1000712, PLACE1001000, PLACE1001168, PLACE1001185, PLACE1001241, PLACE1001294, PLACE1001311, PLACE1001395, PLACE1001570, PLACE1001608, PLACE1001610, PLACE1001716, PLACE1001746, PLACE1001817, PLACE1001821, PLACE1001844, PLACE1001897, PLACE1002066, PLACE1002119, PLACE1002157, PLACE1002205, PLACE1002256, PLACE1002259, PLACE1002399, PLACE1002438, PLACE1002474, PLACE1002477, PLACE1002500, PLACE1002514, PLACE1002578, PLACE1002815, PLACE1002851, PLACE1002968, PLACE1003108, PLACE1003174, PLACE1003200, PLACE1003238, PLACE1003256, PLACE1003334, PLACE1003342, PLACE1003516, PLACE1003521, PLACE1003537, PLACE1003592, PLACE1003596, PLACE1003723, PLACE1003760, PLACE1003771, PLACE1003783, PLACE1003795, PLACE1003892, PLACE1003968, PLACE1004103, PLACE1004256, PLACE1004405, PLACE1004460, PLACE1004506, PLACE1004629, PLACE1004674, PLACE1004813, PLACE1004979, PLACE1005066, PLACE1005101, PLACE1005102, PLACE1005128, PLACE1005181, PLACE1005287, PLACE1005305, PLACE1005327, PLACE1005477, PLACE1005595, PLACE1005603, PLACE1005666, PLACE1005804, PLACE1005884, PLACE1005934, PLACE1006076, PLACE1006119, PLACE1006159, PLACE1006164, PLACE1006170, PLACE1006382, PLACE1006492, PLACE1006629, PLACE1006704, PLACE1006731, PLACE1006760, PLACE1006779, PLACE1006795, PLACE1006805, PLACE1006962, PLACE1007045, PLACE1007111, PLACE1007282, PLACE1007386, PLACE1007416, PLACE1007484, PLACE1007544, PLACE1007645, PLACE1007743, PLACE1007746, PLACE1007807, PLACE1007858, PLACE1008002, PLACE1008181, PLACE1008273, PLACE1008368, PLACE1008405, PLACE1008532, PLACE1008568, PLACE1008625, PLACE1008696, PLACE1008867, PLACE1009027, PLACE1009039, PLACE1009045, PLACE1009110, PLACE1009298, PLACE1009328, PLACE1009581, PLACE1009621, PLACE1009622, PLACE1009637, PLACE1009925, PLACE1009935, PLACE1010089, PLACE1010106, PLACE1010152, PLACE1010274, PLACE1010491, PLACE1010629, PLACE1010630, PLACE1010714, PLACE1010739, PLACE1010891, PLACE1010896, PLACE1010925, PLACE1010965, PLACE1011026, PLACE1011046, PLACE1011214, PLACE1011399, PLACE1011433, PLACE1011492, PLACE1011641, PLACE1011649, PLACE1011719, PLACE1011762, PLACE1011858, PLACE1011923, PLACE2000014, PLACE2000039, PLACE2000216, PLACE2000302, PLACE2000317, PLACE2000342, PLACE2000347, PLACE2000379, PLACE3000121, PLACE3000124, PLACE3000160, PLACE3000242, PLACE3000271, PLACE3000353, PLACE3000362, PLACE3000365, PLACE3000400, PLACE3000401, PLACE4000034, PLACE4000089, PLACE4000522, PLACE4000558,
SKNMC1000050, THYRO1000040, THYRO1000197, THYRO1000241, THYRO1000327, THYRO1000394, THYRO1000488, THYRO1000501, THYRO1000585, THYRO1000596, THYRO1000625, THYRO1000805, THYRO1000934, THYRO1001133, THYRO1001134, THYRO1001173, THYRO1001213, THYRO1001262, THYRO1001290, THYRO1001721, Y79AA1000037, Y79AA1000800, Y79AA1000976, Y79AA1001078, Y79AA1001228, Y79AA1001299, Y79AA1001402, Y79AA1001585, Y79AA1001696, Y79AA1001711,
Y79AA1001827, Y79AA1001875, Y79AA1002027, Y79AA1002211, Y79AA1002234,
Y79AA1002258.

Clones of which expression levels decrease by RA/inhibitor are as follows:
HEMBA1000012, HEMBA1000501, HEMBA1000946, HEMBA1003220, HEMBA1003403, HEMBA1003569, HEMBA1003591, HEMBA1003926, HEMBA1004168, HEMBA1004507, HEMBA1005009, HEMBA1005296, HEMBA1005528, HEMBA1005570, HEMBA1006467, HEMBA1006486, HEMBA1006492, HEMBA1007322, HEMBB1000055, HEMBB1000244, HEMBB1001665, MAMMA1000684, MAMMA1001139, MAMMA1001743, NT2RM1000257, NT2RM1000318, NT2RM1000539, NT2RM1000666, NT2RM2000092, NT2RM2000192, NT2RM2000371, NT2RM2000594, NT2RM4000511, NT2RM4001140, NT2RM4001754, NT2RM4001905, NT2RM4001940, NT2RM4002593, NT2RP1000086, NT2RP1000439, NT2RP1001073, NT2RP2000098, NT2RP2000965, NT2RP2001397, NT2RP2002047, NT2RP2004226, NT2RP2004396, NT2RP2004655, NT2RP2005126, NT2RP2005464, NT2RP2005712, NT2RP2005859, NT2RP2005890, NT2RP3000980, NT2RP3001383, NT2RP3001621, NT2RP3002081, NT2RP3002181, NT2RP3002244, NT2RP3002590, NT2RP3003059, NT2RP3004258, NT2RP3004378, NT2RP3004527, NT2RP3004594, NT2RP4001760, NT2RP4001950, NT2RP4002047, NT2RP4002408, NT2RP5003459, OVARC1000004, OVARC1000035, OVARC1000431, OVARC1001051, OVARC1001129, OVARC1001176, OVARC1001261, OVARC1001342, OVARC1001942, OVARC1001943, PLACE1002171, PLACE1002465, PLACE1003190, PLACE1003375, PLACE1004128, PLACE1005026, PLACE1005876, PLACE1005923, PLACE1007257, PLACE1007375, PLACE1007507, PLACE1008941, PLACE1010624, PLACE1011090, PLACE1011219, THYRO1000270, Y79AA1000346, Y79AA1001541.

Clones of which expression levels increase in the presence of both RA and RA/inhibitor are as follows:
HEMBA1000046, HEMBA1000307, HEMBA1000504, HEMBA1000588, HEMBA1000682, HEMBA1000726, HEMBA1000943, HEMBA1001071, HEMBA1001094, HEMBA1001122, HEMBA1001323, HEMBA1001361, HEMBA1001455, HEMBA1001869, HEMBA1002084, HEMBA1002583, HEMBA1002628, HEMBA1003096, HEMBA1003142, HEMBA1003276, HEMBA1003309, HEMBA1003463, HEMBA1003597, HEMBA1003617, HEMBA1003725, HEMBA1003803, HEMBA1003879, HEMBA1003989, HEMBA1004000, HEMBA1004015, HEMBA1004024, HEMBA1004049, HEMBA1004056, HEMBA1004199, HEMBA1004248, HEMBA1004356, HEMBA1004666, HEMBA1004770, HEMBA1004803, HEMBA1004923, HEMBA1004934, HEMBA1004954, HEMBA1005039, HEMBA1005075, HEMBA1005113, HEMBA1005219, HEMBA1005232, HEMBA1005251, HEMBA1005304, HEMBA1005367, HEMBA1005410, HEMBA1005411, HEMBA1005548, HEMBA1005581, HEMBA1005631, HEMBA1005666, HEMBA1005780, HEMBA1006485, HEMBA1006559, HEMBA1006579, HEMBA1006754, HEMBB1000059, HEMBB1000575, HEMBB1000709, HEMBB1000848, HEMBB1000985, HEMBB1001117, HEMBB1001210, HEMBB1001394, HEMBB1001668, HEMBB1001695, HEMBB1002049, HEMBB1002254, HEMBB1002266, HEMBB1002371, HEMBB1002614, HEMBB1002617, HEMBB1002697, MAMMA1000241, MAMMA1000424, MAMMA1000616, MAMMA1000731, MAMMA1000824, MAMMA1001038, MAMMA1001243, MAMMA1002769, MAMMA1002871, MAMMA1002941,
NT2RM1000725, NT2RM1000829, NT2RM2000504, NT2RM2000635, NT2RM2000821, NT2RM2001582, NT2RM2001592, NT2RM2001632, NT2RM2001648, NT2RM2001671, NT2RM2001695, NT2RM2001879, NT2RM4000290, NT2RM4000425, NT2RM4000471, NT2RM4000531, NT2RM4000852, NT2RM4001047, NT2RM4001454, NT2RM4001557, NT2RM4001566, NT2RM4001582, NT2RM4001953, NT2RM4002409, NT2RM4002558, NT2RM4002594, NT2RP1000259, NT2RP1000418, NT2RP1000574, NT2RP1000782, NT2RP1000856, NT2RP1000943, NT2RP1000988, NT2RP1001013, NT2RP1001173, NT2RP1001546, NT2RP2000091, NT2RP2000208, NT2RP2000274, NT2RP2000329, NT2RP2000369, NT2RP2000634, NT2RP2000987, NT2RP2001277, NT2RP2001290, NT2RP2001366, NT2RP2001423, NT2RP2001436, NT2RP2001467, NT2RP2001506, NT2RP2001601, NT2RP2001926, NT2RP2001985, NT2RP2002041, NT2RP2002046, NT2RP2002078, NT2RP2002124, NT2RP2002193, NT2RP2002312, NT2RP2002316, NT2RP2002426, NT2RP2002457, NT2RP2002475, NT2RP2002520, NT2RP2002595, NT2RP2002672, NT2RP2002701, NT2RP2003099, NT2RP2003137, NT2RP2003206, NT2RP2003230, NT2RP2003272, NT2RP2003280, NT2RP2003393, NT2RP2003445, NT2RP2003456, NT2RP2003596, NT2RP2003871, NT2RP2004681, NT2RP2004743, NT2RP2004775, NT2RP2004933, NT2RP2004967, NT2RP2005003, NT2RP2005289, NT2RP2005453, NT2RP2005555, NT2RP2005767, NT2RP2005853, NT2RP2006043, NT2RP2006393, NT2RP2006436, NT2RP2006441, NT2RP2006467, NT2RP2006565, NT2RP3000359, NT2RP3000366, NT2RP3000403, NT2RP3000418, NT2RP3000441, NT2RP3000561, NT2RP3000759, NT2RP3001007, NT2RP3001126, NT2RP3001355, NT2RP3001396, NT2RP3001449, NT2RP3001490, NT2RP3001679, NT2RP3001752, NT2RP3001782, NT2RP3001799, NT2RP3001989, NT2RP3002142, NT2RP3002248, NT2RP3002343, NT2RP3002484, NT2RP3002529, NT2RP3002549, NT2RP3002687, NT2RP3003032, NT2RP3003139, NT2RP3003193, NT2RP3003204, NT2RP3003210, NT2RP3003212, NT2RP3003264, NT2RP3003282, NT2RP3003500, NT2RP3004041, NT2RP3004215, NT2RP4000147, NT2RP4000259, NT2RP4000360, NT2RP4000448, NT2RP4000524, NT2RP4001079, NT2RP4001150, NT2RP4001274, NT2RP4001353, NT2RP4001547, NT2RP4001677, OVARC1000092, OVARC1000321, OVARC1000384, OVARC1000408, OVARC1000414, OVARC1000520, OVARC1000526, OVARC1000588, OVARC1000679, OVARC1000682, OVARC1000769, OVARC1000862, OVARC1000984, OVARC1001000, OVARC1001004, OVARC1001154, OVARC1001170, OVARC1001173, OVARC1001200, OVARC1001268, OVARC1001376, OVARC1001476, OVARC1001542, OVARC1001873, OVARC1001987, OVARC1002082,
PLACE1000014, PLACE1000048, PLACE1000184, PLACE1000246, PLACE1000292, PLACE1000332, PLACE1000347, PLACE1000712, PLACE1001000, PLACE1001294, PLACE1001311, PLACE1001395, PLACE1001570, PLACE1001608, PLACE1001610, PLACE1001746, PLACE1001821, PLACE1001844, PLACE1001897, PLACE1002066, PLACE1002119, PLACE1002157, PLACE1002205, PLACE1002256, PLACE1002259, PLACE1002438, PLACE1002474, PLACE1002477, PLACE1002500, PLACE1002578, PLACE1002815, PLACE1002851, PLACE1002968, PLACE1003108, PLACE1003174, PLACE1003200, PLACE1003256, PLACE1003334, PLACE1003516, PLACE1003592, PLACE1003723, PLACE1003760, PLACE1003771, PLACE1003795, PLACE1003892, PLACE1003968, PLACE1004103, PLACE1004256, PLACE1004629, PLACE1004979, PLACE1005102, PLACE1005128, PLACE1005305, PLACE1005477, PLACE1005666, PLACE1005804, PLACE1005934, PLACE1006076, PLACE1006119, PLACE1006159, PLACE1006164, PLACE1006170, PLACE1006492, PLACE1006629, PLACE1006704, PLACE1006760, PLACE1006795, PLACE1006962, PLACE1007045, PLACE1007386, PLACE1007416, PLACE1007484, PLACE1007544, PLACE1007645, PLACE1007743, PLACE1007807, PLACE1007858, PLACE1008002, PLACE1008273, PLACE1008368, PLACE1008532, PLACE1008568, PLACE1008696, PLACE1008867, PLACE1009027, PLACE1009039, PLACE1009298, PLACE1009328, PLACE1009621, PLACE1009637, PLACE1010089, PLACE1010106, PLACE1010152, PLACE1010491, PLACE1010630, PLACE1010739, PLACE1010896, PLACE1010925, PLACE1010965, PLACE1011046, PLACE1011214, PLACE1011433, PLACE1011719, PLACE1011762, PLACE2000039, PLACE2000216, PLACE2000302, PLACE2000342, PLACE2000347, PLACE2000379, PLACE3000121, PLACE3000124, PLACE3000242, PLACE3000271, PLACE3000362, PLACE3000365, PLACE3000400, PLACE3000401, PLACE4000034, PLACE4000089, PLACE4000522, PLACE4000558, THYRO1000197, THYRO1000241, THYRO1000327, THYRO1000394, THYRO1000488, THYRO1000585, THYRO1000625, THYRO1000805, THYRO1000934, THYRO1001133, THYRO1001173, THYRO1001213, THYRO1001262, THYRO1001290, Y79AA1000800, Y79AA1000976, Y79AA1001585, Y79AA1001696, Y79AA1001711, Y79AA1002258.

Clones of which expression levels decrease in the presence of both RA and RA/inhibitor are as follows:HEMBA1000946, HEMBA1003569, HEMBA1005570, NT2RM1000666, NT2RM2000092, NT2RM2000594, NT2RM4001754, NT2RM4001905, NT2RP2002047, NT2RP3000980, NT2RP3002081, NT2RP3004594, NT2RP4001950, NT2RP4002408, OVARC1000431, OVARC1001942, OVARC1001943, PLACE1003190, PLACE1005923, PLACE1007257, PLACE1010624, Y79AA1000346.

These are neurological disease-associated clones.

### Analysis of rheumatoid arthritis-associated genes

The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center, http://www.rheuma-net.or.jp/). Recent studies have also revealed that tissue necrosis factor (TNF)-α participates in the onset (Current opinion in immunology 1999, 11, 657-662). When the expression of a gene exhibits responsiveness to the action of TNF on synovial cells, the gene is considered to be involved in rheumatoid arthritis.

A survey was performed for genes of which expression levels are varied in response to TNF-α in the primary cell culture of synovial tissue. The primary cultured cells of the smooth muscle (Cell Applications) were grown to be confluent in a culture dish, and then, human TNF-α (Boehringer-Mannheim) was added at a final concentration of 10 ng/ml thereto. The culture was further continued for 24 hours.

Total RNA was extracted from the cells by using S.N.A.P.^{(TM)} Total RNA Isolation kit (Invitrogen). The labeling of probe used for hybridization was carried out by using 10 µg of the total RNA according to the same methods as described above. The data were obtained in triplicate (n=3). The data of signal value representing gene expression level in the cells in the presence of TNF stimulation were compared with those in the absence of the stimulation. The comparison was performed by statistical treatment of two-sample t-test. Clones with significant difference in the signal distribution were selected under the condition of p<0.05. In this analysis,
clones with the difference can be statistically detected even when the signals were low.
Accordingly, clones with signal value of 40 or less were also assessed for the selection.
Table 352 shows the expression level of each cDNA in synovial cells cultured in the absence or presence of TNF.

Averaged signal values (M₁, M₂) and sample variances (s₁², s₂²) for each gene were calculated in each of the cells, and then, the pooled sample variances s² were obtained from the sample variances of the two types of cells to be compared. The t-values were determined according to the following formula: t=(M₁-M₂)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively.
The tables also include the information of an increase (+) or decrease (-) in the expression level of a gene in the stimulated cells when the level is compared with that of unstimulated cells.

Clones of which expression levels are elevated by TNF-α are as follows:
HEMBA1000005, HEMBA1000012, HEMBA1000020, HEMBA1000046, HEMBA1000076, HEMBA1000111, HEMBA1000168, HEMBA1000185, HEMBA1000201, HEMBA1000231, HEMBA1000243, HEMBA1000280, HEMBA1000282, HEMBA1000304, HEMBA1000307, HEMBA1000327, HEMBA1000356, HEMBA1000376, HEMBA1000387, HEMBA1000390, HEMBA1000418, HEMBA1000460, HEMBA1000491, HEMBA1000501, HEMBA1000518, HEMBA1000519, HEMBA1000520, HEMBA1000531, HEMBA1000534, HEMBA1000542, HEMBA1000545, HEMBA1000591, HEMBA1000592, HEMBA1000594, HEMBA1000636, HEMBA1000655, HEMBA1000657, HEMBA1000673, HEMBA1000682, HEMBA1000686, HEMBA1000722, HEMBA1000726, HEMBA1000827, HEMBA1000870, HEMBA1000918, HEMBA1000971, HEMBA1000974, HEMBA1000986, HEMBA1001019, HEMBA1001043, HEMBA1001051, HEMBA1001059, HEMBA1001060, HEMBA1001071, HEMBA1001080, HEMBA1001109, HEMBA1001140, HEMBA1001172, HEMBA1001196, HEMBA1001213, HEMBA1001226, HEMBA1001281, HEMBA1001299, HEMBA1001302, HEMBA1001303, HEMBA1001323, HEMBA1001326, HEMBA1001327, HEMBA1001330, HEMBA1001351, HEMBA1001407, HEMBA1001411, HEMBA1001446, HEMBA1001454, HEMBA1001569, HEMBA1001647, HEMBA1001714, HEMBA1001800, HEMBA1001804, HEMBA1001809, HEMBA1001888, HEMBA1001912, HEMBA1001921, HEMBA1001967, HEMBA1002084, HEMBA1002161, HEMBA1002166, HEMBA1002241, HEMBA1002337, HEMBA1002363, HEMBA1002389, HEMBA1002458, HEMBA1002460, HEMBA1002469, HEMBA1002538, HEMBA1002542, HEMBA1002547, HEMBA1002609, HEMBA1002624, HEMBA1002659, HEMBA1002750, HEMBA1002770, HEMBA1002779, HEMBA1002810, HEMBA1002816, HEMBA1002818, HEMBA1002850, HEMBA1002863, HEMBA1003021, HEMBA1003033, HEMBA1003078, HEMBA1003166, HEMBA1003202, HEMBA1003204, HEMBA1003229, HEMBA1003235, HEMBA1003276, HEMBA1003286, HEMBA1003296, HEMBA1003370, HEMBA1003376, HEMBA1003403, HEMBA1003418, HEMBA1003433, HEMBA1003447, HEMBA1003560, HEMBA1003569, HEMBA1003571, HEMBA1003591, HEMBA1003597, HEMBA1003598, HEMBA1003621, HEMBA1003656, HEMBA1003662, HEMBA1003680, HEMBA1003715, HEMBA1003725, HEMBA1003729, HEMBA1003733, HEMBA1003742, HEMBA1003773, HEMBA1003783, HEMBA1003950, HEMBA1004012, HEMBA1004015, HEMBA1004048, HEMBA1004074, HEMBA1004086, HEMBA1004111, HEMBA1004131, HEMBA1004202, HEMBA1004203, HEMBA1004207, HEMBA1004248, HEMBA1004274, HEMBA1004321, HEMBA1004330, HEMBA1004356, HEMBA1004366, HEMBA1004405, HEMBA1004408, HEMBA1004429, HEMBA1004499, HEMBA1004507, HEMBA1004509, HEMBA1004542, HEMBA1004596, HEMBA1004604, HEMBA1004776, HEMBA1004889, HEMBA1004934, HEMBA1004978, HEMBA1005019, HEMBA1005047, HEMBA1005206, HEMBA1005219, HEMBA1005274, HEMBA1005331, HEMBA1005338, HEMBA1005394, HEMBA1005423, HEMBA1005576, HEMBA1005732, HEMBA1005746, HEMBA1006091, HEMBA1006142, HEMBA1006173, HEMBA1006198, HEMBA1006253, HEMBA1006268, HEMBA1006309, HEMBA1006377, HEMBA1006474, HEMBA1006486, HEMBA1006492, HEMBA1006502, HEMBA1006535, HEMBA1006579, HEMBA1006648, HEMBA1006659, HEMBA1006885, HEMBA1006929, HEMBA1006941, HEMBA1007078, HEMBA1007080, HEMBA1007121, HEMBA1007194, HEMBA1007300, HEMBA1007301, HEMBA1007322, HEMBB1000036, HEMBB1000044, HEMBB1000089, HEMBB1000215, HEMBB1000217, HEMBB1000272, HEMBB1000420, HEMBB1000591, HEMBB1000593, HEMBB1000631, HEMBB1000835, HEMBB1000887, HEMBB1000908, HEMBB1000975, HEMBB1000985, HEMBB1001011, HEMBB1001014, HEMBB1001112, HEMBB1001133, HEMBB1001331, HEMBB1001337, HEMBB1001366, HEMBB1001367, HEMBB1001384, HEMBB1001394, HEMBB1001429, HEMBB1001463, HEMBB1001619, HEMBB1001684, HEMBB1001706, HEMBB1001753, HEMBB1001797, HEMBB1001802, HEMBB1001812, HEMBB1001874, HEMBB1001910, HEMBB1001915, HEMBB1001973, HEMBB1001983, HEMBB1001990, HEMBB1002190, HEMBB1002193, HEMBB1002249, HEMBB1002329, HEMBB1002342, HEMBB1002371, HEMBB1002409, HEMBB1002442, HEMBB1002489, HEMBB1002492, HEMBB1002520, HEMBB1002534, HEMBB1002596, HEMBB1002664, HEMBB1002692, HEMBB1002697, HEMBB1002705, MAMMA1000092, MAMMA1000155, MAMMA1000163, MAMMA1000173, MAMMA1000175, MAMMA1000227, MAMMA1000241, MAMMA1000257, MAMMA1000264, MAMMA1000266, MAMMA1000270, MAMMA1000307, MAMMA1000410, MAMMA1000413, MAMMA1000416, MAMMA1000421, MAMMA1000472, MAMMA1000501, MAMMA1000605, MAMMA1000643, MAMMA1000670, MAMMA1000684, MAMMA1000696, MAMMA1000732, MAMMA1000752, MAMMA1000802, MAMMA1000824, MAMMA1000905, MAMMA1000921, MAMMA1000931, MAMMA1000957, MAMMA1000962, MAMMA1000998, MAMMA1001008, MAMMA1001050, MAMMA1001074, MAMMA1001078, MAMMA1001292, MAMMA1001397, MAMMA1001476, MAMMA1001743, MAMMA1001744, MAMMA1001754, MAMMA1001760, MAMMA1001785, MAMMA1001858, MAMMA1001908, MAMMA1002236, MAMMA1002267, MAMMA1002292, MAMMA1002311, MAMMA1002322, MAMMA1002359, MAMMA1002362, MAMMA1002485, MAMMA1002494, MAMMA1002597, MAMMA1002598, MAMMA1002665, MAMMA1002671, MAMMA1002684, MAMMA1002748, MAMMA1002775, MAMMA1002830, MAMMA1002858, MAMMA1002868, MAMMA1002886, MAMMA1002887, MAMMA1002892, MAMMA1002909, MAMMA1002937, MAMMA1002947, MAMMA1002964, MAMMA1002970, MAMMA1003013, MAMMA1003150, NT2RM1000039, NT2RM1000062, NT2RM1000080, NT2RM1000086, NT2RM1000127, NT2RM1000132, NT2RM1000187, NT2RM1000199, NT2RM1000244, NT2RM1000256, NT2RM1000272, NT2RM1000318, NT2RM1000354, NT2RM1000377, NT2RM1000430, NT2RM1000499, NT2RM1000539, NT2RM1000553, NT2RM1000563, NT2RM1000699, NT2RM1000742, NT2RM1000826, NT2RM1000829, NT2RM1000833, NT2RM1000882, NT2RM1000898, NT2RM1000905, NT2RM1001092, NT2RM2000013, NT2RM2000032, NT2RM2000042, NT2RM2000101, NT2RM2000124, NT2RM2000192, NT2RM2000259, NT2RM2000260, NT2RM2000363, NT2RM2000368, NT2RM2000402, NT2RM2000452, NT2RM2000952, NT2RM2001221, NT2RM2002014, NT2RM2002030, NT2RM4000156, NT2RM4000349, NT2RM4000395, NT2RM4000457, NT2RM4000511, NT2RM4000514, NT2RM4000698, NT2RM4000764, NT2RM4001016, NT2RM4001084, NT2RM4001594, NT2RM4001629, NT2RM4001662, NT2RM4001841, NT2RM4002093, NT2RM4002109, NT2RM4002145, NT2RM4002189, NT2RM4002194, NT2RM4002226, NT2RP1000170, NT2RP1000439, NT2RP1000478, NT2RP1000513, NT2RP1000701, NT2RP1000856, NT2RP1001361, NT2RP2000097, NT2RP2000239, NT2RP2000288, NT2RP2000328, NT2RP2000329, NT2RP2000369, NT2RP2000422, NT2RP2000842, NT2RP2000965, NT2RP2001245, NT2RP2001440, NT2RP2001560, NT2RP2001634, NT2RP2001663, NT2RP2001677, NT2RP2001762, NT2RP2002270, NT2RP2002312, NT2RP2002316, NT2RP2002333, NT2RP2002706, NT2RP2002925, NT2RP2002959, NT2RP2002987, NT2RP2003125, NT2RP2003137, NT2RP2003237, NT2RP2003272, NT2RP2003596, NT2RP2003604, NT2RP2003643, NT2RP2003968, NT2RP2003976, NT2RP2004194, NT2RP2004321, NT2RP2005037, NT2RP2005140, NT2RP2005204, NT2RP2005293, NT2RP2005457, NT2RP2005555, NT2RP2005600, NT2RP2005701, NT2RP2005719, NT2RP2005722, NT2RP2005773, NT2RP2005890, NT2RP2006023, NT2RP2006071, NT2RP3000186, NT2RP3000341, NT2RP3000599, NT2RP3000632, NT2RP3000644, NT2RP3000852, NT2RP3000968, NT2RP3001096, NT2RP3001109, NT2RP3001126, NT2RP3001147, NT2RP3001449, NT2RP3001529, NT2RP3001753, NT2RP3001854, NT2RP3001915, NT2RP3001969, NT2RP3002081, NT2RP3002142, NT2RP3002399, NT2RP3002590, NT2RP3002603, NT2RP3002810, NT2RP3002876, NT2RP3003311, NT2RP3003330, NT2RP3003672, NT2RP3004209, NT2RP3004378, NT2RP4000078, NT2RP4000541, NT2RP4000588, NT2RP4001219, NT2RP4001228, NT2RP4001276, NT2RP4001507, NT2RP4002047, NT2RP5003459, NT2RP5003492, OVARC1000085, OVARC1000087, OVARC1000106, OVARC1000151, OVARC1000198, OVARC1000431, OVARC1000440, OVARC1000564, OVARC1000605, OVARC1000679, OVARC1000883, OVARC1000912, OVARC1000960, OVARC1000971, OVARC1001038, OVARC1001055, OVARC1001085, OVARC1001129, OVARC1001167, OVARC1001339, OVARC1001425, OVARC1001745, OVARC1001762, OVARC1001766, OVARC1001942, OVARC1002044, OVARC1002138, PLACE1000004, PLACE1000005, PLACE1000420, PLACE1000547, PLACE1000562, PLACE1000653, PLACE1001168, PLACE1001311, PLACE1001377, PLACE1001920, PLACE1001983, PLACE1002066, PLACE1002072, PLACE1002140, PLACE1002171, PLACE1002319, PLACE1002474, PLACE1002499, PLACE1002532, PLACE1002665, PLACE1003025, PLACE1003145, PLACE1003361, PLACE1003605, PLACE1003704, PLACE1003783, PLACE1003885, PLACE1004405, PLACE1004629, PLACE1004686, PLACE1004930, PLACE1005066, PLACE1006077, PLACE1005630, PLACE1005876, PLACE1006143, PLACE1006325, PLACE1006488, PLACE1006805, PLACE1006829, PLACE1007286, PLACE1007858, PLACE1008201, PLACE1009045, PLACE1009113, PLACE1009621, PLACE1010106, PLACE1010310, PLACE1010622, PLACE1010944, PLACE1010965, PLACE1011185, PLACE1011332, PLACE1011635, PLACE1011646, PLACE1011725, PLACE2000014, PLACE2000264, PLACE2000394, PLACE2000419, PLACE3000160, PLACE3000220, PLACE3000254, PLACE3000271, PLACE3000339, PLACE3000341, PLACE3000350, PLACE3000353, PLACE3000401, PLACE4000300, SKNMC1000091, THYRO1000855, THYRO1001559, Y79AA1000065, Y79AA1000202, Y79AA1000214, Y79AA1000346, Y79AA1000784, Y79AA1000833, Y79AA1000968, Y79AA1001555, Y79AA1002220.

Clones of which expression levels decrease by TNF-α are as follows:
HEMBA1002150, HEMBB1000240, NT2RM2000469, NT2RM2000984, NT2RM2001688, _ NT2RM4000290, NT2RM4000496, NT2RM4000590, NT2RM4001047, NT2RM4001582, NT2RM4001611, NT2RM4001650, NT2RM4002075, NT2RM4002128, NT2RP1000174, NT2RP1000243, NT2RP1000581, NT2RP1000688, NT2RP1000767, NT2RP1000825, NT2RP1001185, NT2RP1001286, NT2RP1001432, NT2RP1001457, NT2RP2000001, NT2RP2000248, NT2RP2000841, NT2RP2001813, NT2RP2002137, NT2RP2002928, NT2RP2003517, NT2RP2003559, NT2RP2003564, NT2RP2004933, NT2RP2005038, NT2RP2006365, NT2RP3000072, NT2RP3000320, NT2RP3000484, NT2RP3000980, NT2RP3001111, NT2RP3001420, NT2RP3001495, NT2RP3002056, NT2RP3002057, NT2RP3002545, NT2RP3002713, NT2RP3002799, NT2RP3002869, NT2RP3002953, NT2RP3002955, NT2RP3003282, NT2RP3003290, NT2RP3003384, NT2RP3003385, NT2RP3003870, NT2RP3004207, NT2RP3004262, NT2RP3004527, NT2RP4000500, NT2RP4000524, NT2RP4000787, NT2RP4000927, NT2RP4000955, NT2RP4000989, NT2RP4001442, NT2RP4001638, NT2RP4001950, NT2RP4002888, NT2RP5003524, OVARC1001270, PLACE1000246, PLACE1002816.

These are rheumatoid arthritis-associated clones.

### Analysis of ultraviolet radiation damage-associated genes

It is known that ultraviolet rays give considerably adverse influence on the health. In recent years, there have been significant risks of tissue damage by ultraviolet rays because of destruction of the ozone layer. Thus, ultraviolet radiation has been recognized as a risk factor for skin diseases such as skin cancers (United States Environmental Protection Agency: Ozone Depletion Home Page, http://www.epa.gov/ozone/). Genes of which expression levels are varied in skin epidermal cells exposed to ultraviolet rays are considered to be associated with skin damage caused by ultraviolet radiation.

After primary cultured skin fibroblast cells were irradiated with ultraviolet ray and were cultured, a survey was performed for genes of which expression levels were varied depending on the irradiation of ultraviolet ray. First, after cultured to be confluent, the primary cultured skin fibroblast cells (Cell Applications) were exposed to 10,000 µJ/cm² of 254-nm ultraviolet light.

Messenger RNAs were, then, extracted by using a FastTrack™ 2.0 mRNA Isolation kit (Invitrogen Co.) from the unexposed cells and from the cells that were exposed to the ultraviolet light and then cultured for 4 or 24 hours. The labeling of the hybridization probe was carried out by using a 1.5 µg of each mRNA in the same manner as described above. The data were obtained in triplicate (n=3). The hybridization signals were compared between the cells exposed to the ultraviolet light and the unexposed cells. The comparison was preformed by statistical treatment with two-sample t-test. Clones with significant differences in the signal distribution were selected under the condition of p<0.05. In this analysis, even when the signal is lower than others, the difference in the signal values can be detected statistically. Accordingly, clones with signal value of 40 or lower were also assessed for selection.

Tables 353-509 show the expression of each cDNA in skin-derived fibroblast cells exposed and unexposed to ultraviolet light.

Averaged signal values (M₁, M₂) and sample variances (s₁², s₂²) were calculated for each gene in each of the cells, and then, the pooled sample variances s² were obtained from the sample variances of the two types of cells to be compared. The t values were determined according to the following formula: t=(M₁-M₂)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information of an increase (+) or decrease (-) in the expression level of a gene in the exposed cells in comparison with that of unexposed cells.

The expression levels of the following clones were elevated 4 or 24 hours after the ultraviolet irradiation:
HEMBA1000542, HEMBA1001808, HEMBA1002177, HEMBA1003314, MAMMA1001874, NT2RM2001100, NT2RP2005732, NT2RP3000592, NT2RP4000657, OVARC1000004, OVARC1001092, OVARC1001342, PLACE1002816, NT2RM4001002, NT2RM4001813, NT2RM4002266, NT2RP2001174, NT2RP2001196, NT2RP2005358, NT2RP3000690, NT2RP3001216, NT2RP3003464, PLACE1006382, THYRO1000070, THYRO1001100, Y79AA1000342.

The expression levels of the following clones were decreased 4 or 24 hours after the ultraviolet irradiation:
HEMBA1000005, HEMBA1000150, HEMBA1000156, HEMBA1000158, HEMBA1000168, HEMBA1000231, HEMBA1000304, HEMBA1000307, HEMBA1000333, HEMBA1000366, HEMBA1000369, HEMBA1000390, HEMBA1000396, HEMBA1000418, HEMBA1000434, HEMBA1000464, HEMBA1000469, HEMBA1000490, HEMBA1000504, HEMBA1000505, HEMBA1000557, HEMBA1000657, HEMBA1000673, HEMBA1000682, HEMBA1000686, HEMBA1000727, HEMBA1000752, HEMBA1000851, HEMBA1000852, HEMBA1000870, HEMBA1000872, HEMBA1001085, HEMBA1001121, HEMBA1001133, HEMBA1001235, HEMBA1001265, HEMBA1001281, HEMBA1001289, HEMBA1001299, HEMBA1001303, HEMBA1001310, HEMBA1001323, HEMBA1001595, HEMBA1001620, HEMBA1001640, HEMBA1001678, HEMBA1001712, HEMBA1001835, HEMBA1001950, HEMBA1001987, HEMBA1002253, HEMBA1002321, HEMBA1002341, HEMBA1002419, HEMBA1002679, HEMBA1002728, HEMBA1002818, HEMBA1002935, HEMBA1002999, HEMBA1003034, HEMBA1003071, HEMBA1003098, HEMBA1003142, HEMBA1003175, HEMBA1003202, HEMBA1003212, HEMBA1003220, HEMBA1003276, HEMBA1003373, HEMBA1003417, HEMBA1003447, HEMBA1003528, HEMBA1003684, HEMBA1003799, HEMBA1003885, HEMBA1003989, HEMBA1004011, HEMBA1004055, HEMBA1004133, HEMBA1004225, HEMBA1004272, HEMBA1004353, HEMBA1004631, HEMBA1004669, HEMBA1004705, HEMBA1004753, HEMBA1004776, HEMBA1004803, HEMBA1004816, HEMBA1004900, HEMBA1005047, HEMBA1005079, HEMBA1005101, HEMBA1005149, HEMBA1005152, HEMBA1005202, HEMBA1005314, HEMBA1005372, HEMBA1005511, HEMBA1005513, HEMBA1005518, HEMBA1005570, HEMBA1005577, HEMBA1005581, HEMBA1005588, HEMBA1005609, HEMBA1005632, HEMBA1005853, HEMBA1006031, HEMBA1006035, HEMBA1006485, HEMBA1006486, HEMBA1006502, HEMBA1006696, HEMBA1006789, HEMBA1006796, HEMBA1007085, HEMBA1007224, HEMBA1007301, HEMBA1007319, HEMBA1007341, HEMBA1007342, HEMBB1000036, HEMBB1000037, HEMBB1000217, HEMBB1000266, HEMBB1000317, HEMBB1000336, HEMBB1000354, HEMBB1000369, HEMBB1000399, HEMBB1000434, HEMBB1000438, HEMBB1000592, HEMBB1000673, HEMBB1000789, HEMBB1000810, HEMBB1000883, HEMBB1000887, HEMBB1001105, HEMBB1001182, HEMBB1001242, HEMBB1001267, HEMBB1001424, HEMBB1001464, HEMBB1001531, HEMBB1001618, HEMBB1001996, HEMBB1002092, HEMBB1002139, HEMBB1002142, HEMBB1002190, HEMBB1002453, HEMBB1002520, HEMBB1002550, HEMBB1002556, HEMBB1002600, HEMBB1002664, MAMMA1000009, MAMMA1000055, MAMMA1000069, MAMMA1000133, MAMMA1000171, MAMMA1000173, MAMMA1000287, MAMMA1000416, MAMMA1000585, MAMMA1000713, MAMMA1000760, MAMMA1000798, MAMMA1000831, MAMMA1000875, MAMMA1000876, MAMMA1000877, MAMMA1000906, MAMMA1000931, MAMMA1000962, MAMMA1001133, MAMMA1001139, MAMMA1001243, MAMMA1001271, MAMMA1001274, MAMMA1001298, MAMMA1001606, MAMMA1001630, MAMMA1001670, MAMMA1001743, MAMMA1001751, MAMMA1002140, MAMMA1002145, MAMMA1002158, MAMMA1002170, MAMMA1002236, MAMMA1002311, MAMMA1002498, MAMMA1002754, MAMMA1002780, MAMMA1002820, MAMMA1002843, MAMMA1002844, MAMMA1002871, MAMMA1003047, NT2RM1000037, NT2RM1000039, NT2RM1000080, NT2RM1000086, NT2RM1000341, NT2RM1000499, NT2RM1000669, NT2RM1000746, NT2RM1000781, NT2RM1000885, NT2RM1000905, NT2RM1000962, NT2RM2000239, NT2RM2000260, NT2RM2000371, NT2RM2000639, NT2RM2000649, NT2RM2000735, NT2RM2000821, NT2RM2000984, NT2RM2001035, NT2RM2001065, NT2RM2001105, NT2RM2001177, NT2RM2001194, NT2RM2001196, NT2RM2001243, NT2RM2001256, NT2RM2001424, NT2RM2001588, NT2RM2001635, NT2RM2001648, NT2RM2001652, NT2RM2001668, NT2RM2001706, NT2RM2001727, NT2RM2001730, NT2RM2001743, NT2RM2001753, NT2RM2001760, NT2RM2001771, NT2RM2001785, NT2RM2001800, NT2RM2001855, NT2RM2001896, NT2RM2001997, NT2RM2002030, NT2RM2002049, NT2RM2002091, NT2RM2002142, NT2RM2002145, NT2RM2002178, NT2RM2002580, NT2RM4000215, NT2RM4000344, NT2RM4000368, NT2RM4000421, NT2RM4000425, NT2RM4000457, NT2RM4000496, NT2RM4000515, NT2RM4000712, NT2RM4000787, NT2RM4000813, NT2RM4000820, NT2RM4000852, NT2RM4000950, NT2RM4000996, NT2RM4001016, NT2RM4001047, NT2RM4001054, NT2RM4001140, NT2RM4001151, NT2RM4001187, NT2RM4001204, NT2RM4001258, NT2RM4001437, NT2RM4001454, NT2RM4001489, NT2RM4001605, NT2RM4001611, NT2RM4001666, NT2RM4001710, NT2RM4001714, NT2RM4001715, NT2RM4001731, NT2RM4001741, NT2RM4001746, NT2RM4001856, NT2RM4001938, NT2RM4001940, NT2RM4001984, NT2RM4001987, NT2RM4002013, NT2RM4002054, NT2RM4002055, NT2RM4002073, NT2RM4002145, NT2RM4002146, NT2RM4002161, NT2RM4002174, NT2RM4002194, NT2RM4002205, NT2RM4002213, NT2RM4002266, NT2RM4002278, NT2RM4002558, NT2RM4002565, NT2RM4002593, NT2RM4002594, NT2RP1000063, NT2RP1000111, NT2RP1000124, NT2RP1000174, NT2RP1000348, NT2RP1000443, NT2RP1000522, NT2RP1000677, NT2RP1000695, NT2RP1000730, NT2RP1000767, NT2RP1000834, NT2RP1000856, NT2RP1000944, NT2RP1001014, NT2RP1001073, NT2RP1001079, NT2RP1001177, NT2RP1001185, NT2RP1001199, NT2RP1001253, NT2RP1001310, NT2RP1001313, NT2RP1001385, NT2RP1001410, NT2RP1001449, NT2RP1001546, NT2RP2000120, NT2RP2000126, NT2RP2000205, NT2RP2000224, NT2RP2000274, NT2RP2000298, NT2RP2000310, NT2RP2000327, NT2RP2000328, NT2RP2000329, NT2RP2000337, NT2RP2000369, NT2RP2000414, NT2RP2000498, NT2RP2000510, NT2RP2000715, NT2RP2000819, NT2RP2000931, NT2RP2000938, NT2RP2001044, NT2RP2001065, NT2RP2001081, NT2RP2001137, NT2RP2001312, NT2RP2001327, NT2RP2001366, NT2RP2001381, NT2RP2001420, NT2RP2001427, NT2RP2001449, NT2RP2001511, NT2RP2001526, NT2RP2001560, NT2RP2001576, NT2RP2001581, NT2RP2001601, NT2RP2001613, NT2RP2001628, NT2RP2001663, NT2RP2001675, NT2RP2001677, NT2RP2001678, NT2RP2001699, NT2RP2001721, NT2RP2001740, NT2RP2001813, NT2RP2001861, NT2RP2001876, NT2RP2001907, NT2RP2001926, NT2RP2001946, NT2RP2001985, NT2RP2002032, NT2RP2002046, NT2RP2002076, NT2RP2002099, NT2RP2002105, NT2RP2002137, NT2RP2002172, NT2RP2002208, NT2RP2002219, NT2RP2002231, NT2RP2002256, NT2RP2002270, NT2RP2002292, NT2RP2002316, NT2RP2002333, NT2RP2002520, NT2RP2002549, NT2RP2002621, NT2RP2002706, NT2RP2002710, NT2RP2002750, NT2RP2002800, NT2RP2002862, NT2RP2002880, NT2RP2002925, NT2RP2002929, NT2RP2002939, NT2RP2002959, NT2RP2002993, NT2RP2003000, NT2RP2003158, NT2RP2003243, NT2RP2003265, NT2RP2003277, NT2RP2003280, NT2RP2003295, NT2RP2003297, NT2RP2003433, NT2RP2003445, NT2RP2003456, NT2RP2003506, NT2RP2003543, NT2RP2003559, NT2RP2003567, NT2RP2003604, NT2RP2003706, NT2RP2003777, NT2RP2003781, NT2RP2003871, NT2RP2003952, NT2RP2003976, NT2RP2004013, NT2RP2004014, NT2RP2004081, NT2RP2004142, NT2RP2004165, NT2RP2004170, NT2RP2004194, NT2RP2004196, NT2RP2004239, NT2RP2004240, NT2RP2004321, NT2RP2004364, NT2RP2004365, NT2RP2004373, NT2RP2004389, NT2RP2004392, NT2RP2004399, NT2RP2004538, NT2RP2004587, NT2RP2004600, NT2RP2004602, NT2RP2004614, NT2RP2004664, NT2RP2004675, NT2RP2004681, NT2RP2004710, NT2RP2004743, NT2RP2004767, NT2RP2004799, NT2RP2004816, NT2RP2004959, NT2RP2004962, NT2RP2005000, NT2RP2005003, NT2RP2005012, NT2RP2005031, NT2RP2005037, NT2RP2005108, NT2RP2005126, NT2RP2005144, NT2RP2005147, NT2RP2005289, NT2RP2005293, NT2RP2005325, NT2RP2005354, NT2RP2005441, NT2RP2005464, NT2RP2005491, NT2RP2005495, NT2RP2005498, NT2RP2005501, NT2RP2005555, NT2RP2005557, NT2RP2005605, NT2RP2005622, NT2RP2005645, NT2RP2005651, NT2RP2005654, NT2RP2005669, NT2RP2005694, NT2RP2005701, NT2RP2005752, NT2RP2005773, NT2RP2005804, NT2RP2005835, NT2RP2005841, NT2RP2005857, NT2RP2005890, NT2RP2005901, NT2RP2005942, NT2RP2006023, NT2RP2006052, NT2RP2006069, NT2RP2006166, NT2RP2006184, NT2RP2006186, NT2RP2006196, NT2RP2006238, NT2RP2006258, NT2RP2006312, NT2RP2006320, NT2RP2006565, NT2RP2006571, NT2RP3000050, NT2RP3000055, NT2RP3000072, NT2RP3000080, NT2RP3000109, NT2RP3000134, NT2RP3000142, NT2RP3000149, NT2RP3000186, NT2RP3000197, NT2RP3000207, NT2RP3000220, NT2RP3000247, NT2RP3000251, NT2RP3000361, NT2RP3000366, NT2RP3000418, NT2RP3000449, NT2RP3000451, NT2RP3000484, NT2RP3000487, NT2RP3000512, NT2RP3000527, NT2RP3000542, NT2RP3000561, NT2RP3000562, NT2RP3000578, NT2RP3000596, NT2RP3000605, NT2RP3000624, NT2RP3000644, NT2RP3000661, NT2RP3000665, NT2RP3000739, NT2RP3000850, NT2RP3000868, NT2RP3000980, NT2RP3001084, NT2RP3001096, NT2RP3001109, NT2RP3001111, NT2RP3001115, NT2RP3001119, NT2RP3001120, NT2RP3001126, NT2RP3001140, NT2RP3001155, NT2RP3001176, NT2RP3001272, NT2RP3001297, NT2RP3001325, NT2RP3001339, NT2RP3001398, NT2RP3001447, NT2RP3001457, NT2RP3001459, NT2RP3001495, NT2RP3001554, NT2RP3001580, NT2RP3001587, NT2RP3001608, NT2RP3001672, NT2RP3001676, NT2RP3001712, NT2RP3001730, NT2RP3001782, NT2RP3001799, NT2RP3001929, NT2RP3001943, NT2RP3002002, NT2RP3002004, NT2RP3002045, NT2RP3002054, NT2RP3002057, NT2RP3002142, NT2RP3002163, NT2RP3002165, NT2RP3002173, NT2RP3002273, NT2RP3002351, NT2RP3002631, NT2RP3002659, NT2RP3002671, NT2RP3002713, NT2RP3002955, NT2RP3003071, NT2RP3003078, NT2RP3003139, NT2RP3003242, NT2RP3003301, NT2RP3003302, NT2RP3003313, NT2RP3003330, NT2RP3003377, NT2RP3003384, NT2RP3003411, NT2RP3003701, NT2RP3003831, NT2RP3003870, NT2RP3003992, NT2RP3004013, NT2RP3004070, NT2RP3004148, NT2RP3004206, NT2RP3004207, NT2RP3004215, NT2RP3004332, NT2RP3004341, NT2RP3004349, NT2RP3004399, NT2RP4000023, NT2RP4000051, NT2RP4000129, NT2RP4000147, NT2RP4000212, NT2RP4000214, NT2RP4000323, NT2RP4000449, NT2RP4000515, NT2RP4000519, NT2RP4000528, NT2RP4000556, NT2RP4000588, NT2RP4000638, NT2RP4000648, NT2RP4000973, NT2RP4000996, NT2RP4001006, NT2RP4001029, NT2RP4001041, NT2RP4001100, NT2RP4001117, NT2RP4001174, NT2RP4001235, NT2RP4001274, NT2RP4001276, NT2RP4001315, NT2RP4001373, NT2RP4001389, NT2RP4001433, NT2RP4001502, NT2RP4001524, NT2RP4001567, NT2RP4001574, NT2RP4001677, NT2RP4001753, NT2RP4001927, NT2RP4002052, NT2RP4002058, NT2RP4002071, NT2RP4002078, NT2RP4002083, NT2RP5003461, NT2RP5003512, NT2RP5003522, OVARC1000085, OVARC1000091, OVARC1000106, OVARC1000109, OVARC1000114, OVARC1000148, OVARC1000168, OVARC1000198, OVARC1000240, OVARC1000241, OVARC1000288, OVARC1000302, OVARC1000309, OVARC1000326, OVARC1000335, OVARC1000347, OVARC1000384, OVARC1000411, OVARC1000414, OVARC1000420, OVARC1000431, OVARC1000437, OVARC1000440, OVARC1000442, OVARC1000465, OVARC1000473, OVARC1000479, OVARC1000486, OVARC1000526, OVARC1000556, OVARC1000557, OVARC1000564, OVARC1000573, OVARC1000578, OVARC1000588, OVARC1000605, OVARC1000622, OVARC1000678, OVARC1000679, OVARC1000681, OVARC1000703, OVARC1000730, OVARC1000746, OVARC1000769, OVARC1000781, OVARC1000787, OVARC1000800, OVARC1000834, OVARC1000846, OVARC1000850, OVARC1000862, OVARC1000876, OVARC1000883, OVARC1000891, OVARC1000897, OVARC1000912, OVARC1000915, OVARC1000924, OVARC1000937, OVARC1000945, OVARC1000948, OVARC1000959, OVARC1000960, OVARC1000984, OVARC1000996, OVARC1000999, OVARC1001000, OVARC1001010, OVARC1001011, OVARC1001032, OVARC1001034, OVARC1001038, OVARC1001040, OVARC1001044, OVARC1001055, OVARC1001062, OVARC1001085, OVARC1001117, OVARC1001118, OVARC1001162, OVARC1001167, OVARC1001170, OVARC1001173, OVARC1001180, OVARC1001232, OVARC1001240, OVARC1001261, OVARC1001268, OVARC1001270, OVARC1001271, OVARC1001282, OVARC1001296, OVARC1001306, OVARC1001329, OVARC1001330, OVARC1001339, OVARC1001341, OVARC1001344, OVARC1001360, OVARC1001369, OVARC1001376, OVARC1001381, OVARC1001399, OVARC1001417, OVARC1001419, OVARC1001436, OVARC1001496, OVARC1001506, OVARC1001525, OVARC1001542, OVARC1001600, OVARC1001668, OVARC1001702, OVARC1001731, OVARC1001745, OVARC1001762, OVARC1001791, OVARC1001802, OVARC1001812, OVARC1001813, OVARC1001820, OVARC1001861, OVARC1001873, OVARC1001879, OVARC1001880, OVARC1001883, OVARC1001900, OVARC1001911, OVARC1001916, OVARC1001928, OVARC1001942, OVARC1001949, OVARC1001950, OVARC1001987, OVARC1001989, OVARC1002044, OVARC1002050, OVARC1002066, OVARC1002107, OVARC1002112, OVARC1002127, OVARC1002165, PLACE1000004, PLACE1000078, PLACE1000081, PLACE1000142, PLACE1000184, PLACE1000185, PLACE1000246, PLACE1000292, PLACE1000332, PLACE1000383, PLACE1000401, PLACE1000406, PLACE1000420, PLACE1000421, PLACE1000435, PLACE1000453, PLACE1000481, PLACE1000562, PLACE1000583, PLACE1000610, PLACE1000656, PLACE1000785, PLACE1000798, PLACE1000909, PLACE1000948, PLACE1001010, PLACE1001076, PLACE1001092, PLACE1001104, PLACE1001171, PLACE1001185, PLACE1001279, PLACE1001304, PLACE1001311, PLACE1001323, PLACE1001351, PLACE1001366, PLACE1001502, PLACE1001534, PLACE1001602, PLACE1001603, PLACE1001608, PLACE1001610, PLACE1001632, PLACE1001634, PLACE1001720, PLACE1001729, PLACE1001739, PLACE1001745, PLACE1001746, PLACE1001748, PLACE1001781, PLACE1001799, PLACE1001821, PLACE1001912, PLACE1001928, PLACE1001989, PLACE1002072, PLACE1002090, PLACE1002115, PLACE1002150, PLACE1002170, PLACE1002319, PLACE1002342, PLACE1002395, PLACE1002399, PLACE1002433, PLACE1002437, PLACE1002438, PLACE1002450, PLACE1002499, PLACE1002514, PLACE1002583, PLACE1002685, PLACE1002768, PLACE1002772, PLACE1002782, PLACE1002794, PLACE1002811, PLACE1002815, PLACE1002834, PLACE1002839, PLACE1002853, PLACE1002941, PLACE1002968, PLACE1003044, PLACE1003092, PLACE1003100, PLACE1003145, PLACE1003153, PLACE1003176, PLACE1003190, PLACE1003200, PLACE1003205, PLACE1003238, PLACE1003249, PLACE1003302, PLACE1003353, PLACE1003373, PLACE1003383, PLACE1003516, PLACE1003521, PLACE1003592, PLACE1003611, PLACE1003618, PLACE1003704, PLACE1003709, PLACE1003768, PLACE1003784, PLACE1003833, PLACE1003870, PLACE1003886, PLACE1003888, PLACE1003903, PLACE1003936, PLACE1003968, PLACE1004103, PLACE1004114, PLACE1004118, PLACE1004242, PLACE1004256, PLACE1004284, PLACE1004289, PLACE1004316, PLACE1004336, PLACE1004384, PLACE1004388, PLACE1004405, PLACE1004425, PLACE1004428, PLACE1004451, PLACE1004467, PLACE1004491, PLACE1004510, PLACE1004548, PLACE1004658, PLACE1004672, PLACE1004693, PLACE1004716, PLACE1004777, PLACE1004813, PLACE1004824, PLACE1004827, PLACE1004836, PLACE1004840, PLACE1004885, PLACE1004913, PLACE1004918, PLACE1004930, PLACE1004934, PLACE1004972, PLACE1004982, PLACE1004985, PLACE1005026, PLACE1005101, PLACE1005102, PLACE1005128, PLACE1005176, PLACE1005181, PLACE1005187, PLACE1005232, PLACE1005261, PLACE1005277, PLACE1005287, PLACE1005305, PLACE1005327, PLACE1005331, PLACE1005373, PLACE1005374, PLACE1005409, PLACE1005477, PLACE1005480, PLACE1005481, PLACE1005494, PLACE1005502, PLACE1005526, PLACE1005528, PLACE1005530, PLACE1005550, PLACE1005554, PLACE1005557, PLACE1005595, PLACE1005603, PLACE1005611, PLACE1005630, PLACE1005639, PLACE1005646, PLACE1005656, PLACE1005666, PLACE1005730, PLACE1005755, PLACE1005763, PLACE1005799, PLACE1005803, PLACE1005804, PLACE1005834, PLACE1005845, PLACE1005850, PLACE1005876, PLACE1005884, PLACE1005890, PLACE1005898, PLACE1005921, PLACE1005923, PLACE1005932, PLACE1005934, PLACE1005936, PLACE1005951, PLACE1005966, PLACE1005968, PLACE1005990, PLACE1006002, PLACE1006003, PLACE1006011, PLACE1006017, PLACE1006037, PLACE1006040, PLACE1006119, PLACE1006129, PLACE1006139, PLACE1006143, PLACE1006157, PLACE1006159, PLACE1006164, PLACE1006167, PLACE1006170, PLACE1006187, PLACE1006195, PLACE1006205, PLACE1006223, PLACE1006225, PLACE1006236, PLACE1006239, PLACE1006288, PLACE1006318, PLACE1006357, PLACE1006368, PLACE1006371, PLACE1006438, PLACE1006469, PLACE1006482, PLACE1006488, PLACE1006492, PLACE1006506, PLACE1006531, PLACE1006540, PLACE1006552, PLACE1006617, PLACE1006626, PLACE1006673, PLACE1006704, PLACE1006731, PLACE1006754, PLACE1006779, PLACE1006795, PLACE1006800, PLACE1006805, PLACE1006819, PLACE1006829, PLACE1006860, PLACE1006867, PLACE1006878, PLACE1006883, PLACE1006956, PLACE1006958, PLACE1007014, PLACE1007111, PLACE1007140, PLACE1007178, PLACE1007238, PLACE1007239, PLACE1007242, PLACE1007257, PLACE1007274, PLACE1007276, PLACE1007282, PLACE1007286, PLACE1007301, PLACE1007317, PLACE1007346, PLACE1007367, PLACE1007375, PLACE1007386, PLACE1007402, PLACE1007409, PLACE1007416, PLACE1007460, PLACE1007478, PLACE1007484, PLACE1007488, PLACE1007507, PLACE1007511, PLACE1007524, PLACE1007537, PLACE1007544, PLACE1007557, PLACE1007677, PLACE1007688, PLACE1007690, PLACE1007737, PLACE1007743, PLACE1007829, PLACE1007846, PLACE1007852, PLACE1007858, PLACE1007897, PLACE1007908, PLACE1007946, PLACE1007955, PLACE1007958, PLACE1008122, PLACE1008177, PLACE1008181, PLACE1008198, PLACE1008209, PLACE1008244, PLACE1008330, PLACE1008392, PLACE1008405, PLACE1008424, PLACE1008437, PLACE1008455, PLACE1008457, PLACE1008524, PLACE1008531, PLACE1008532, PLACE1008533, PLACE1008568, PLACE1008625, PLACE1008626, PLACE1008627, PLACE1008630, PLACE1008693, PLACE1008715, PLACE1008748, PLACE1008808, PLACE1008813, PLACE1008887, PLACE1008947, PLACE1009045, PLACE1009060, PLACE1009091, PLACE1009099, PLACE1009150, PLACE1009155, PLACE1009183, PLACE1009186, PLACE1009200, PLACE1009308, PLACE1009328, PLACE1009368, PLACE1009398, PLACE1009410, PLACE1009443, PLACE1009444, PLACE1009459, PLACE1009468, PLACE1009476, PLACE1009493, PLACE1009524, PLACE1009581, PLACE1009607, PLACE1009621, PLACE1009721, PLACE1009798, PLACE1009861, PLACE1009879, PLACE1009886, PLACE1009888, PLACE1009947, PLACE1009995, PLACE1010076, PLACE1010083, PLACE1010089, PLACE1010102, PLACE1010105, PLACE1010106, PLACE1010134, PLACE1010194, PLACE1010261, PLACE1010293, PLACE1010310, PLACE1010324, PLACE1010341, PLACE1010364, PLACE1010481, PLACE1010491, PLACE1010547, PLACE1010579, PLACE1010580, PLACE1010599, PLACE1010628, PLACE1010629, PLACE1010662, PLACE1010714, PLACE1010720, PLACE1010739, PLACE1010743, PLACE1010800, PLACE1010811, PLACE1010833, PLACE1010856, PLACE1010870, PLACE1010877, PLACE1010891, PLACE1010896, PLACE1010900, PLACE1010916, PLACE1010917, PLACE1010925, PLACE1010926, PLACE1010942, PLACE1010947, PLACE1010954, PLACE1010960, PLACE1010965, PLACE1011026, PLACE1011041, PLACE1011046, PLACE1011054, PLACE1011090, PLACE1011165, PLACE1011214, PLACE1011229, PLACE1011273, PLACE1011296, PLACE1011310, PLACE1011371, PLACE1011375, PLACE1011399, PLACE1011419, PLACE1011452, PLACE1011465, PLACE1011472, PLACE1011503, PLACE1011563, PLACE1011567, PLACE1011586, PLACE1011641, PLACE1011643, PLACE1011646, PLACE1011650, PLACE1011664, PLACE1011675, PLACE1011725, PLACE1011729, PLACE1011749, PLACE1011762, PLACE1011778, PLACE1011783, PLACE1011858, PLACE1011891, PLACE1011923, PLACE1011962, PLACE1011982, PLACE1011995, PLACE2000006, PLACE2000007, PLACE2000014, PLACE2000017, PLACE2000030, PLACE2000033, PLACE2000039, PLACE2000050, PLACE2000061, PLACE2000100, PLACE2000111, PLACE2000124, PLACE2000132, PLACE2000140, PLACE2000164, PLACE2000170, PLACE2000172, PLACE2000176, PLACE2000187, PLACE2000216, PLACE2000223, PLACE2000264, PLACE2000274, PLACE2000335, PLACE2000341, PLACE2000342, PLACE2000347, PLACE2000366, PLACE2000373, PLACE2000398, PLACE2000419, PLACE2000433, PLACE2000435, PLACE2000455, PLACE2000458, PLACE2000465, PLACE3000020, PLACE3000029, PLACE3000059, PLACE3000103, PLACE3000119, PLACE3000136, PLACE3000156, PLACE3000157, PLACE3000158, PLACE3000160, PLACE3000197, PLACE3000199, PLACE3000207, PLACE3000218, PLACE3000220, PLACE3000221, PLACE3000226, PLACE3000242, PLACE3000244, PLACE3000254, PLACE3000271, PLACE3000276, PLACE3000304, PLACE3000310, PLACE3000320, PLACE3000350, PLACE3000353, PLACE3000363, PLACE3000365, PLACE3000373, PLACE3000388, PLACE3000399, PLACE3000400, PLACE3000455, PLACE4000052, PLACE4000093, PLACE4000128, PLACE4000129, PLACE4000131, PLACE4000147, PLACE4000156, PLACE4000211, PLACE4000222, PLACE4000230, PLACE4000233, PLACE4000261, PLACE4000269, PLACE4000270, PLACE4000320, PLACE4000323, PLACE4000326, PLACE4000369, PLACE4000379, PLACE4000387, PLACE4000392, PLACE4000411, PLACE4000431, PLACE4000487, PLACE4000494, PLACE4000521, PLACE4000522, PLACE4000548, PLACE4000558, PLACE4000581, PLACE4000590, PLACE4000612, SKNMC1000050, SKNMC1000091, THYRO1000040, THYRO1000085, THYRO1000092, THYRO1000111, THYRO1000121, THYRO1000156, THYRO1000163, THYRO1000186, THYRO1000187, THYRO1000206, THYRO1000241, THYRO1000320, THYRO1000343, THYRO1000358, THYRO1000368, THYRO1000381, THYRO1000387, THYRO1000395, THYRO1000401, THYRO1000452, THYRO1000484, THYRO1000501, THYRO1000502, THYRO1000505, THYRO1000662, THYRO1000666, THYRO1000684, THYRO1000748, THYRO1000787, THYRO1000793, THYRO1000815, THYRO1000829, THYRO1000852, THYRO1000855, THYRO1000895, THYRO1000926, THYRO1000951, THYRO1000952, THYRO1000984, THYRO1000988, THYRO1001031, THYRO1001062, THYRO1001093, THYRO1001120, THYRO1001121, THYRO1001133, THYRO1001134, THYRO1001142, THYRO1001173, THYRO1001177, THYRO1001204, THYRO1001213, THYRO1001290, THYRO1001313, THYRO1001321, THYRO1001322, THYRO1001363, THYRO1001374, THYRO1001401, THYRO1001406, THYRO1001458, THYRO1001617, THYRO1001671, THYRO1001673, THYRO1001703, THYRO1001706, THYRO1001721, THYRO1001738, THYRO1001745, THYRO1001746, THYRO1001809, Y79AA1000013, Y79AA1000033, Y79AA1000059, Y79AA1000202, Y79AA1000230, Y79AA1000268, Y79AA1000346, Y79AA1000420, Y79AA1000480, Y79AA1000539, Y79AA1000540, Y79AA1000574, Y79AA1000627, Y79AA1000705, Y79AA1000734, Y79AA1000748, Y79AA1000752, Y79AA1000774, Y79AA1000784, Y79AA1000794, Y79AA1000800, Y79AA1000802, Y79AA1000805, Y79AA1000833, Y79AA1000850, Y79AA1000962, Y79AA1000966, Y79AA1001041, Y79AA1001048, Y79AA1001061, Y79AA1001078, Y79AA1001105, Y79AA1001177, Y79AA1001185, Y79AA1001211, Y79AA1001216, Y79AA1001281, Y79AA1001299, Y79AA1001312, Y79AA1001323, Y79AA1001384, Y79AA1001402, Y79AA1001533, Y79AA1001548, Y79AA1001581, Y79AA1001594, Y79AA1001665, Y79AA1001679, Y79AA1001692, Y79AA1001696, Y79AA1001711, Y79AA1001805, Y79AA1001827, Y79AA1001846, Y79AA1001848, Y79AA1001866, Y79AA1001875, Y79AA1002027, Y79AA1002083, Y79AA1002089, Y79AA1002093, Y79AA1002103, Y79AA1002115, Y79AA1002139, Y79AA1002208, Y79AA1002209, Y79AA1002220, Y79AA1002234, Y79AA1002246, Y79AA1002307, Y79AA1002311, Y79AA1002351, Y79AA1002361, Y79AA1002399, Y79AA1002433, Y79AA1002472, Y79AA1002482, HEMBA1000542, NT2RM2001100, HEMBA1000012, HEMBA1000201, HEMBA1000216, HEMBA1000244, HEMBA1000251, HEMBA1000303, HEMBA1000355, HEMBA1000356, HEMBA1000456, HEMBA1000488, HEMBA1000519, HEMBA1000591, HEMBA1000592, HEMBA1000636, HEMBA1000637, HEMBA1000722, HEMBA1000817, HEMBA1000934, HEMBA1000971, HEMBA1001052, HEMBA1001059, HEMBA1001122, HEMBA1001123, HEMBA1001213, HEMBA1001226, HEMBA1001257, HEMBA1001302, HEMBA1001326, HEMBA1001327, HEMBA1001570, HEMBA1001651, HEMBA1001745, HEMBA1001847, HEMBA1001912, HEMBA1001921, HEMBA1001942, HEMBA1001979, HEMBA1002039, HEMBA1002185, HEMBA1002199, HEMBA1002229, HEMBA1002257, HEMBA1002265, HEMBA1002337, HEMBA1002475, HEMBA1002513, HEMBA1002515, HEMBA1002624, HEMBA1002628, HEMBA1002712, HEMBA1002794, HEMBA1002816, HEMBA1002876, HEMBA1002939, HEMBA1002968, HEMBA1002971, HEMBA1003064, HEMBA1003077, HEMBA1003086, HEMBA1003304, HEMBA1003328, HEMBA1003376, HEMBA1003402, HEMBA1003556, HEMBA1003560, HEMBA1003568, HEMBA1003597, HEMBA1003598, HEMBA1003656, HEMBA1003715, HEMBA1003733, HEMBA1003760, HEMBA1003773, HEMBA1003784, HEMBA1003803, HEMBA1003838, HEMBA1003864, HEMBA1003950, HEMBA1004012, HEMBA1004138, HEMBA1004143, HEMBA1004241, HEMBA1004246, HEMBA1004264, HEMBA1004276, HEMBA1004354, HEMBA1004502, HEMBA1004538, HEMBA1004670, HEMBA1004752, HEMBA1004771, HEMBA1004880, HEMBA1004923, HEMBA1004972, HEMBA1004995, HEMBA1005029, HEMBA1005039, HEMBA1005159, HEMBA1005315, HEMBA1005338, HEMBA1005410, HEMBA1005447, HEMBA1005500, HEMBA1005506, HEMBA1005583, HEMBA1005606, HEMBA1005755, HEMBA1005813, HEMBA1005909, HEMBA1005931, HEMBA1005934, HEMBA1005991, HEMBA1006002, HEMBA1006036, HEMBA1006067, HEMBA1006091, HEMBA1006142, HEMBA1006235, HEMBA1006283, HEMBA1006291, HEMBA1006293, HEMBA1006344, HEMBA1006492, HEMBA1006535, HEMBA1006569, HEMBA1006612, HEMBA1006676, HEMBA1006767, HEMBA1006877, HEMBA1006885, HEMBA1006926, HEMBA1006996, HEMBA1007018, HEMBA1007066, HEMBA1007080, HEMBA1007112, HEMBA1007178, HEMBA1007203, HEMBA1007243, HEMBB1000226, HEMBB1000240, HEMBB1000272, HEMBB1000638, HEMBB1000706, HEMBB1000826, HEMBB1001014, HEMBB1001112, HEMBB1001119, HEMBB1001317, HEMBB1002342, HEMBB1002371, HEMBB1002381, HEMBB1002387, HEMBB1002409, HEMBB1002457, HEMBB1002477, HEMBB1002489, HEMBB1002502, HEMBB1002510, HEMBB1002582, HEMBB1002596, MAMMA1000103, MAMMA1000155, MAMMA1000183, MAMMA1000198, MAMMA1000221, MAMMA1000241, MAMMA1000270, MAMMA1000284, MAMMA1000309, MAMMA1000339, MAMMA1000348, MAMMA1000410, MAMMA1000421, MAMMA1000422, MAMMA1000429, MAMMA1000444, MAMMA1000478, MAMMA1000524, MAMMA1000605, MAMMA1000738, MAMMA1000761, MAMMA1000776, MAMMA1000778, MAMMA1000782, MAMMA1000802, MAMMA1000839, MAMMA1000843, MAMMA1000908, MAMMA1000943, MAMMA1000998, MAMMA1001080, MAMMA1001105, MAMMA1001260, MAMMA1001324, MAMMA1001446, MAMMA1001627, MAMMA1001908, MAMMA1002084, MAMMA1002174, MAMMA1002360, MAMMA1002384, MAMMA1002650, MAMMA1002685, MAMMA1002796, MAMMA1002973, MAMMA1003004, MAMMA1003166, NT2RM1000018, NT2RM1000187, NT2RM1000244, NT2RM1000256, NT2RM1000260, NT2RM1000377, NT2RM1000388, NT2RM1000555, NT2RM1000661, NT2RM1000699, NT2RM1000770, NT2RM1000780, NT2RM1000800, NT2RM1000802, NT2RM1000894, NT2RM1000898, NT2RM1001003, NT2RM1001043, NT2RM1001044, NT2RM1001074, NT2RM1001139, NT2RM2000101, NT2RM2000368, NT2RM2000402, NT2RM2000407, NT2RM2000422, NT2RM2000577, NT2RM2000581, NT2RM2000612, NT2RM2000691, NT2RM2000714, NT2RM2000718, NT2RM2000837, NT2RM2001131, NT2RM2001152, NT2RM2001221, NT2RM2001238, NT2RM2001306, NT2RM2001420, NT2RM2001524, NT2RM2001582, NT2RM2001592, NT2RM2001632, NT2RM2001659, NT2RM2001671, NT2RM2001675, NT2RM2001696, NT2RM2001716, NT2RM2001803, NT2RM2001805, NT2RM2001903, NT2RM2001983, NT2RM4000085, NT2RM4000155, NT2RM4000229, NT2RM4000290, NT2RM4000324, NT2RM4000395, NT2RM4000433, NT2RM4000471, NT2RM4000531, NT2RM4000532, NT2RM4000616, NT2RM4000700, NT2RM4000741, NT2RM4000751, NT2RM4000778, NT2RM4000779, NT2RM4000848, NT2RM4000855, NT2RM4000887, NT2RM4000895, NT2RM4001032, NT2RM4001203, NT2RM4001316, NT2RM4001414, NT2RM4001483, NT2RM4001519, NT2RM4001522, NT2RM4001557, NT2RM4001565, NT2RM4001582, NT2RM4001629, NT2RM4001682, NT2RM4001776, NT2RM4001783, NT2RM4002075, NT2RM4002093, NT2RM4002109, NT2RM4002189, NT2RM4002256, NT2RM4002294, NT2RM4002373, NT2RM4002504, NT2RP1000035, NT2RP1000040, NT2RP1000086, NT2RP1000112, NT2RP1000163, NT2RP1000358, NT2RP1000409, NT2RP1000478, NT2RP1000481, NT2RP1000574, NT2RP1000577, NT2RP1000629, NT2RP1000630, NT2RP1000688, NT2RP1000733, NT2RP1000796, NT2RP1000916, NT2RP1000959, NT2RP1000966, NT2RP1001011, NT2RP1001033, NT2RP1001286, NT2RP1001302, NT2RP1001361, NT2RP1001457, NT2RP1001482, NT2RP2000054, NT2RP2000079, NT2RP2000091, NT2RP2000097, NT2RP2000161, NT2RP2000195, NT2RP2000232, NT2RP2000233, NT2RP2000248, NT2RP2000283, NT2RP2000422, NT2RP2000438, NT2RP2000448, NT2RP2000459, NT2RP2000731, NT2RP2000758, NT2RP2000764, NT2RP2000892, NT2RP2000943, NT2RP2001036, NT2RP2001070, NT2RP2001218, NT2RP2001467, NT2RP2001506, NT2RP2001520, NT2RP2001597, NT2RP2001634, NT2RP2001660, NT2RP2001762, NT2RP2001947, NT2RP2001991, NT2RP2002033, NT2RP2002041, NT2RP2002047, NT2RP2002066, NT2RP2002070, NT2RP2002193, NT2RP2002235, NT2RP2002252, NT2RP2002394, NT2RP2002537, NT2RP2002727, NT2RP2002736, NT2RP2002752, NT2RP2002753, NT2RP2002769, NT2RP2002778, NT2RP2002857, NT2RP2002928, NT2RP2002954, NT2RP2002980, NT2RP2002986, NT2RP2003121, NT2RP2003161, NT2RP2003272, NT2RP2003329, NT2RP2003339, NT2RP2003391, NT2RP2003393, NT2RP2003394, NT2RP2003401, NT2RP2003466, NT2RP2003513, NT2RP2003564, NT2RP2003581, NT2RP2003629, NT2RP2003727, NT2RP2003764, NT2RP2003793, NT2RP2003840, NT2RP2003885, NT2RP2004066, NT2RP2004187, NT2RP2004226, NT2RP2004242,

NT2RP2004245, NT2RP2004316, NT2RP2004366, NT2RP2004396, NT2RP2004463, NT2RP2004512, NT2RP2004655, NT2RP2004736, NT2RP2004775, NT2RP2004791, NT2RP2004802, NT2RP2004841, NT2RP2004897, NT2RP2004933, NT2RP2004936, NT2RP2004961, NT2RP2004967, NT2RP2004982, NT2RP2005018, NT2RP2005038, NT2RP2005116, NT2RP2005140, NT2RP2005159, NT2RP2005162, NT2RP2005276, NT2RP2005288, NT2RP2005315, NT2RP2005344, NT2RP2005407, NT2RP2005453, NT2RP2005457, NT2RP2005476, NT2RP2005490, NT2RP2005525, NT2RP2005540, NT2RP2005549, NT2RP2005600, NT2RP2005675, NT2RP2005690, NT2RP2005712, NT2RP2005722, NT2RP2005723, NT2RP2005763, NT2RP2005812, NT2RP2005853, NT2RP2005859, NT2RP2005933, NT2RP2005980, NT2RP2006038, NT2RP2006043, NT2RP2006071, NT2RP2006098, NT2RP2006100, NT2RP2006103, NT2RP2006106, NT2RP2006261, NT2RP2006334, NT2RP2006464, NT2RP2006573, NT2RP3000255, NT2RP3000267, NT2RP3000341, NT2RP3000403, NT2RP3000526, NT2RP3000531, NT2RP3000584, NT2RP3000590, NT2RP3000685, NT2RP3000759, NT2RP3000847, NT2RP3000865, NT2RP3000875, NT2RP3000968, NT2RP3000994, NT2RP3001107, NT2RP3001116, NT2RP3001147, NT2RP3001150, NT2RP3001236, NT2RP3001307, NT2RP3001318, NT2RP3001399, NT2RP3001420, NT2RP3001426, NT2RP3001472, NT2RP3001497, NT2RP3001527, NT2RP3001607, NT2RP3001671, NT2RP3001690, NT2RP3001727, NT2RP3001915, NT2RP3001931, NT2RP3001944, NT2RP3001989, NT2RP3002147, NT2RP3002244, NT2RP3002330, NT2RP3002343, NT2RP3002501, NT2RP3002587, NT2RP3002602, NT2RP3002682, NT2RP3002687, NT2RP3002770, NT2RP3002785, NT2RP3002869, NT2RP3002877, NT2RP3002969, NT2RP3003145, NT2RP3003157, NT2RP3003212, NT2RP3003264, NT2RP3003278, NT2RP3003282, NT2RP3003290, NT2RP3003327, NT2RP3003344, NT2RP3003353, NT2RP3003491, NT2RP3003543, NT2RP3003665, NT2RP3003918, NT2RP3003932, NT2RP3004110, NT2RP3004155, NT2RP3004262, NT2RP3004334, NT2RP3004428, NT2RP3004466, NT2RP3004475, NT2RP3004504, NT2RP3004507, NT2RP3004527, NT2RP3004544, NT2RP3004572, NT2RP3004578, NT2RP4000109, NT2RP4000246, NT2RP4000360, NT2RP4000376, NT2RP4000398, NT2RP4000455, NT2RP4000518, NT2RP4000524, NT2RP4000878, NT2RP4000879, NT2RP4000955, NT2RP4000989, NT2RP4000997, NT2RP4001004, NT2RP4001010, NT2RP4001078, NT2RP4001122, NT2RP4001126, NT2RP4001149, NT2RP4001206, NT2RP4001219, NT2RP4001260, NT2RP4001339, NT2RP4001353, NT2RP4001414, NT2RP4001442, NT2RP4001447, NT2RP4001474, NT2RP4001551, NT2RP4001803, NT2RP4001893, NT2RP4001896, NT2RP4001946, NT2RP4001950, NT2RP4001966, NT2RP4002081, NT2RP4002408, NT2RP4002791, NT2RP4002888, NT2RP5003524, NT2RP5003534, OVARC1000013, OVARC1000035, OVARC1000087, OVARC1000113, OVARC1000139, OVARC1000145, OVARC1000209, OVARC1000212, OVARC1000408, OVARC1000427, OVARC1000466, OVARC1000496, OVARC1000533, OVARC1000576, OVARC1000640, OVARC1000649, OVARC1000682, OVARC1000689, OVARC1000771, OVARC1000885, OVARC1000890, OVARC1000936, OVARC1000964, OVARC1001065, OVARC1001068, OVARC1001072, OVARC1001107, OVARC1001113, OVARC1001154, OVARC1001169, OVARC1001176, OVARC1001372, OVARC1001391, OVARC1001453, OVARC1001476, OVARC1001480, OVARC1001547, OVARC1001555, OVARC1001610, OVARC1001711, OVARC1001713, OVARC1001726, OVARC1001766, OVARC1001767, OVARC1001768, OVARC1001805, OVARC1001809, OVARC1001828, OVARC1001846, OVARC1001901, OVARC1001943, OVARC1002138, OVARC1002156, PLACE1000005, PLACE1000007; PLACE1000040, PLACE1000048, PLACE1000133, PLACE1000214, PLACE1000347, PLACE1000374, PLACE1000424, PLACE1000492, PLACE1000547, PLACE1000564, PLACE1000599, PLACE1000653, PLACE1000706, PLACE1000748, PLACE1000749, PLACE1000755, PLACE1000841, PLACE1000849, PLACE1000856, PLACE1000931, PLACE1000987, PLACE1001015, PLACE1001024, PLACE1001054, PLACE1001062, PLACE1001118, PLACE1001168, PLACE1001238, PLACE1001377, PLACE1001383, PLACE1001384, PLACE1001399, PLACE1001440, PLACE1001503, PLACE1001545, PLACE1001611, PLACE1001640, PLACE1001691, PLACE1001705, PLACE1001740, PLACE1001817, PLACE1001844, PLACE1001845, PLACE1001869, PLACE1001897, PLACE1002004, PLACE1002073, PLACE1002140, PLACE1002157, PLACE1002171, PLACE1002205, PLACE1002213, PLACE1002474, PLACE1002500, PLACE1002529, PLACE1002537, PLACE1002598, PLACE1002655, PLACE1002908, PLACE1003045, PLACE1003136, PLACE1003174, PLACE1003258, PLACE1003296, PLACE1003334, PLACE1003342, PLACE1003361, PLACE1003366, PLACE1003369, PLACE1003420, PLACE1003454, PLACE1003553, PLACE1003566, PLACE1003583, PLACE1003669, PLACE1003711, PLACE1003723, PLACE1003738, PLACE1003760, PLACE1003762, PLACE1003771, PLACE1003783, PLACE1003850, PLACE1003858, PLACE1003915, PLACE1004128, PLACE1004161, PLACE1004183, PLACE1004197, PLACE1004302, PLACE1004358, PLACE1004437, PLACE1004460, PLACE1004471, PLACE1004506, PLACE1004518, PLACE1004646, PLACE1004722, PLACE1004793, PLACE1004804, PLACE1004838, PLACE1004868, PLACE1004900, PLACE1004902, PLACE1004969, PLACE1004979, PLACE1005086, PLACE1005162, PLACE1005243, PLACE1005266, PLACE1005313, PLACE1005584, PLACE1005698, PLACE1005727, PLACE1005739, PLACE1005851, PLACE1005925, PLACE1006196, PLACE1006248, PLACE1006262, PLACE1006335, PLACE1006360, PLACE1006385, PLACE1006412, PLACE1006414, PLACE1006445, PLACE1006598, PLACE1006629, PLACE1006640, PLACE1006792, PLACE1006815, PLACE1006901, PLACE1006917, PLACE1006962, PLACE1007021, PLACE1007045, PLACE1007112, PLACE1007226, PLACE1007243, PLACE1007454, PLACE1007547, PLACE1007598, PLACE1007618, PLACE1007645, PLACE1007649, PLACE1007706, PLACE1007725, PLACE1007746, PLACE1007843, PLACE1007877, PLACE1007954, PLACE1007969, PLACE1008002, PLACE1008095, PLACE1008111, PLACE1008132, PLACE1008273, PLACE1008275, PLACE1008331, PLACE1008356, PLACE1008368, PLACE1008402, PLACE1008603, PLACE1008650, PLACE1008696, PLACE1008757, PLACE1008807, PLACE1008867, PLACE1008941, PLACE1009020, PLACE1009039, PLACE1009158, PLACE1009172, PLACE1009190, PLACE1009230, PLACE1009319, PLACE1009338, PLACE1009375, PLACE1009434, PLACE1009613, PLACE1009637, PLACE1009659, PLACE1009763, PLACE1009845, PLACE1009921, PLACE1009971, PLACE1009992, PLACE1010023, PLACE1010053, PLACE1010069, PLACE1010074, PLACE1010181, PLACE1010202, PLACE1010231, PLACE1010270, PLACE1010274, PLACE1010321, PLACE1010329, PLACE1010492, PLACE1010522, PLACE1010616, PLACE1010624, PLACE1010631, PLACE1010786, PLACE1011032, PLACE1011114, PLACE1011221, PLACE1011325, PLACE1011520, PLACE1011635, PLACE1011649, PLACE1011682, PLACE1011875, PLACE1011896, PLACE1011964, PLACE1012031, PLACE2000015, PLACE2000021, PLACE2000047, PLACE2000072, PLACE2000097, PLACE2000136, PLACE2000246, PLACE2000302, PLACE2000379, PLACE2000394, PLACE2000425, PLACE2000427, PLACE2000477, PLACE3000009, PLACE3000070, PLACE3000142, PLACE3000145, PLACE3000148, PLACE3000155, PLACE3000169, PLACE3000208, PLACE3000230, PLACE3000322, PLACE3000331, PLACE3000352, PLACE3000401, PLACE3000413, PLACE3000425, PLACE3000477, PLACE4000009, PLACE4000049, PLACE4000089, PLACE4000100, PLACE4000247, PLACE4000250, PLACE4000252, PLACE4000300, PLACE4000344, PLACE4000367, PLACE4000465, PLACE4000489, PLACE4000638, SKNMC1000013, THYRO1000017, THYRO1000026, THYRO1000034, THYRO1000072, THYRO1000132, THYRO1000173, THYRO1000190, THYRO1000197, THYRO1000221, THYRO1000253, THYRO1000270, THYRO1000279, THYRO1000327, THYRO1000394, THYRO1000438, THYRO1000558, THYRO1000569, THYRO1000585, THYRO1000596, THYRO1000625, THYRO1000637, THYRO1000676, THYRO1000734, THYRO1000777, THYRO1000783, THYRO1000805, THYRO1000843, THYRO1000934, THYRO1001033, THYRO1001347, THYRO1001405, THYRO1001411, THYRO1001534, THYRO1001573, THYRO1001584, THYRO1001602, THYRO1001605, THYRO1001772, THYRO1001854, VESEN1000122, Y79AA1000037, Y79AA1000065, Y79AA1000181, Y79AA1000231, Y79AA1000349, Y79AA1000355, Y79AA1000368, Y79AA1000538, Y79AA1000782, Y79AA1001023, Y79AA1001145, Y79AA1001391, Y79AA1001541, Y79AA1001585, Y79AA1001705, Y79AA1001781, Y79AA1001923, Y79AA1001963, Y79AA1002125, Y79AA1002229, Y79AA1002407, Y79AA1002487.

These clones are ultraviolet radiation damage-associated clones.

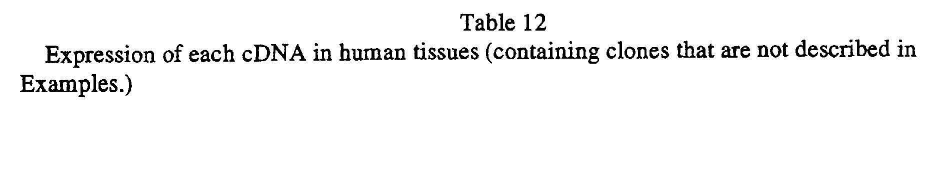

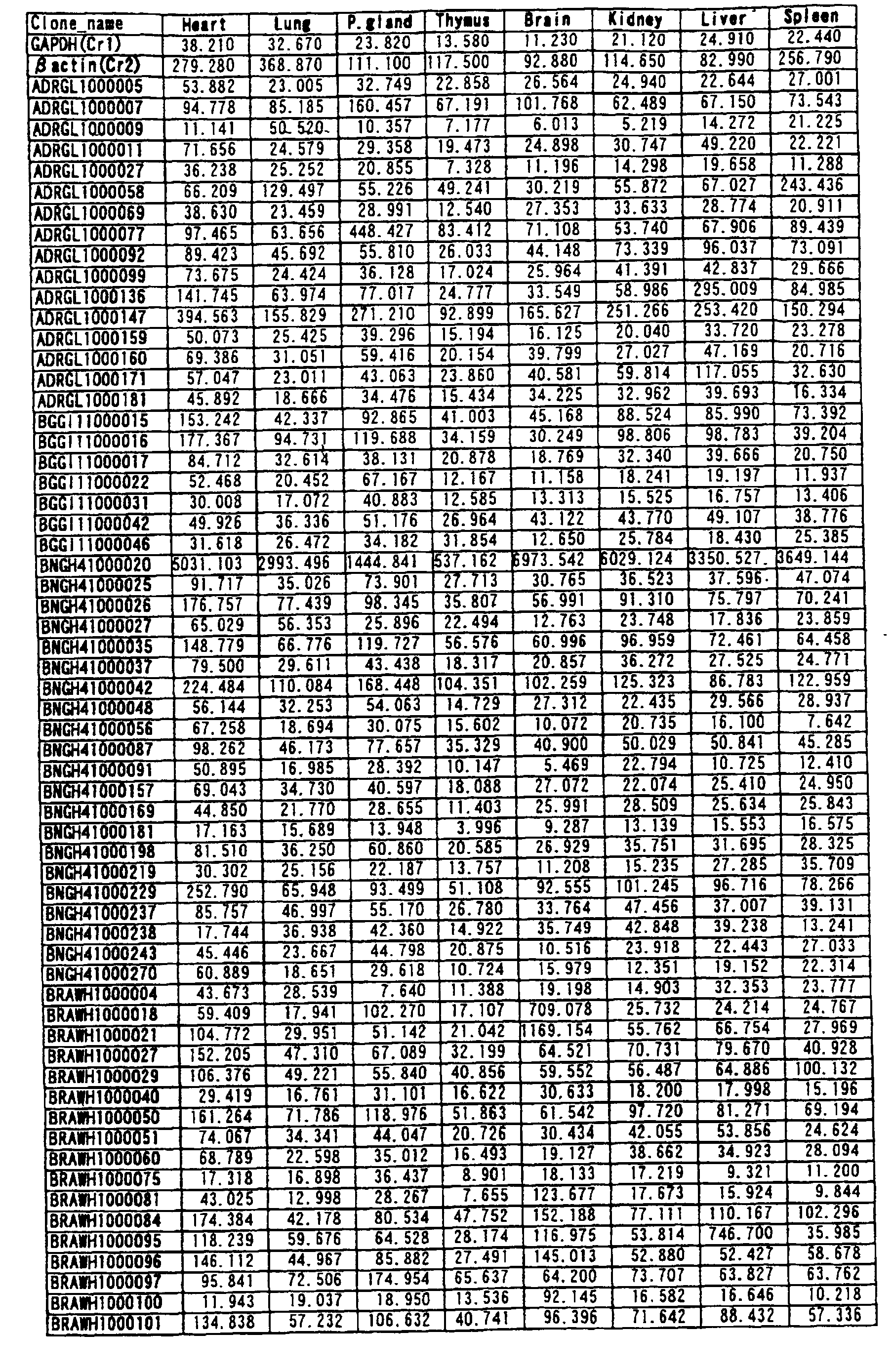

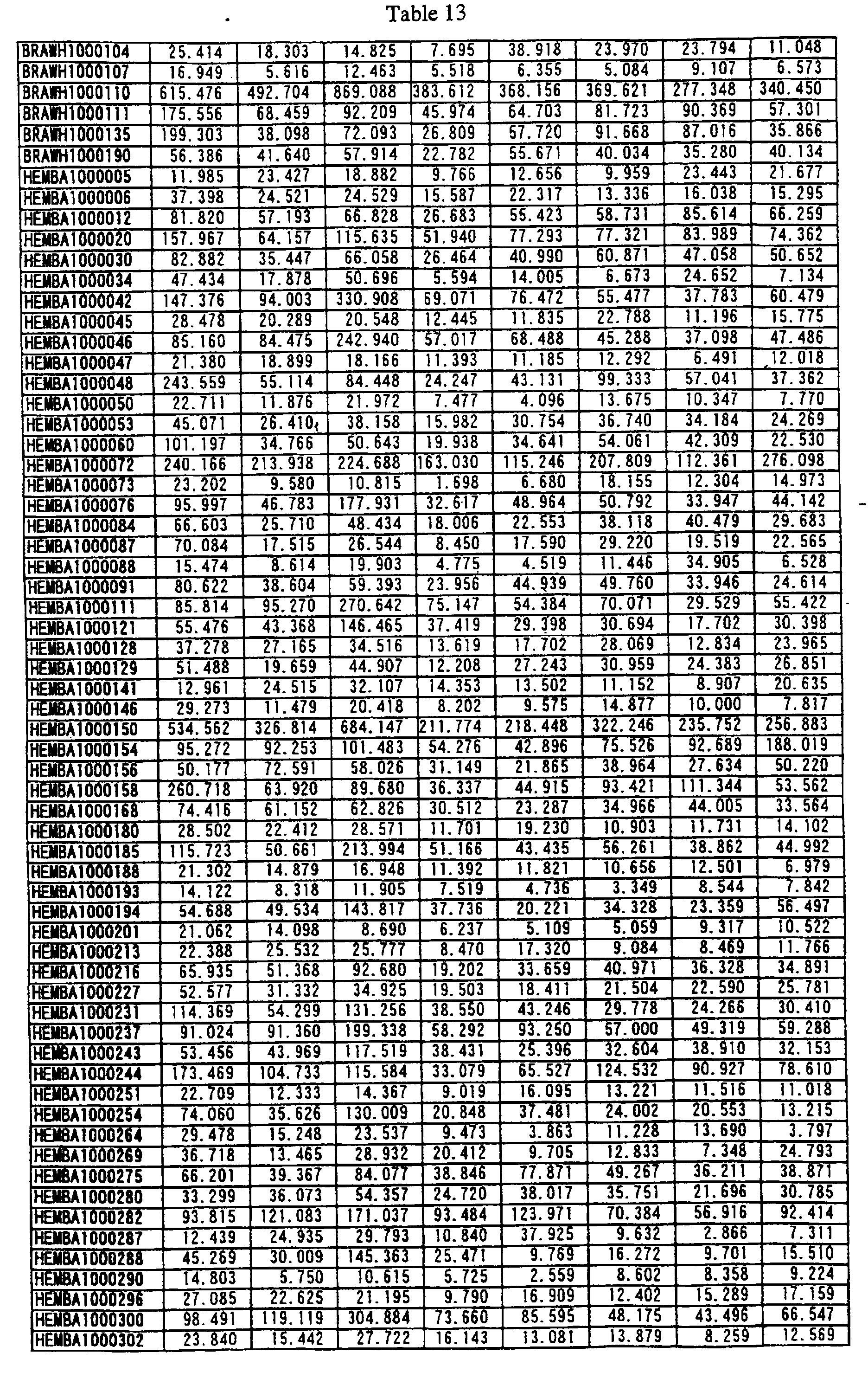

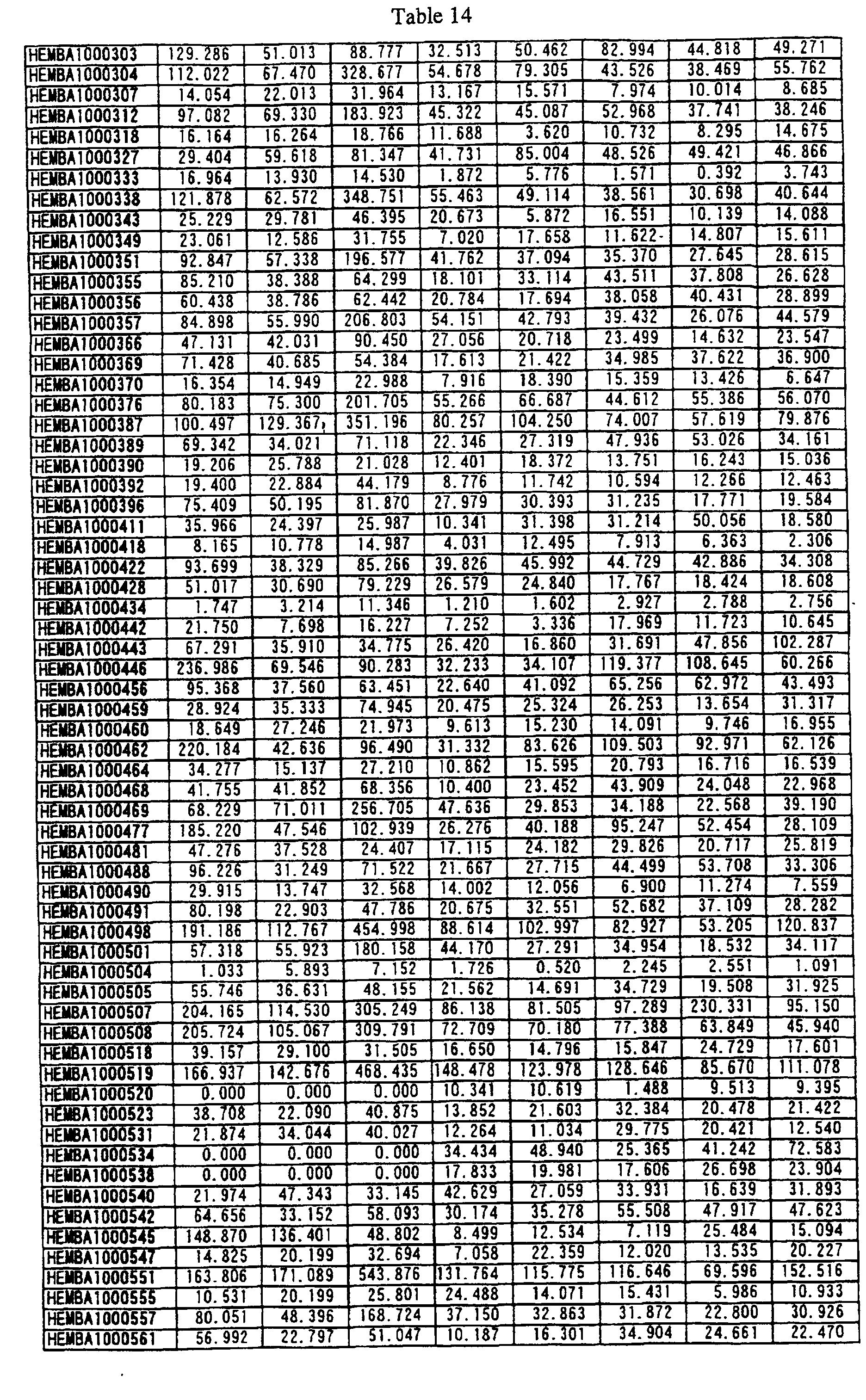

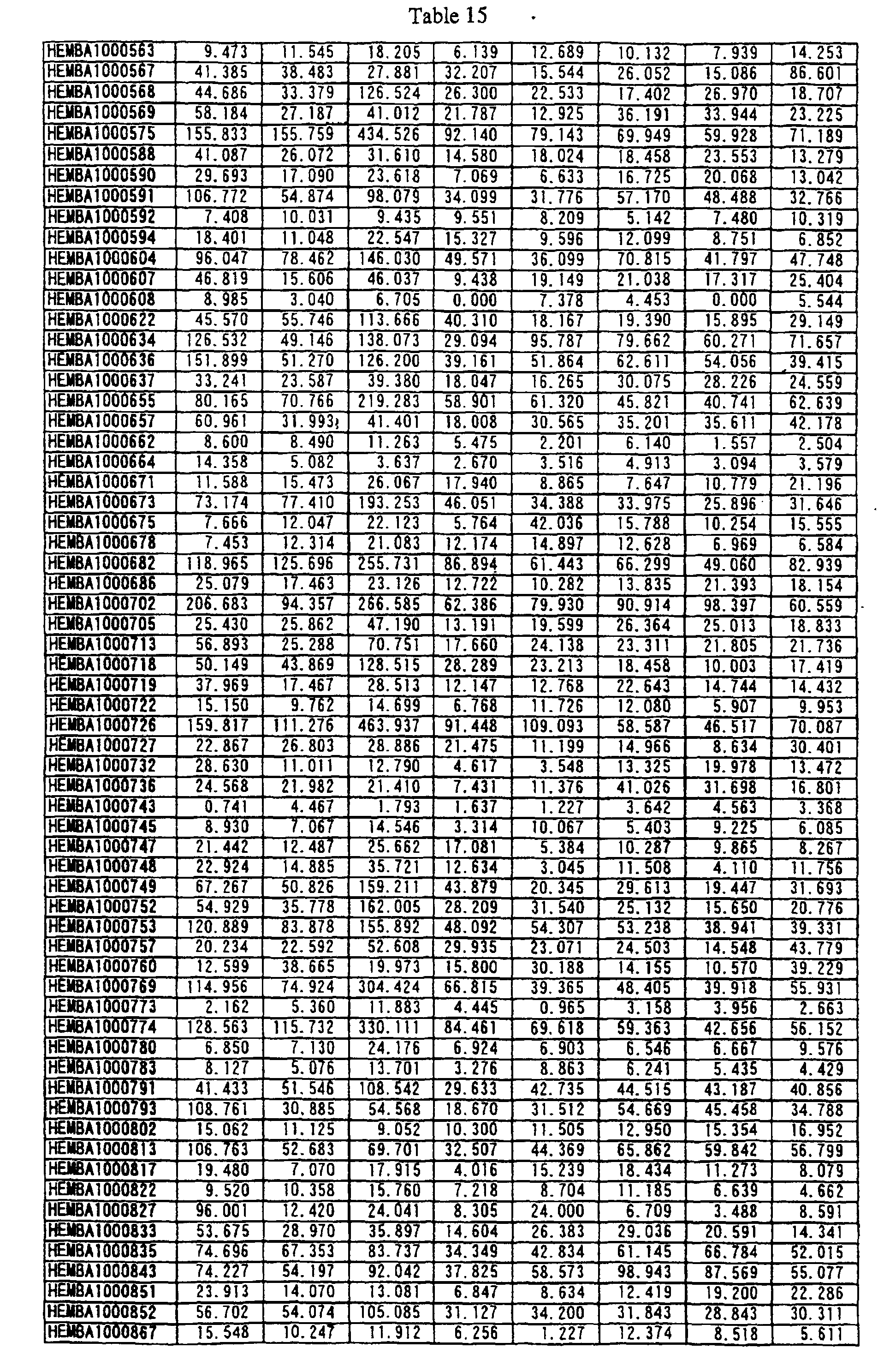

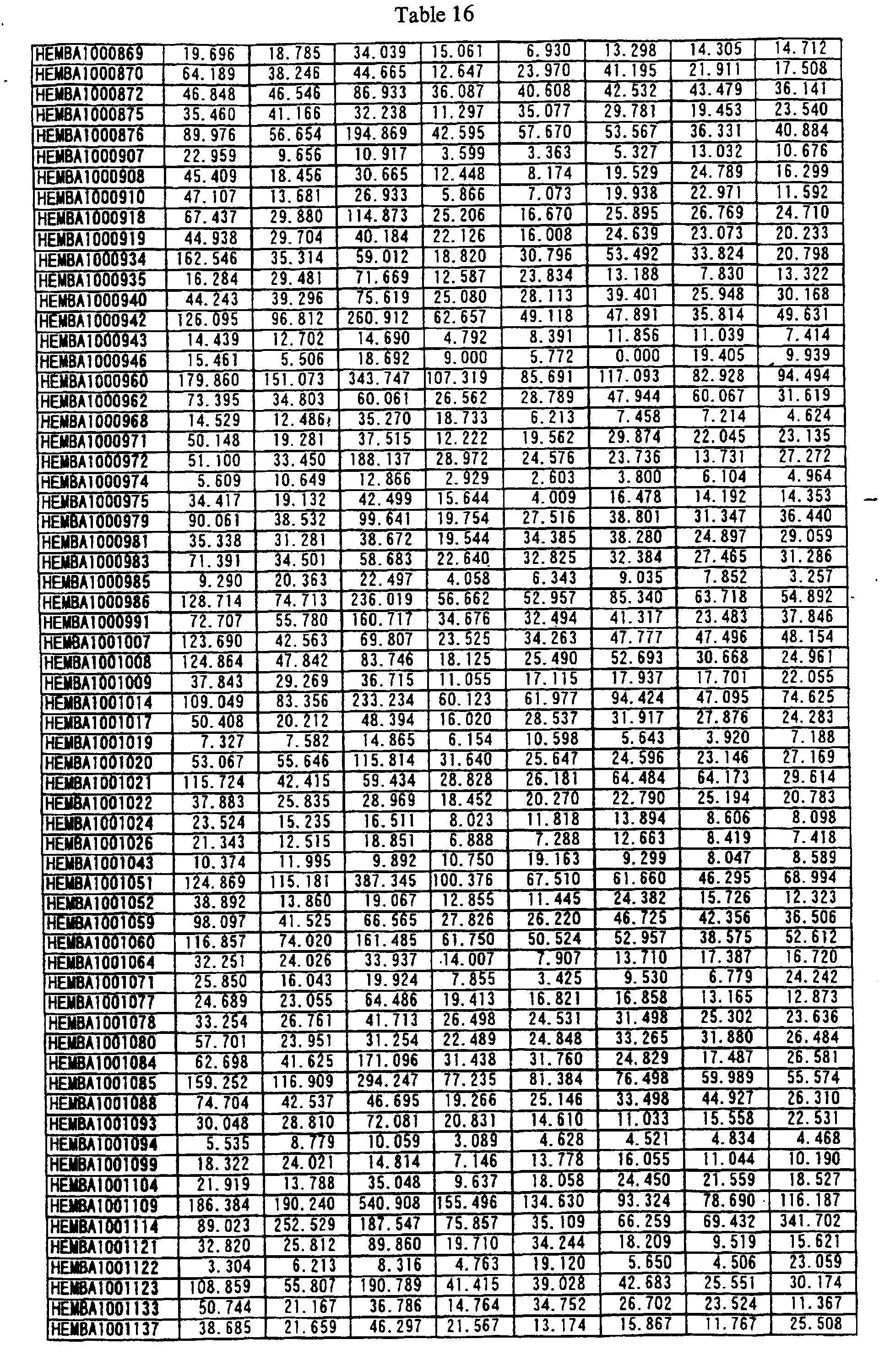

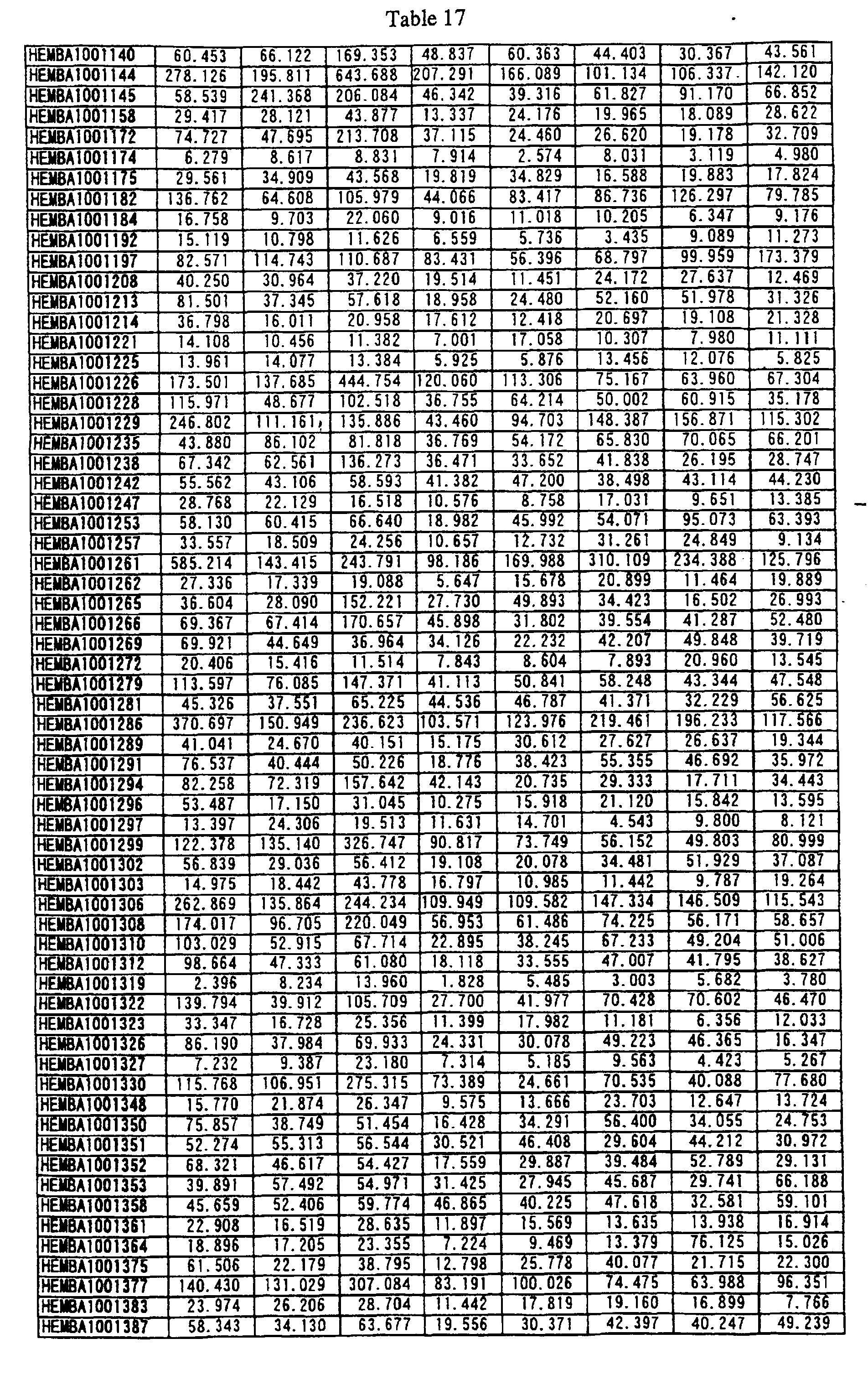

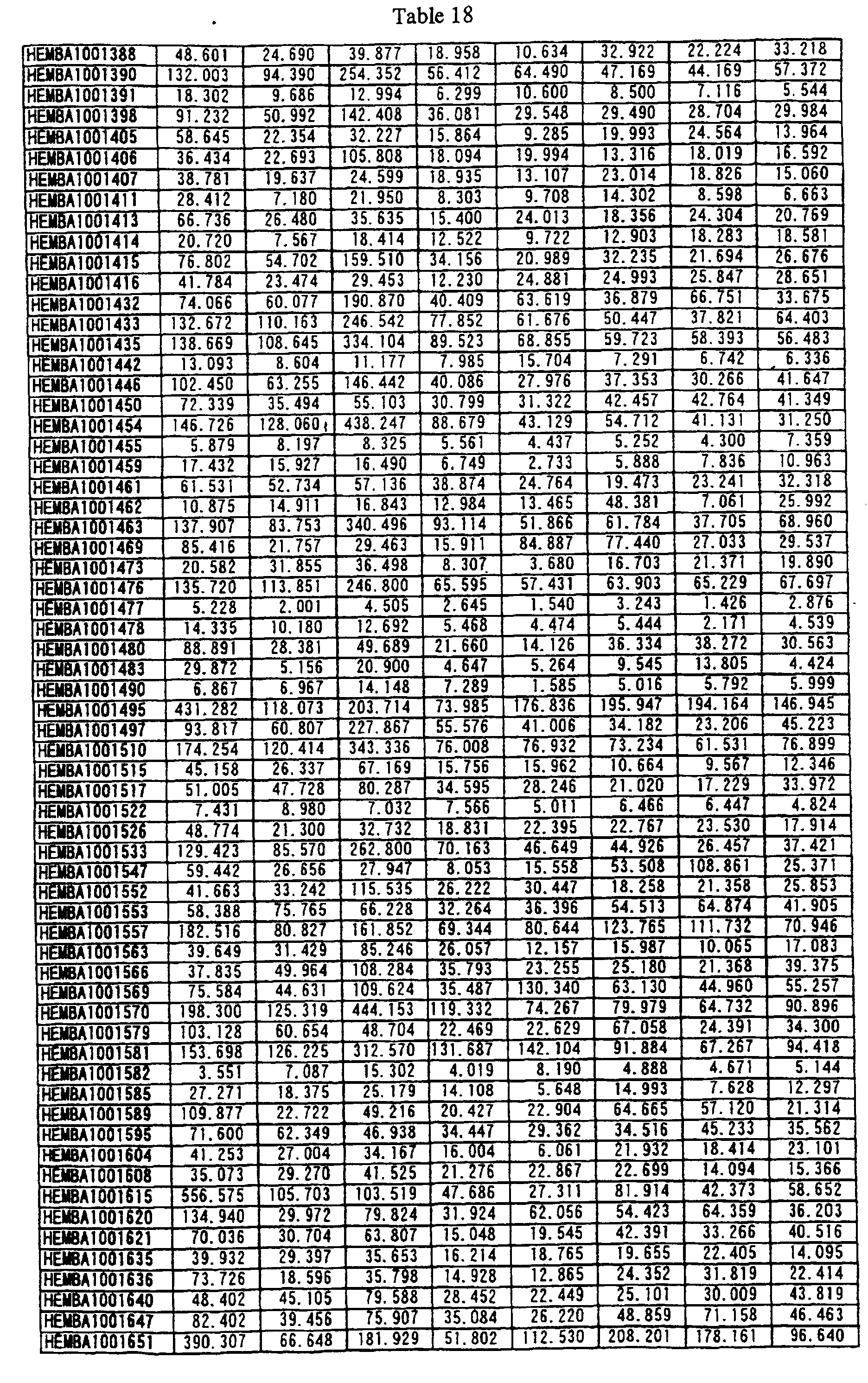

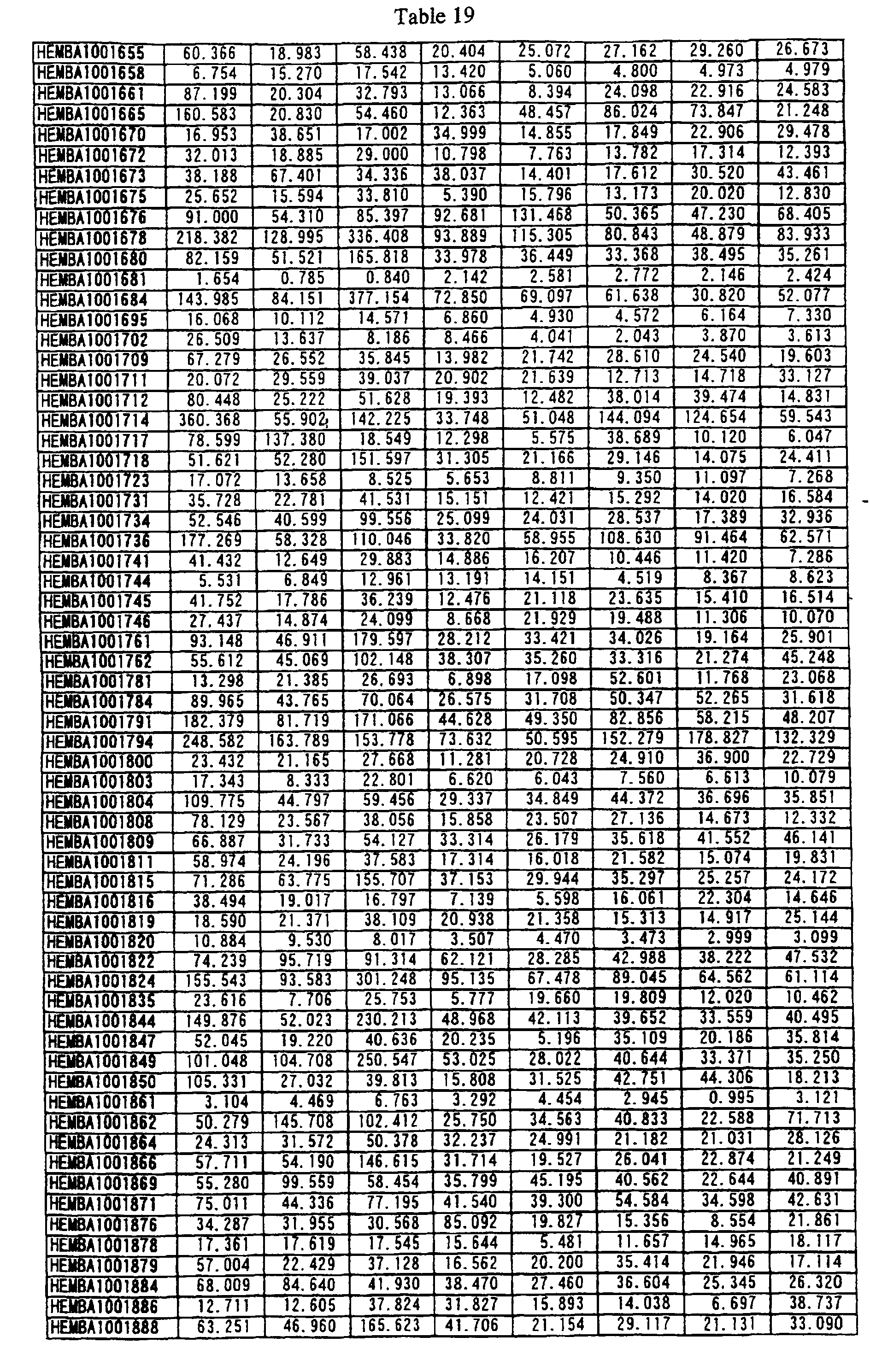

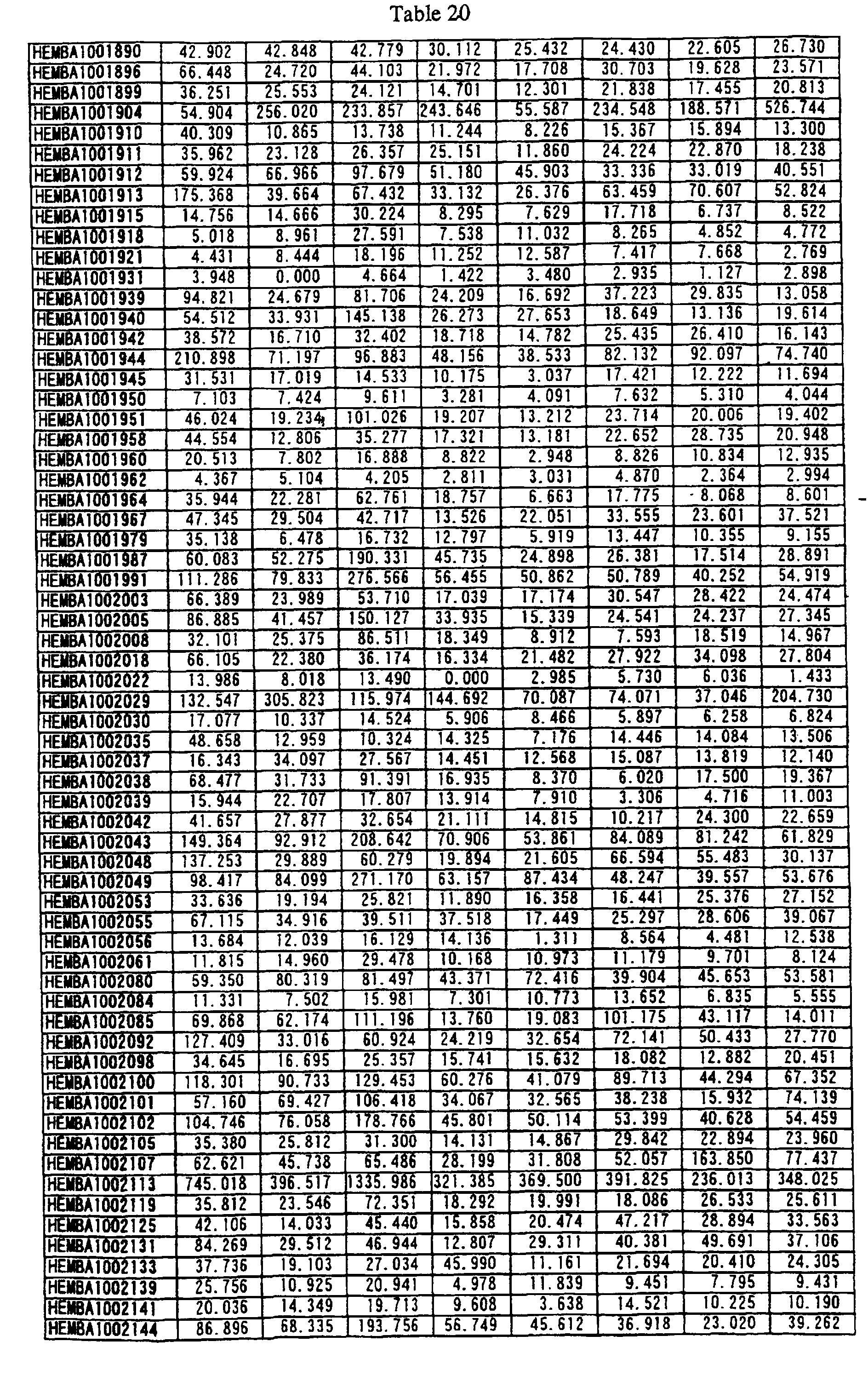

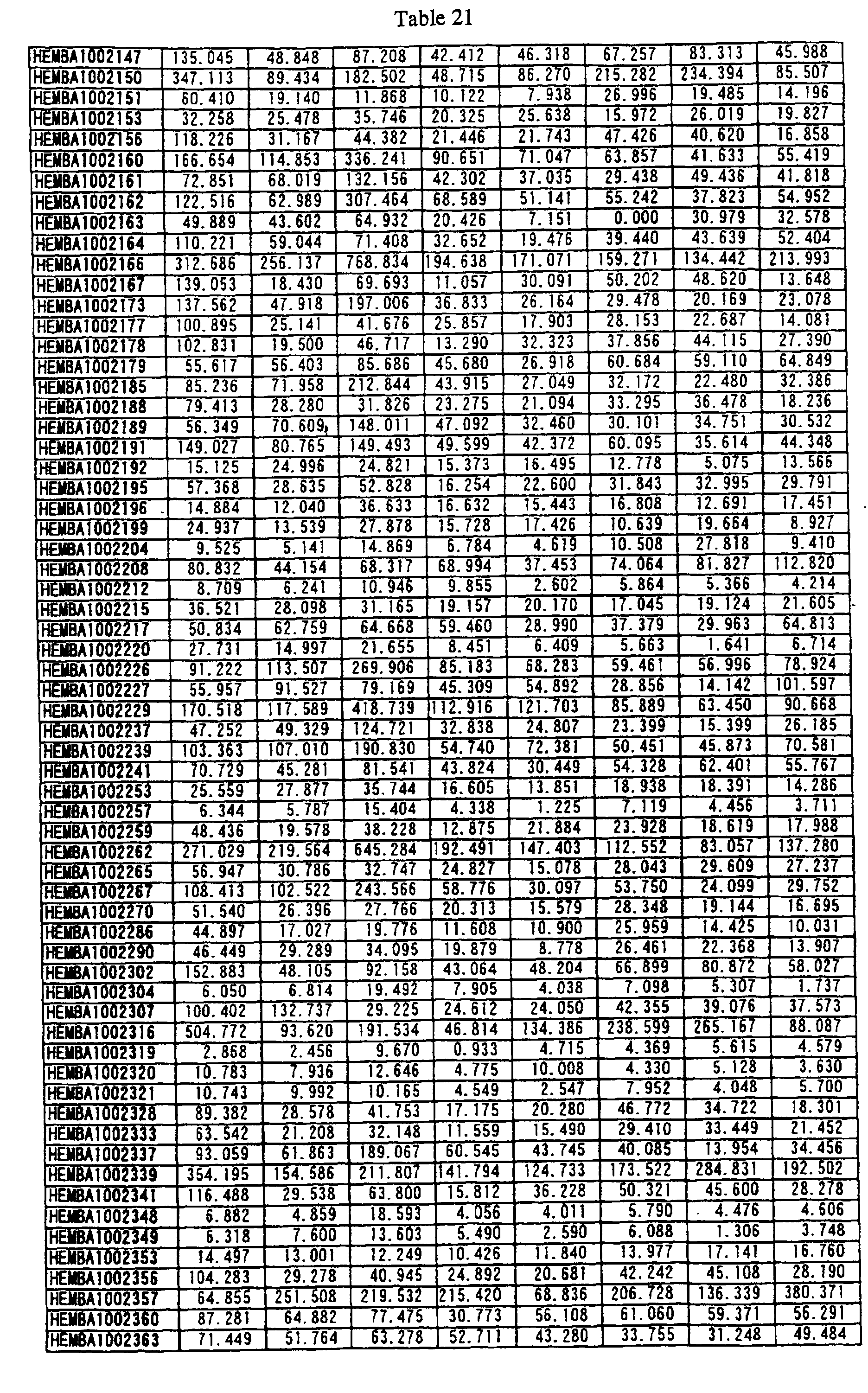

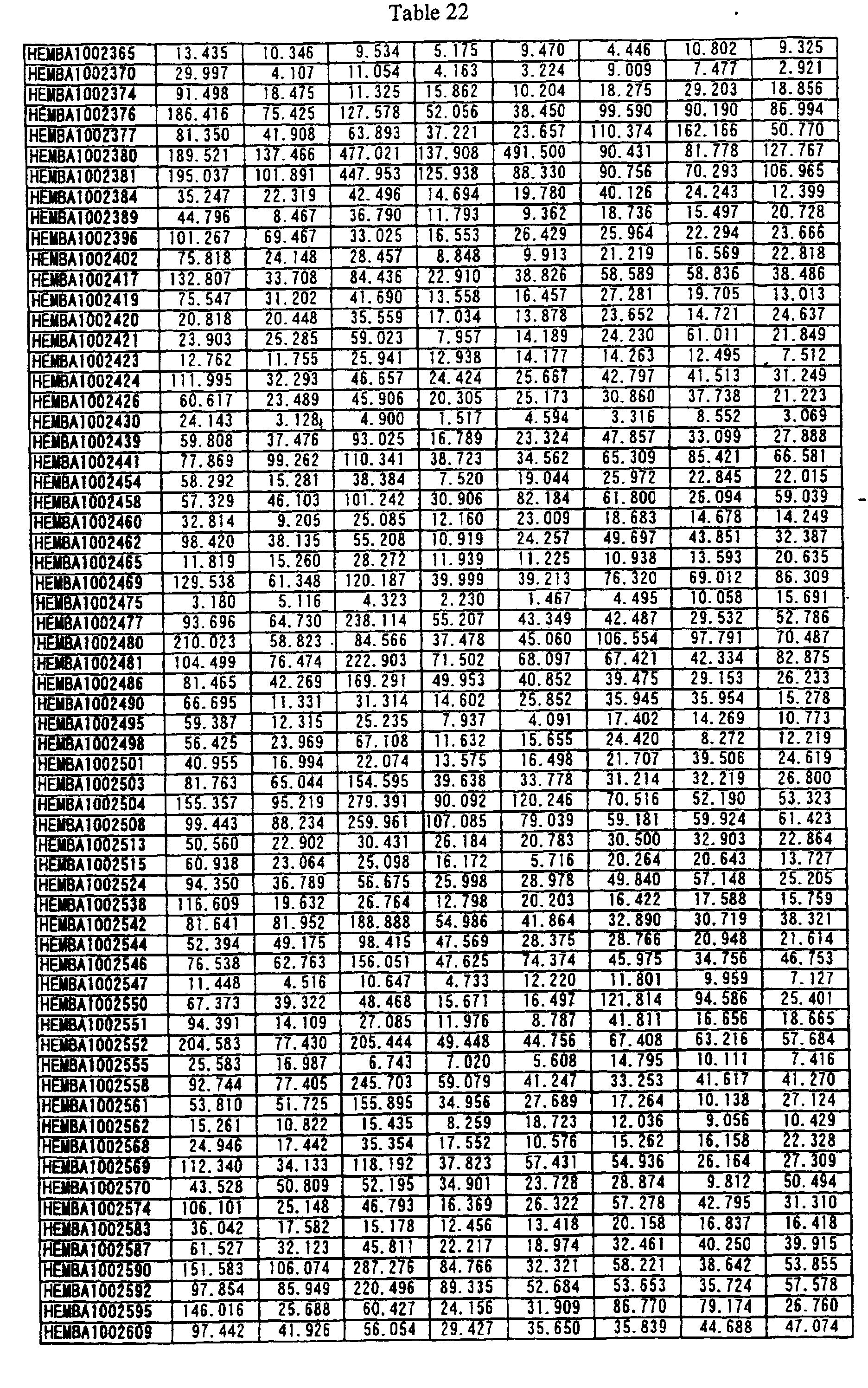

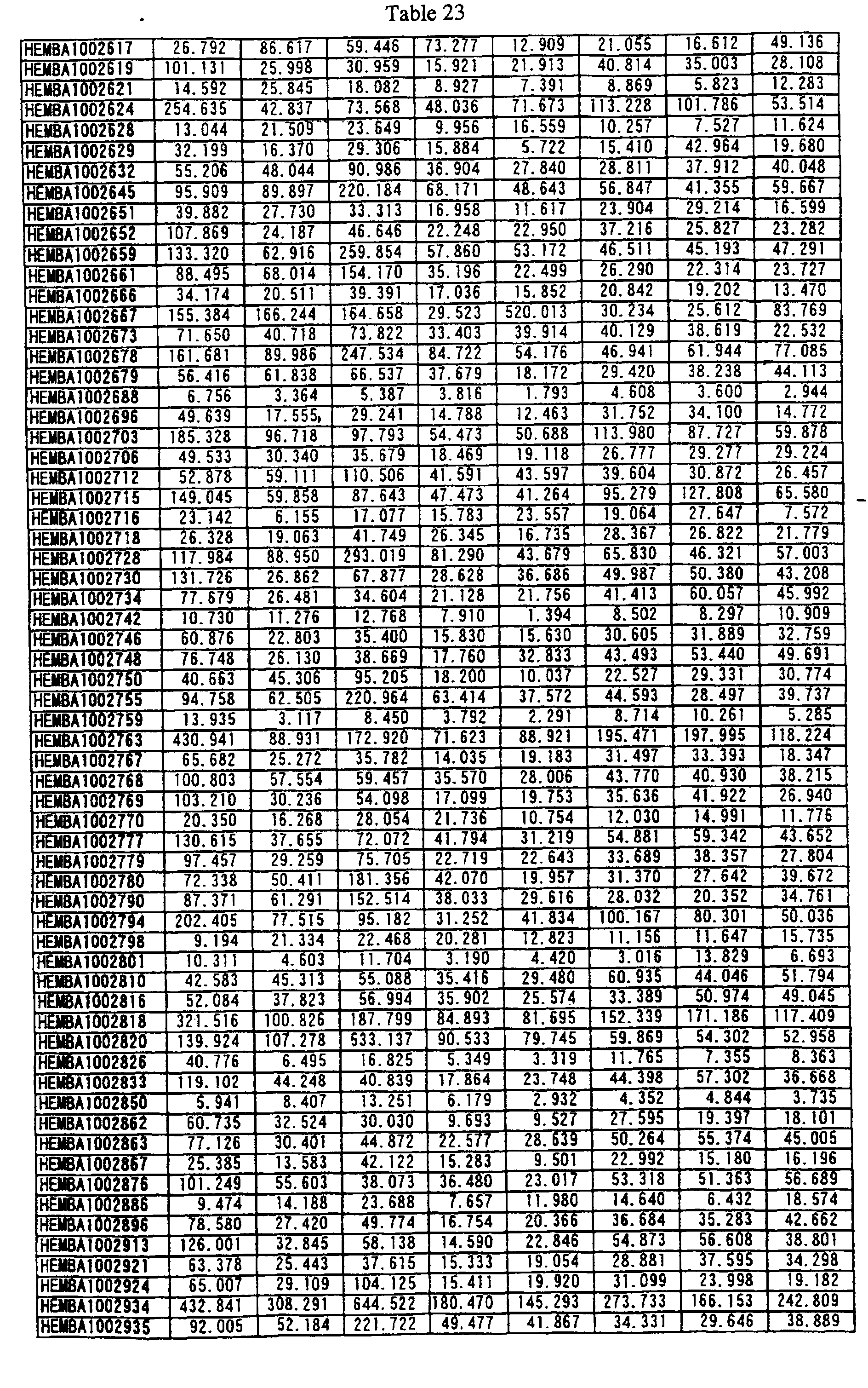

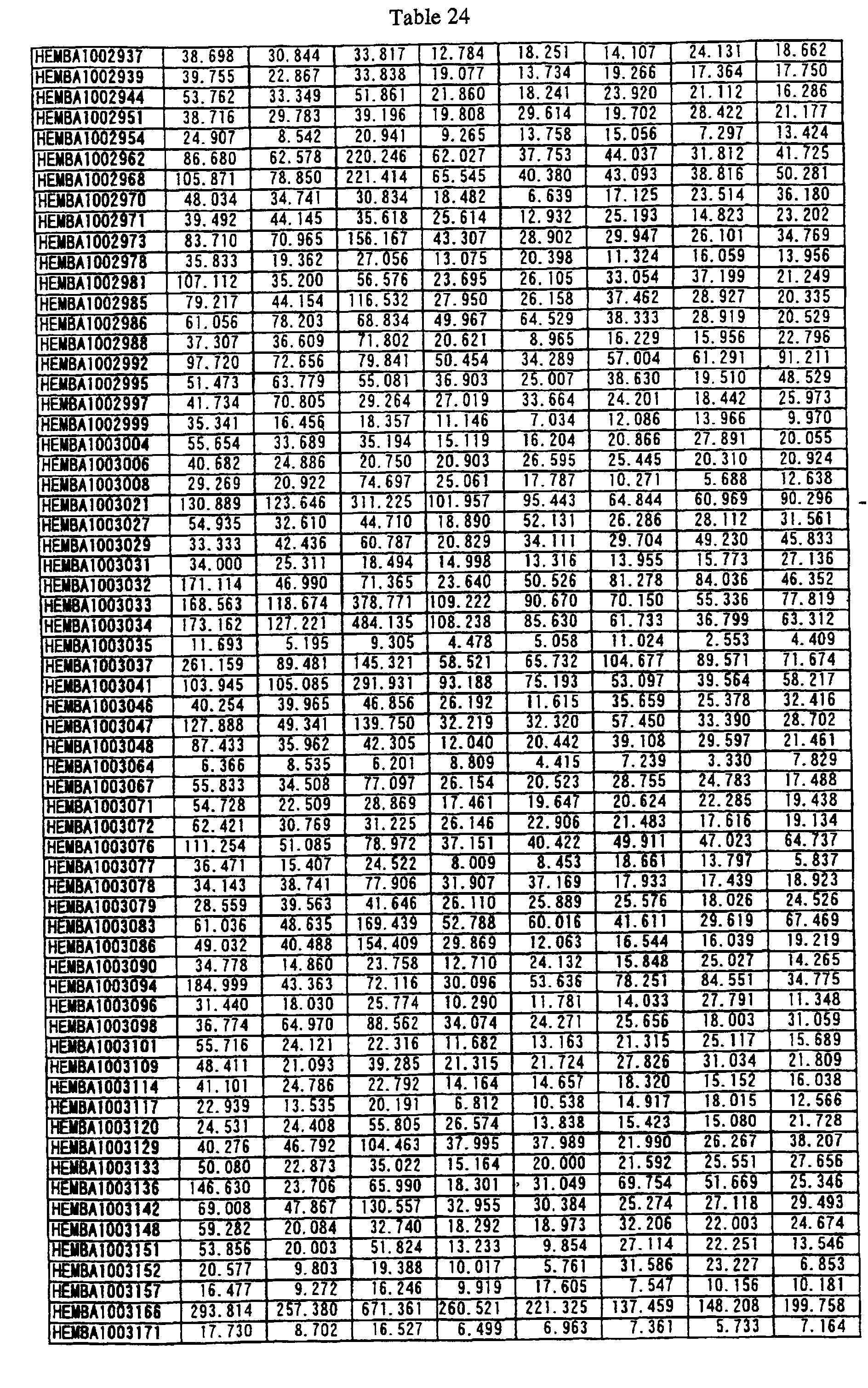

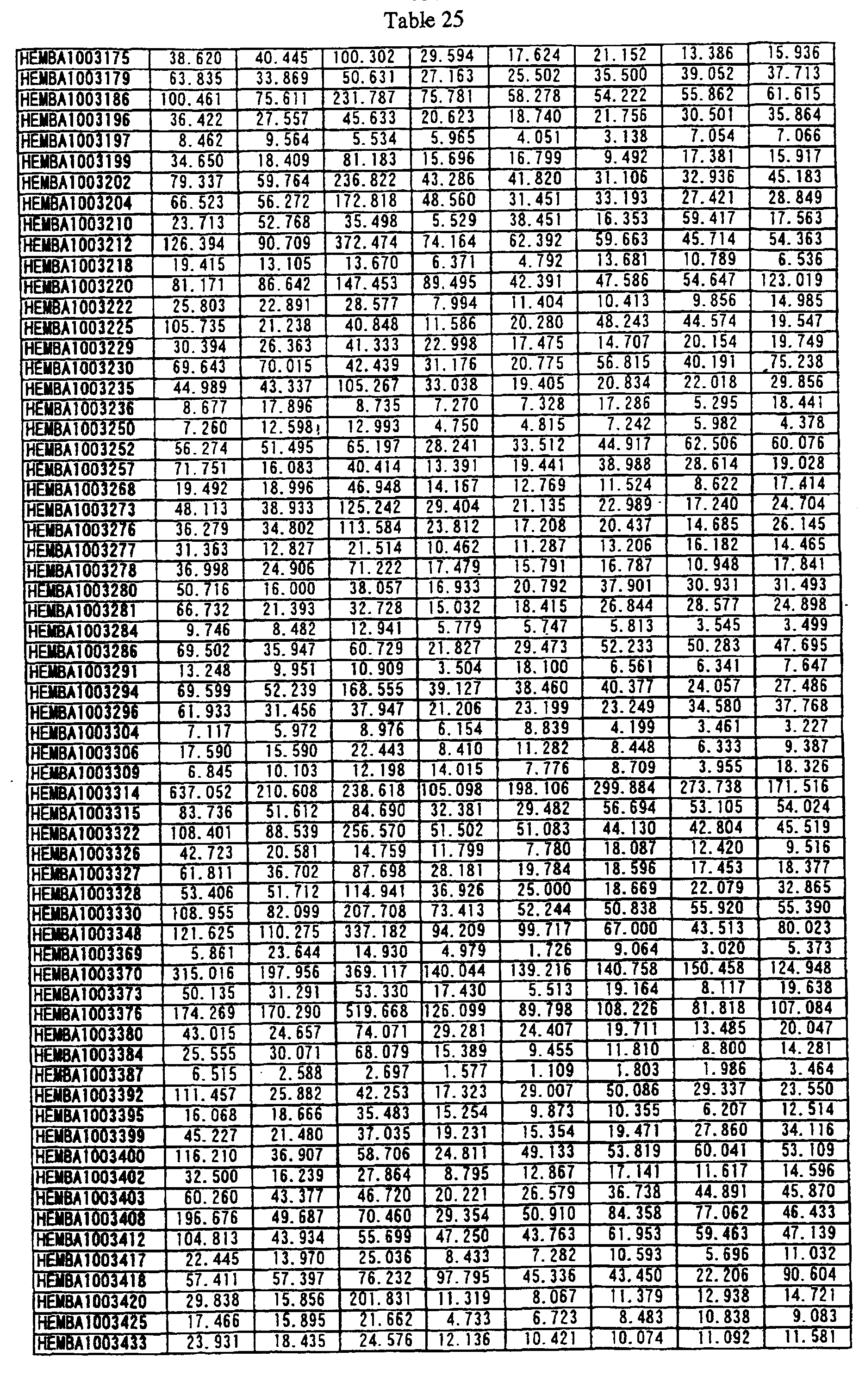

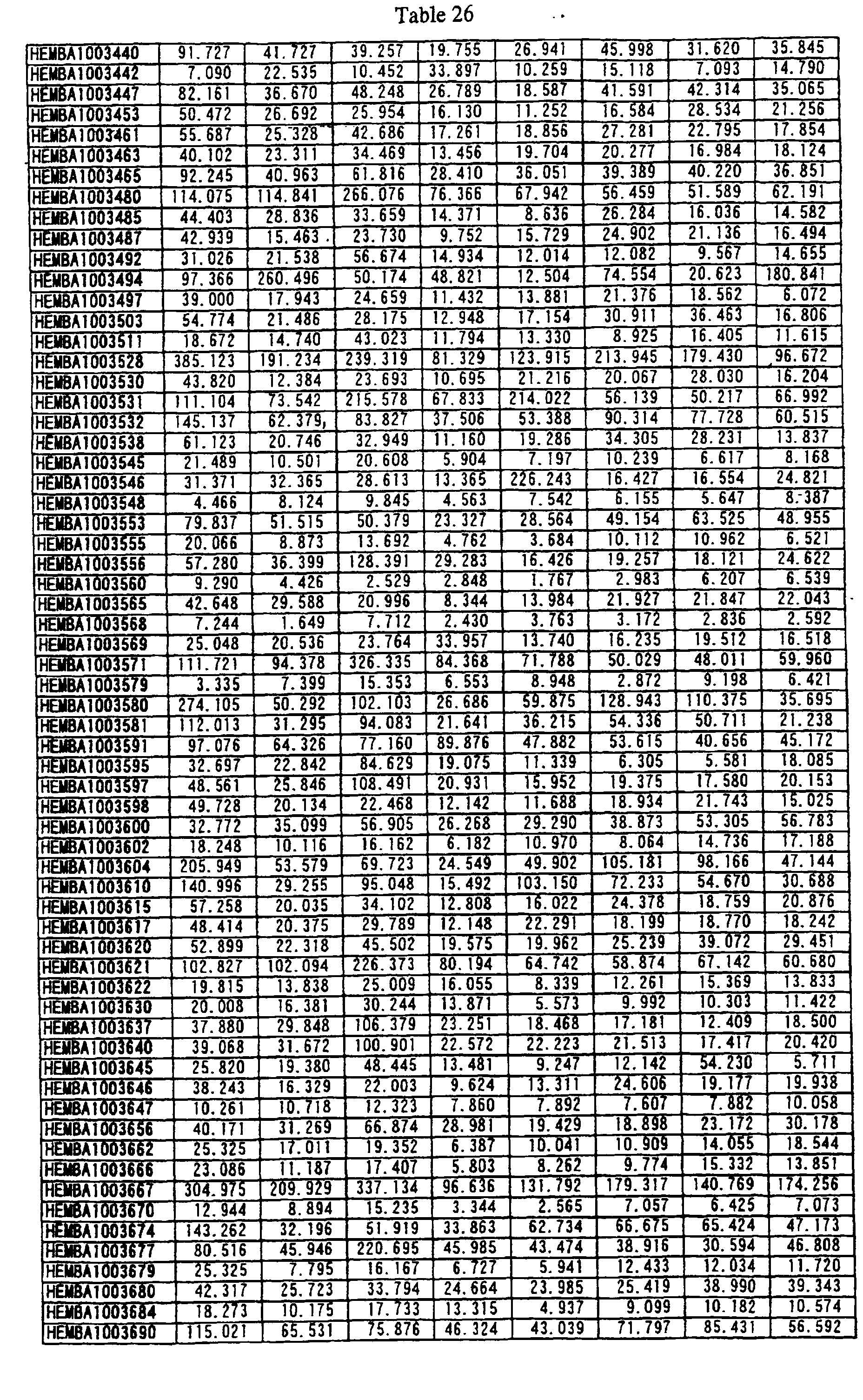

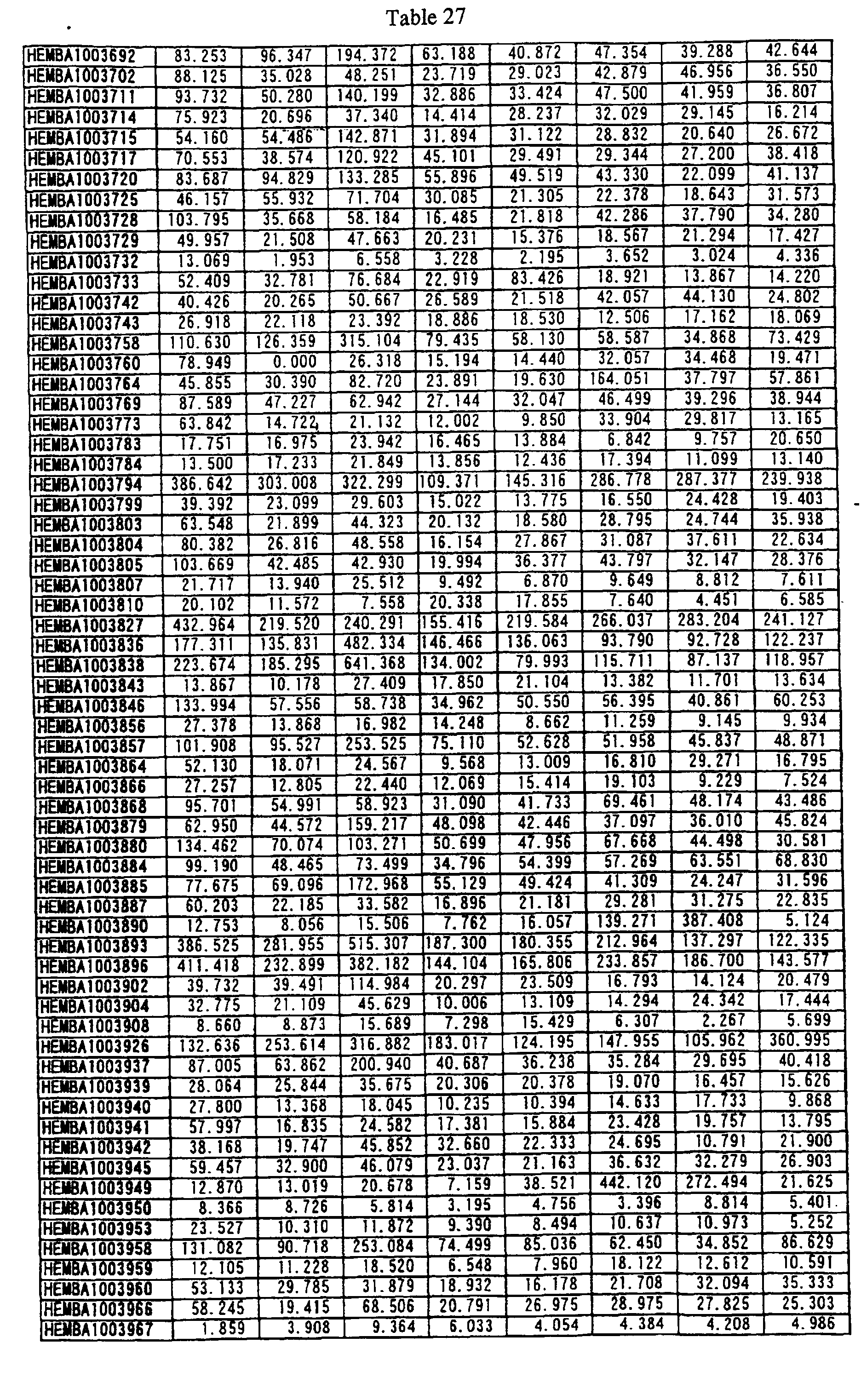

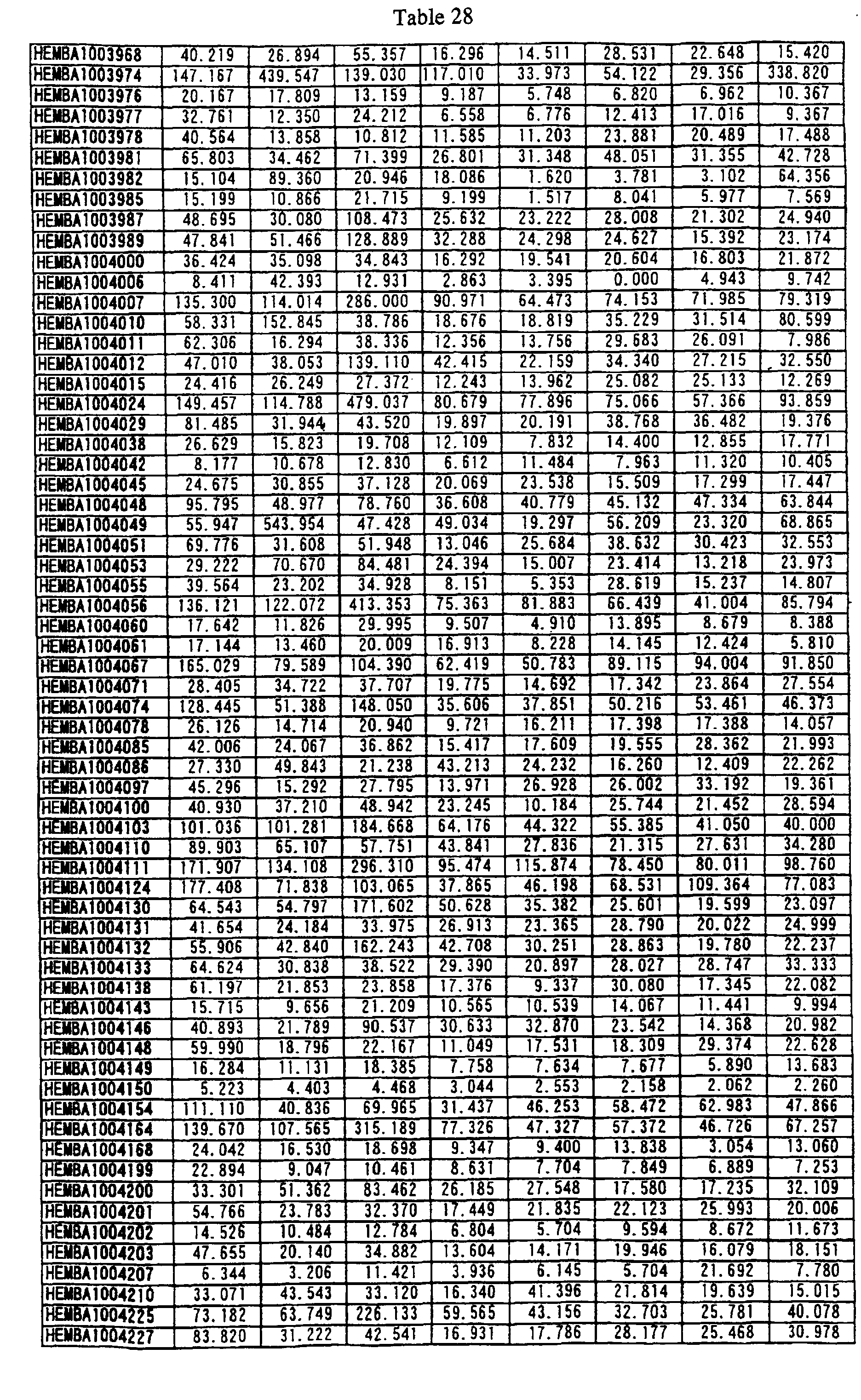

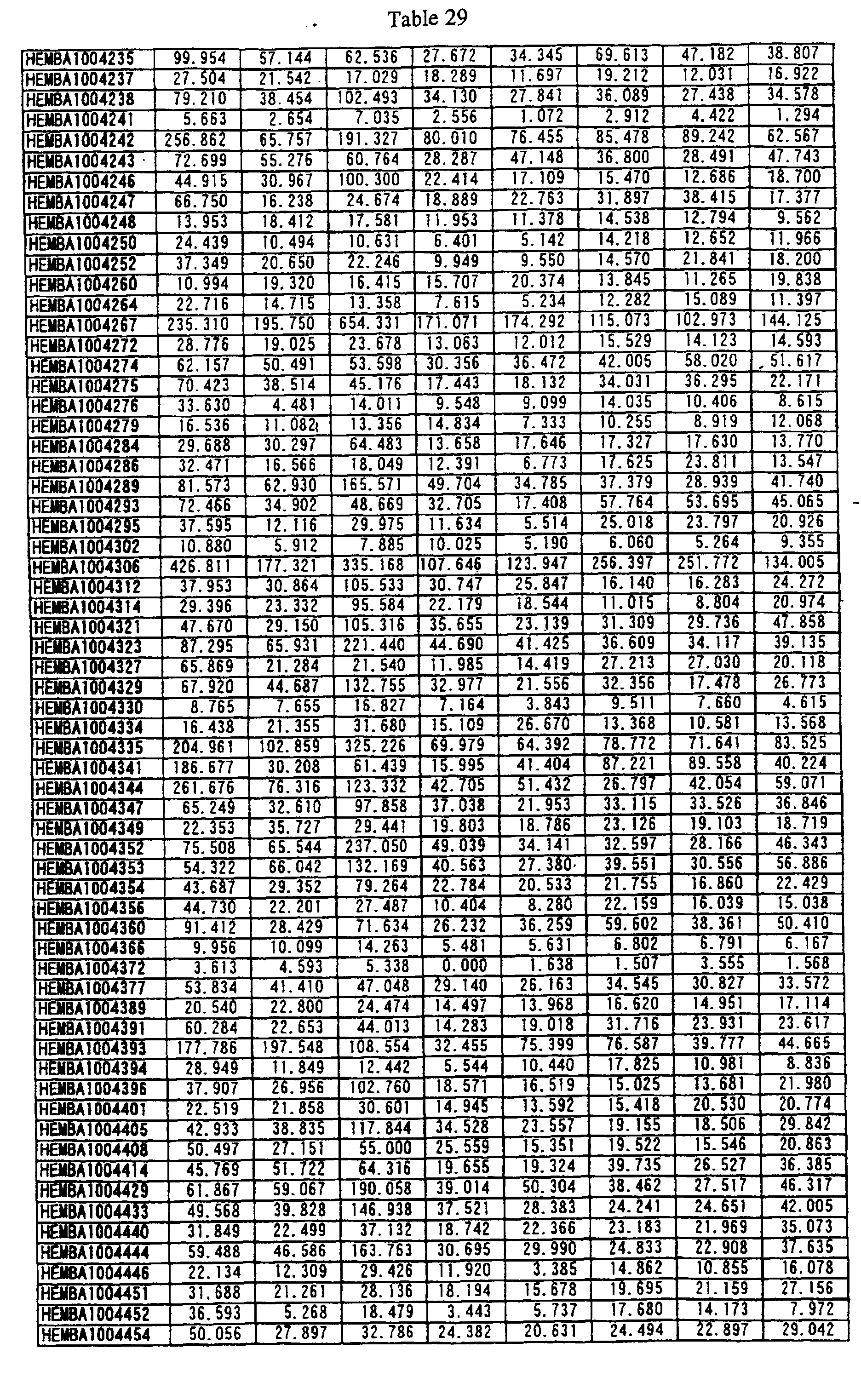

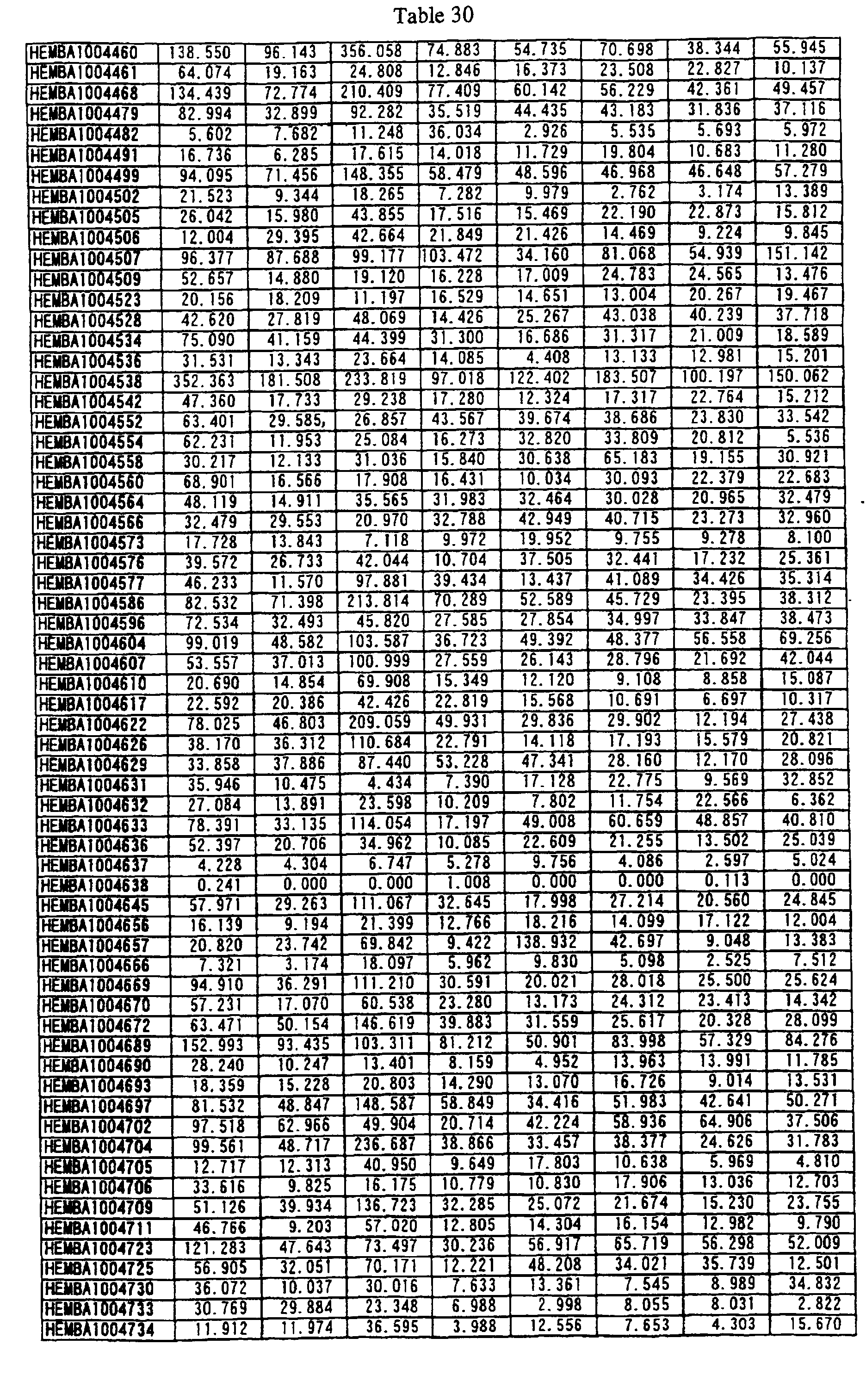

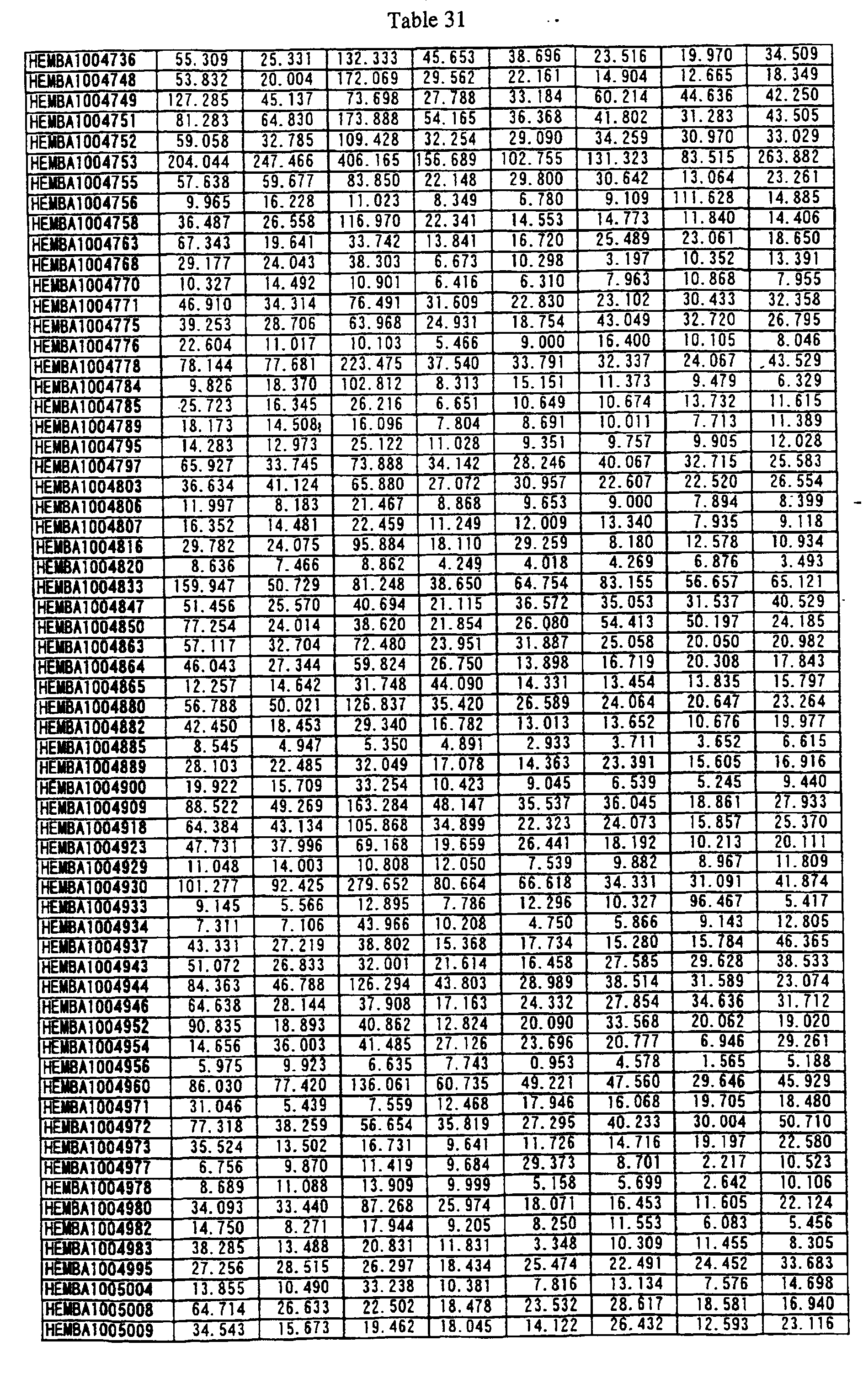

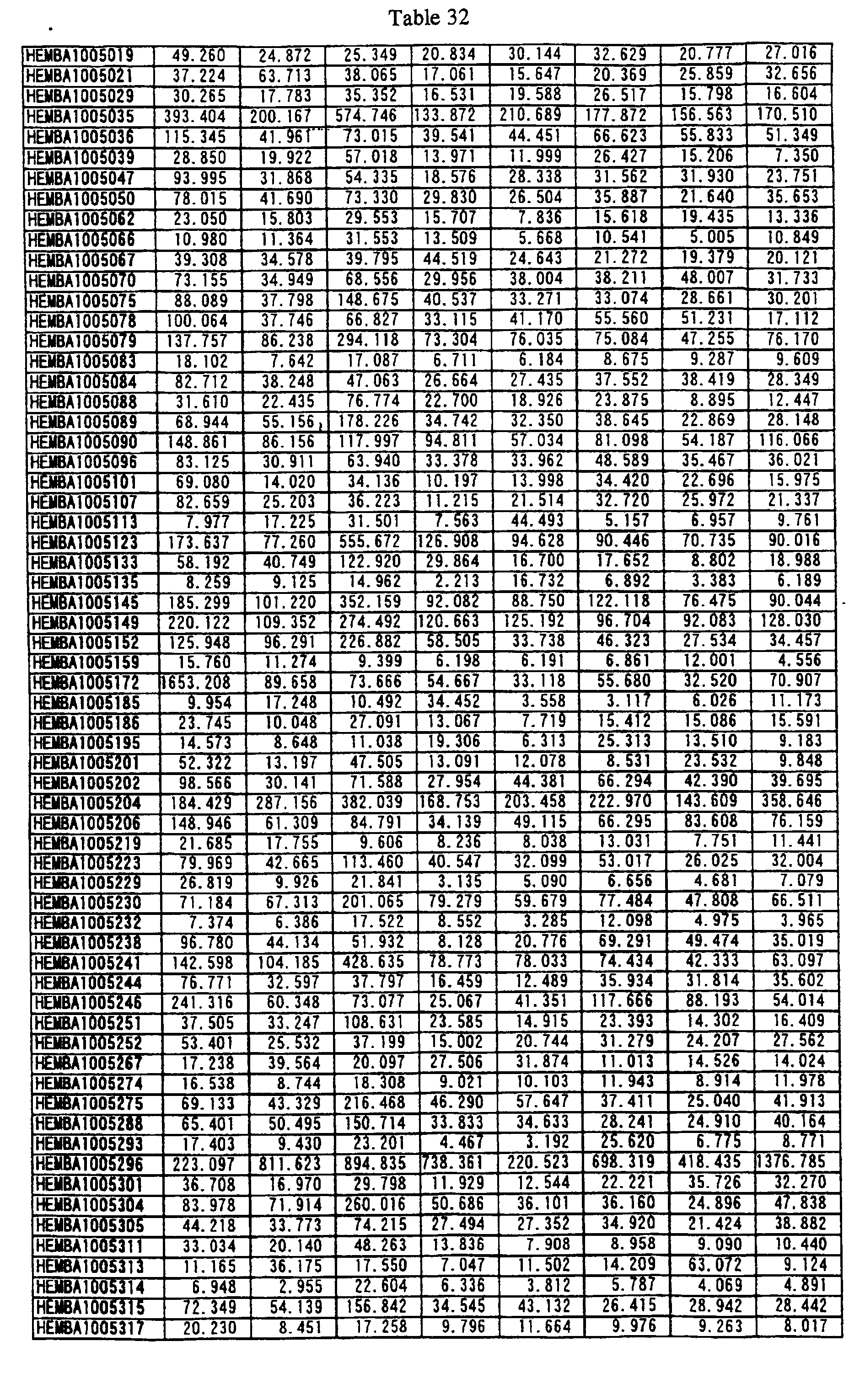

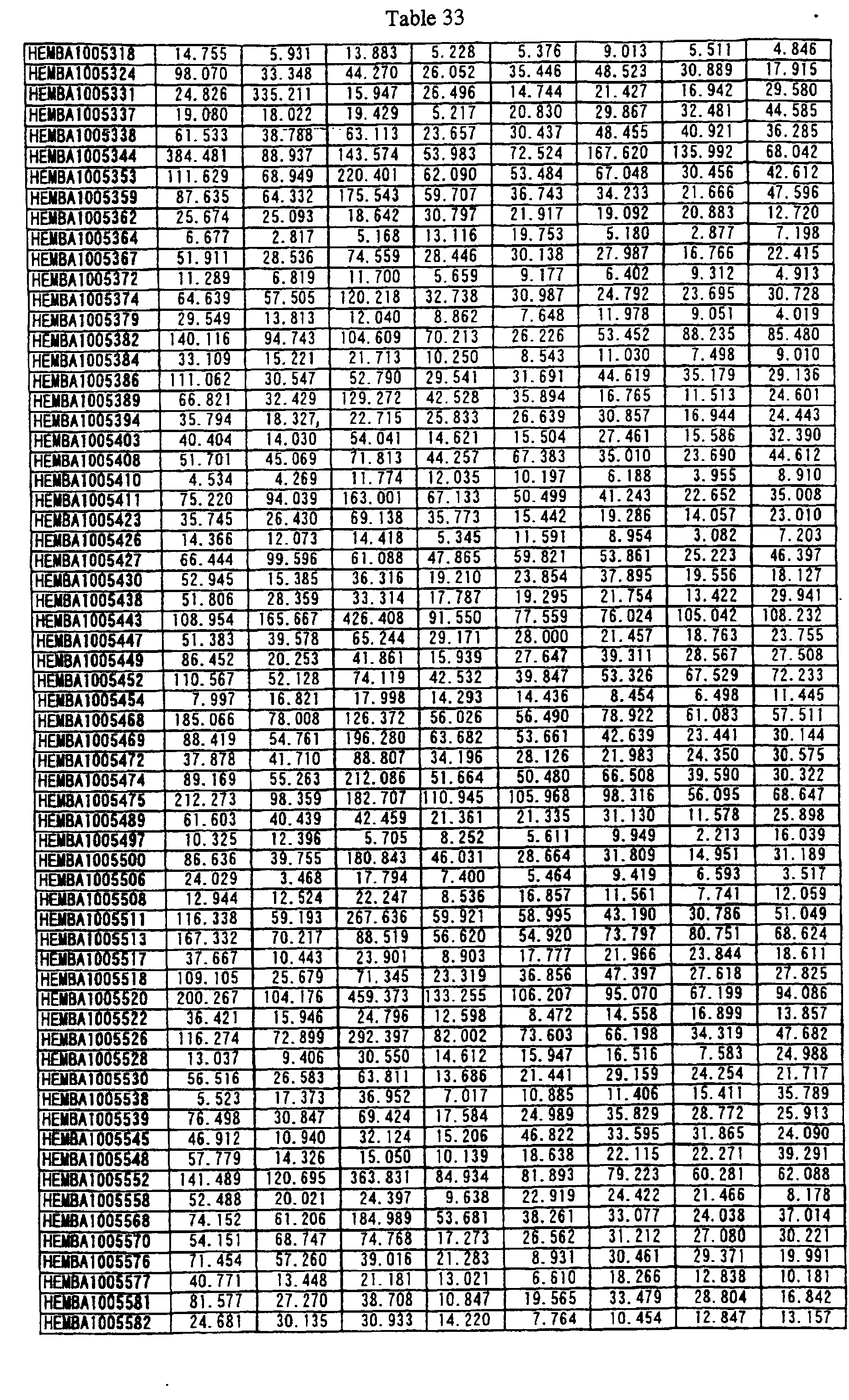

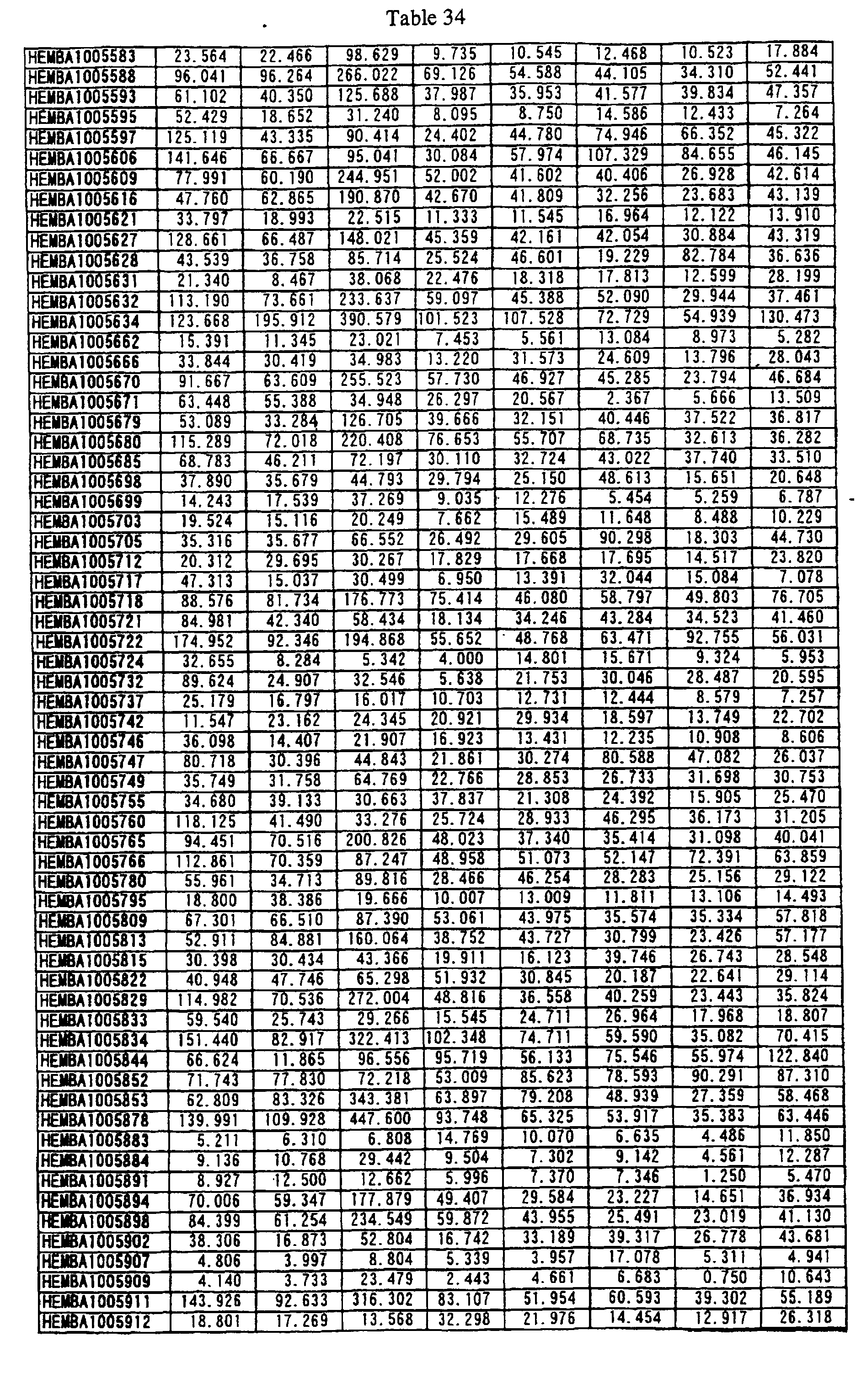

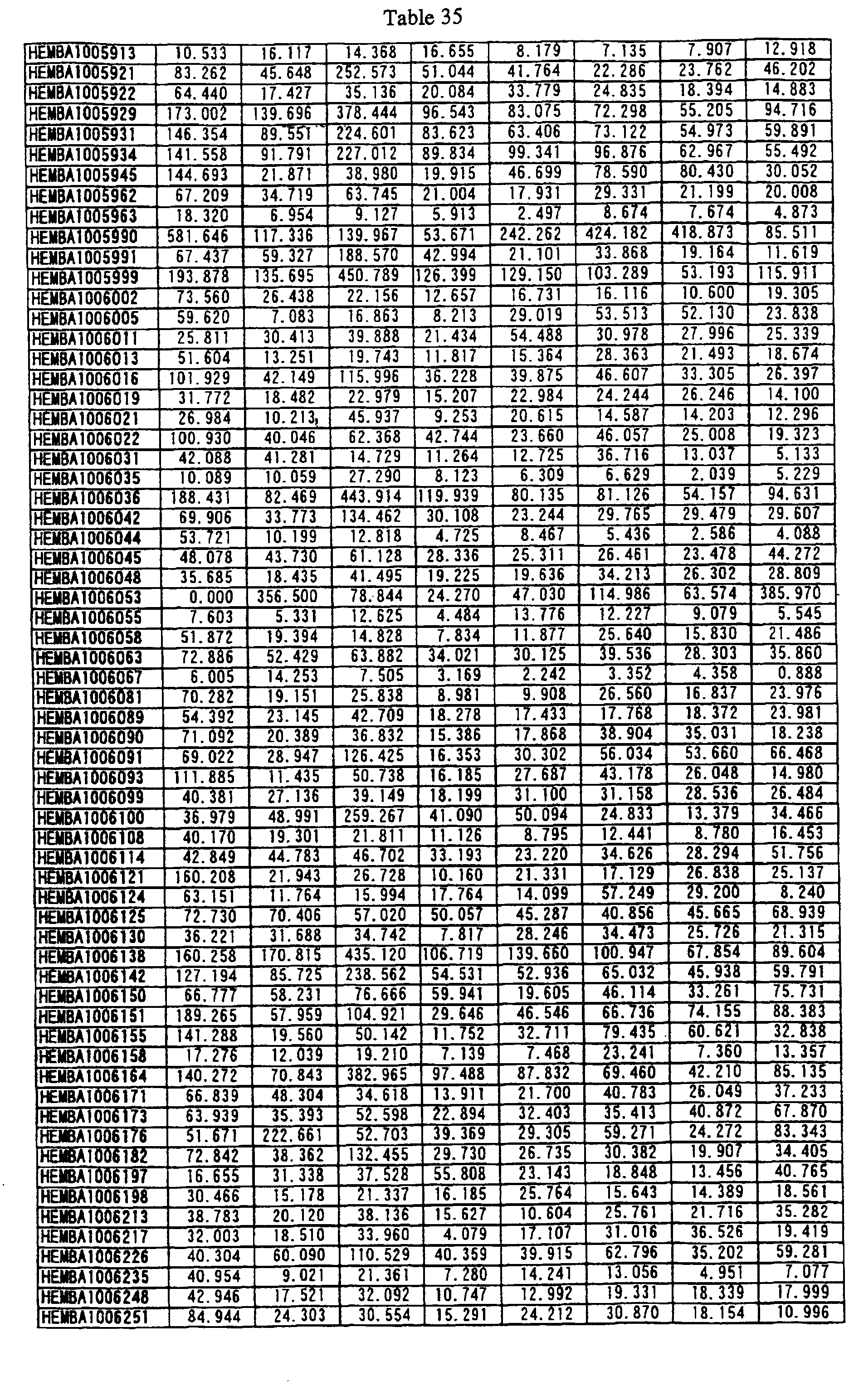

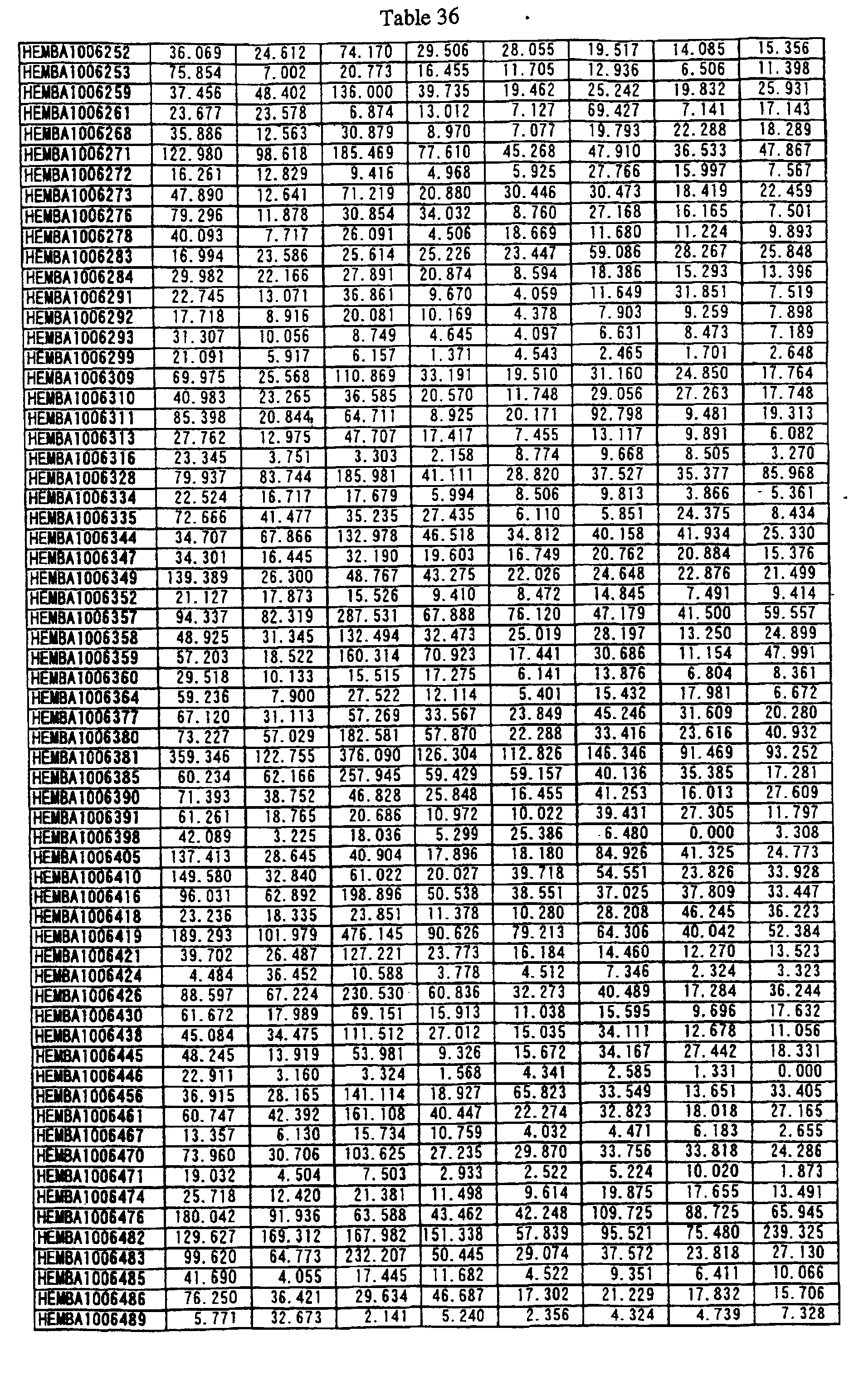

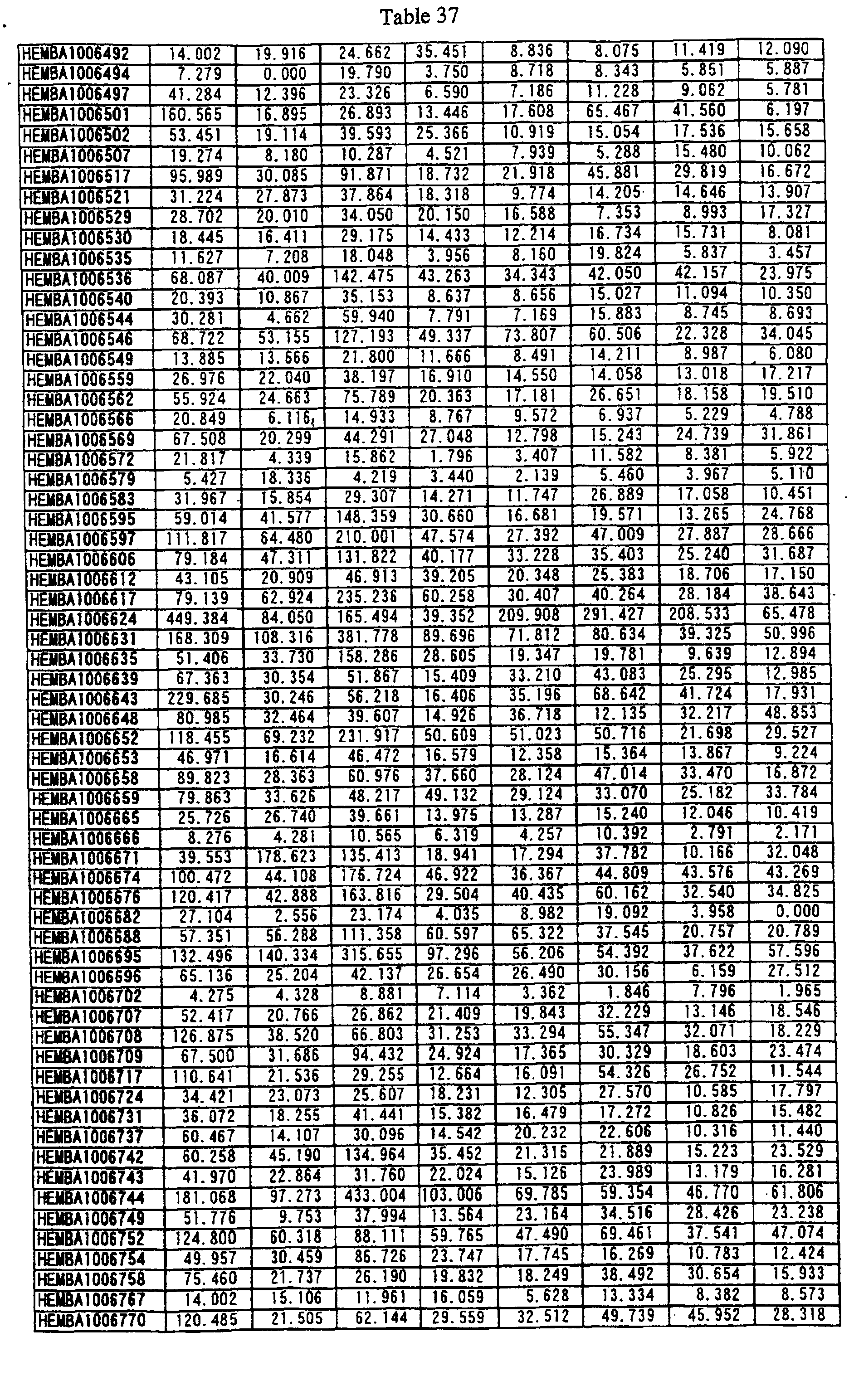

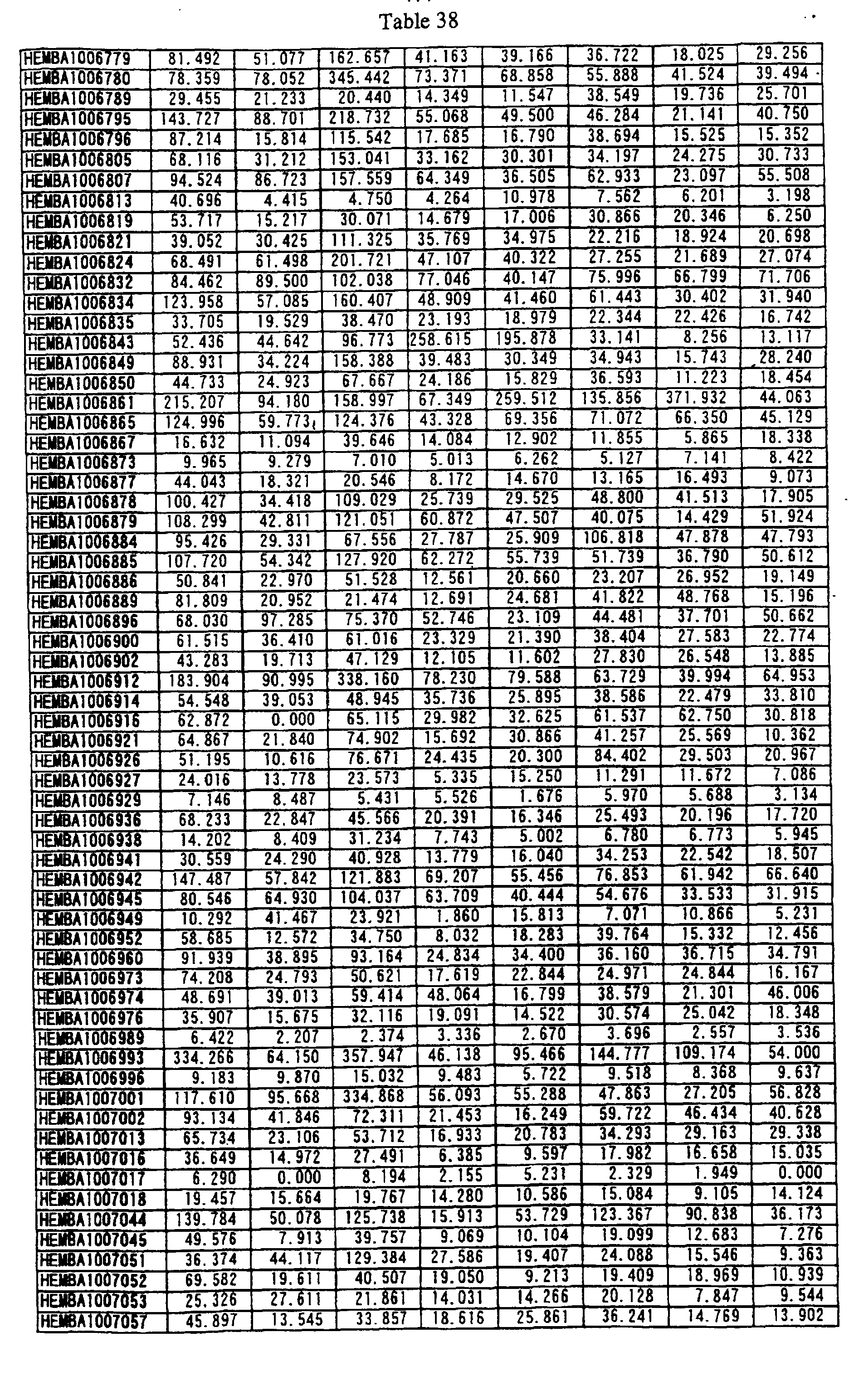

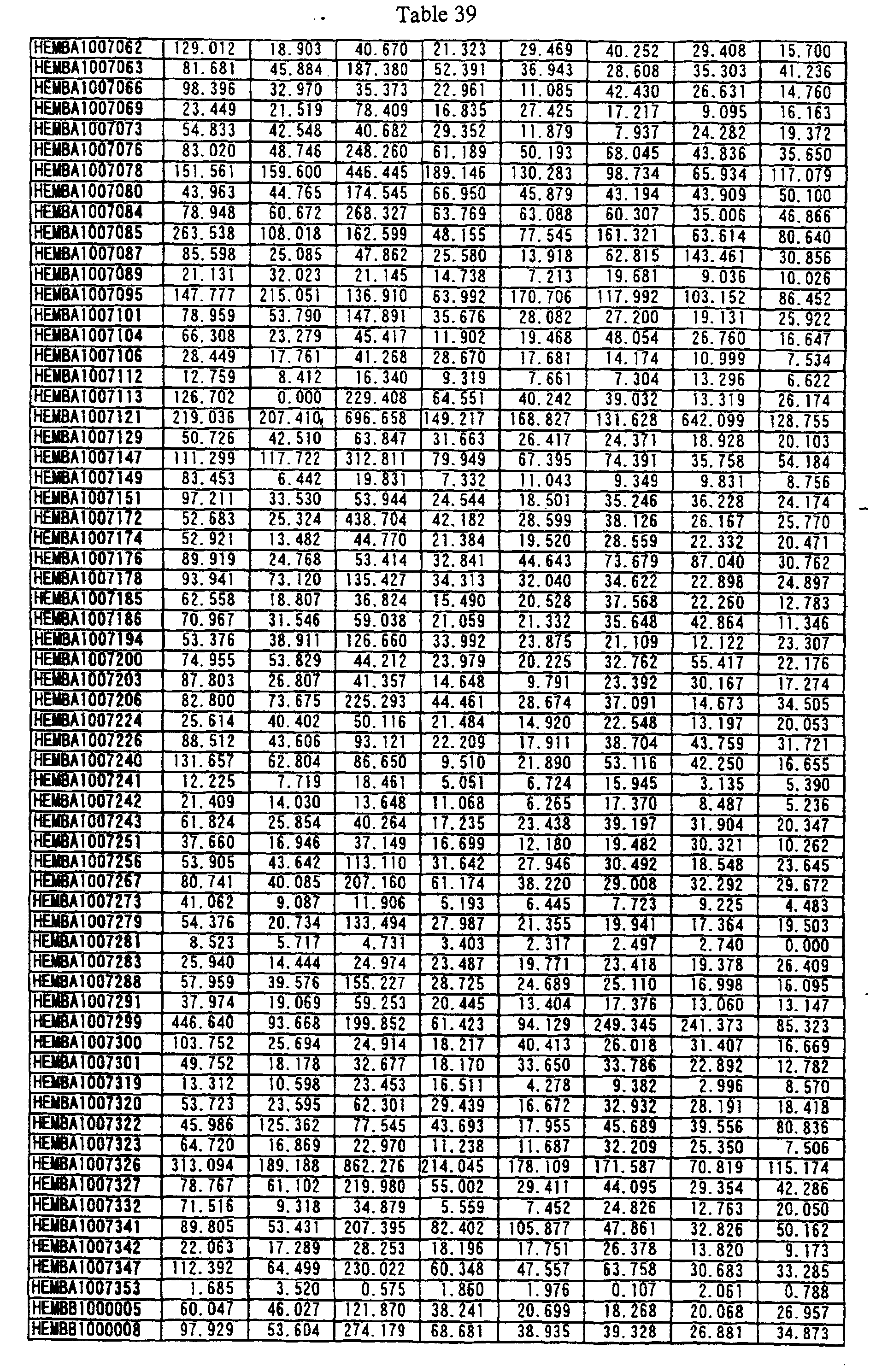

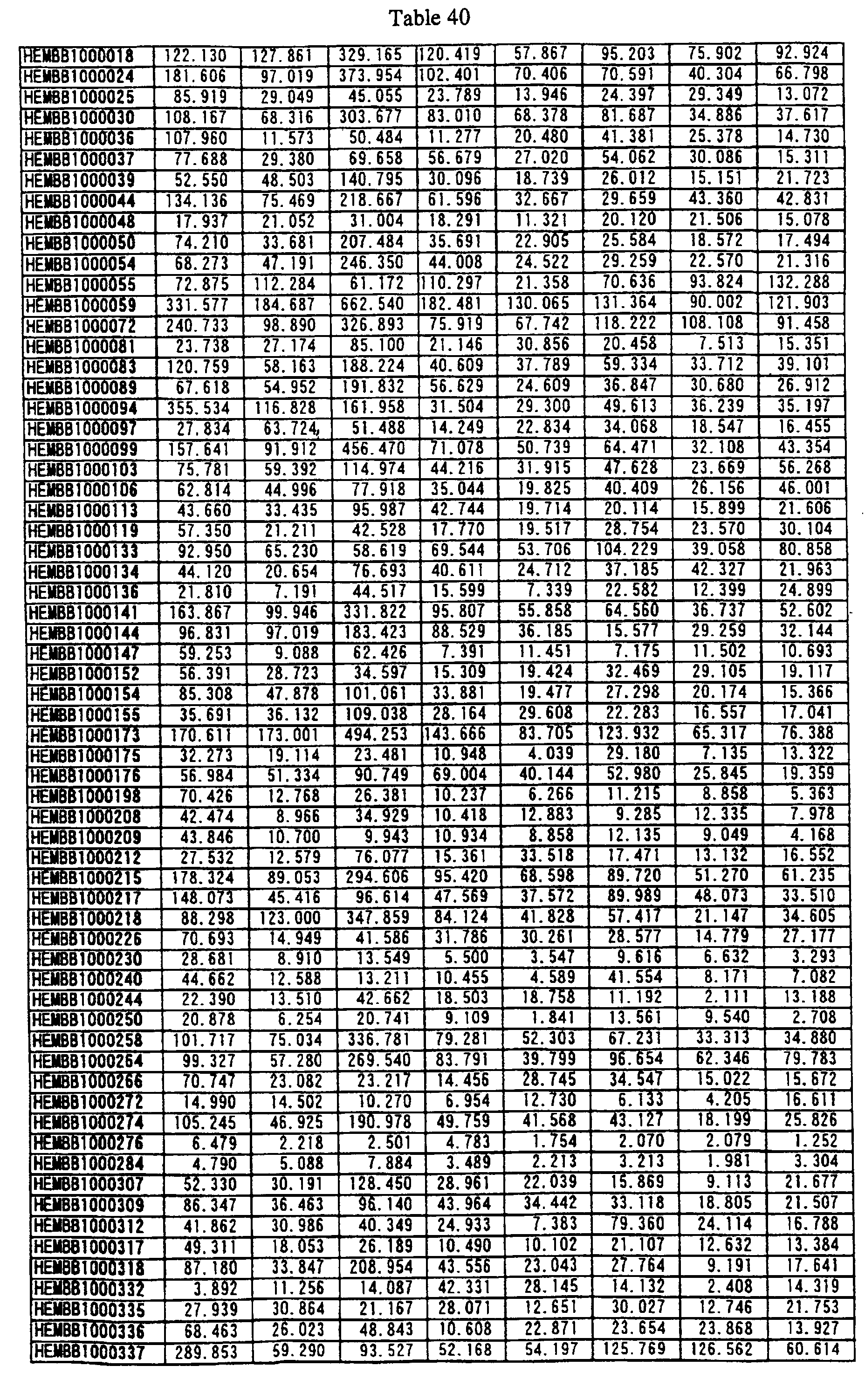

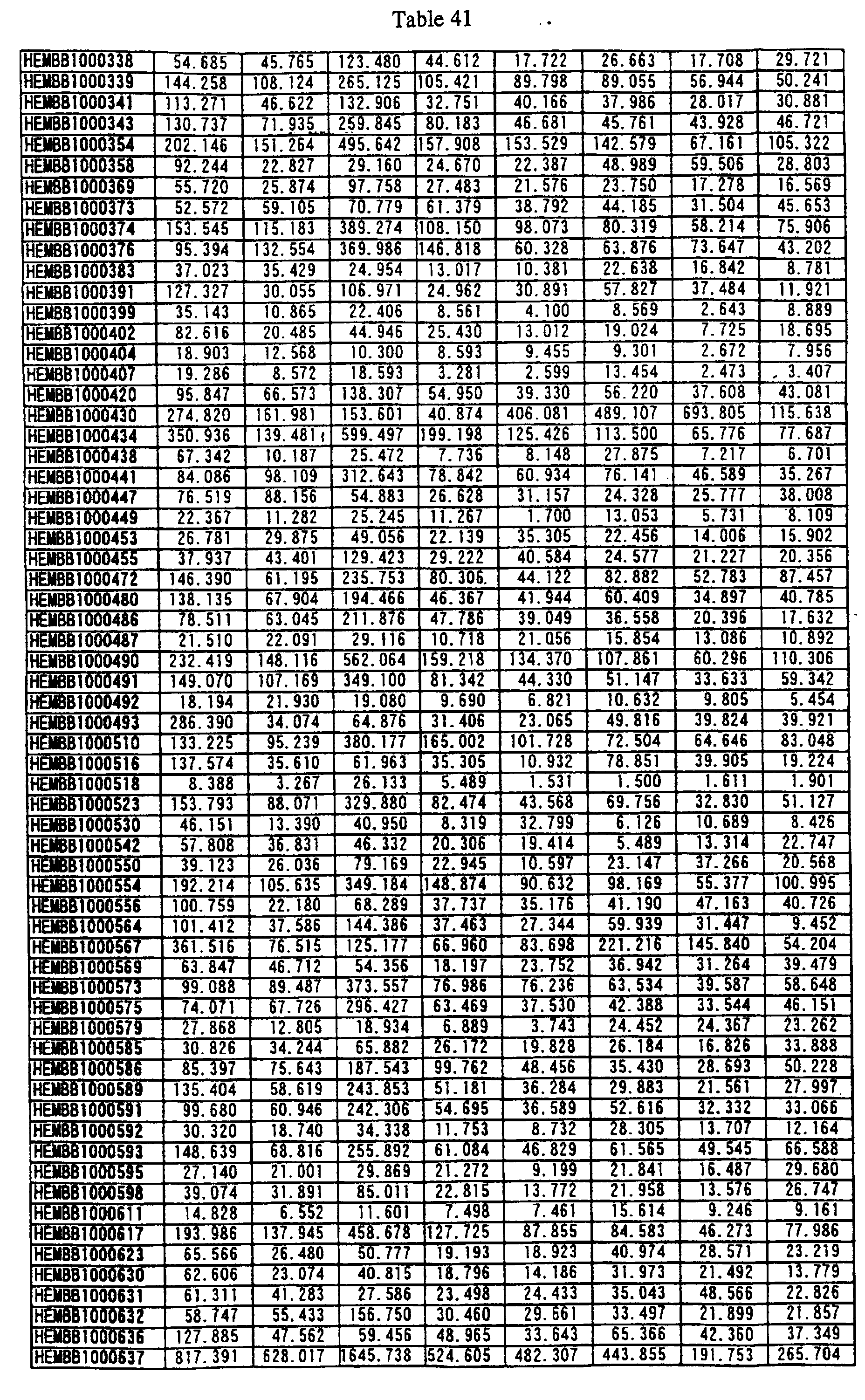

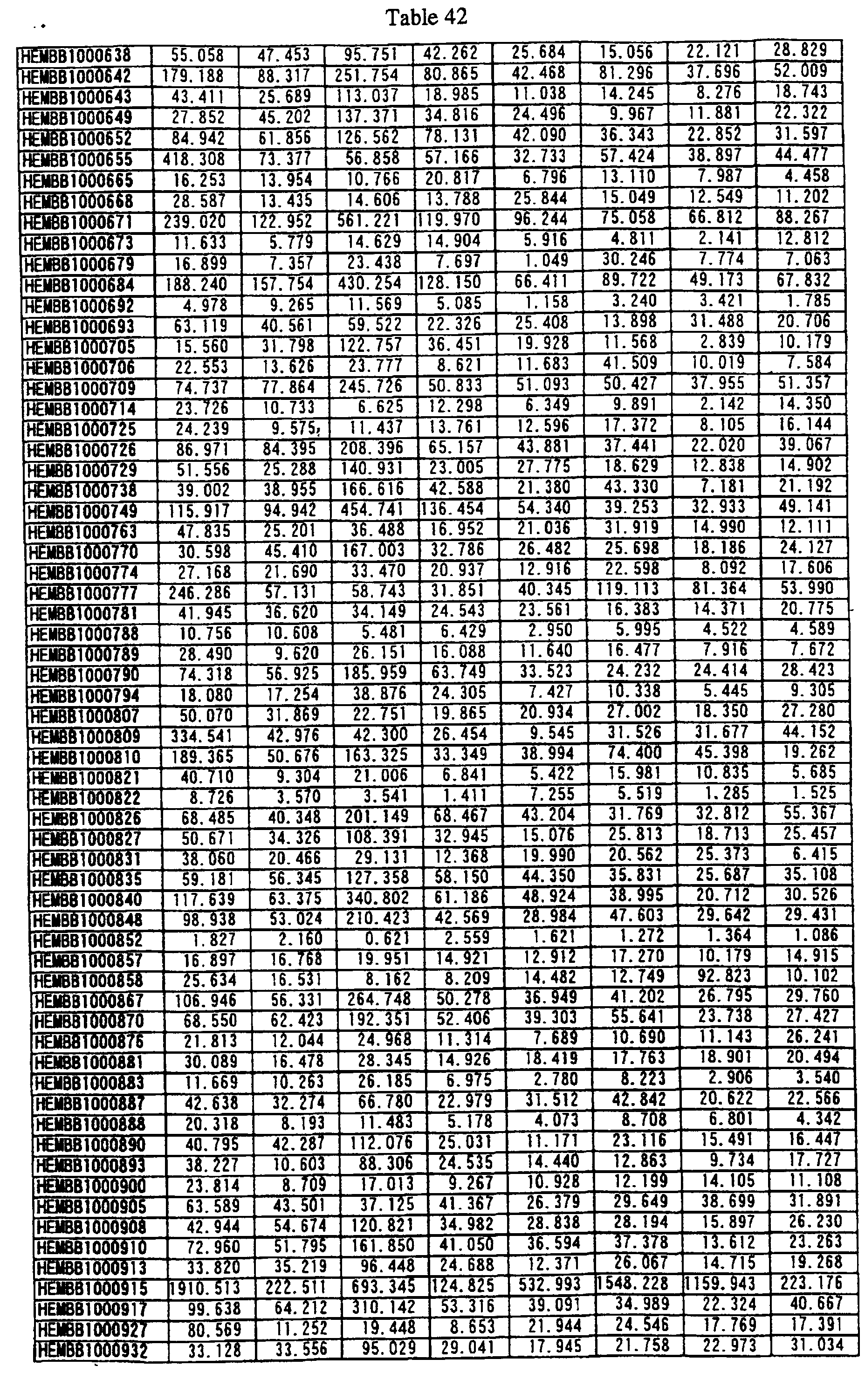

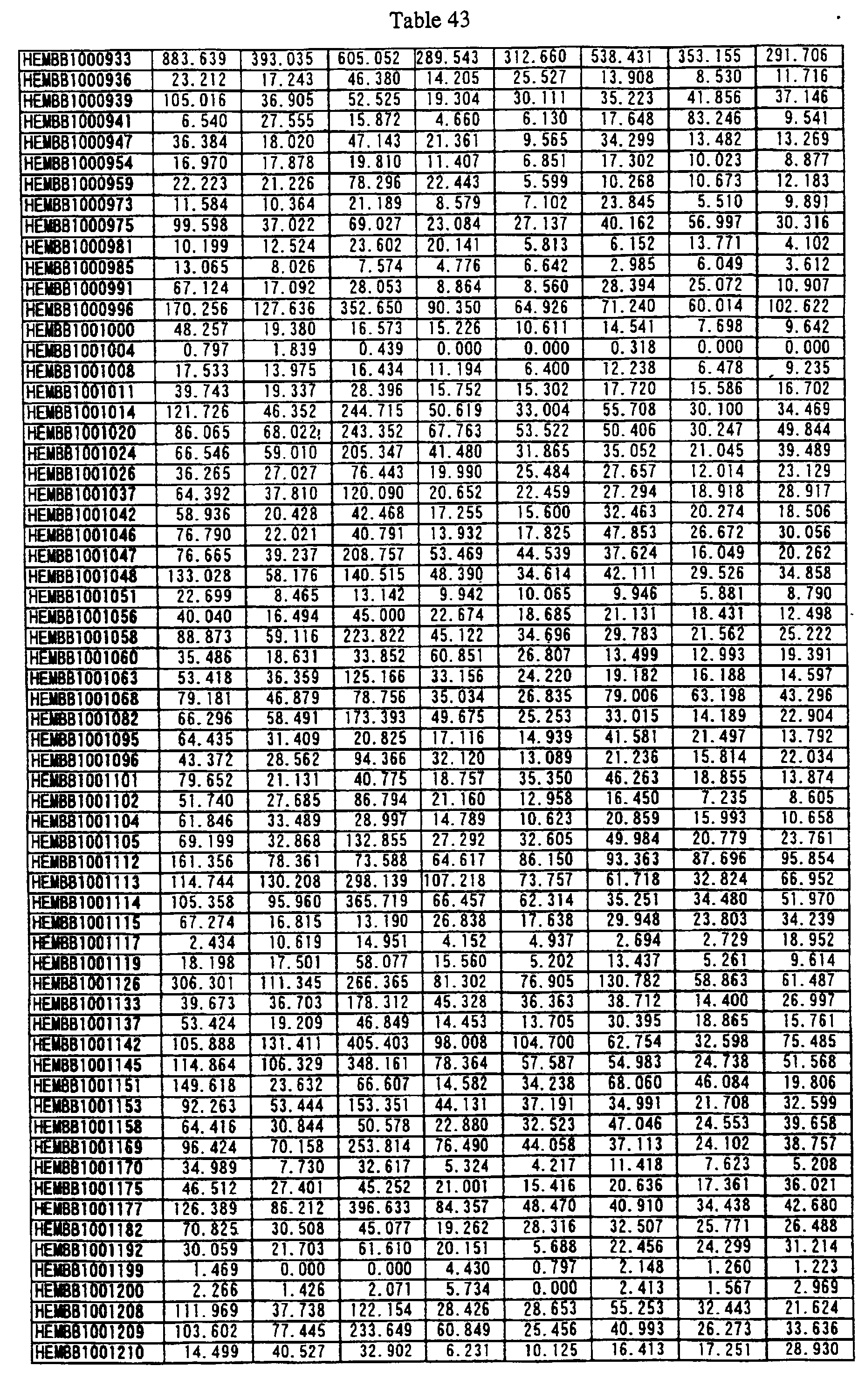

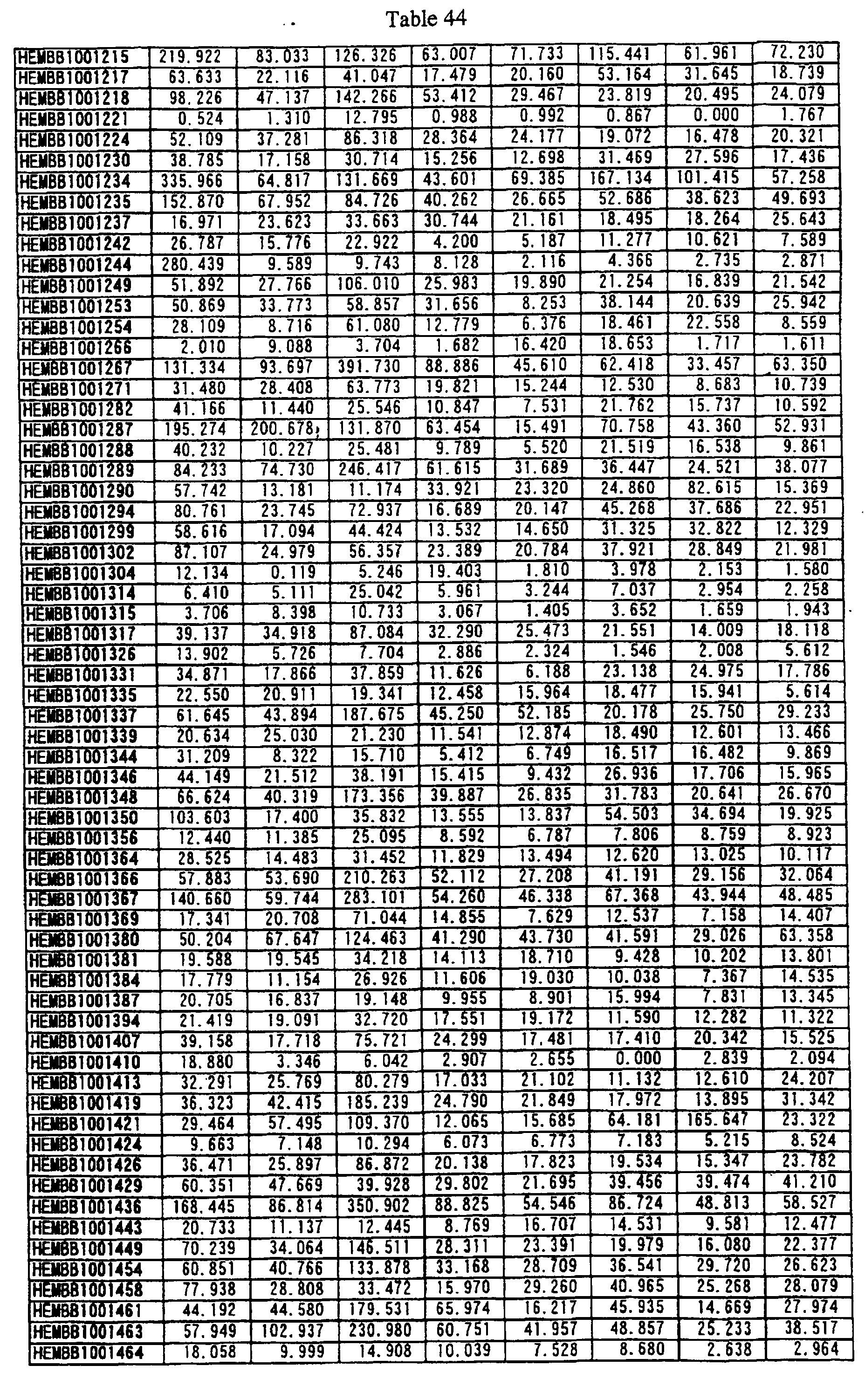

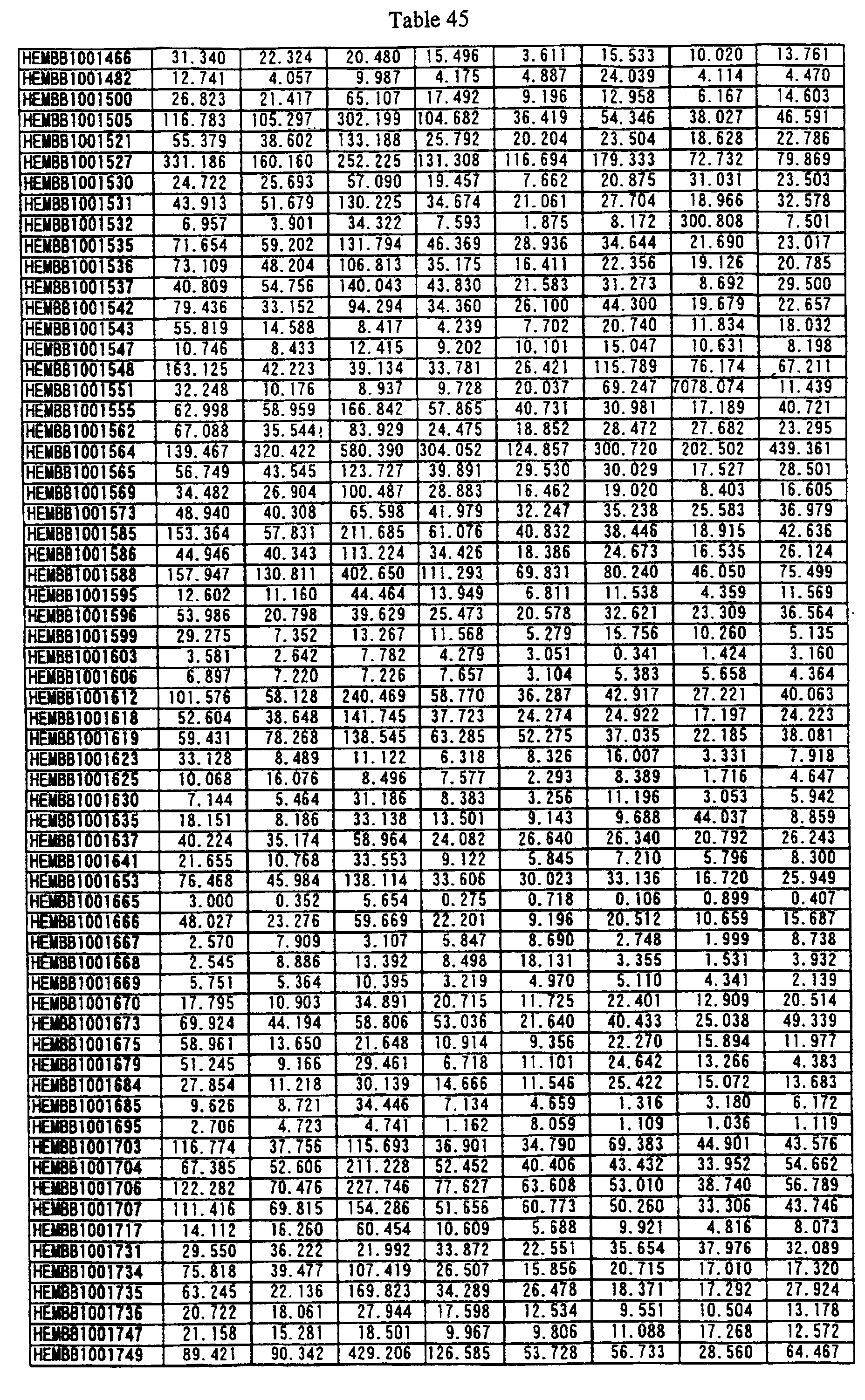

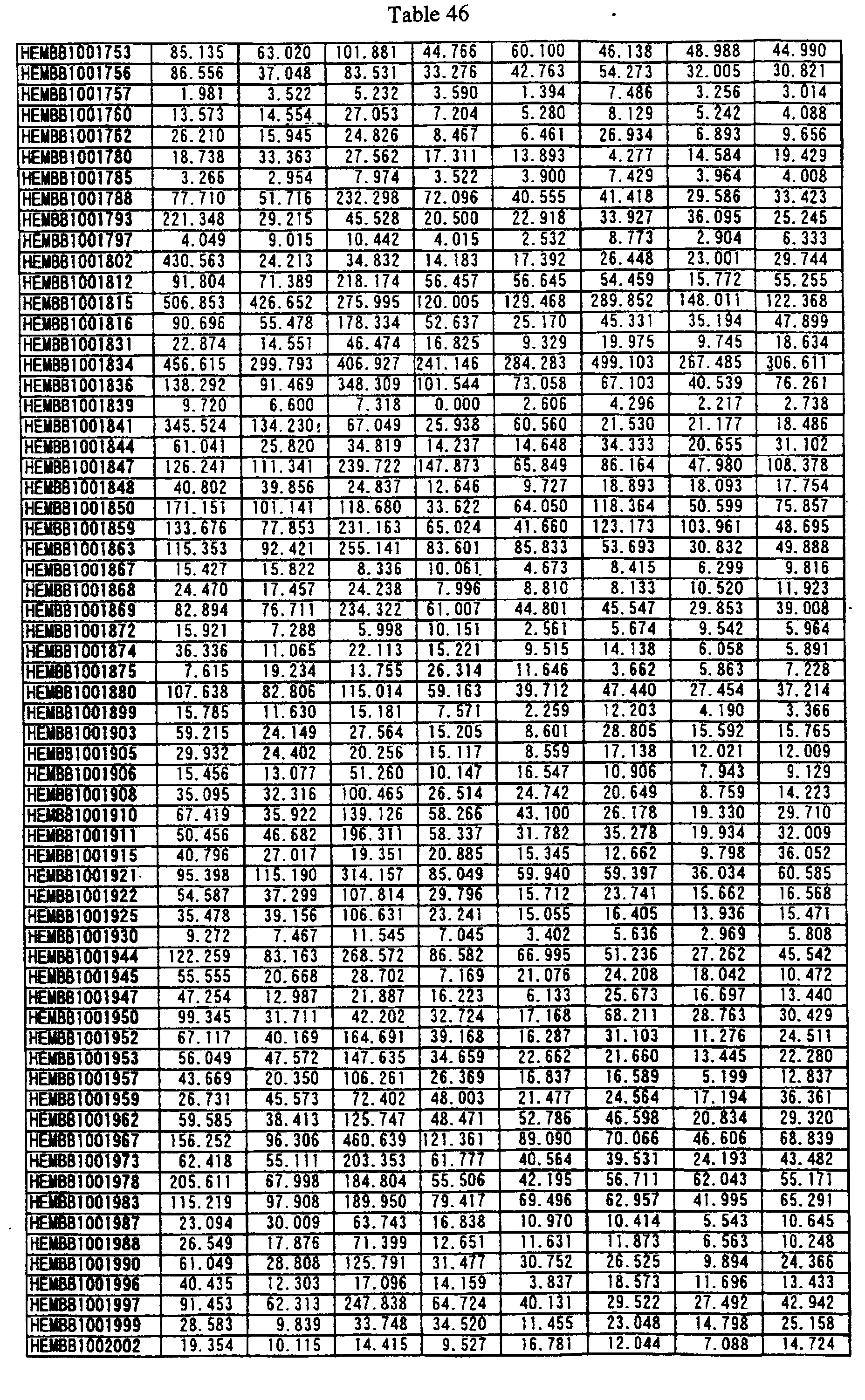

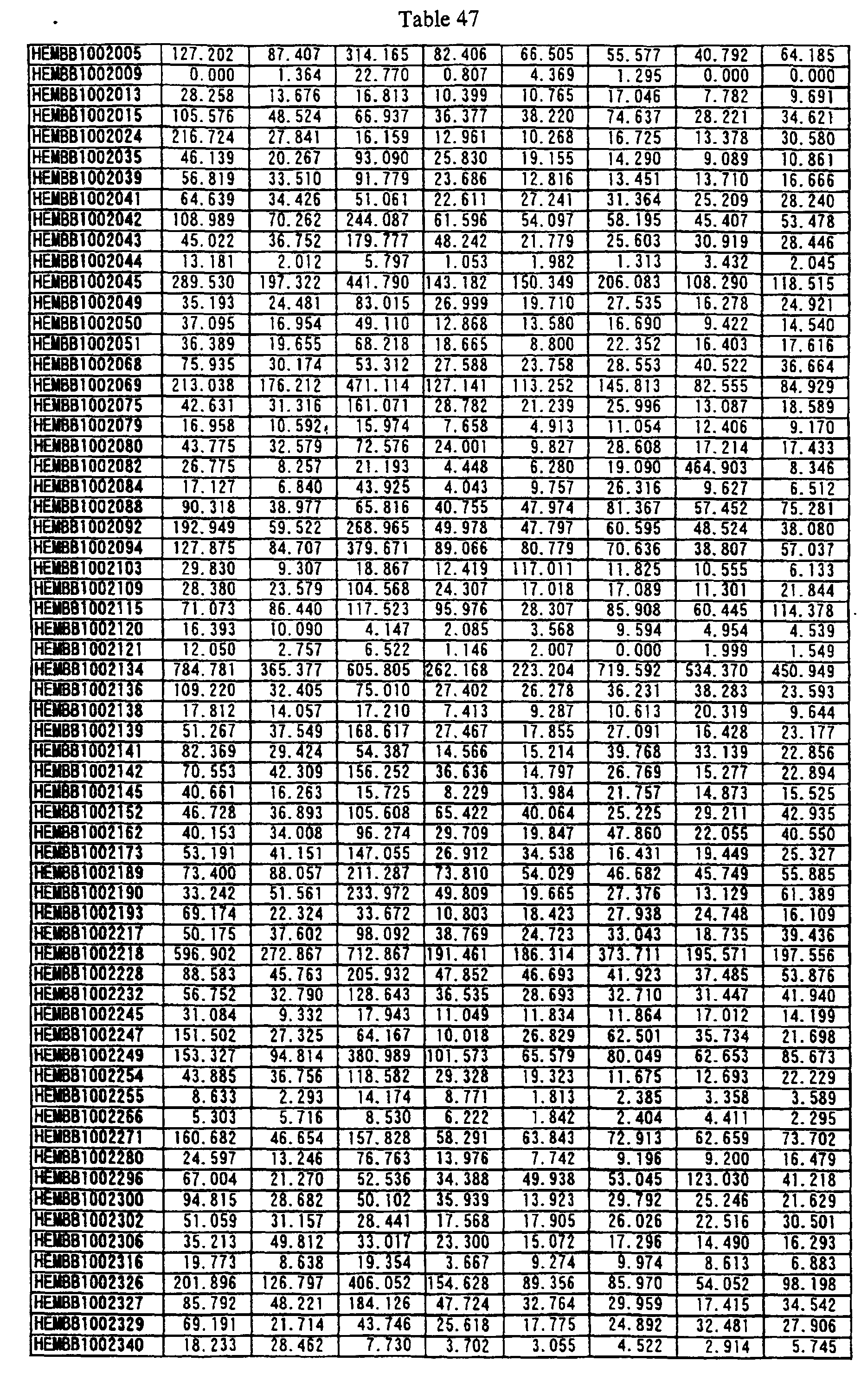

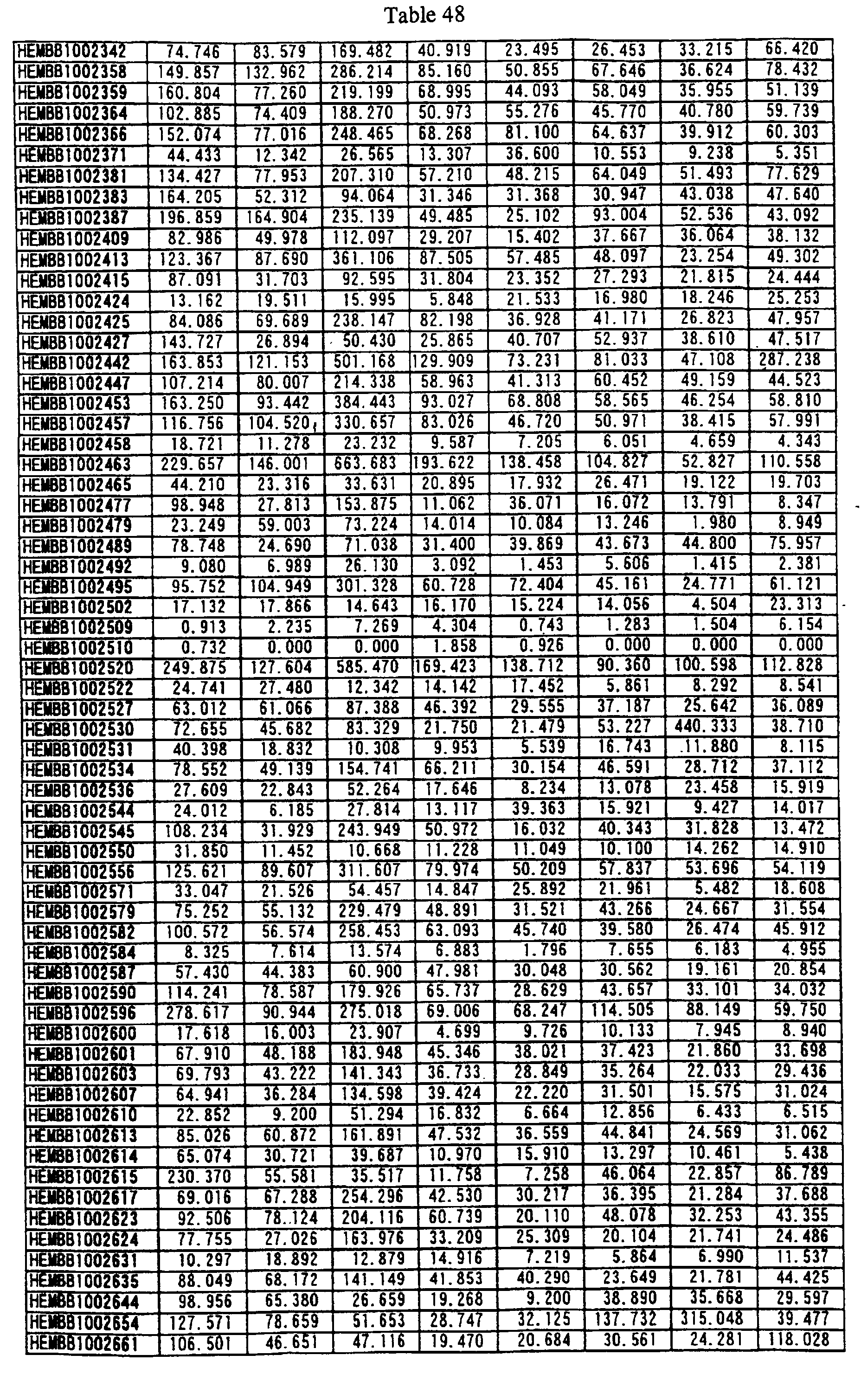

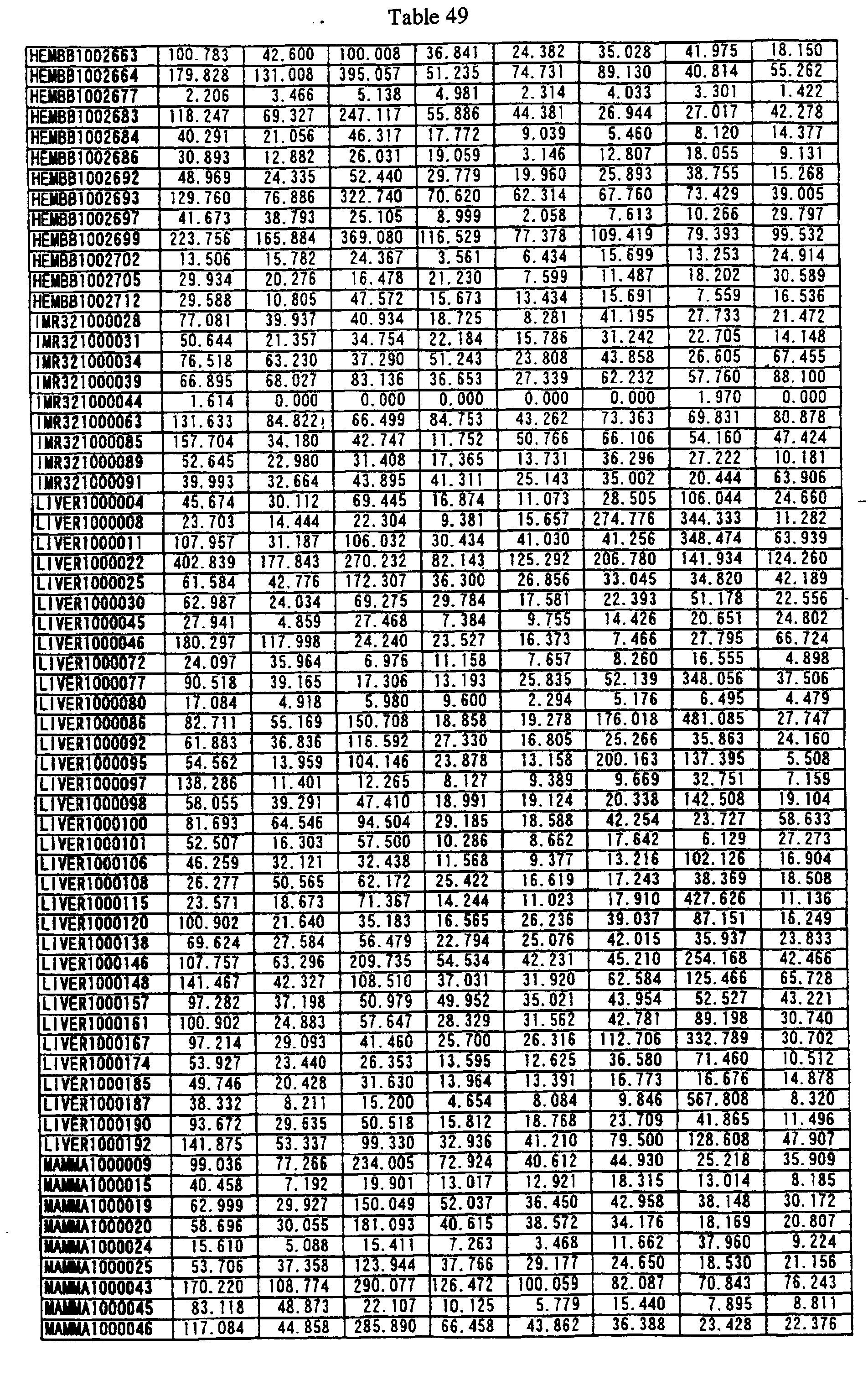

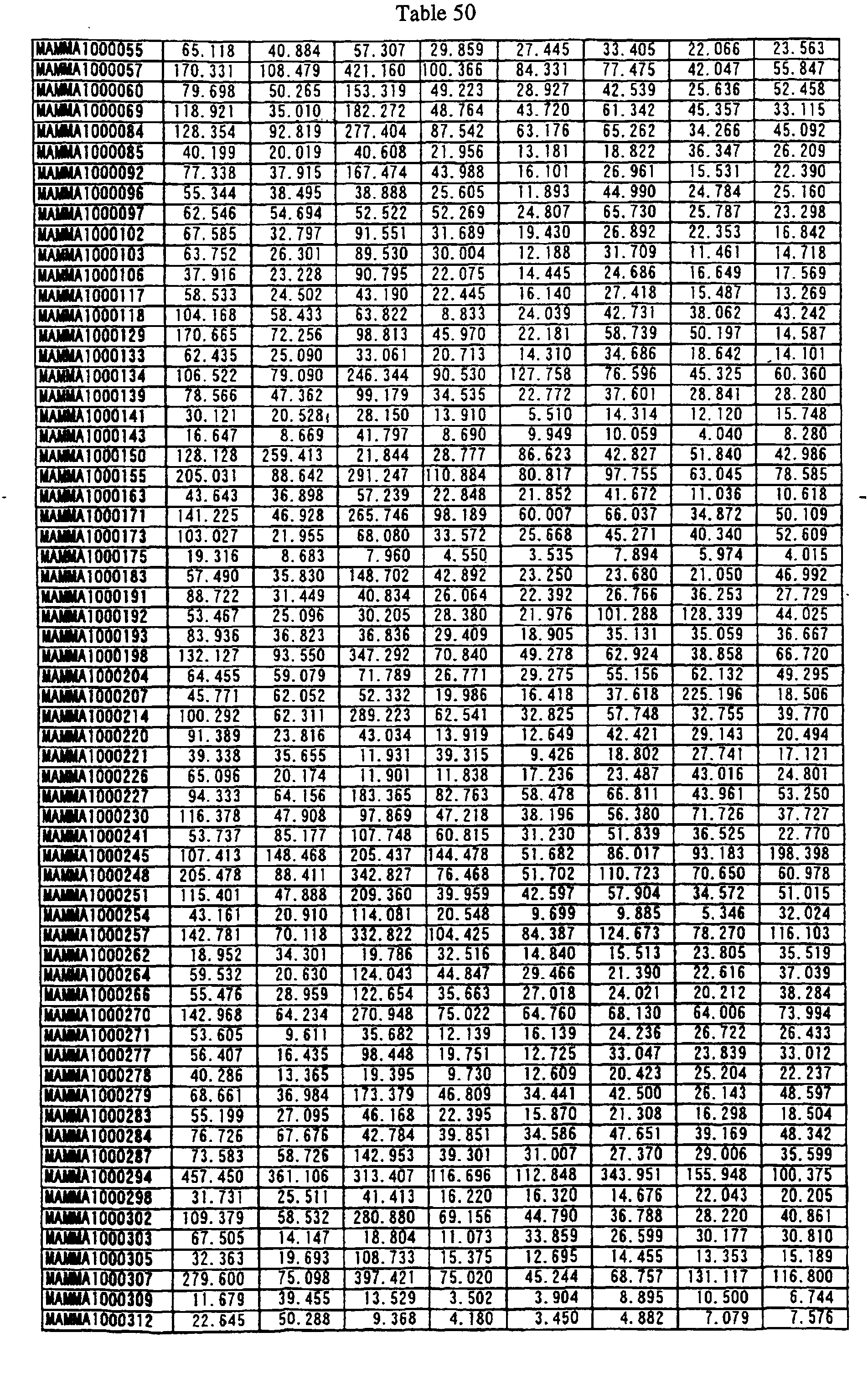

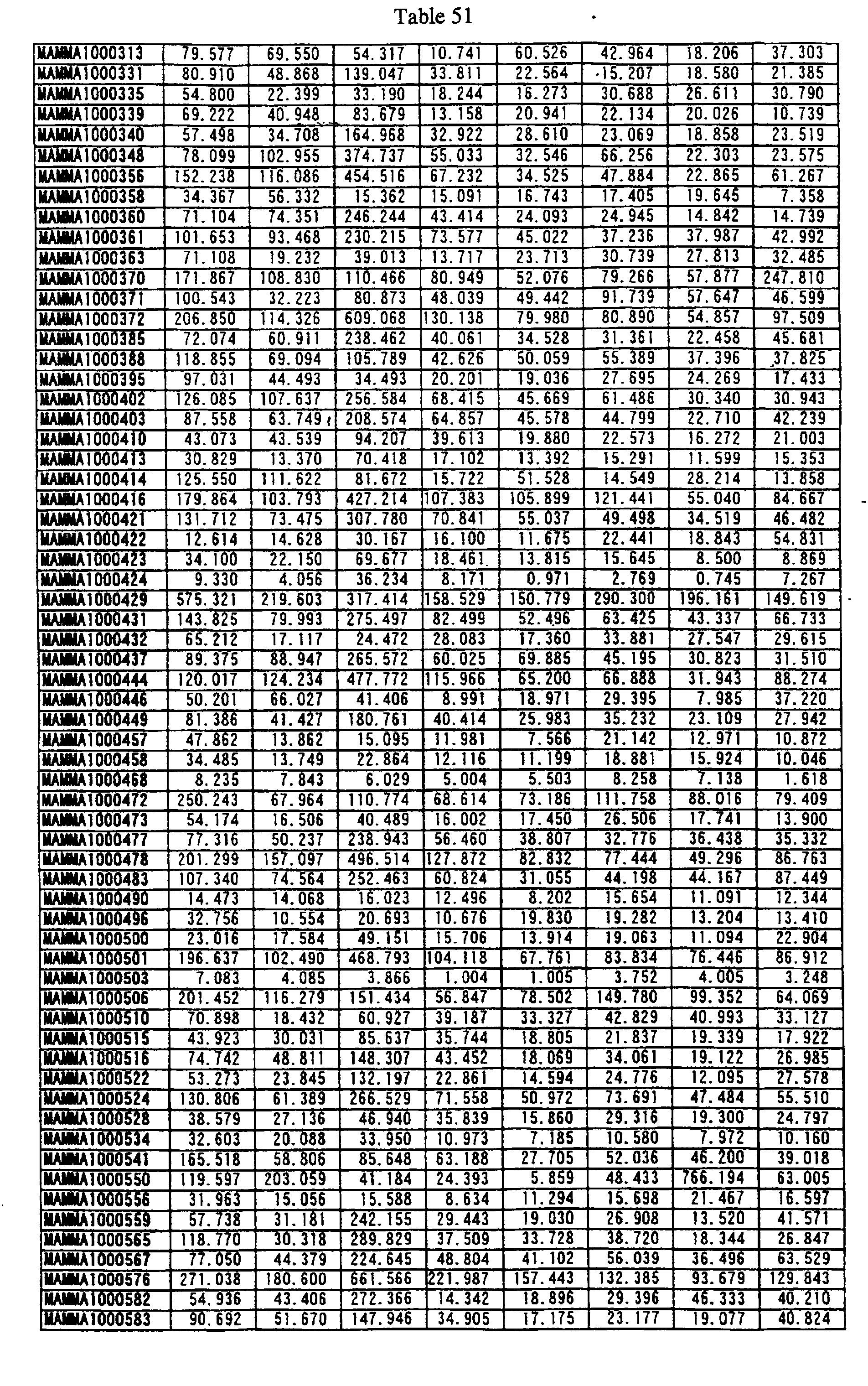

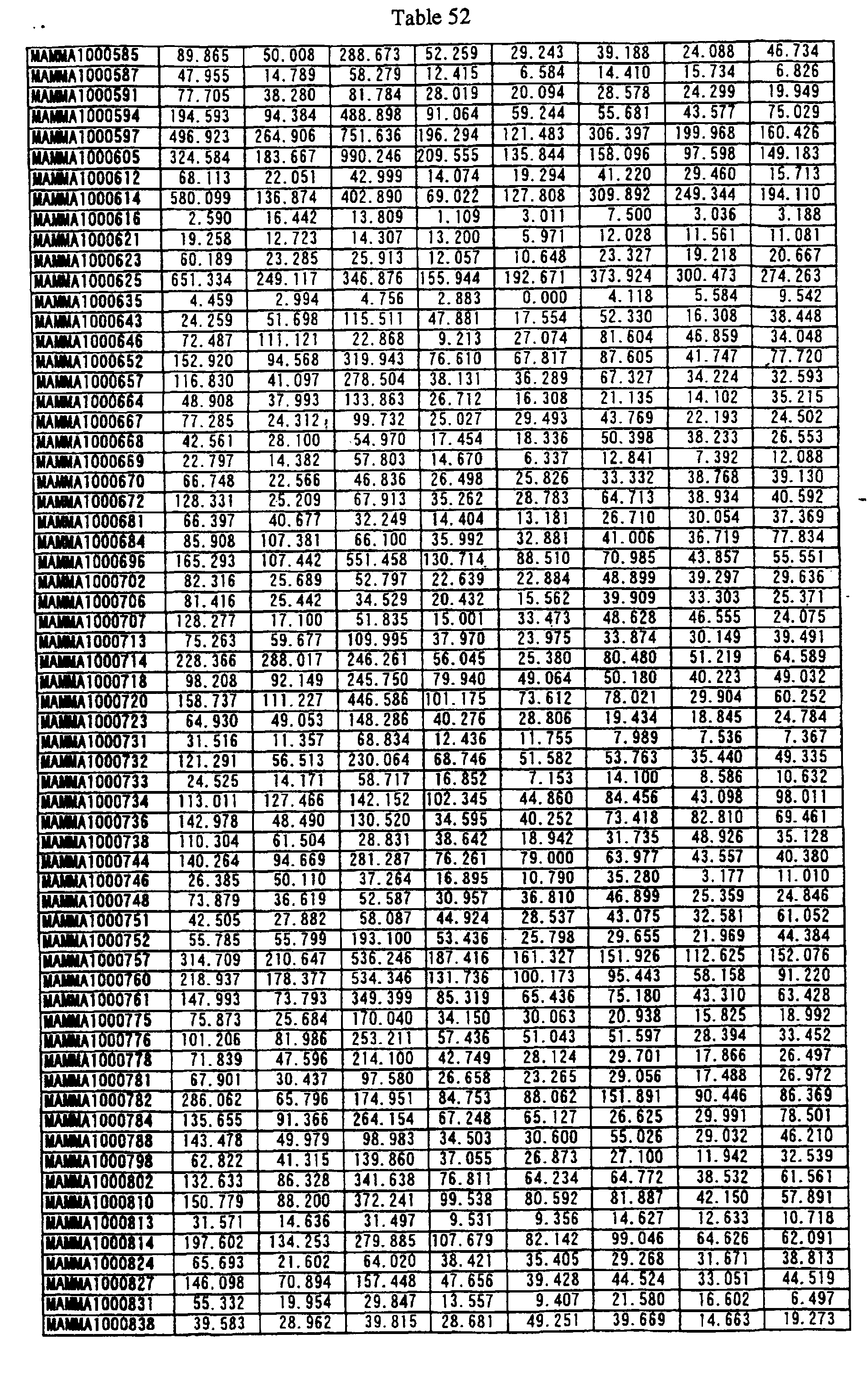

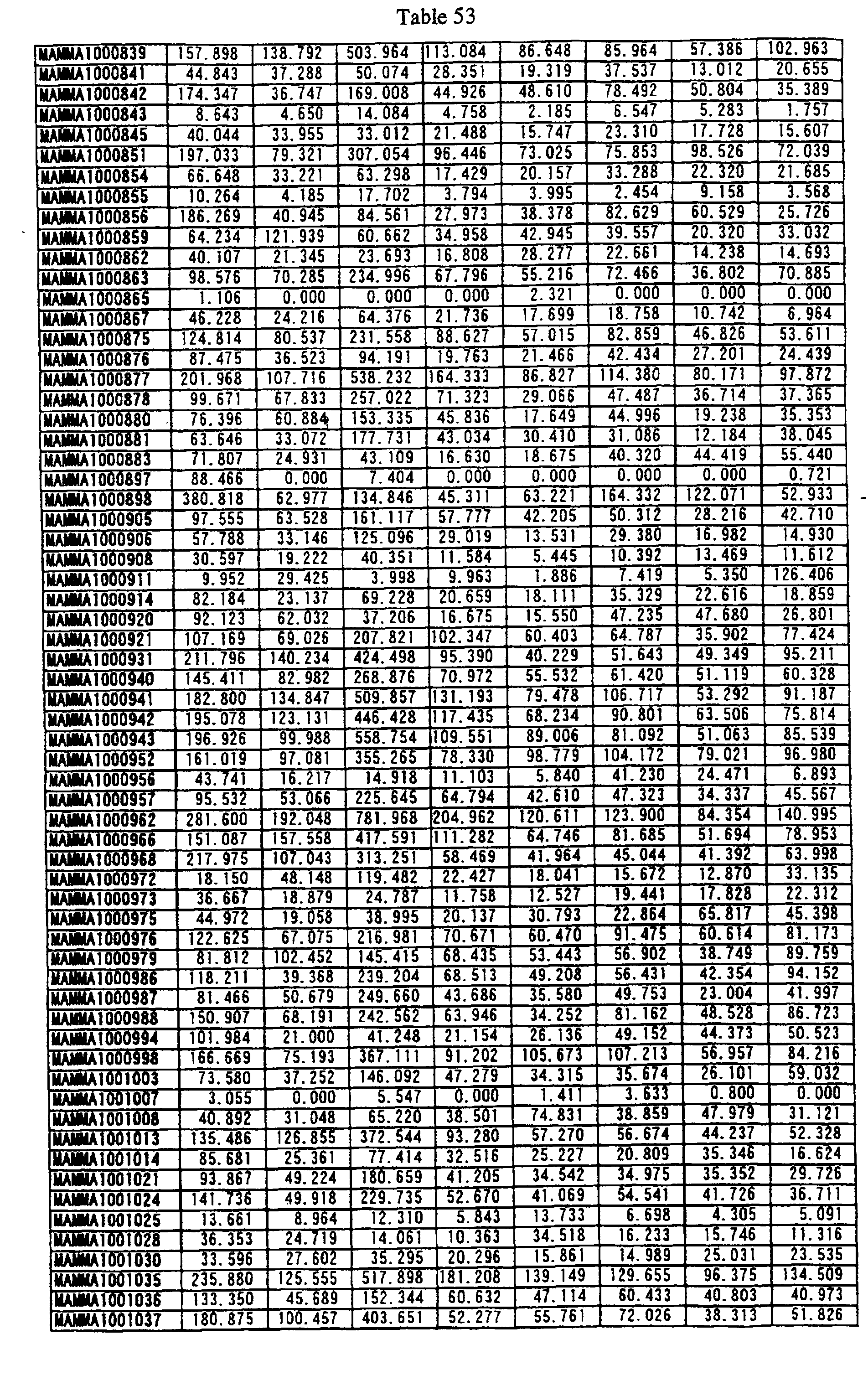

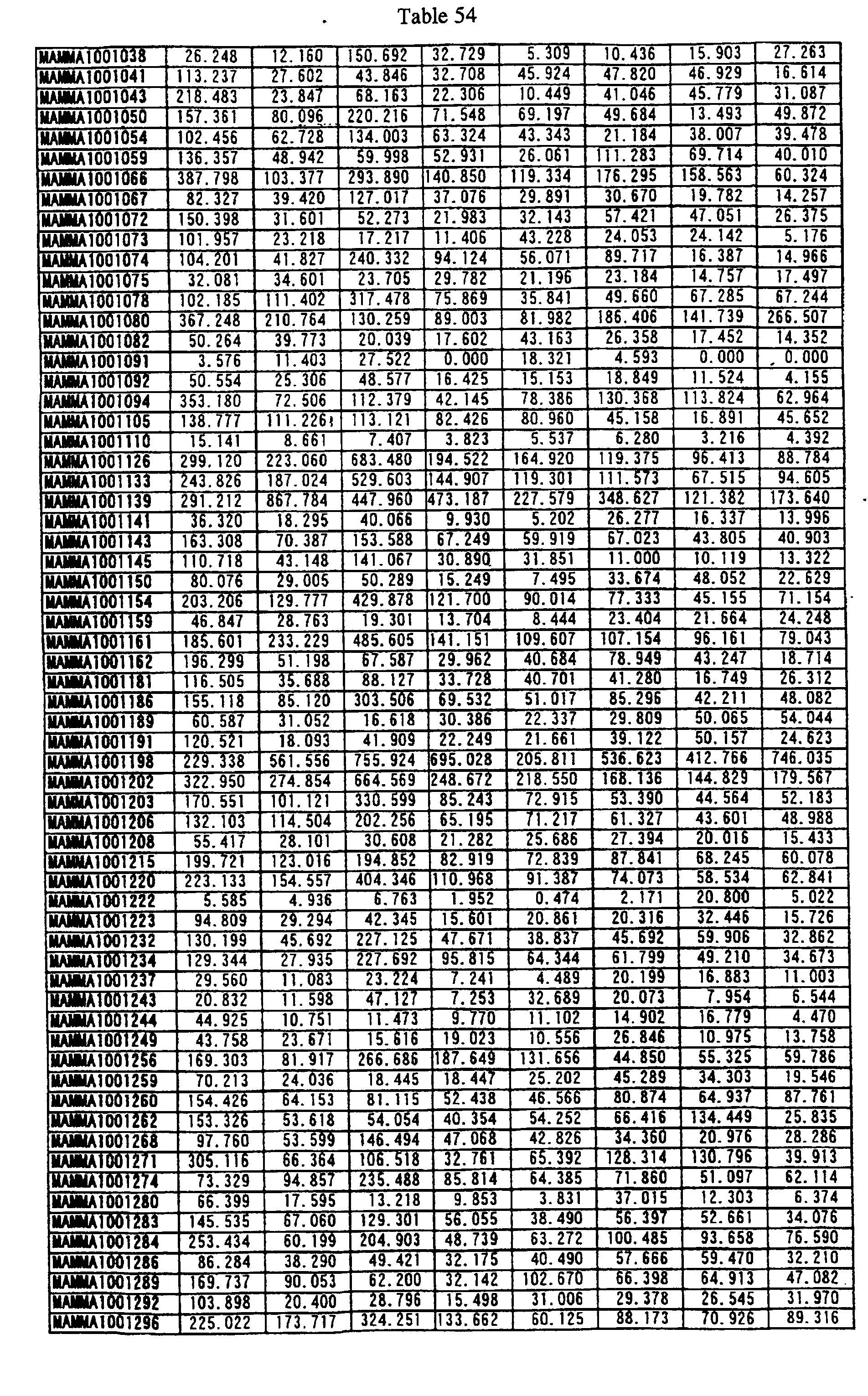

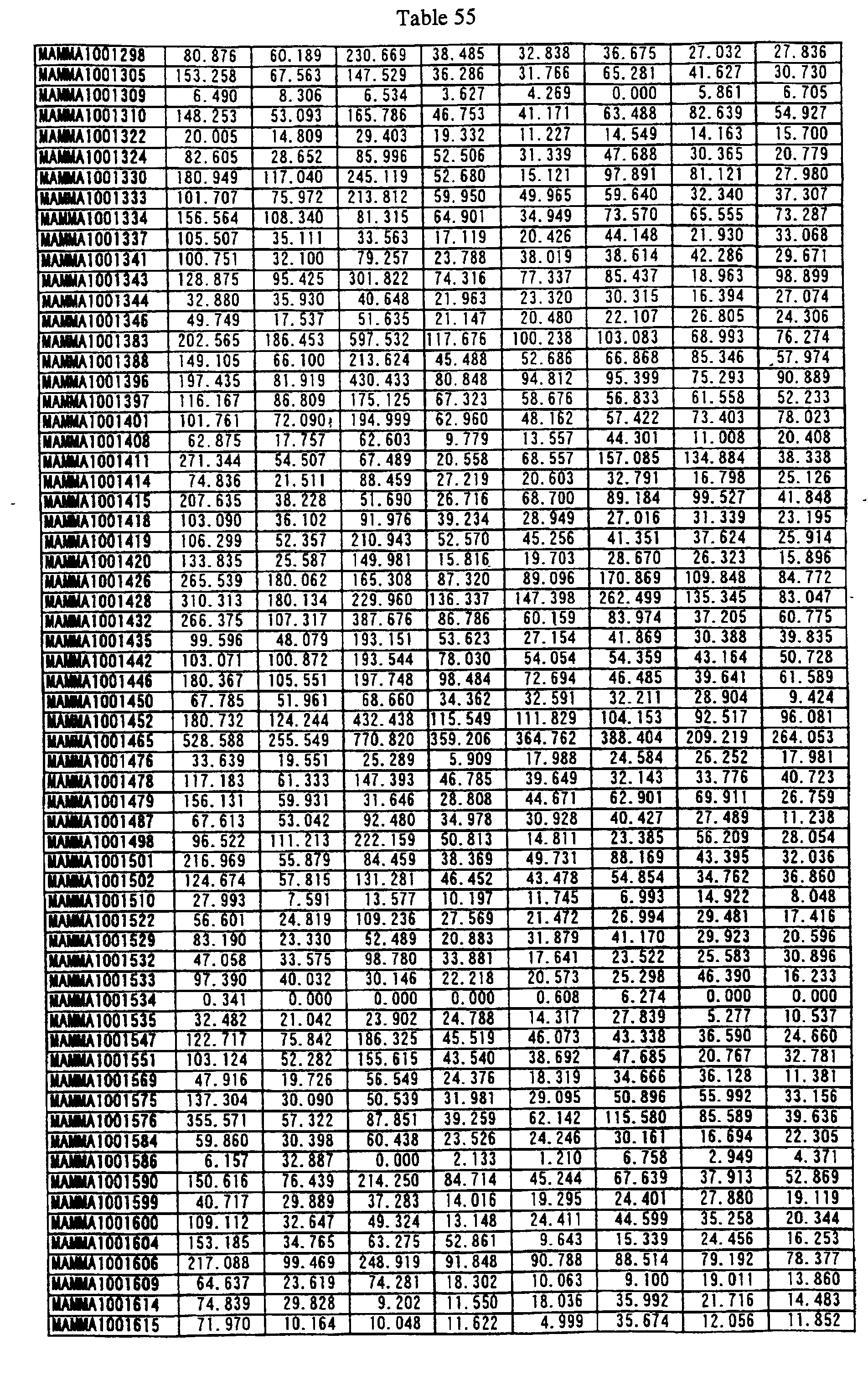

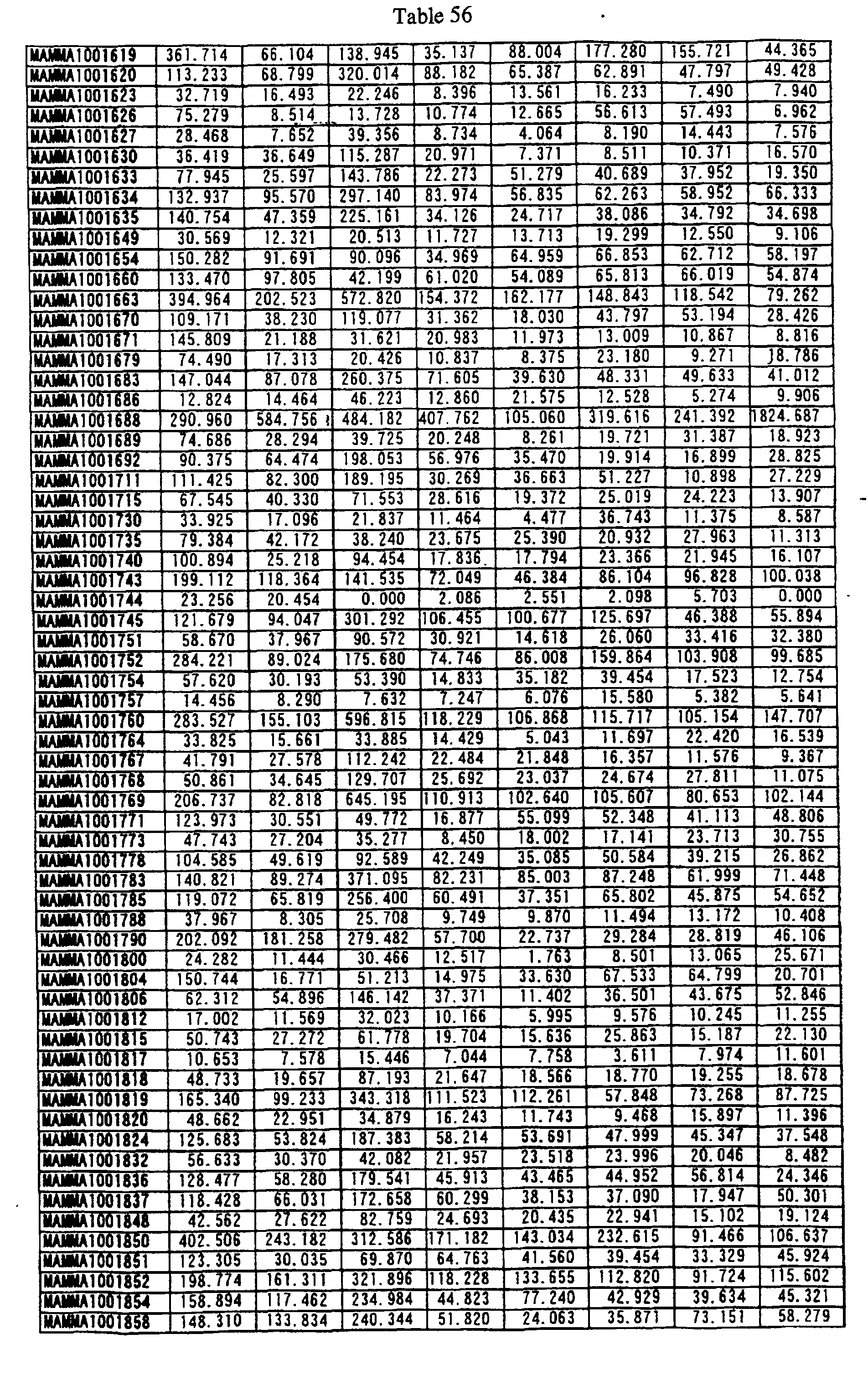

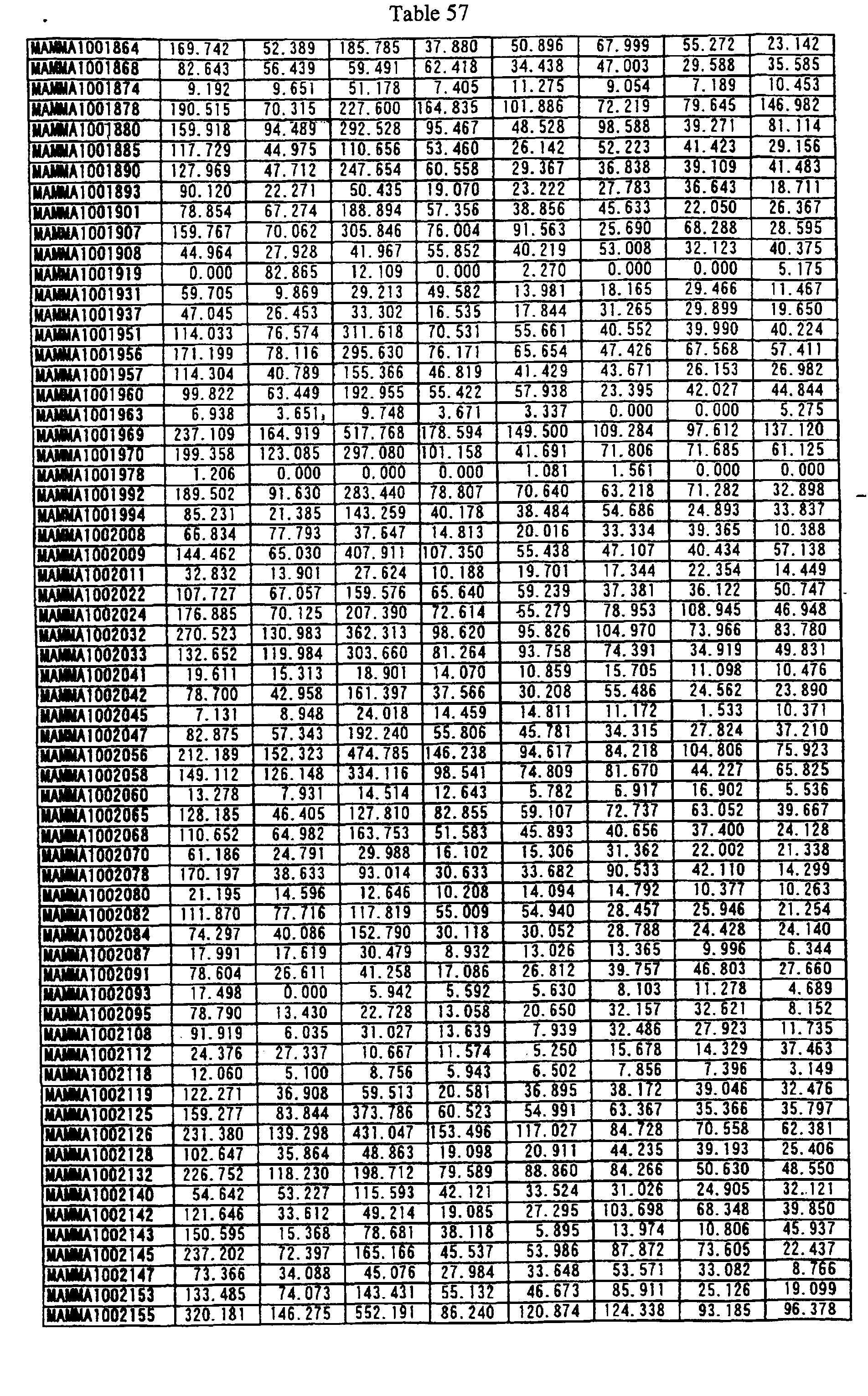

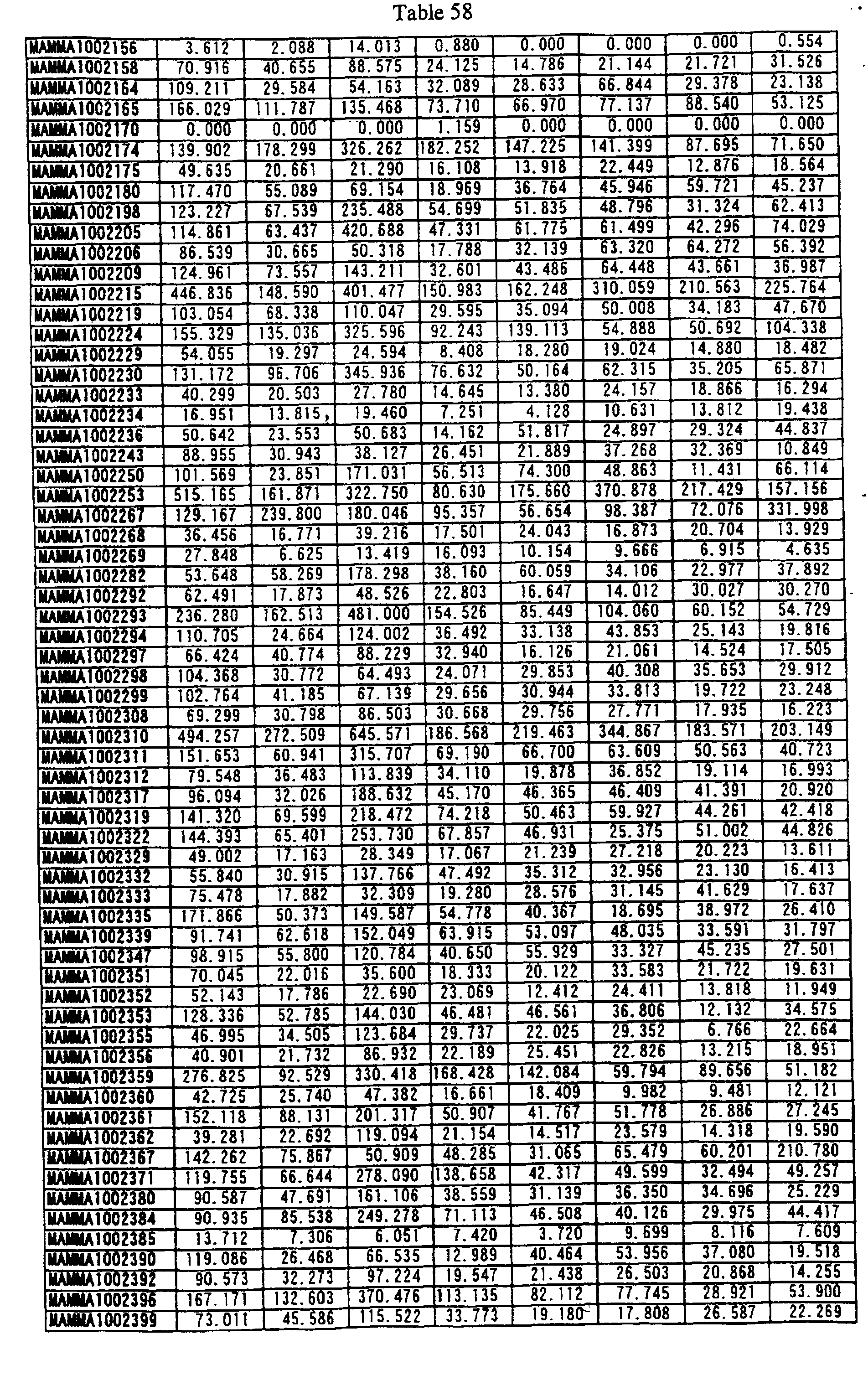

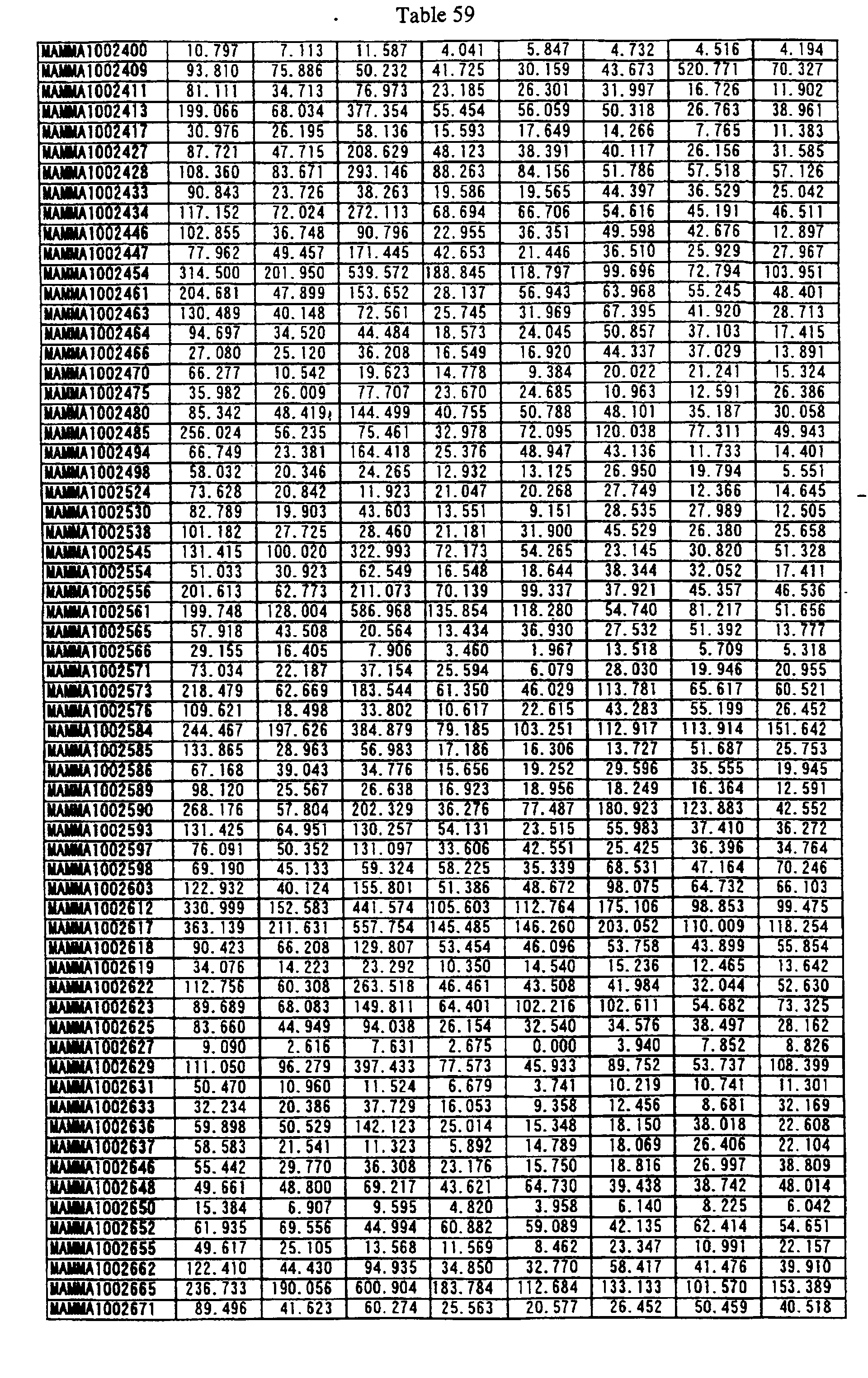

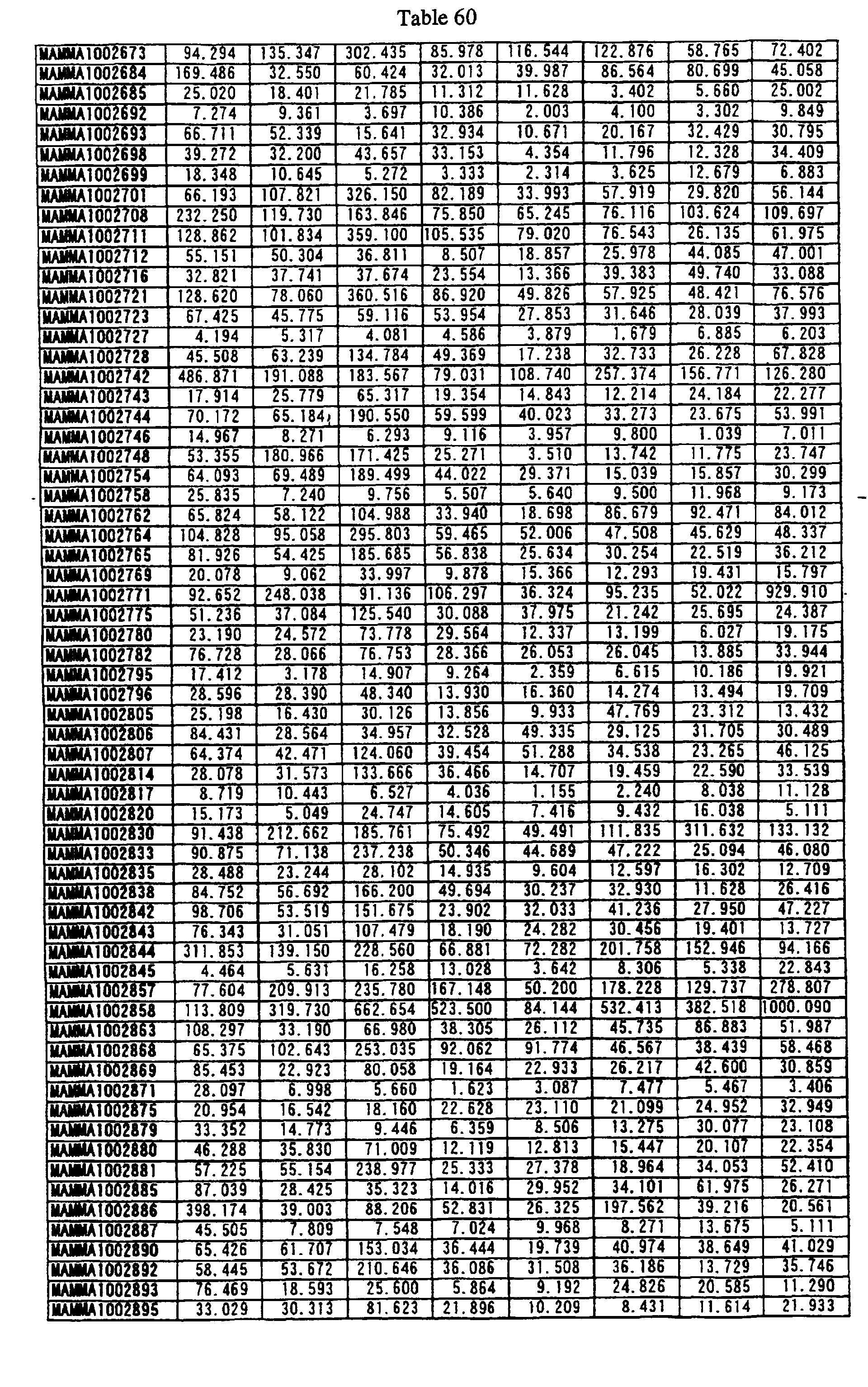

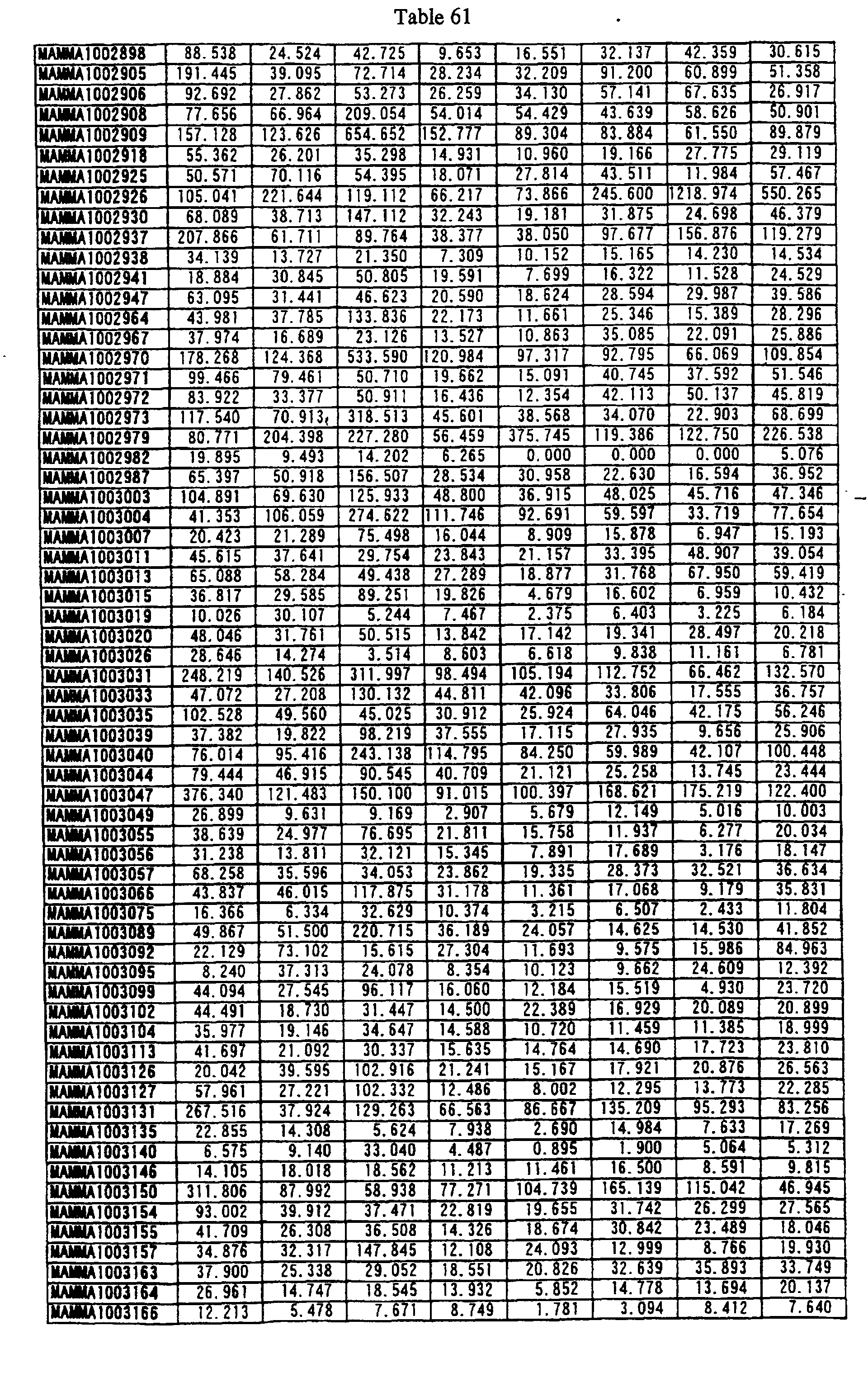

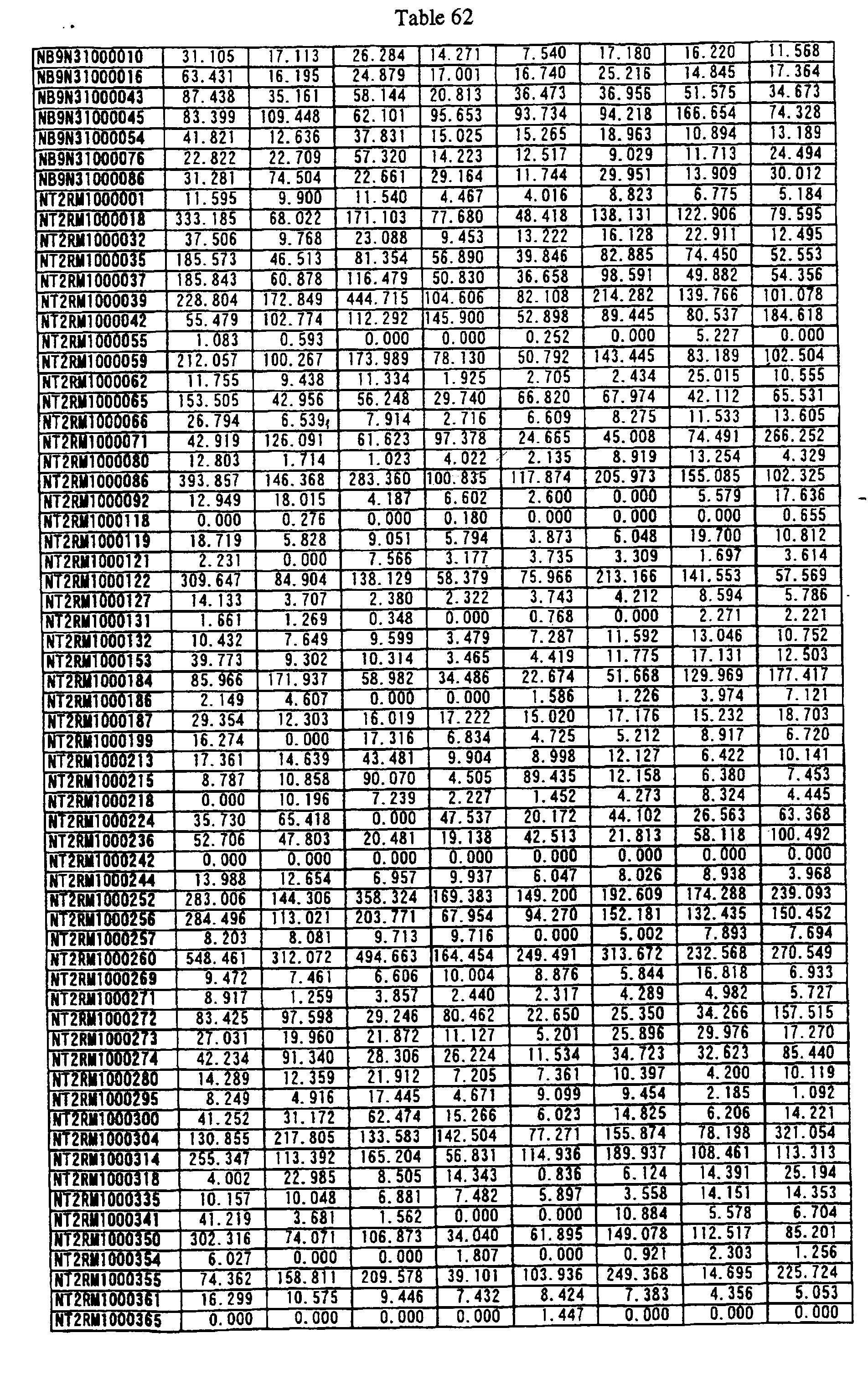

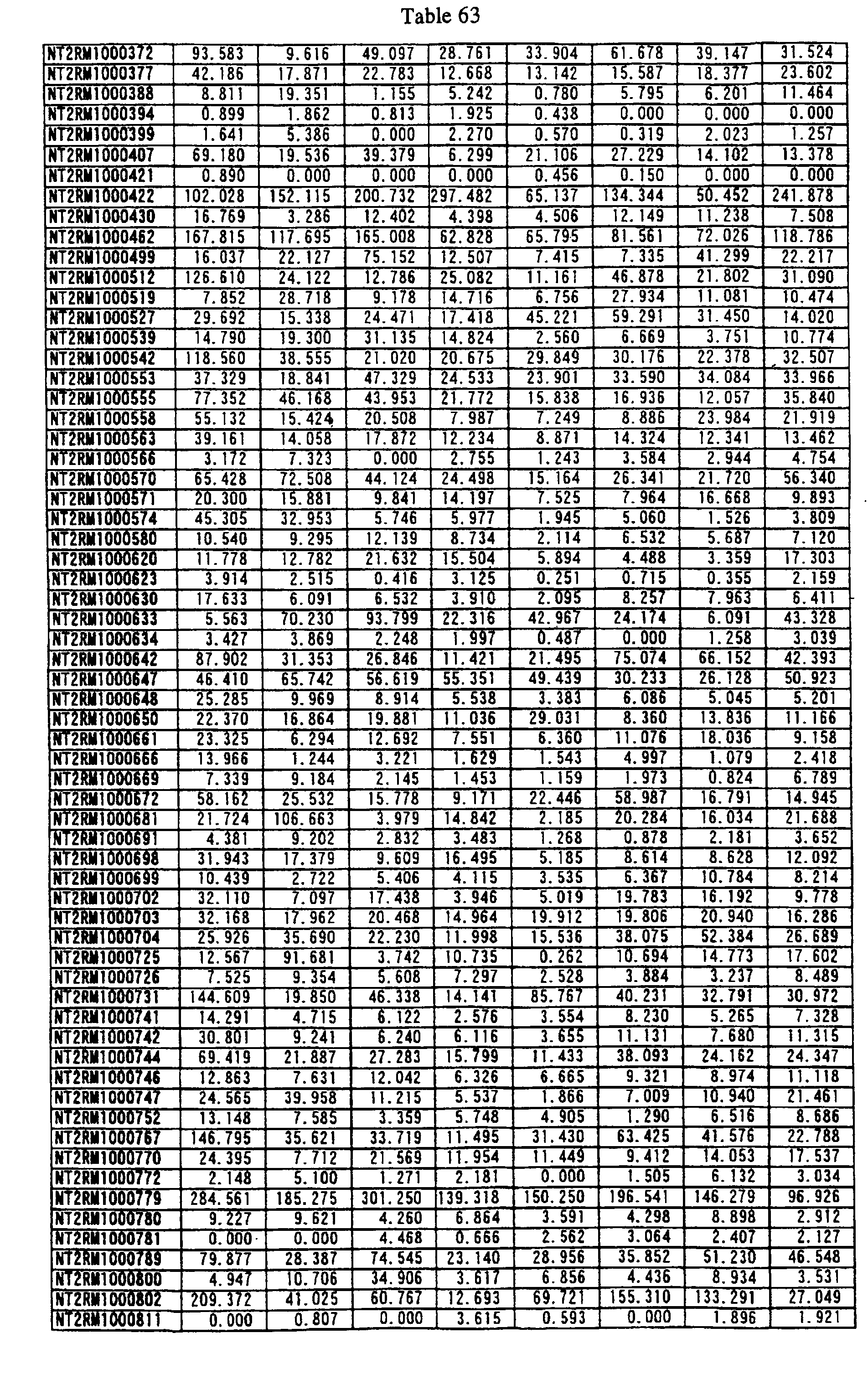

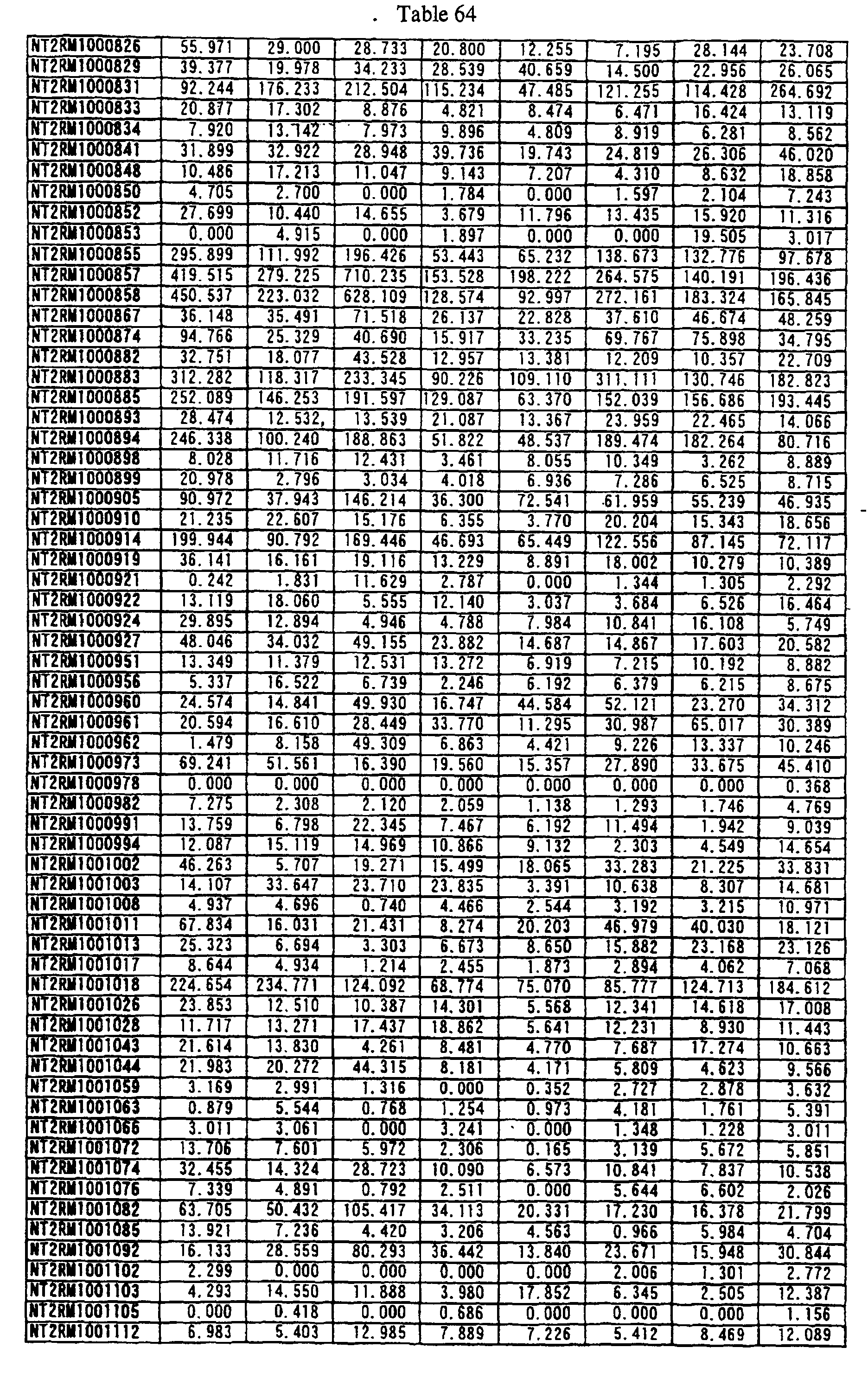

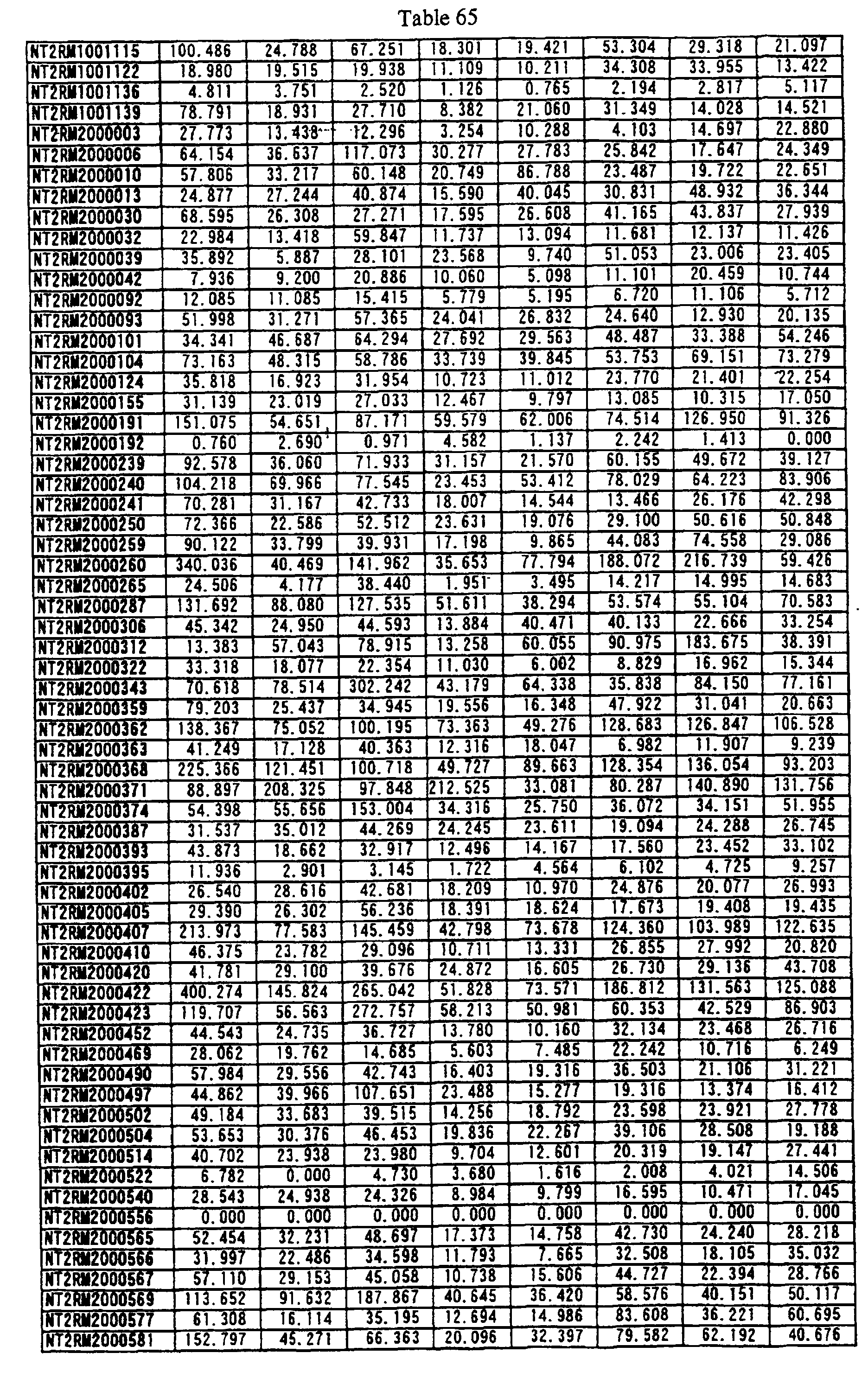

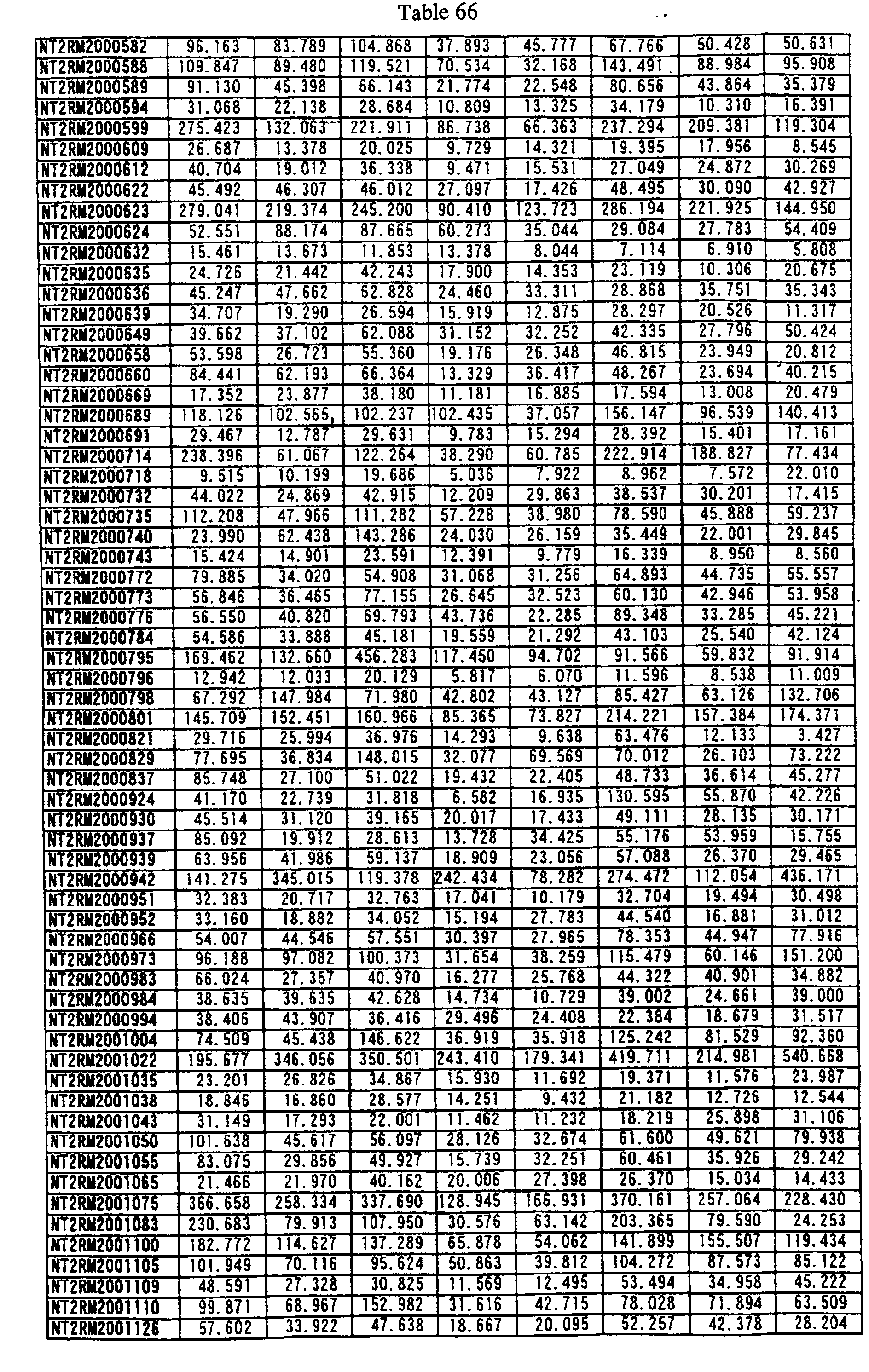

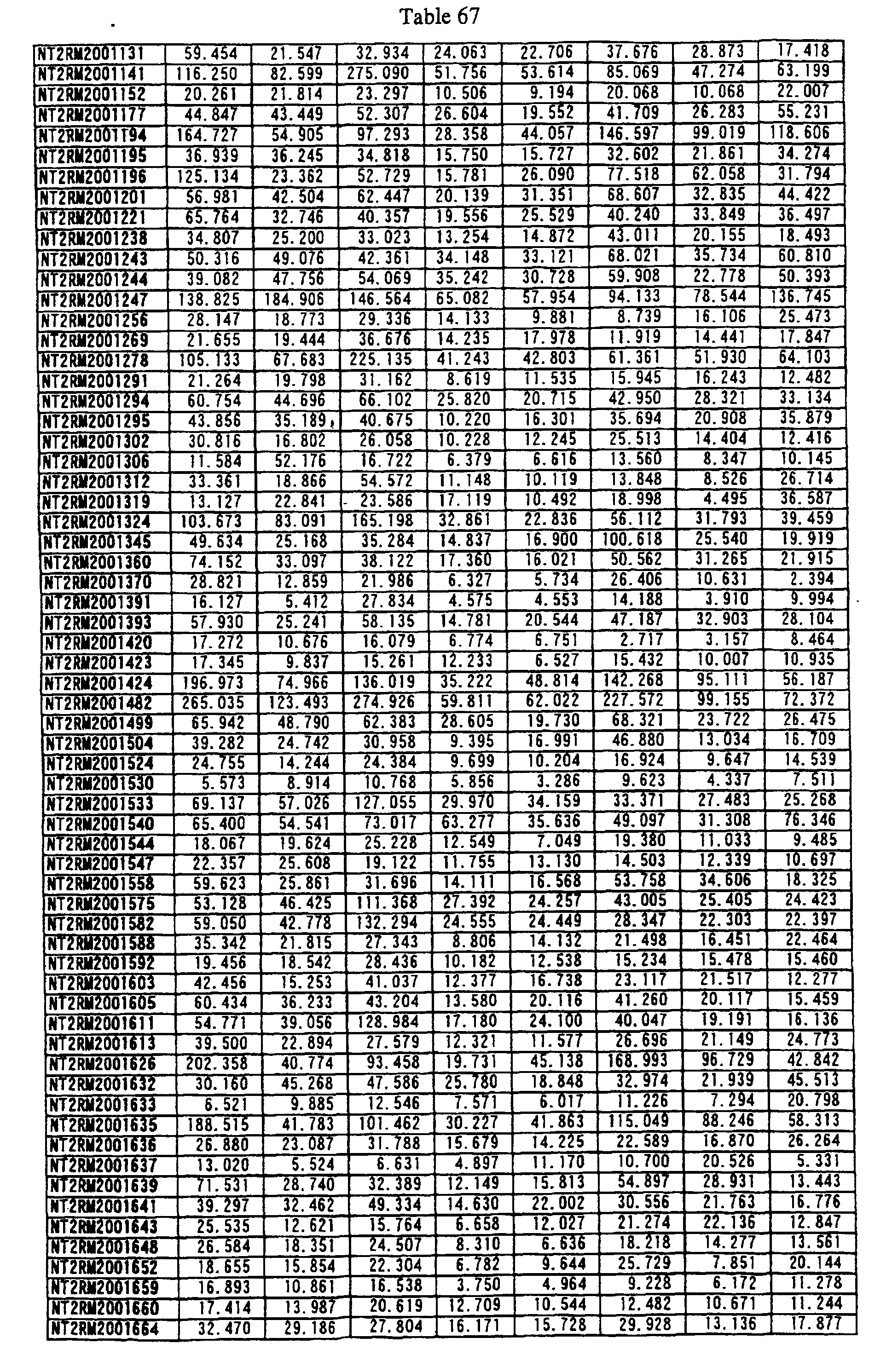

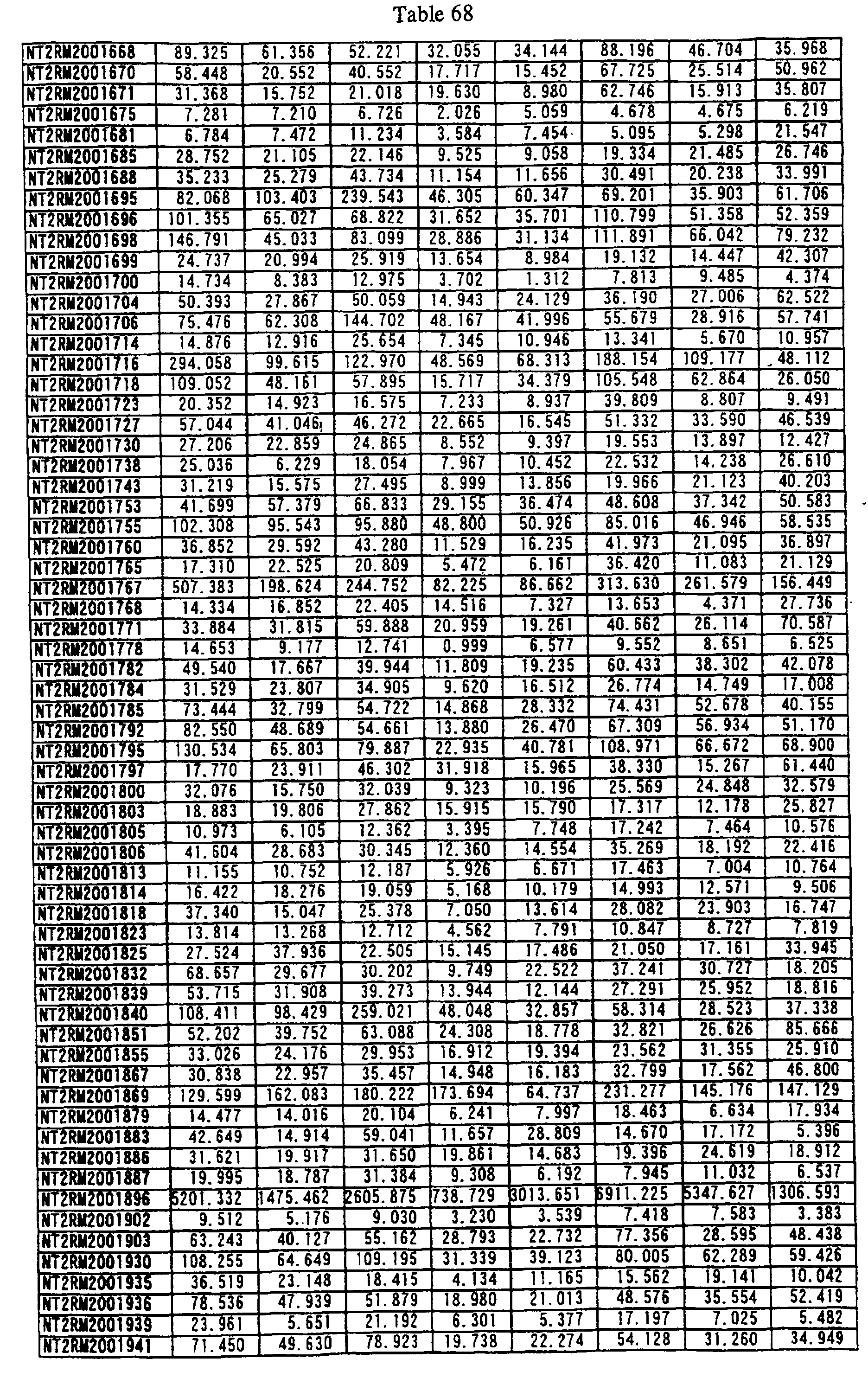

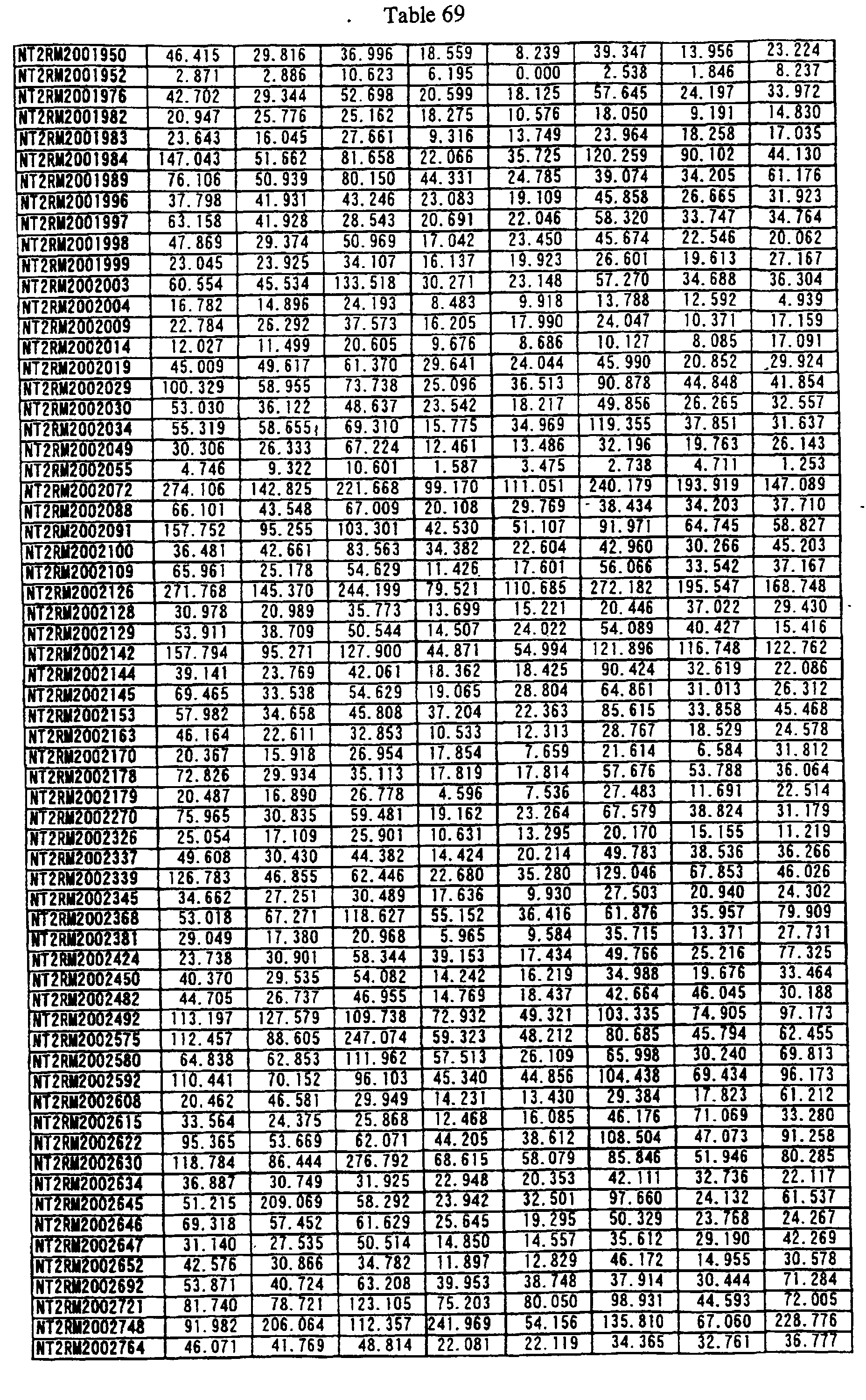

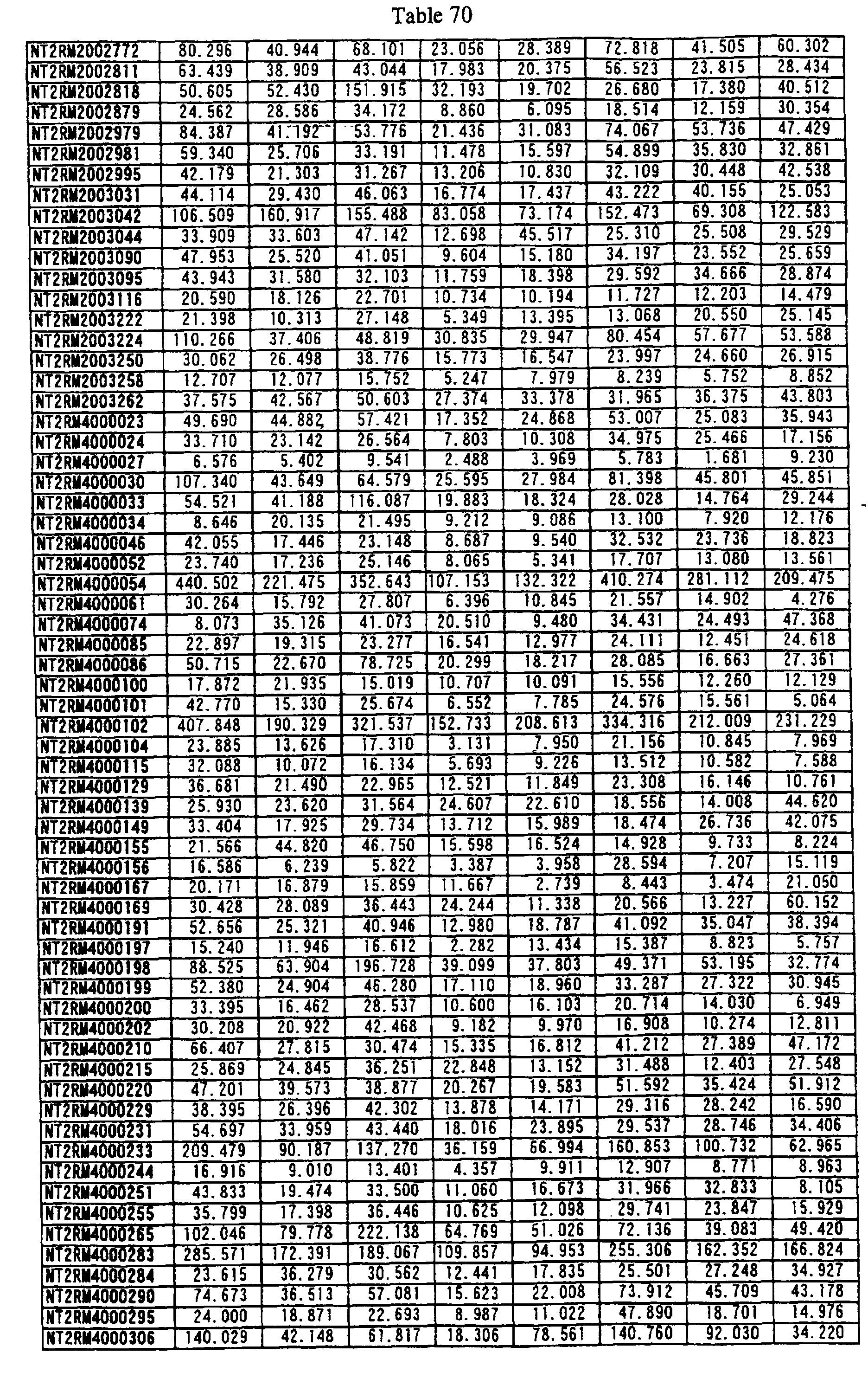

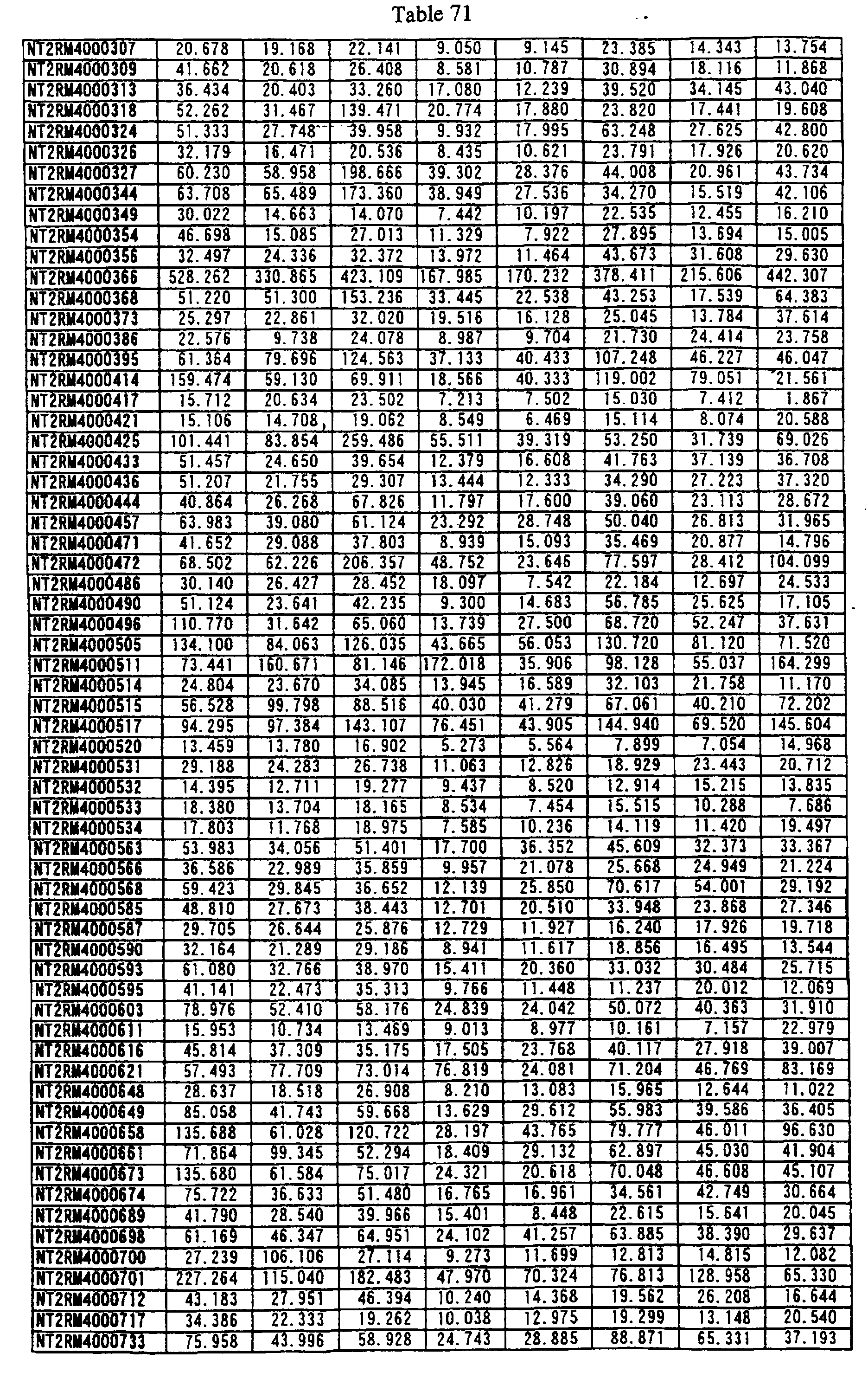

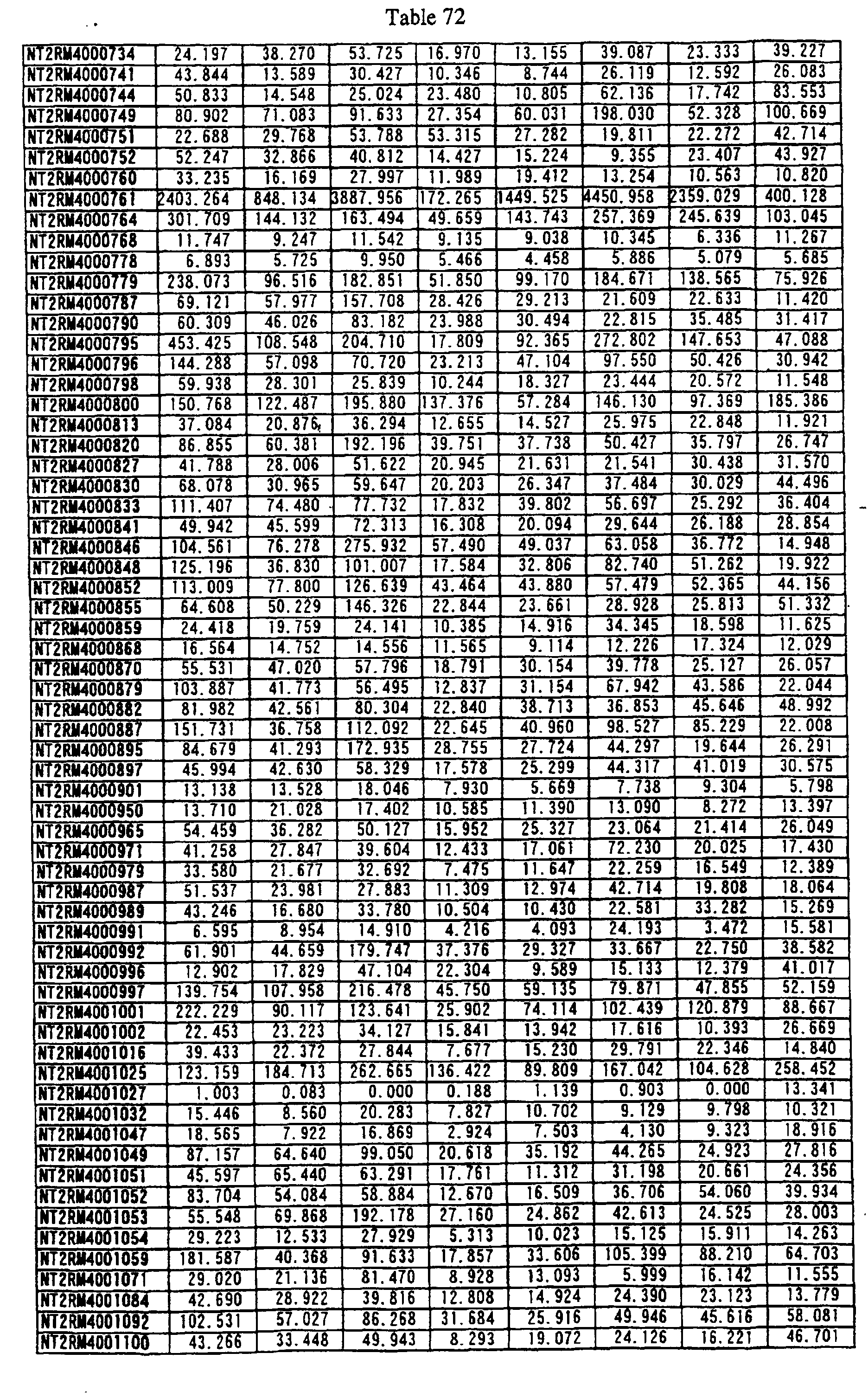

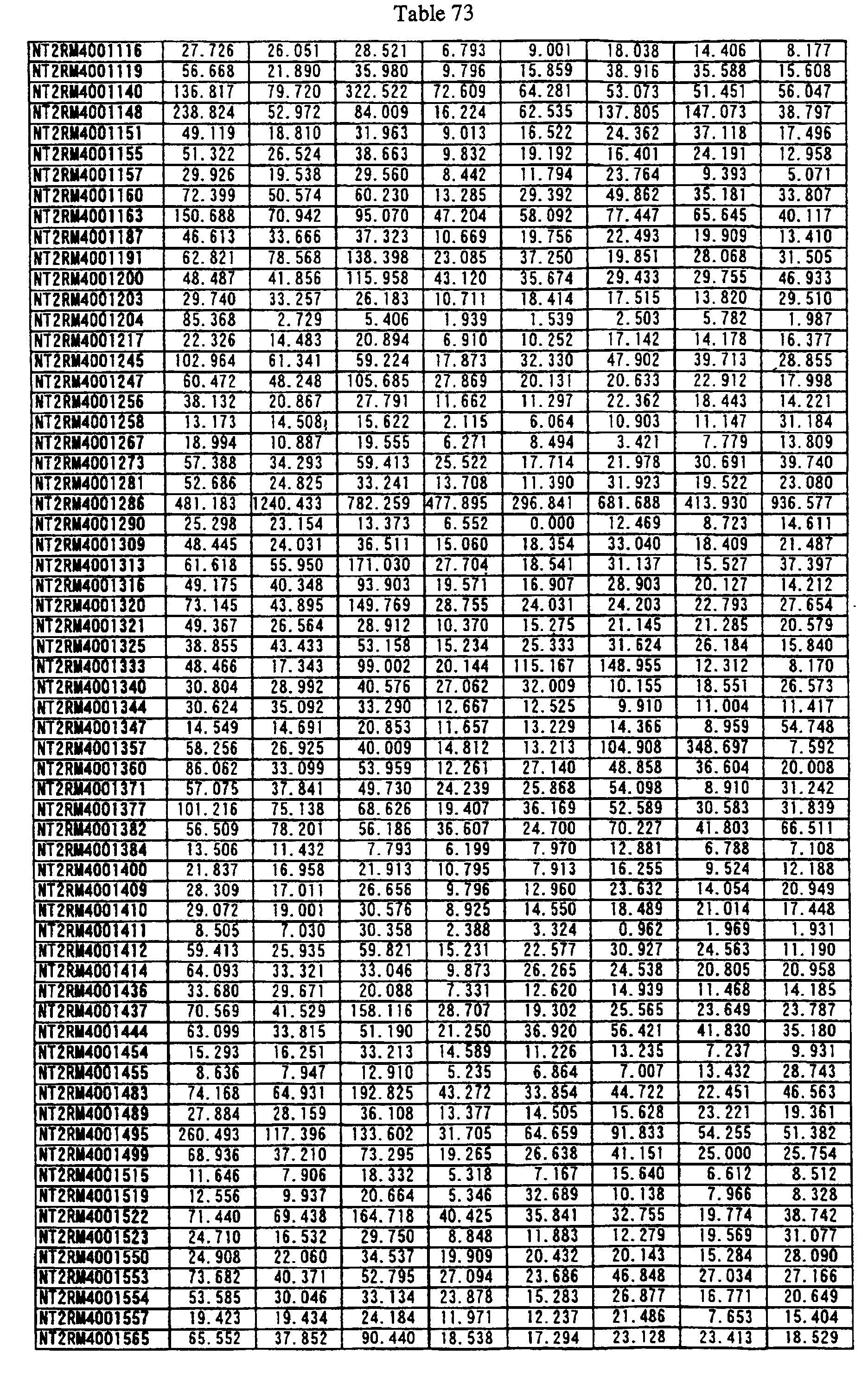

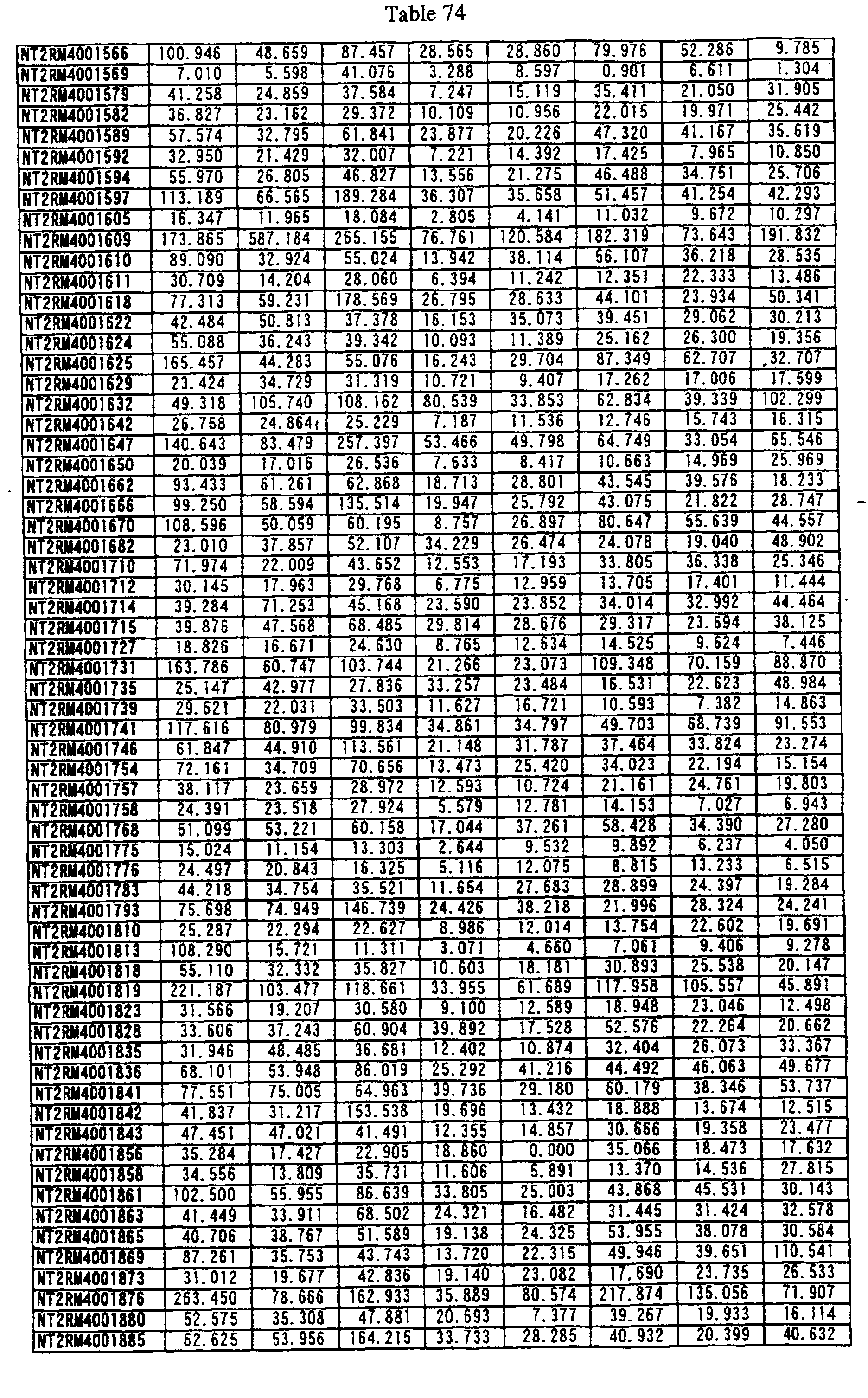

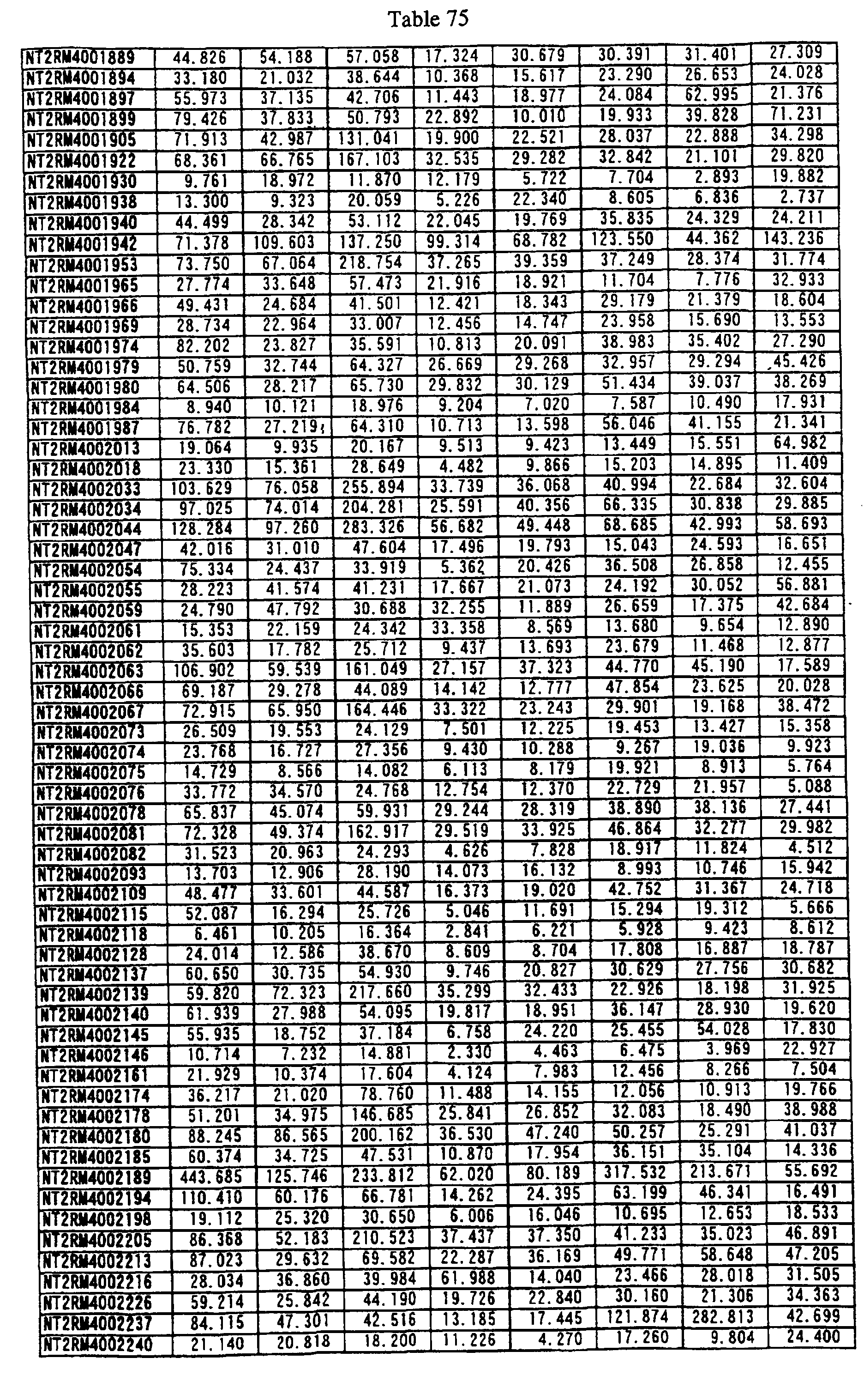

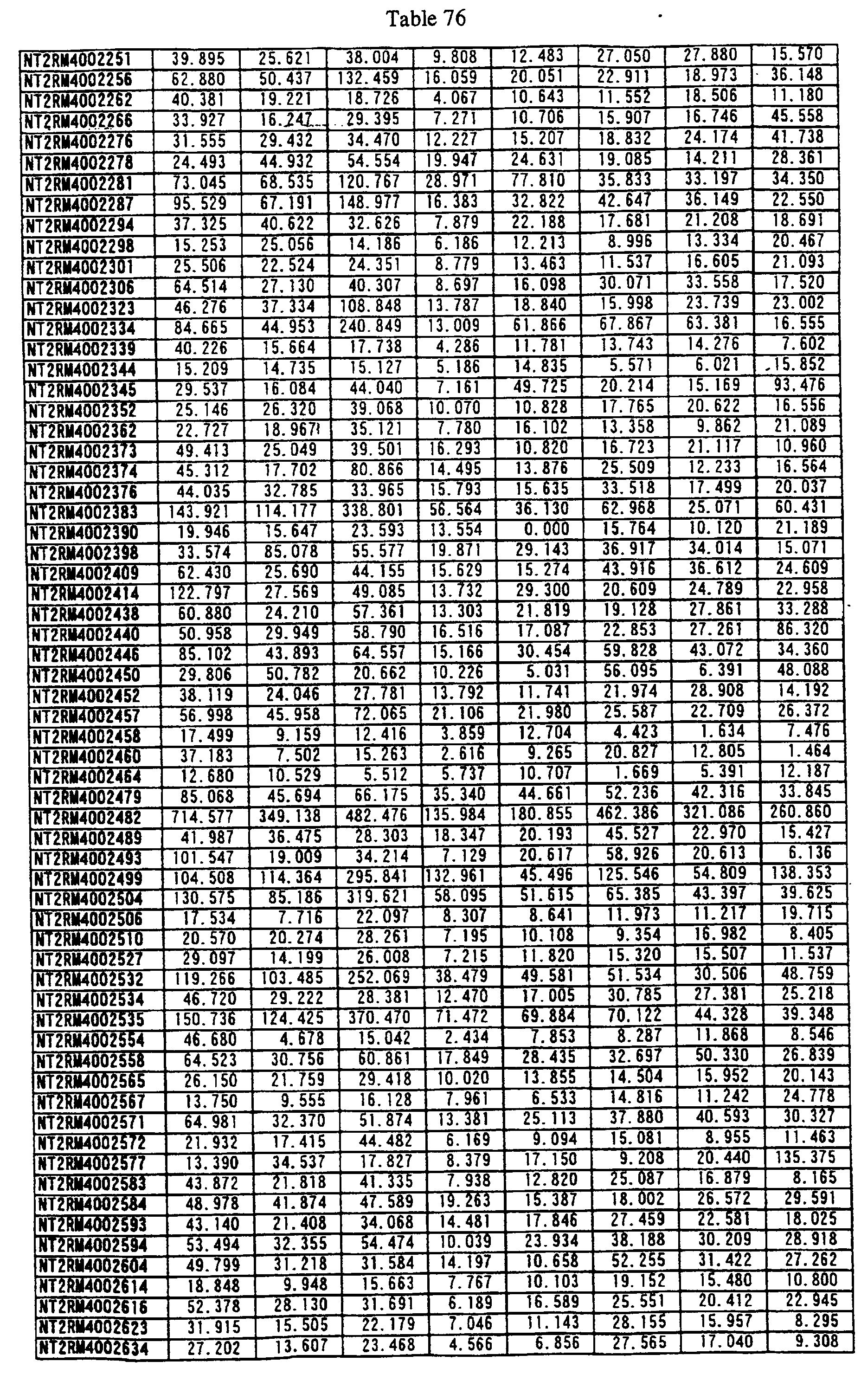

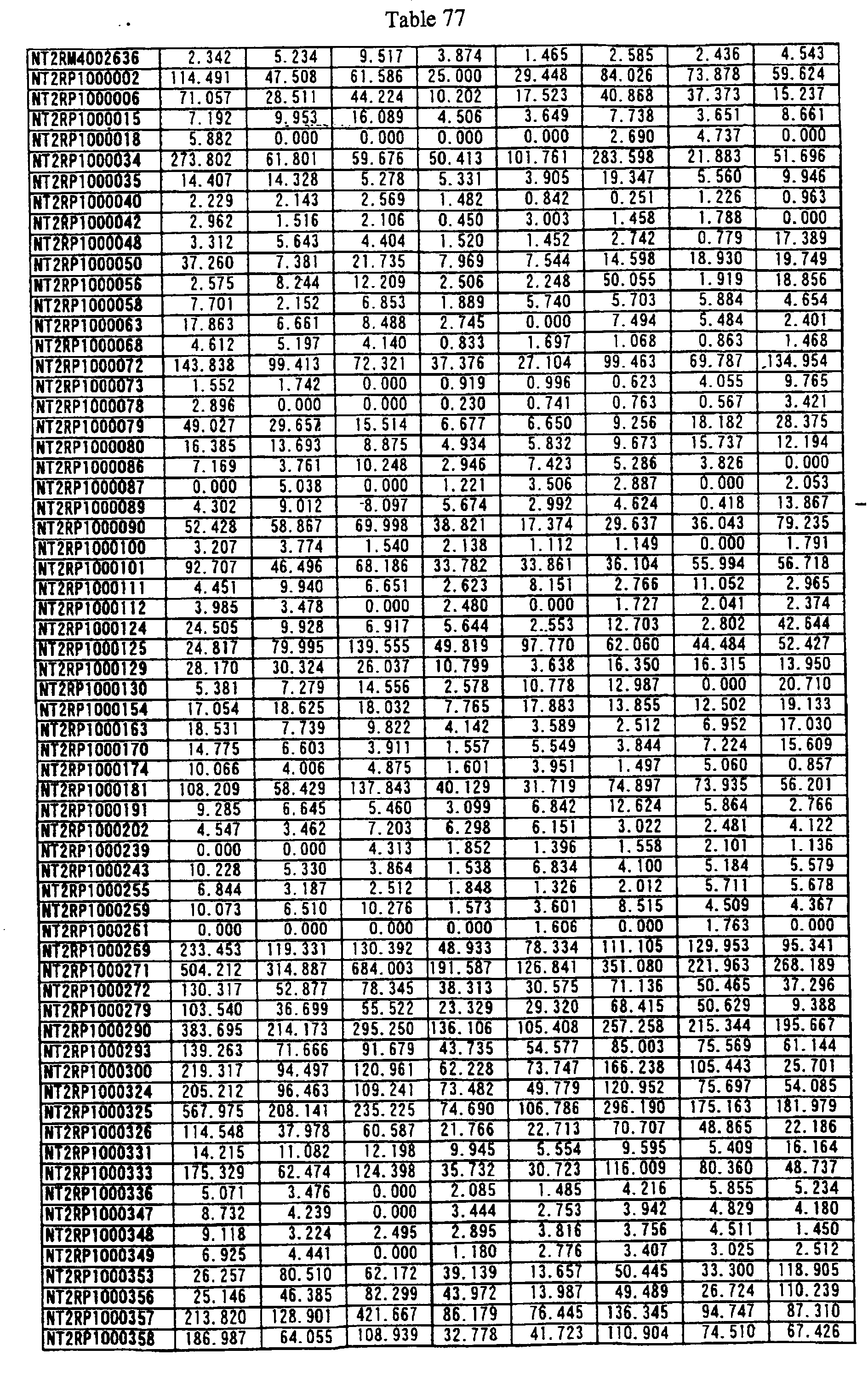

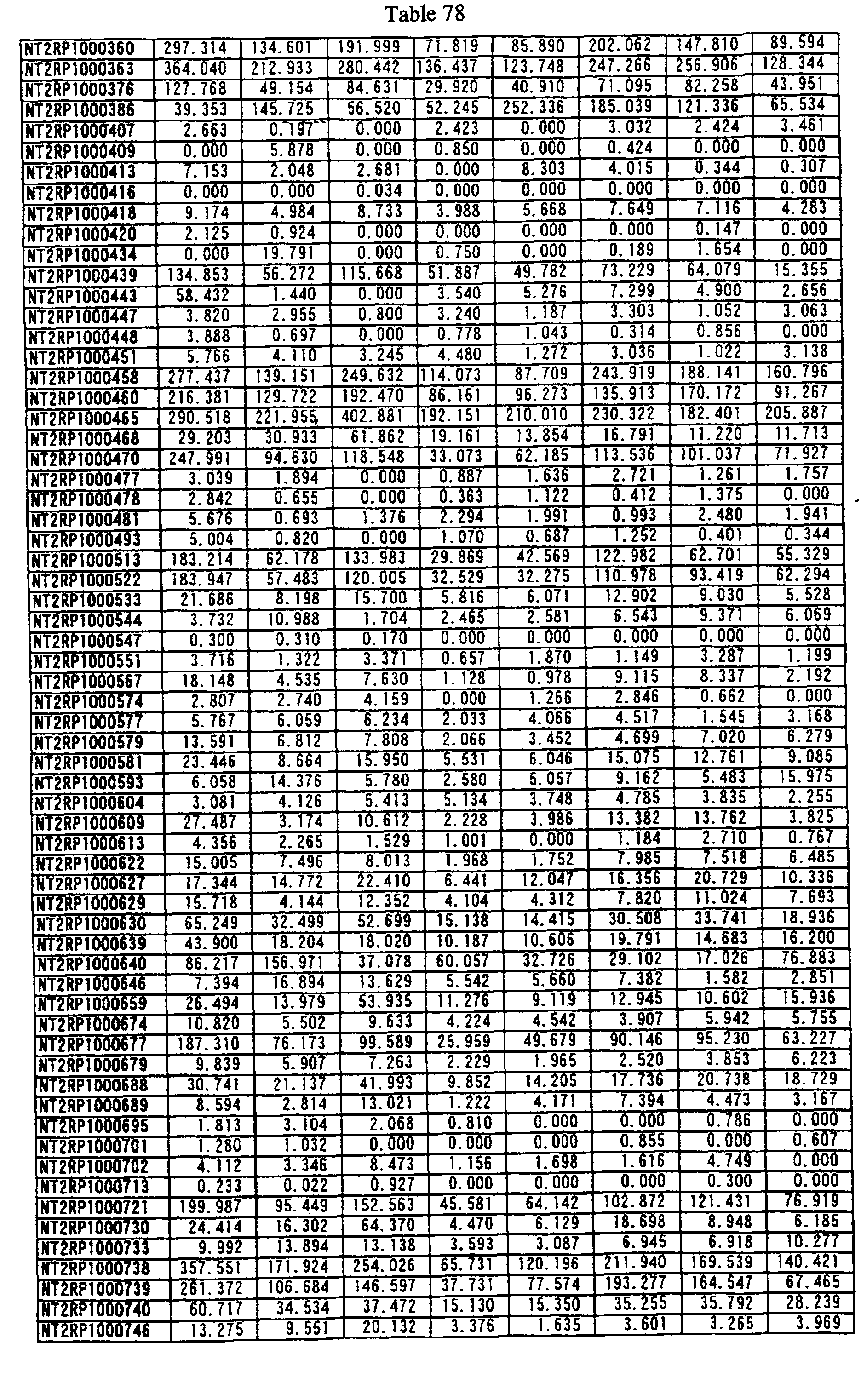

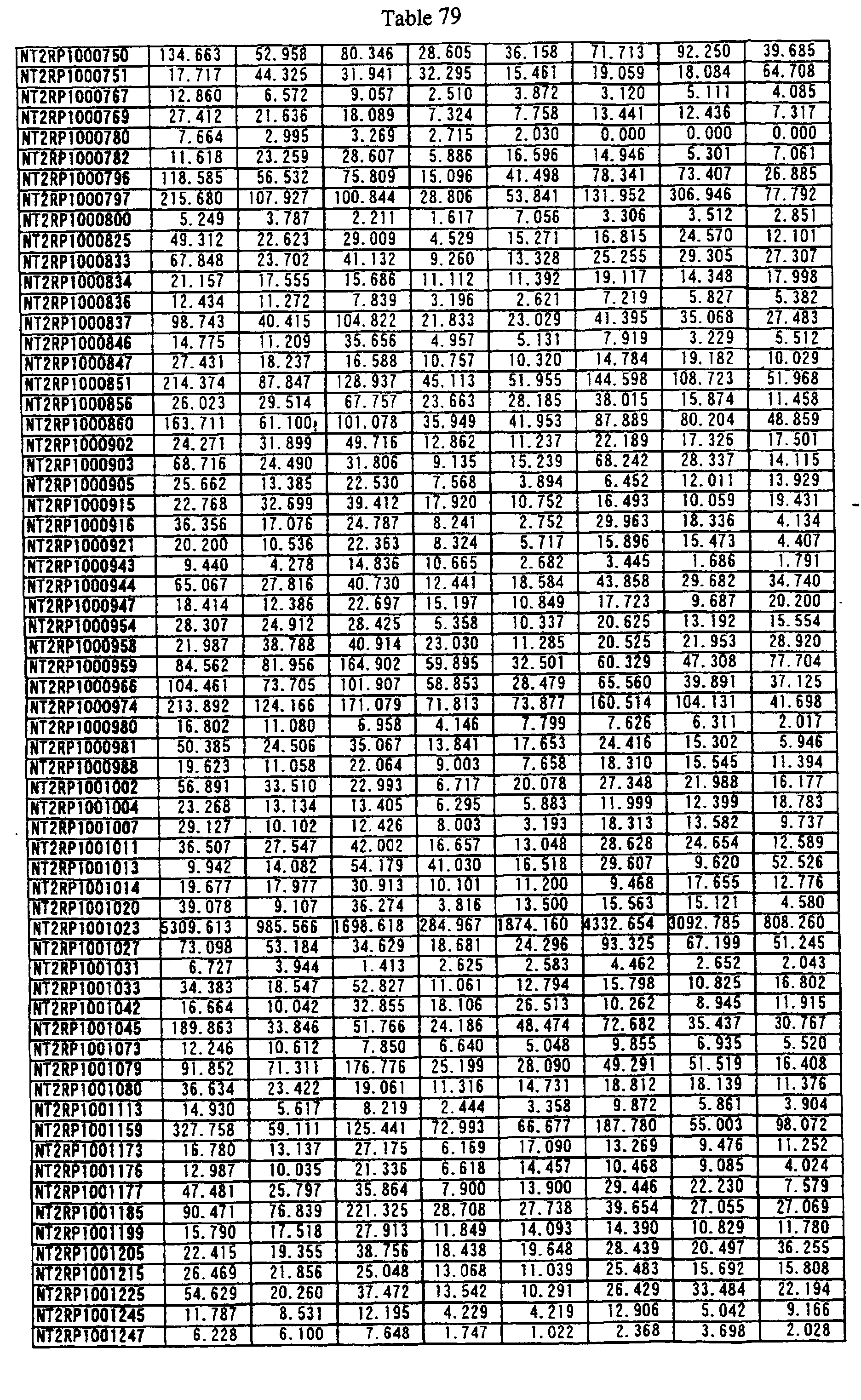

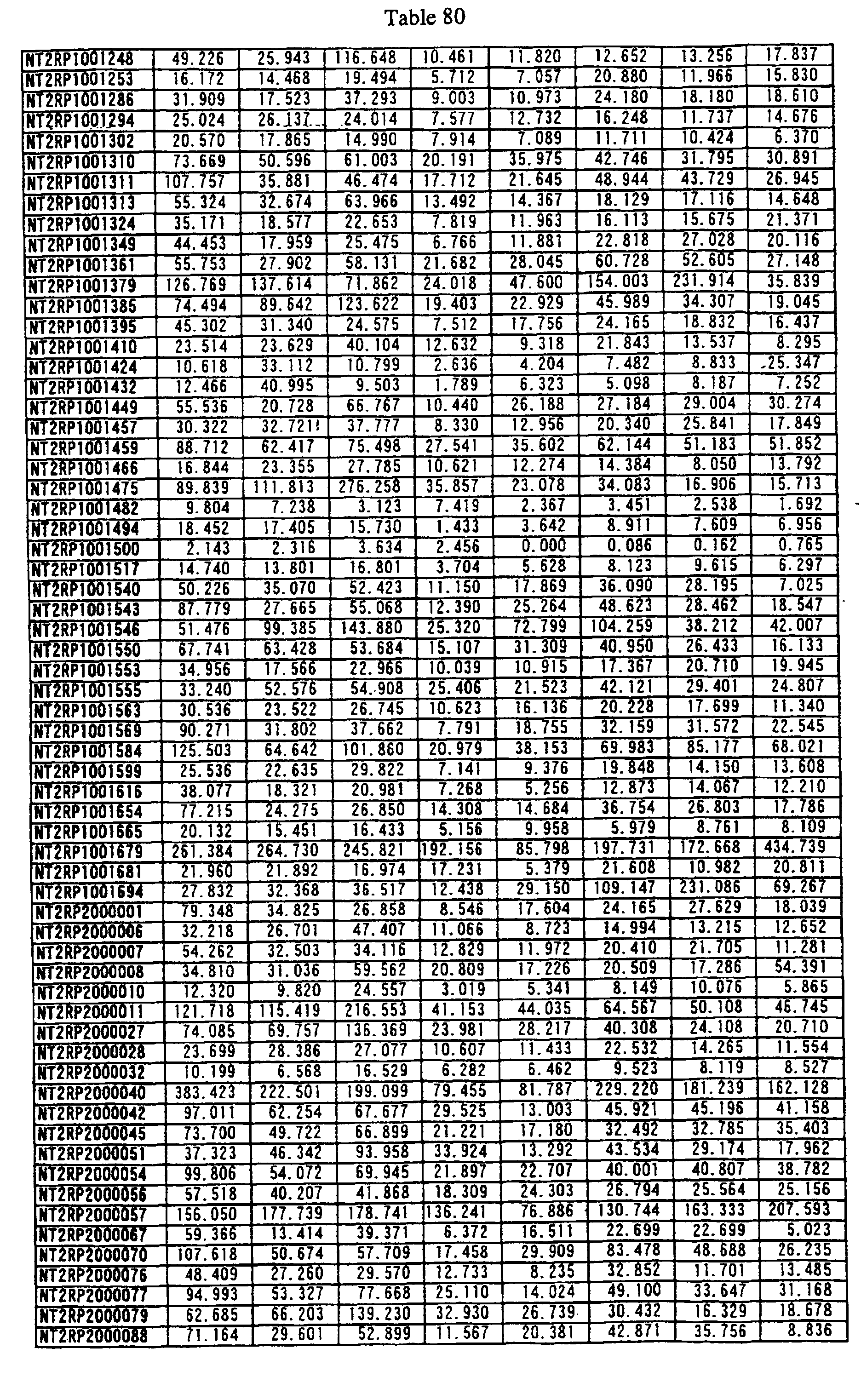

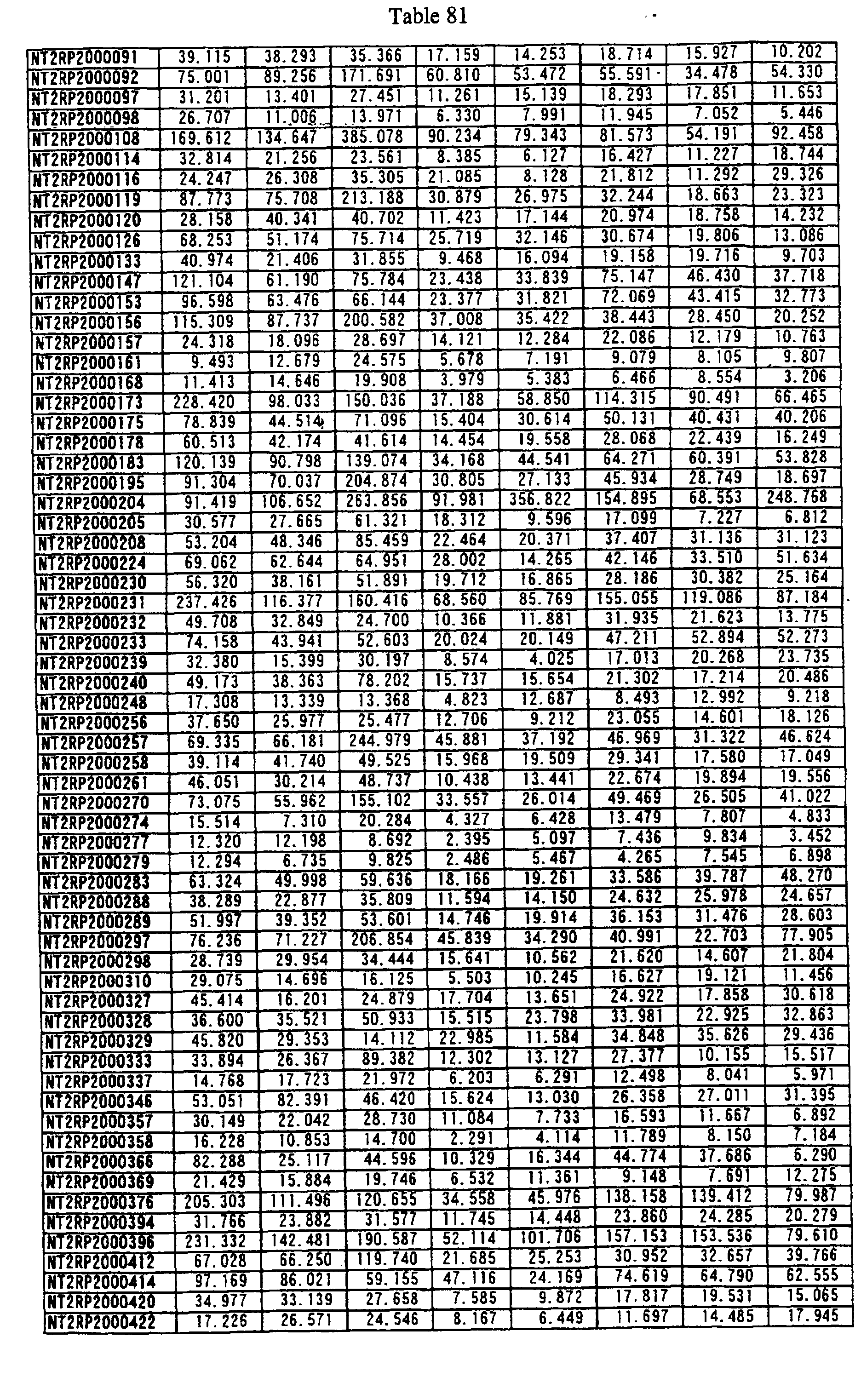

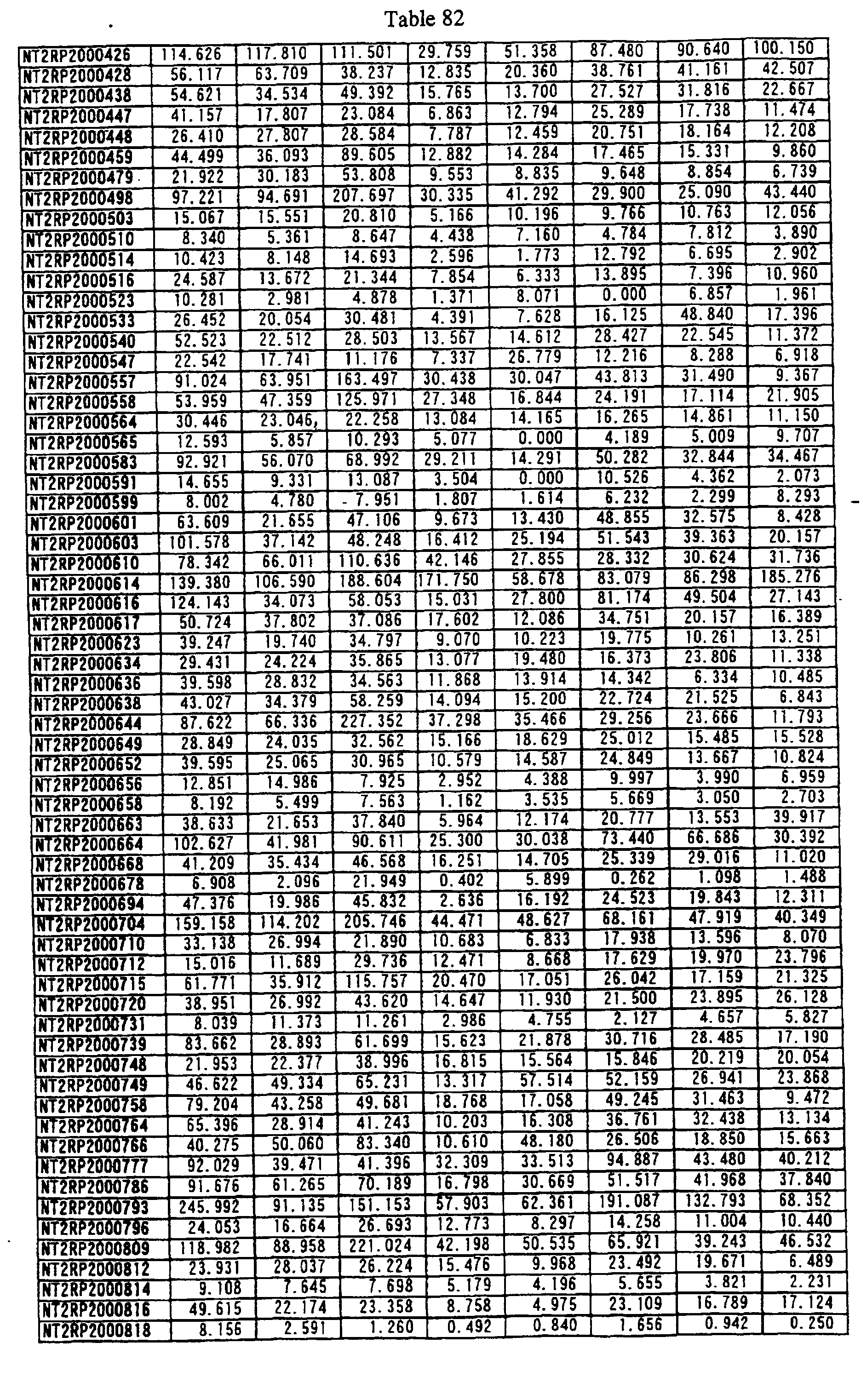

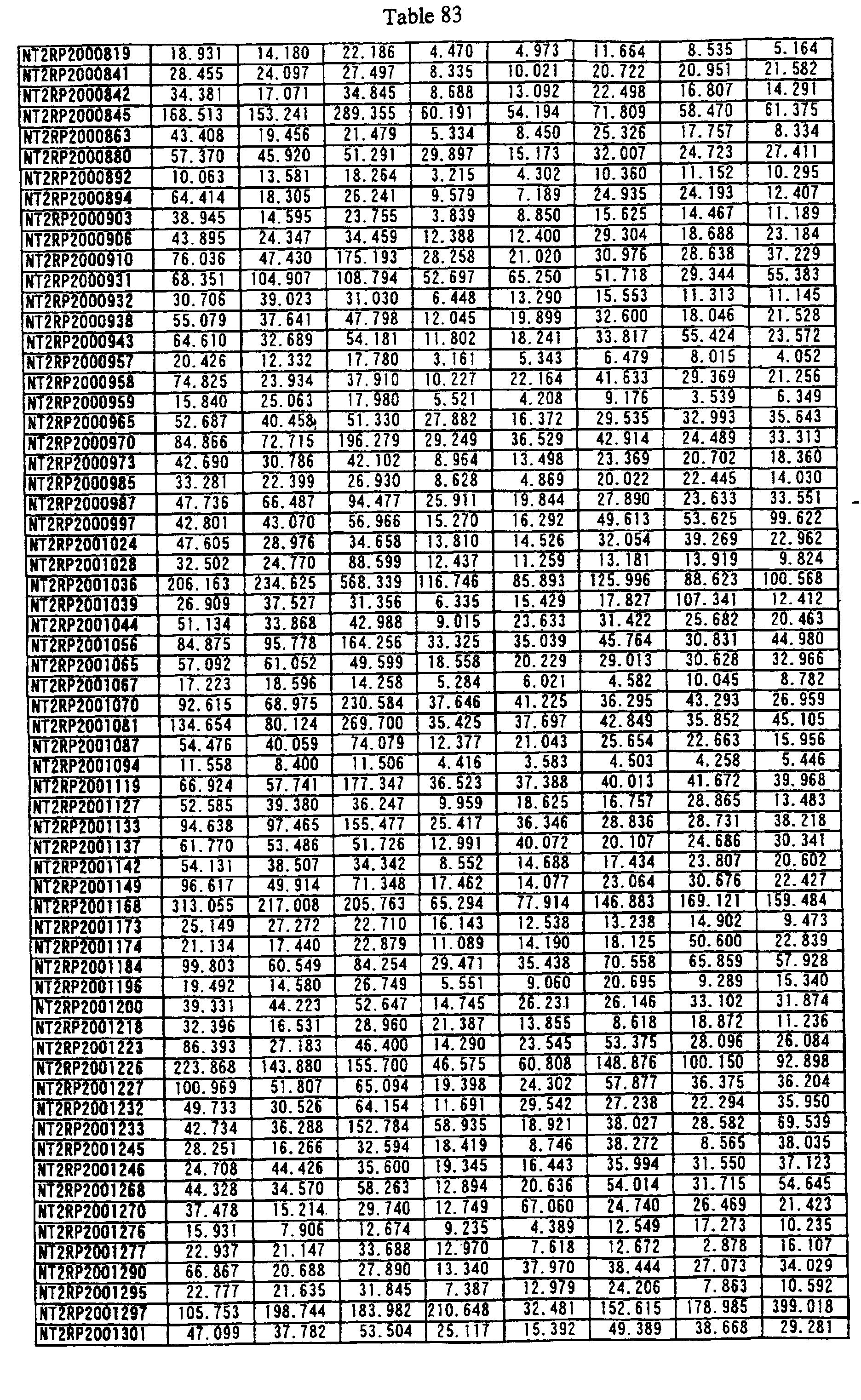

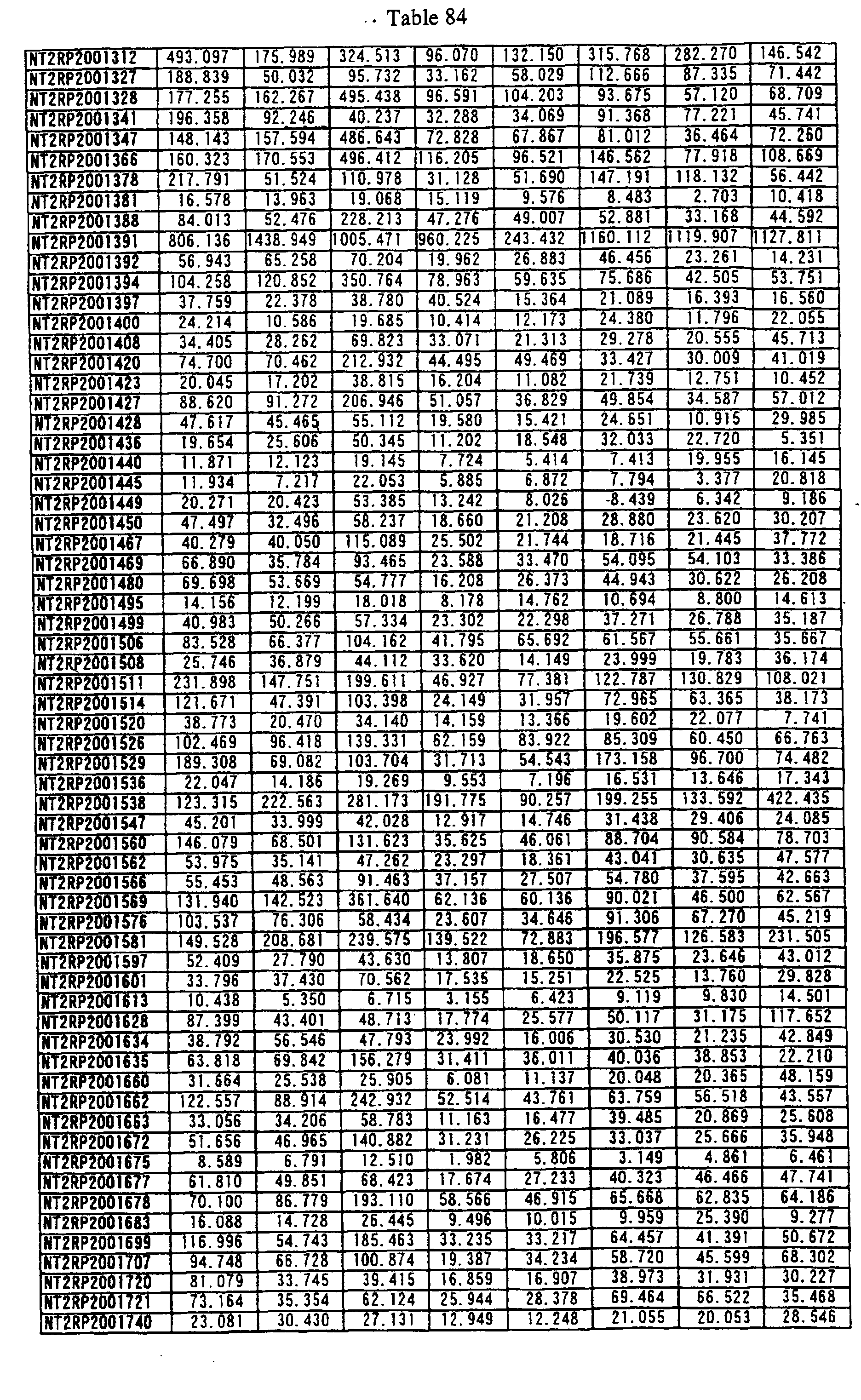

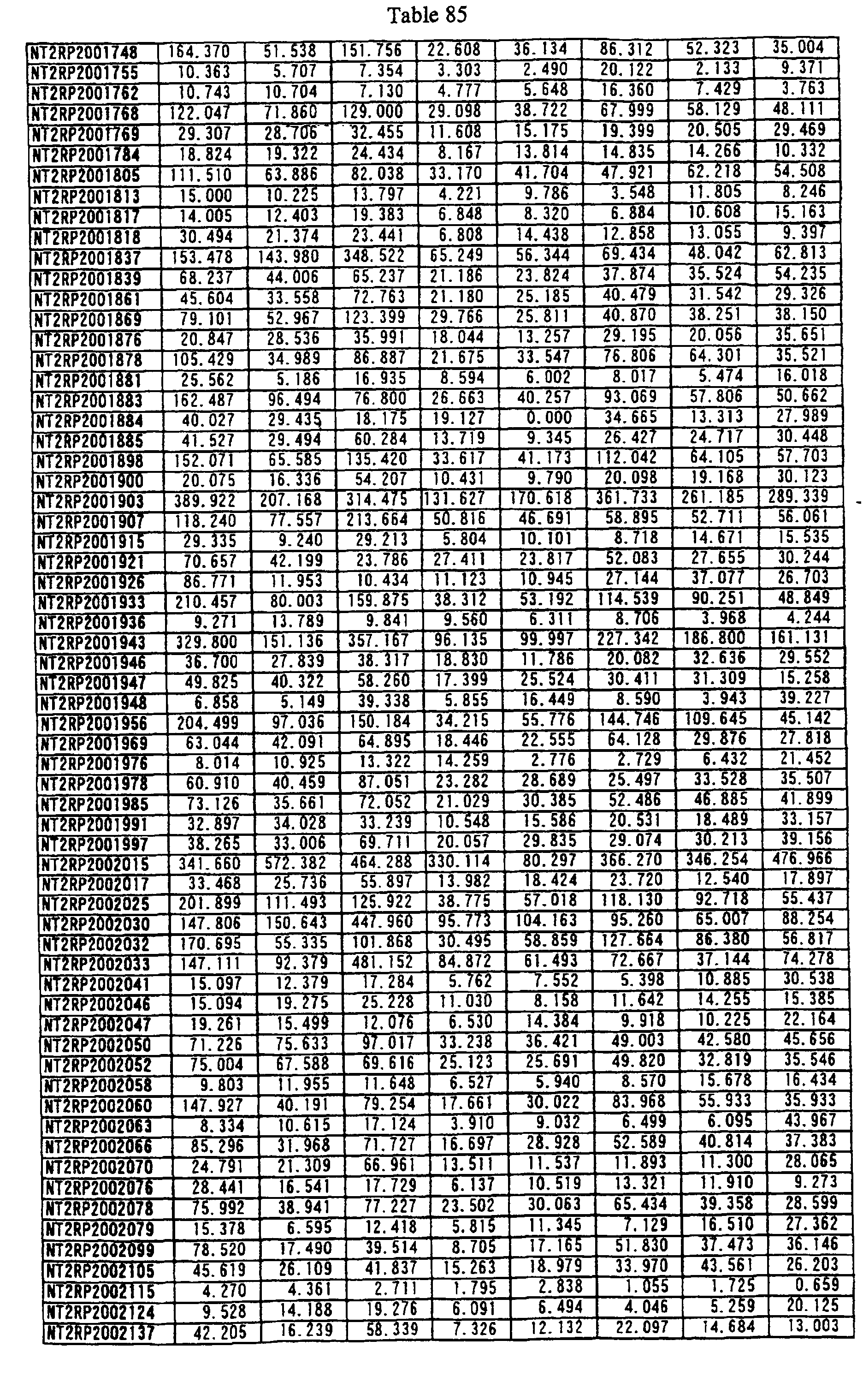

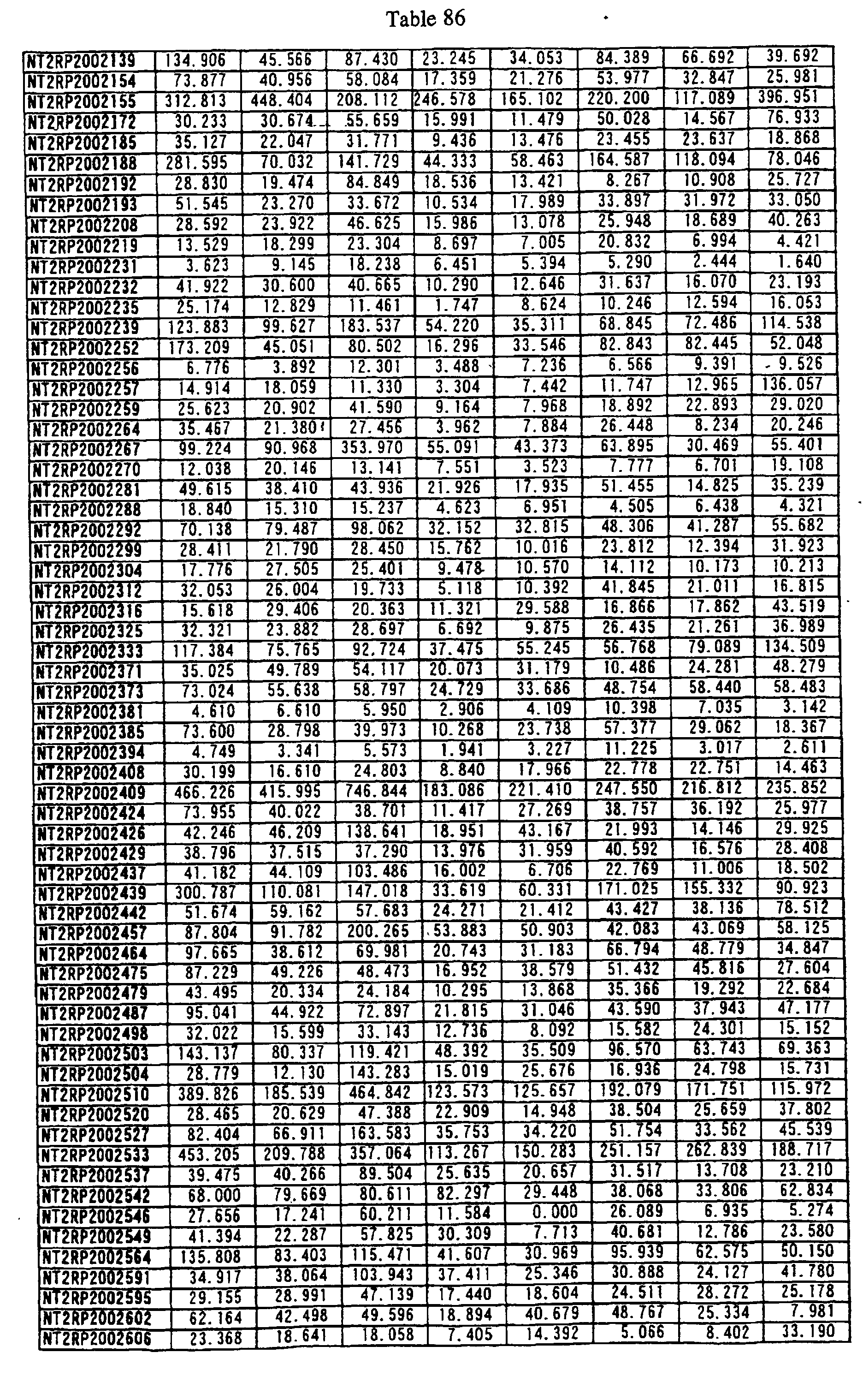

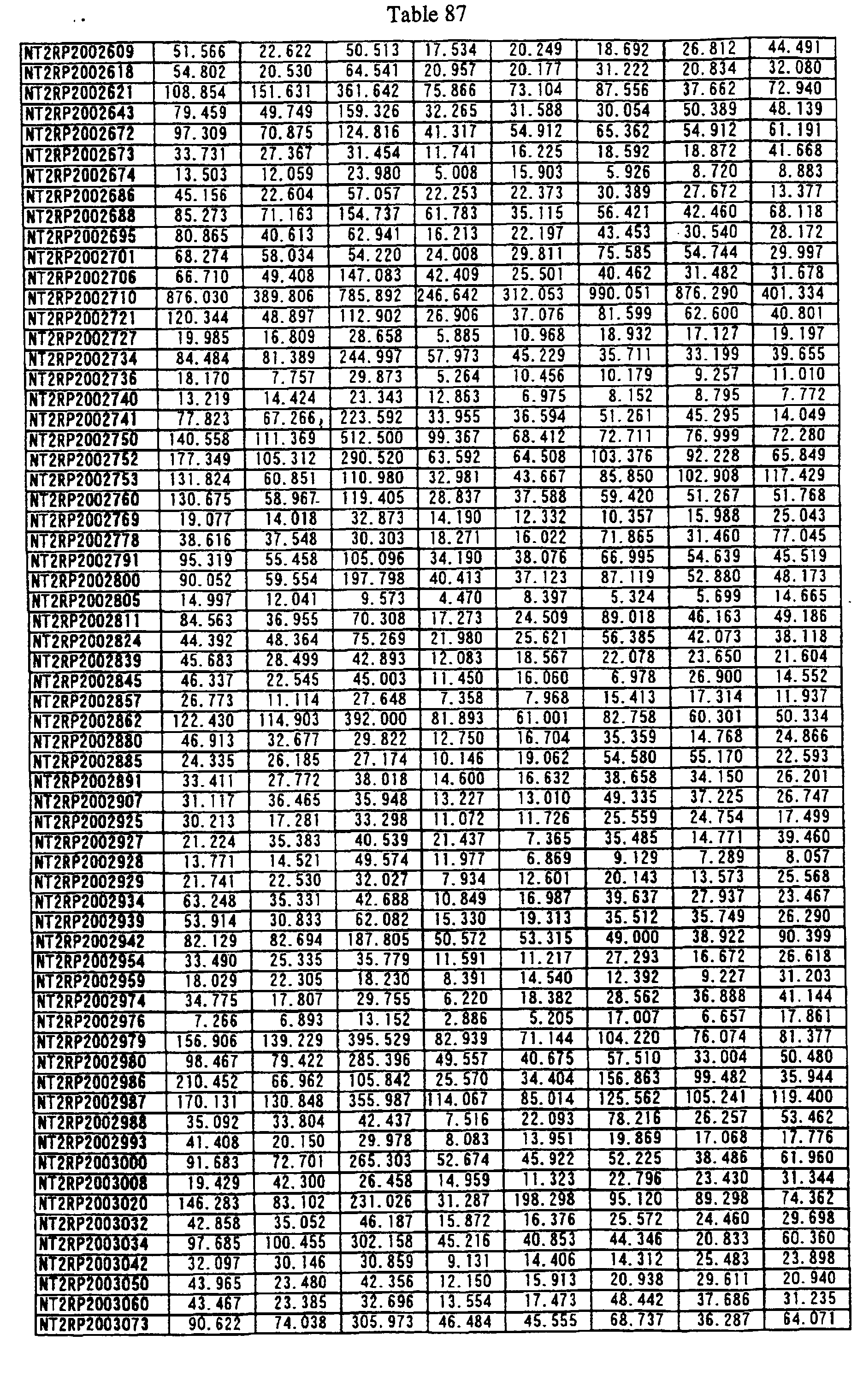

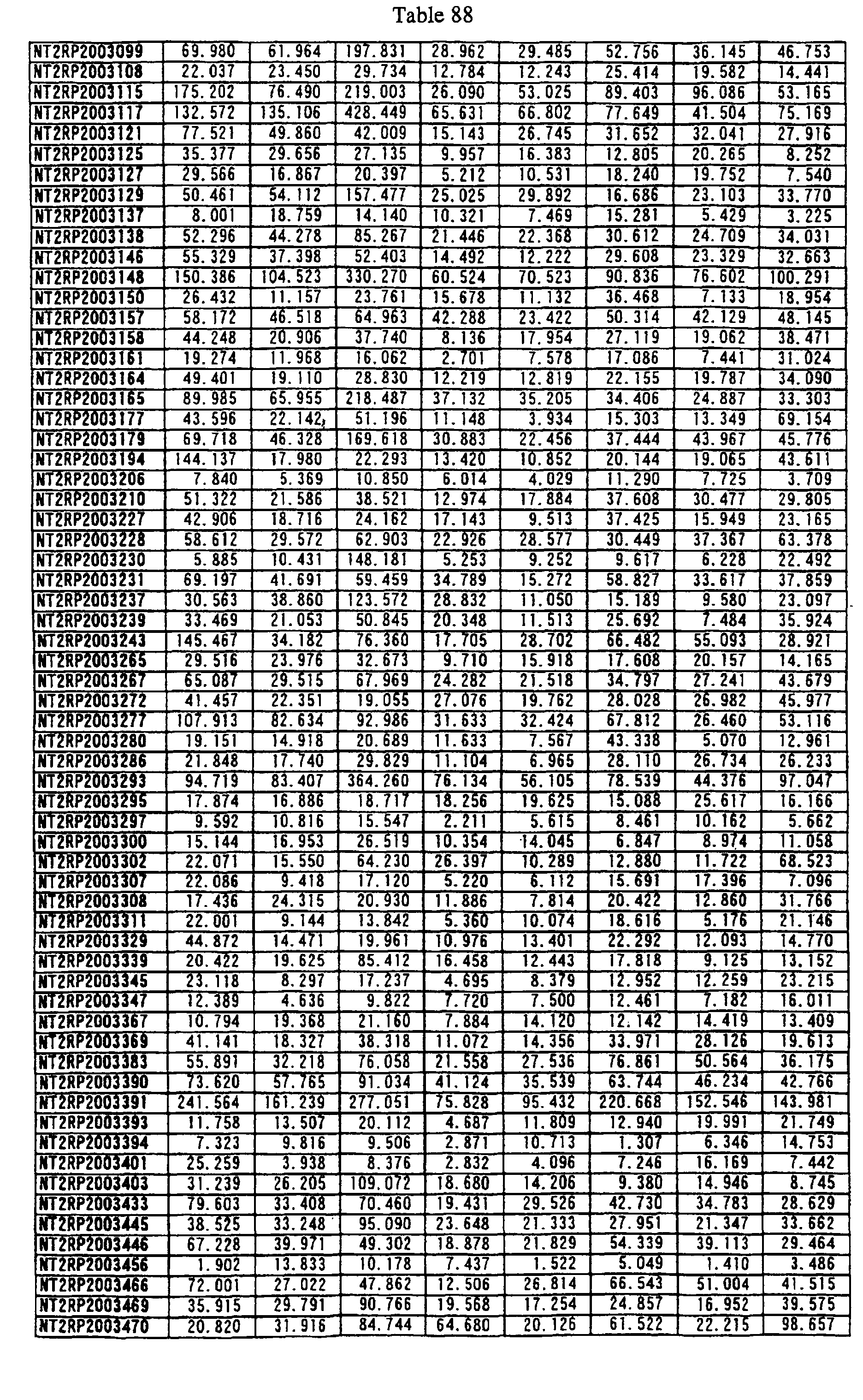

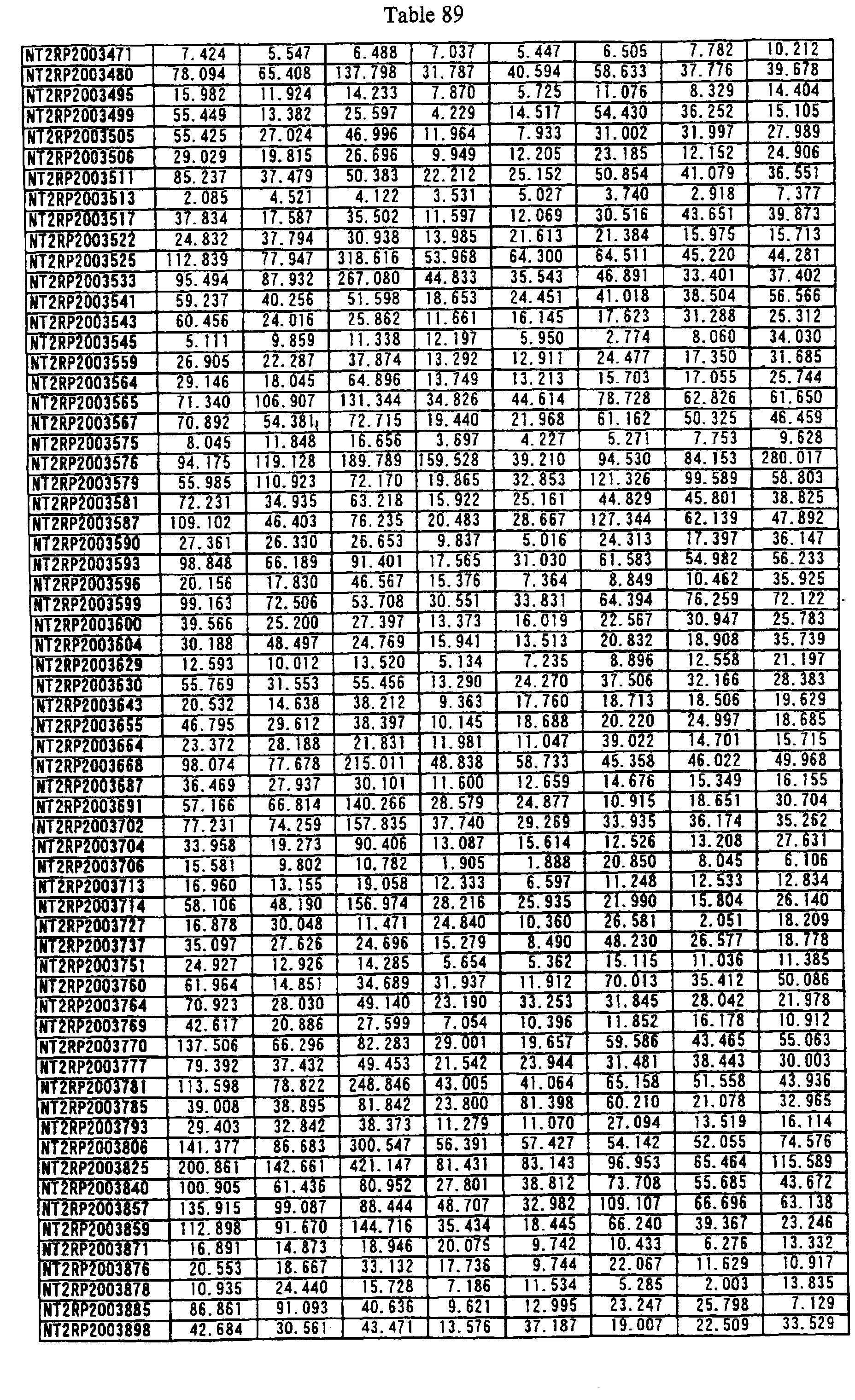

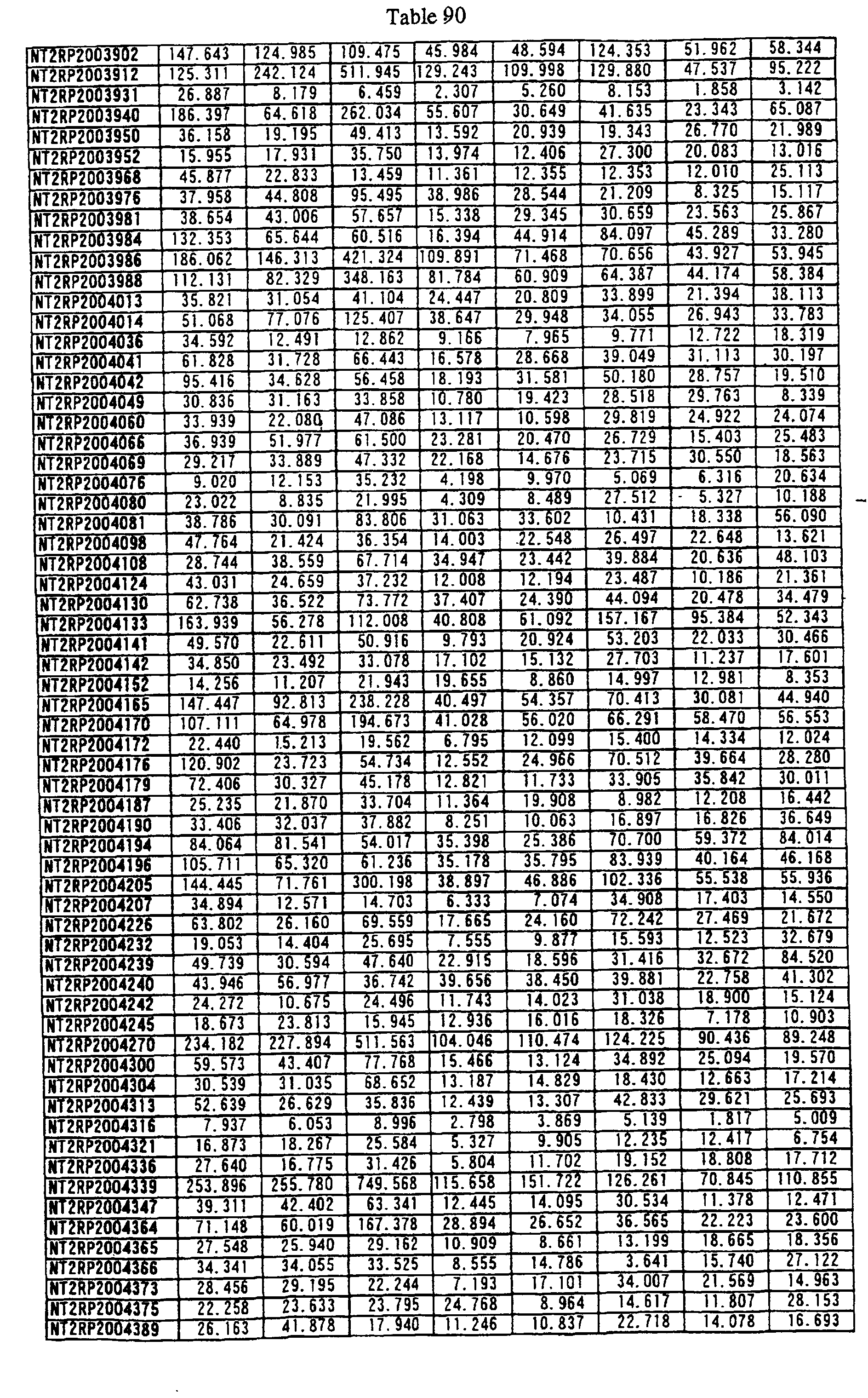

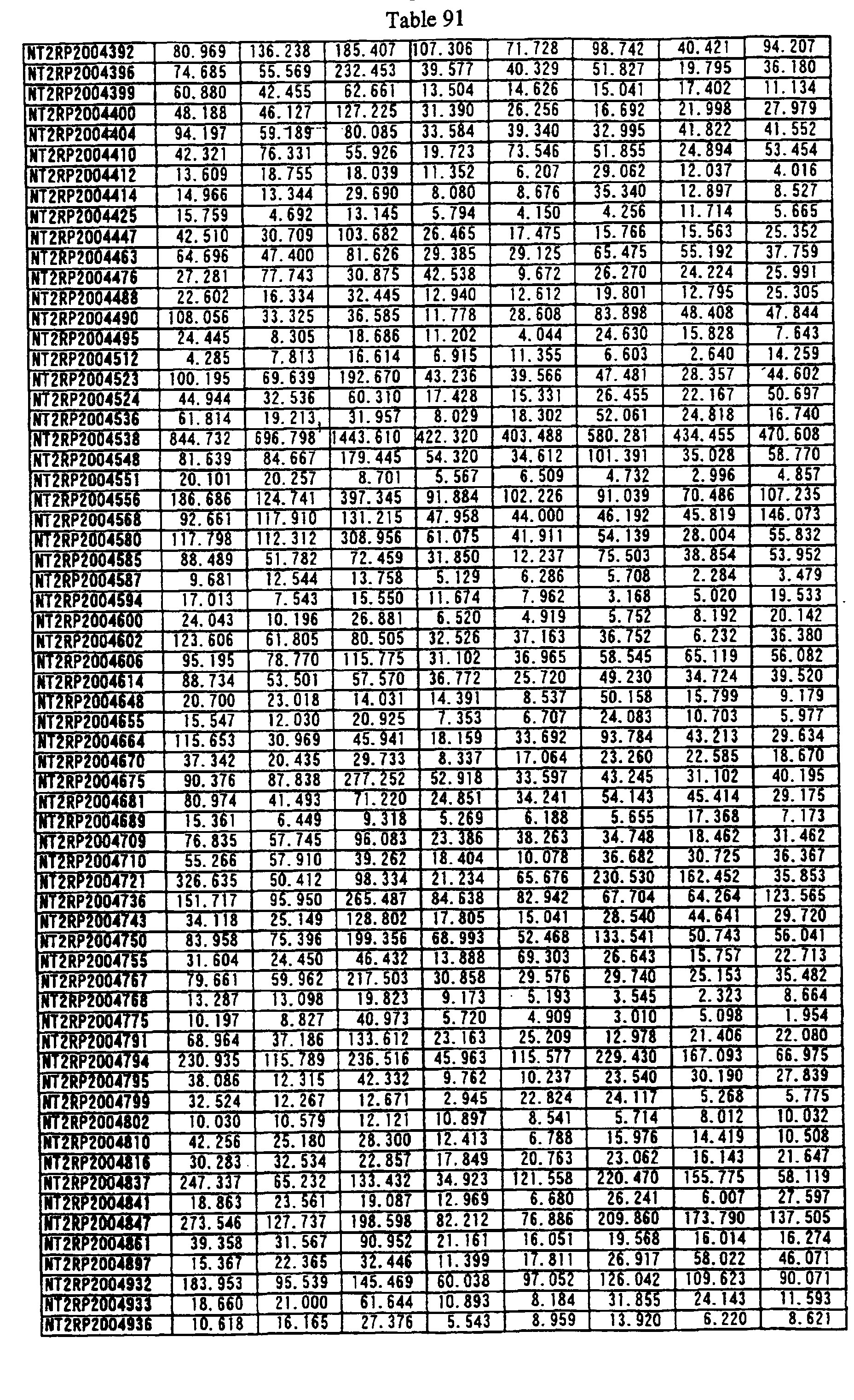

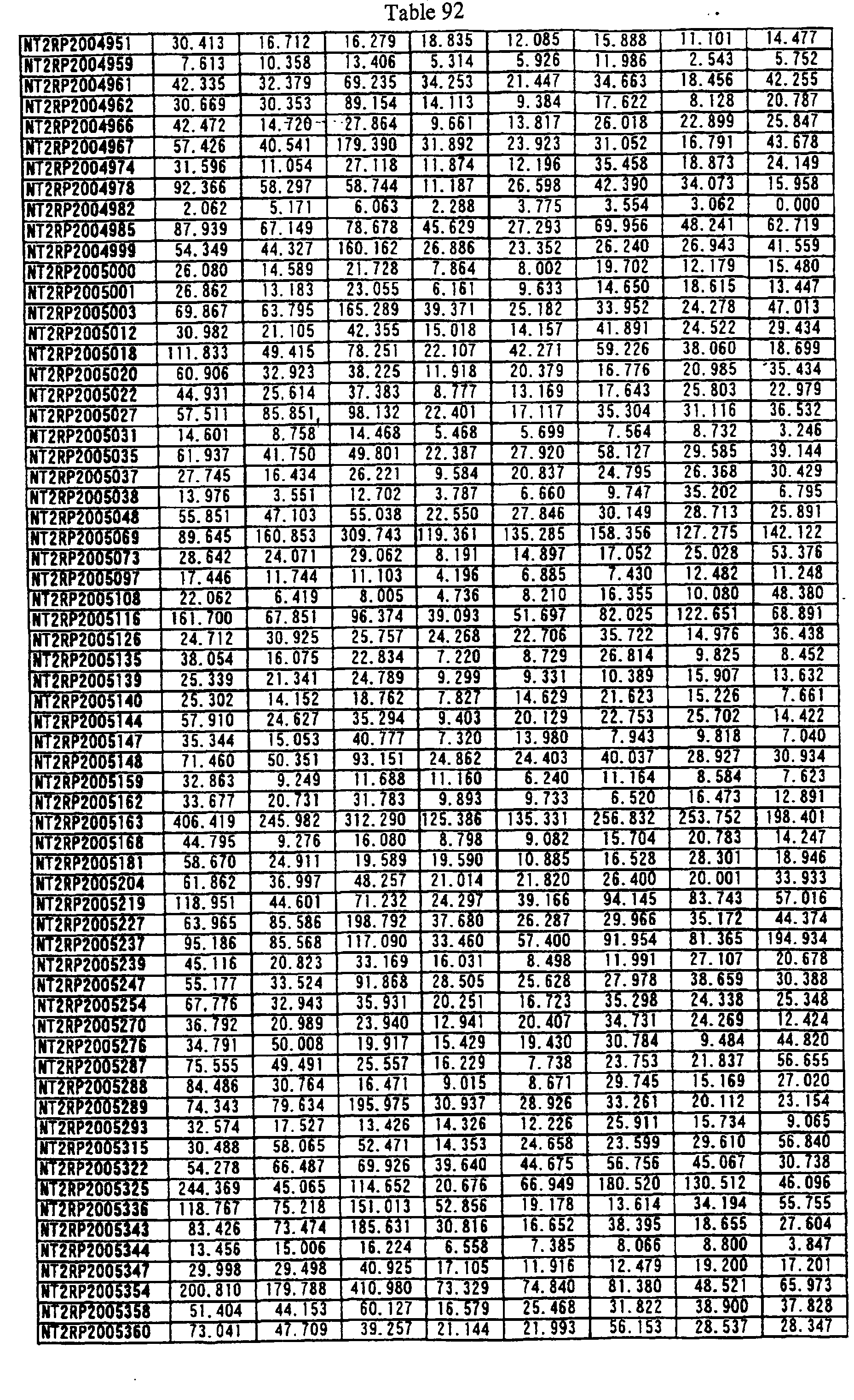

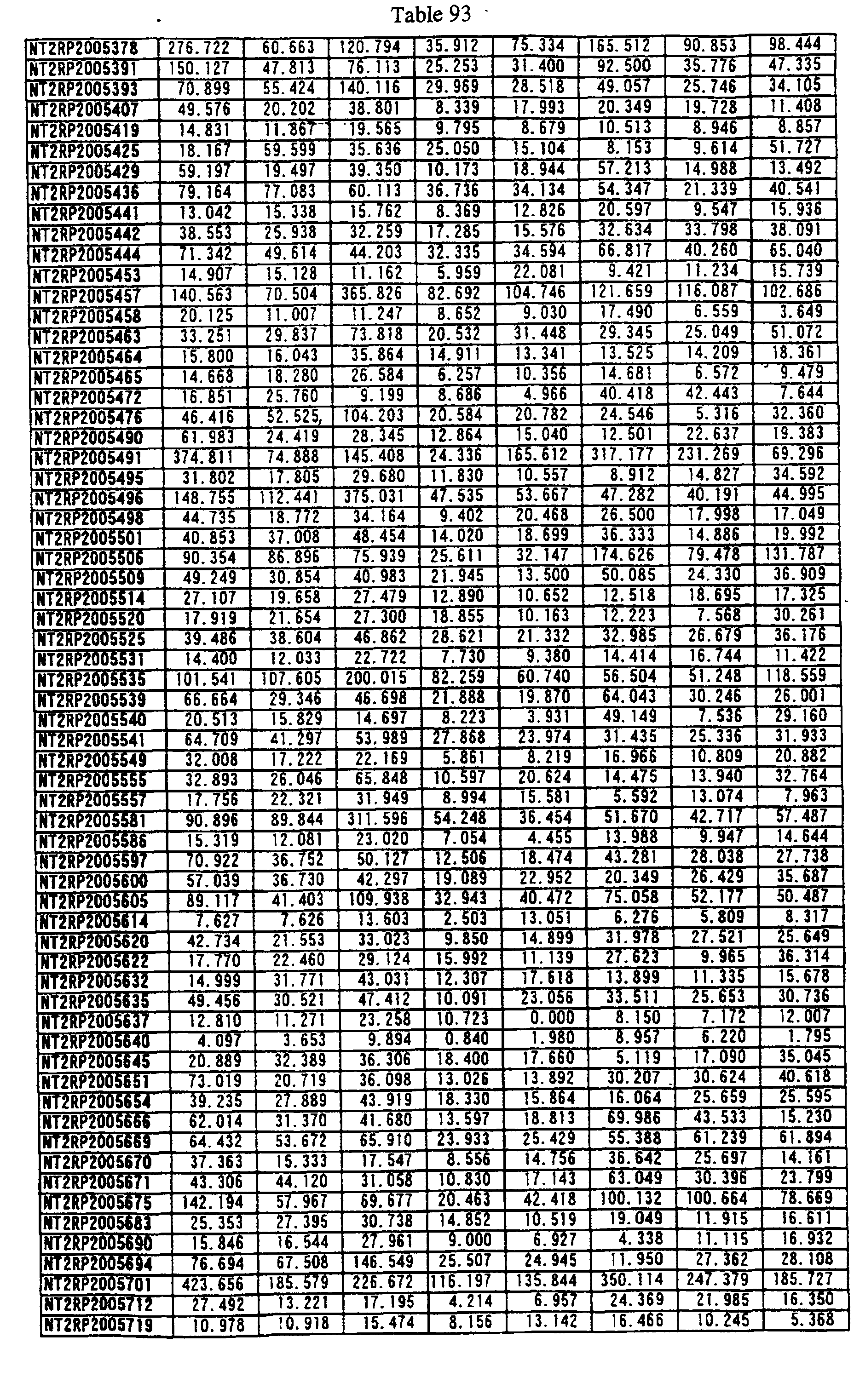

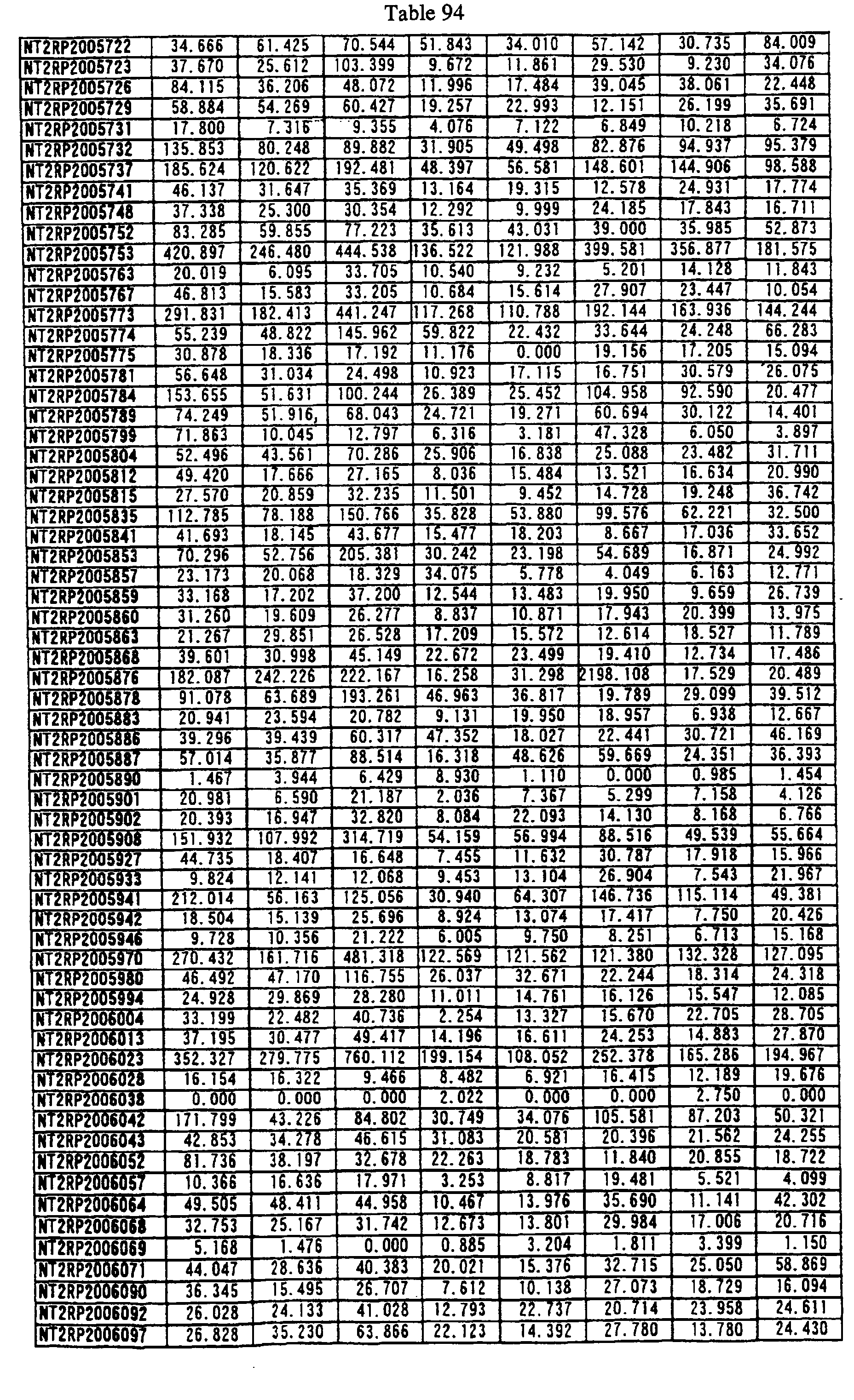

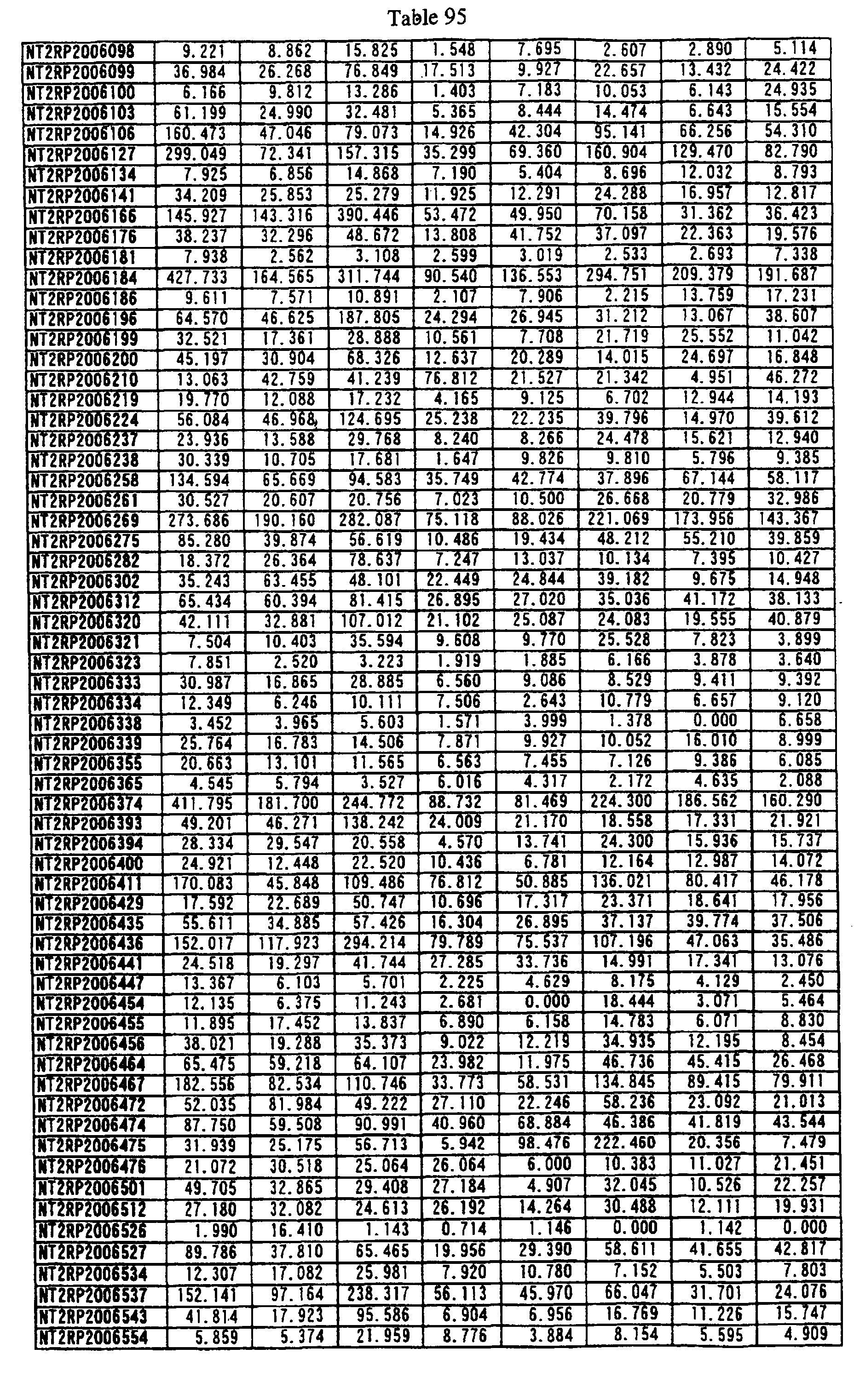

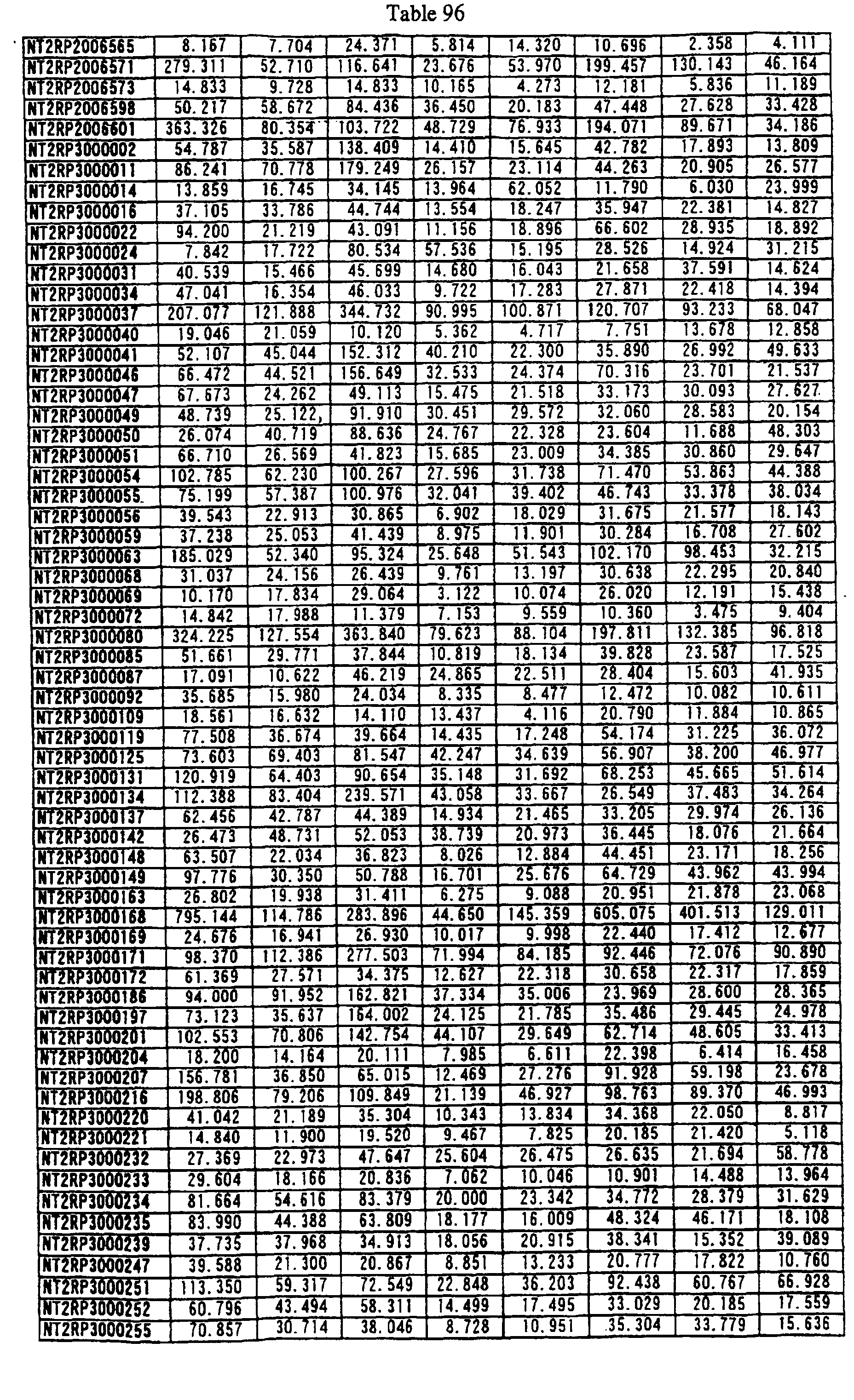

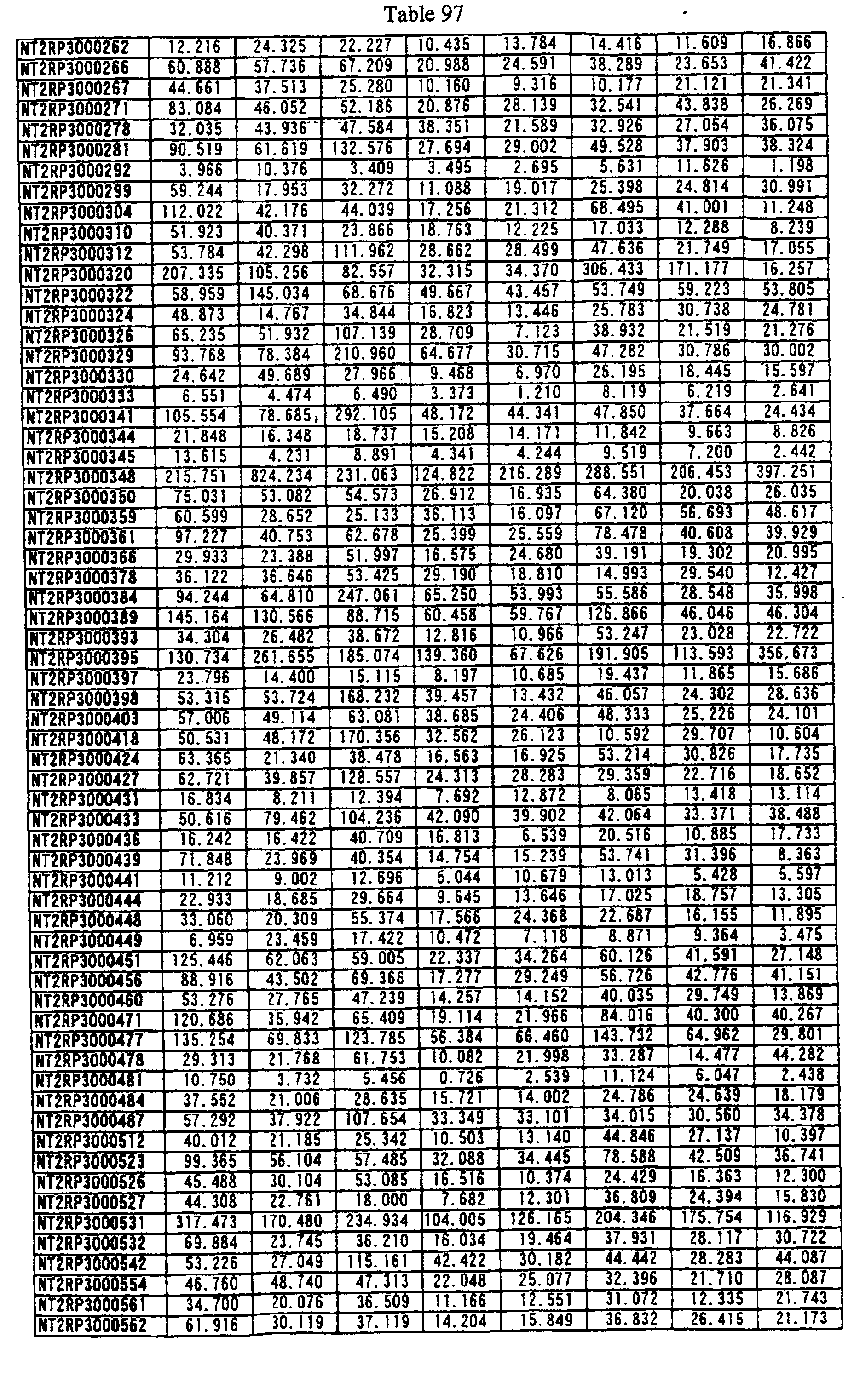

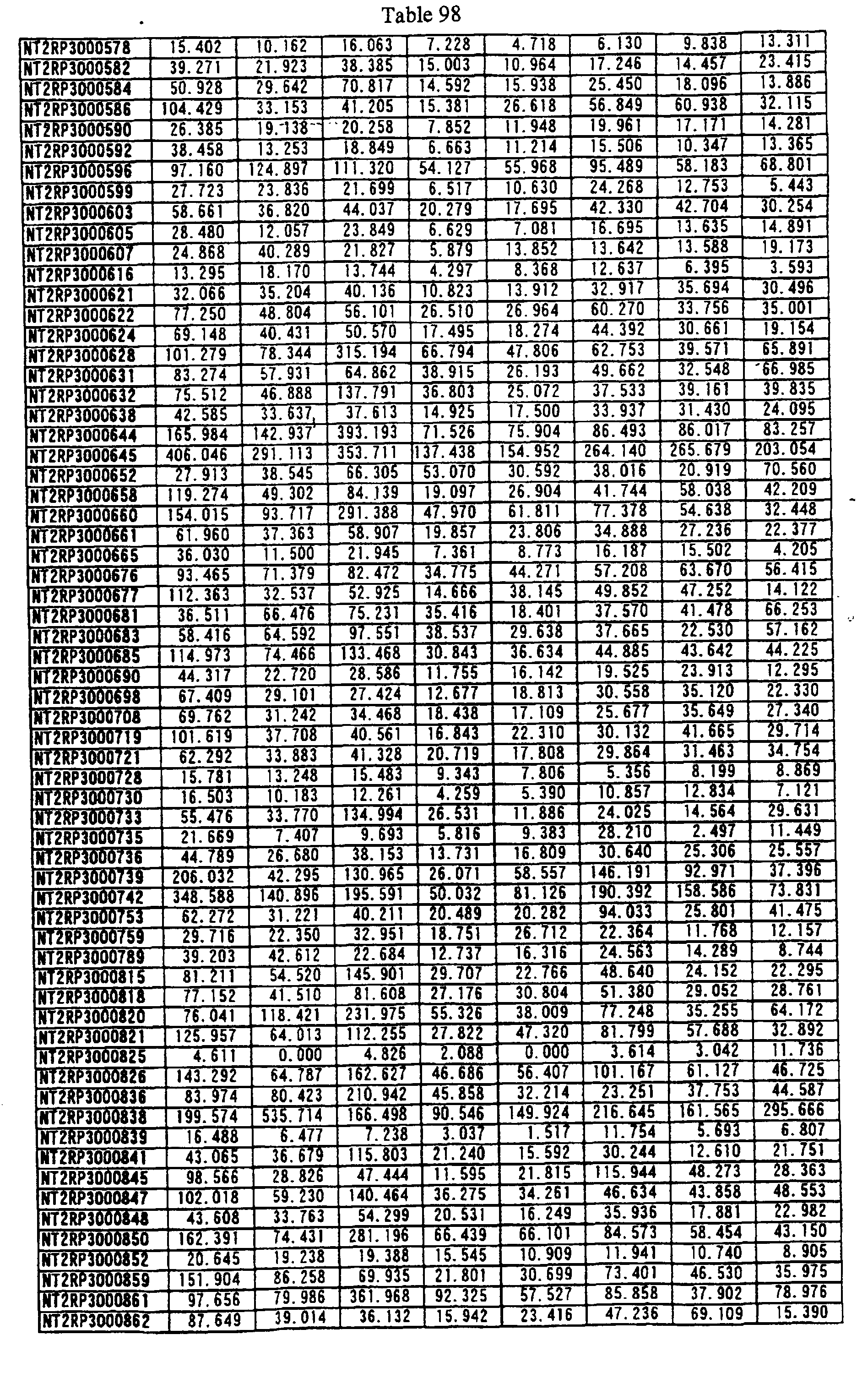

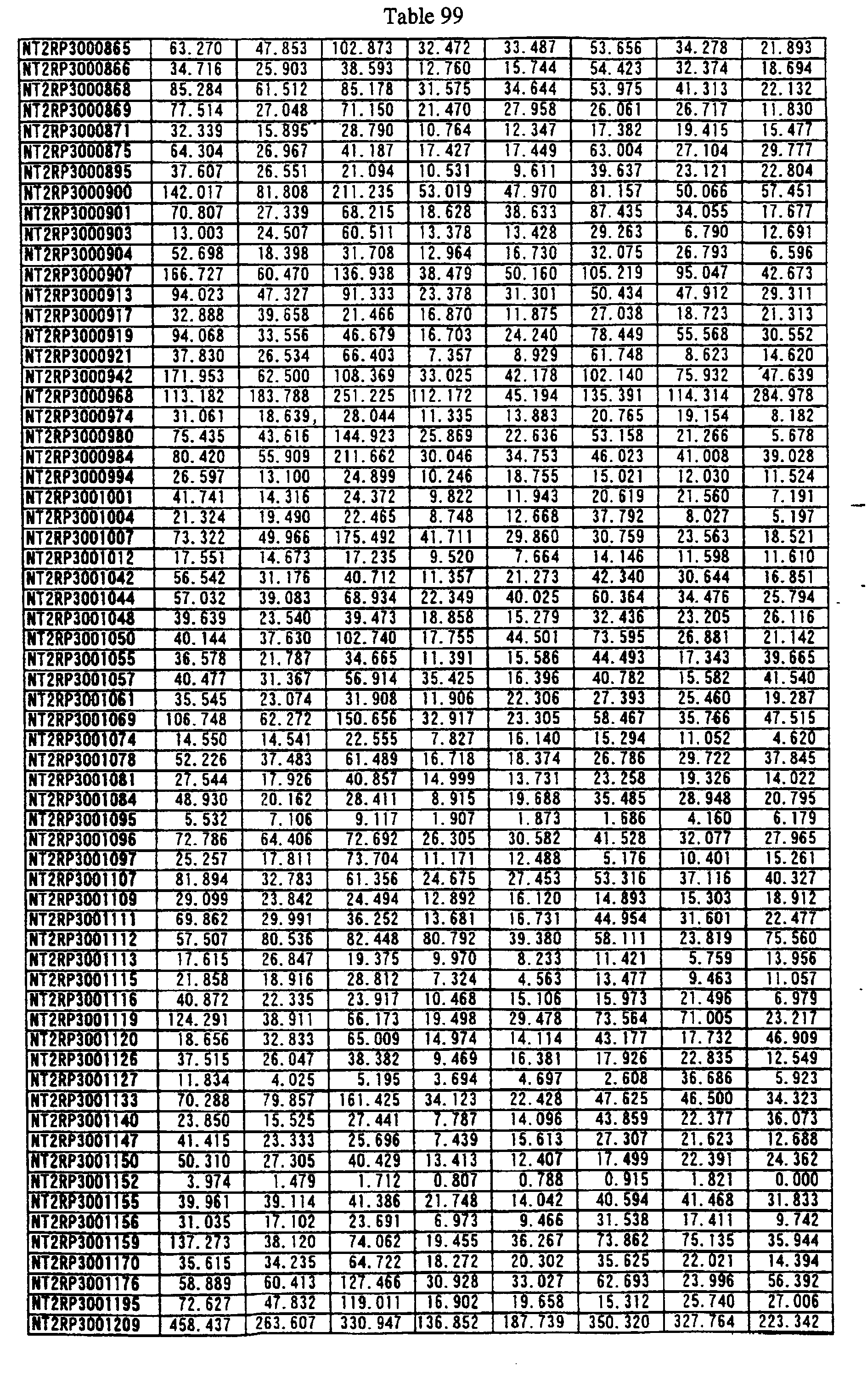

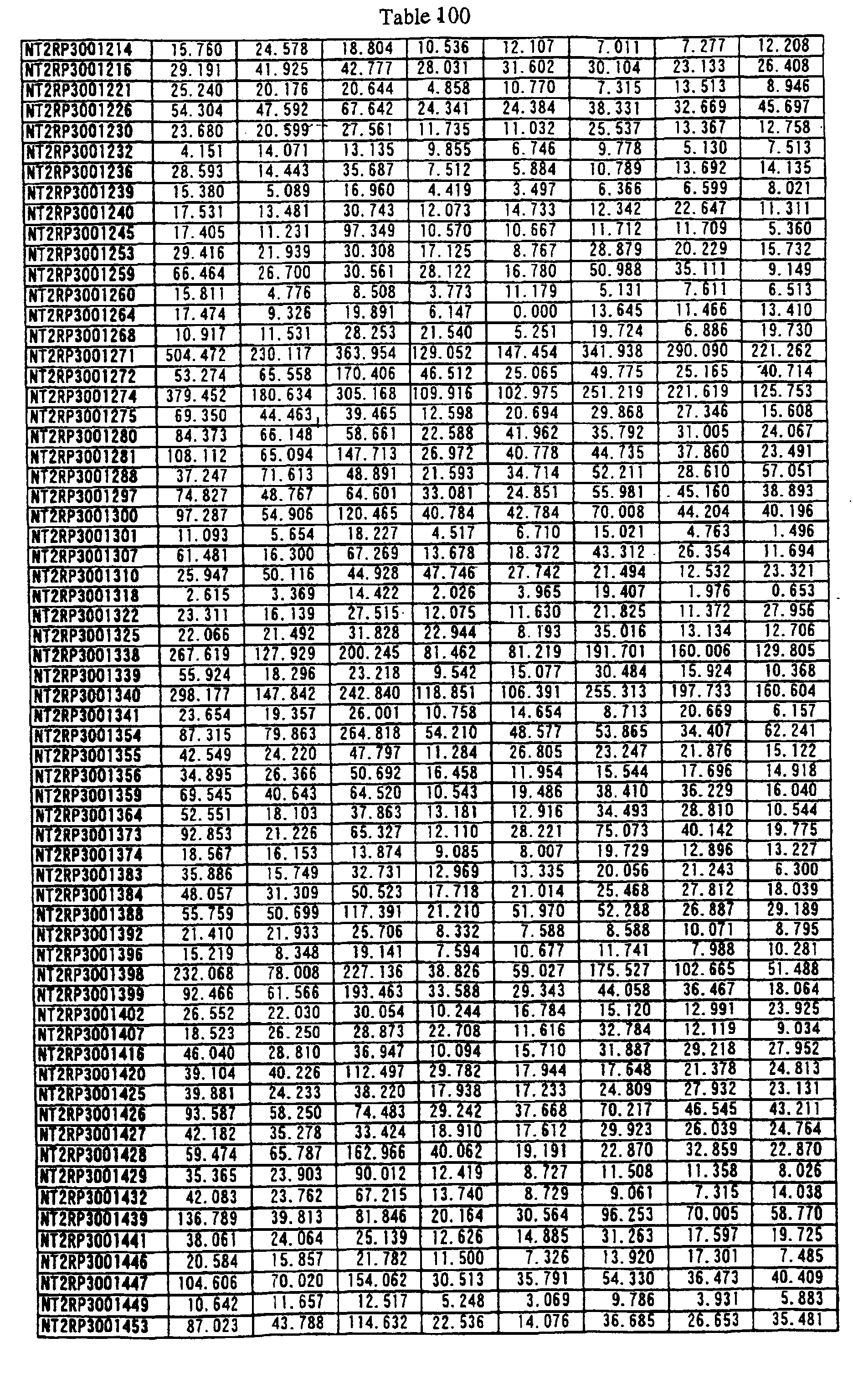

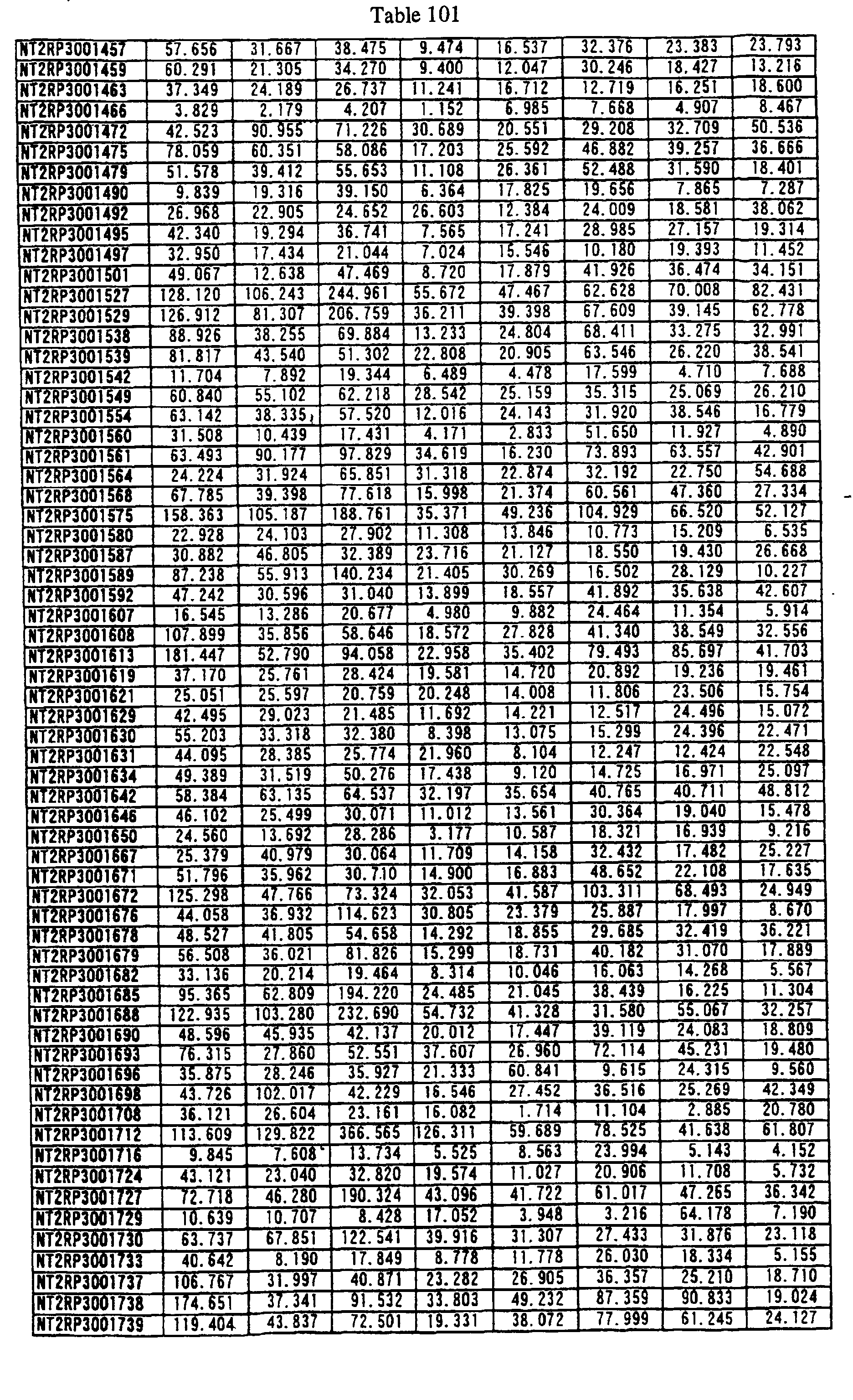

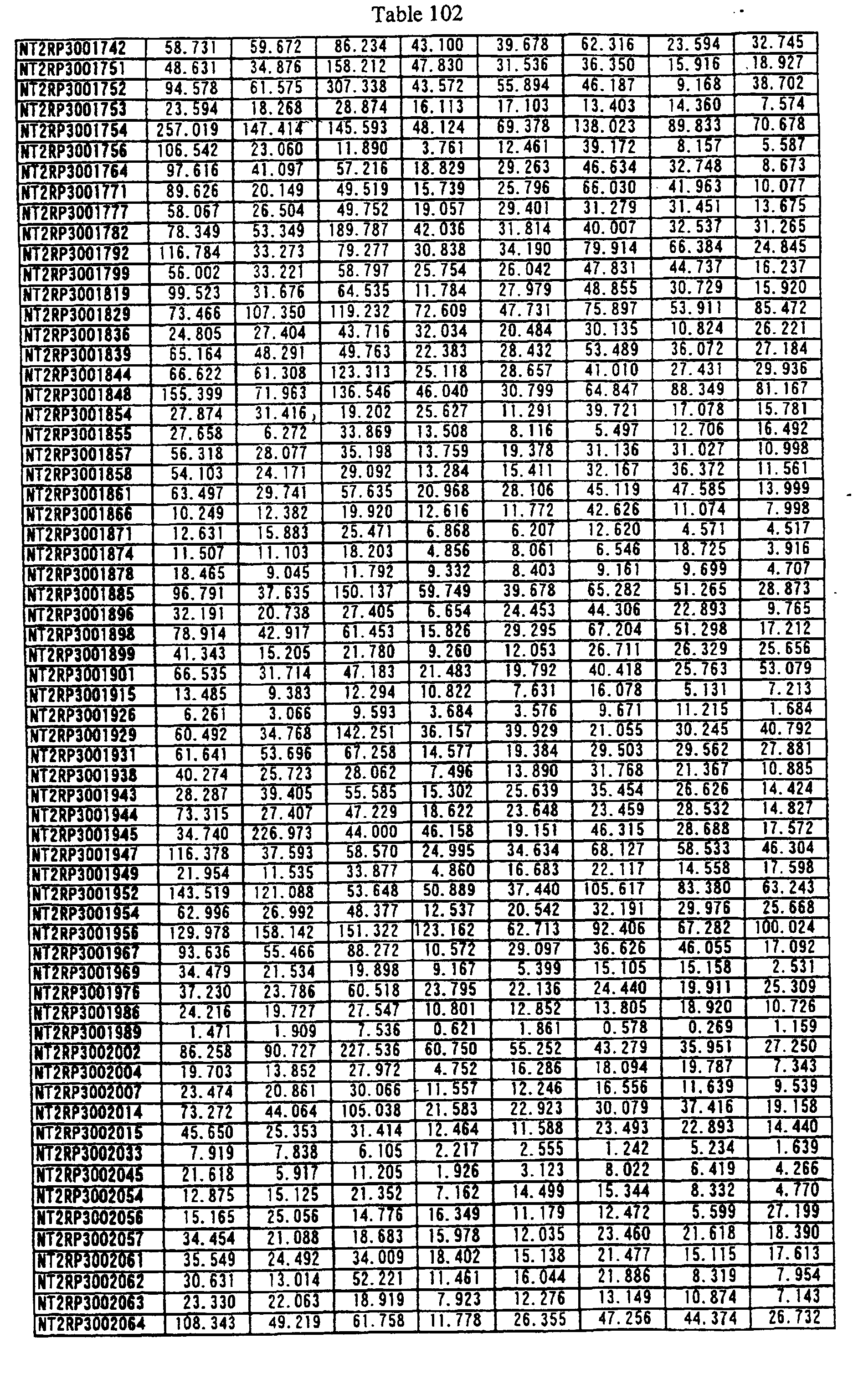

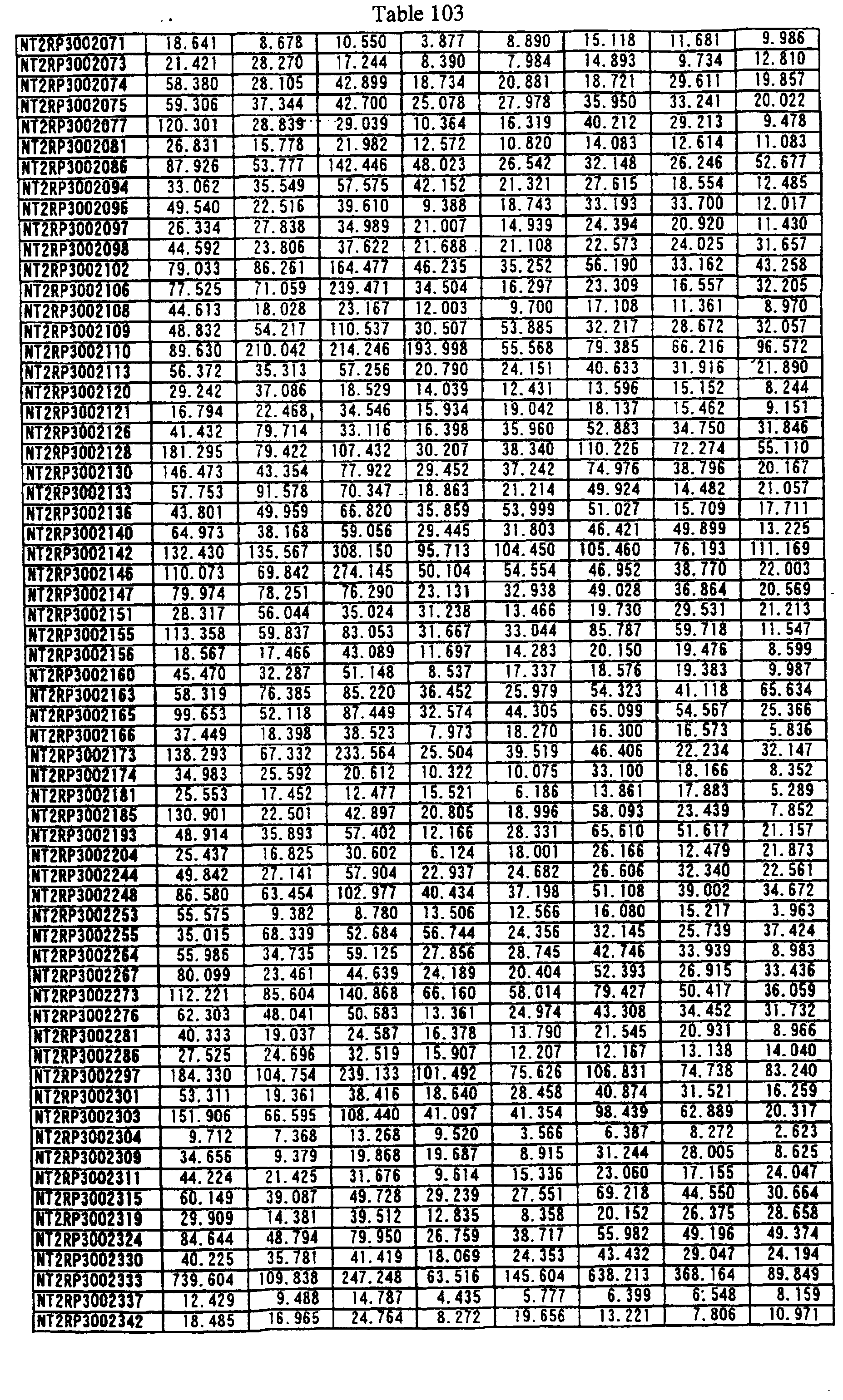

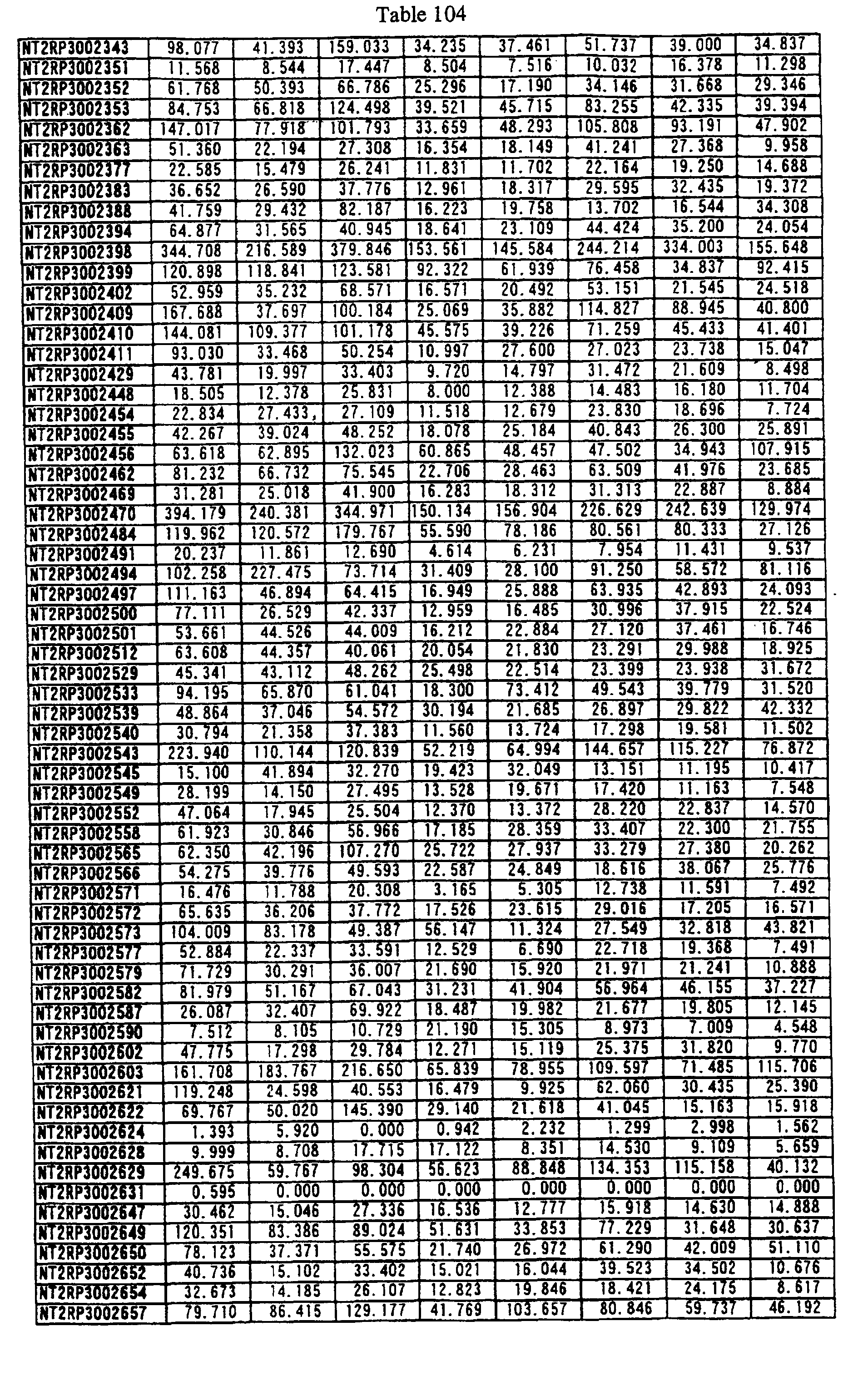

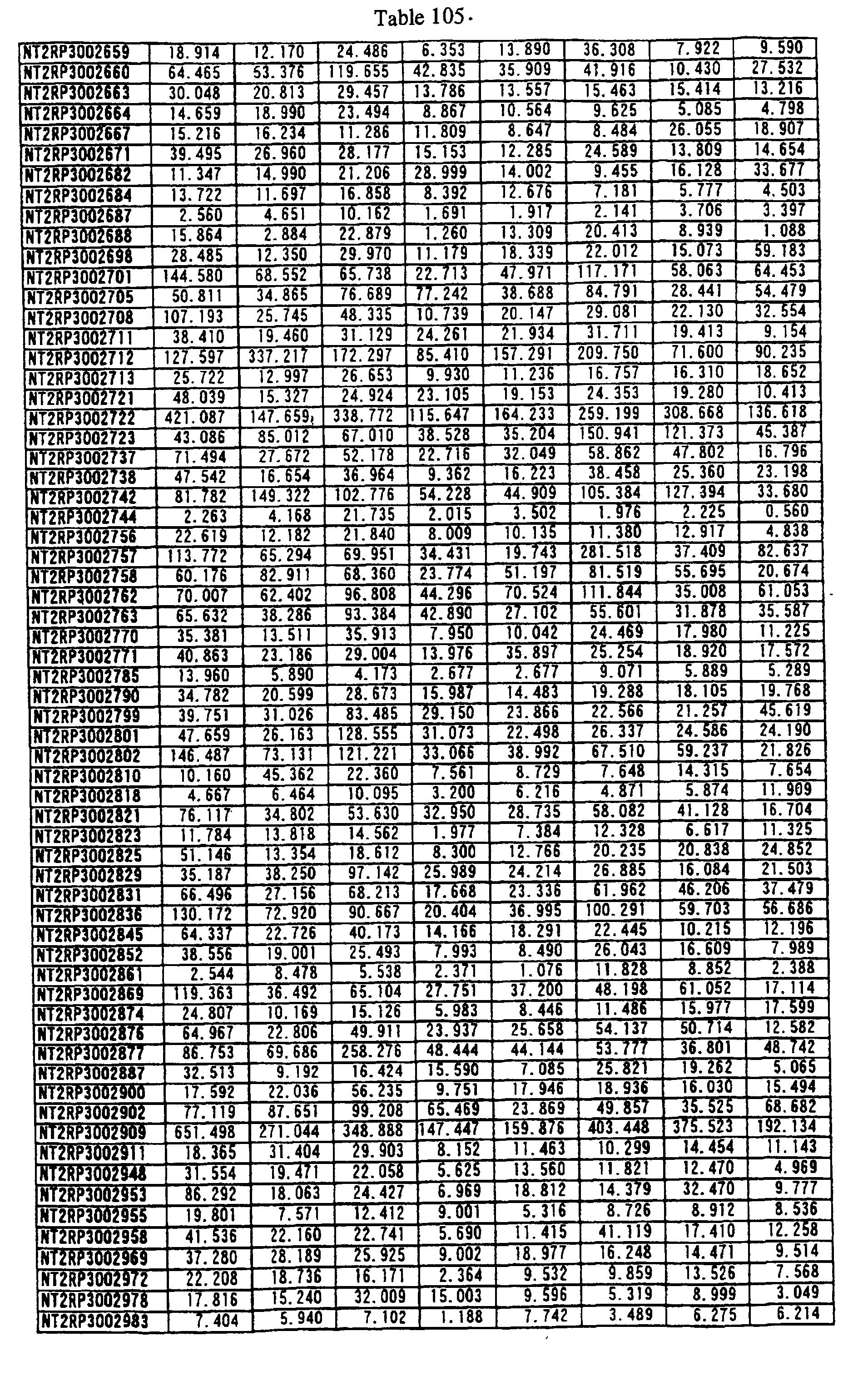

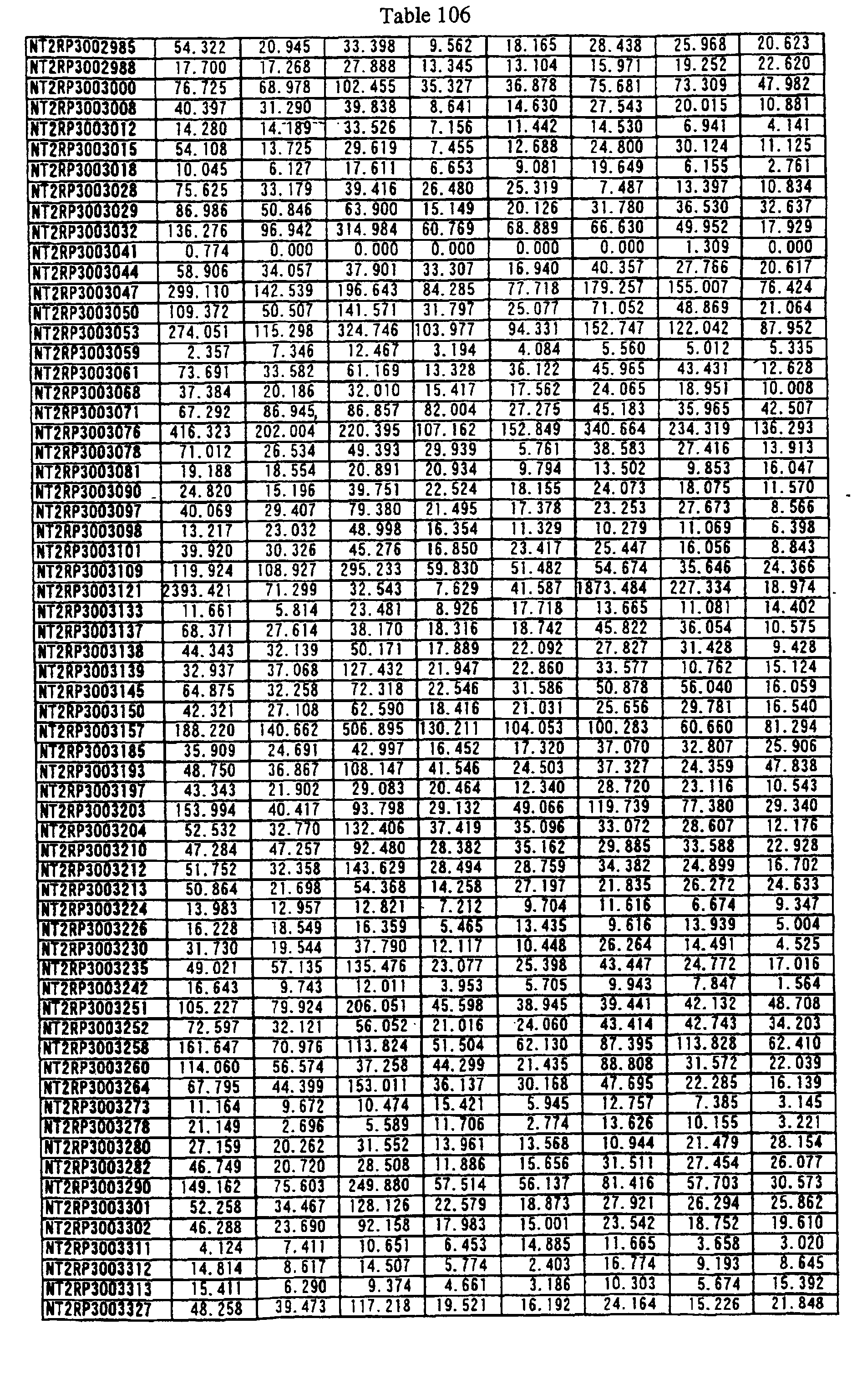

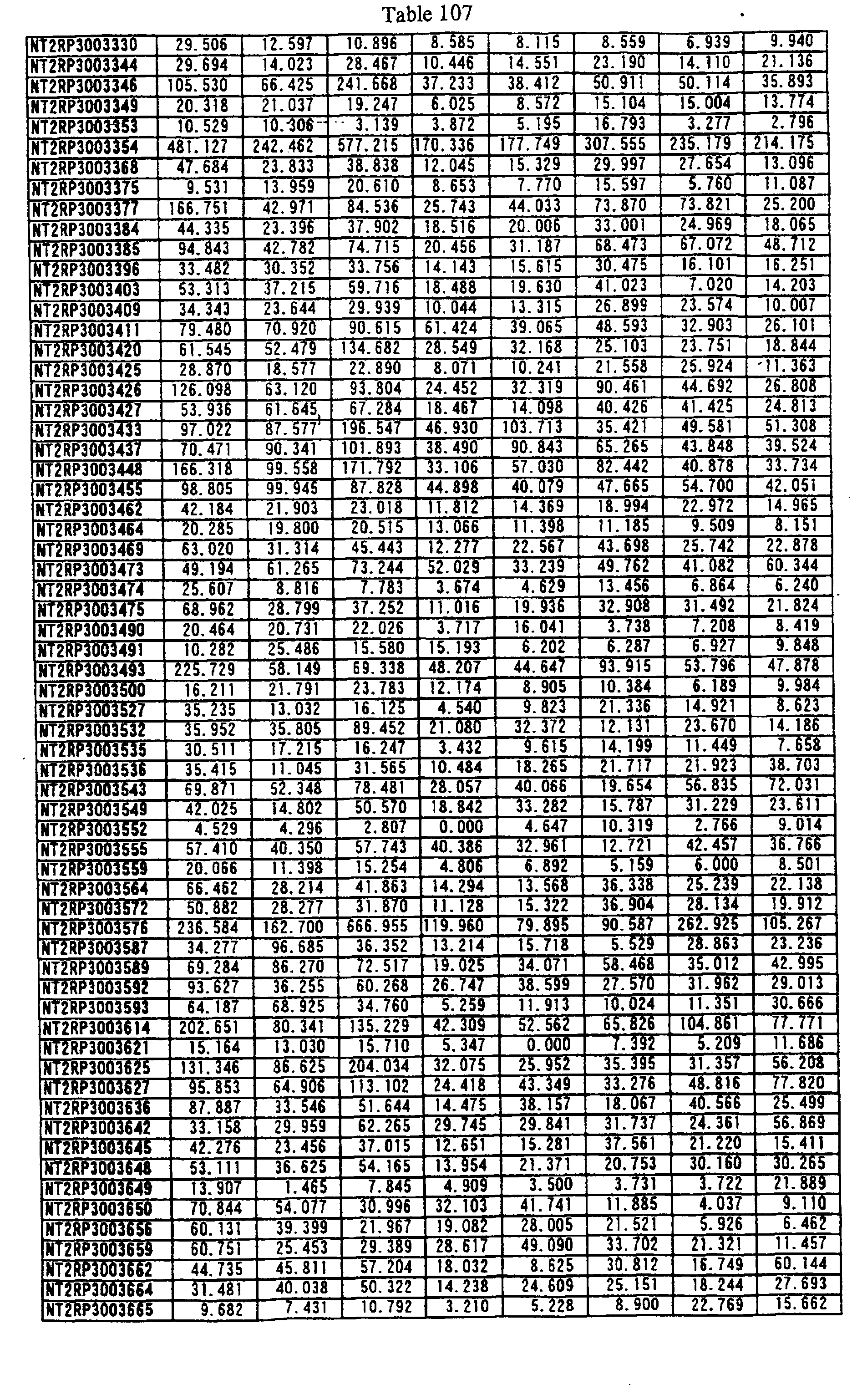

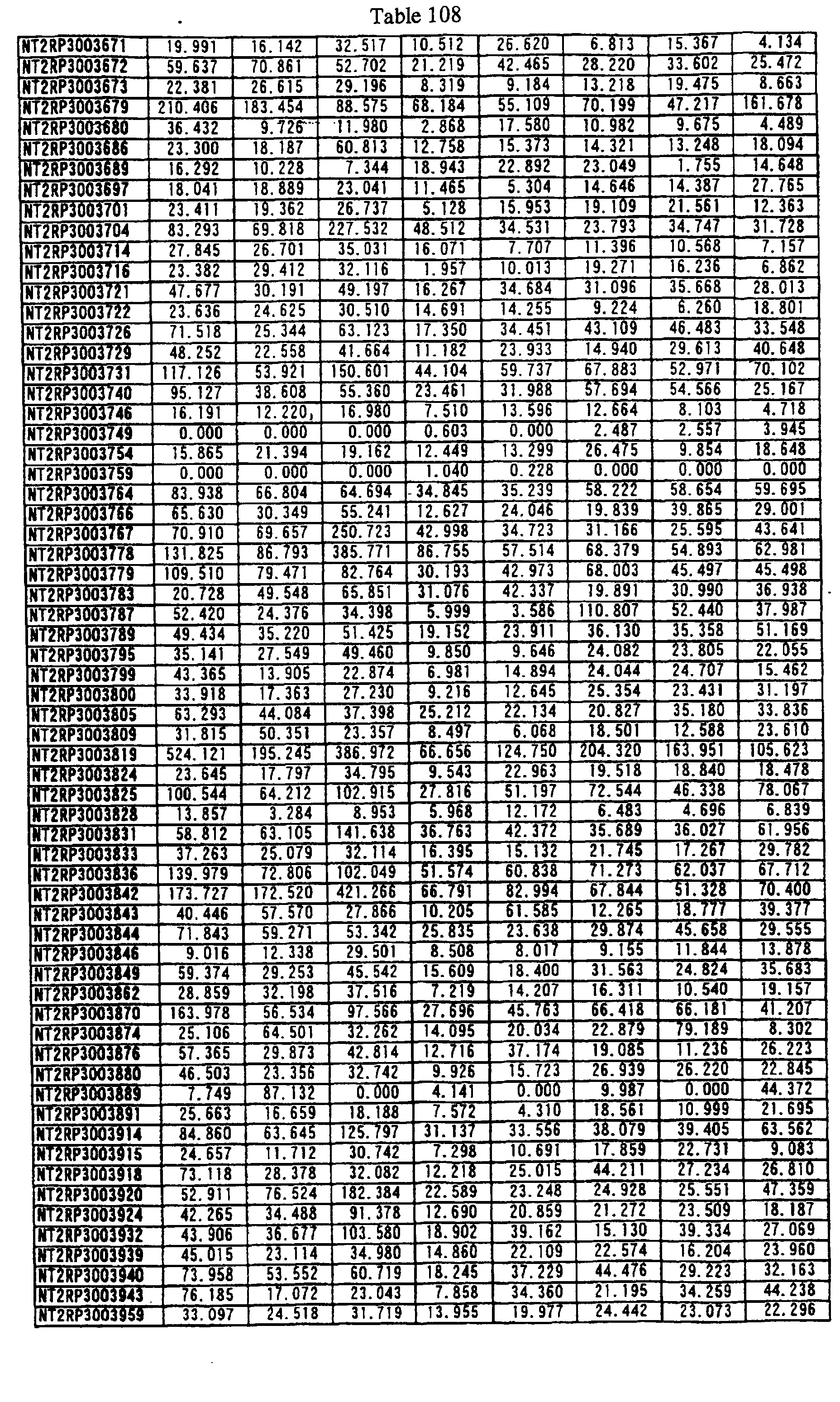

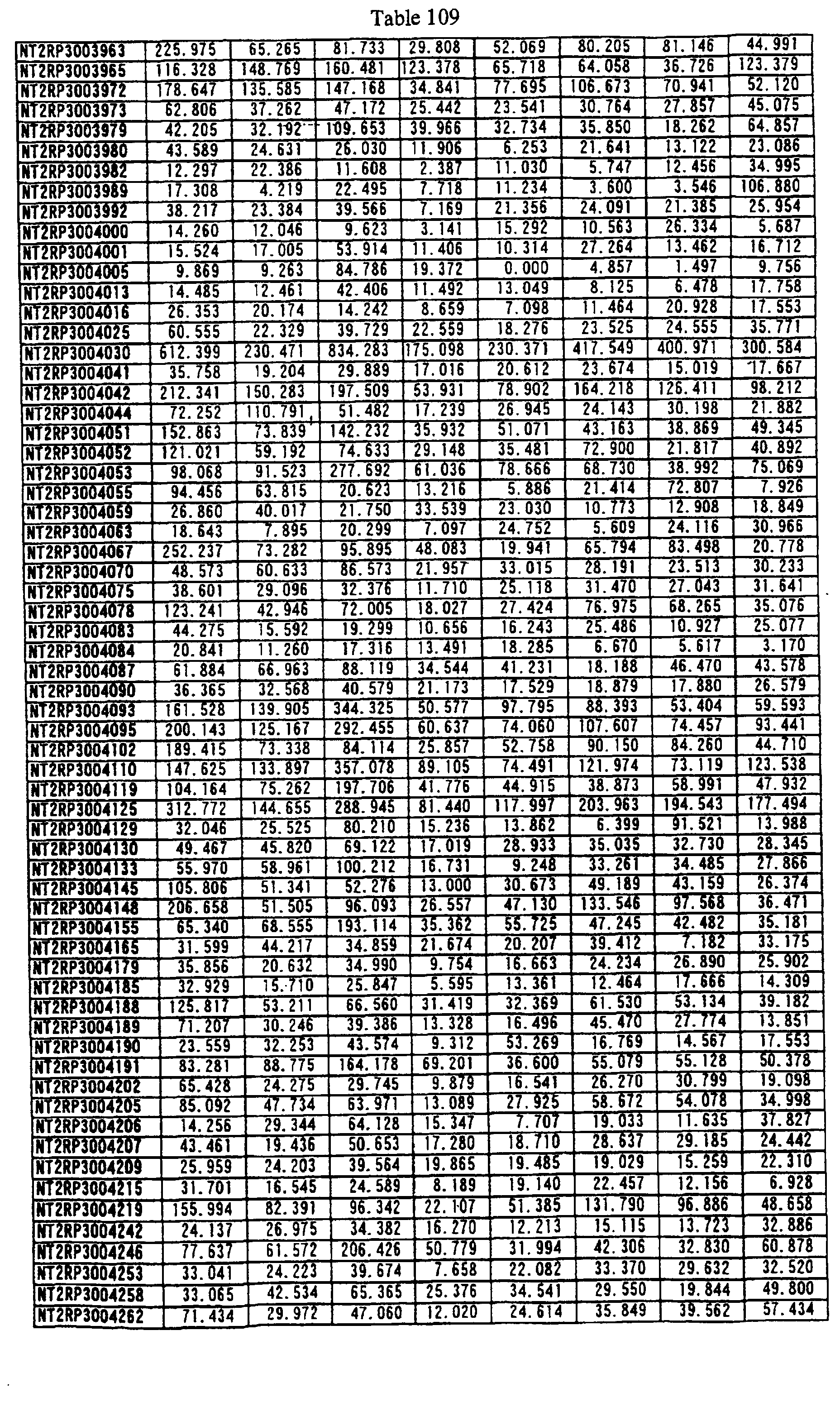

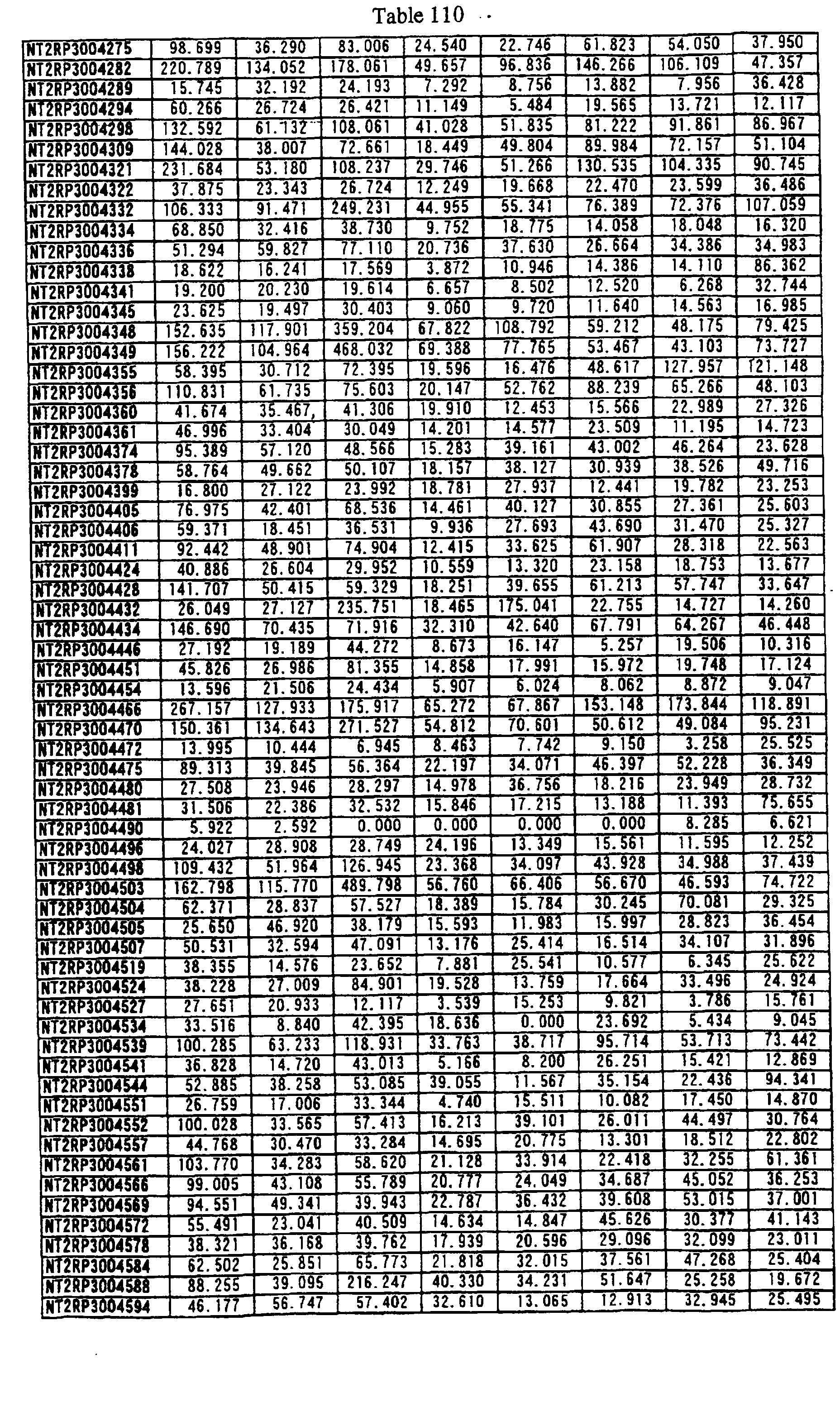

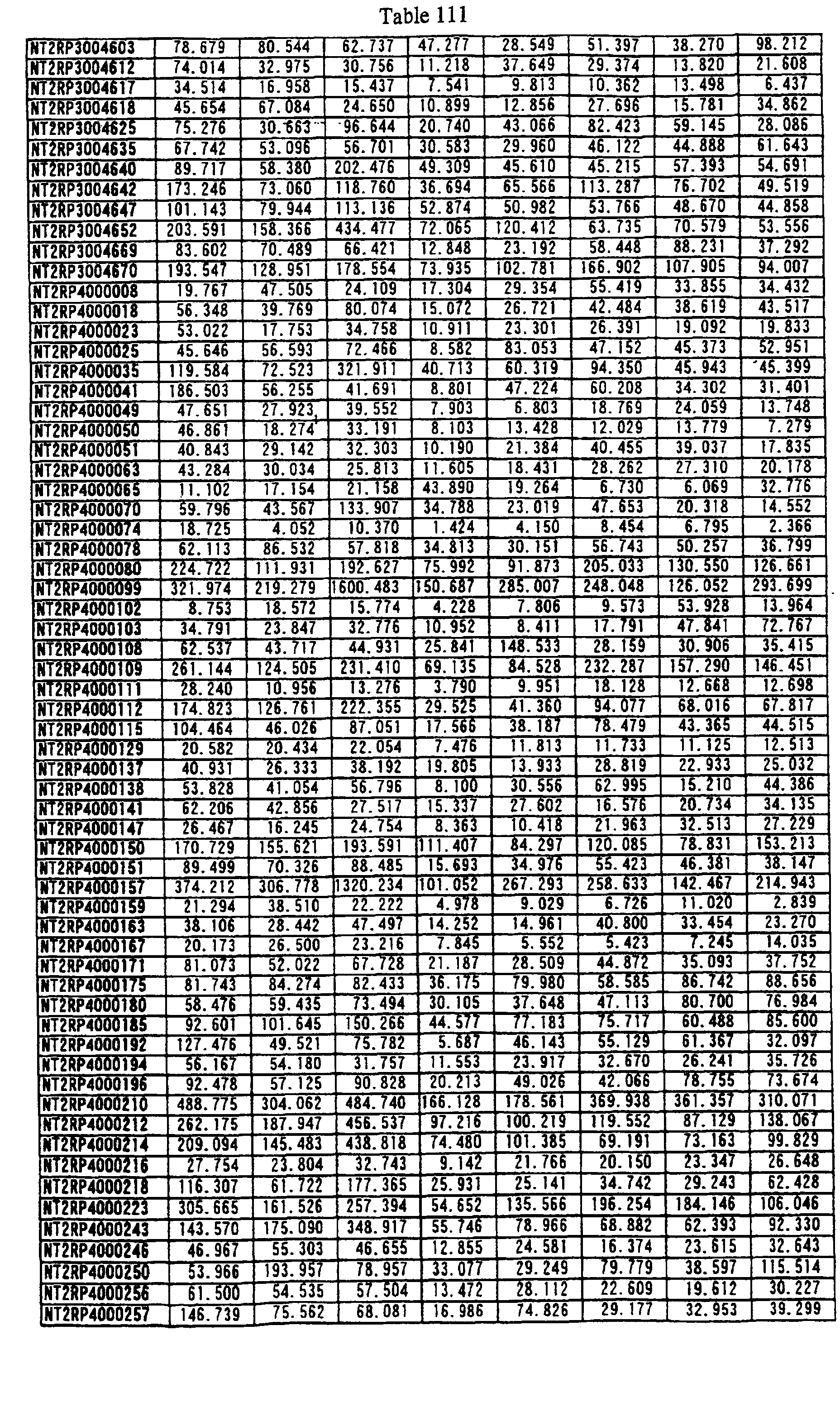

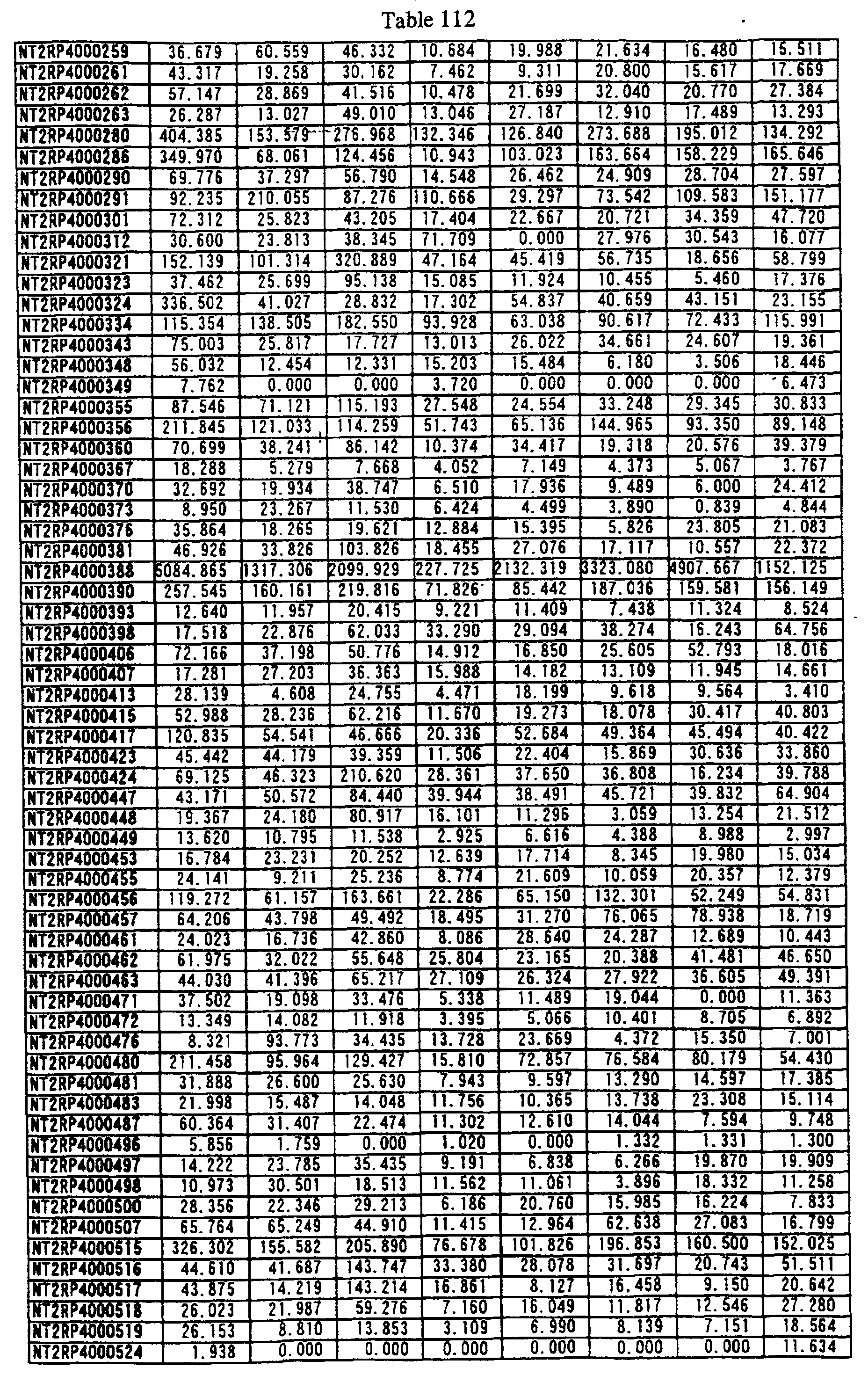

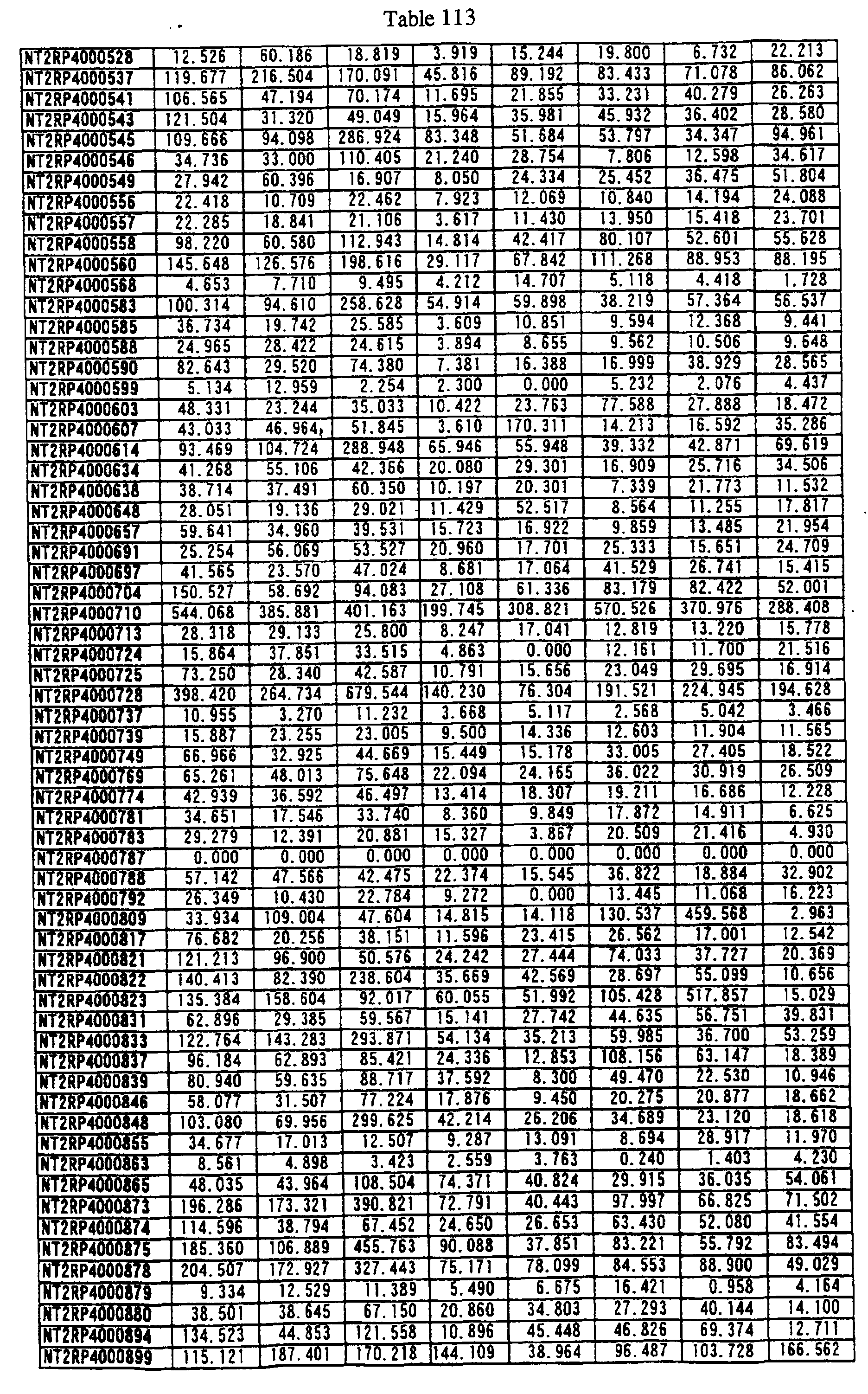

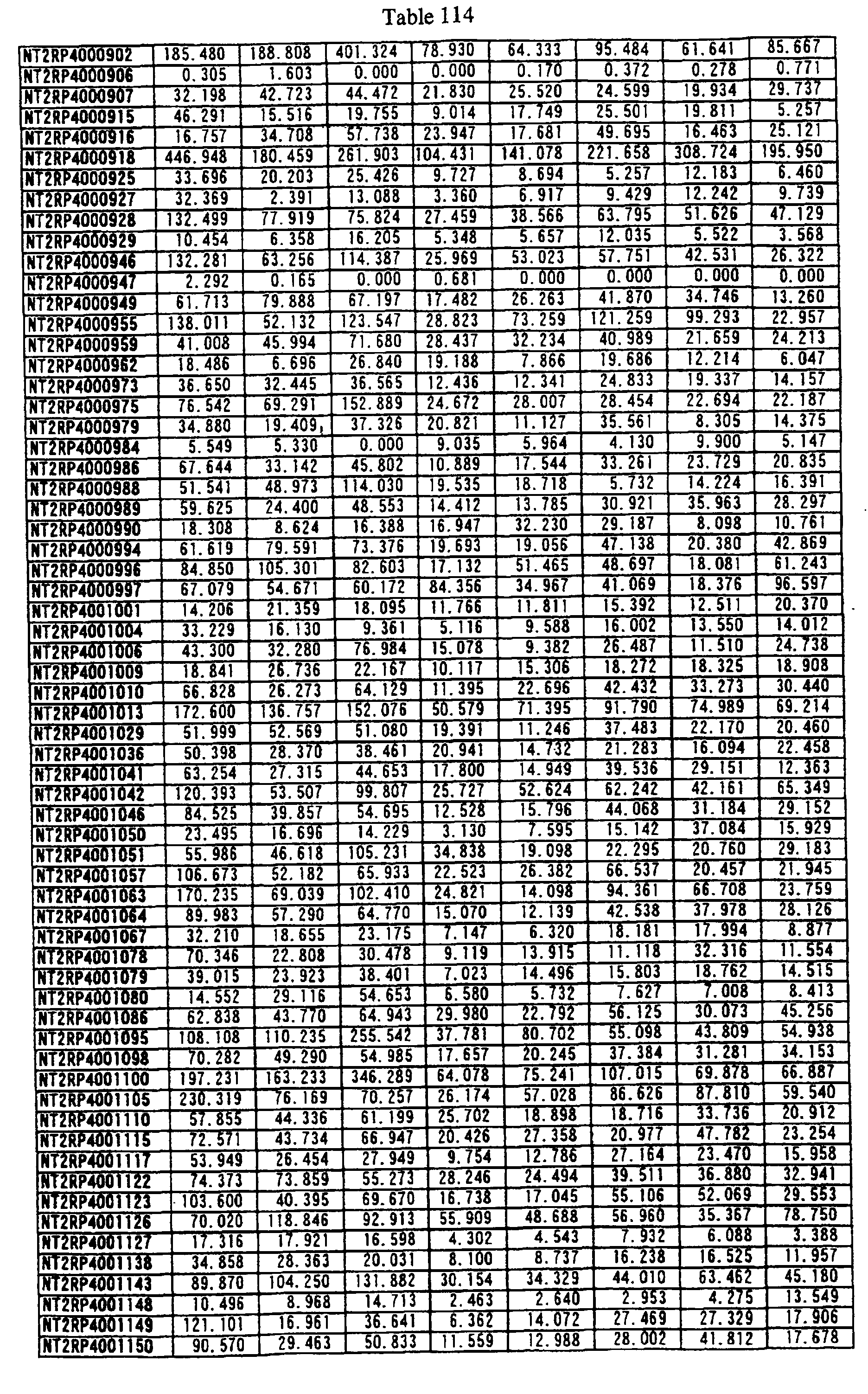

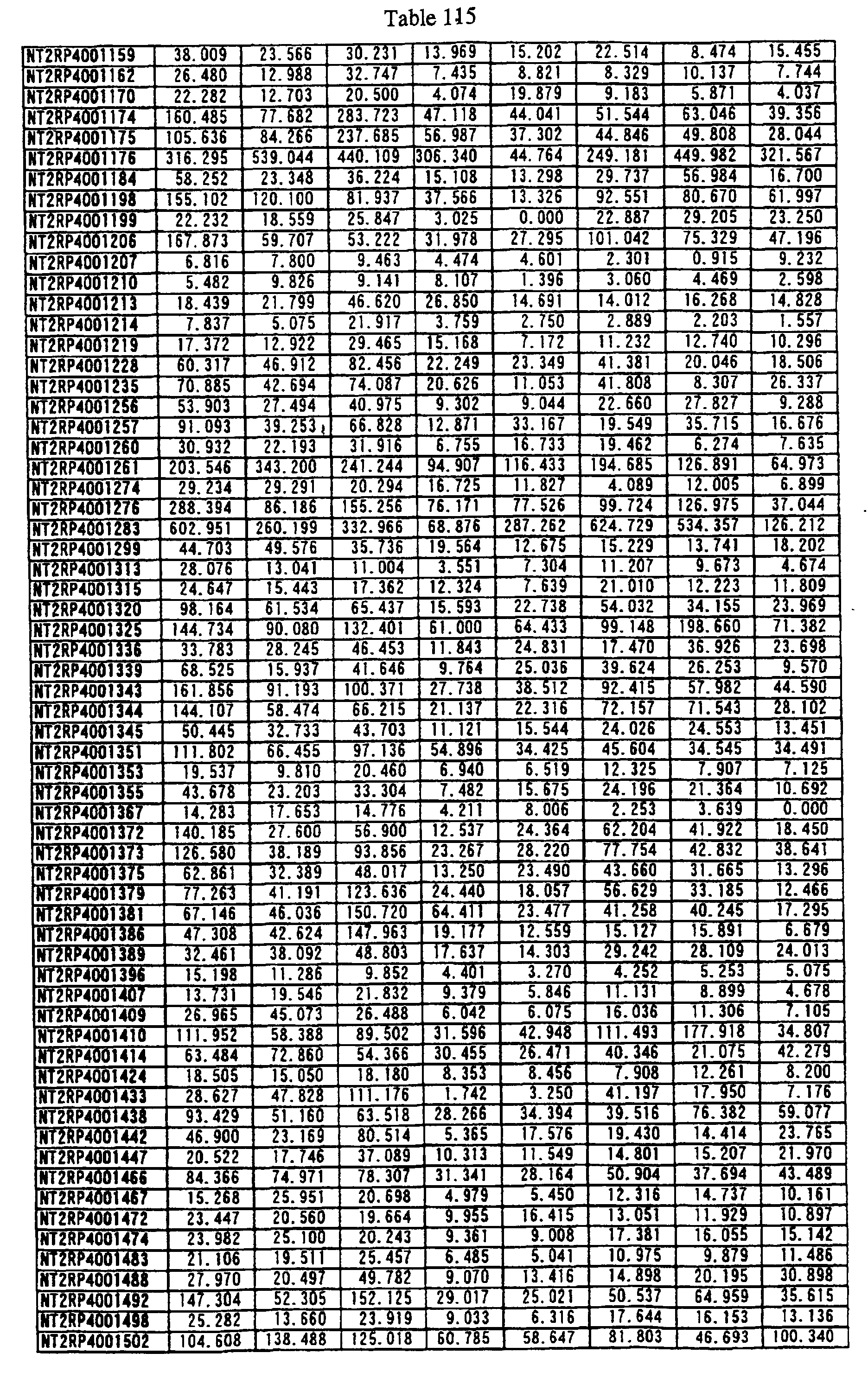

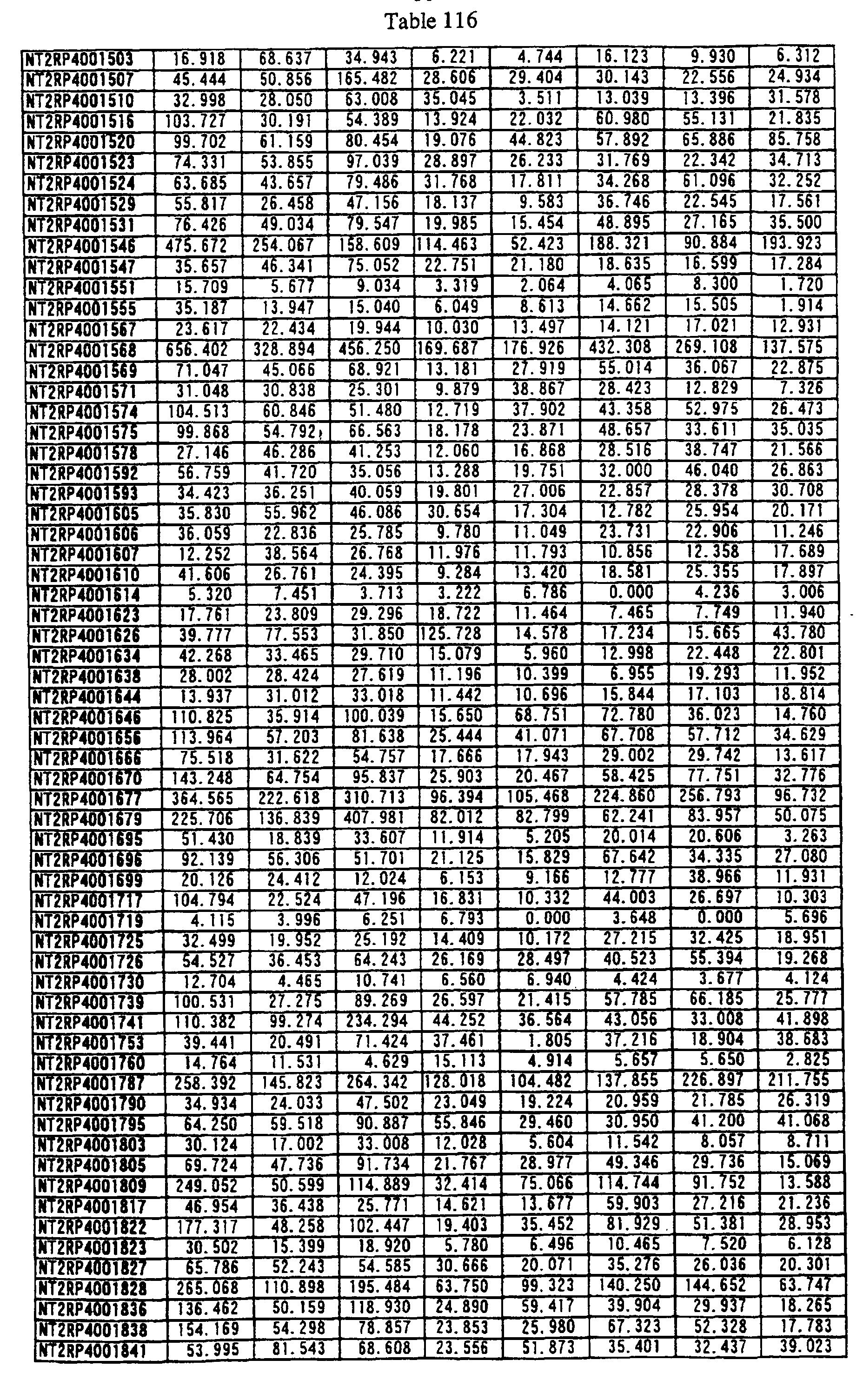

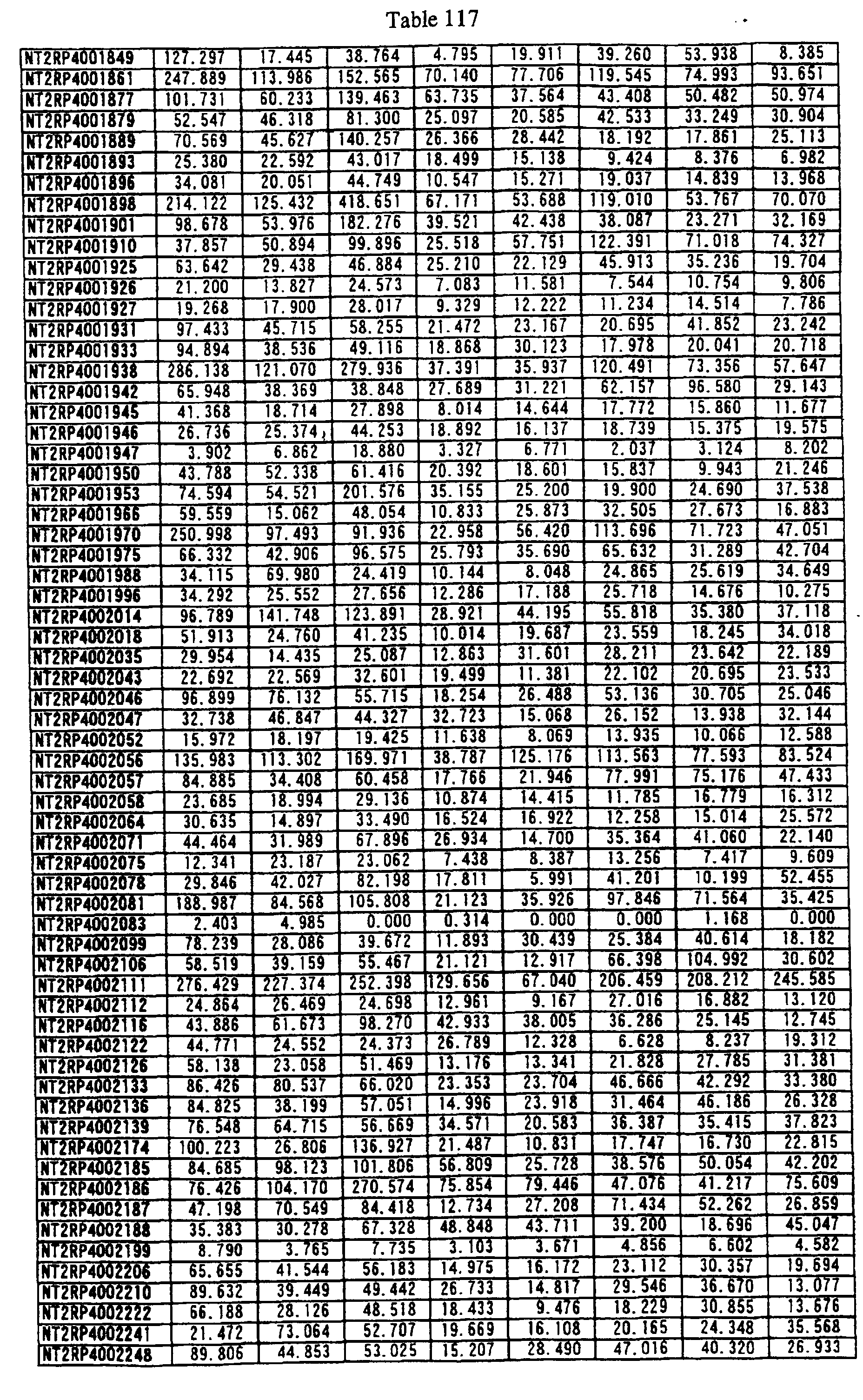

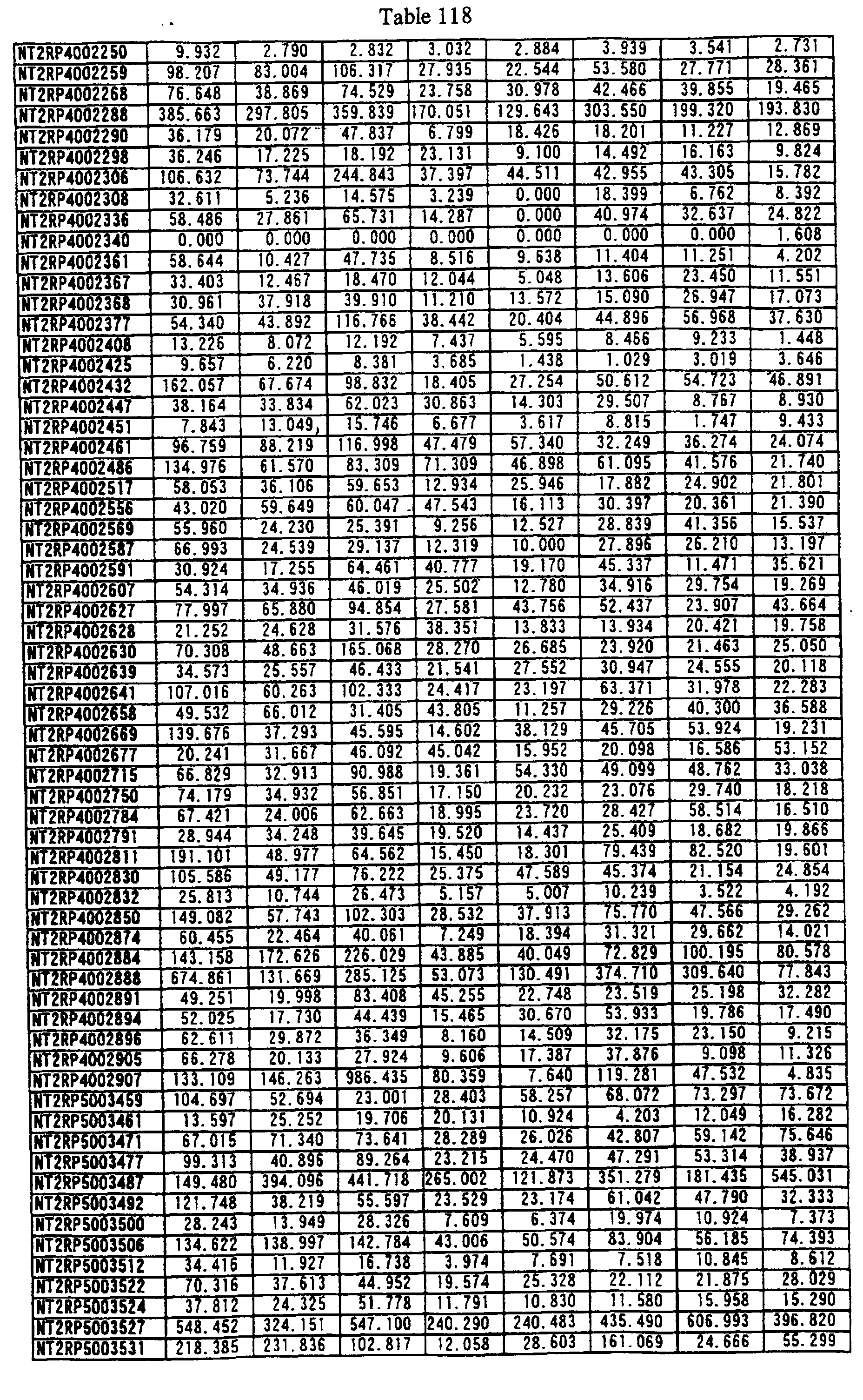

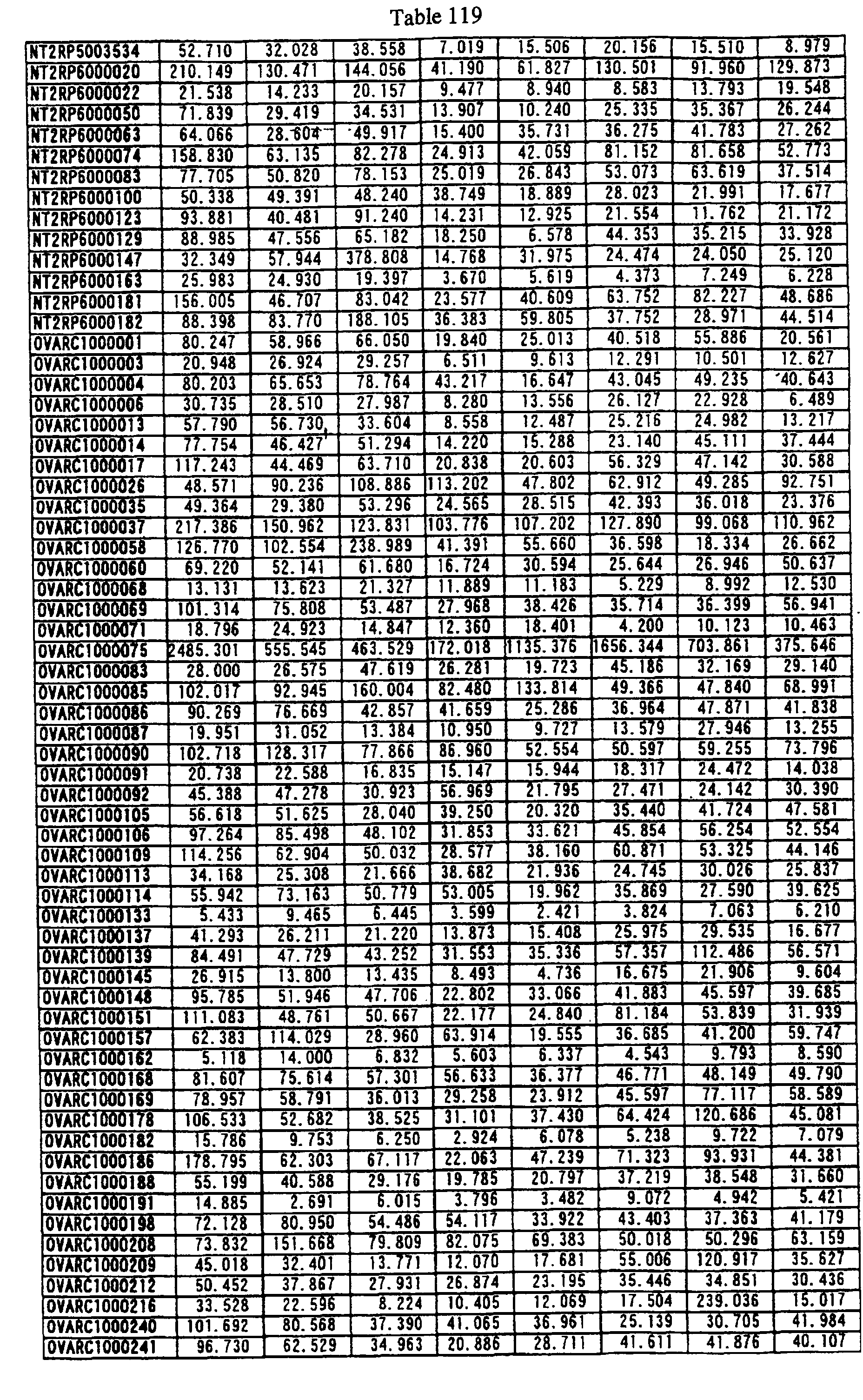

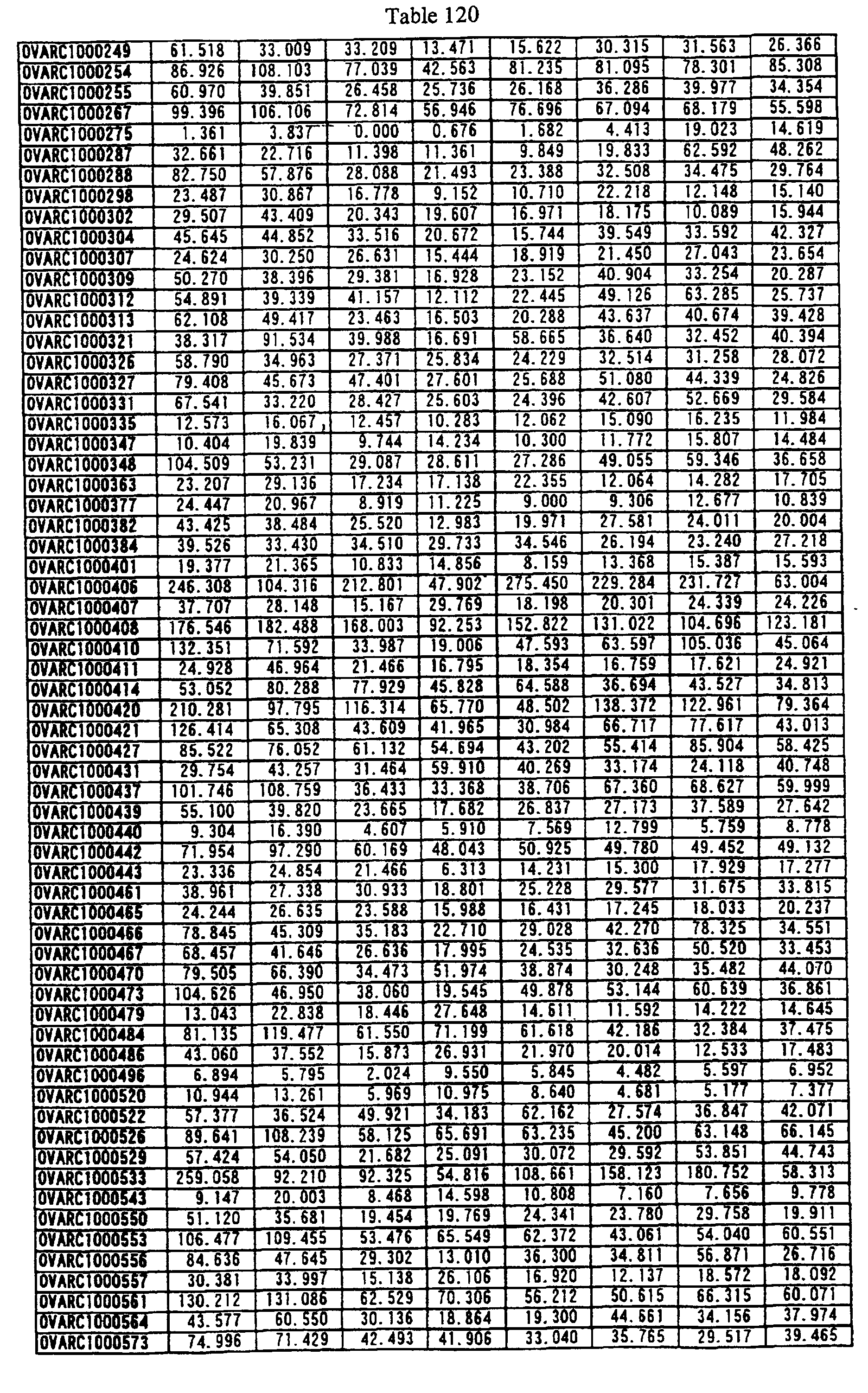

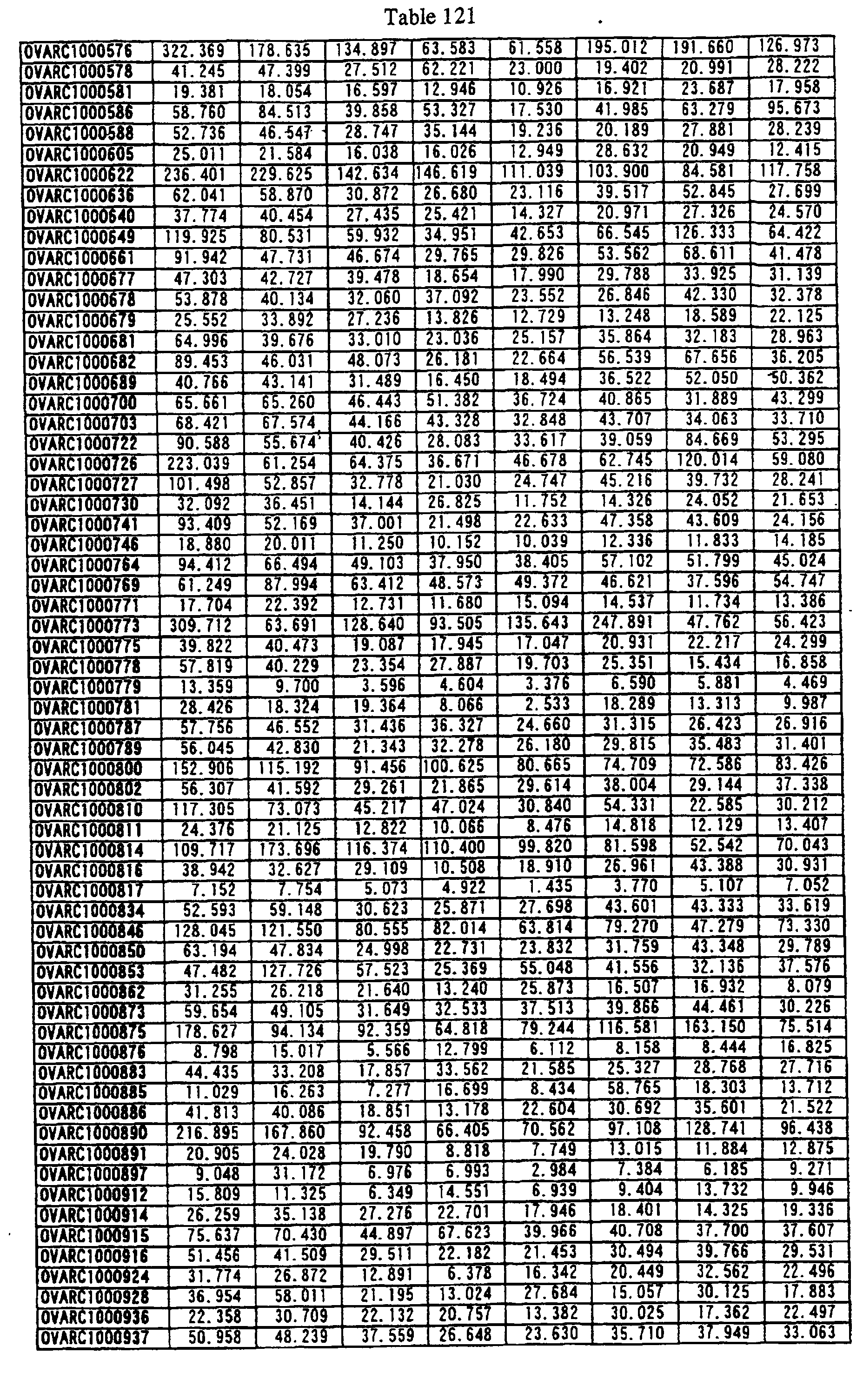

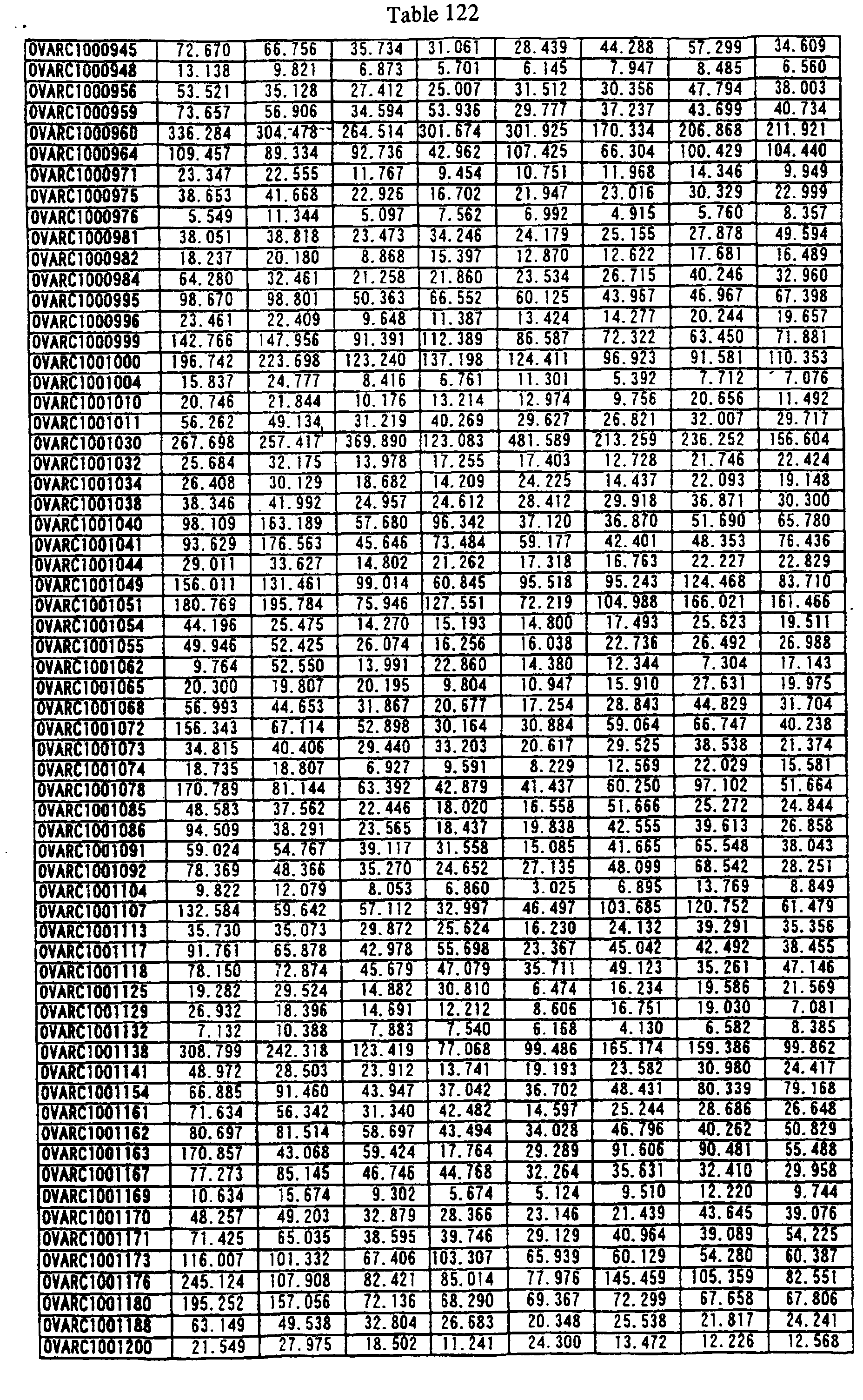

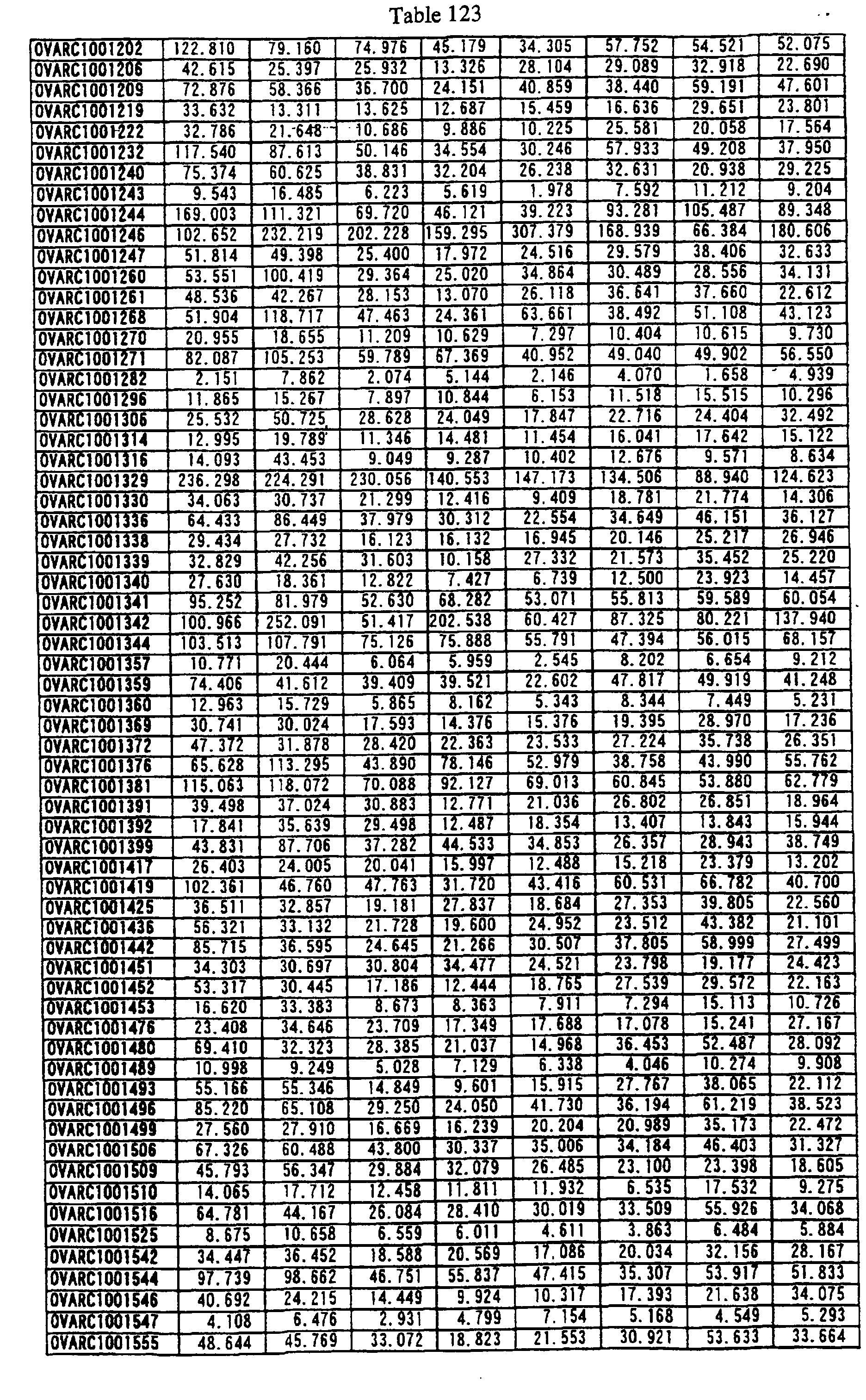

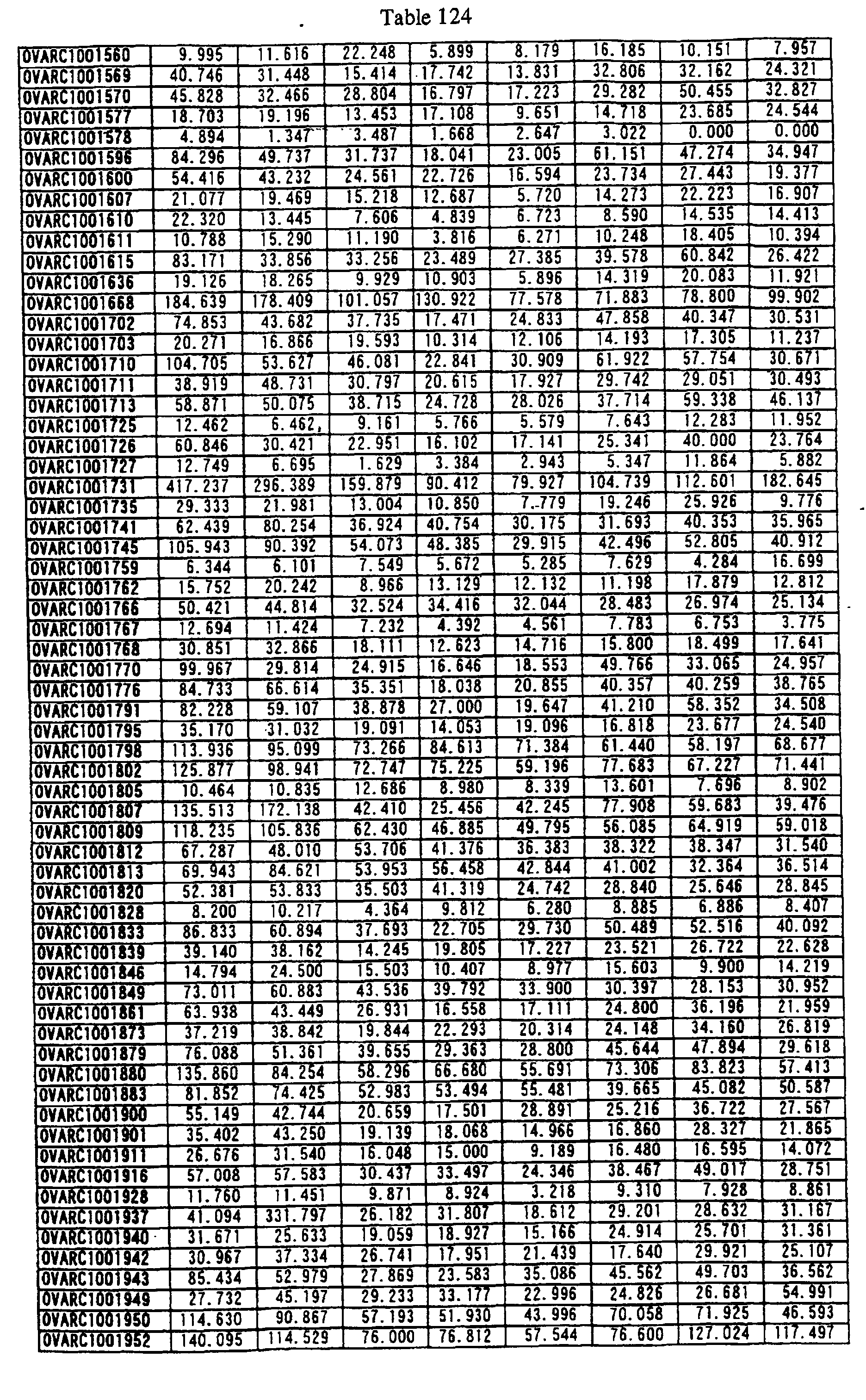

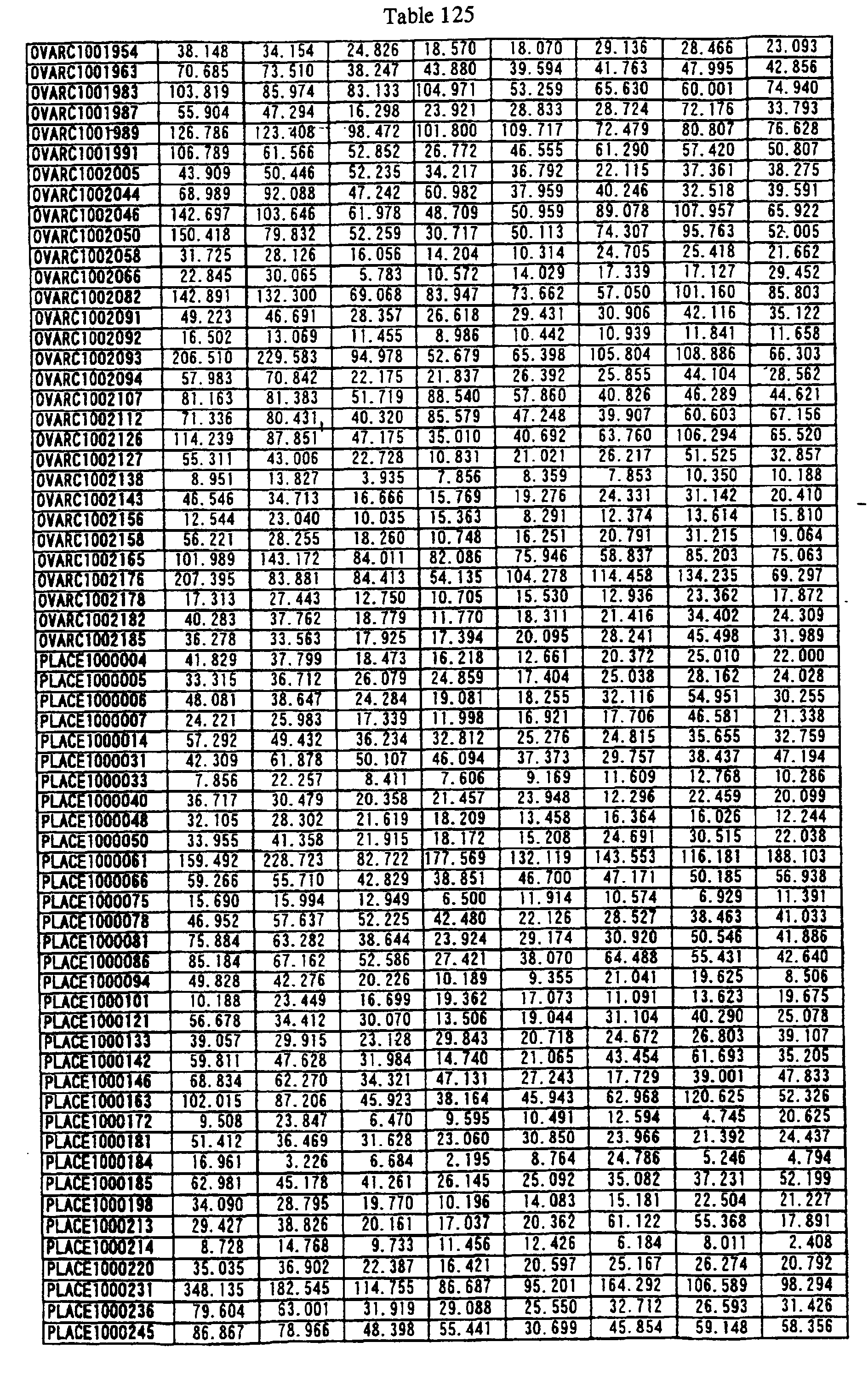

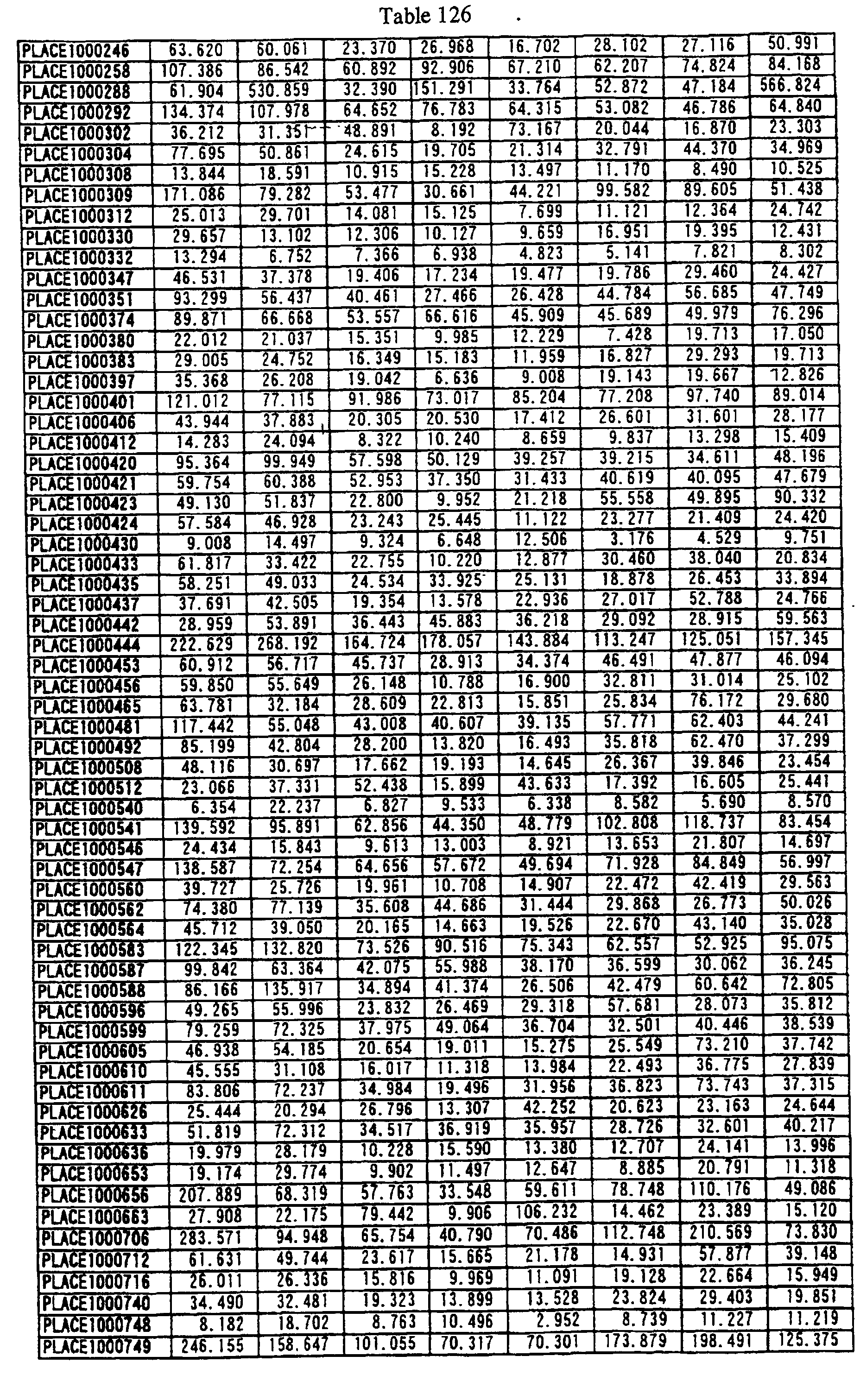

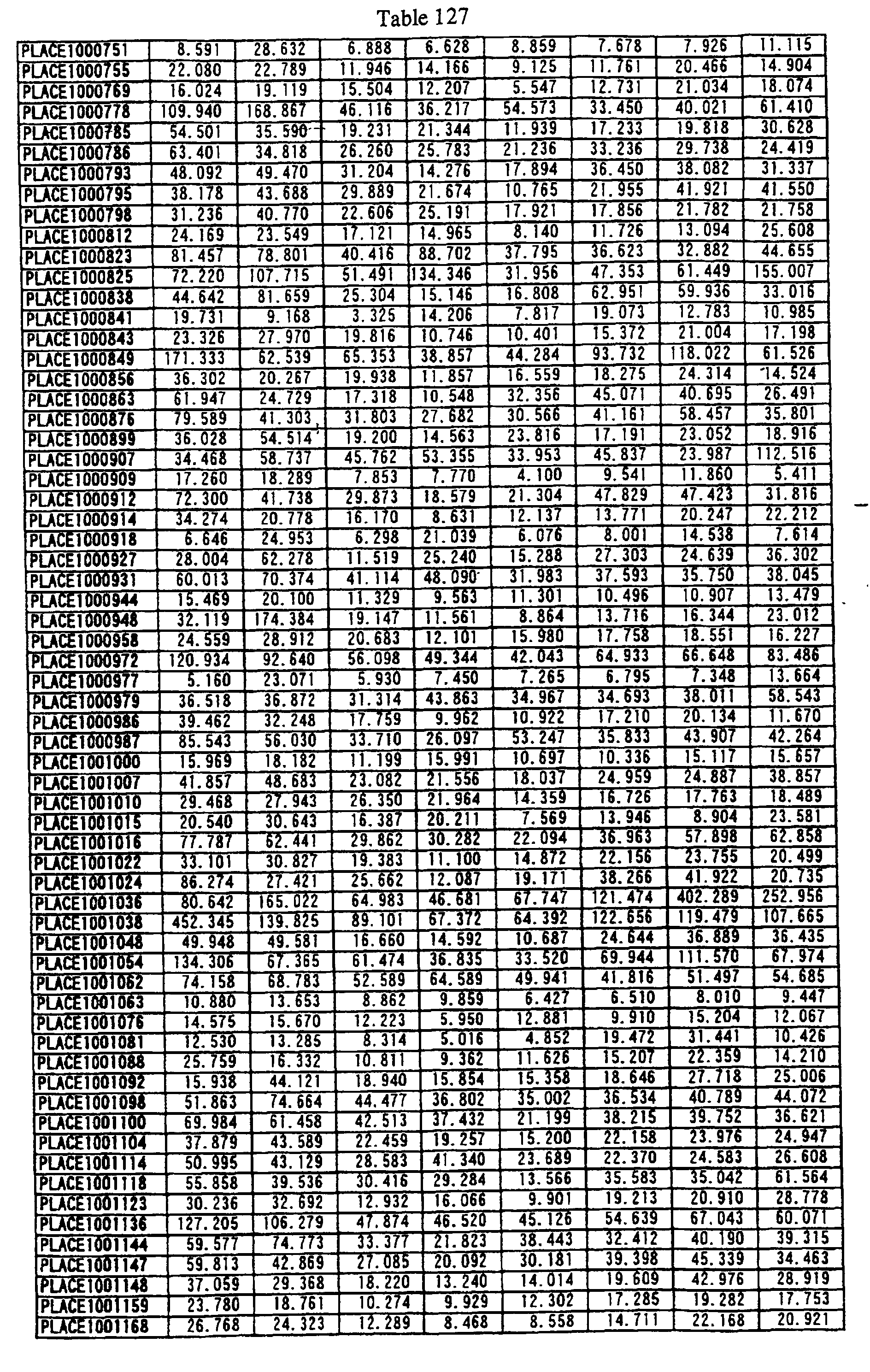

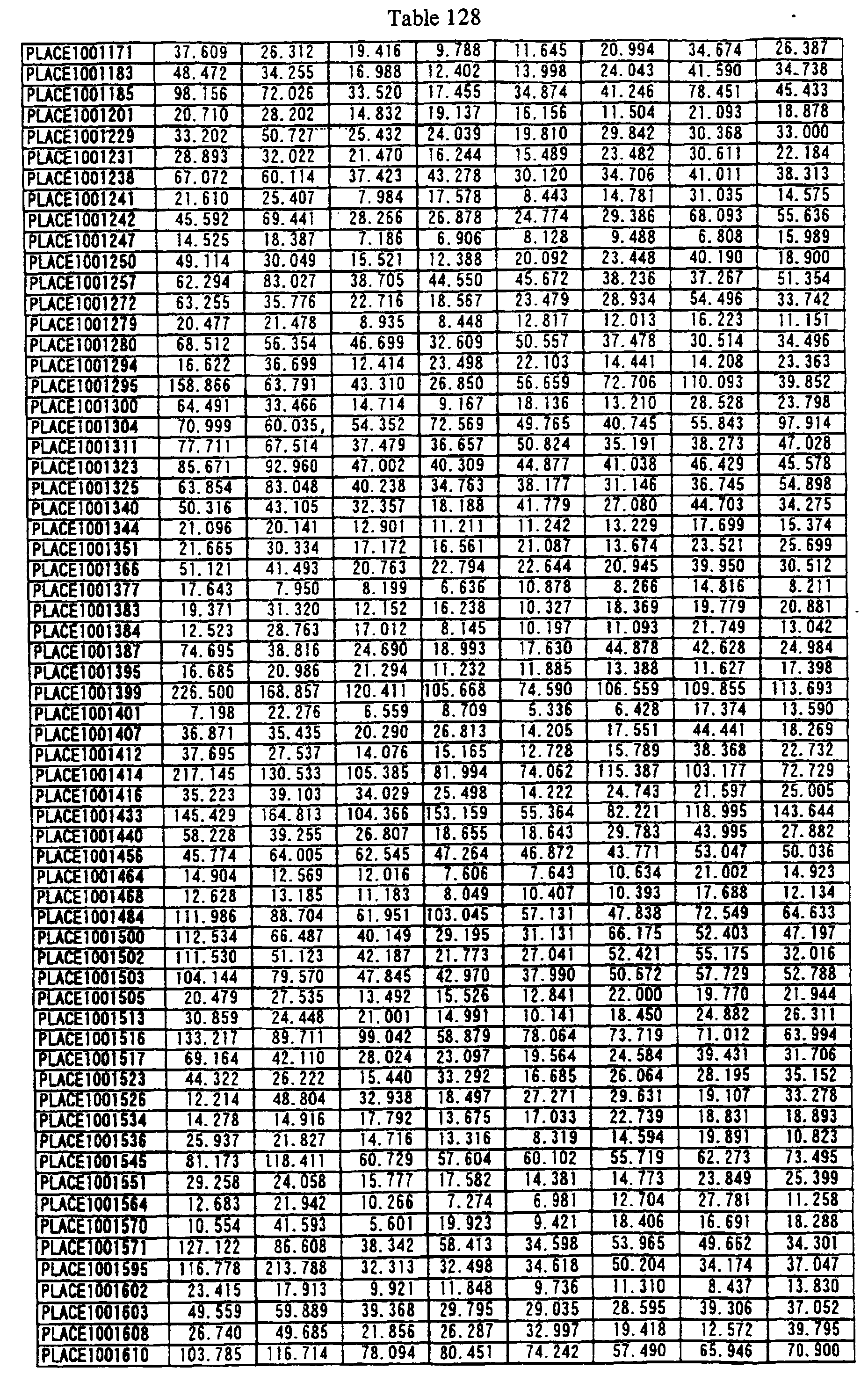

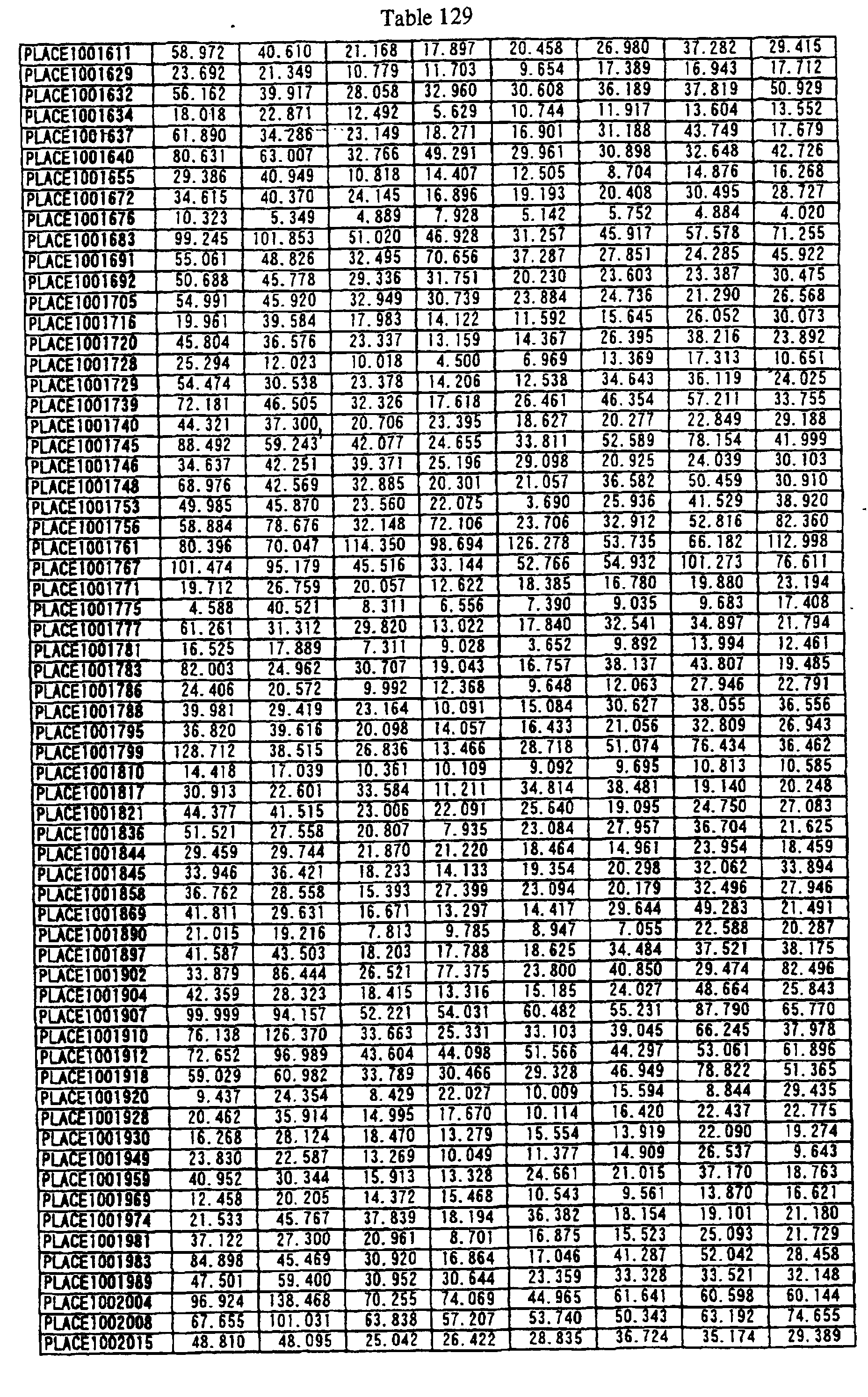

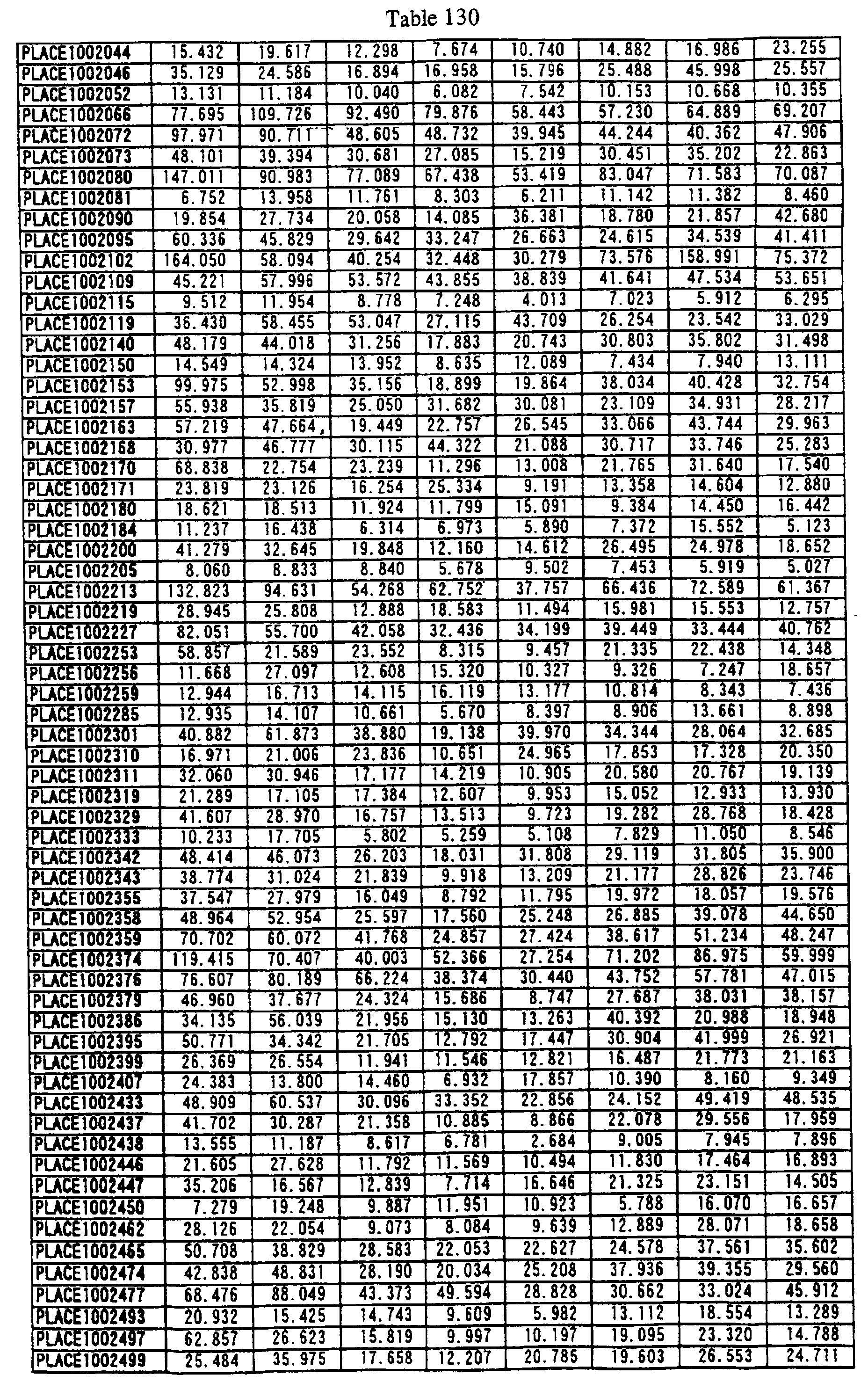

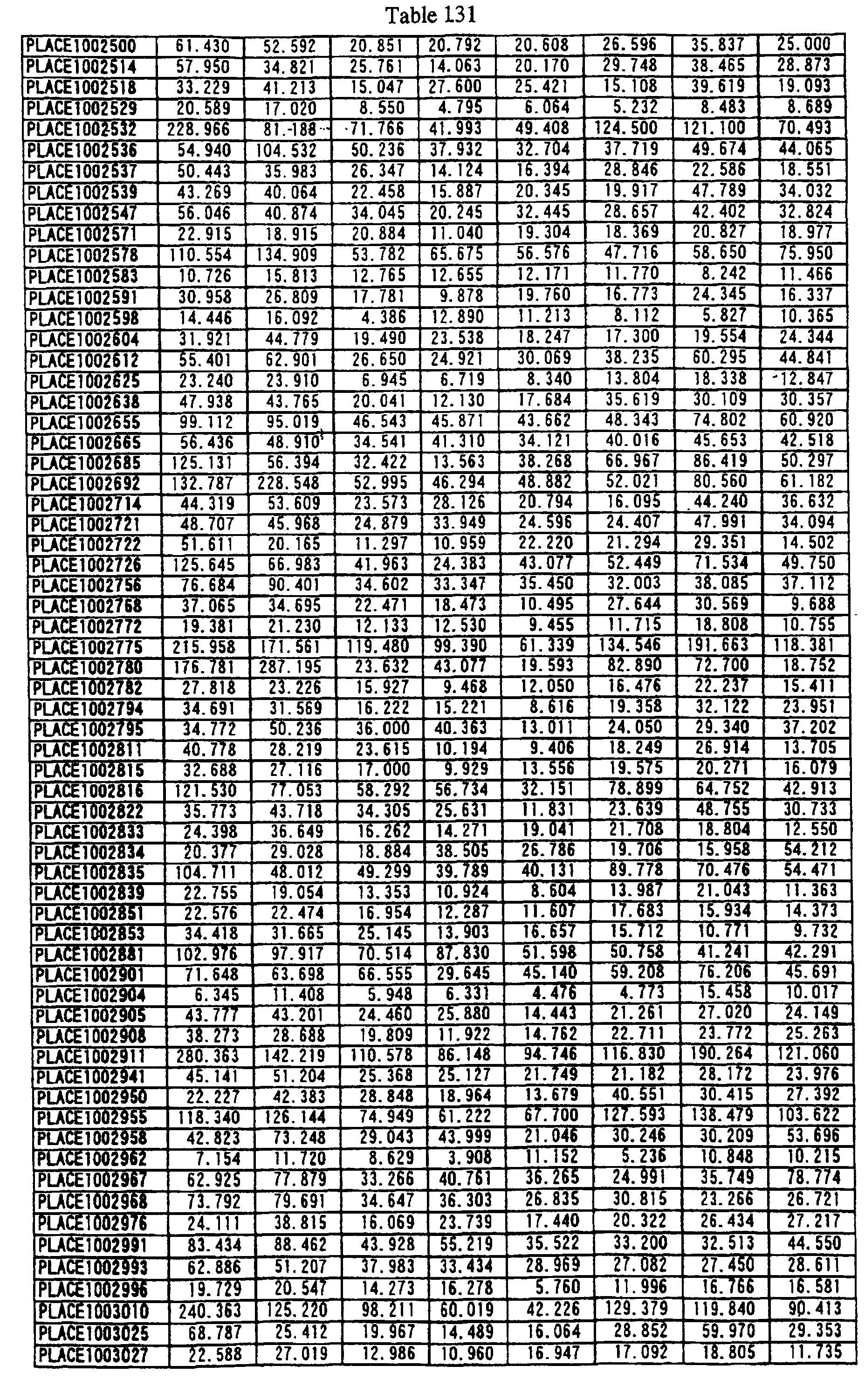

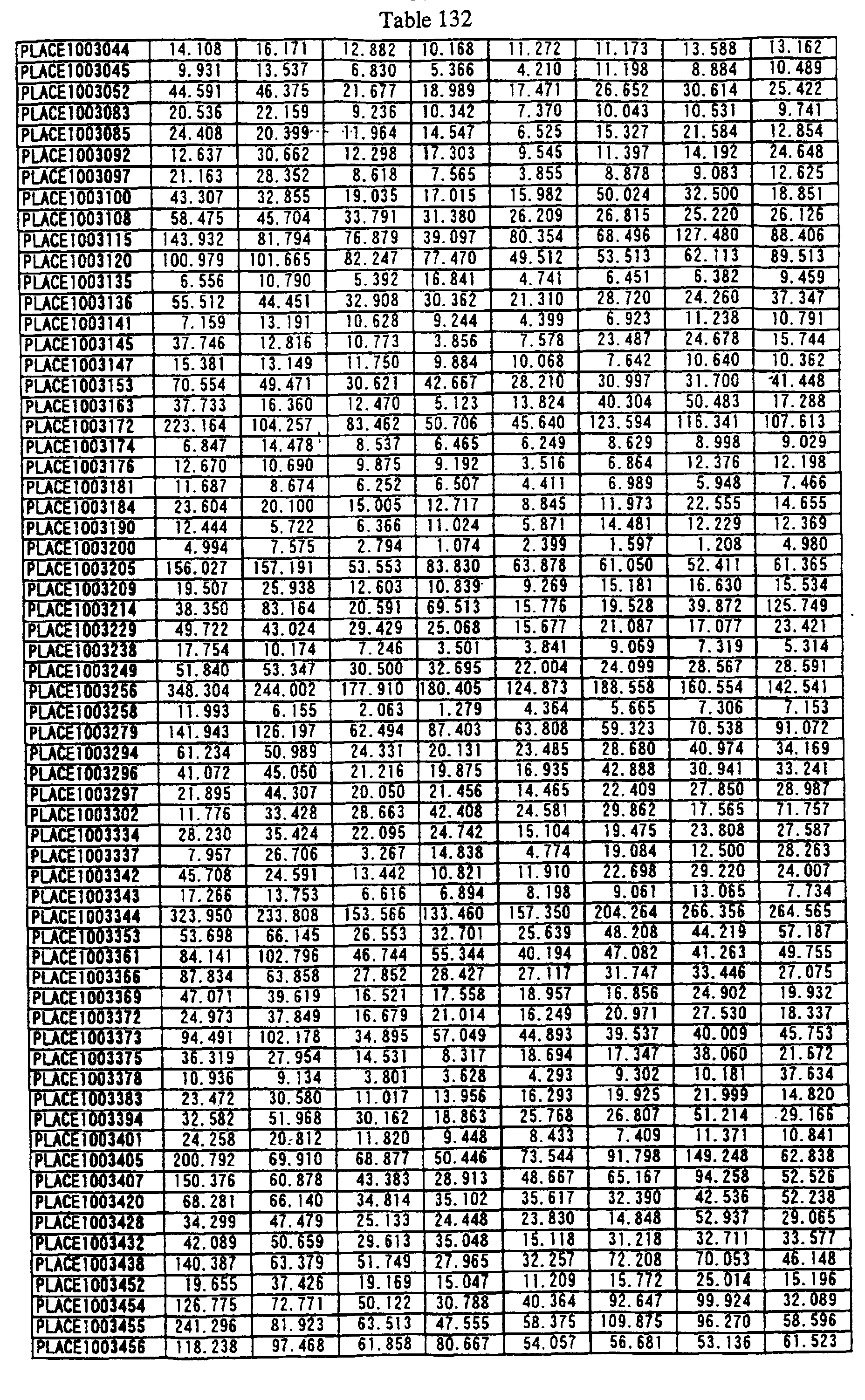

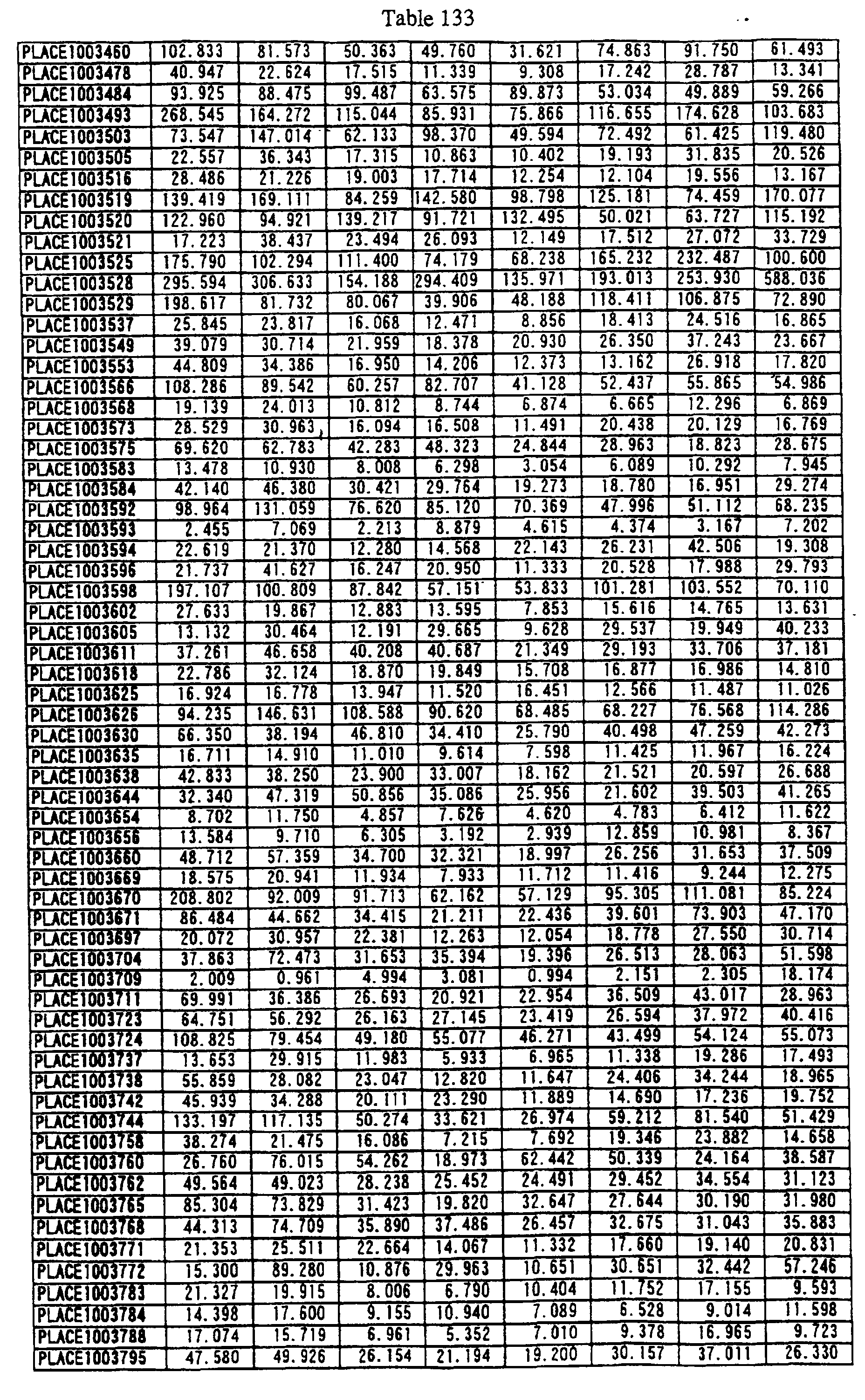

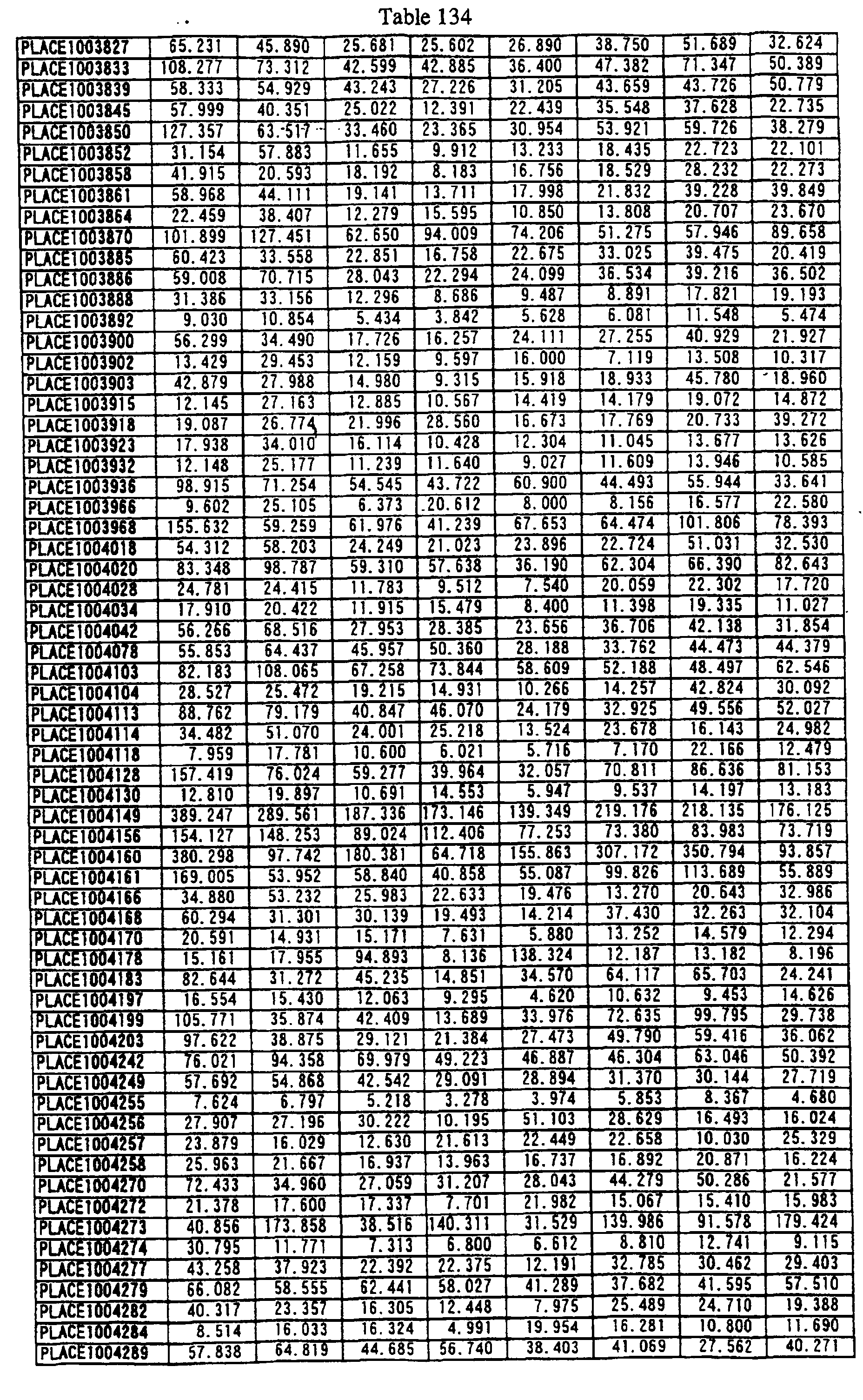

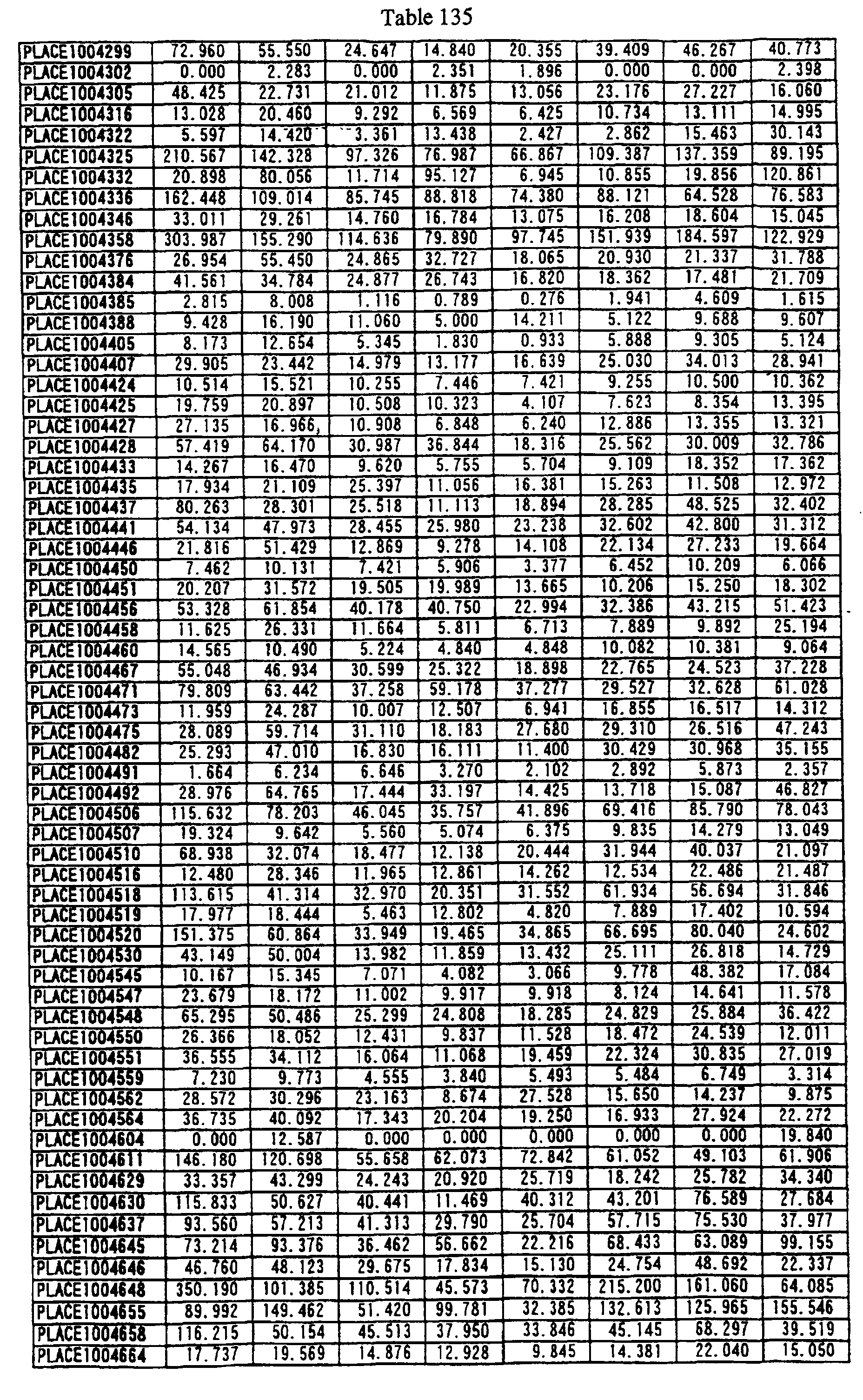

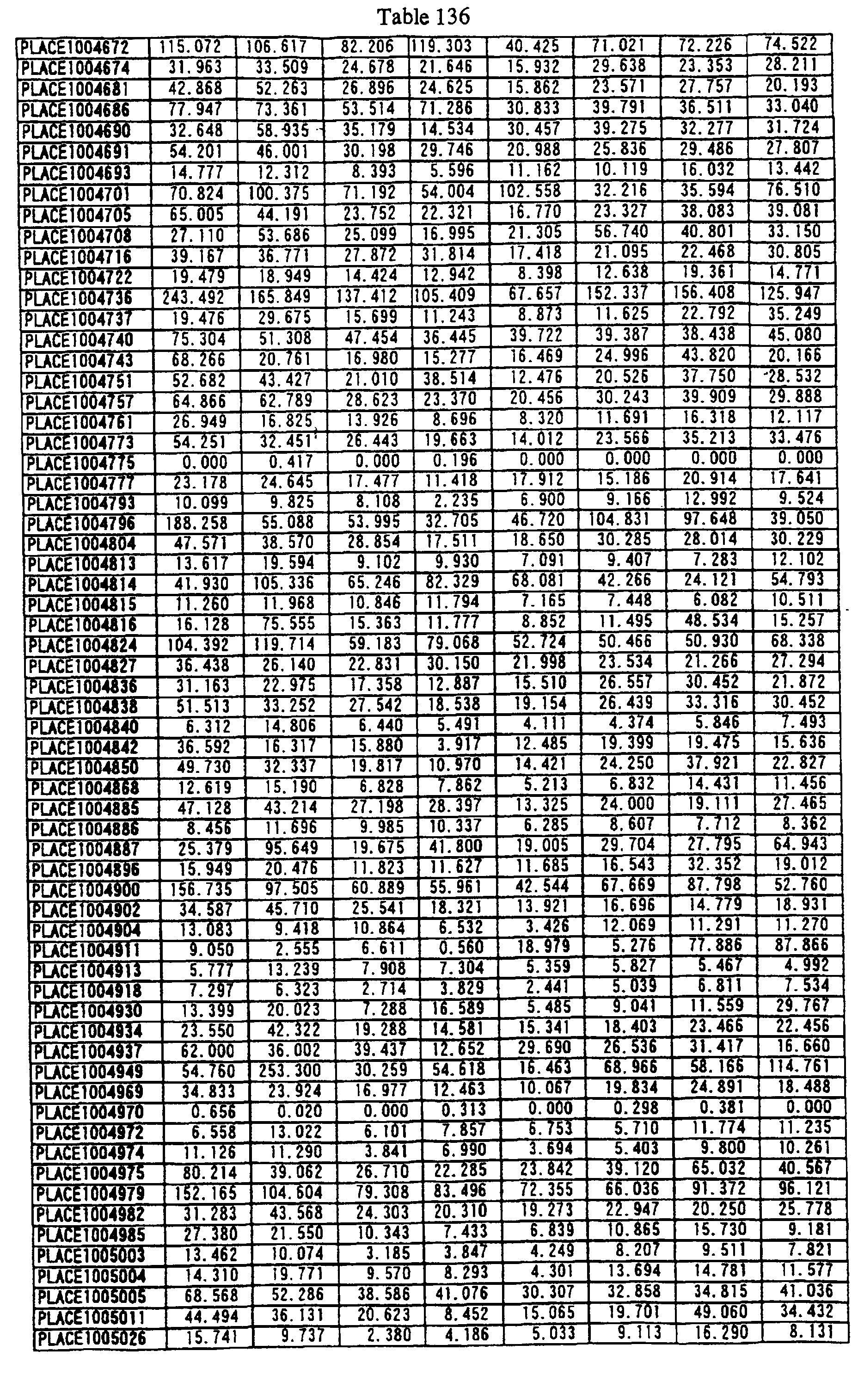

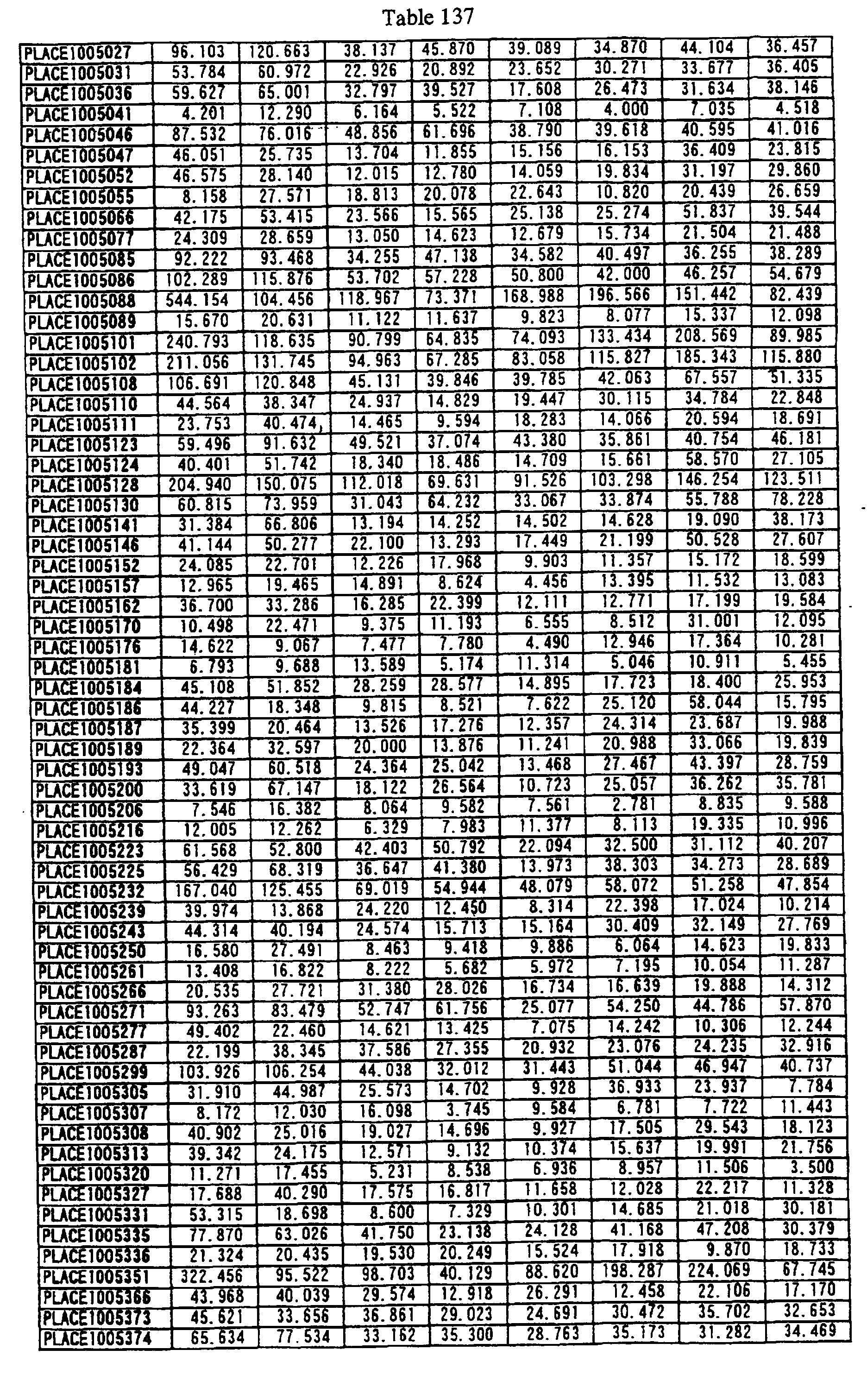

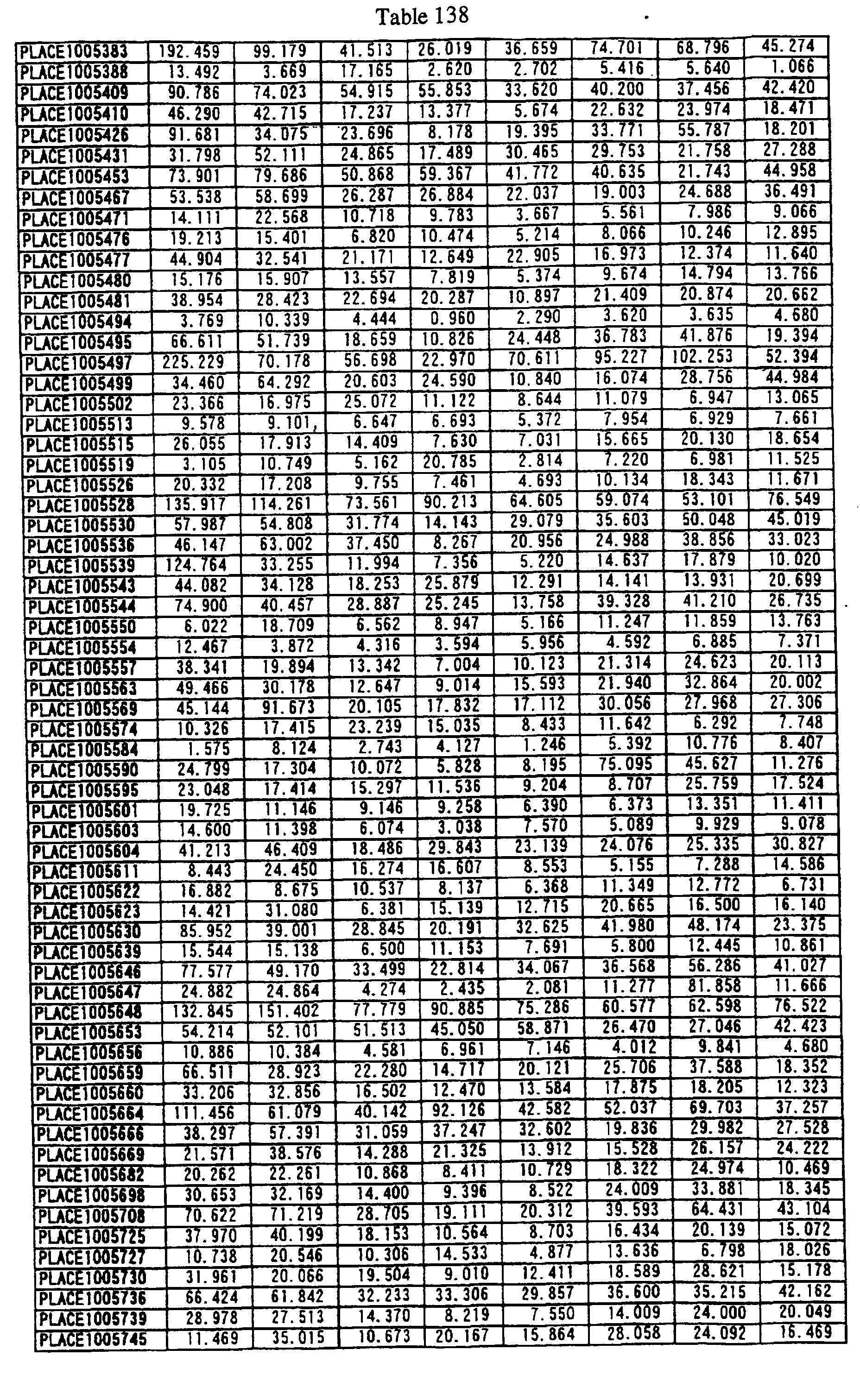

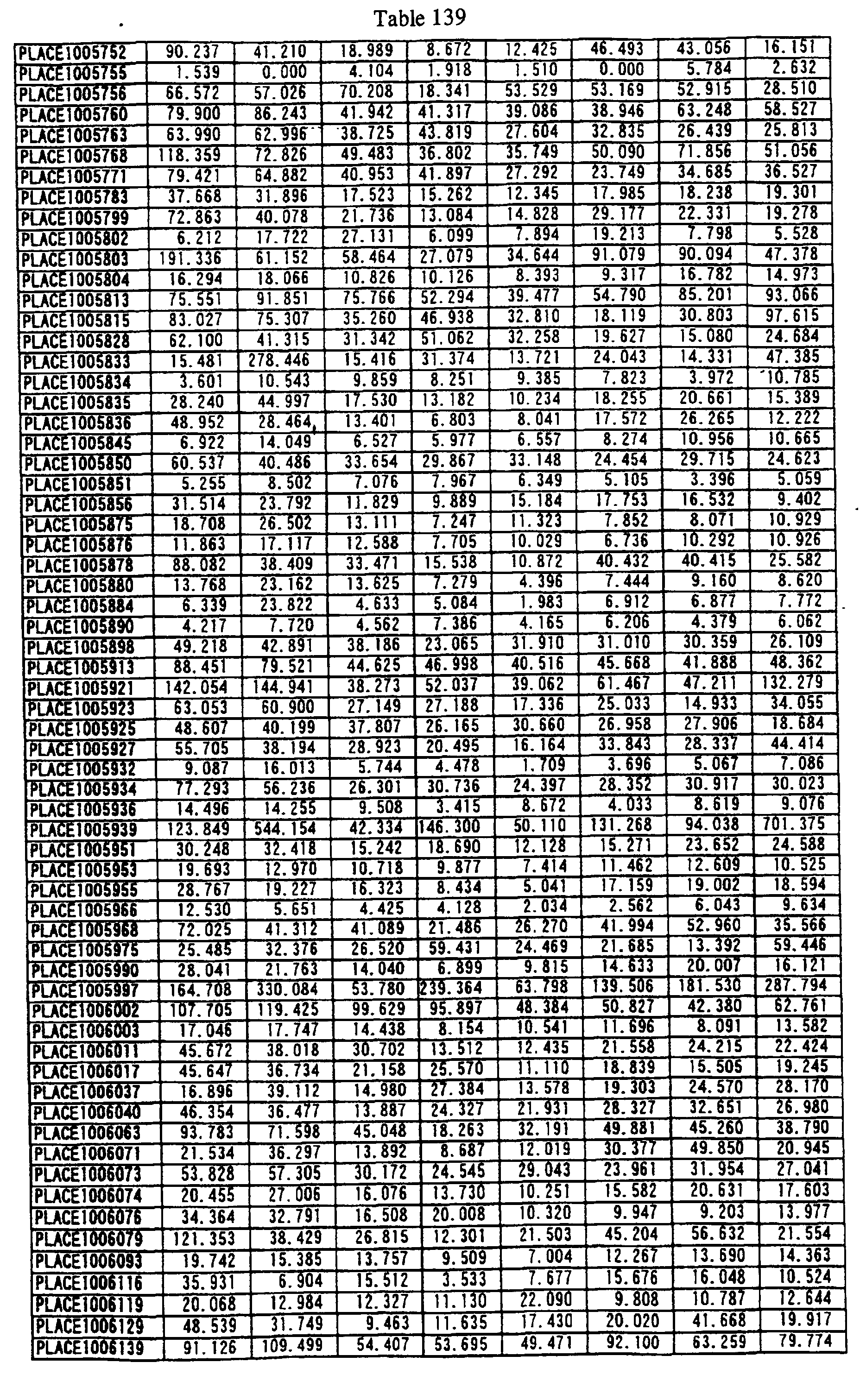

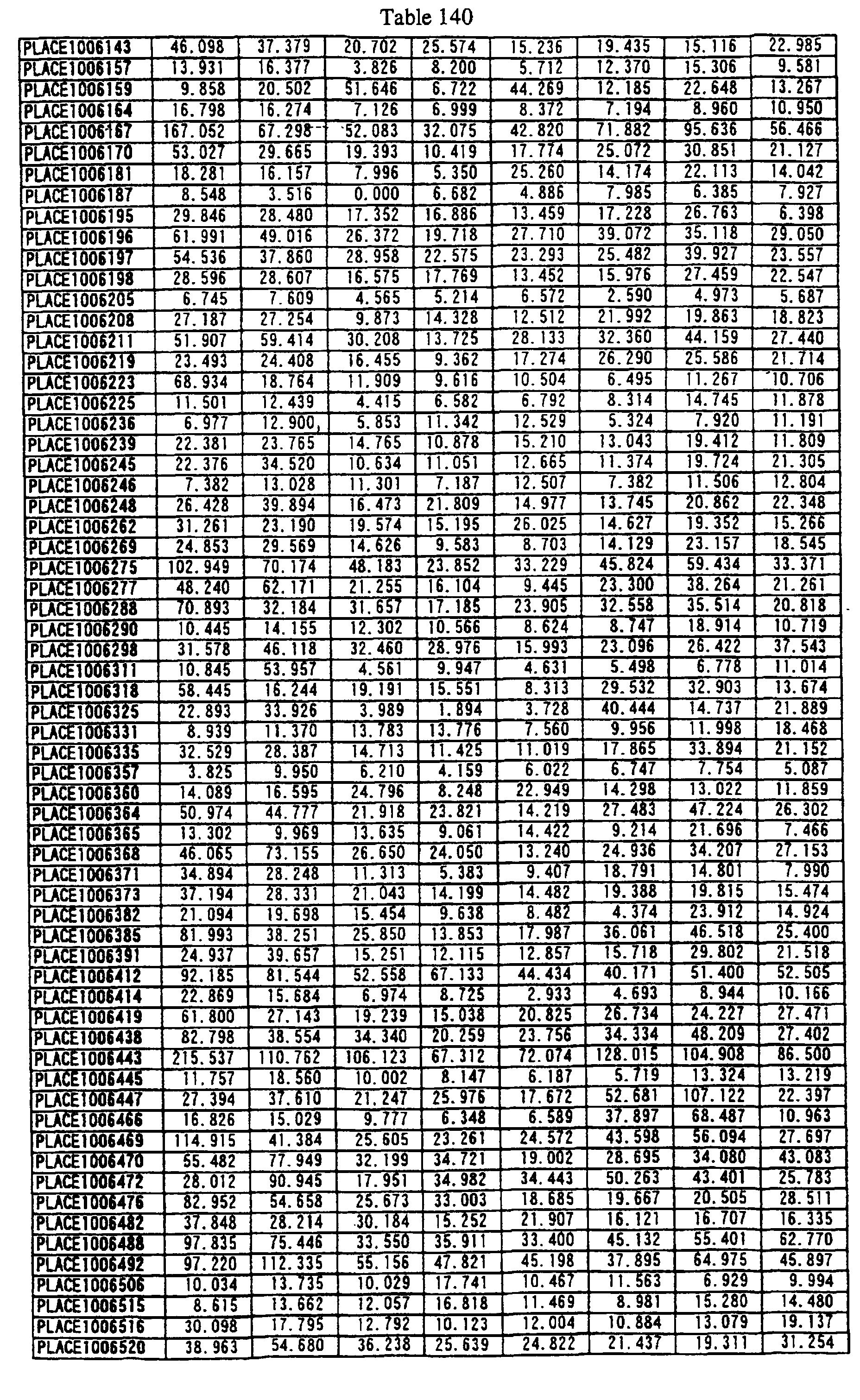

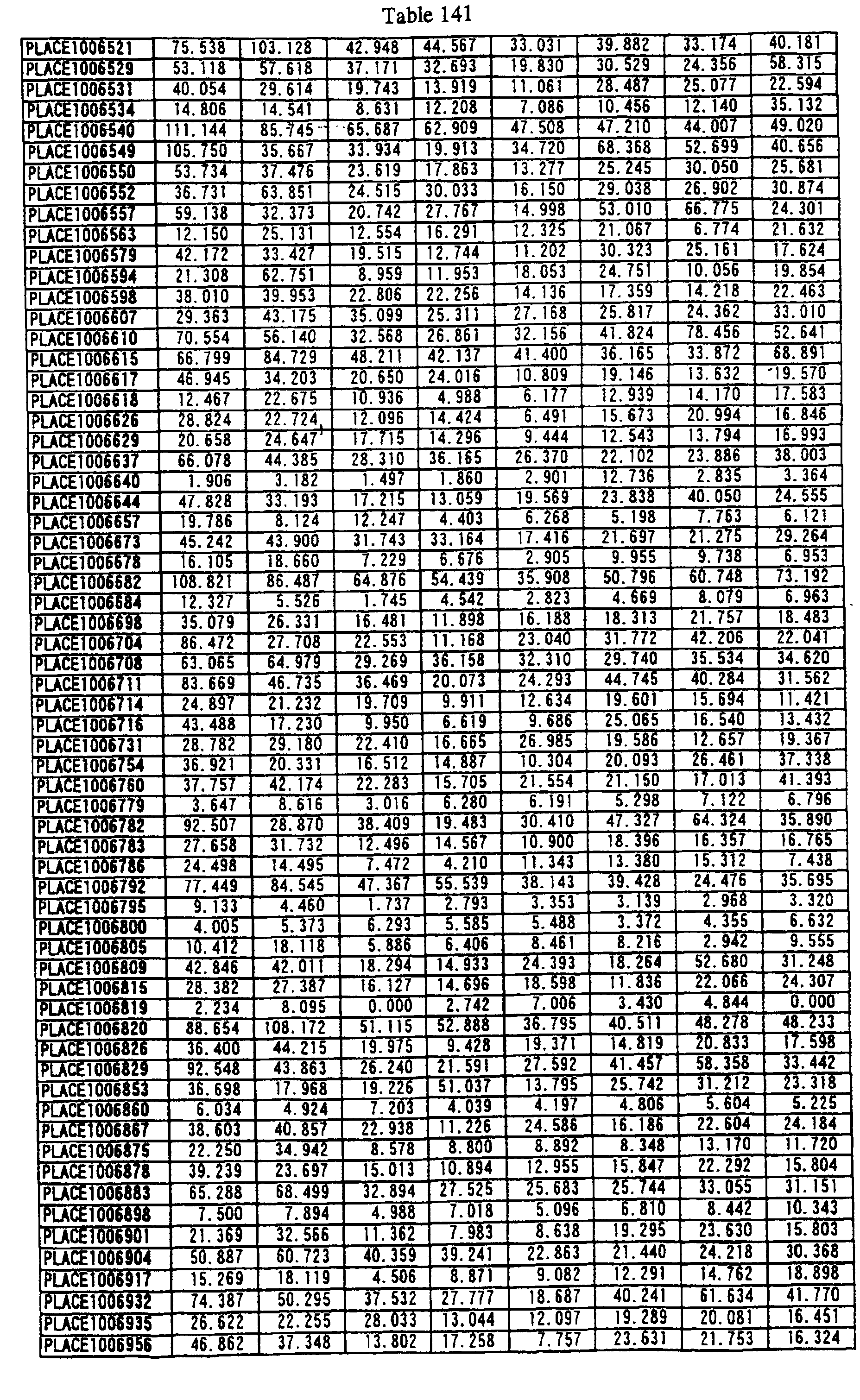

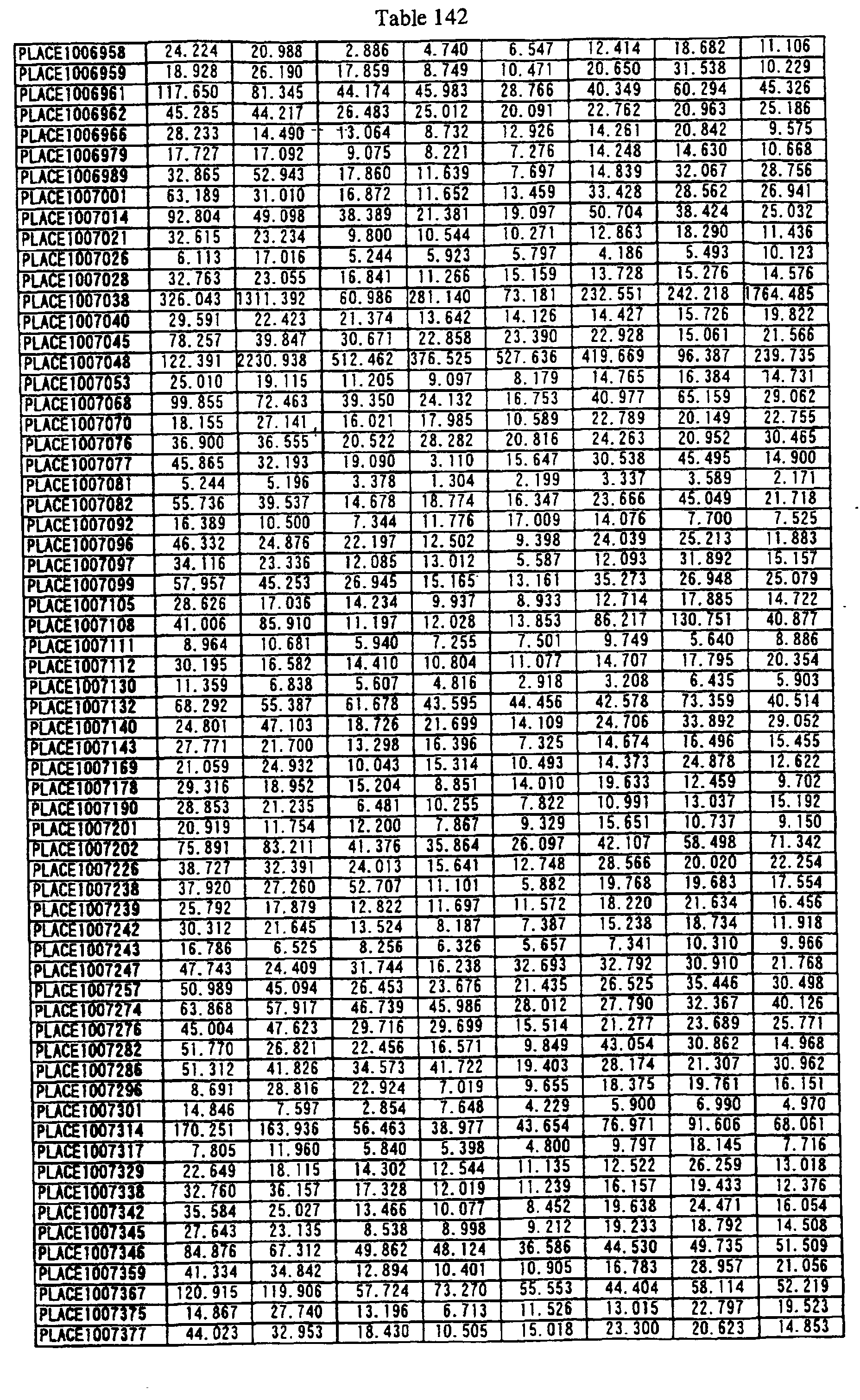

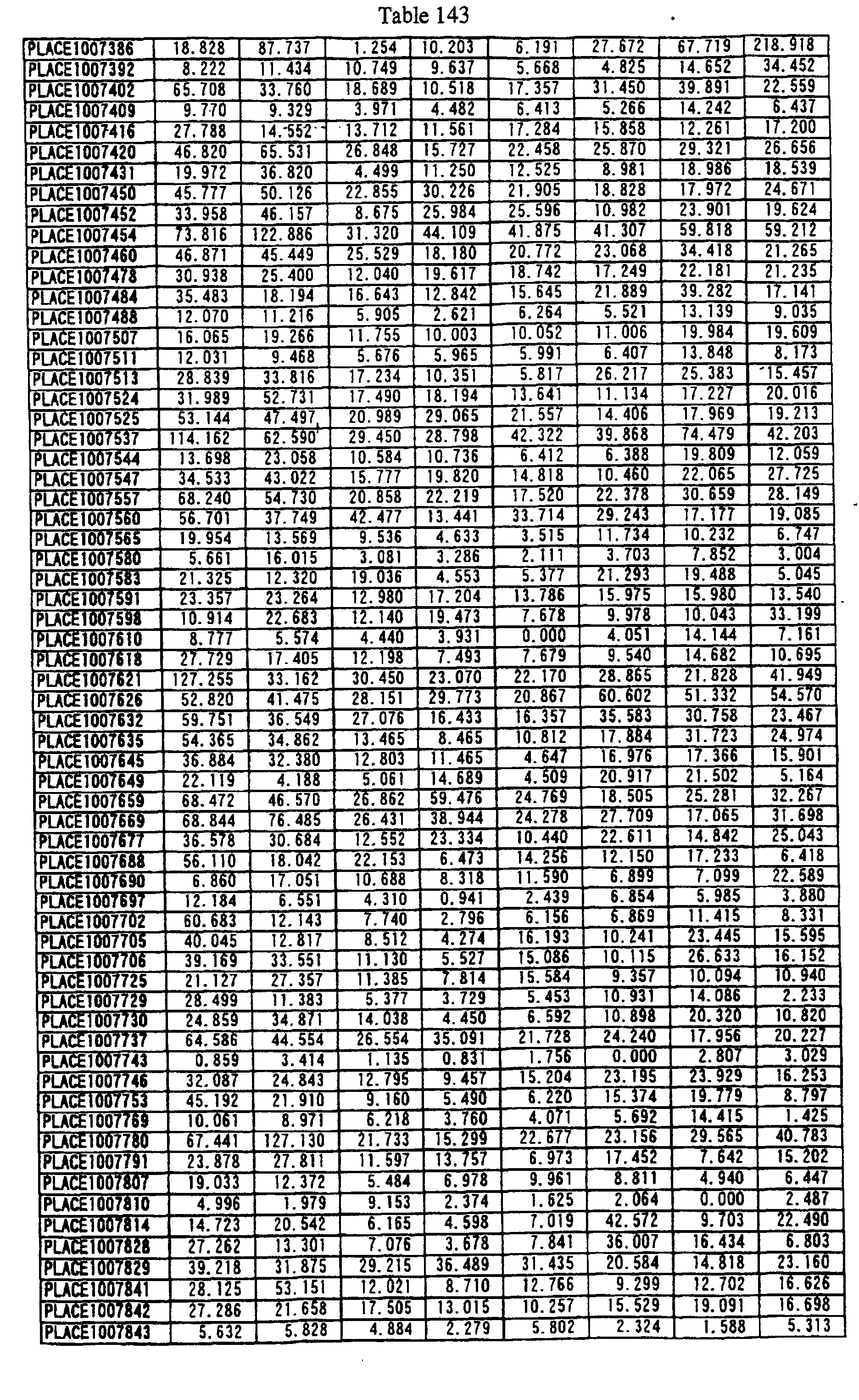

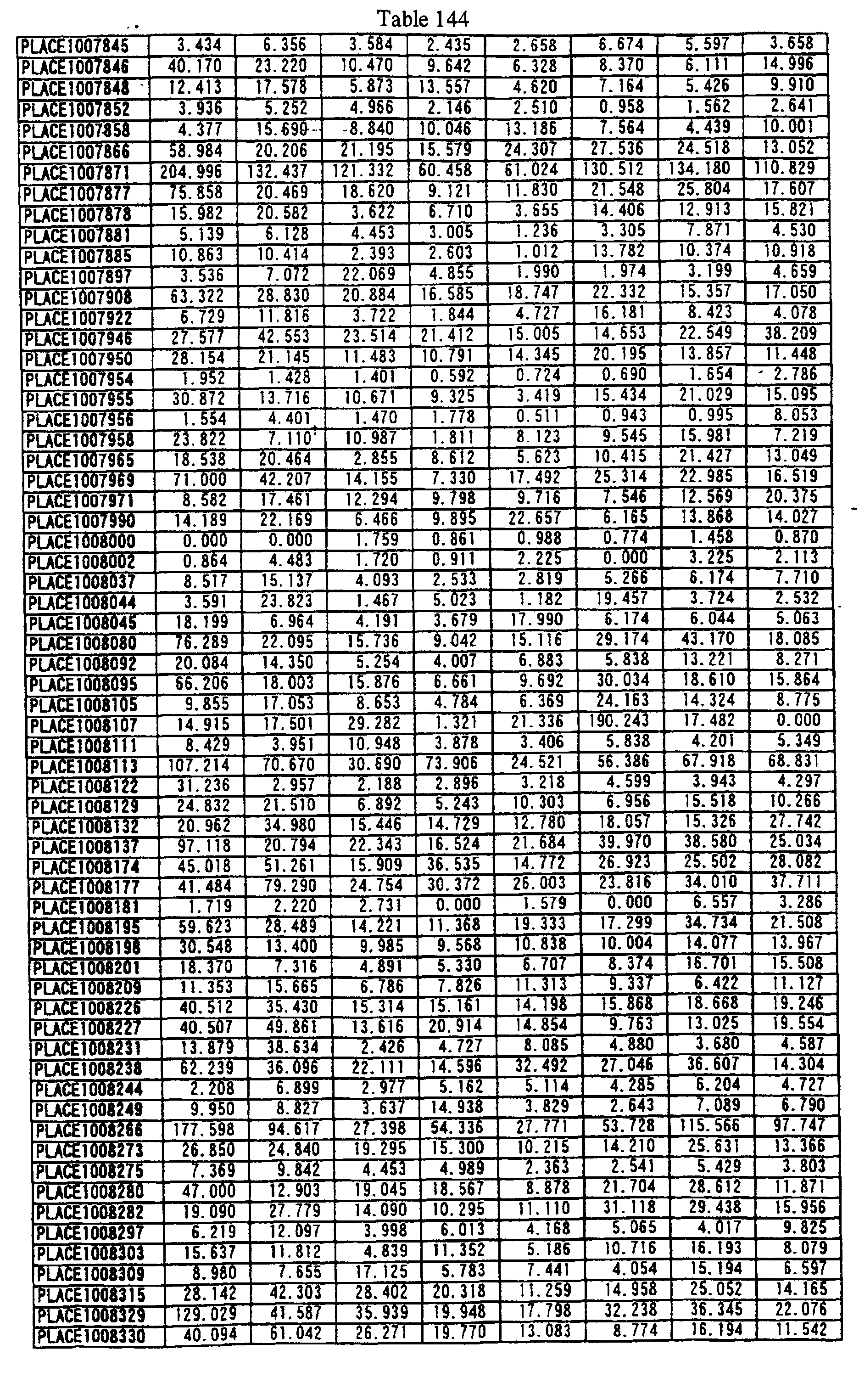

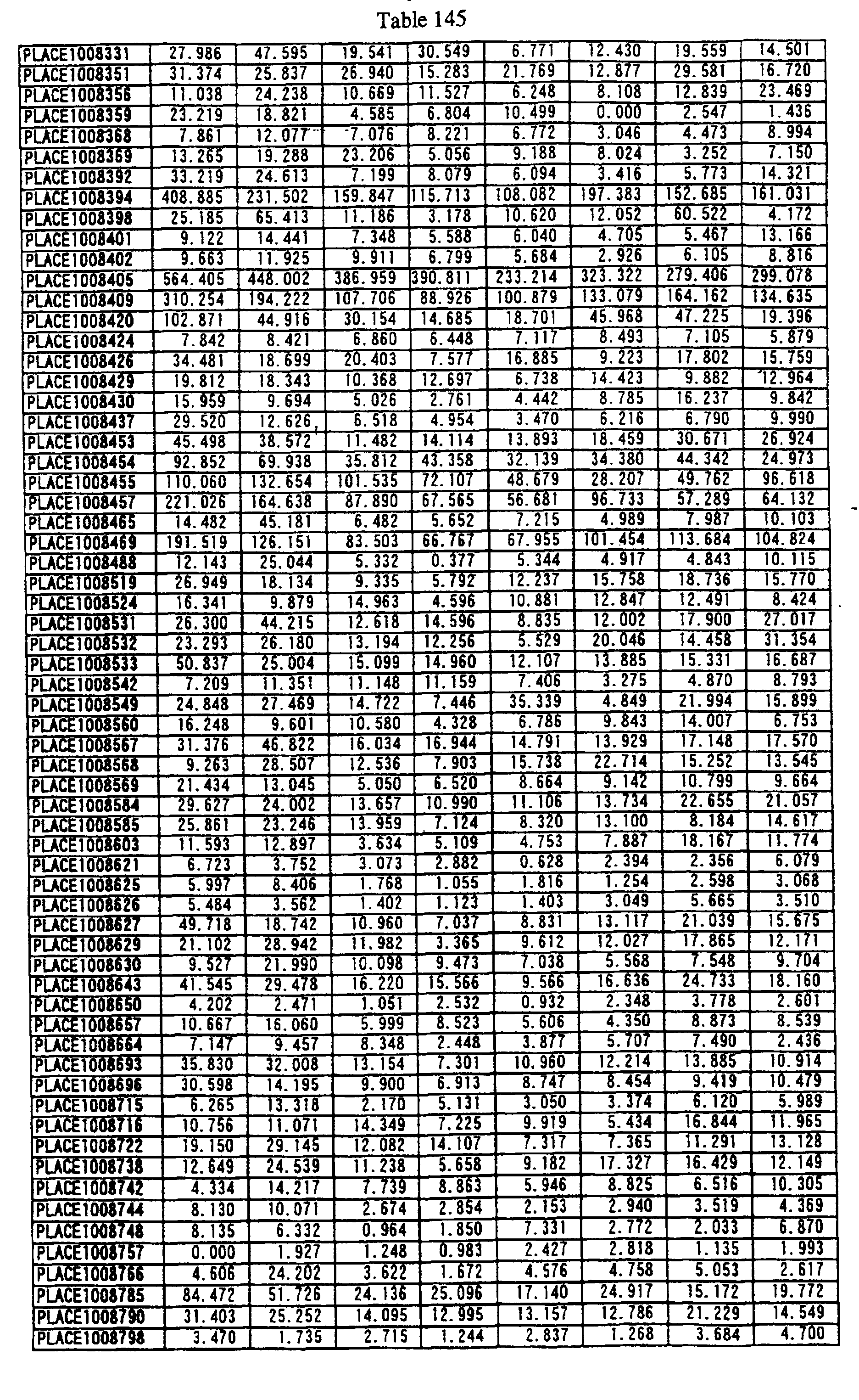

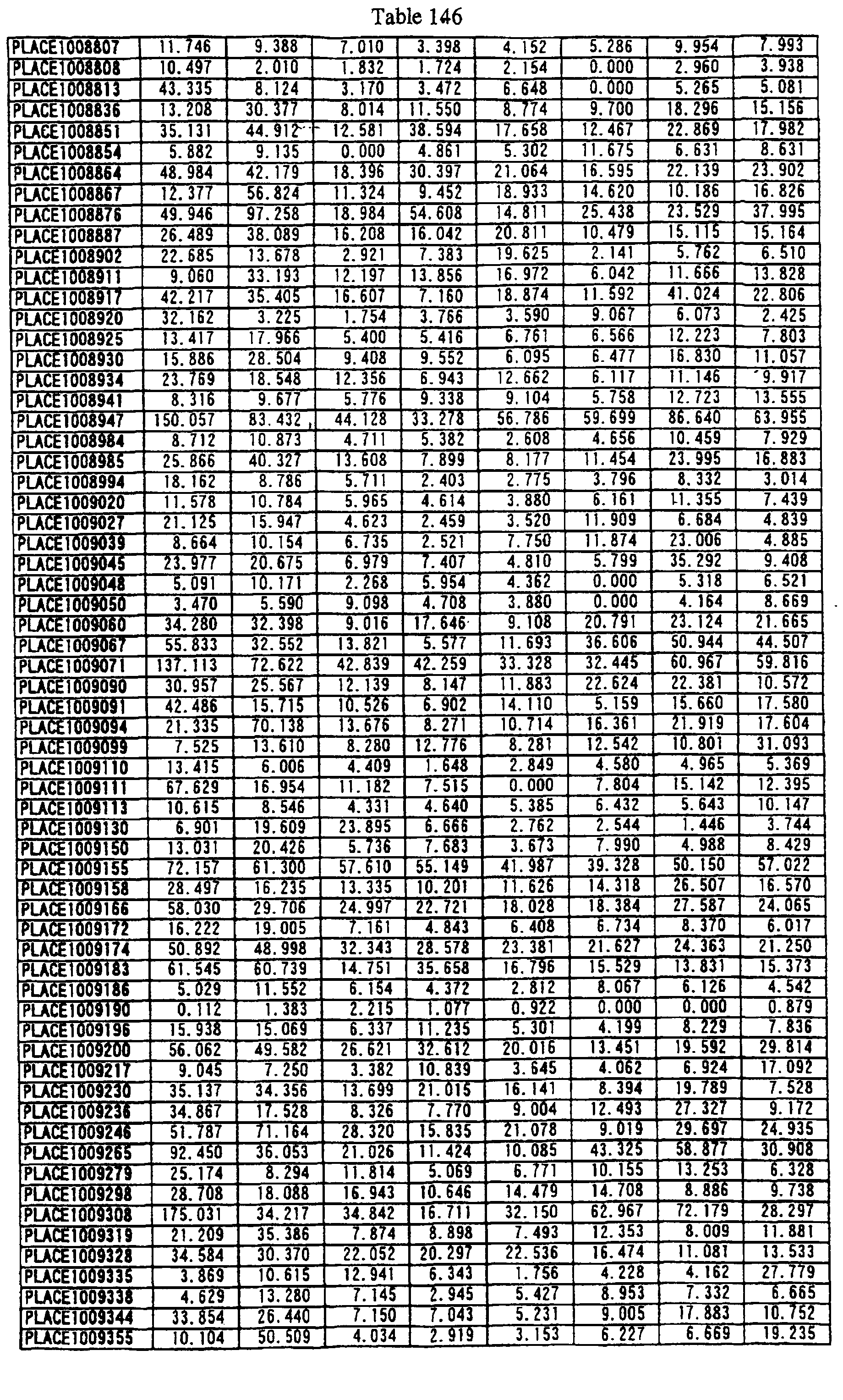

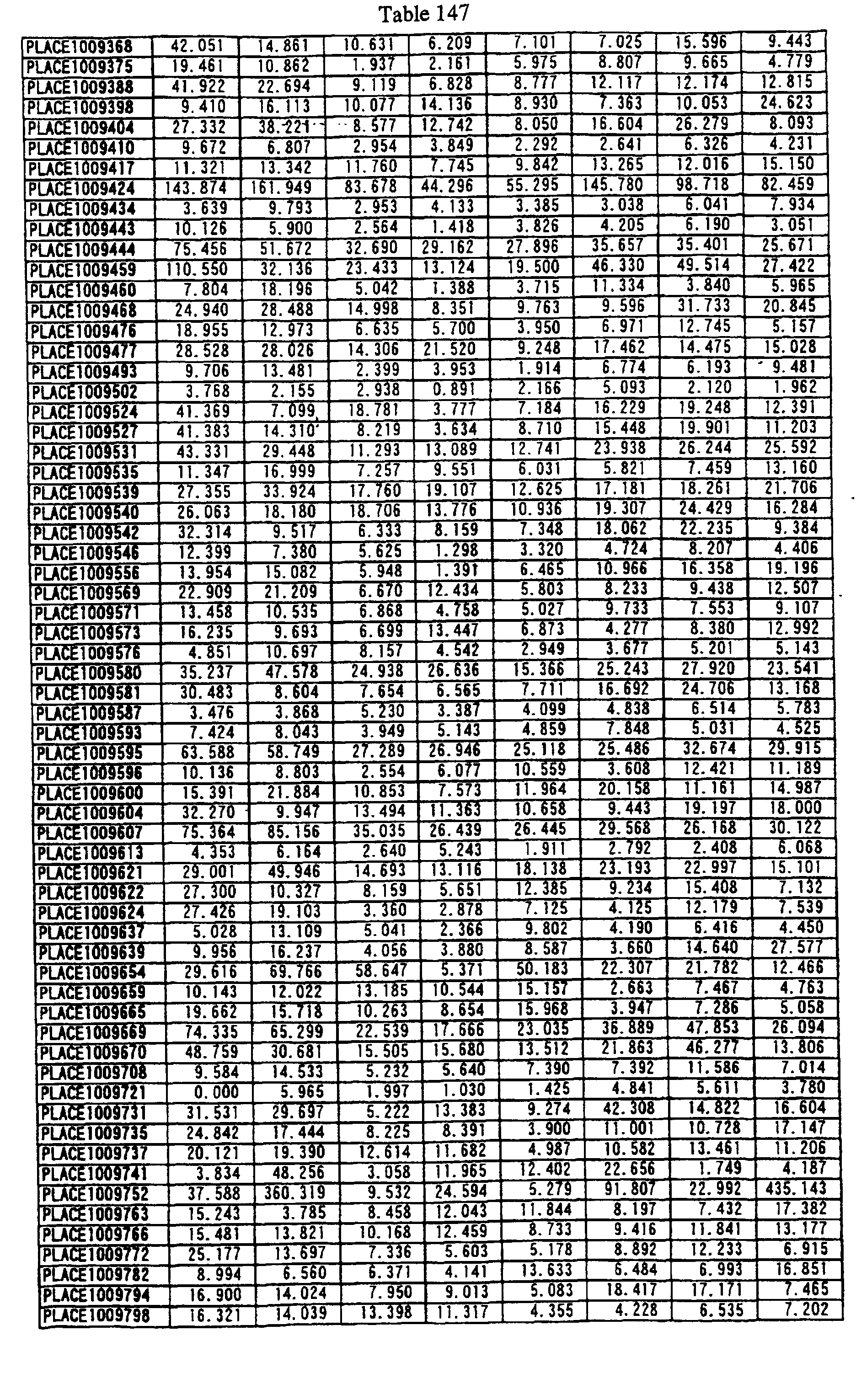

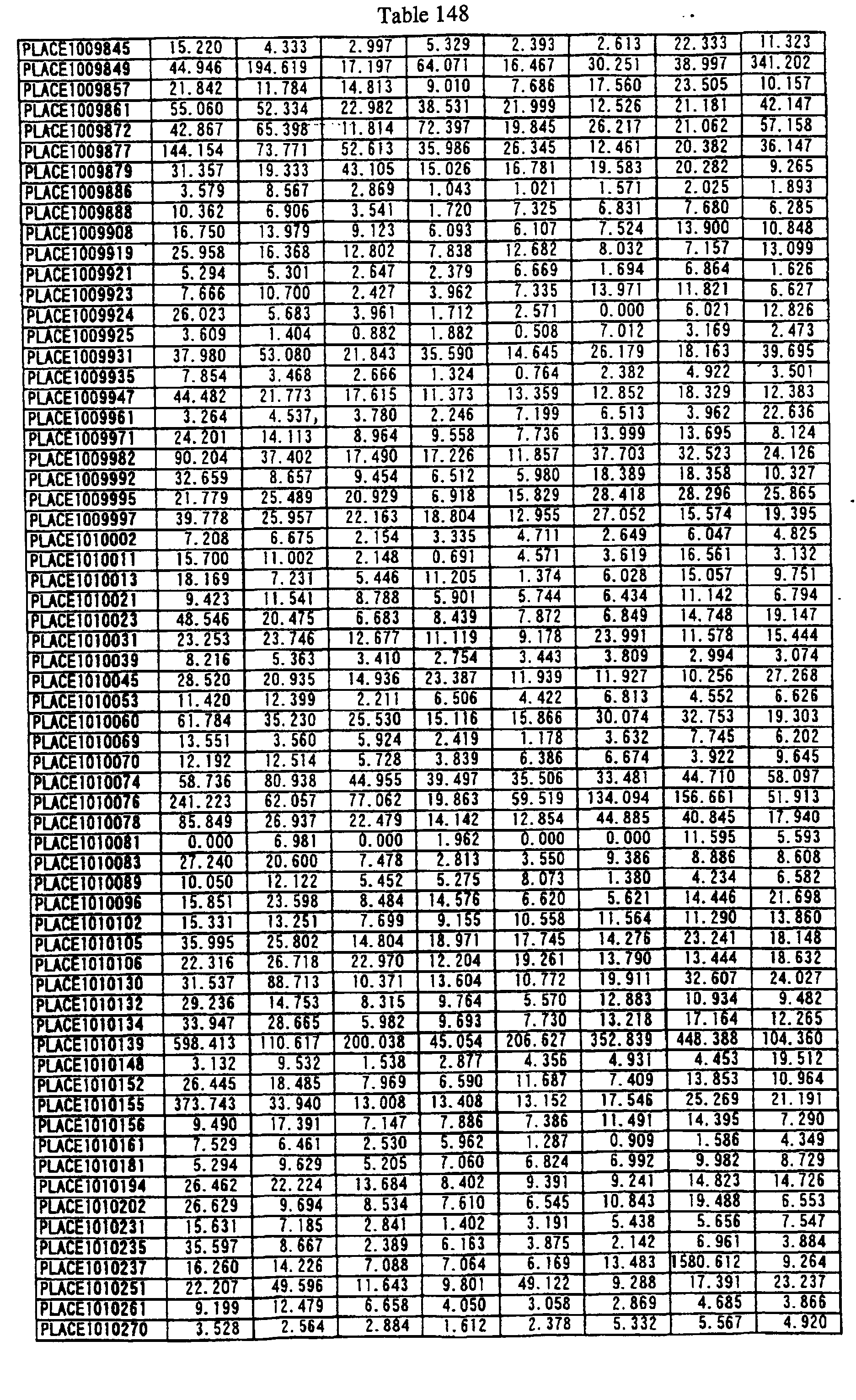

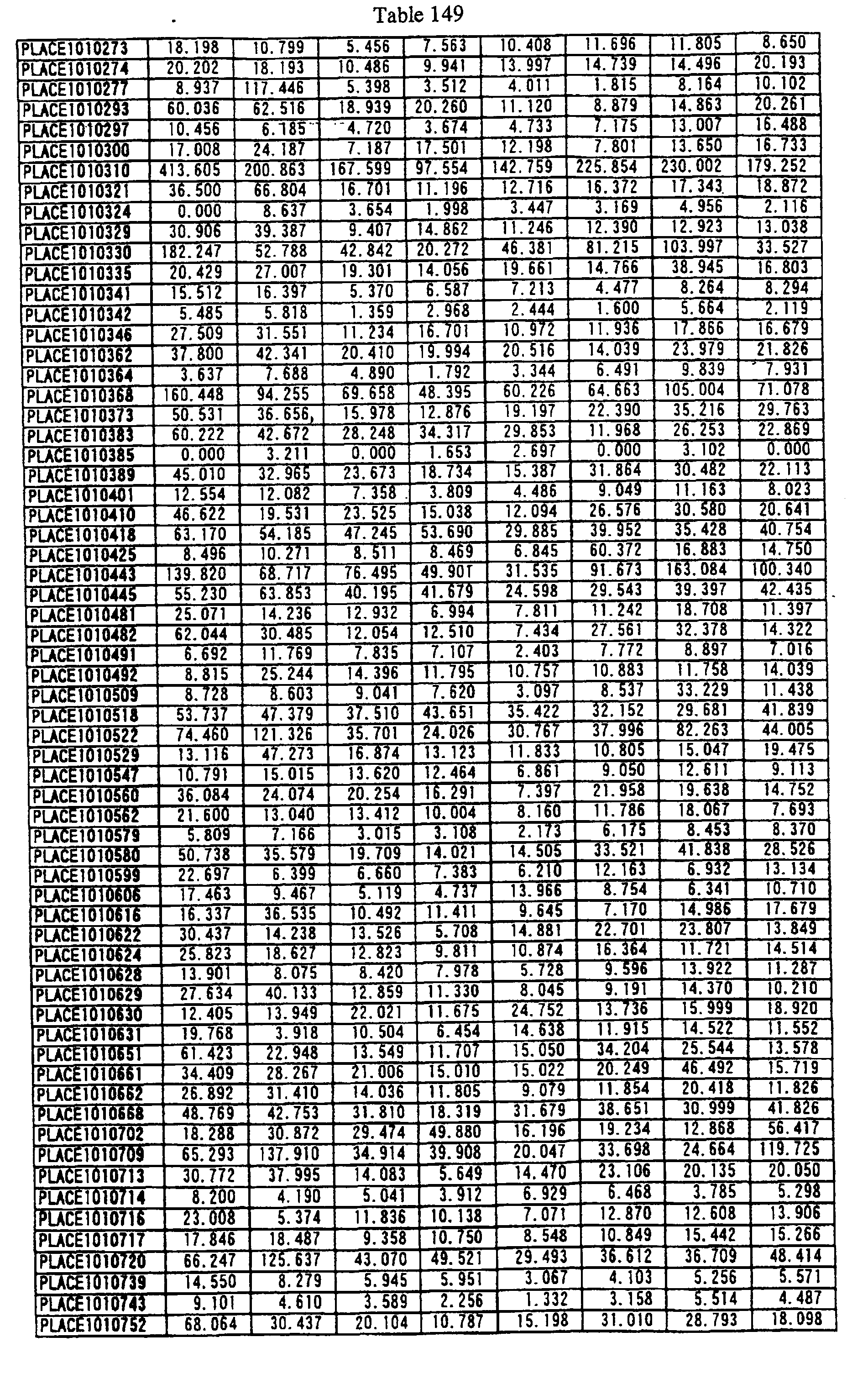

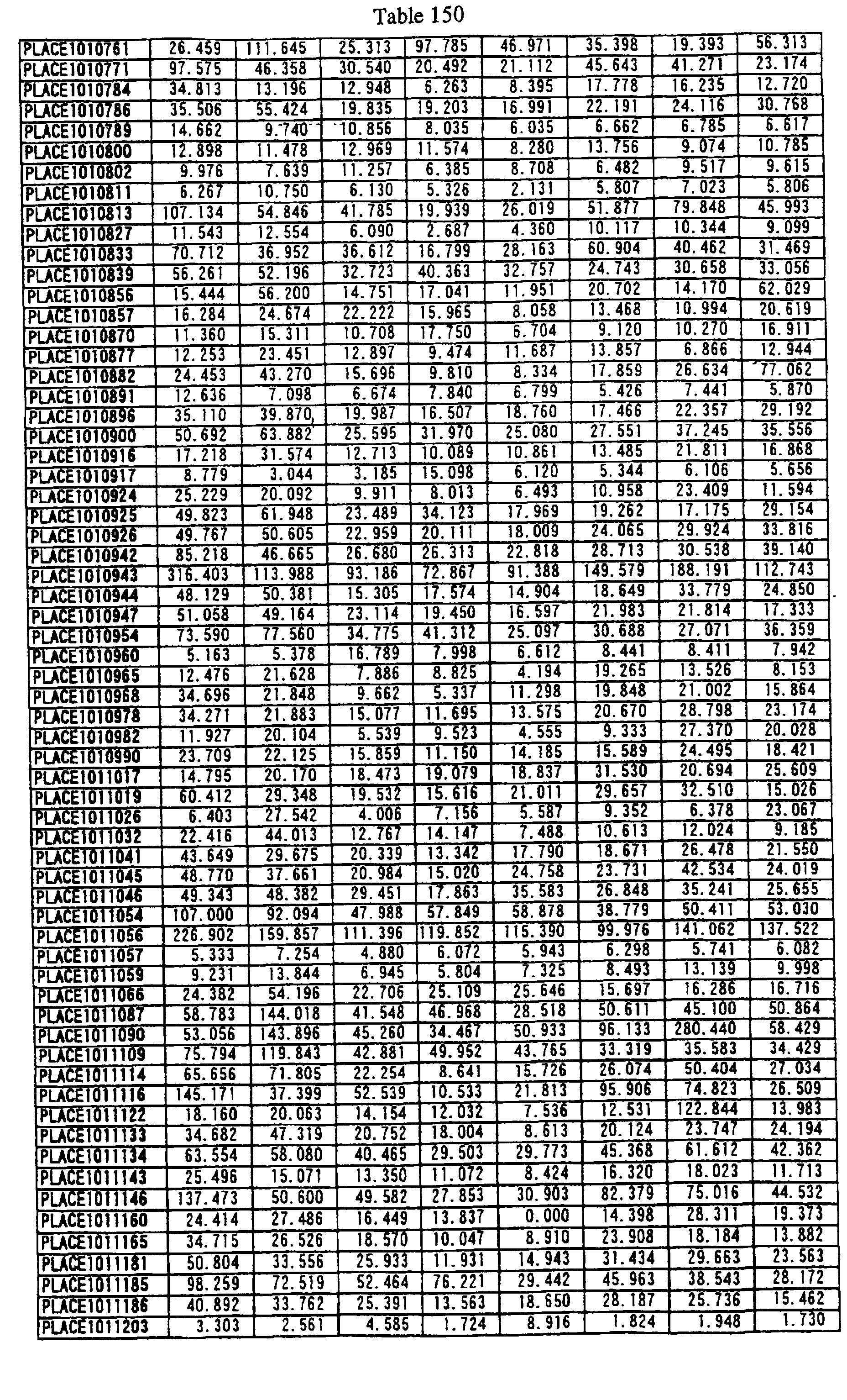

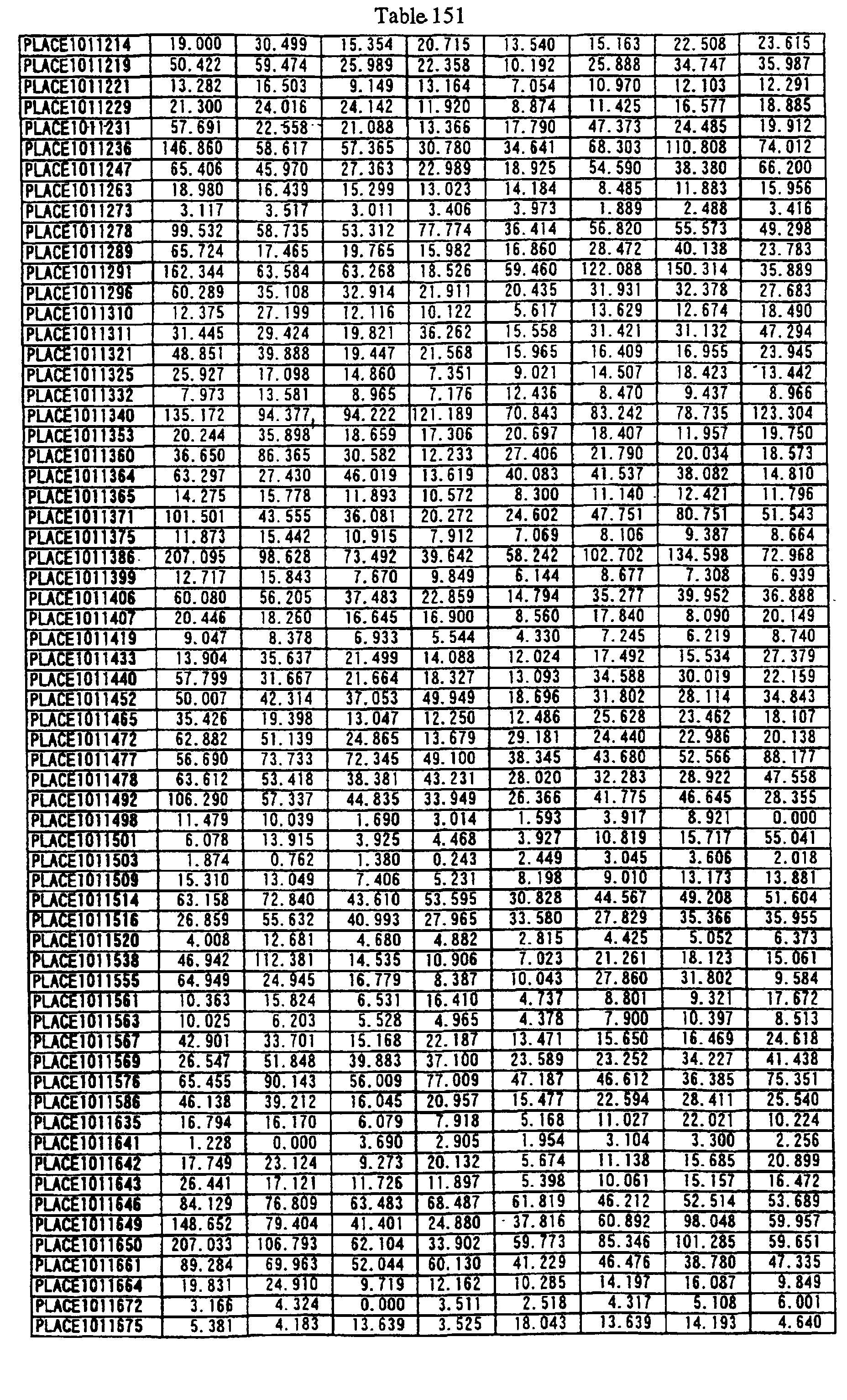

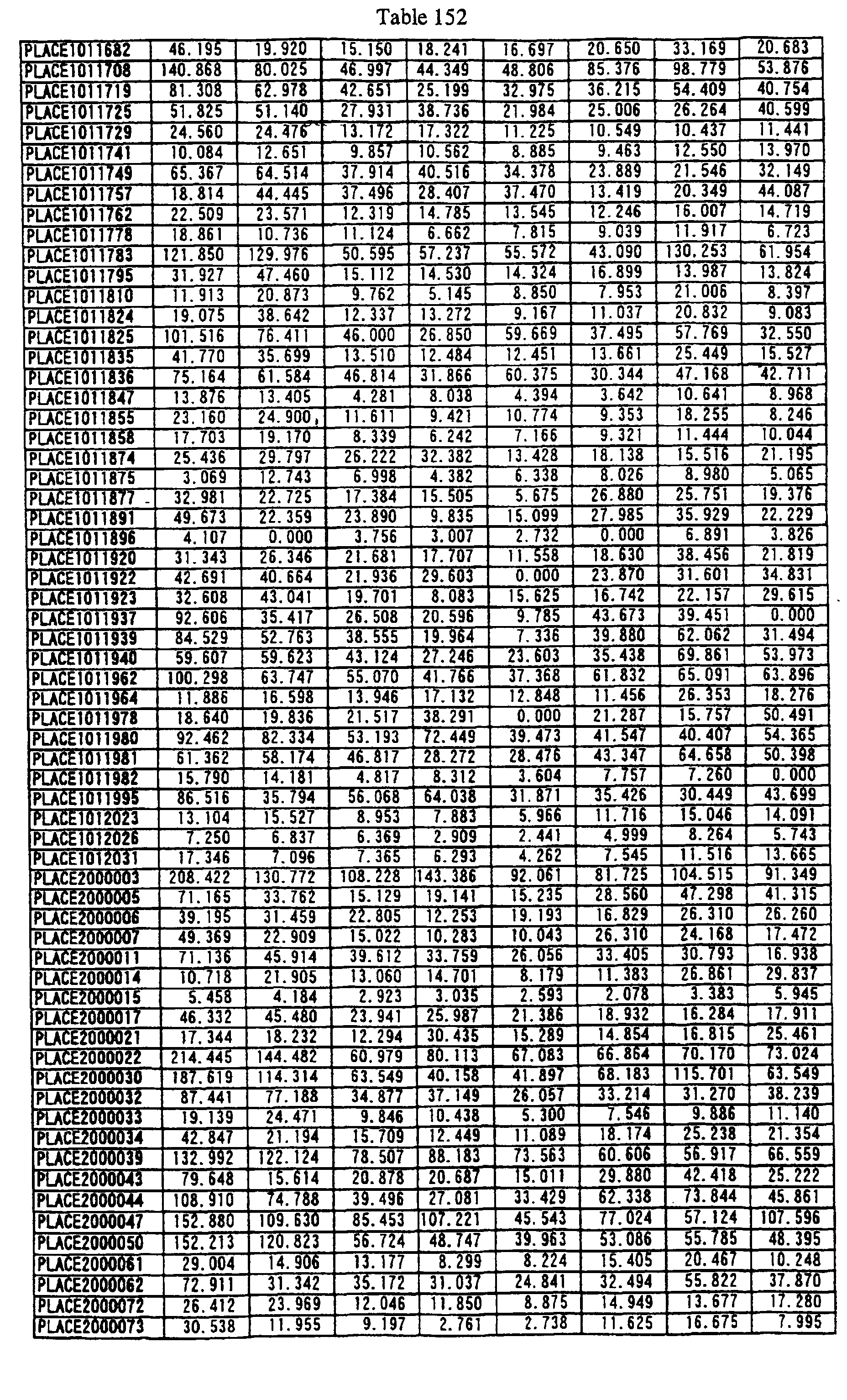

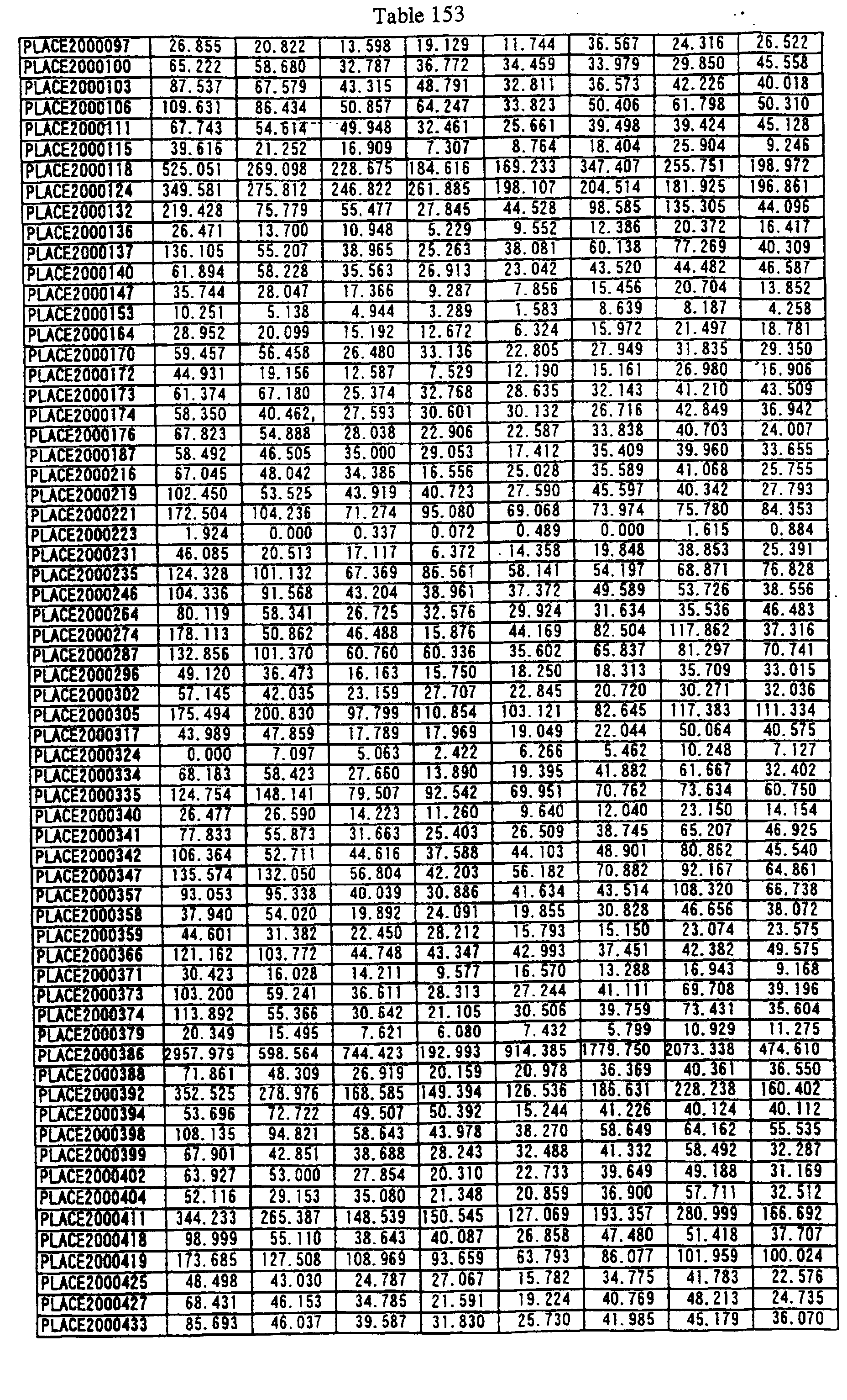

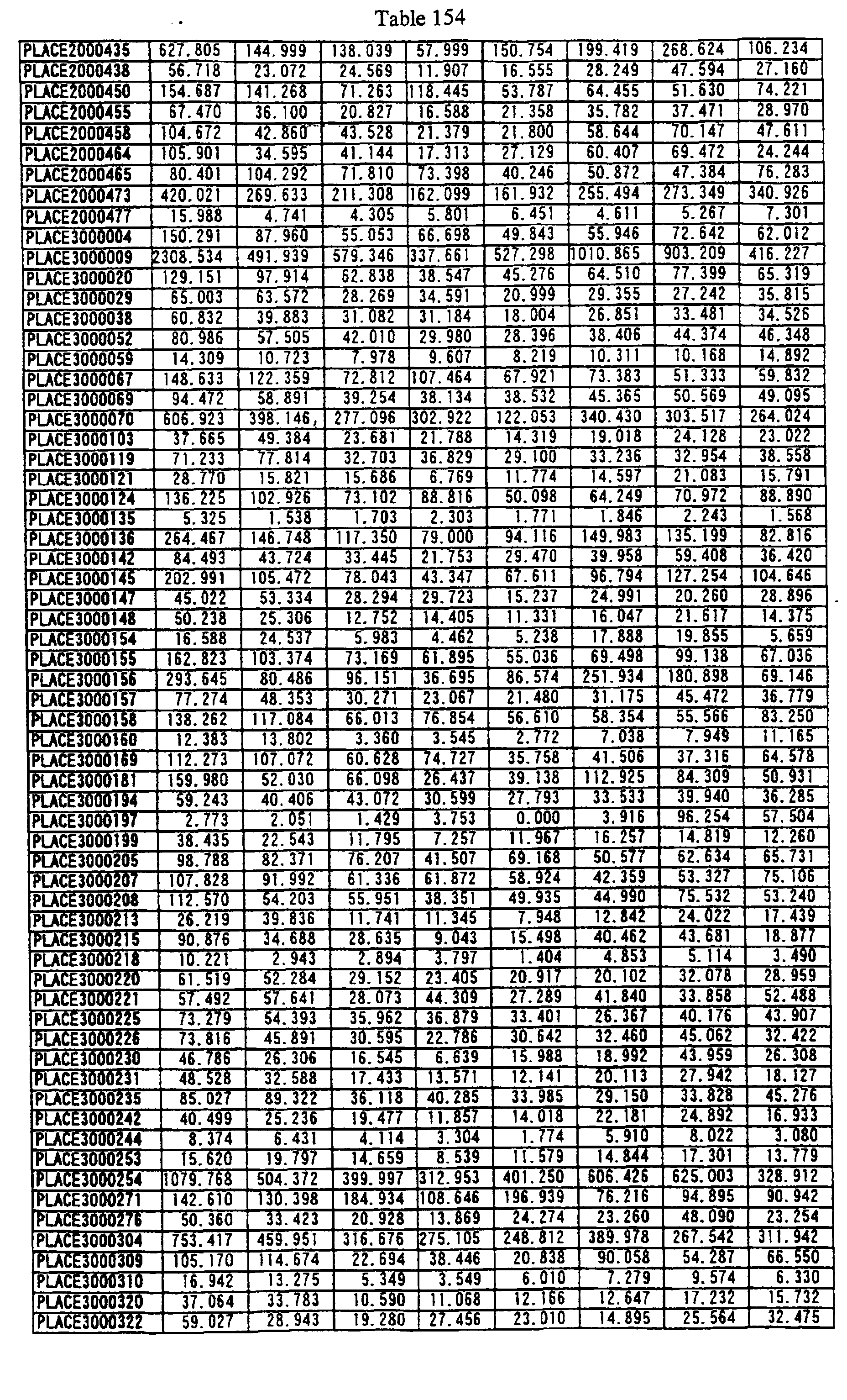

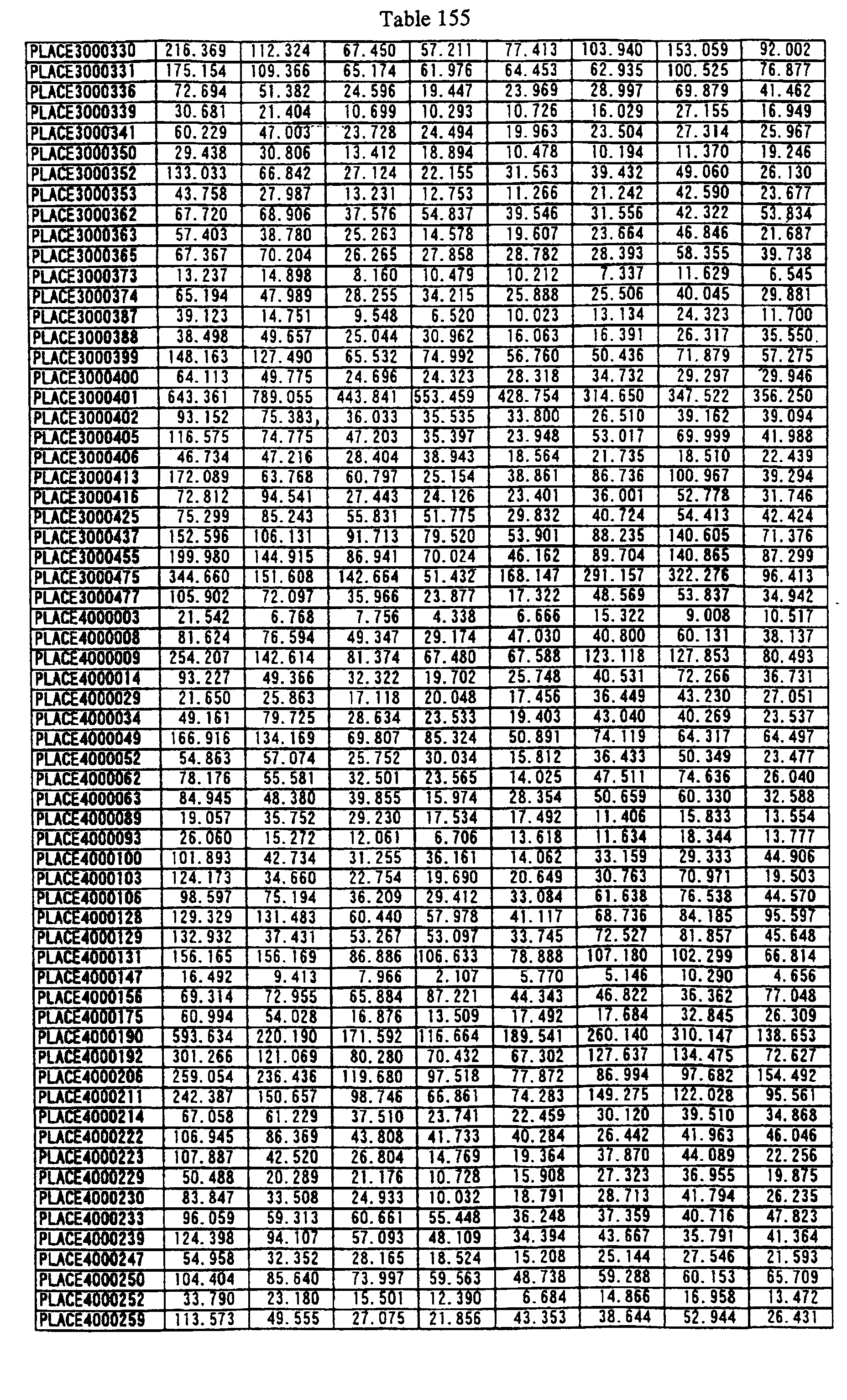

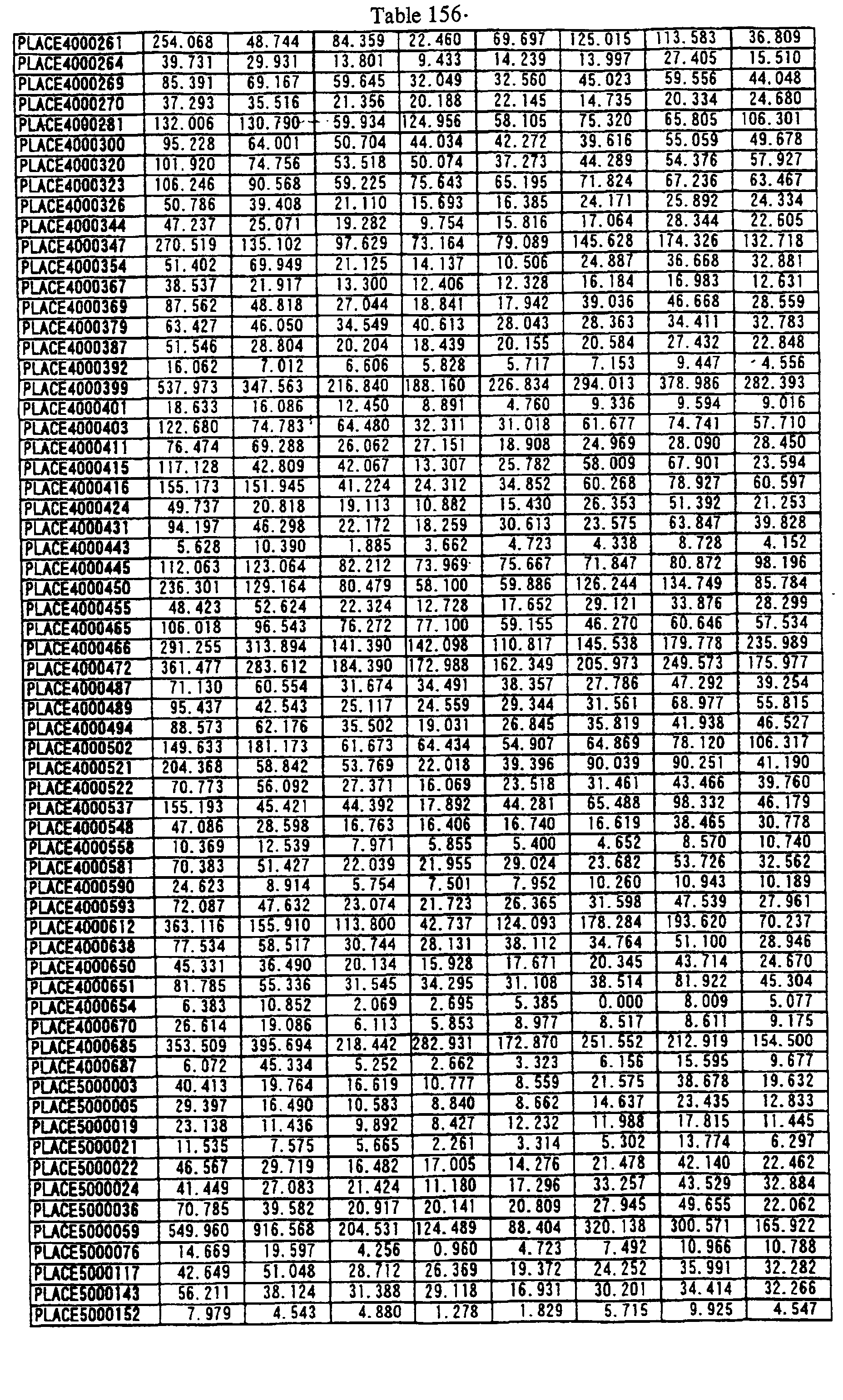

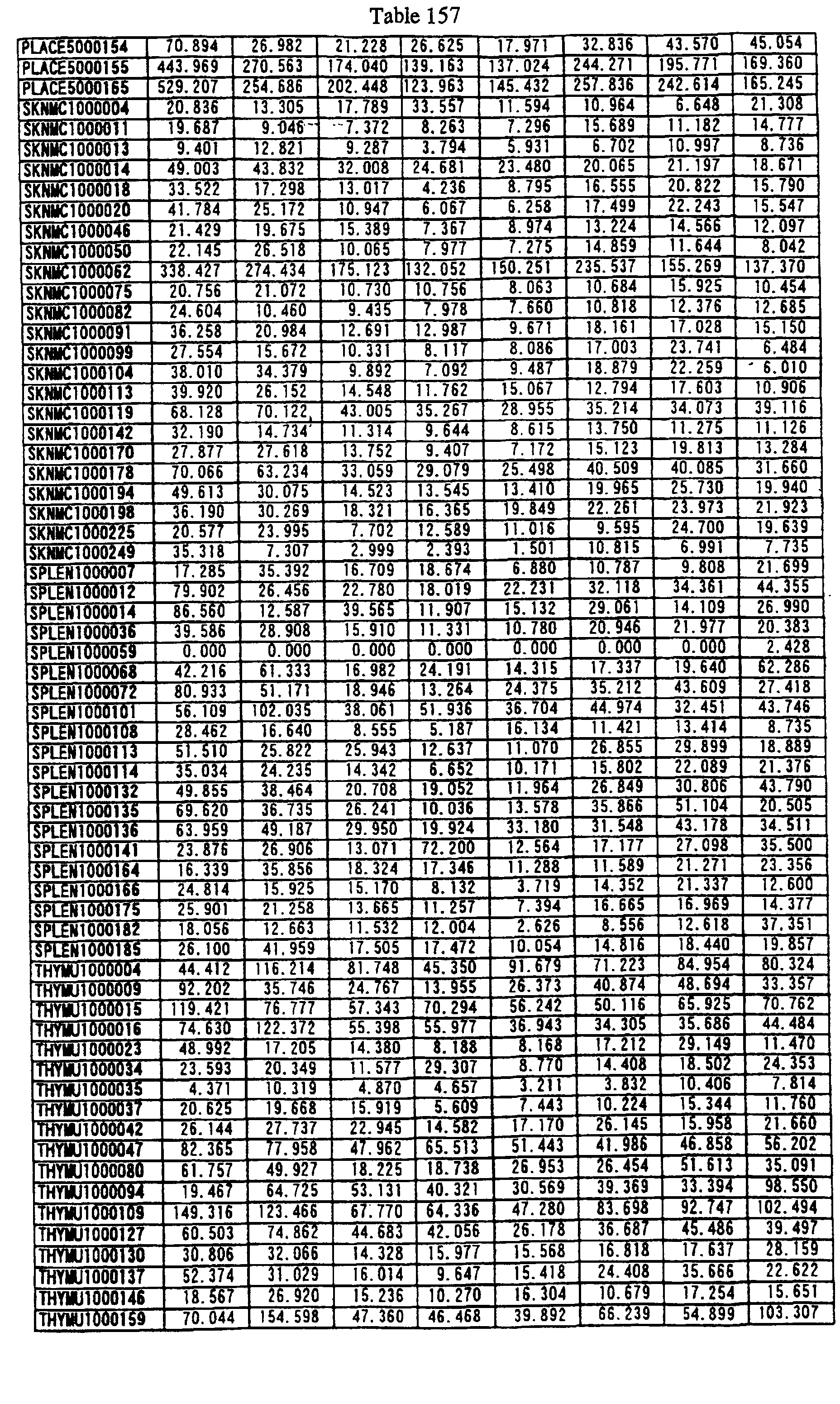

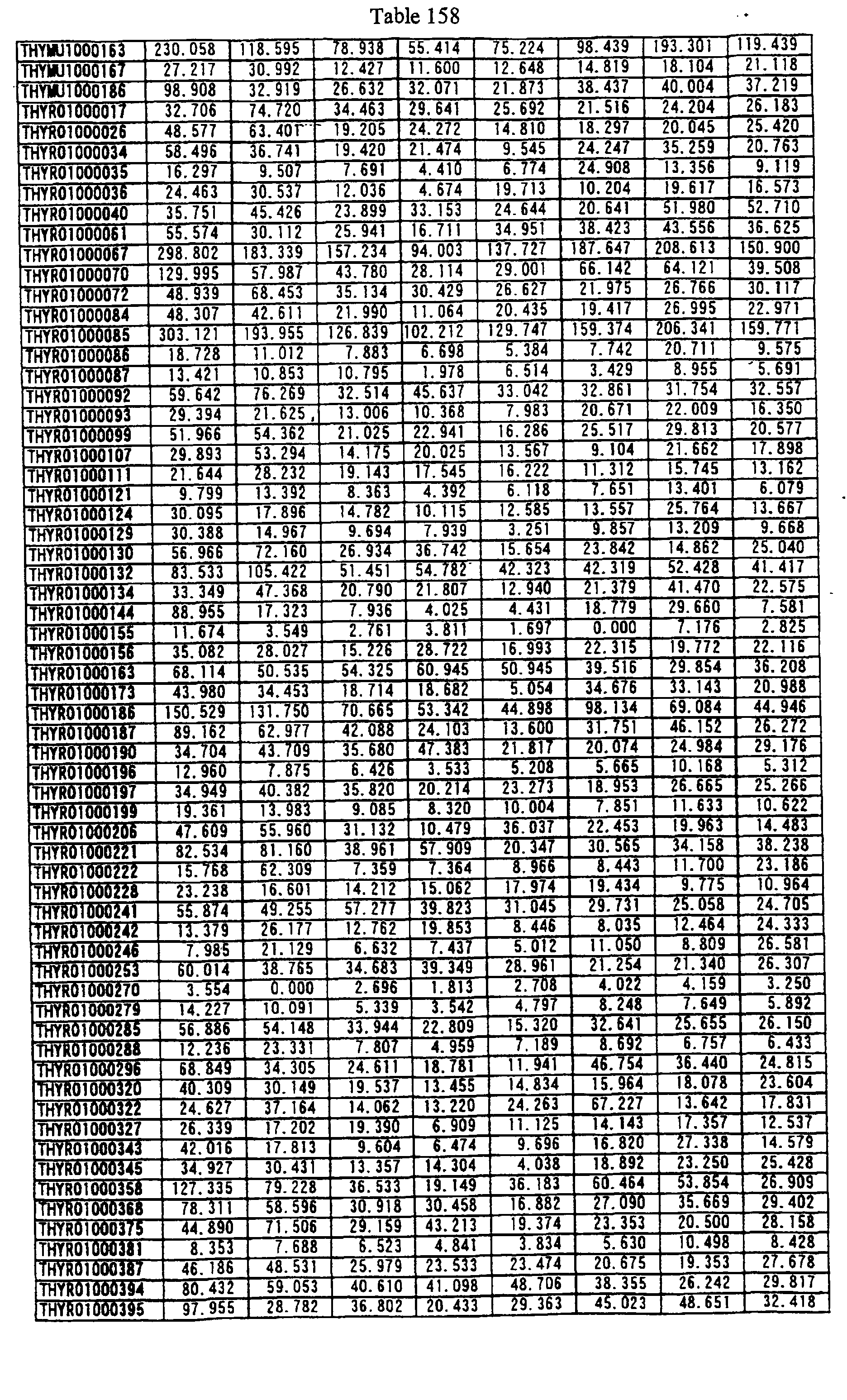

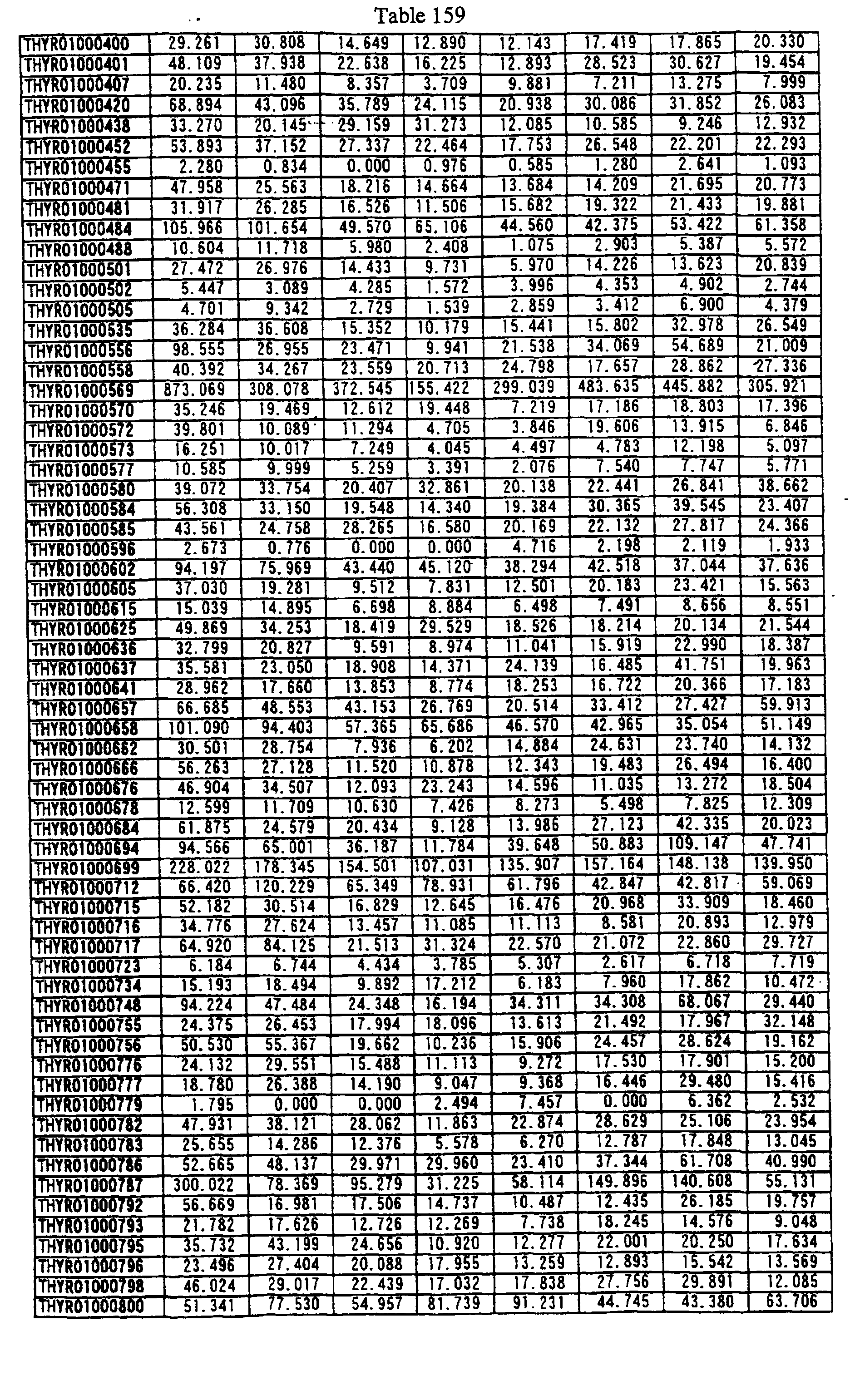

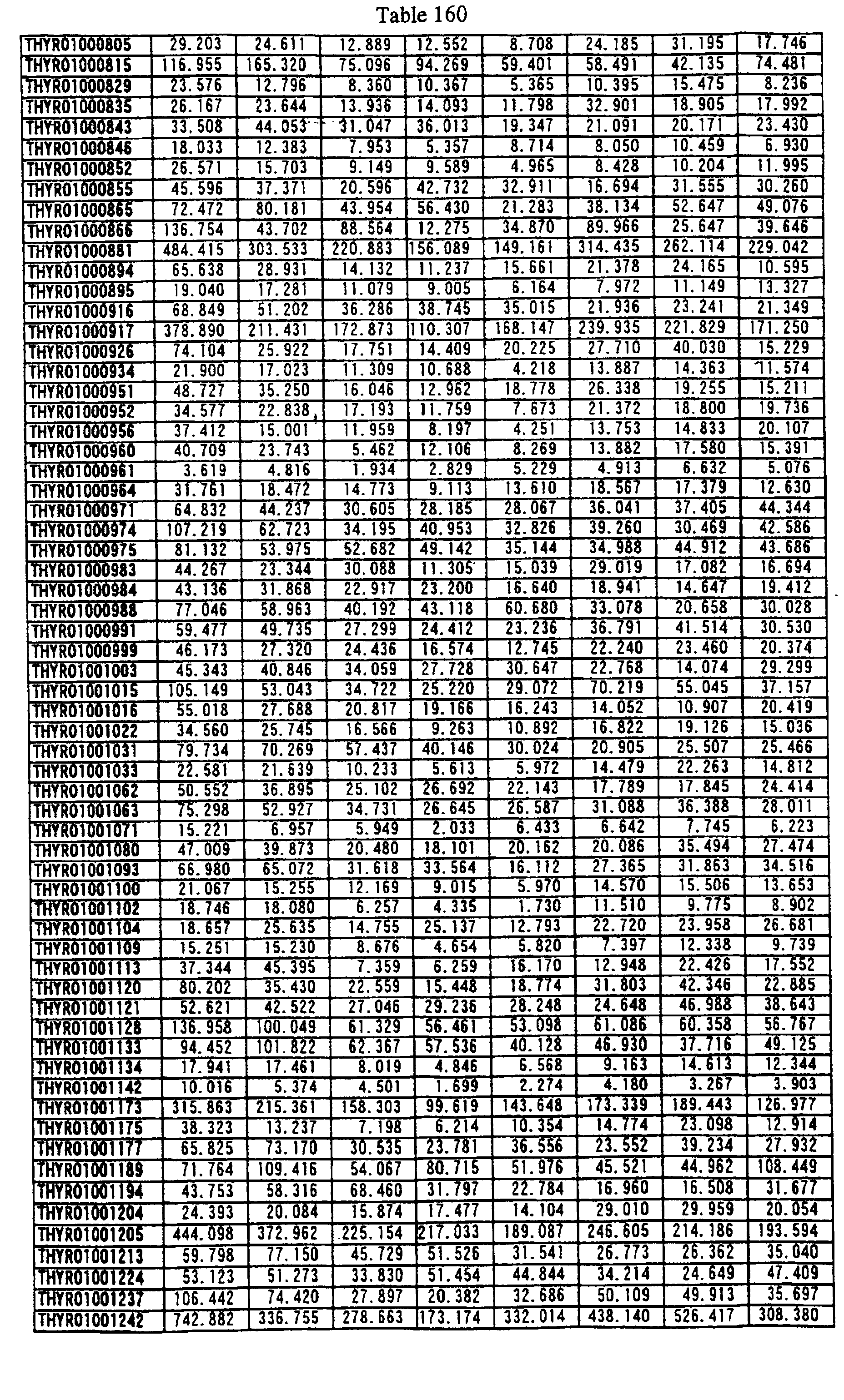

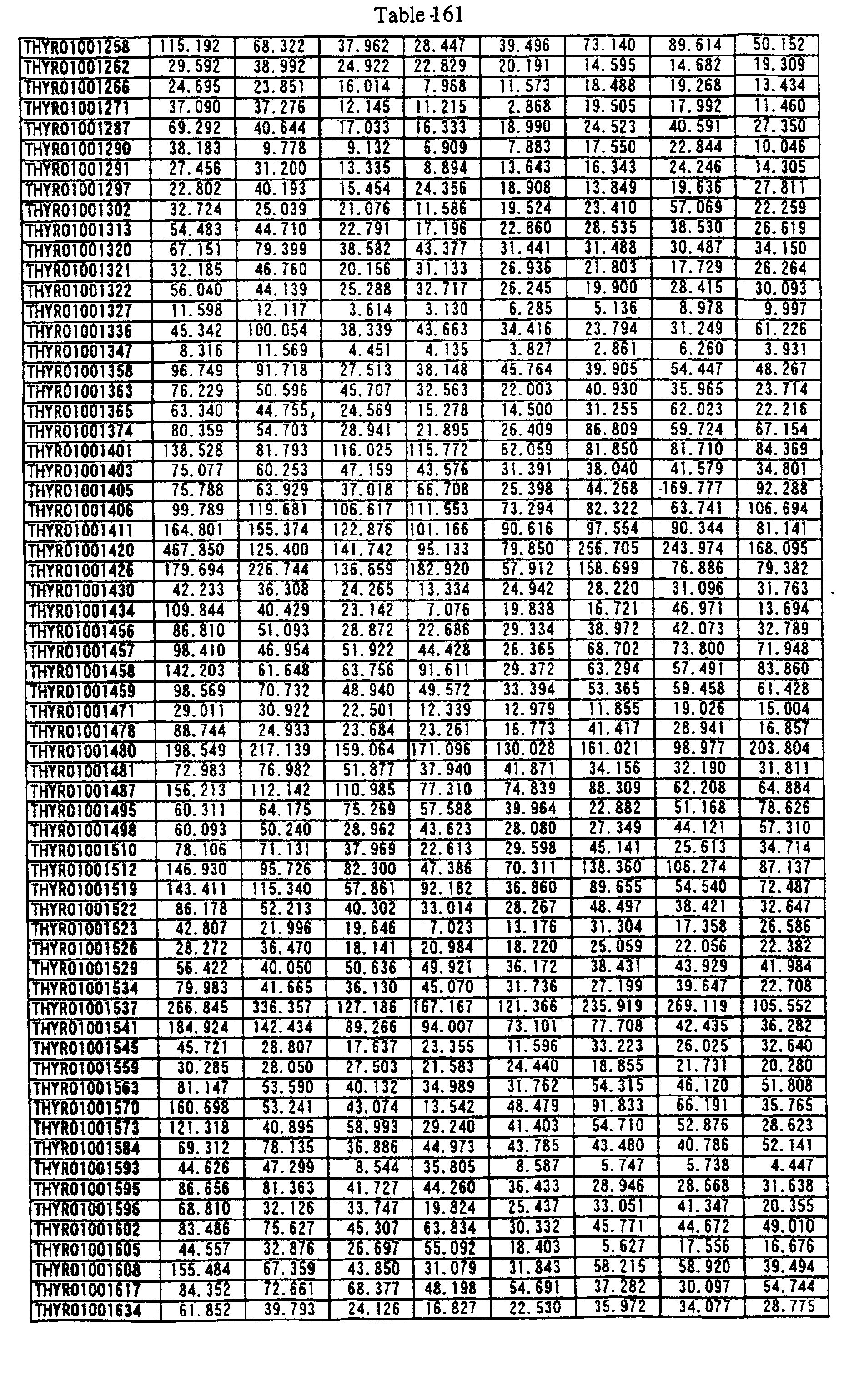

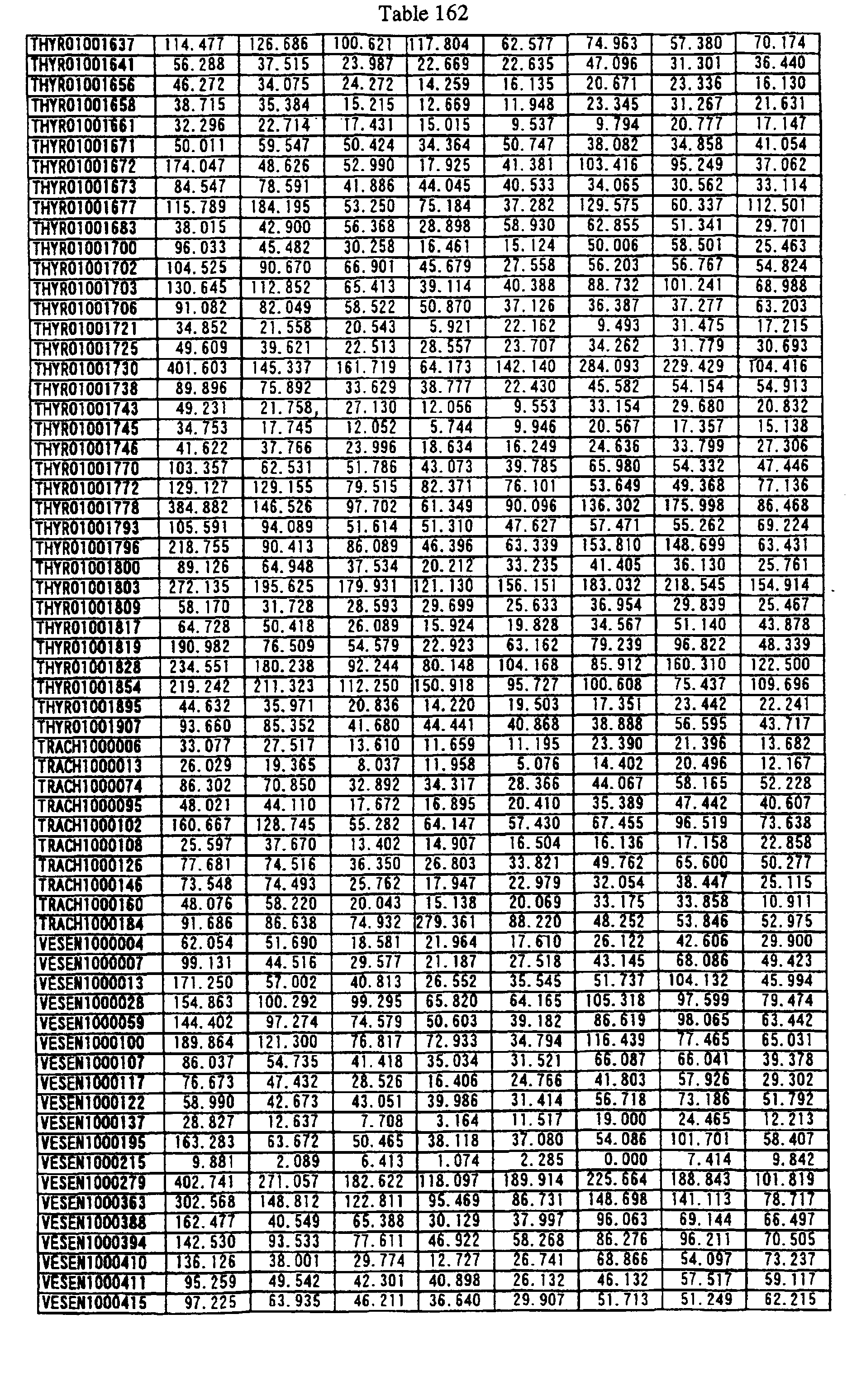

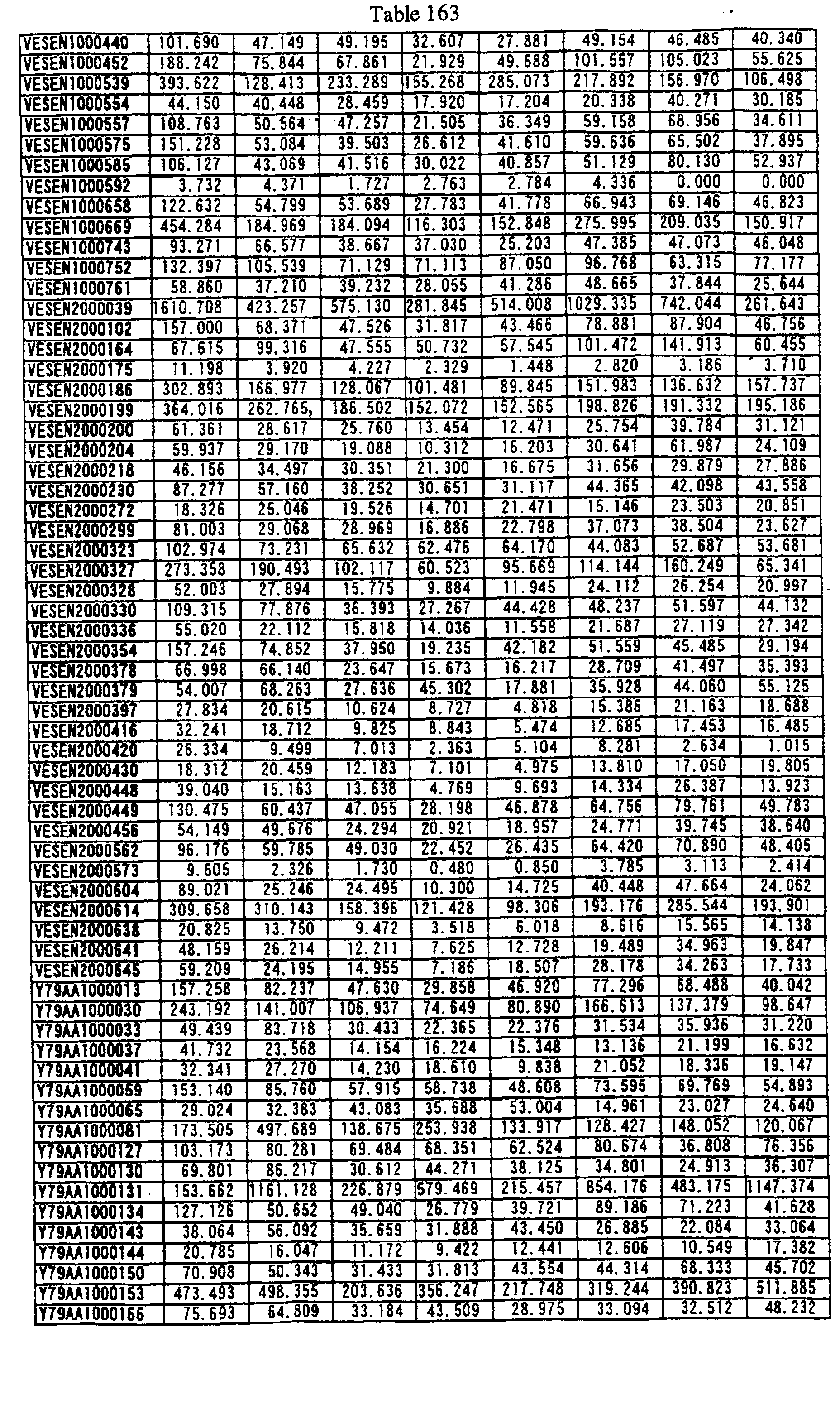

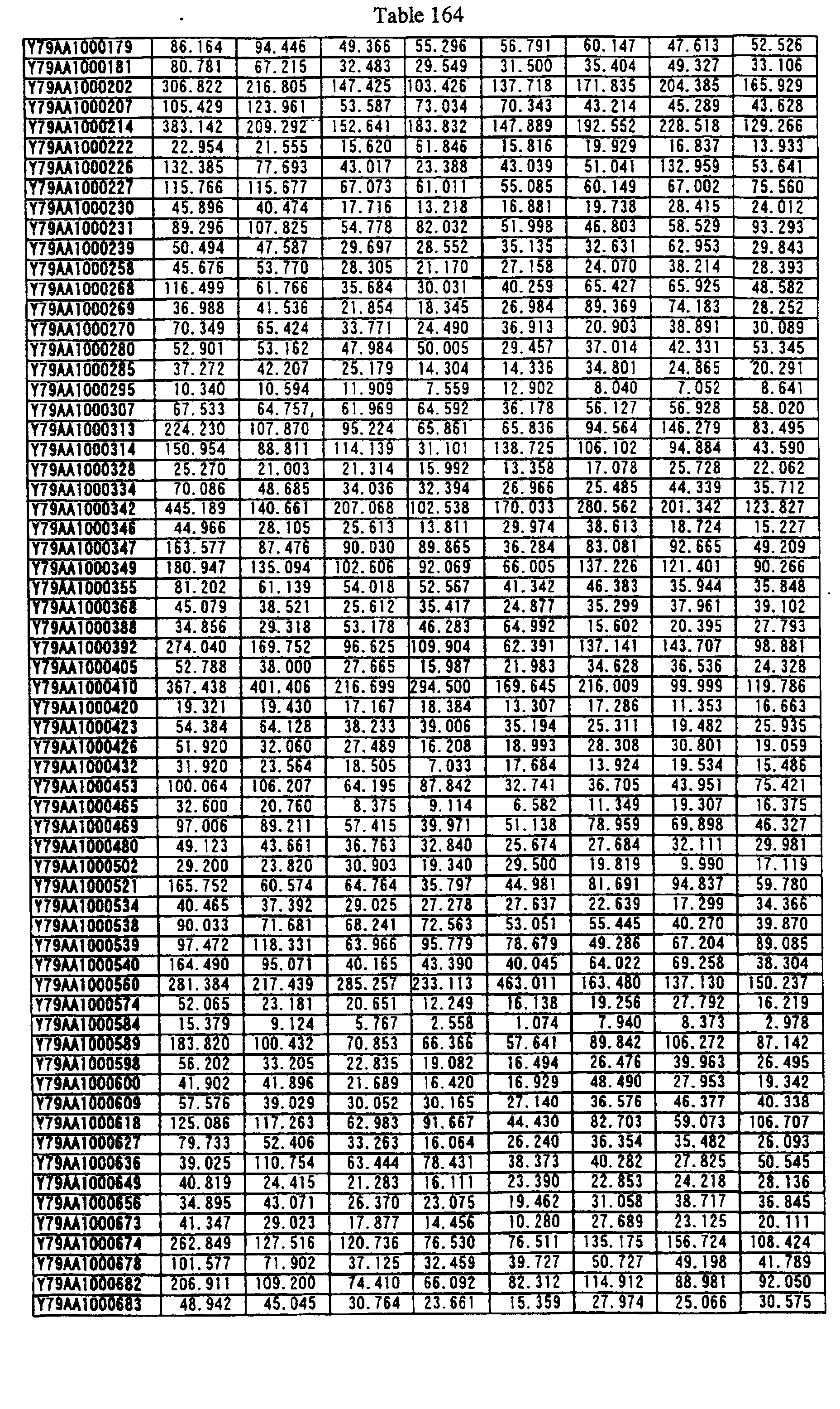

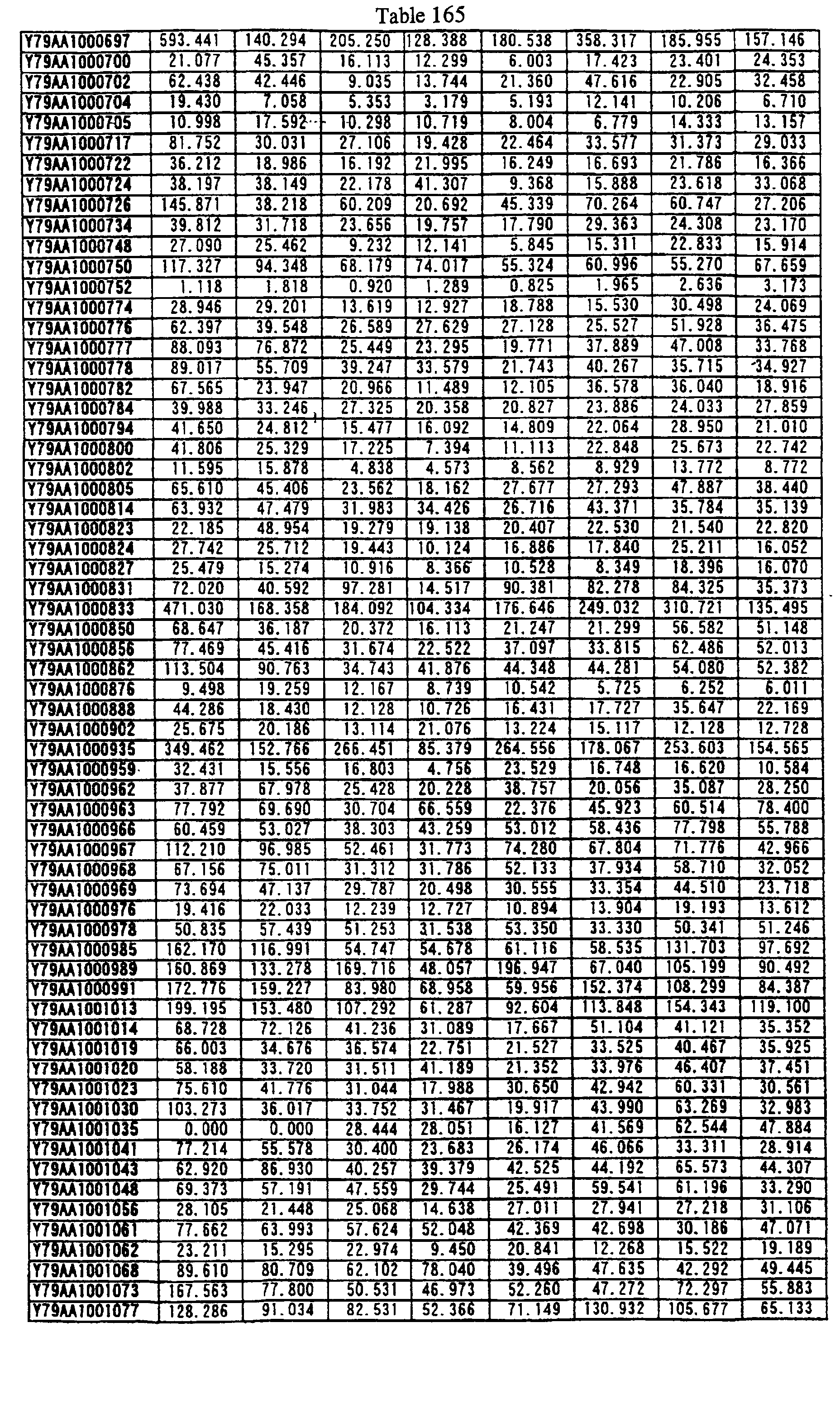

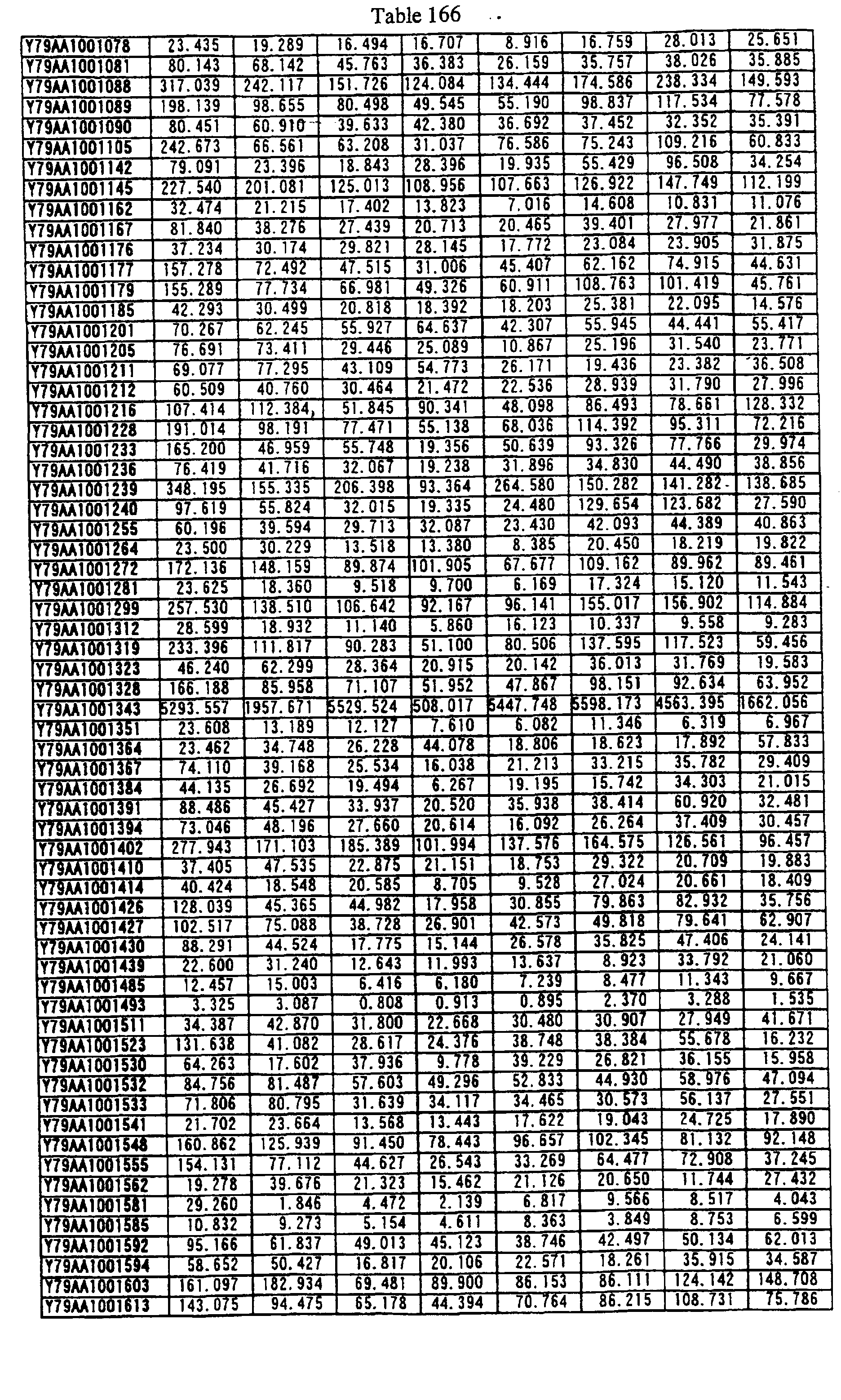

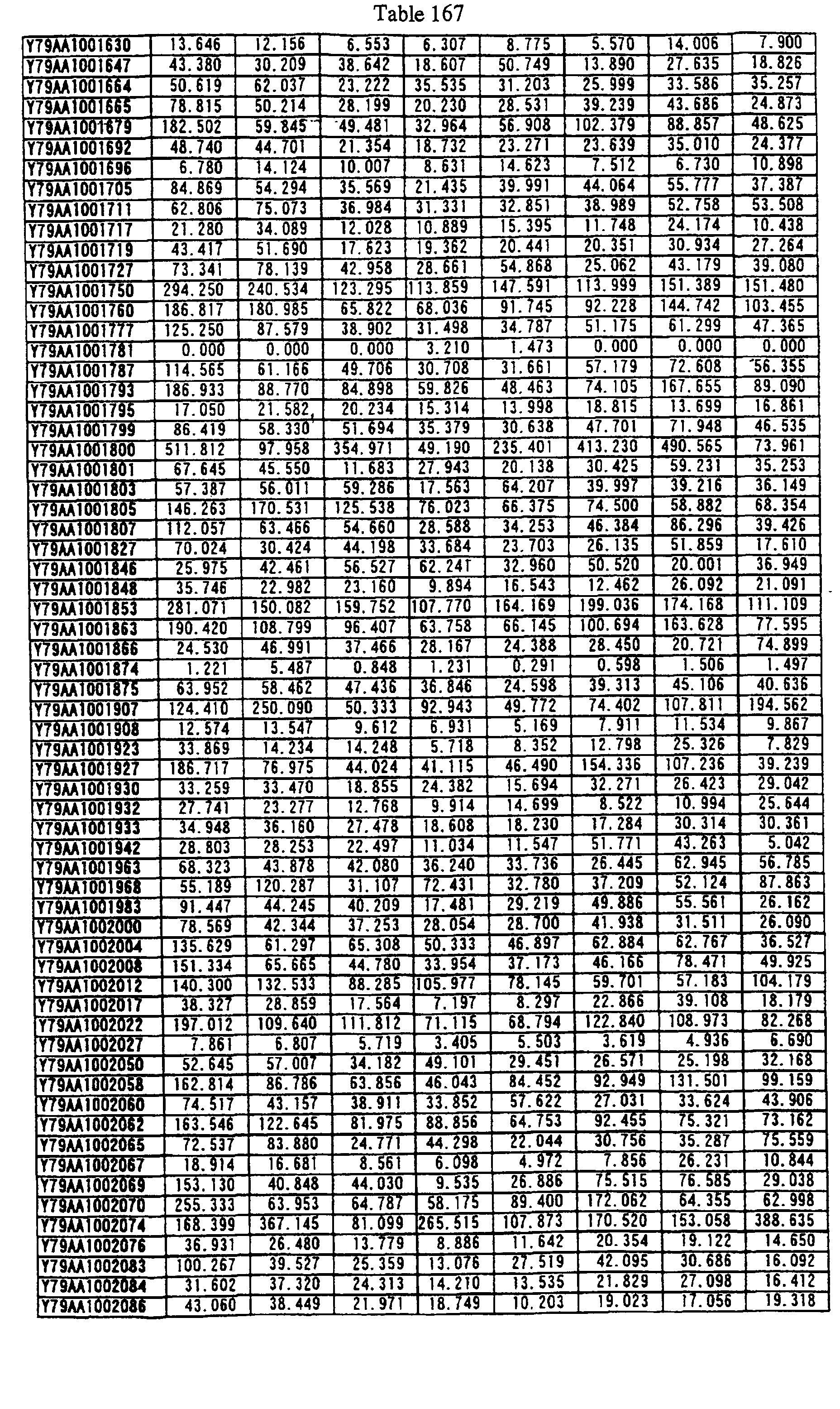

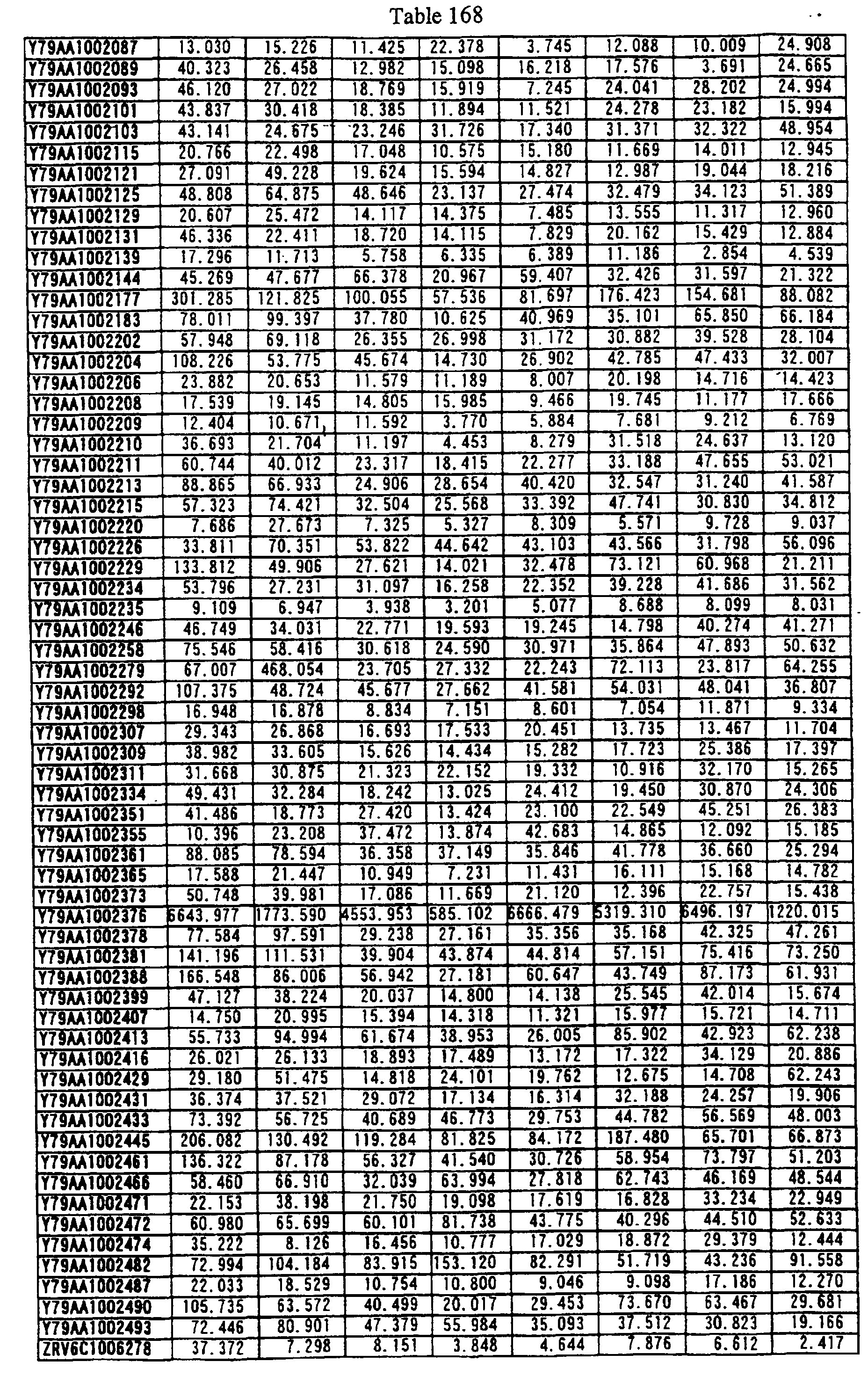

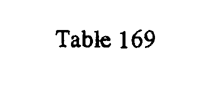

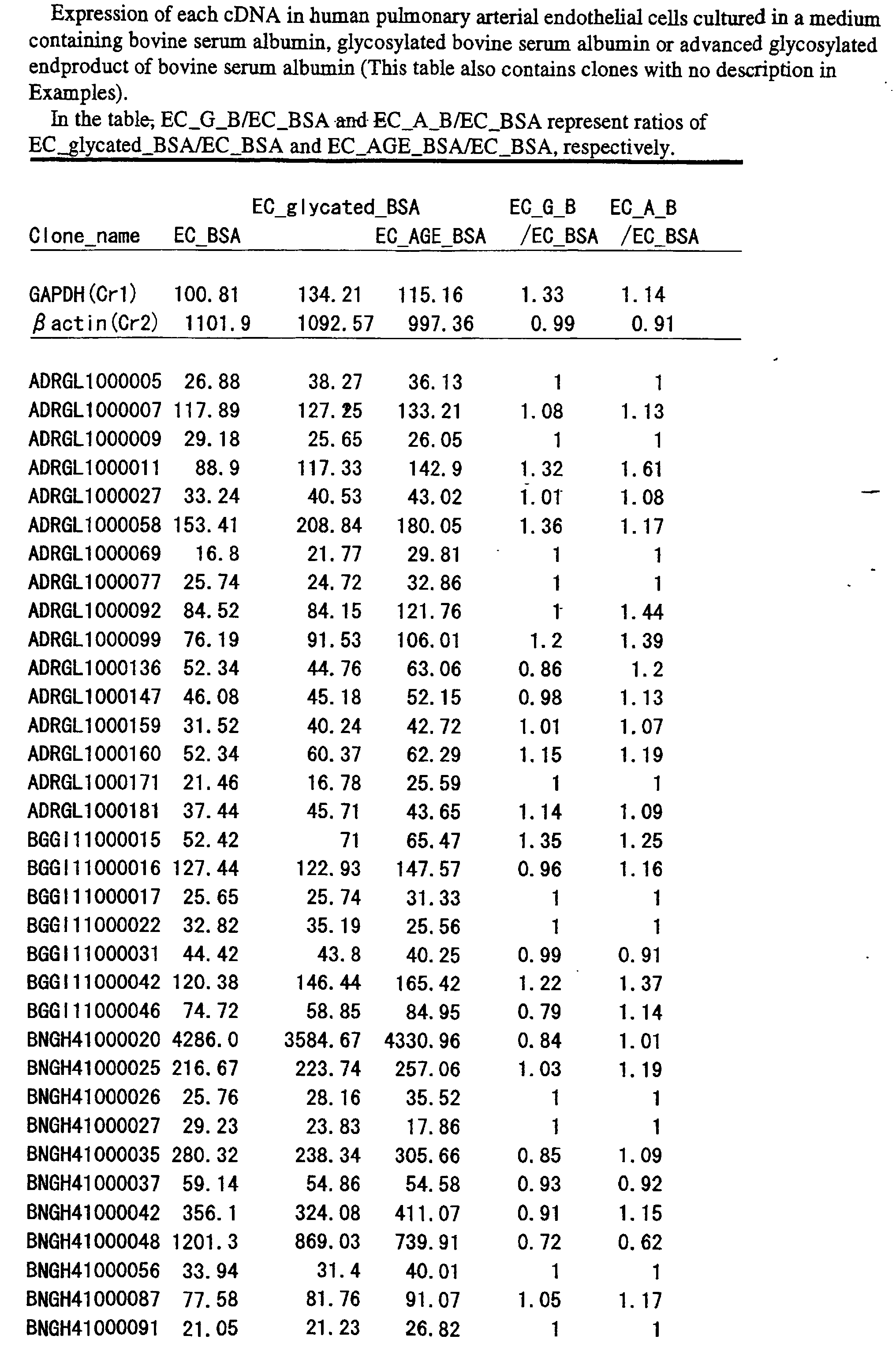

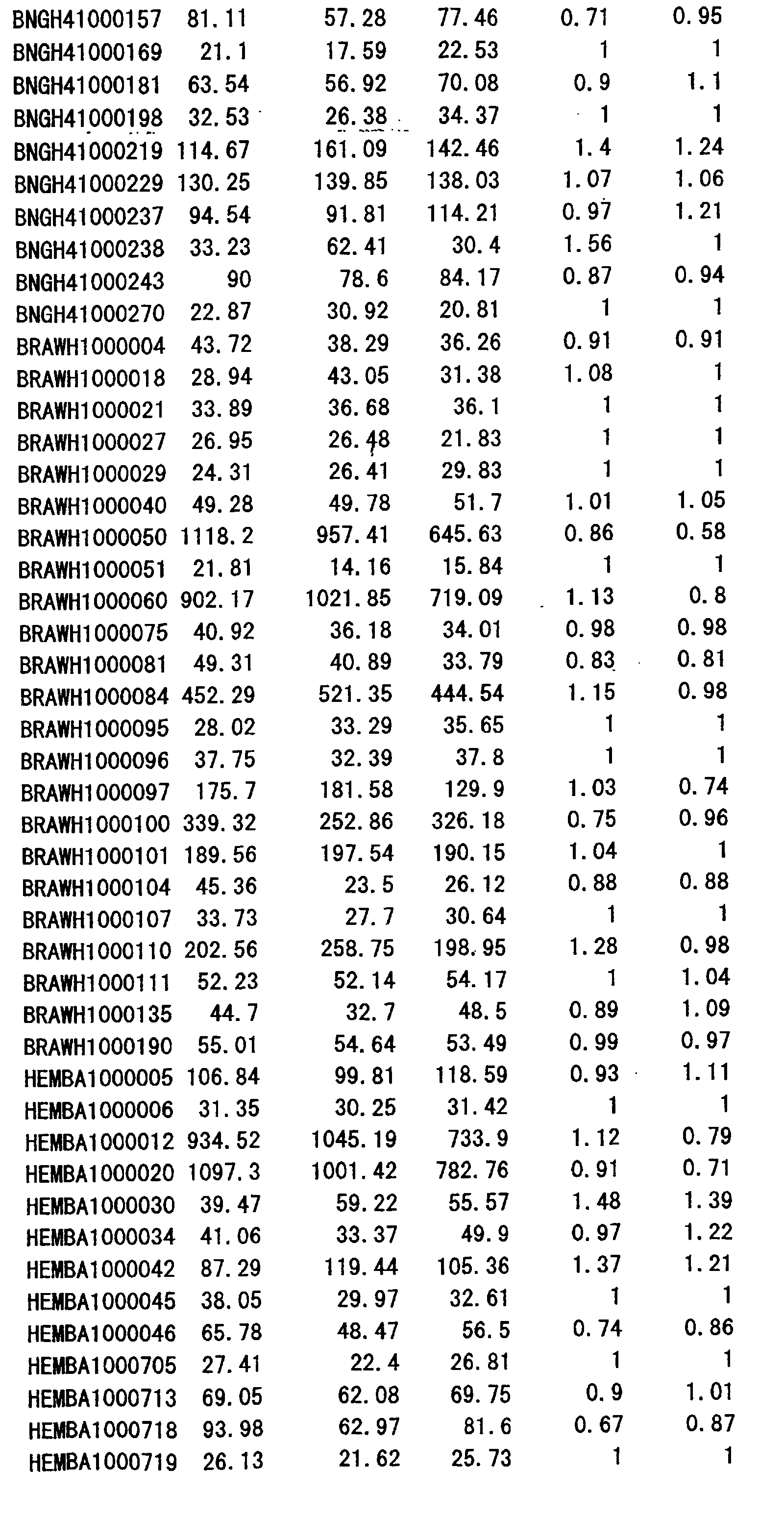

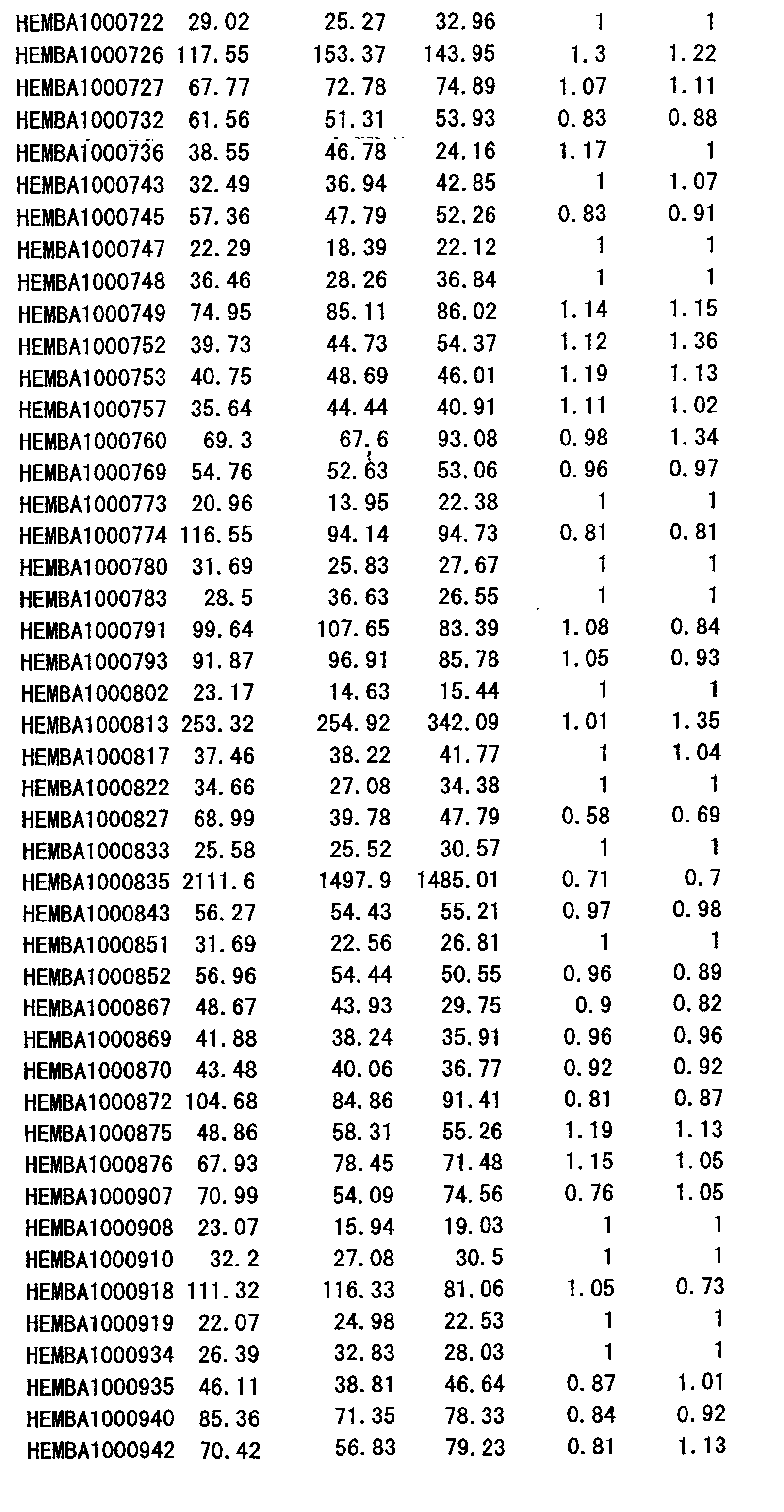

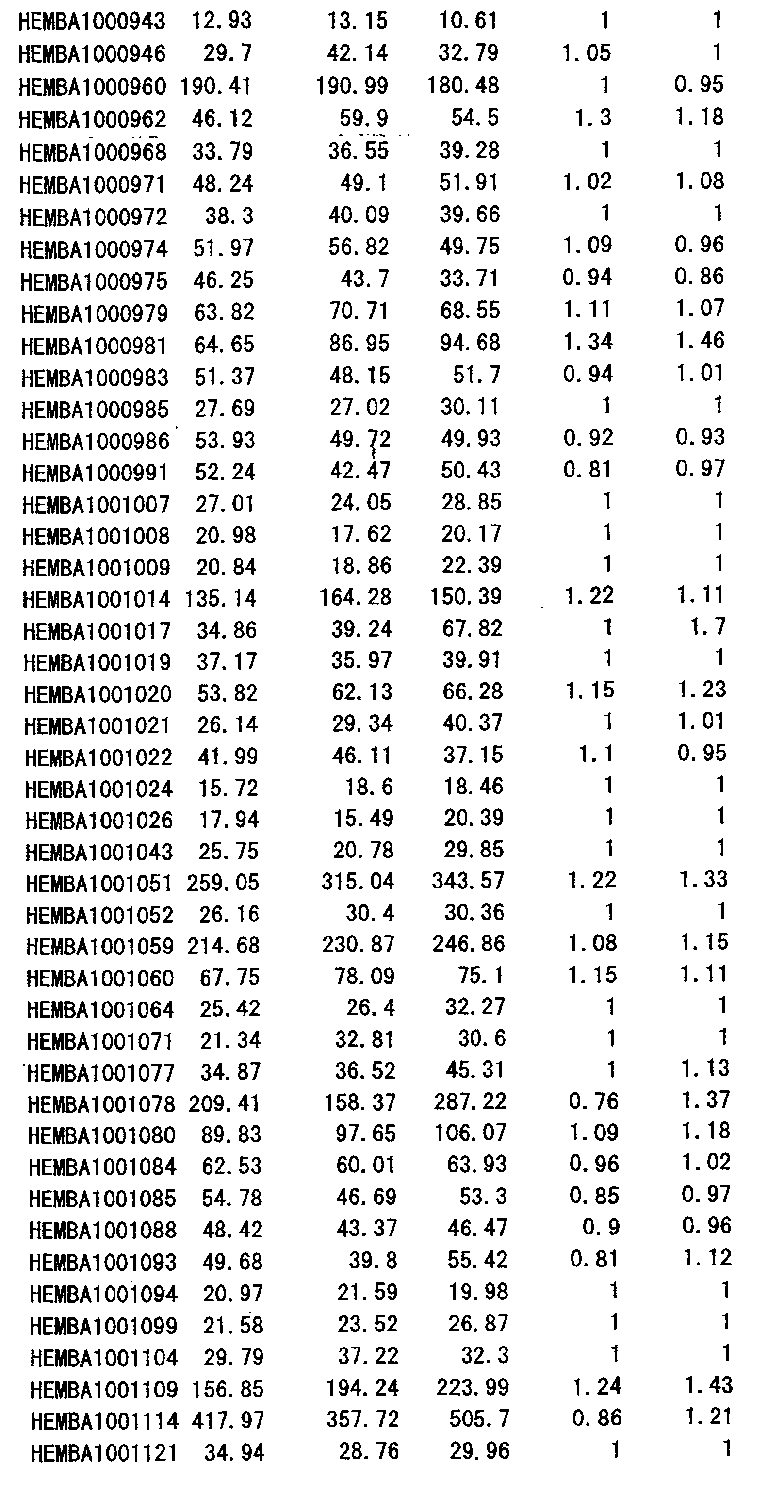

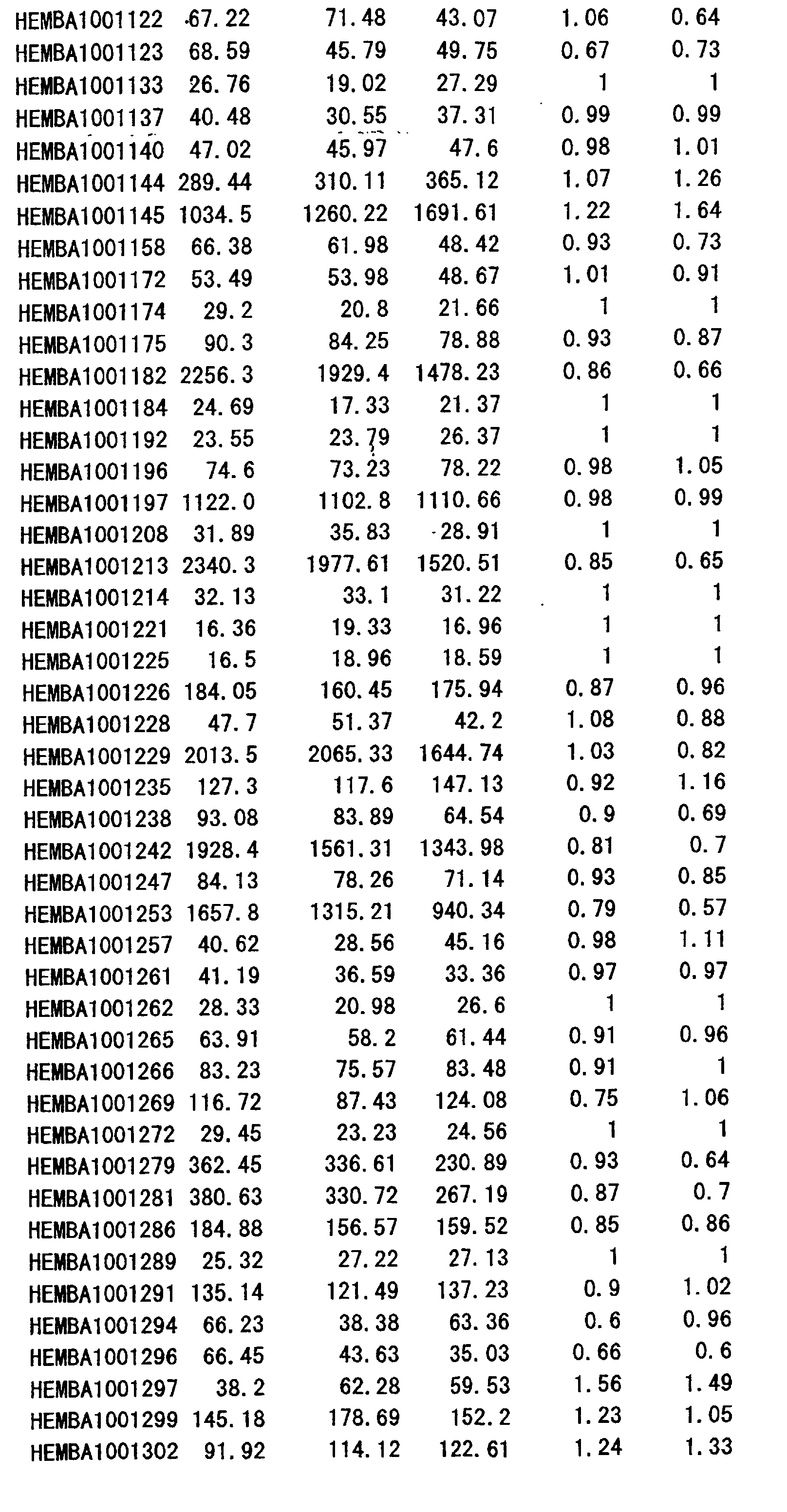

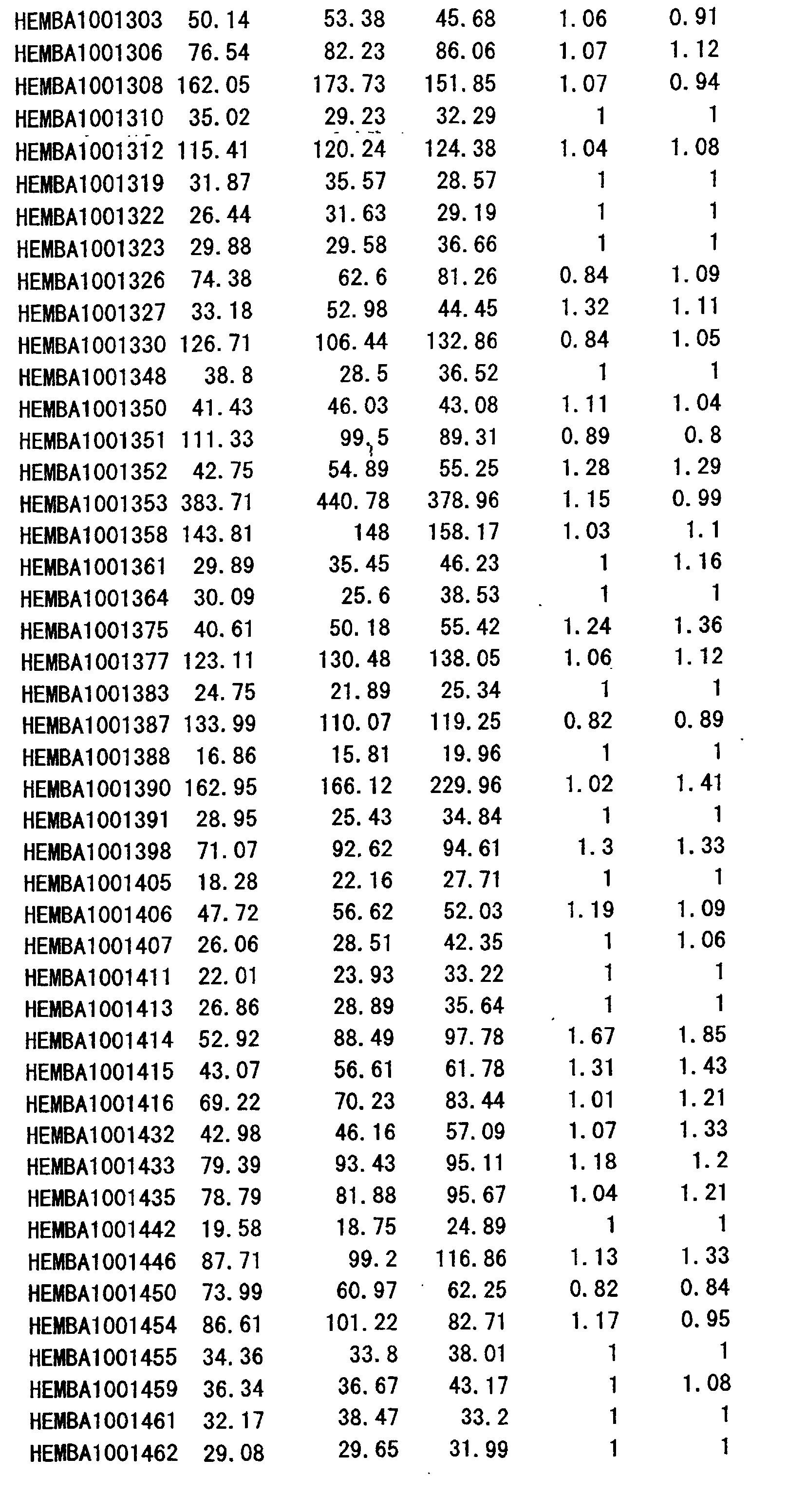

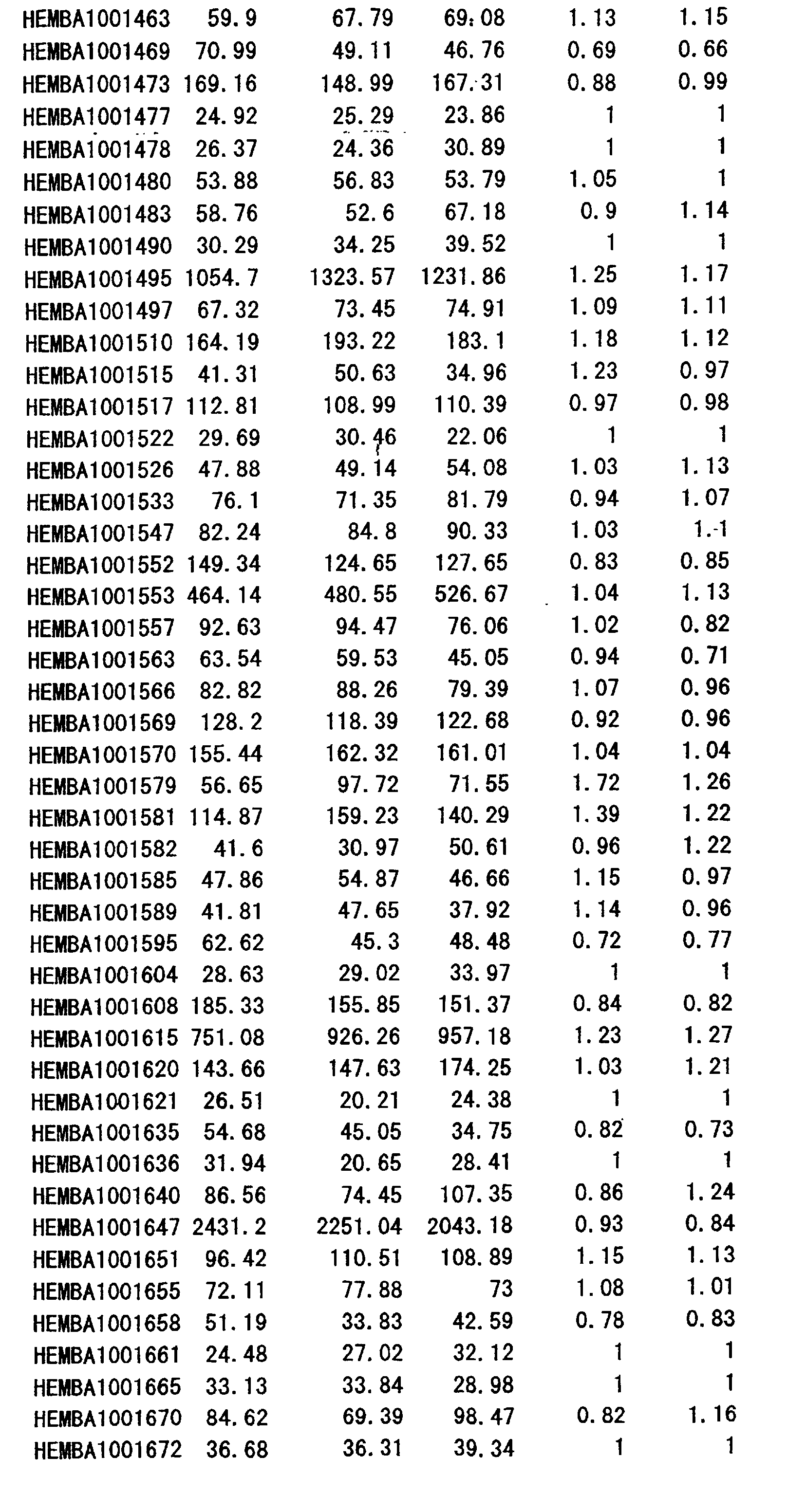

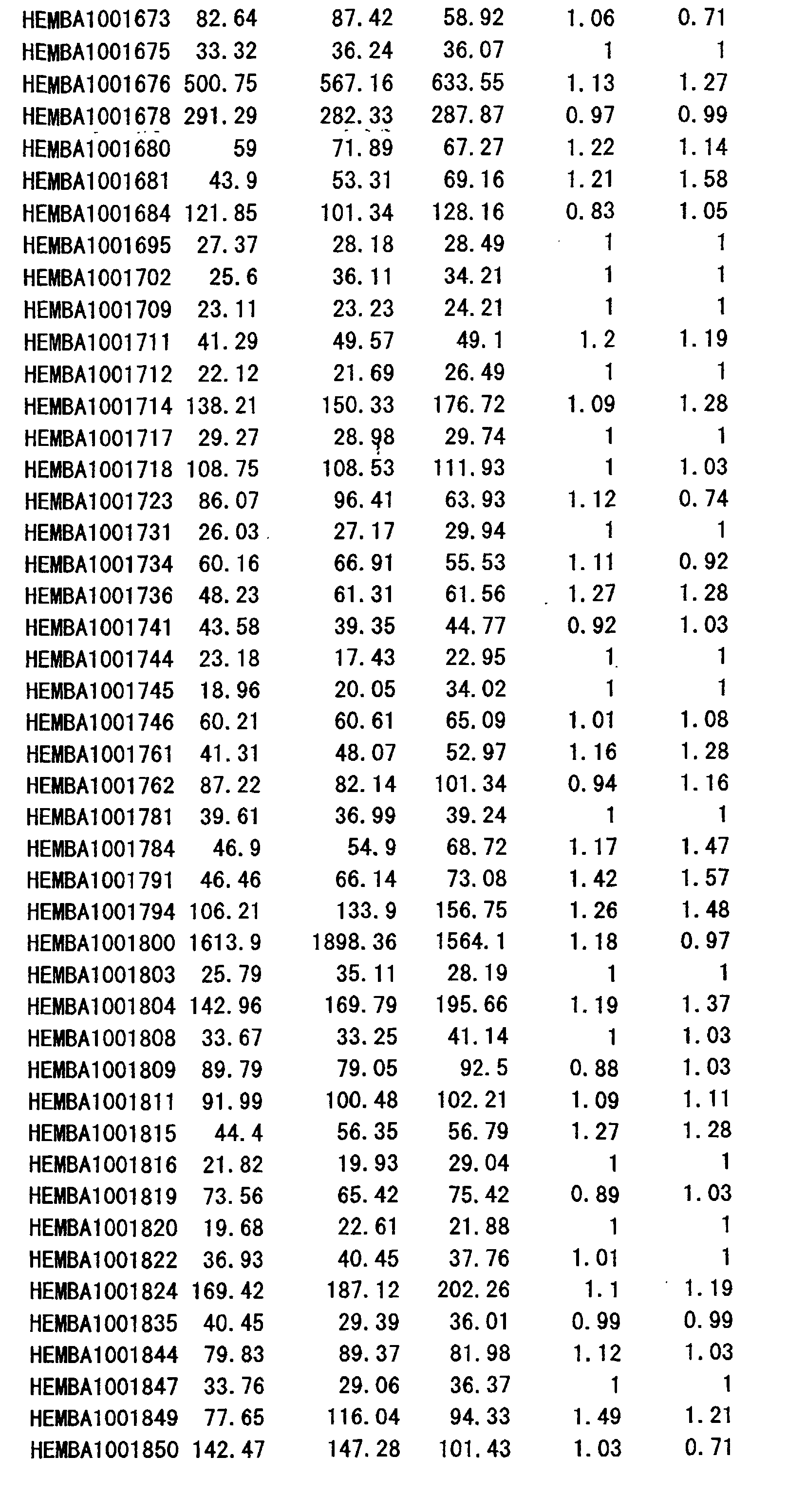

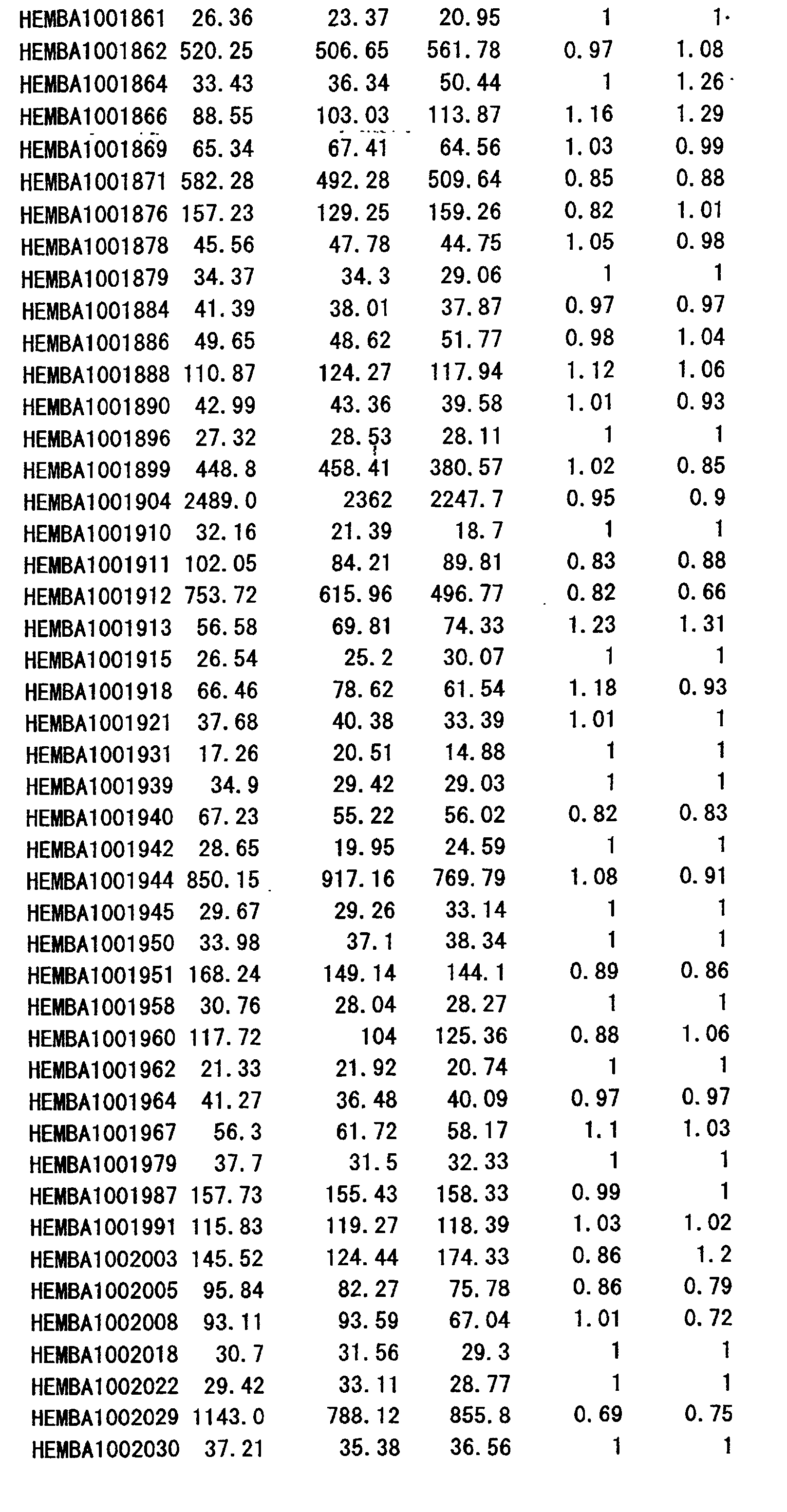

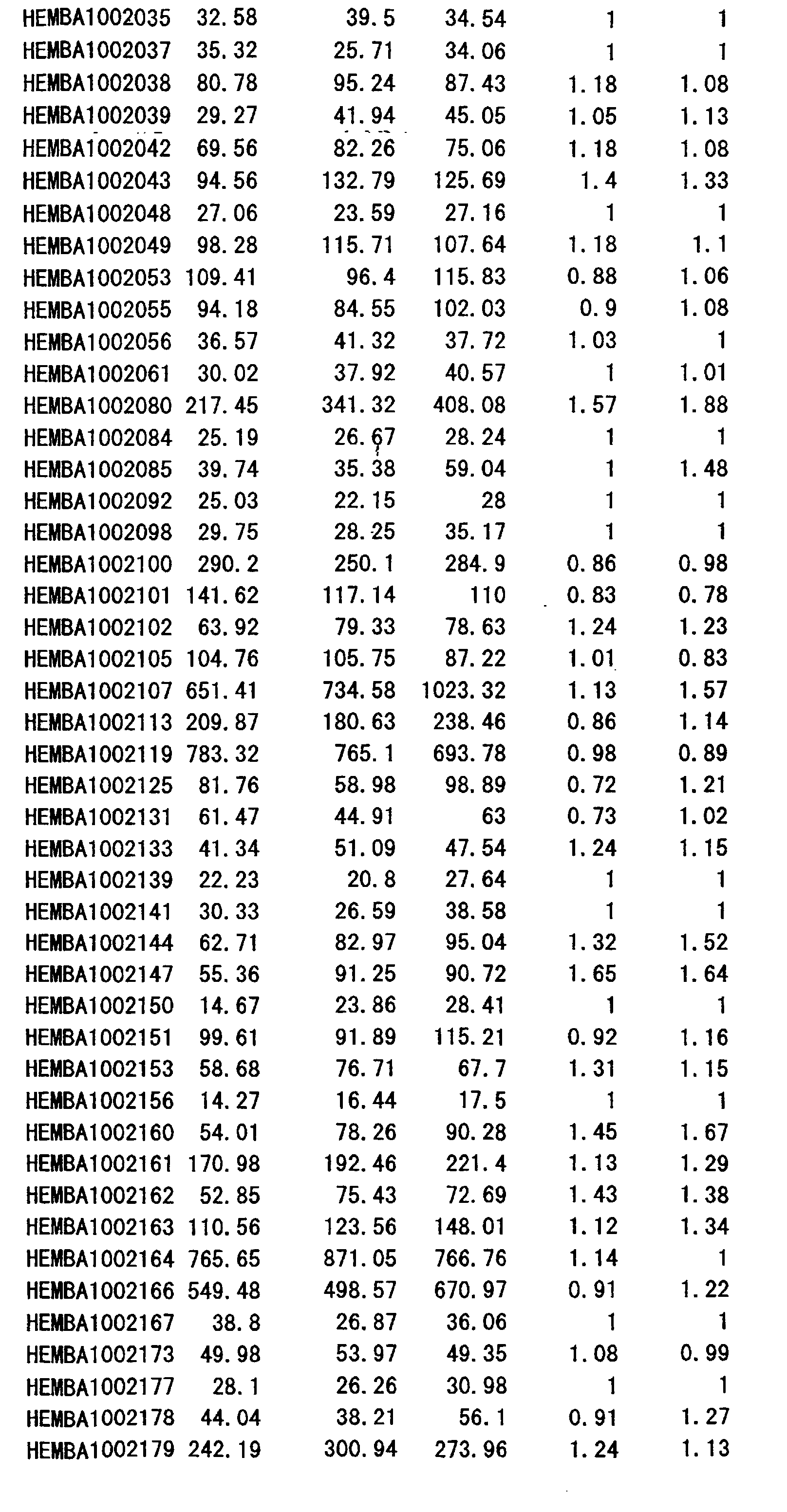

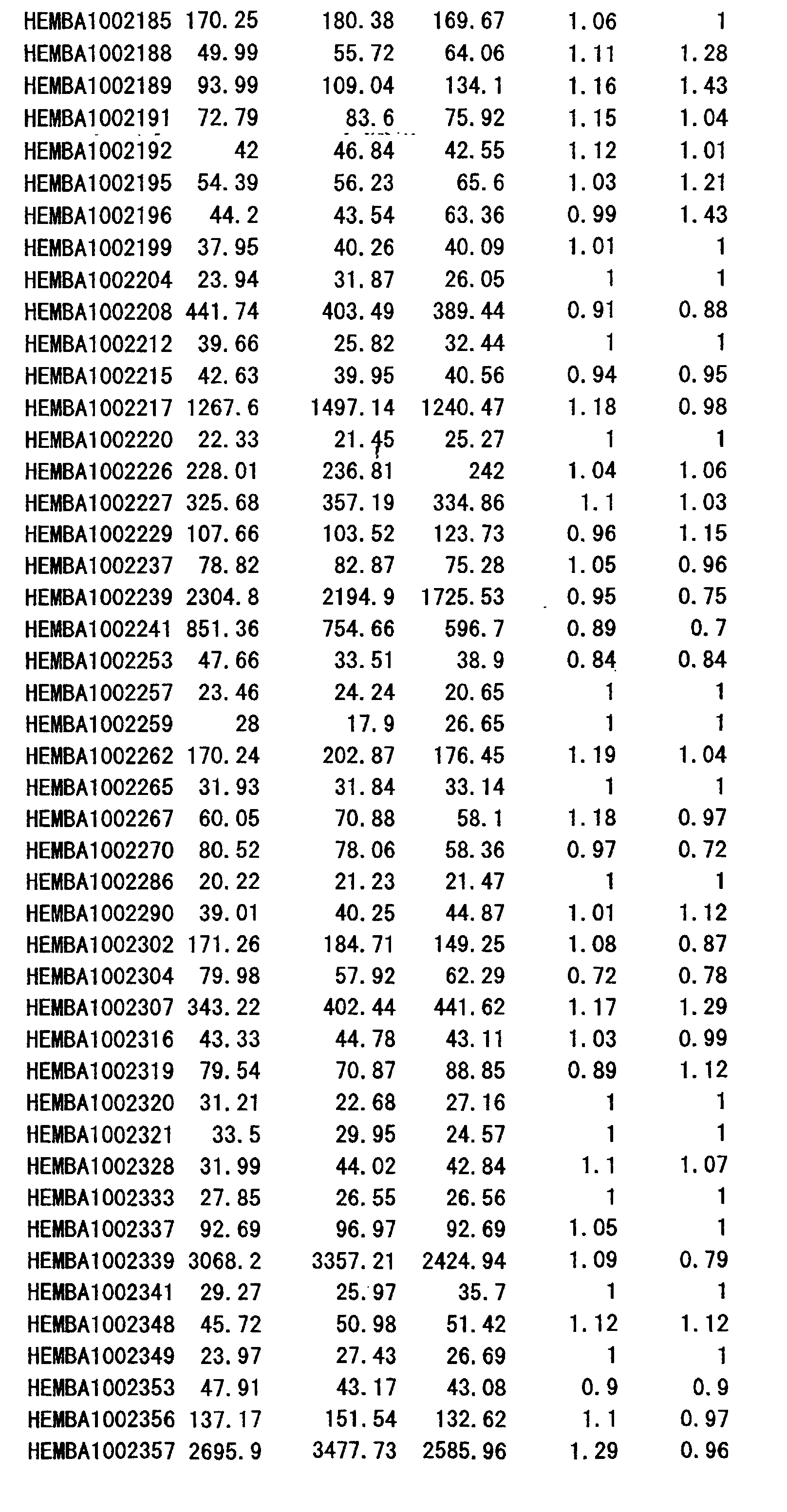

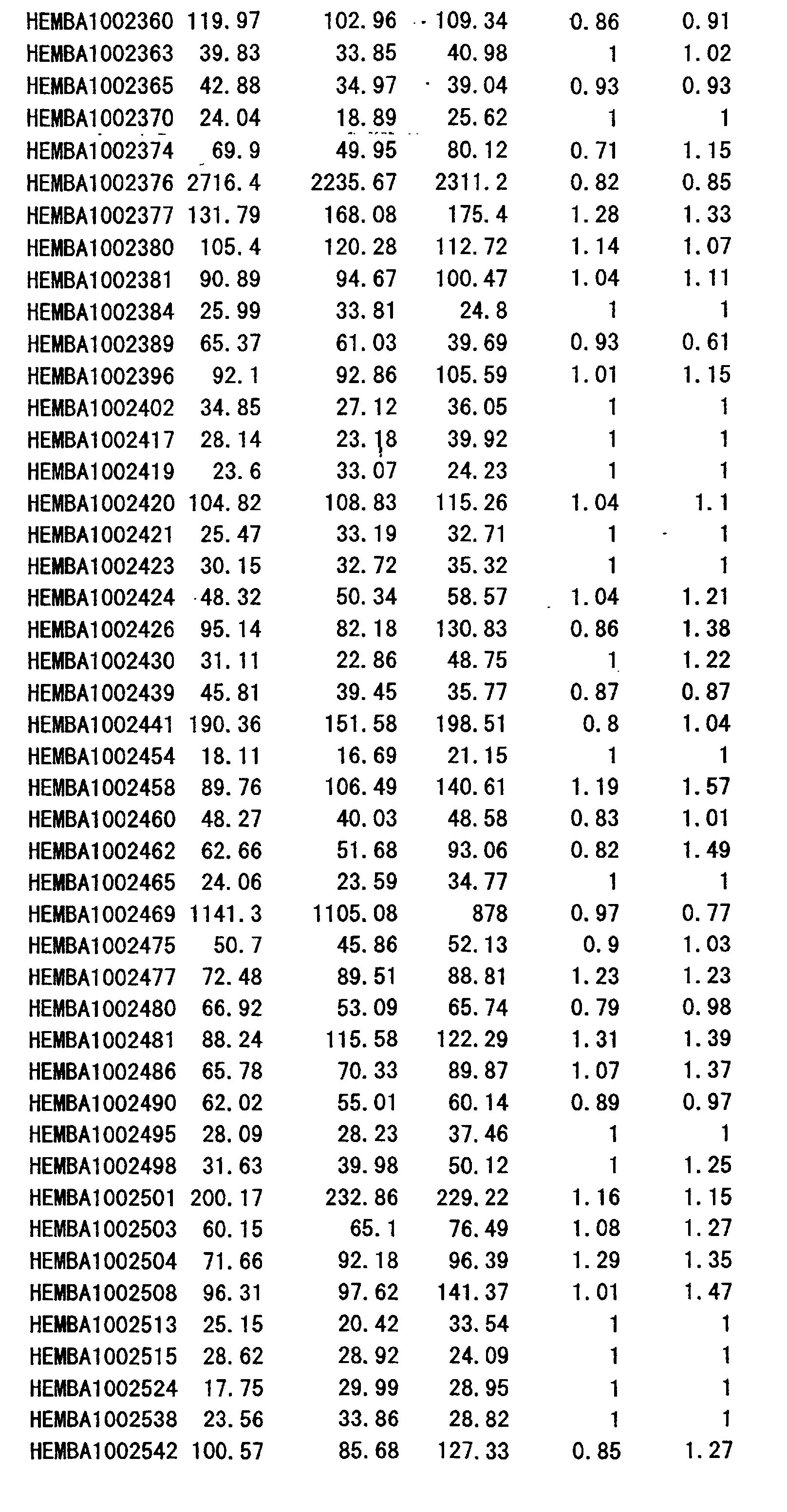

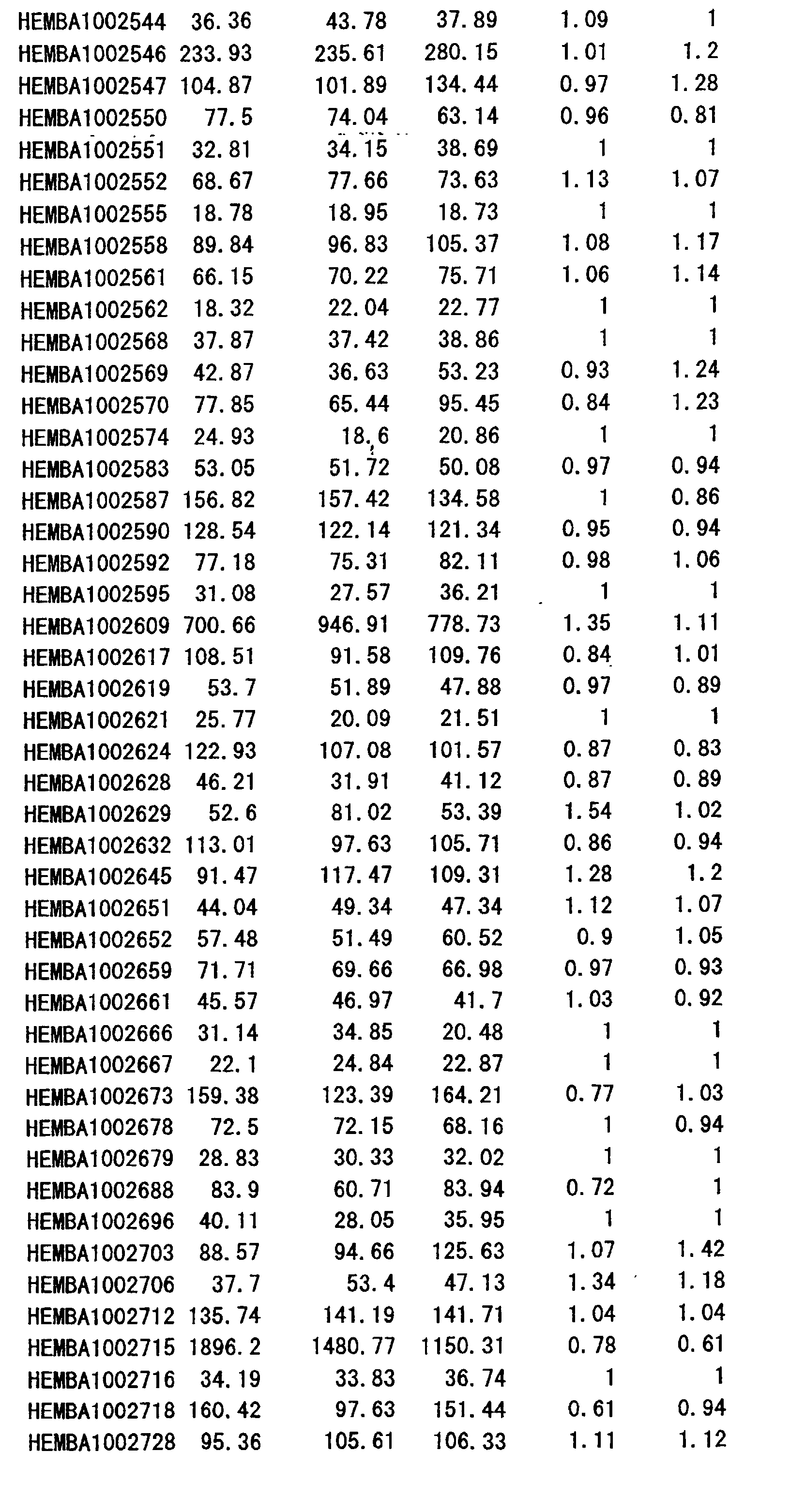

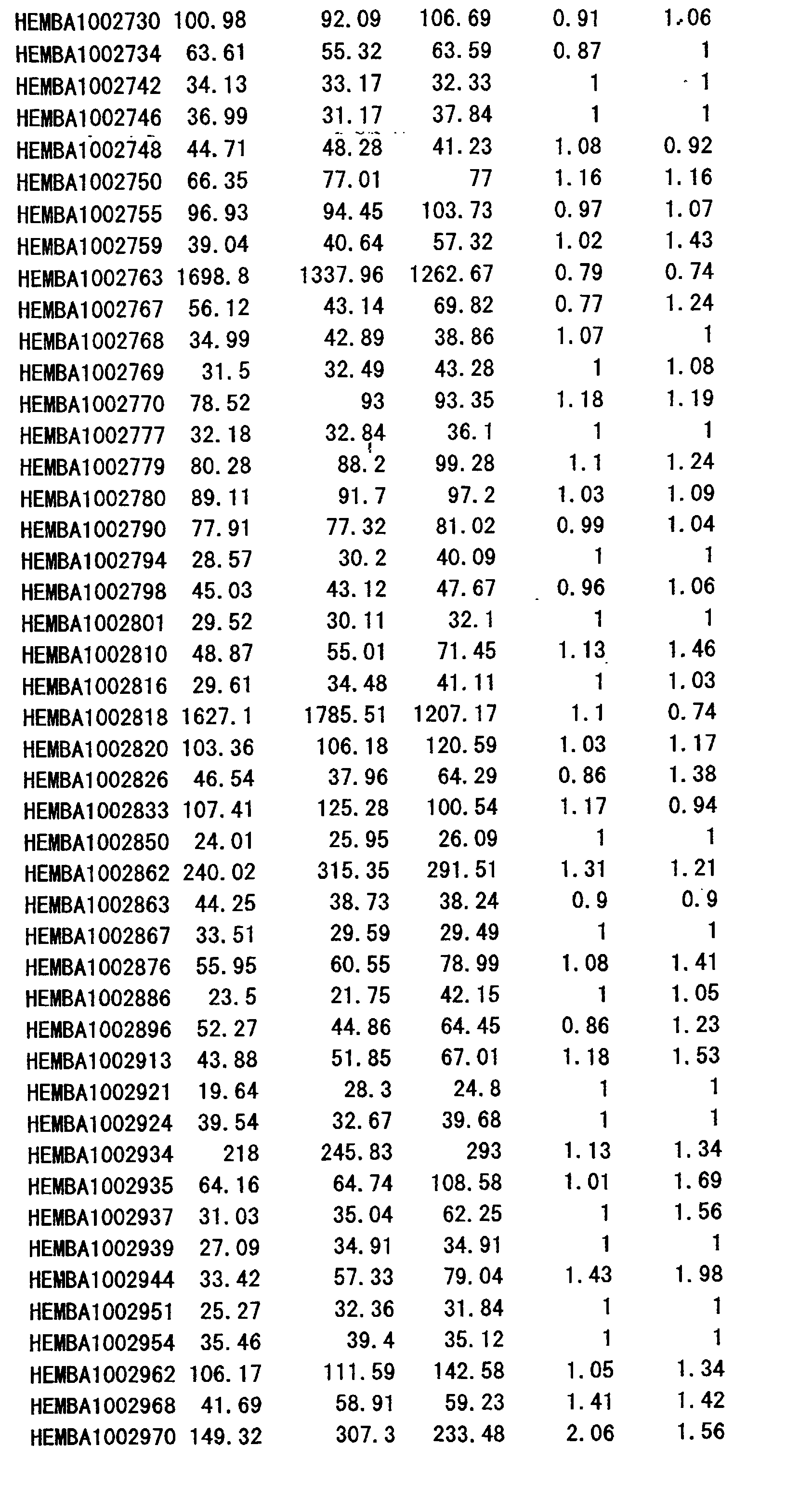

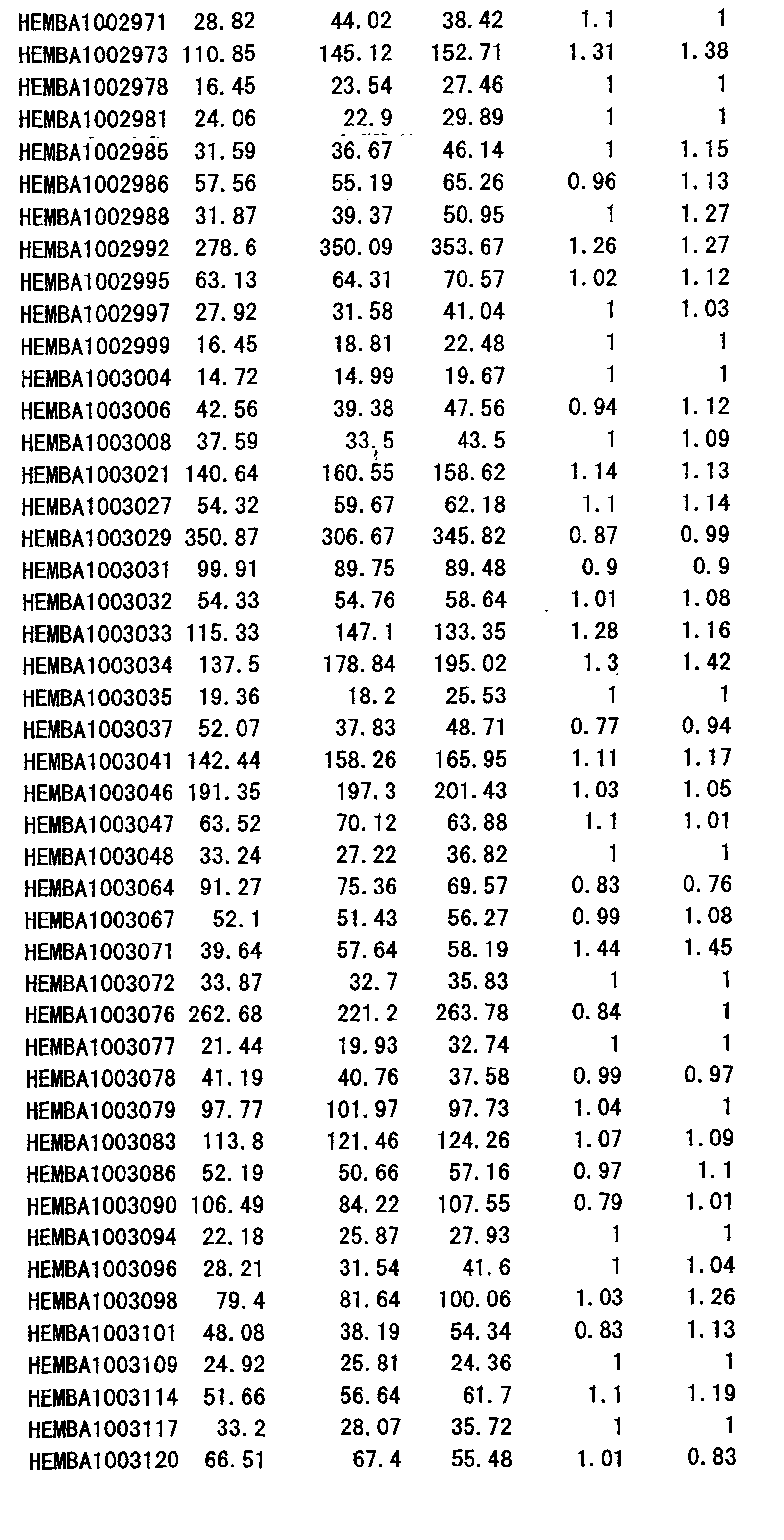

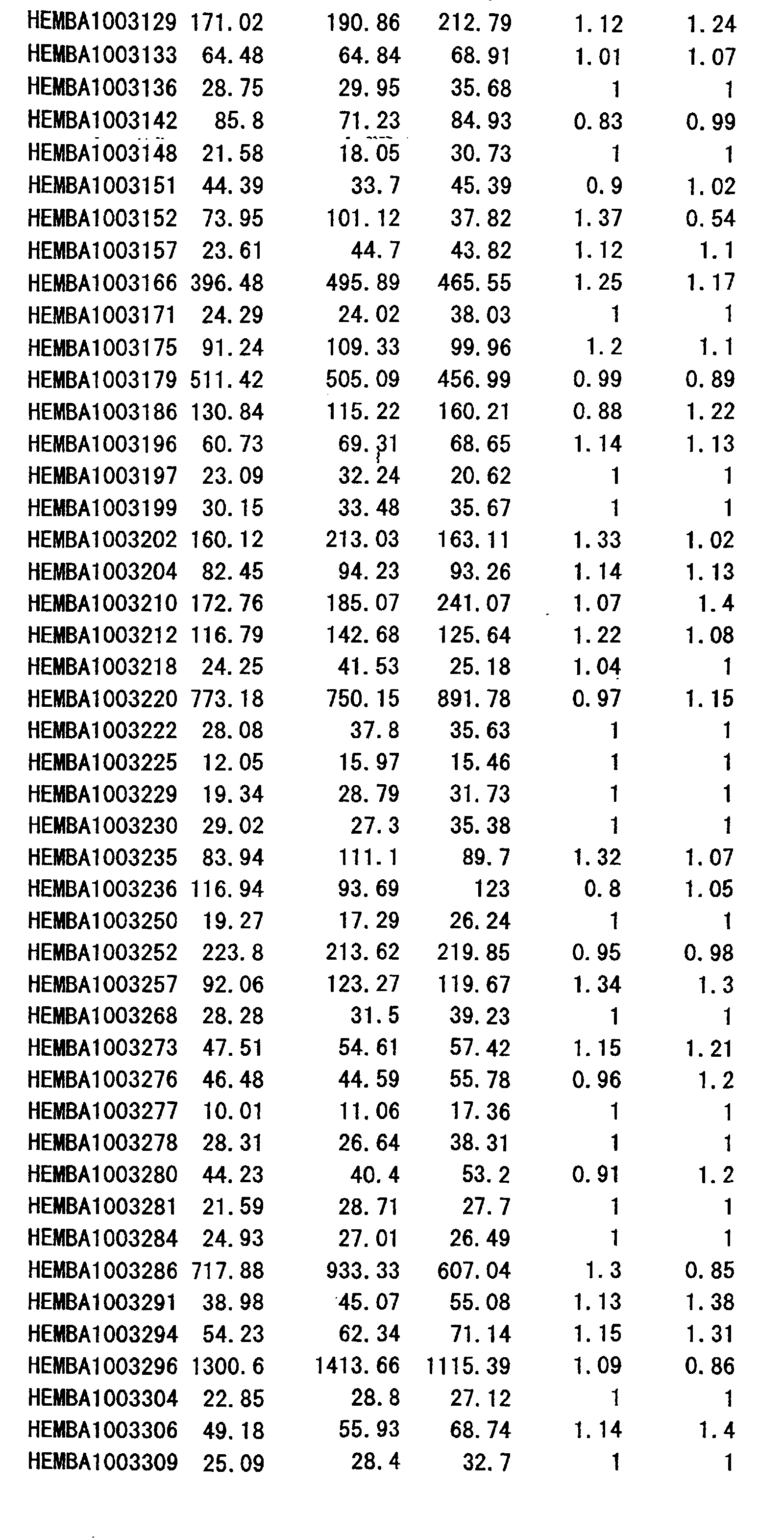

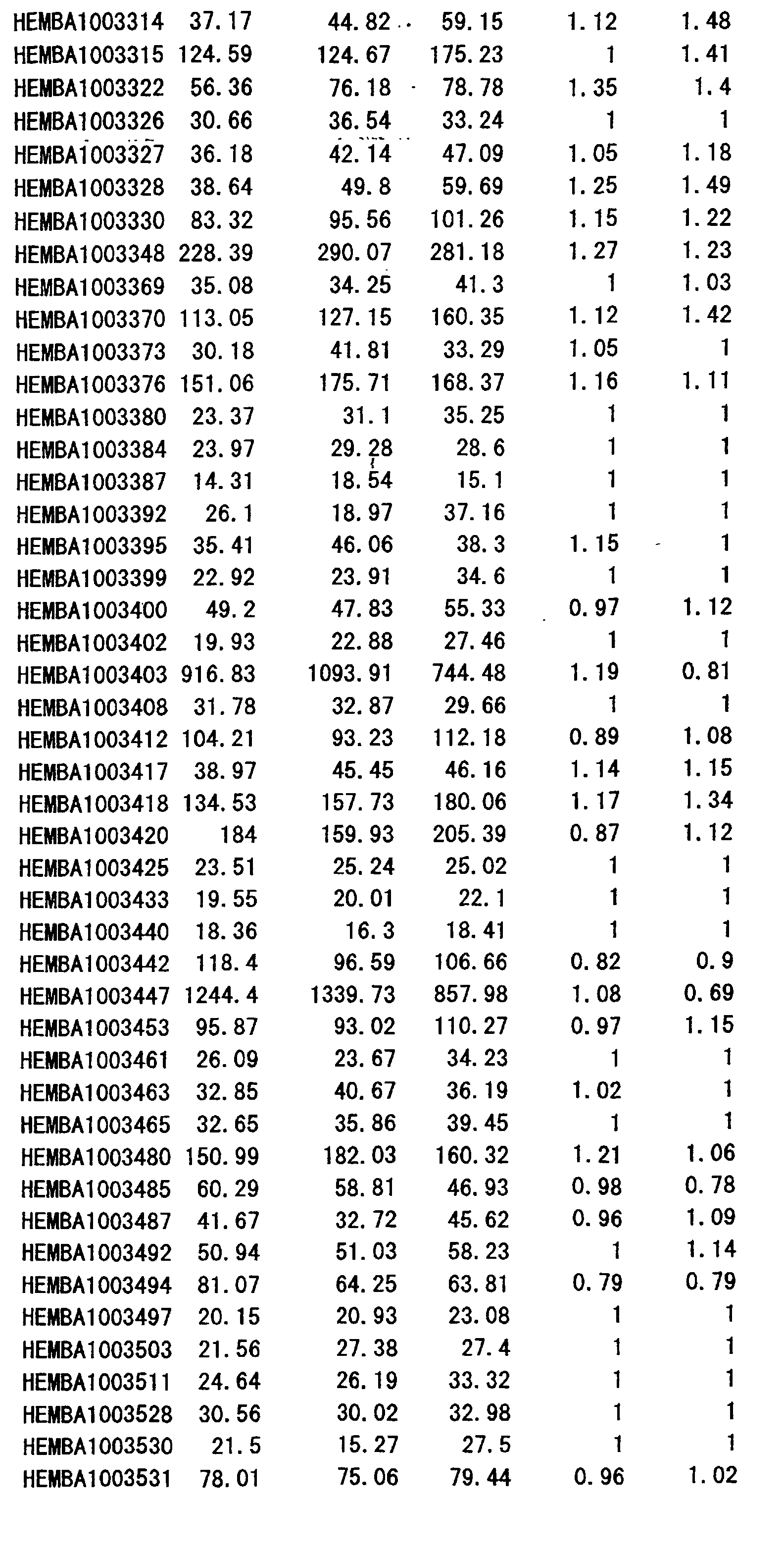

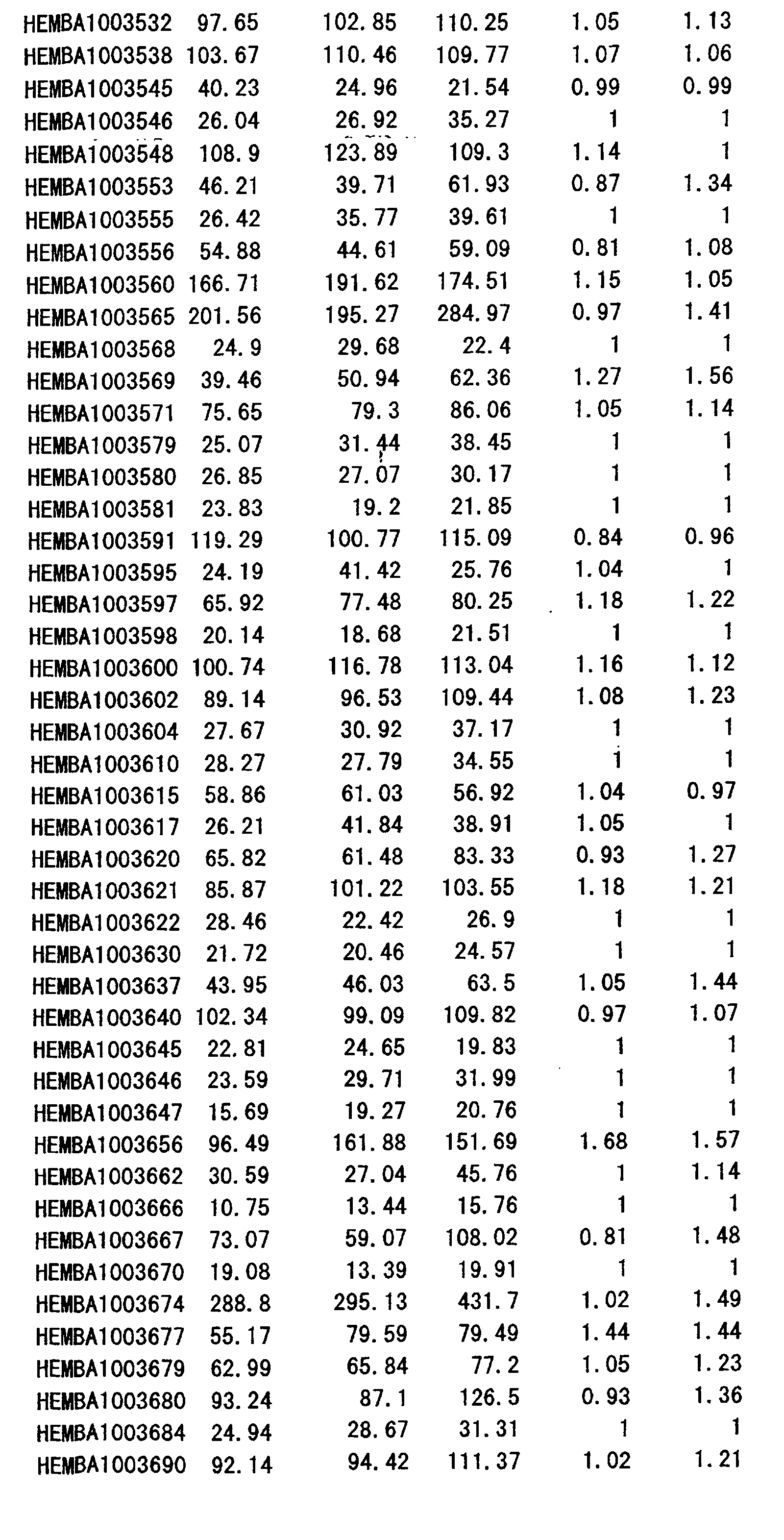

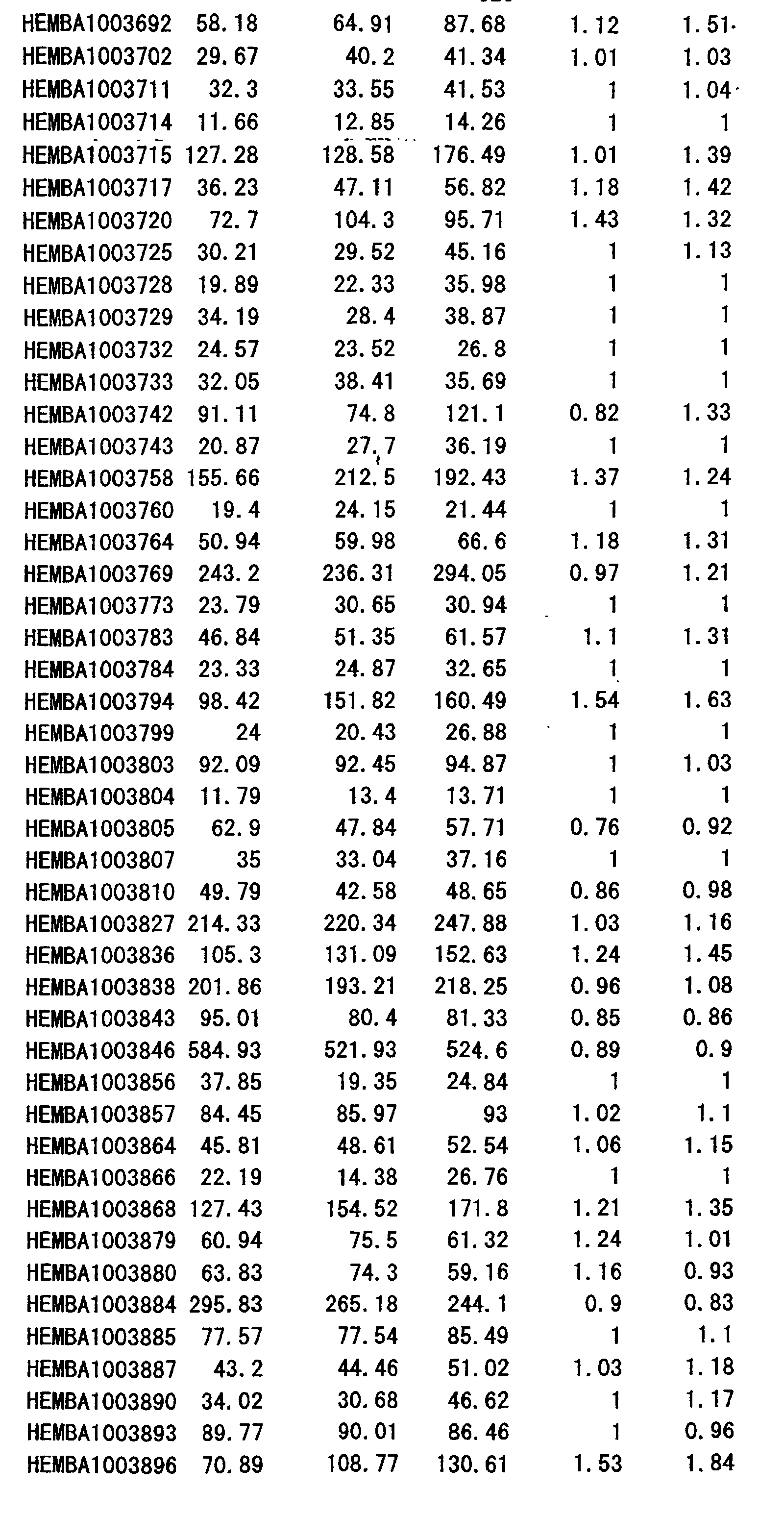

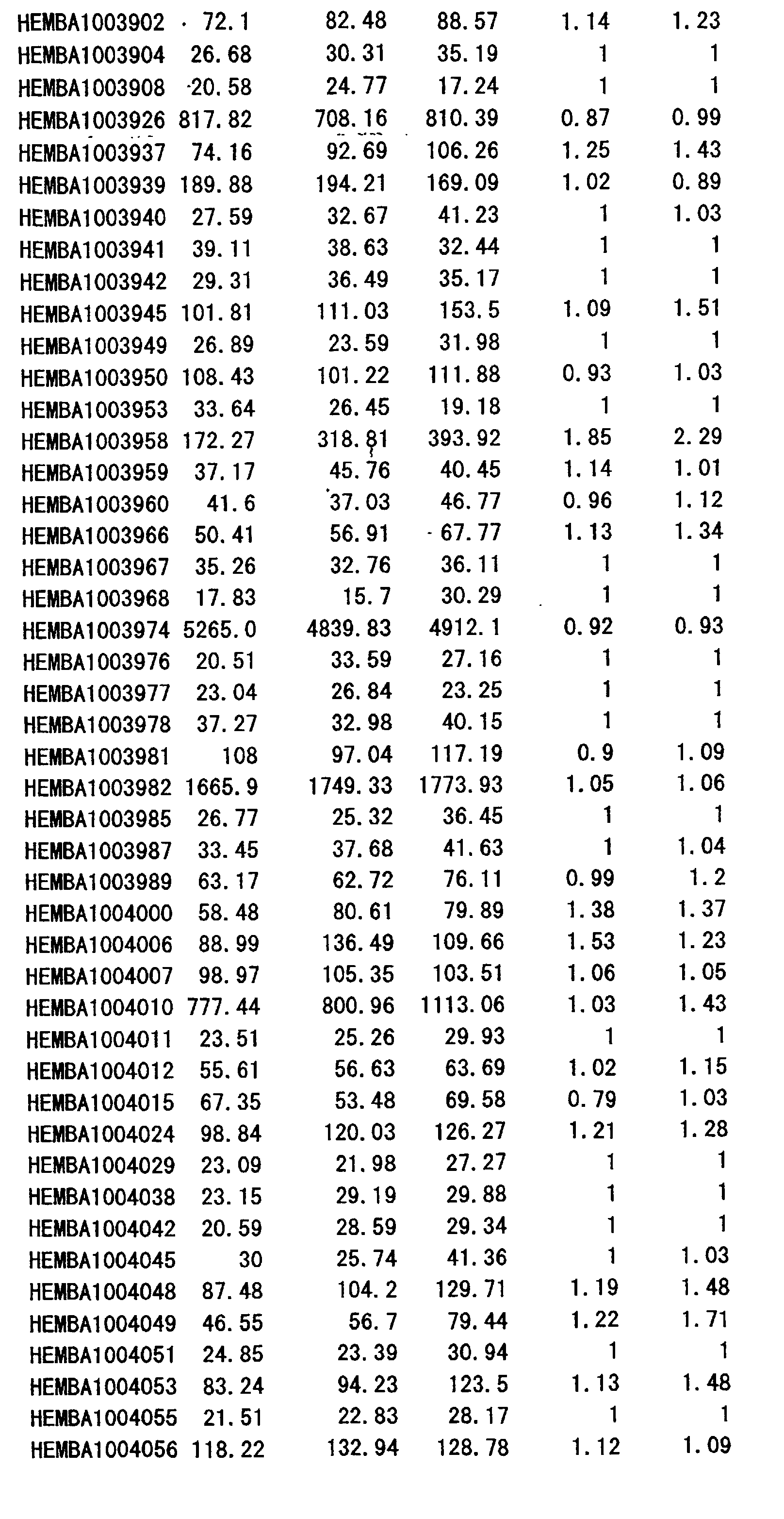

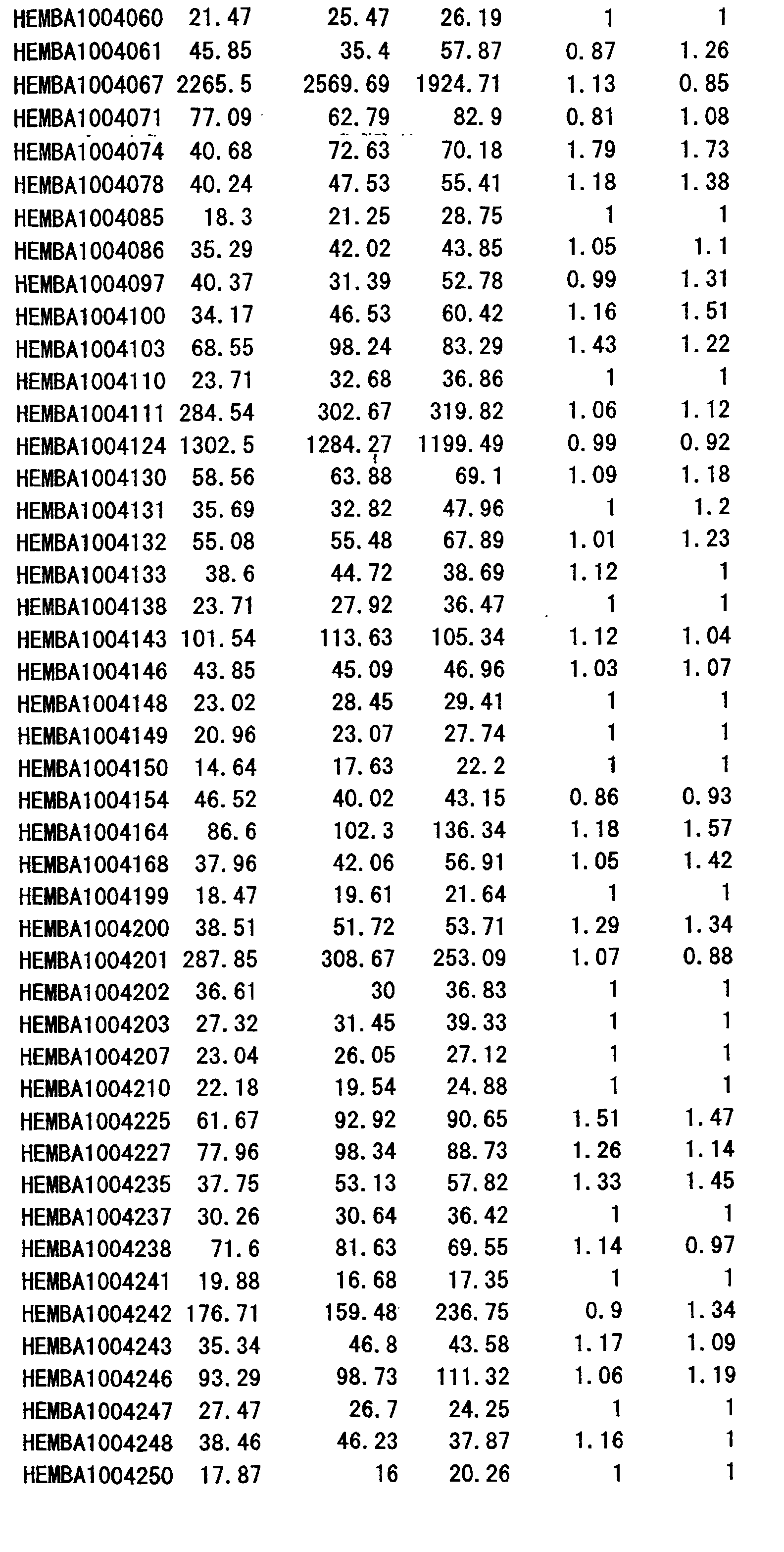

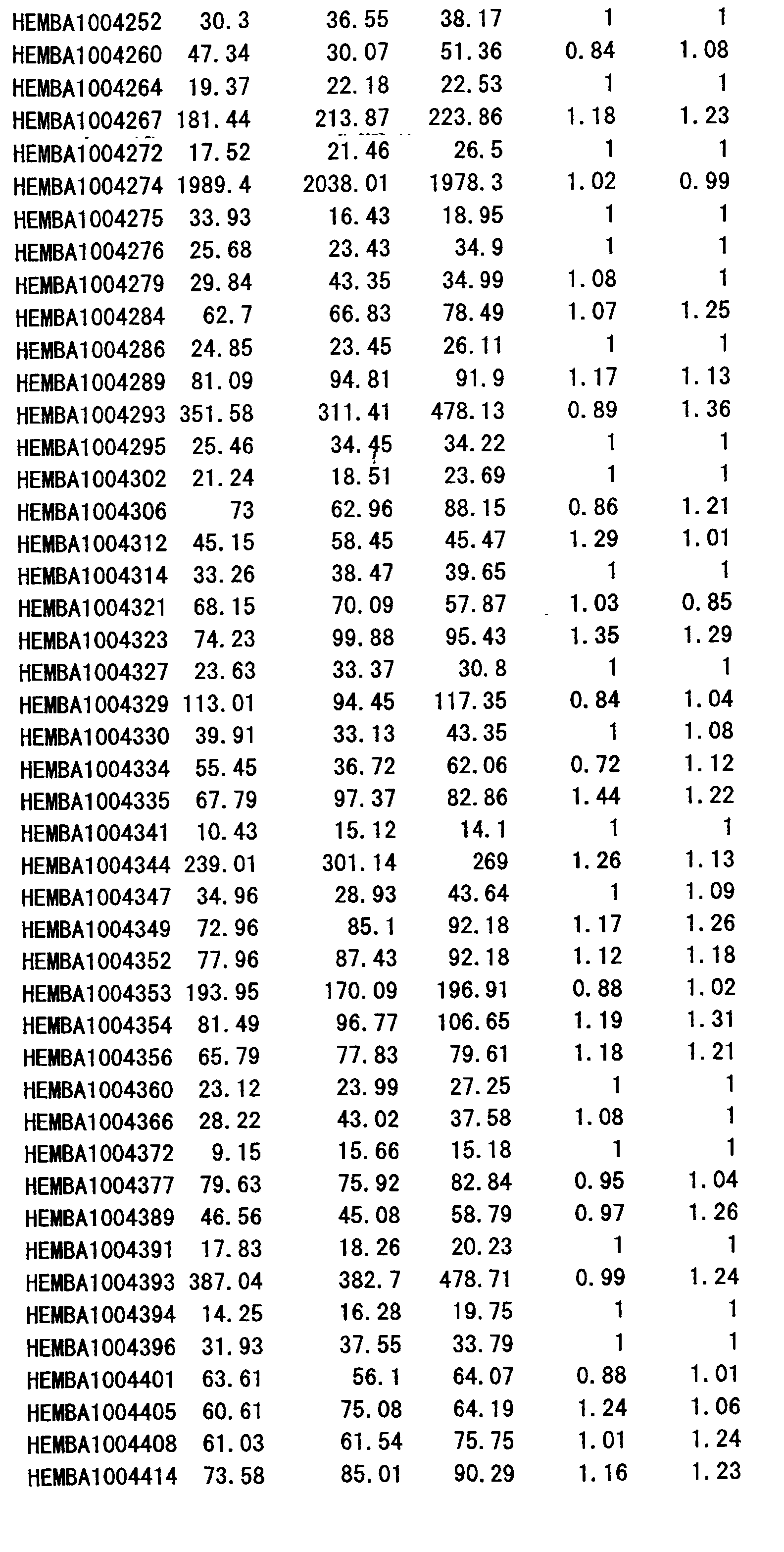

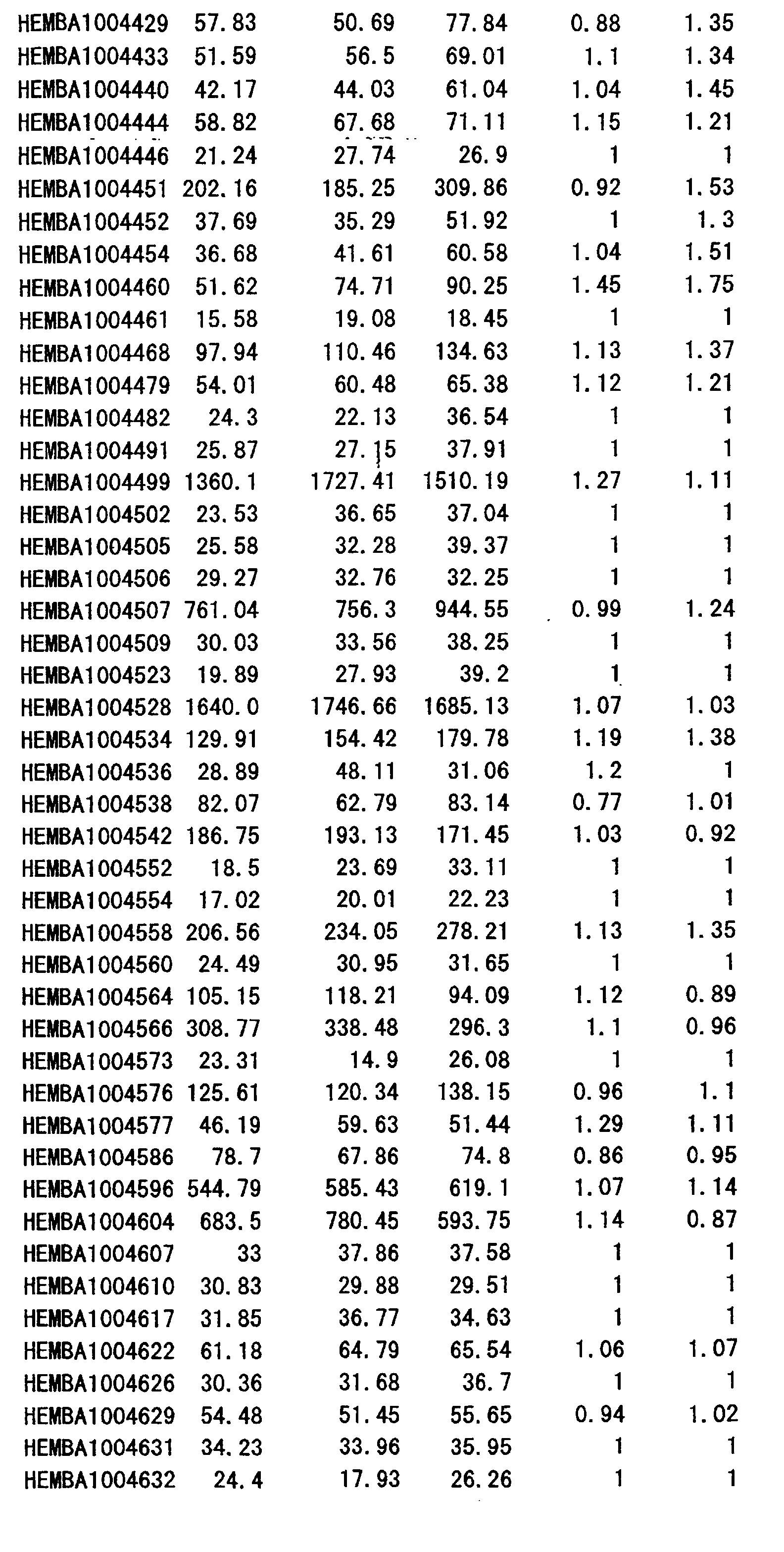

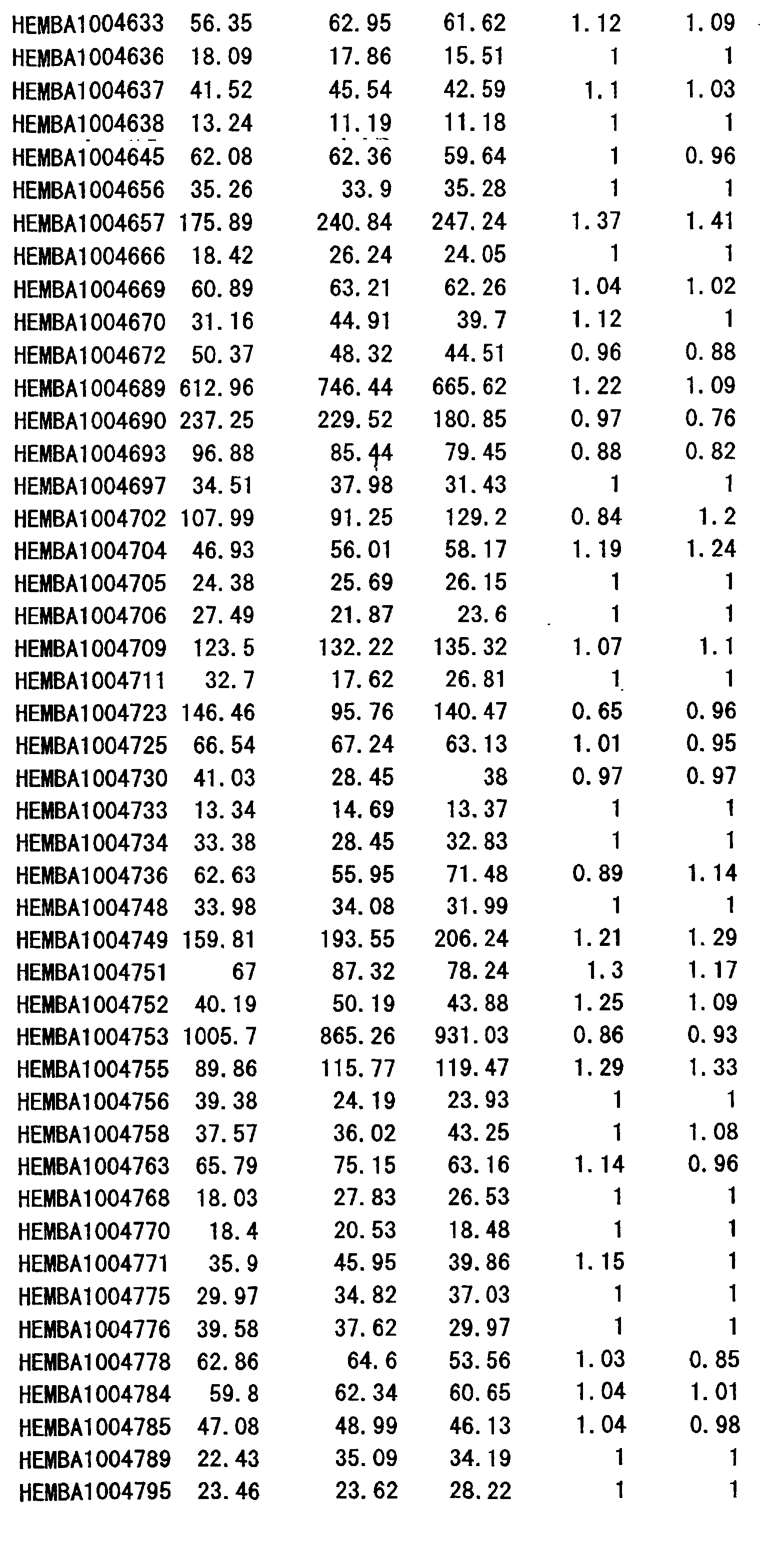

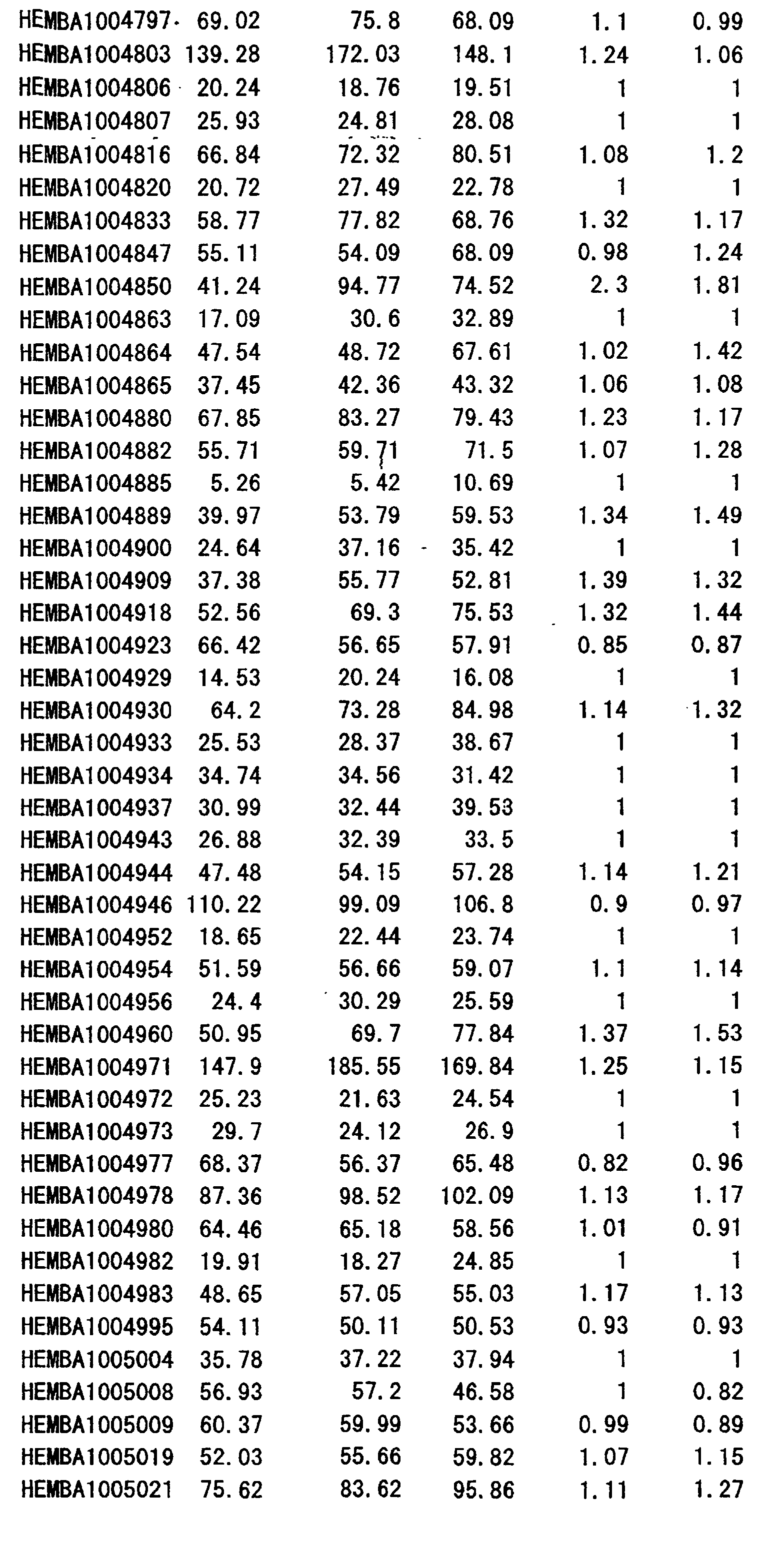

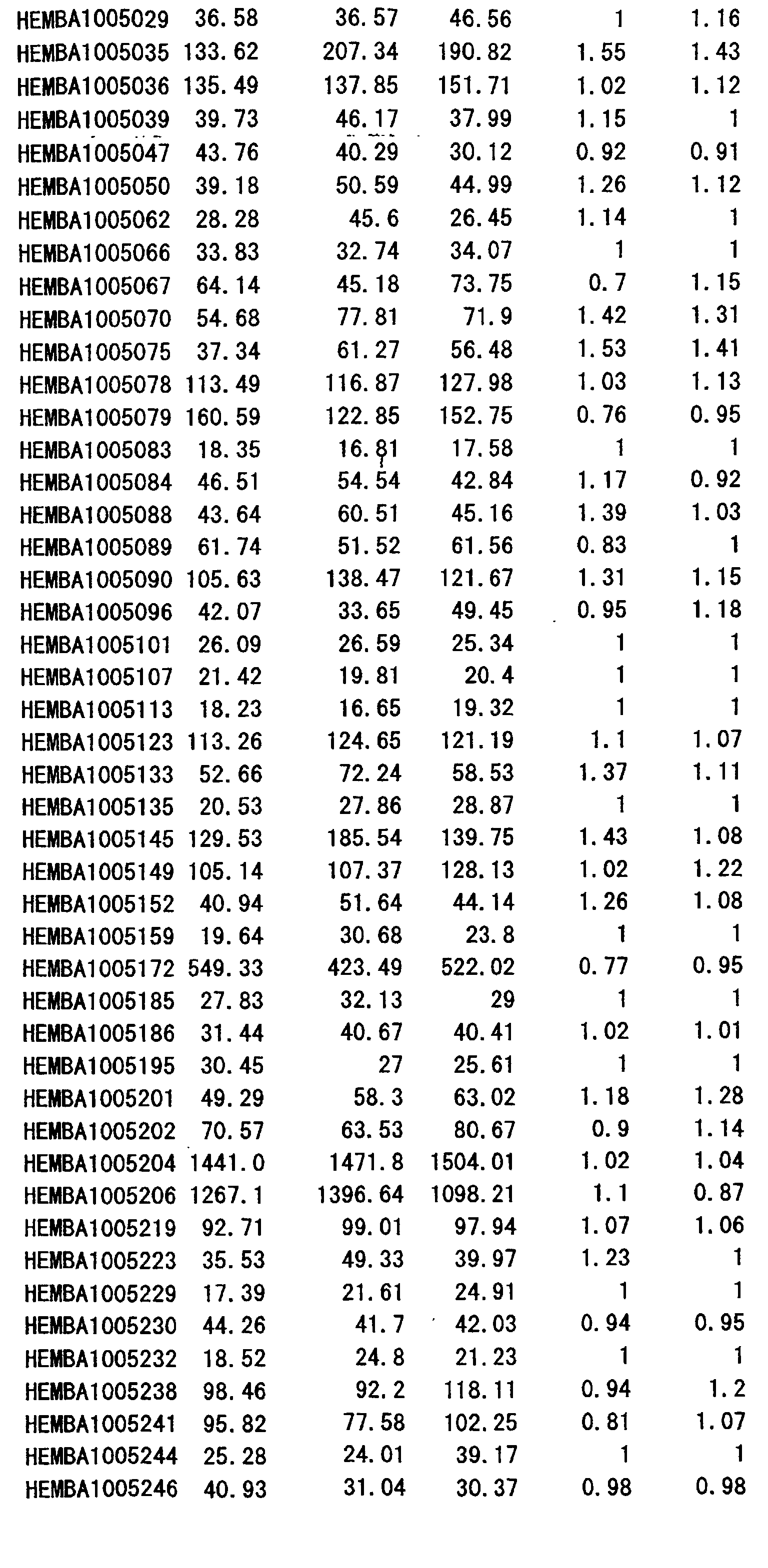

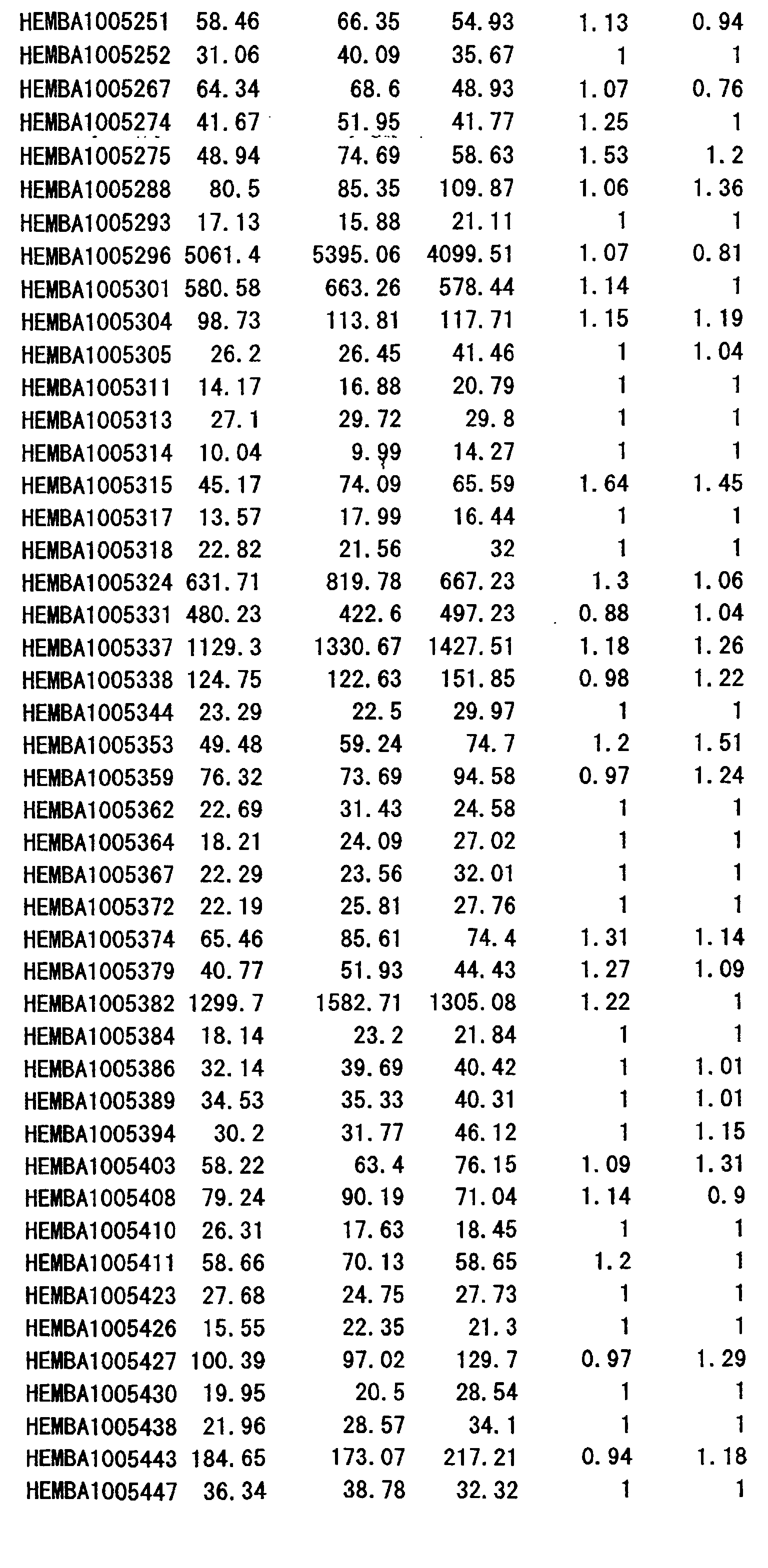

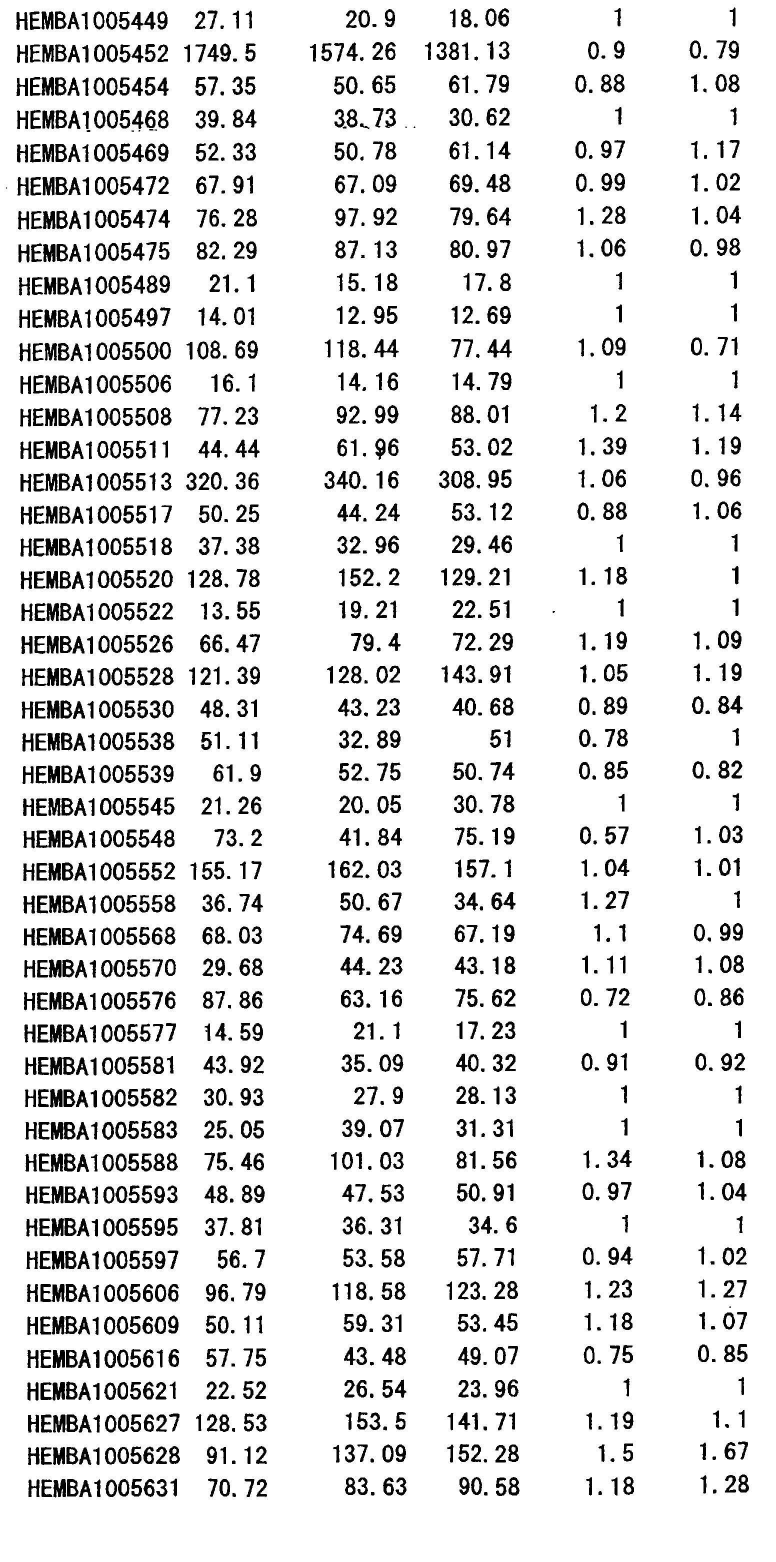

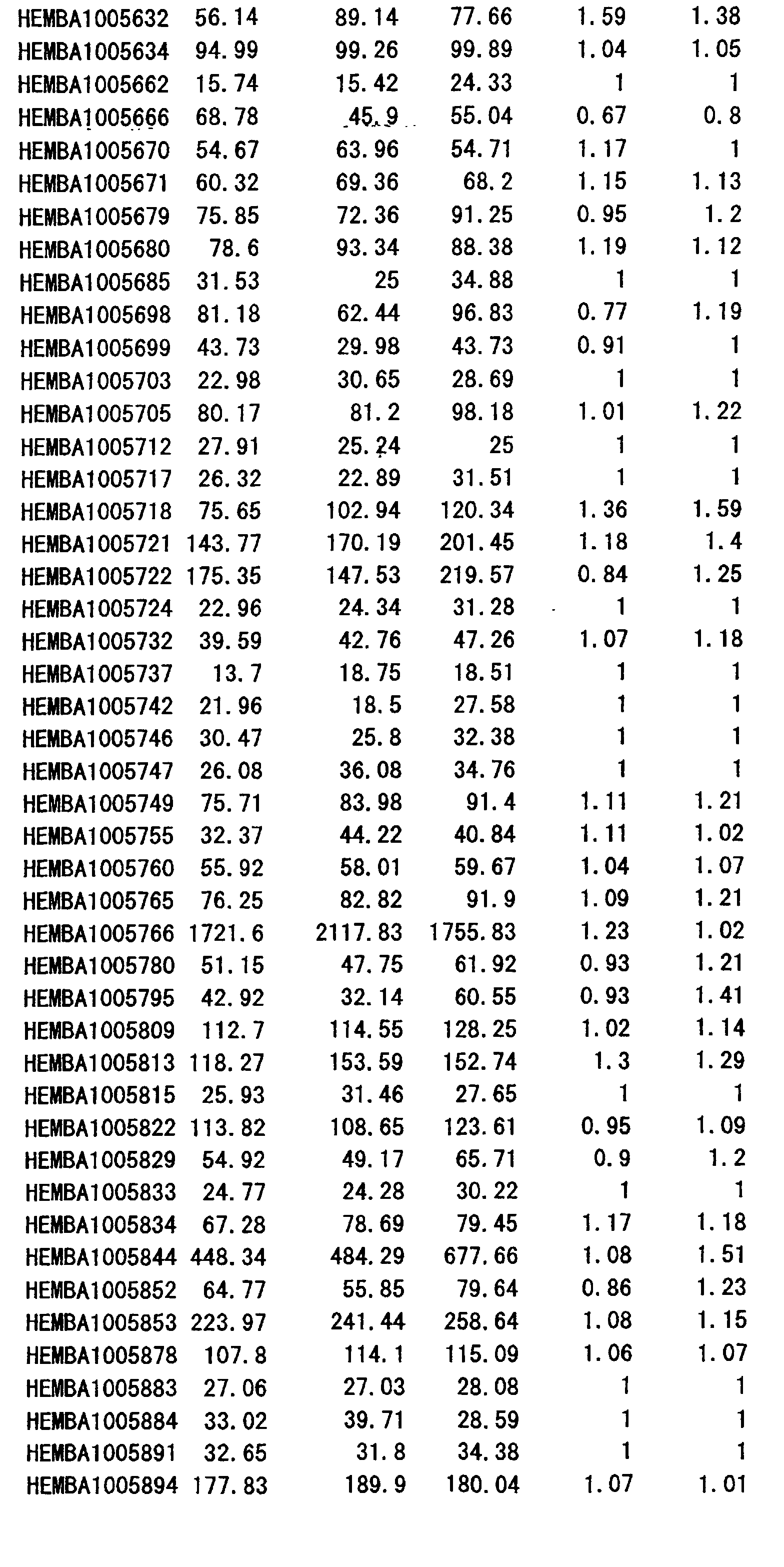

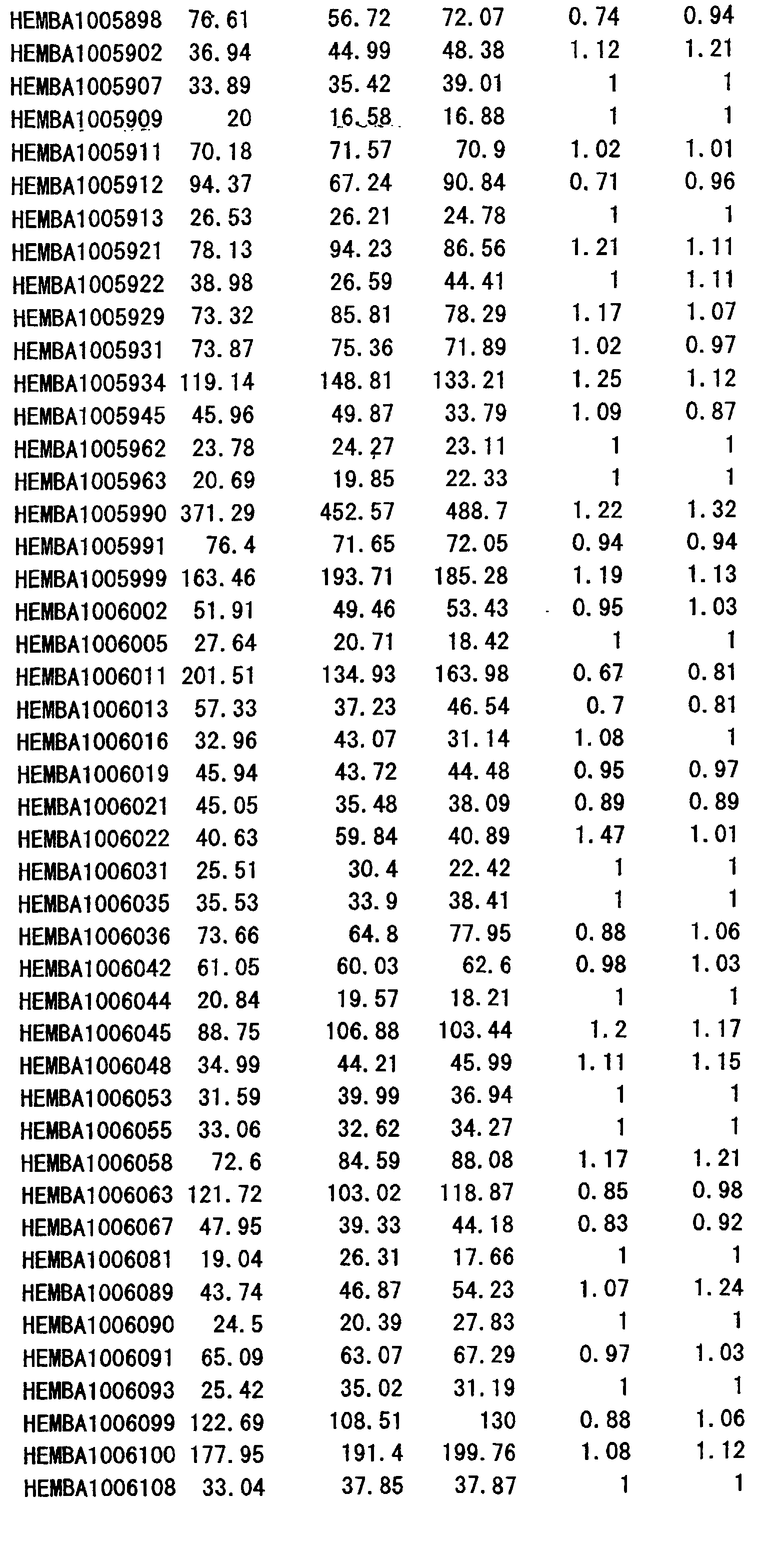

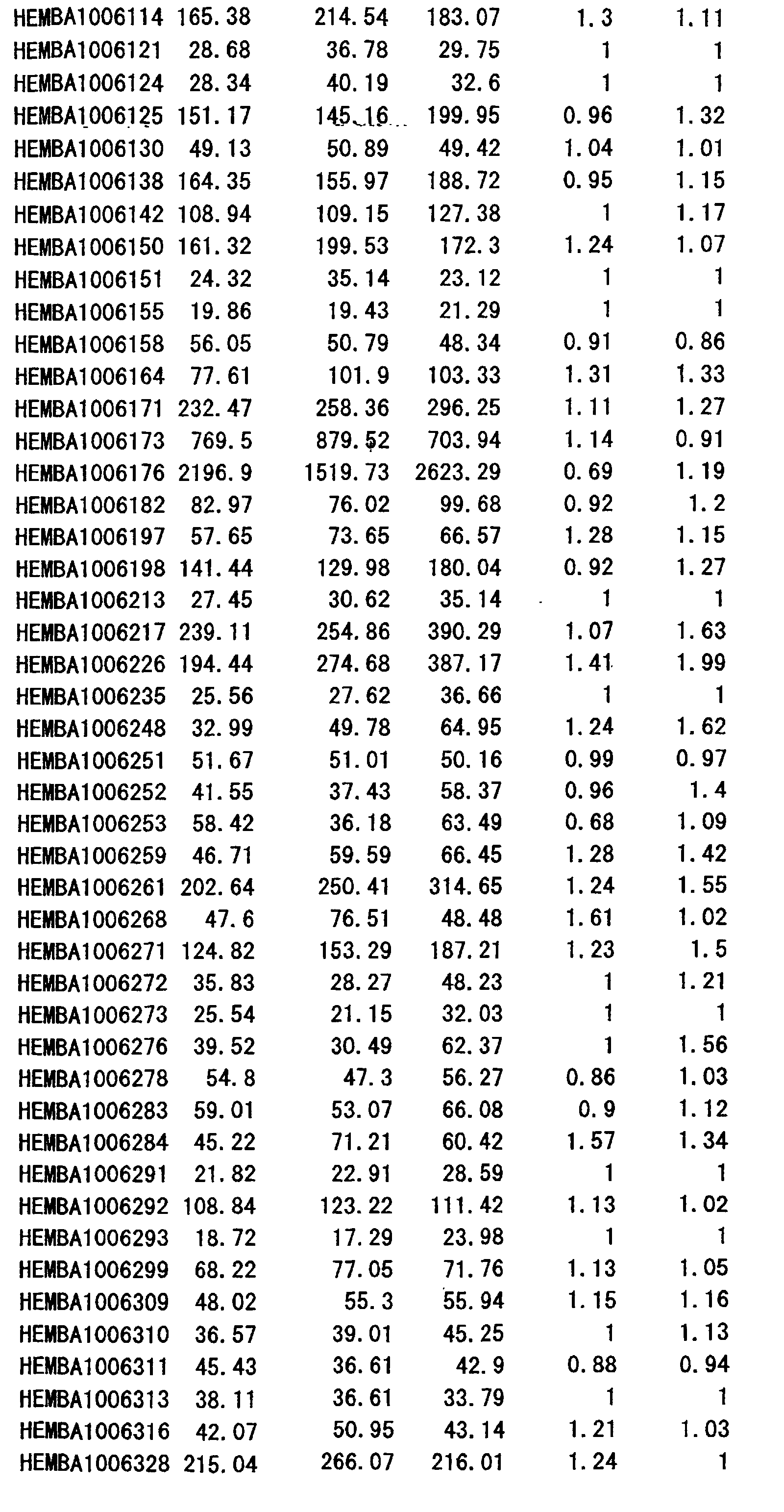

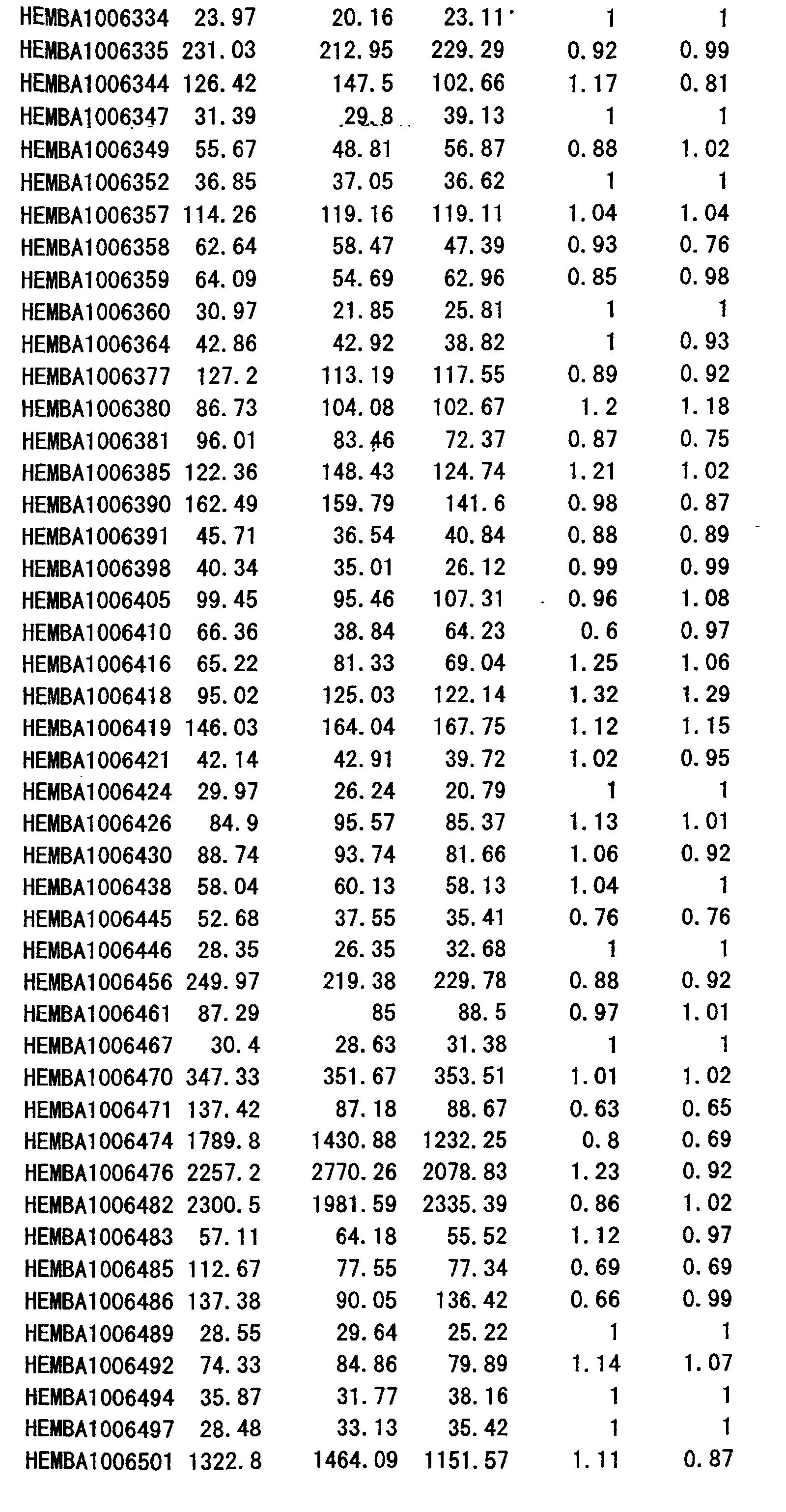

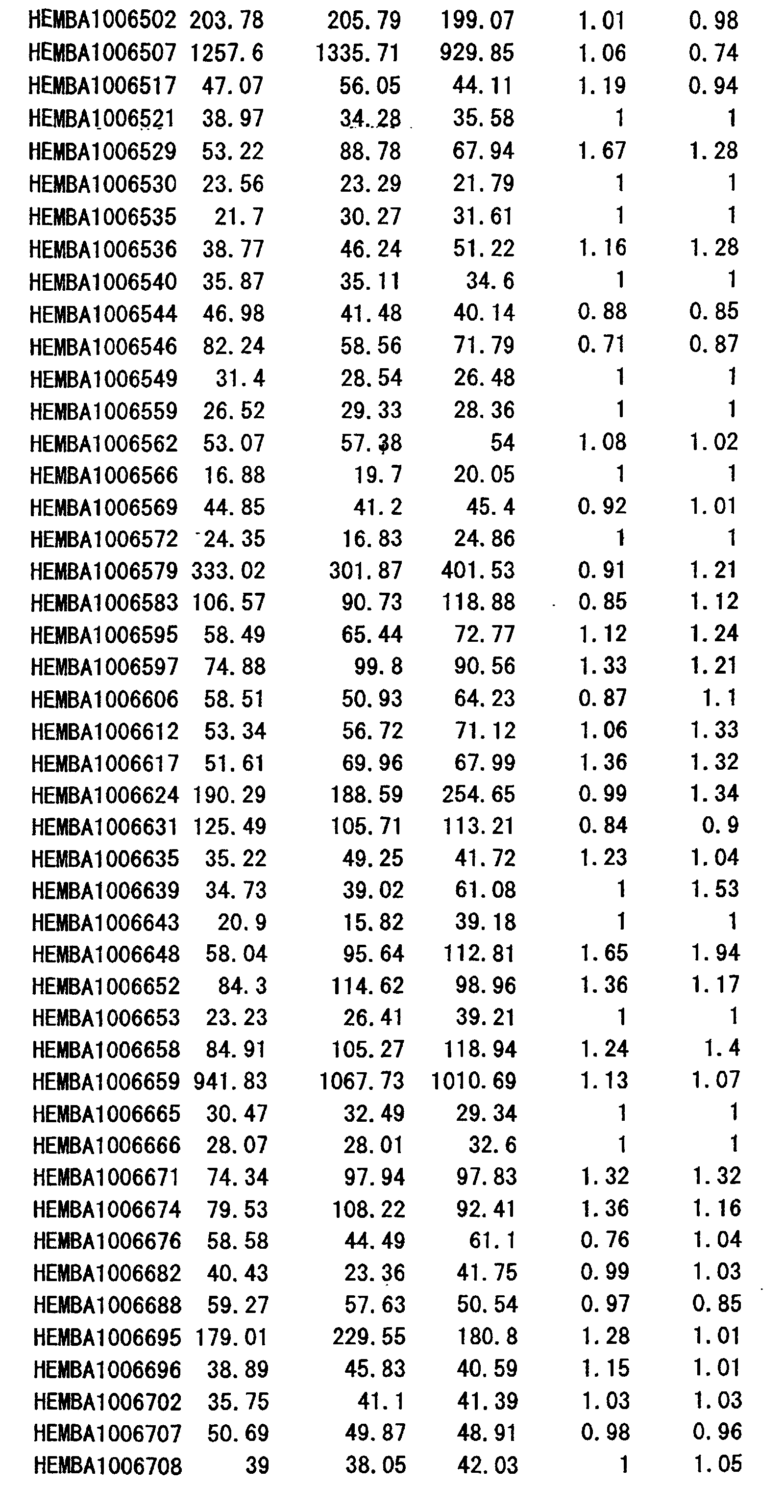

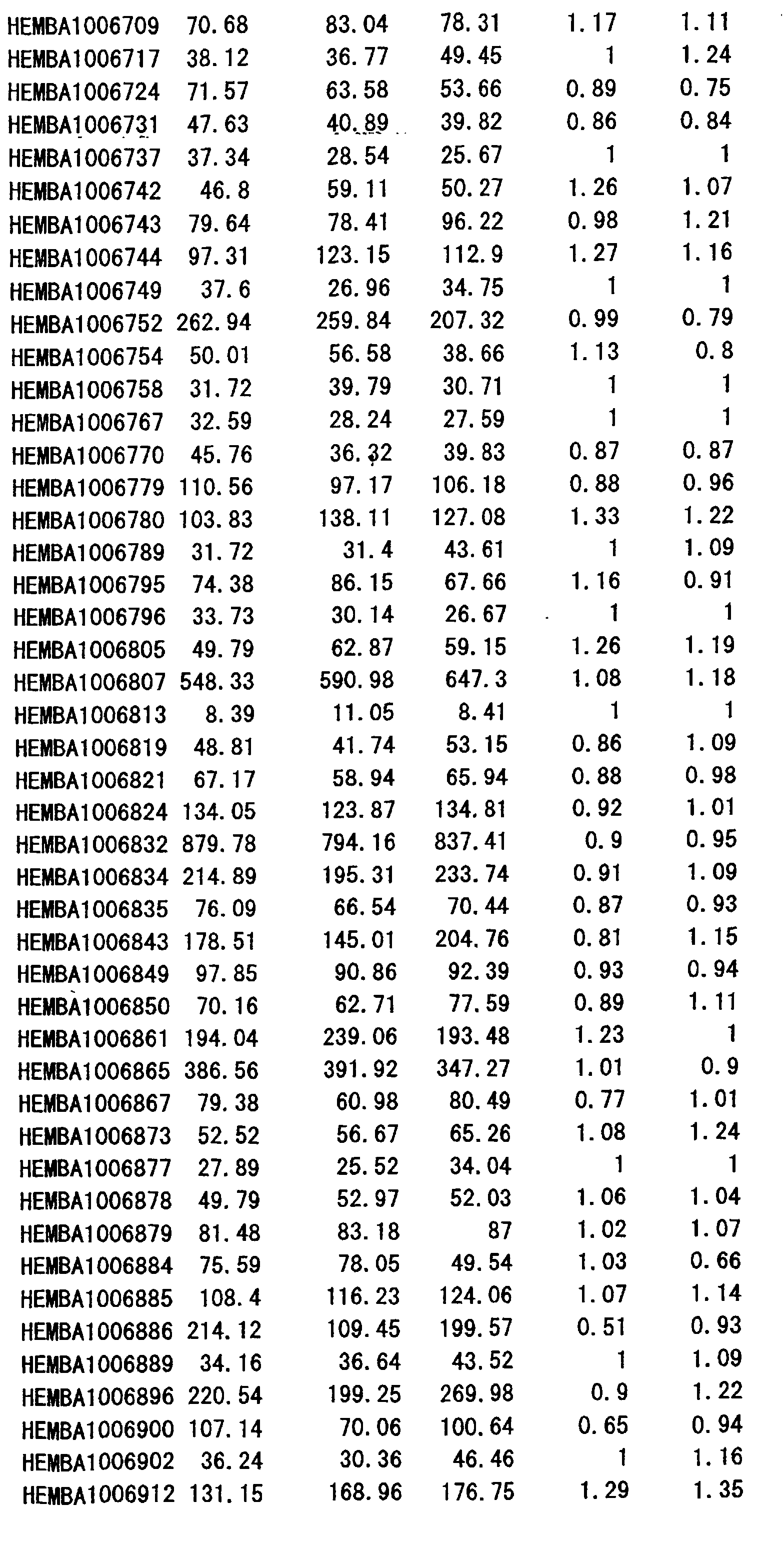

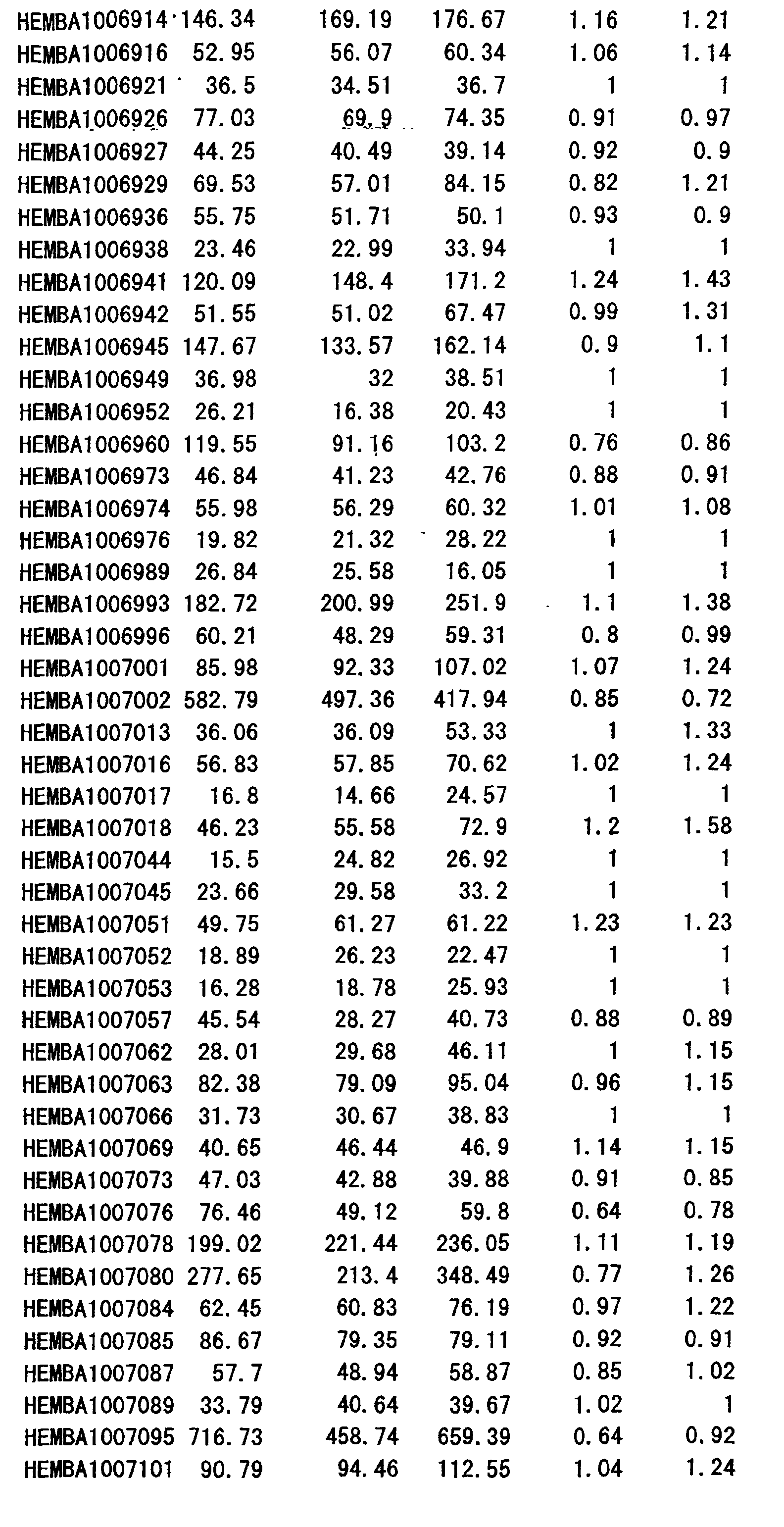

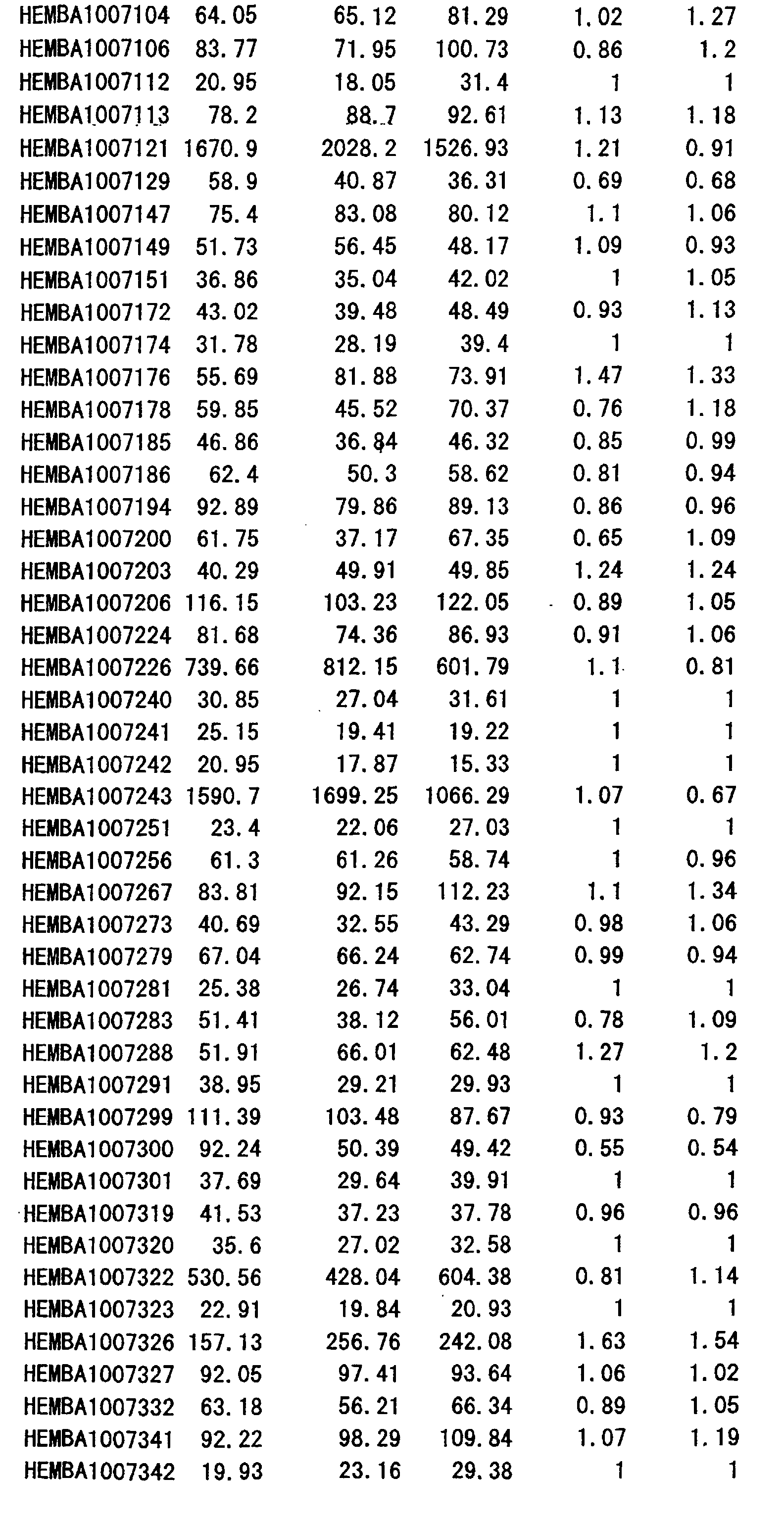

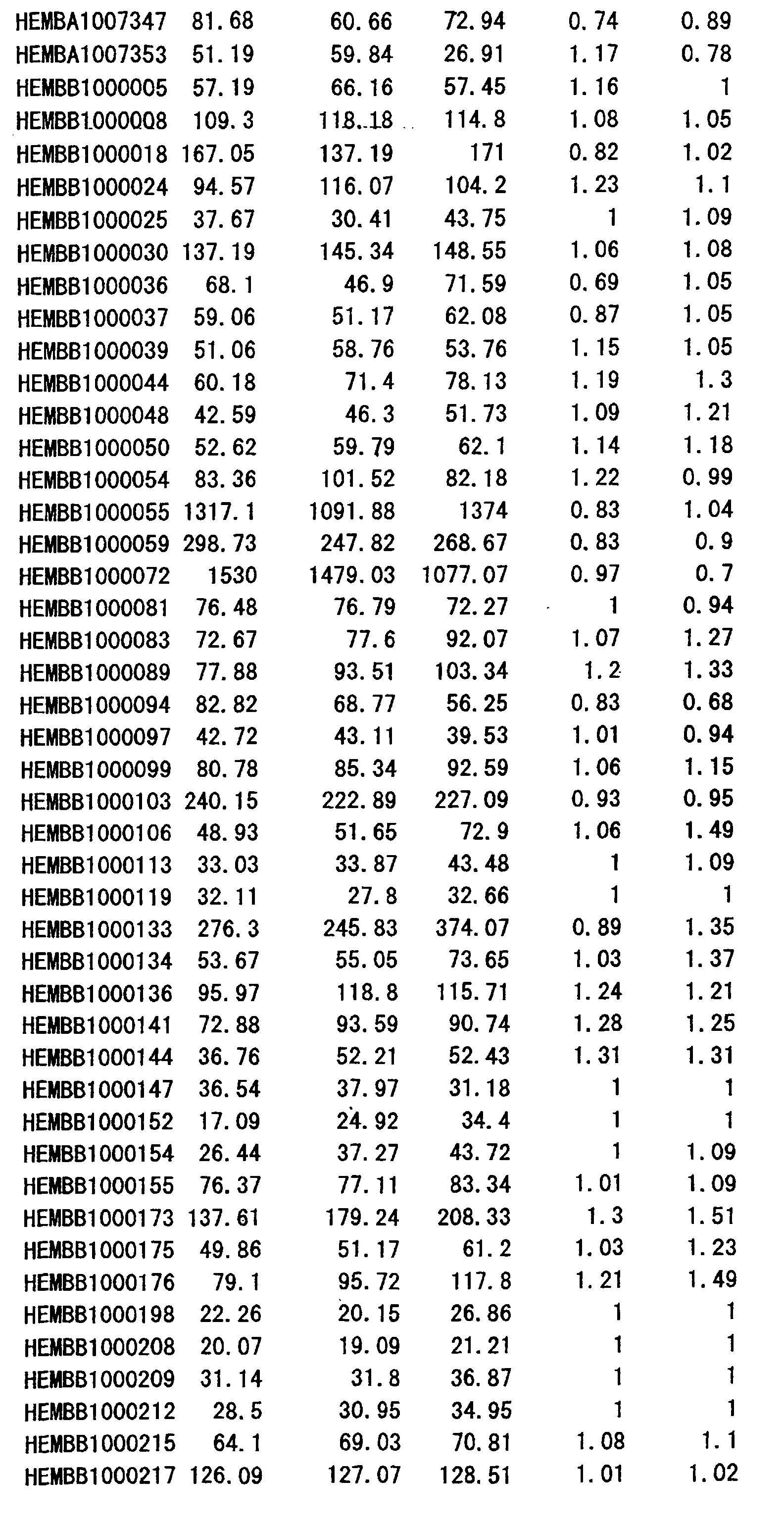

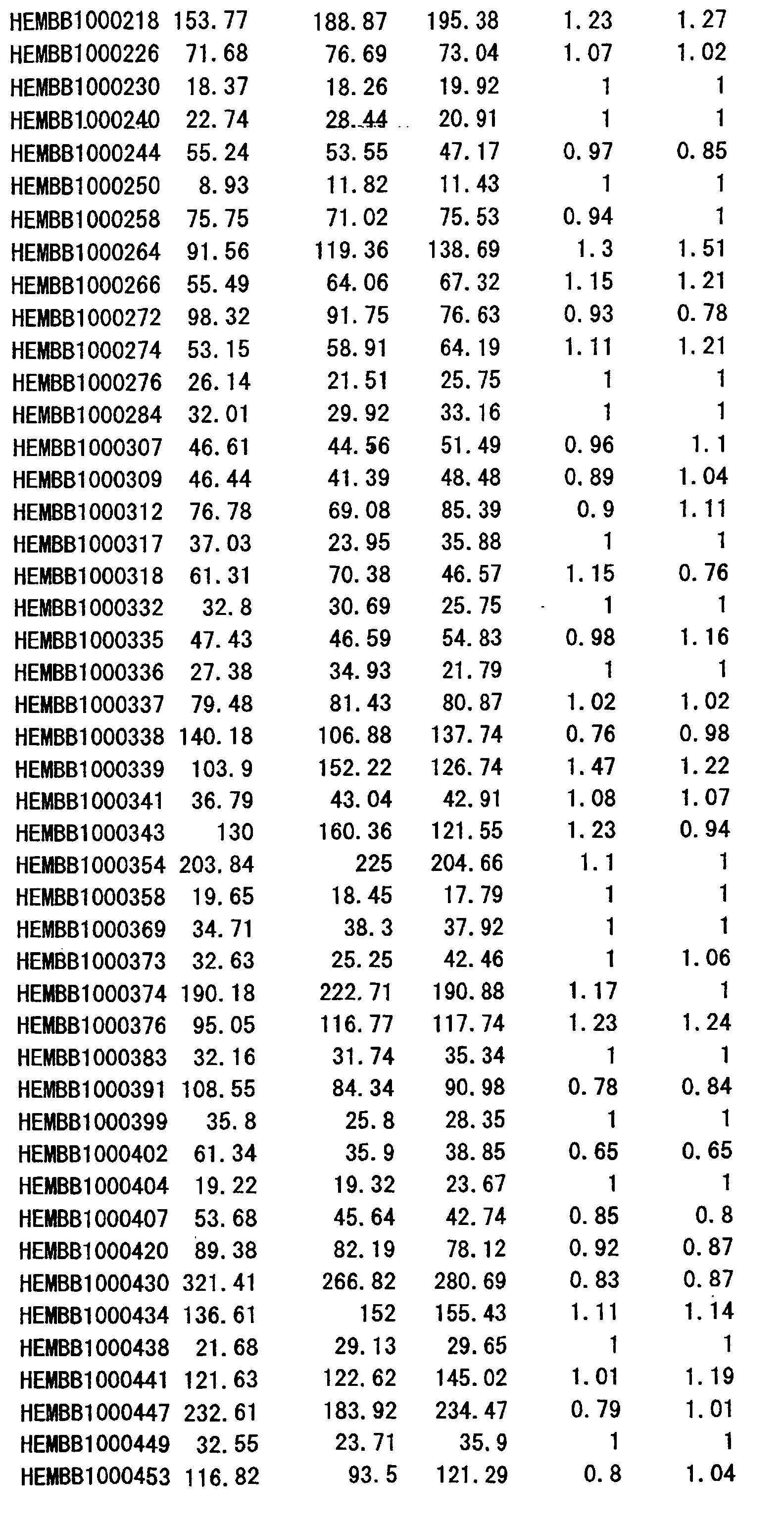

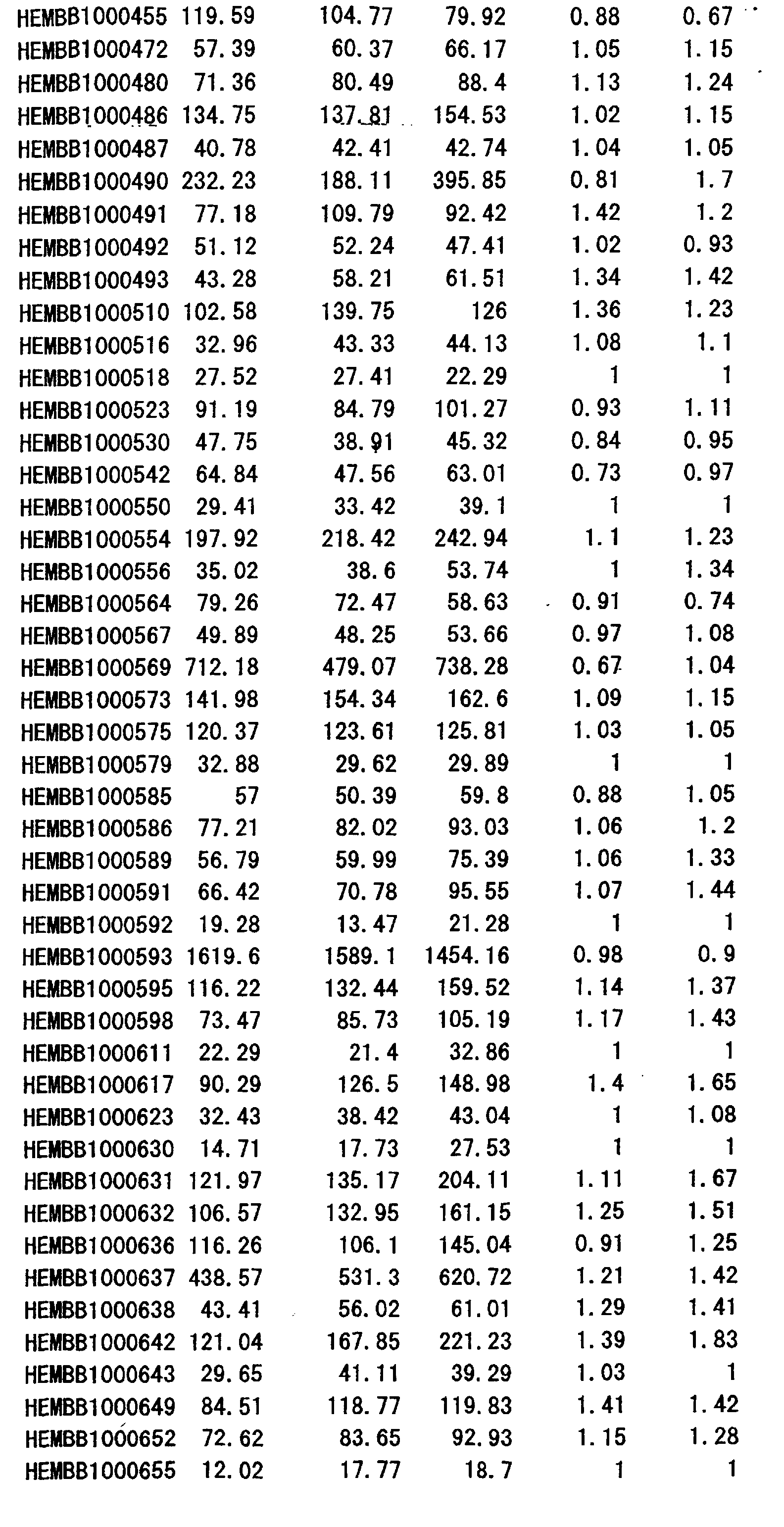

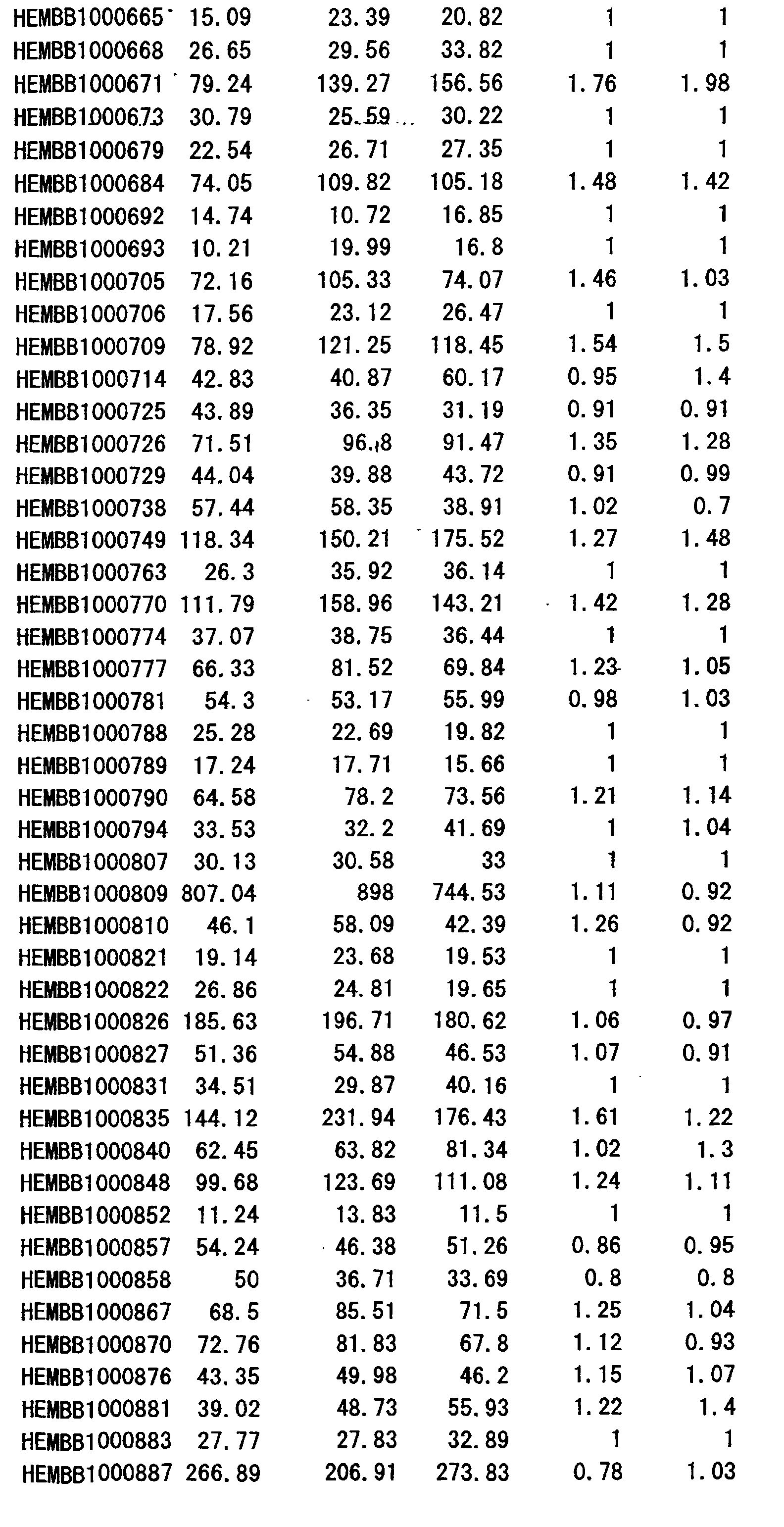

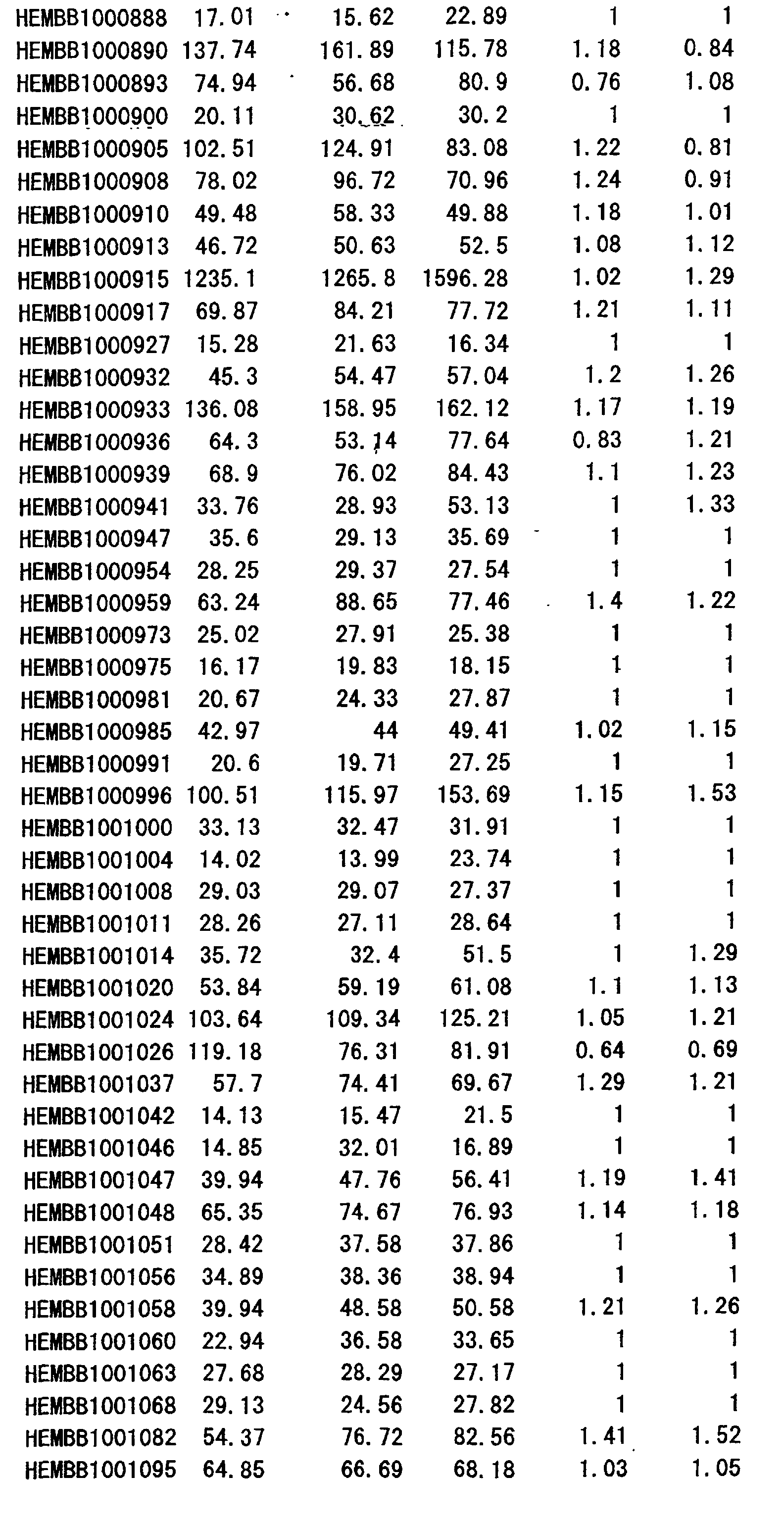

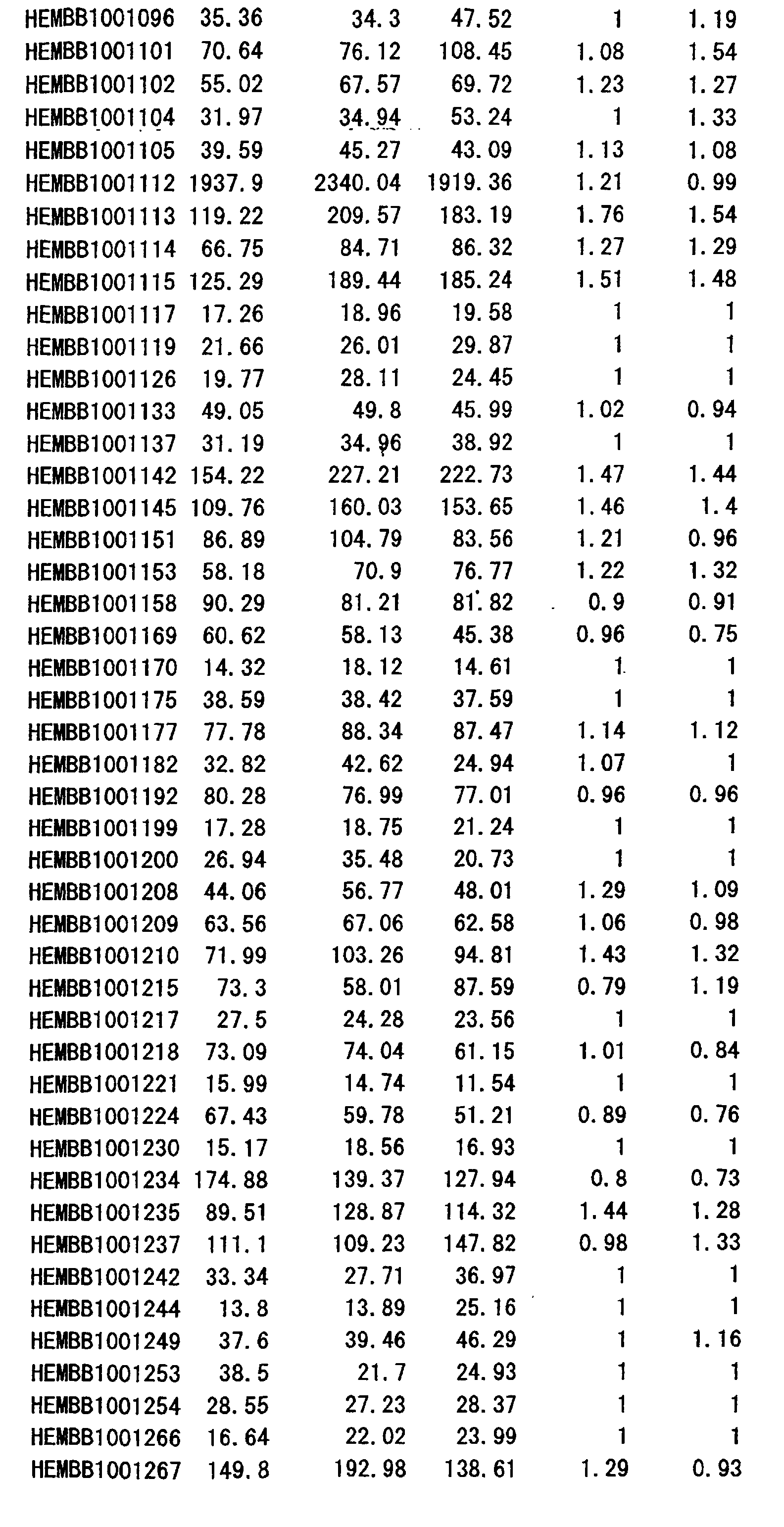

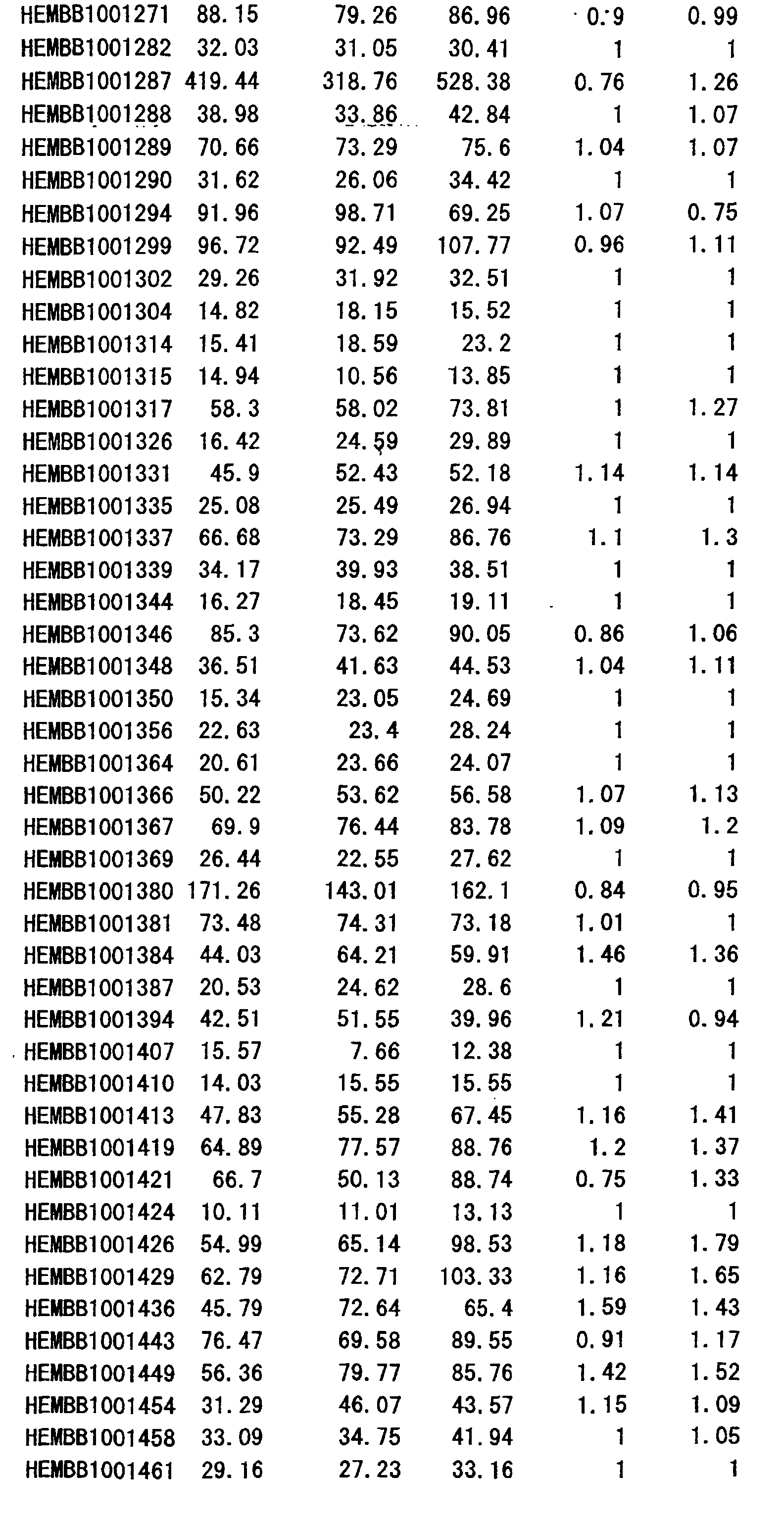

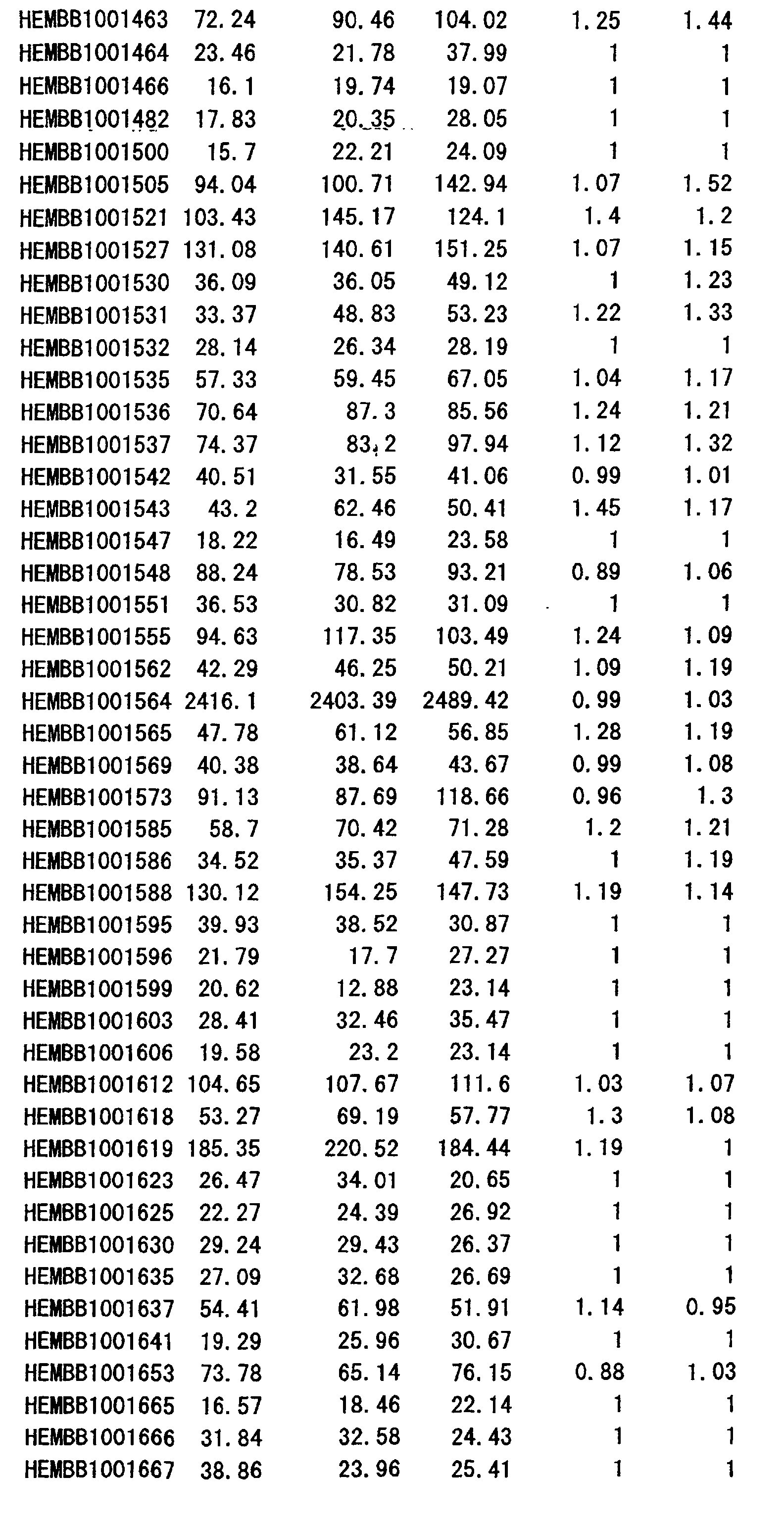

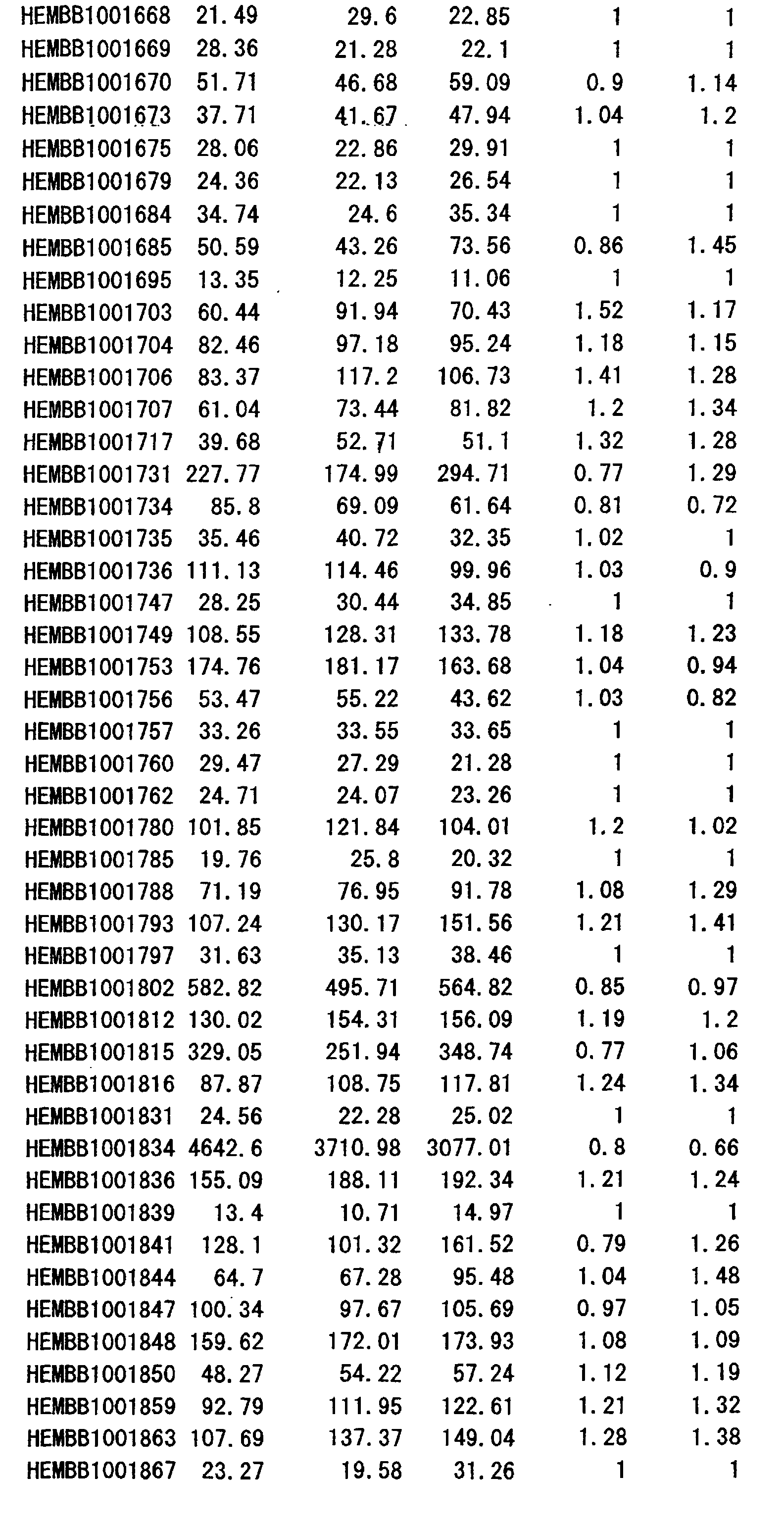

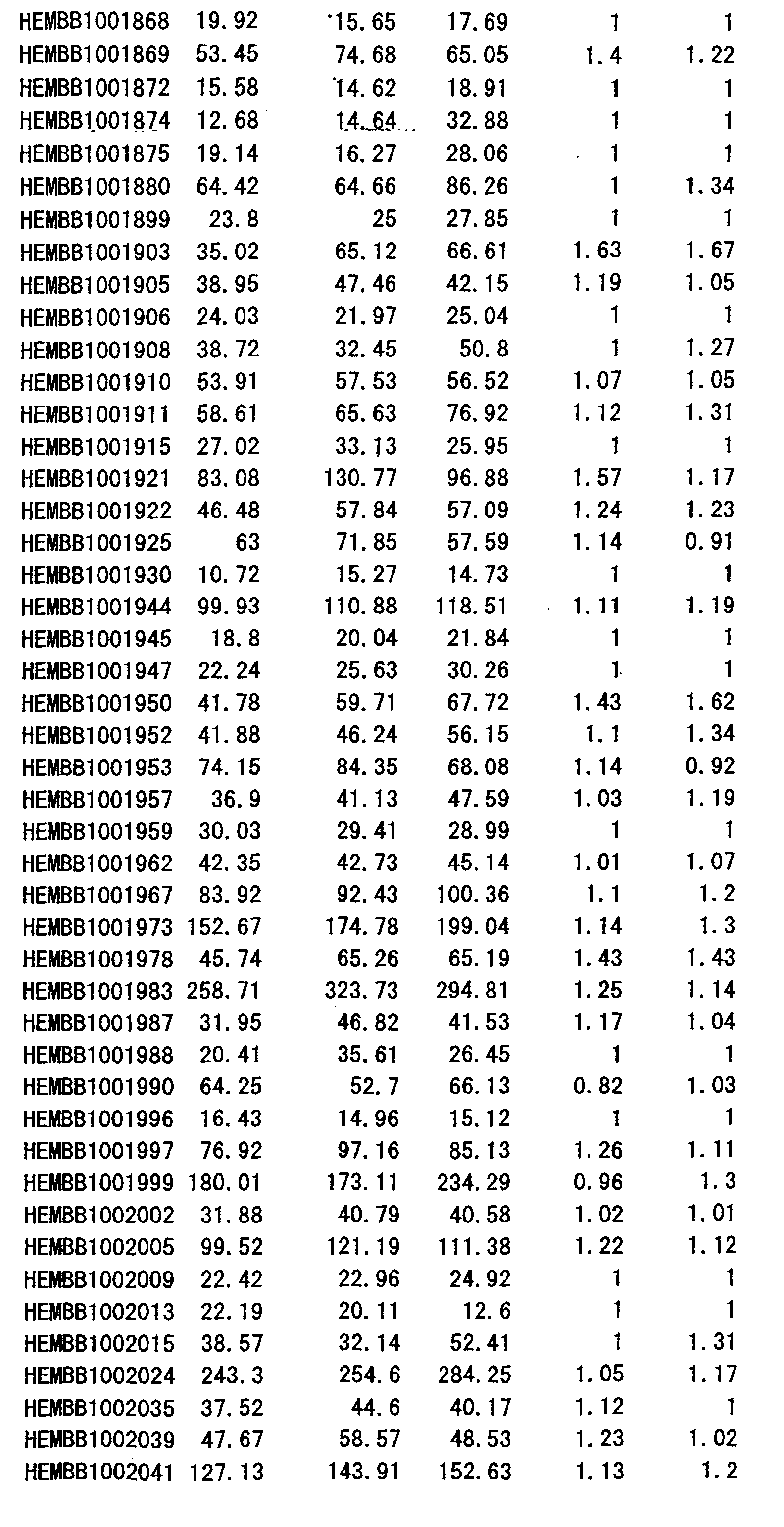

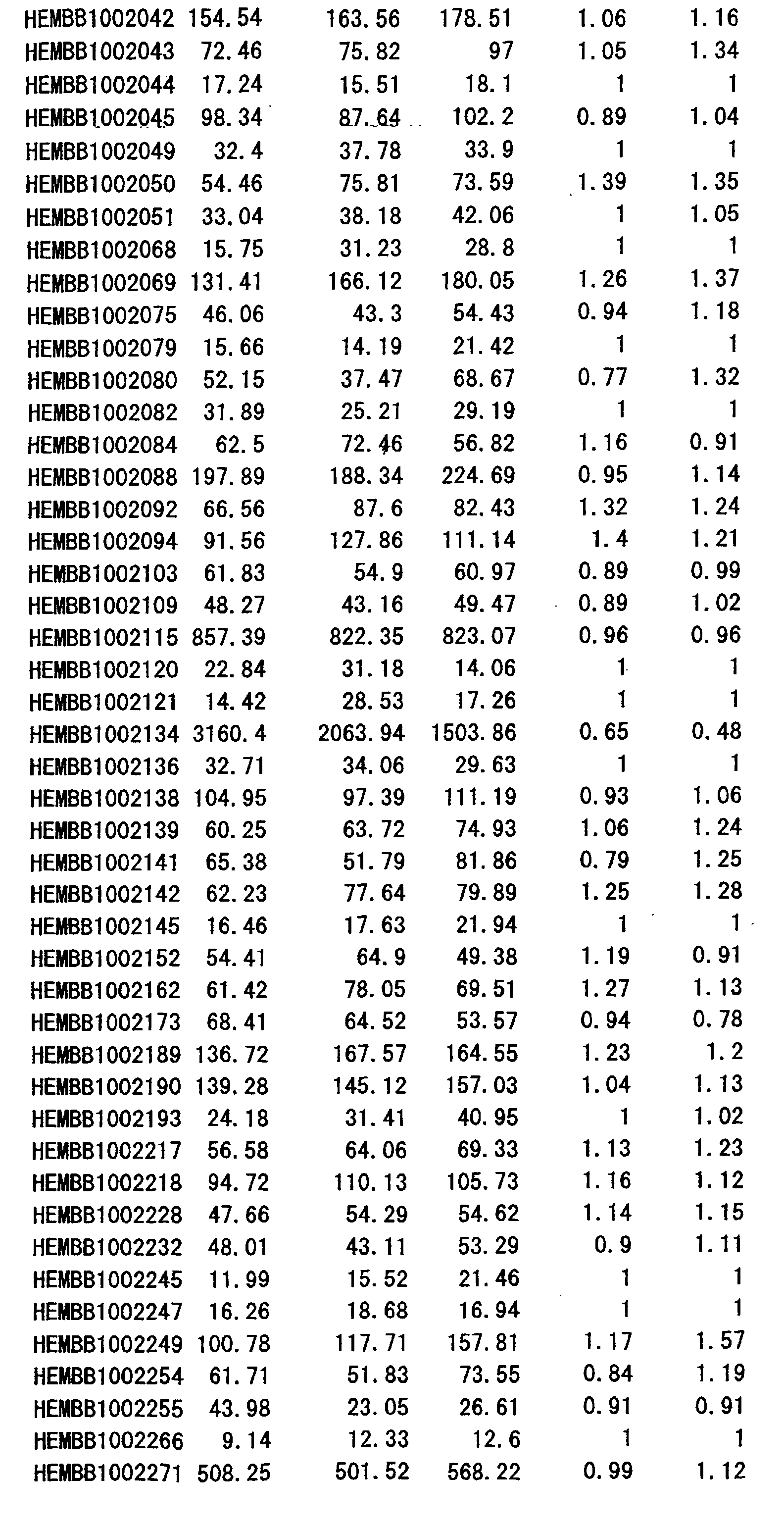

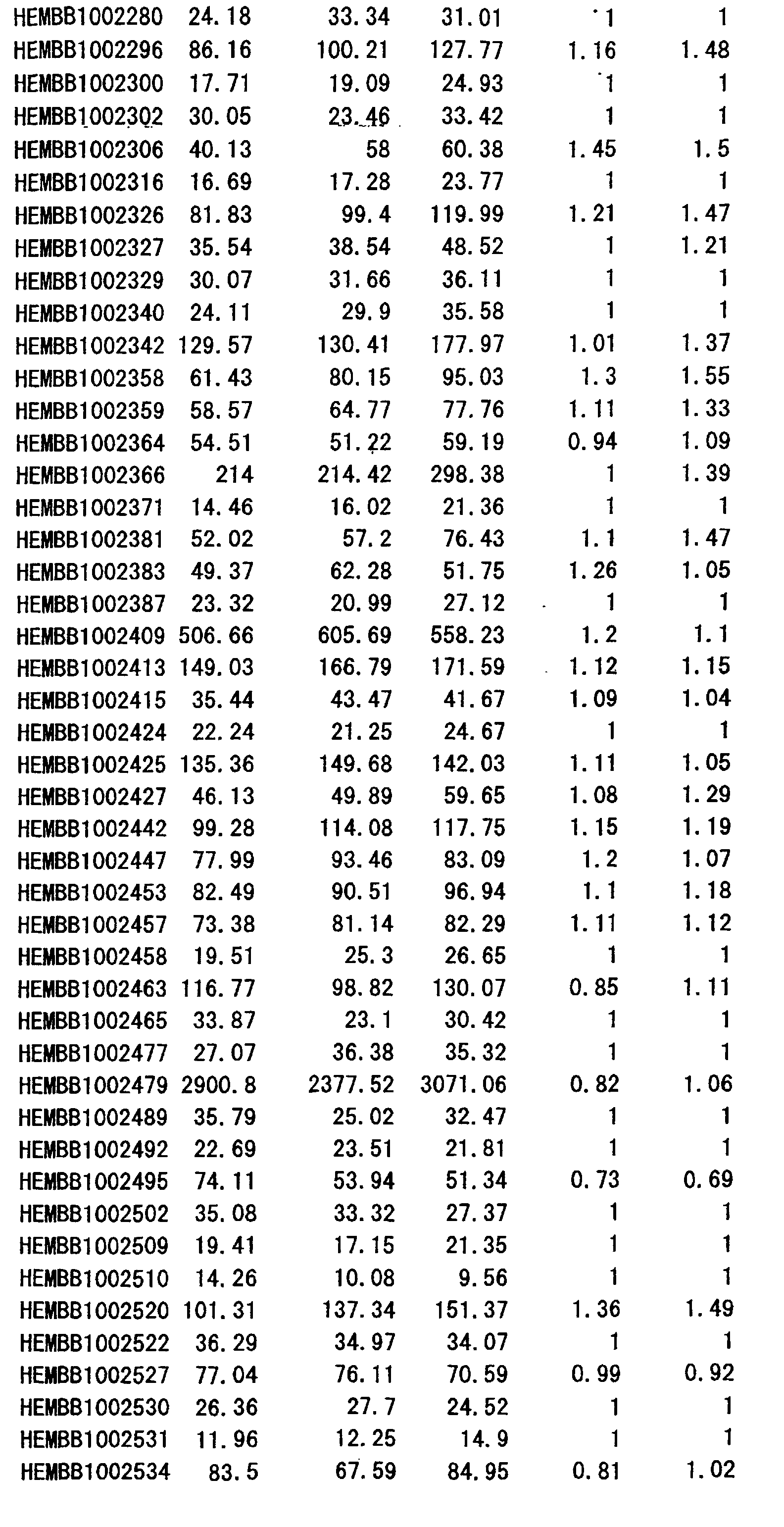

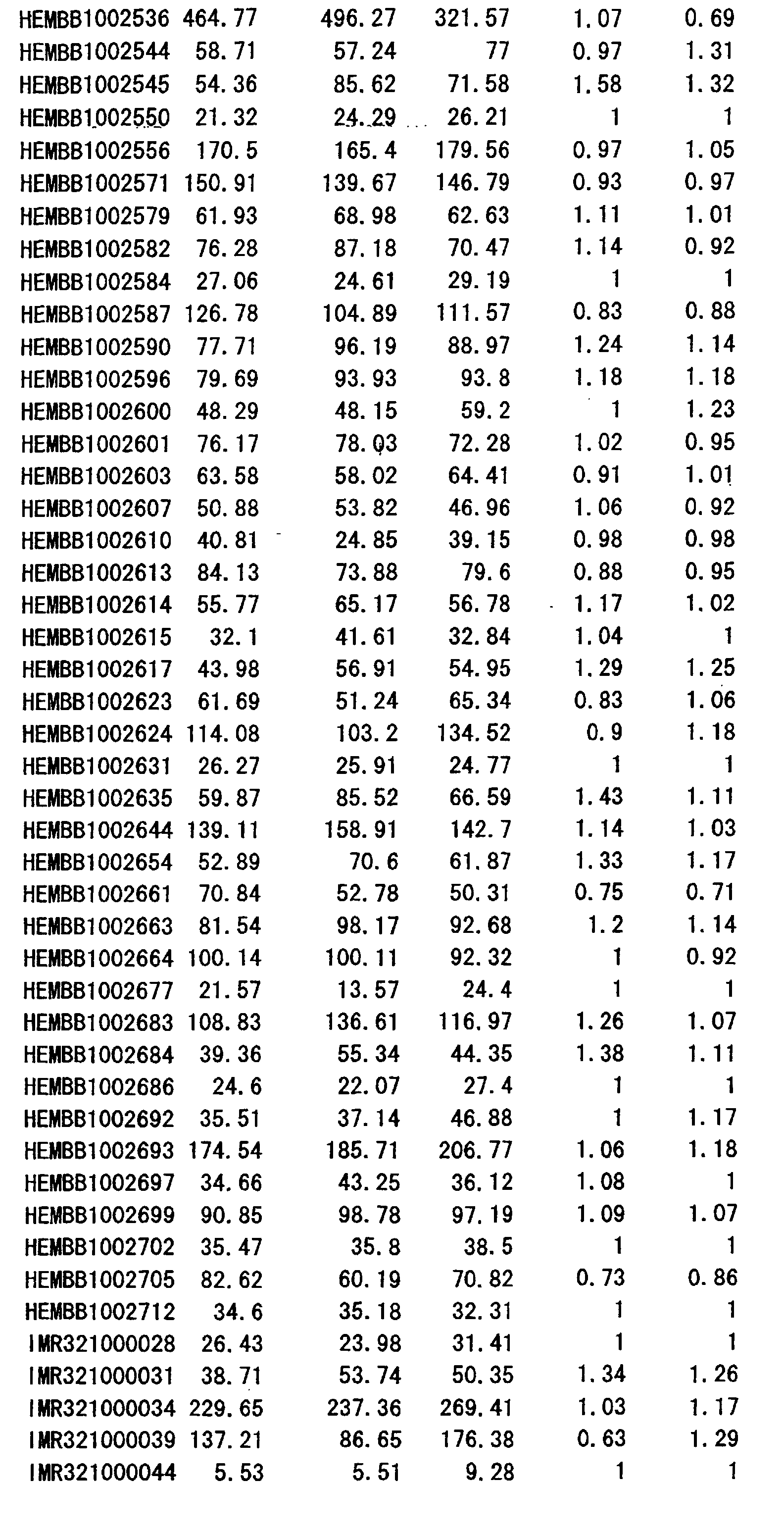

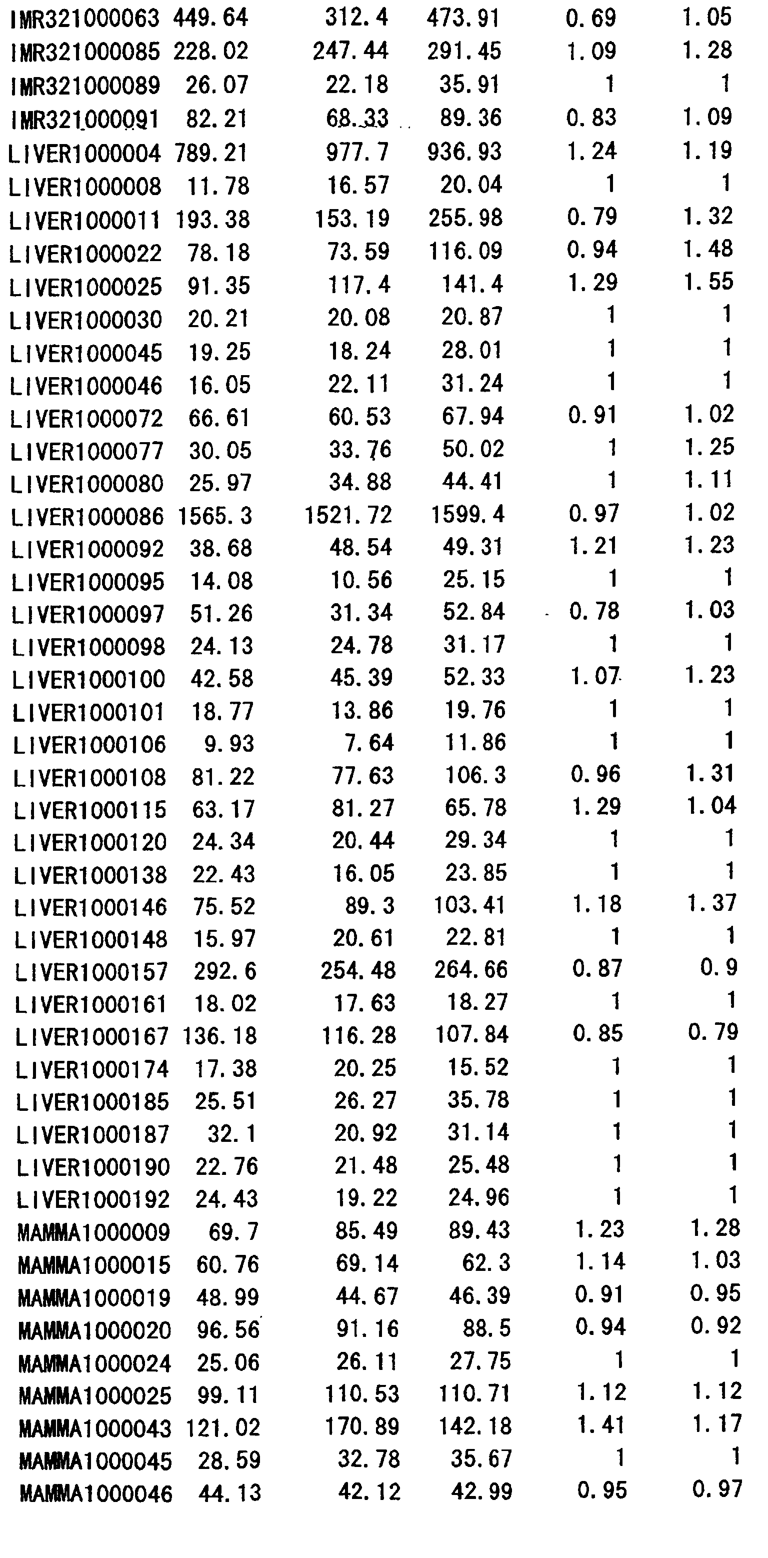

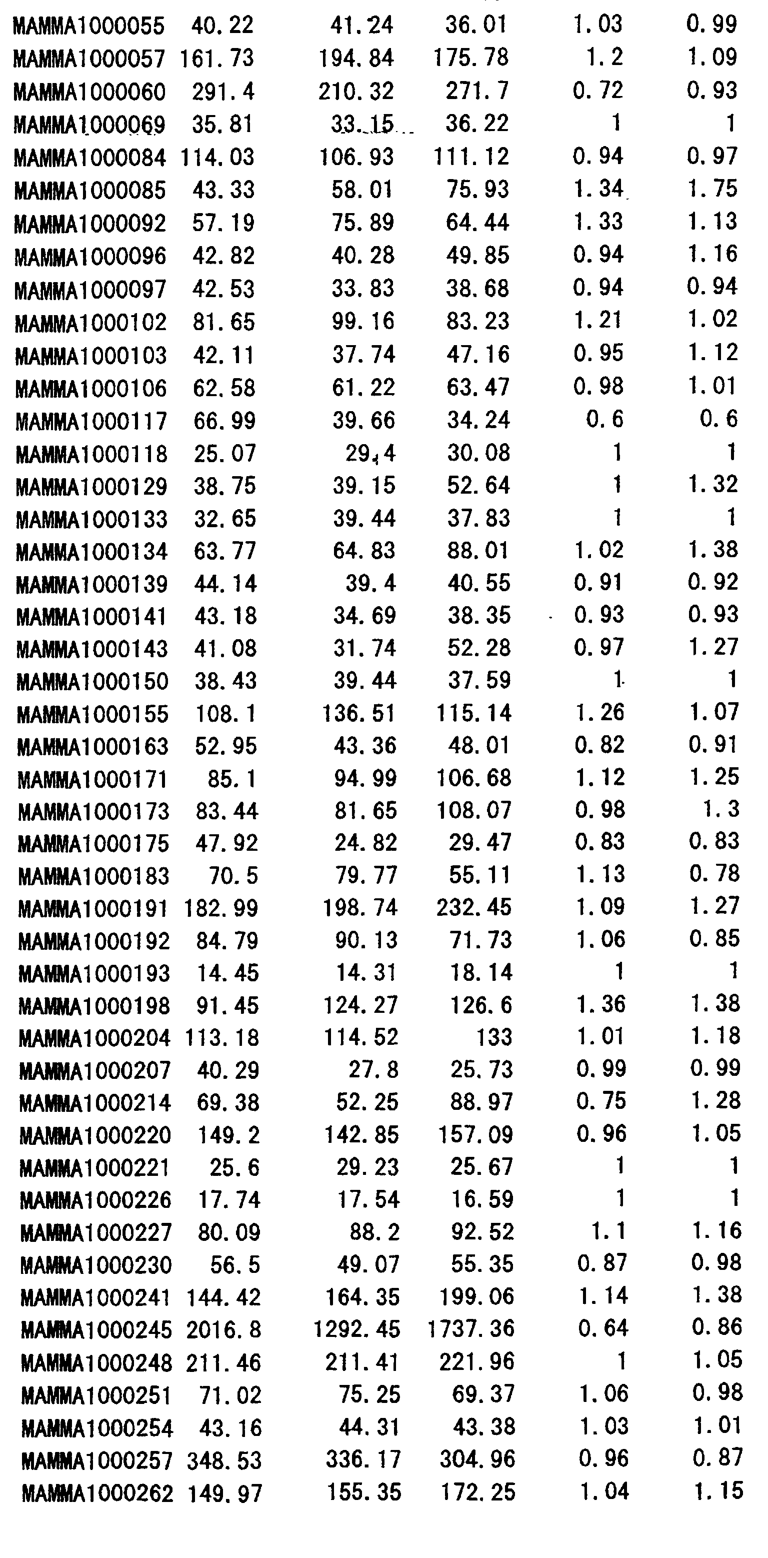

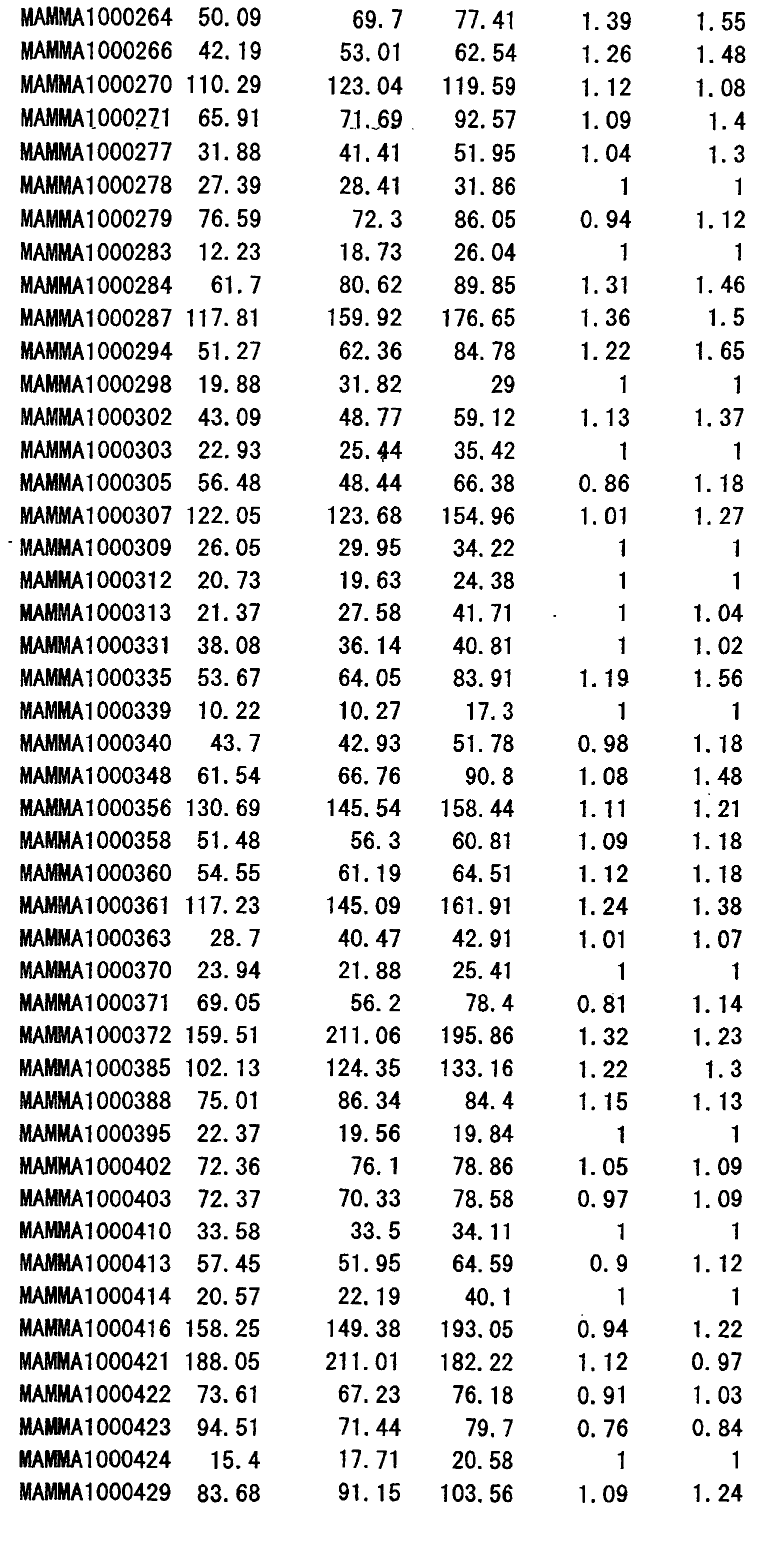

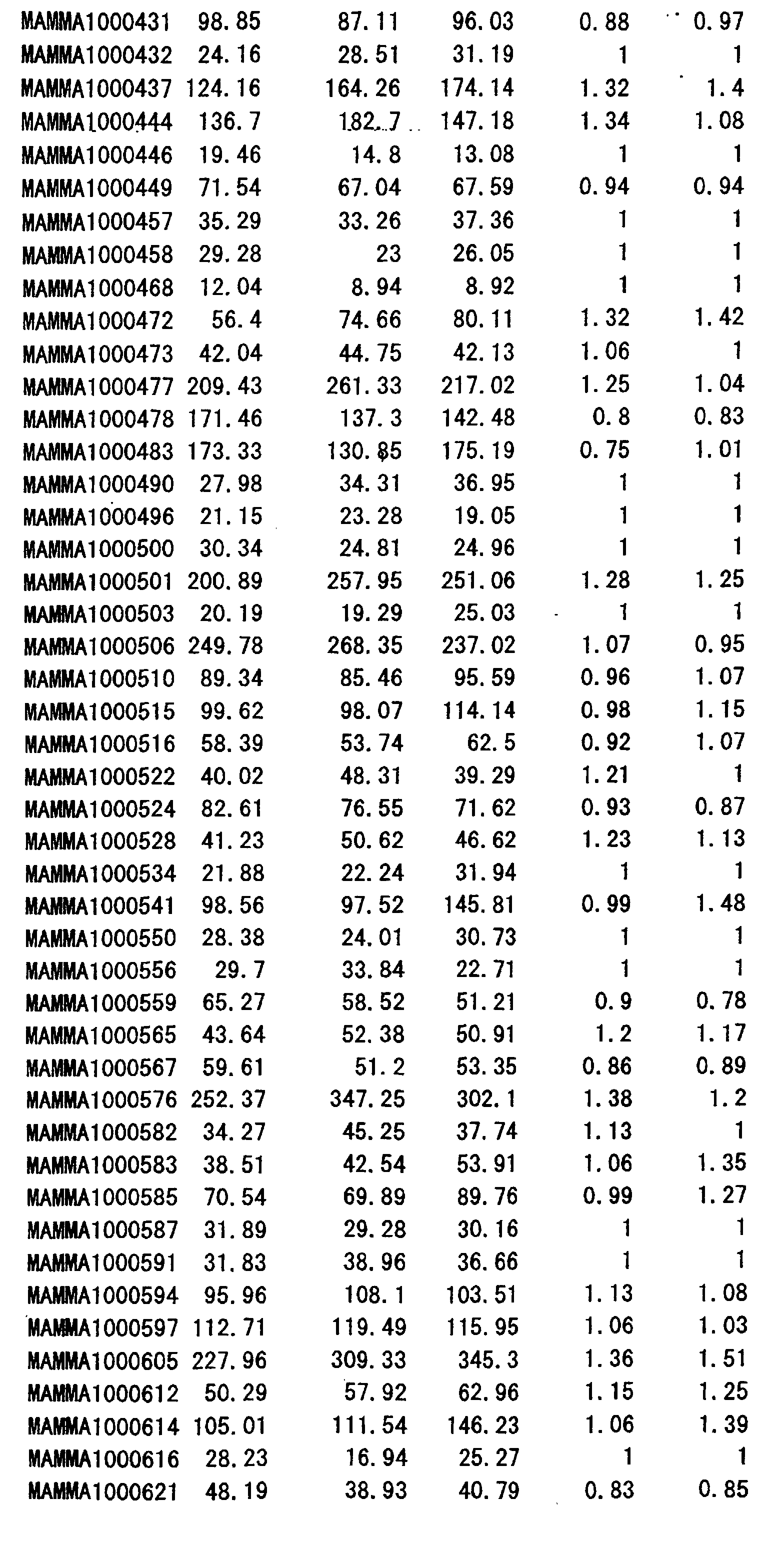

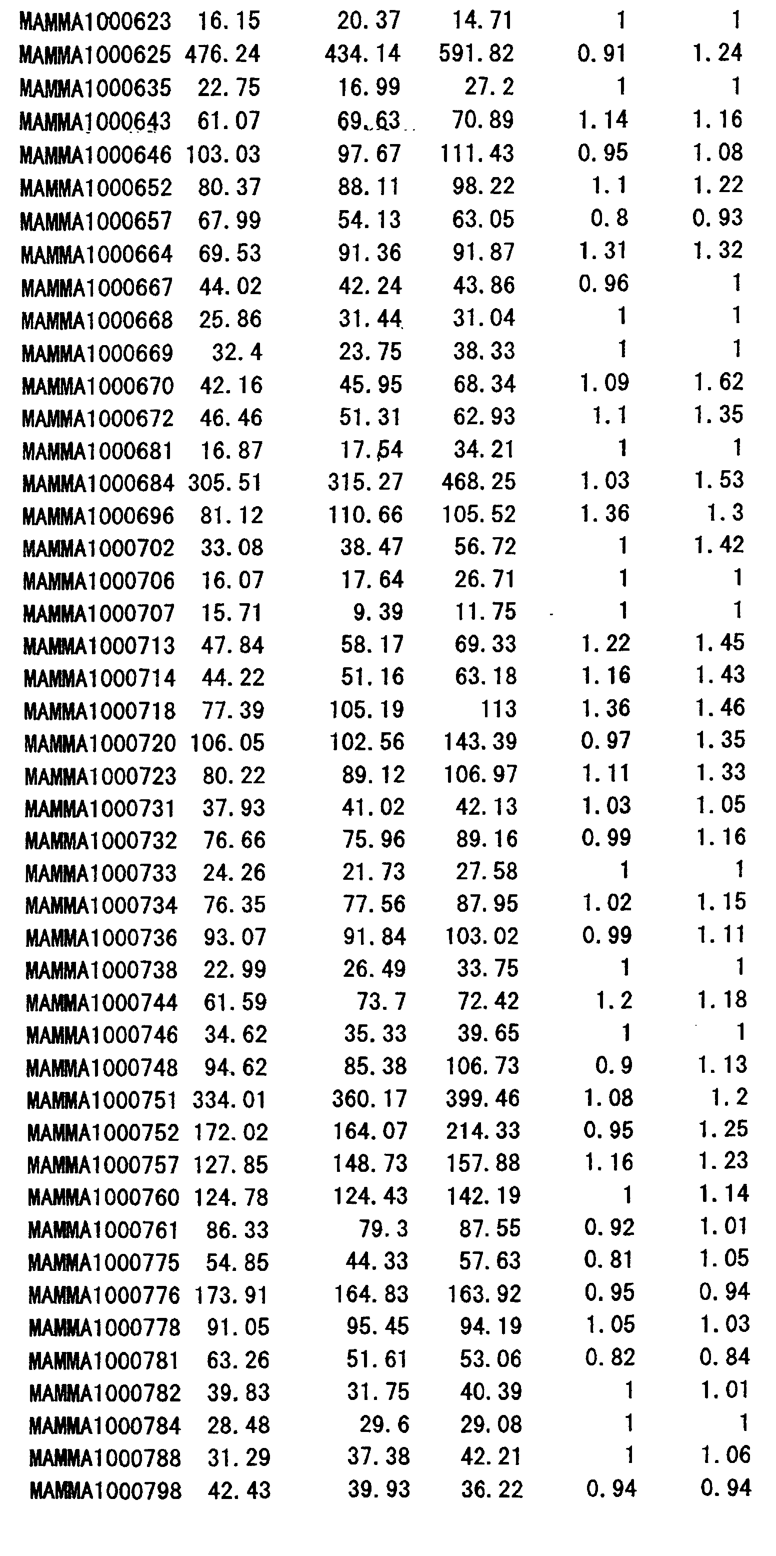

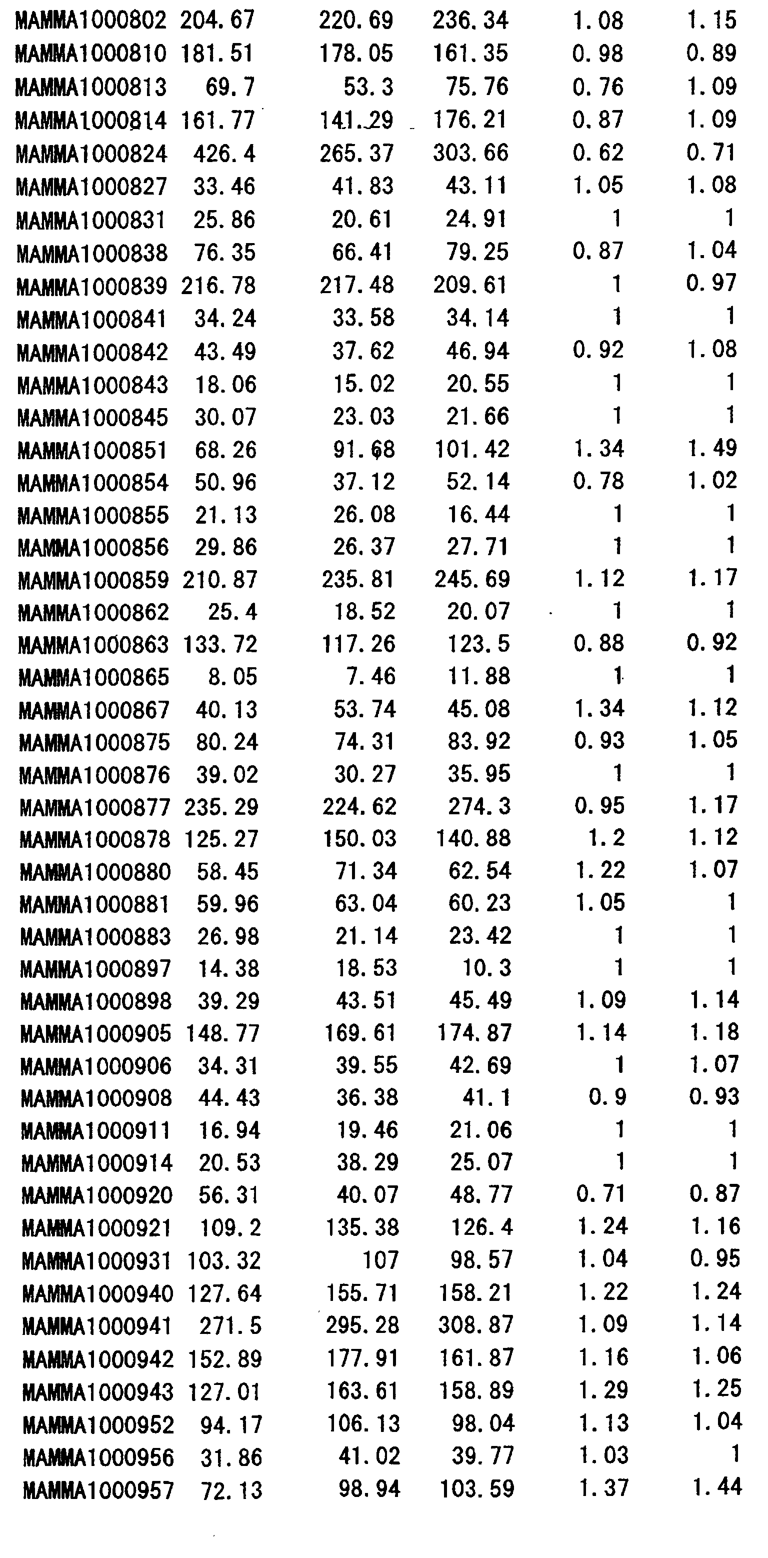

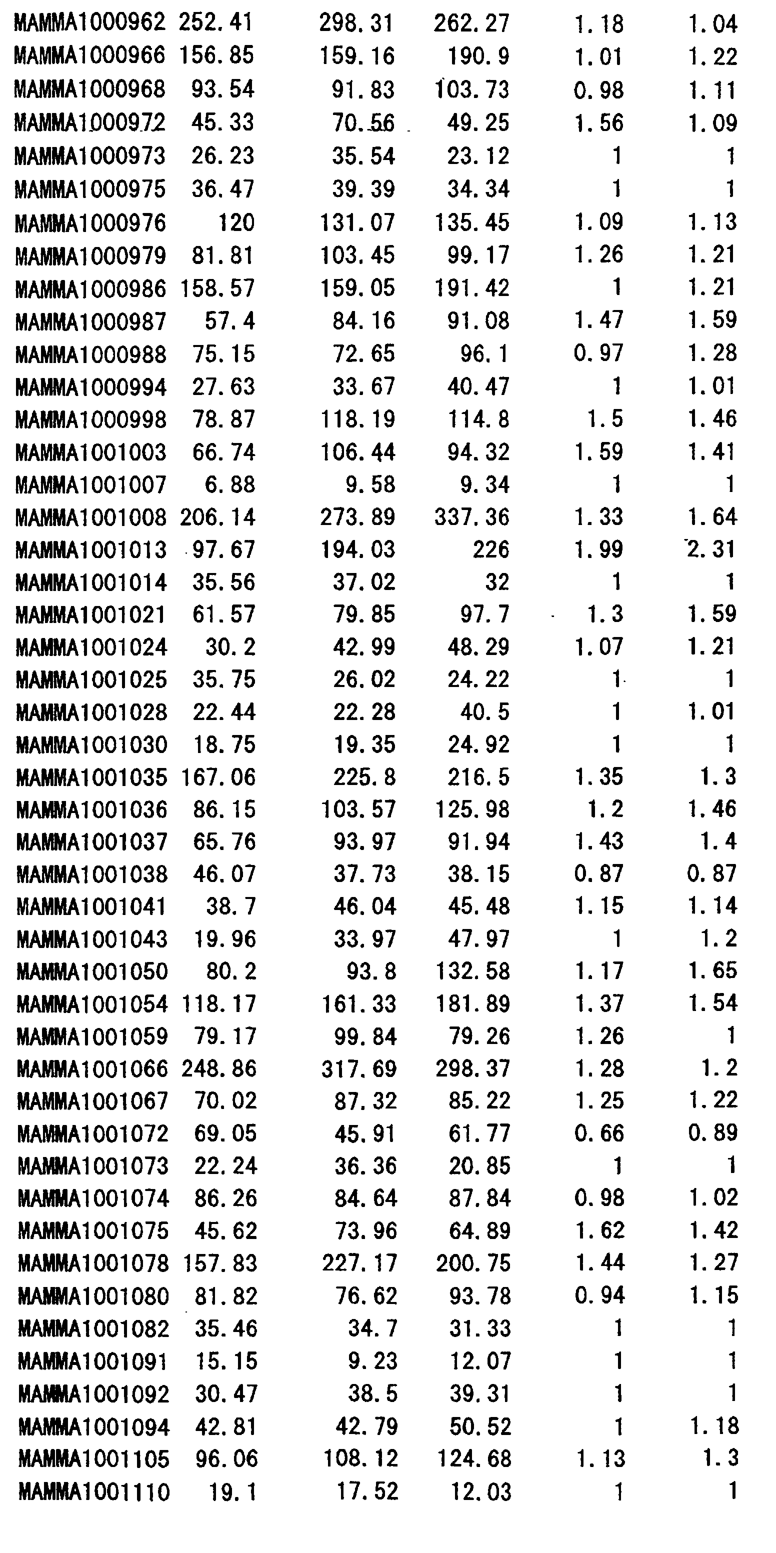

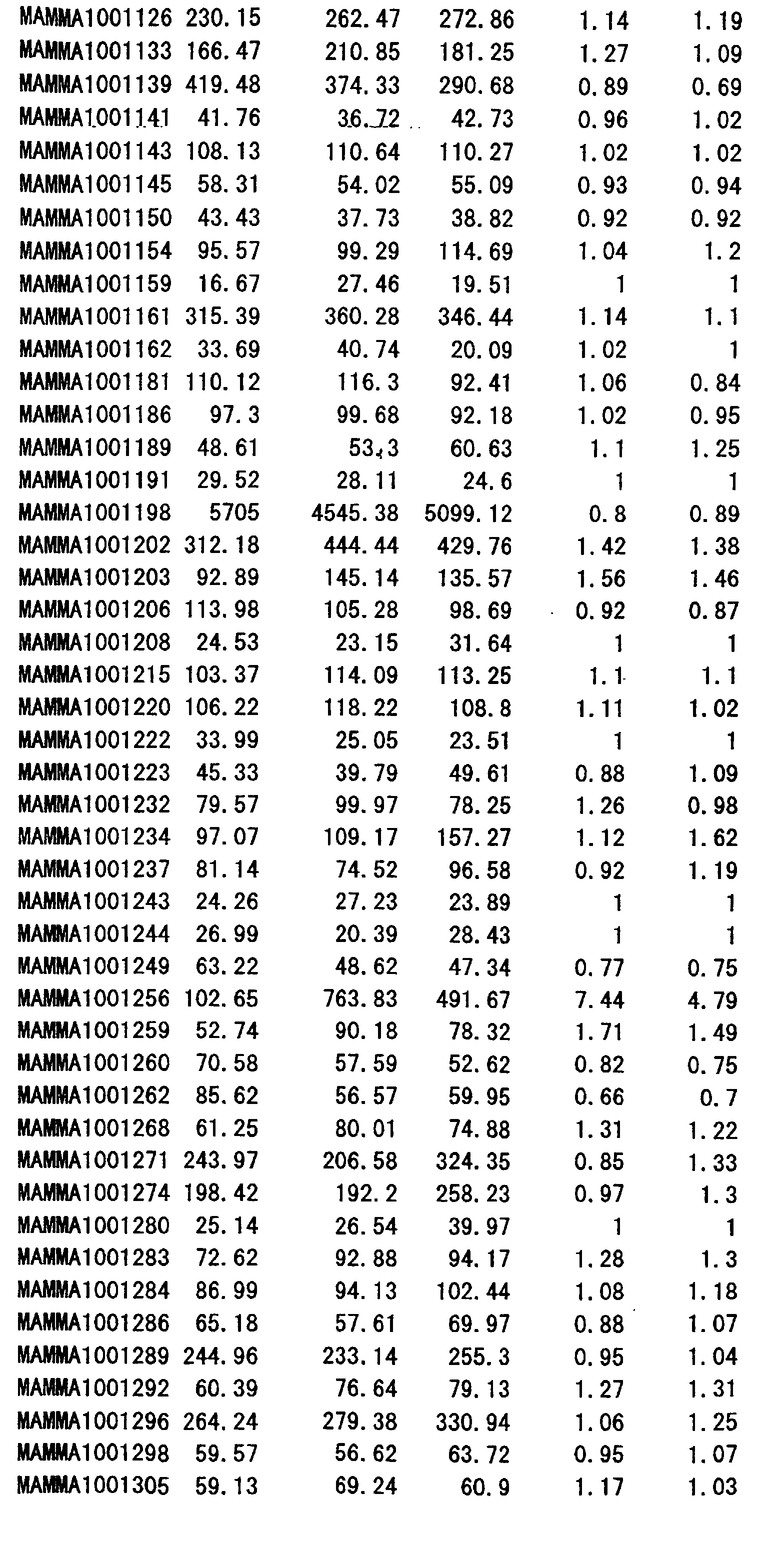

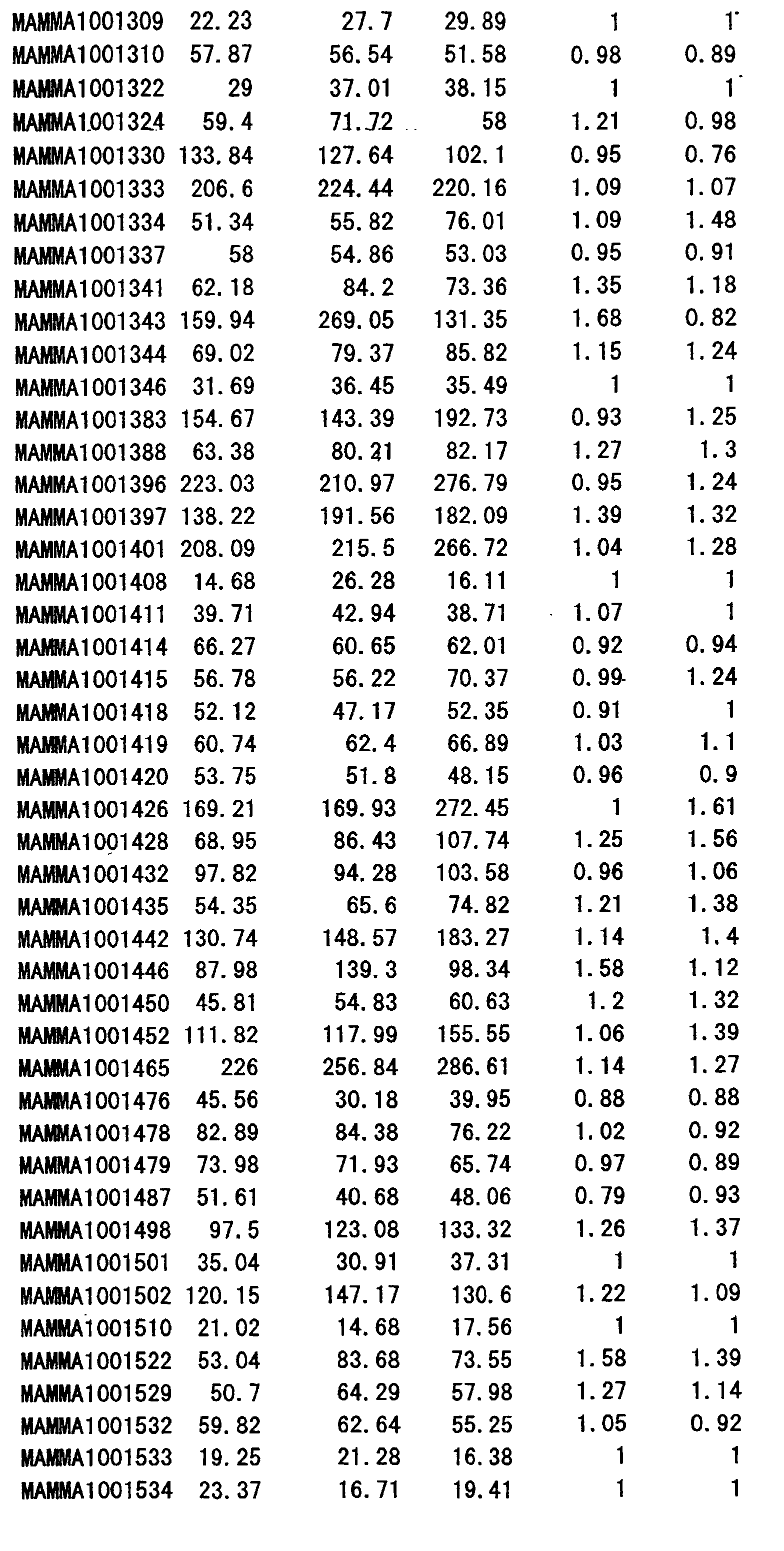

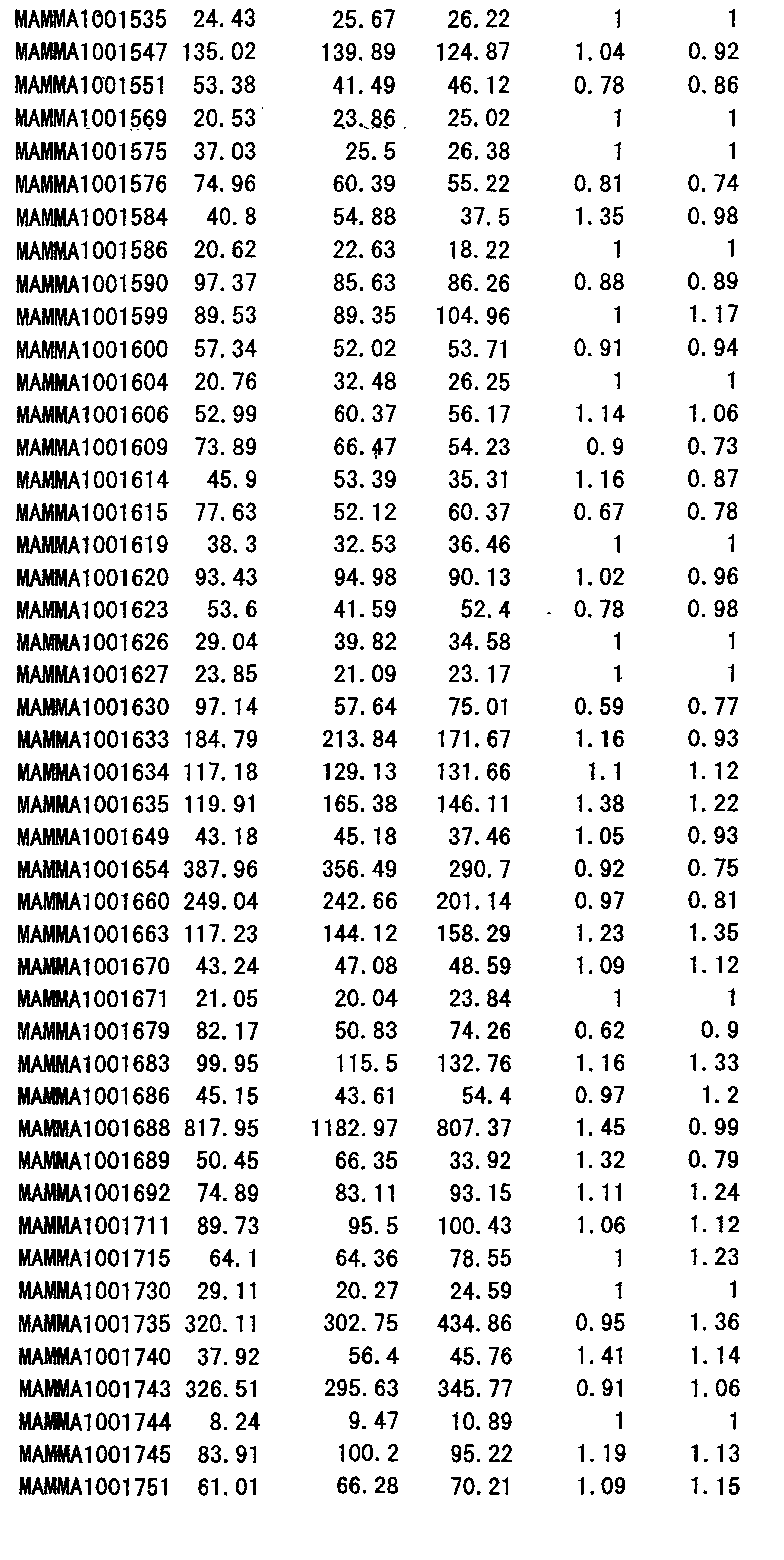

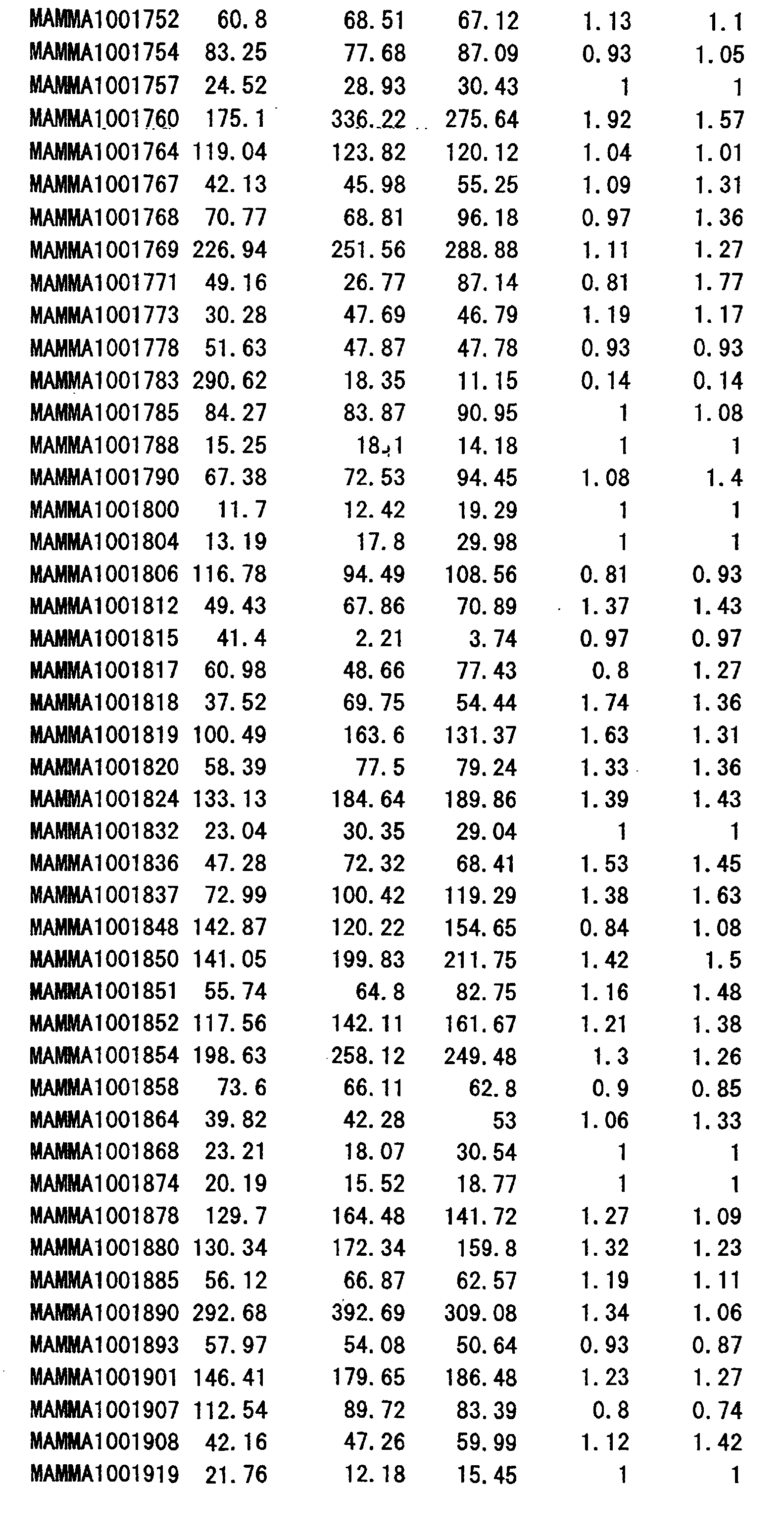

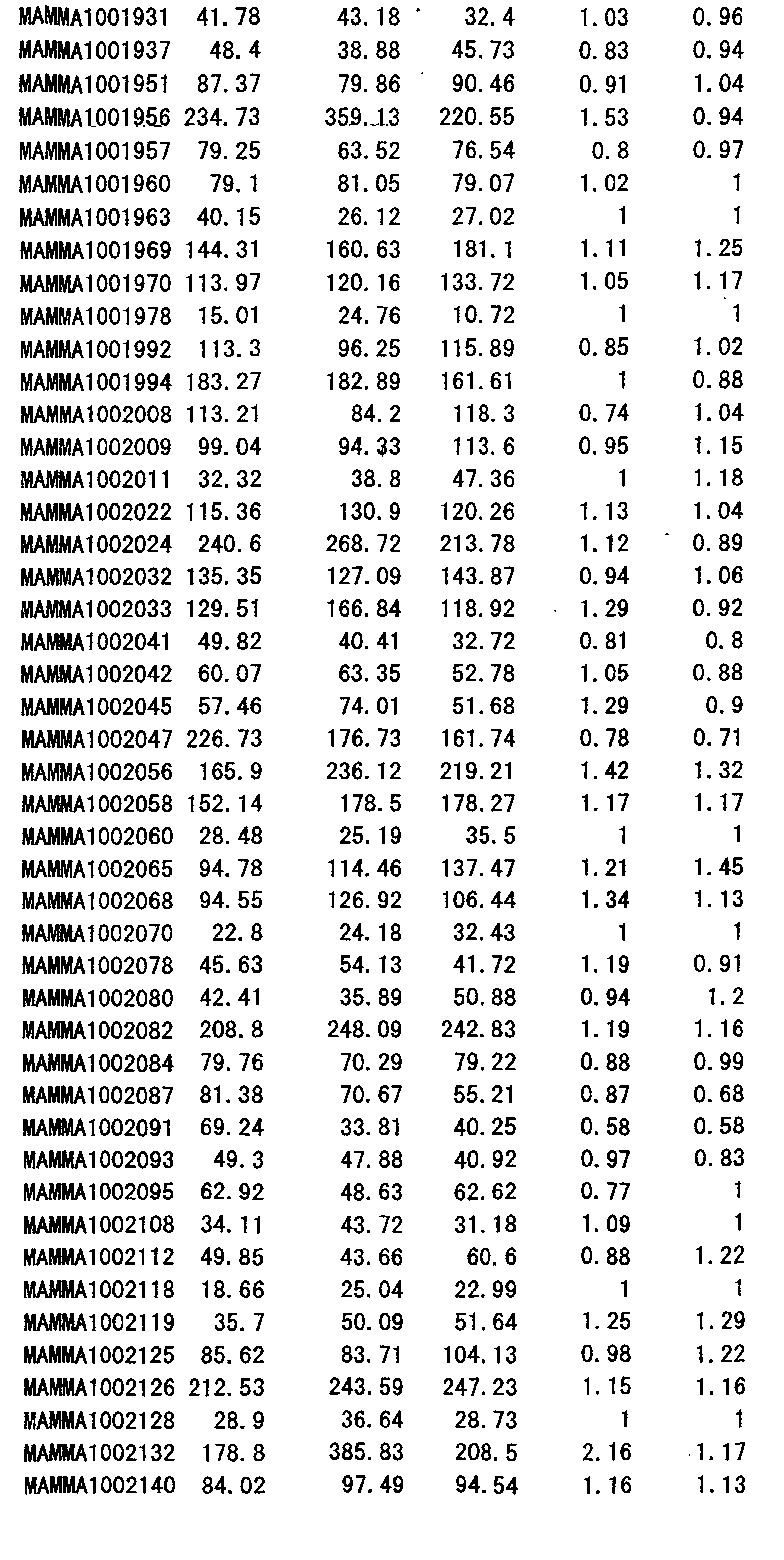

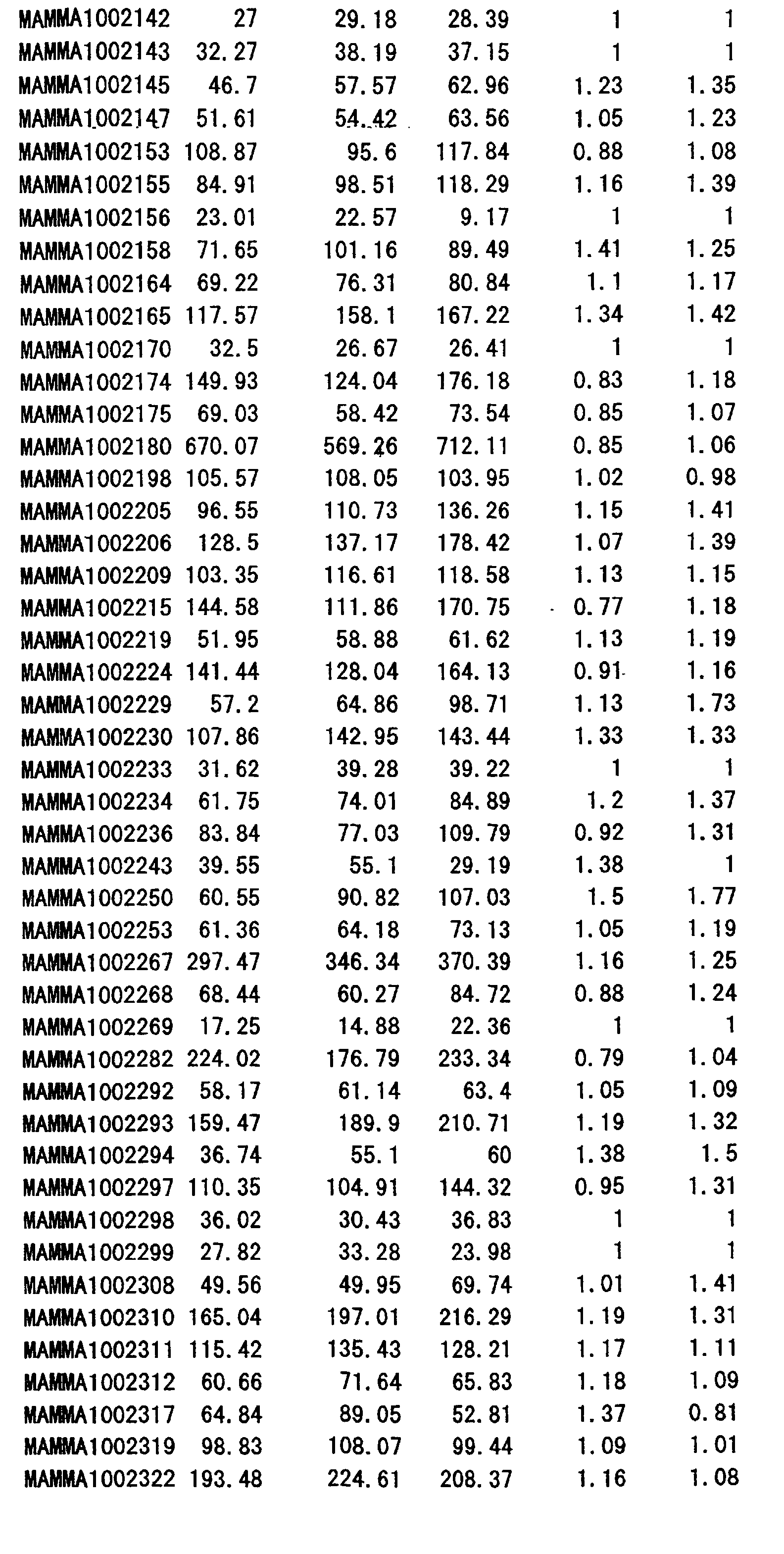

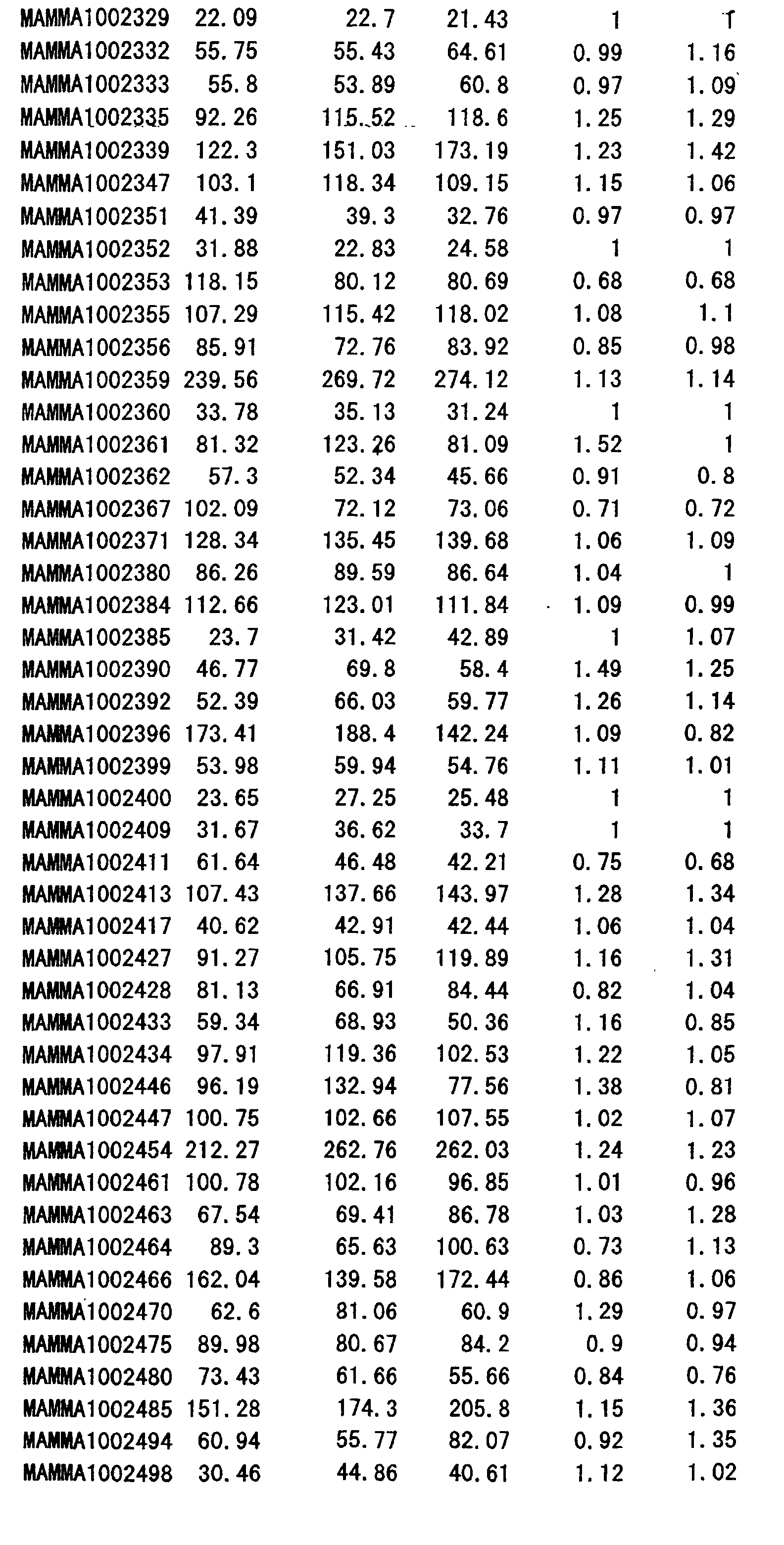

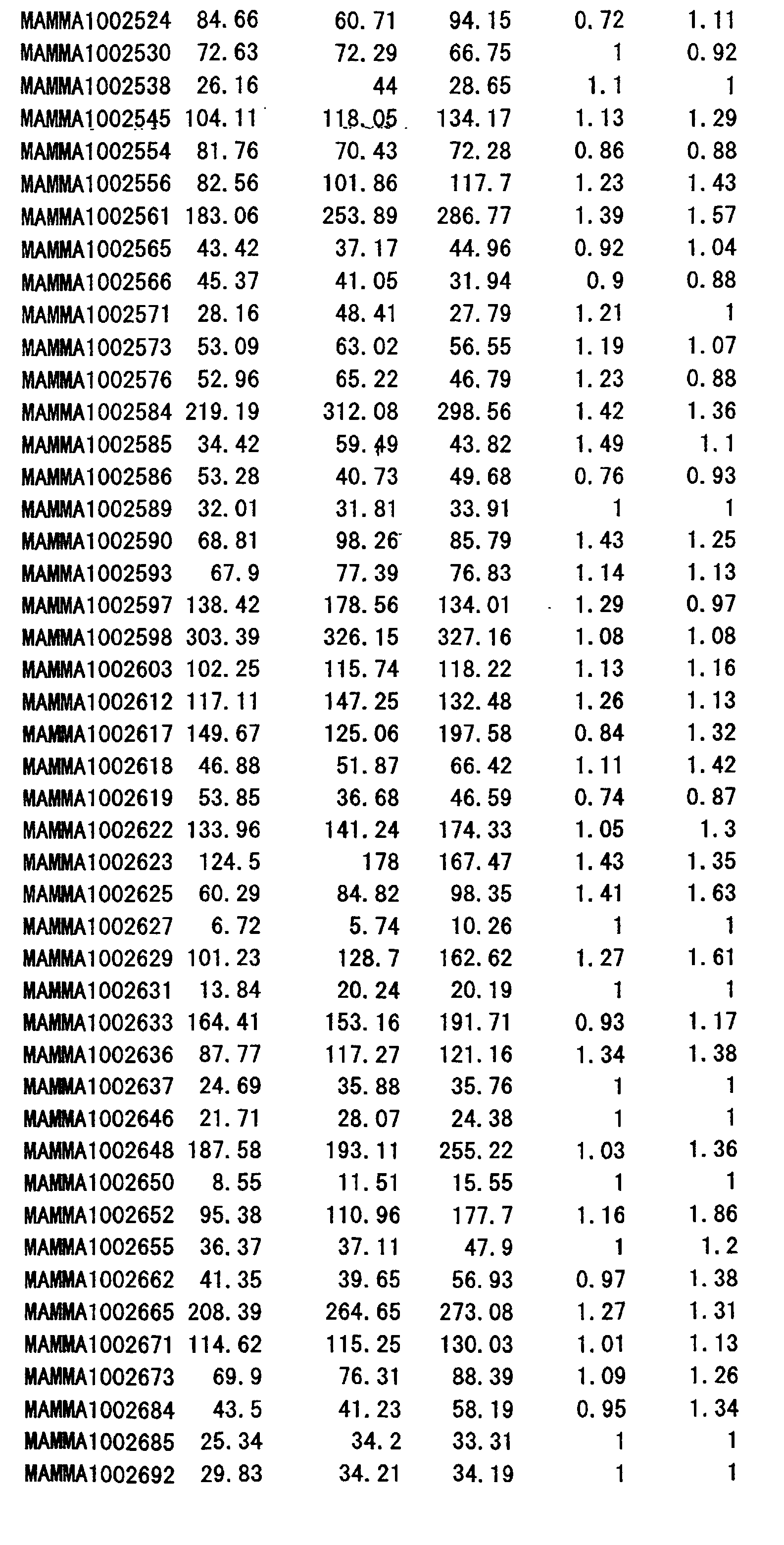

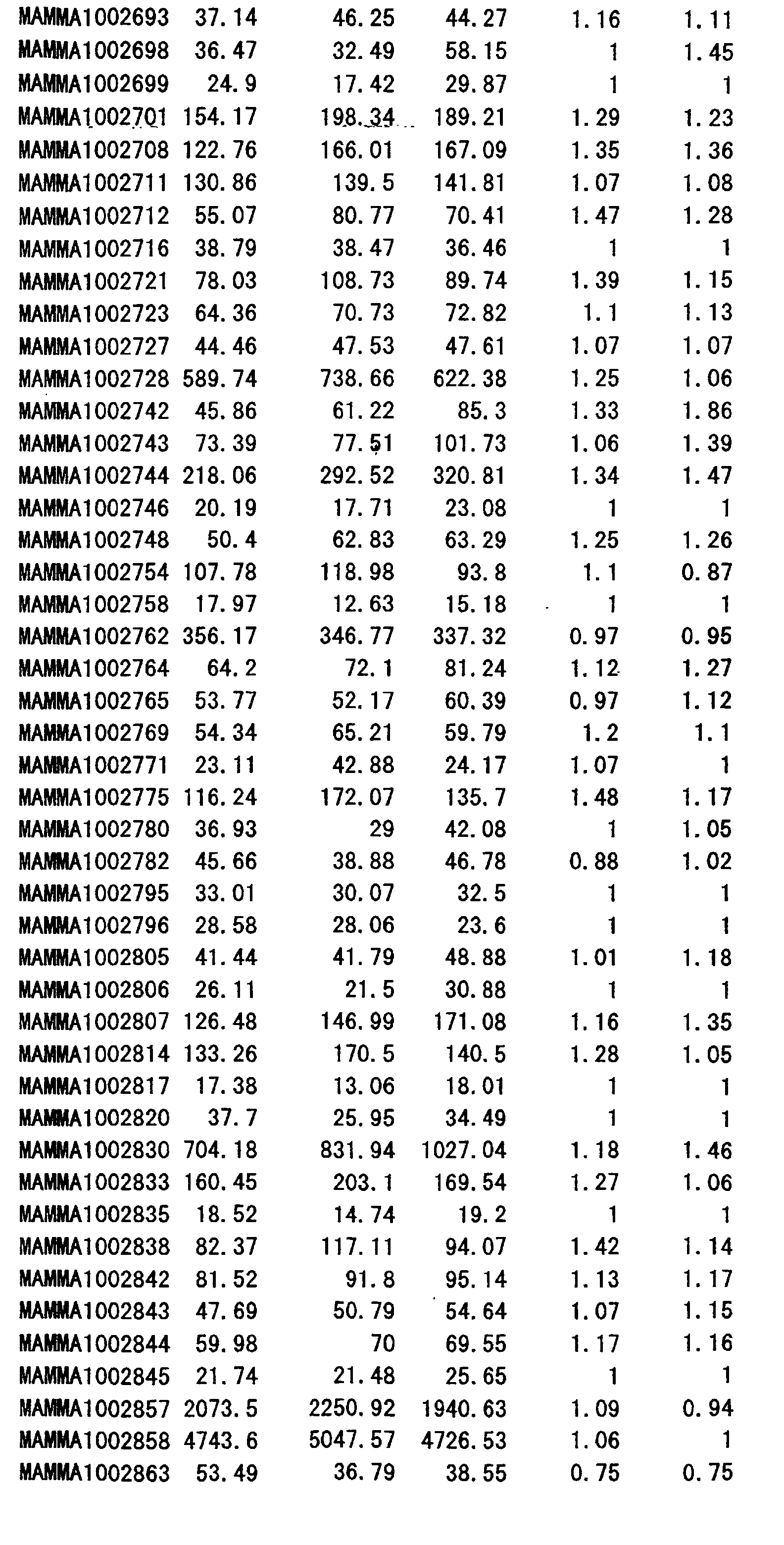

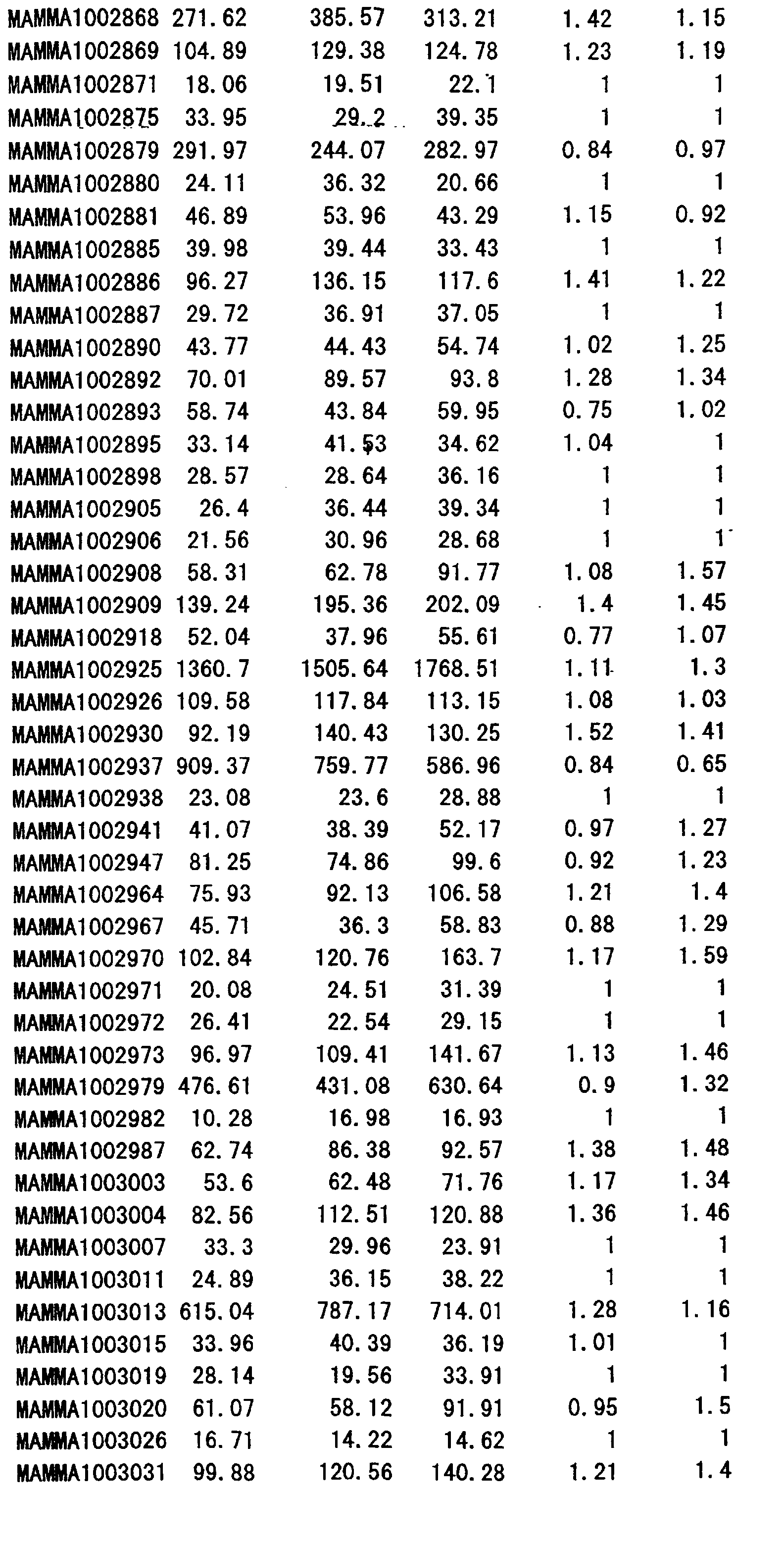

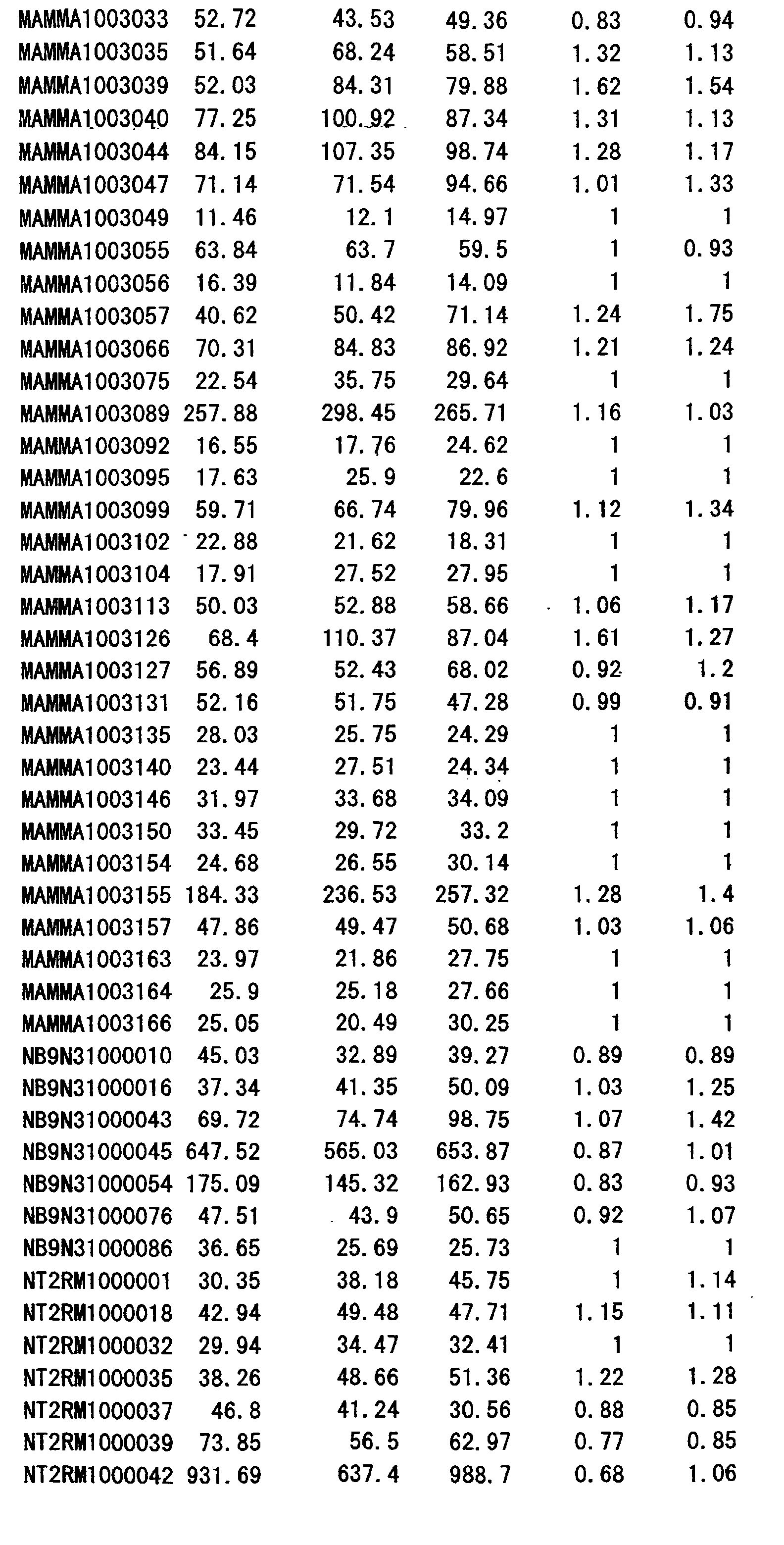

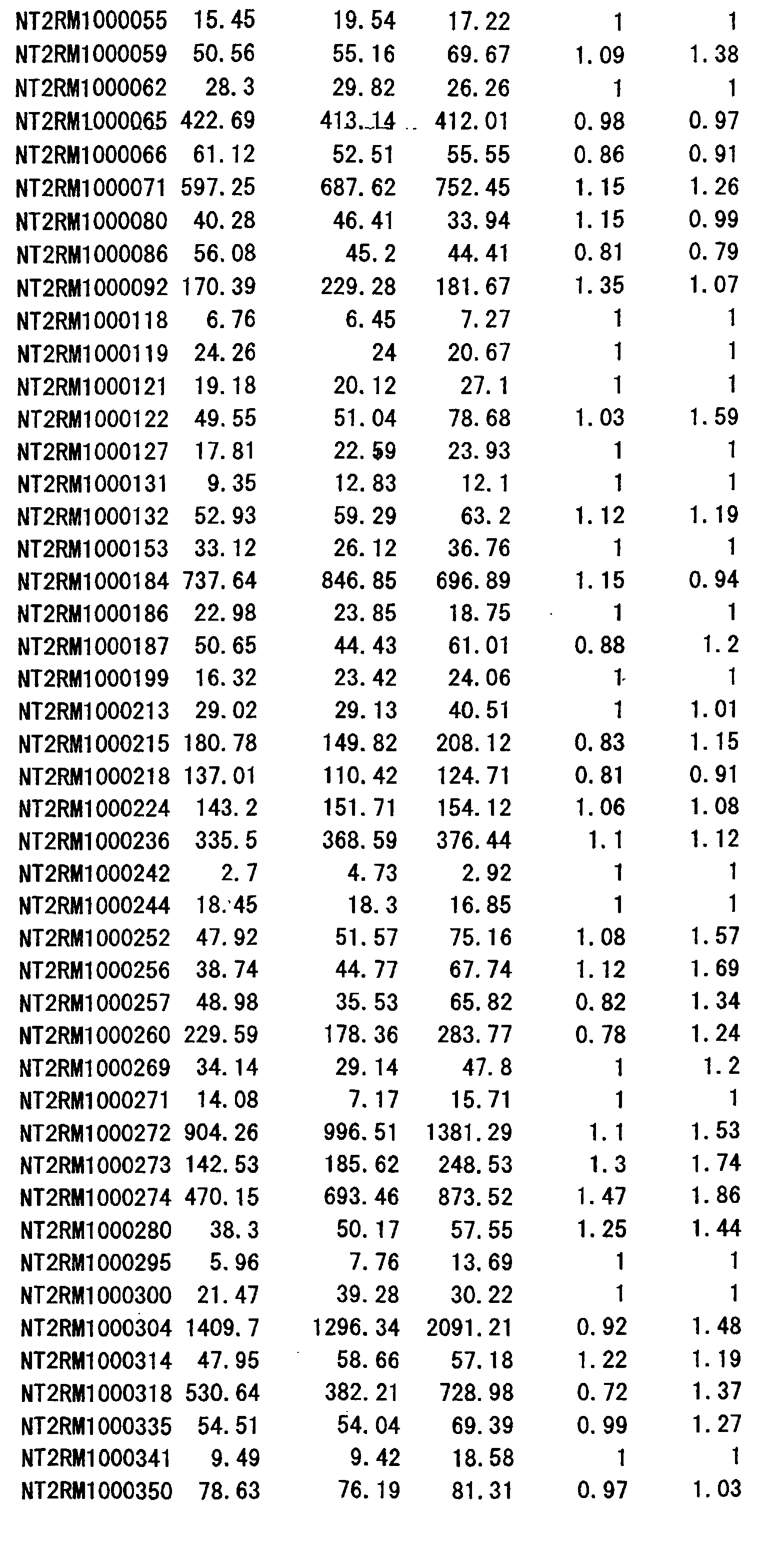

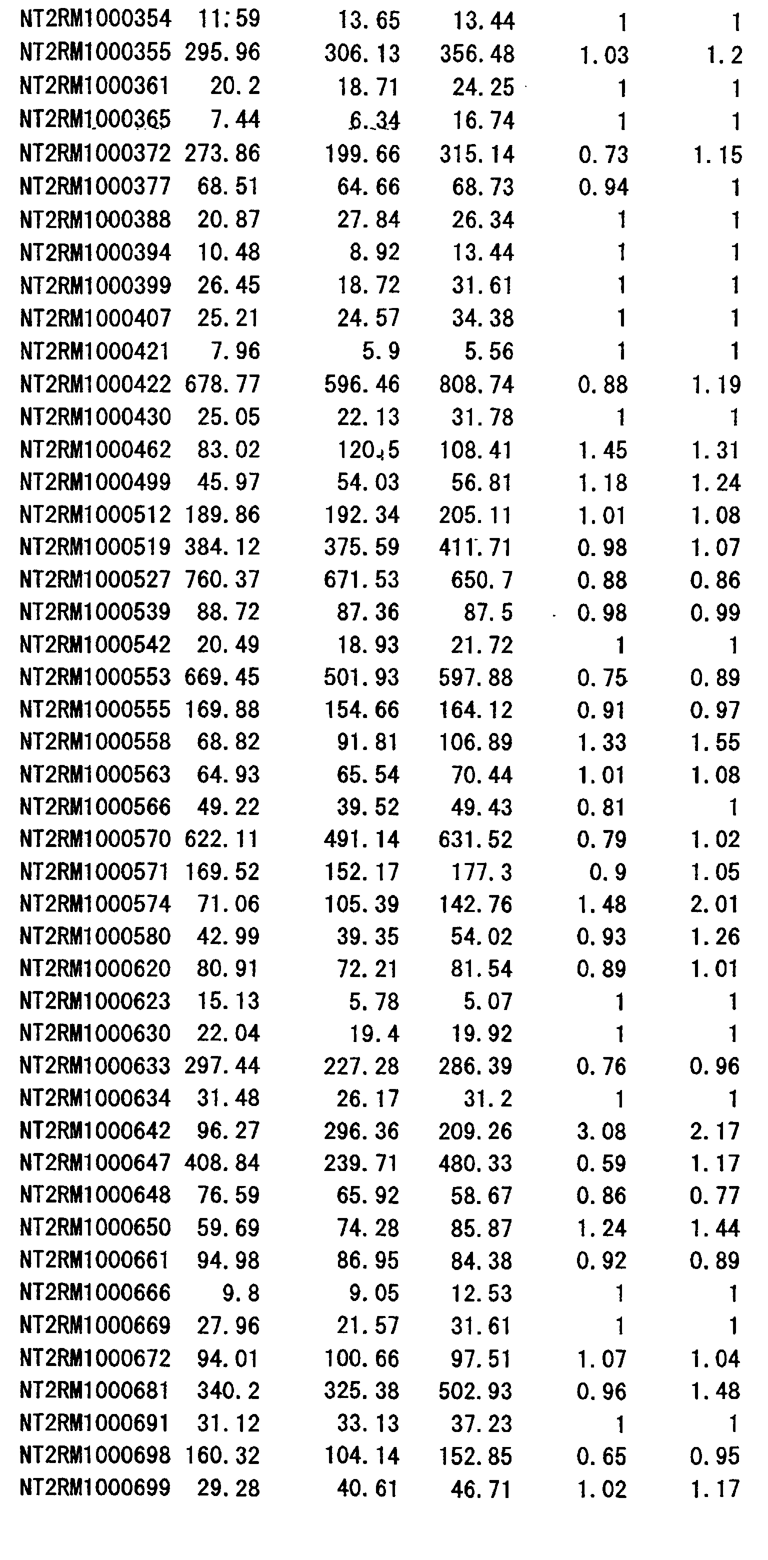

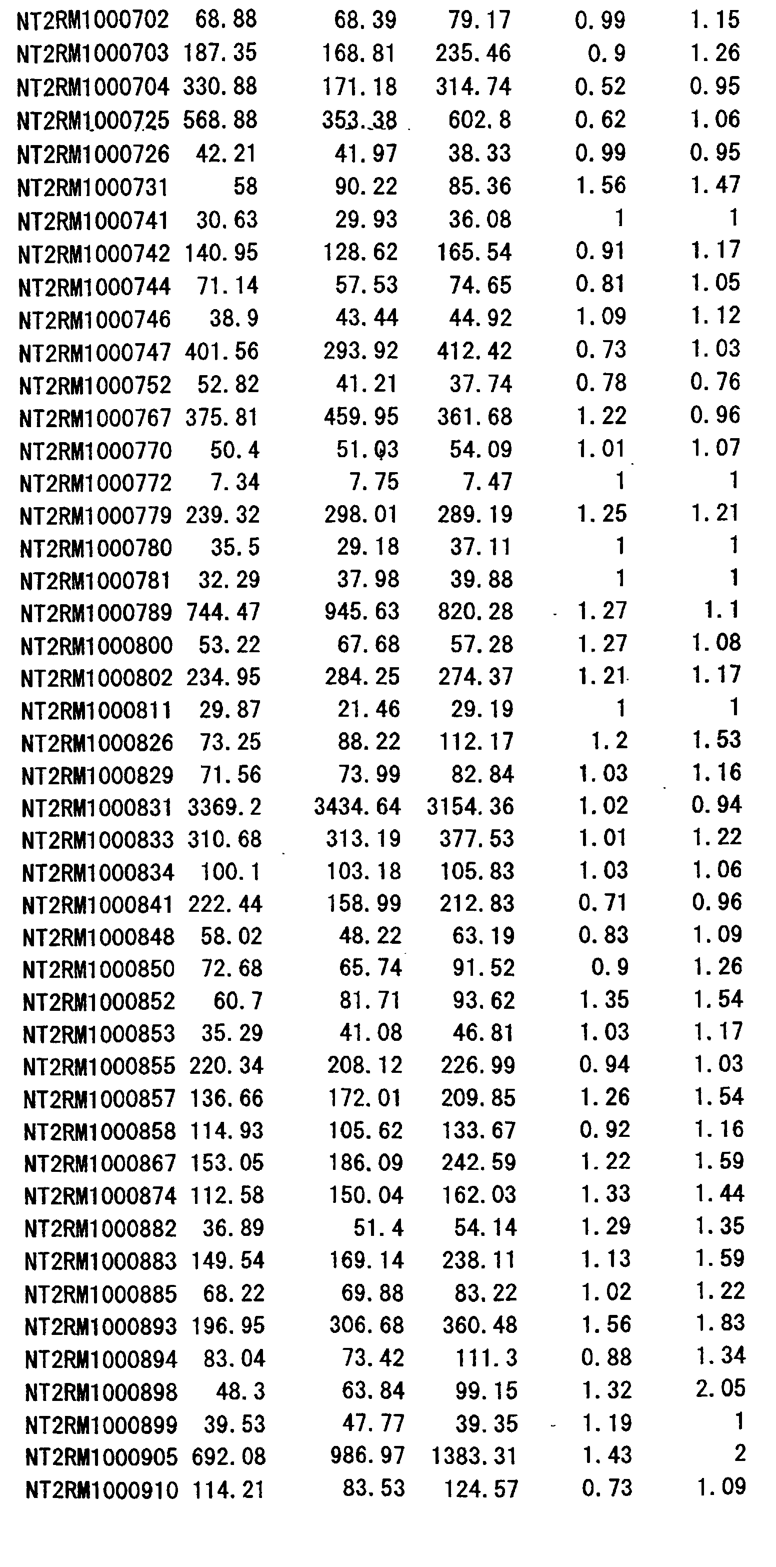

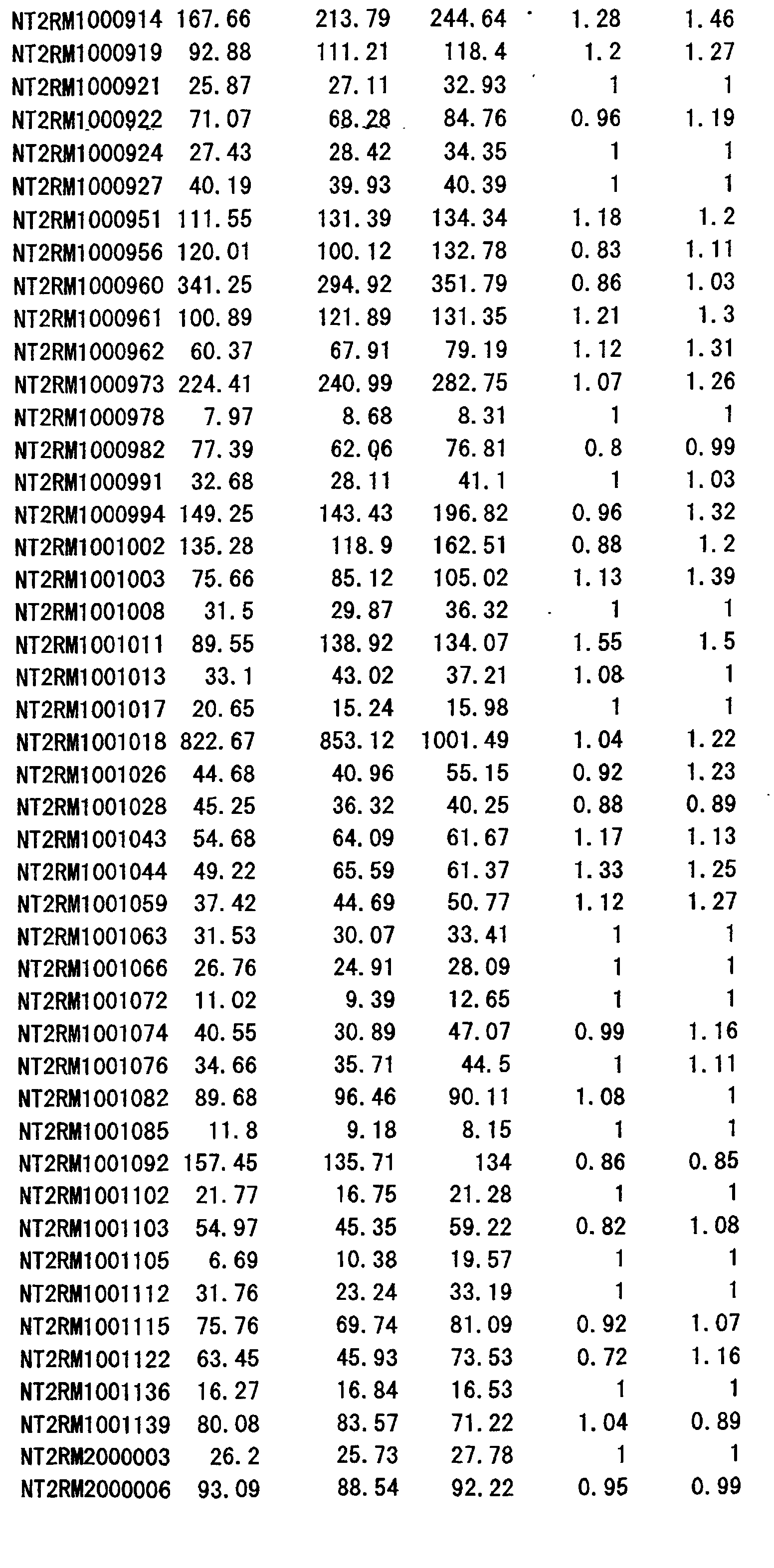

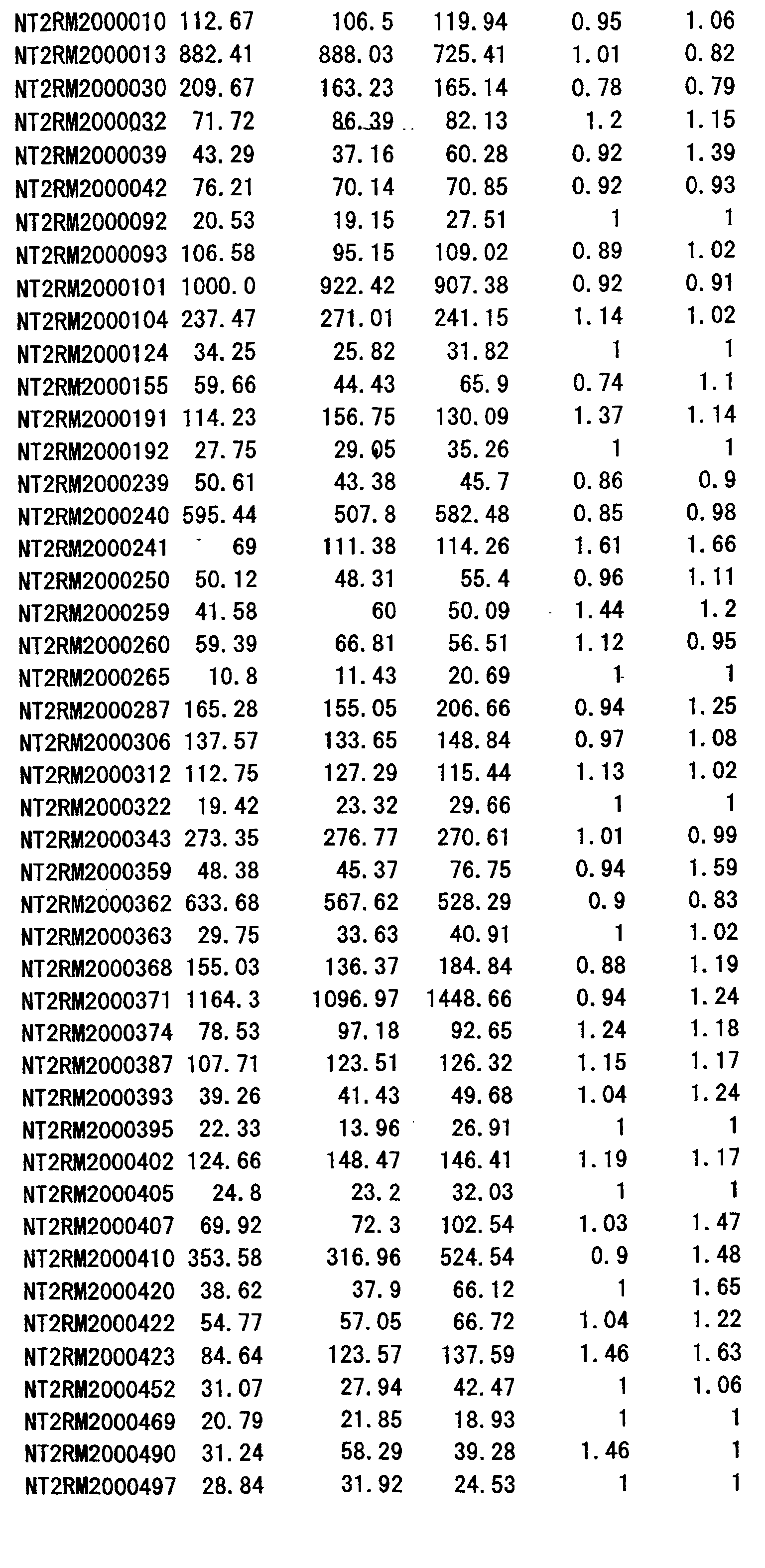

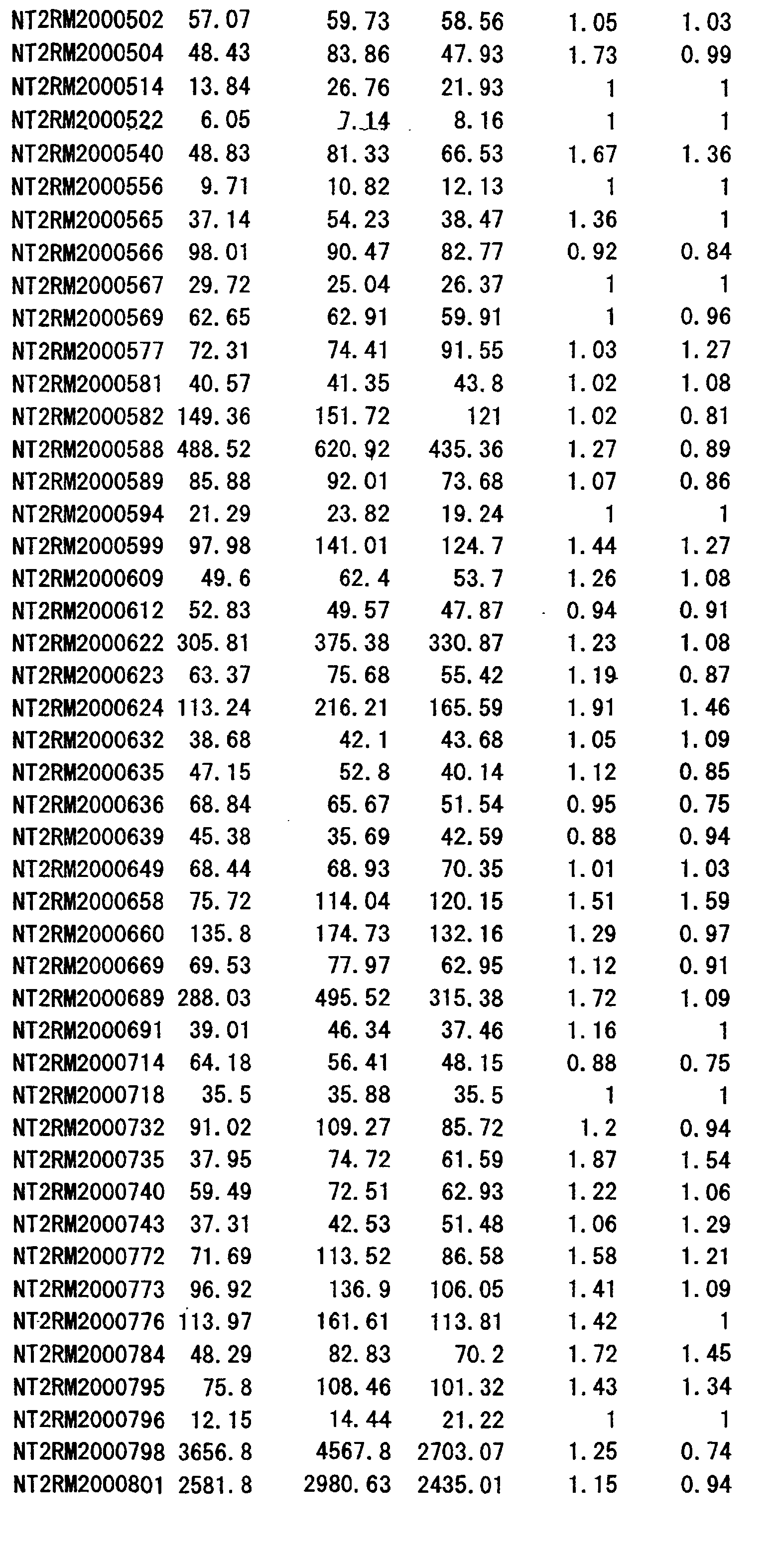

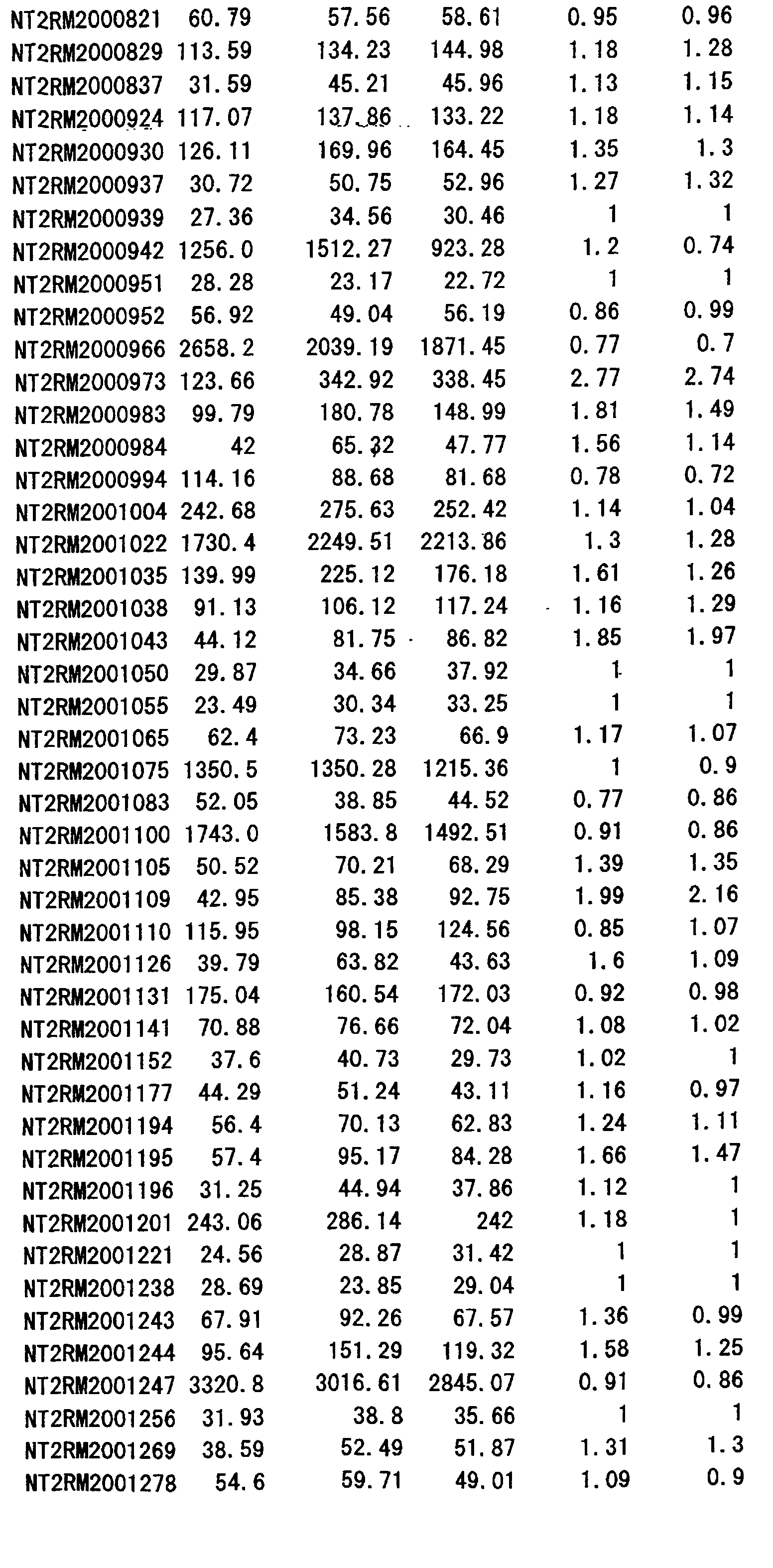

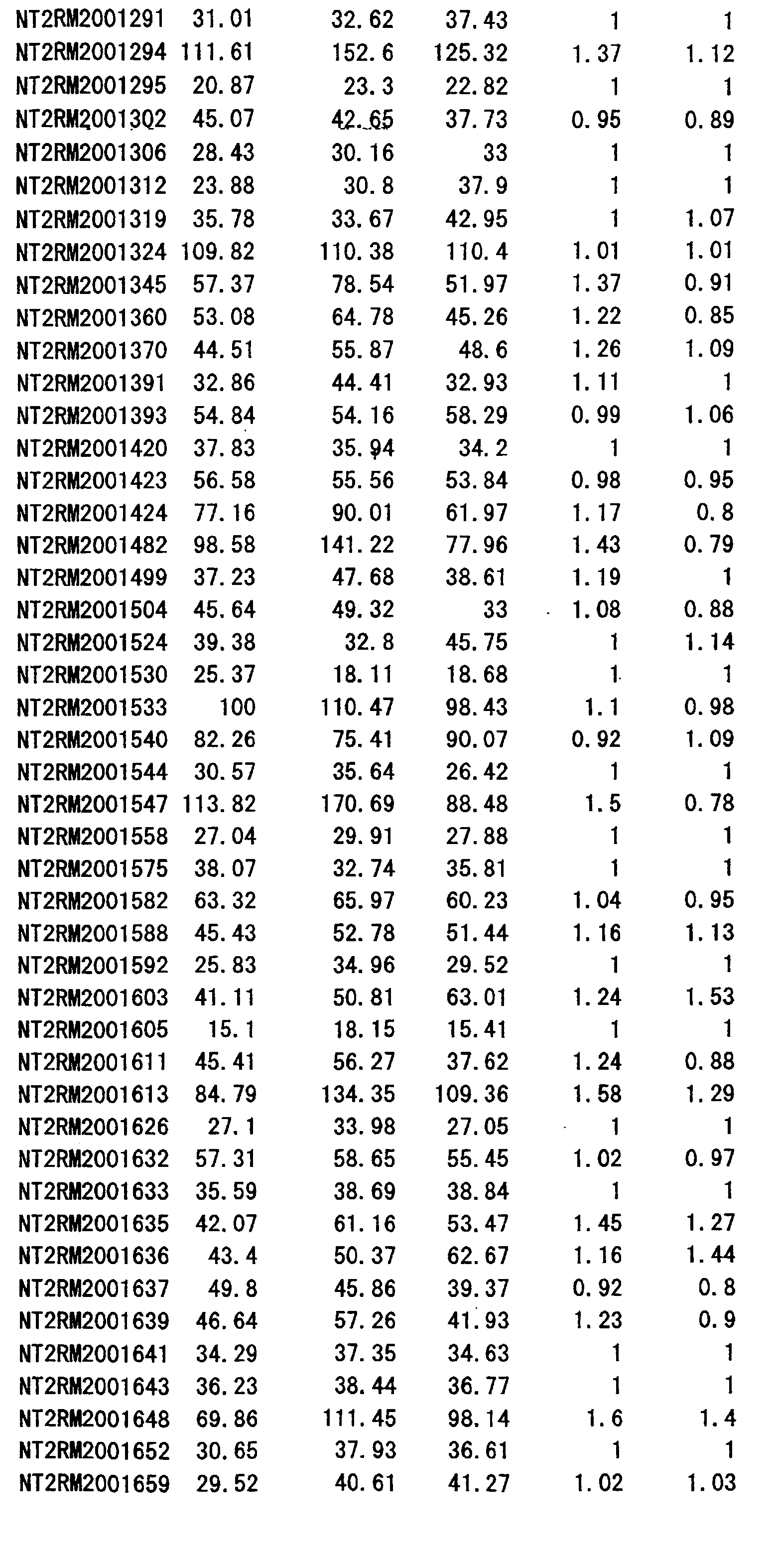

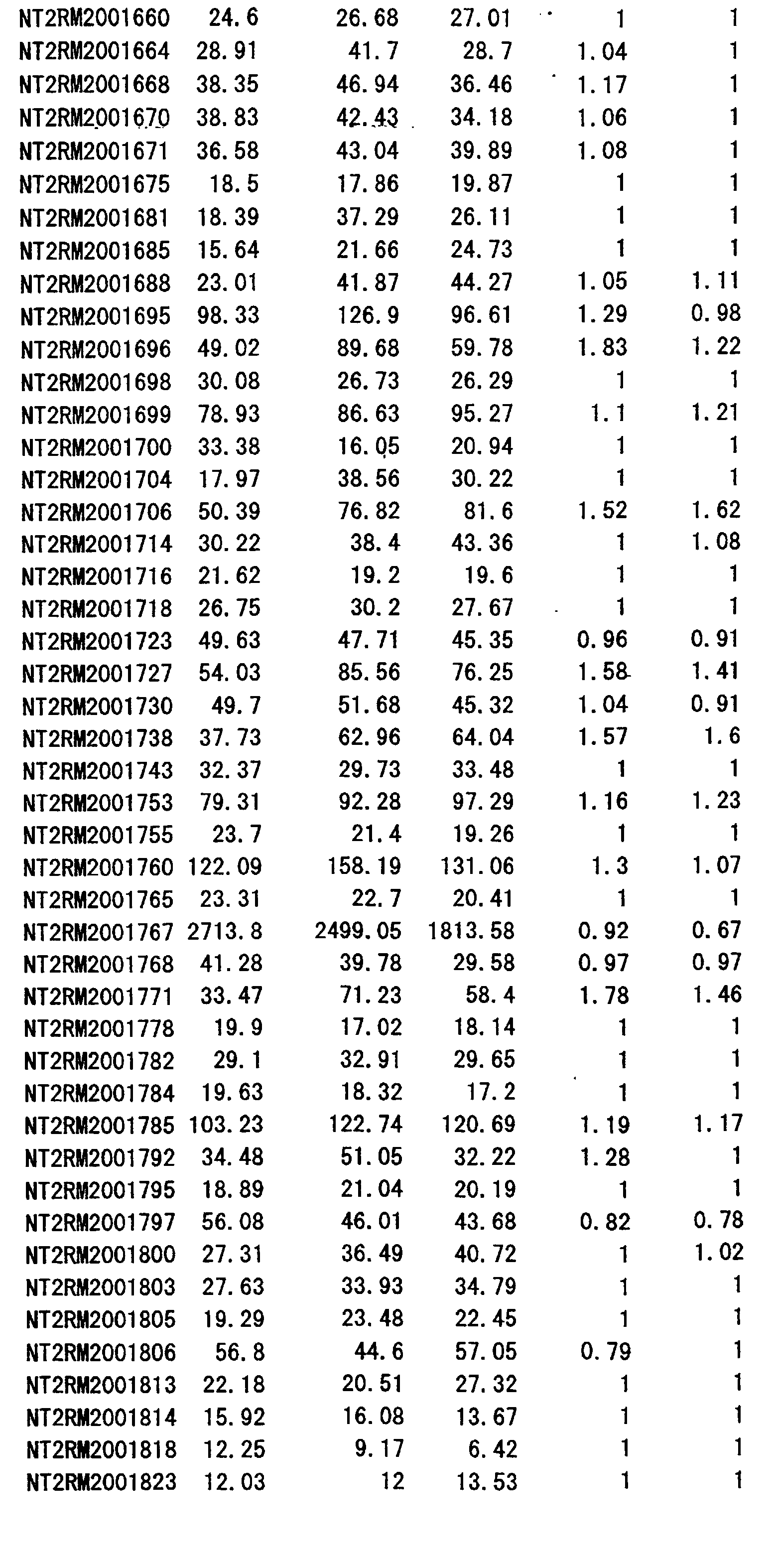

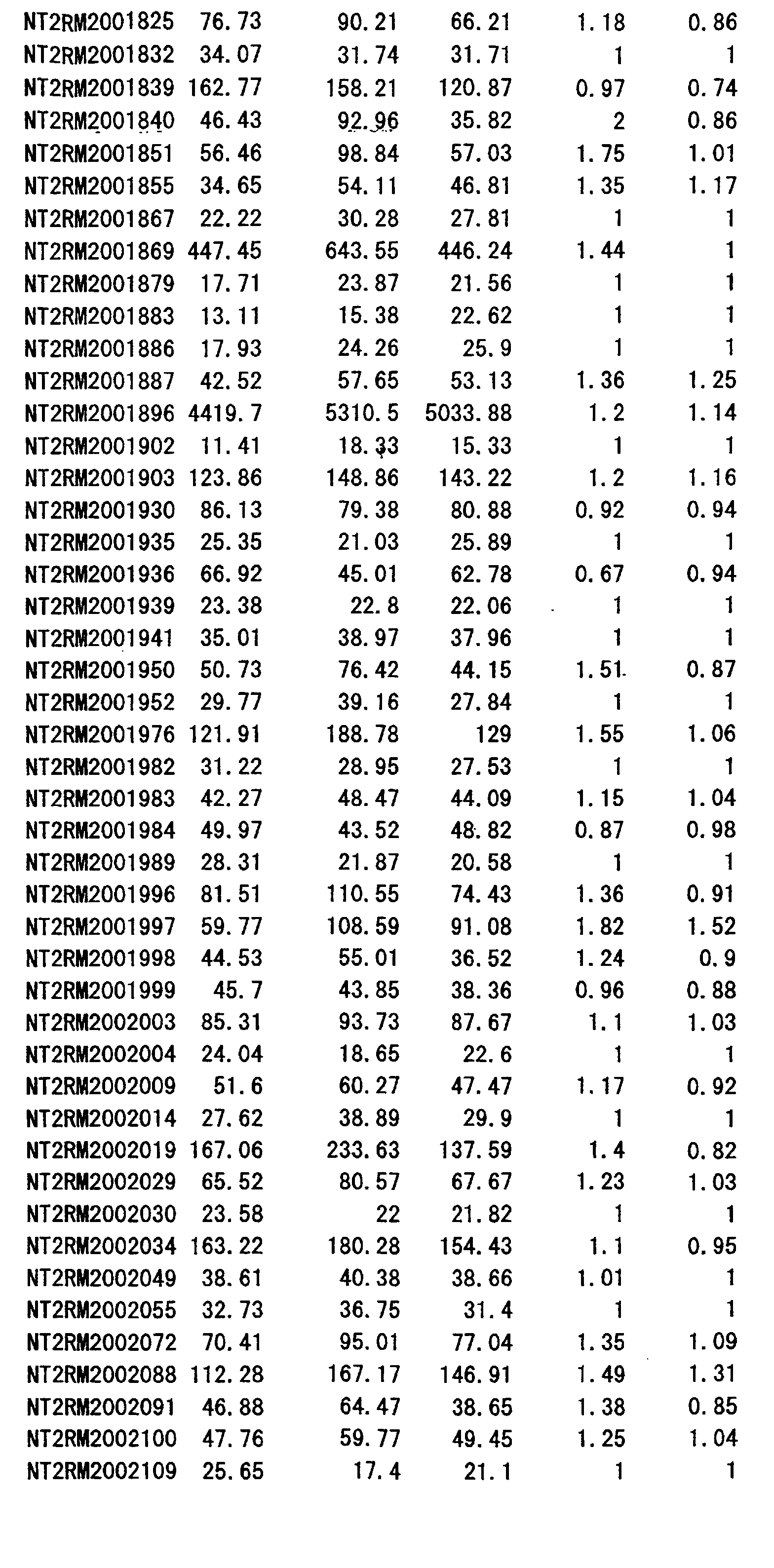

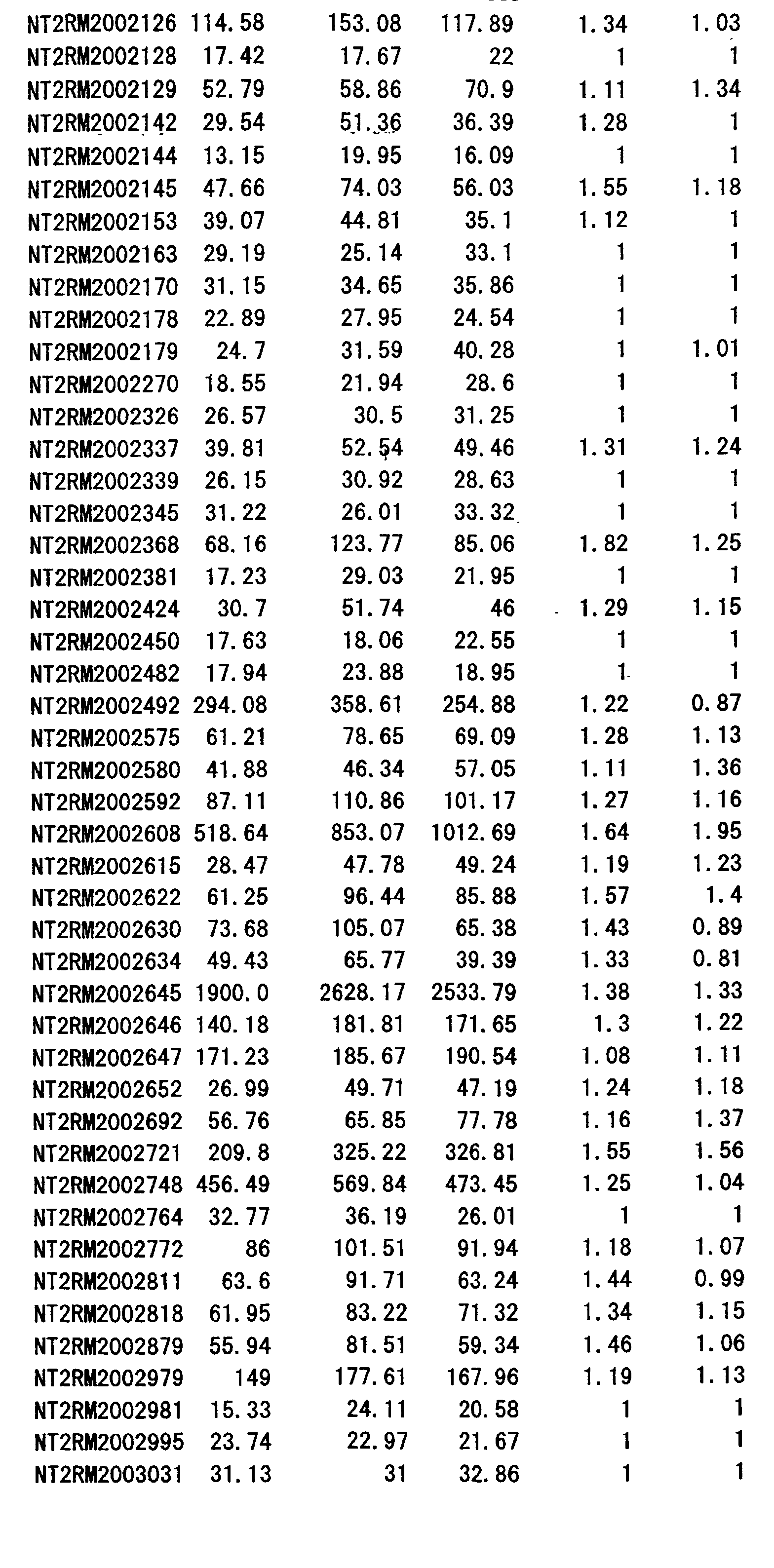

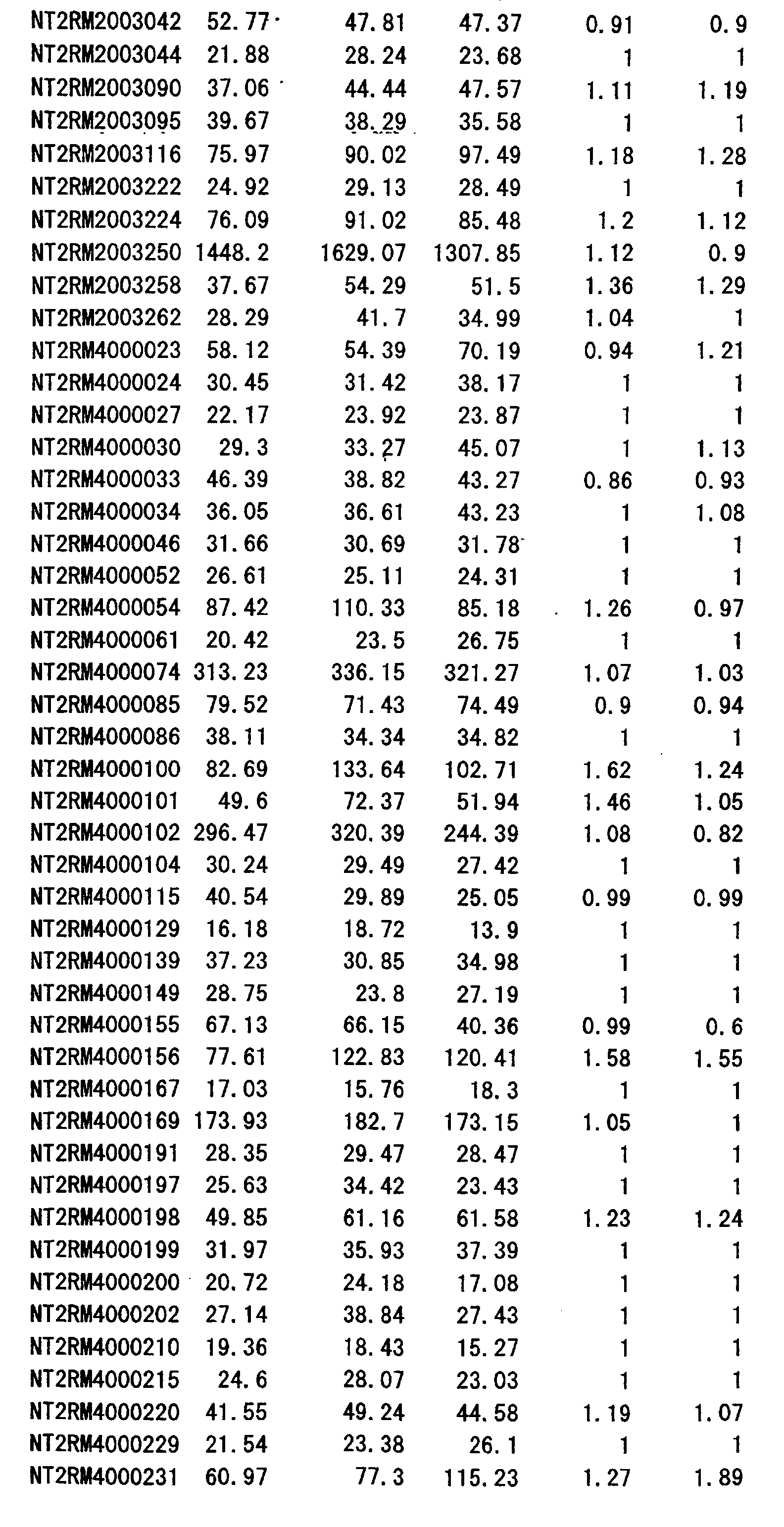

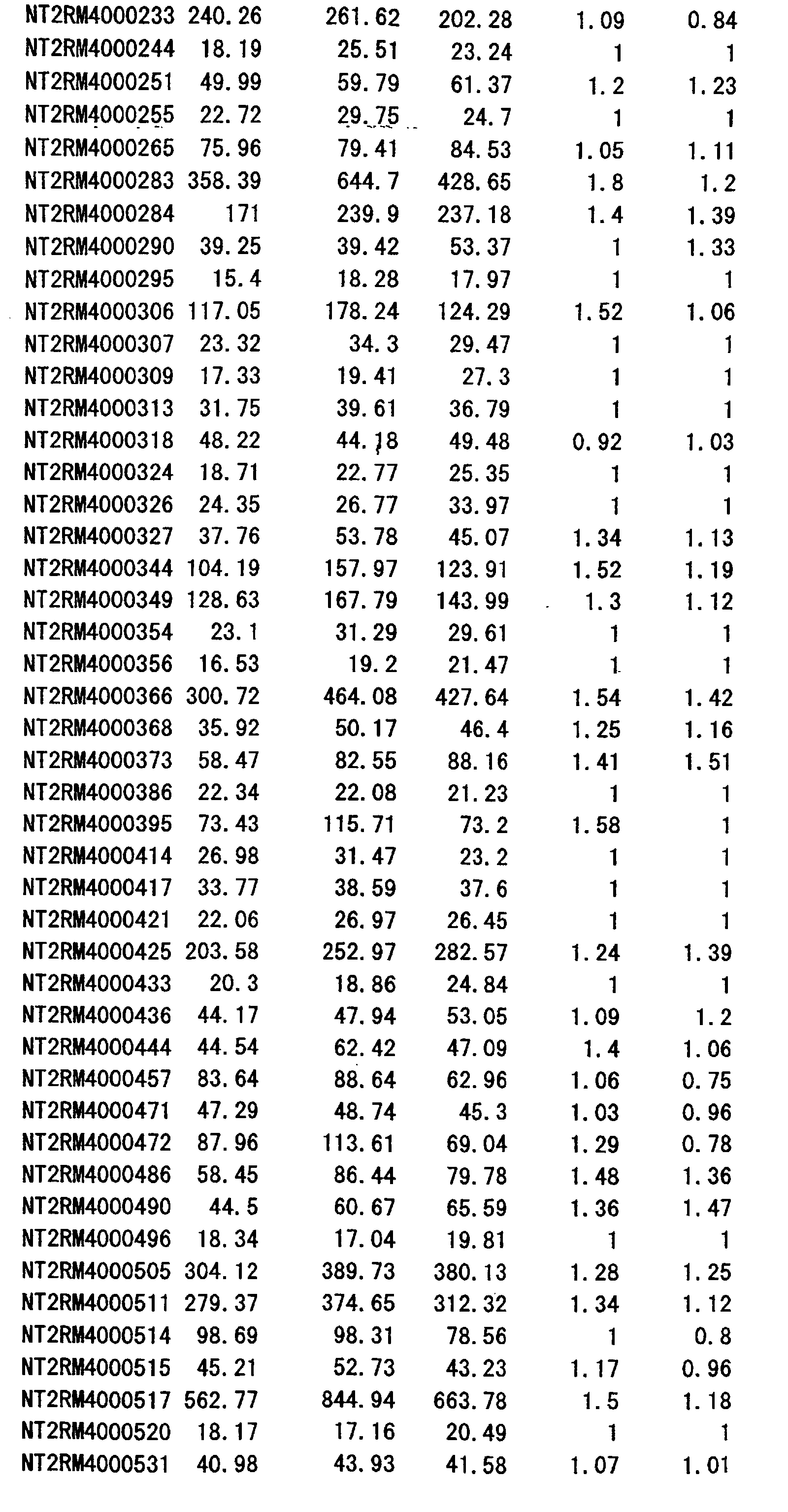

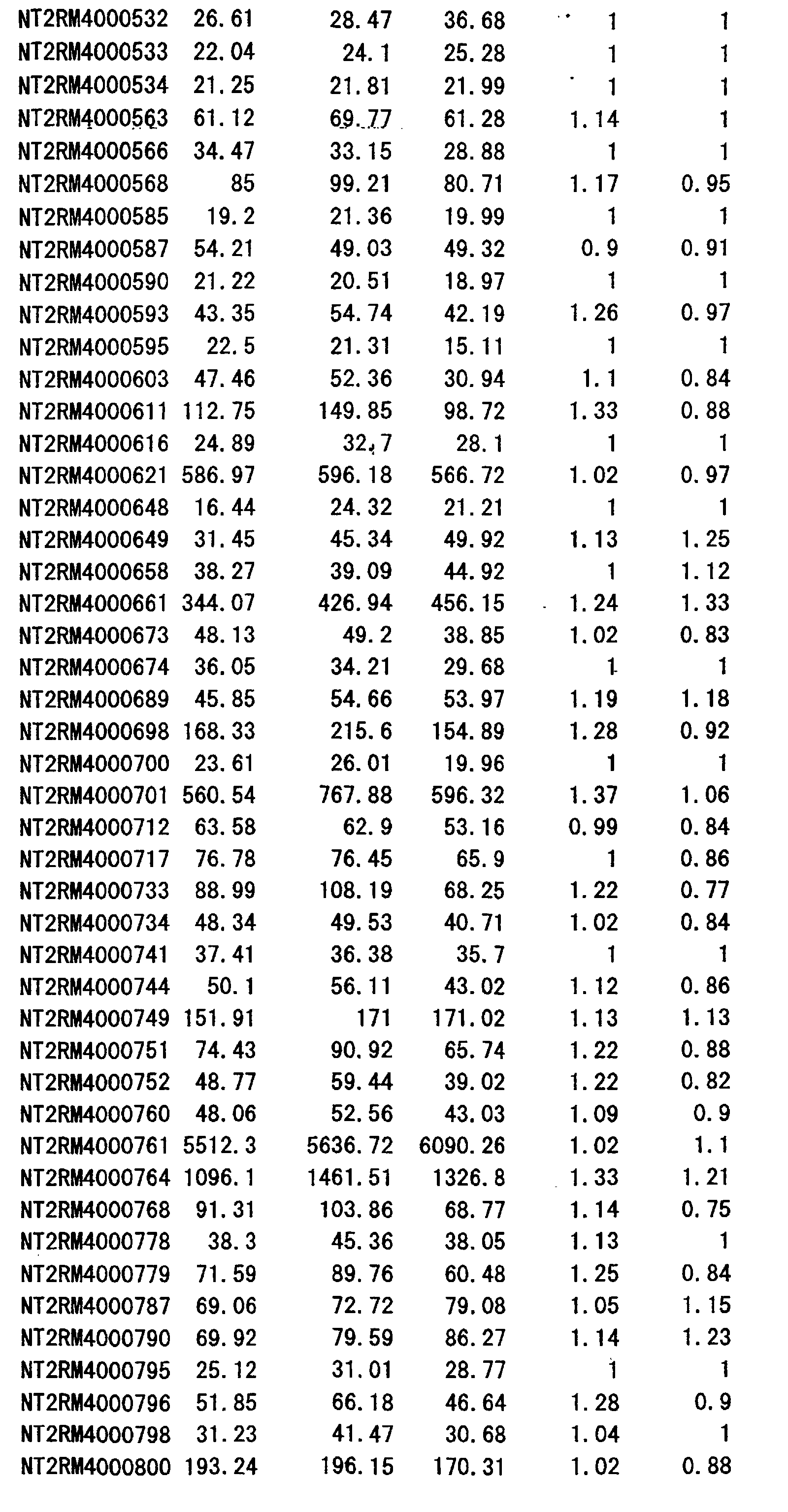

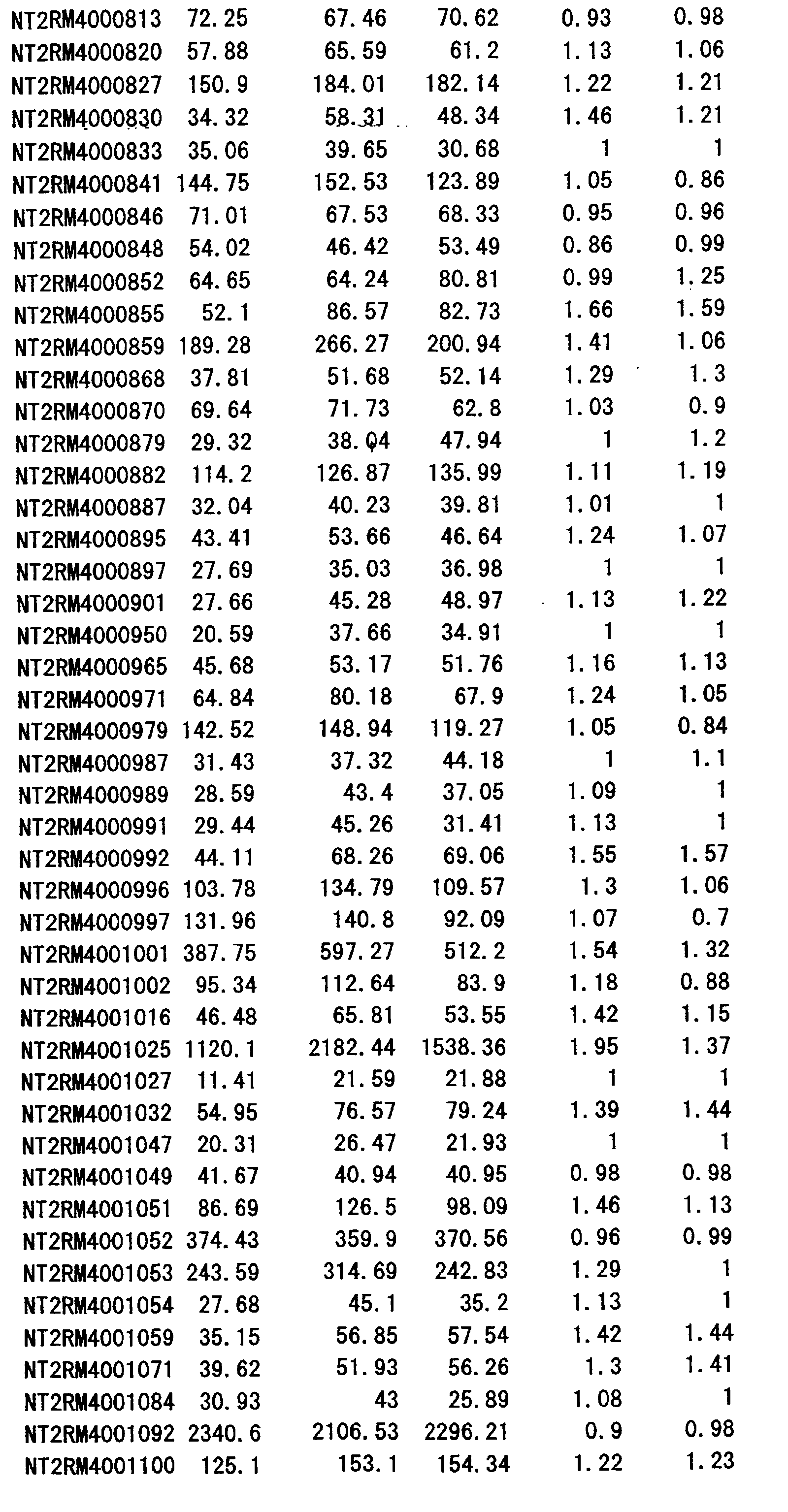

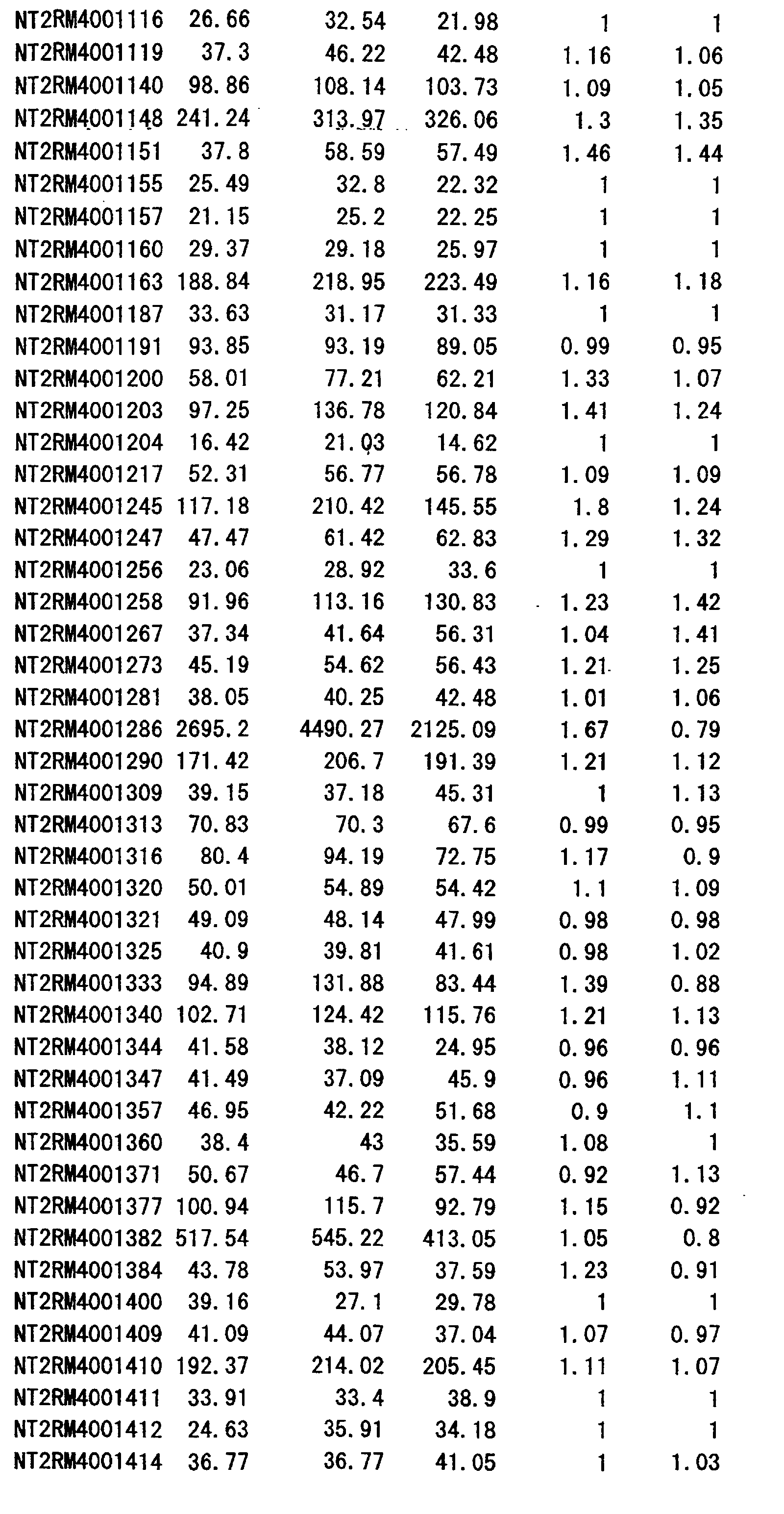

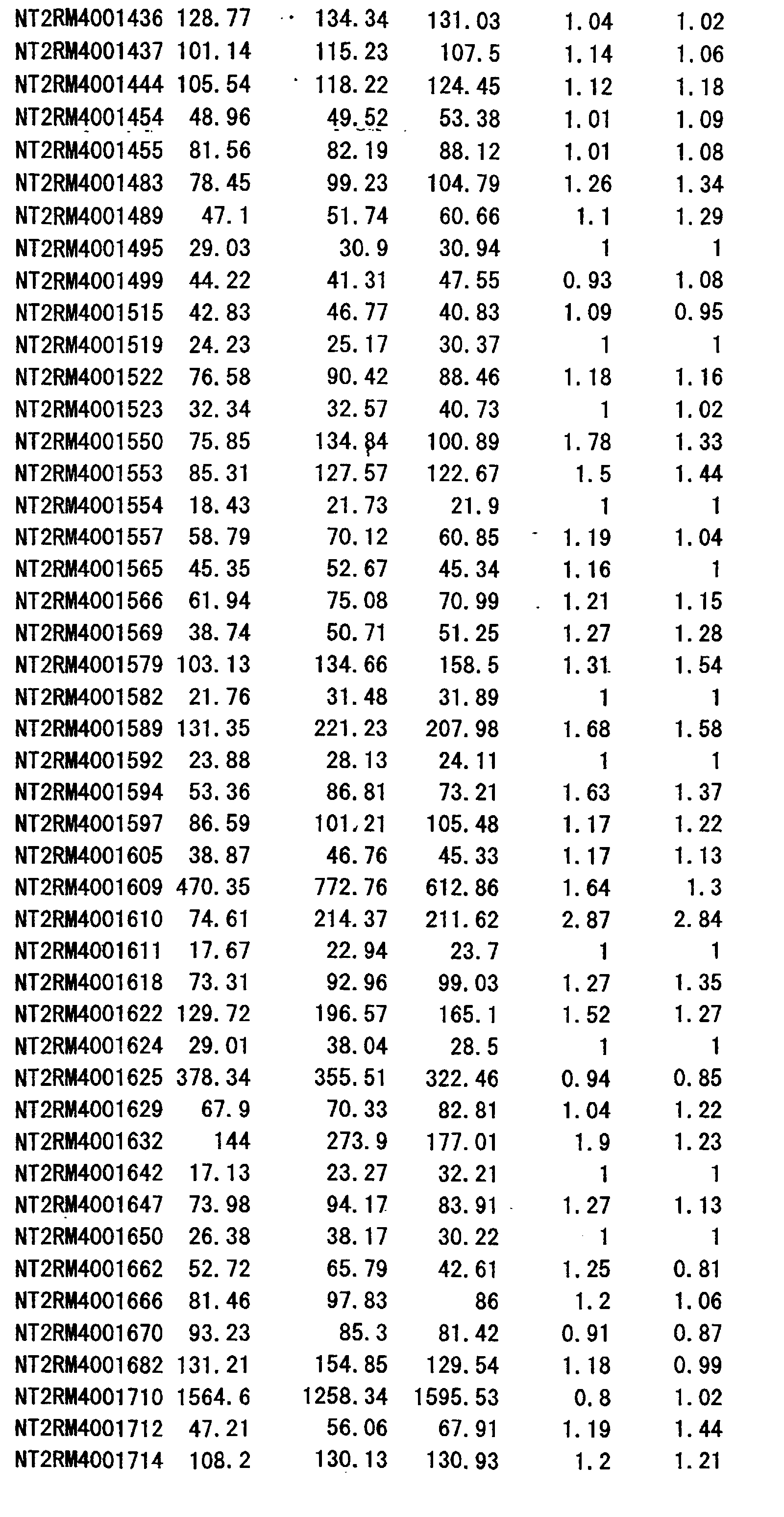

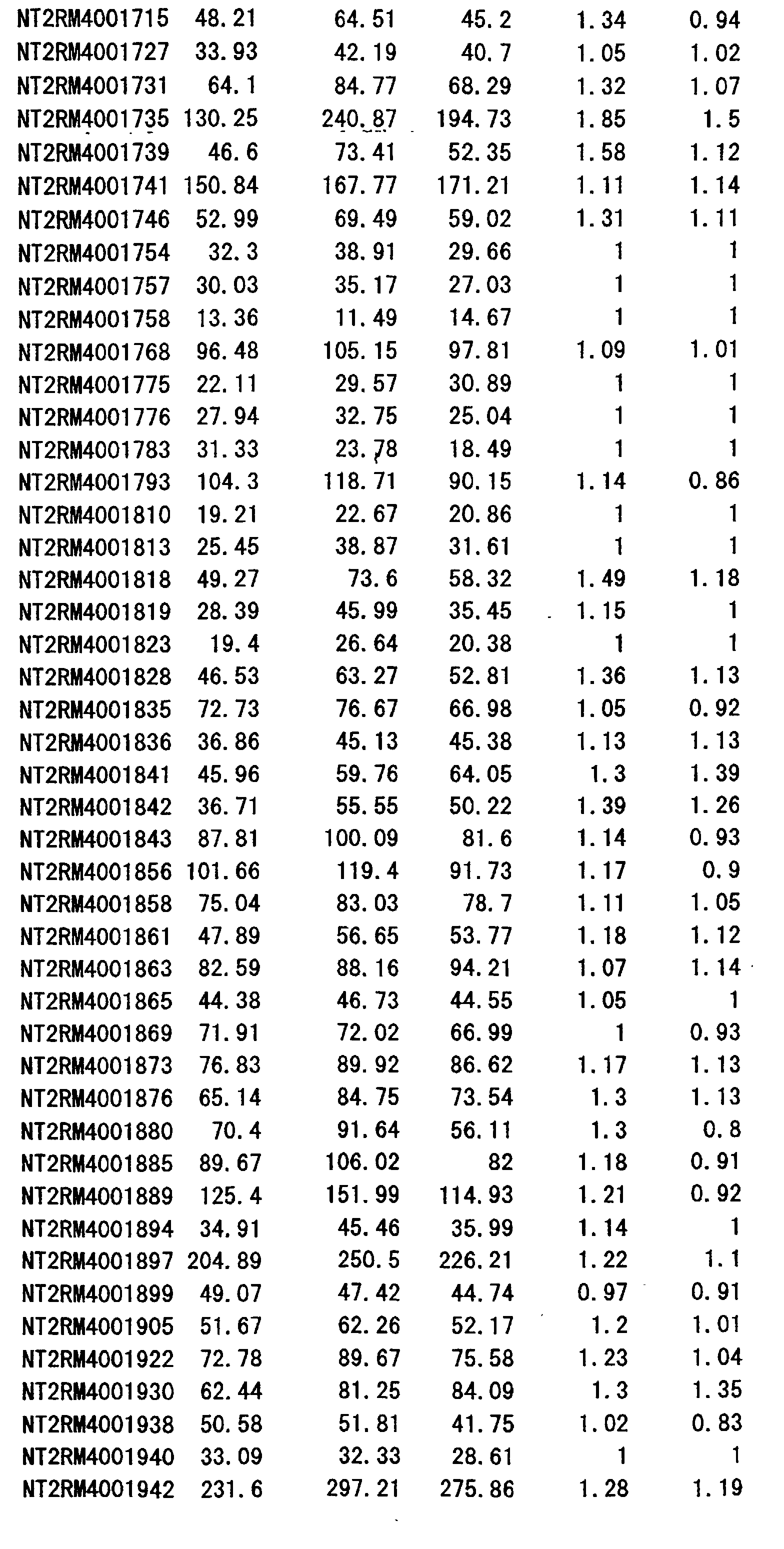

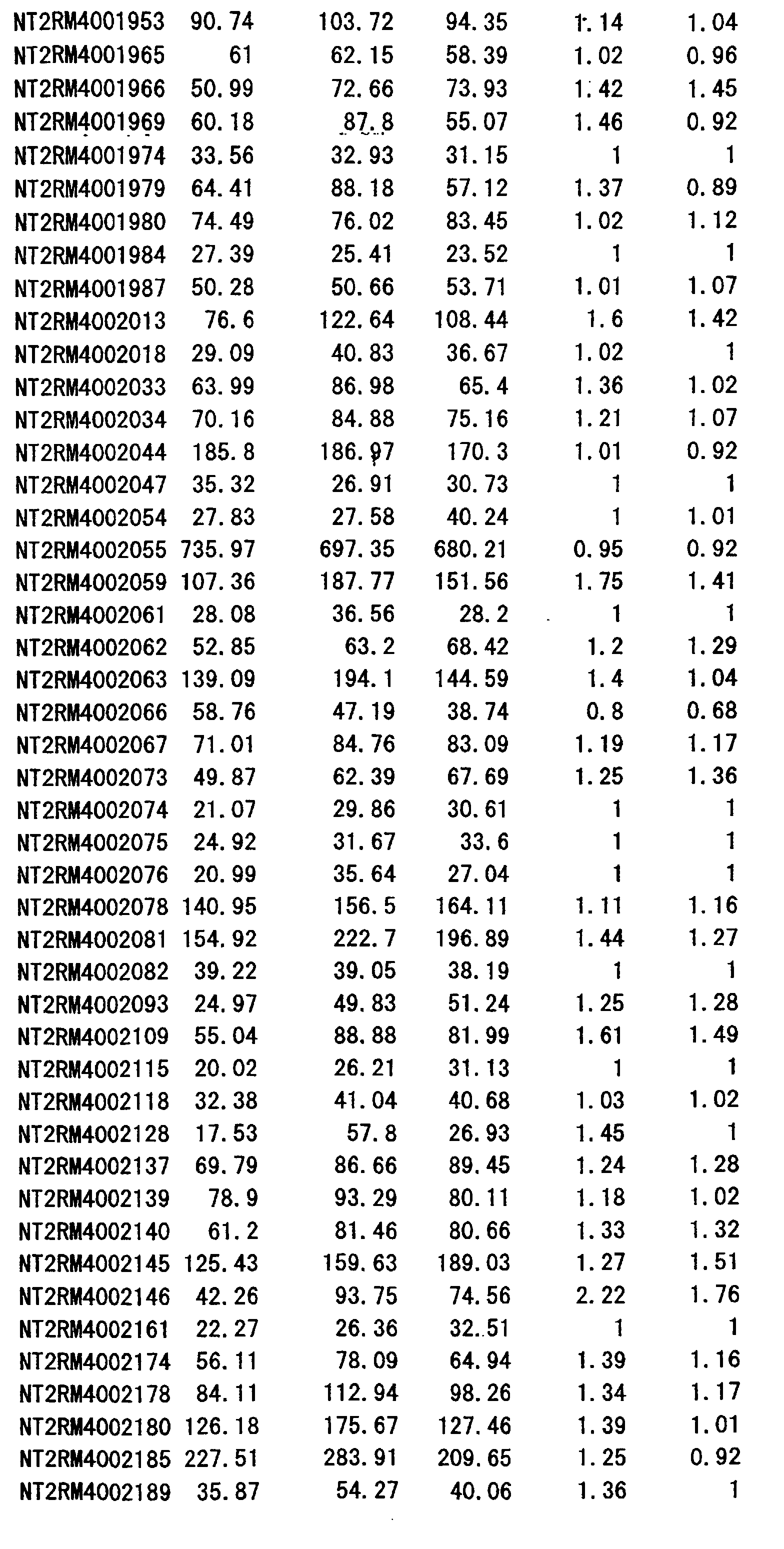

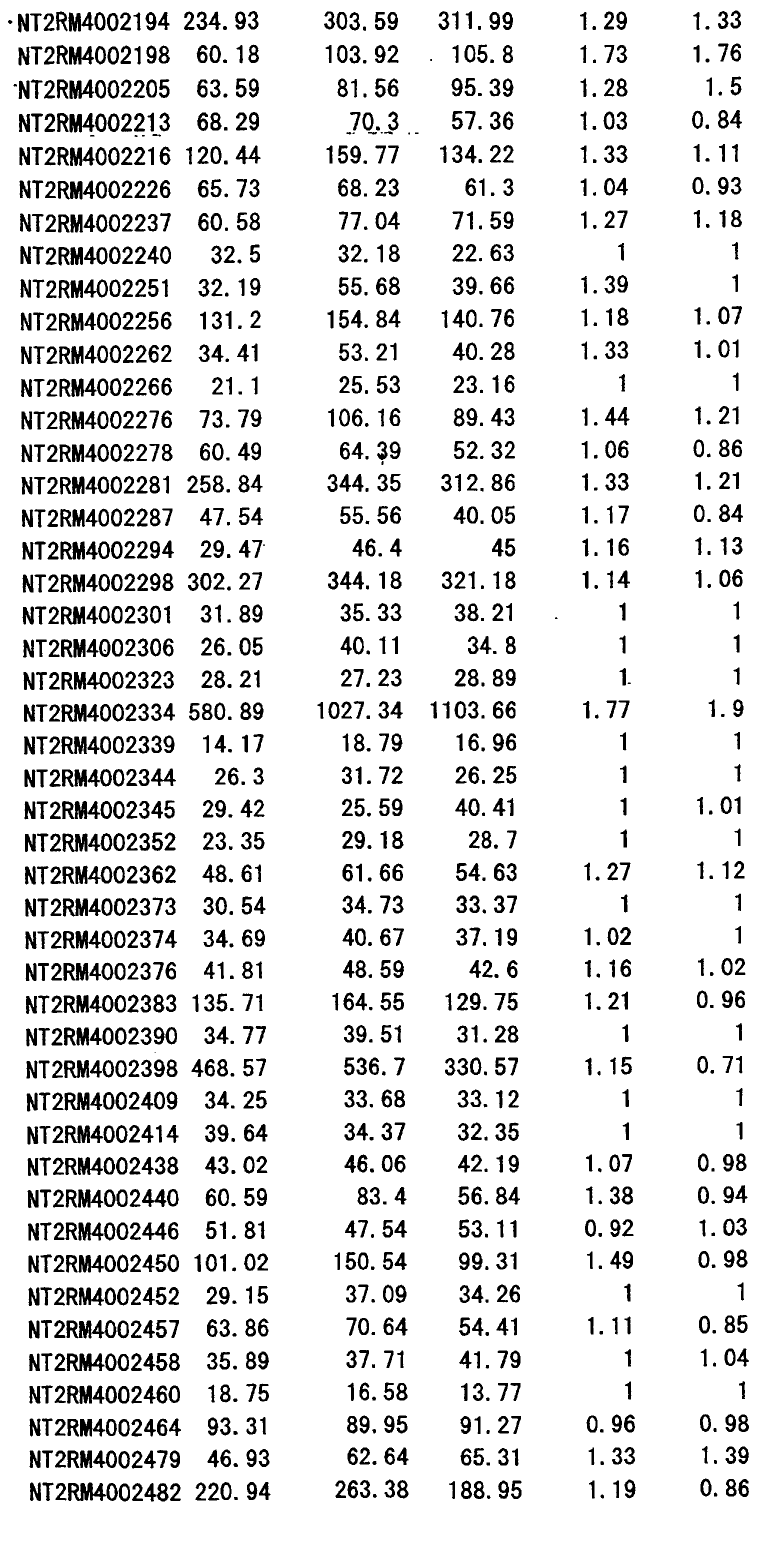

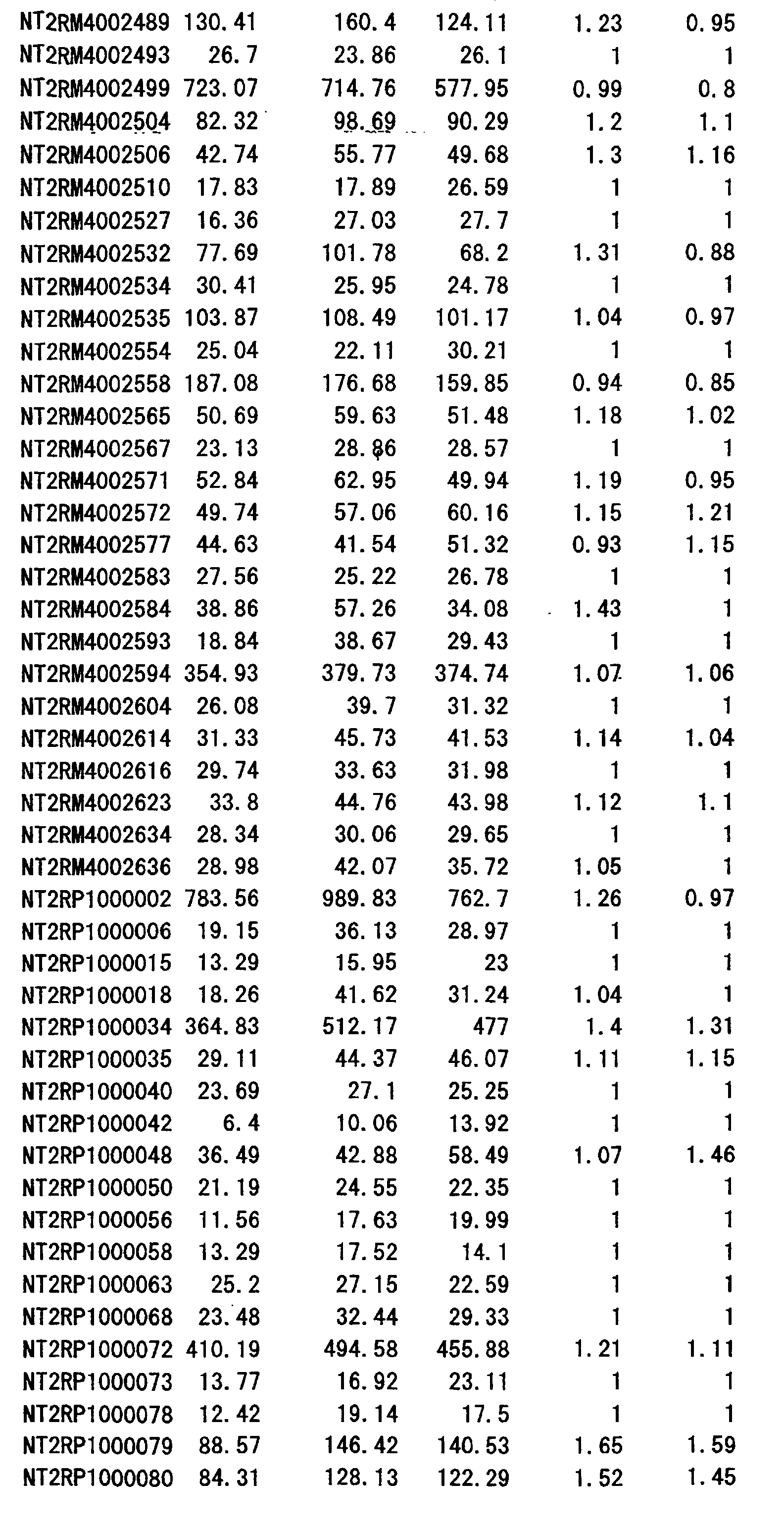

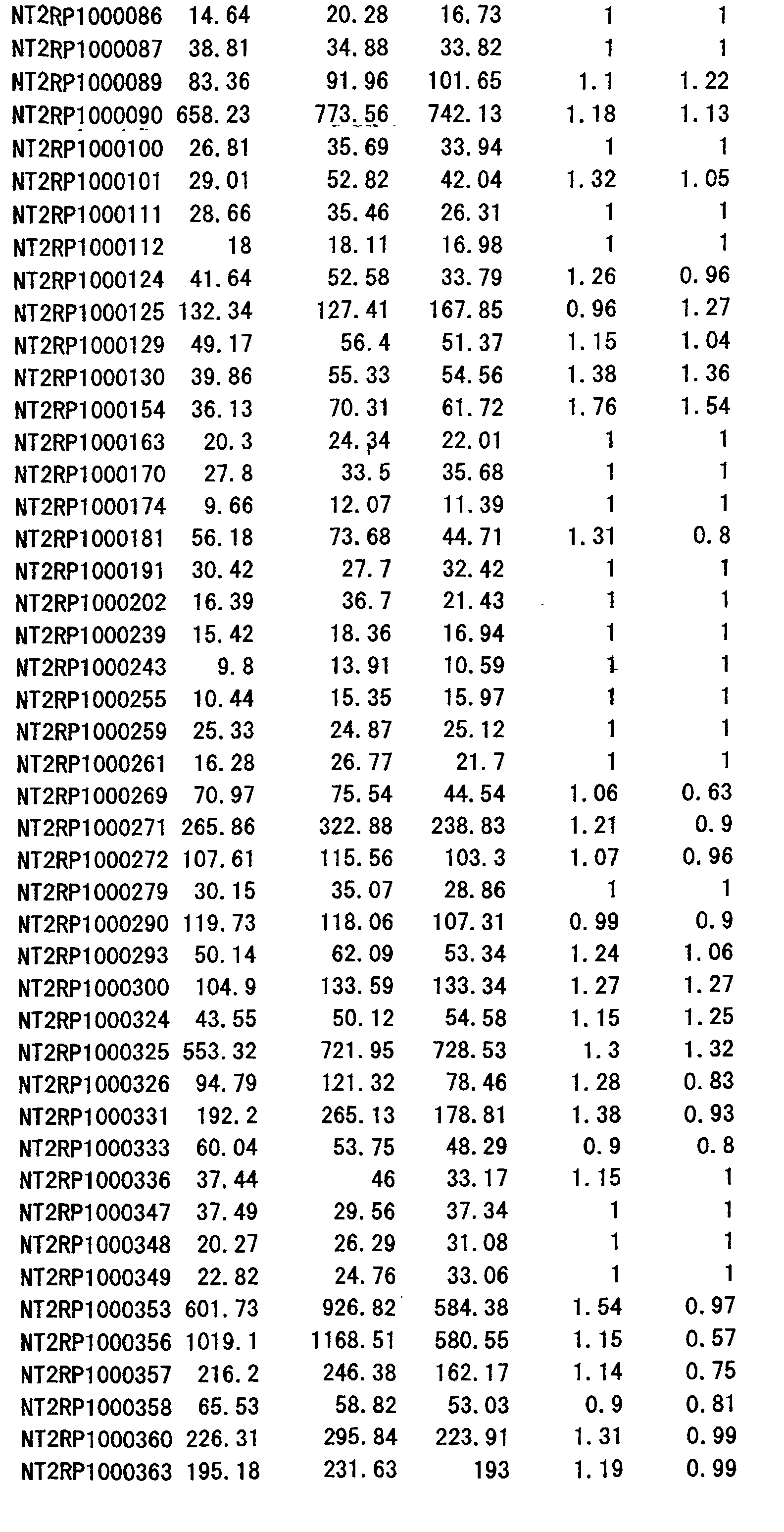

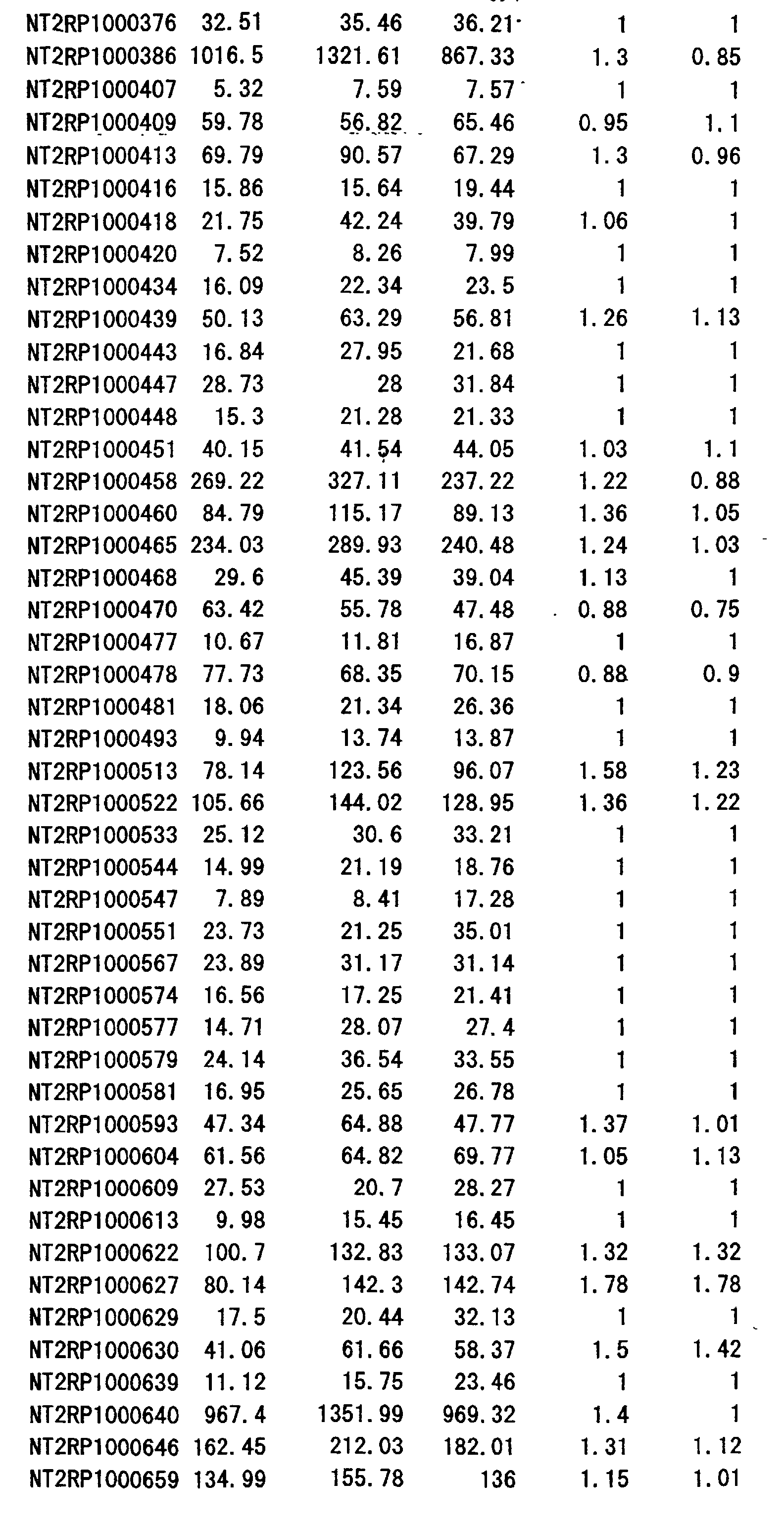

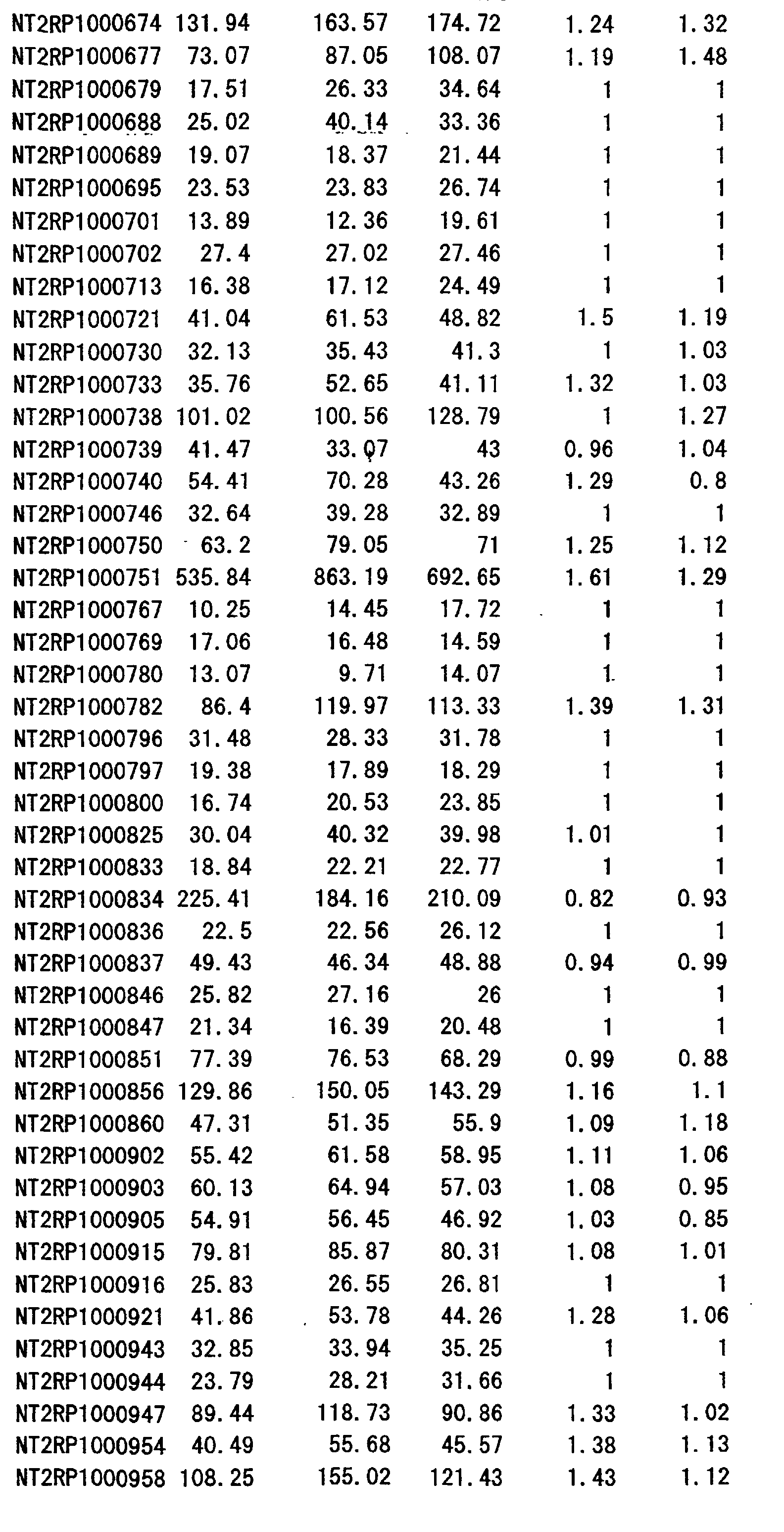

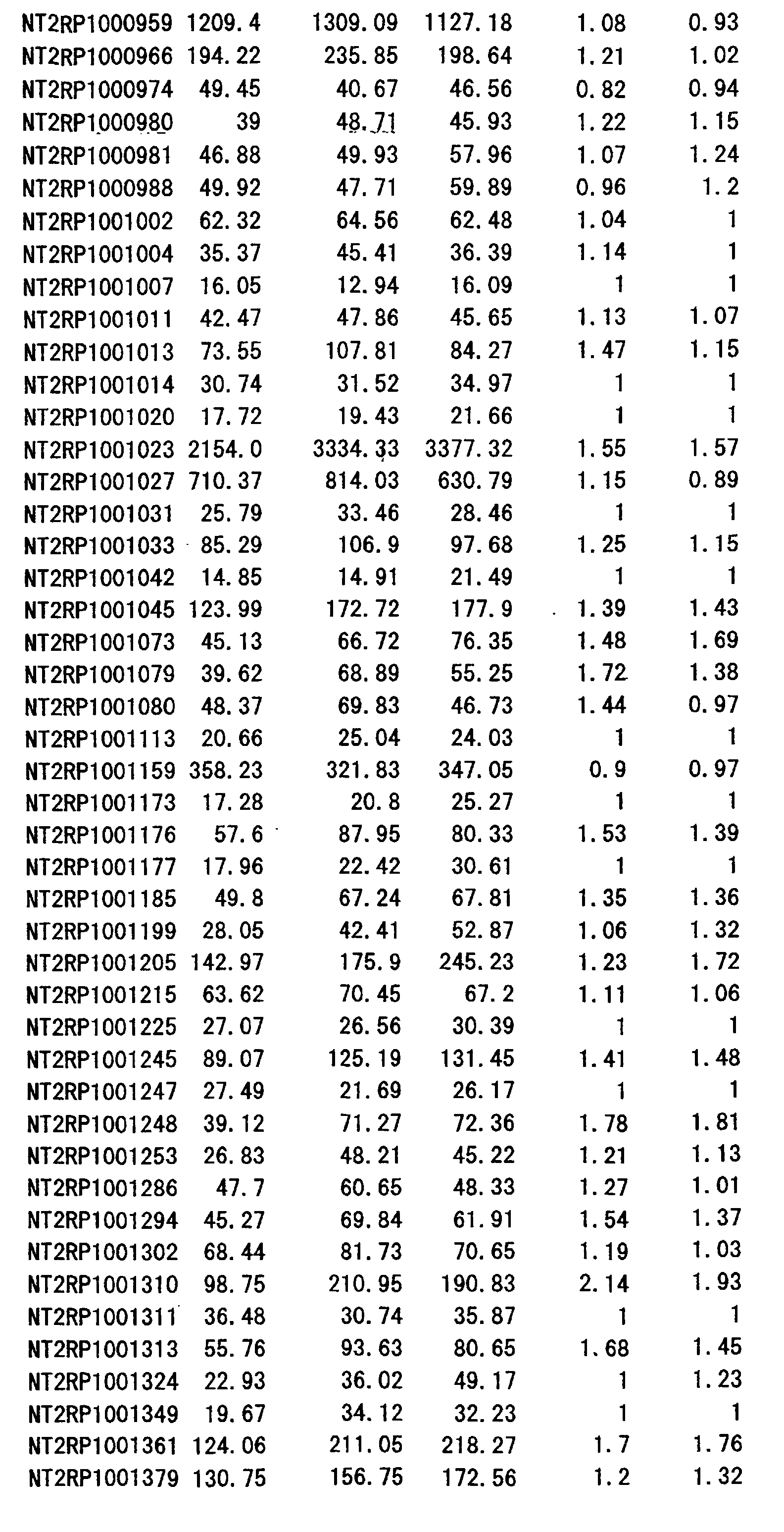

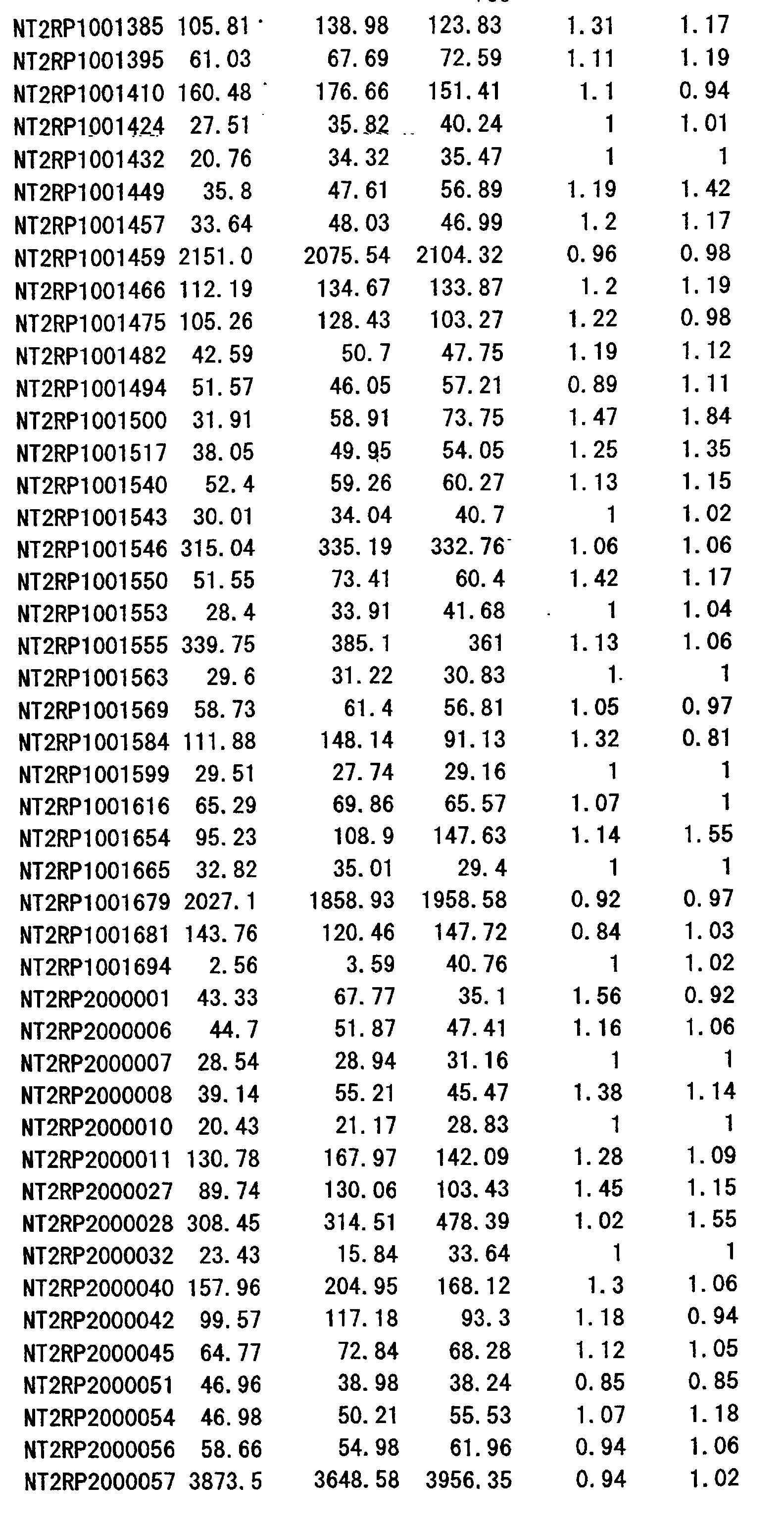

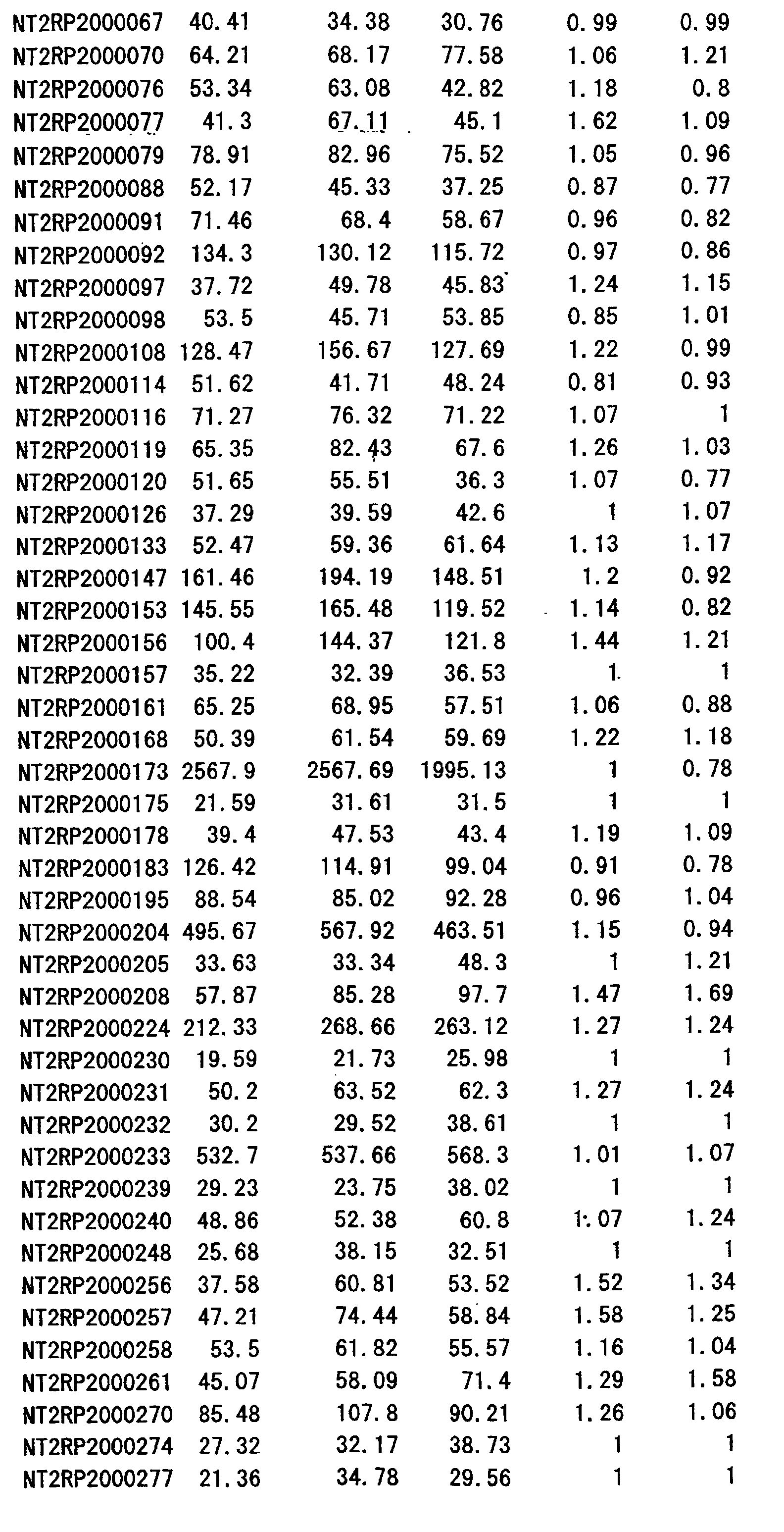

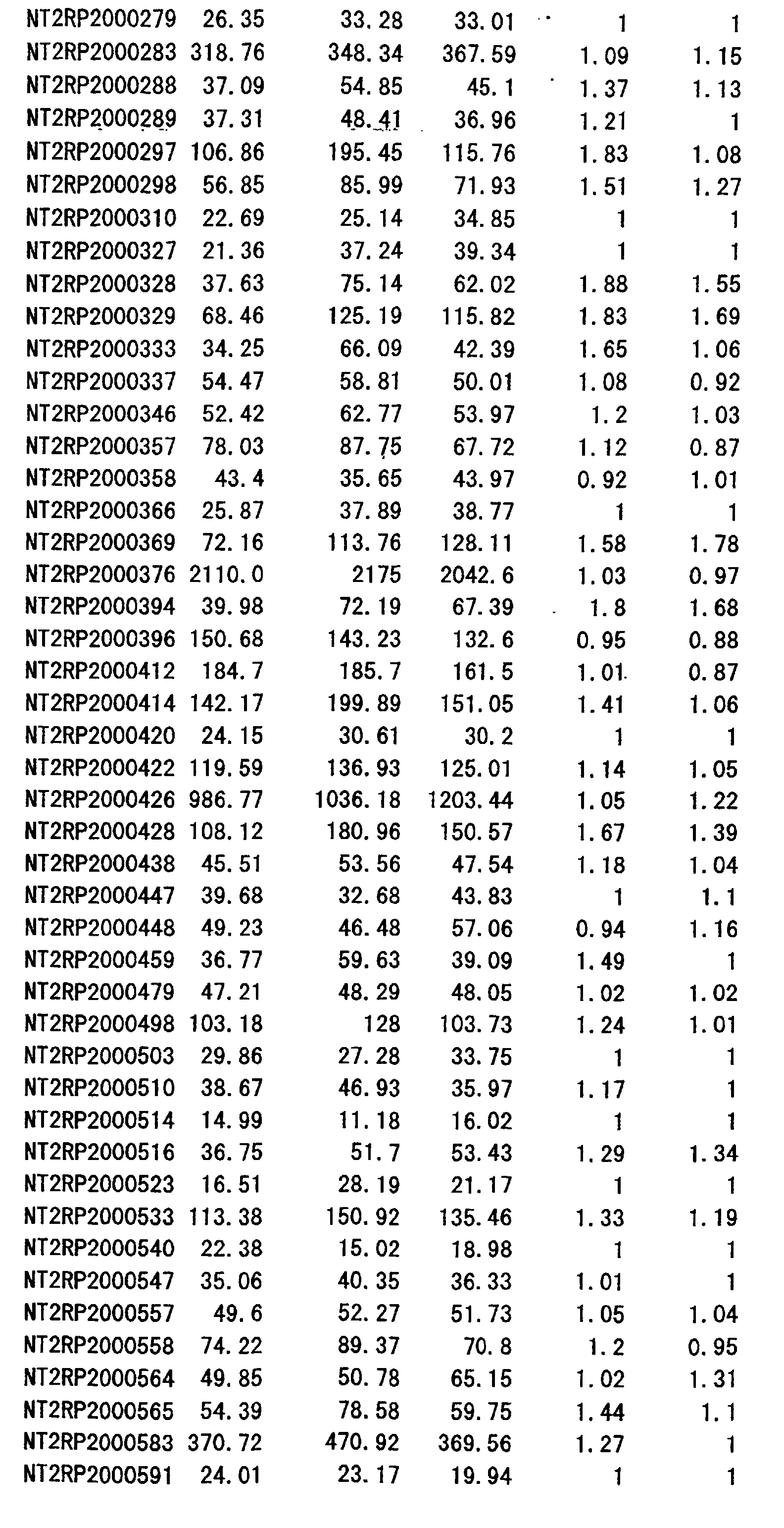

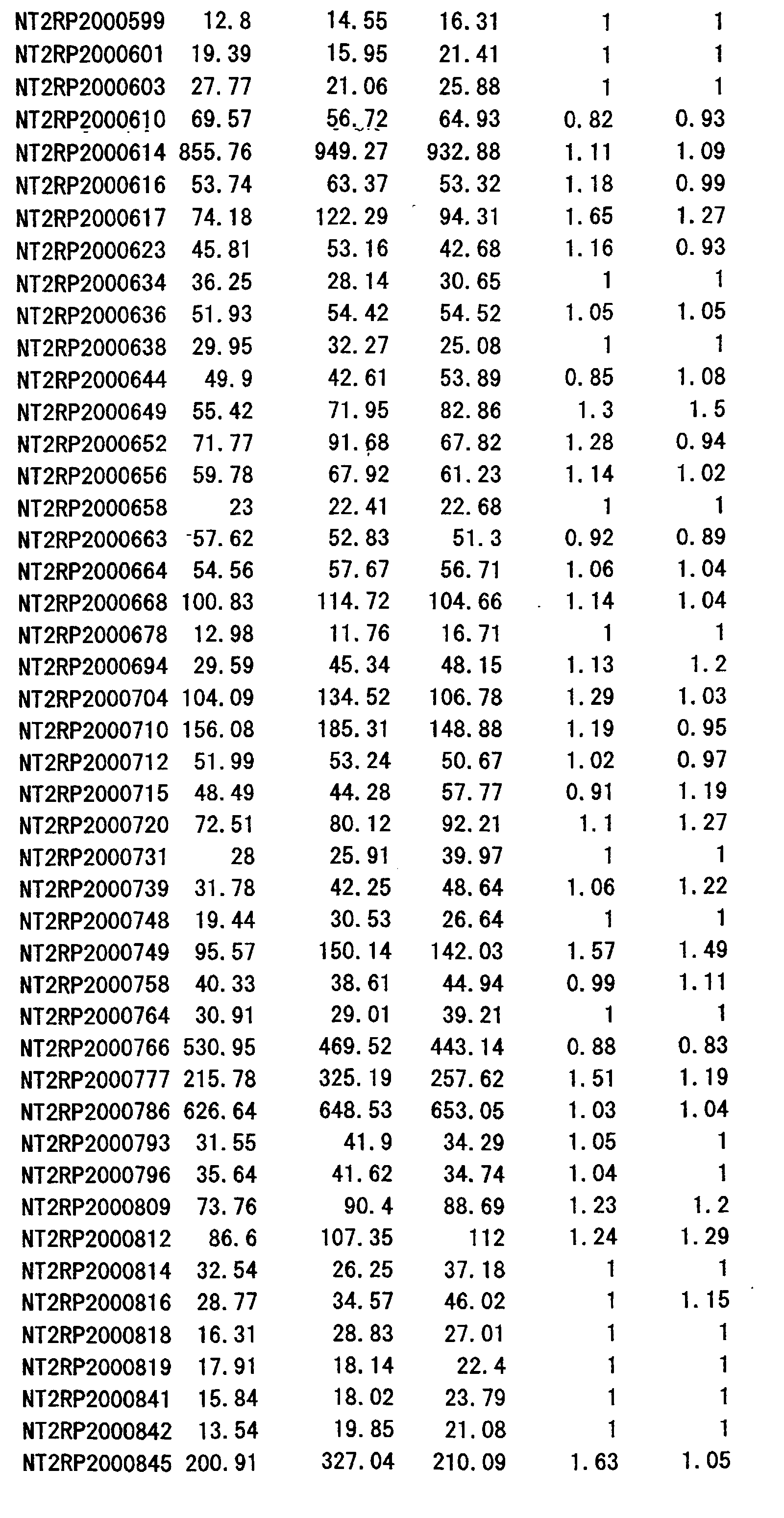

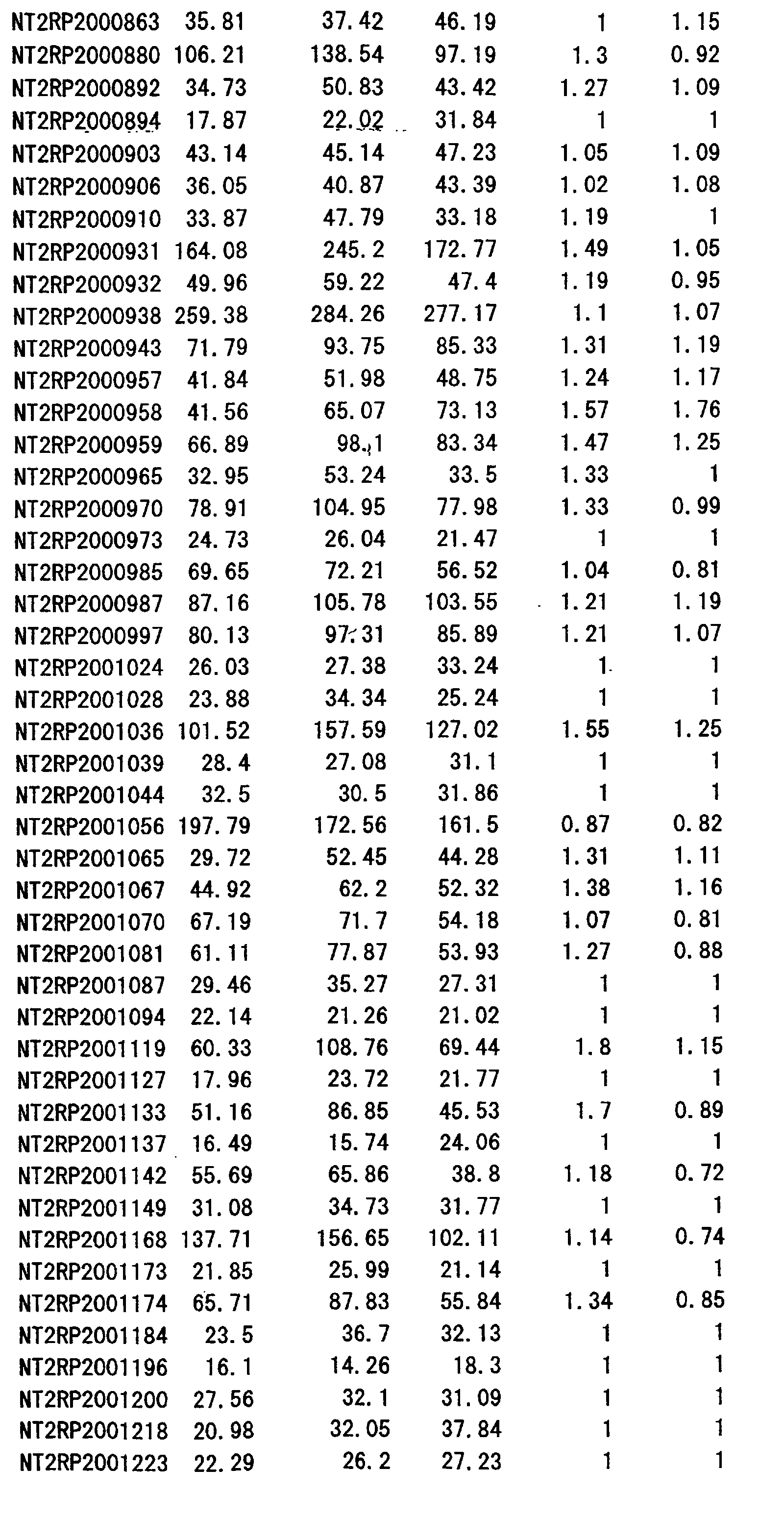

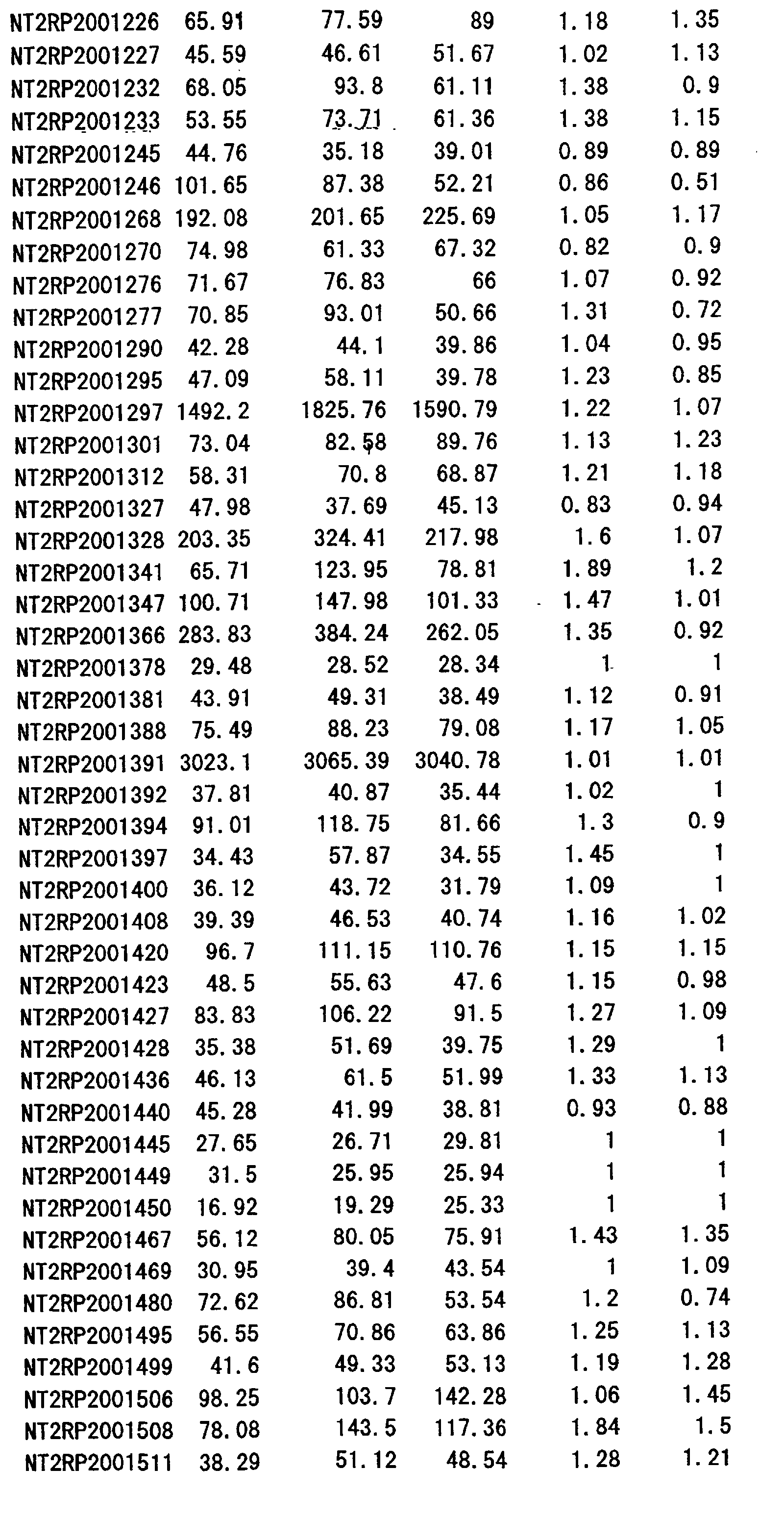

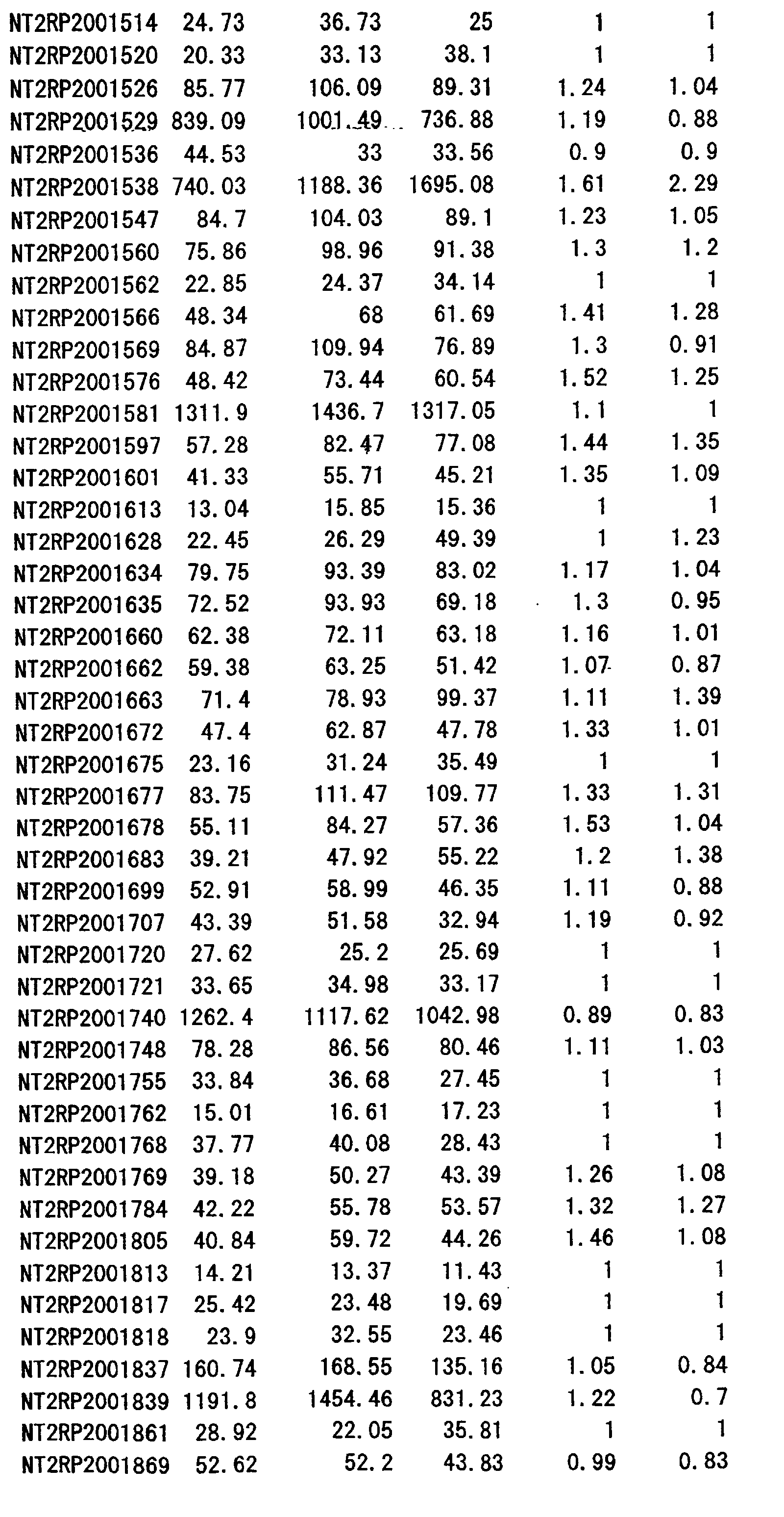

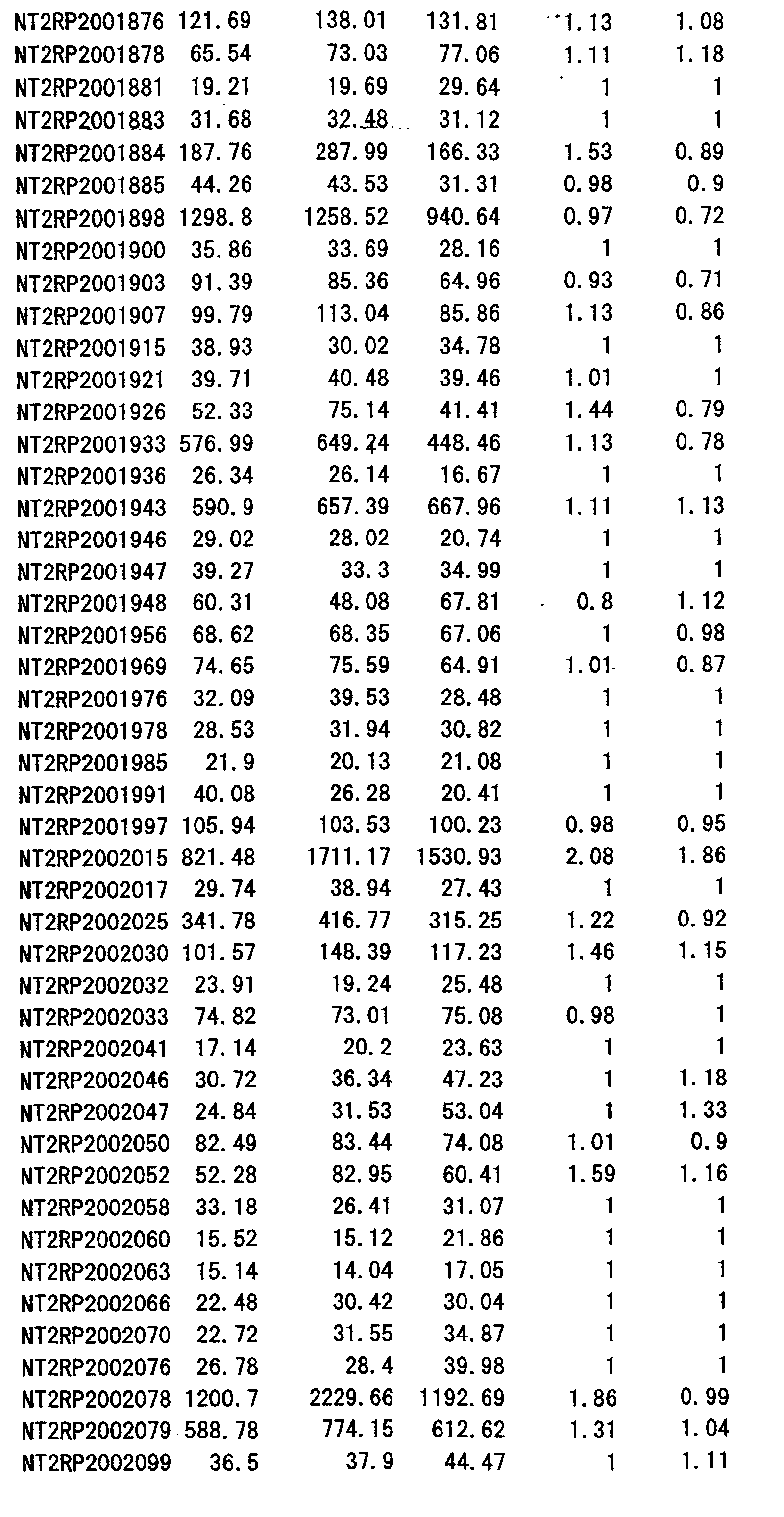

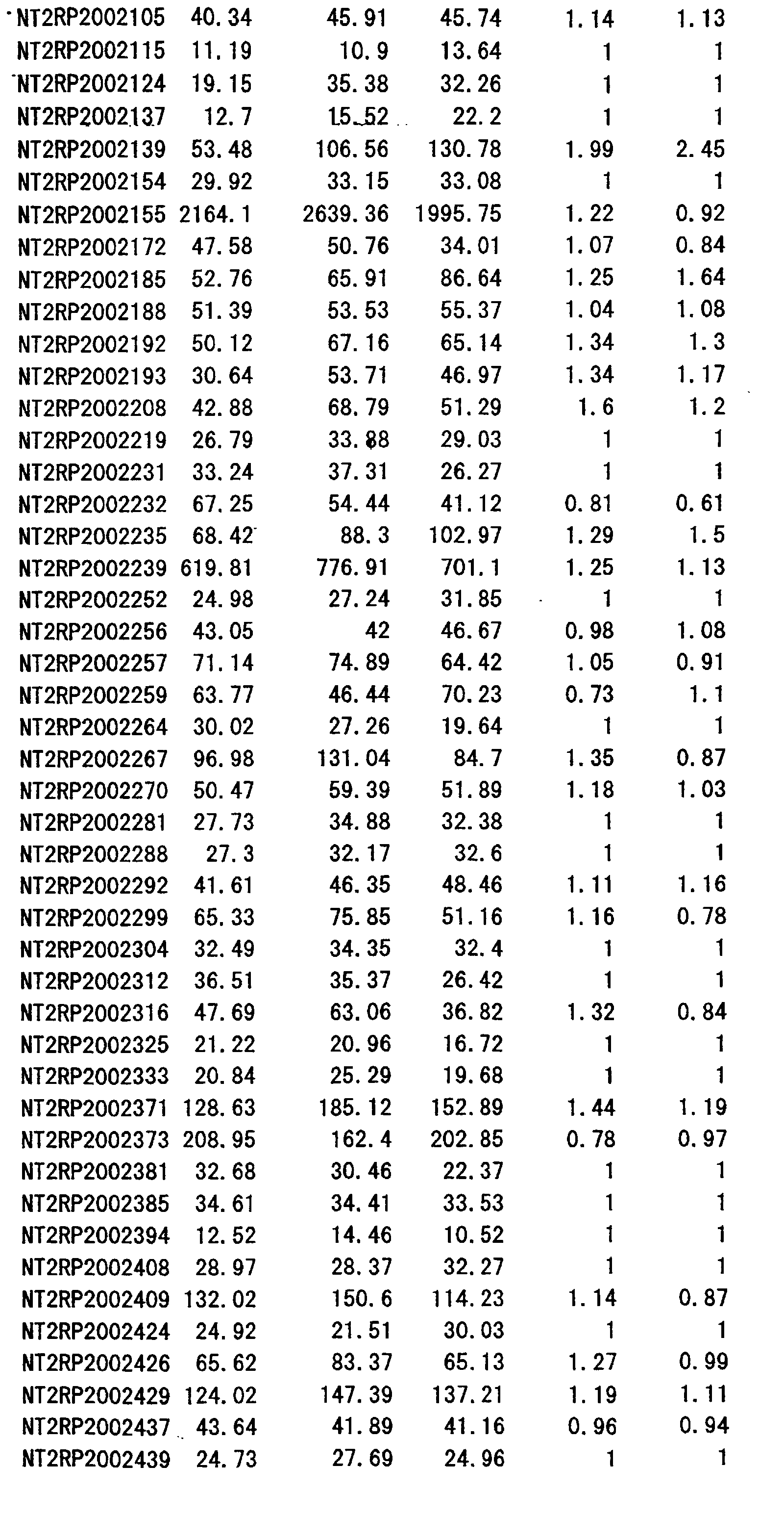

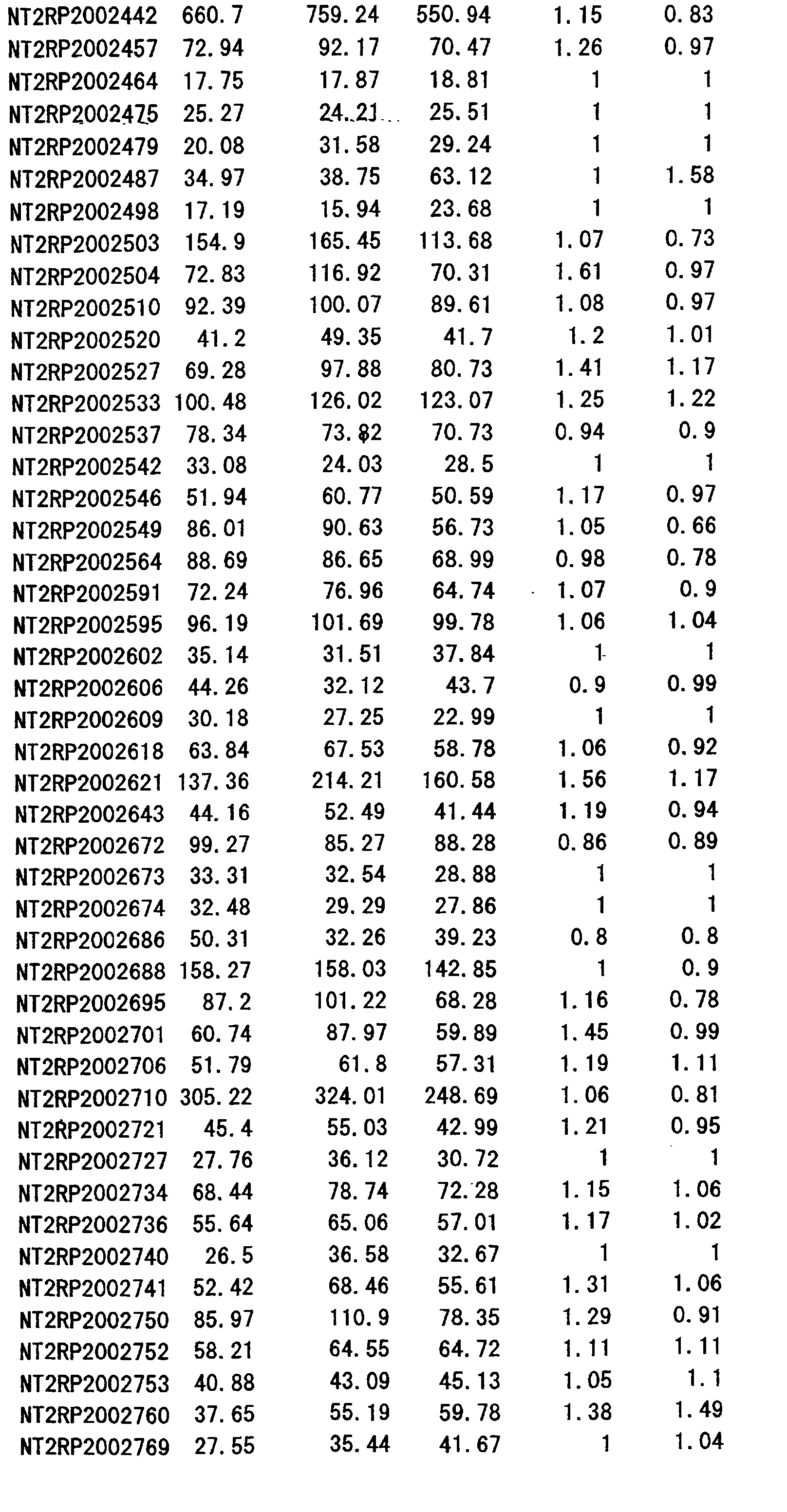

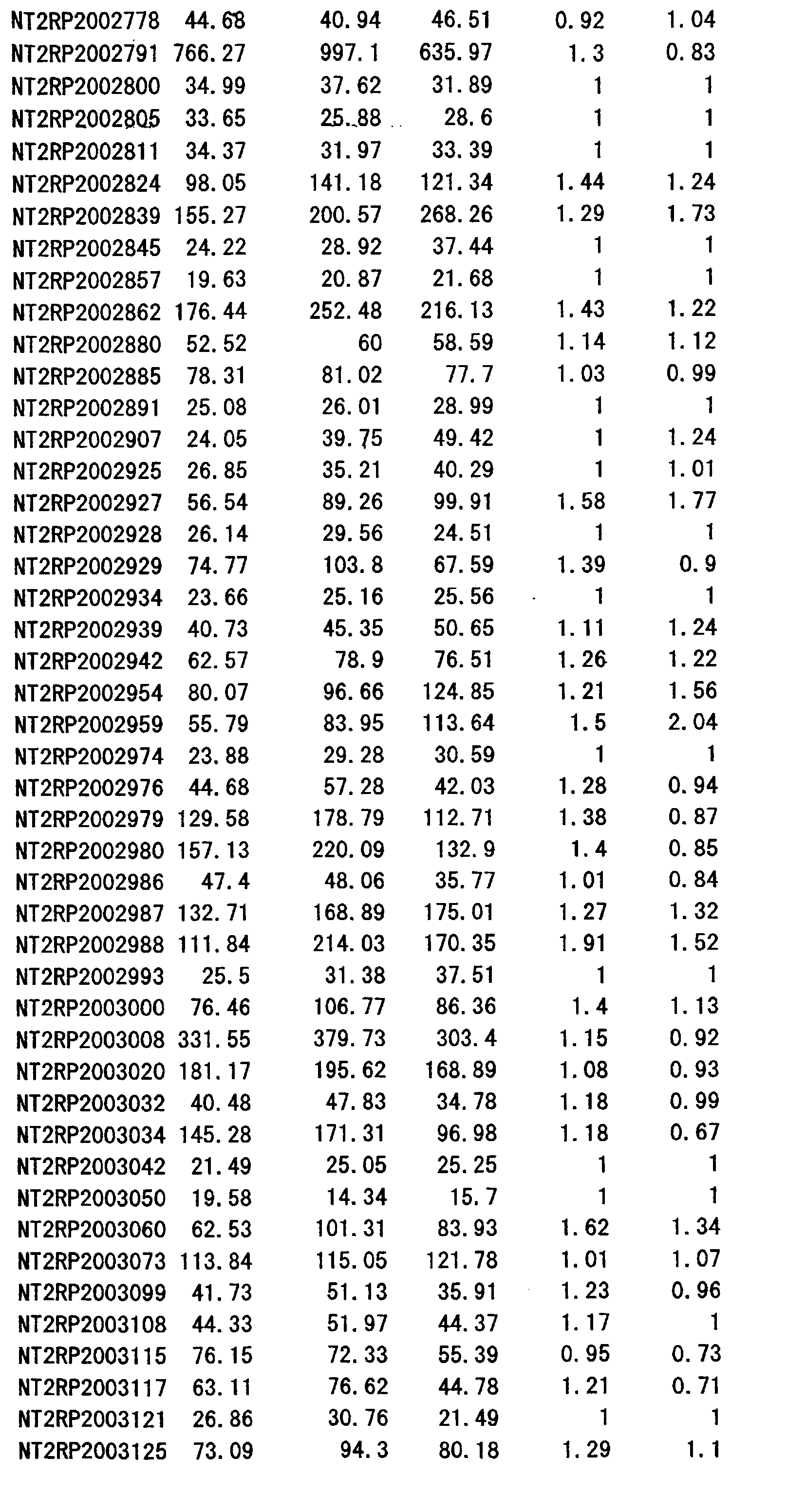

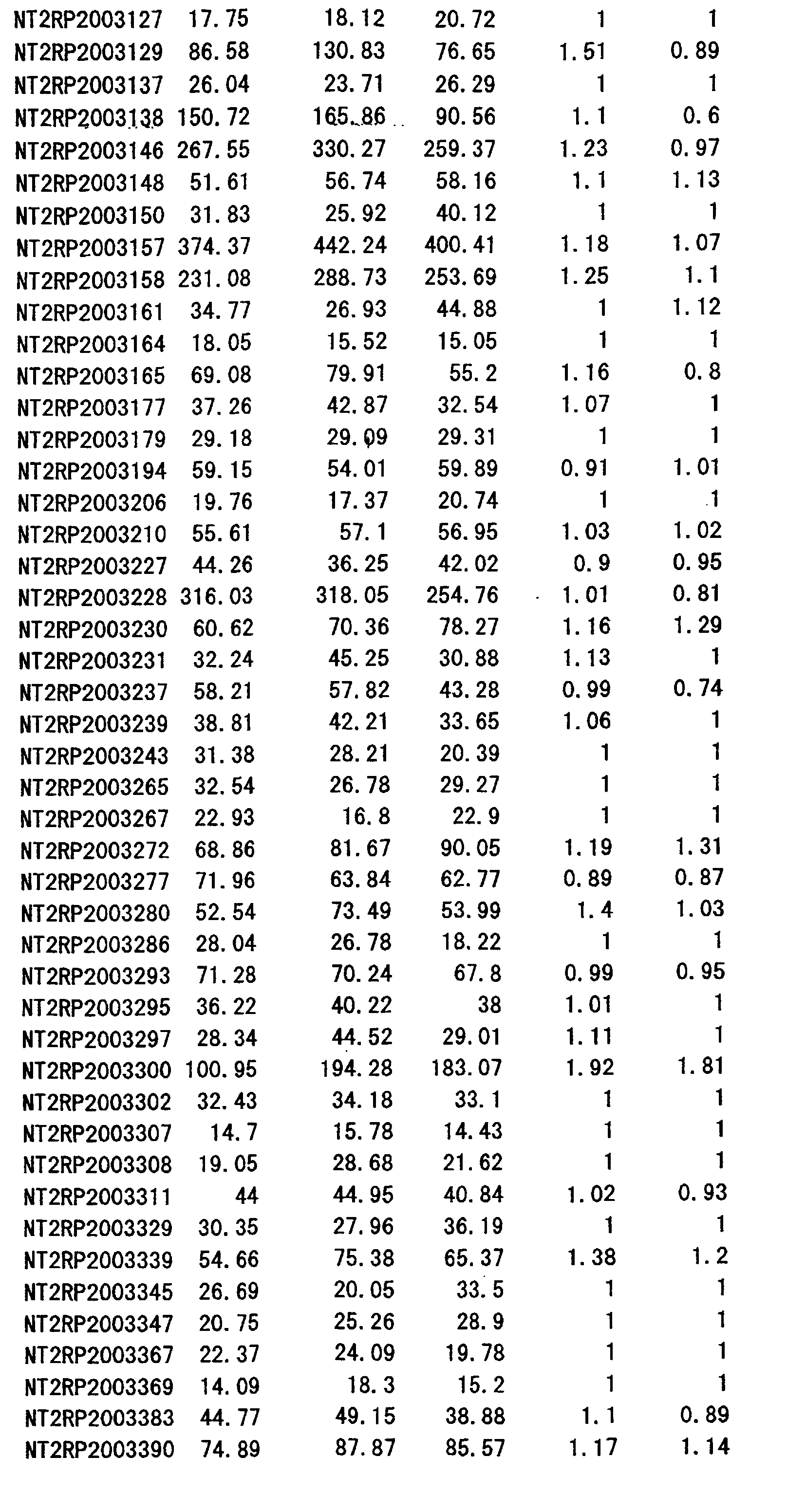

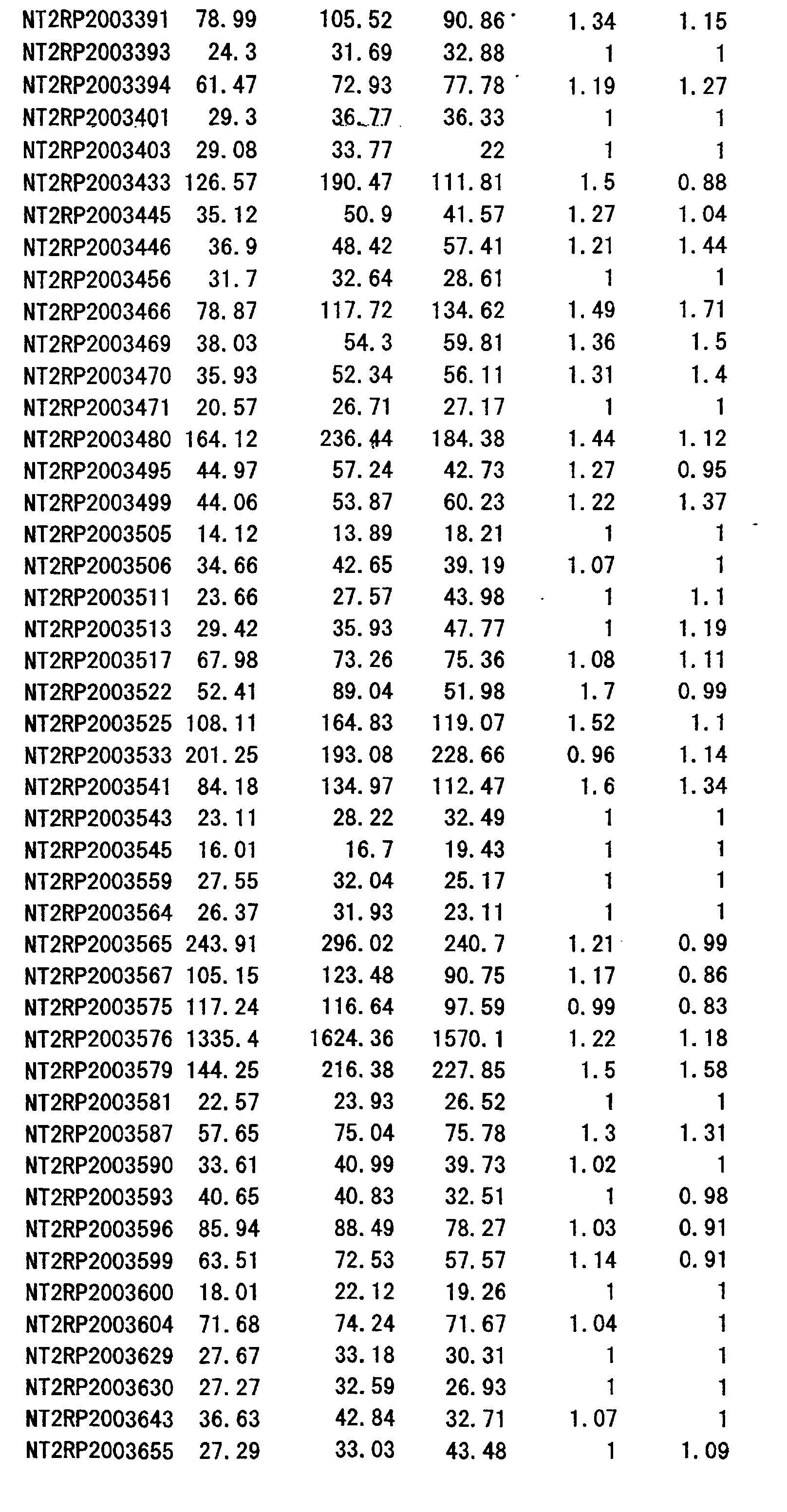

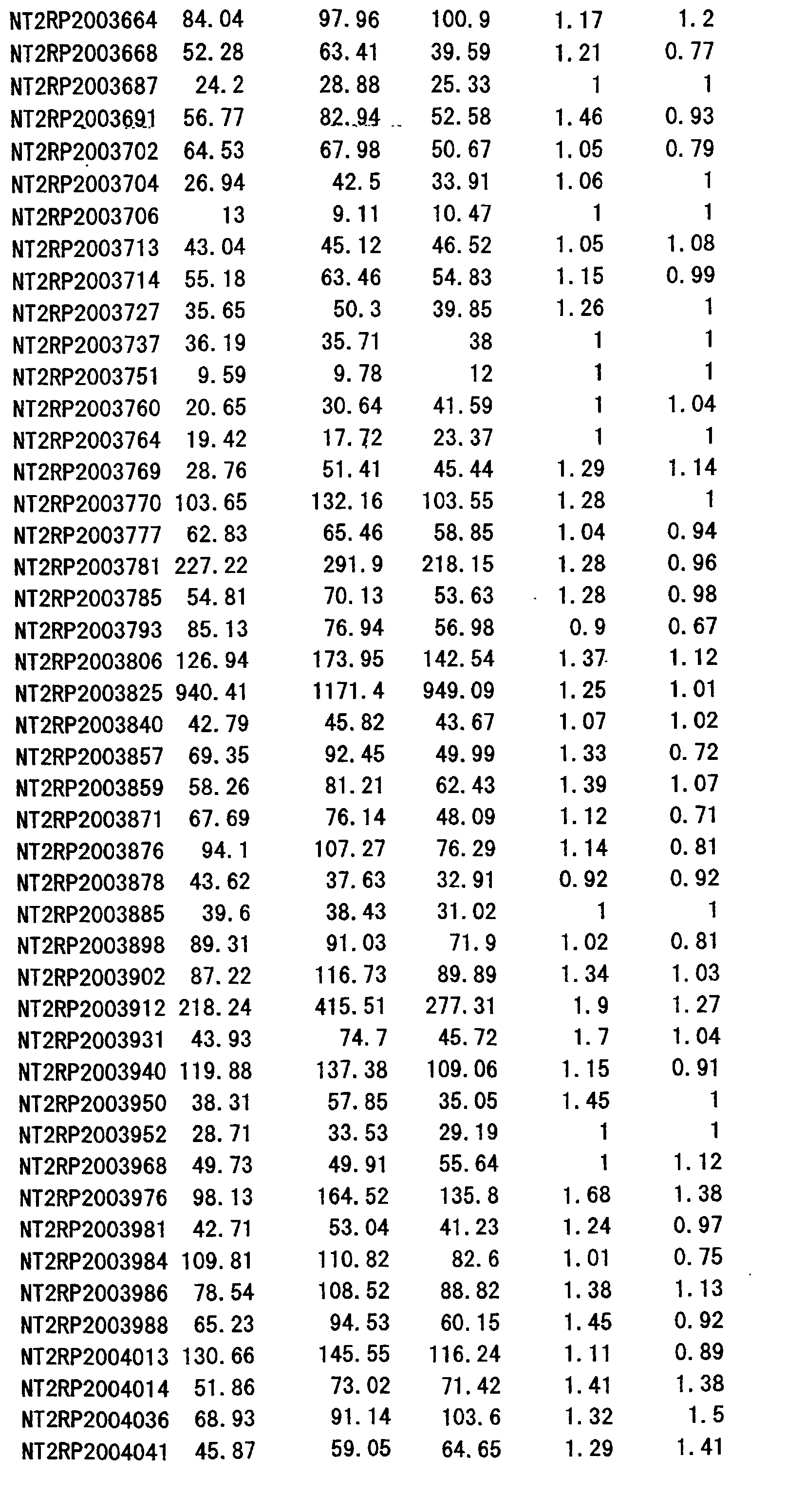

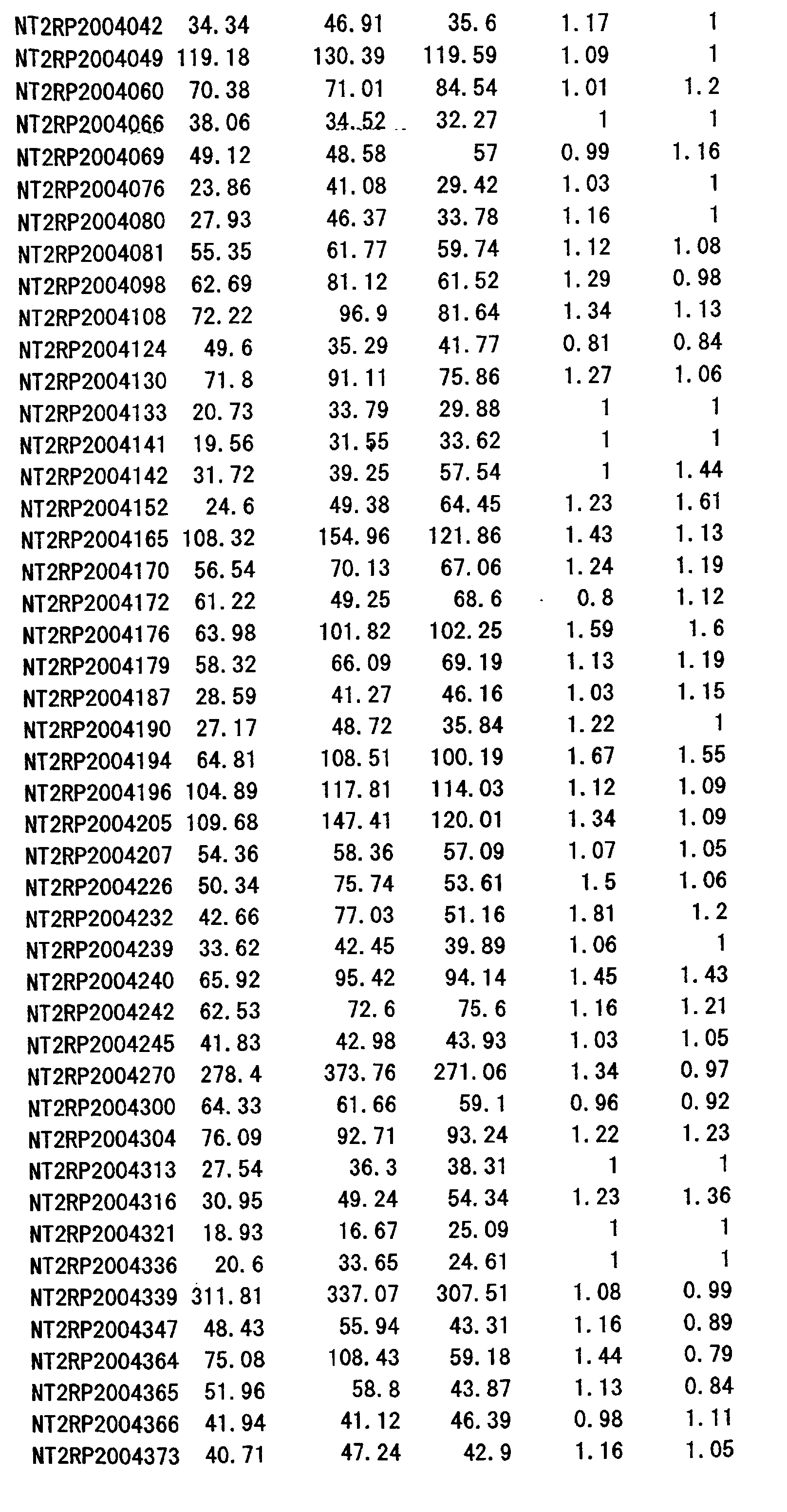

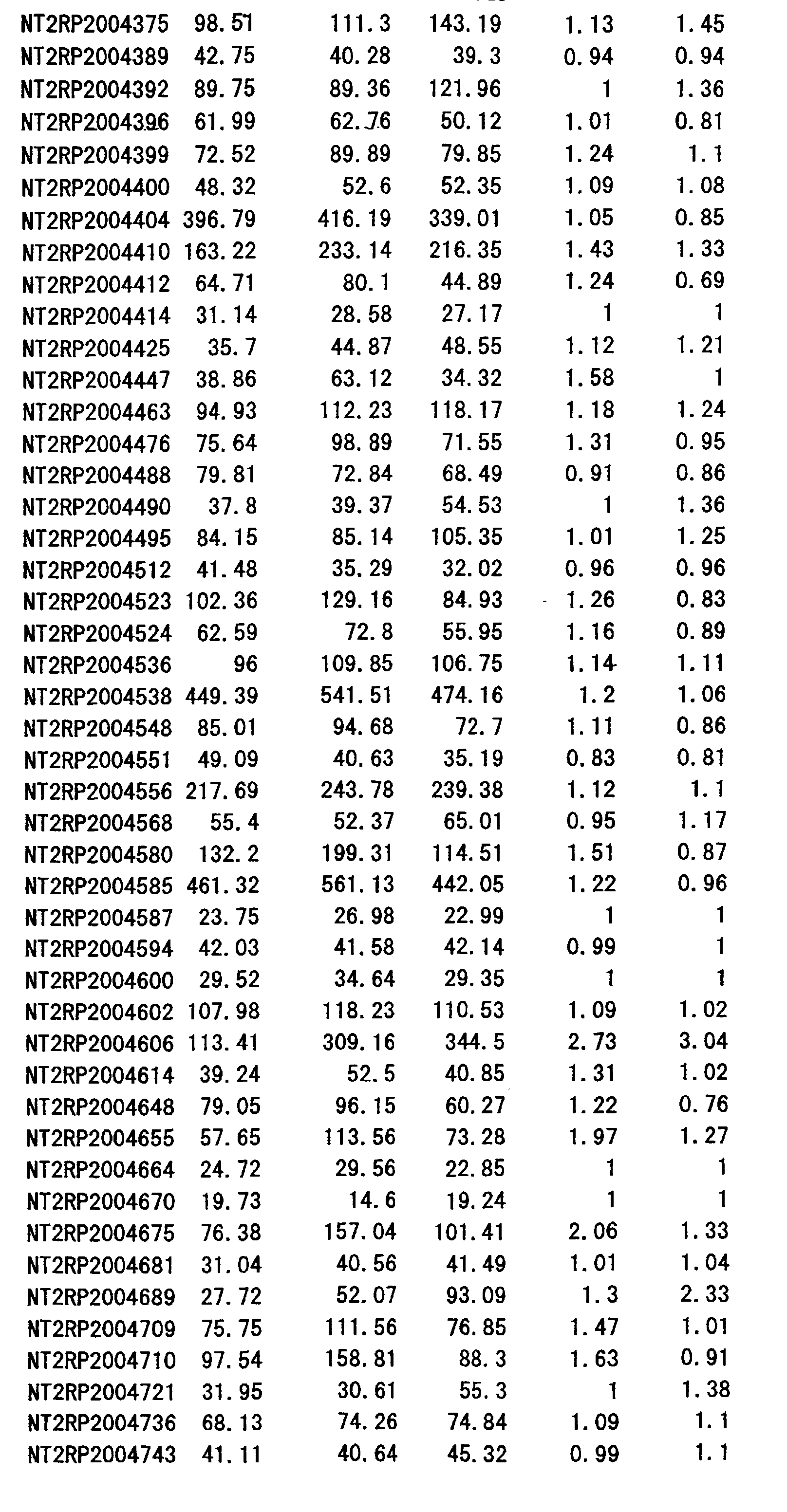

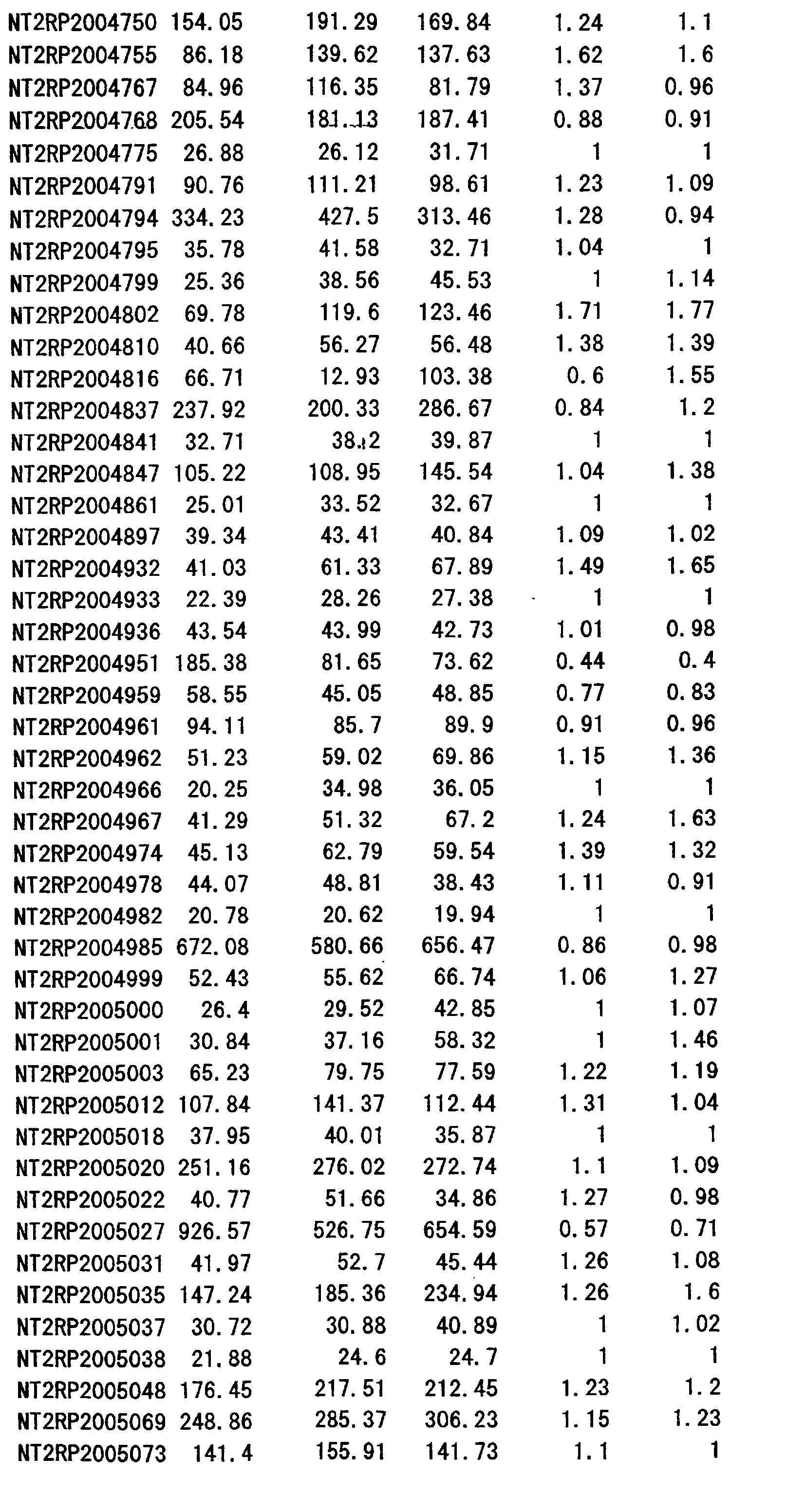

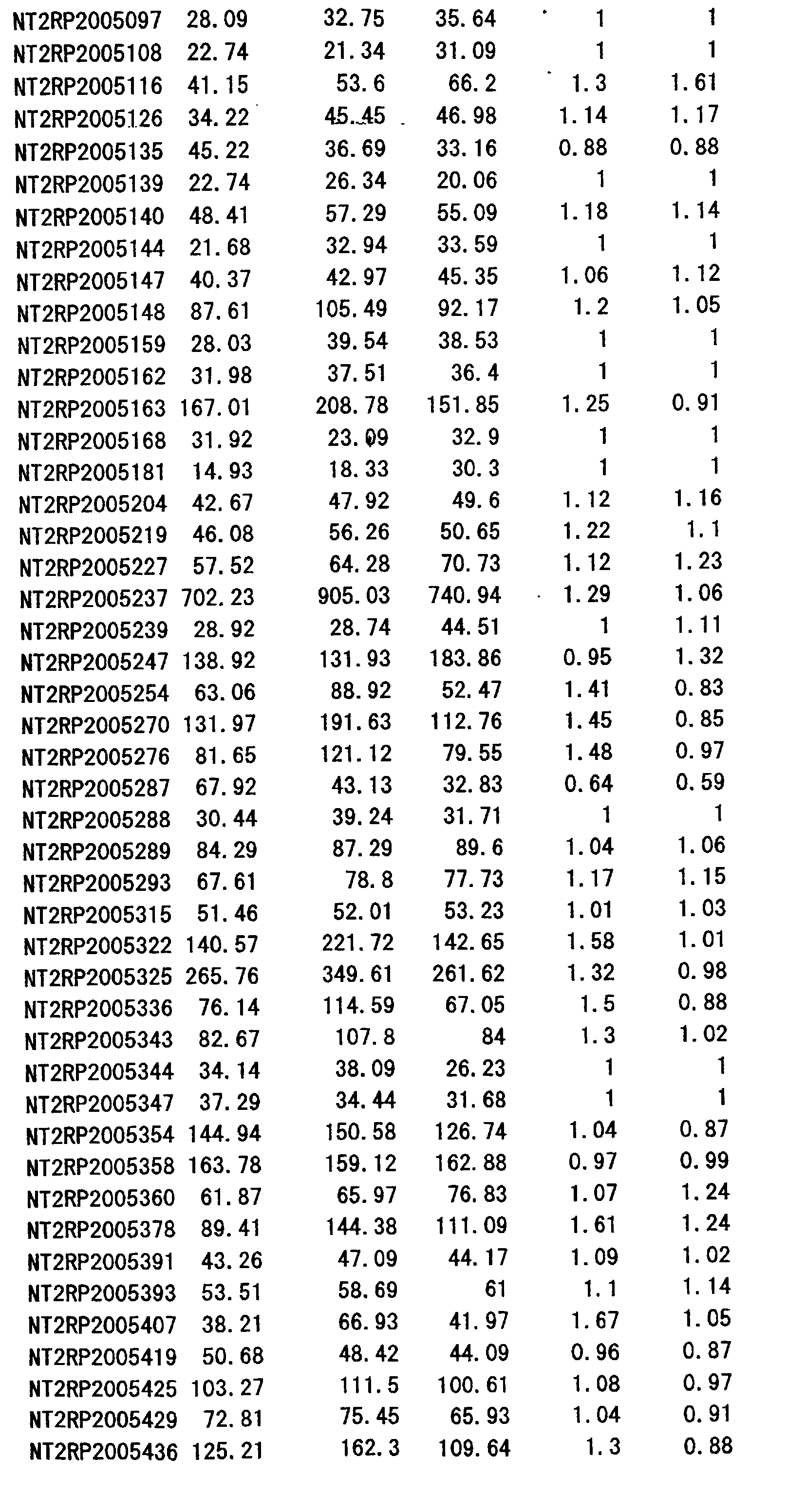

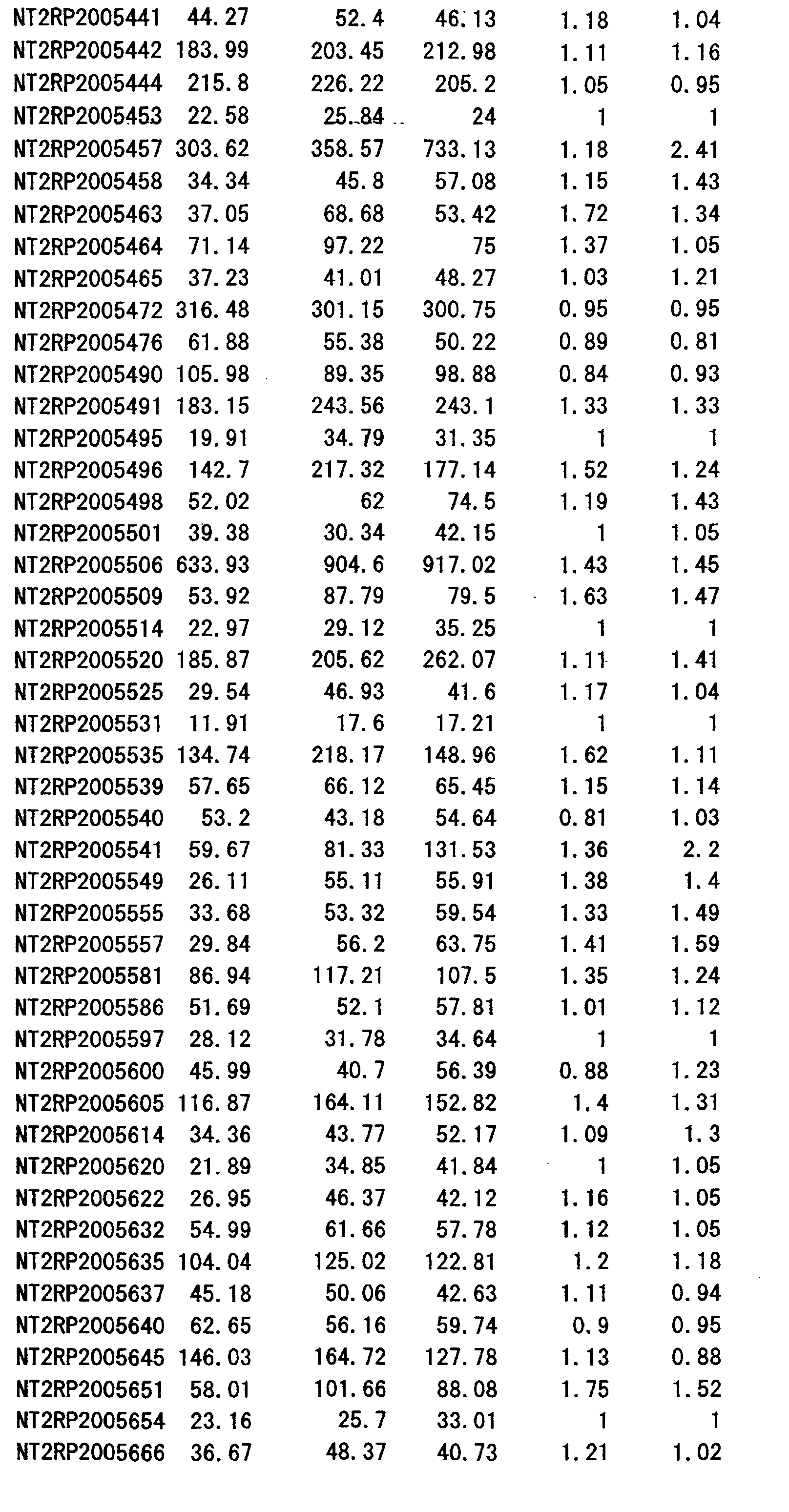

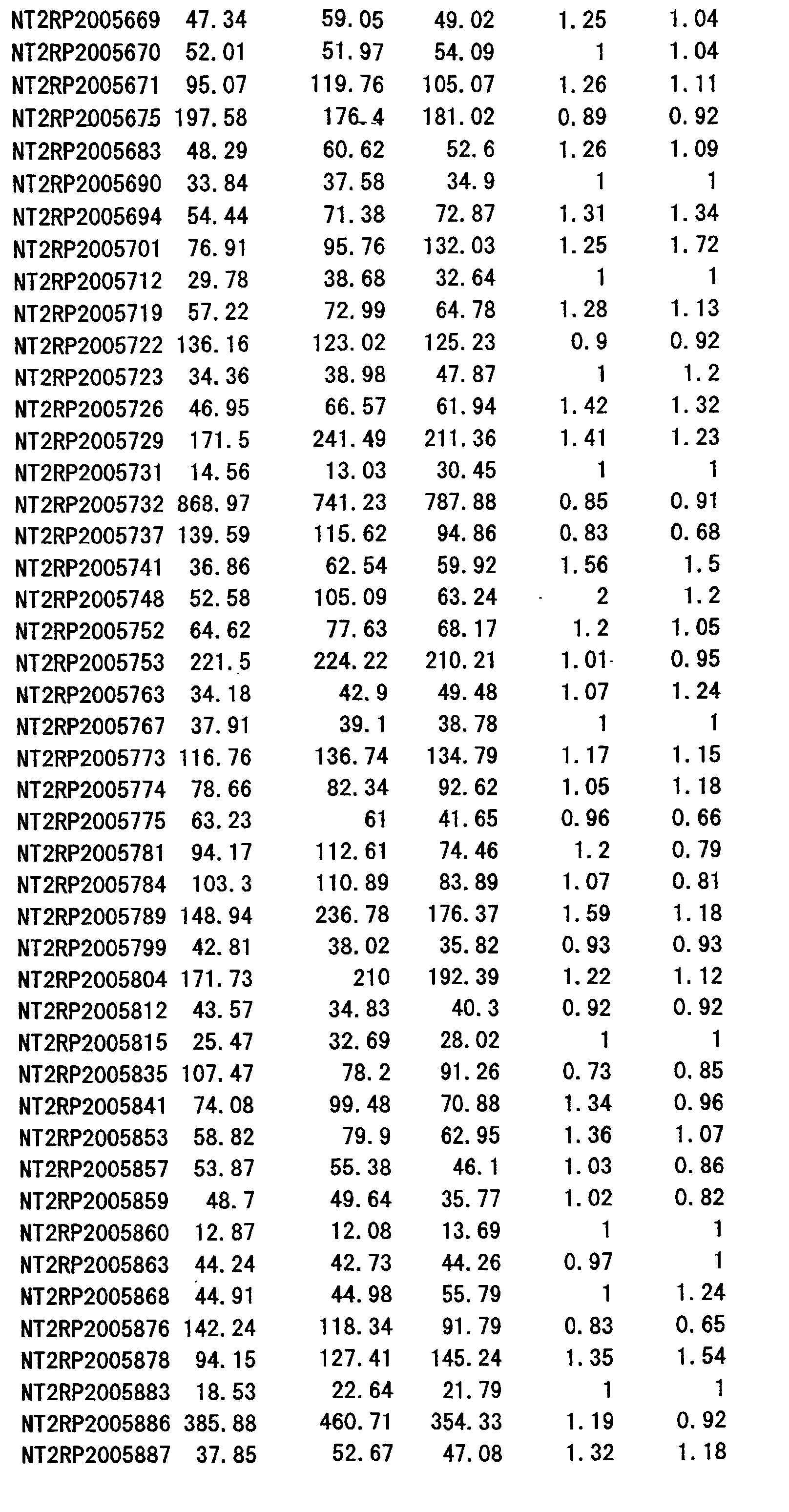

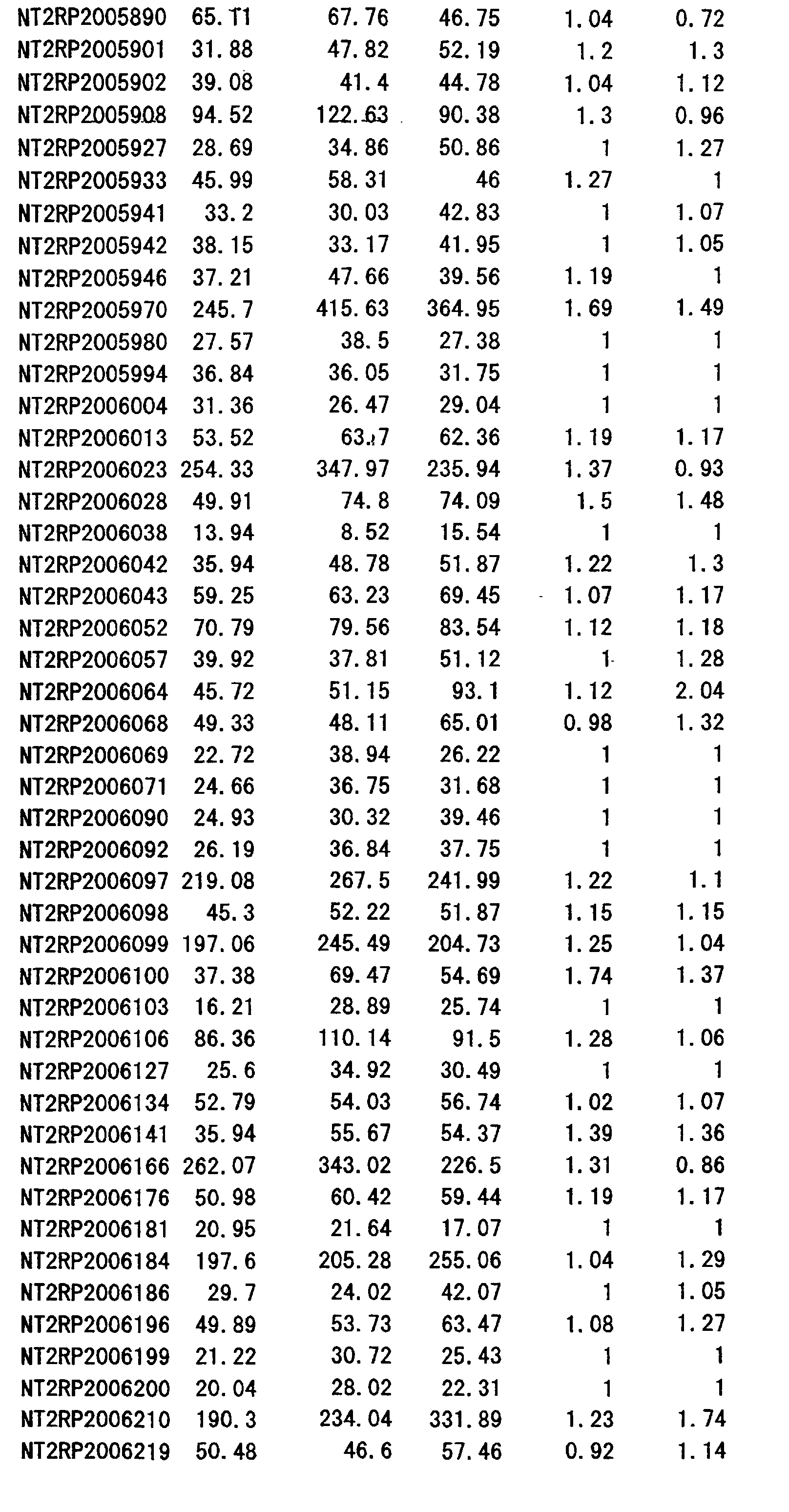

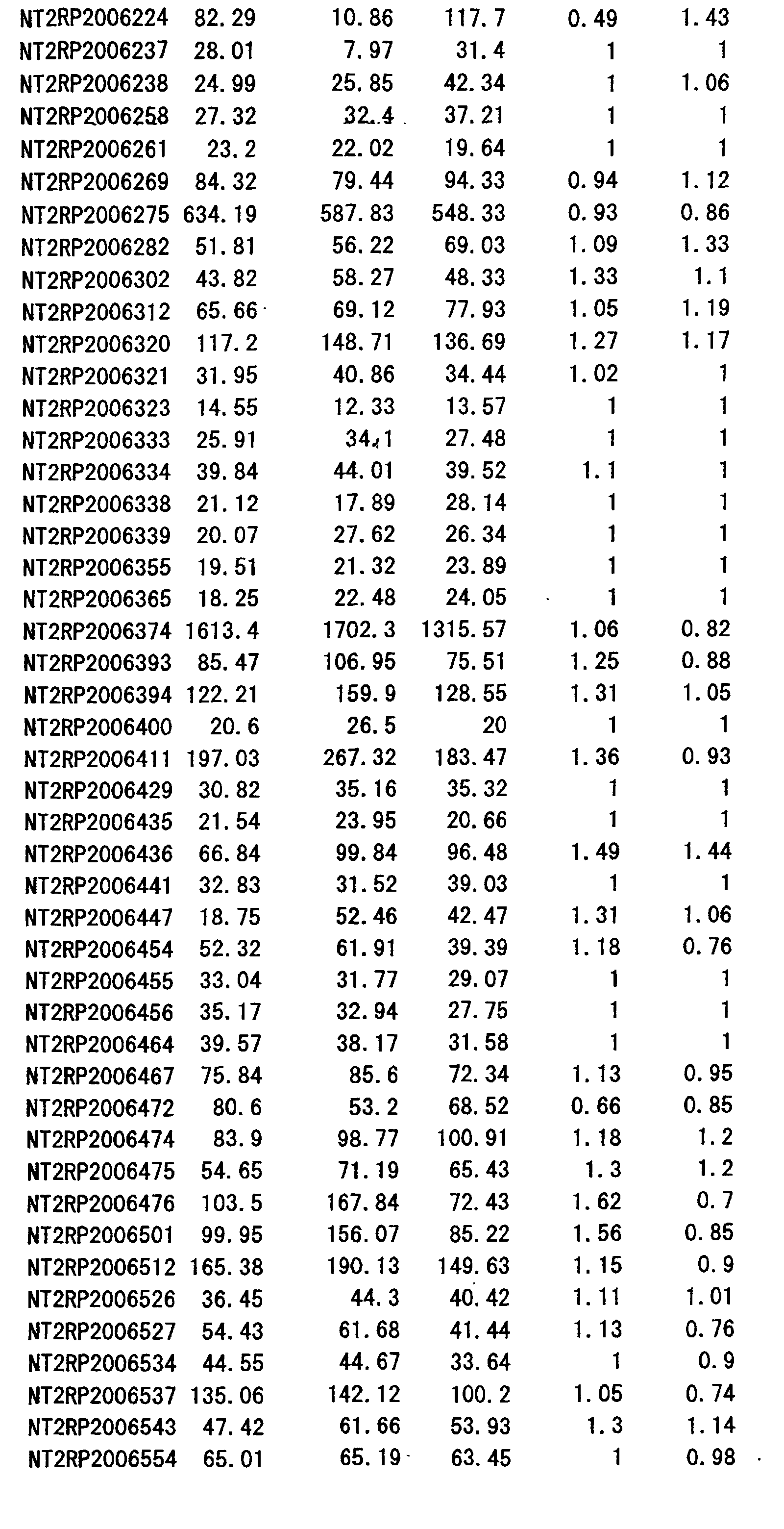

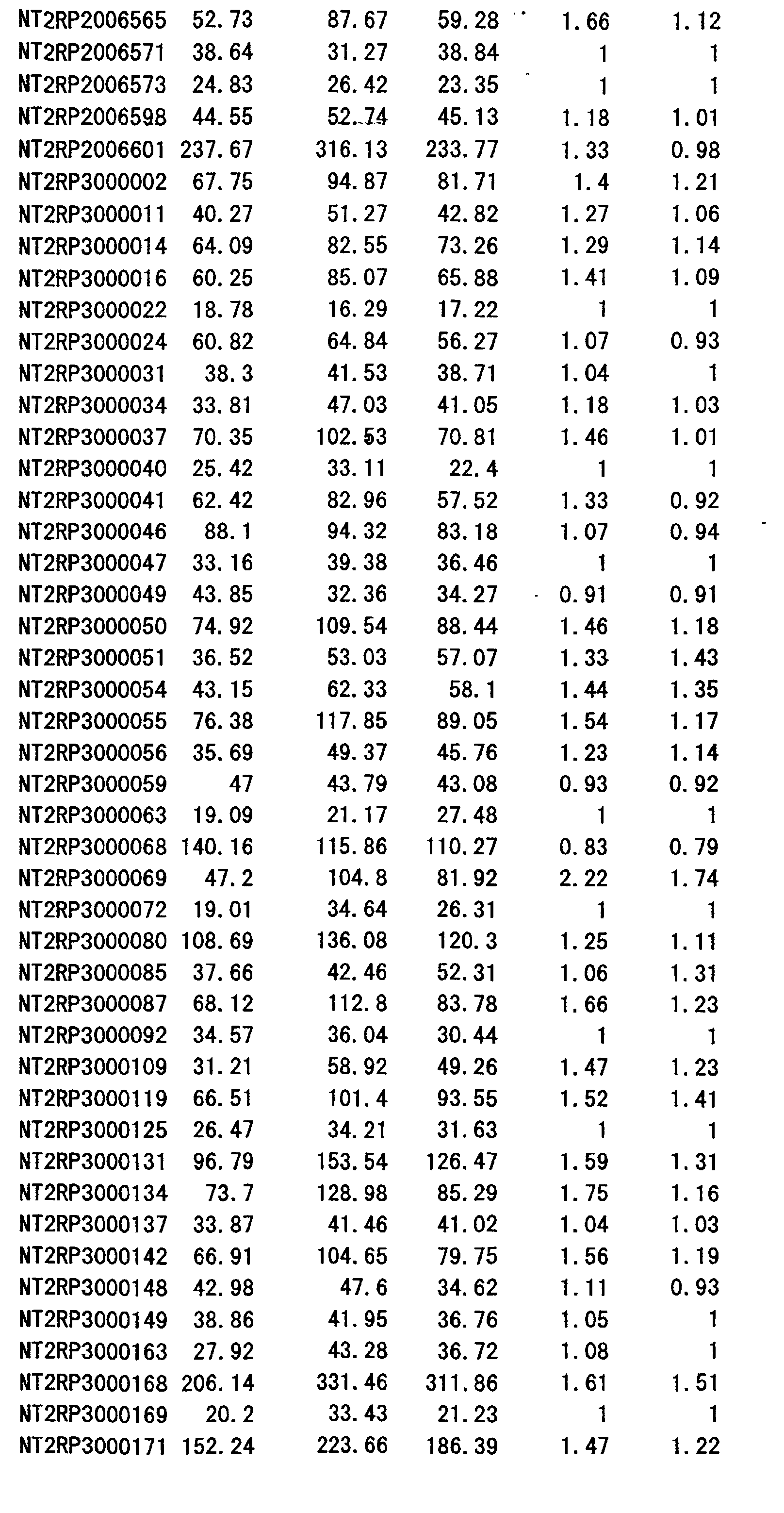

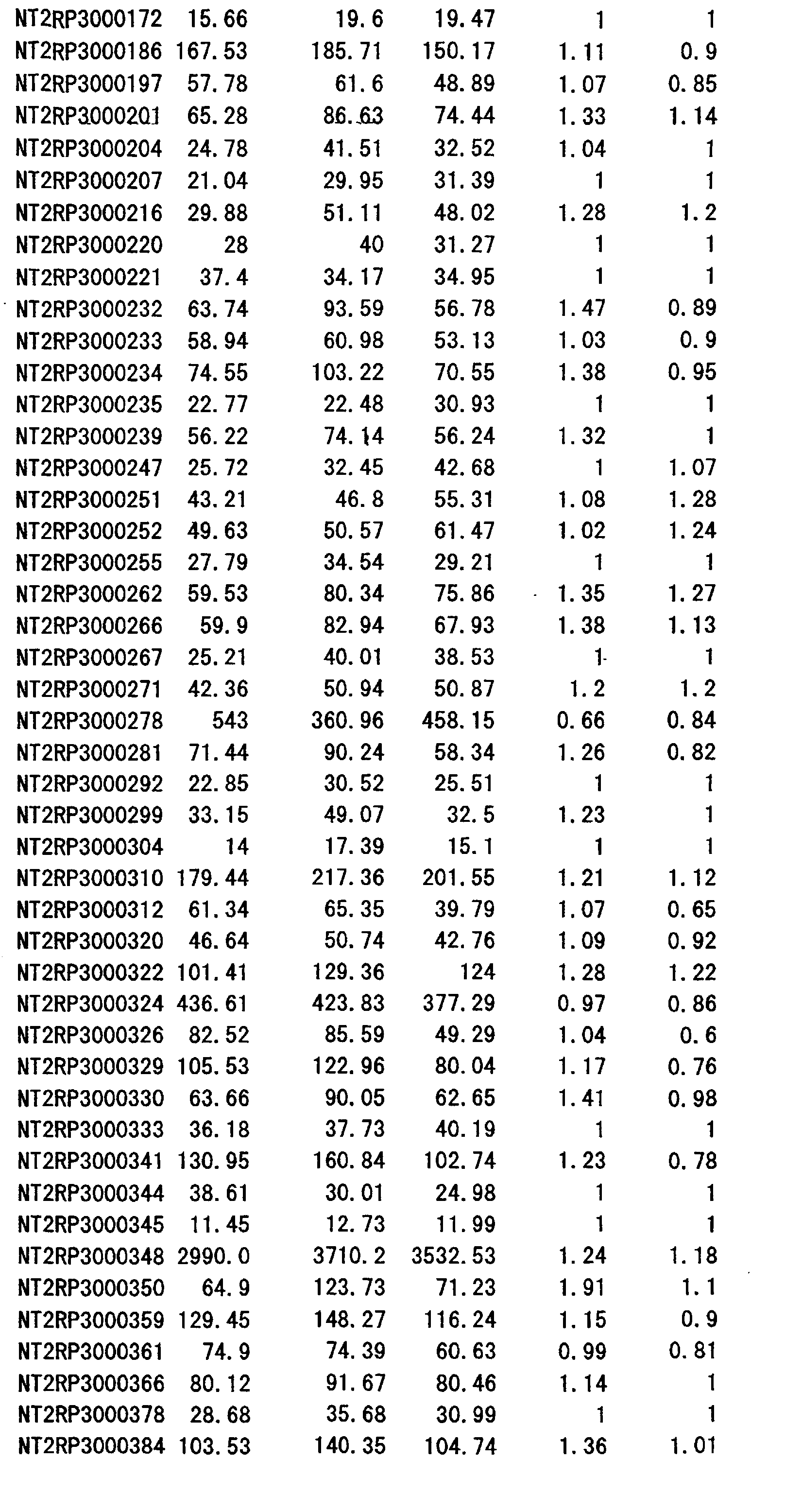

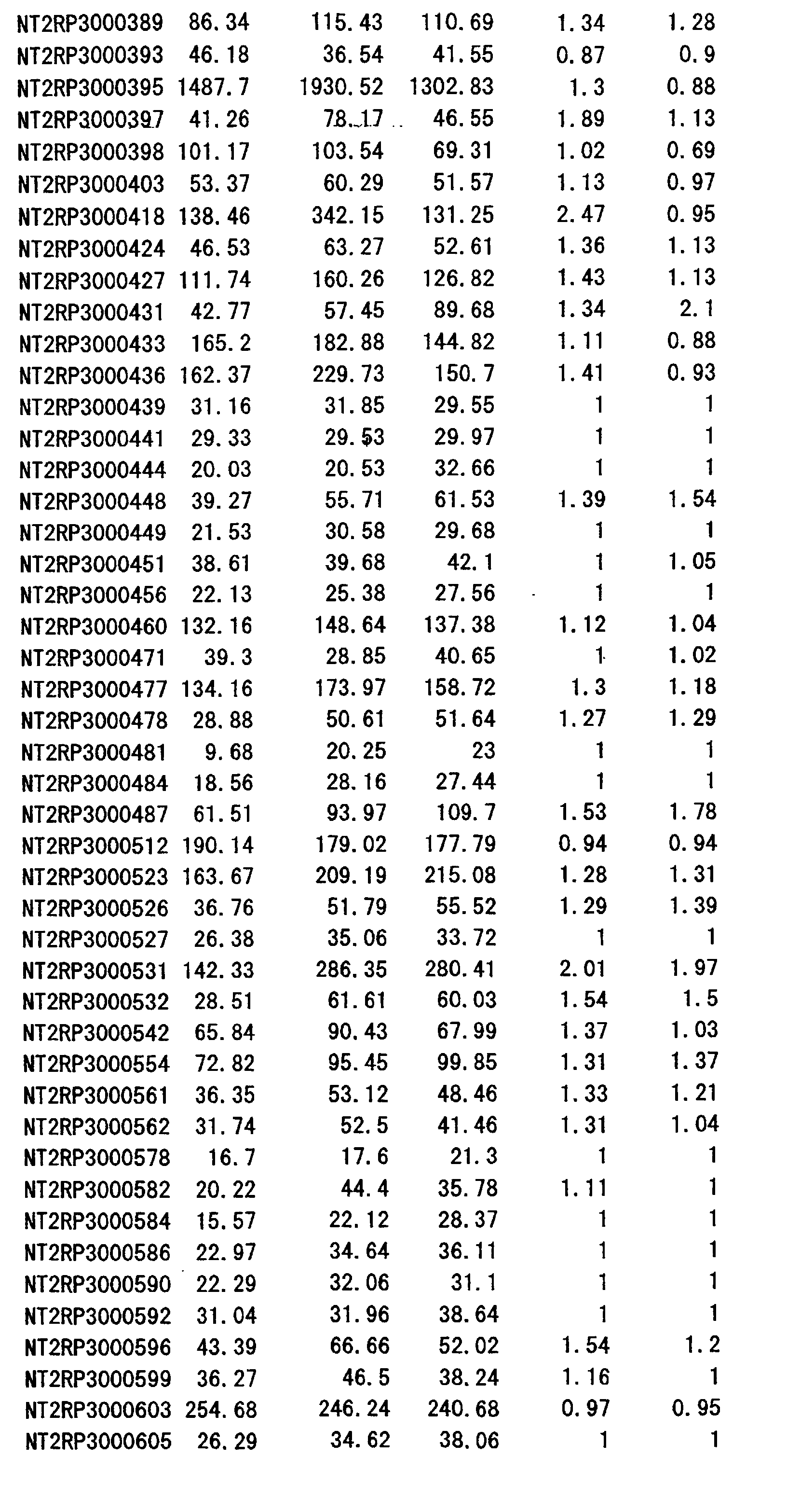

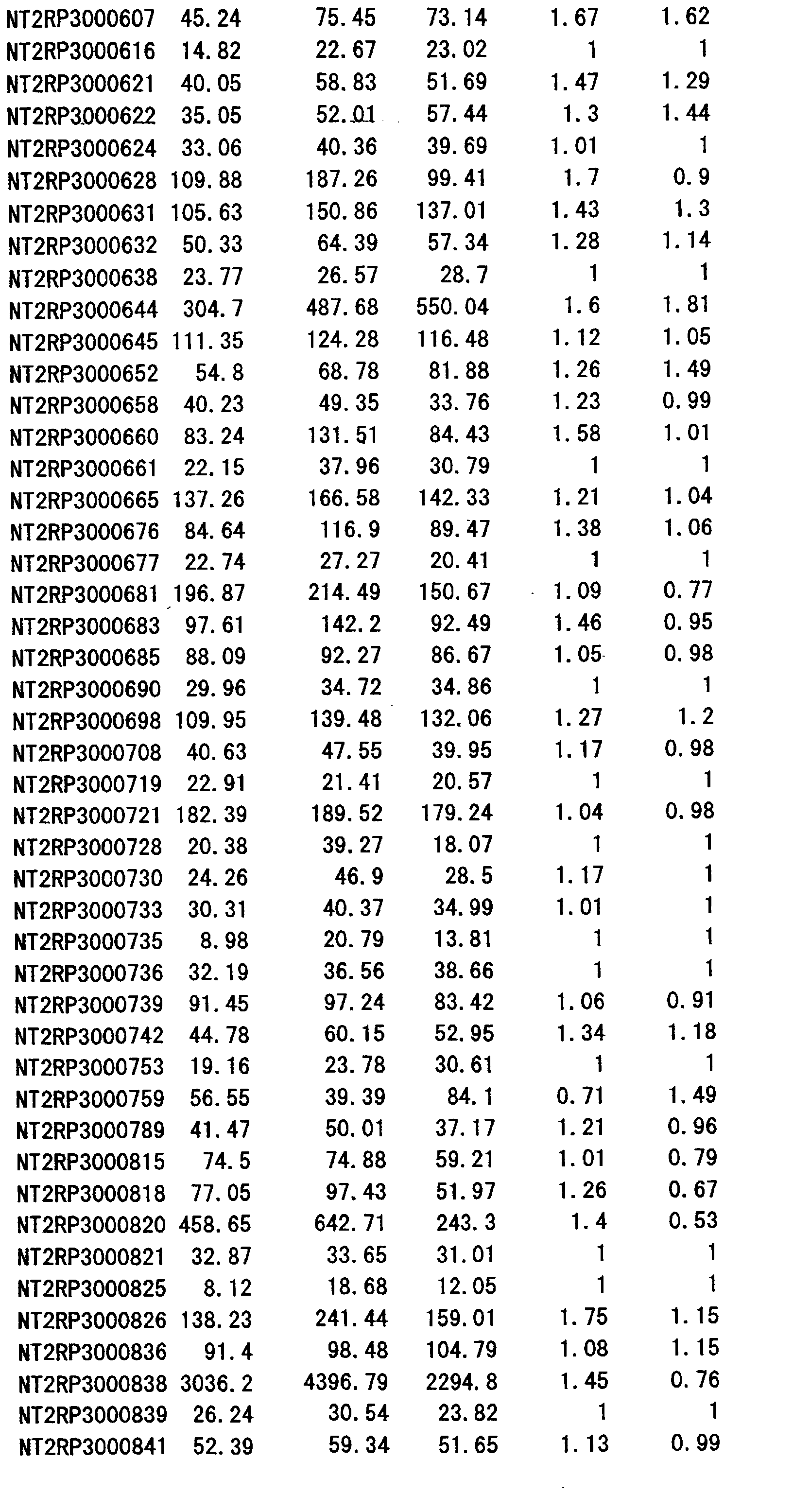

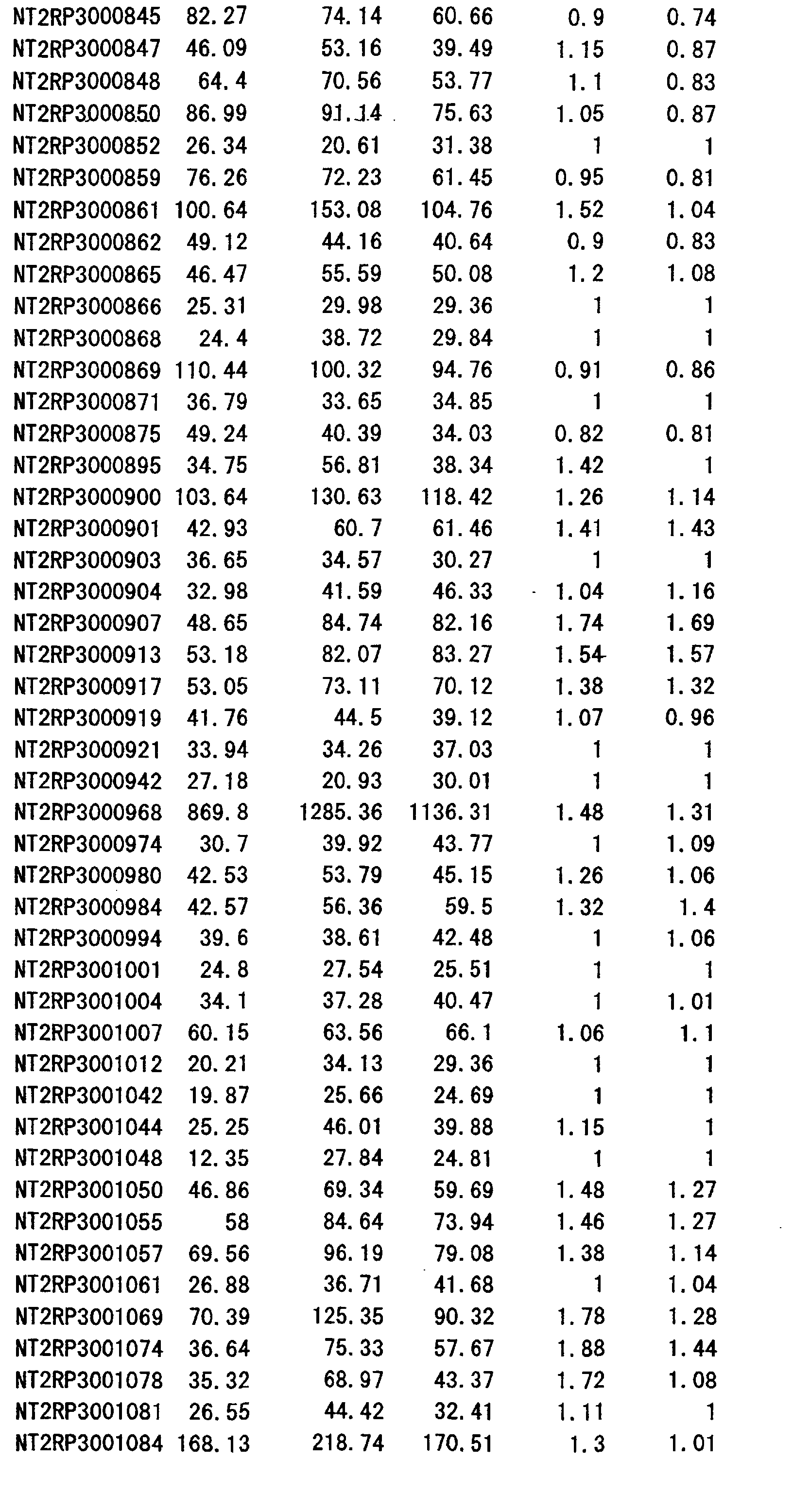

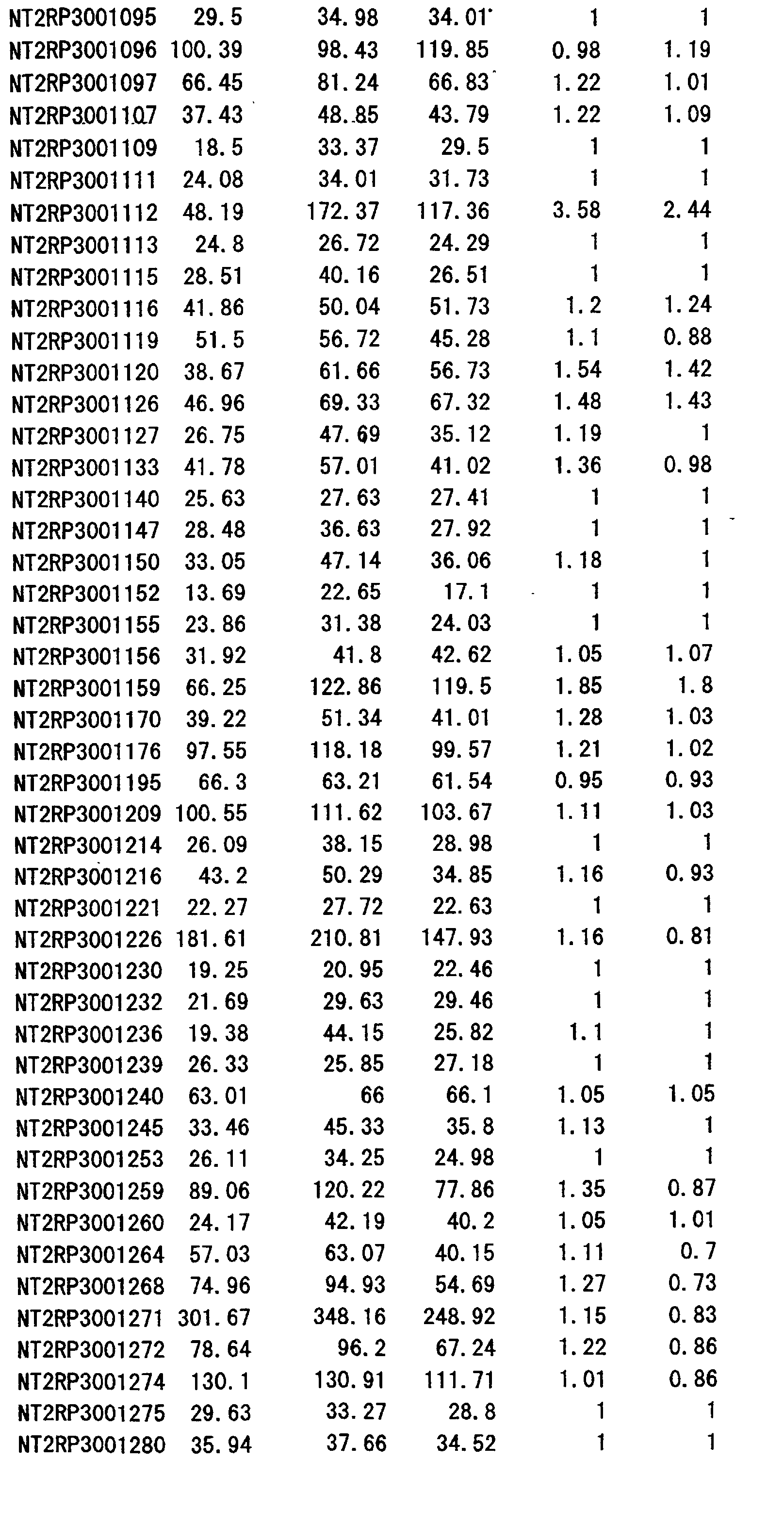

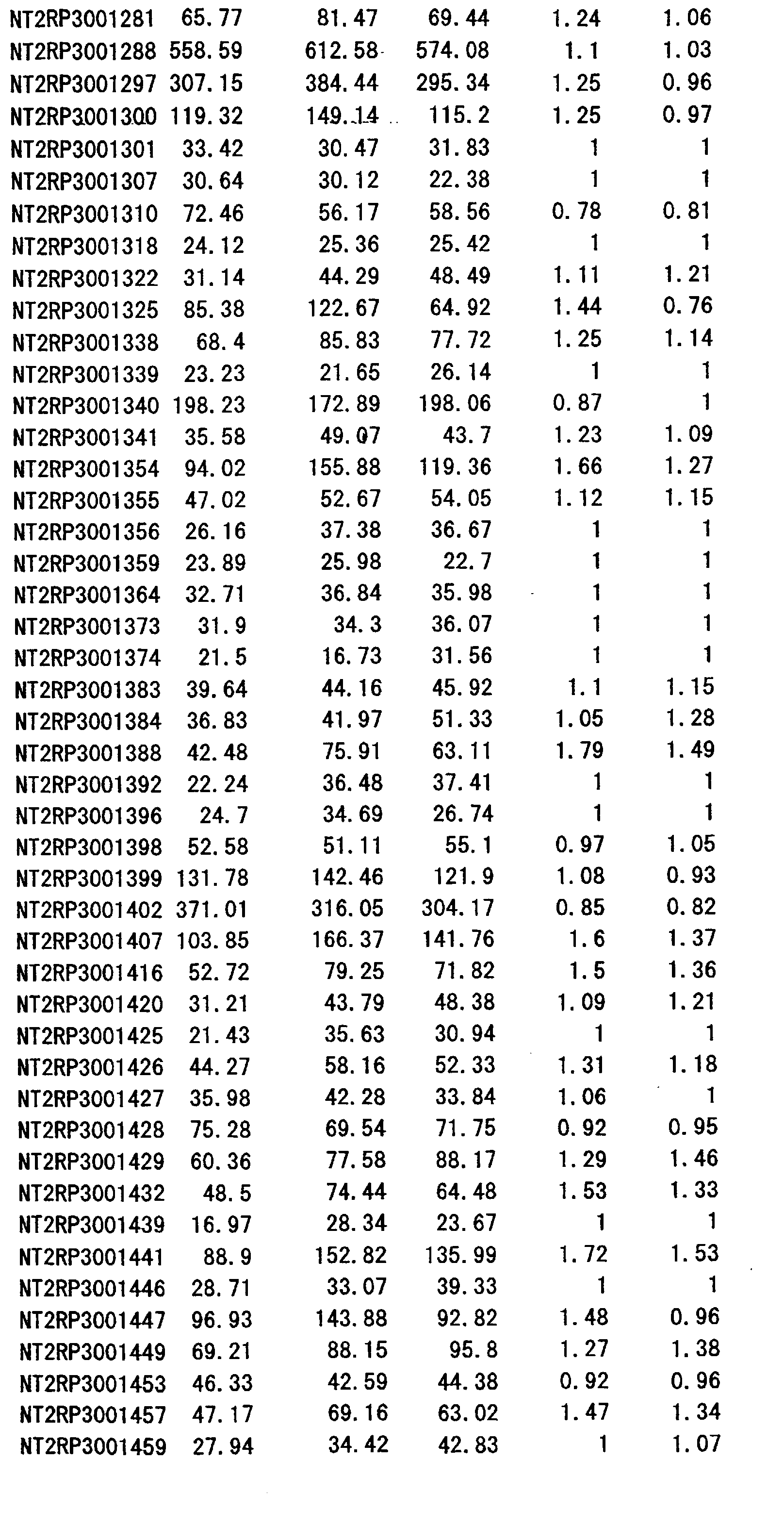

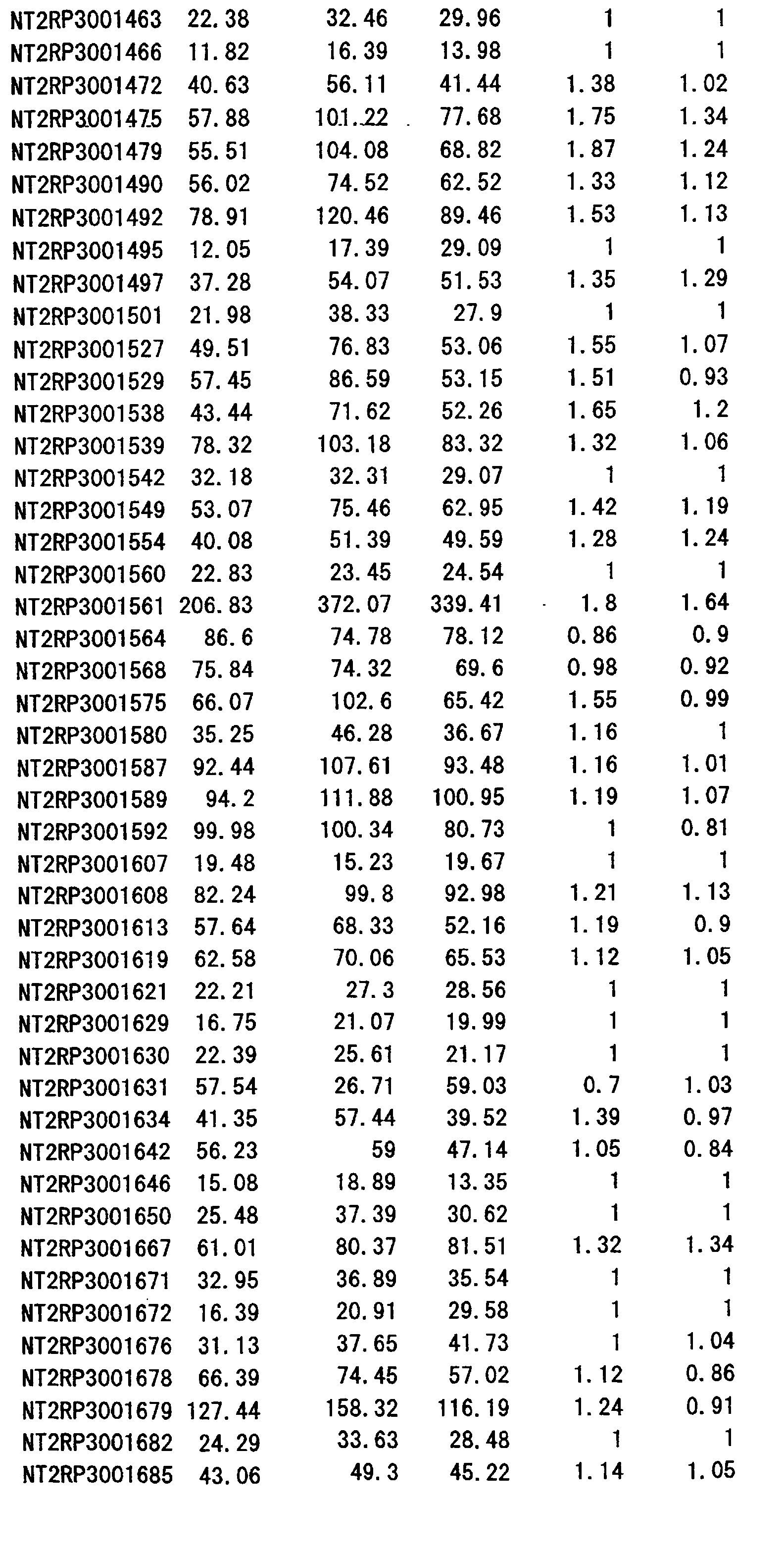

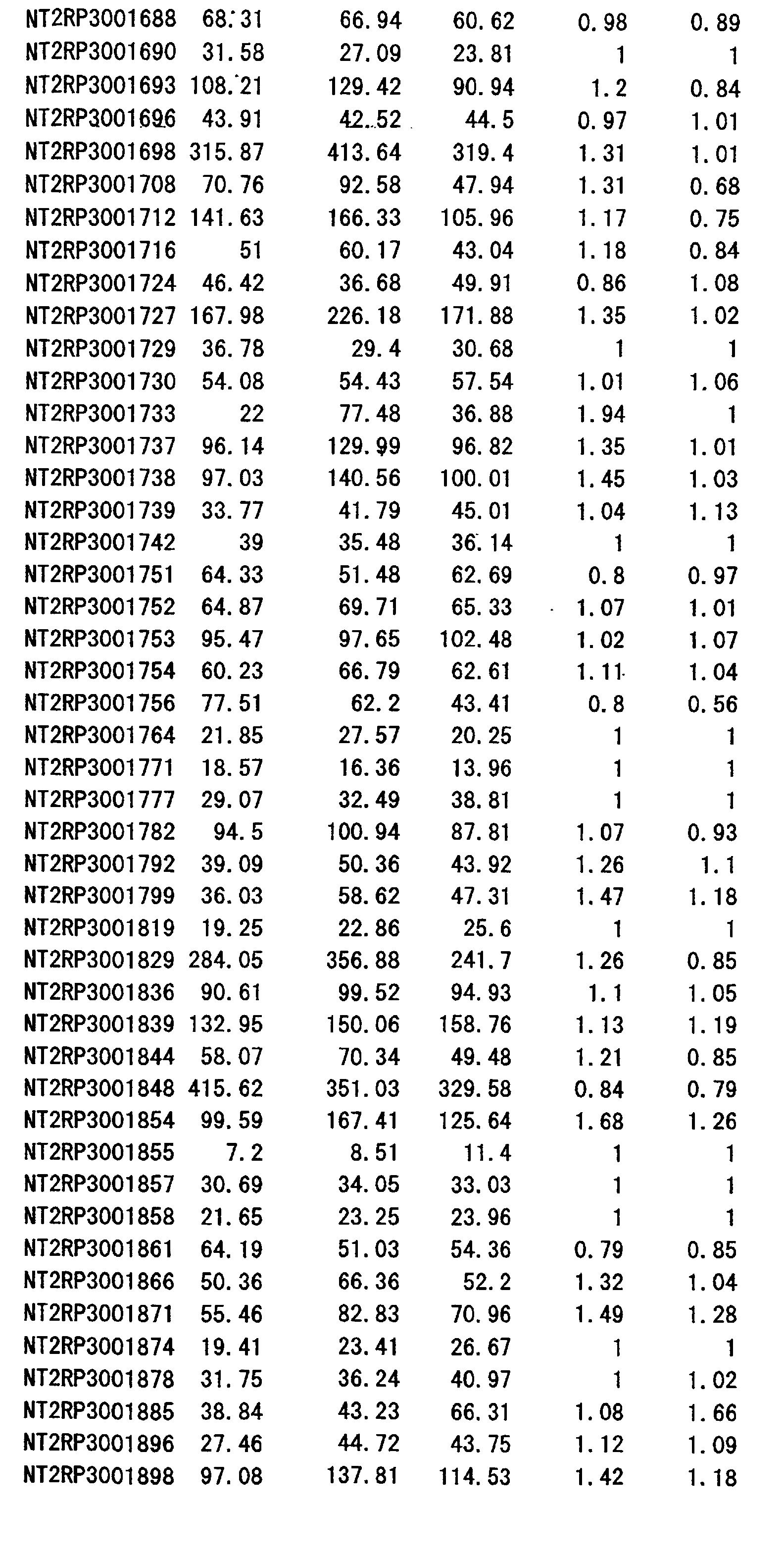

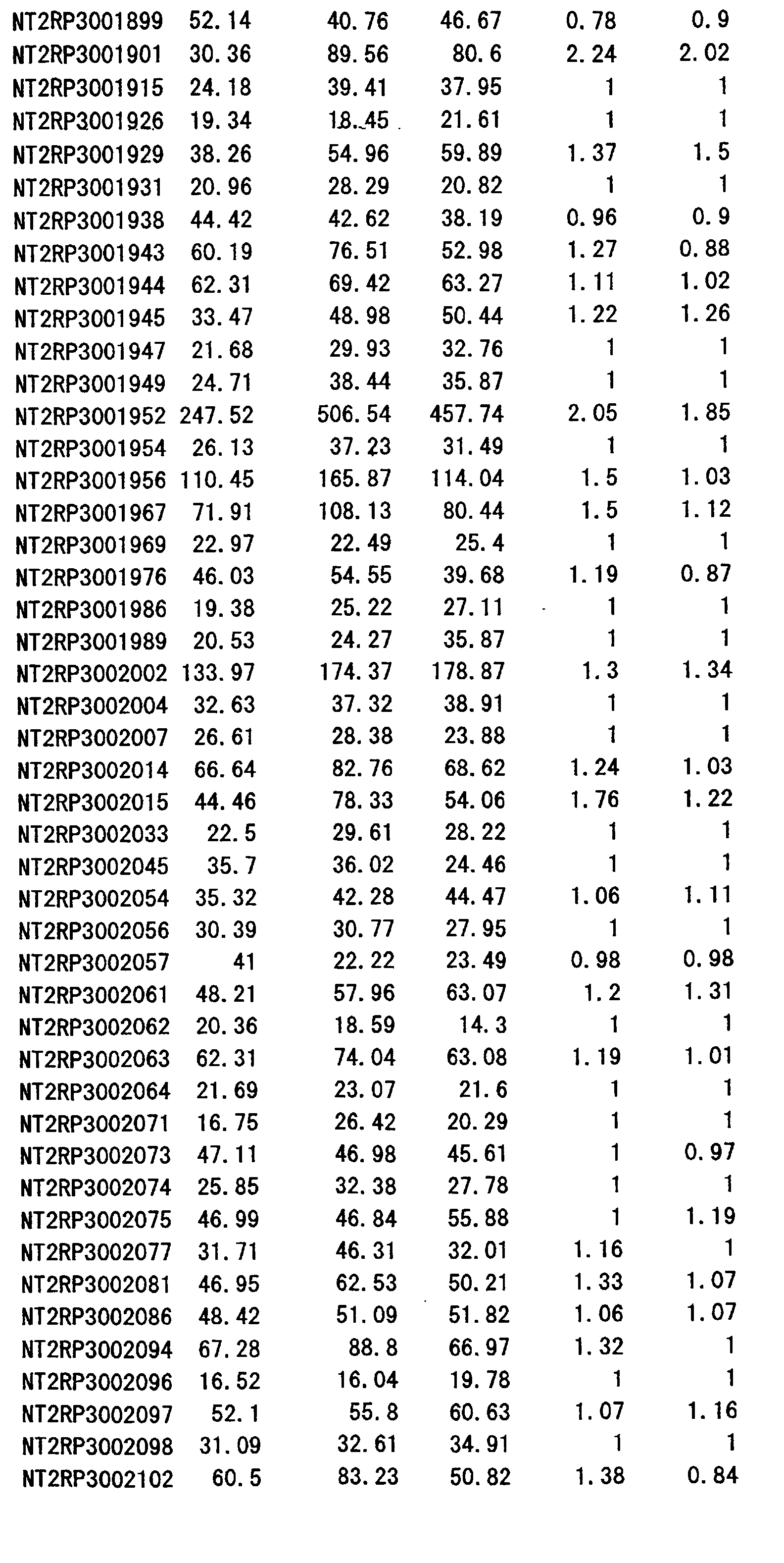

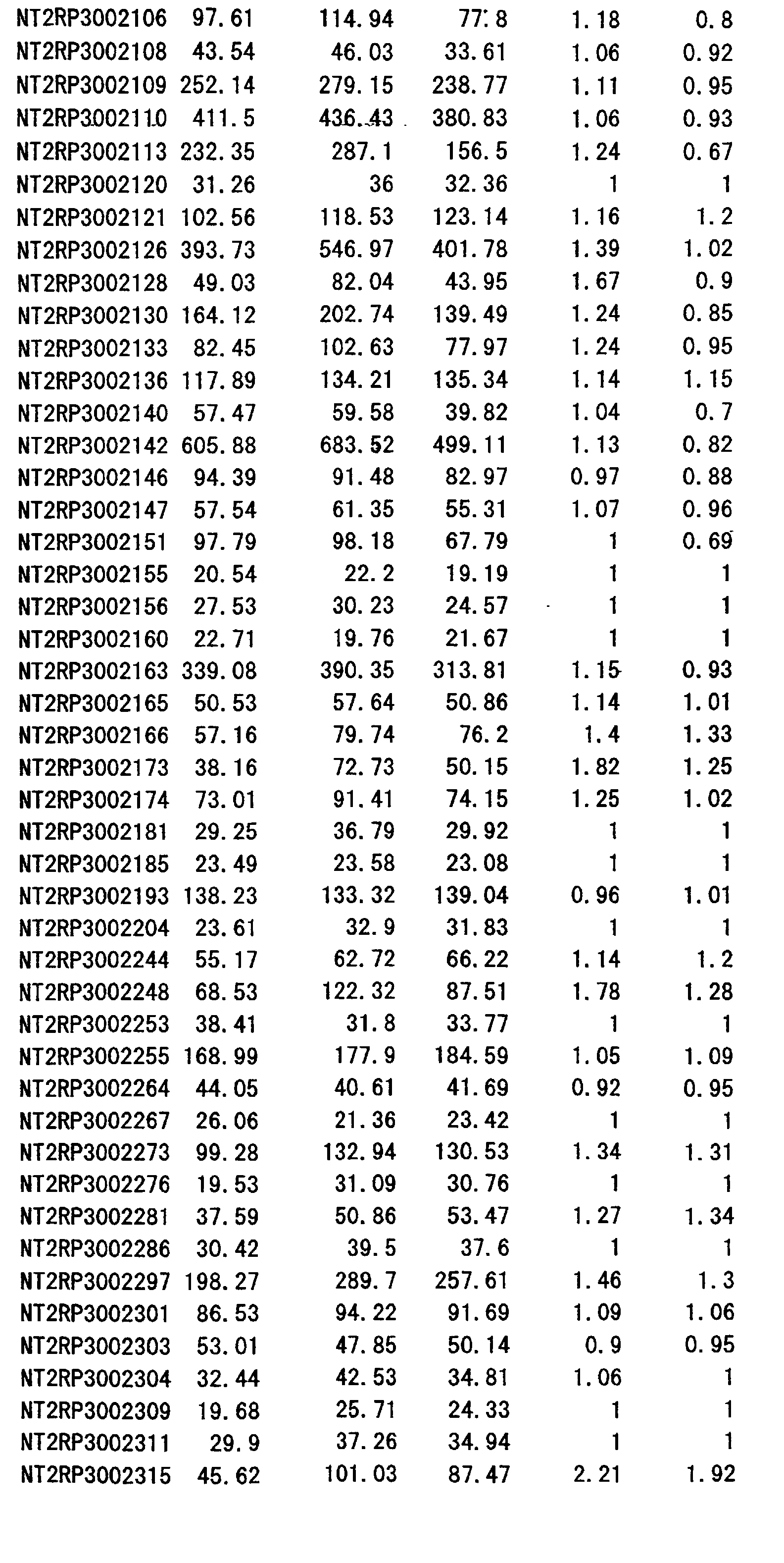

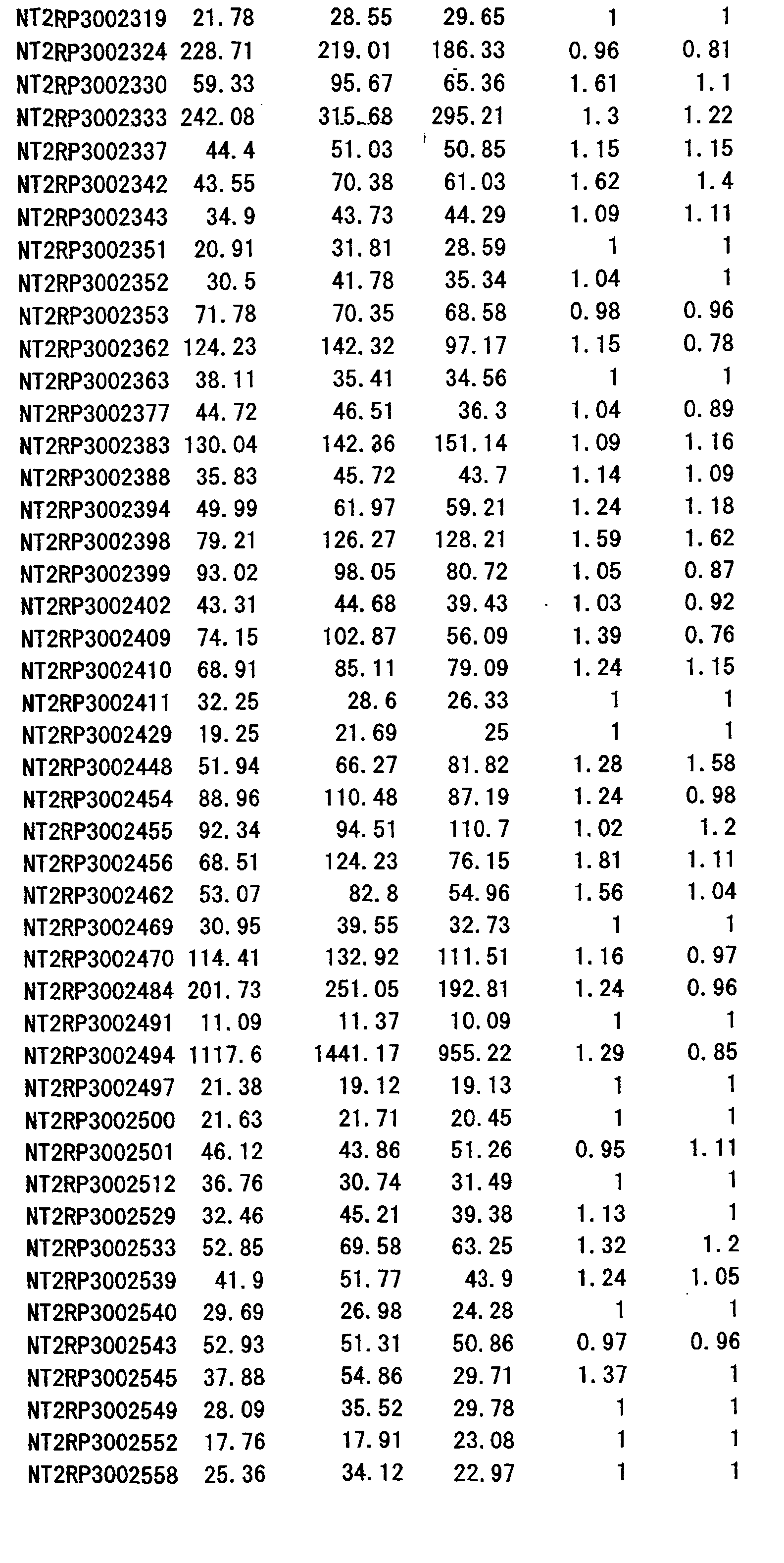

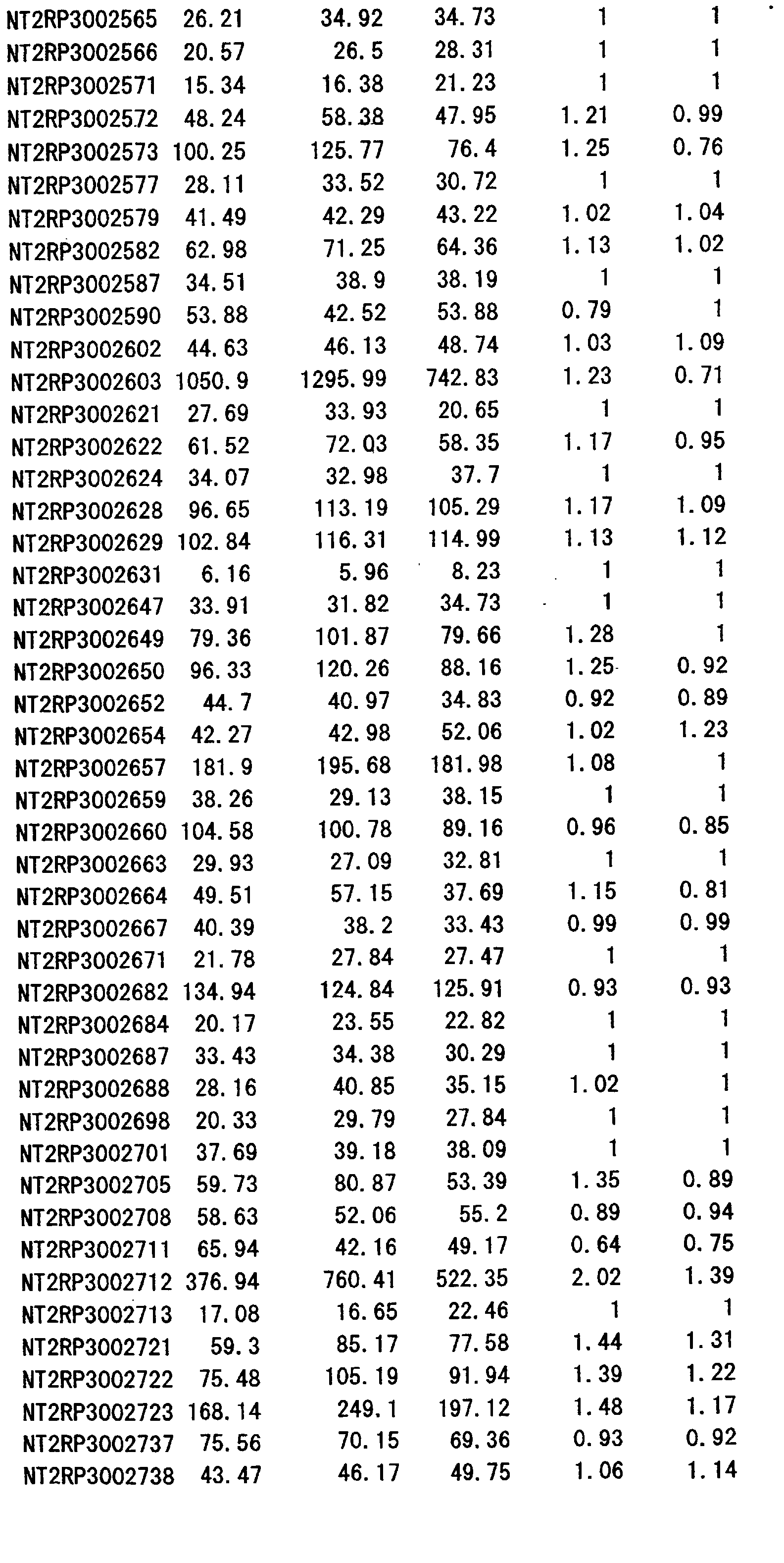

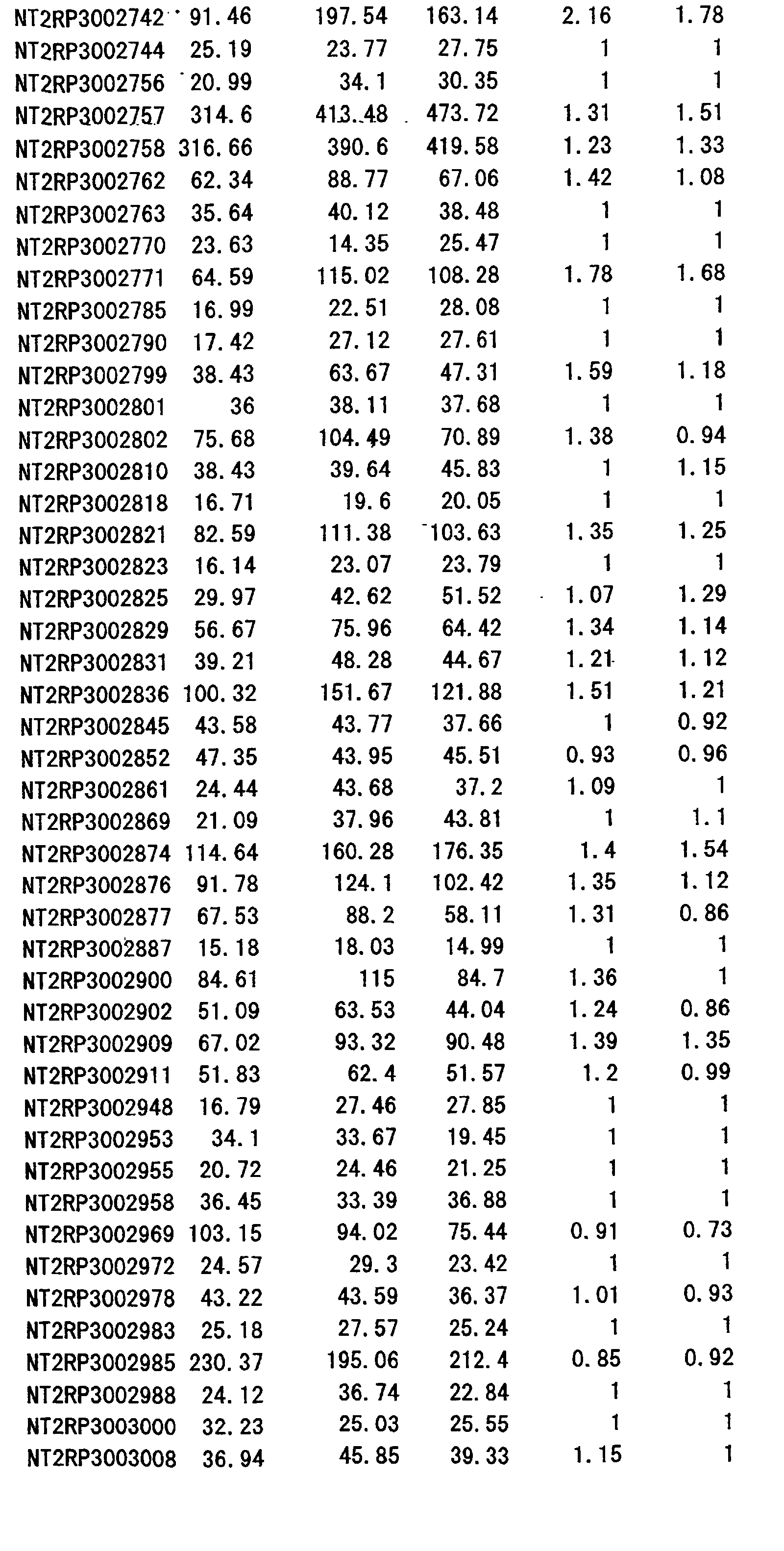

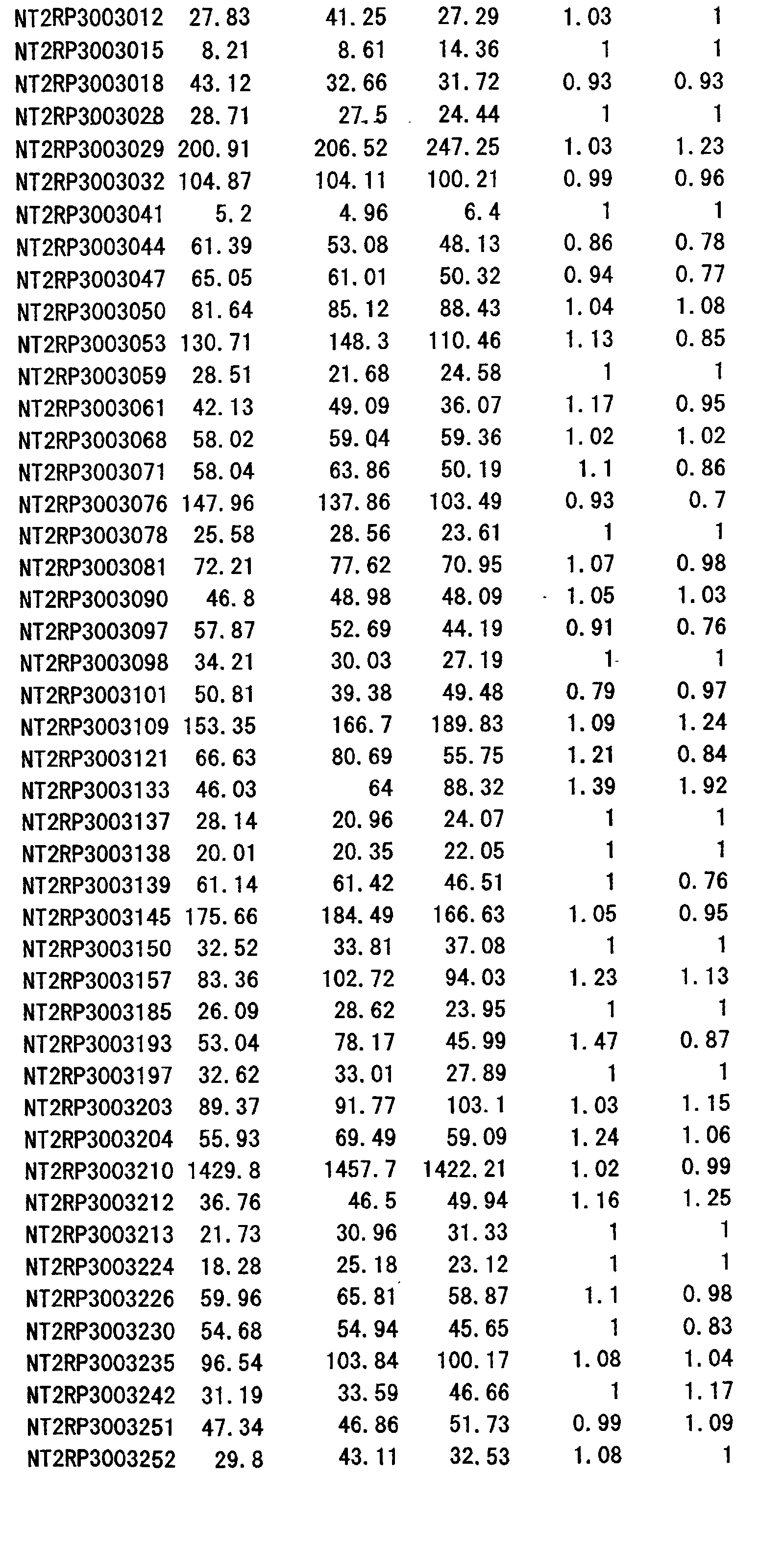

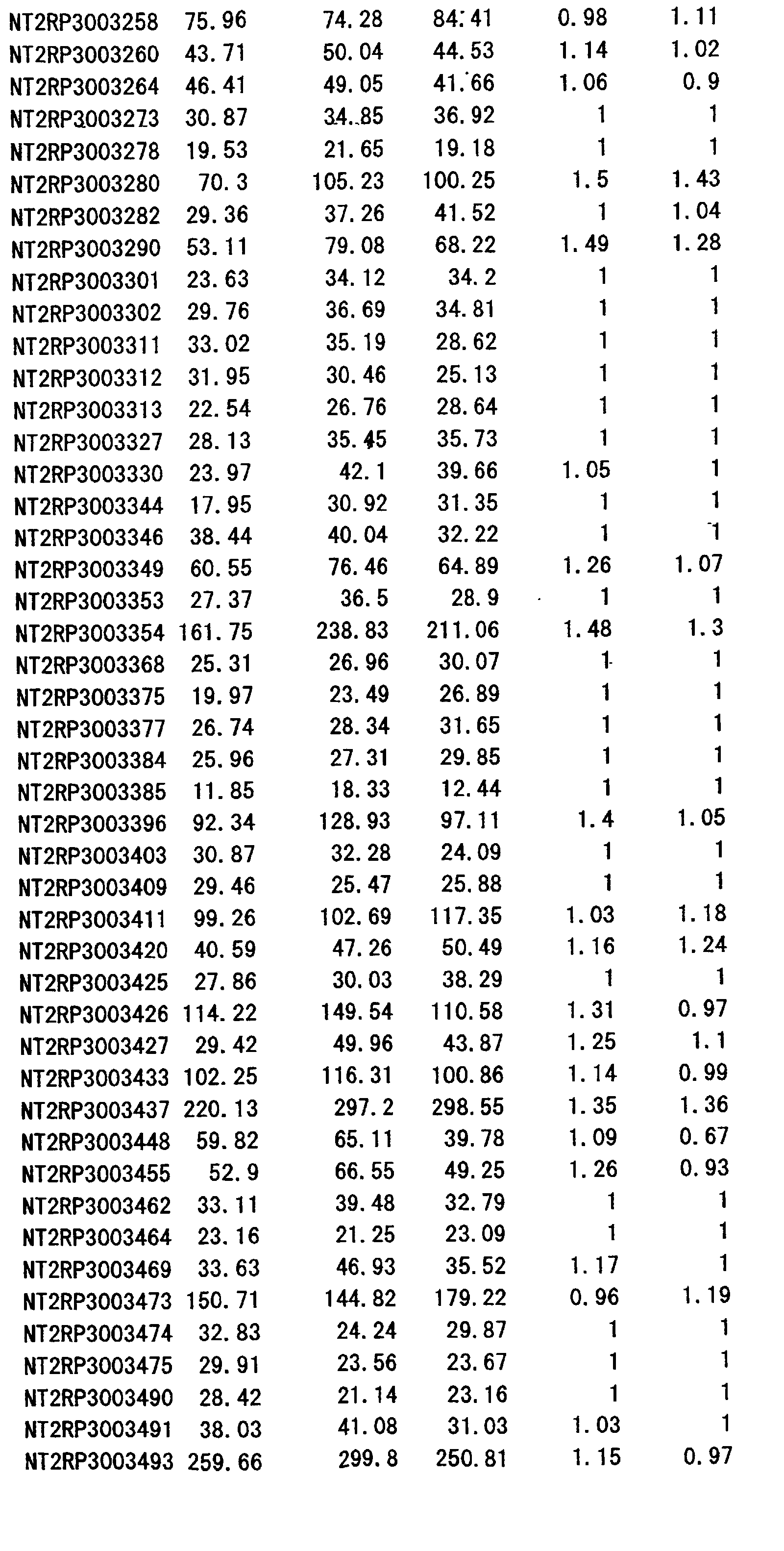

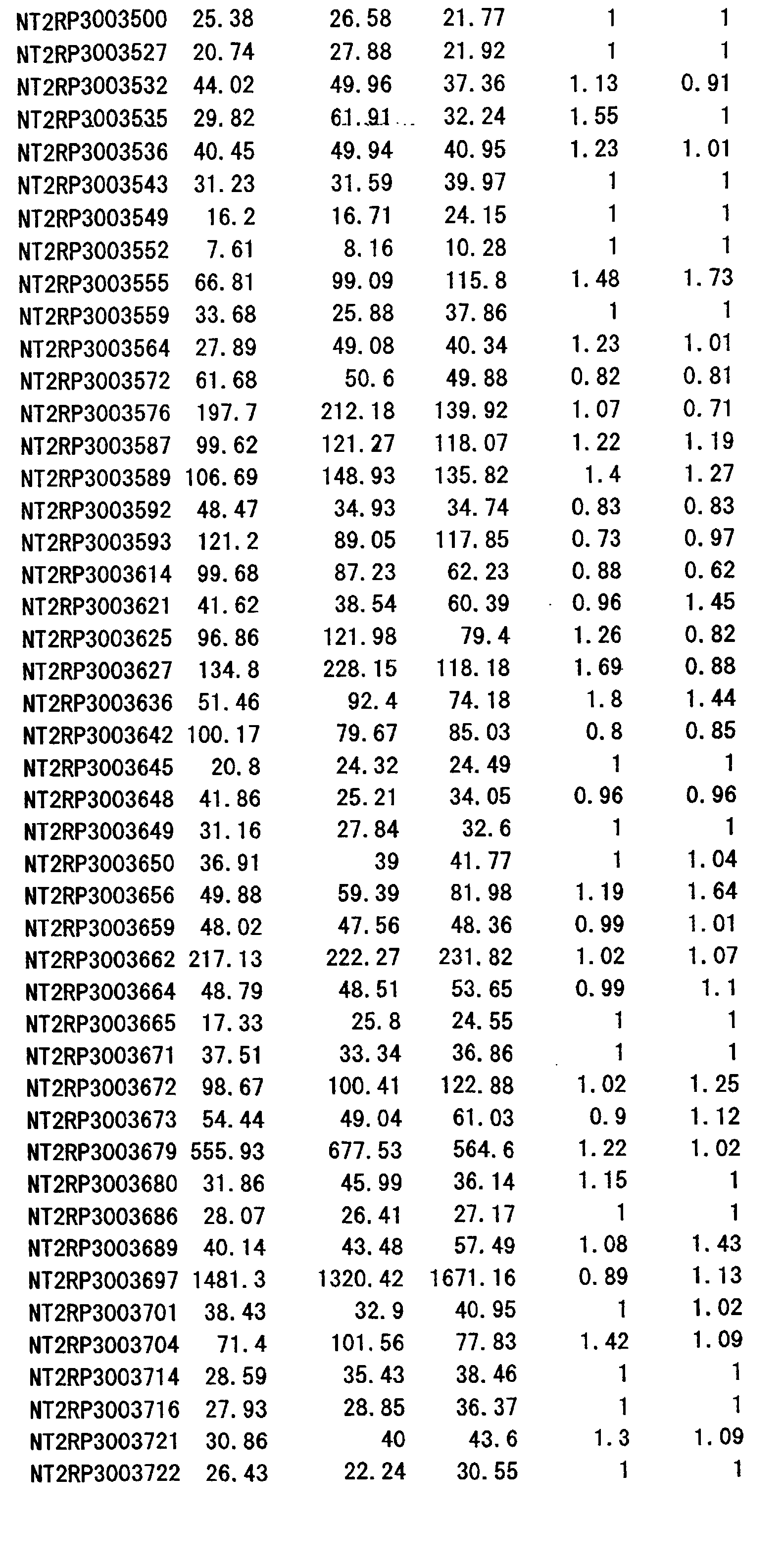

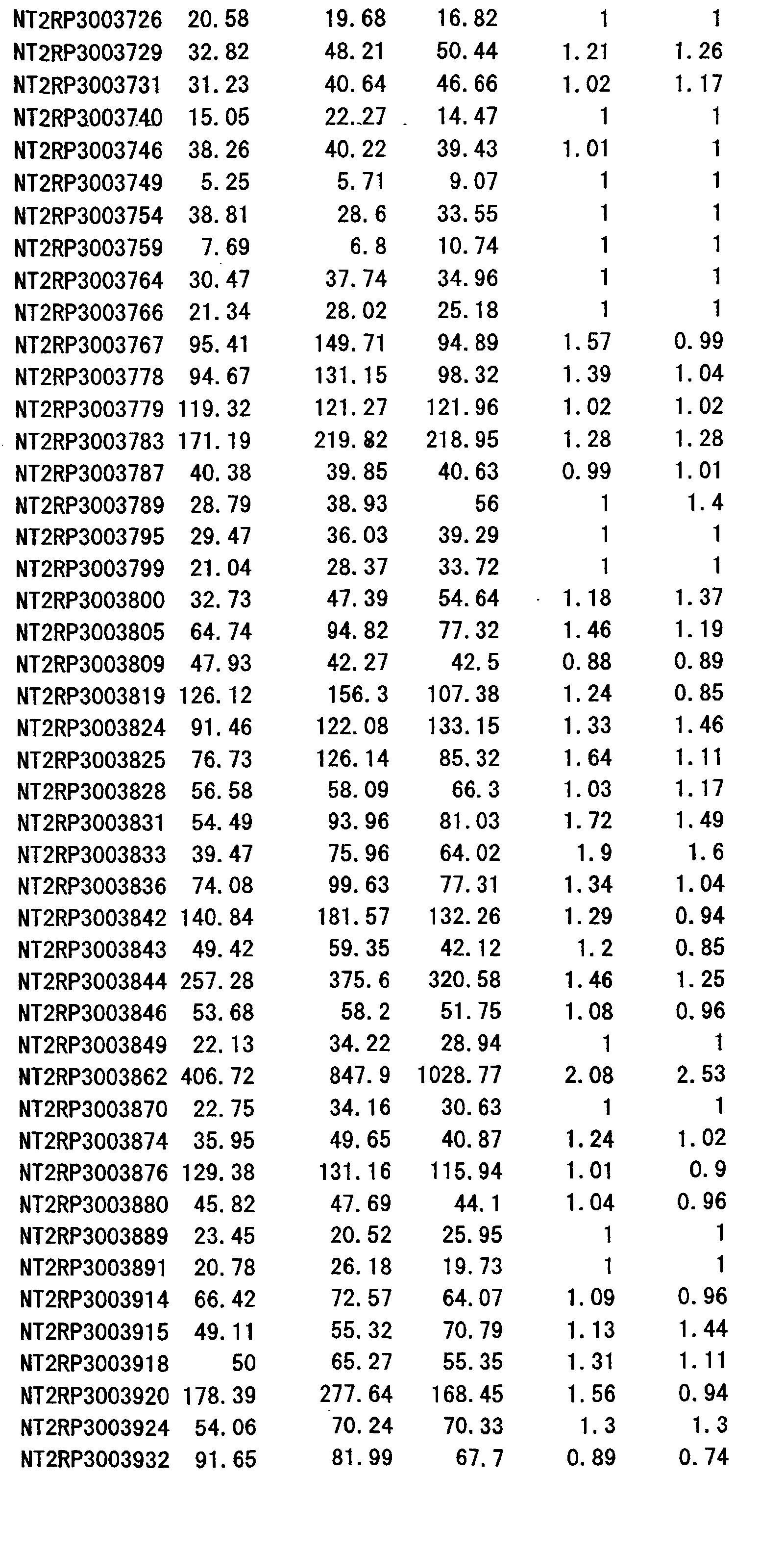

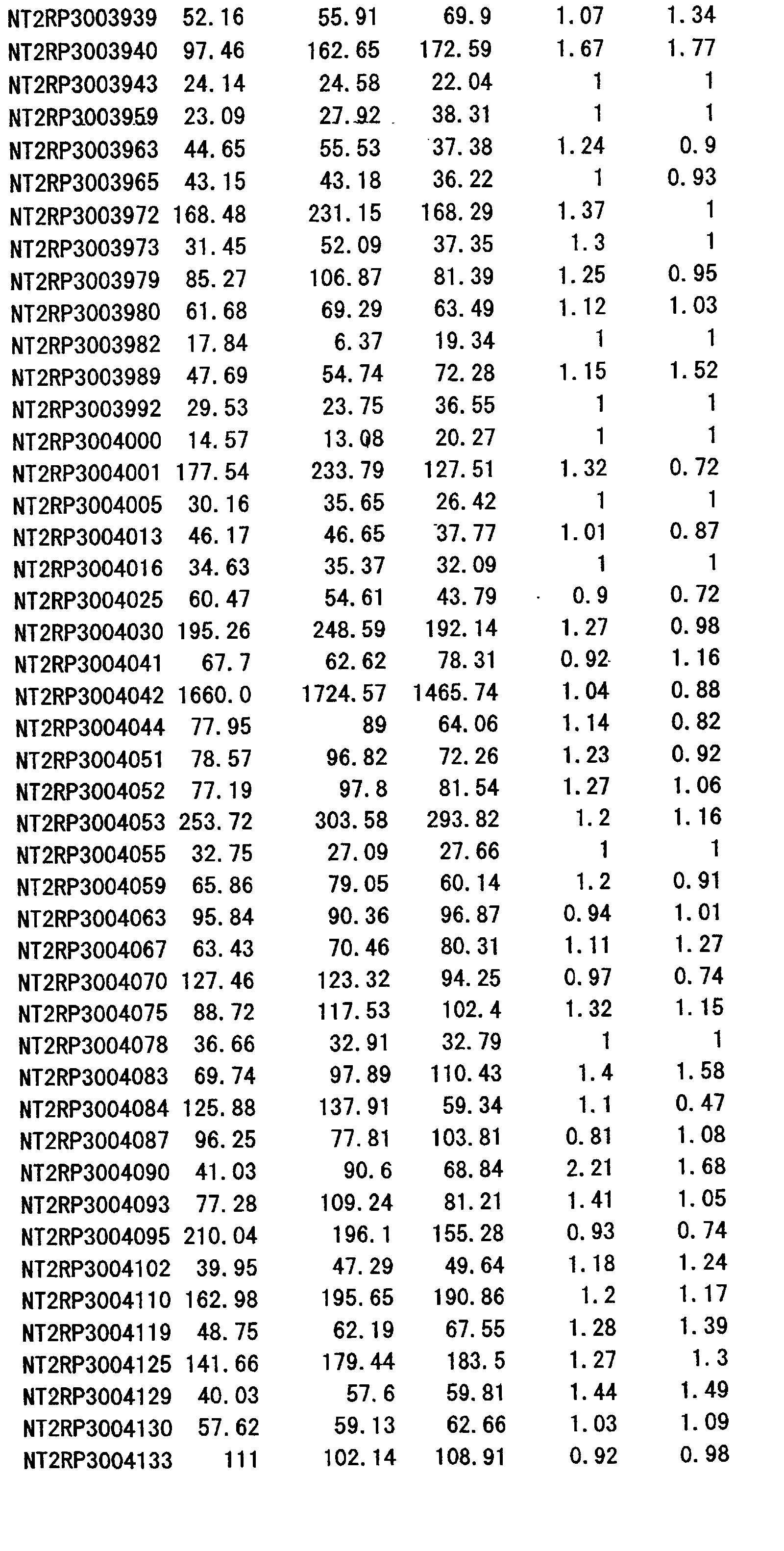

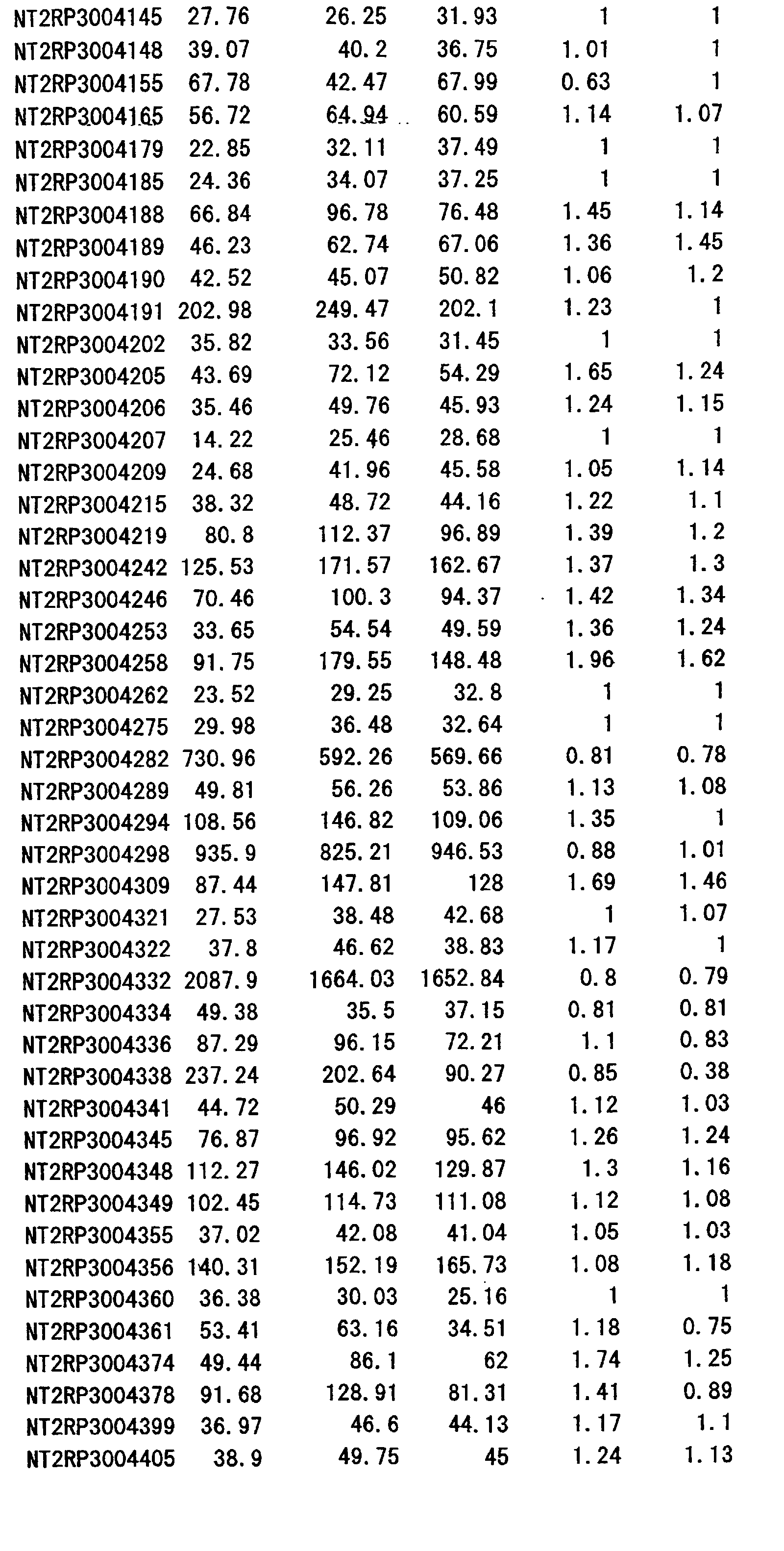

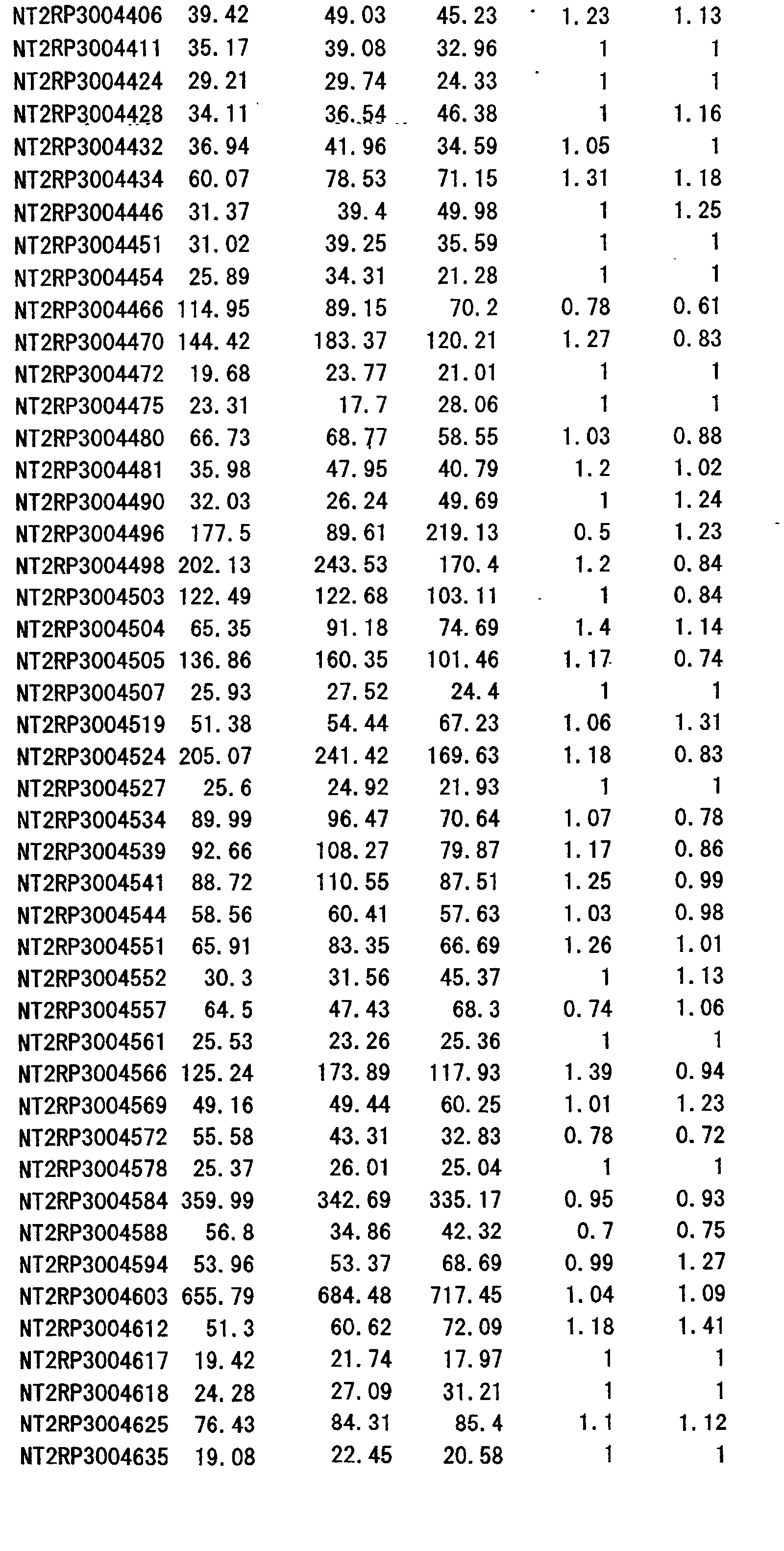

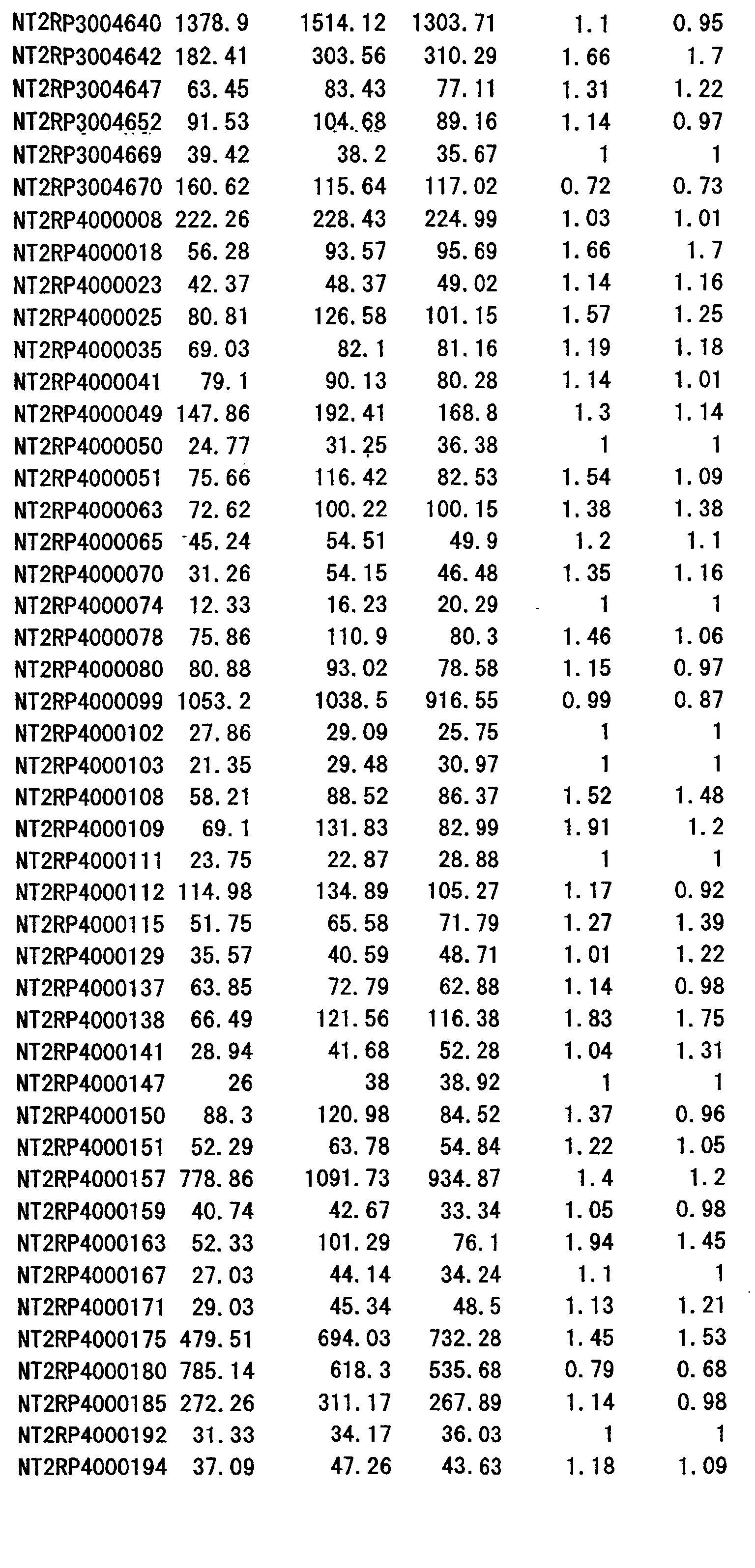

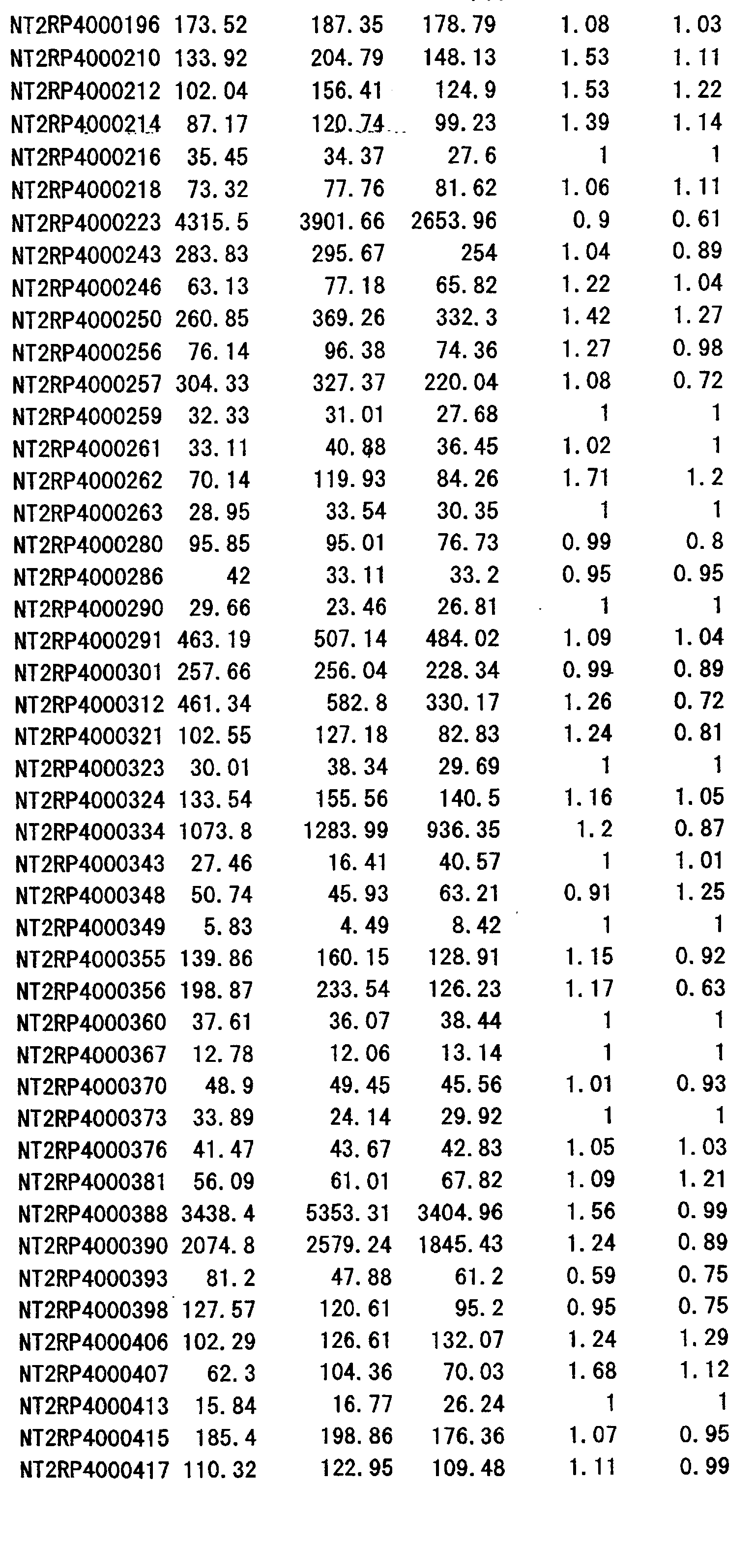

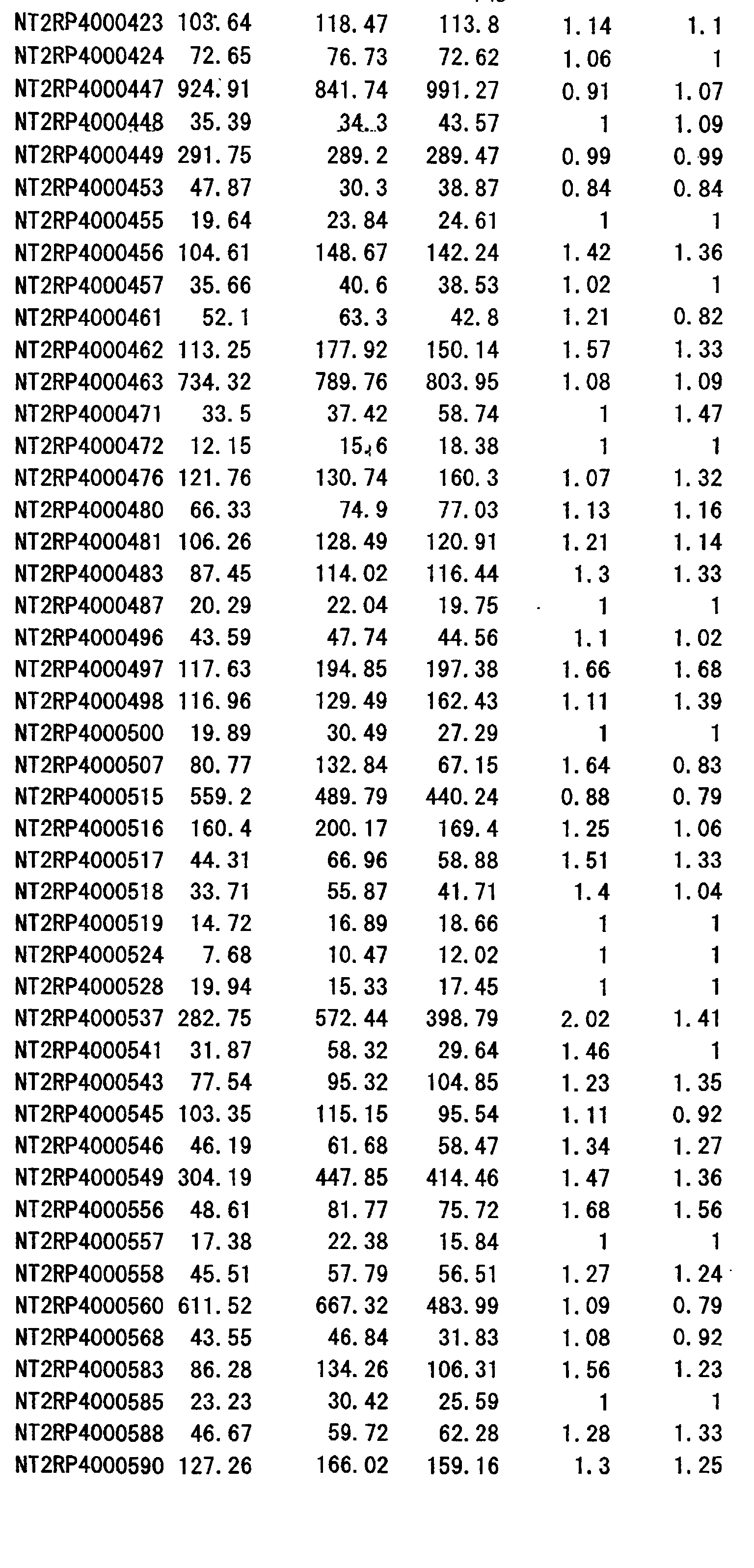

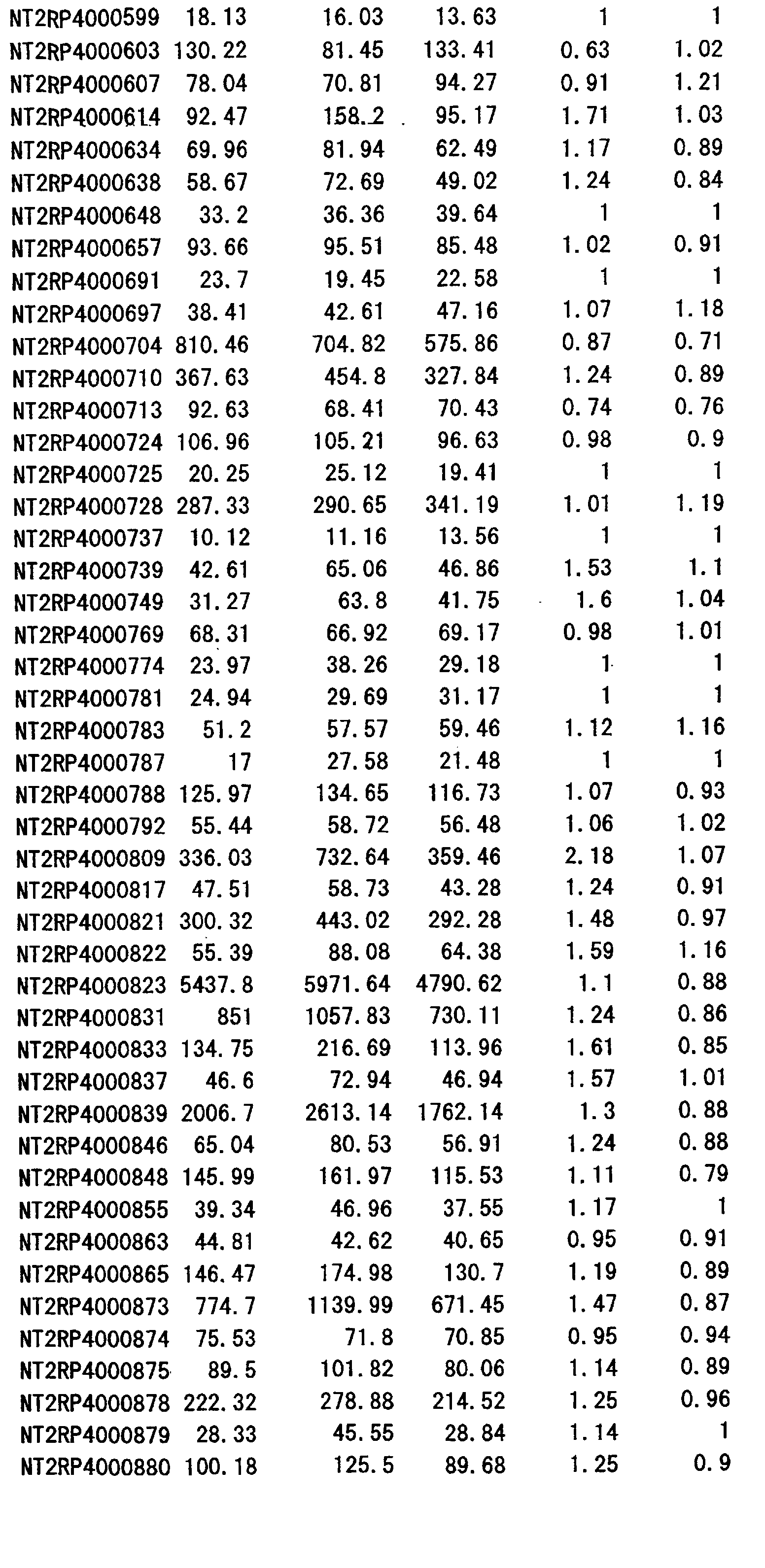

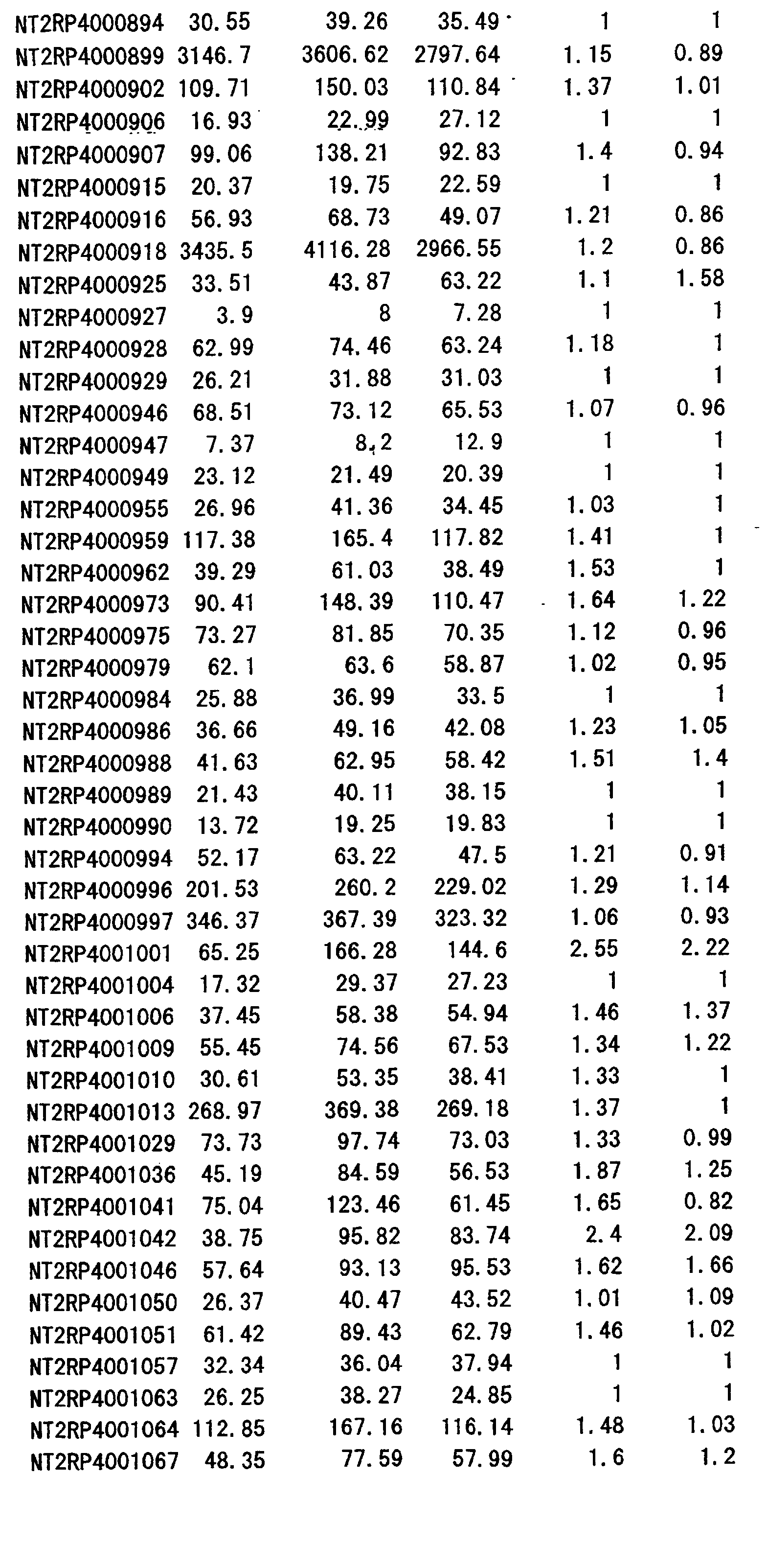

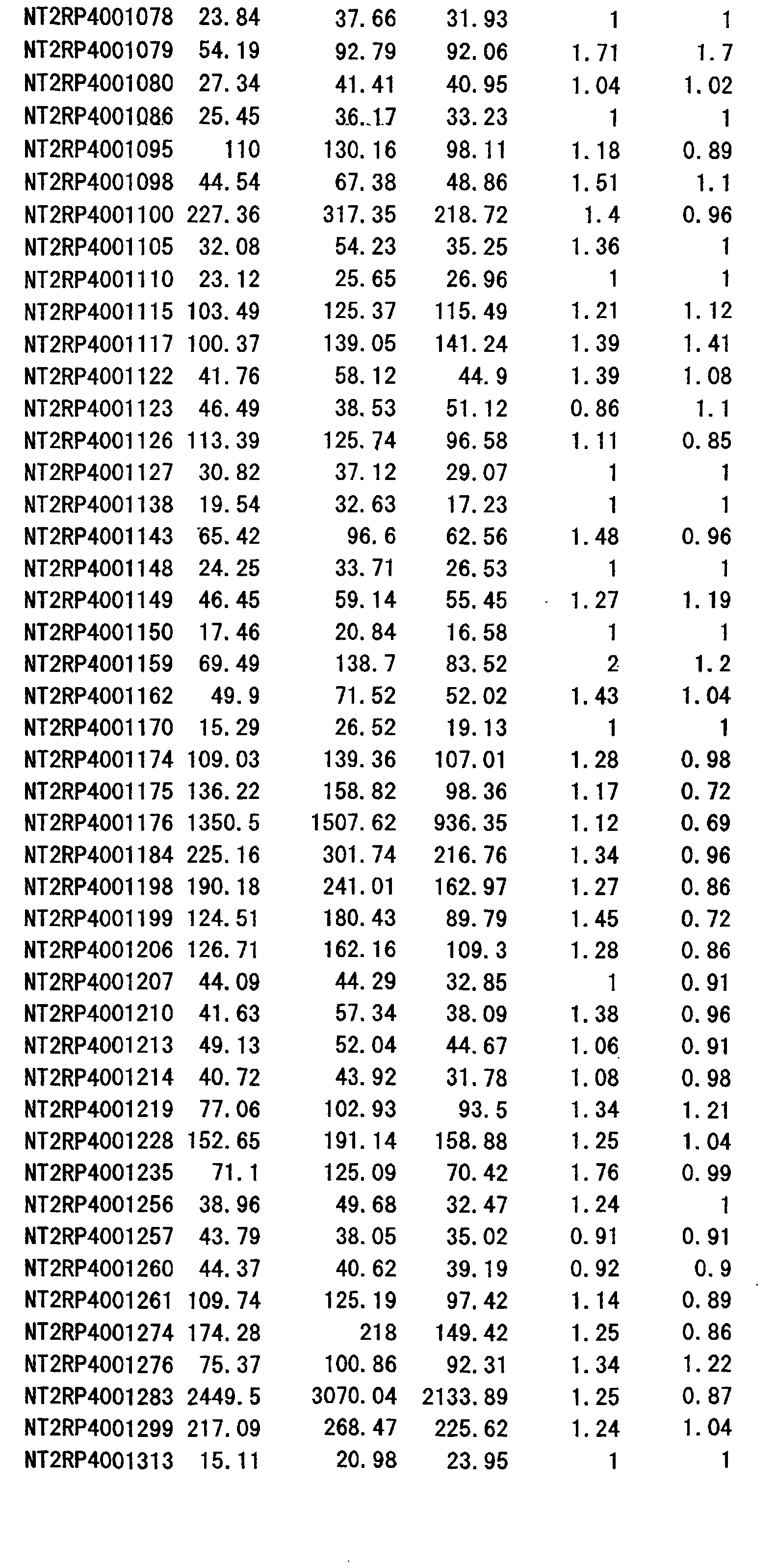

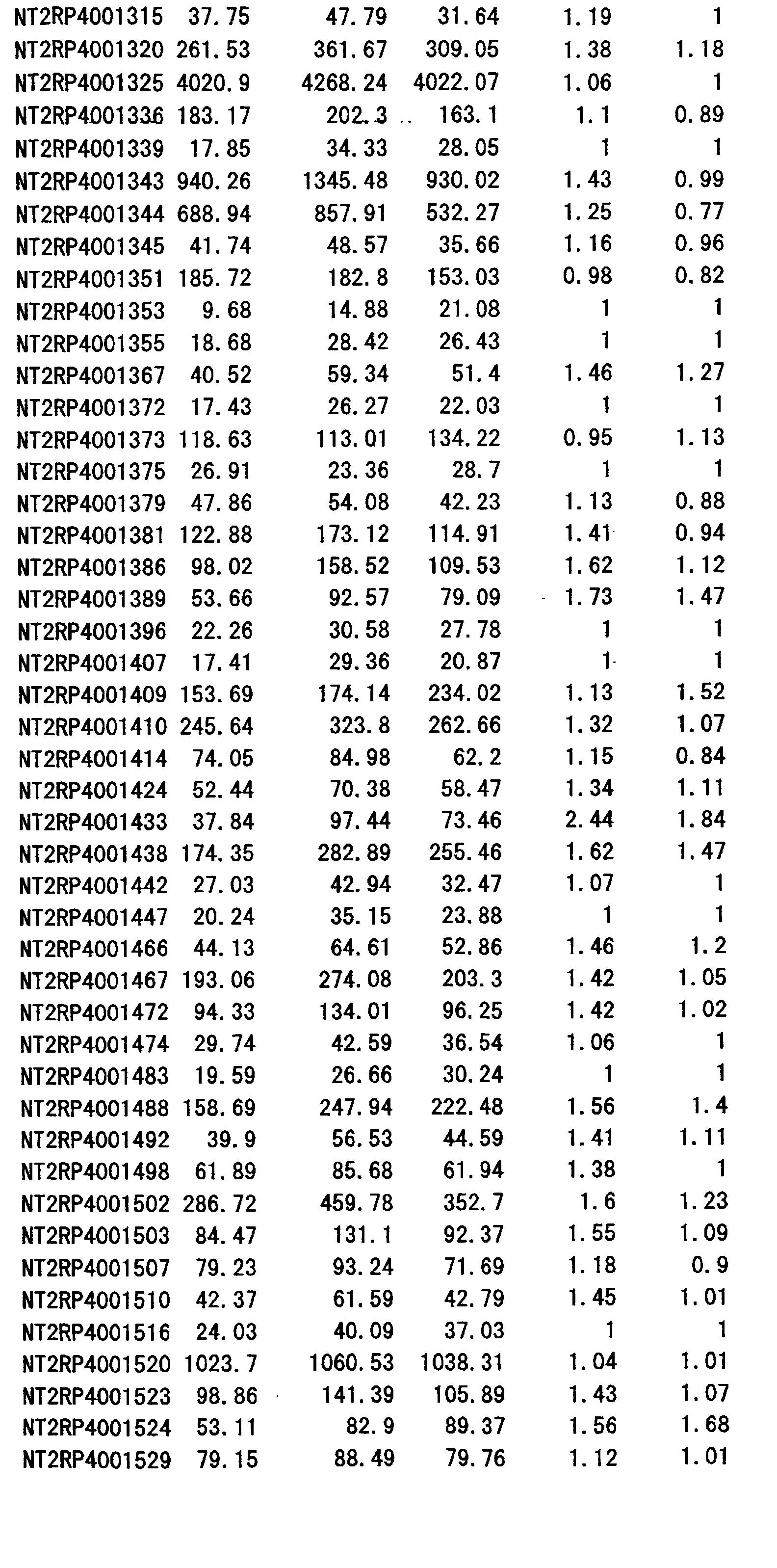

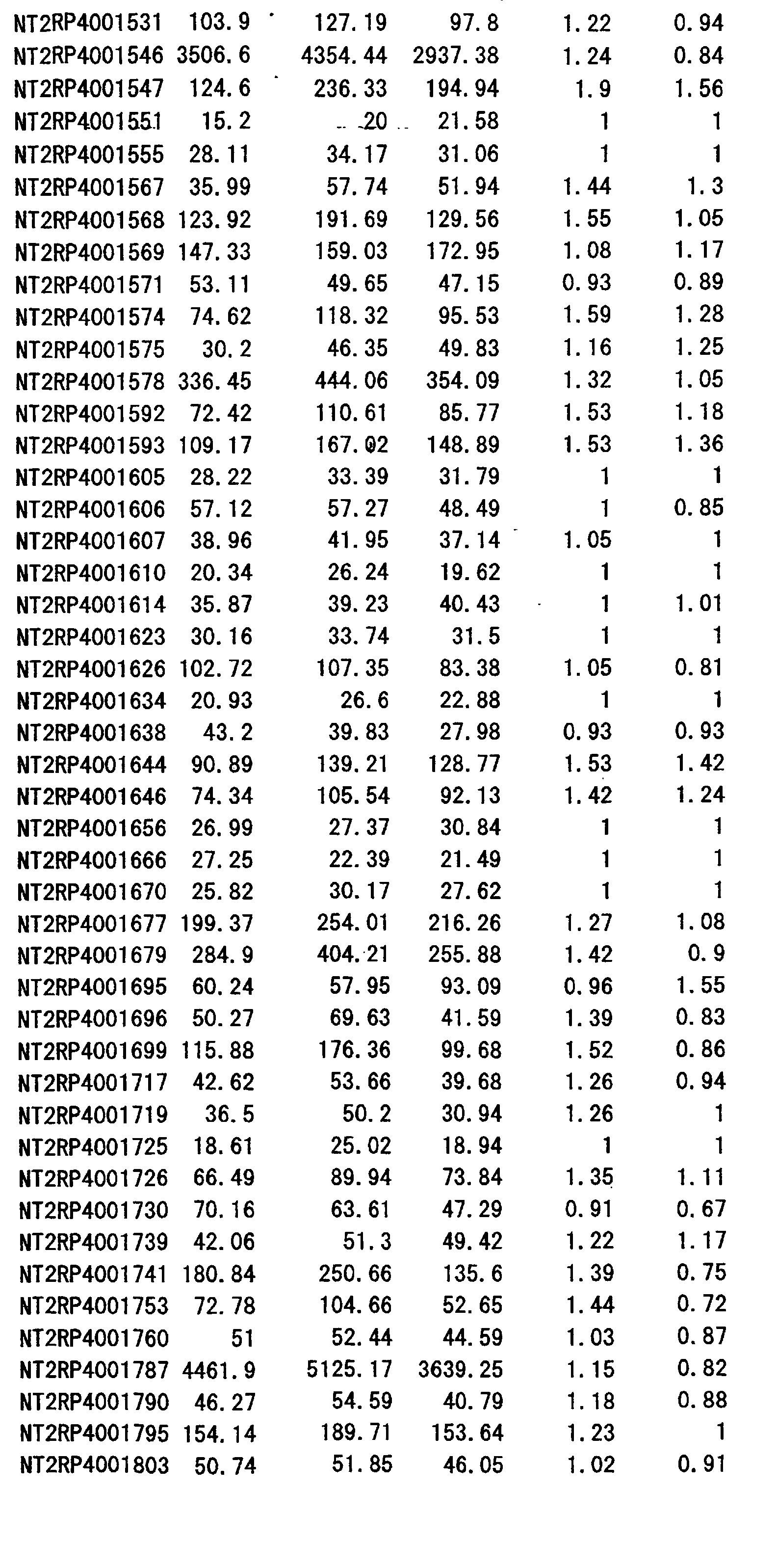

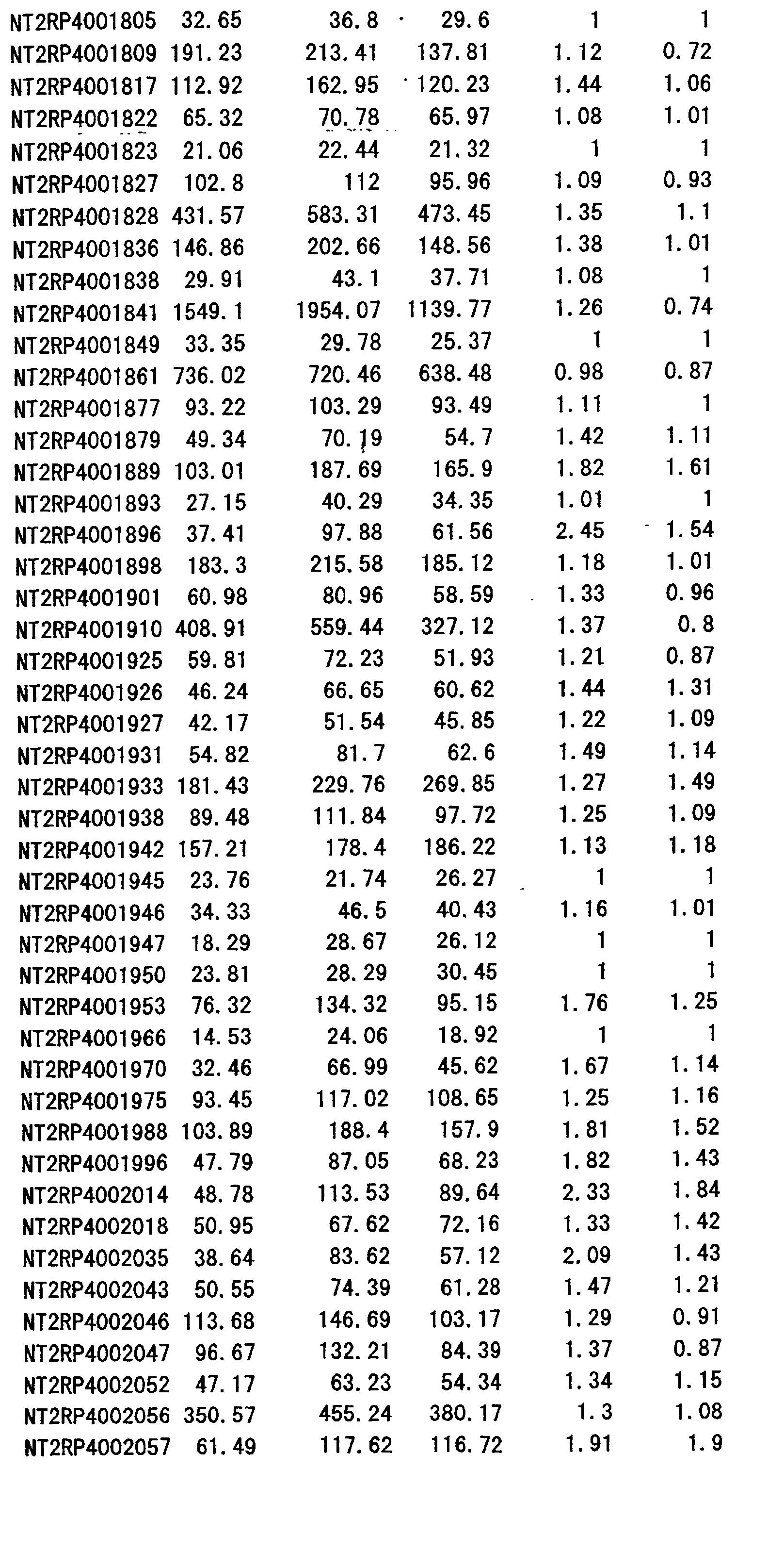

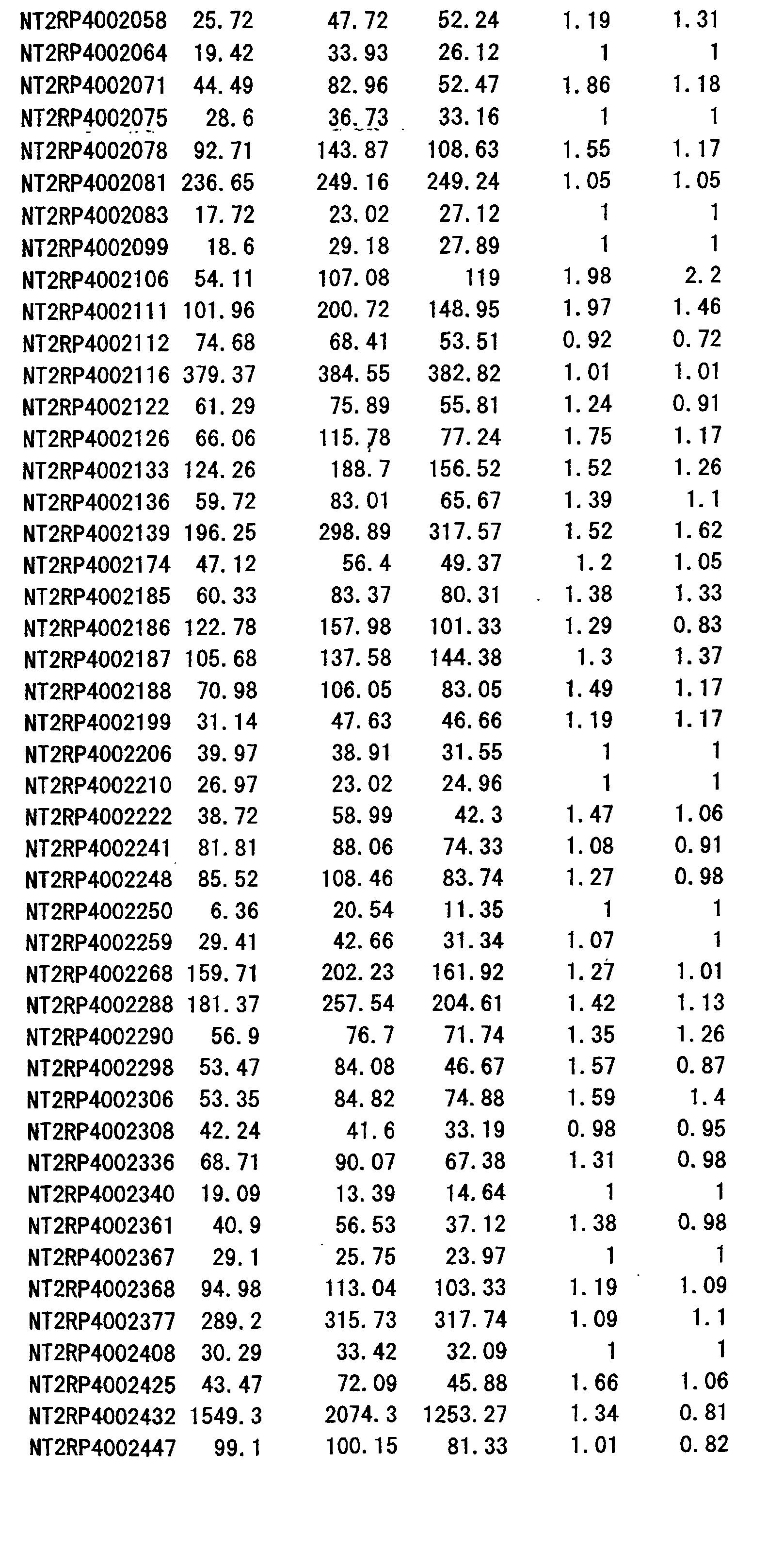

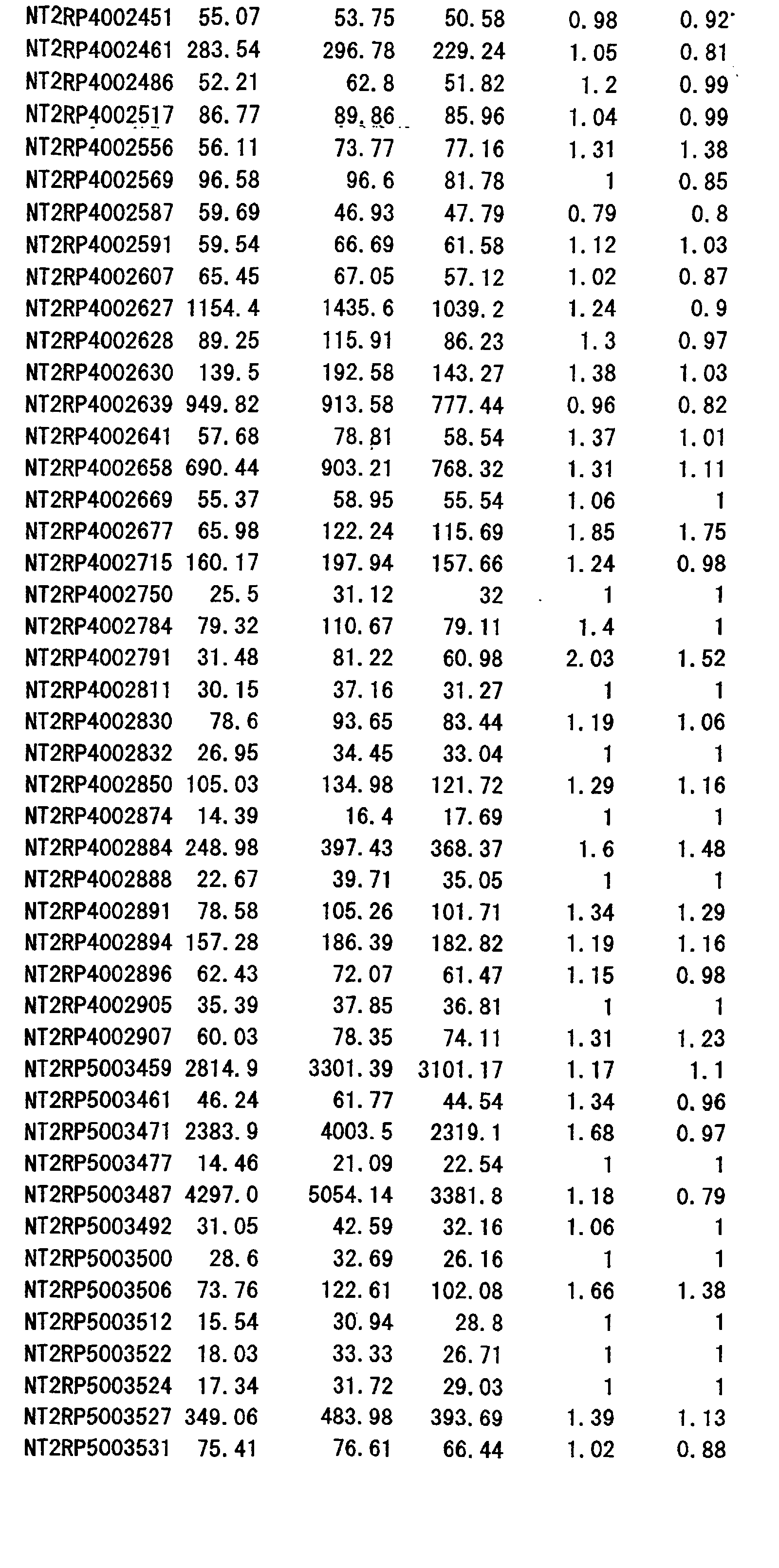

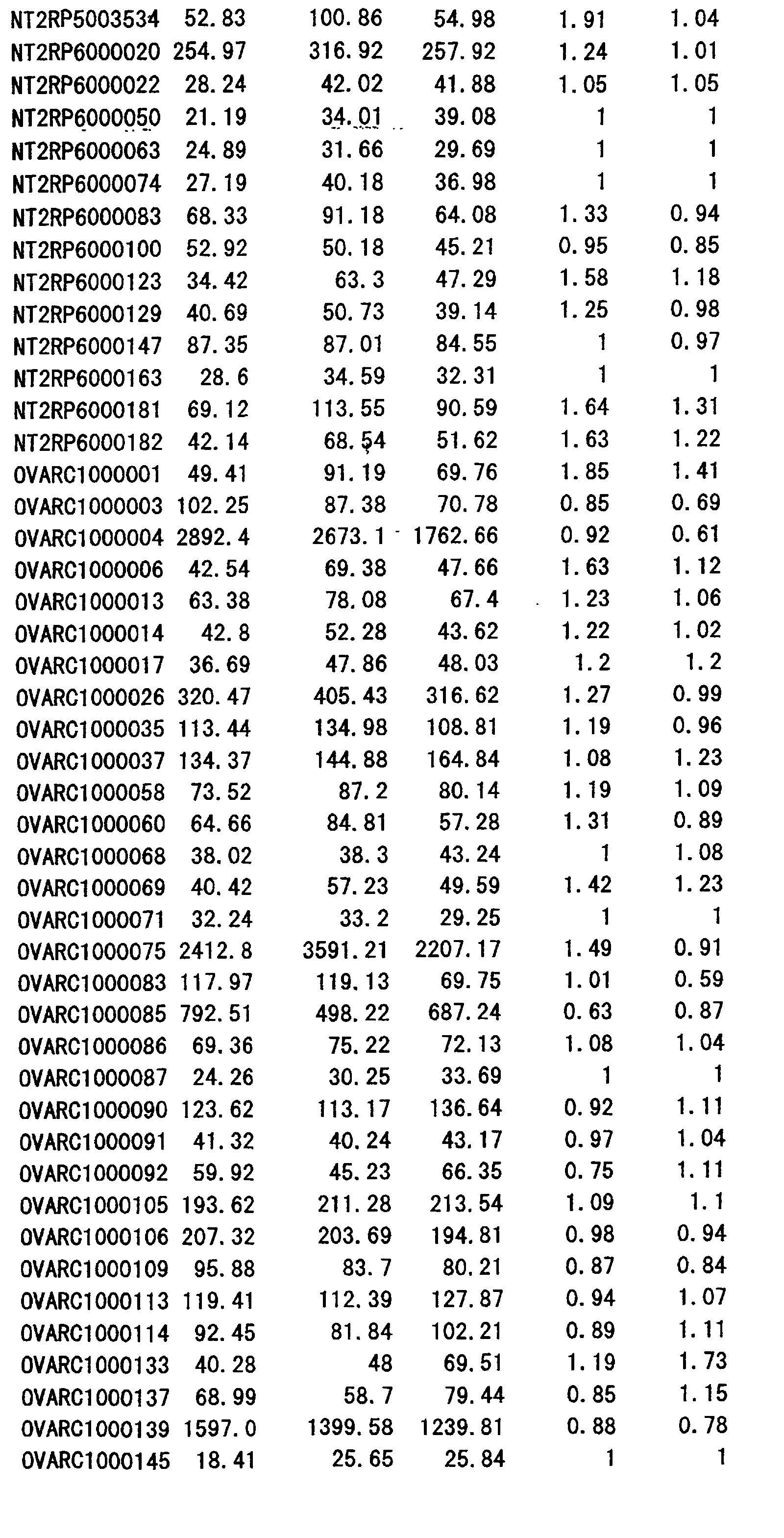

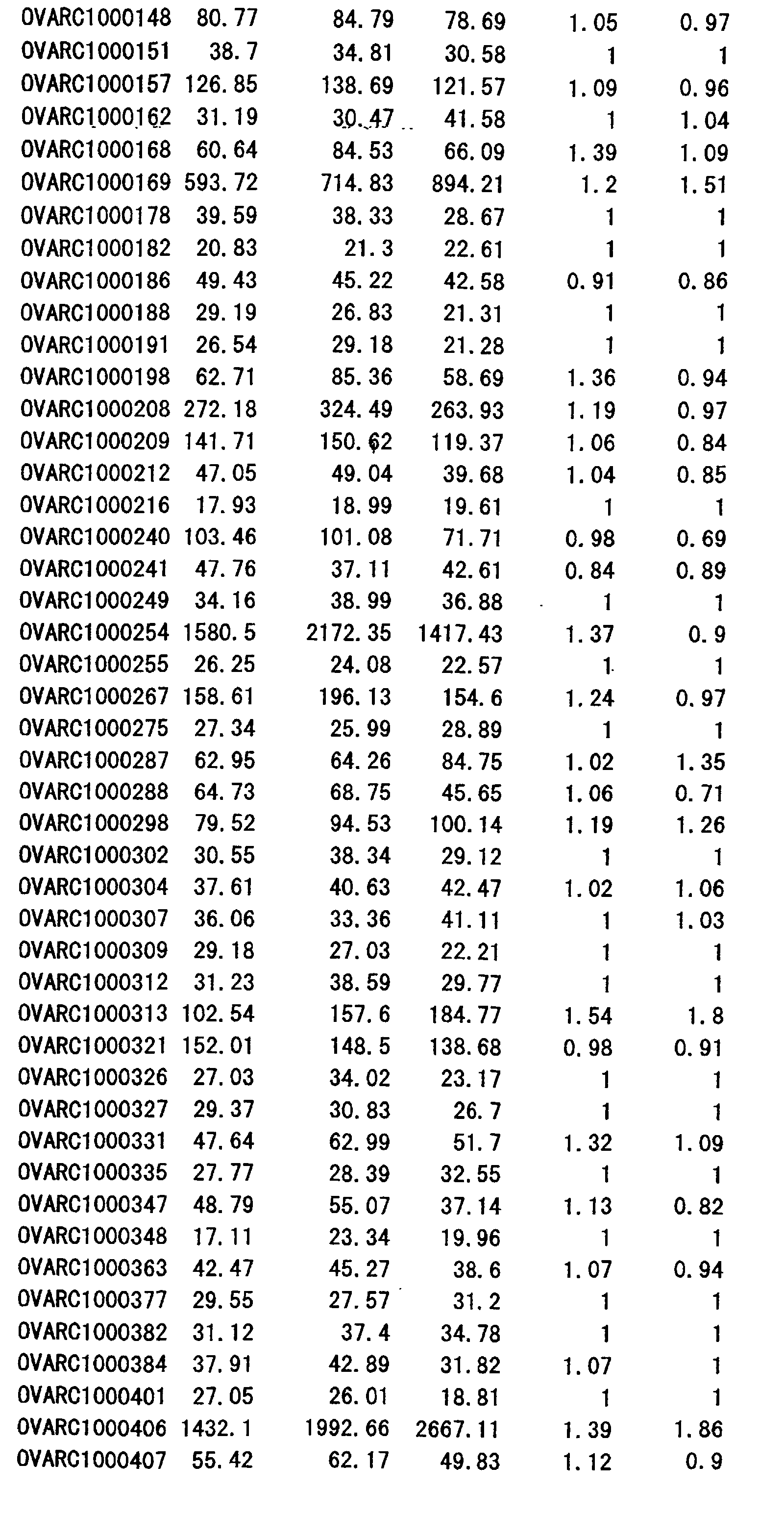

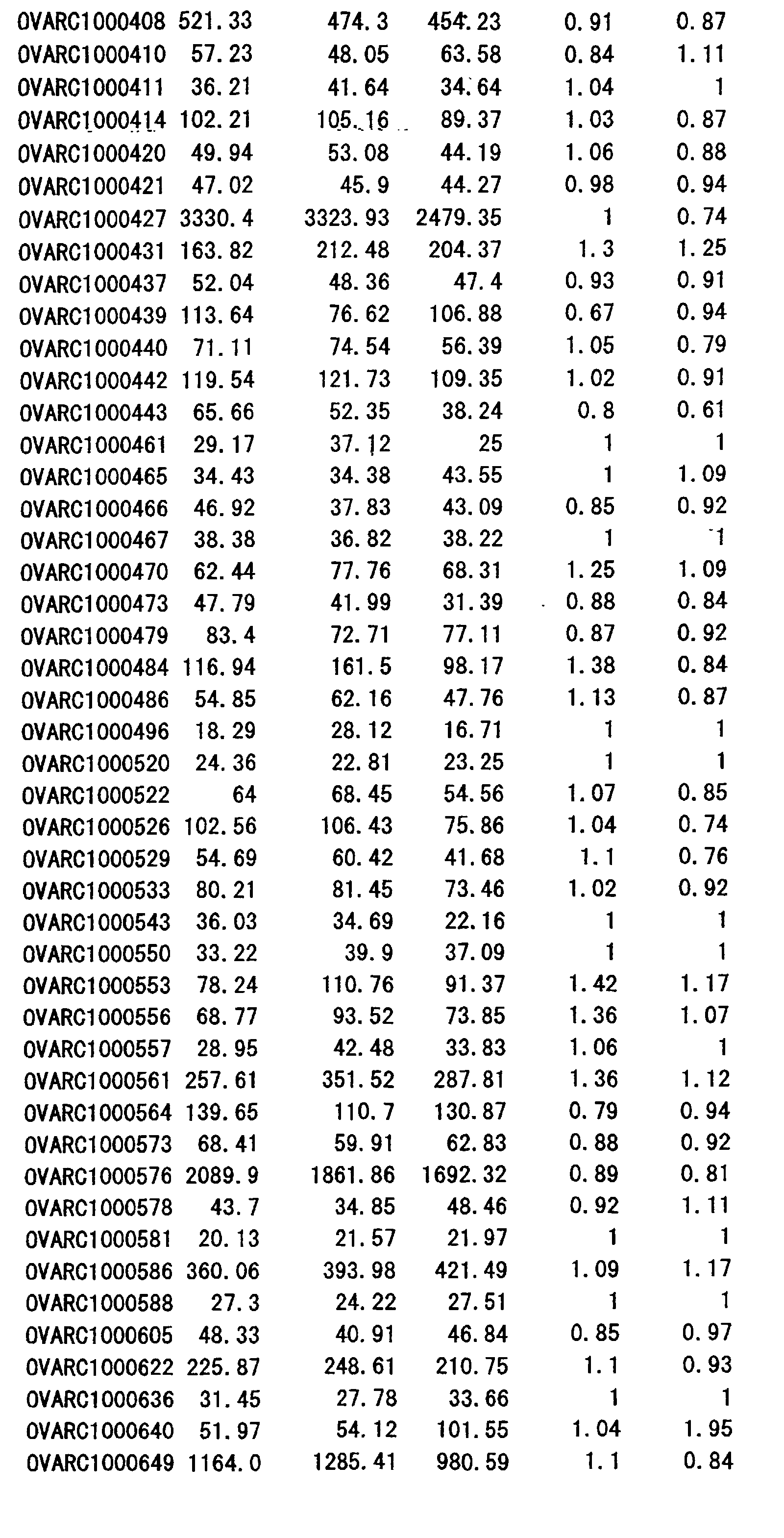

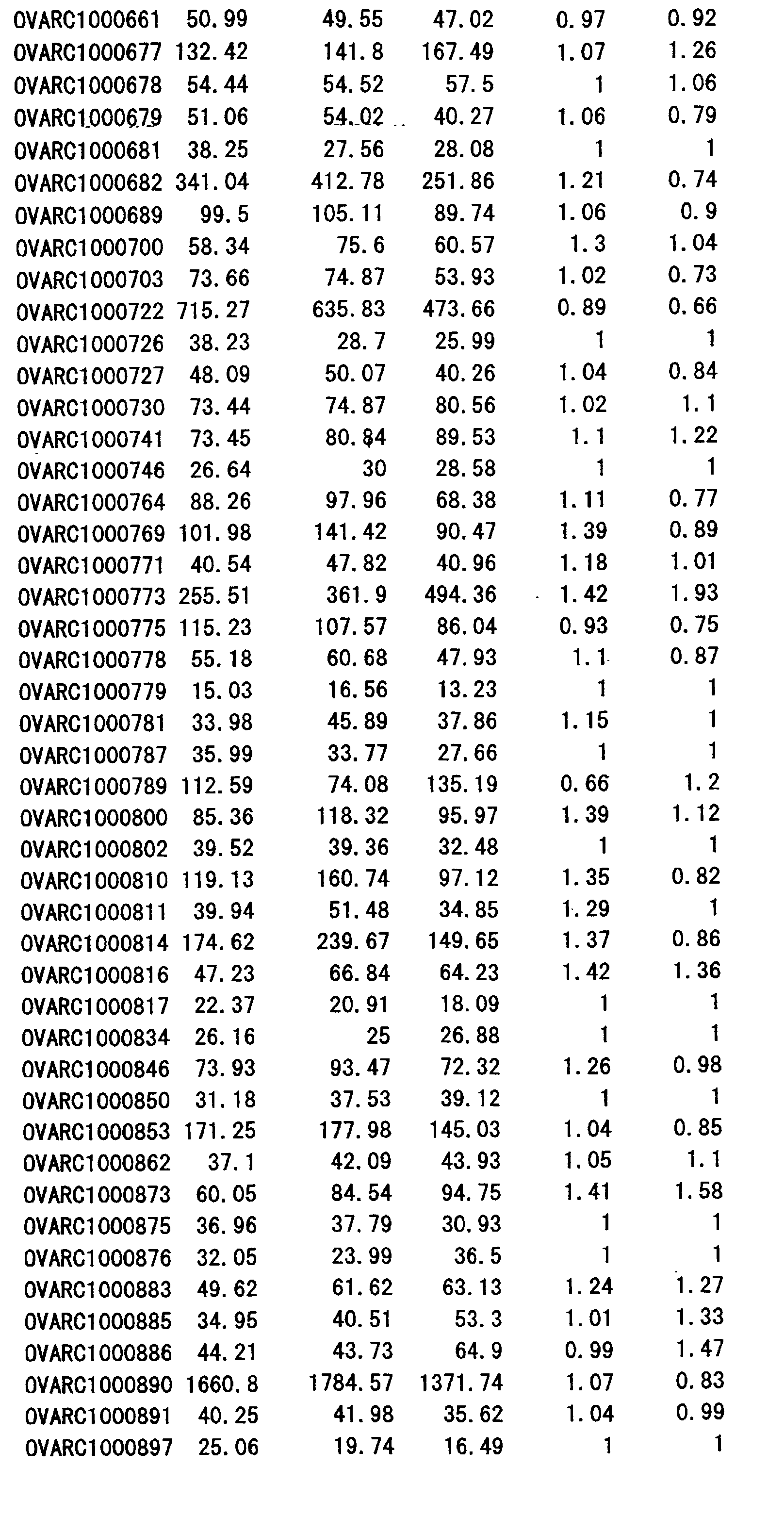

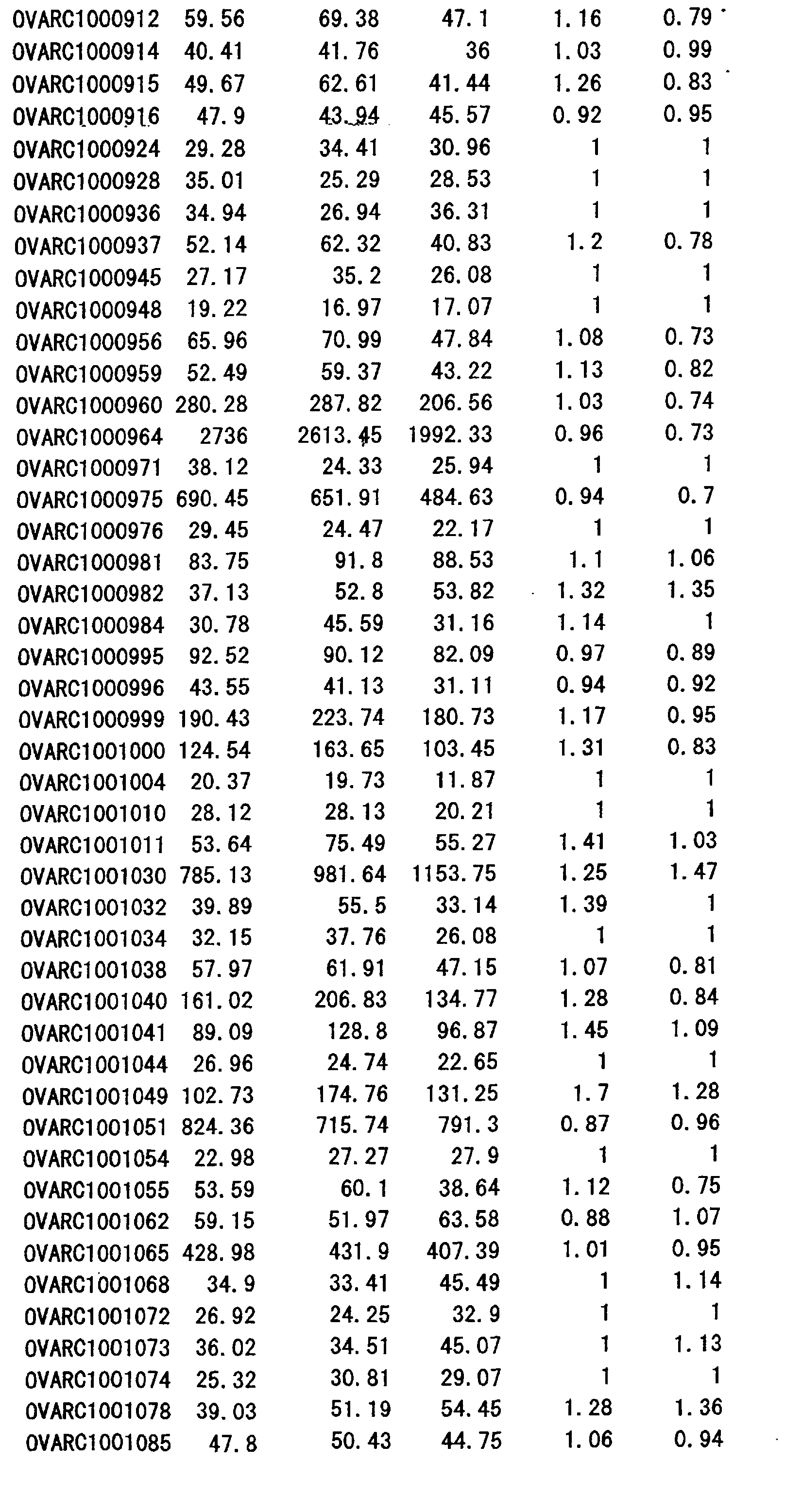

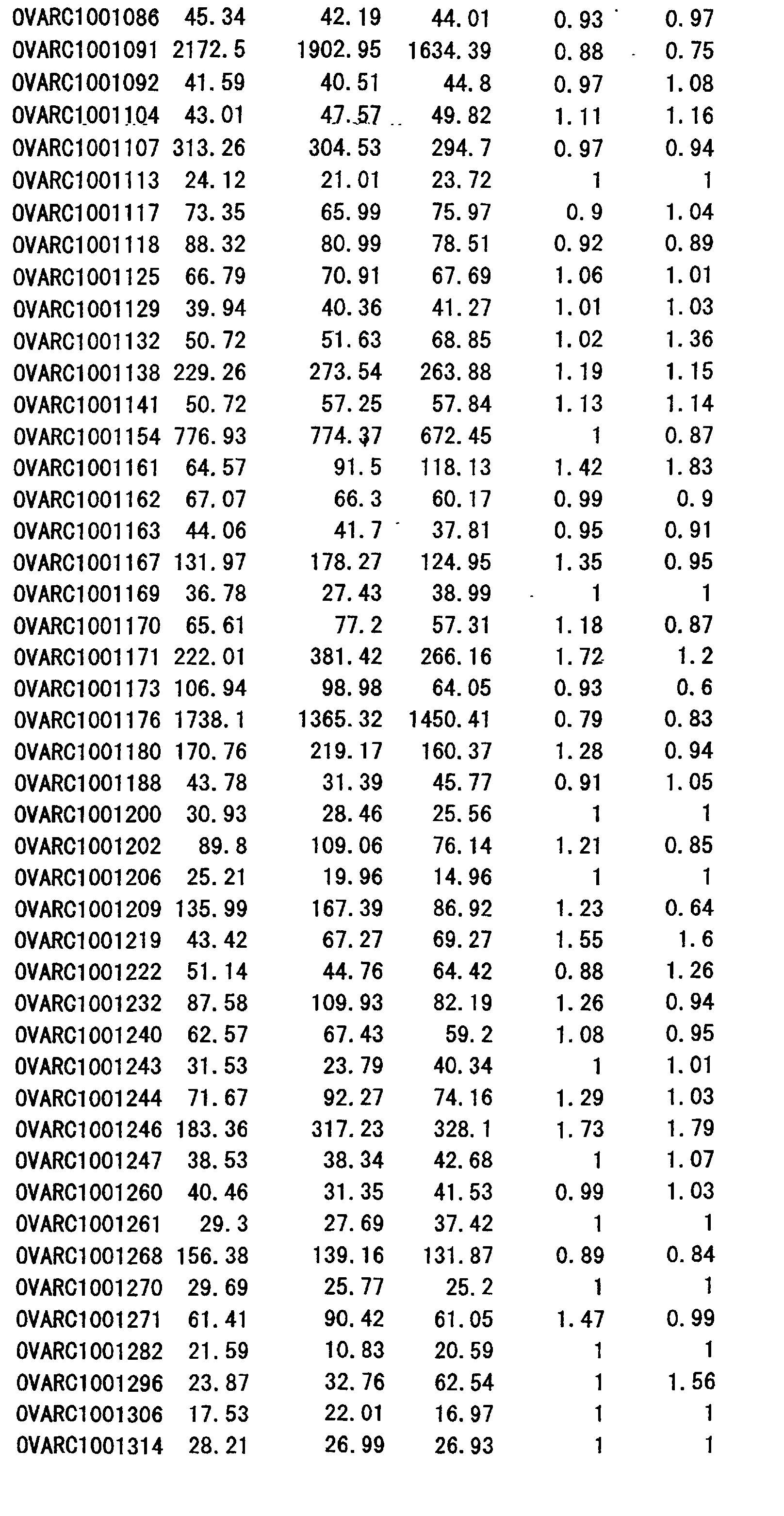

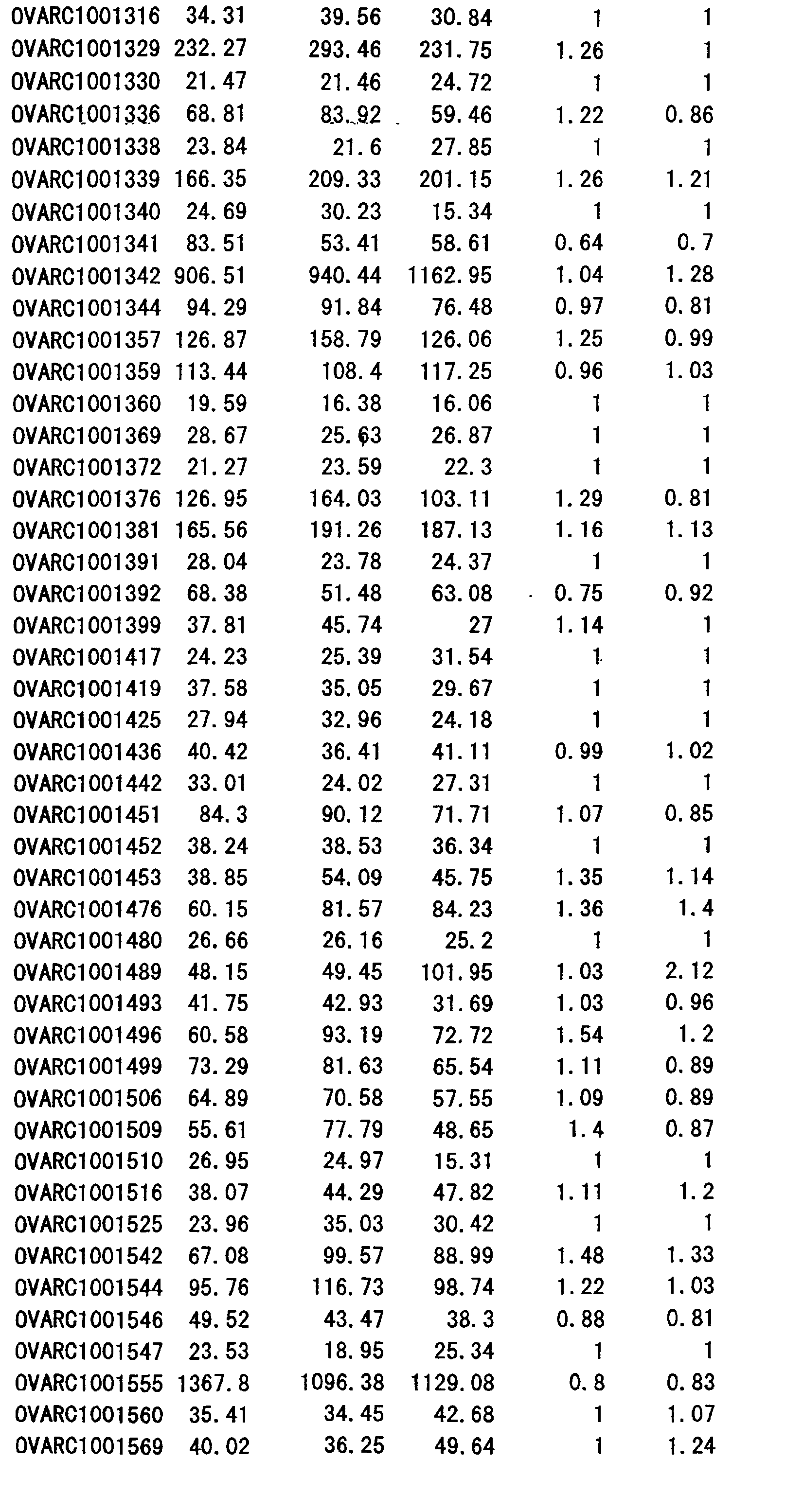

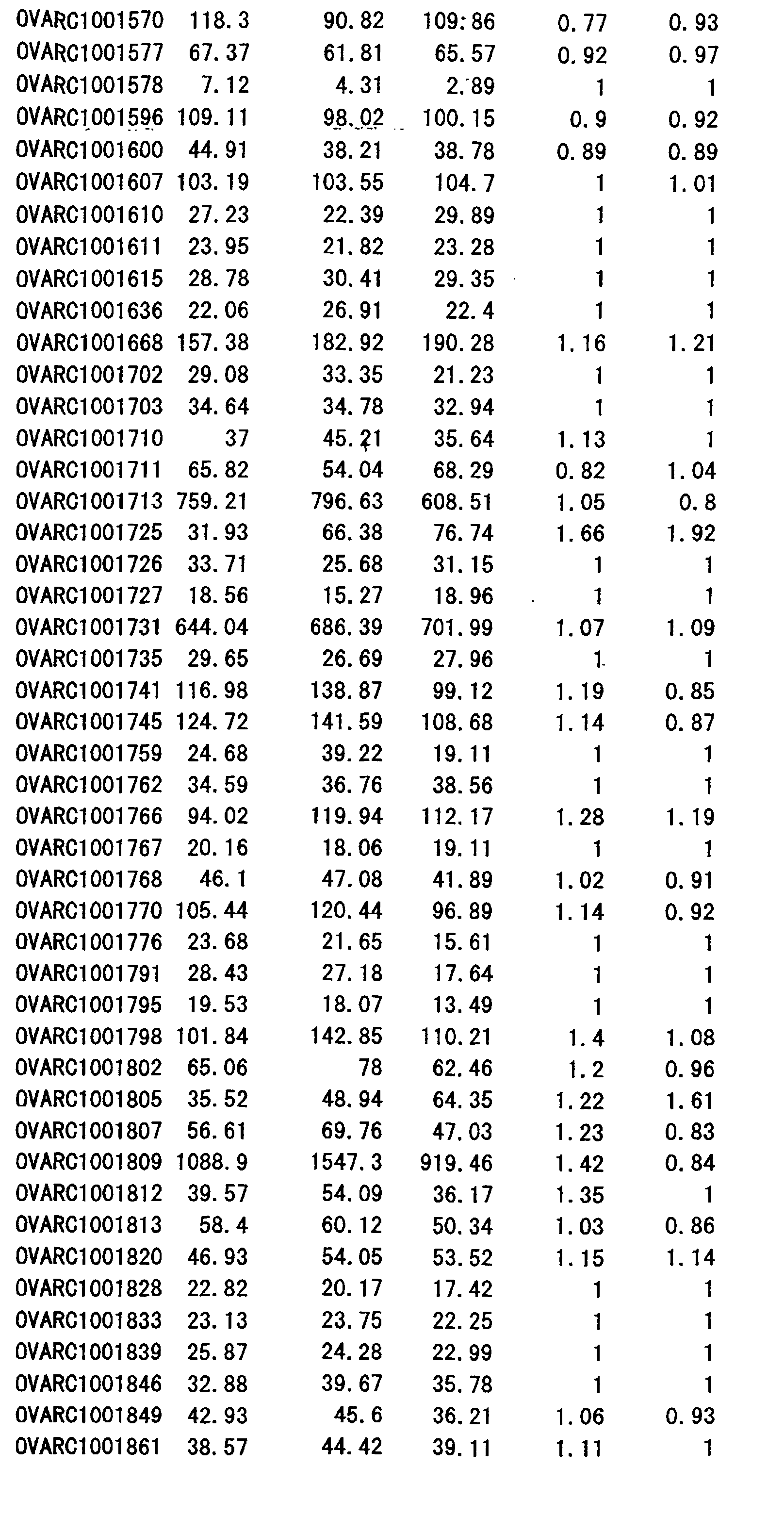

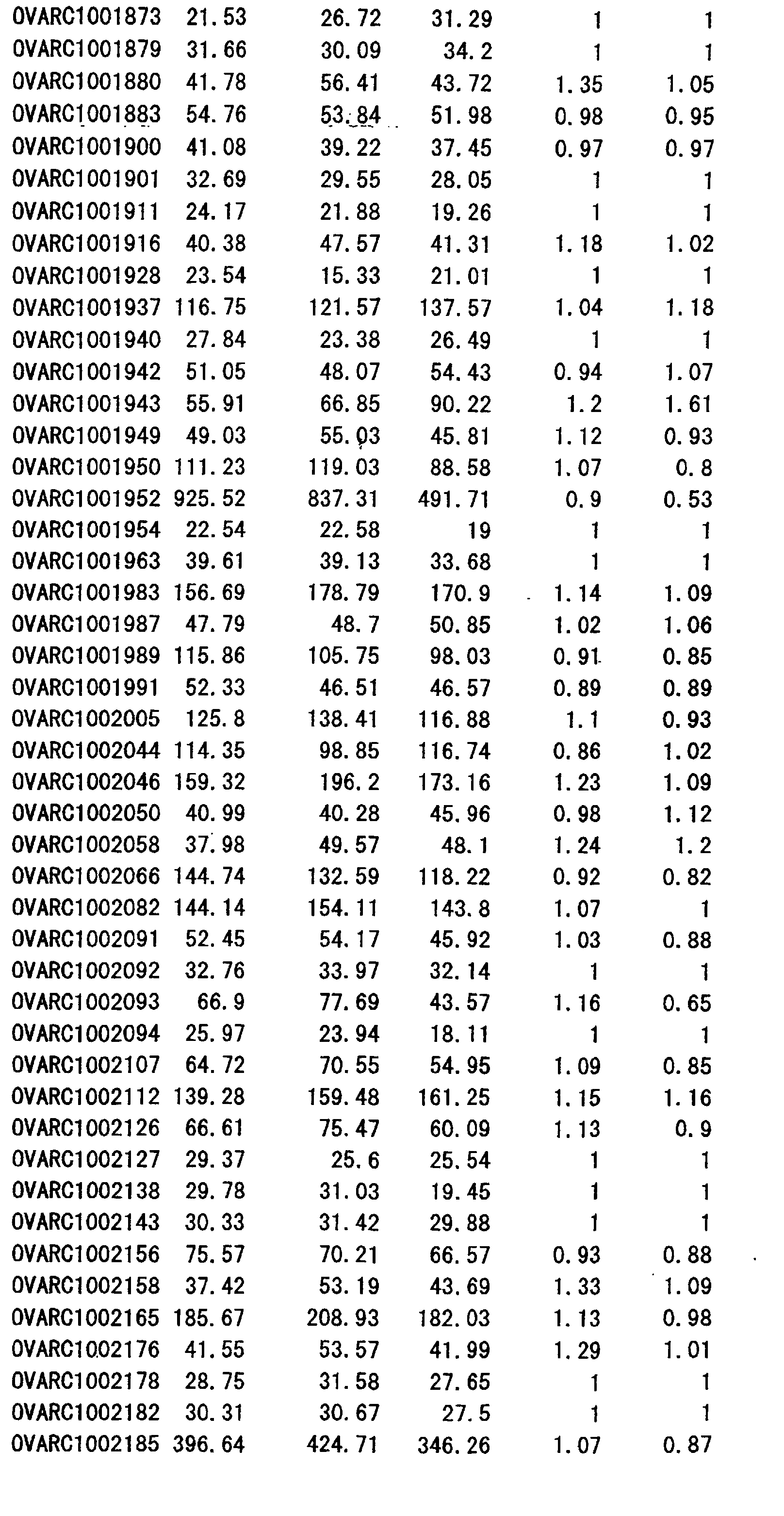

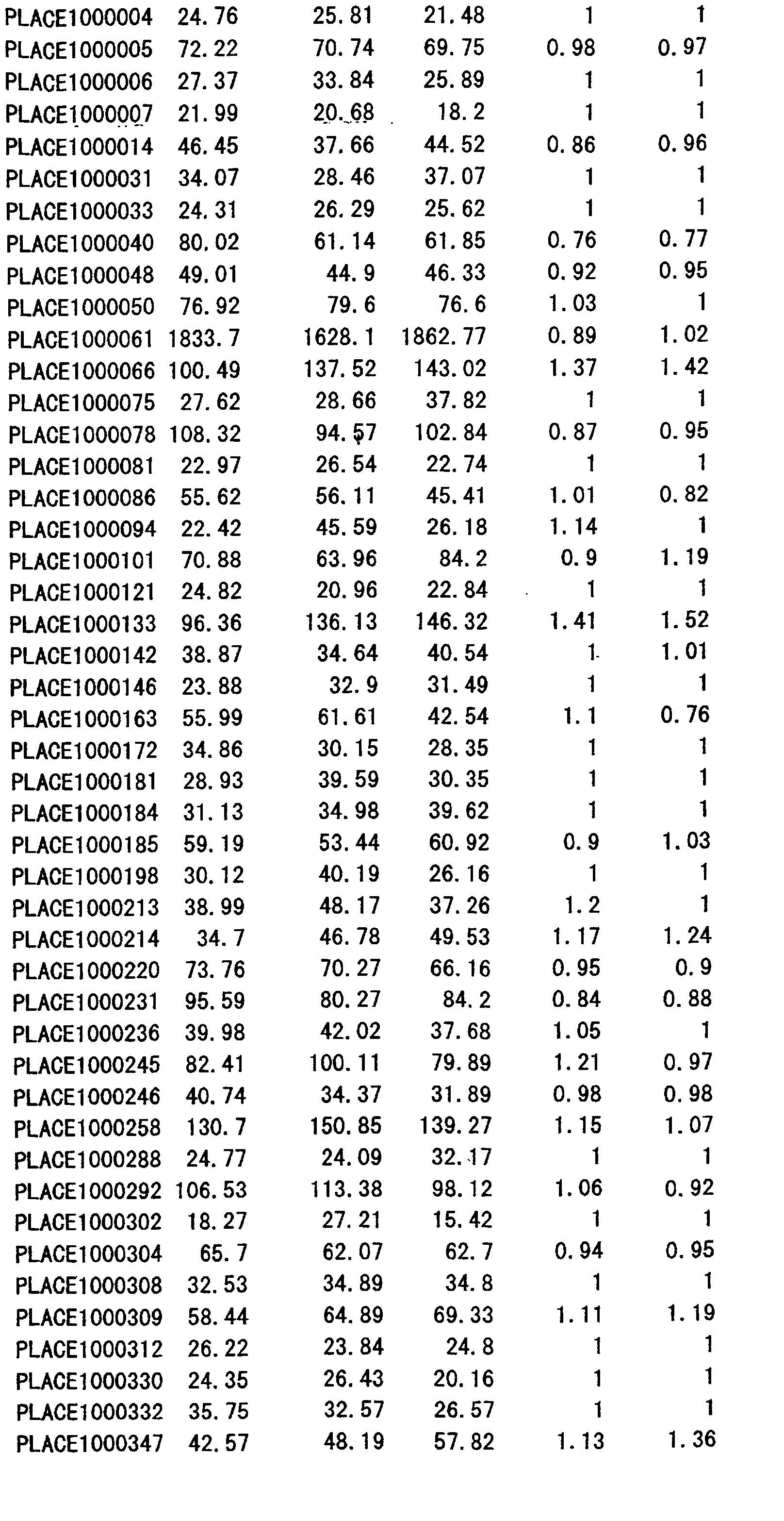

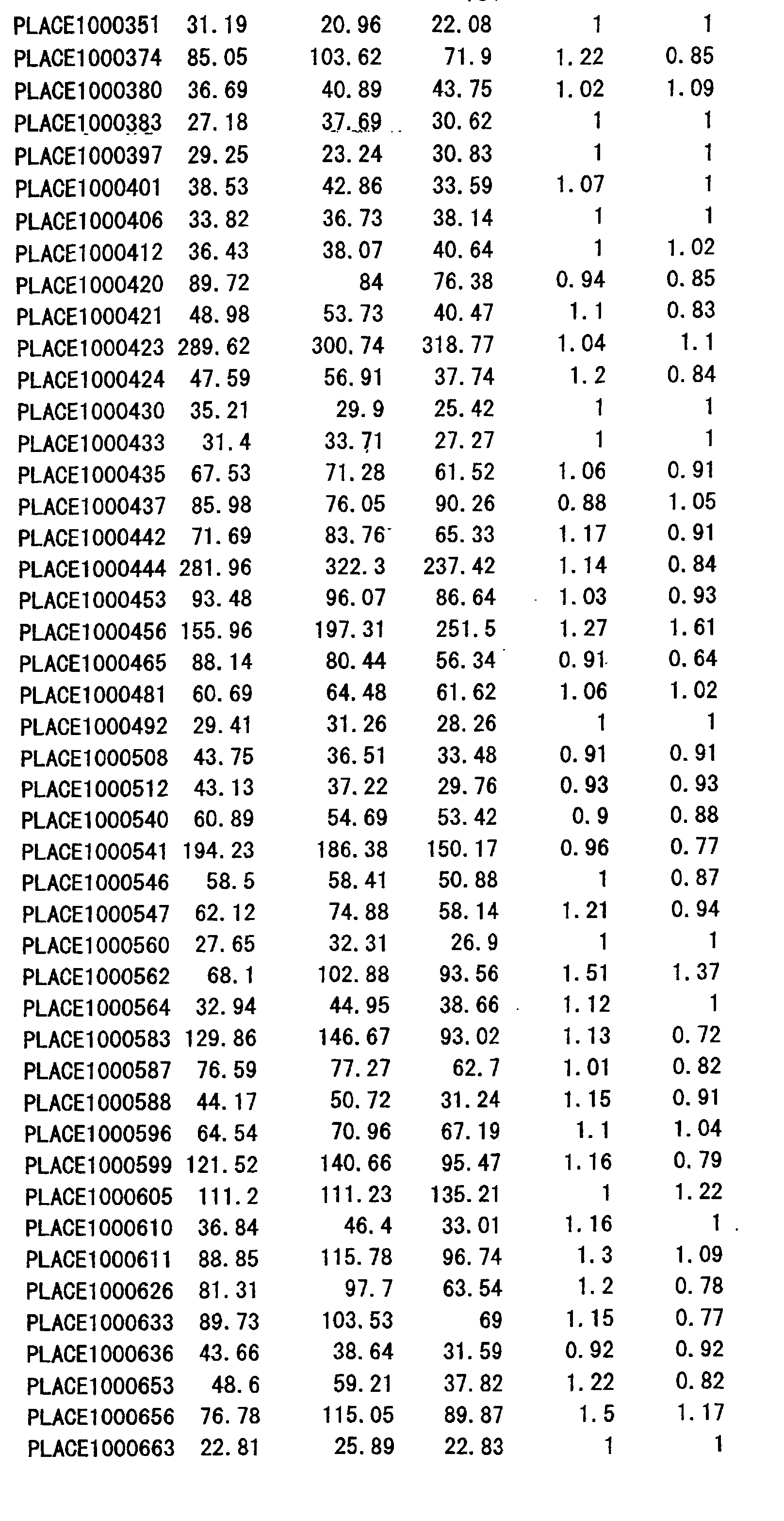

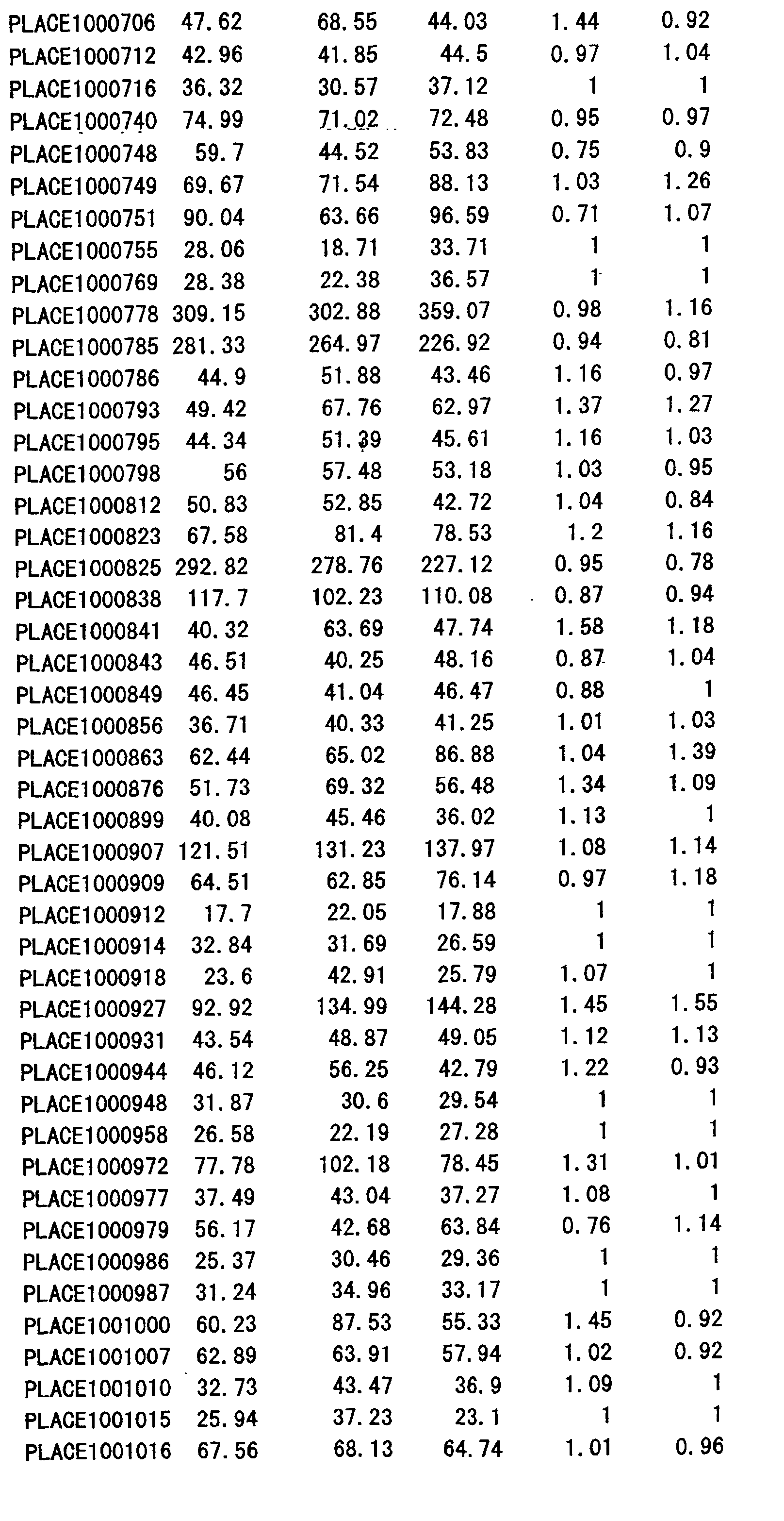

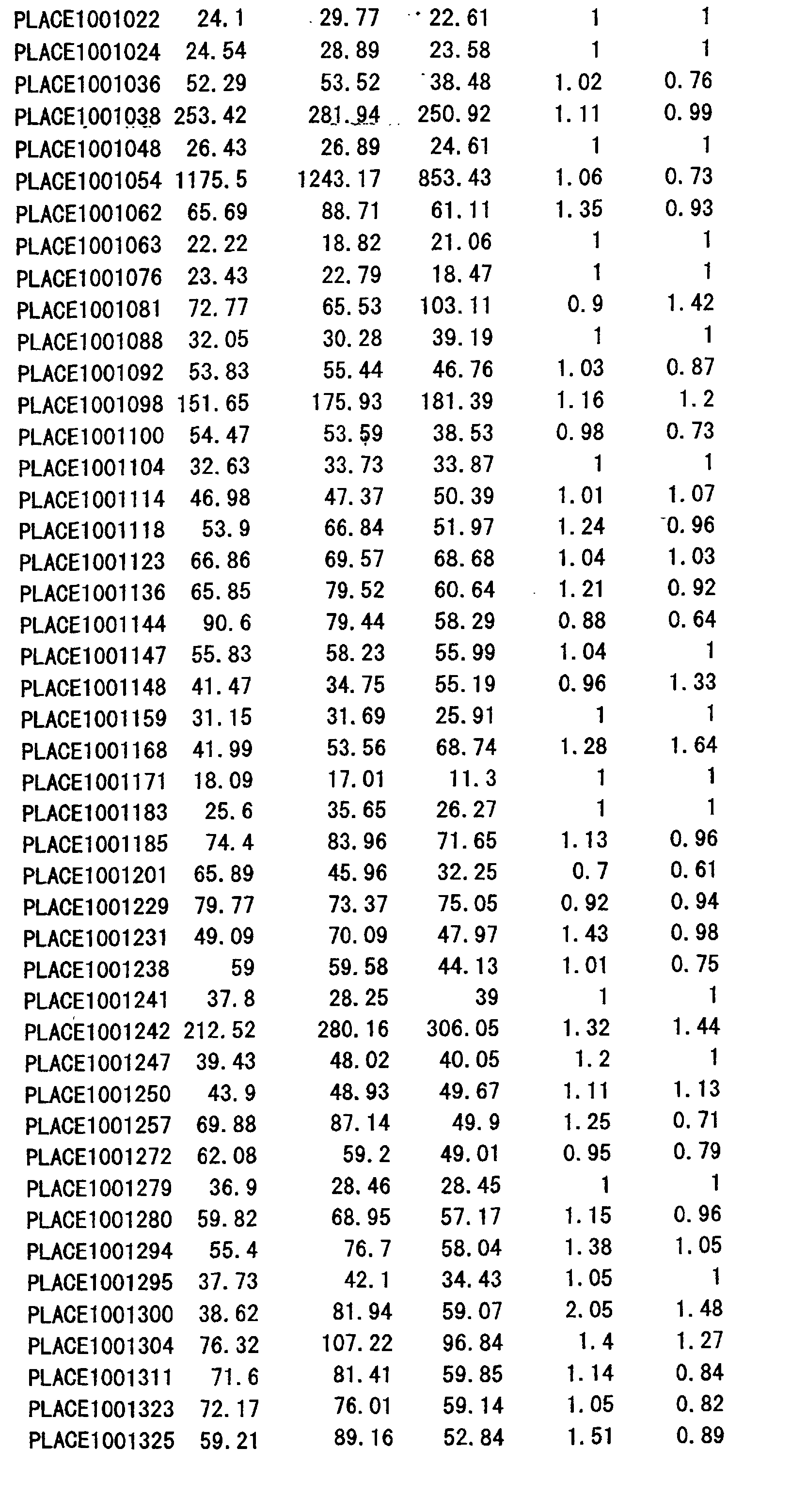

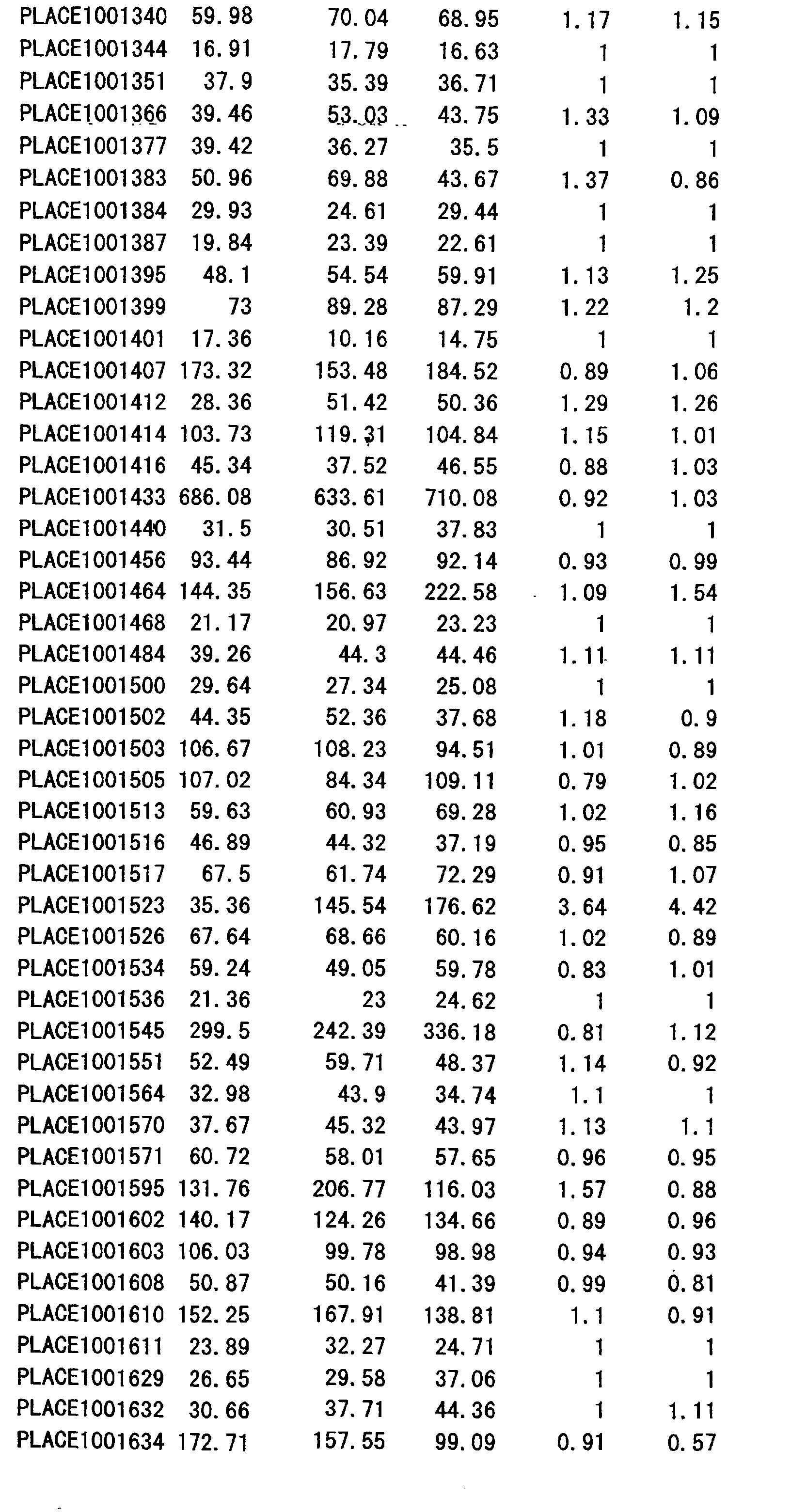

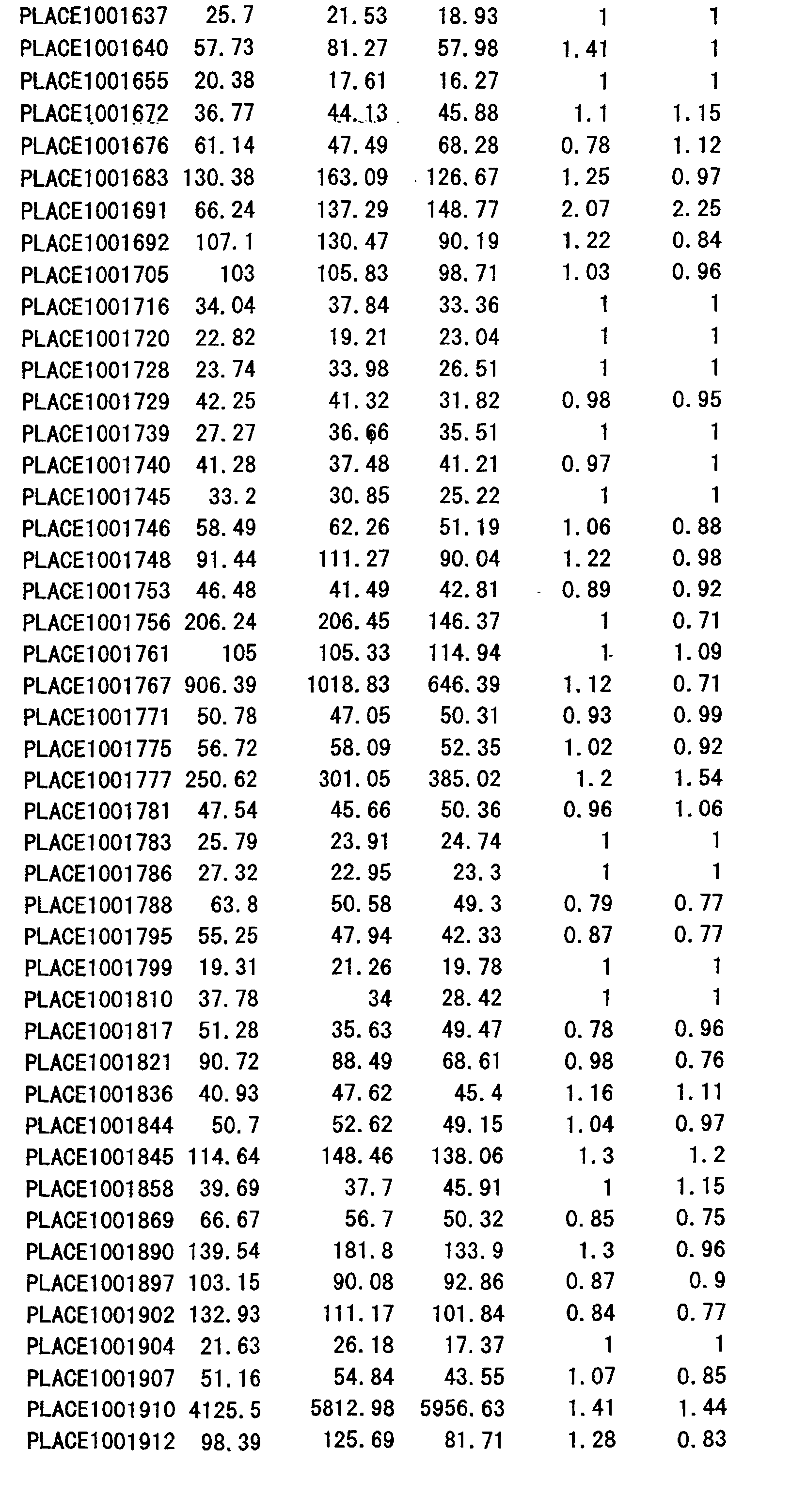

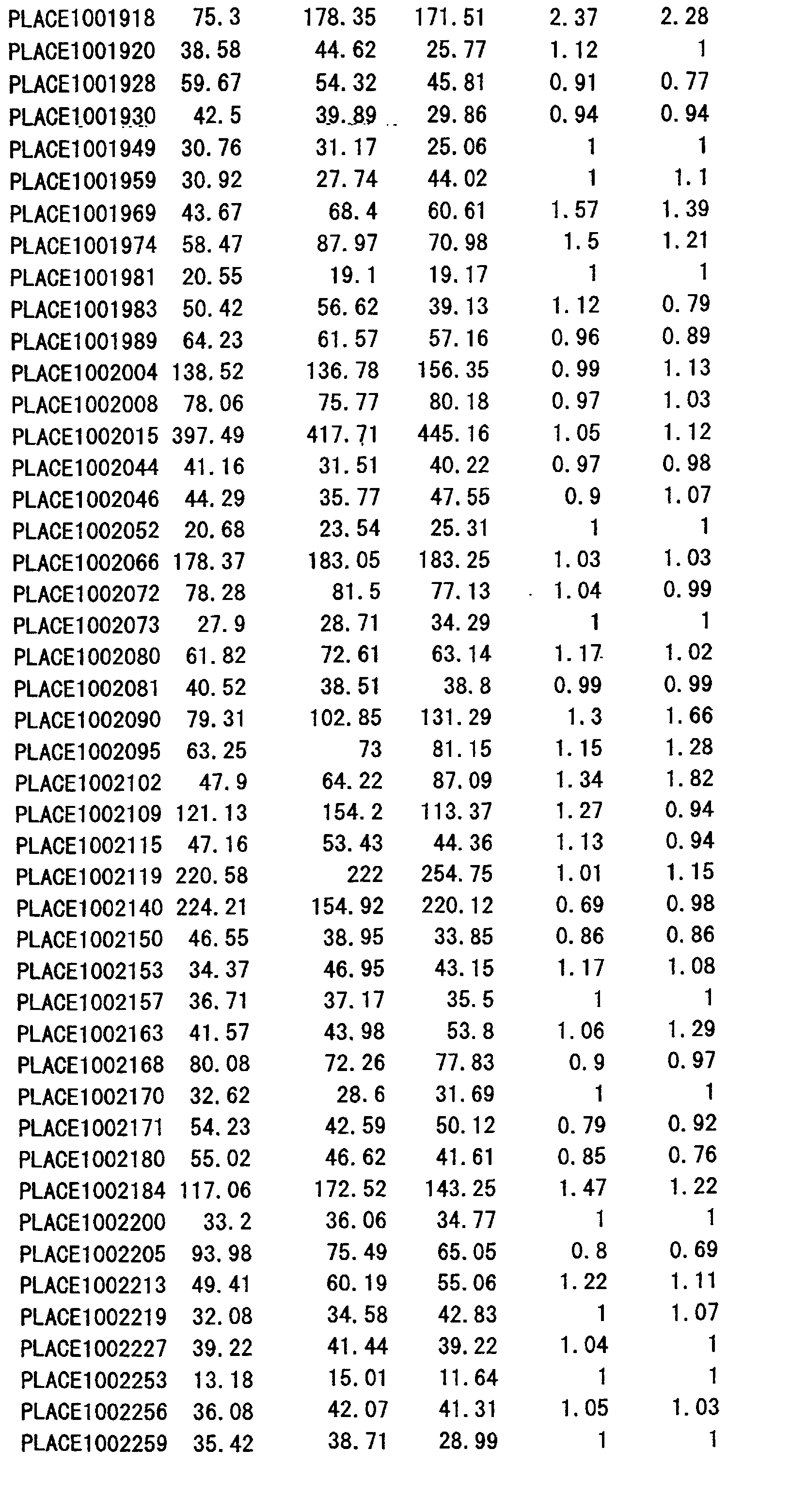

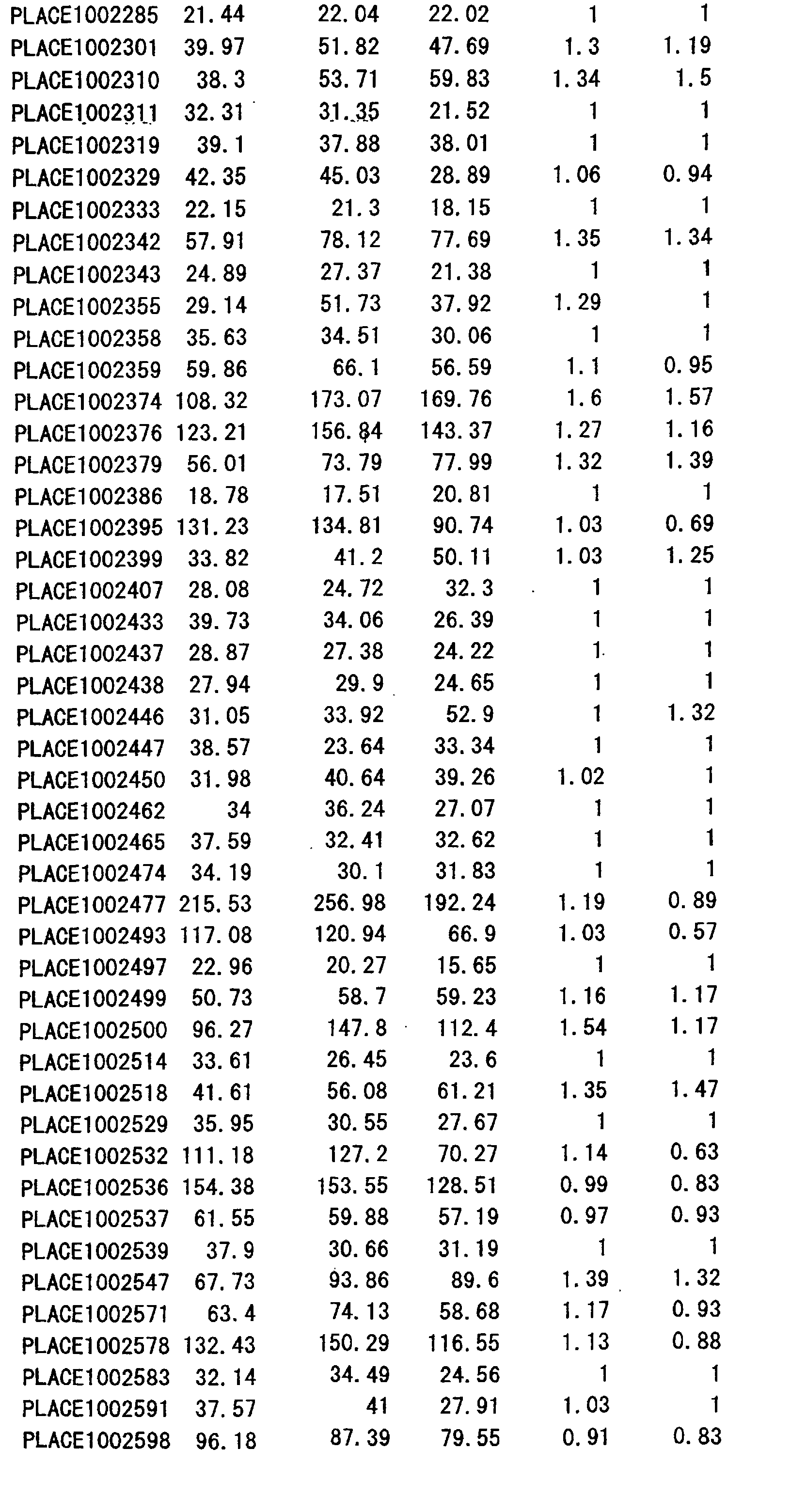

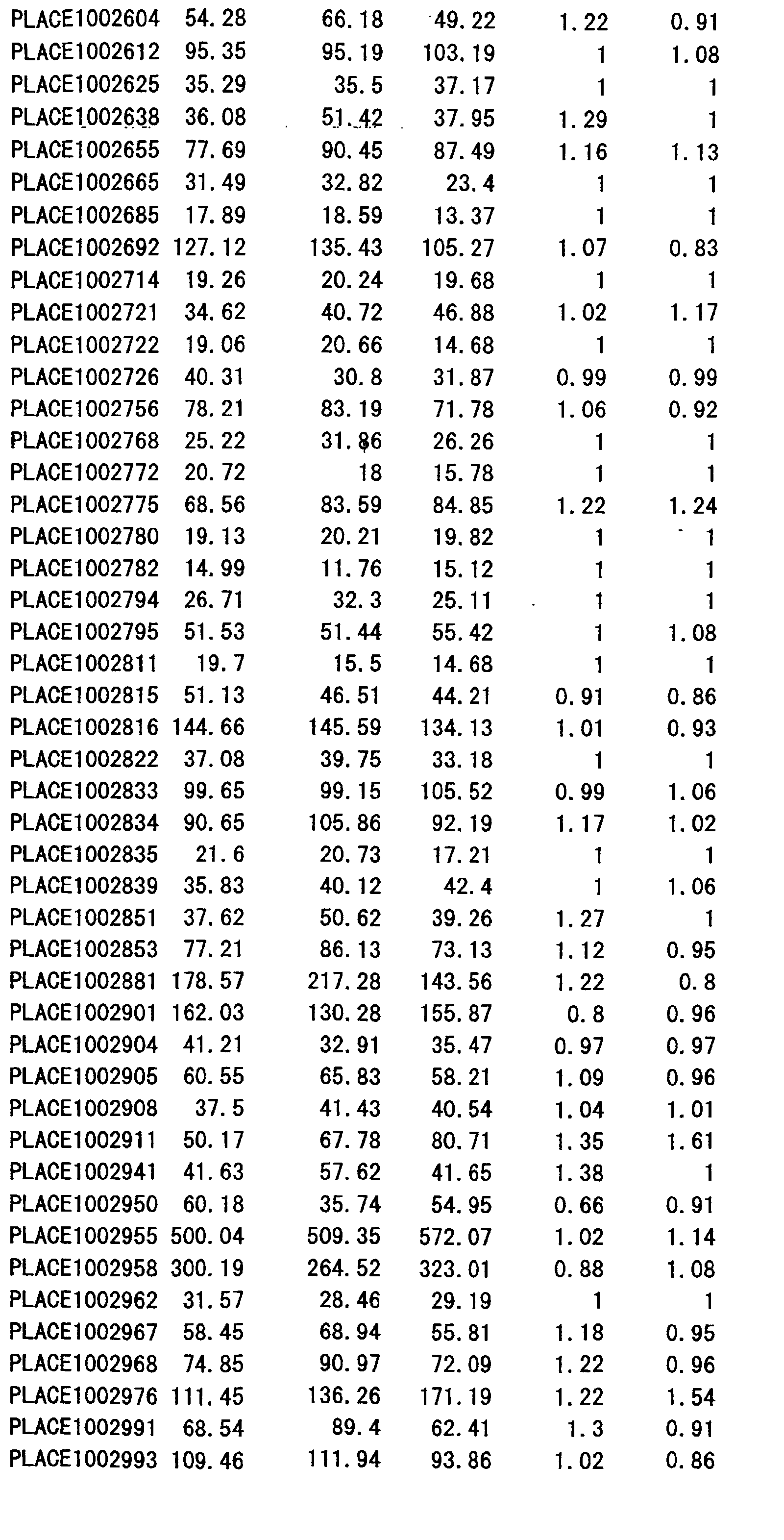

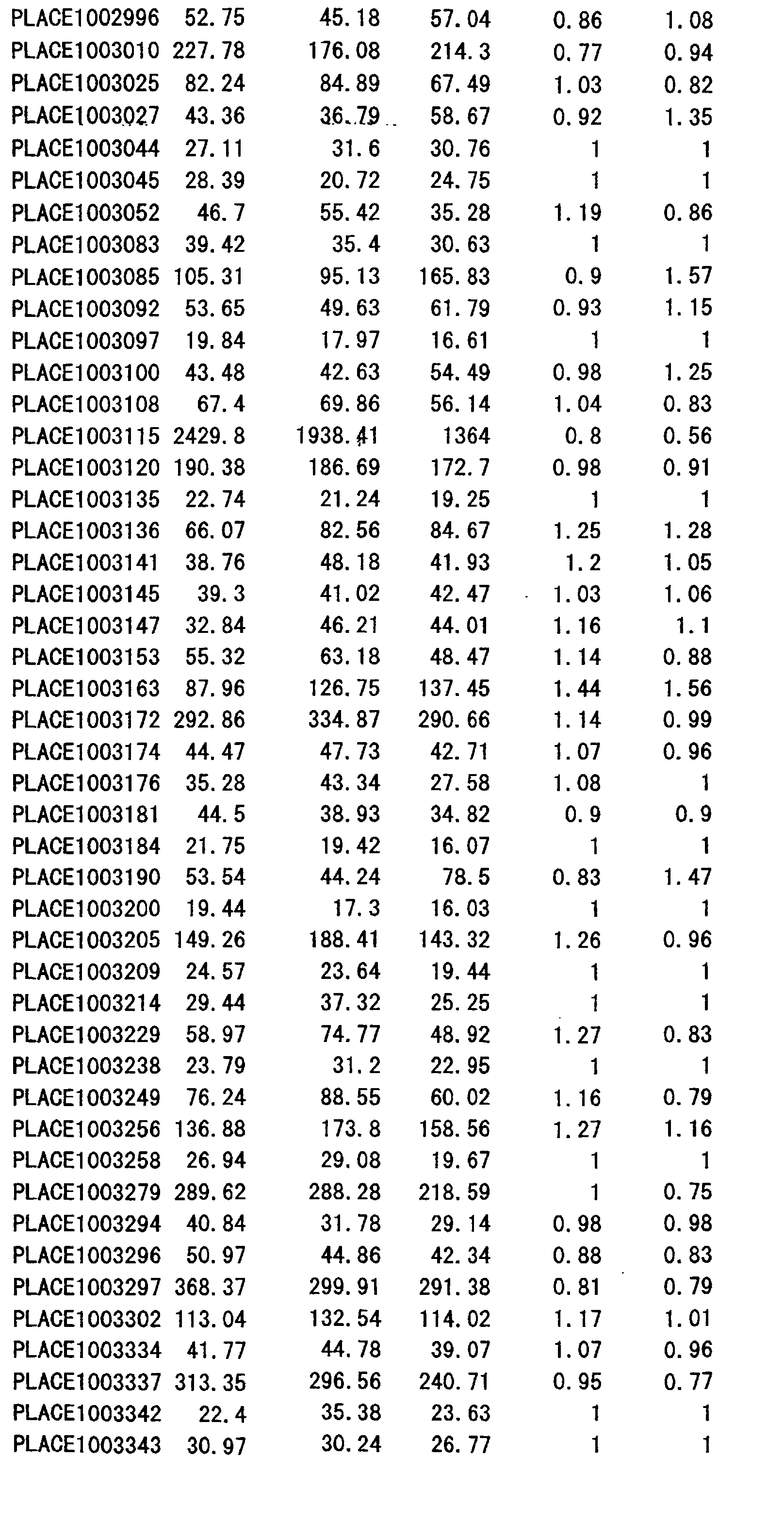

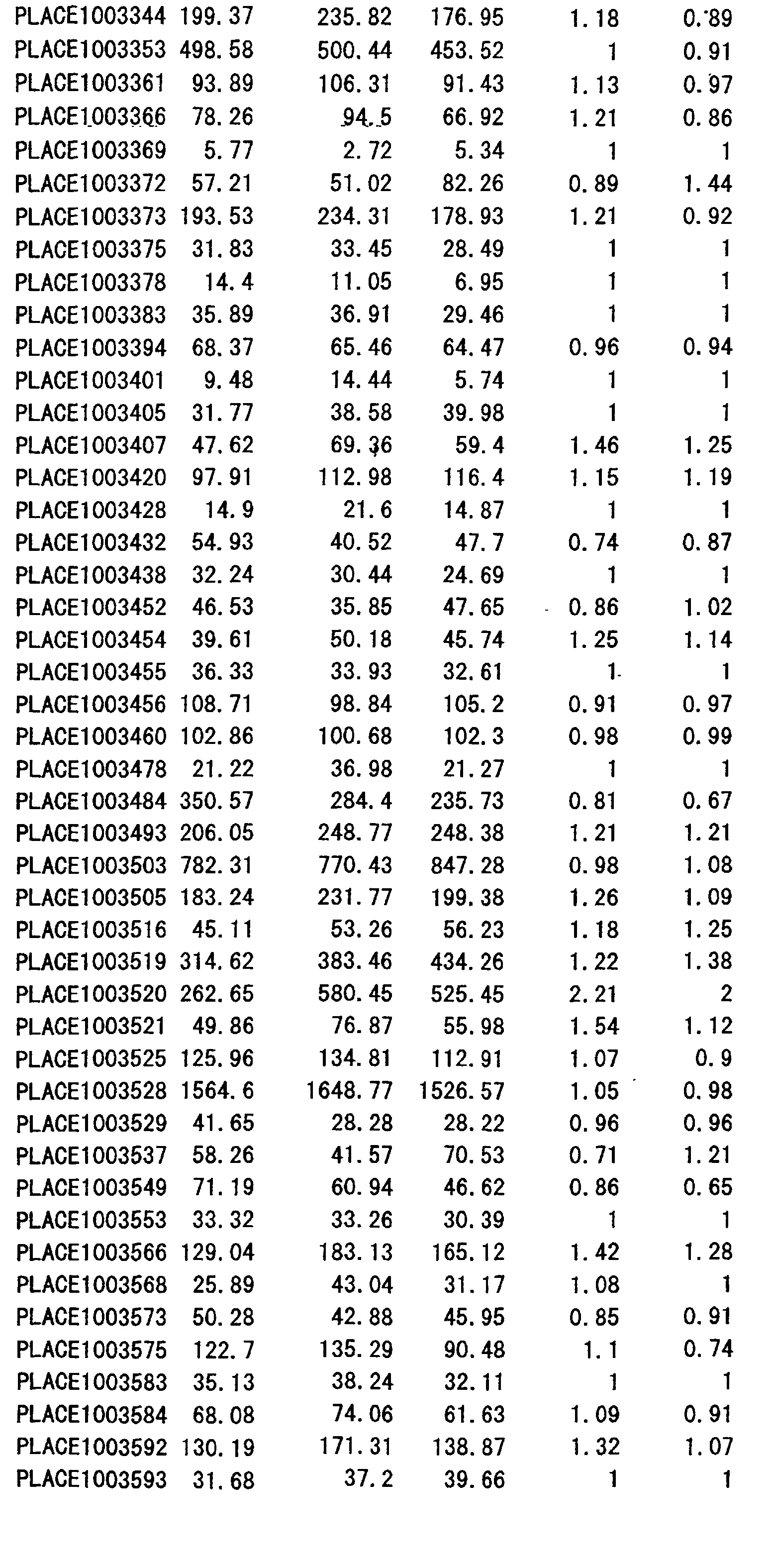

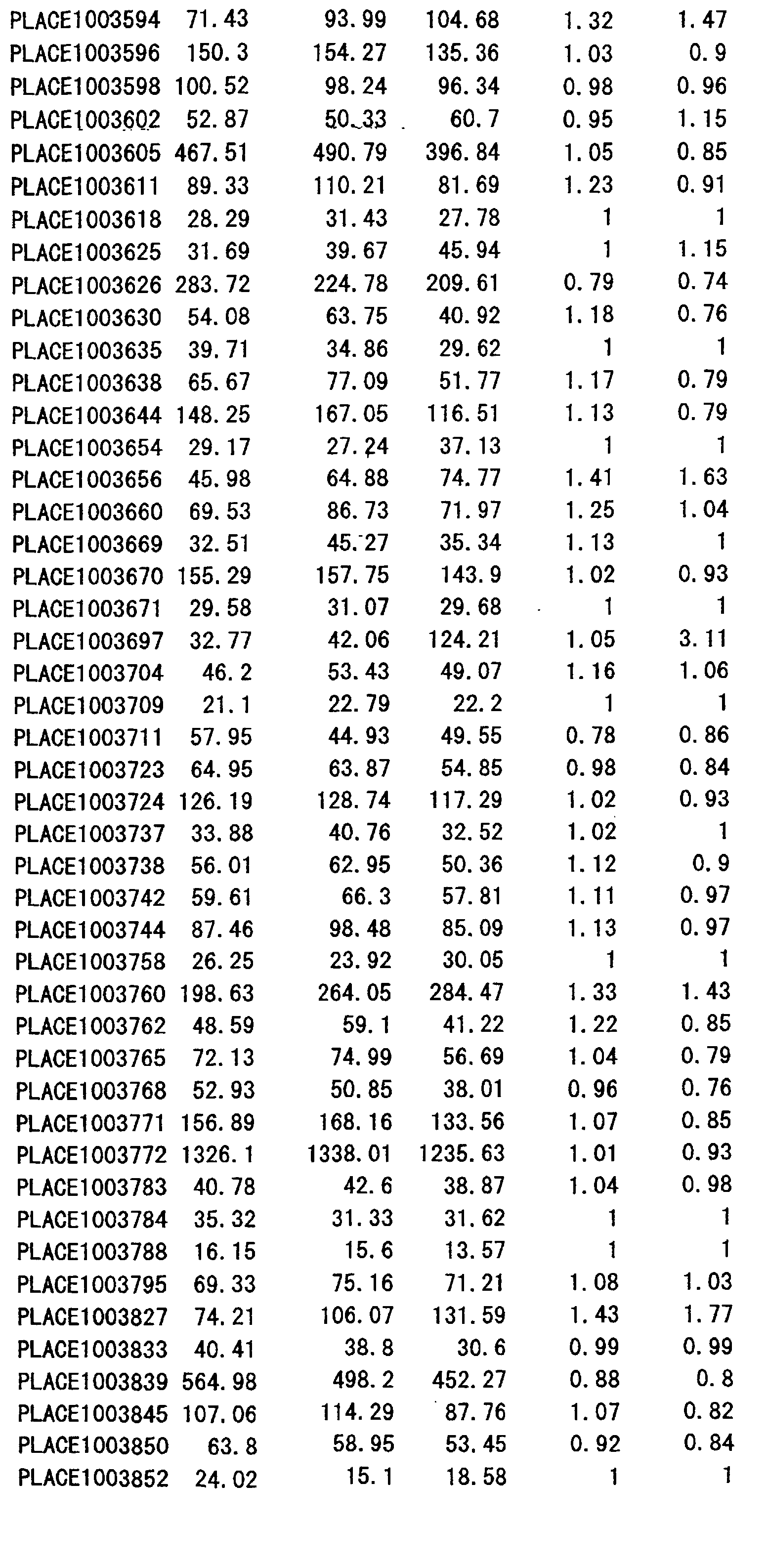

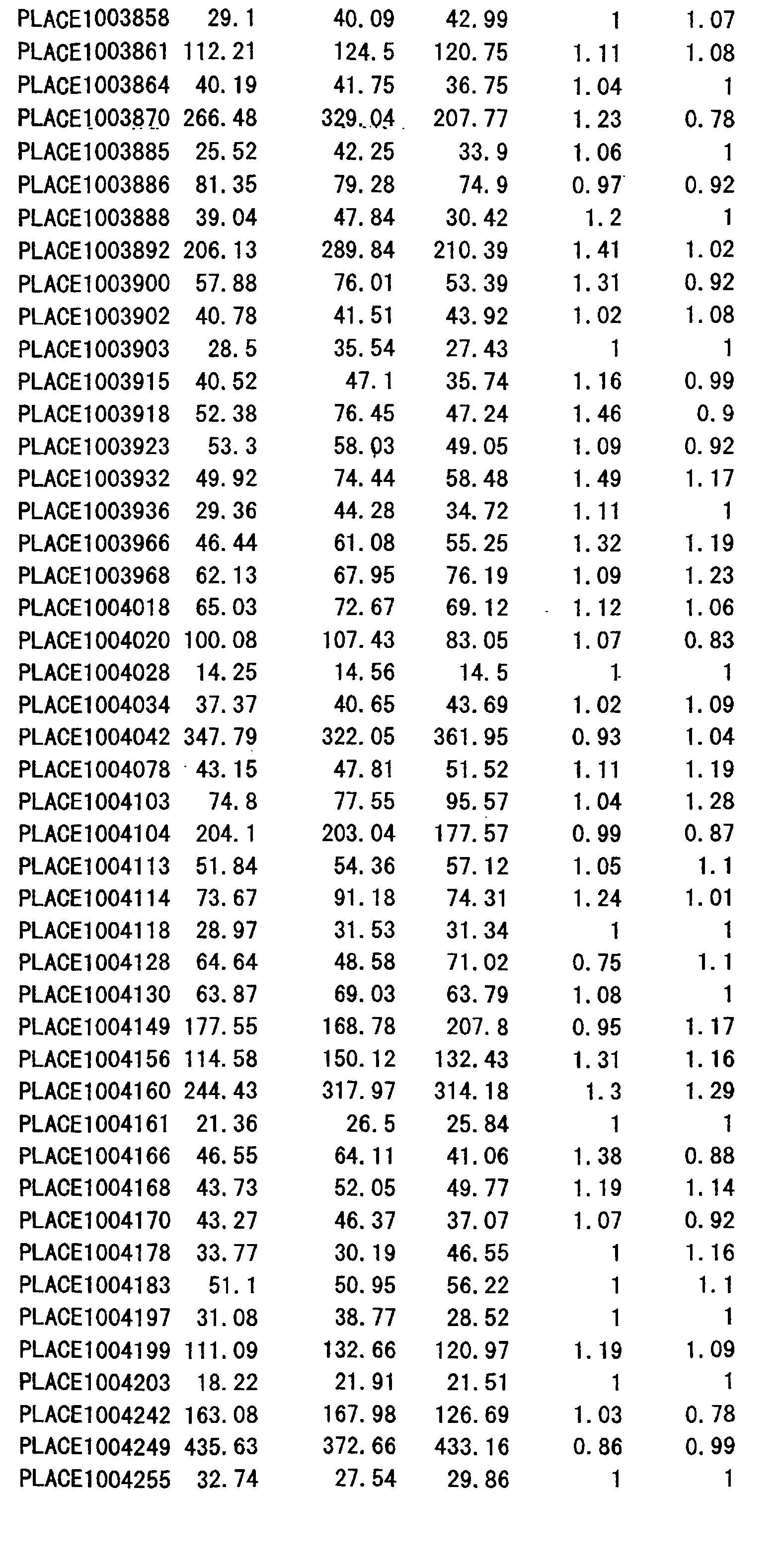

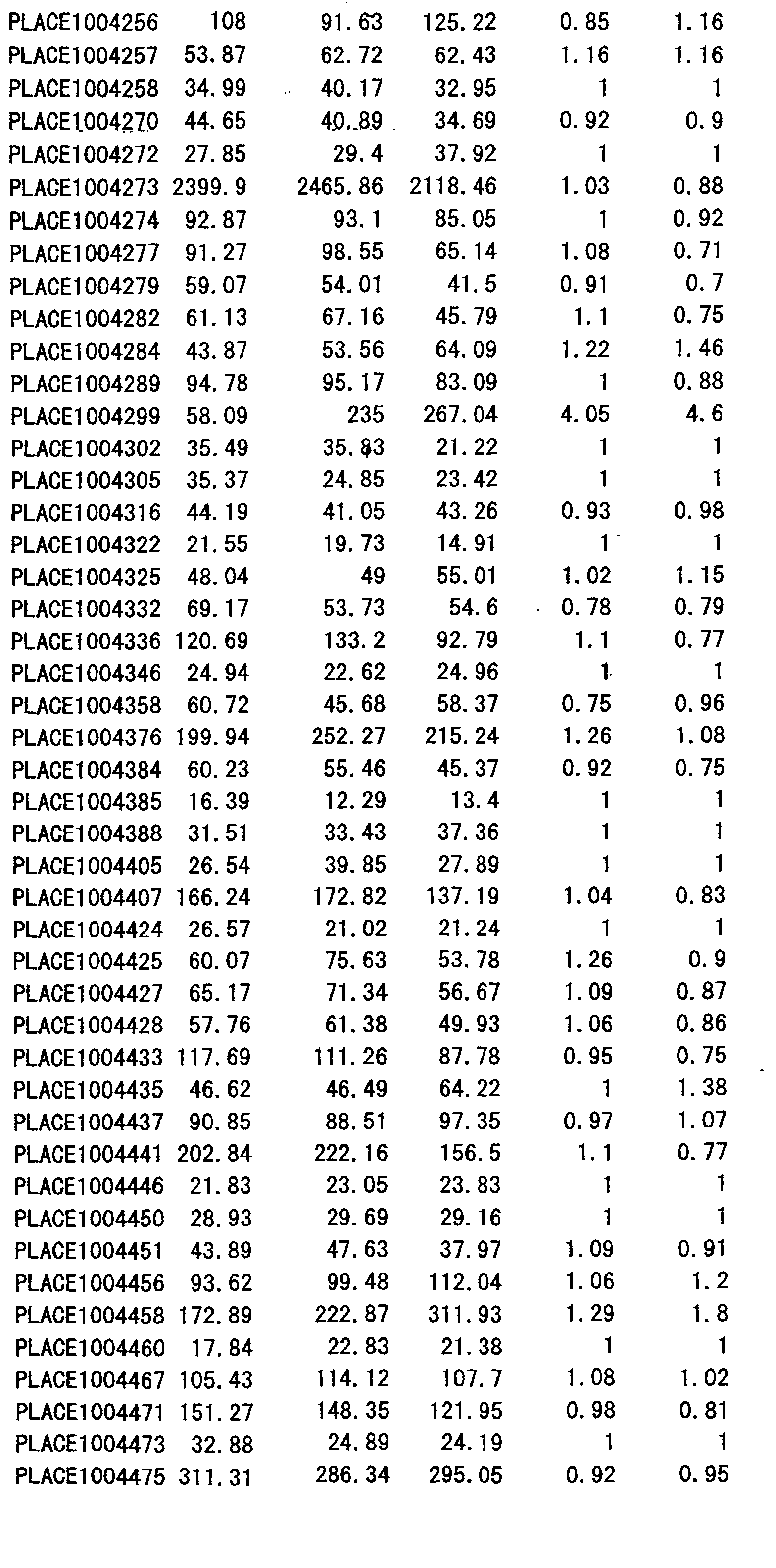

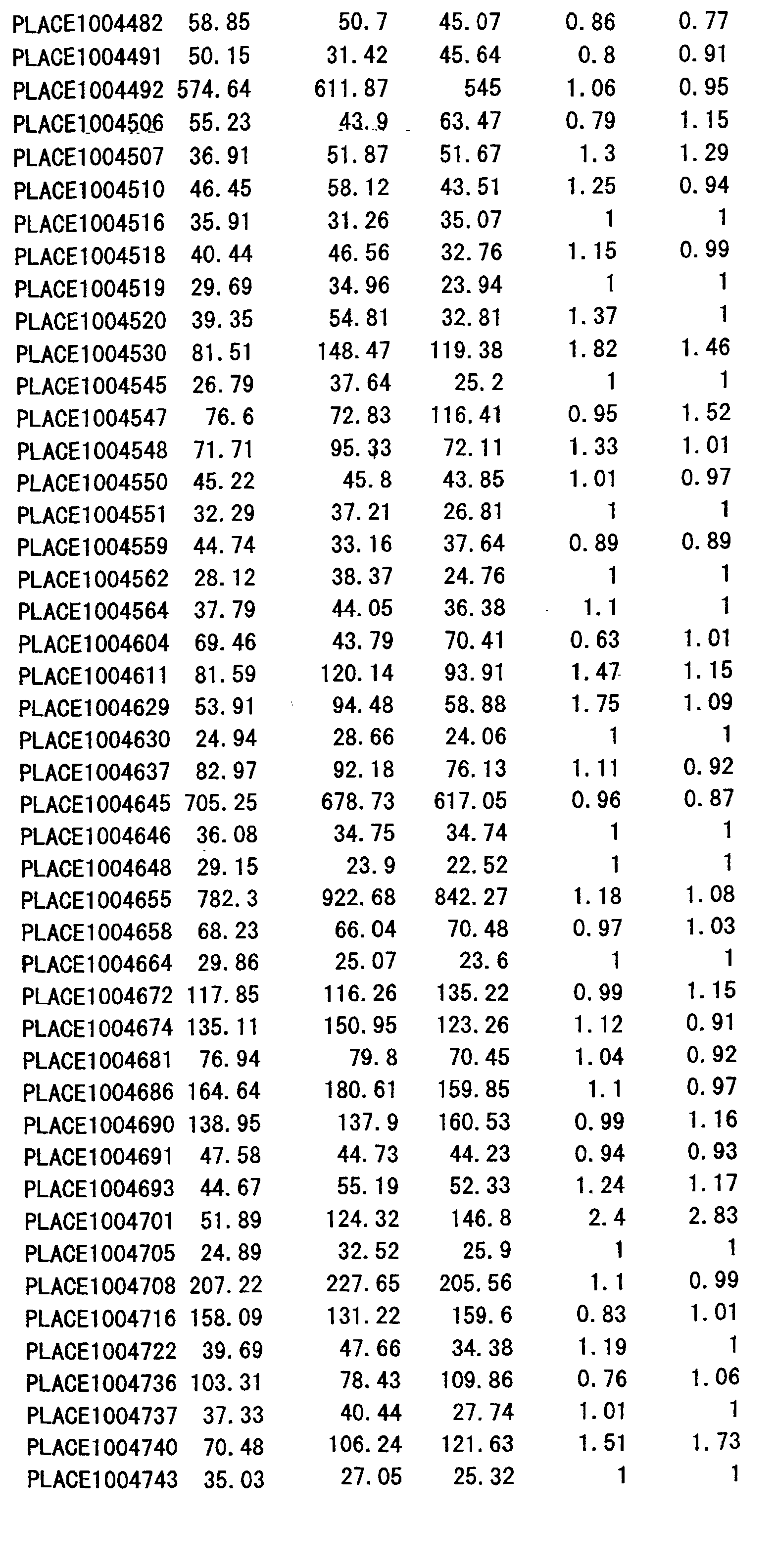

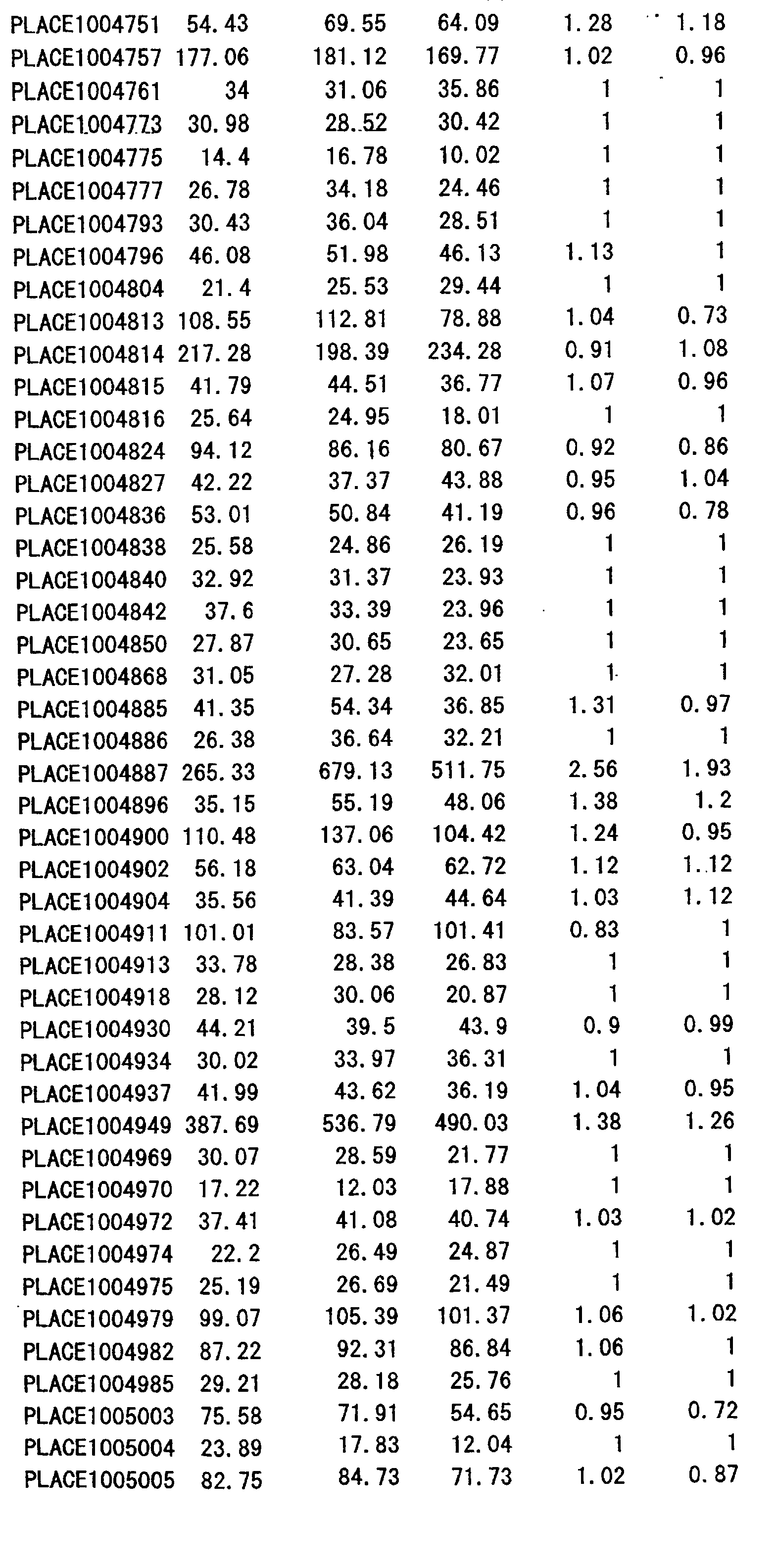

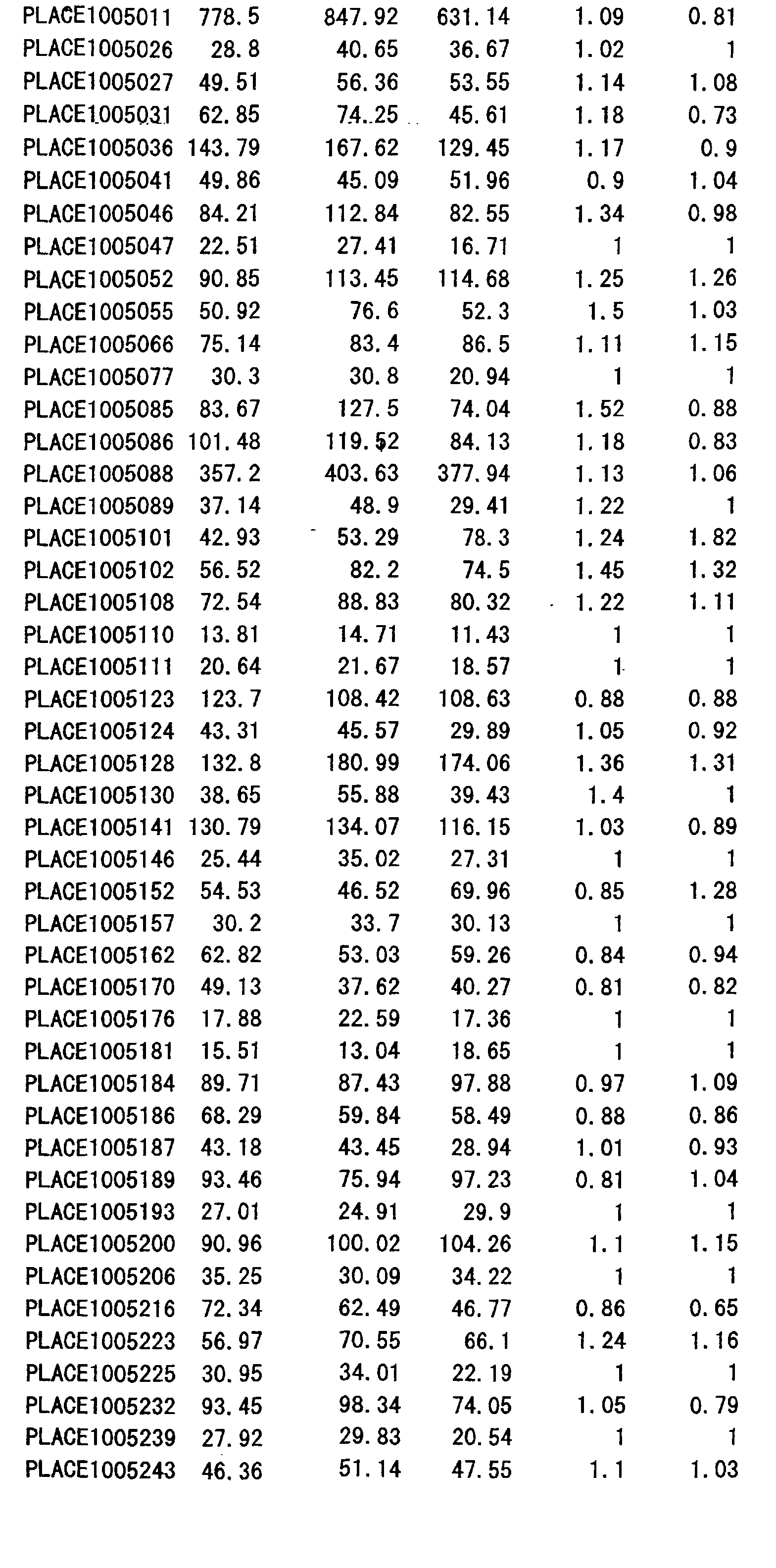

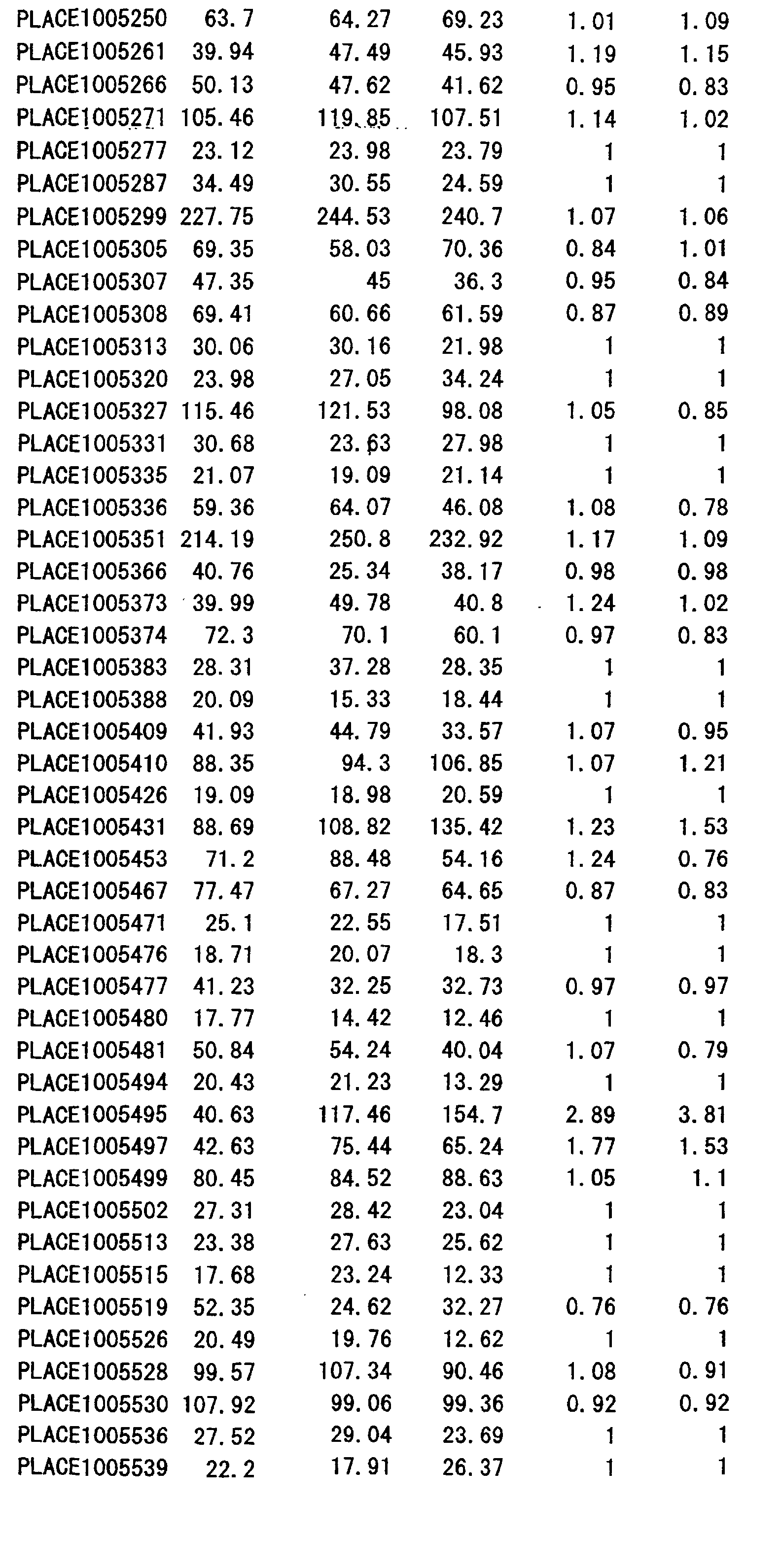

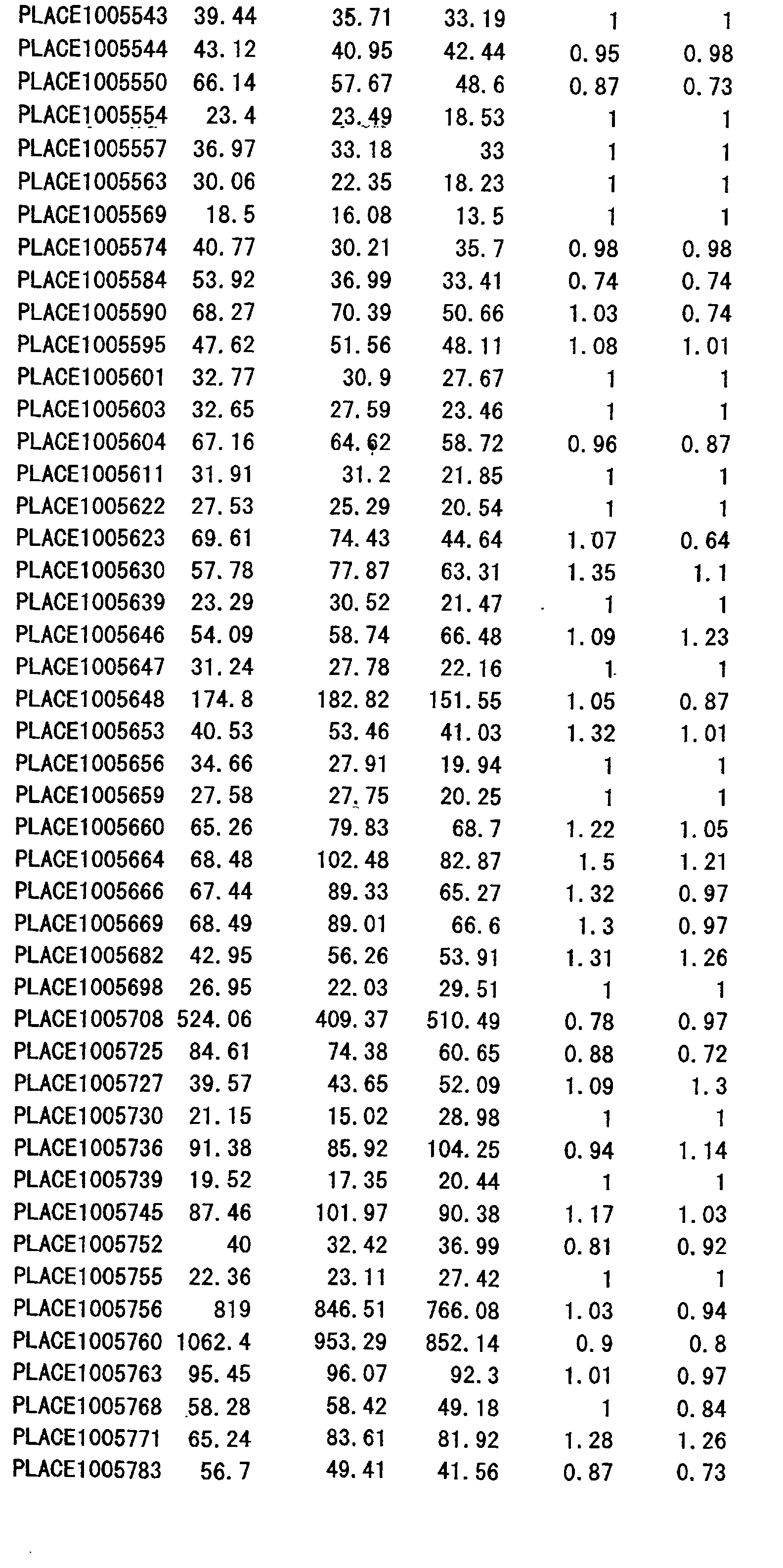

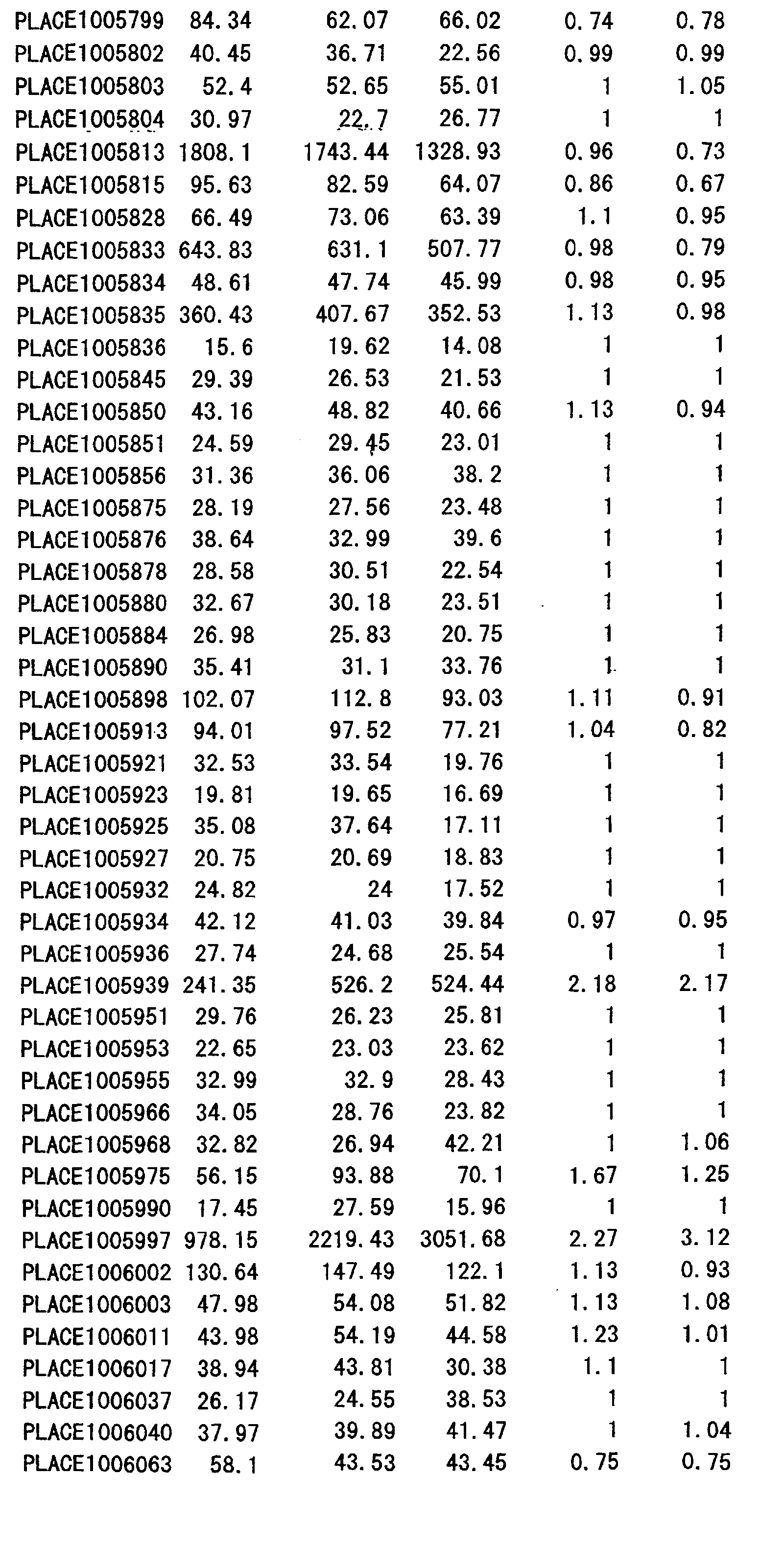

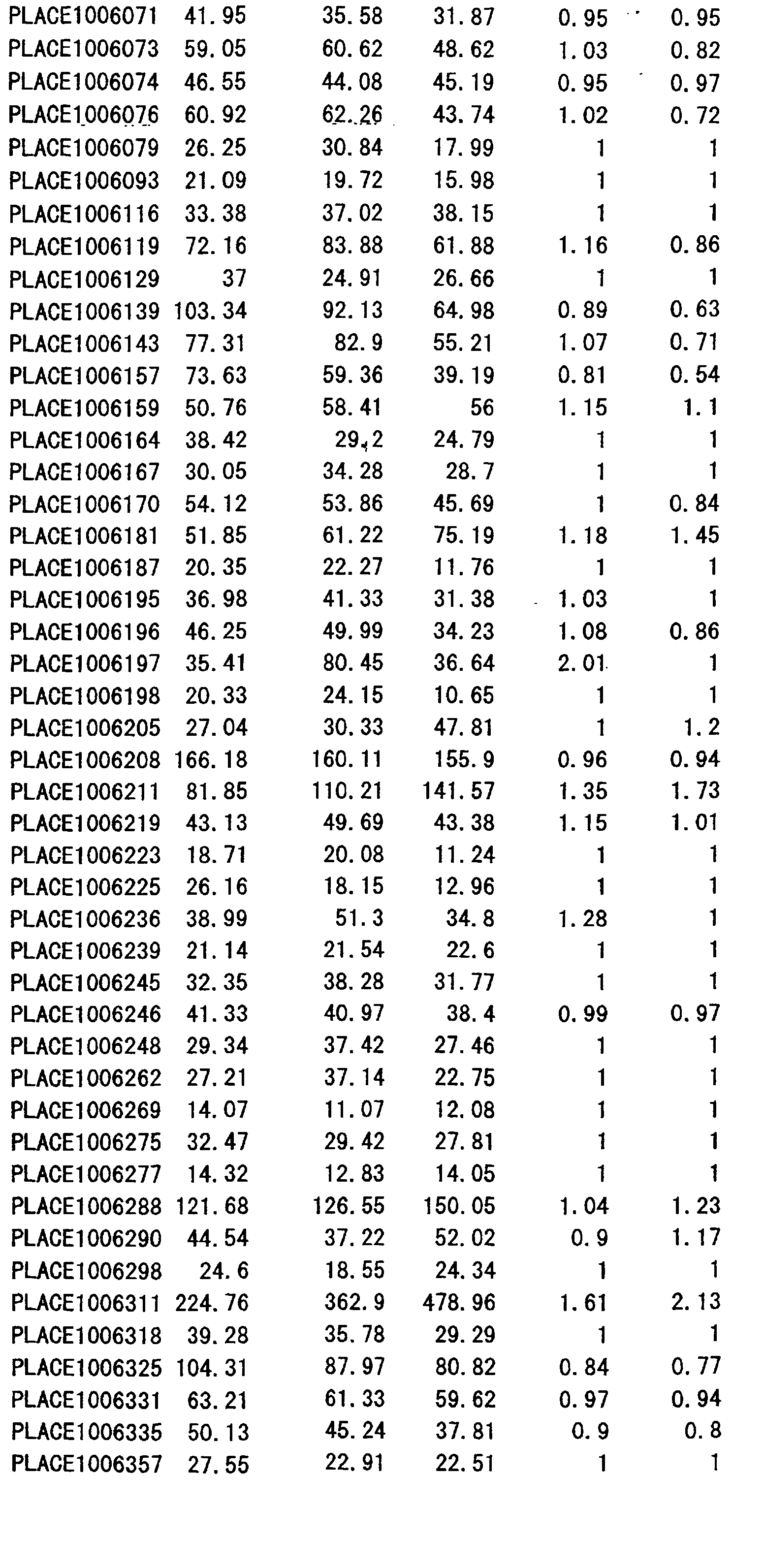

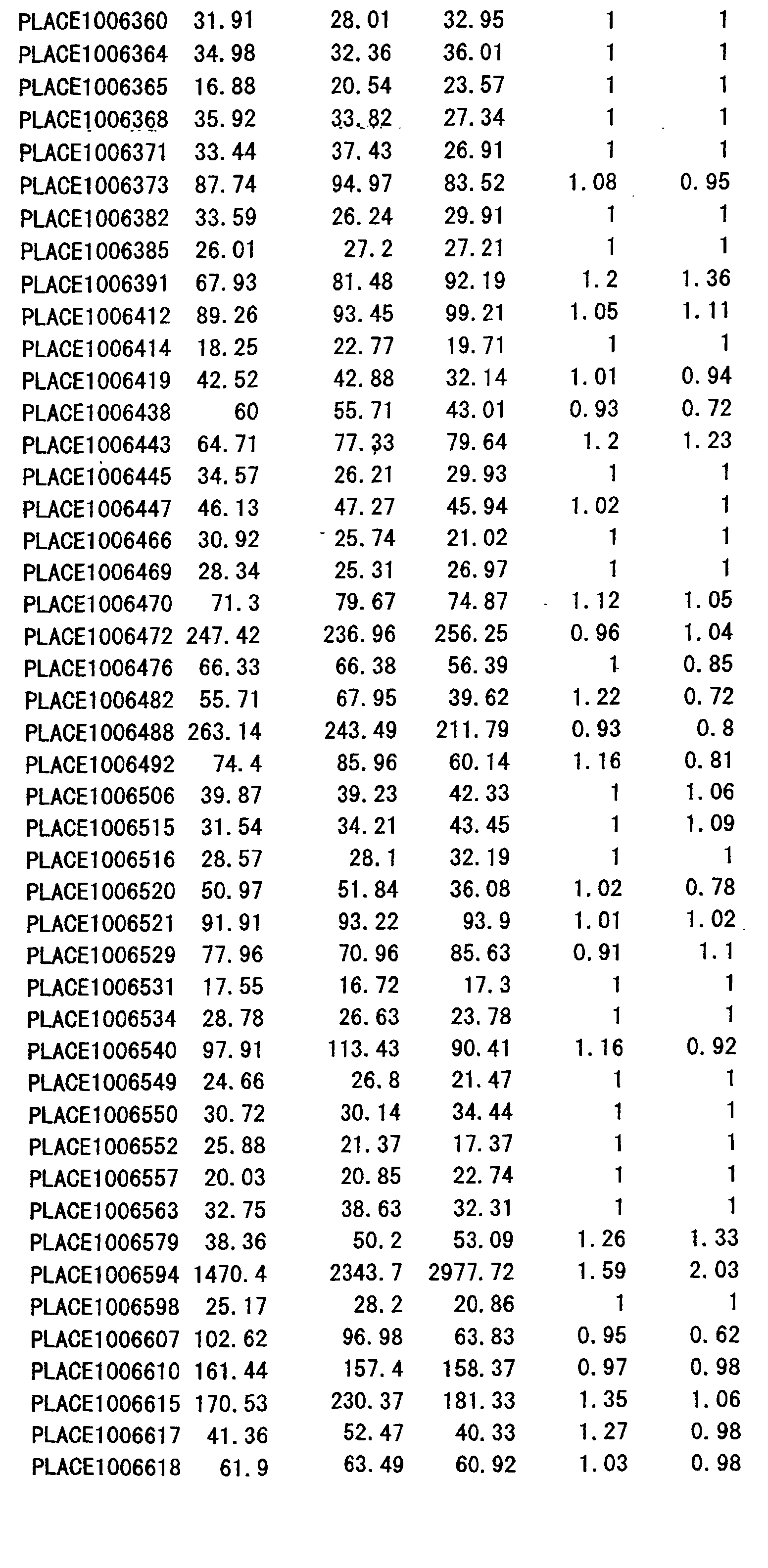

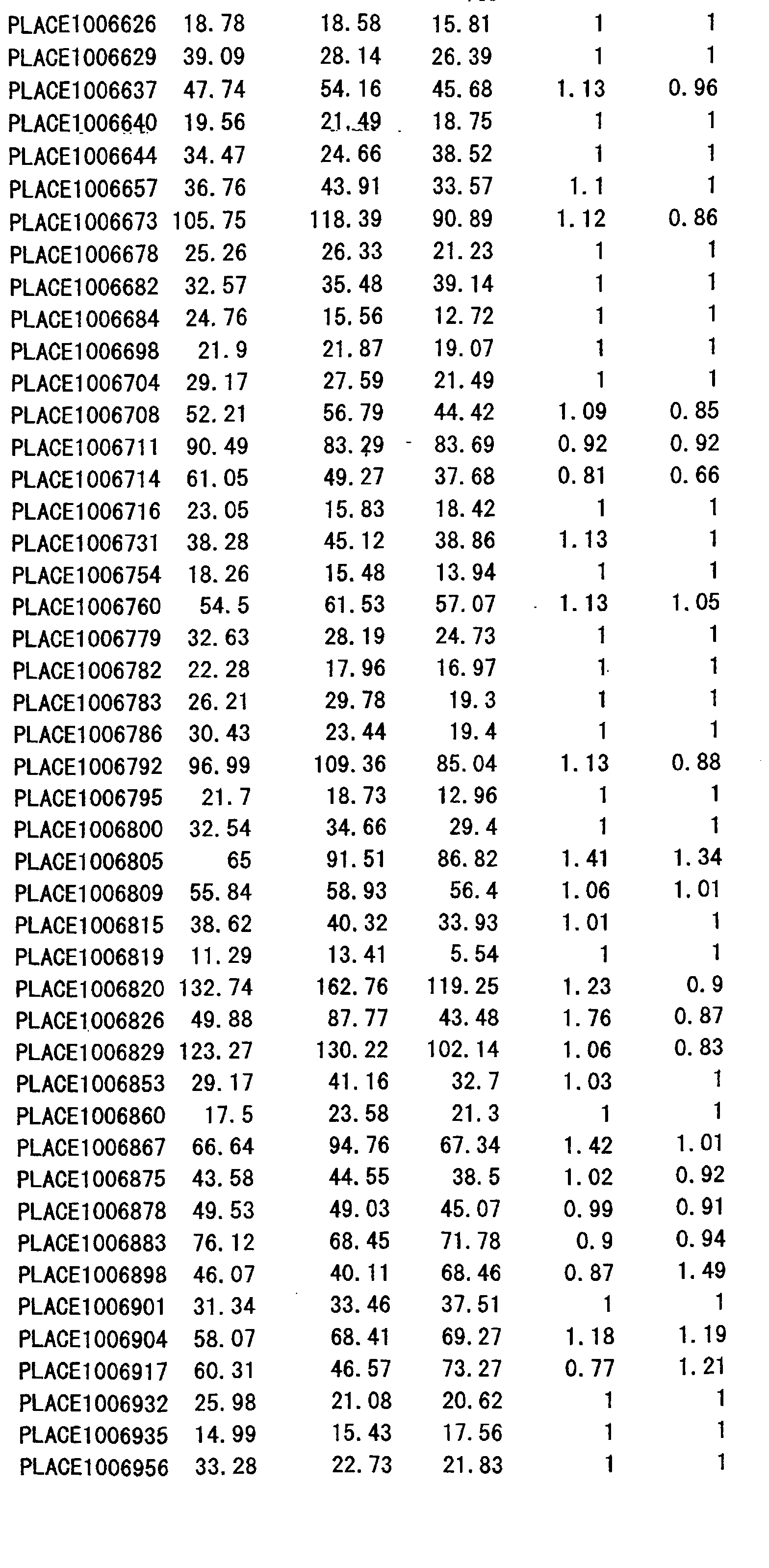

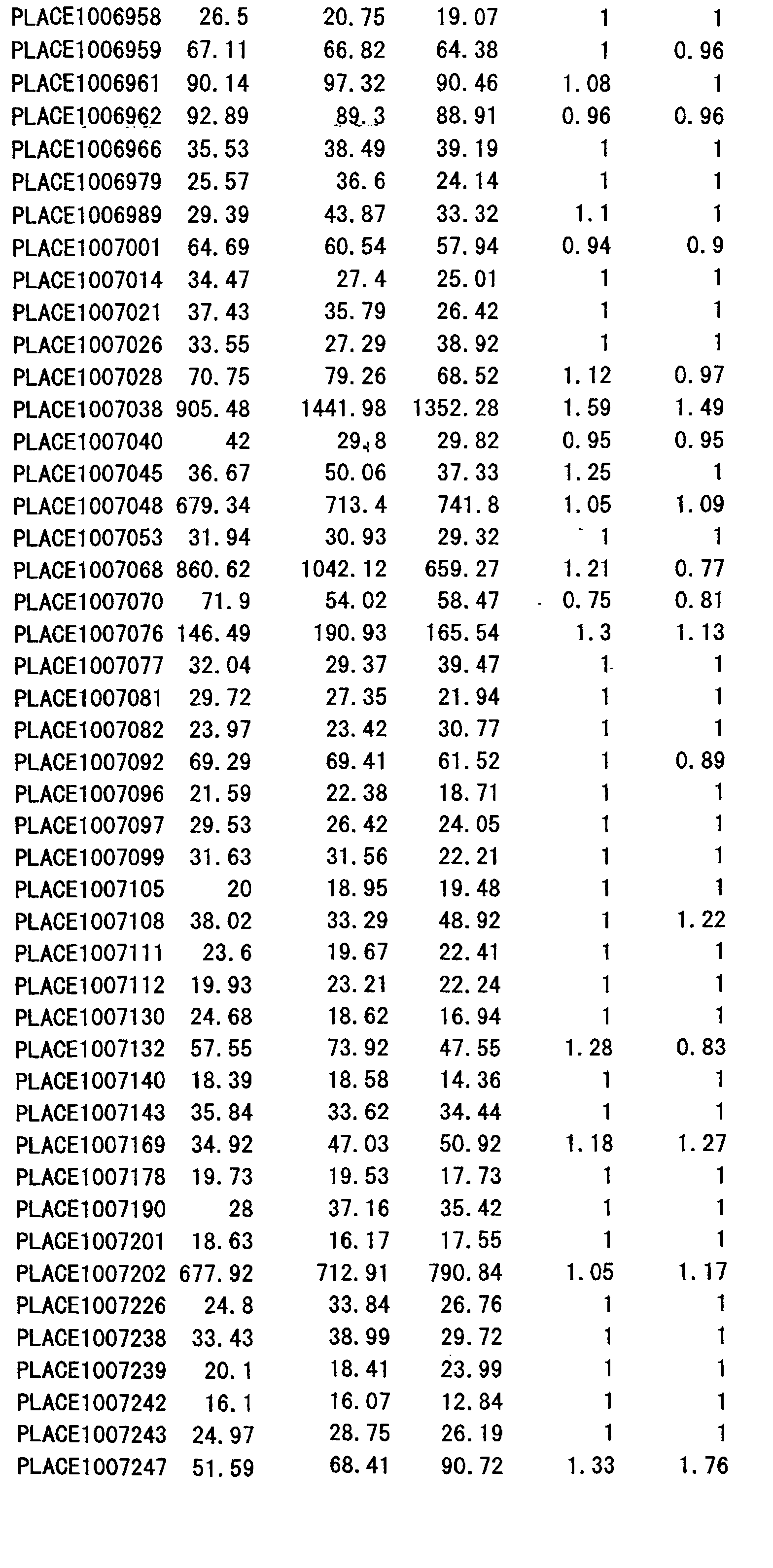

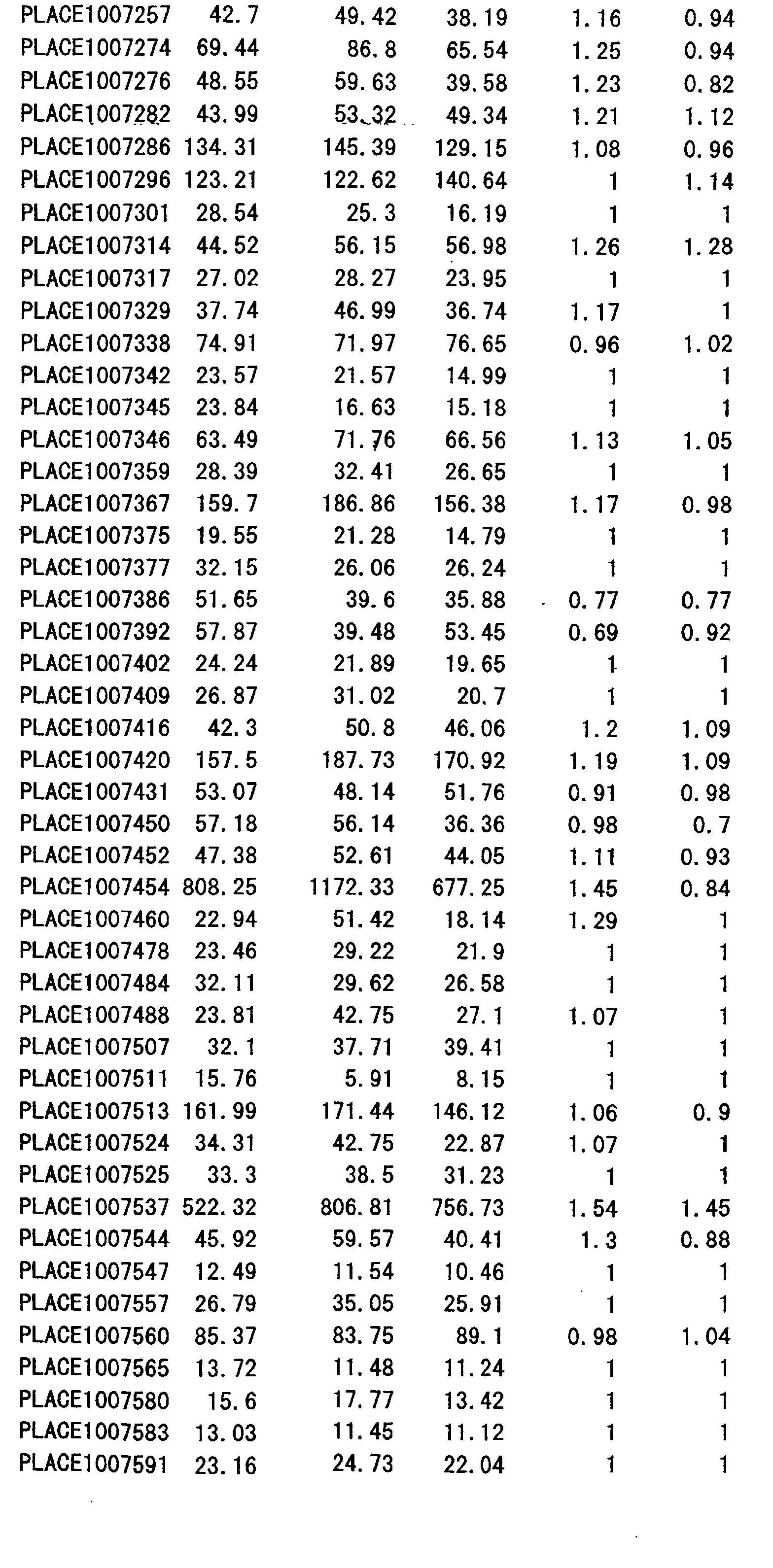

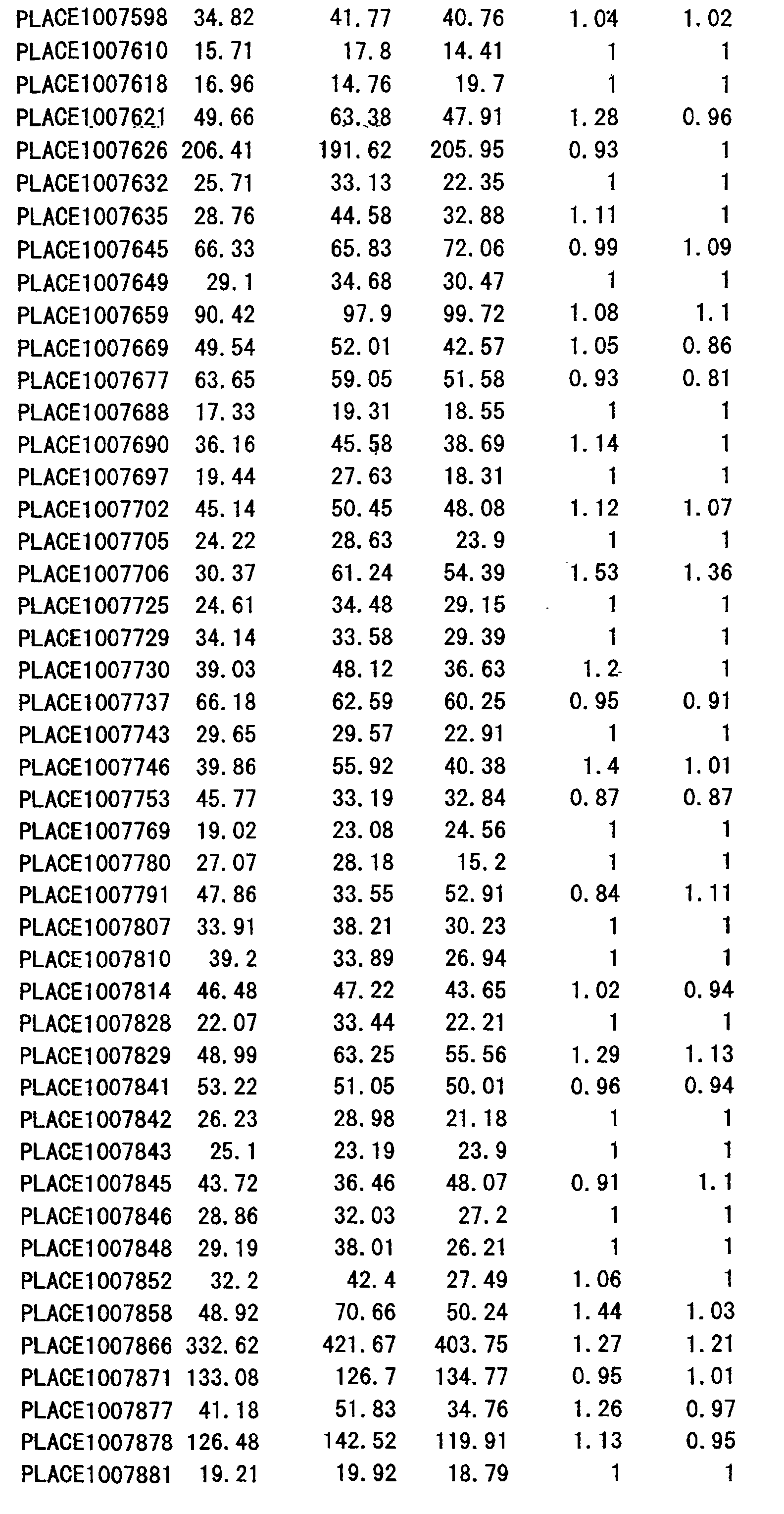

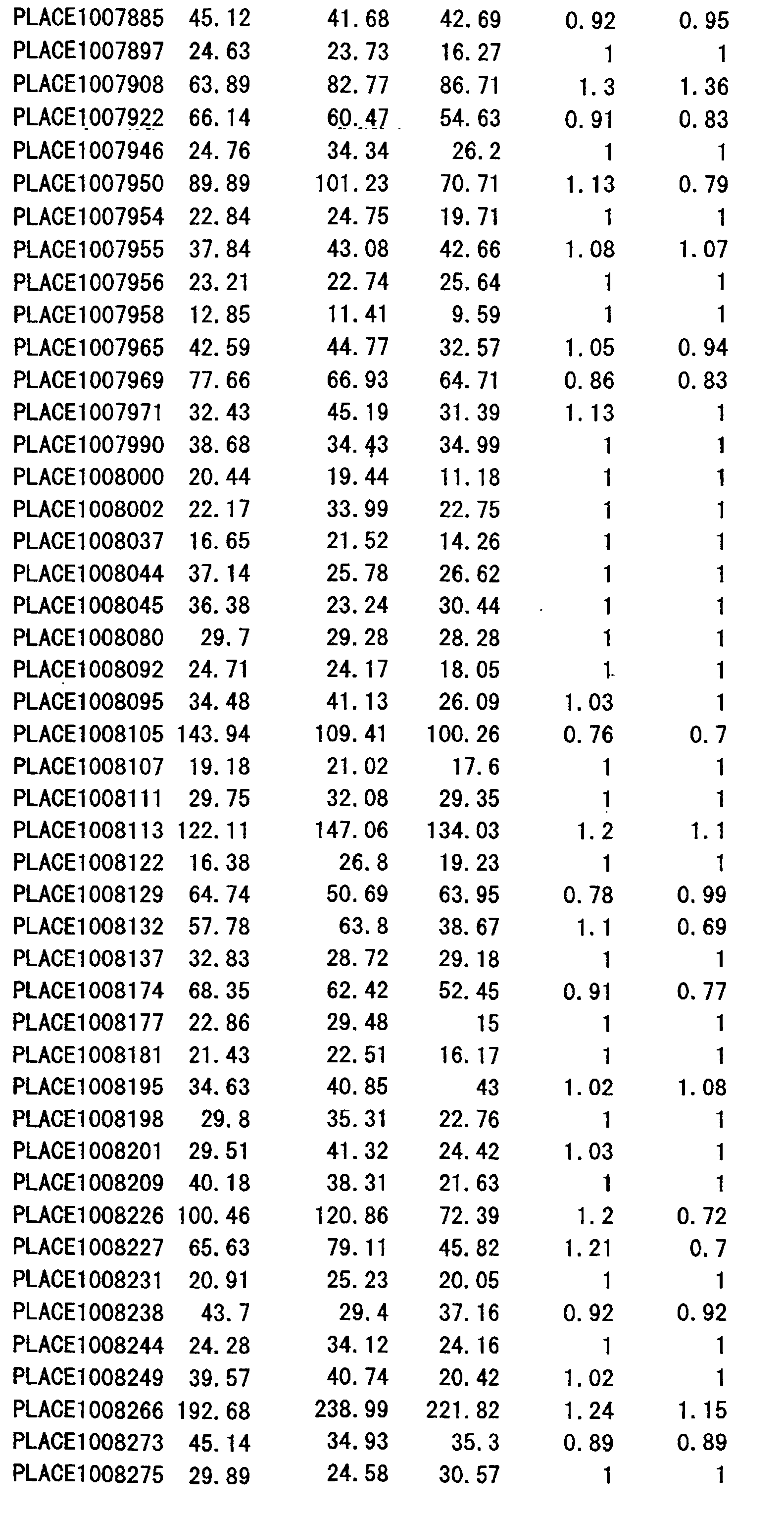

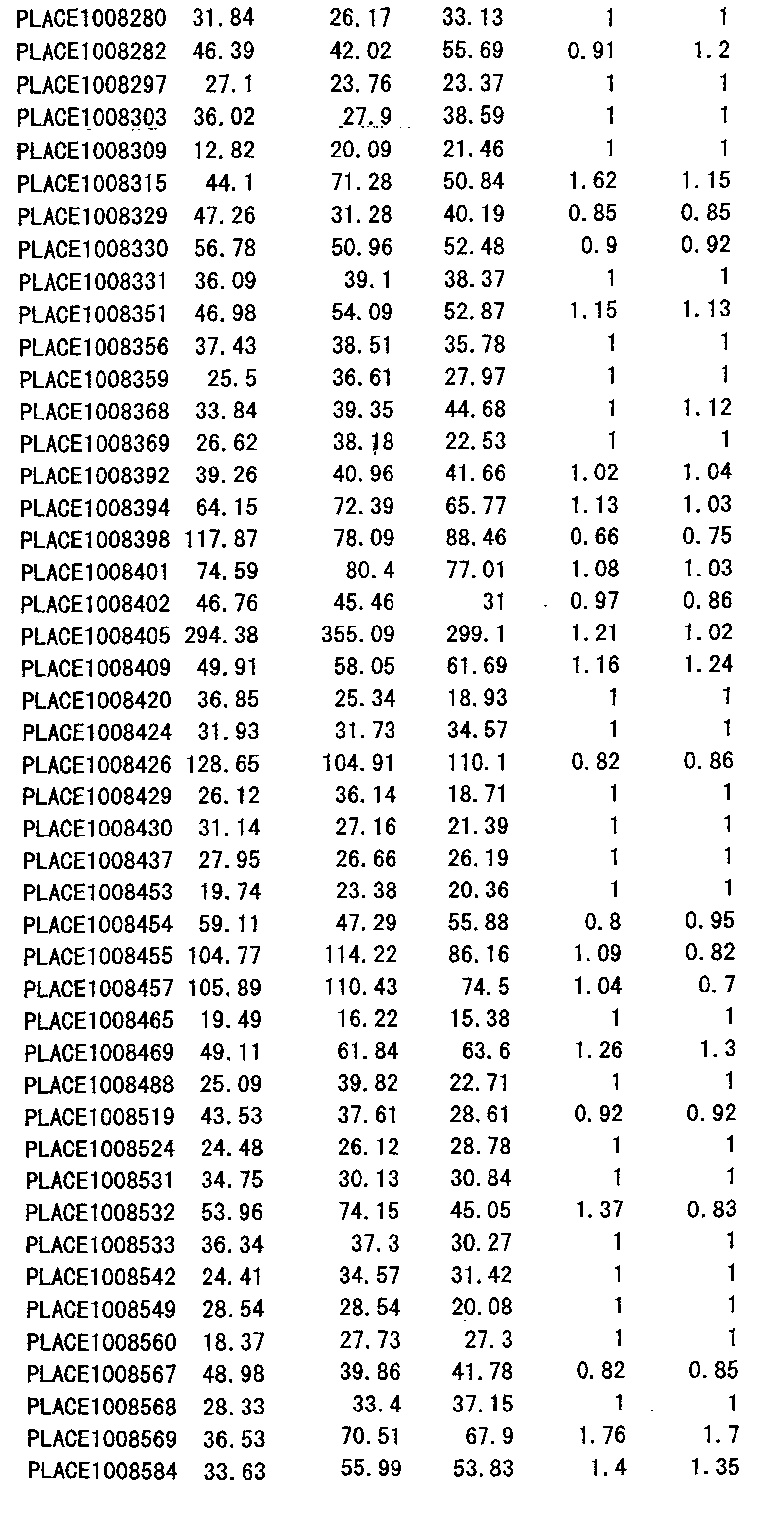

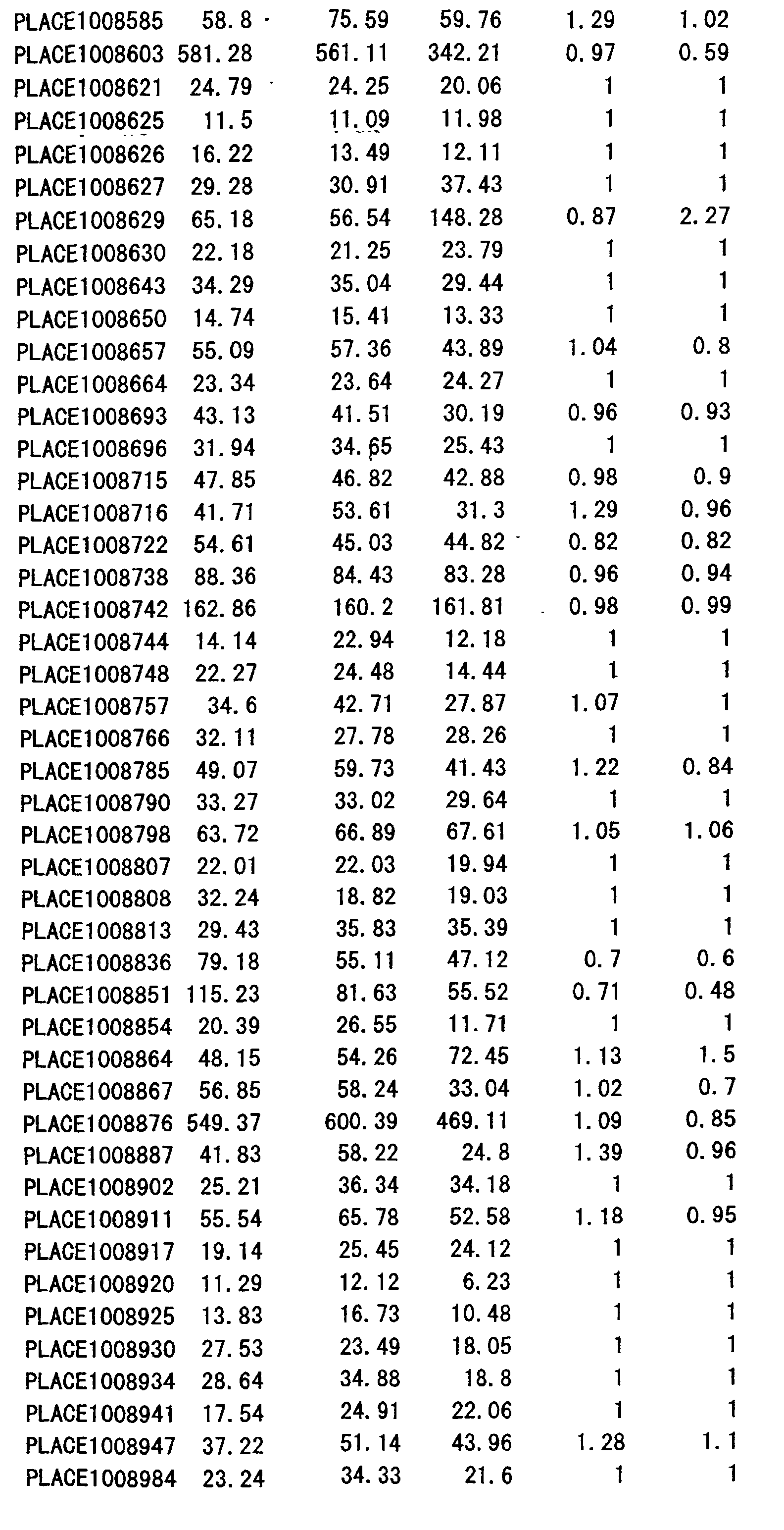

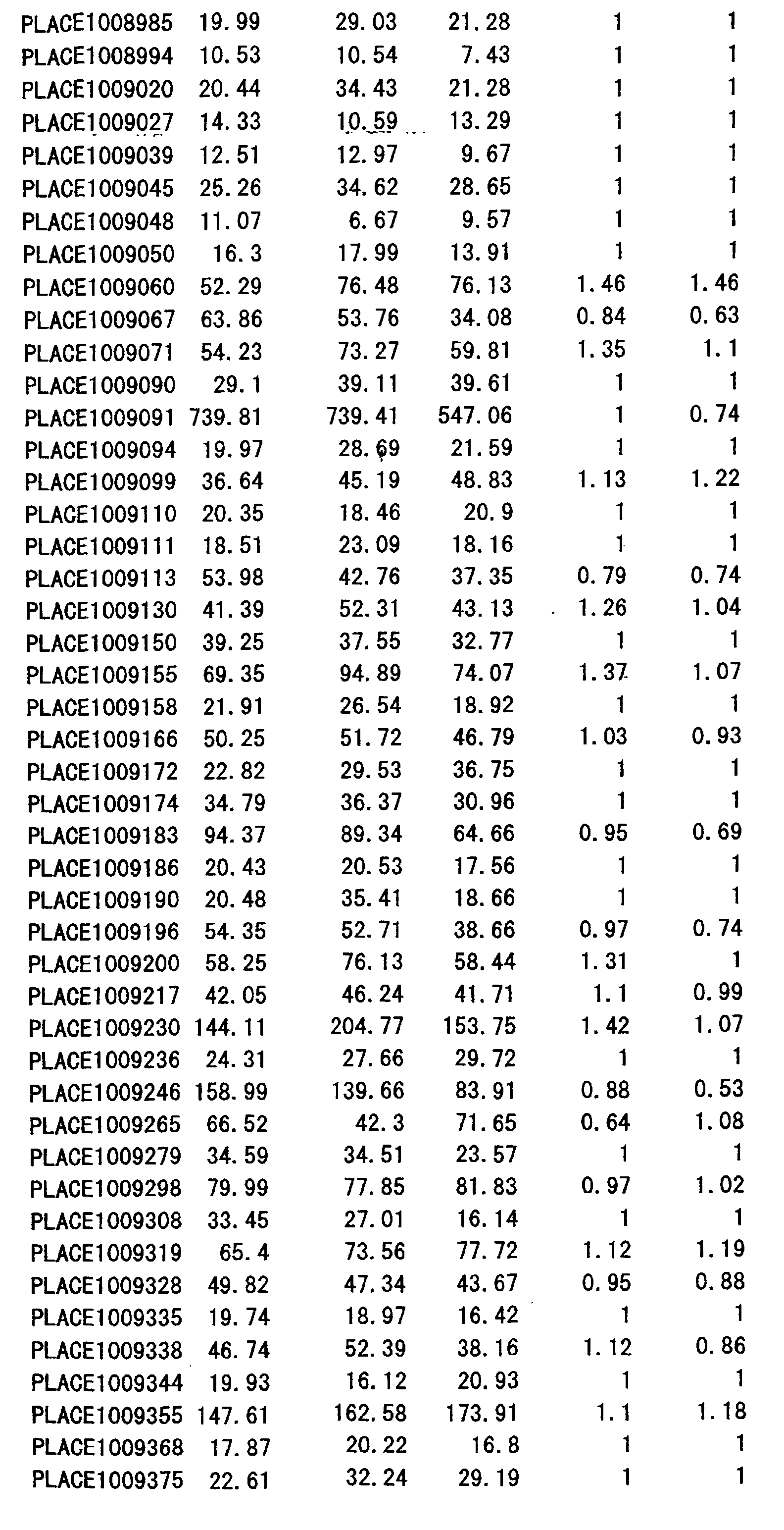

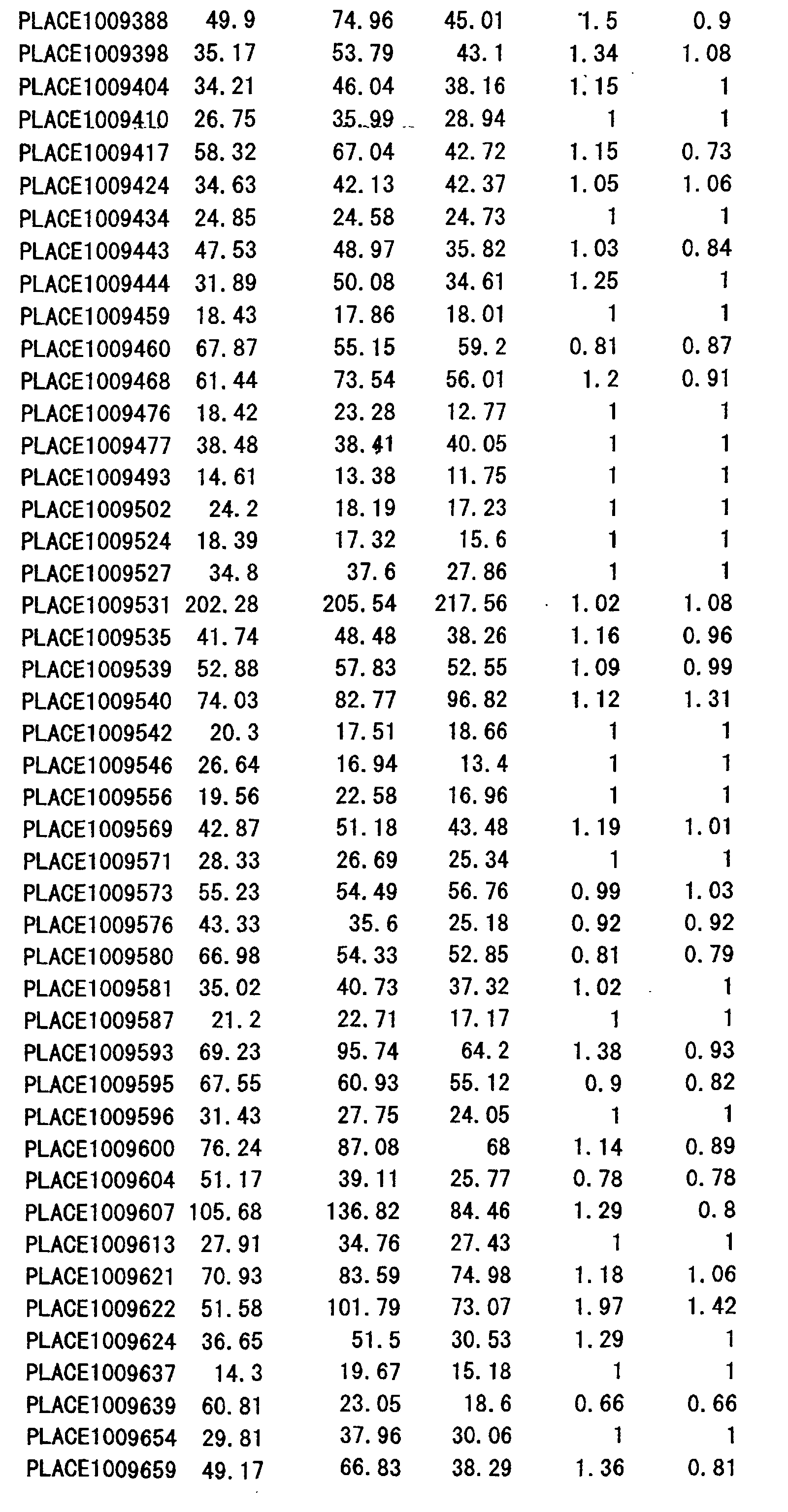

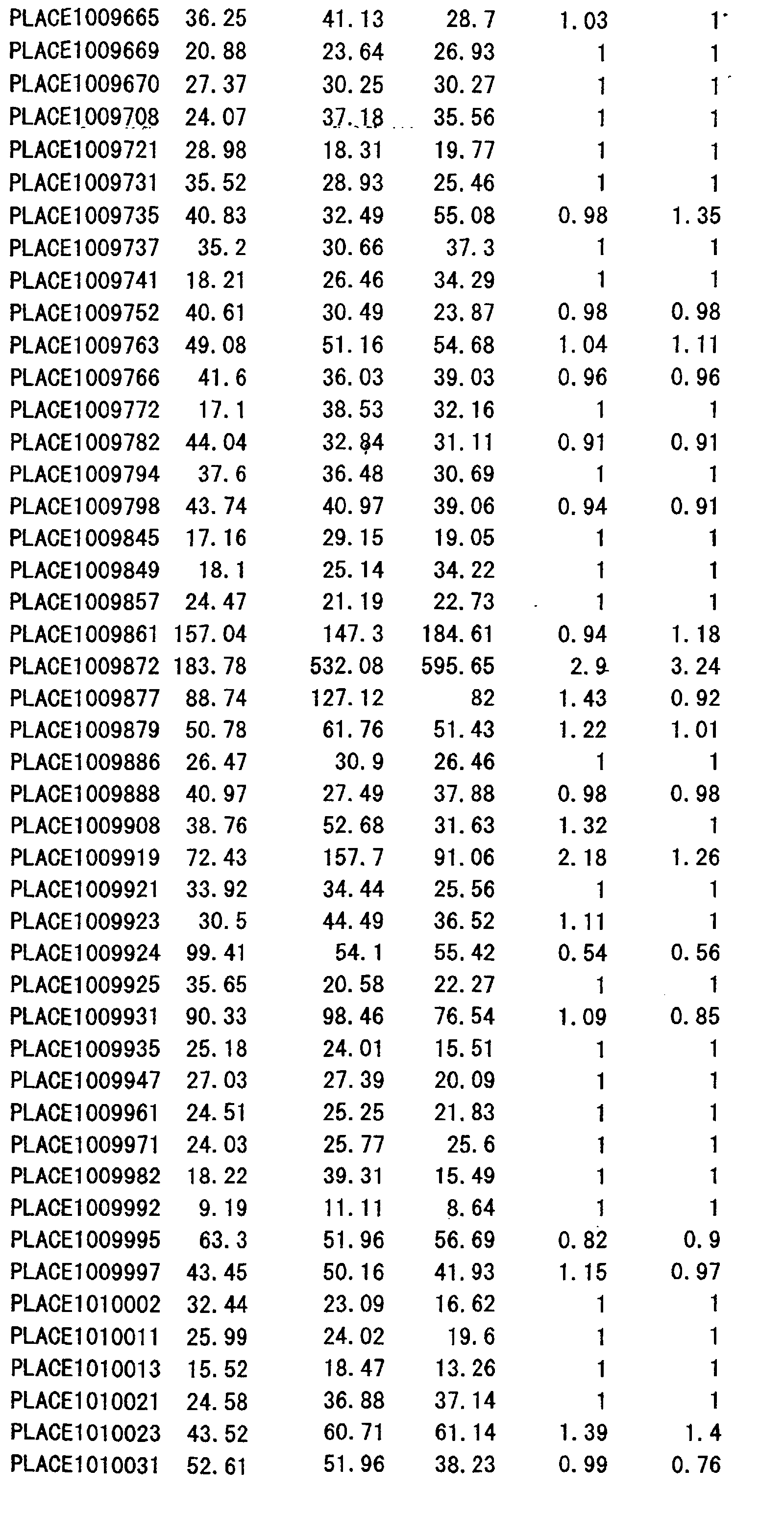

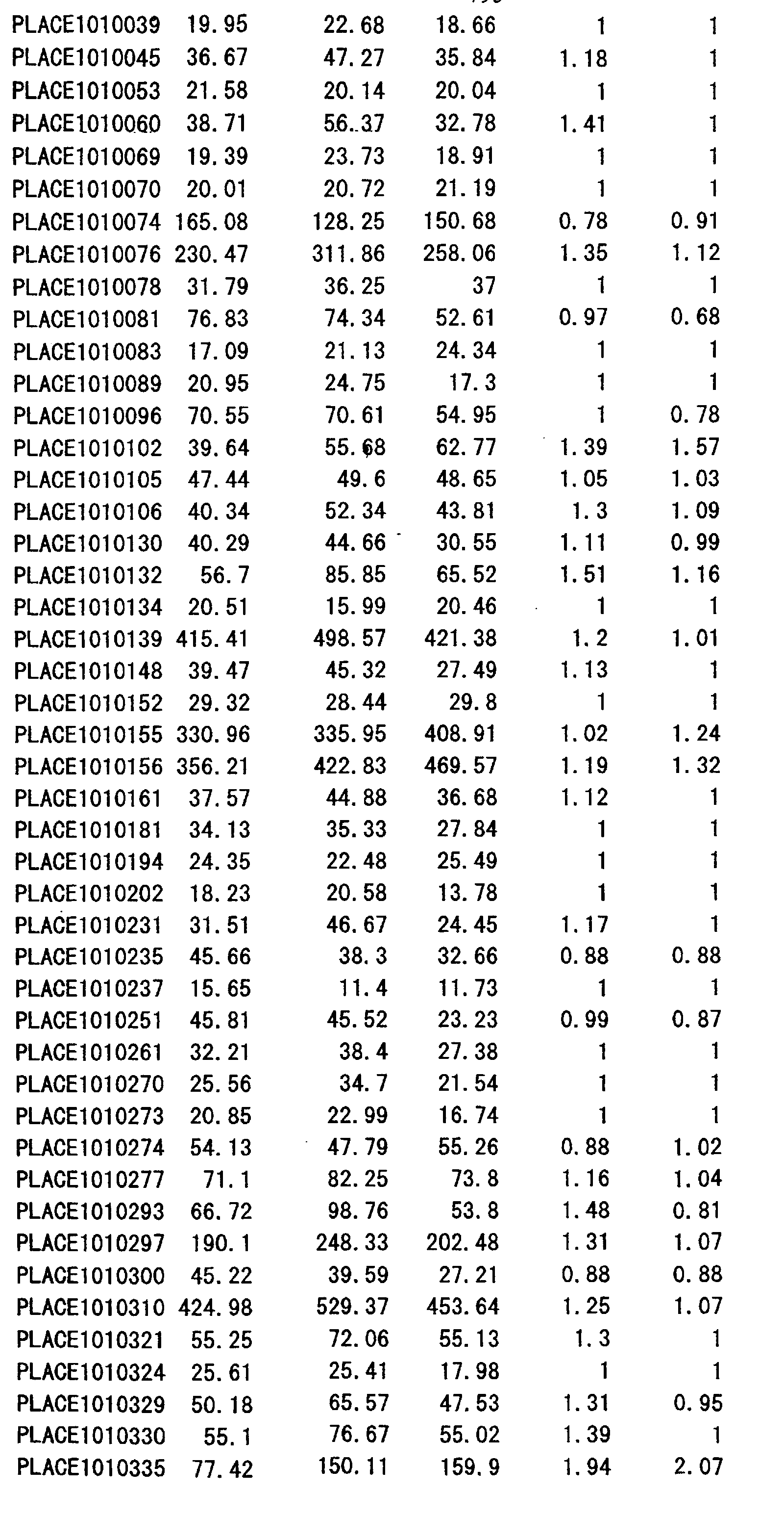

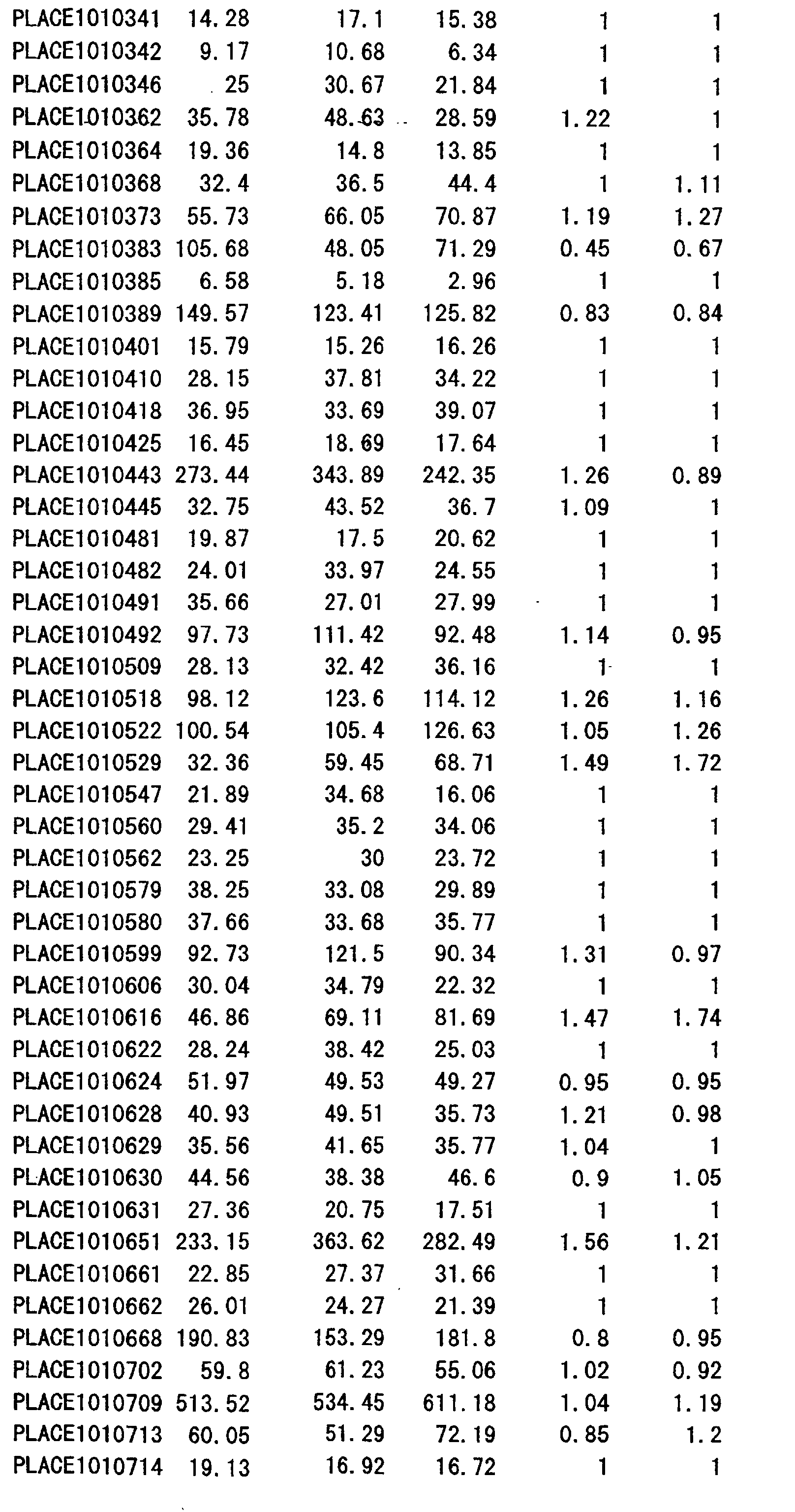

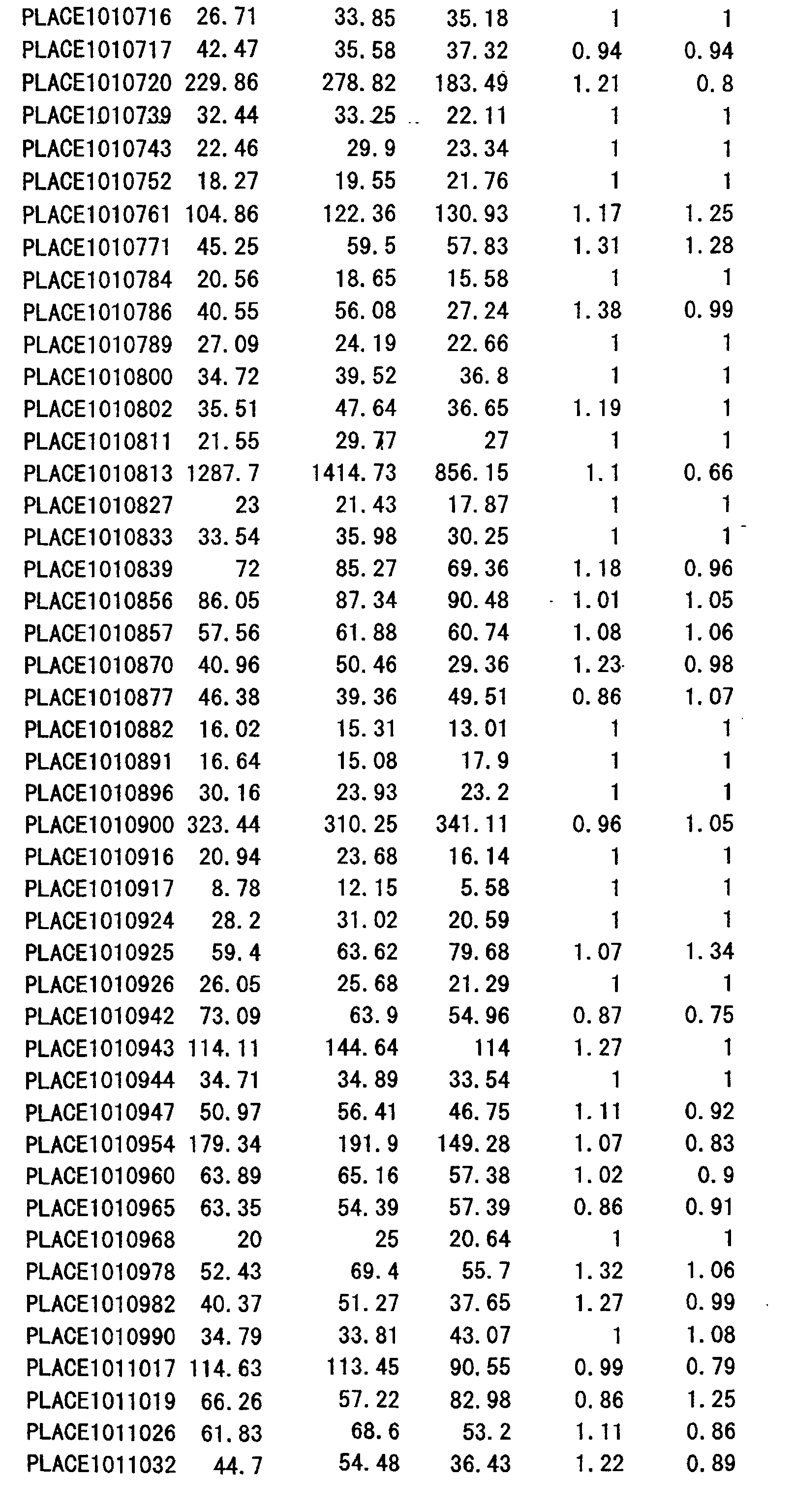

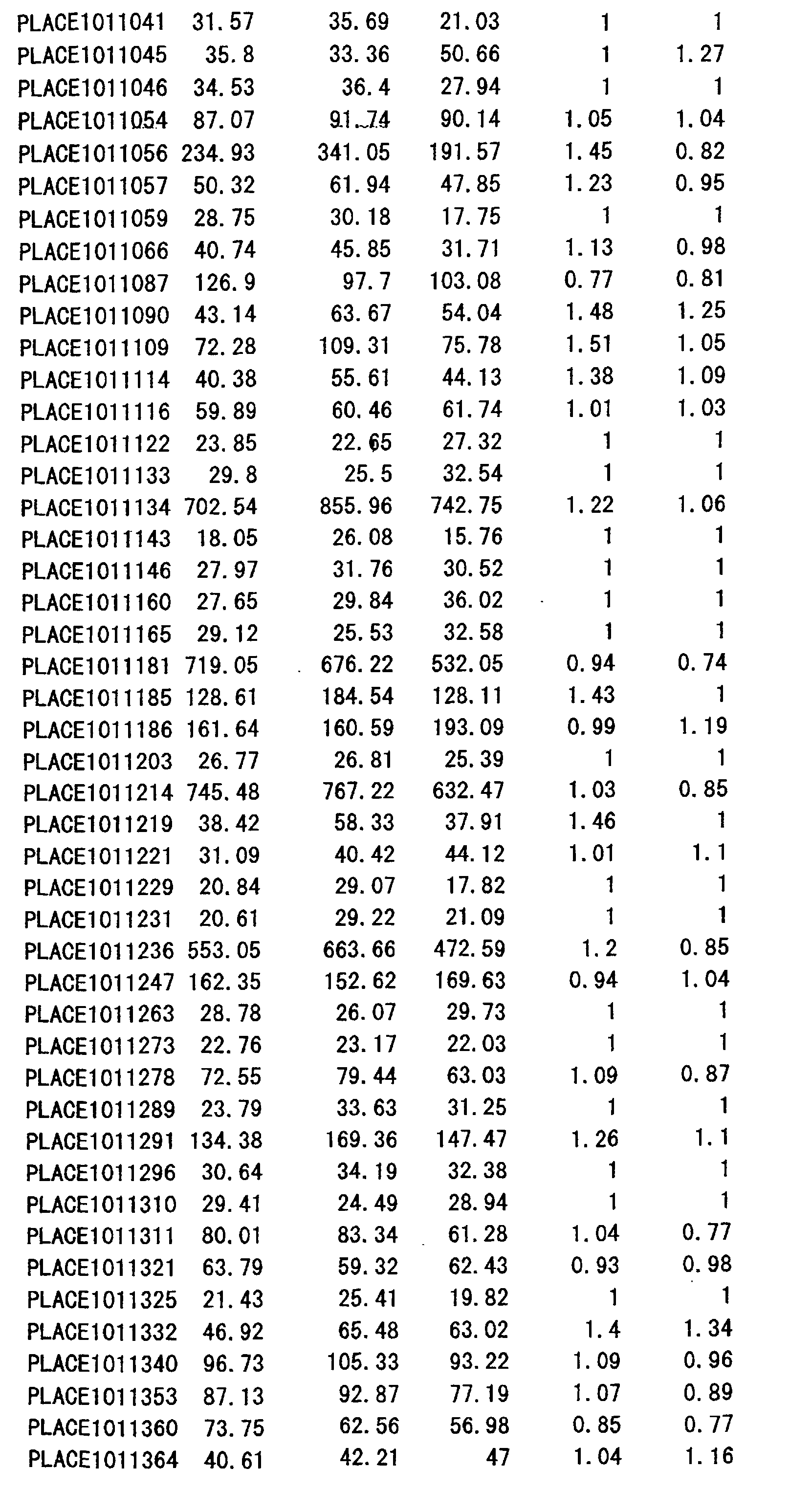

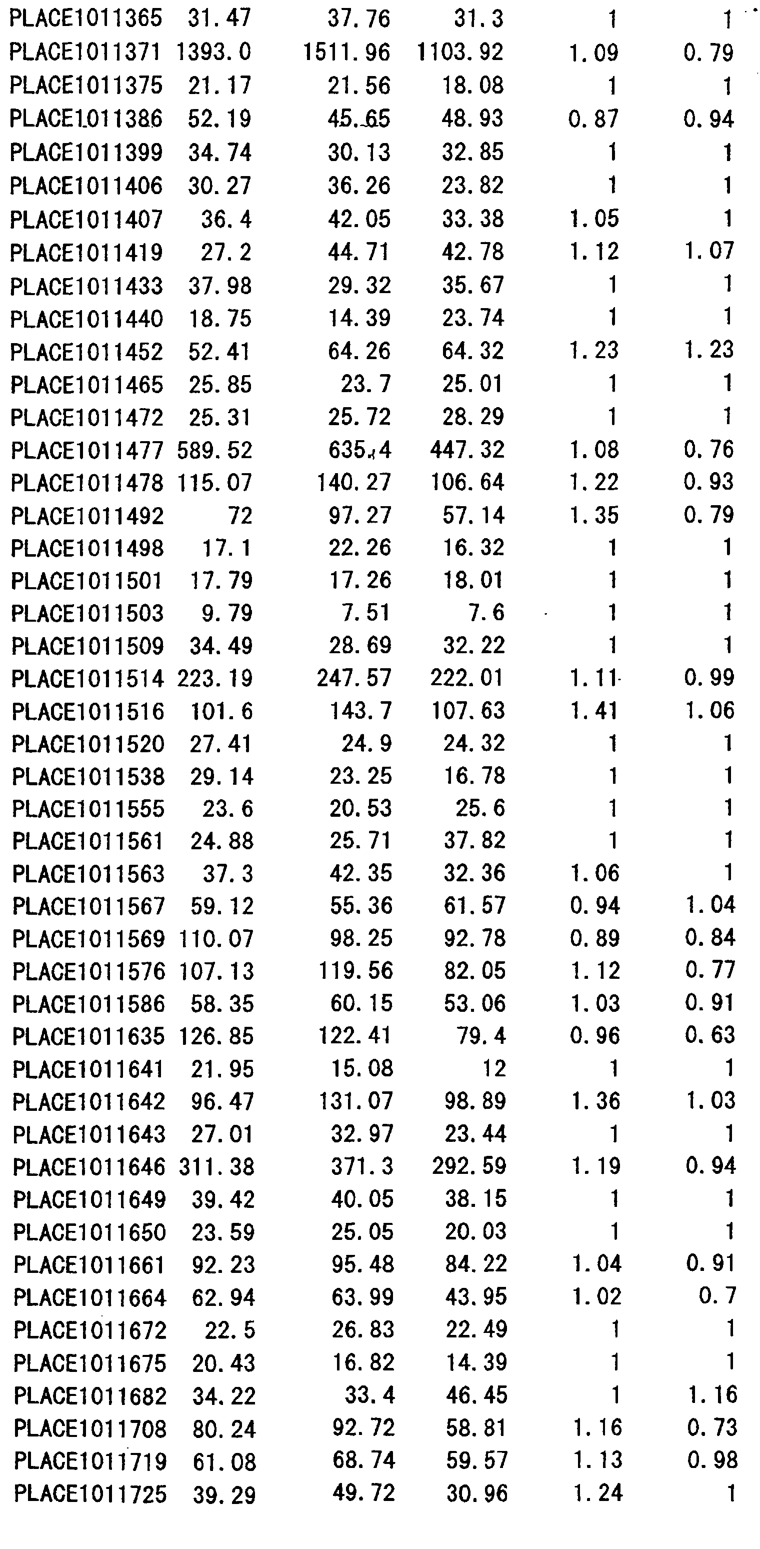

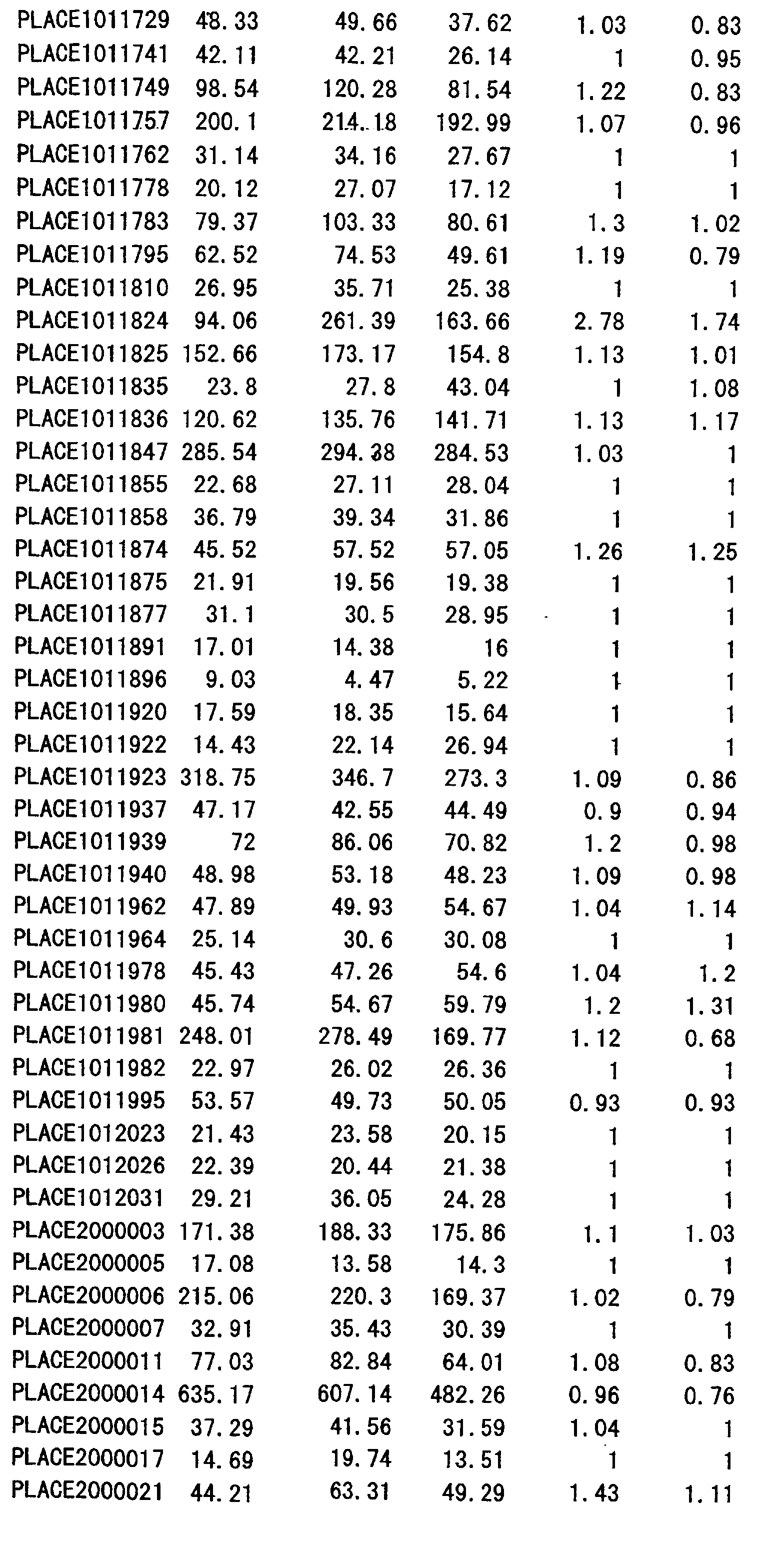

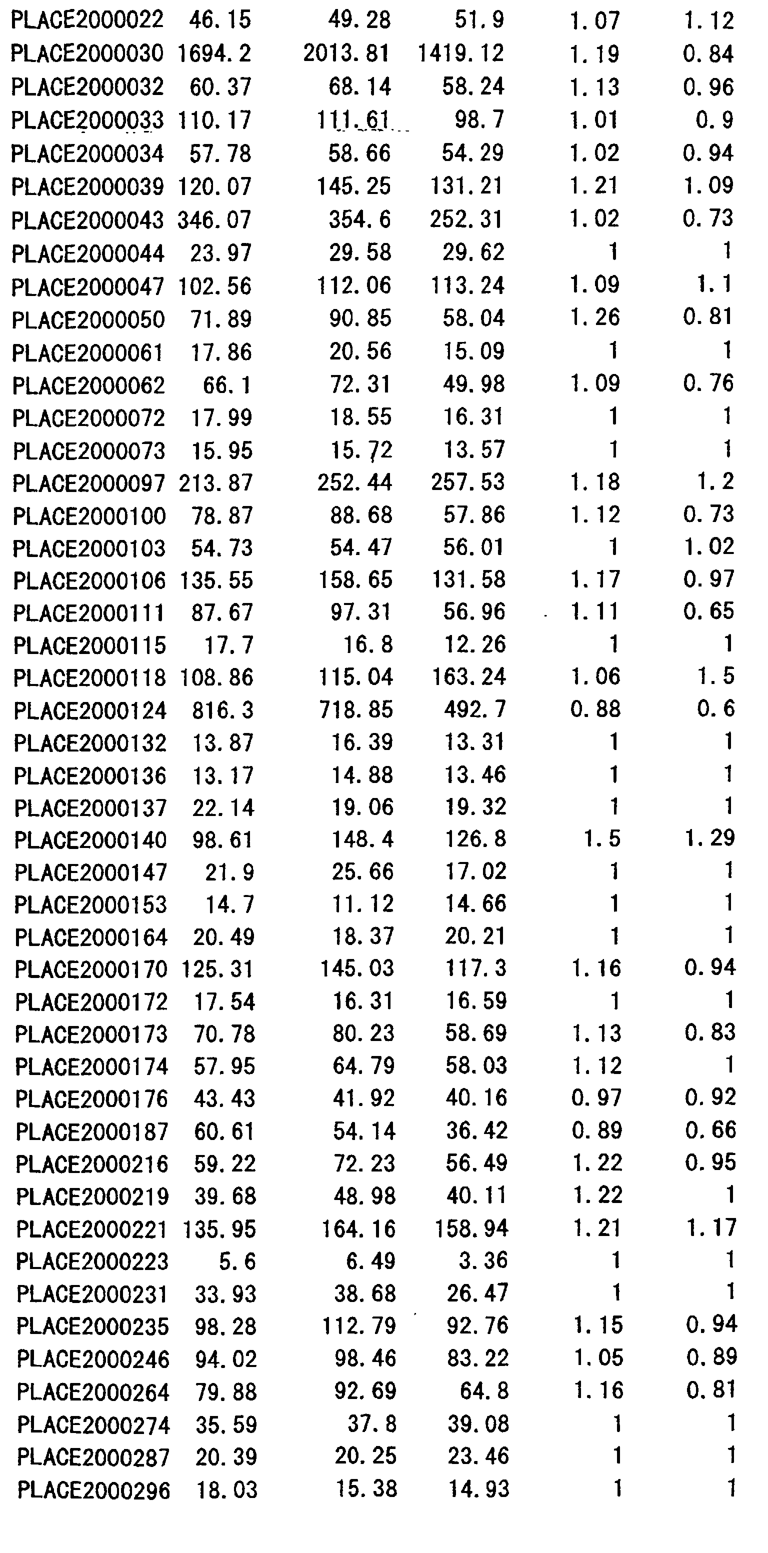

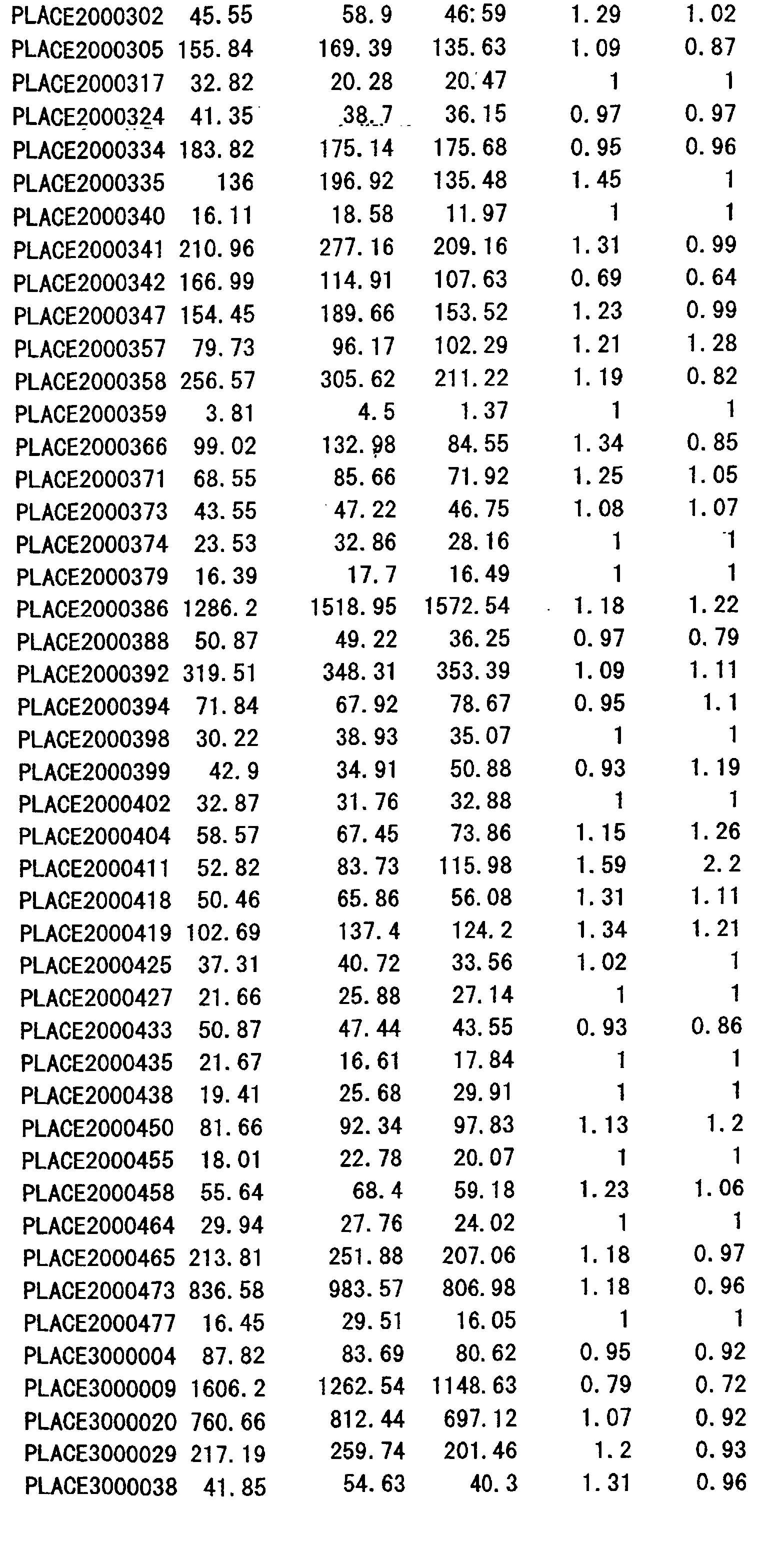

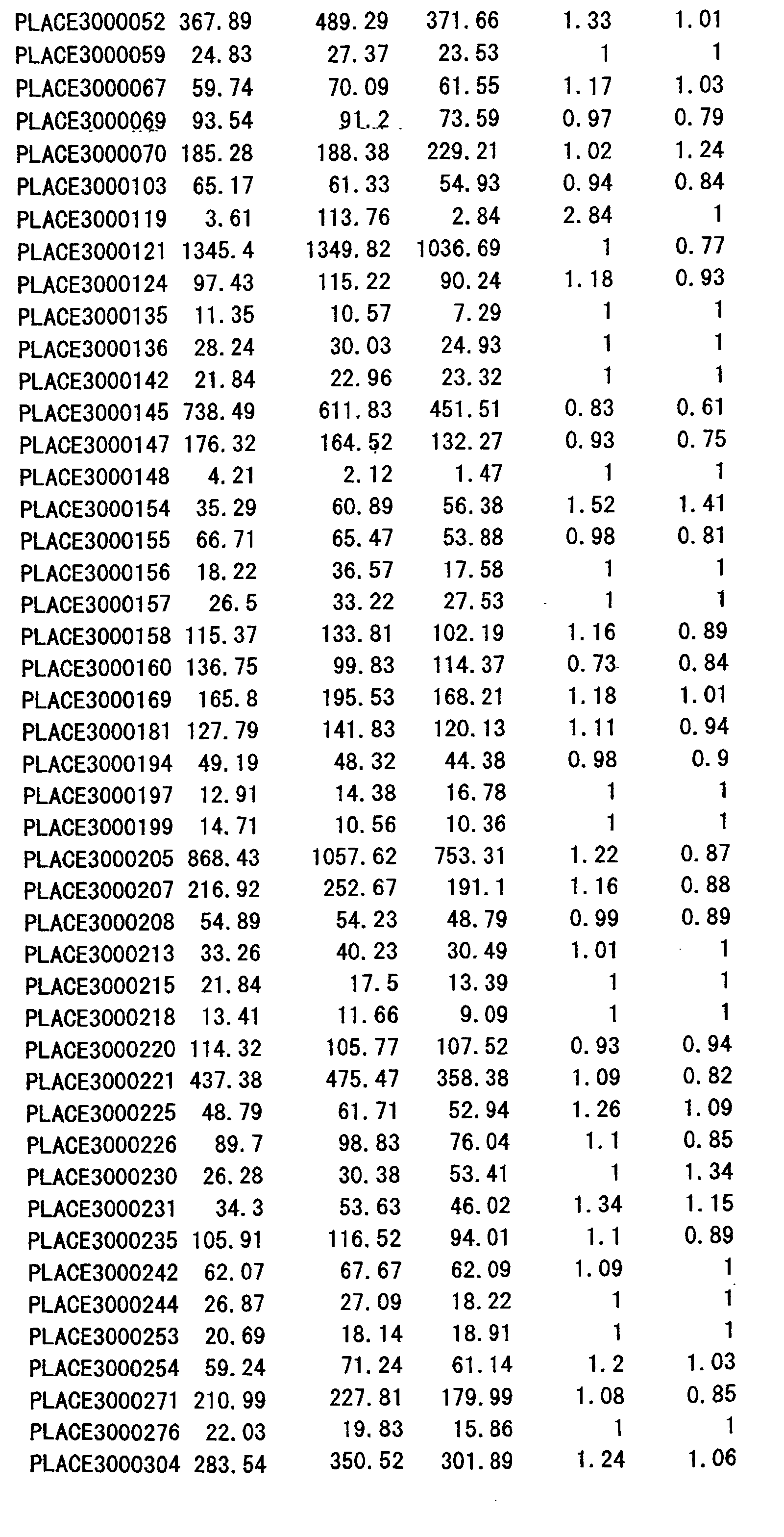

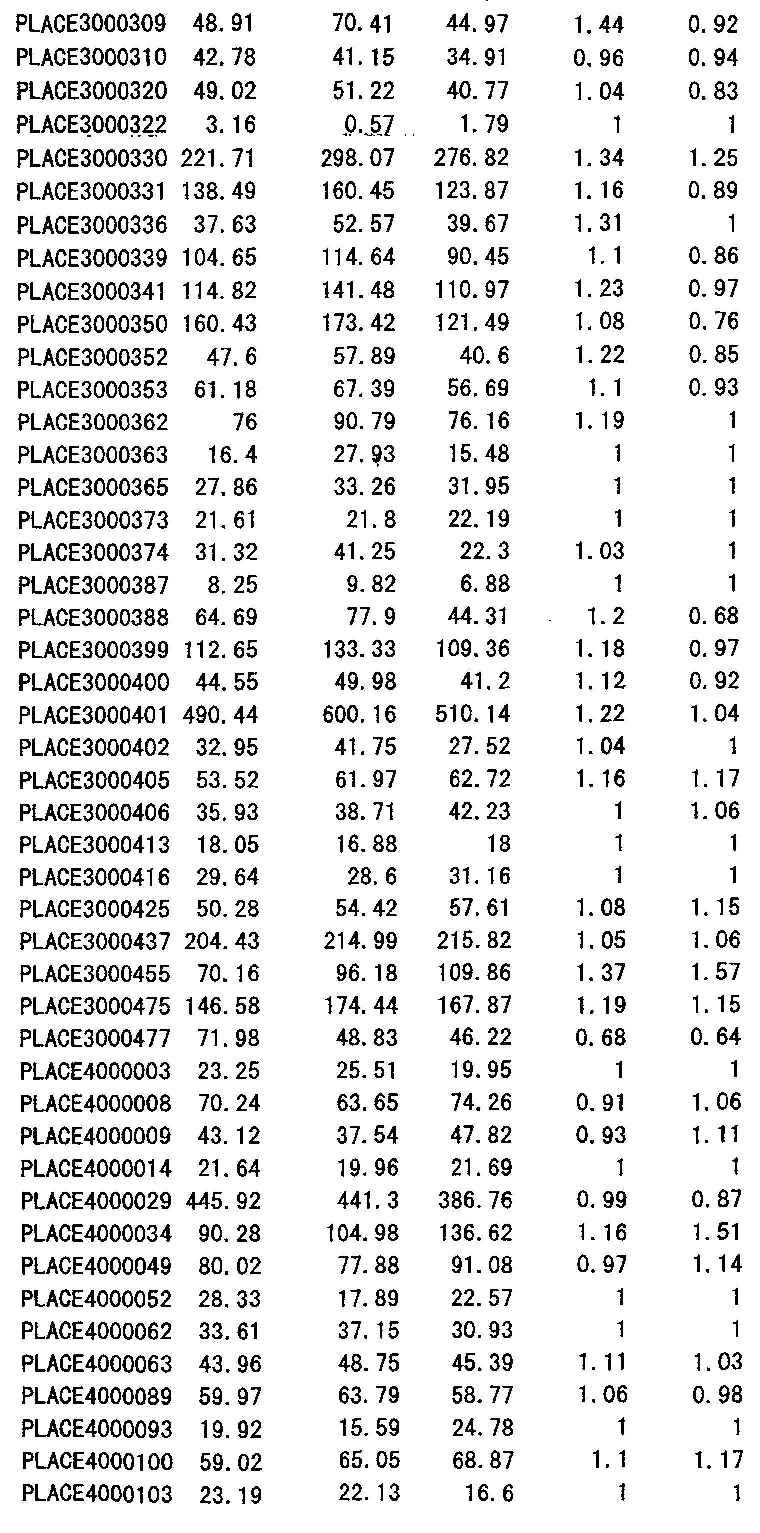

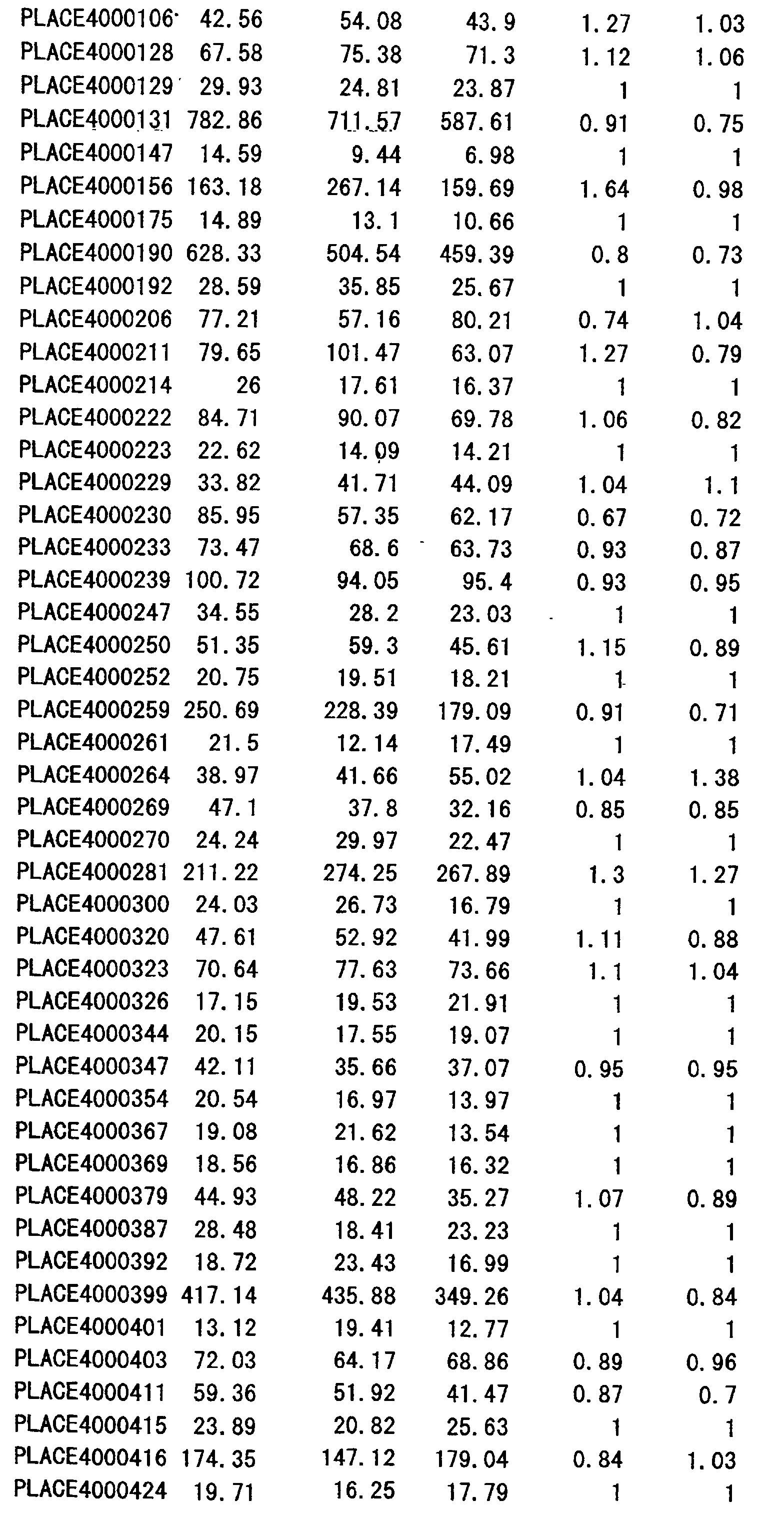

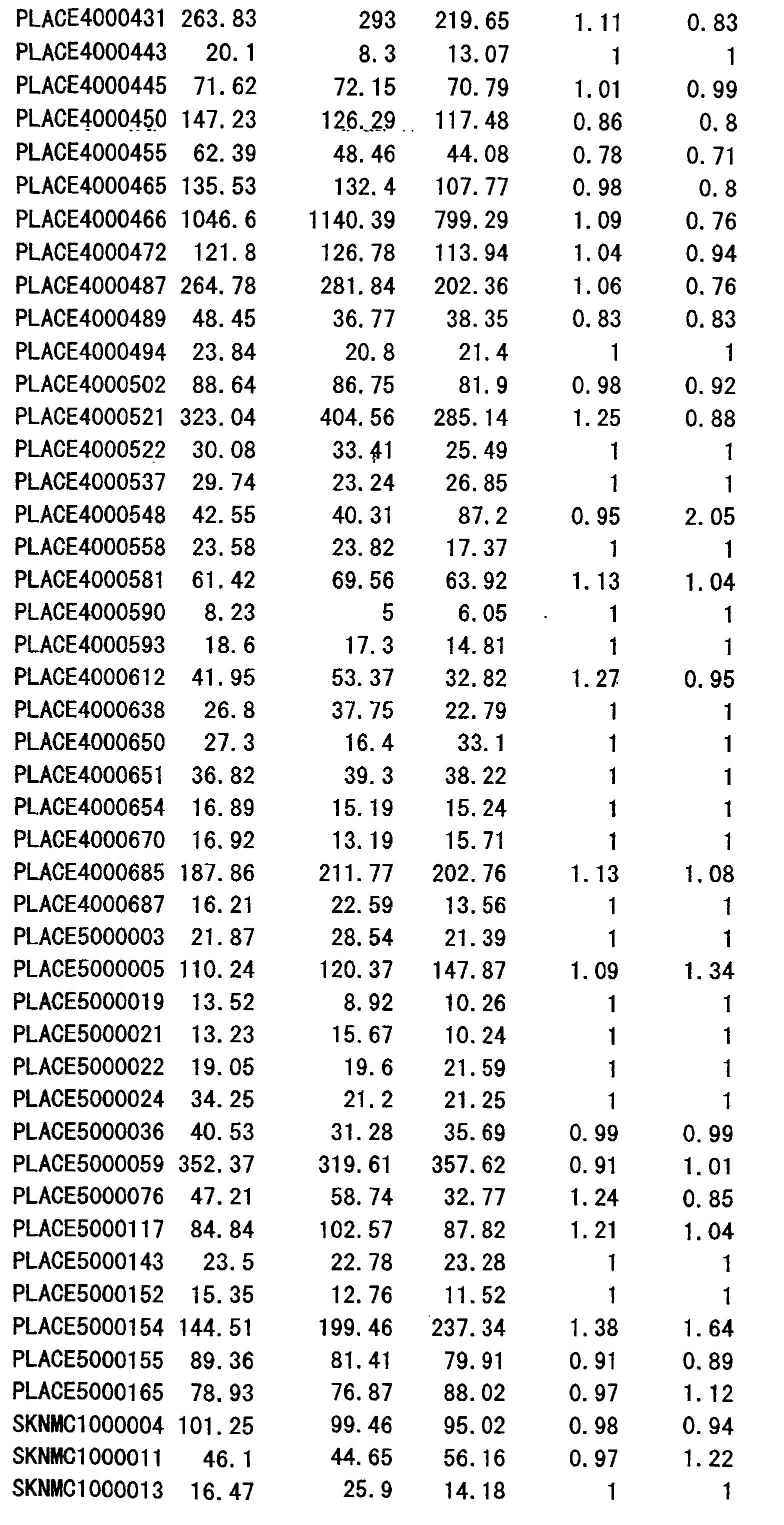

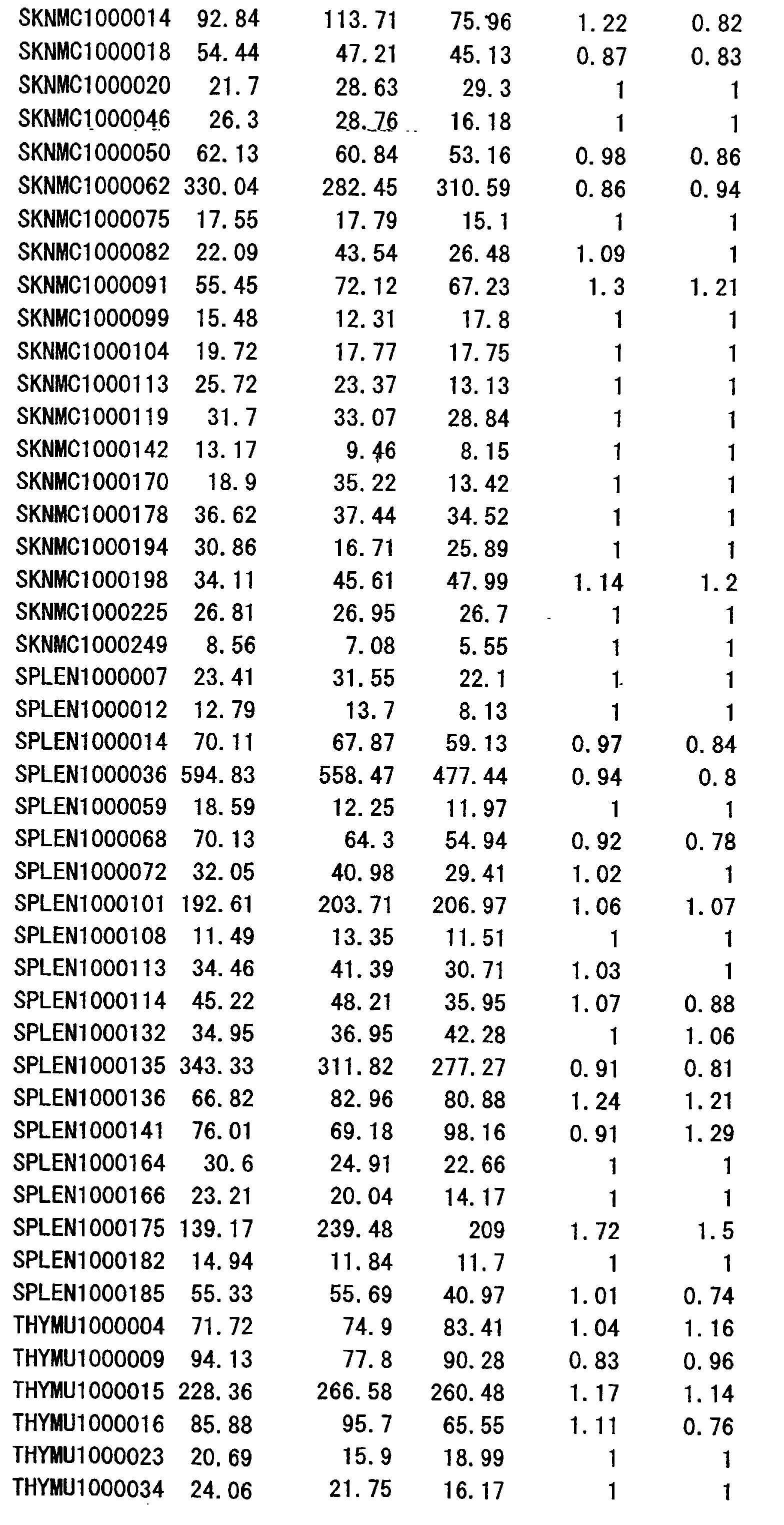

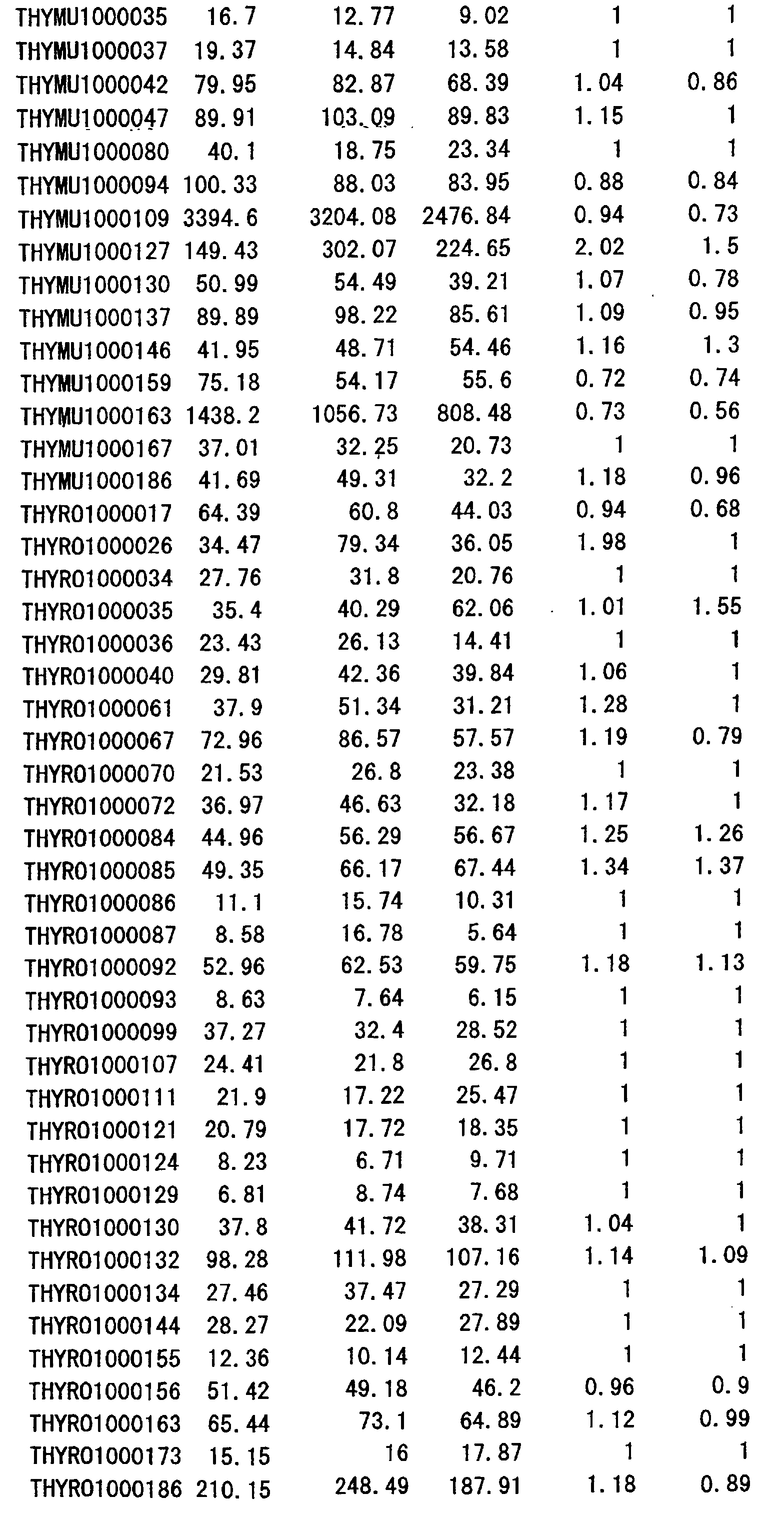

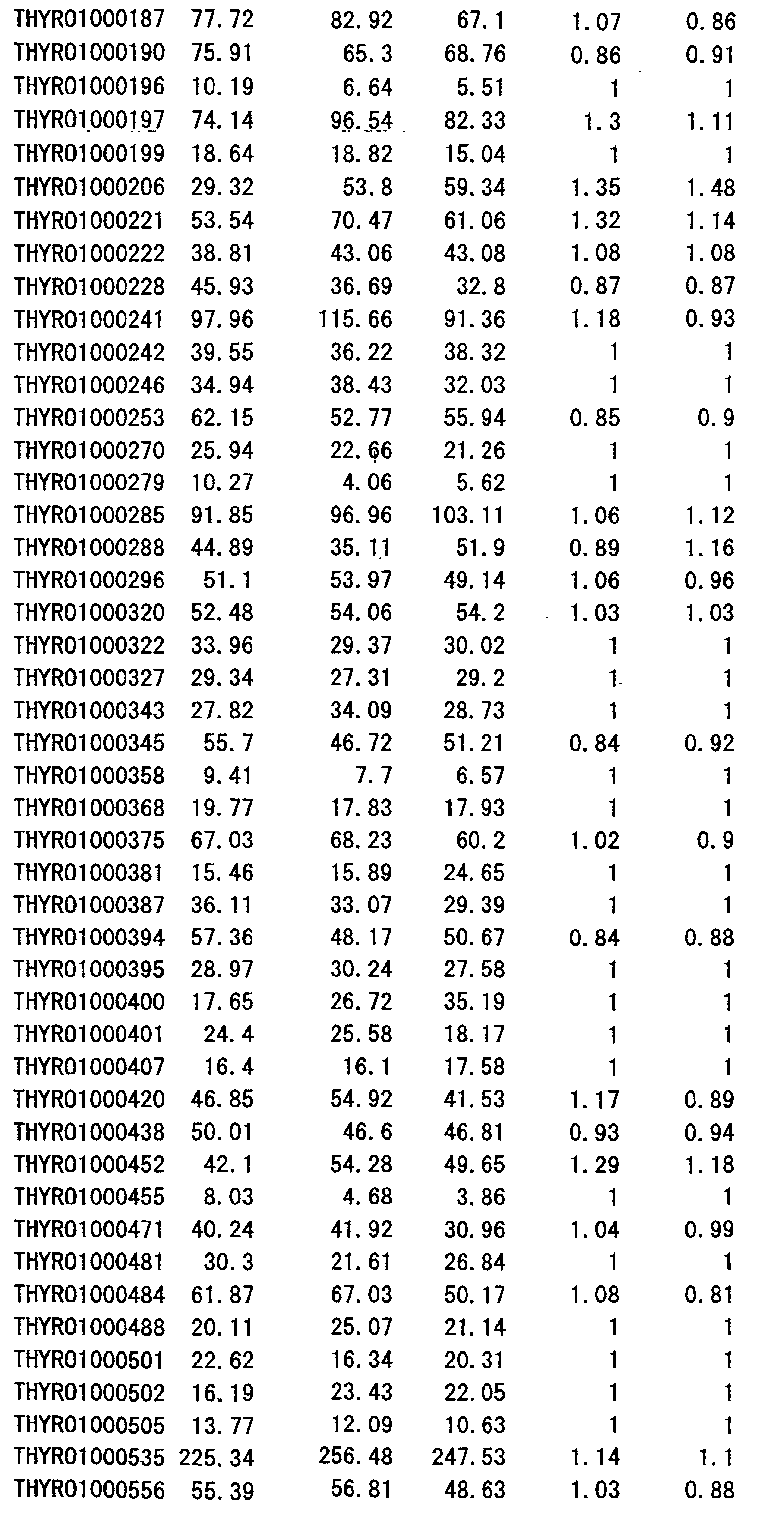

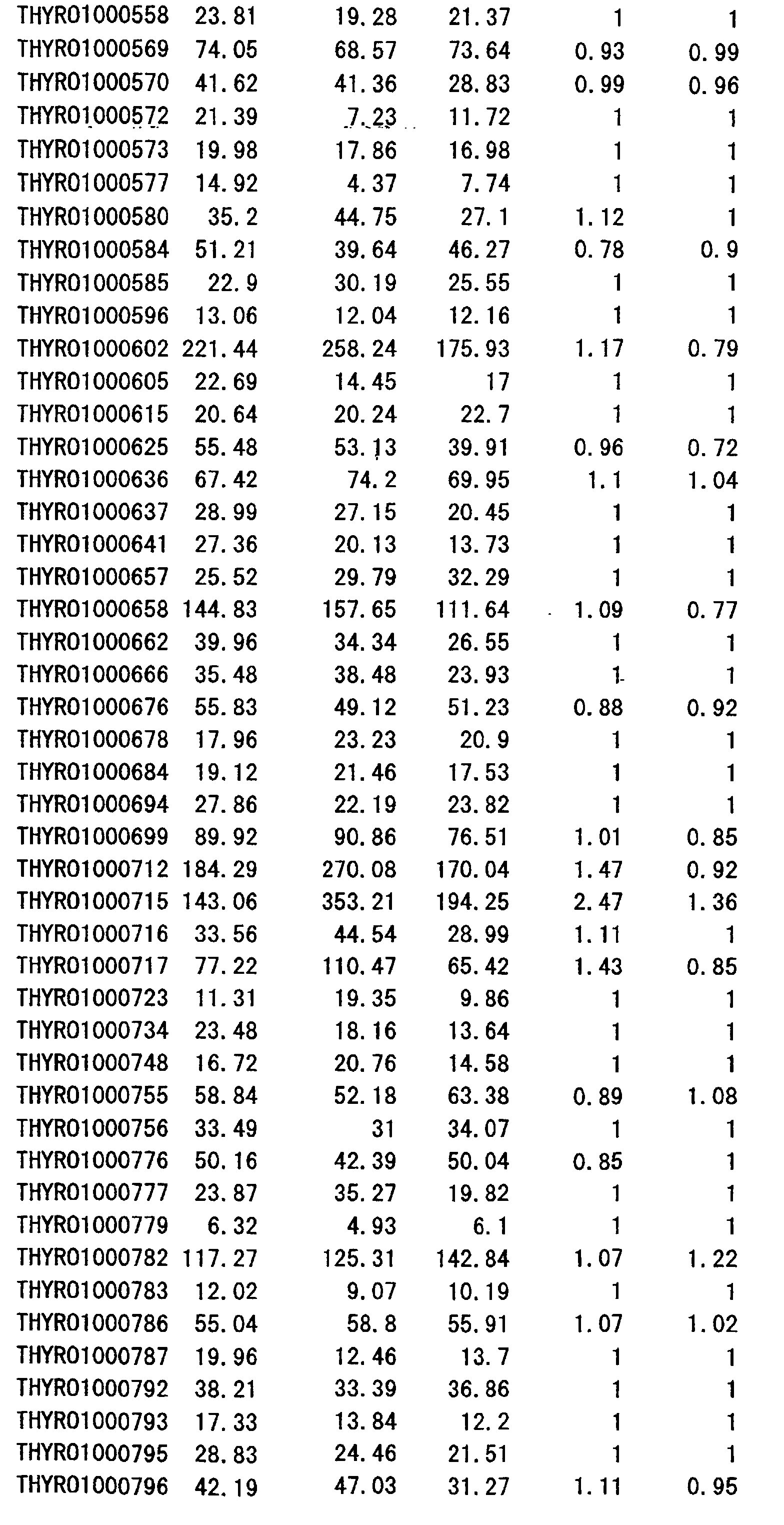

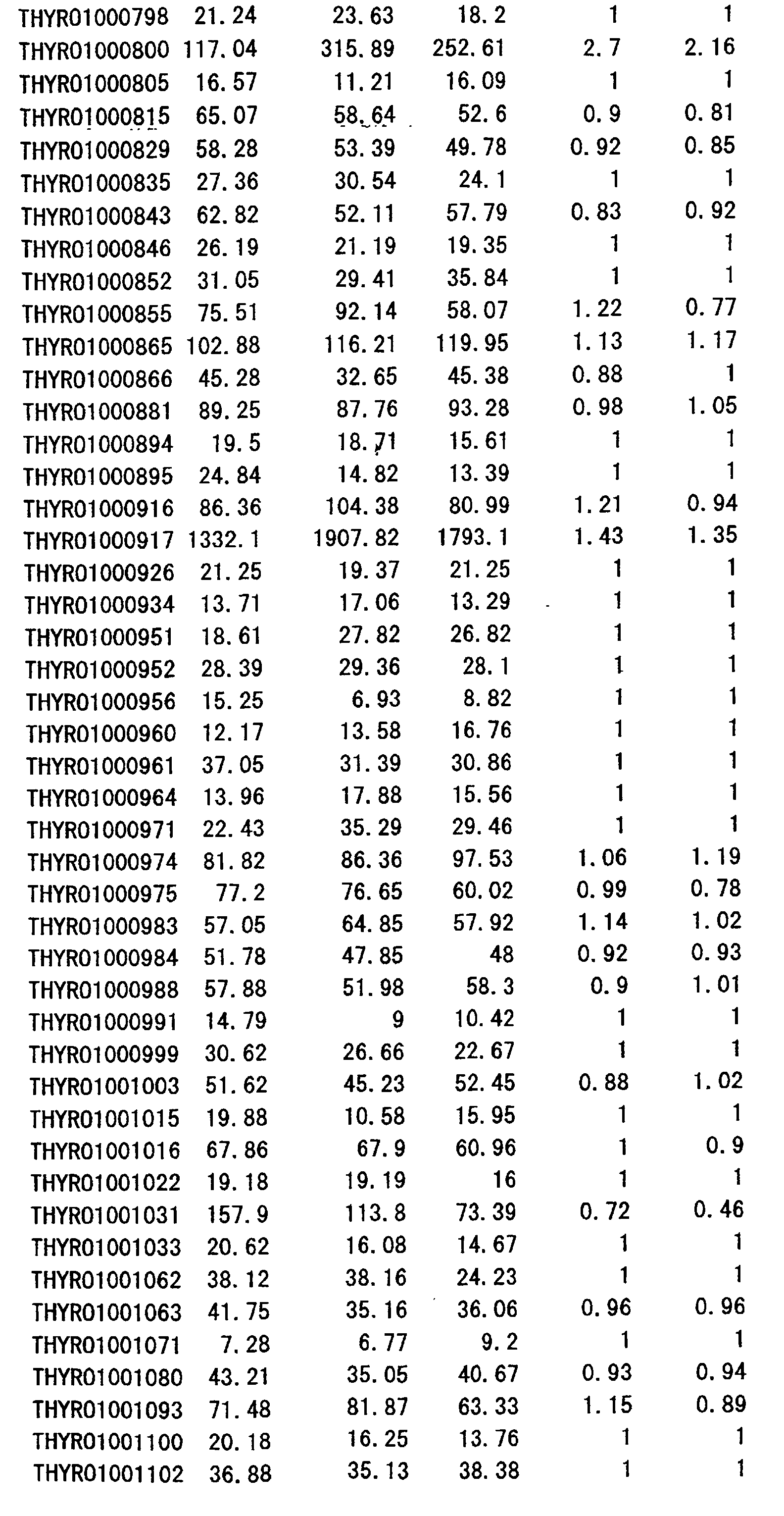

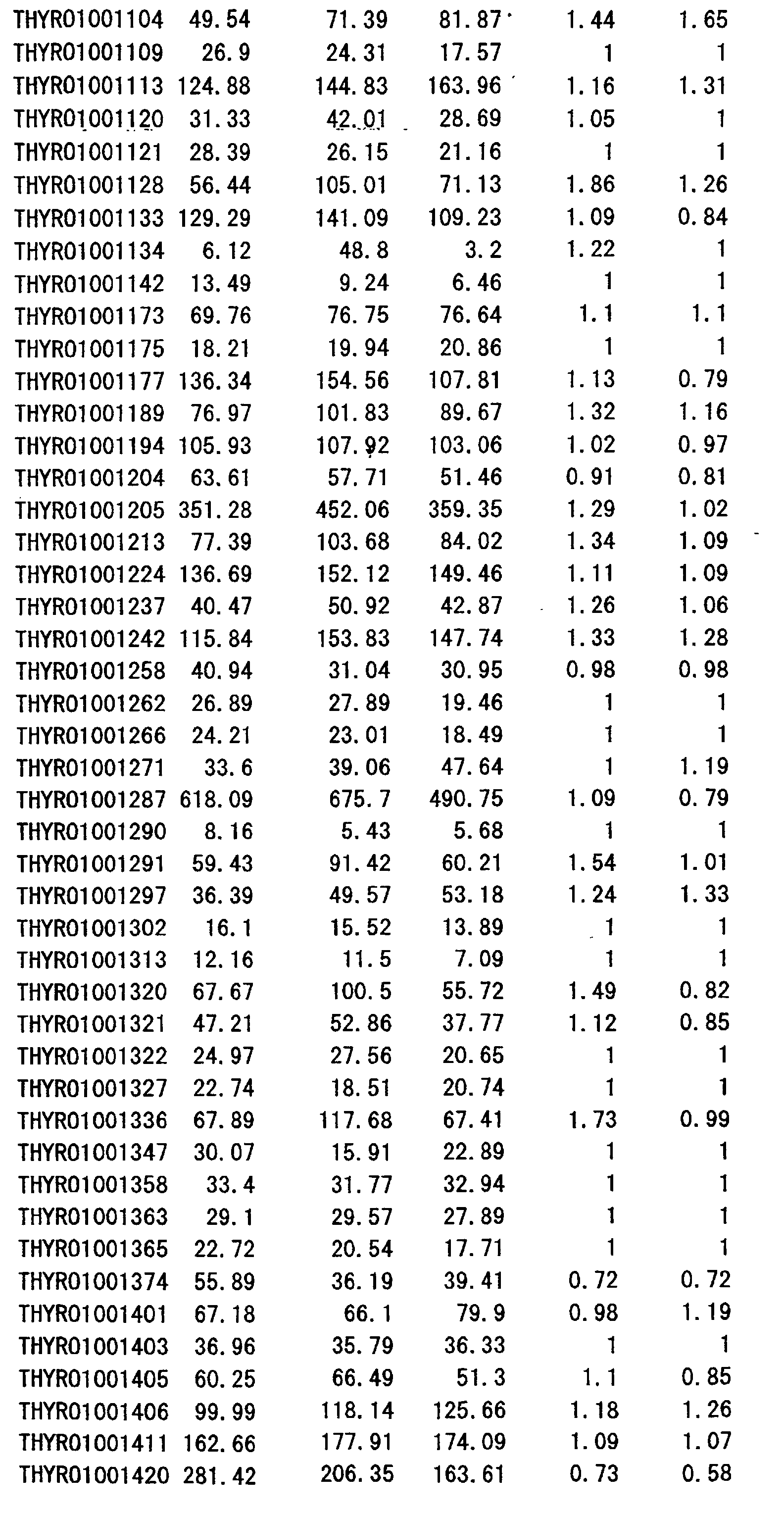

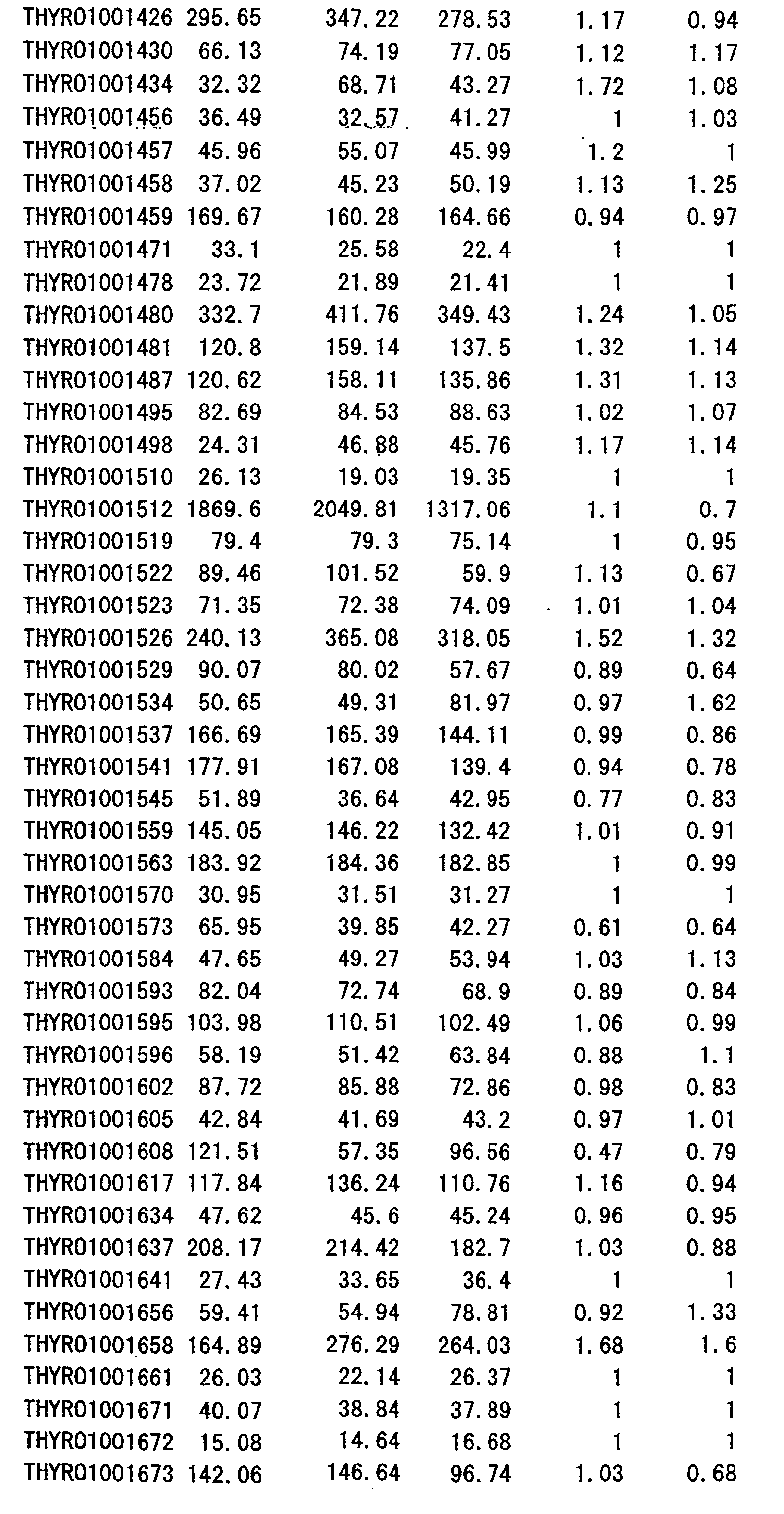

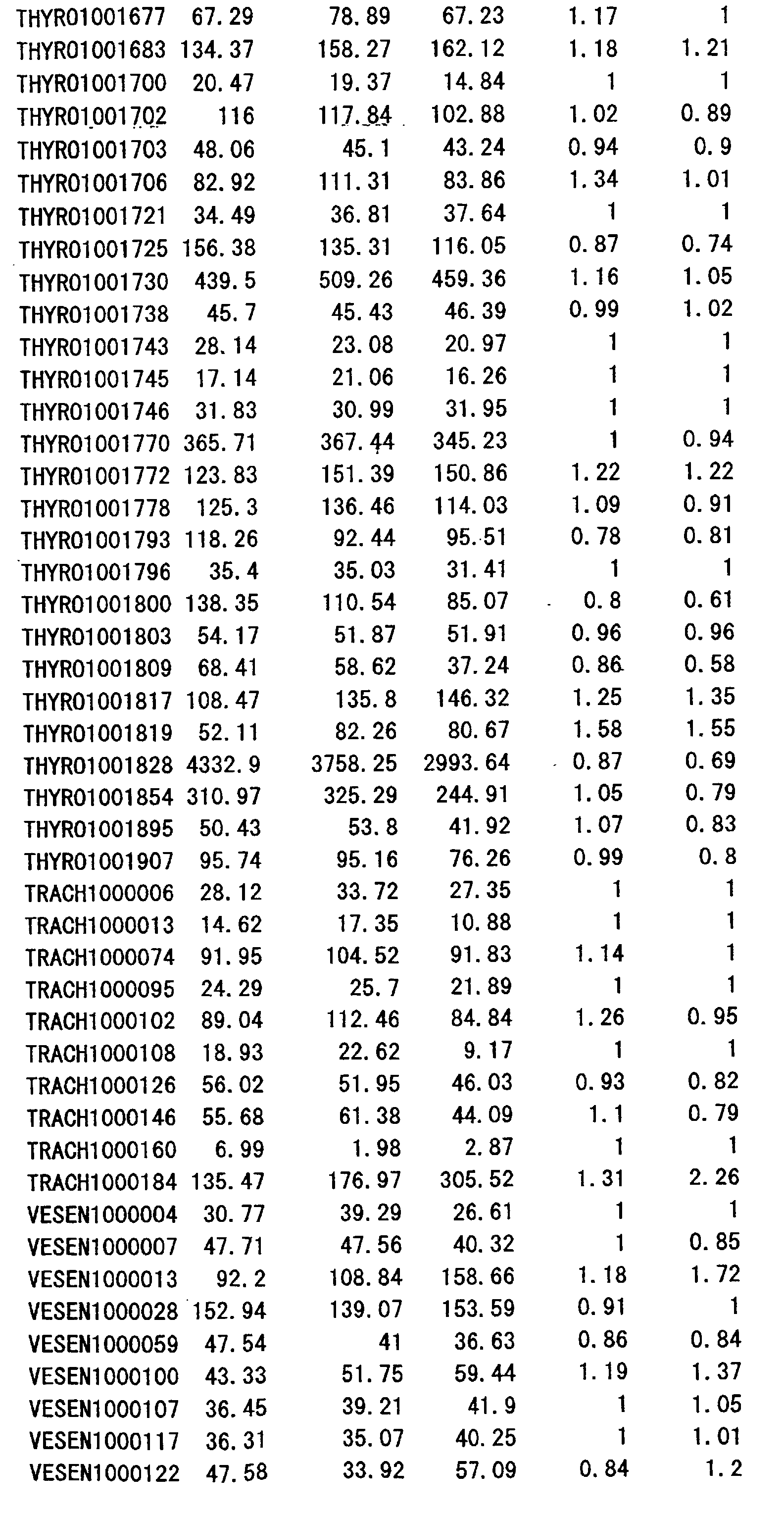

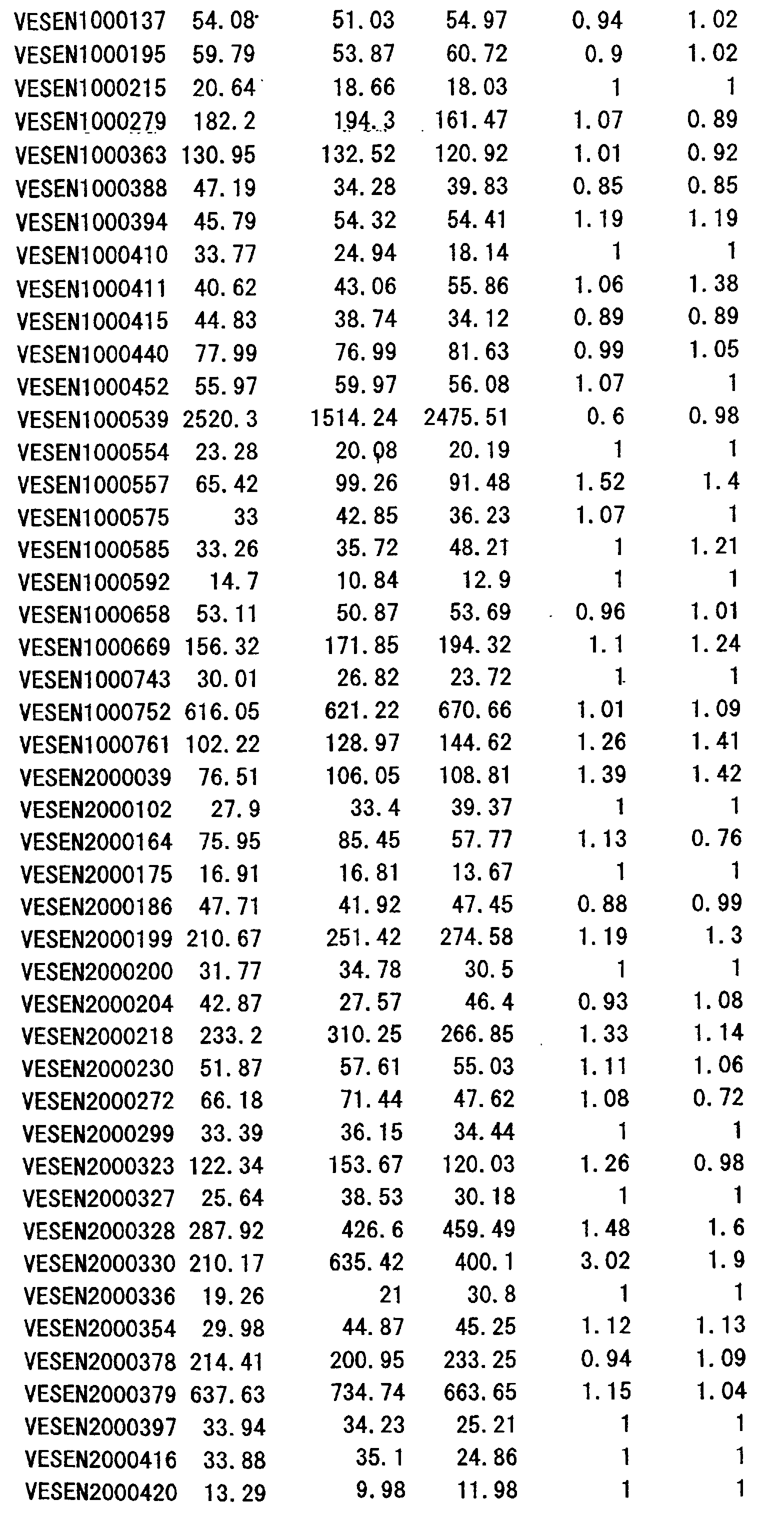

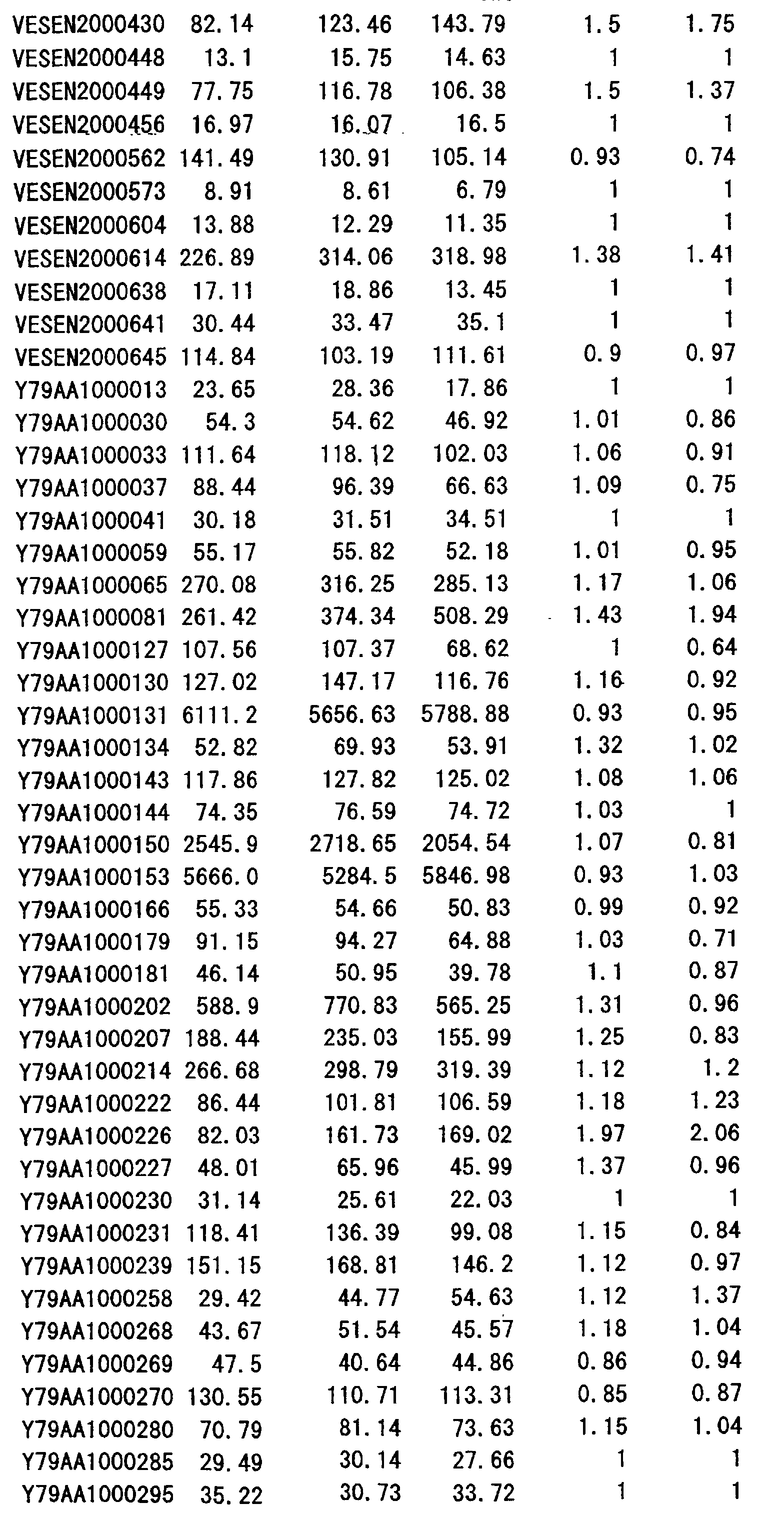

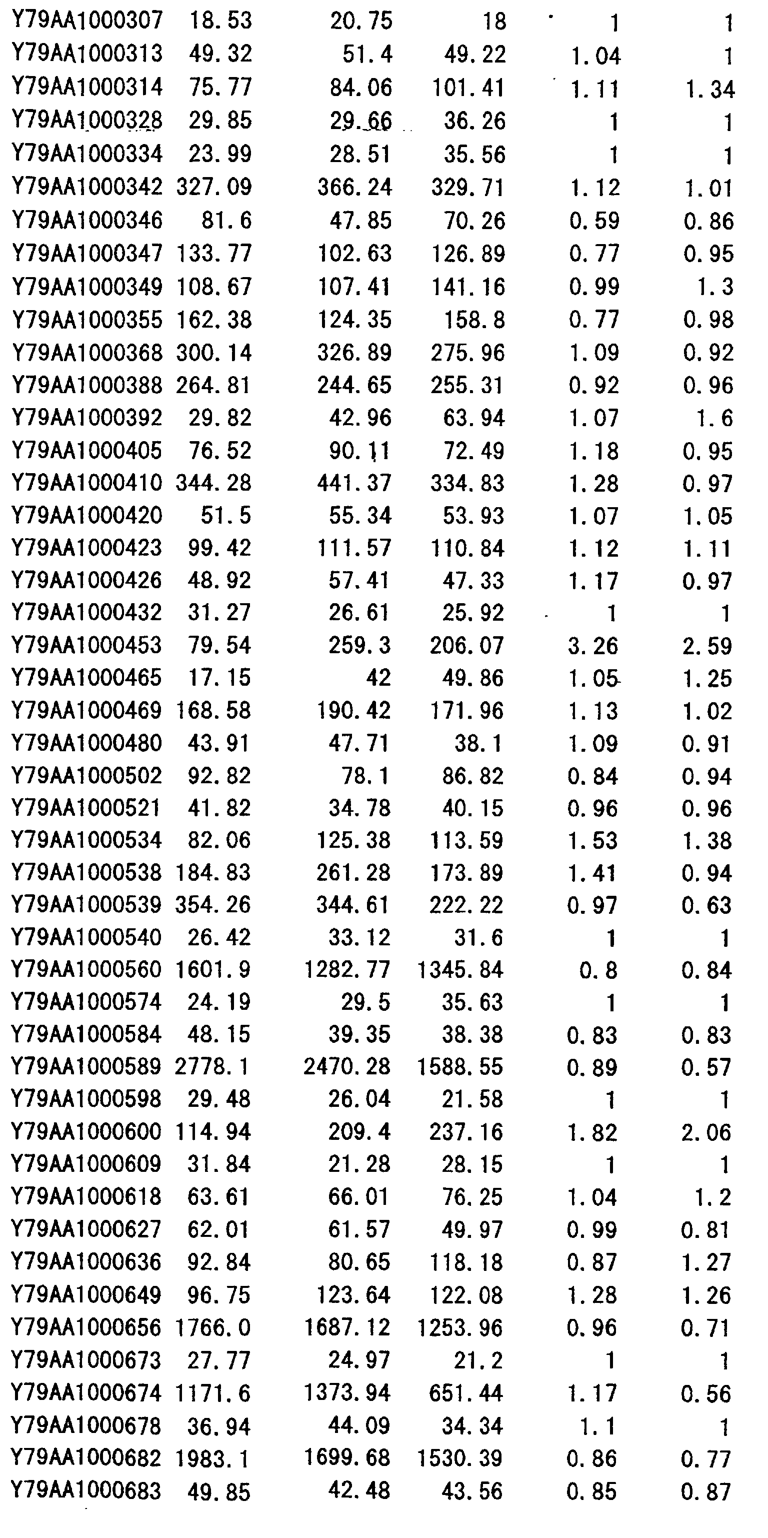

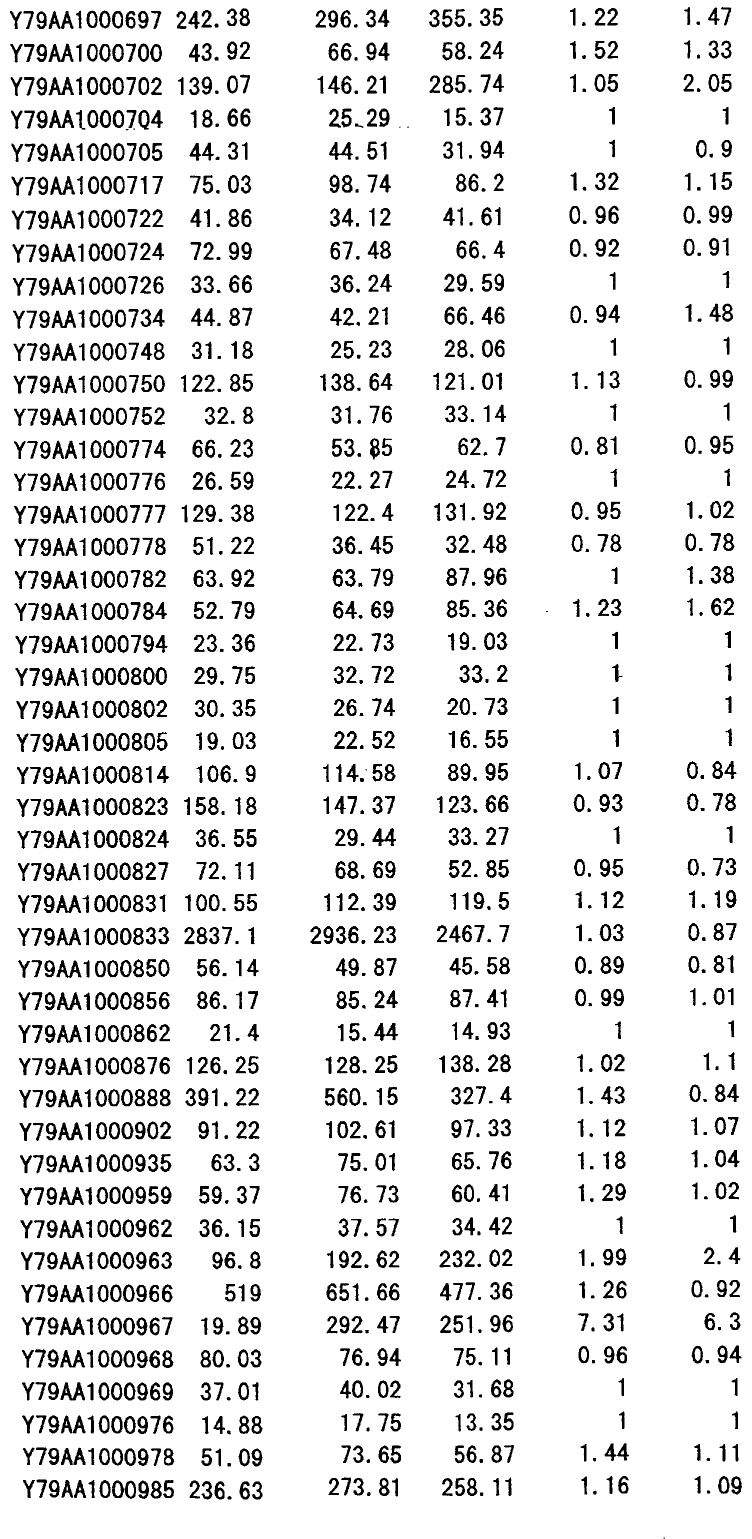

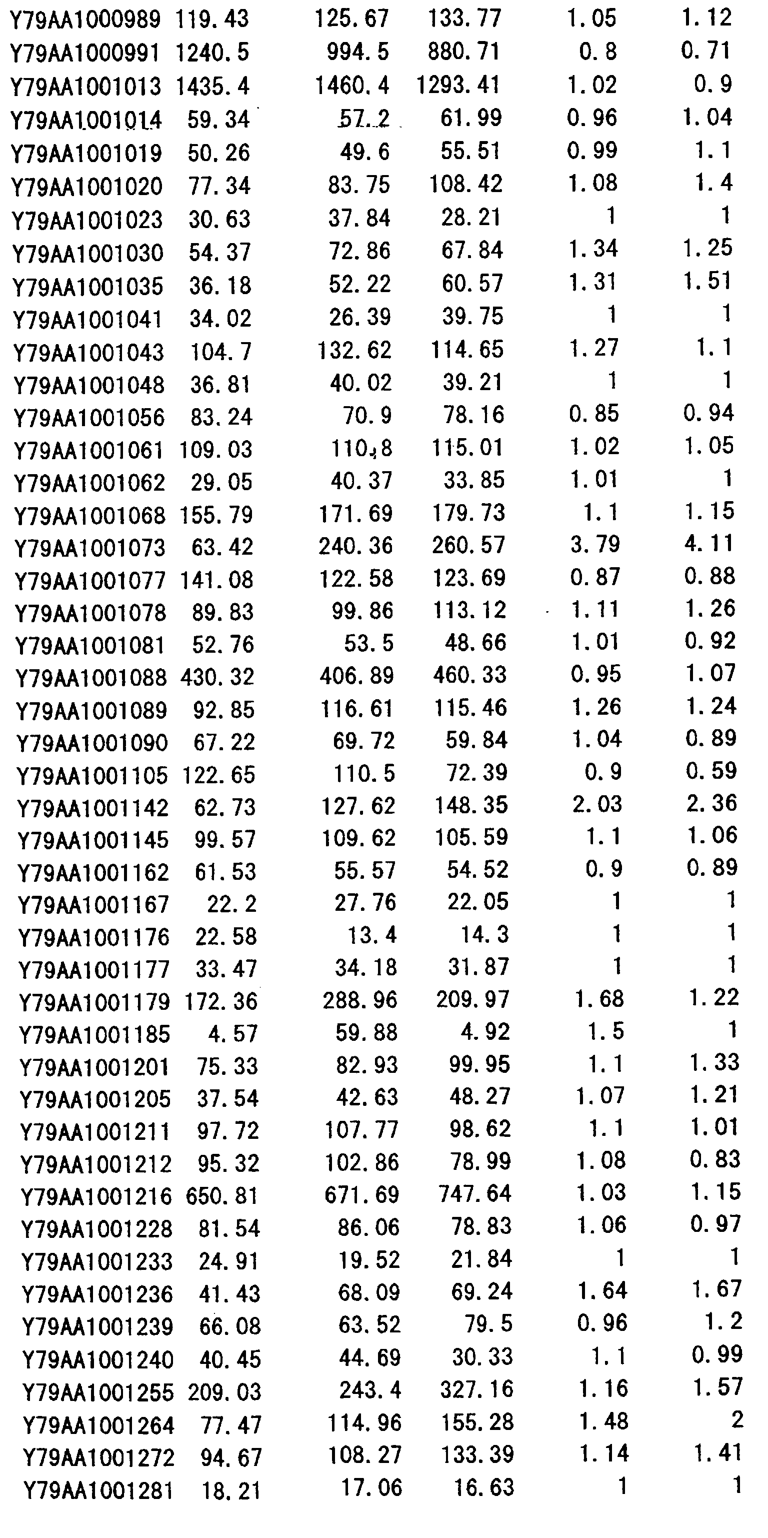

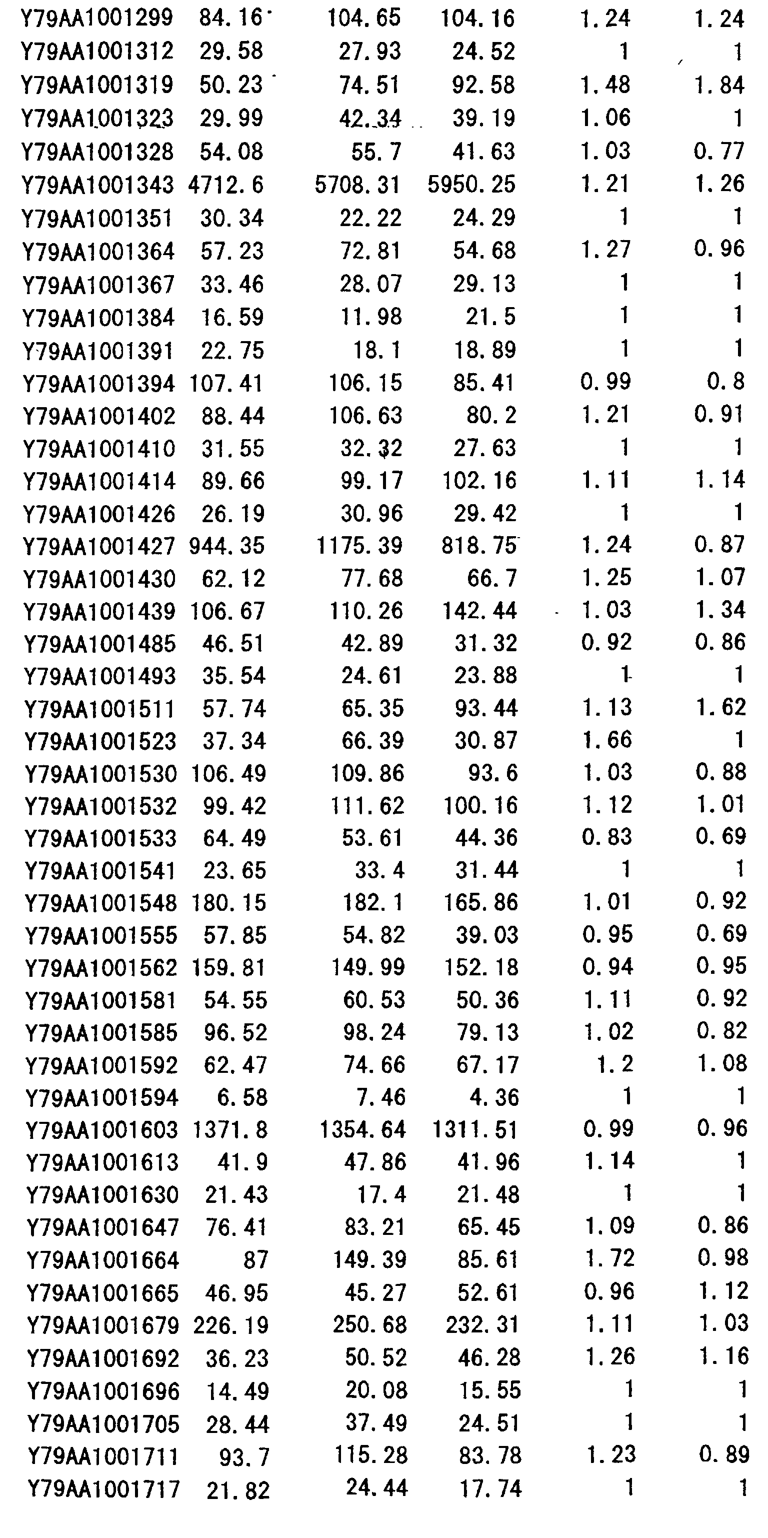

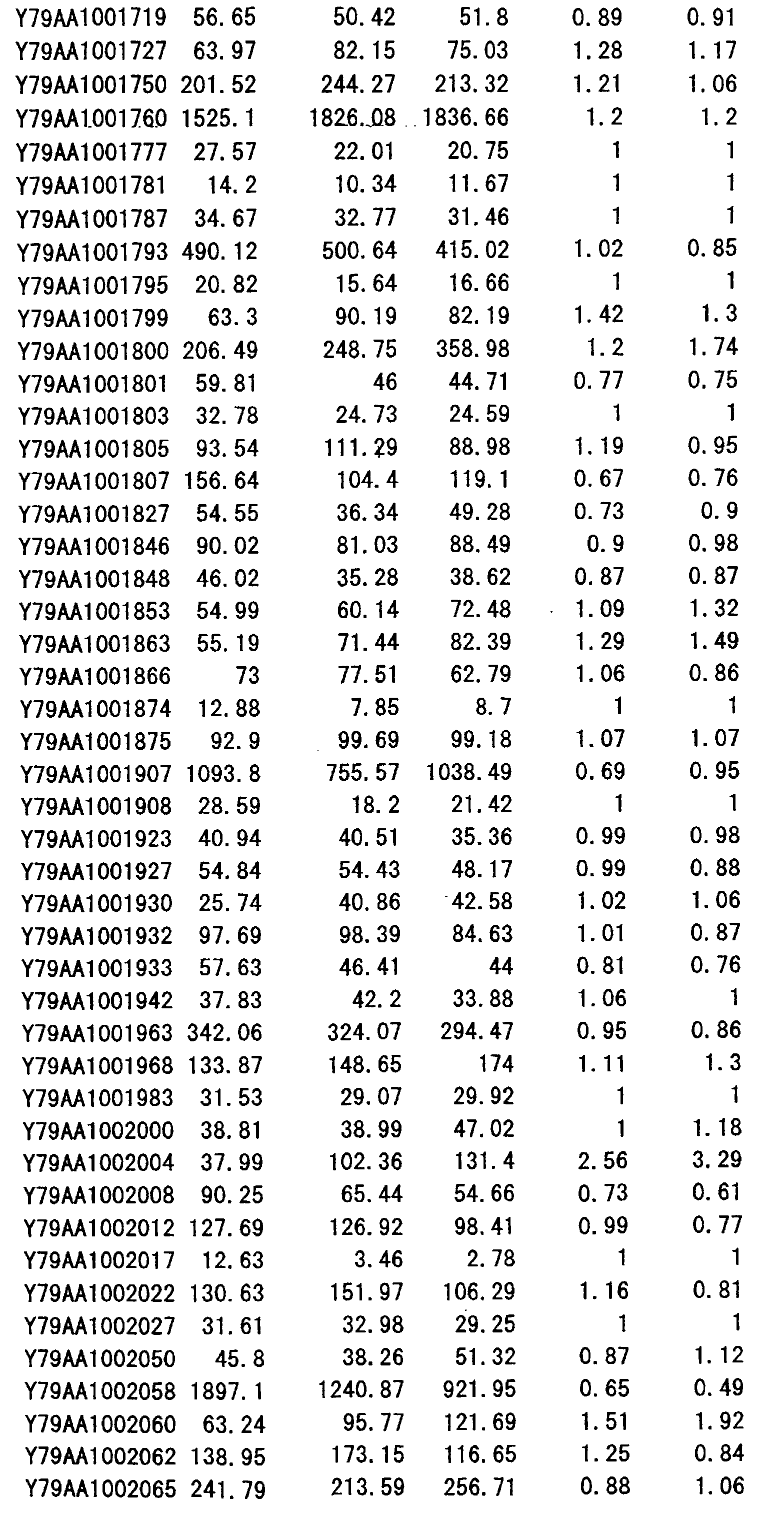

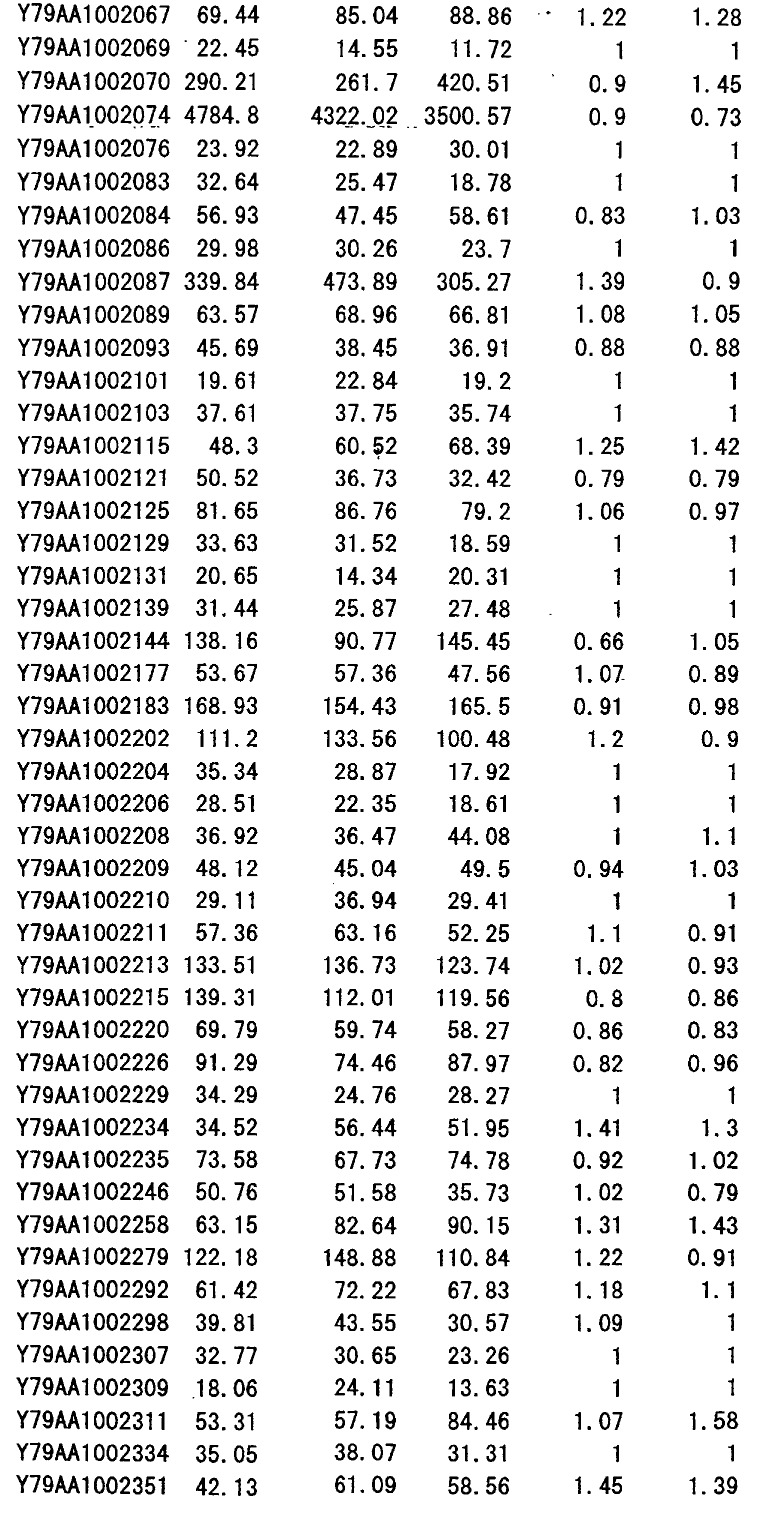

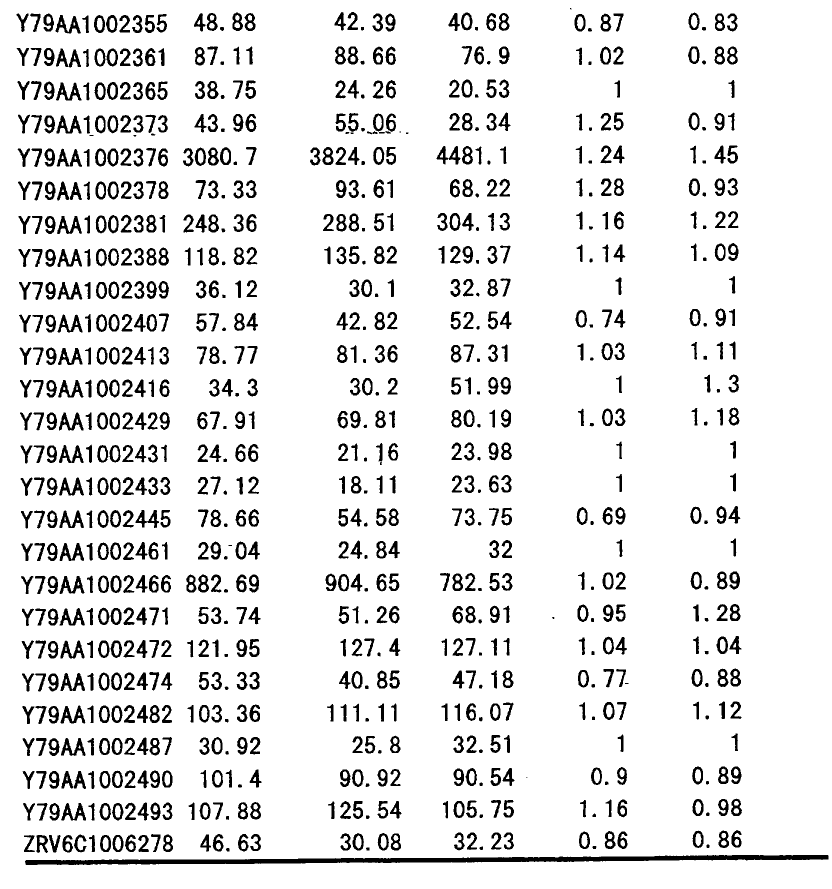

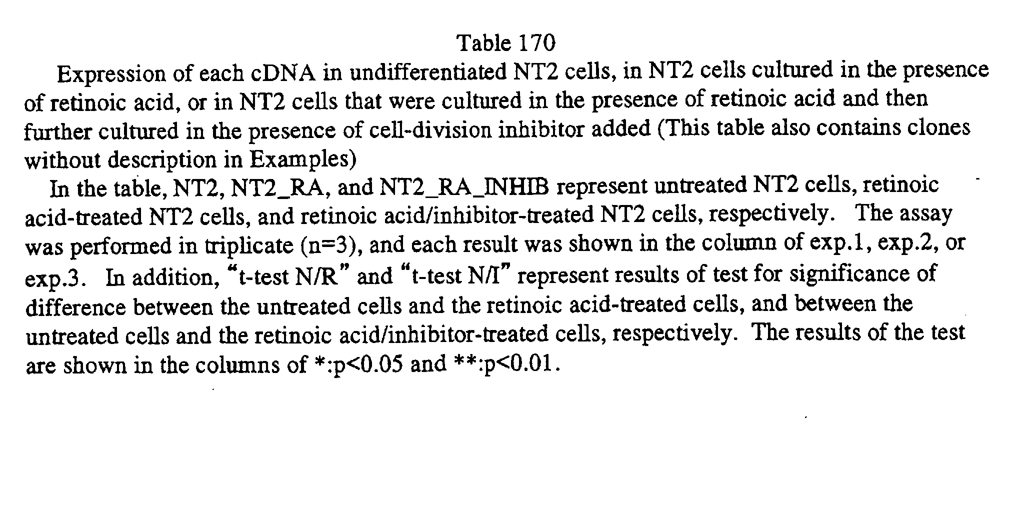

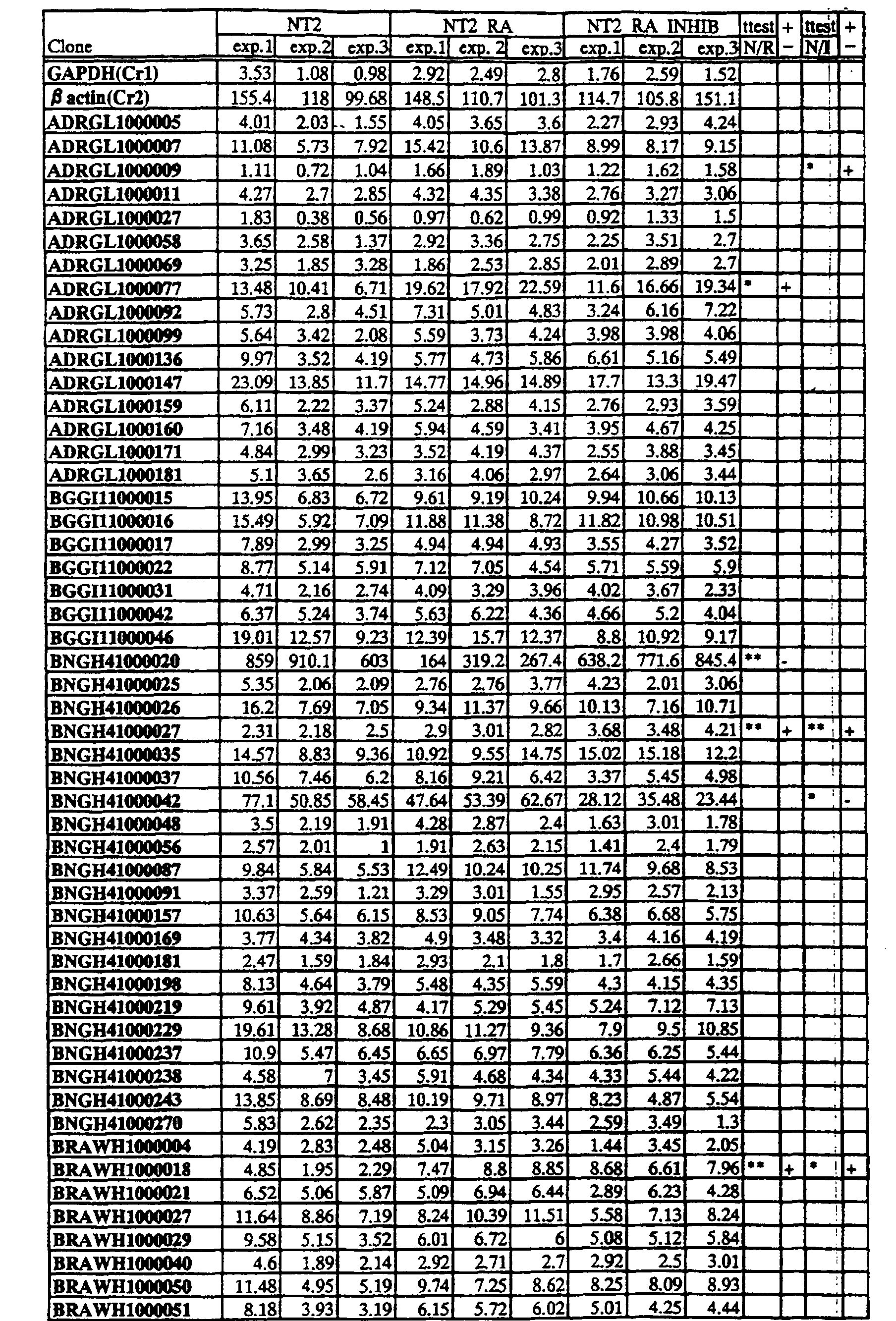

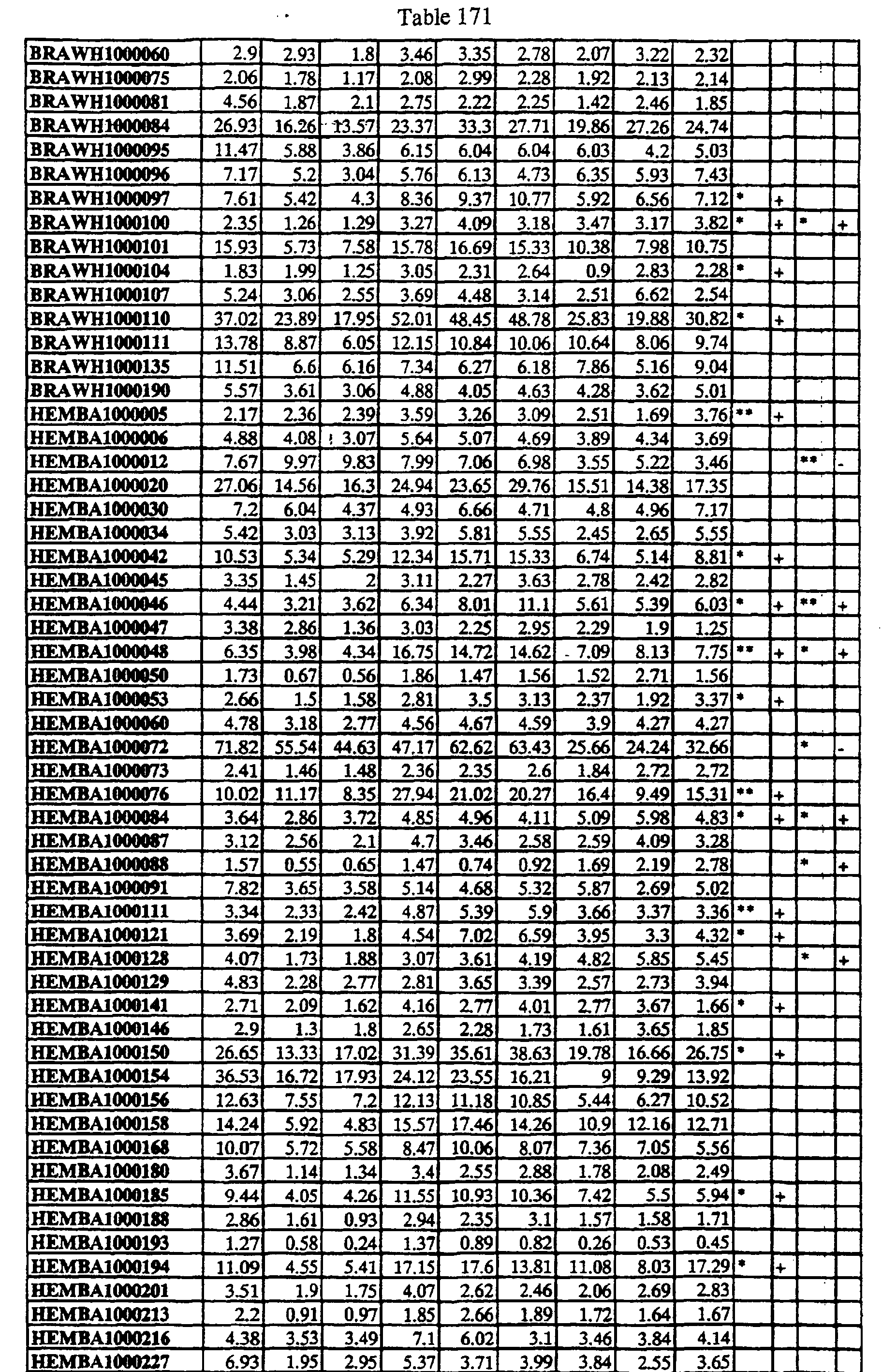

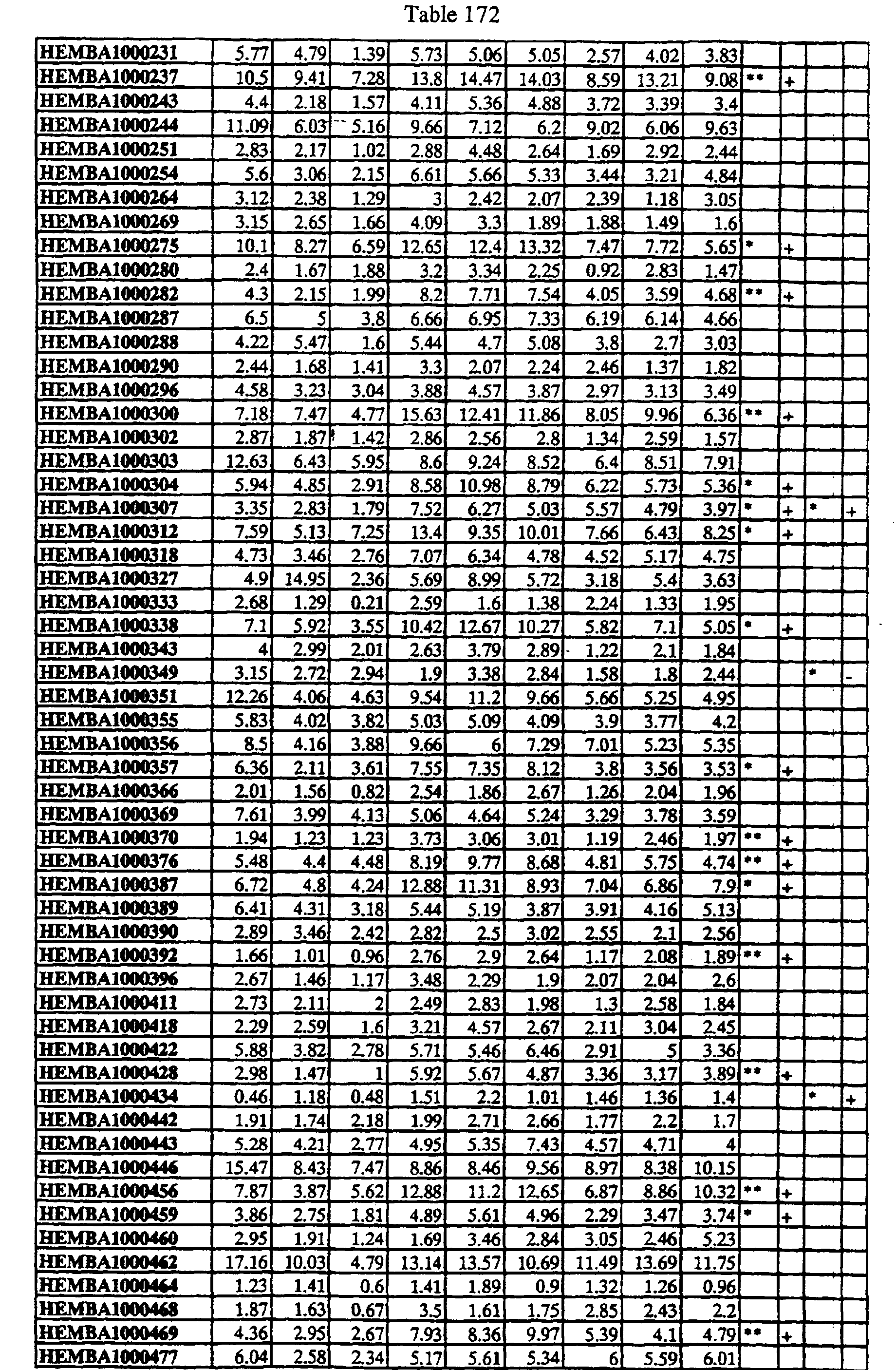

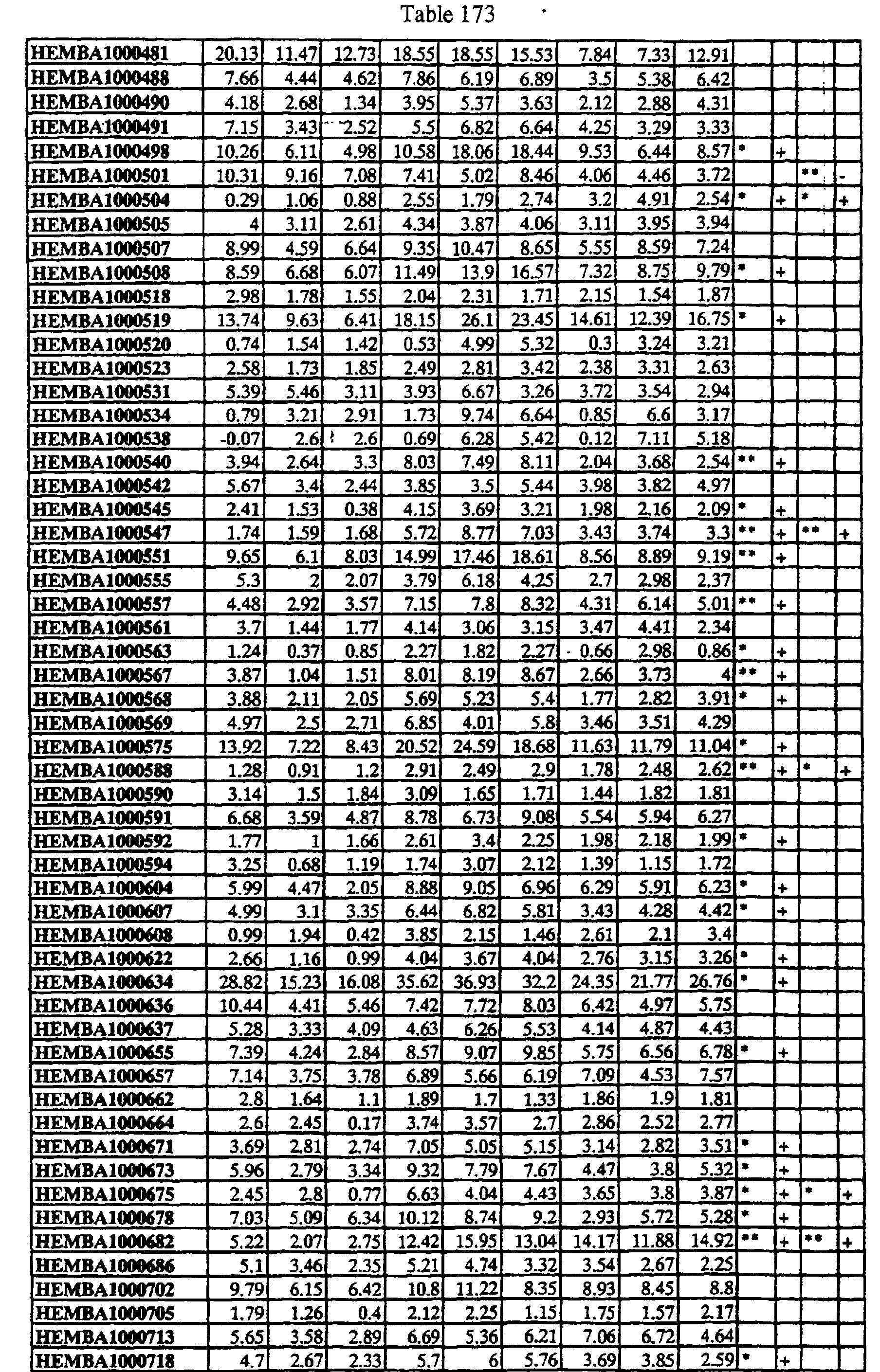

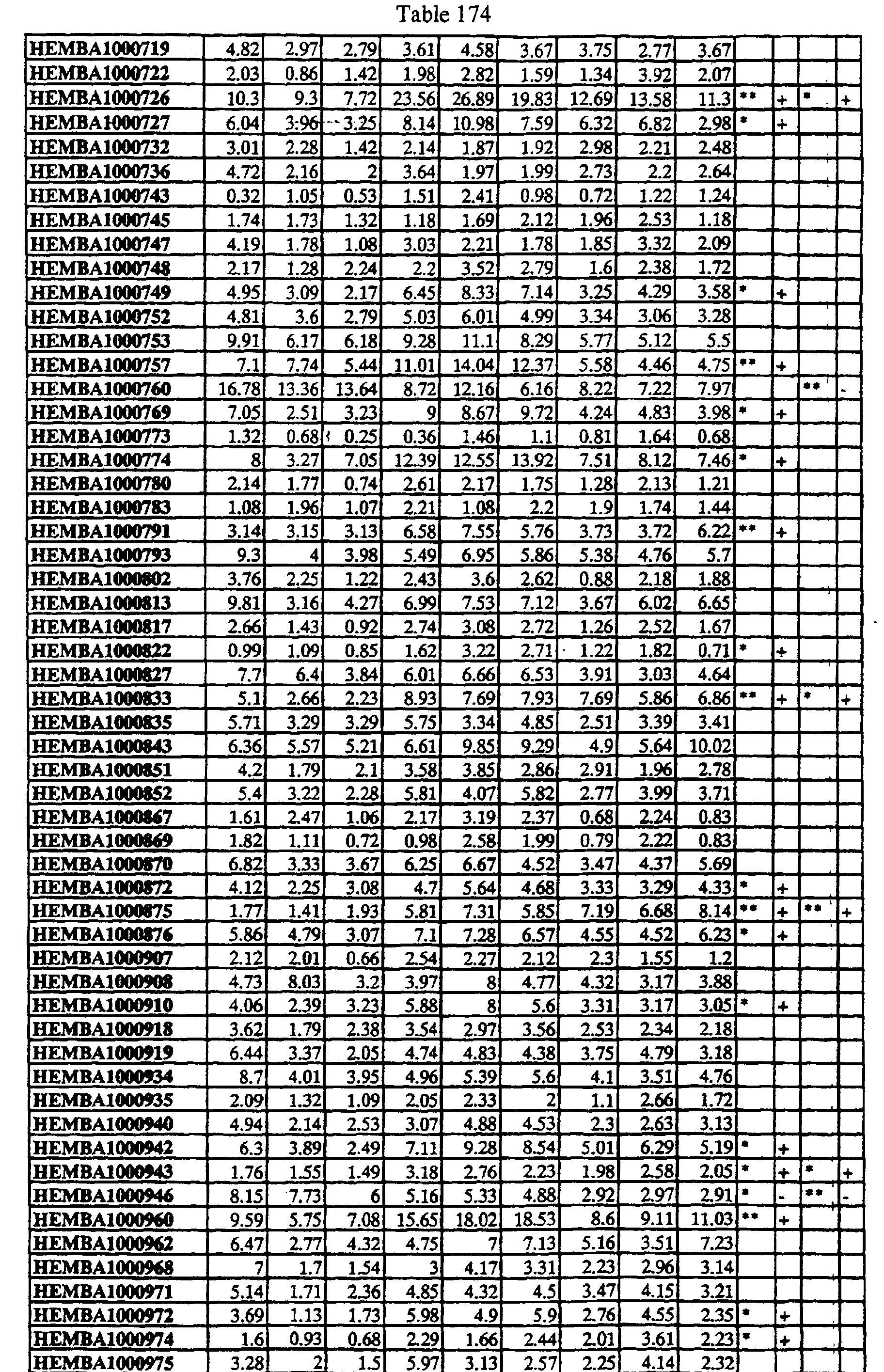

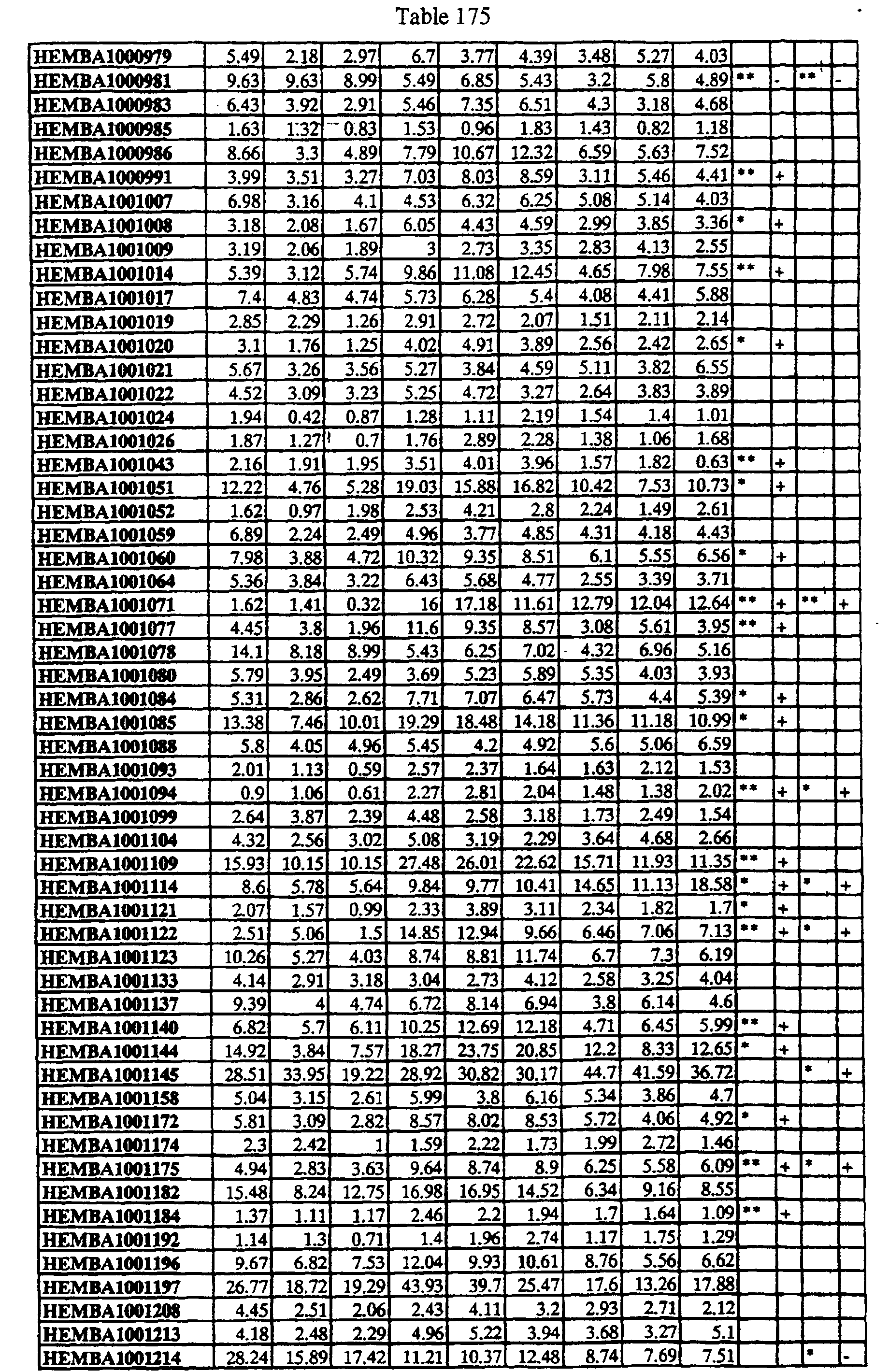

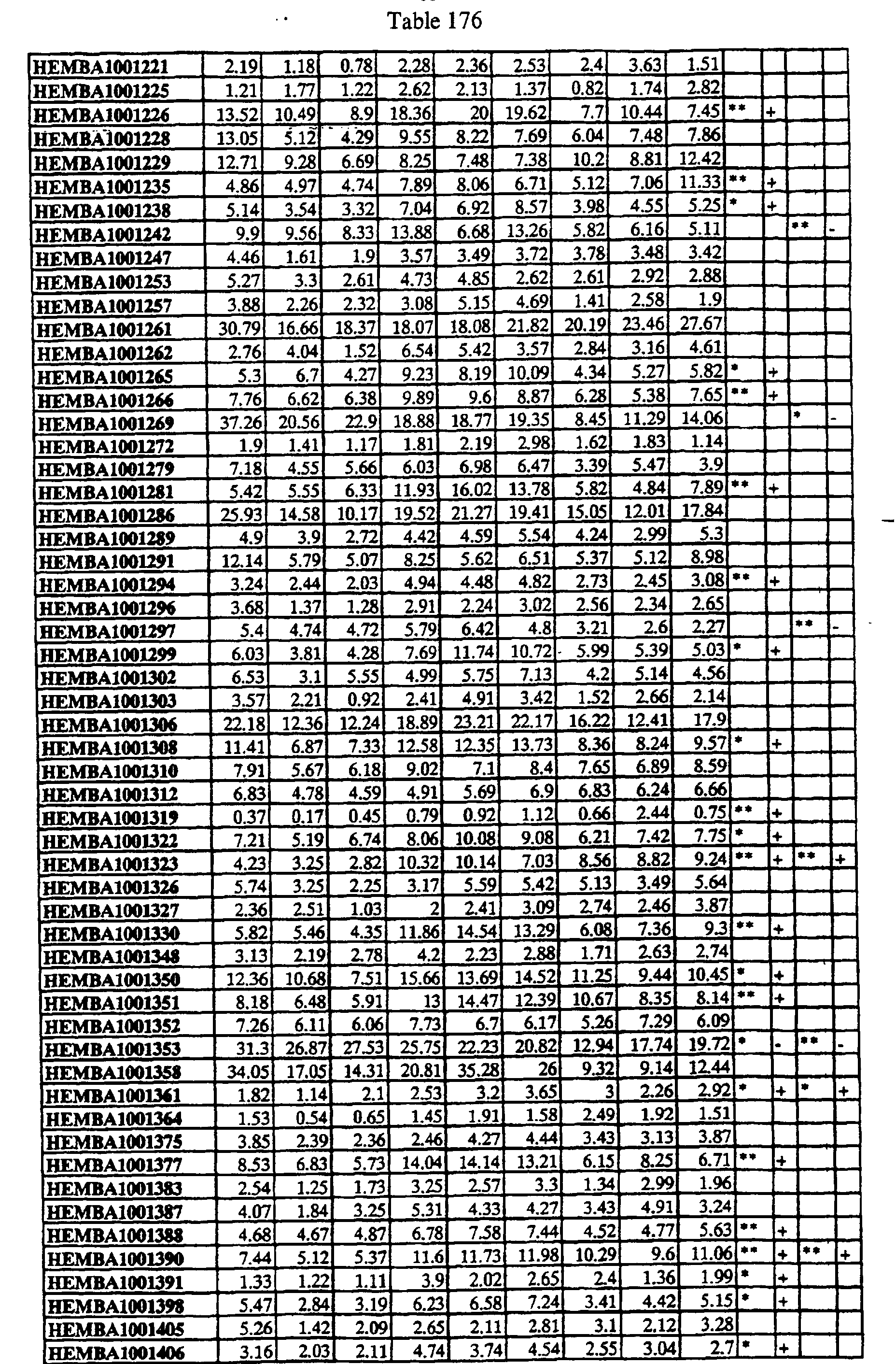

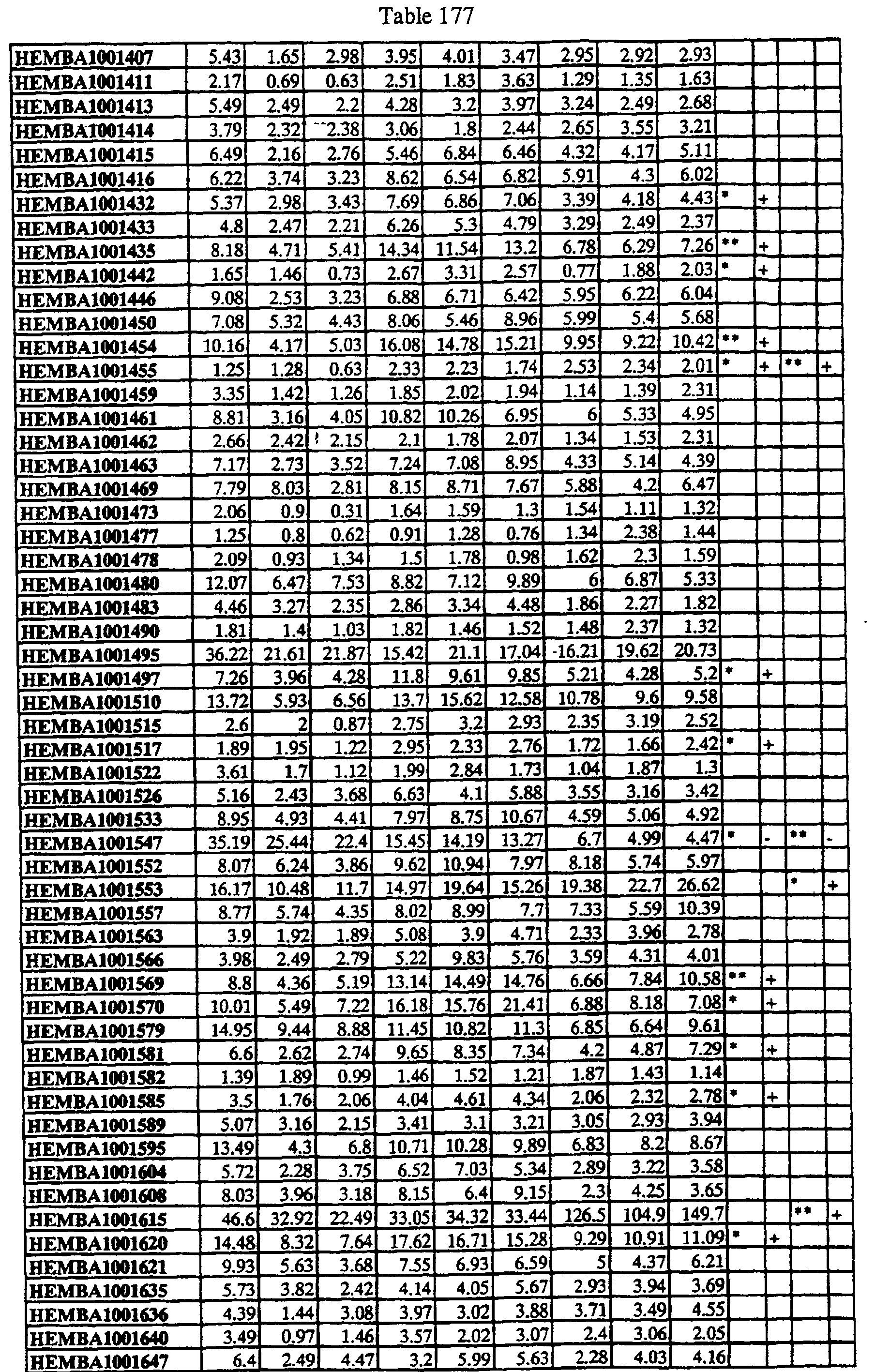

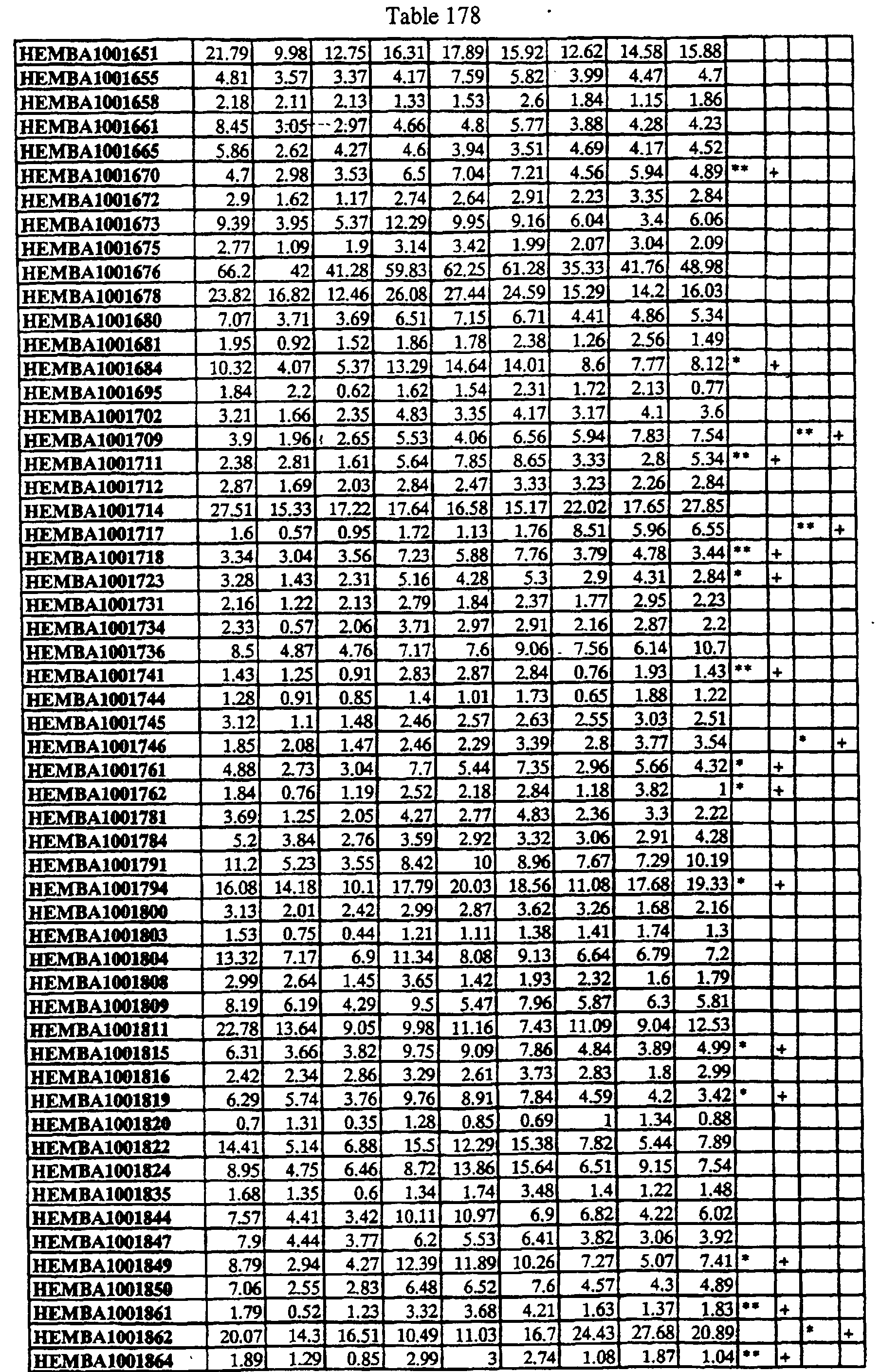

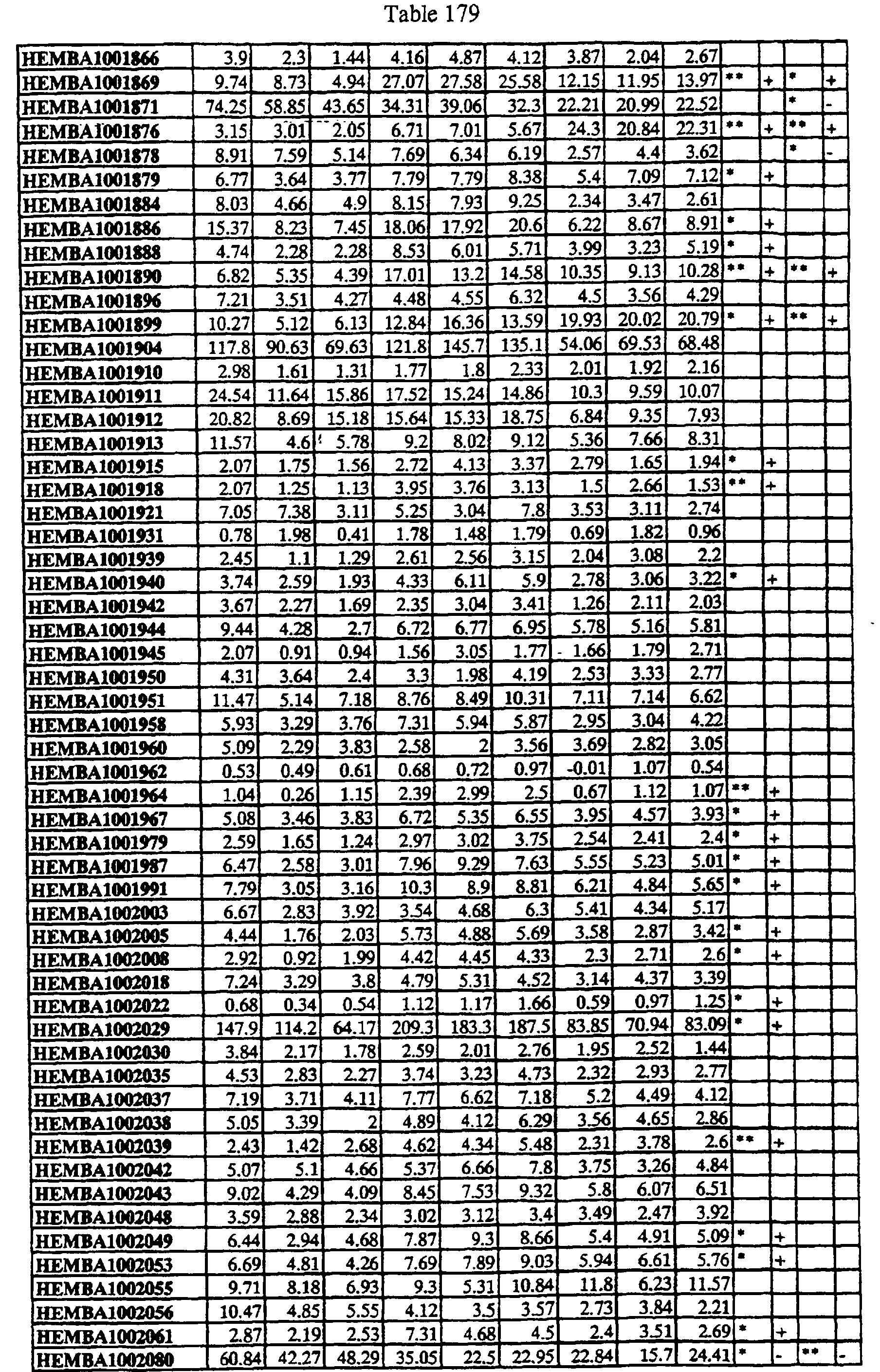

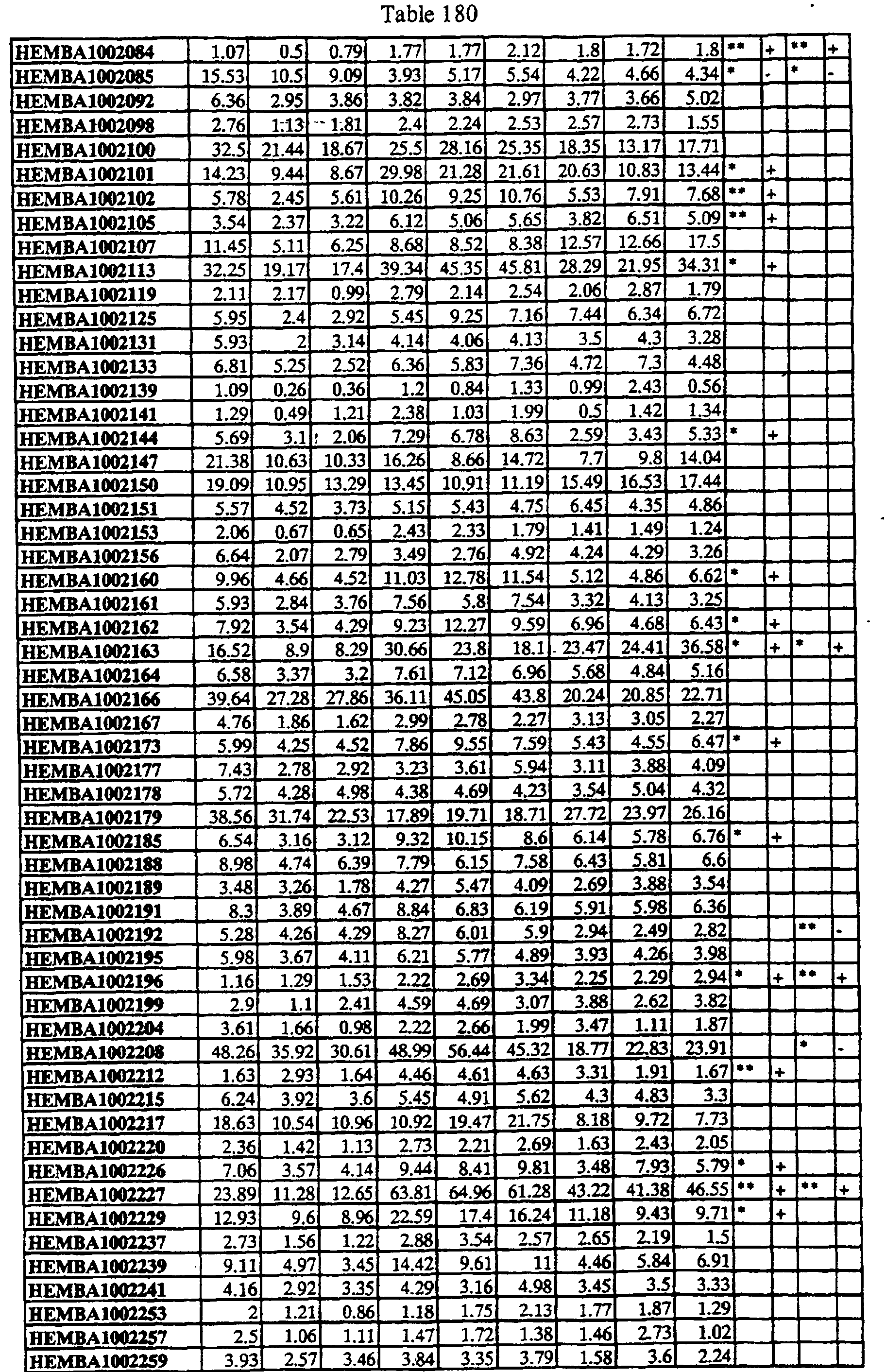

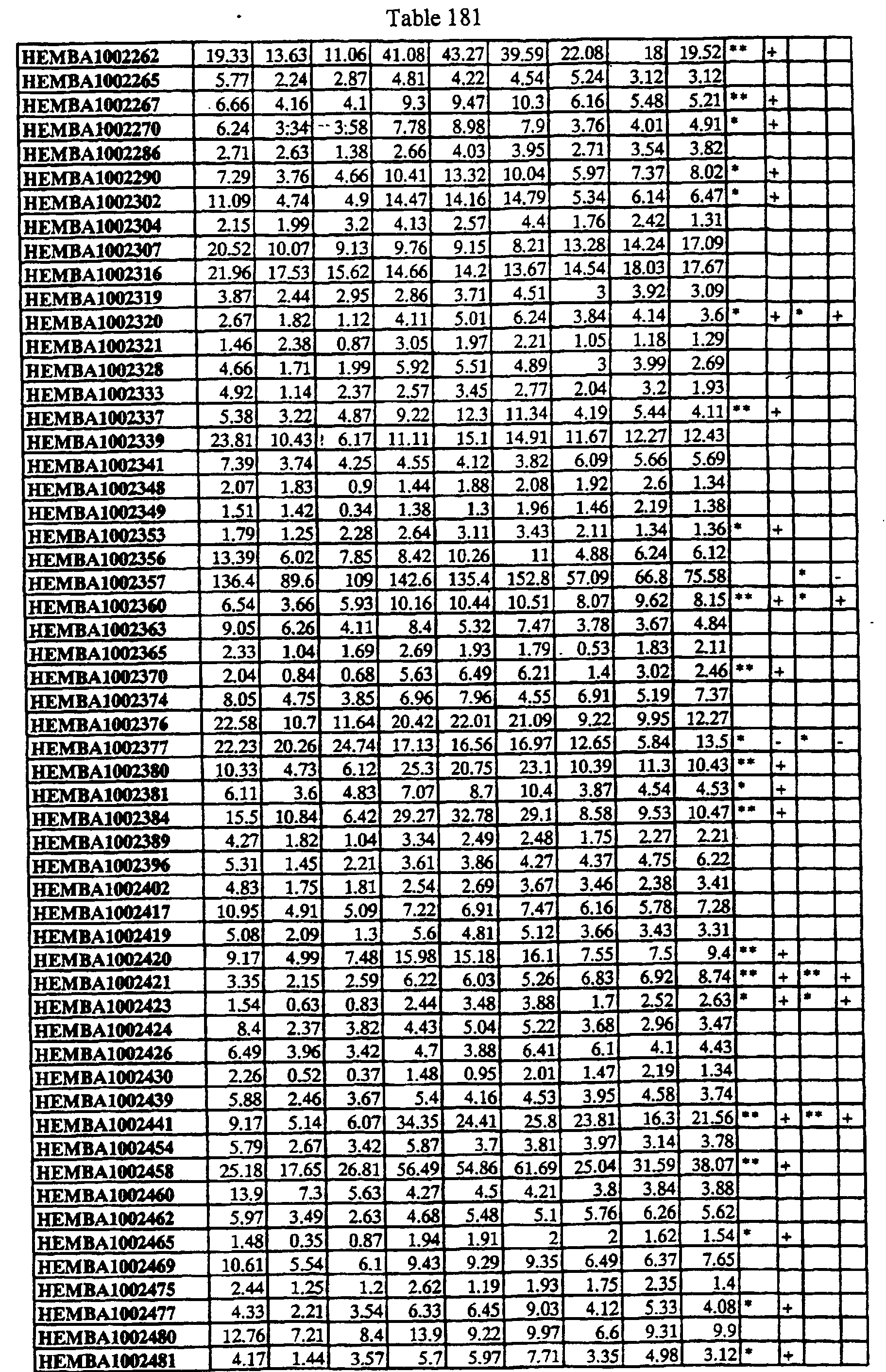

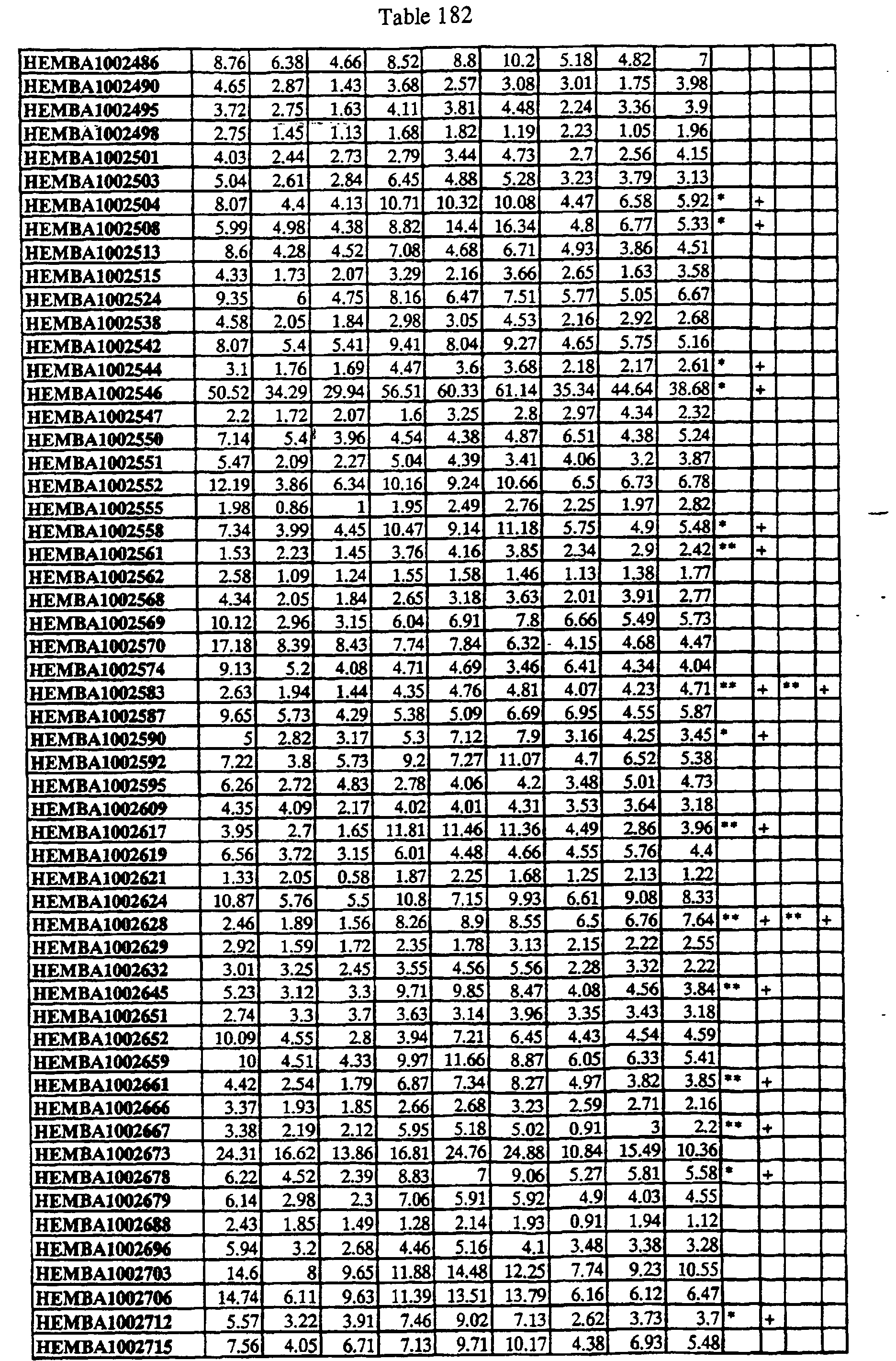

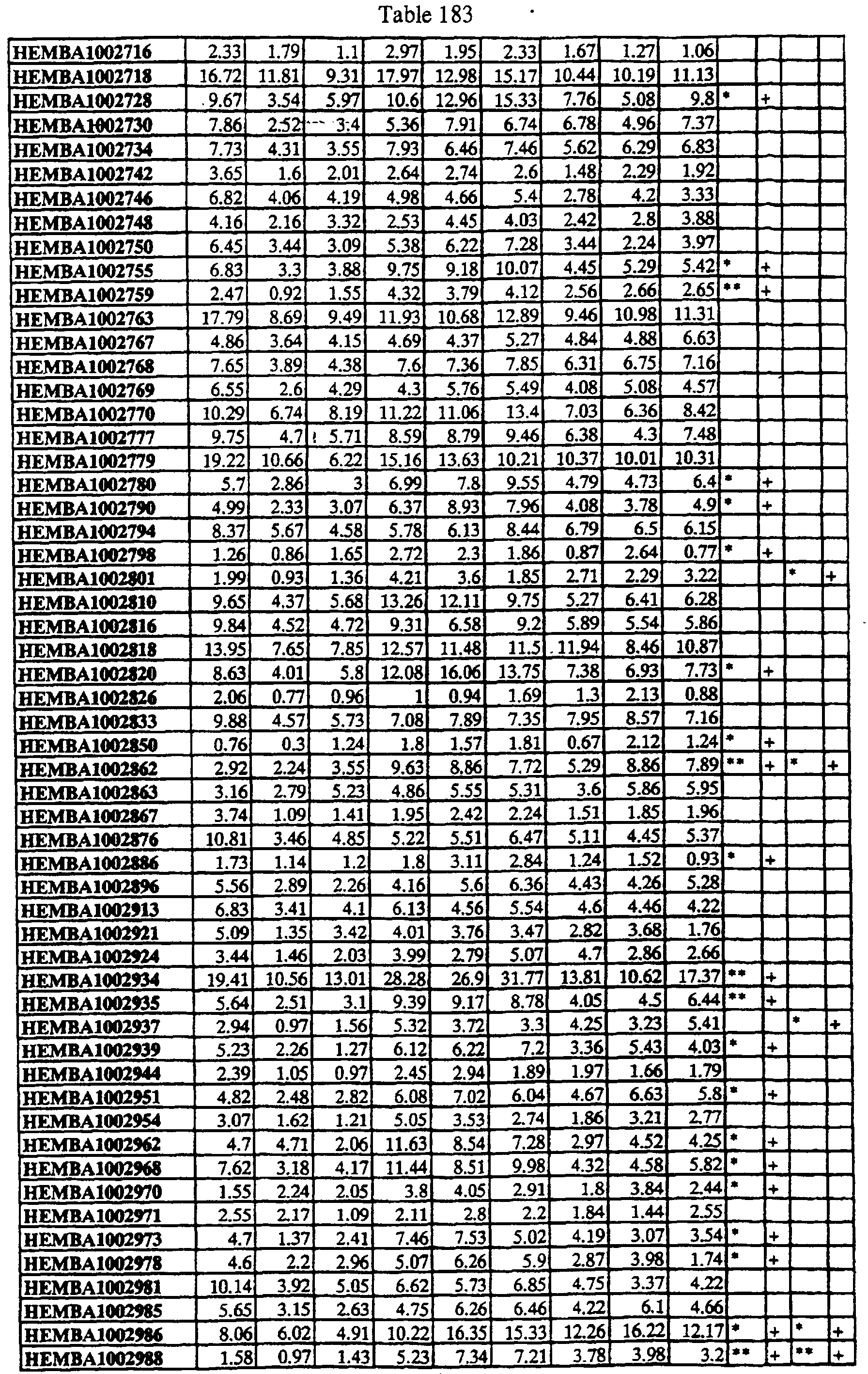

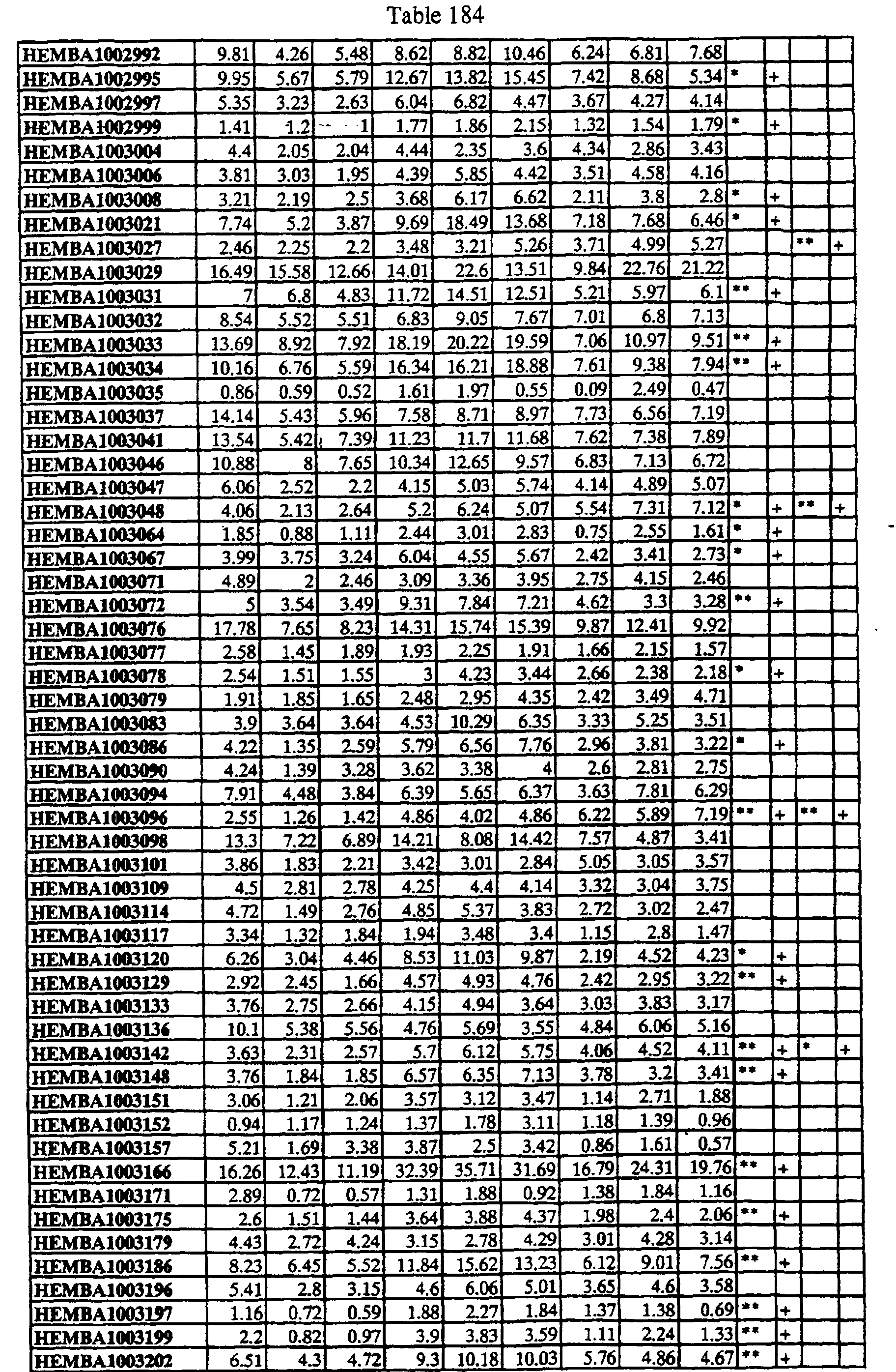

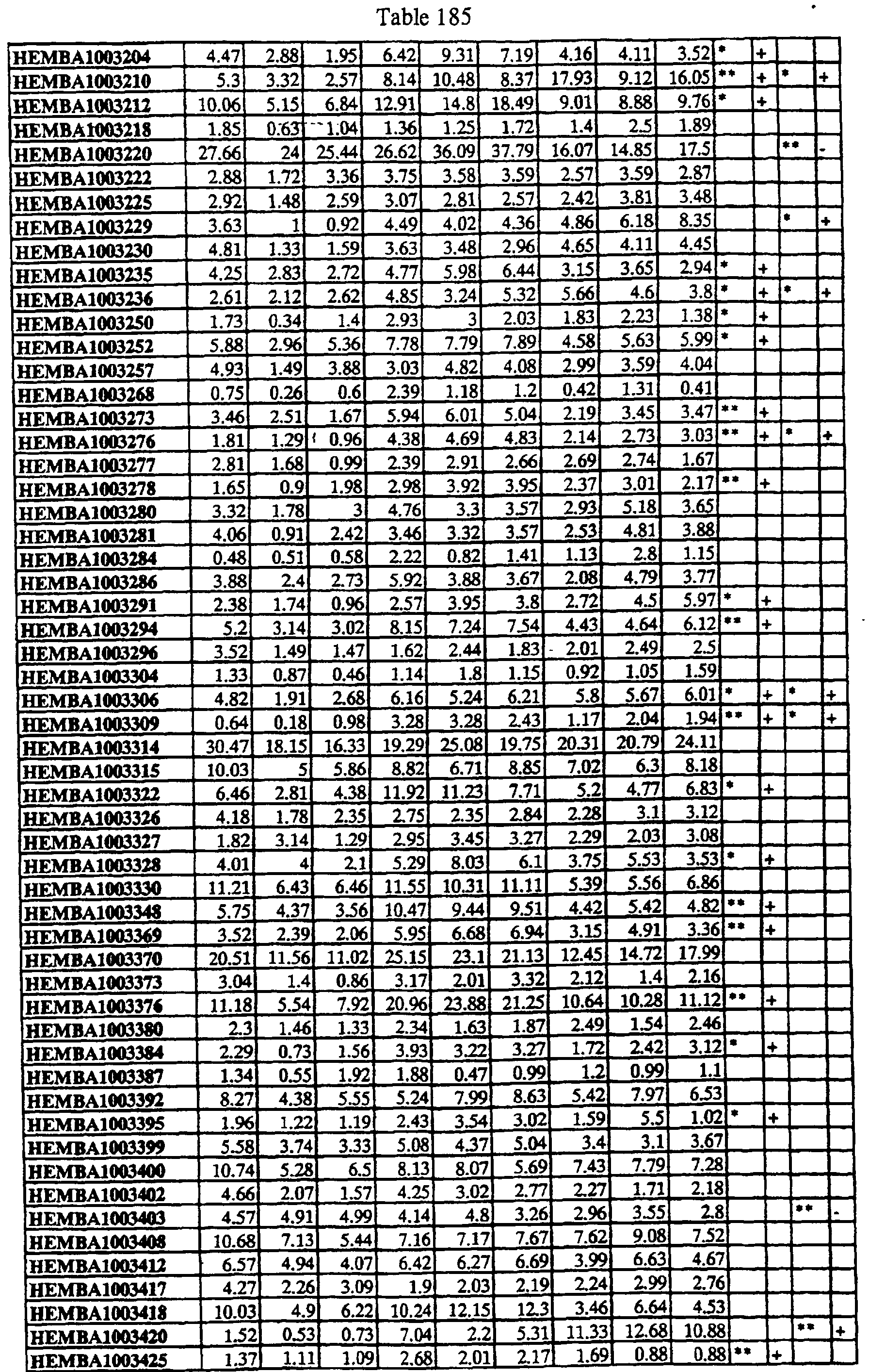

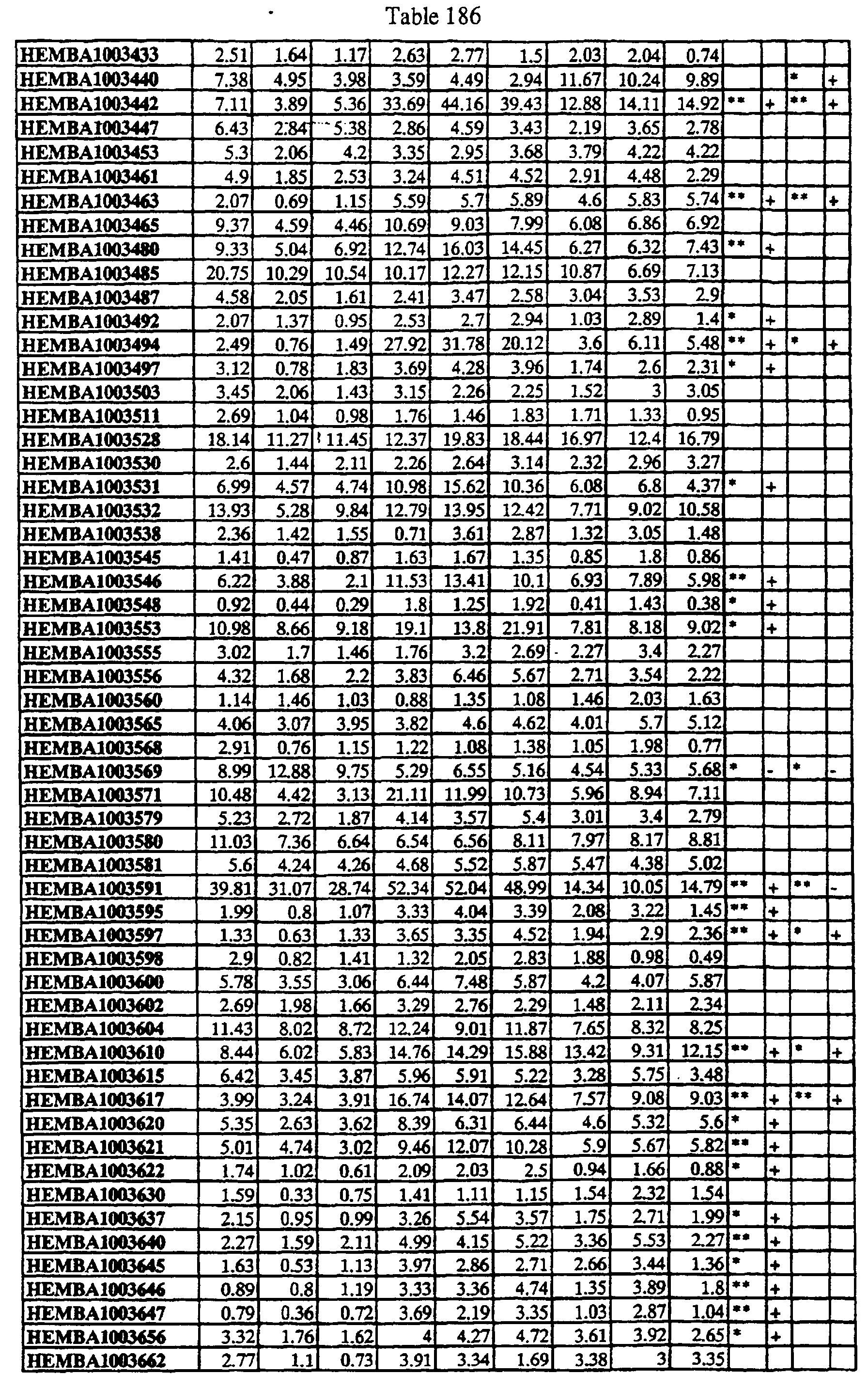

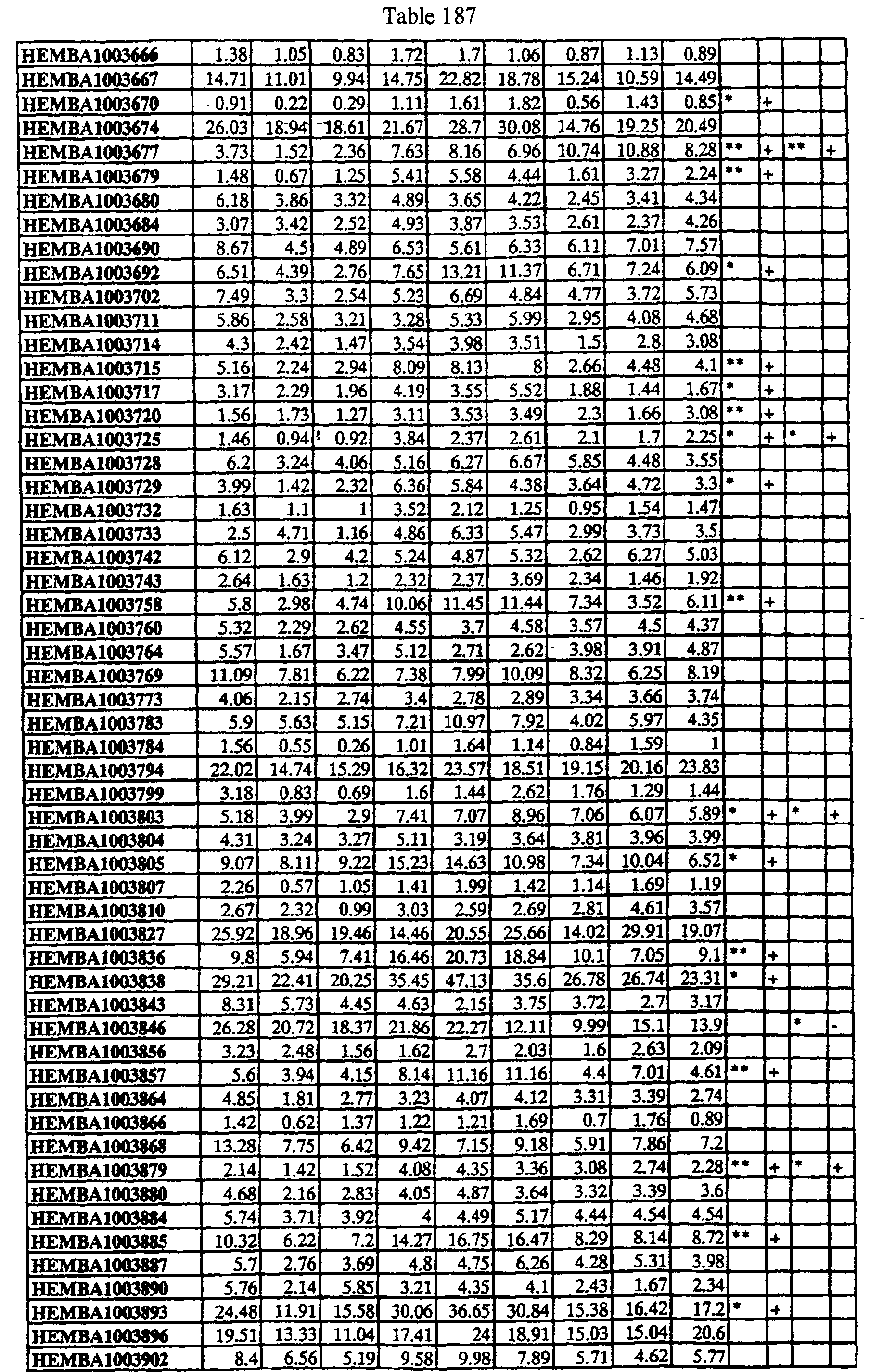

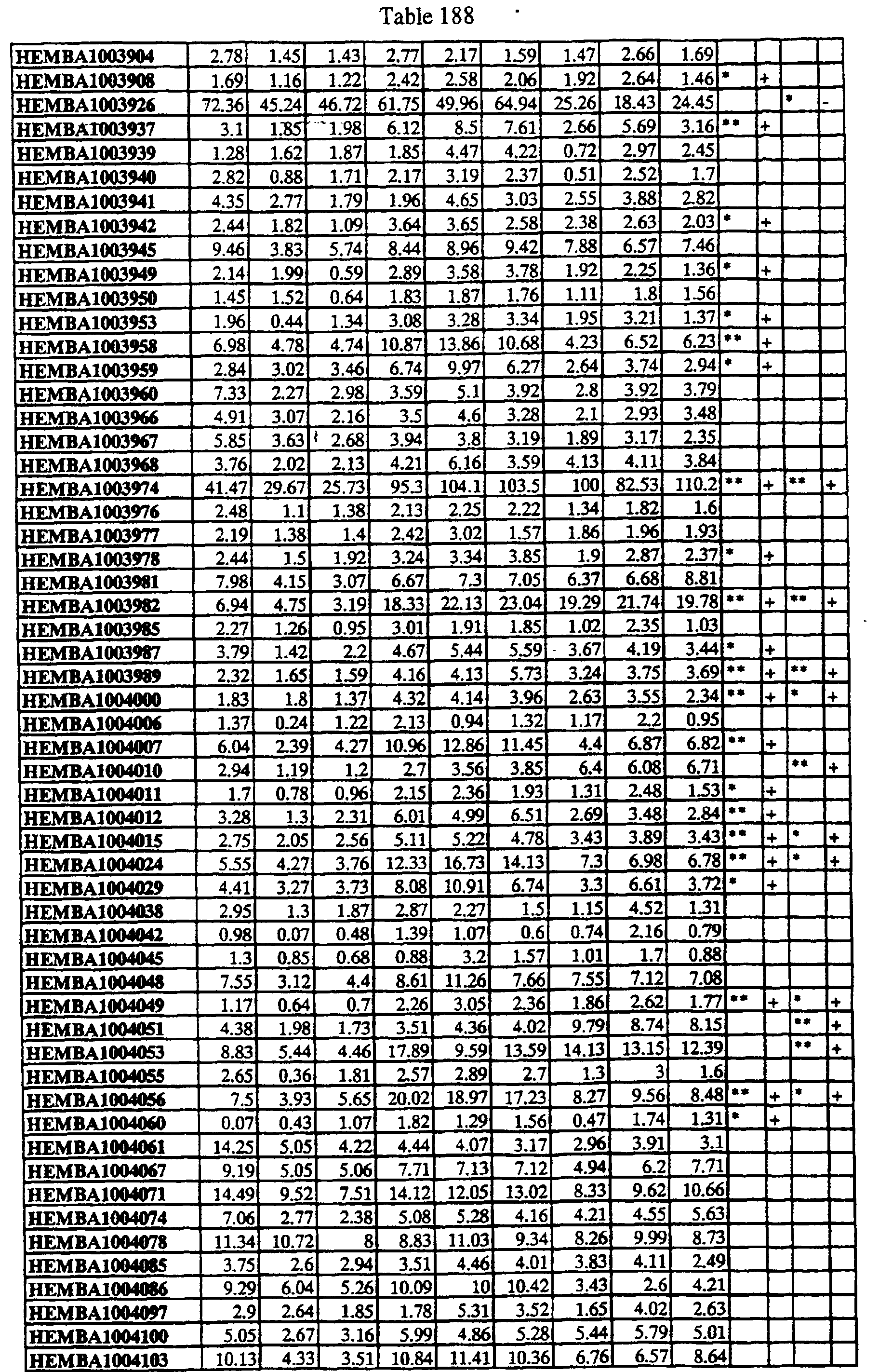

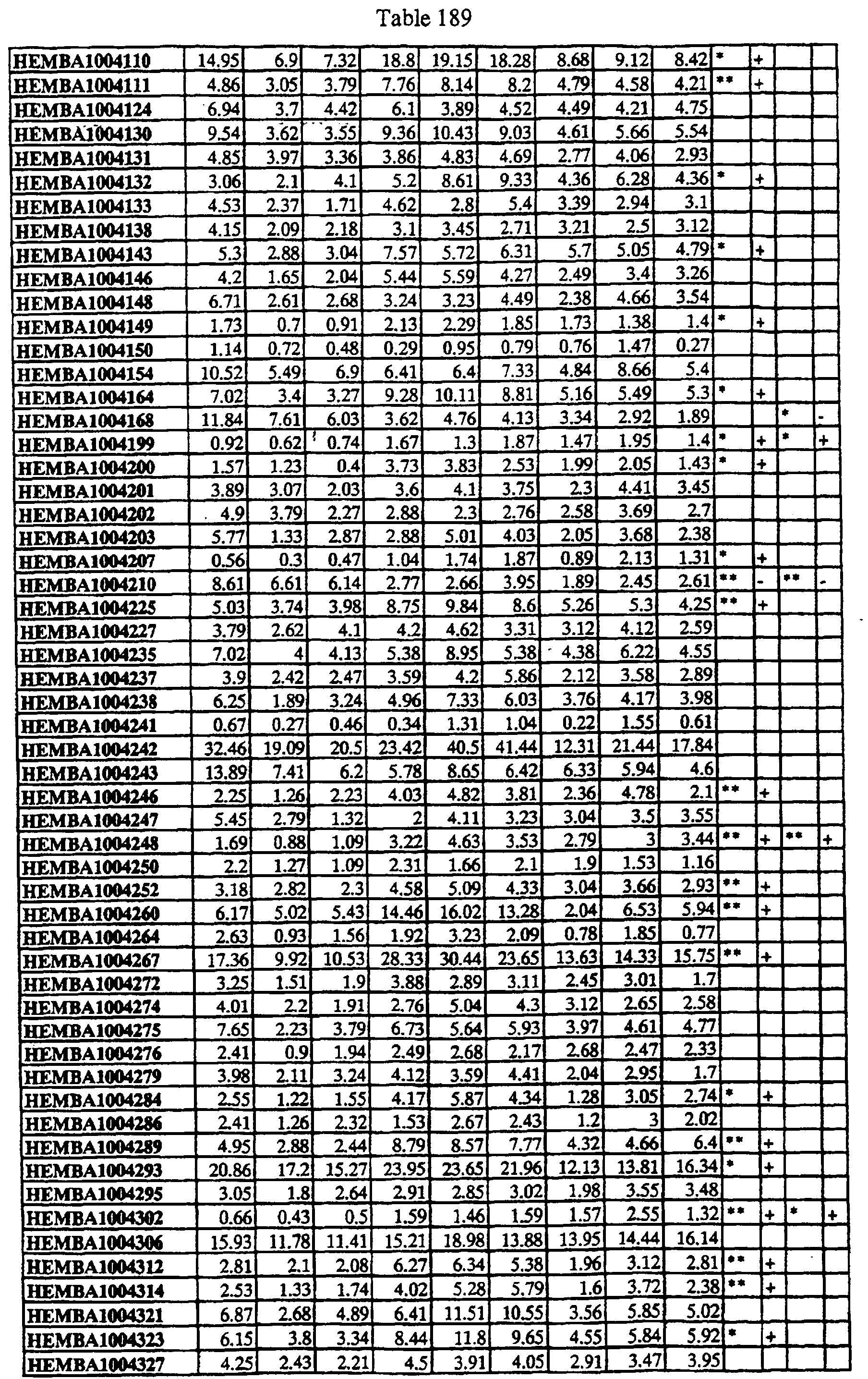

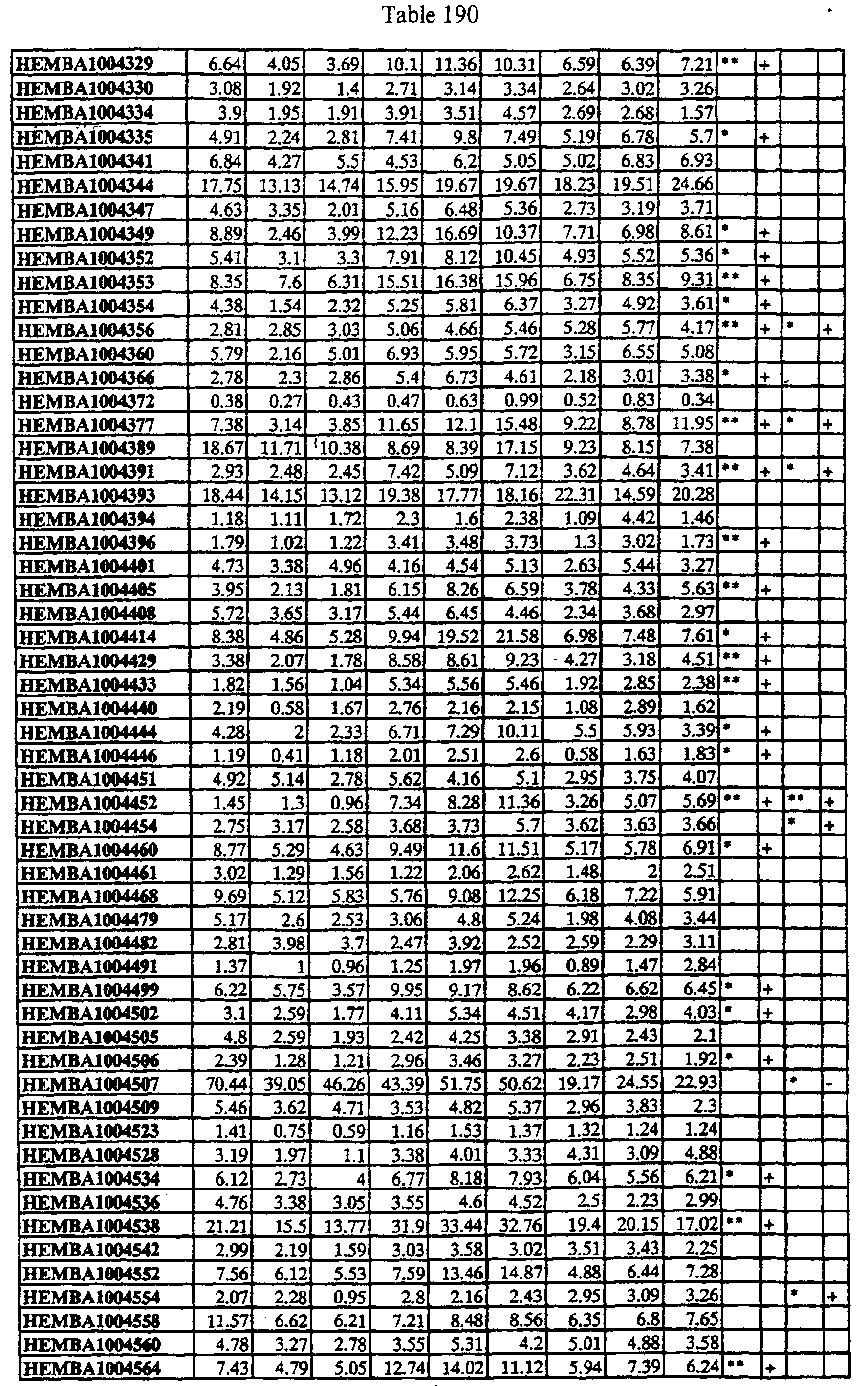

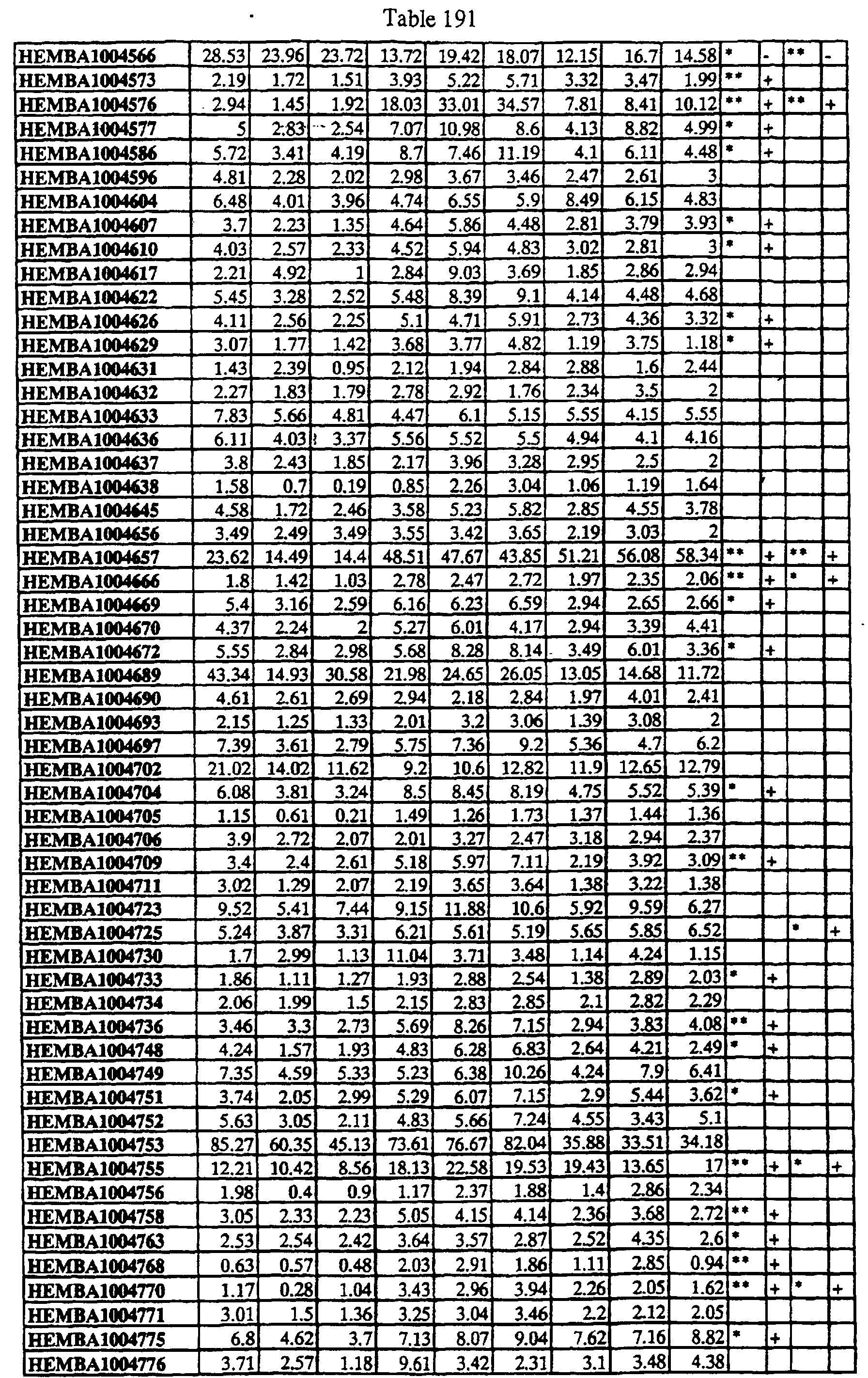

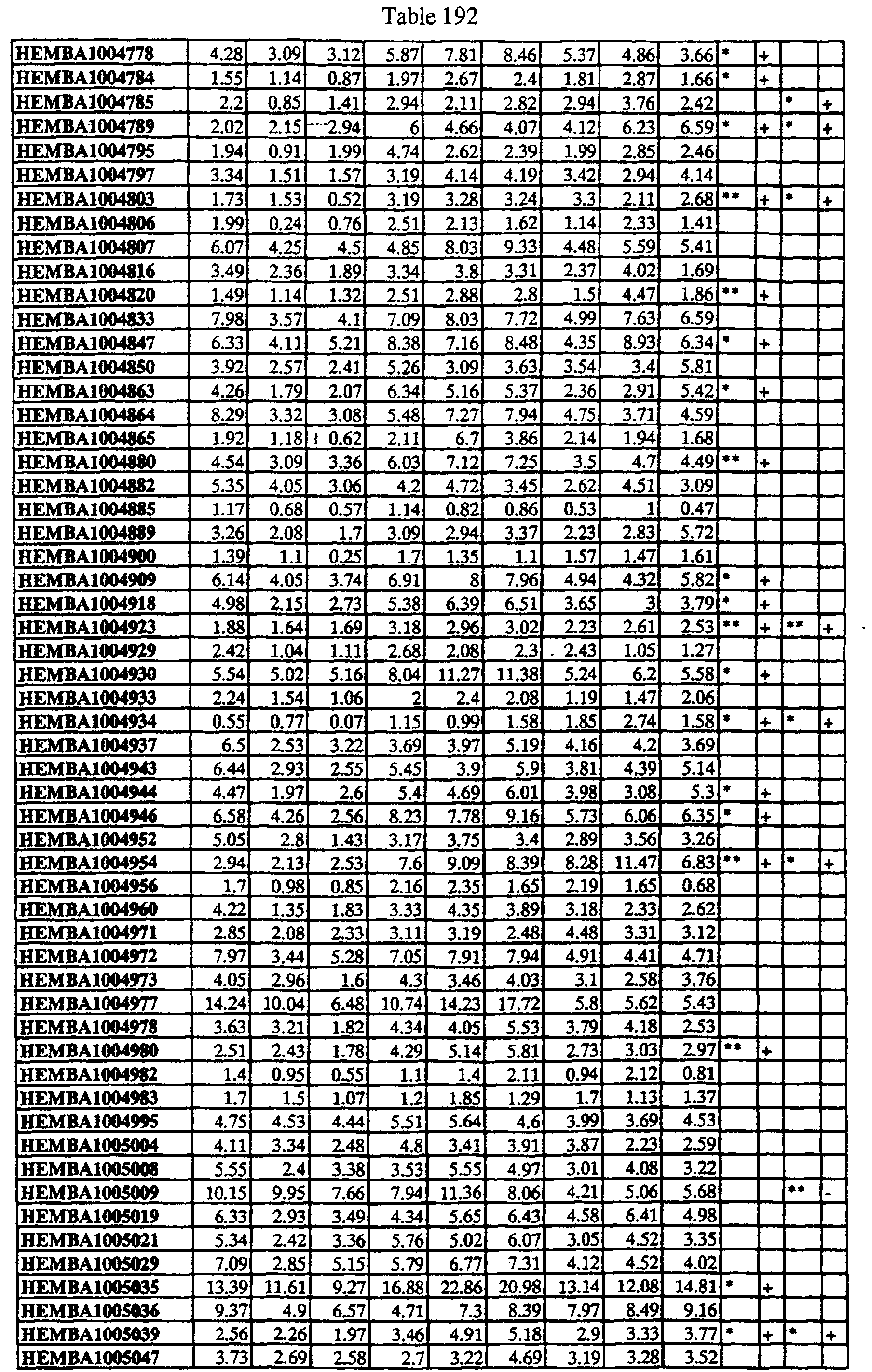

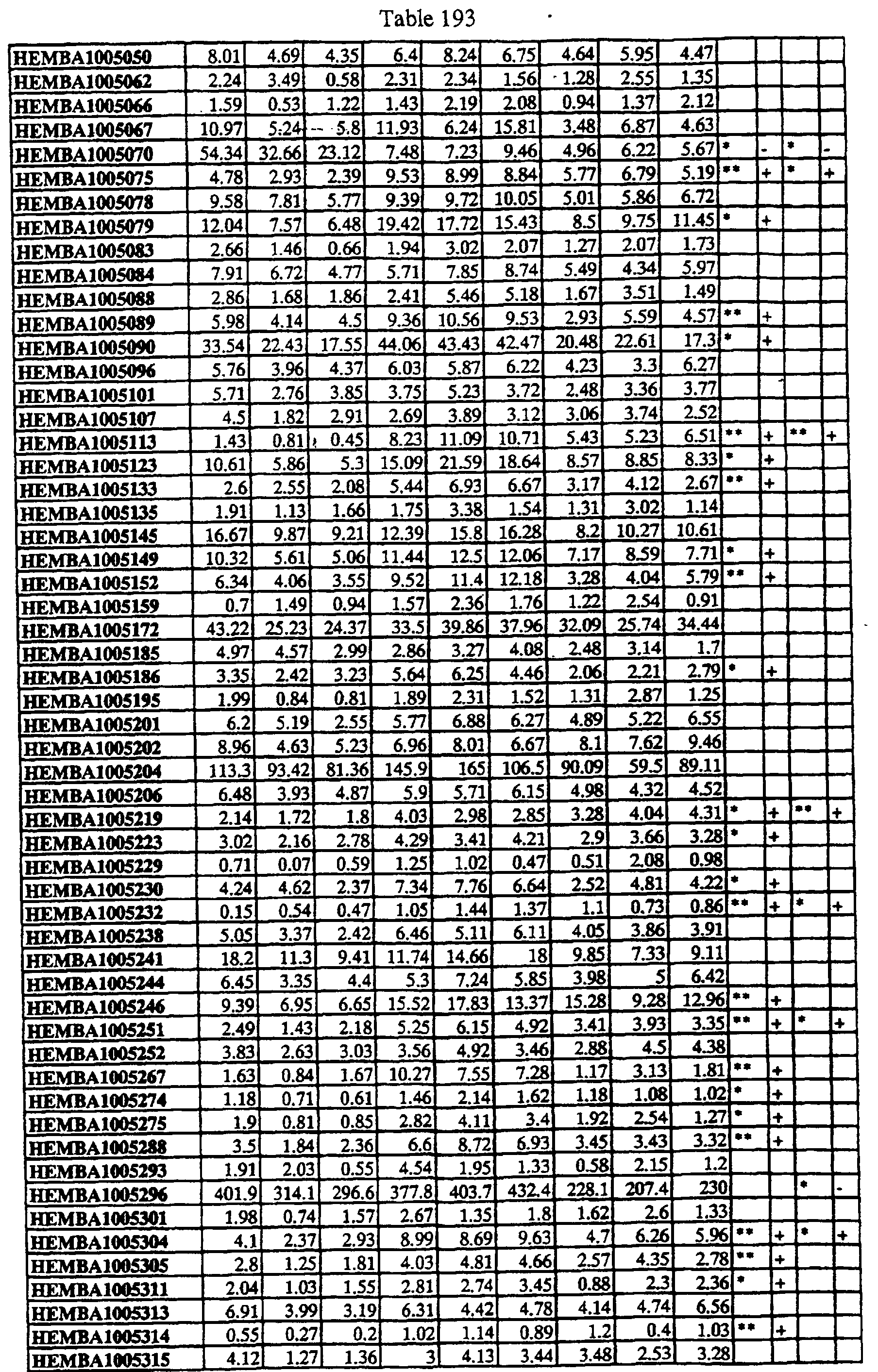

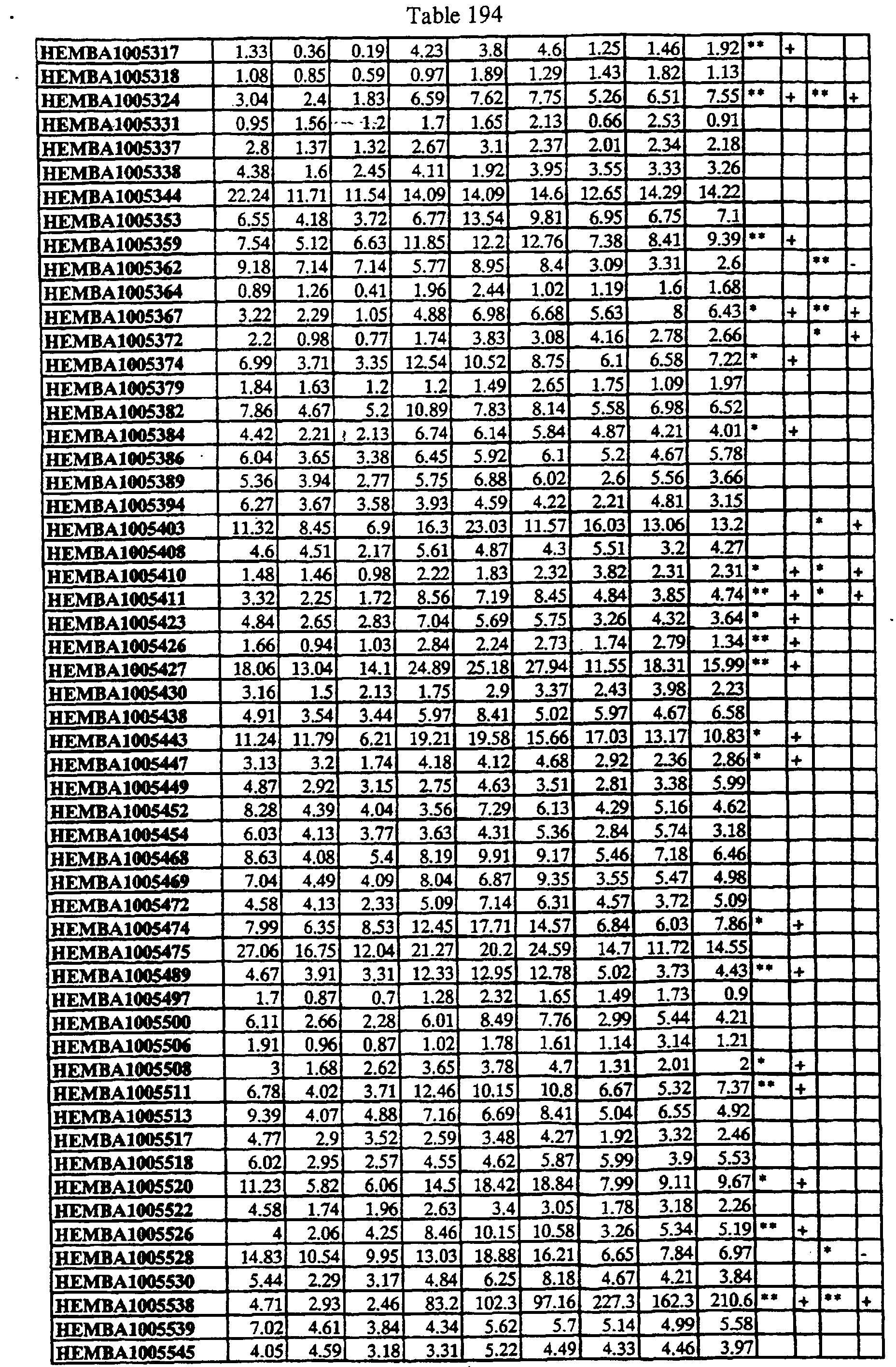

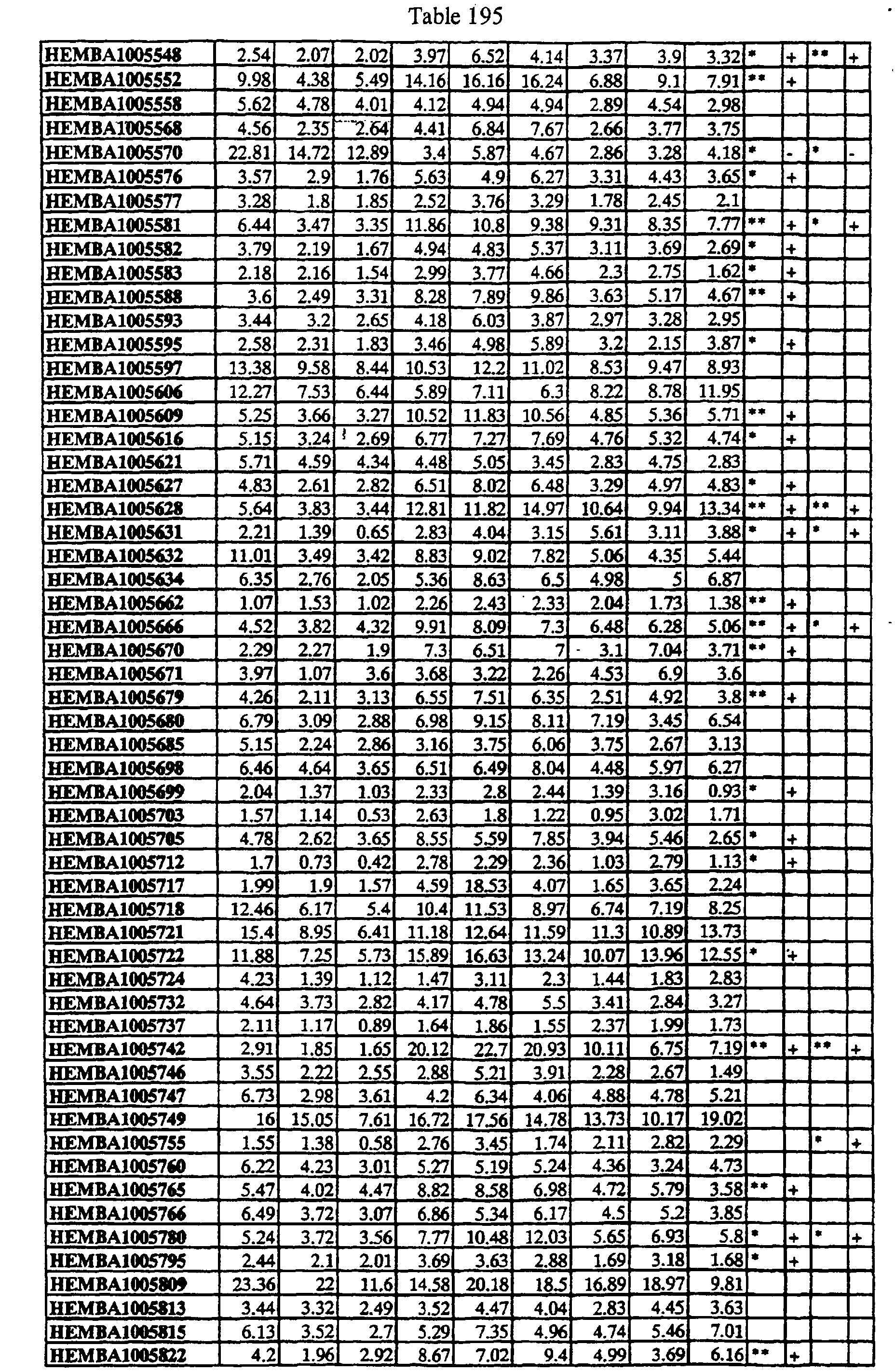

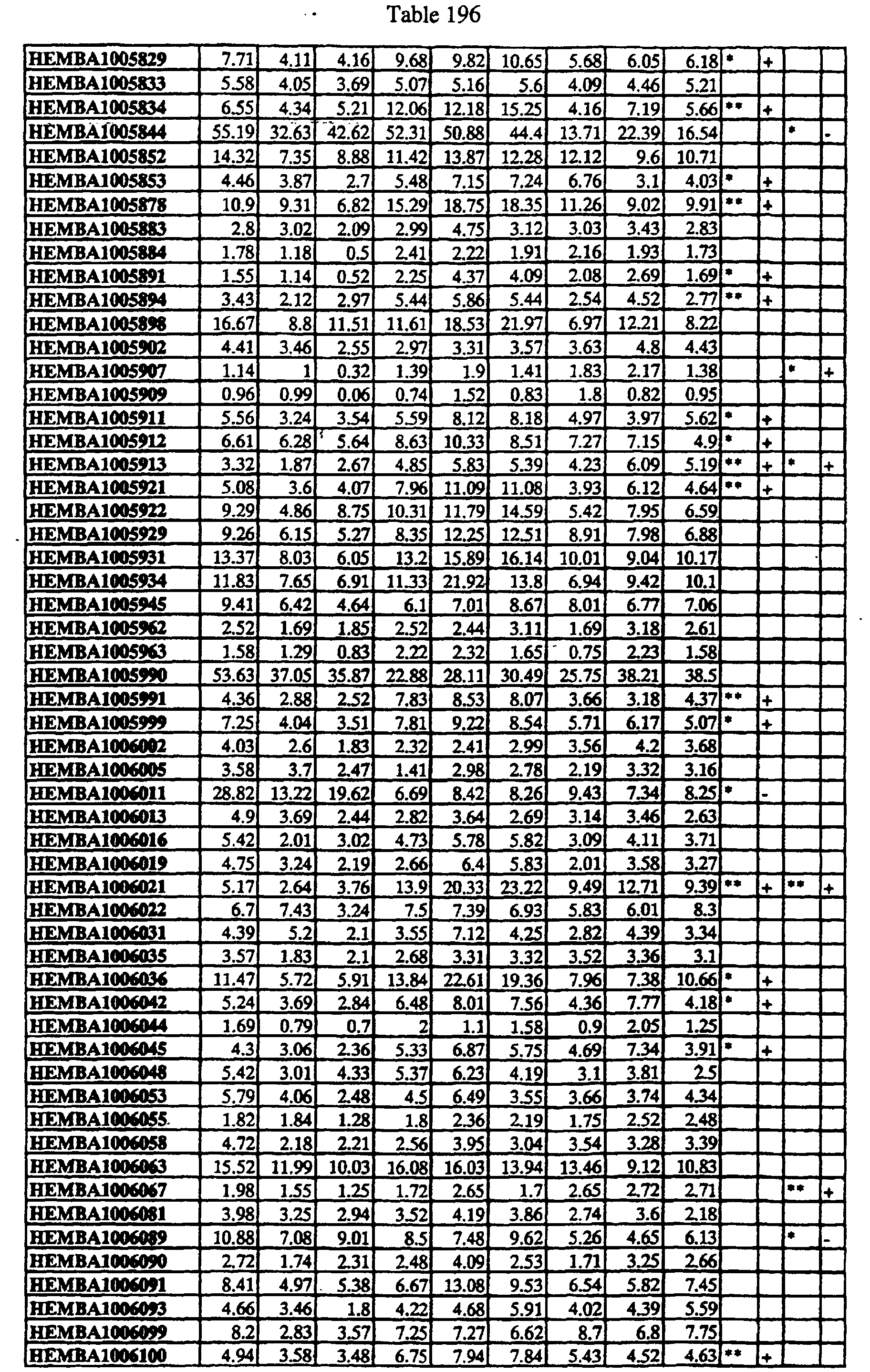

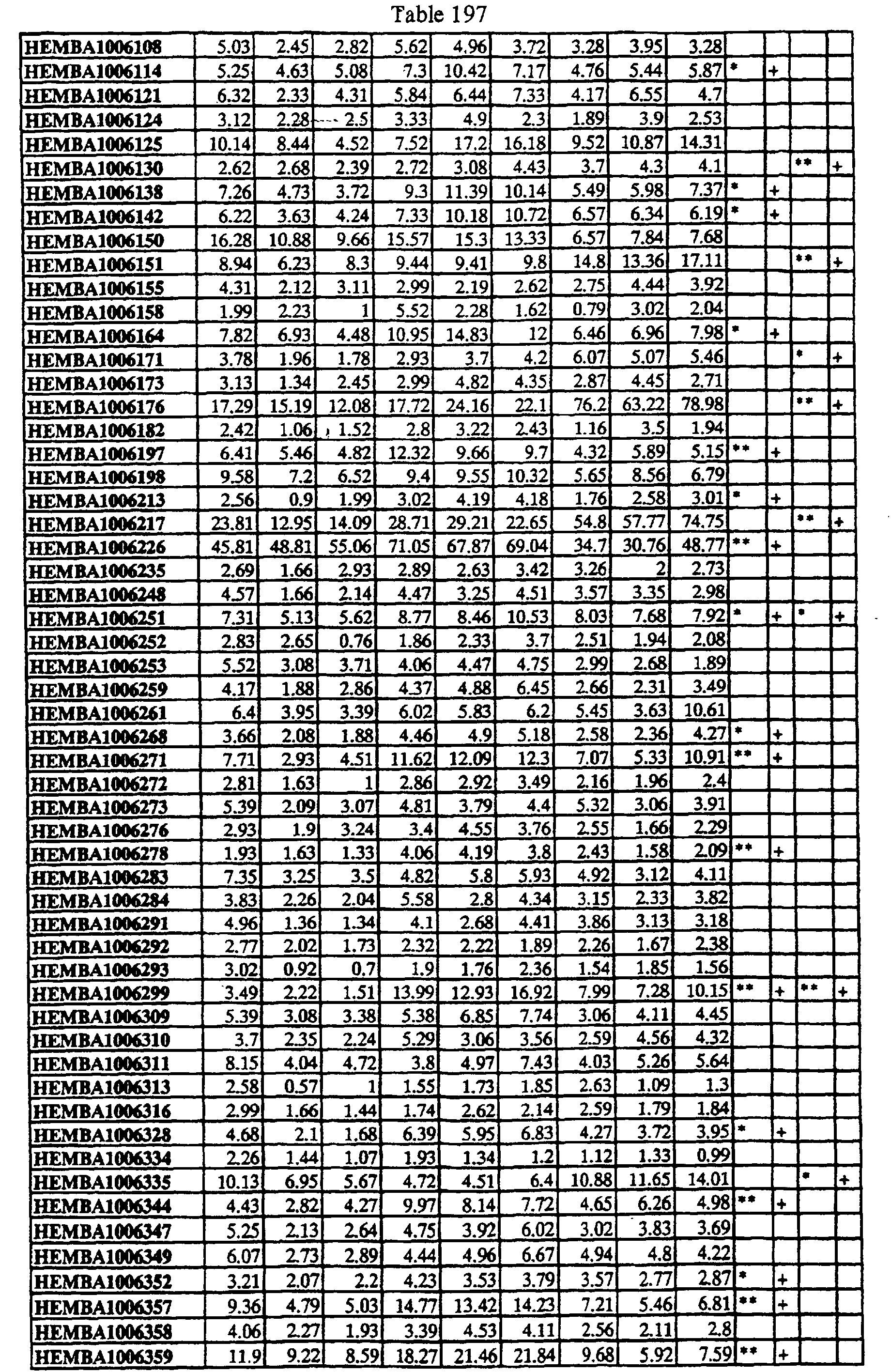

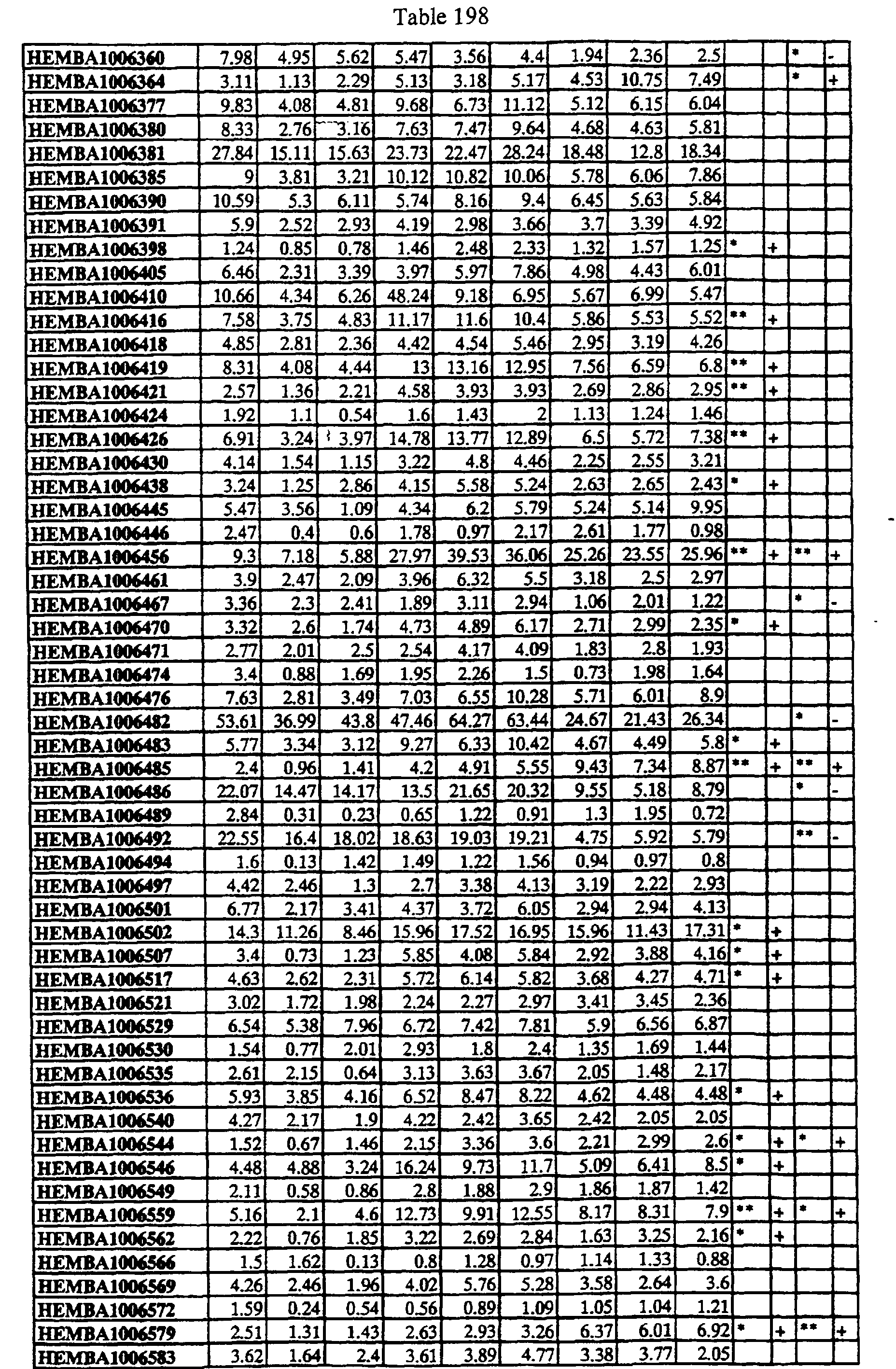

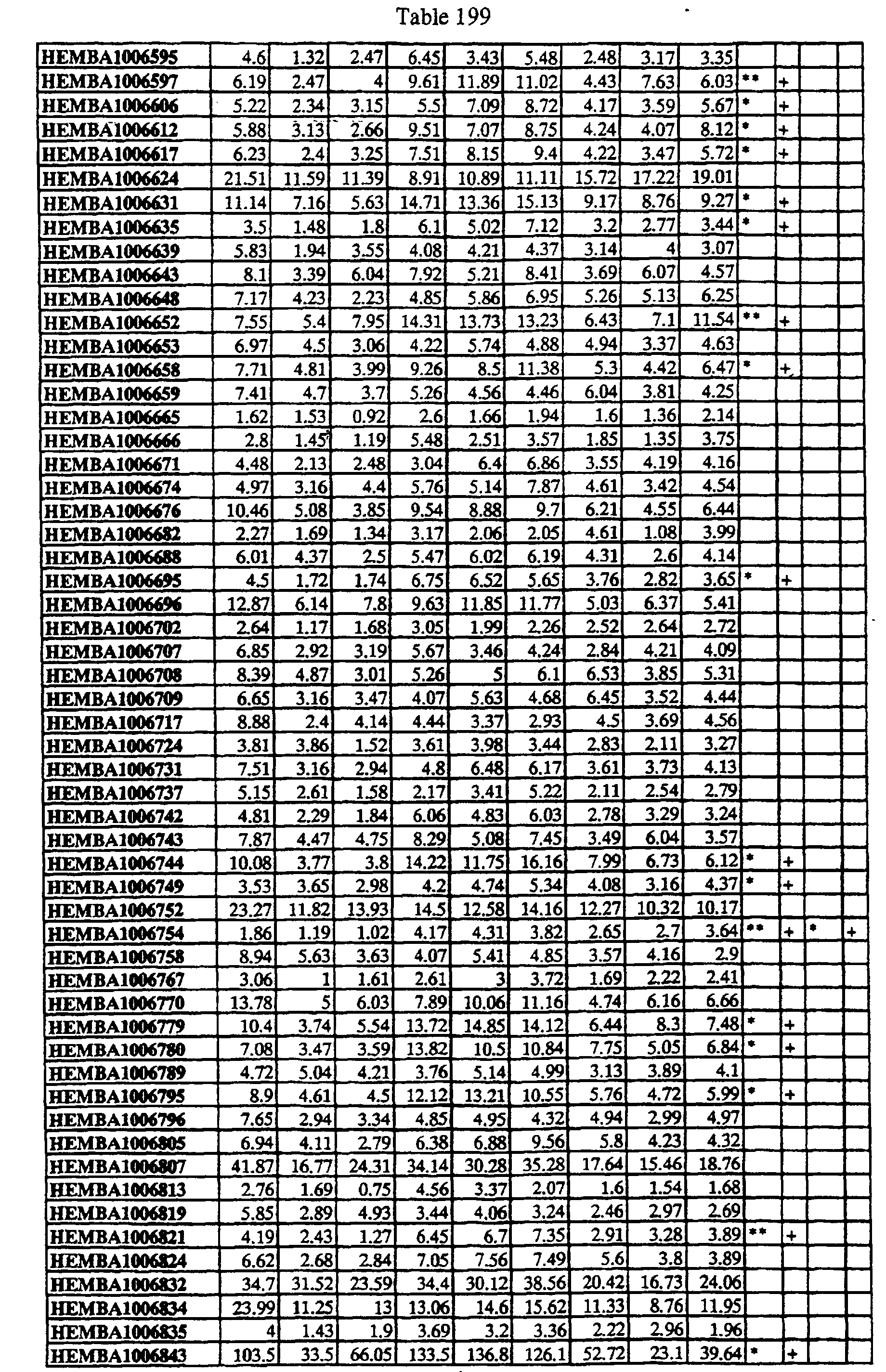

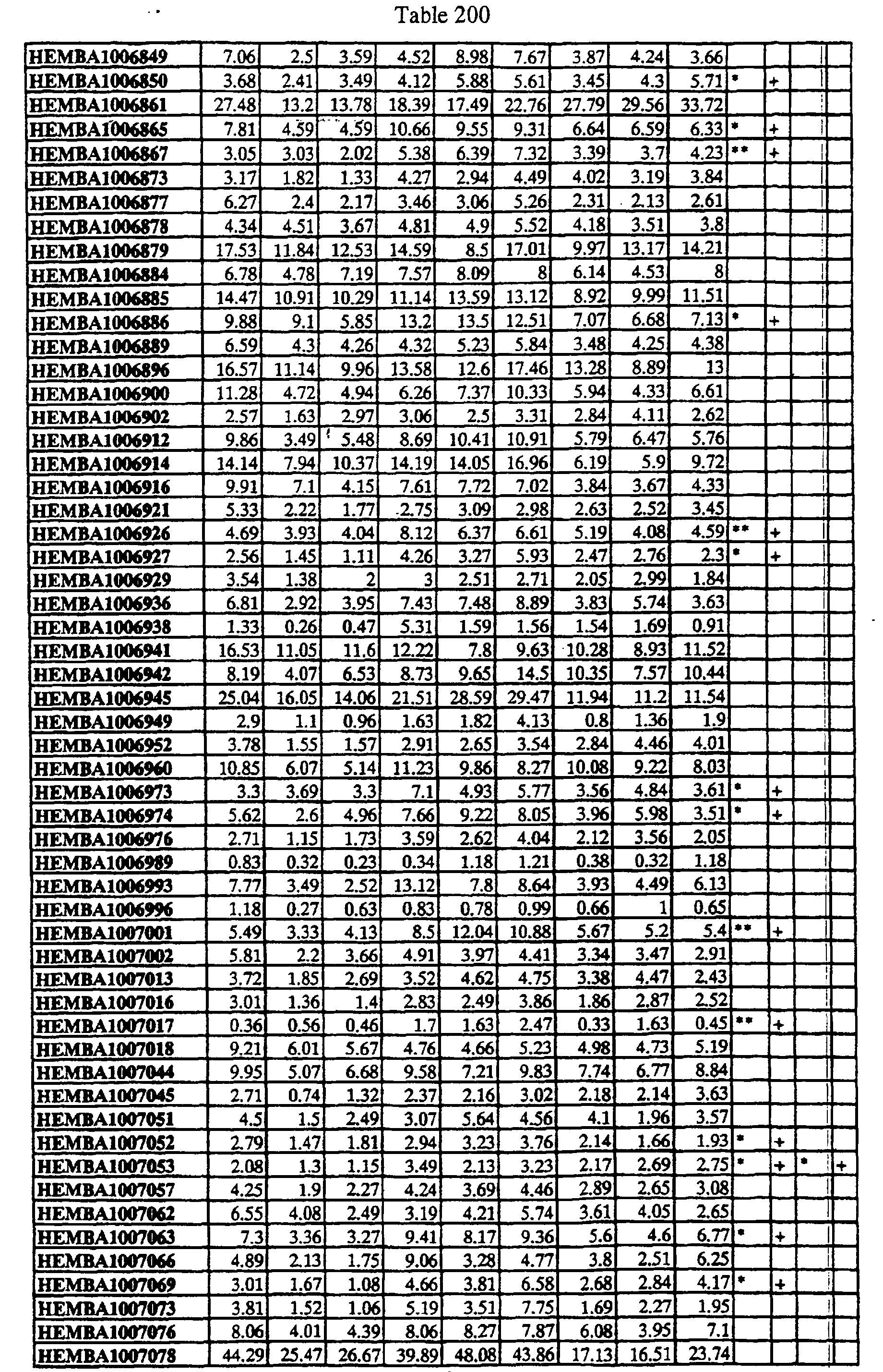

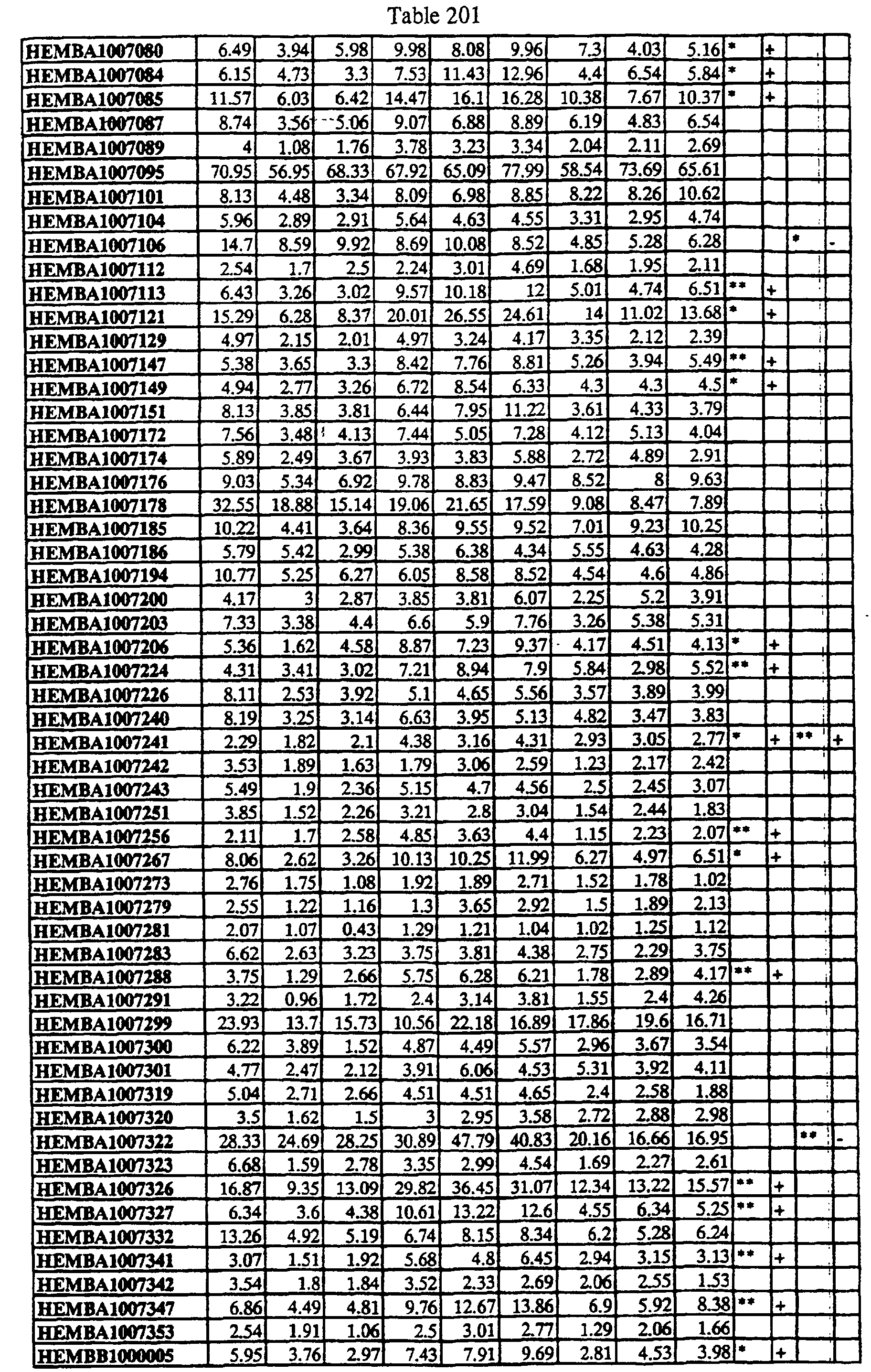

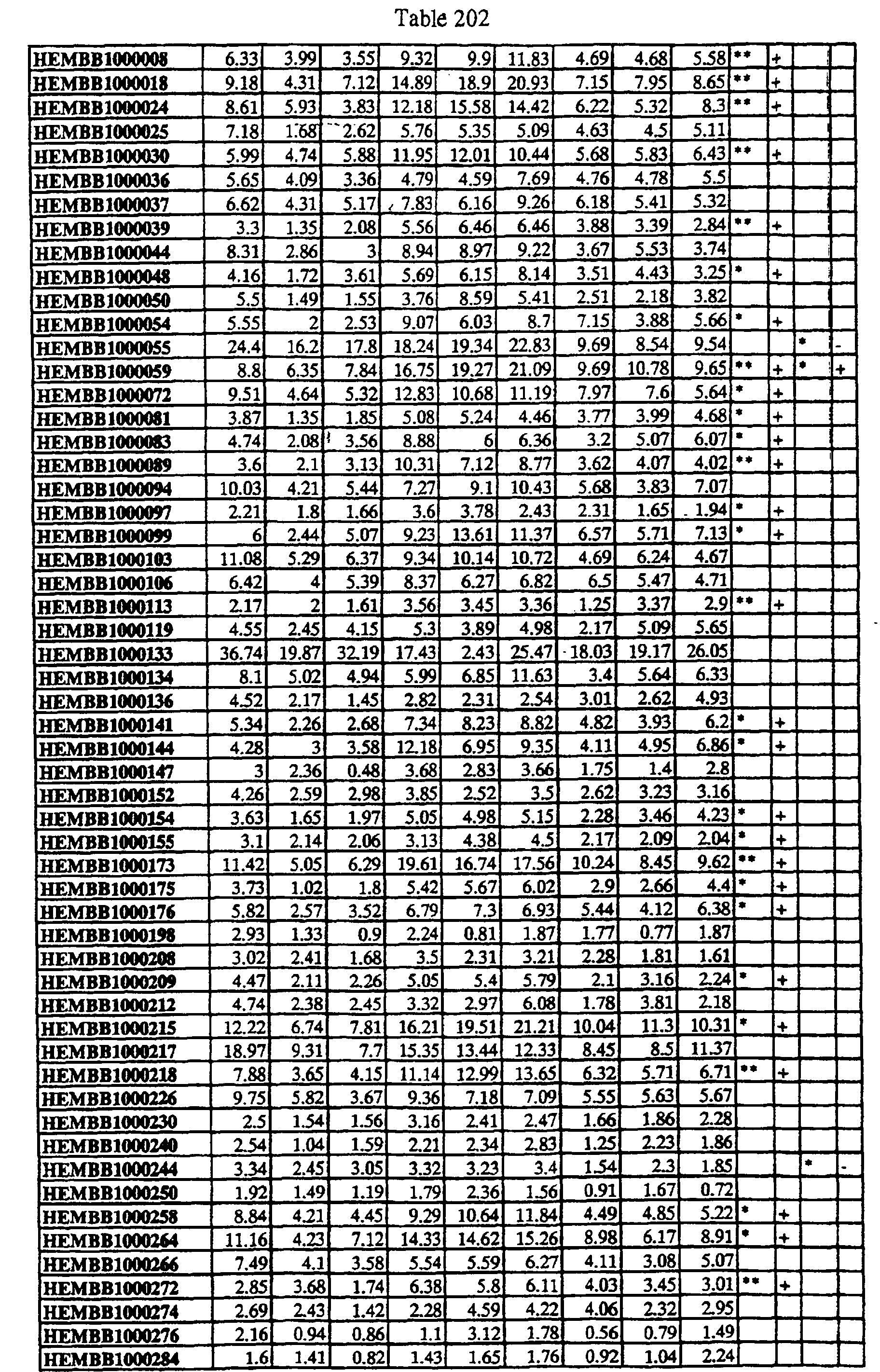

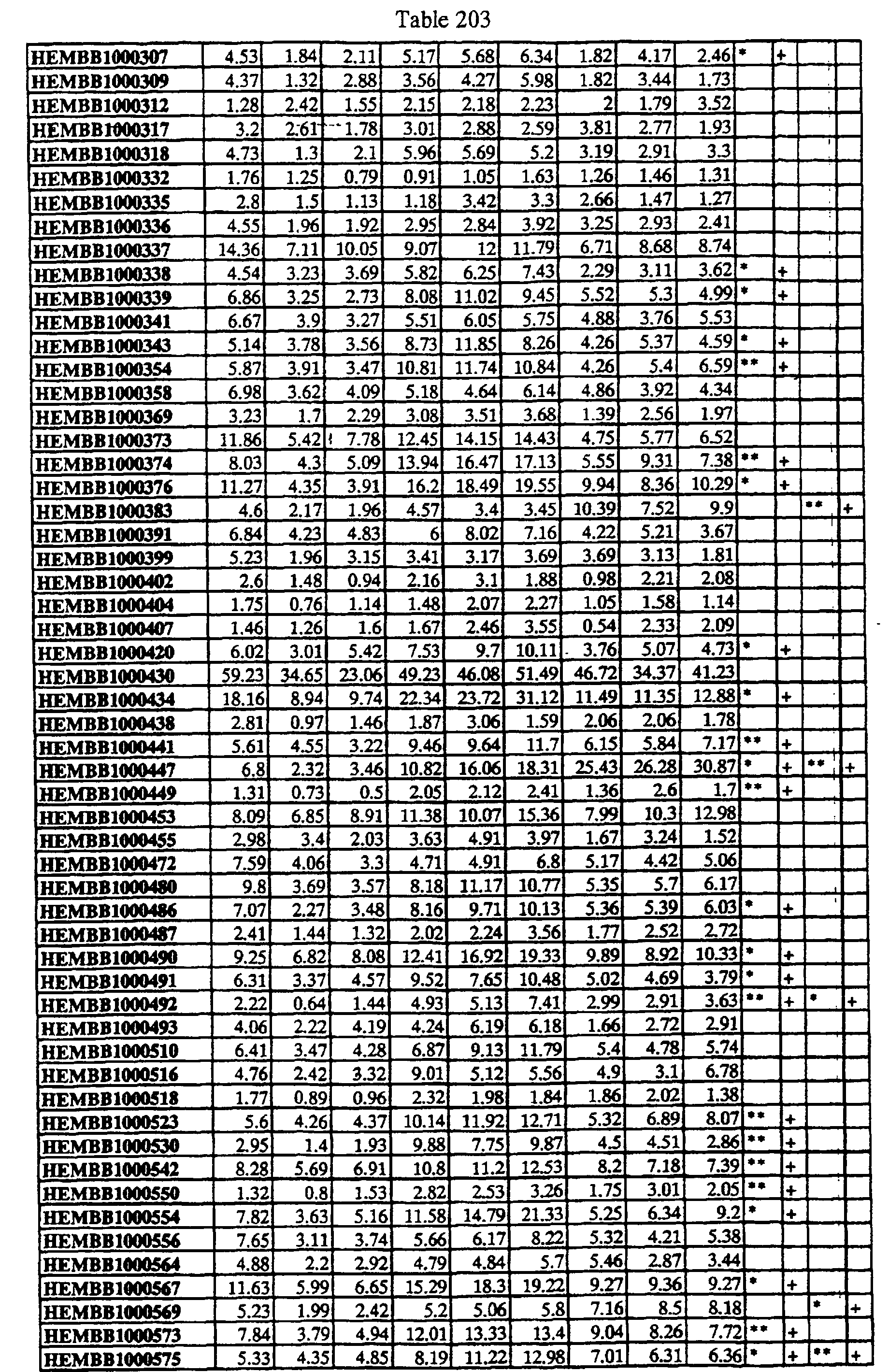

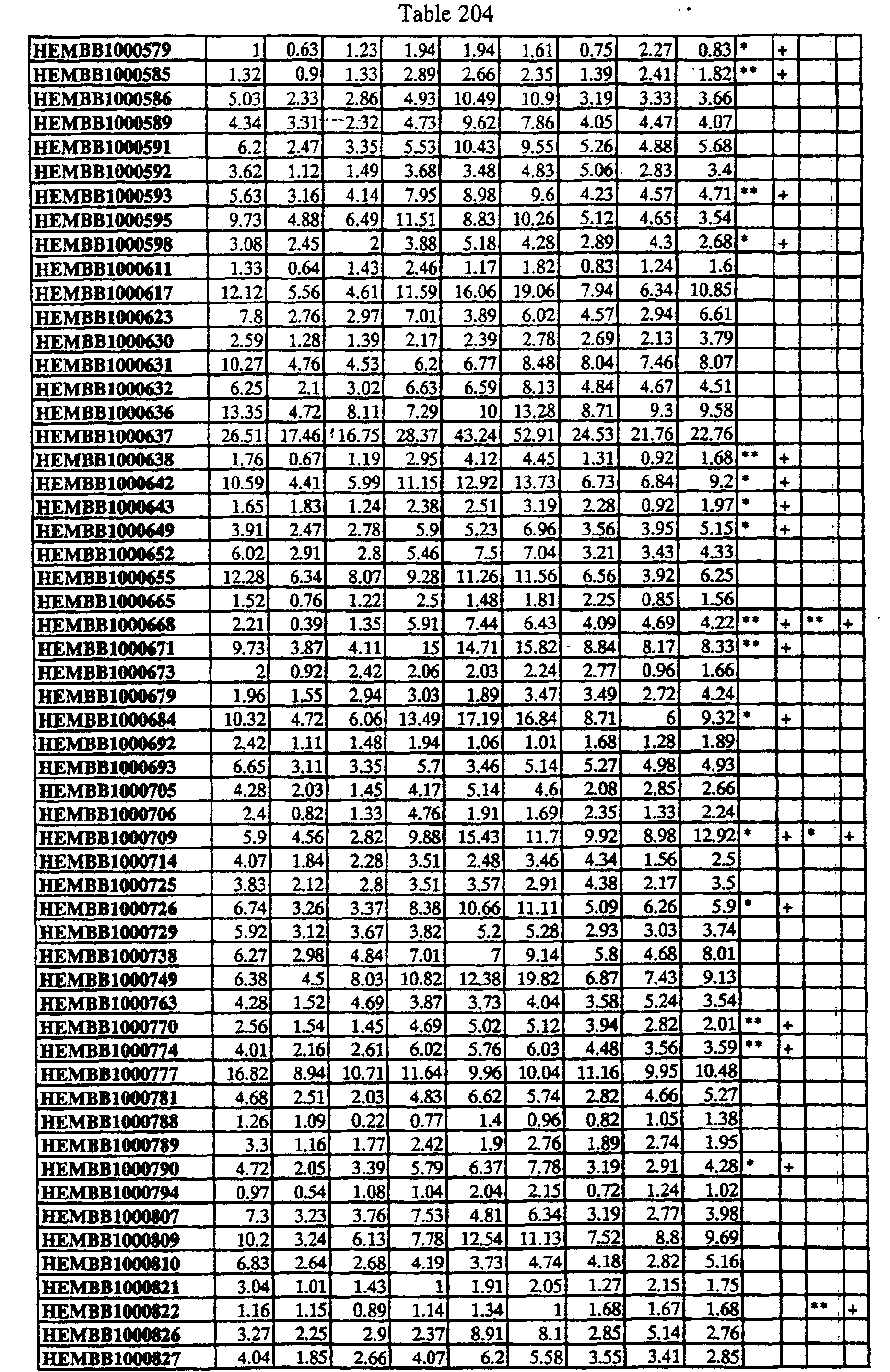

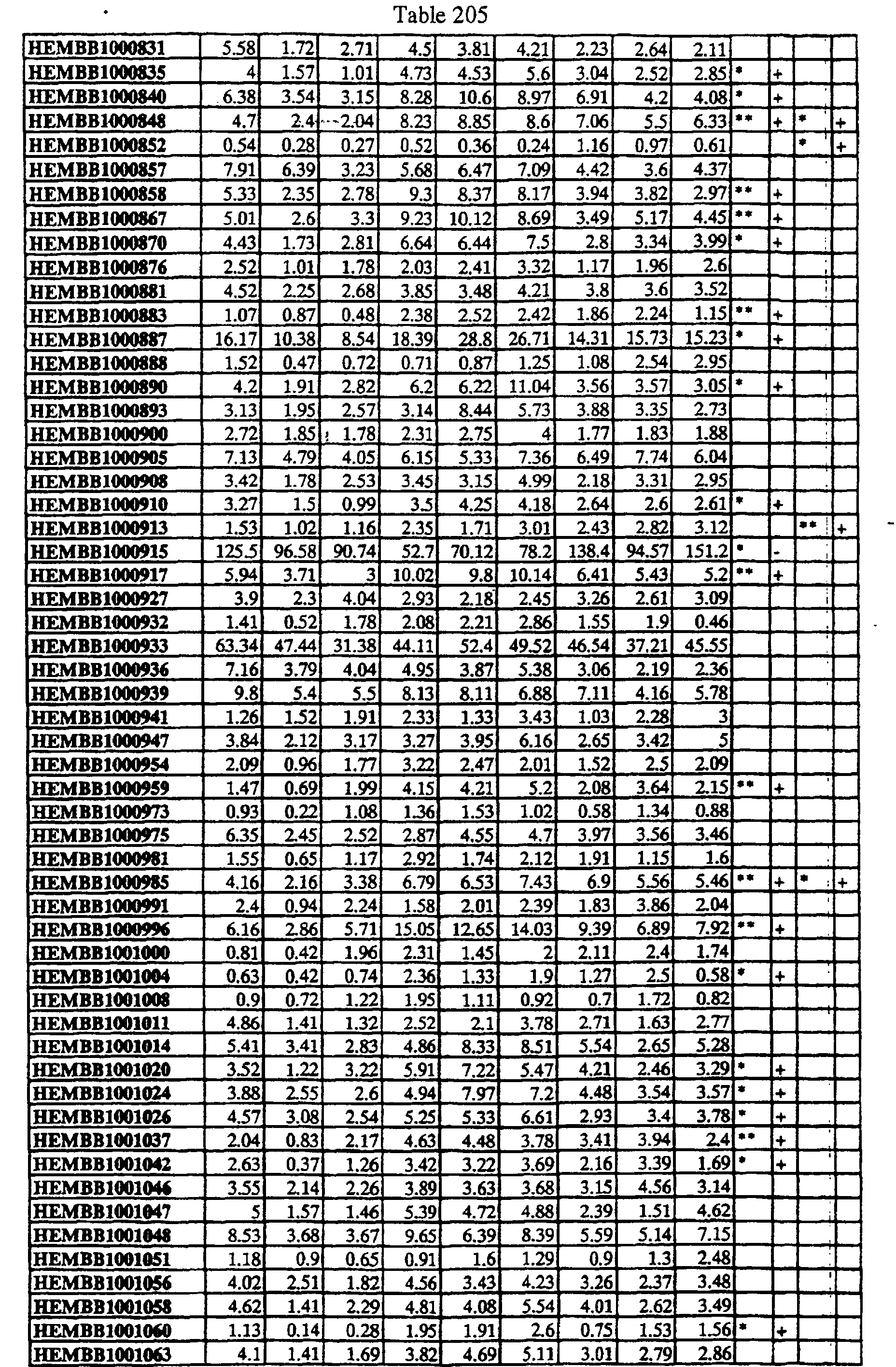

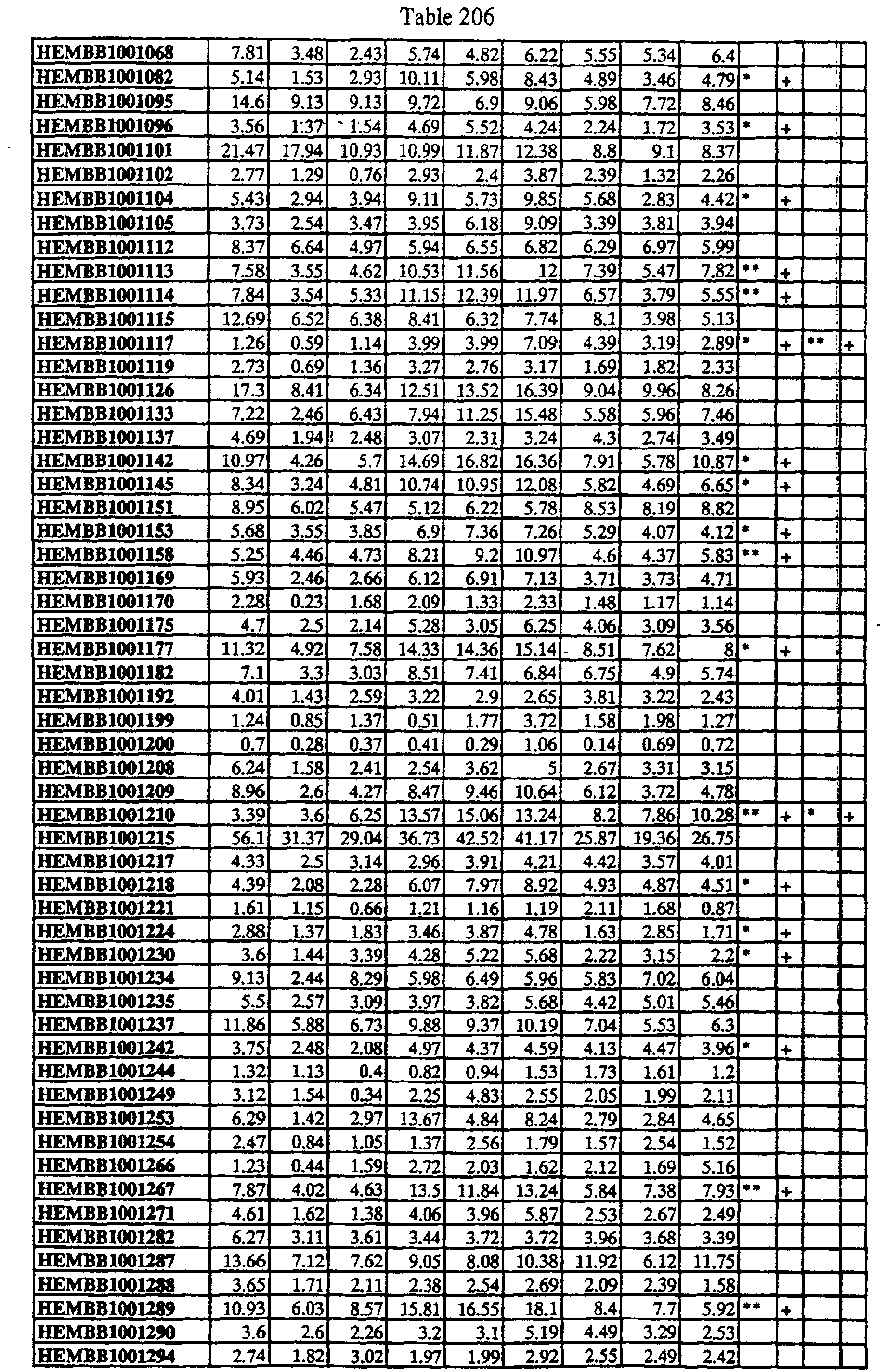

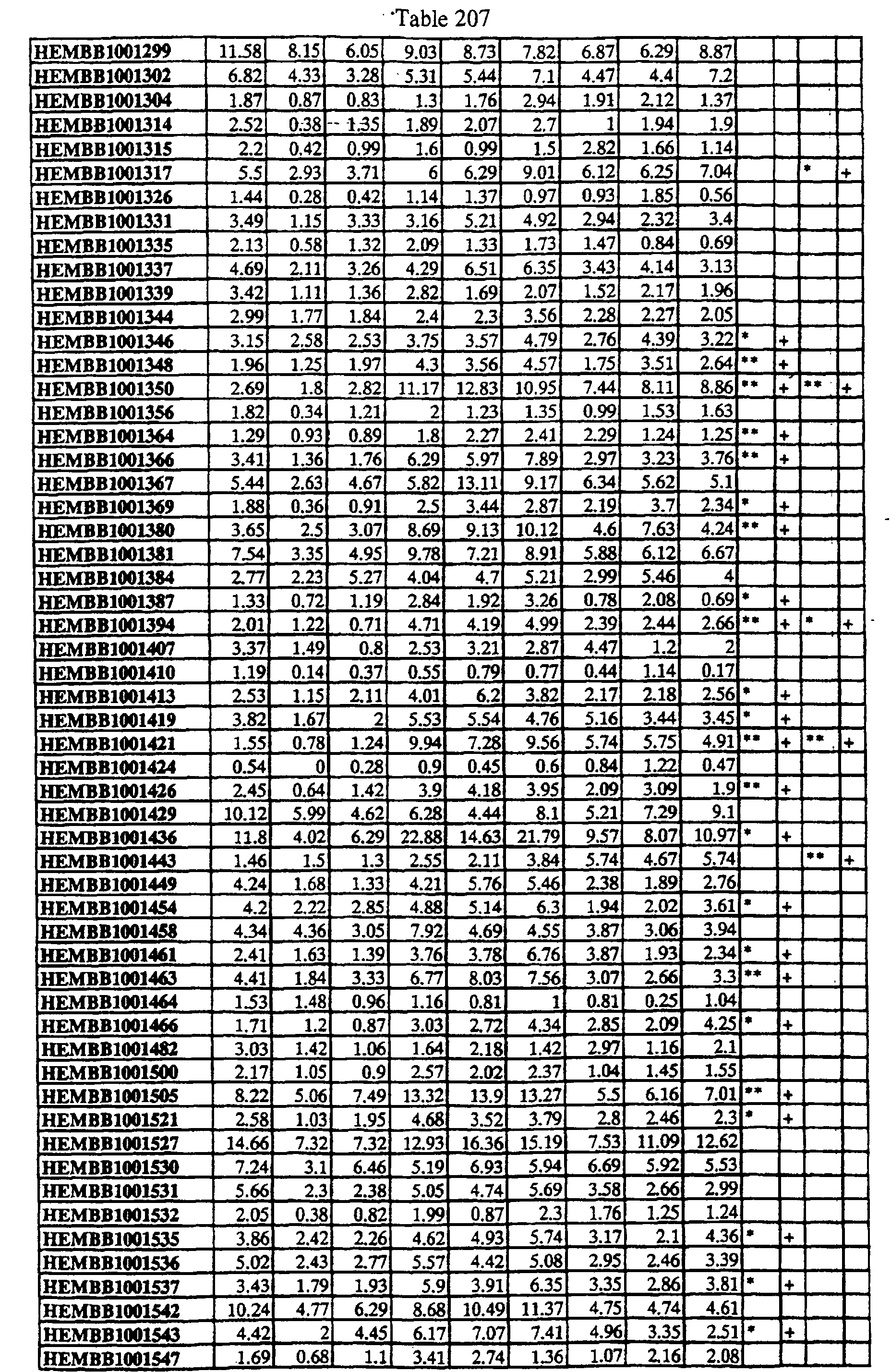

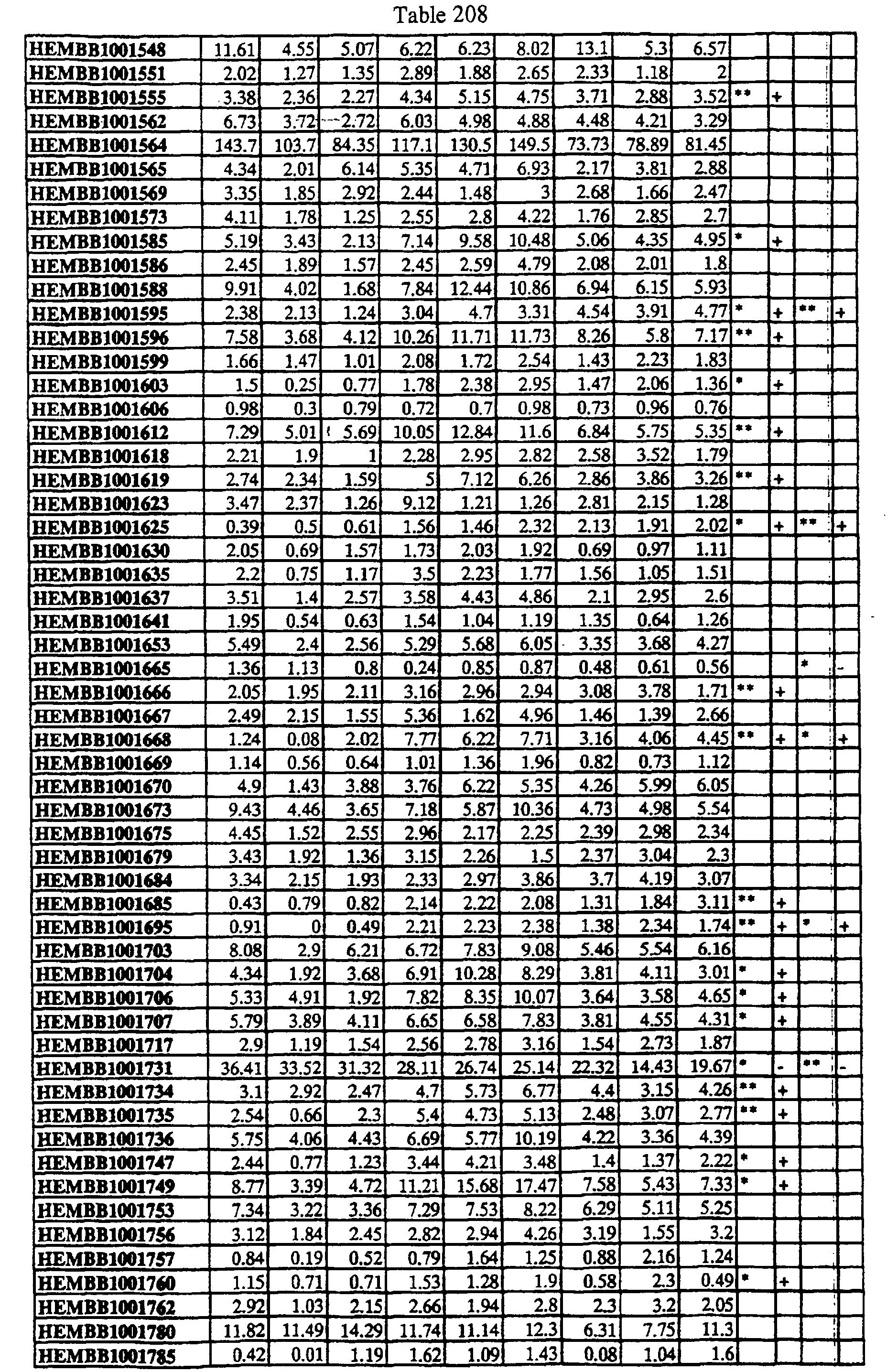

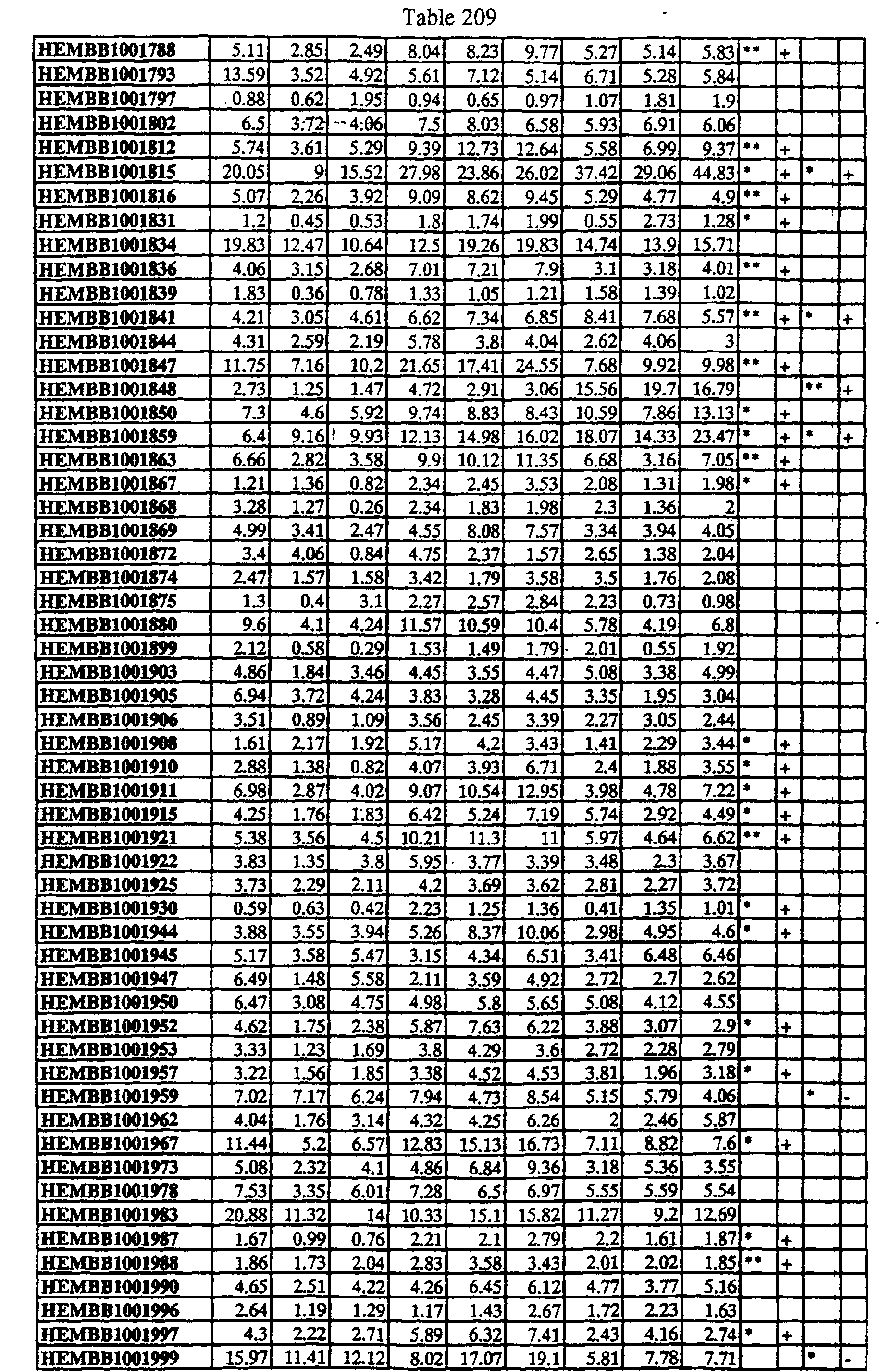

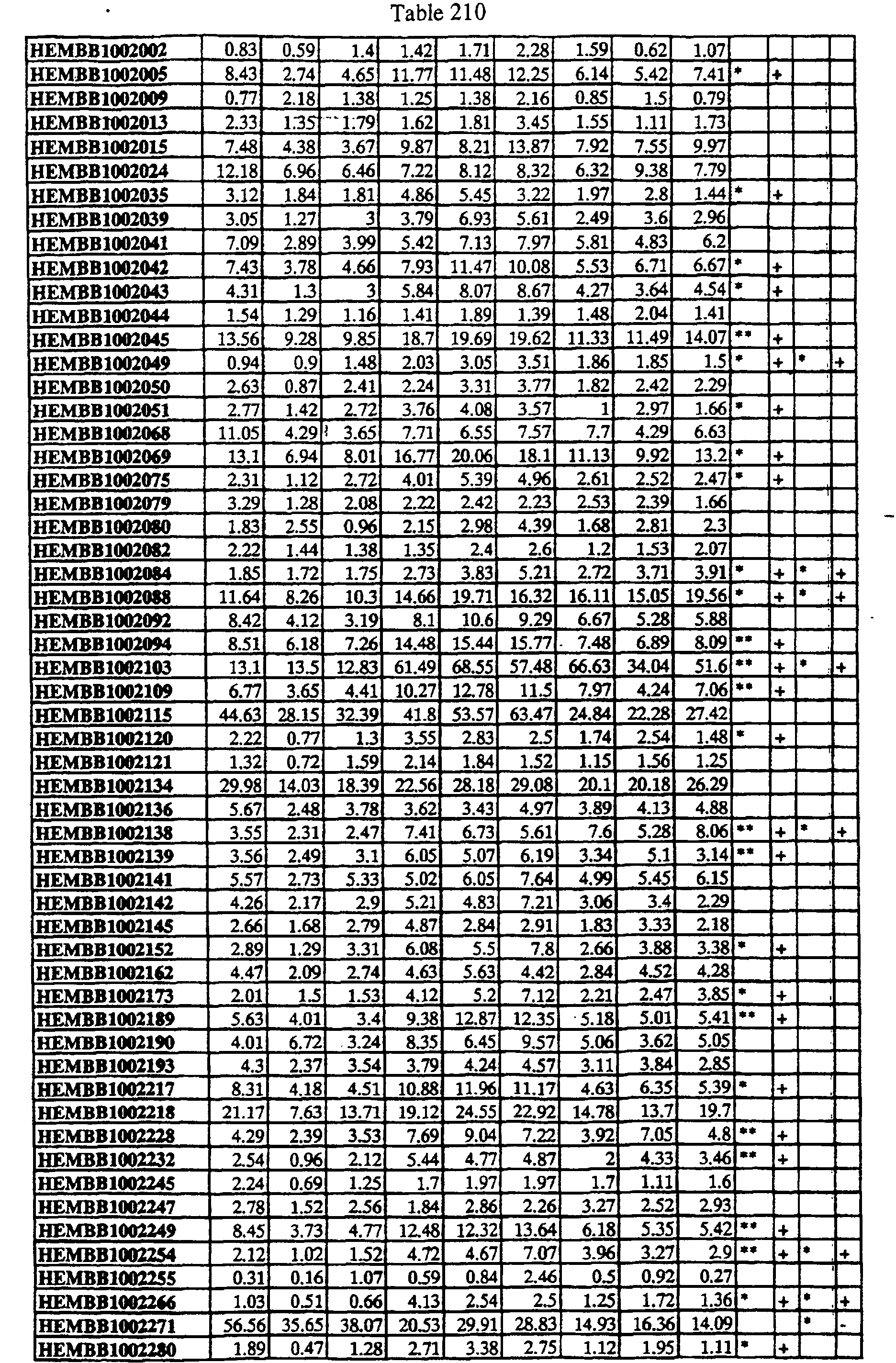

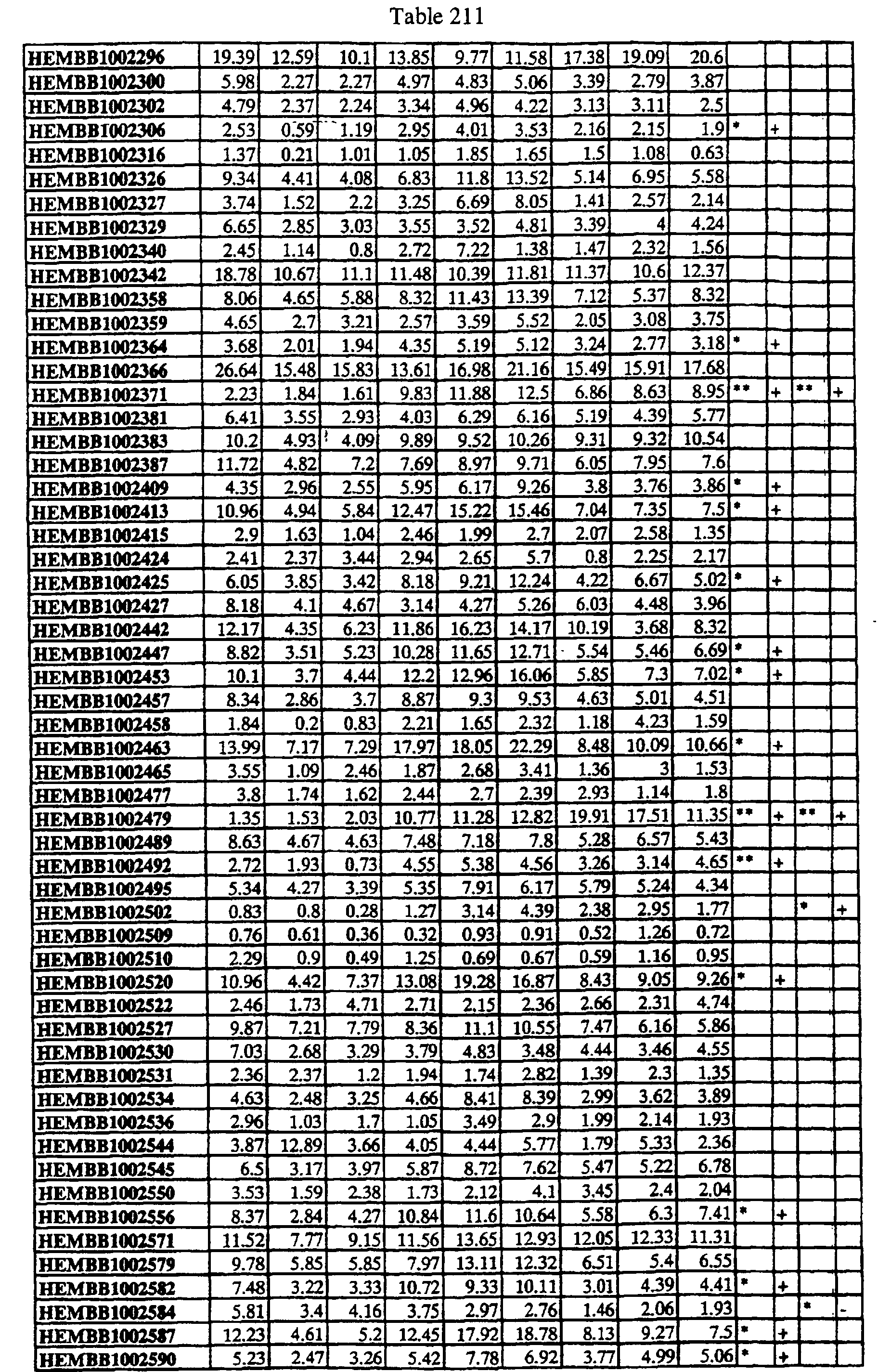

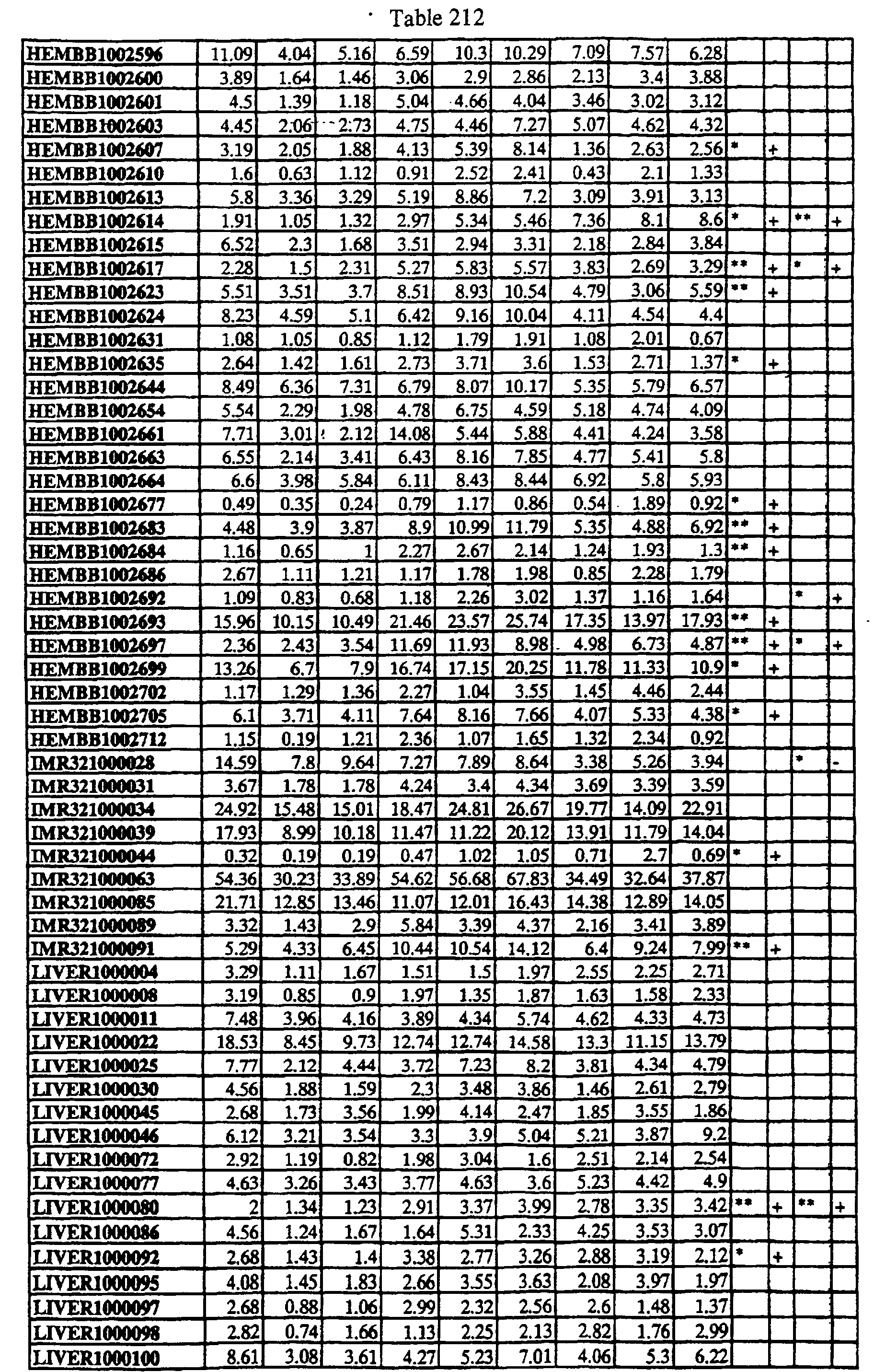

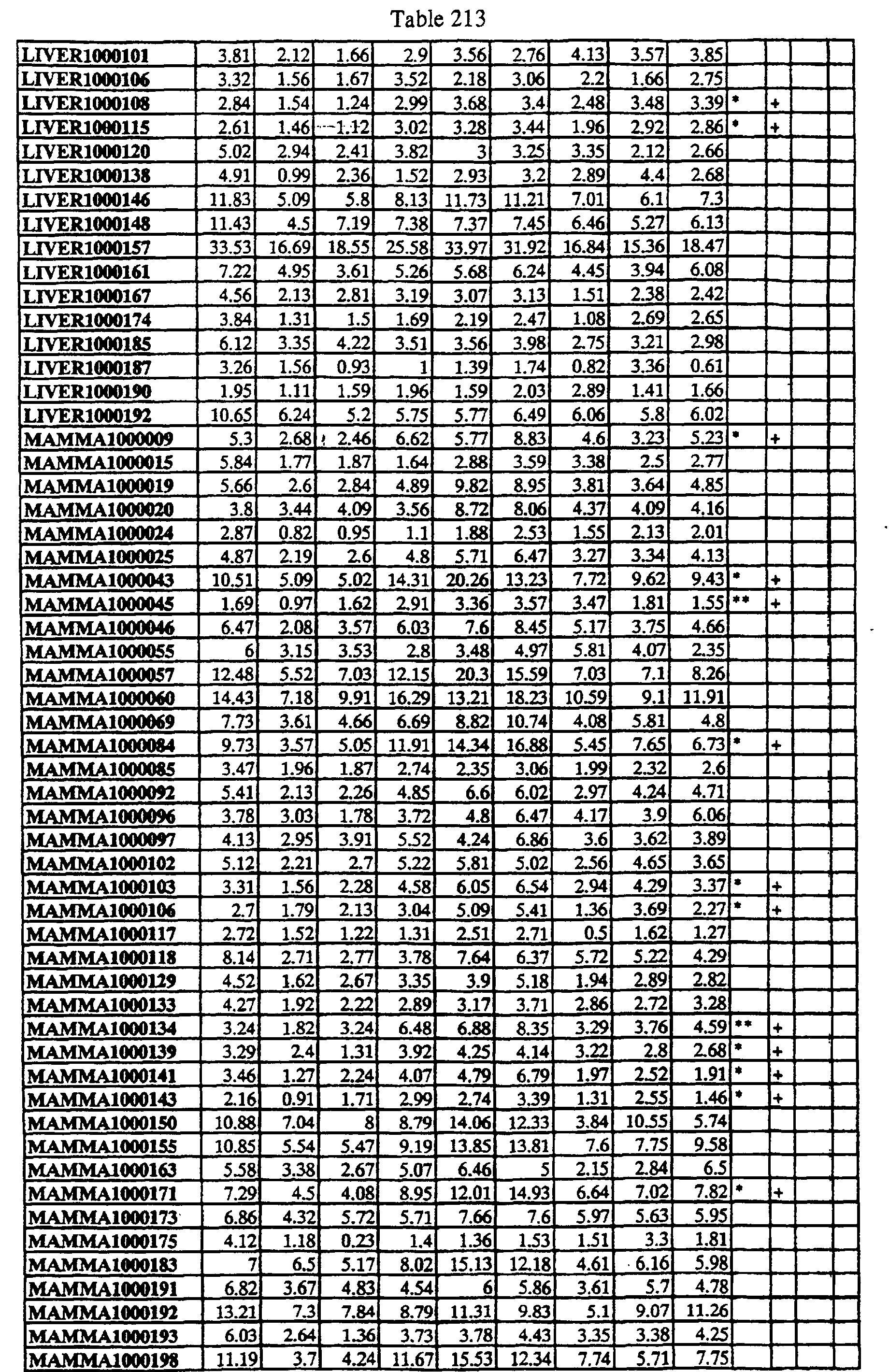

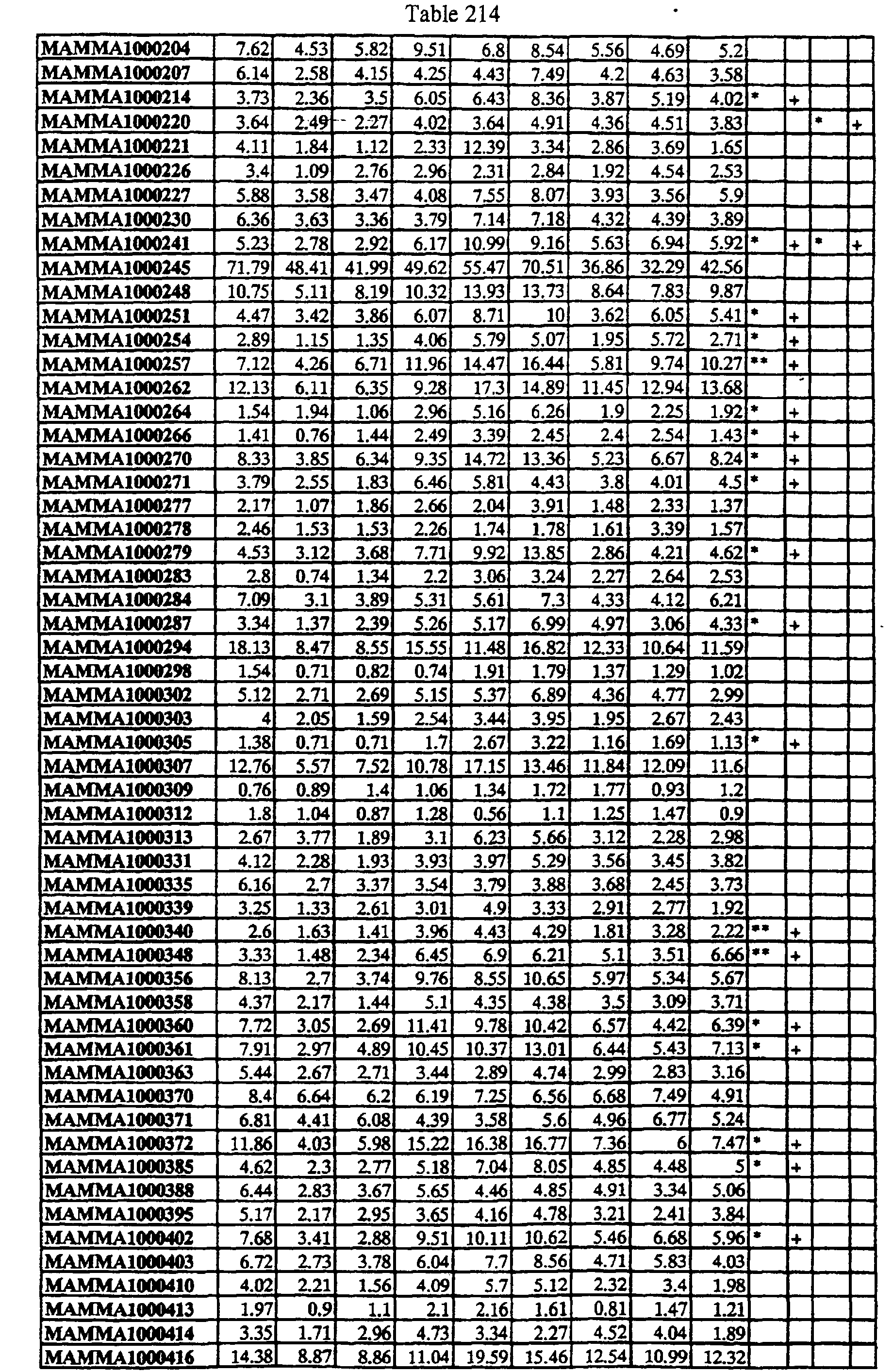

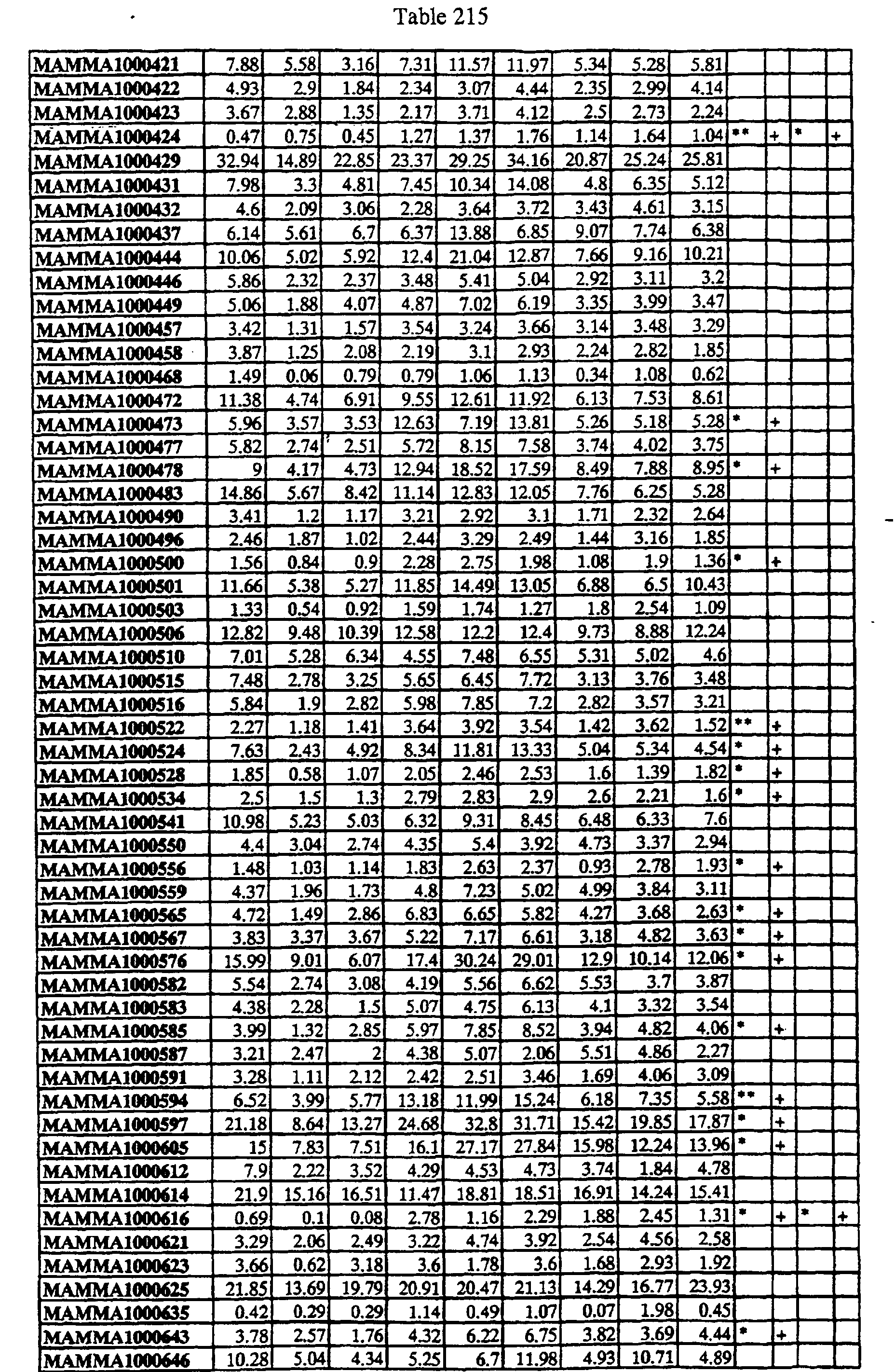

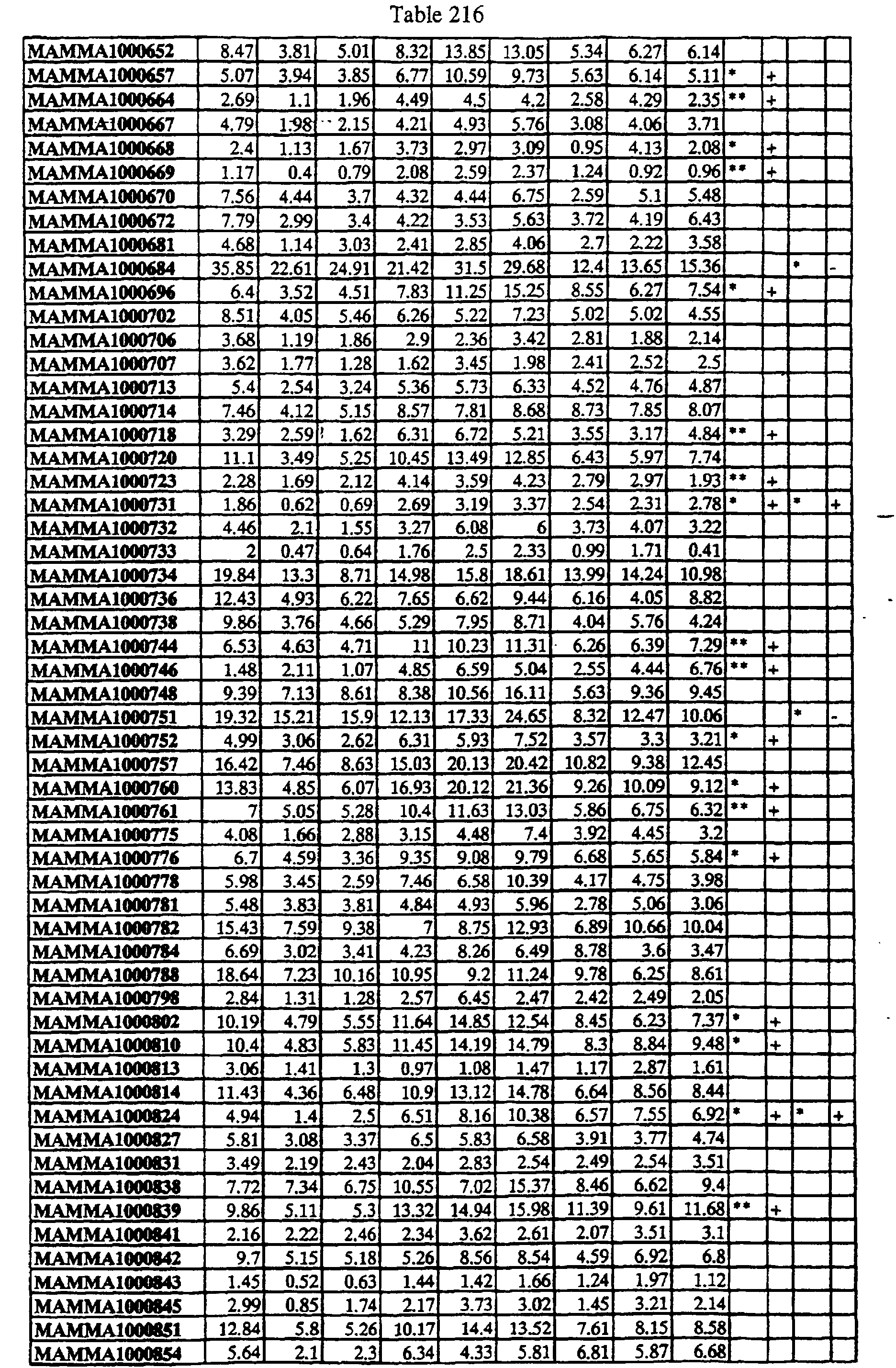

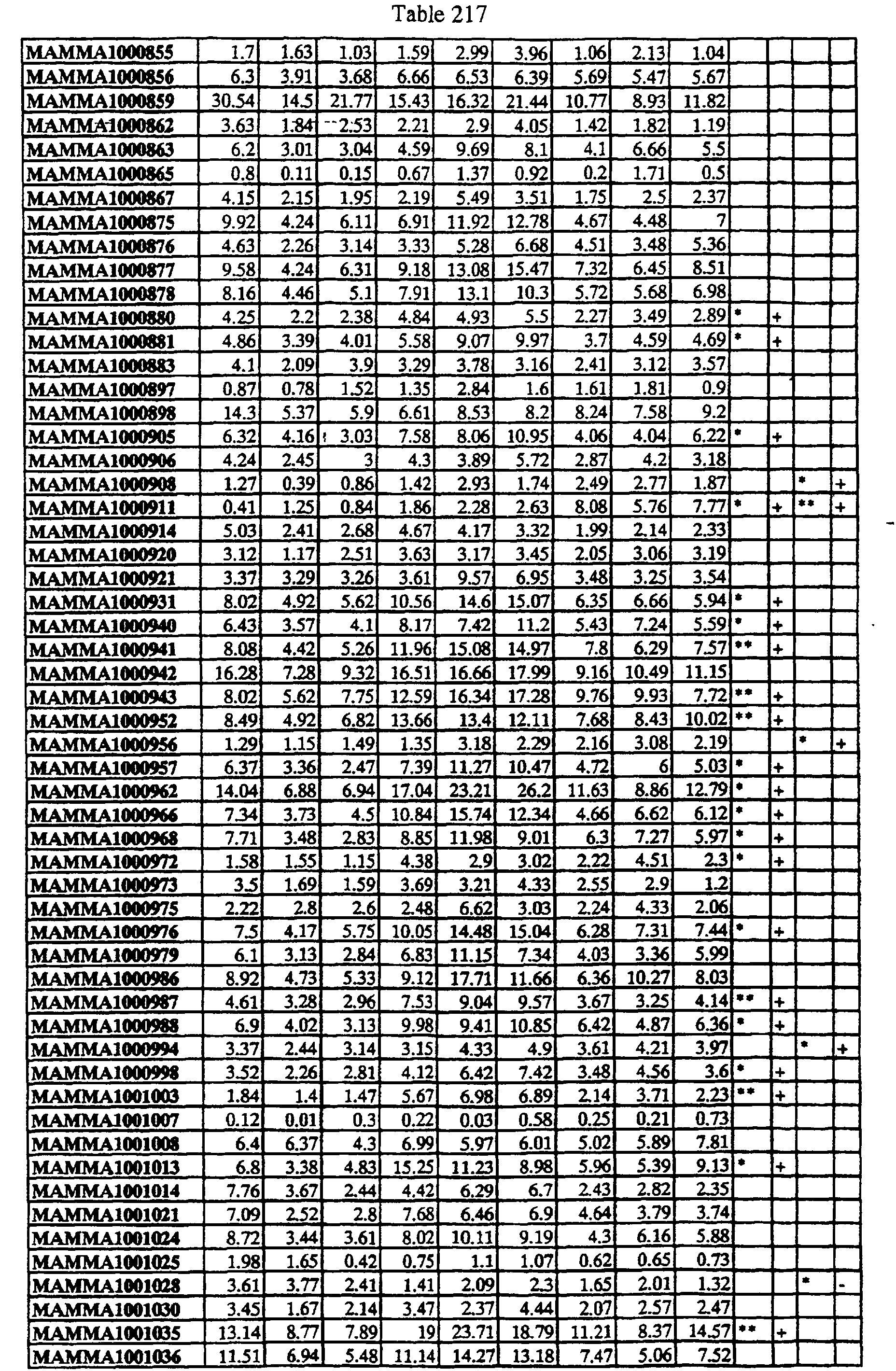

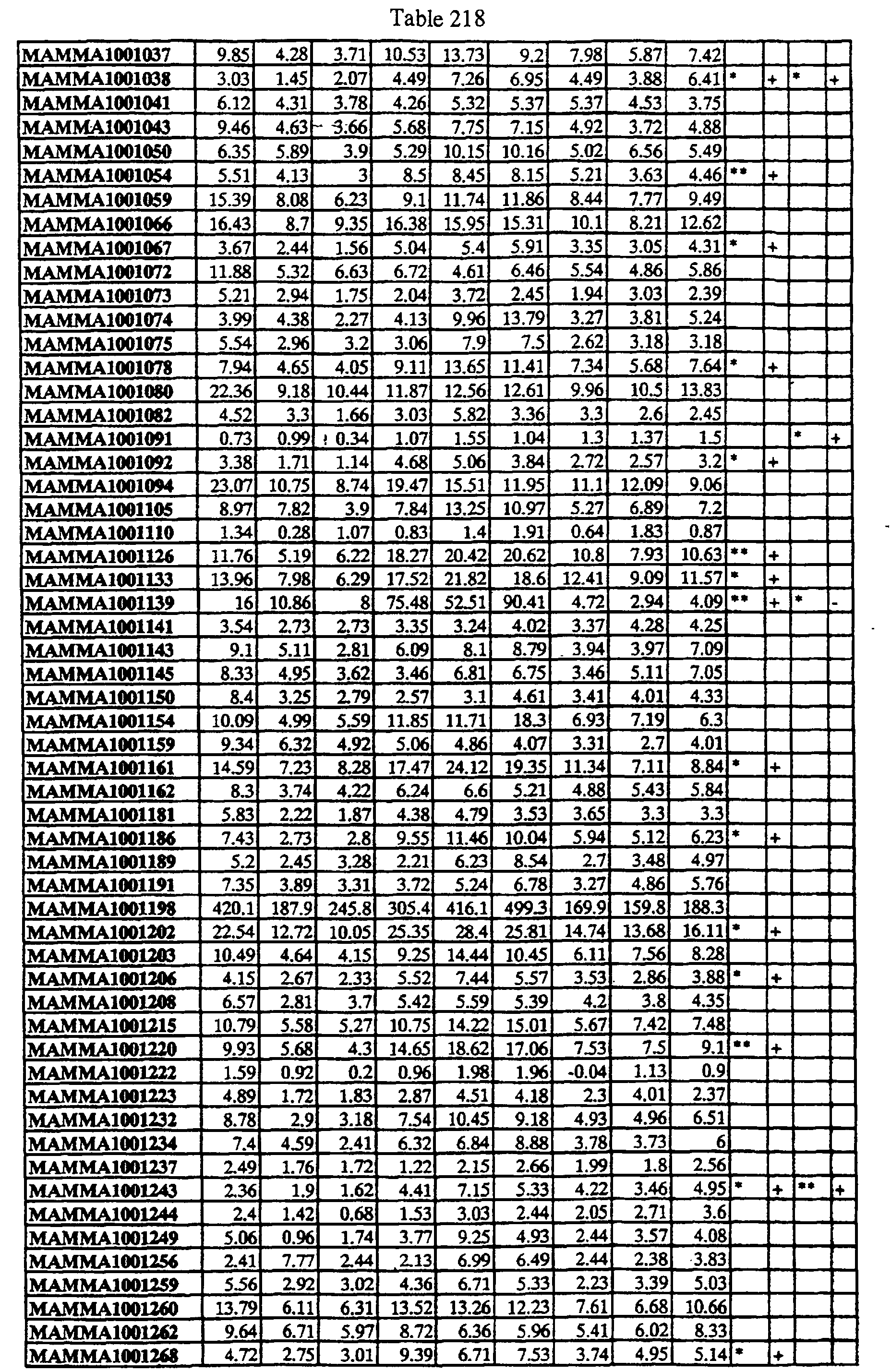

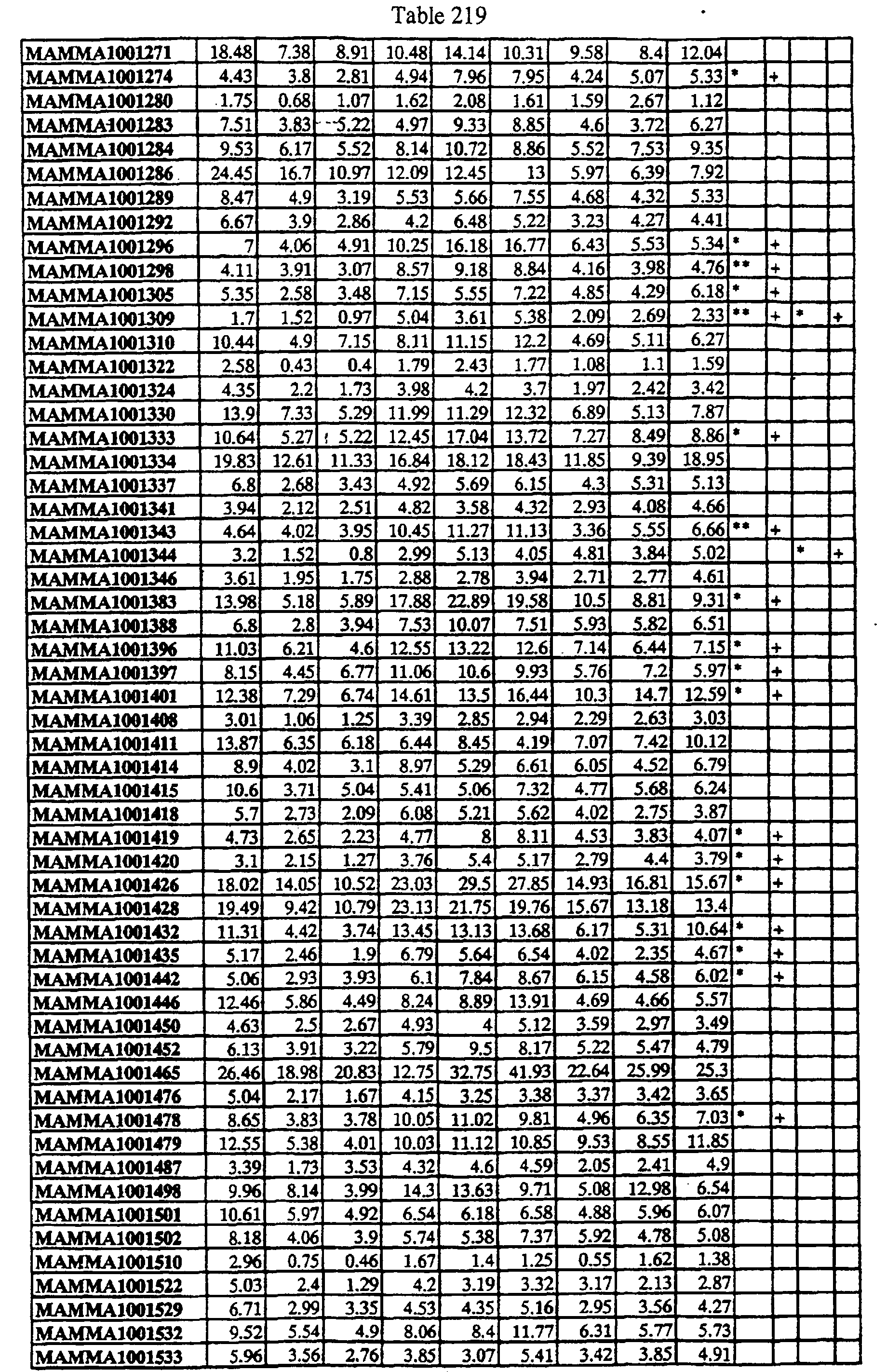

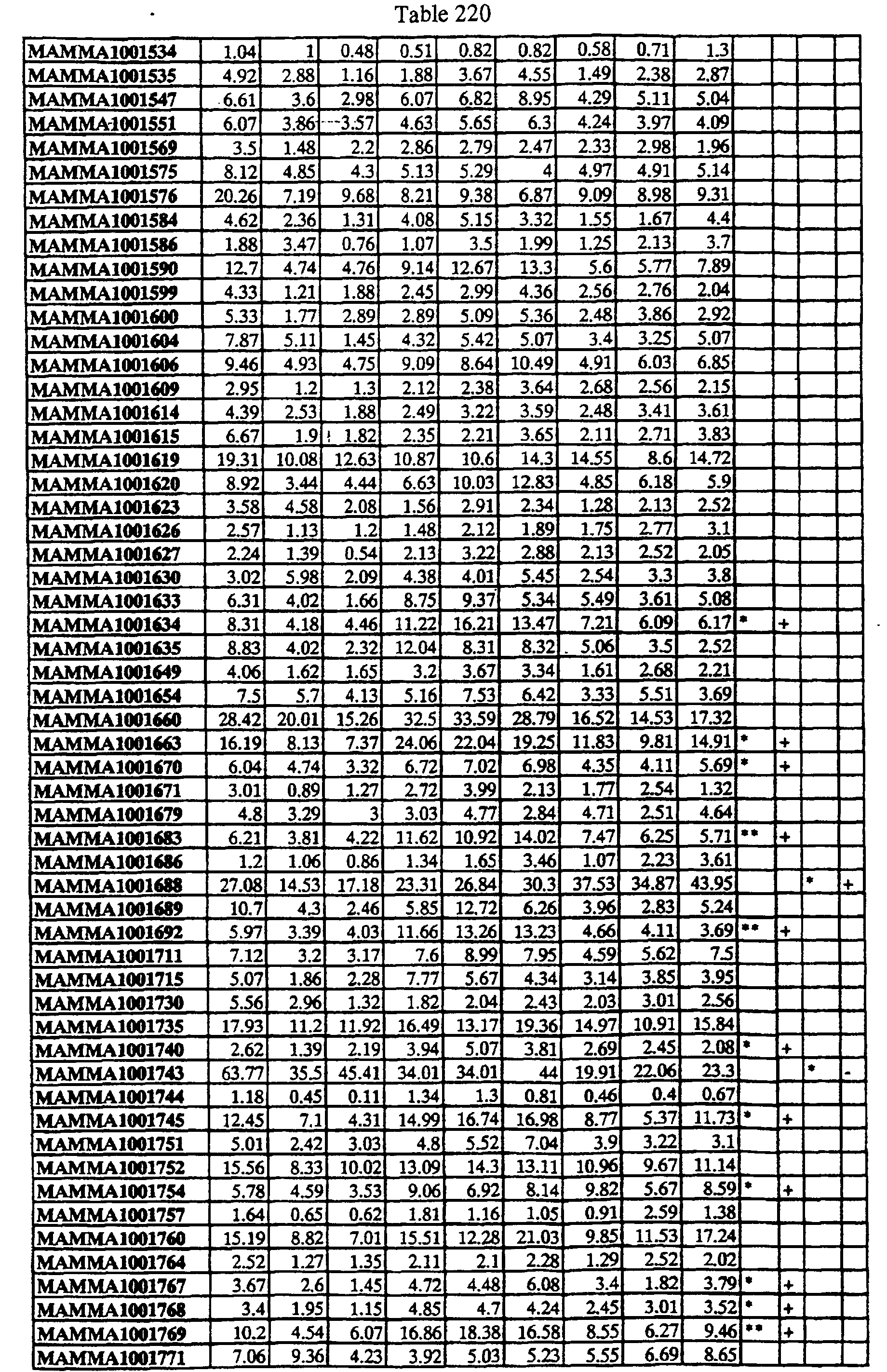

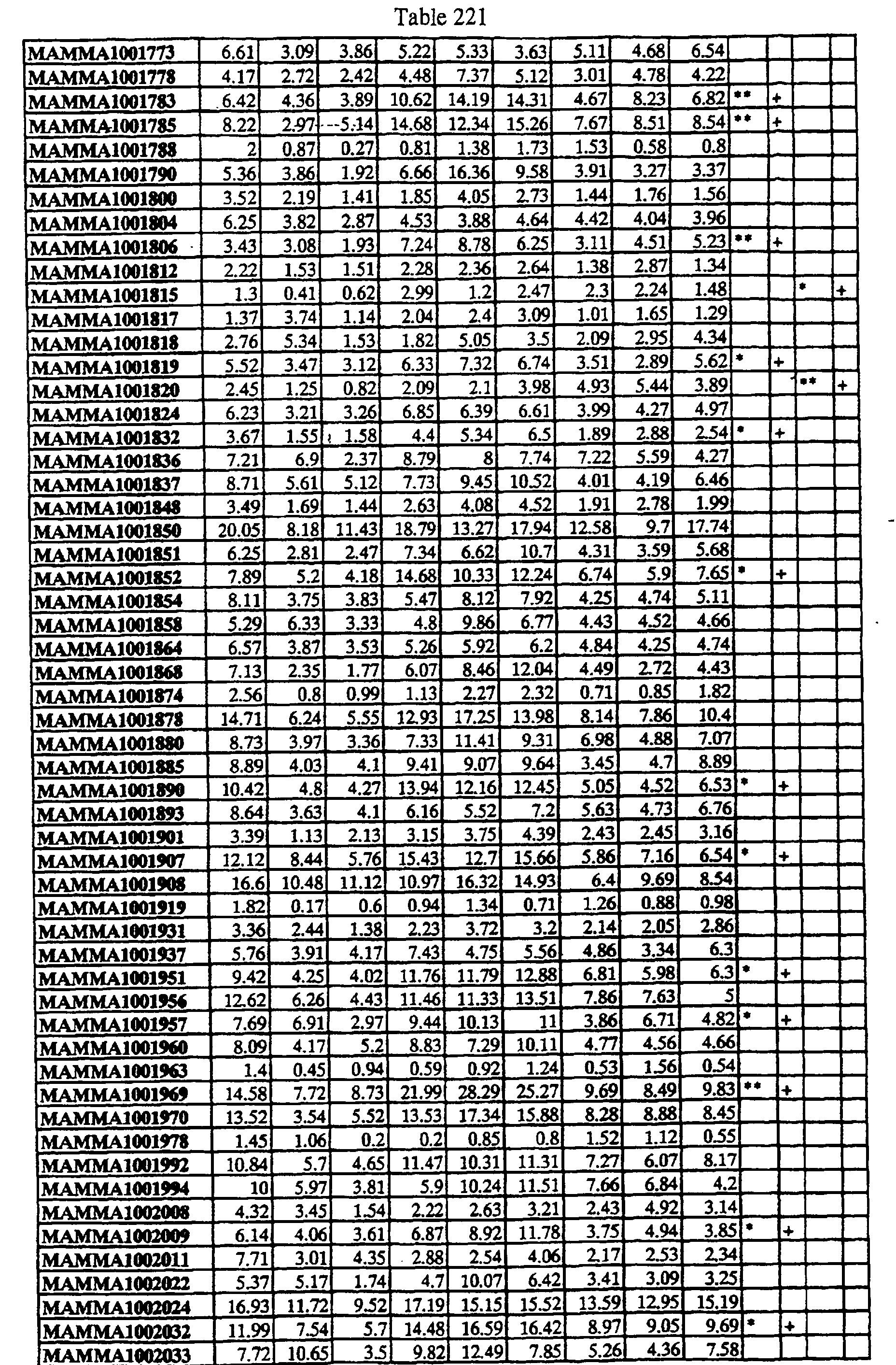

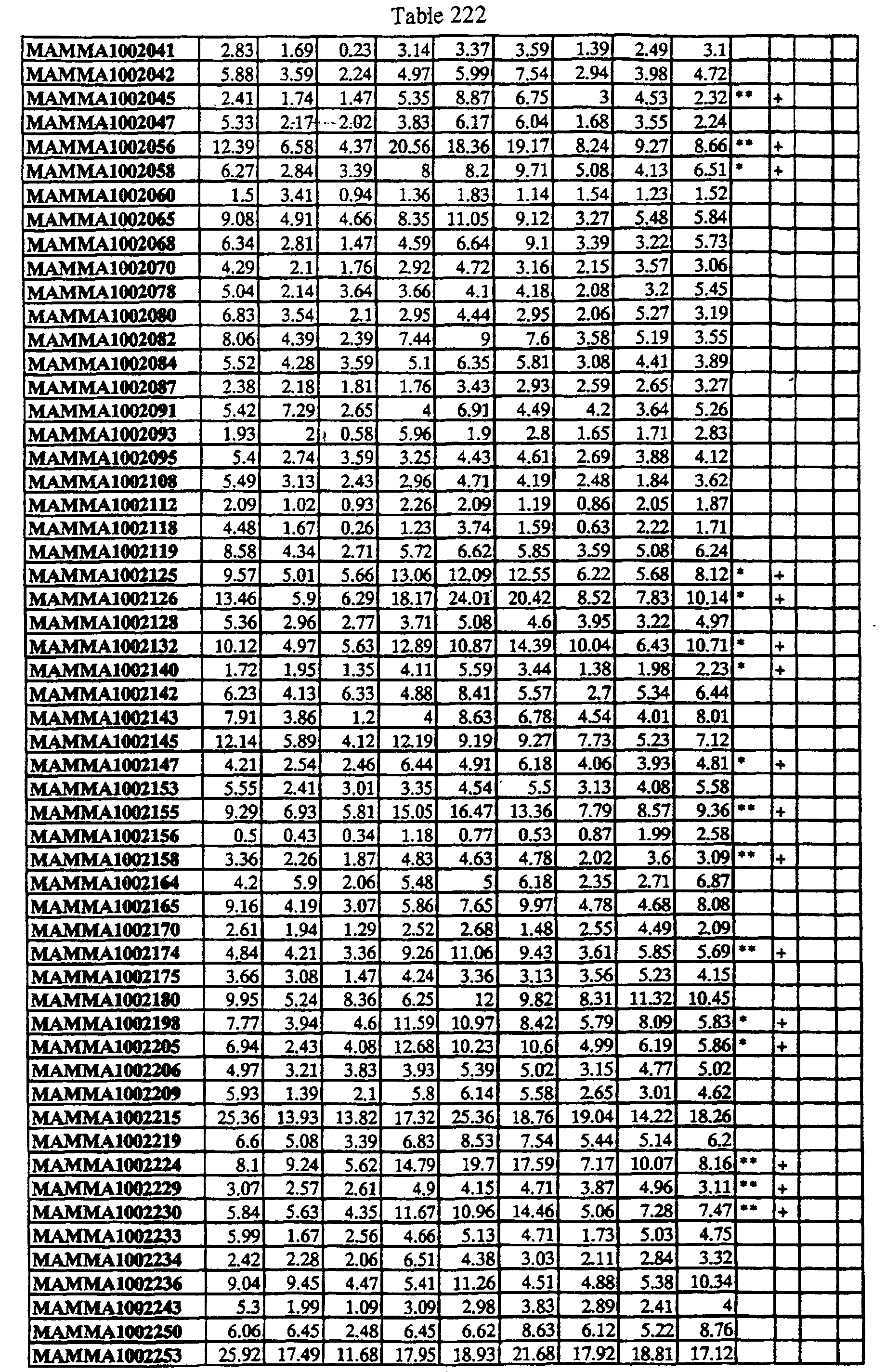

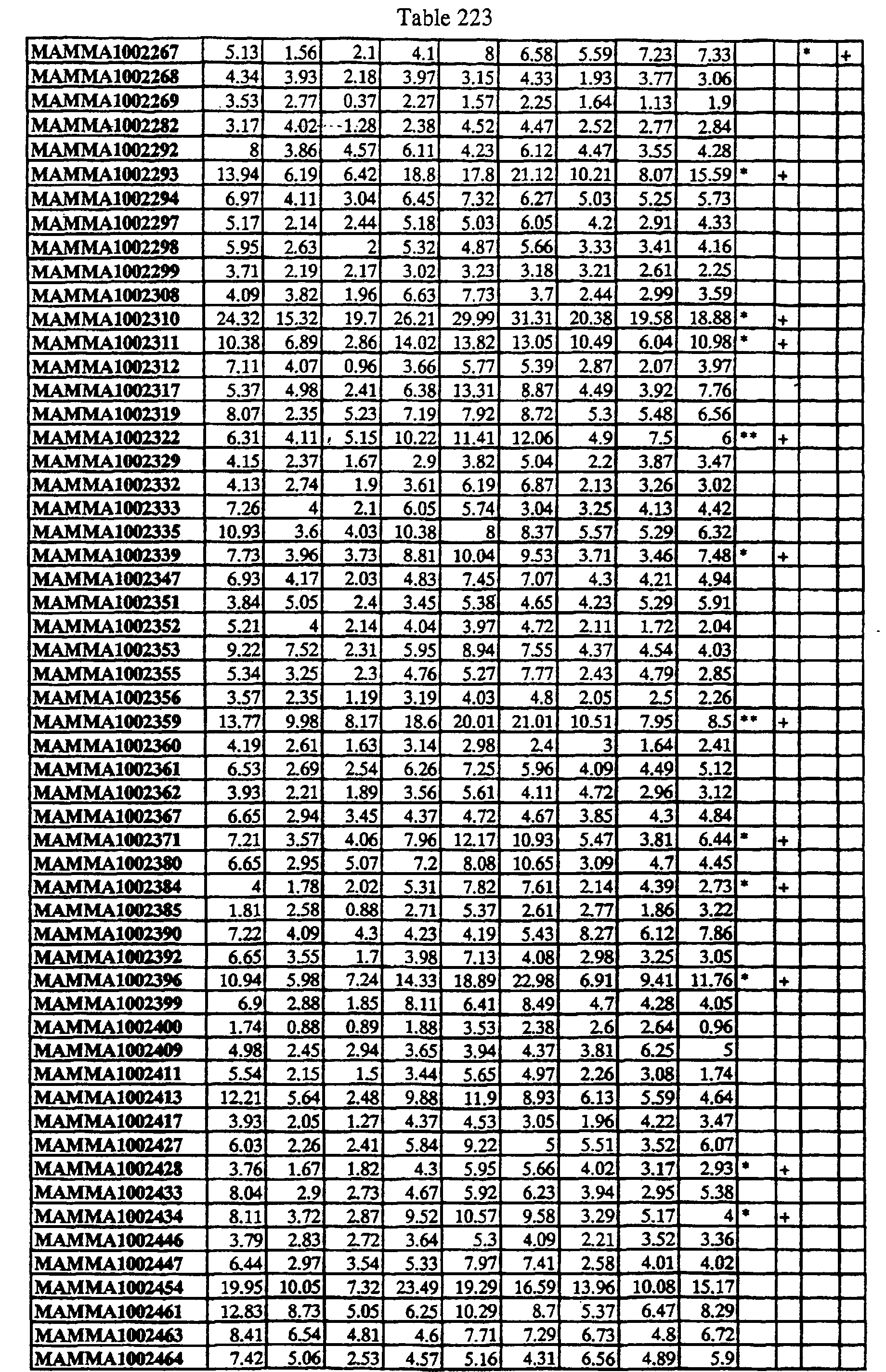

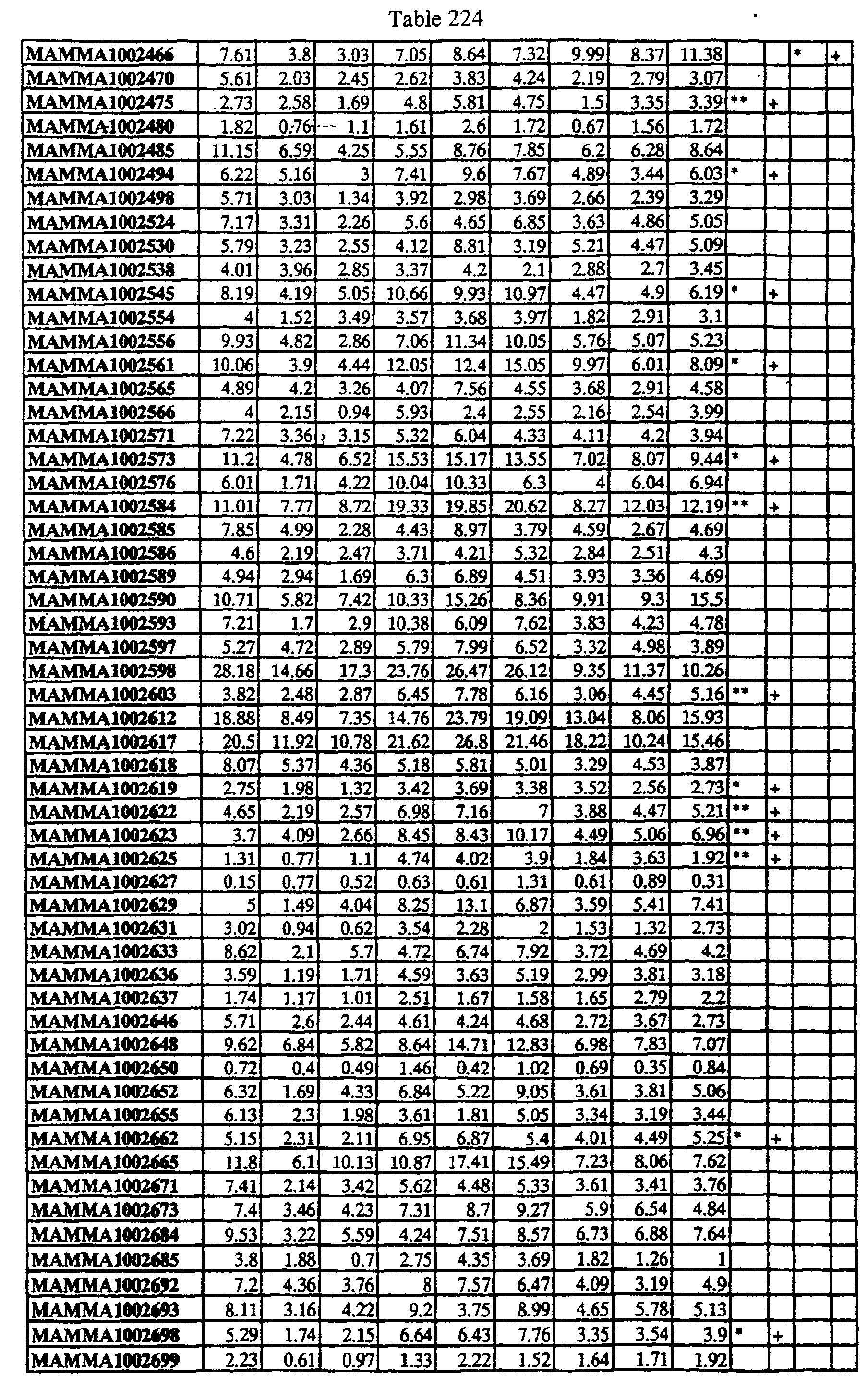

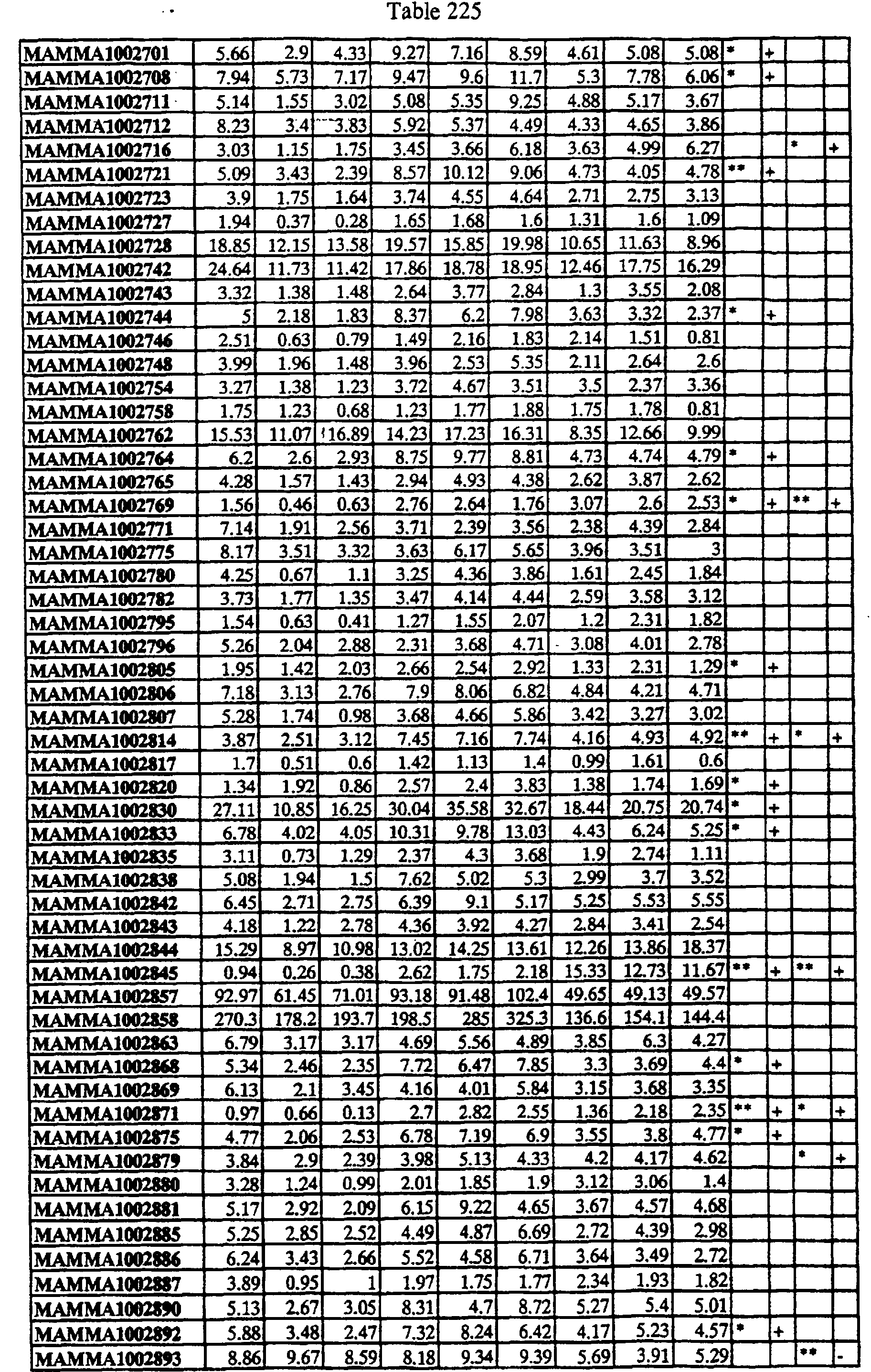

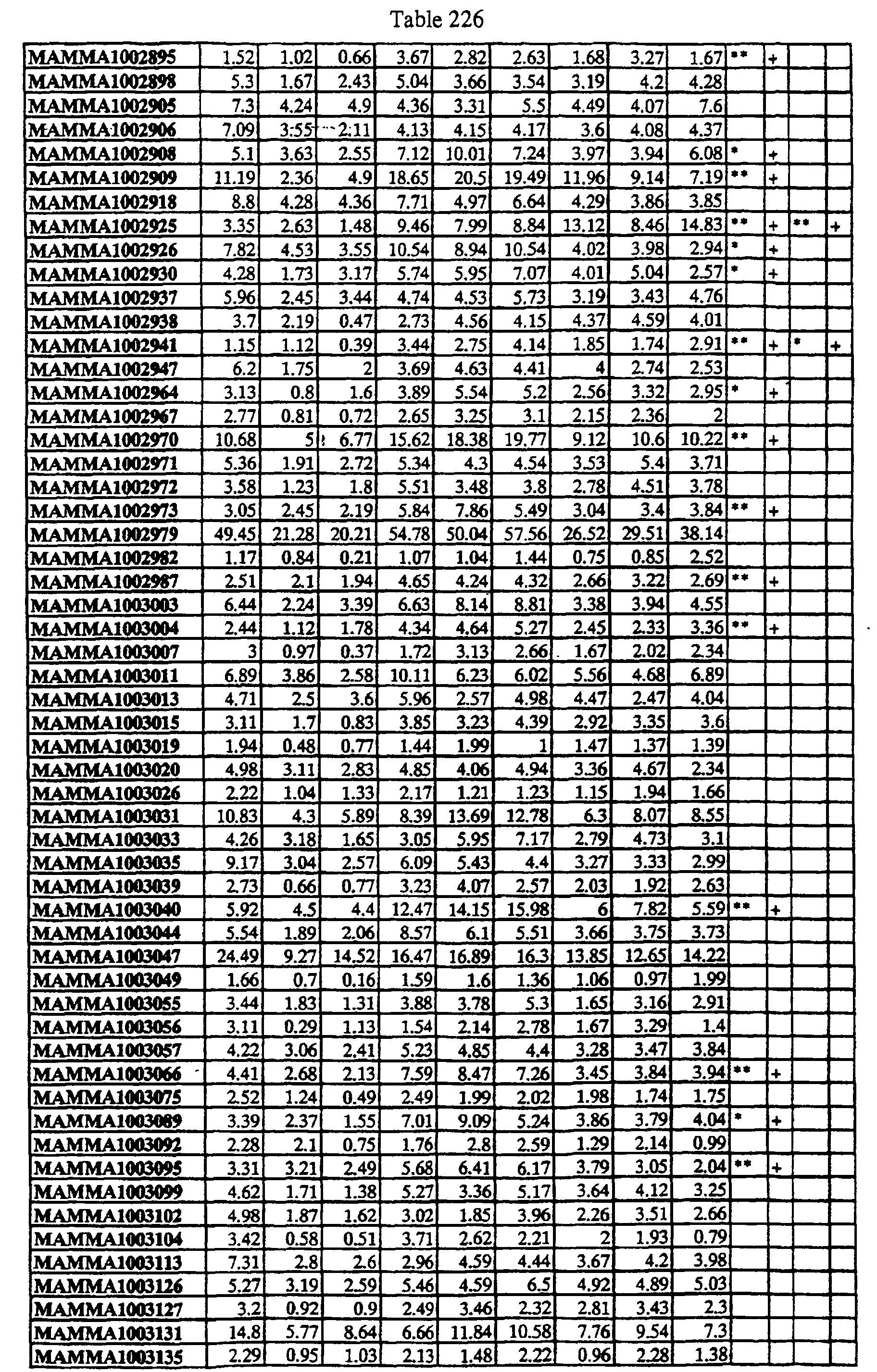

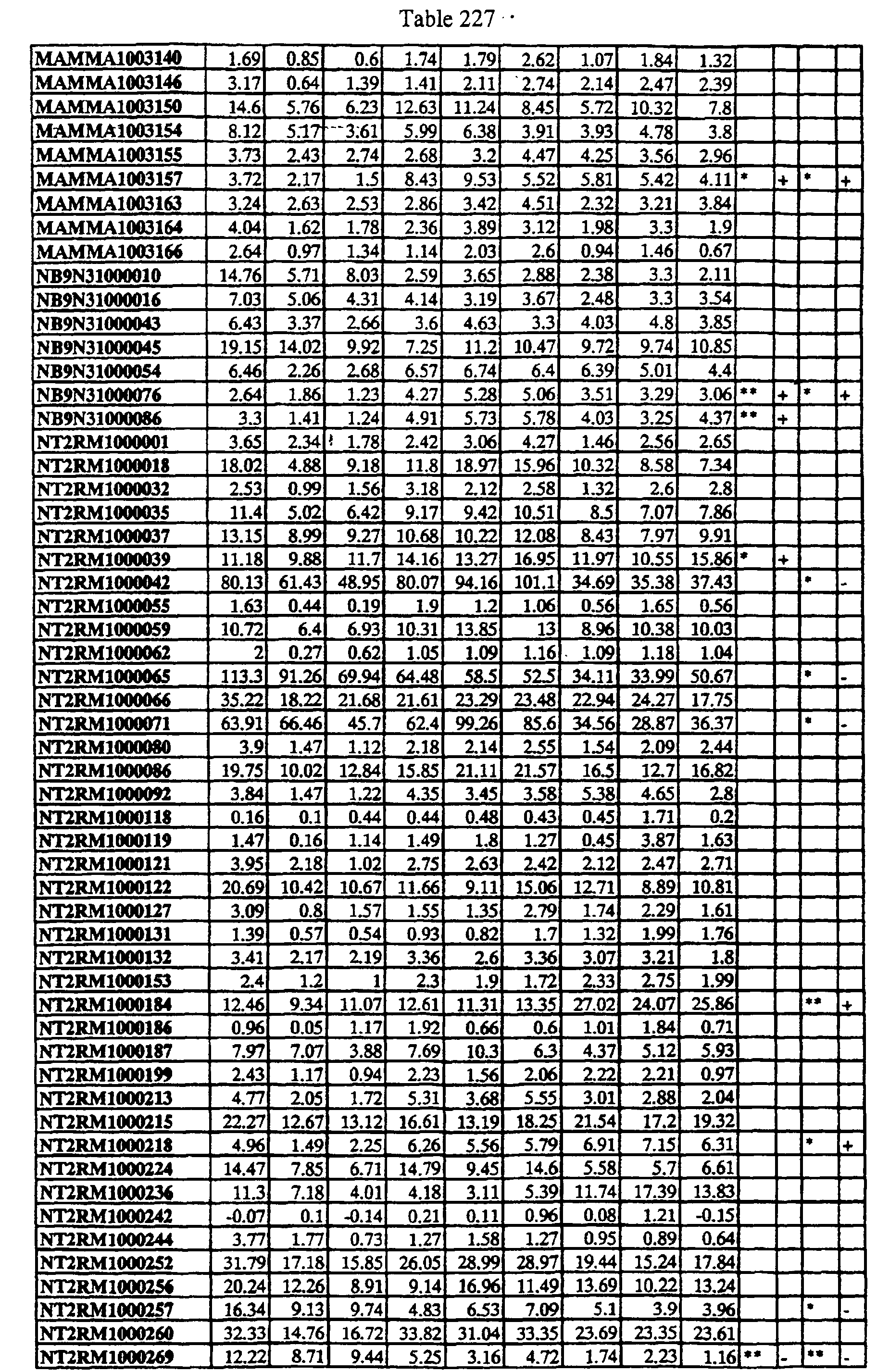

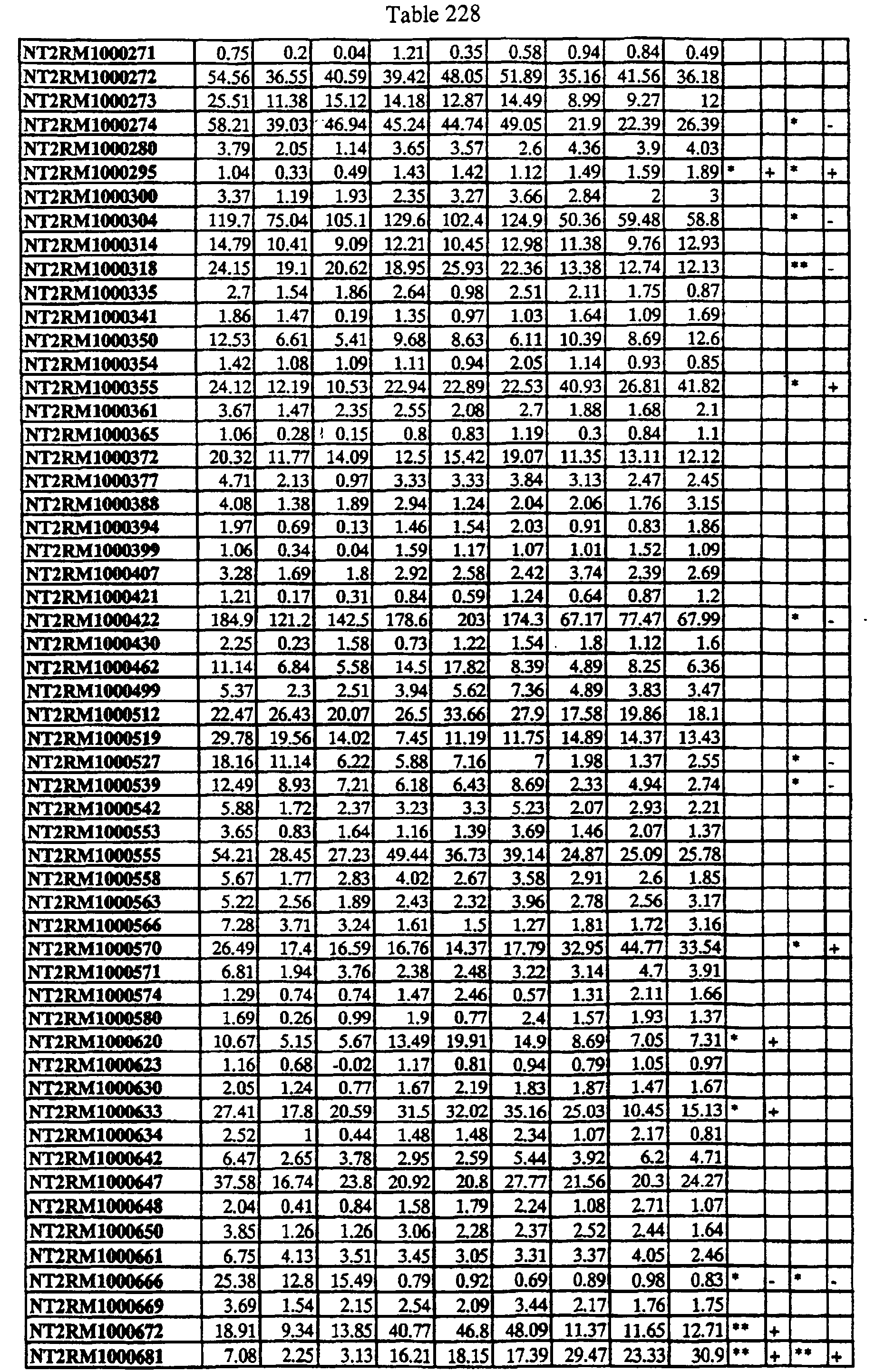

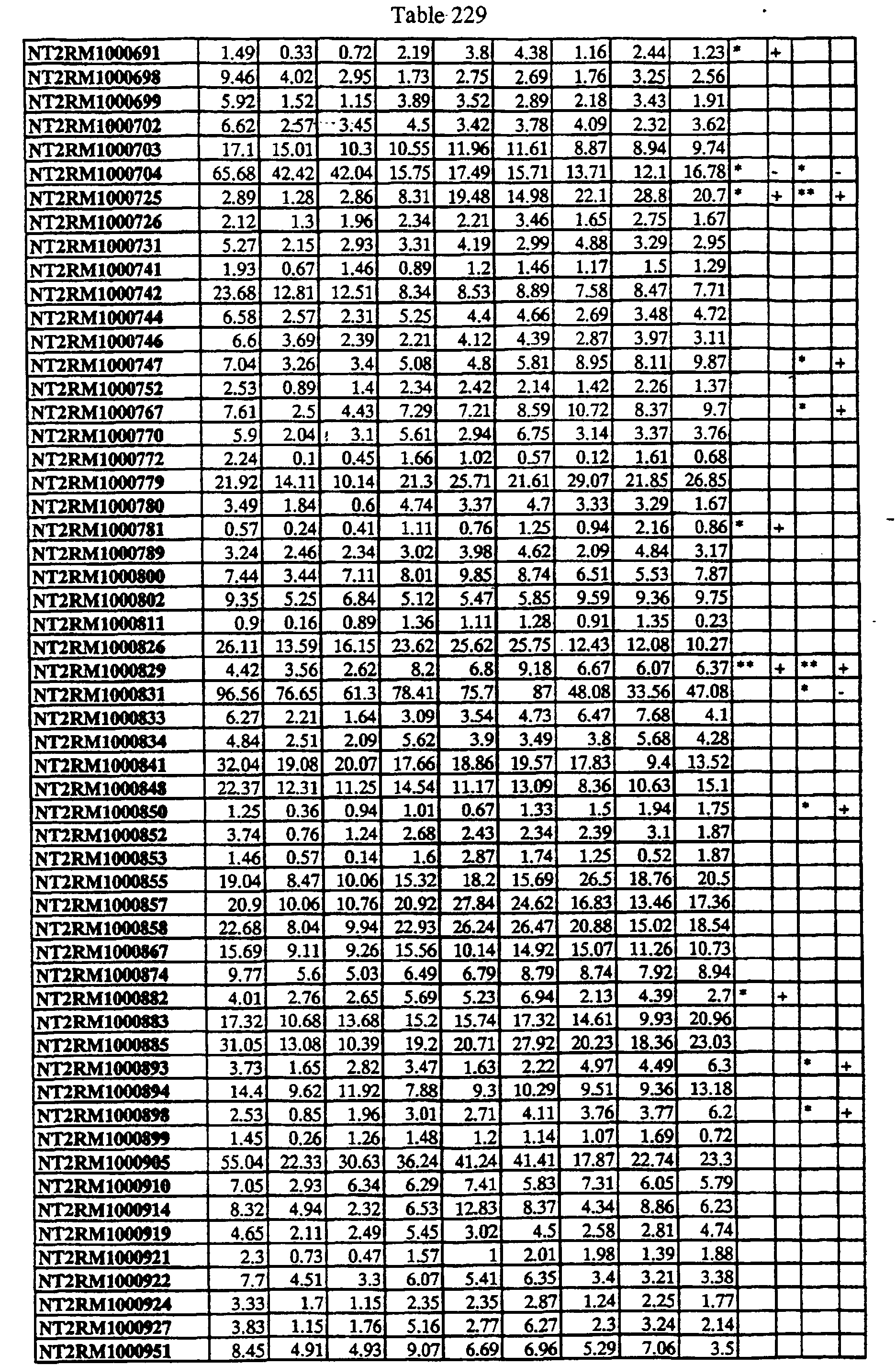

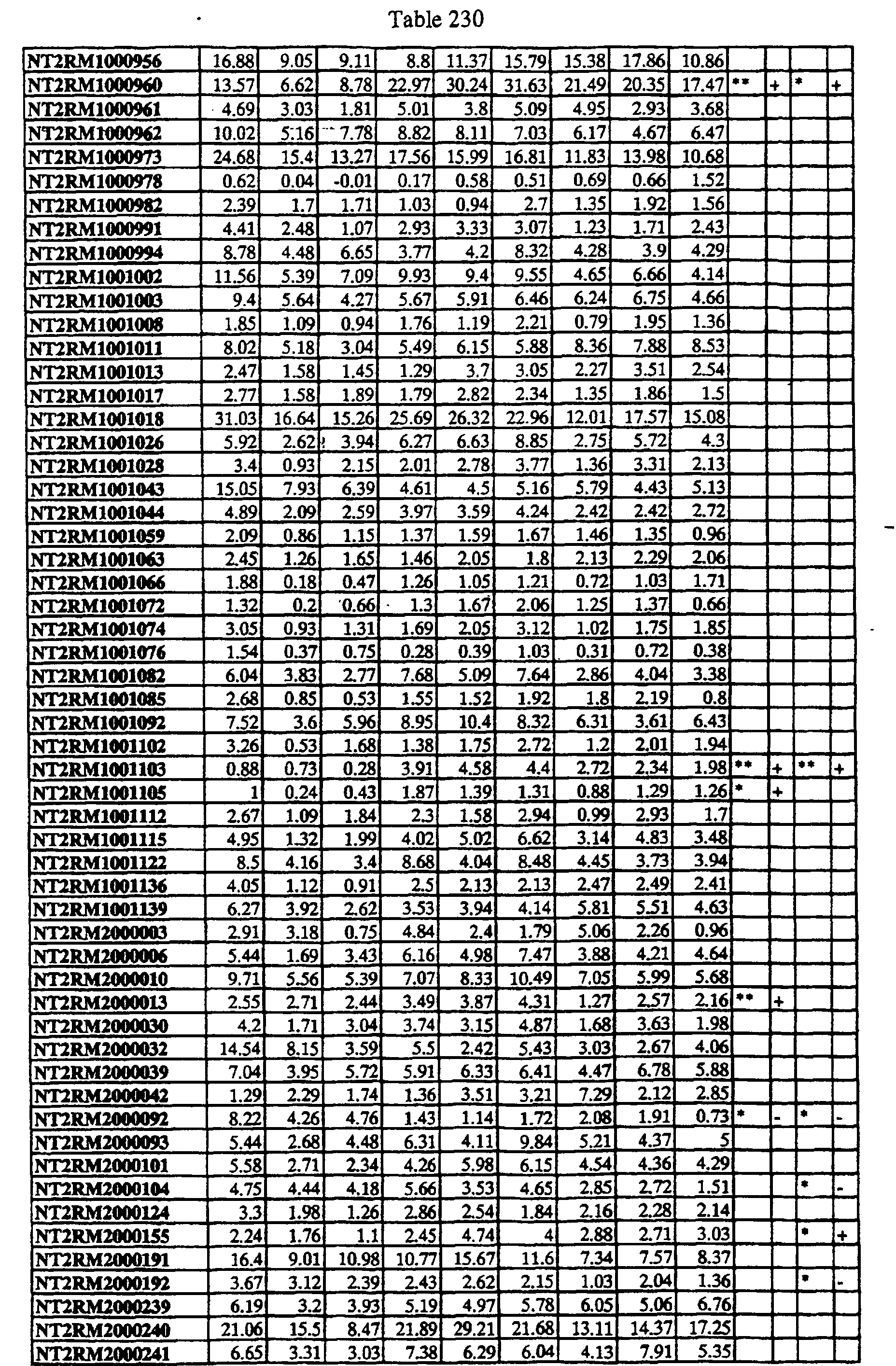

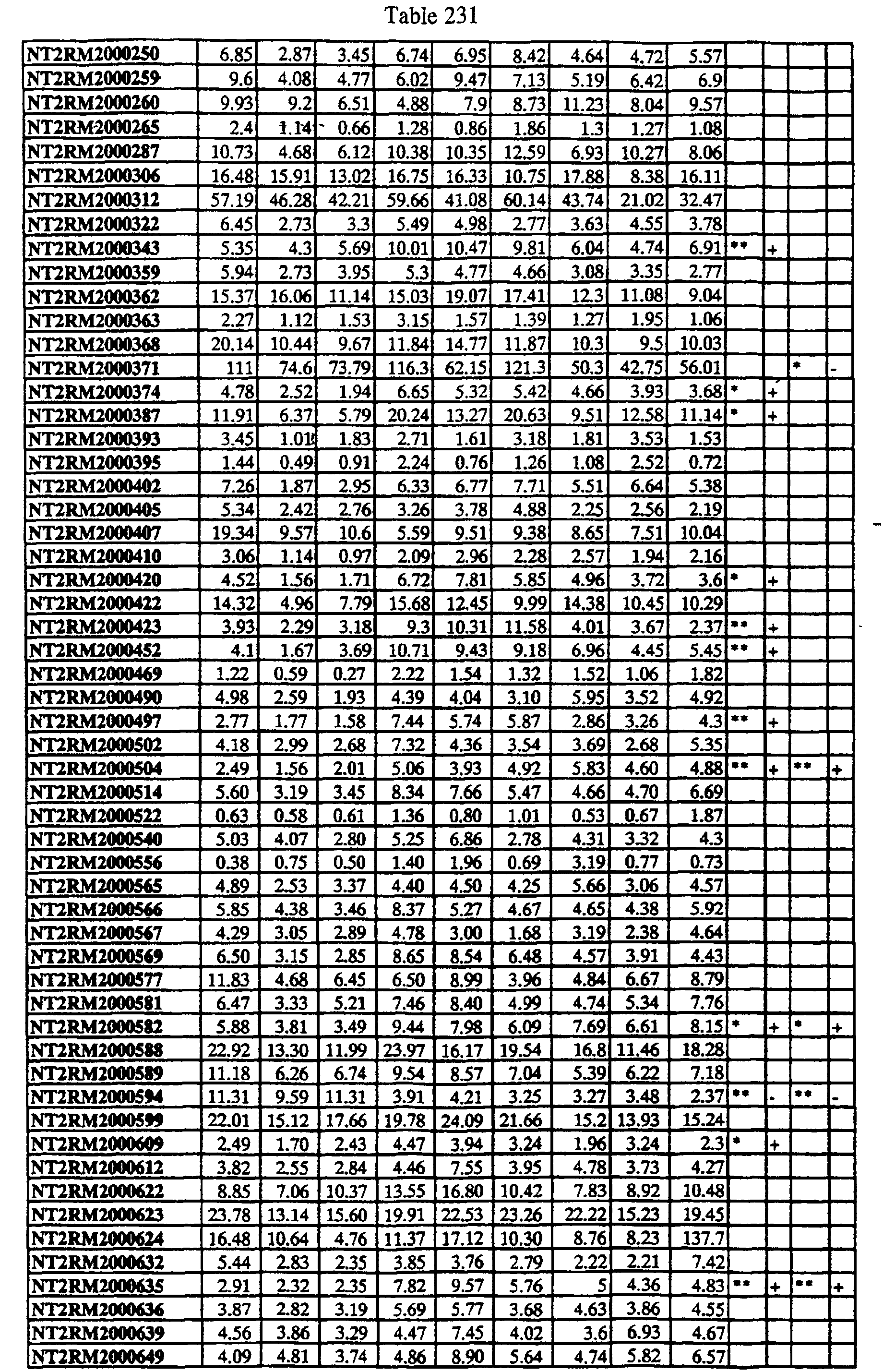

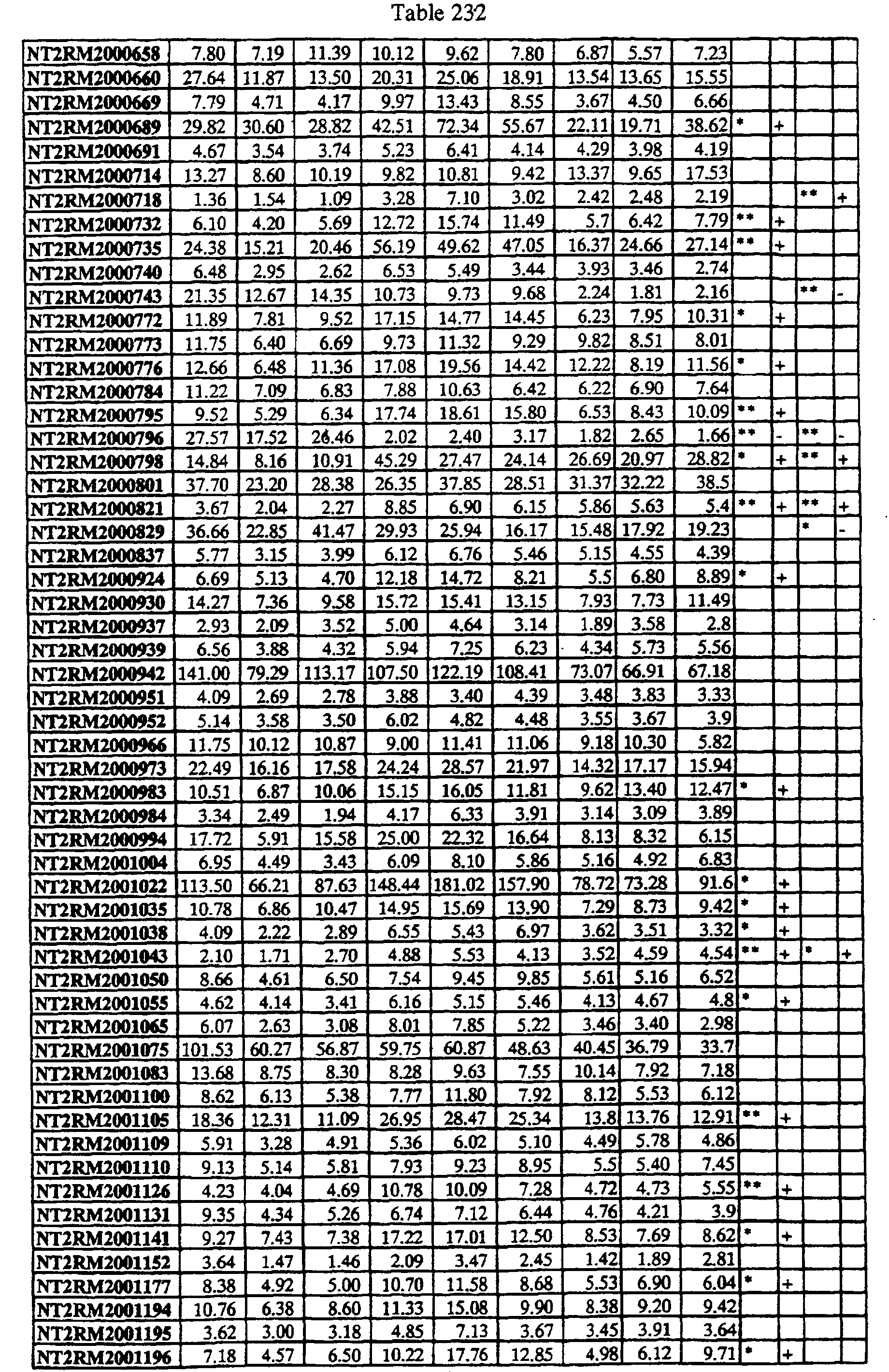

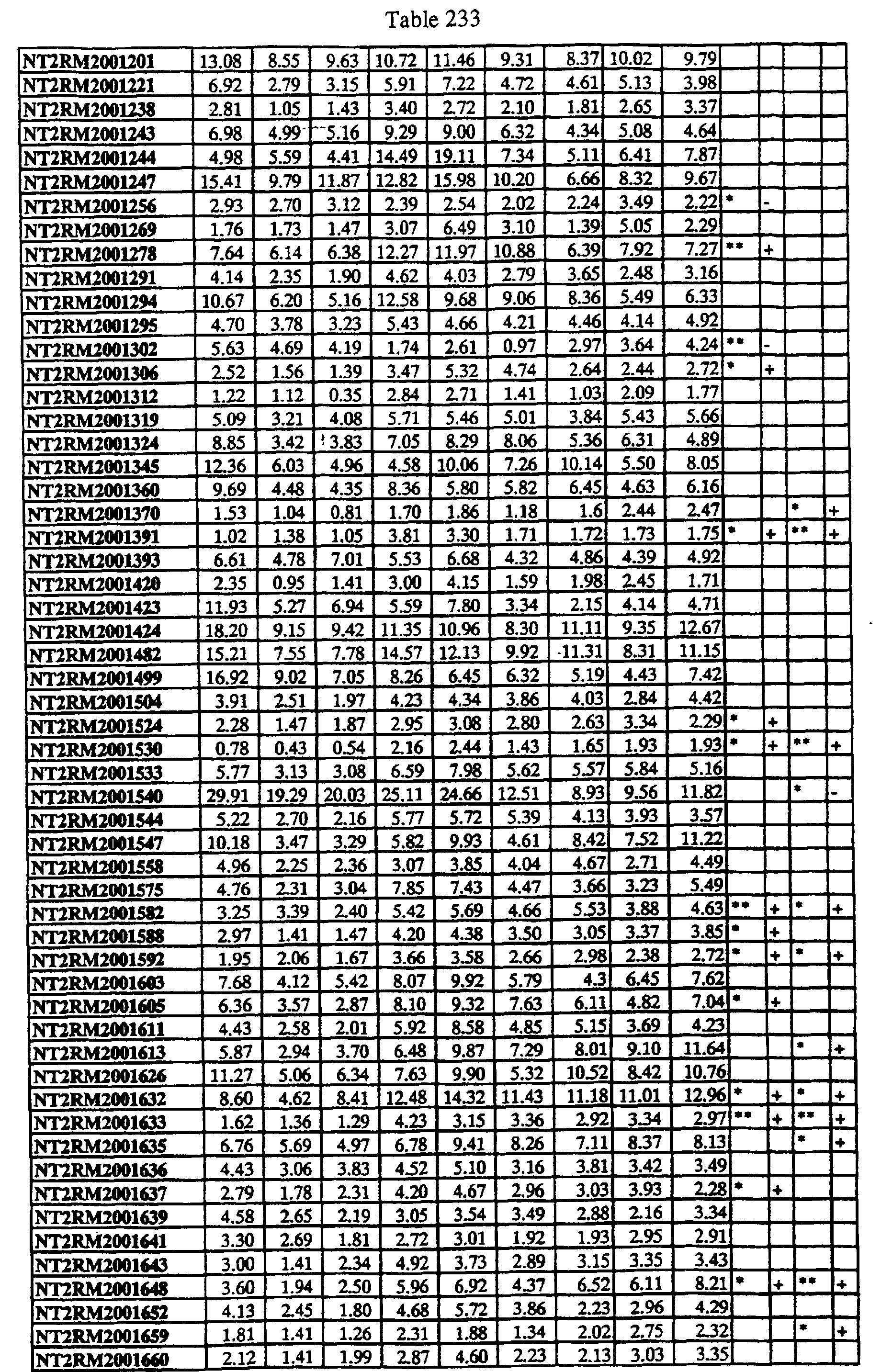

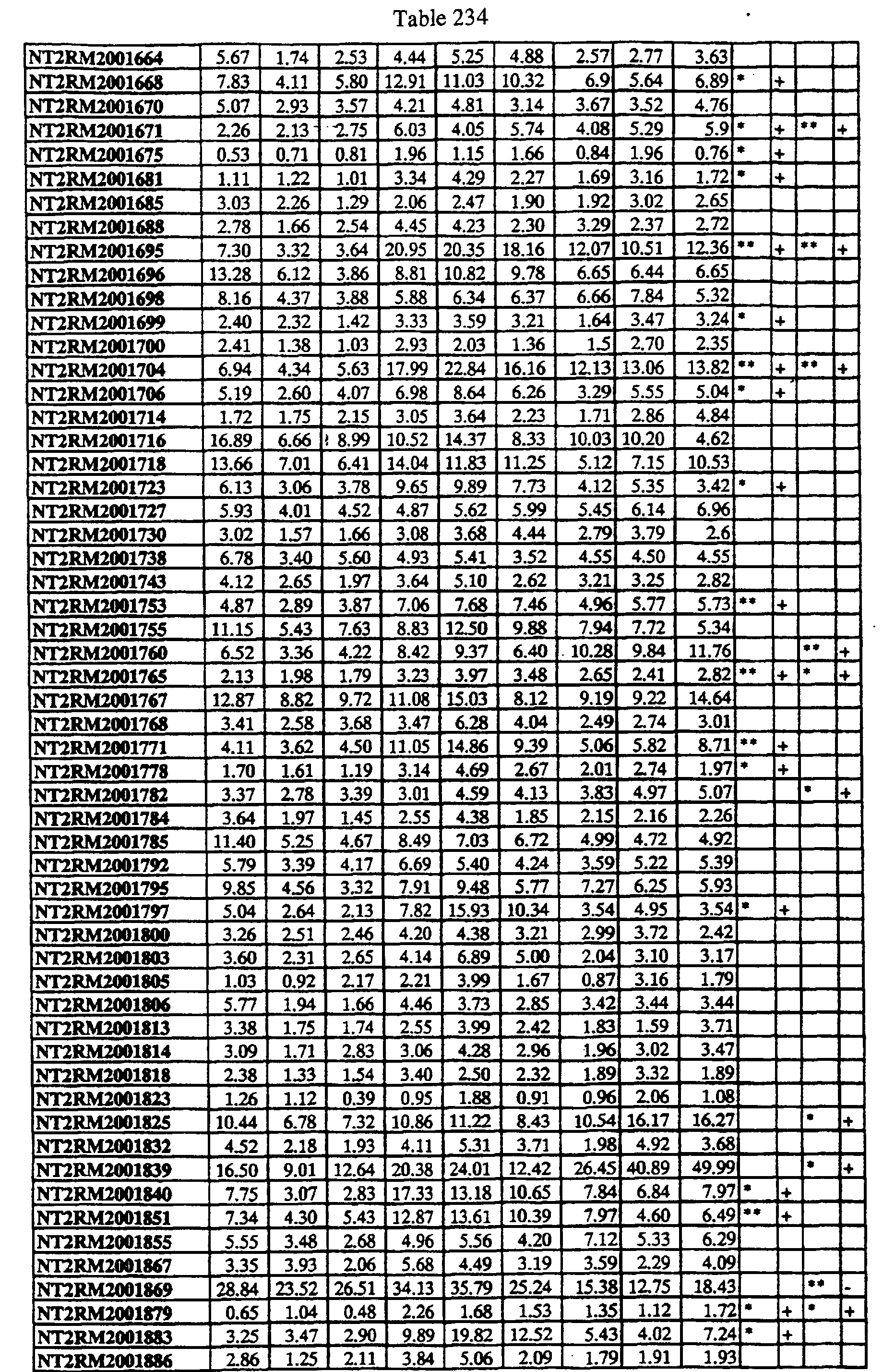

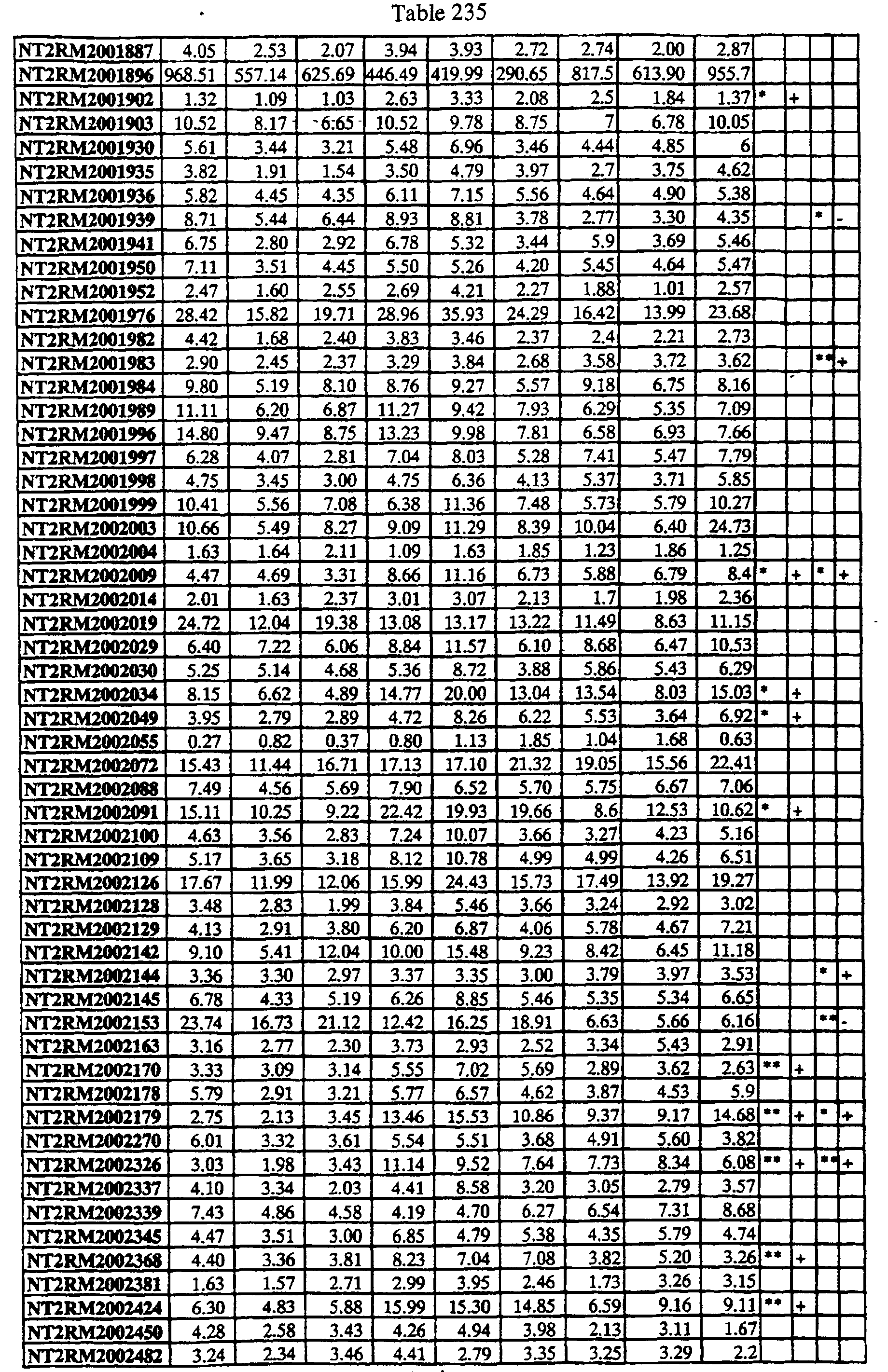

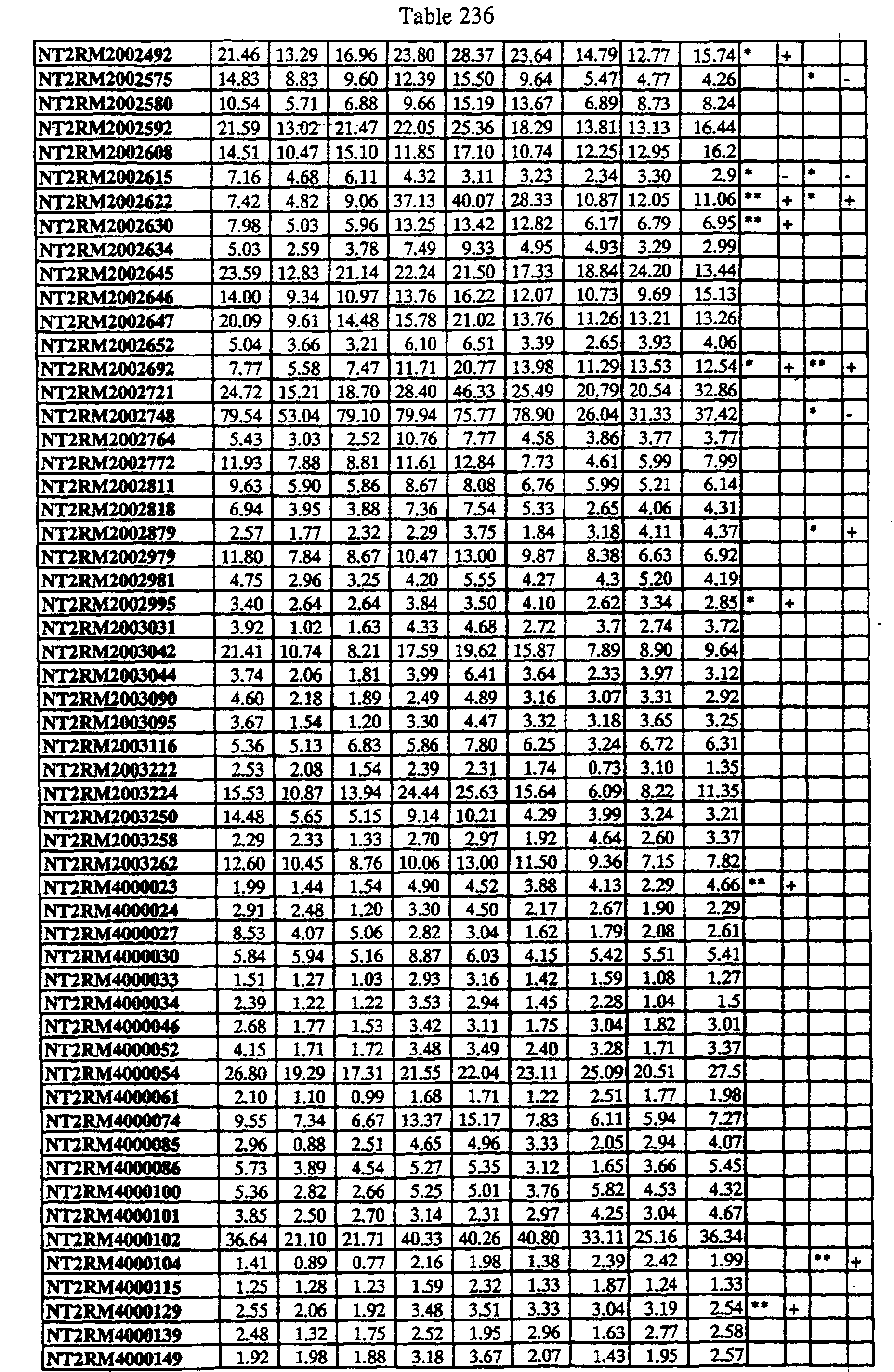

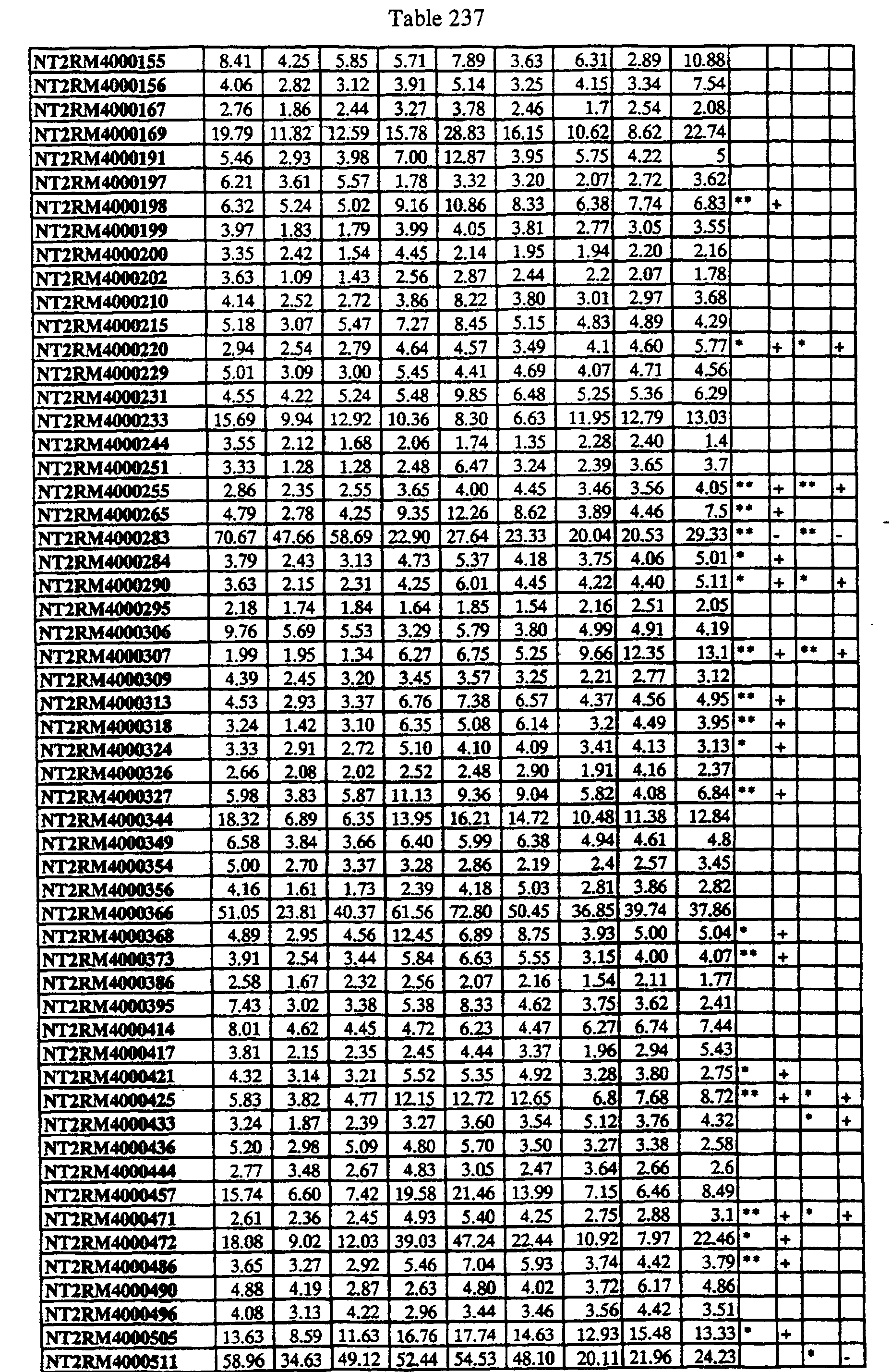

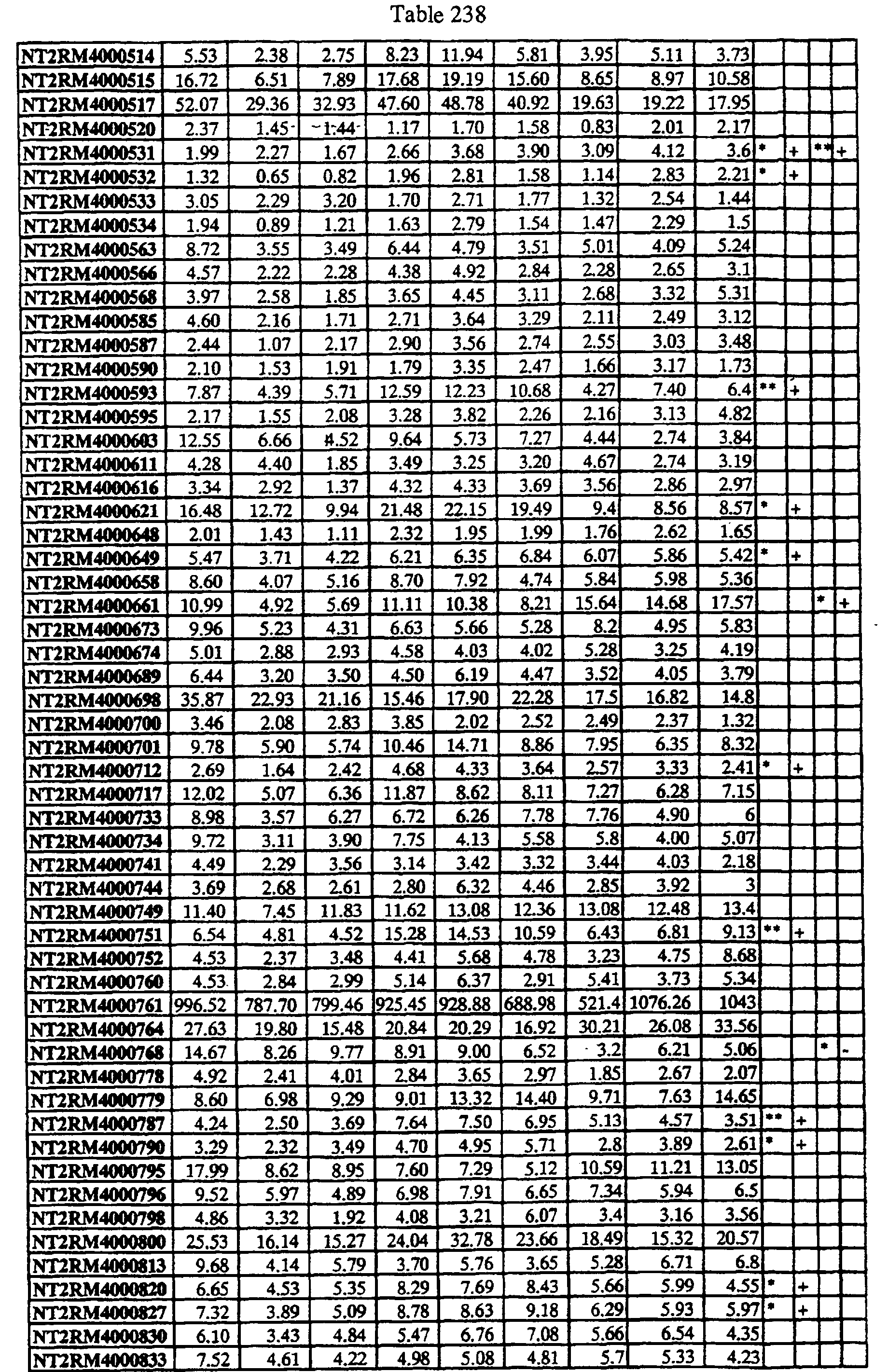

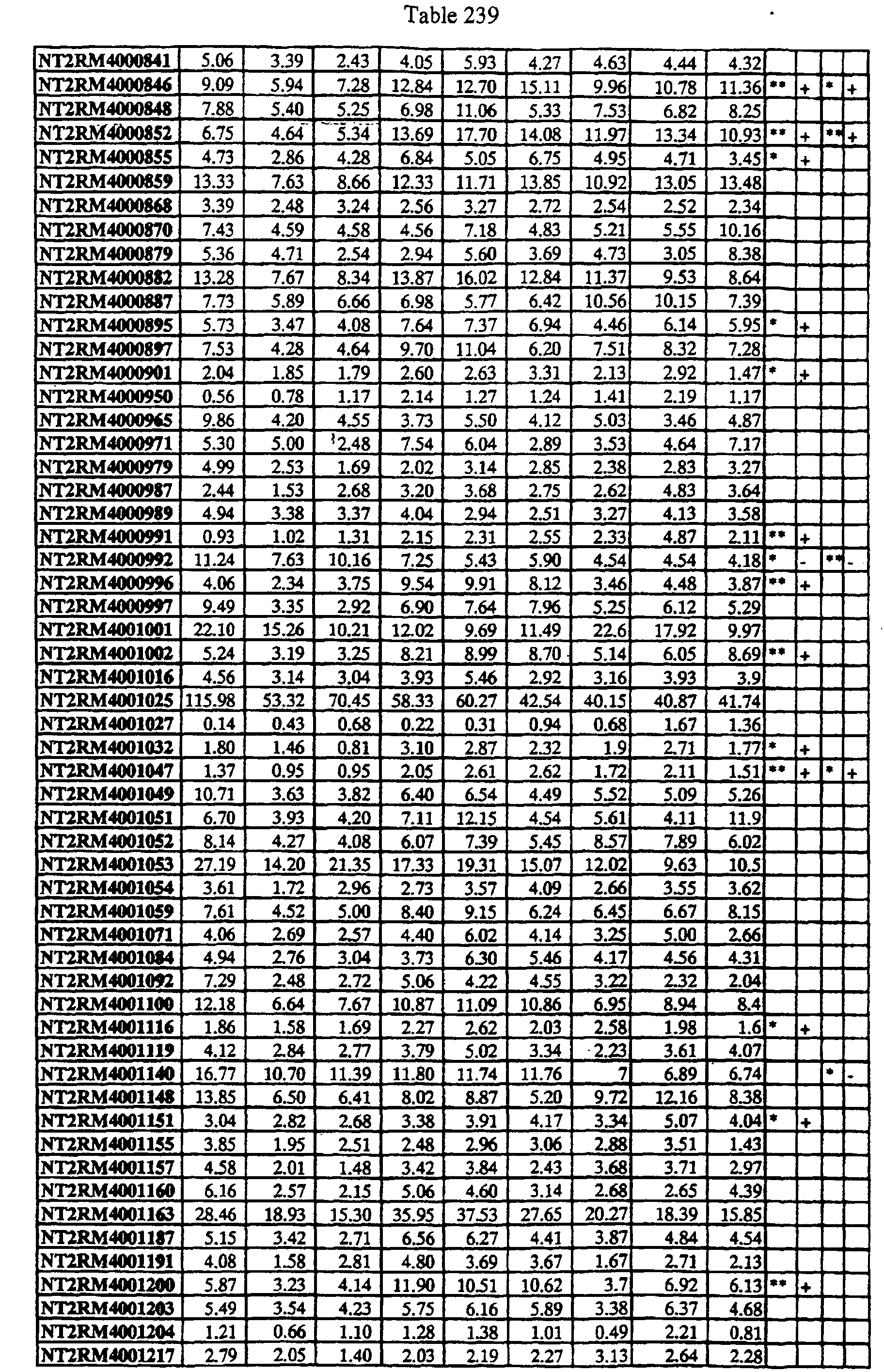

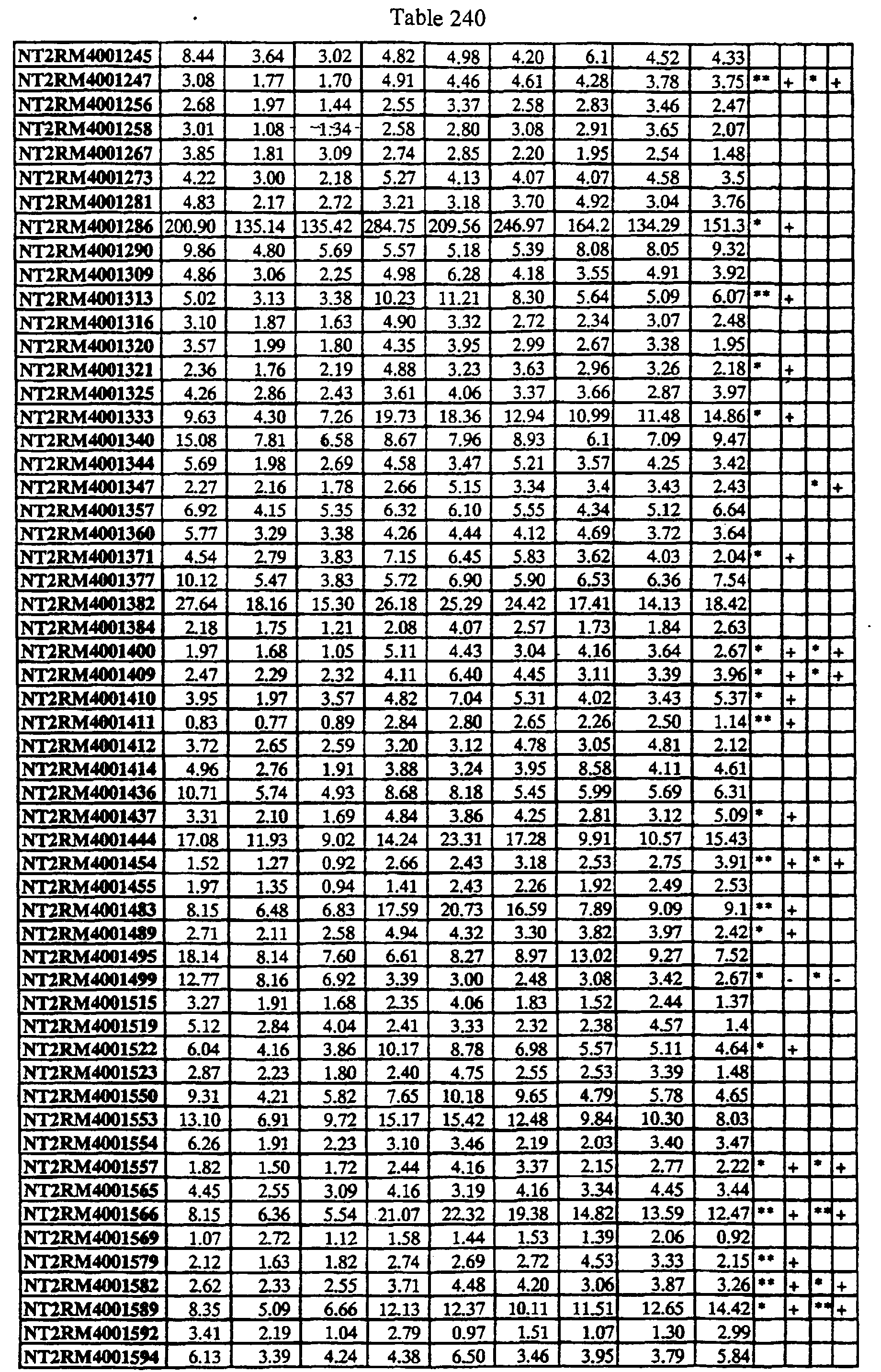

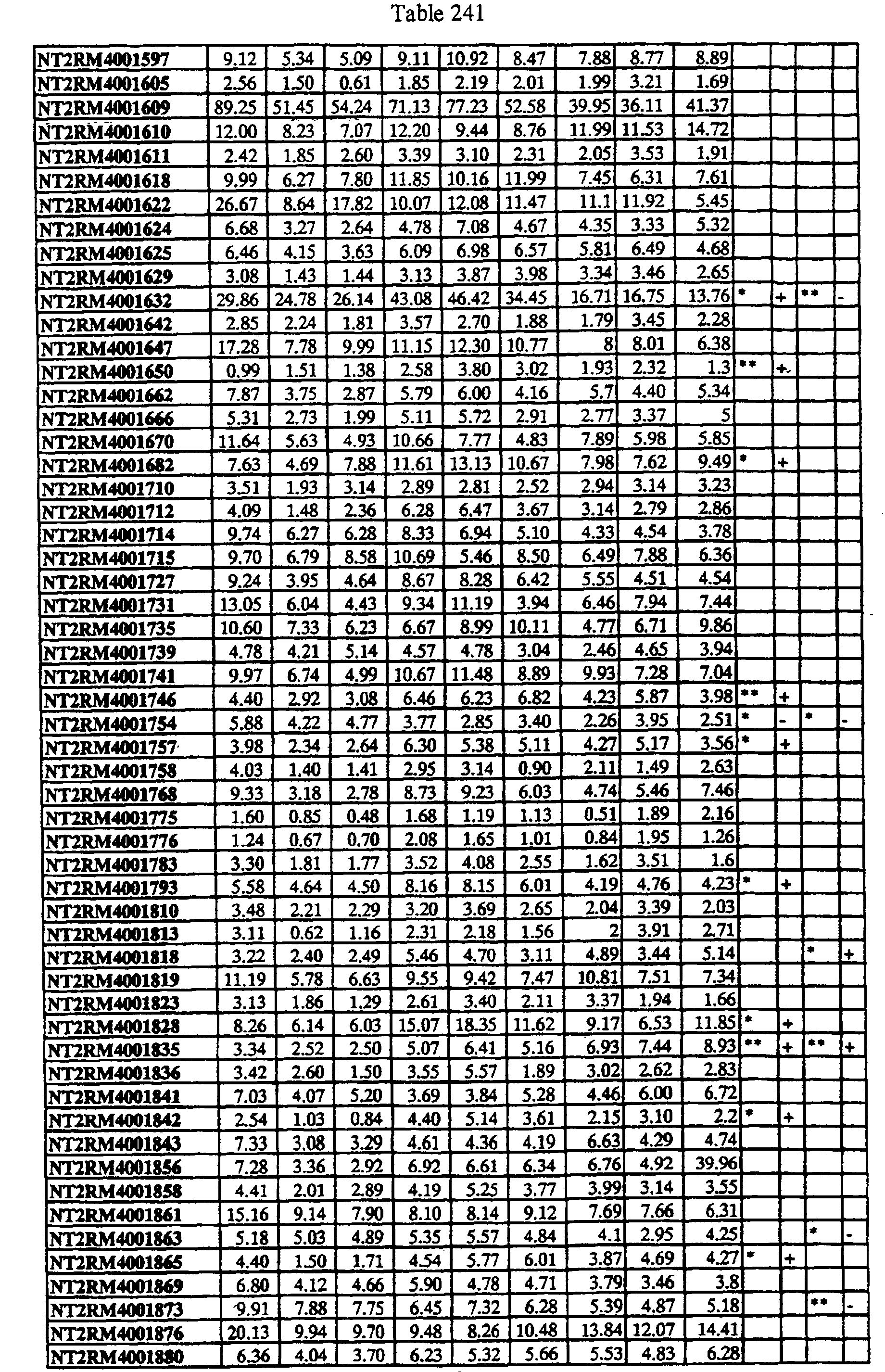

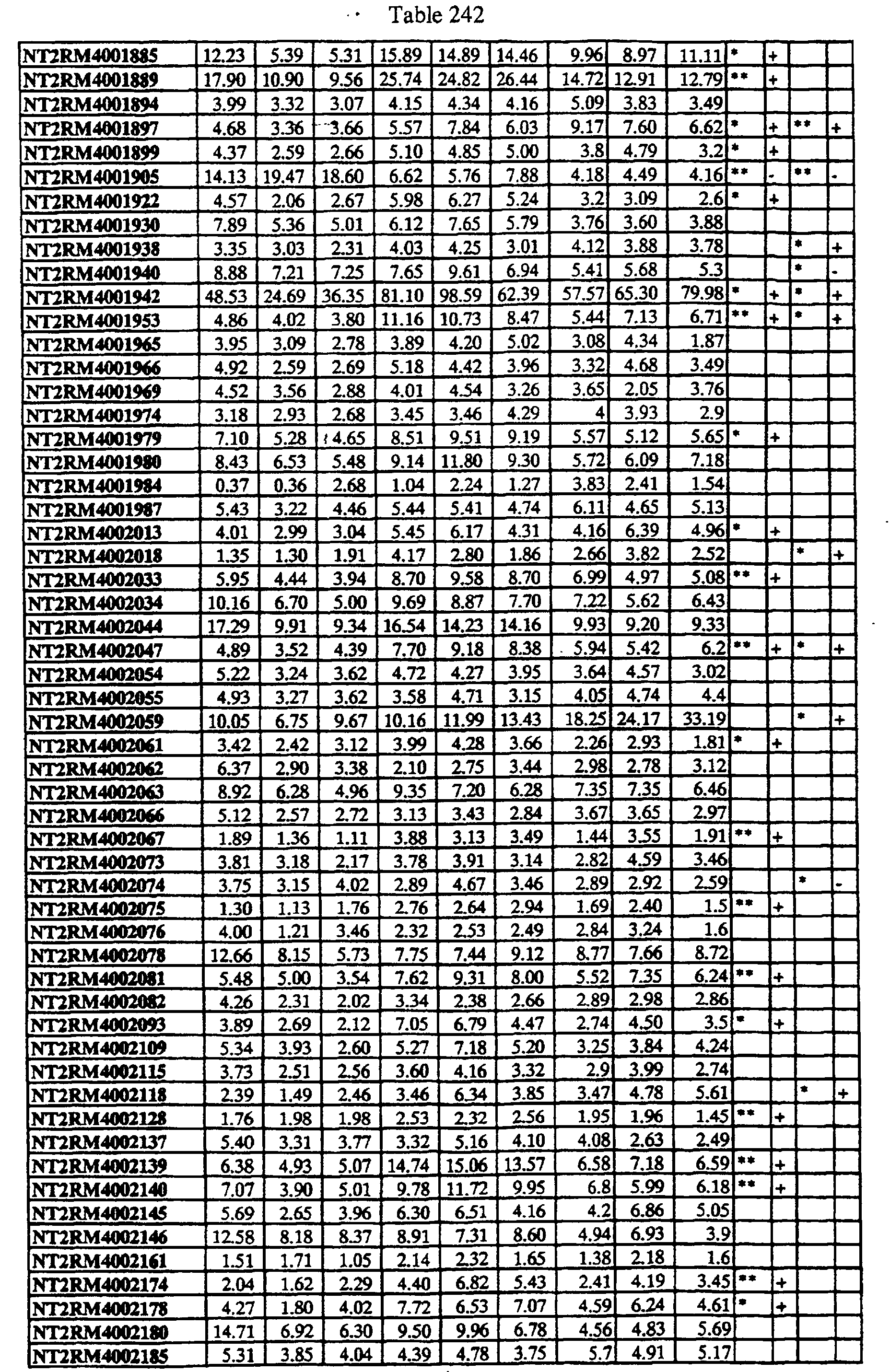

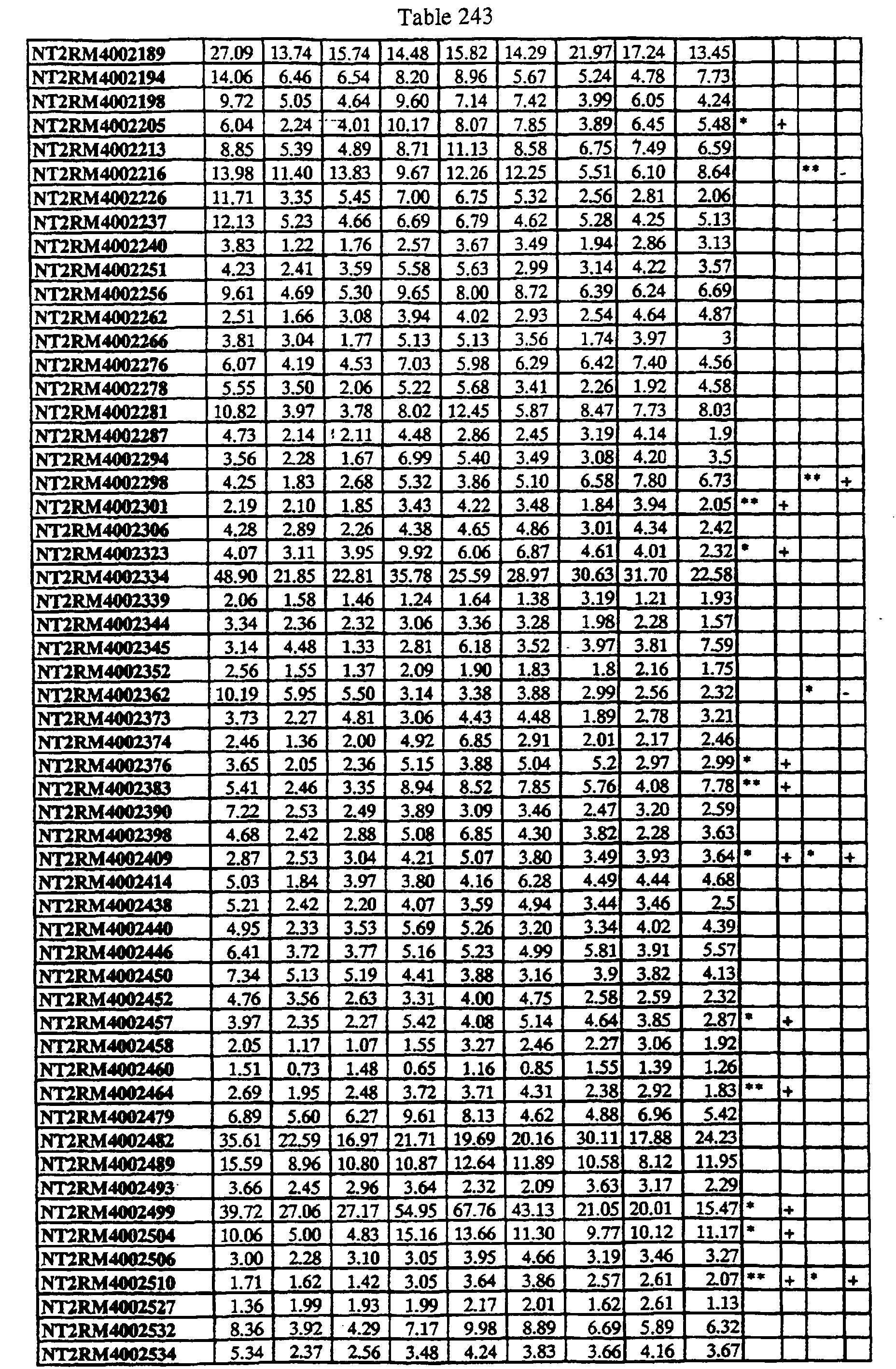

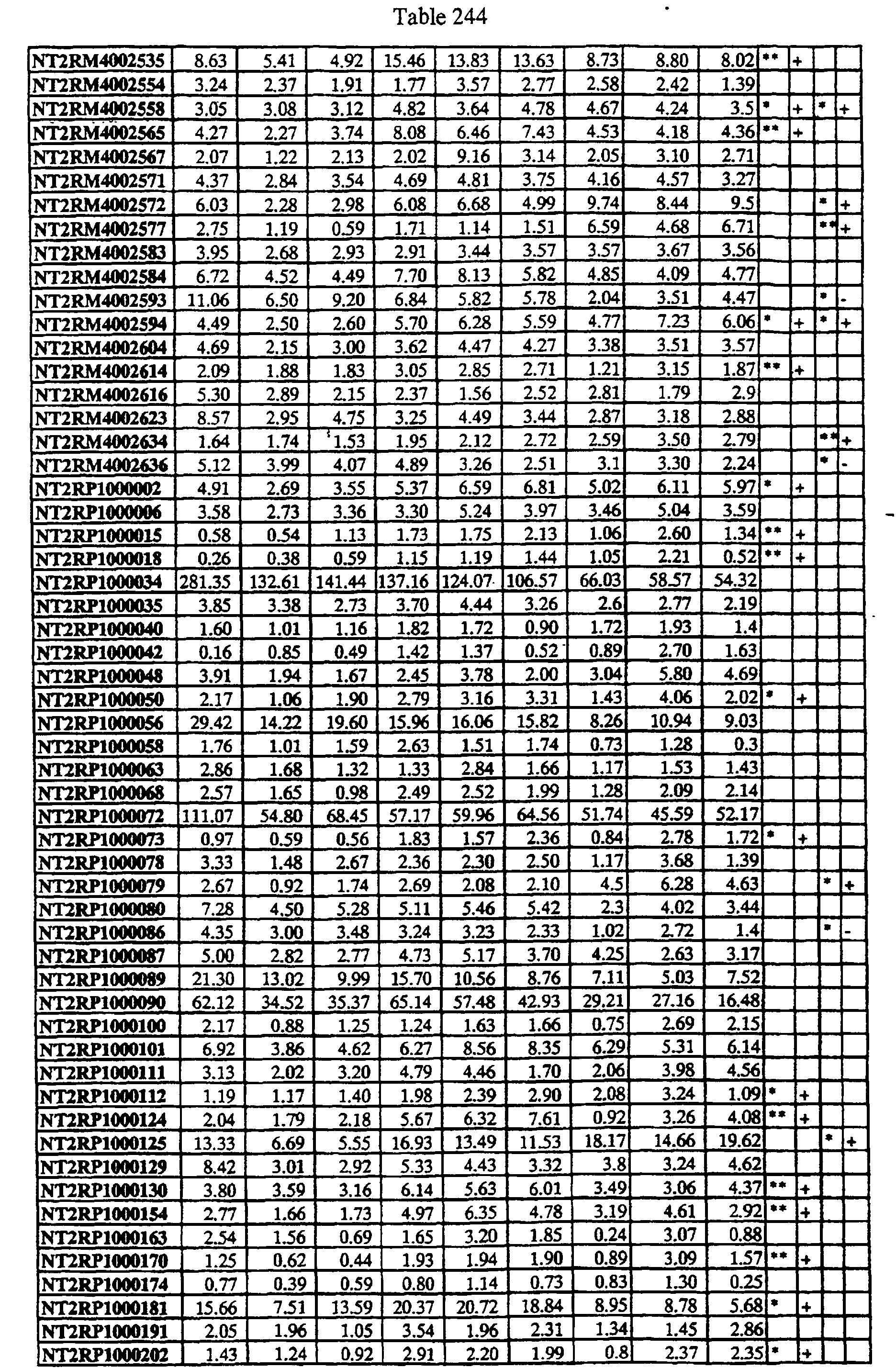

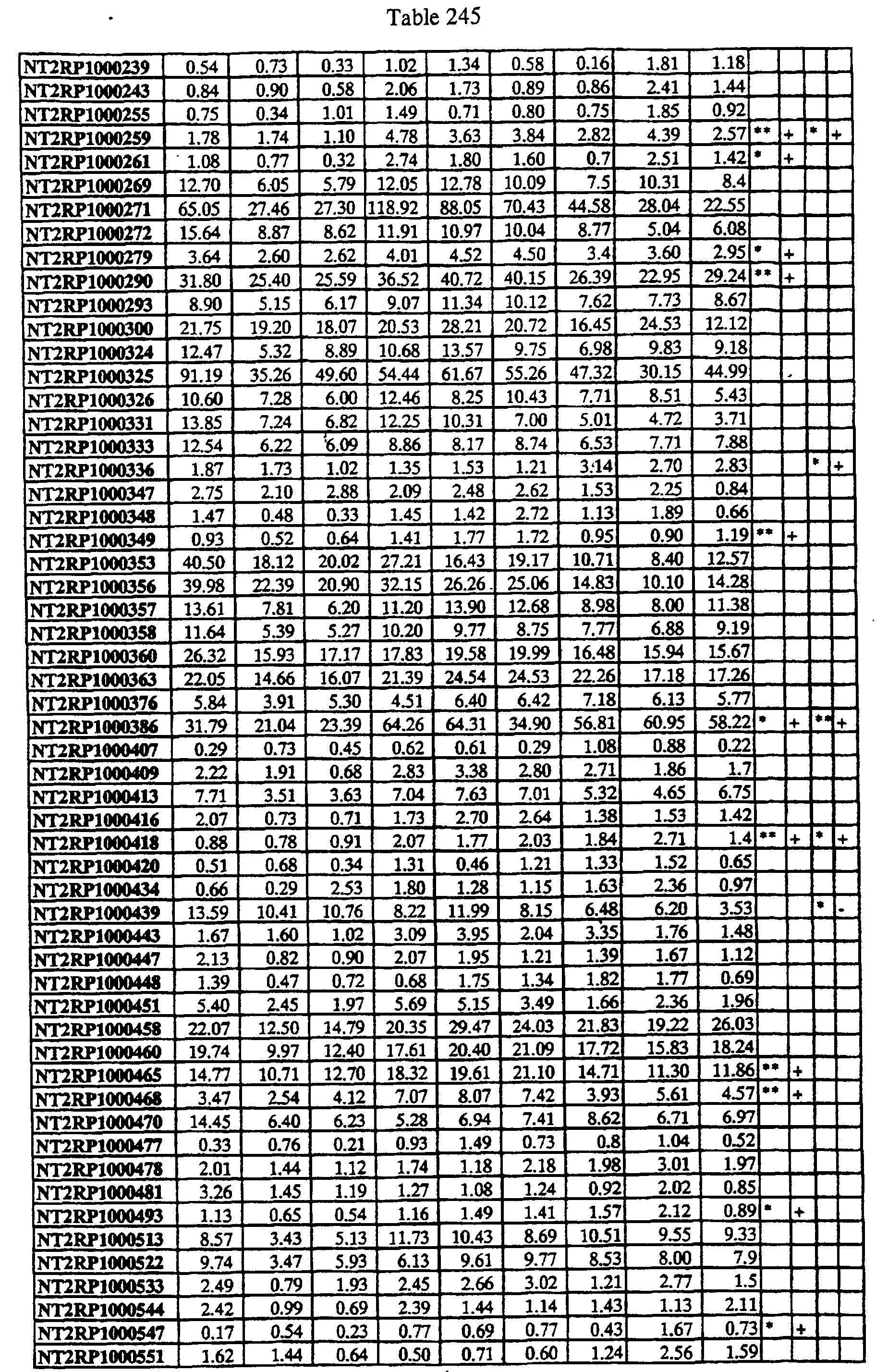

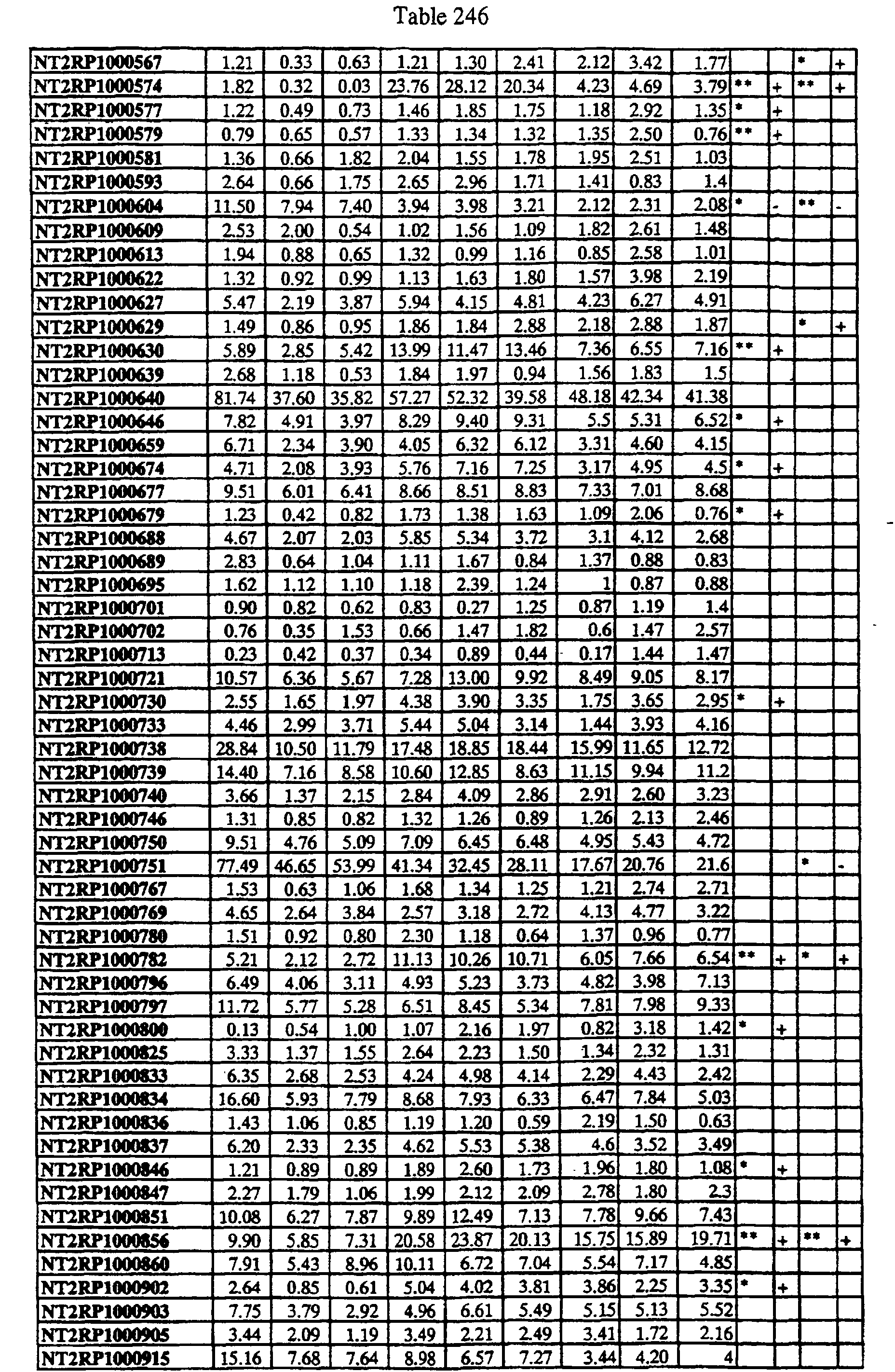

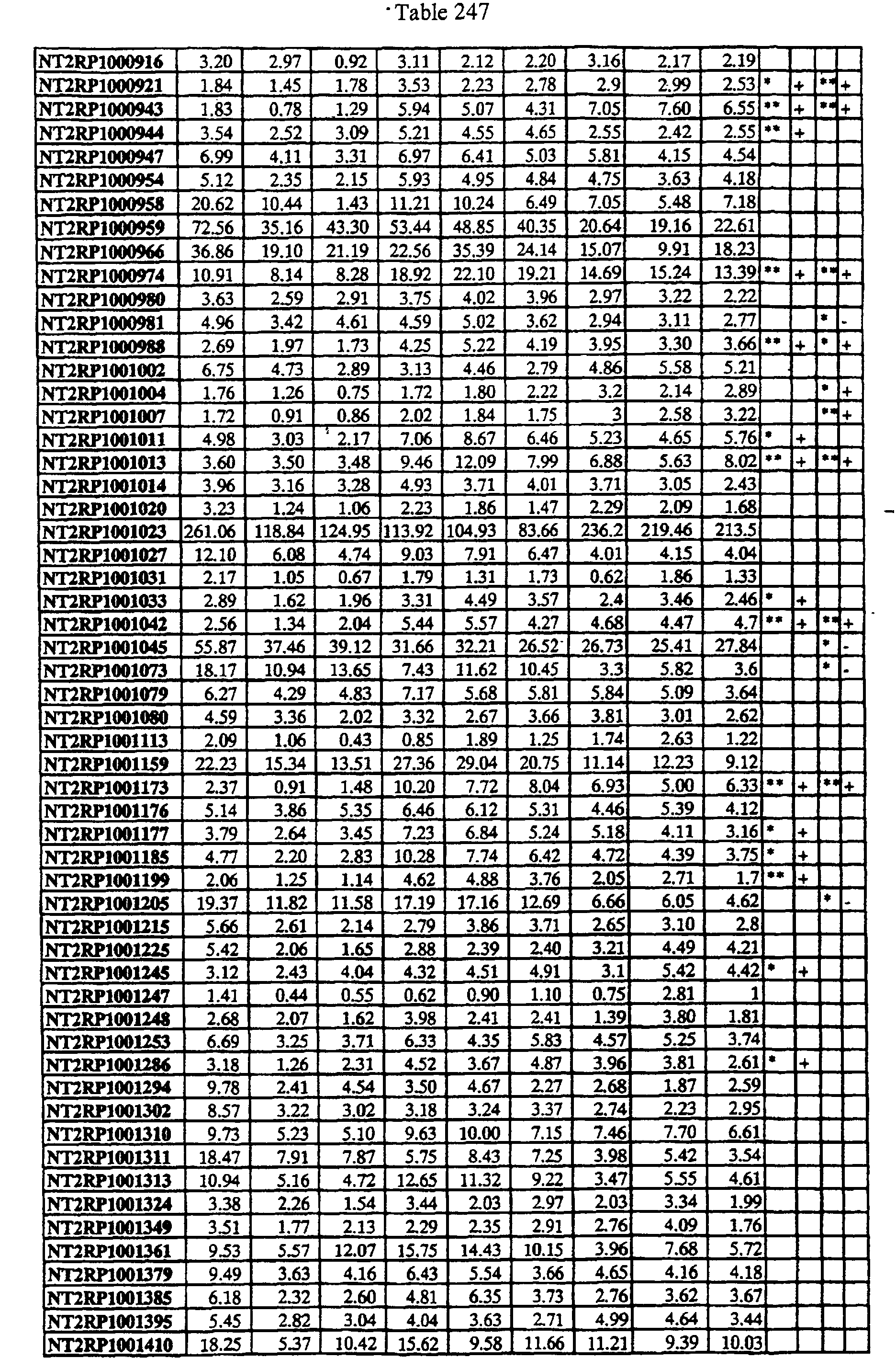

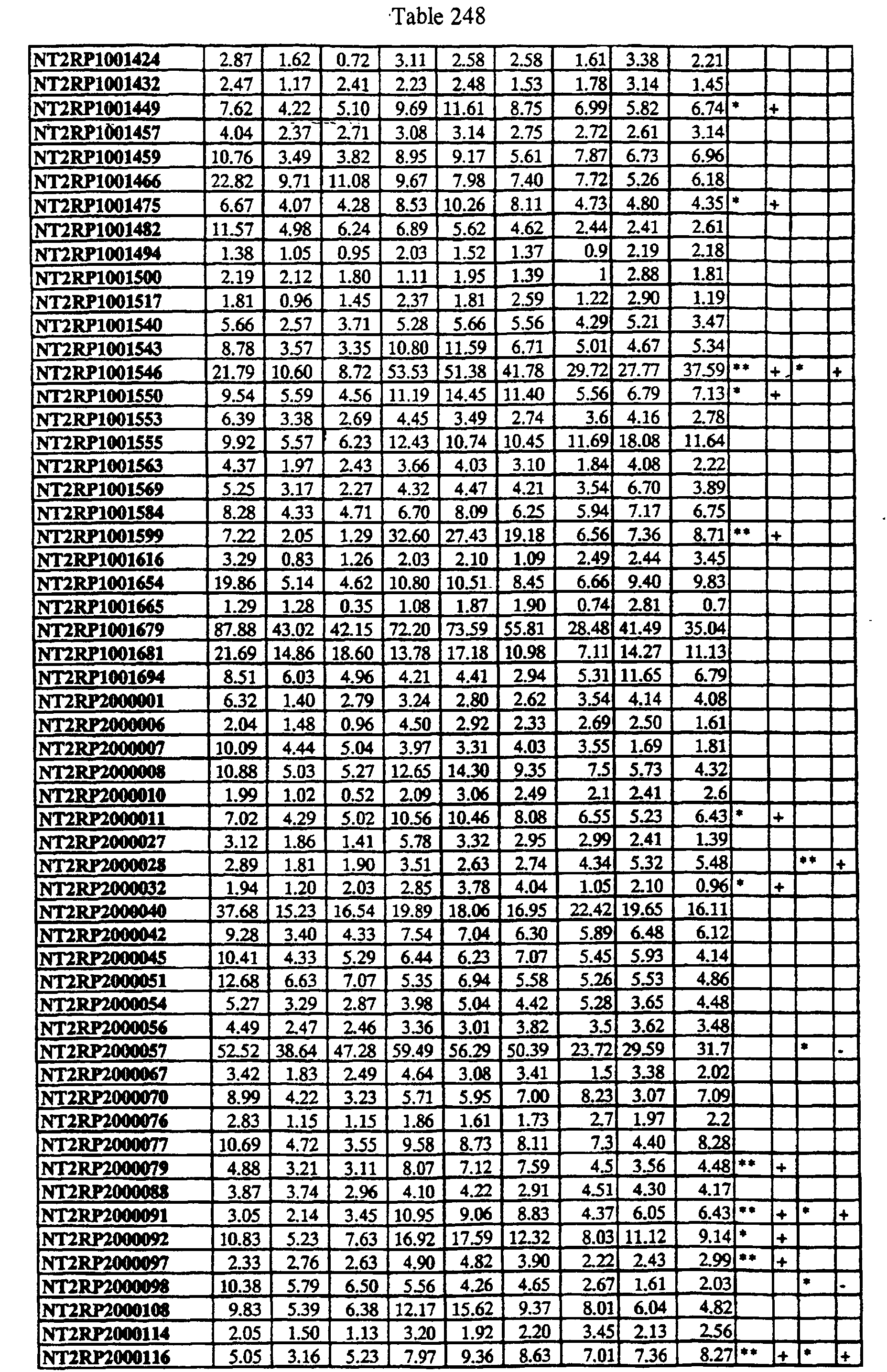

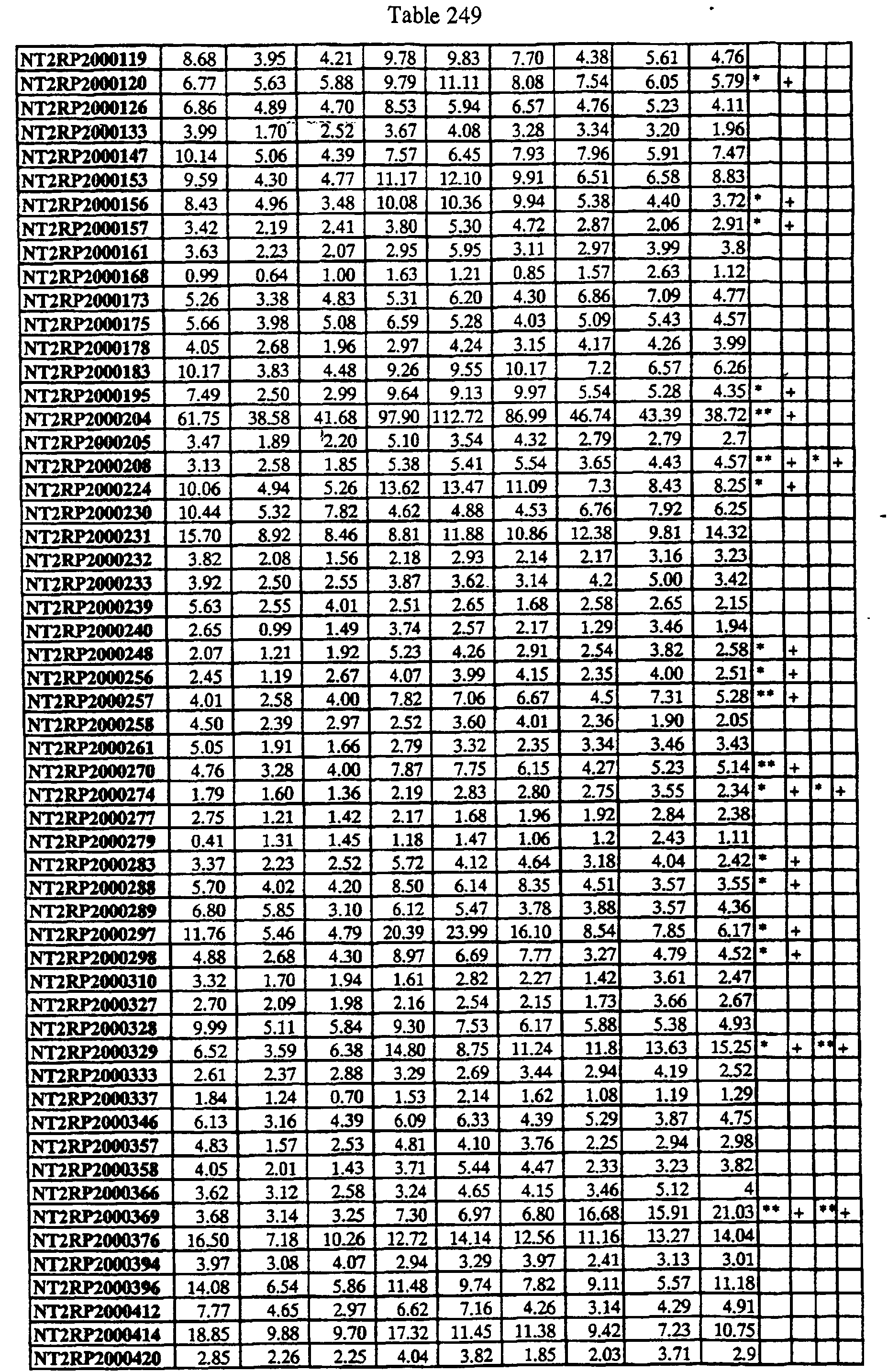

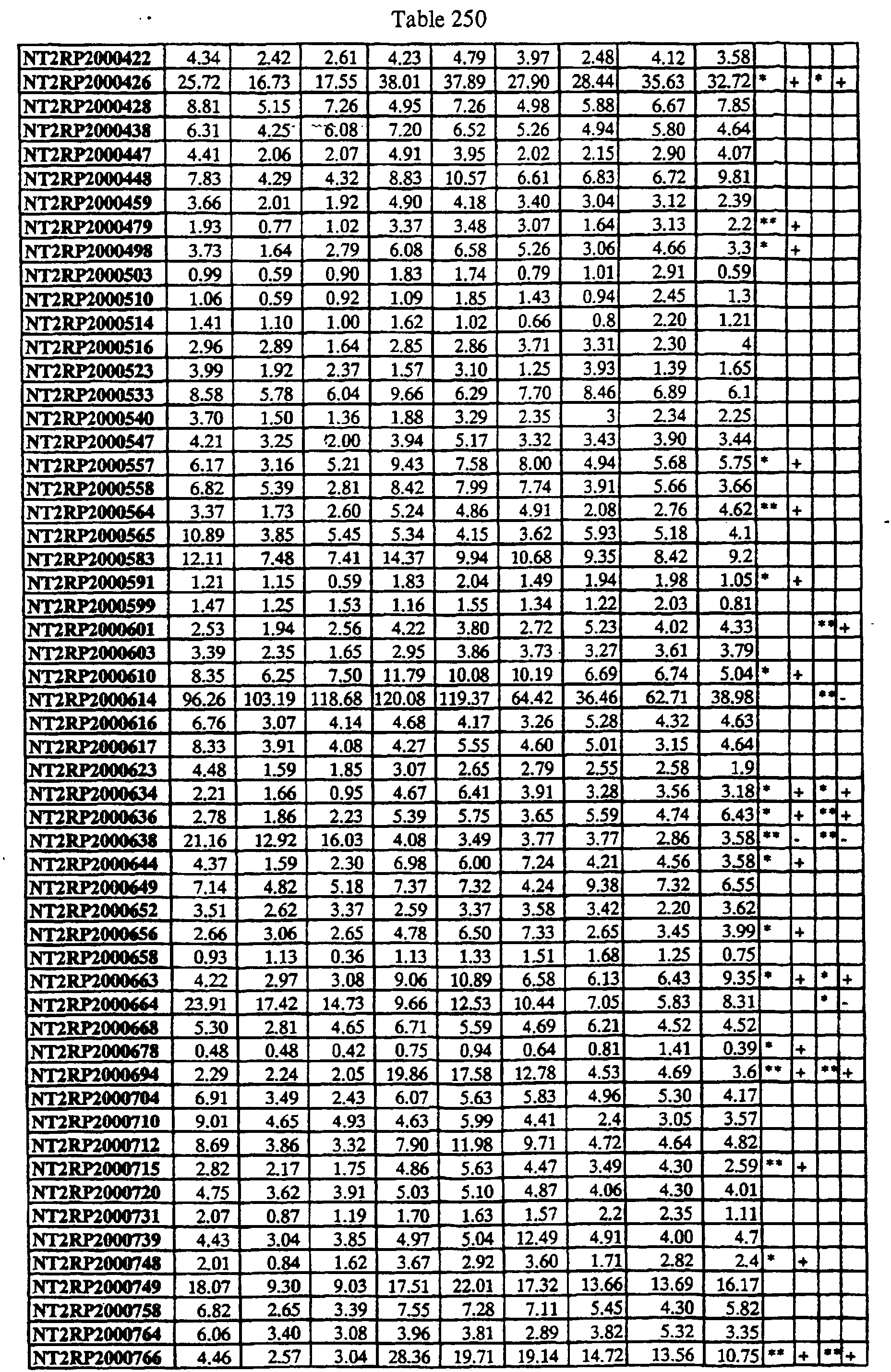

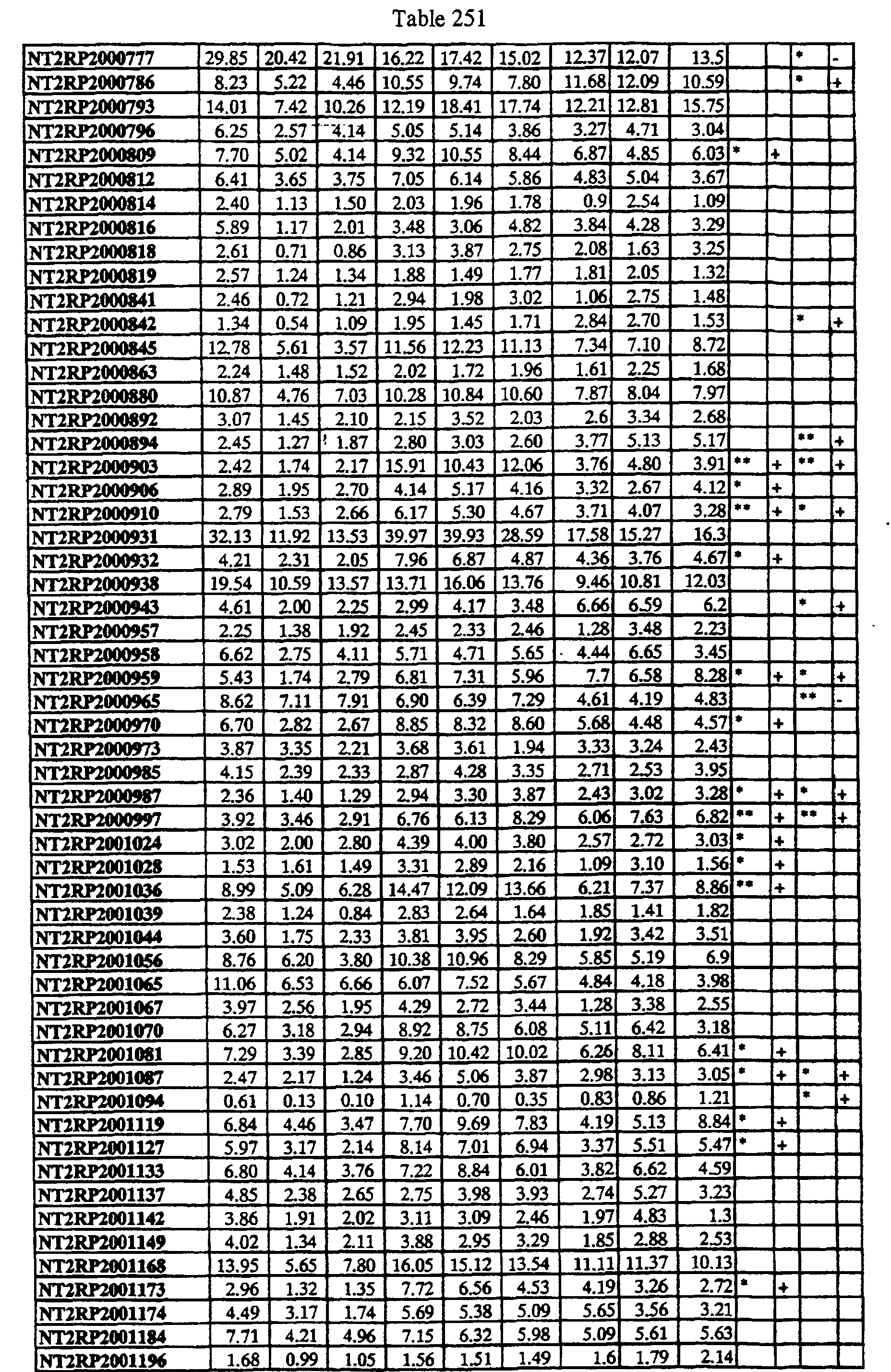

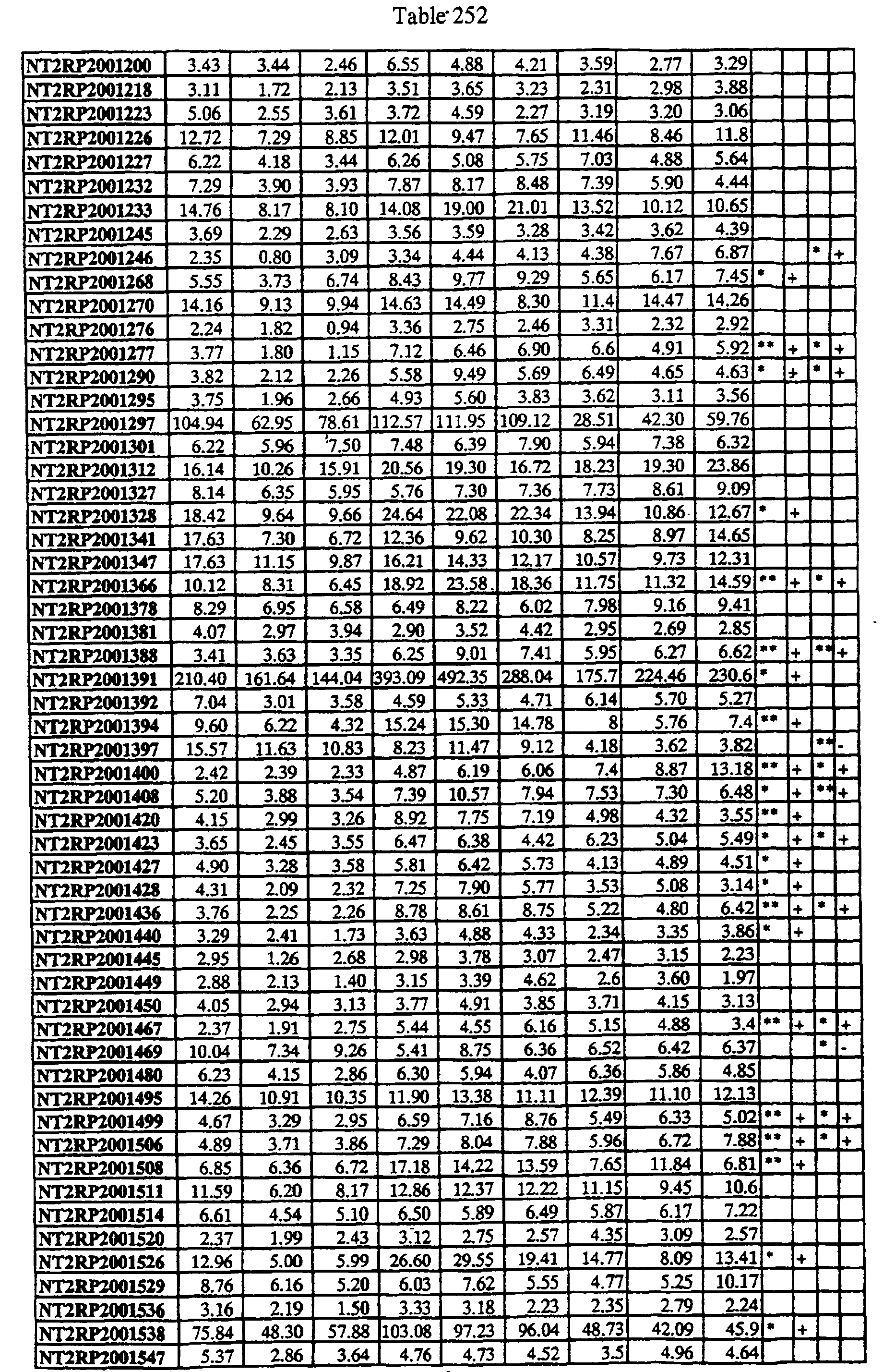

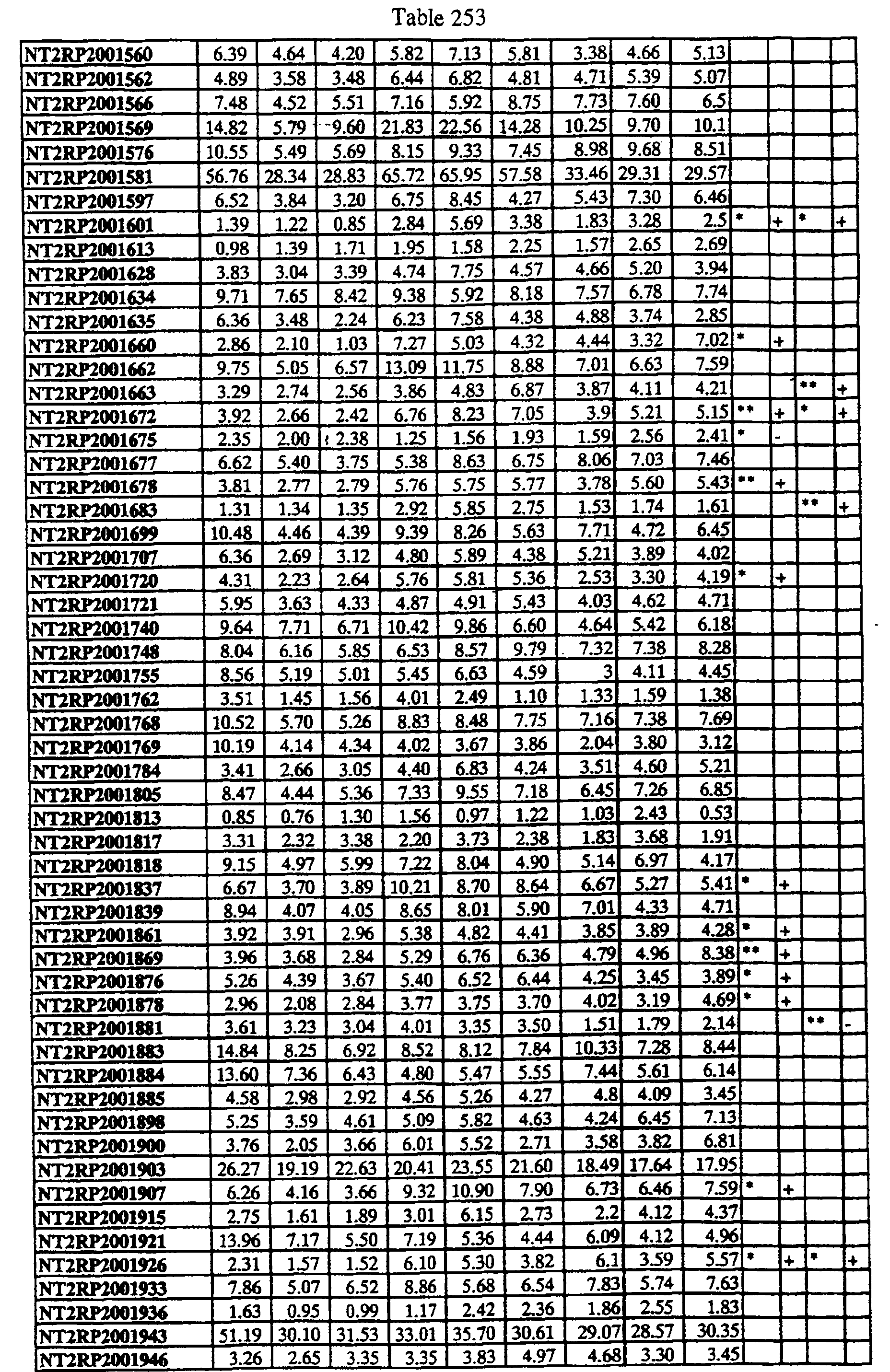

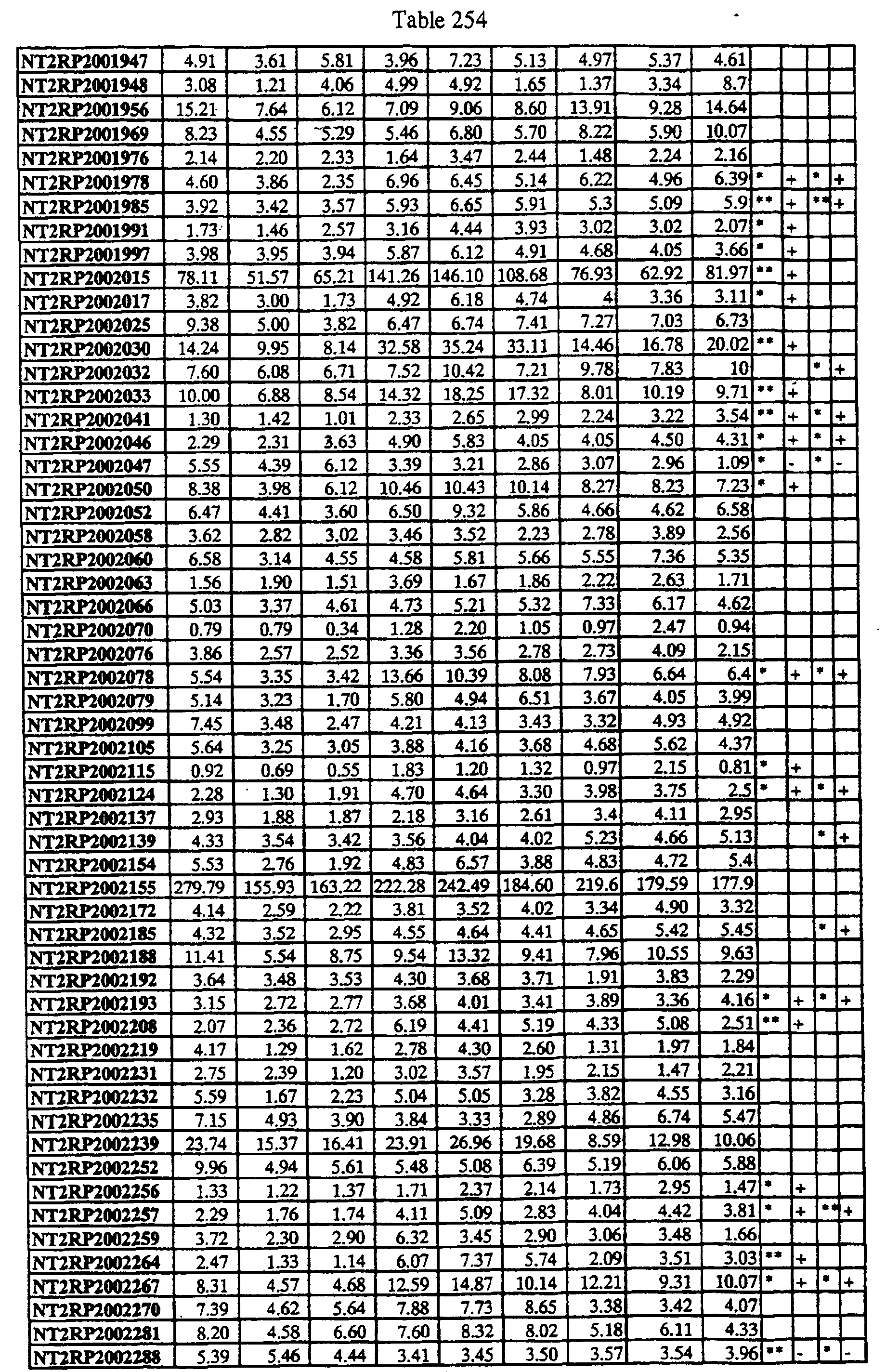

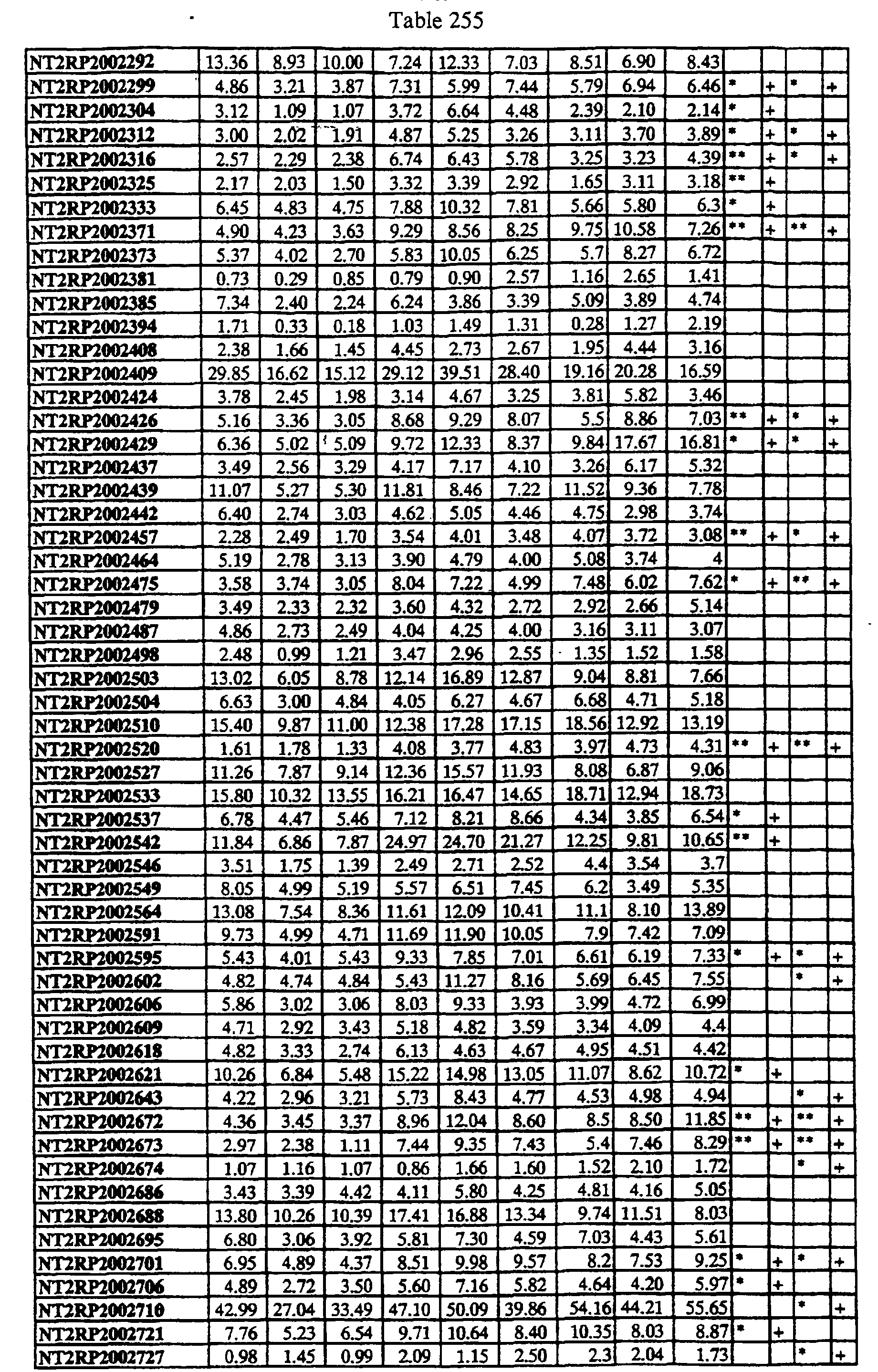

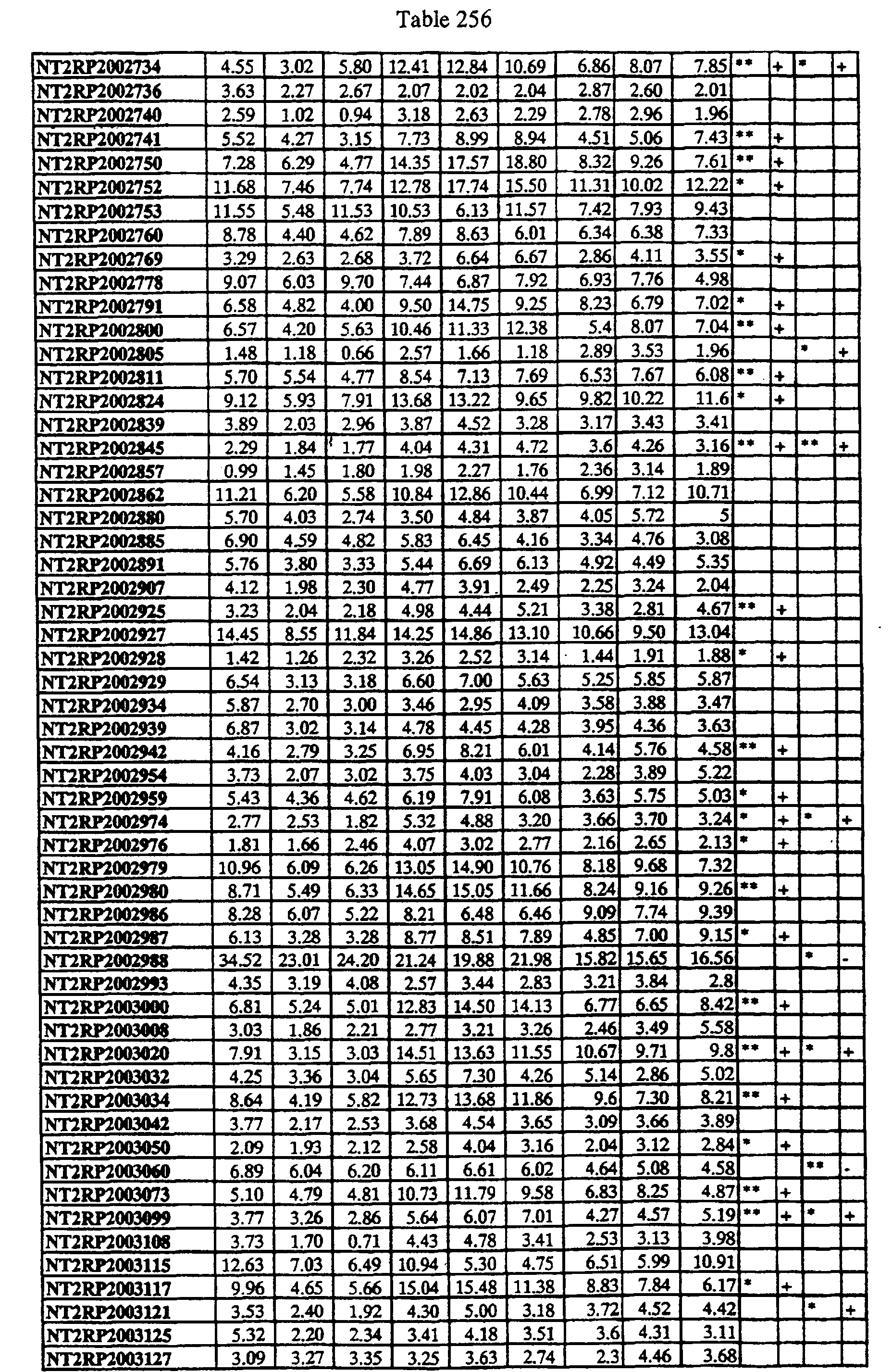

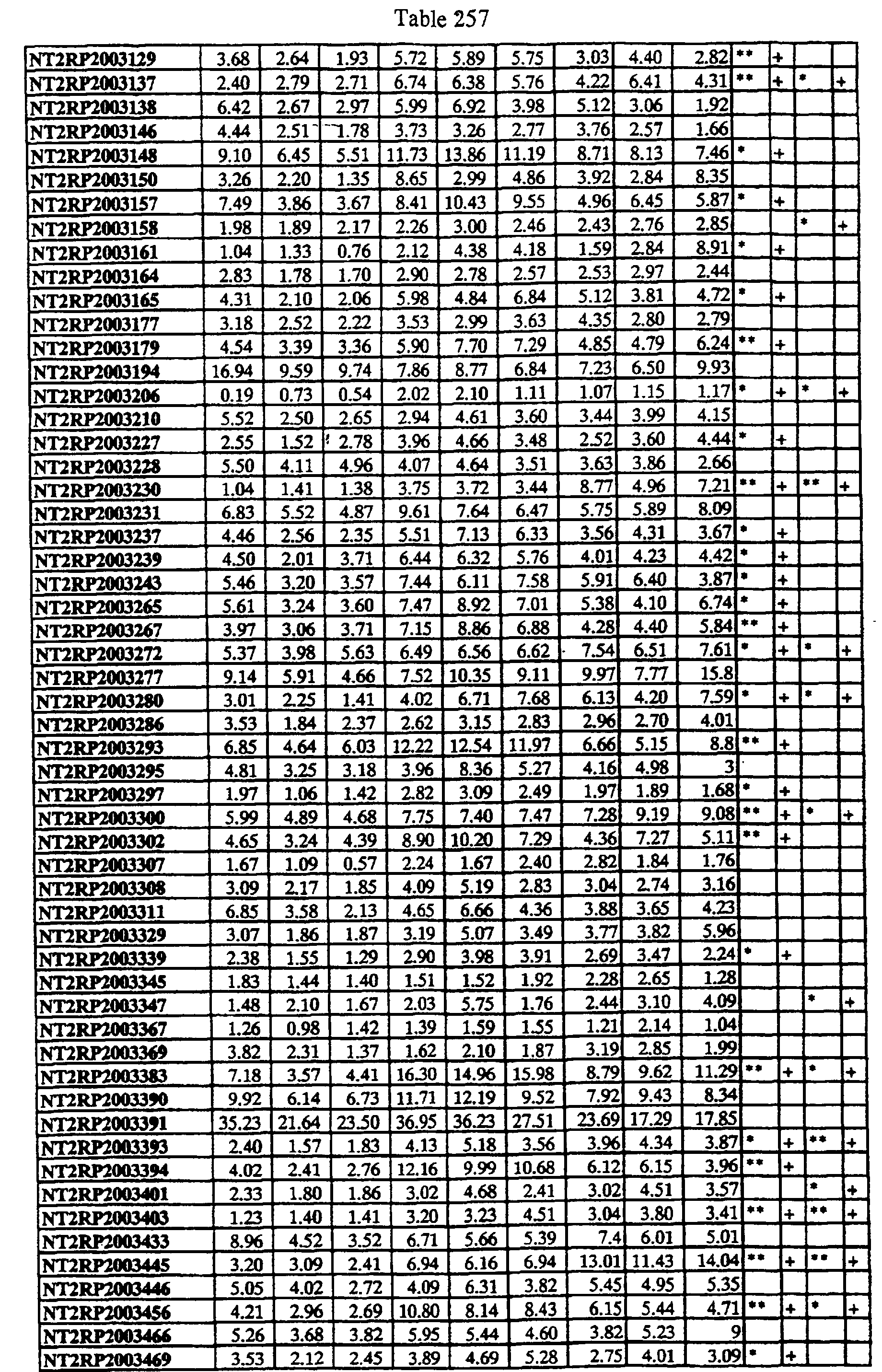

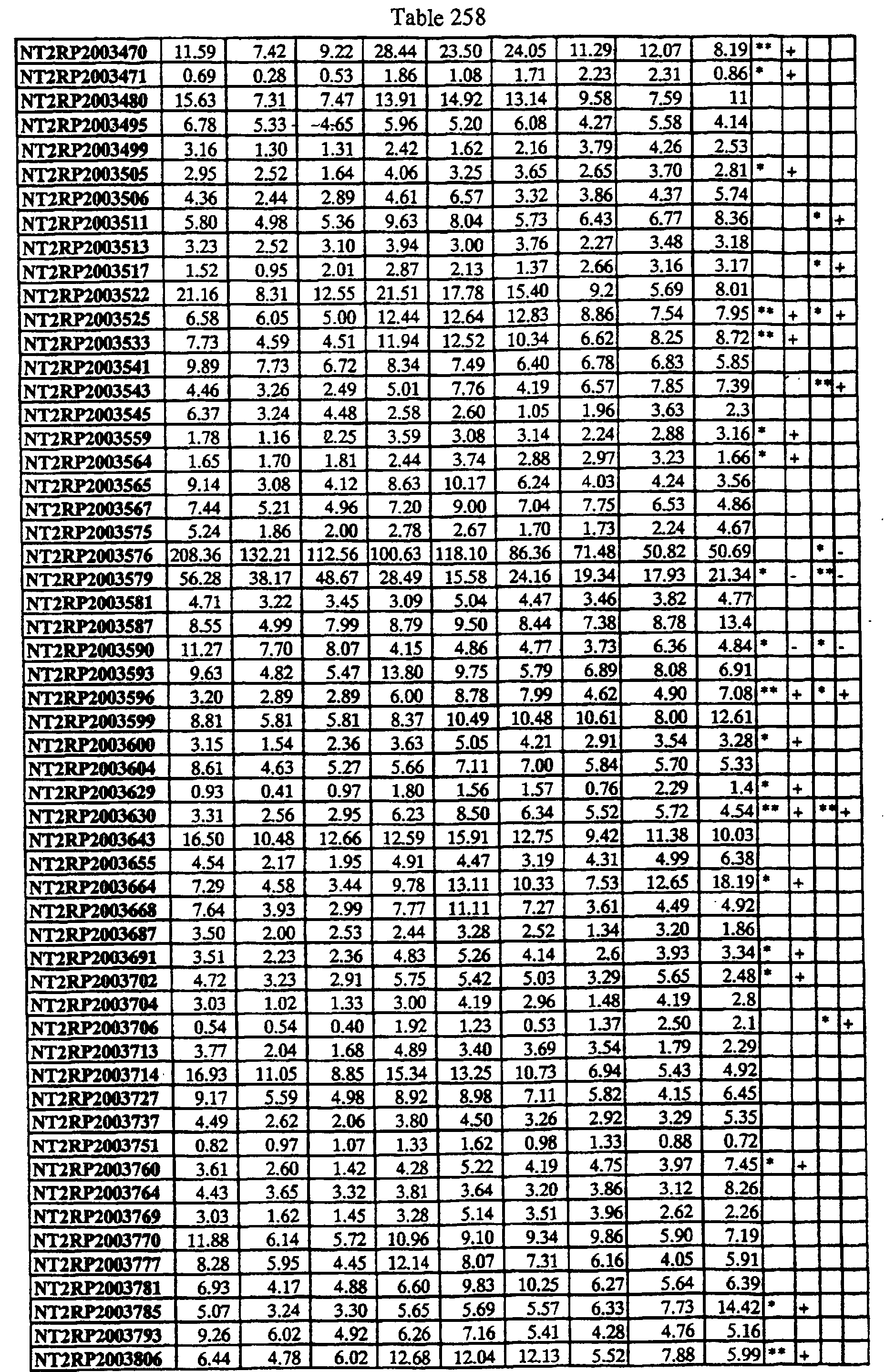

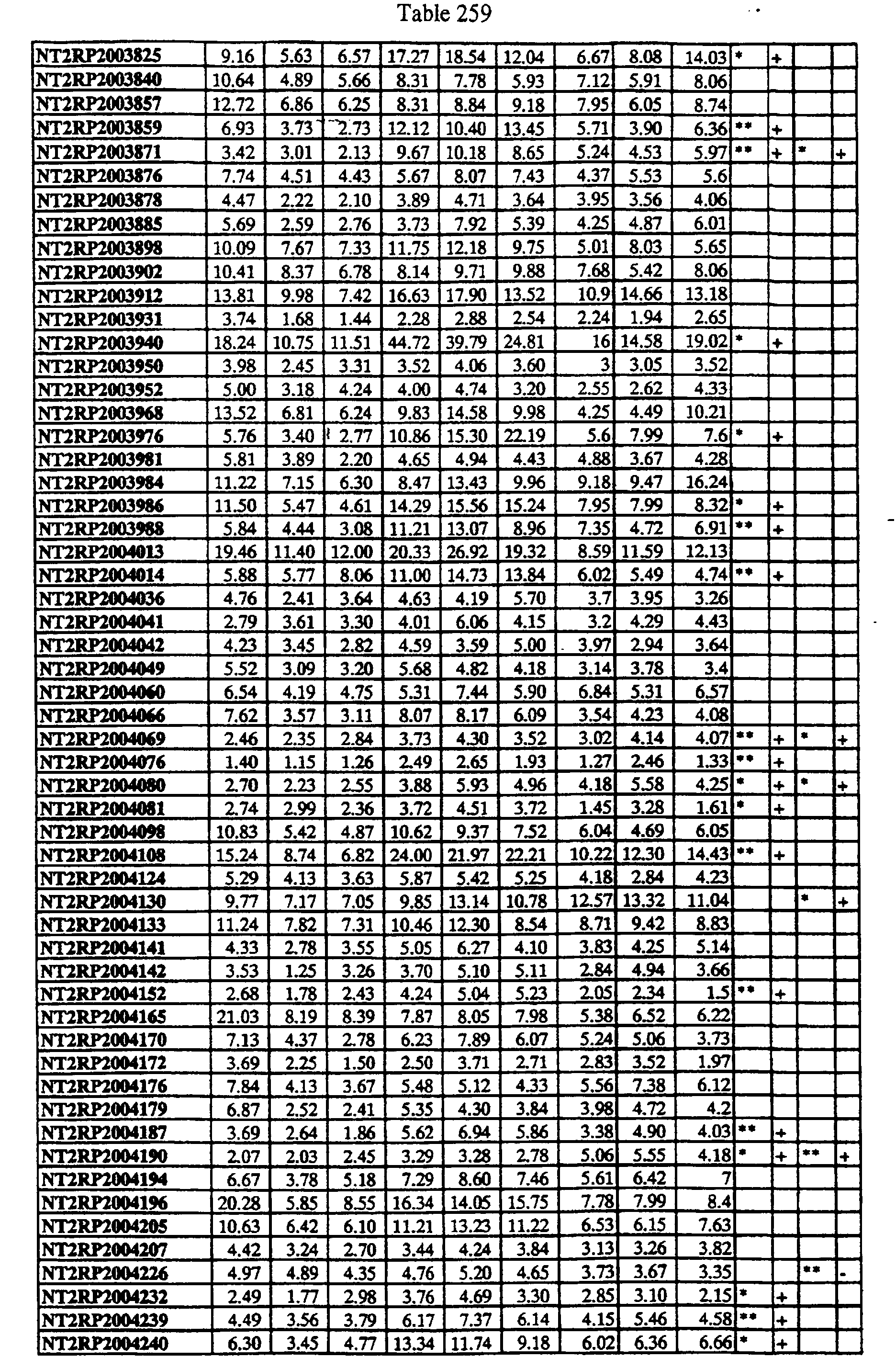

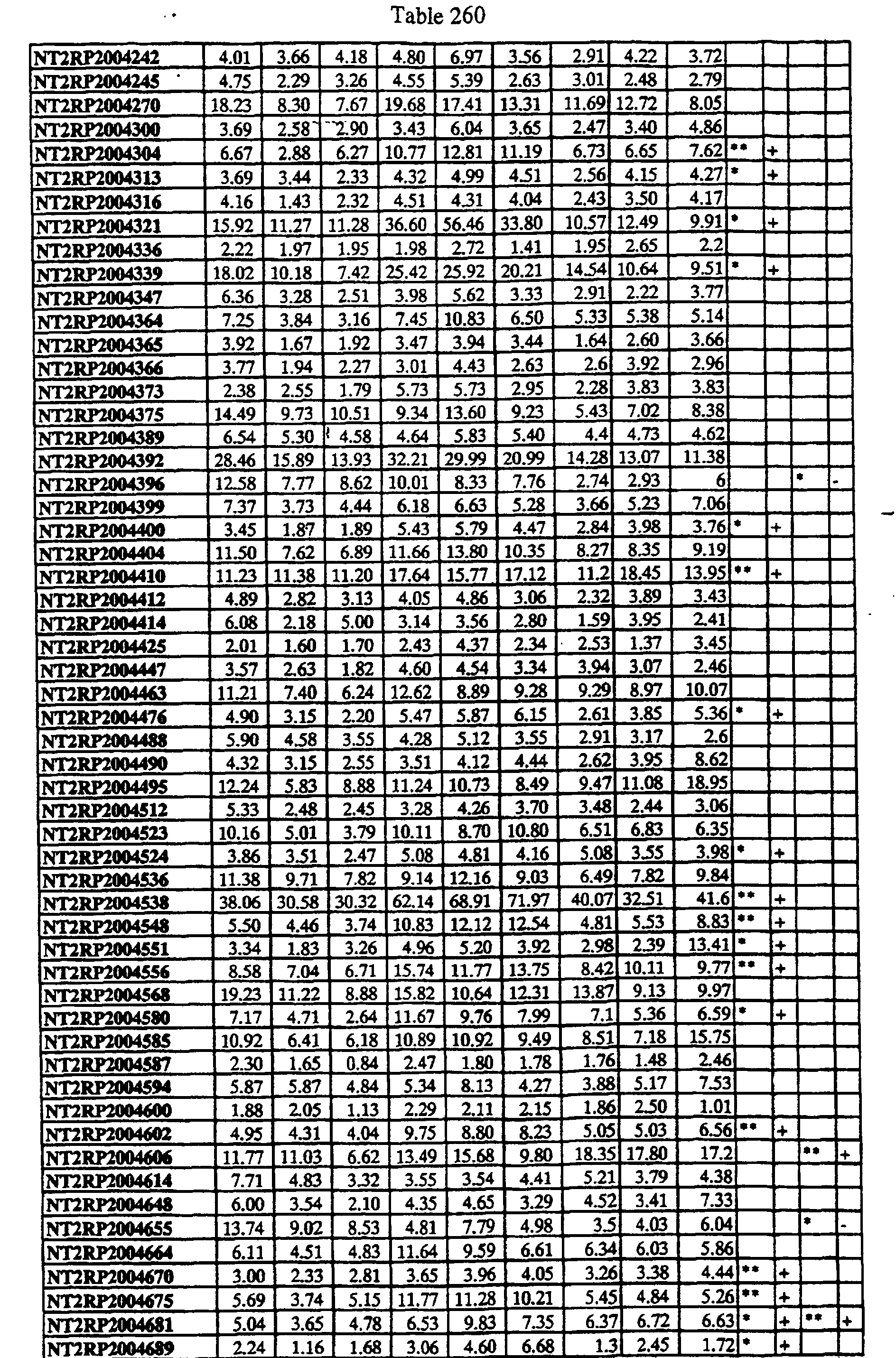

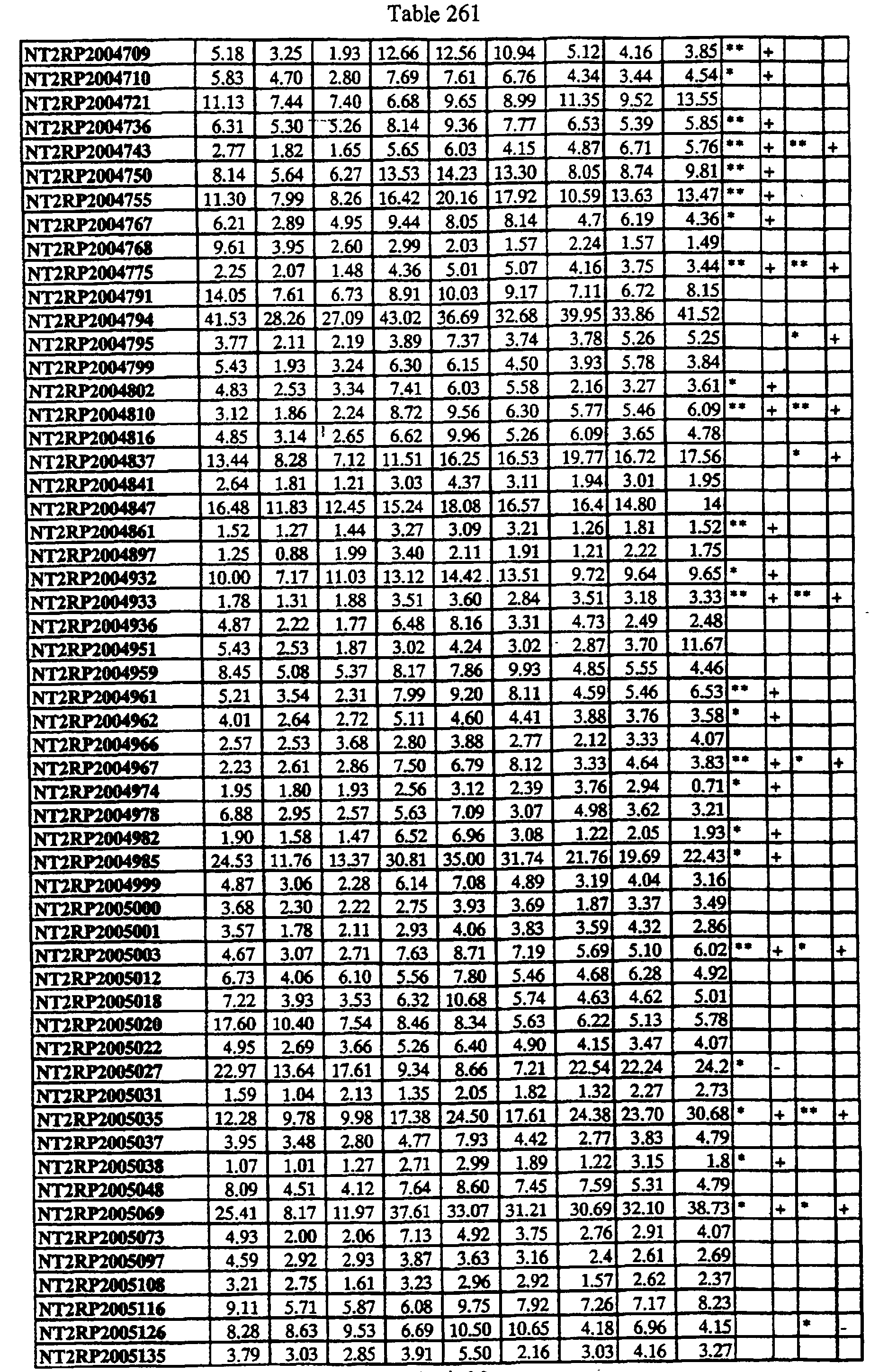

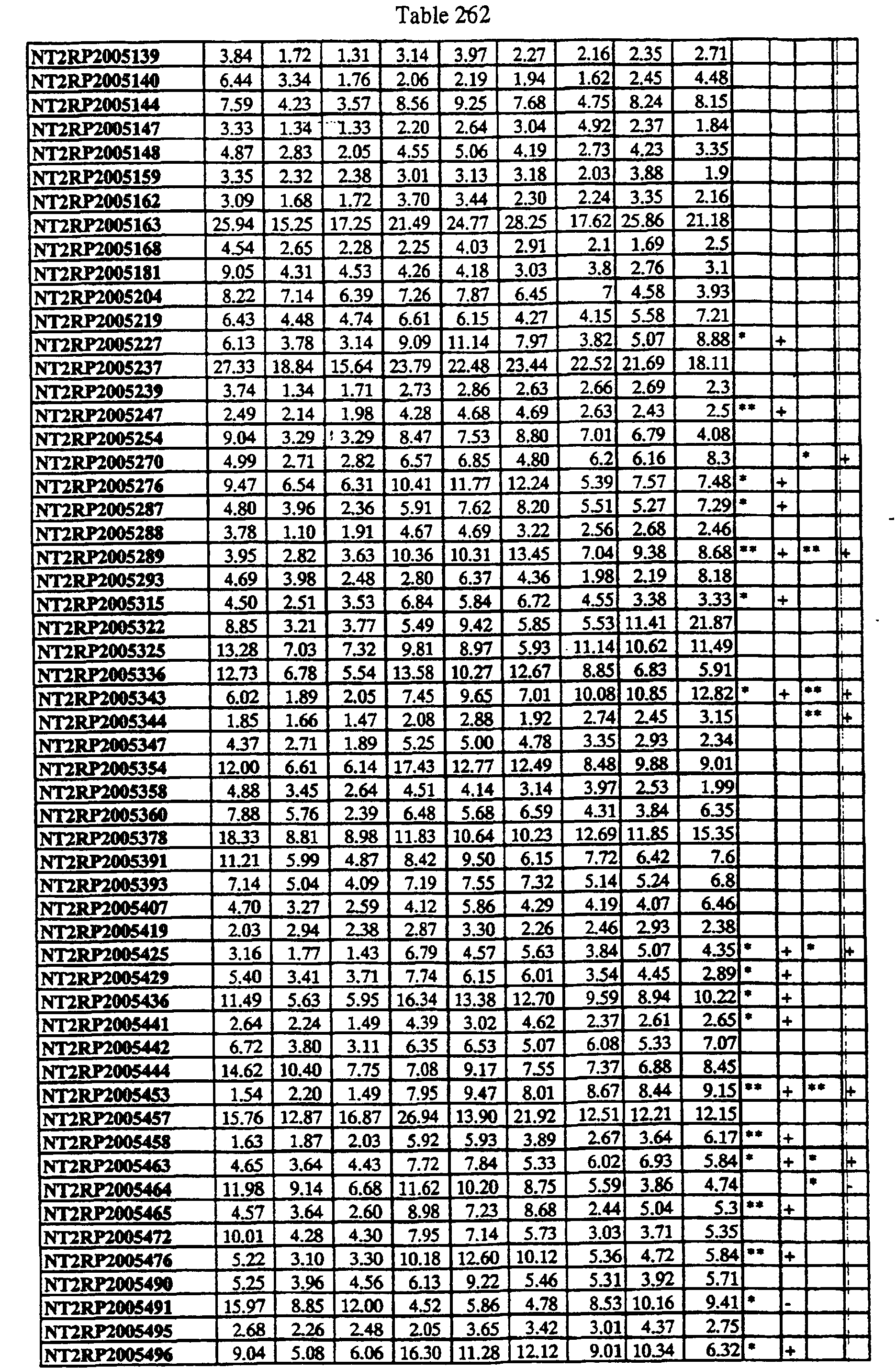

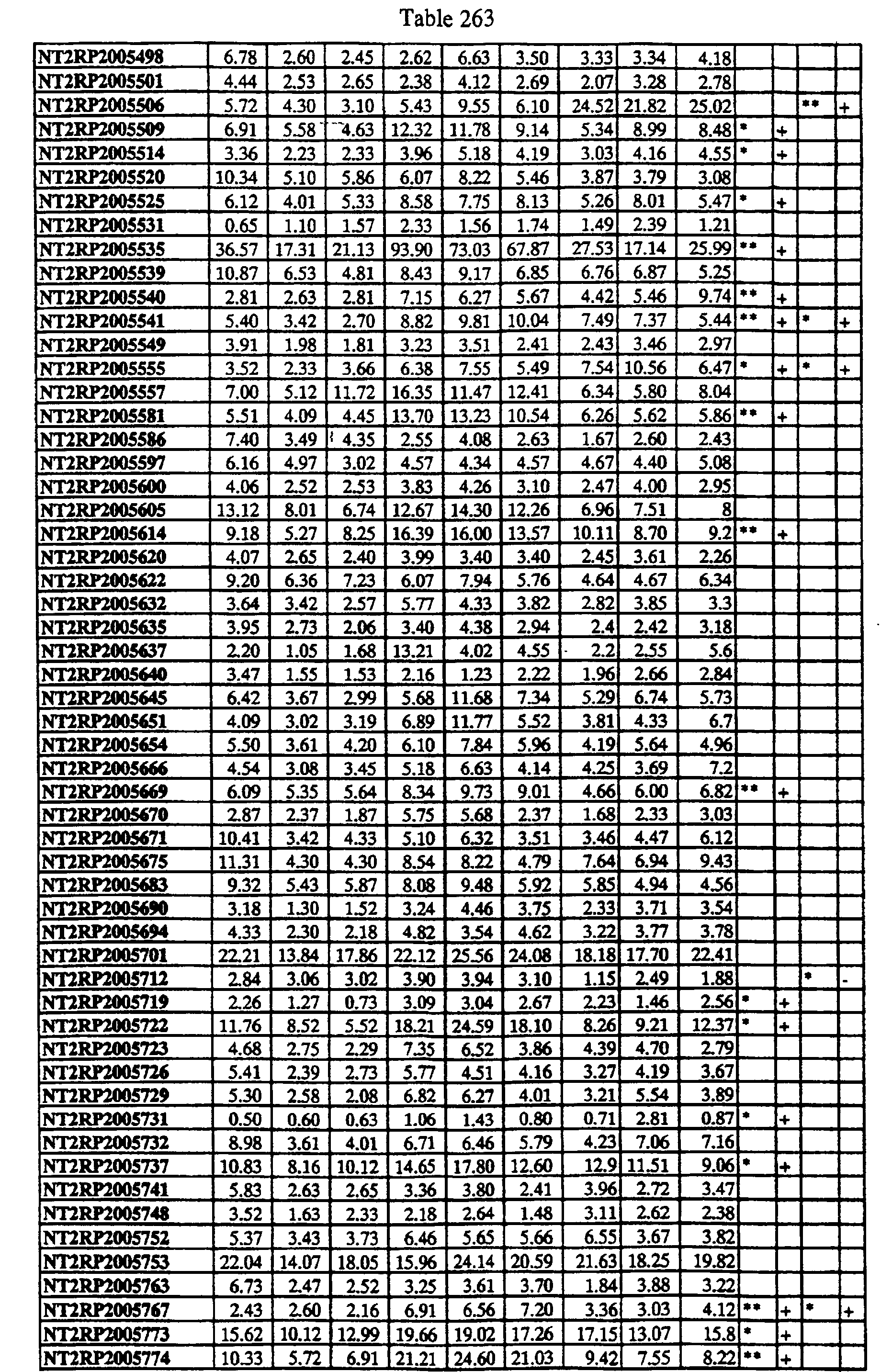

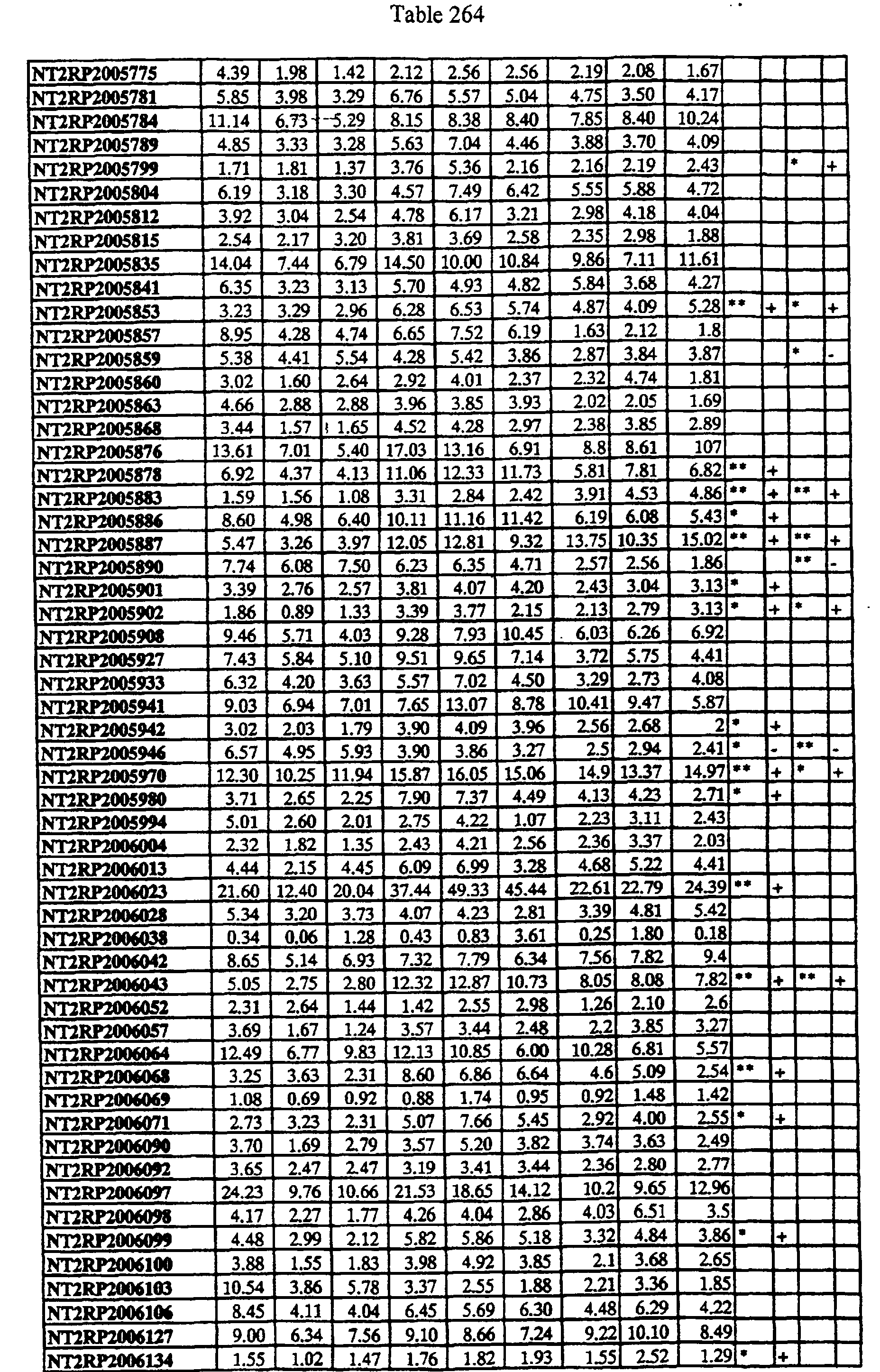

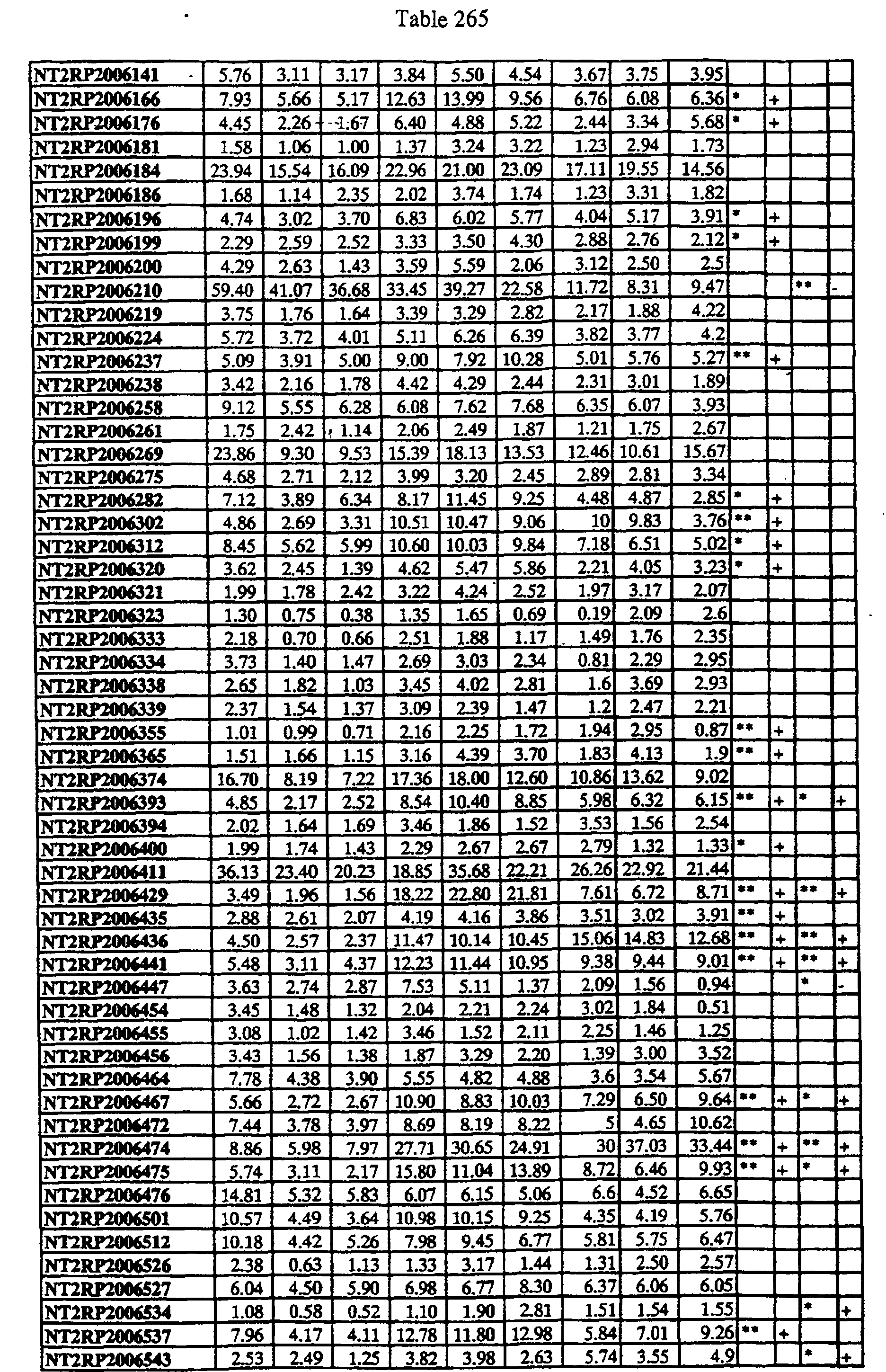

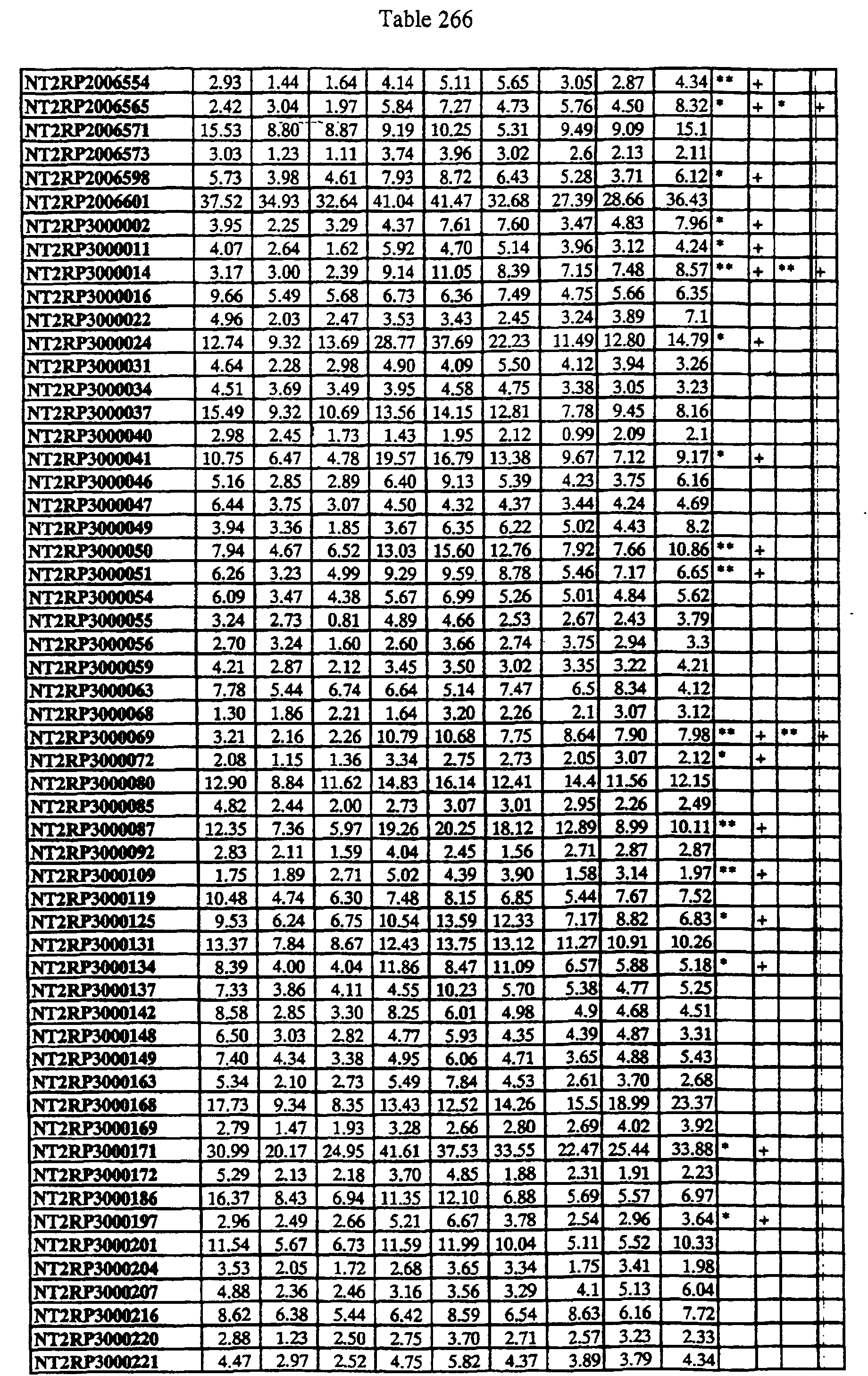

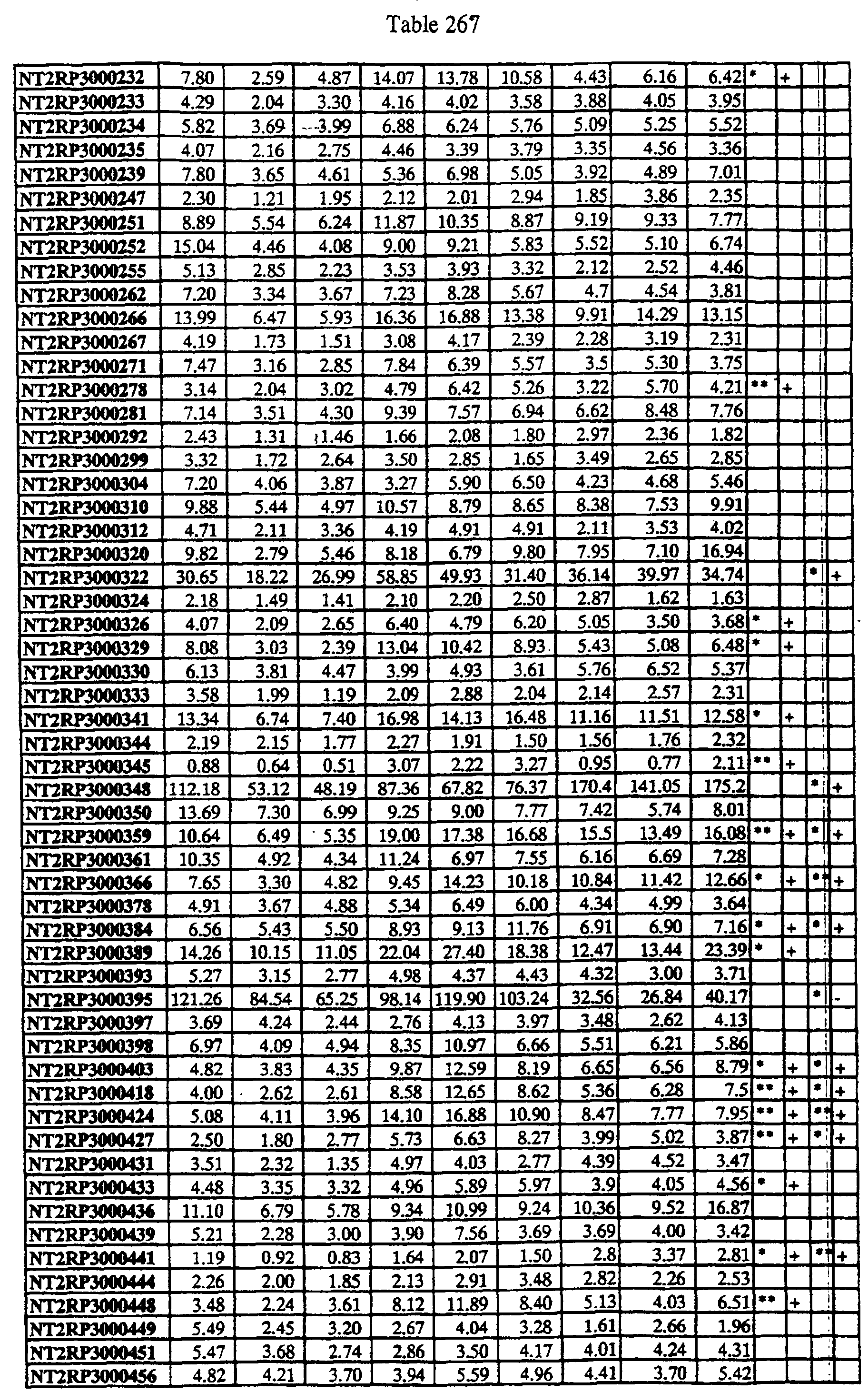

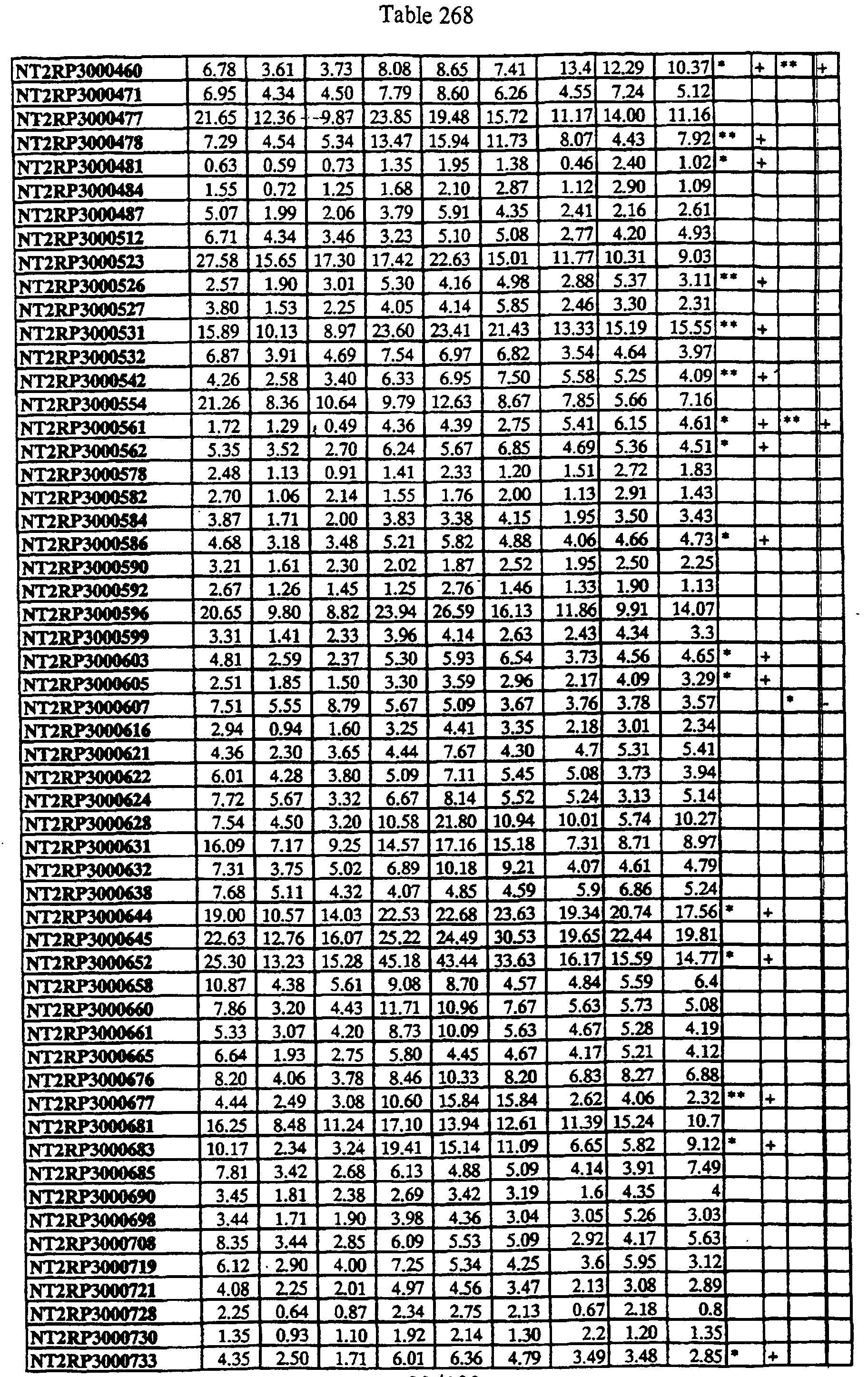

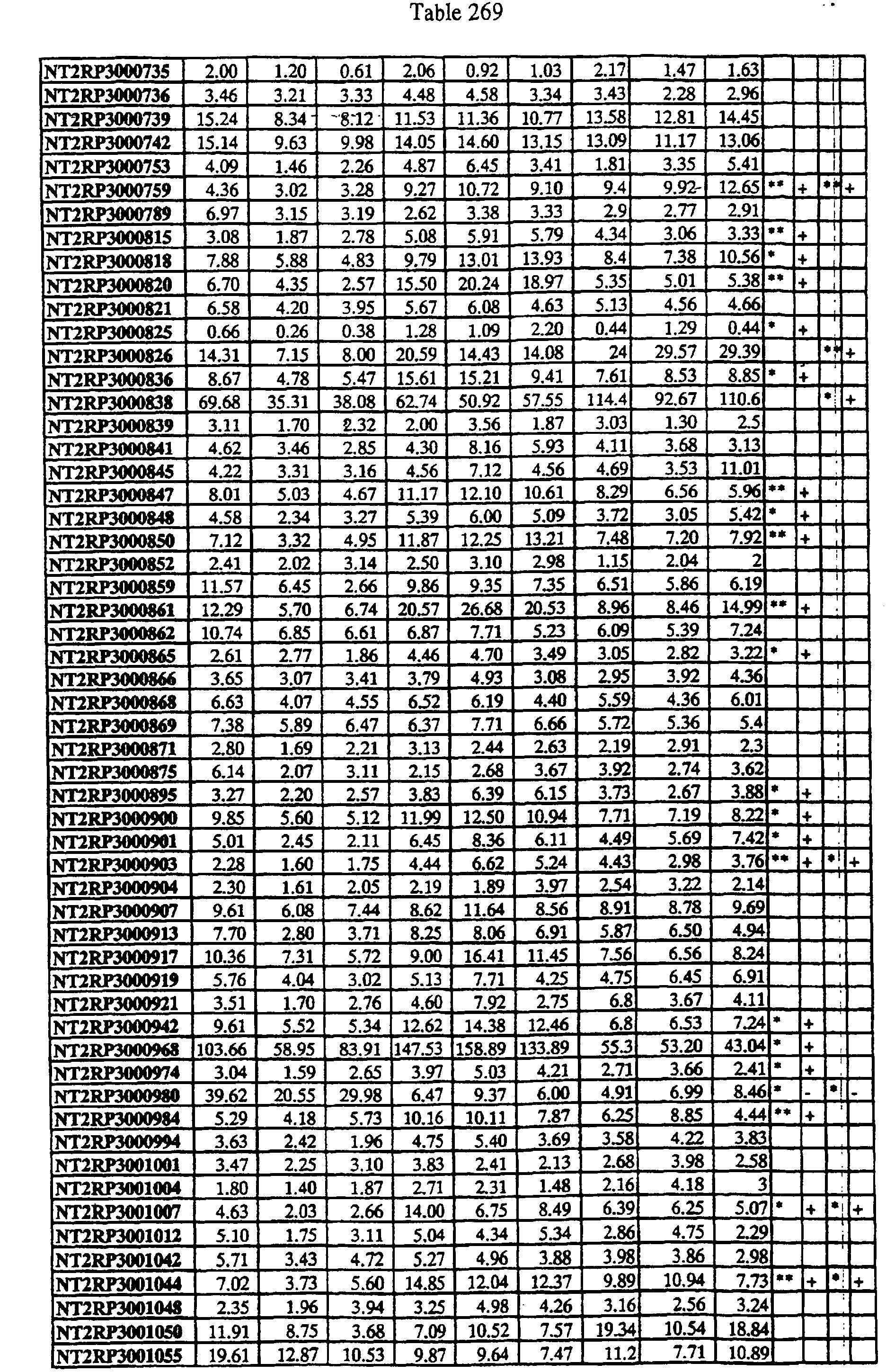

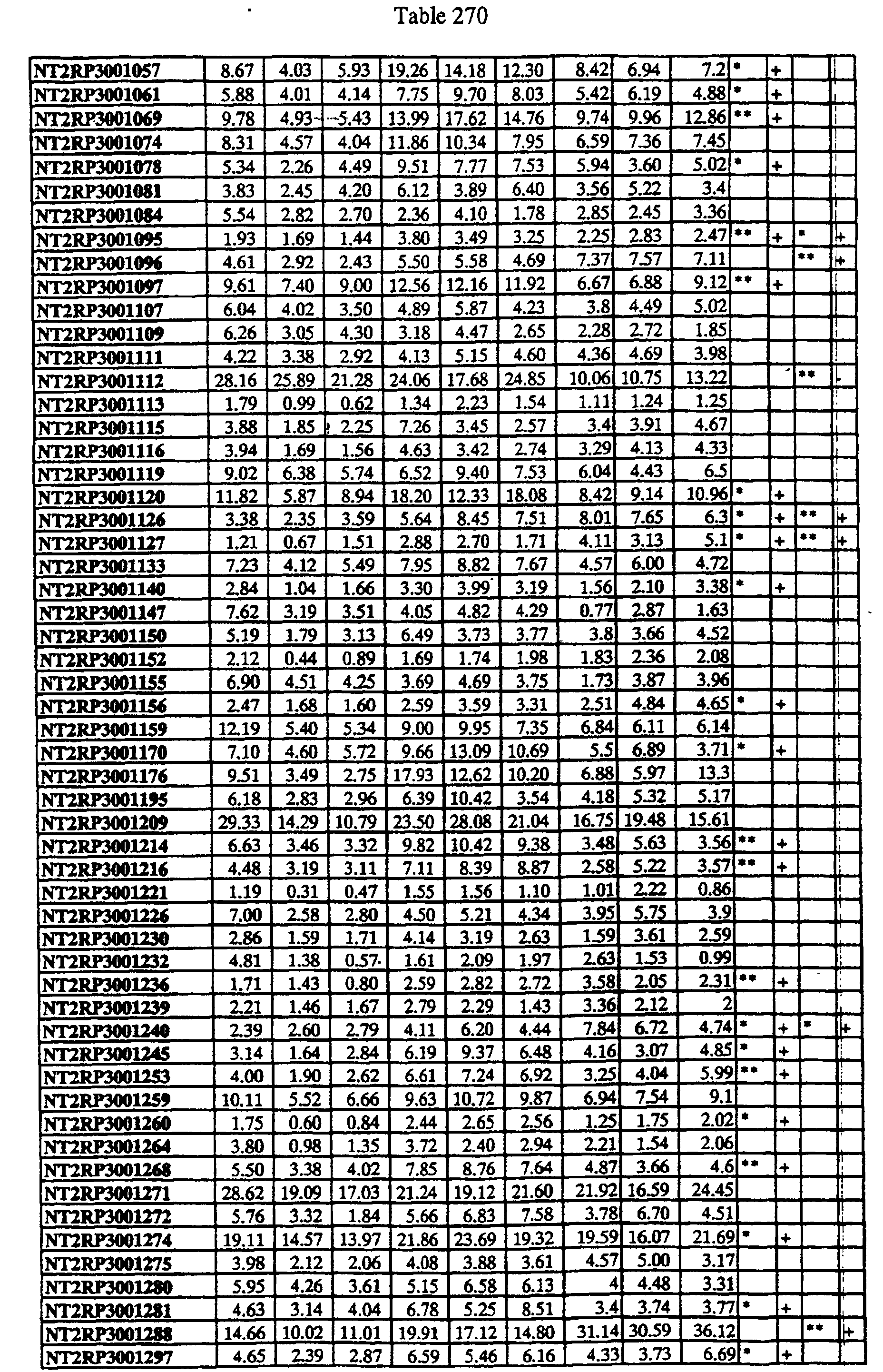

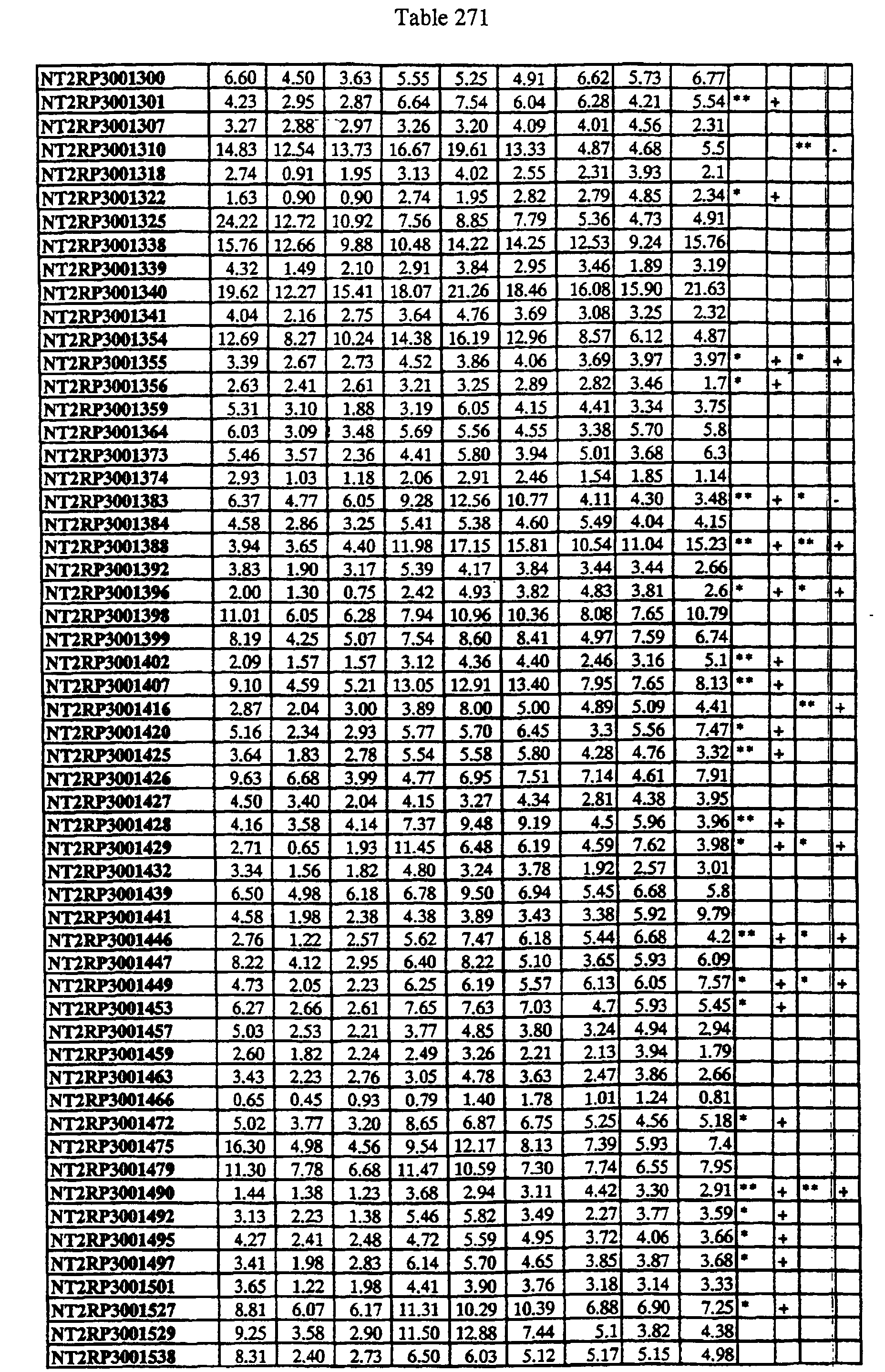

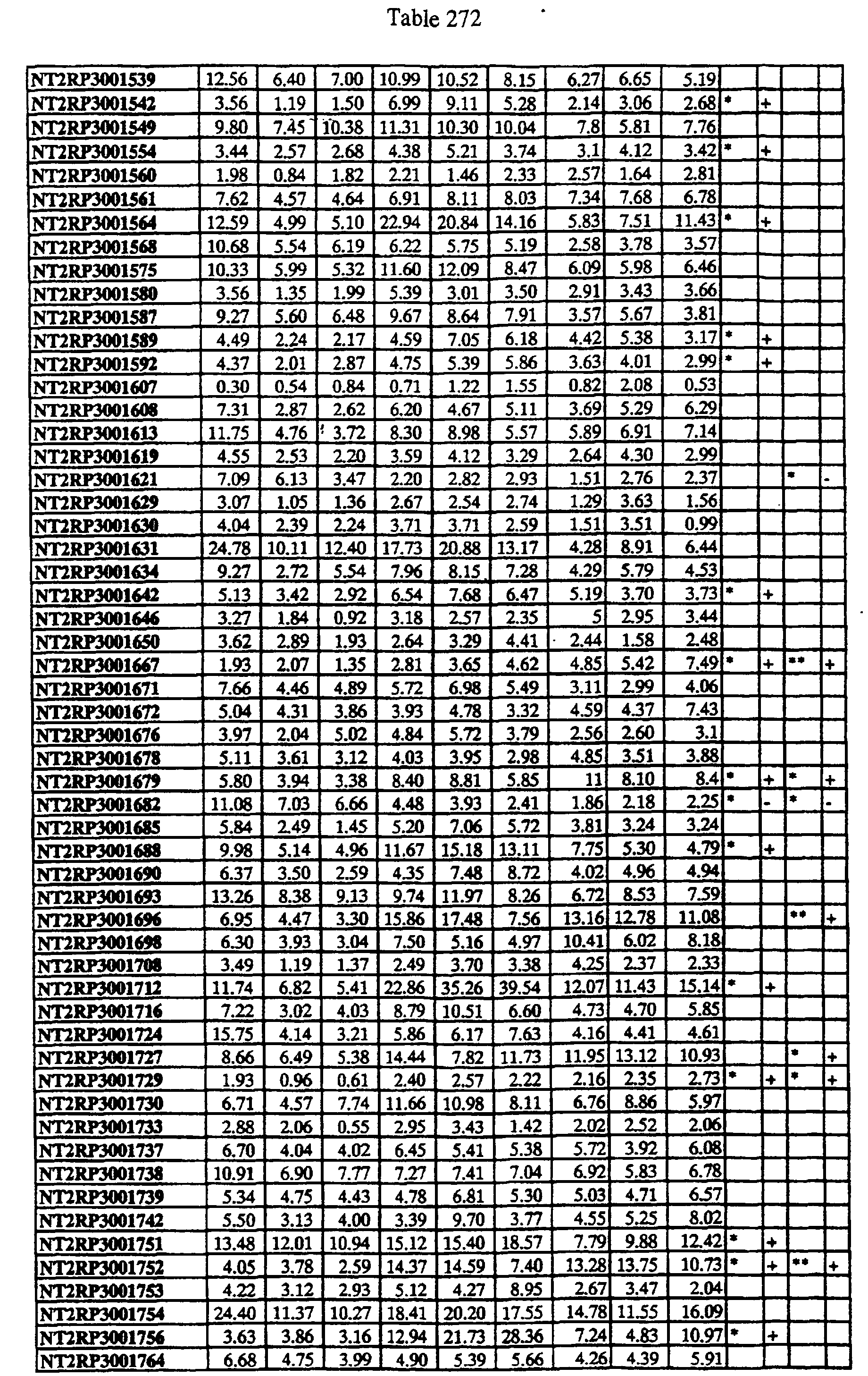

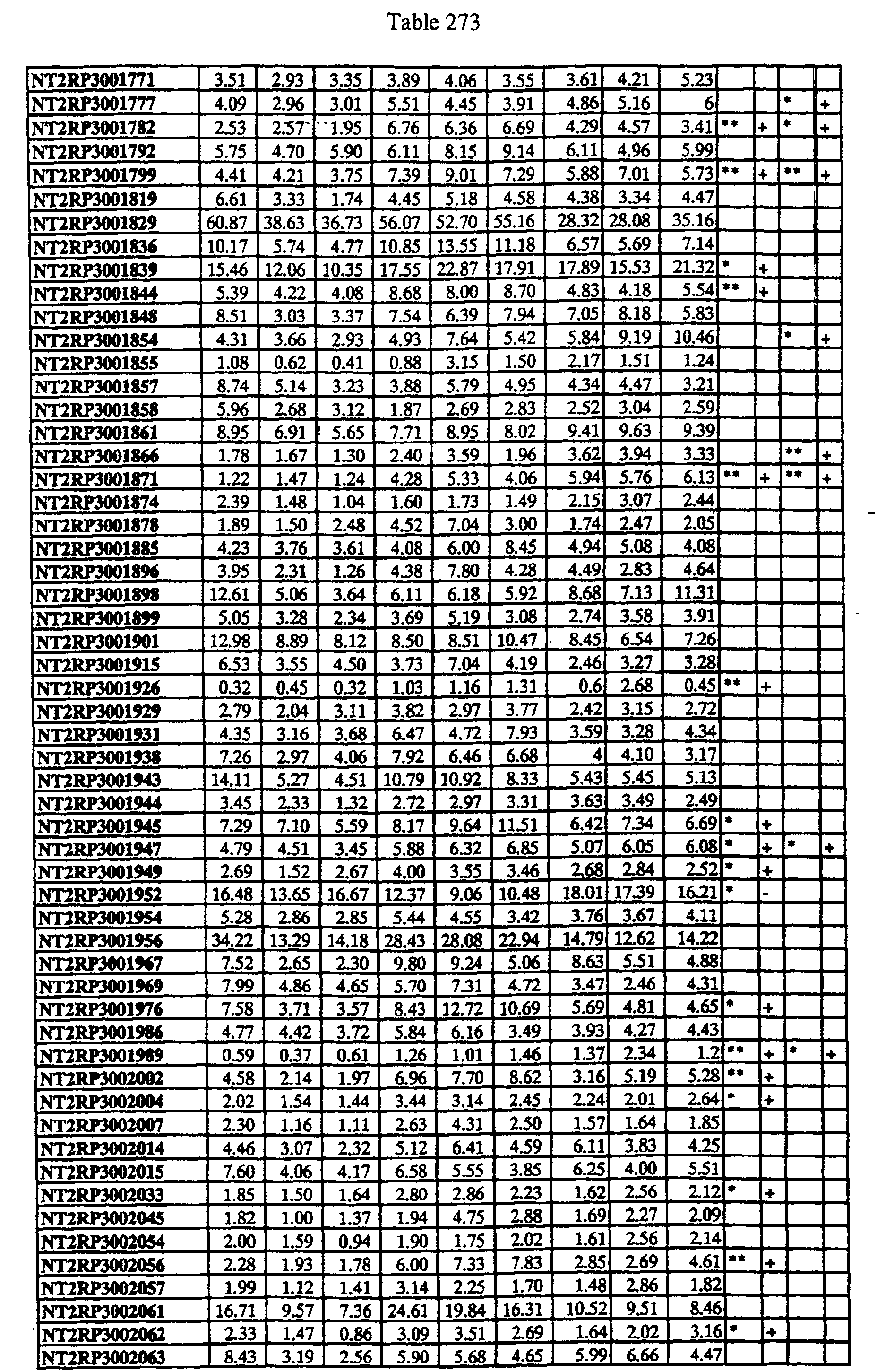

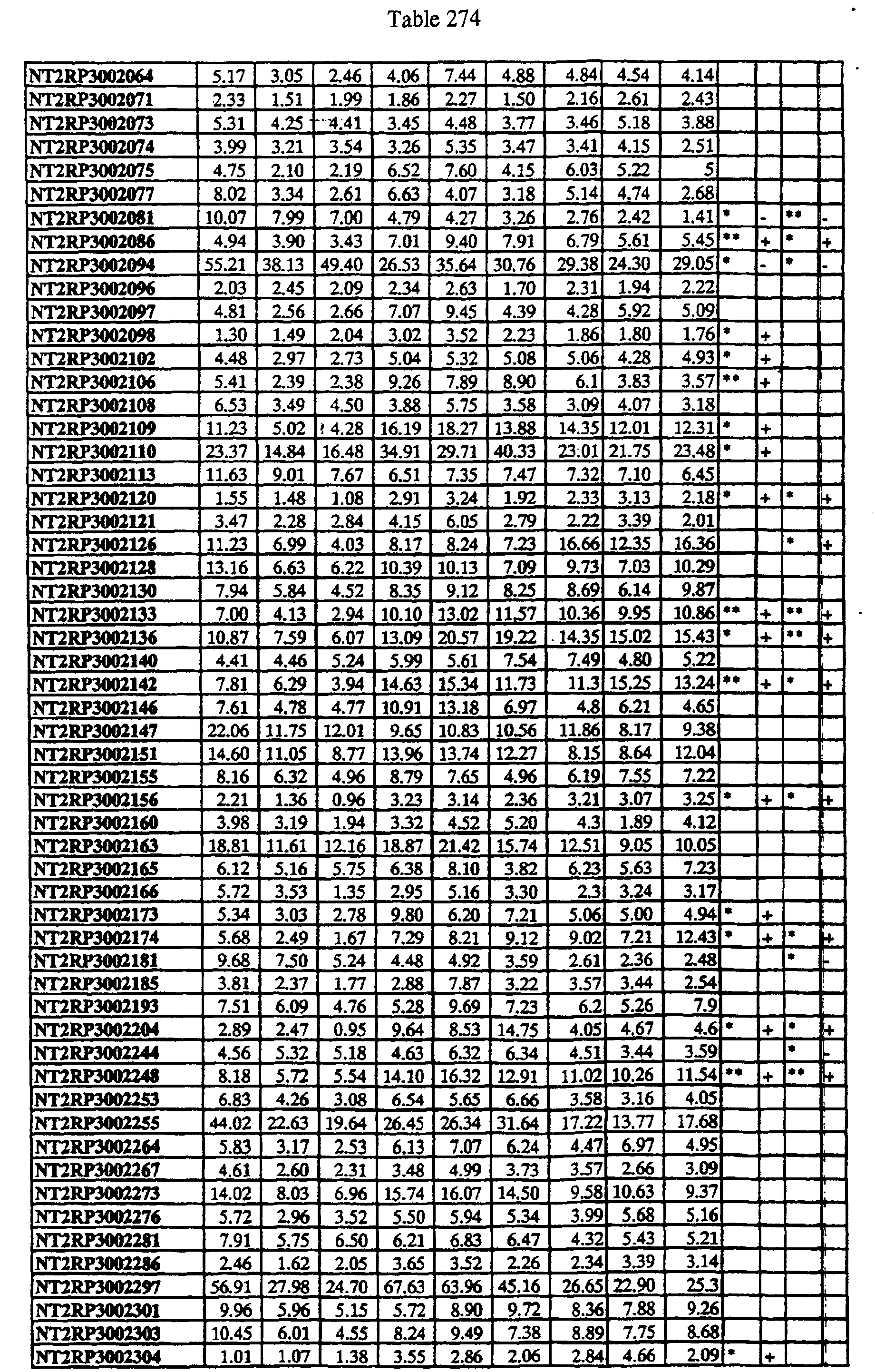

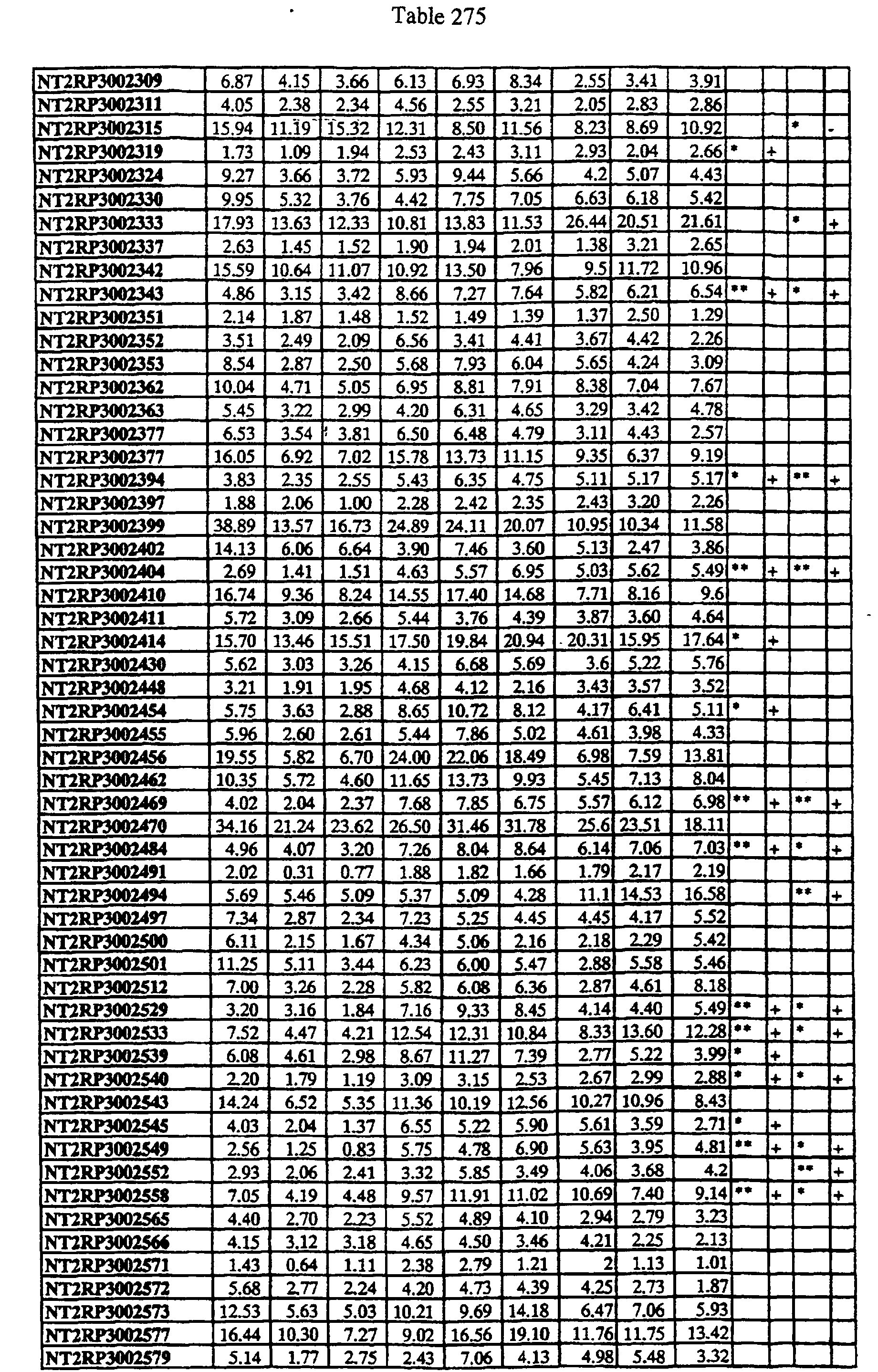

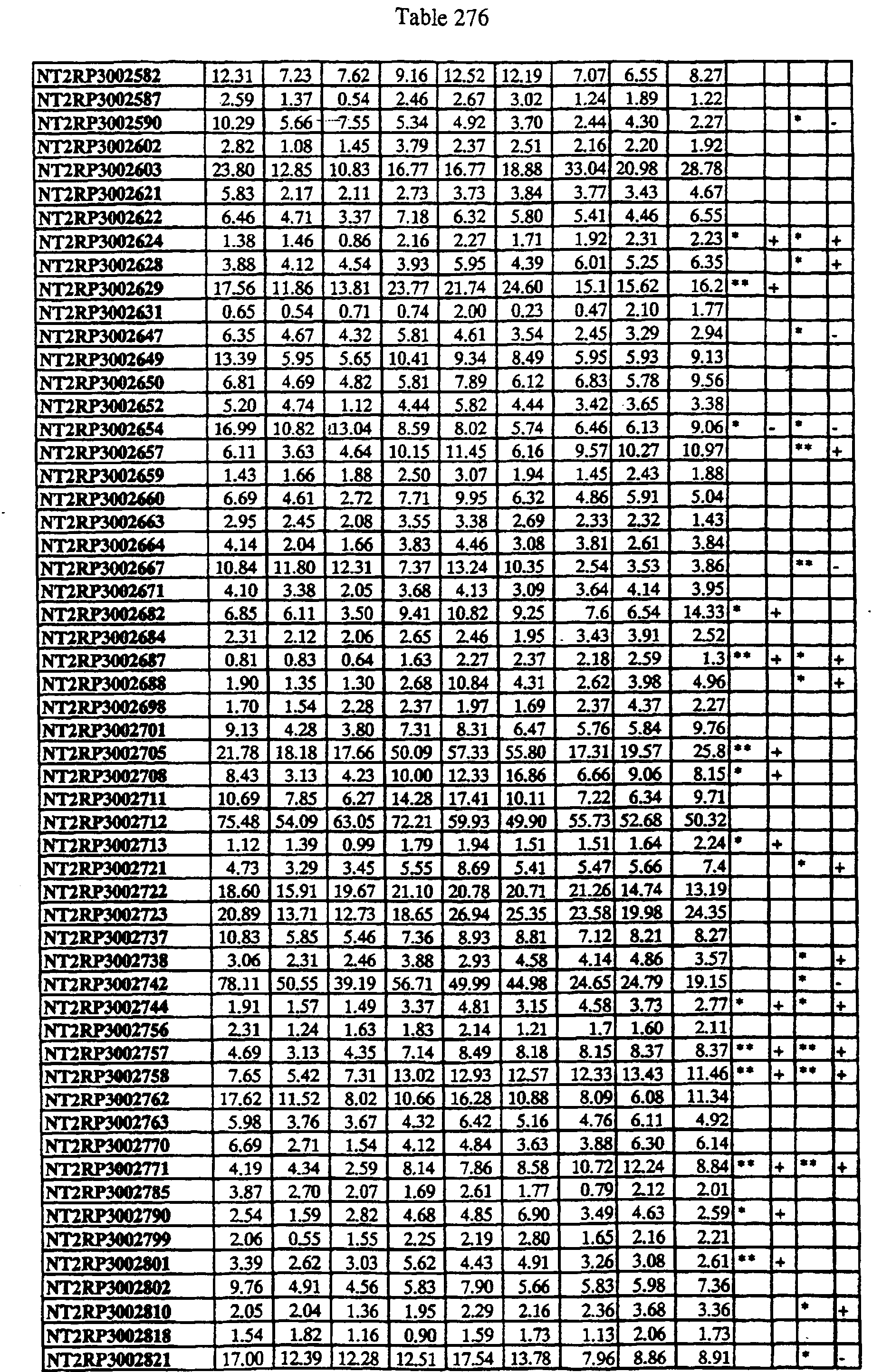

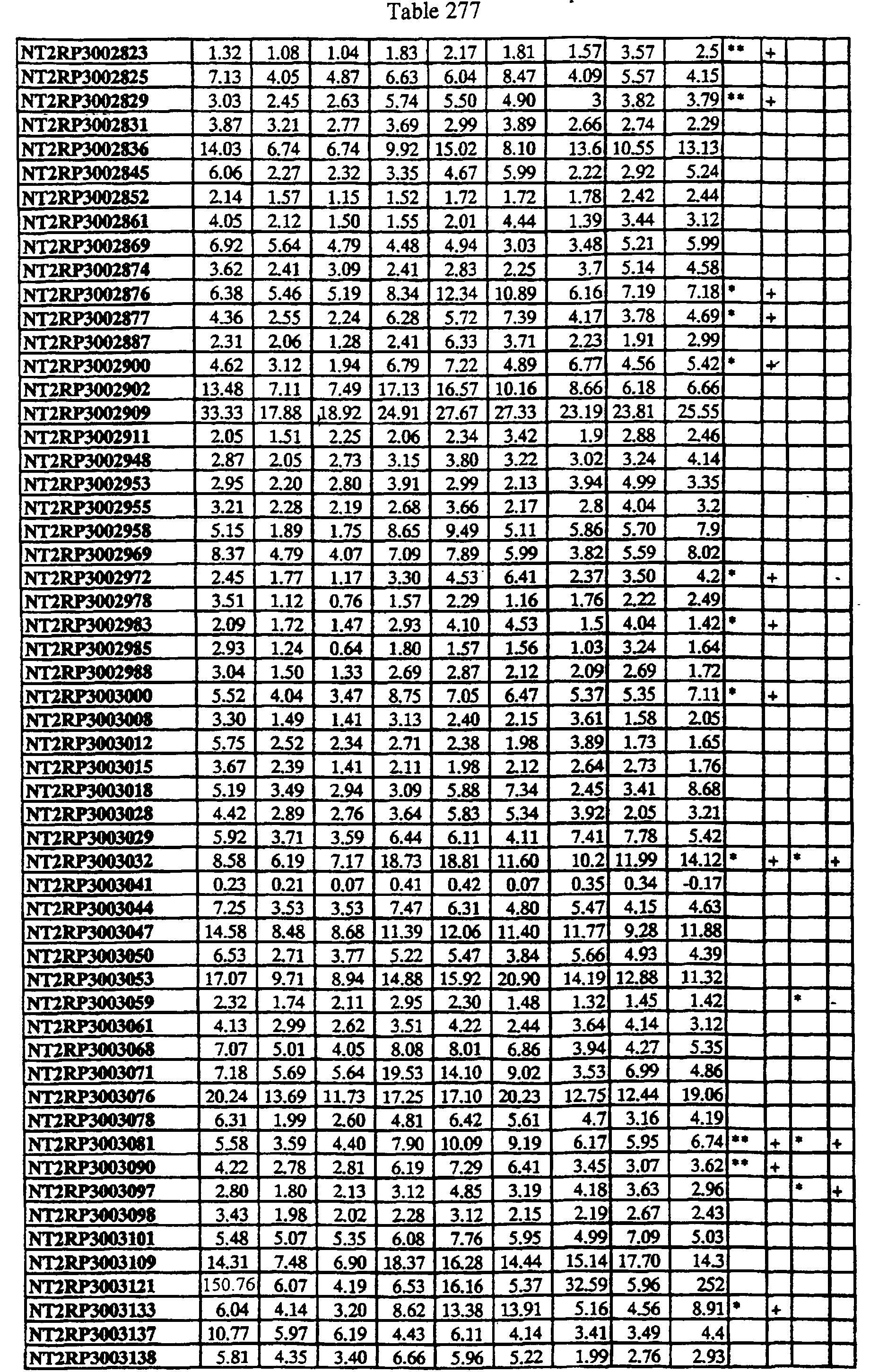

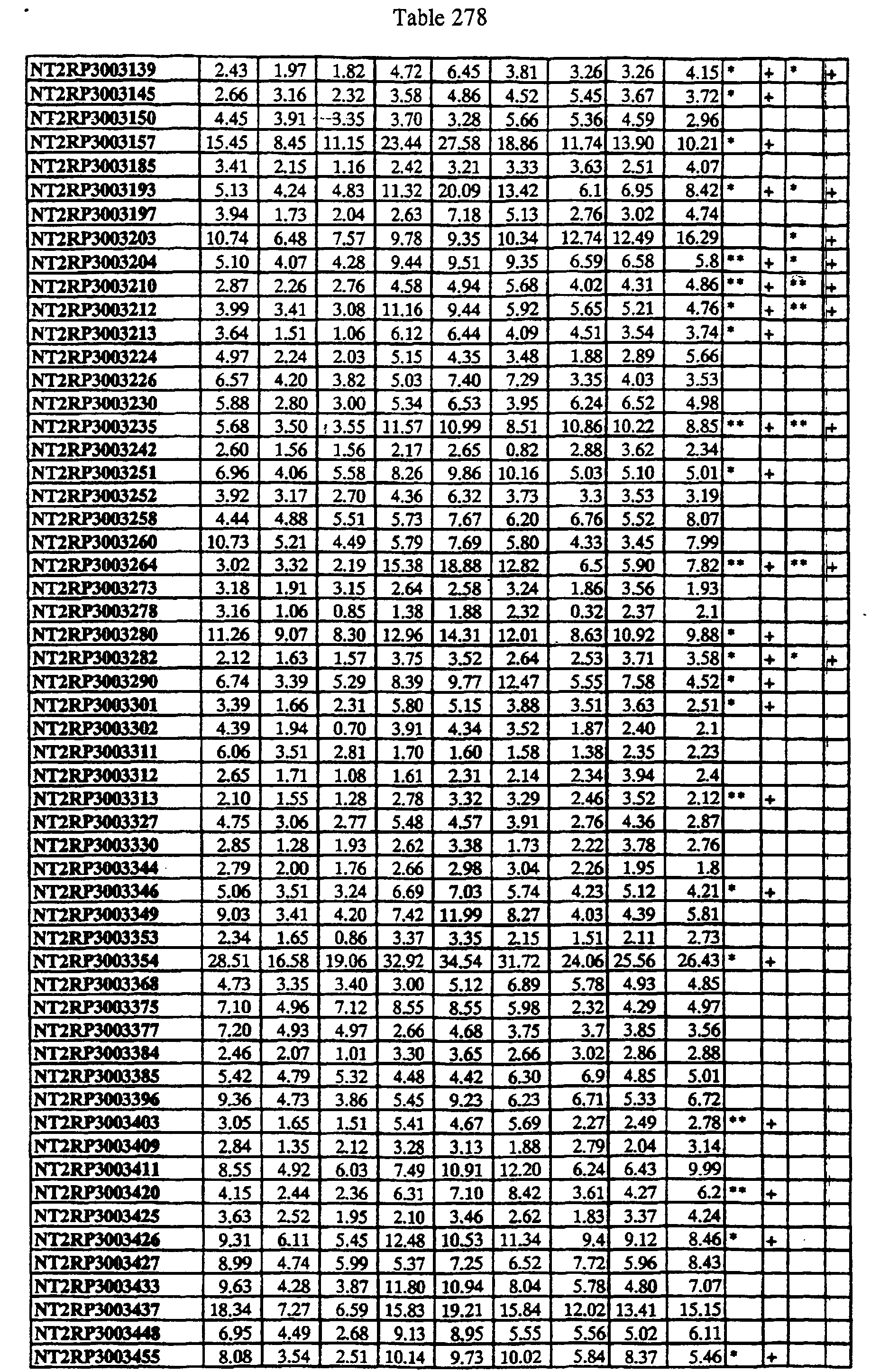

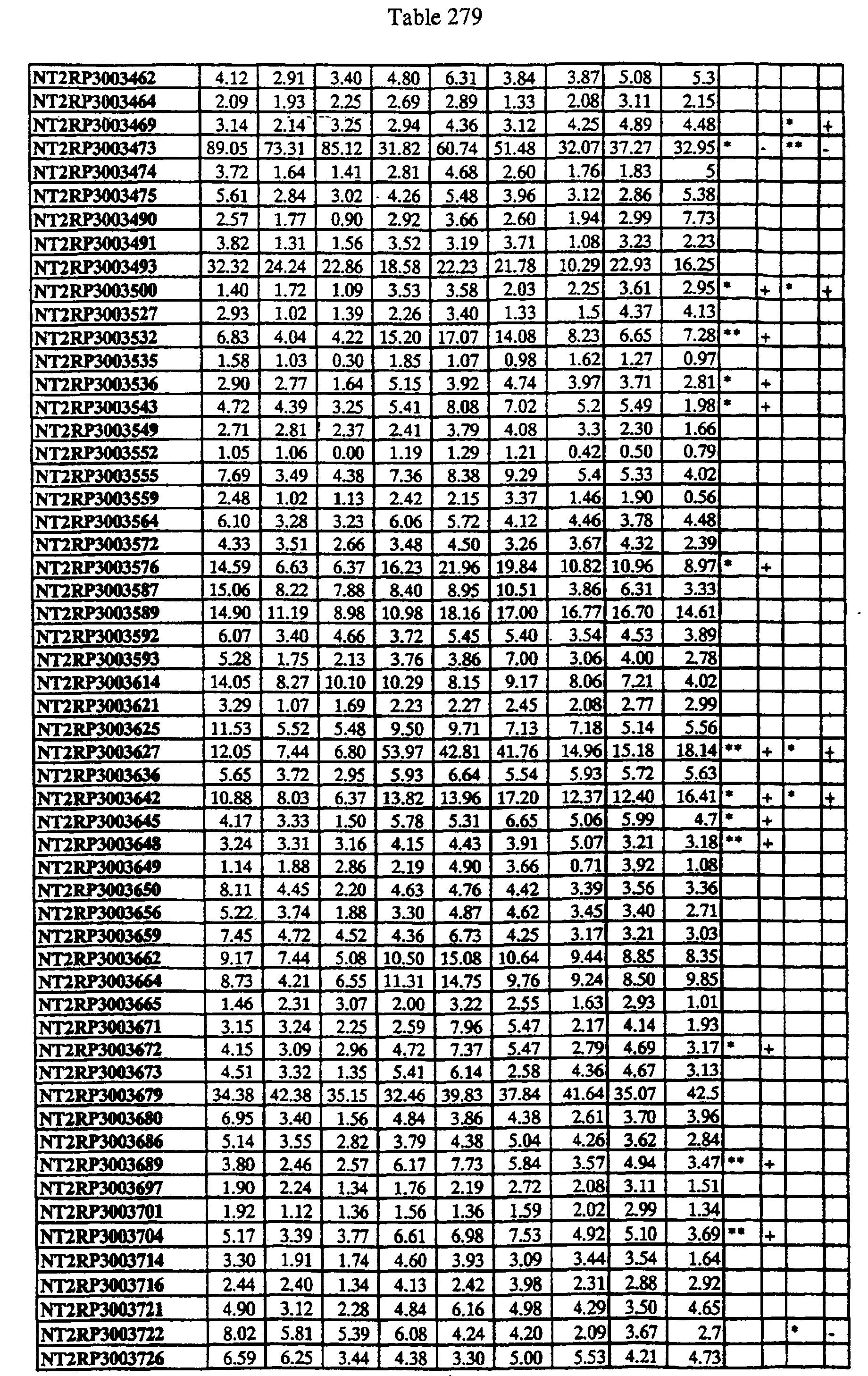

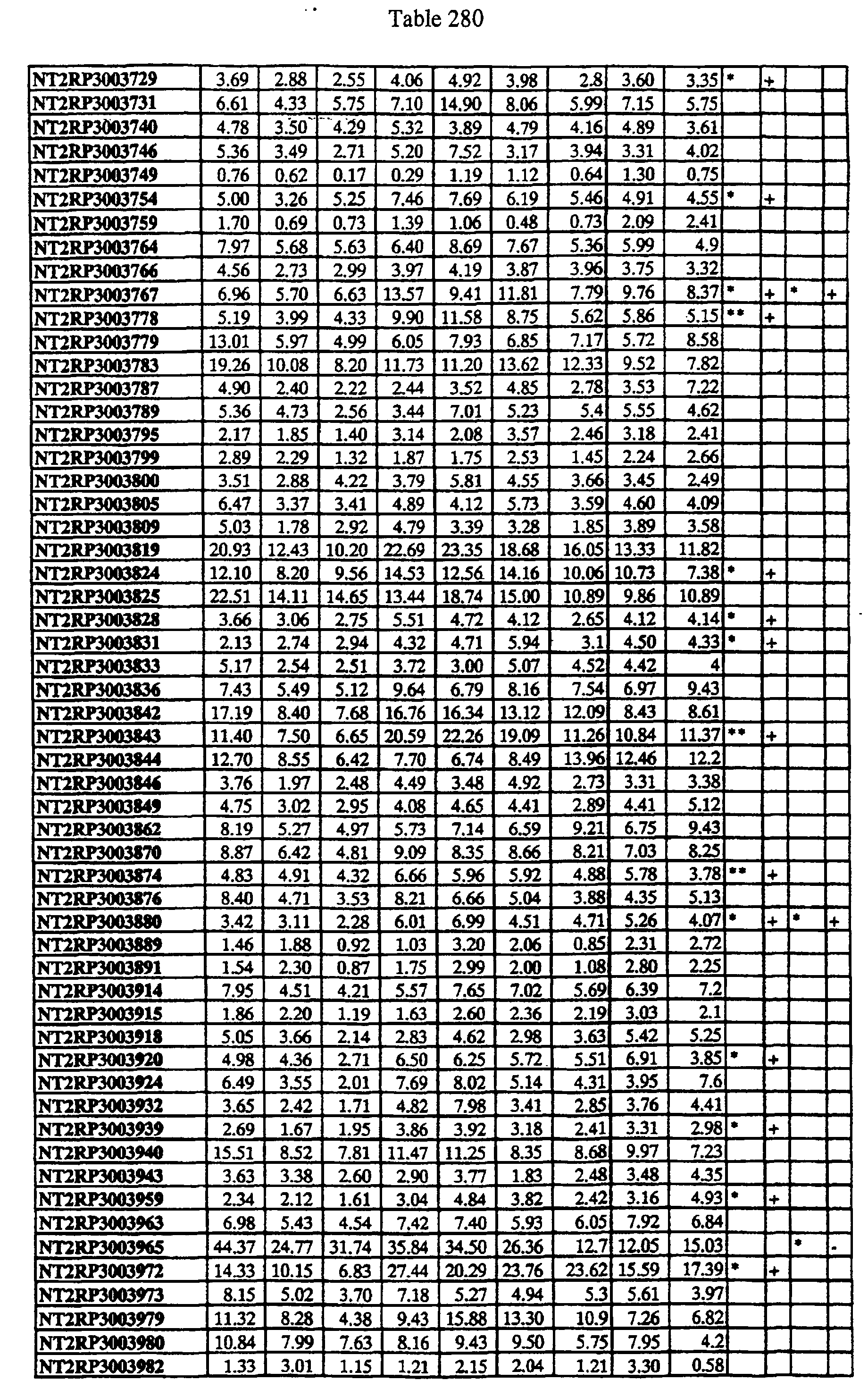

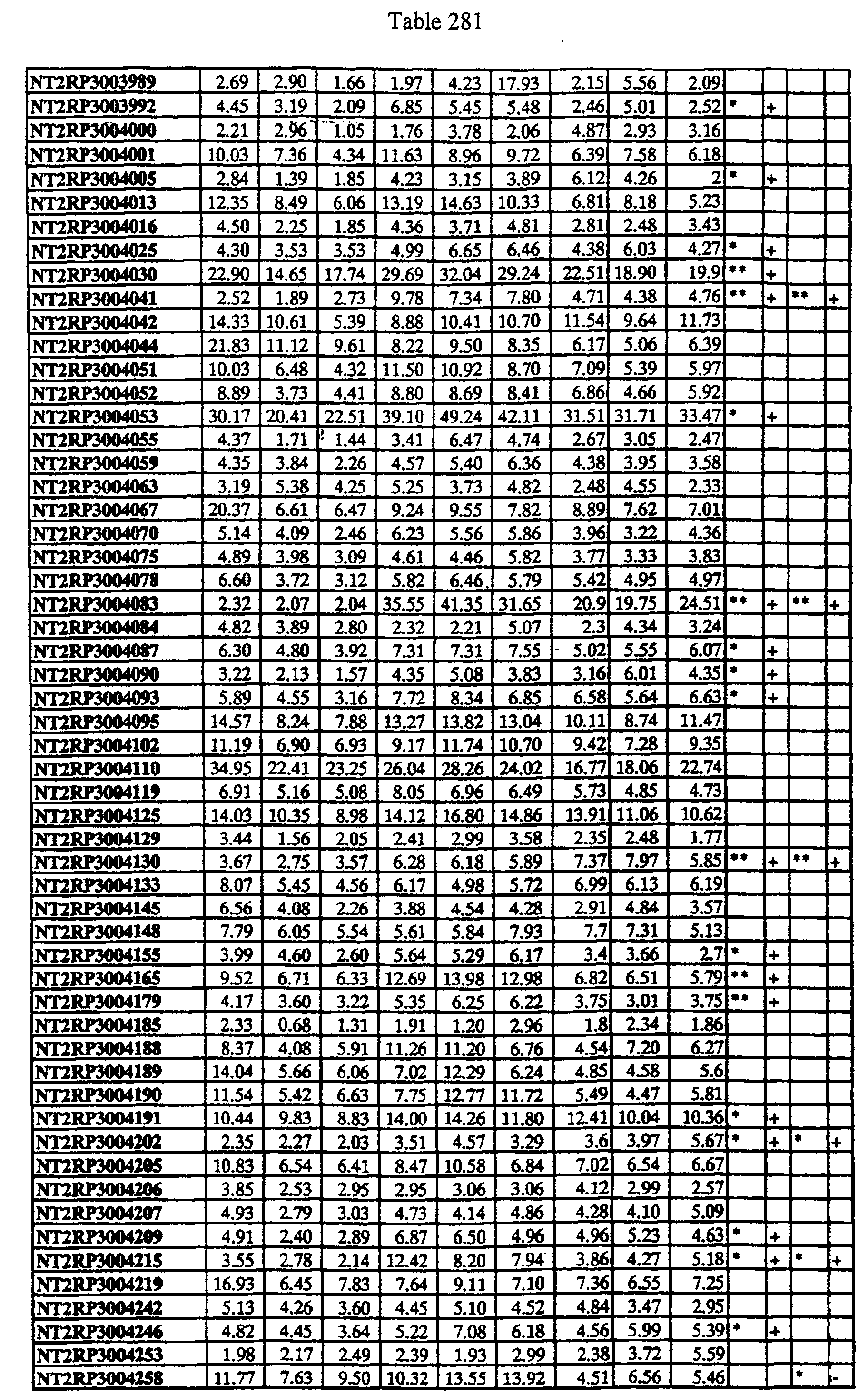

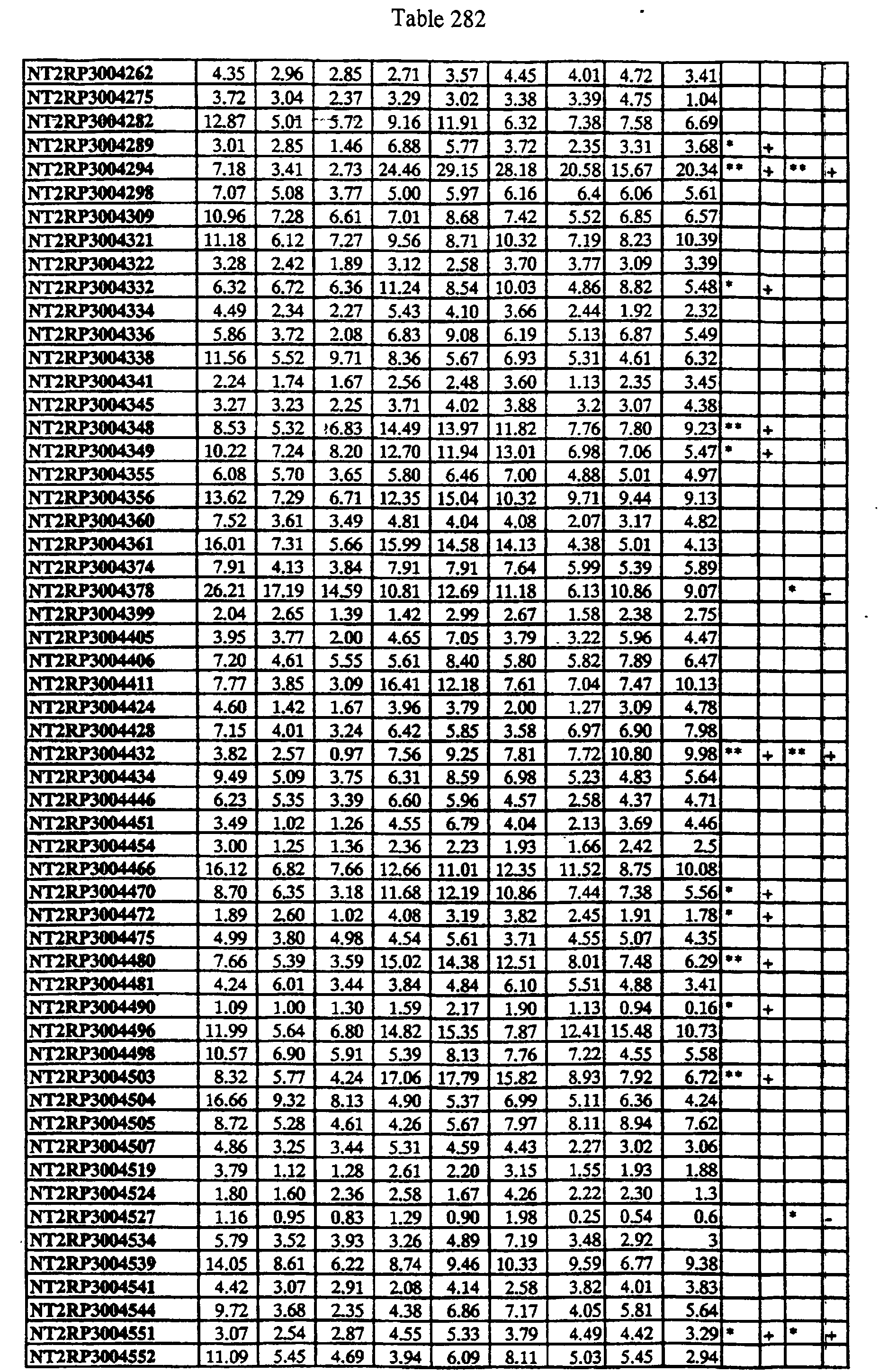

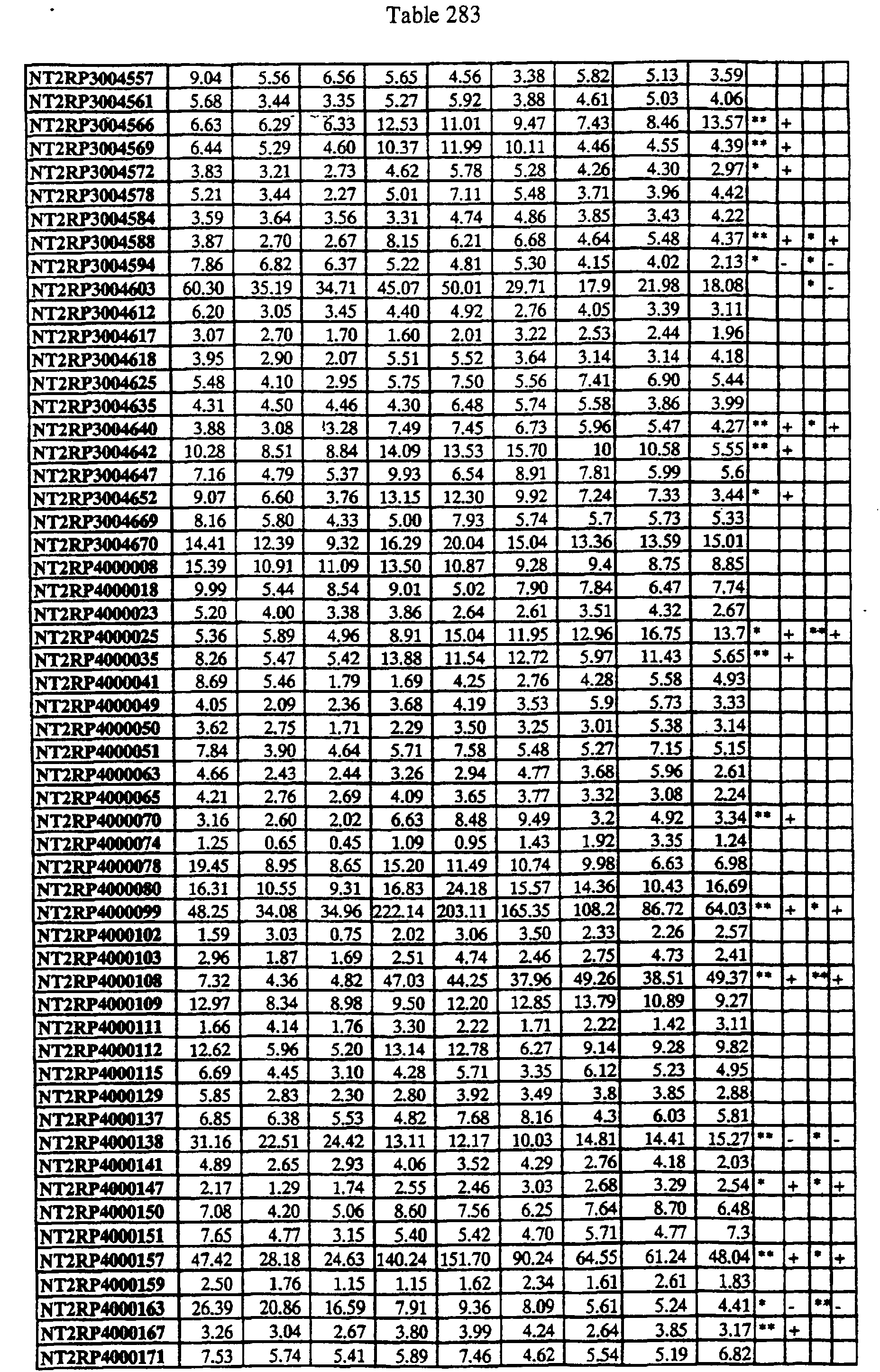

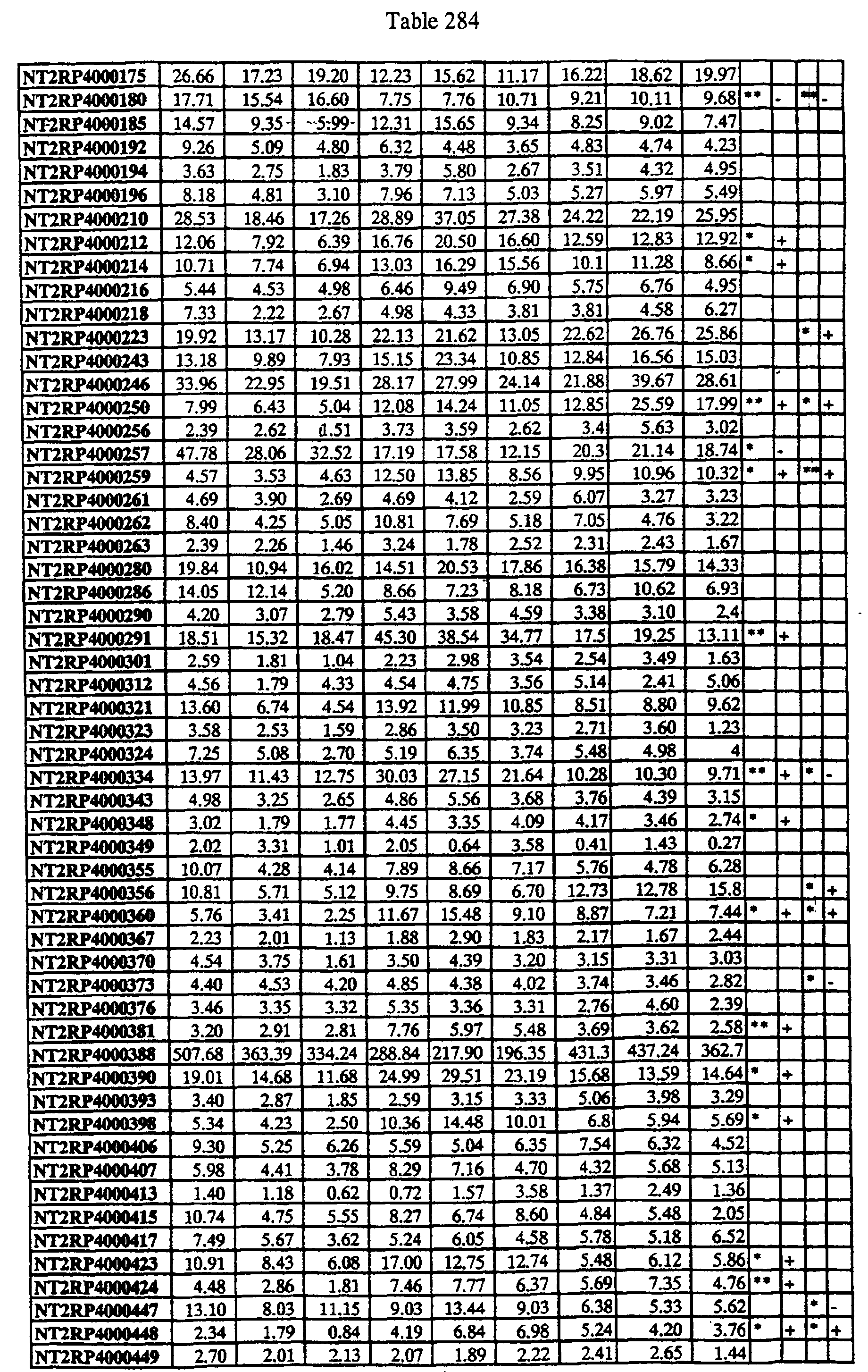

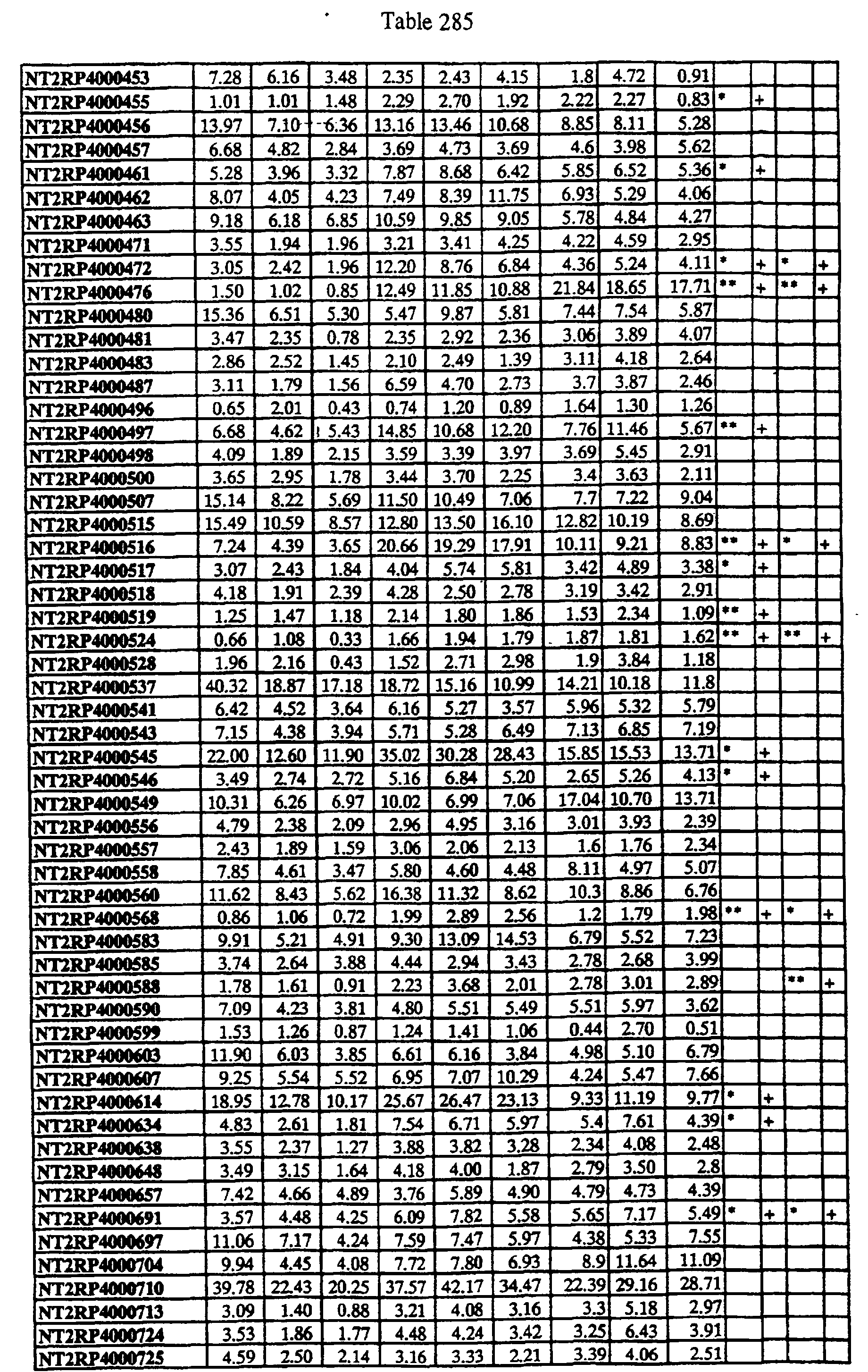

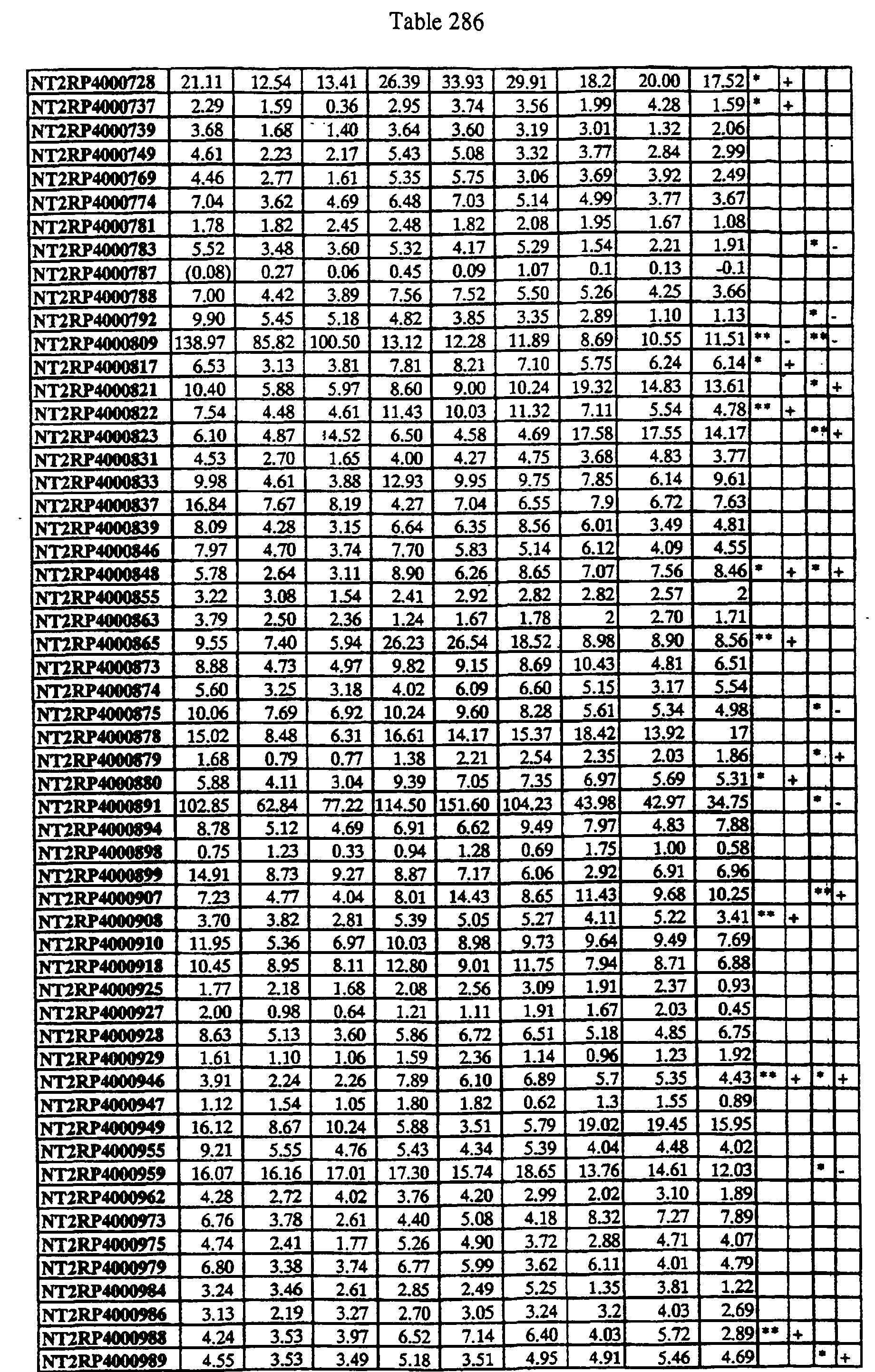

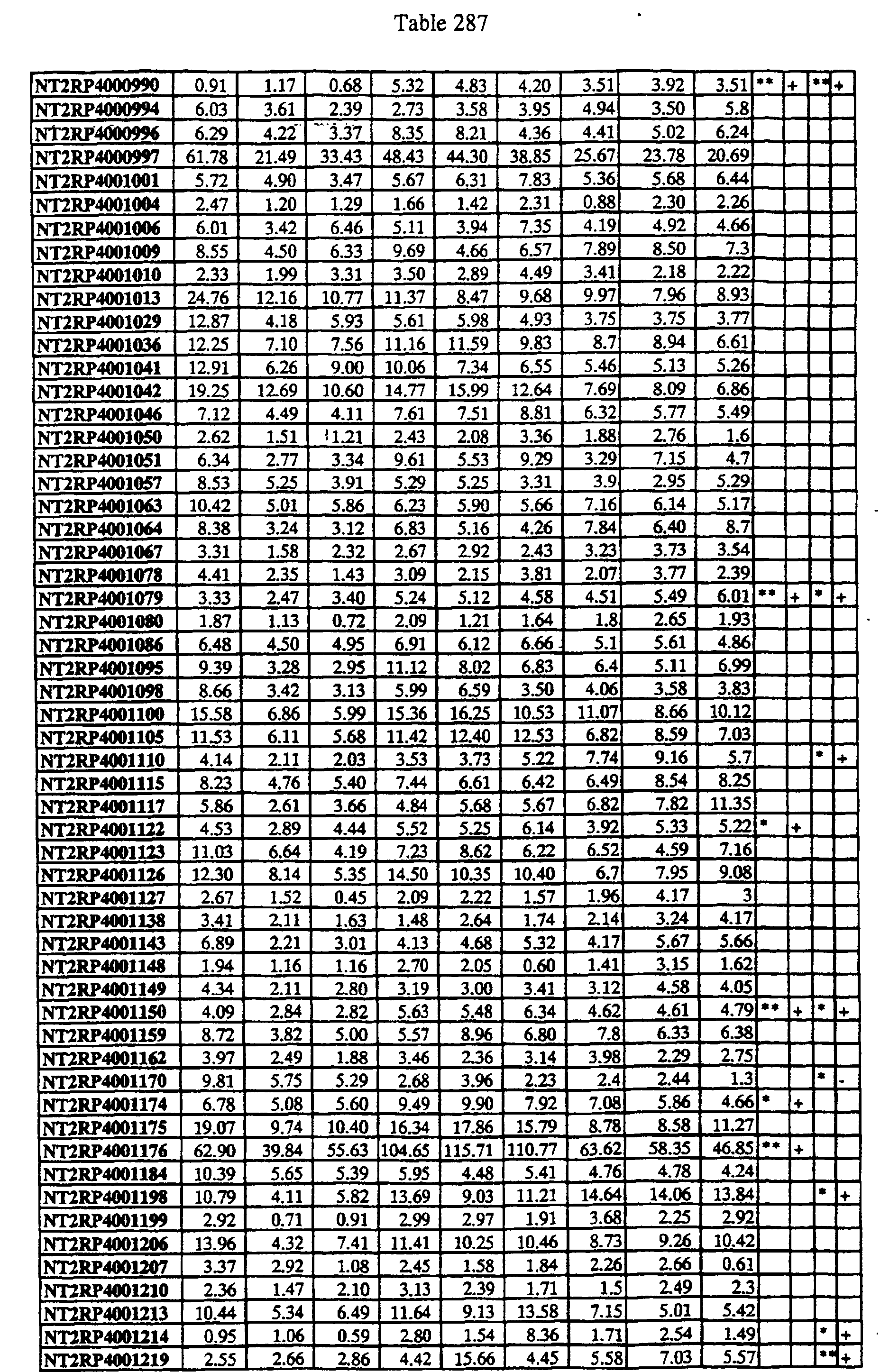

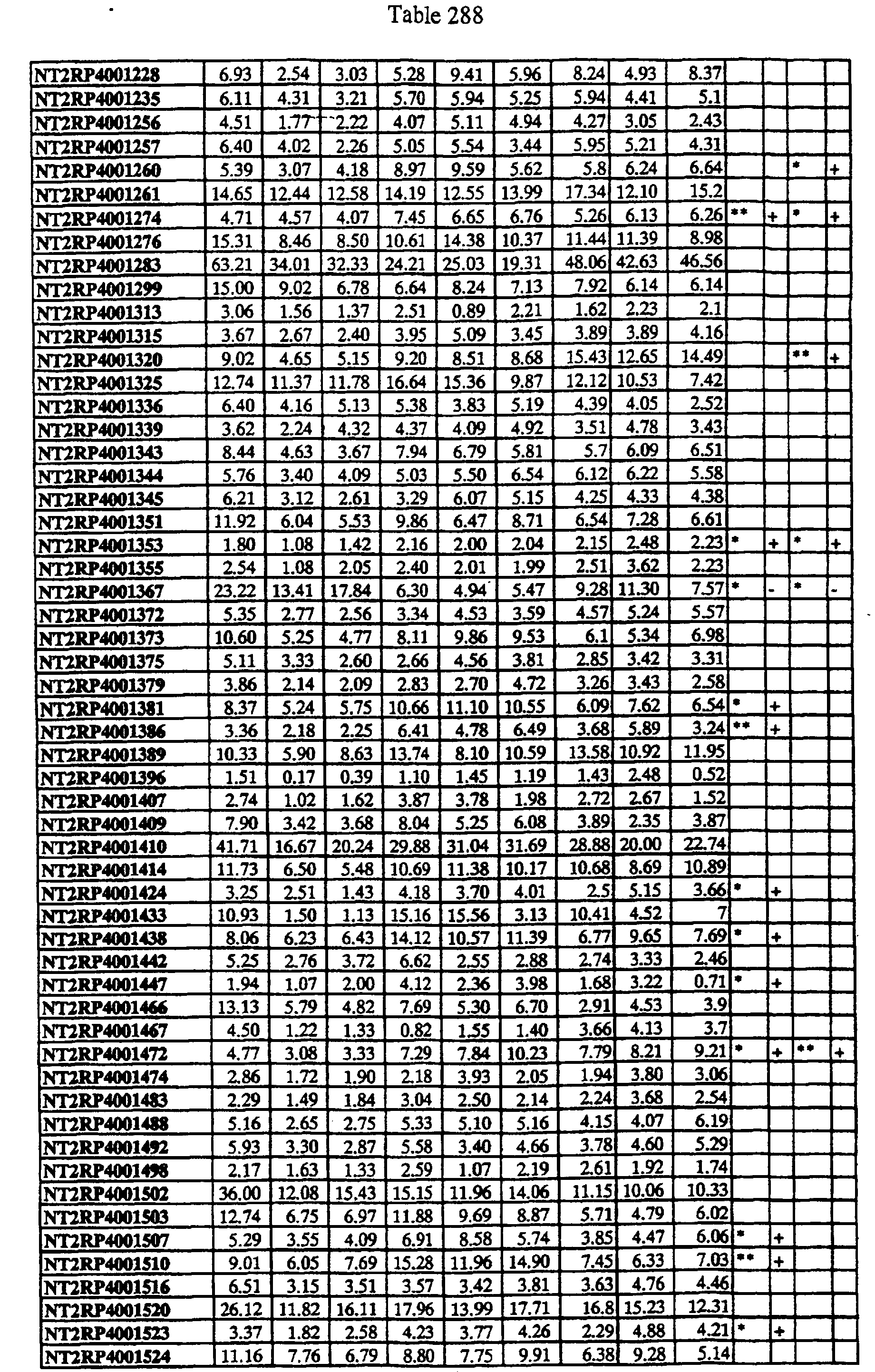

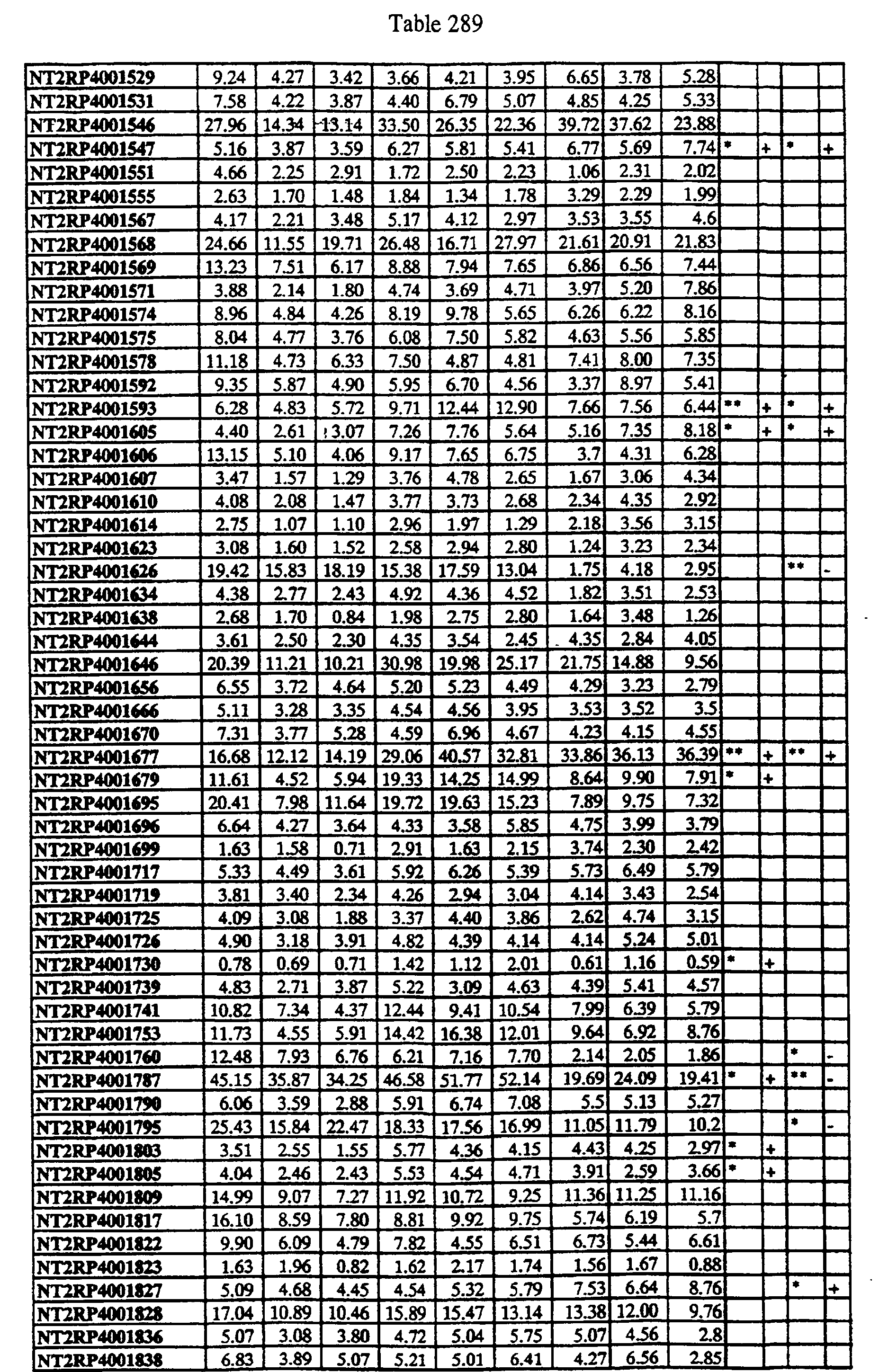

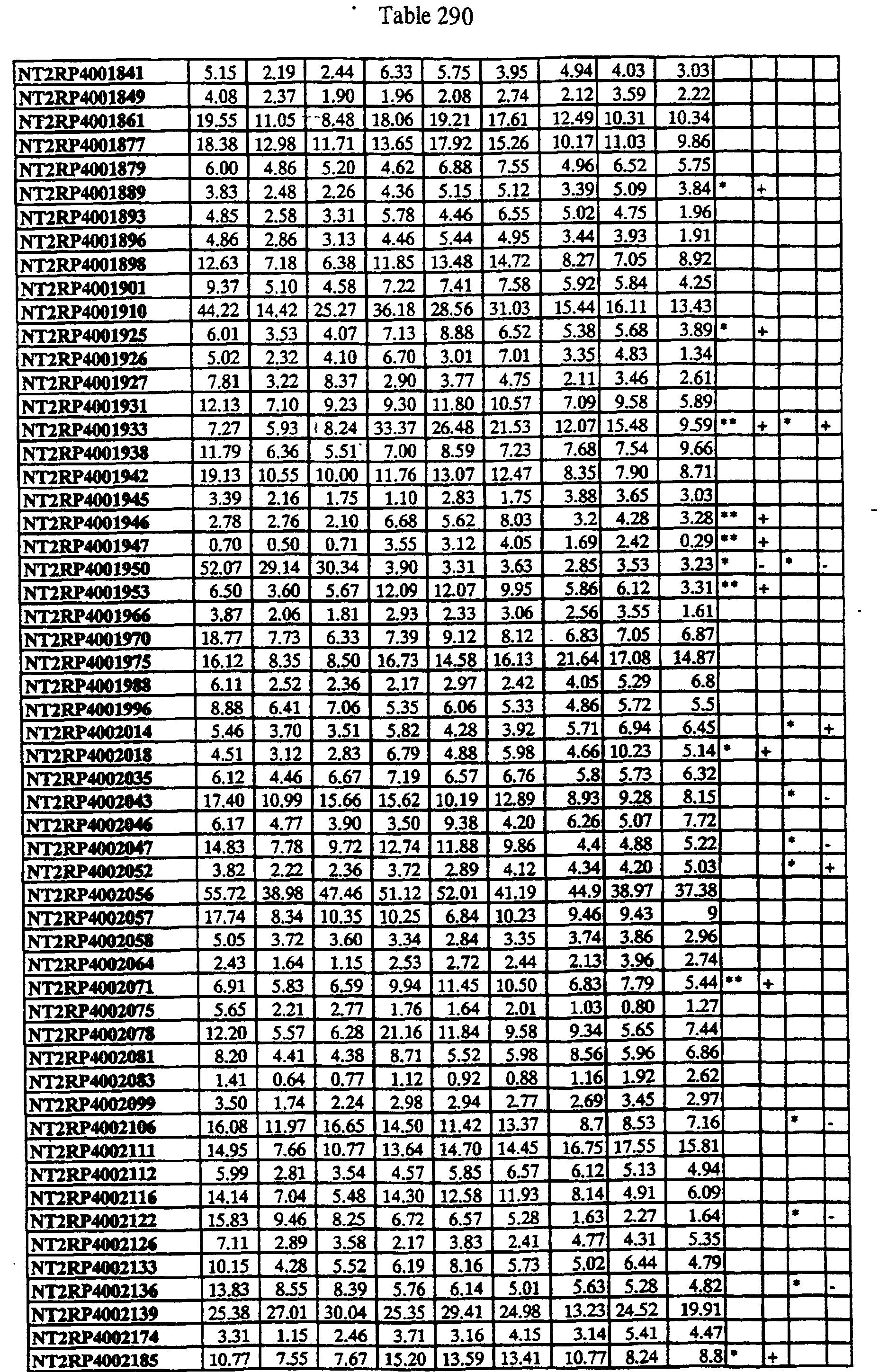

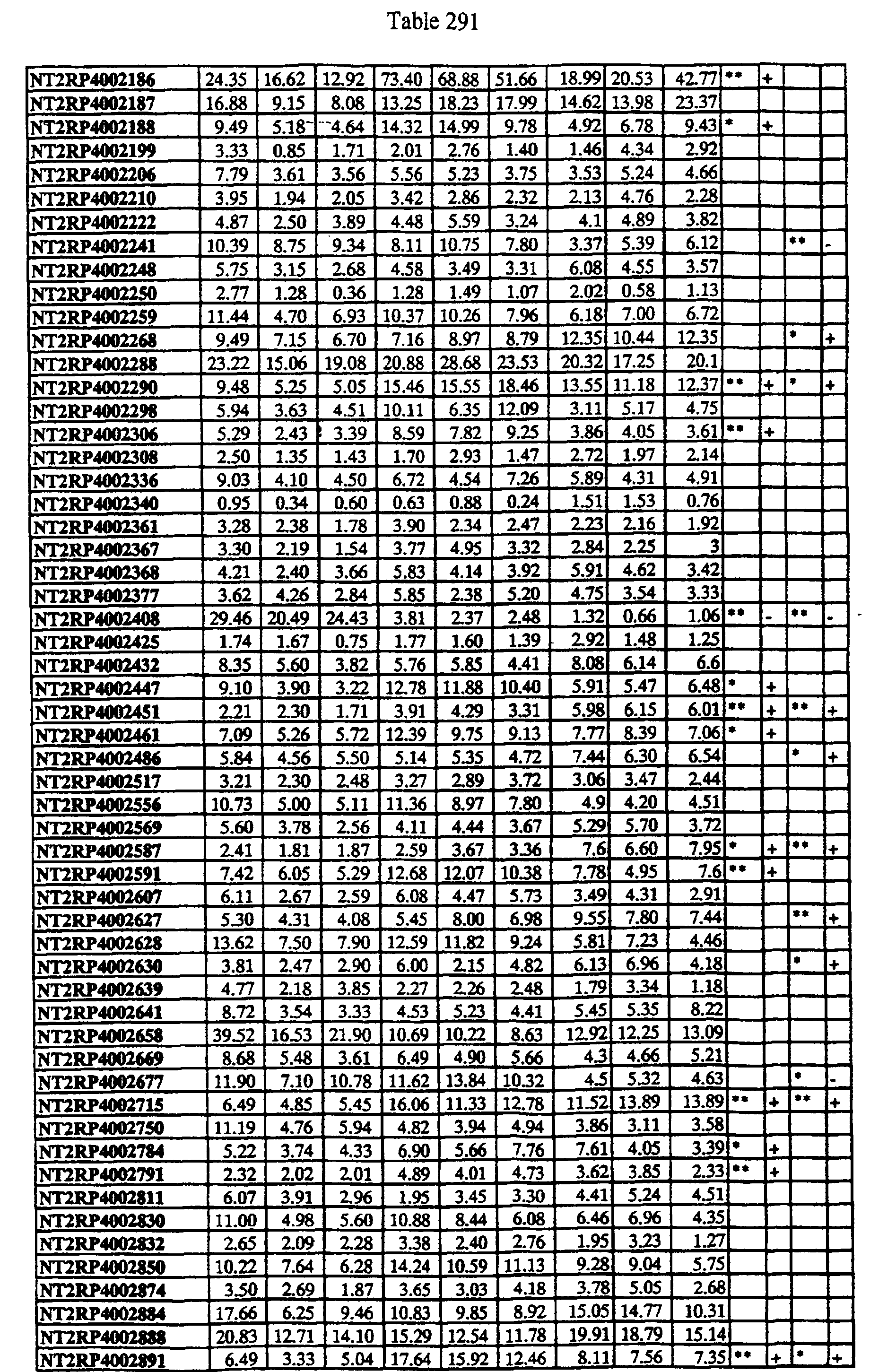

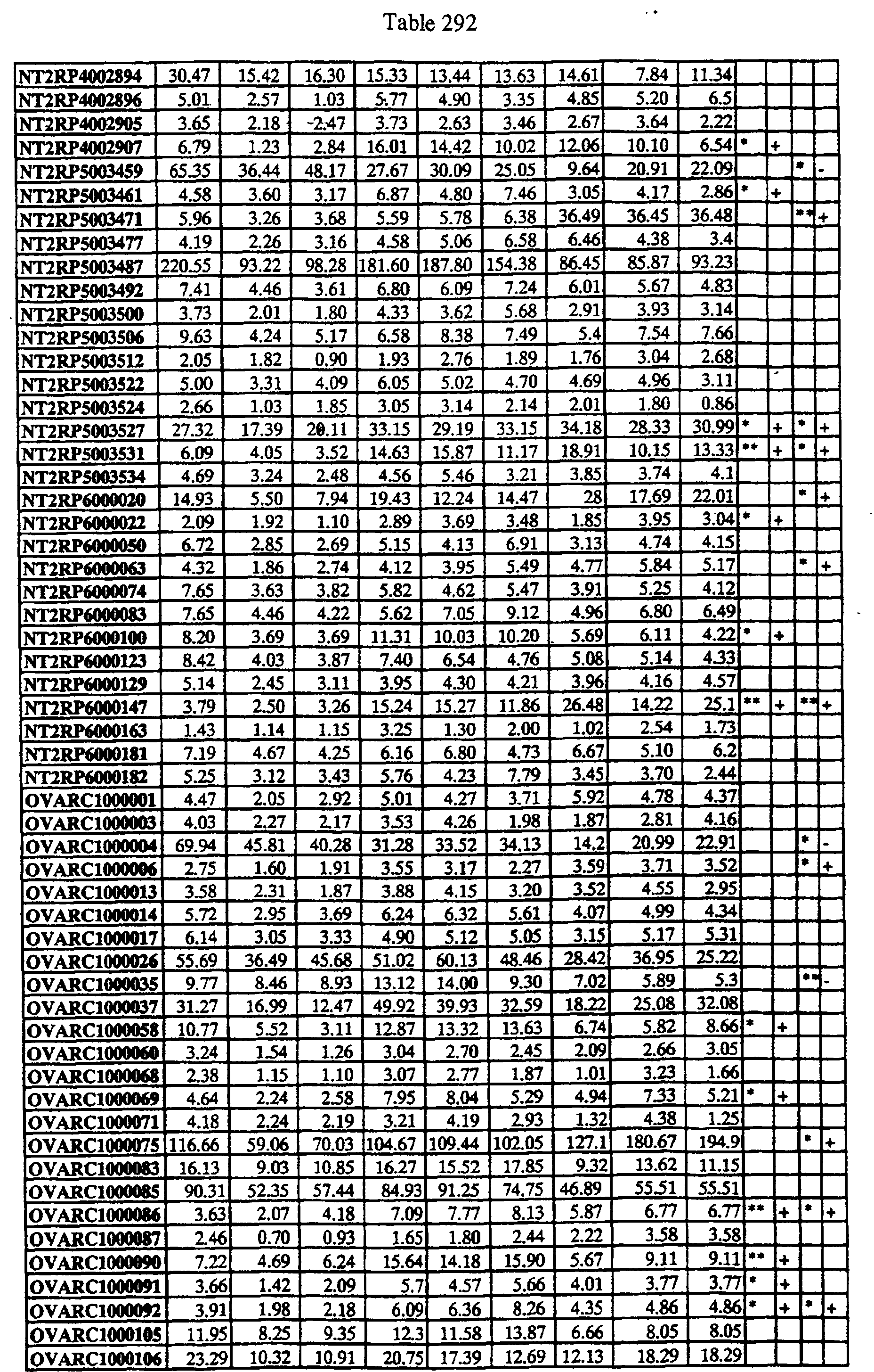

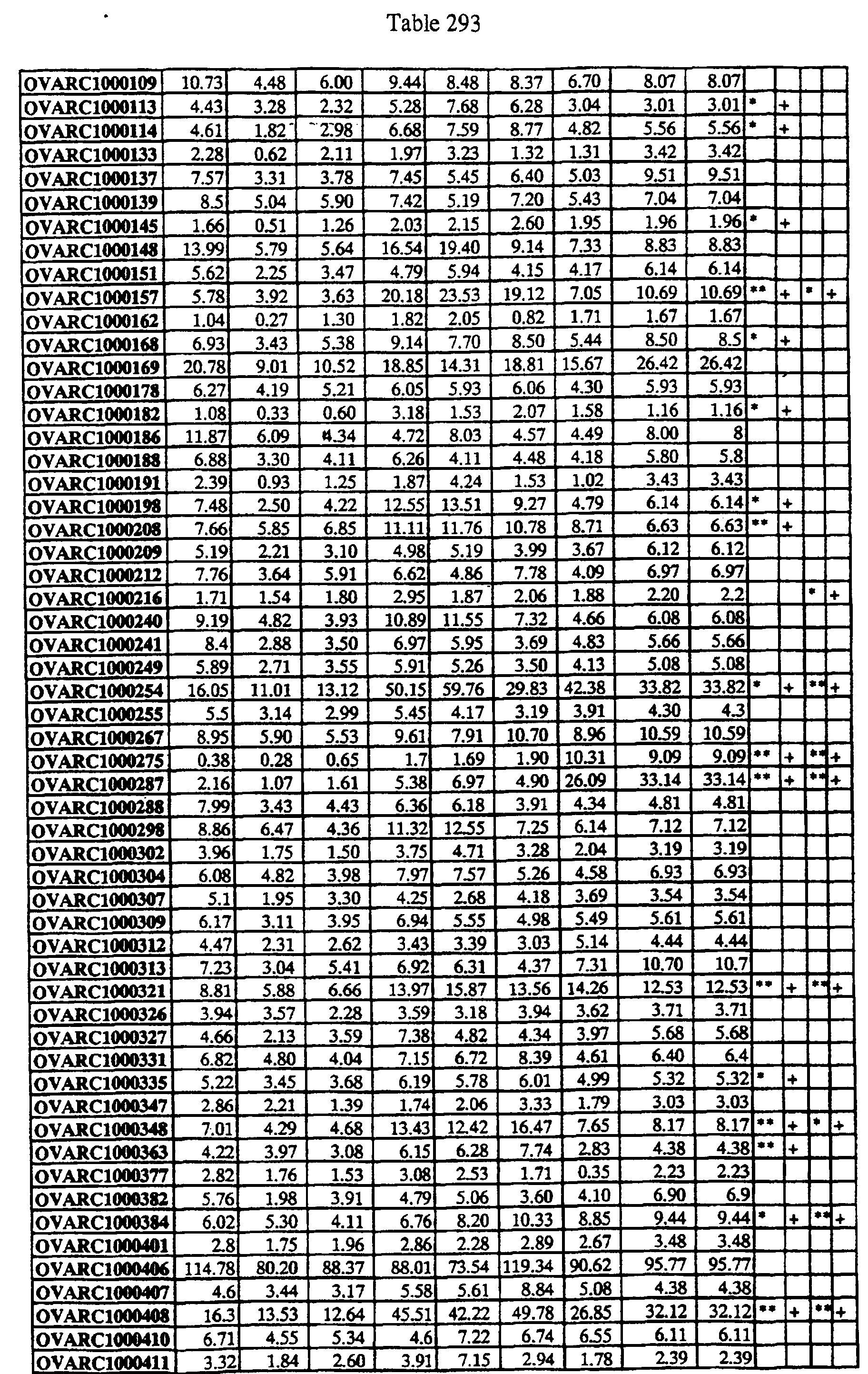

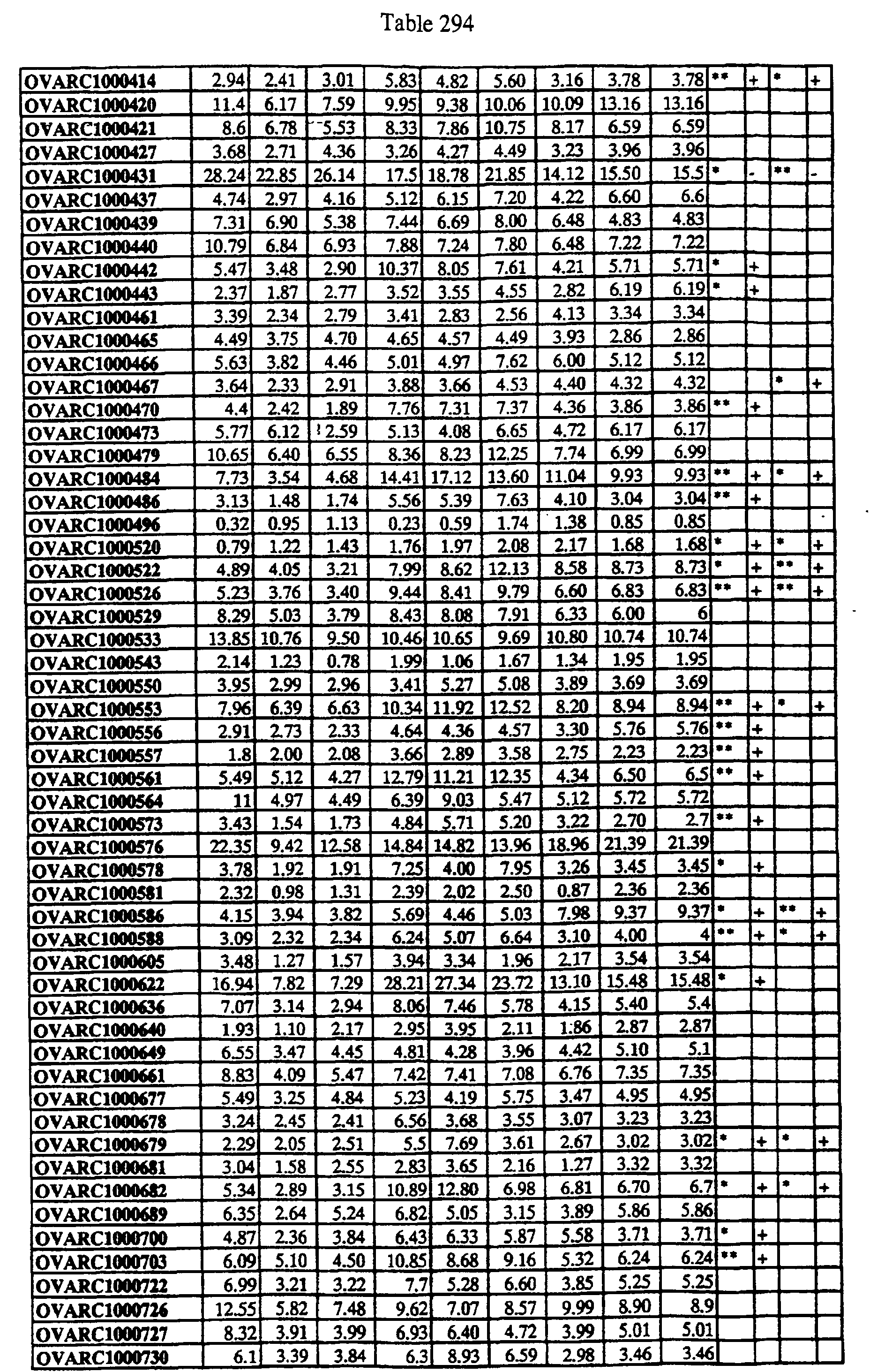

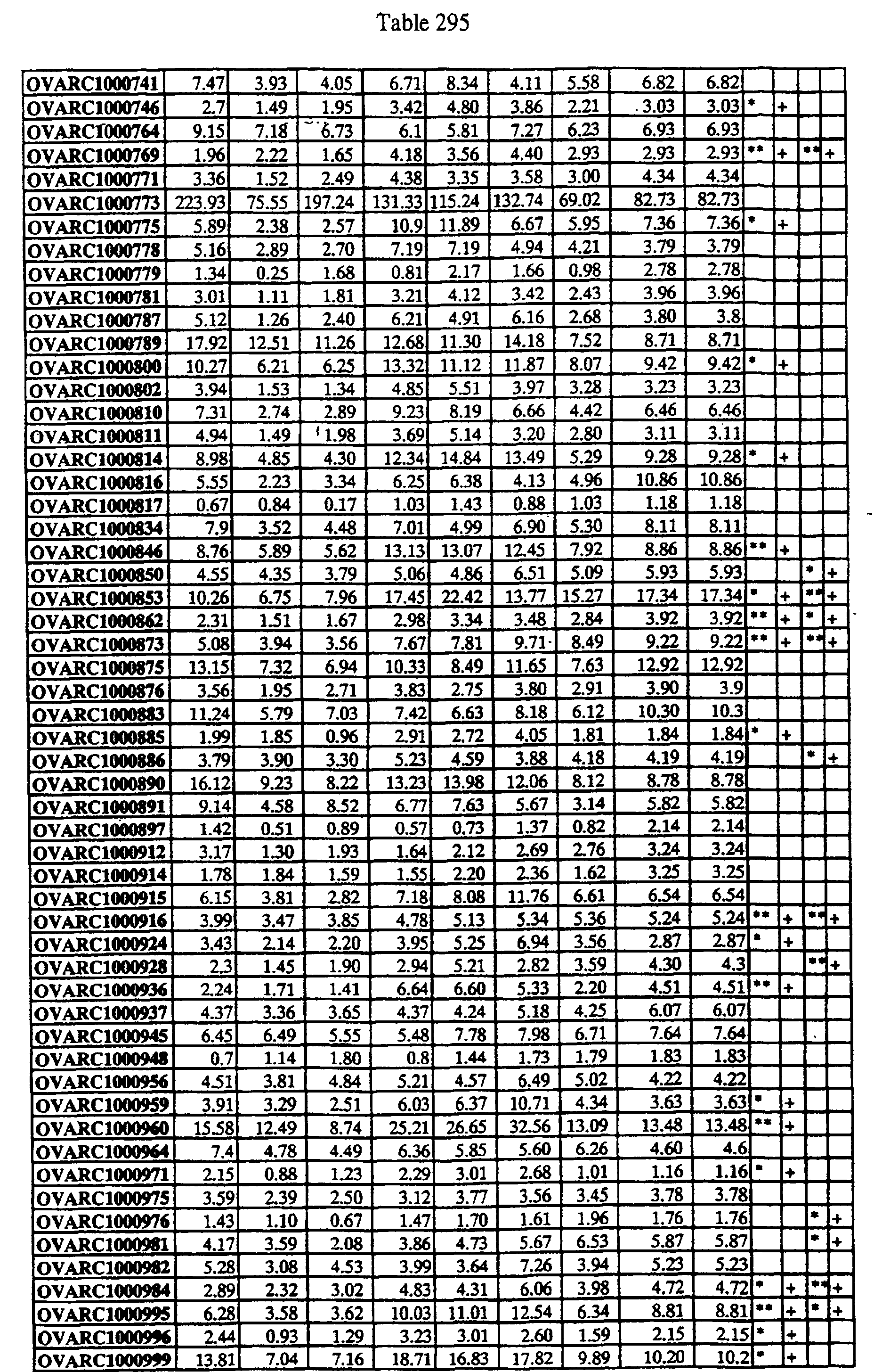

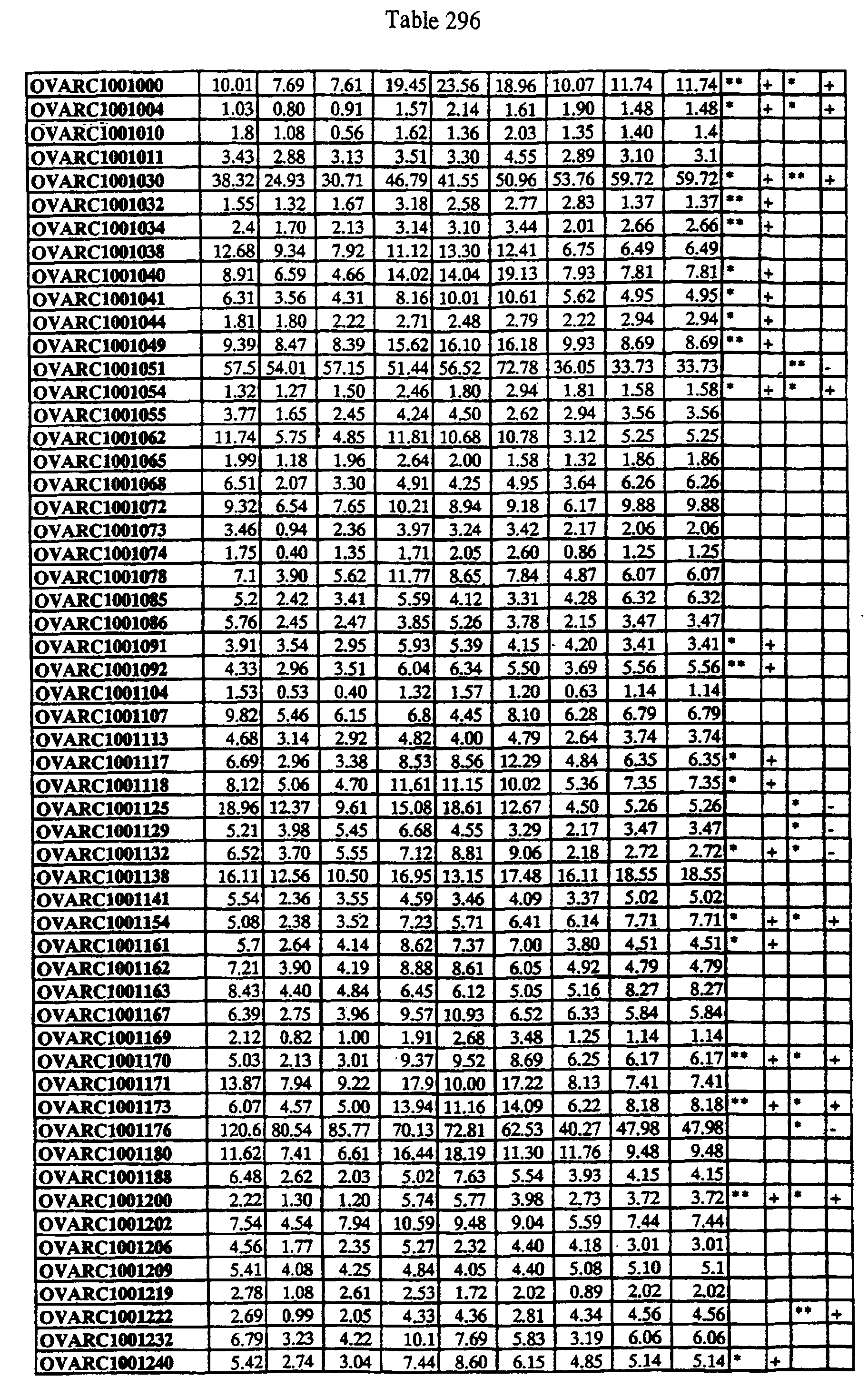

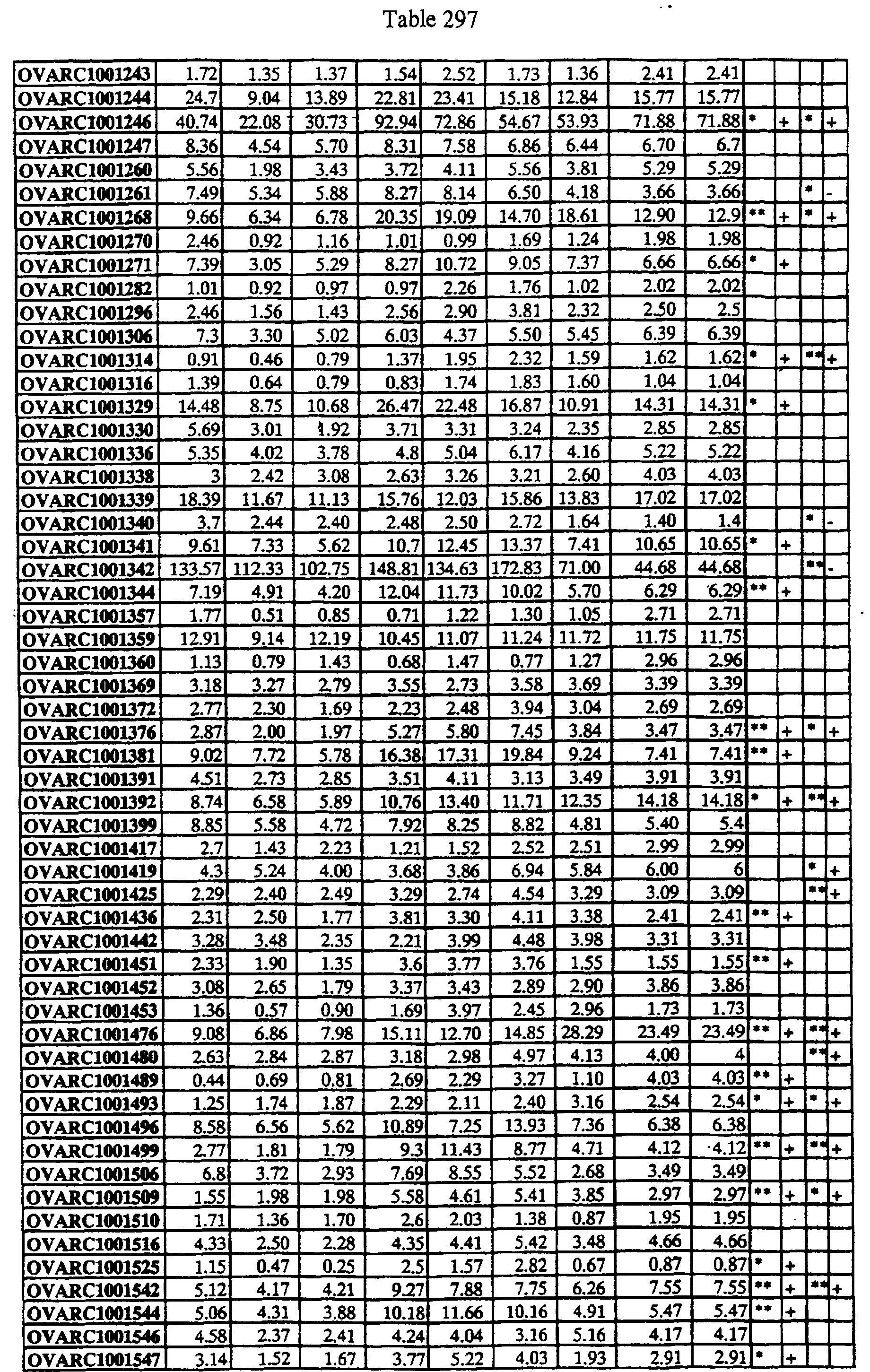

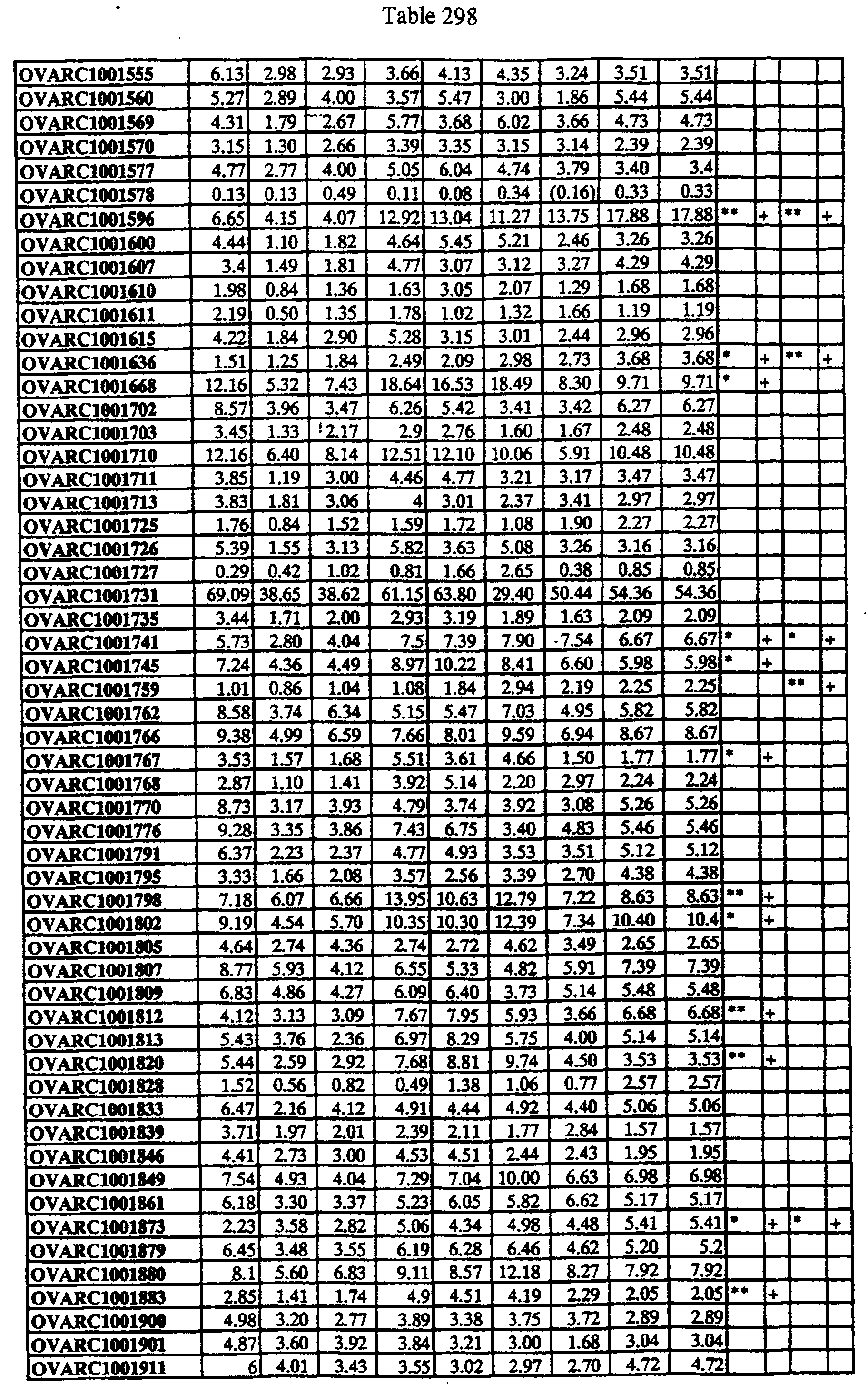

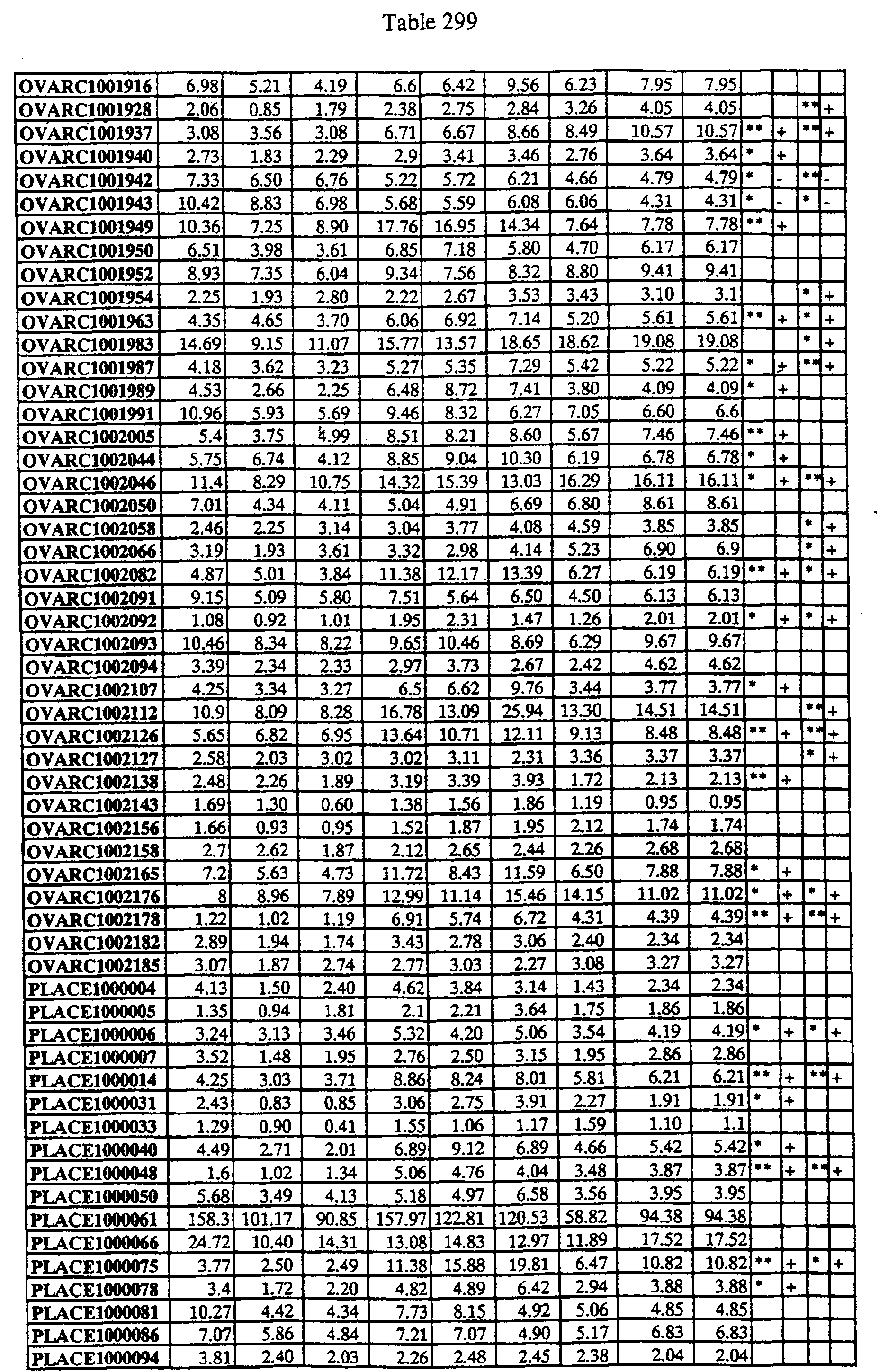

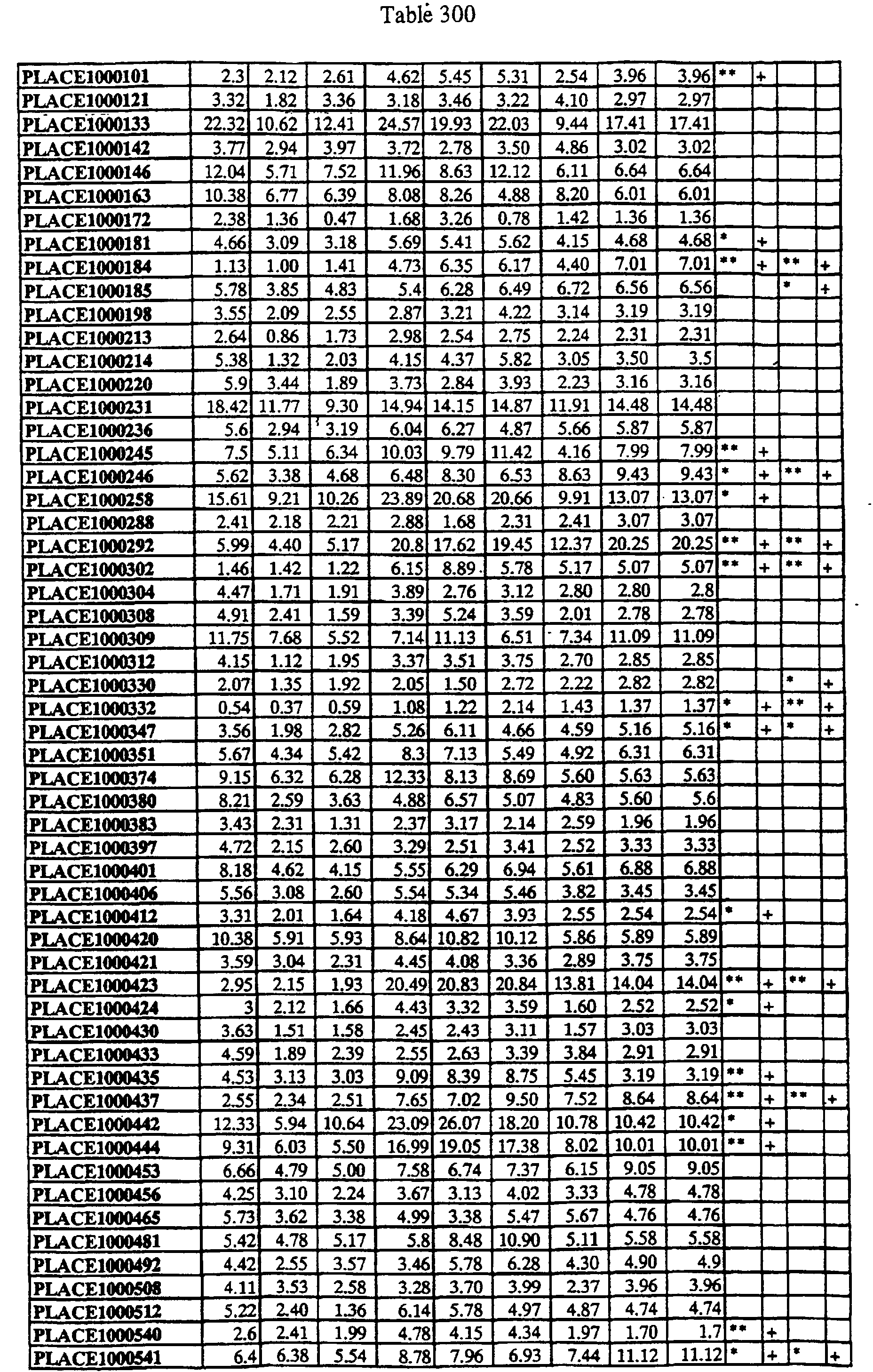

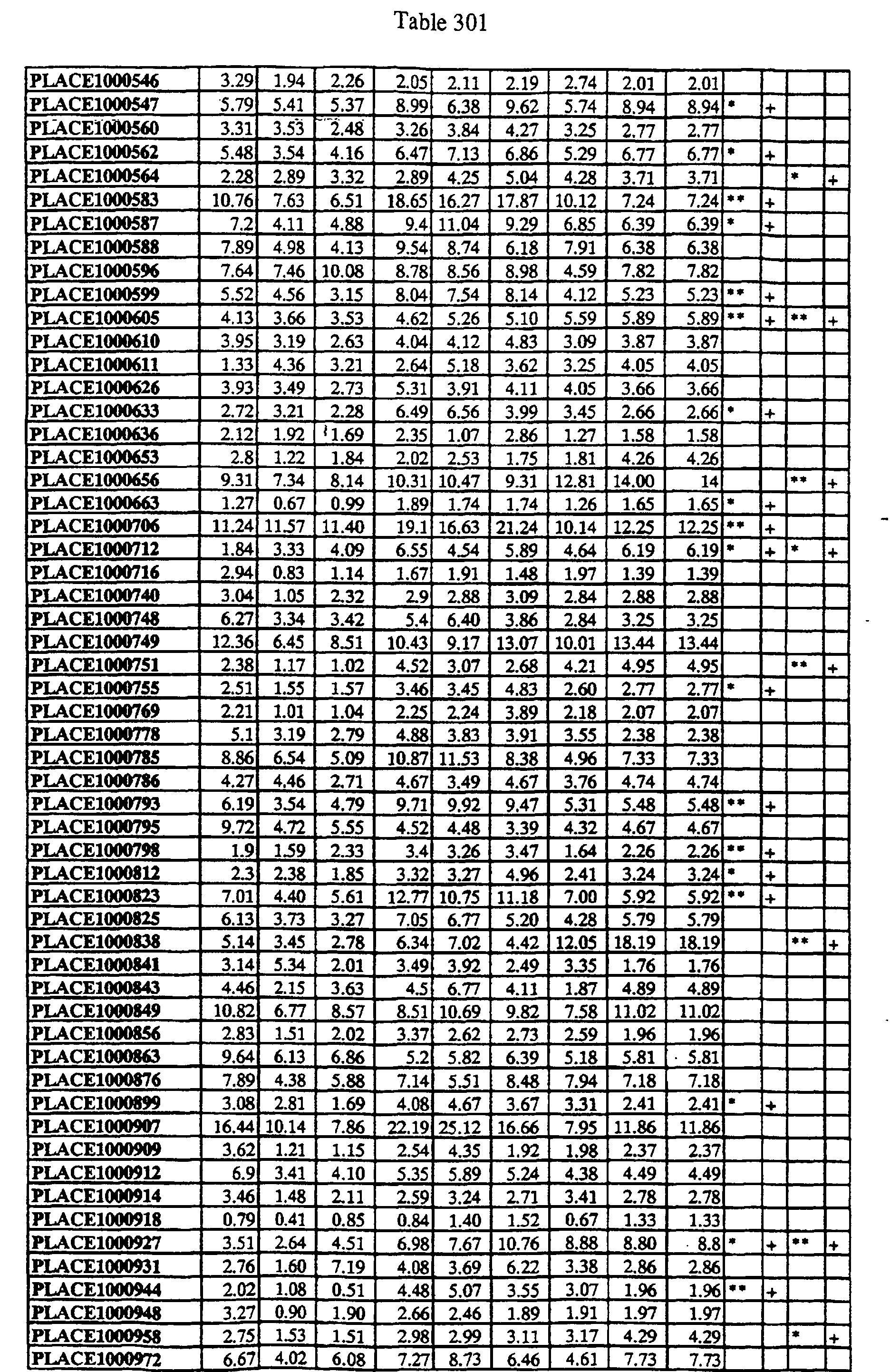

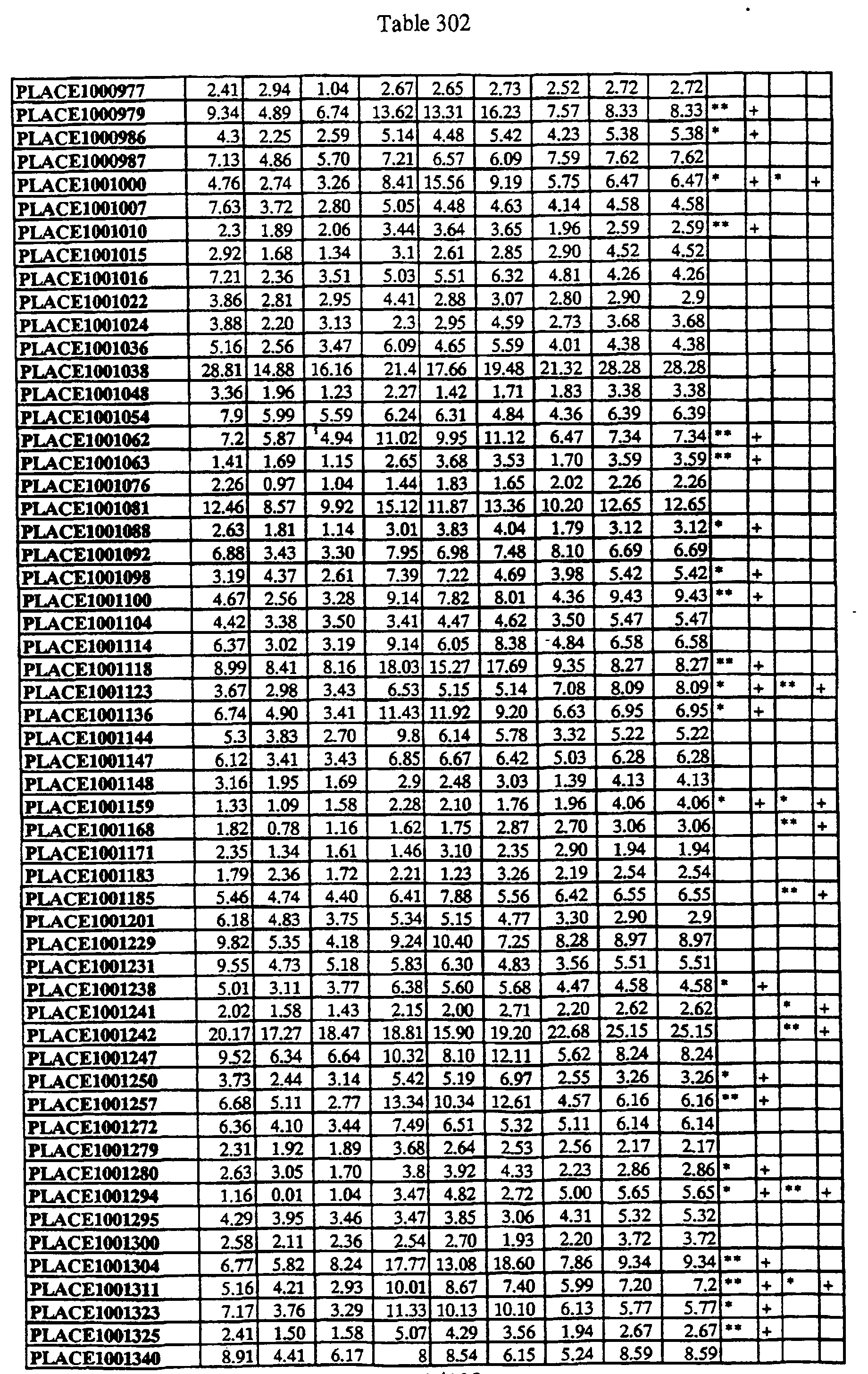

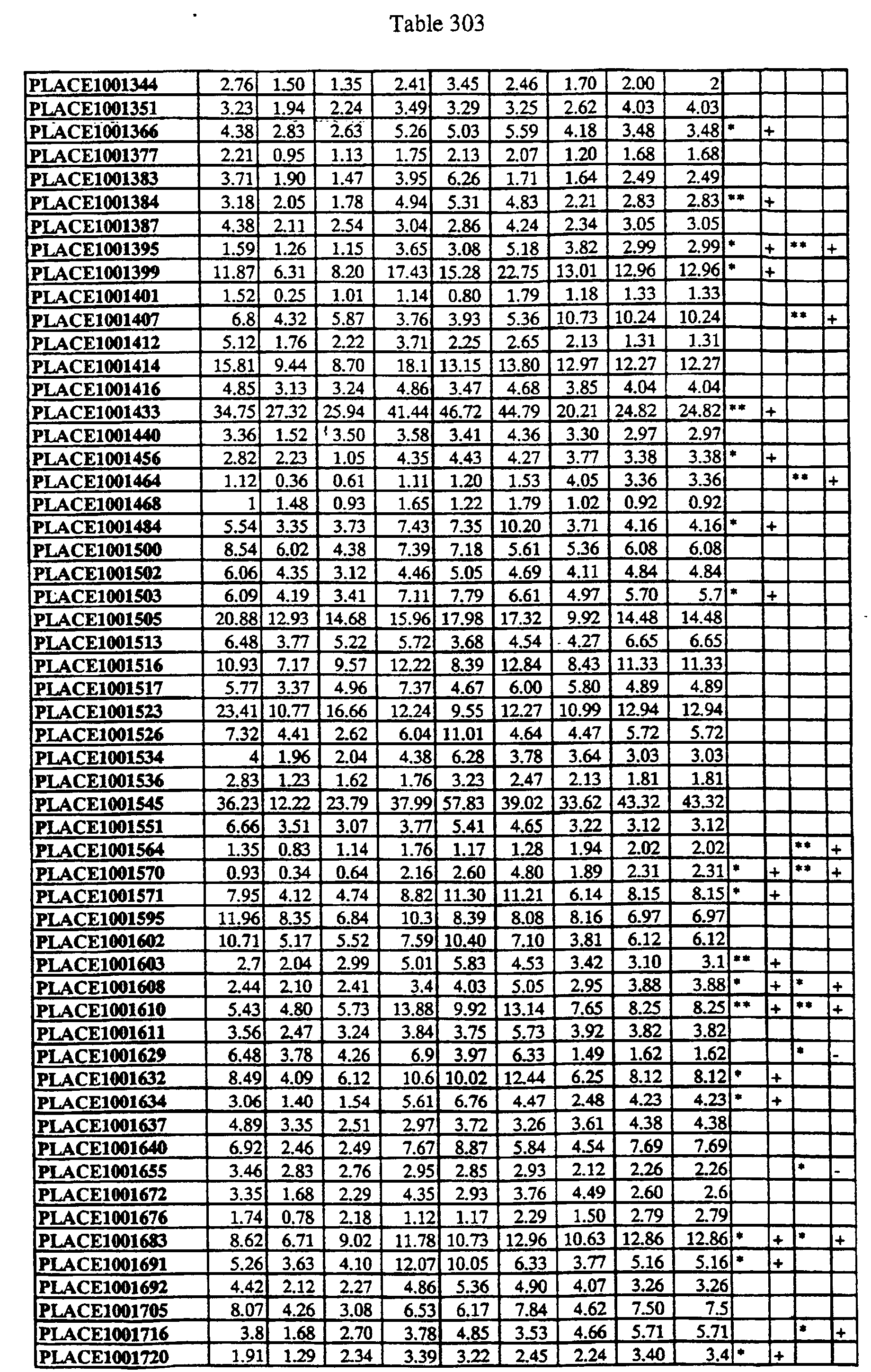

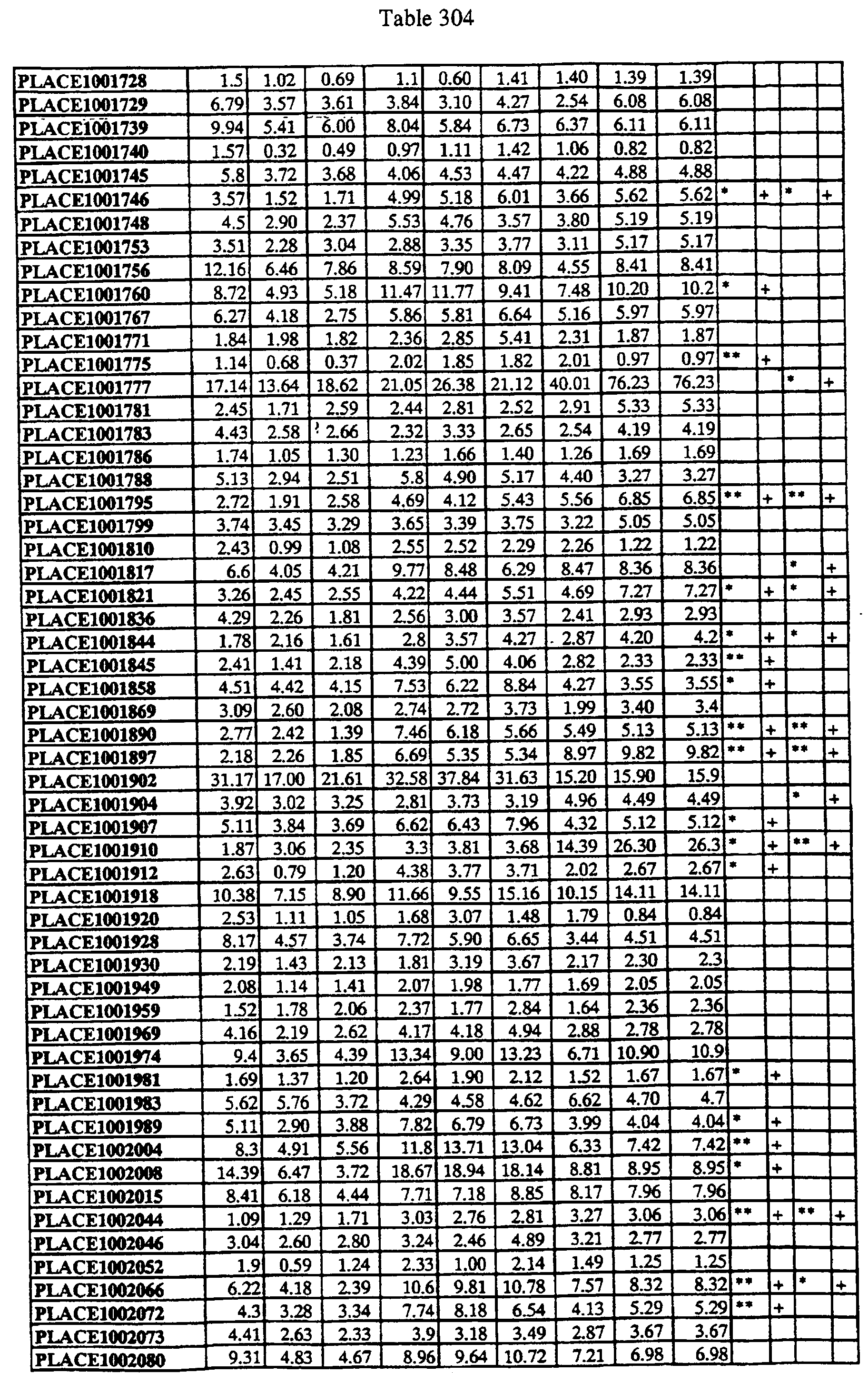

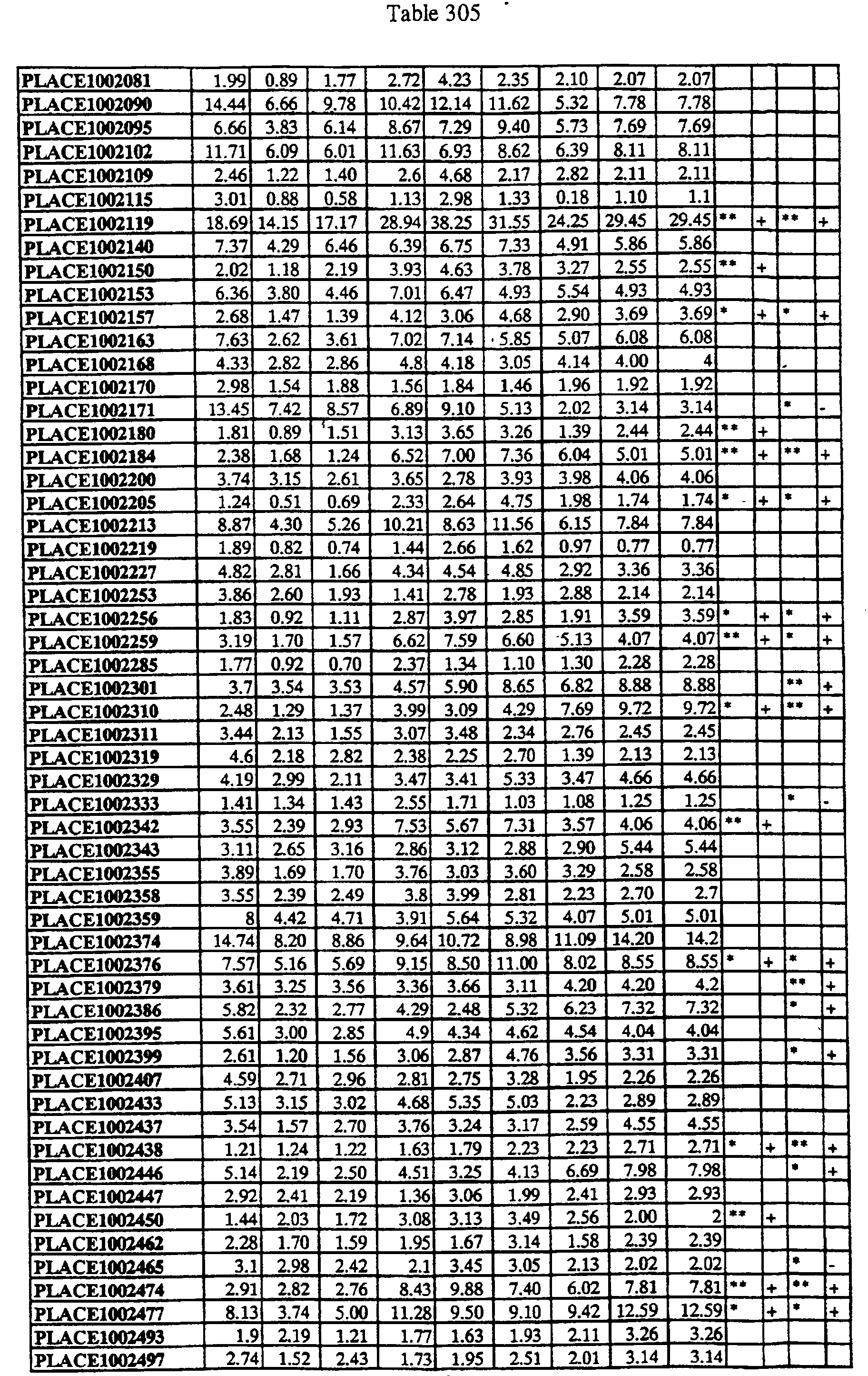

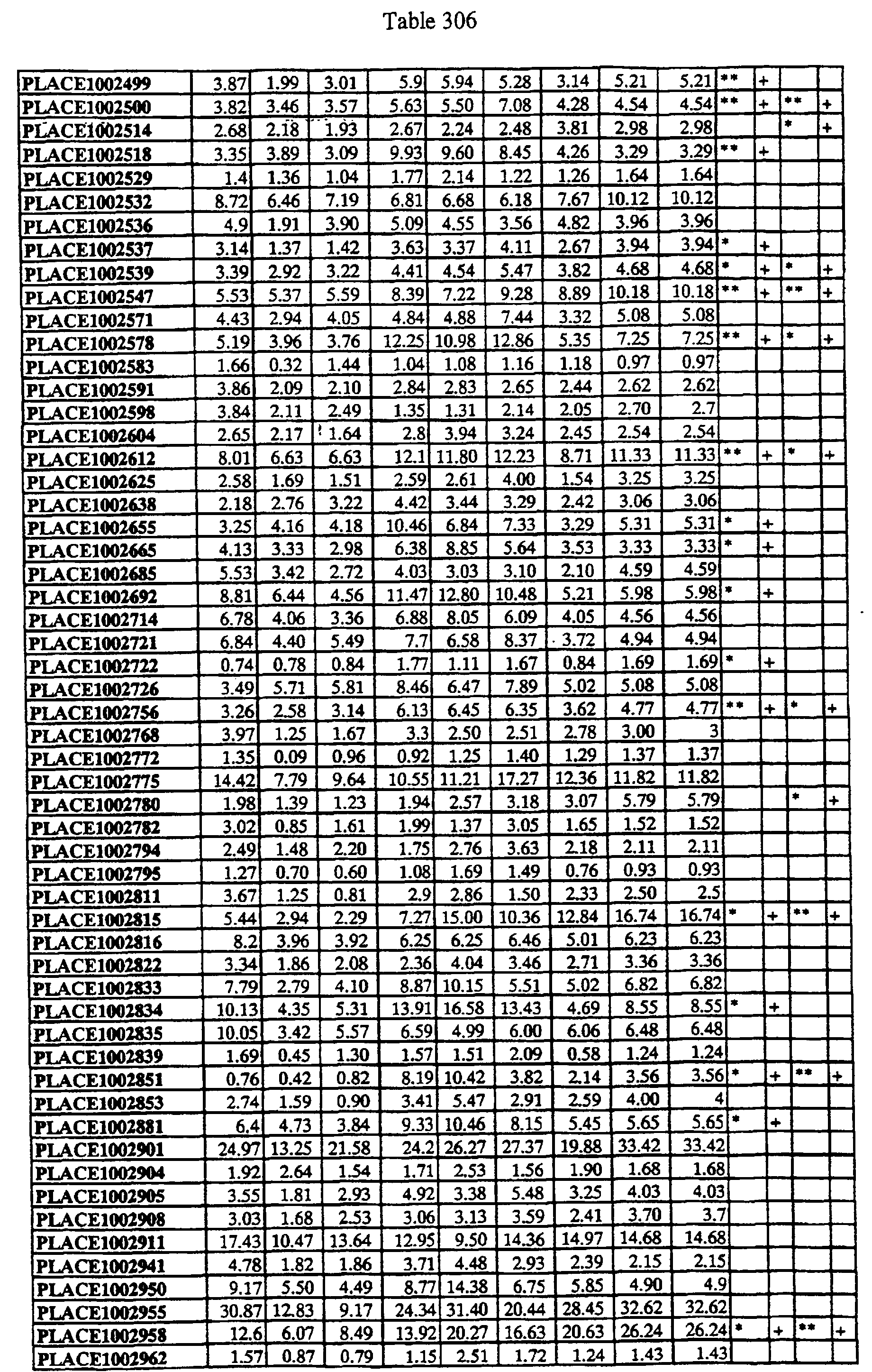

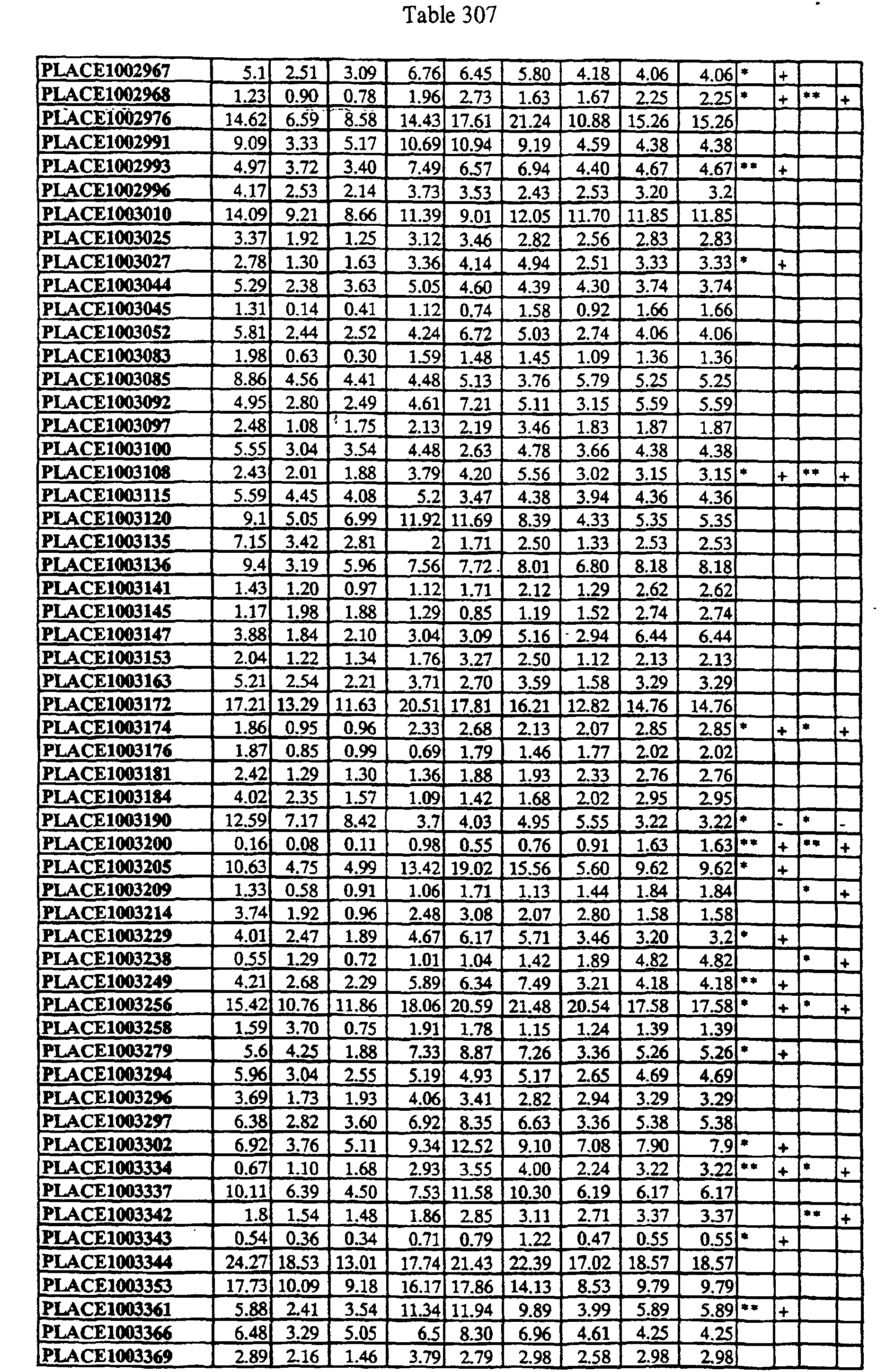

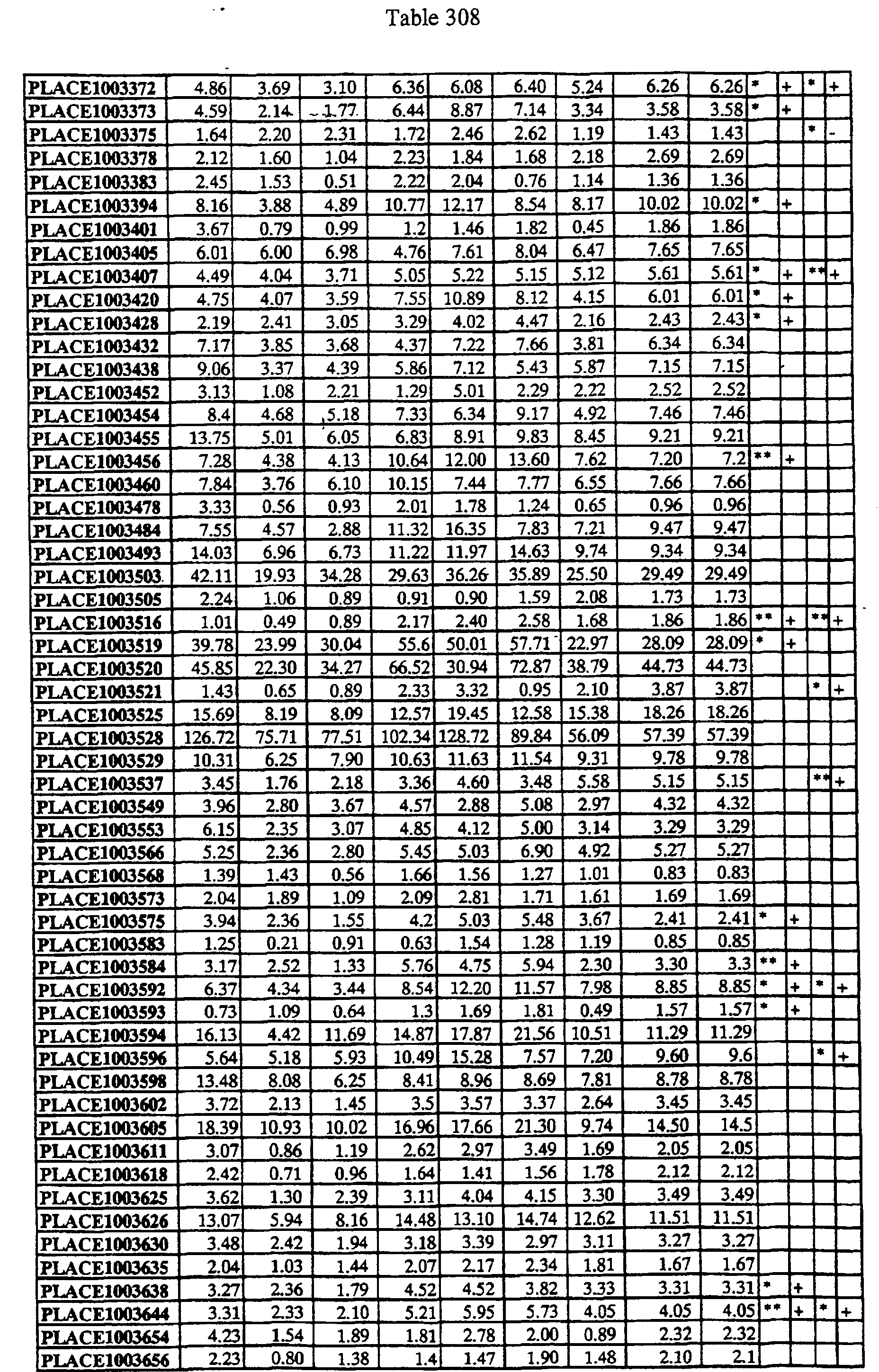

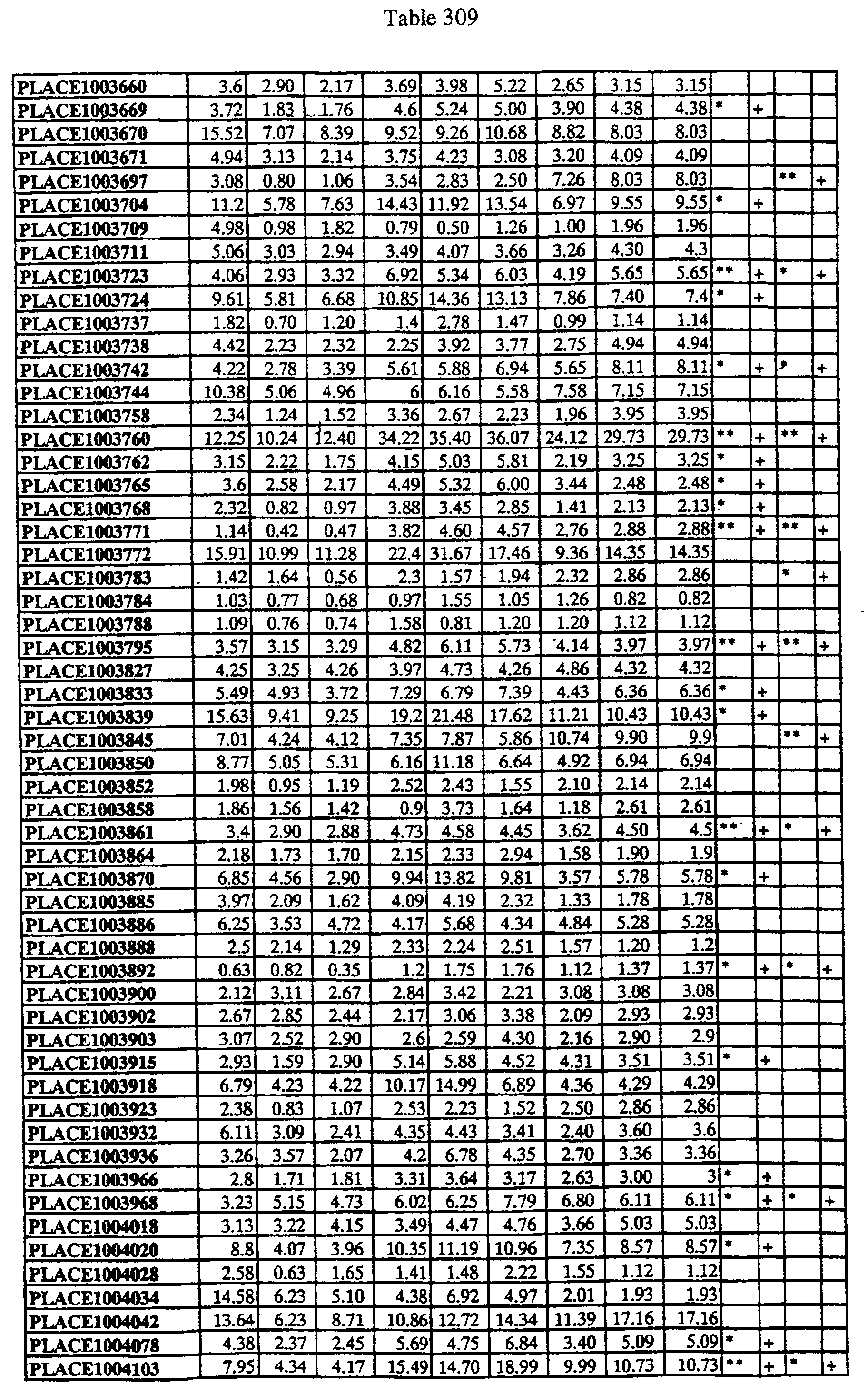

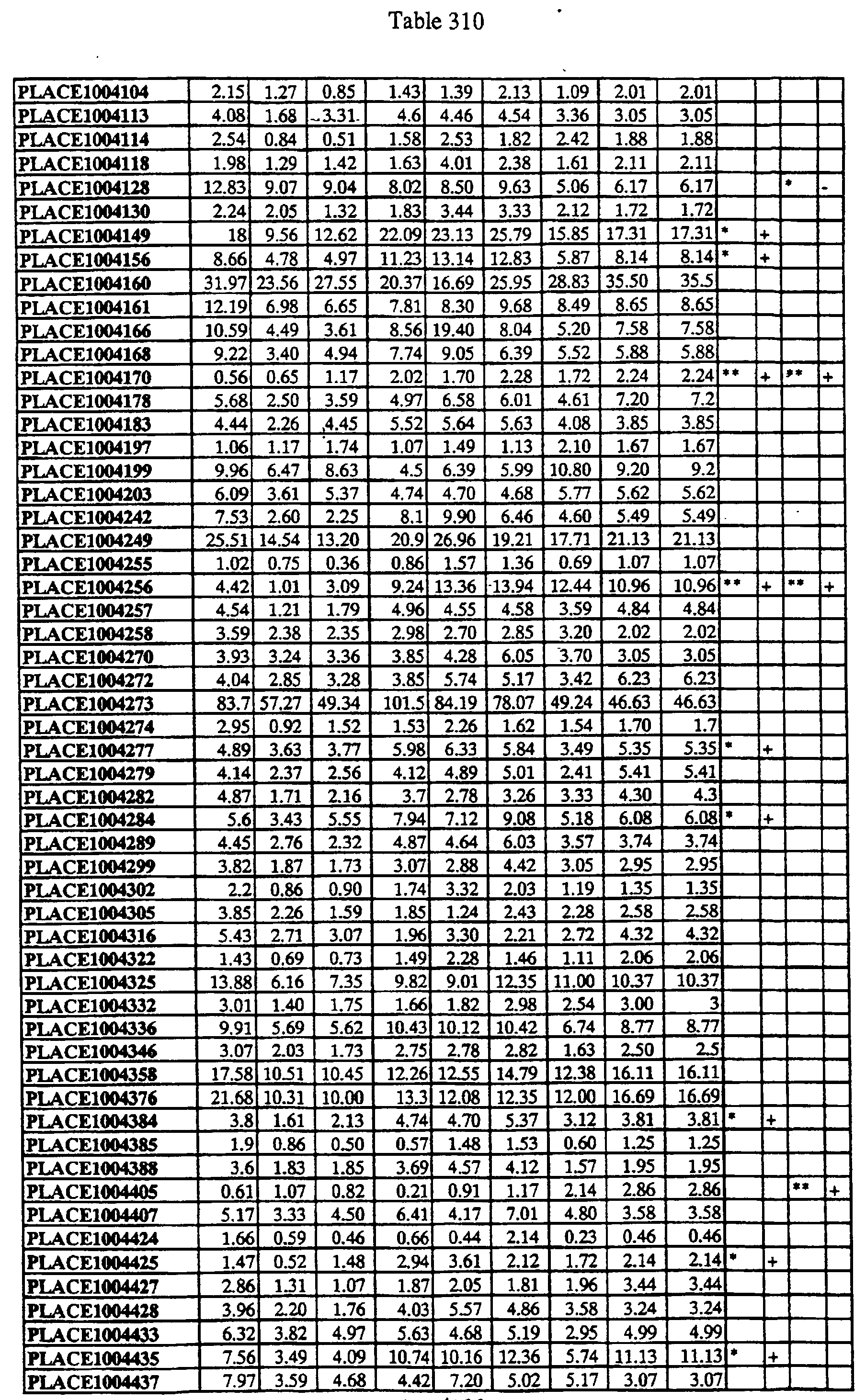

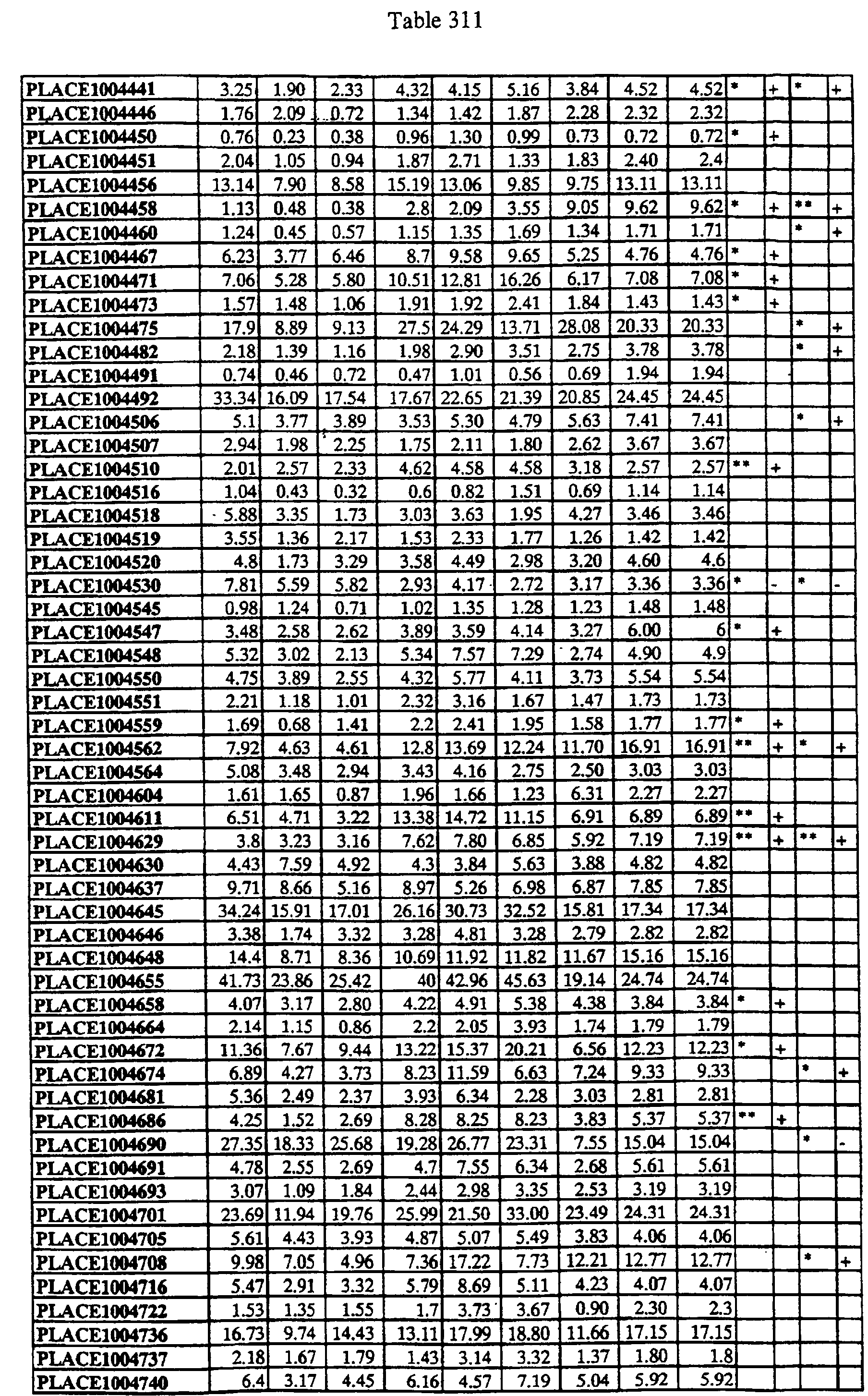

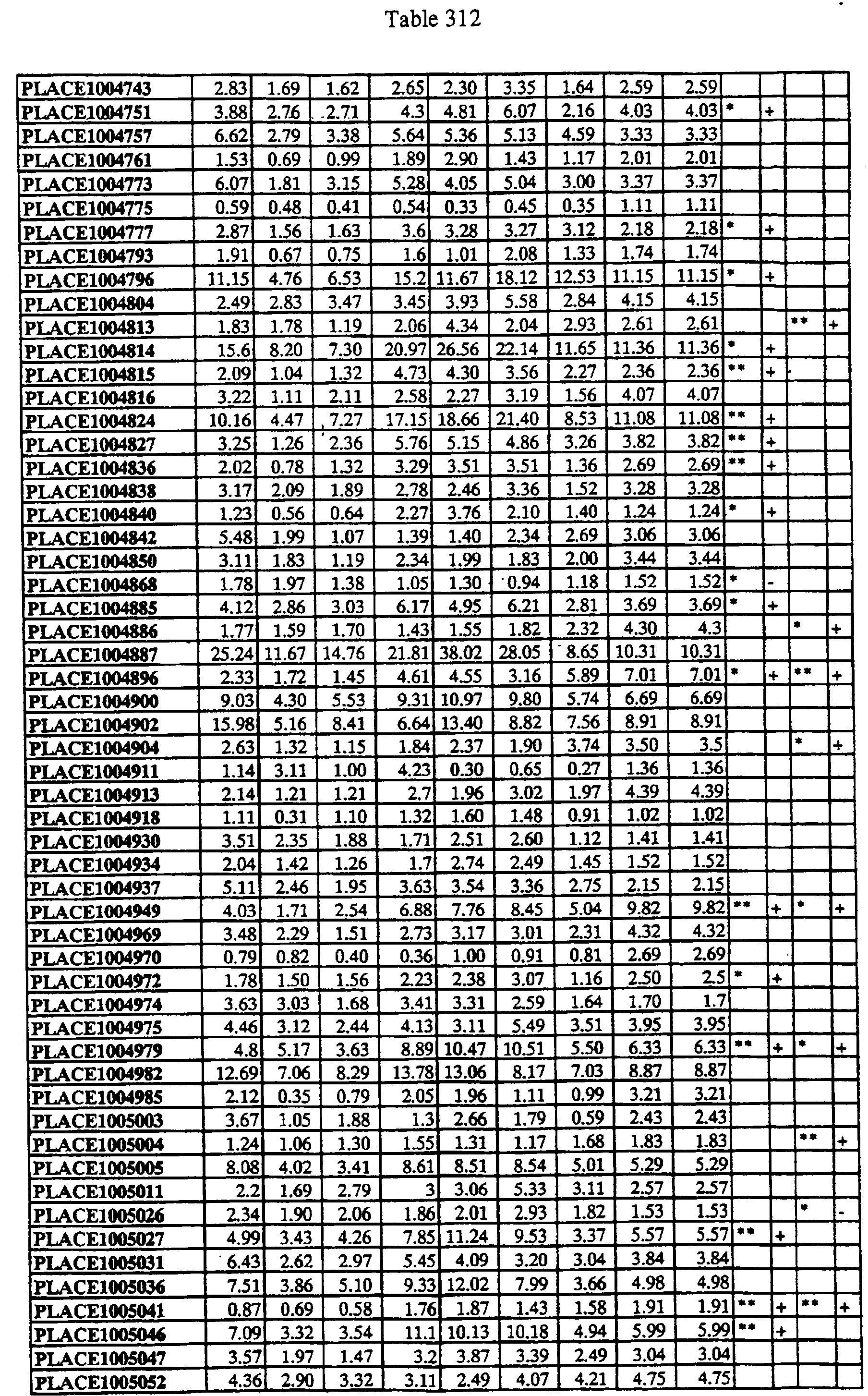

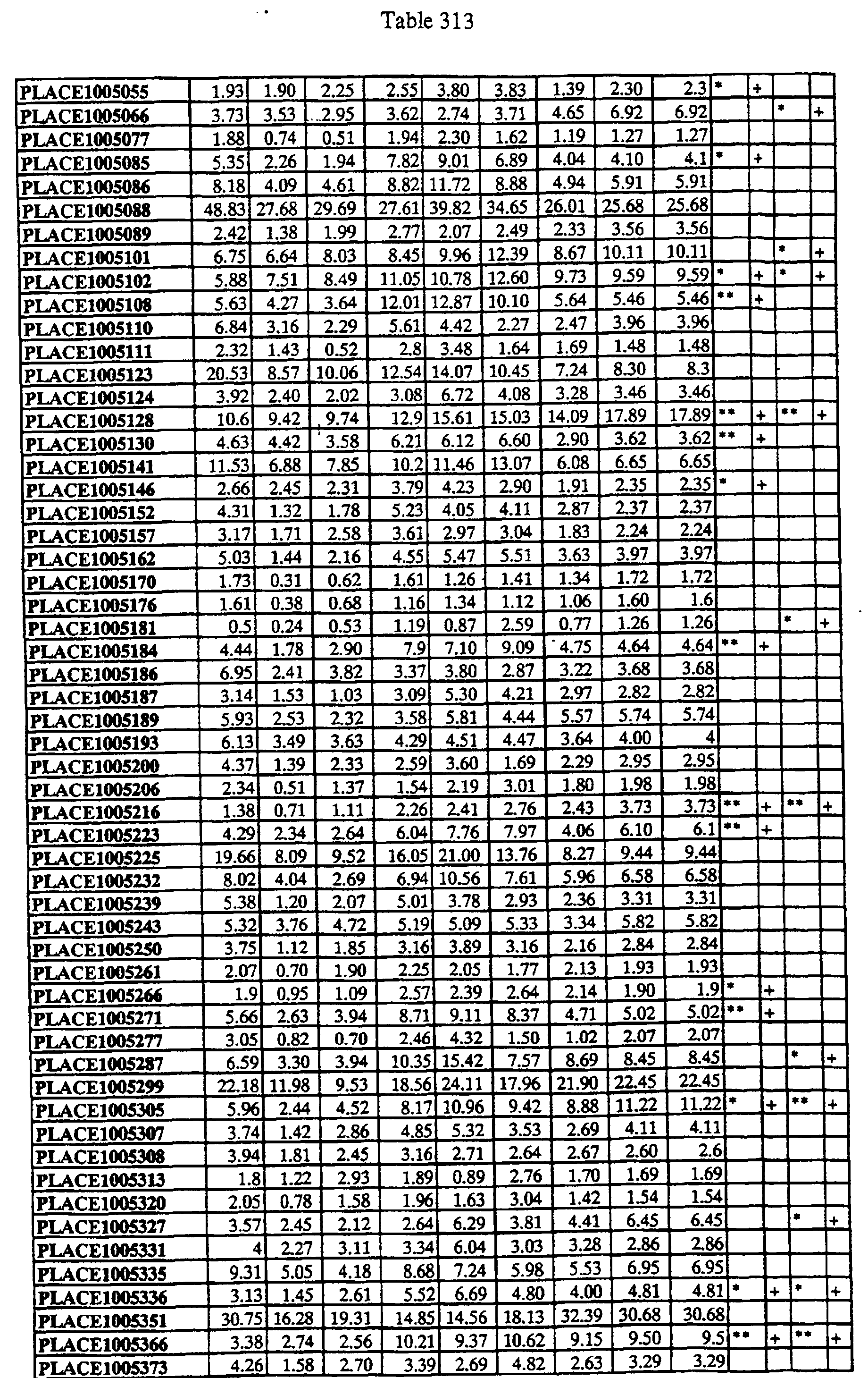

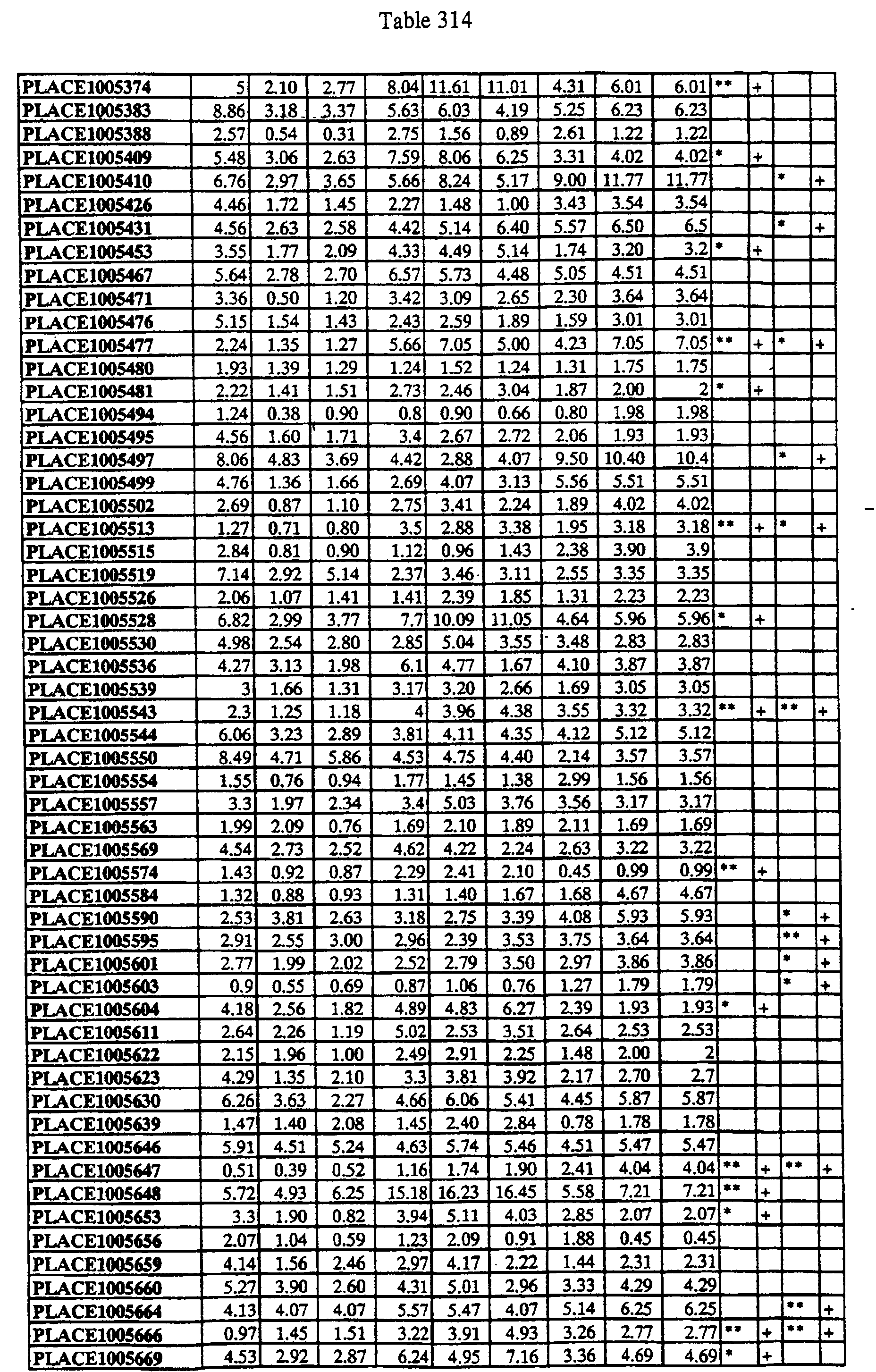

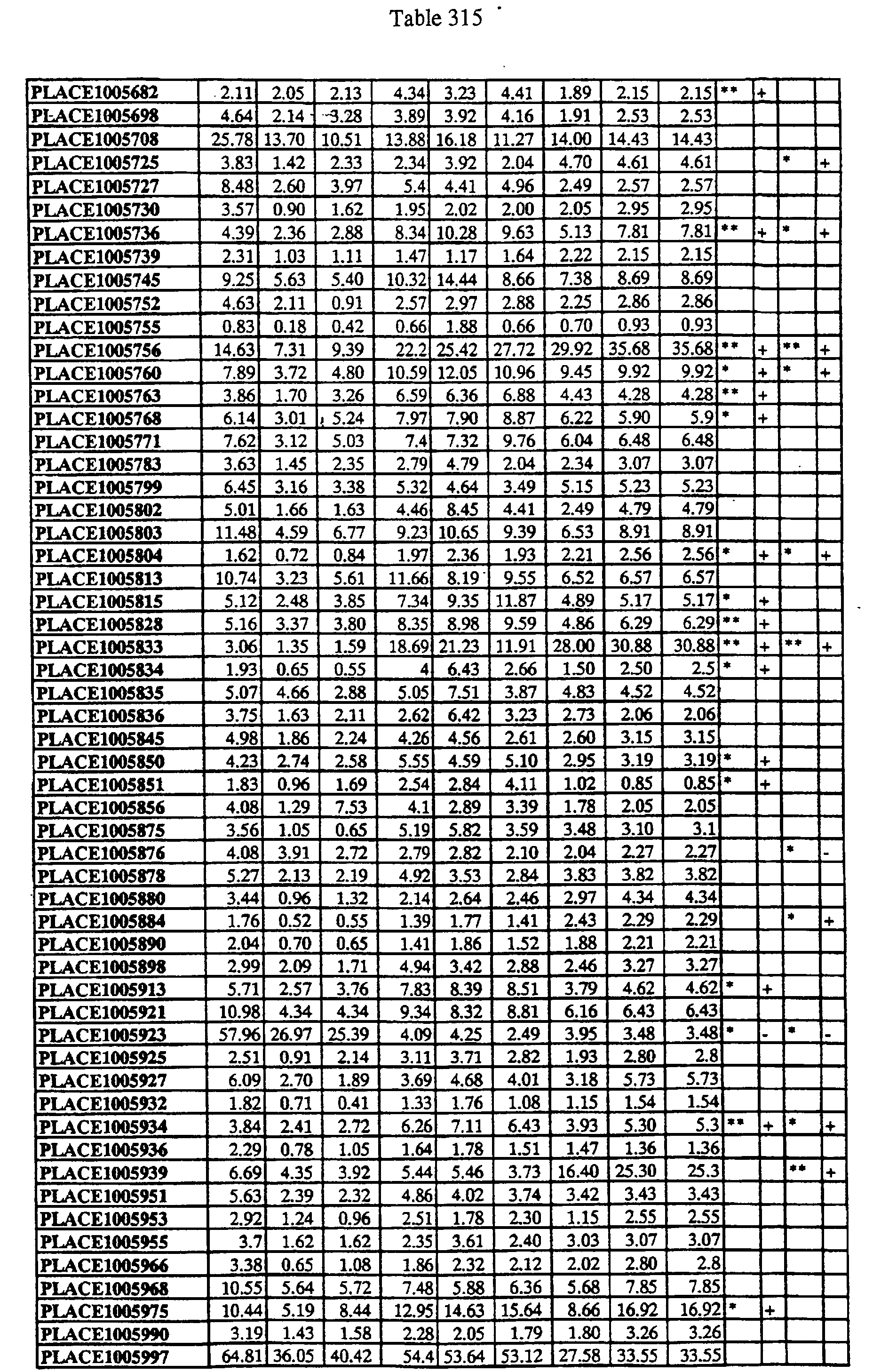

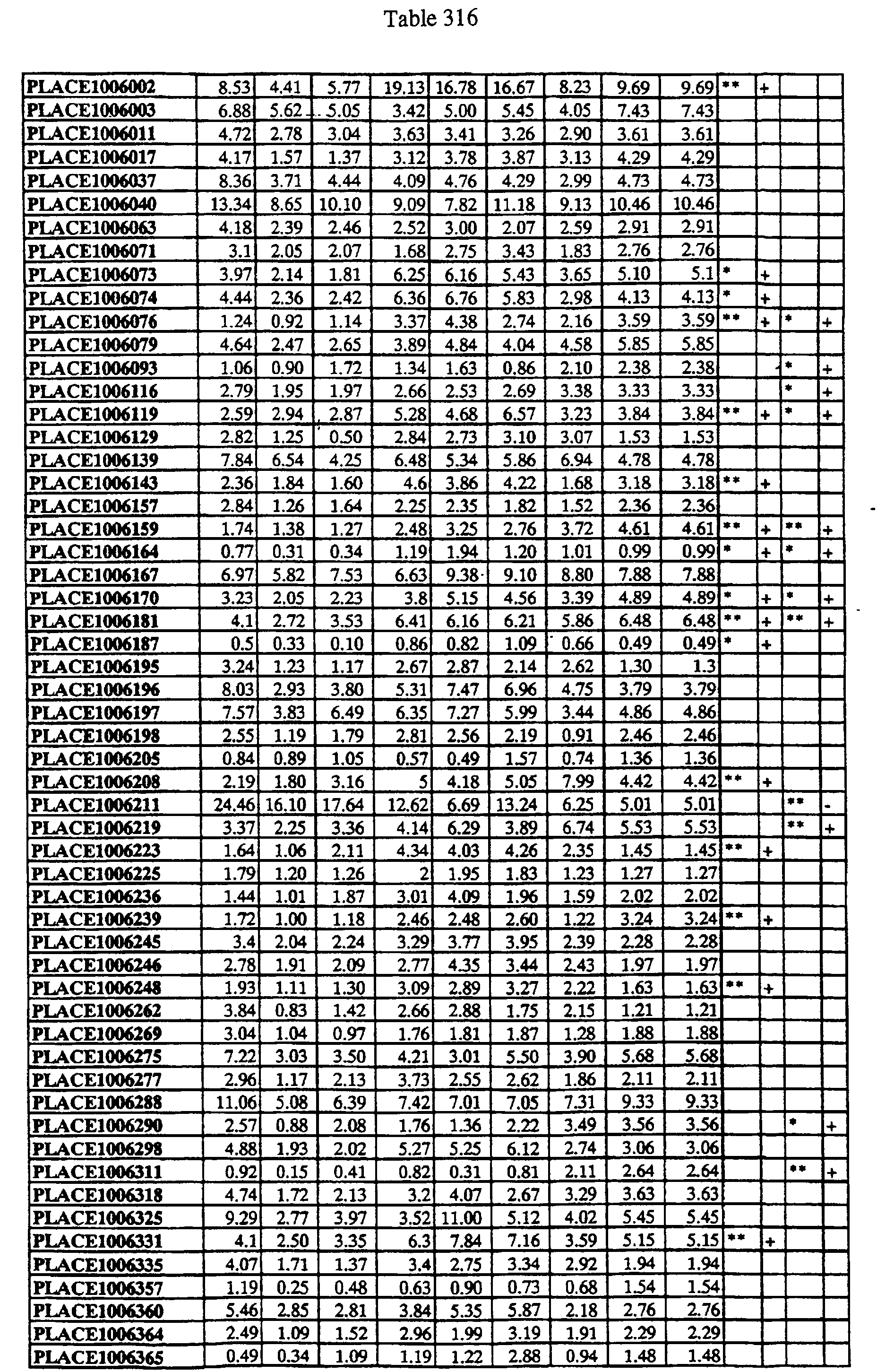

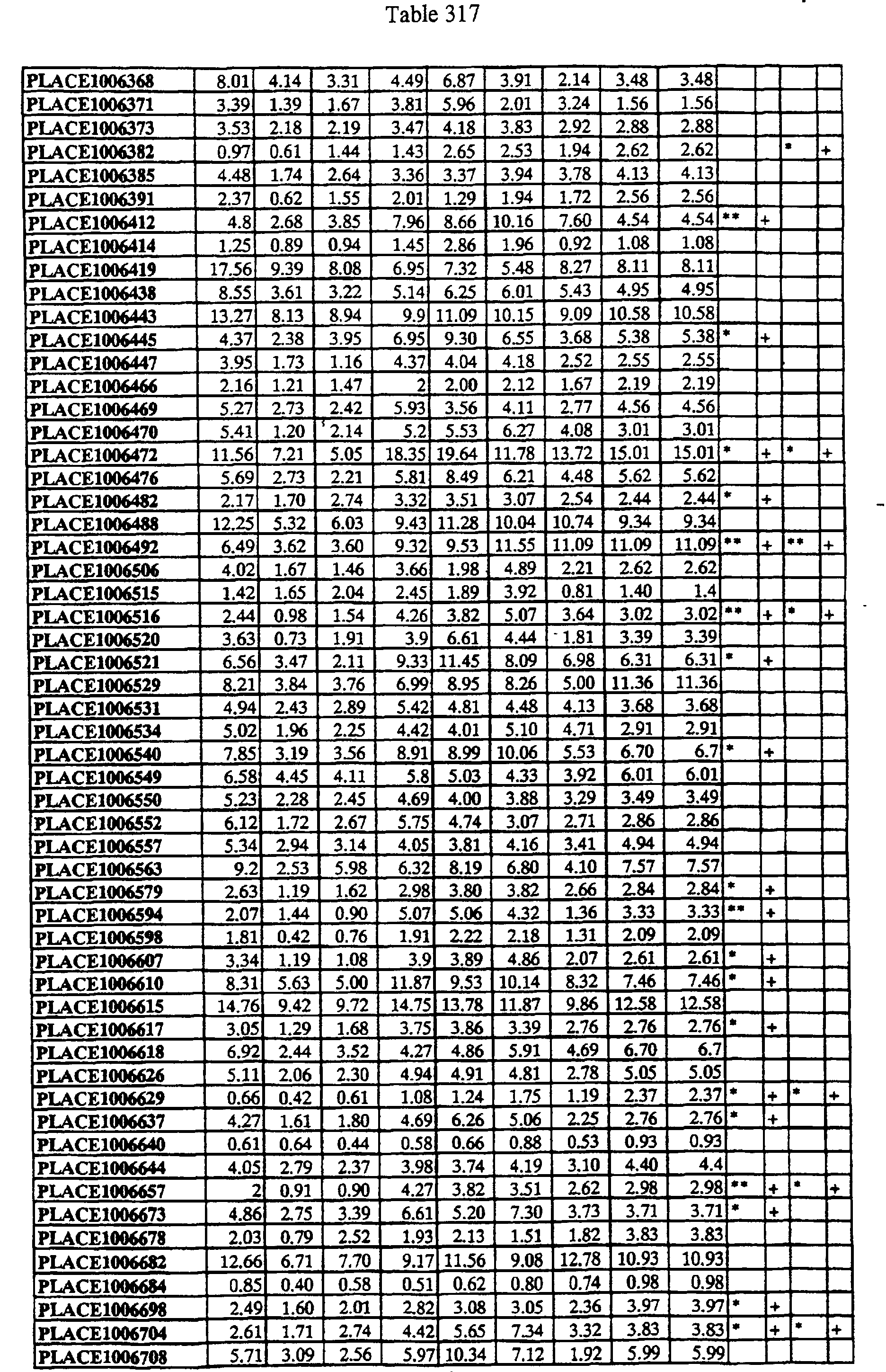

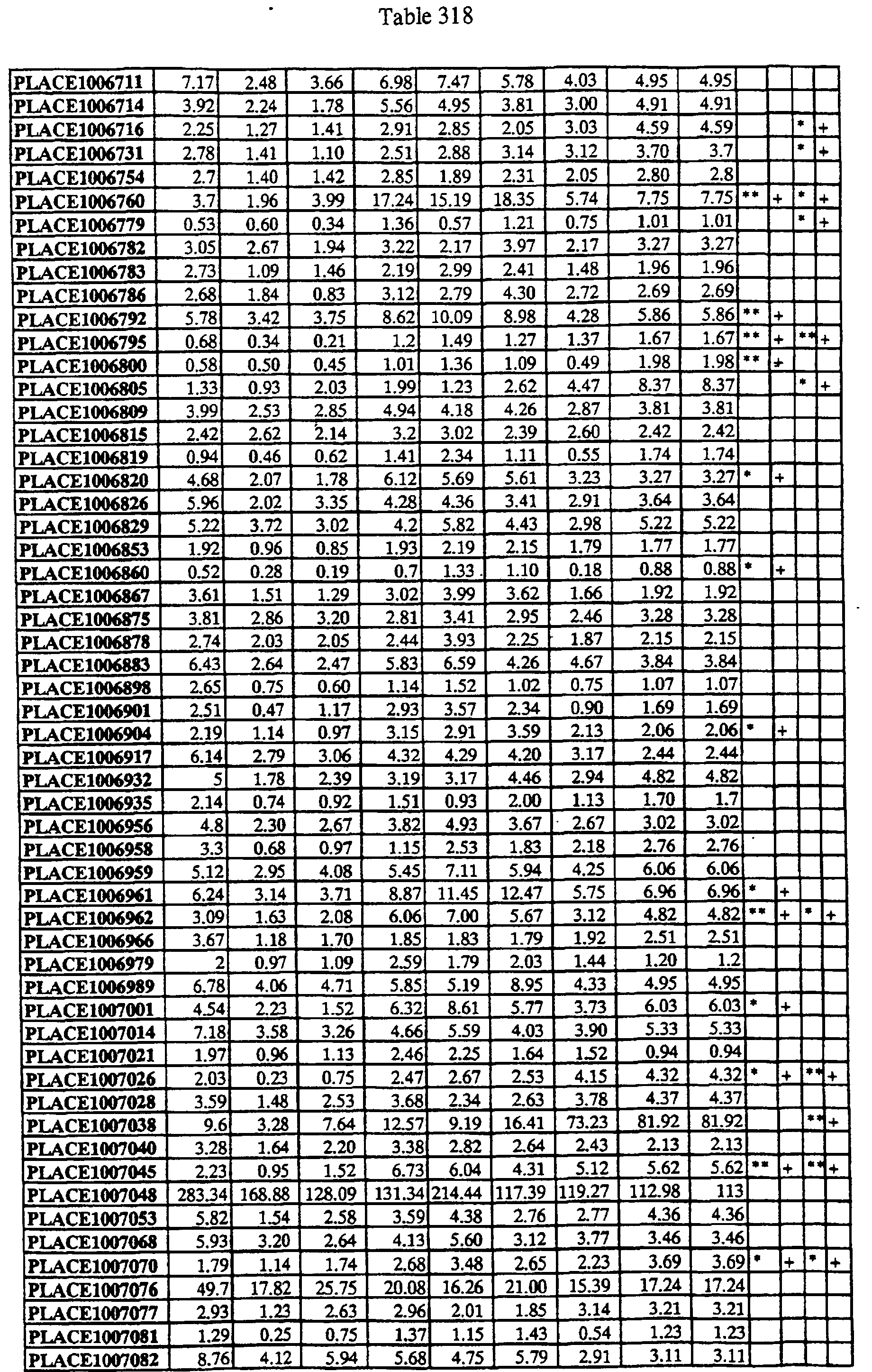

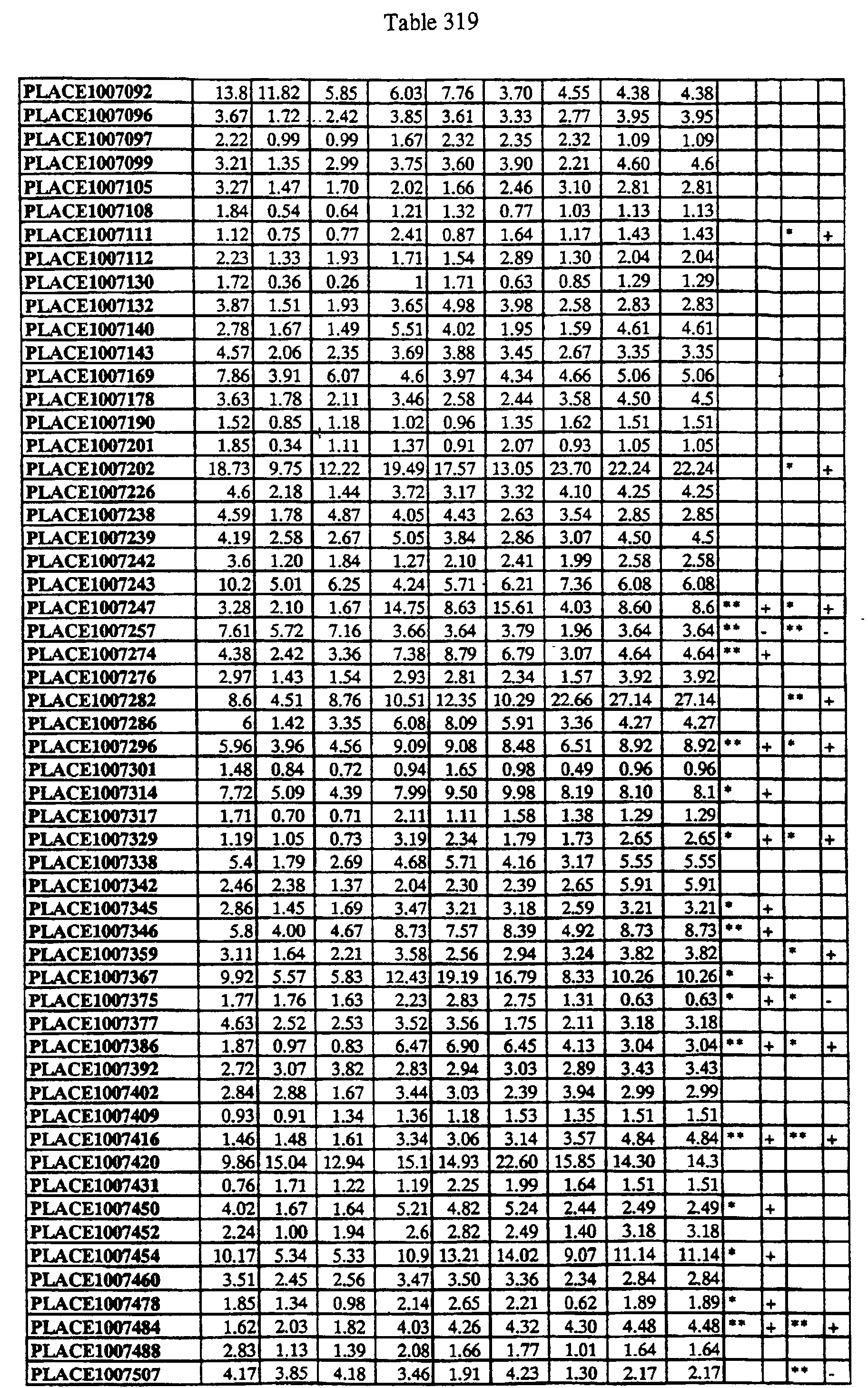

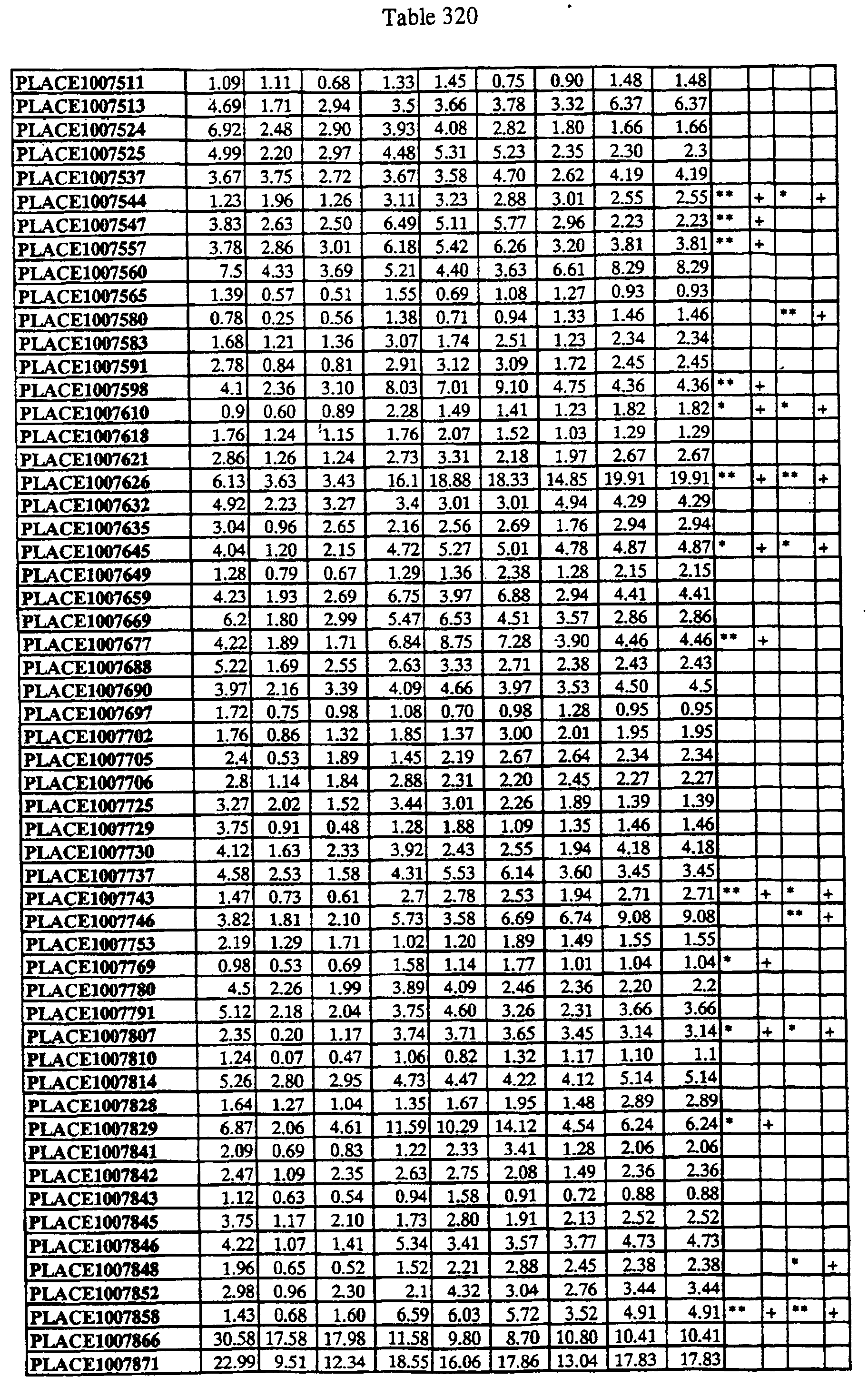

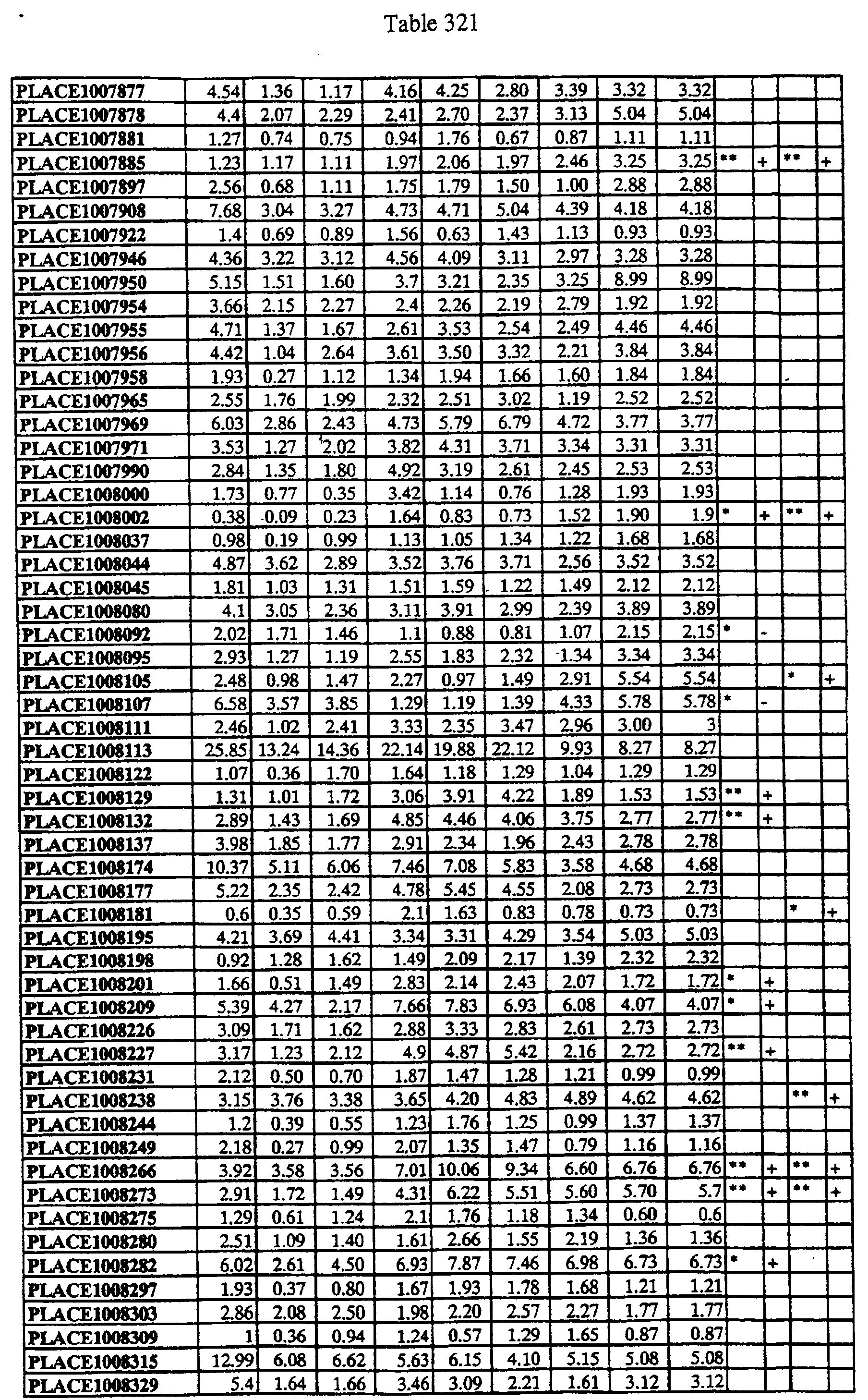

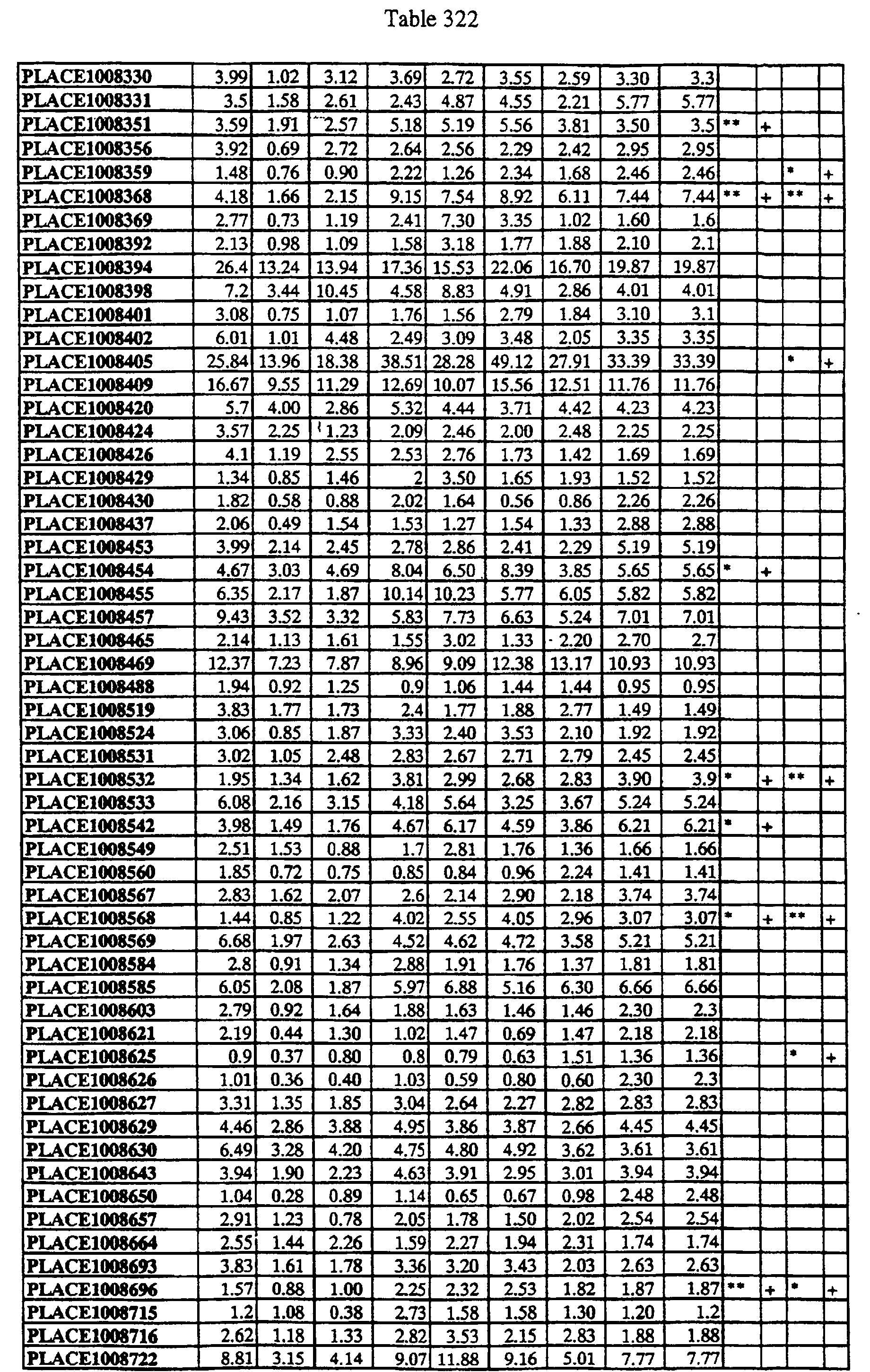

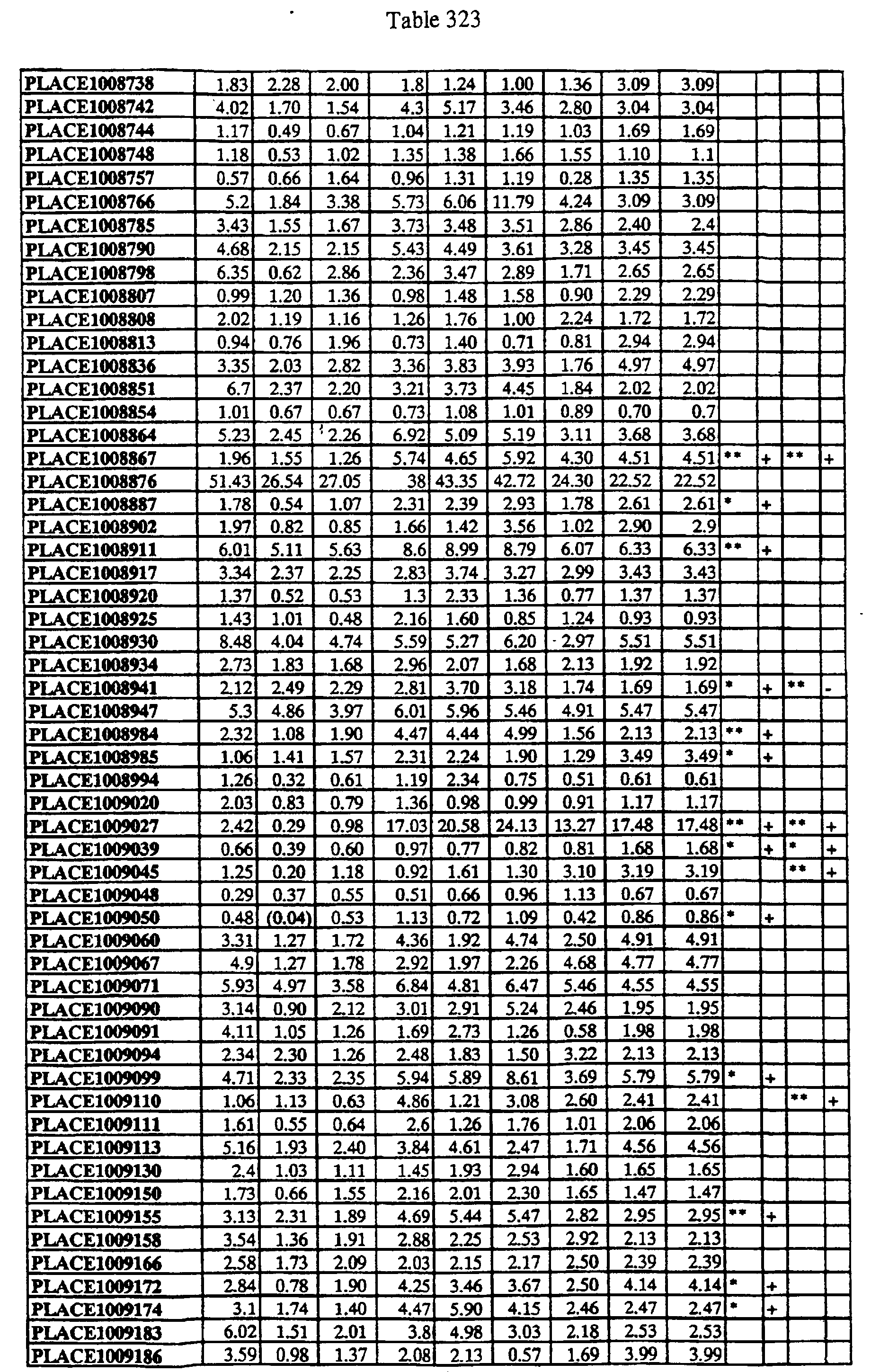

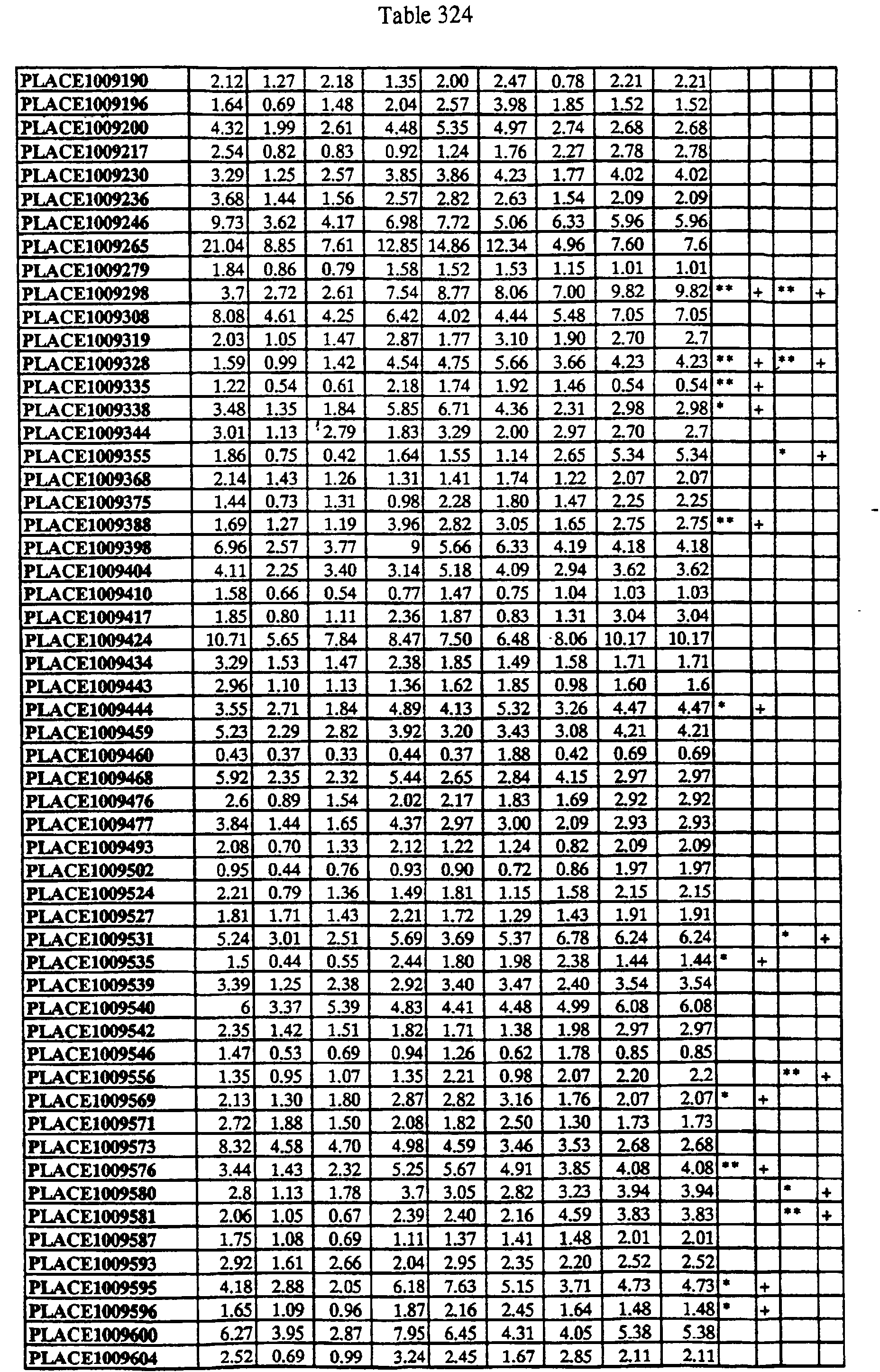

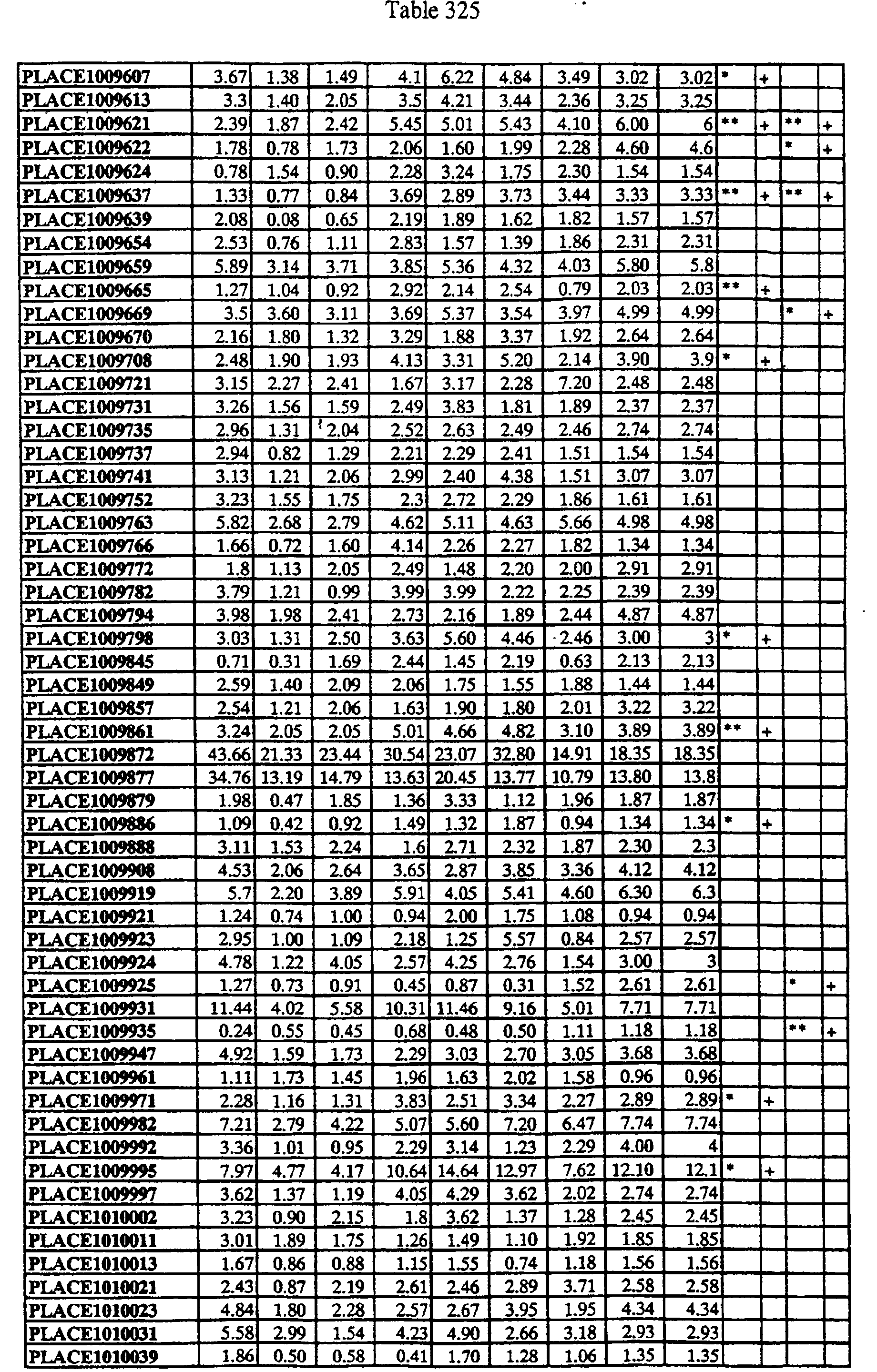

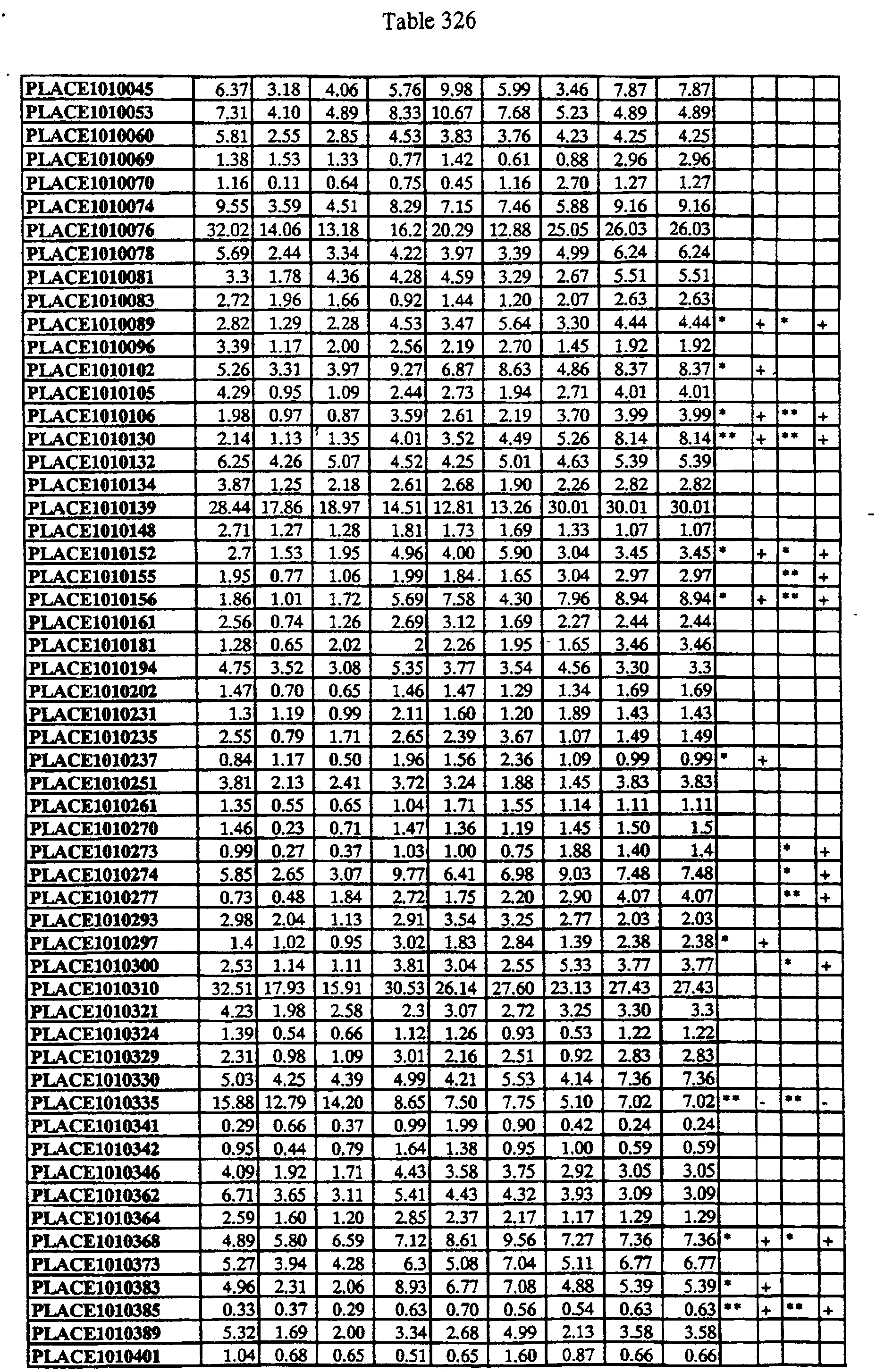

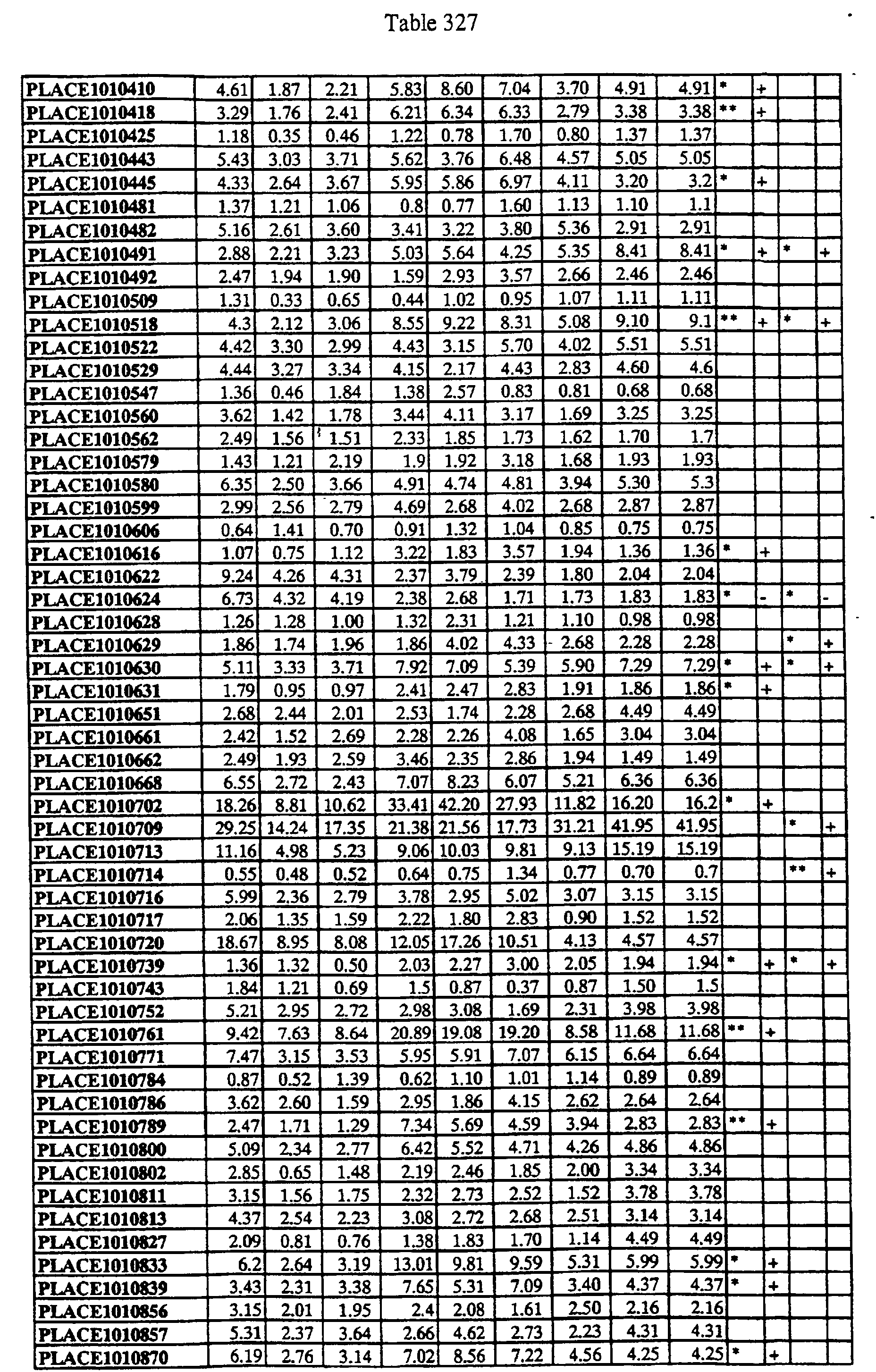

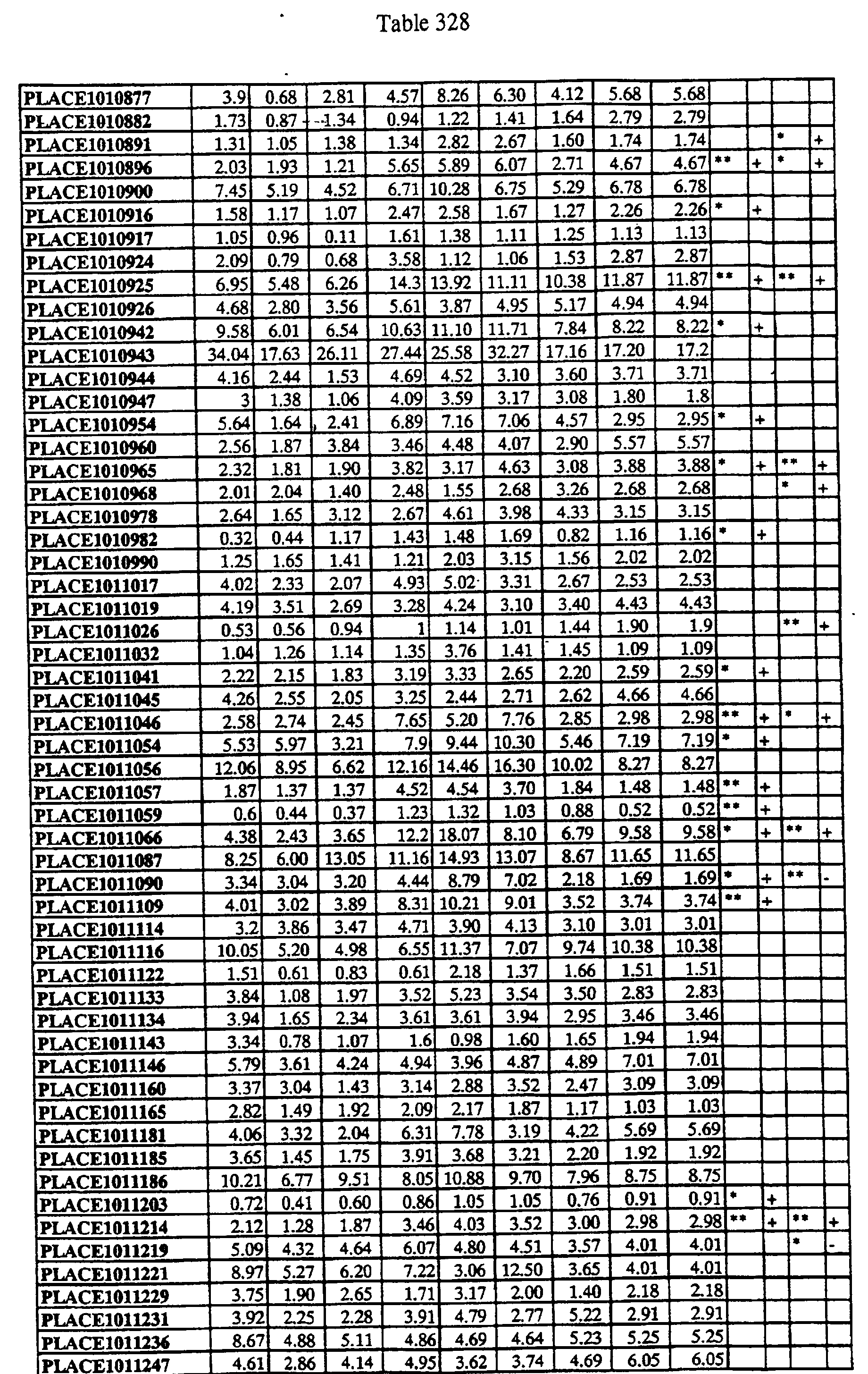

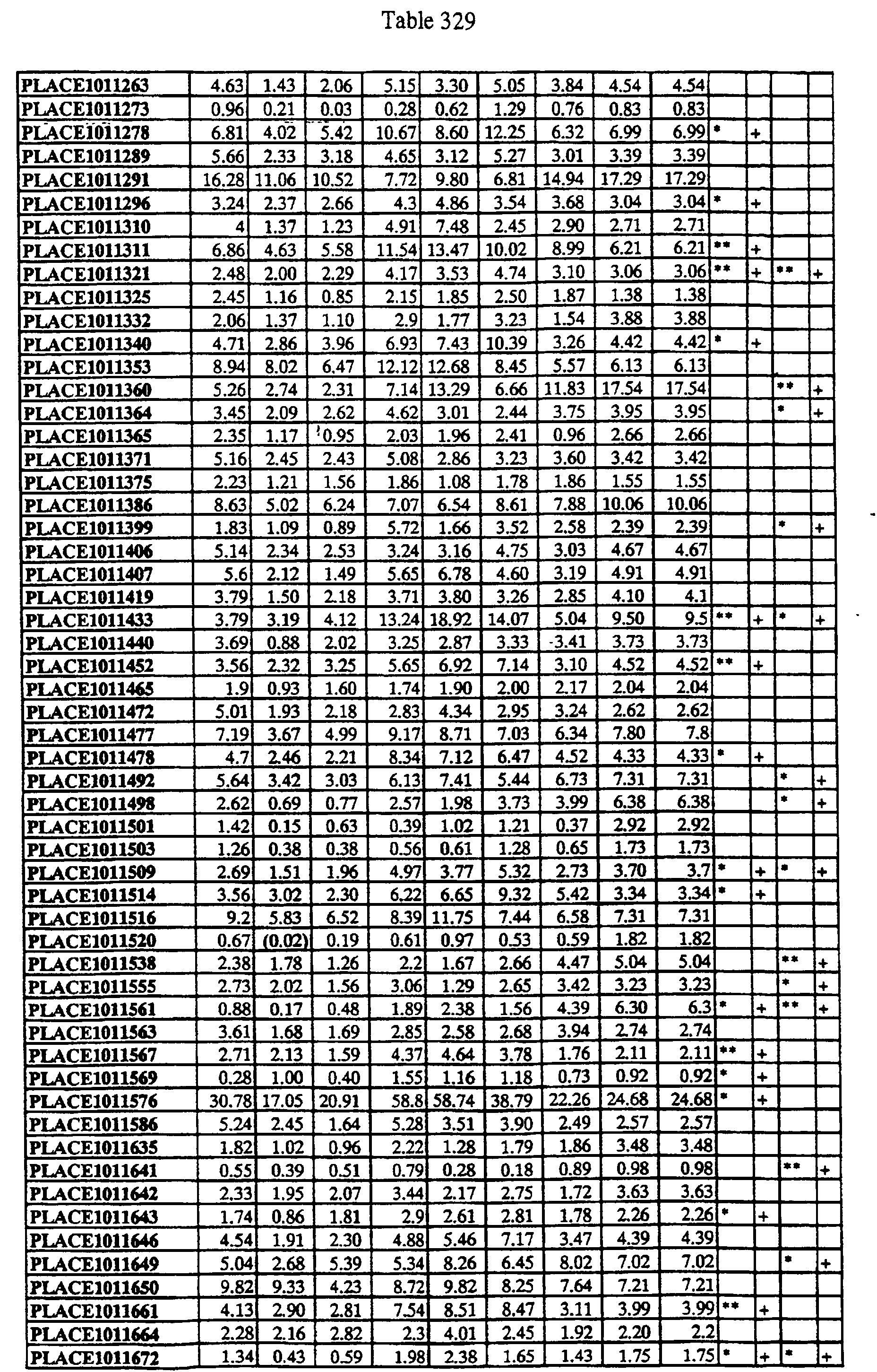

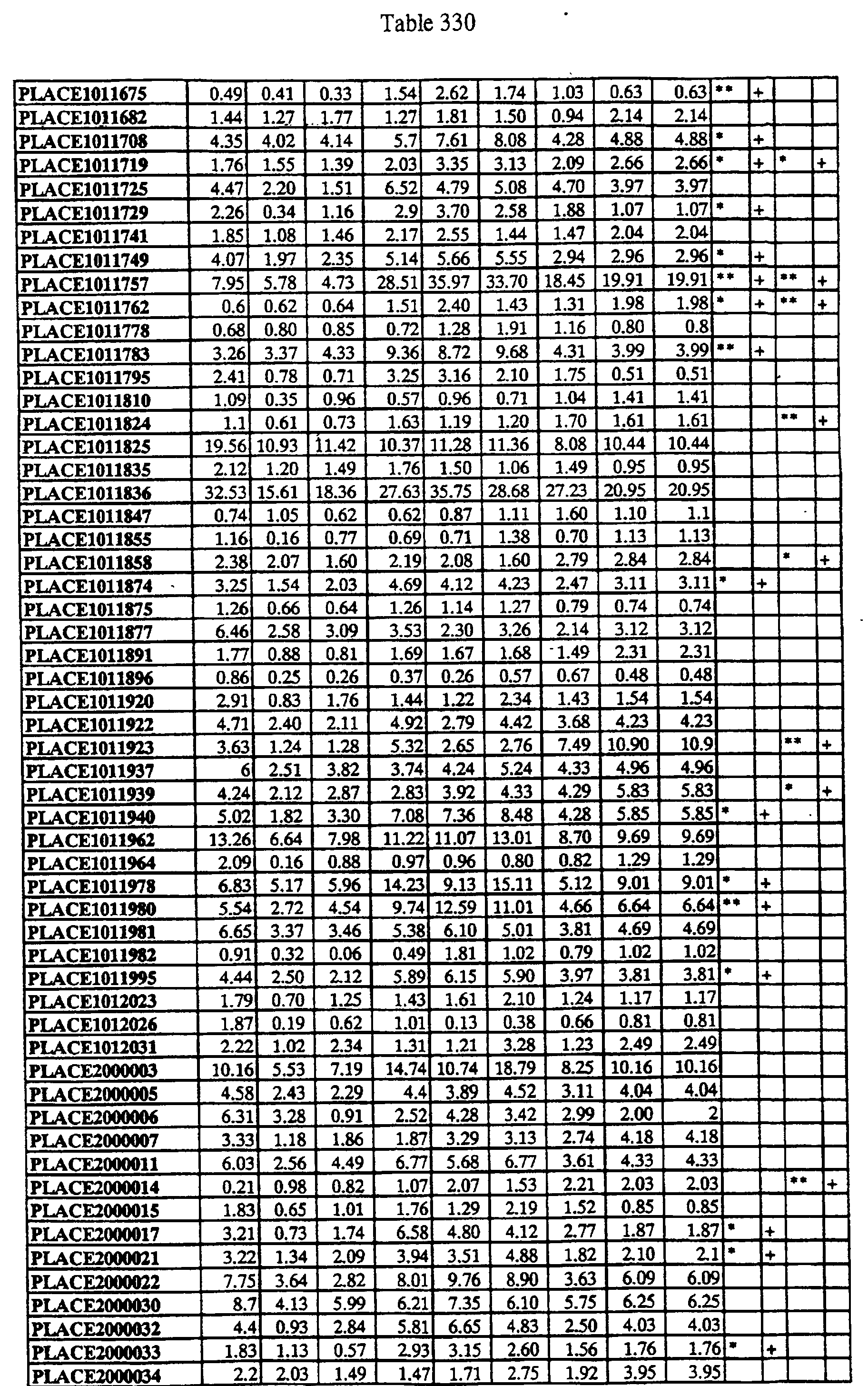

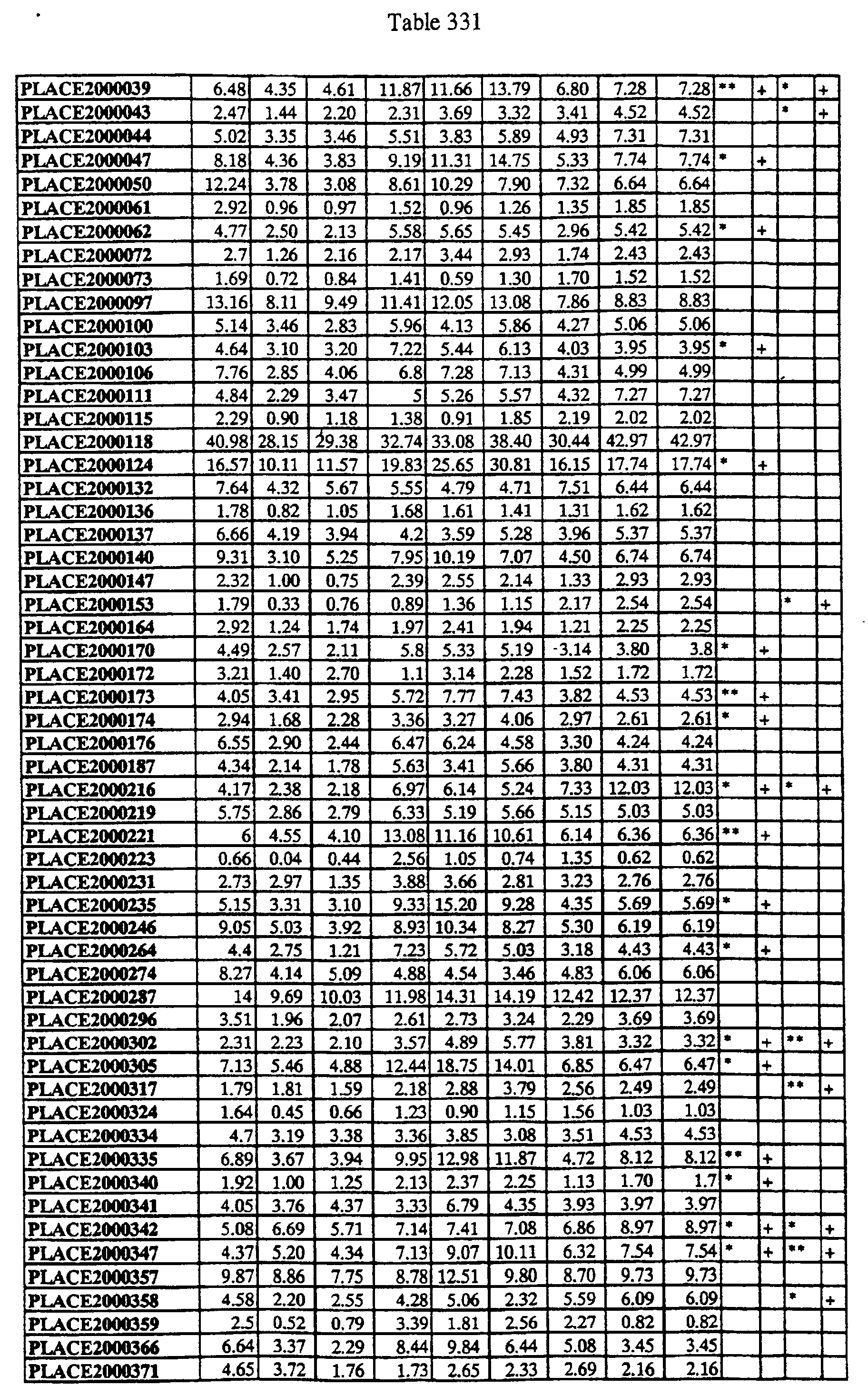

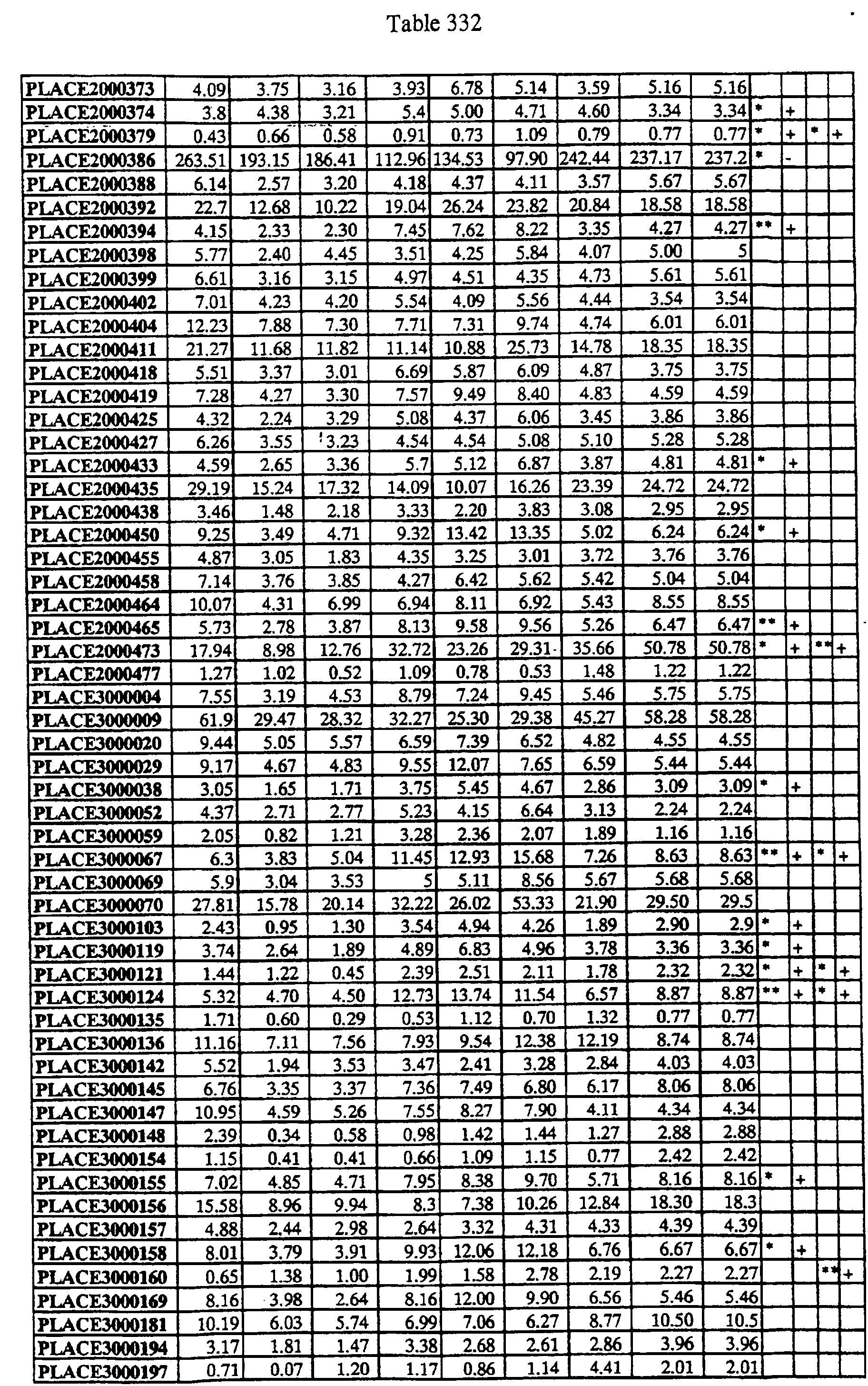

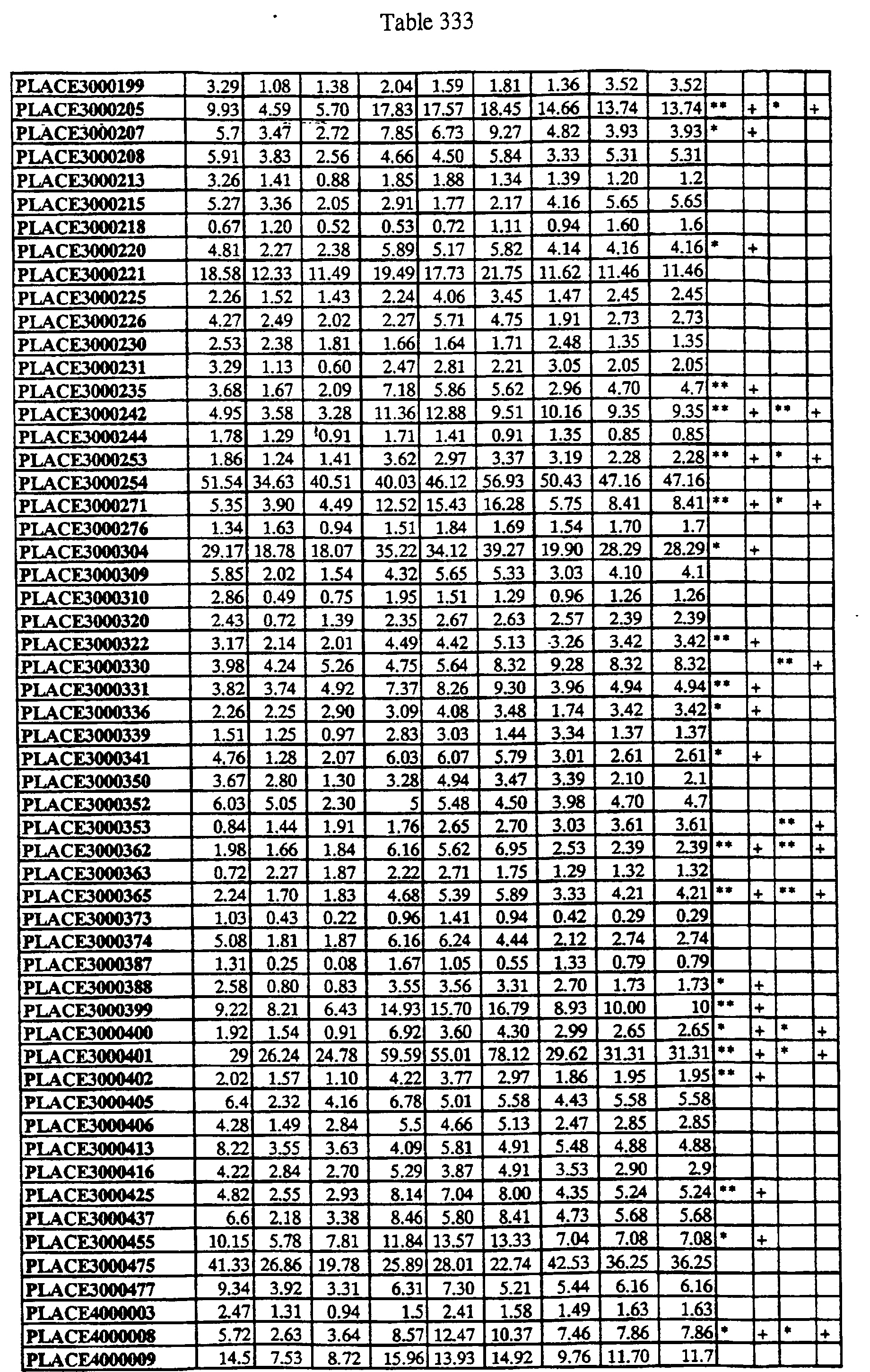

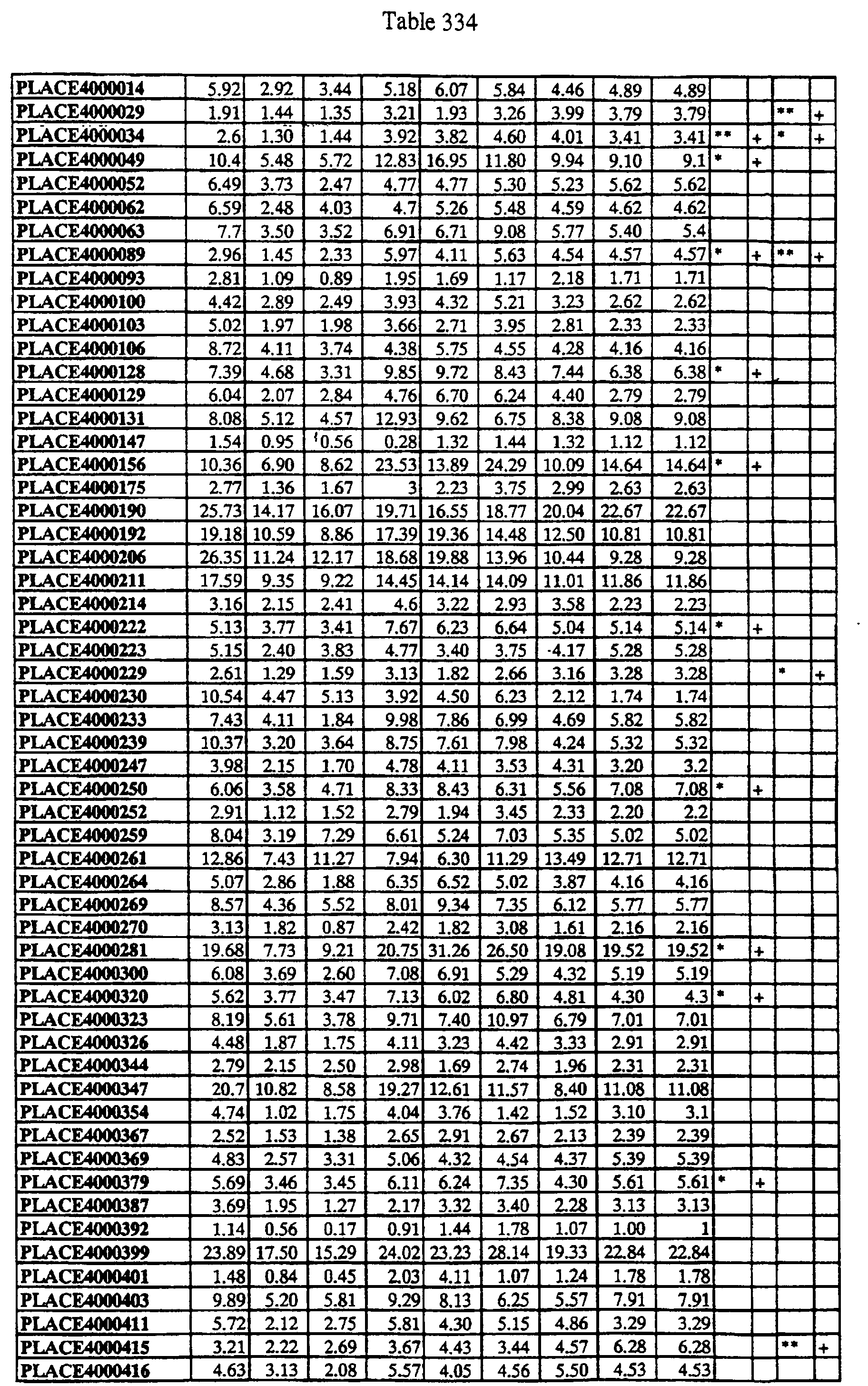

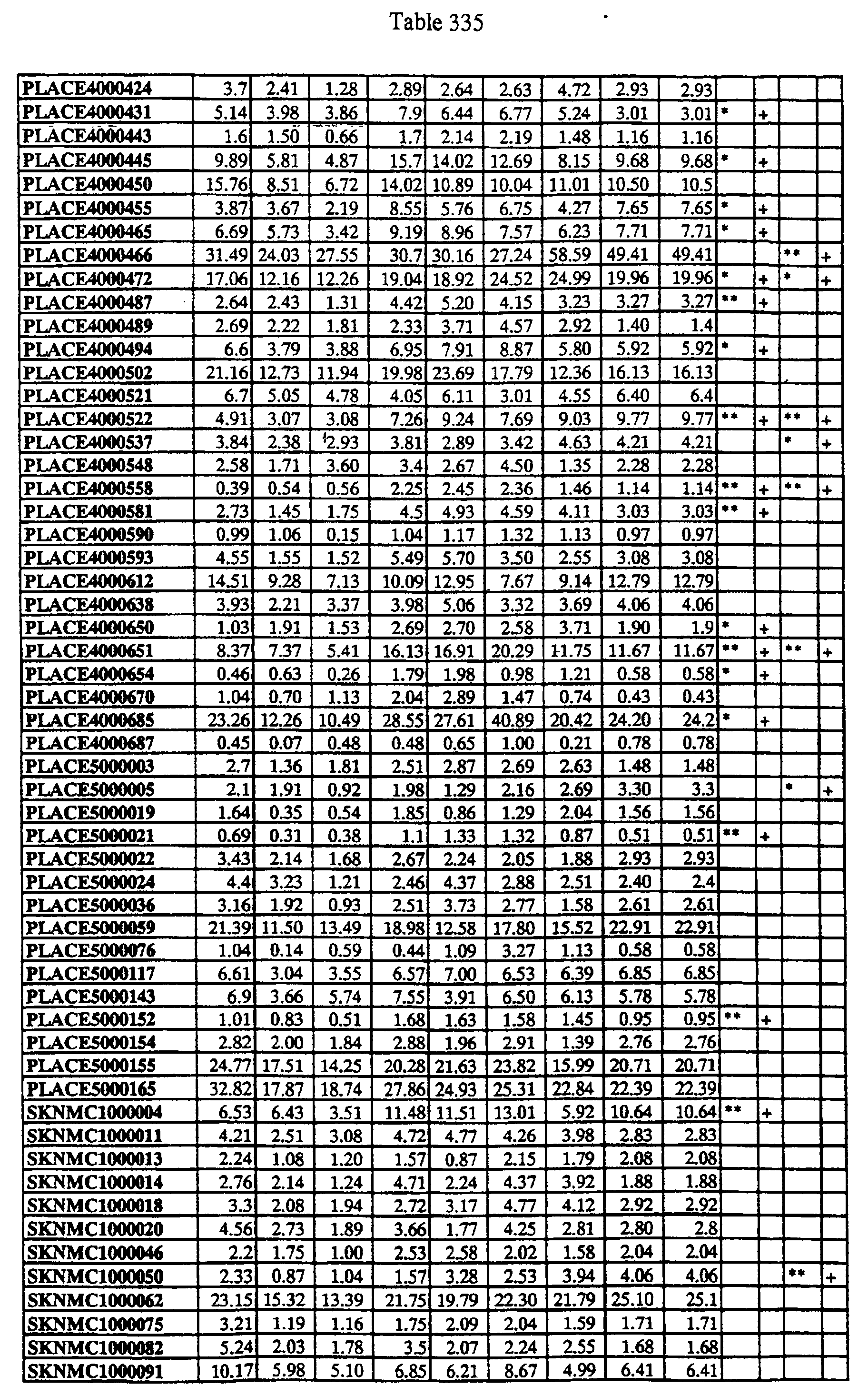

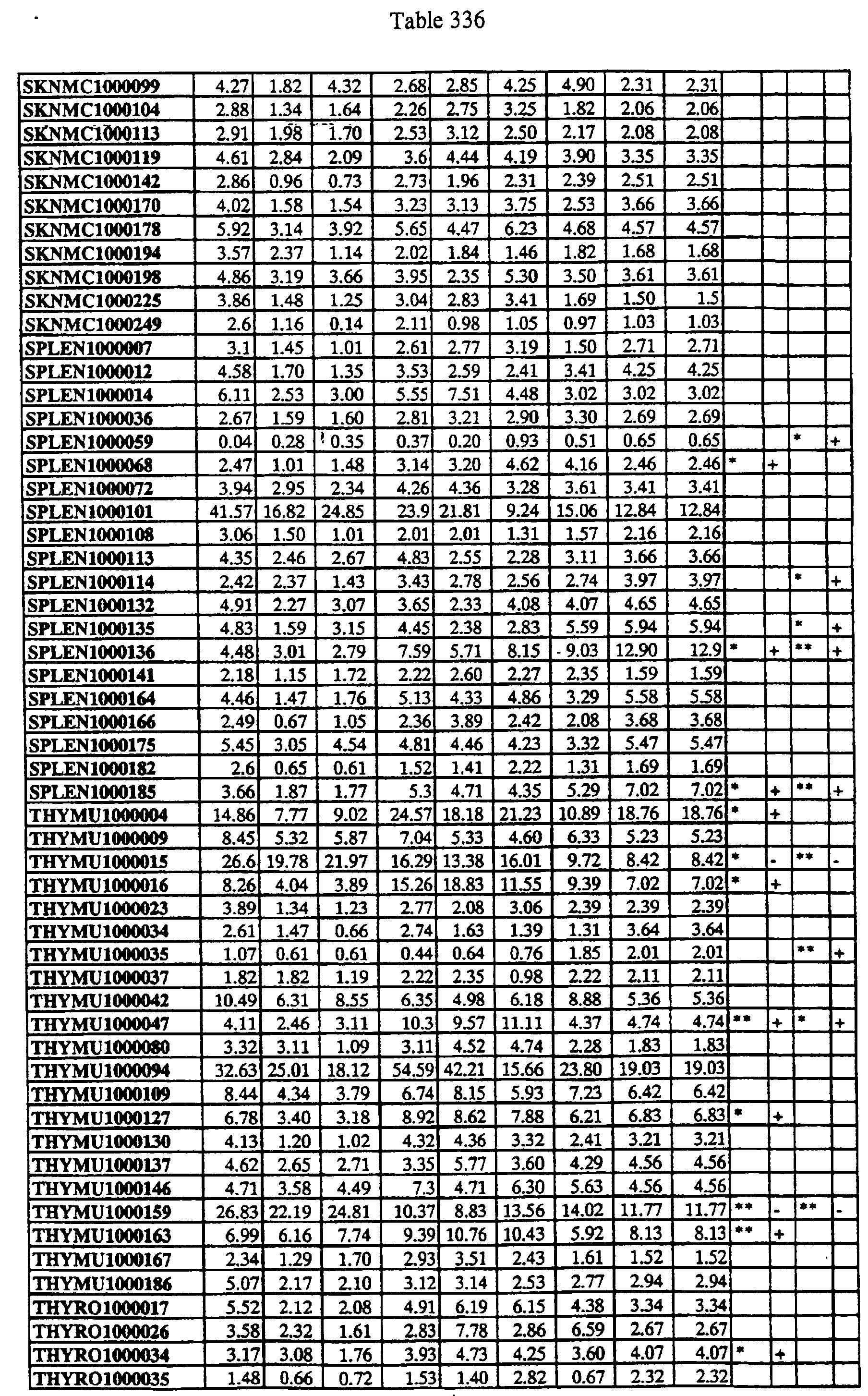

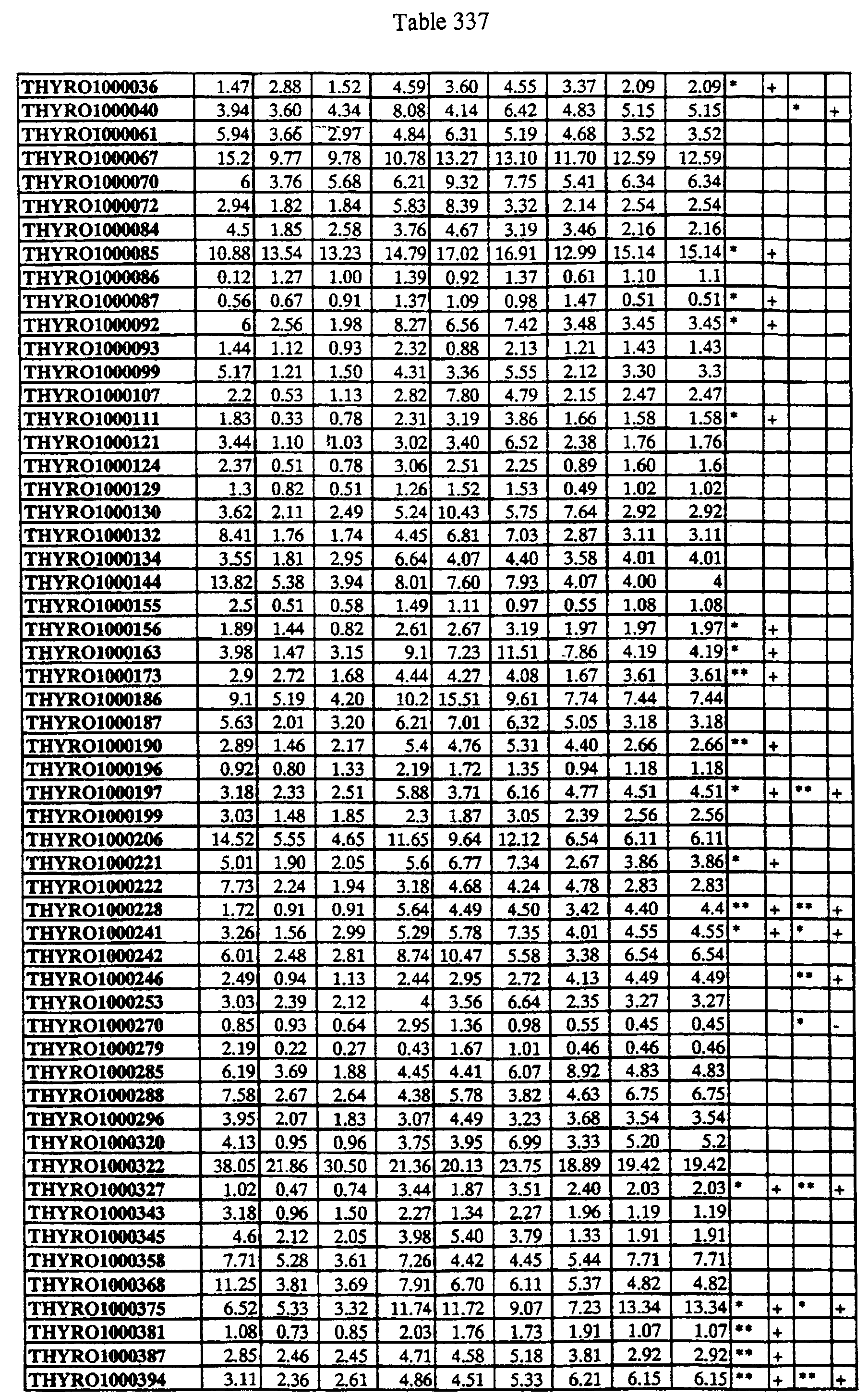

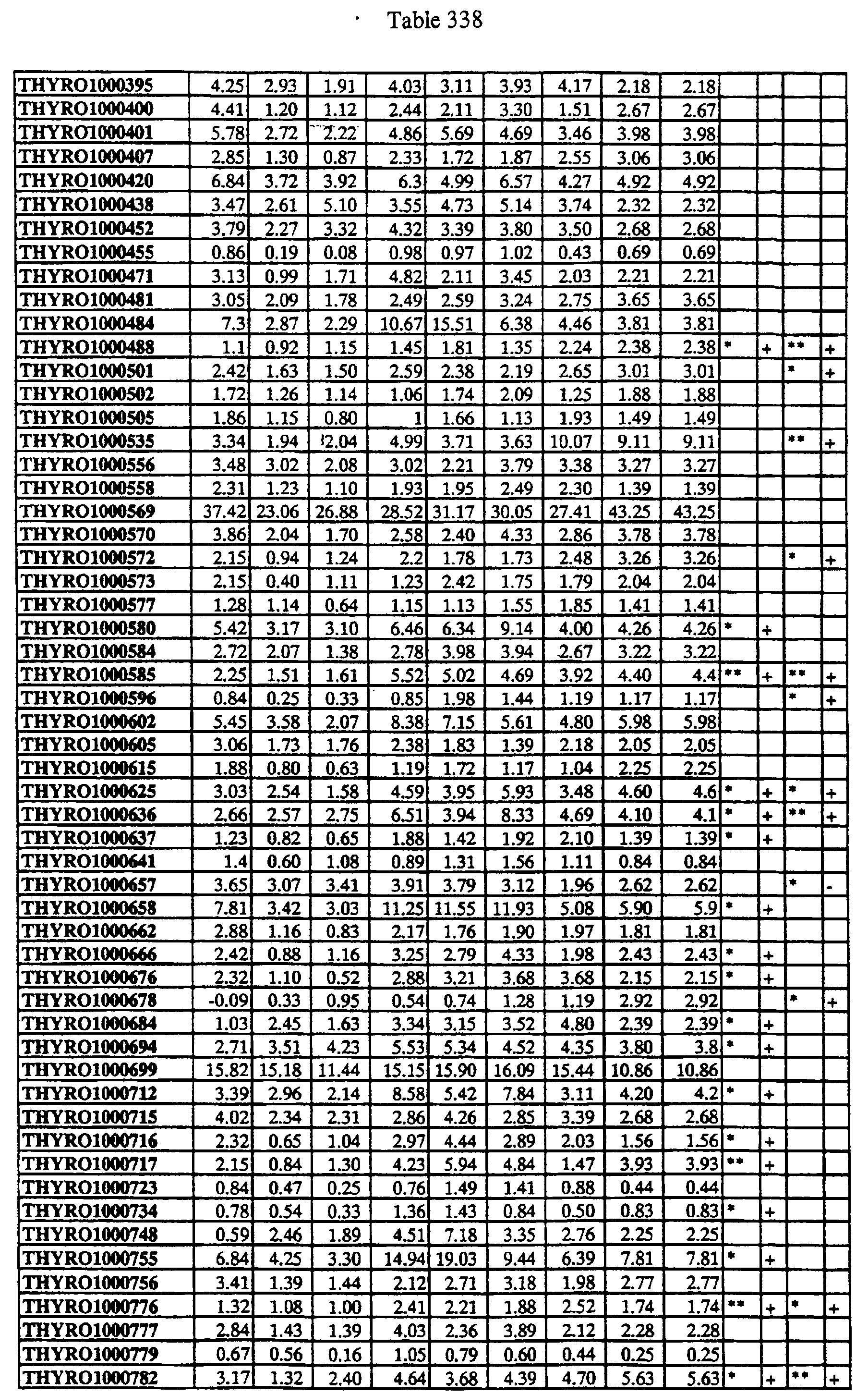

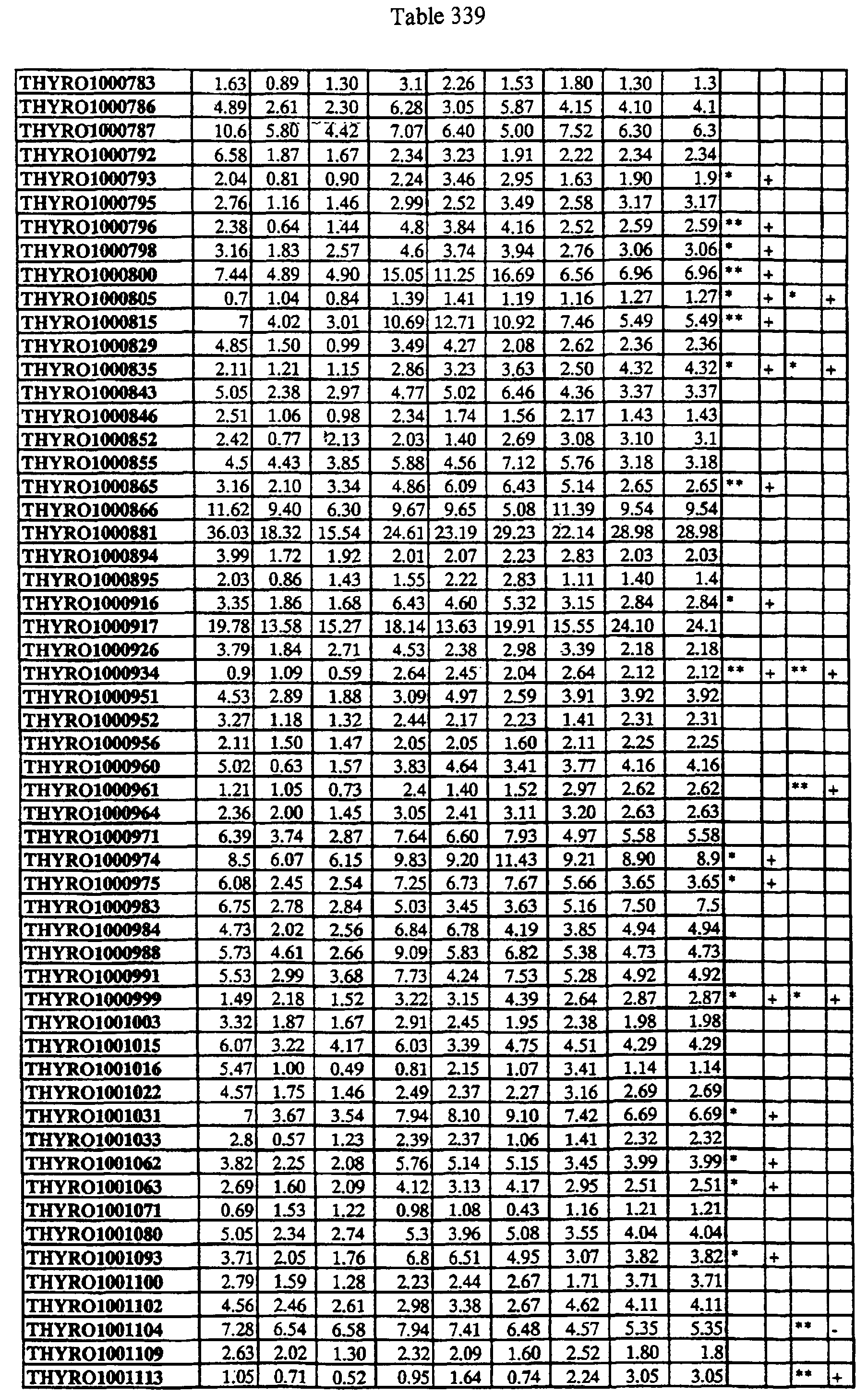

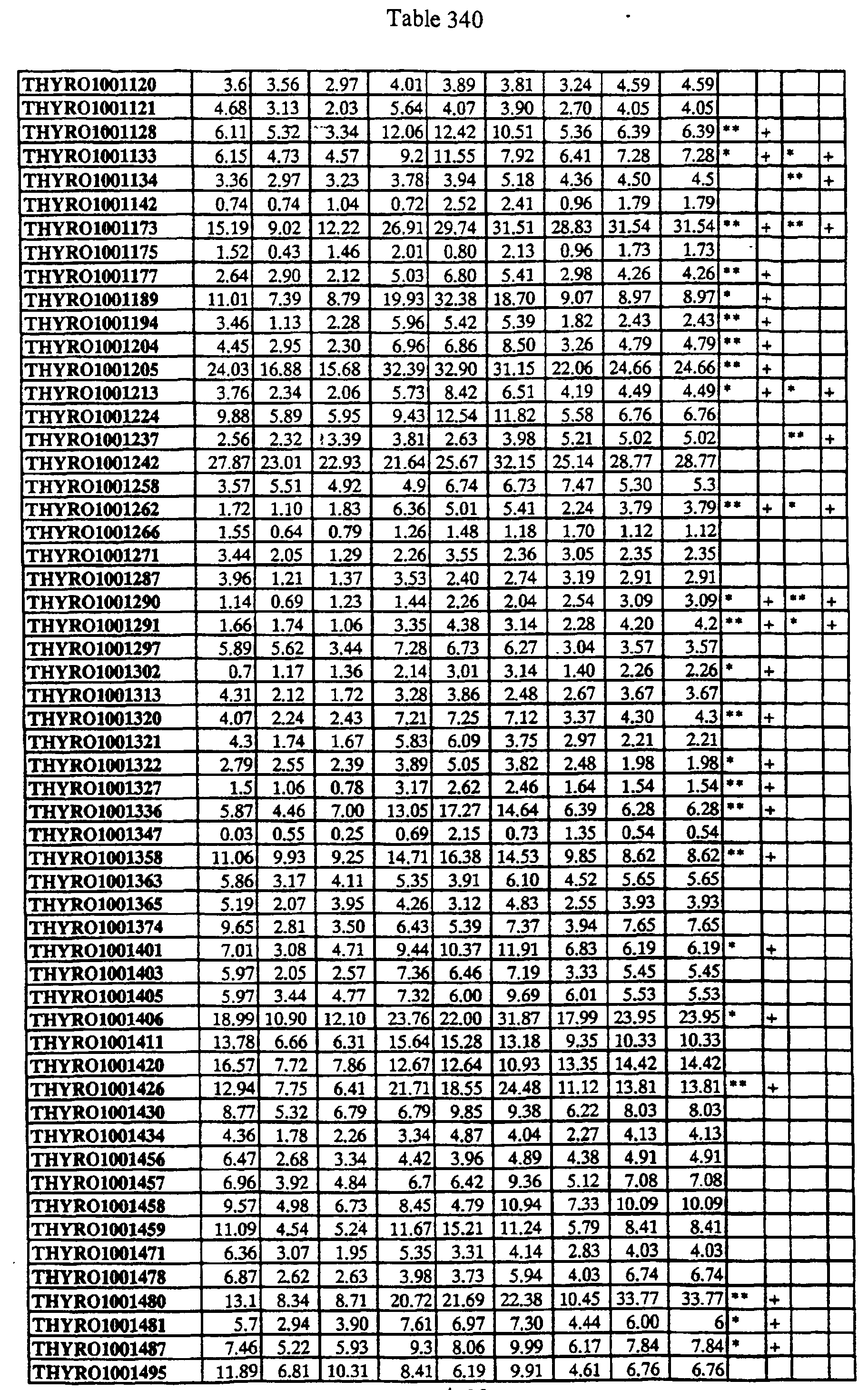

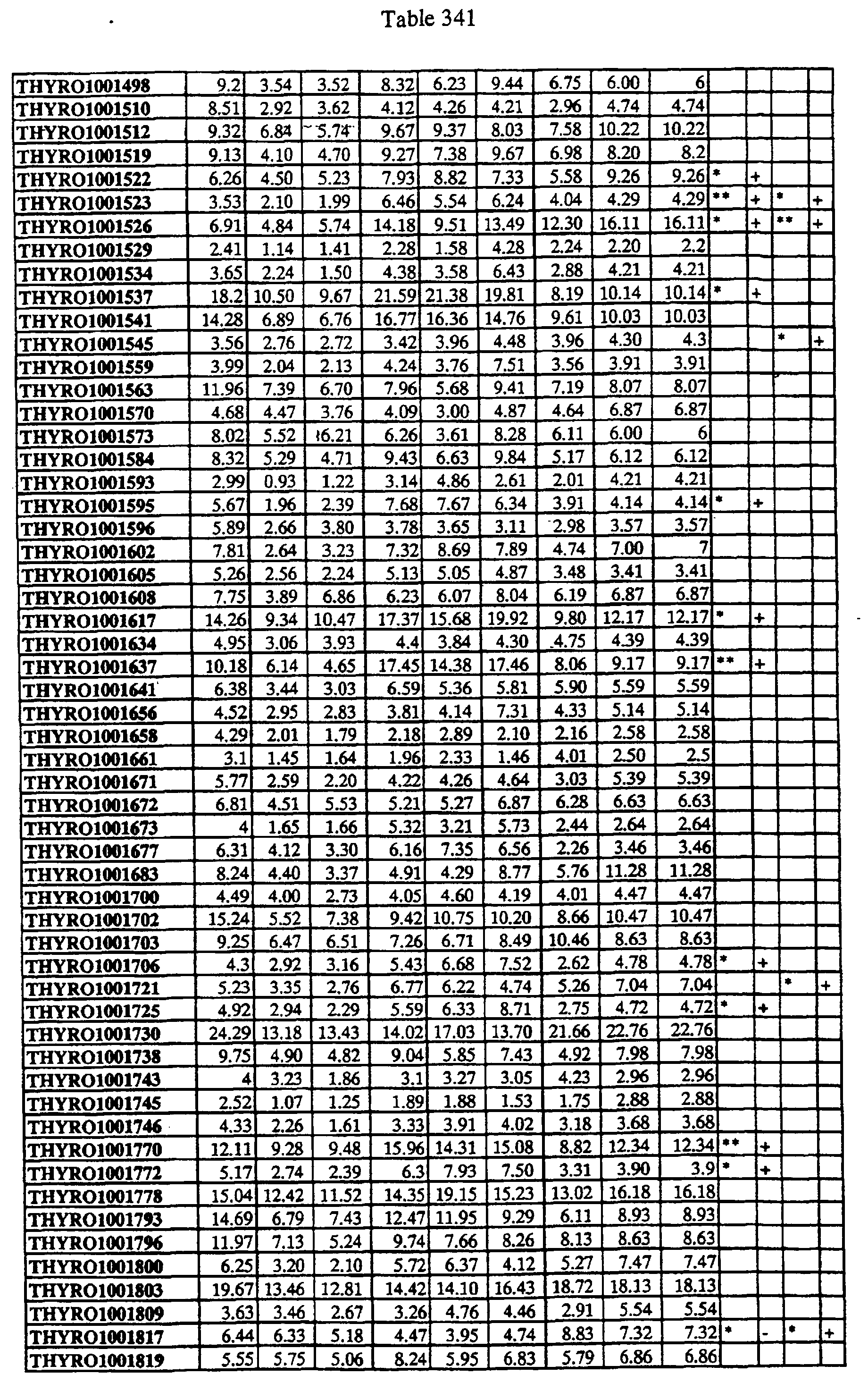

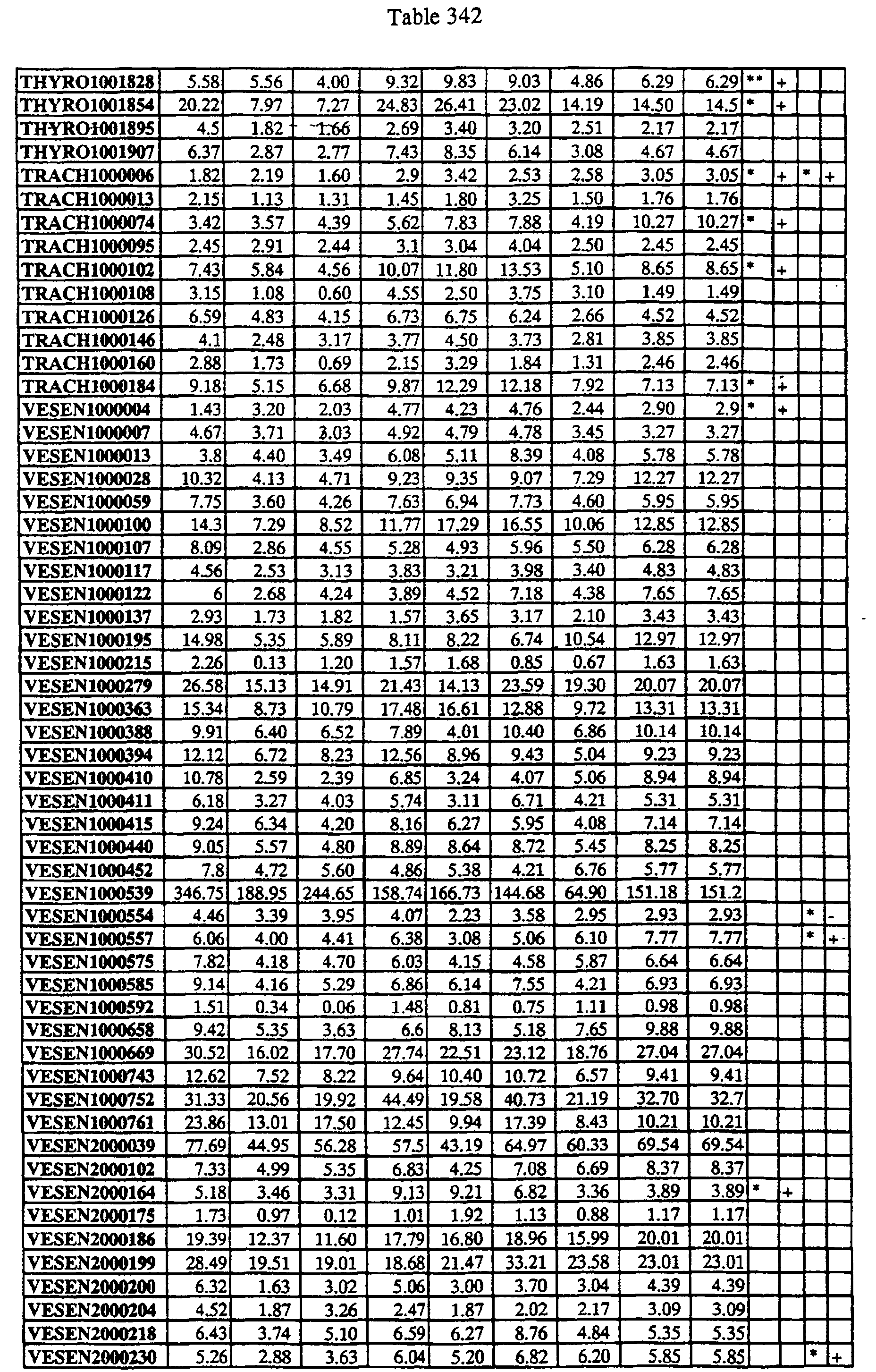

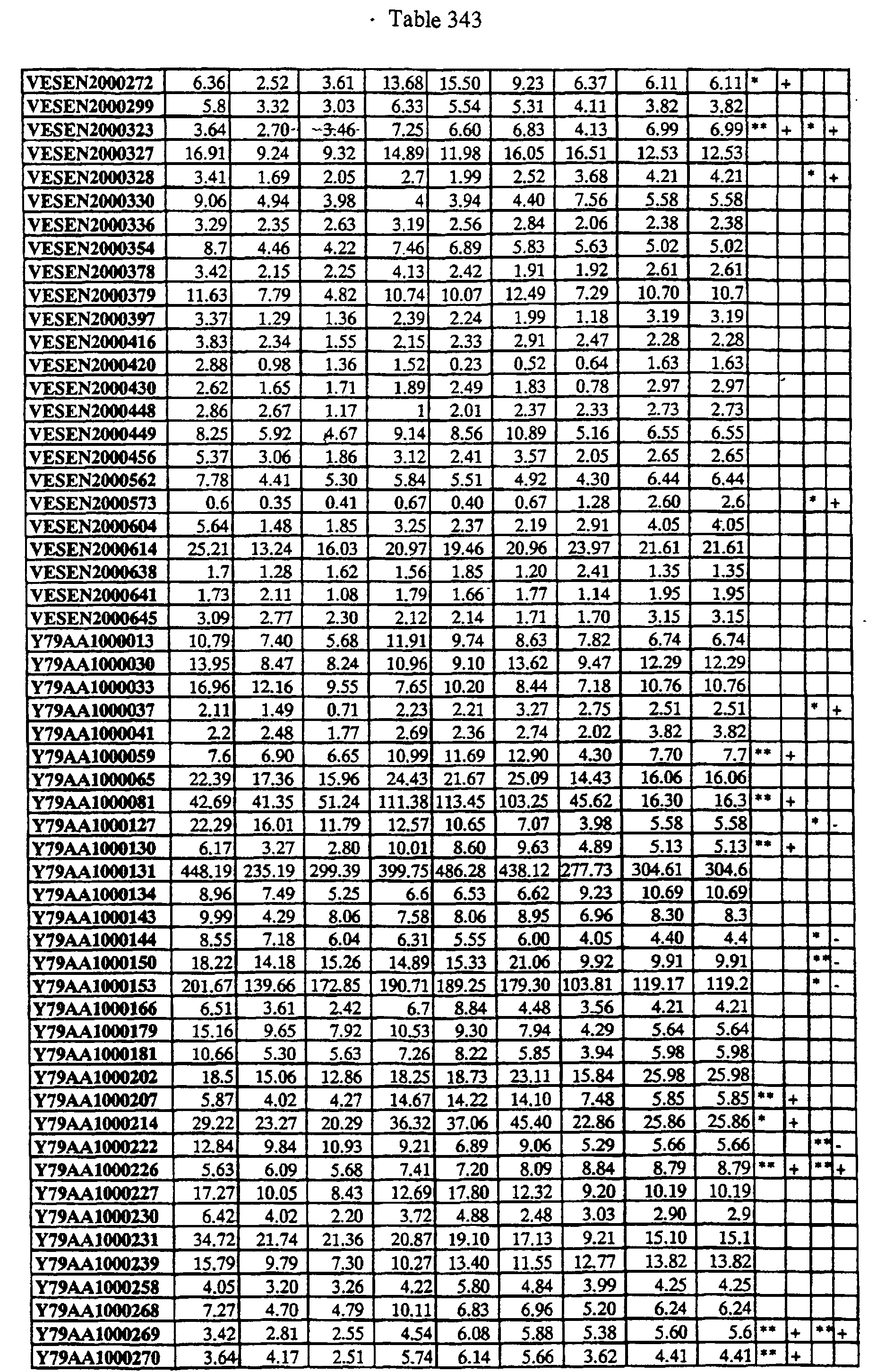

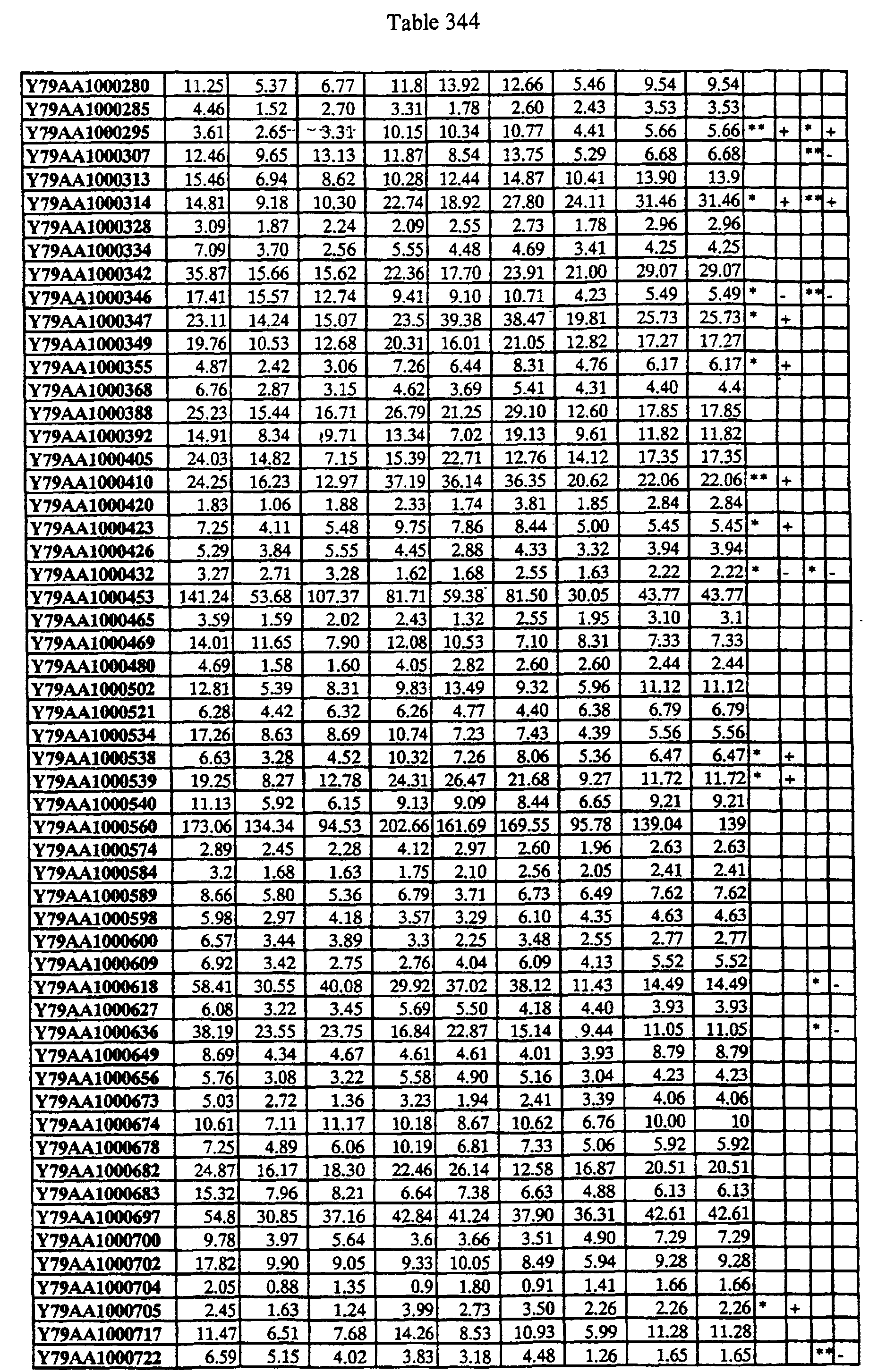

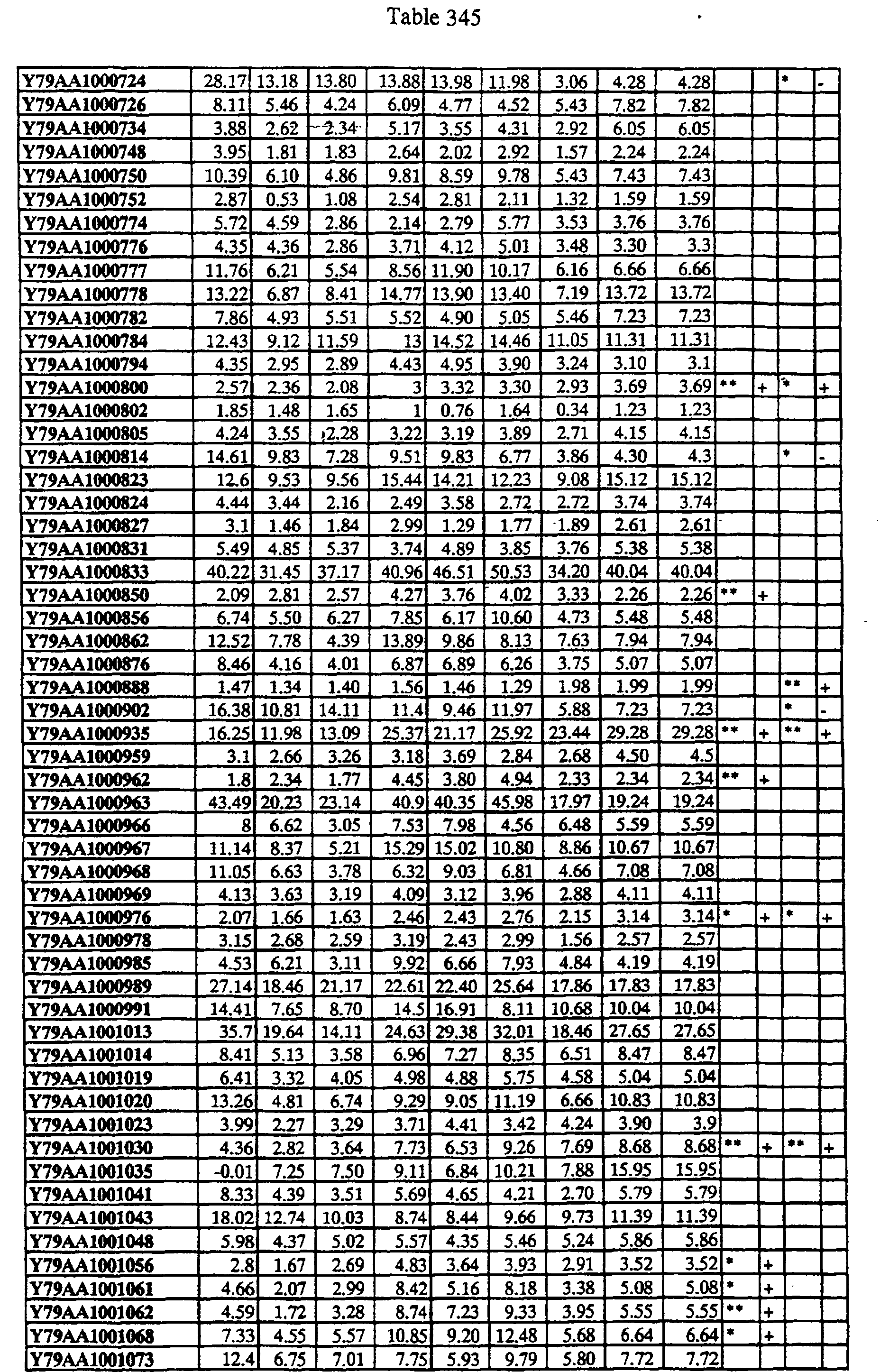

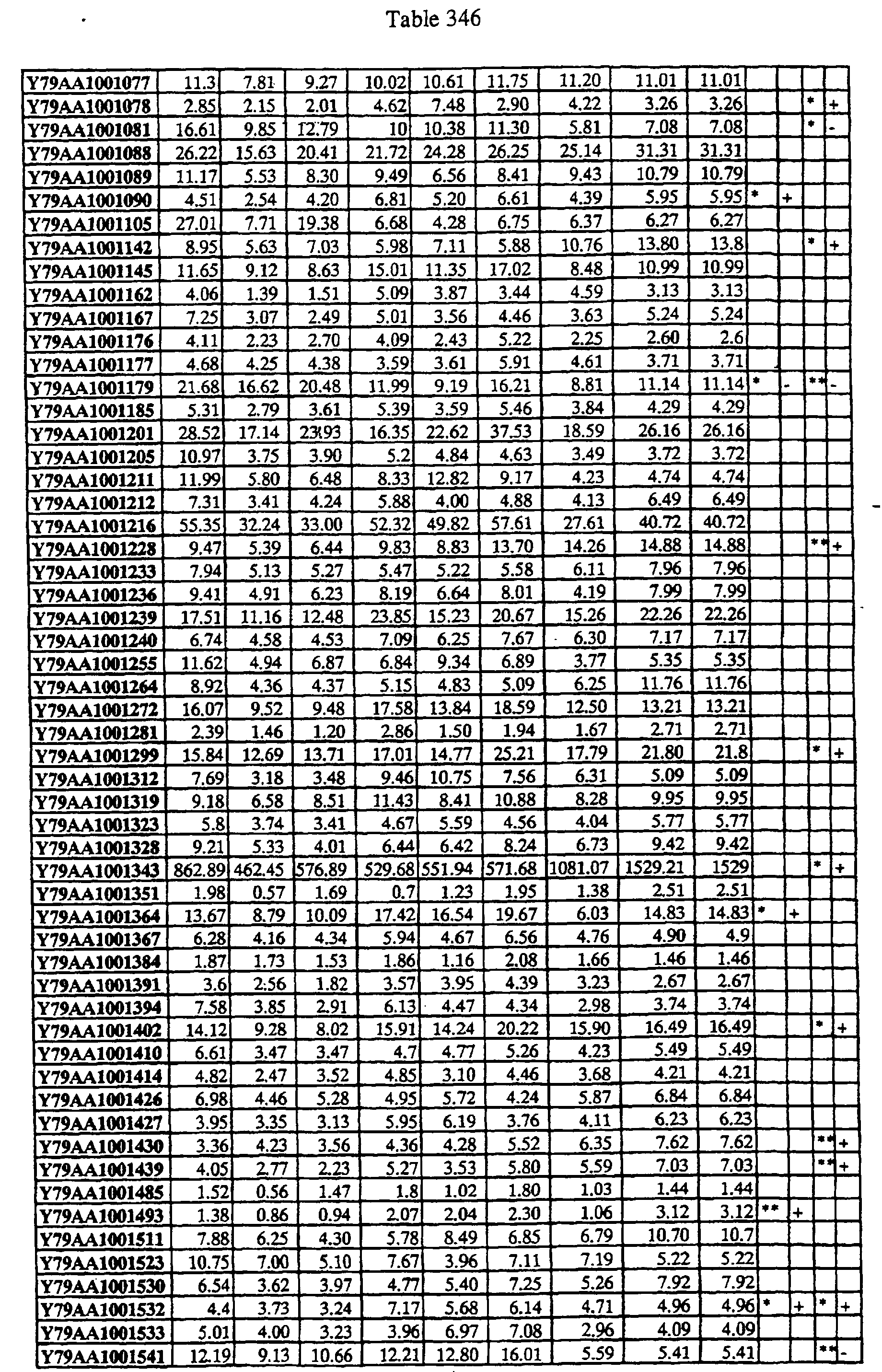

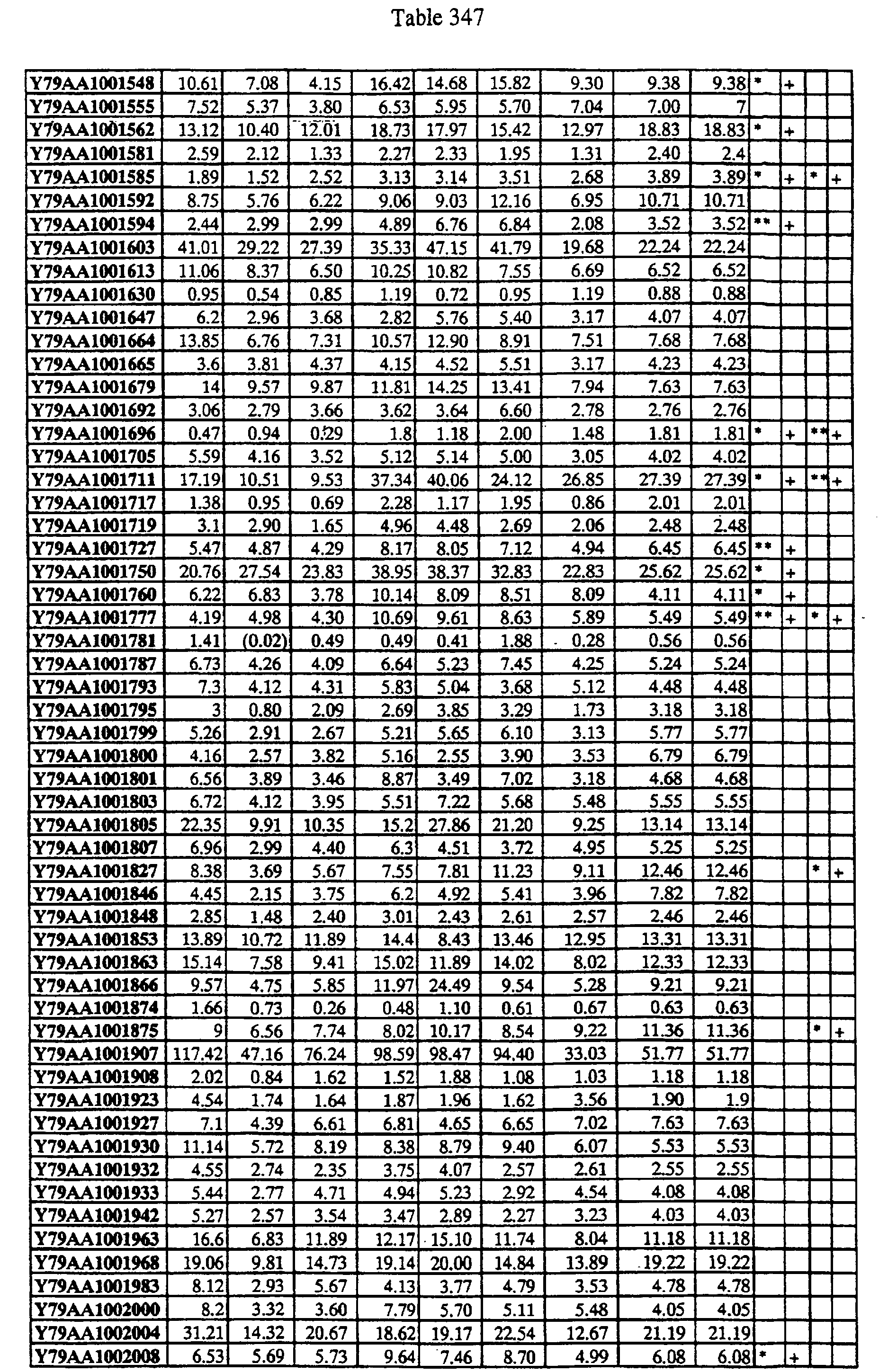

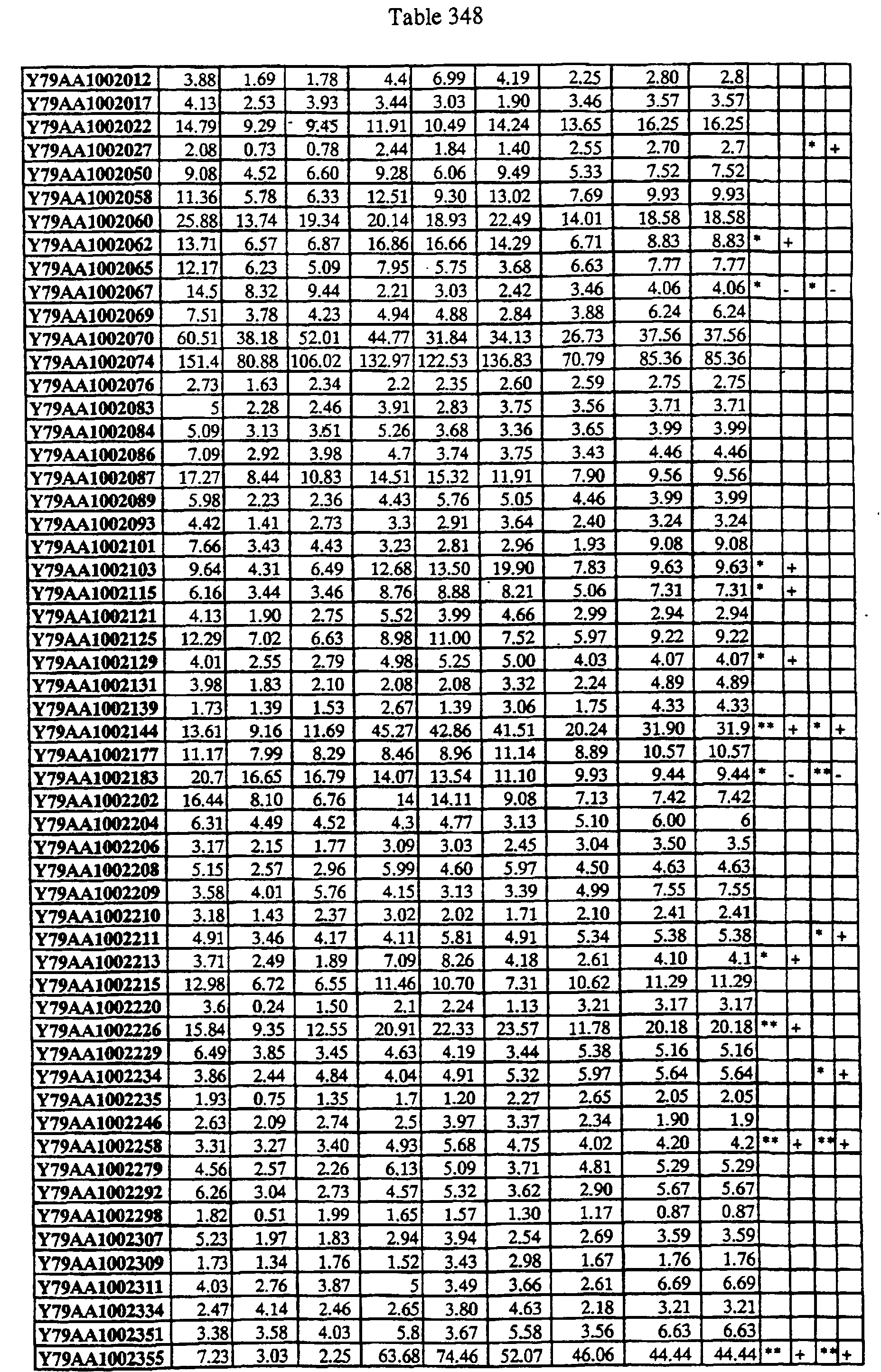

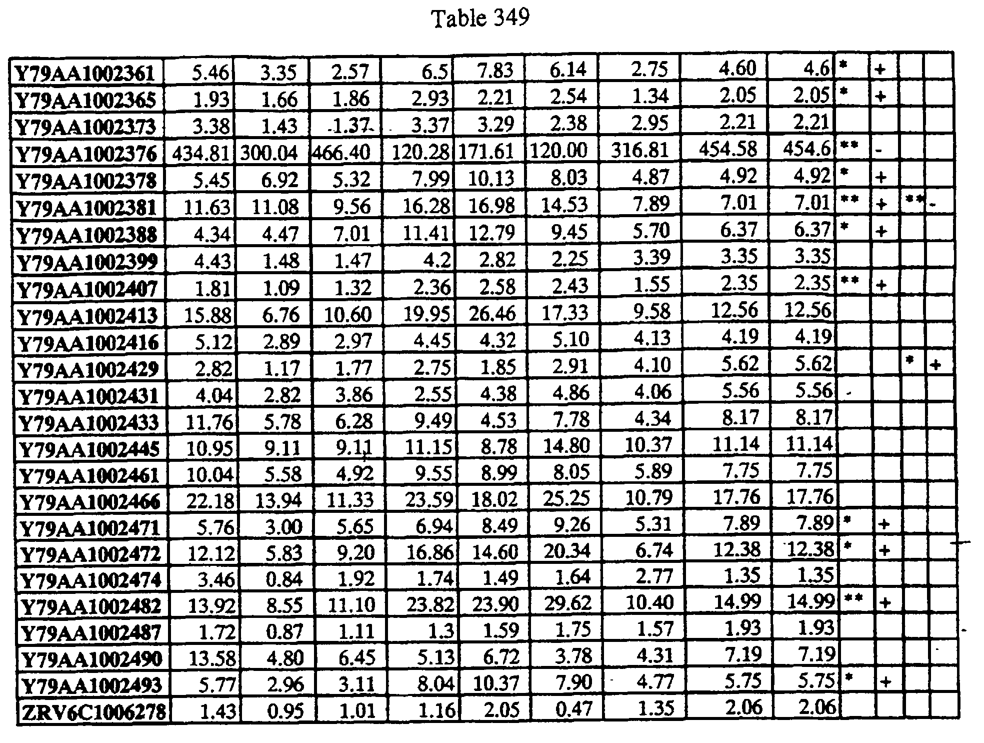

The correspondence of the full-length nucleotide sequences of the present invention and the corresponding deduced amino acid sequences with the clone names are shown below.

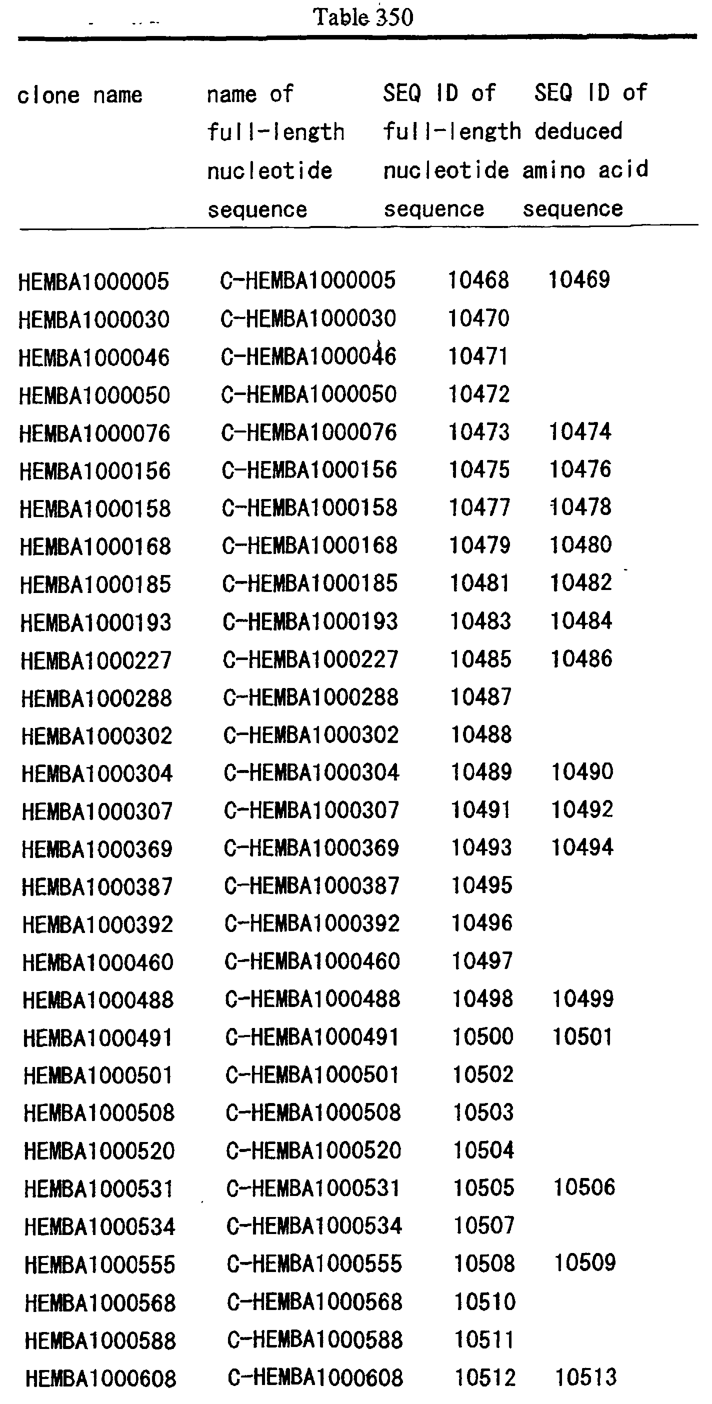

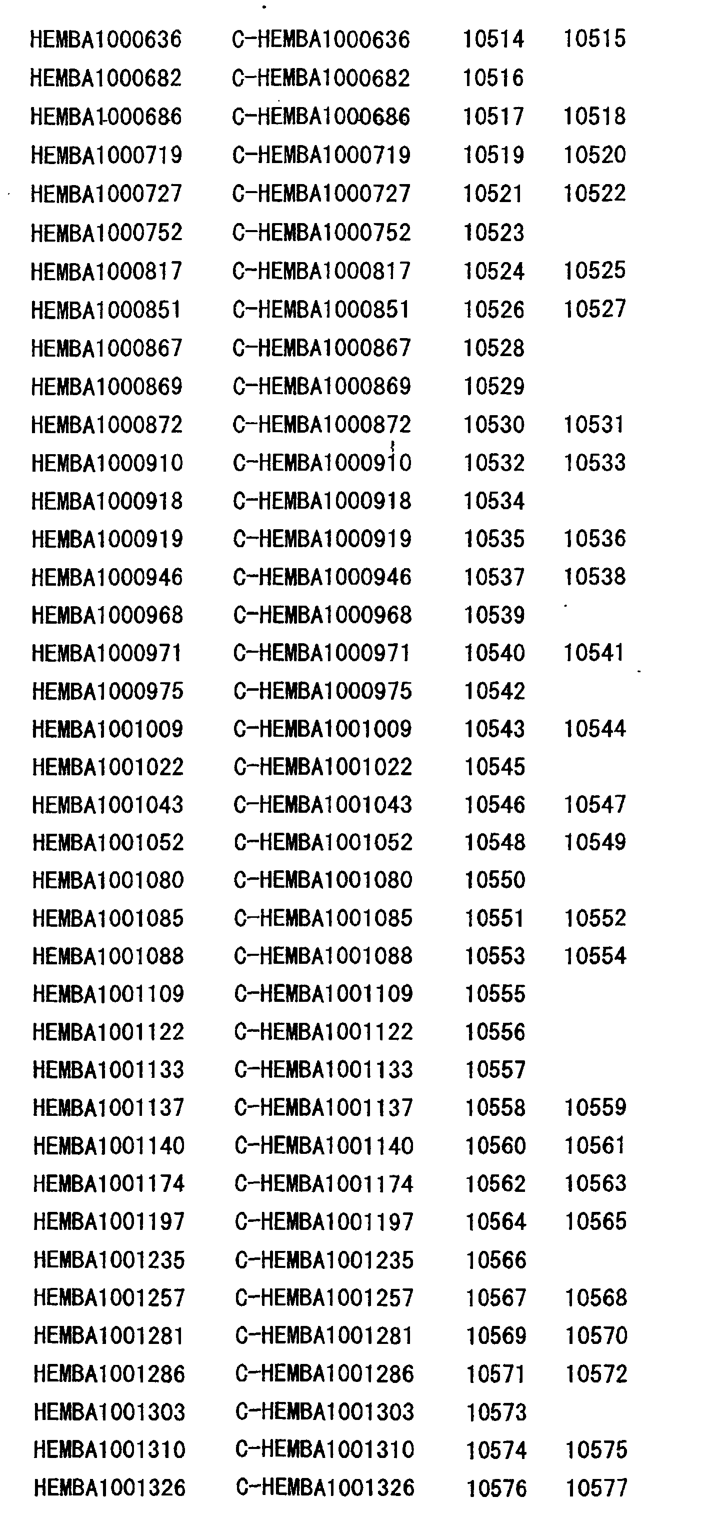

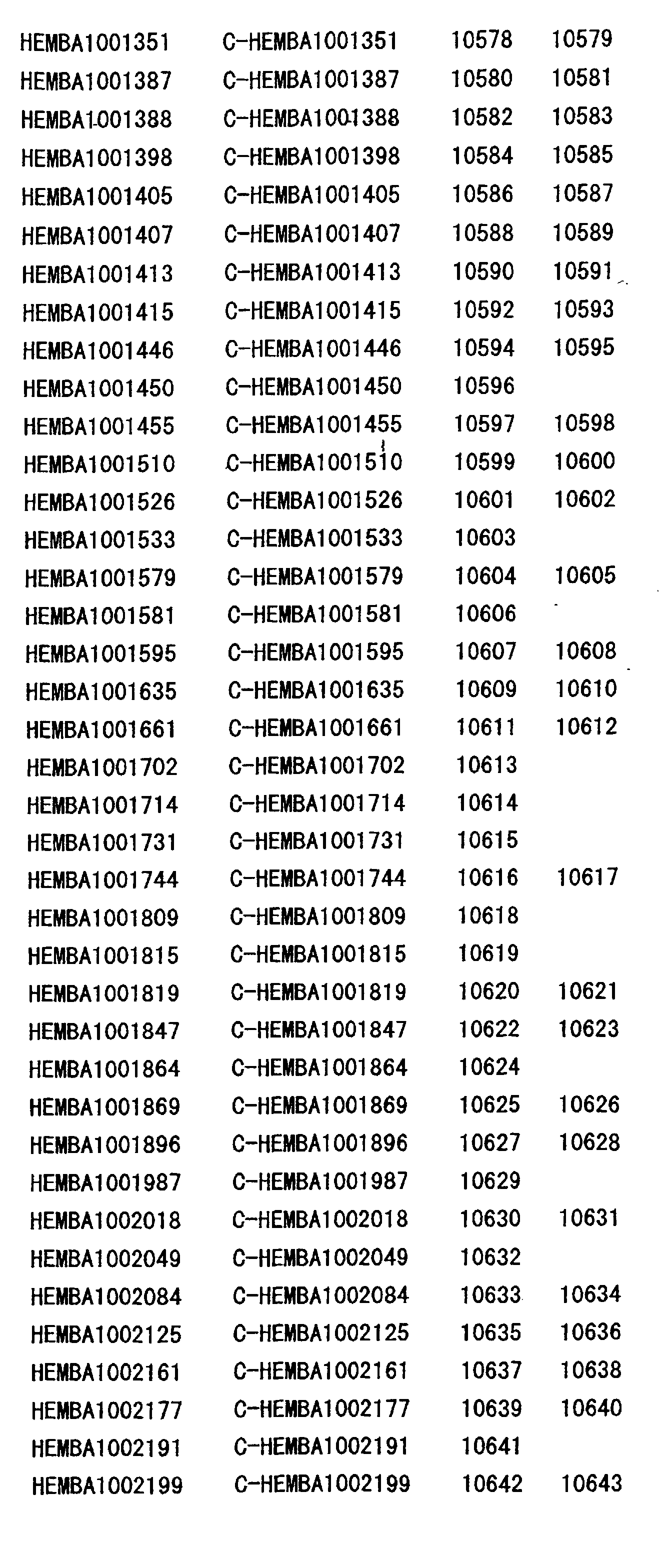

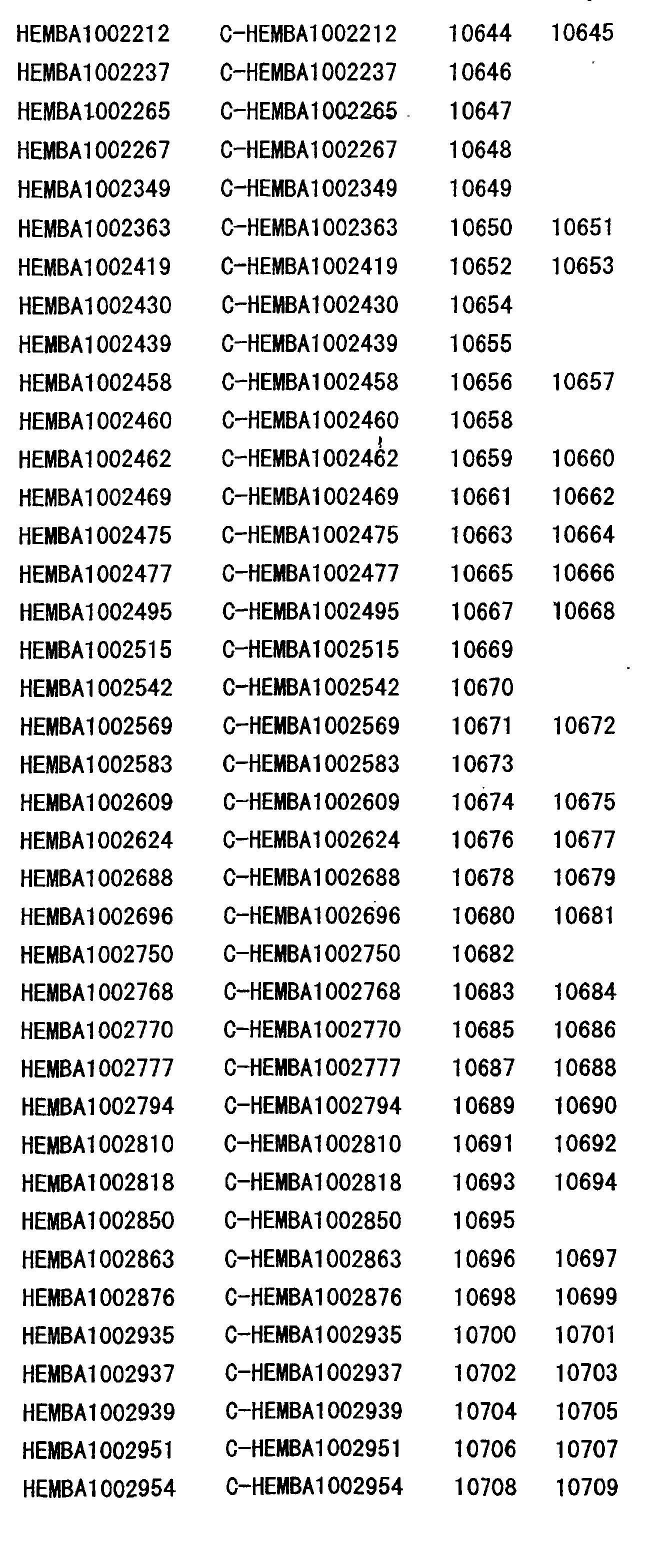

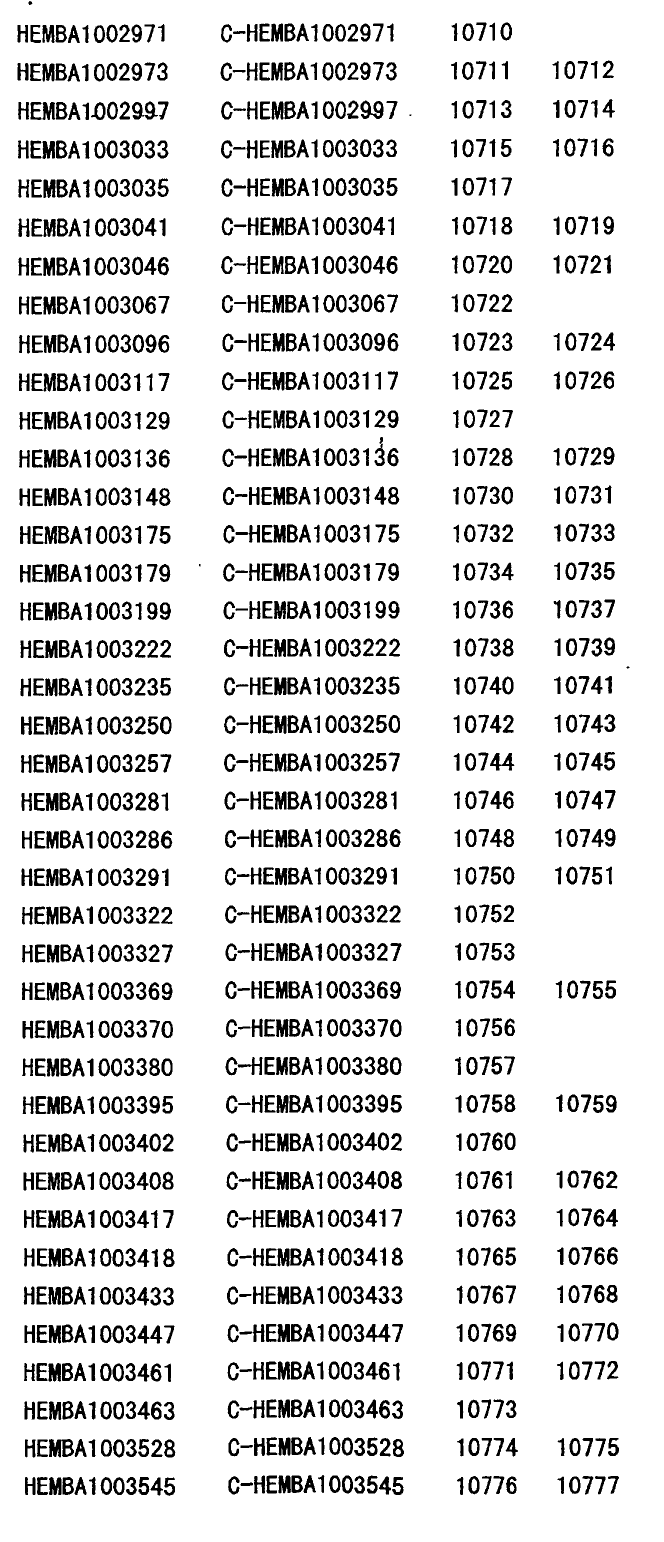

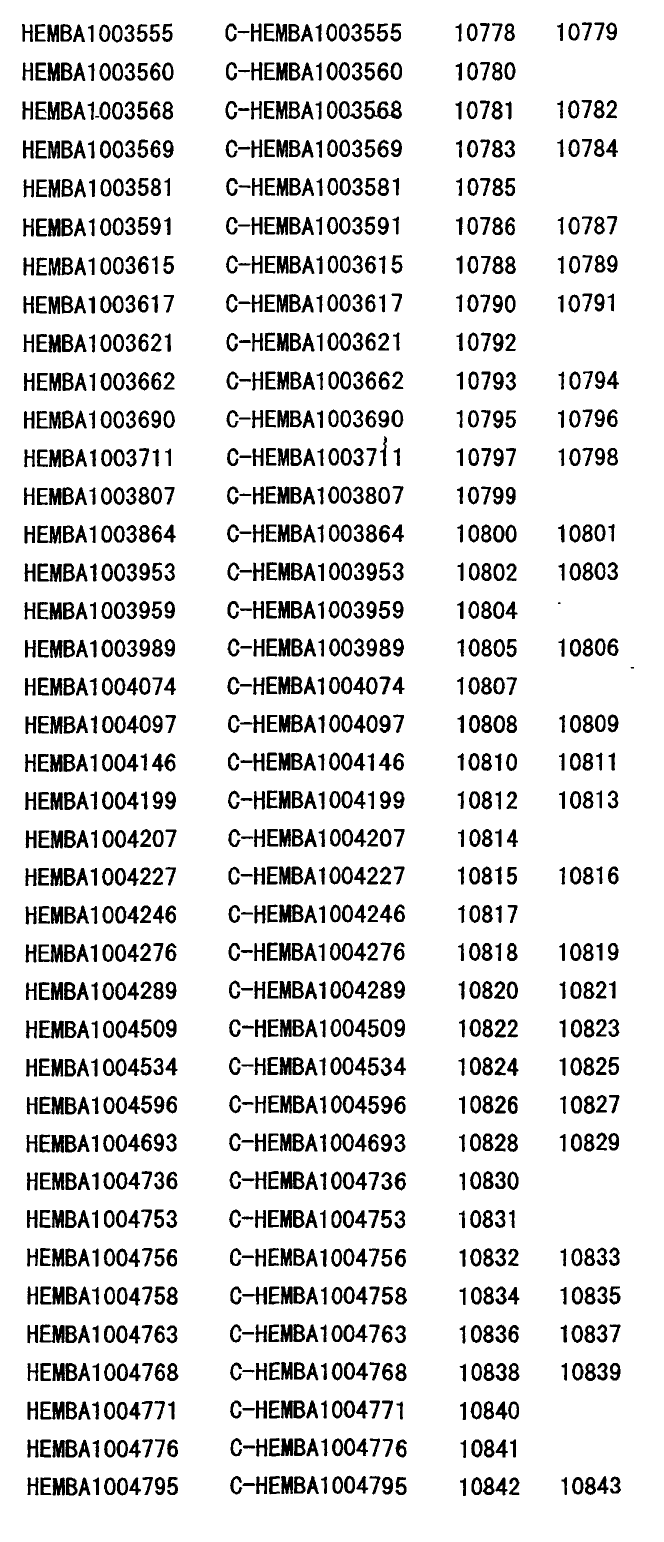

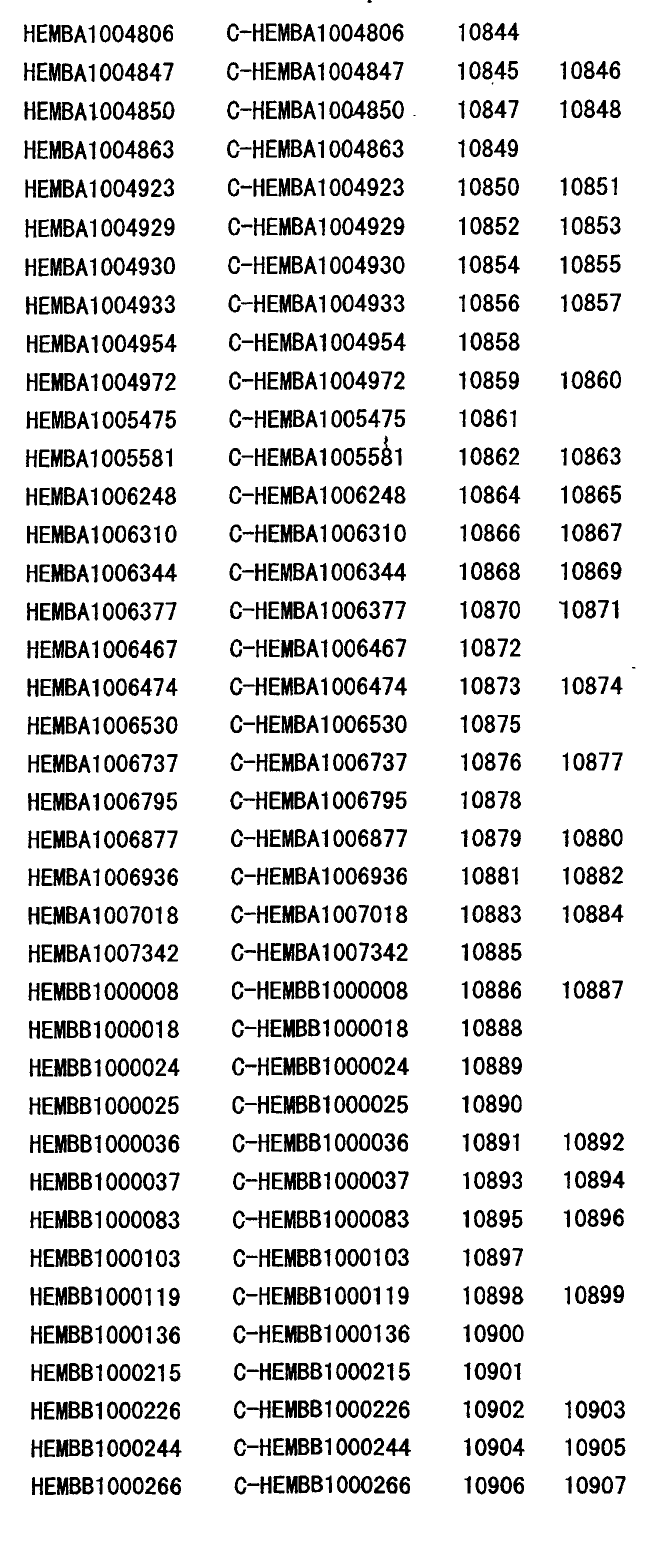

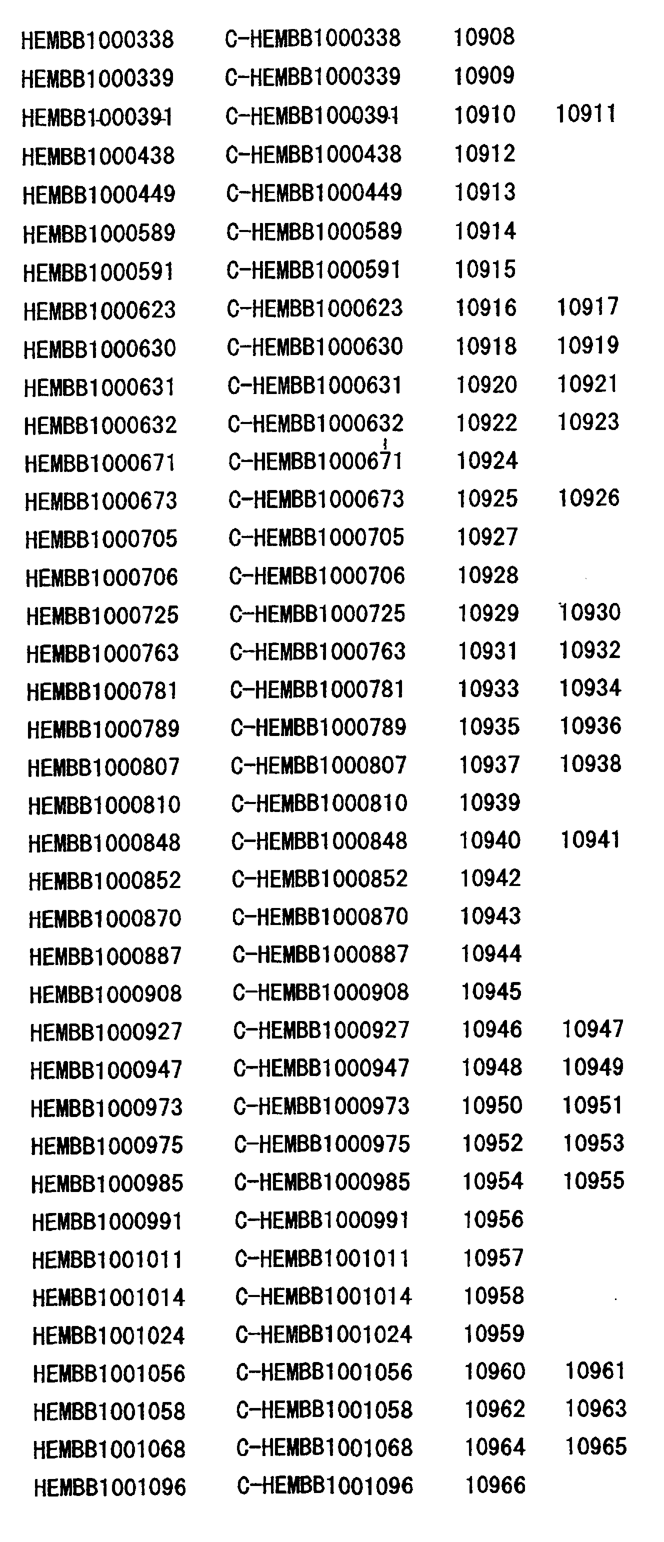

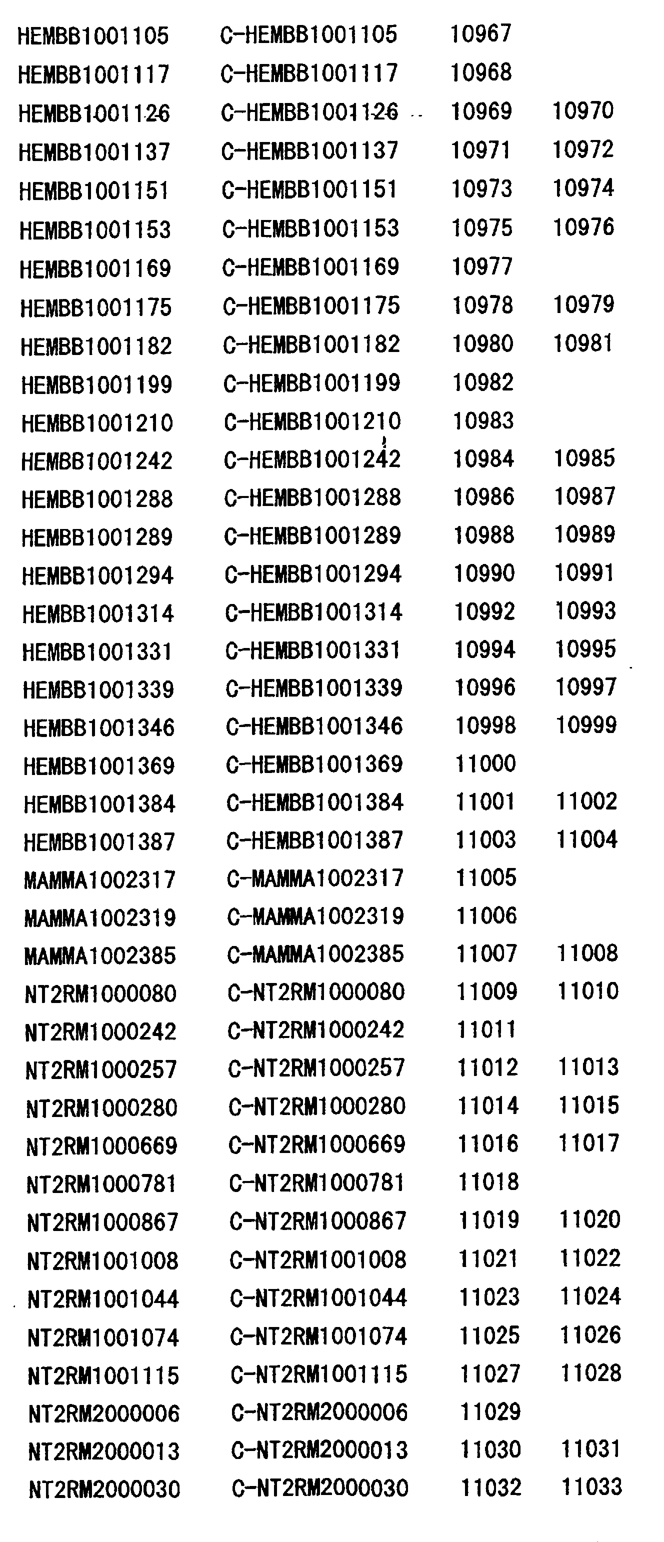

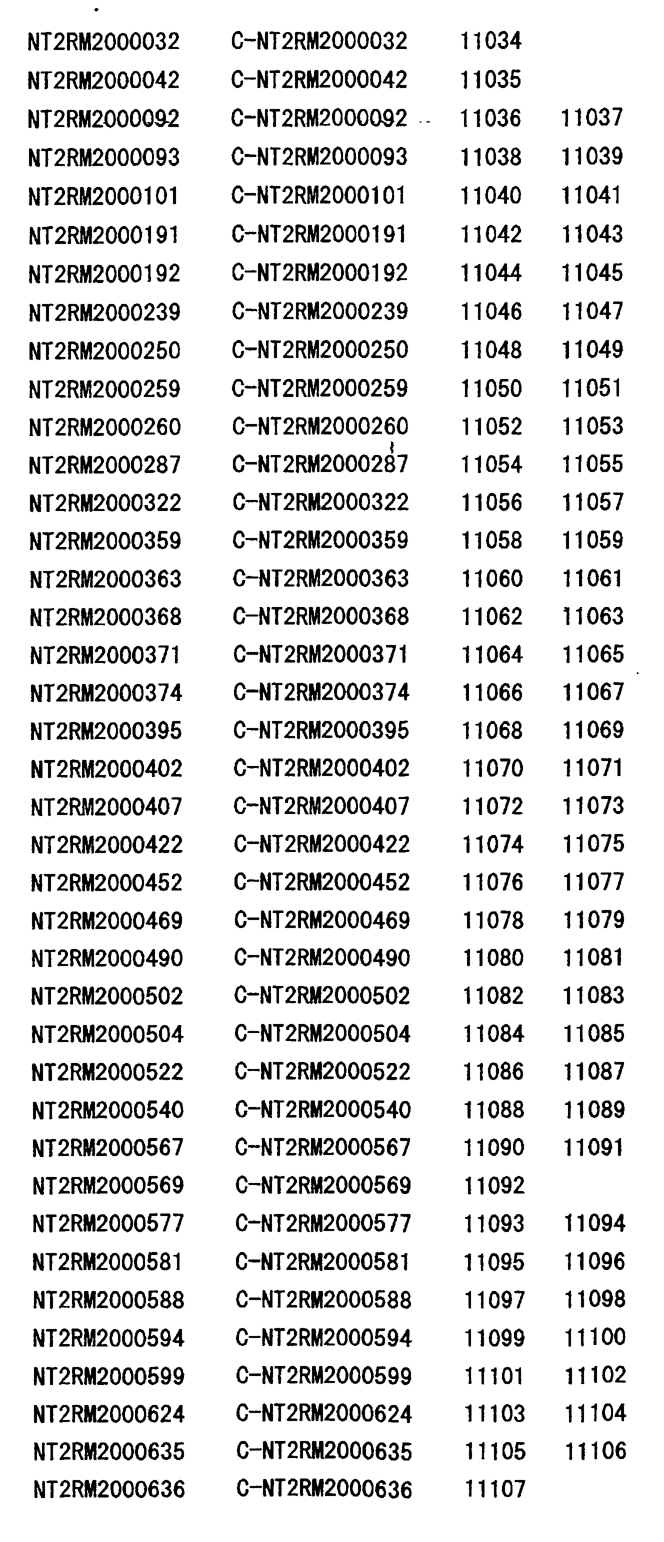

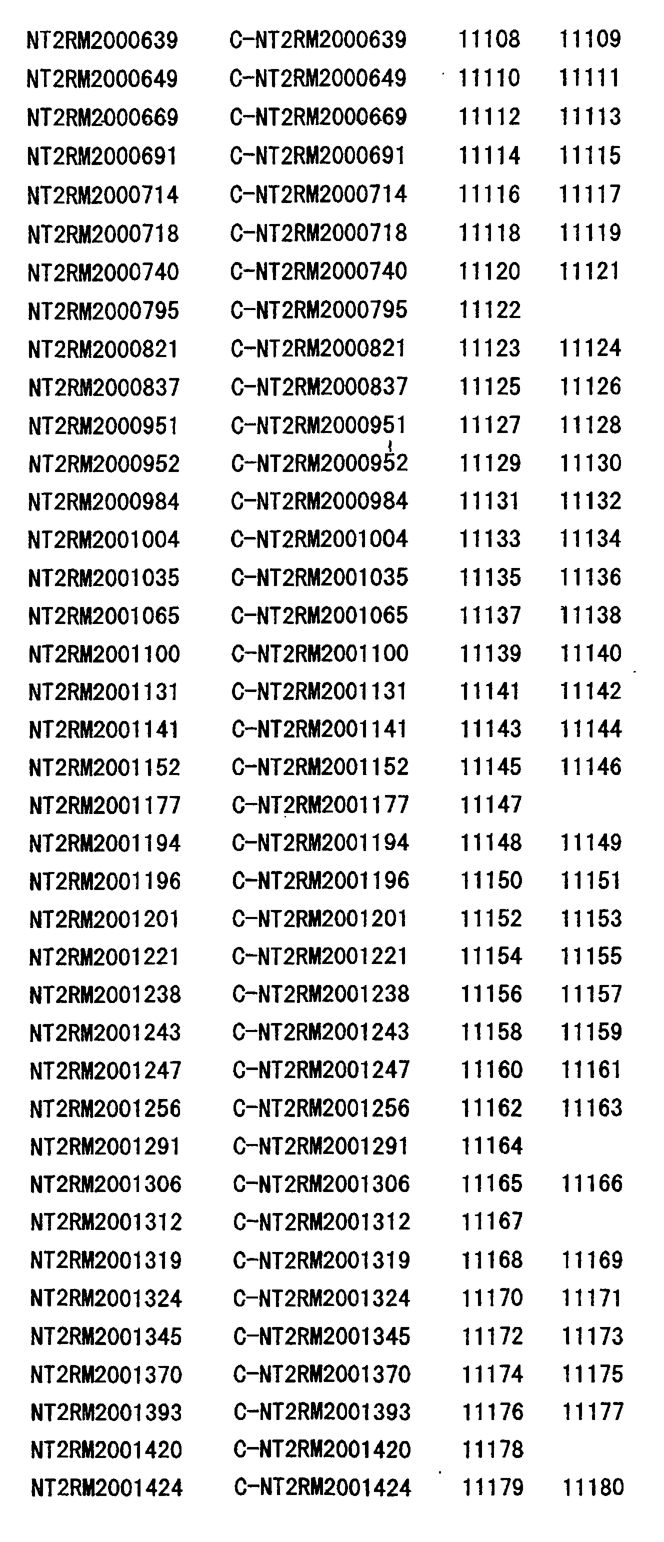

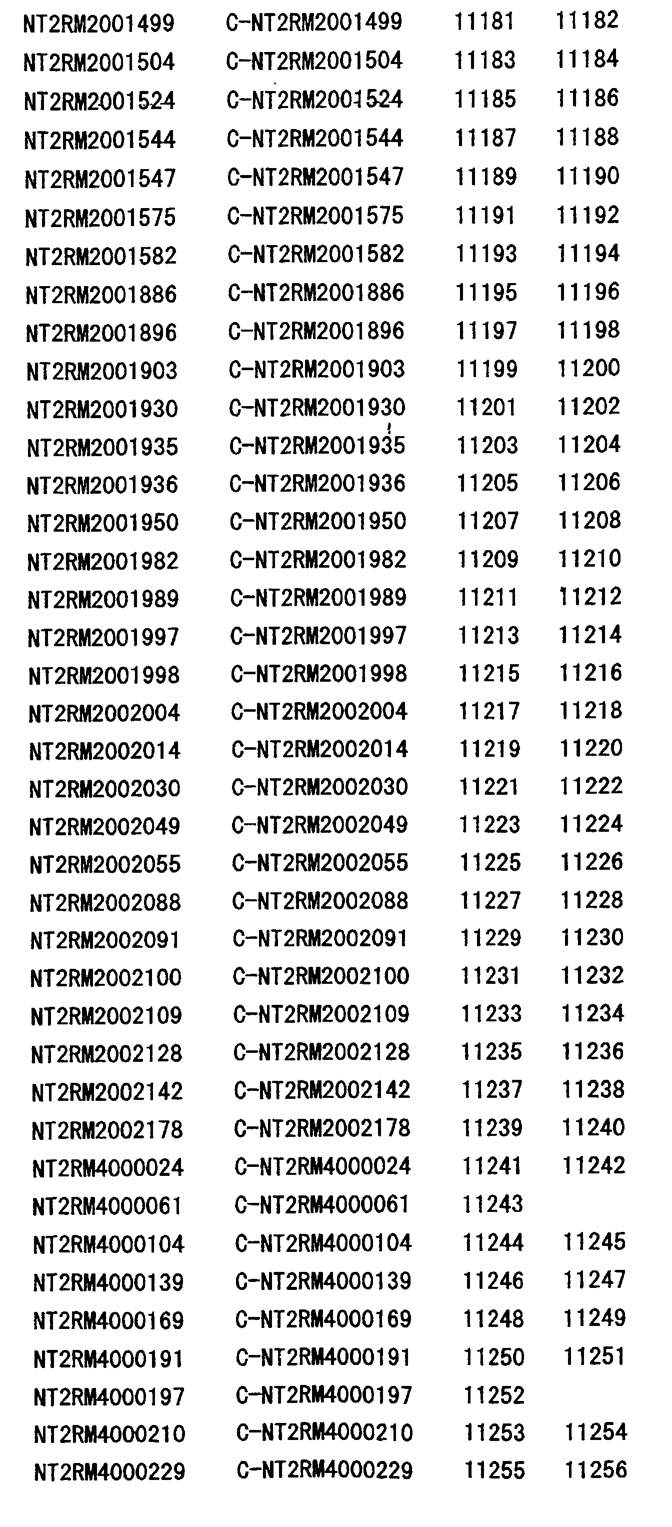

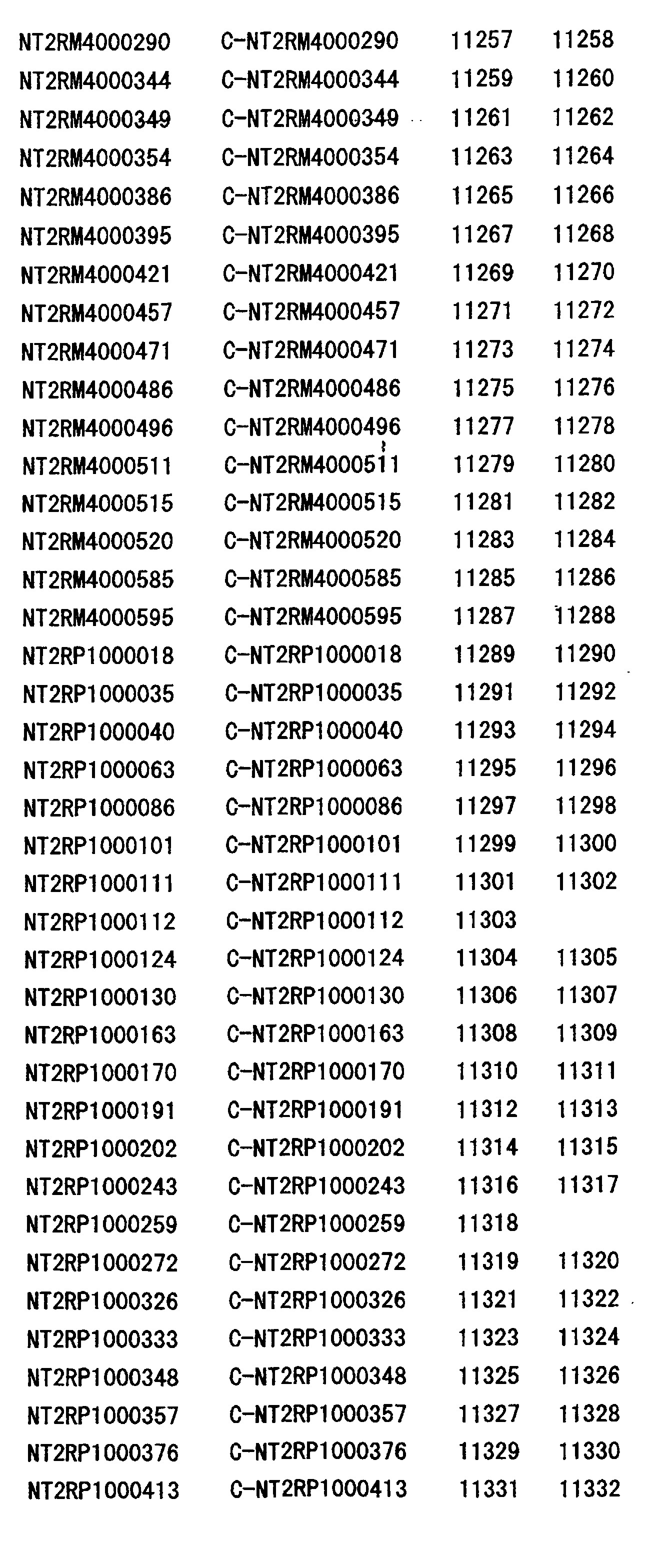

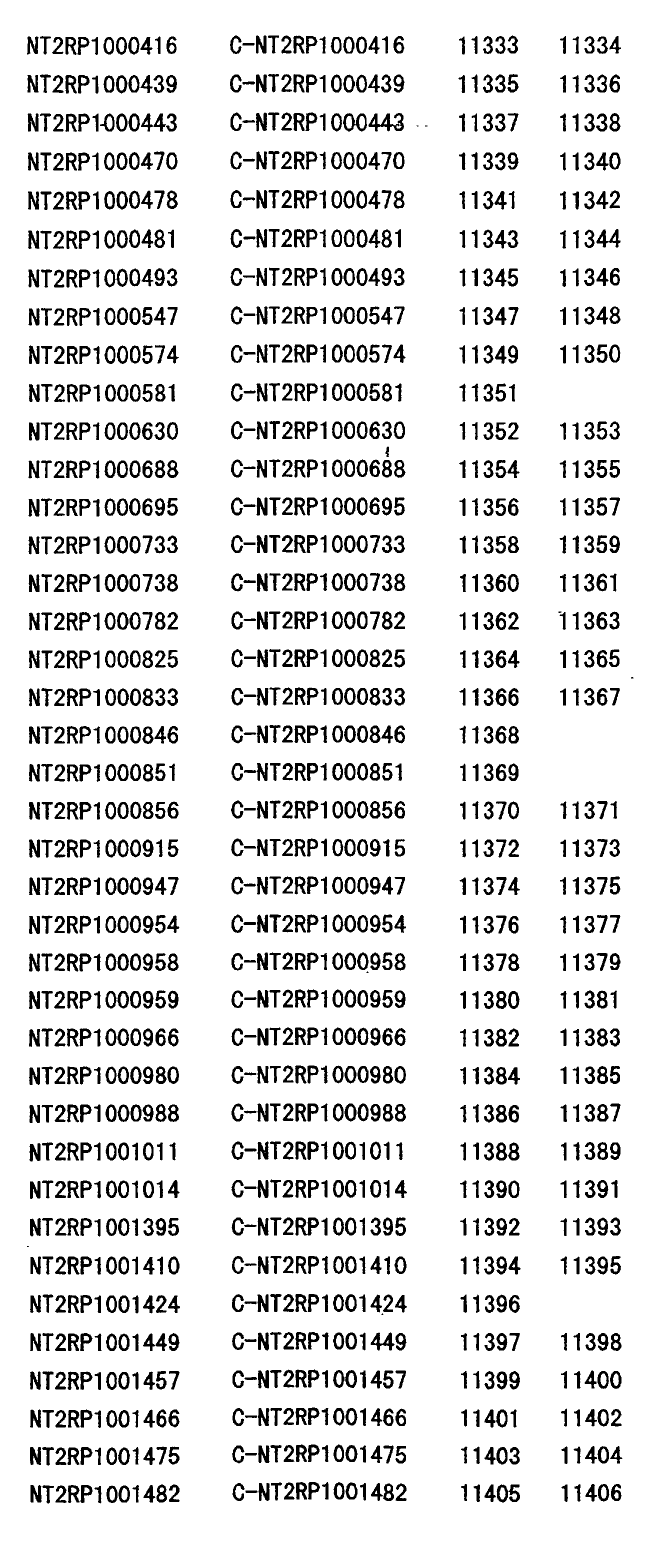

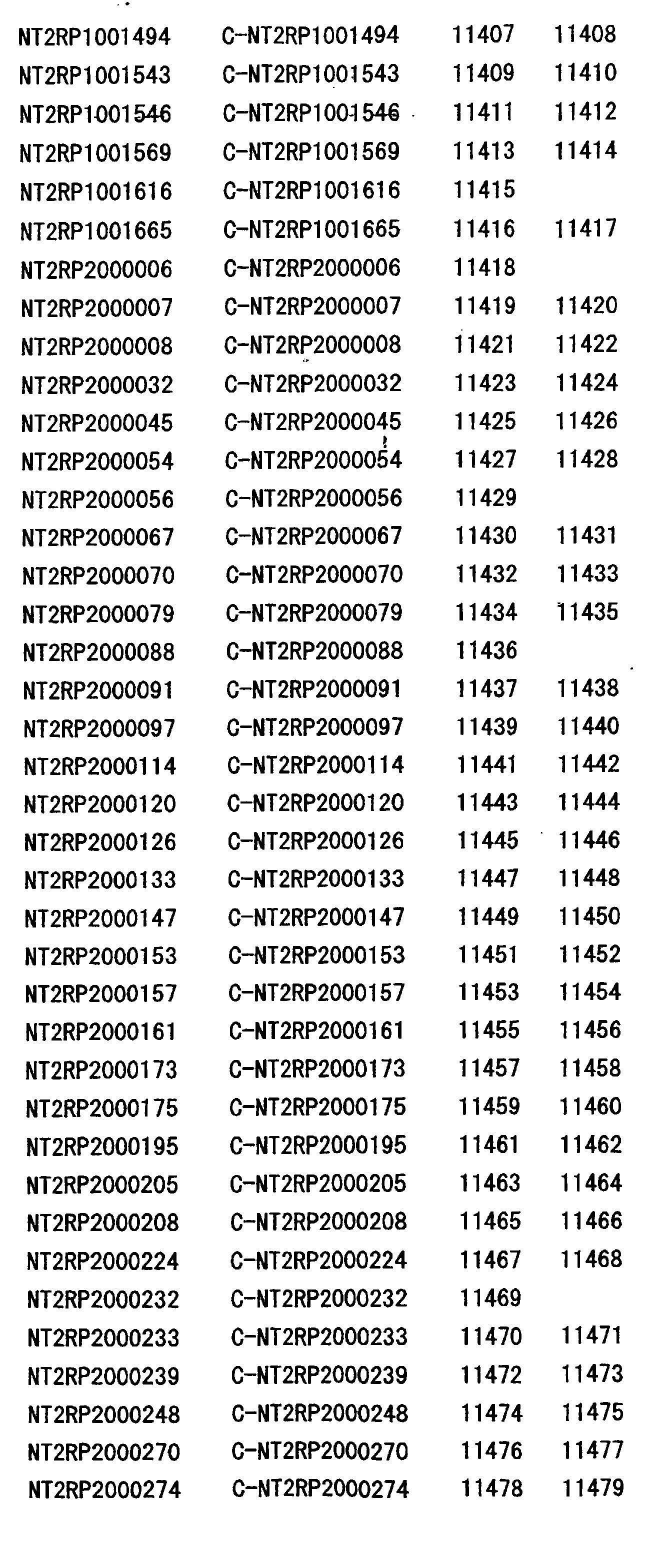

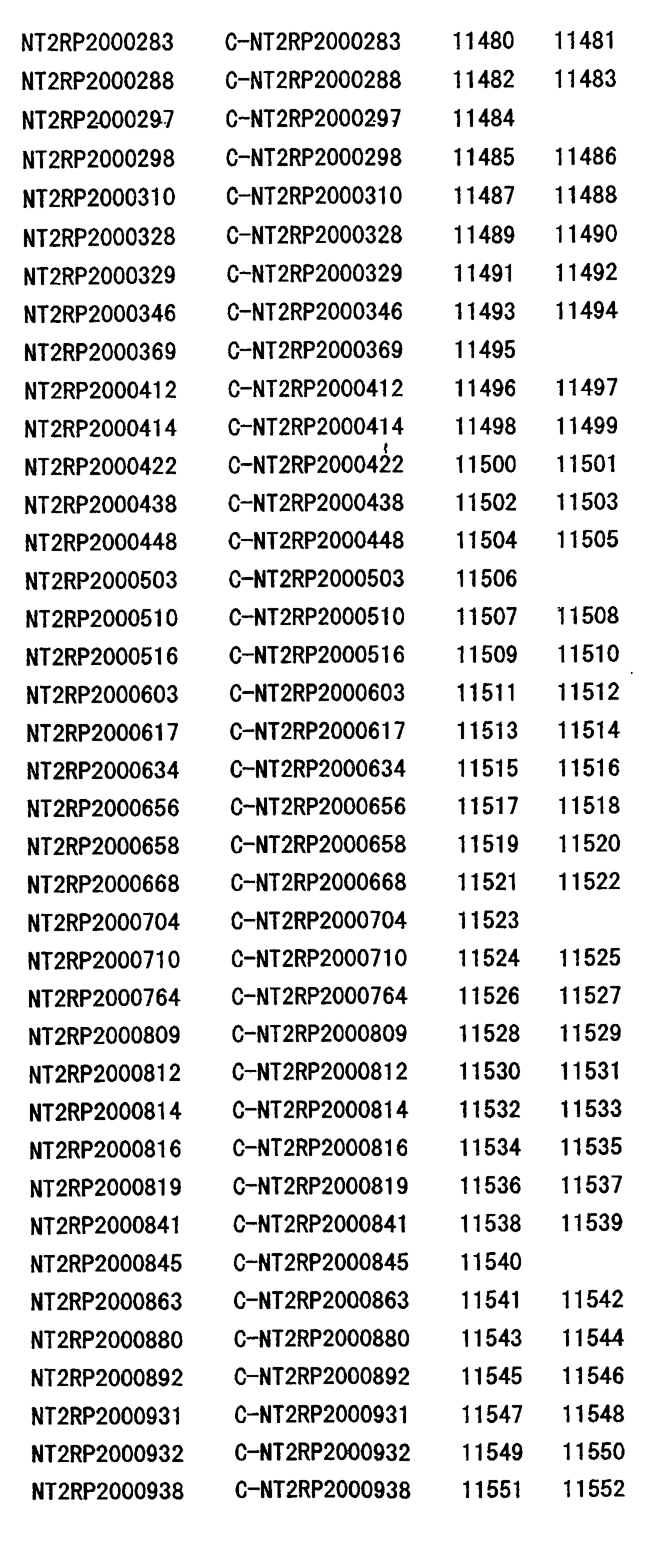

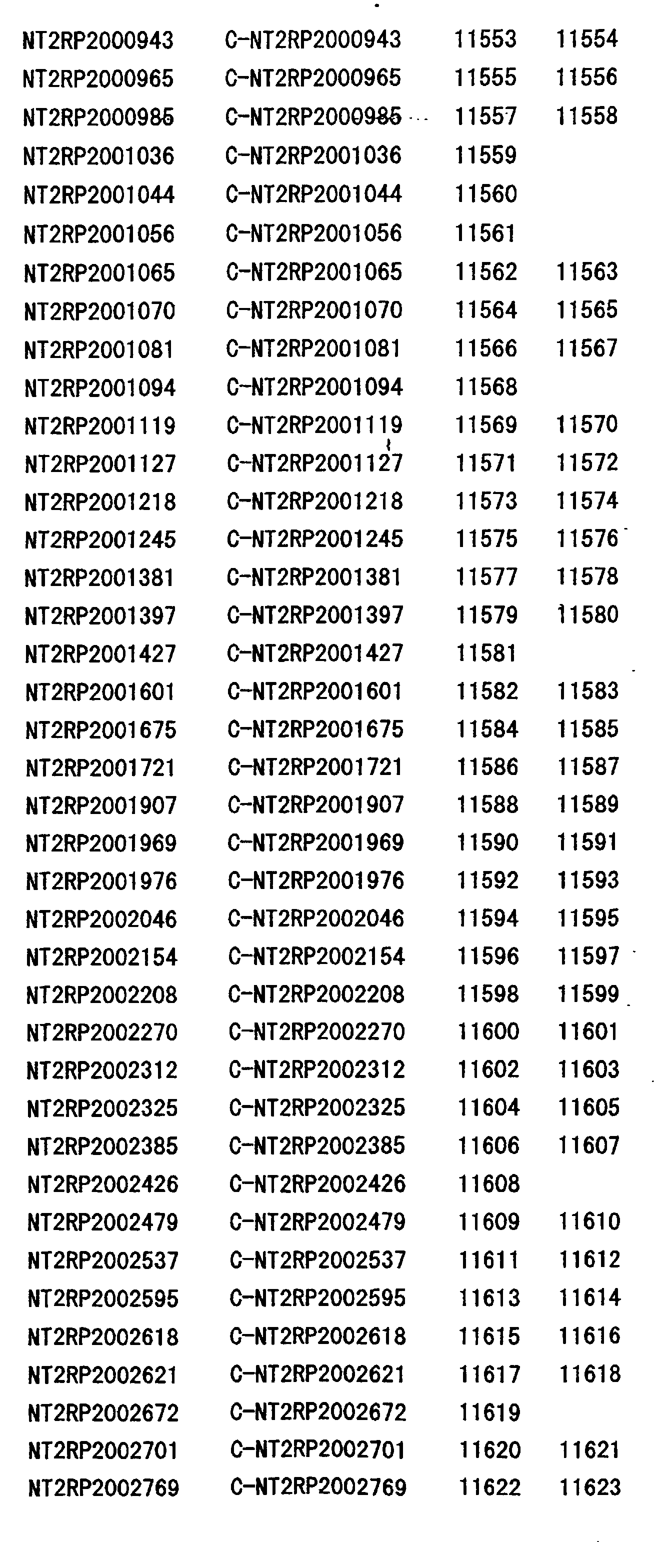

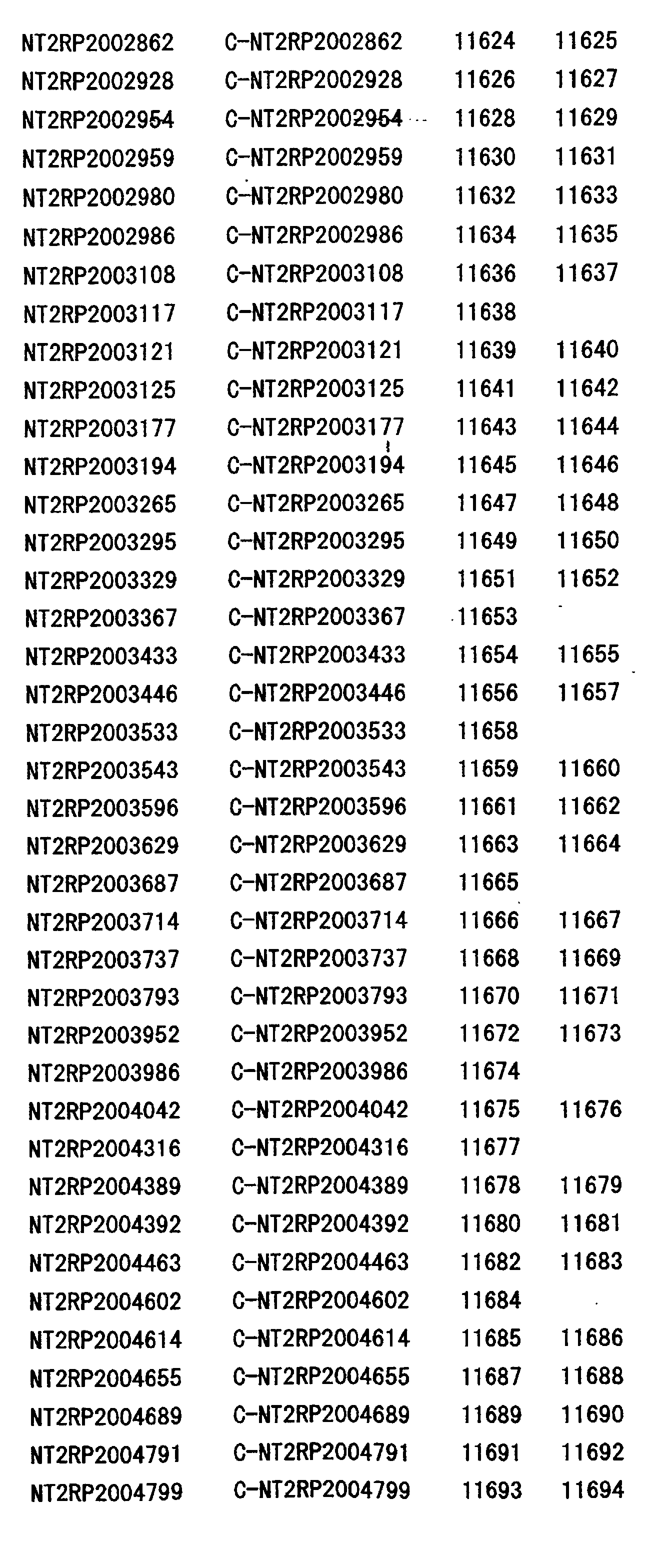

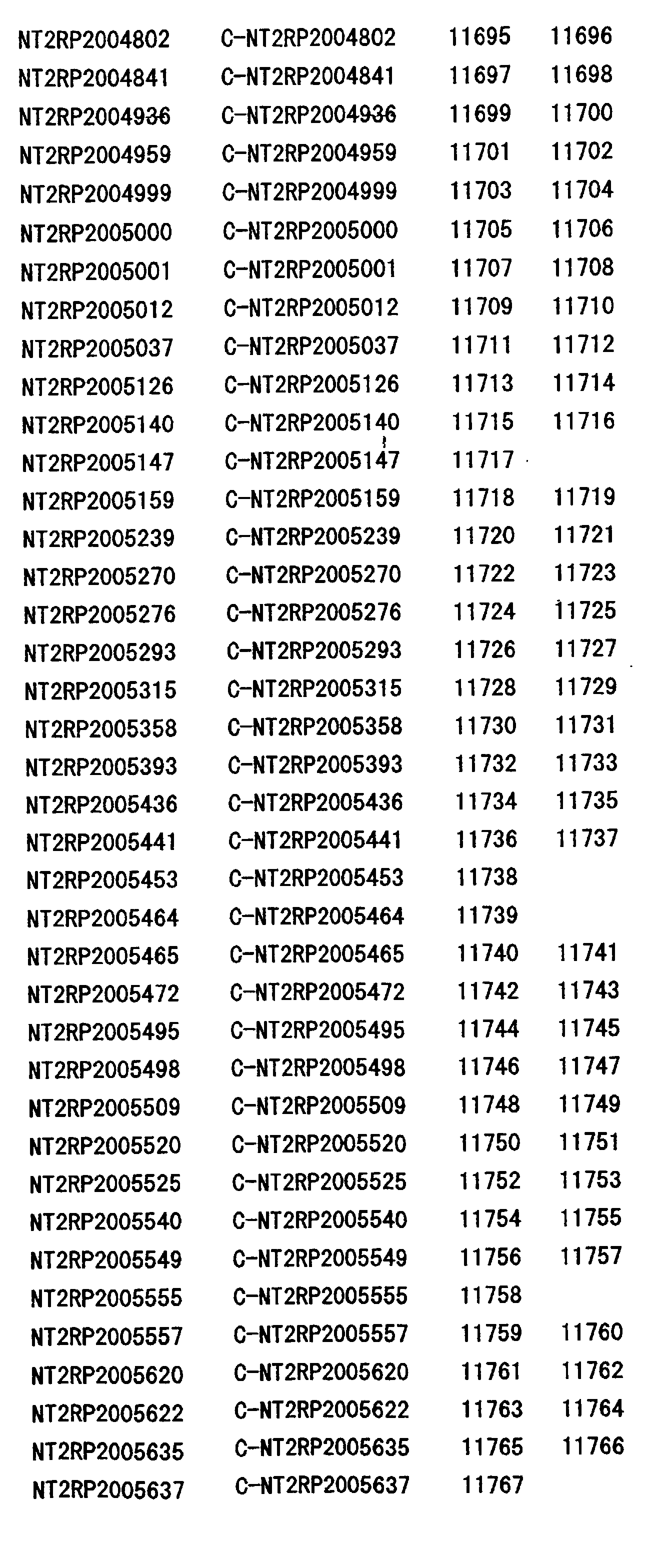

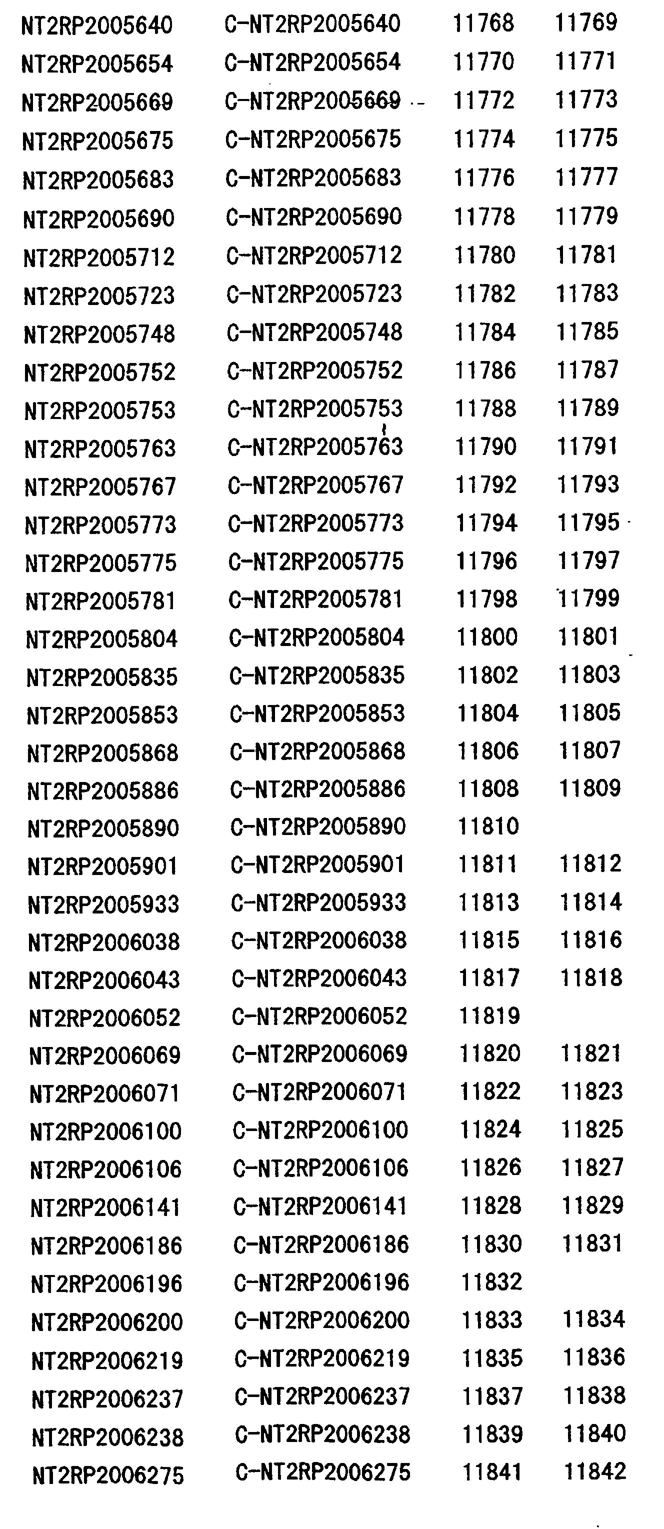

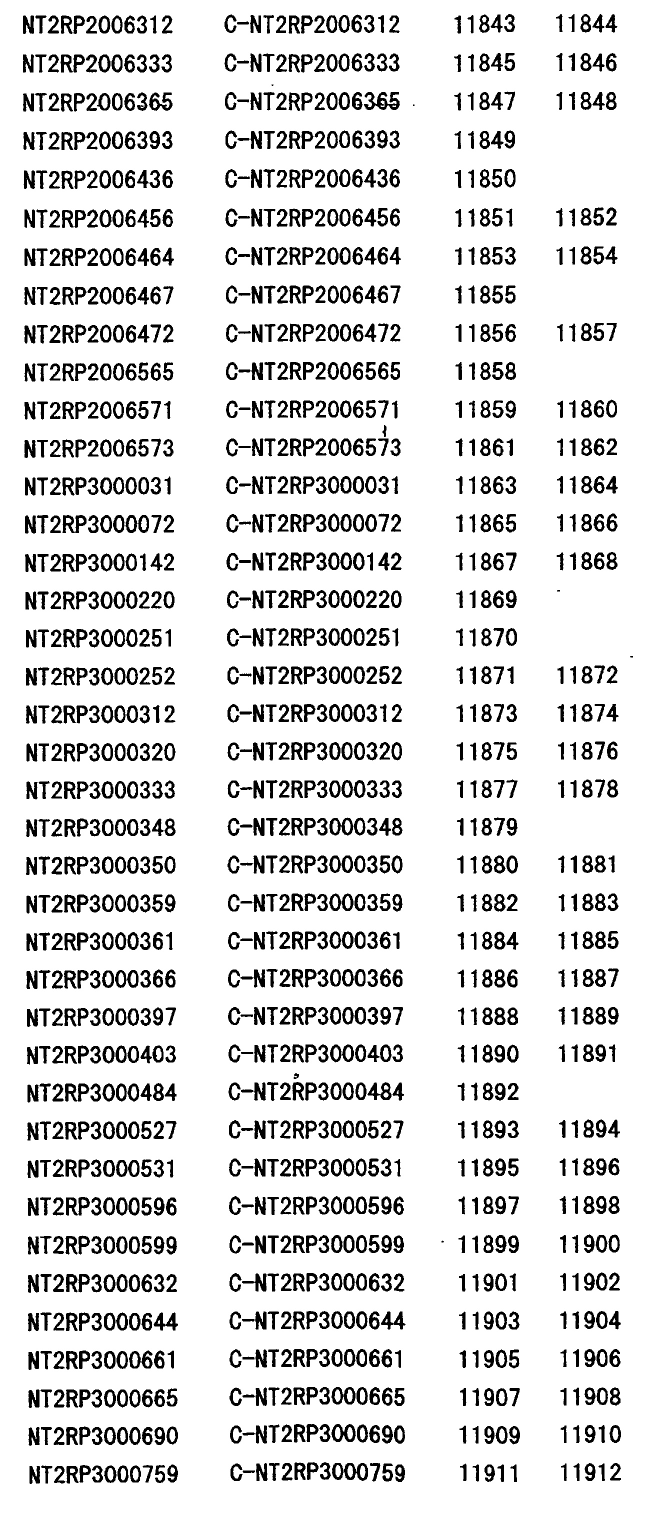

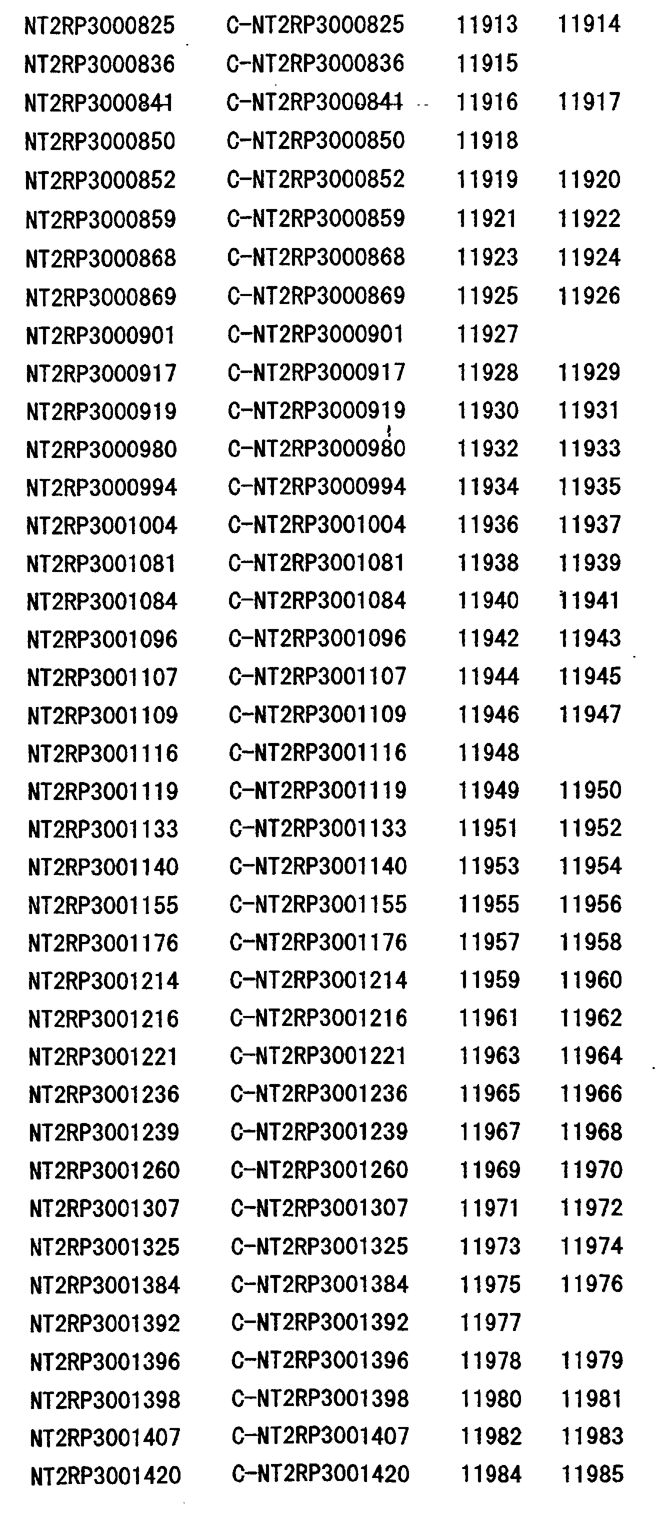

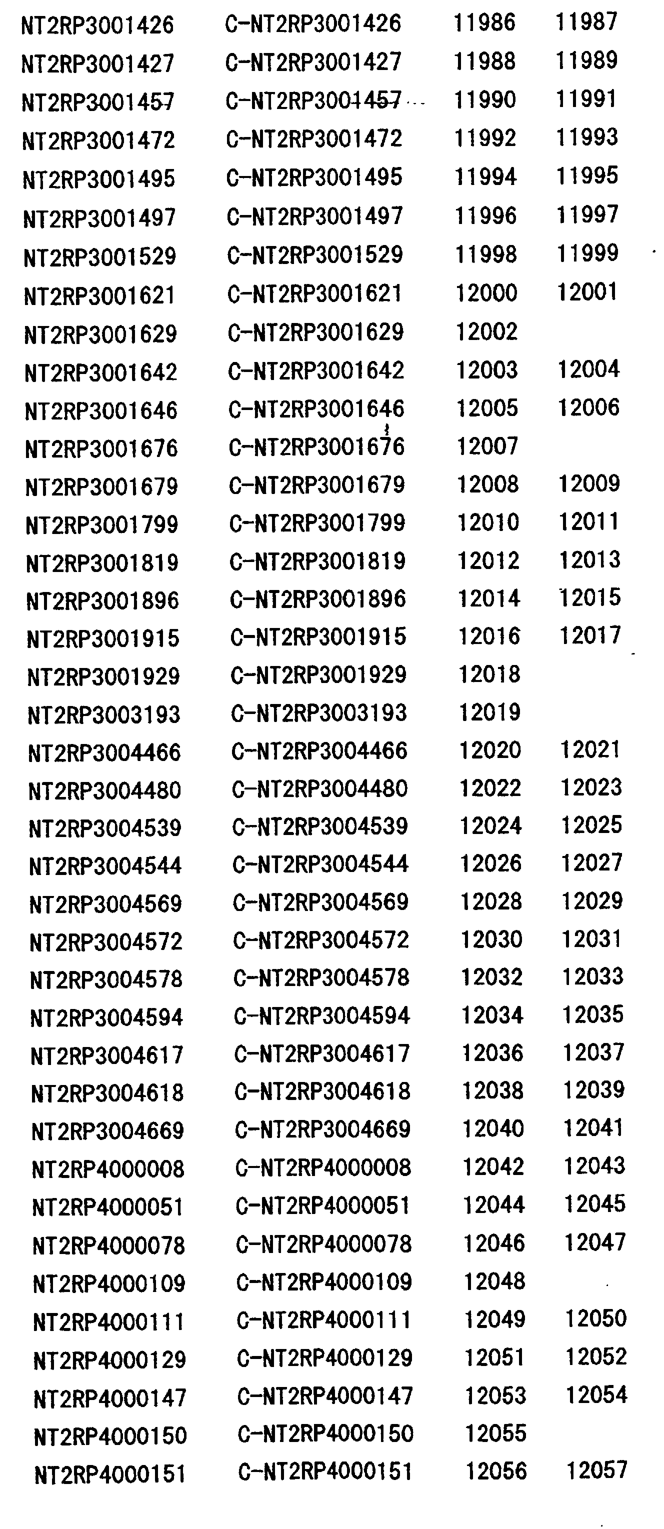

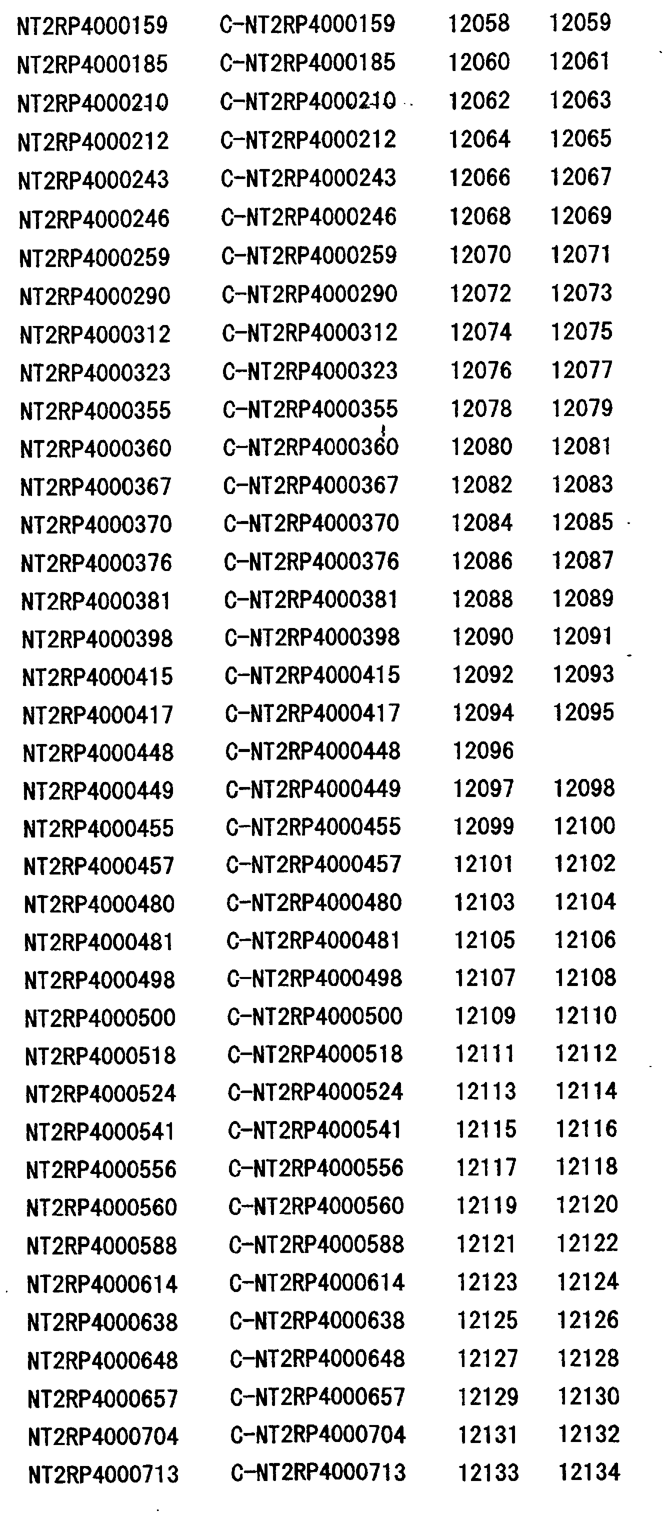

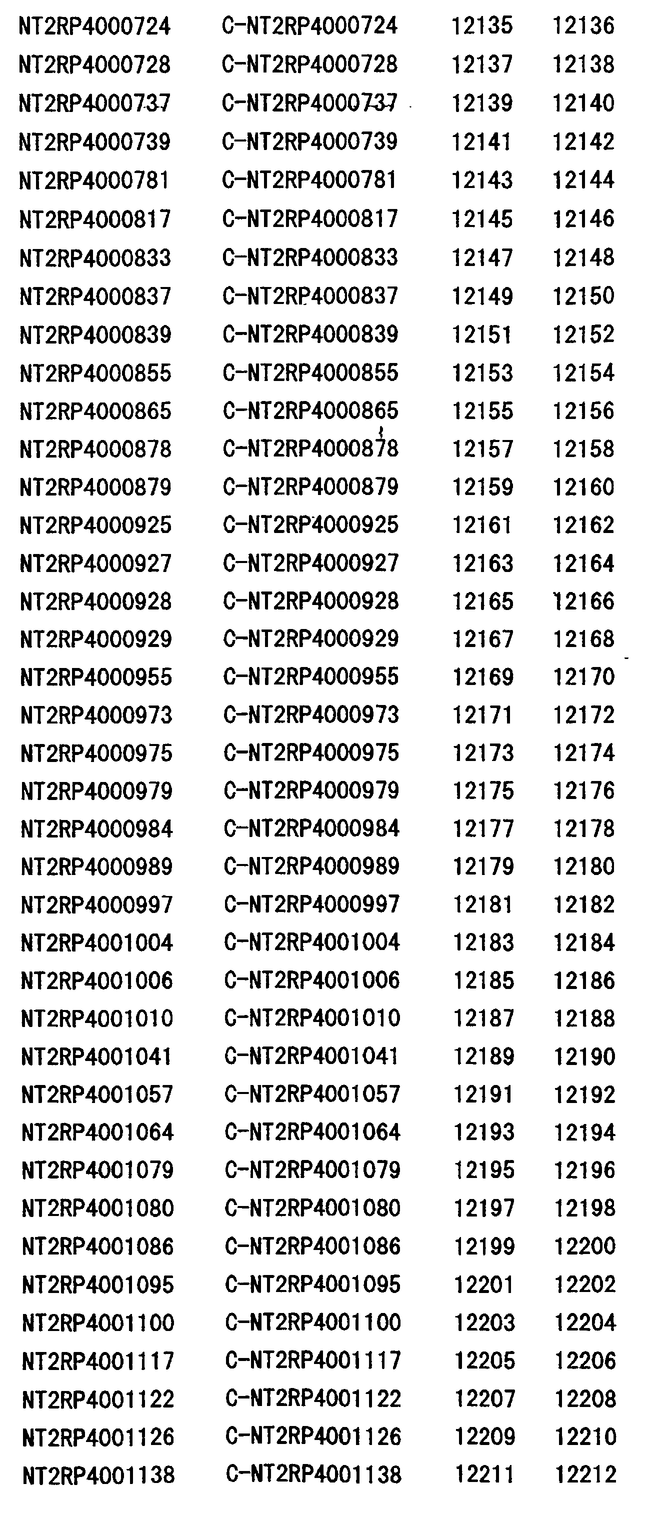

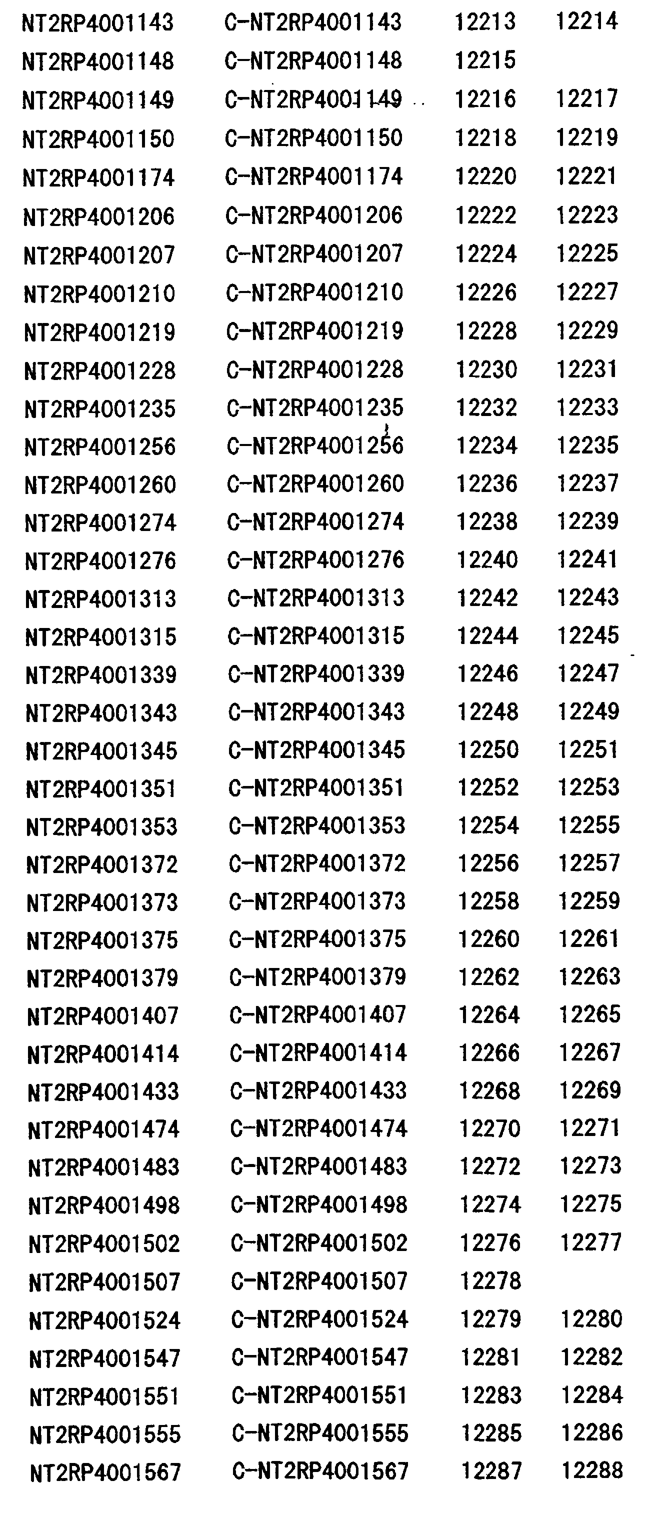

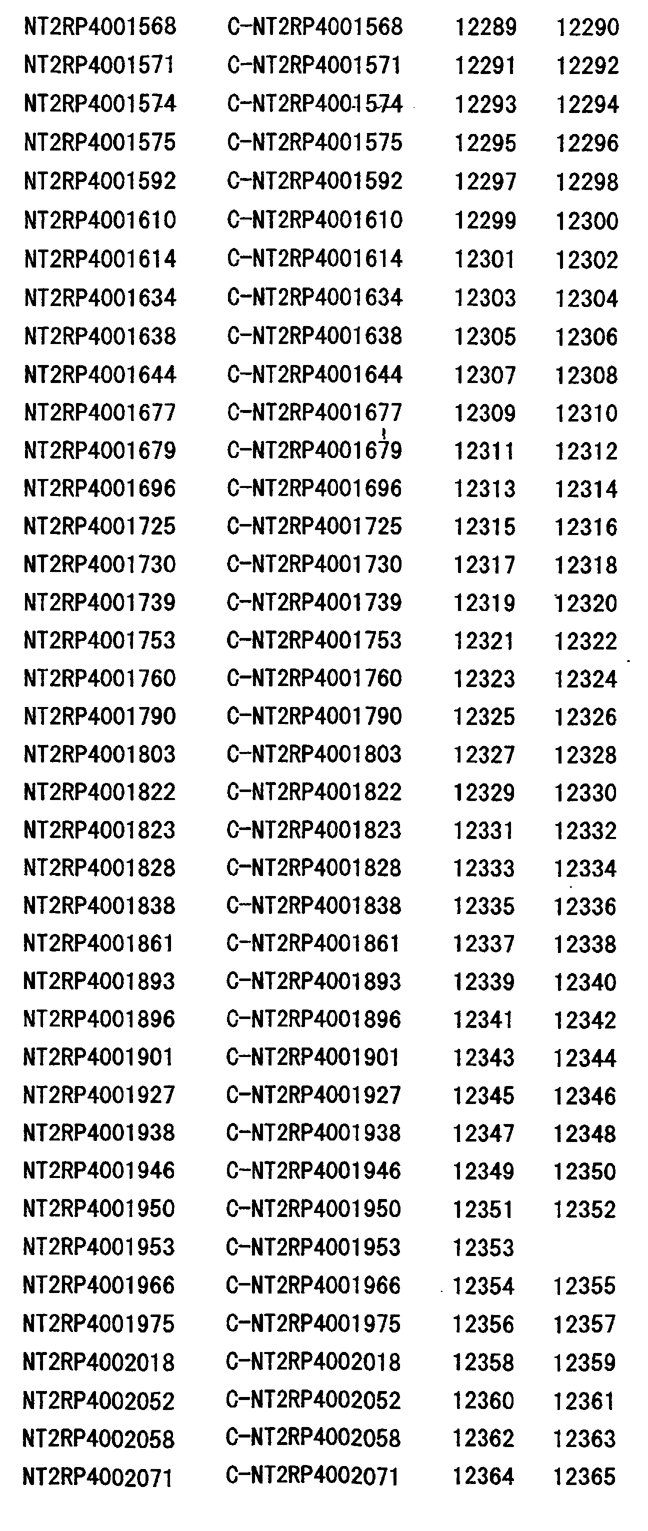

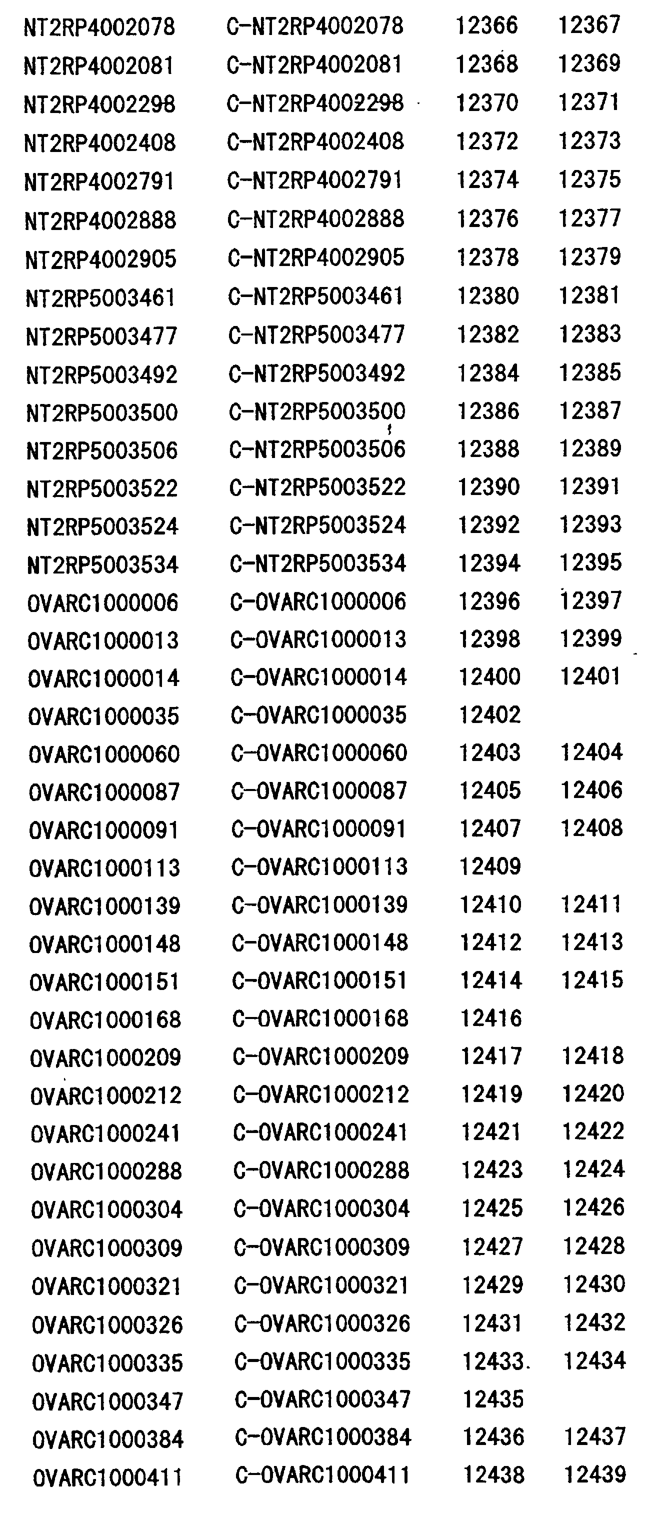

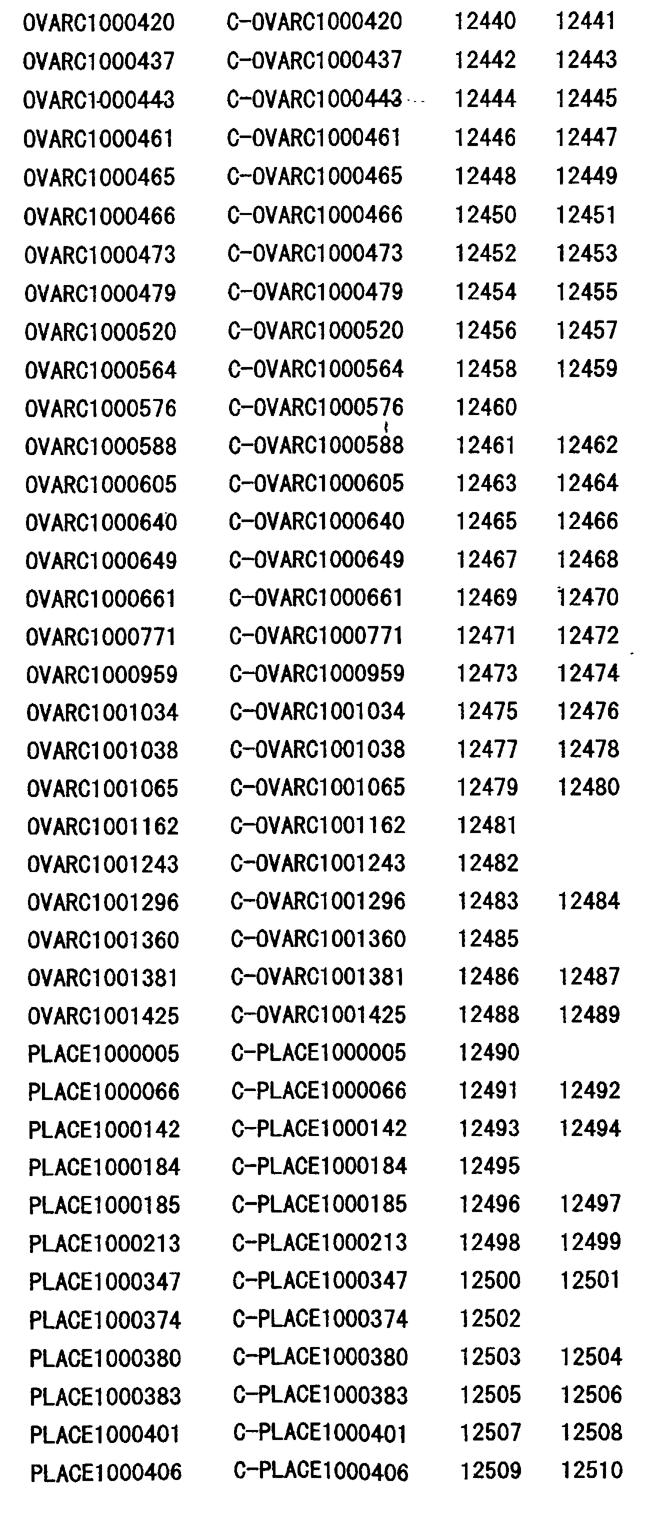

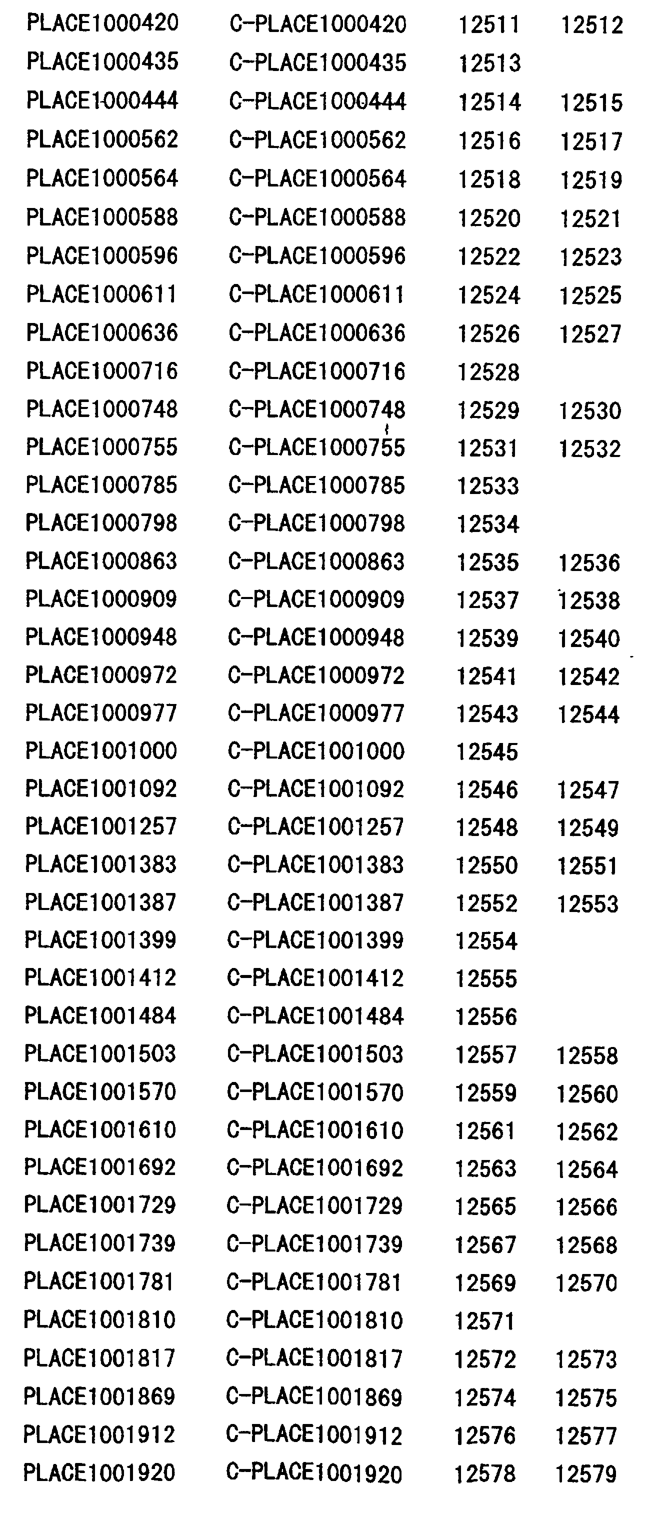

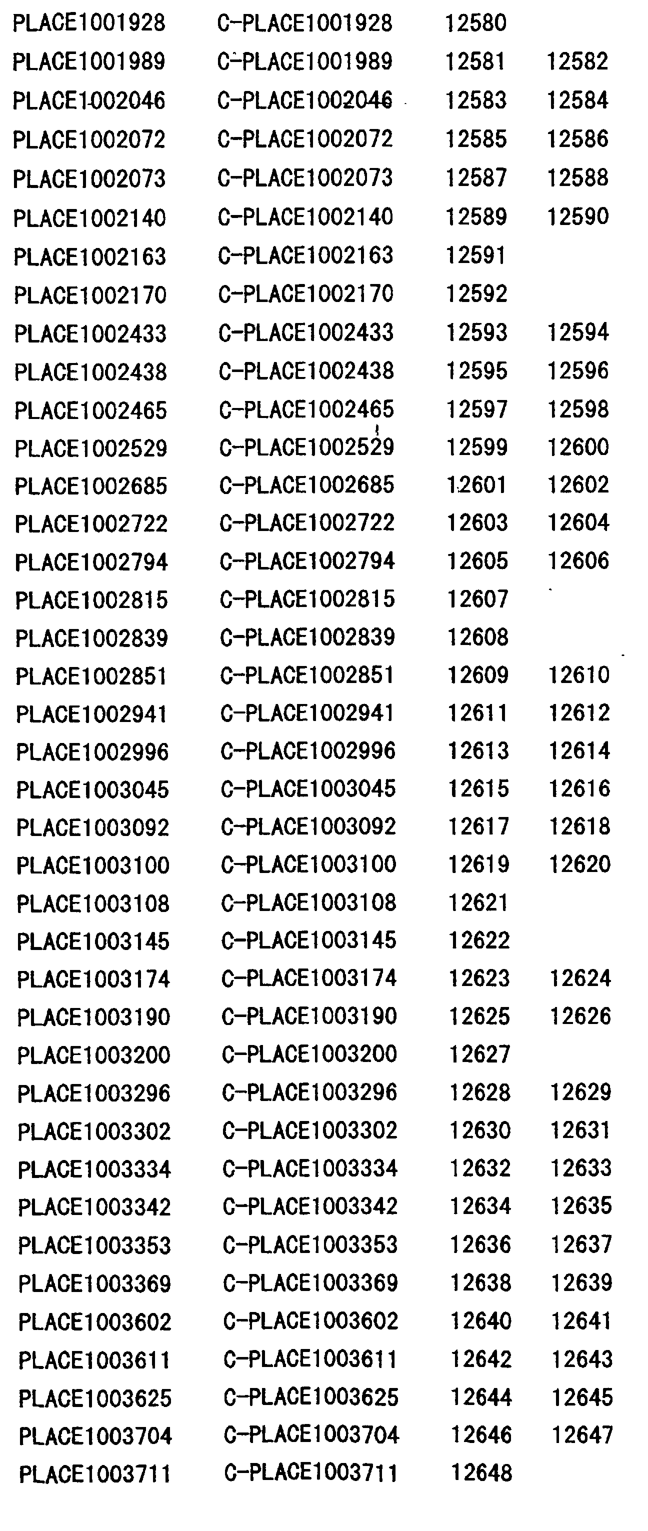

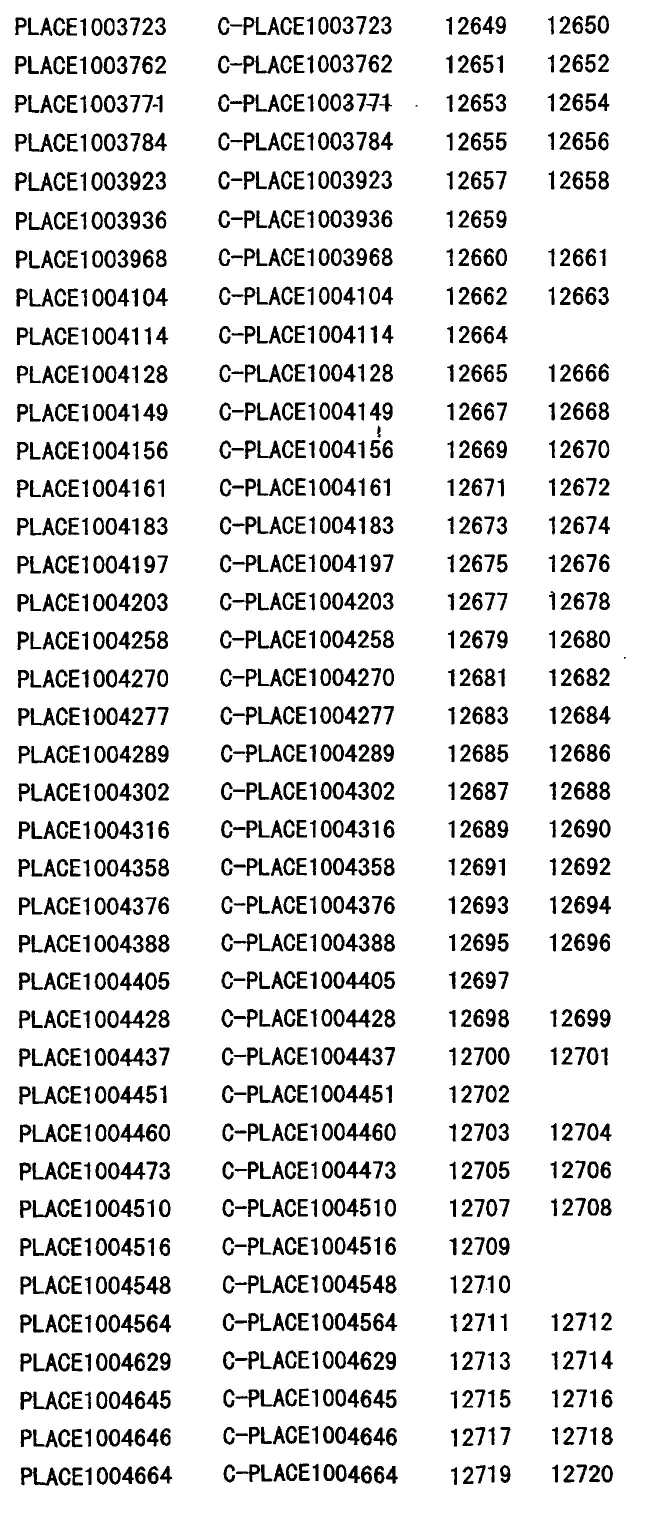

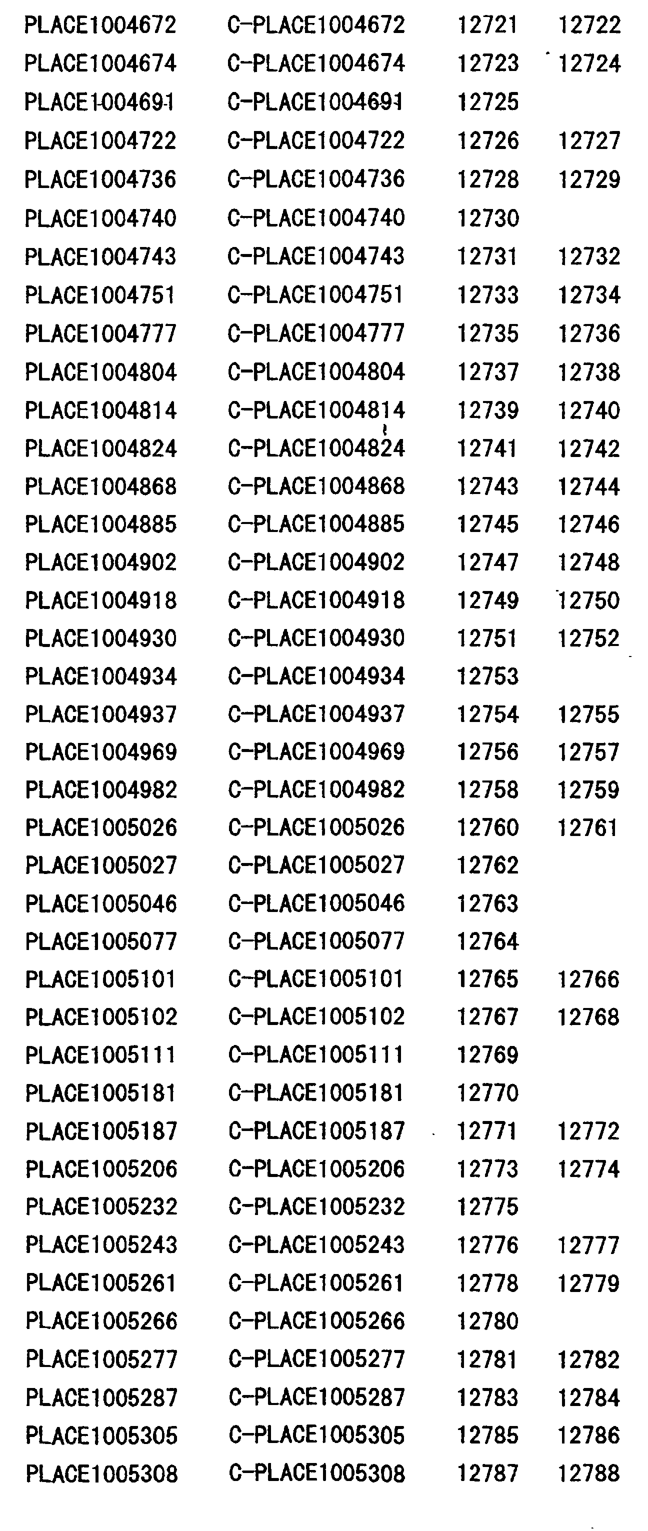

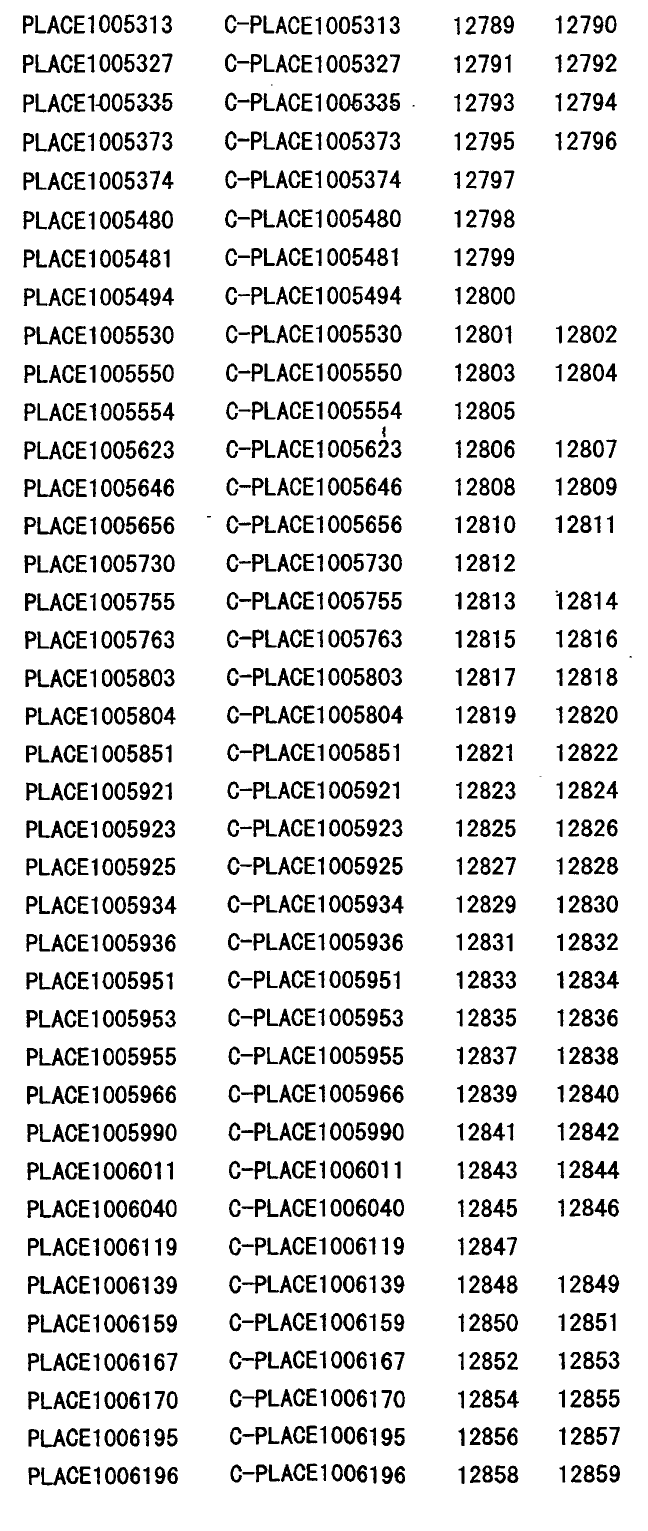

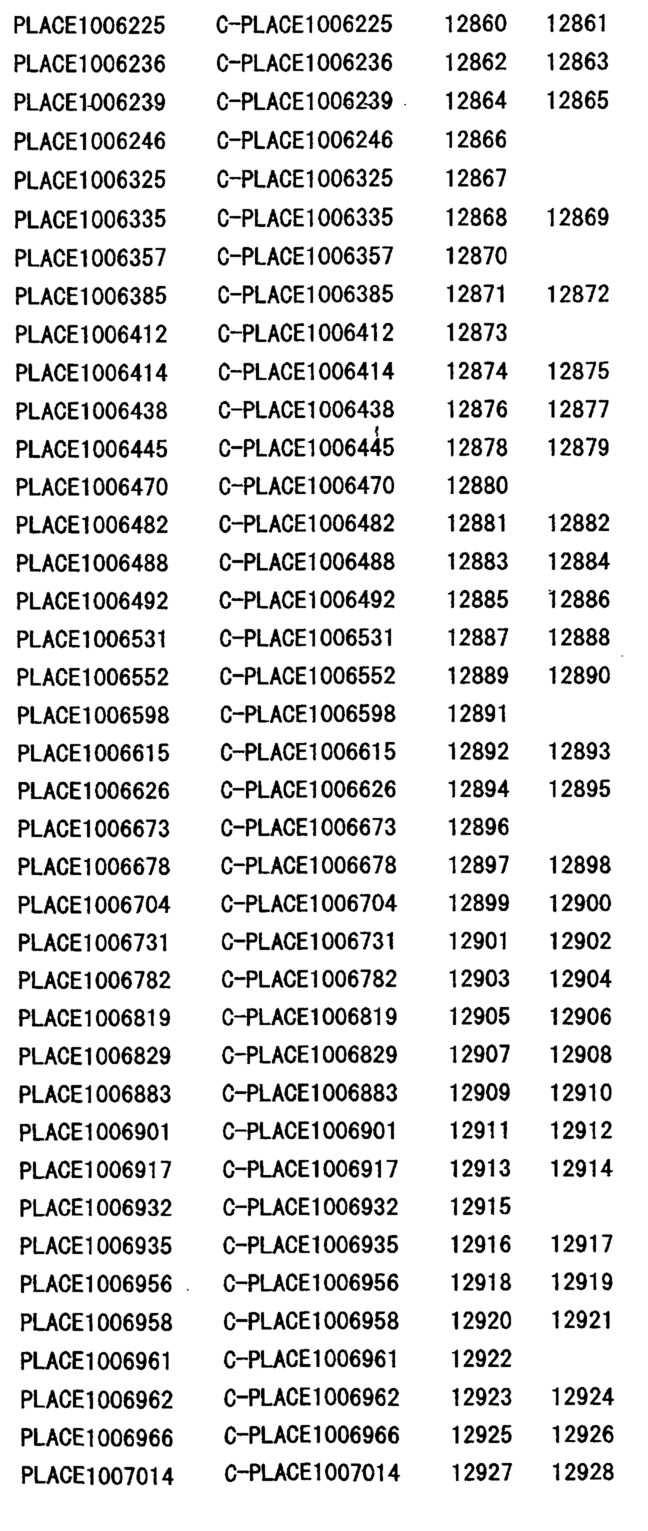

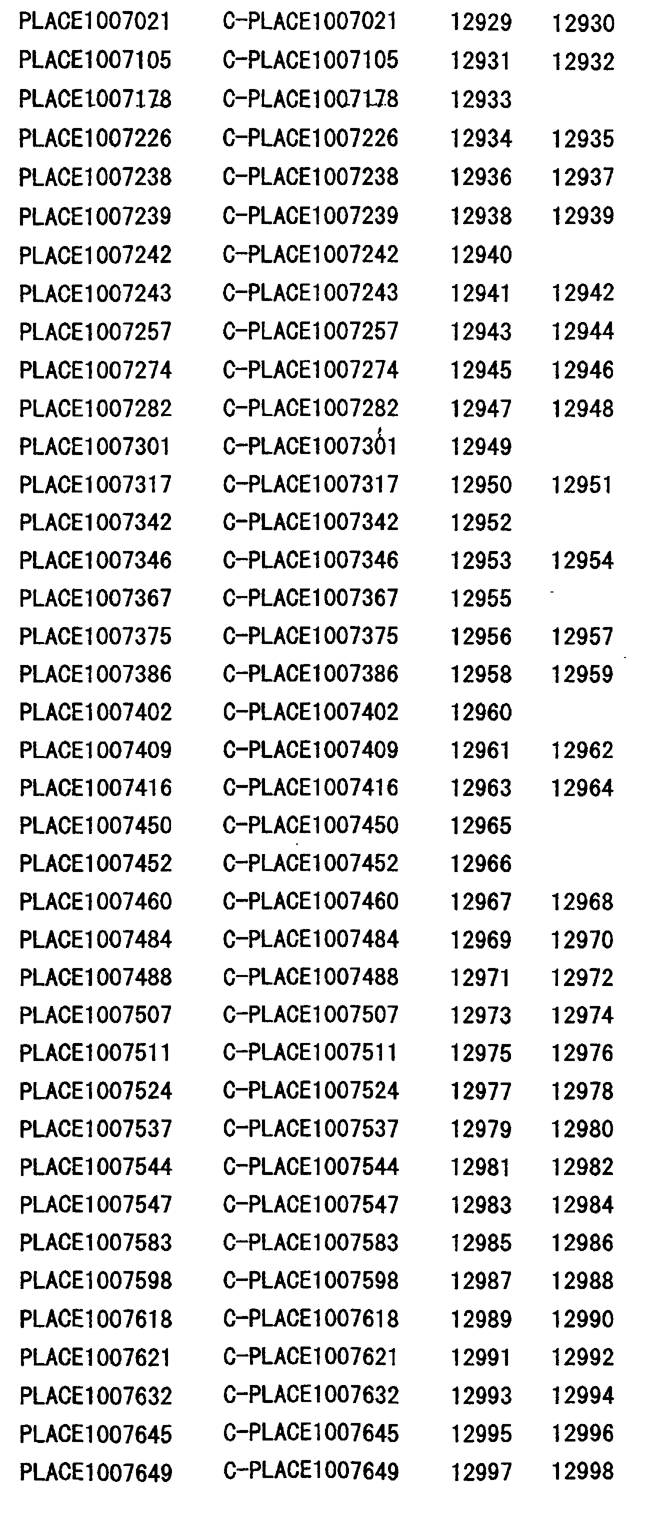

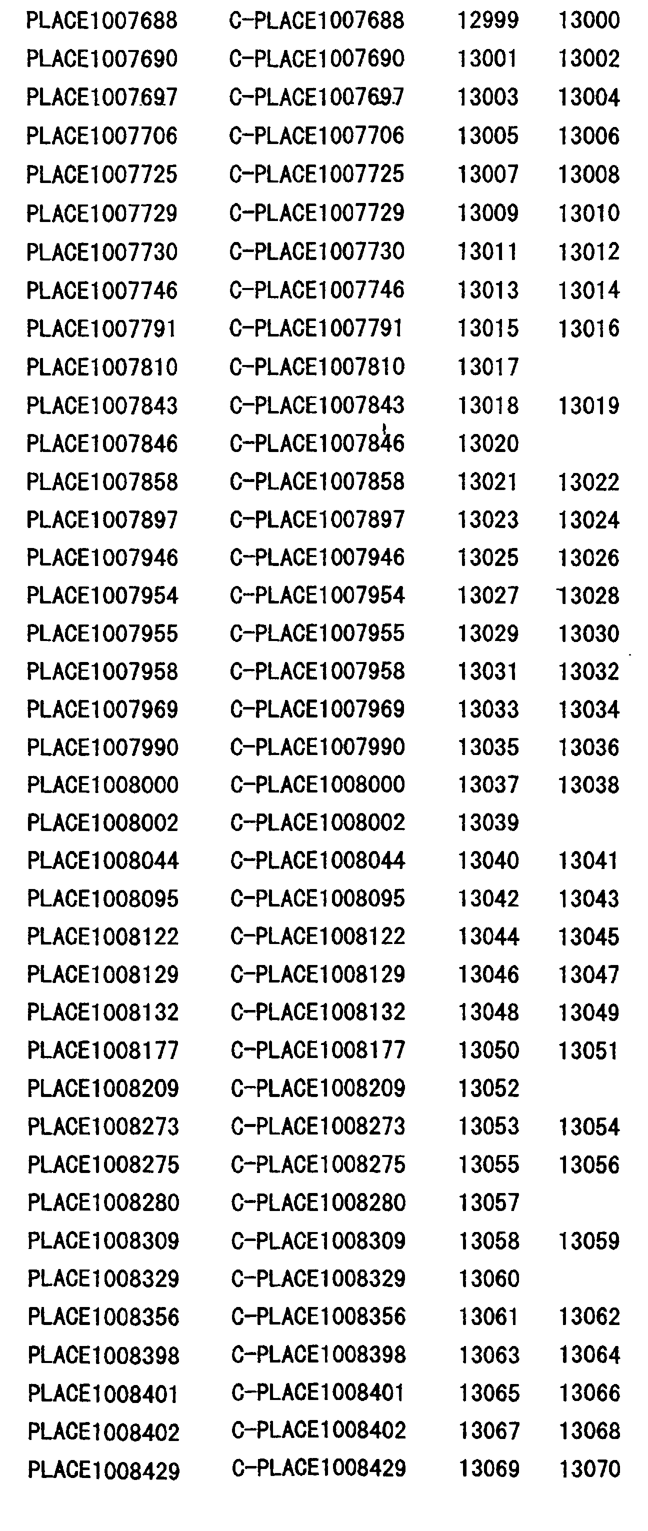

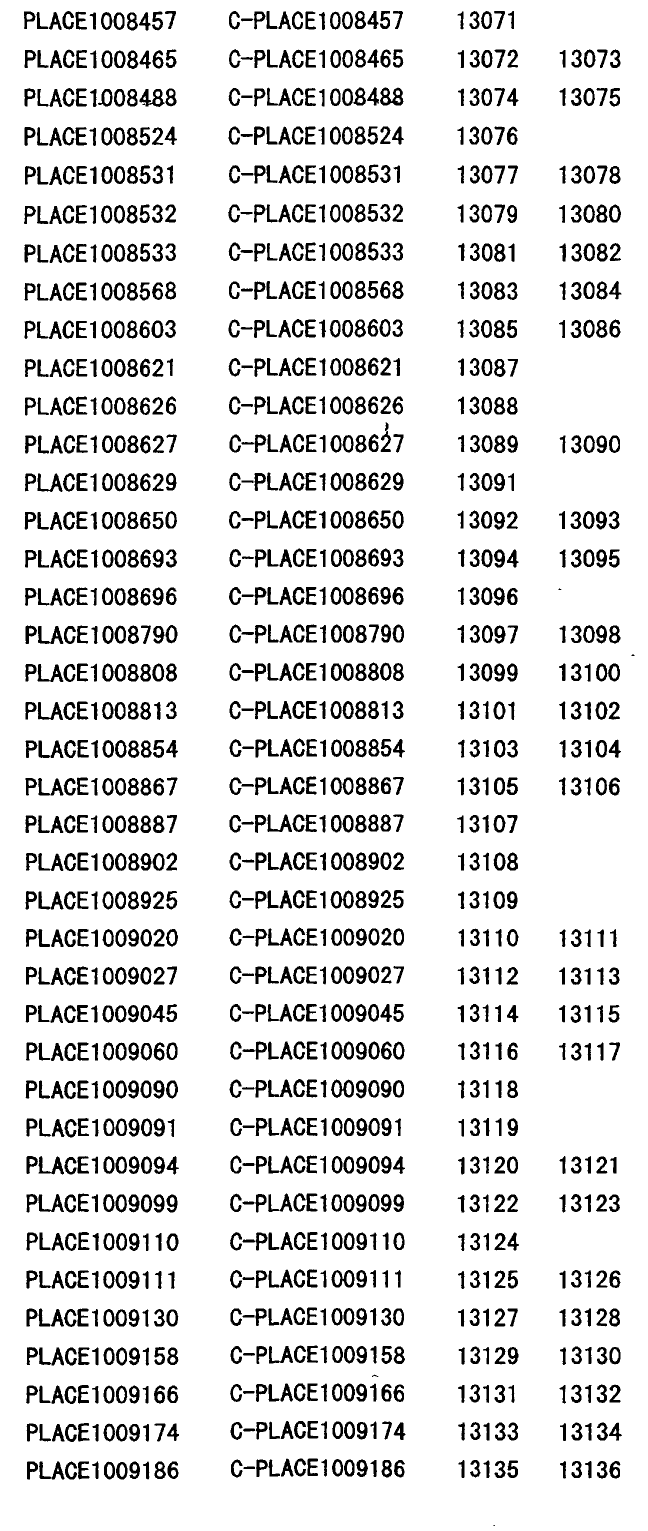

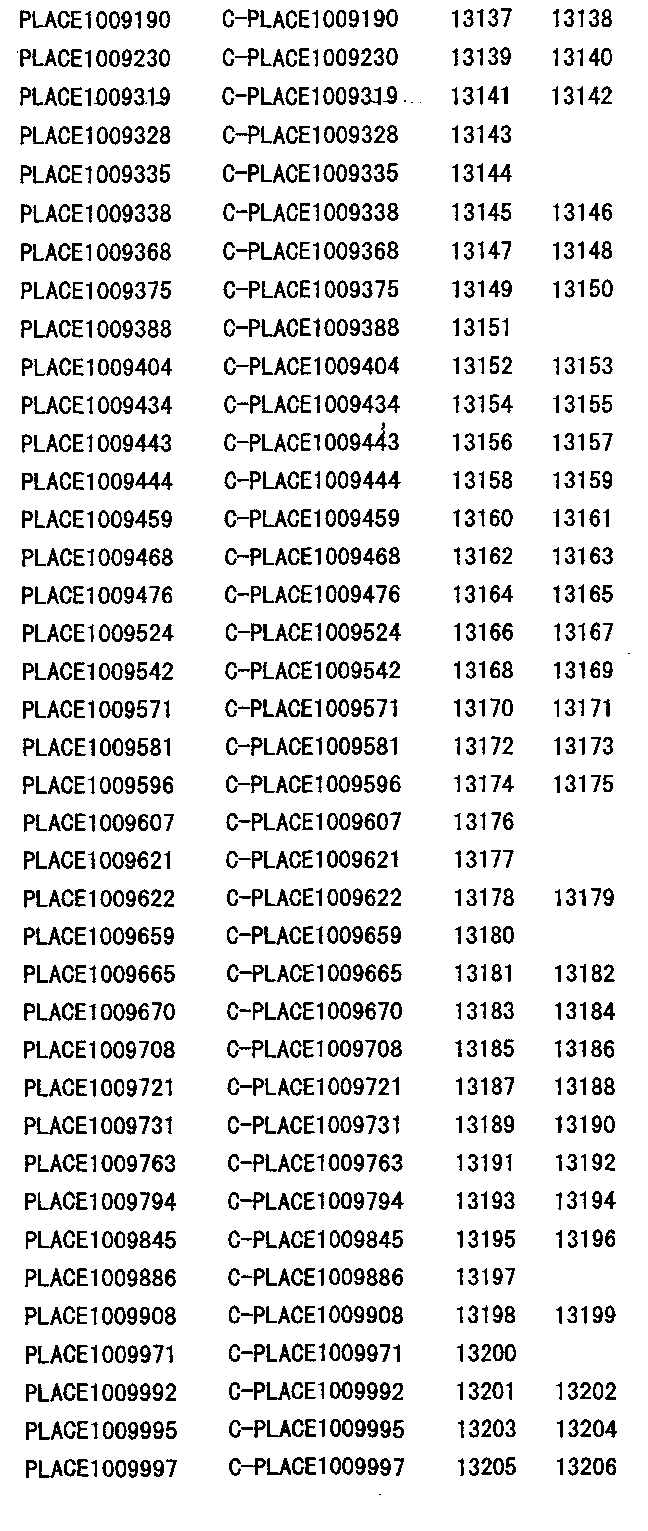

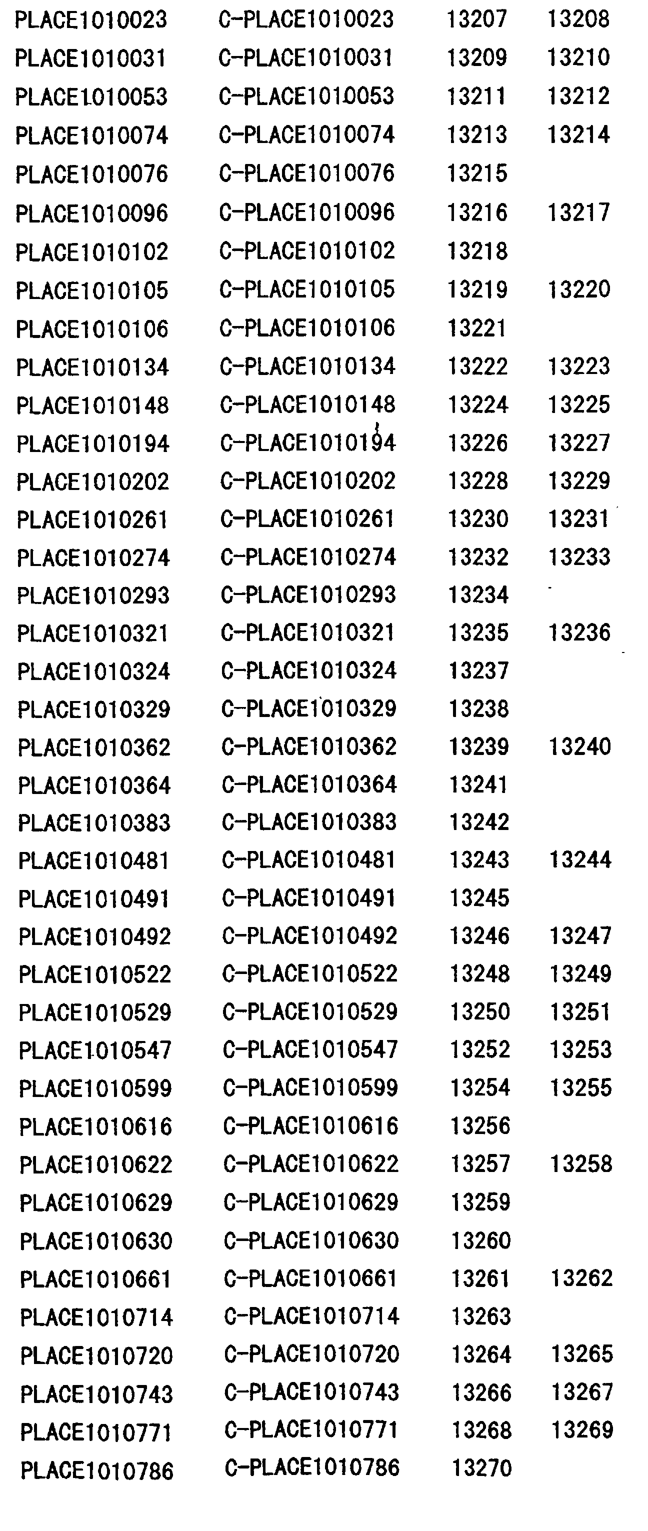

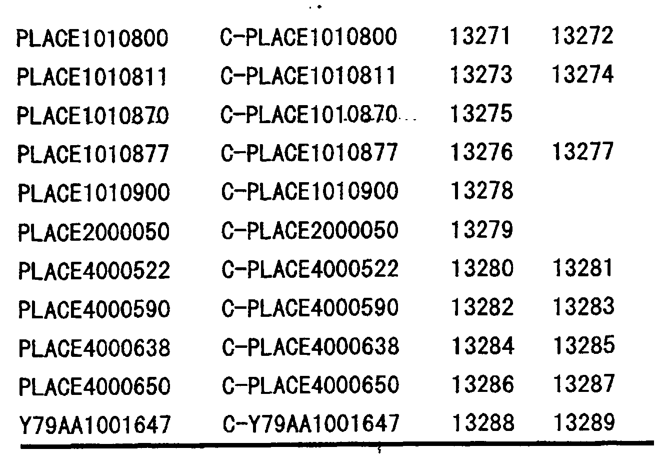

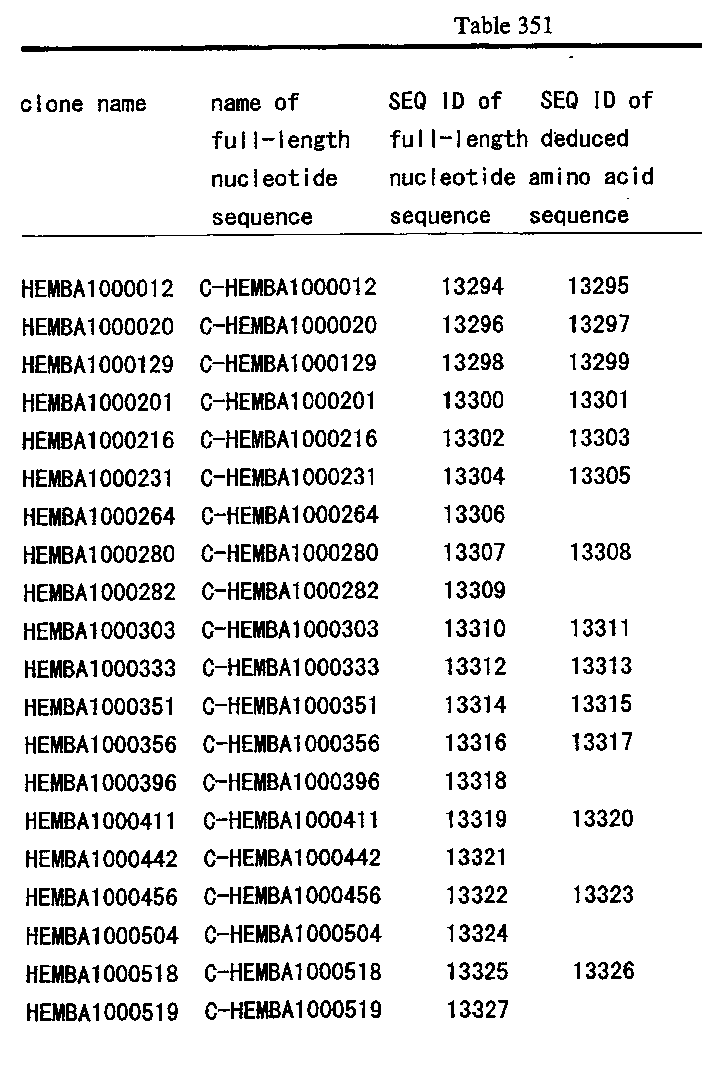

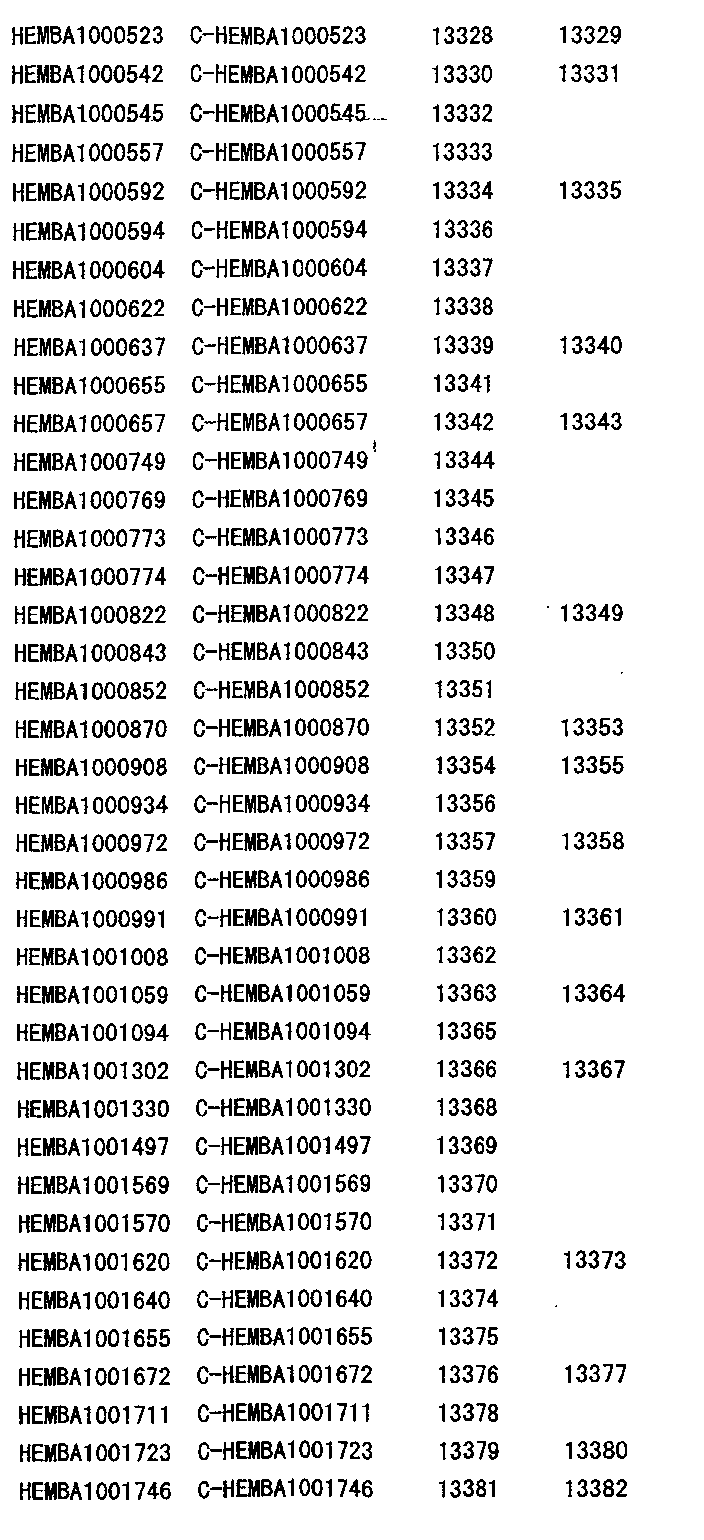

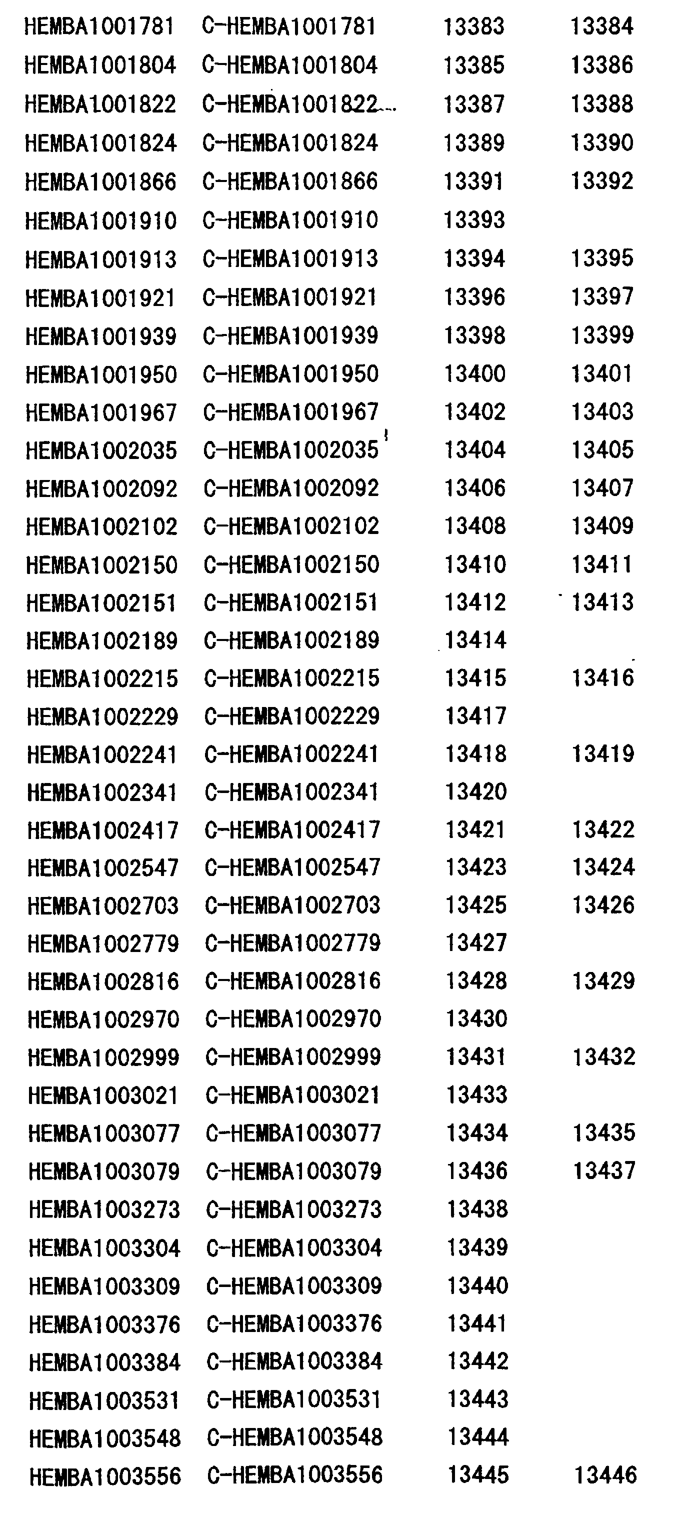

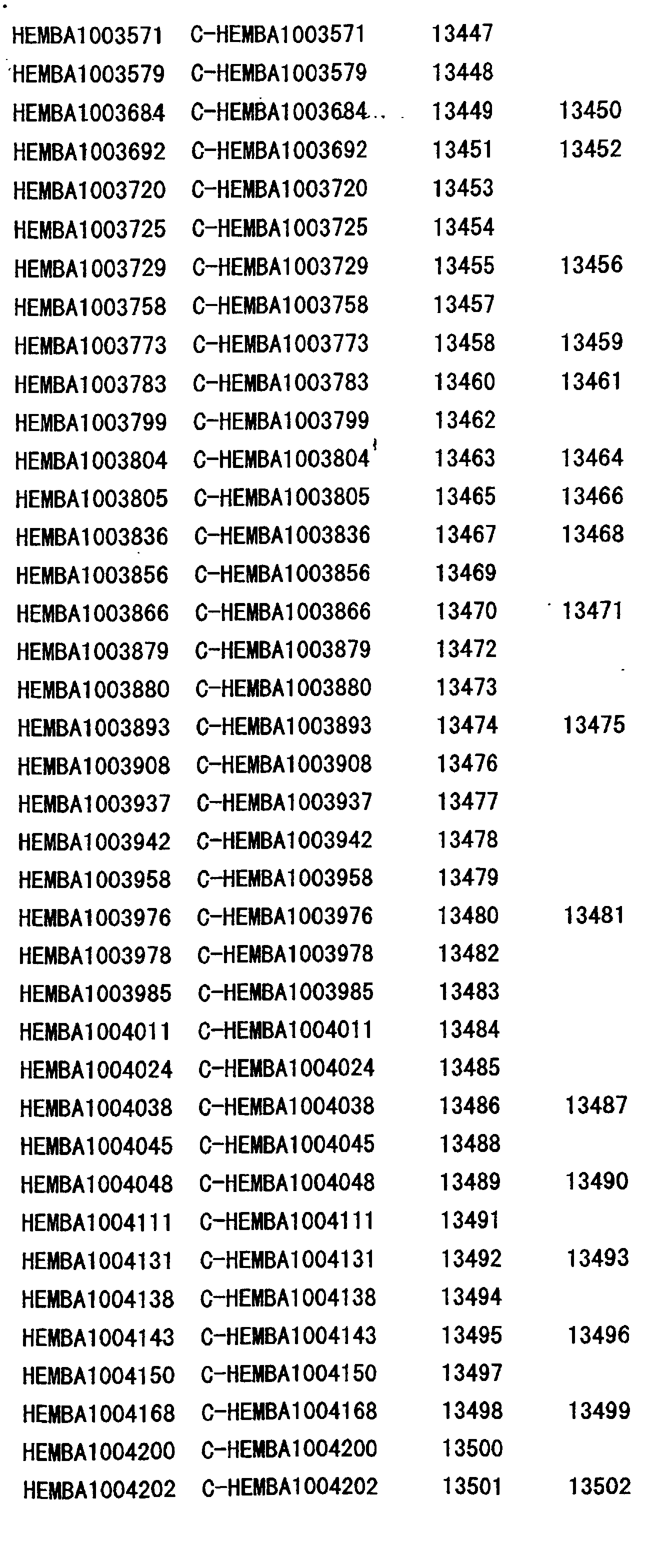

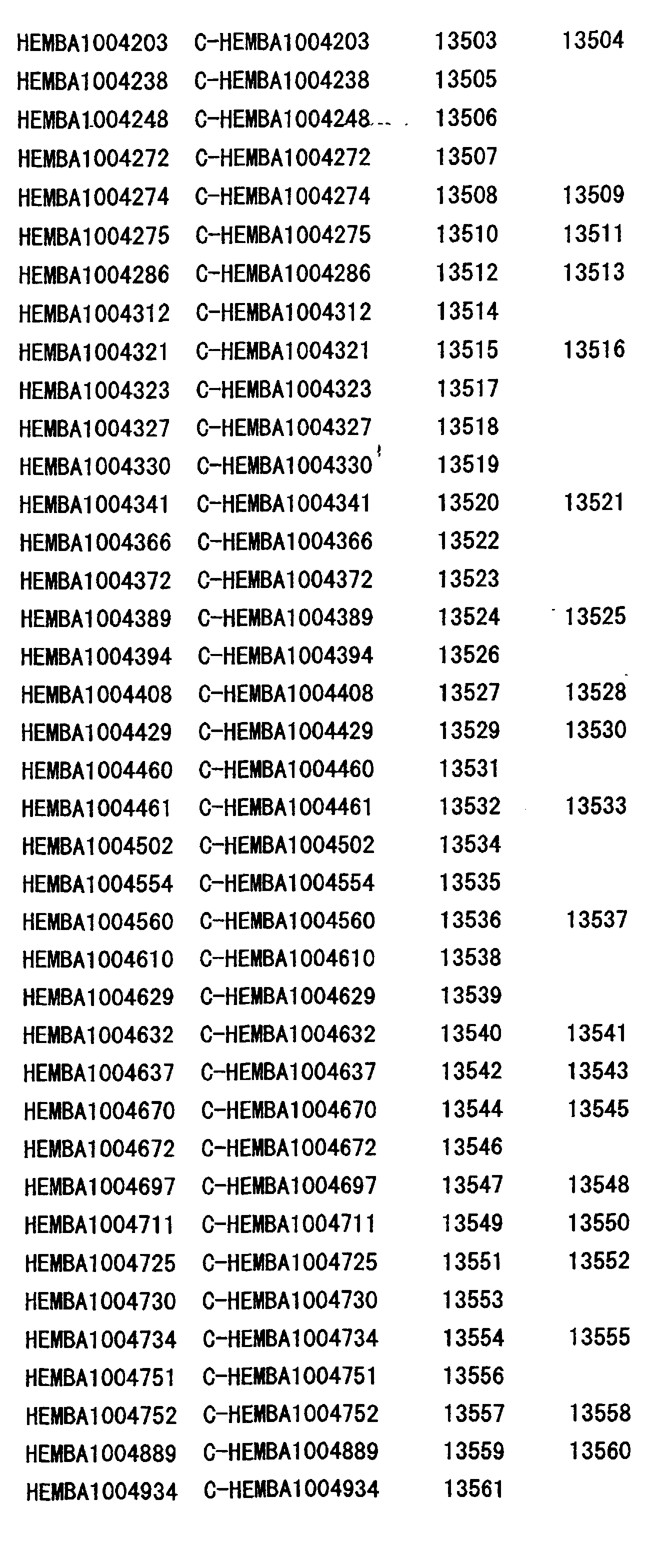

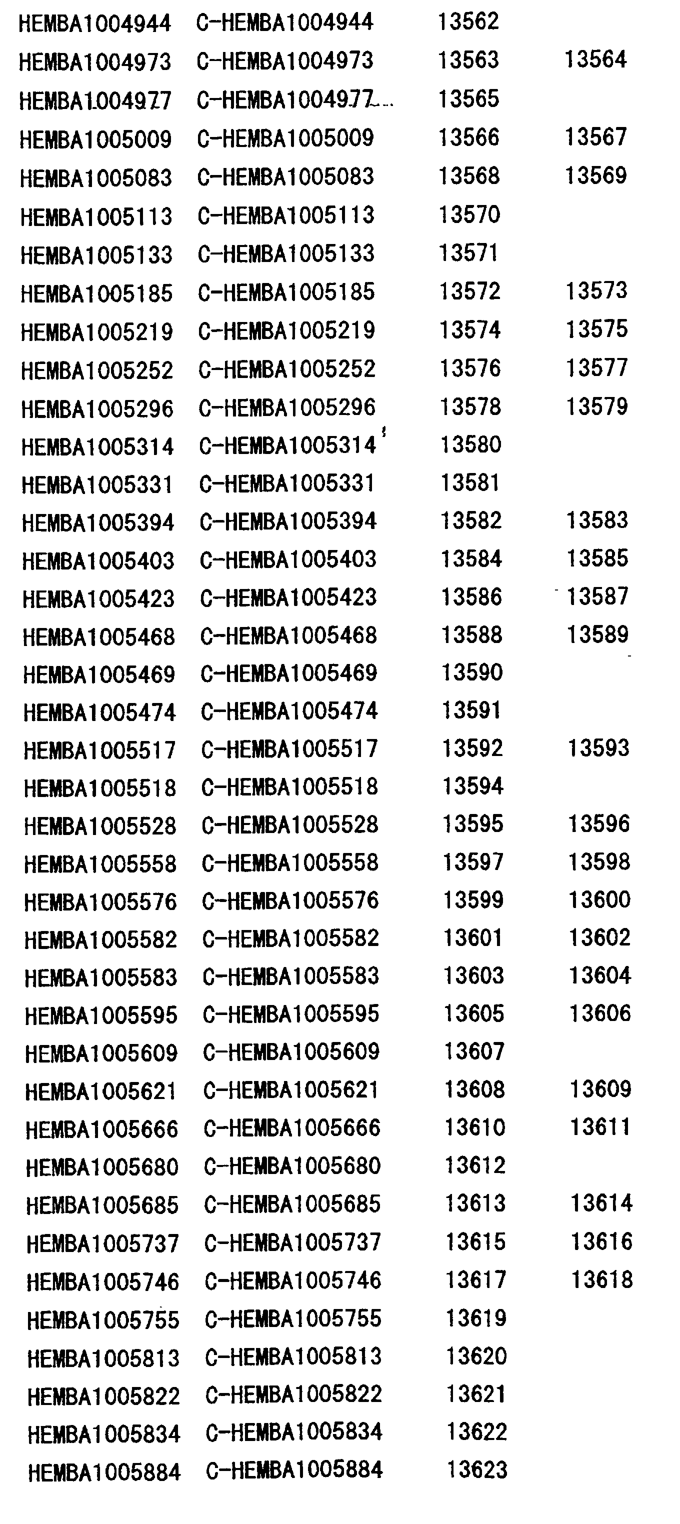

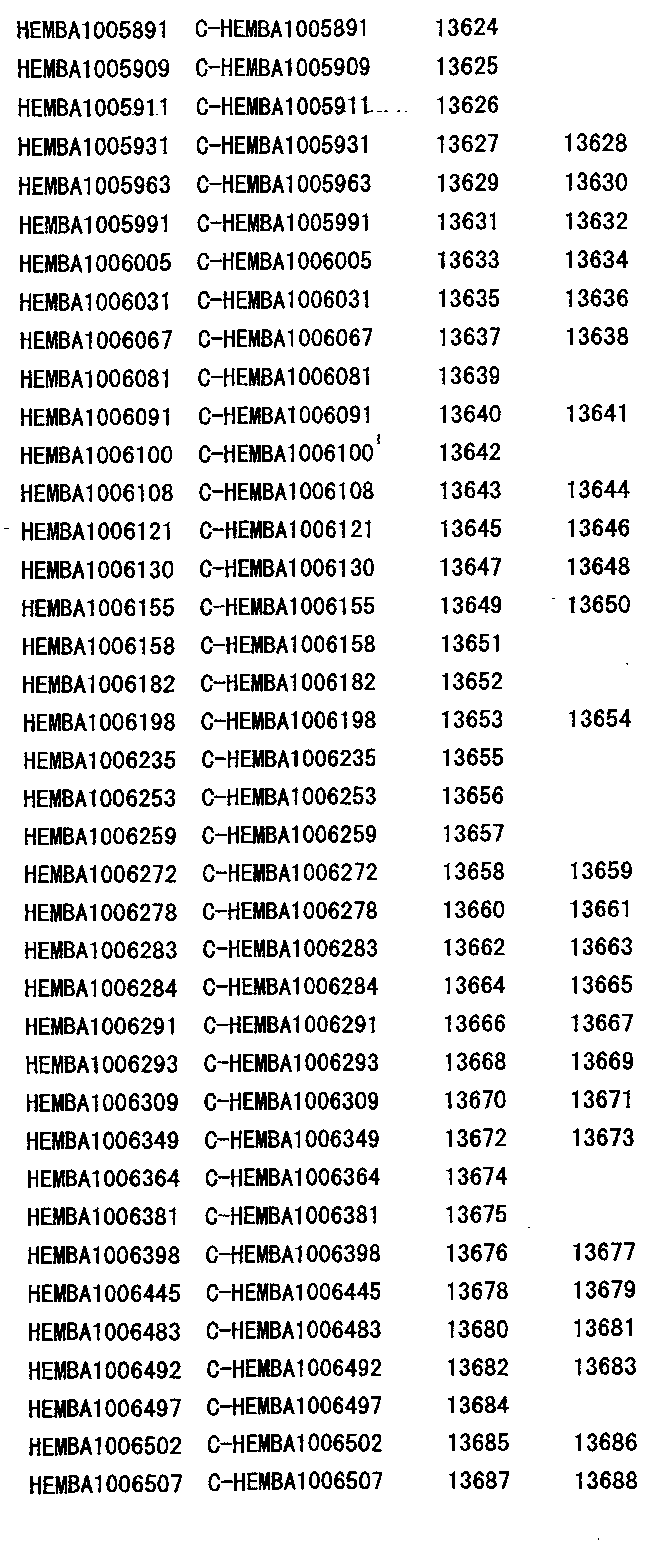

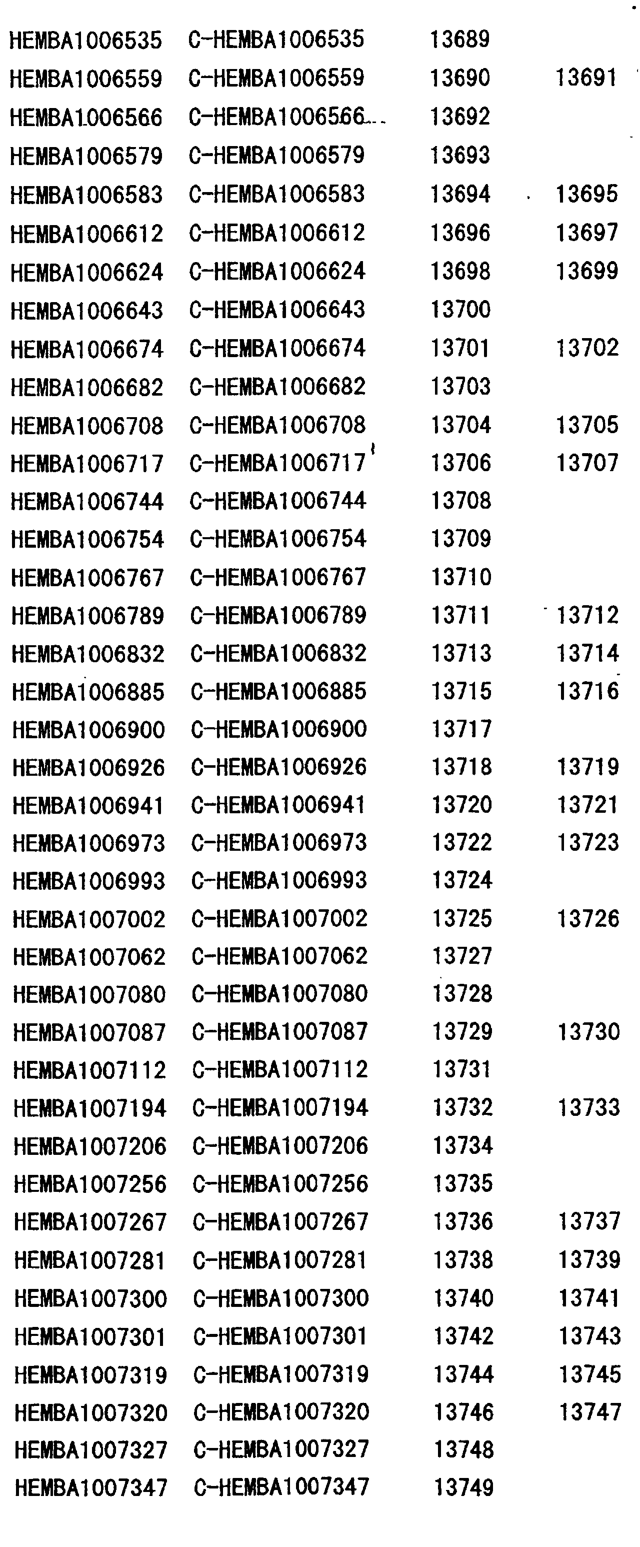

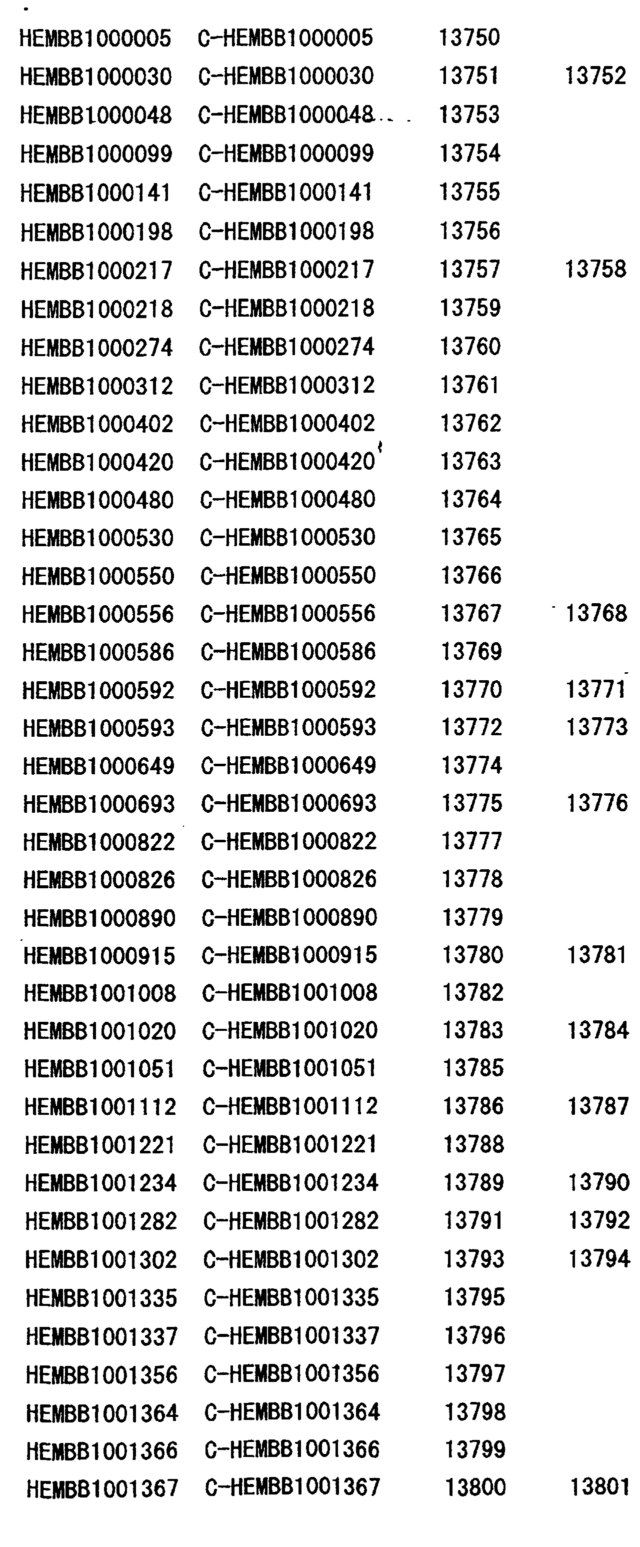

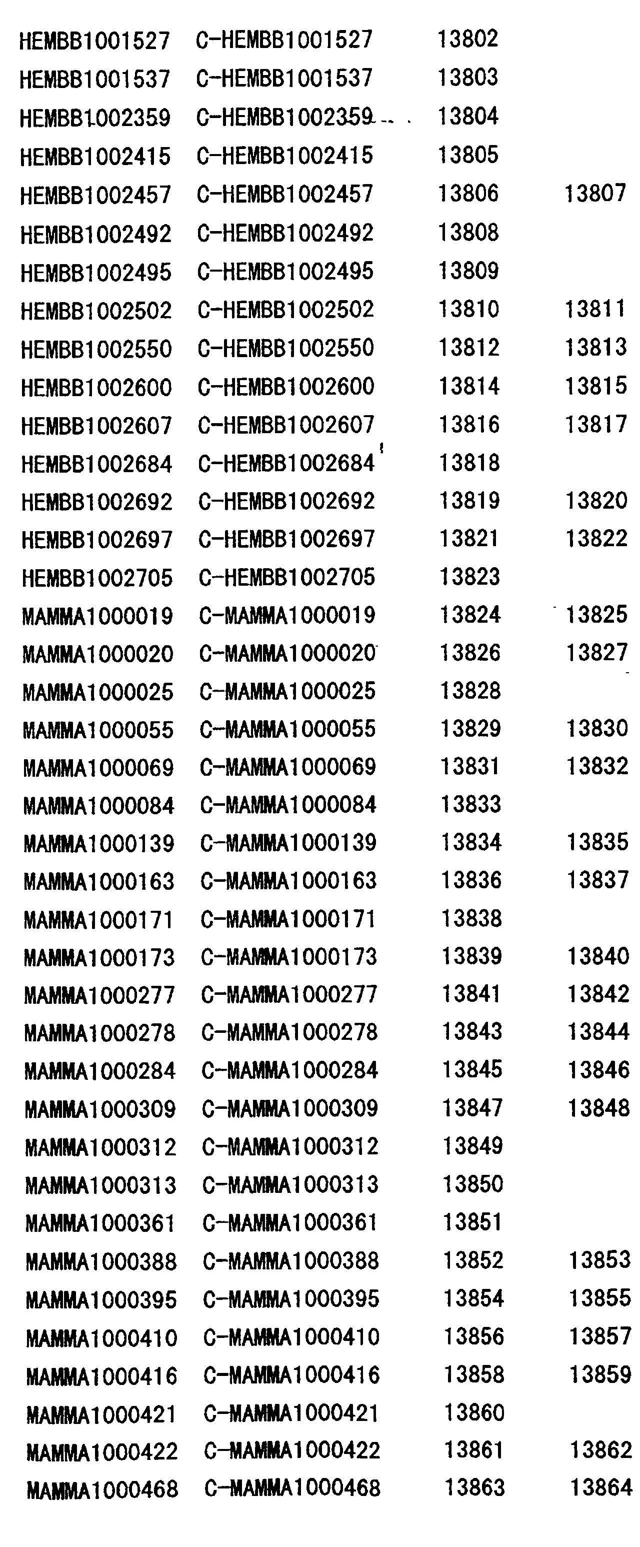

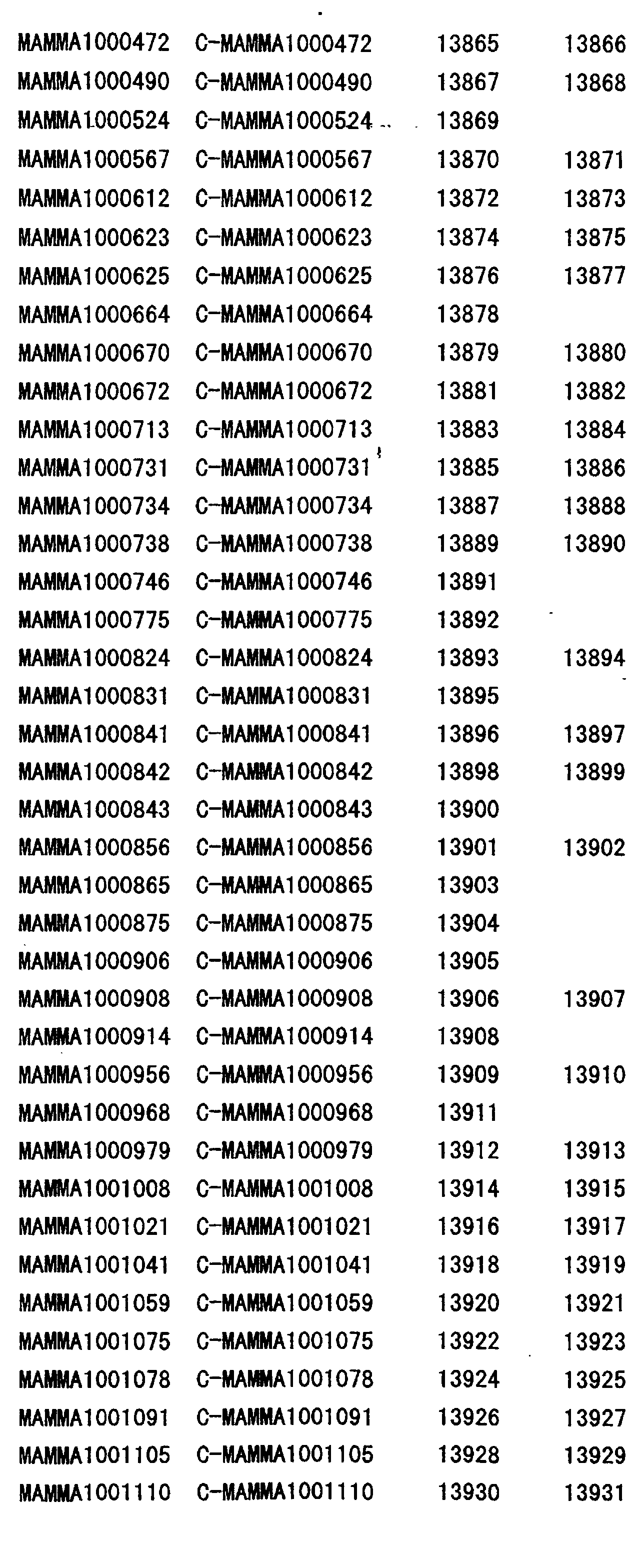

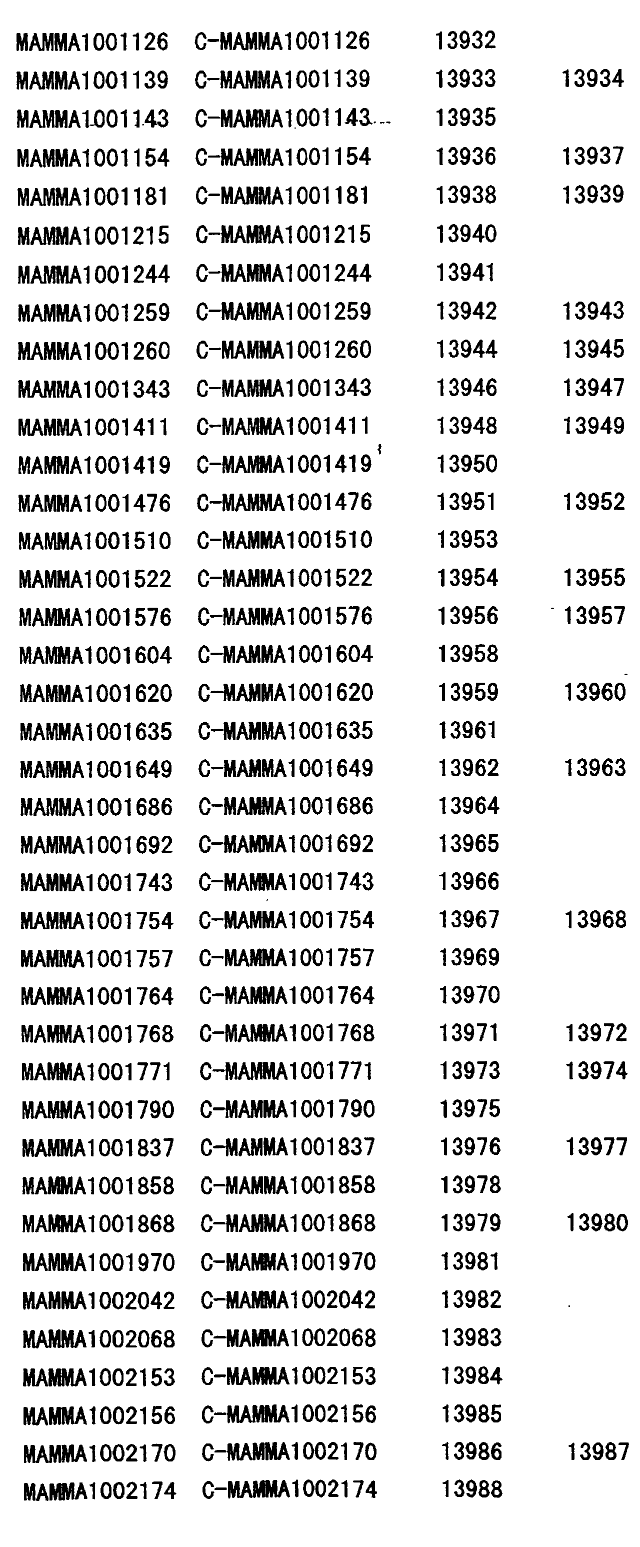

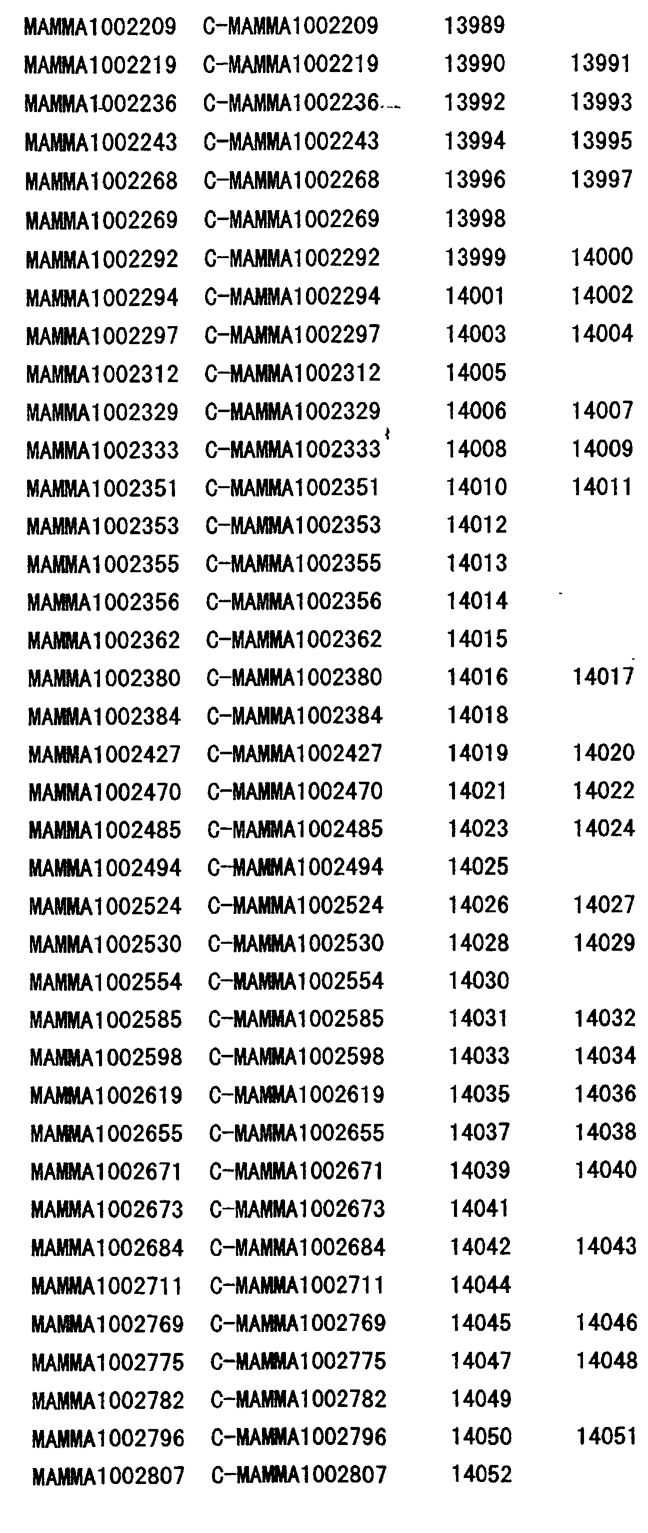

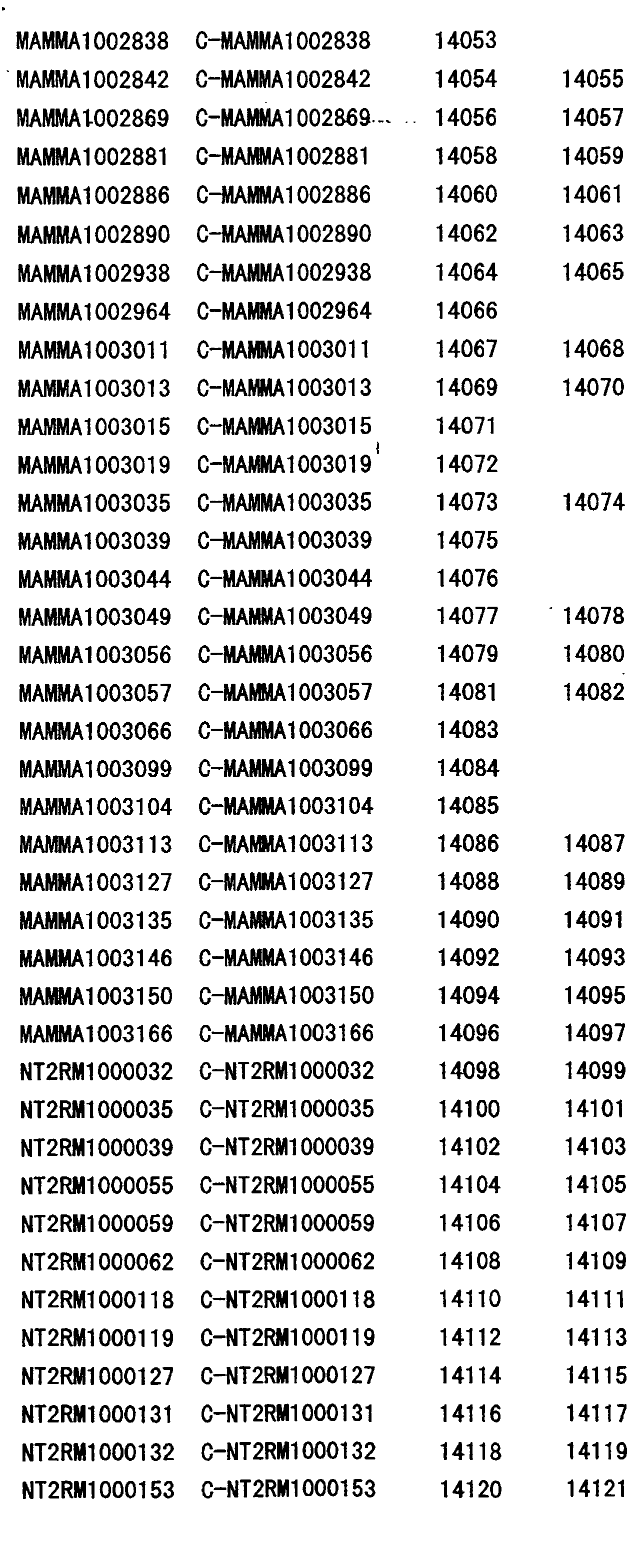

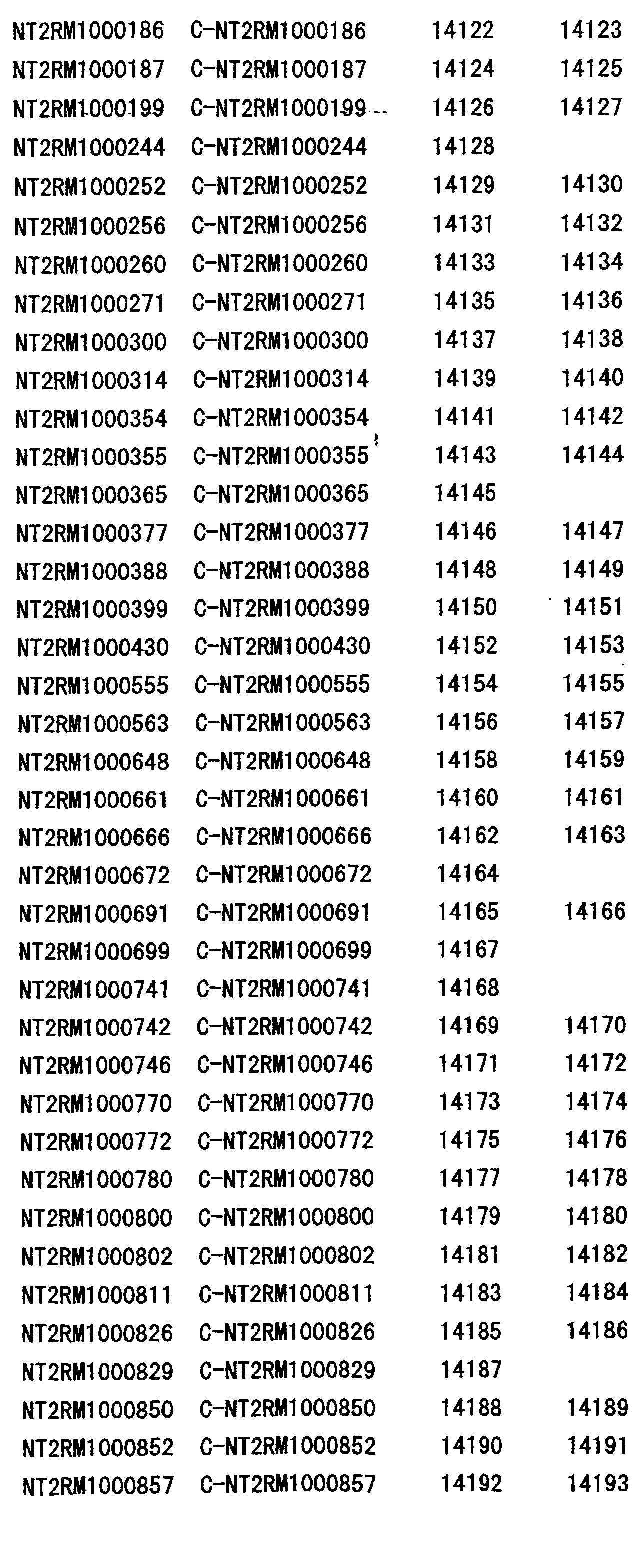

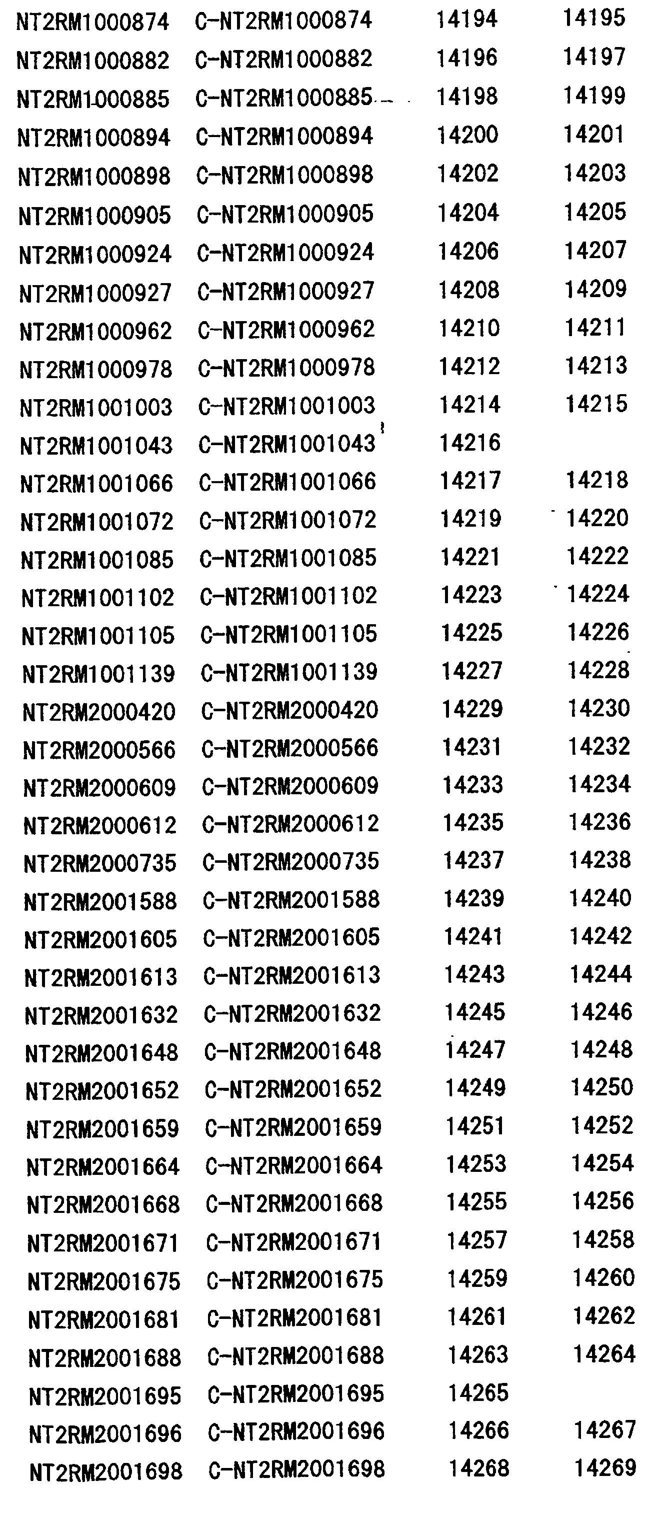

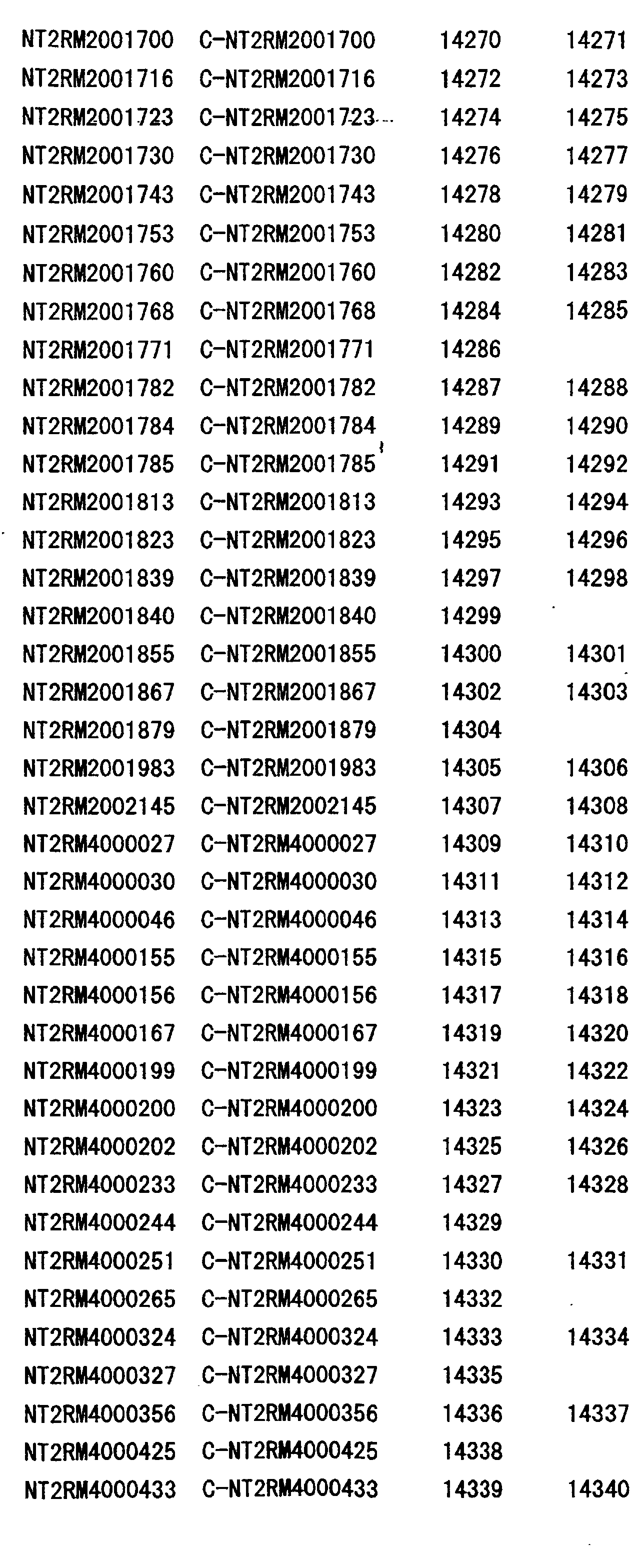

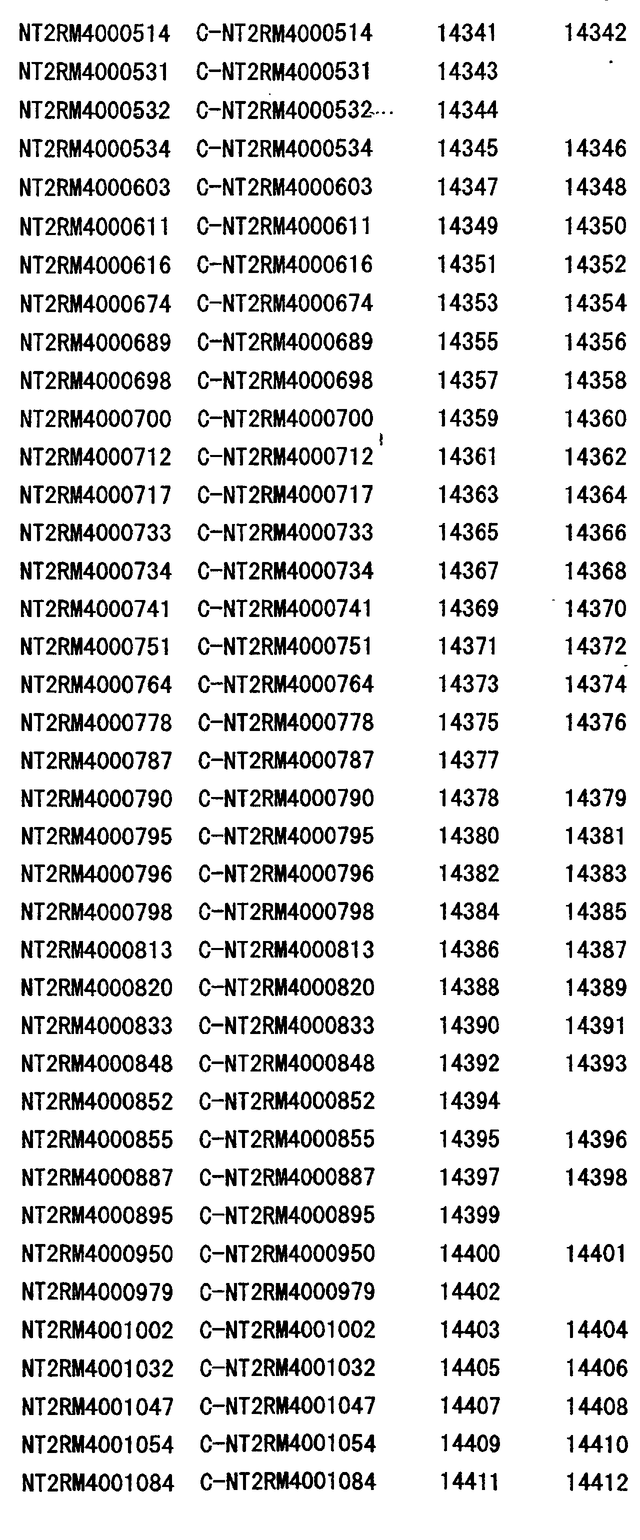

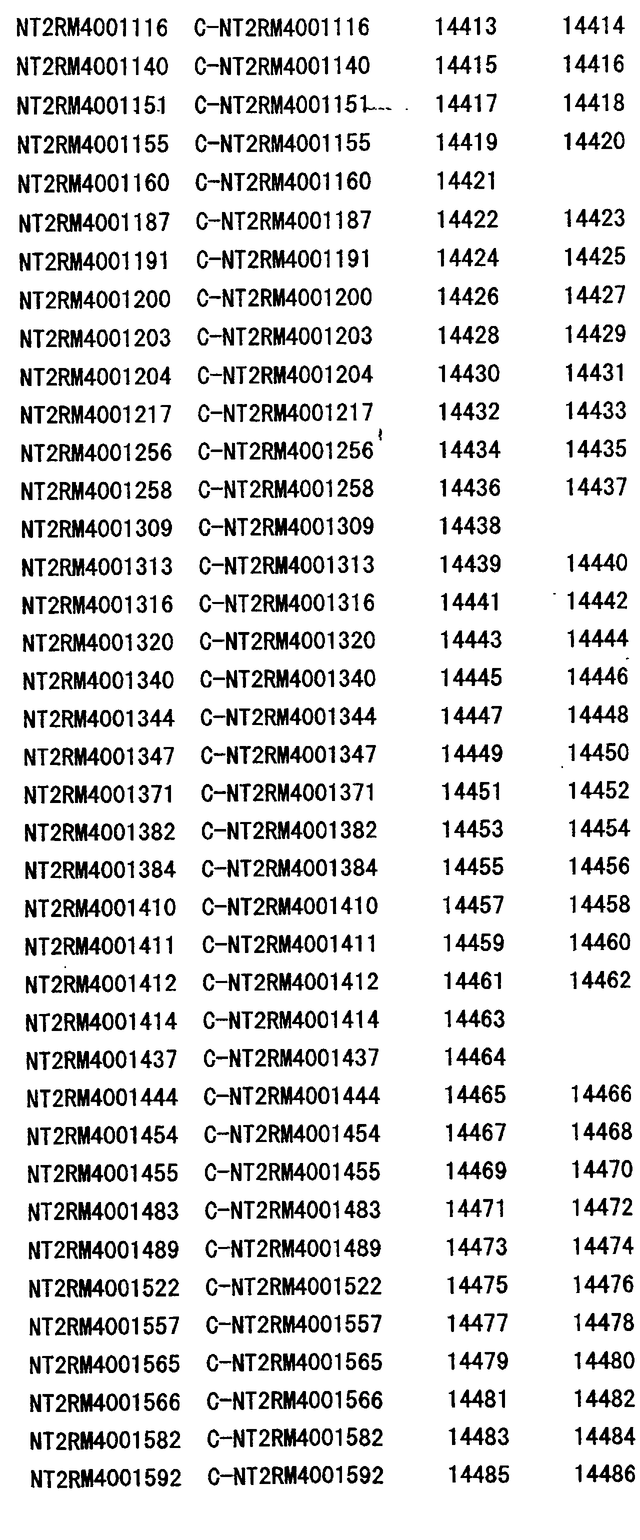

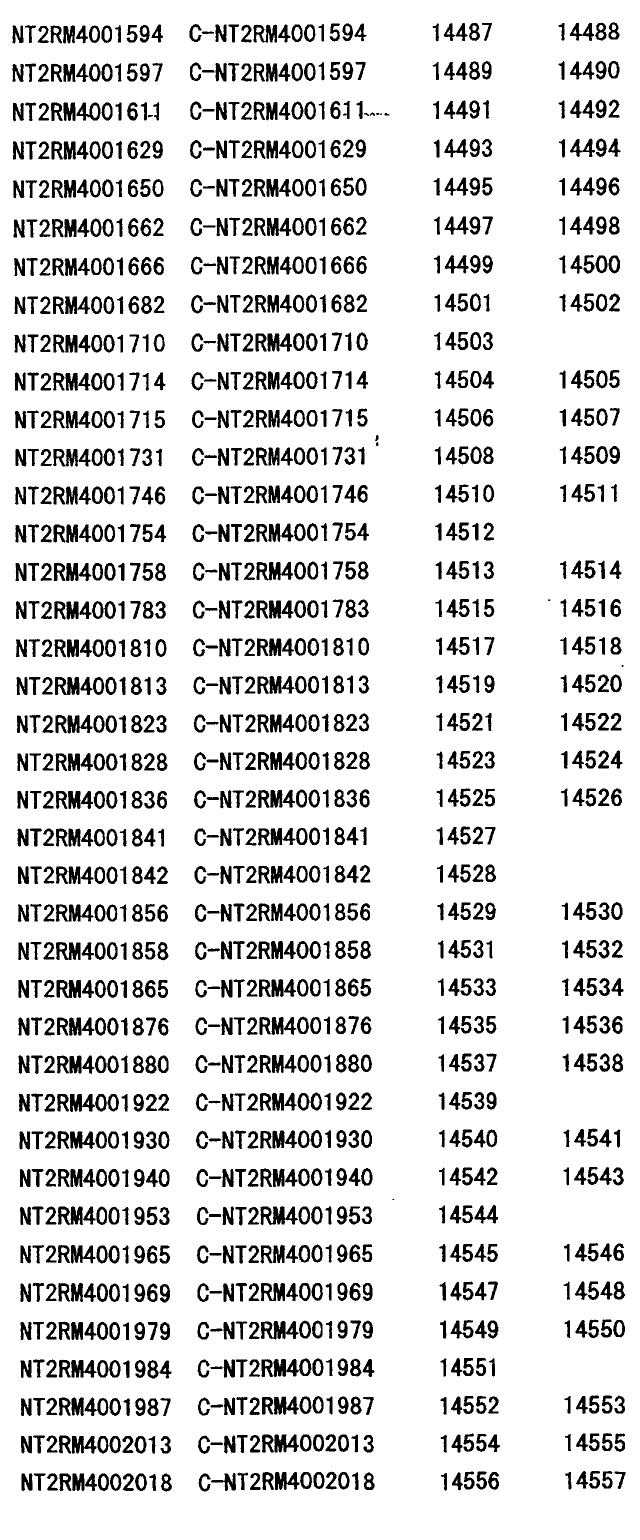

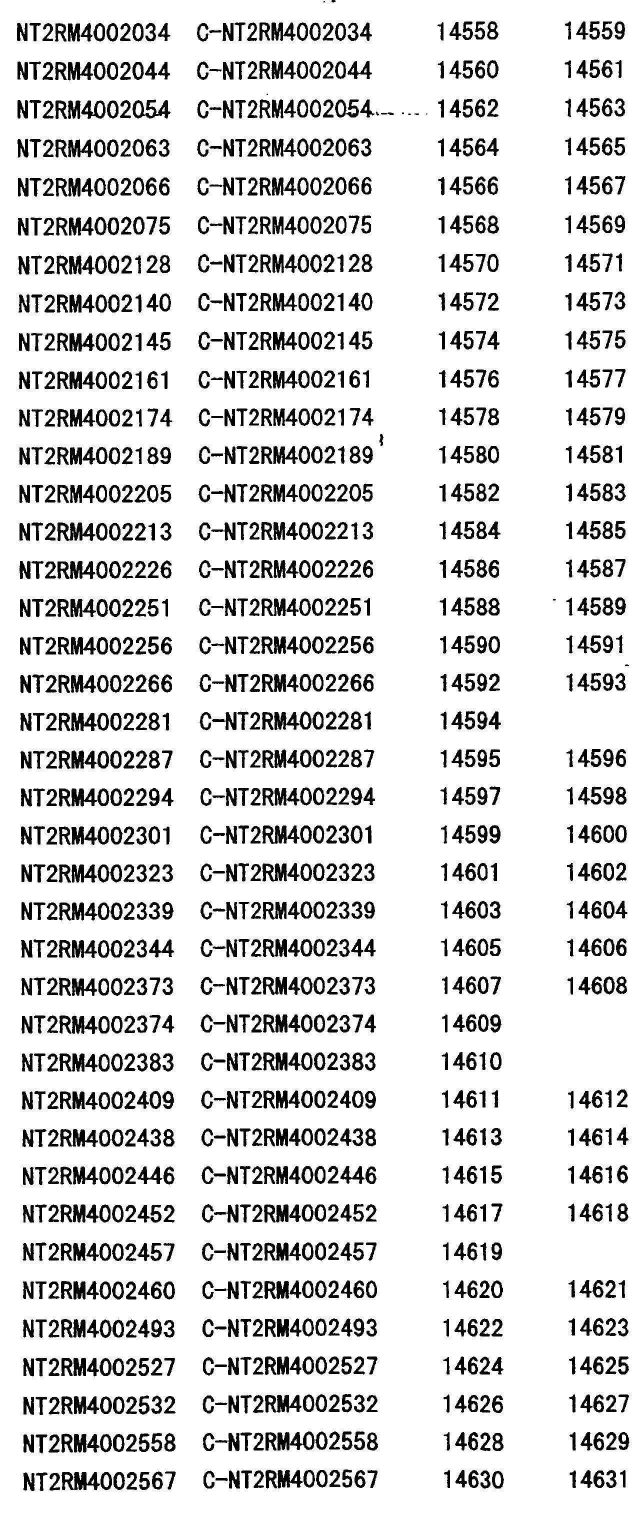

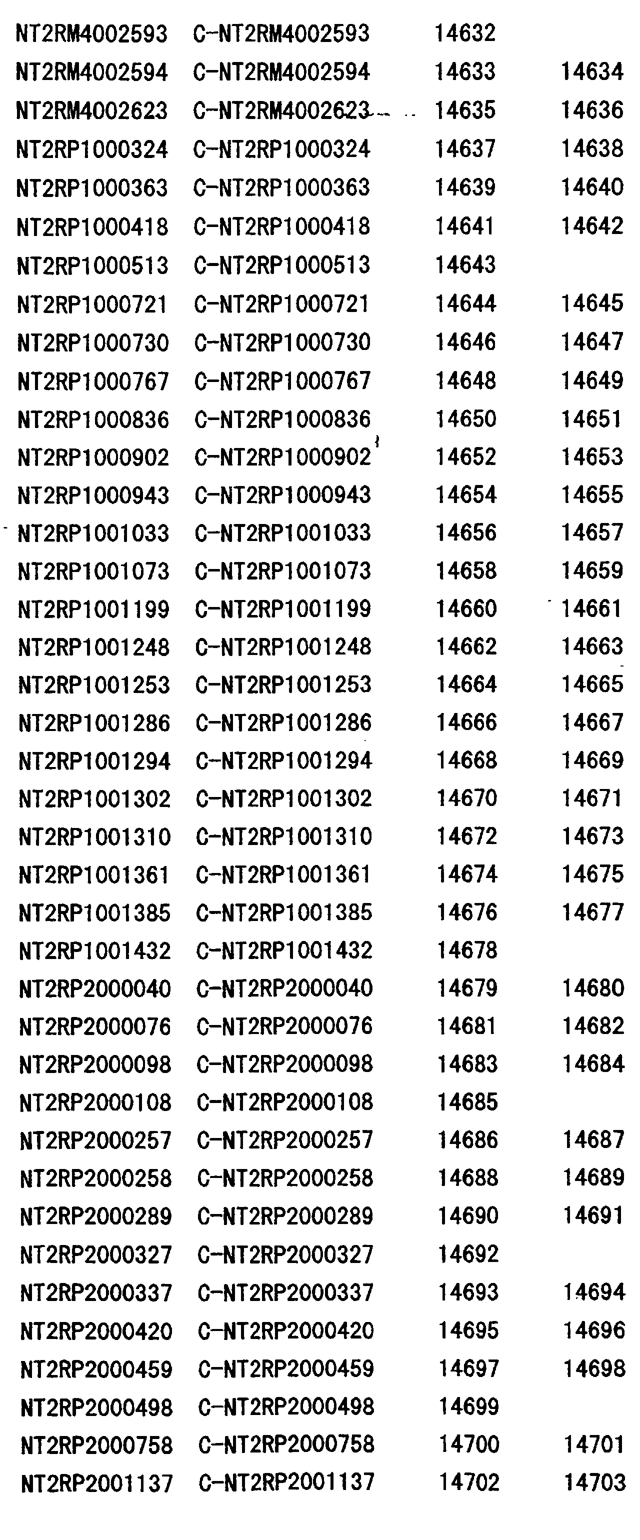

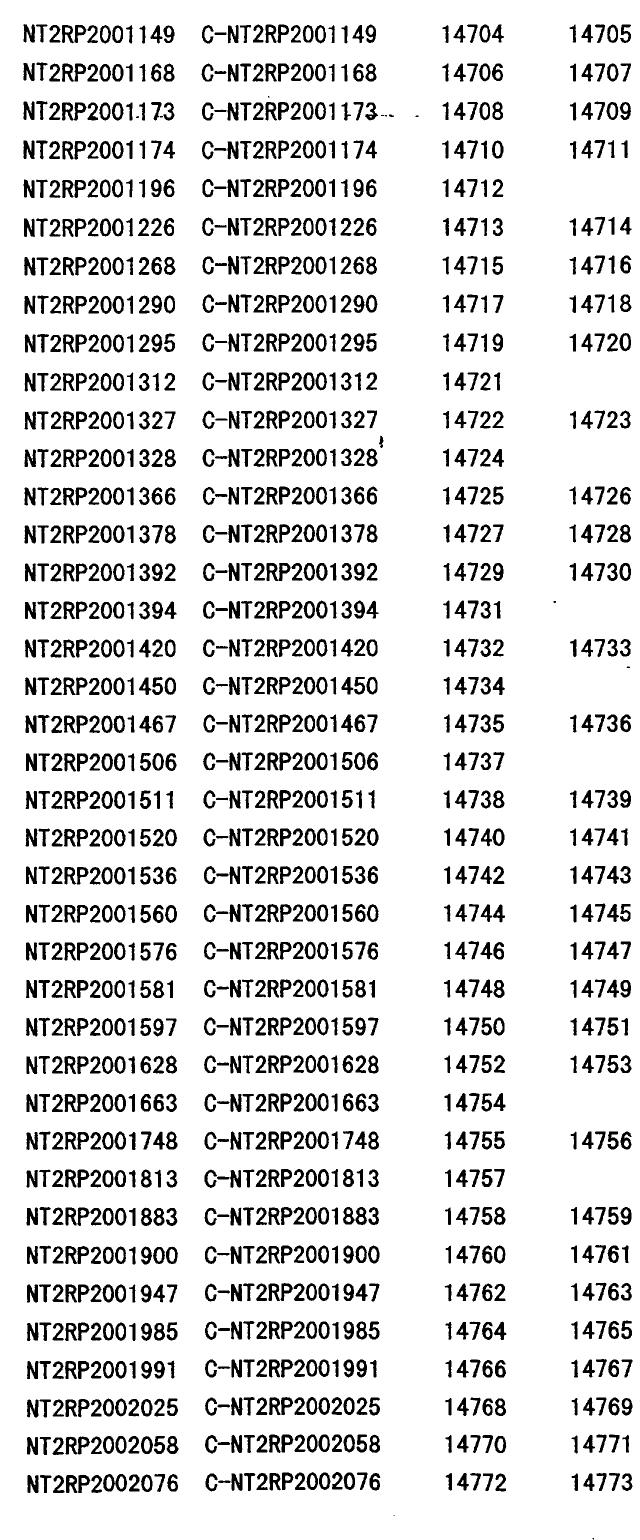

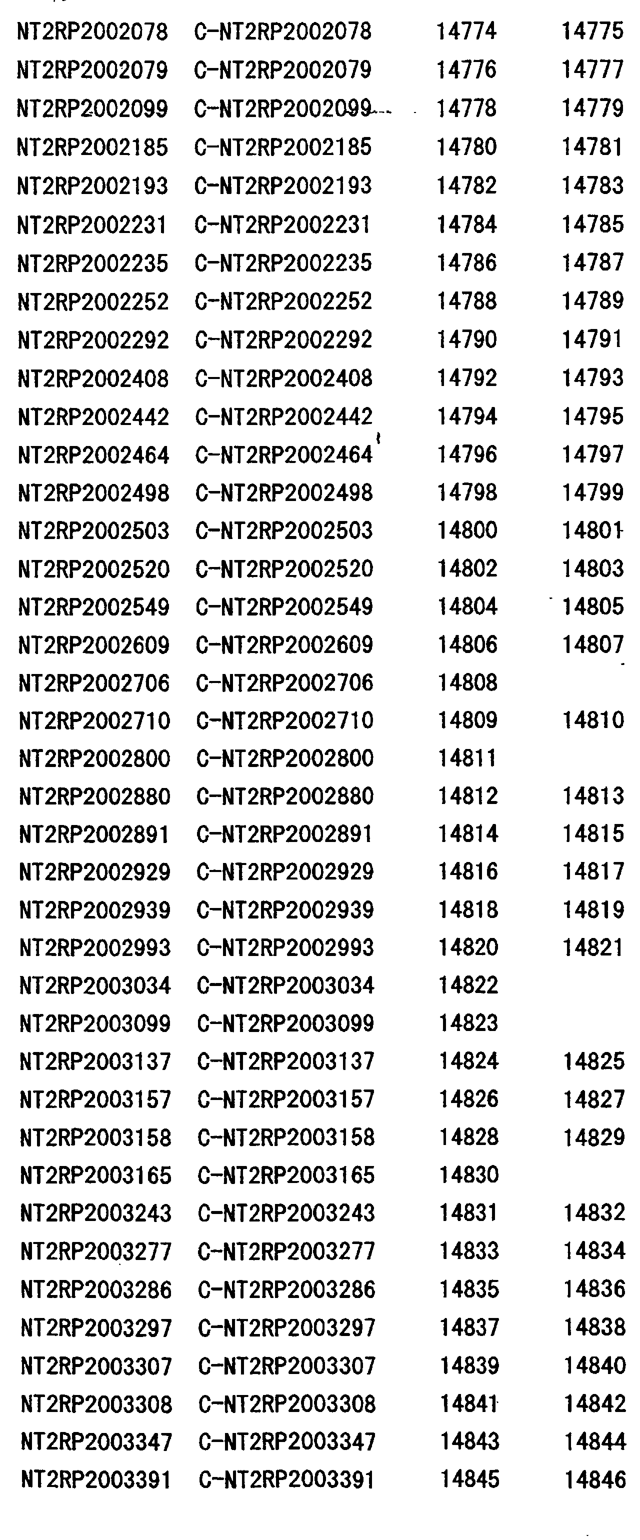

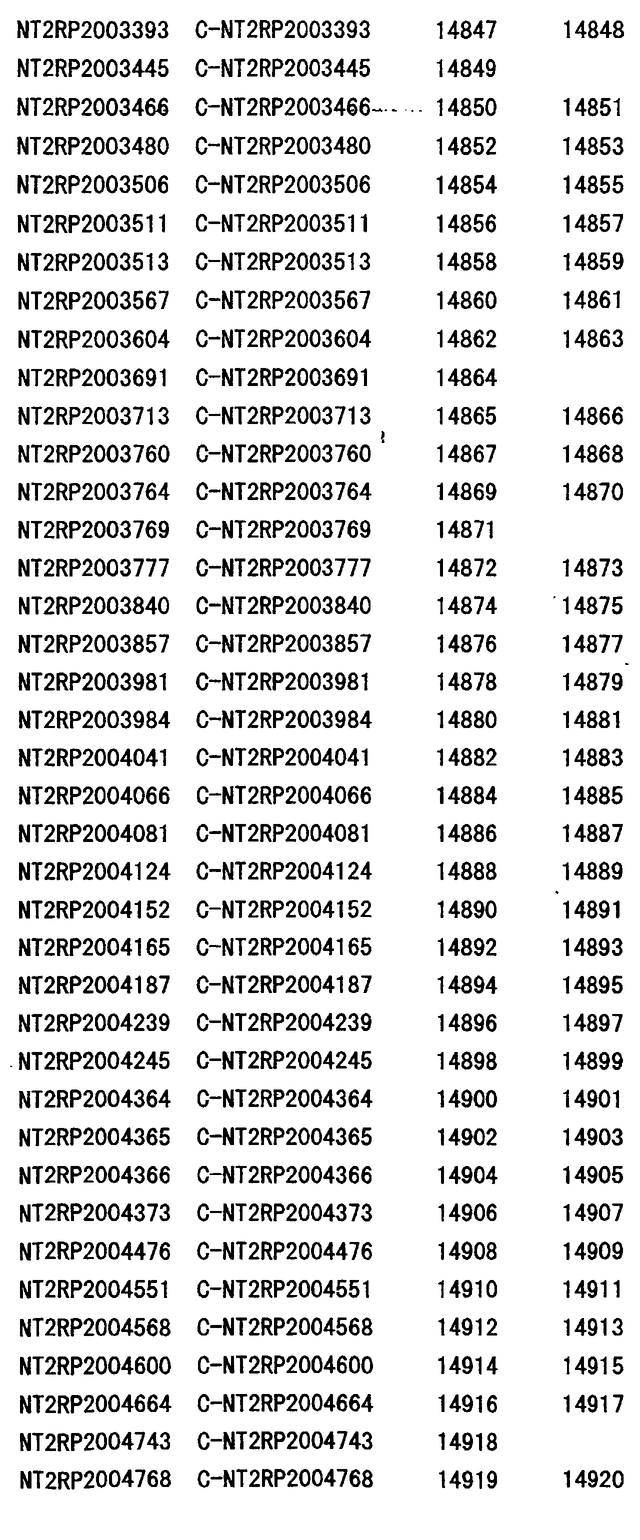

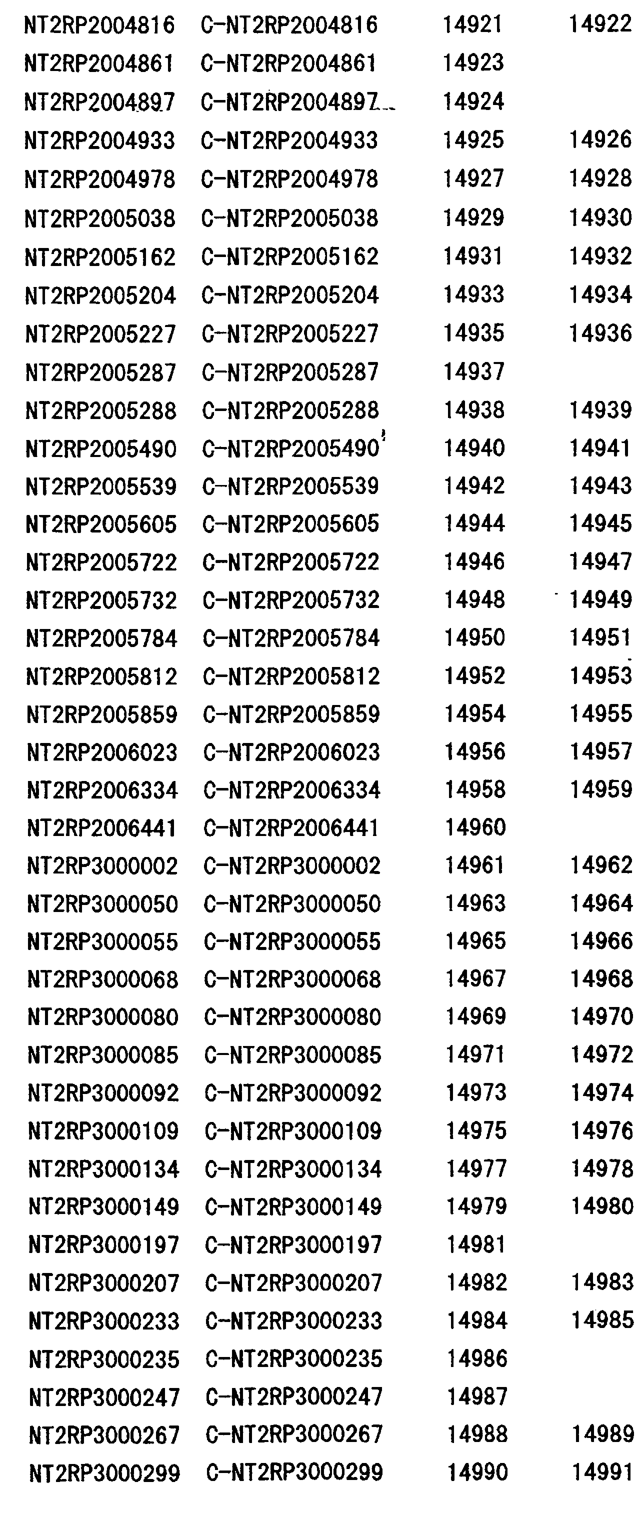

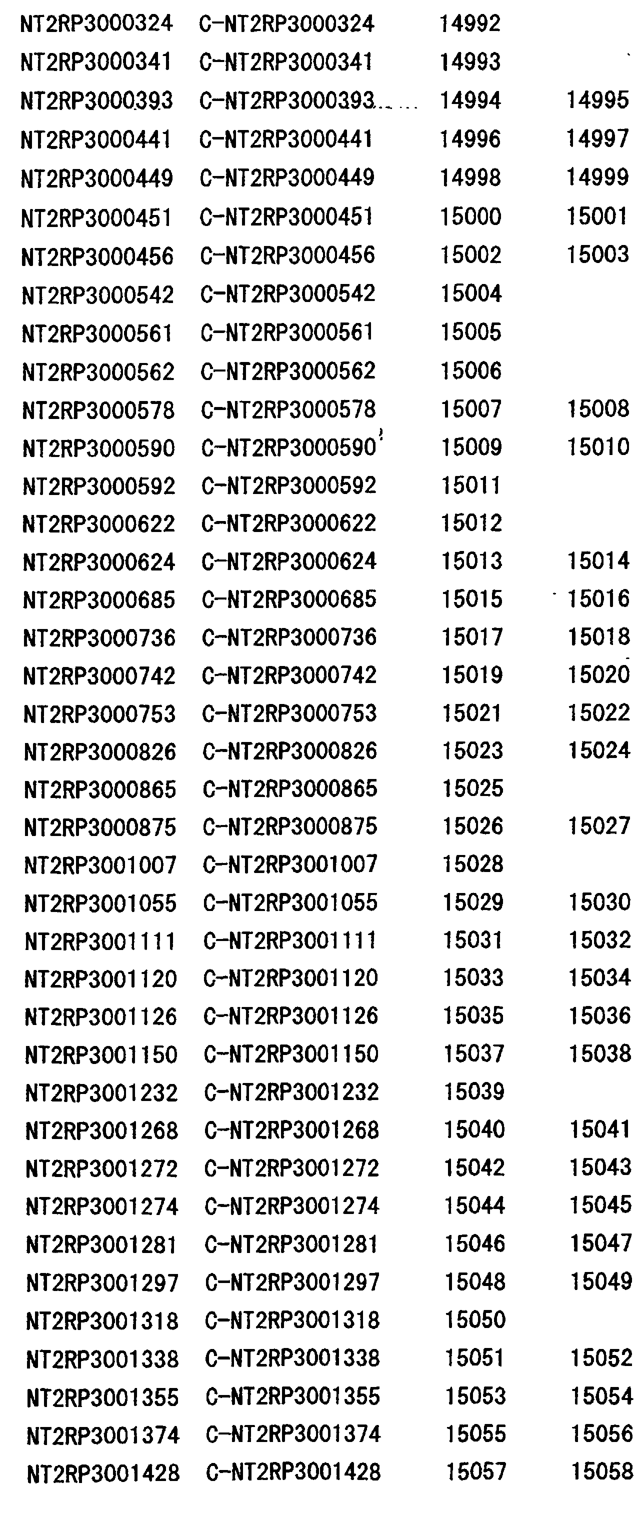

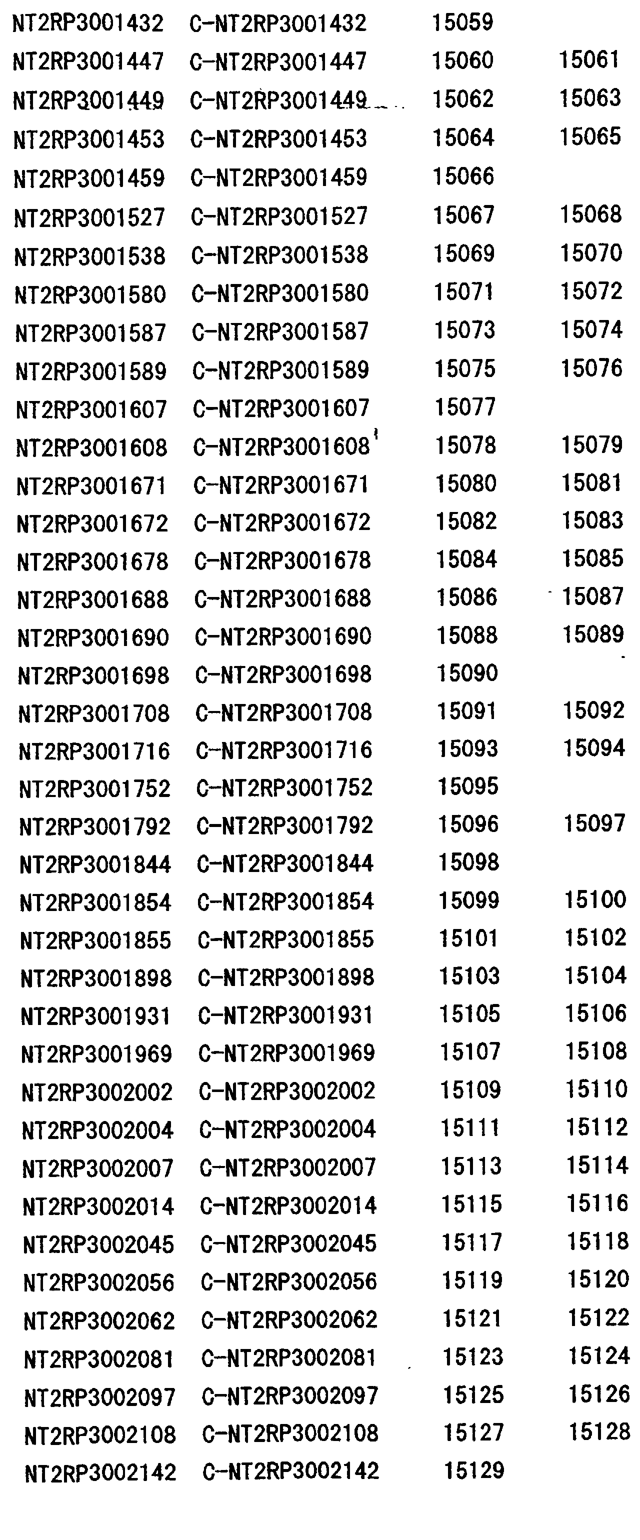

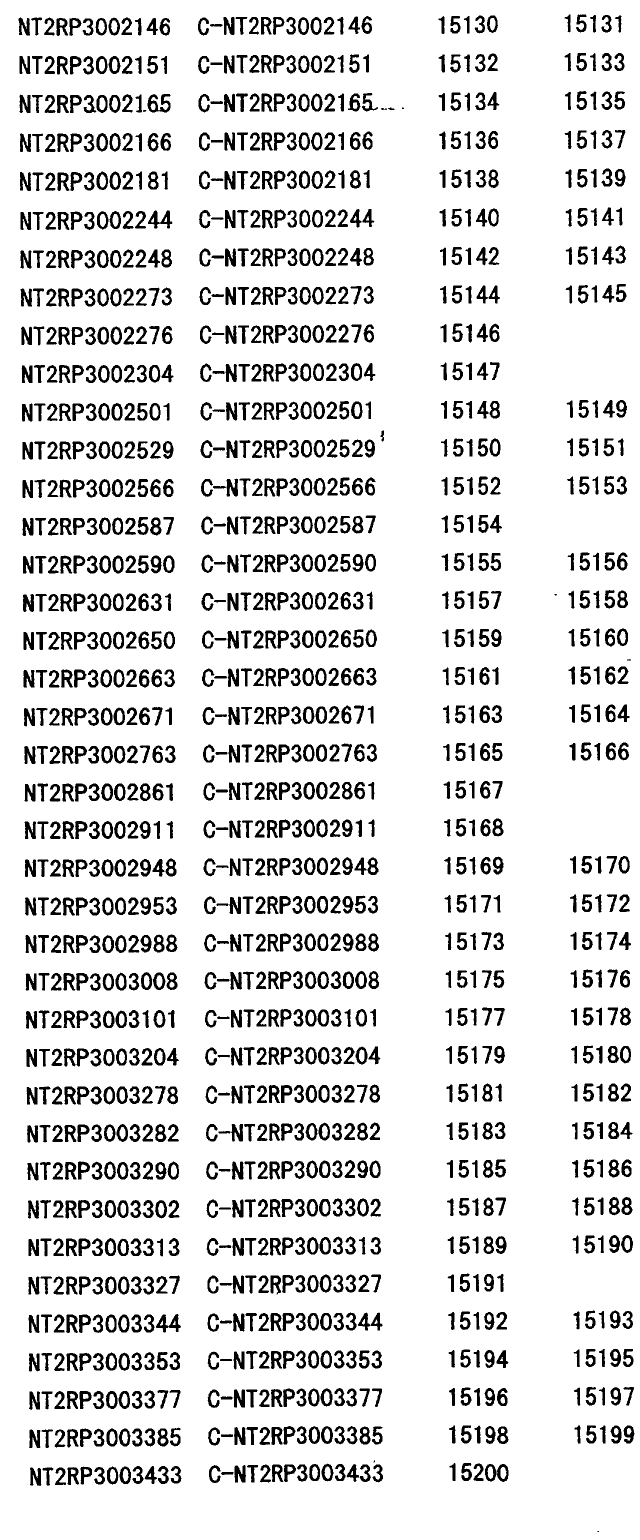

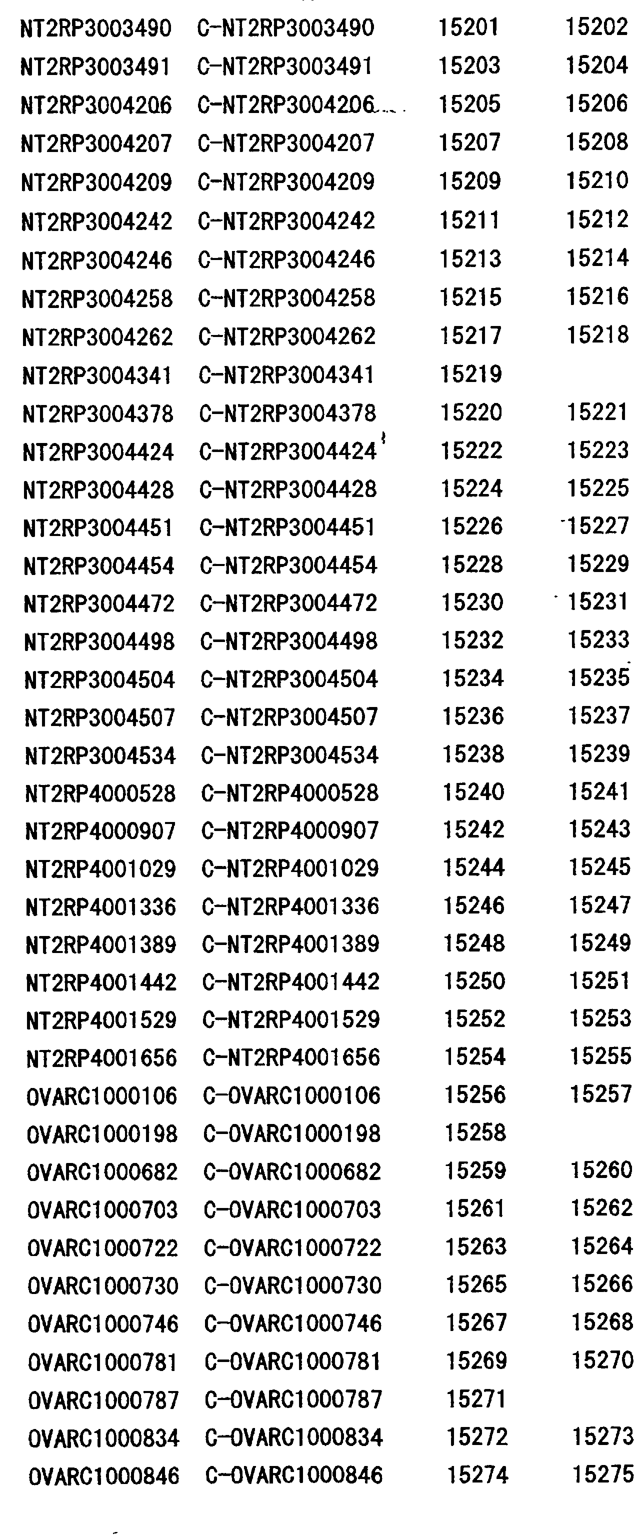

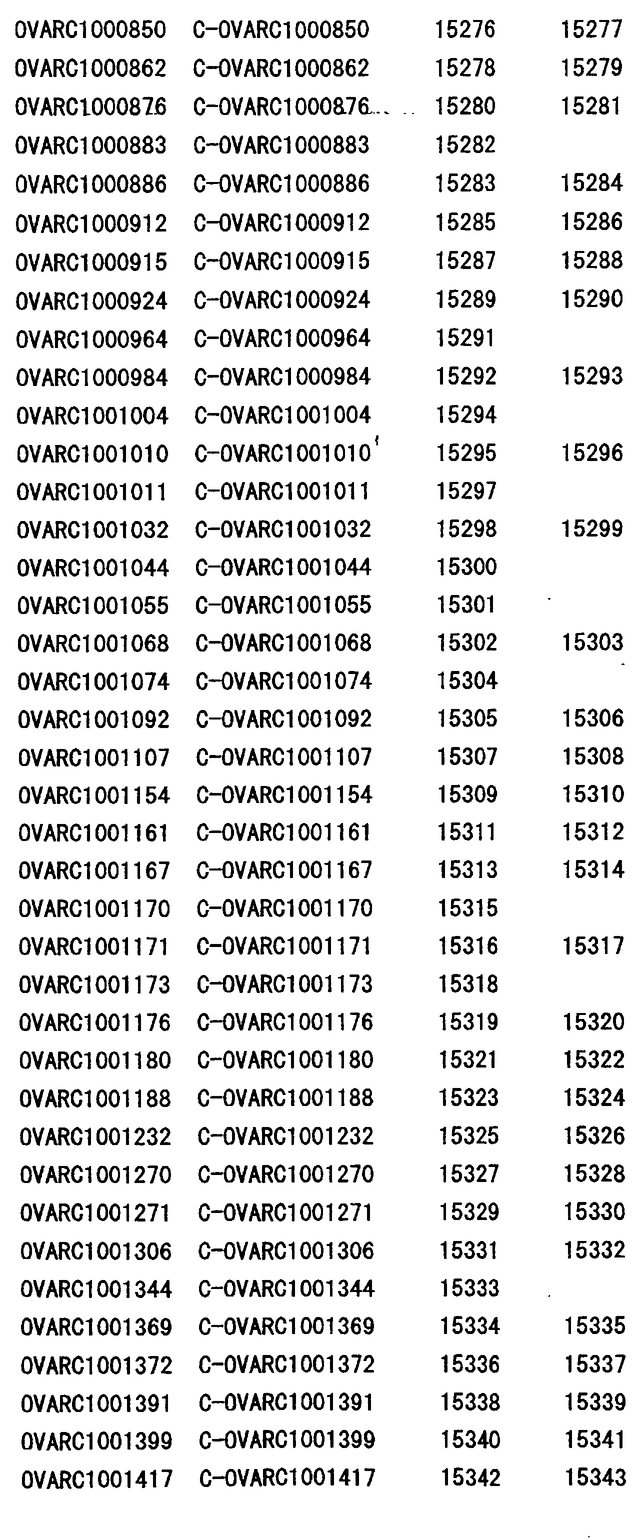

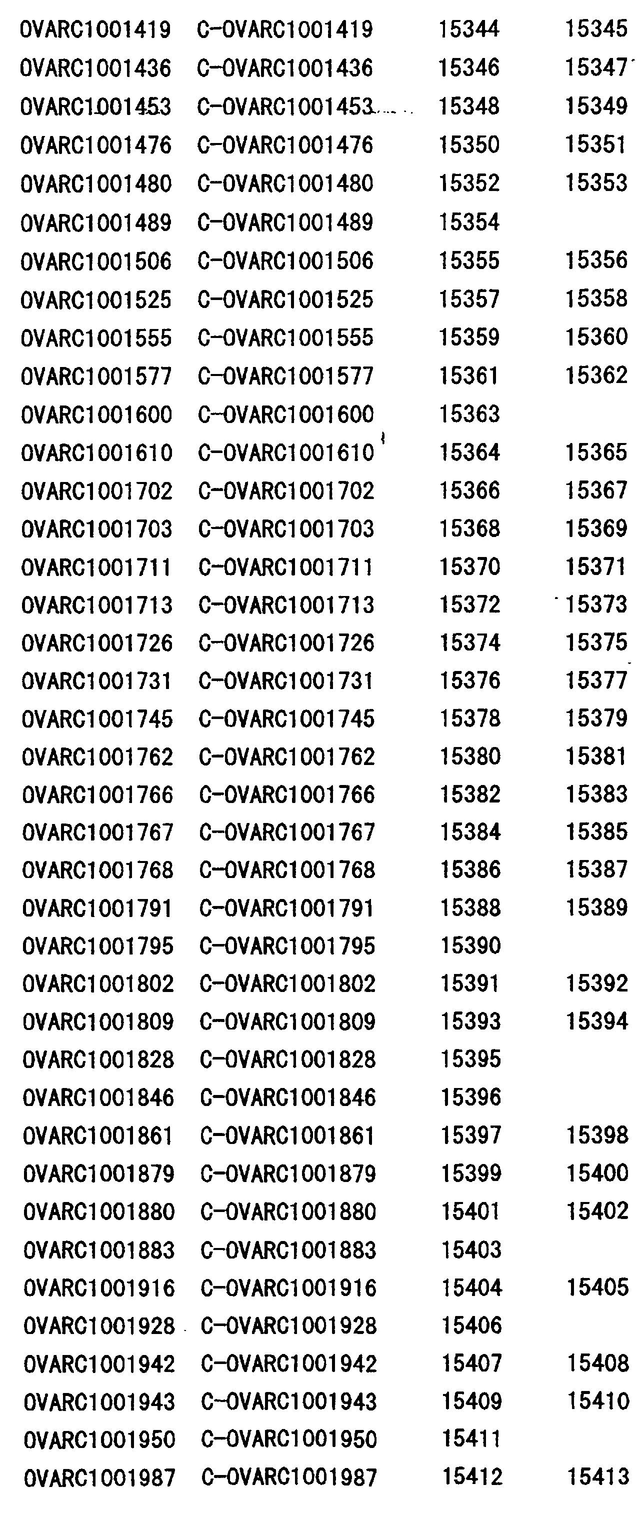

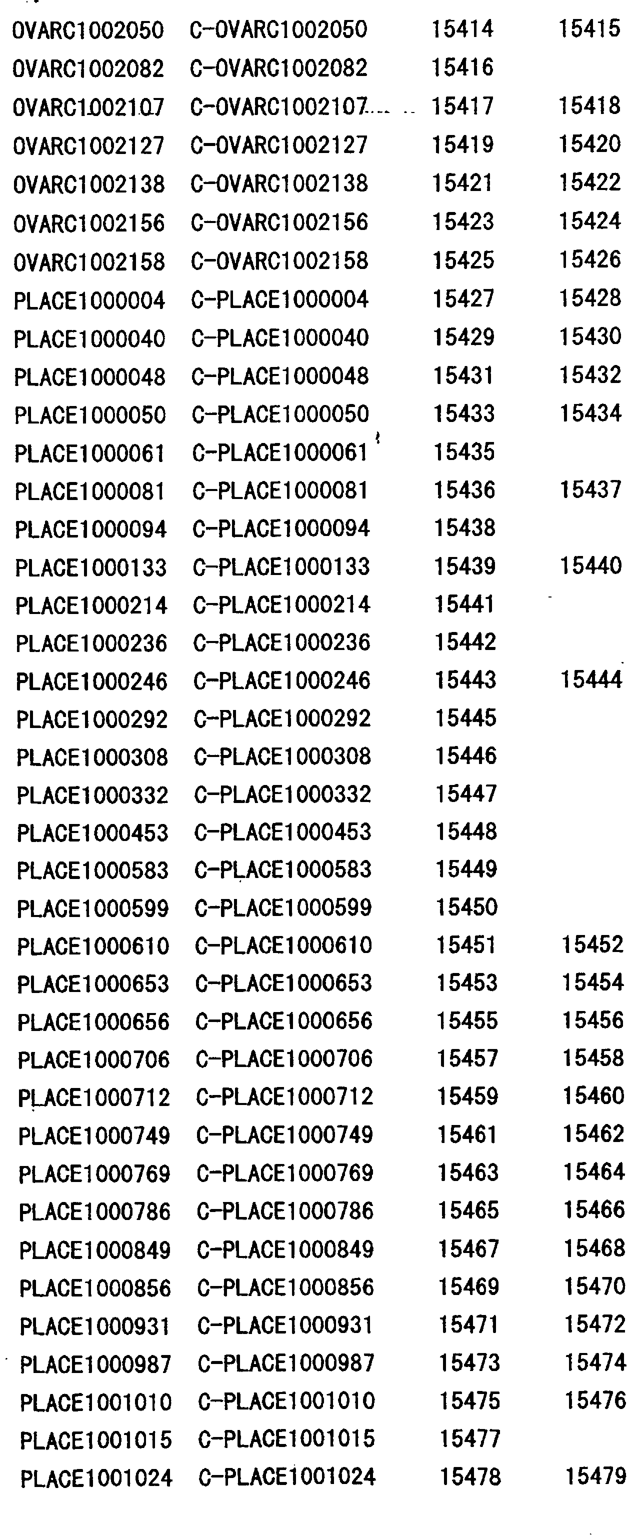

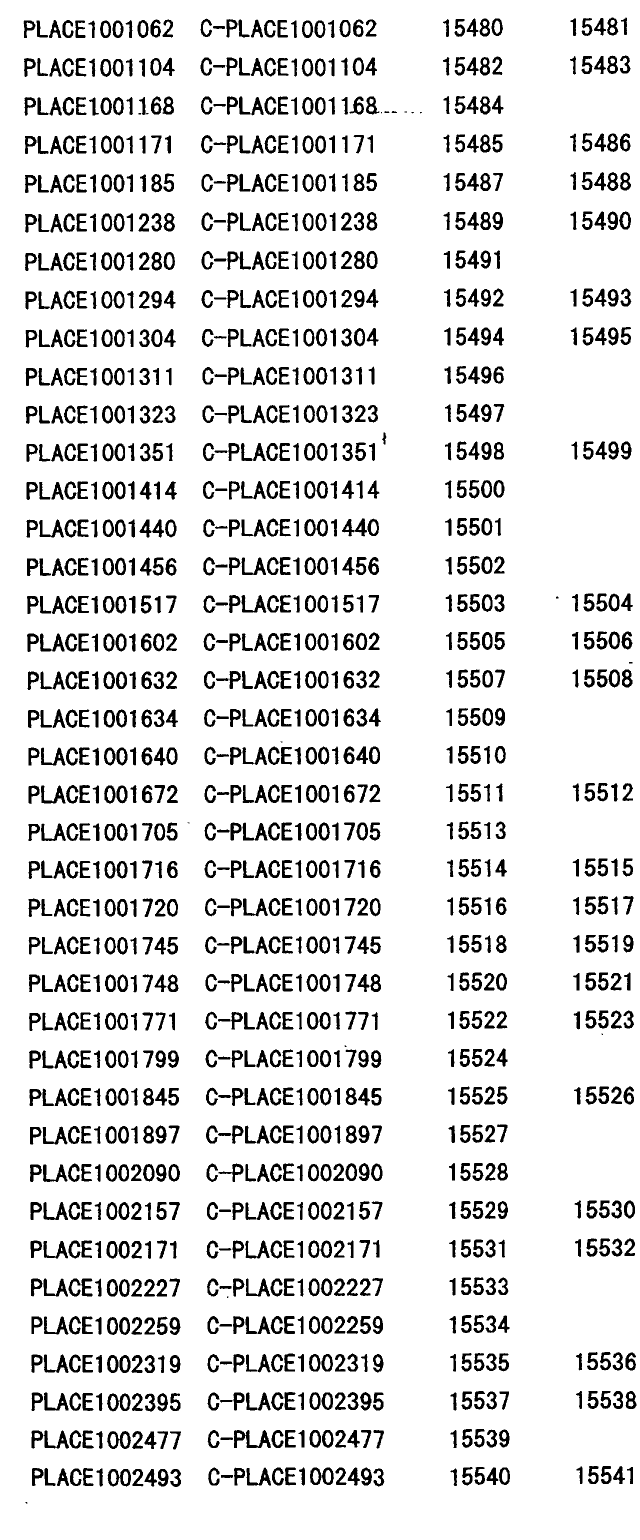

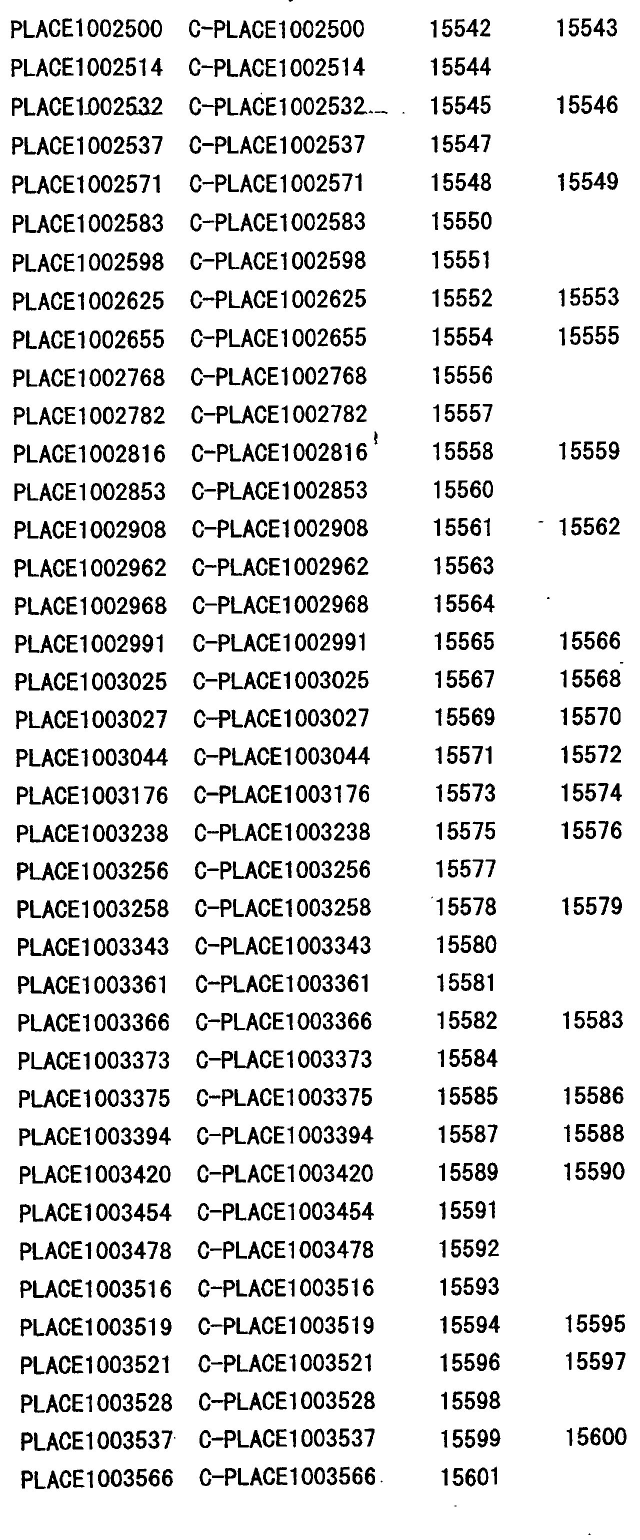

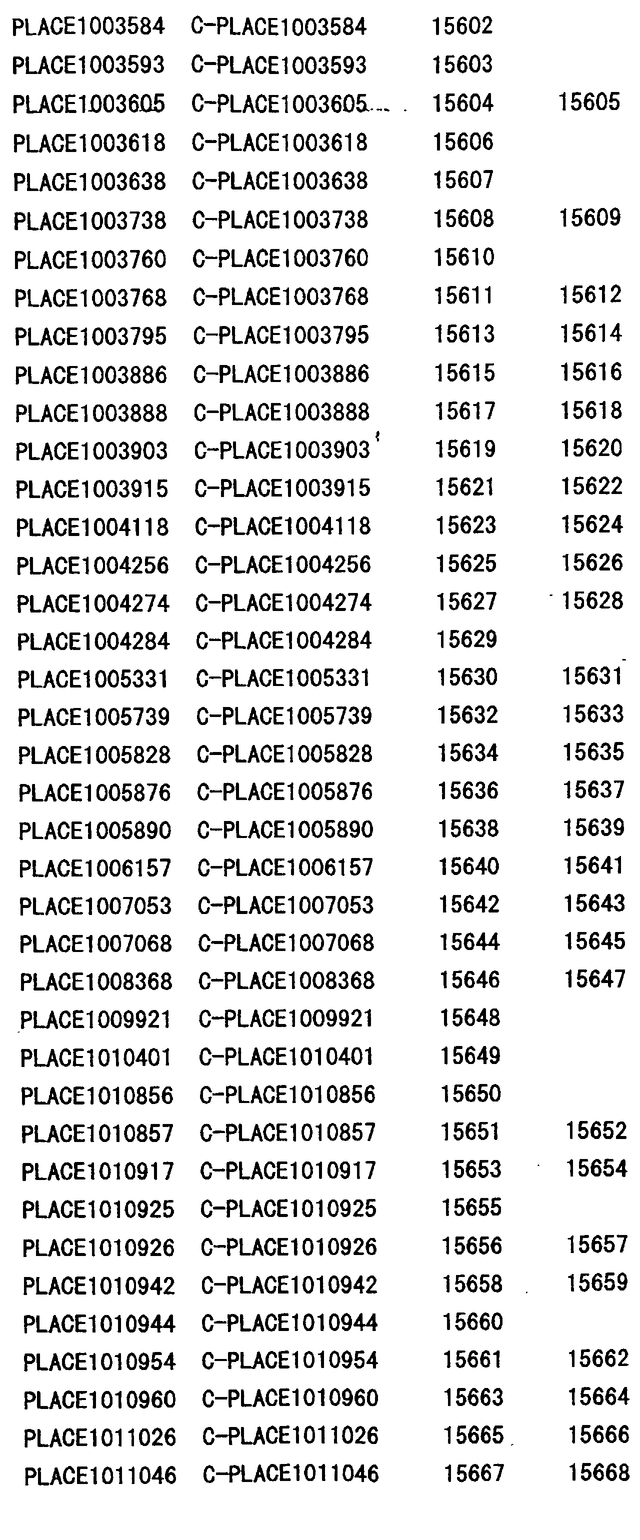

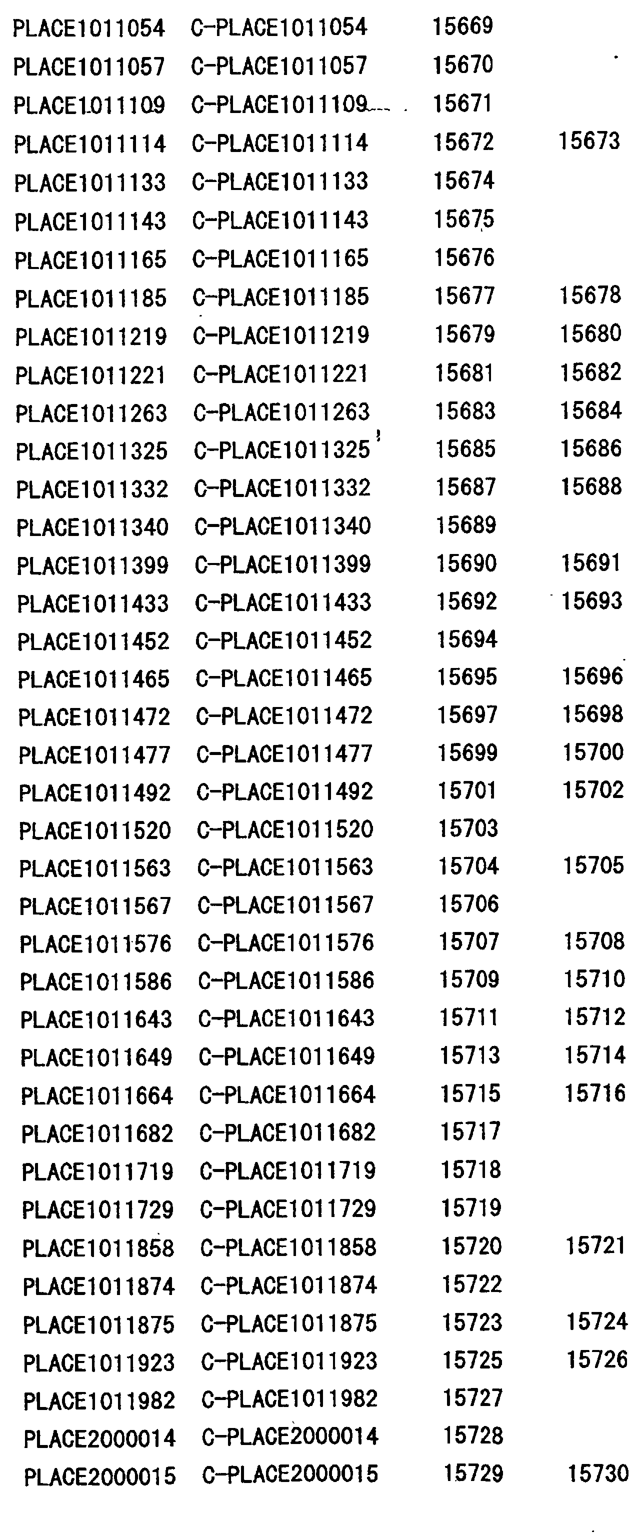

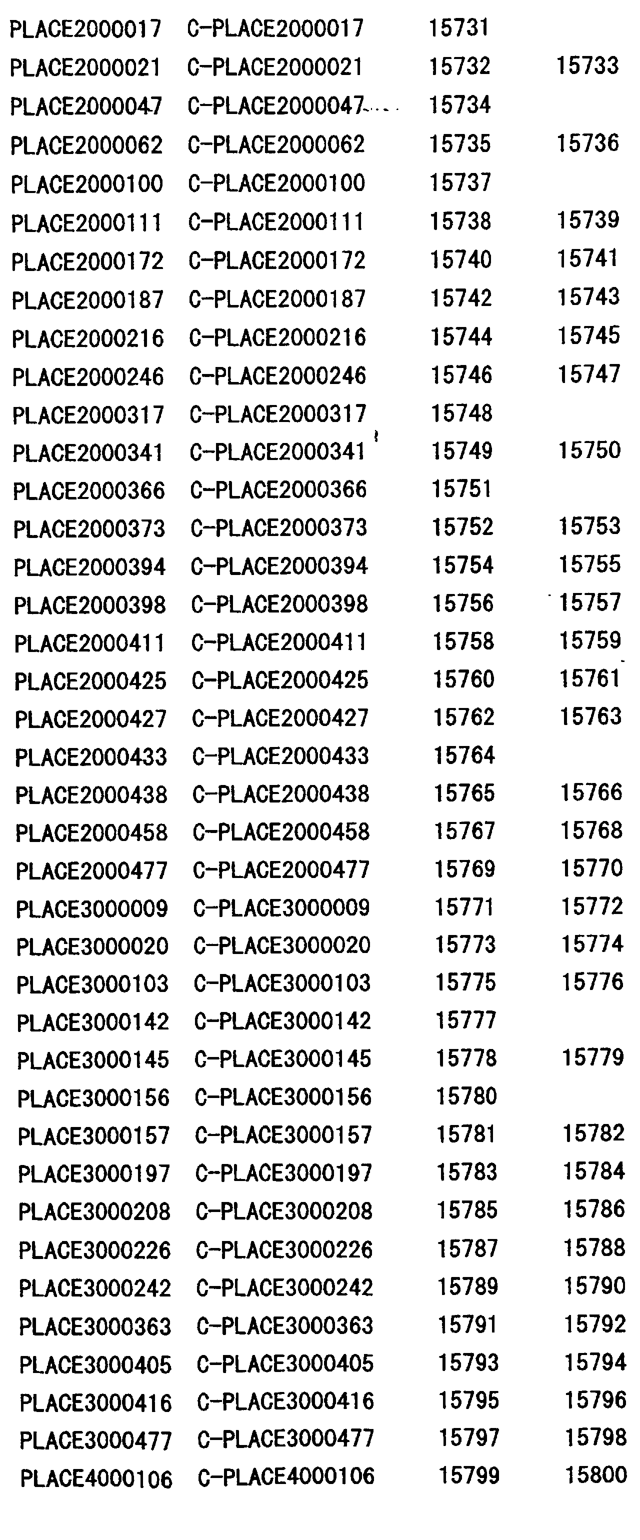

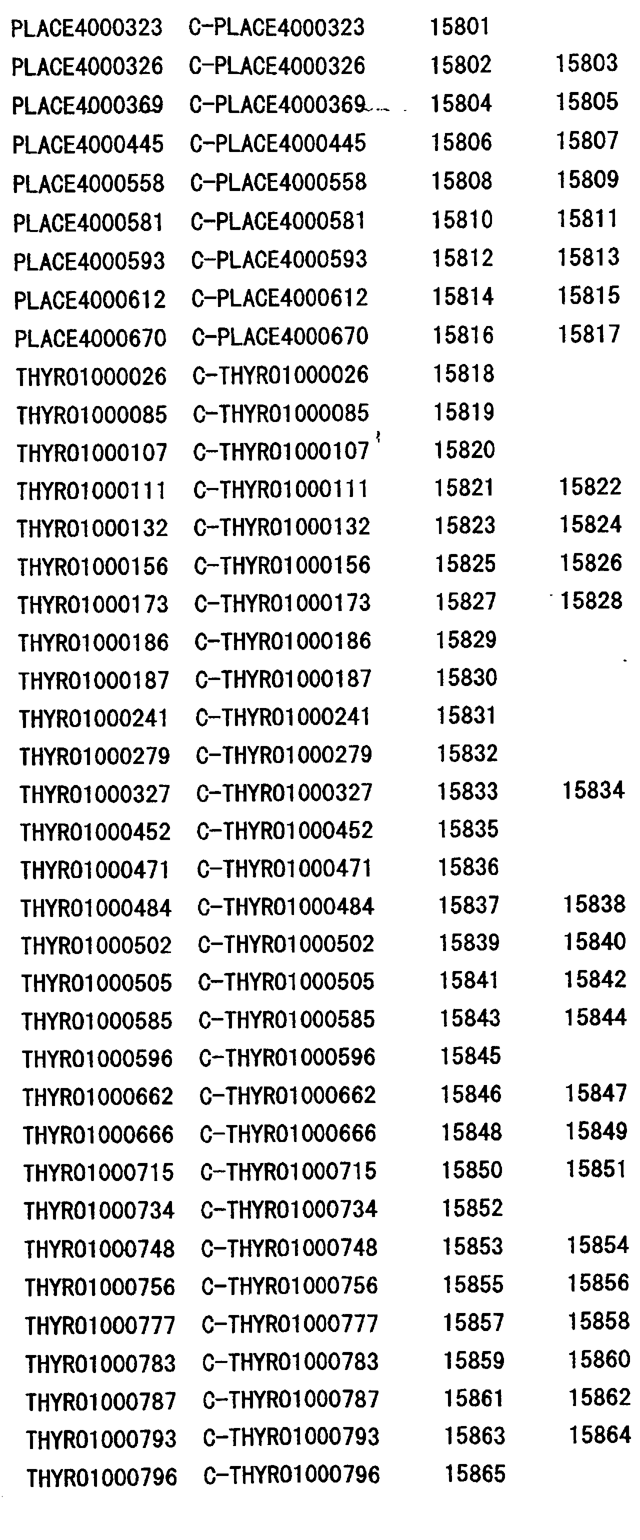

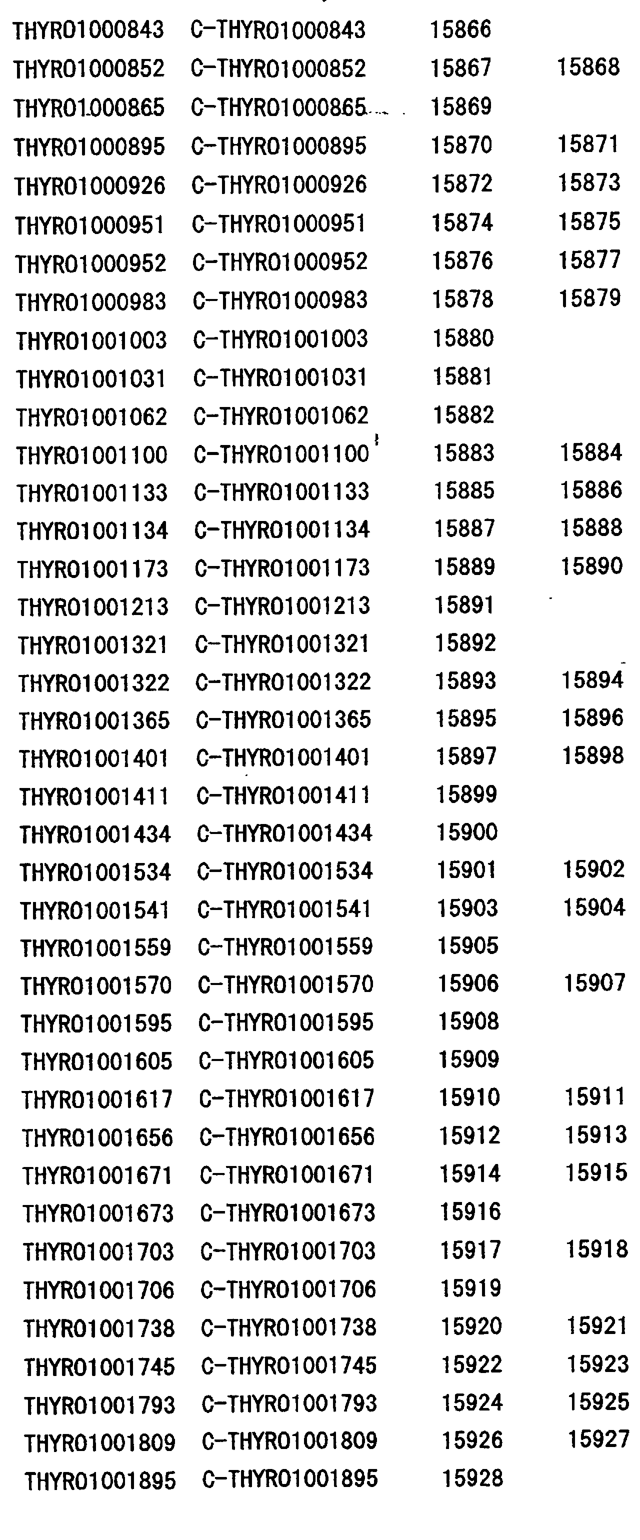

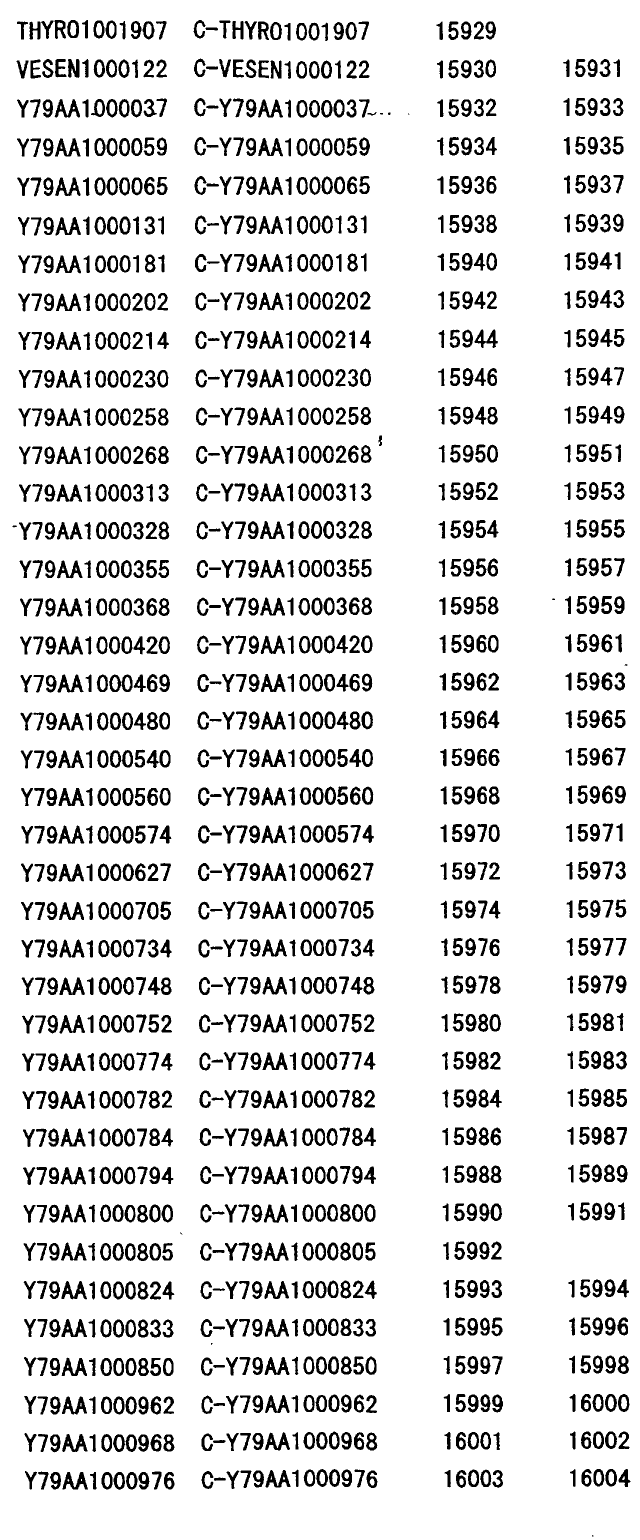

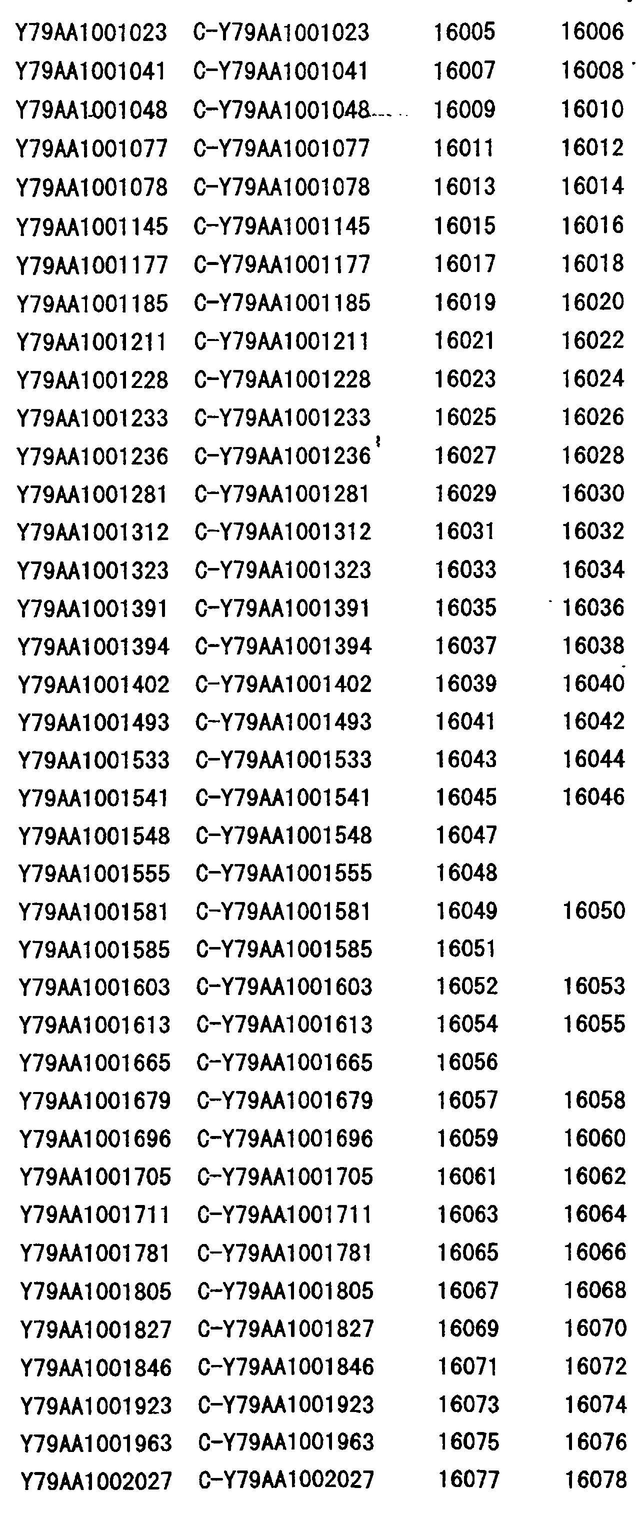

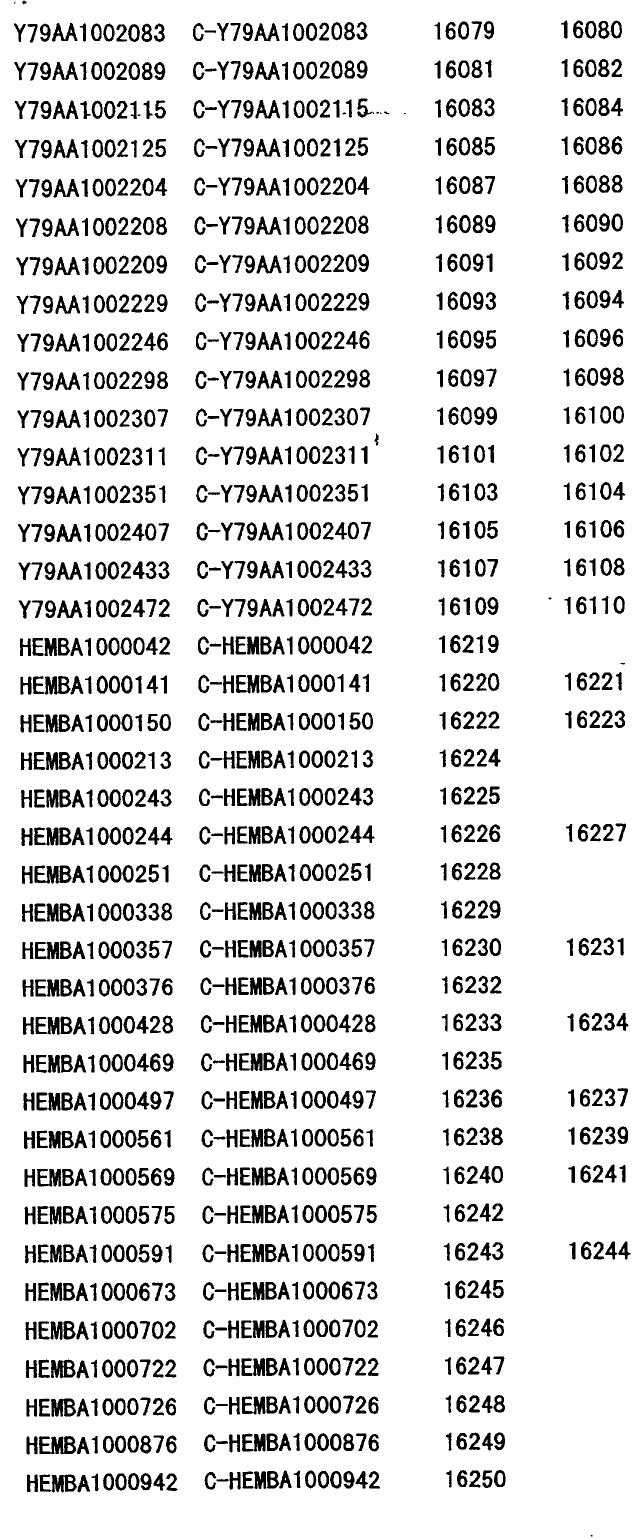

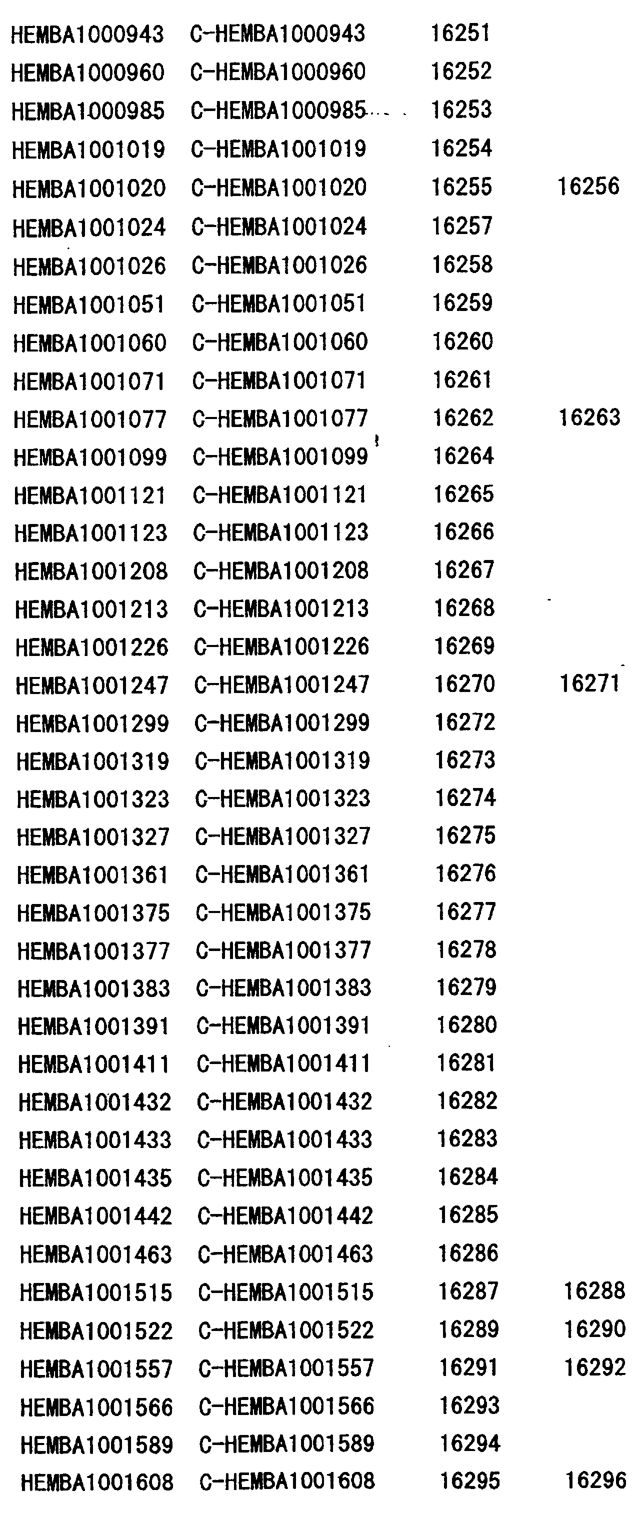

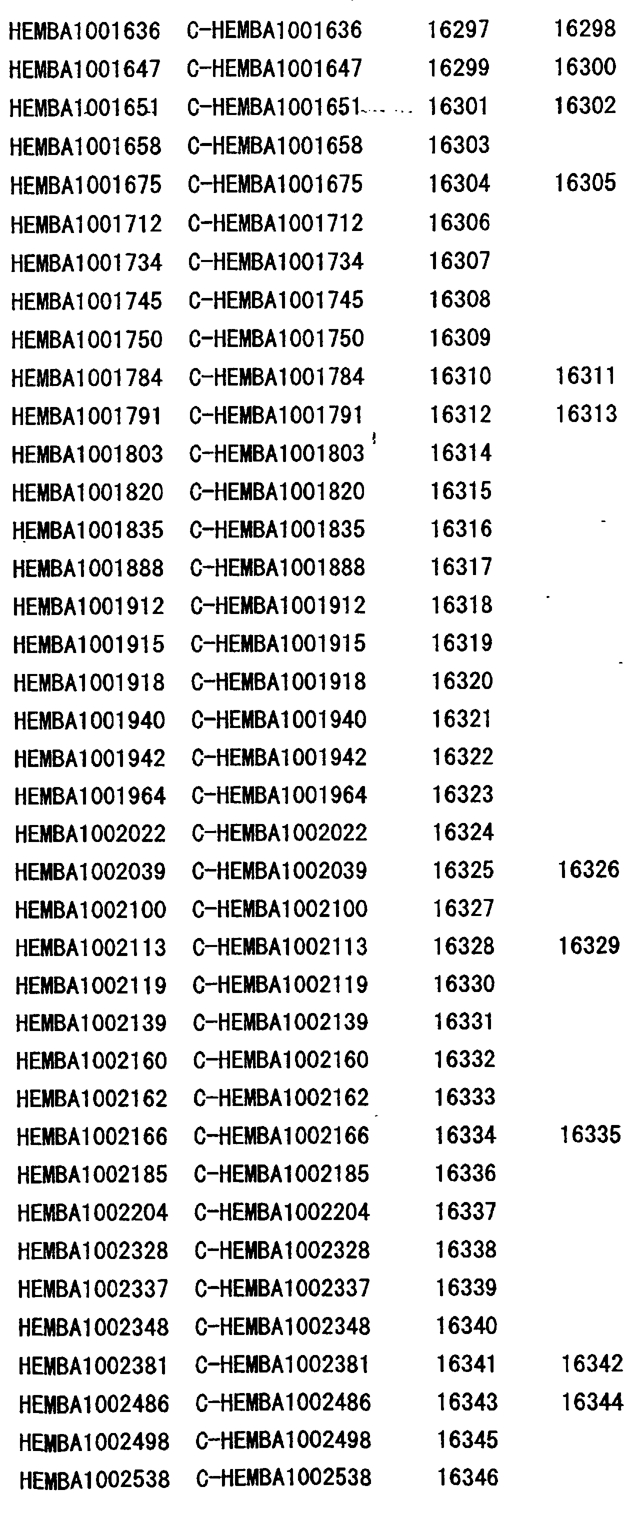

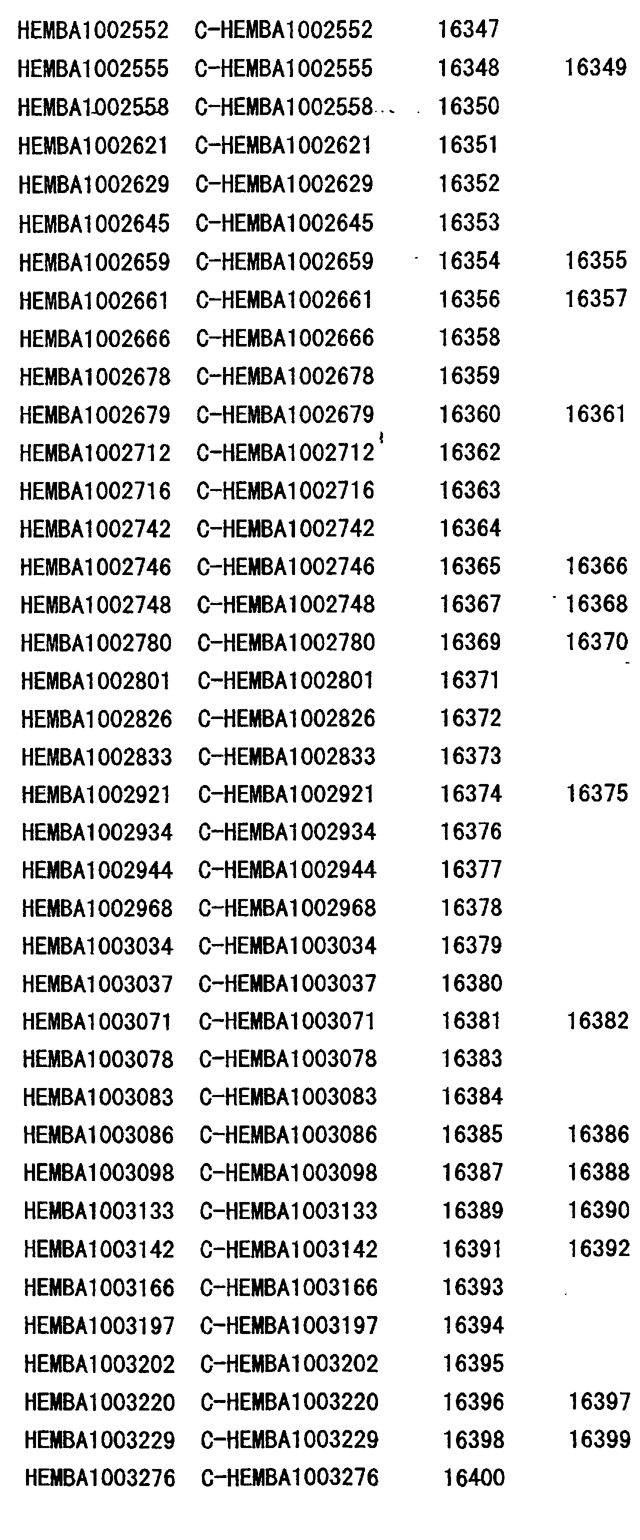

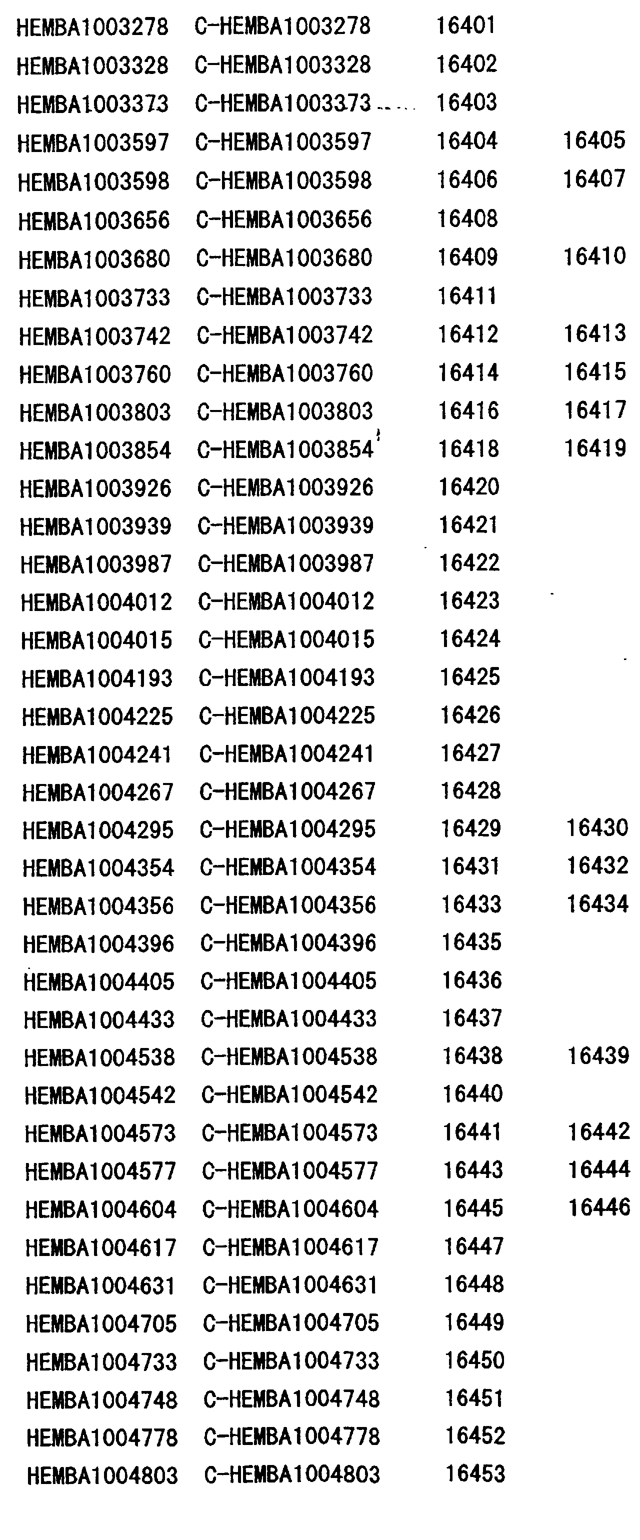

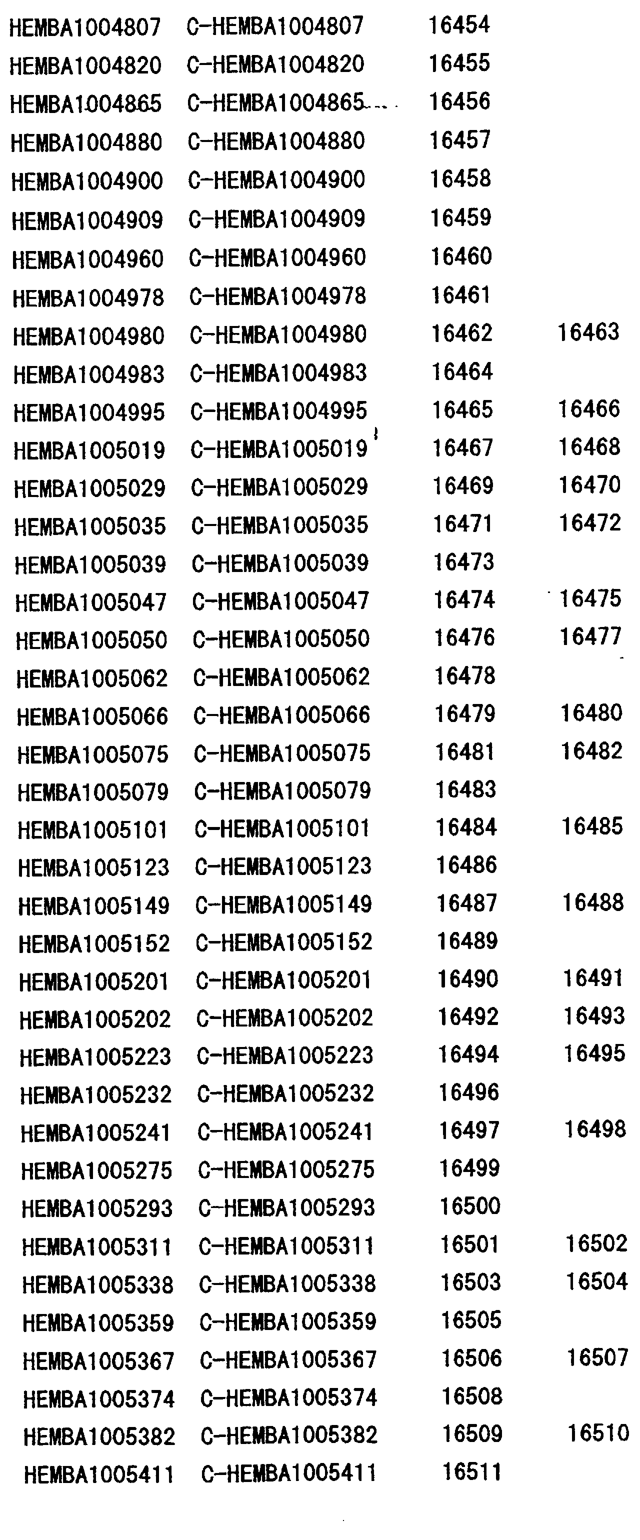

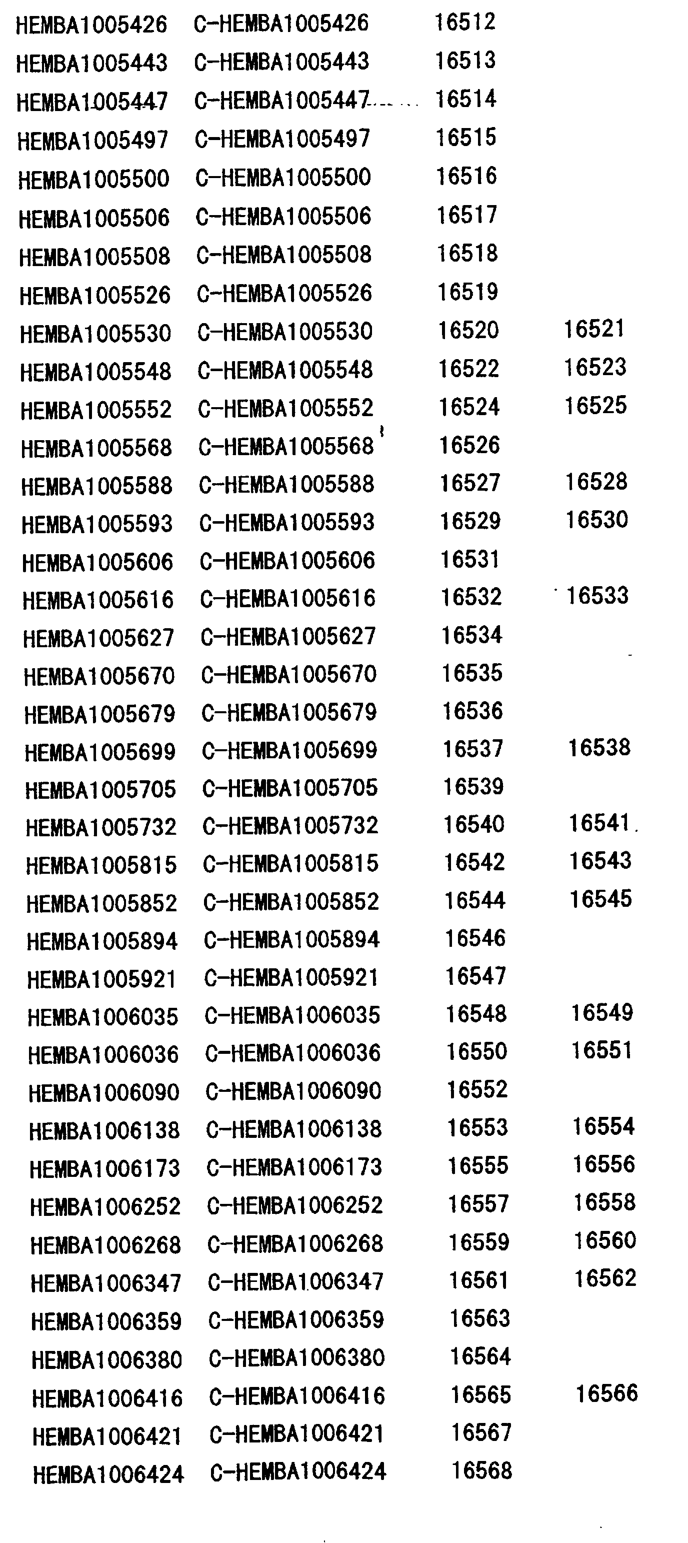

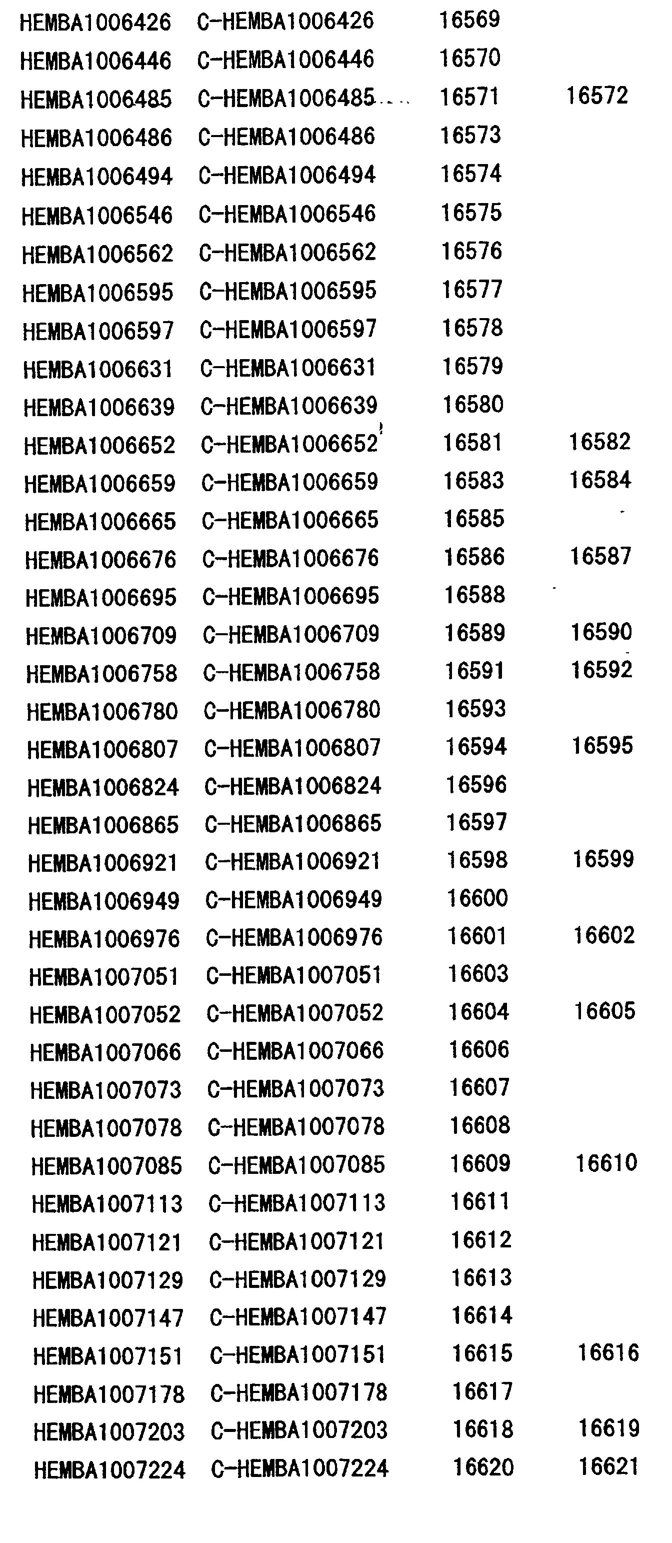

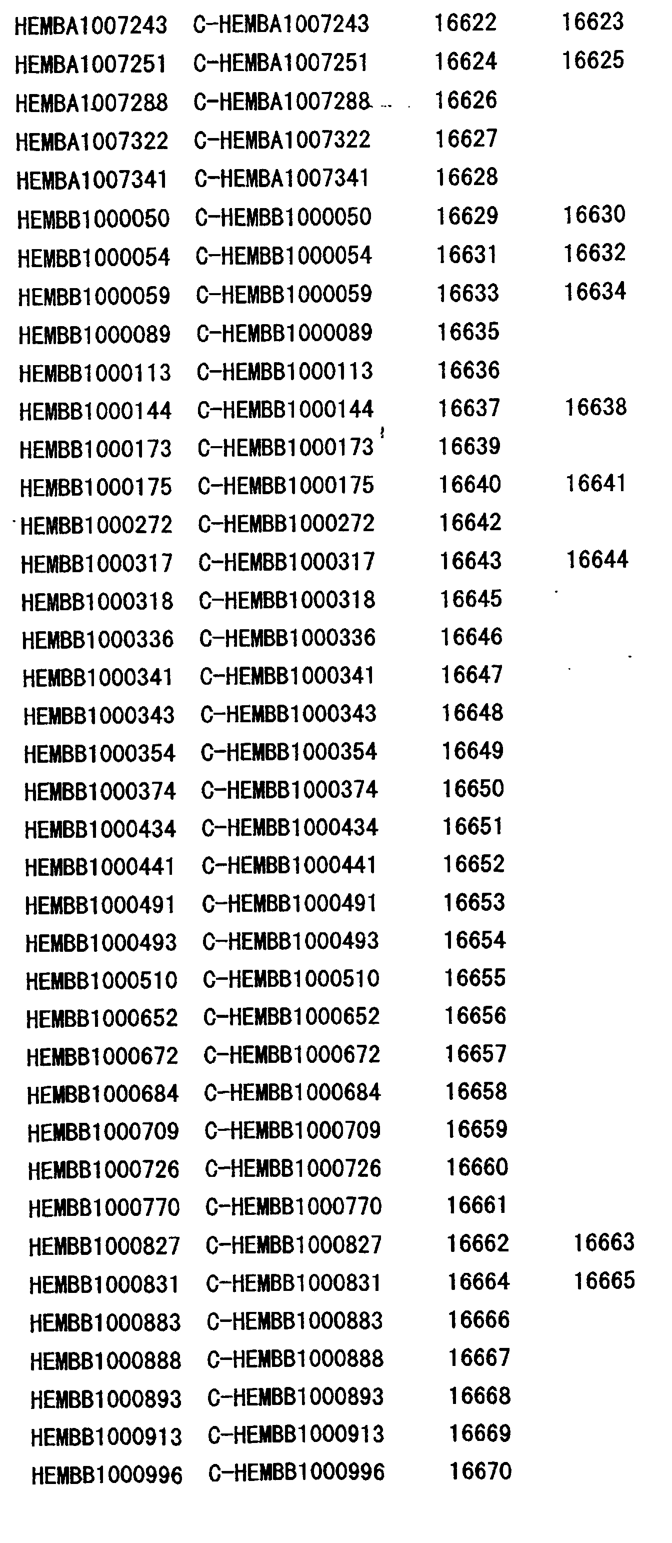

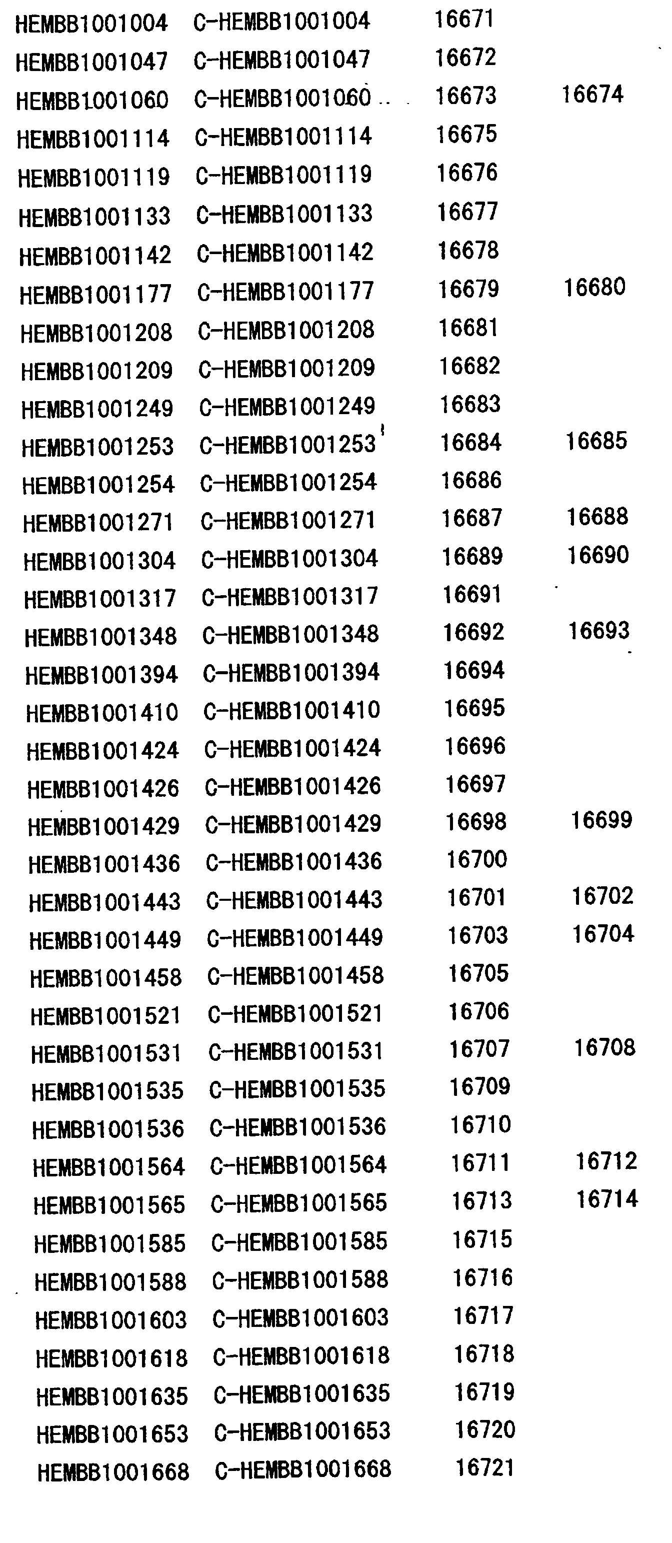

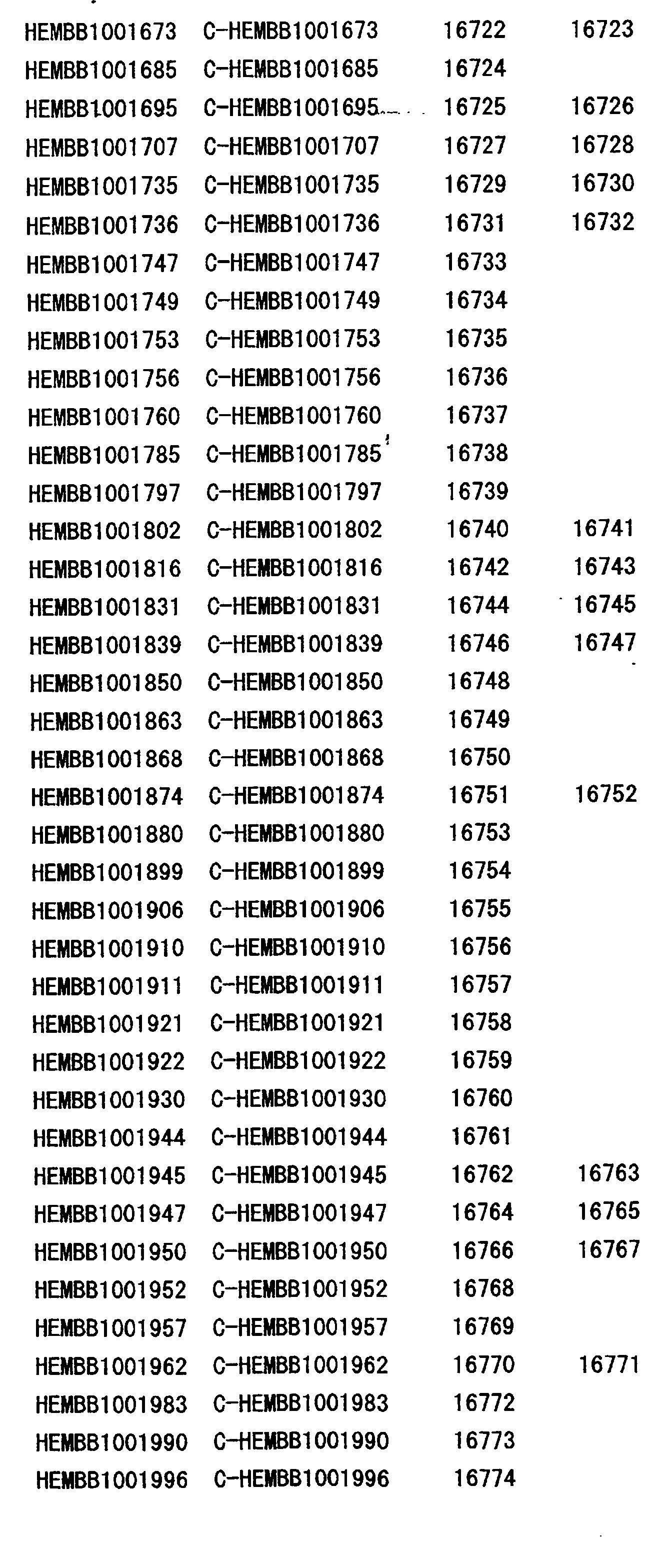

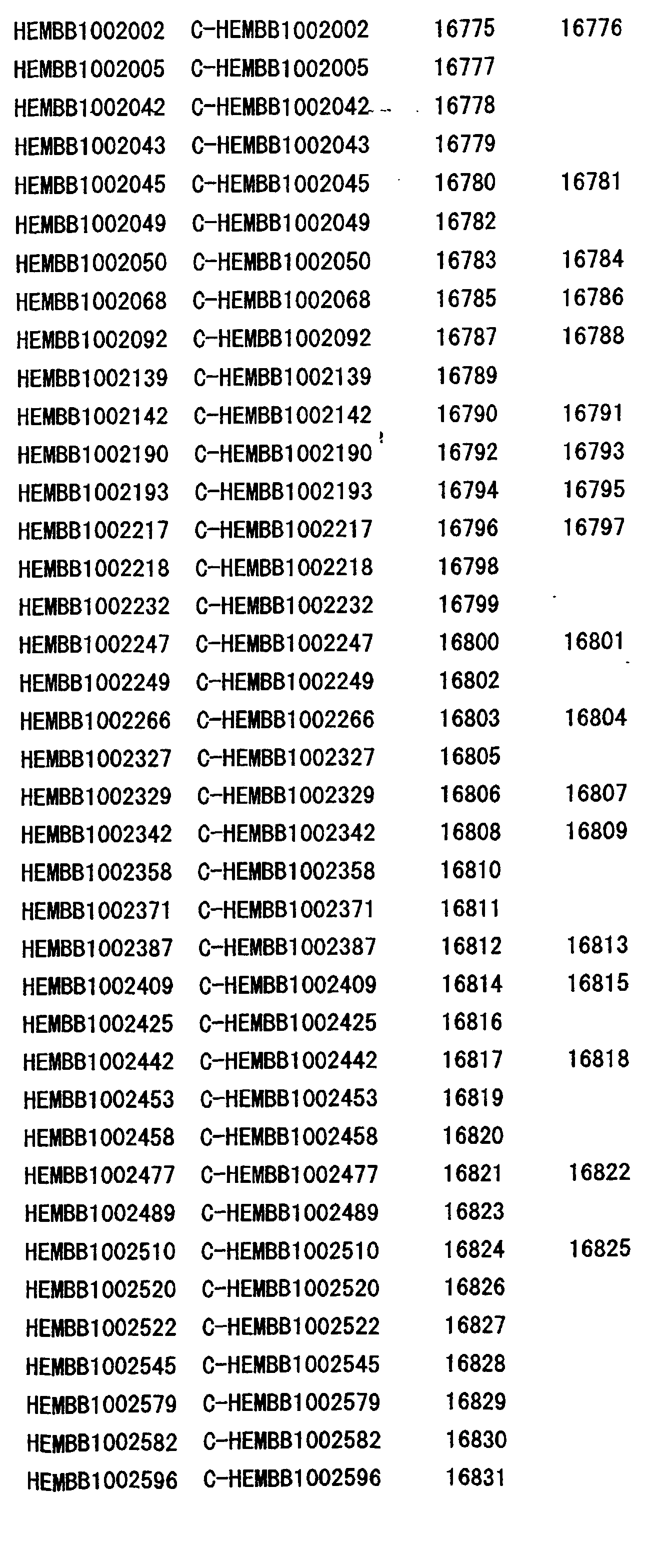

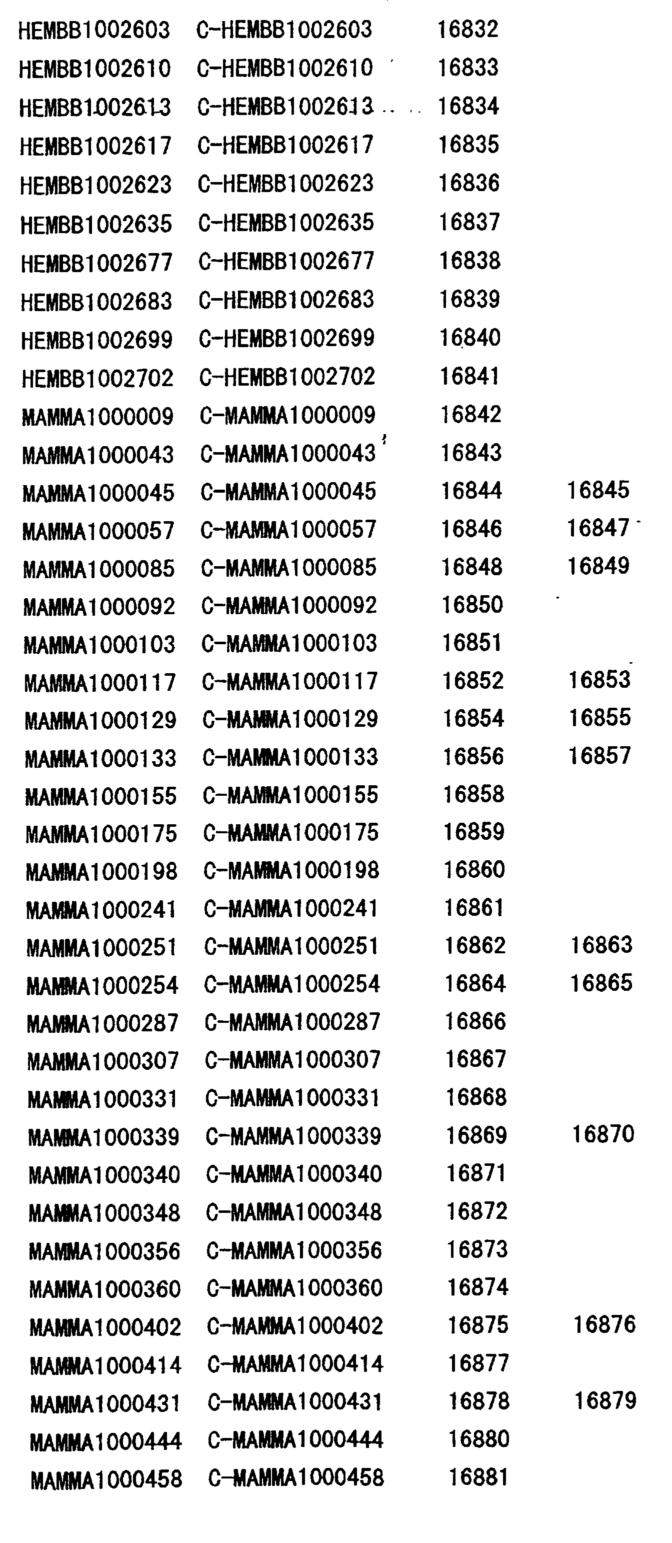

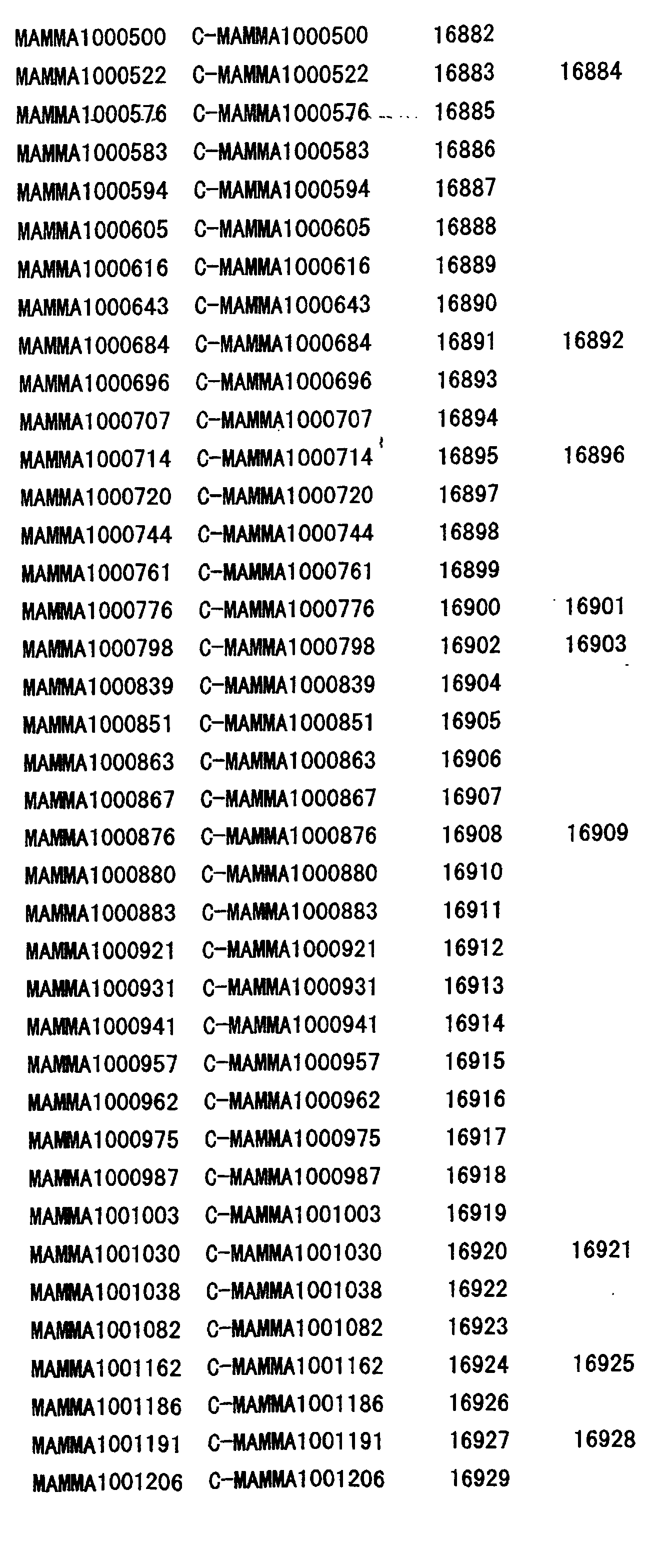

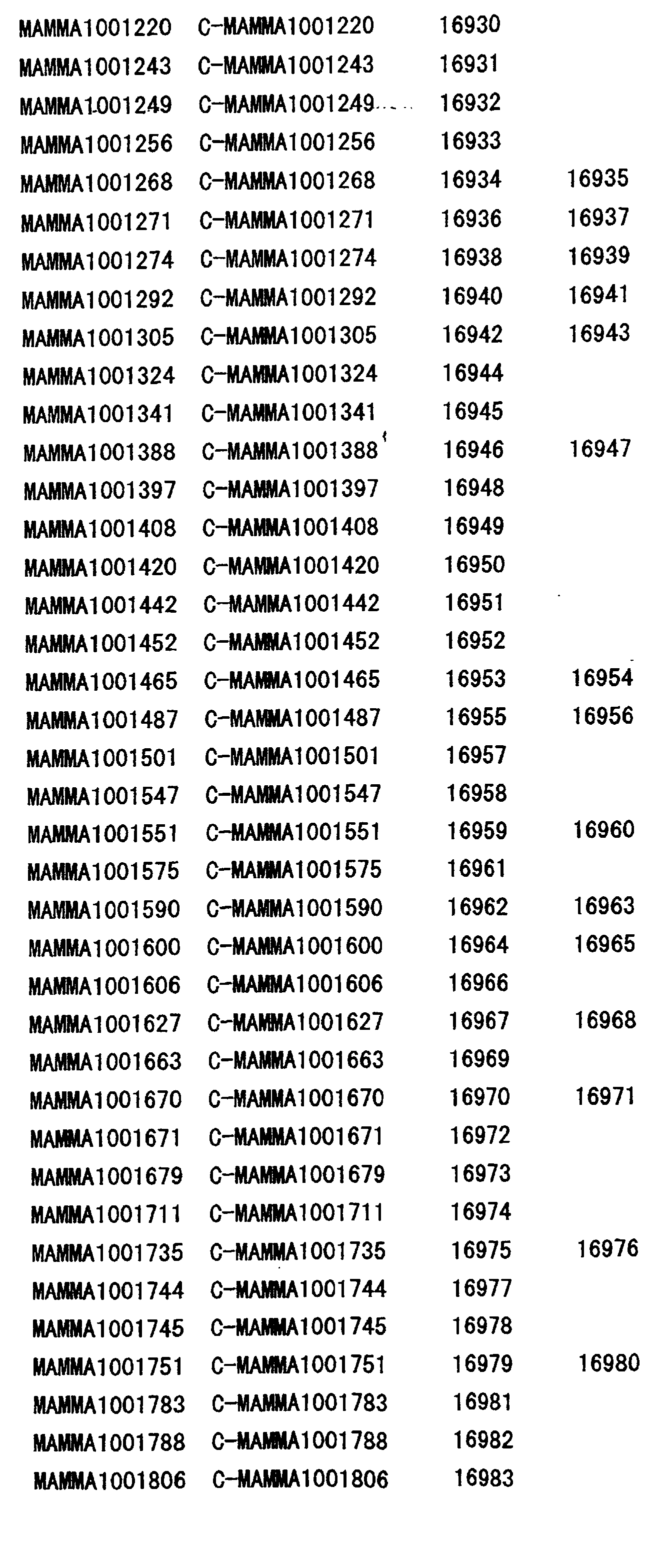

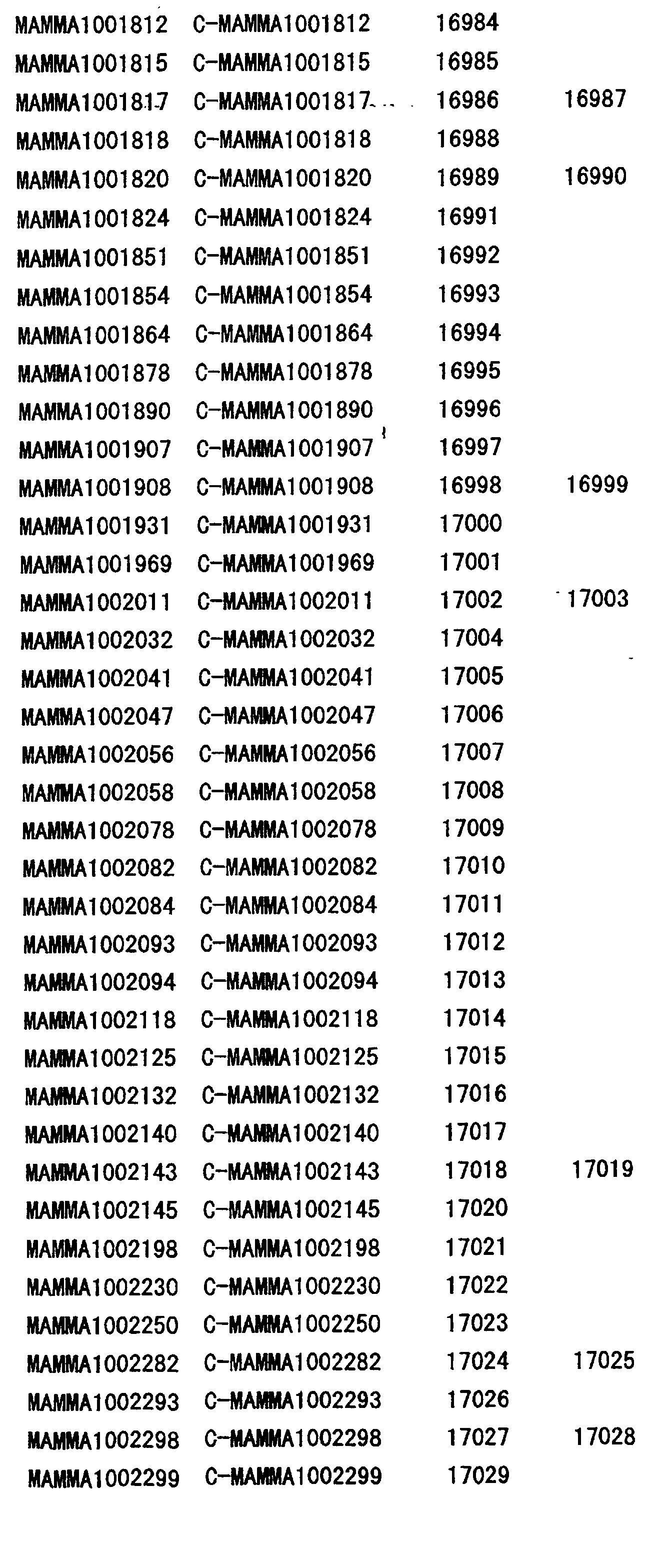

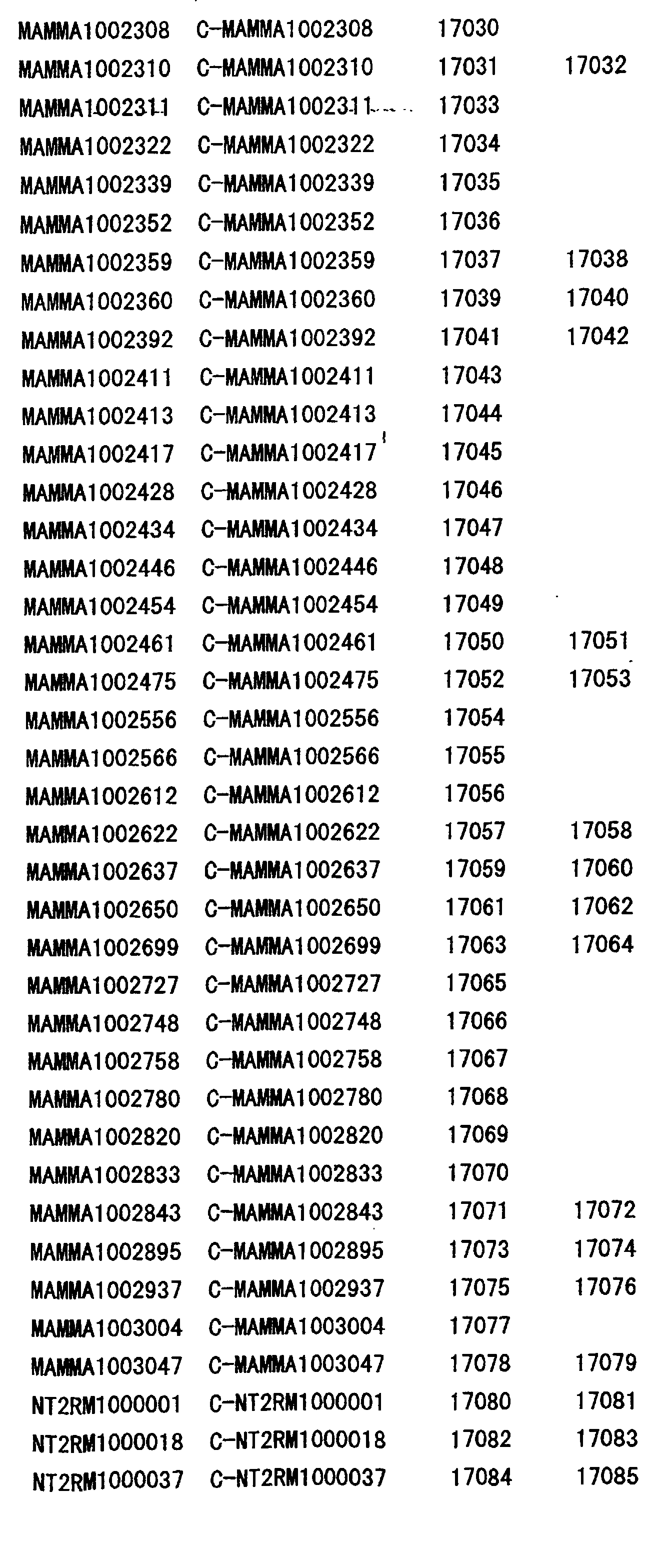

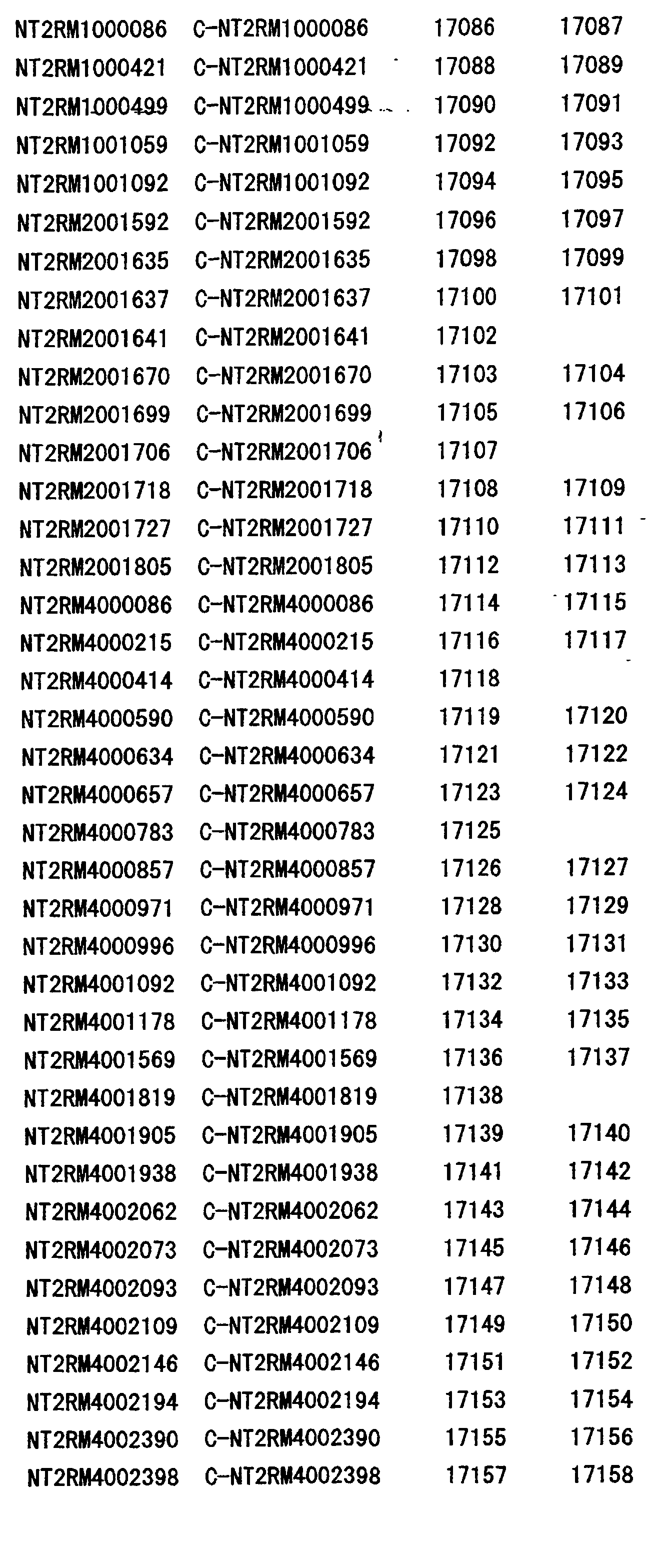

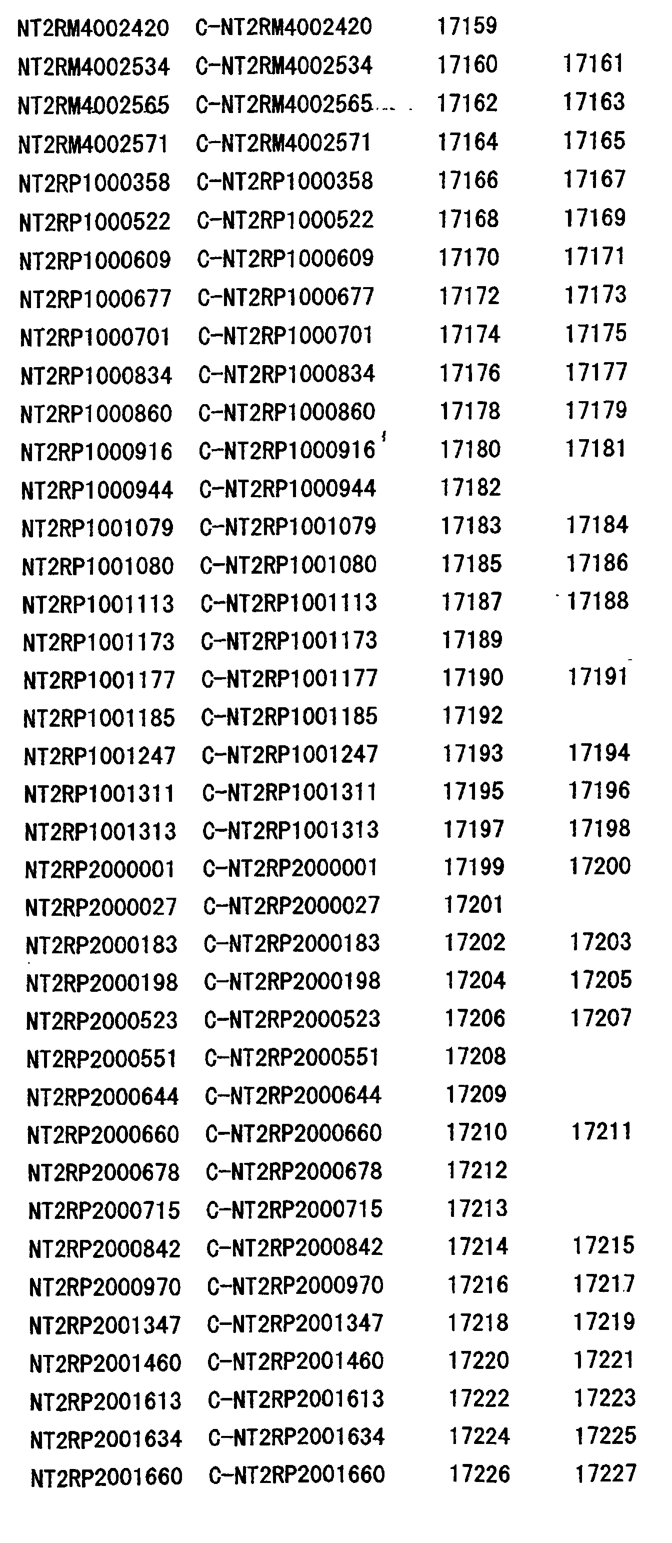

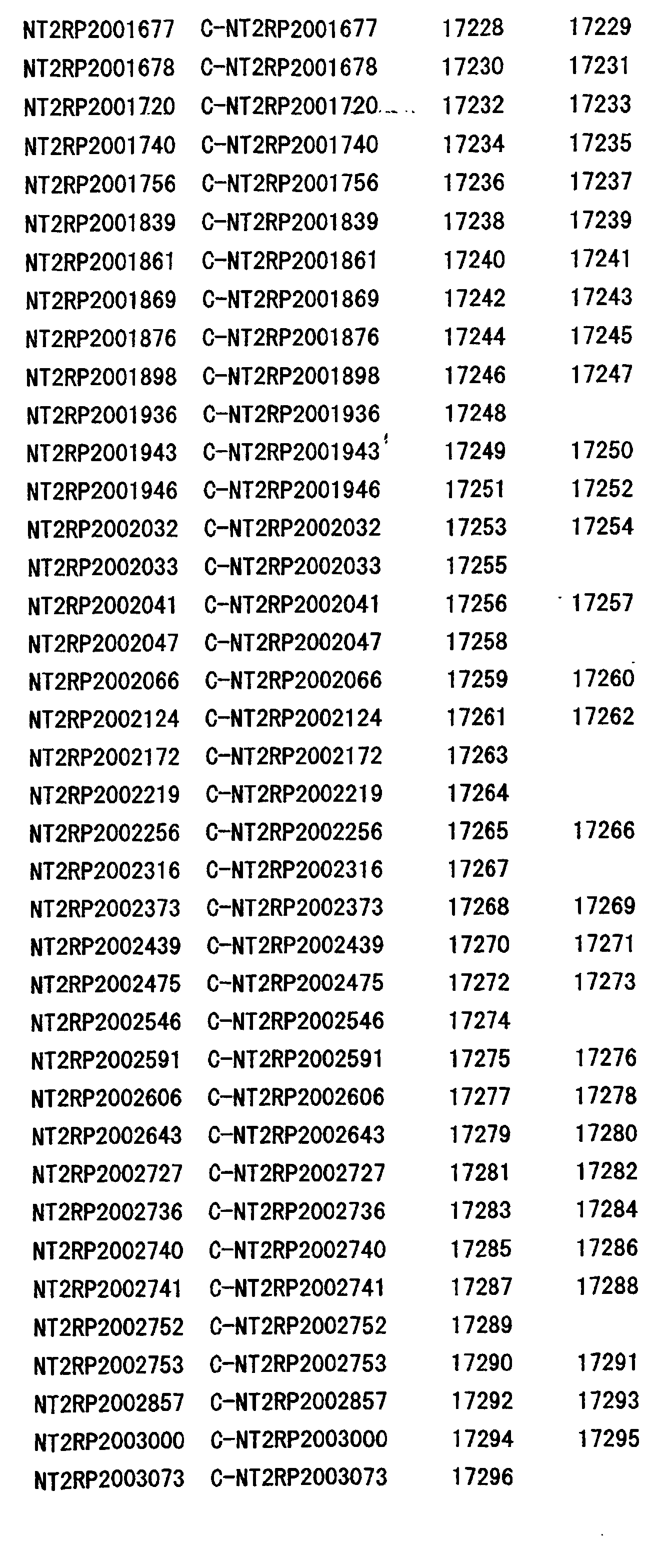

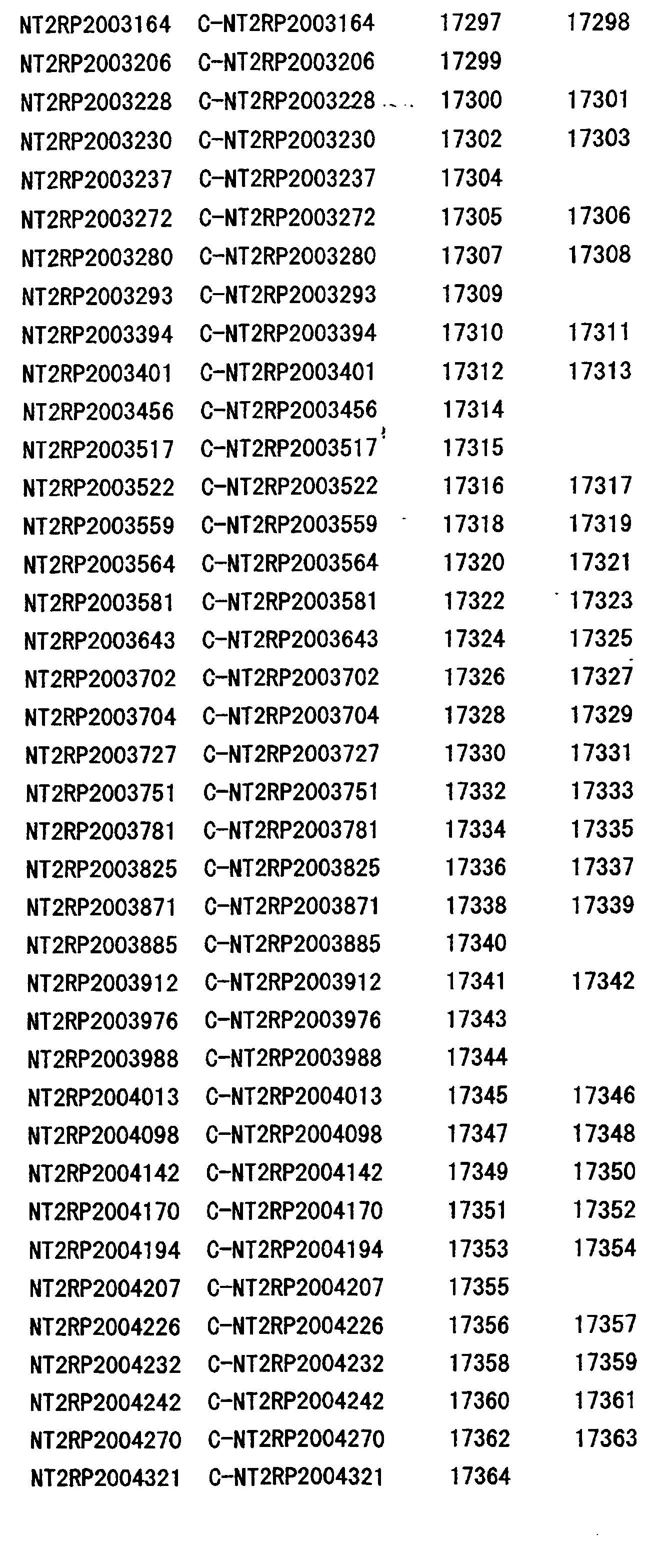

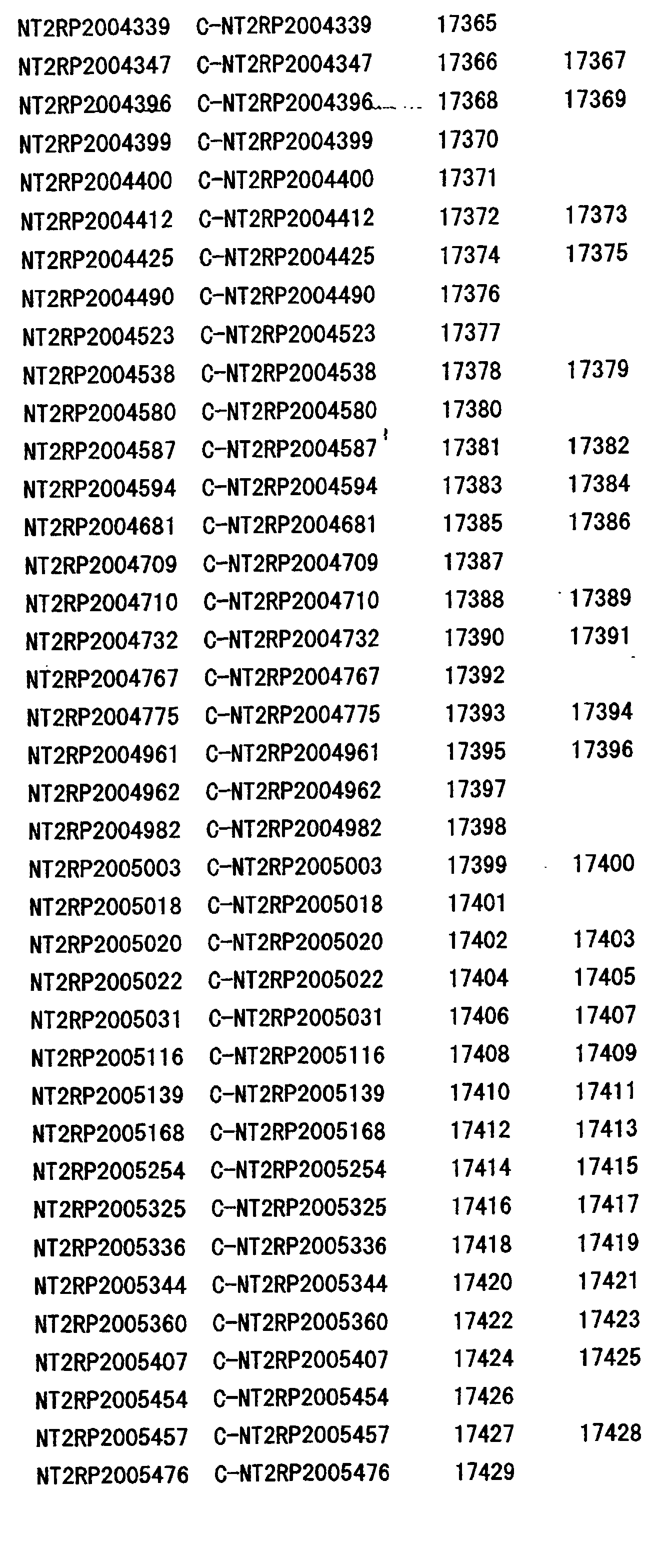

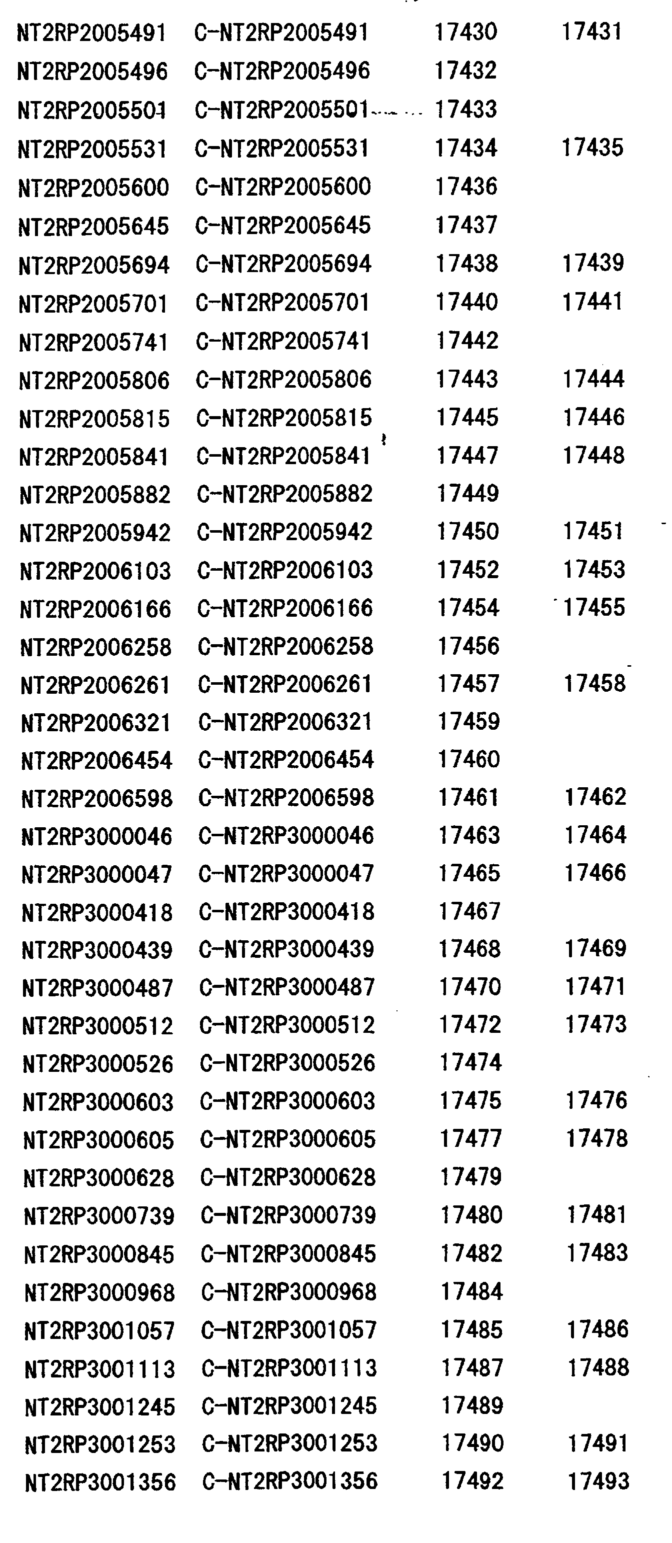

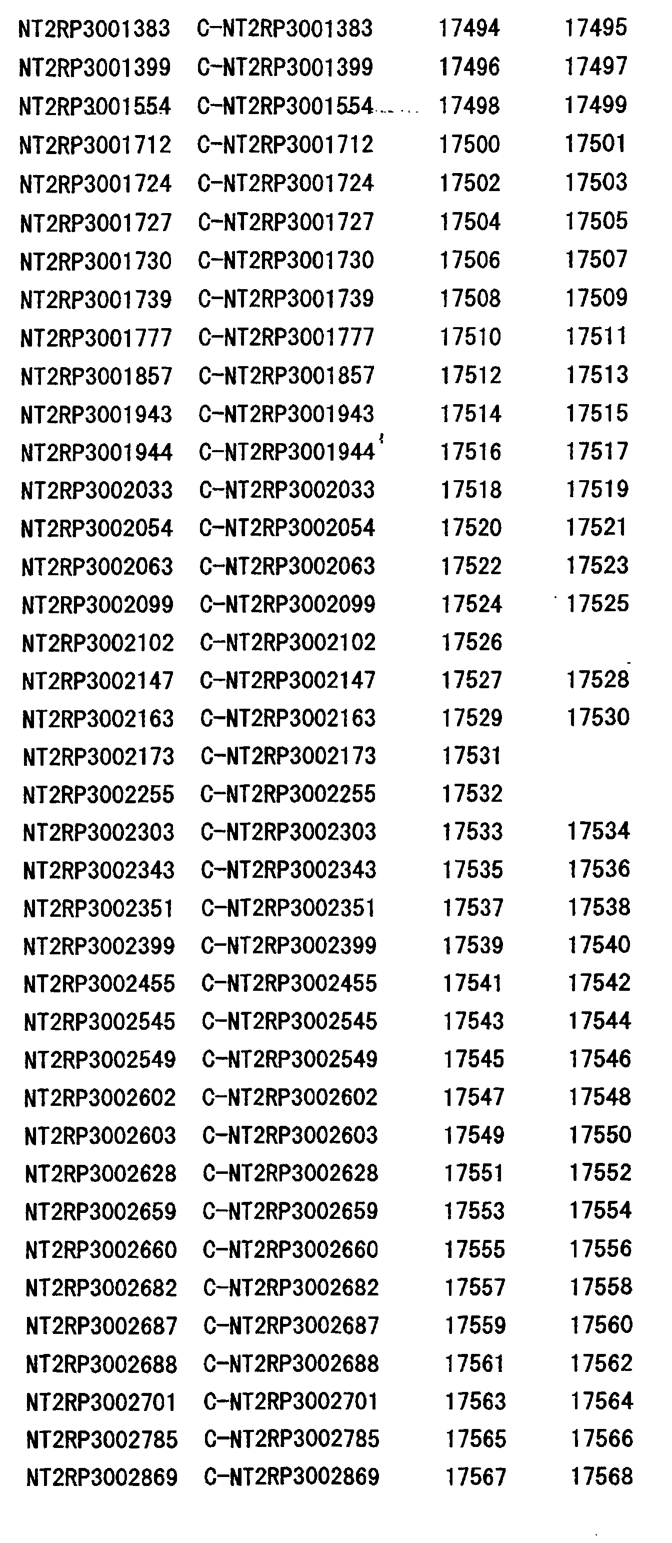

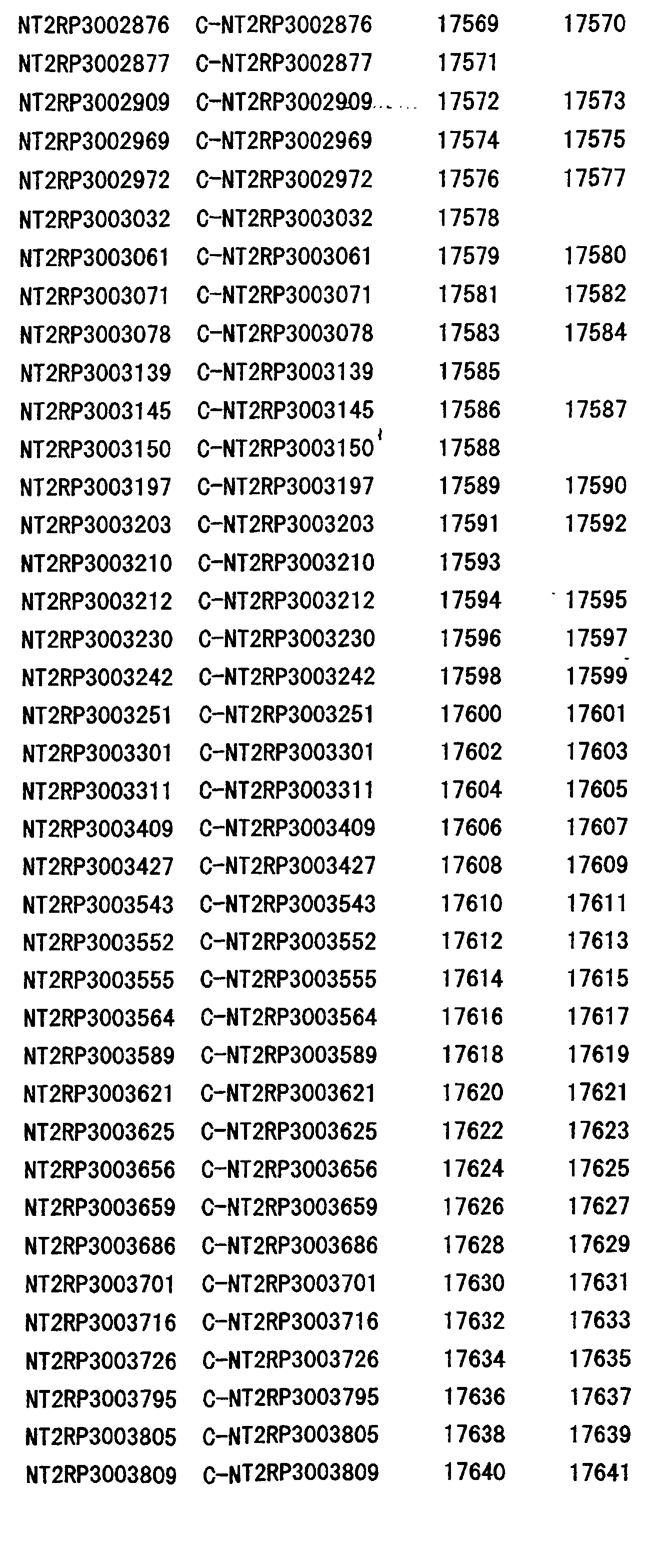

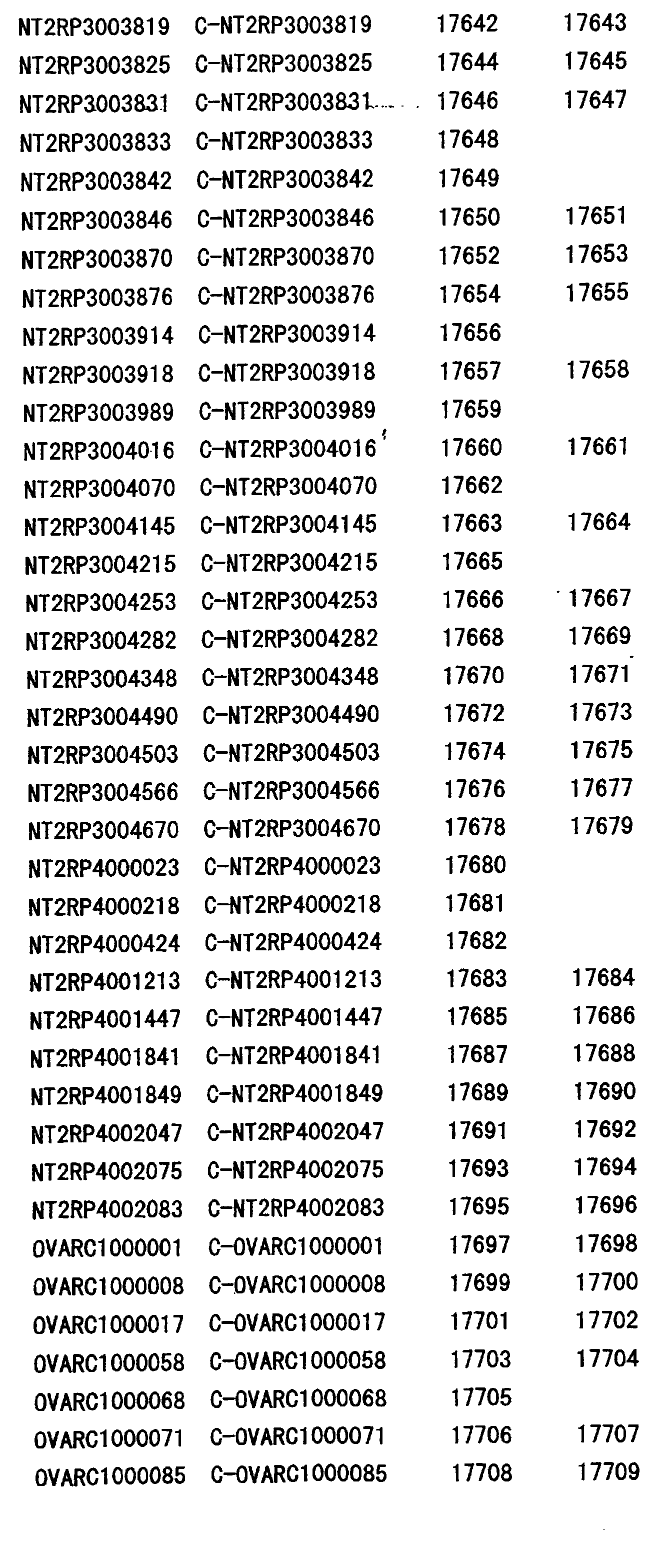

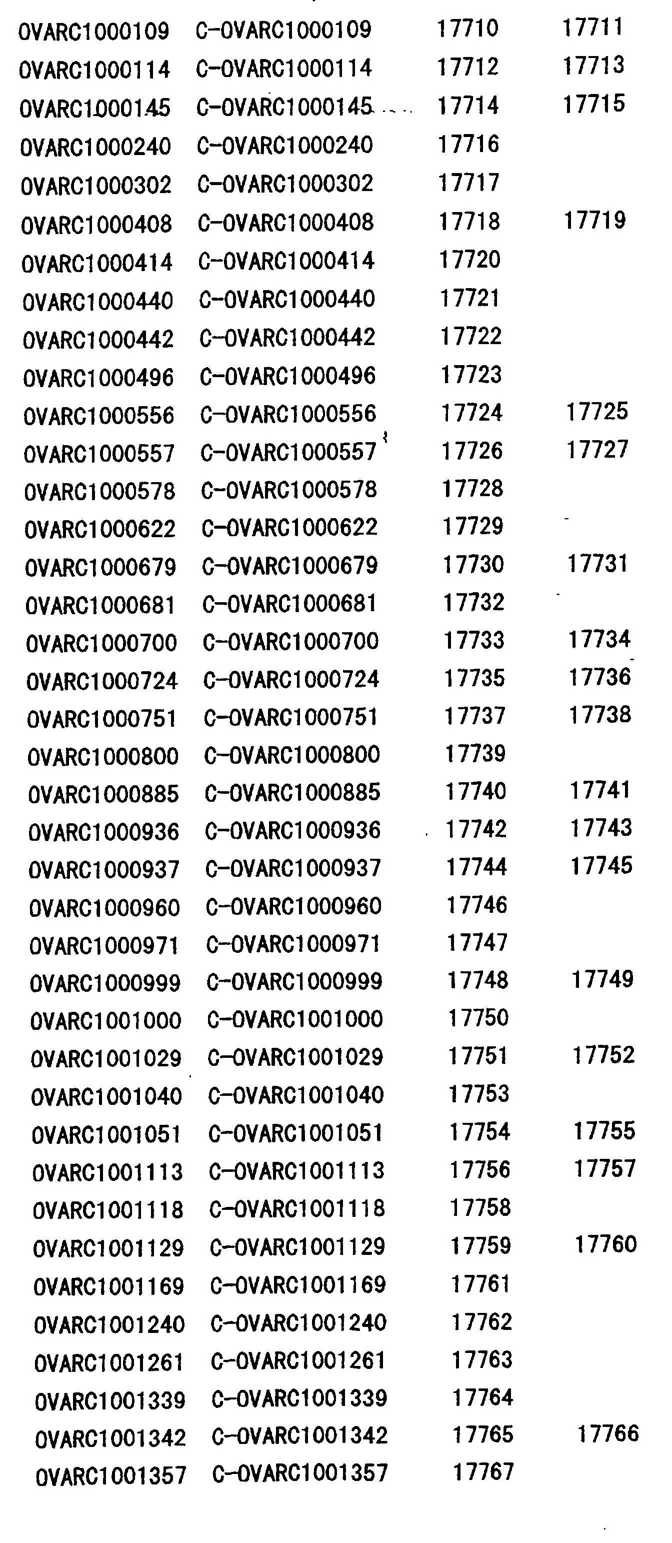

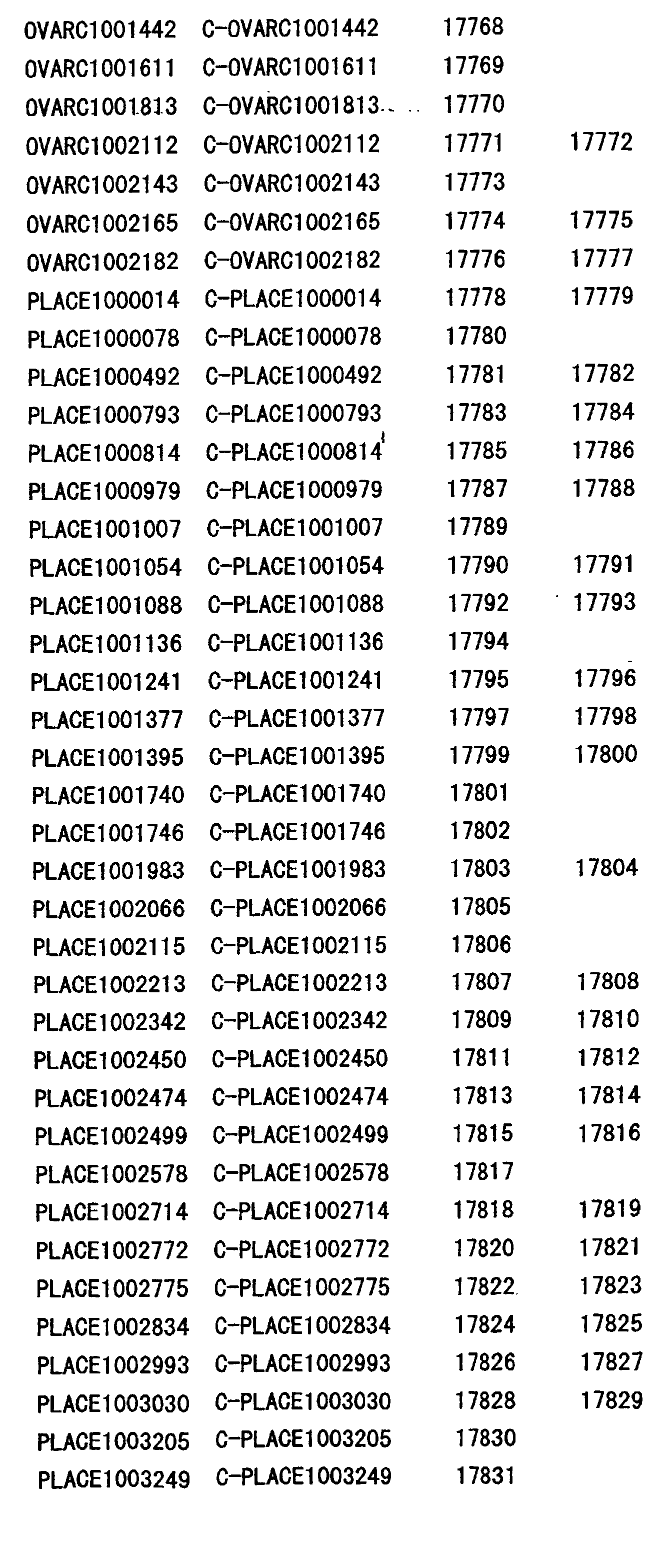

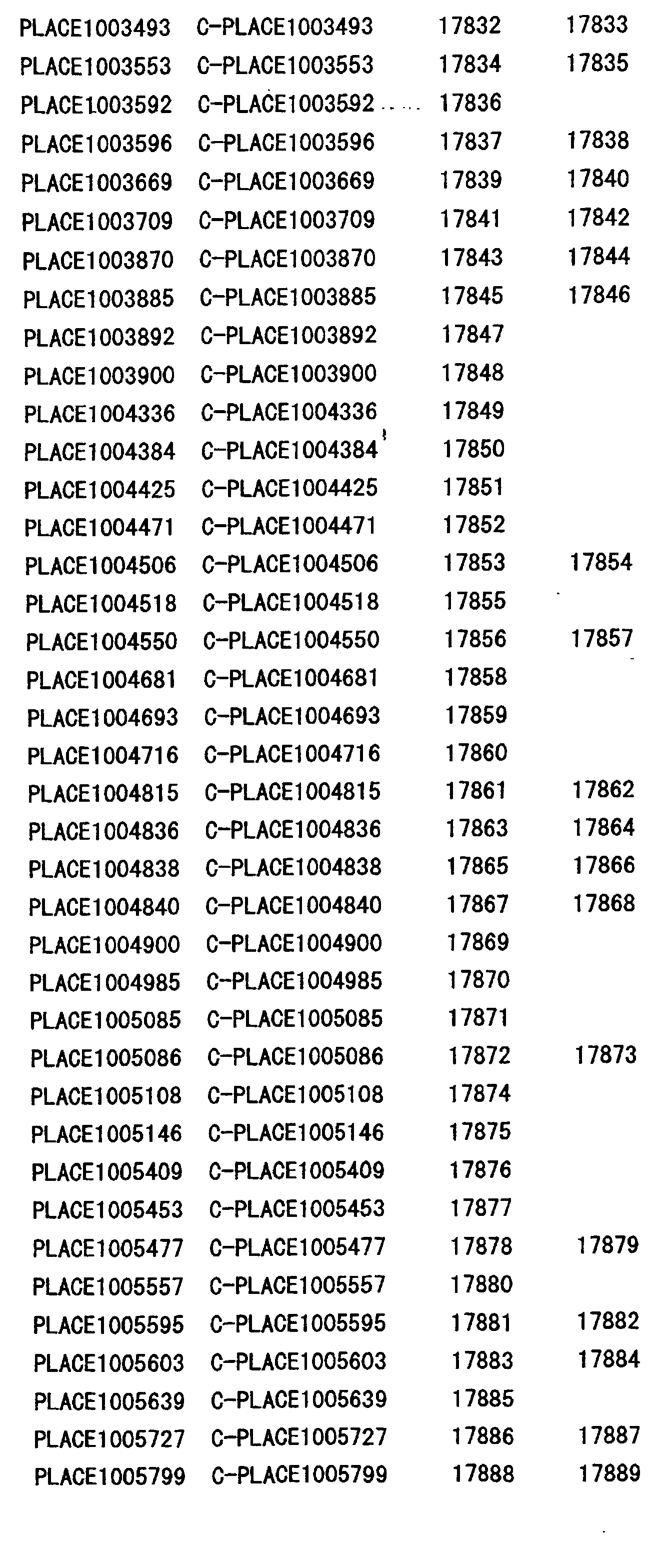

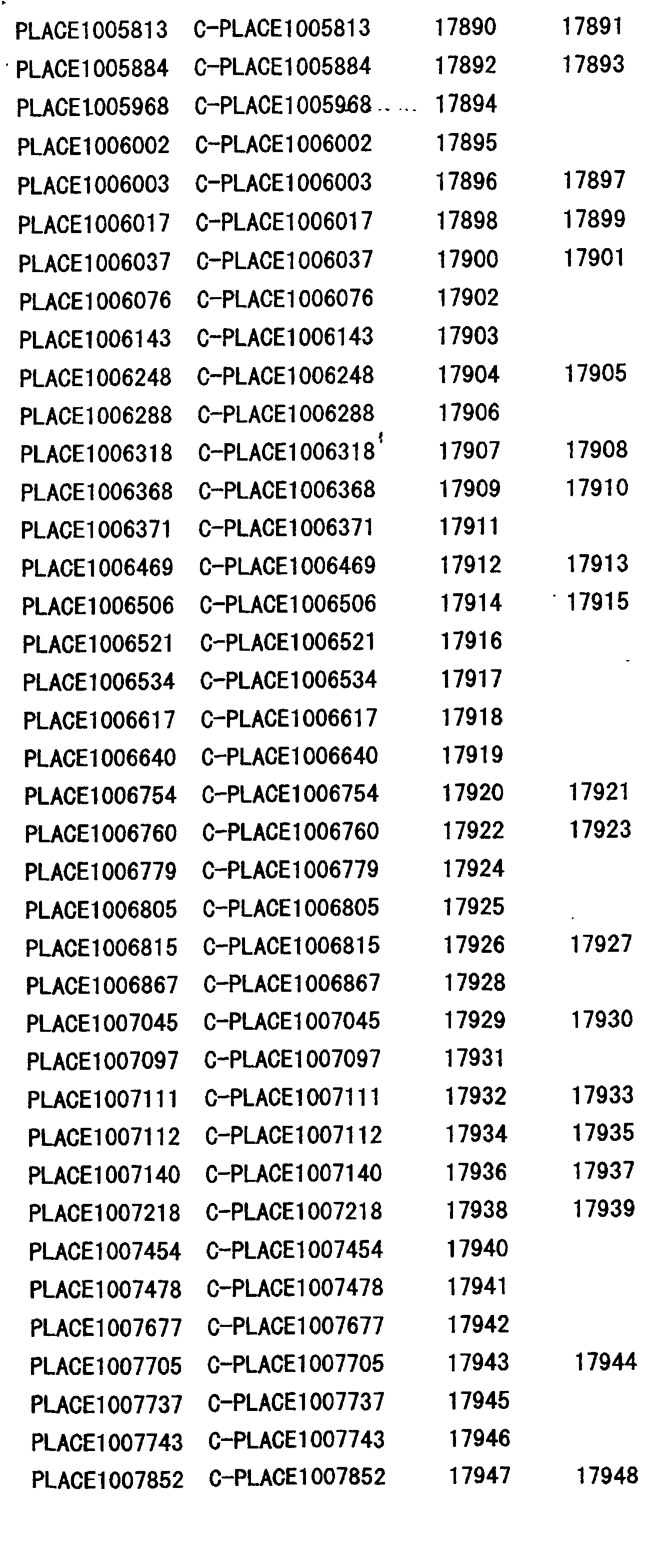

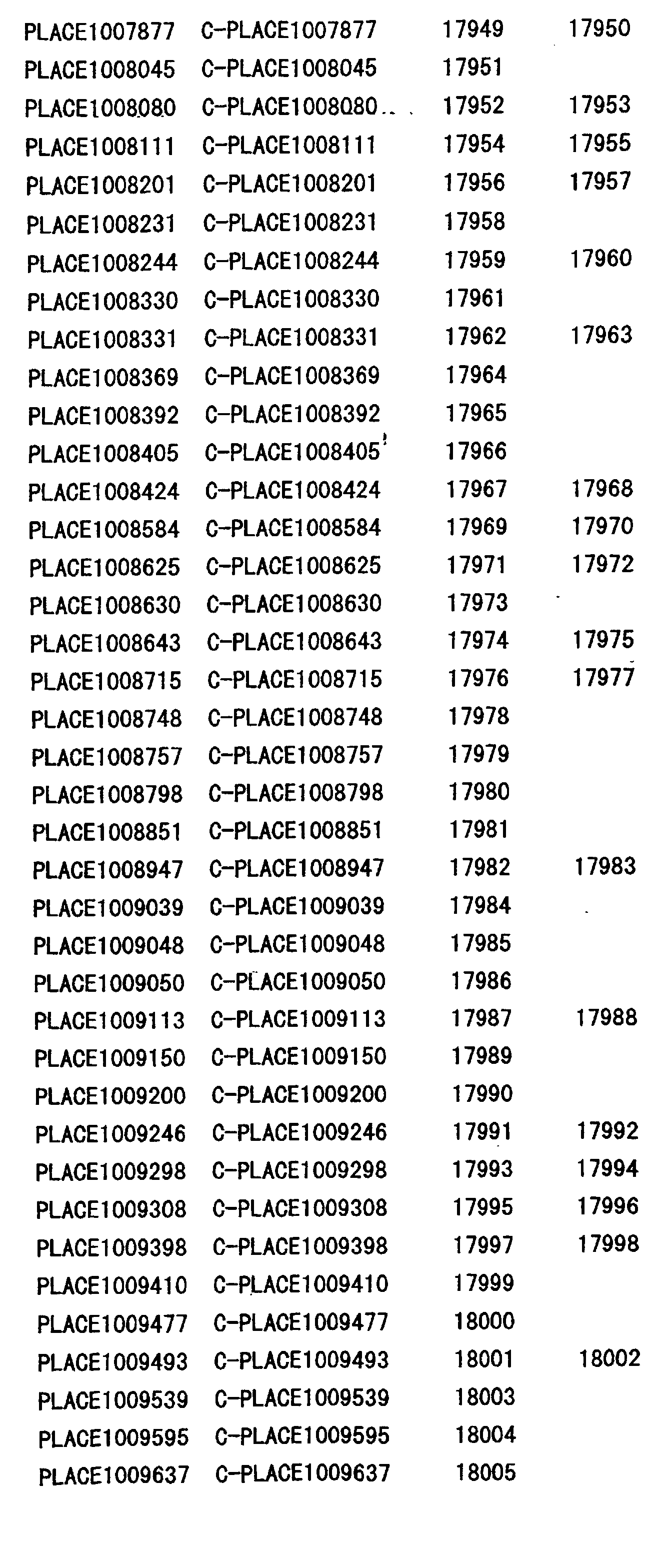

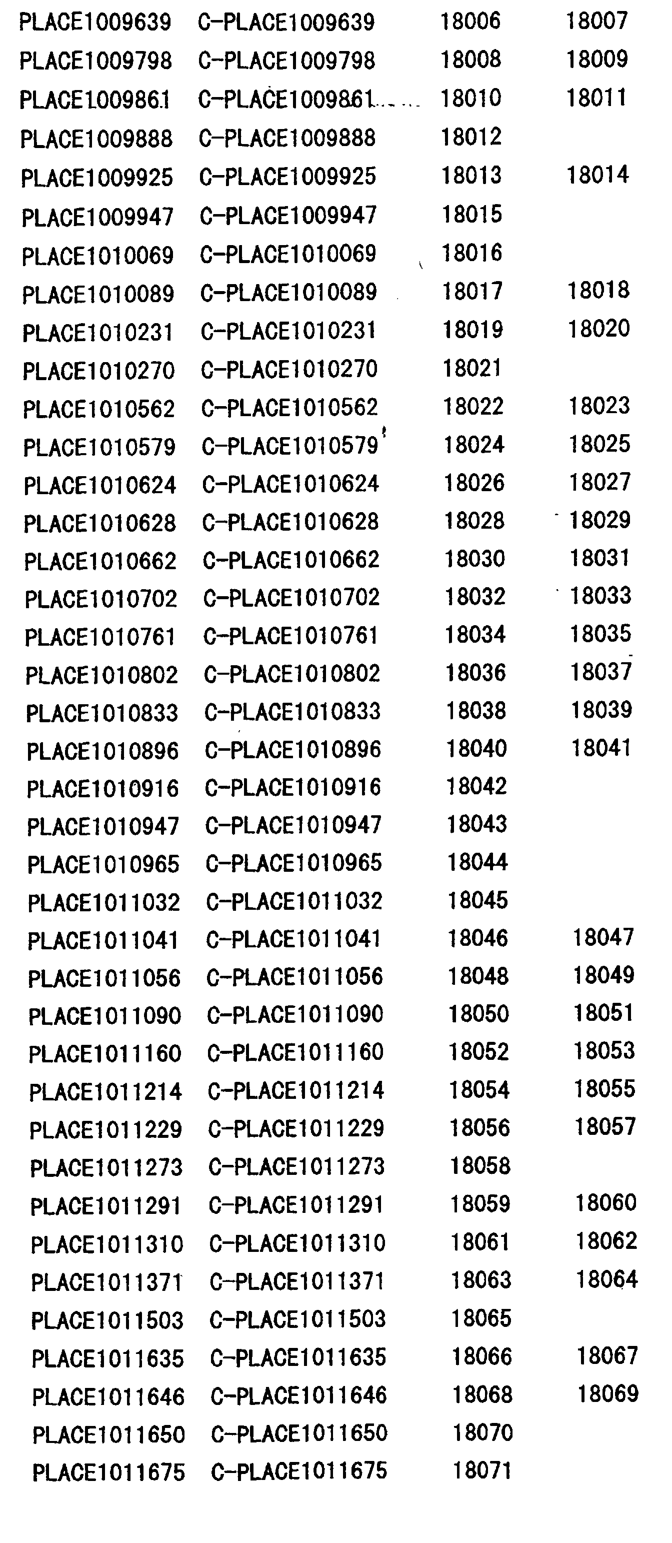

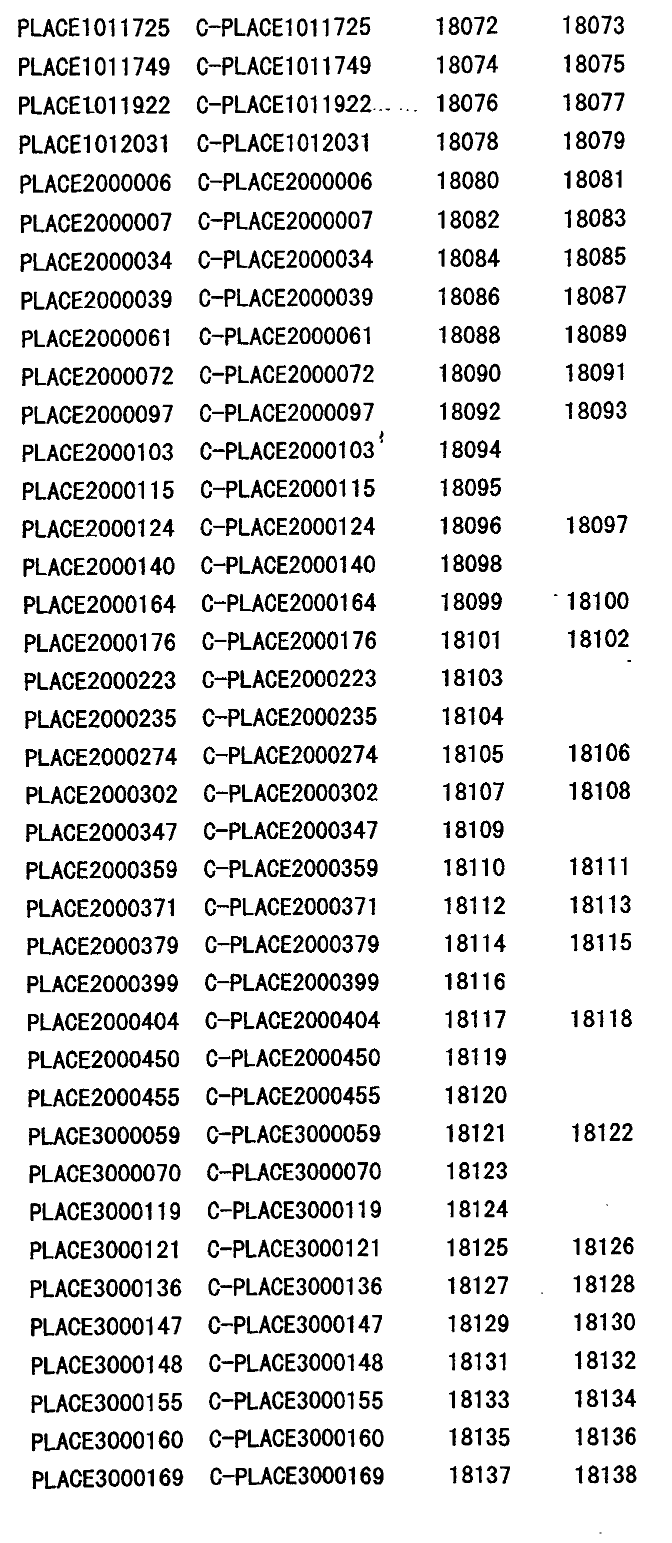

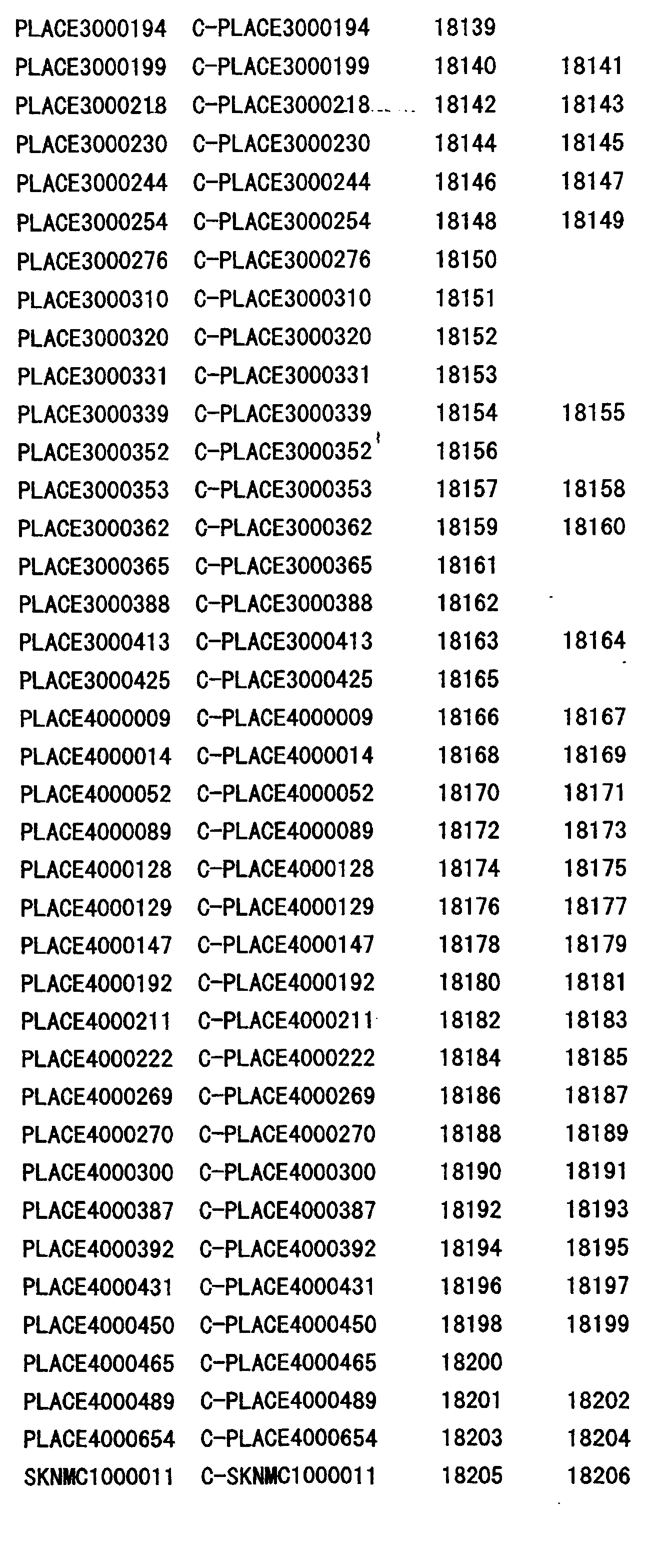

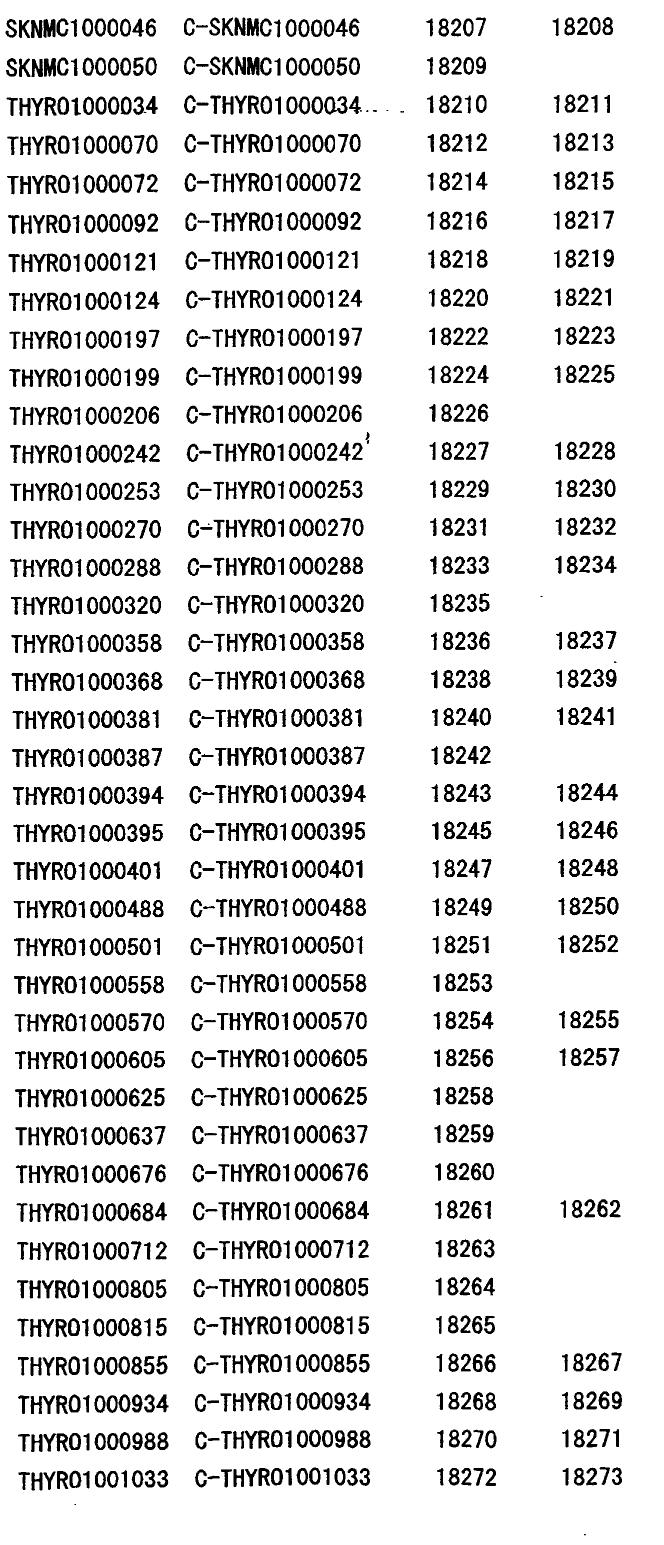

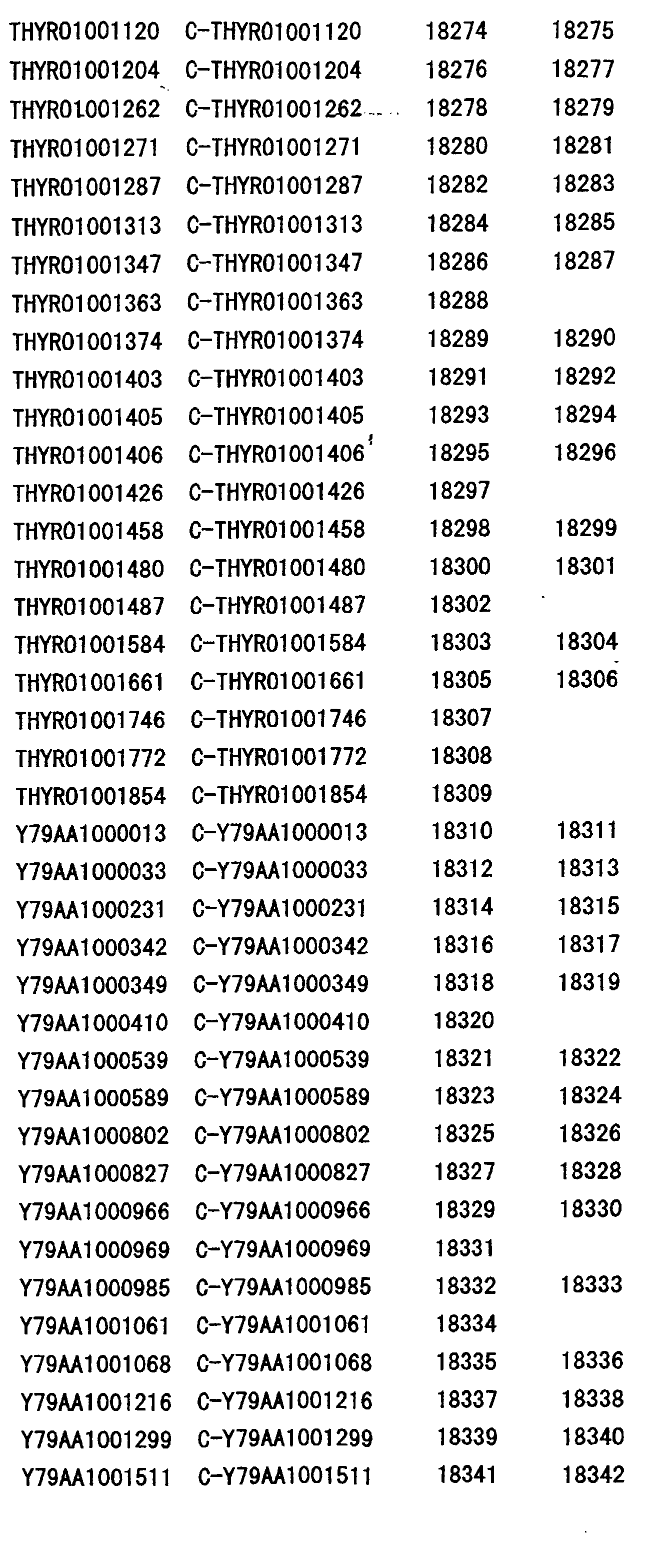

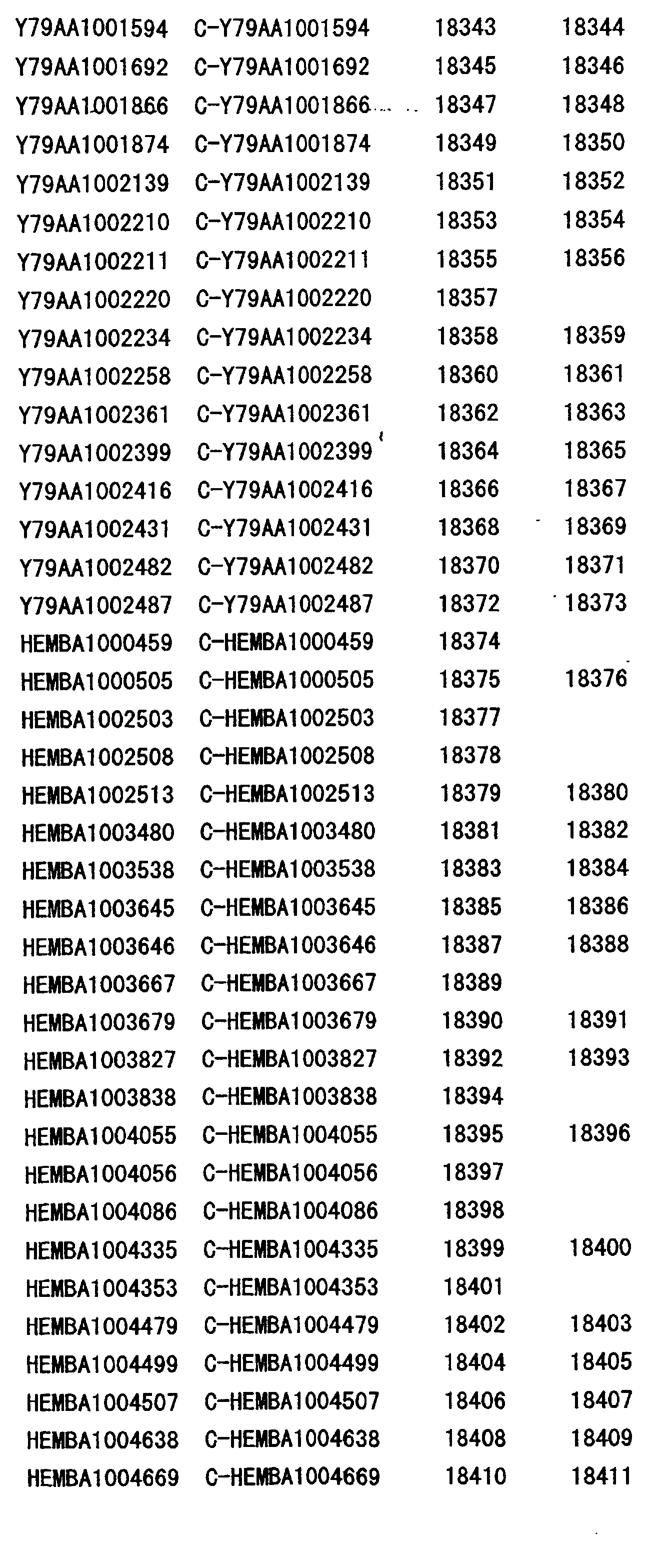

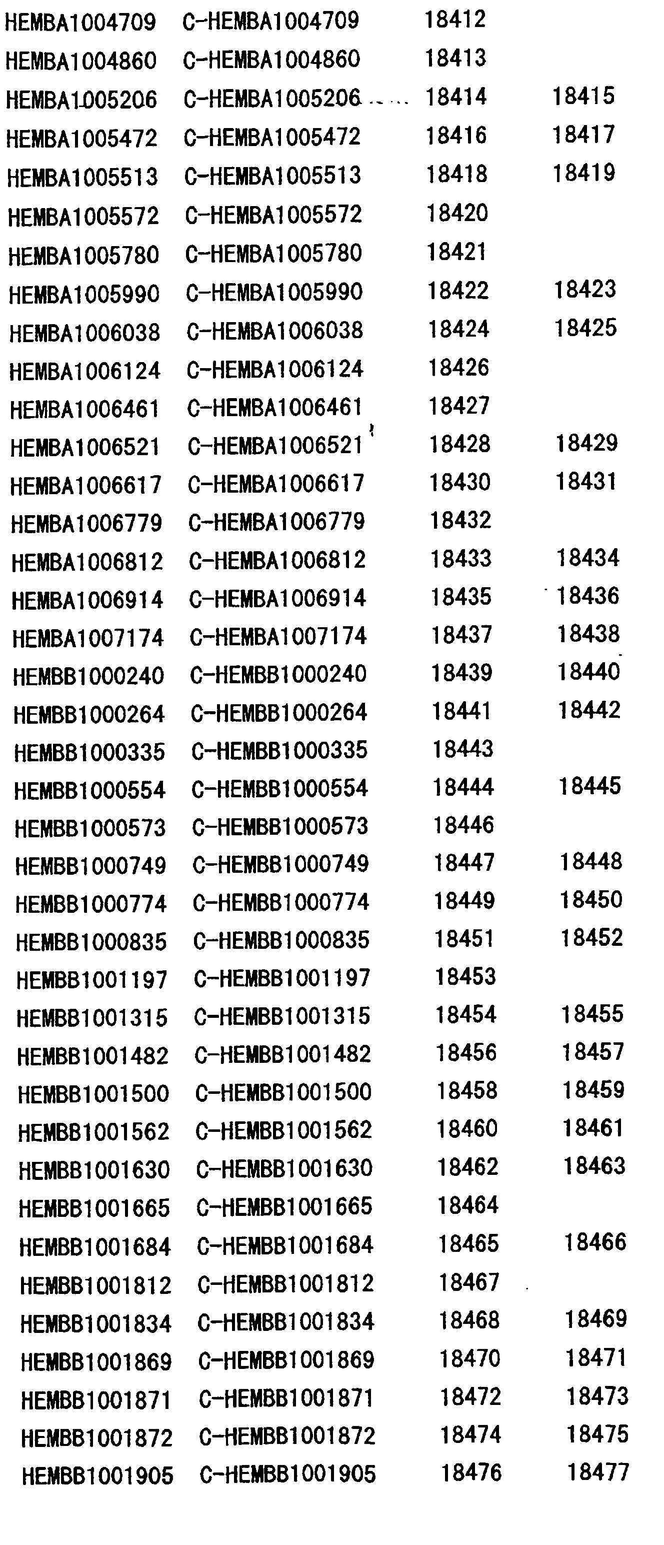

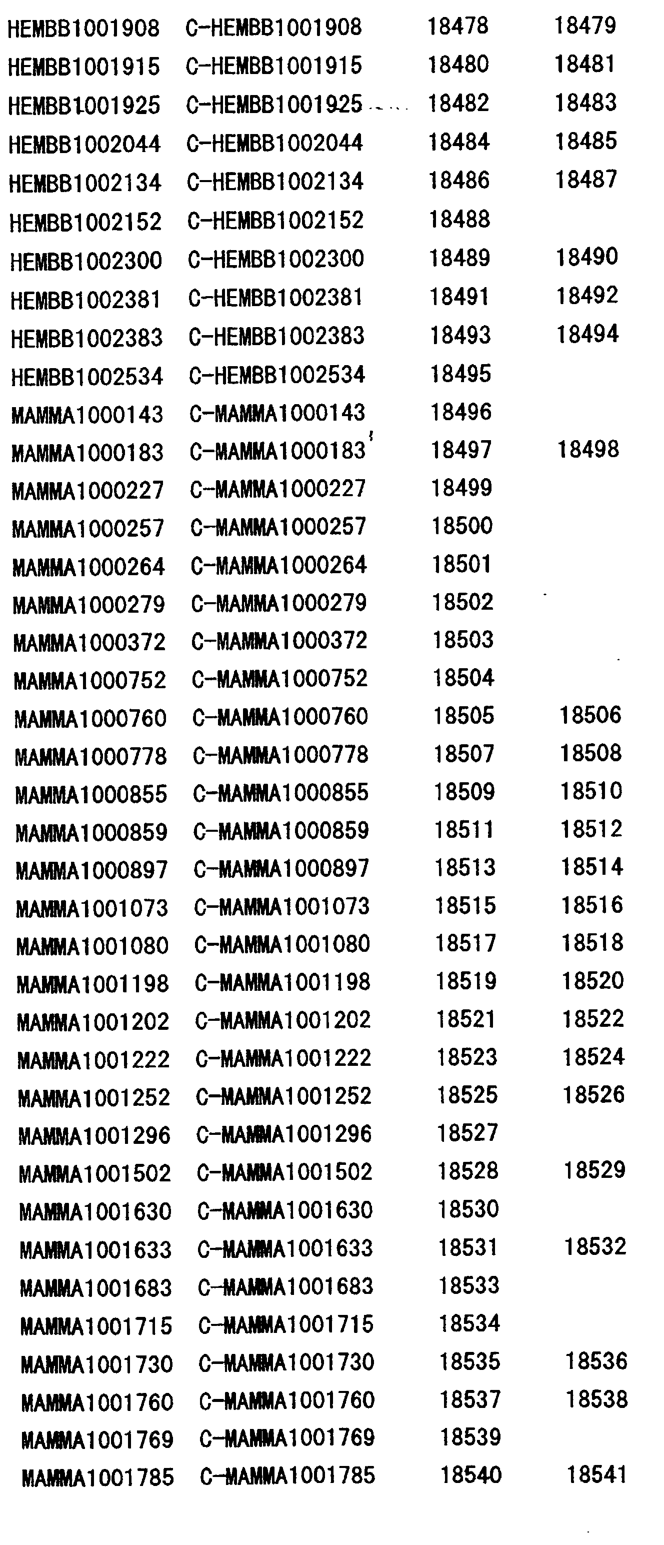

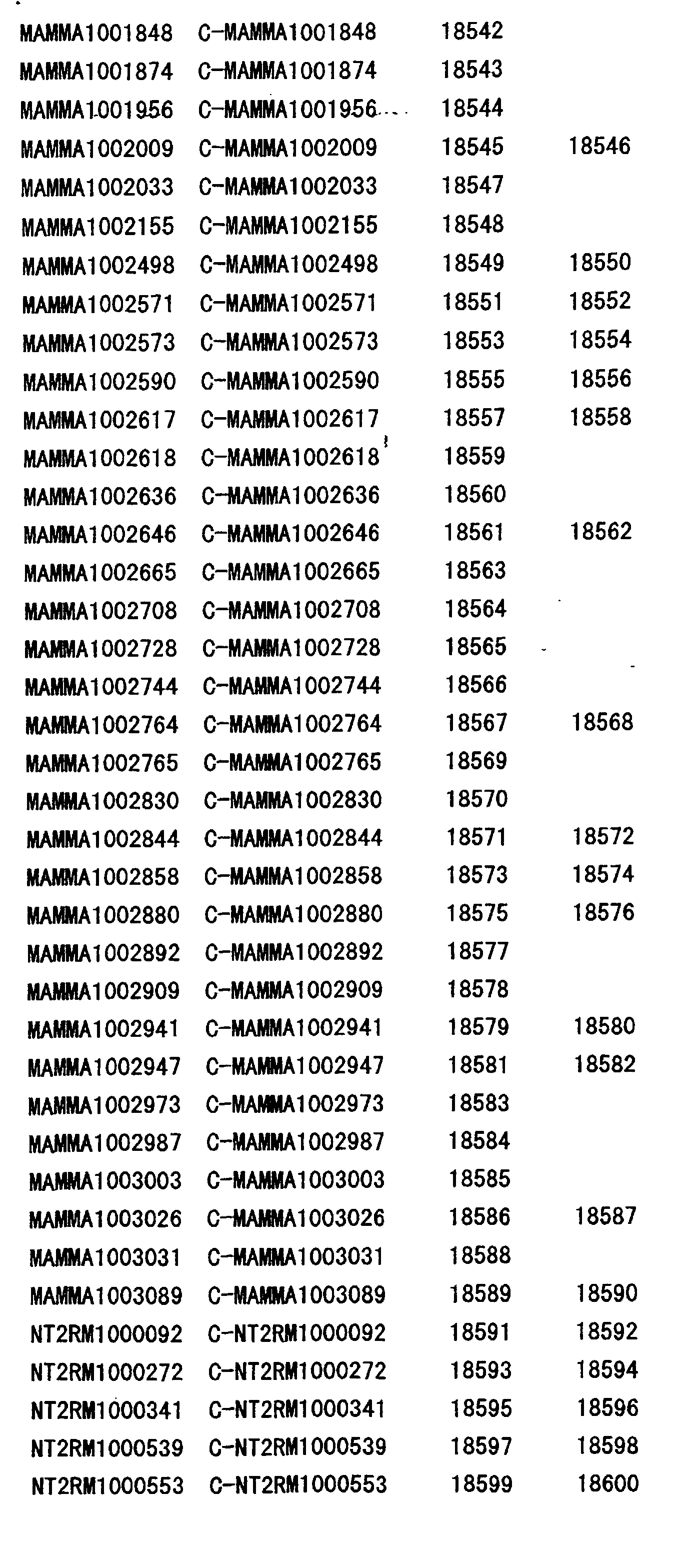

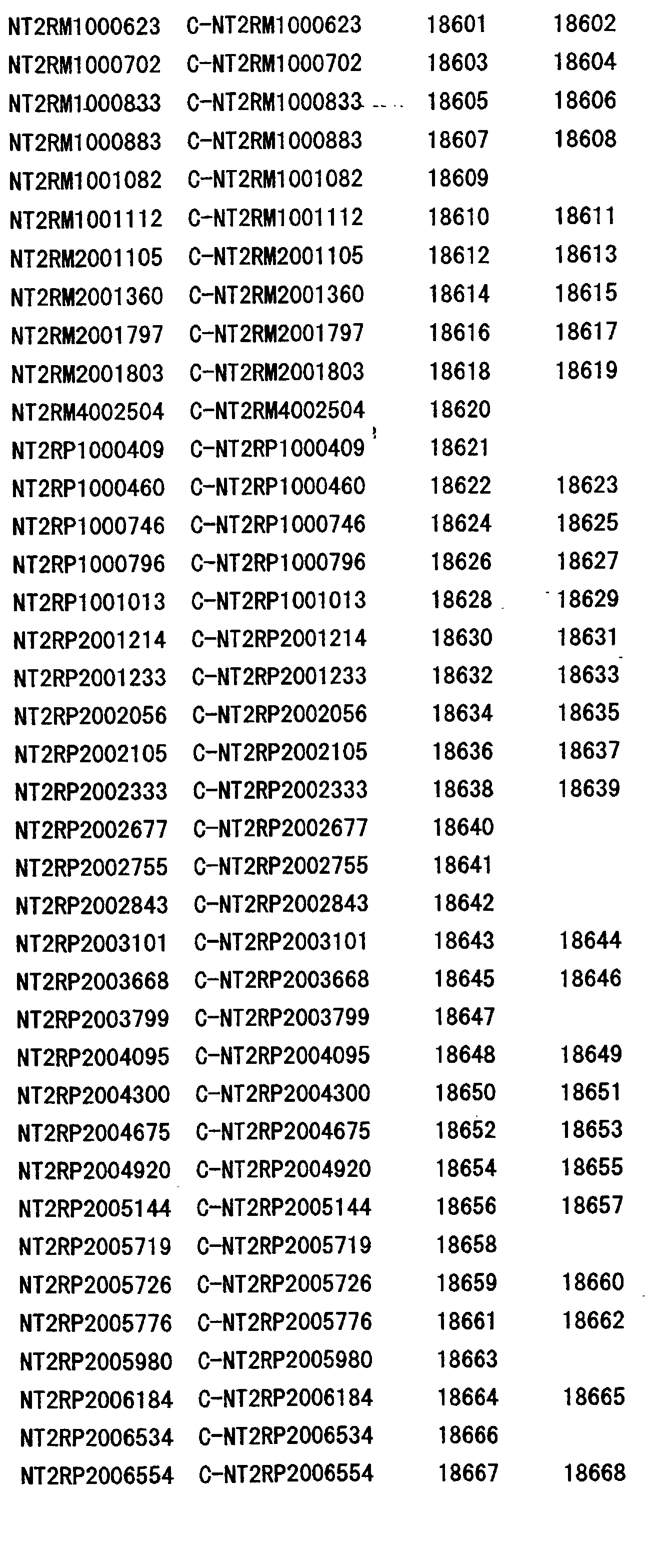

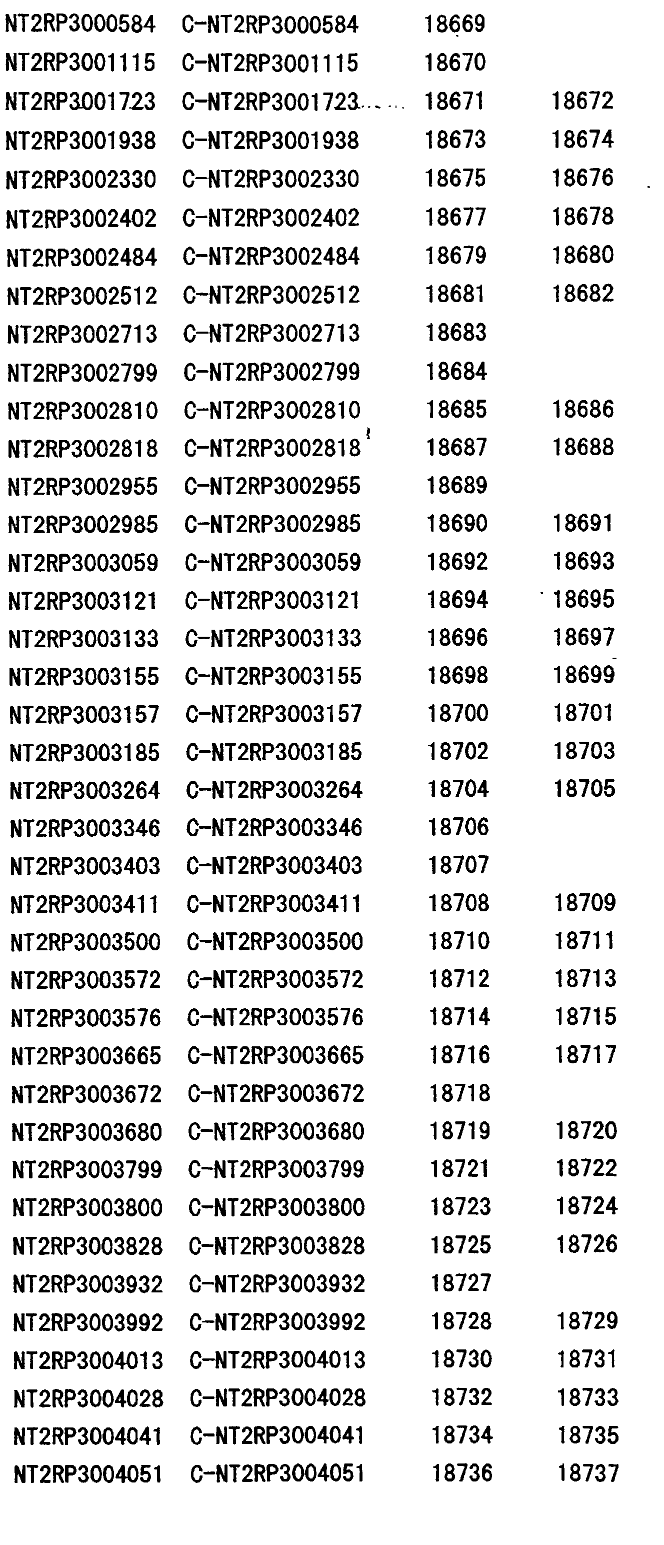

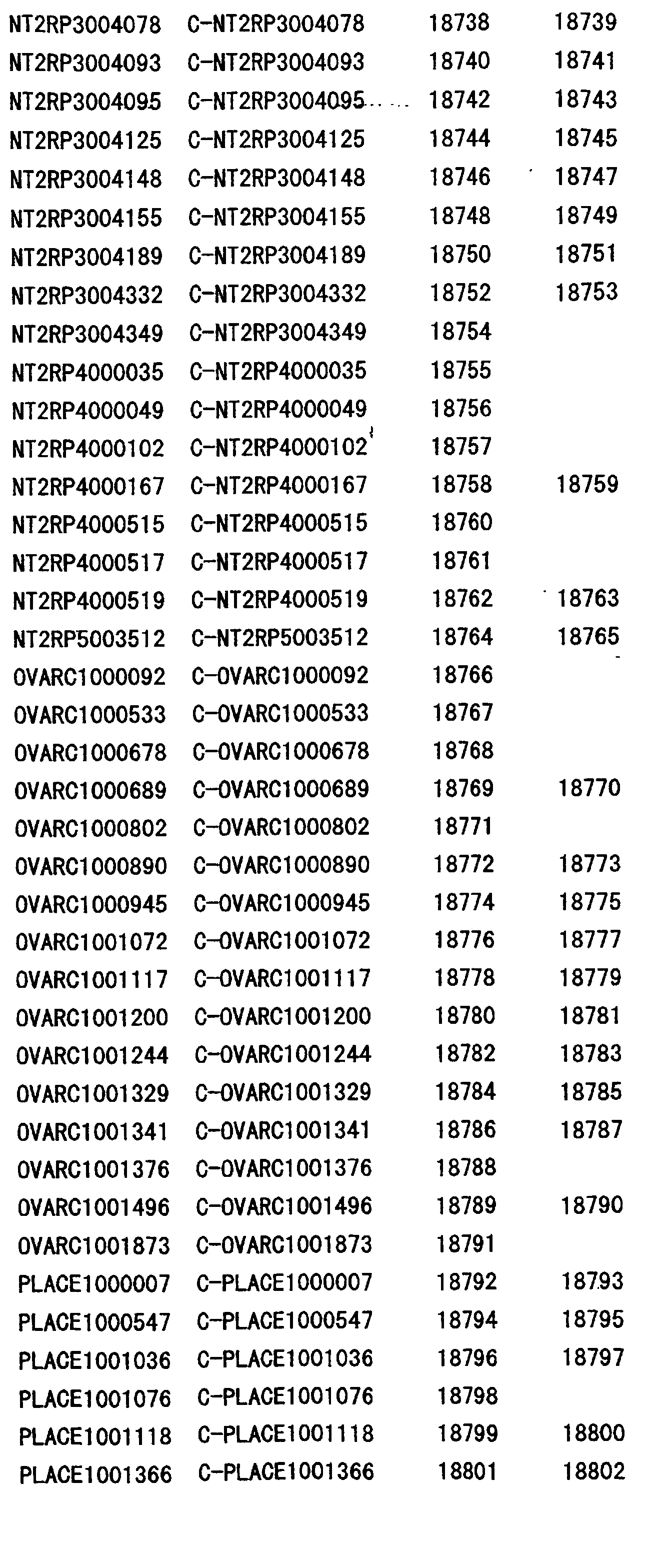

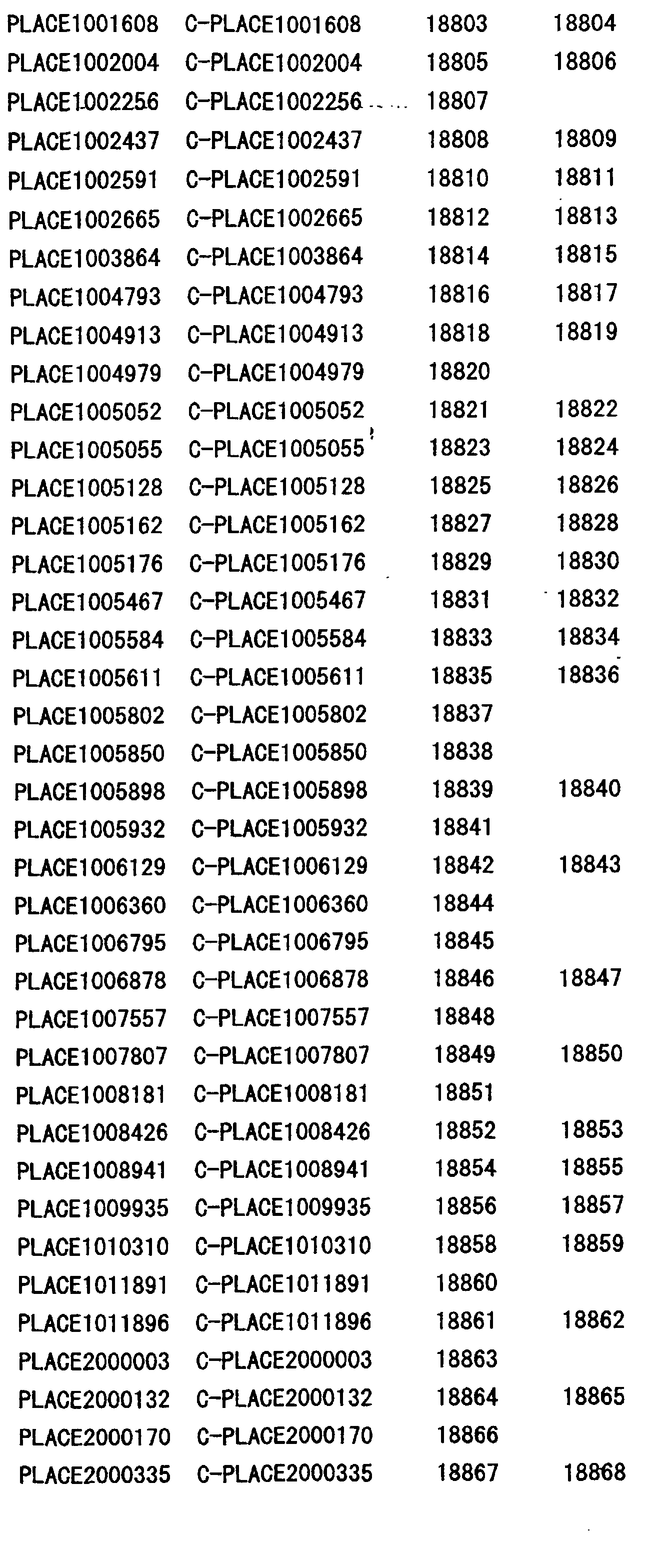

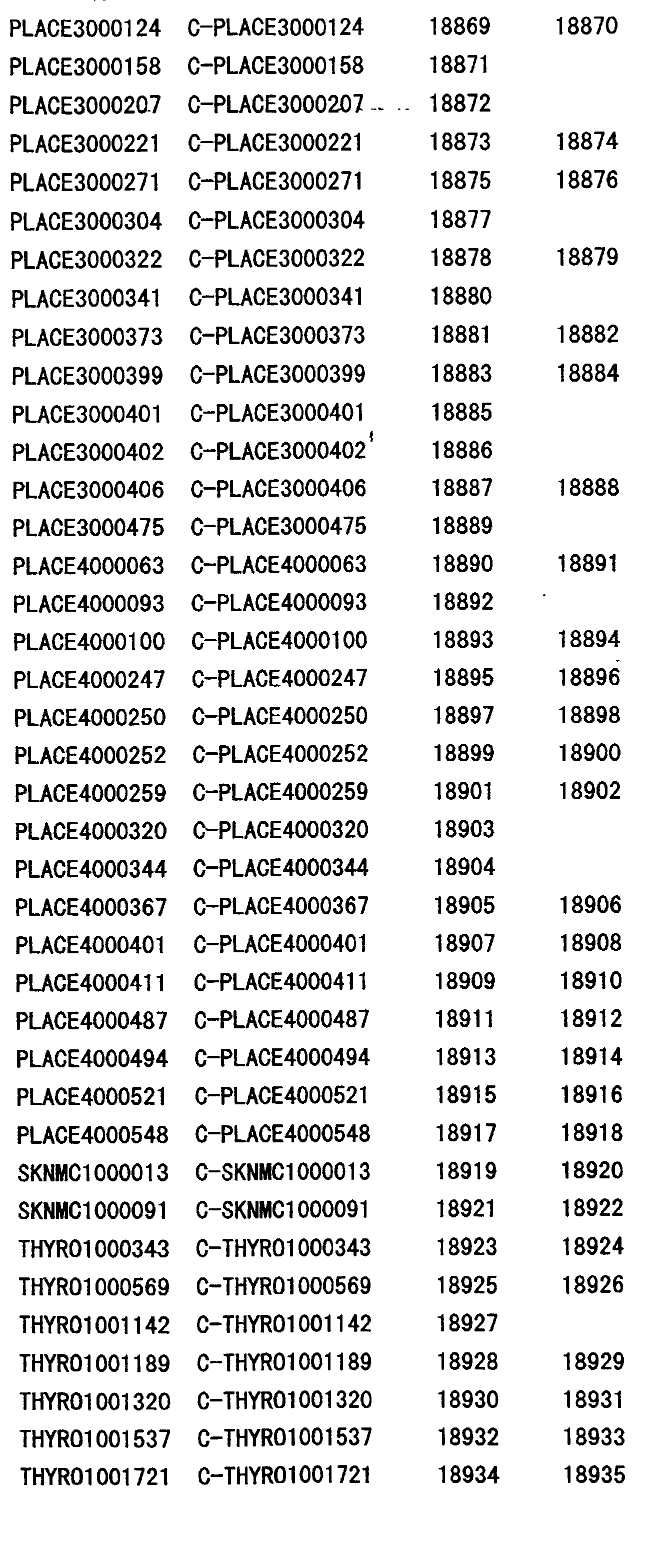

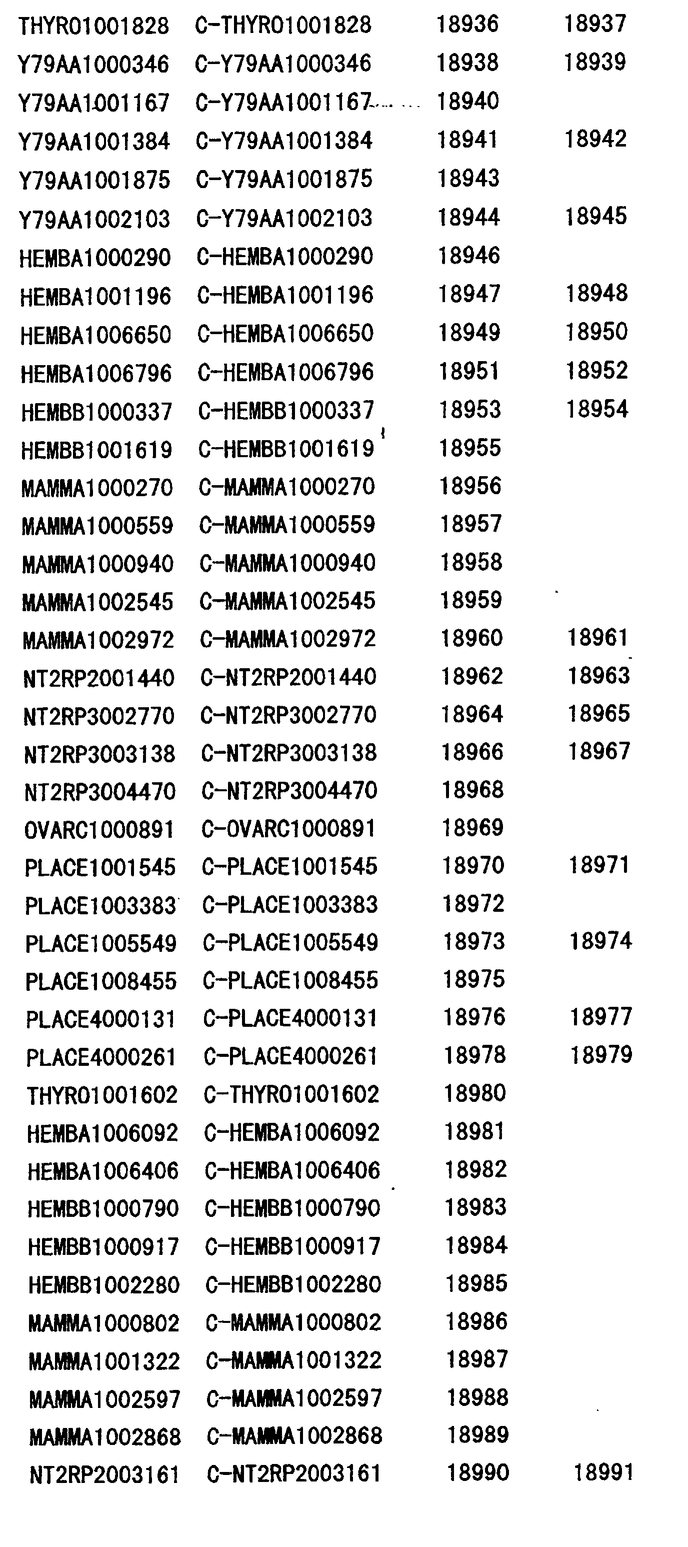

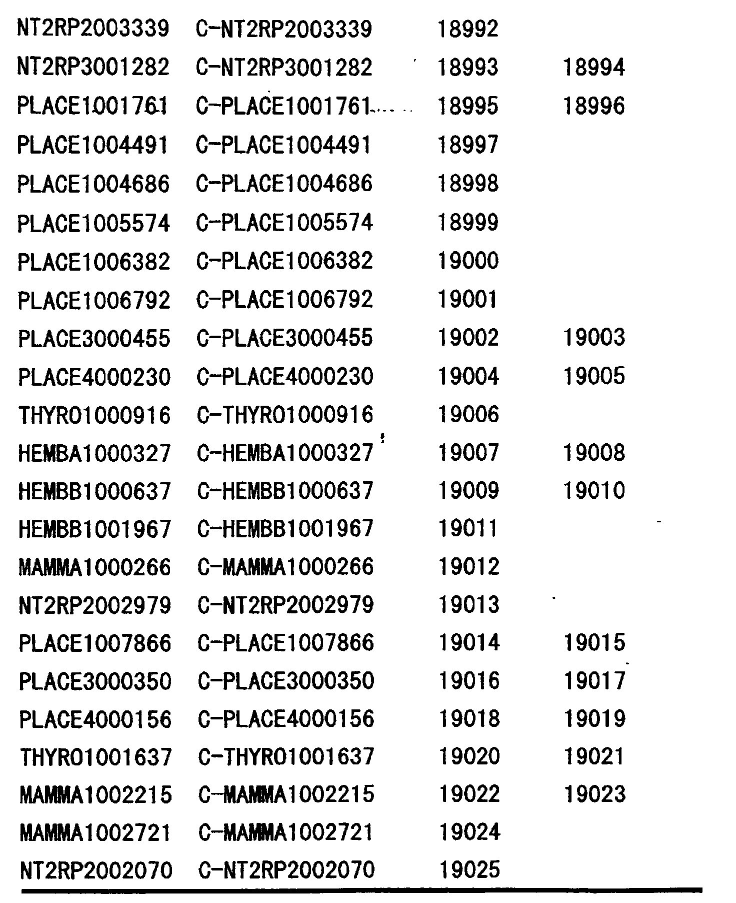

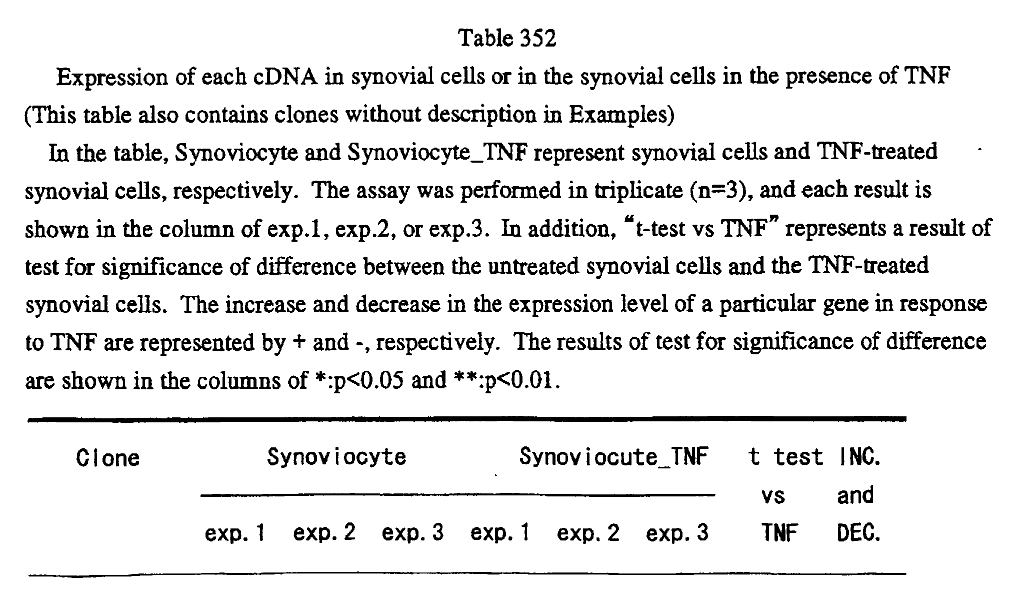

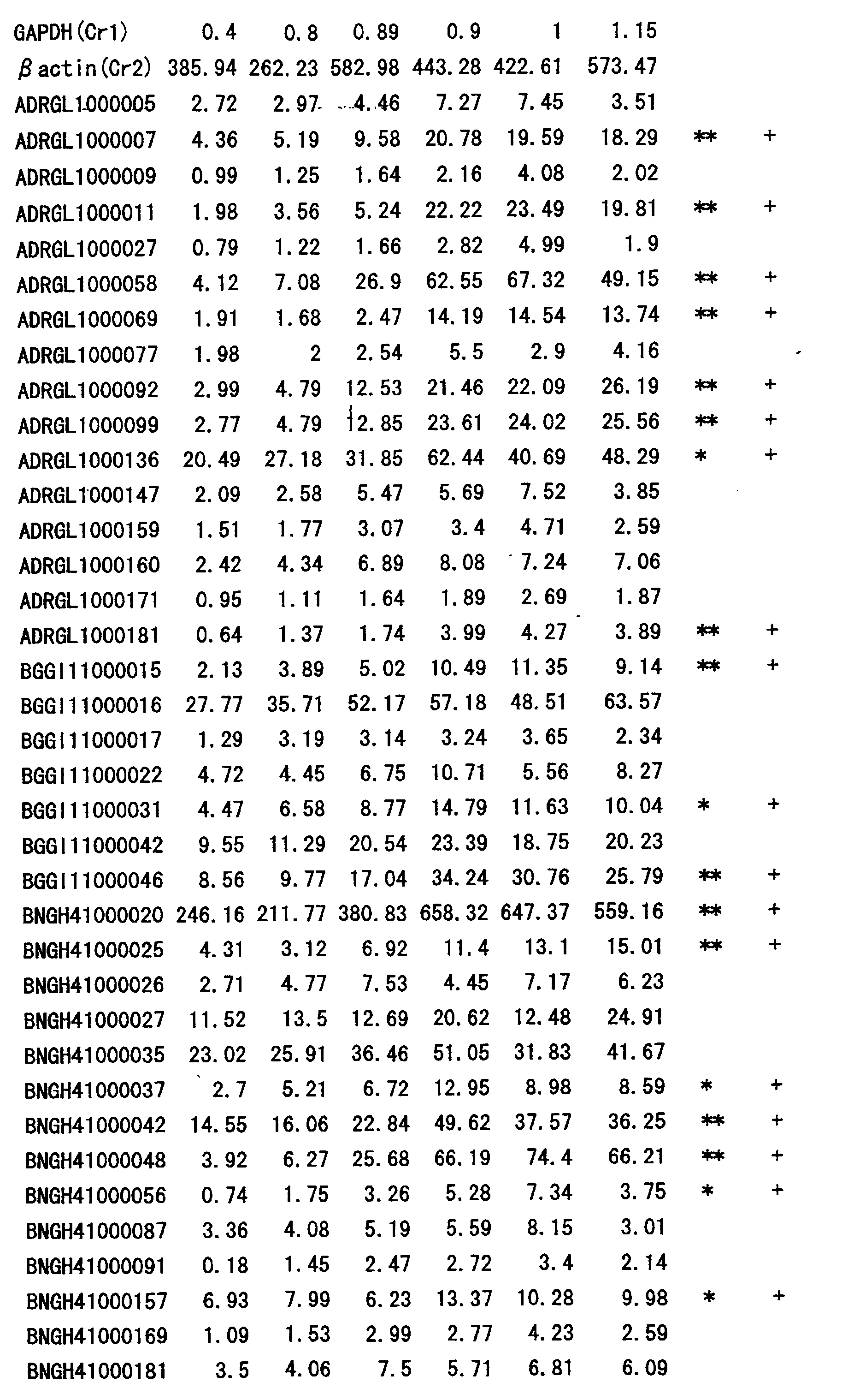

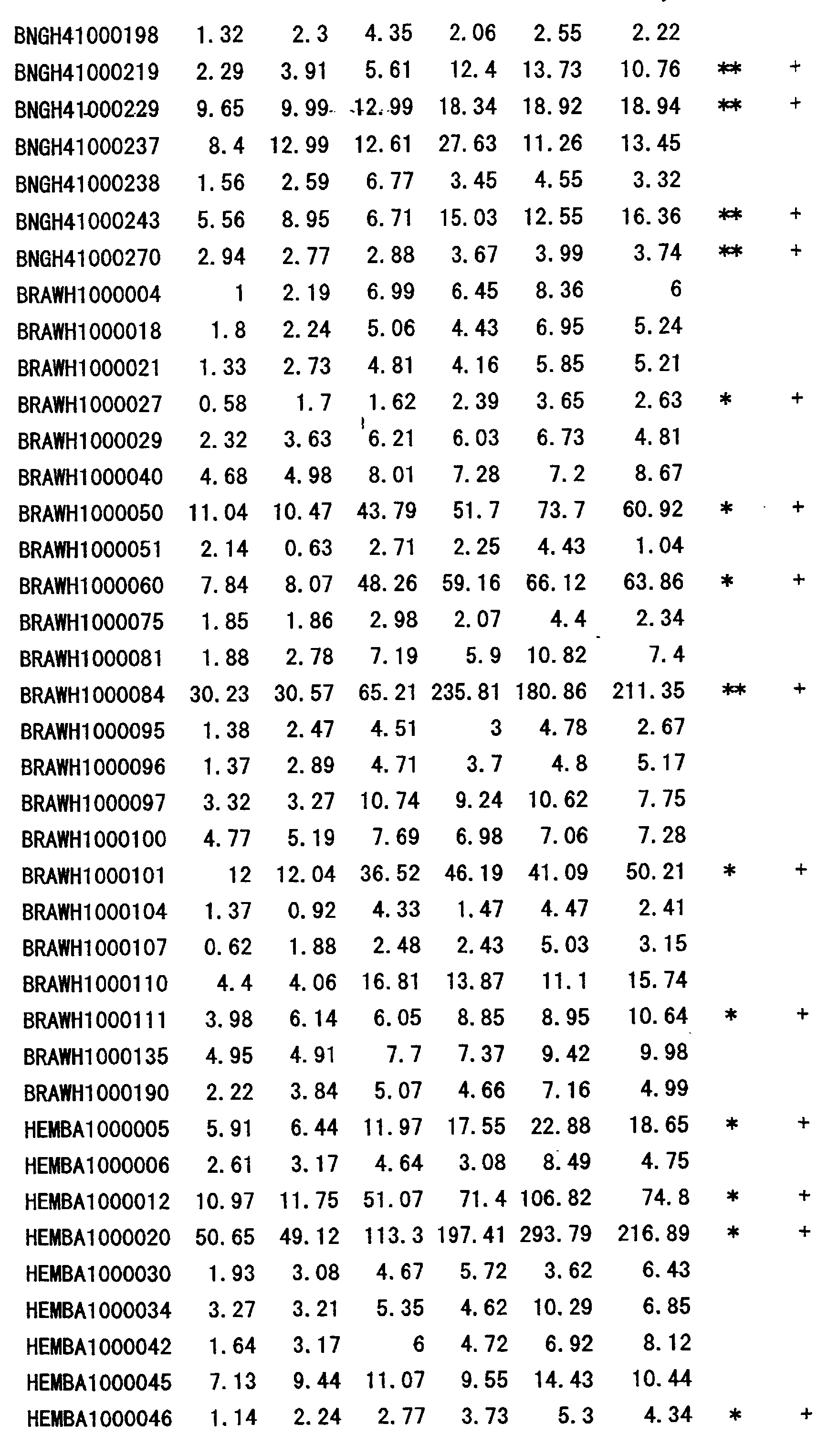

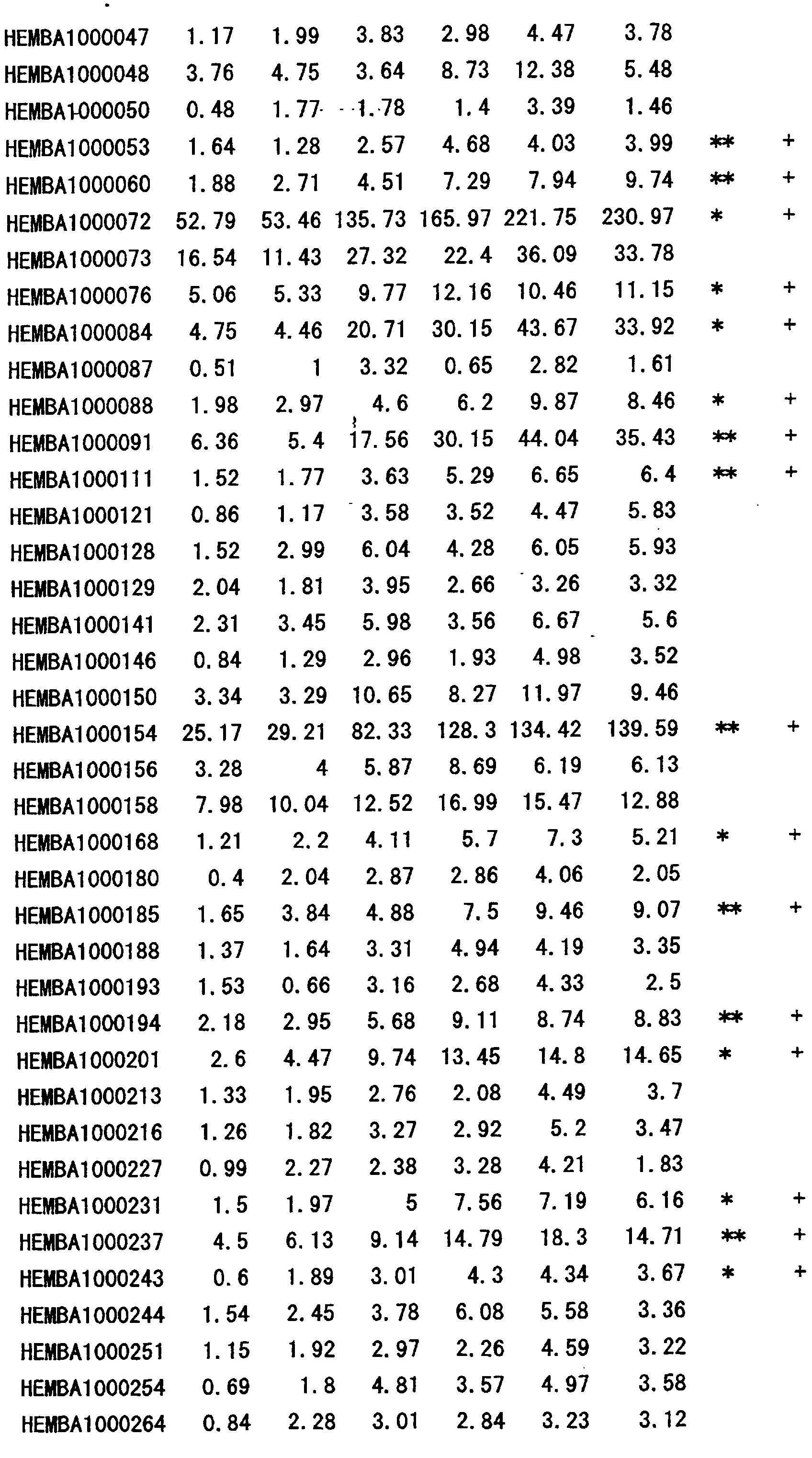

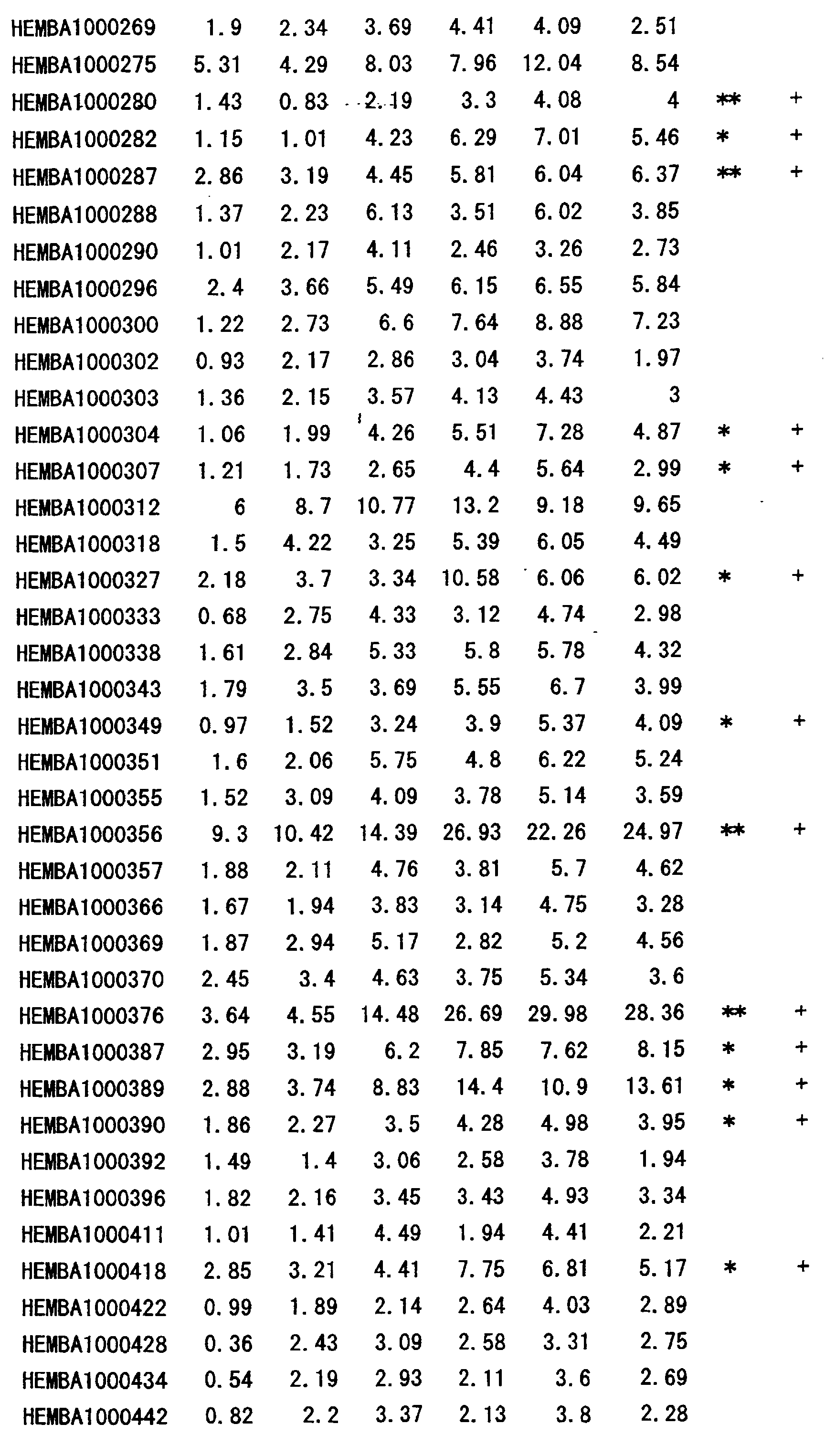

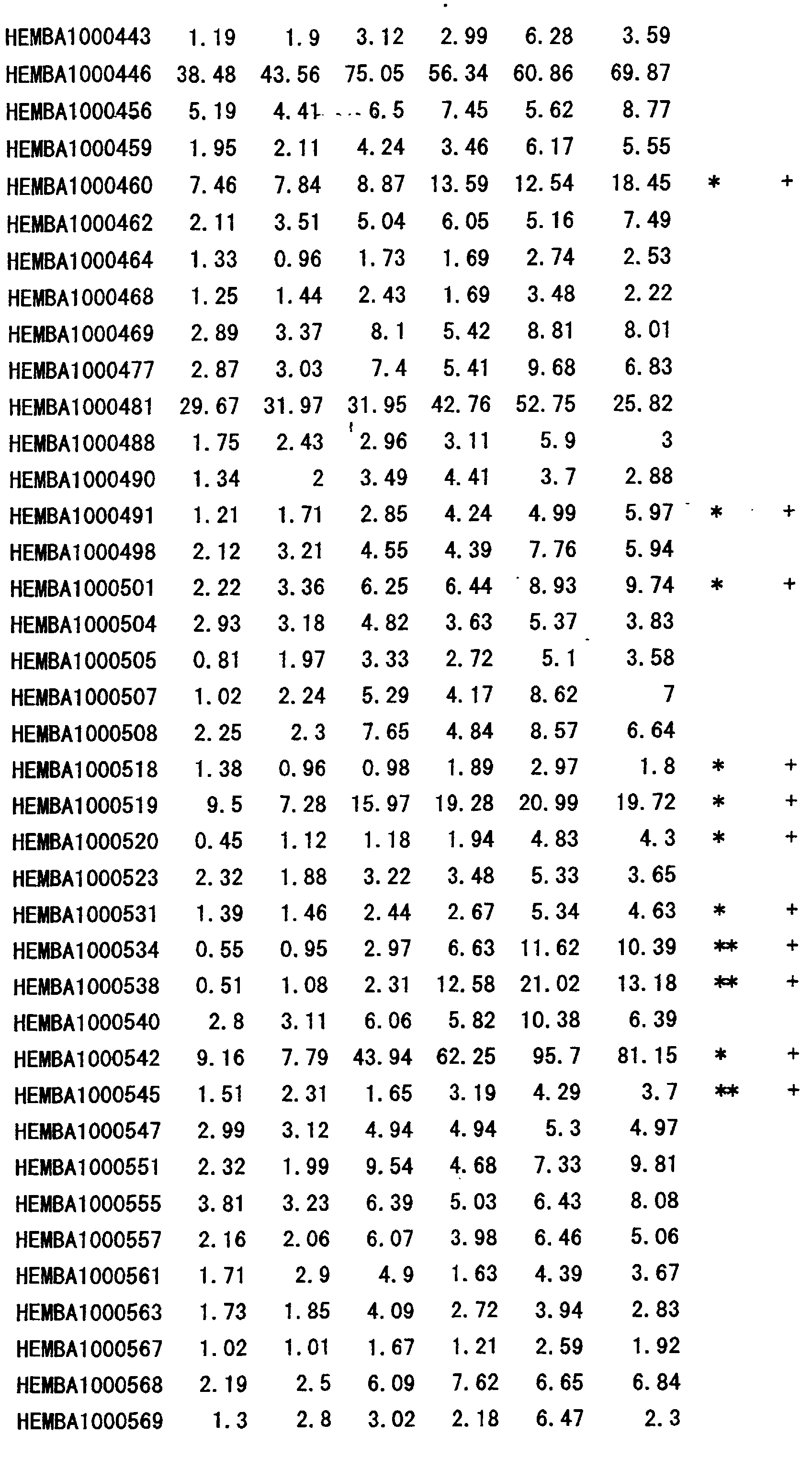

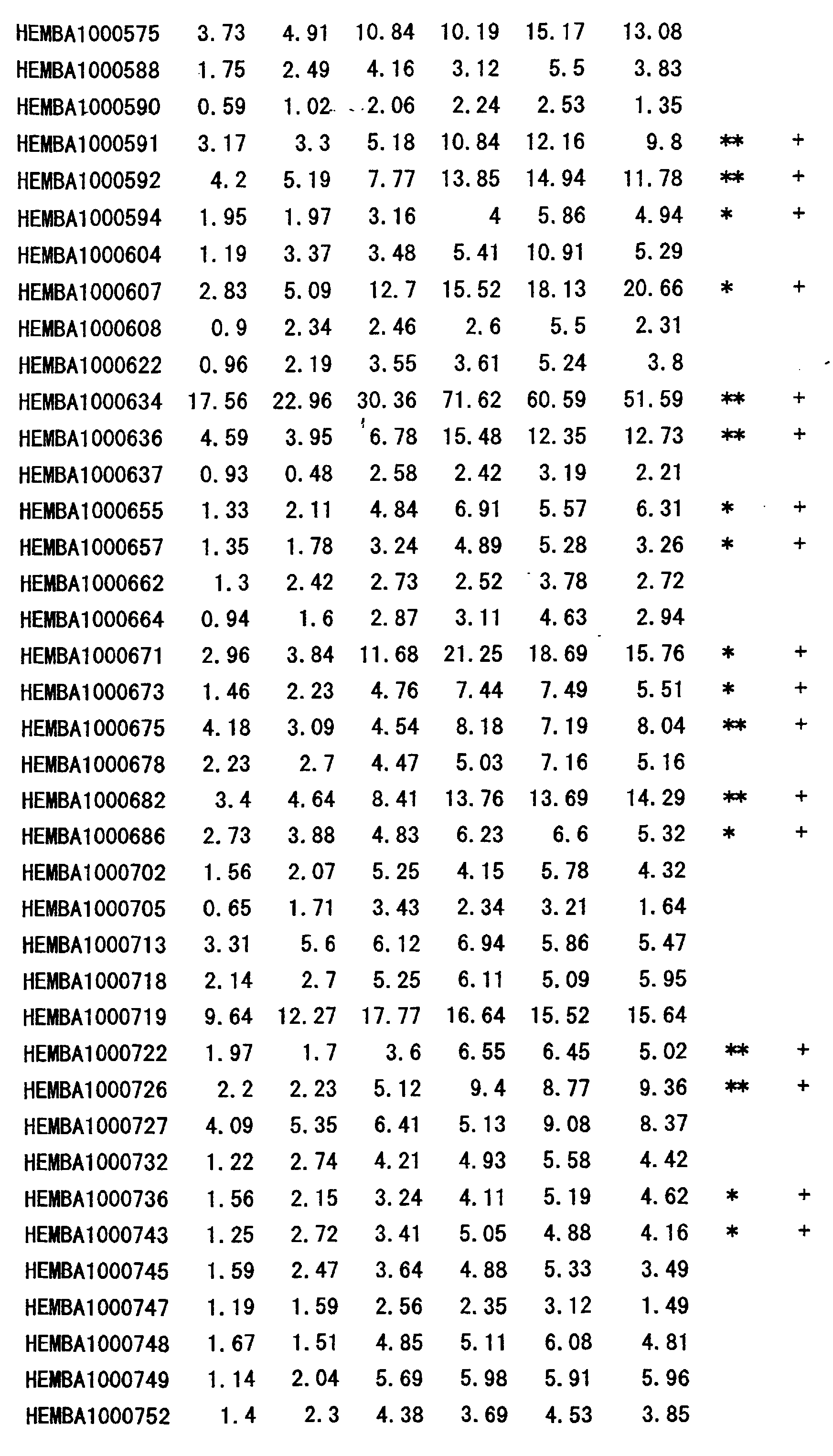

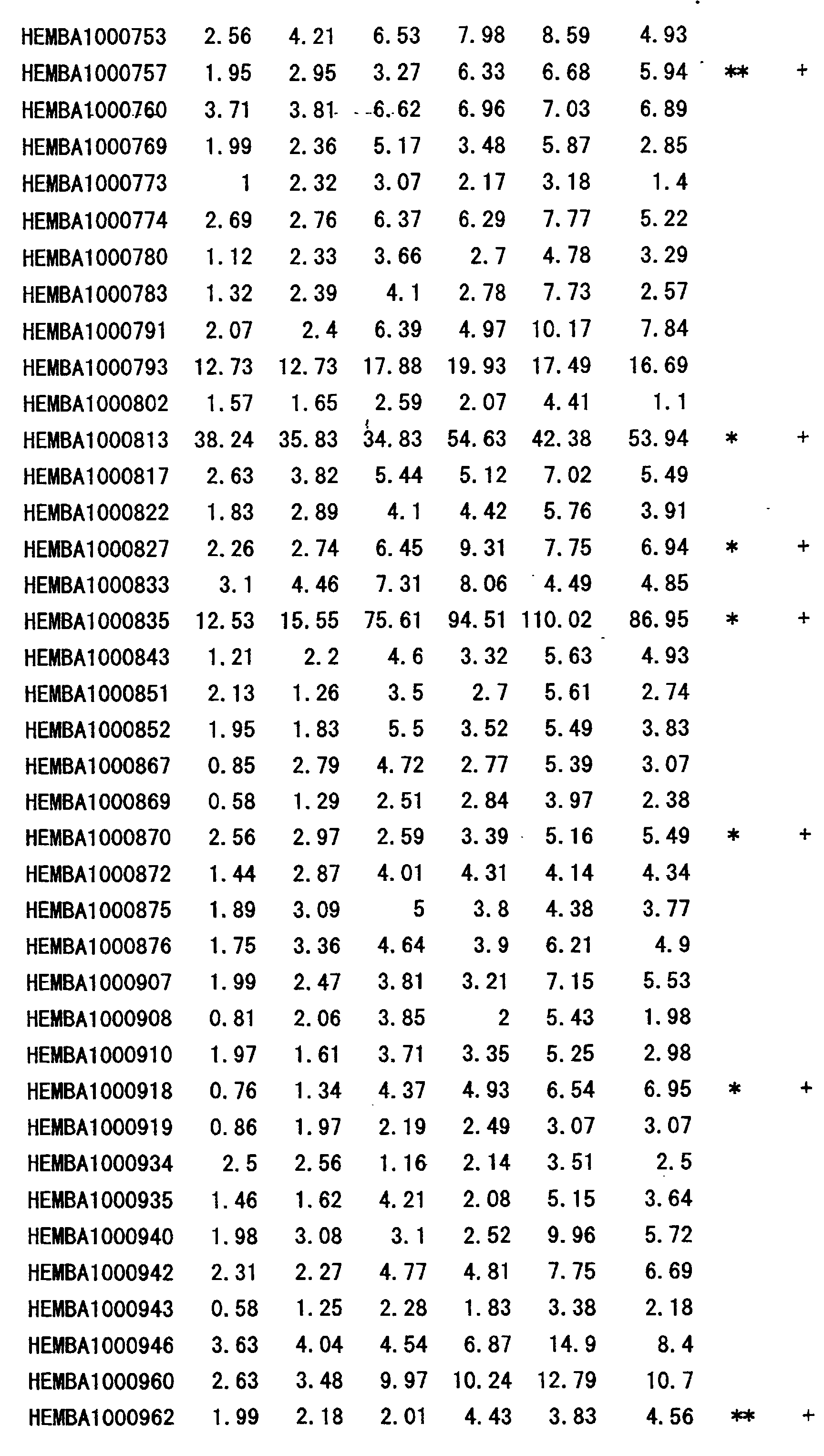

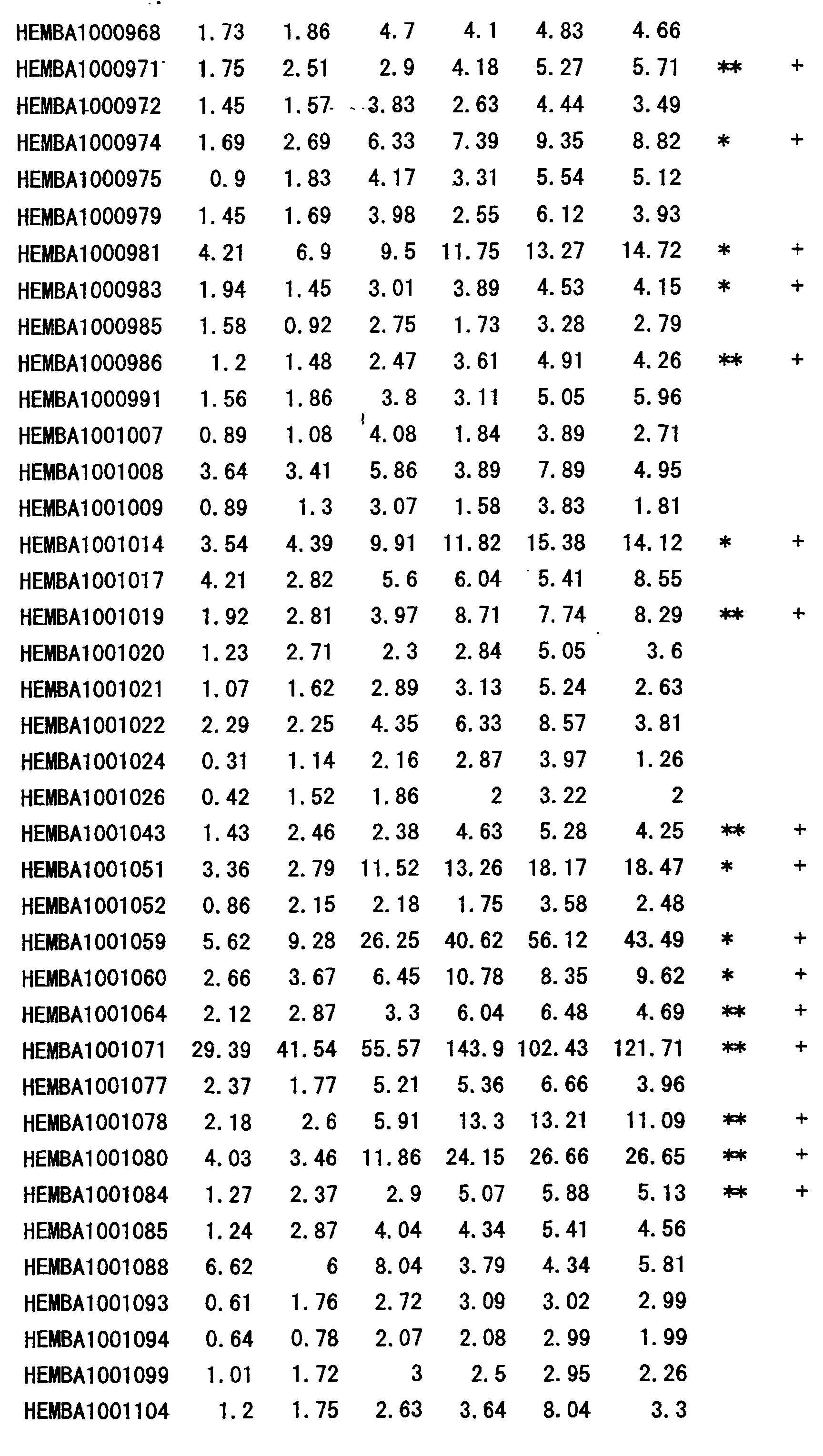

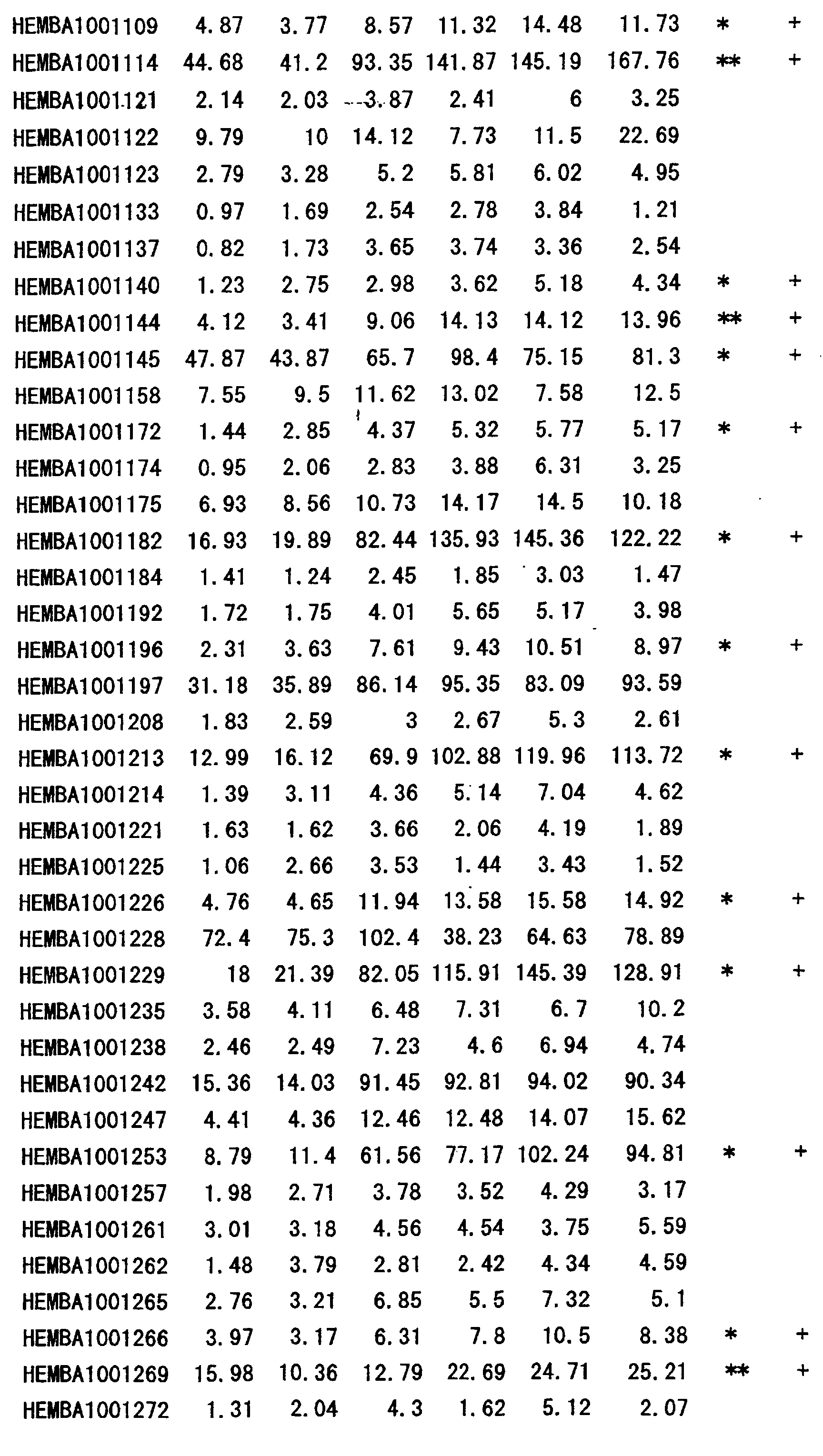

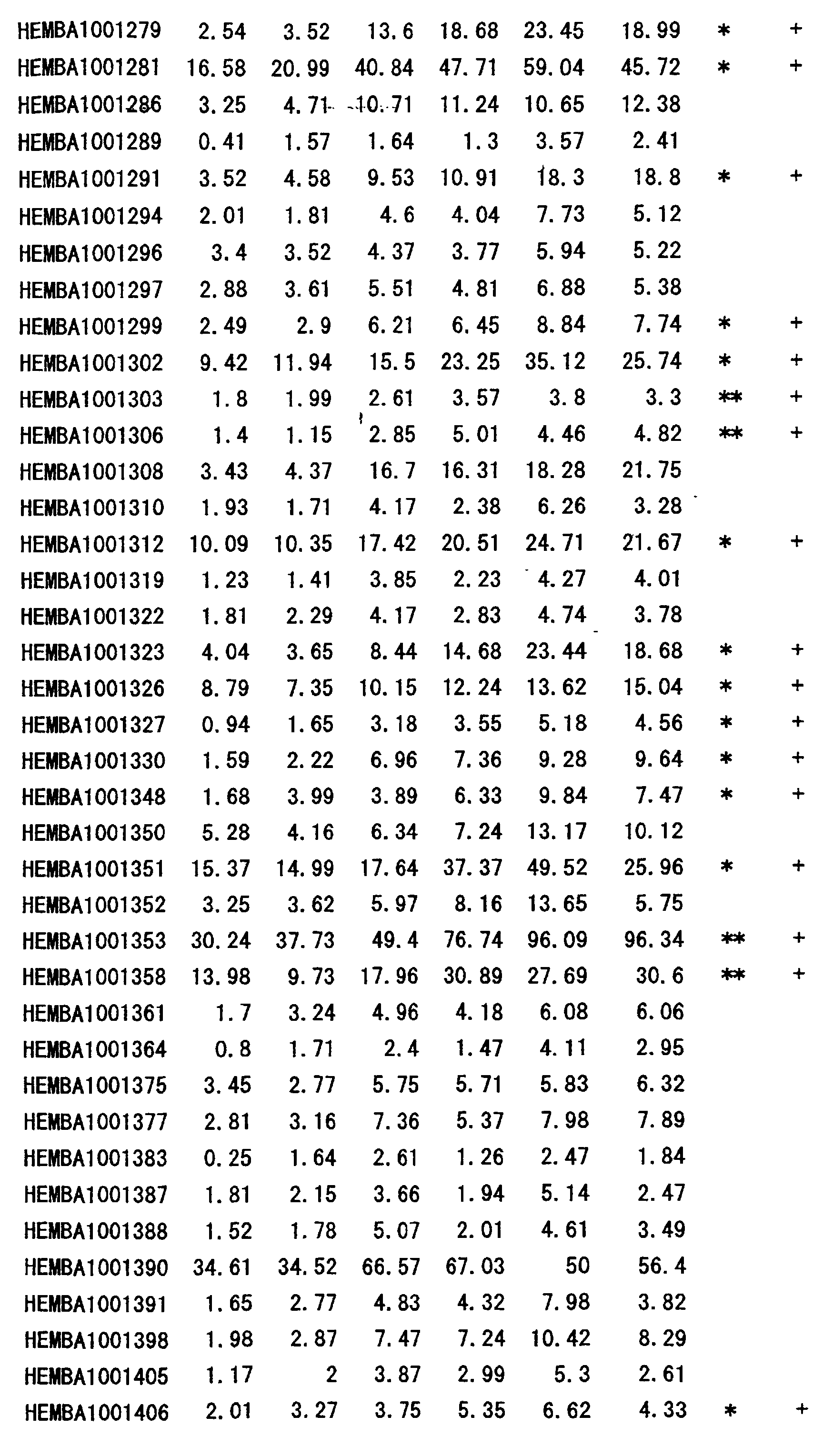

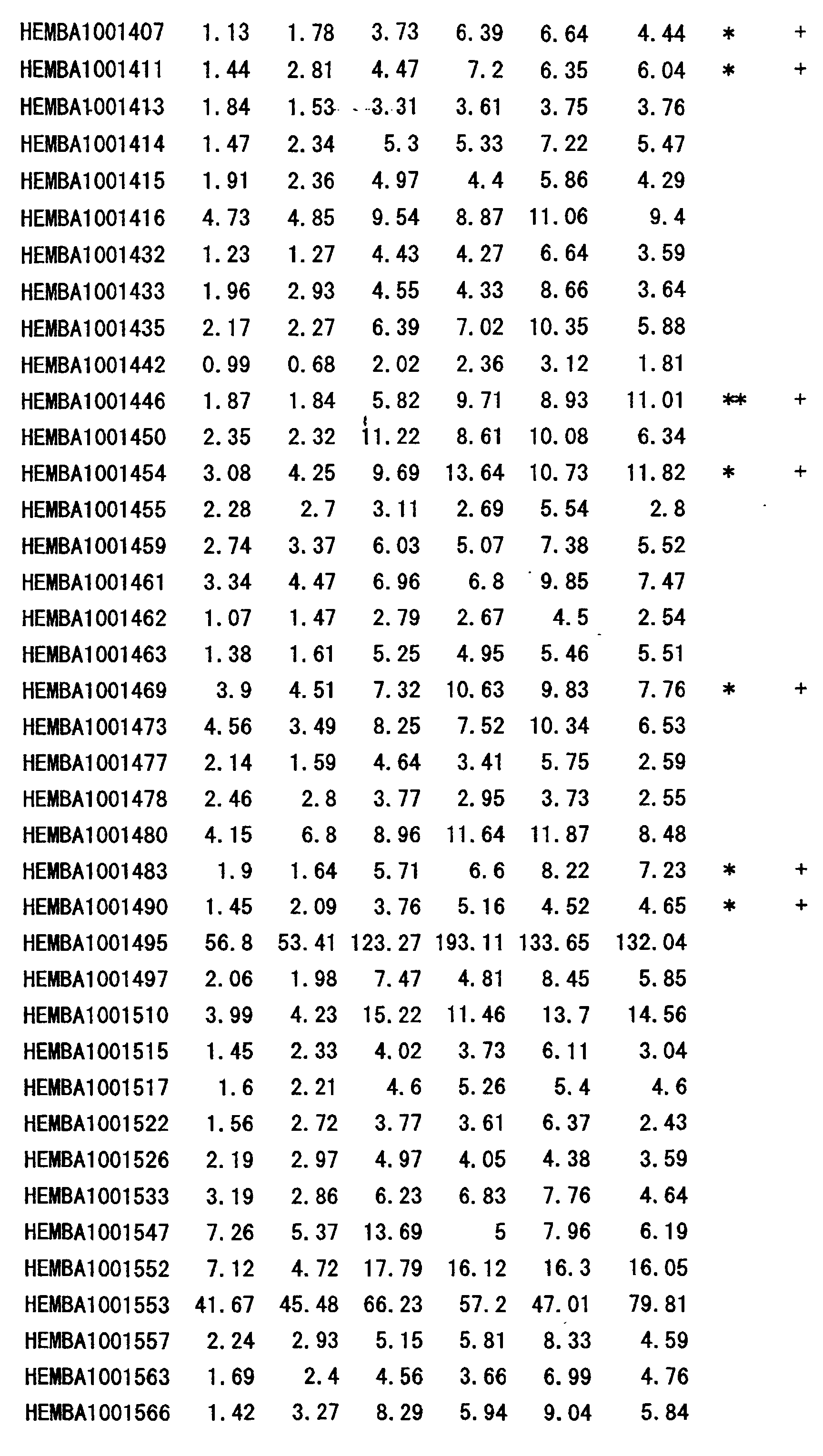

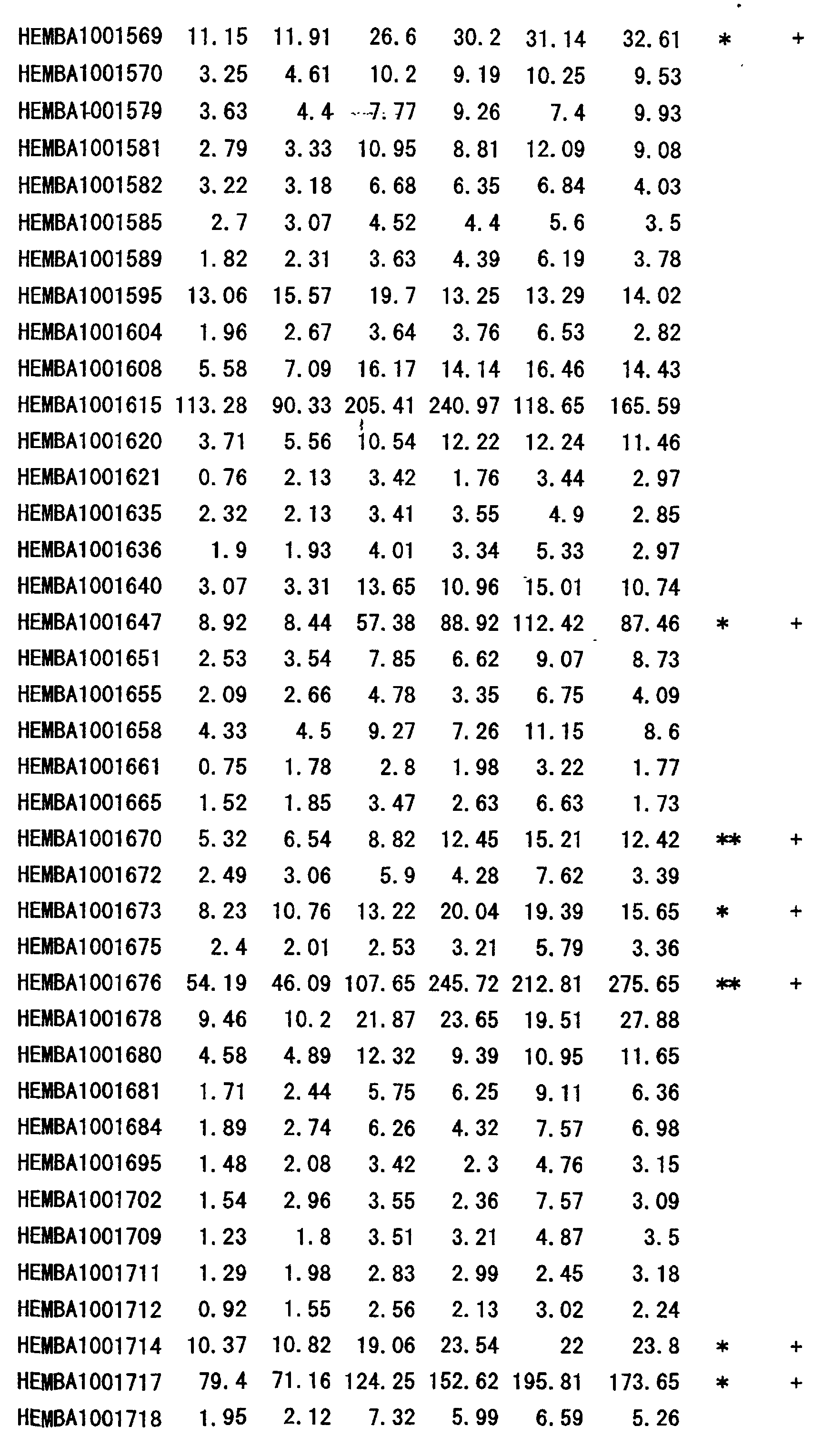

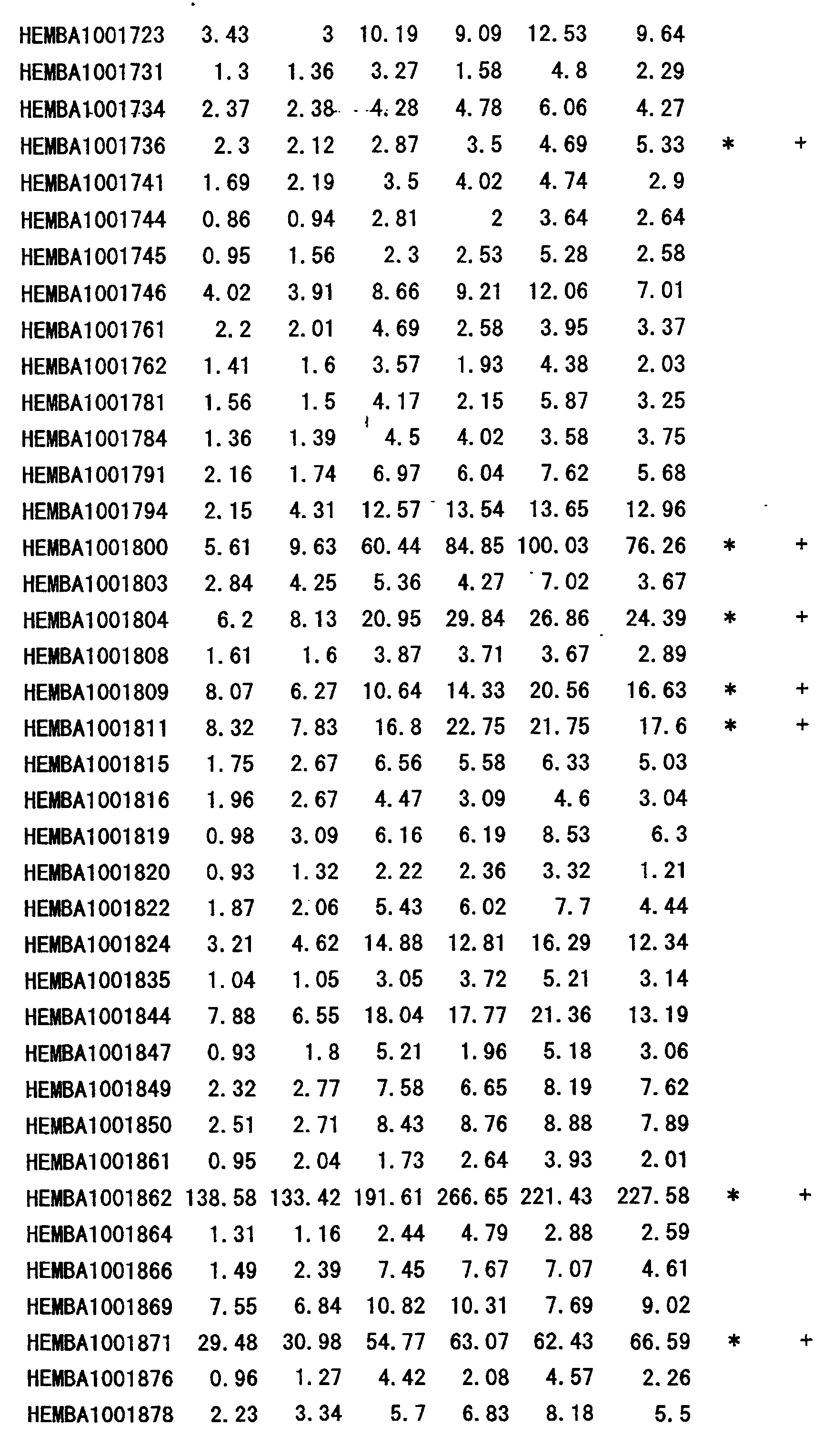

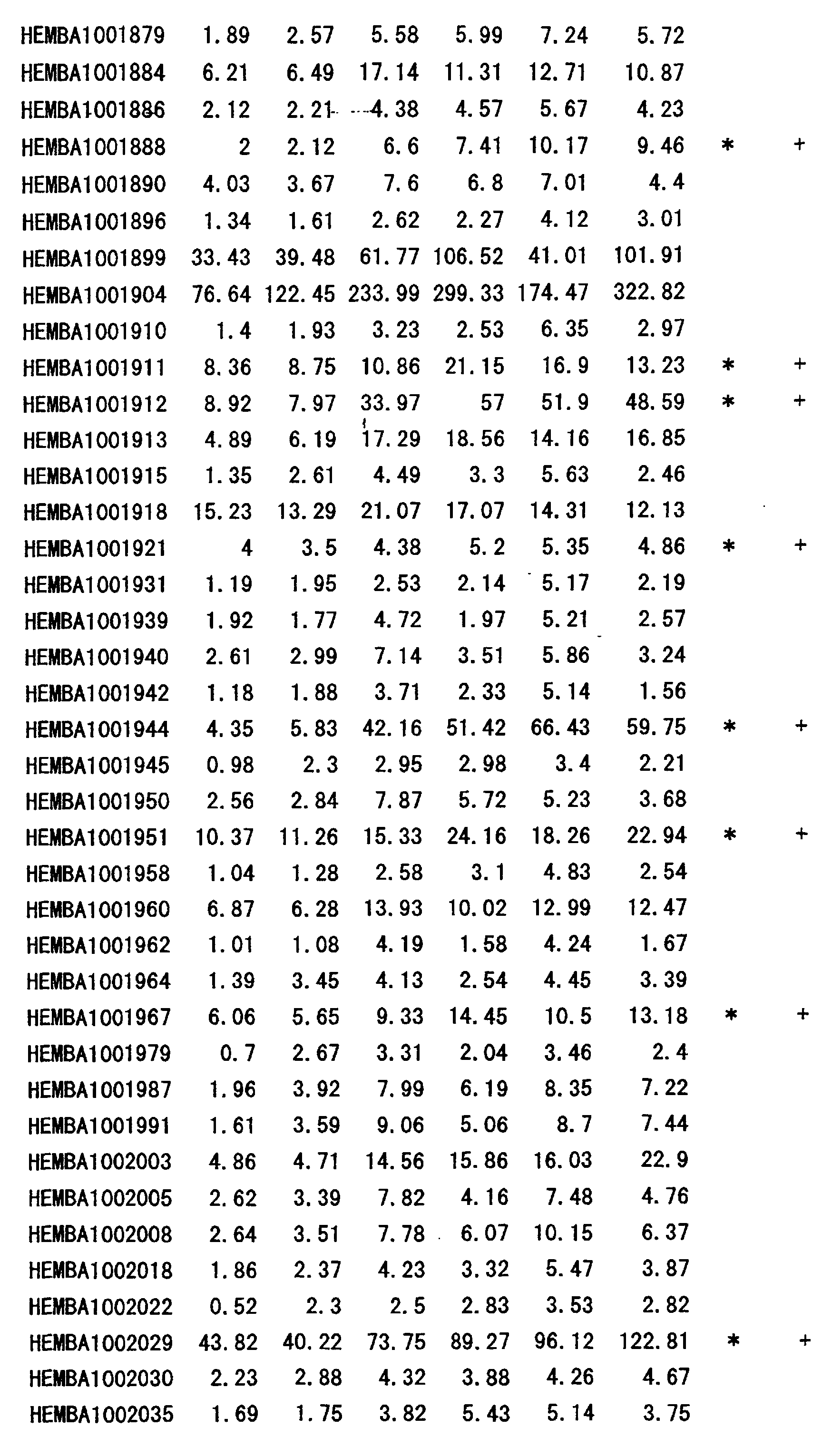

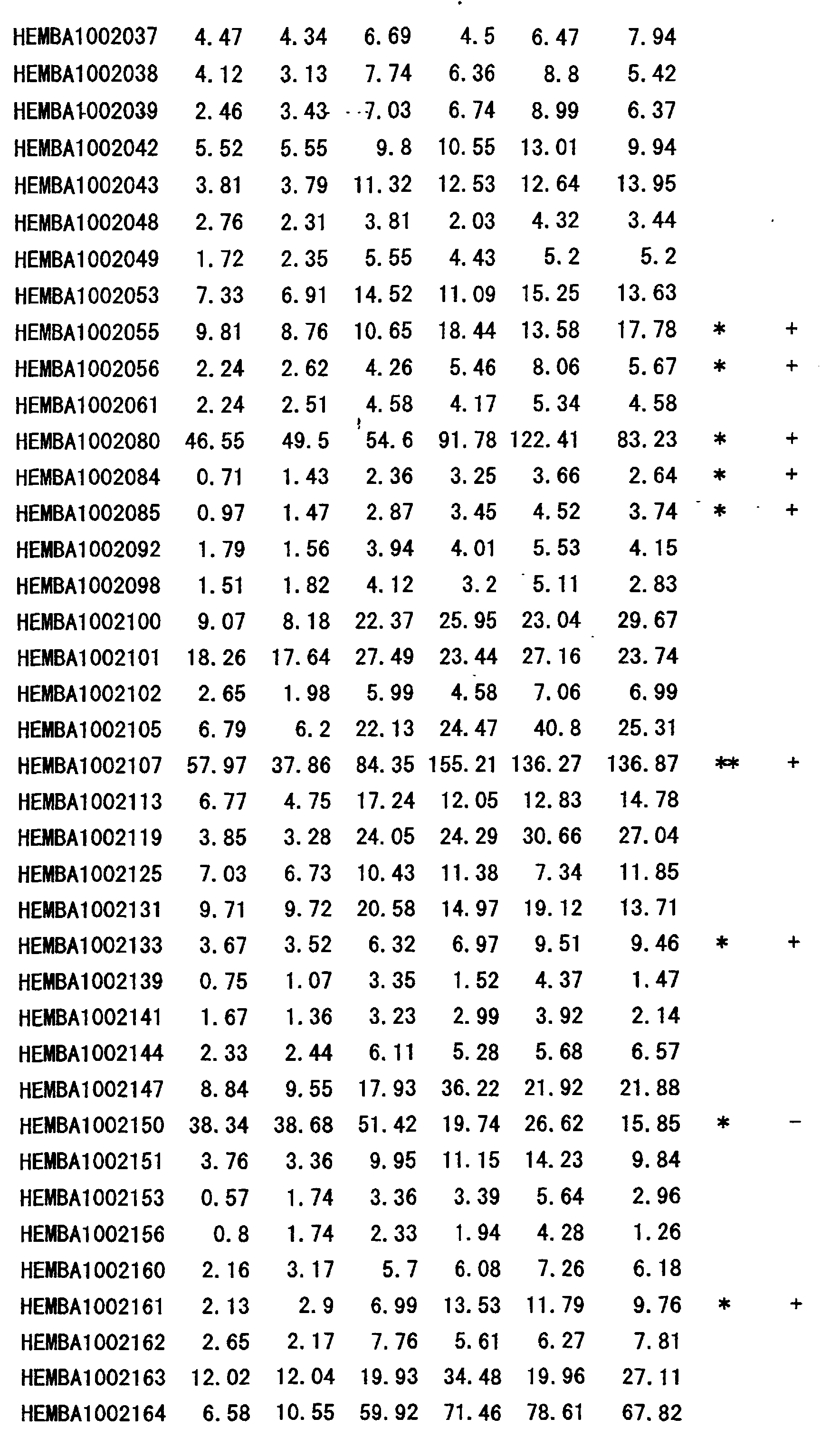

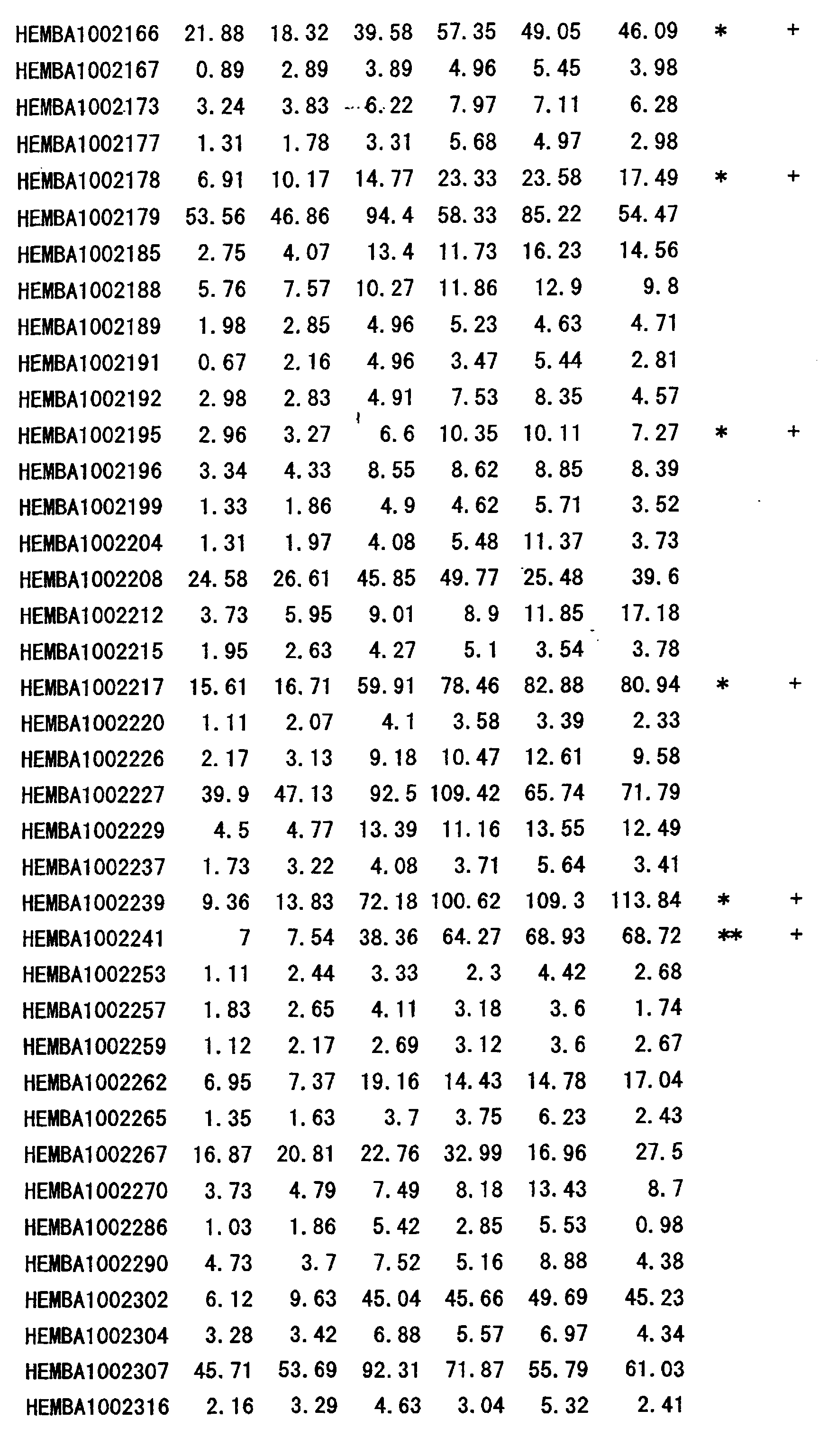

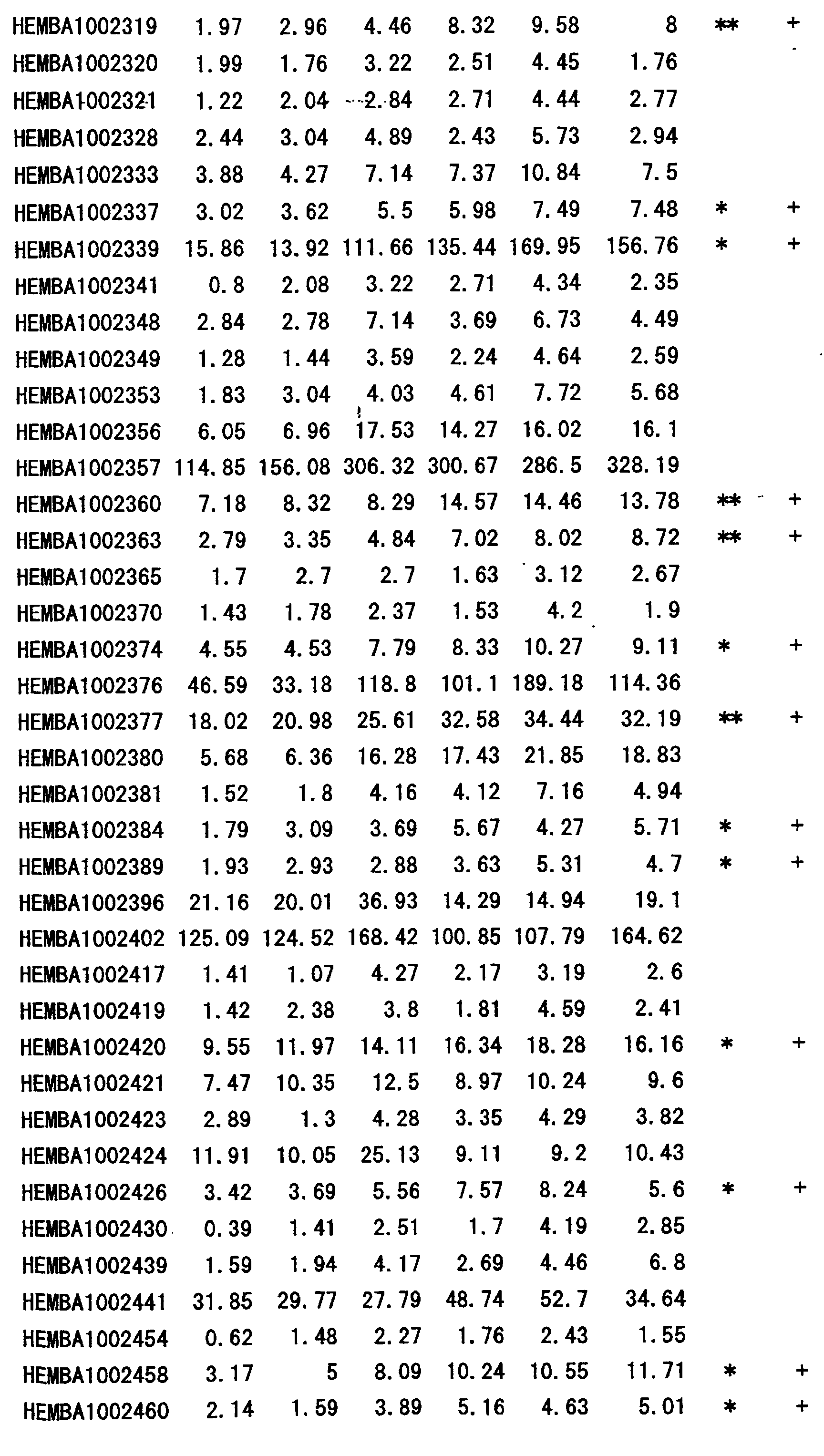

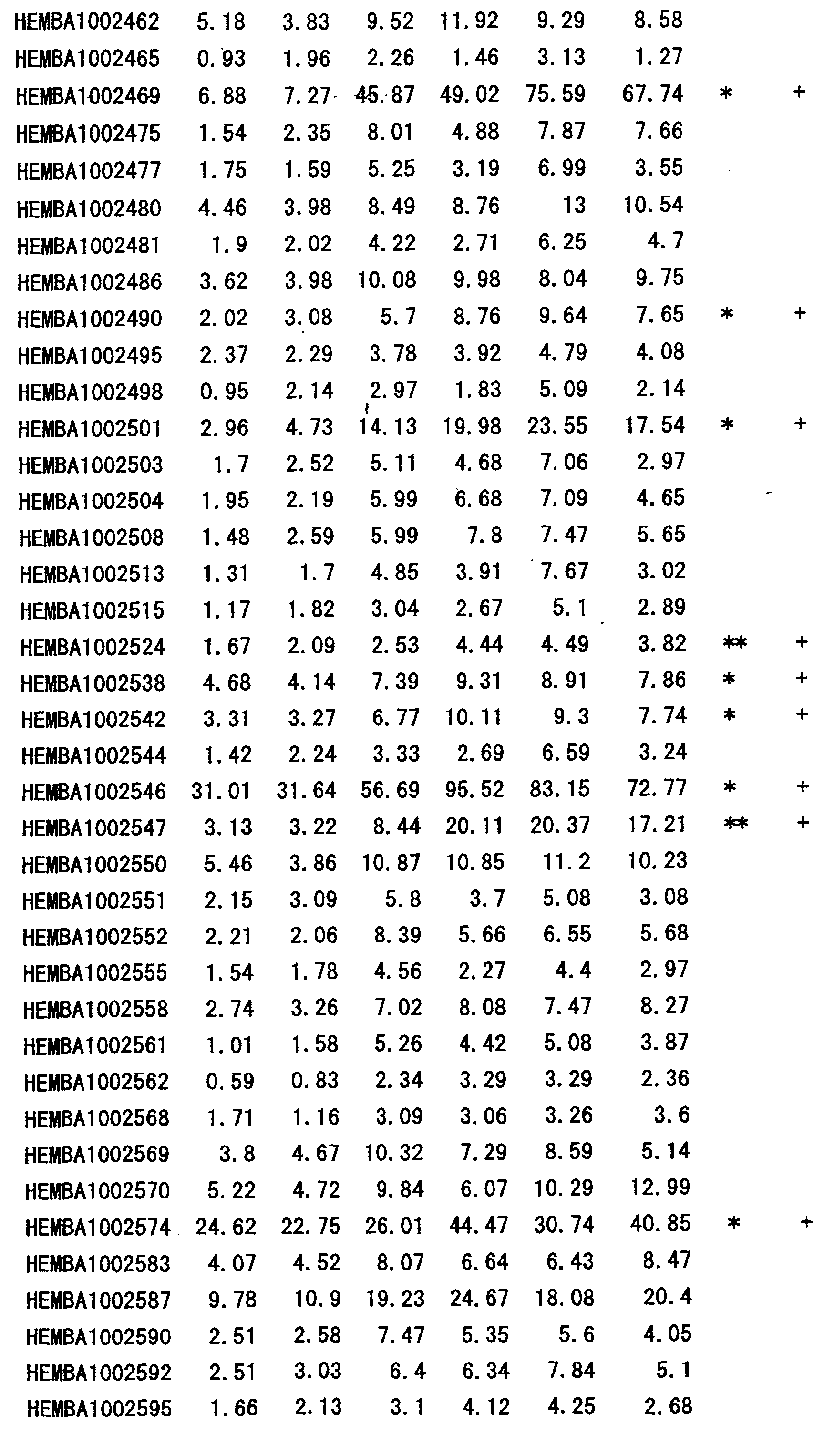

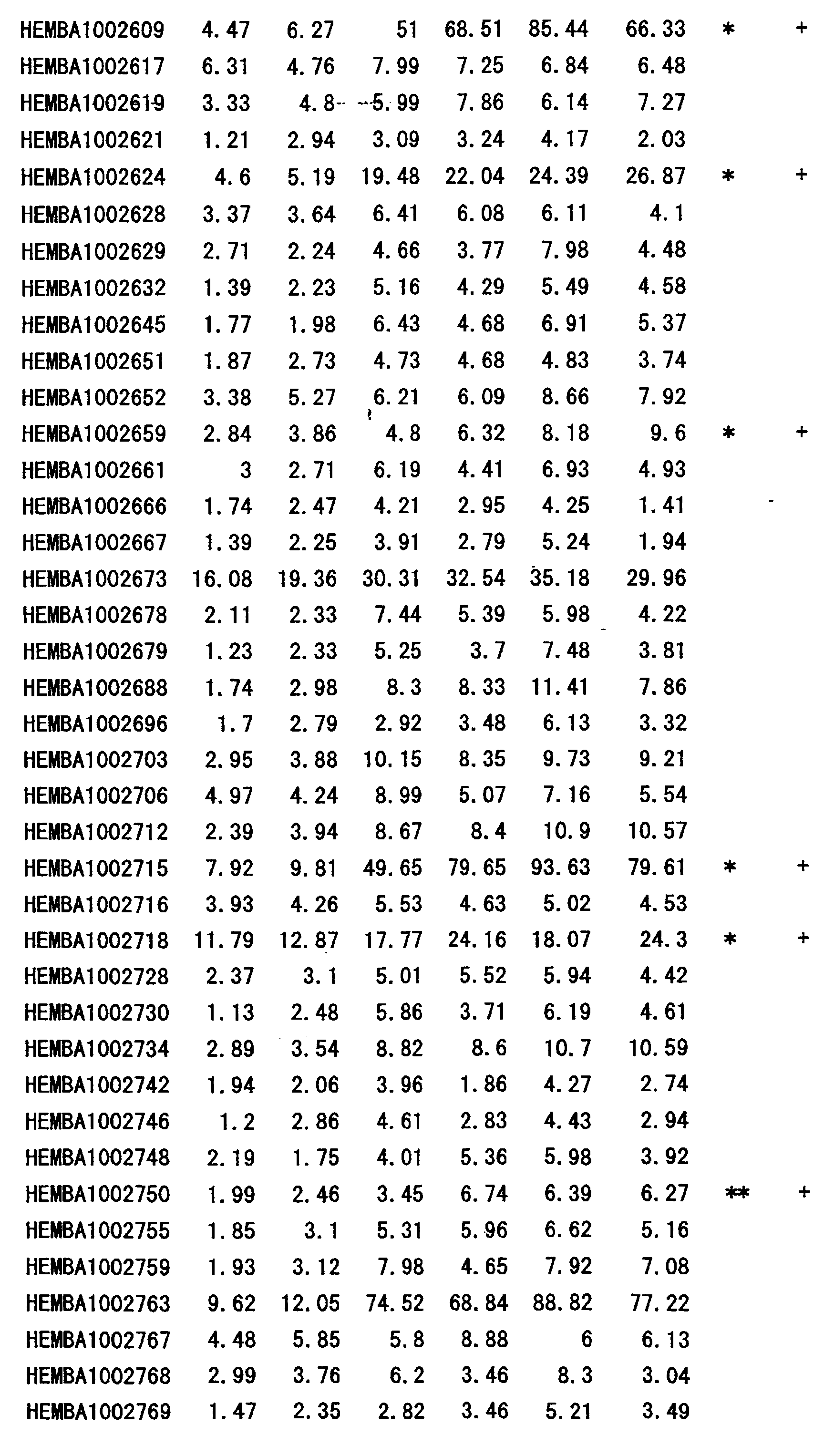

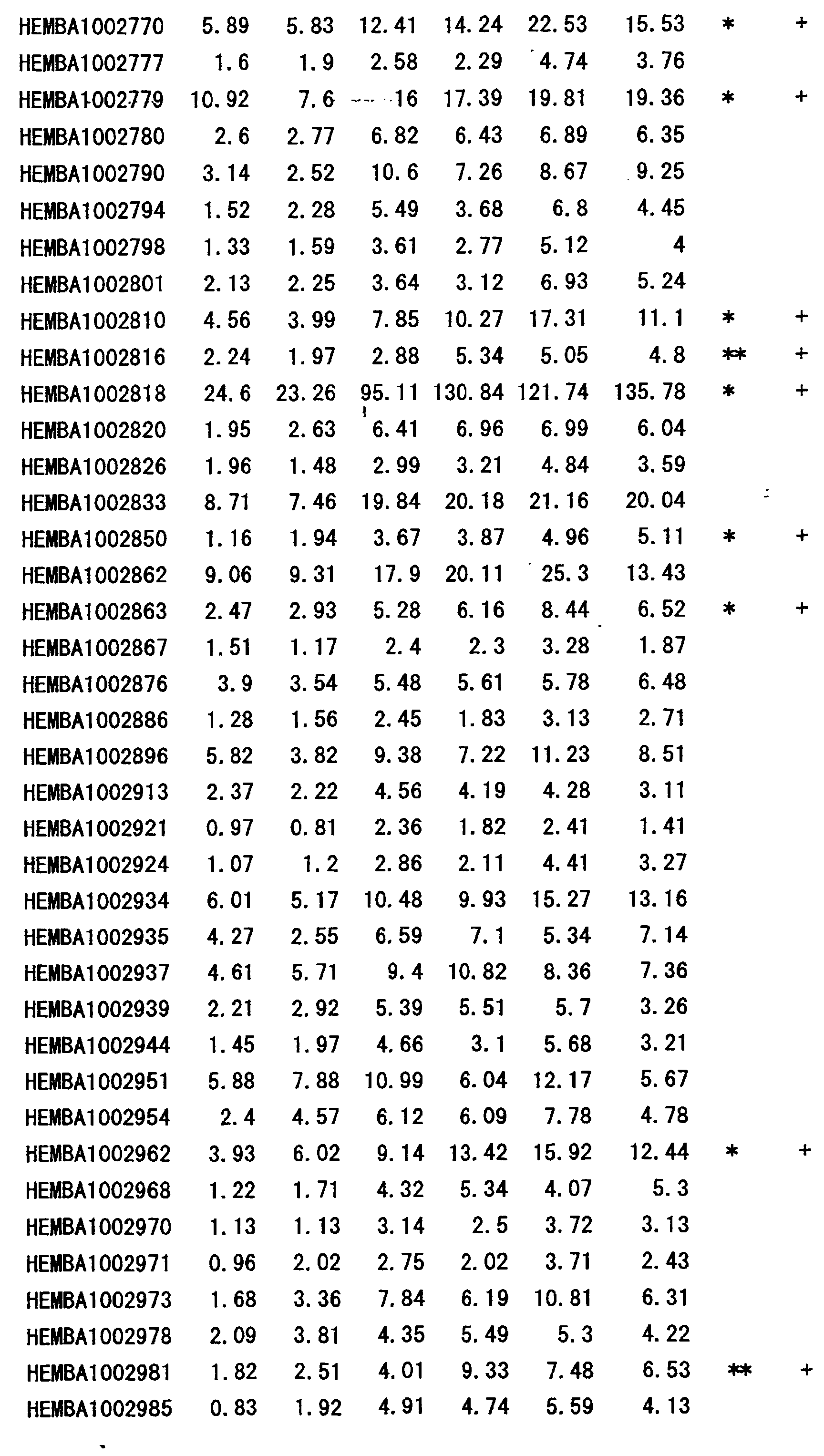

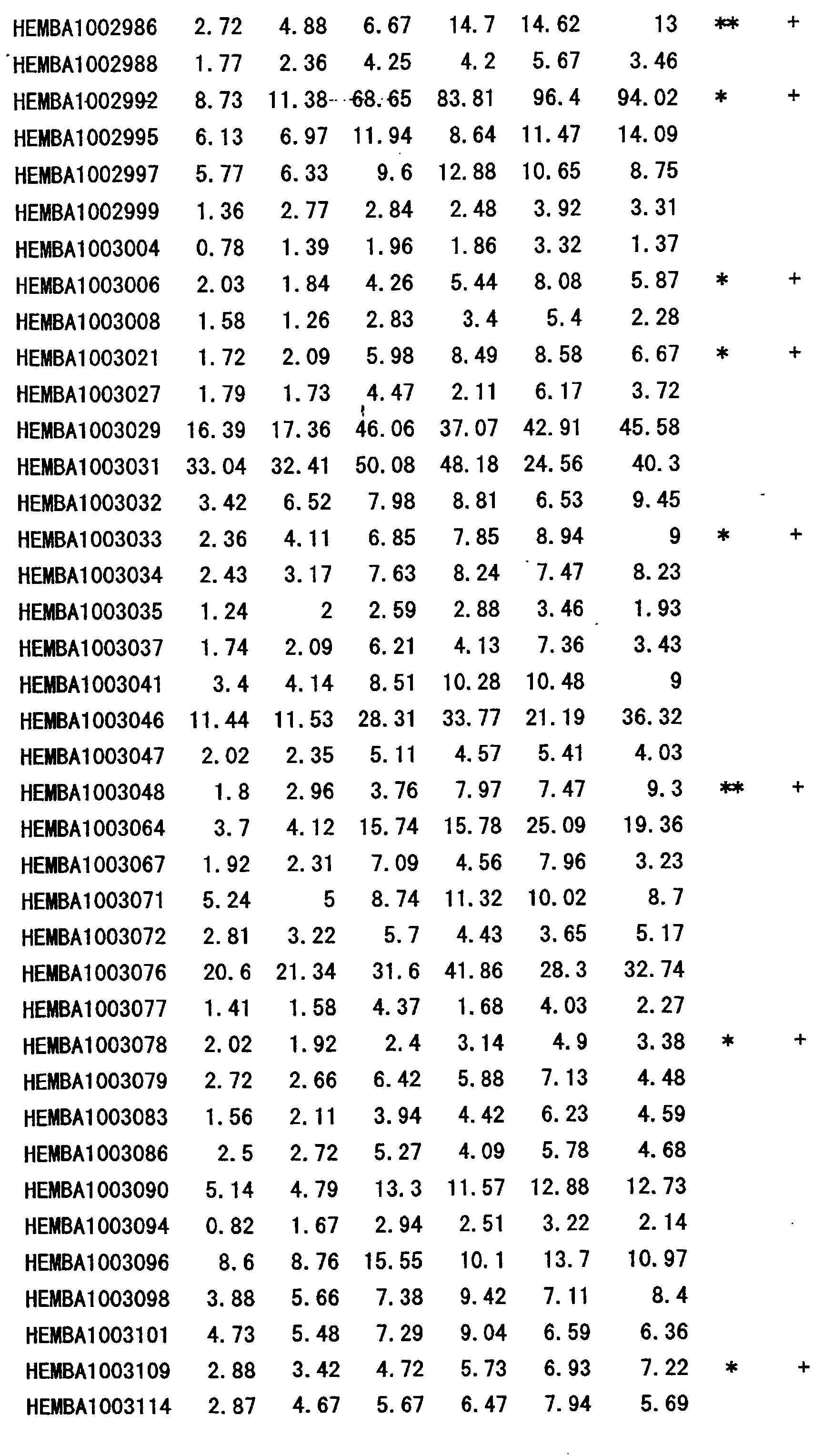

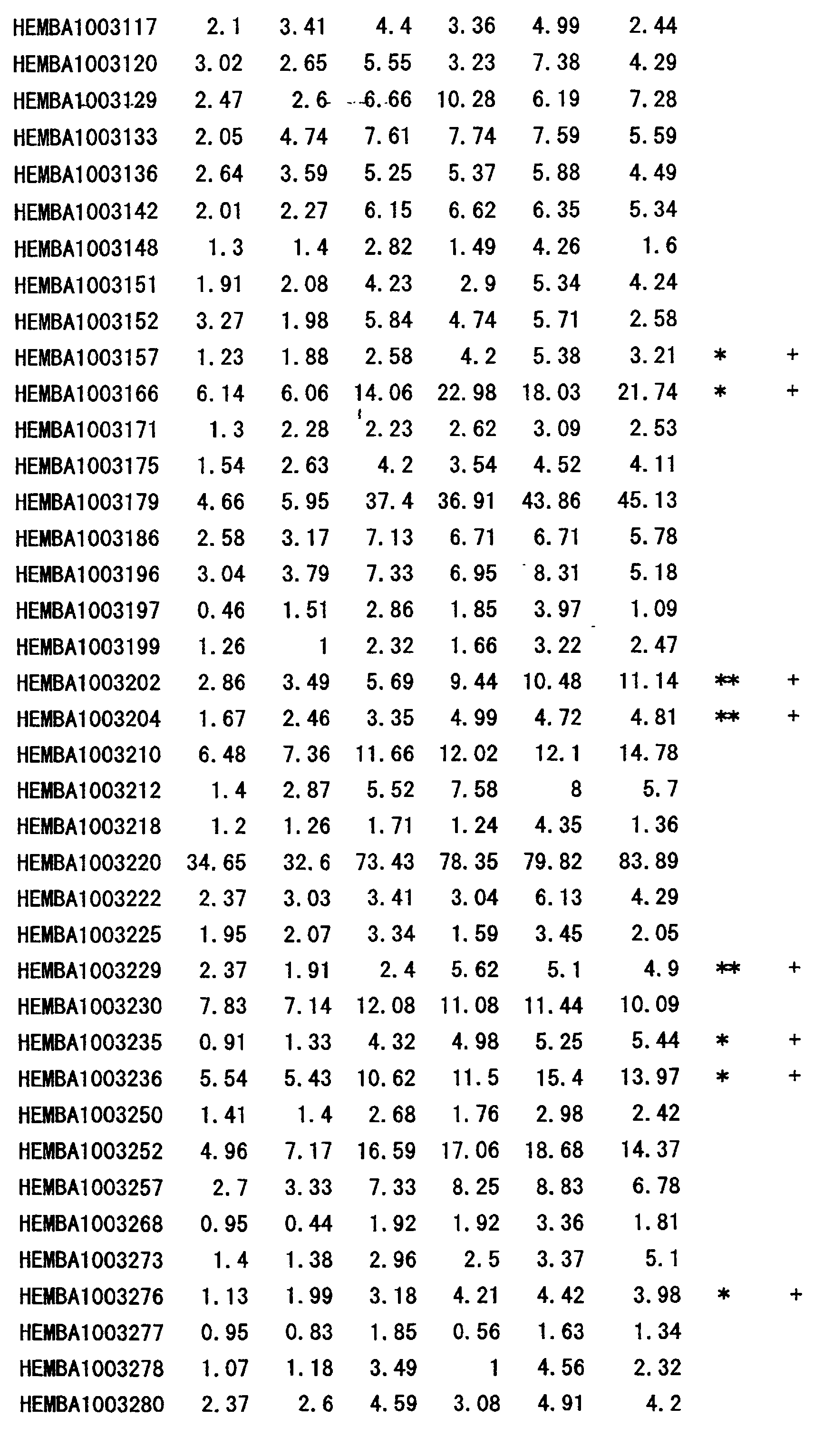

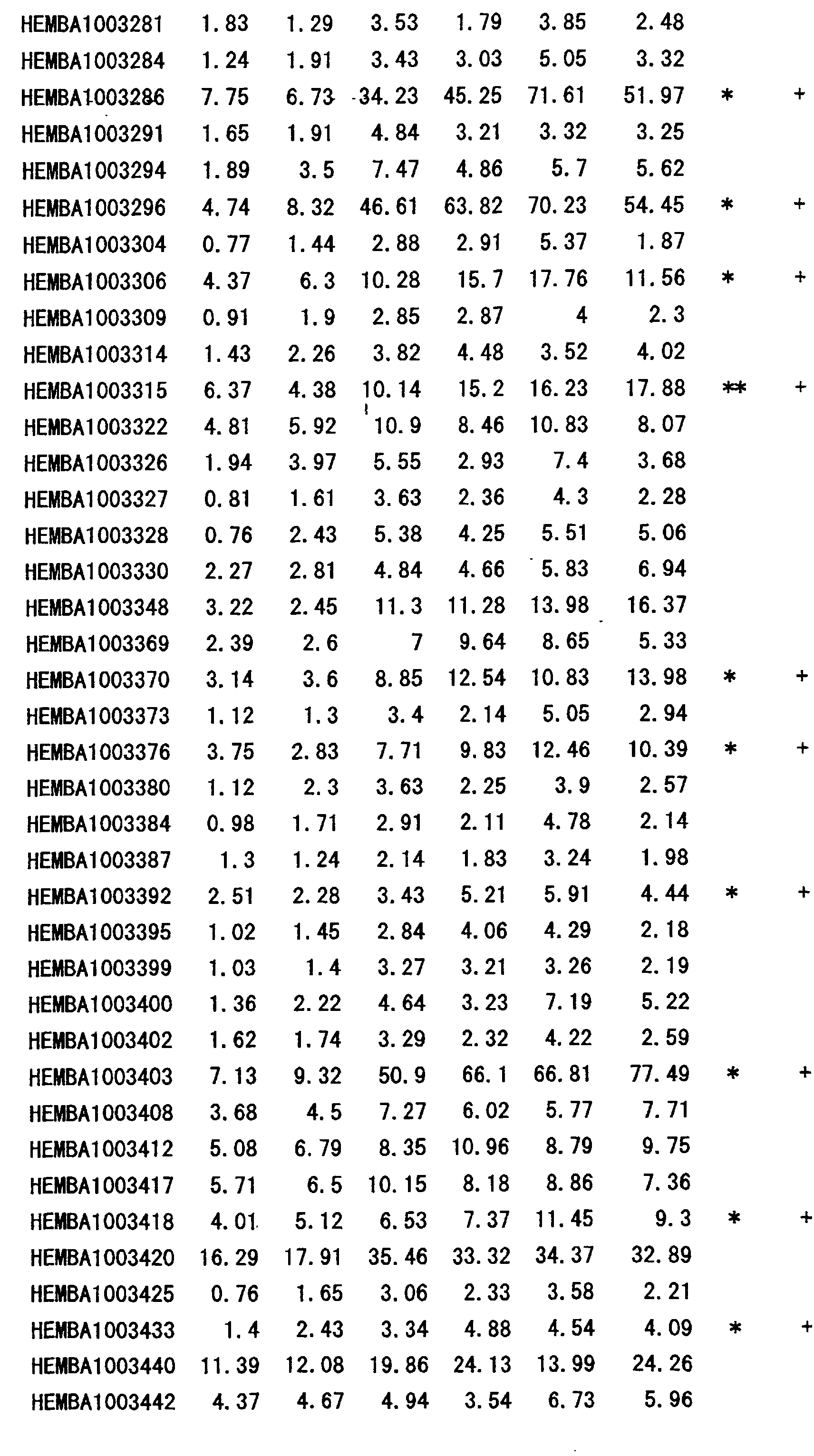

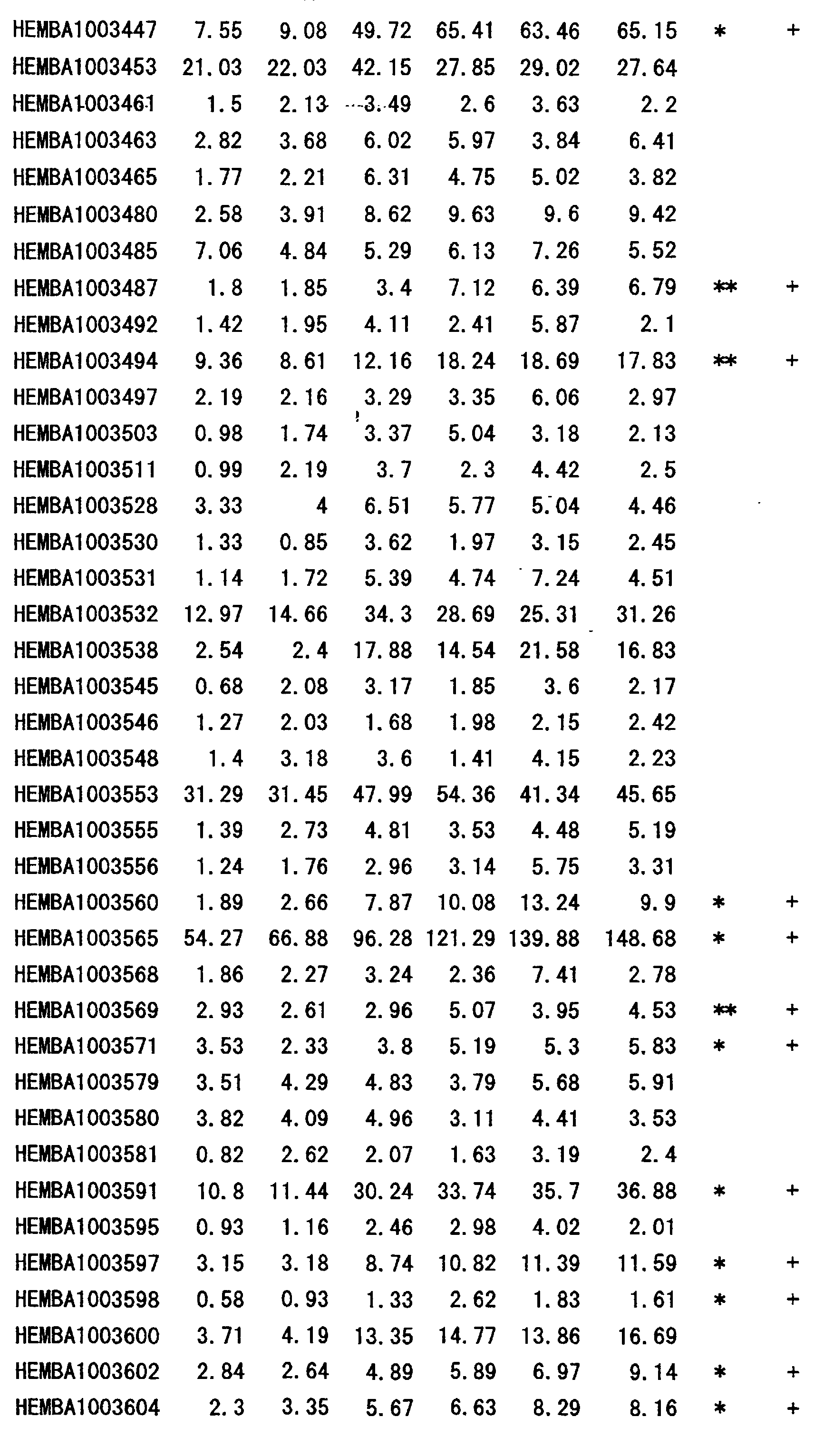

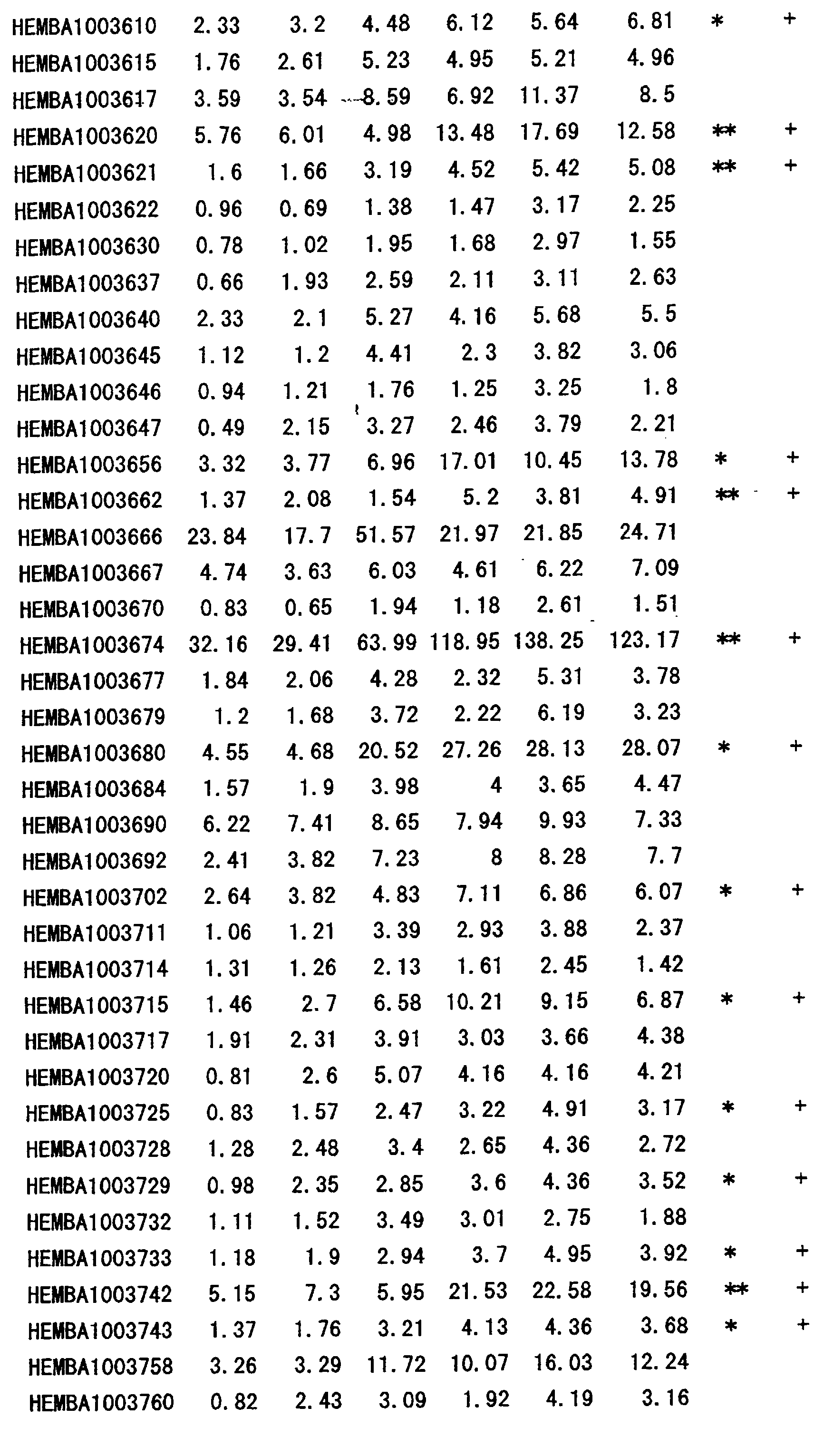

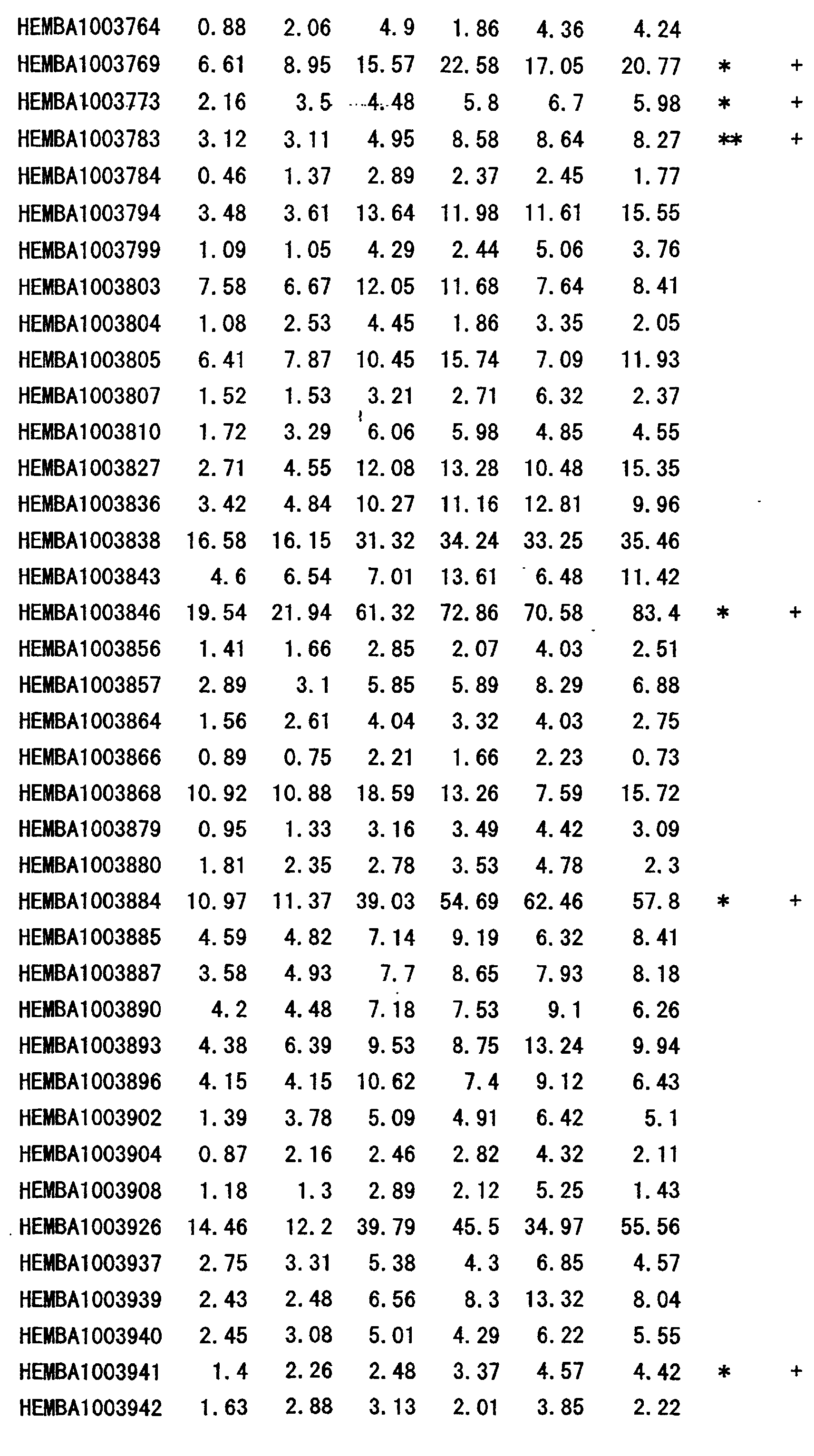

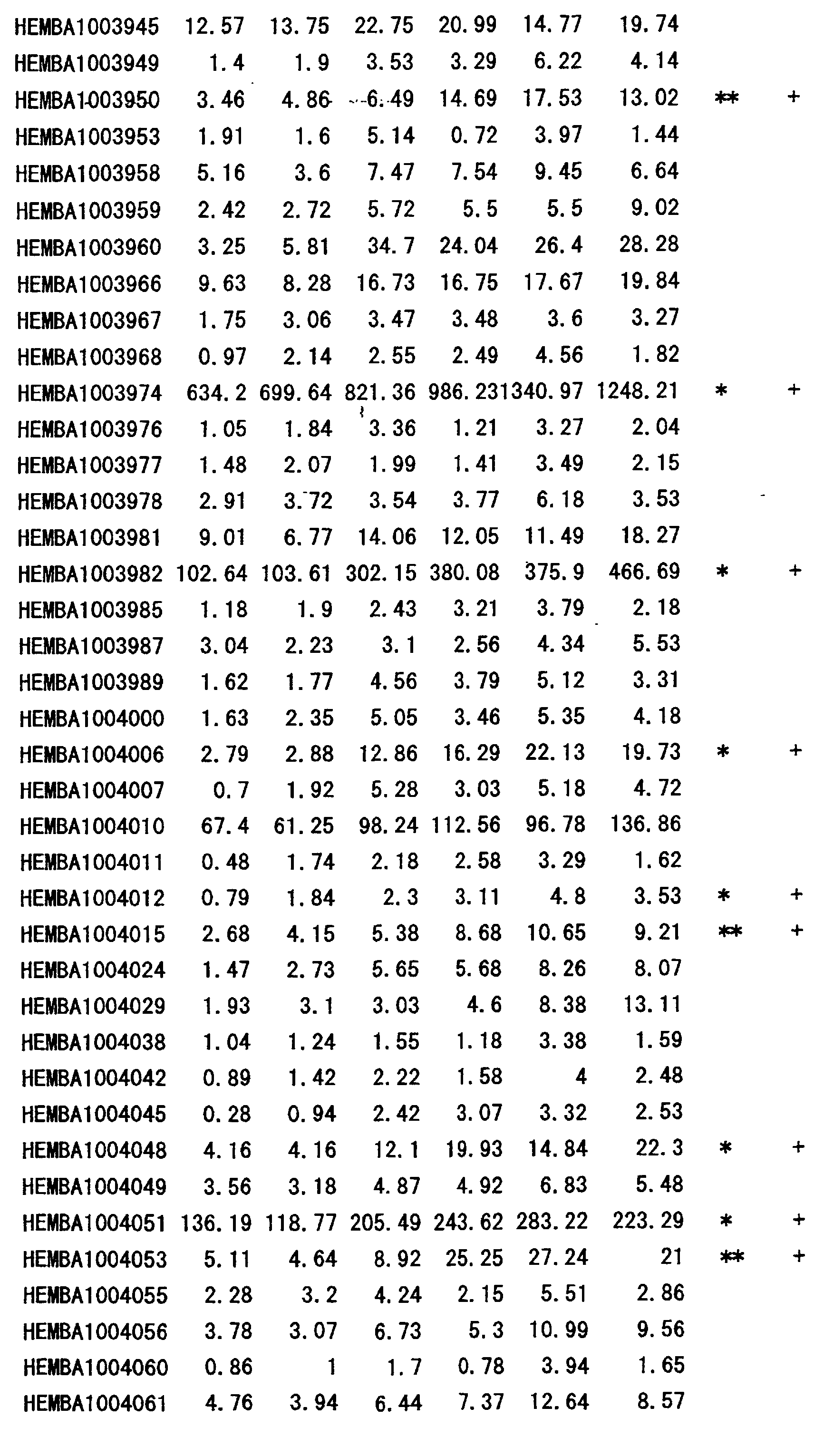

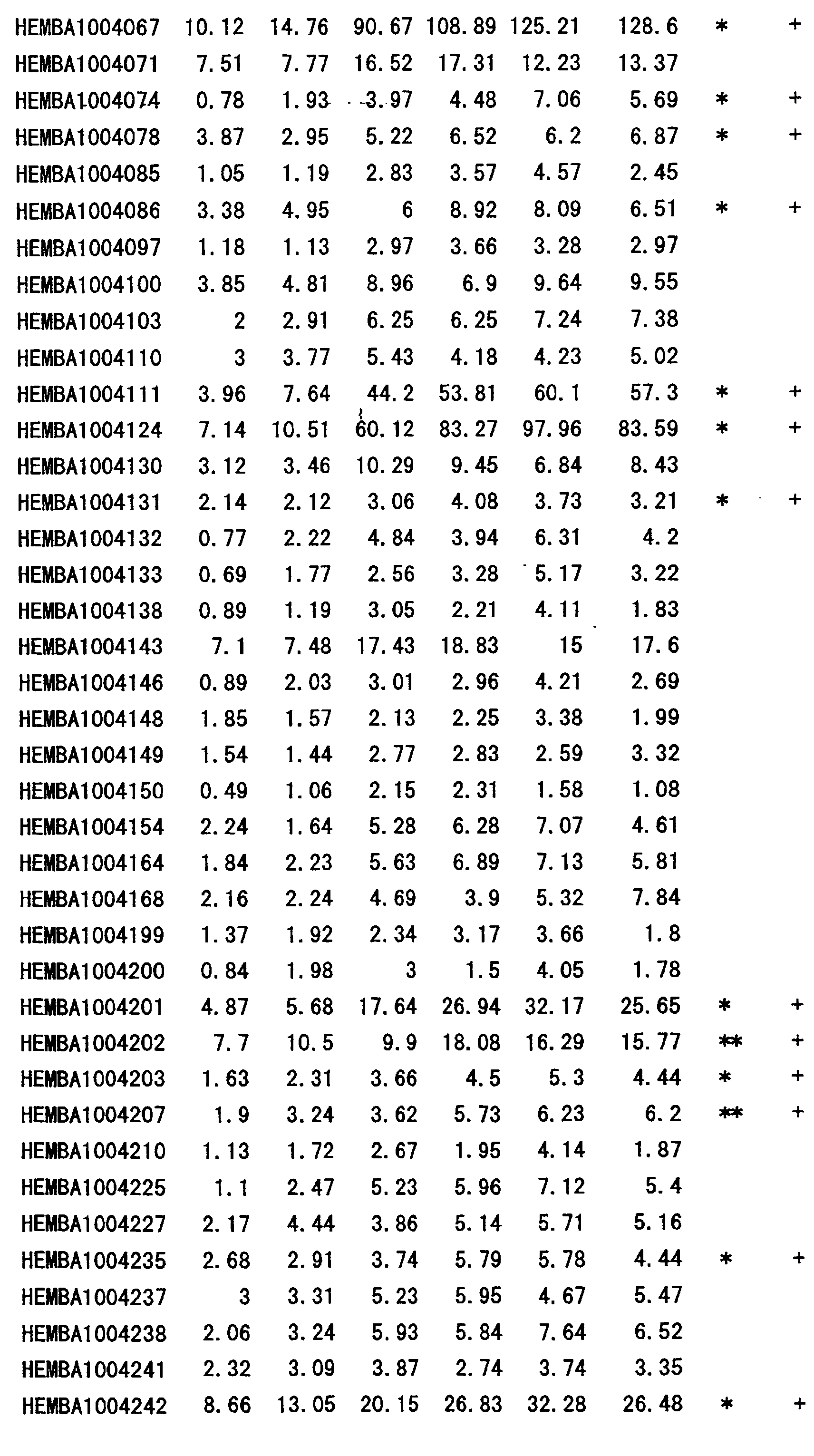

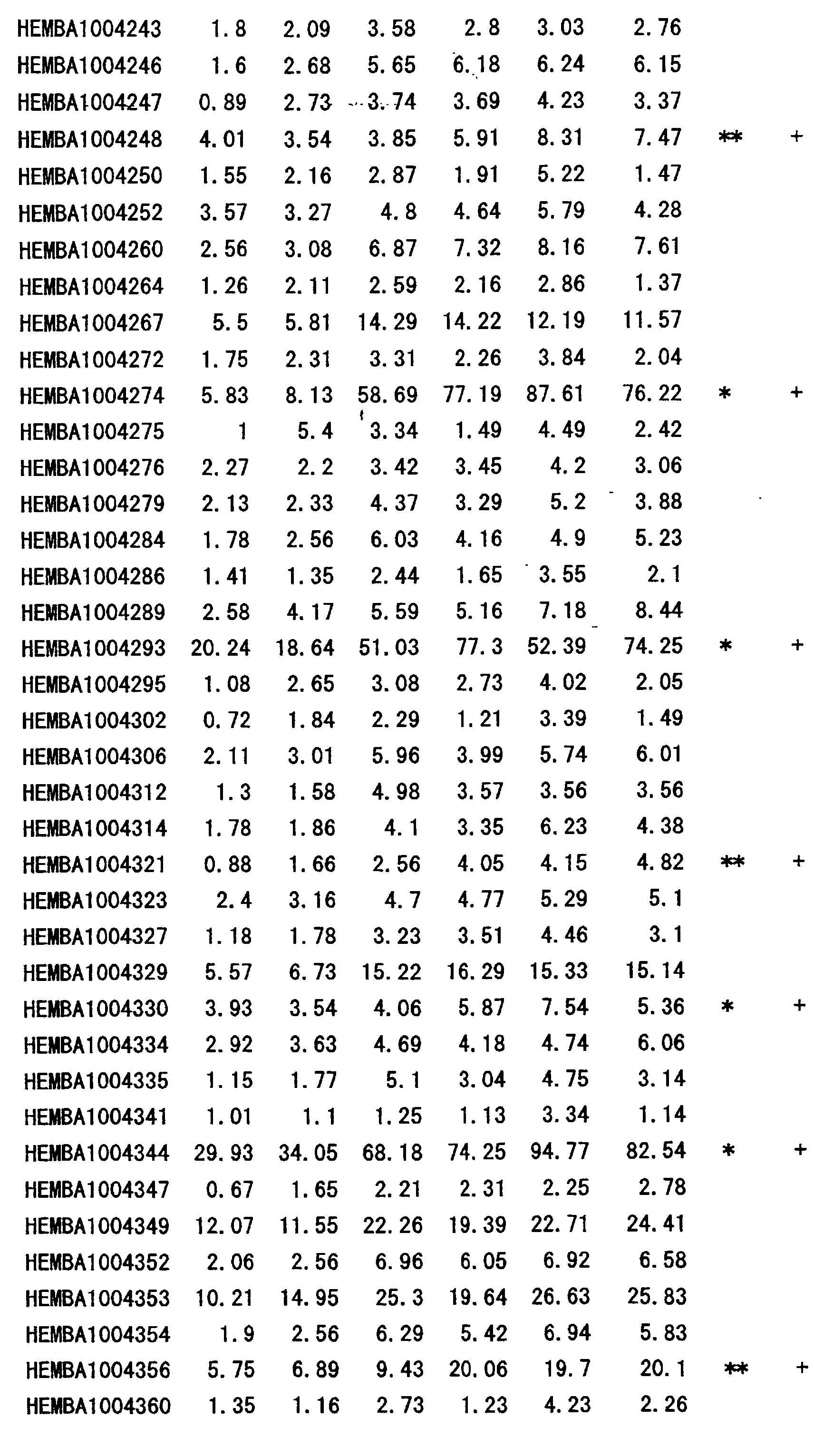

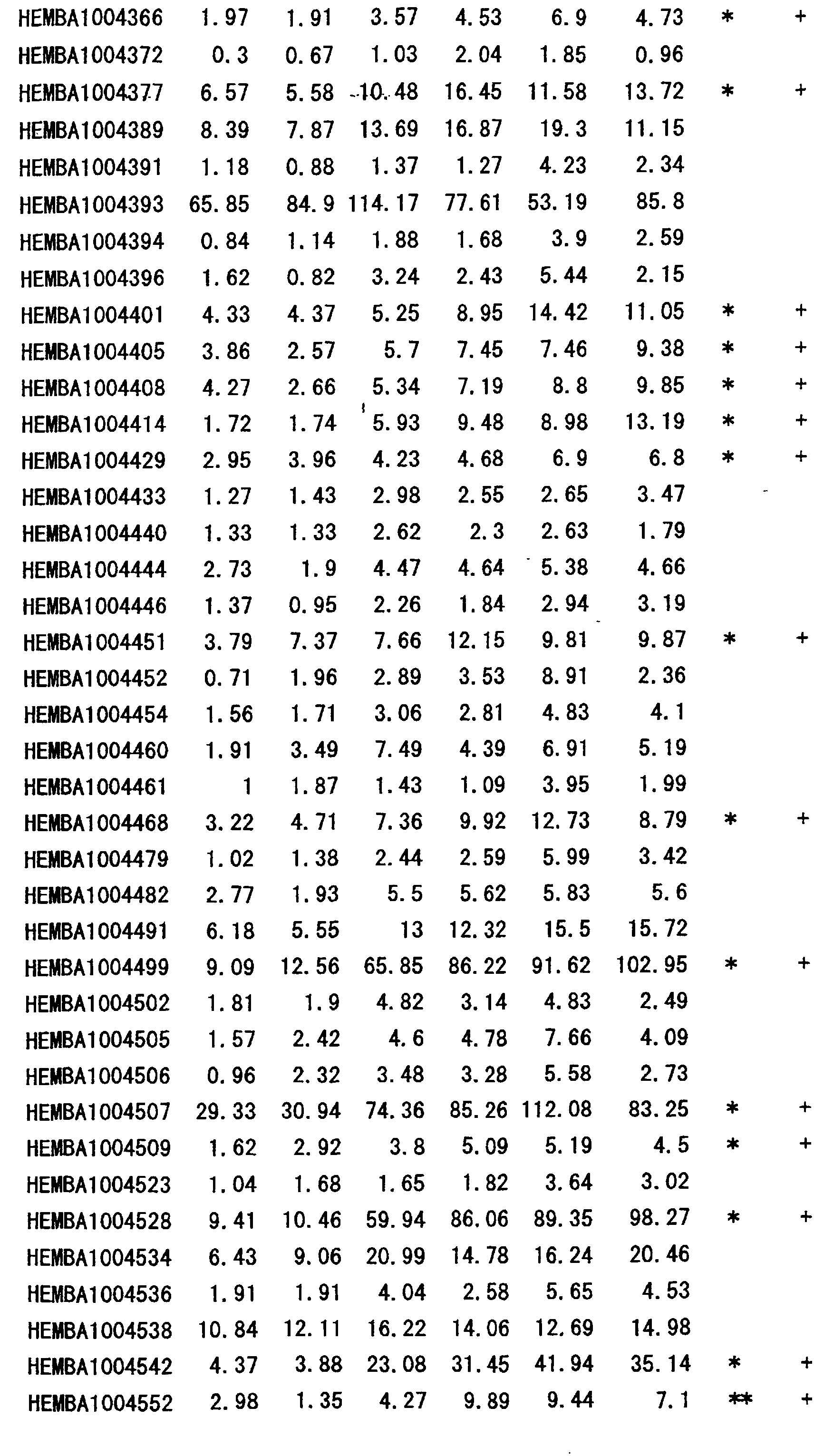

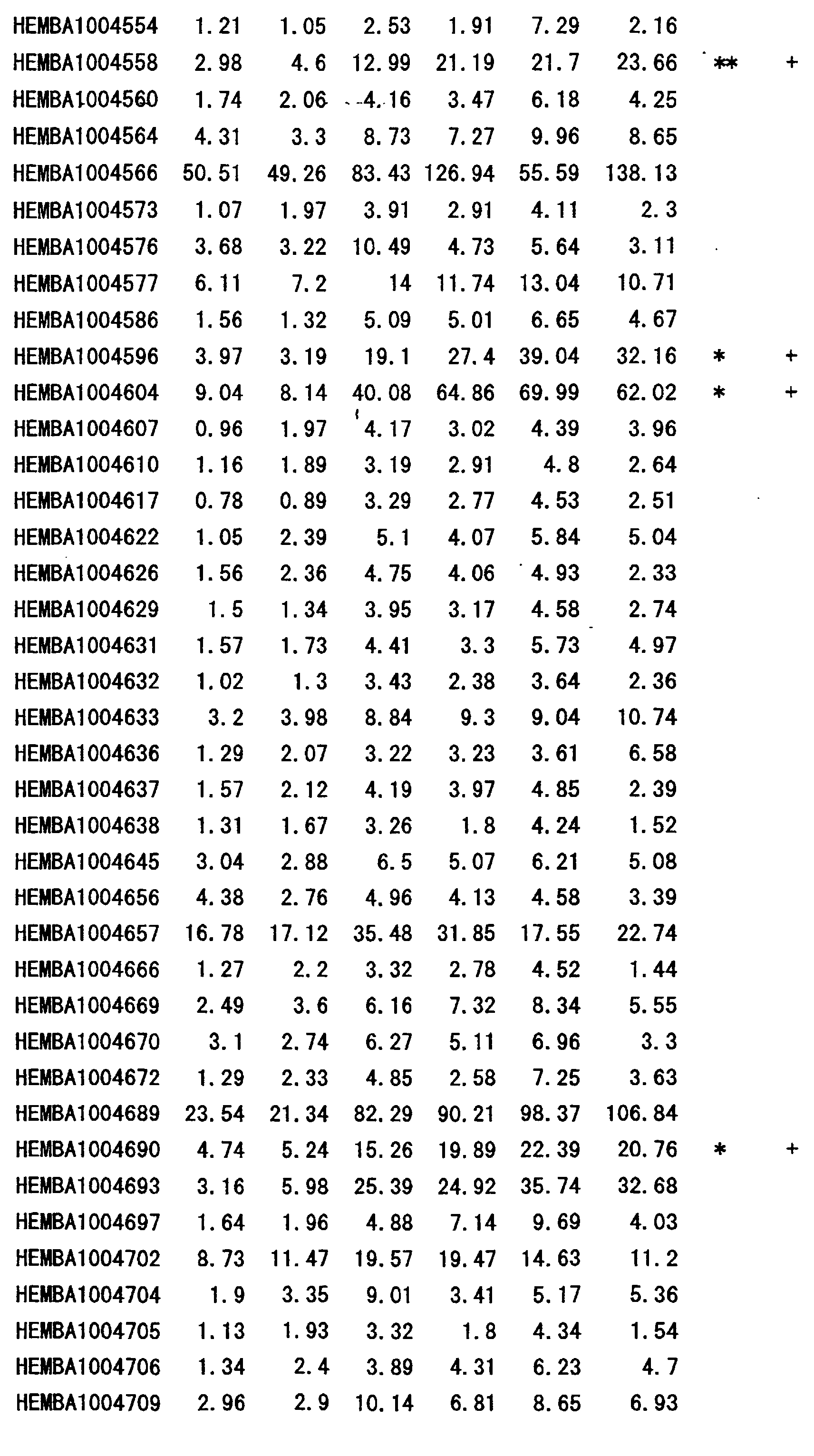

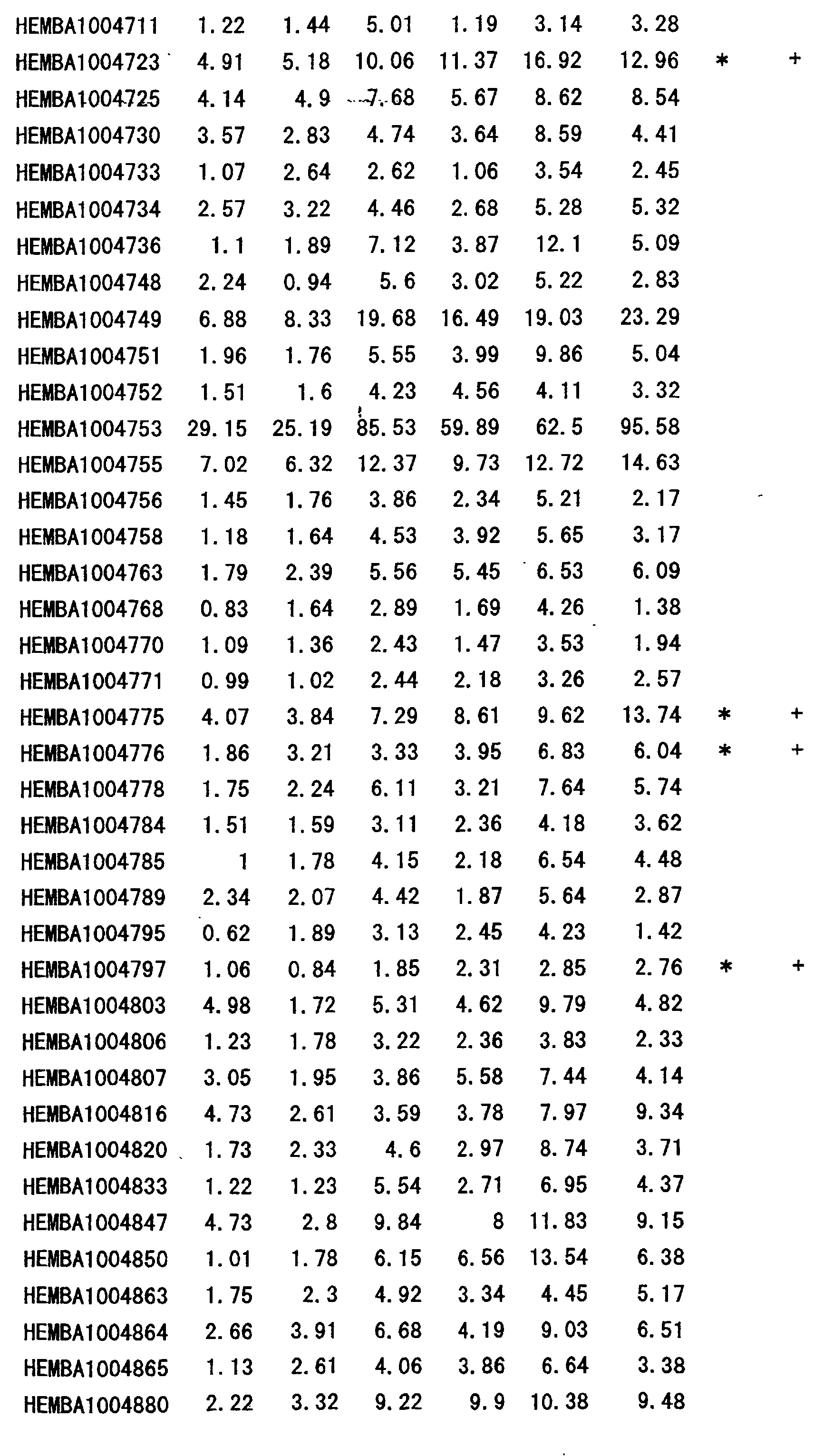

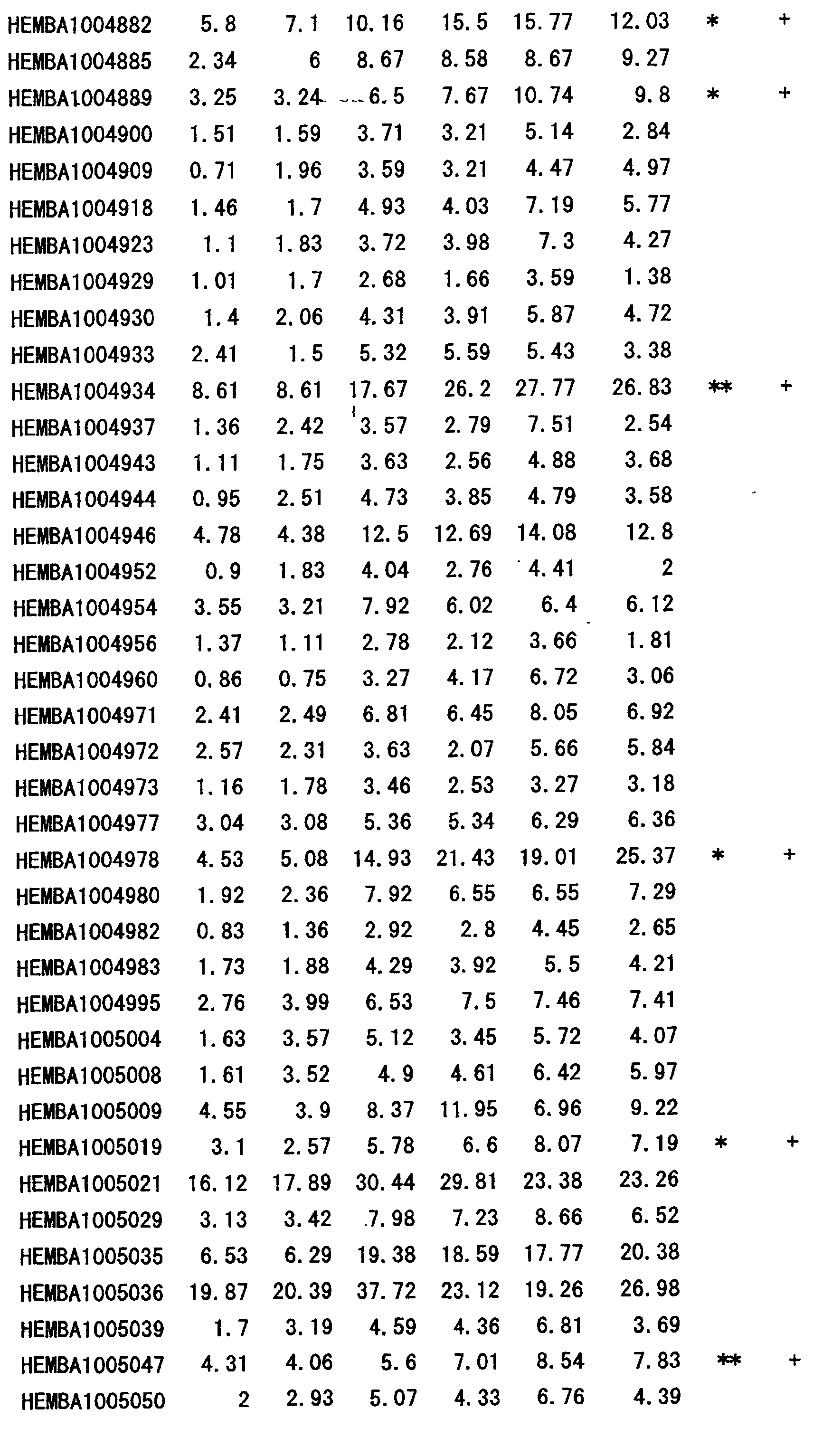

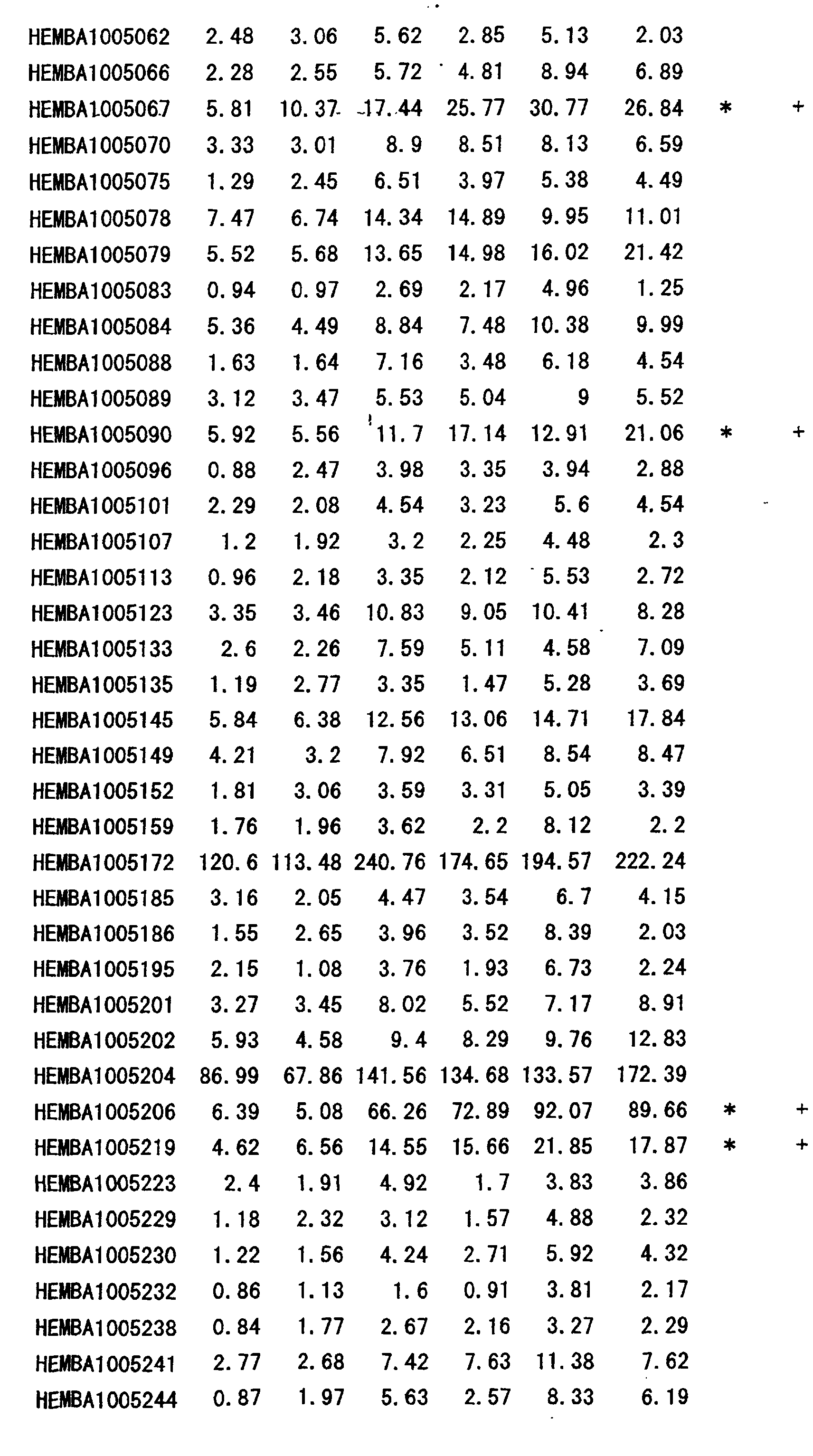

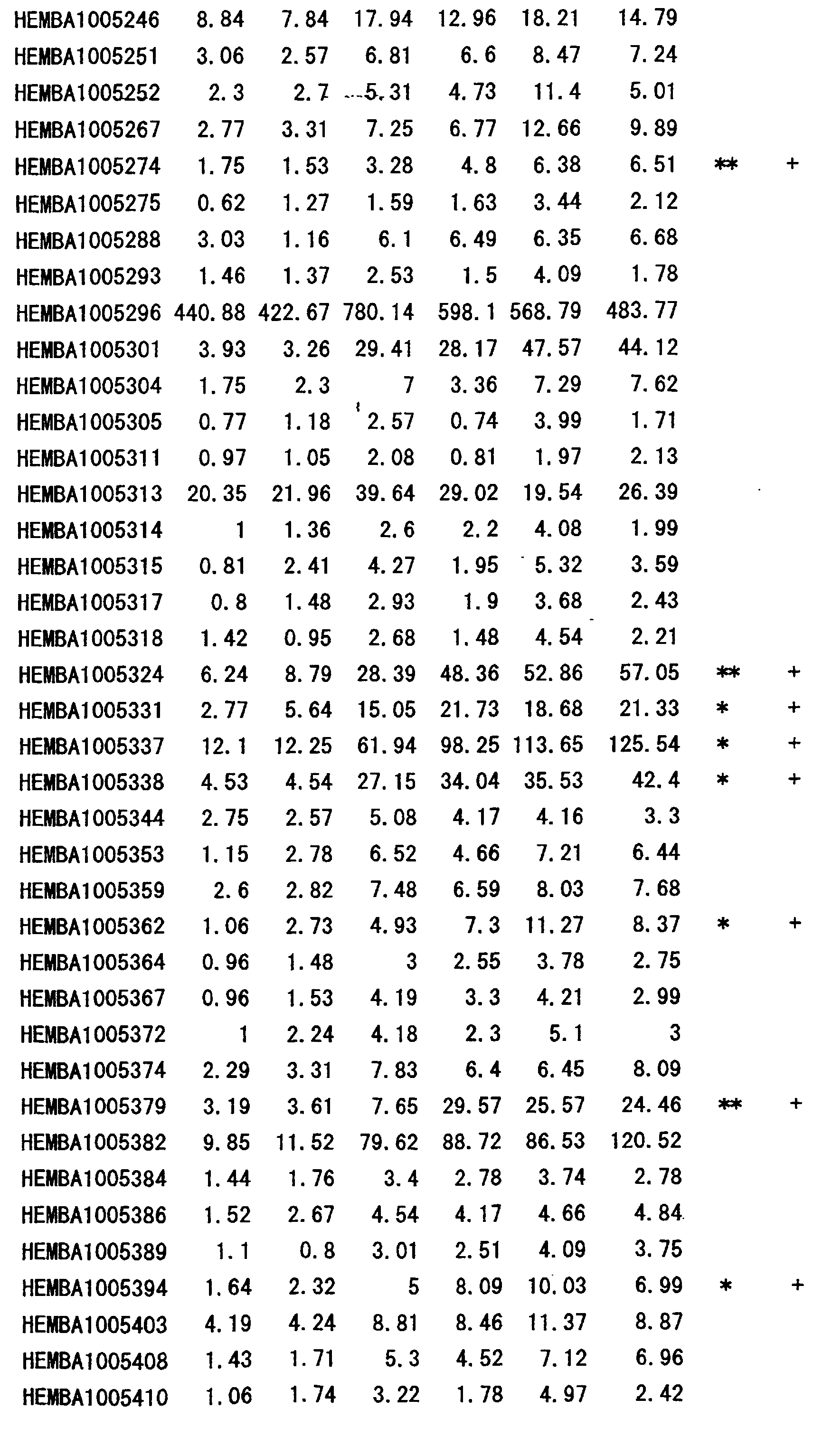

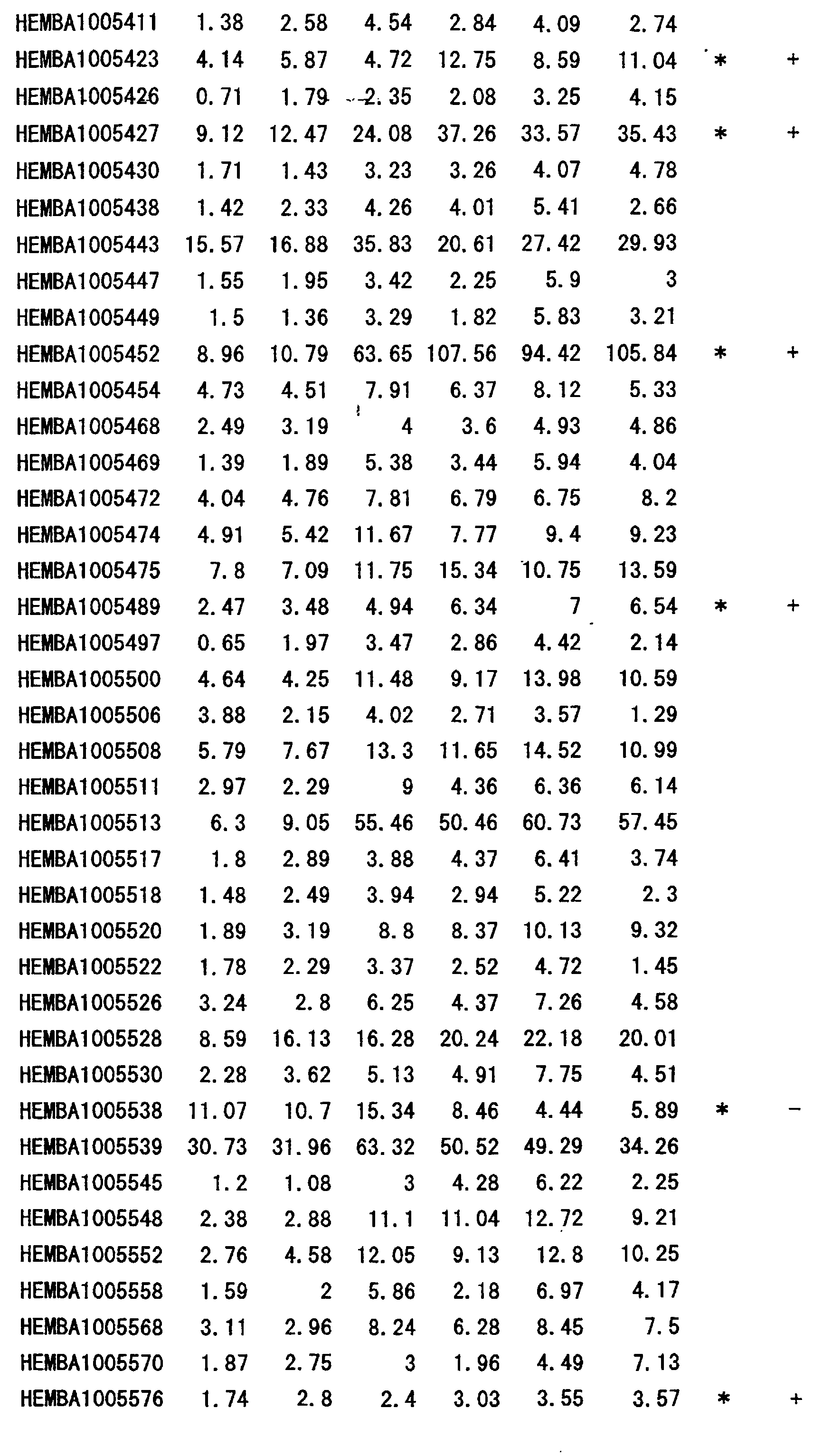

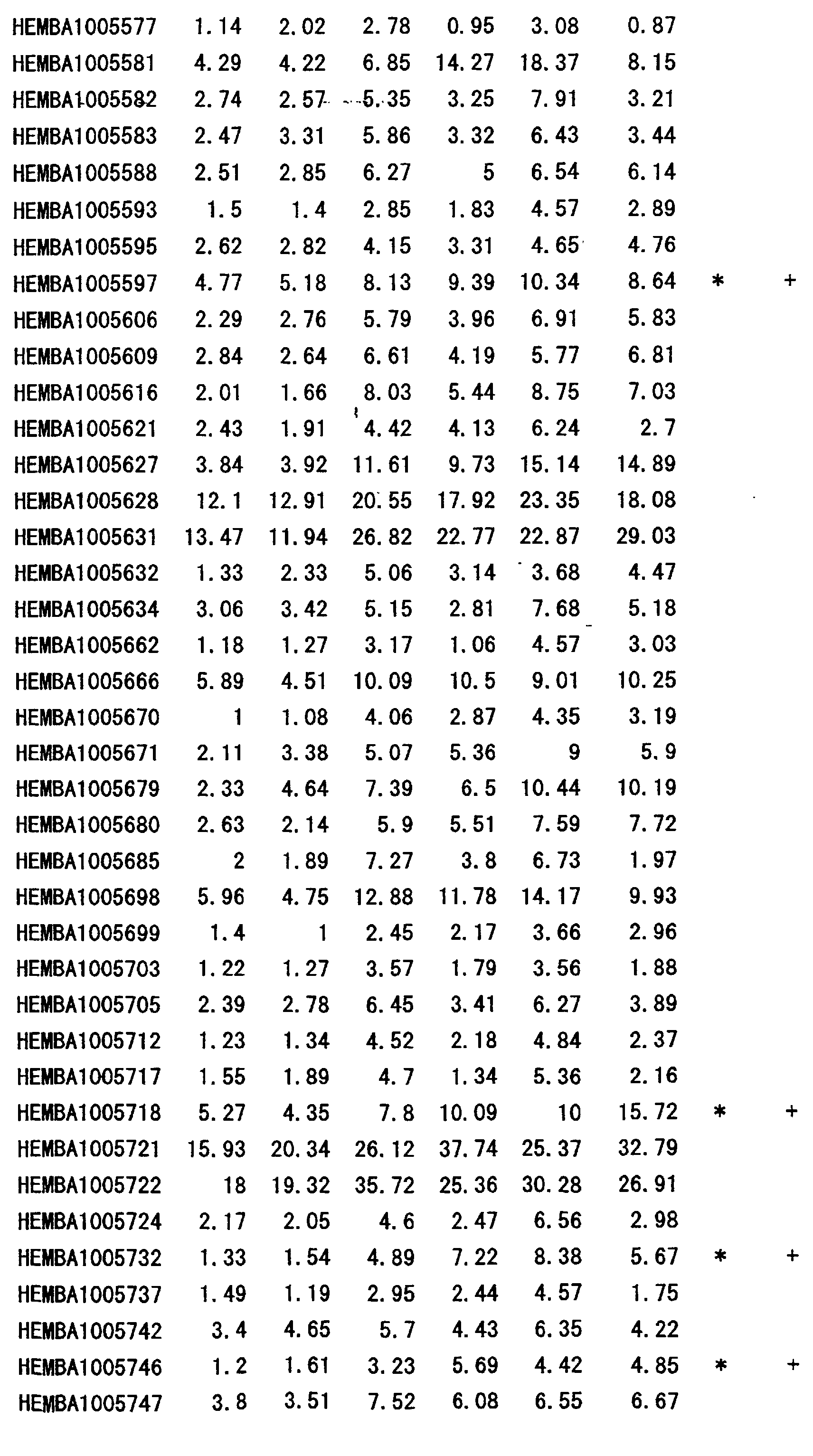

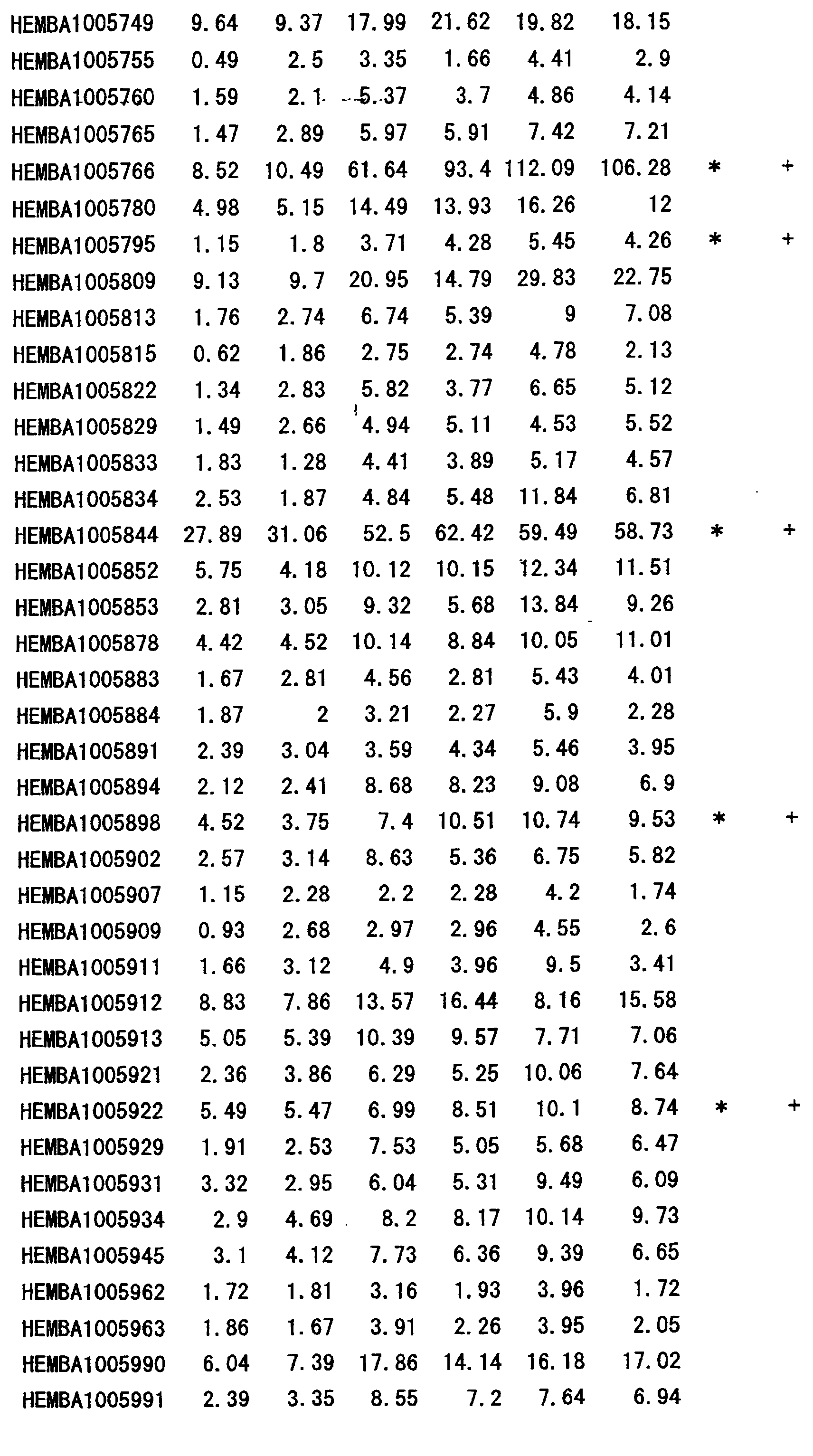

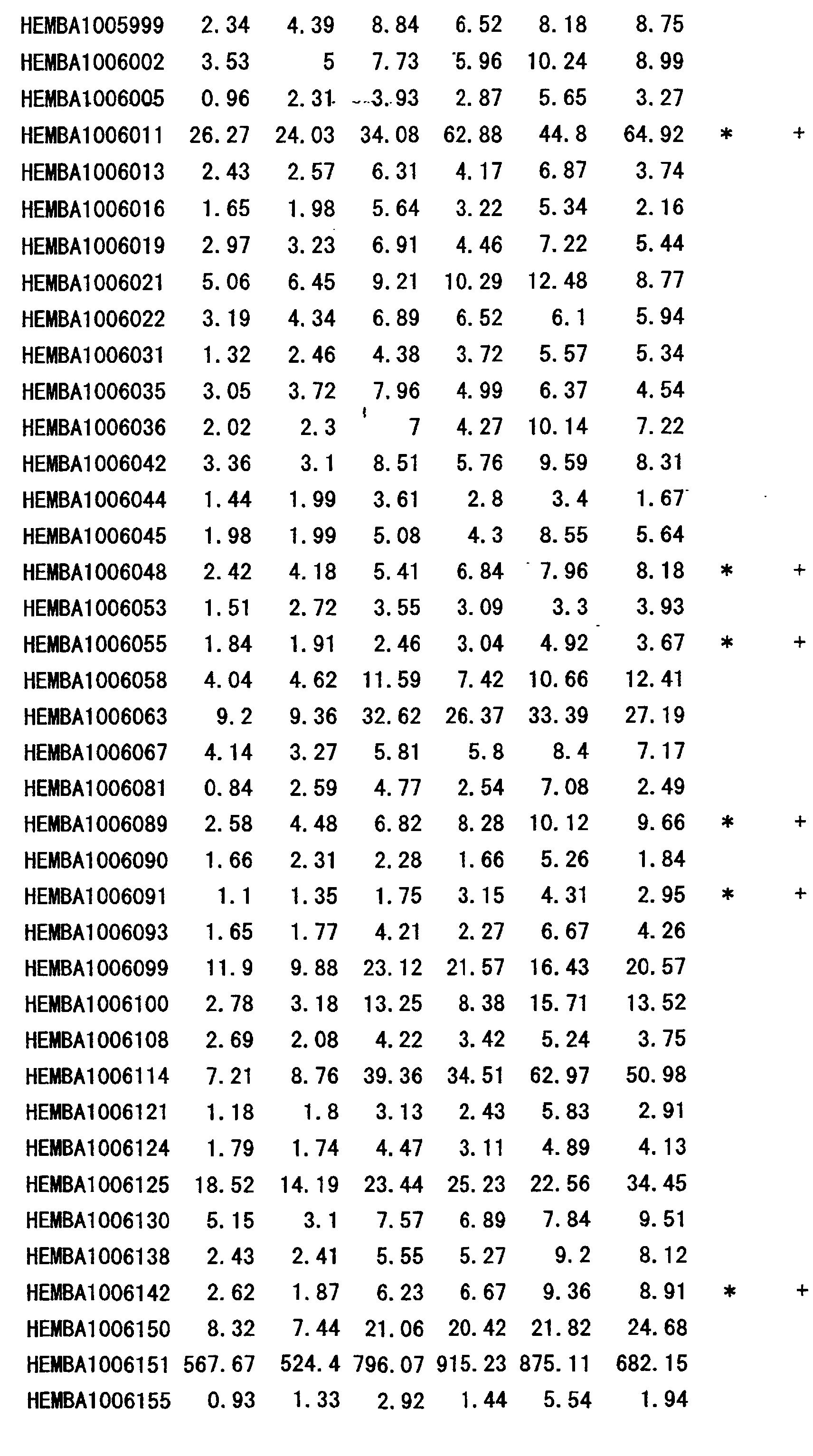

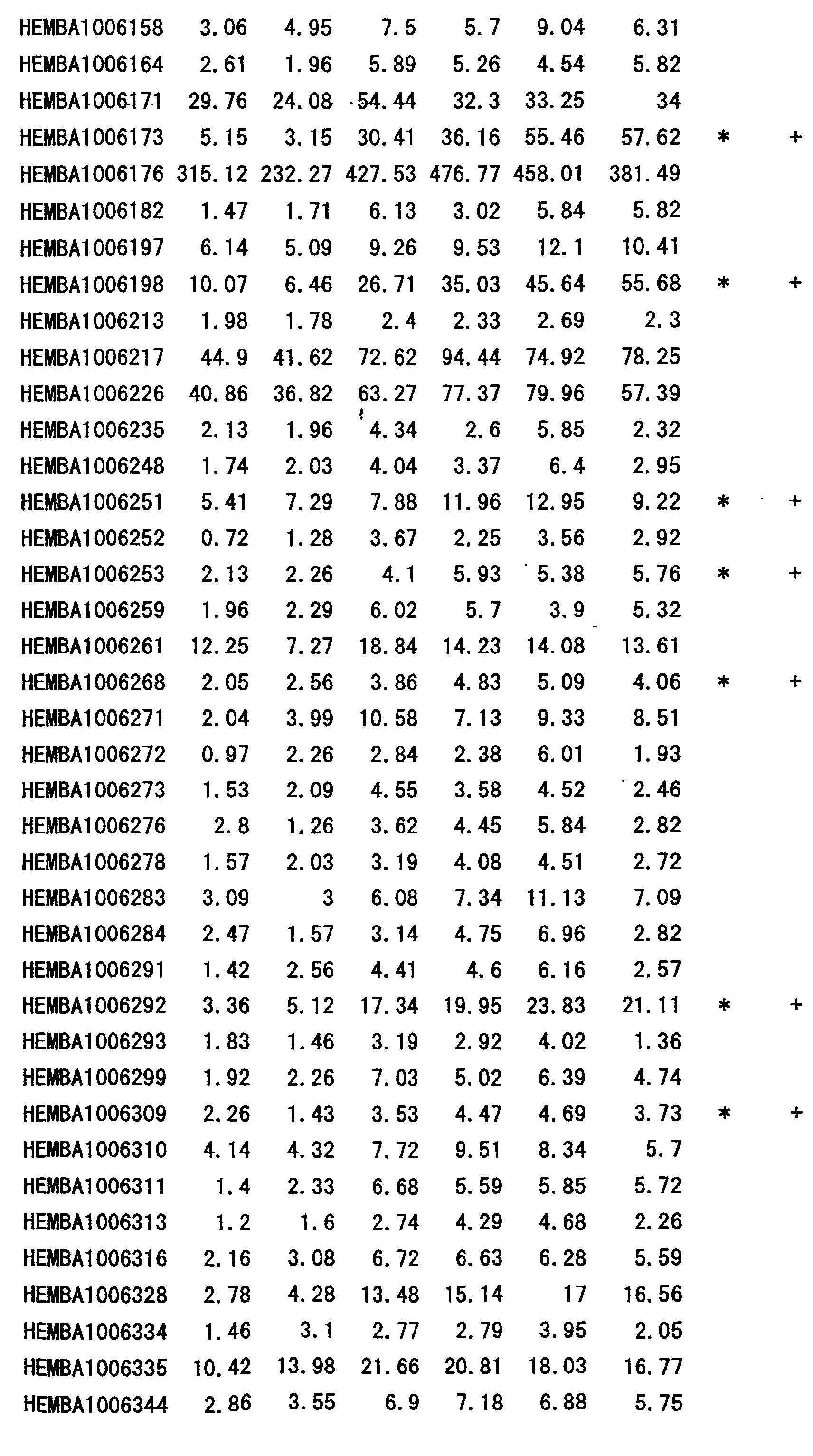

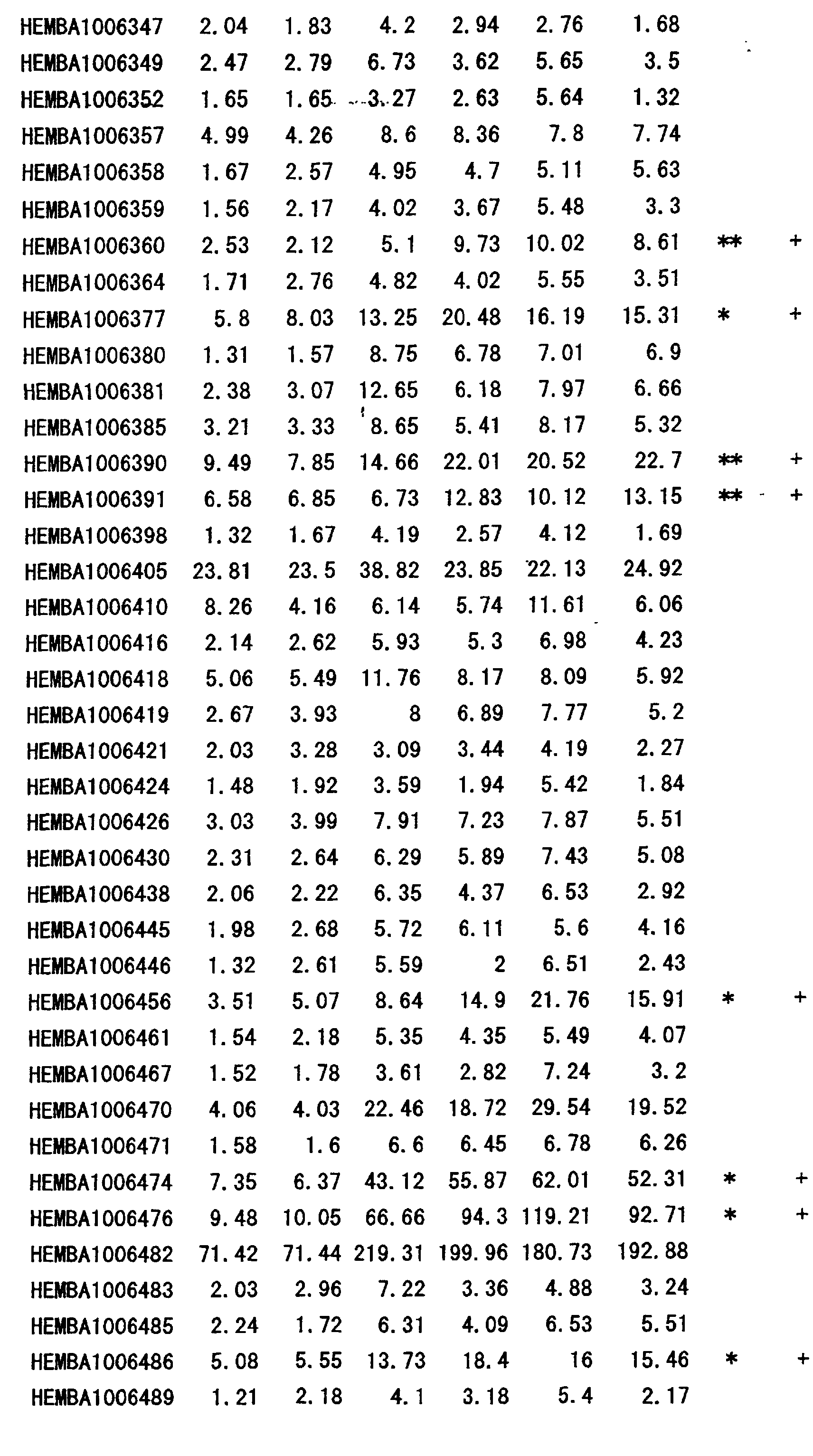

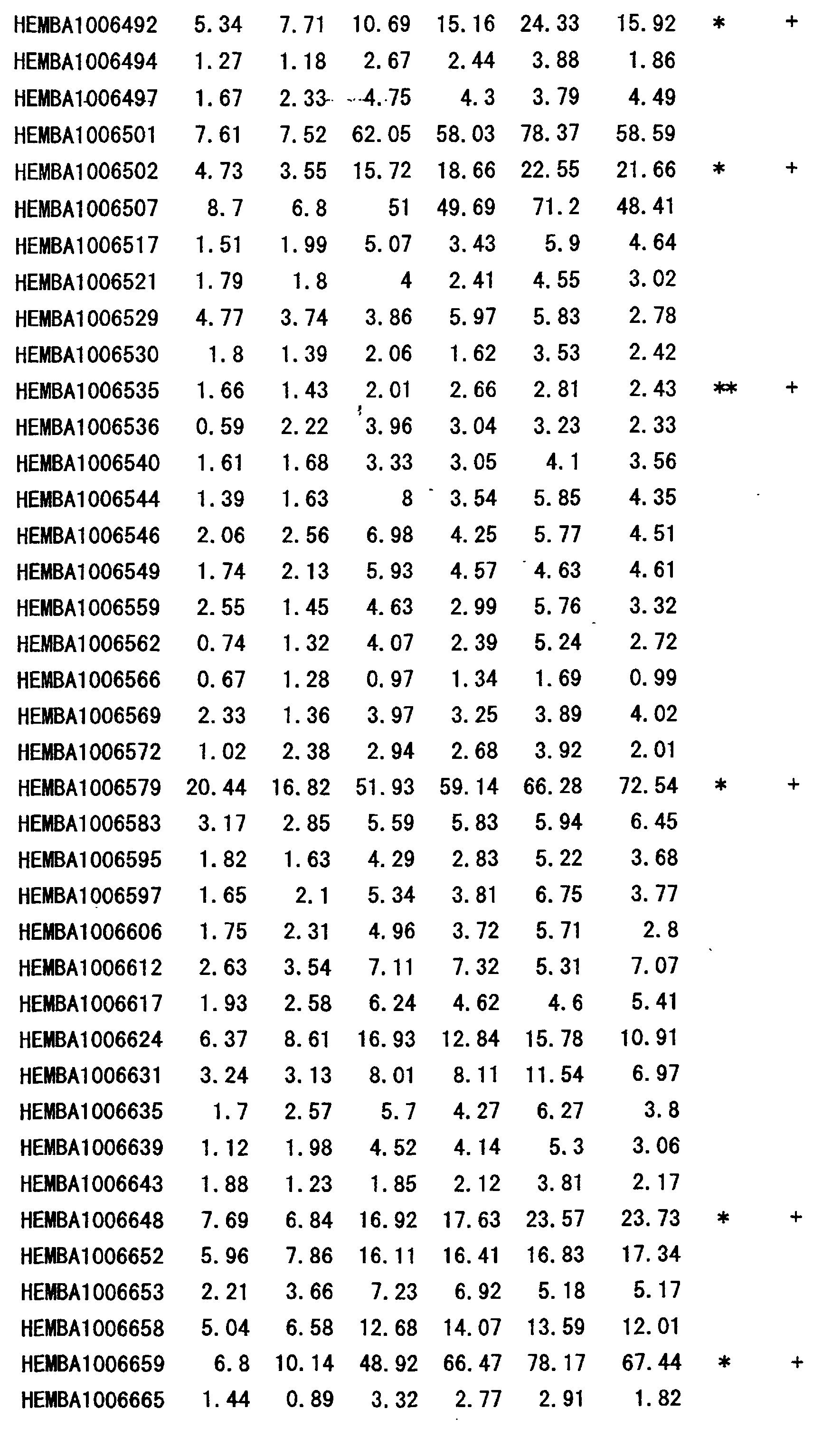

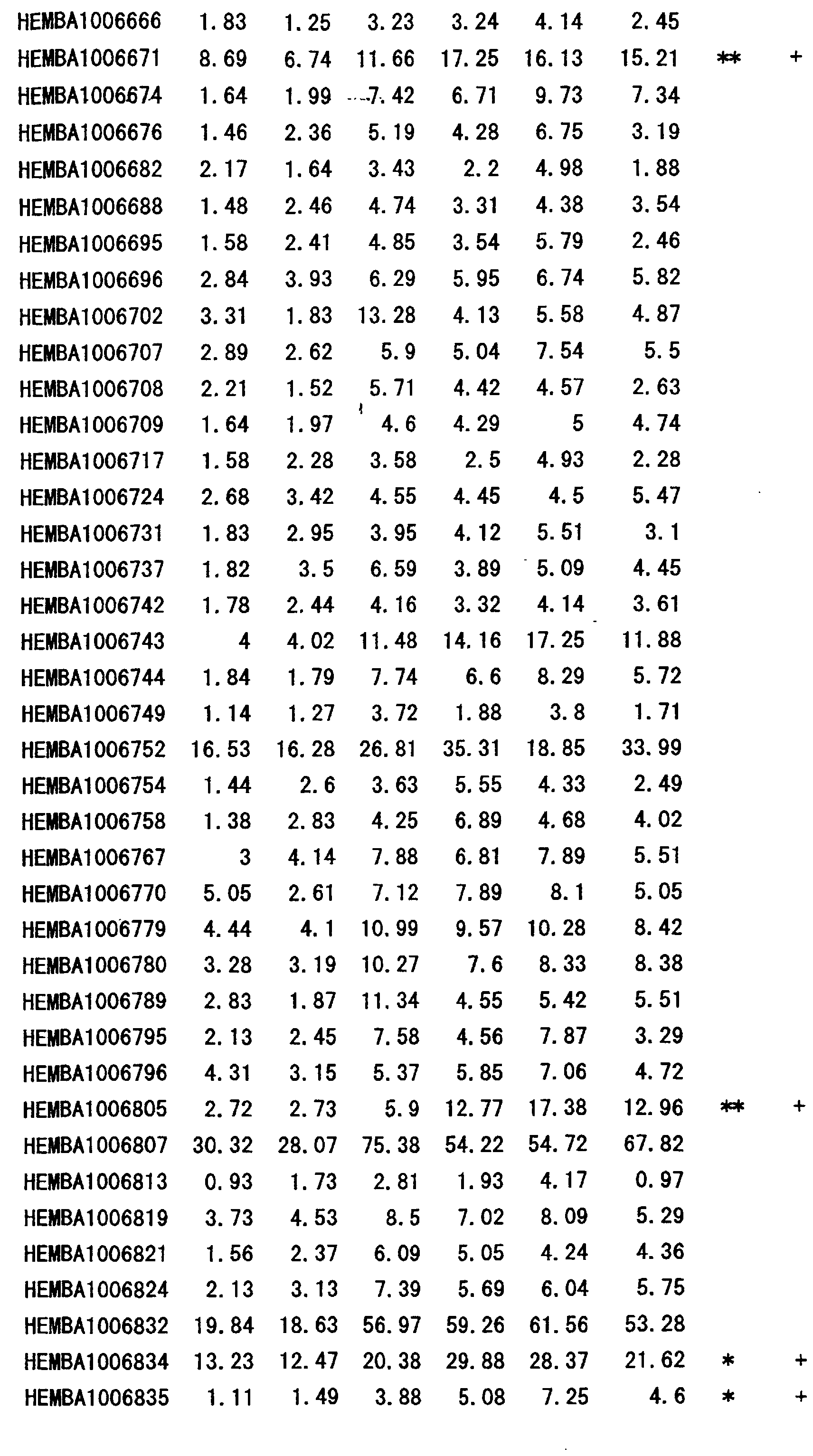

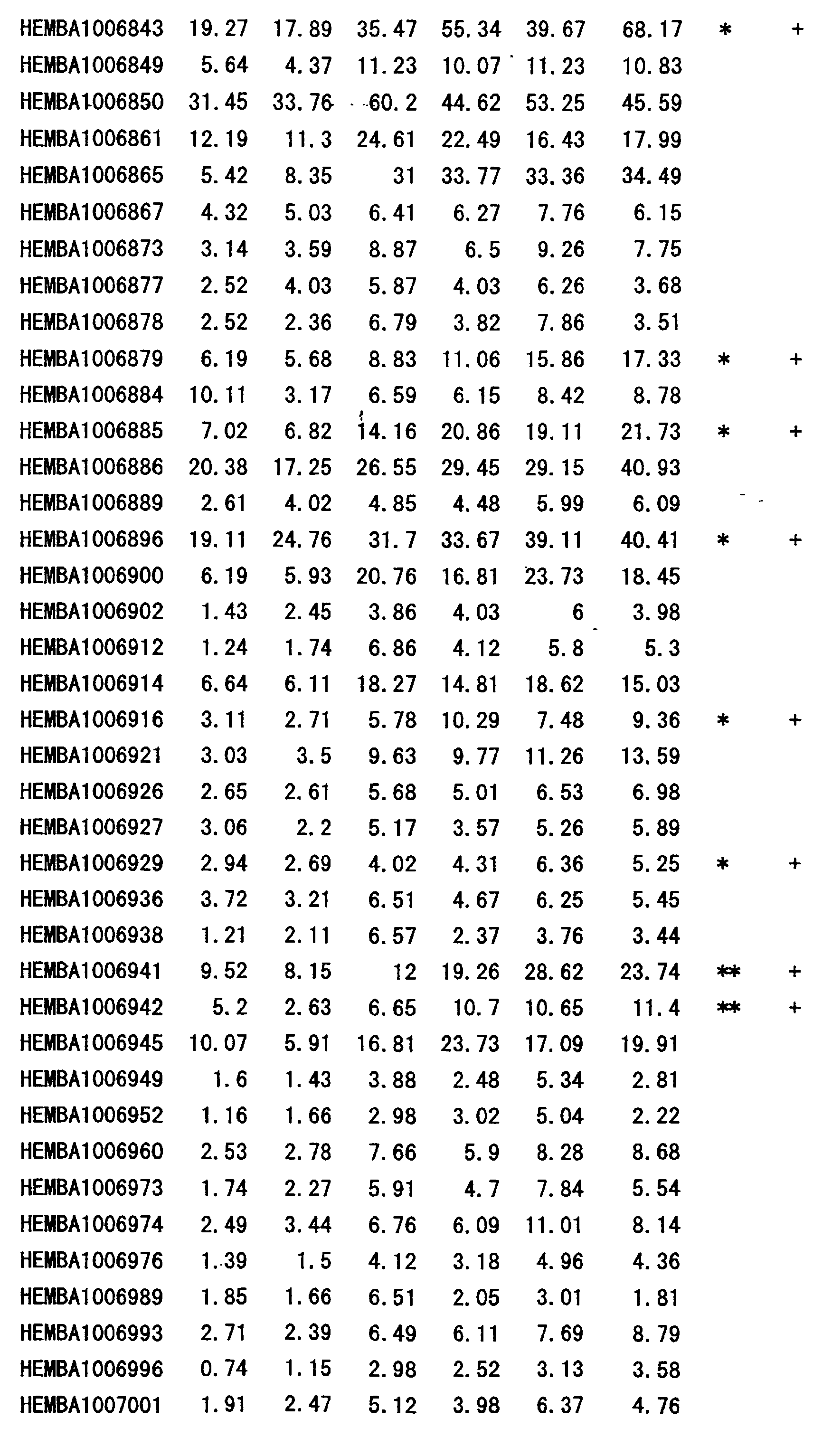

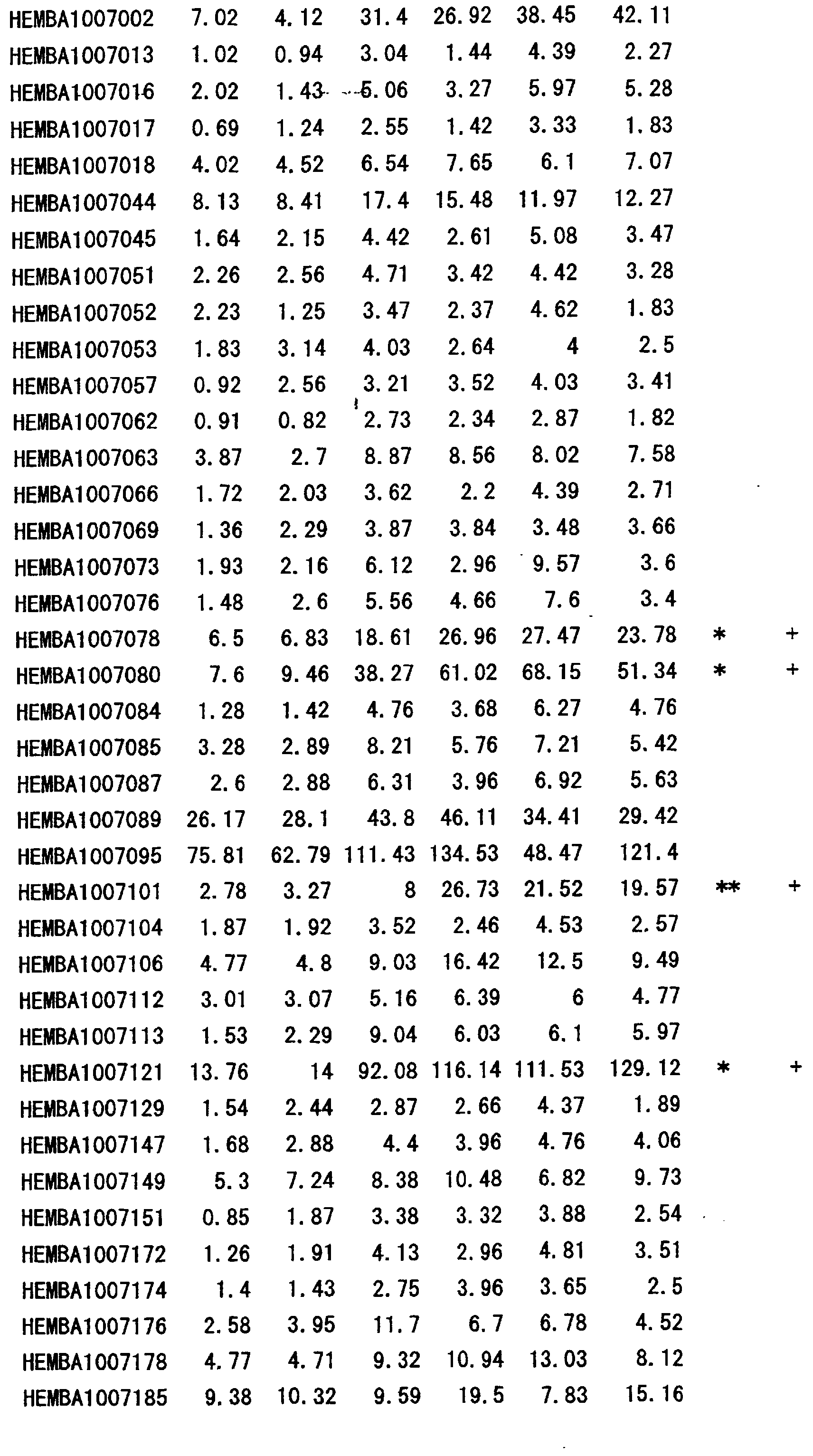

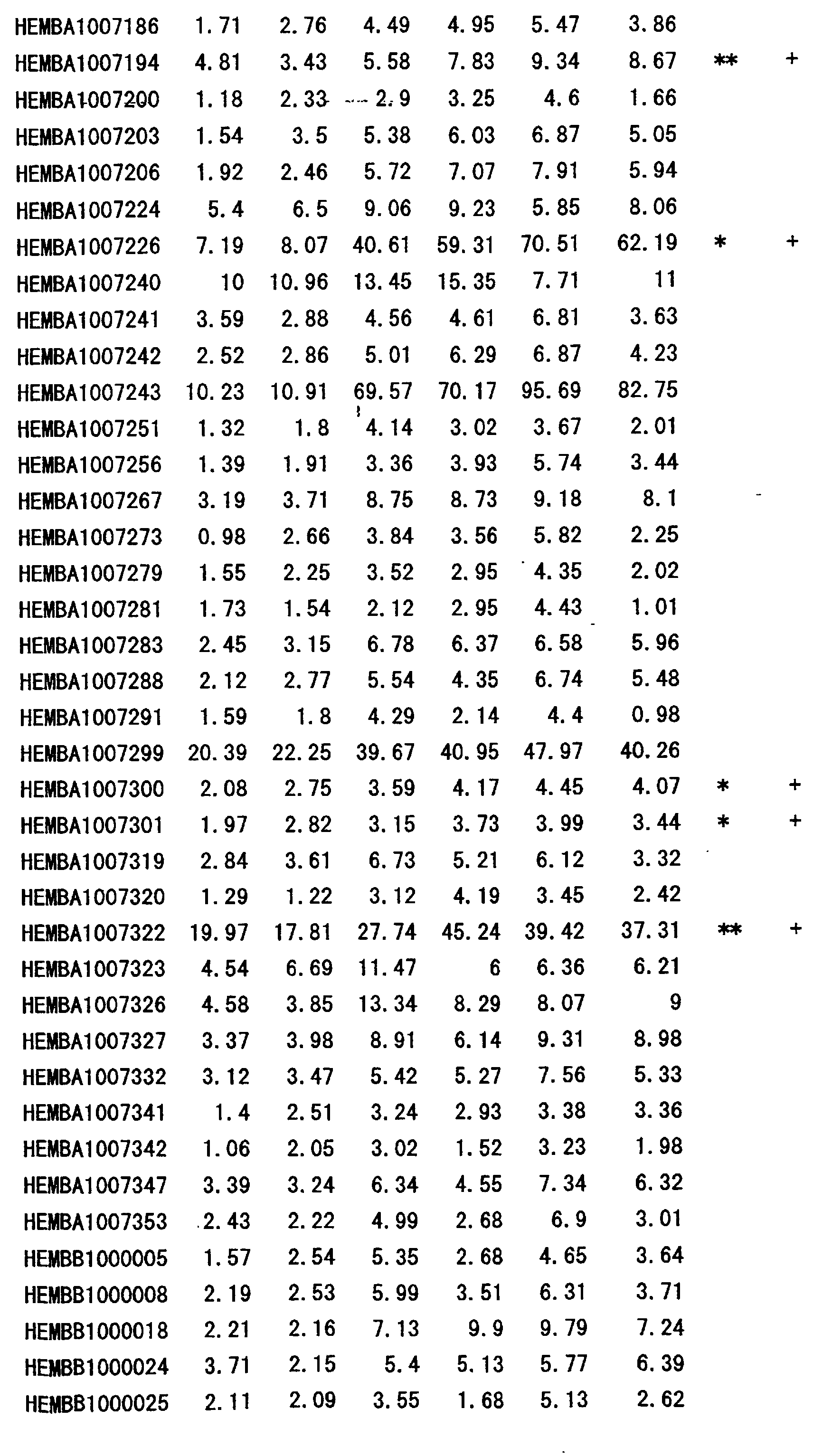

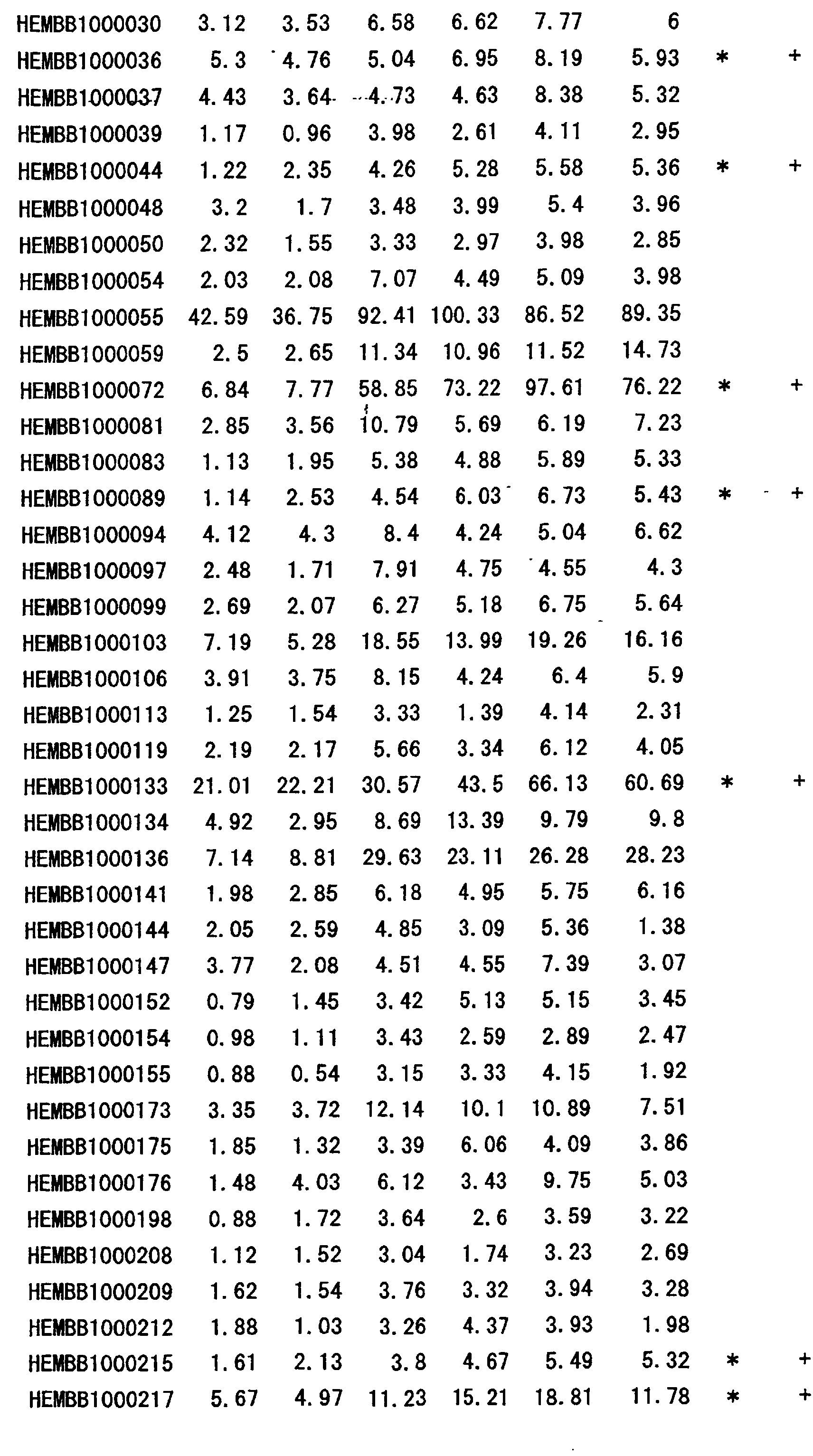

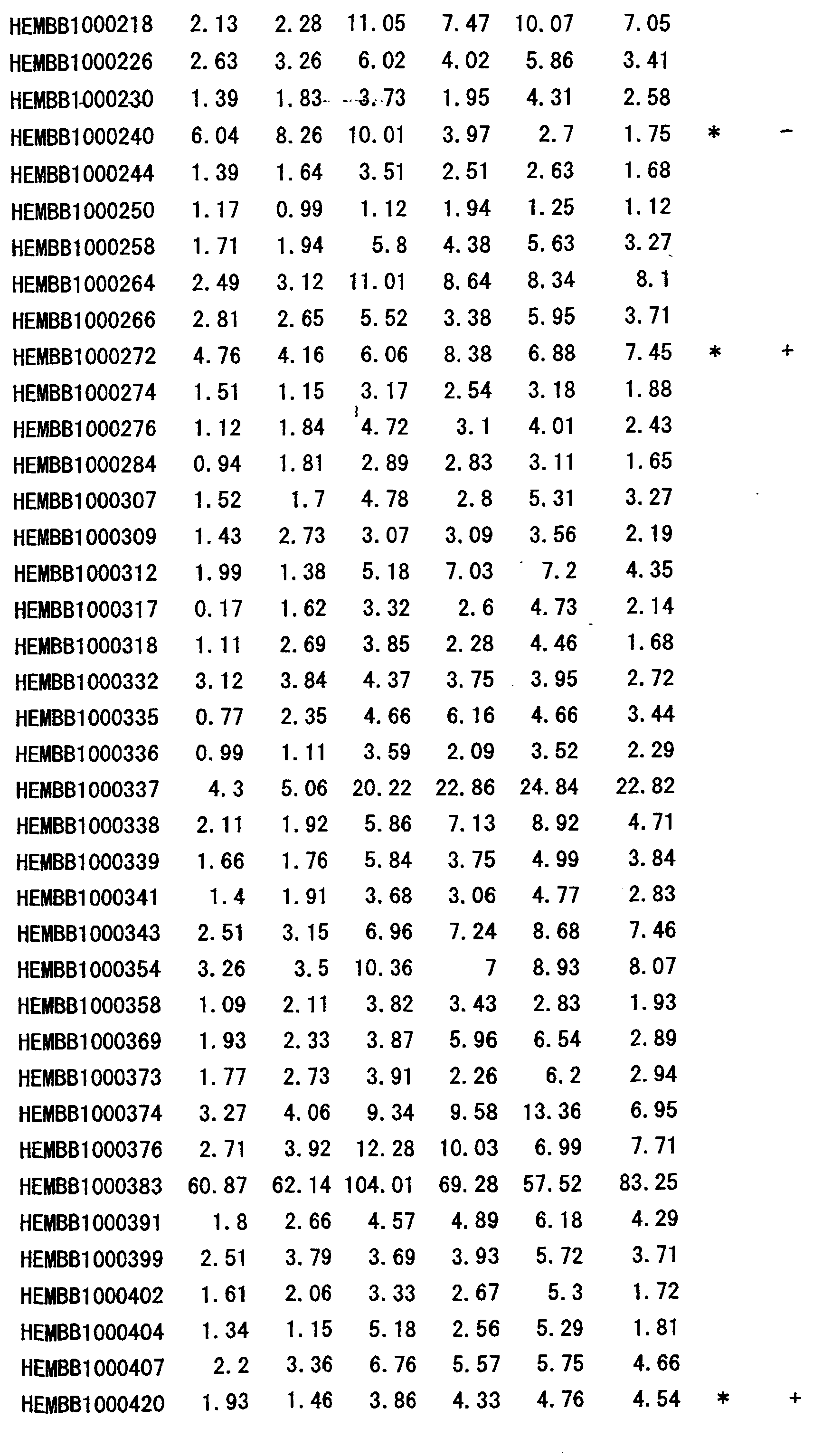

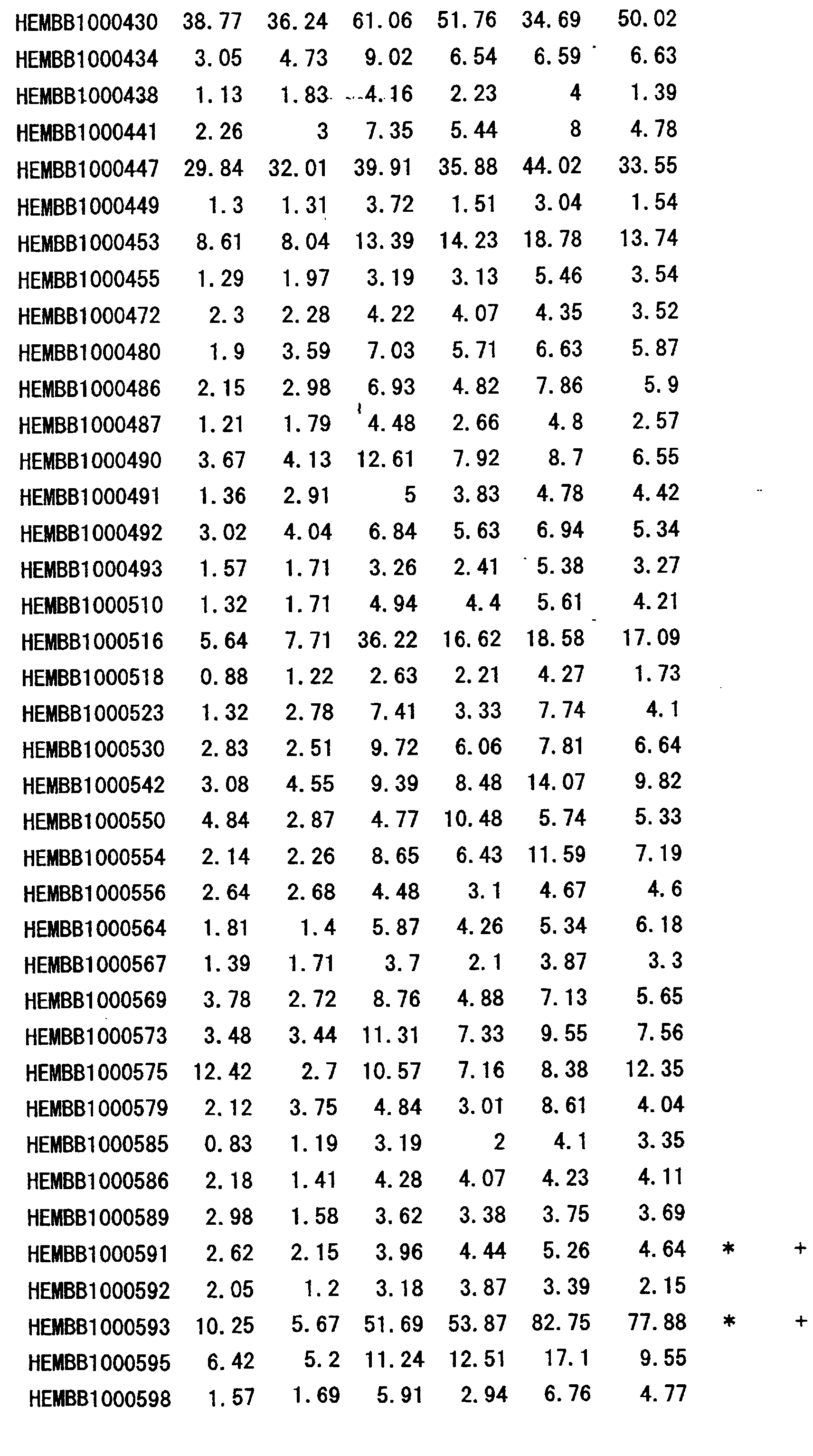

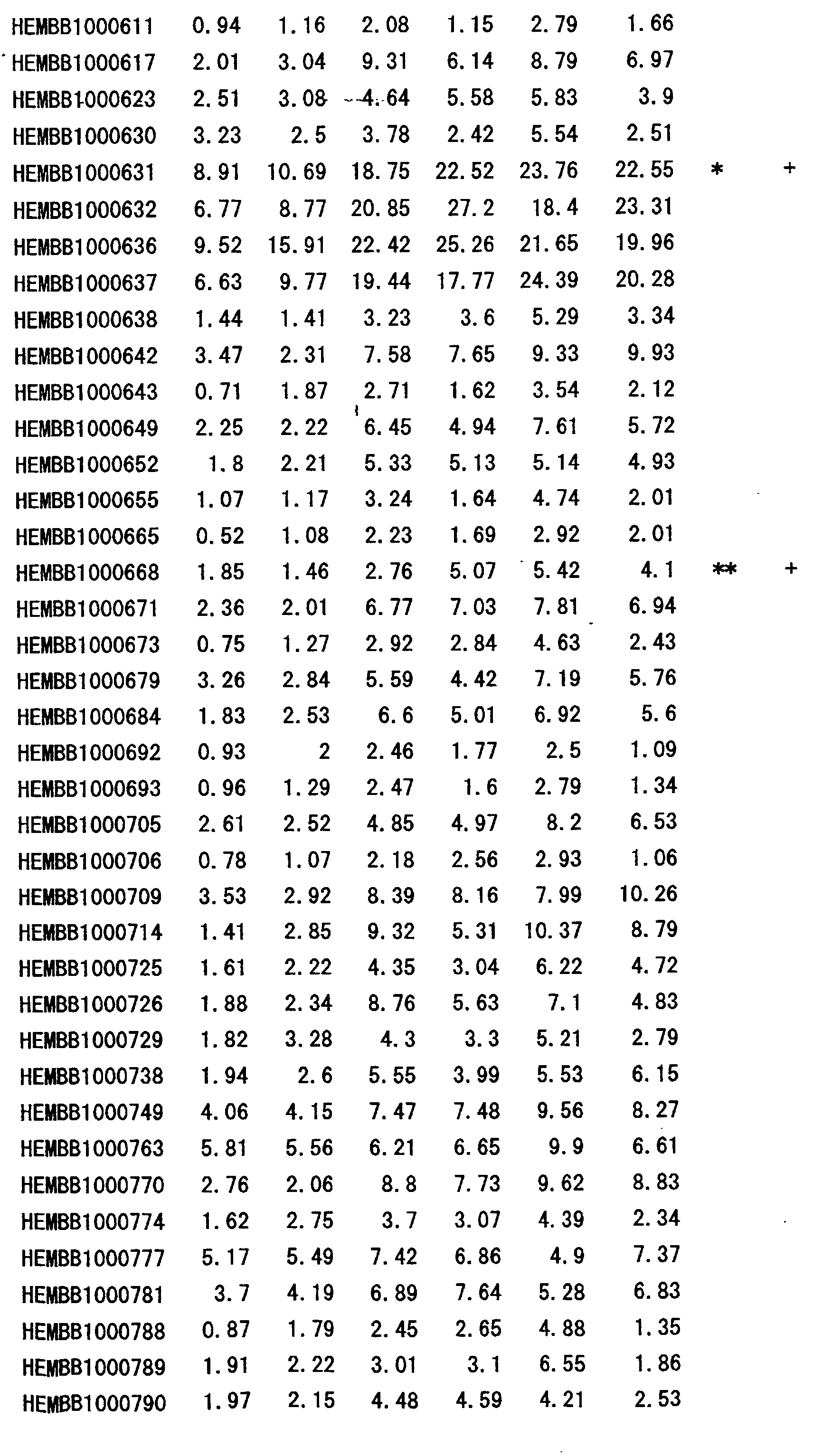

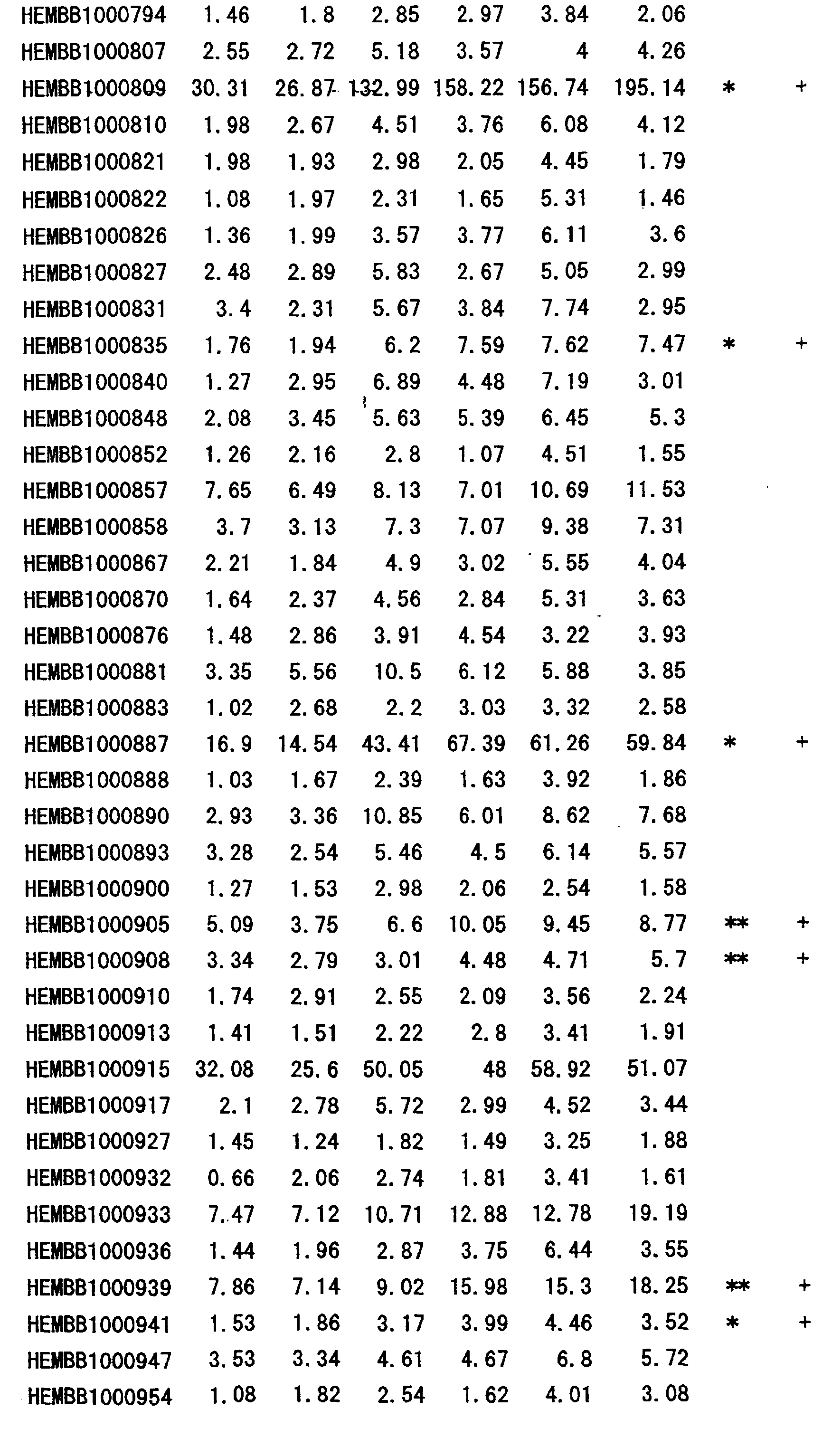

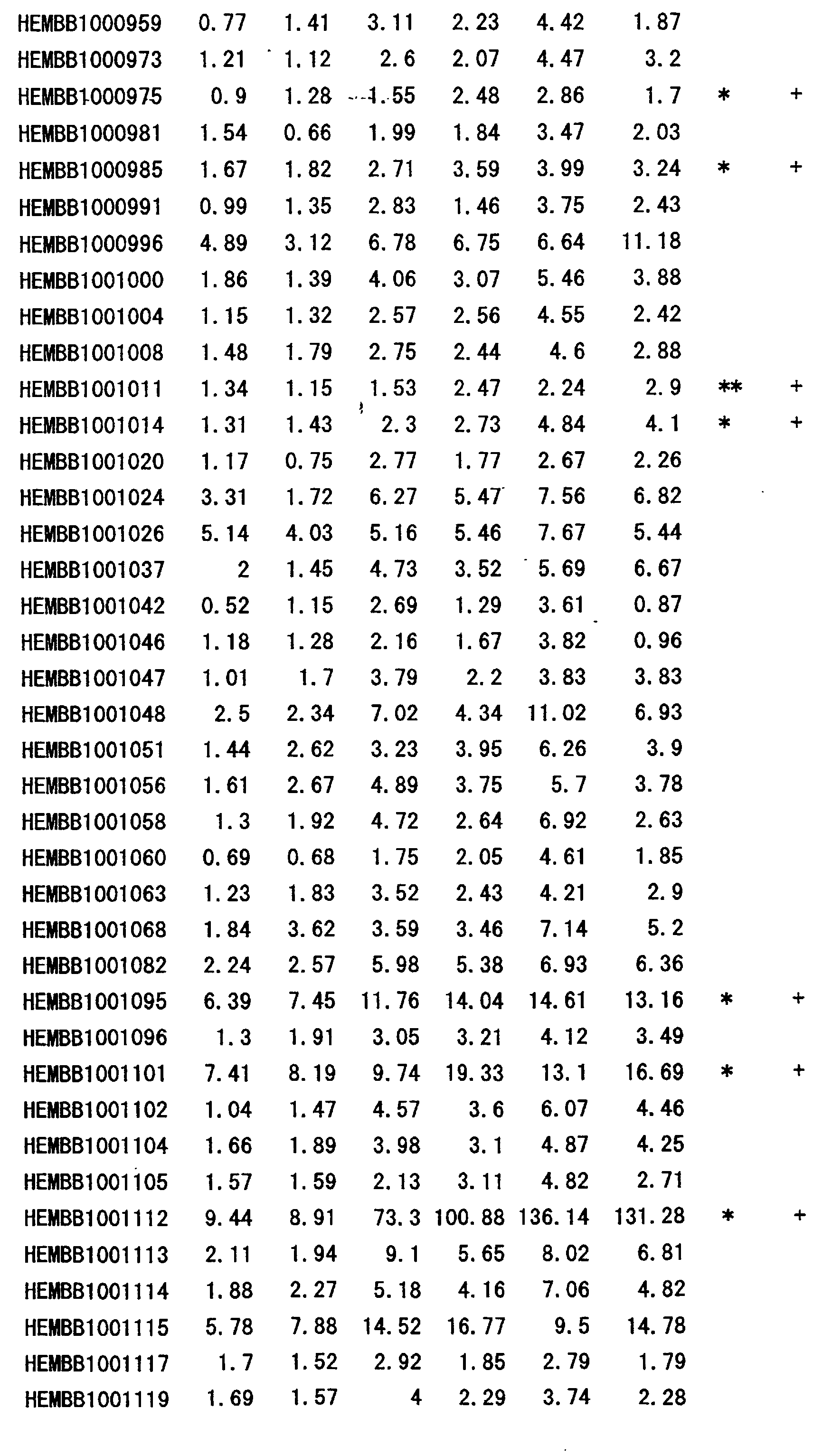

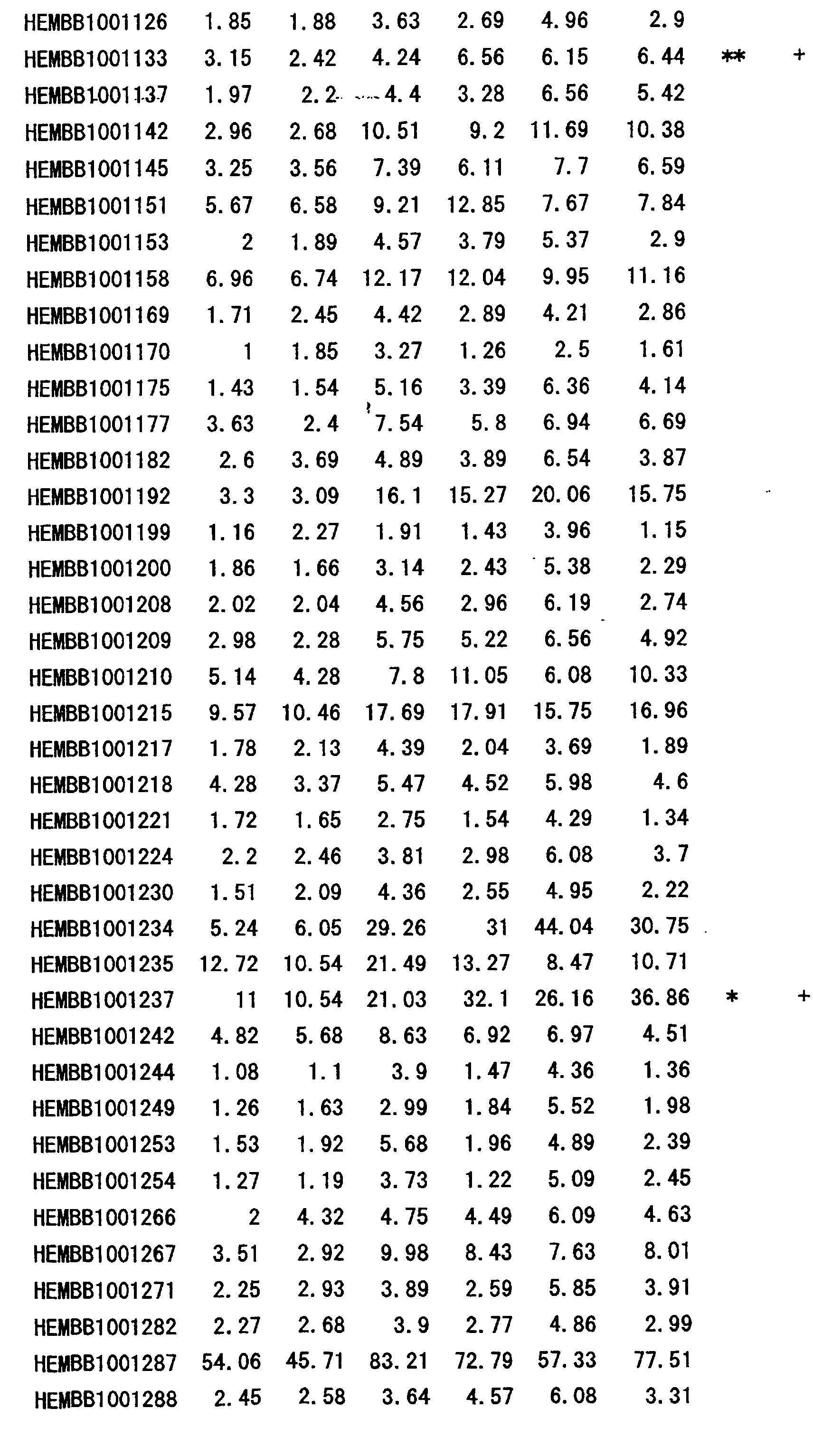

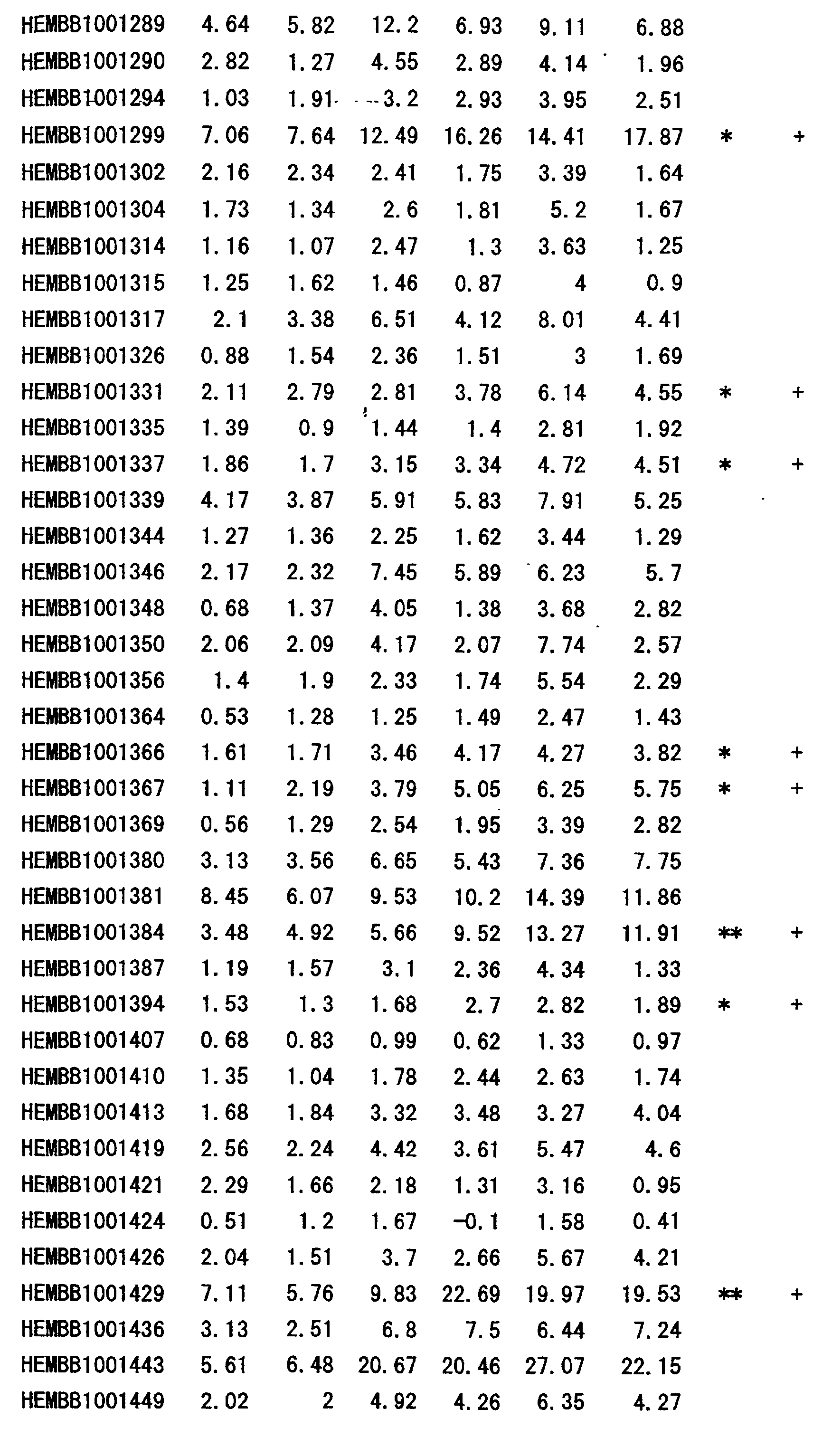

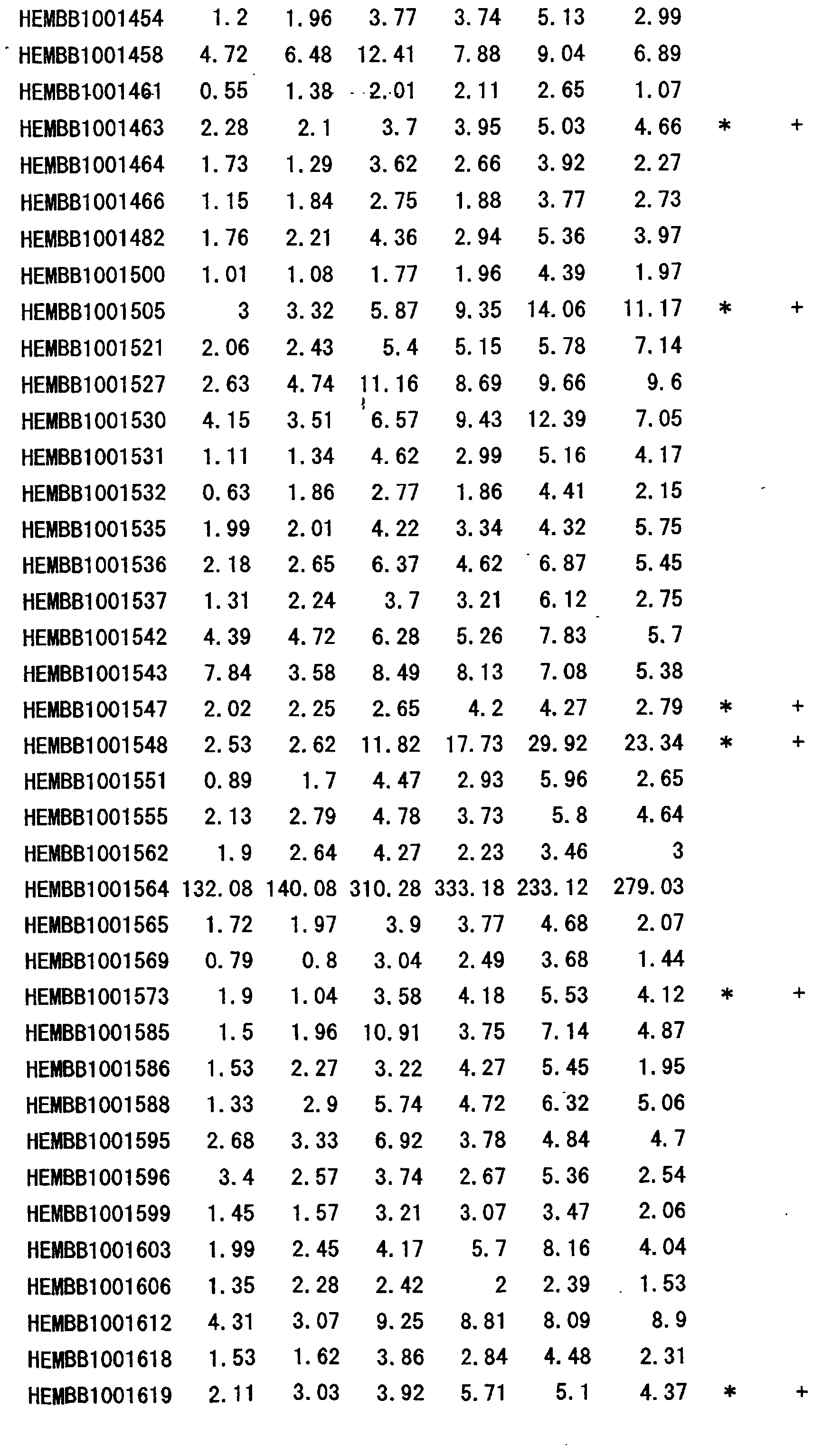

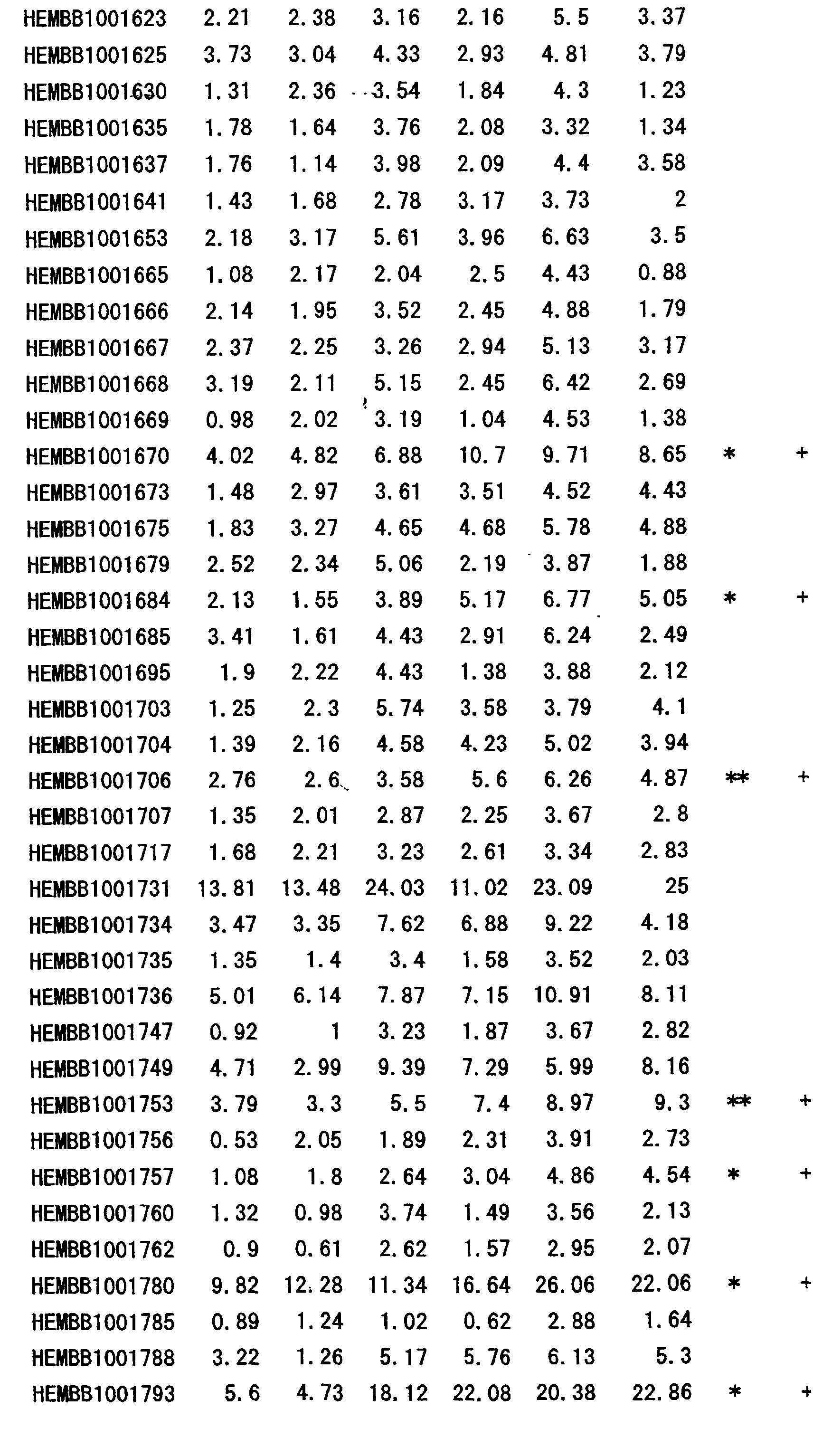

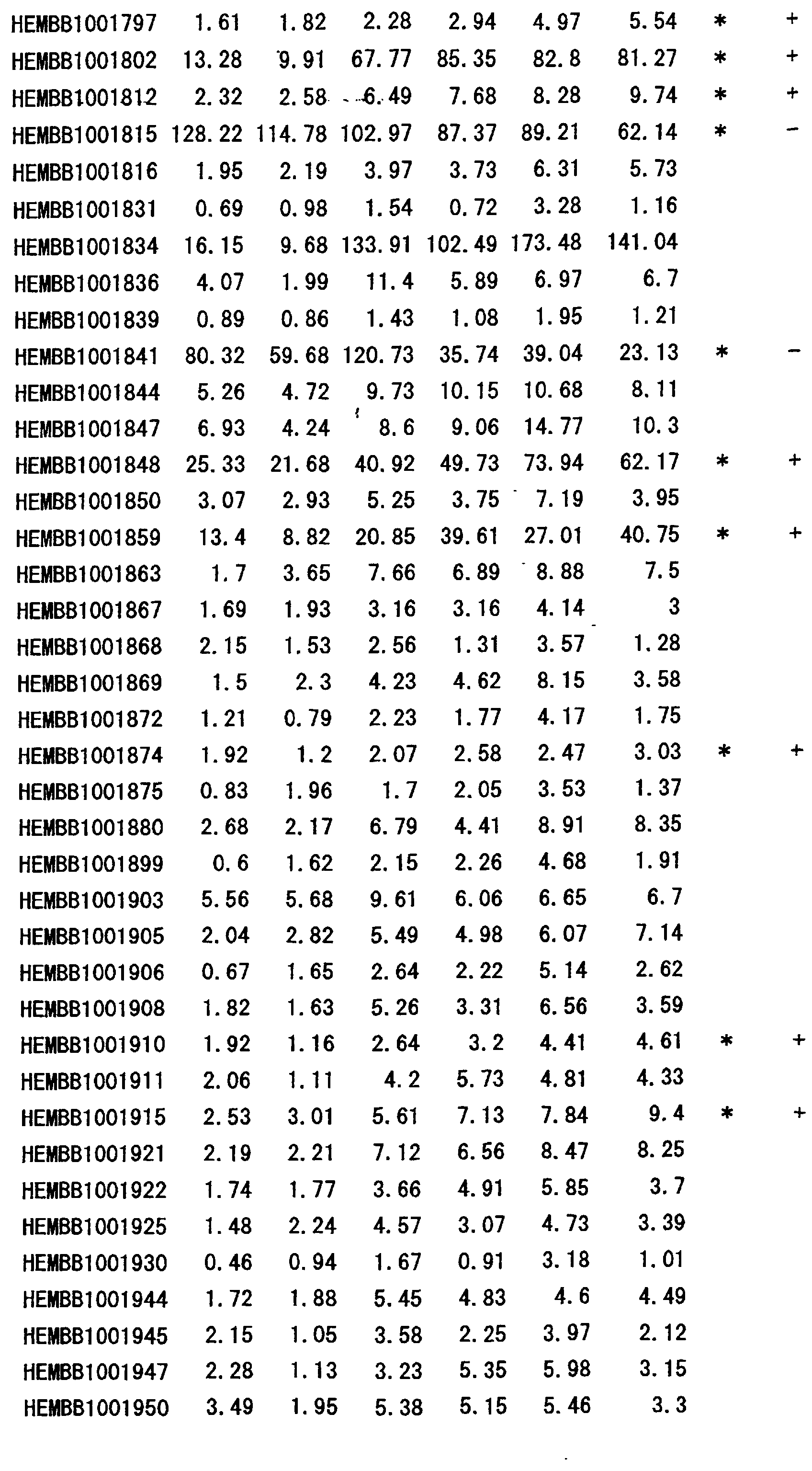

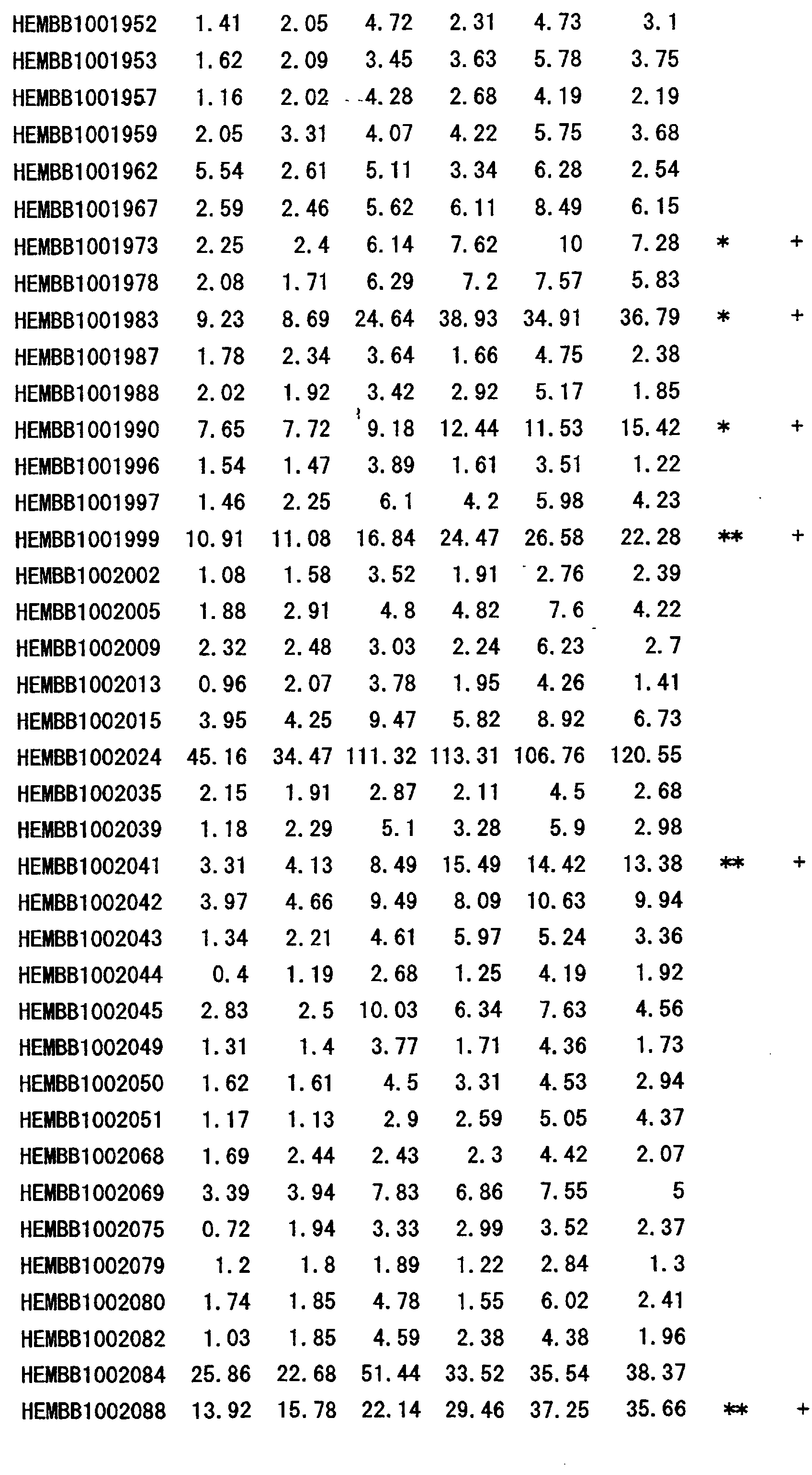

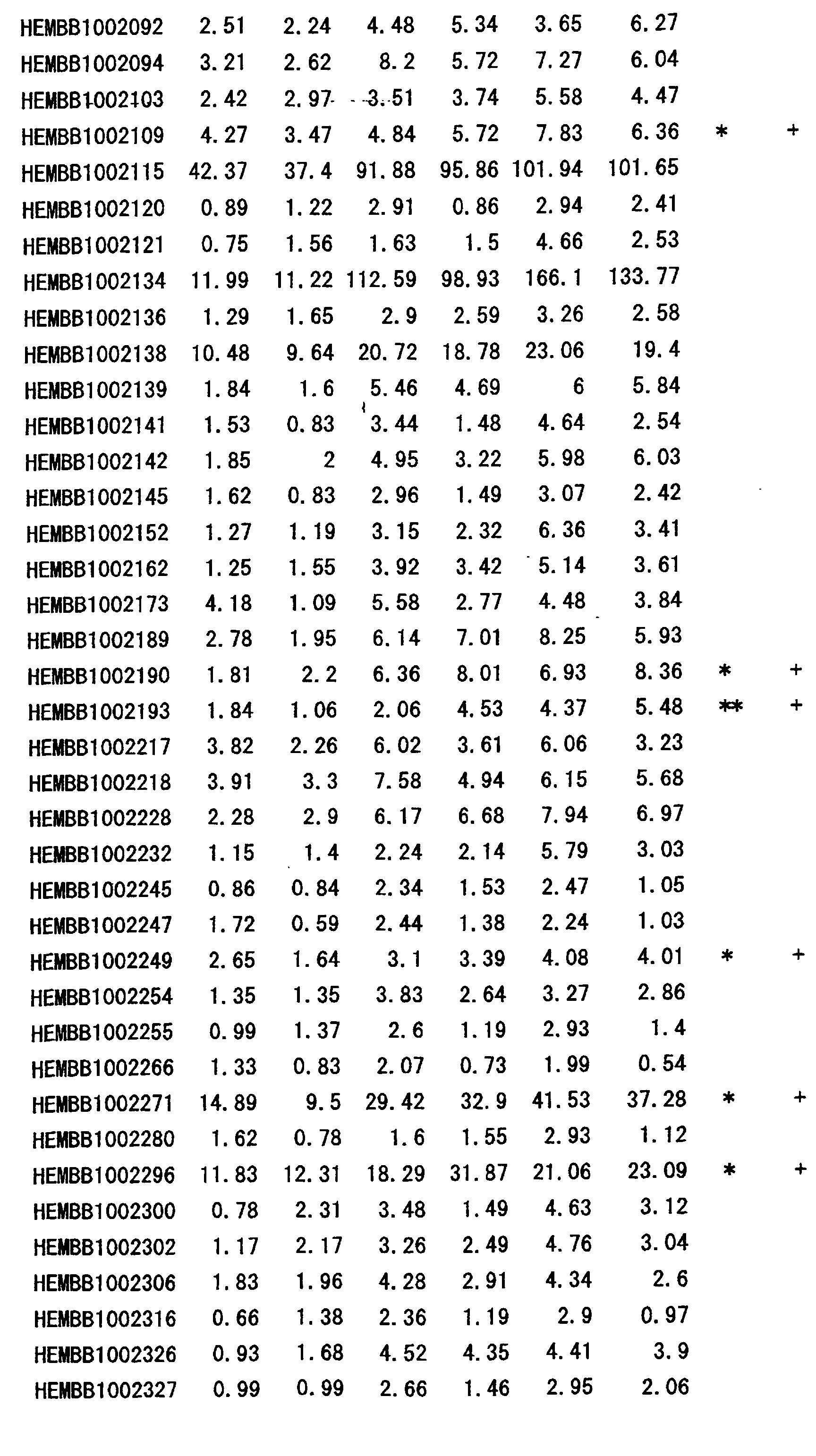

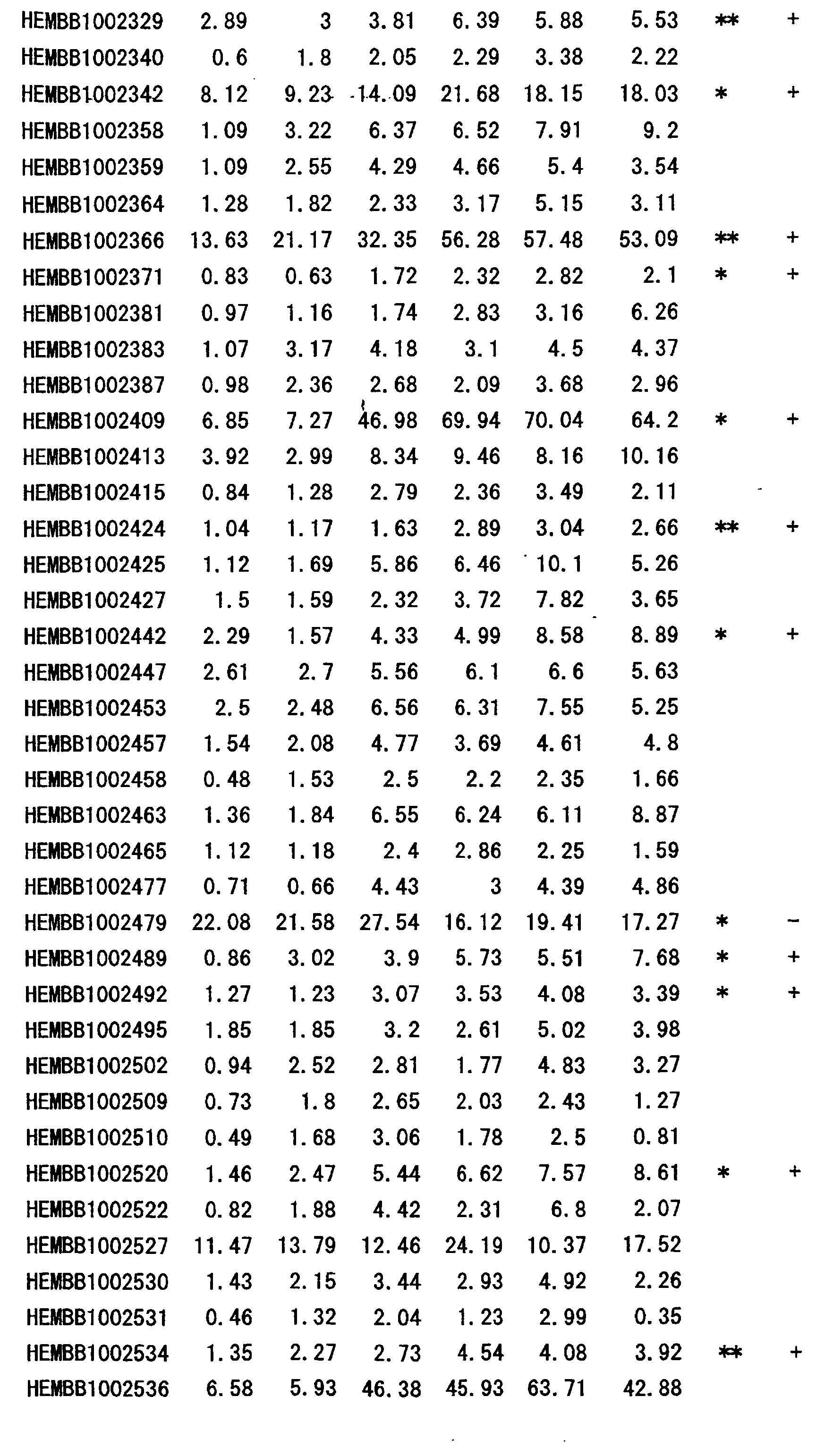

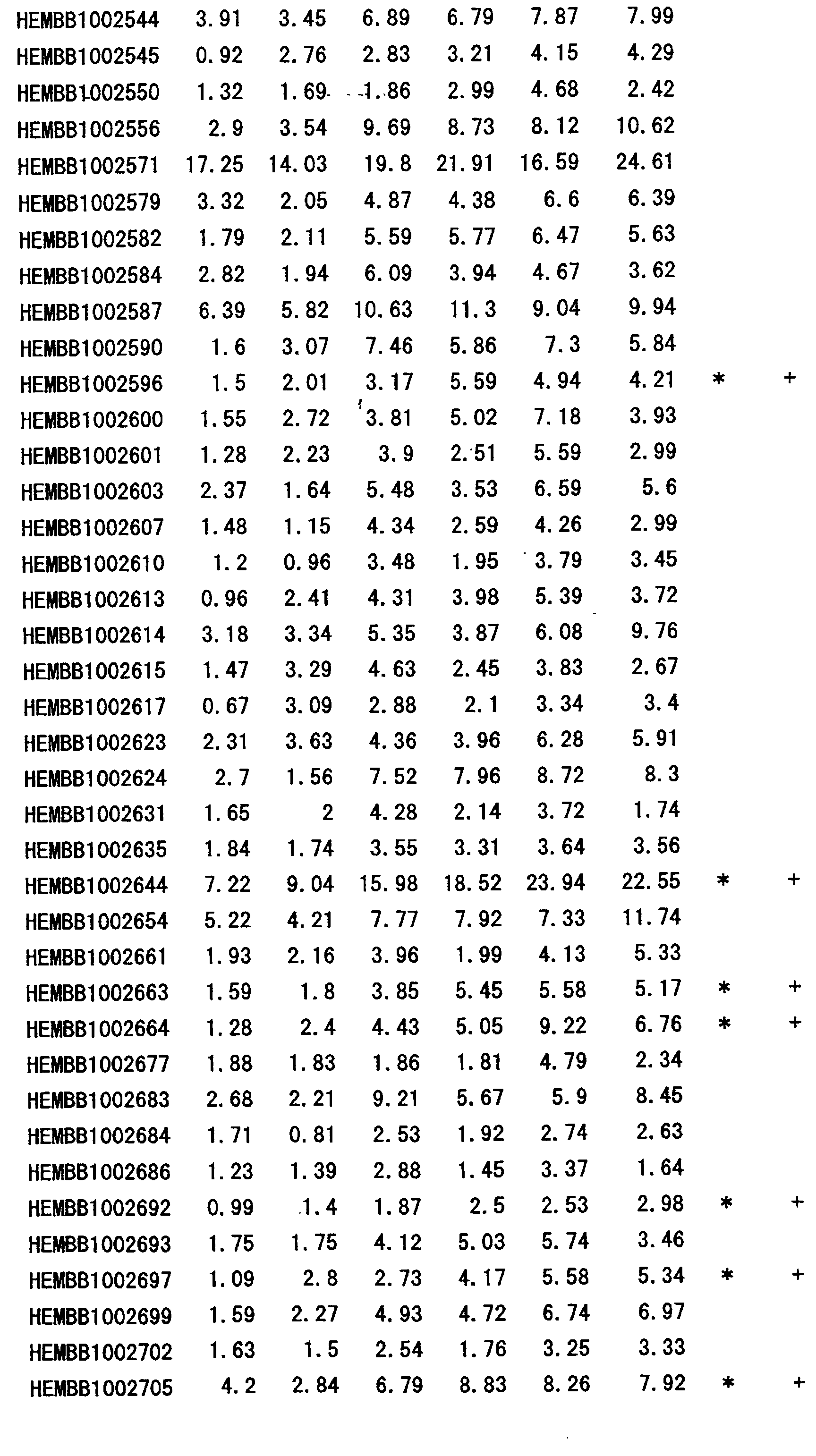

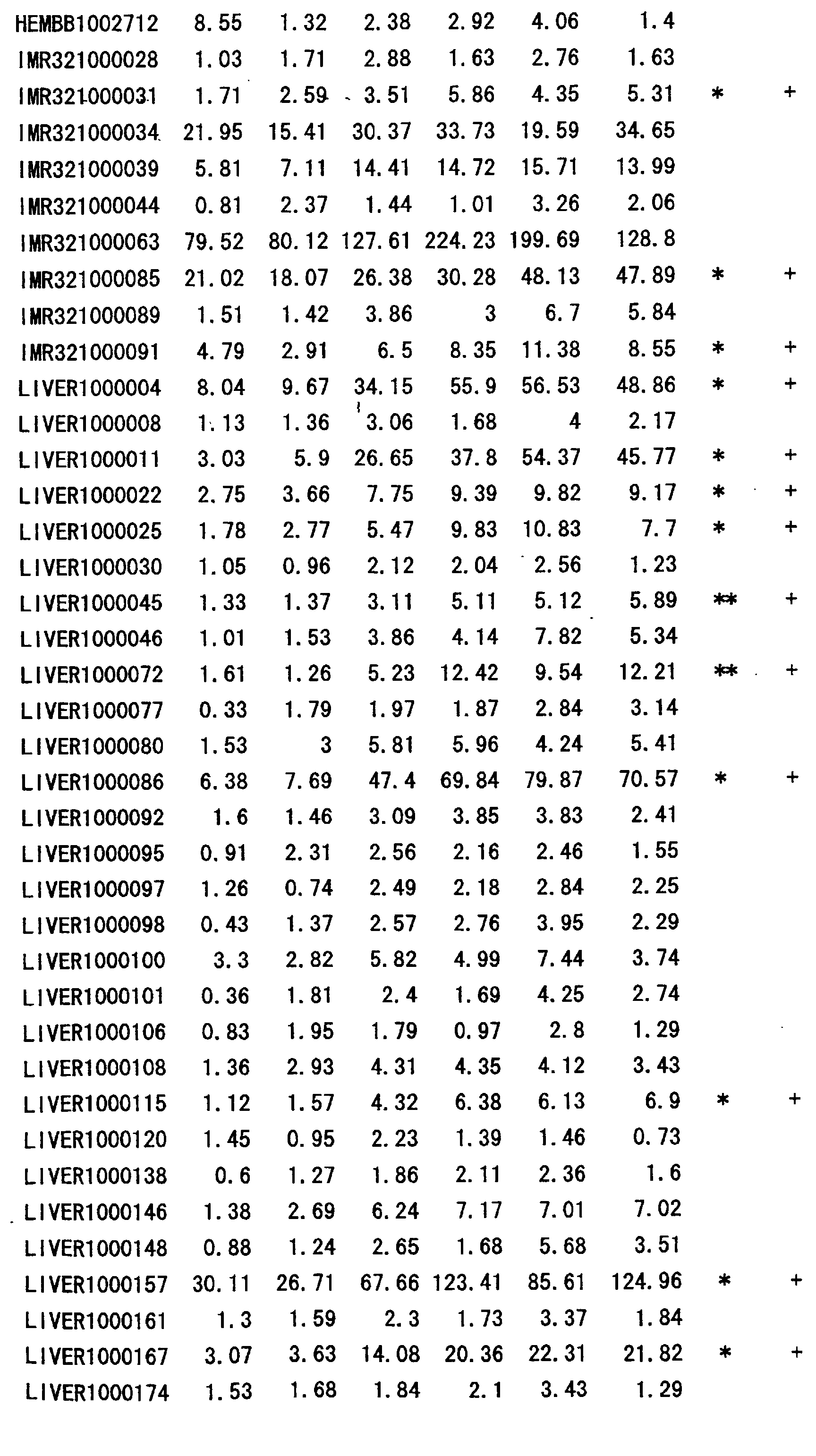

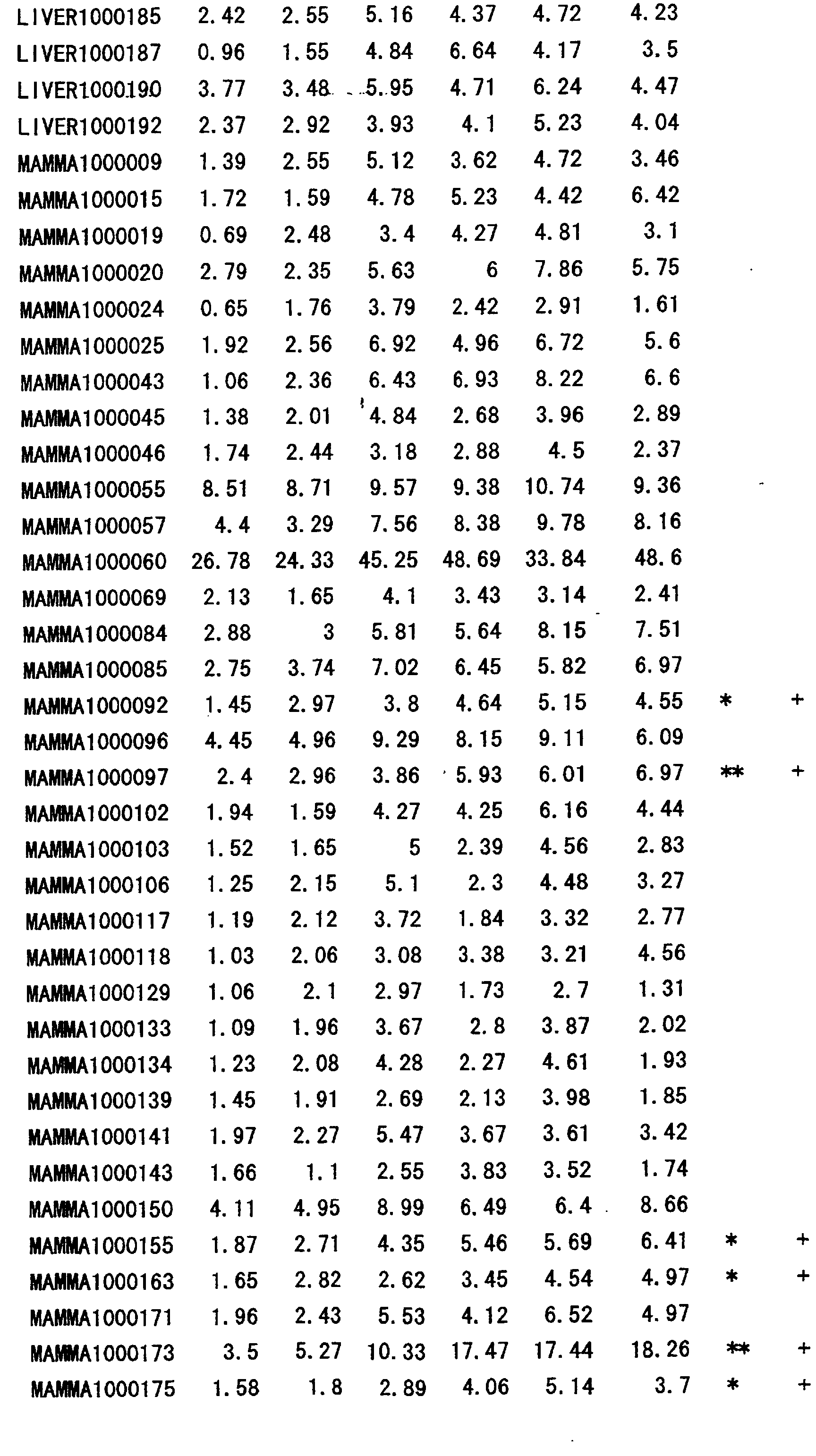

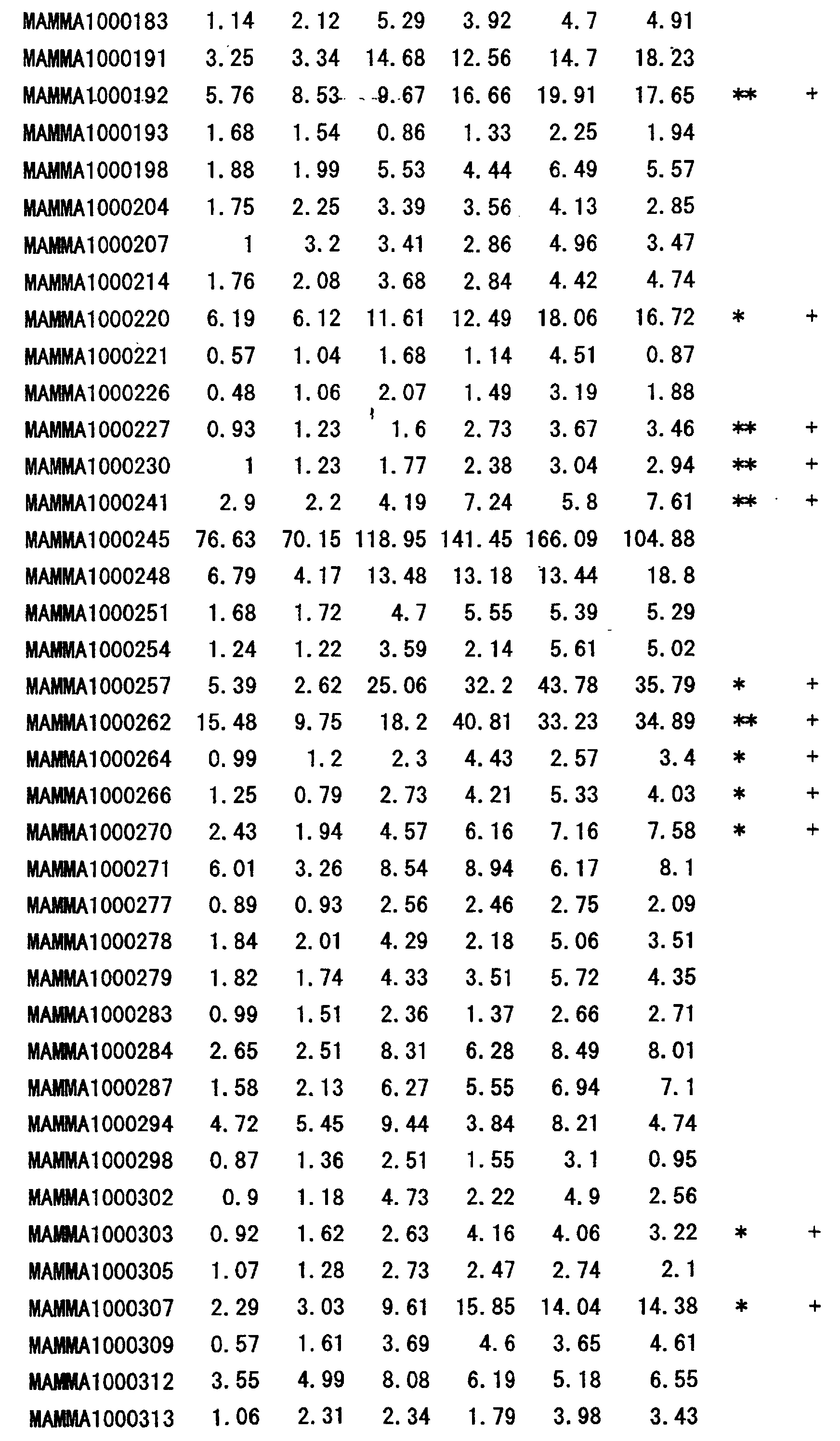

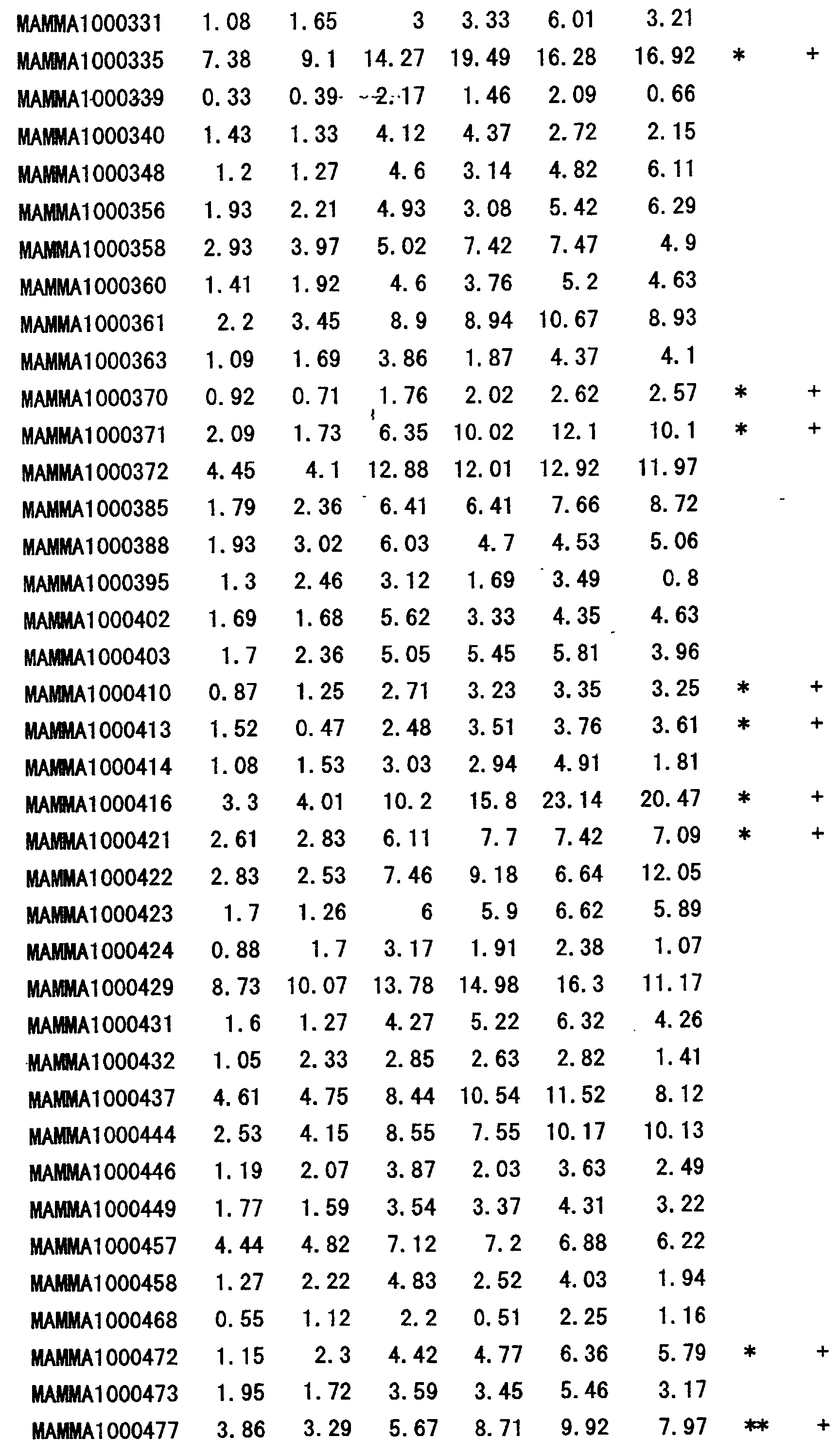

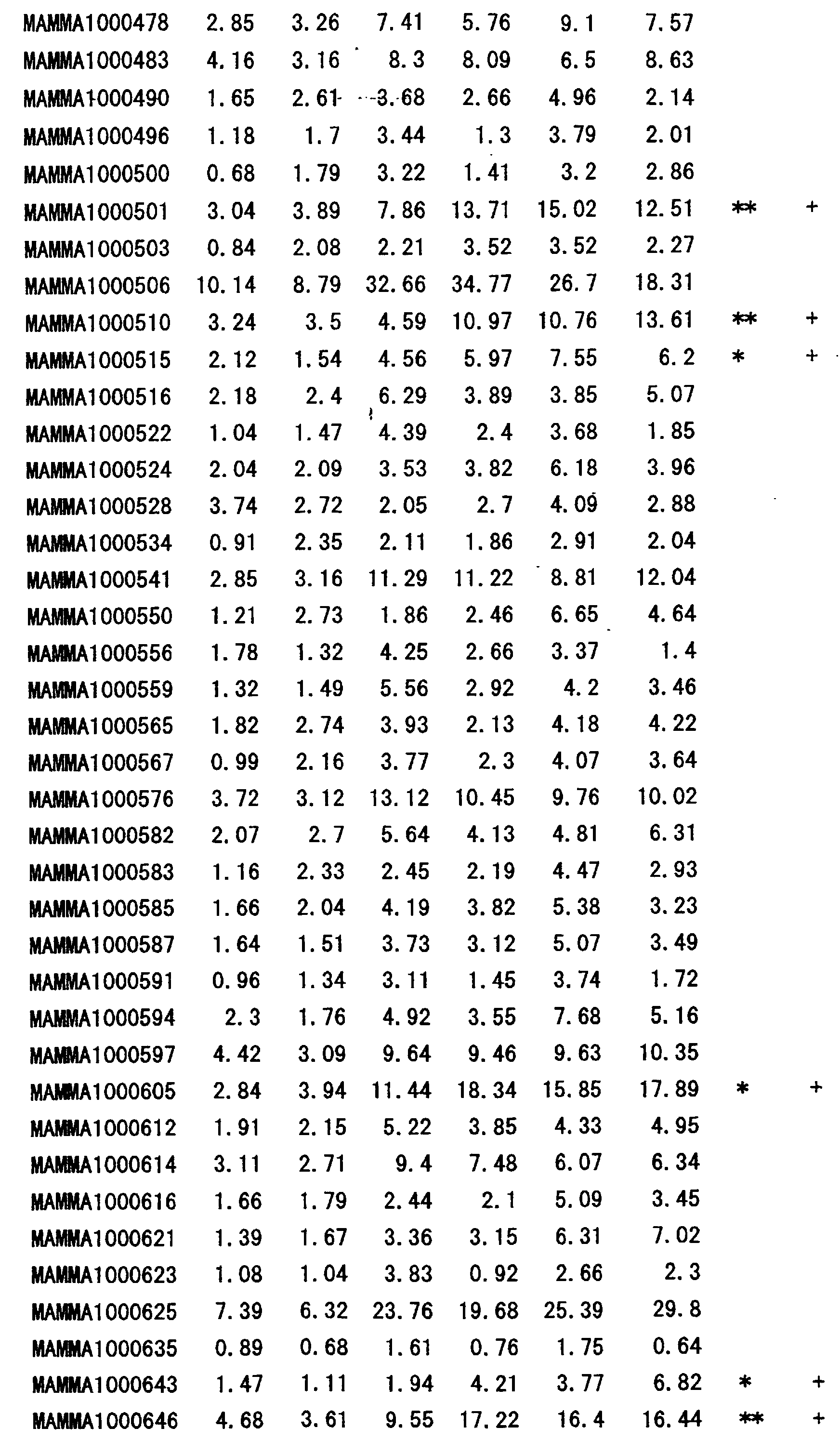

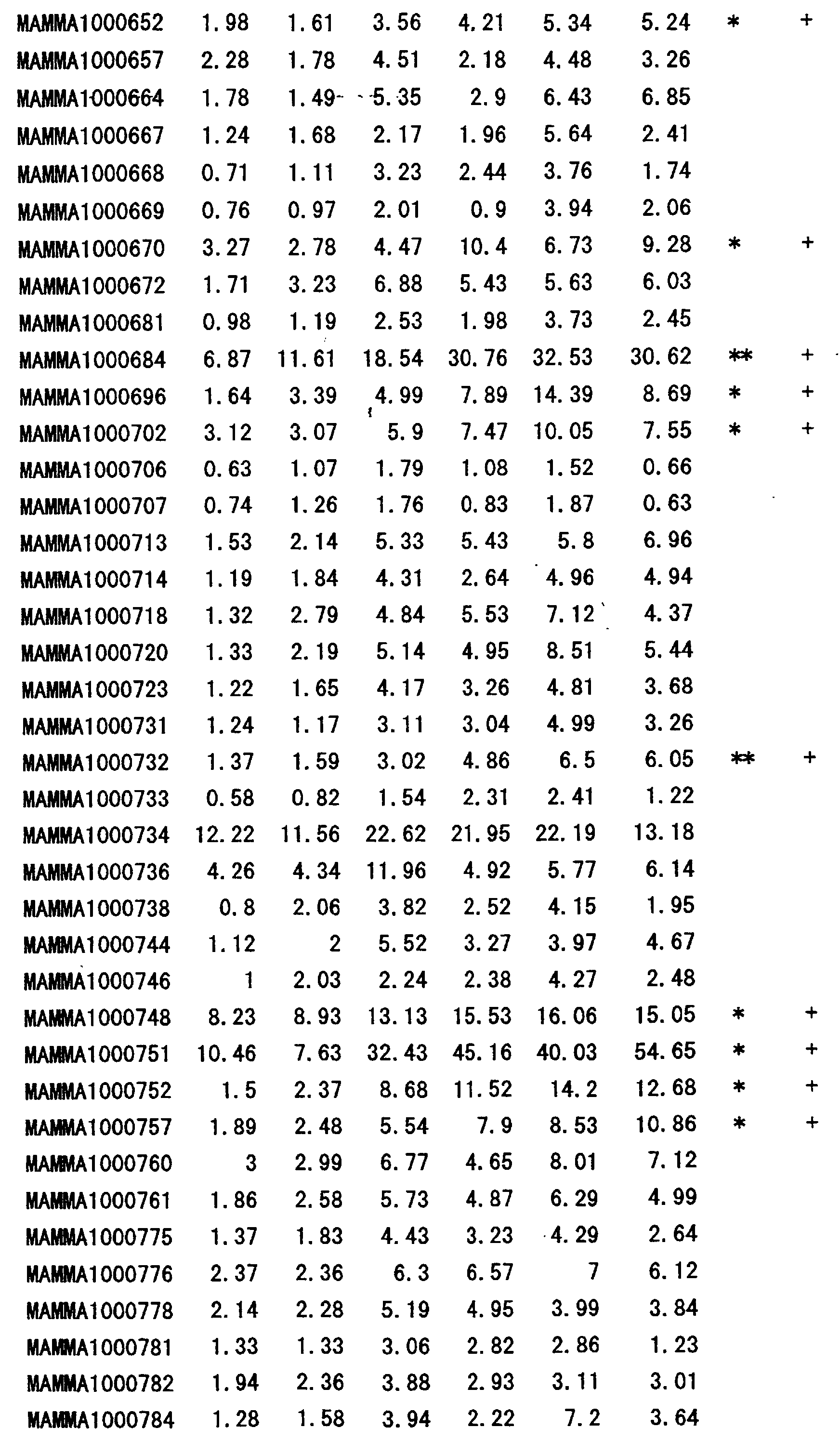

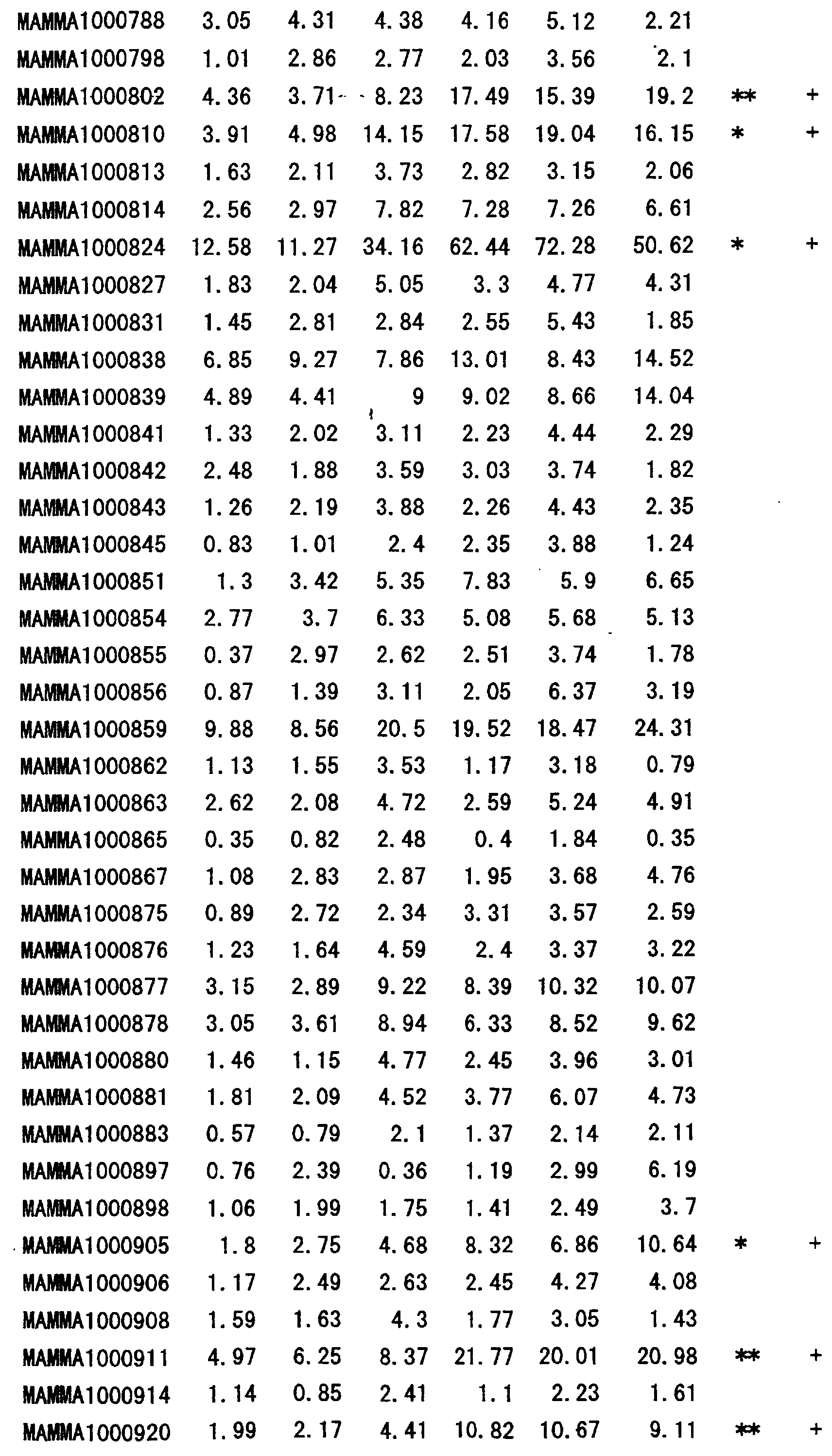

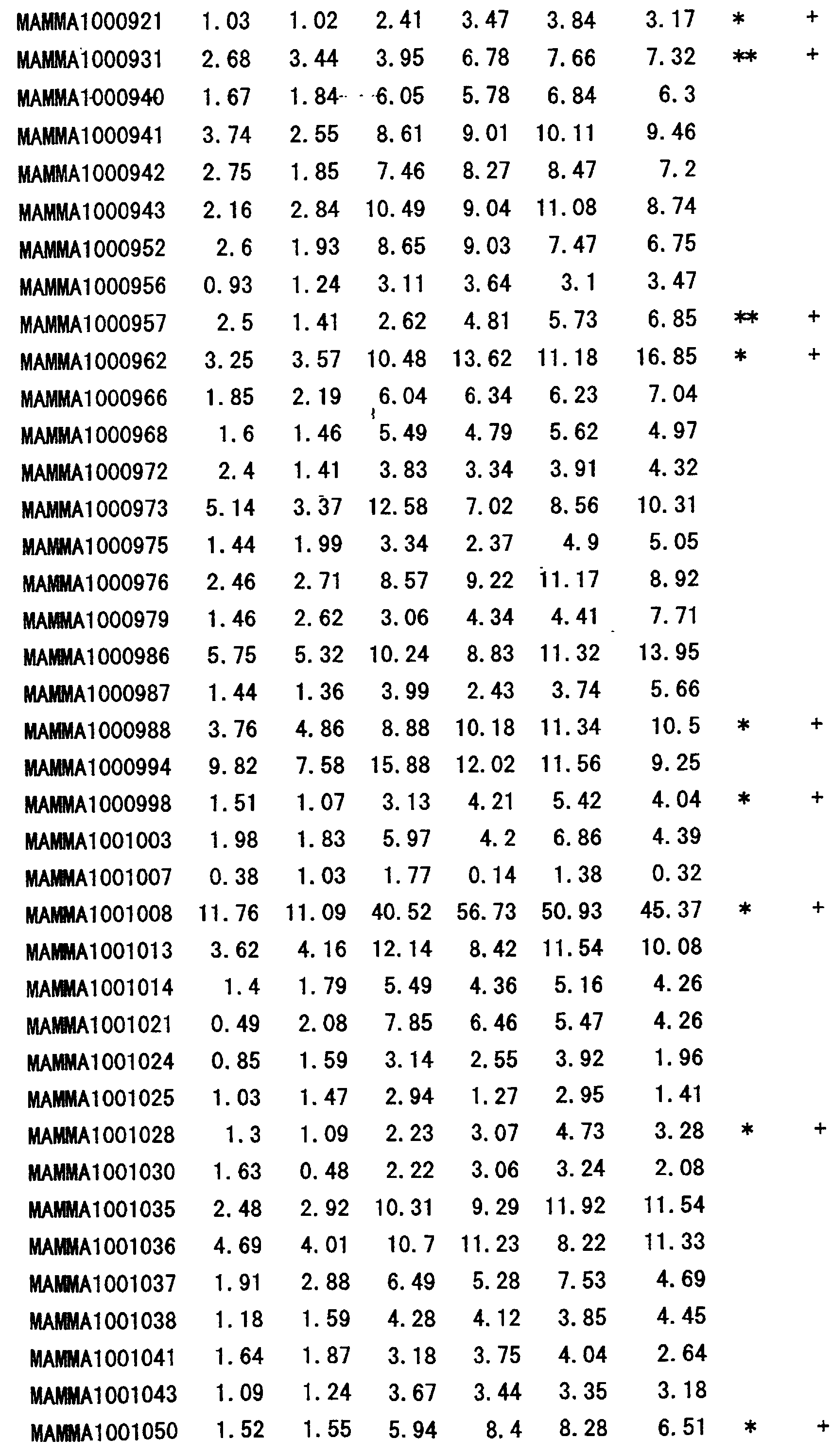

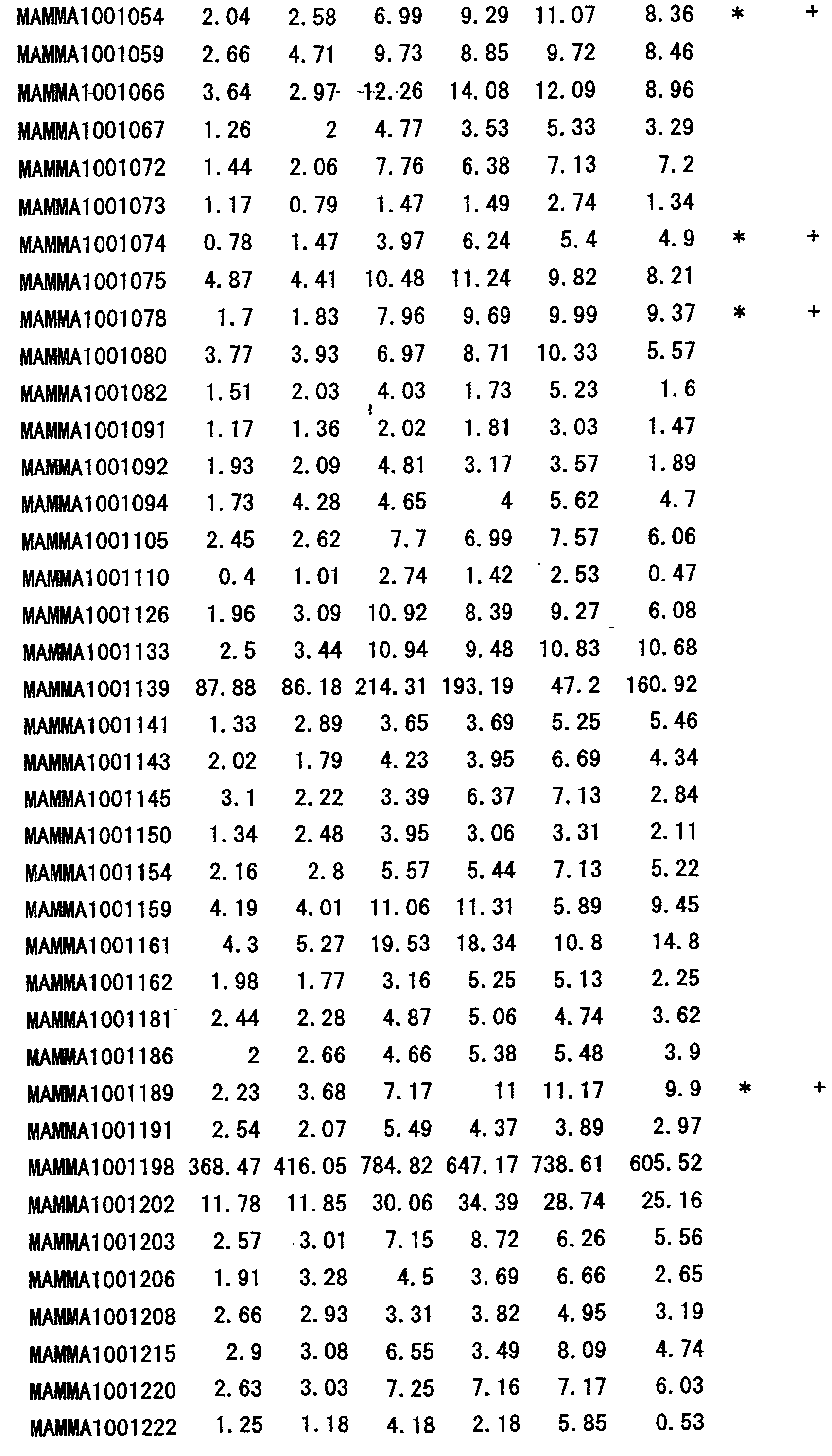

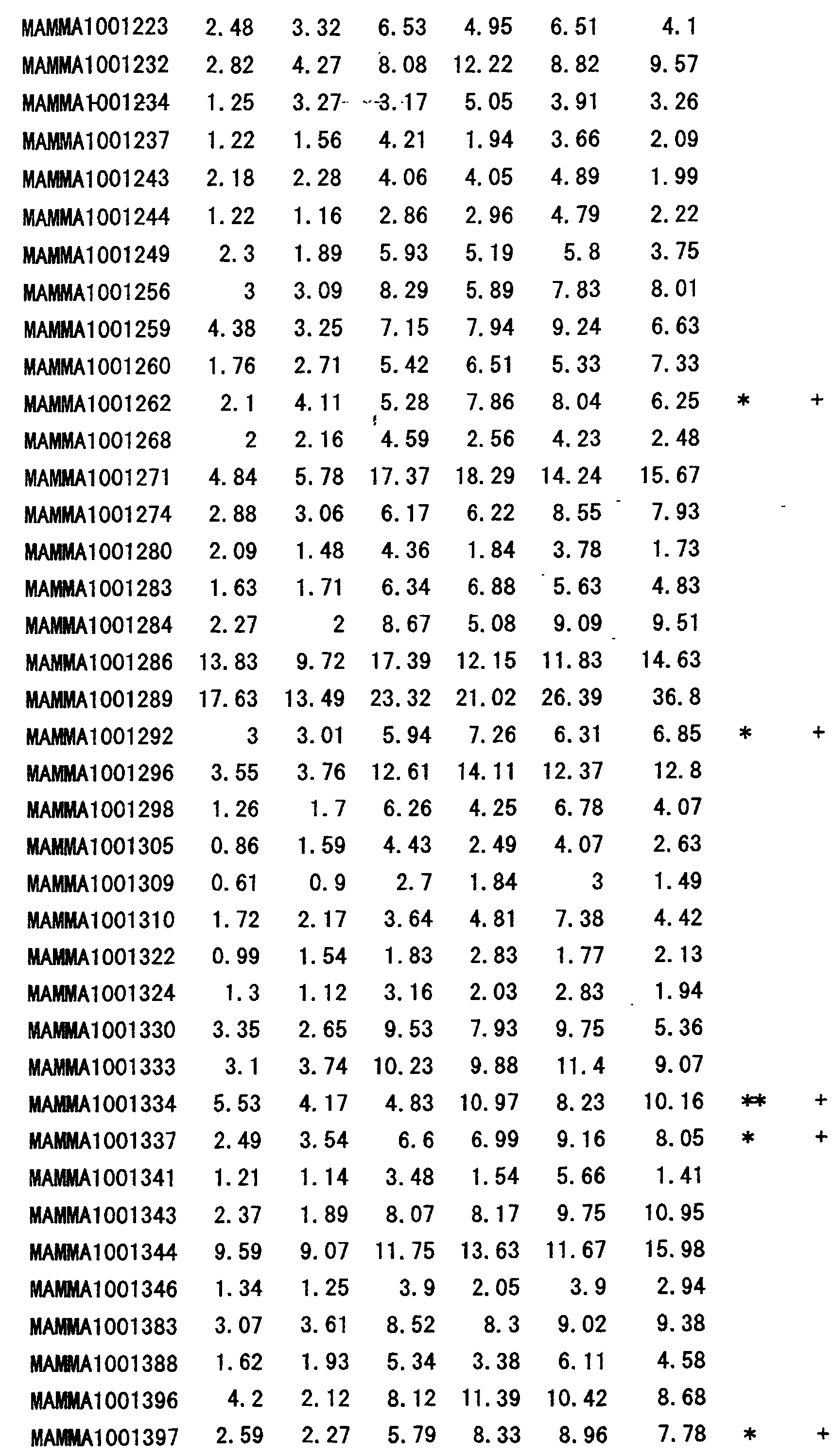

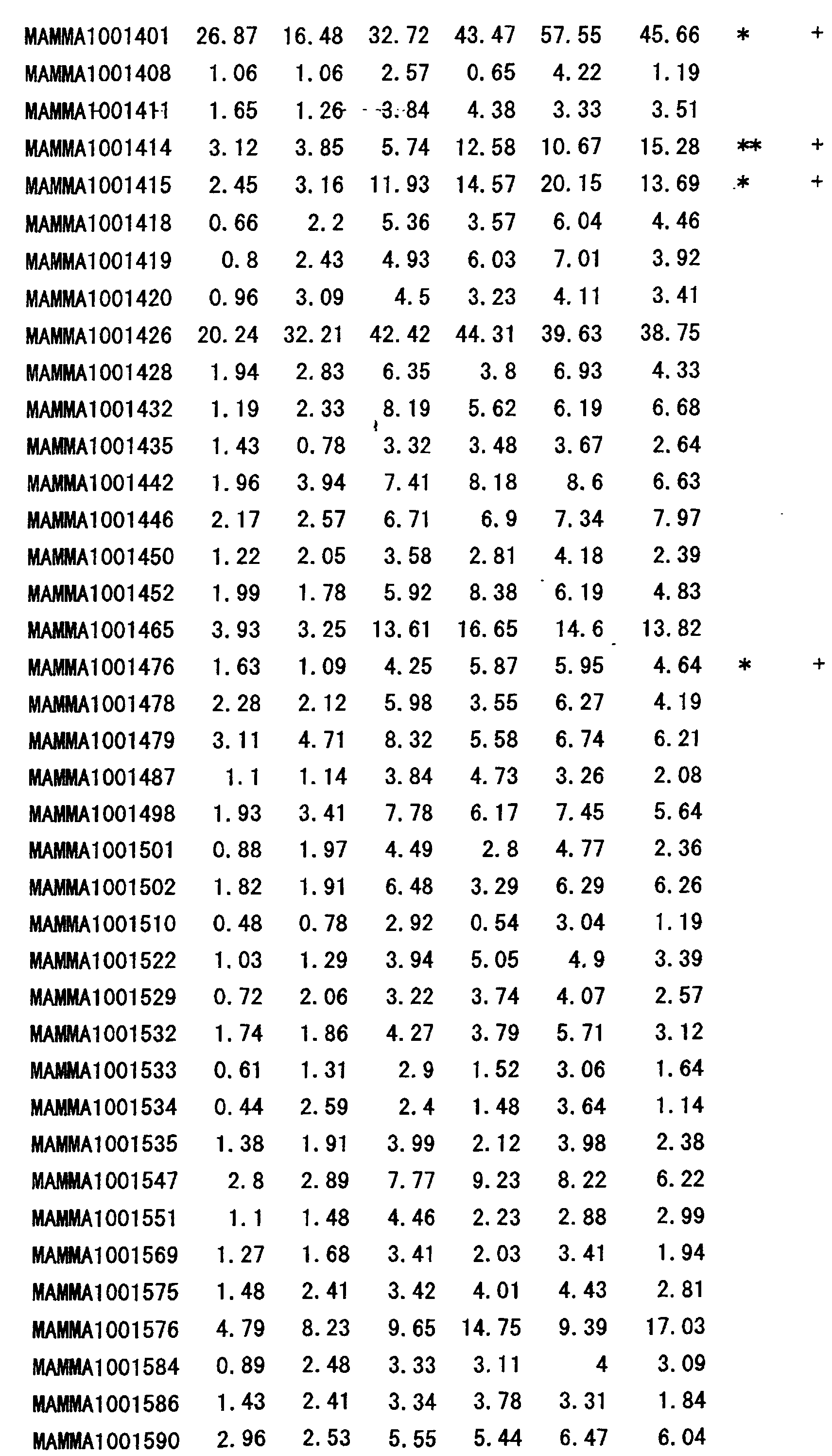

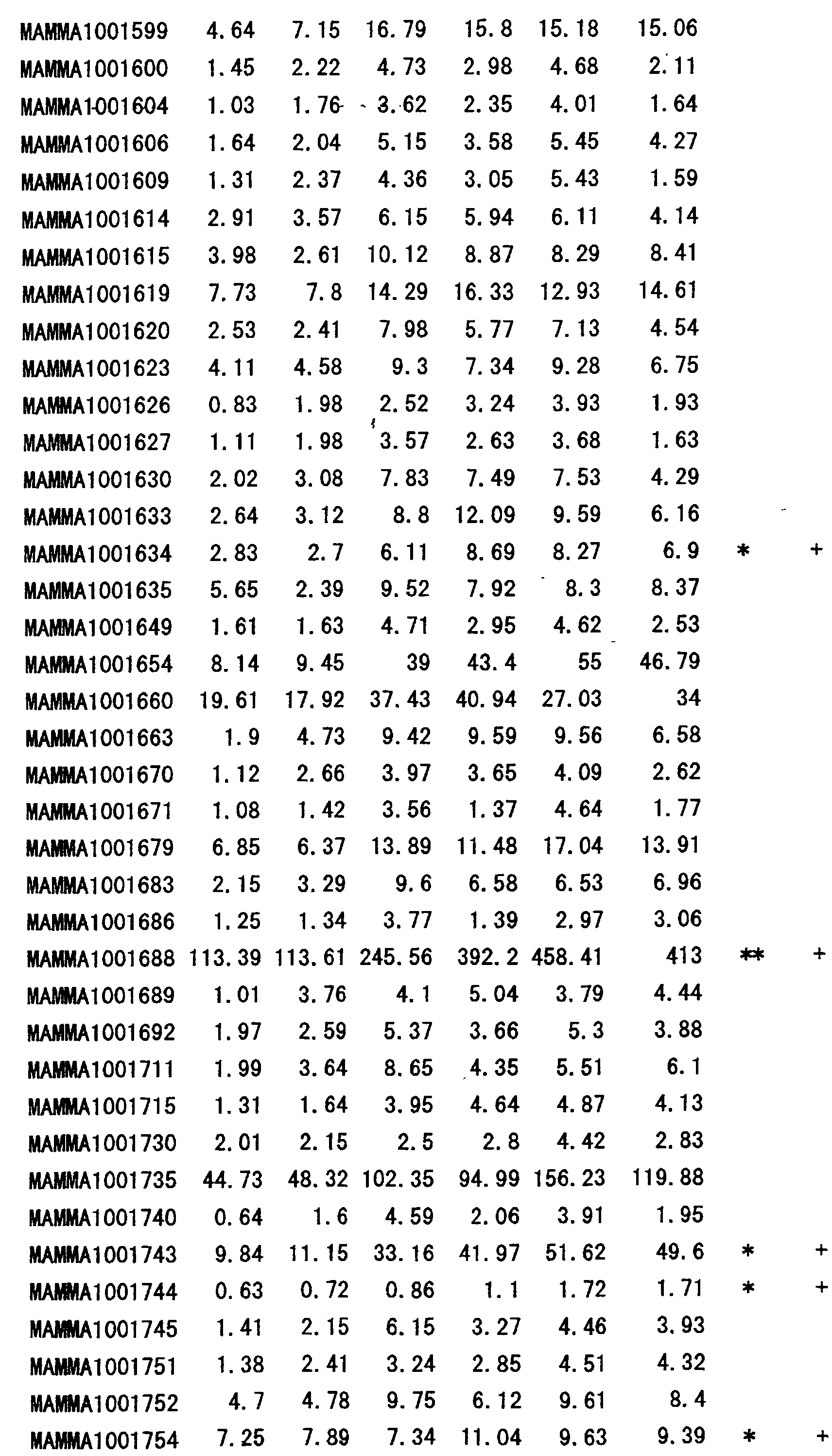

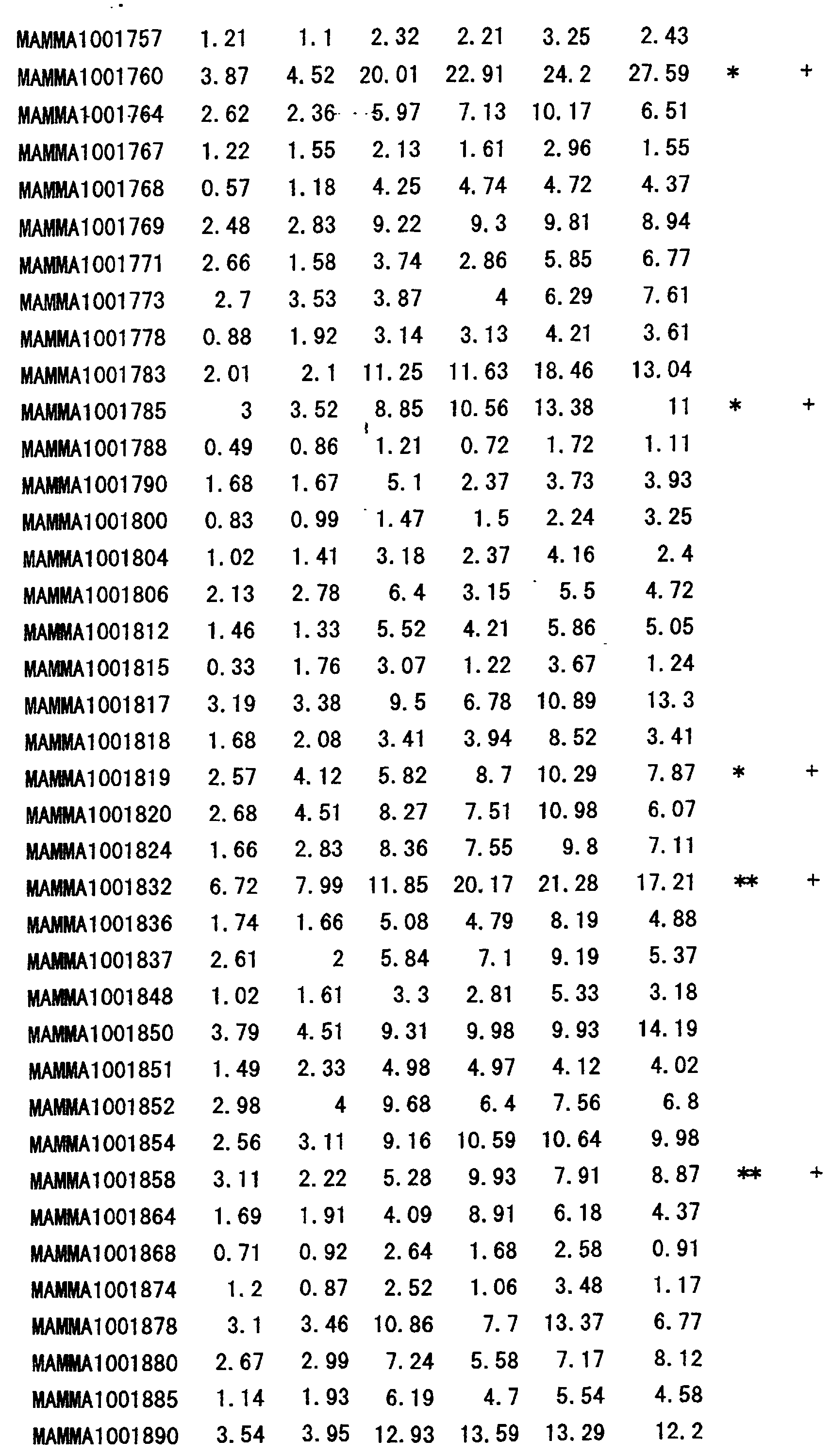

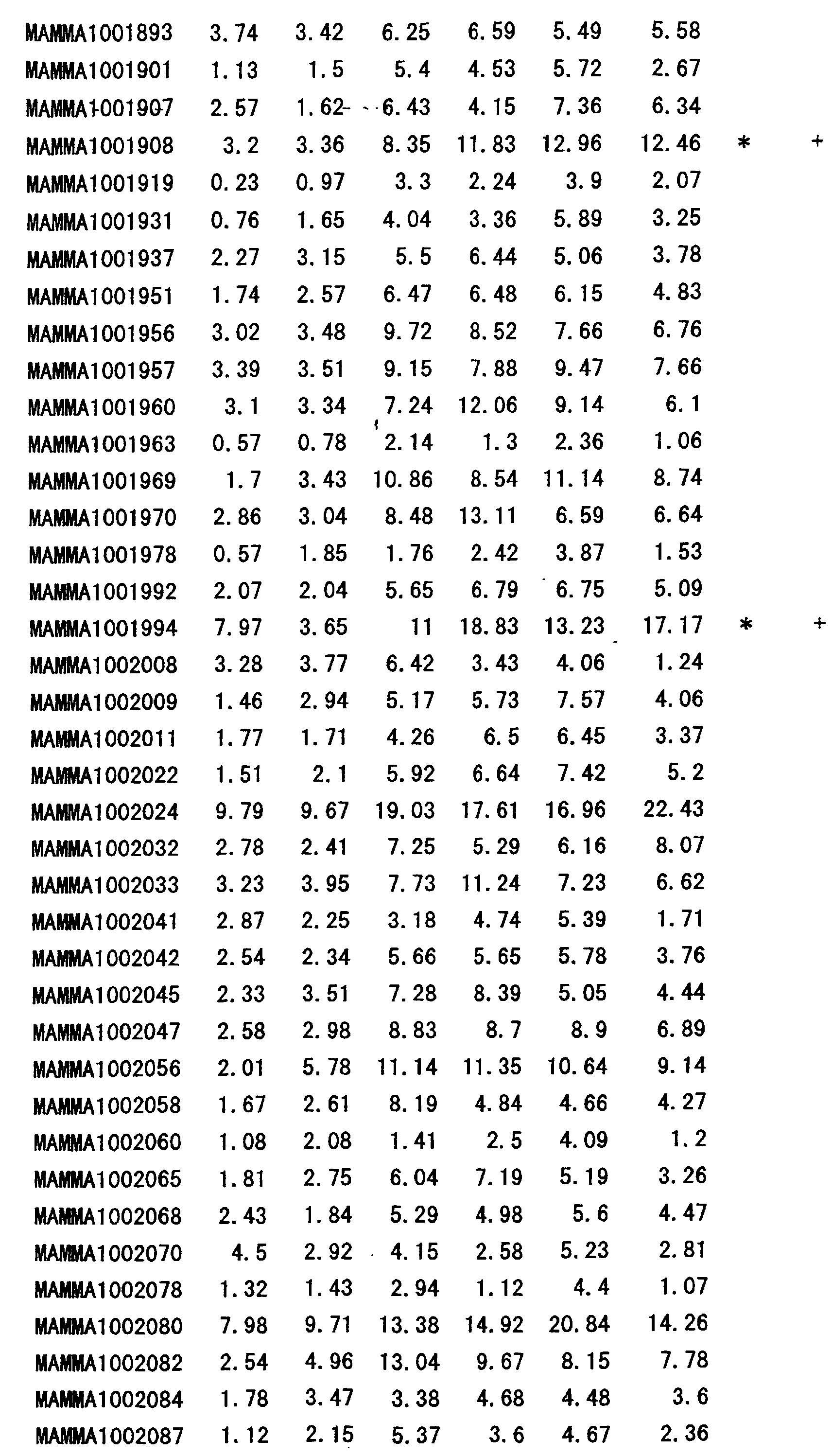

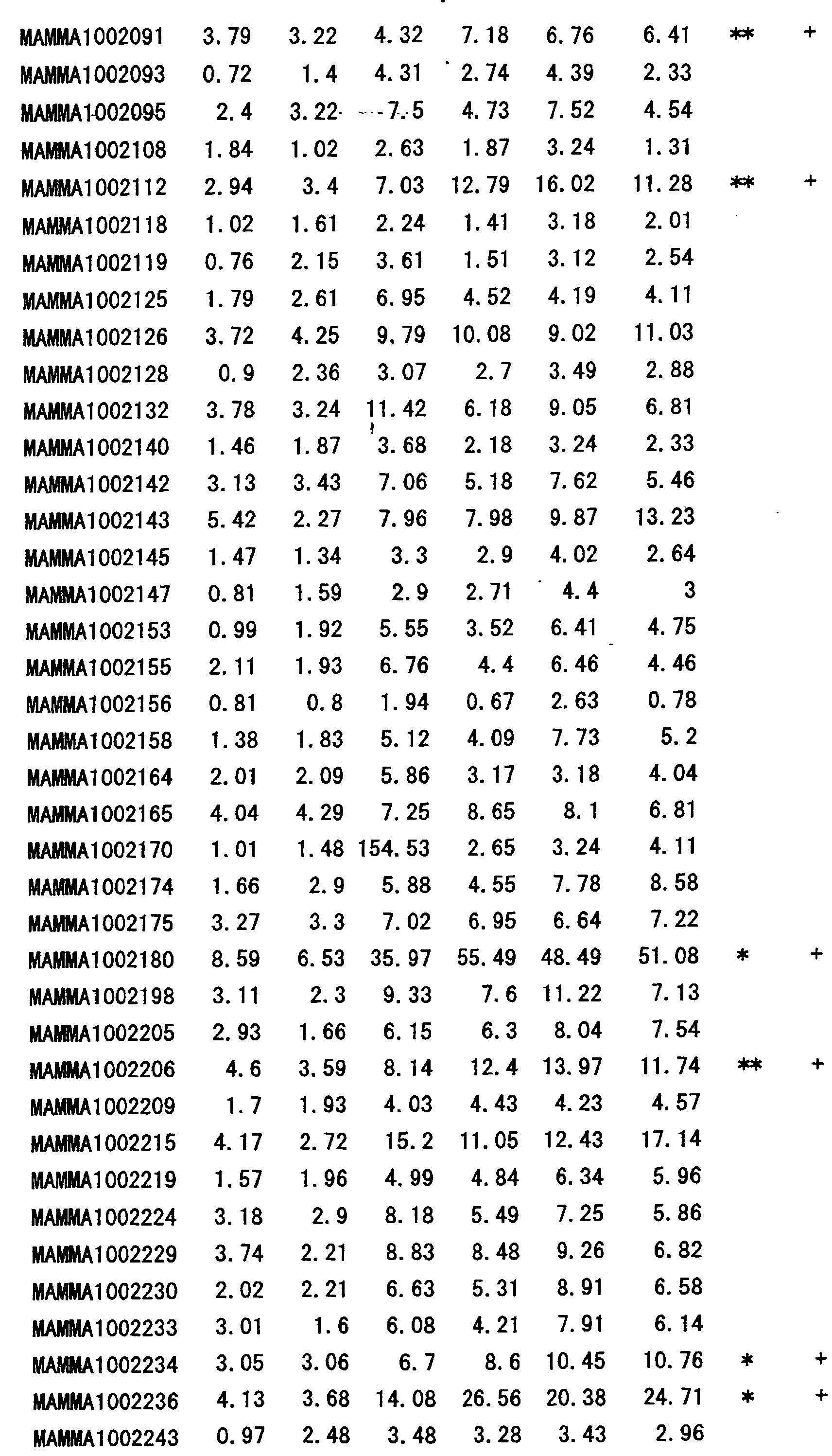

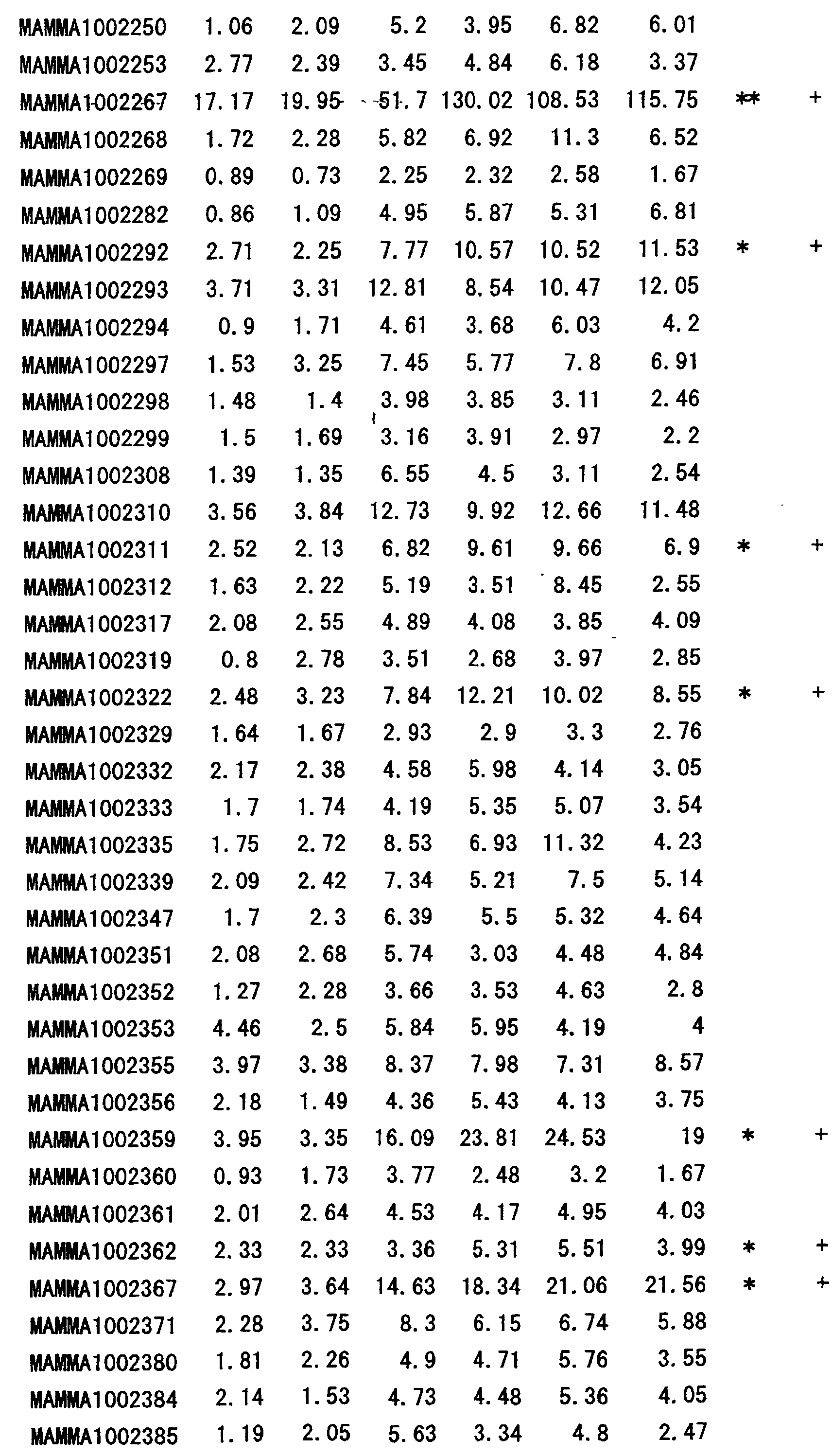

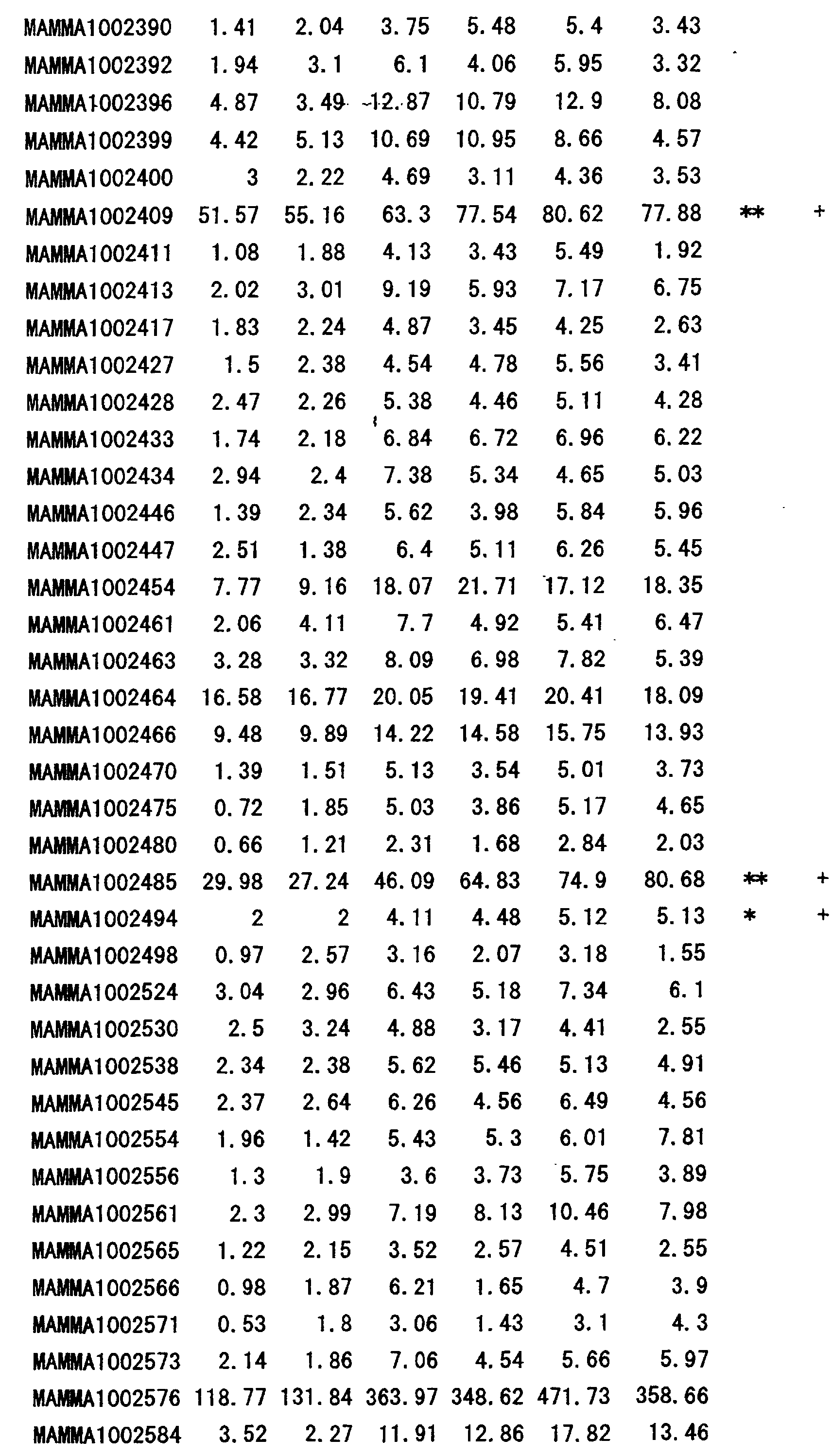

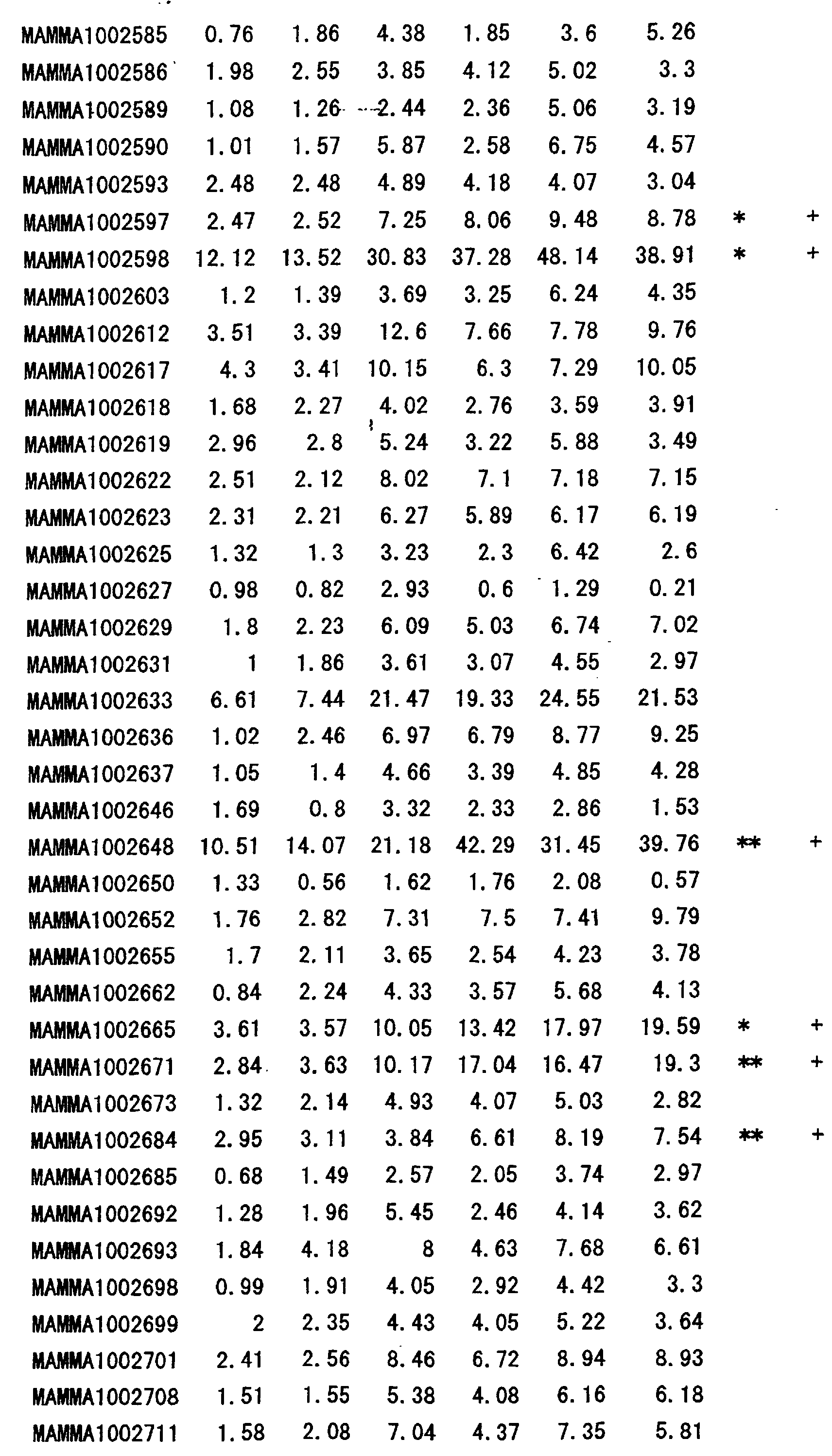

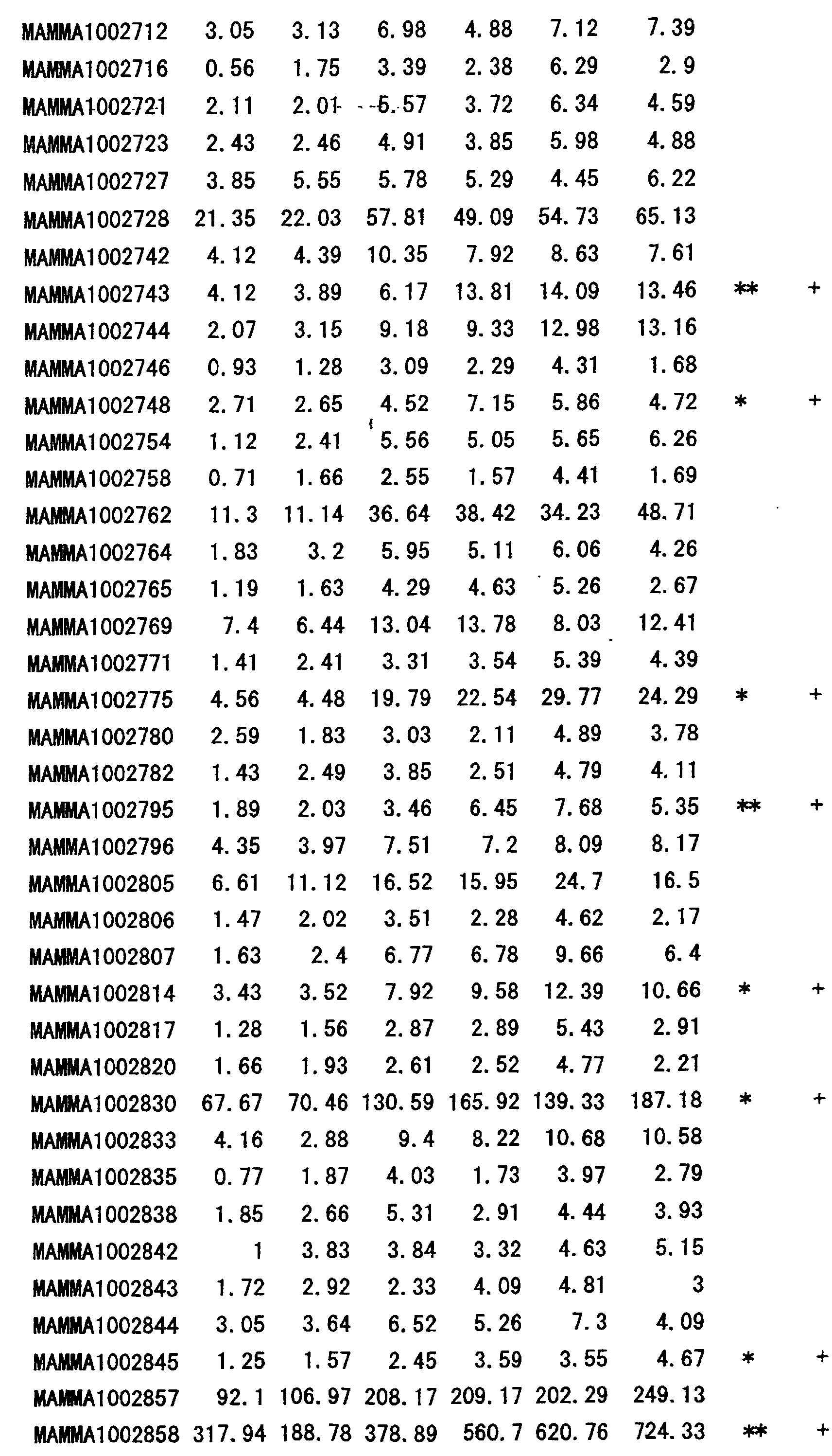

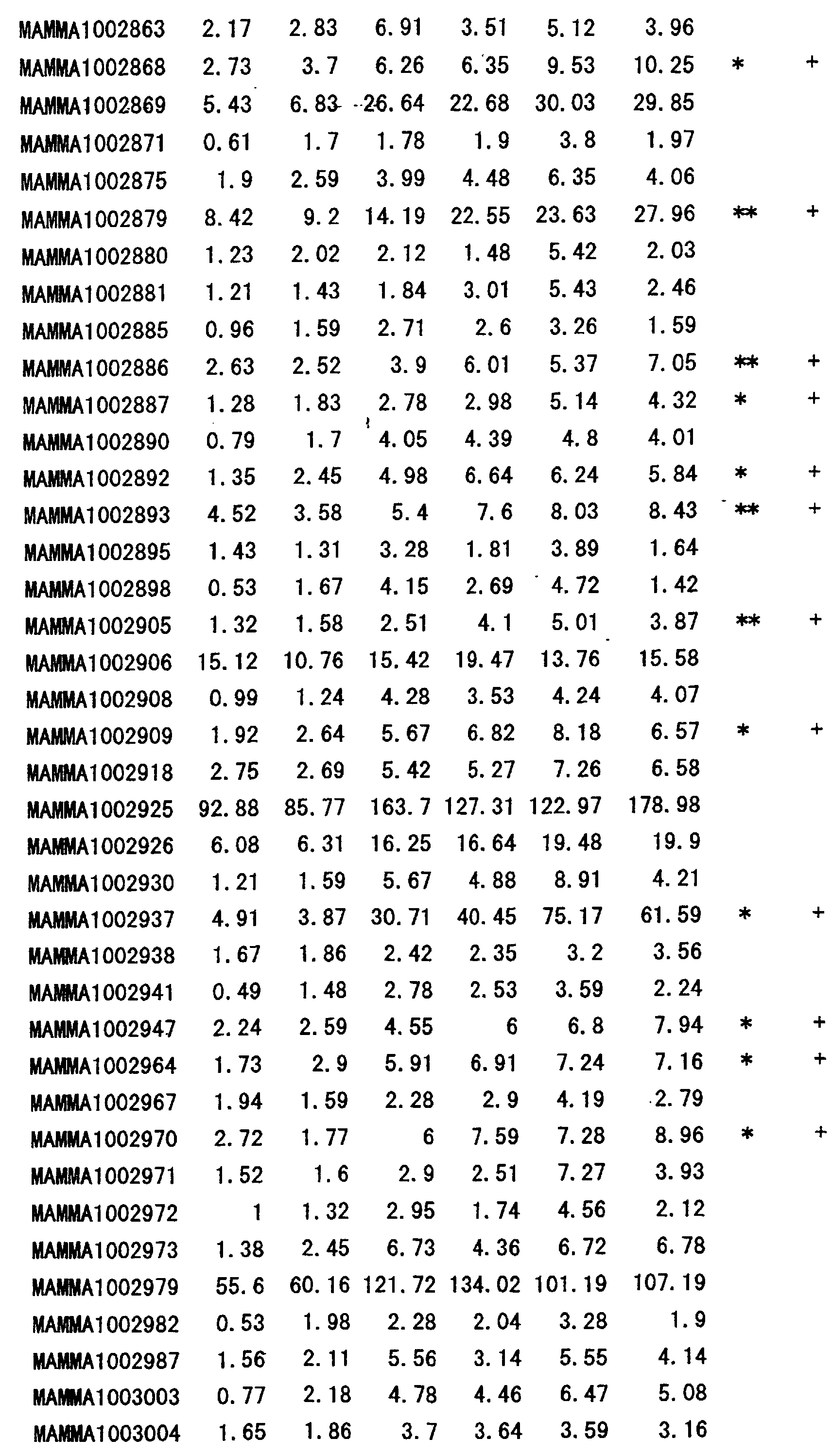

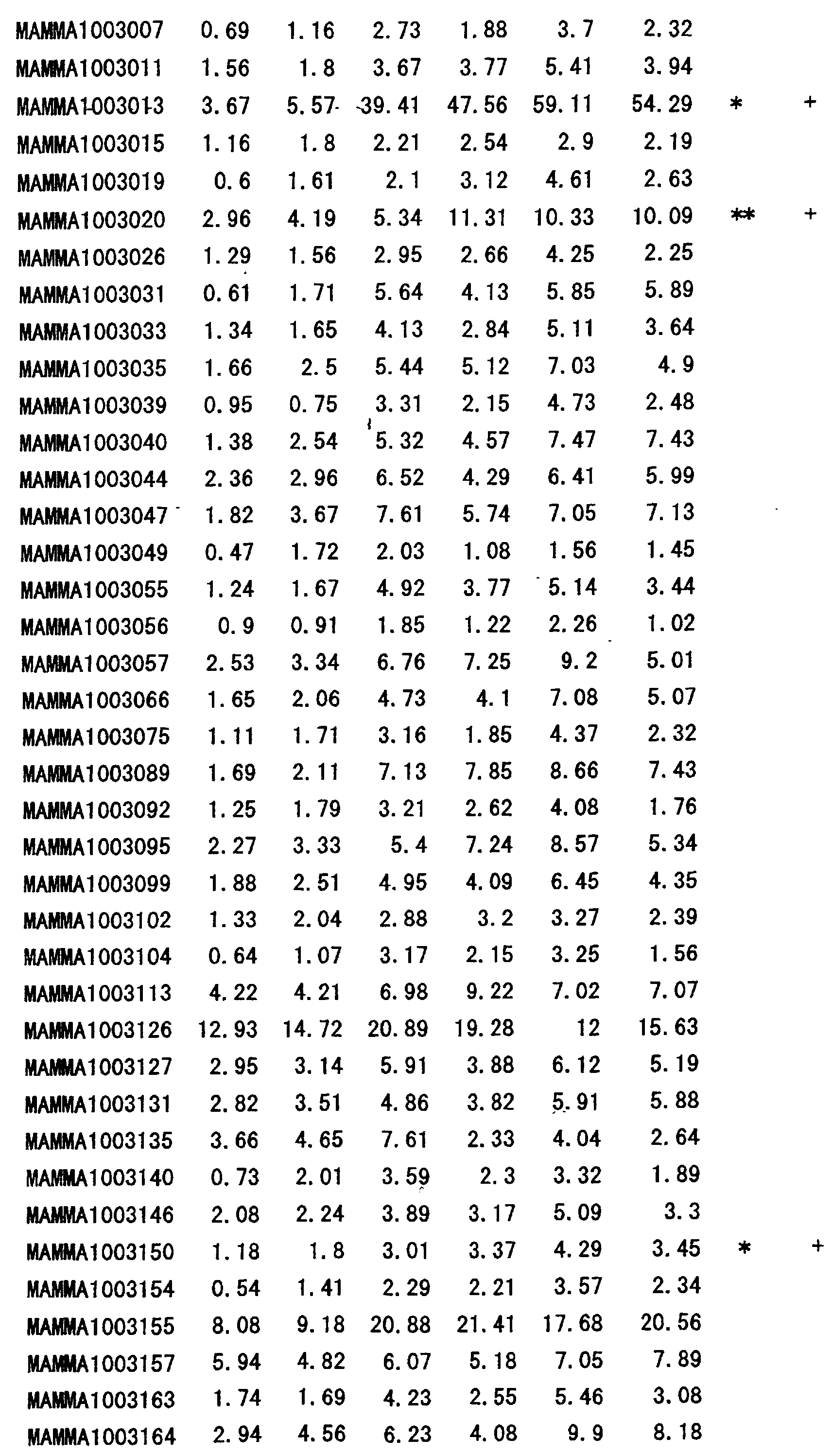

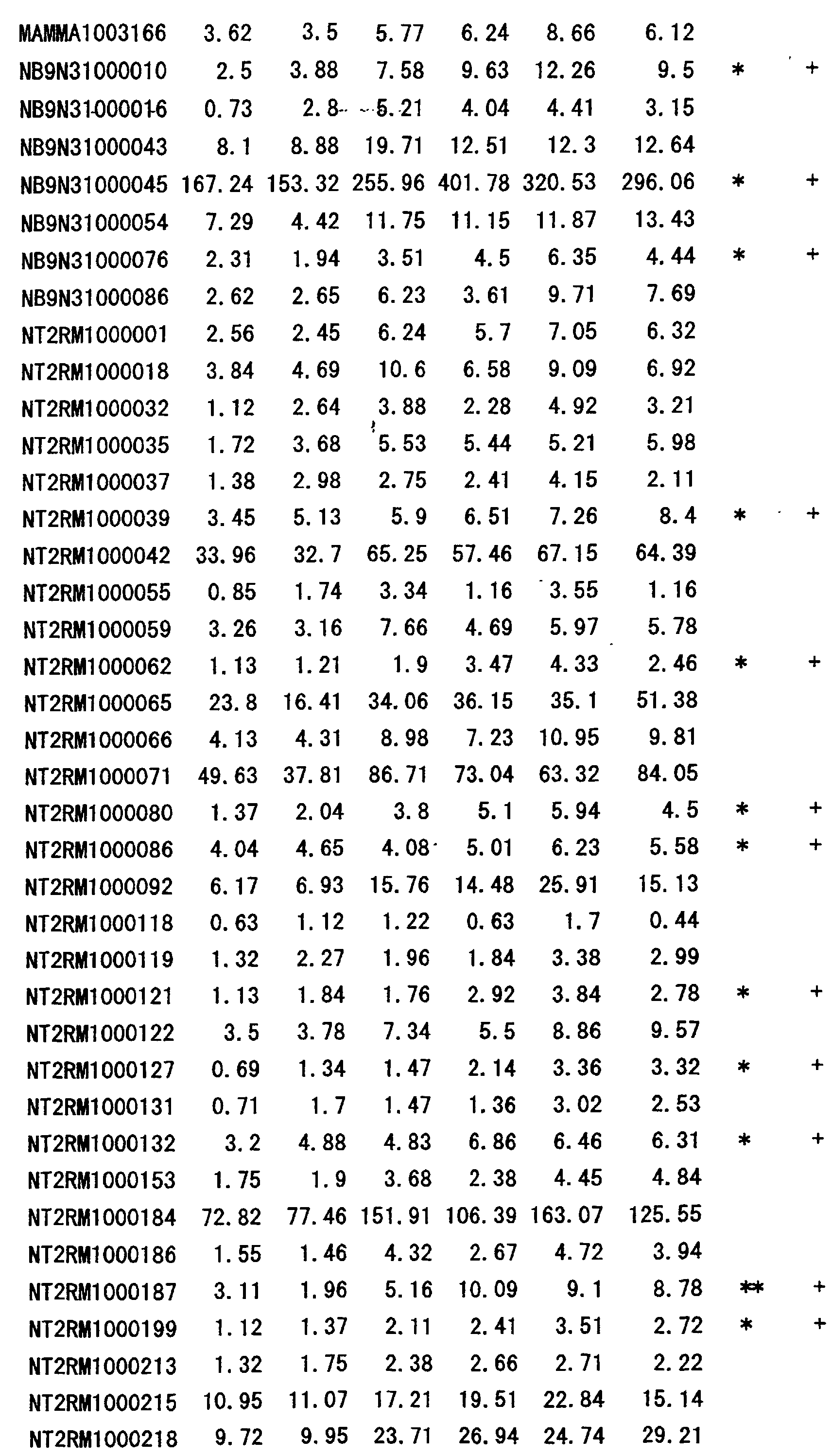

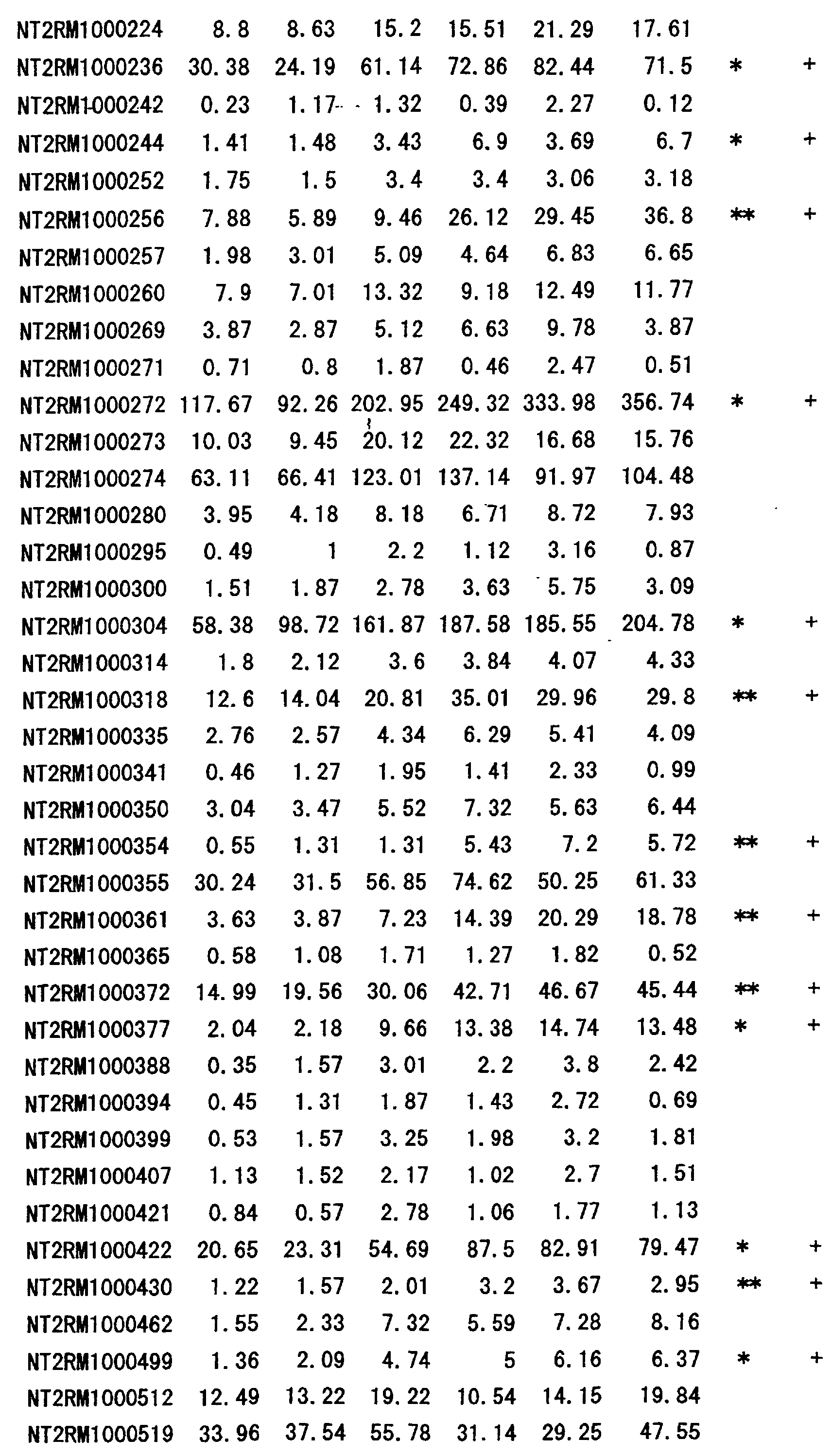

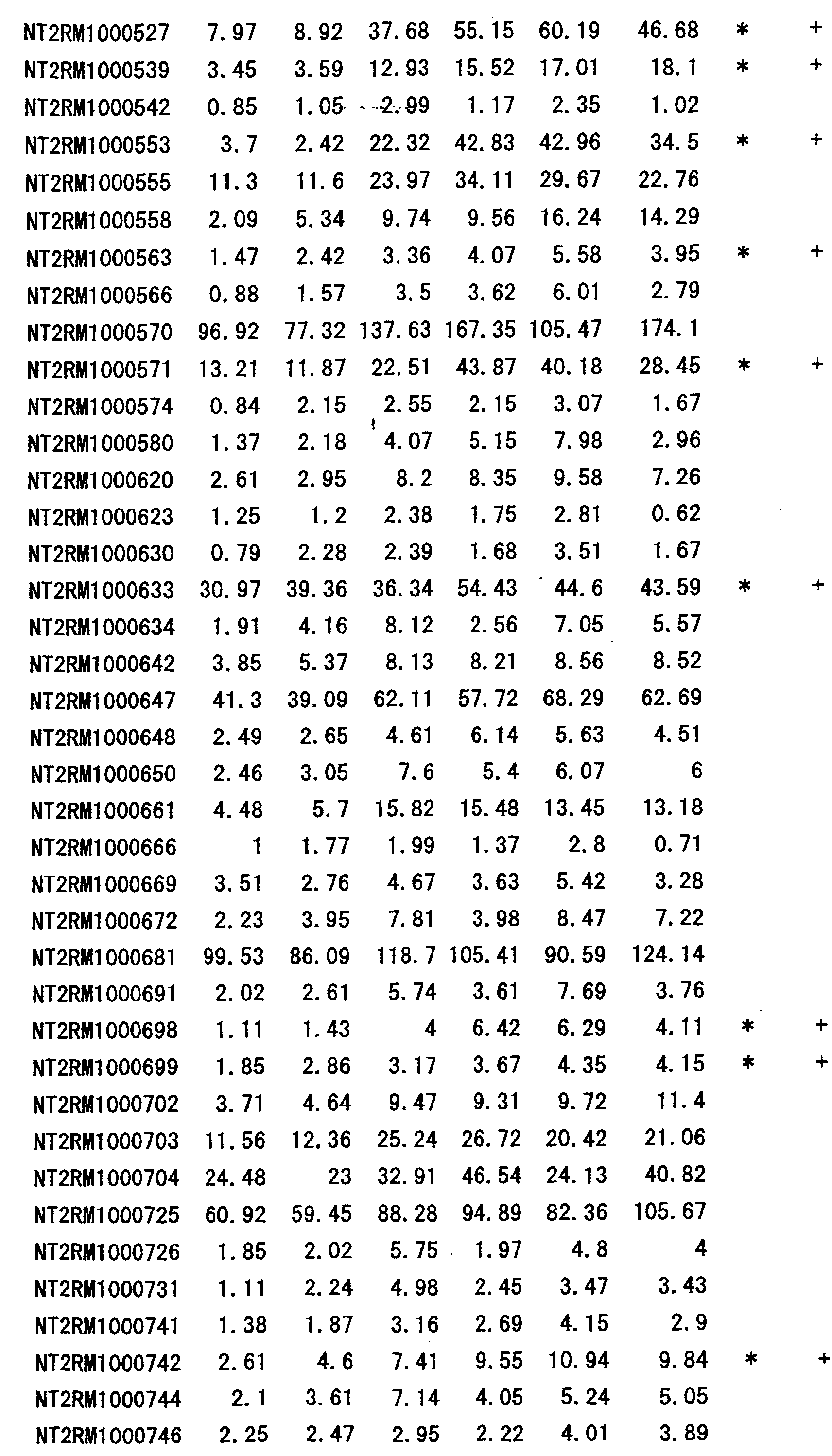

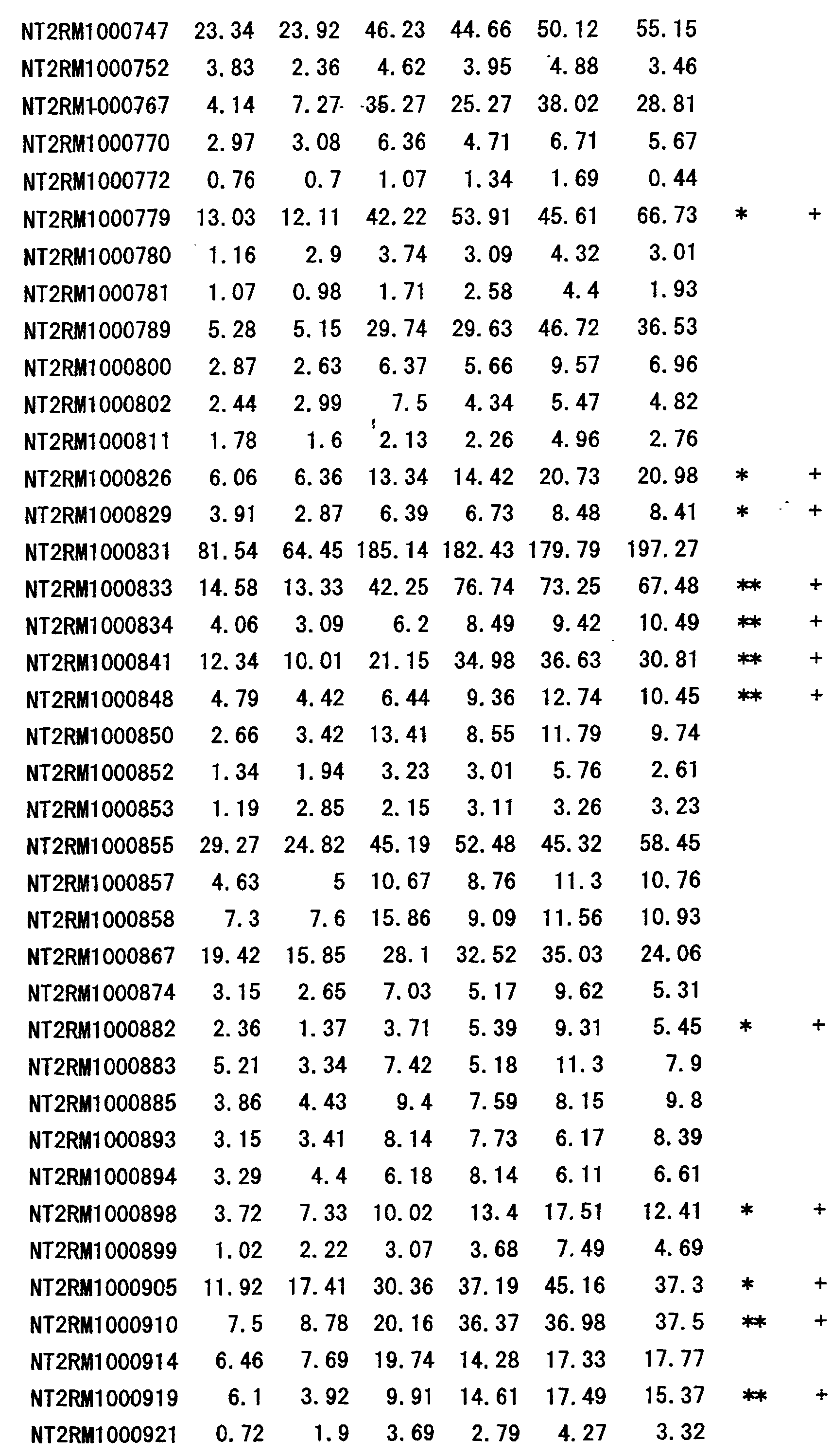

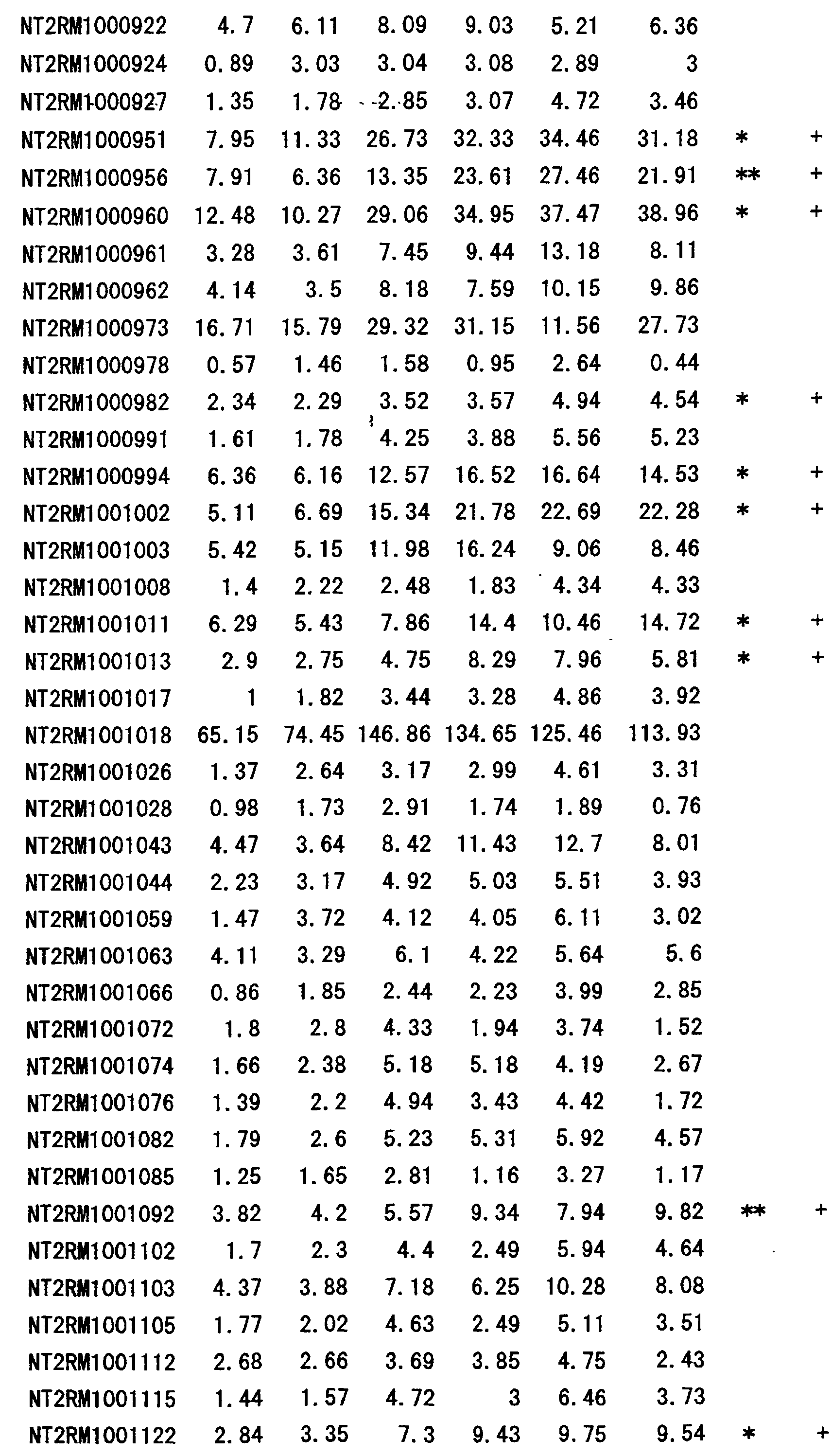

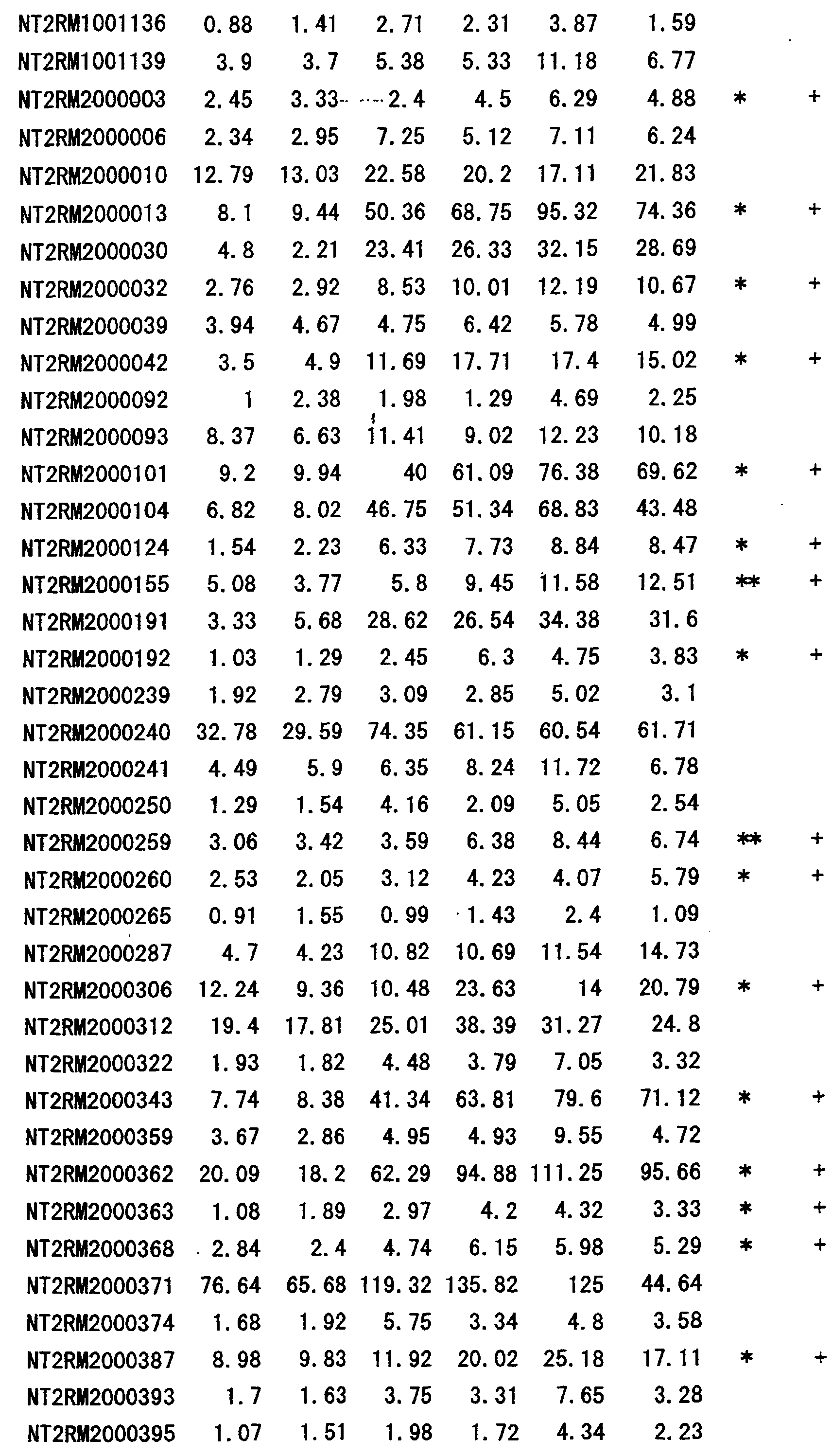

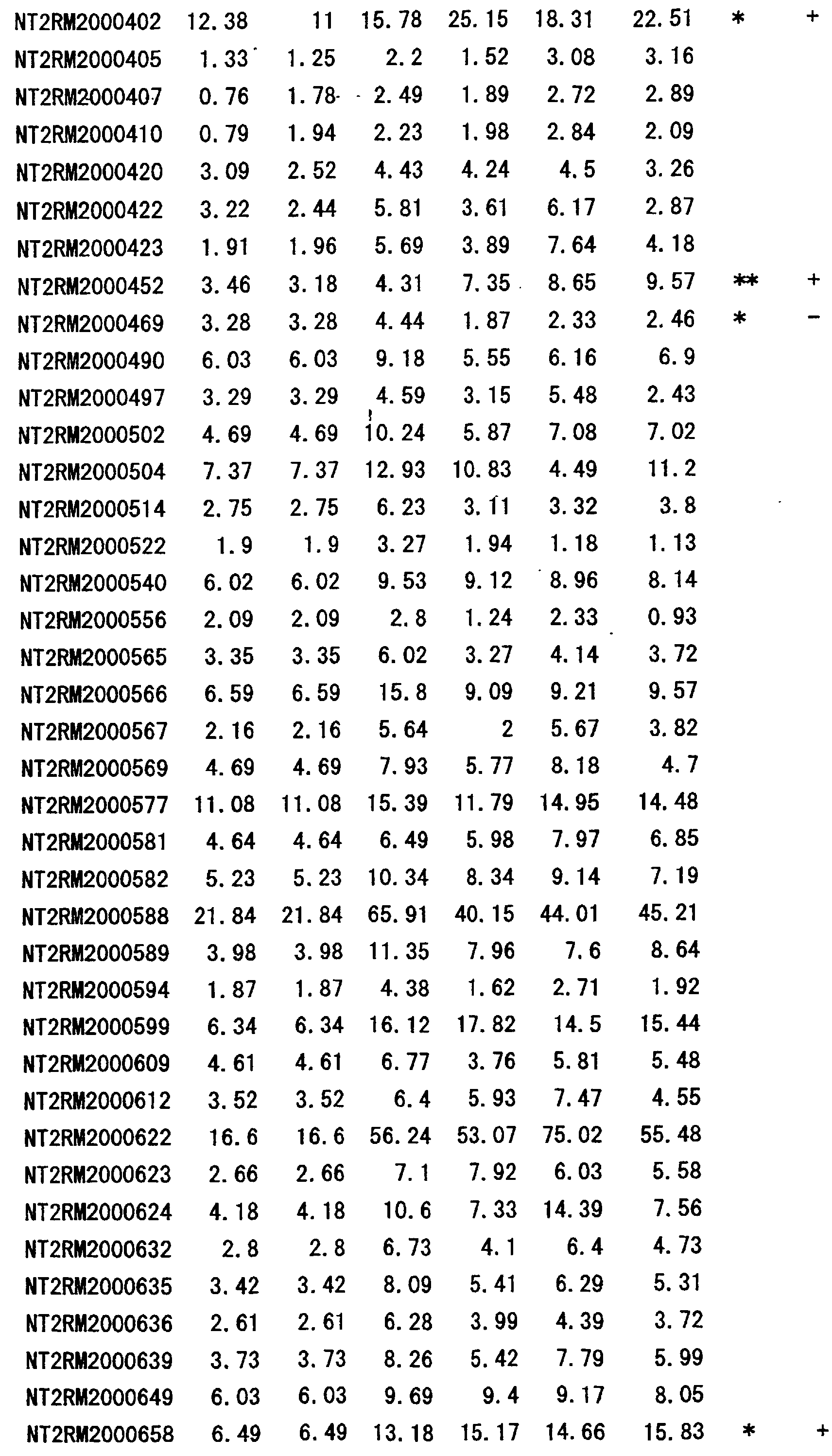

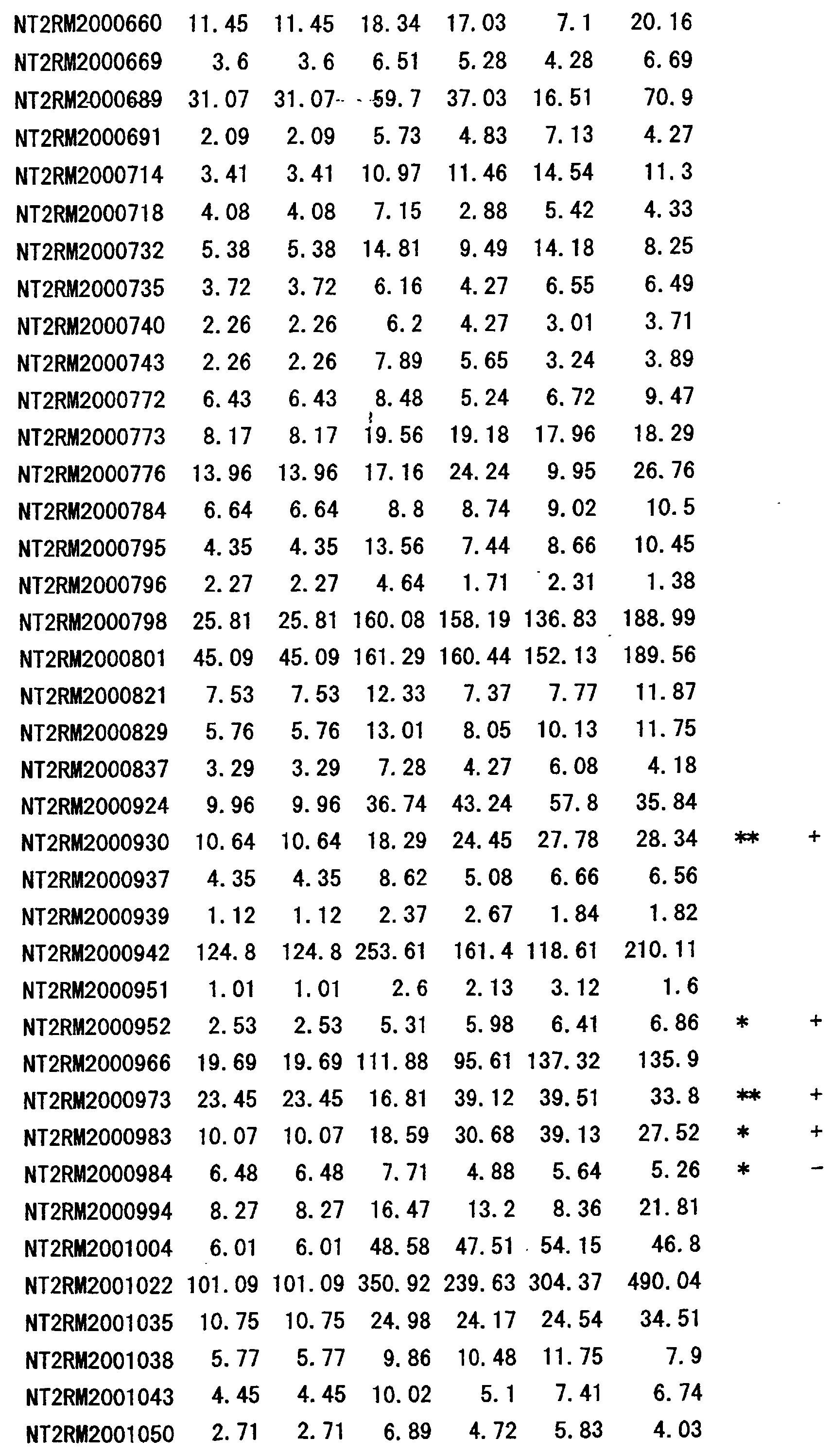

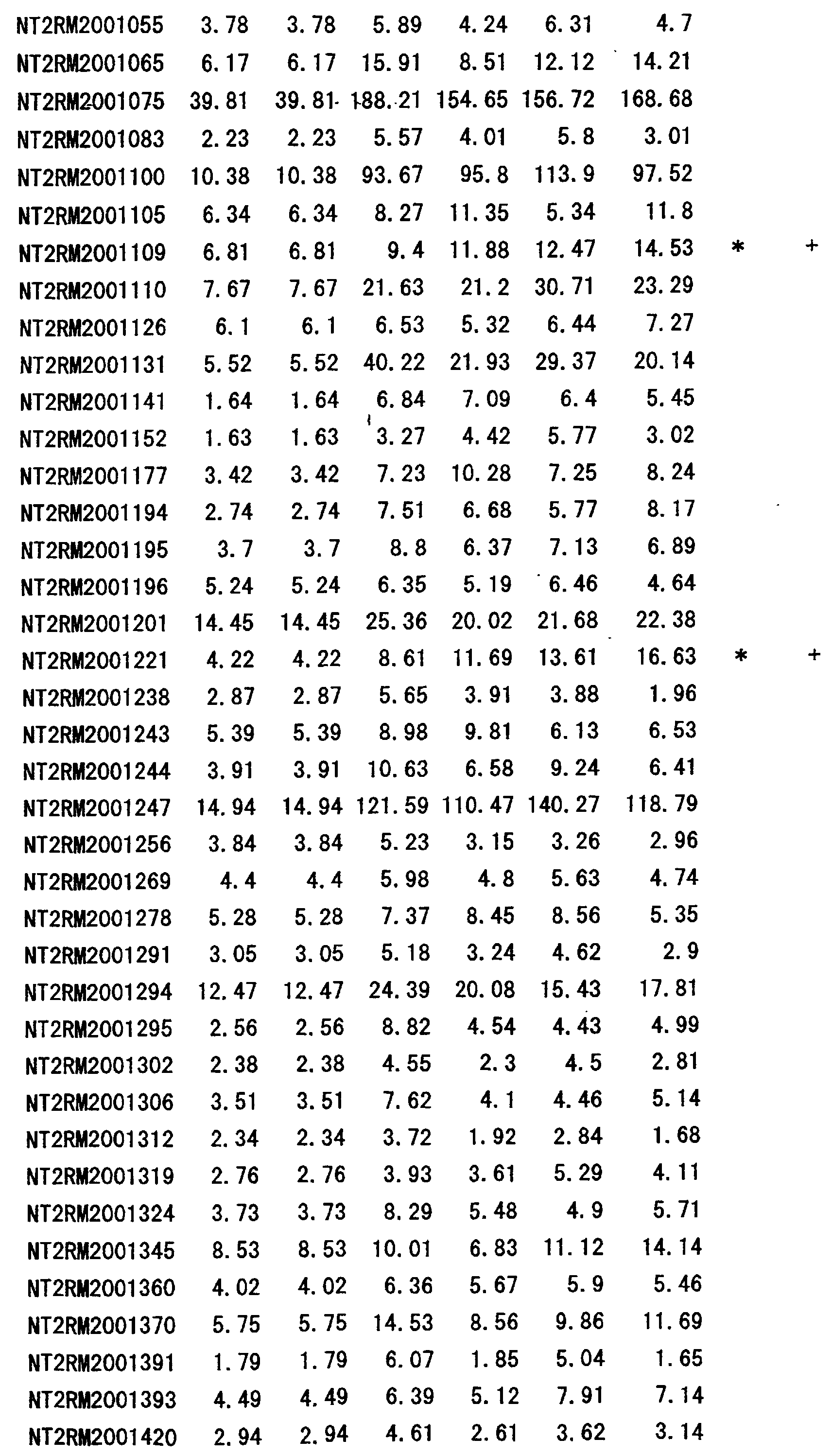

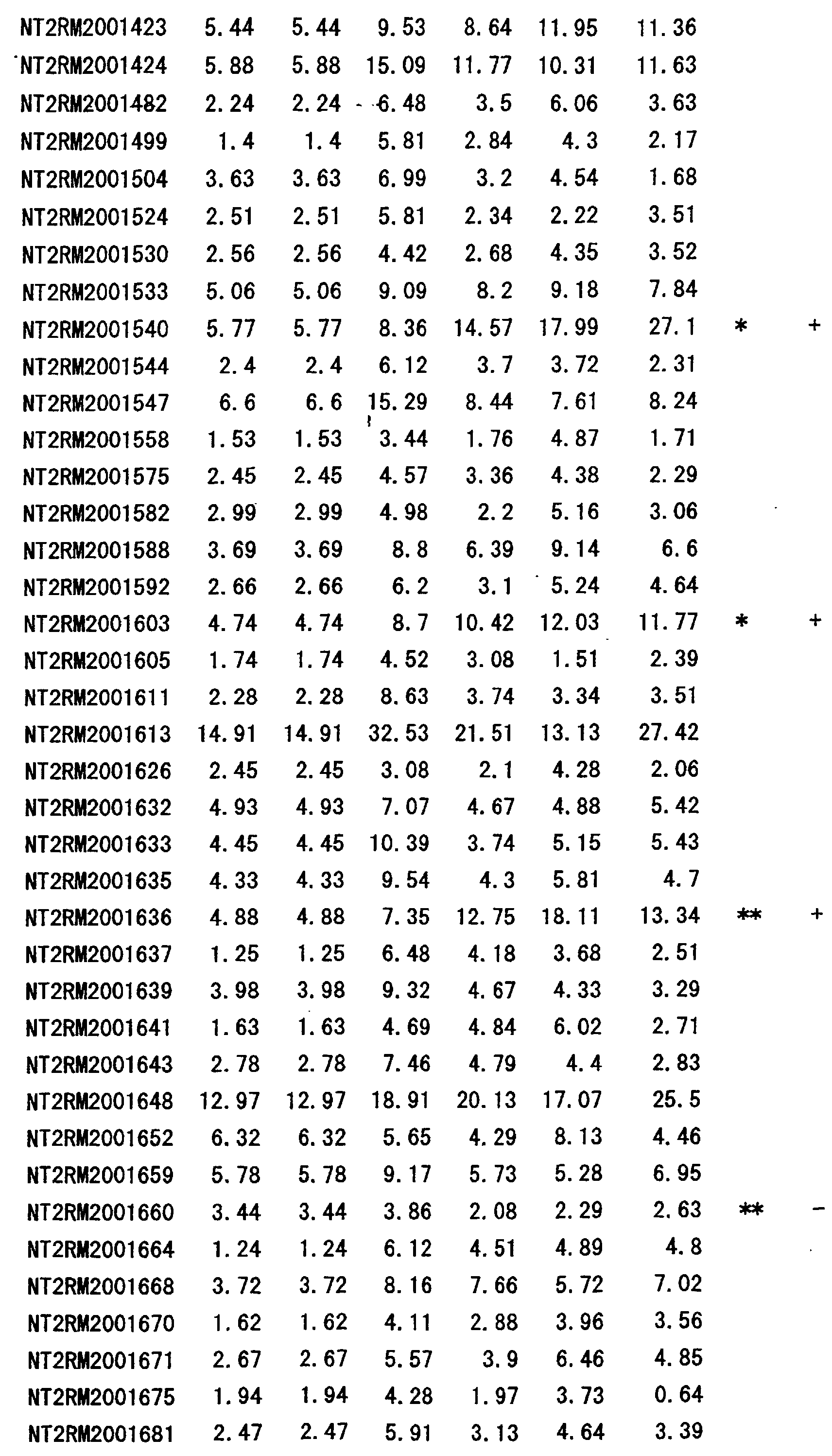

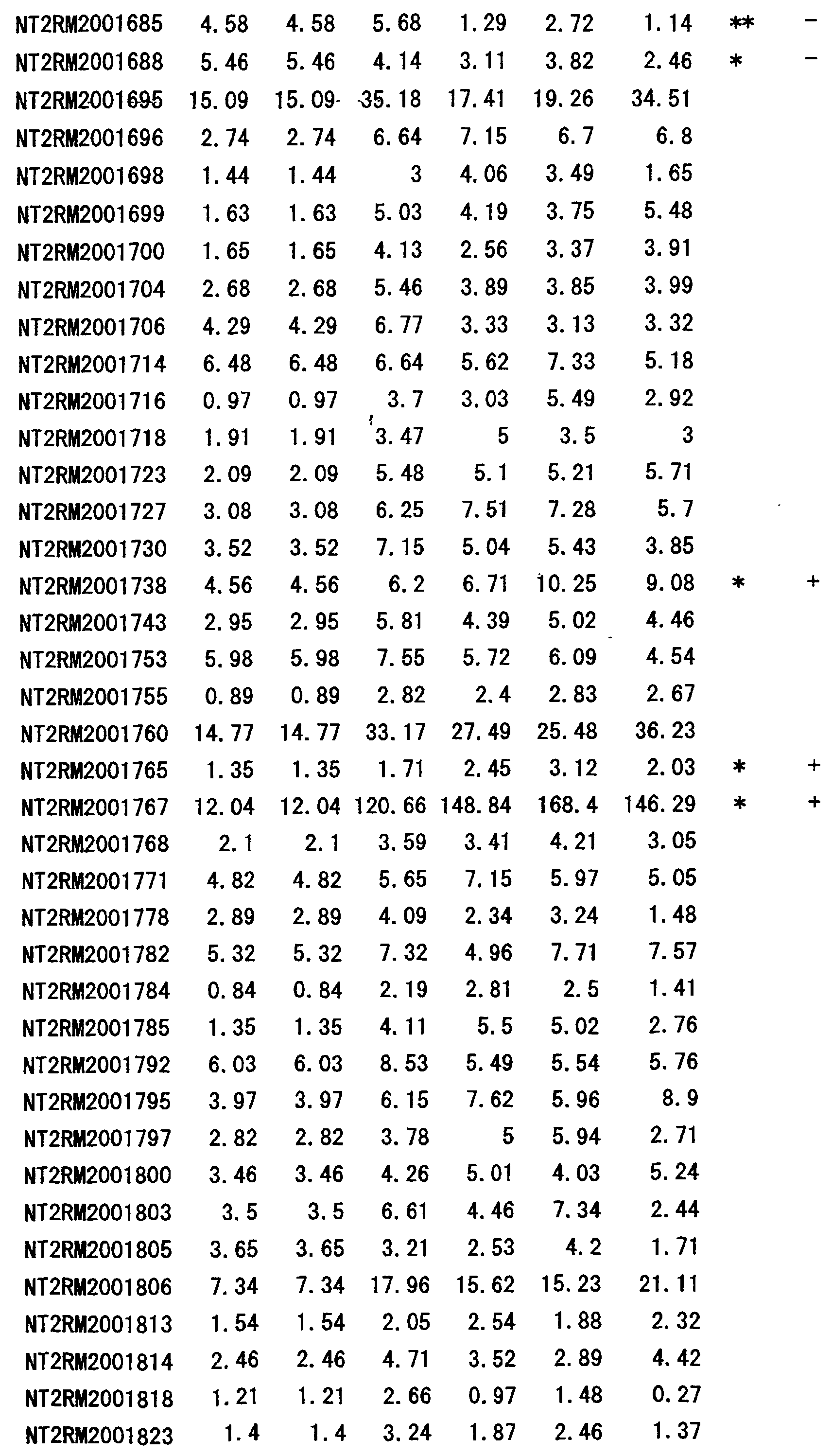

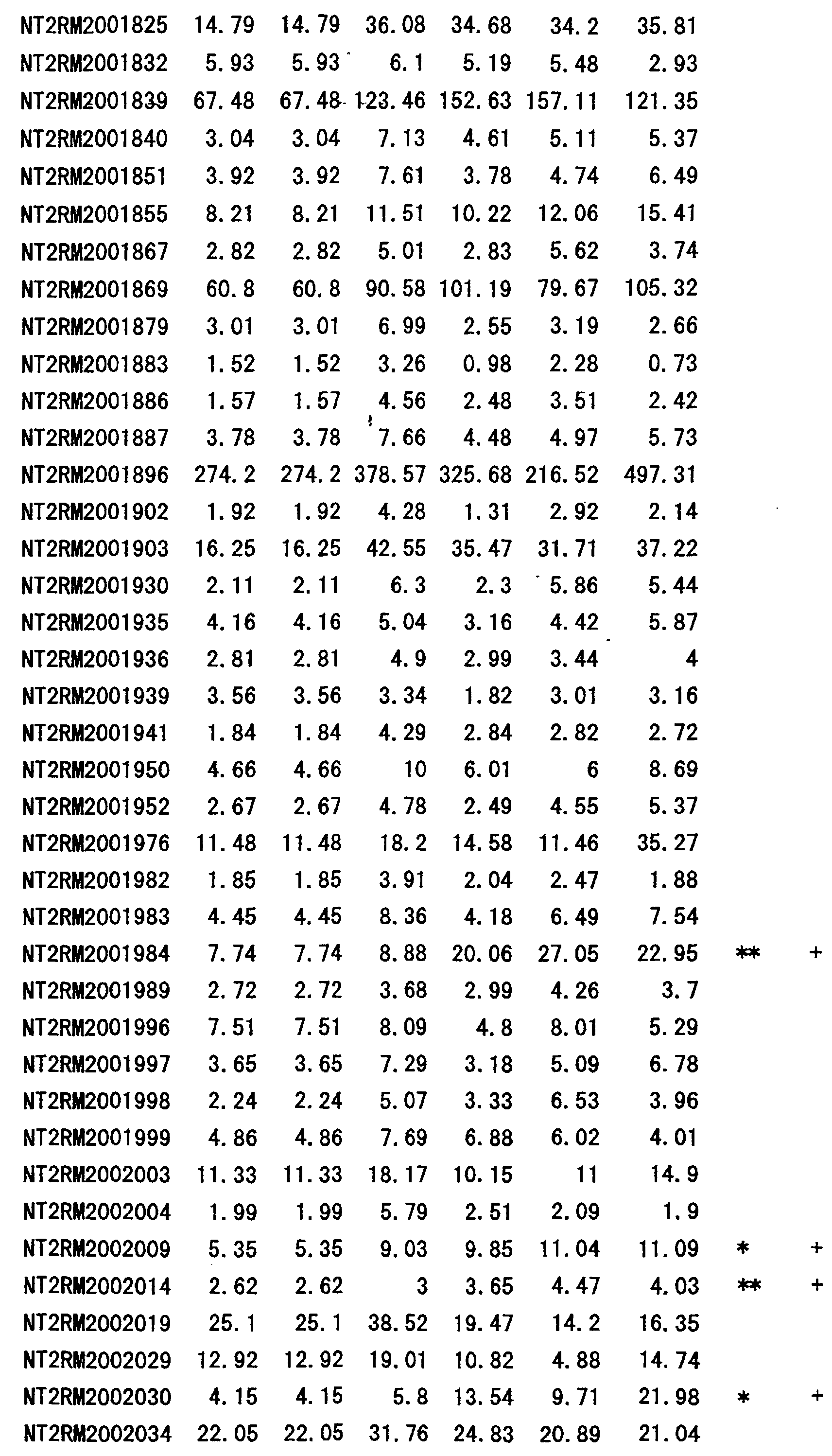

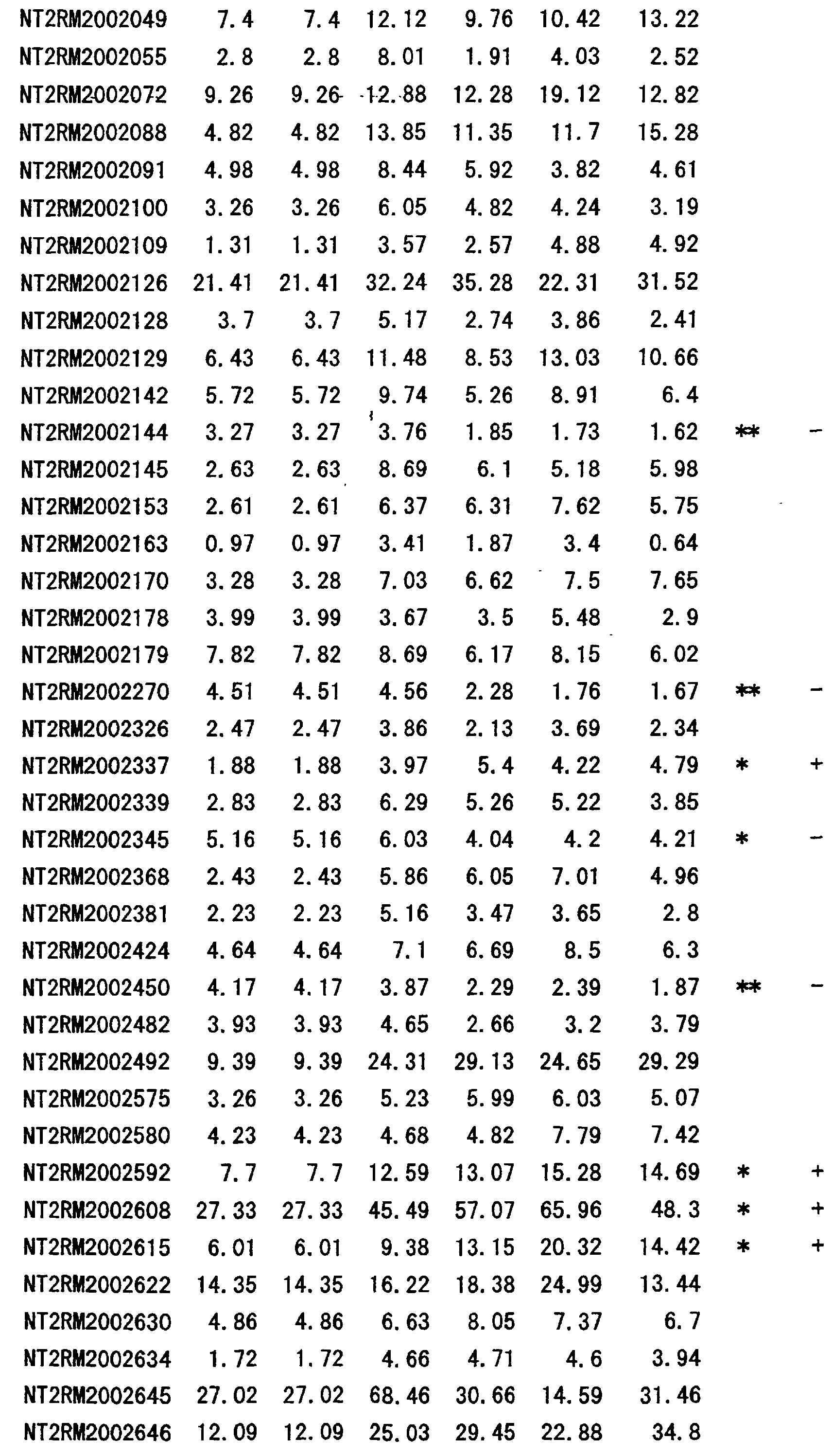

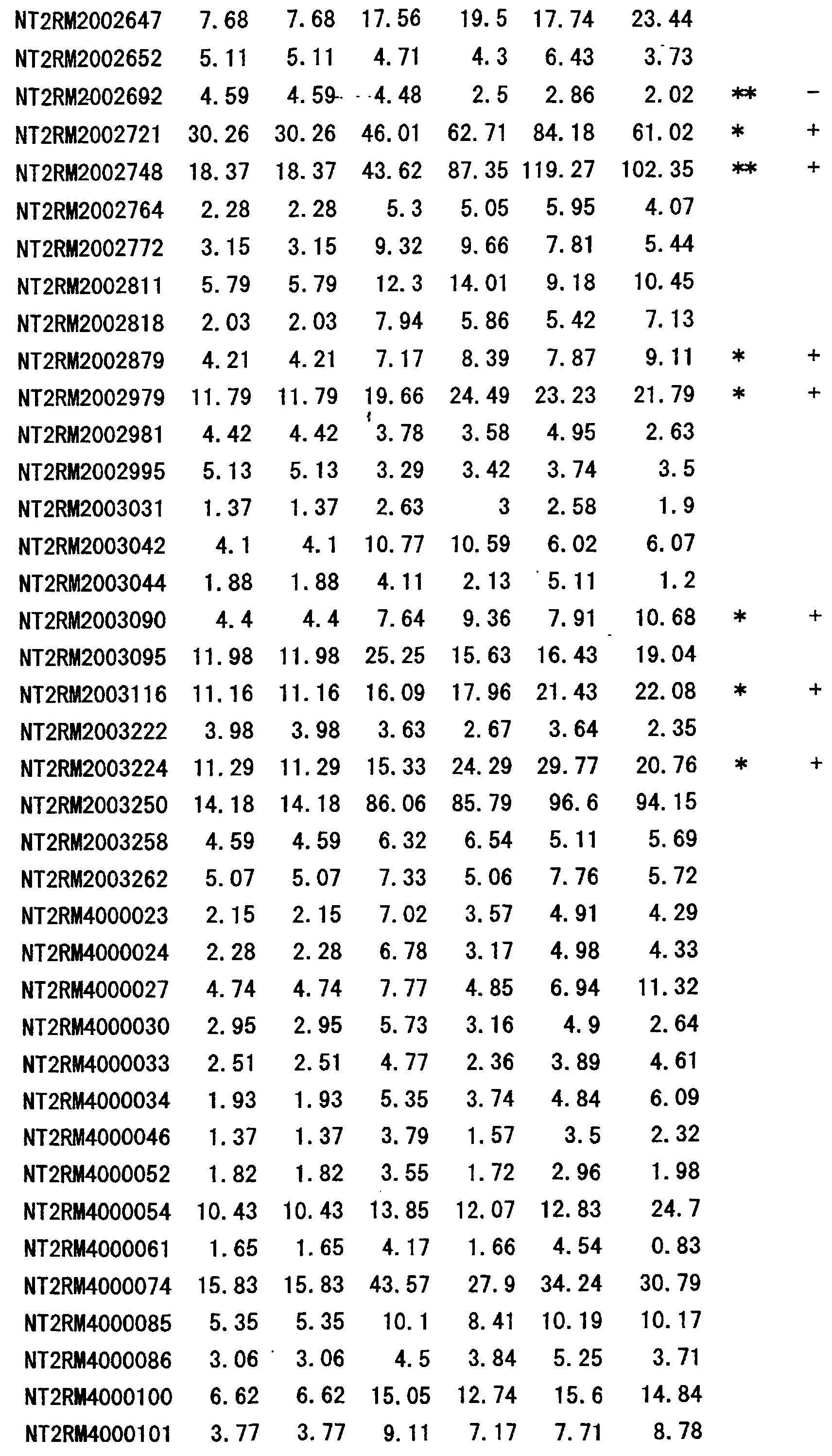

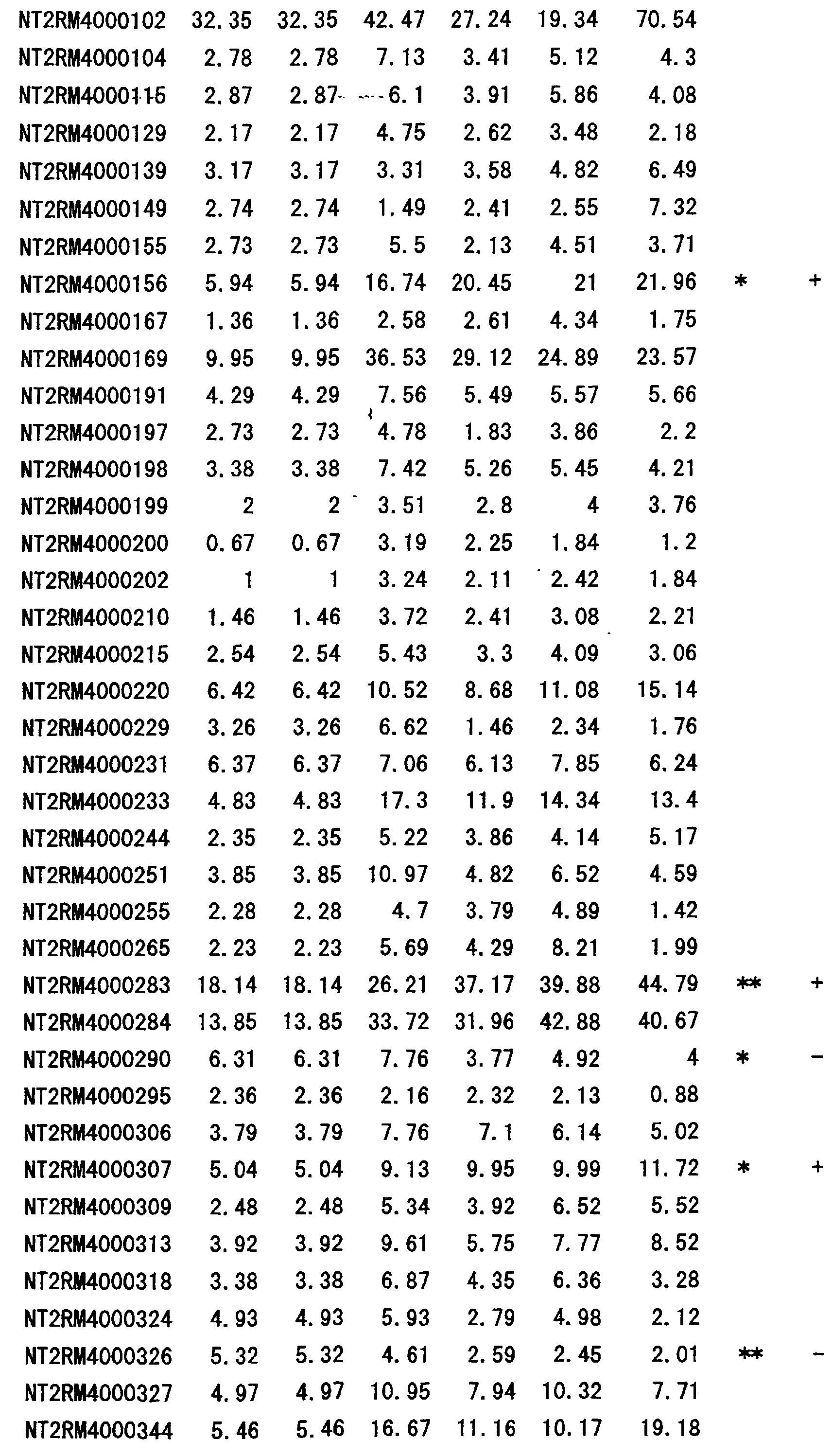

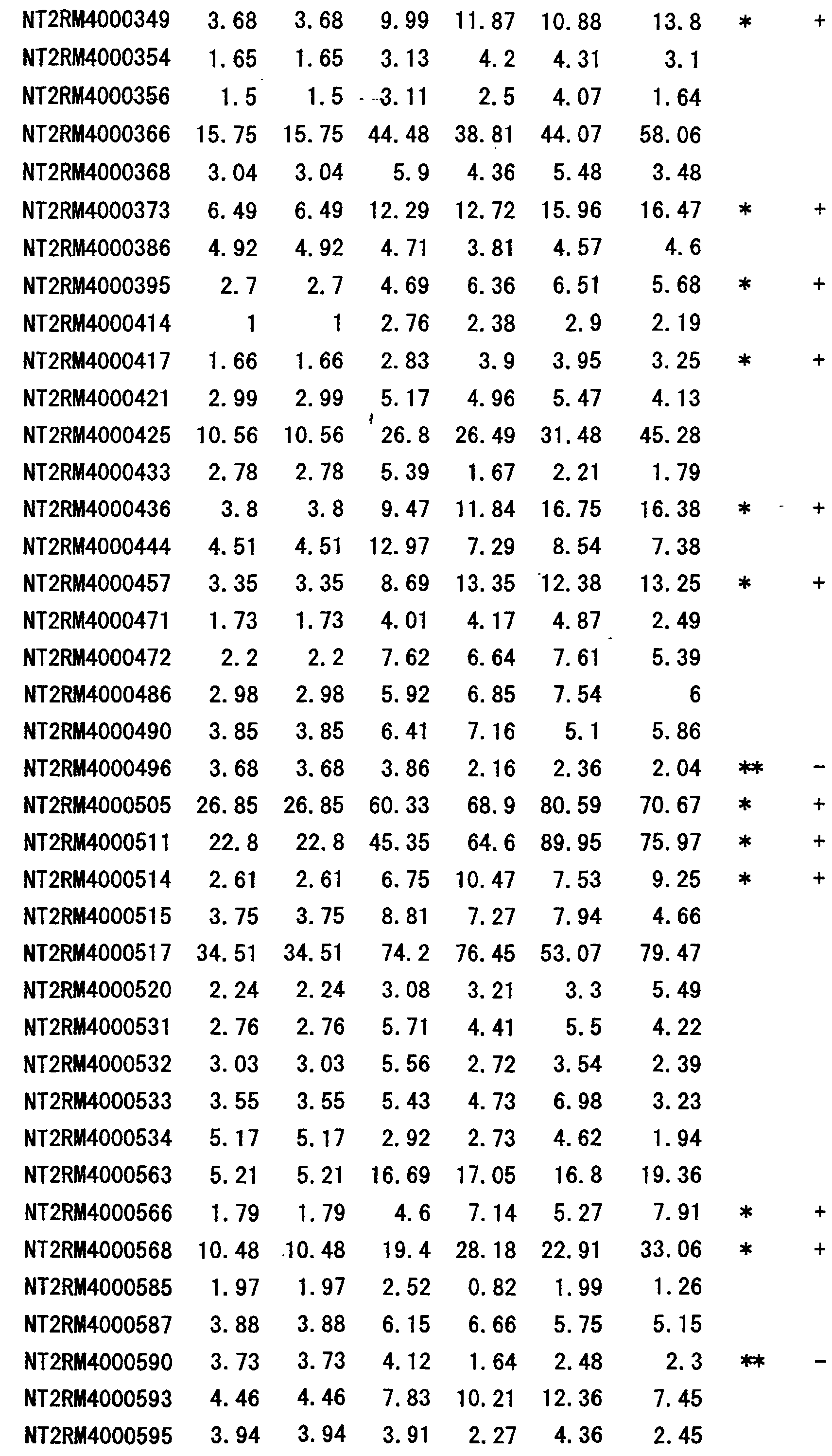

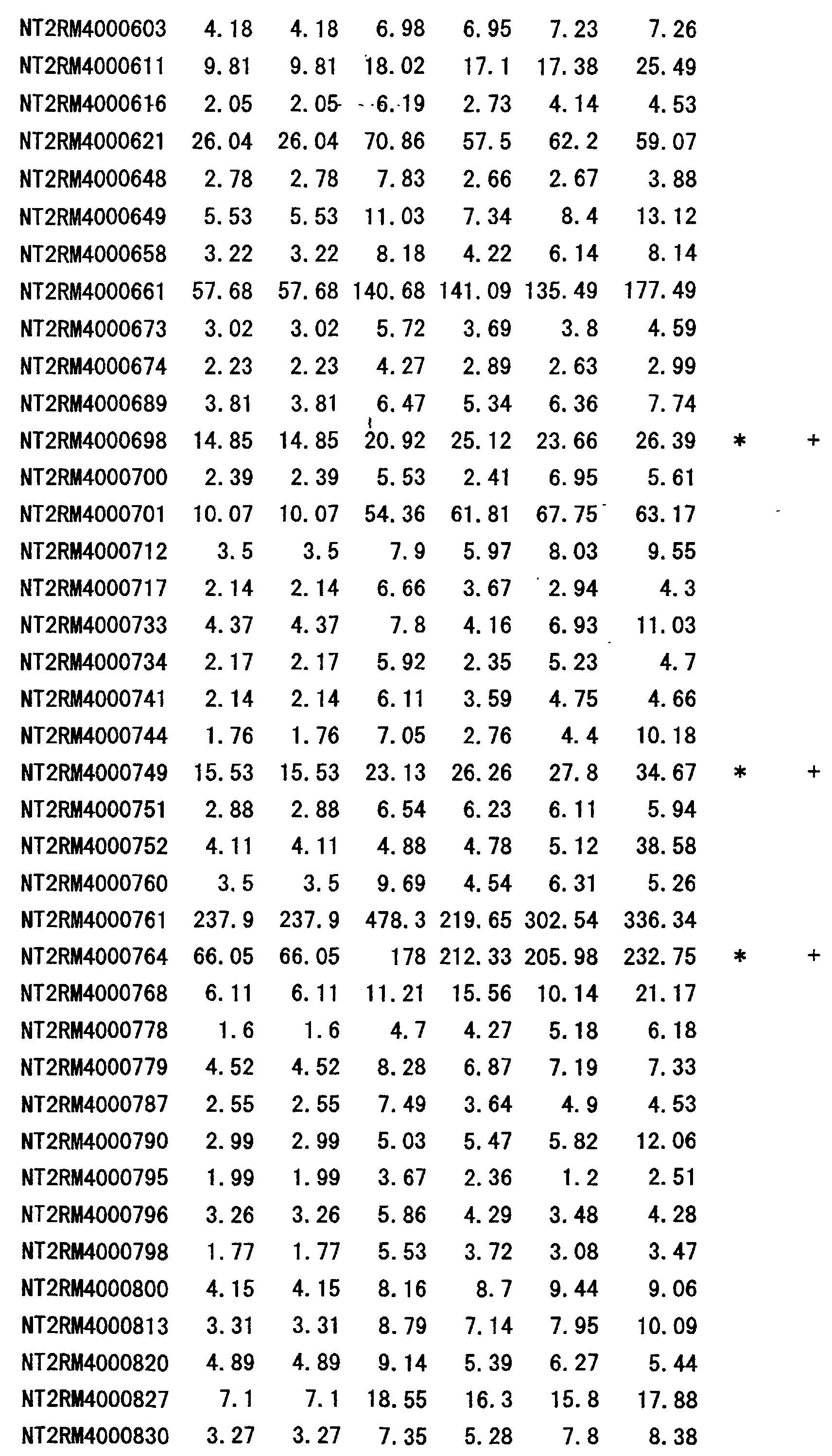

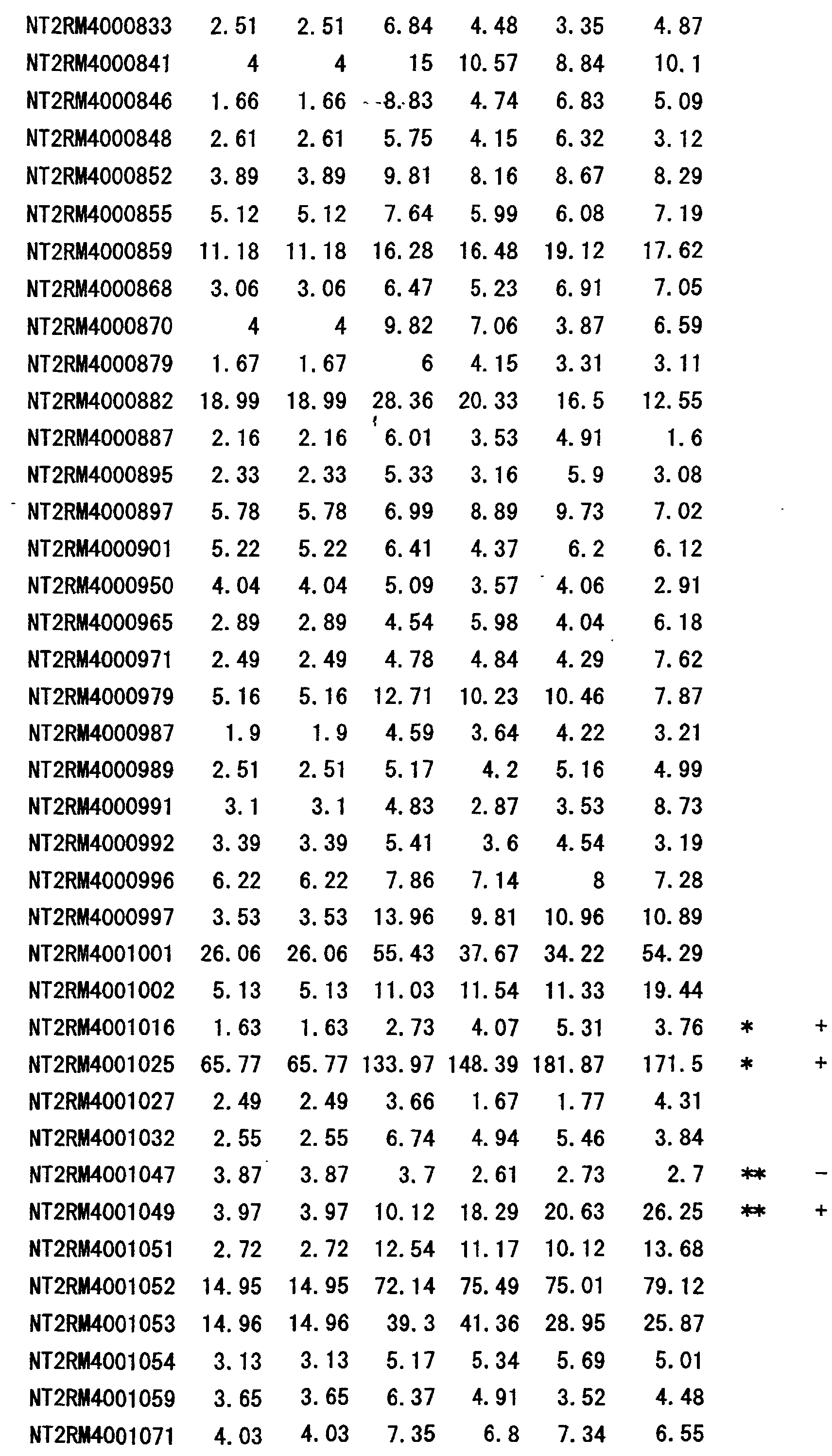

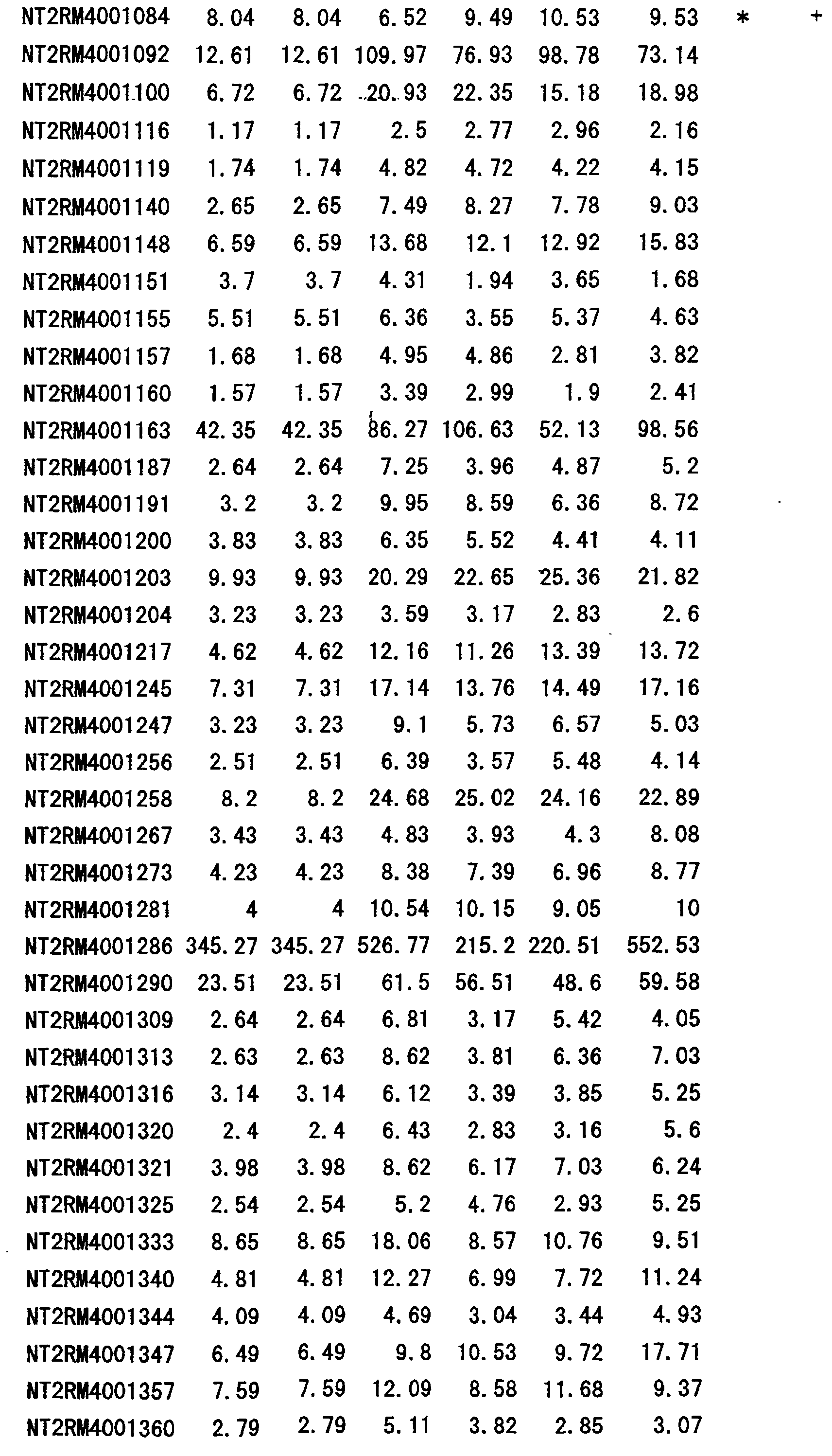

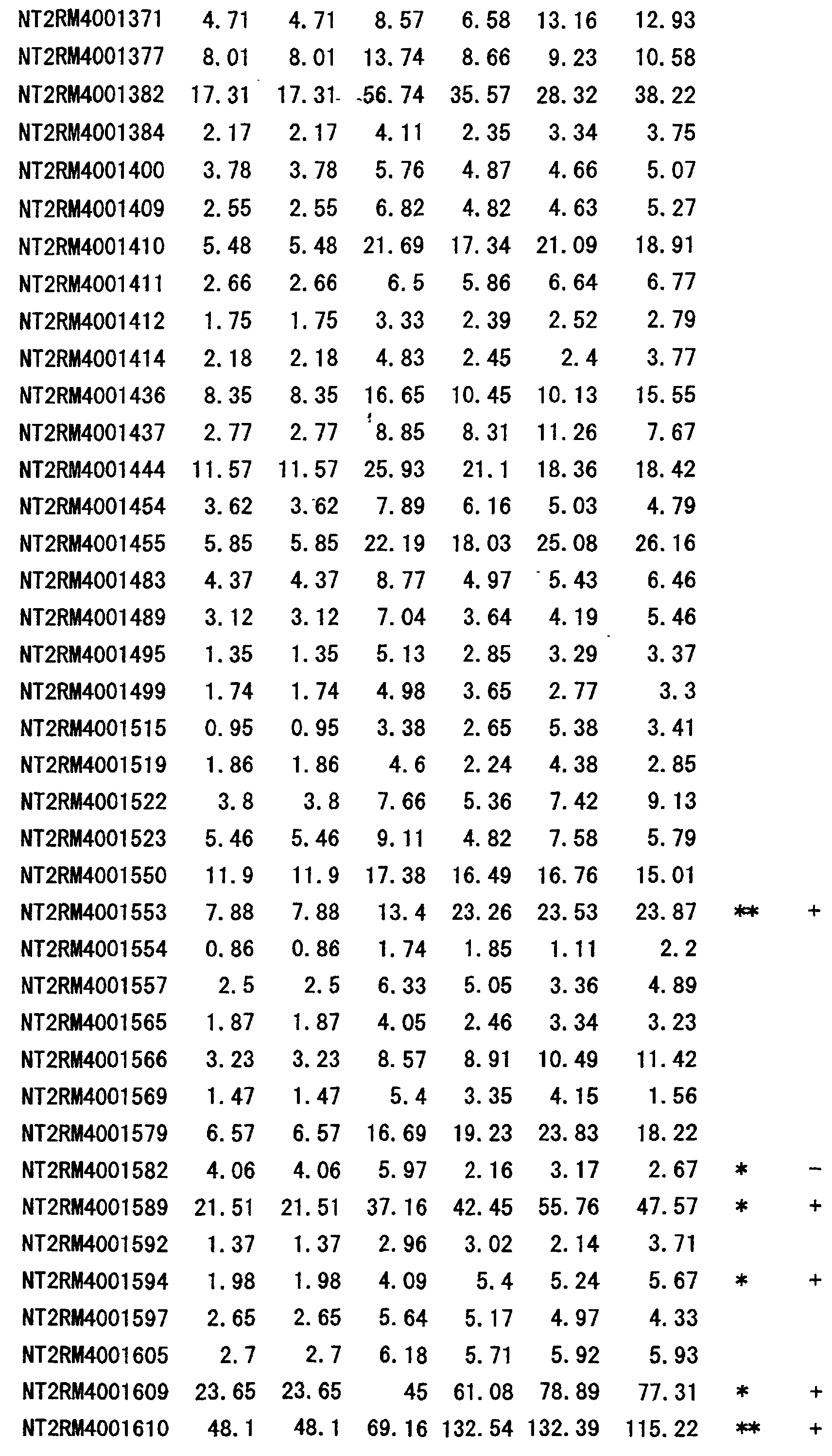

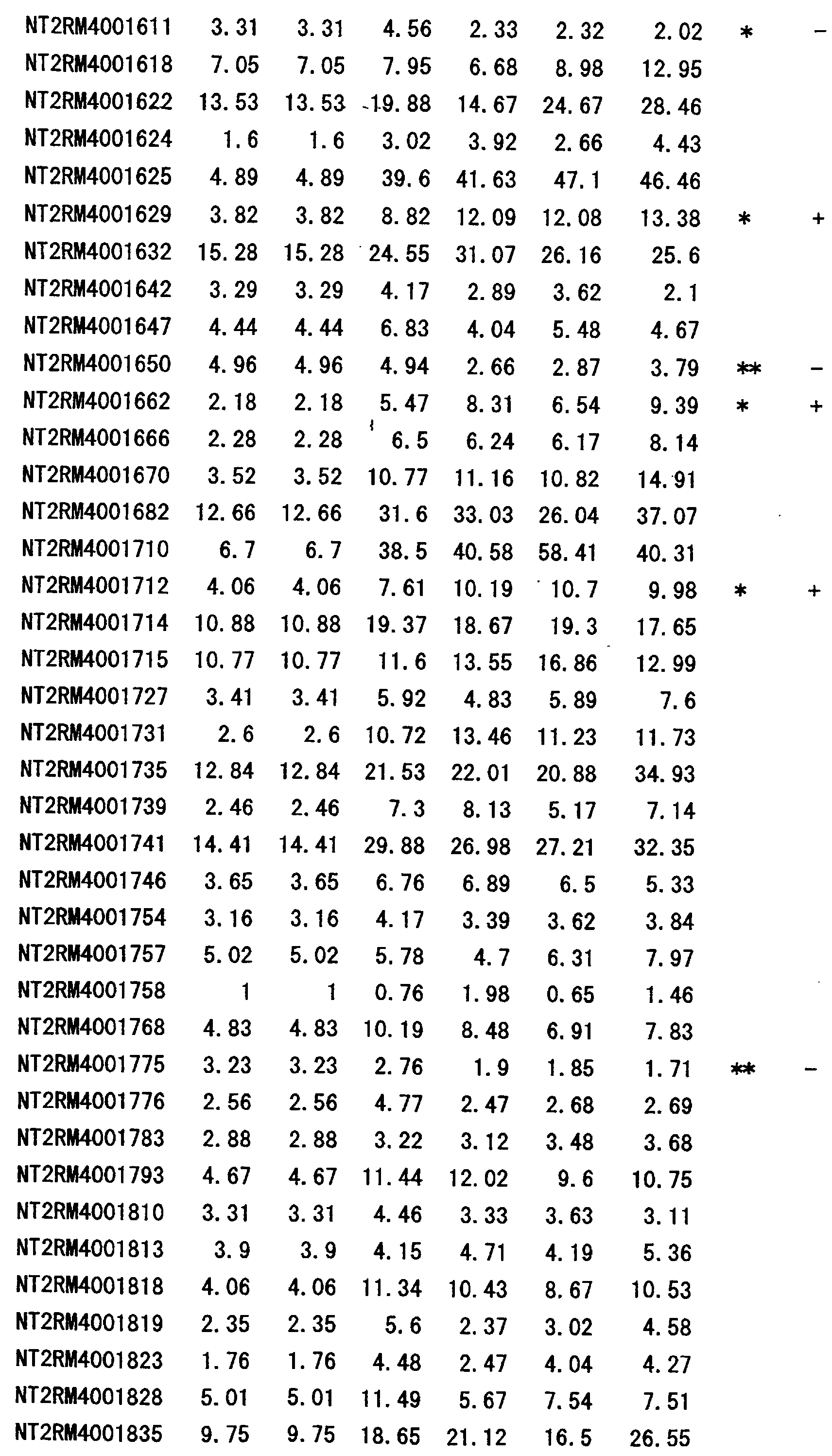

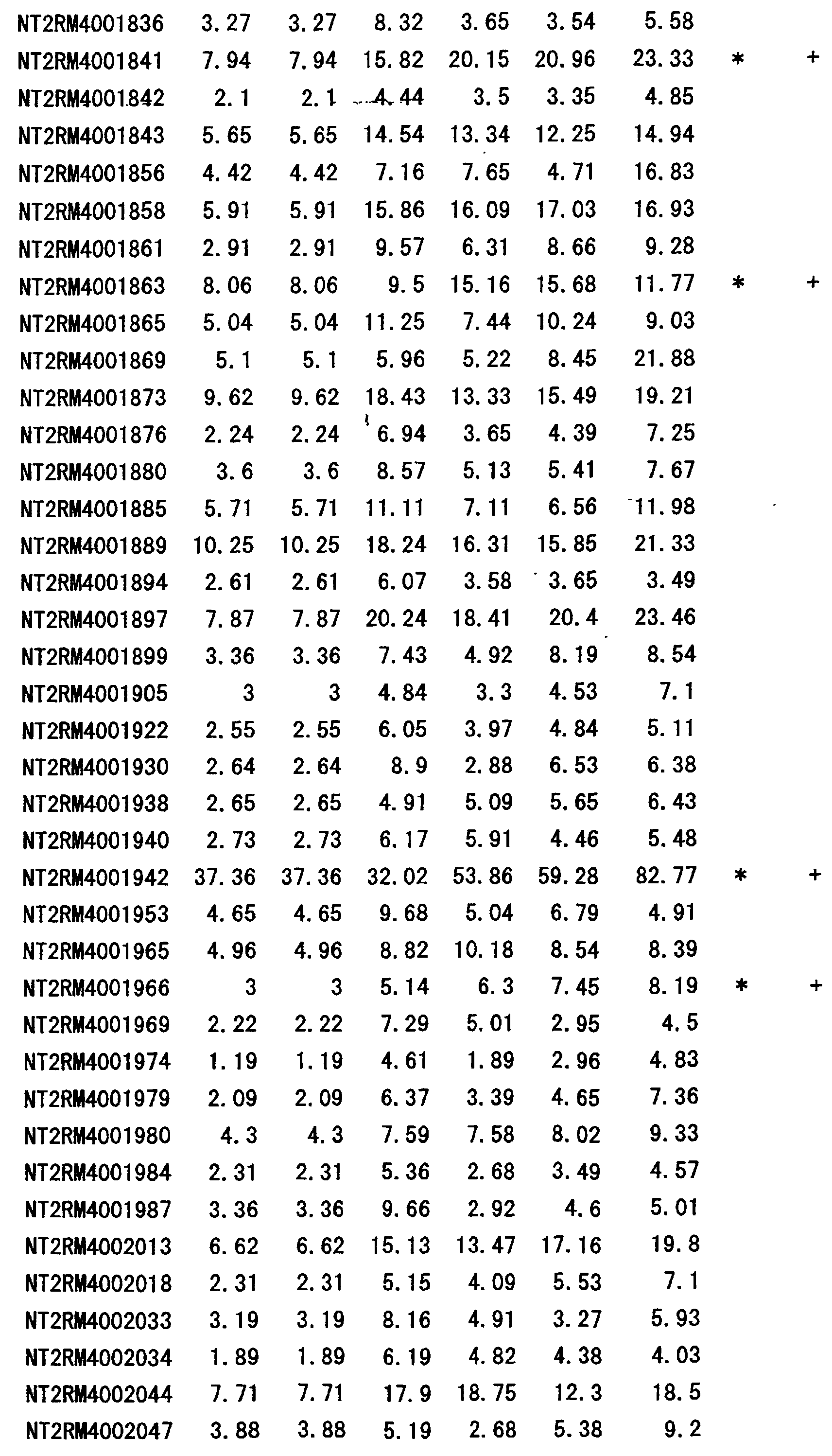

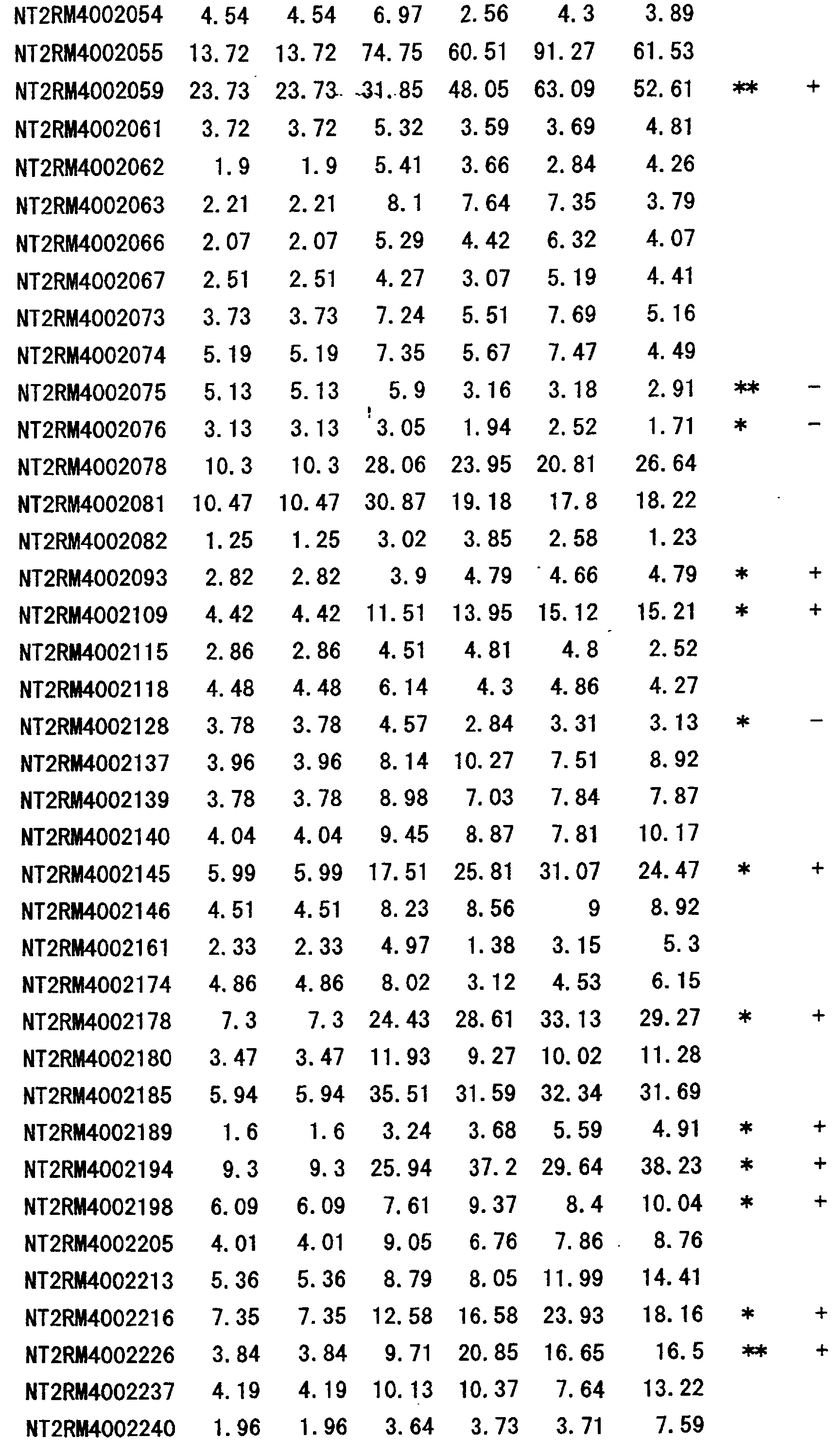

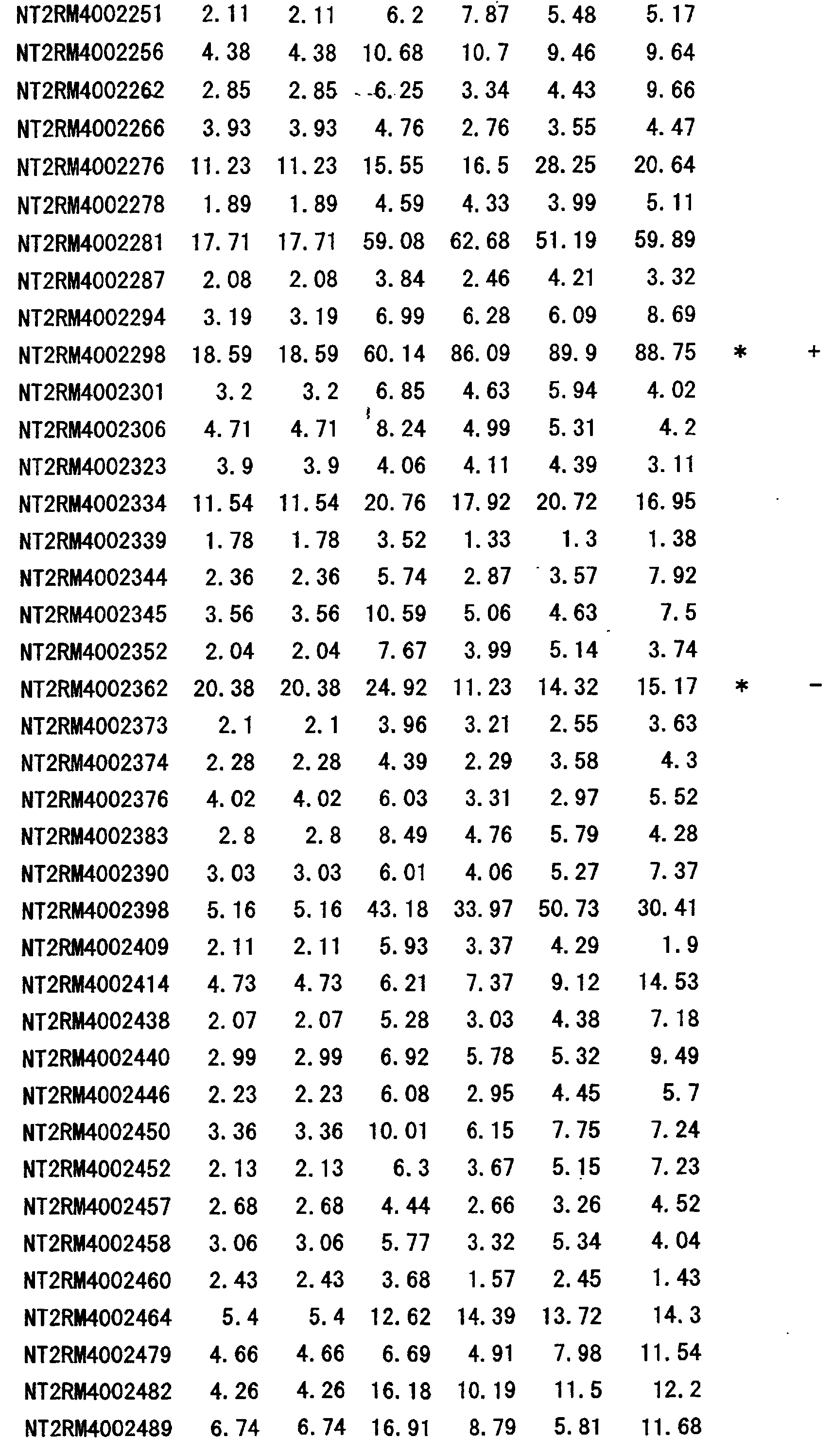

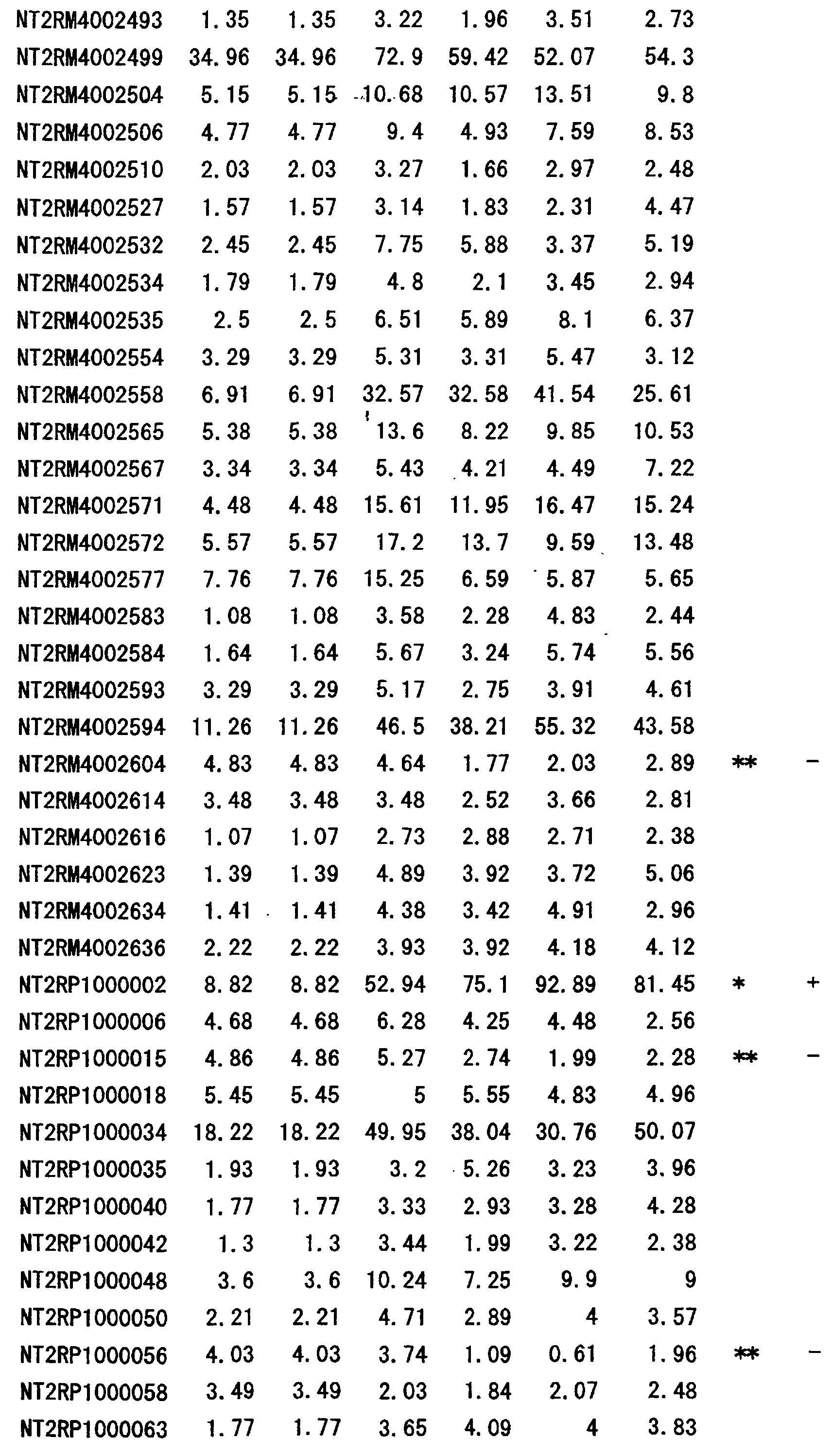

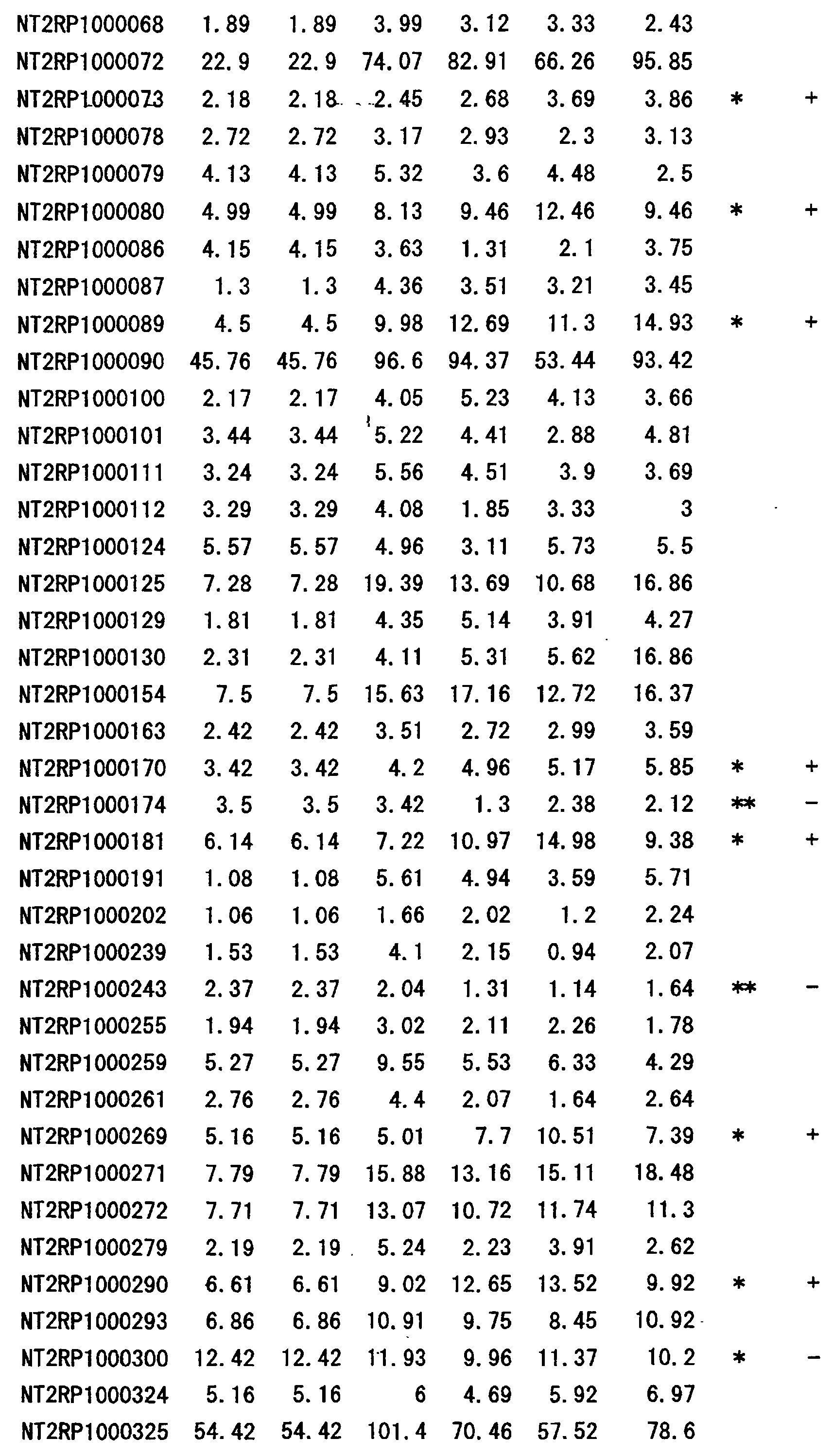

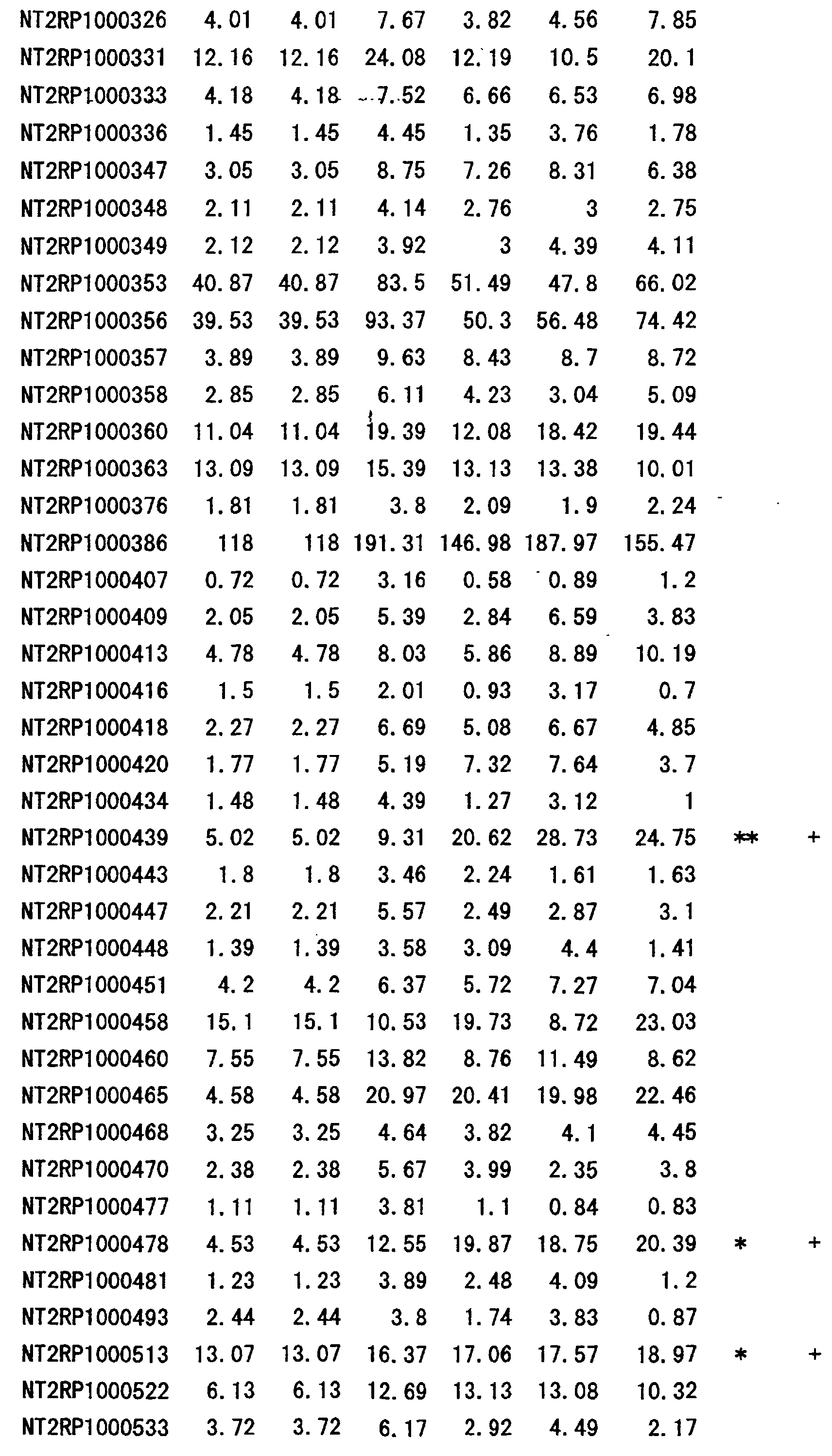

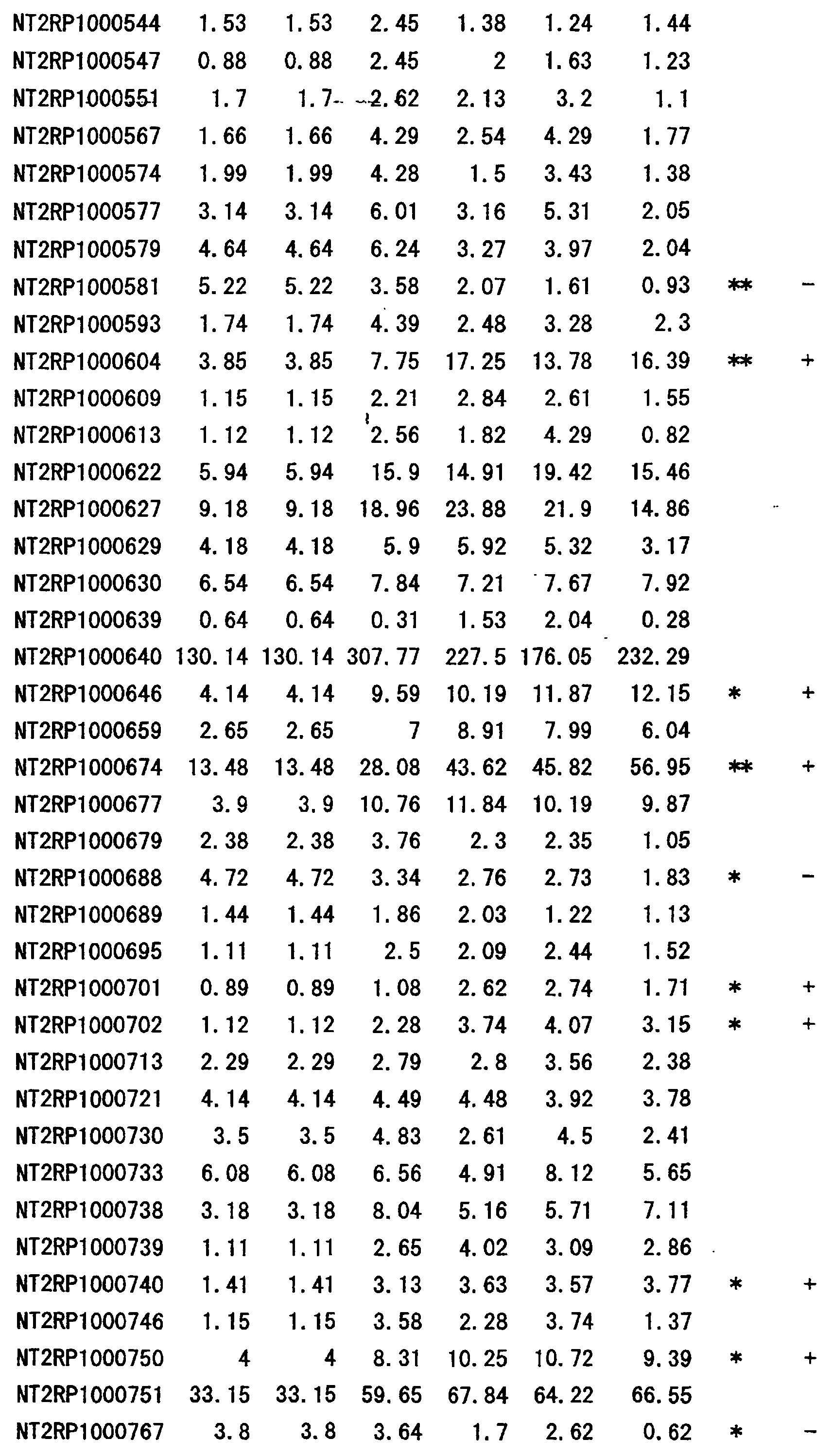

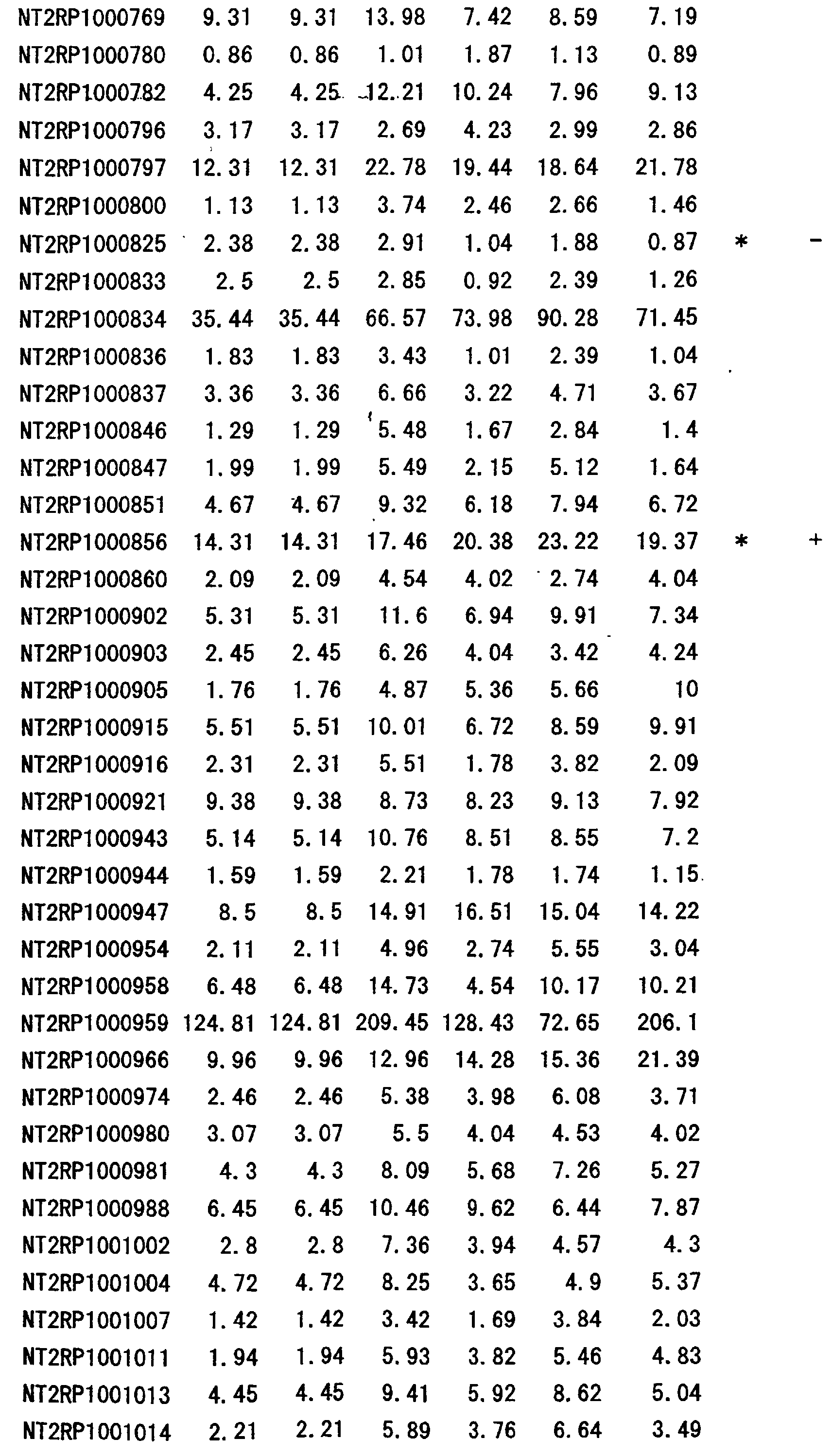

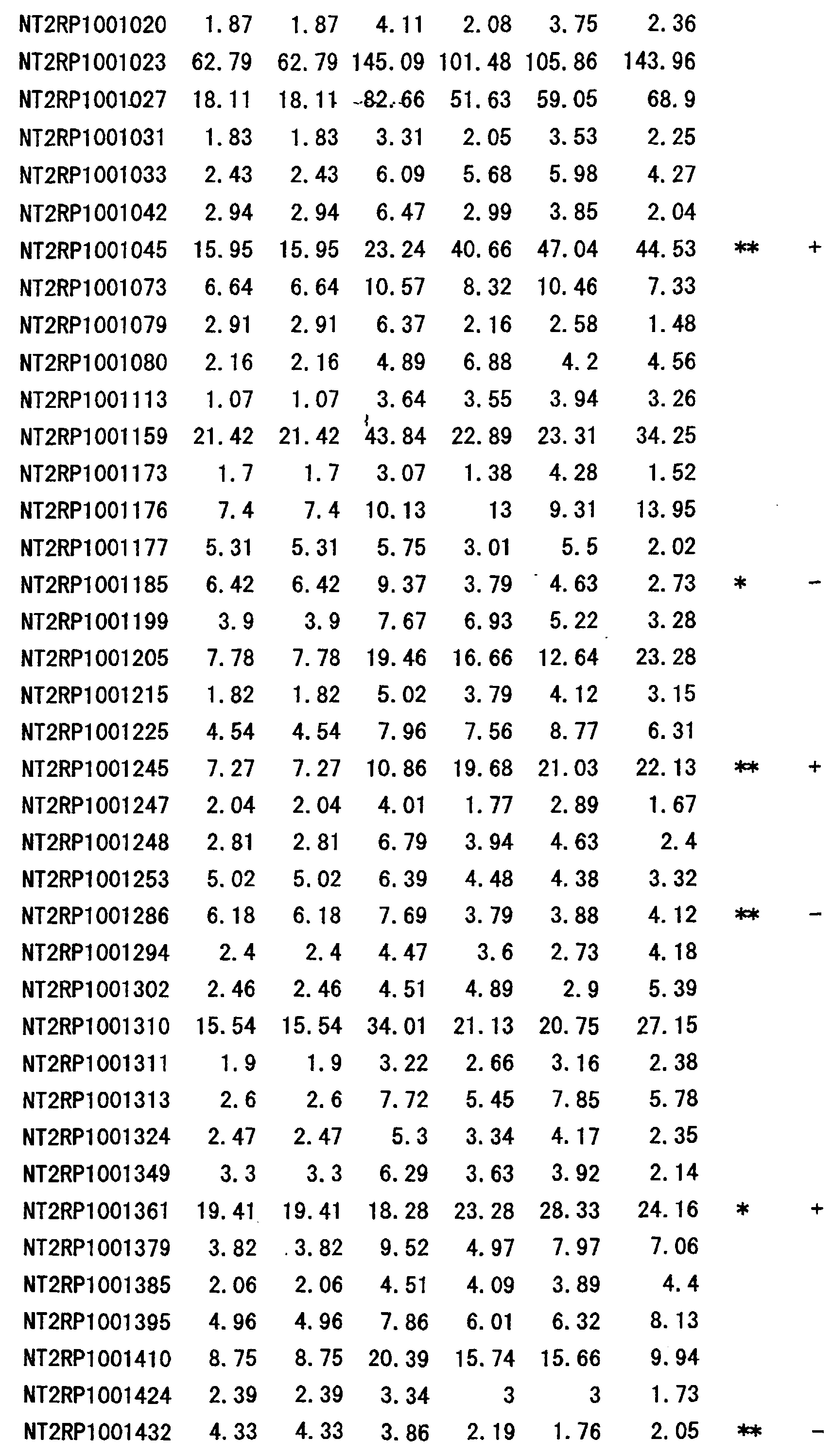

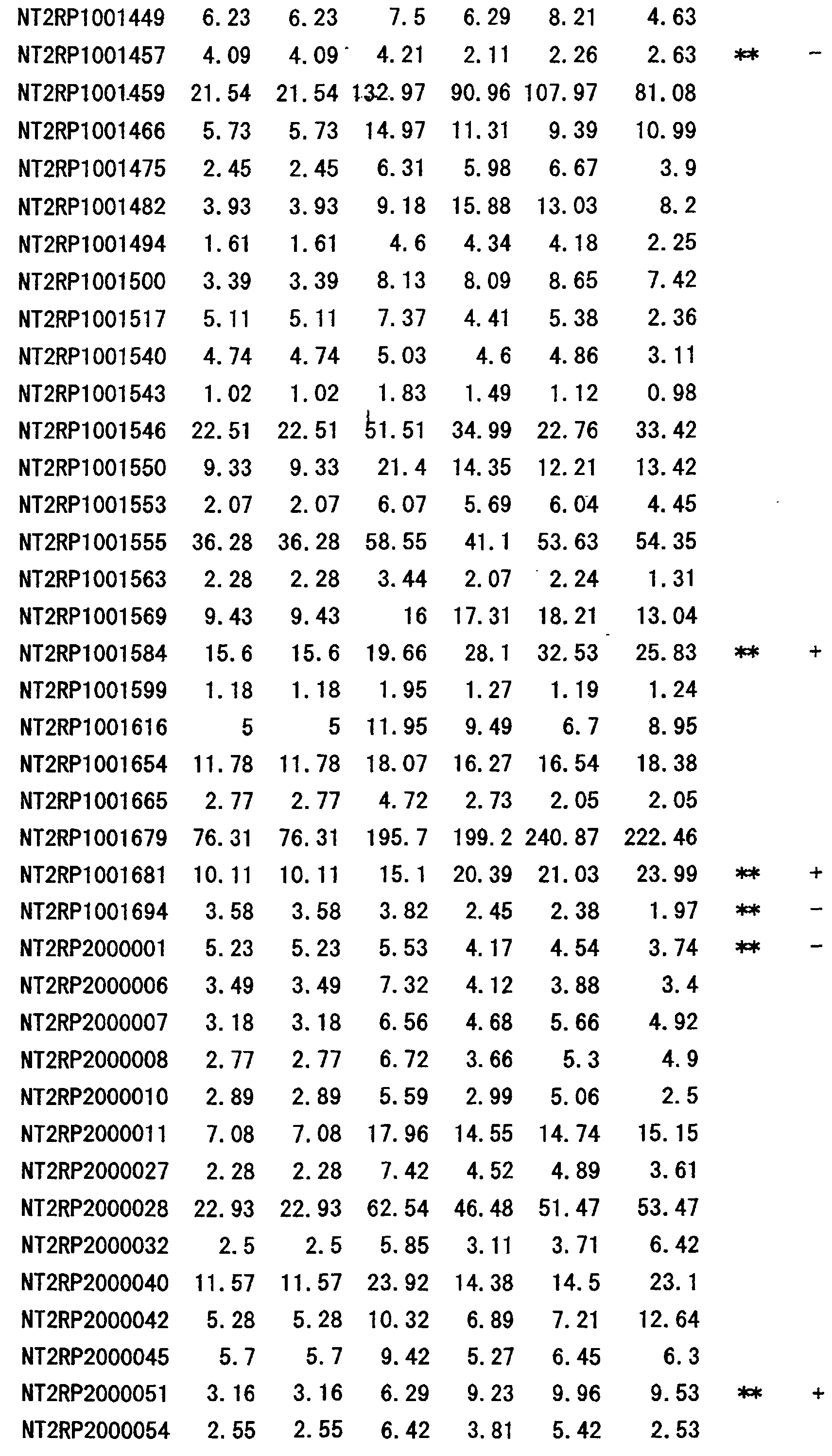

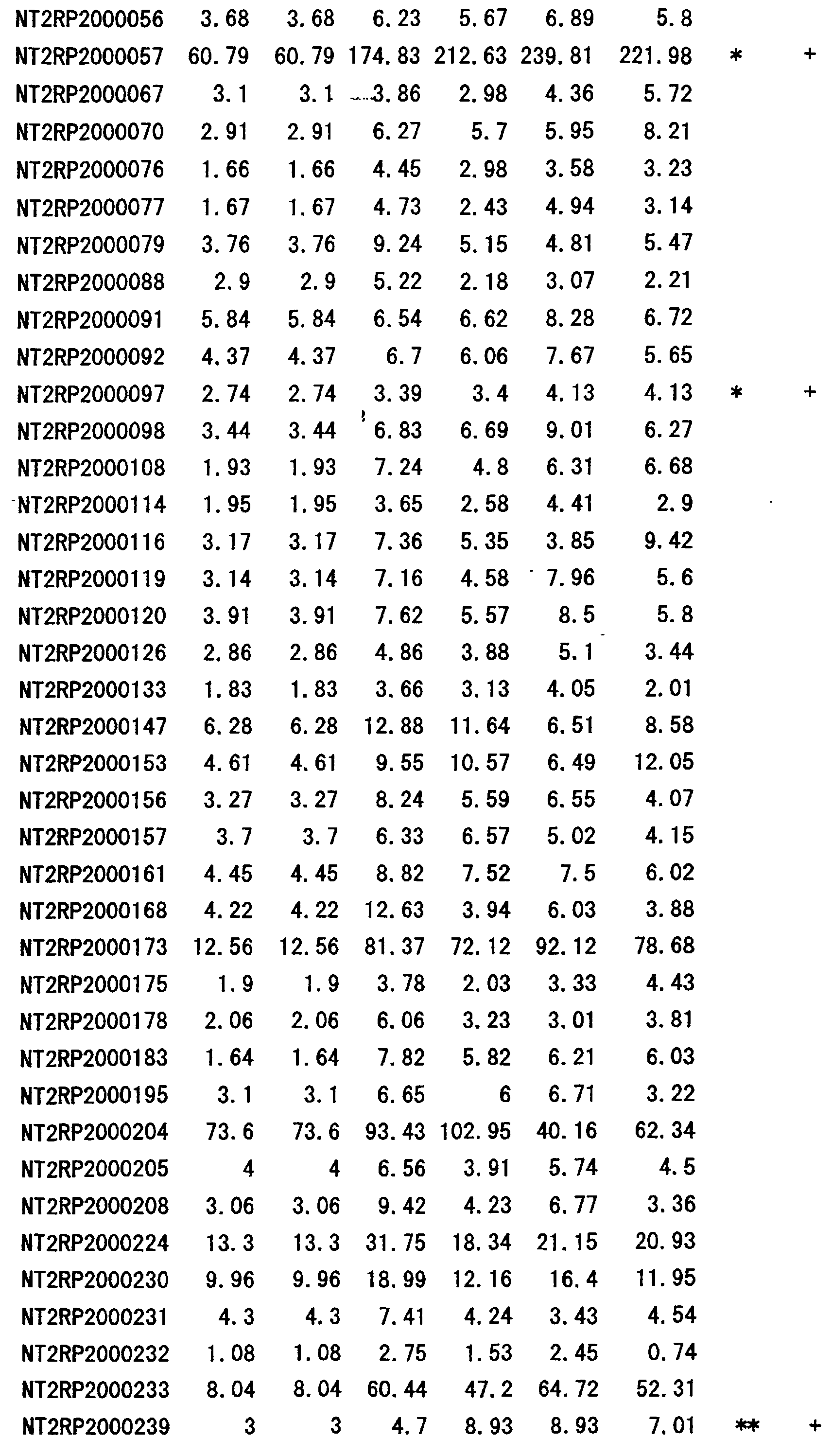

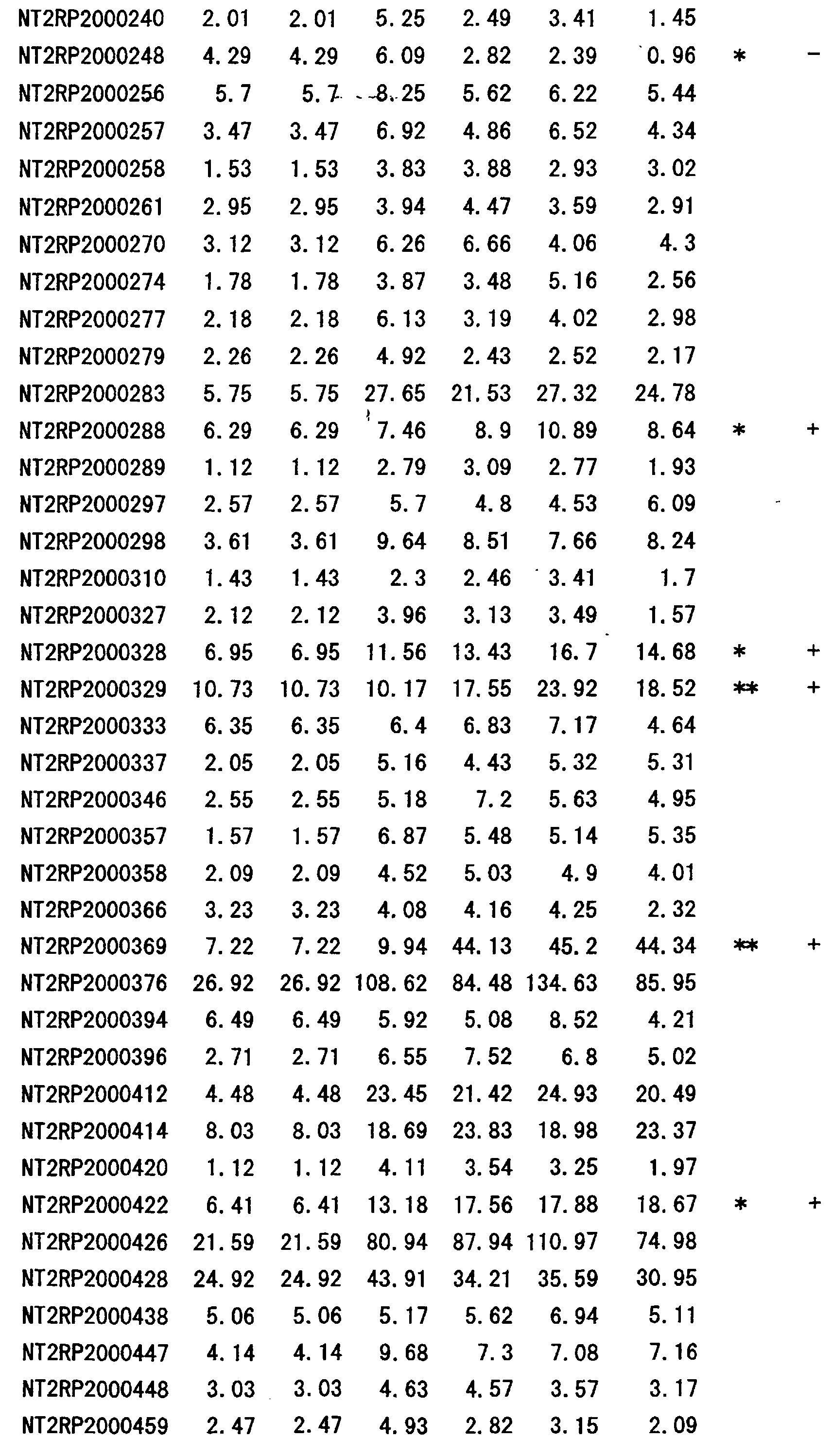

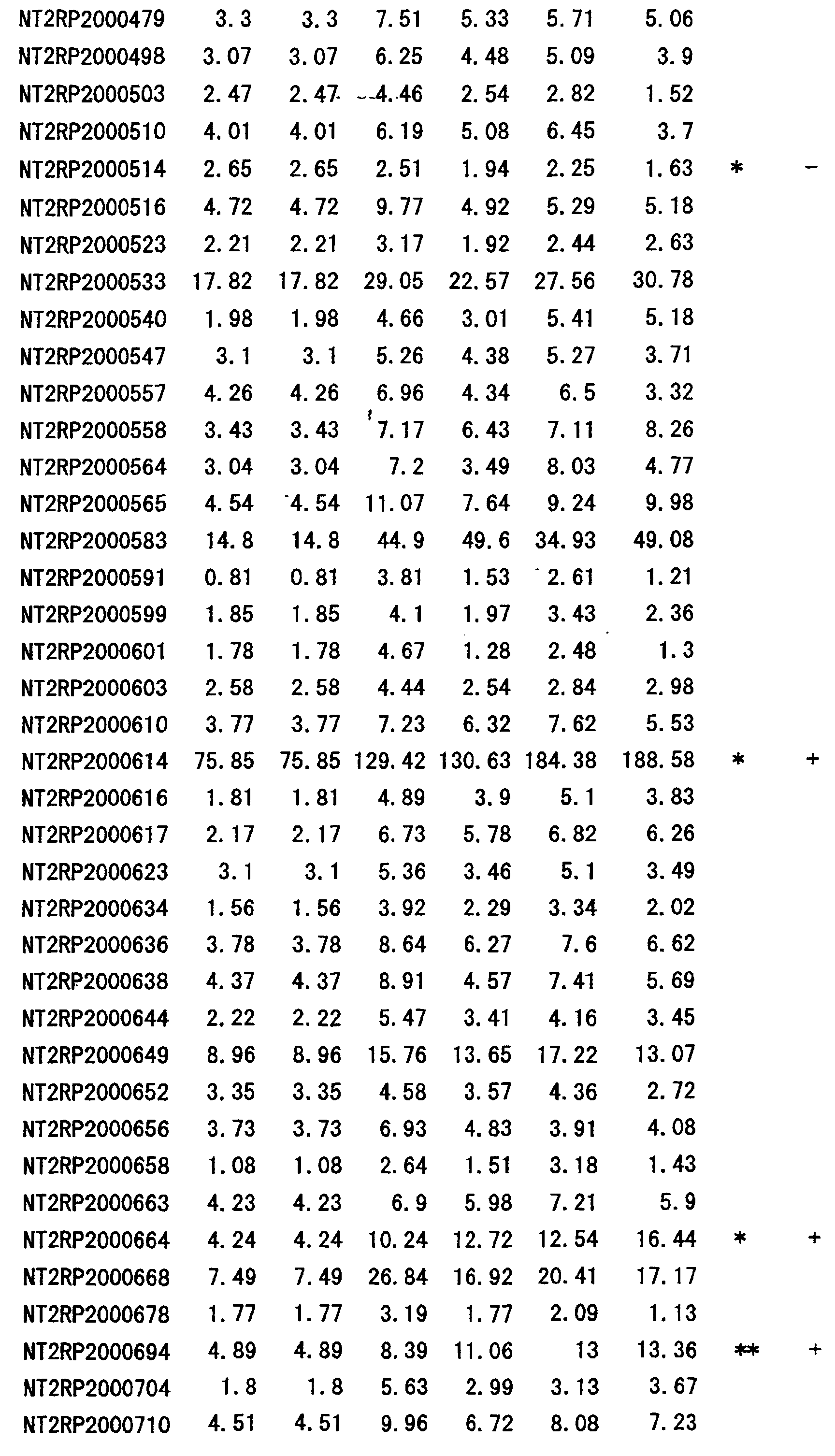

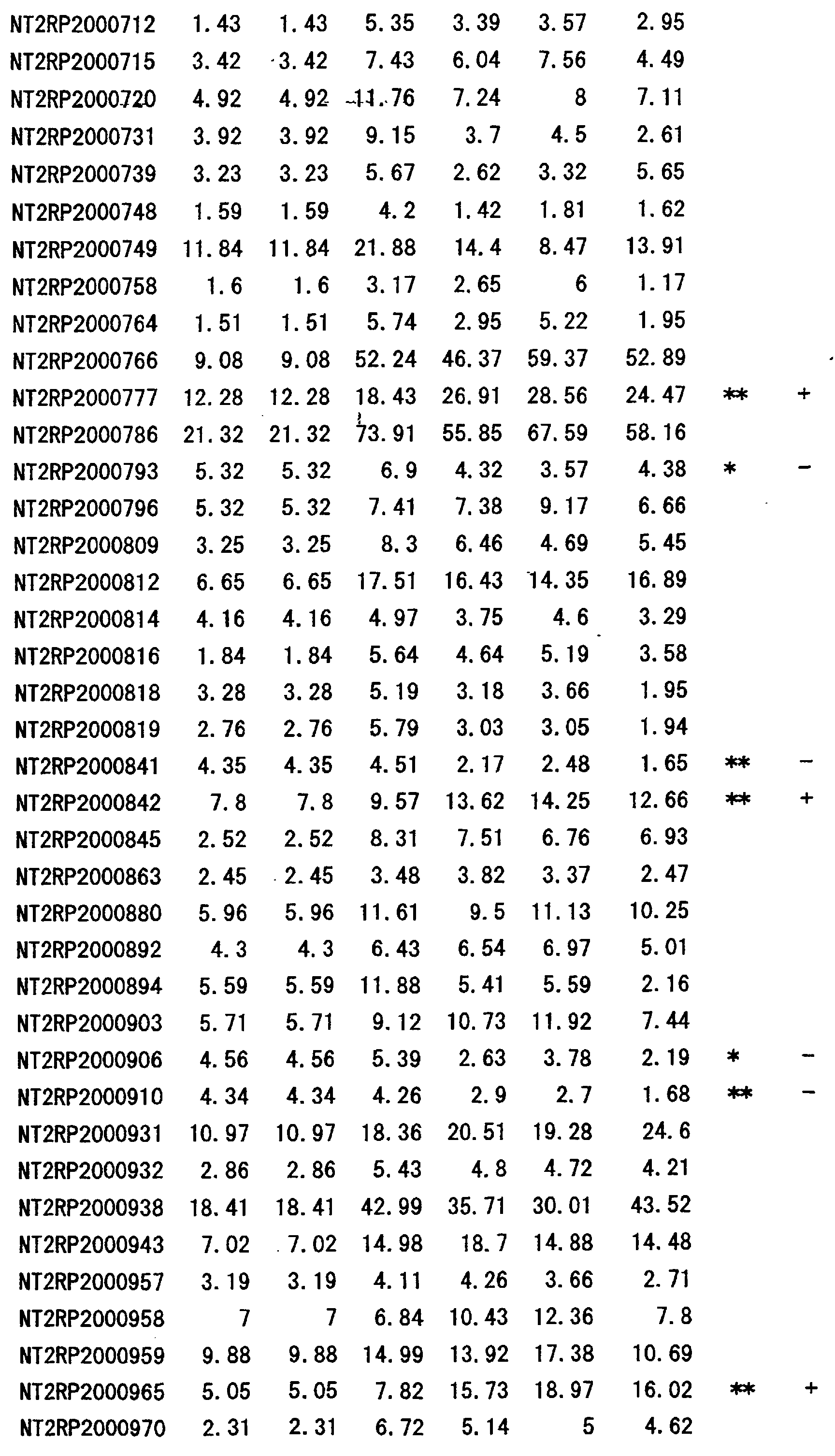

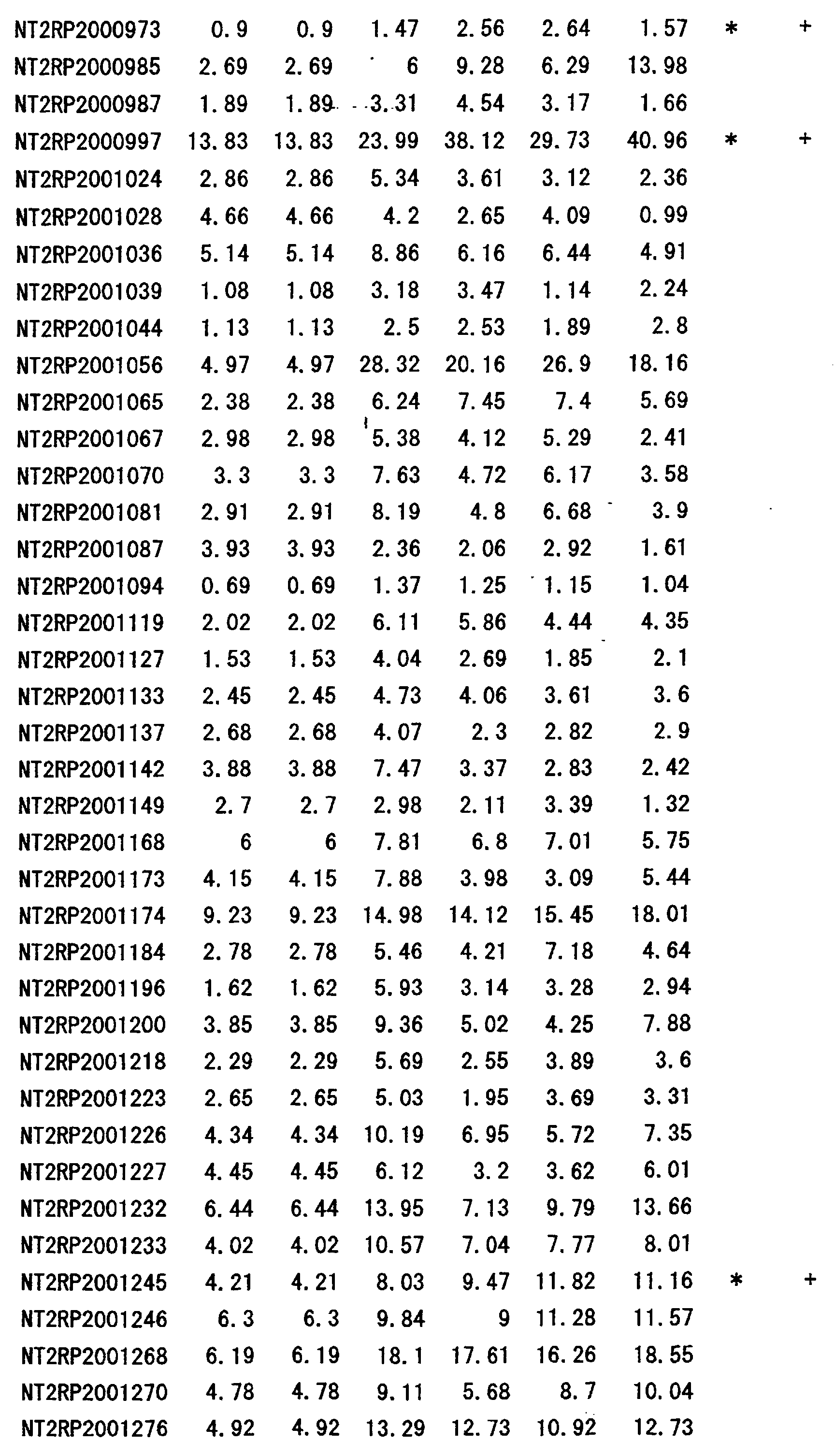

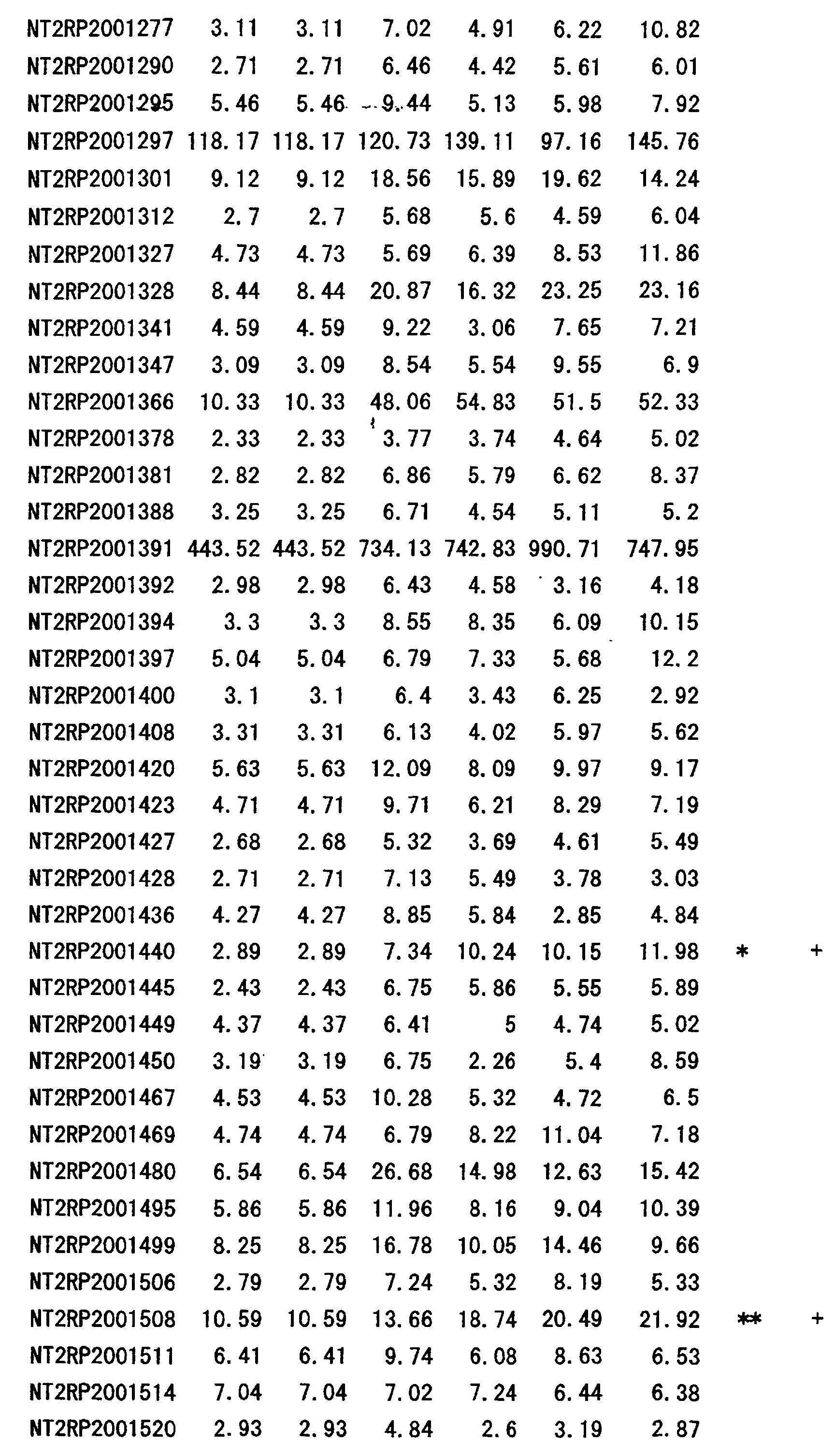

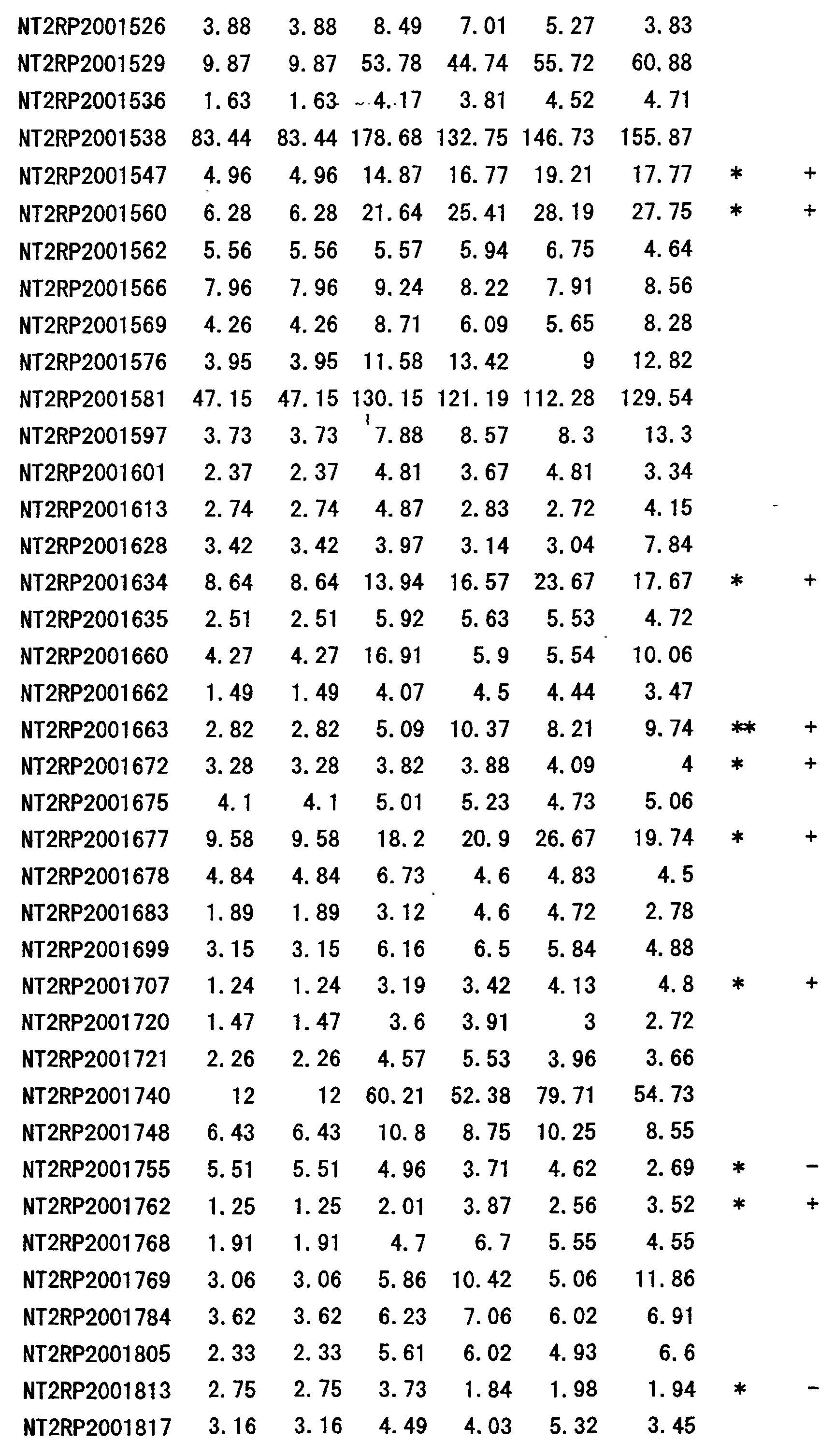

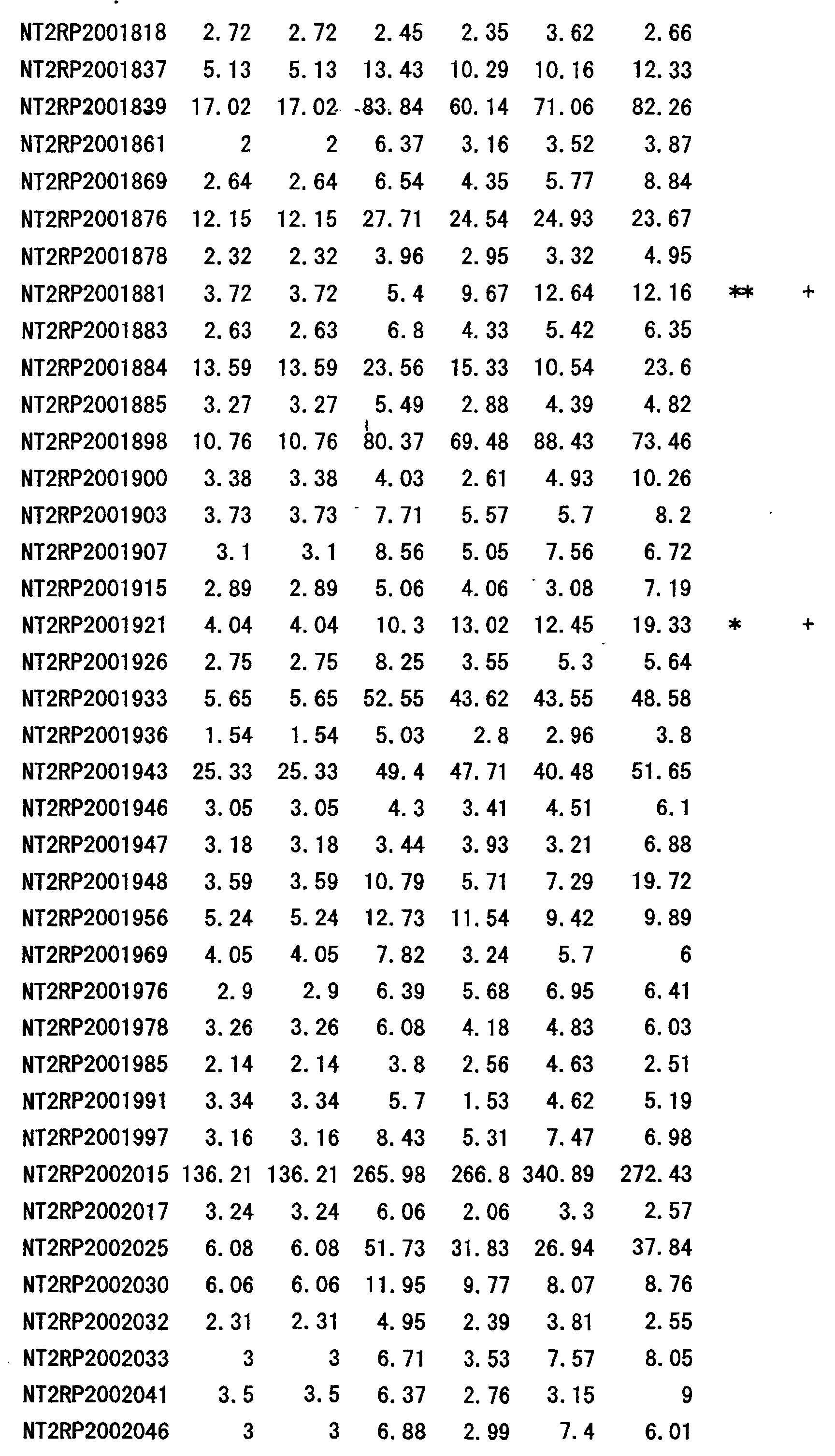

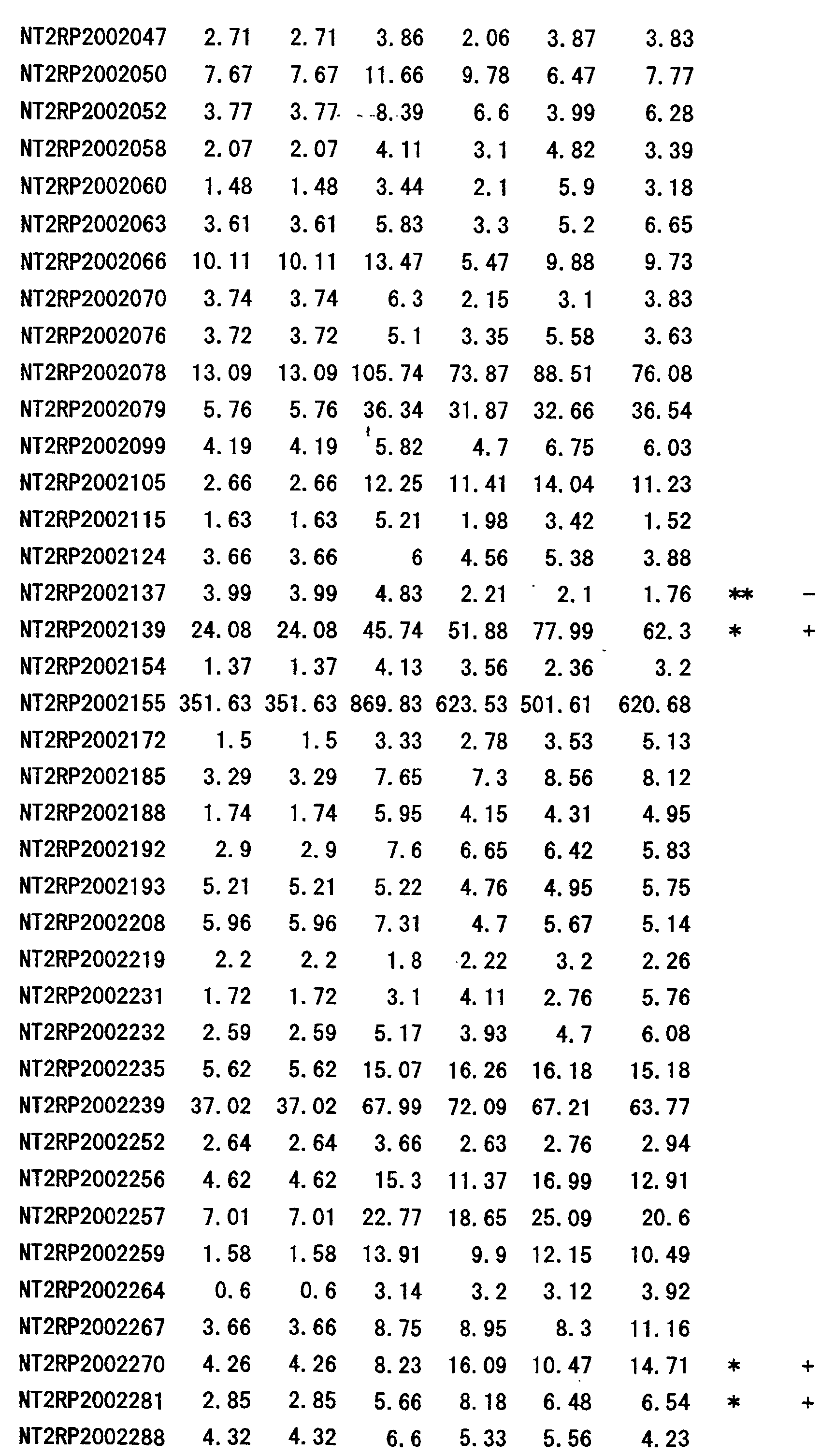

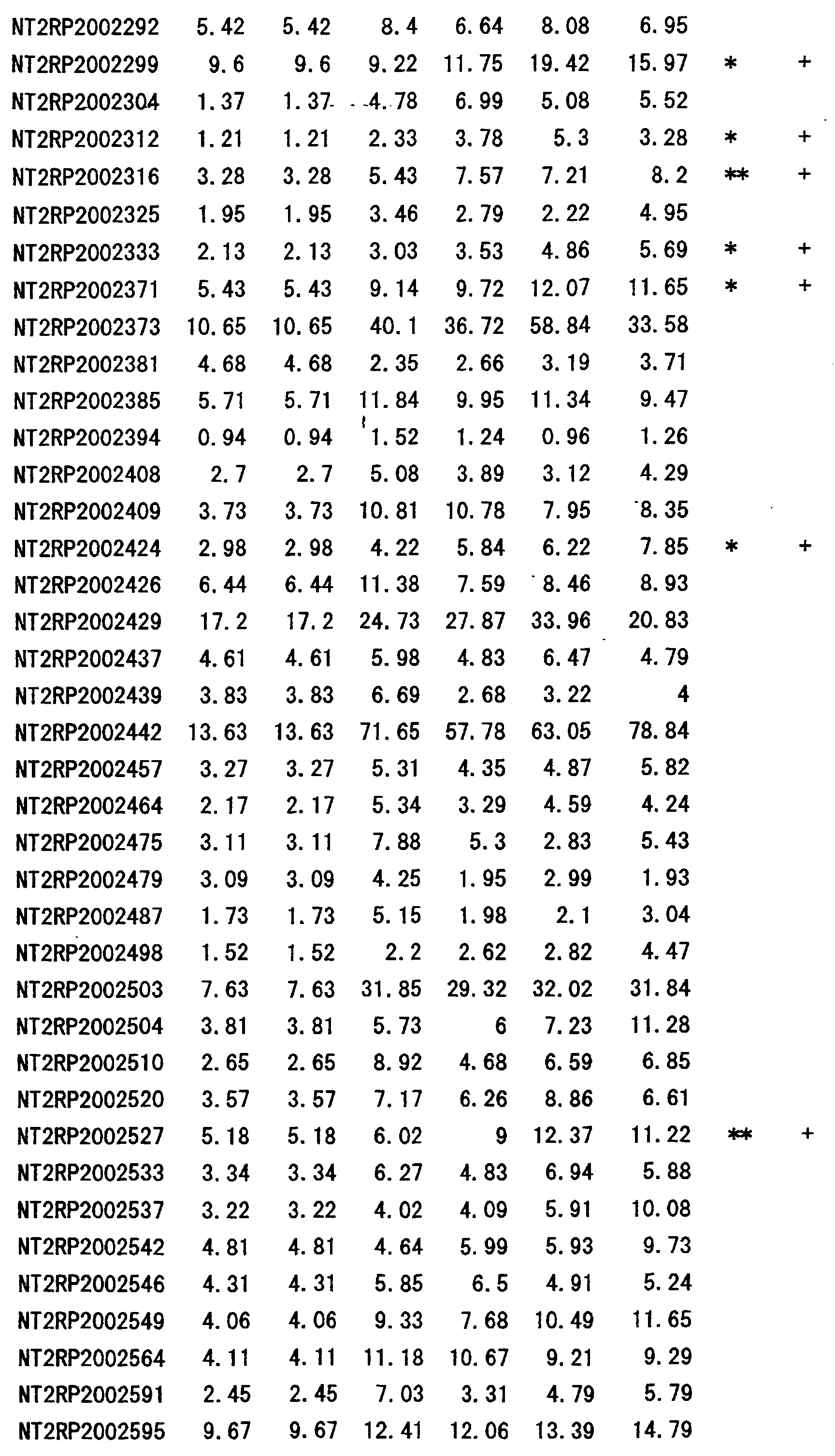

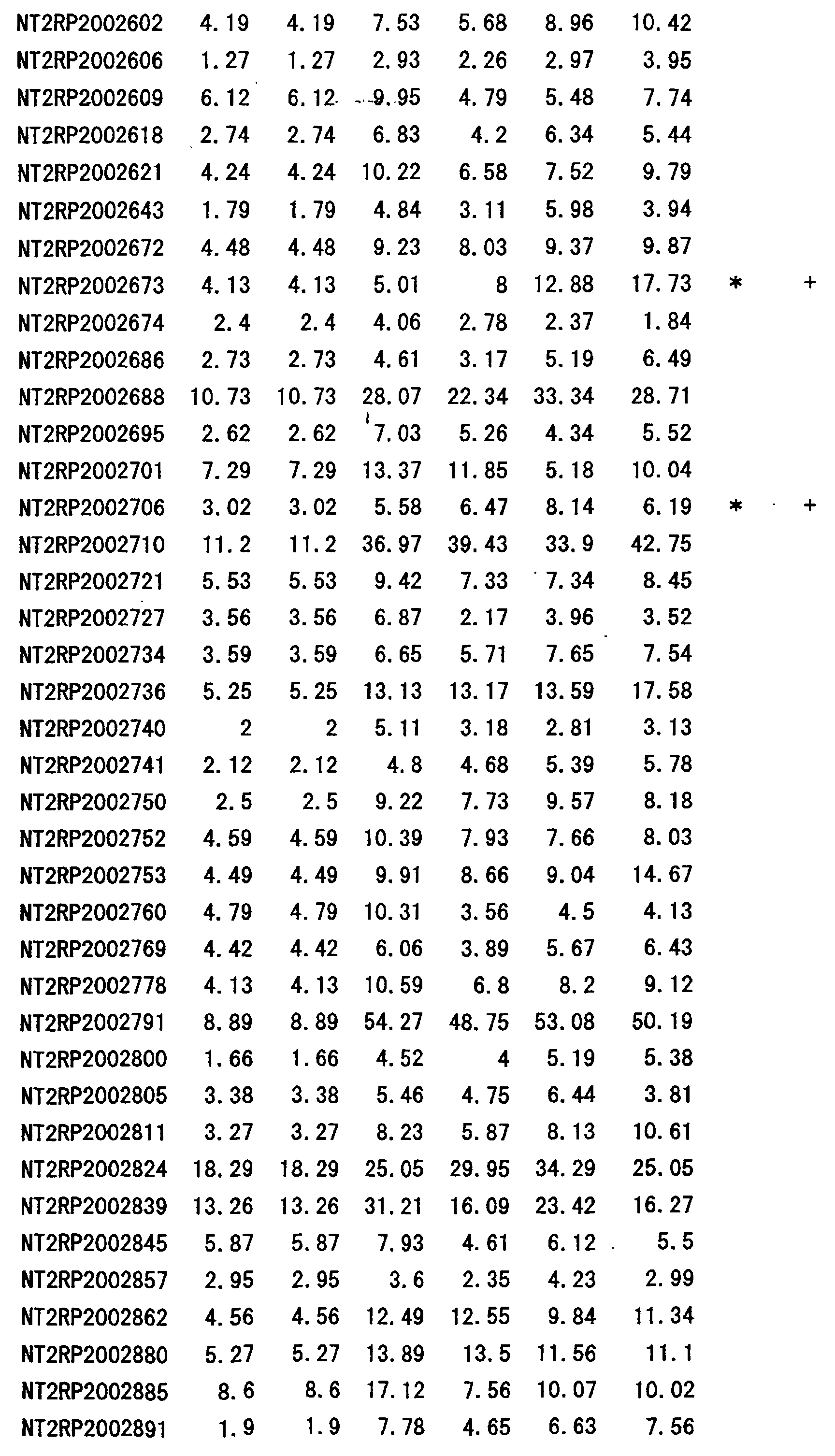

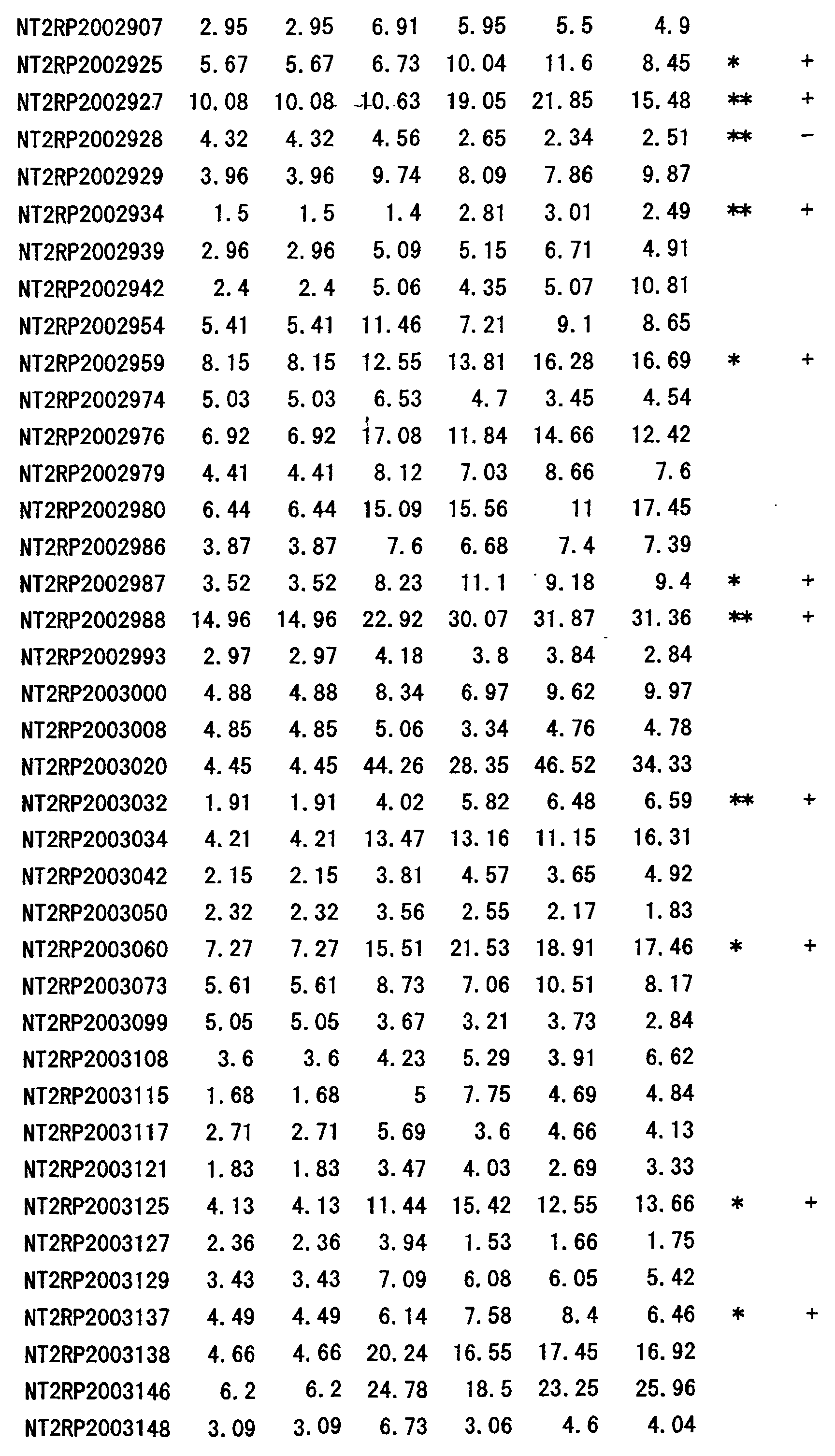

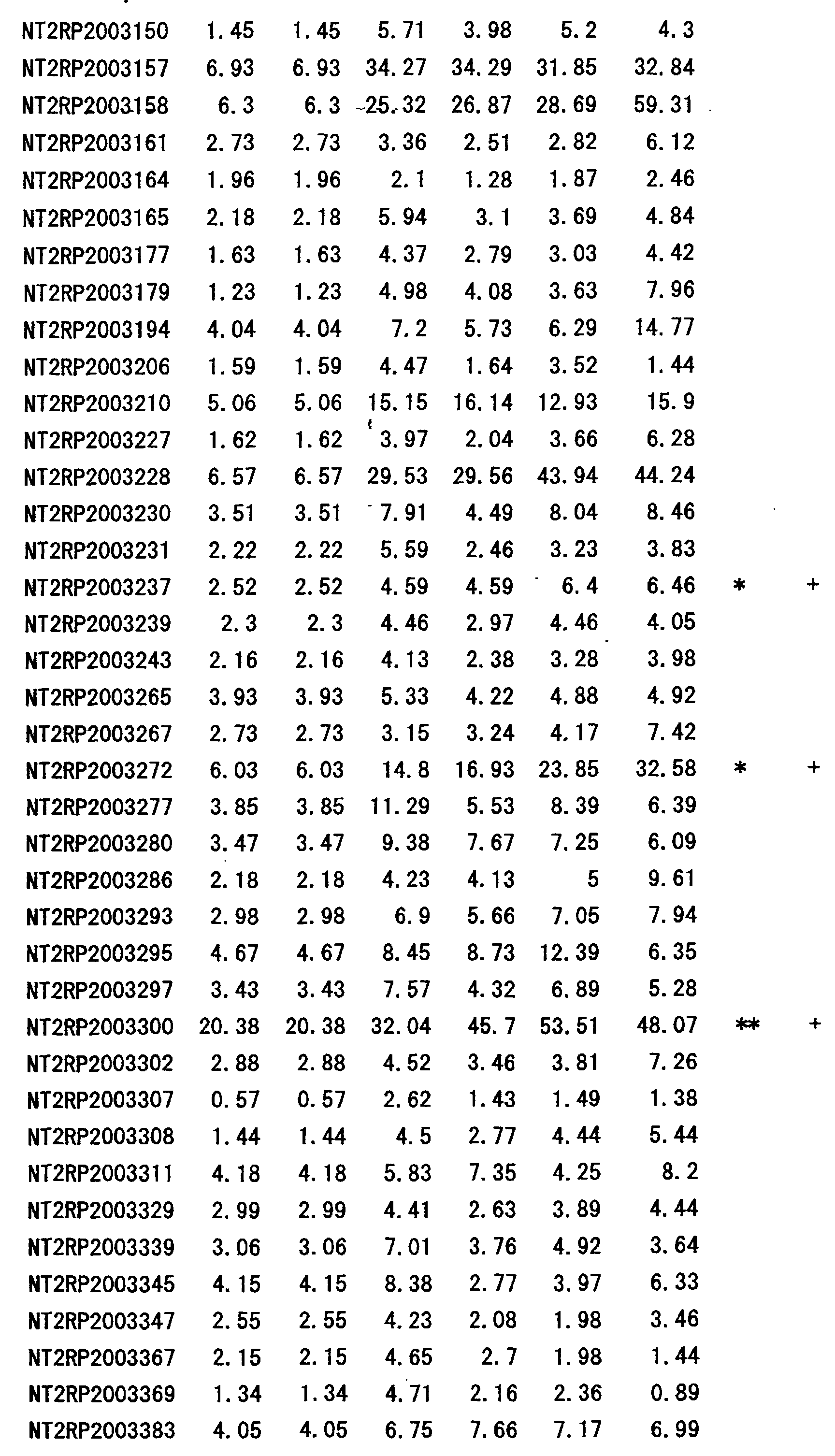

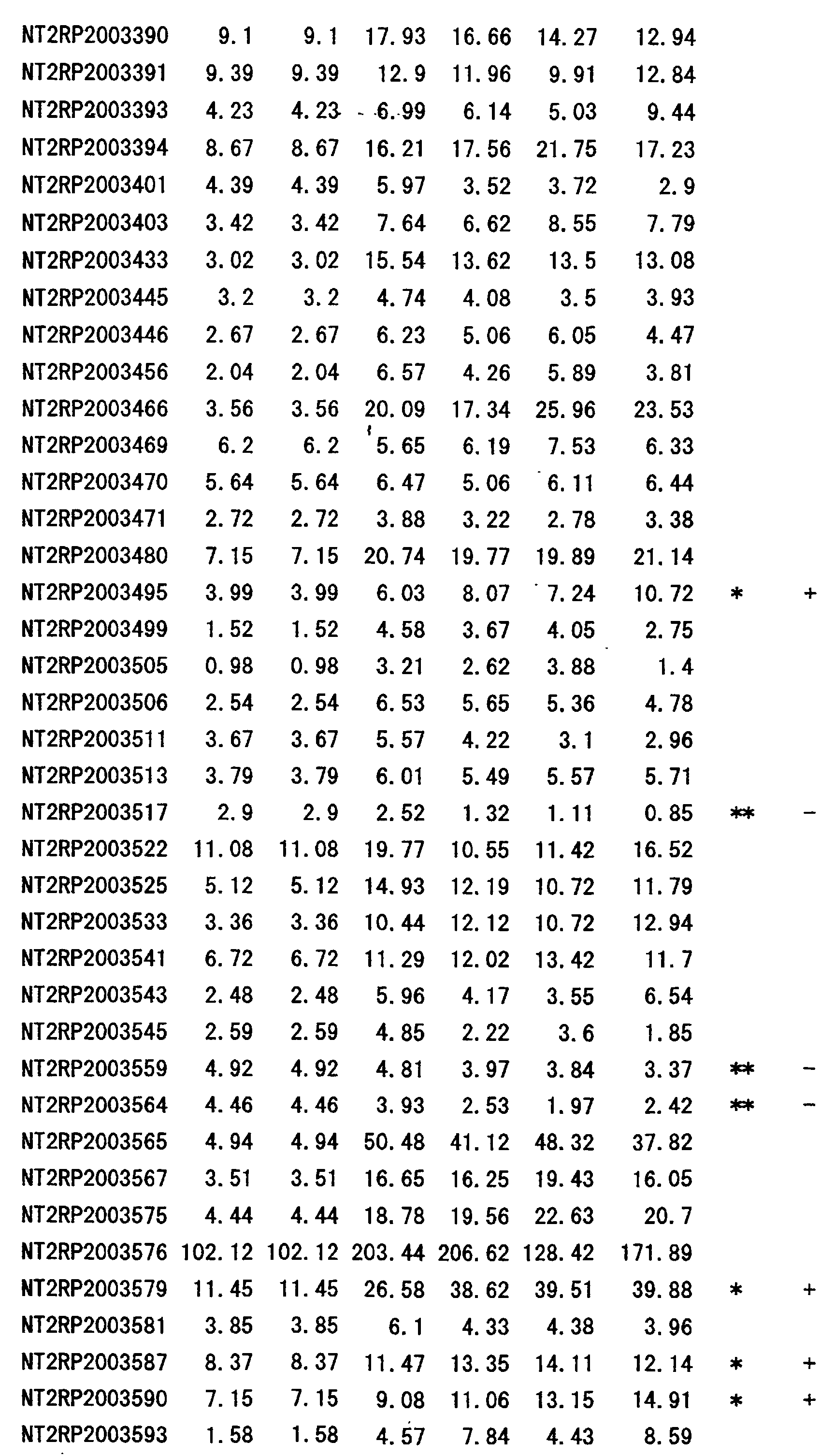

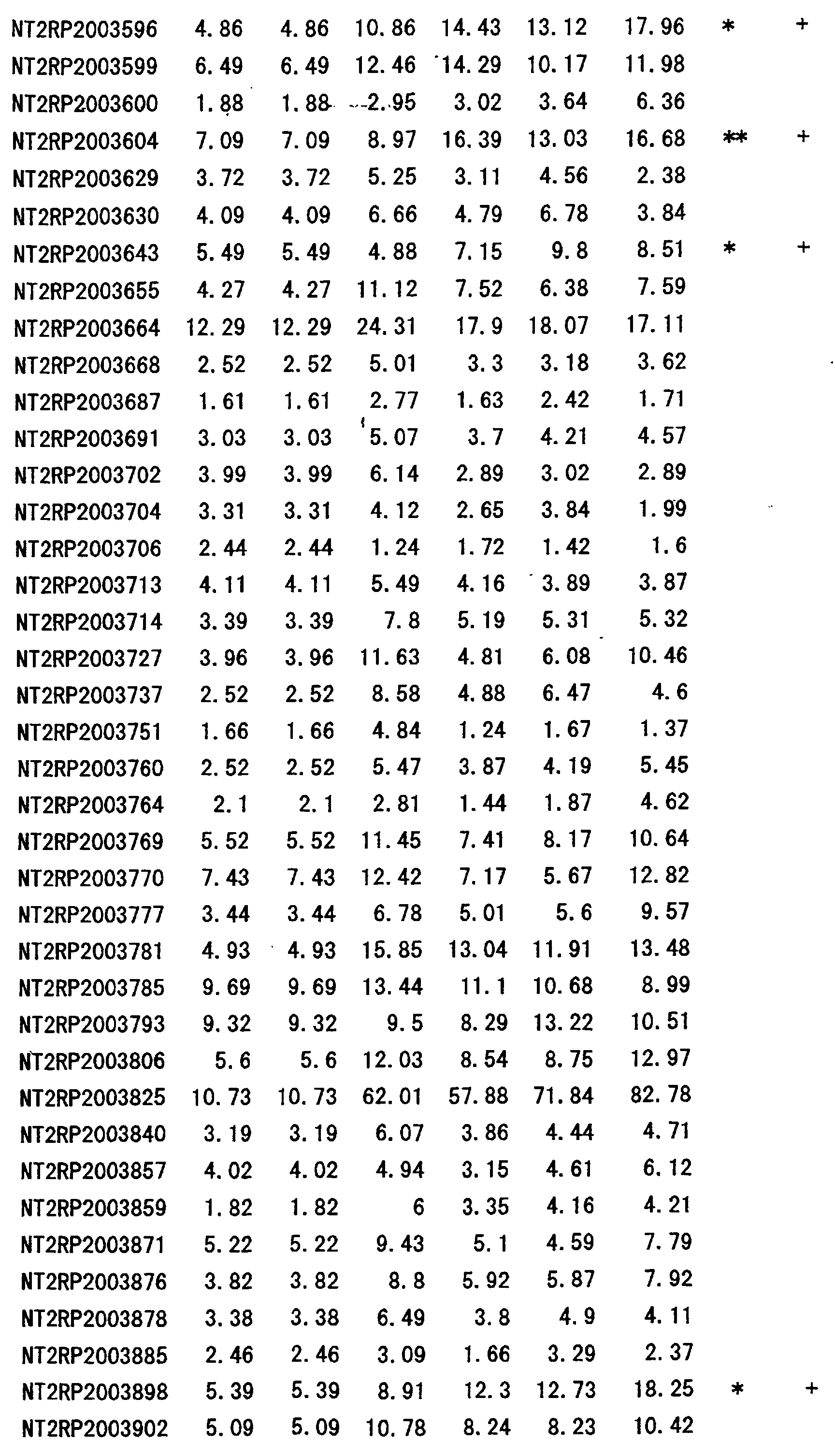

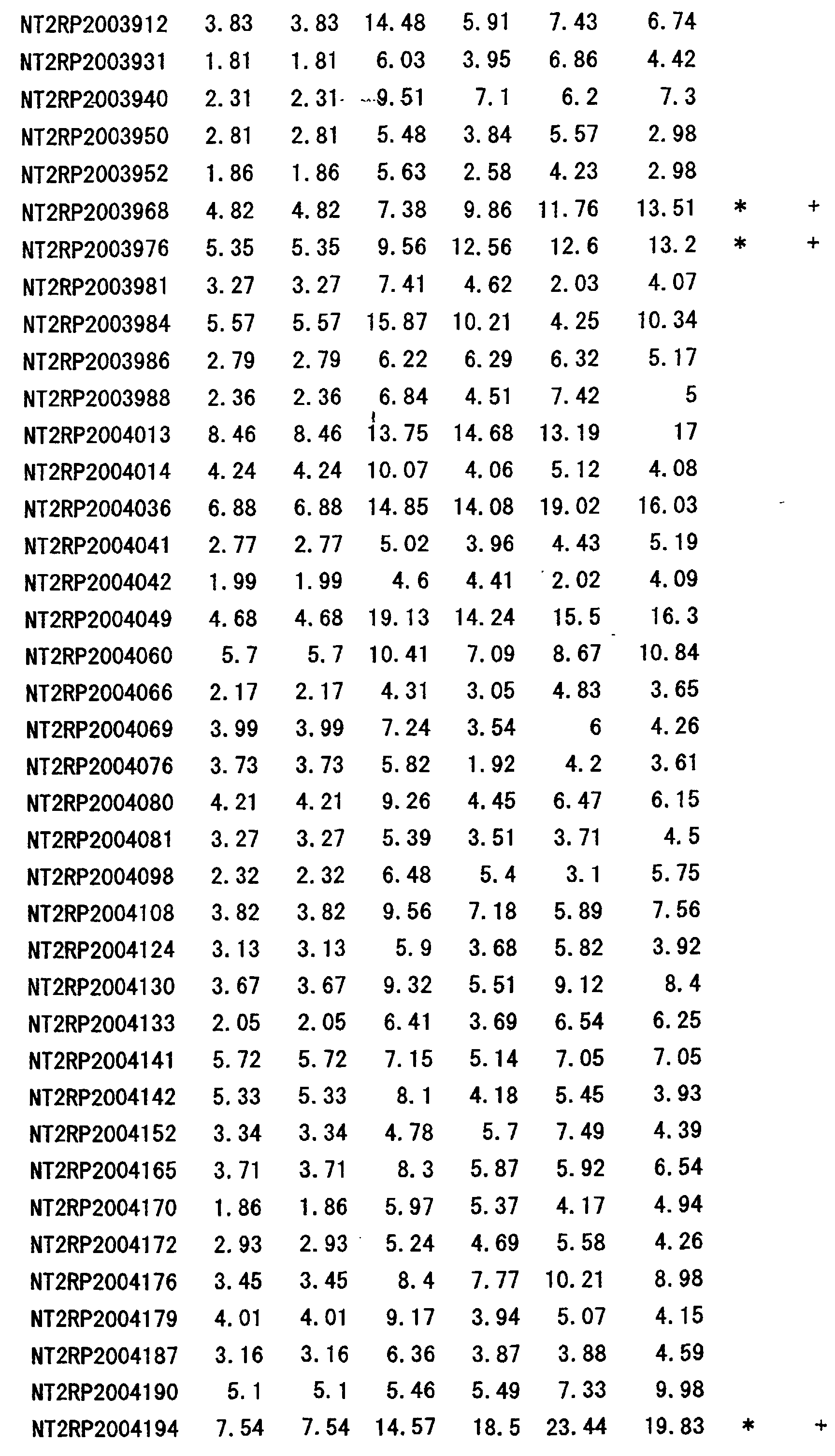

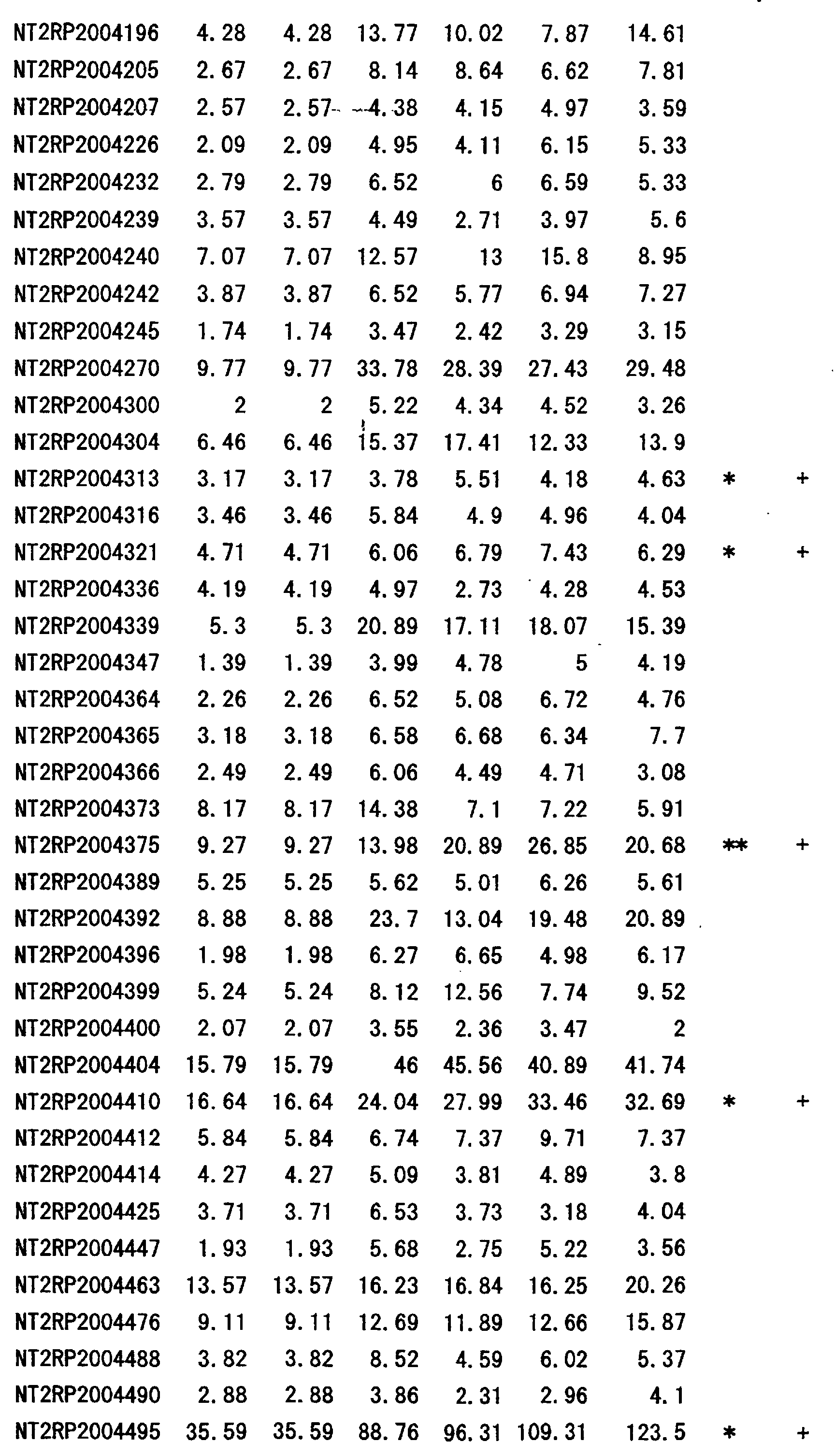

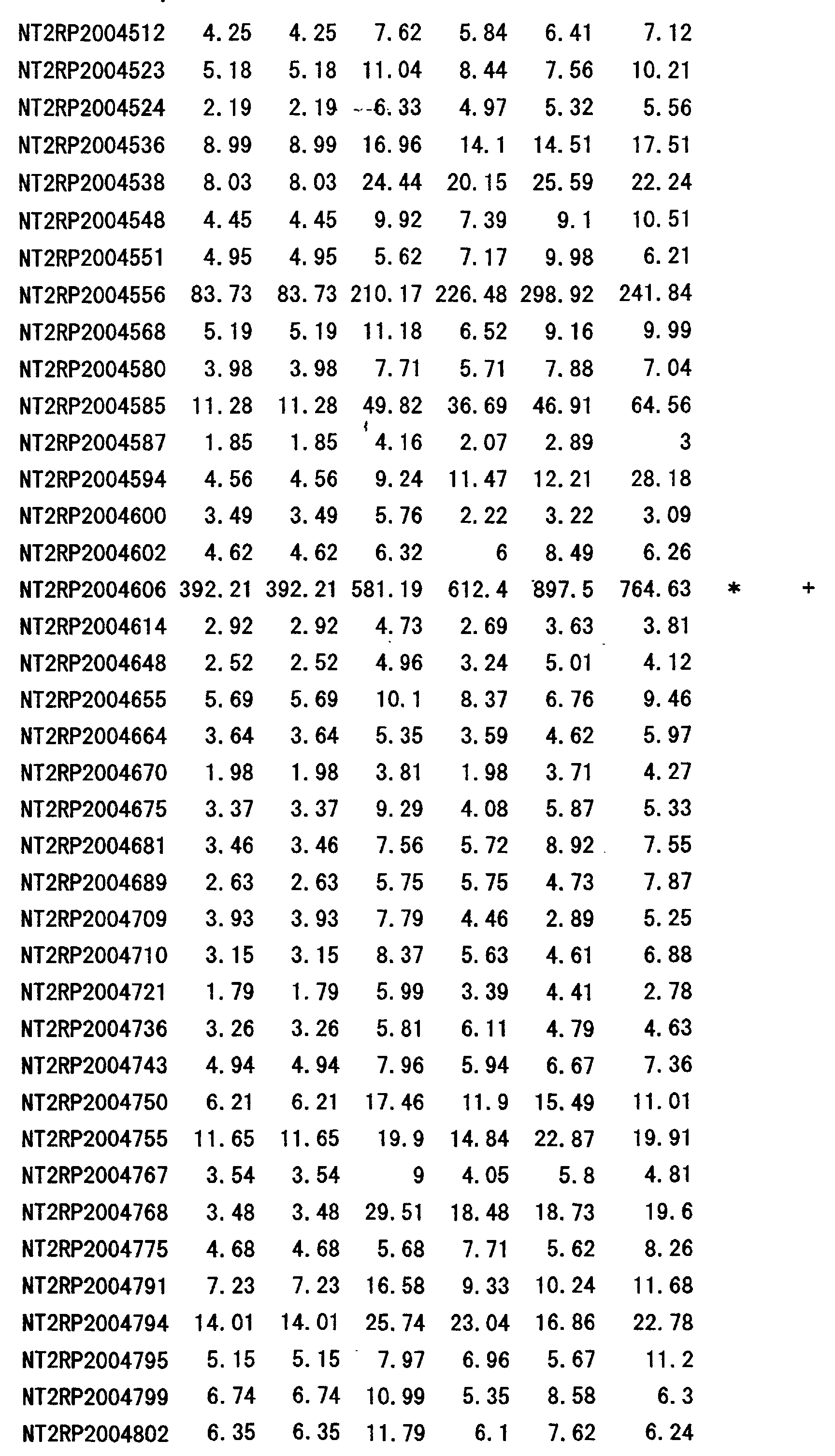

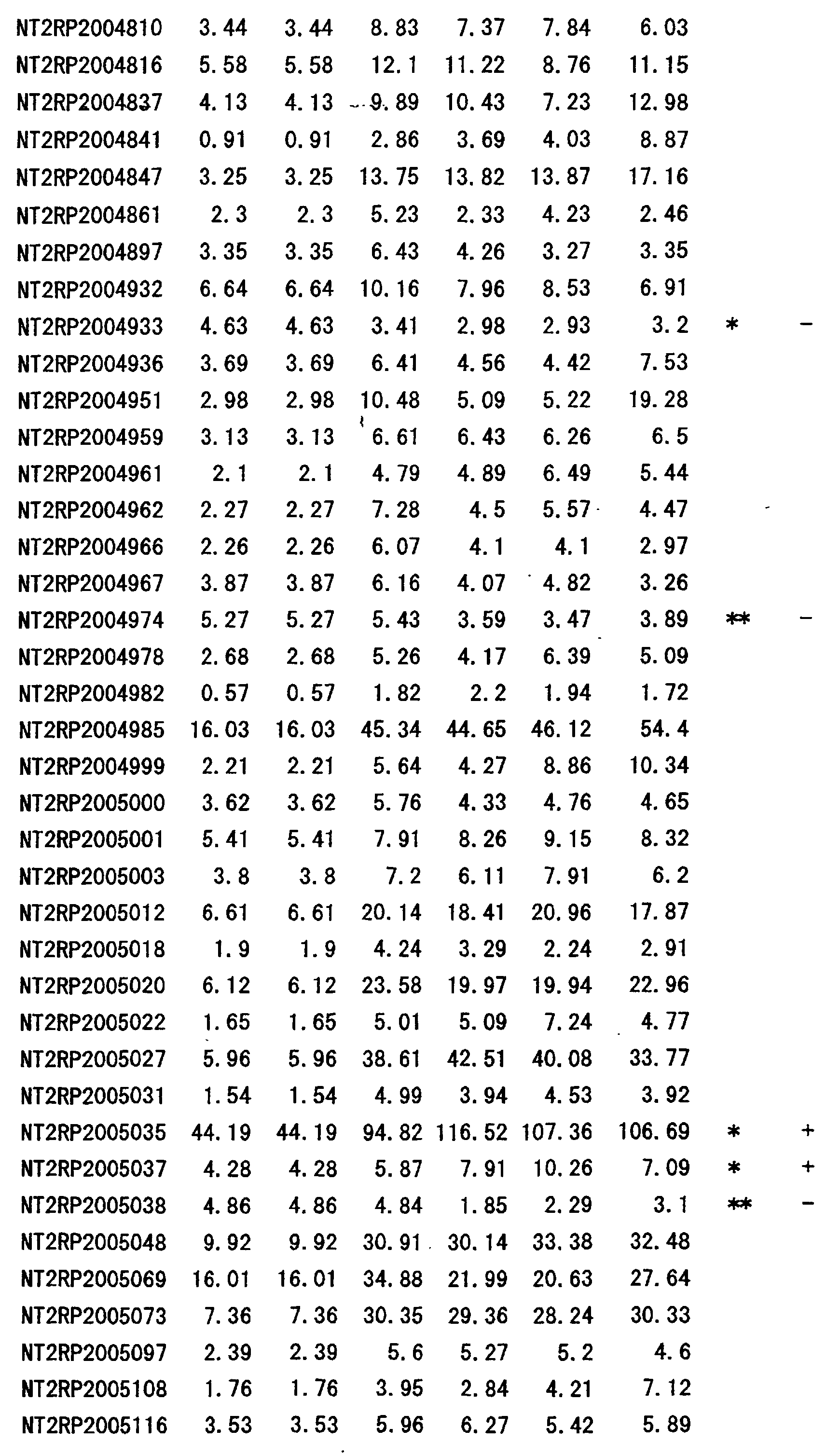

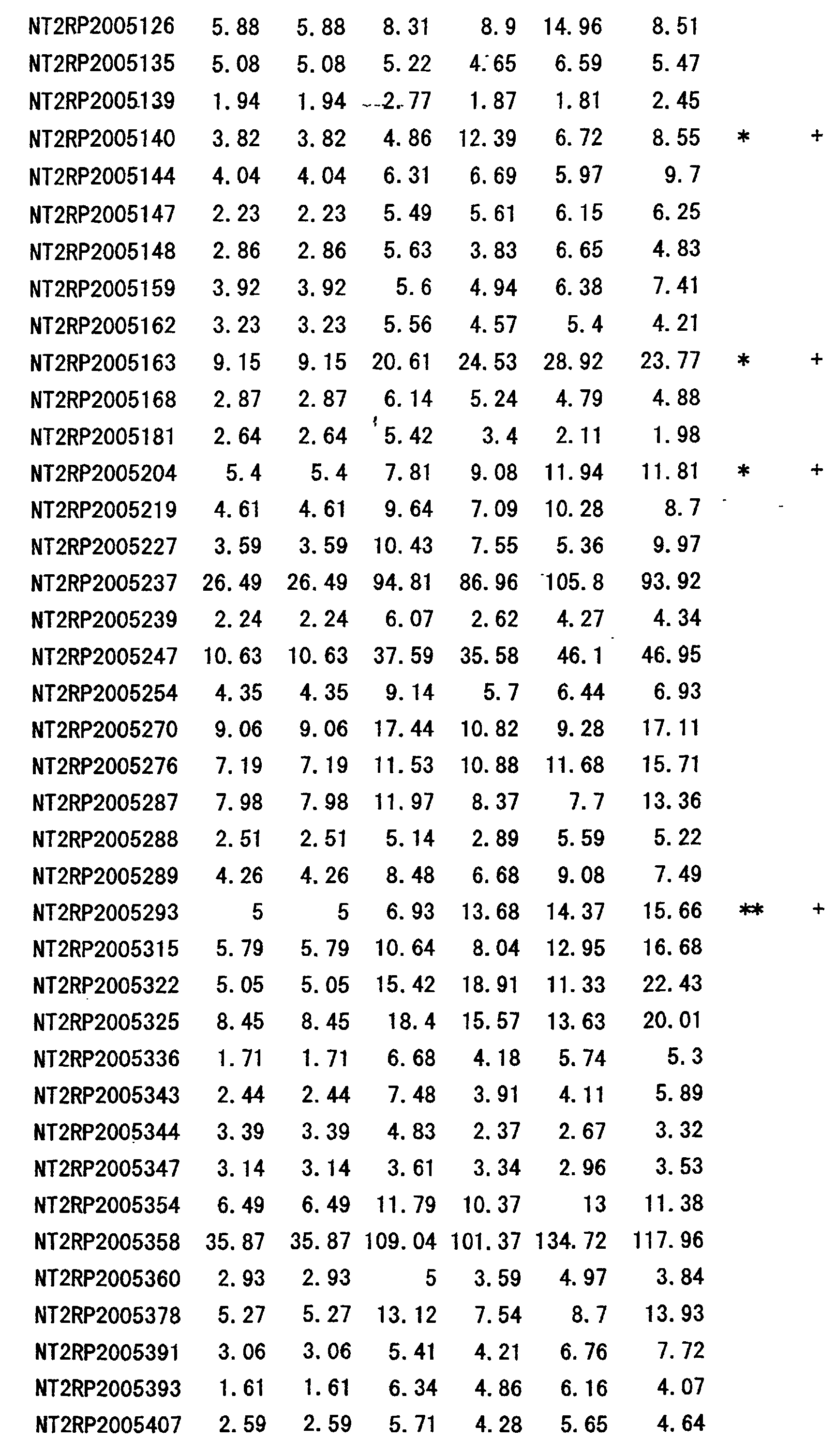

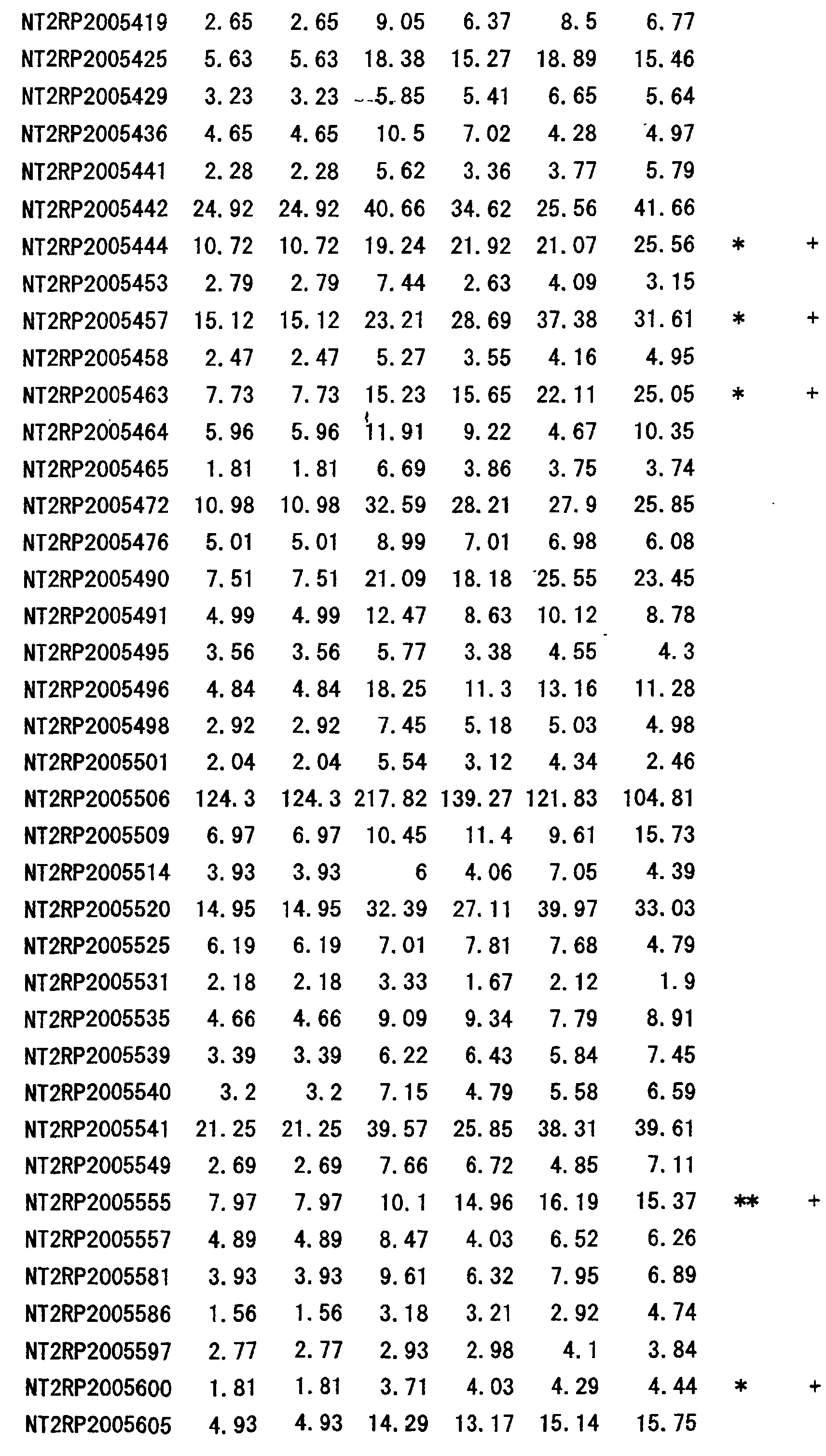

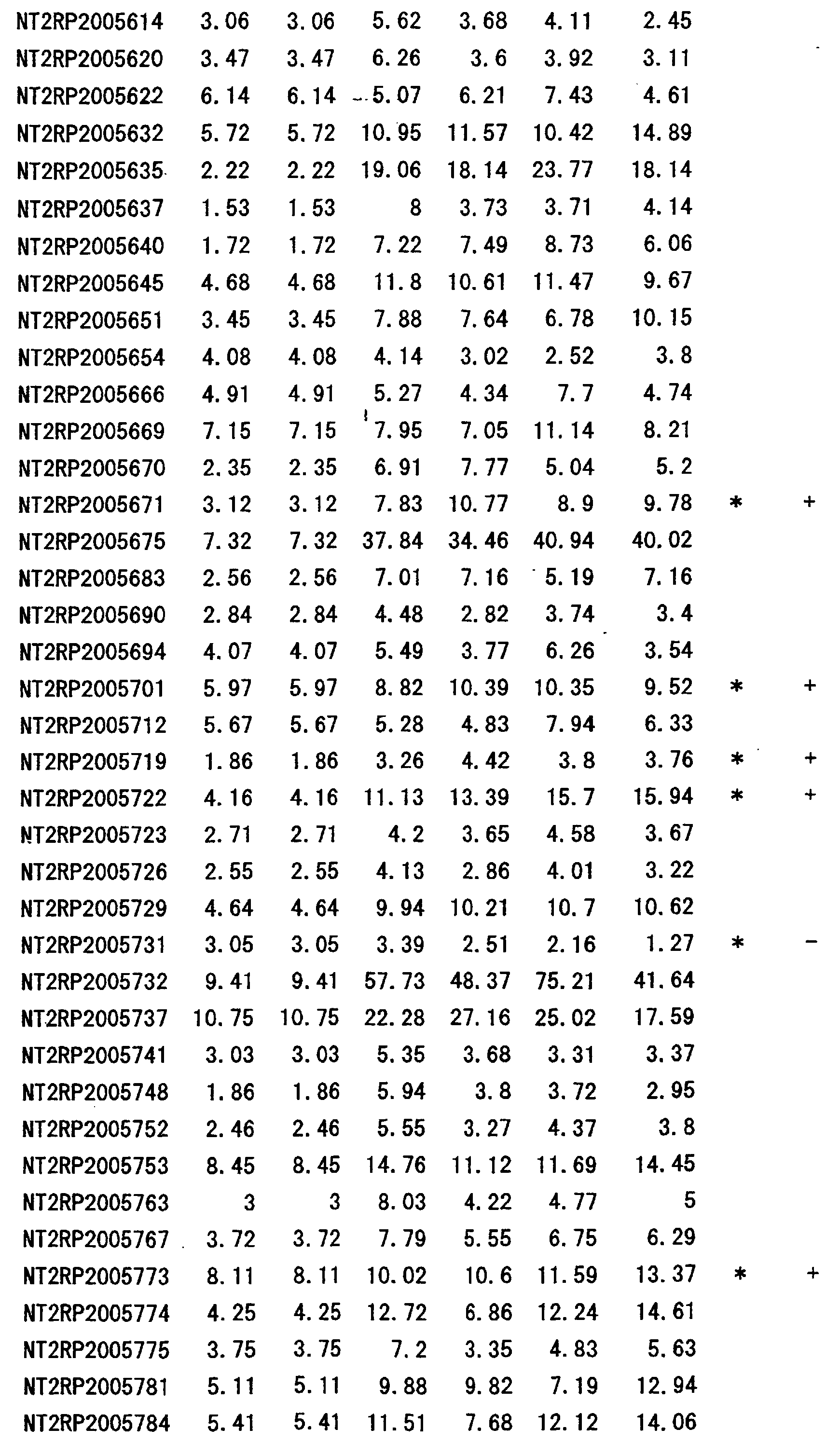

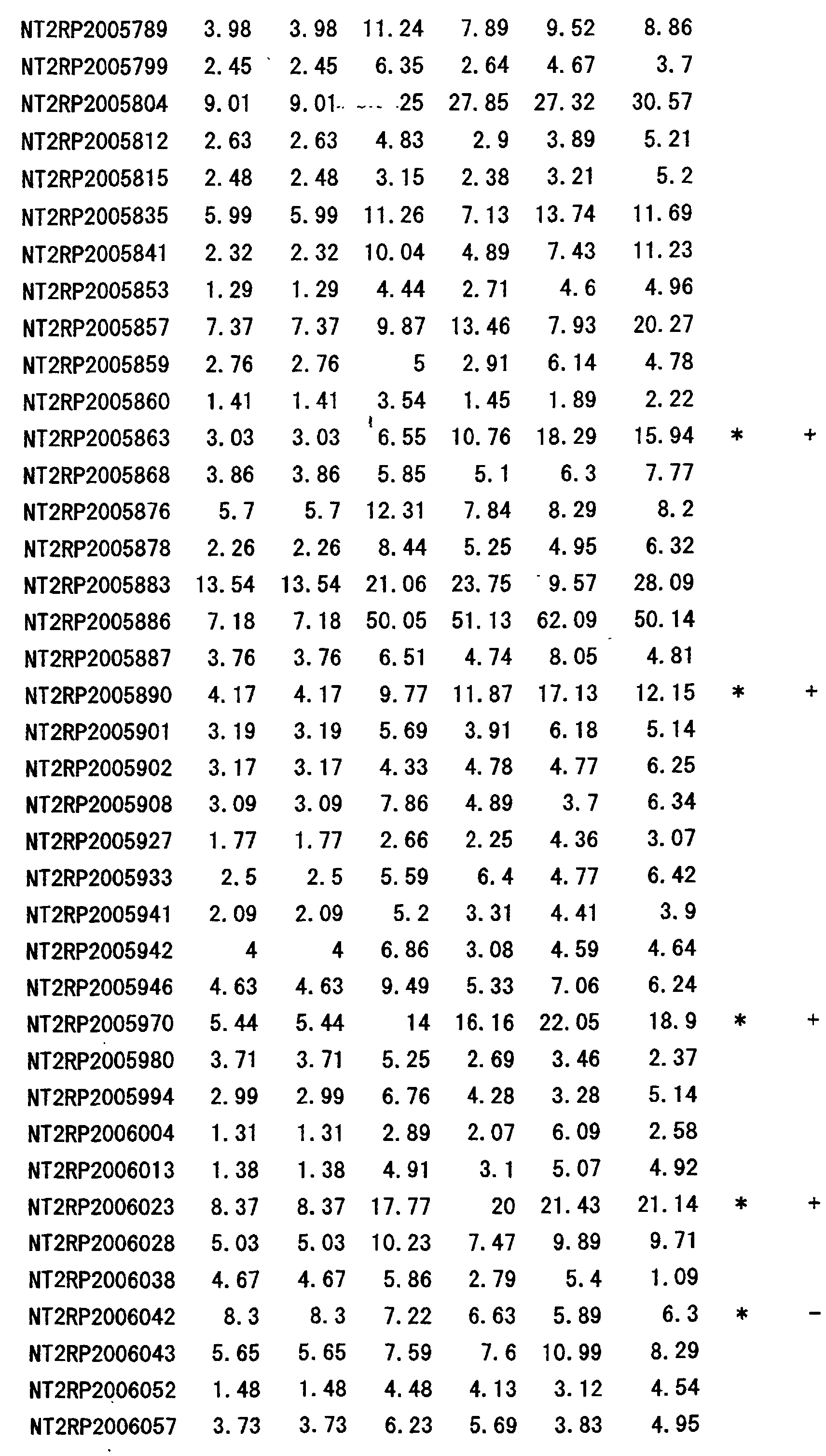

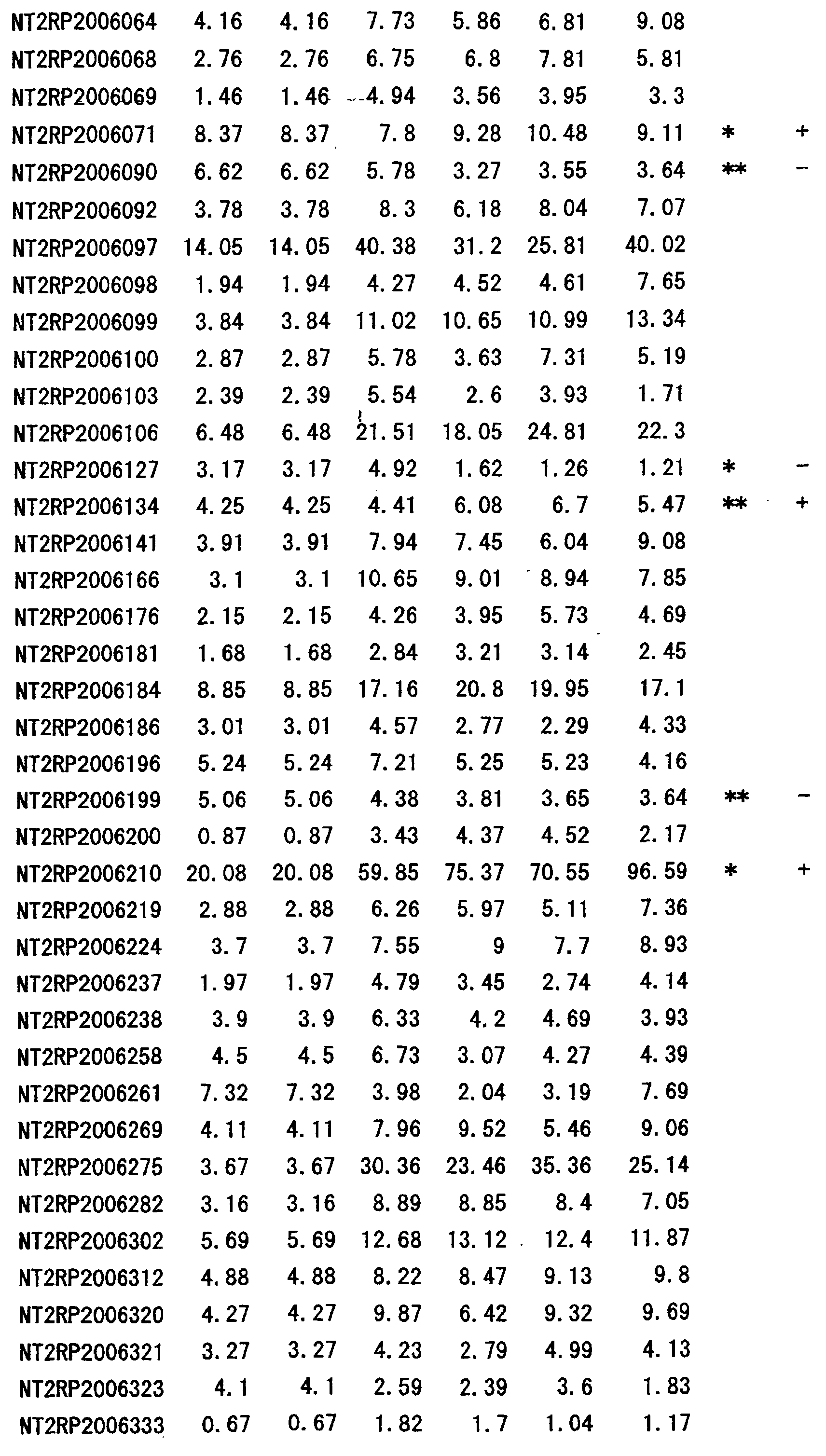

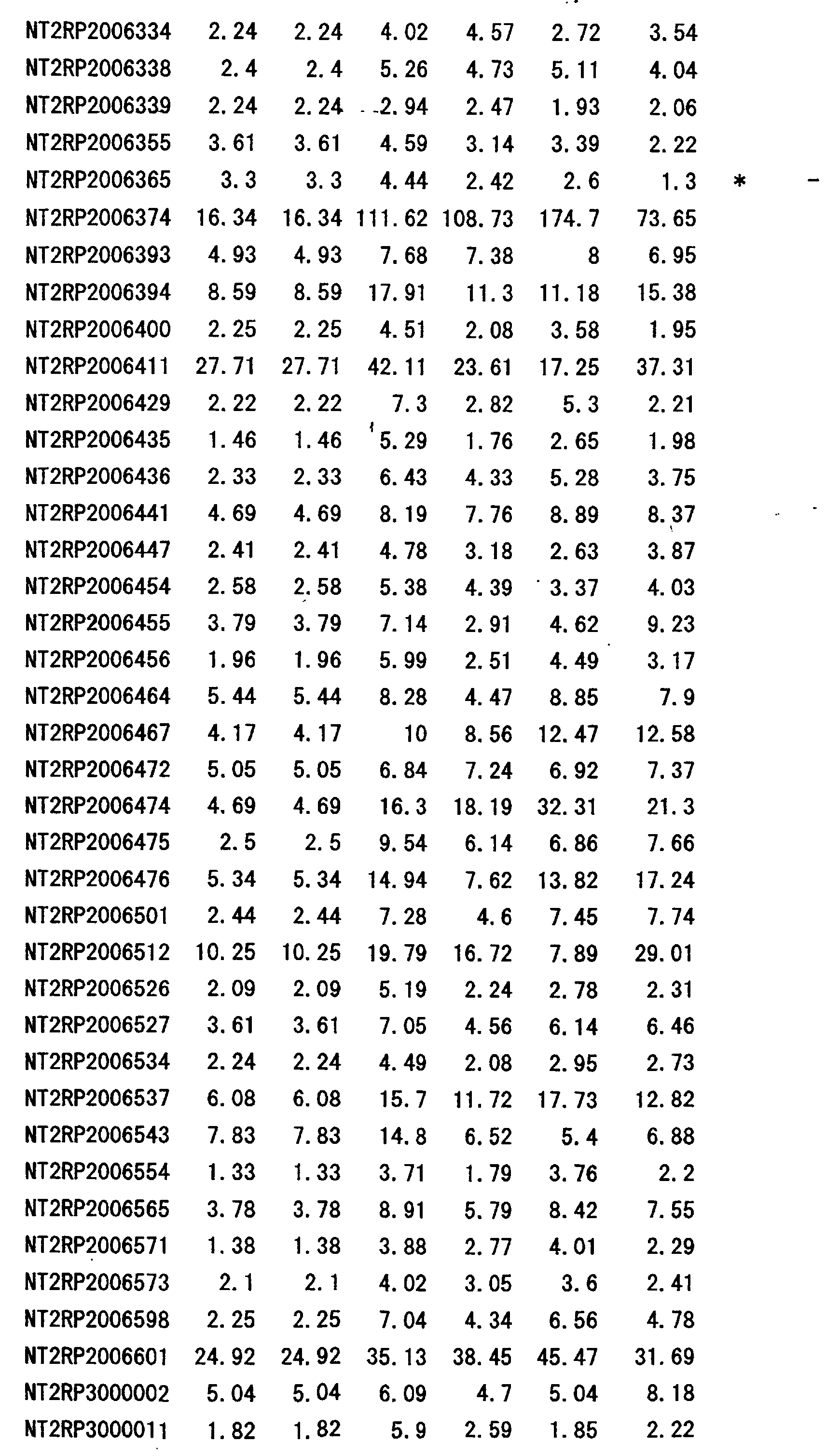

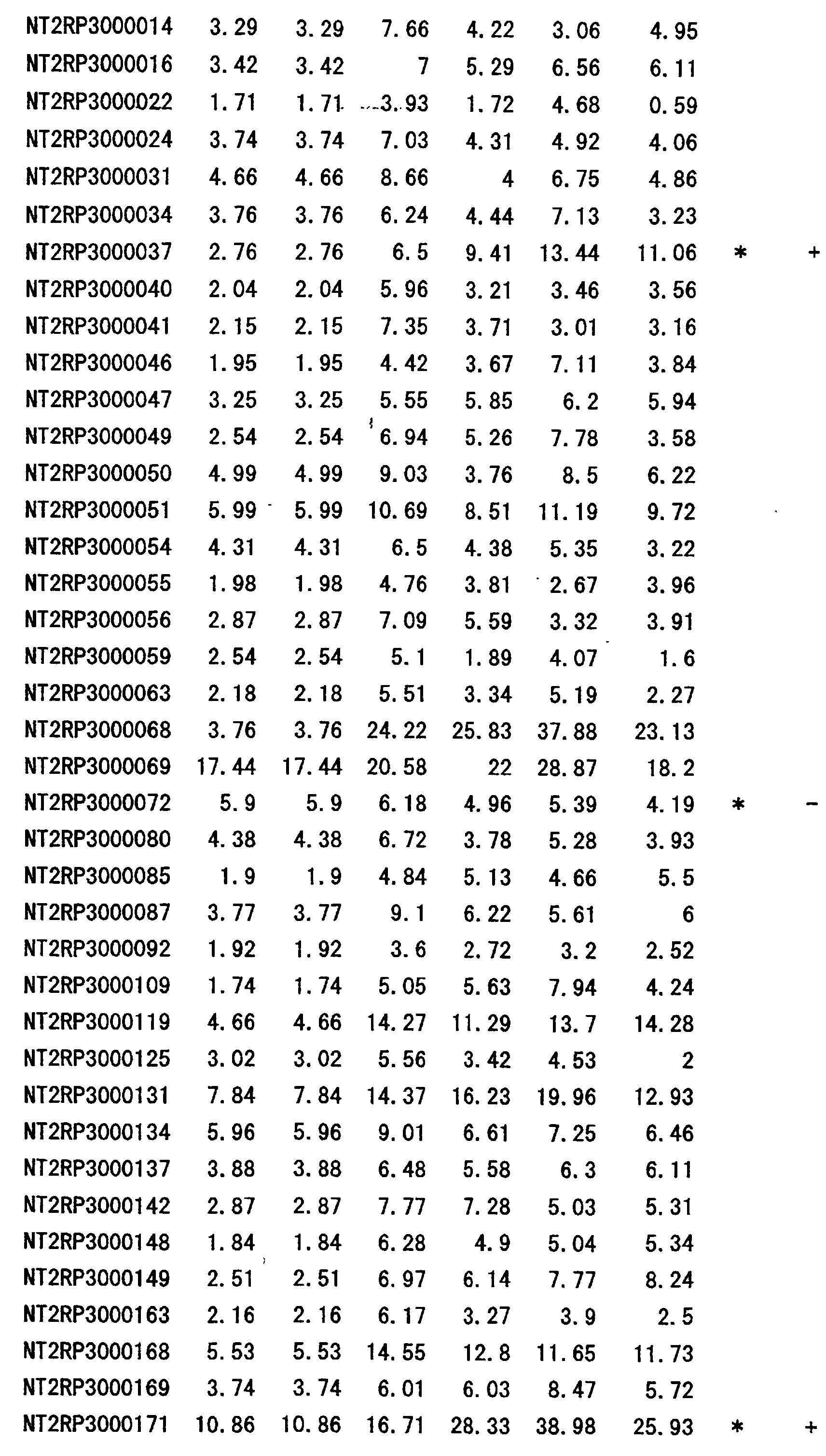

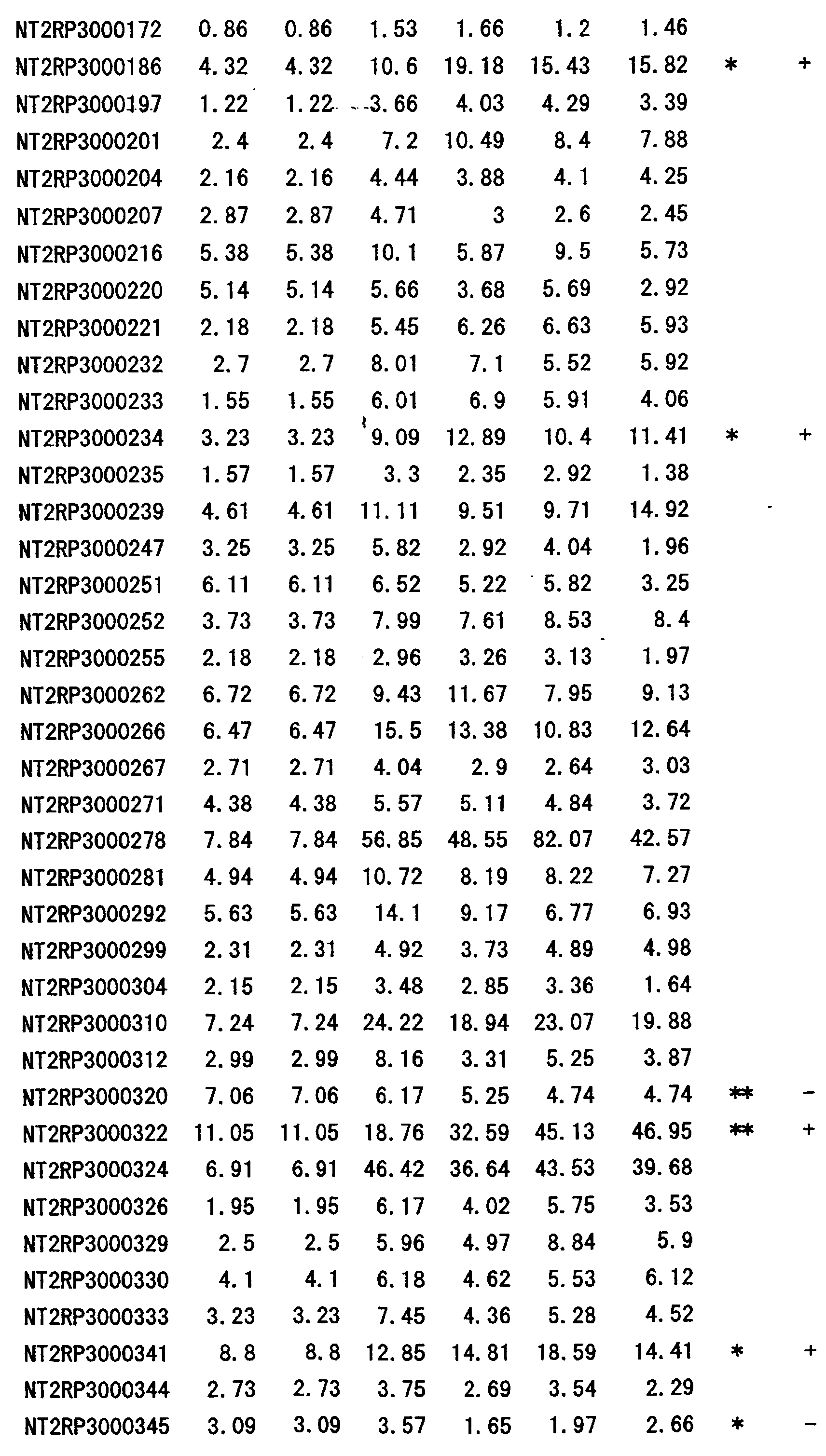

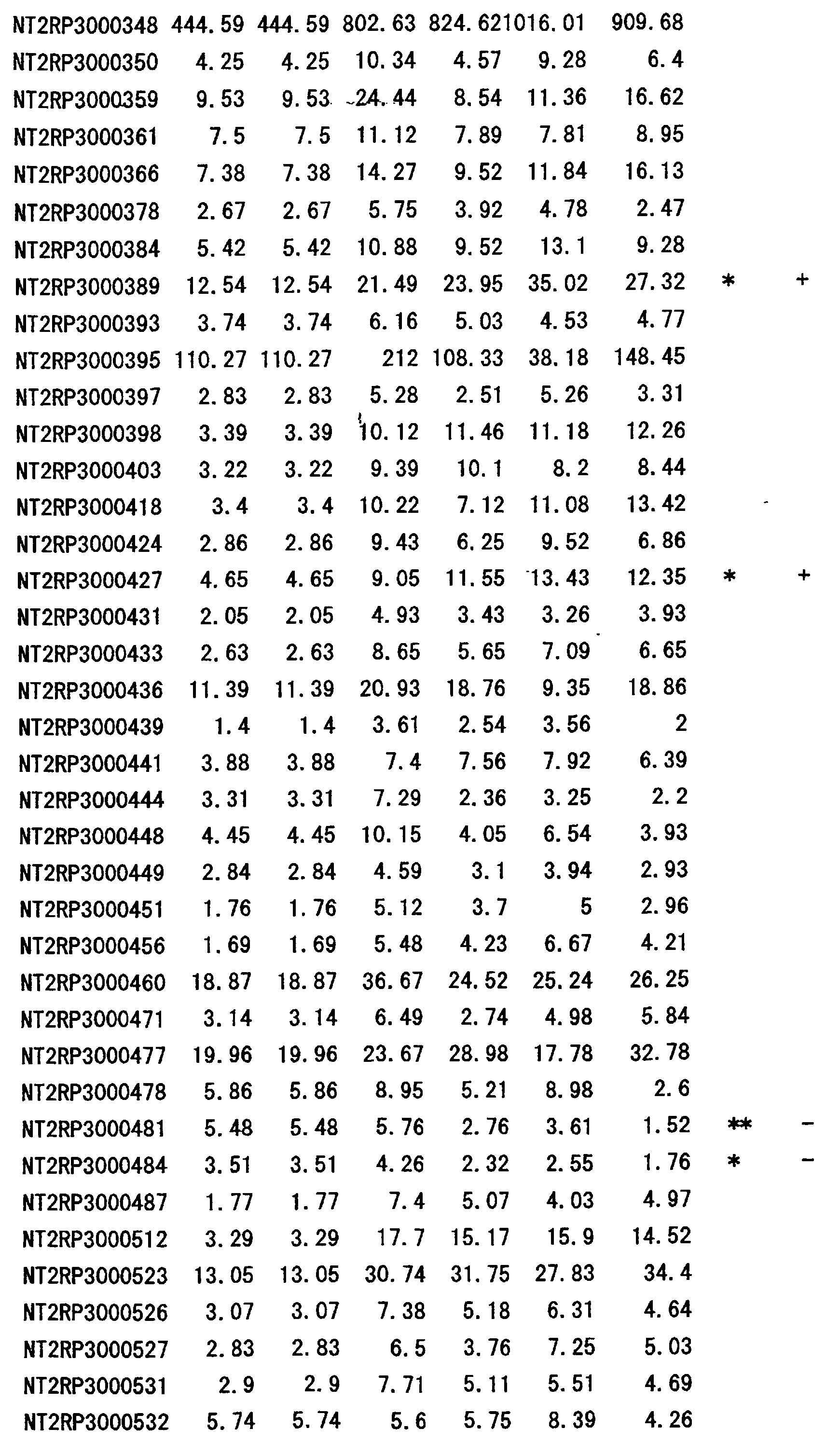

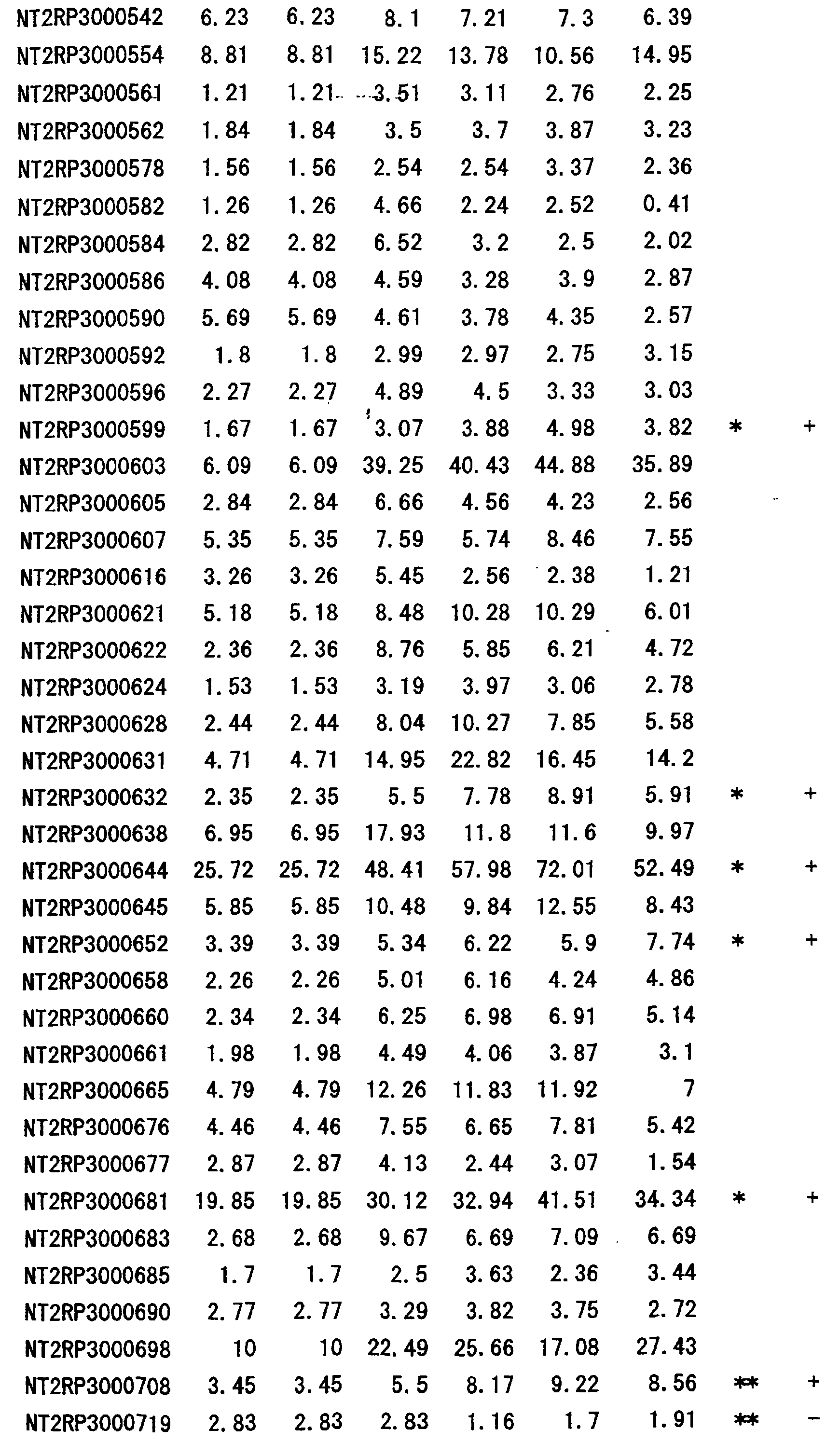

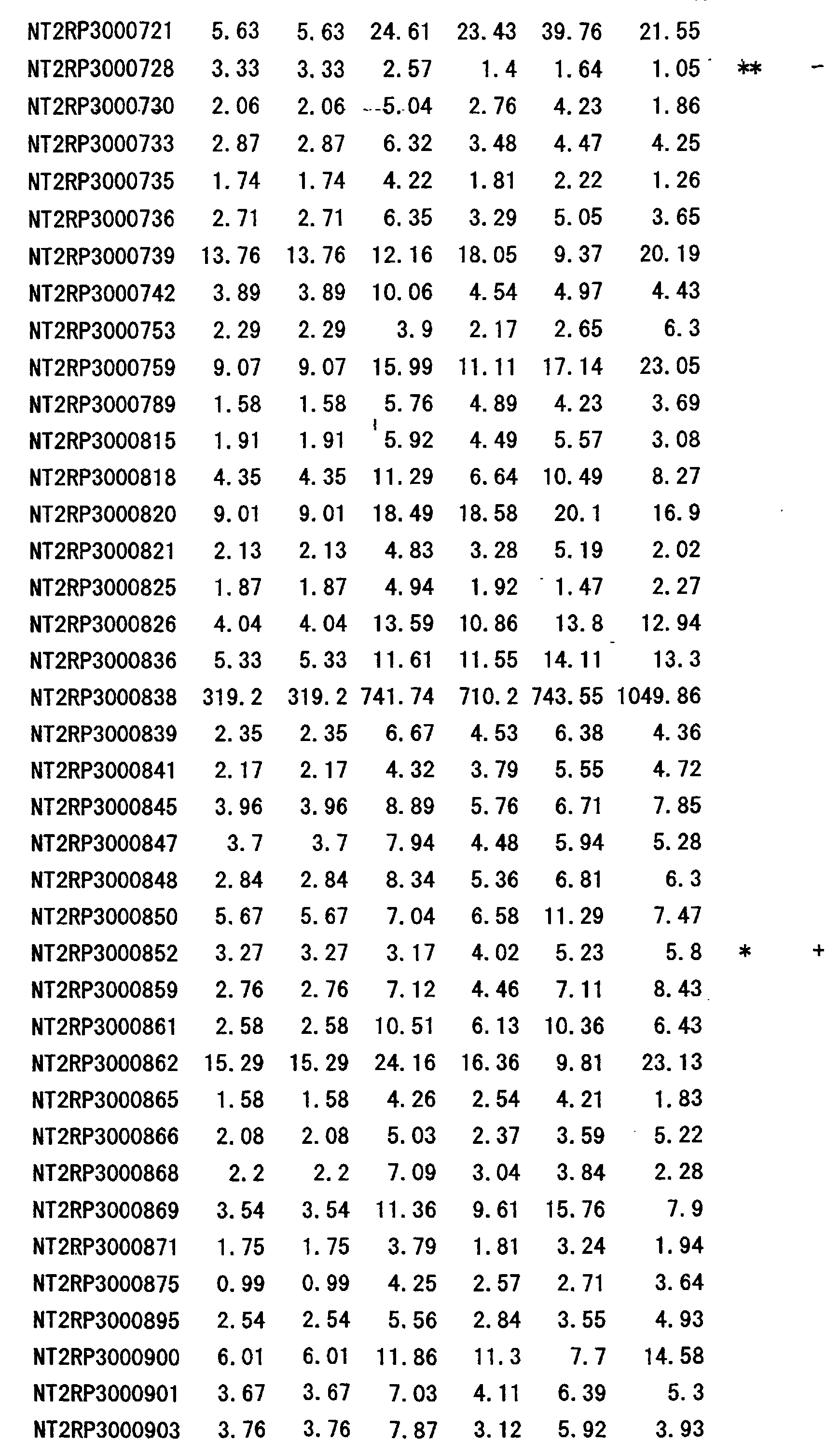

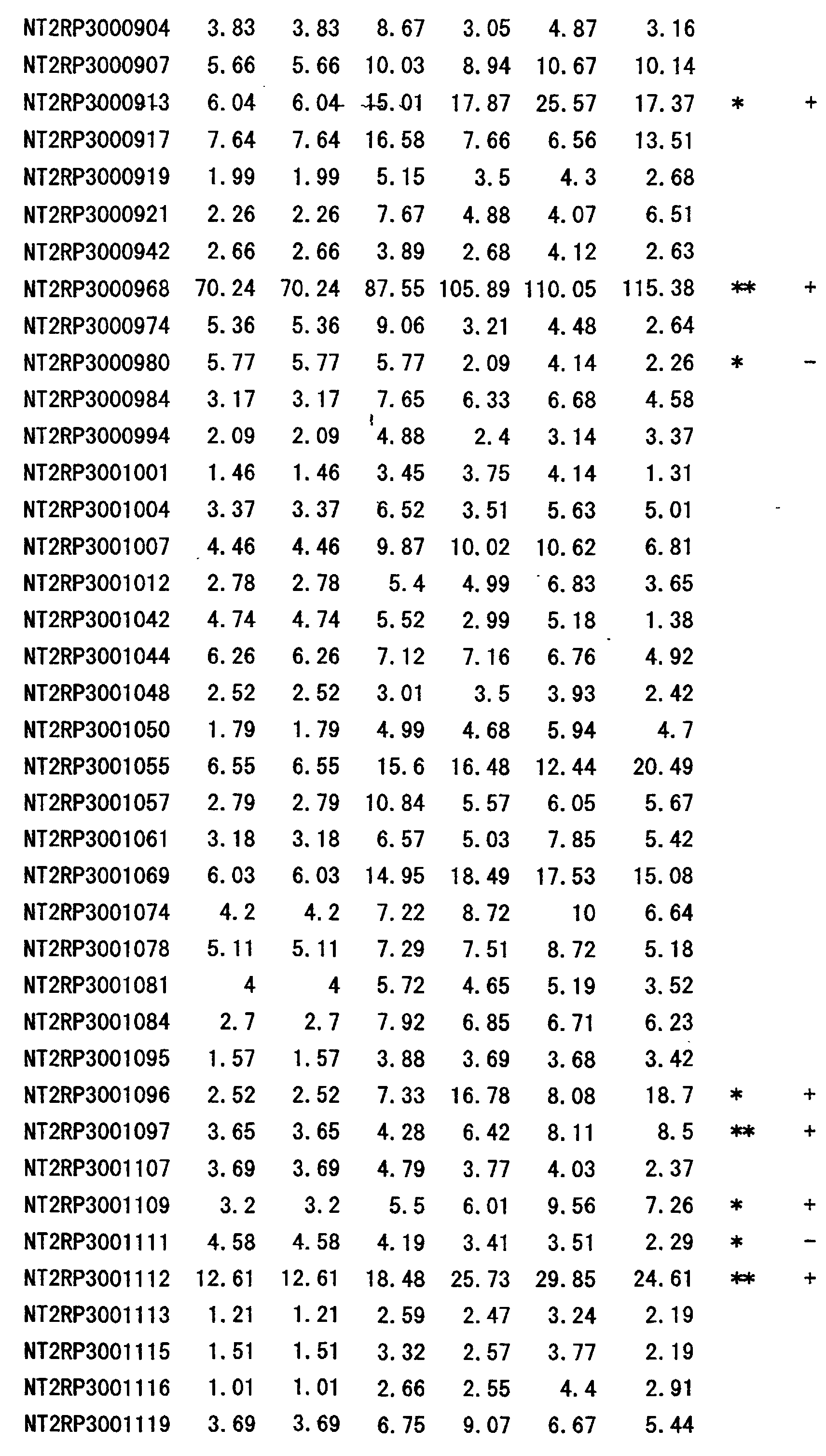

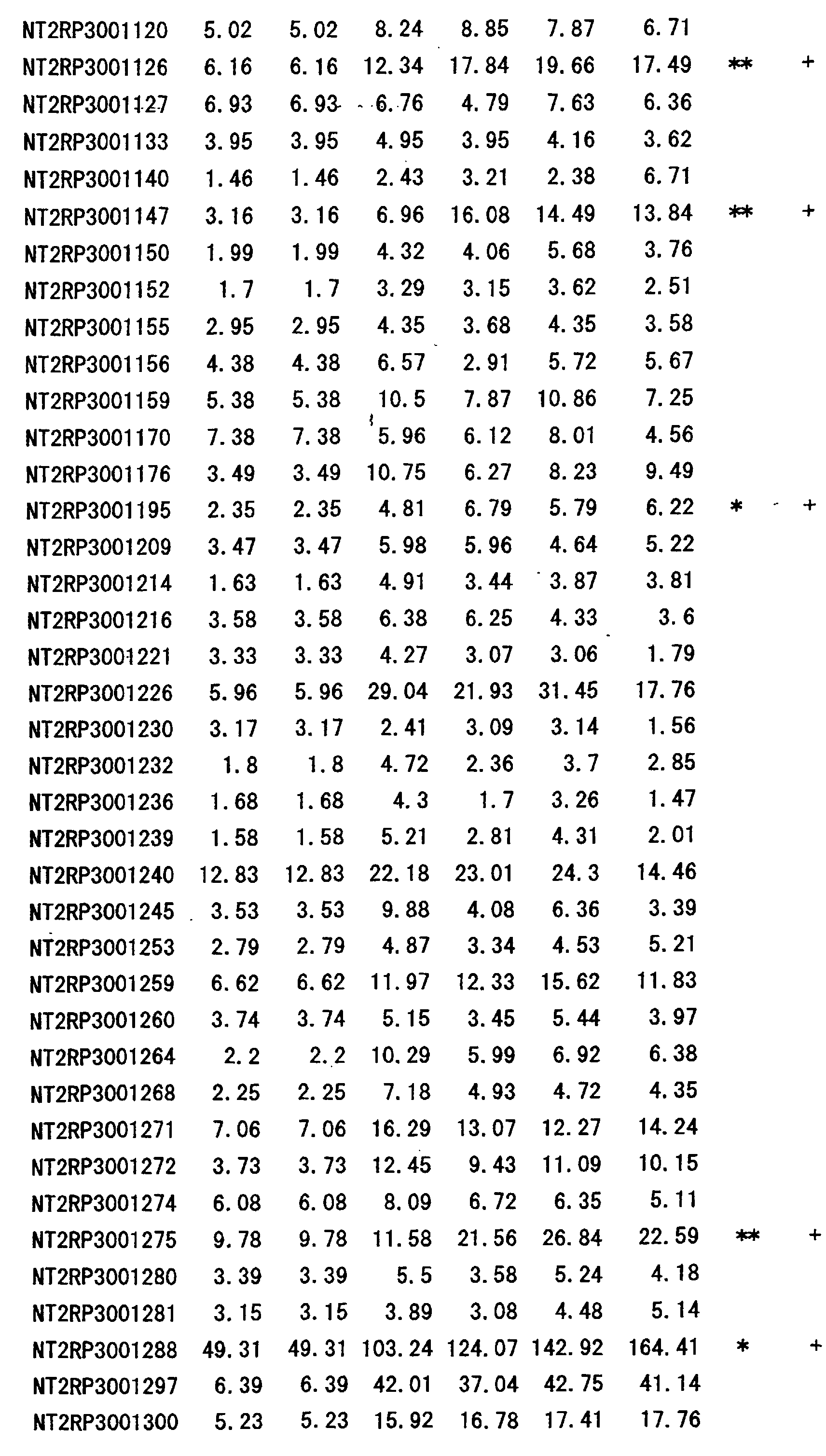

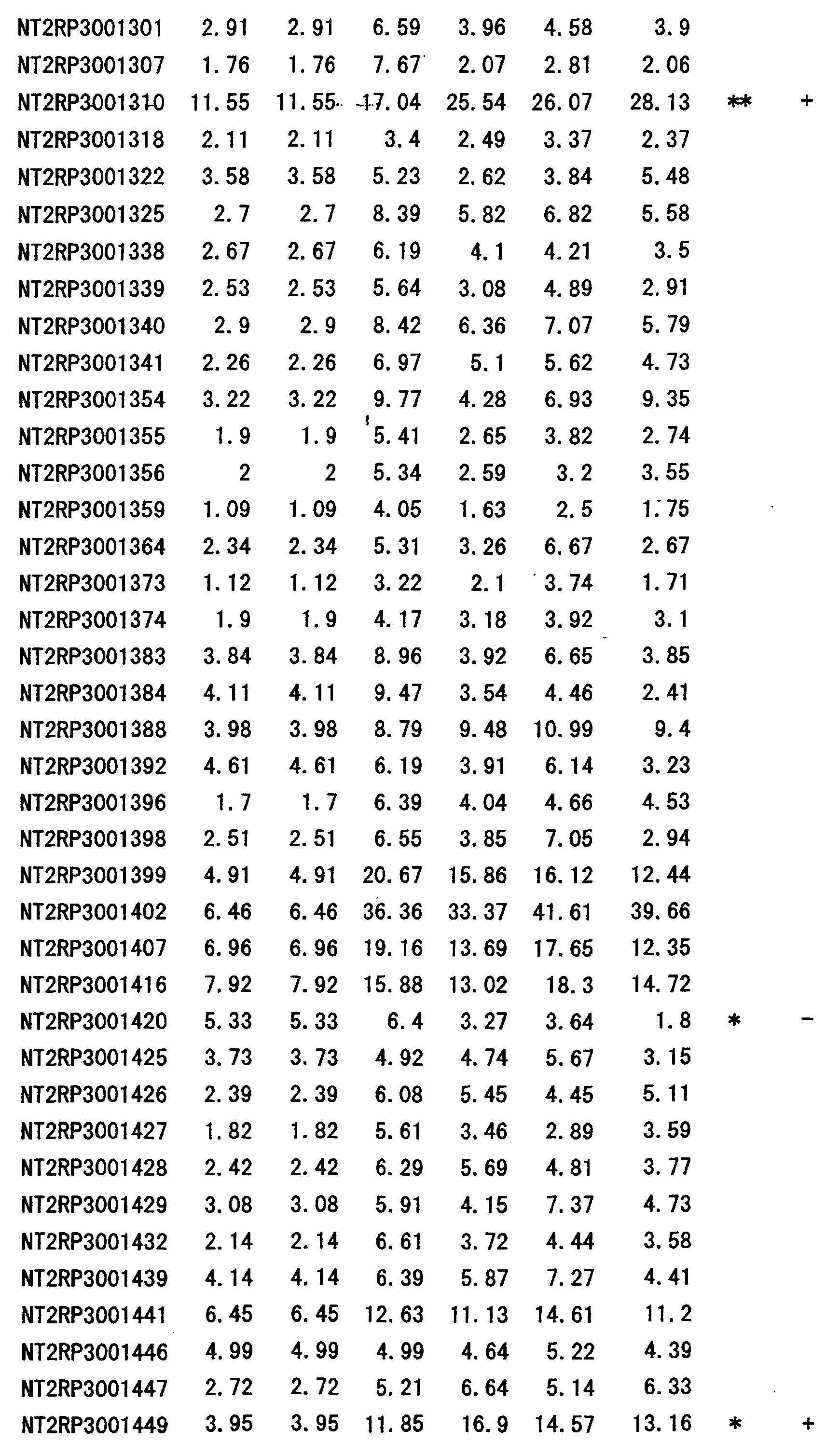

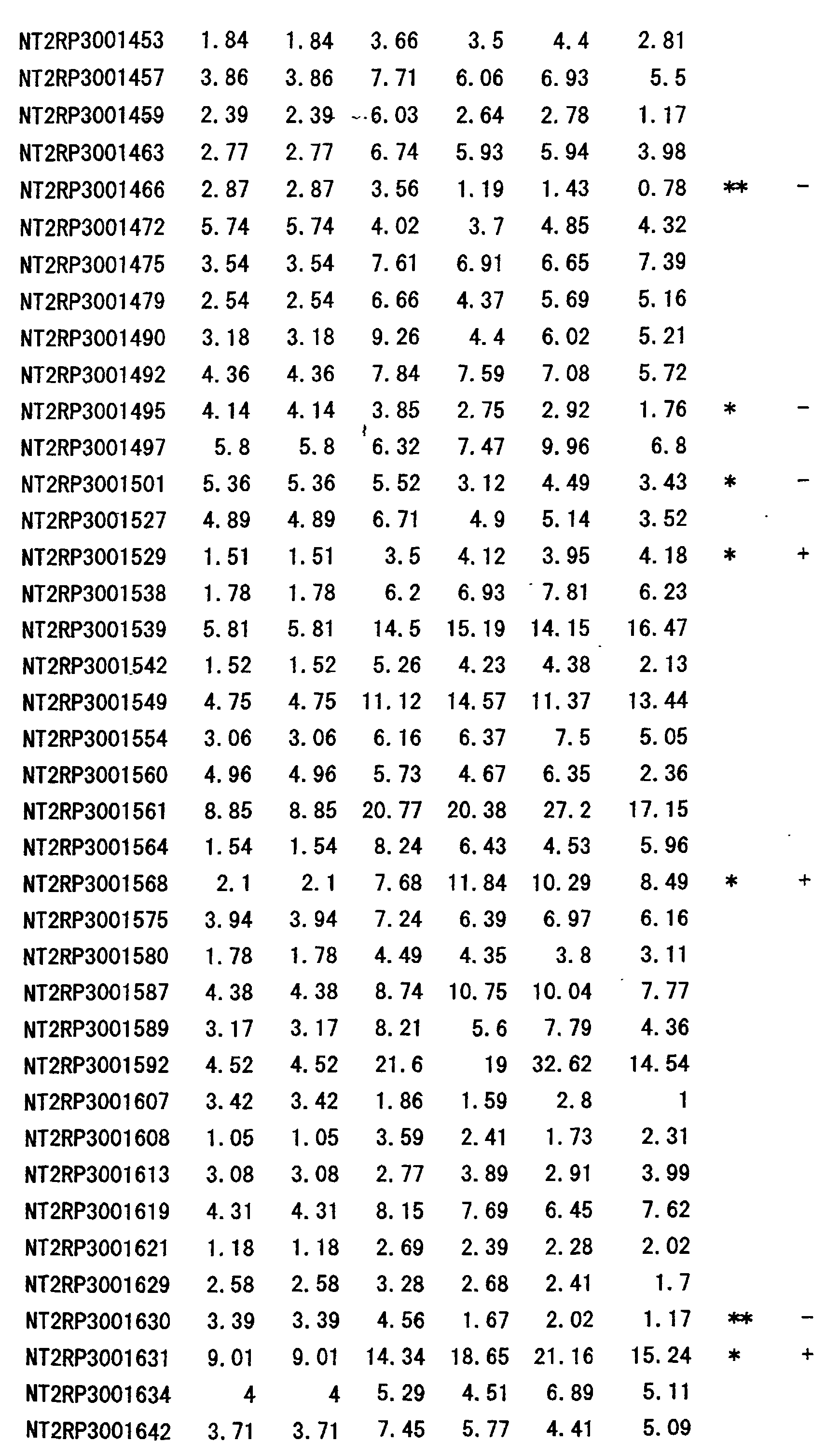

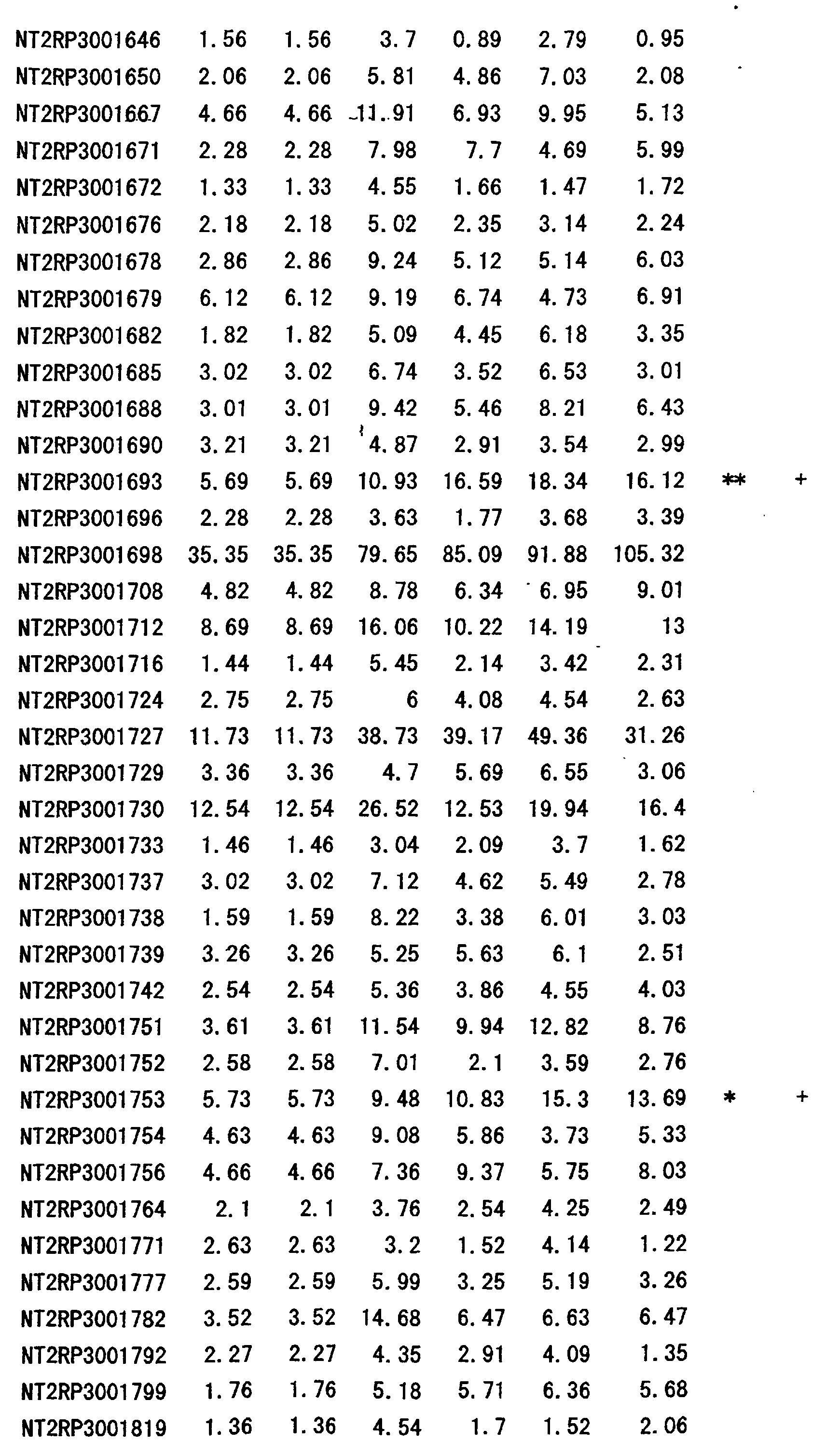

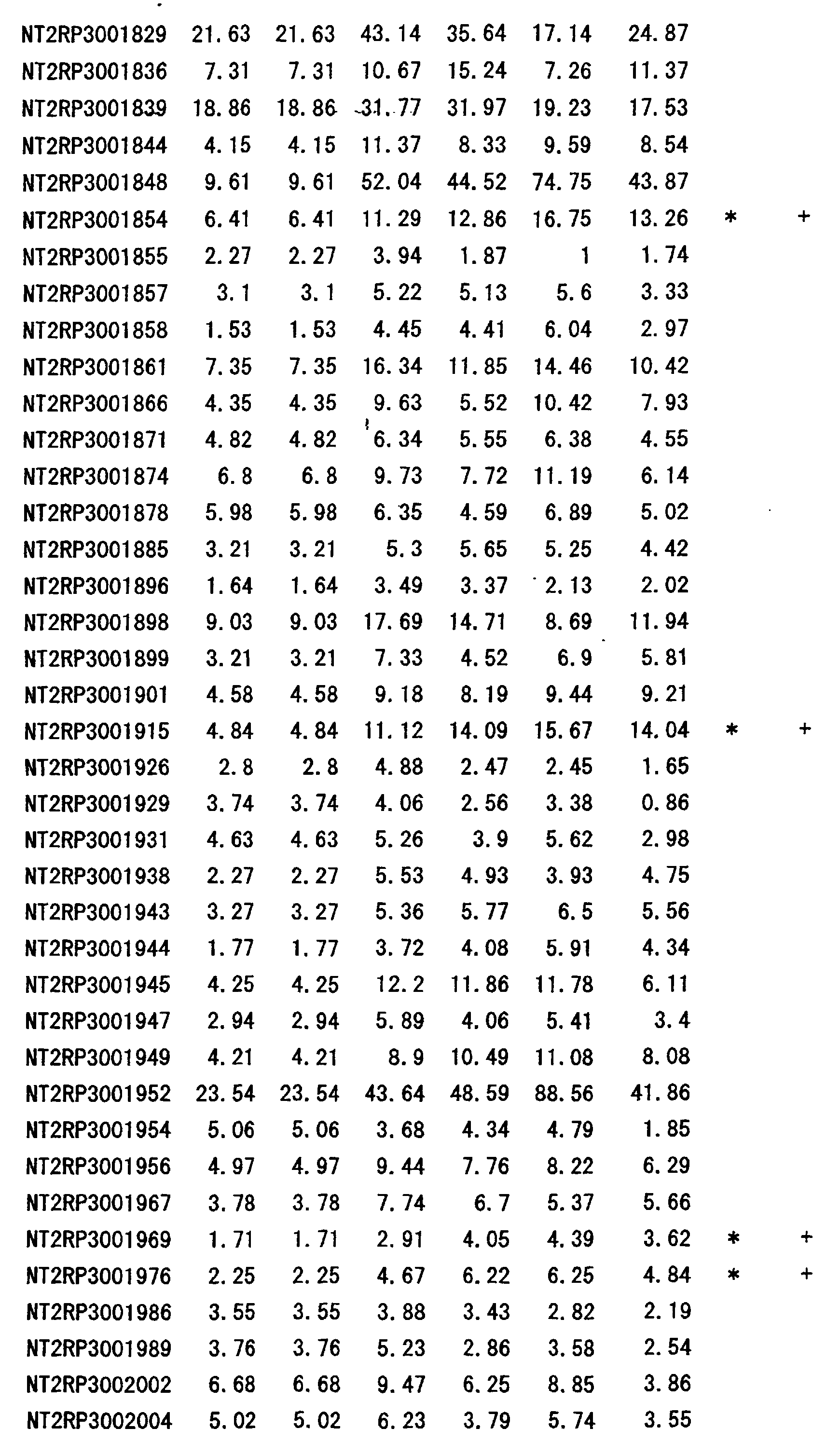

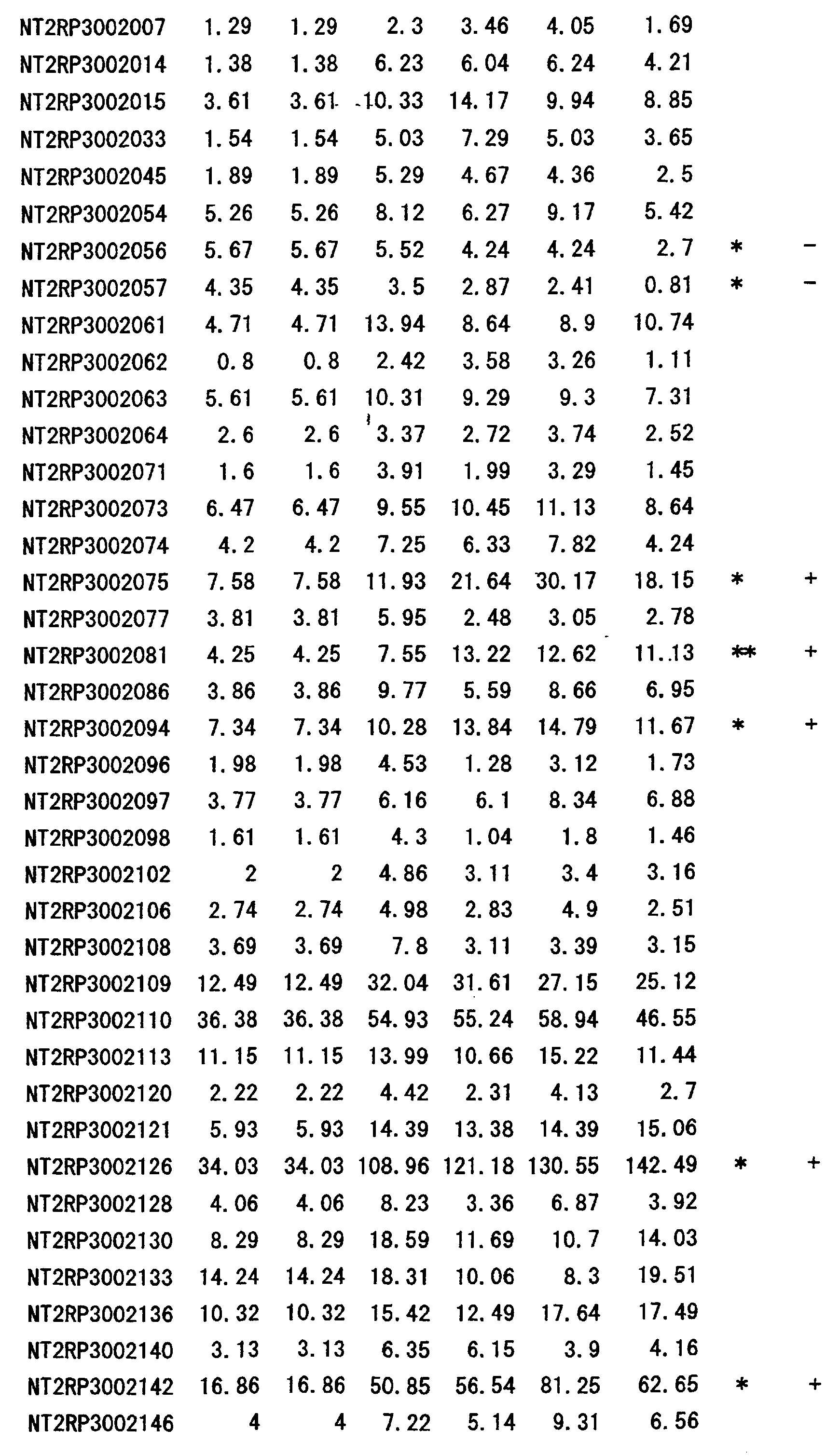

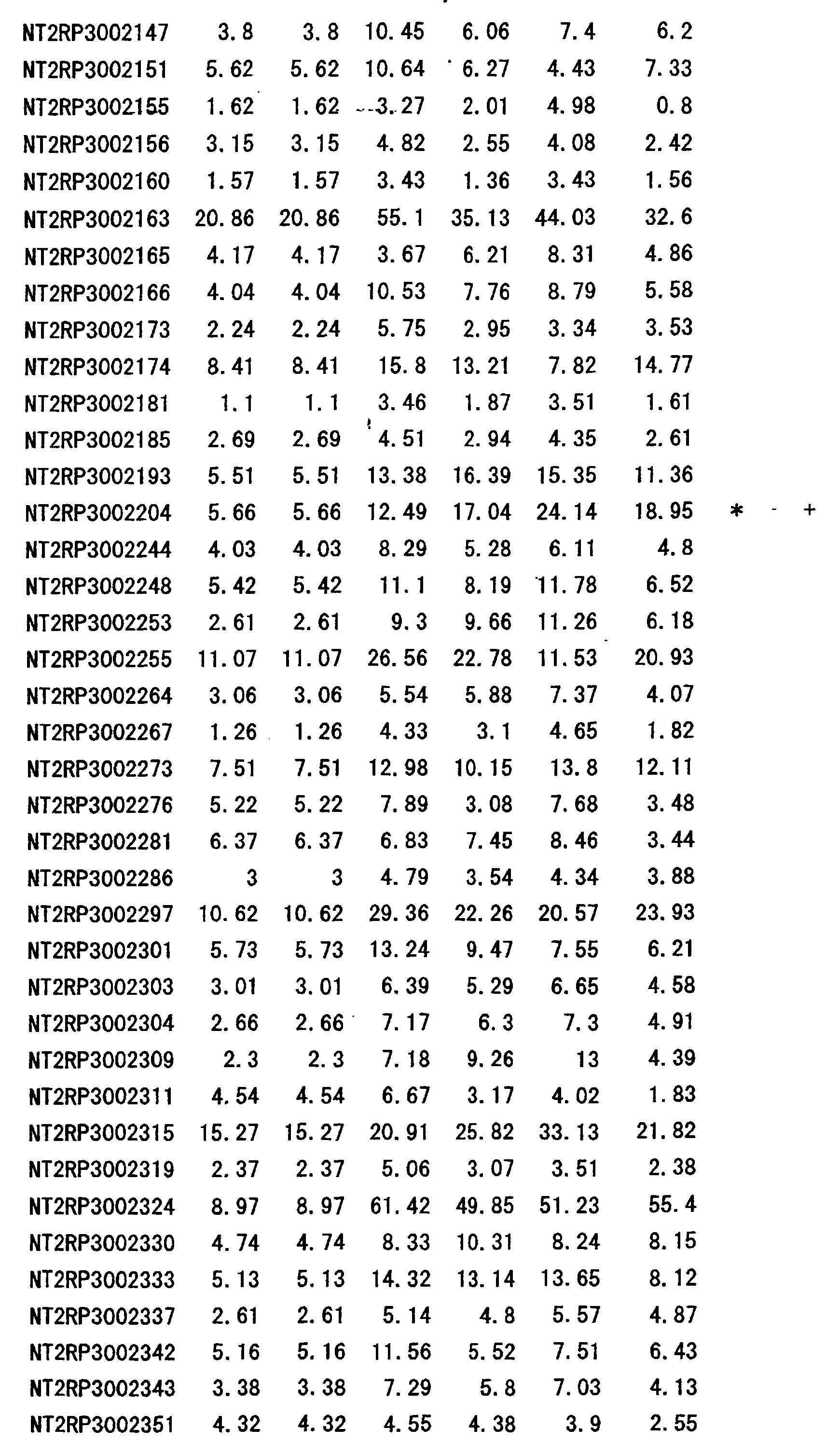

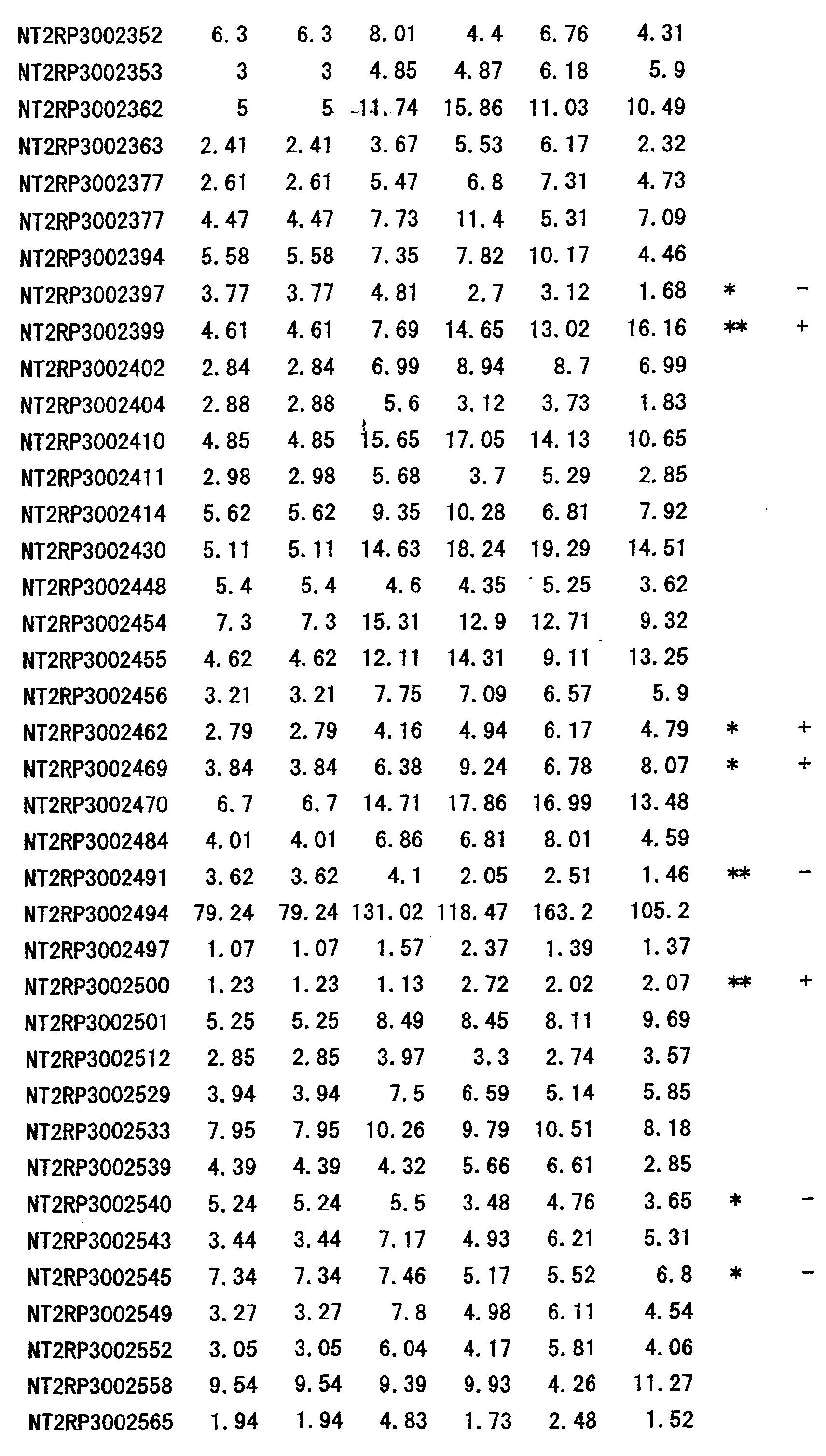

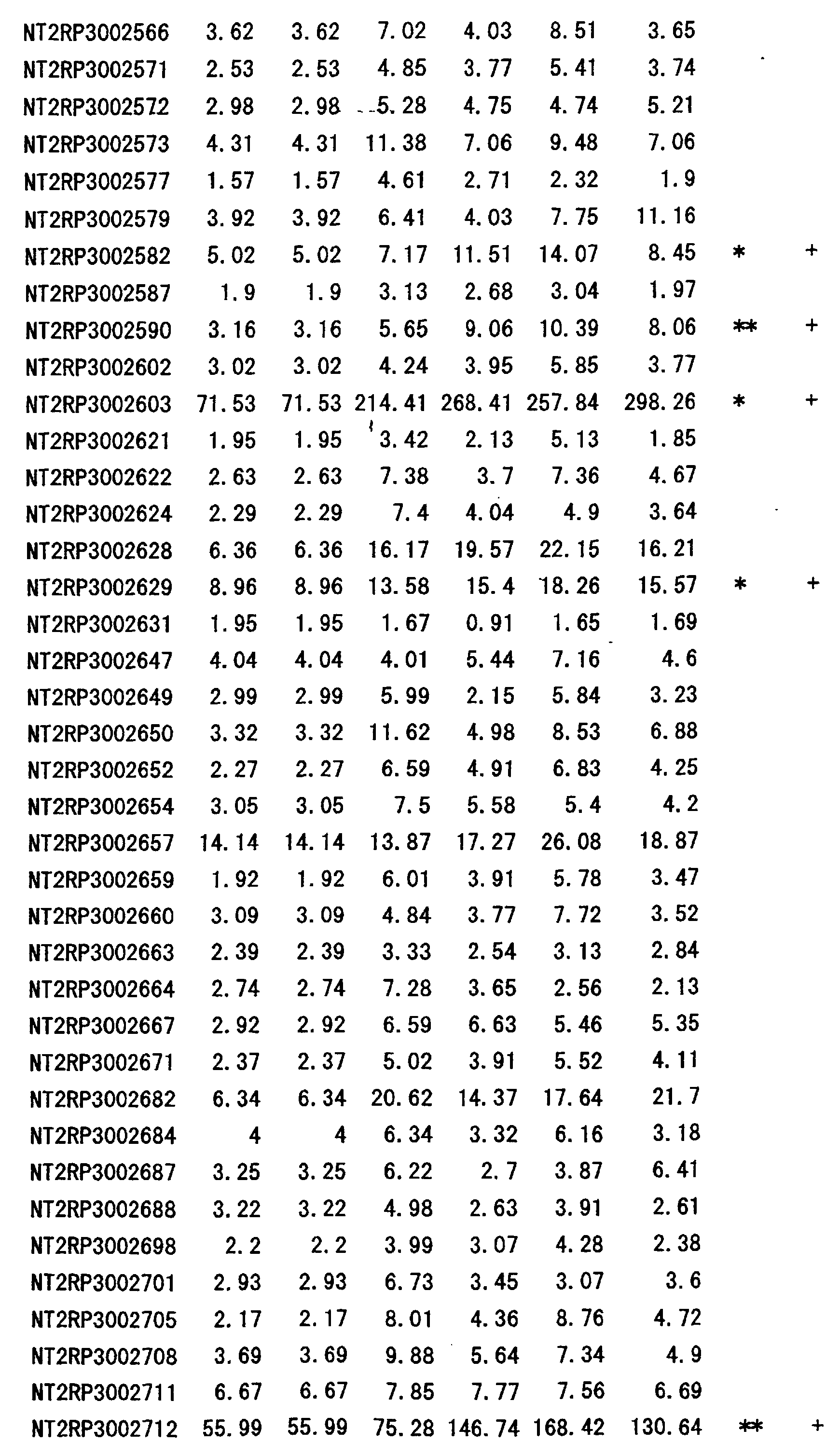

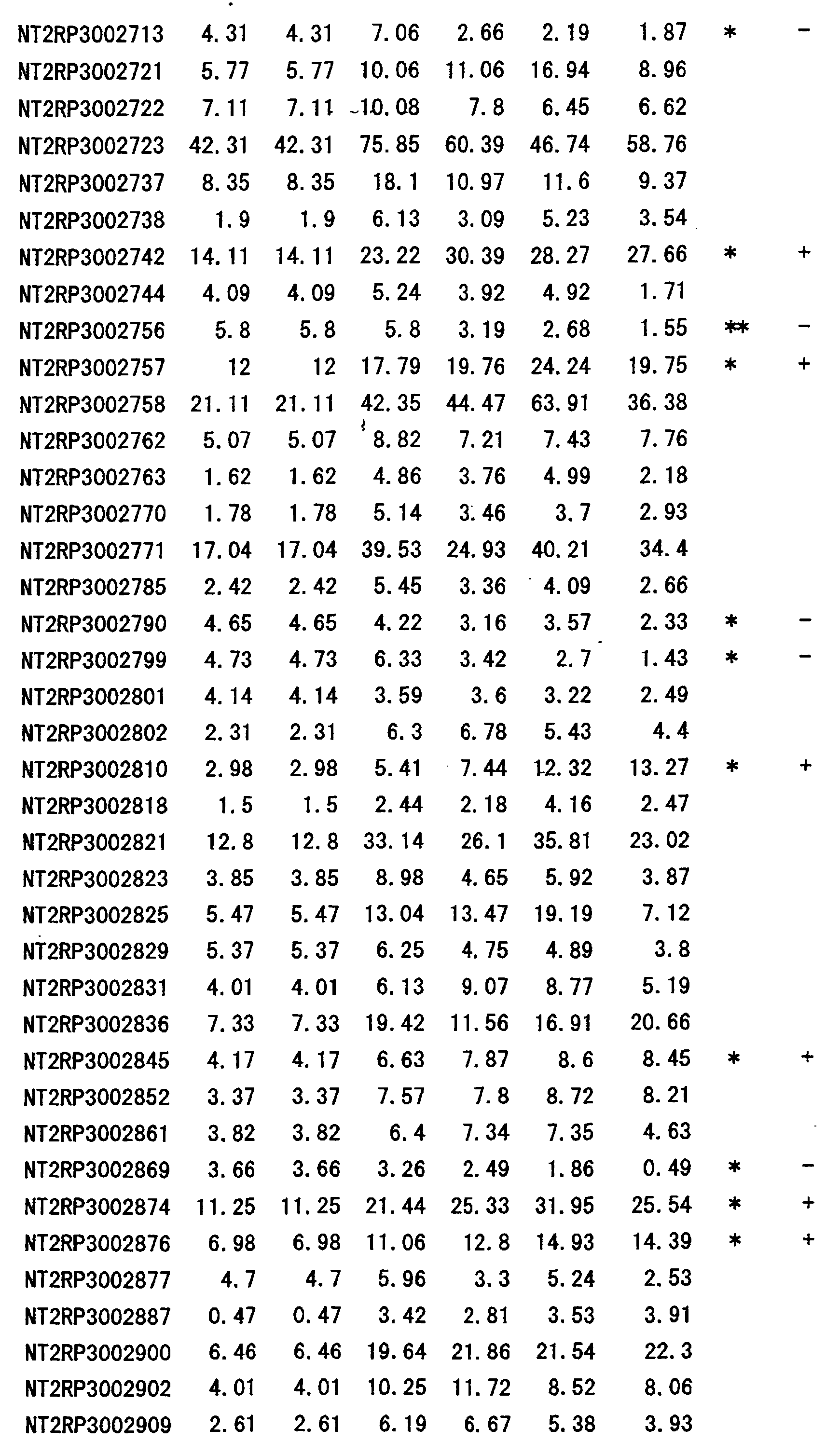

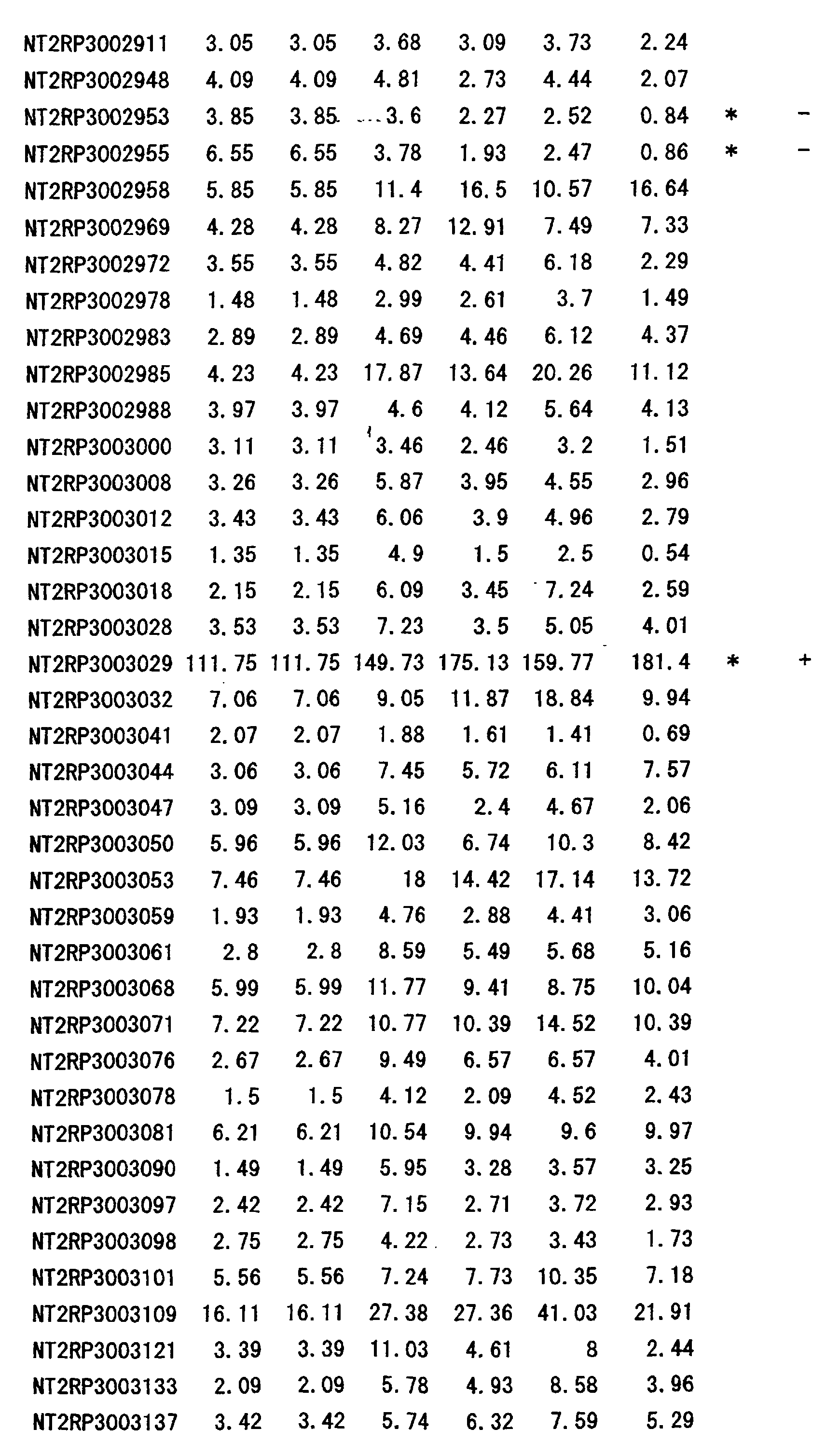

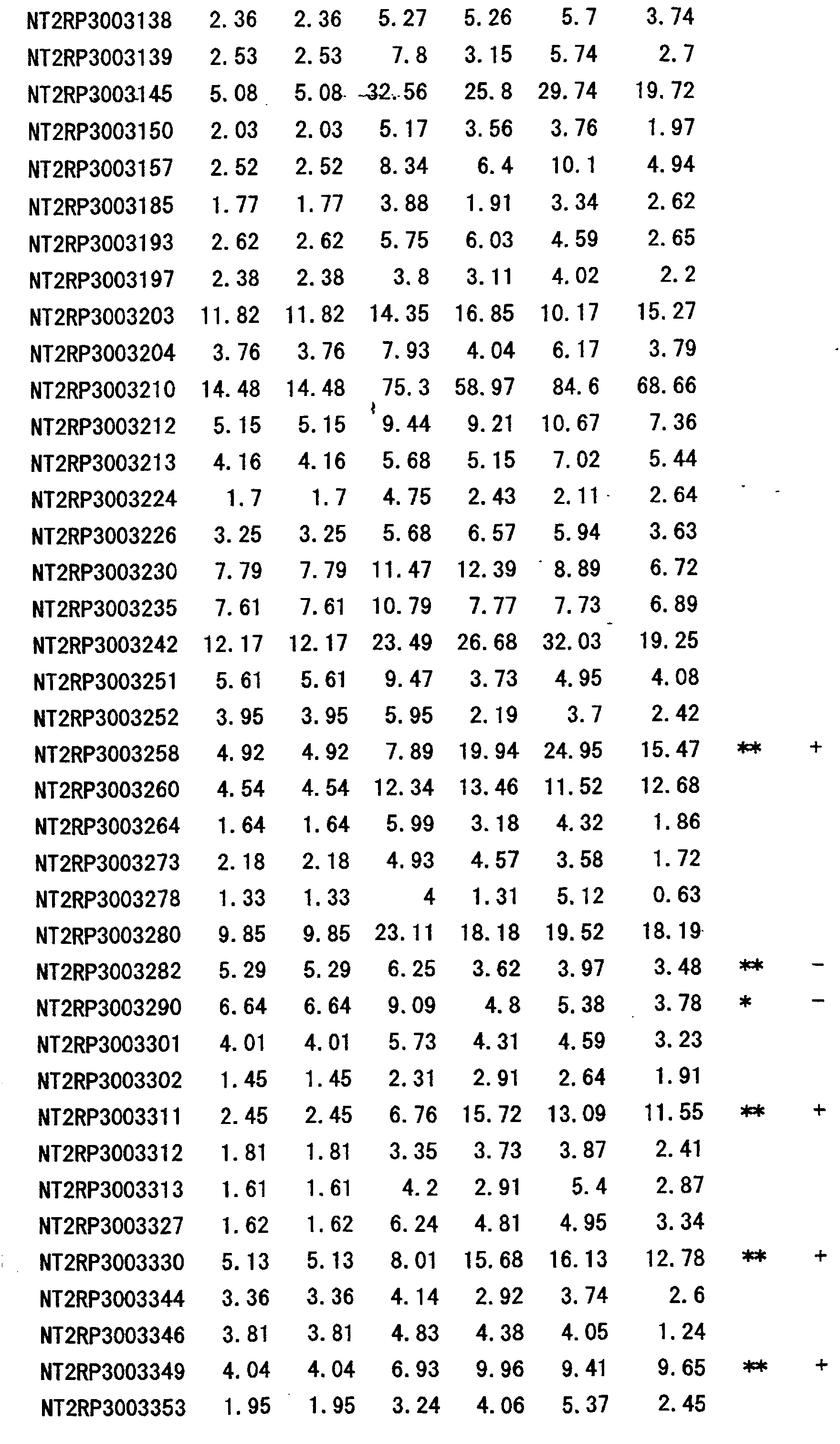

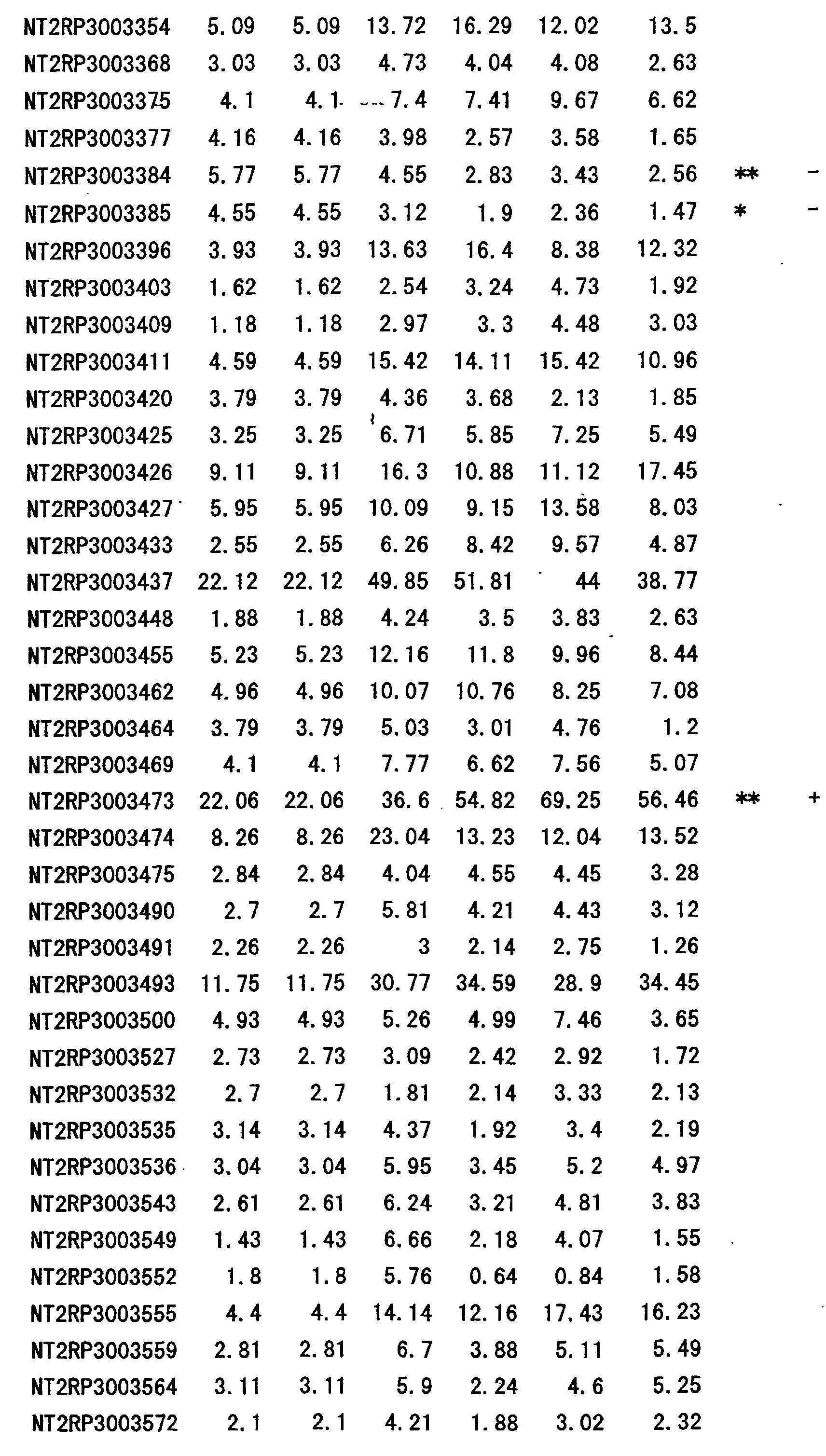

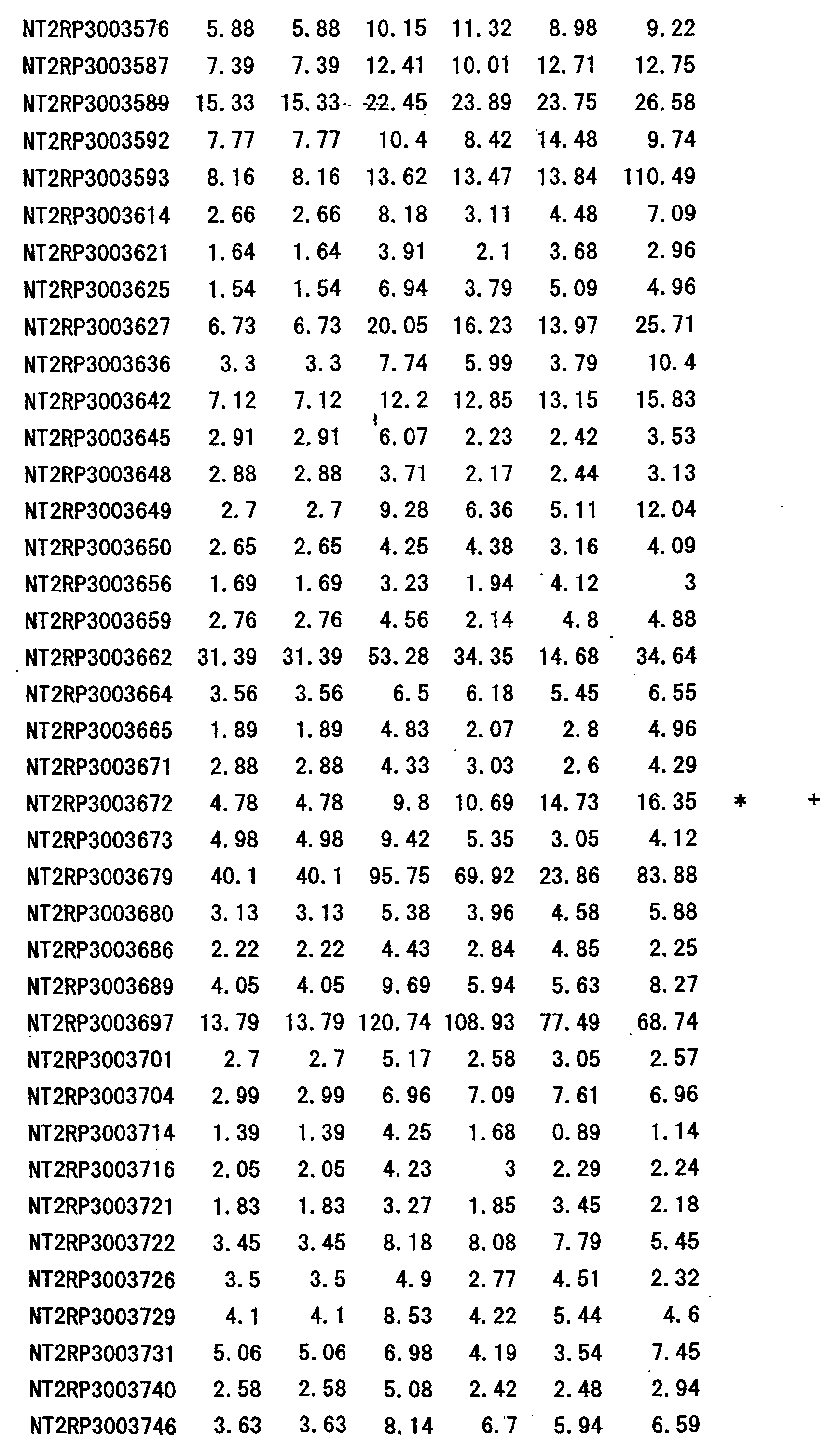

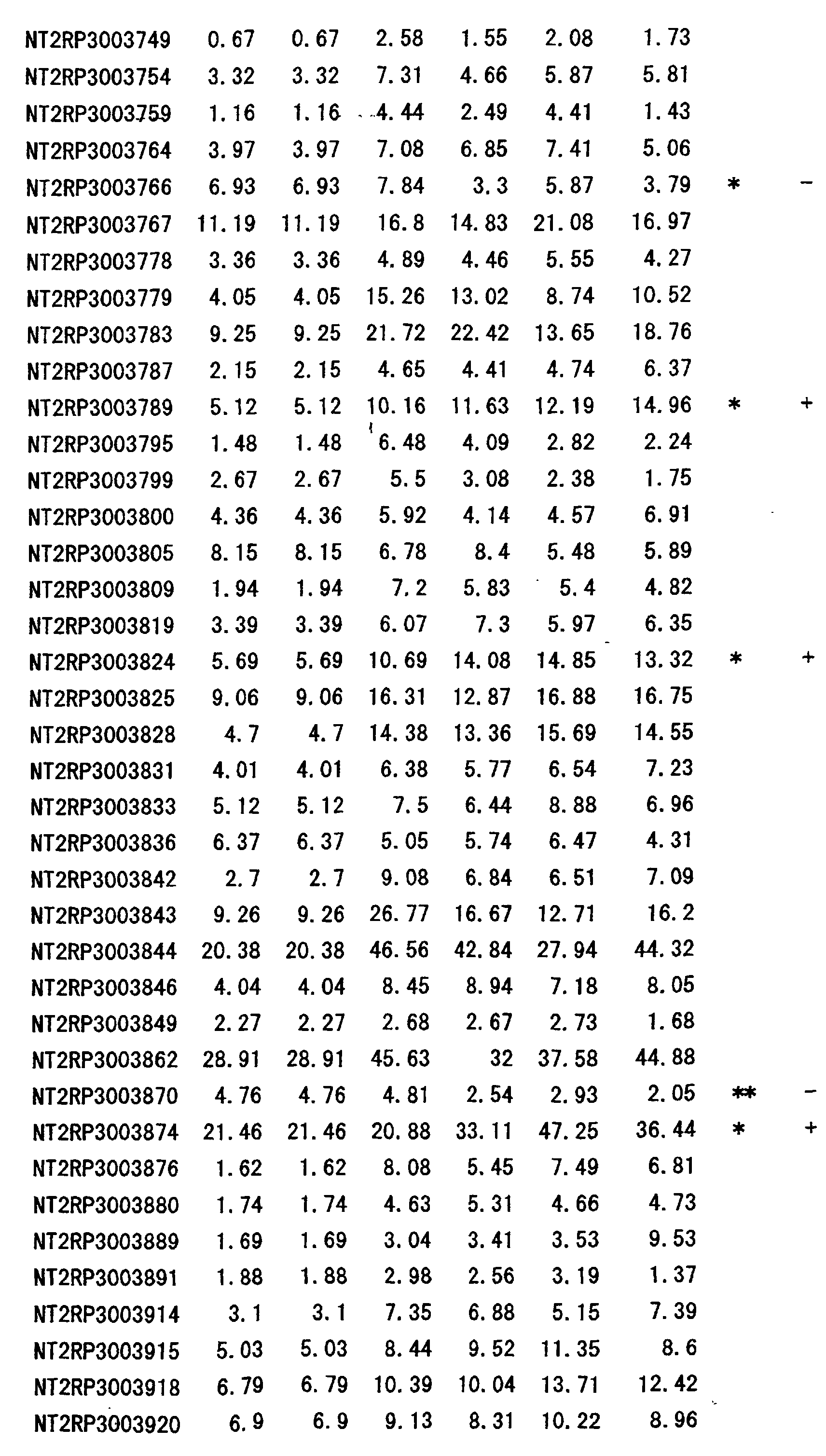

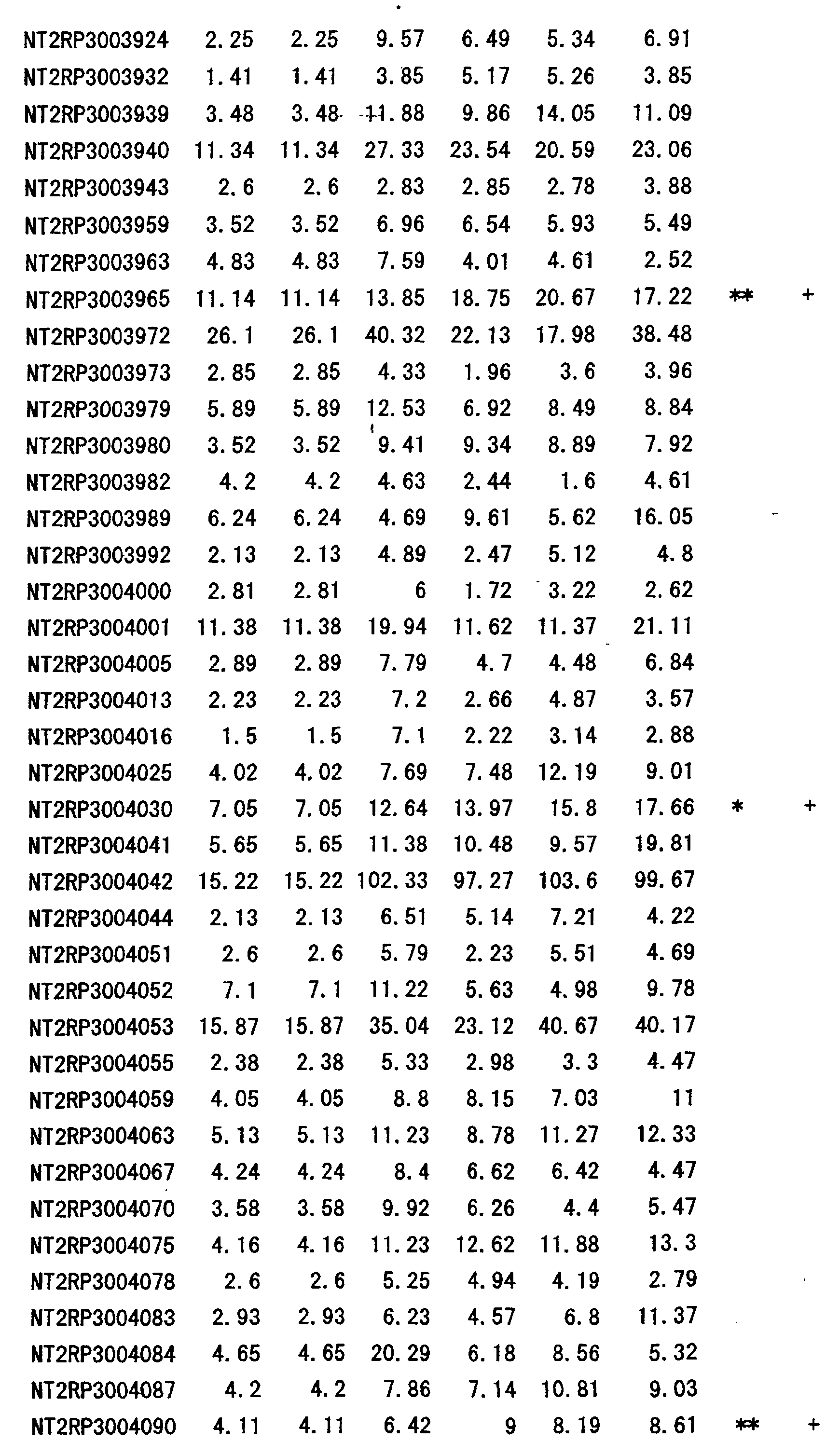

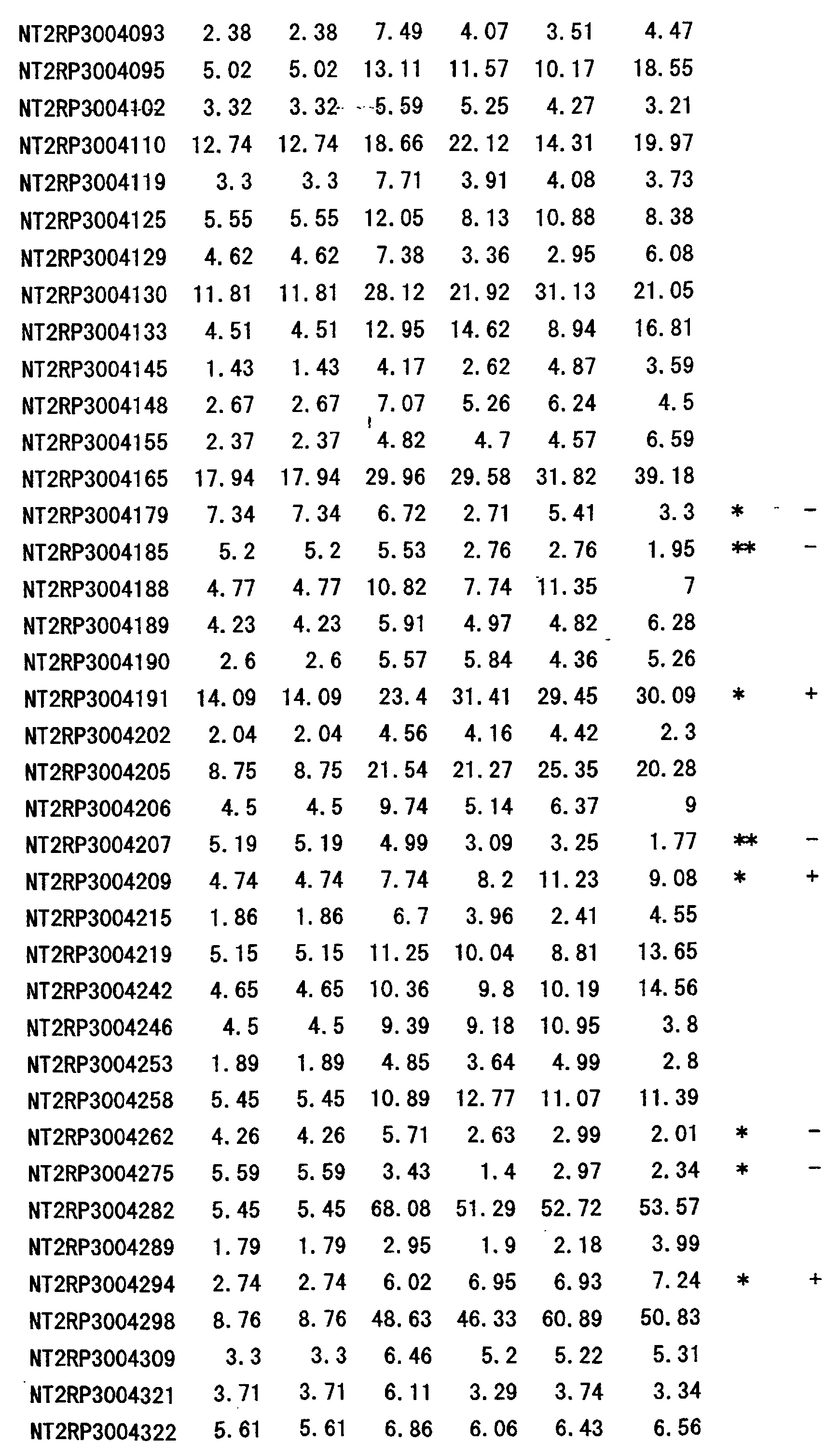

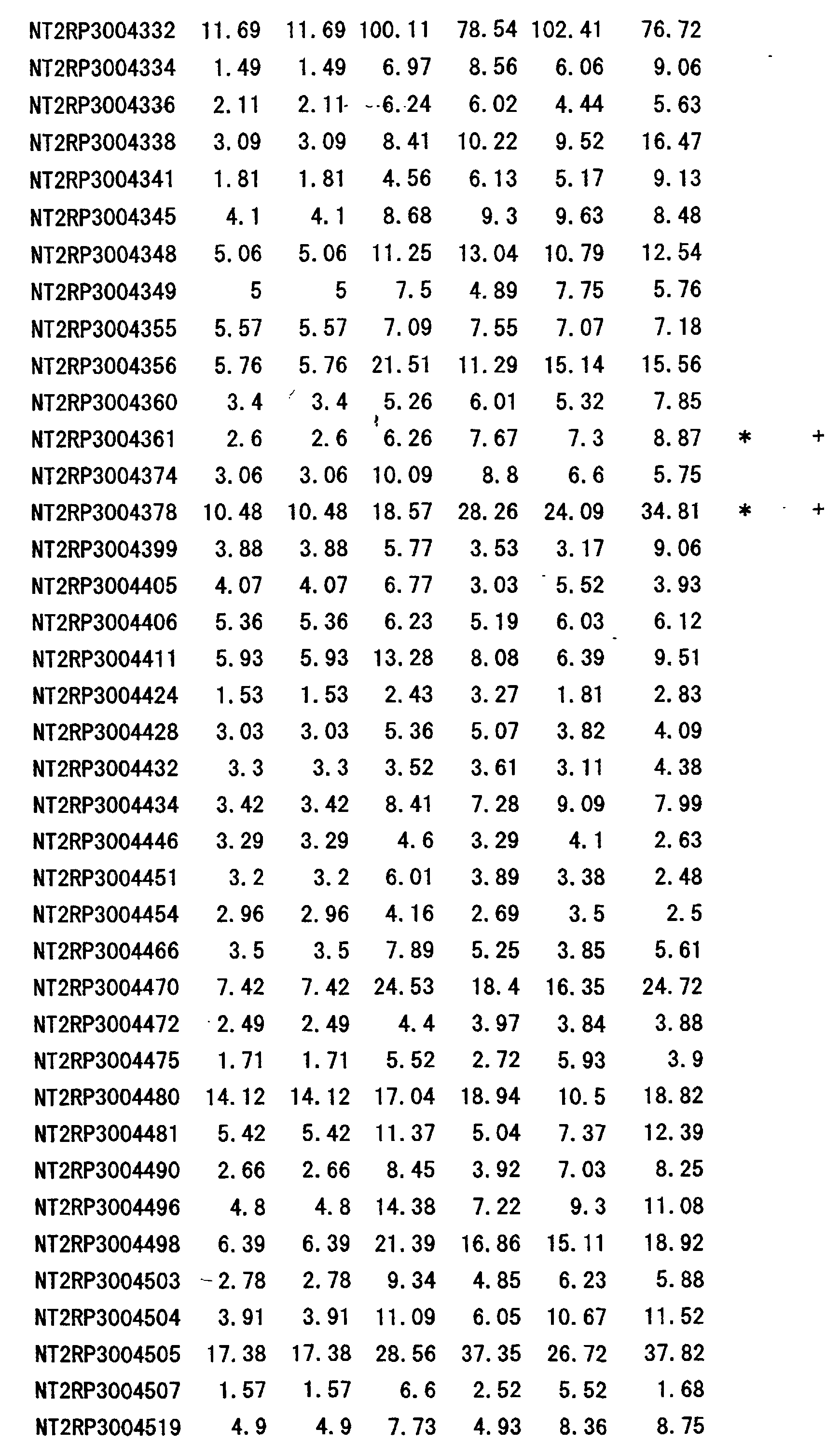

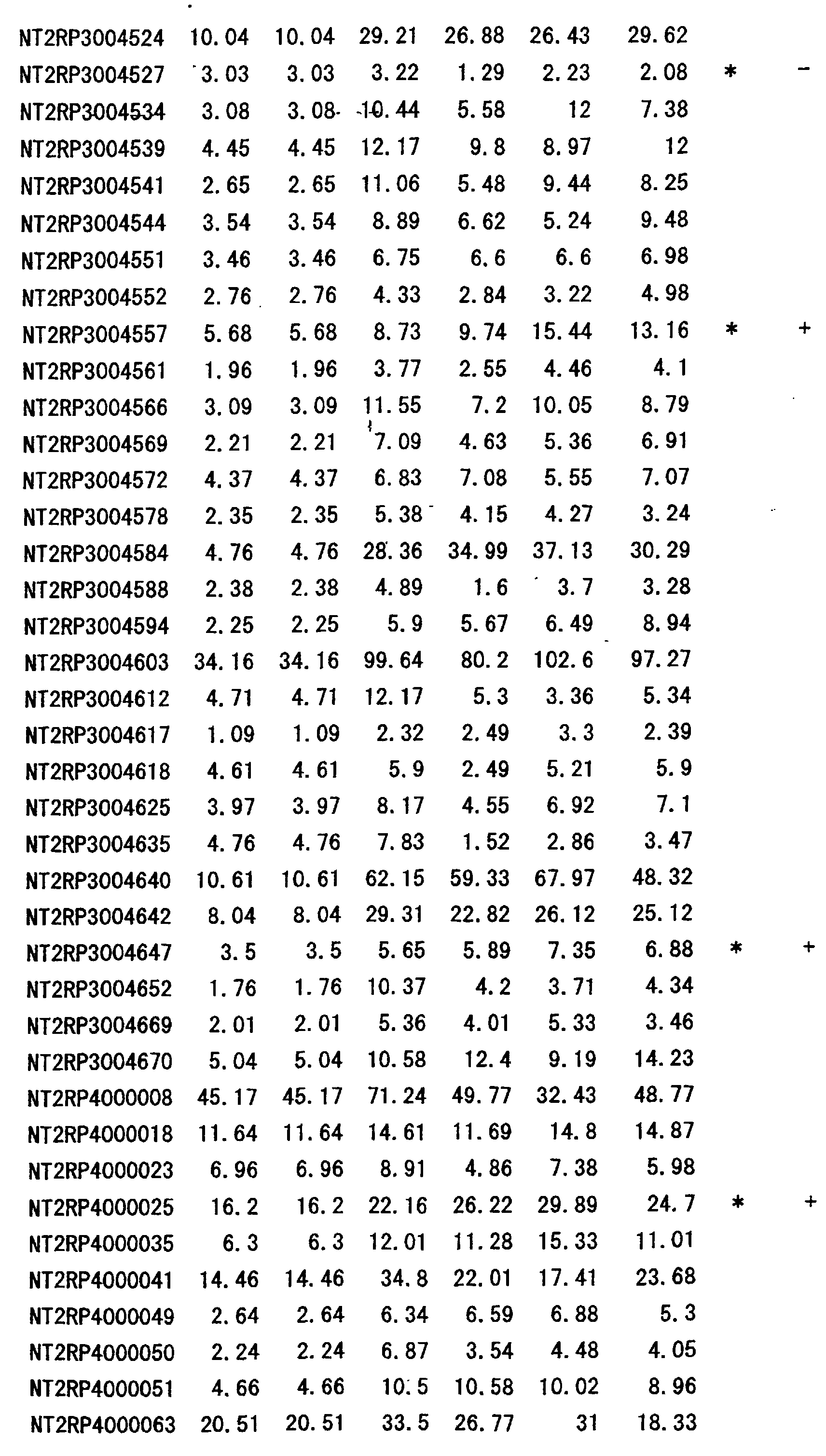

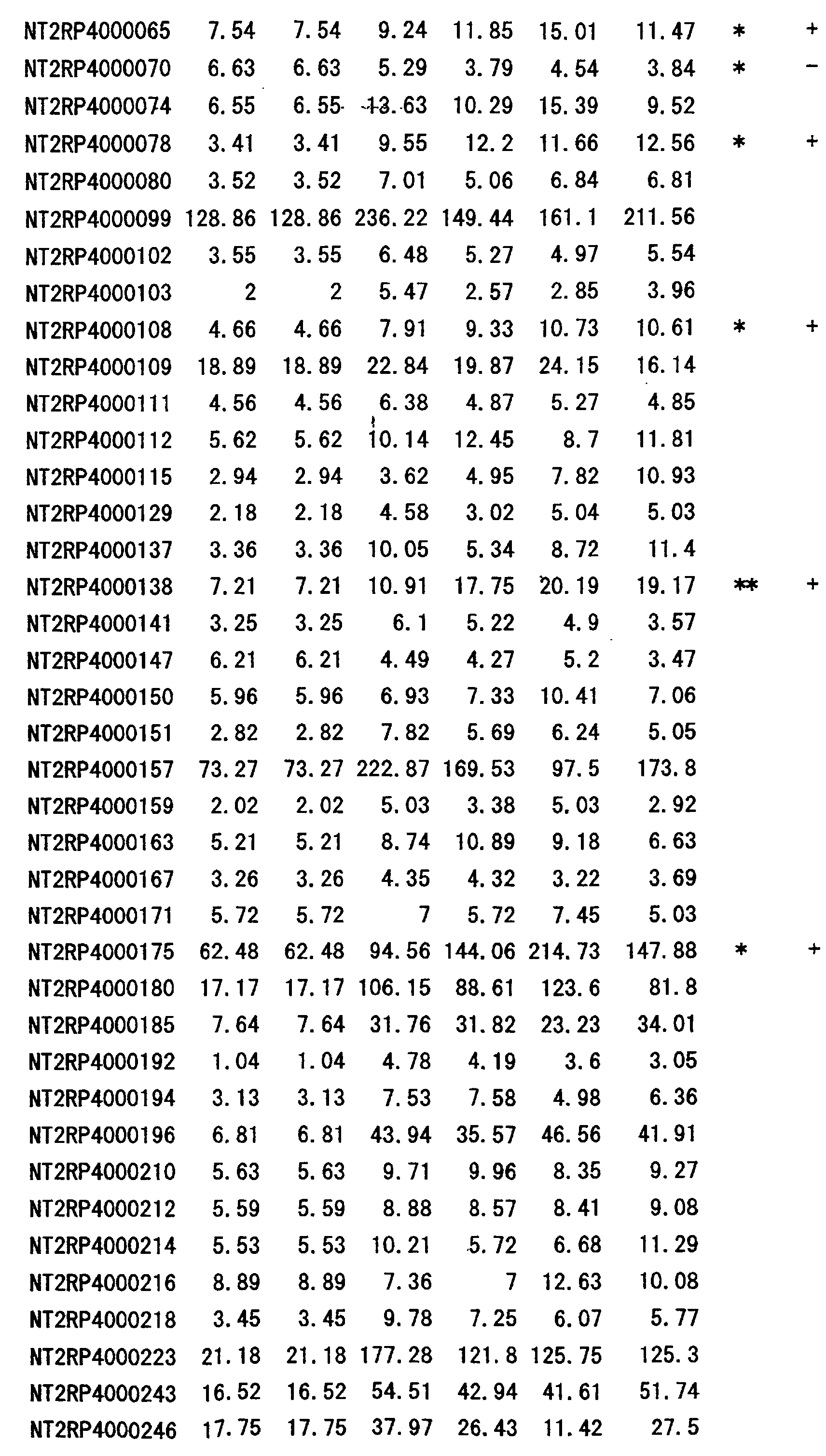

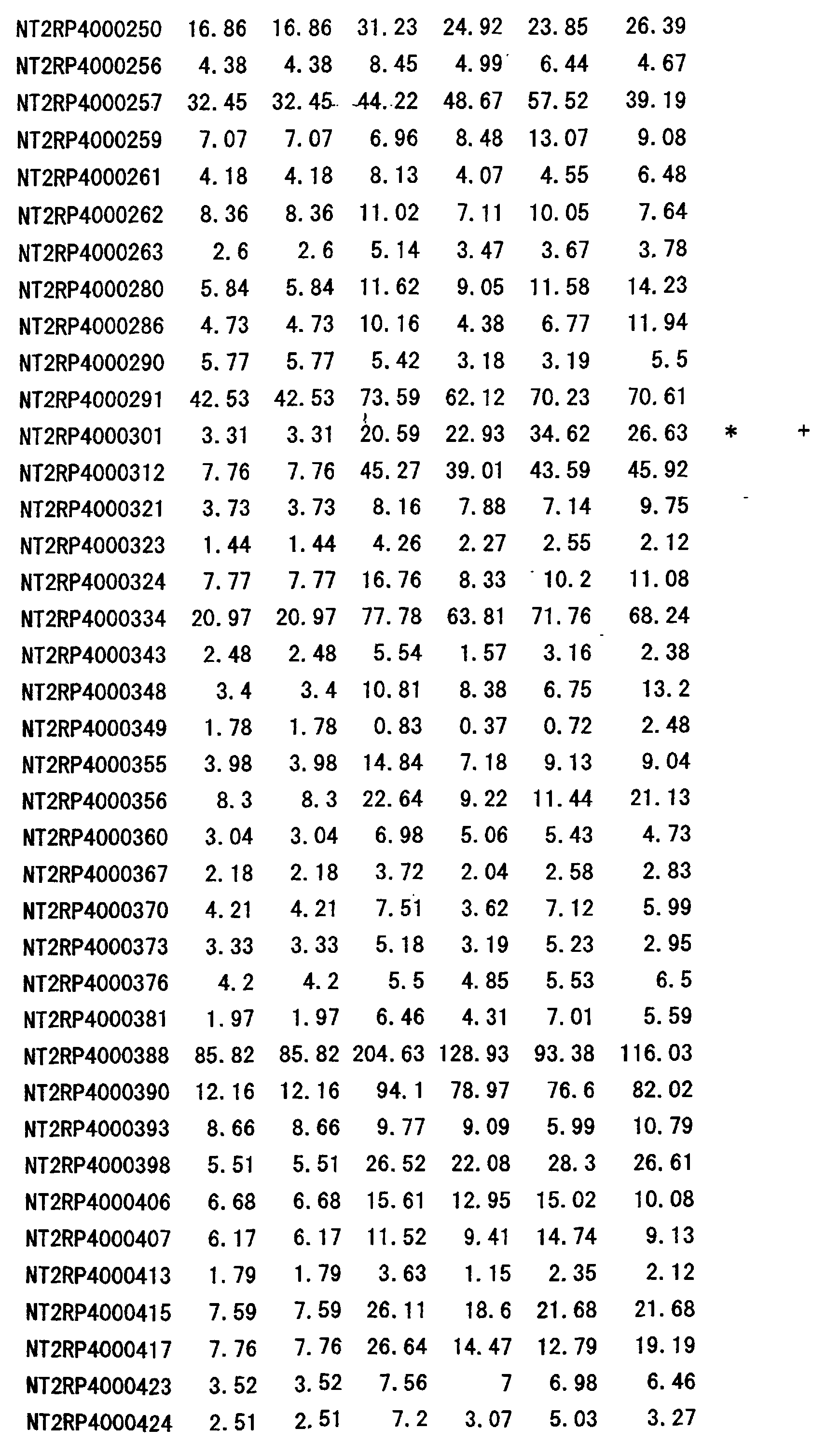

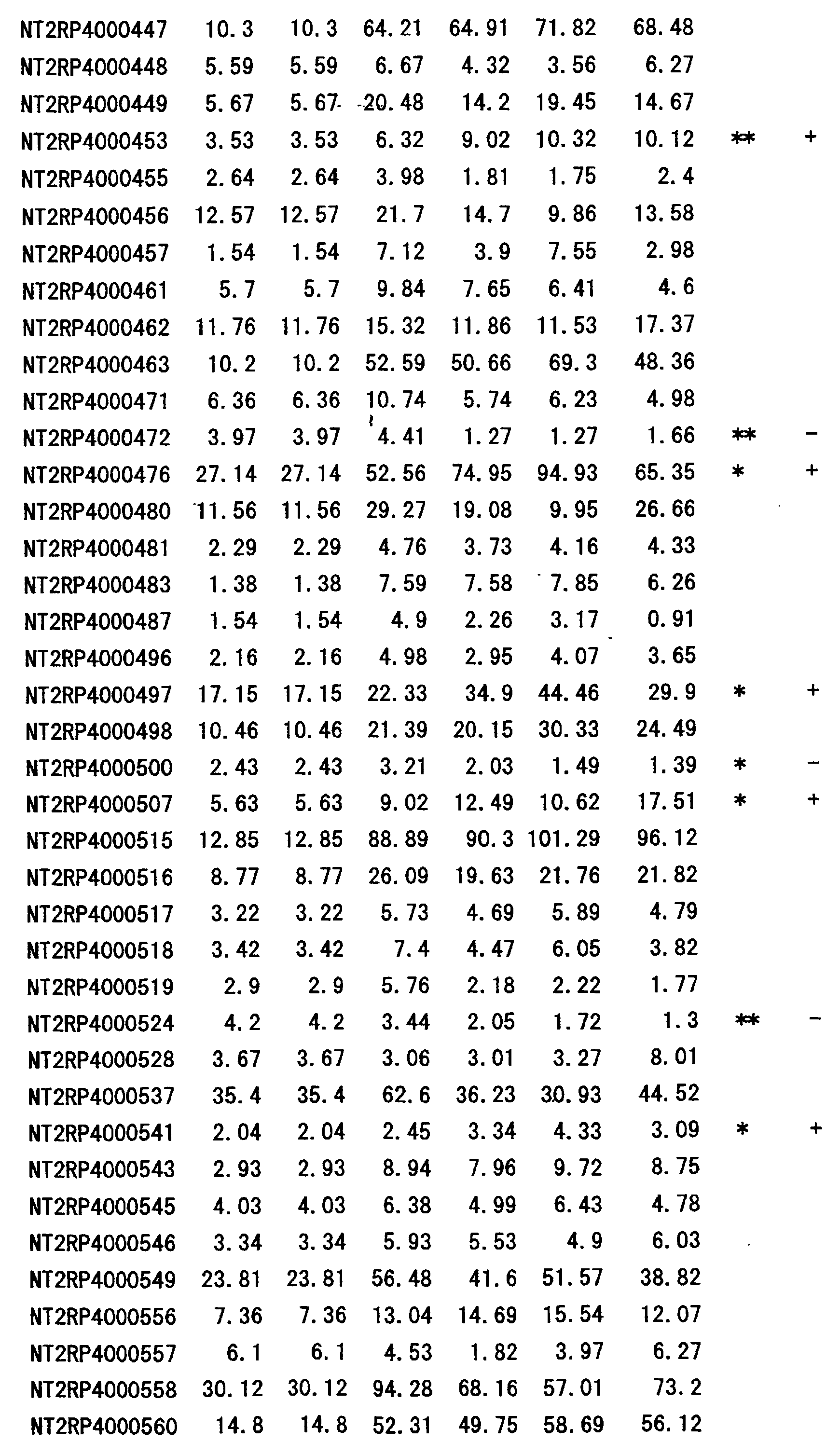

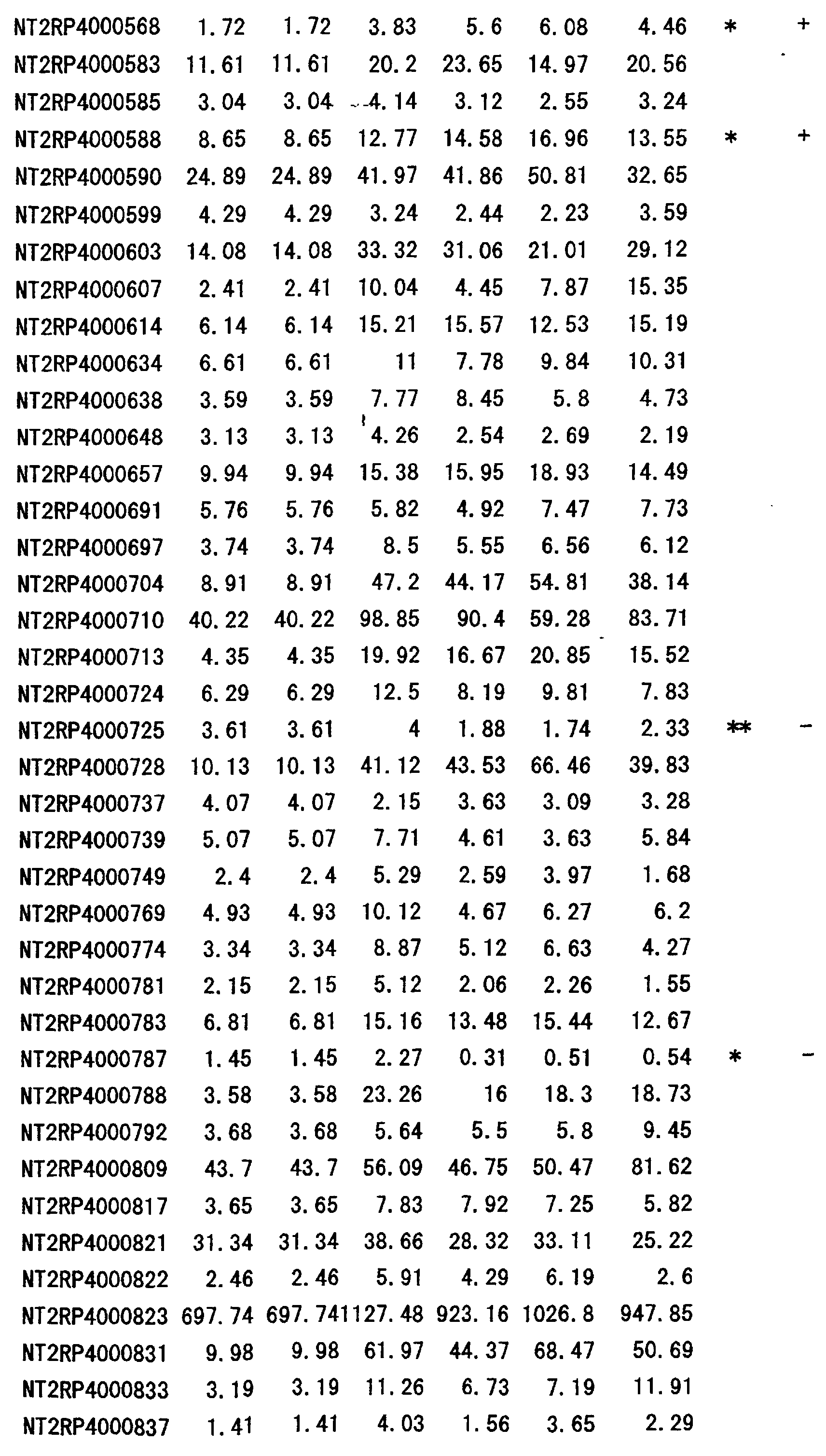

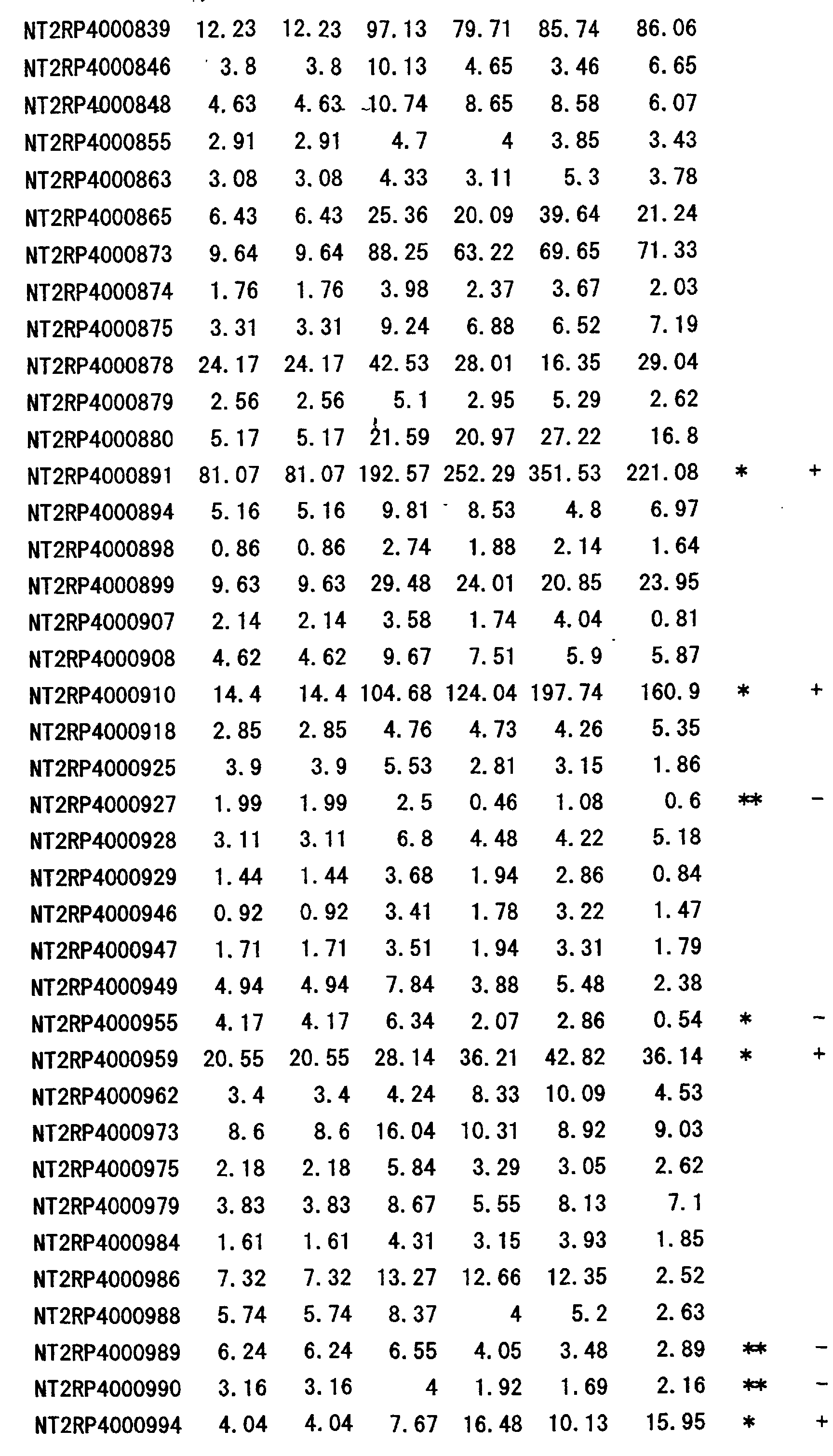

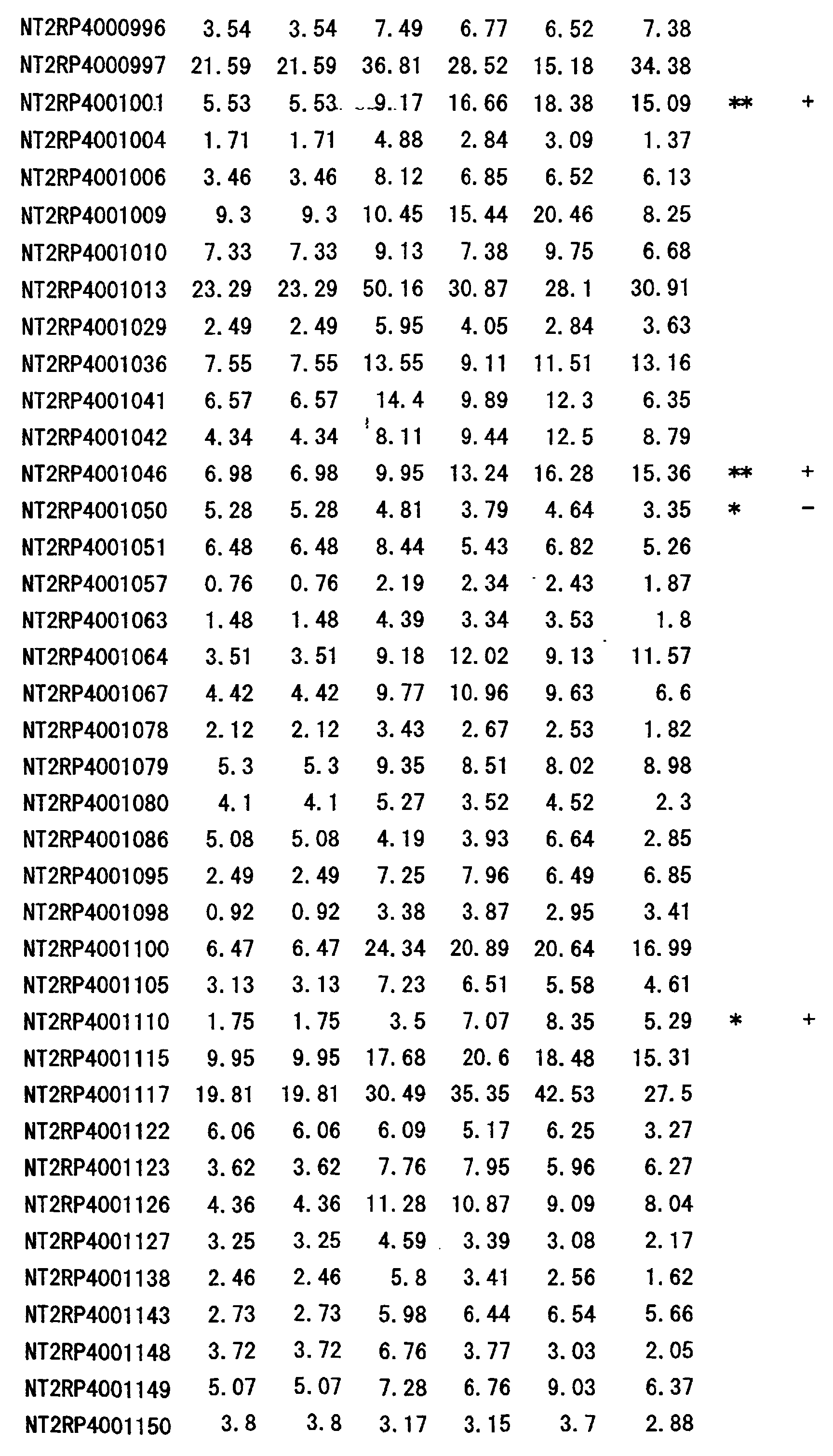

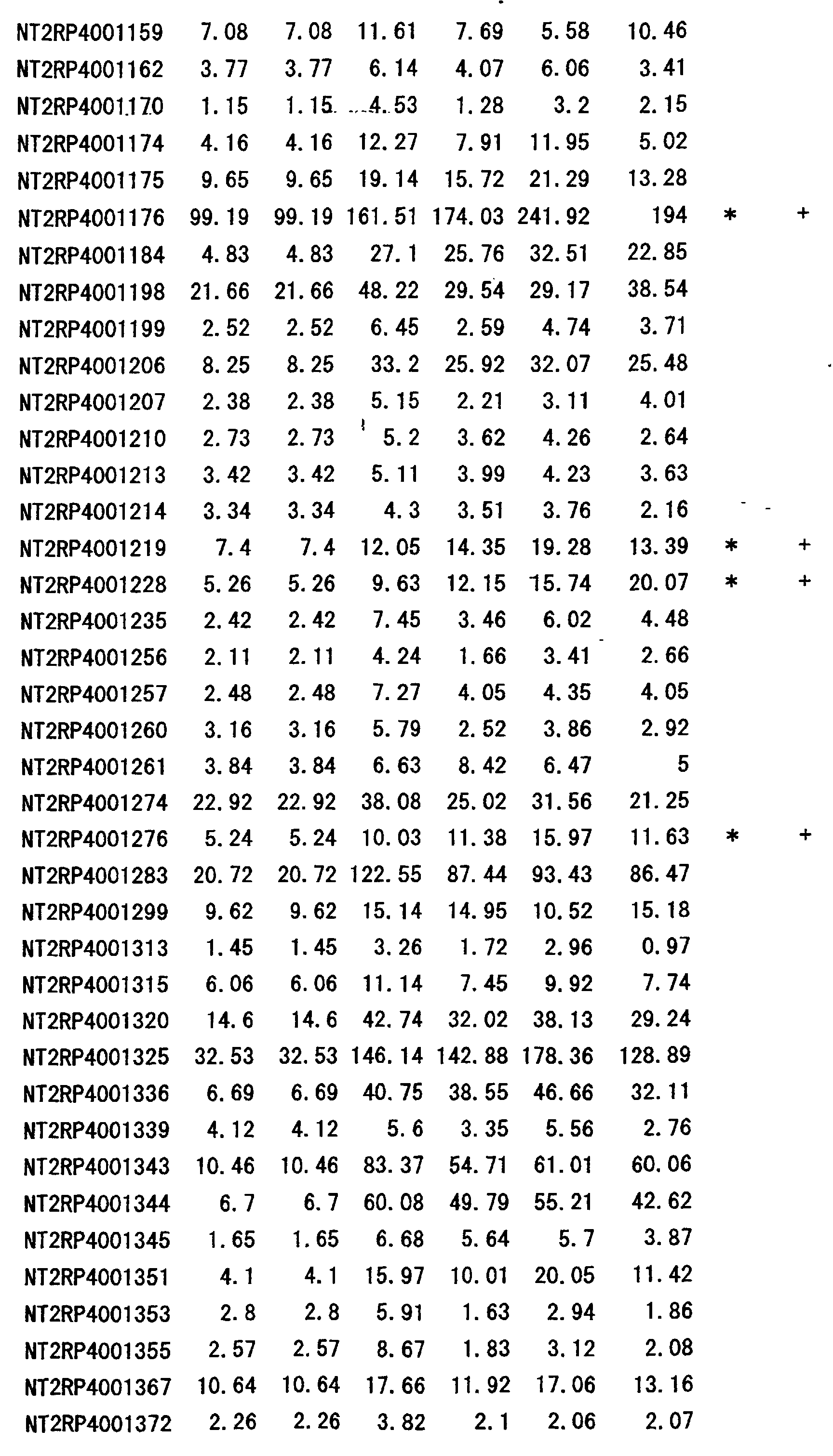

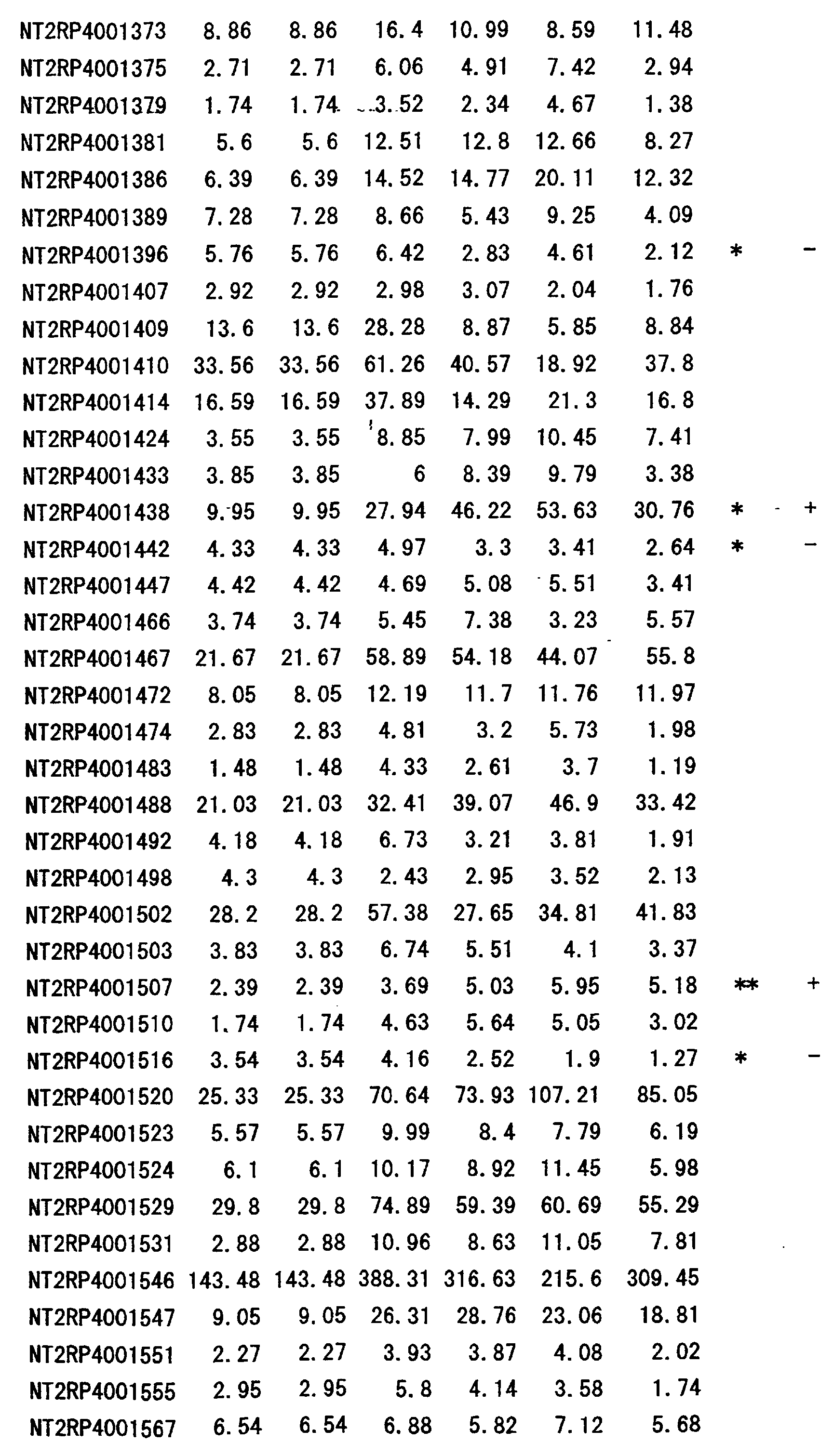

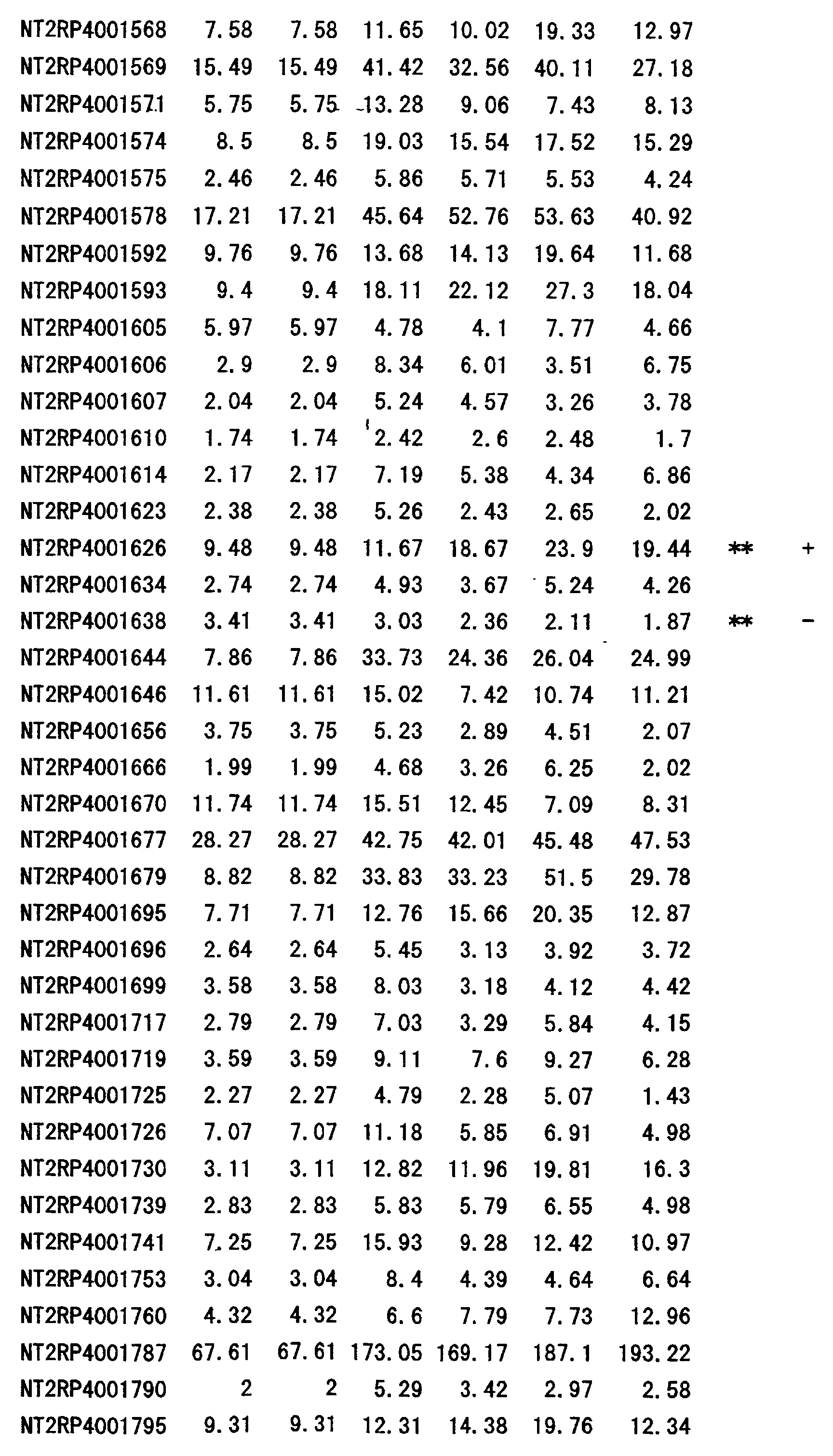

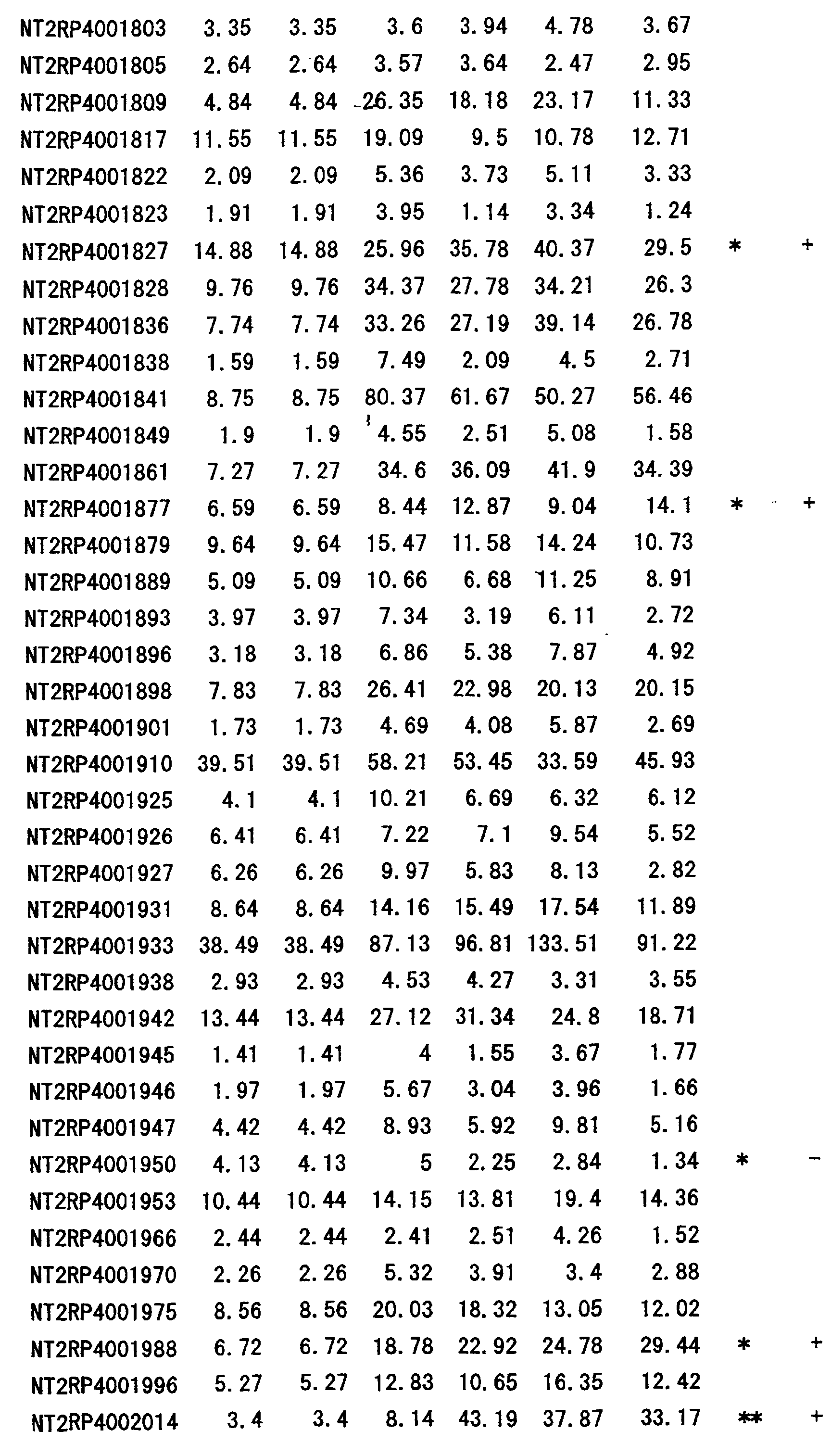

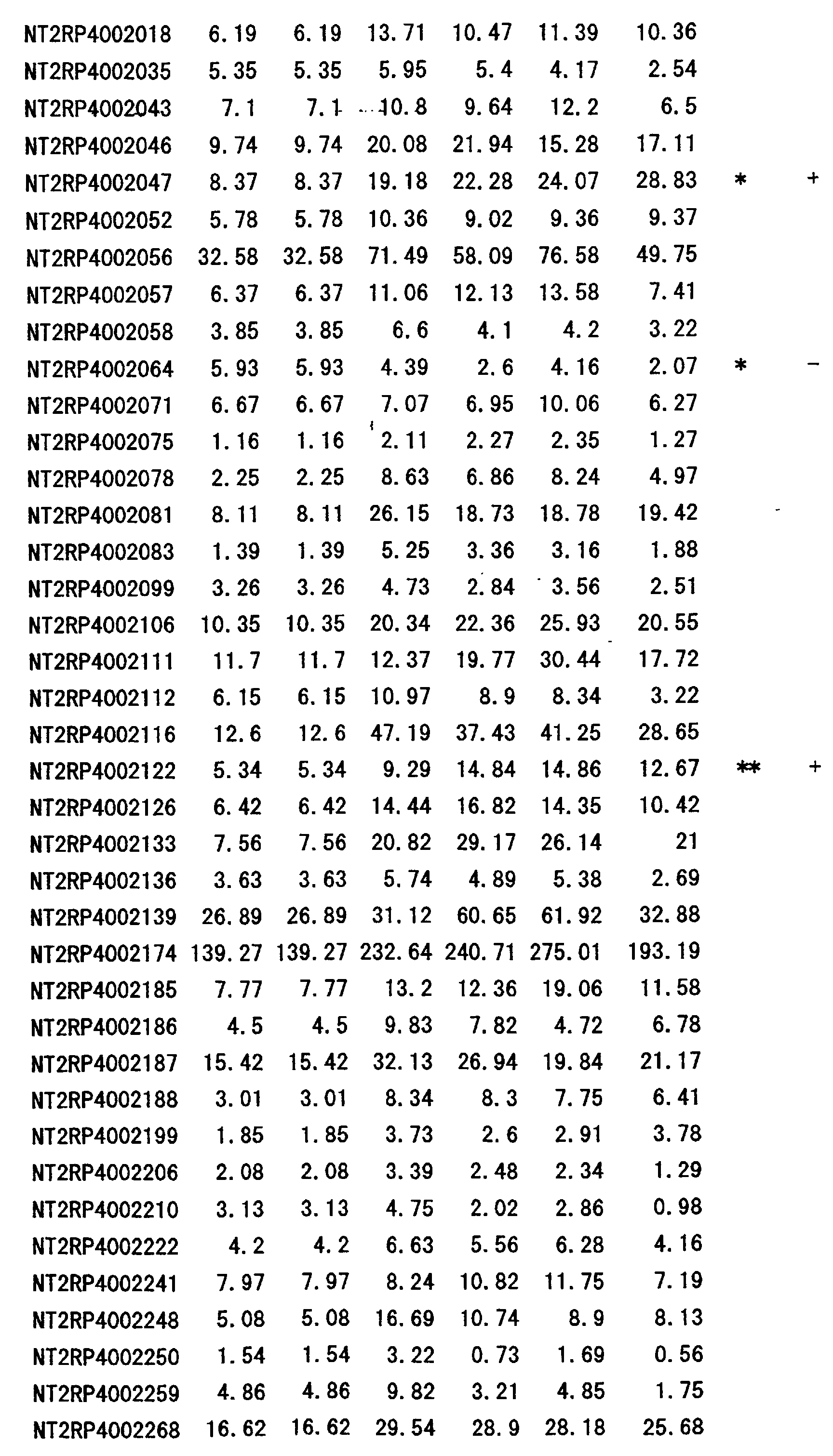

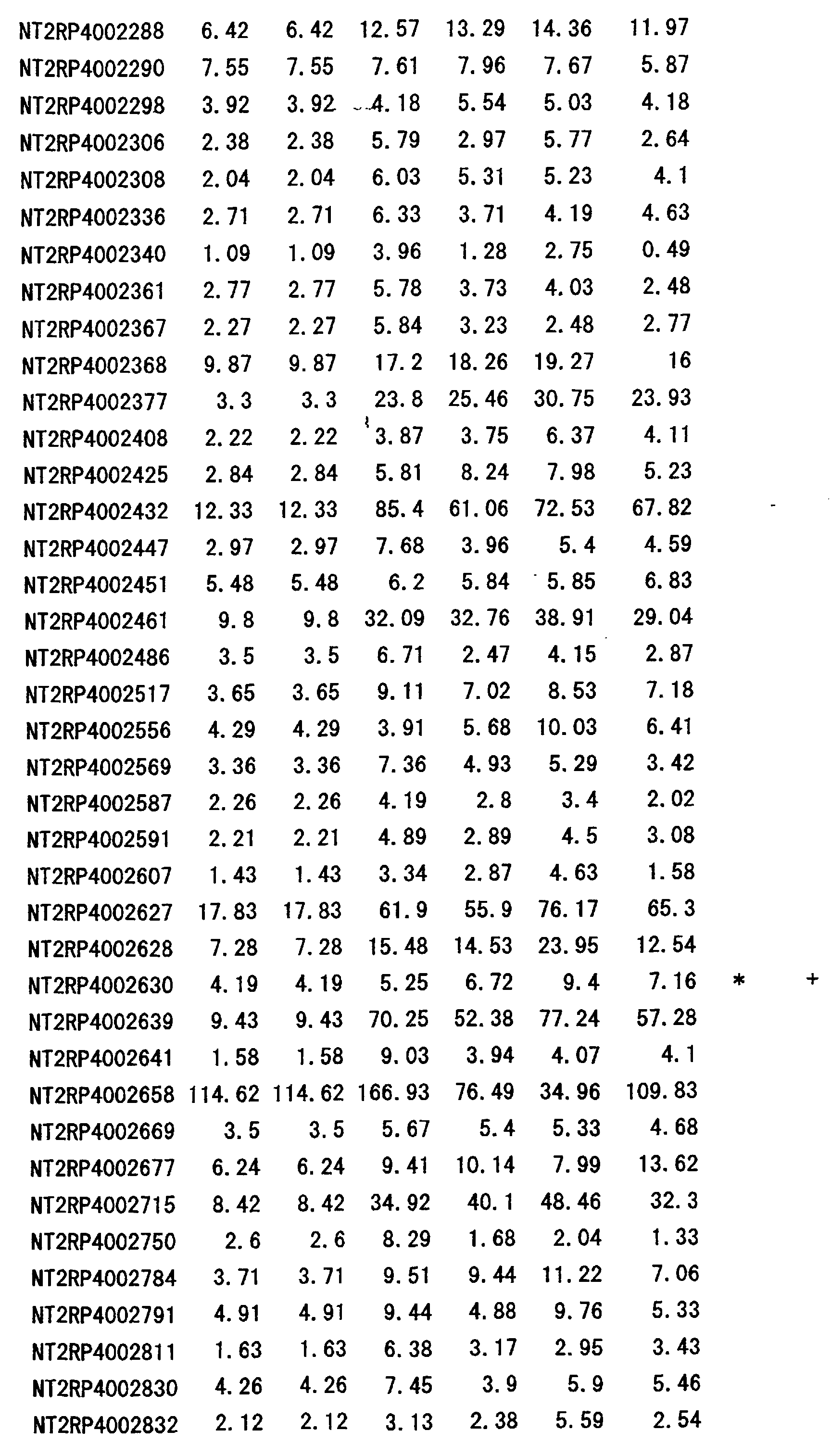

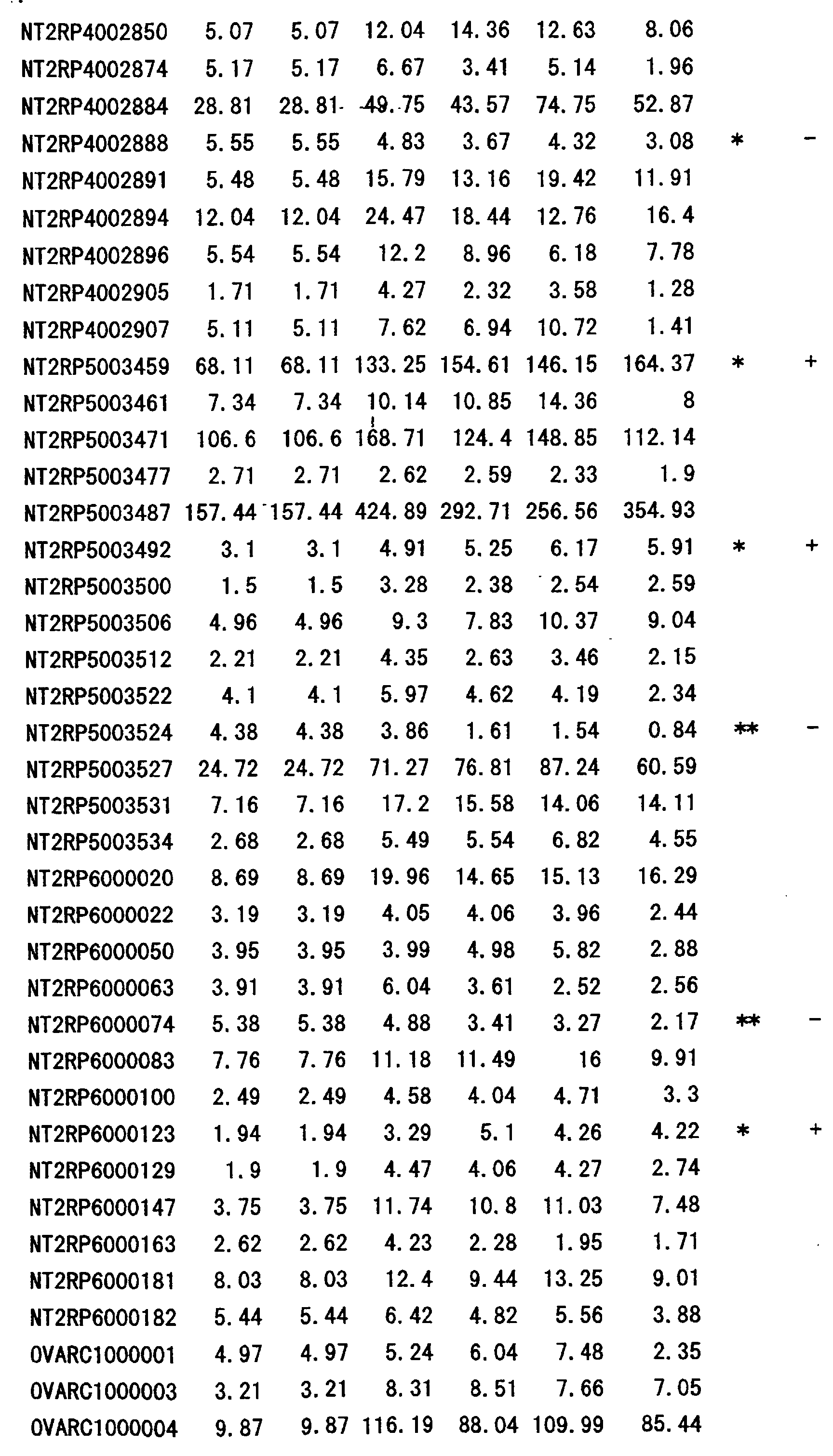

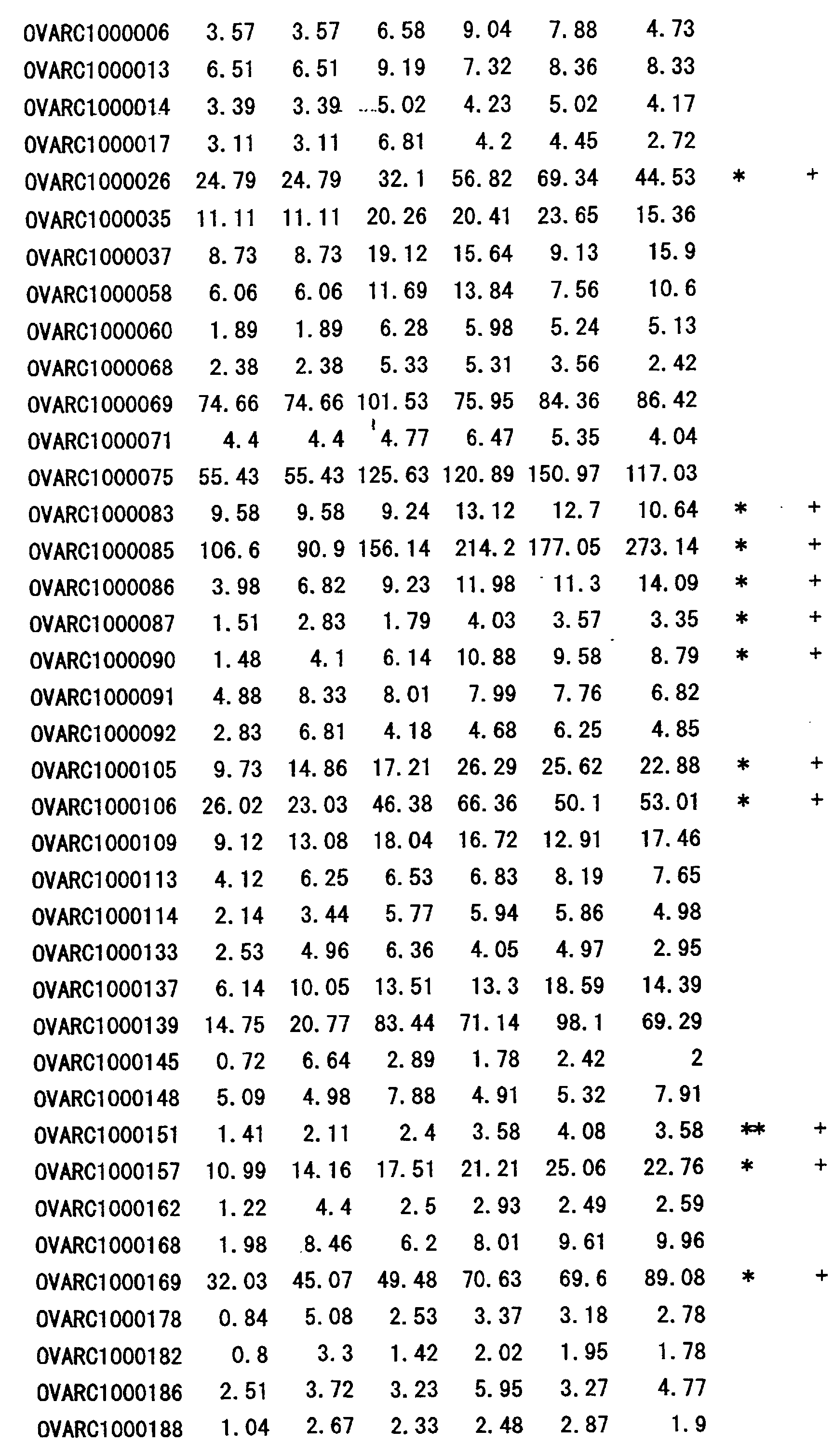

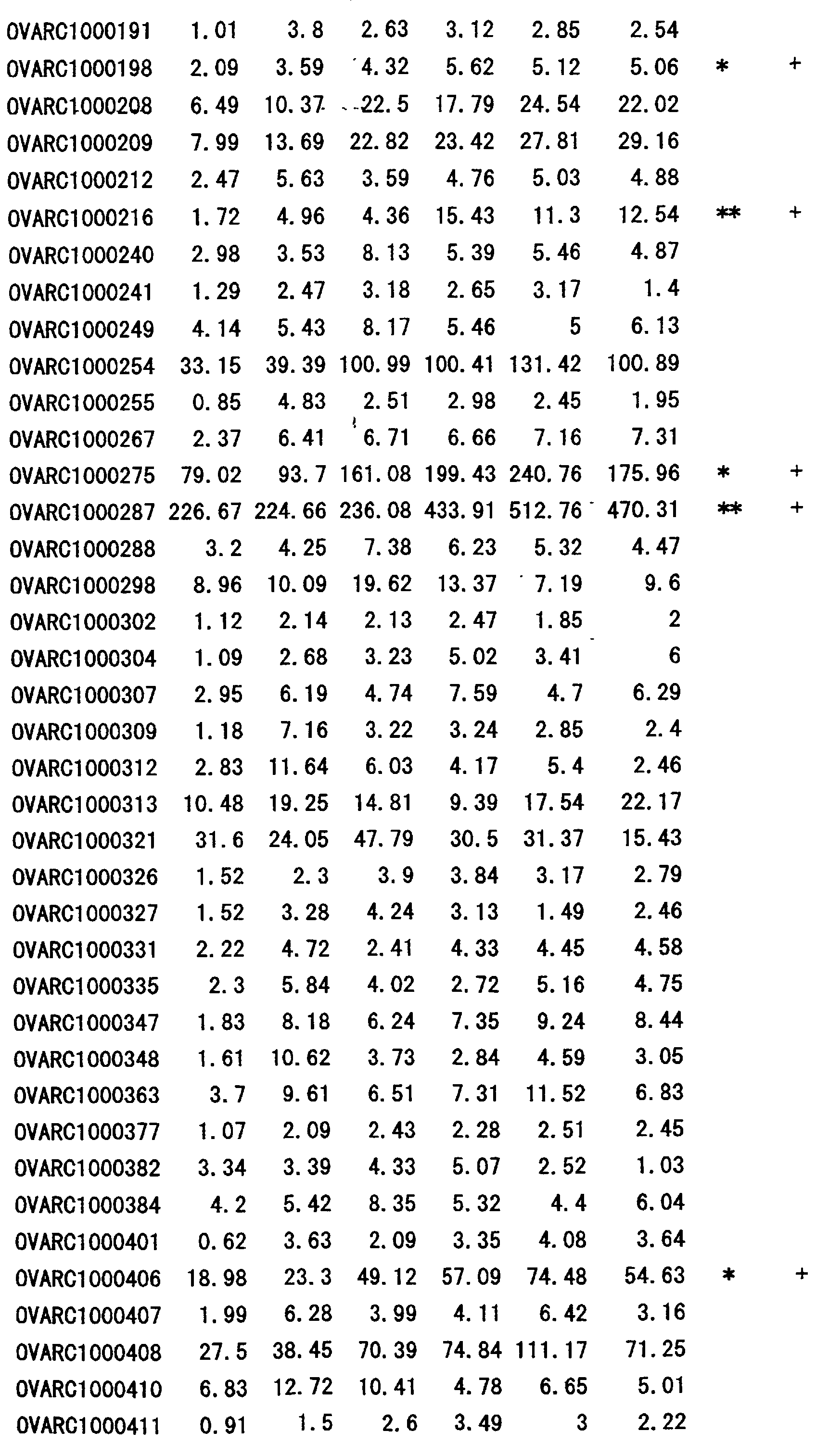

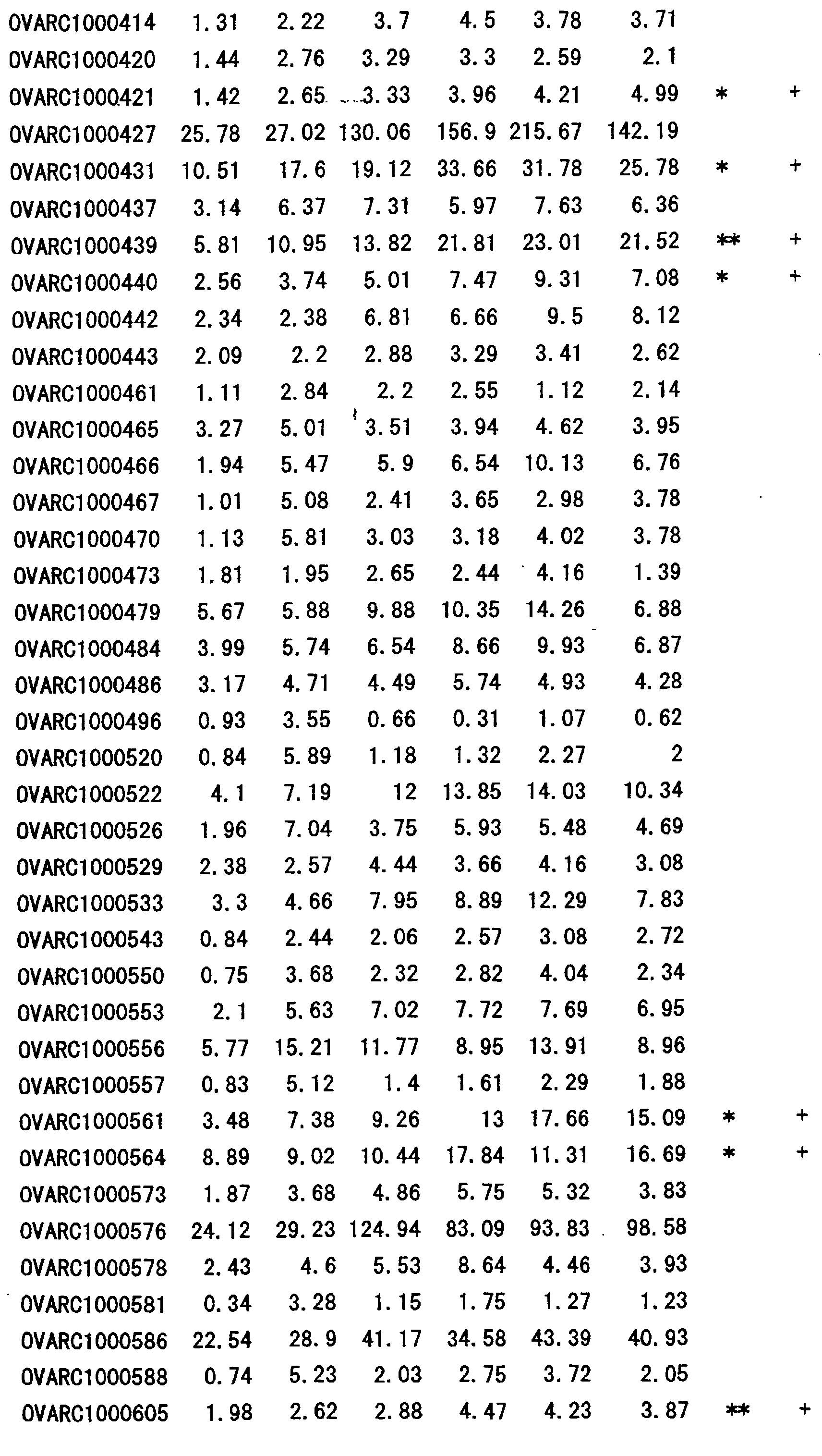

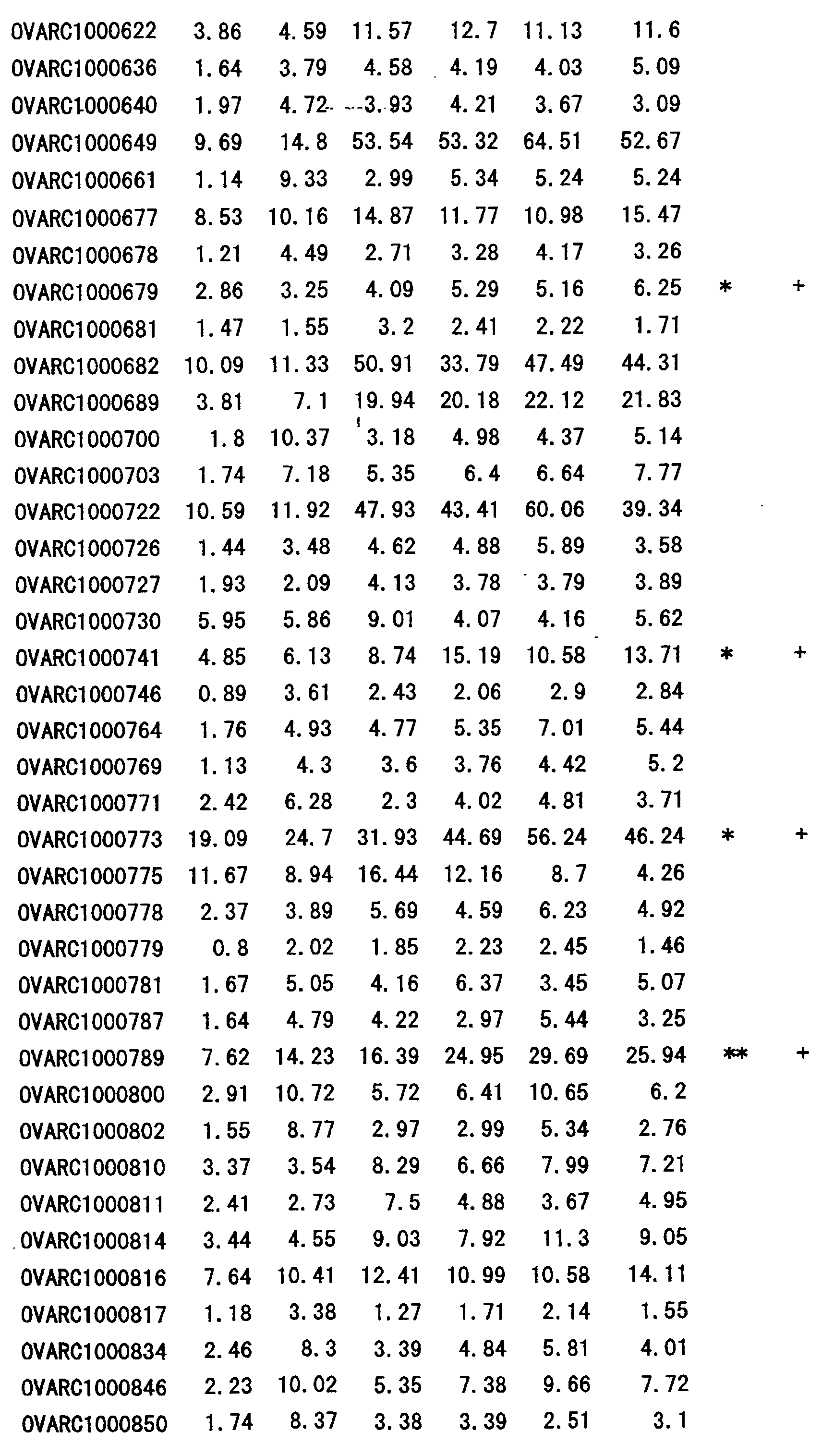

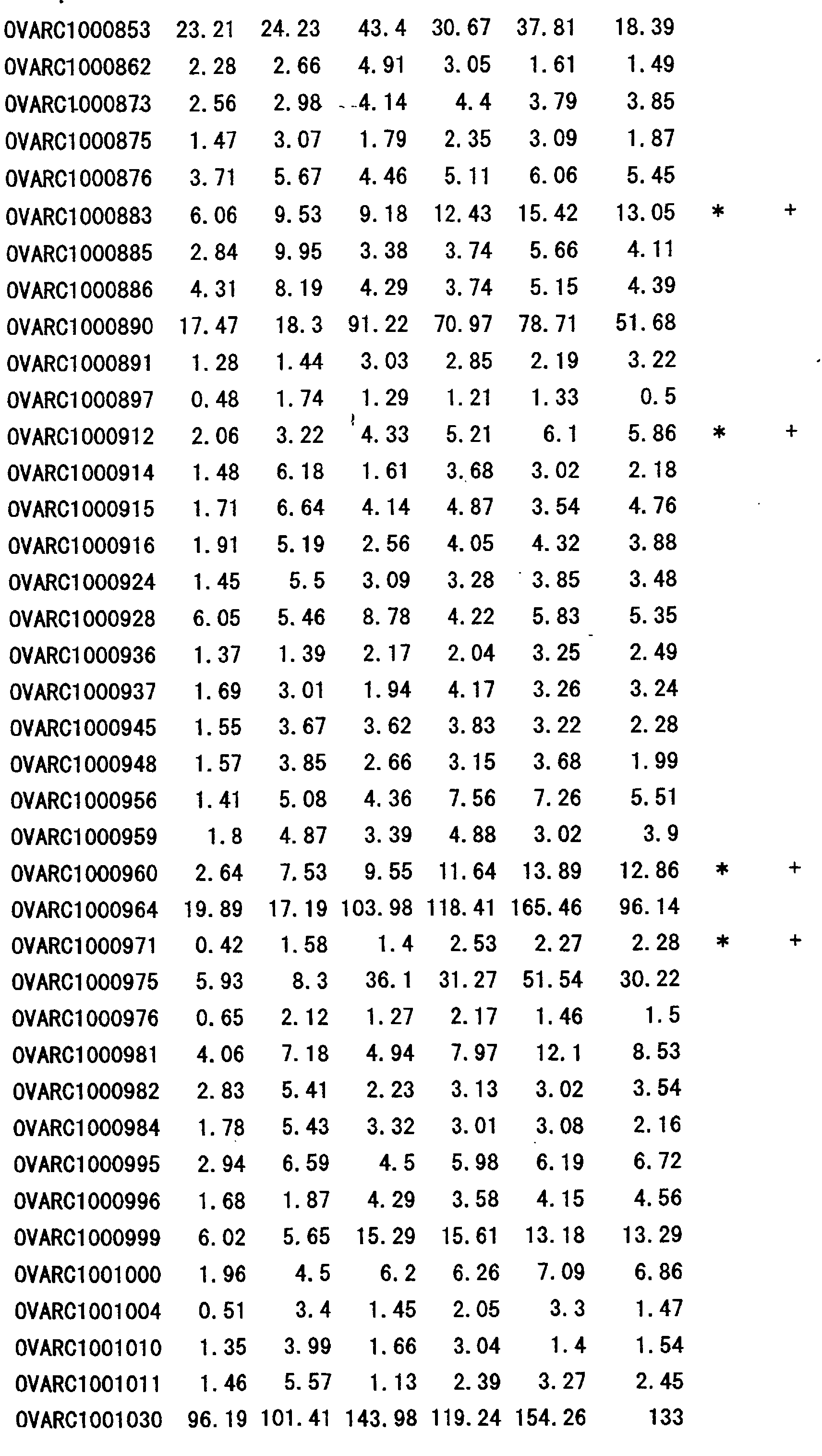

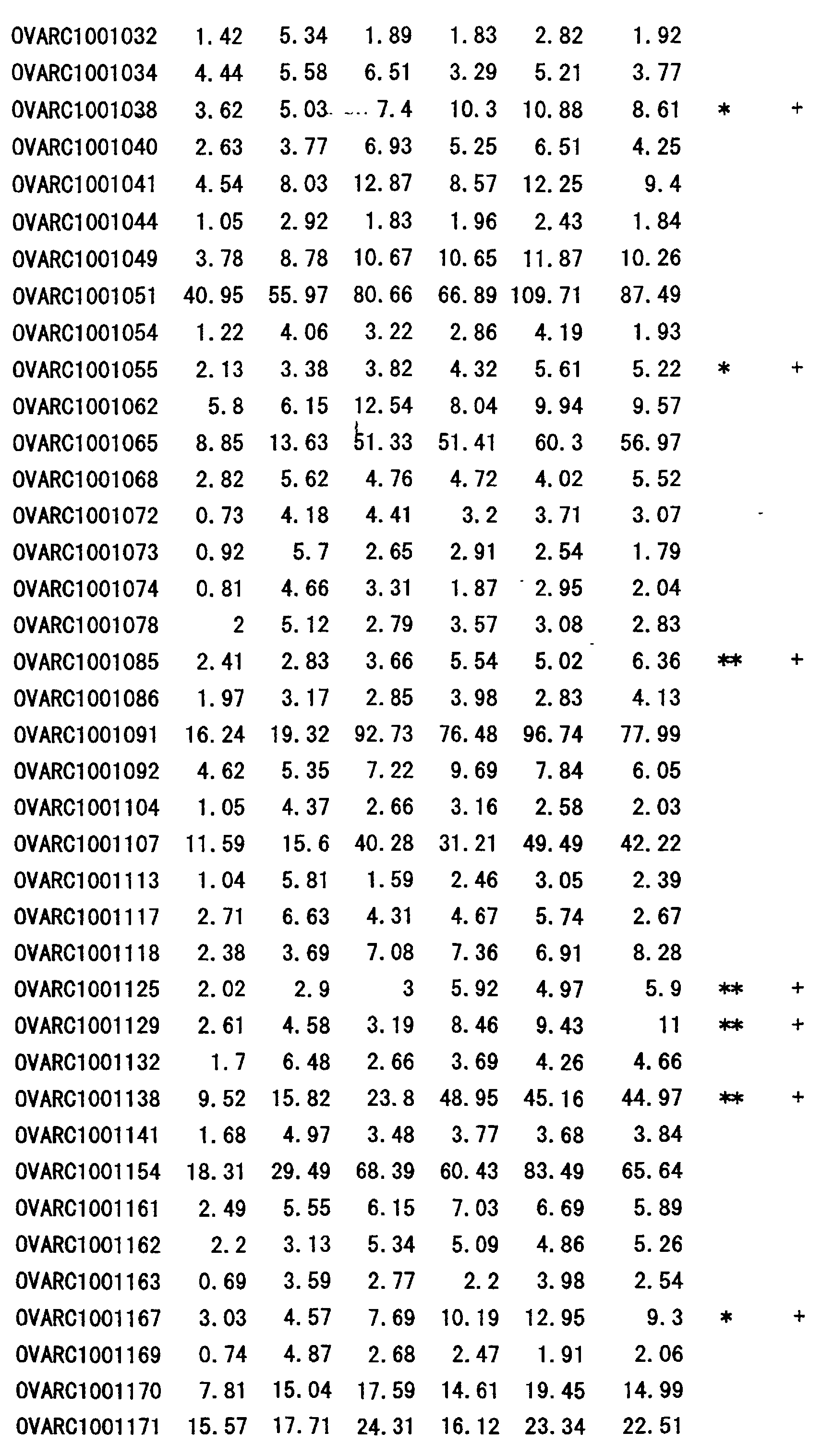

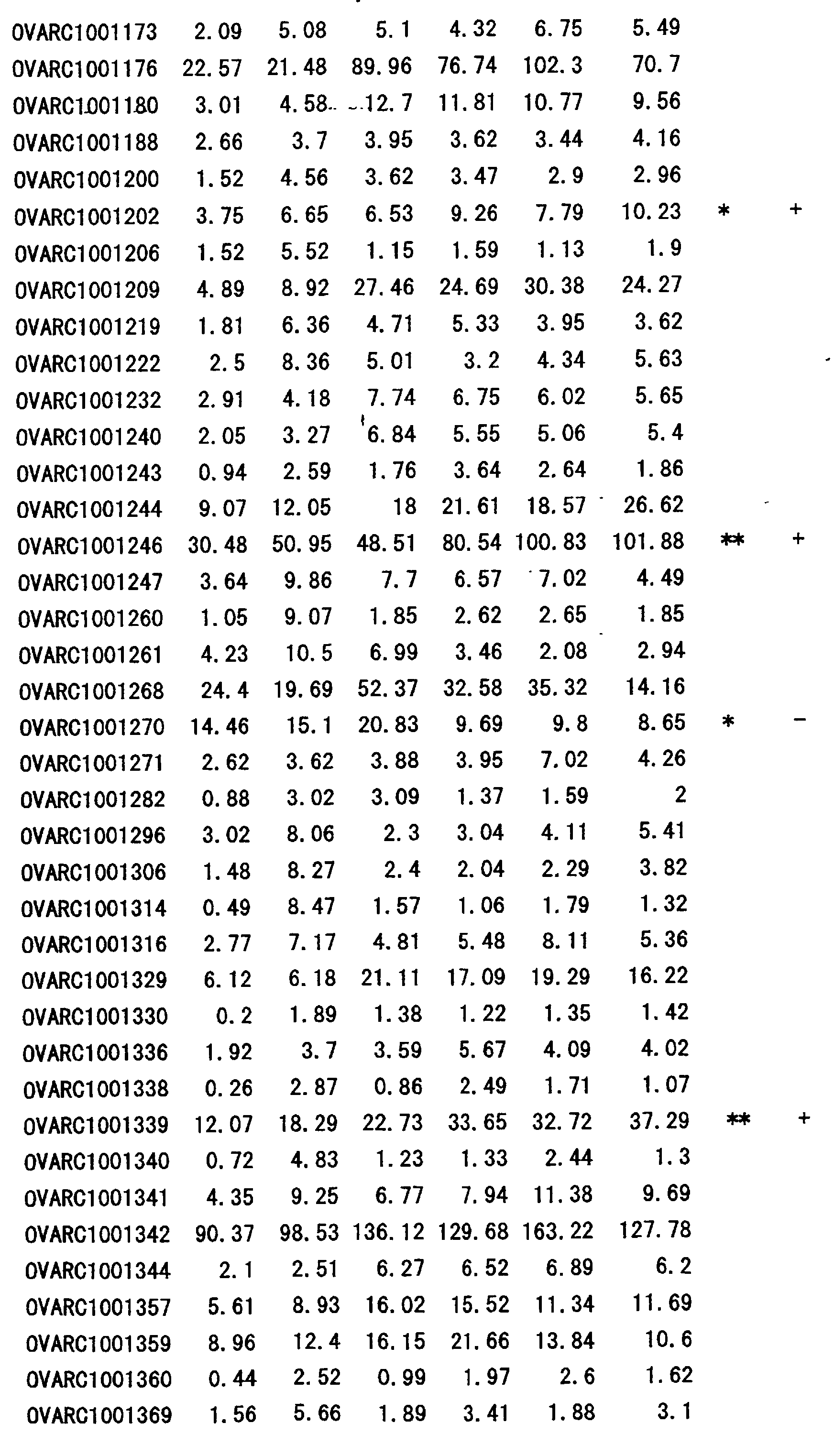

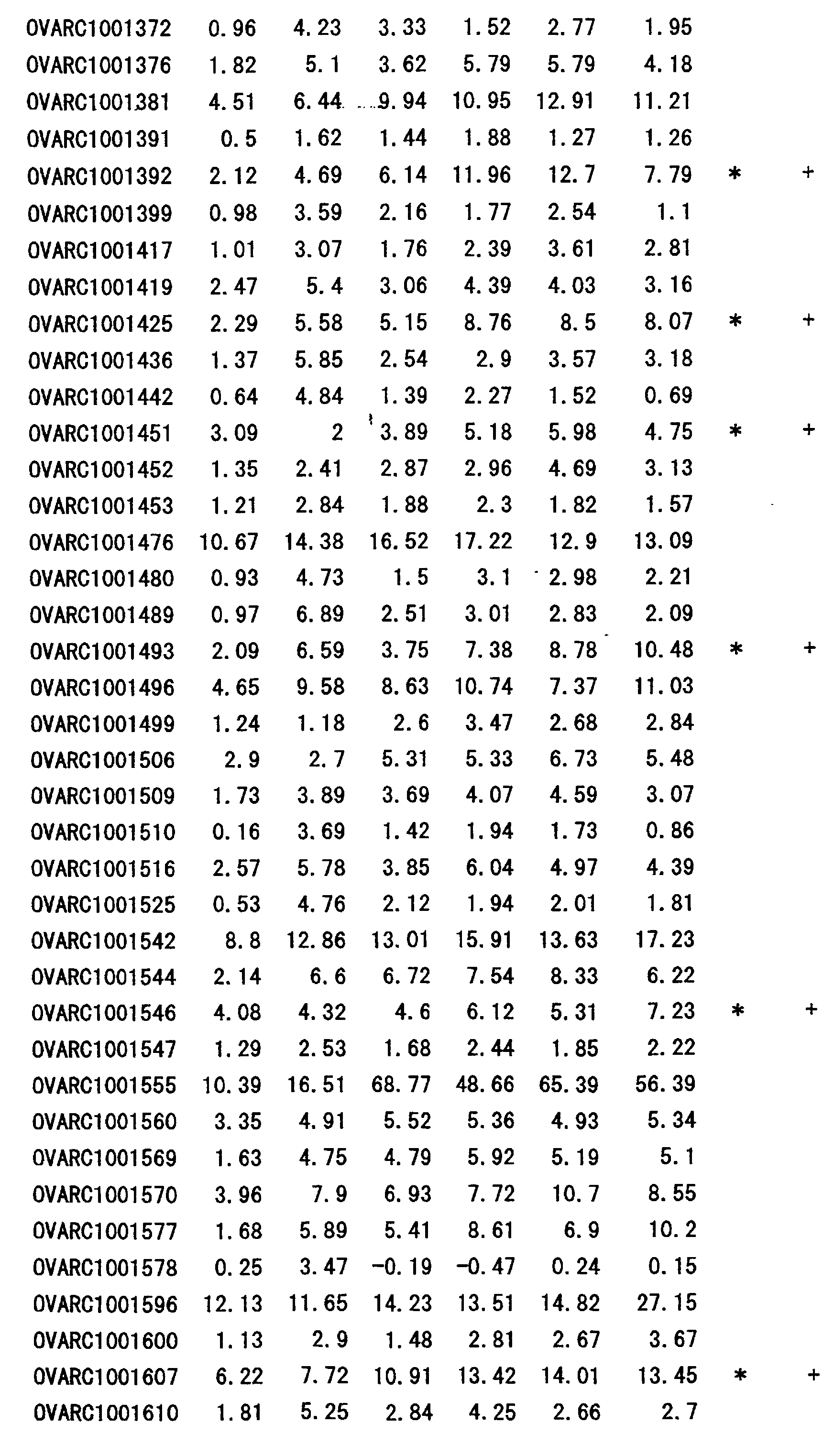

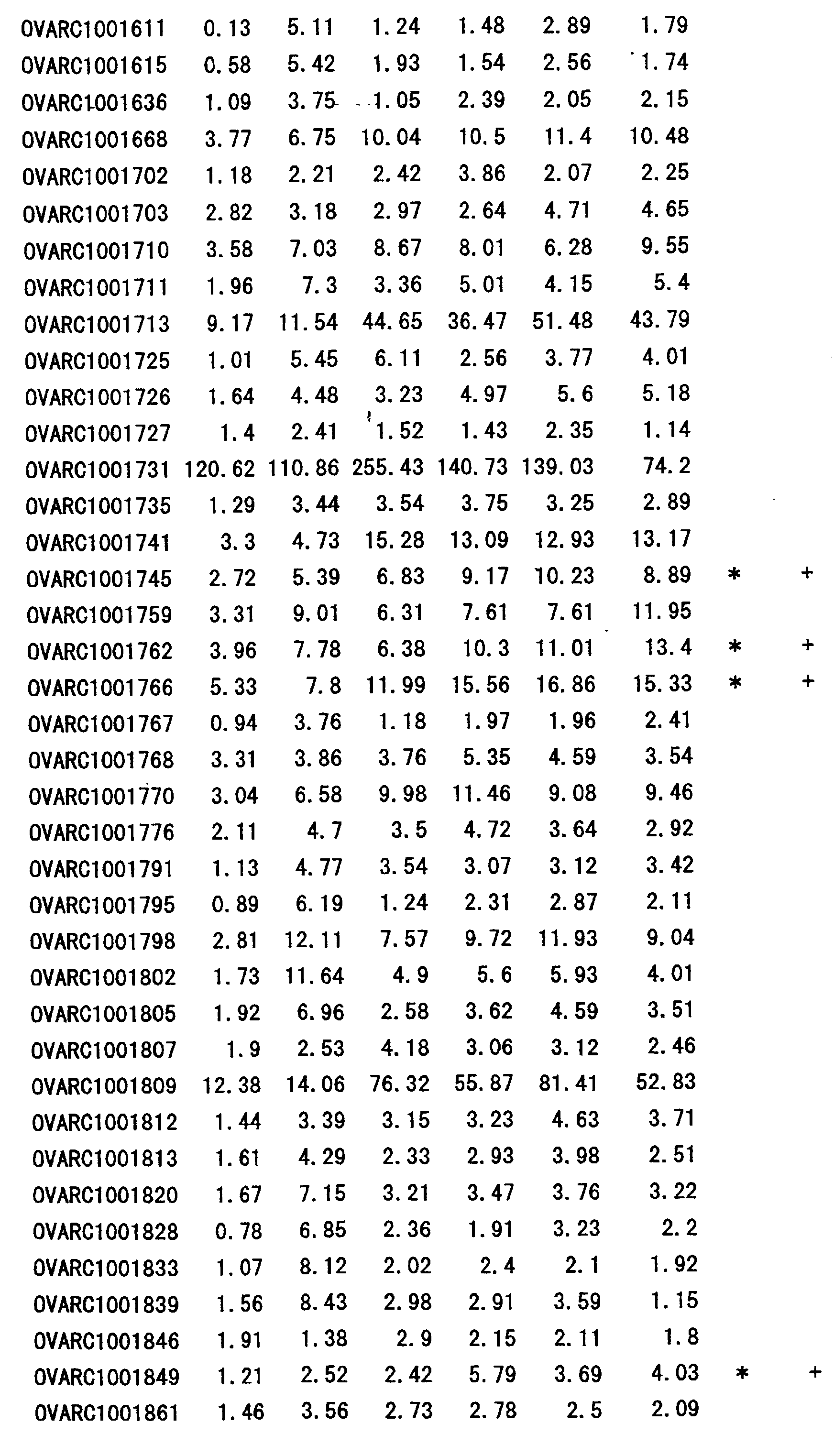

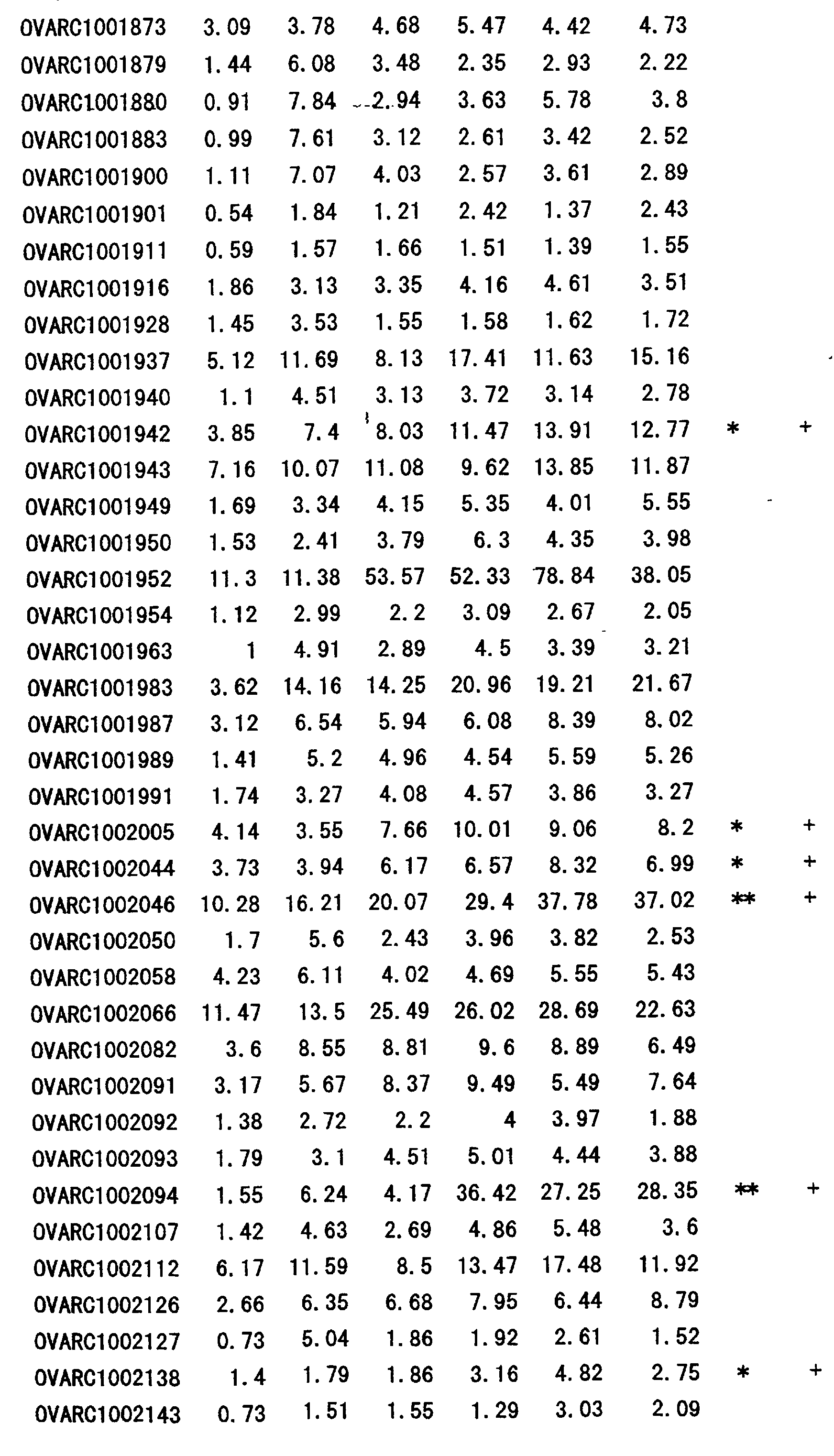

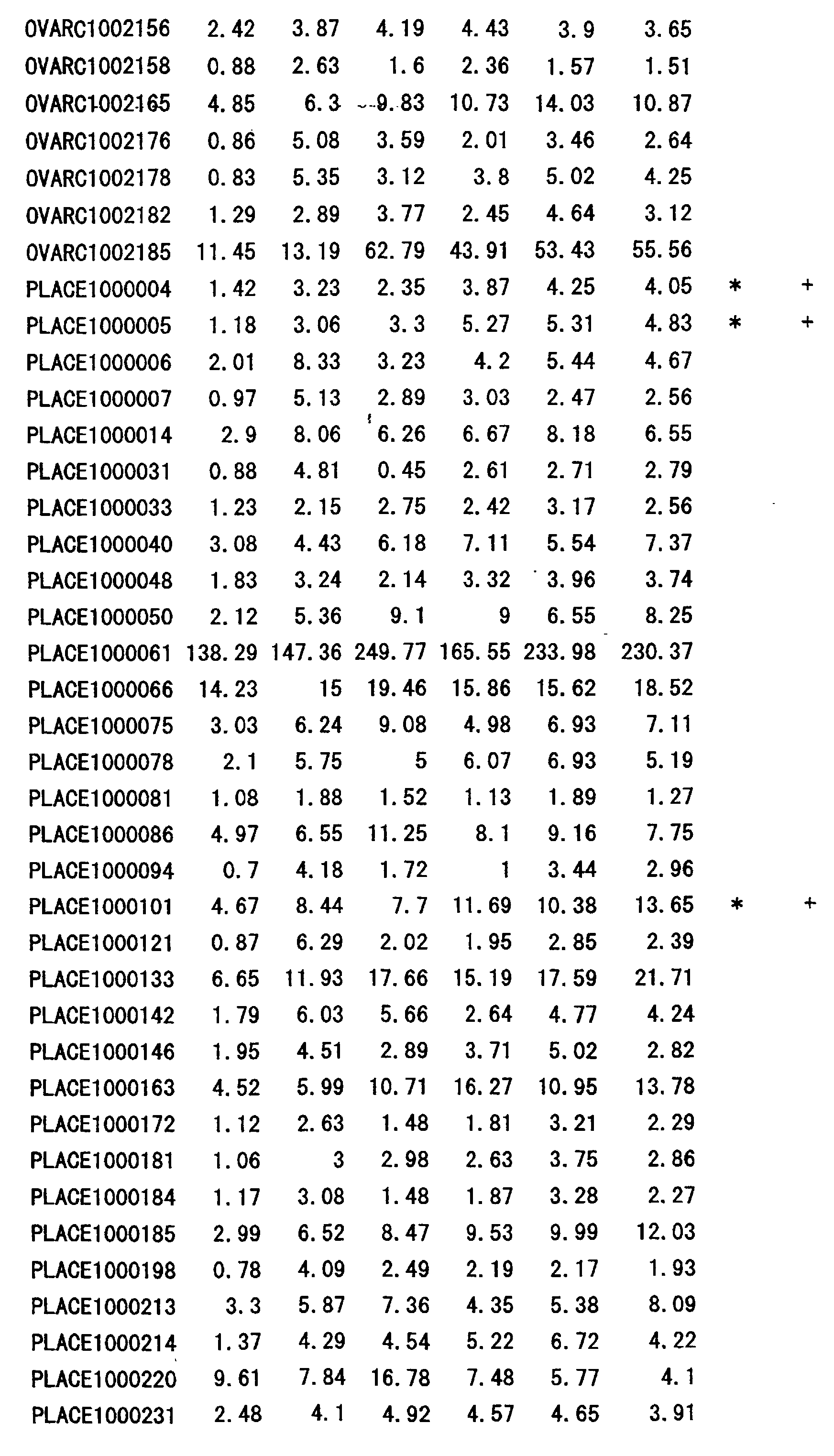

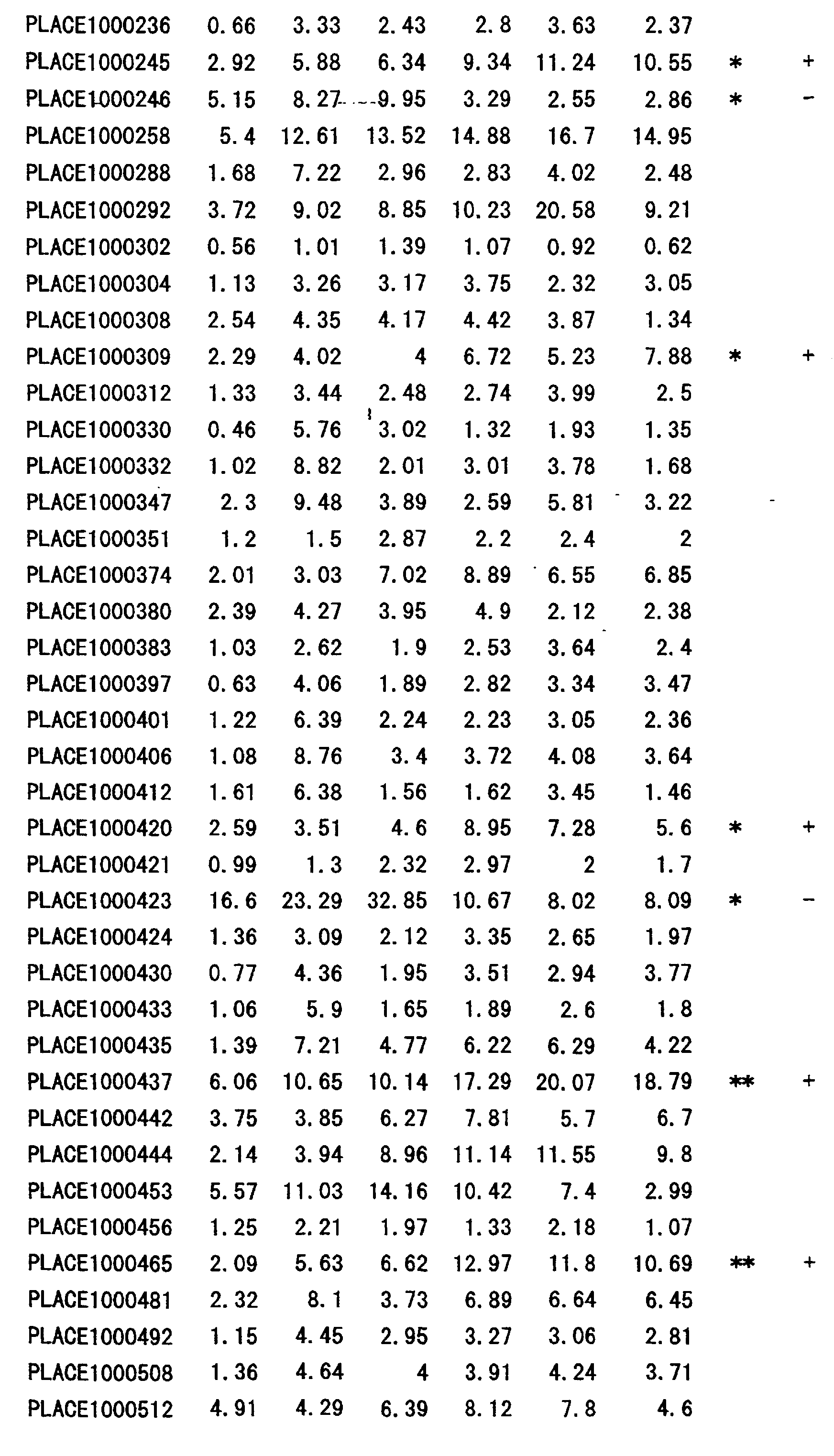

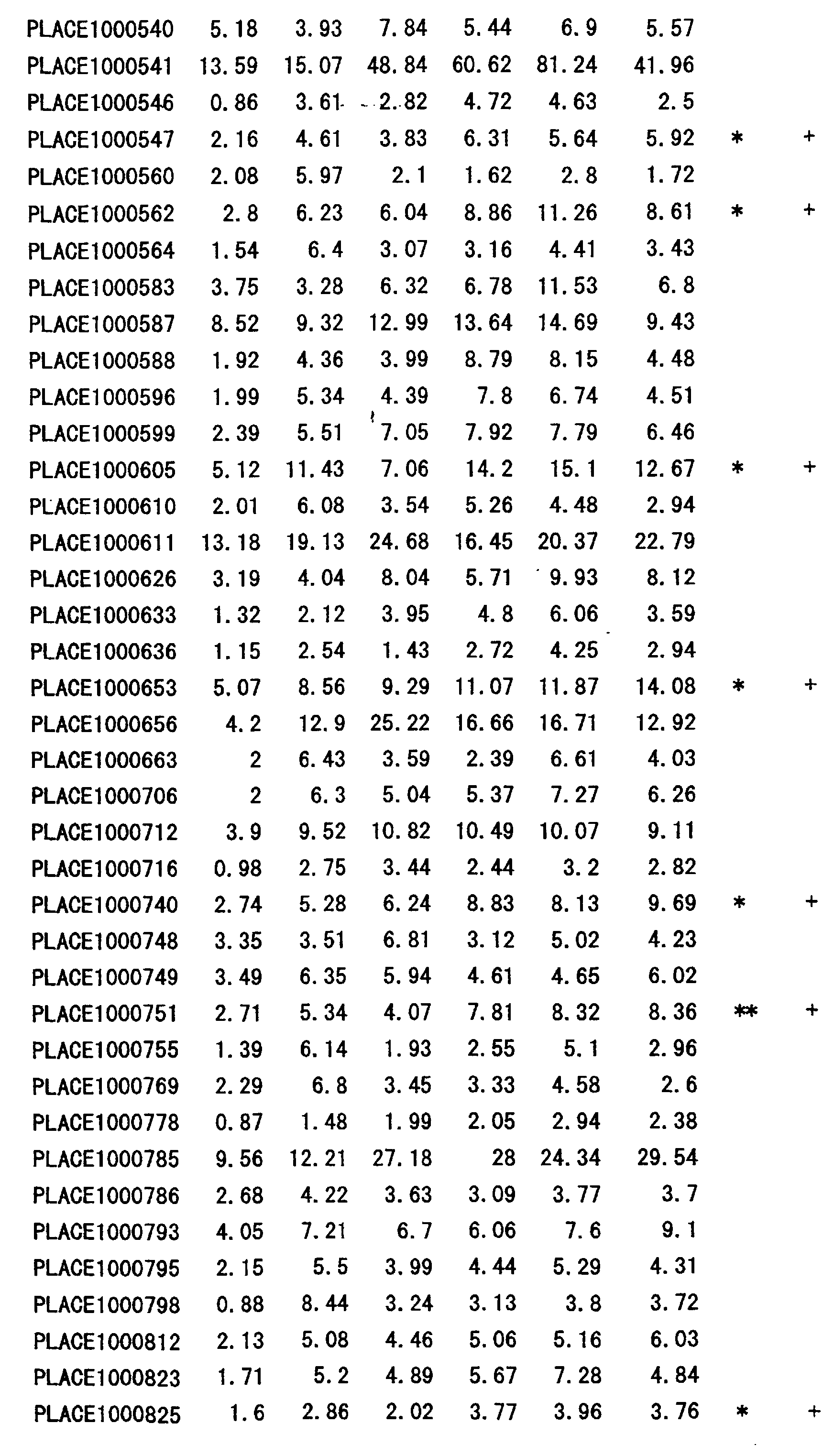

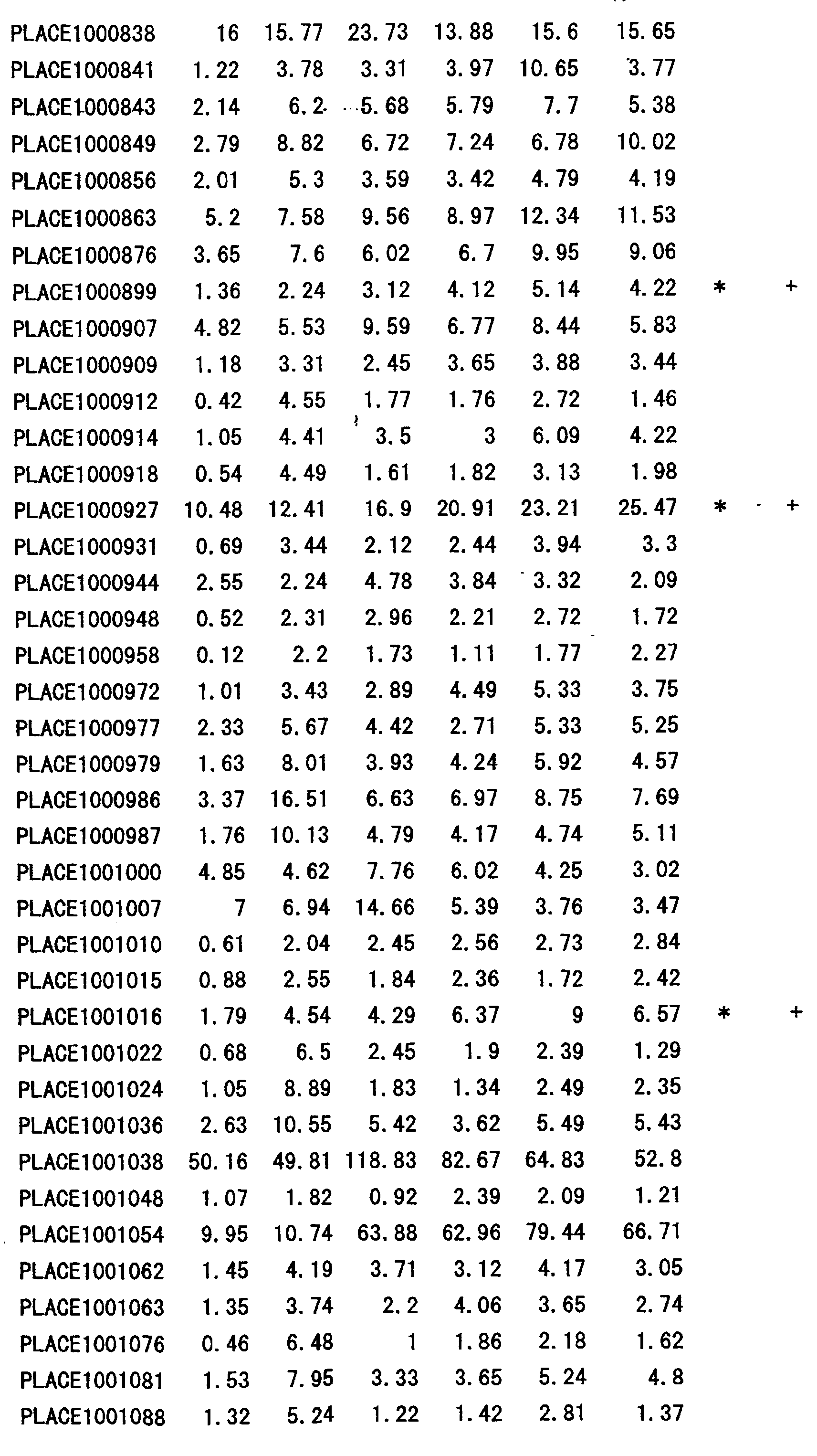

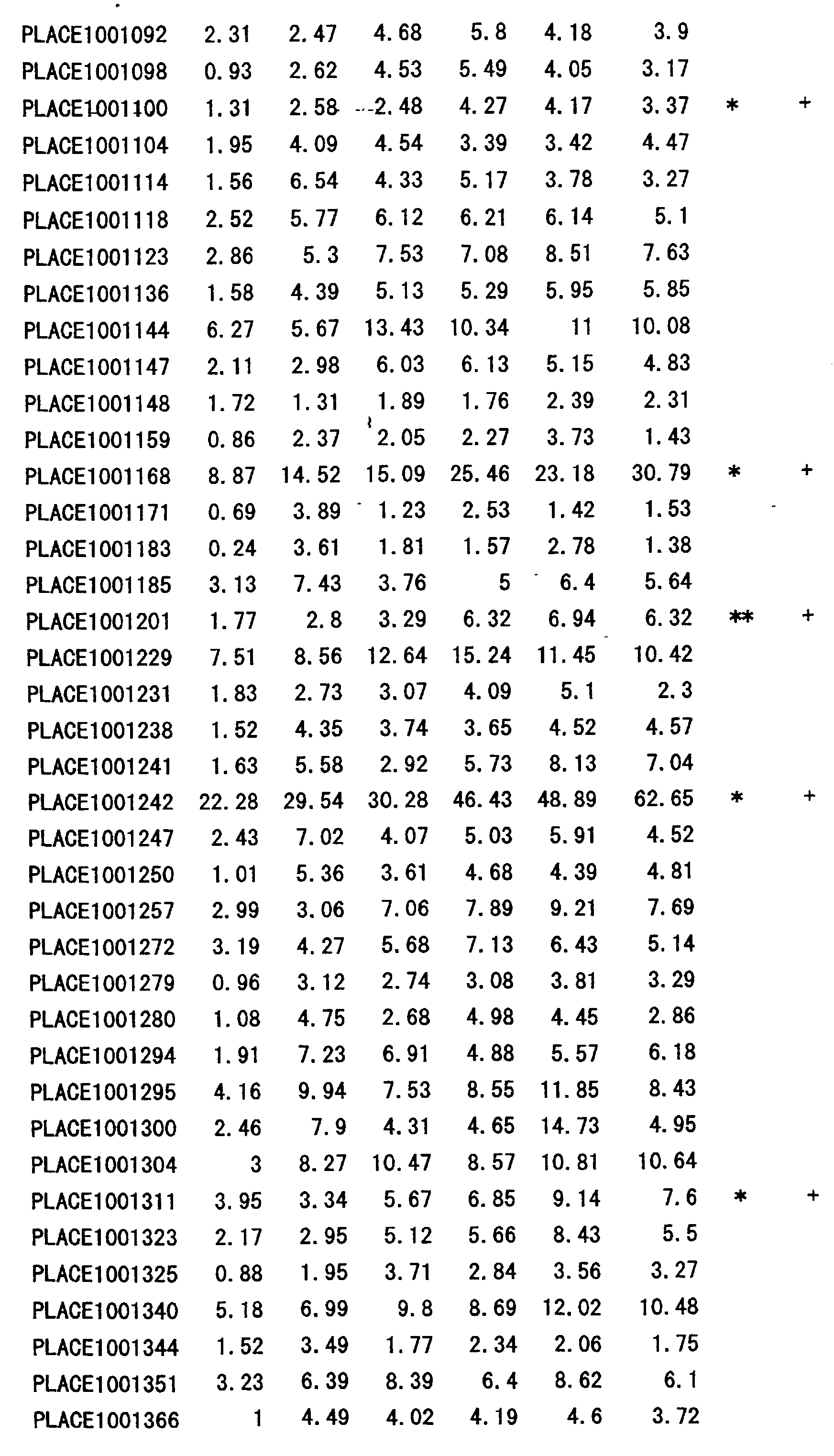

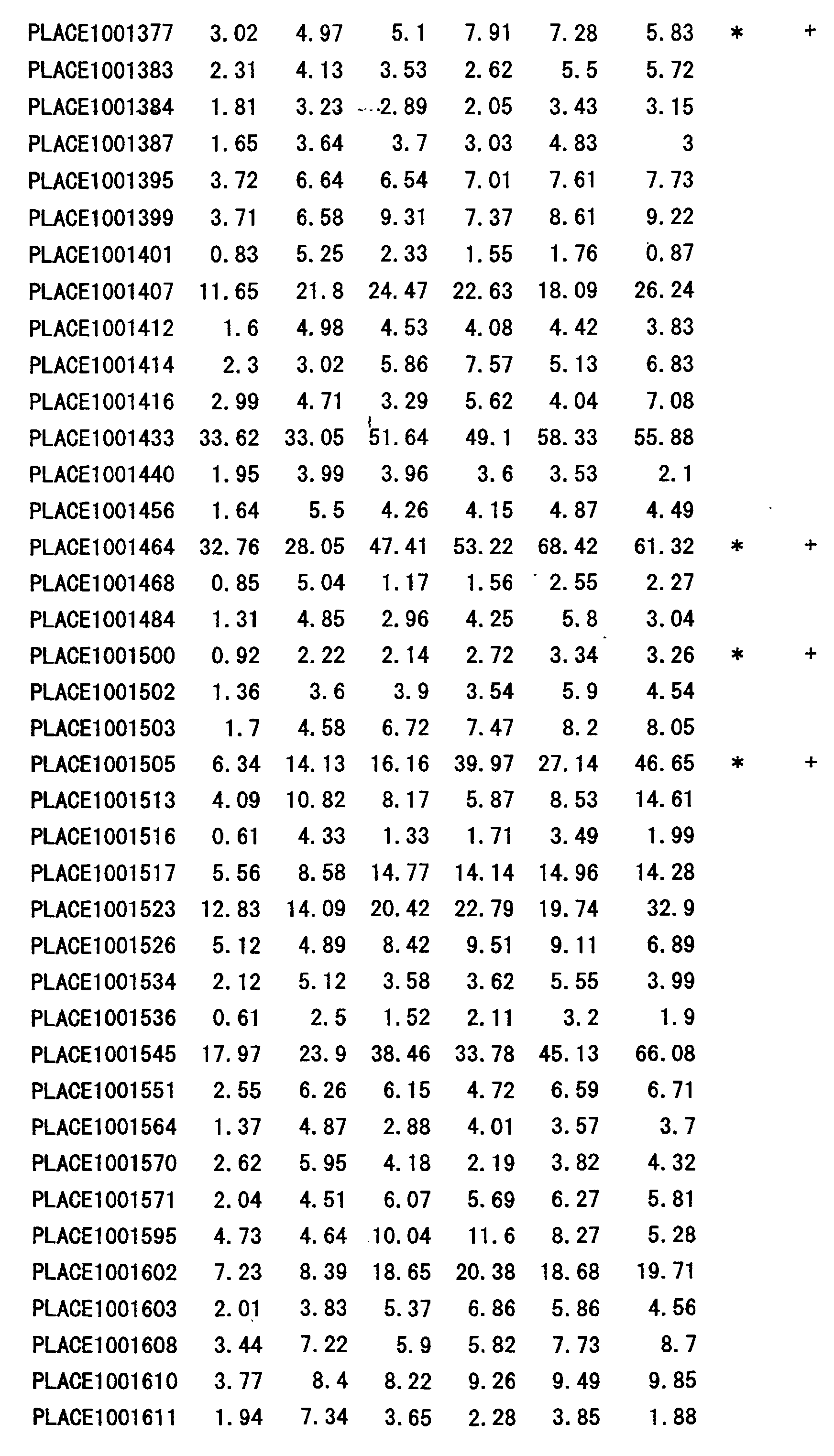

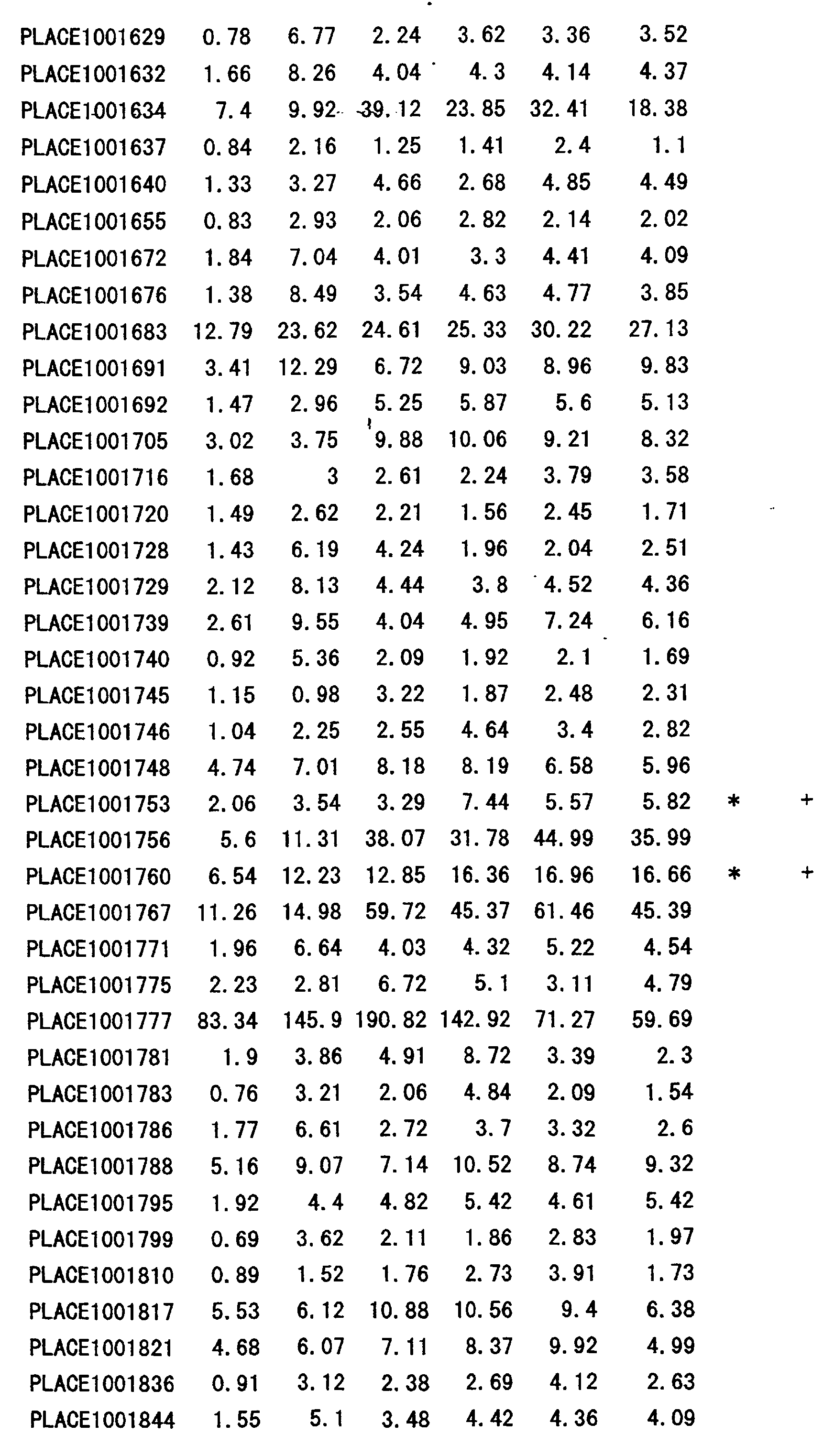

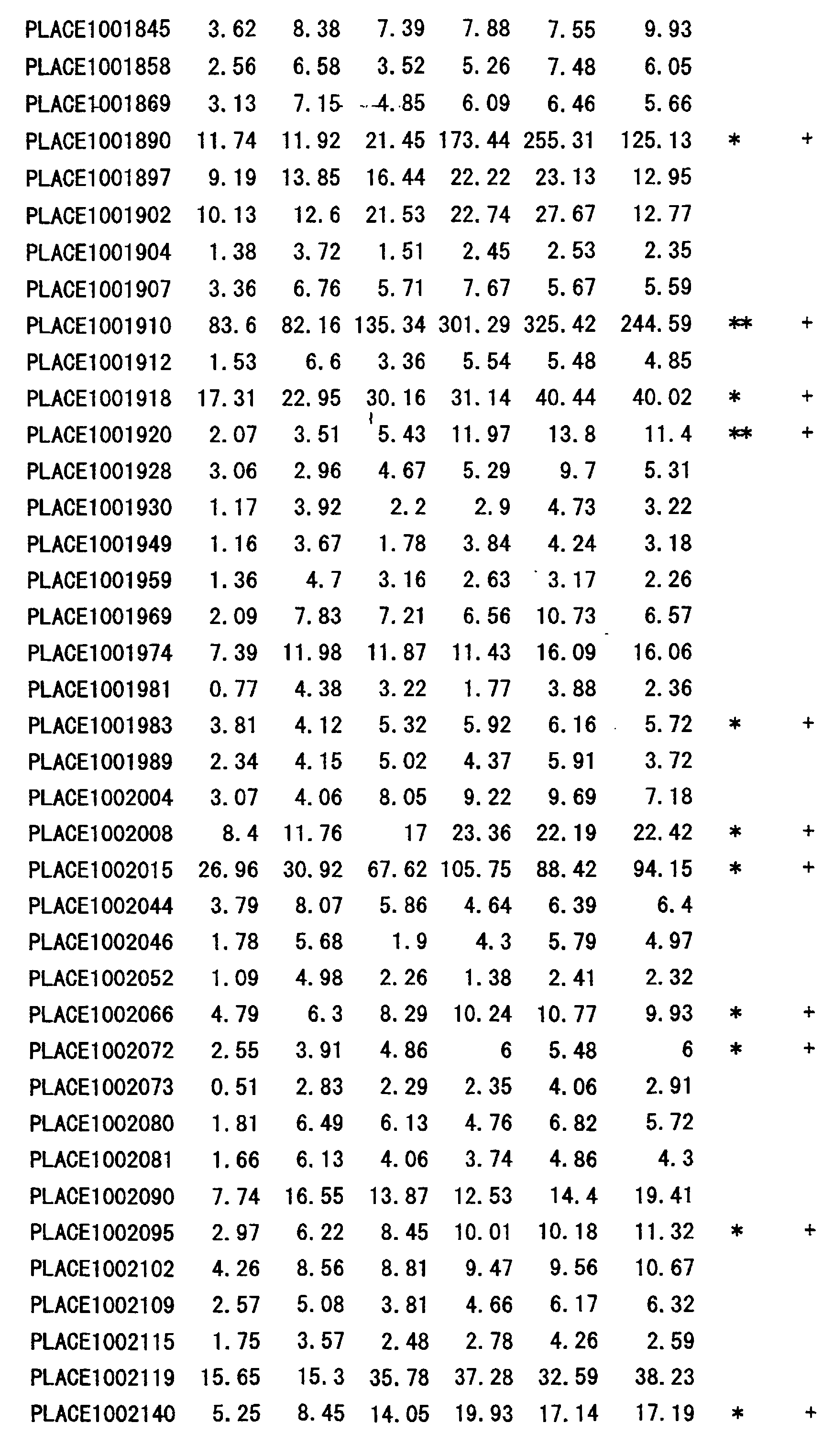

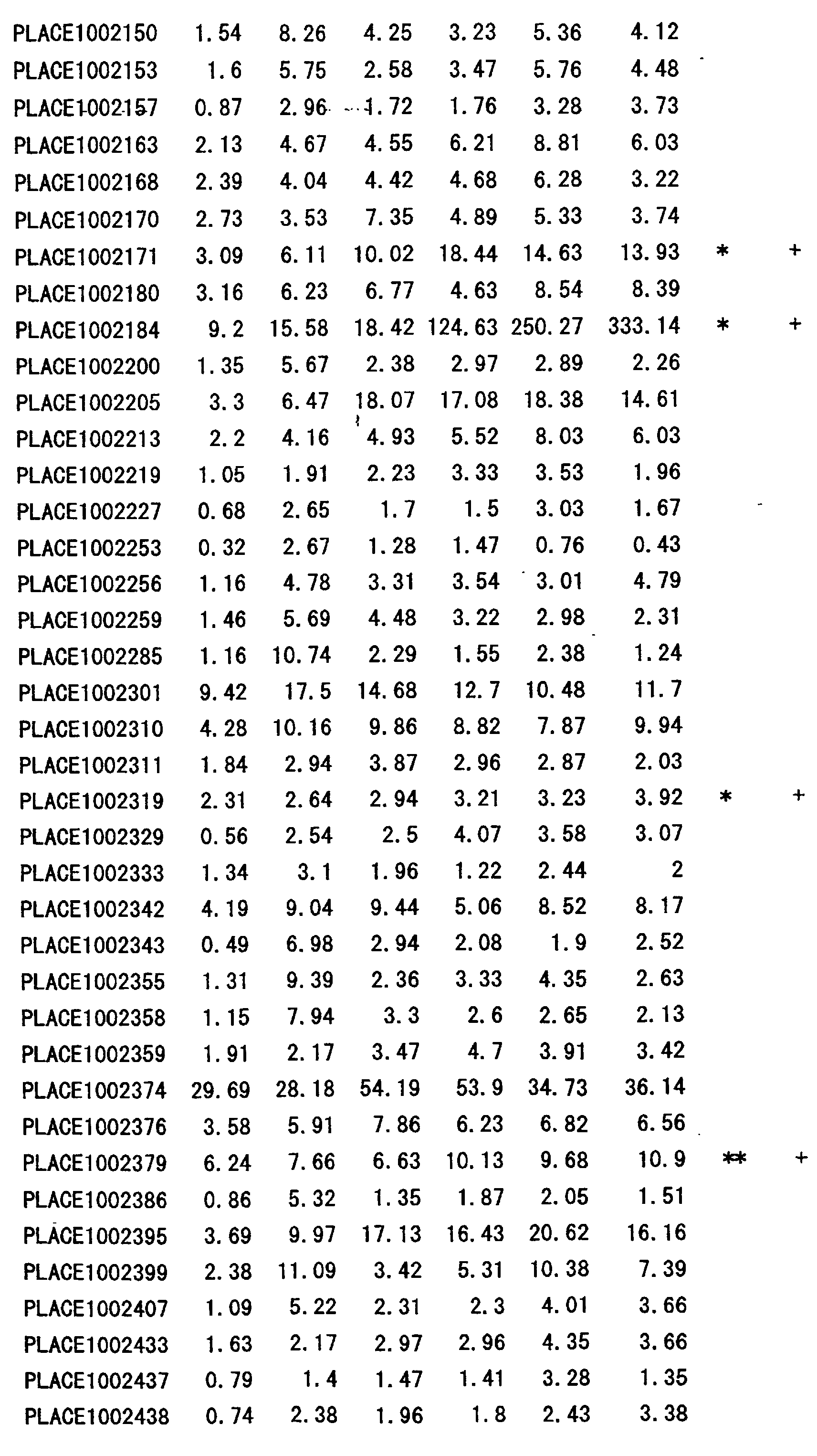

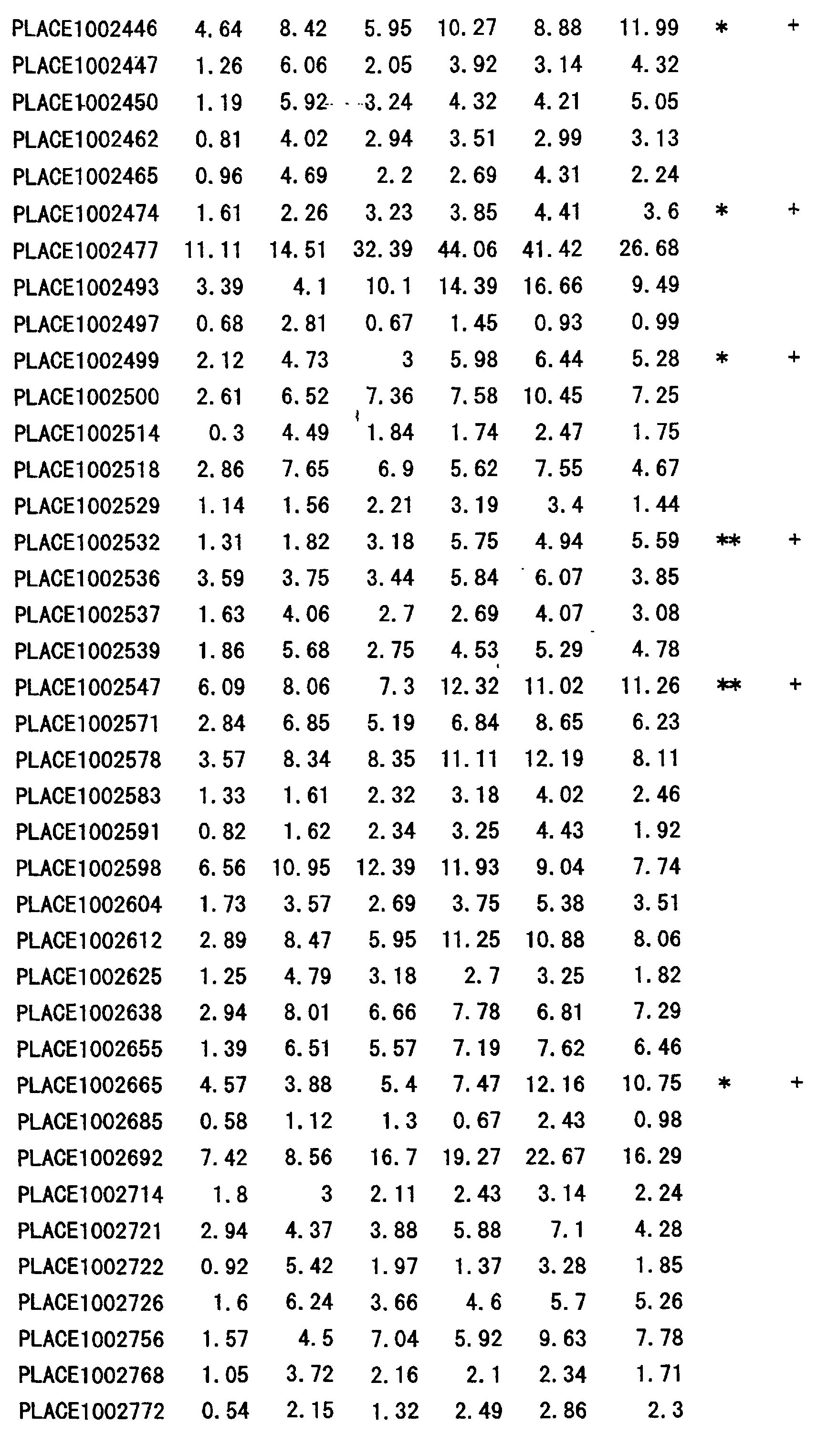

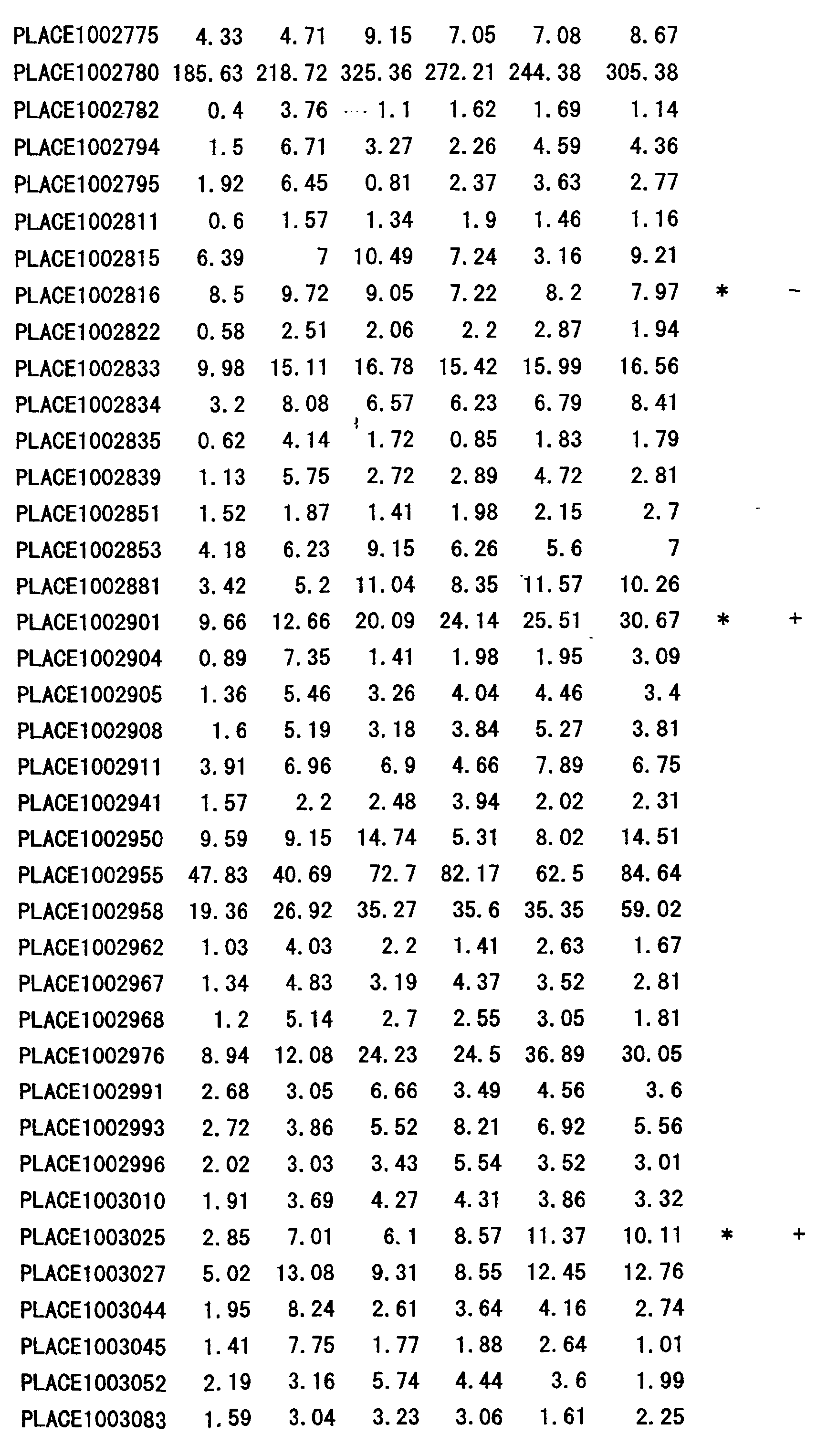

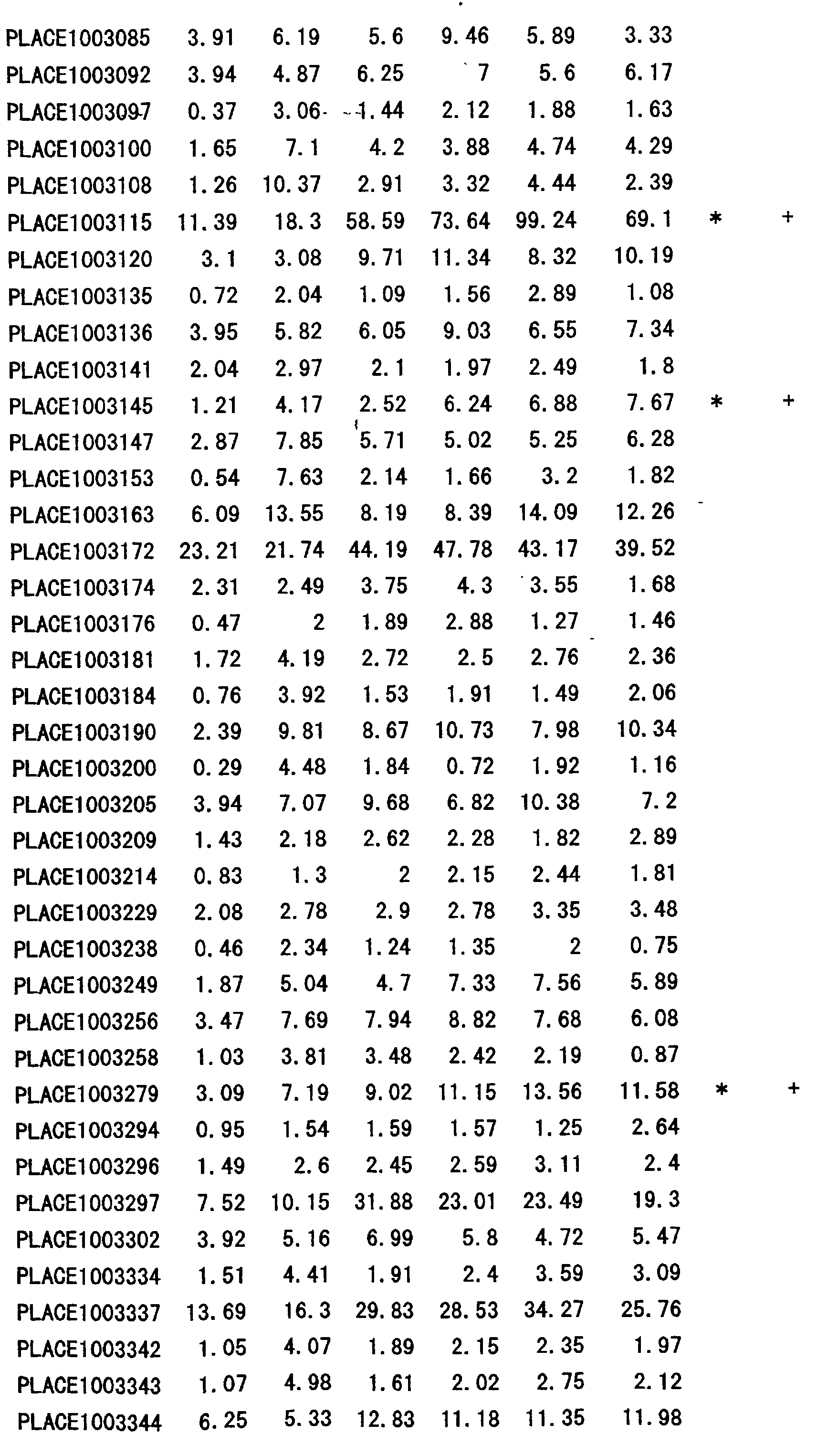

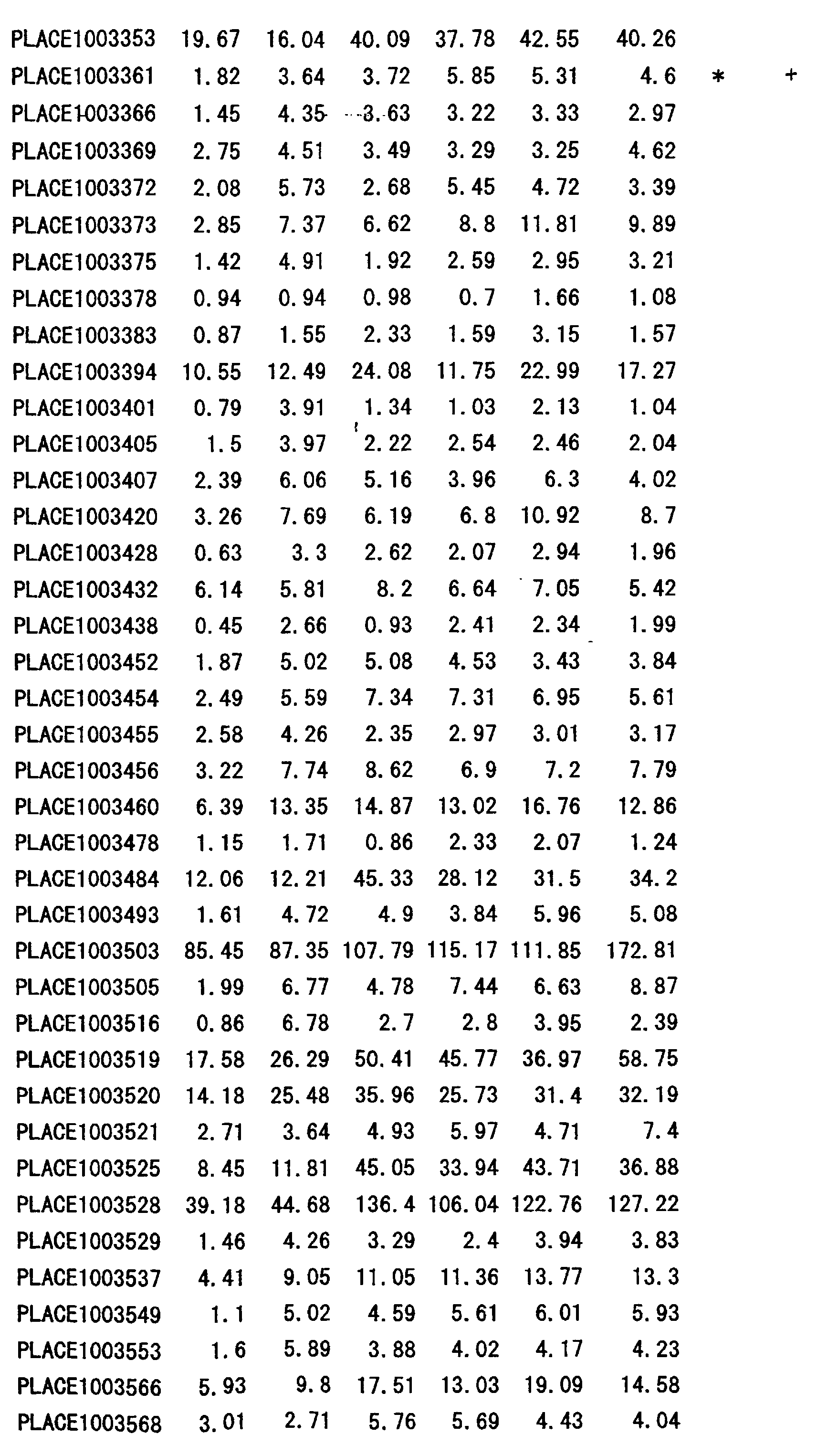

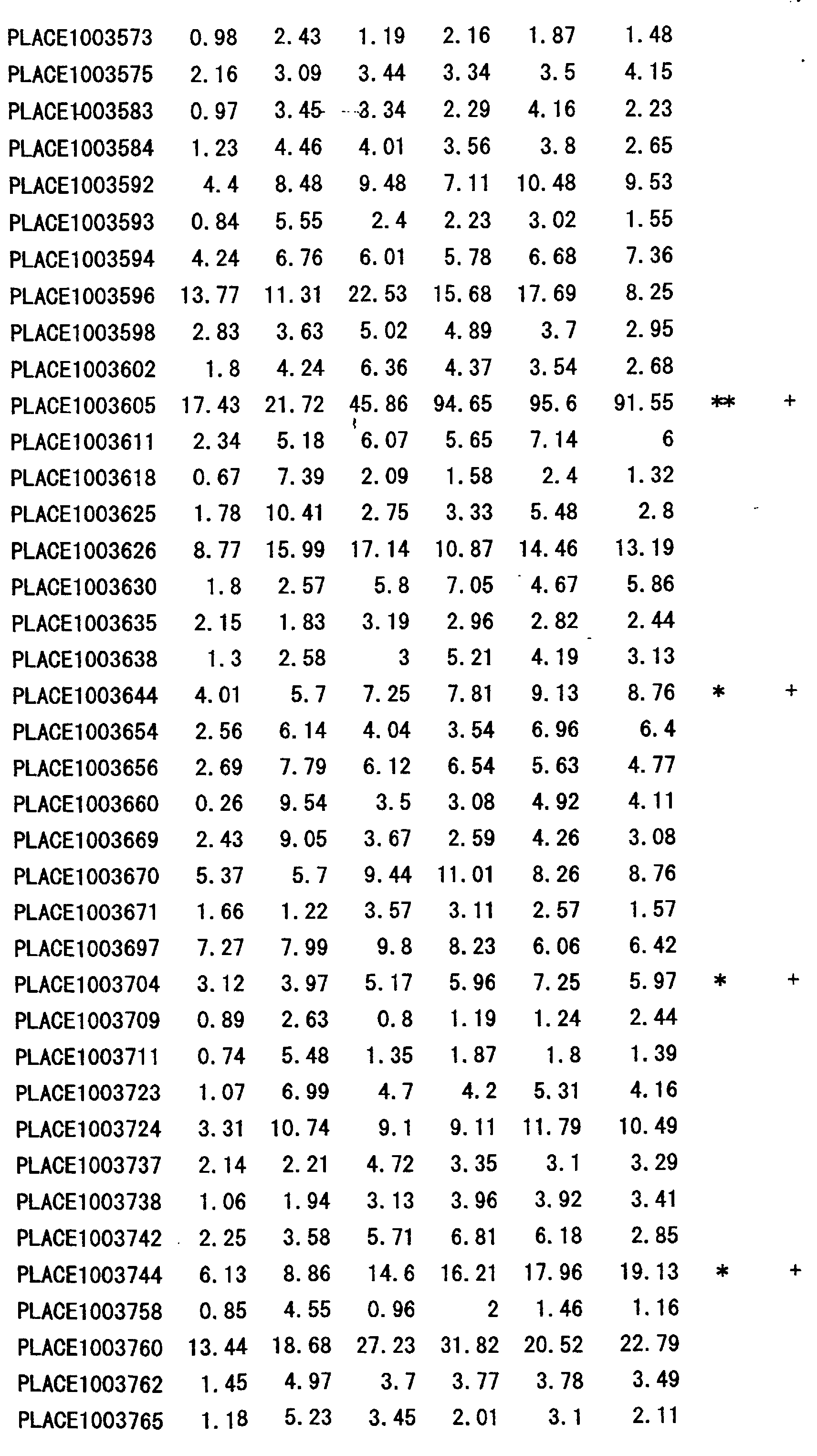

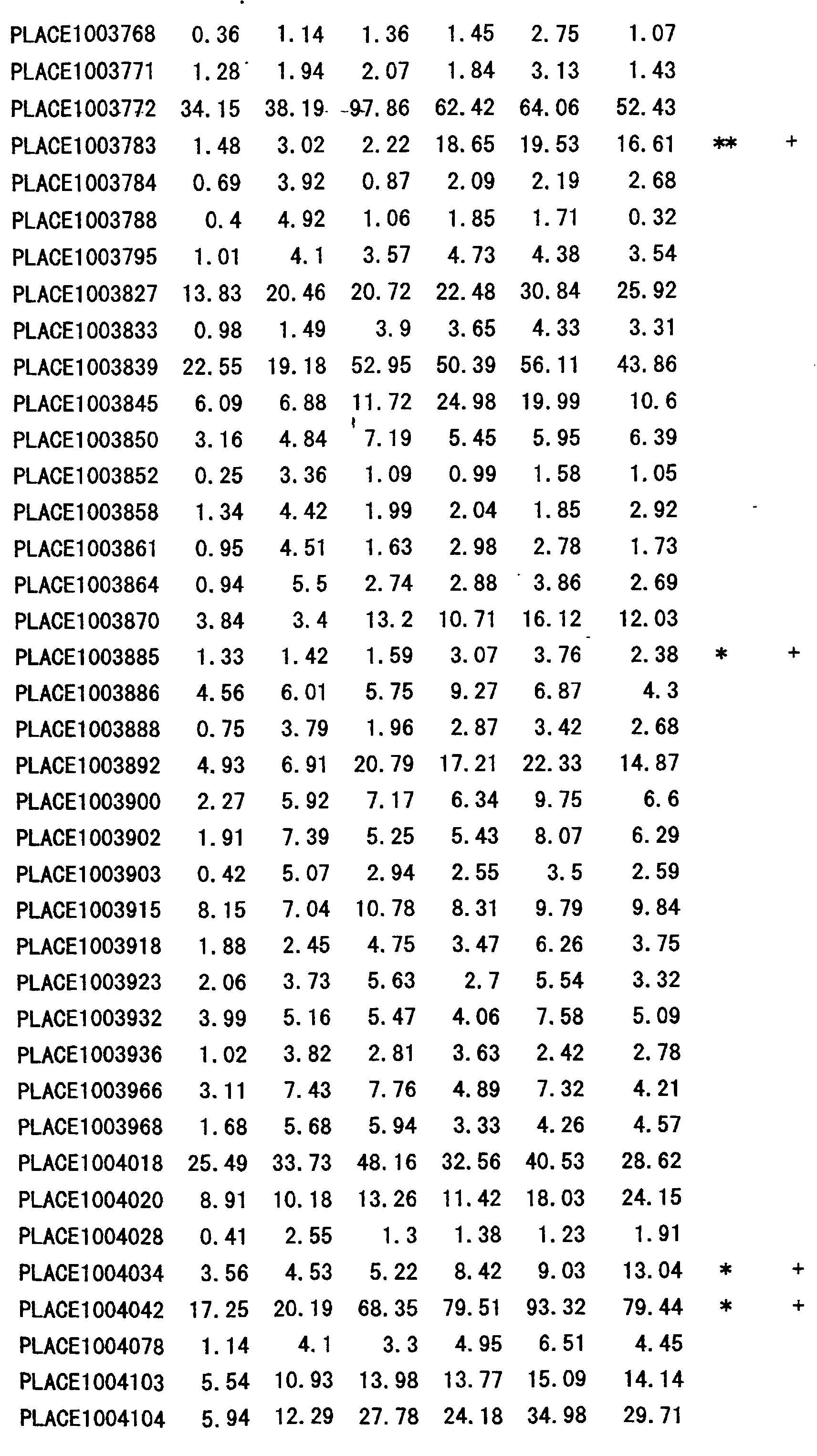

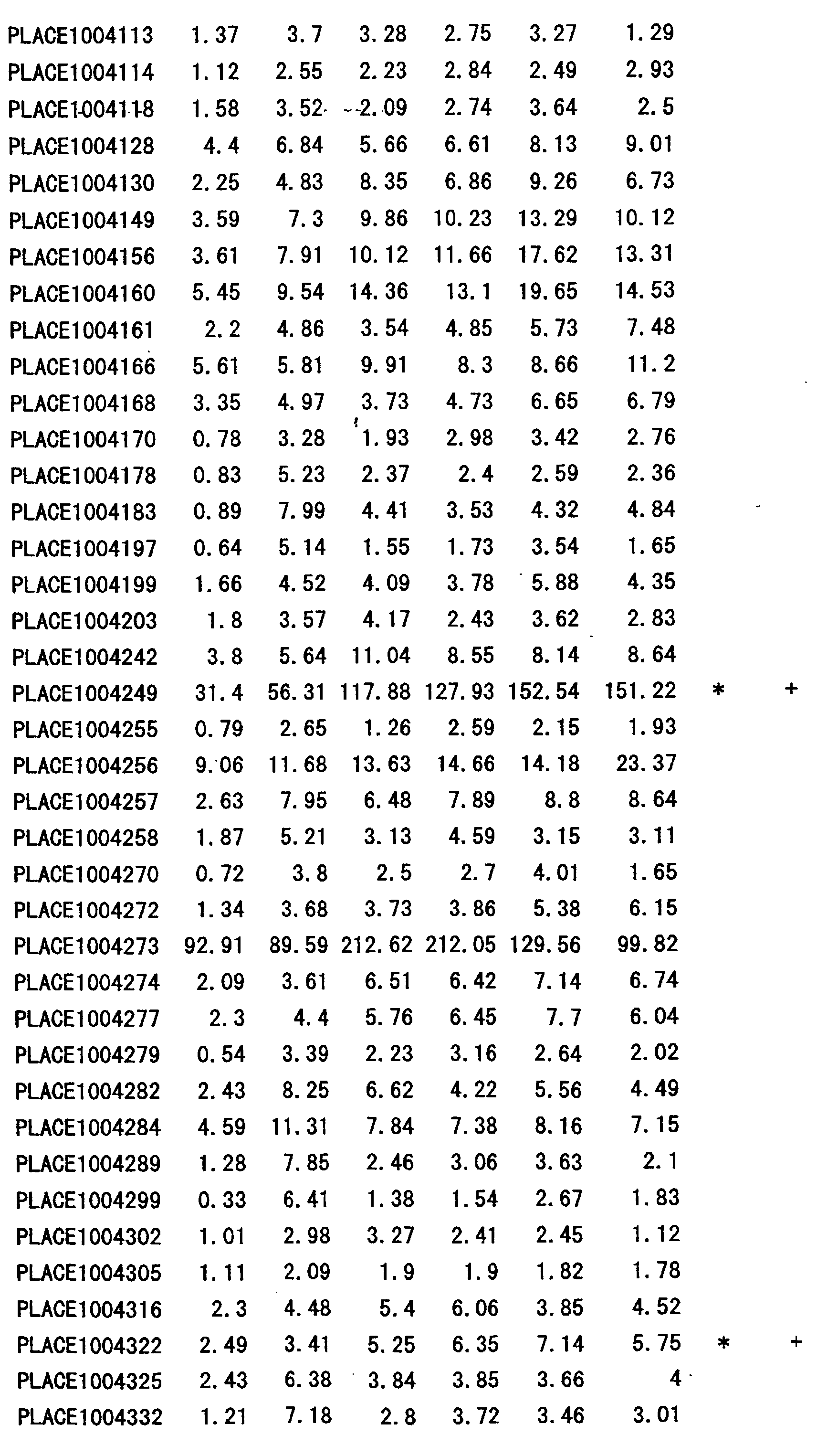

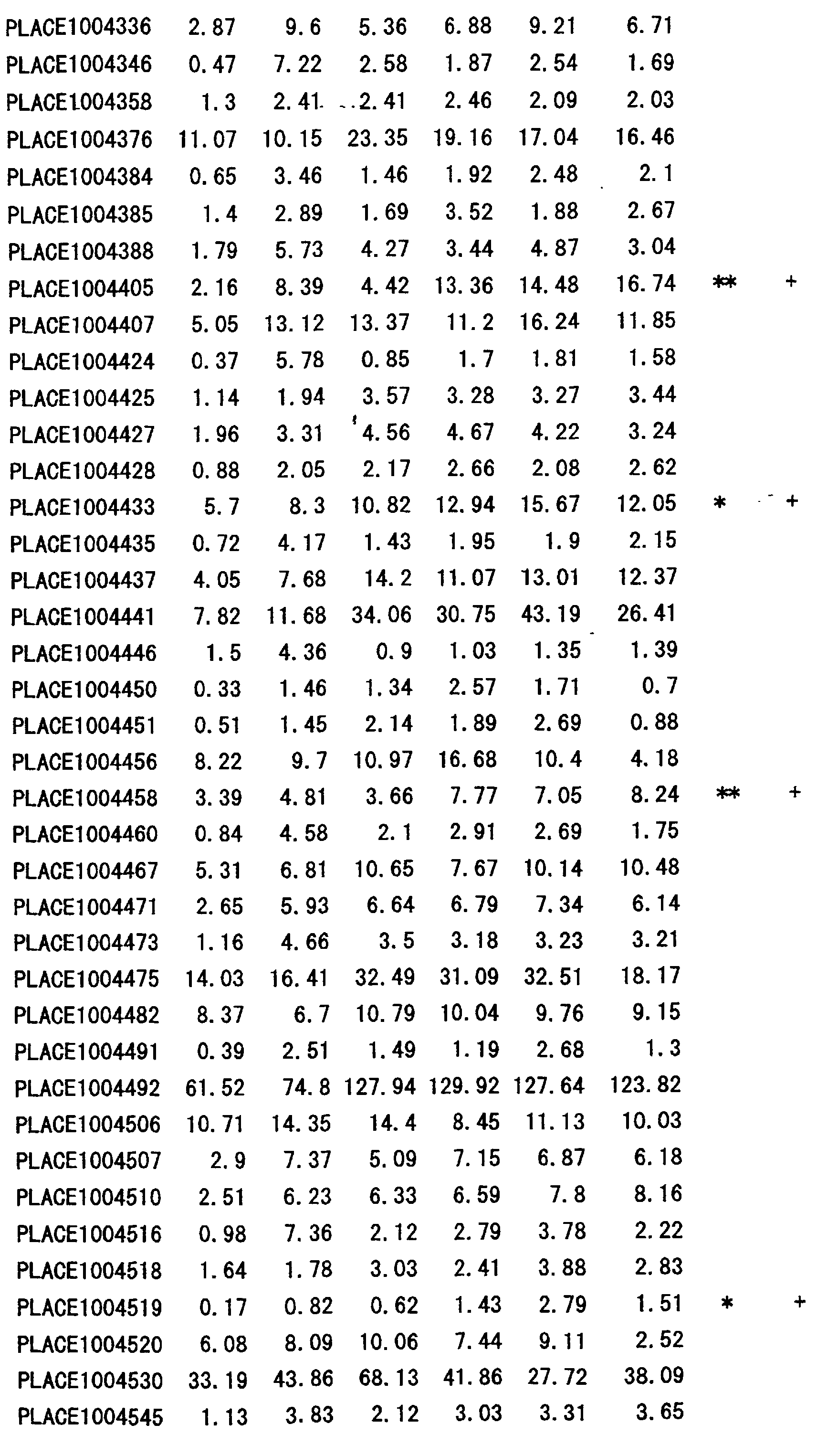

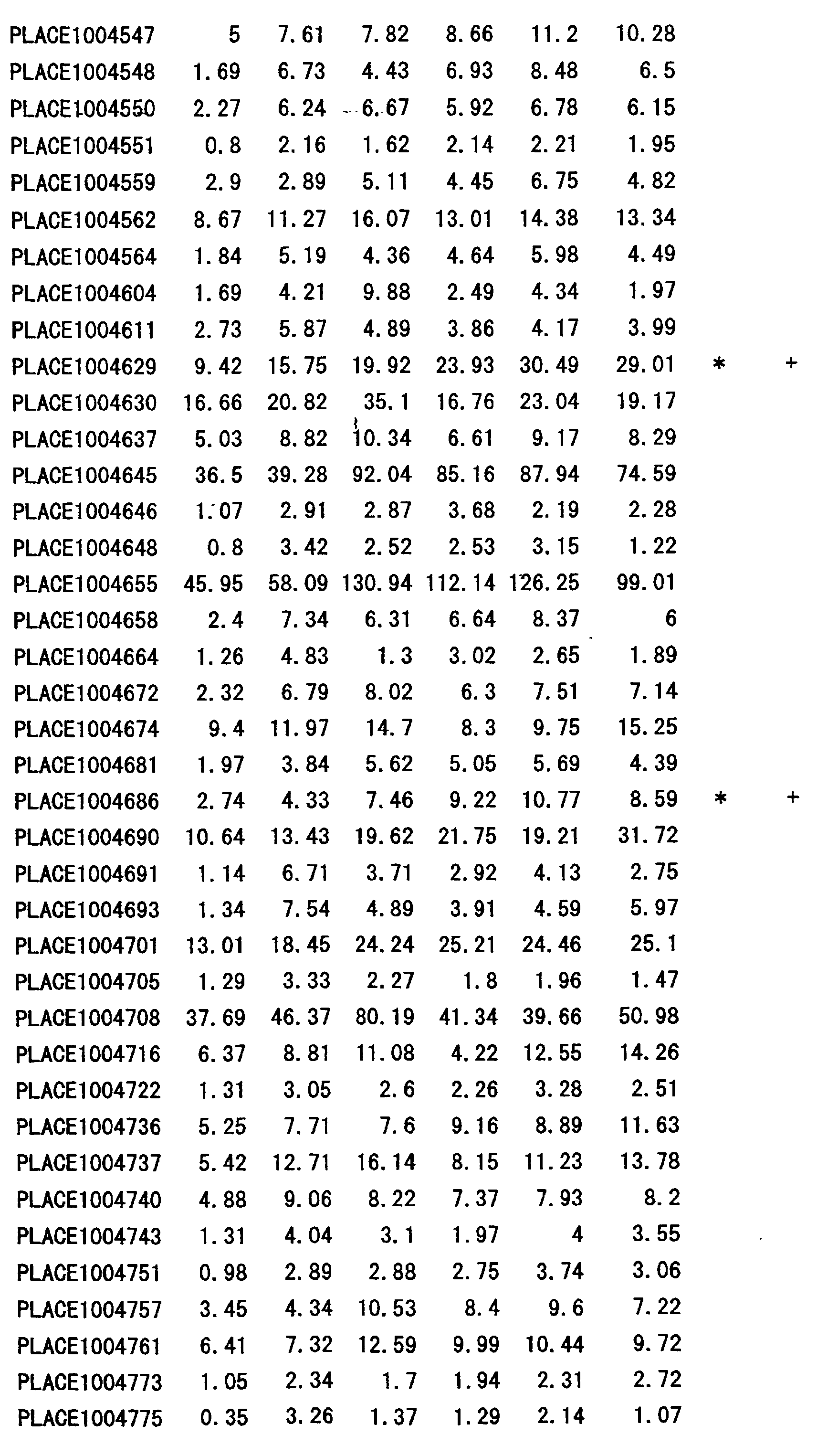

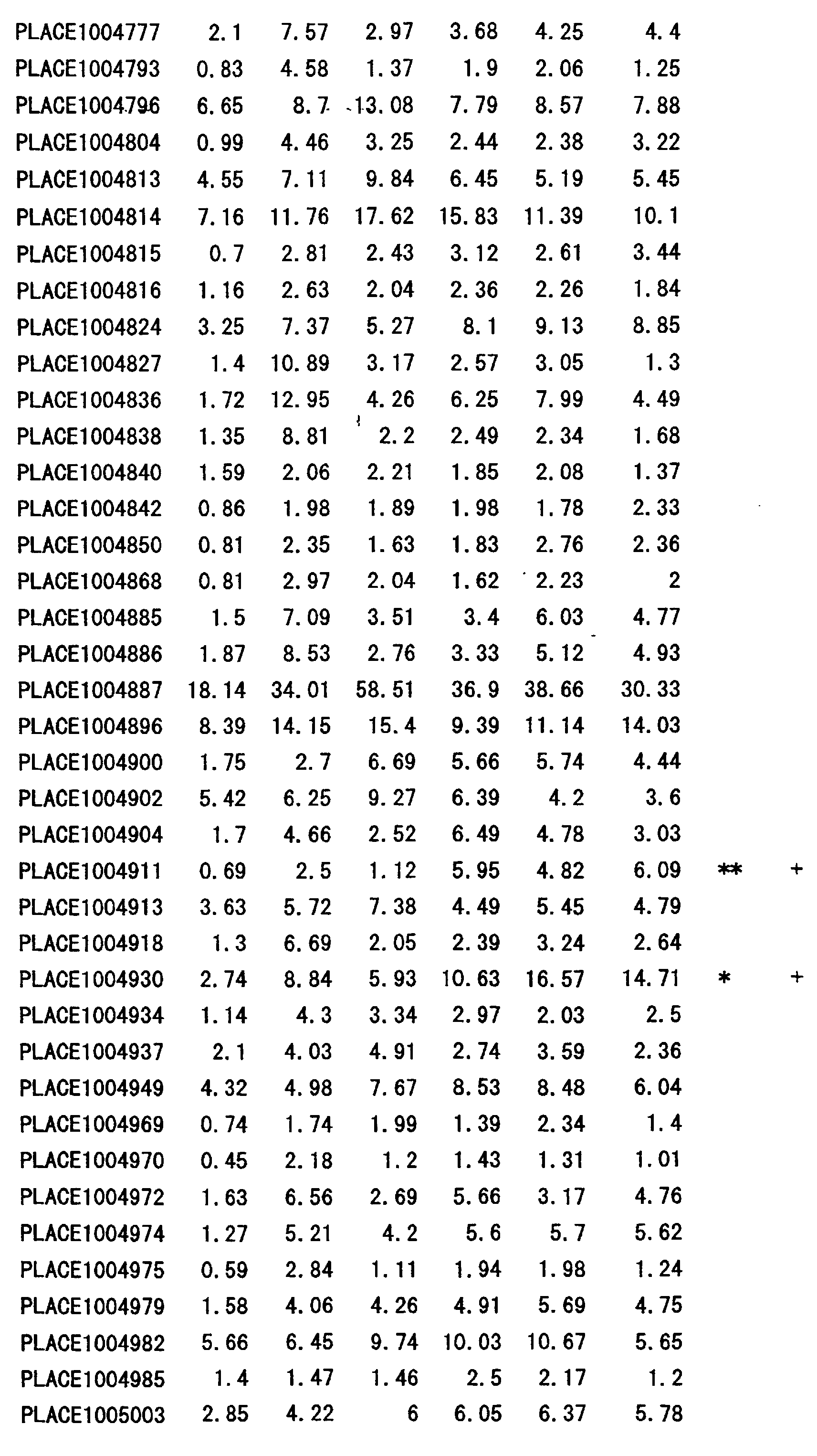

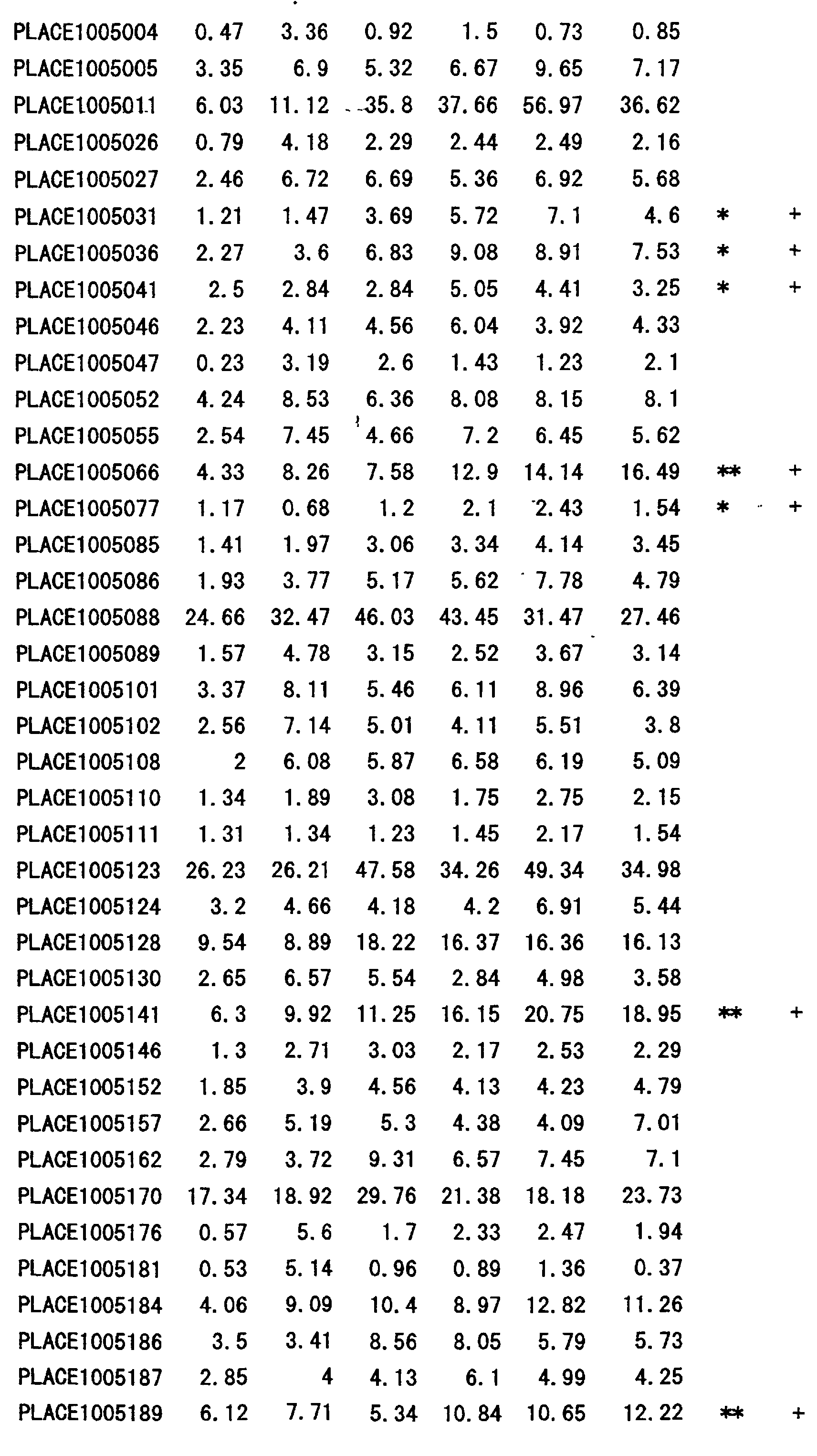

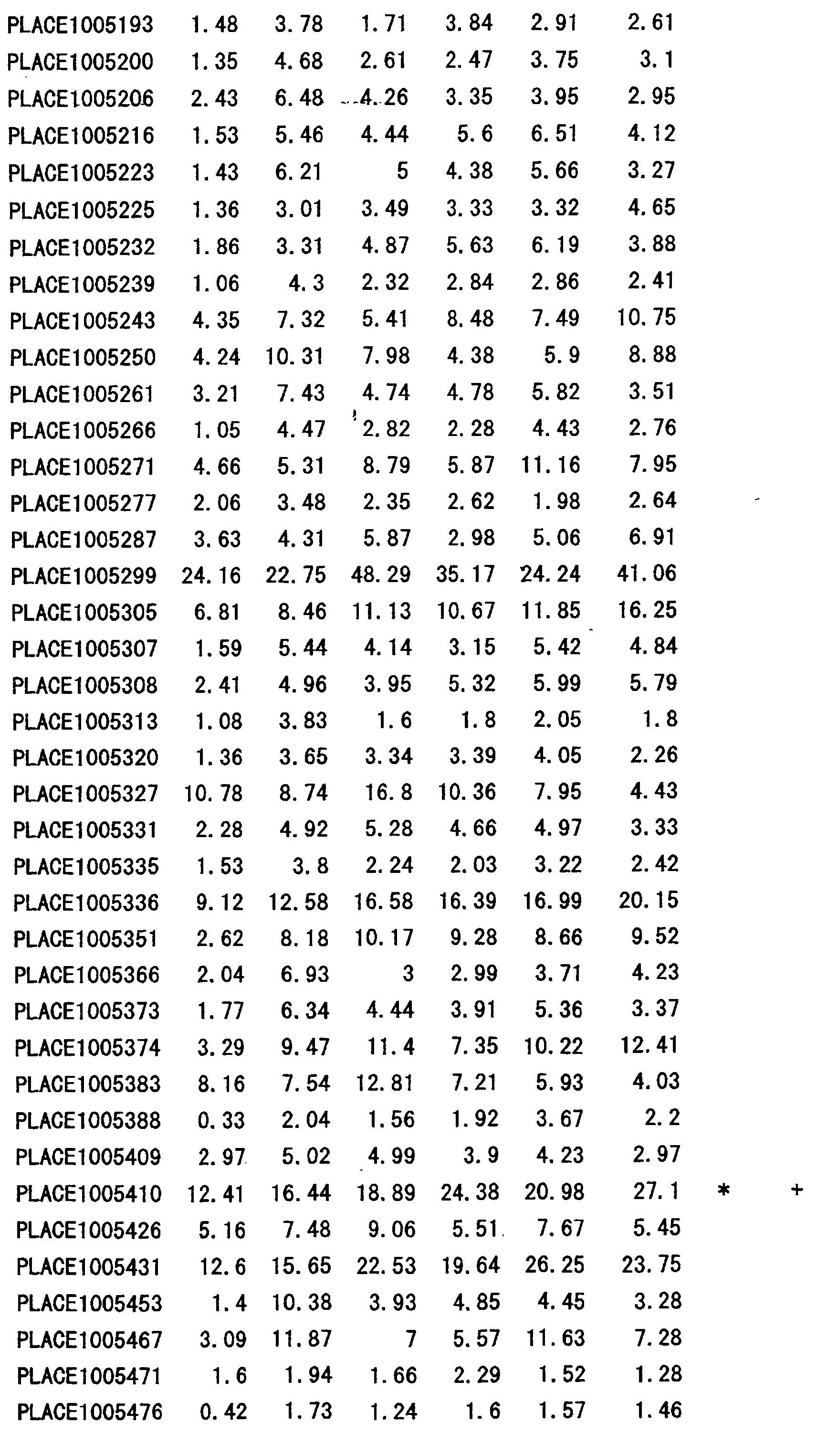

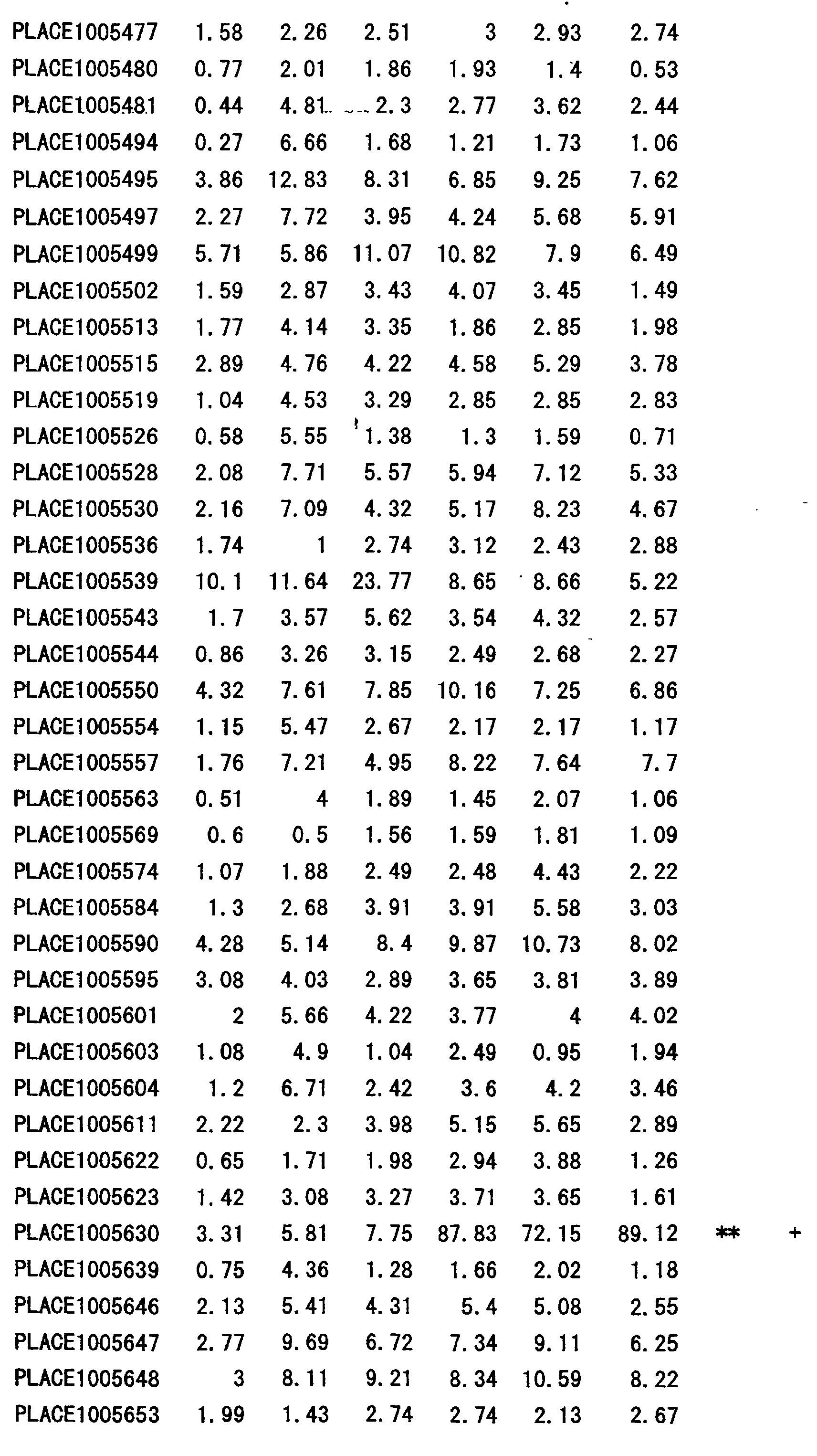

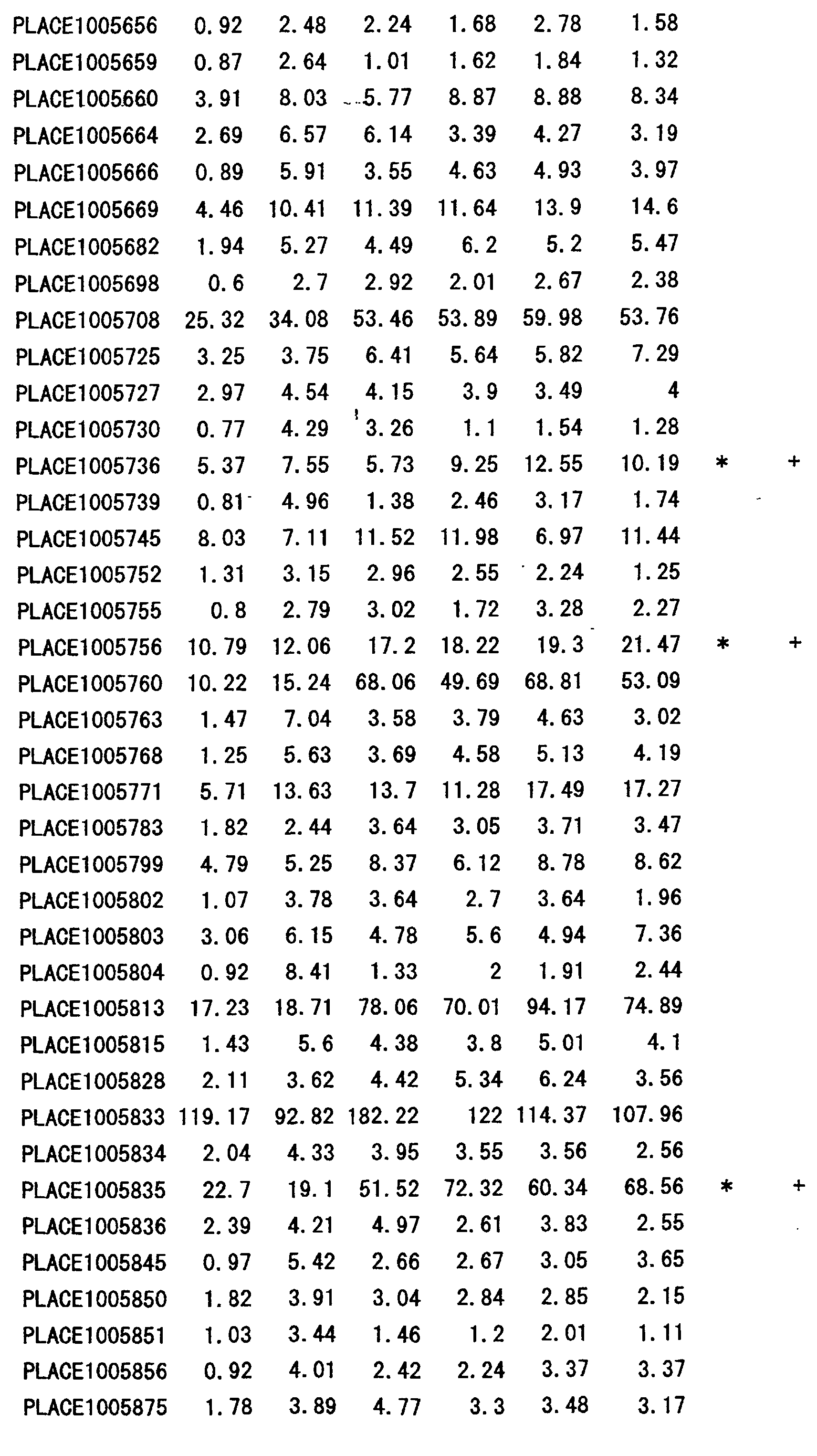

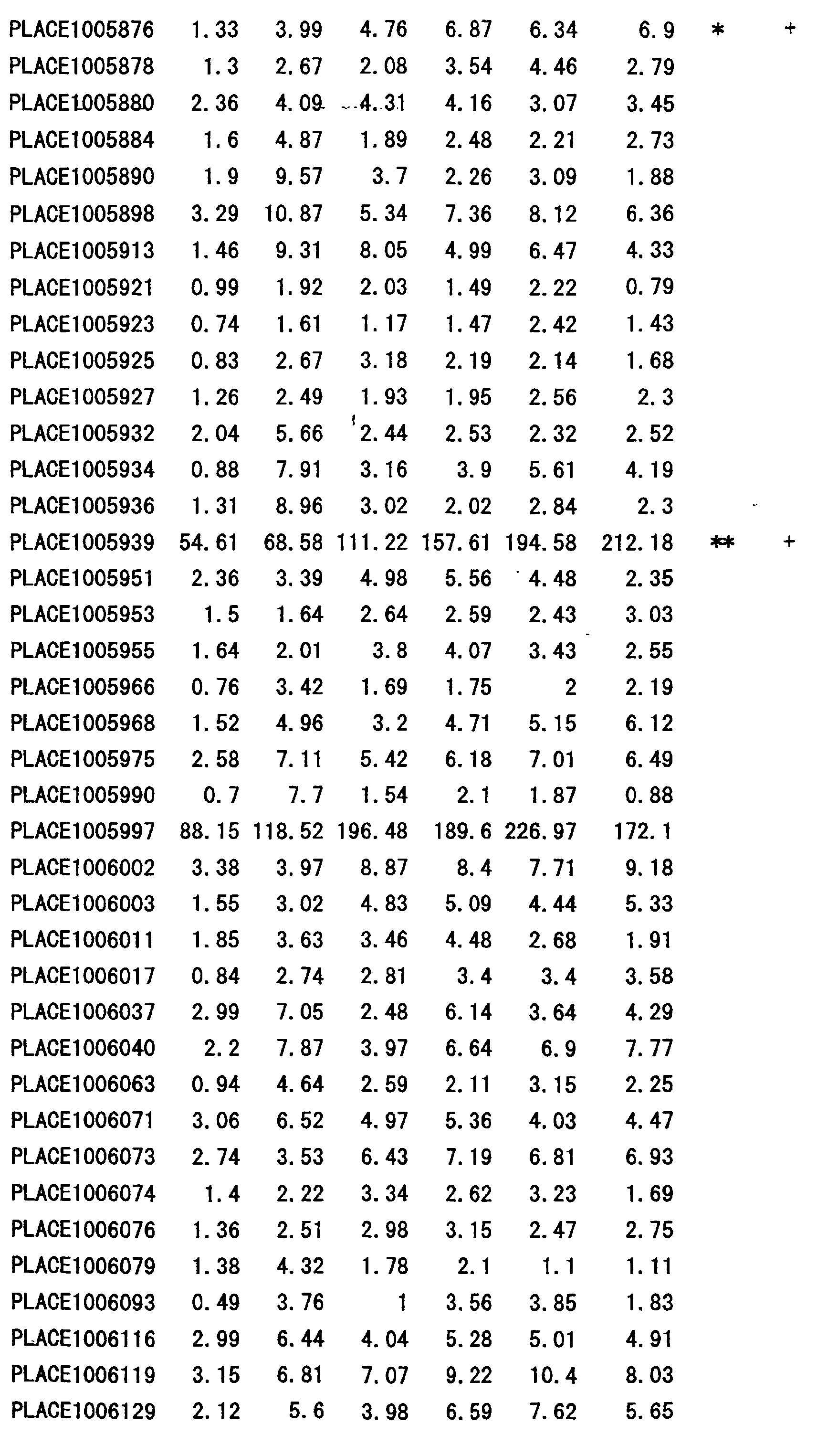

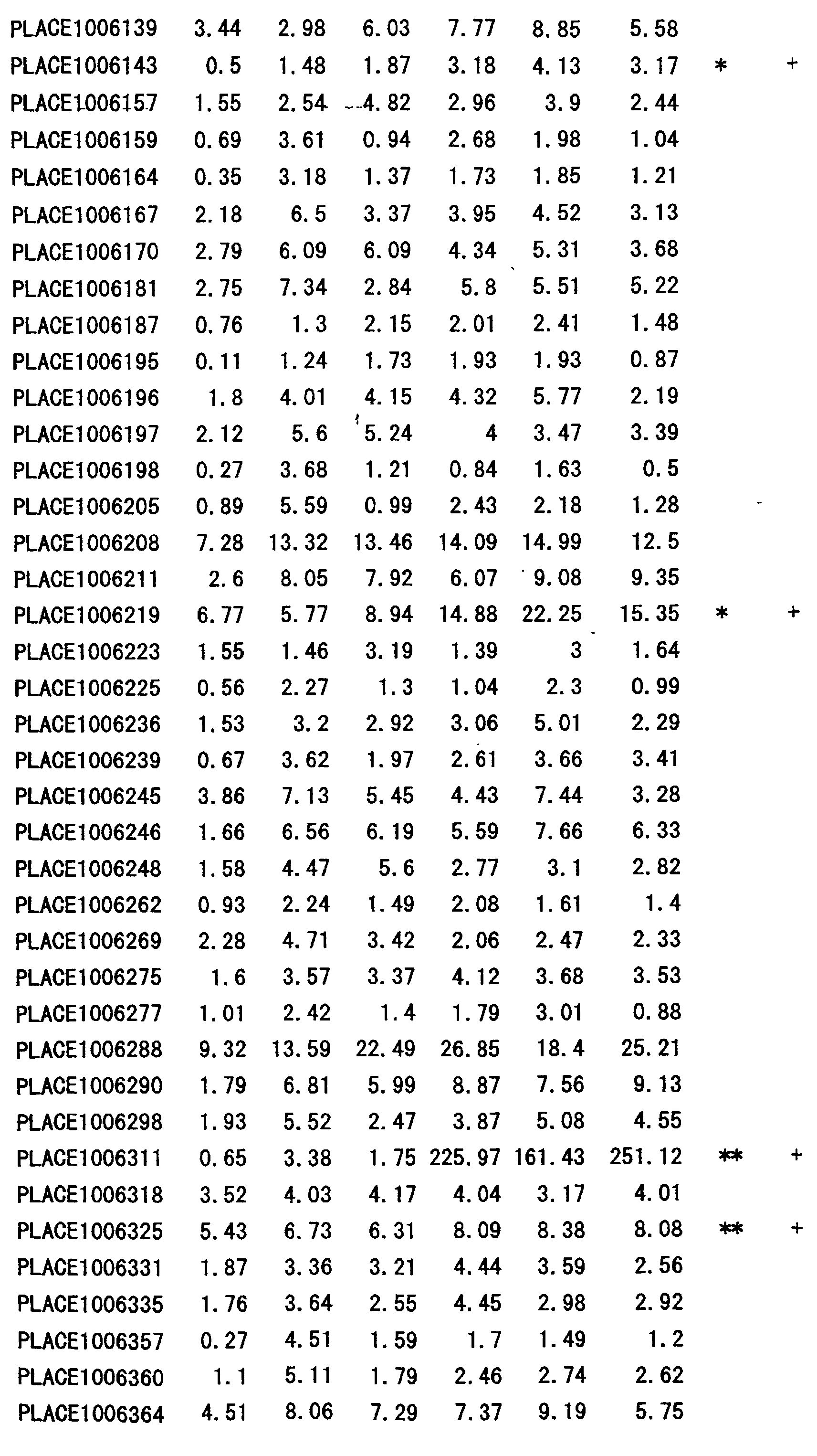

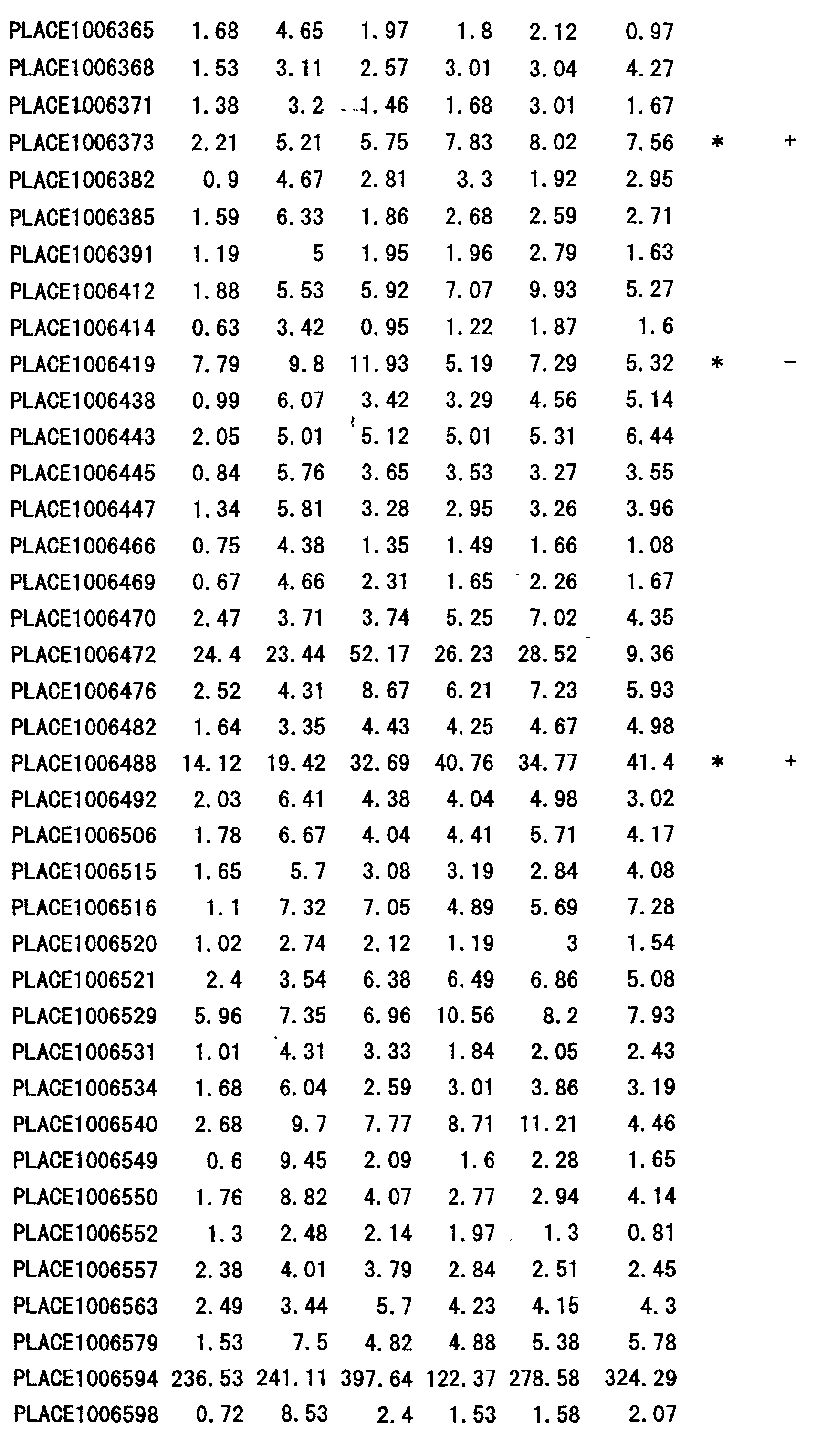

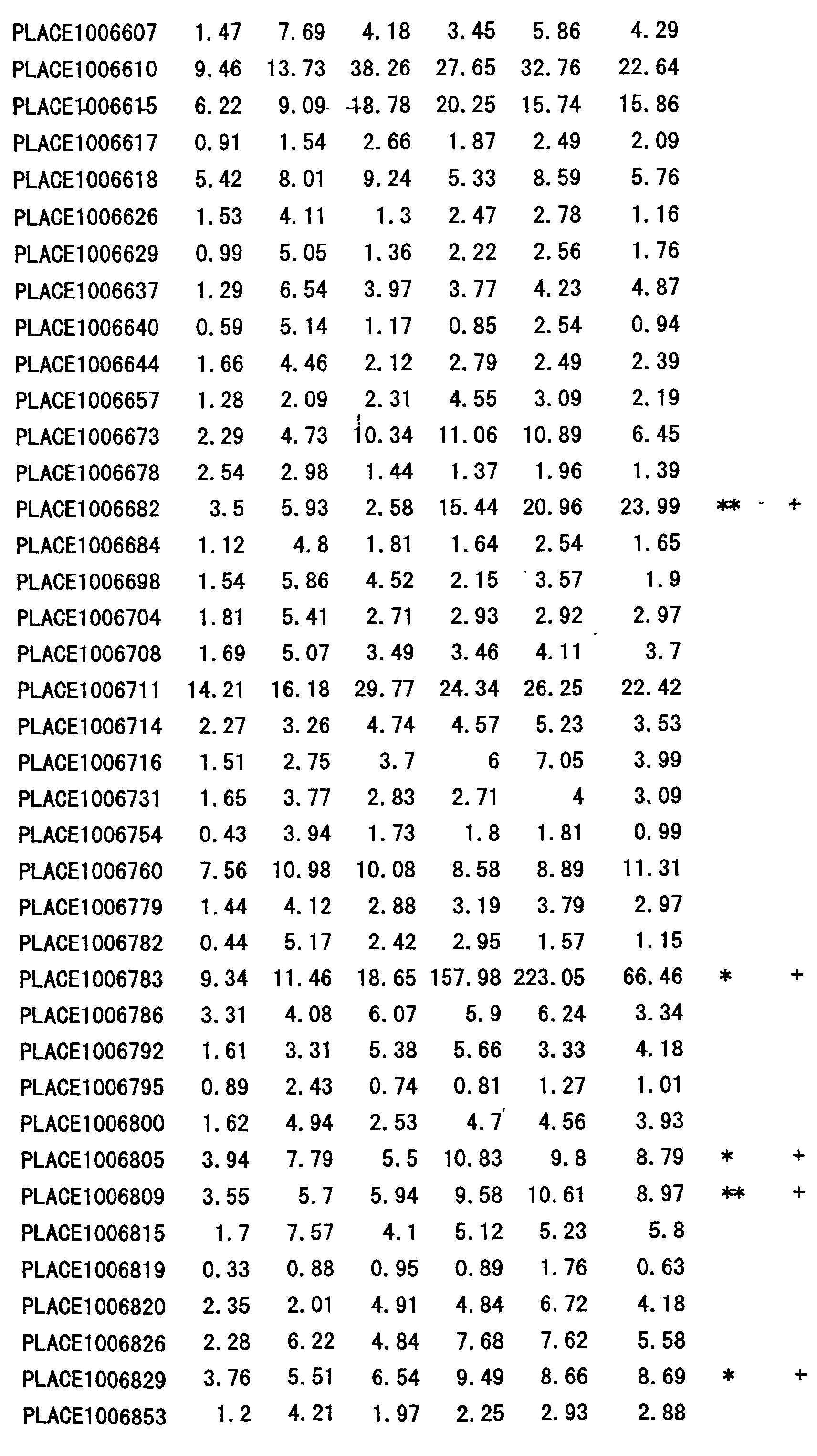

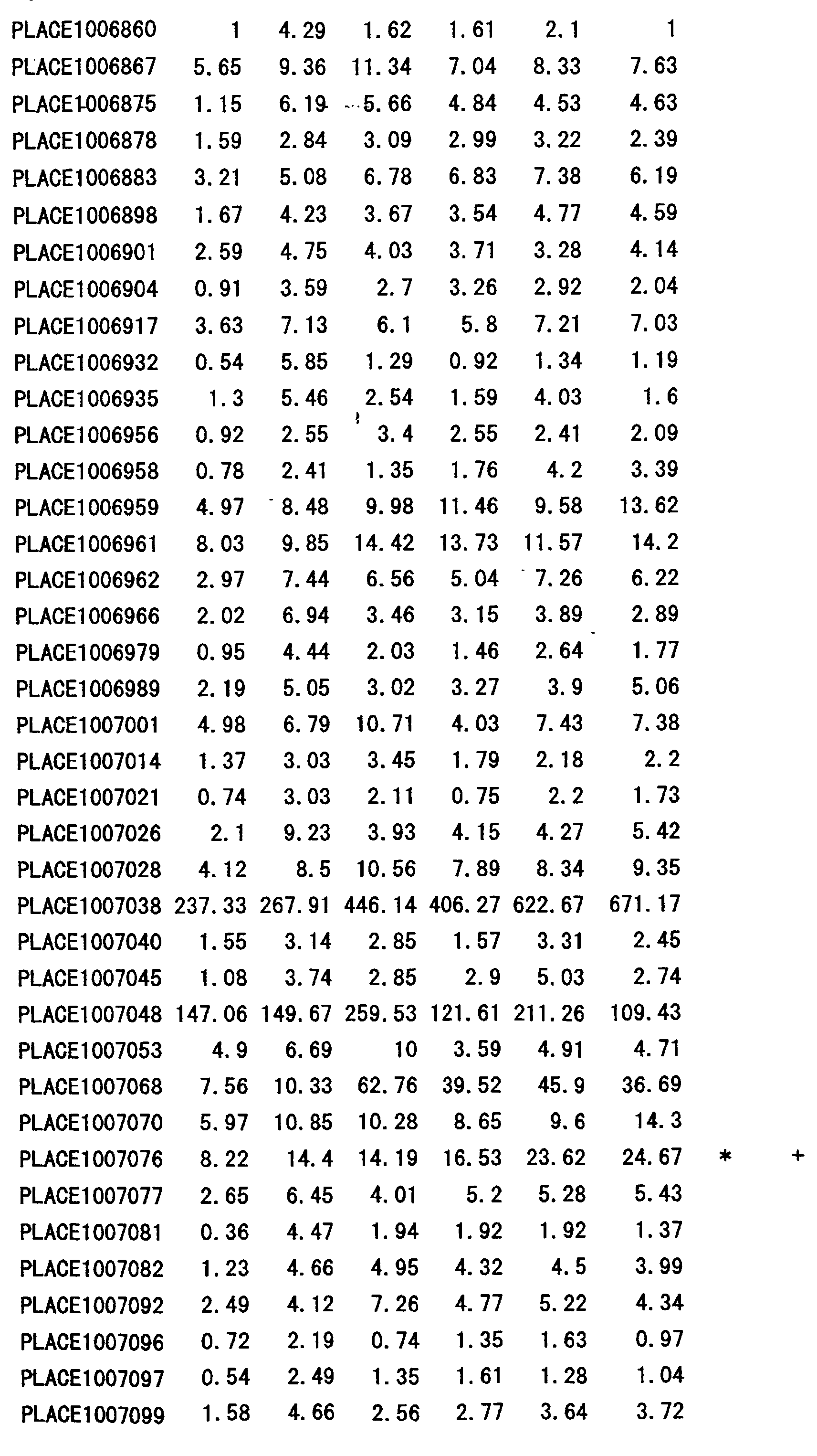

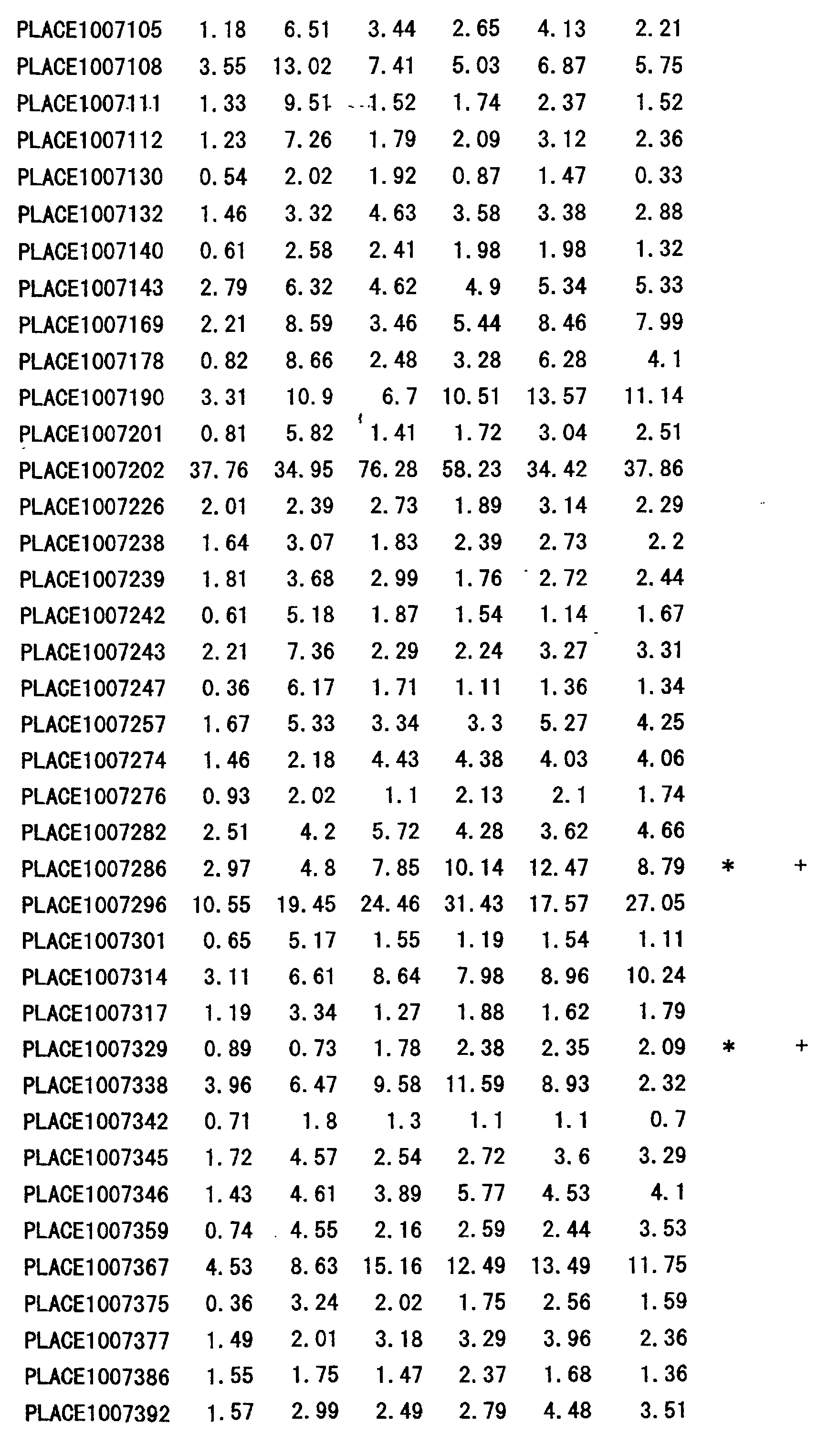

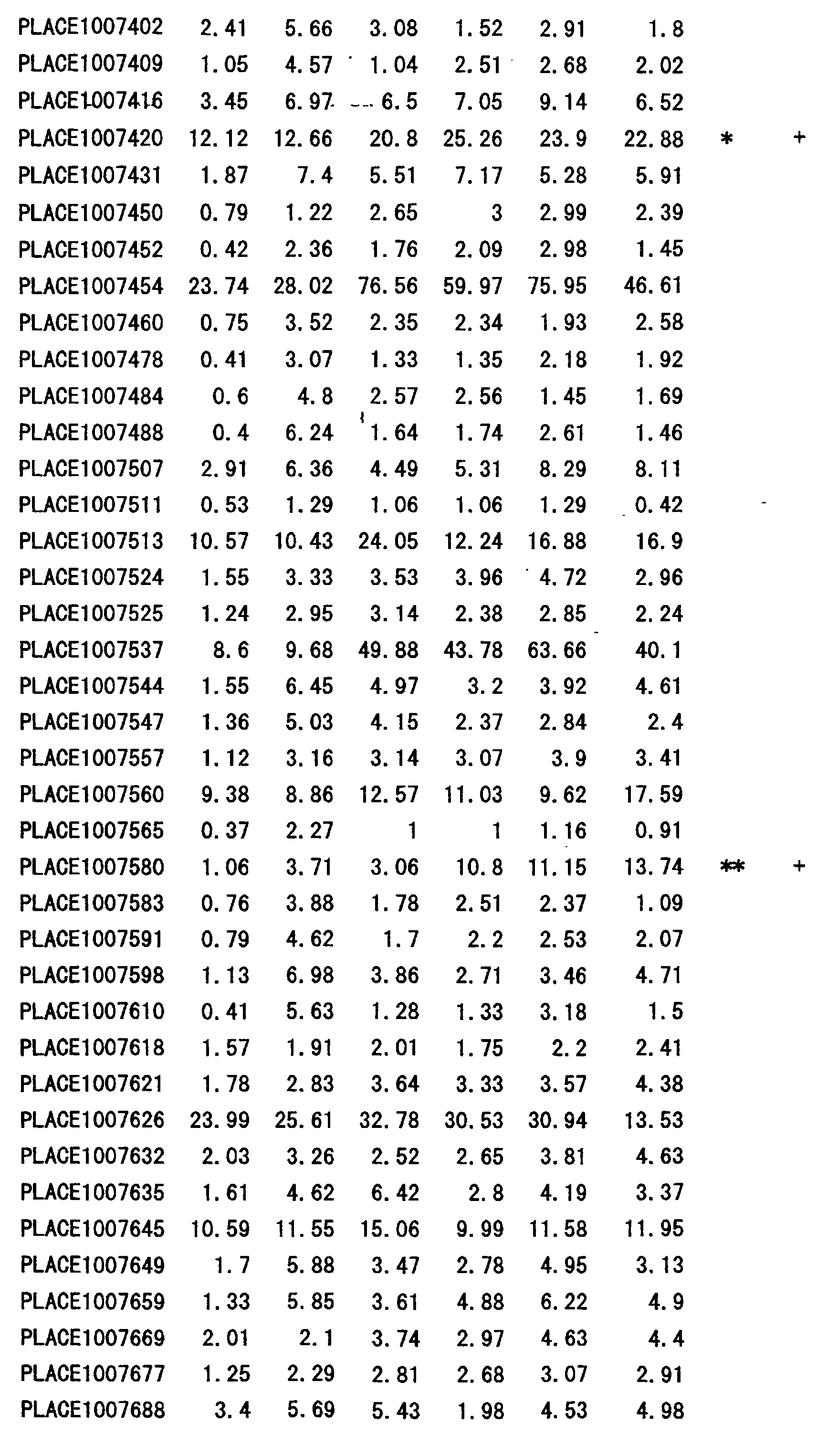

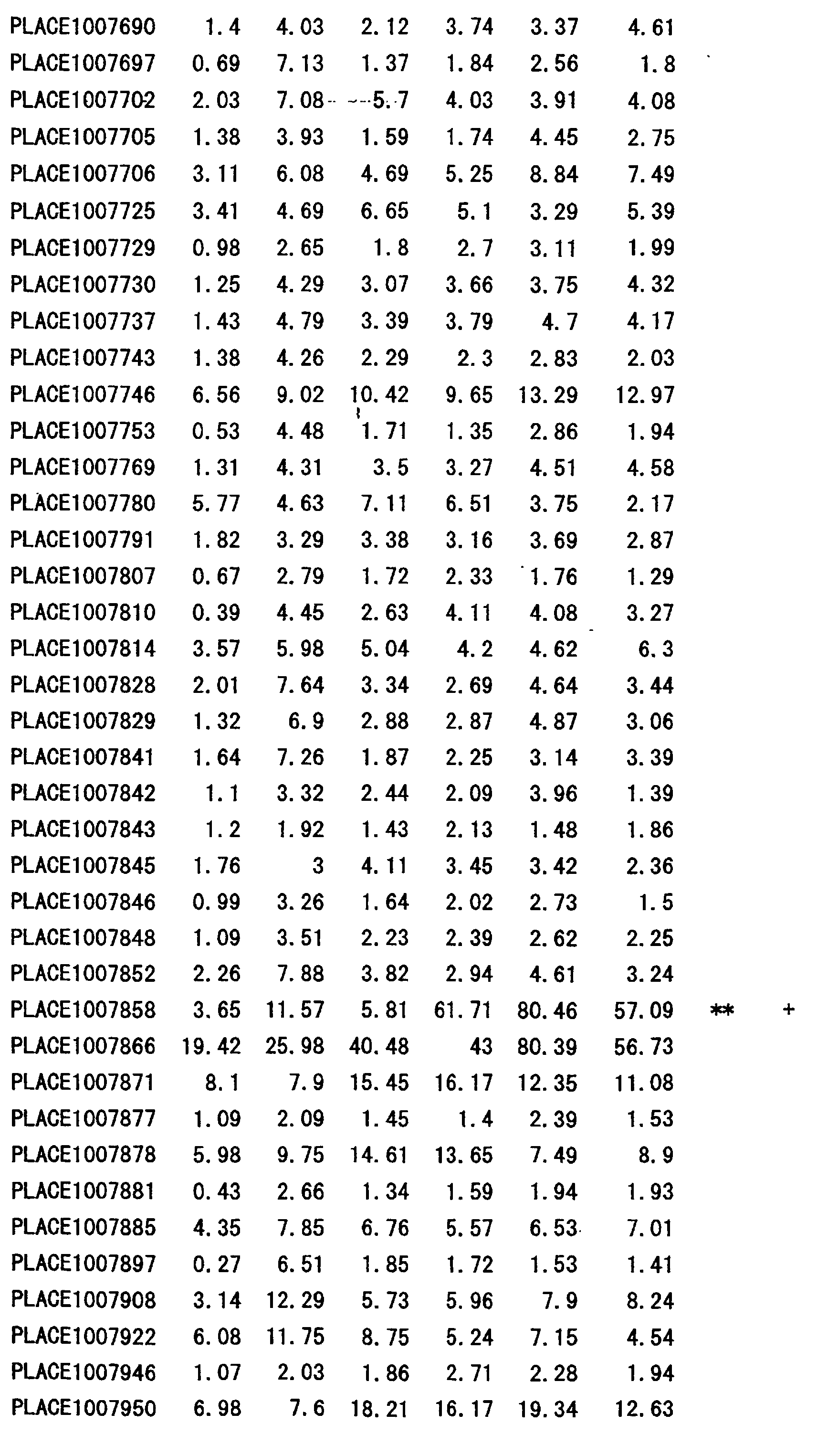

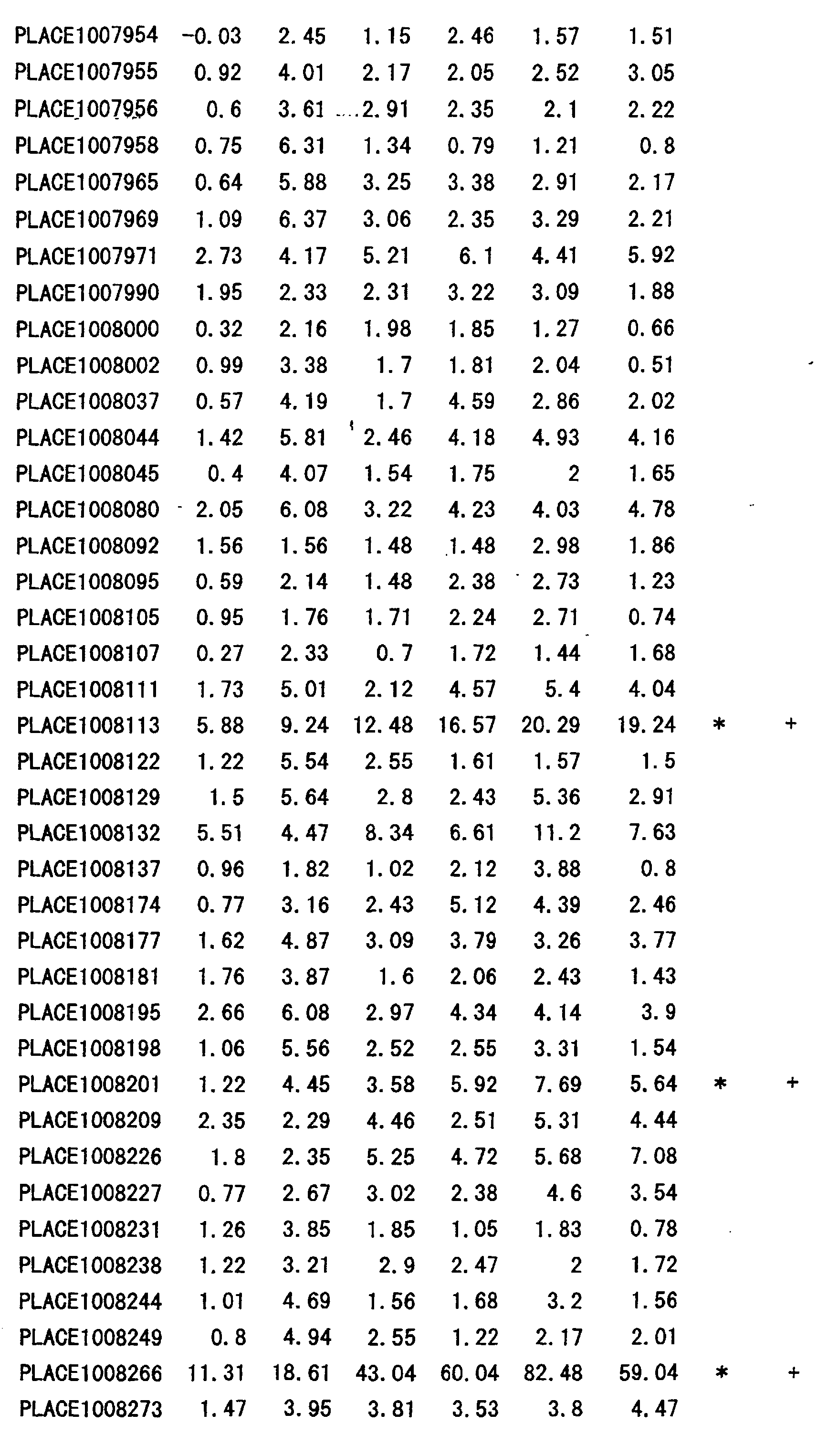

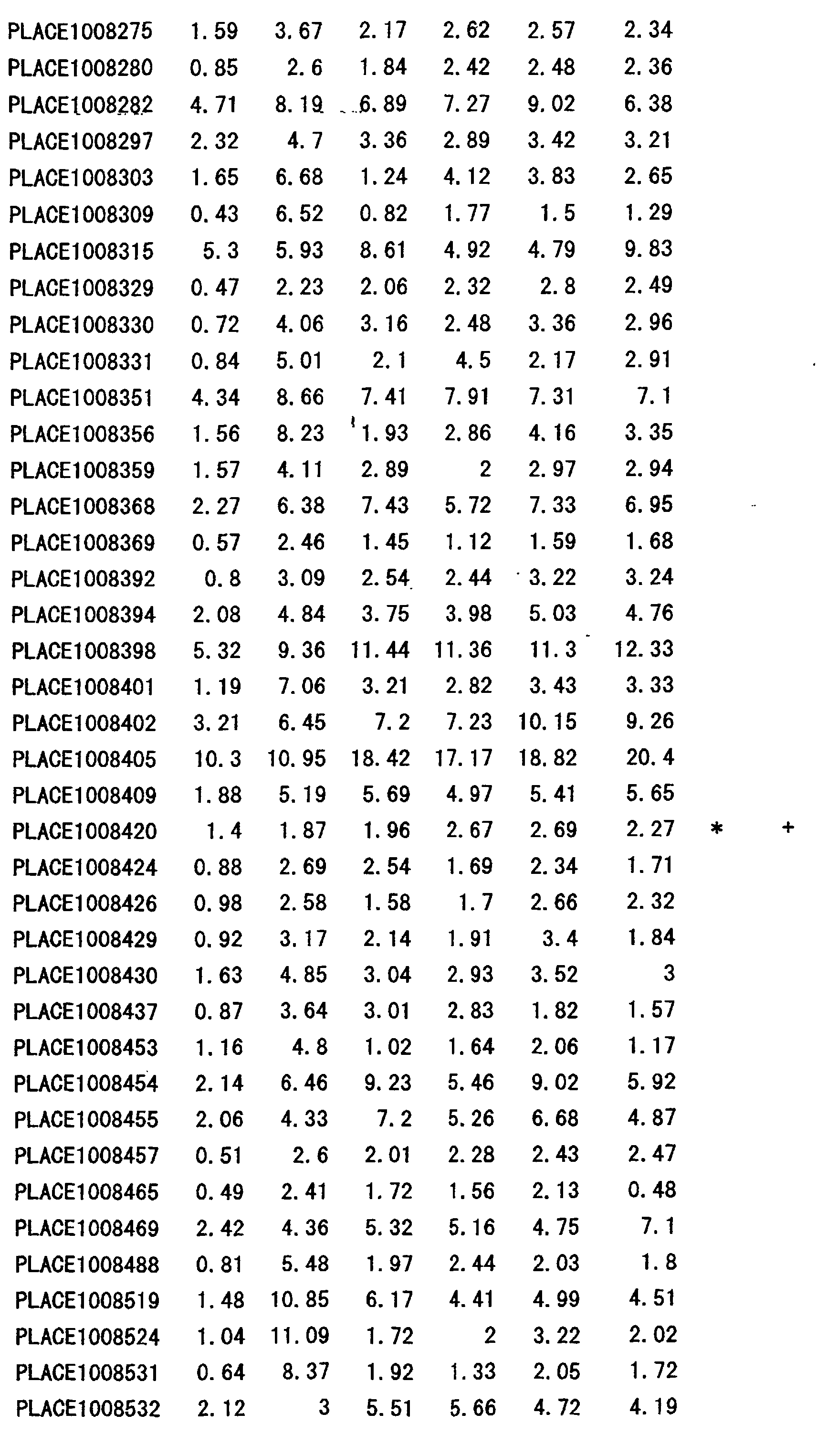

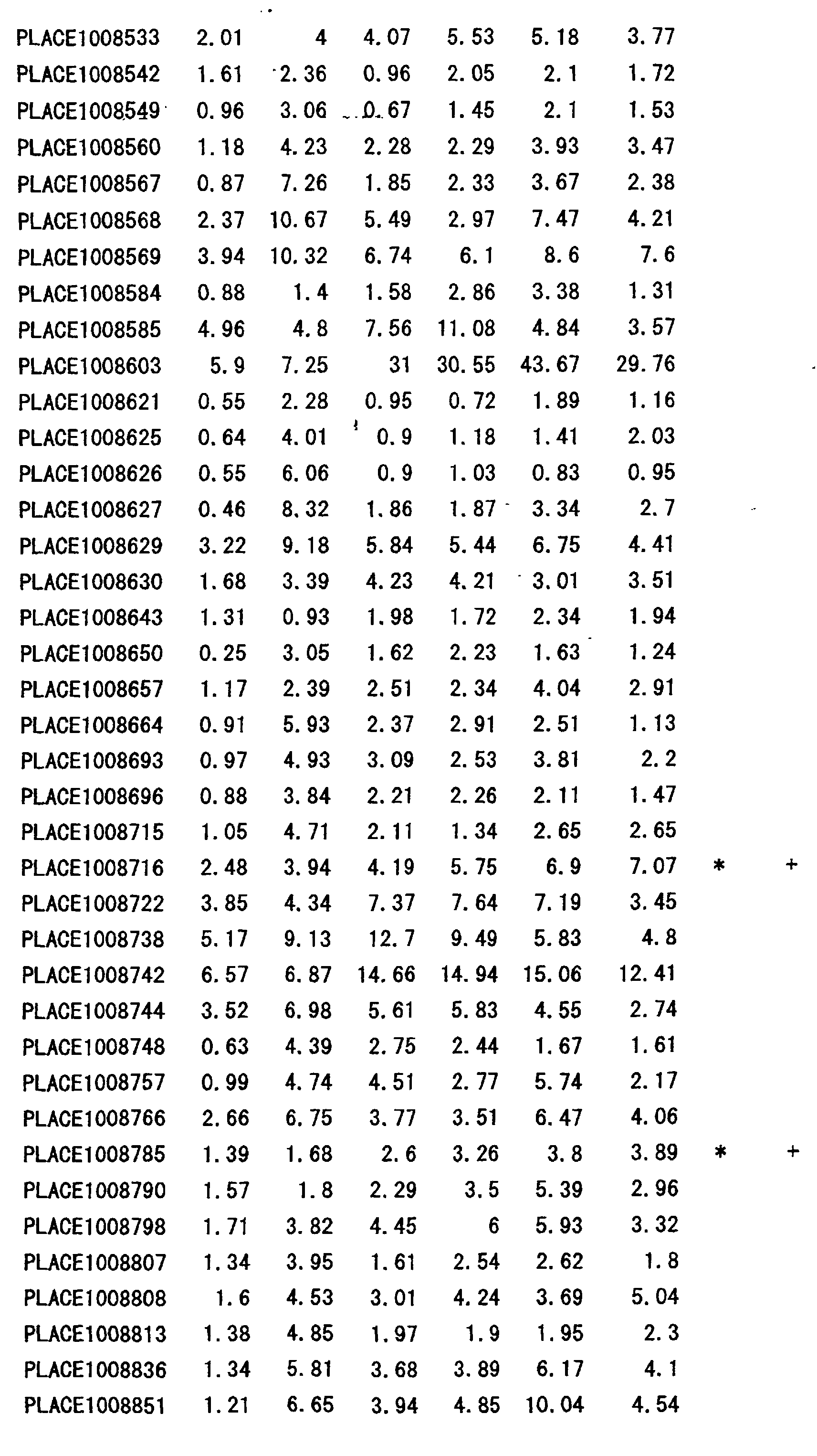

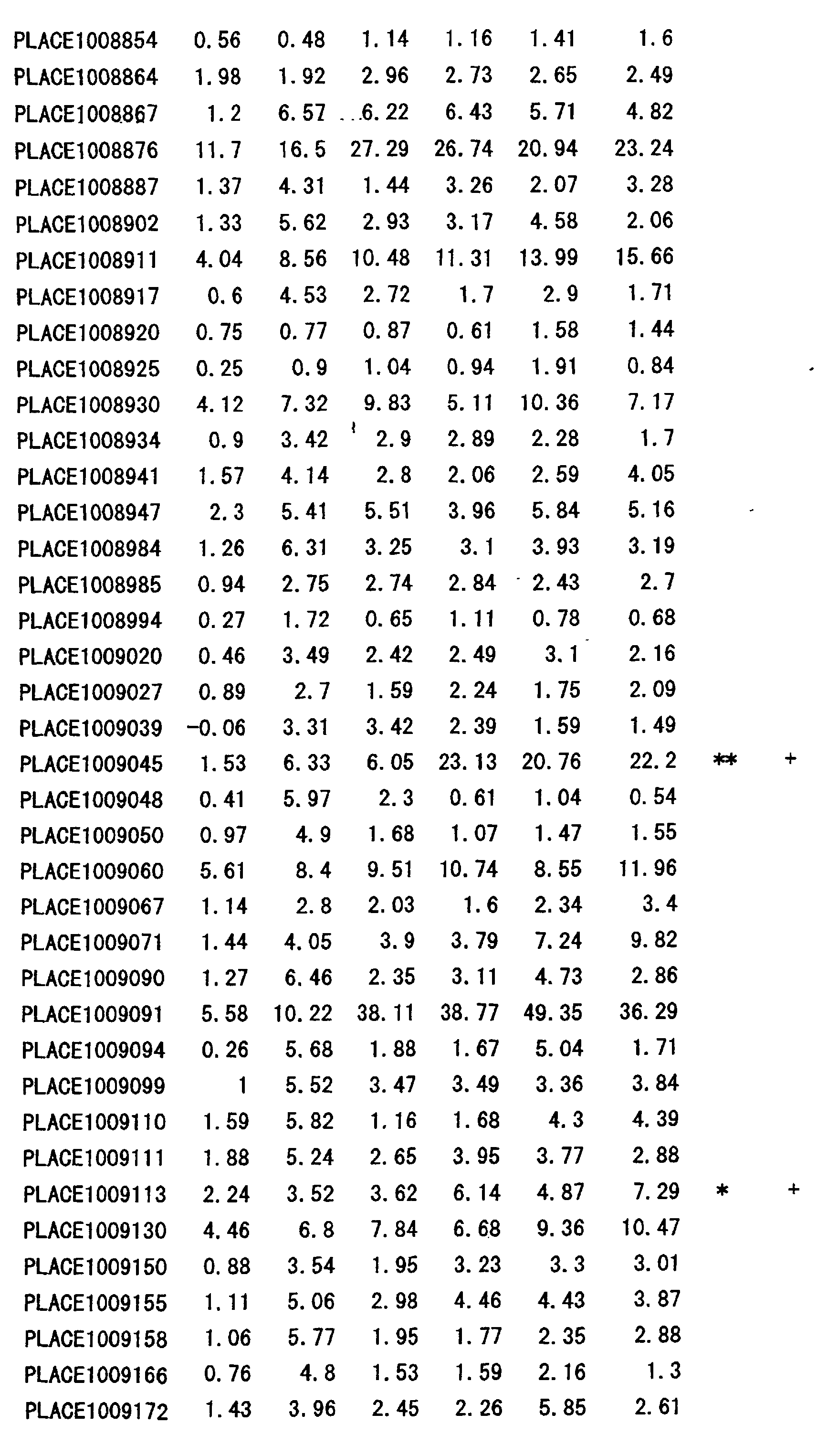

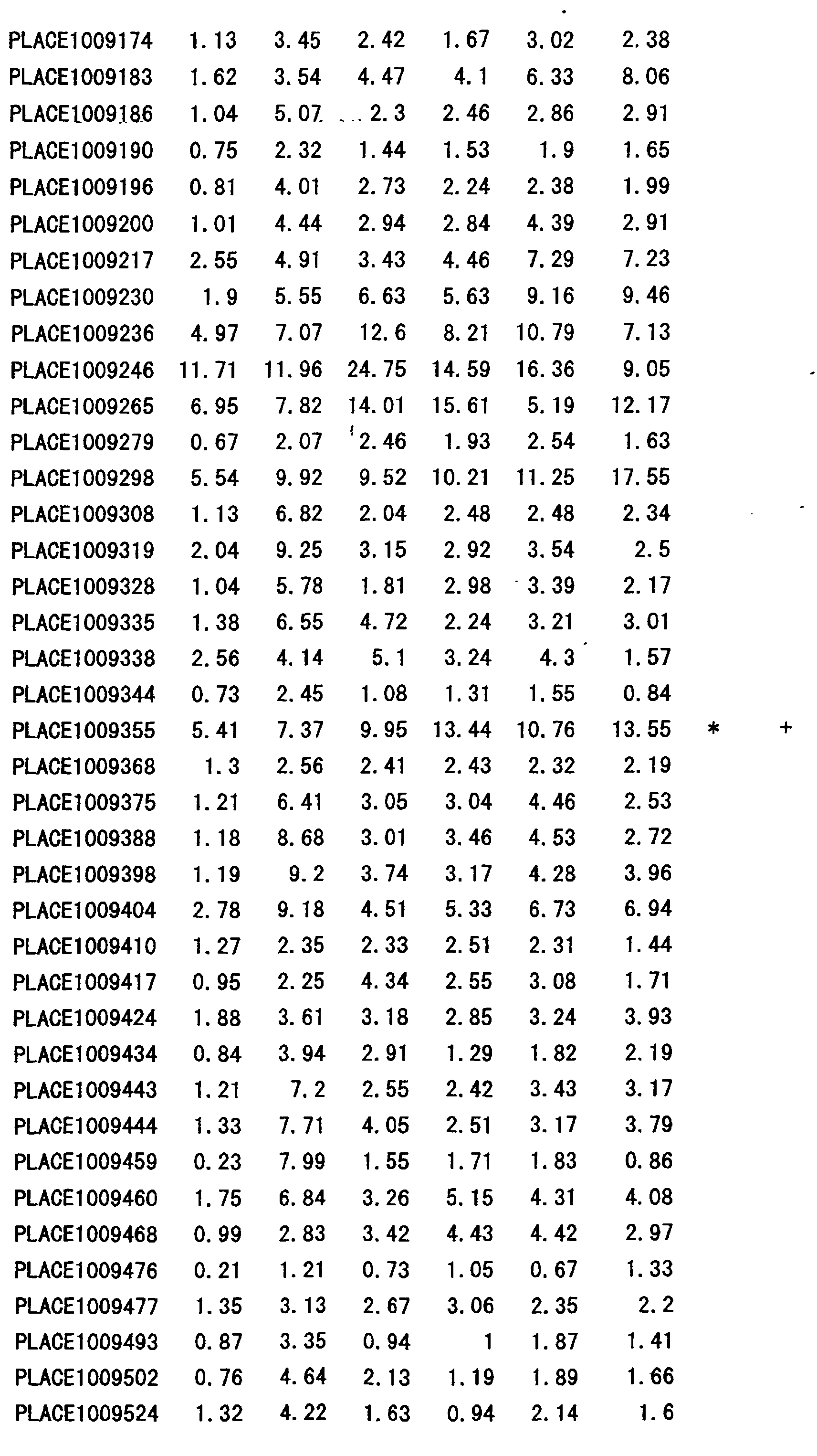

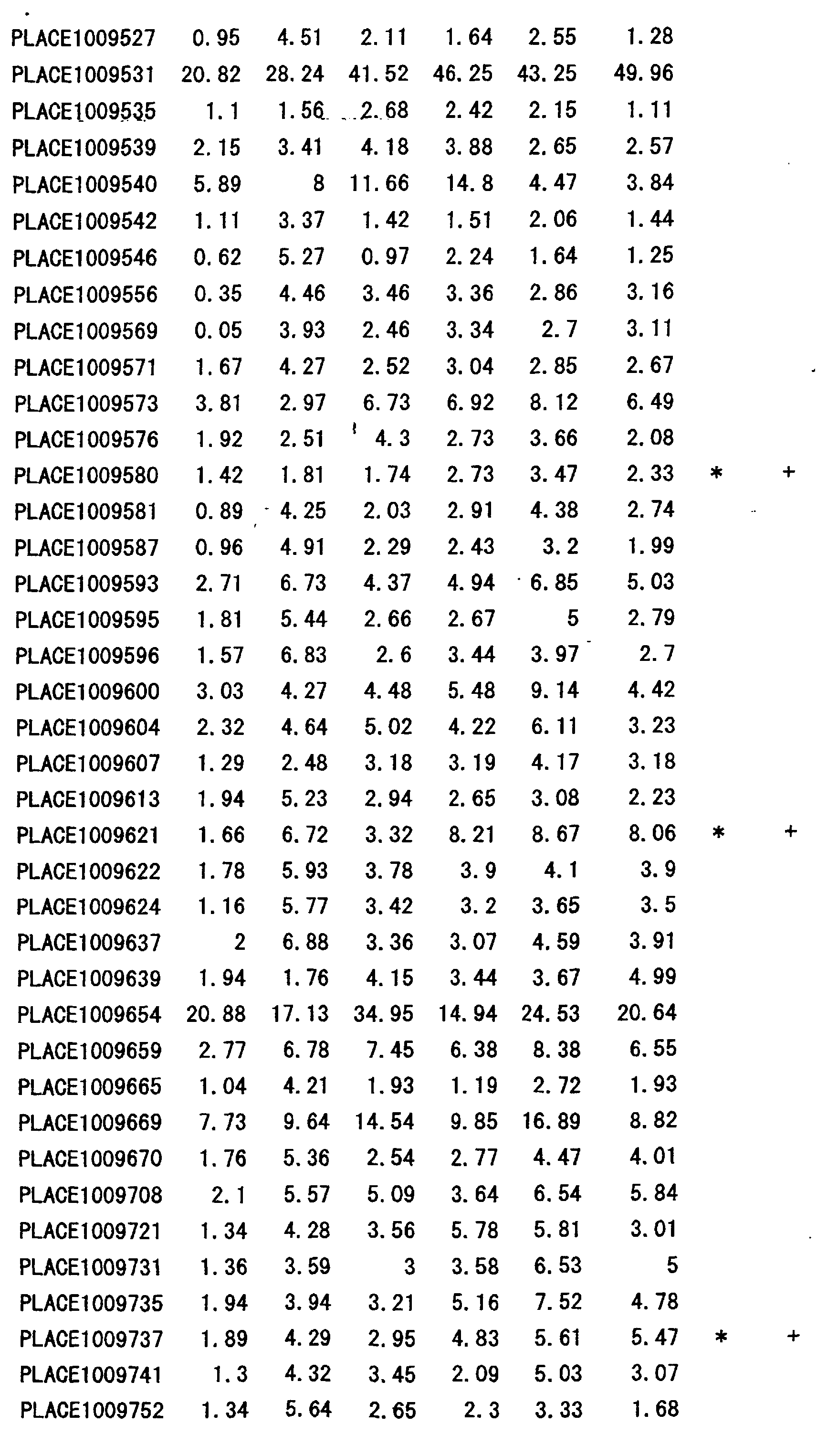

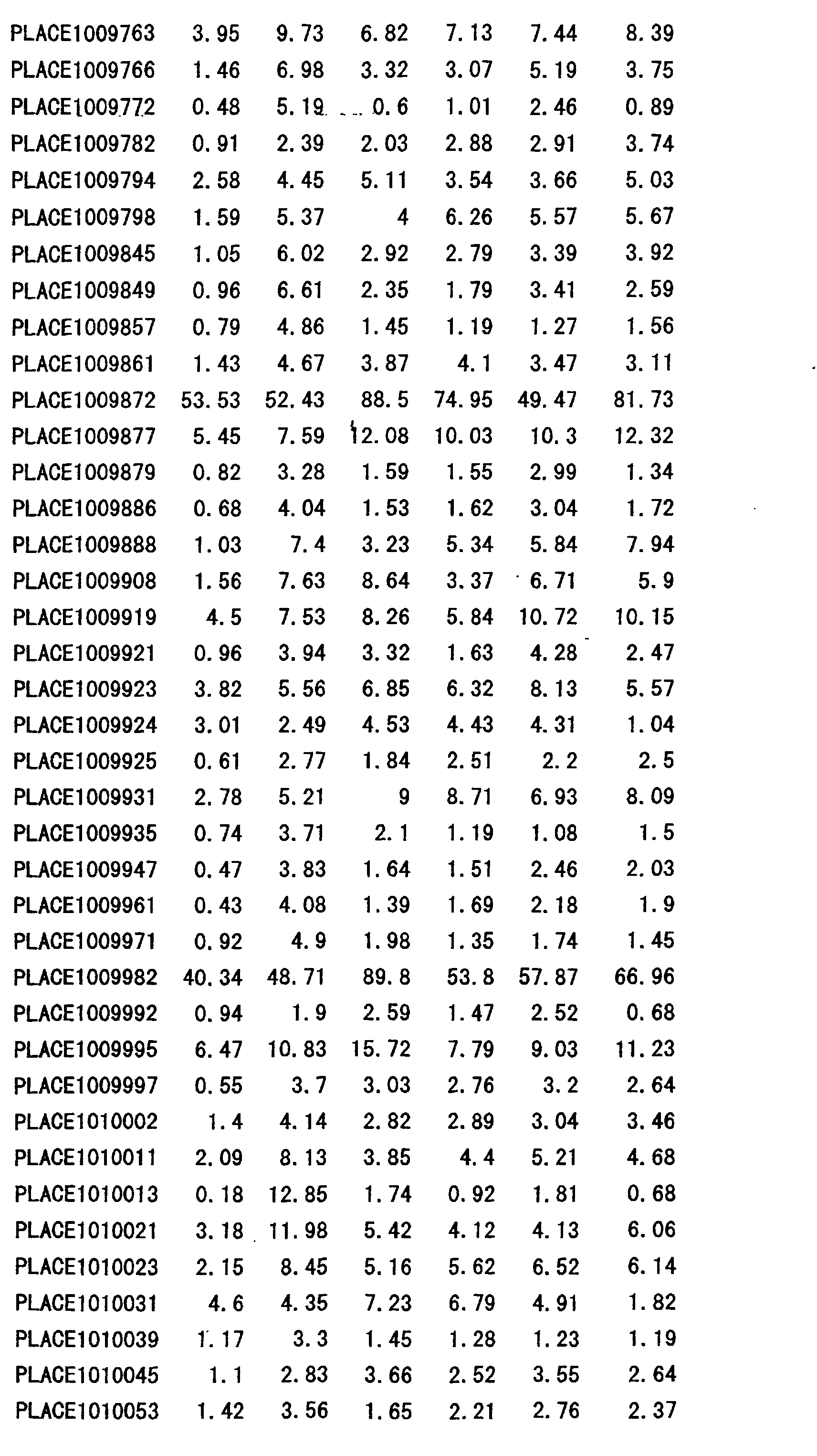

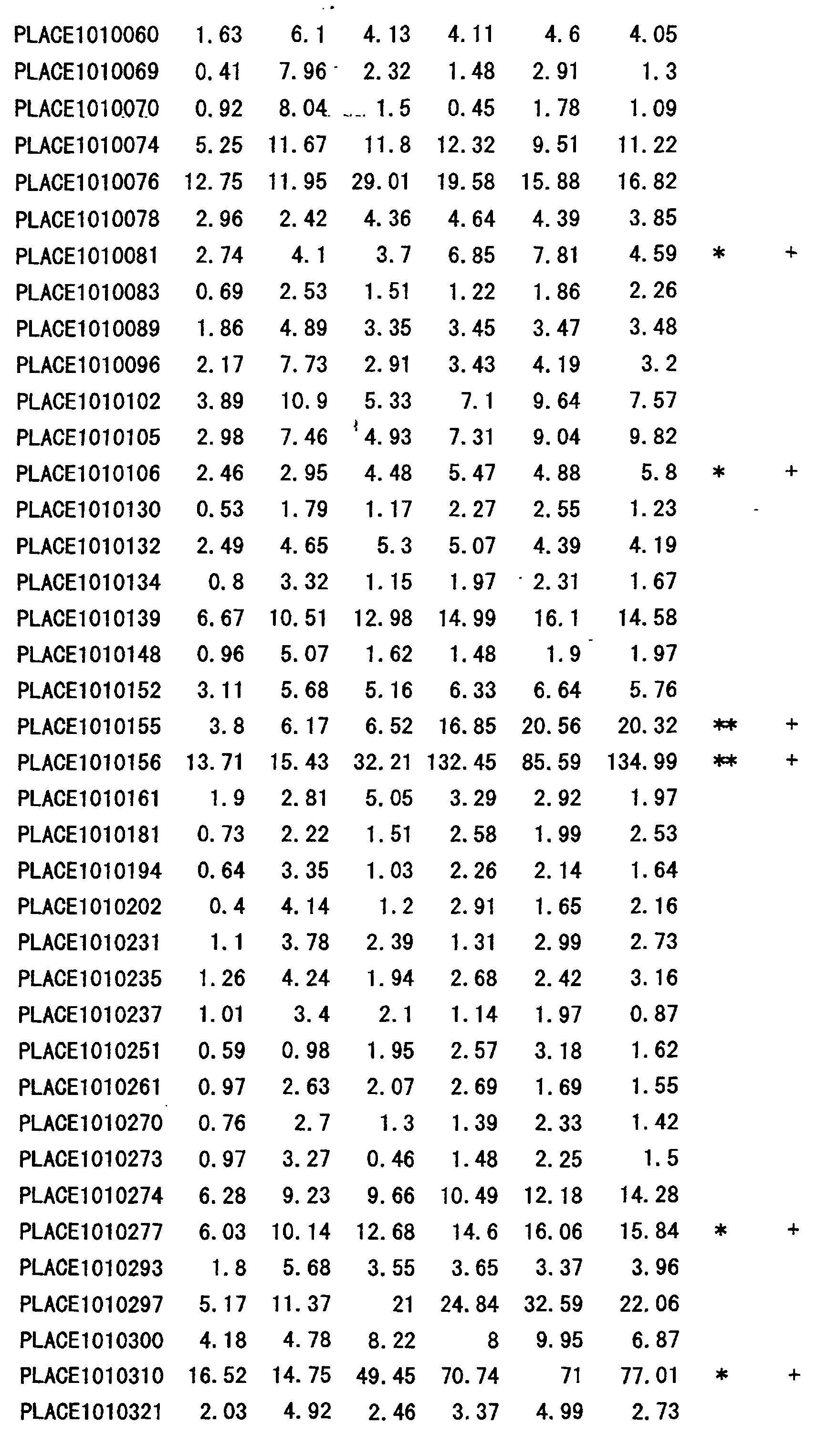

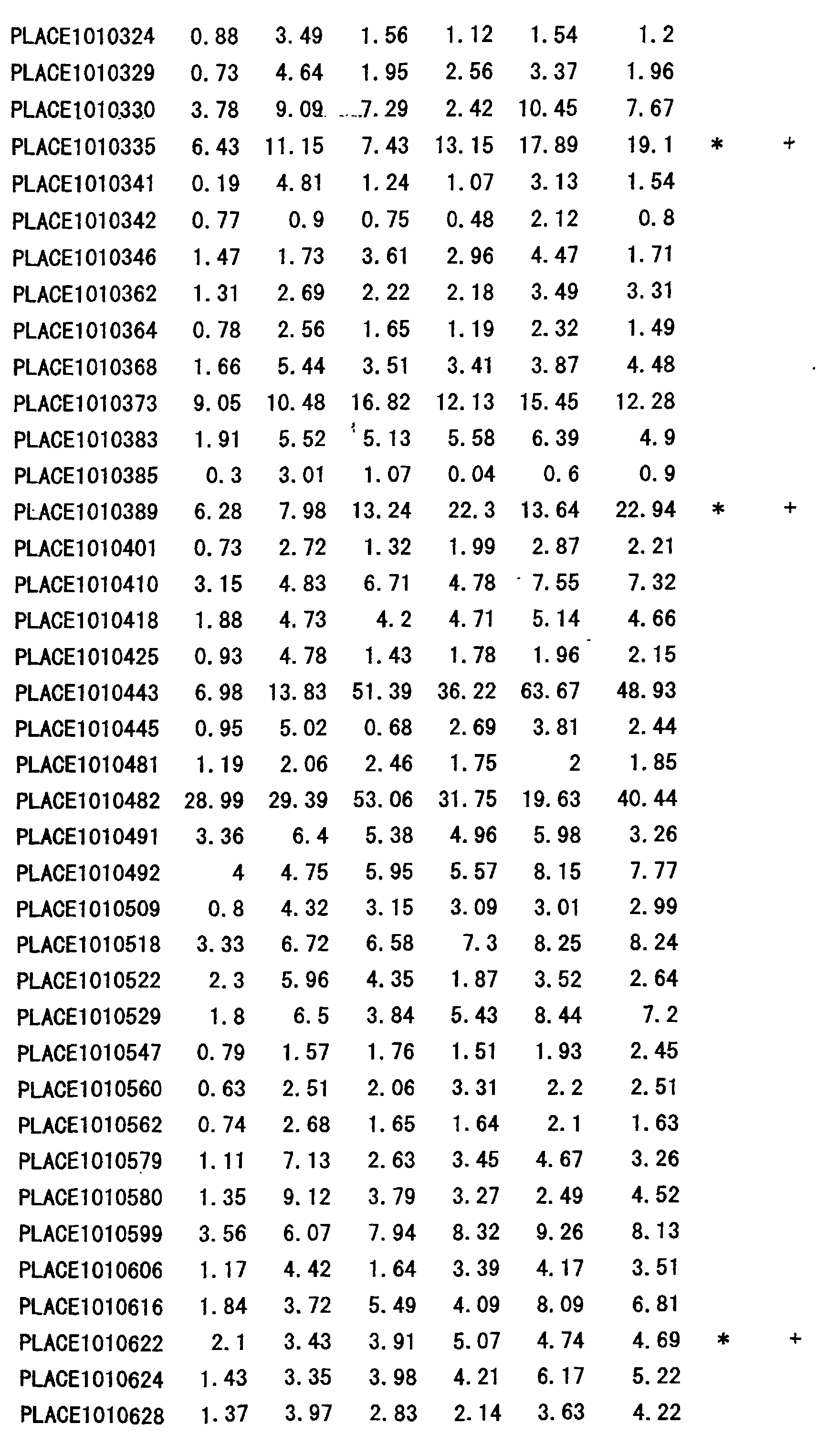

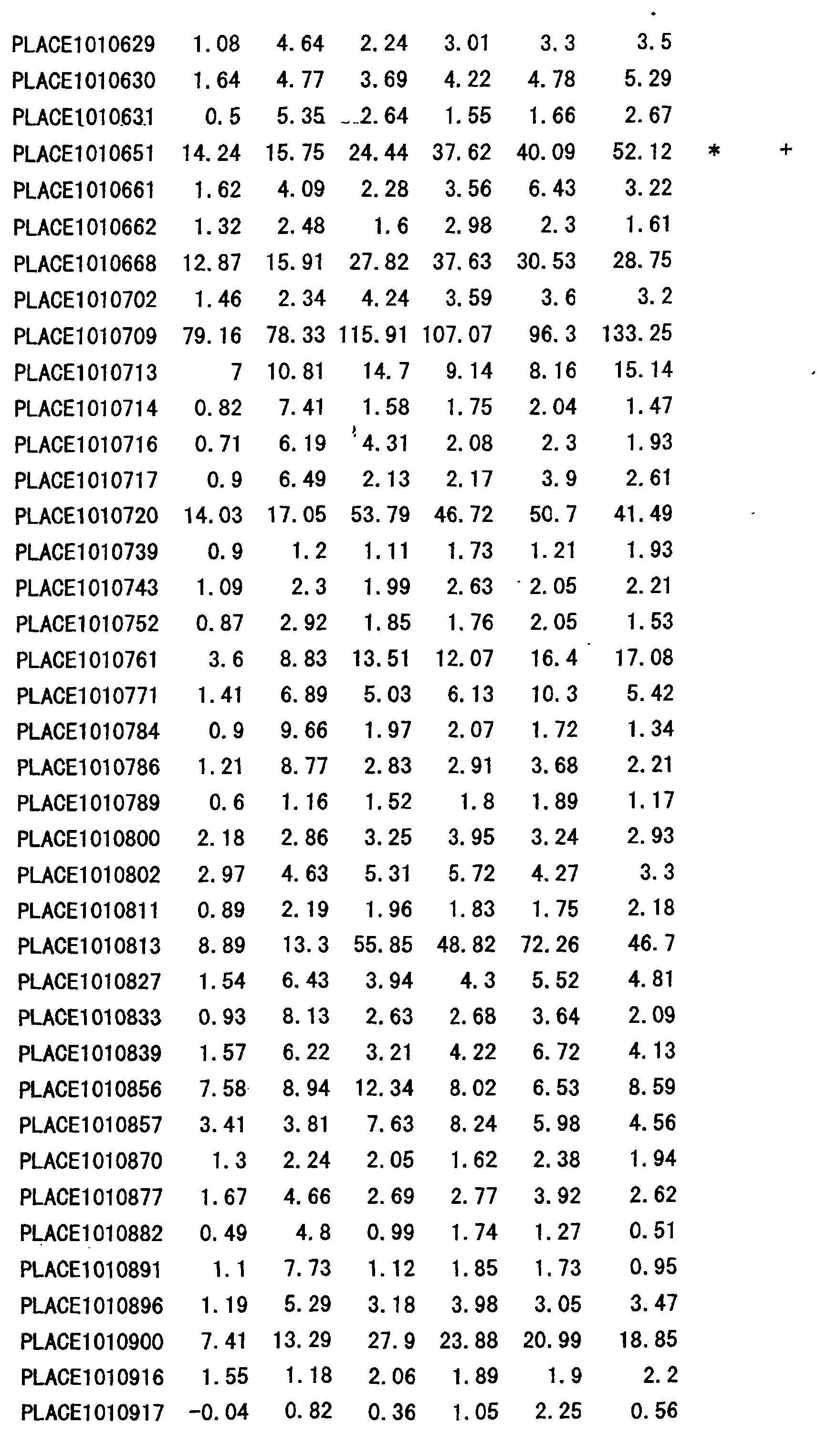

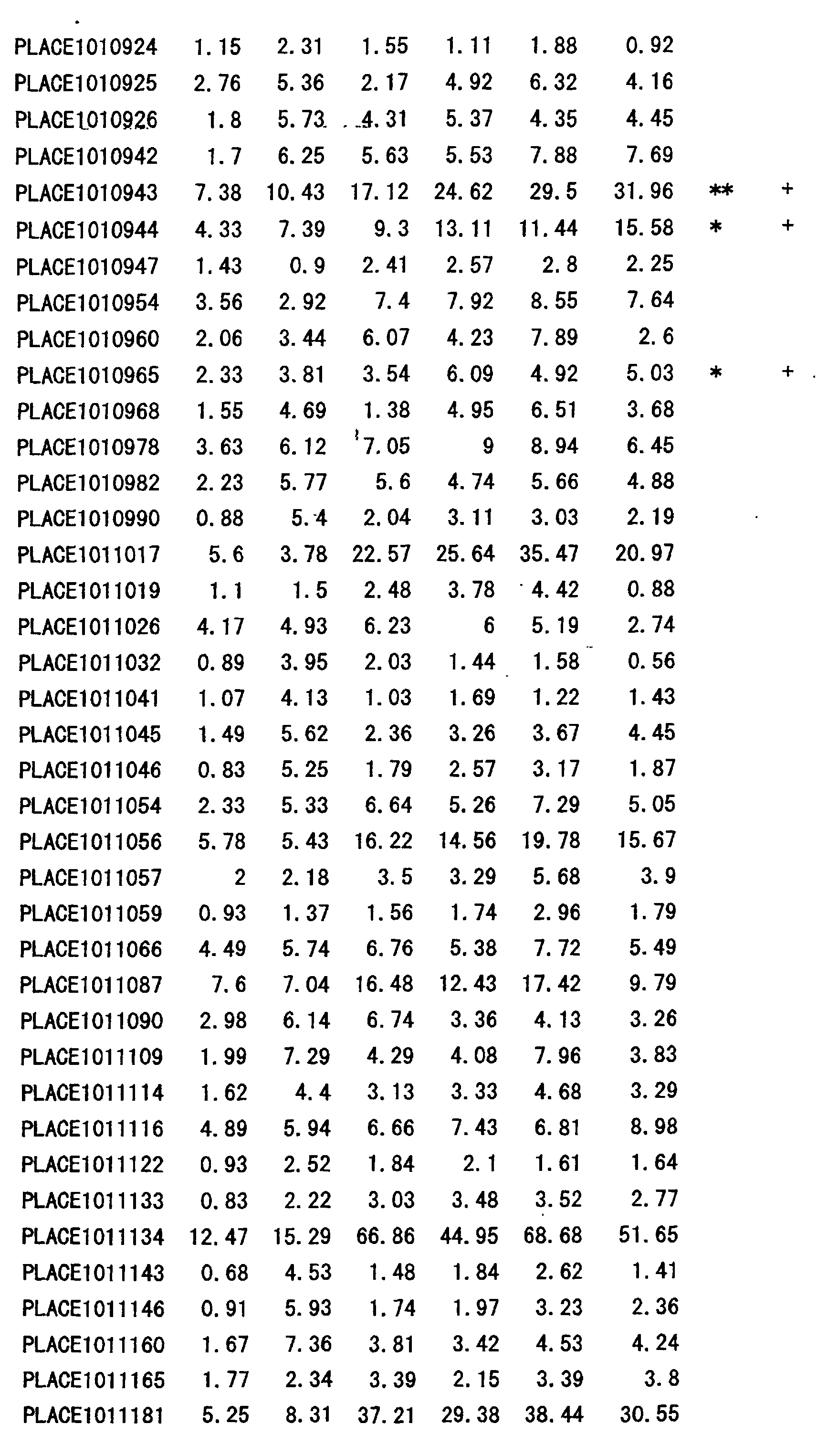

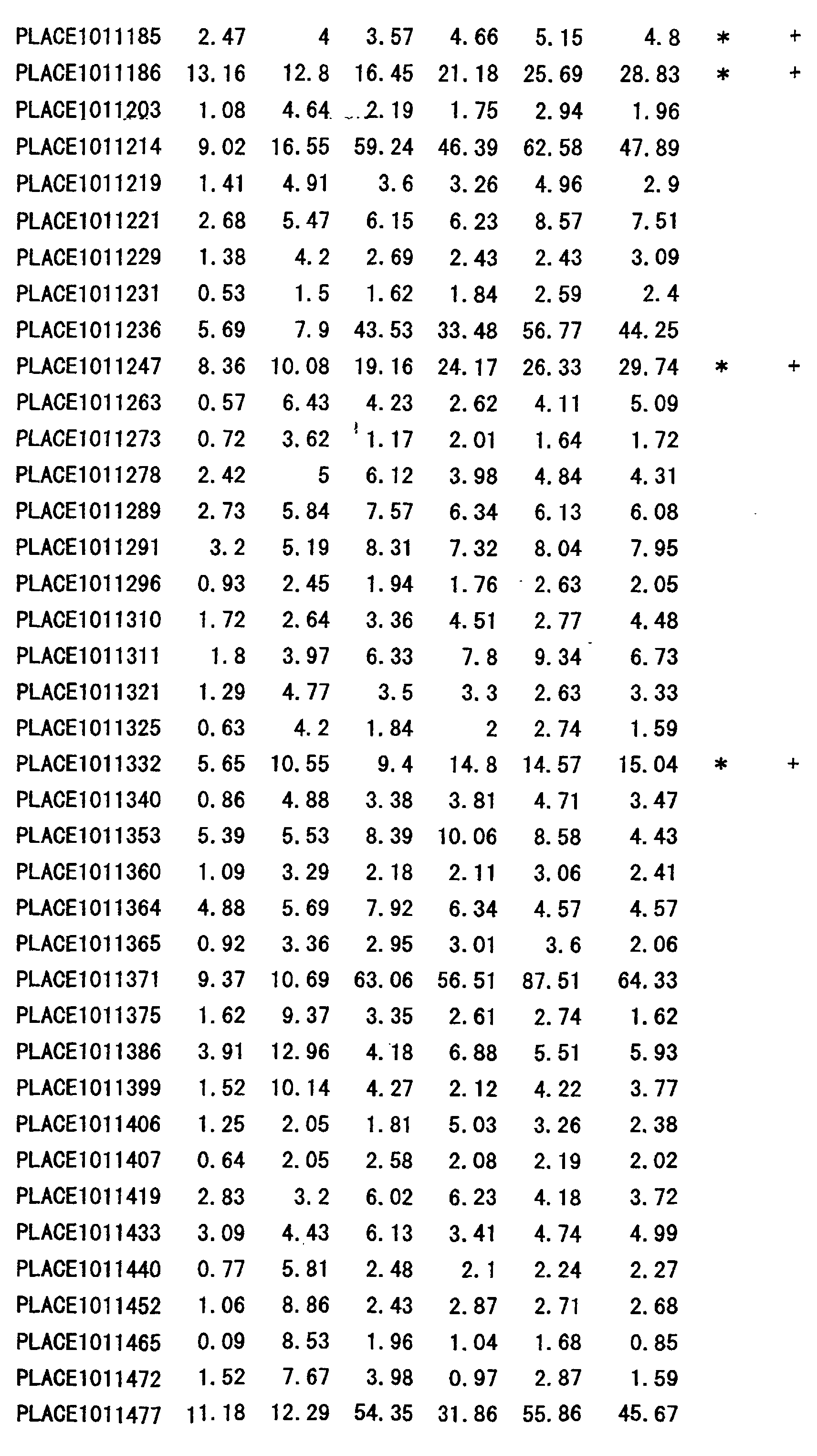

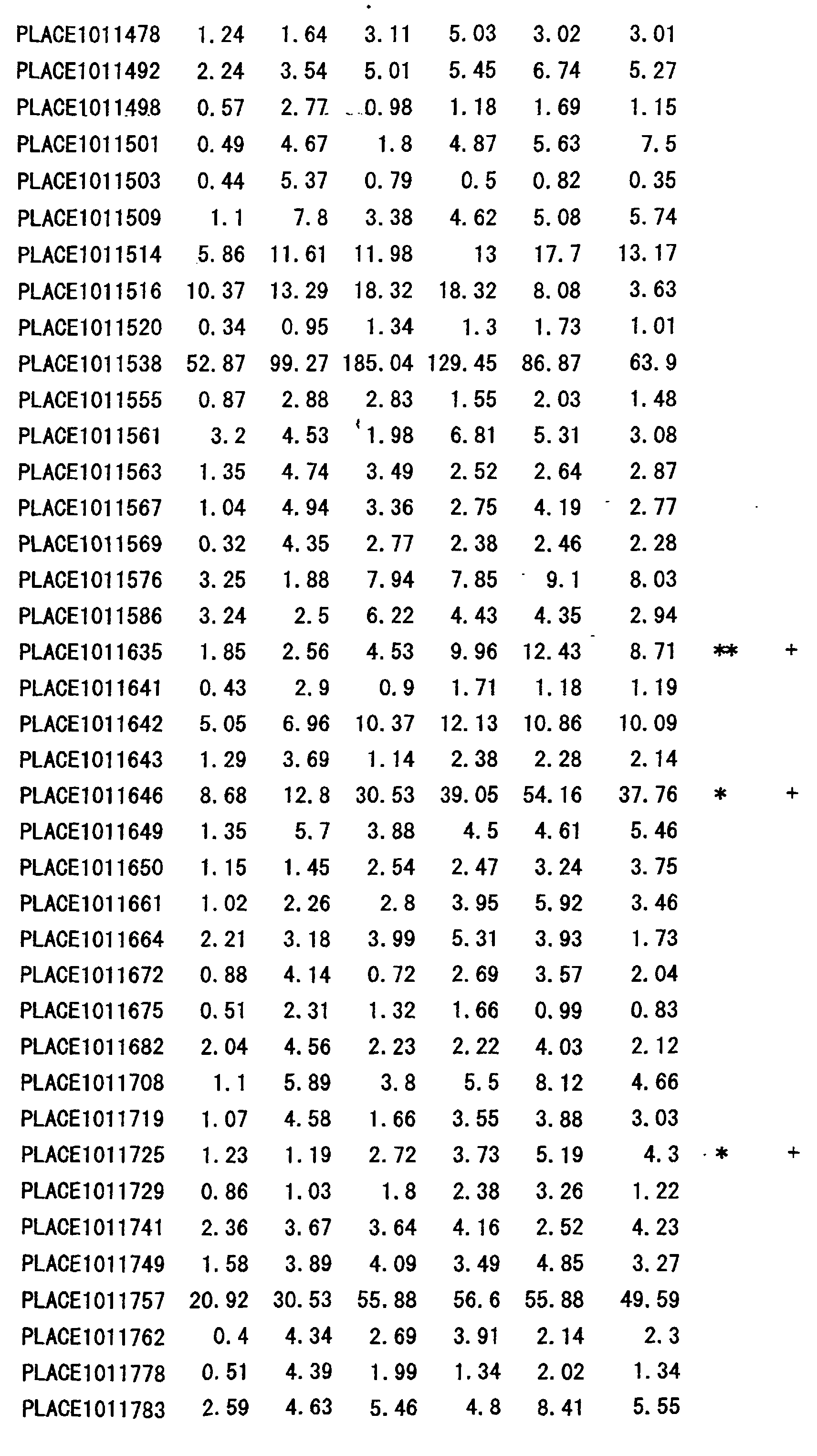

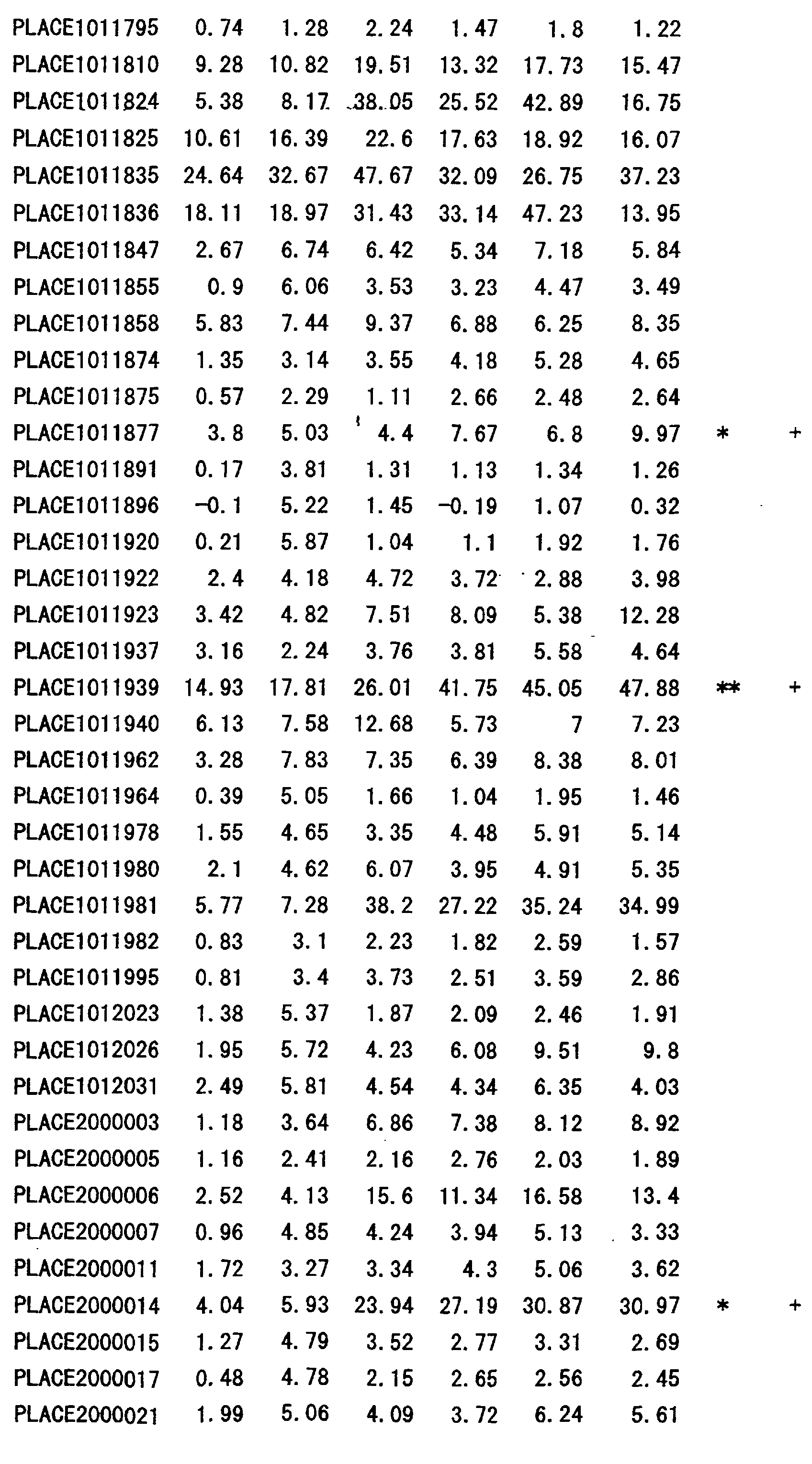

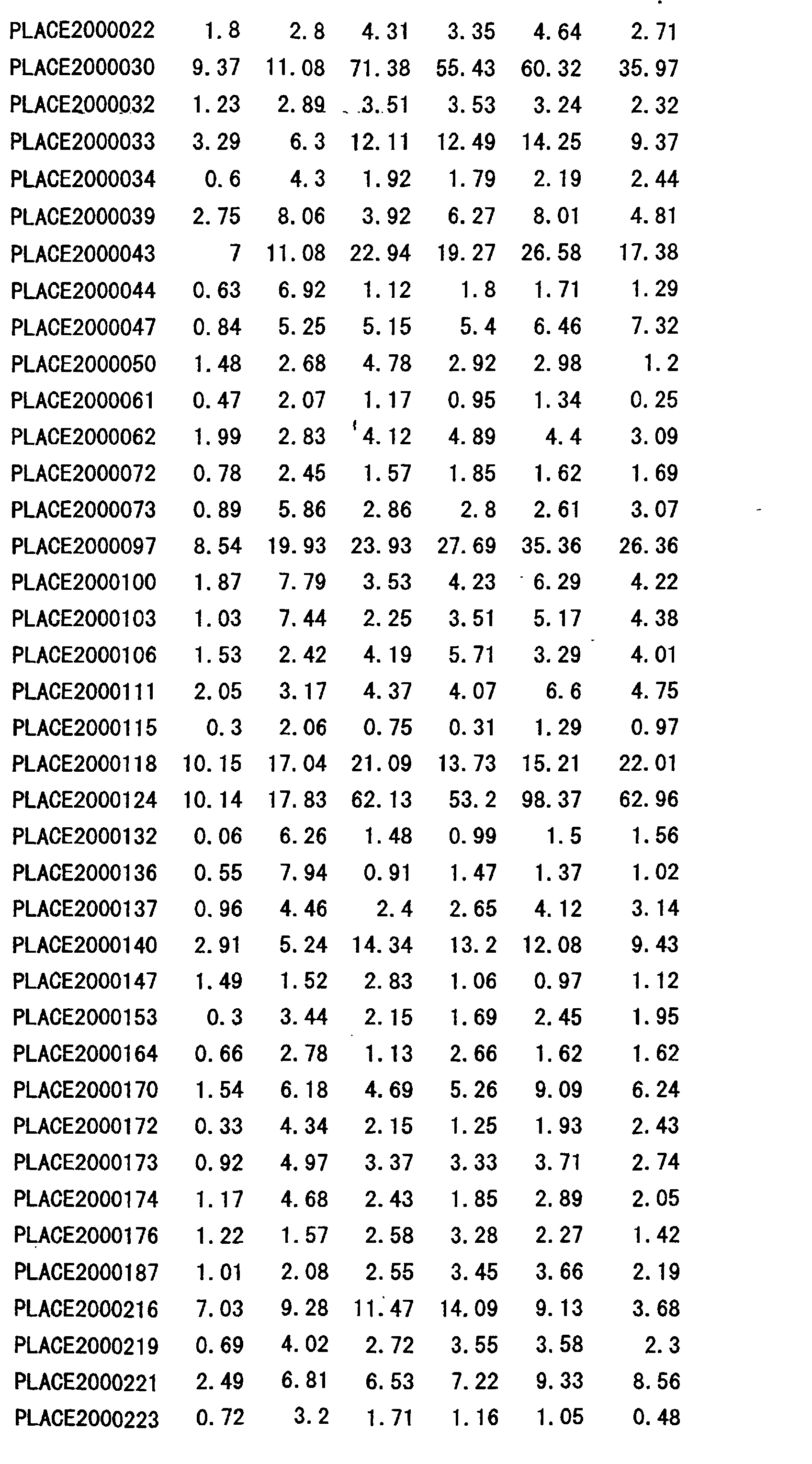

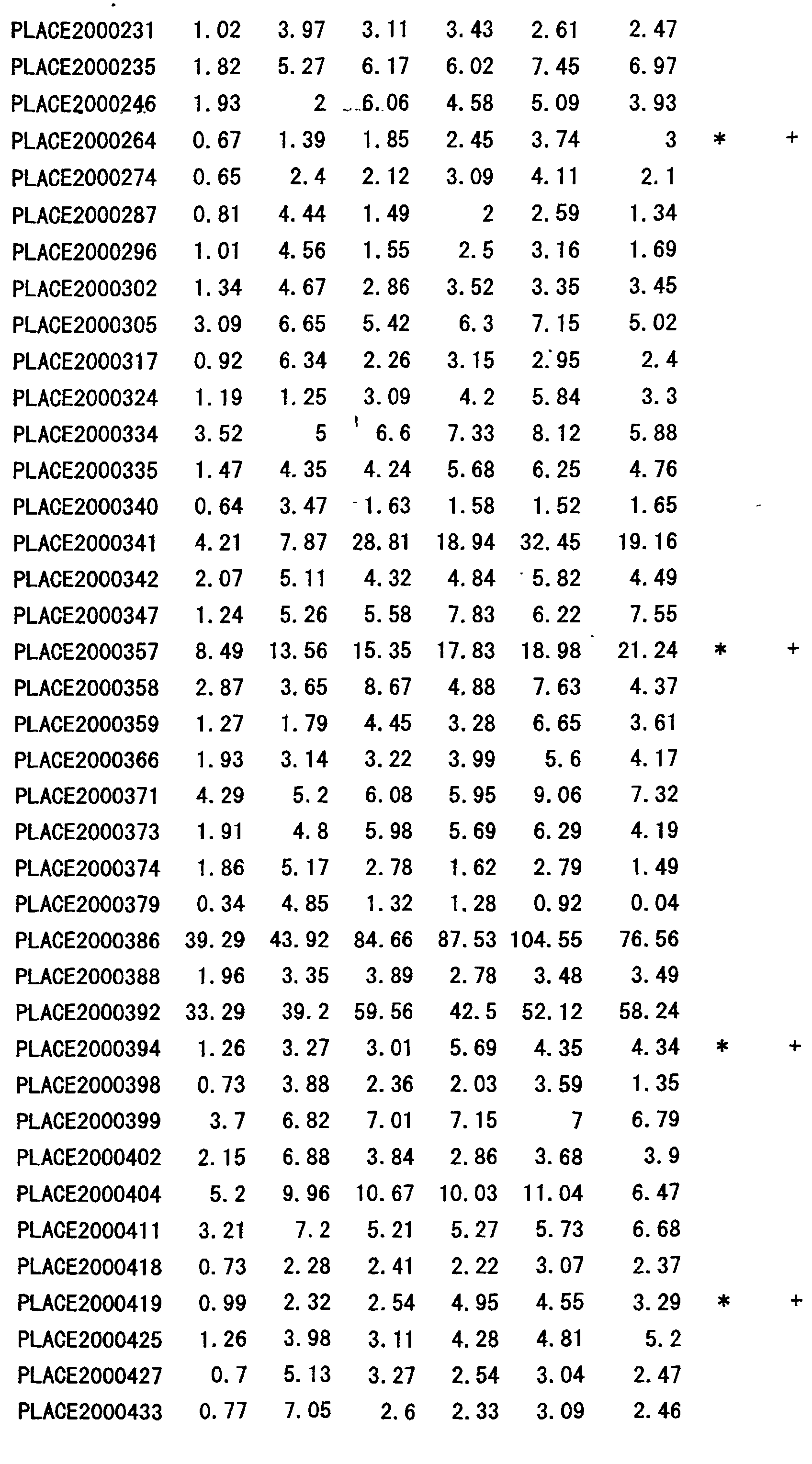

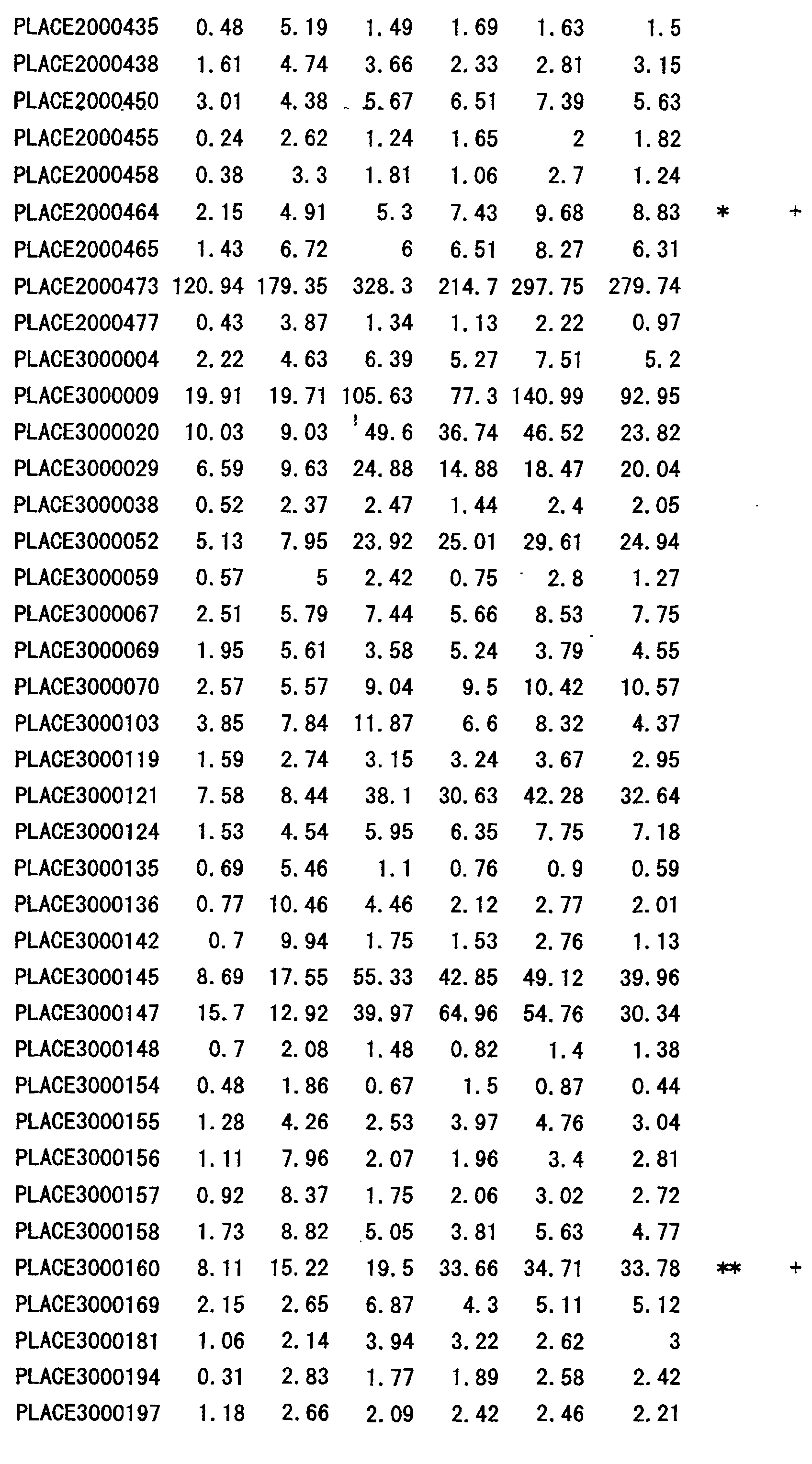

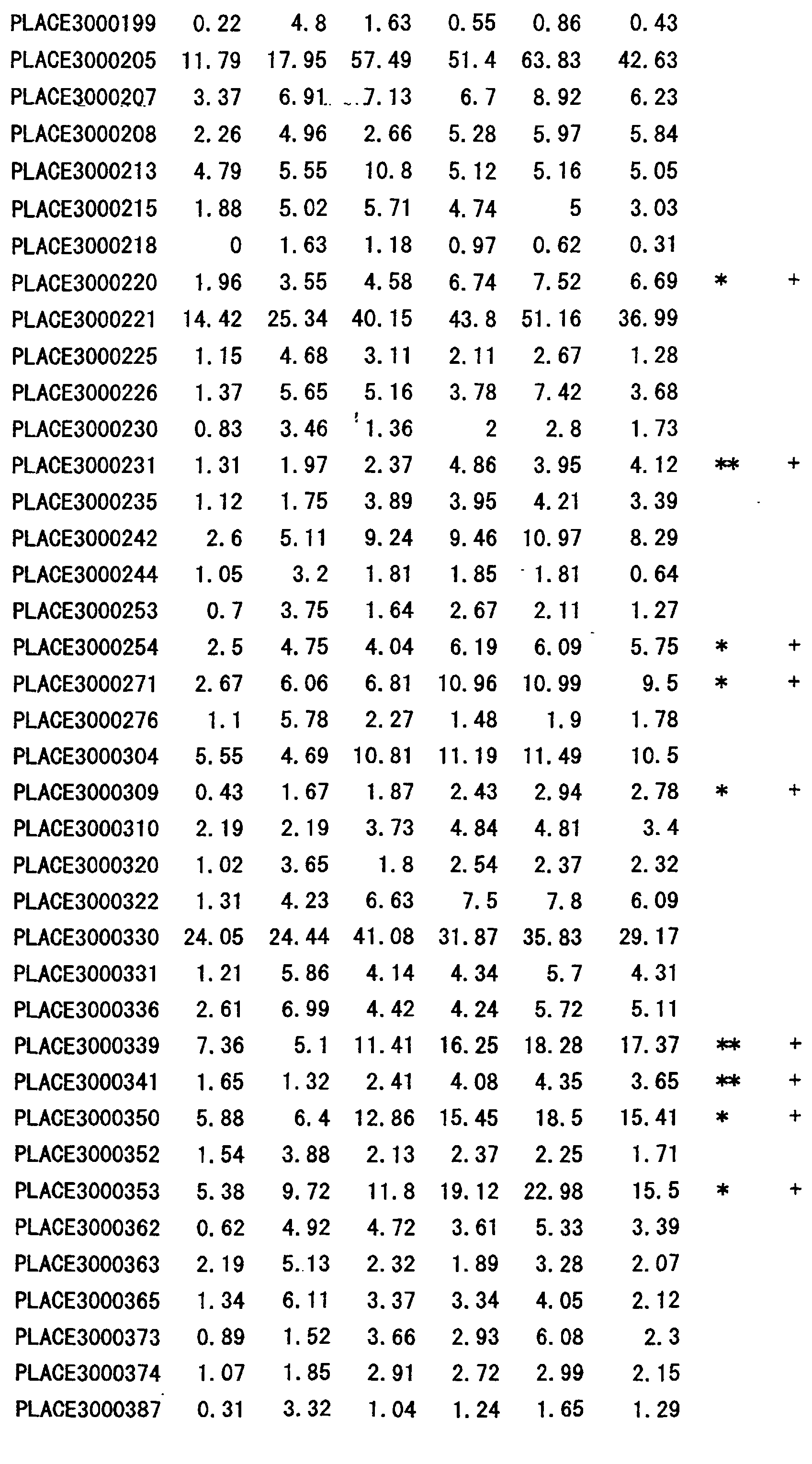

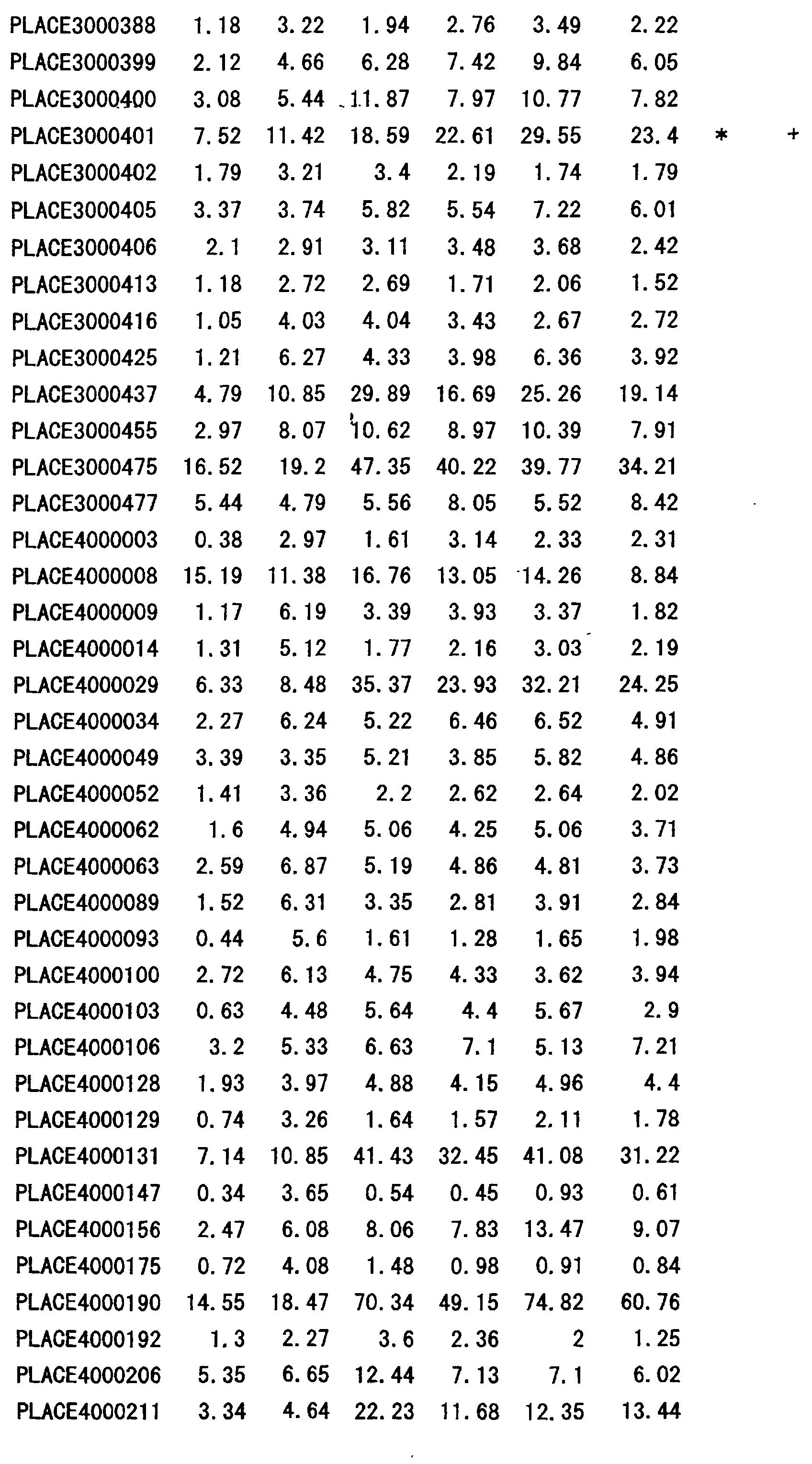

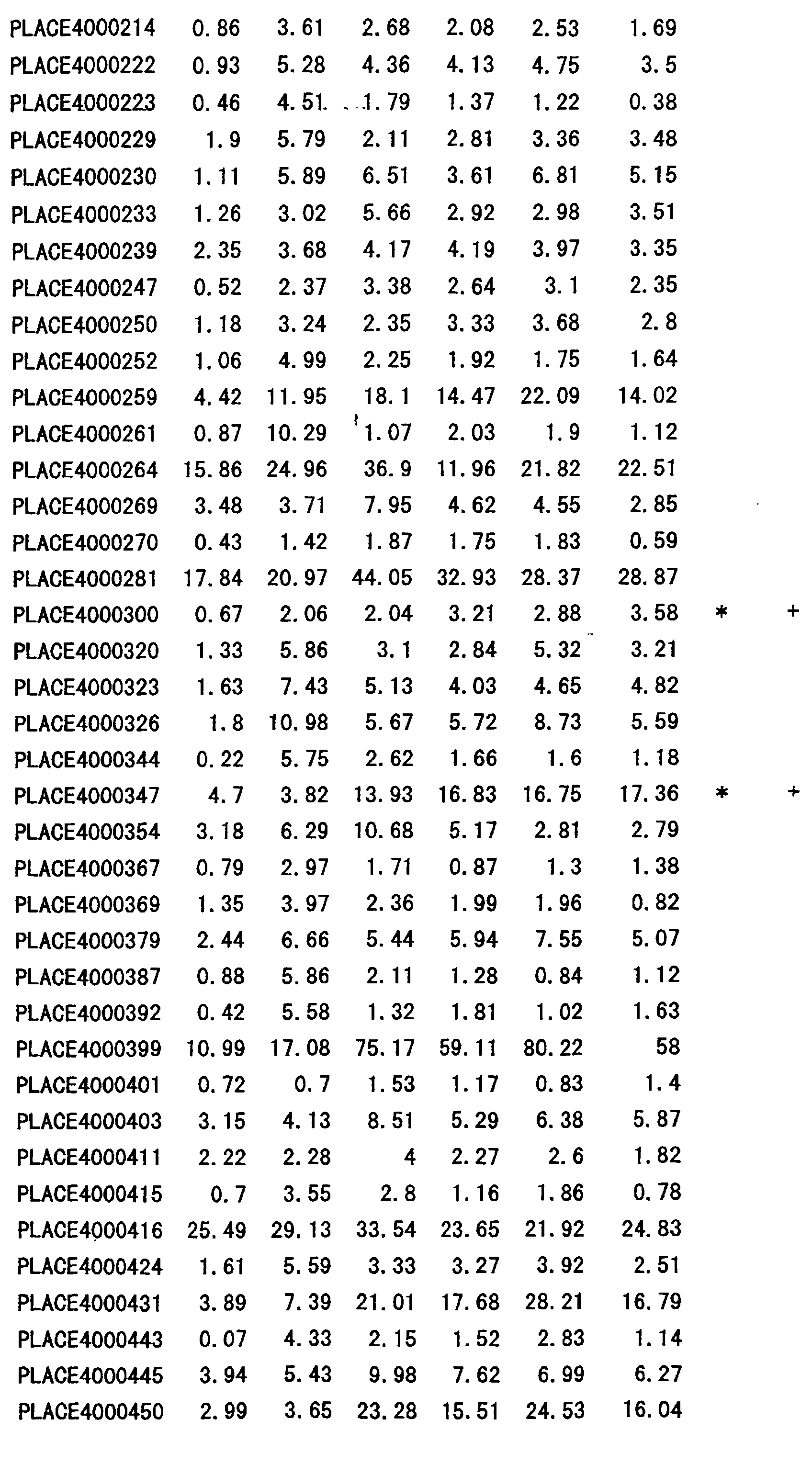

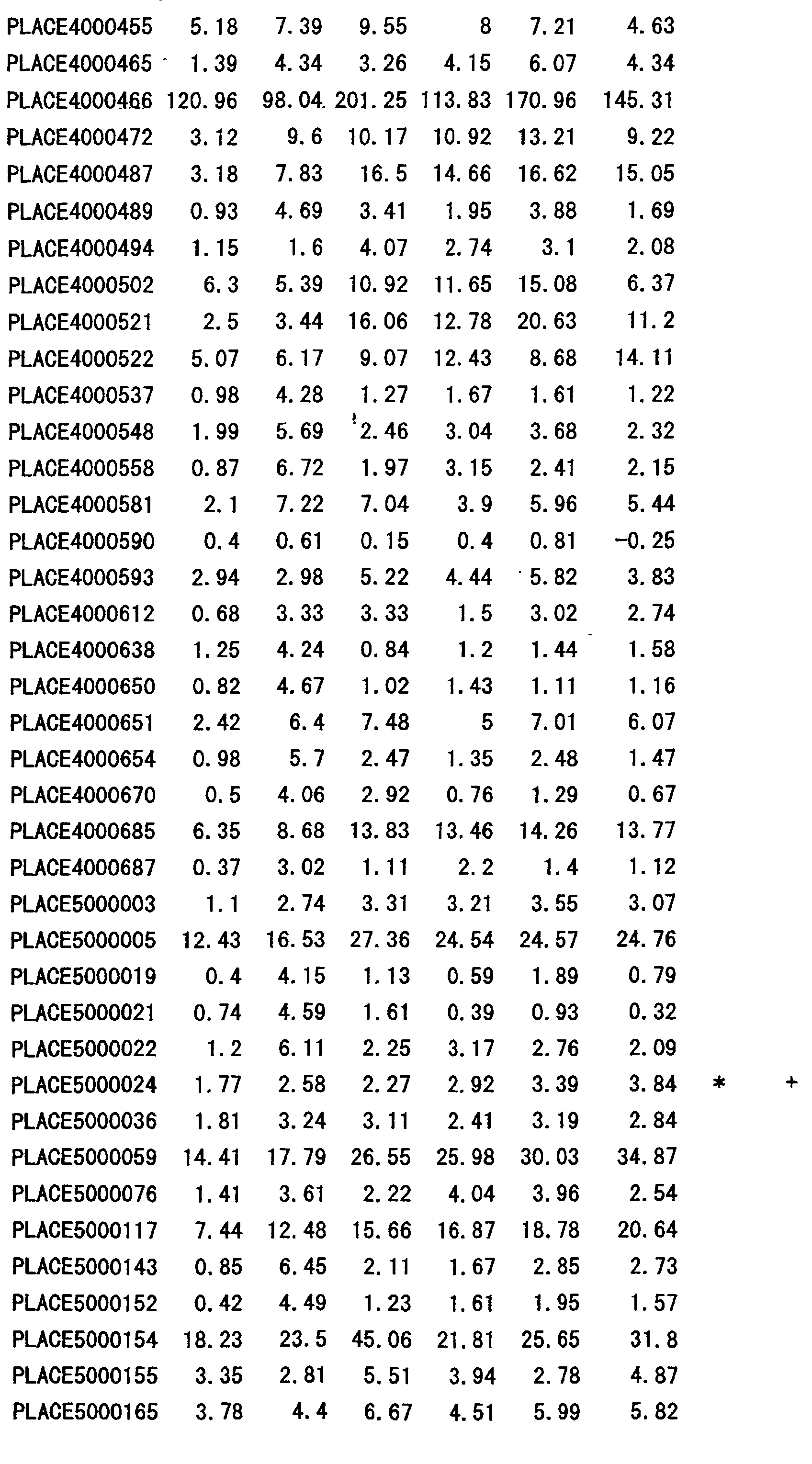

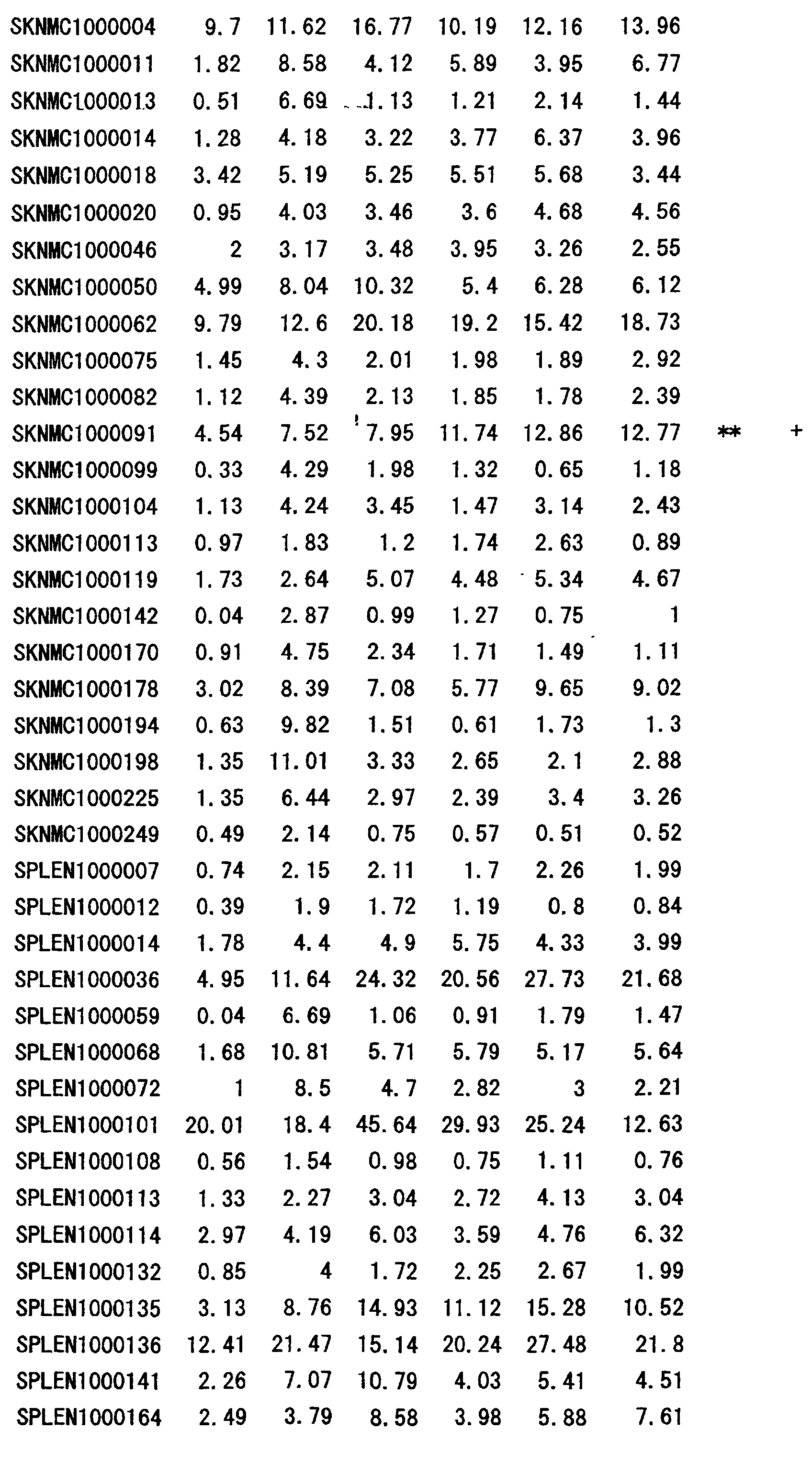

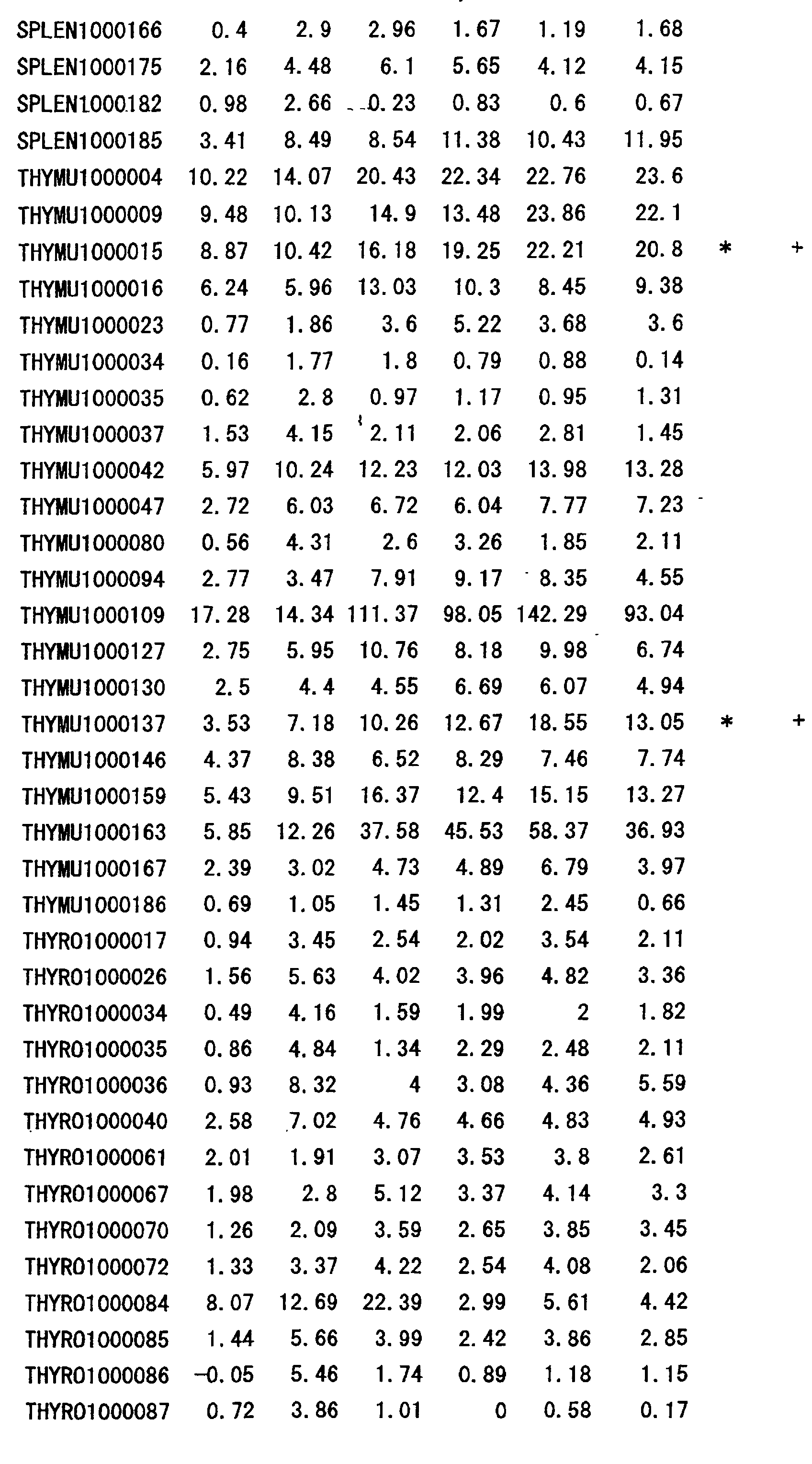

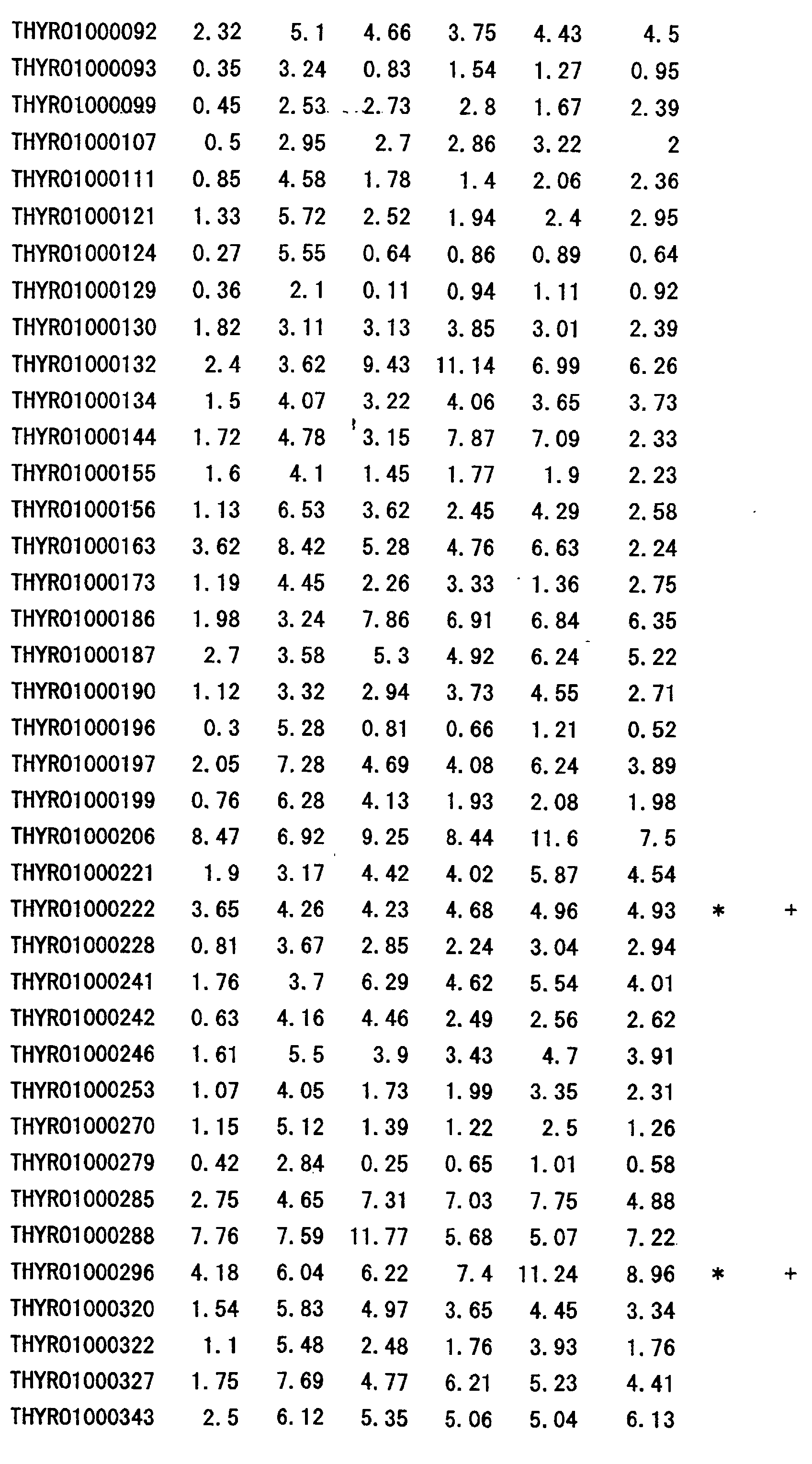

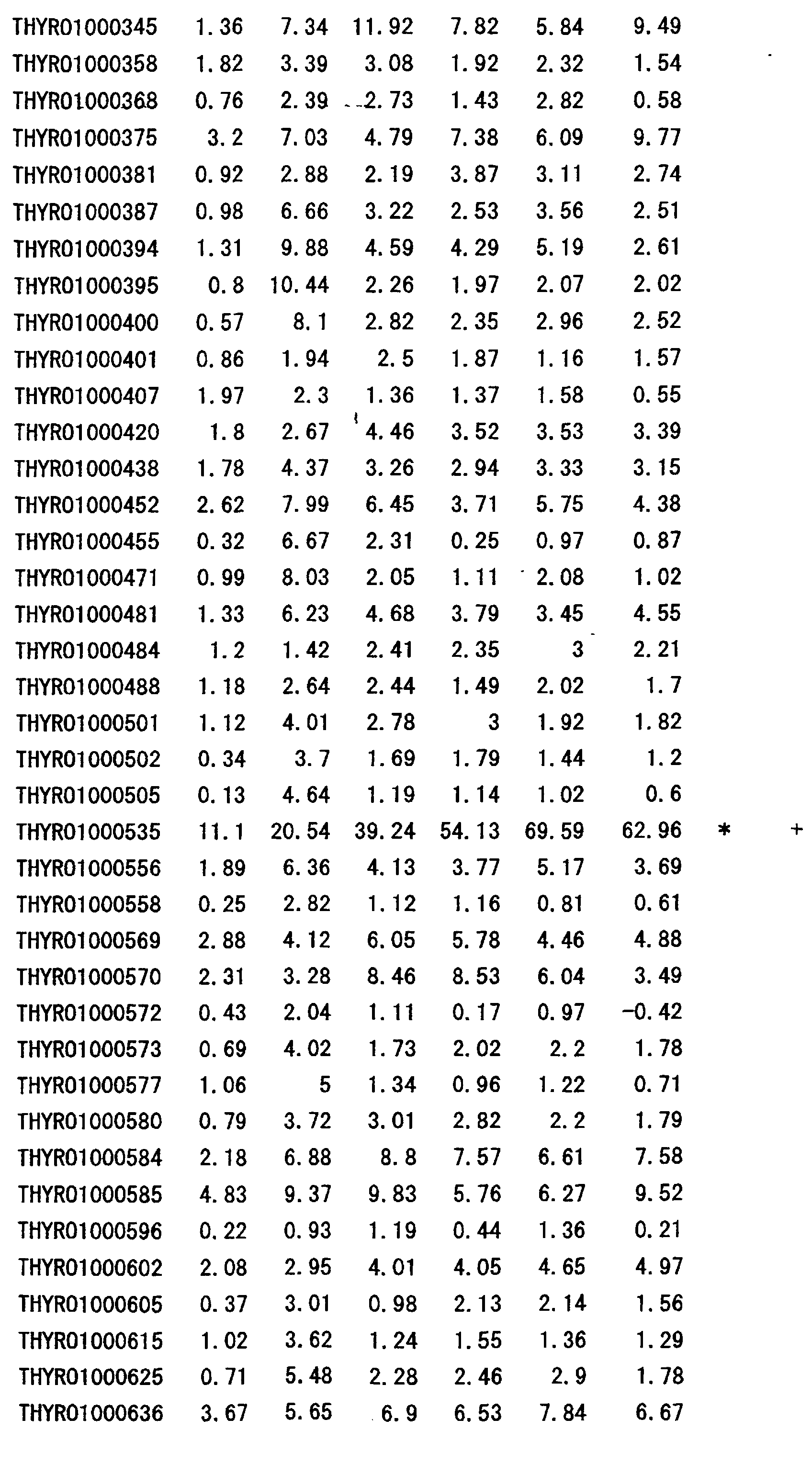

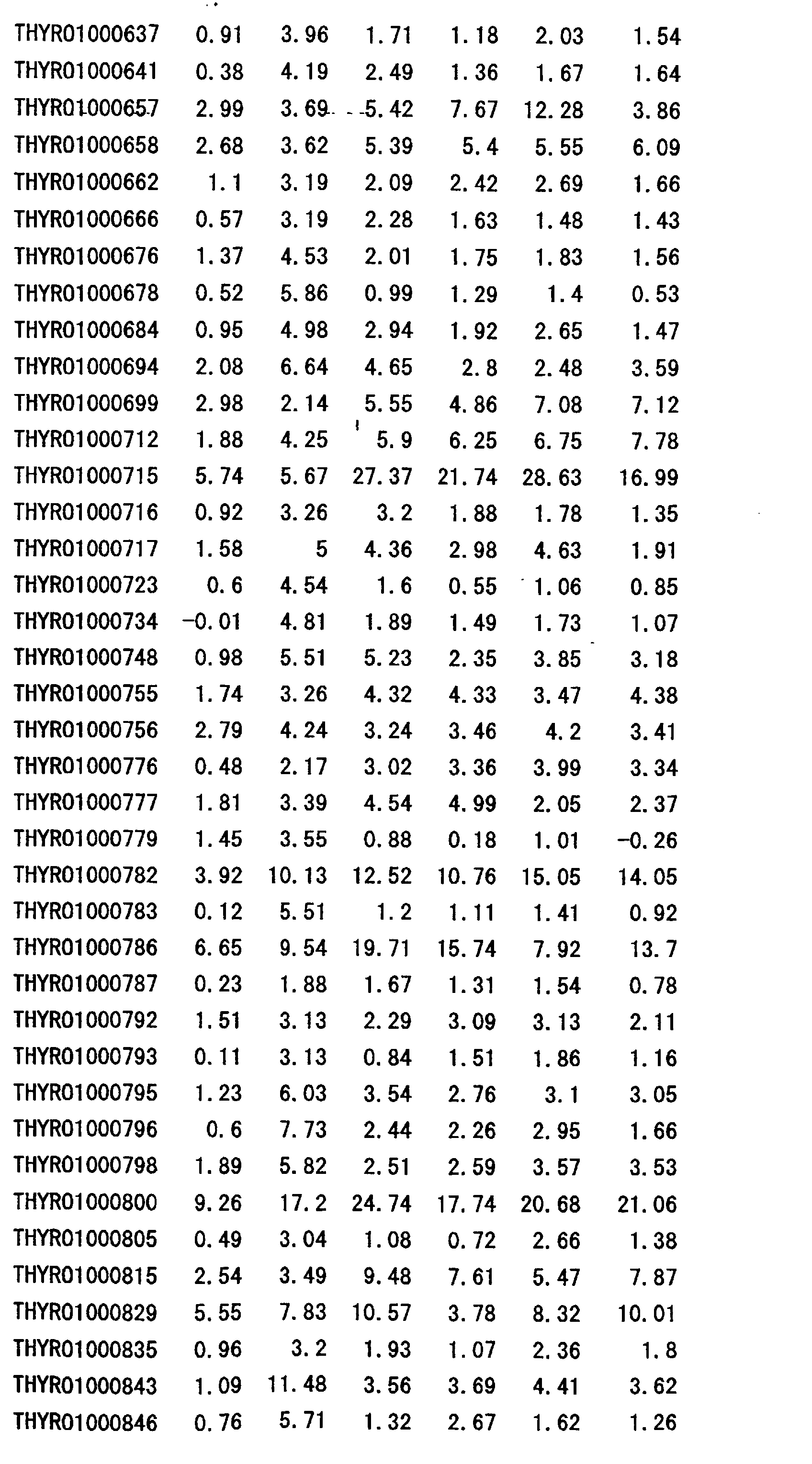

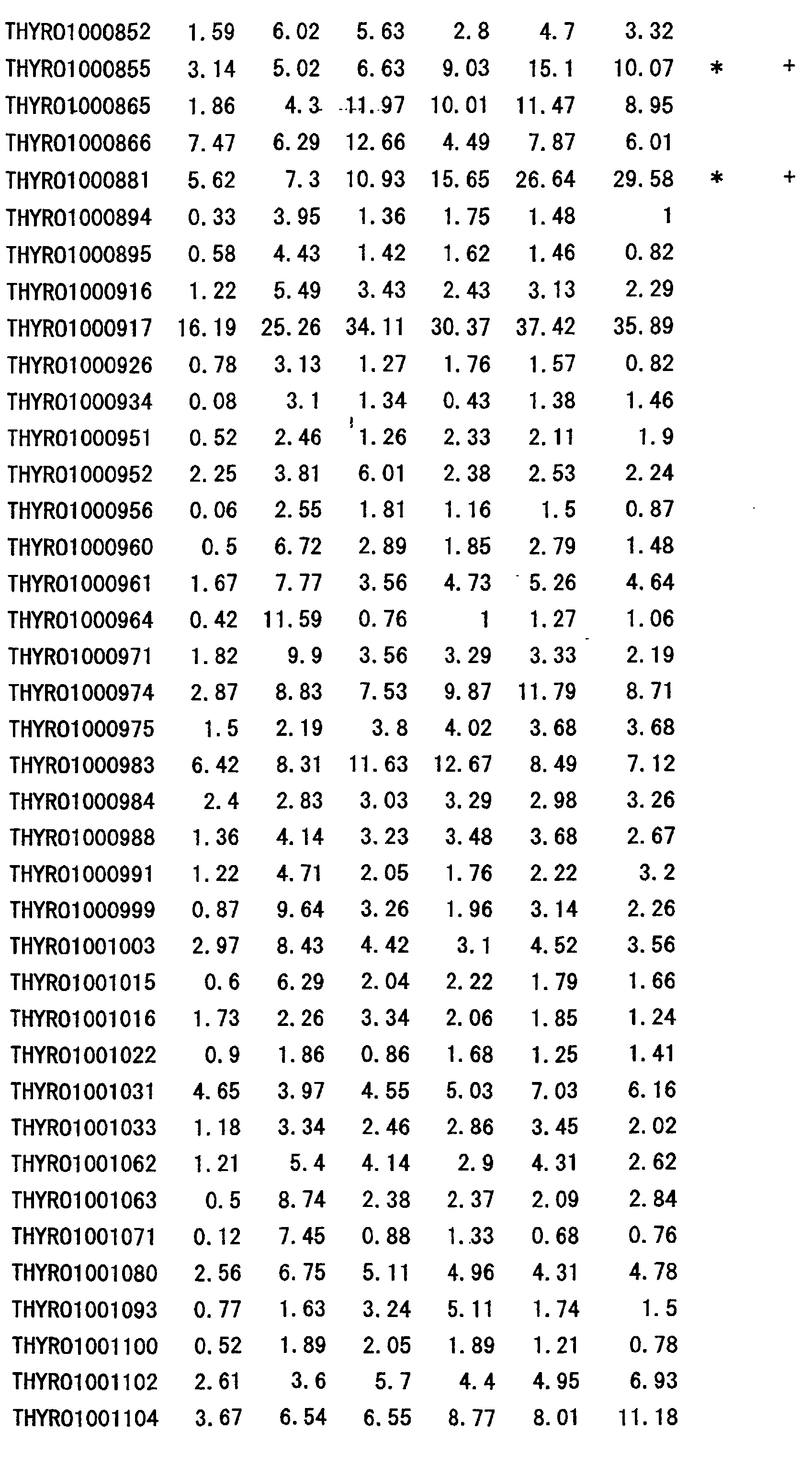

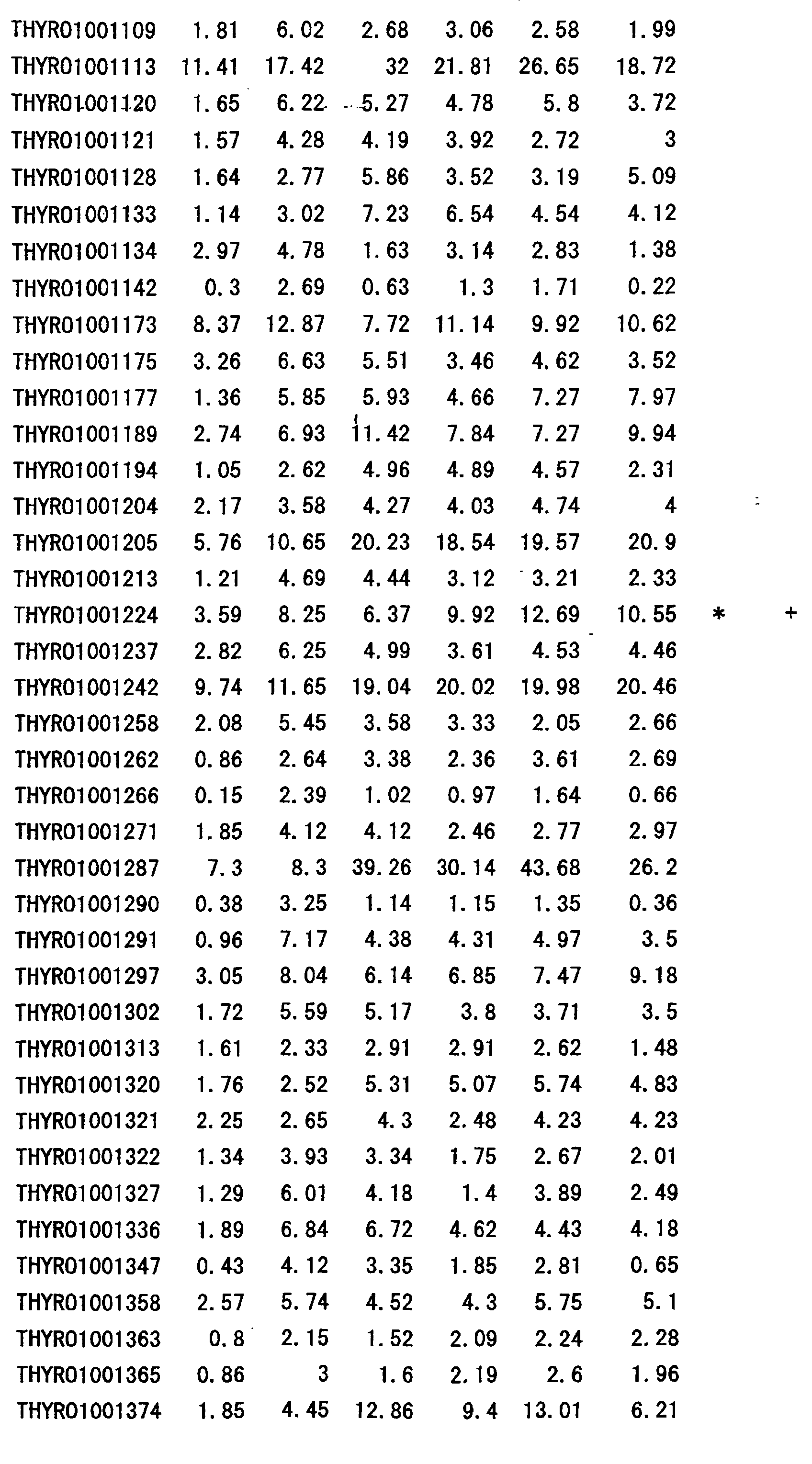

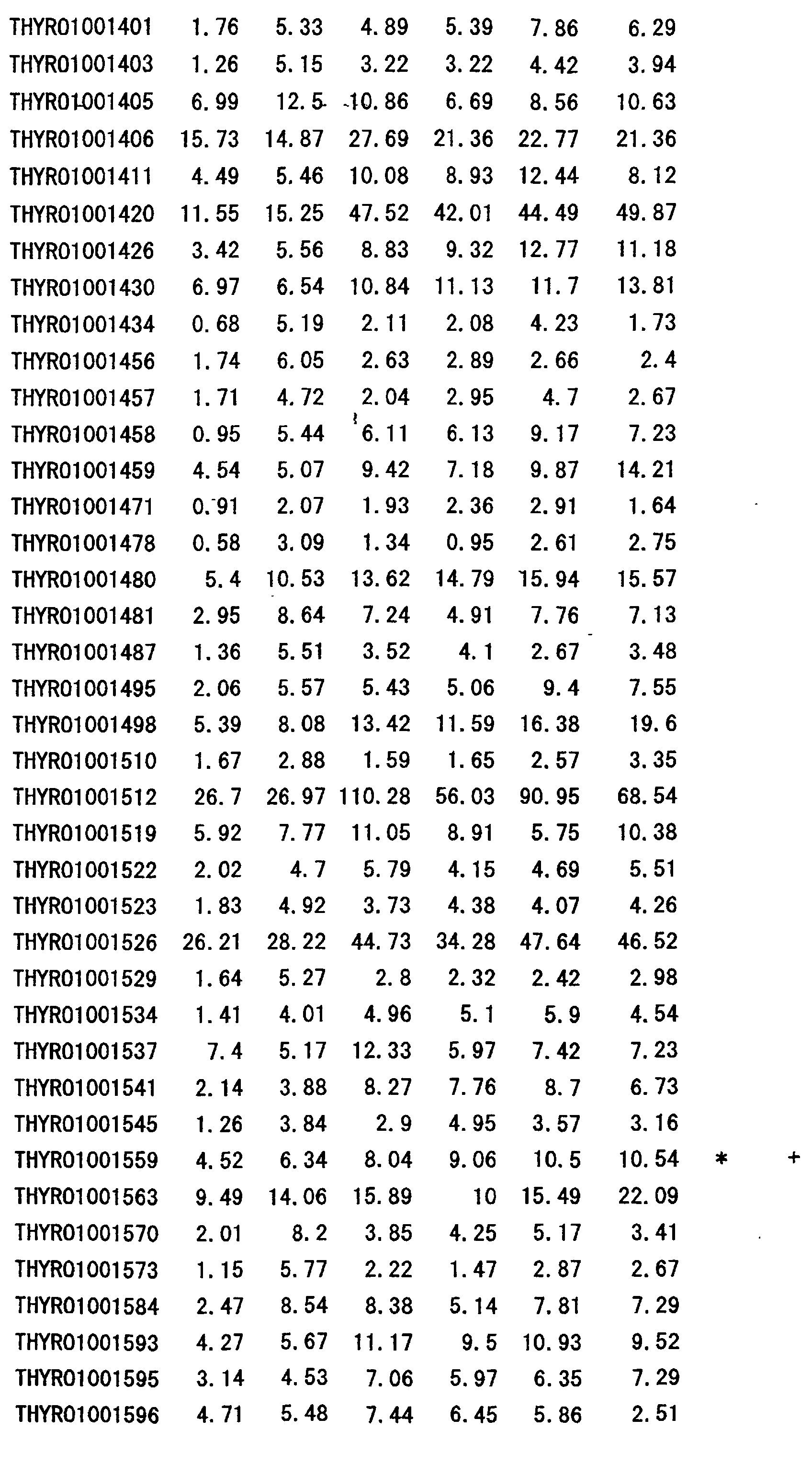

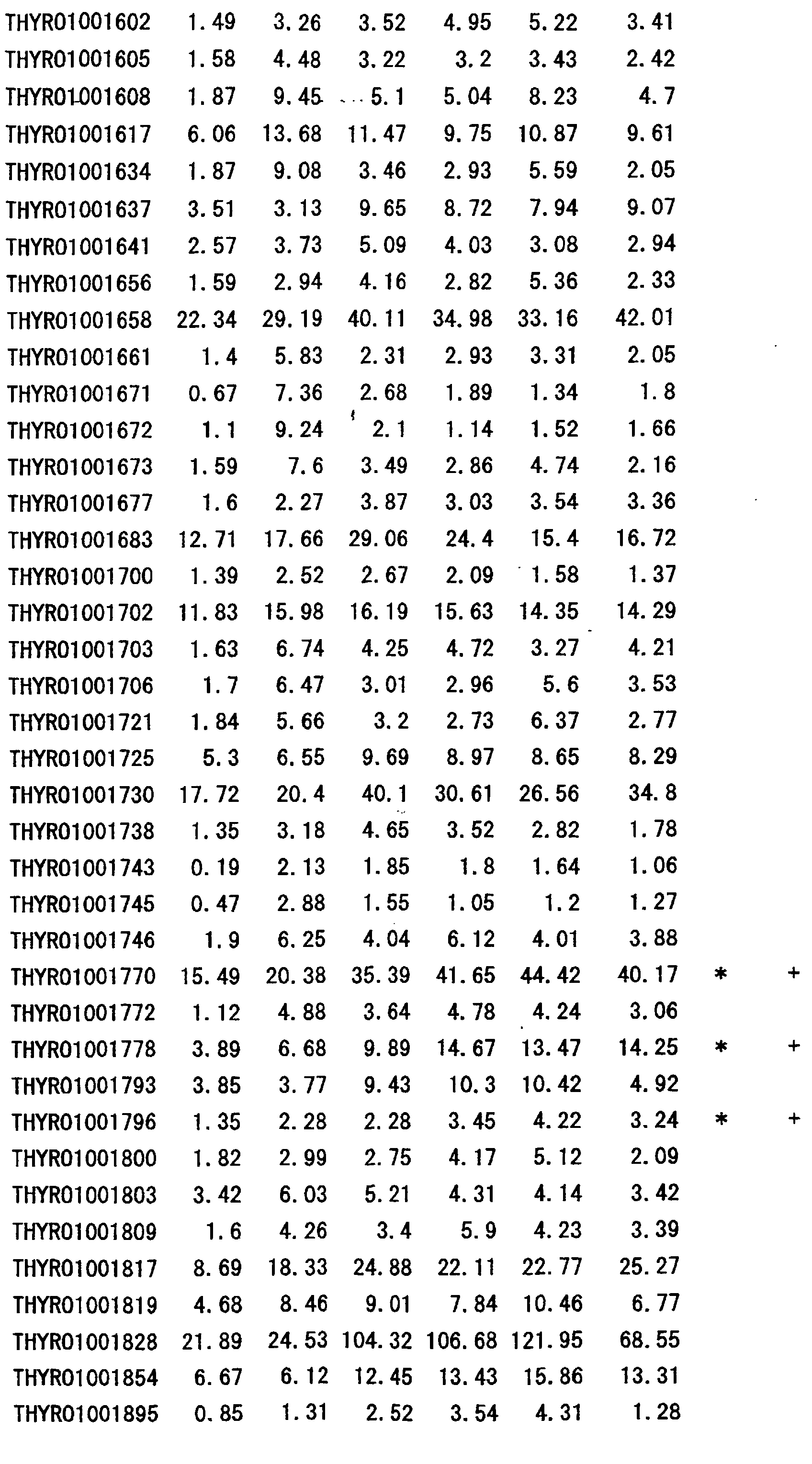

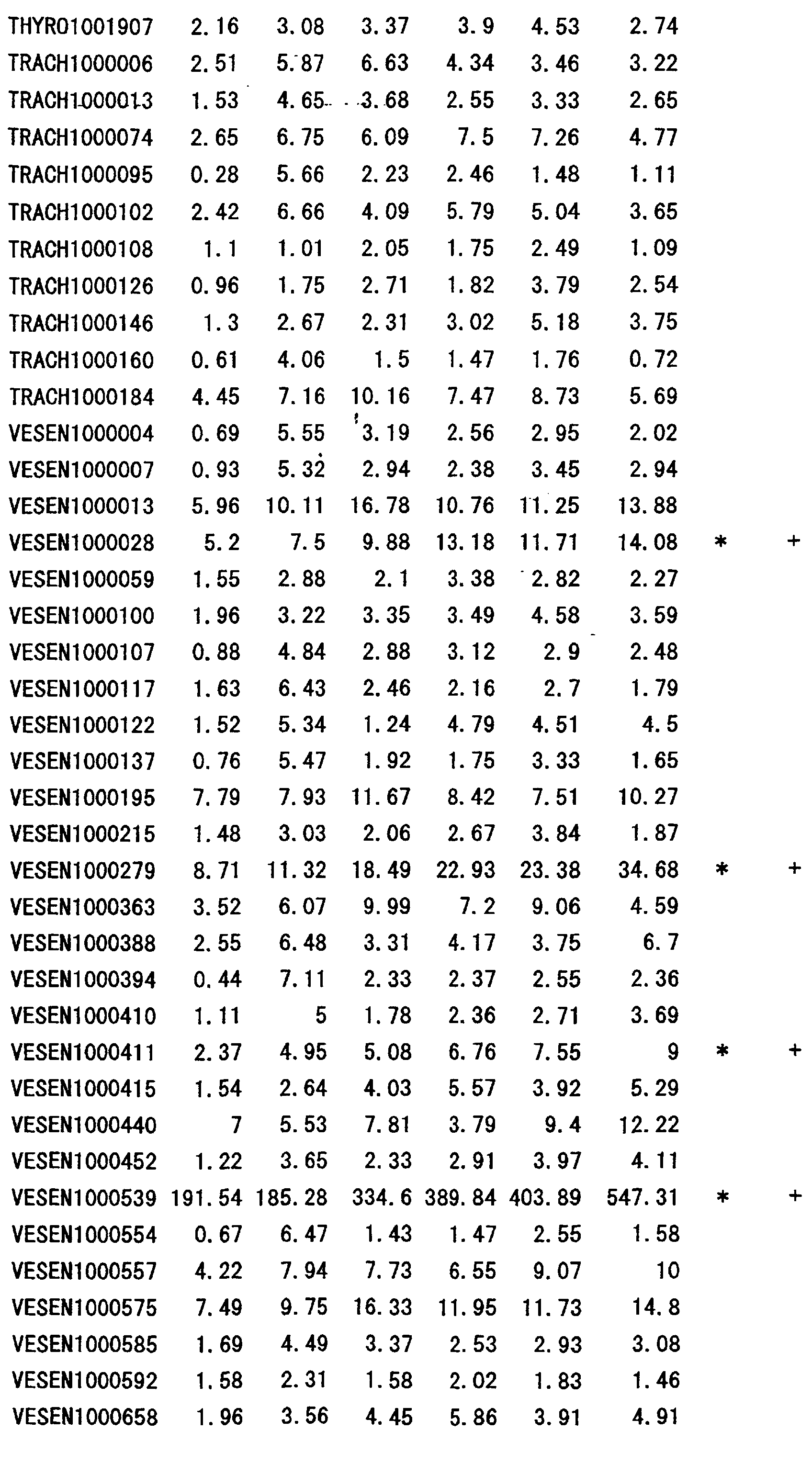

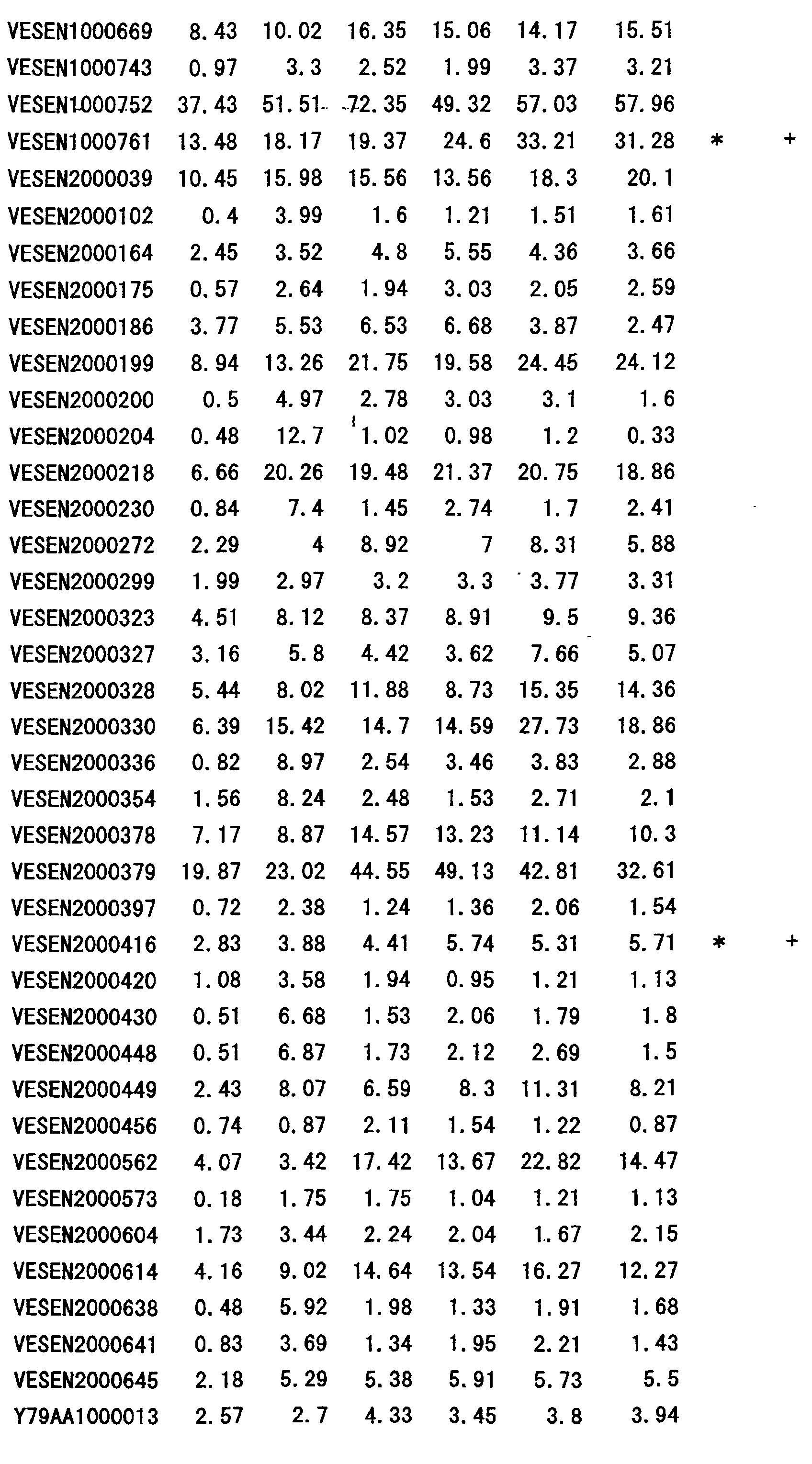

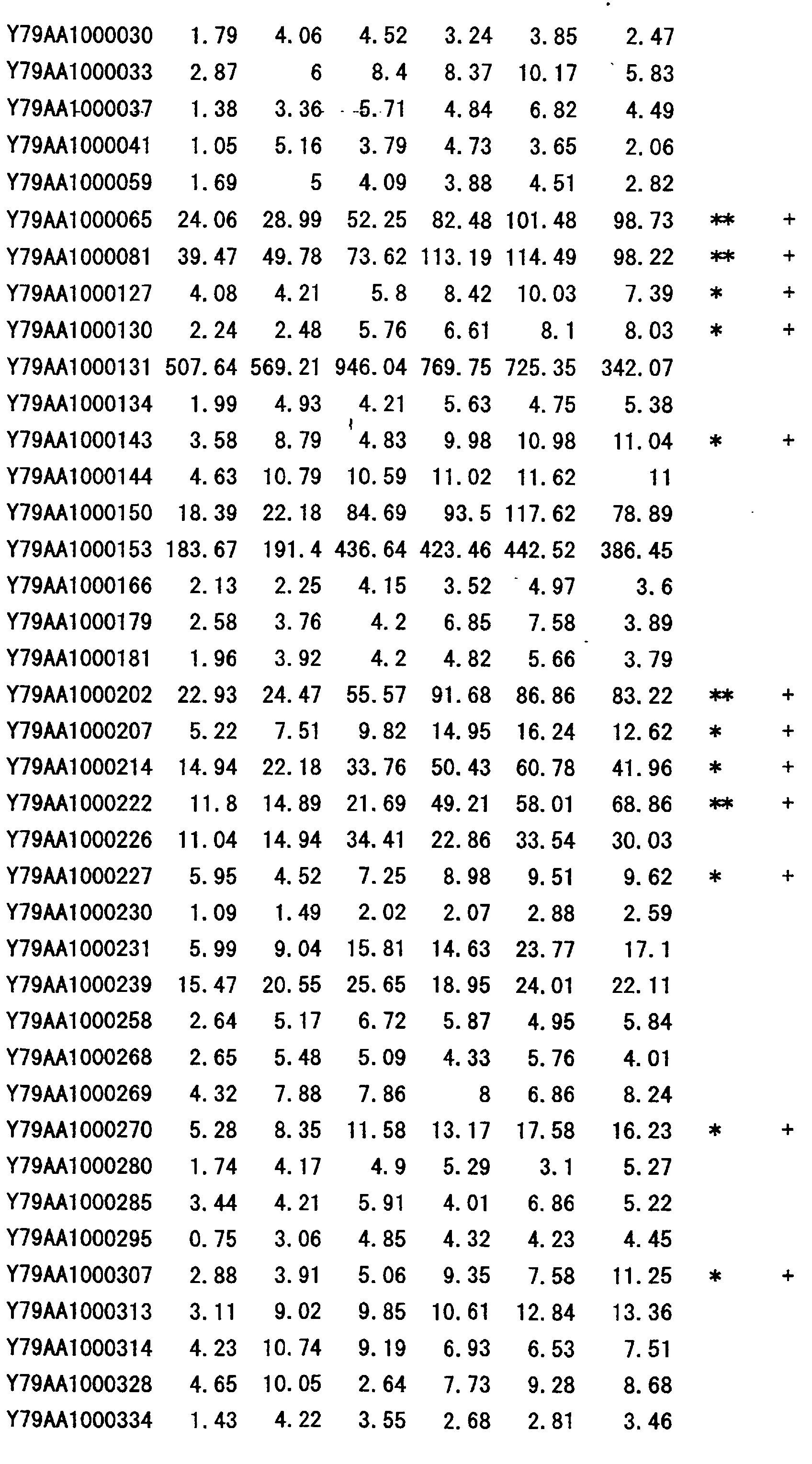

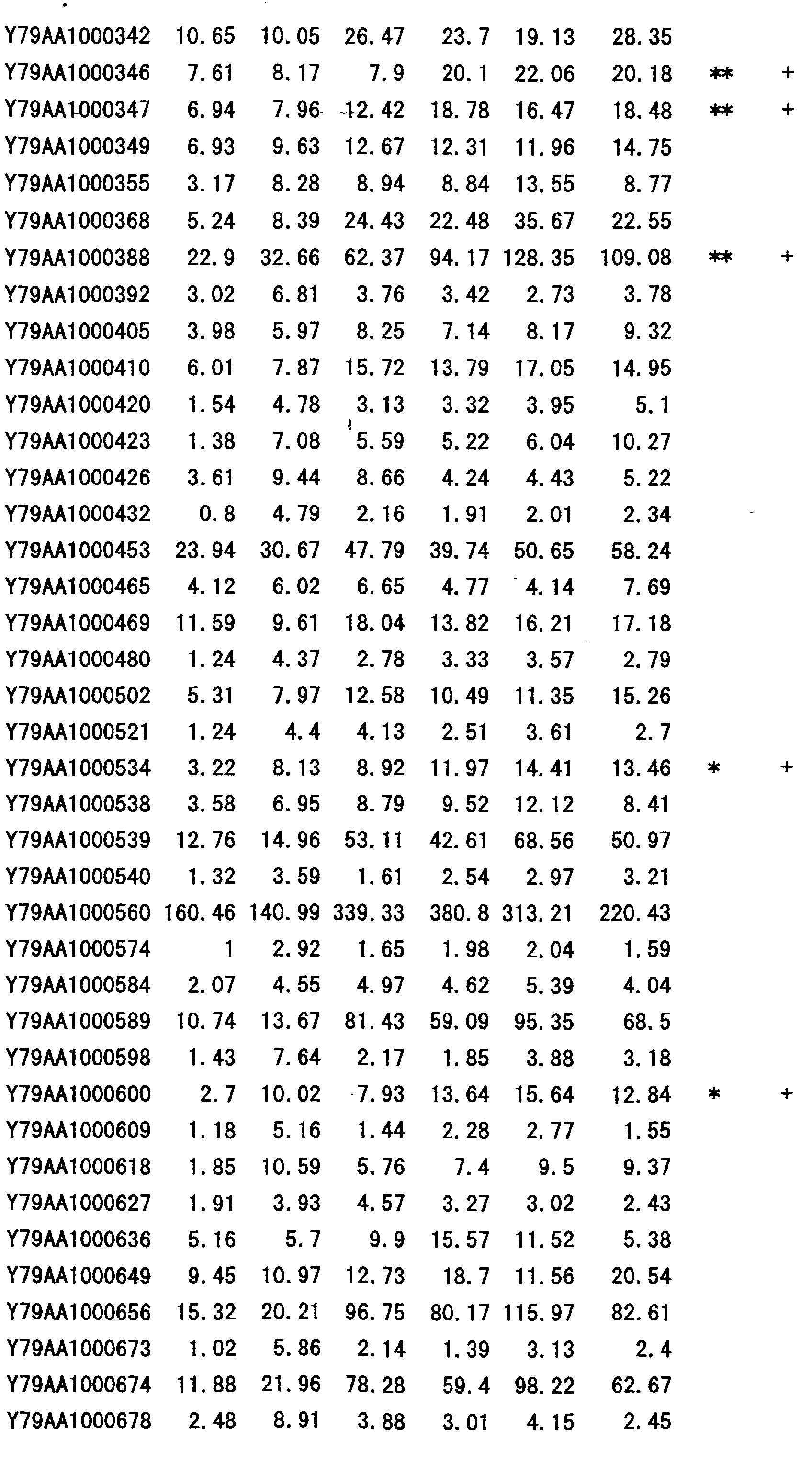

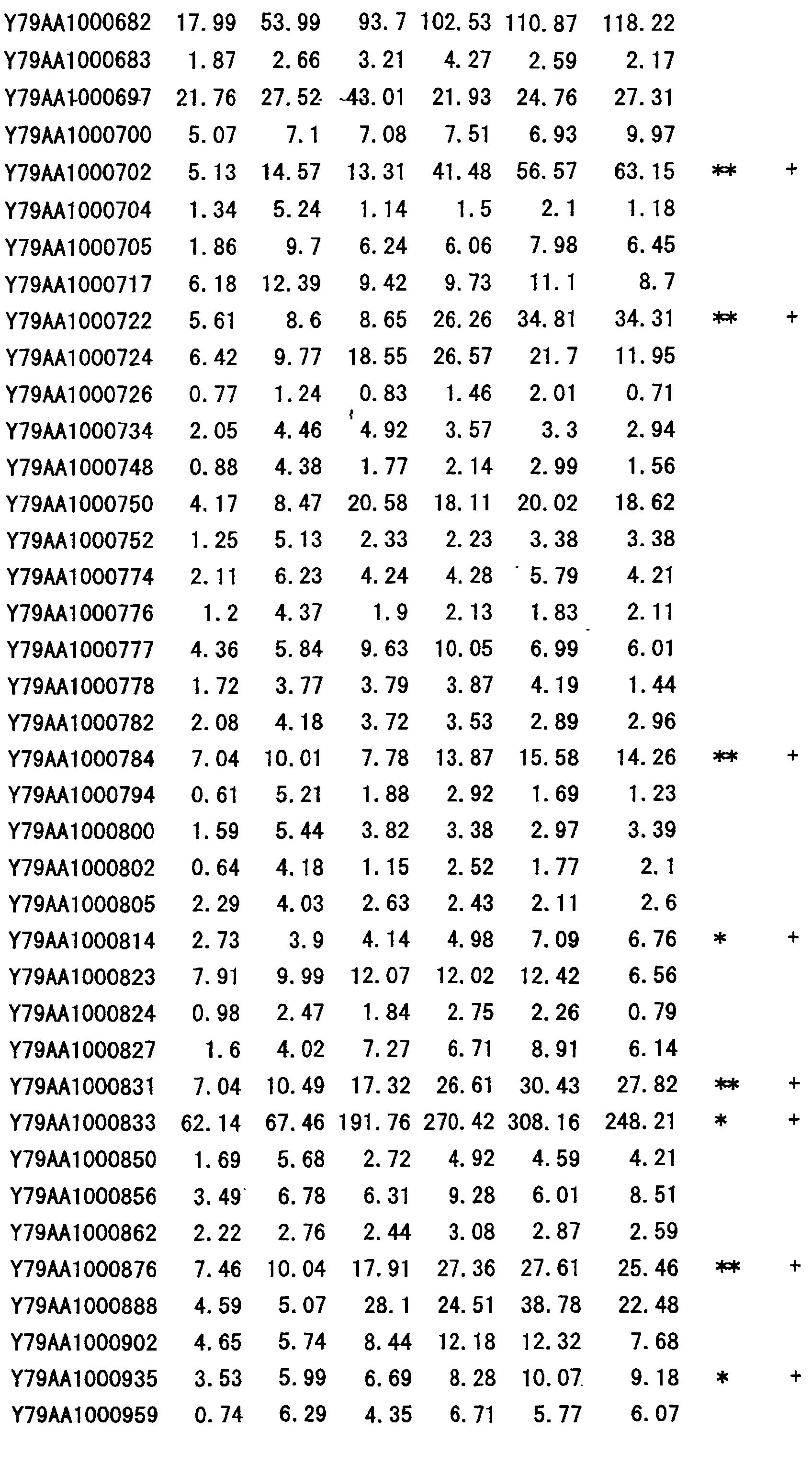

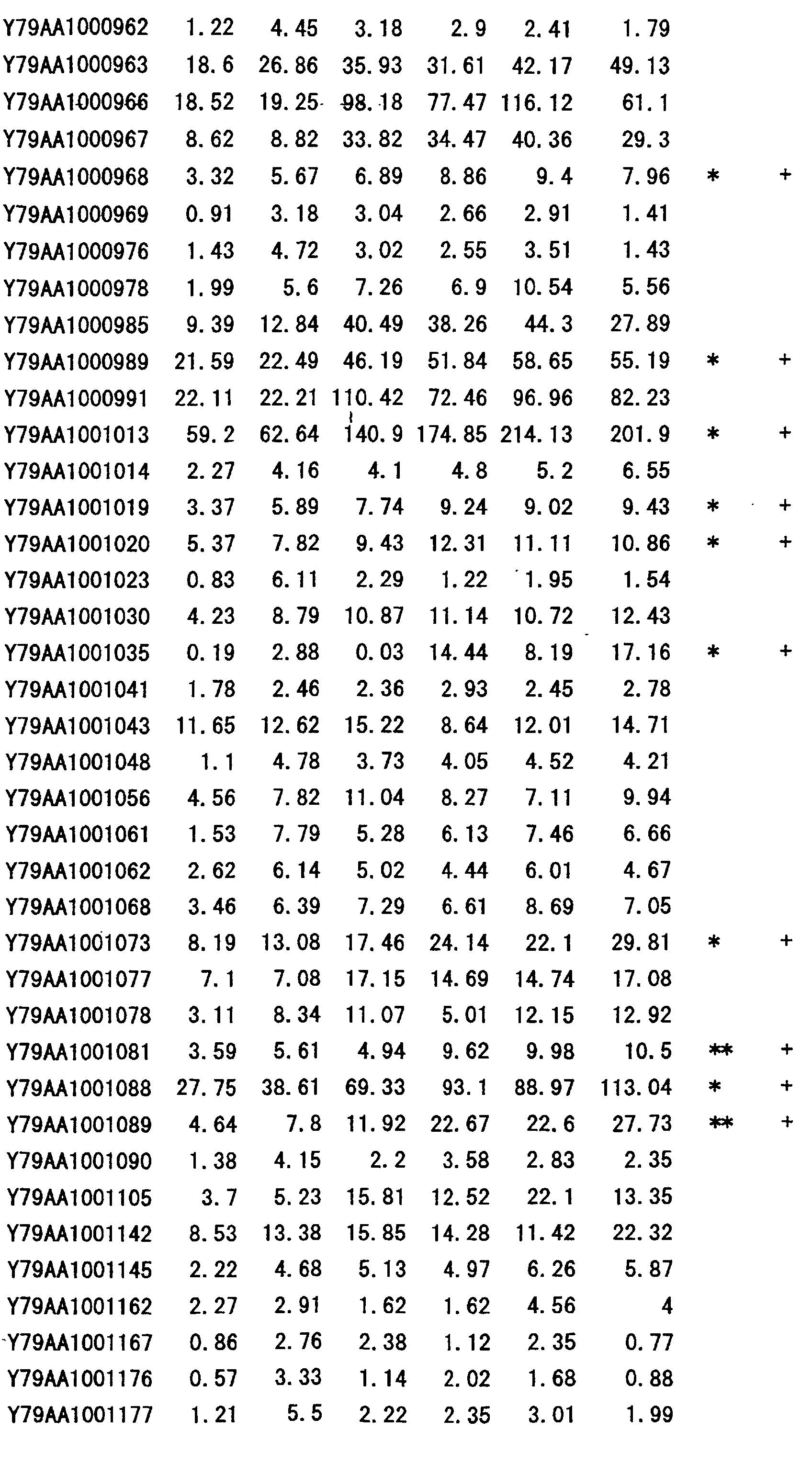

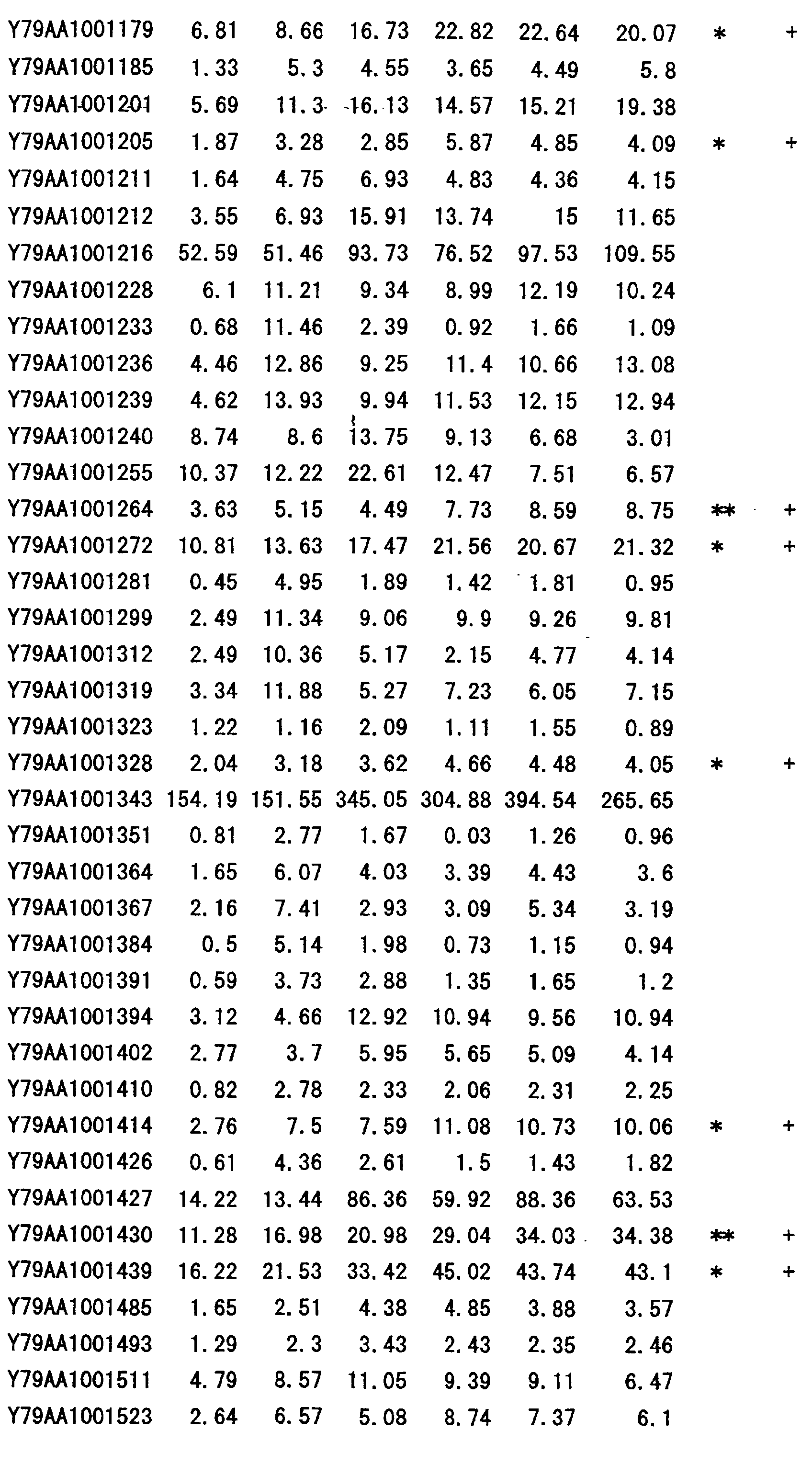

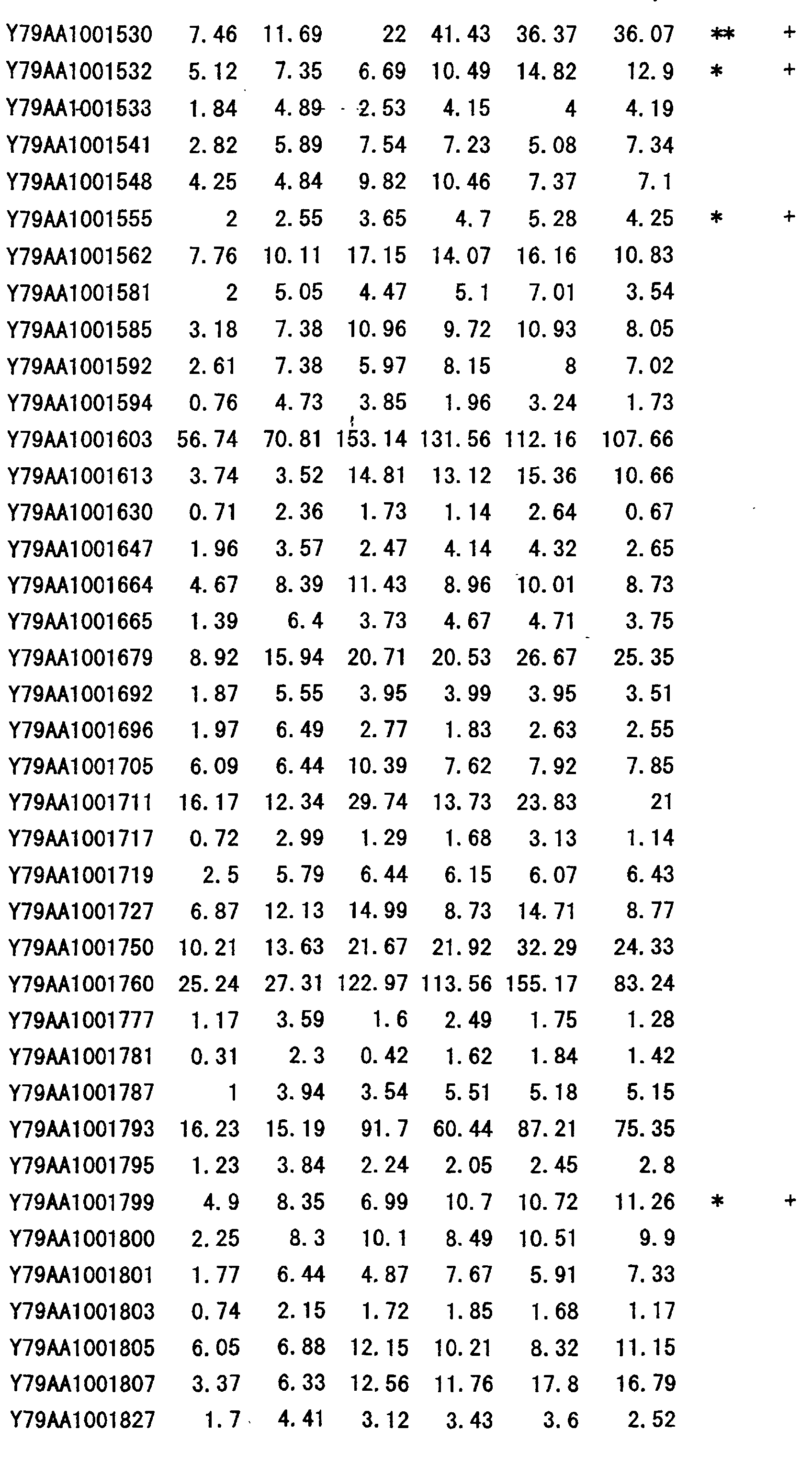

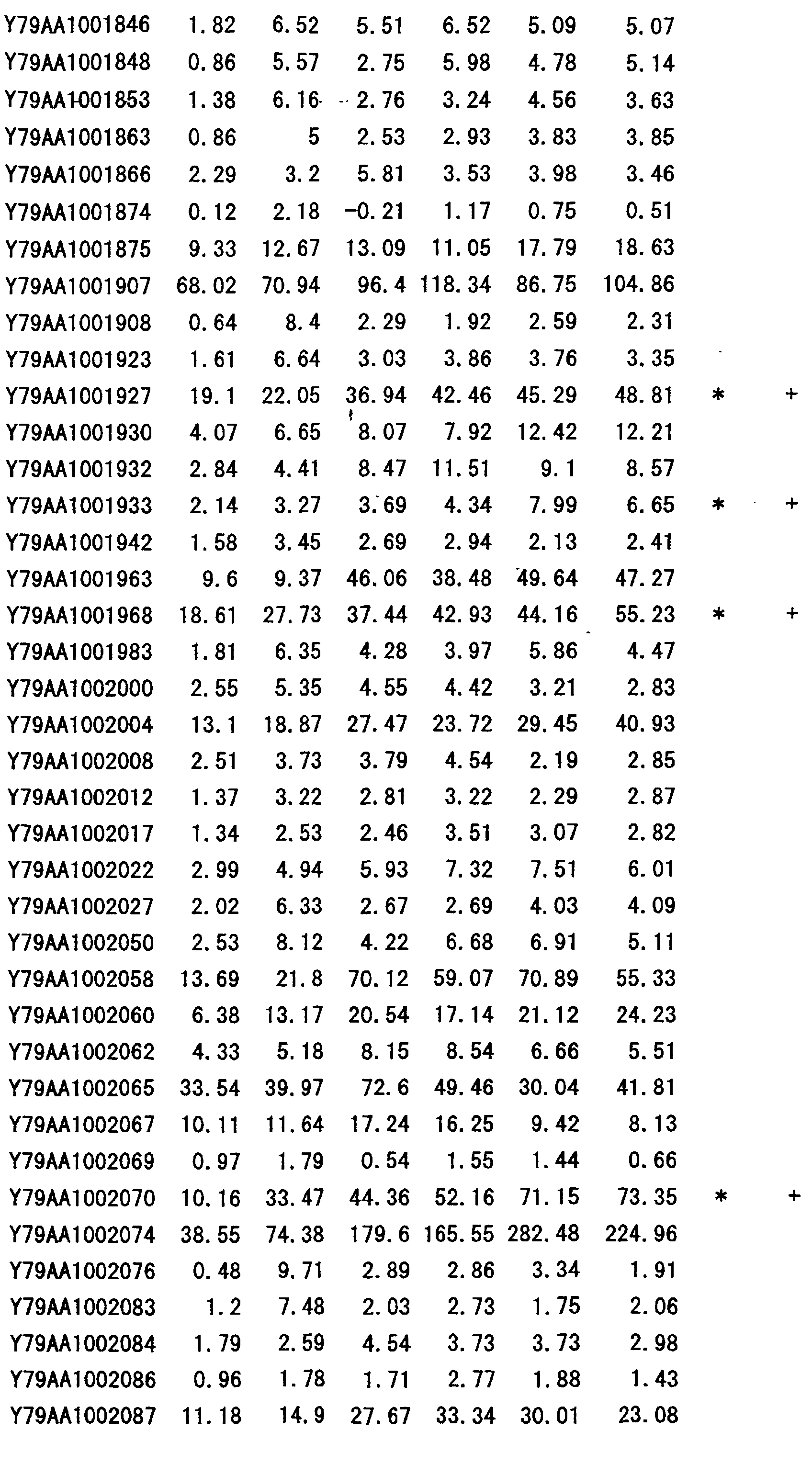

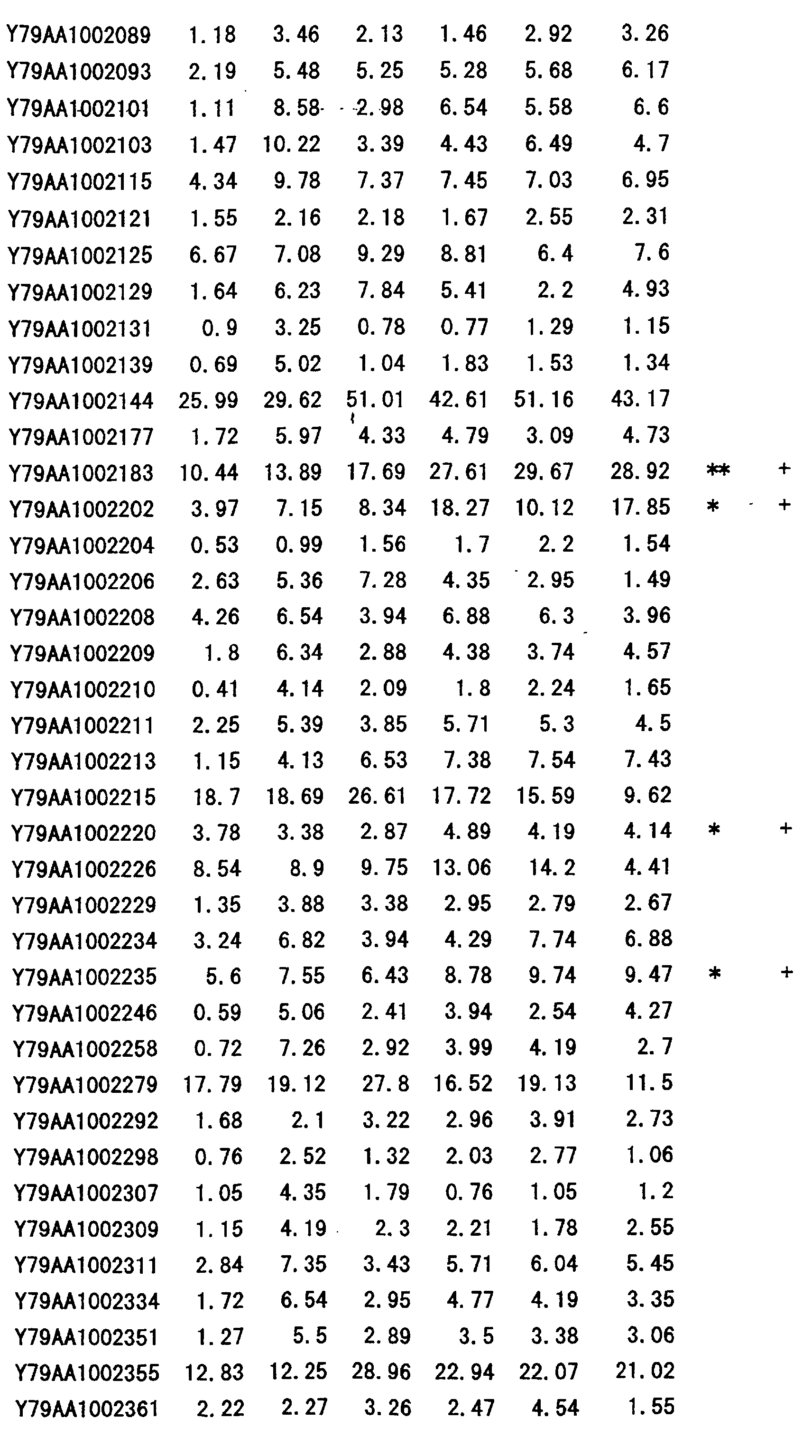

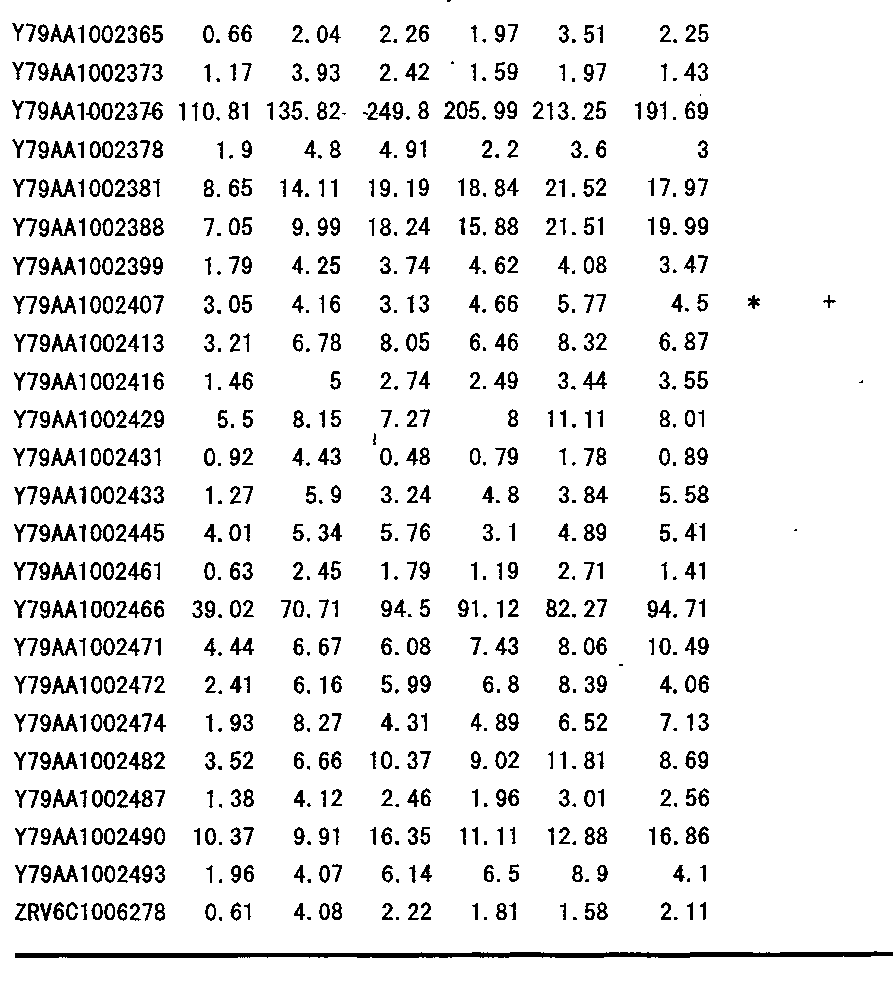

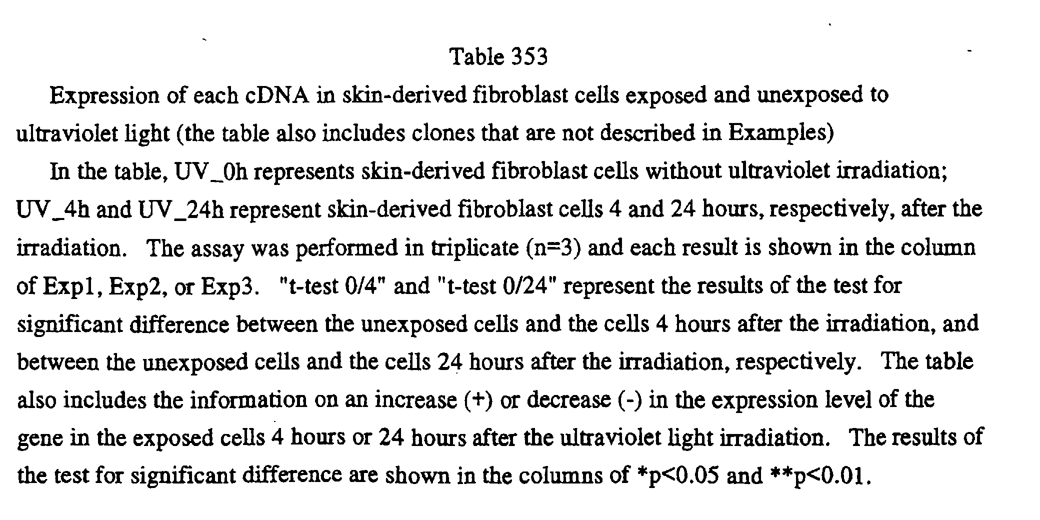

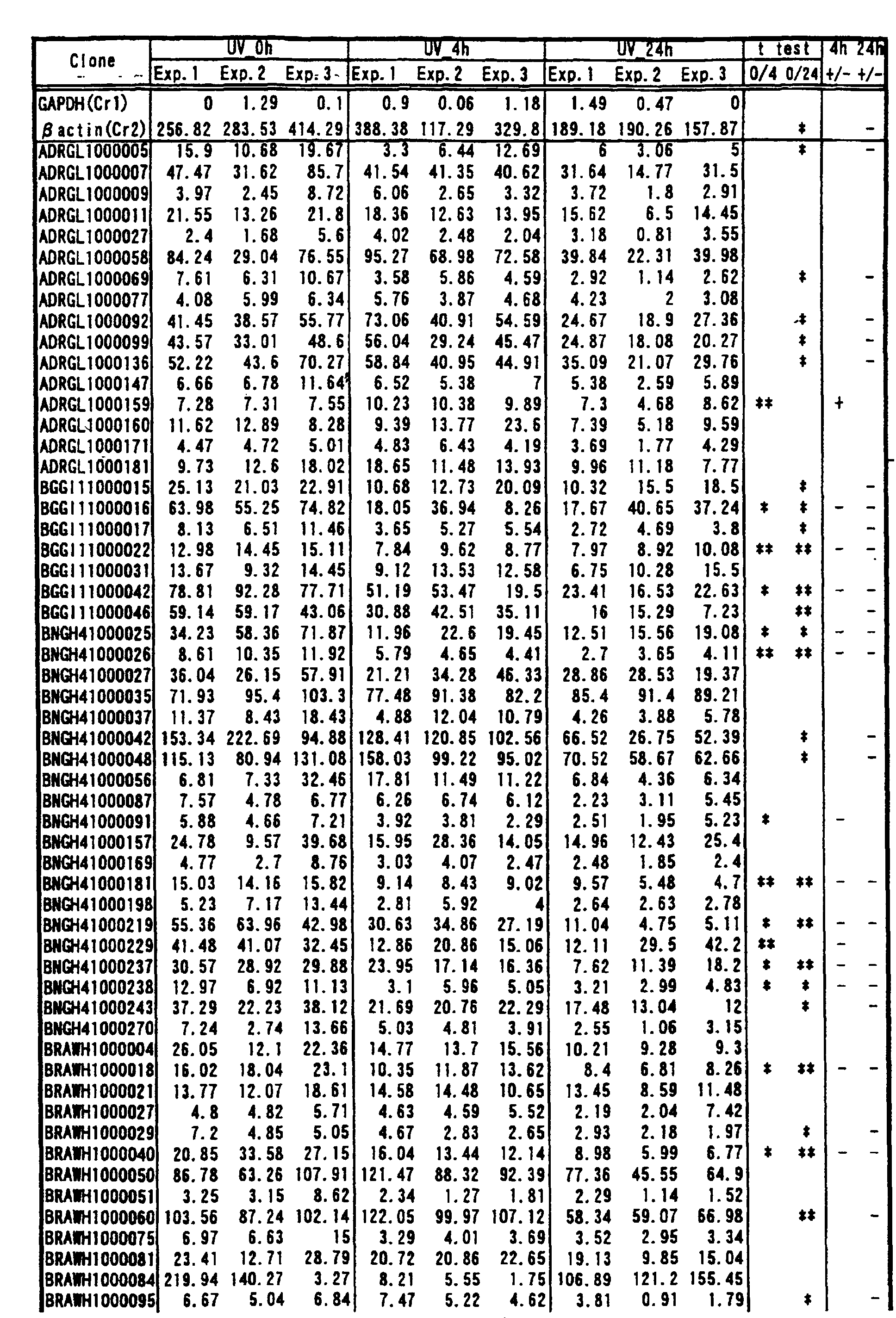

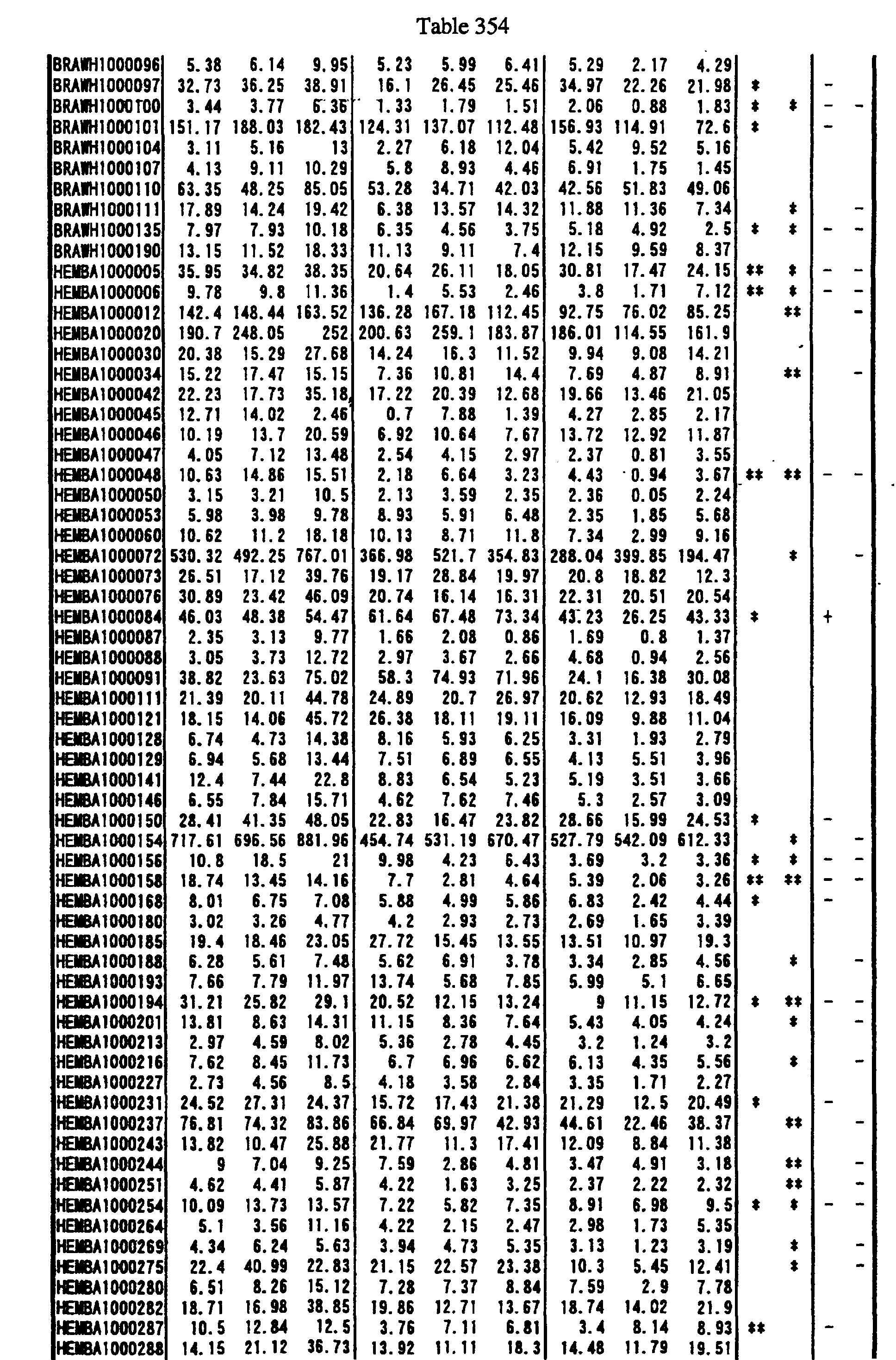

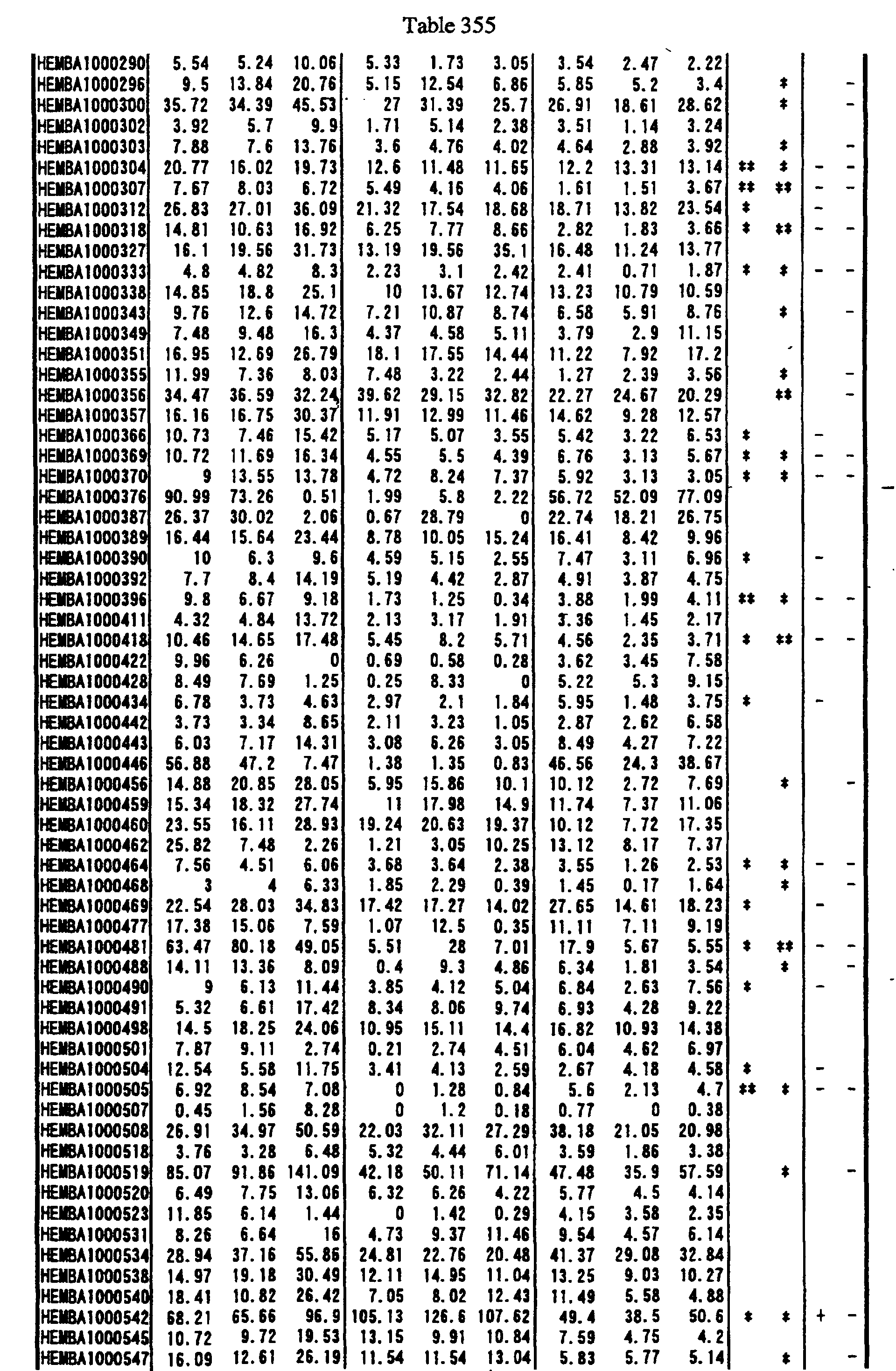

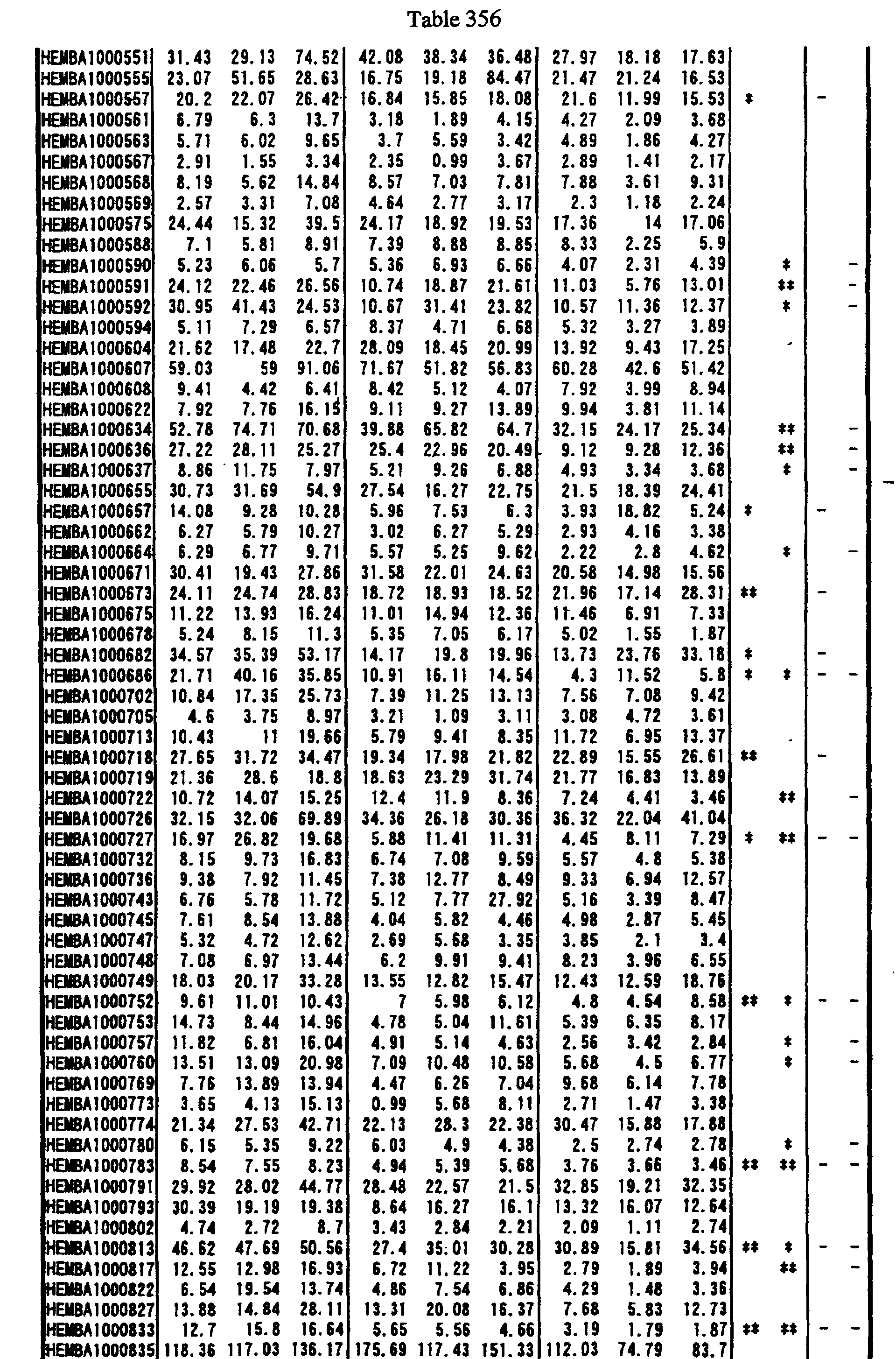

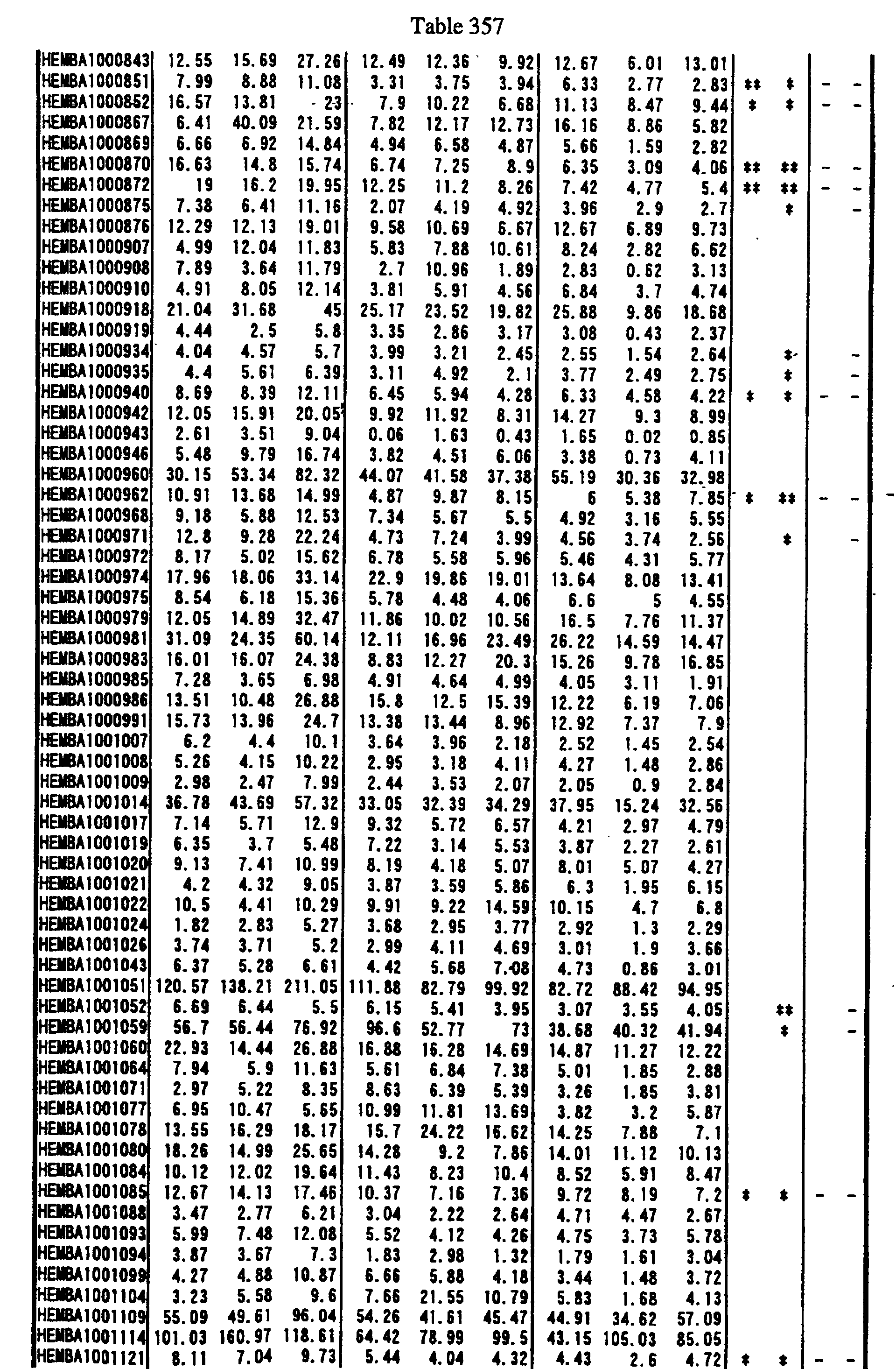

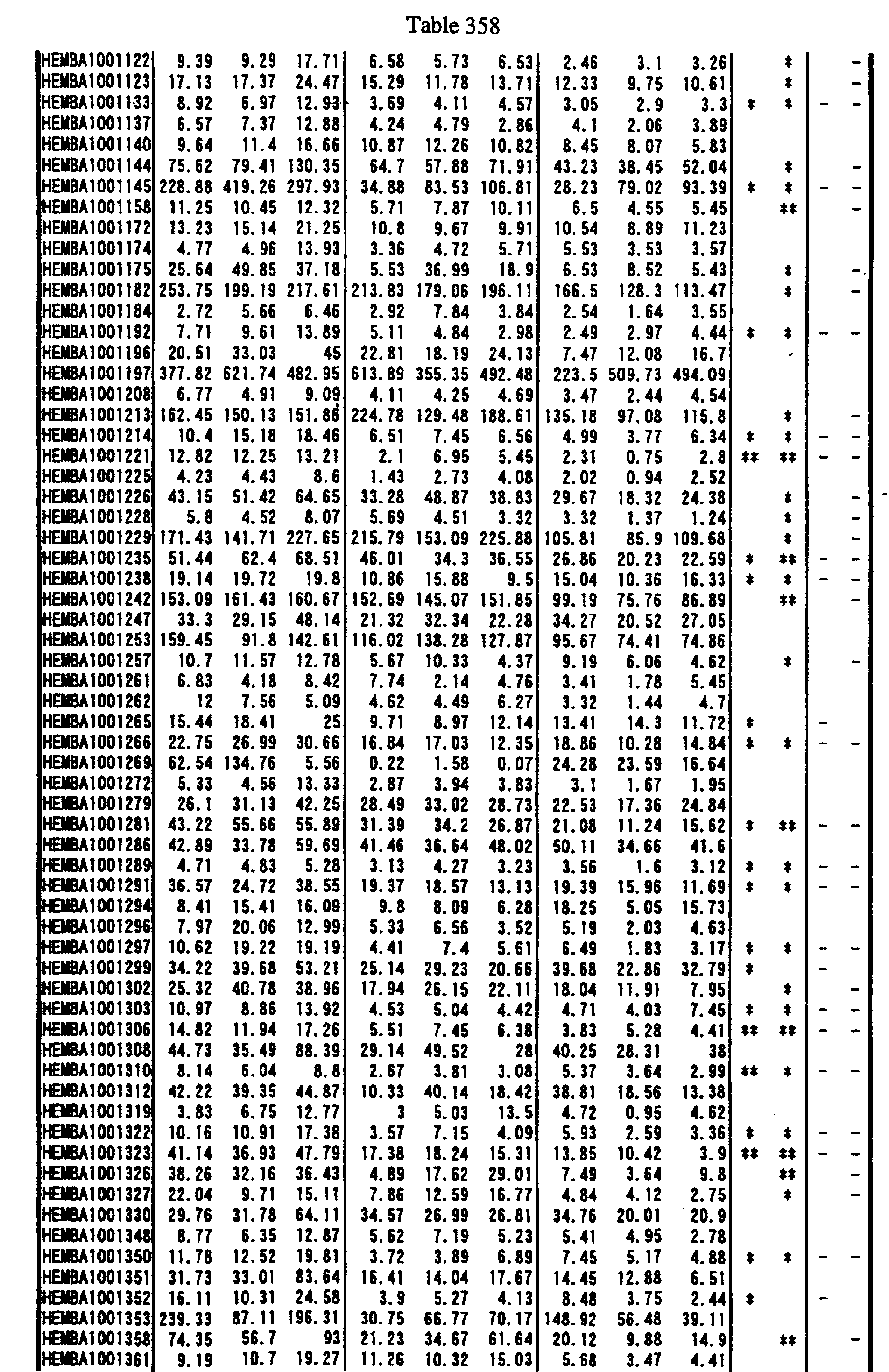

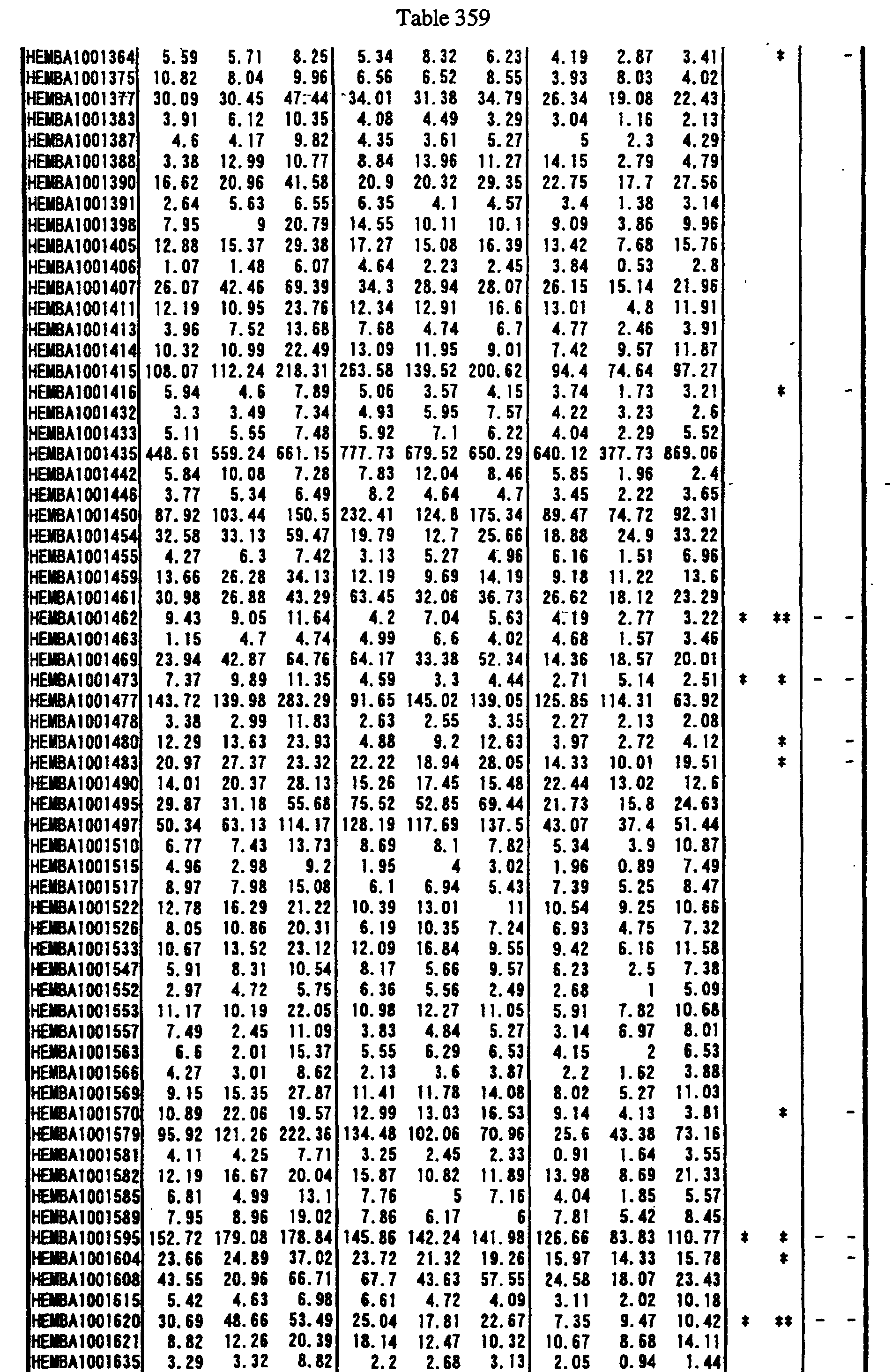

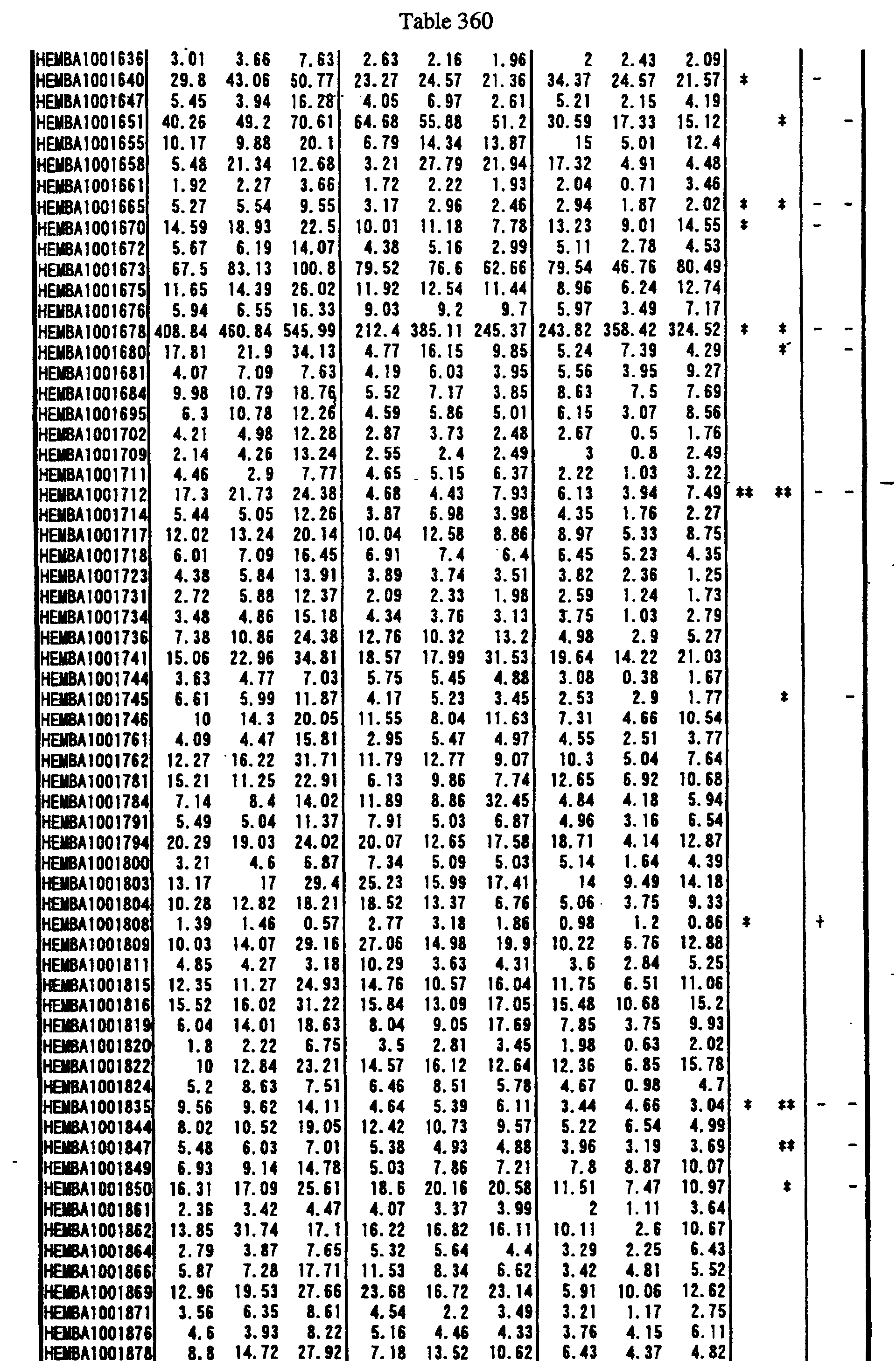

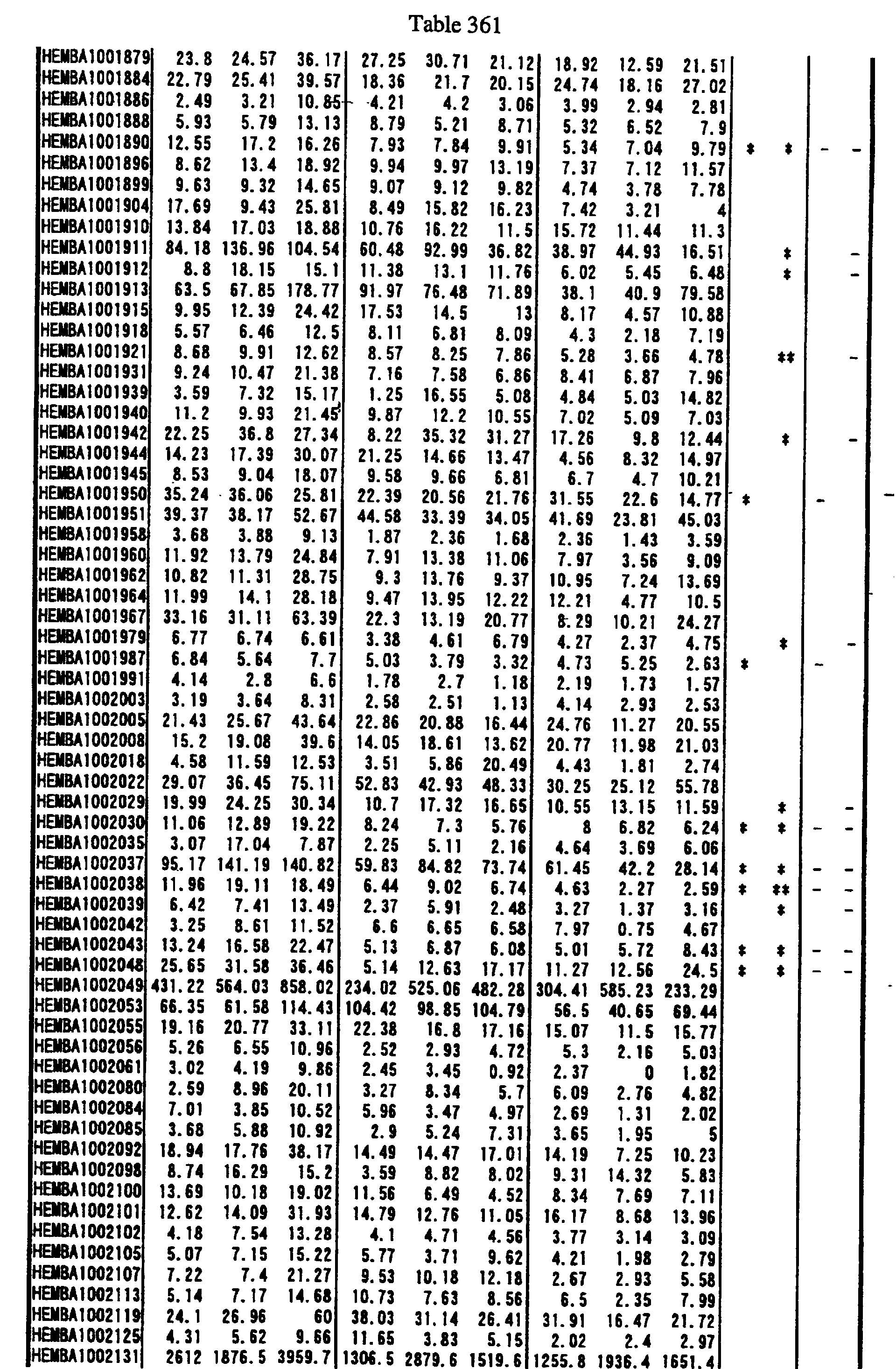

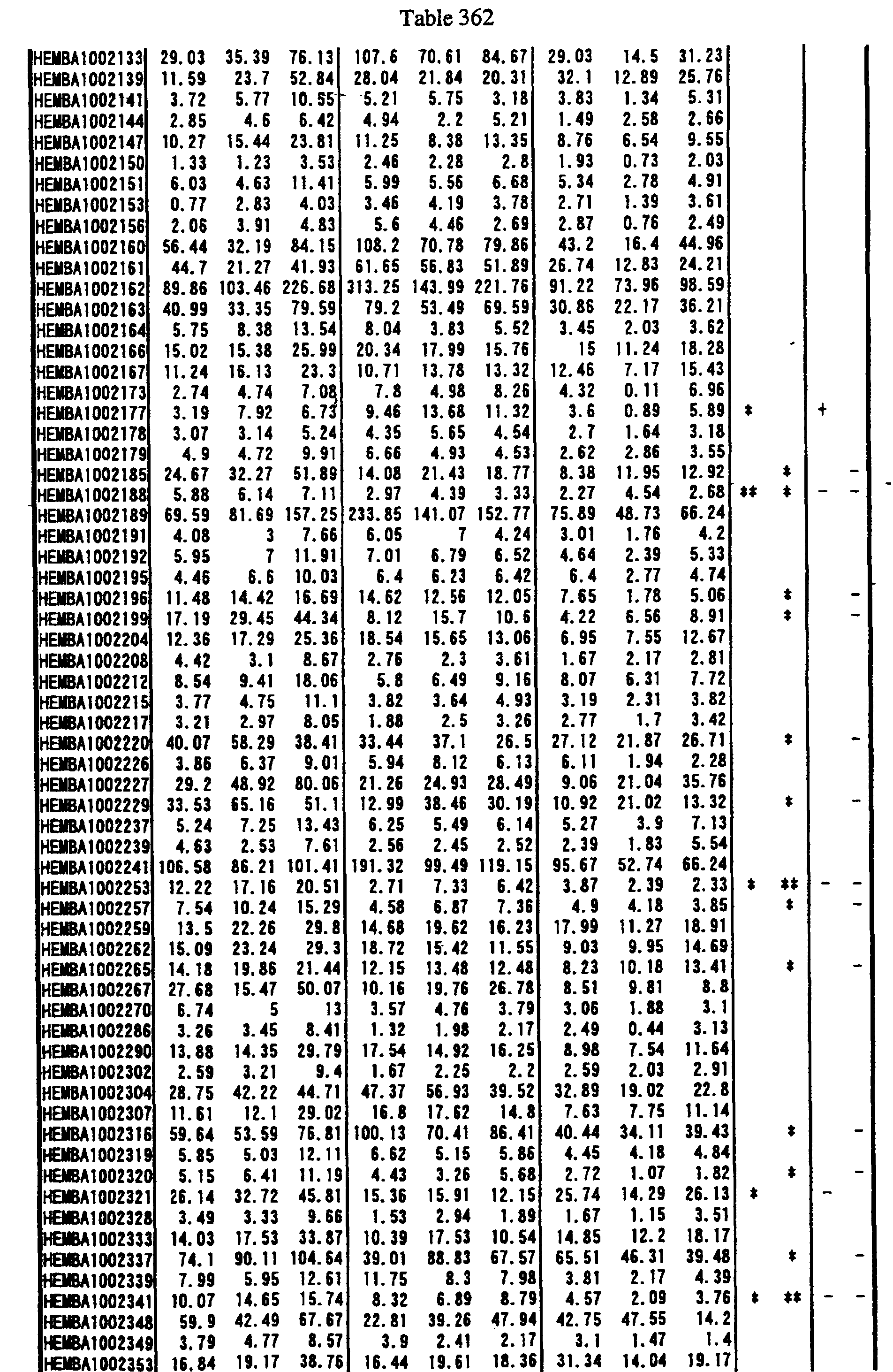

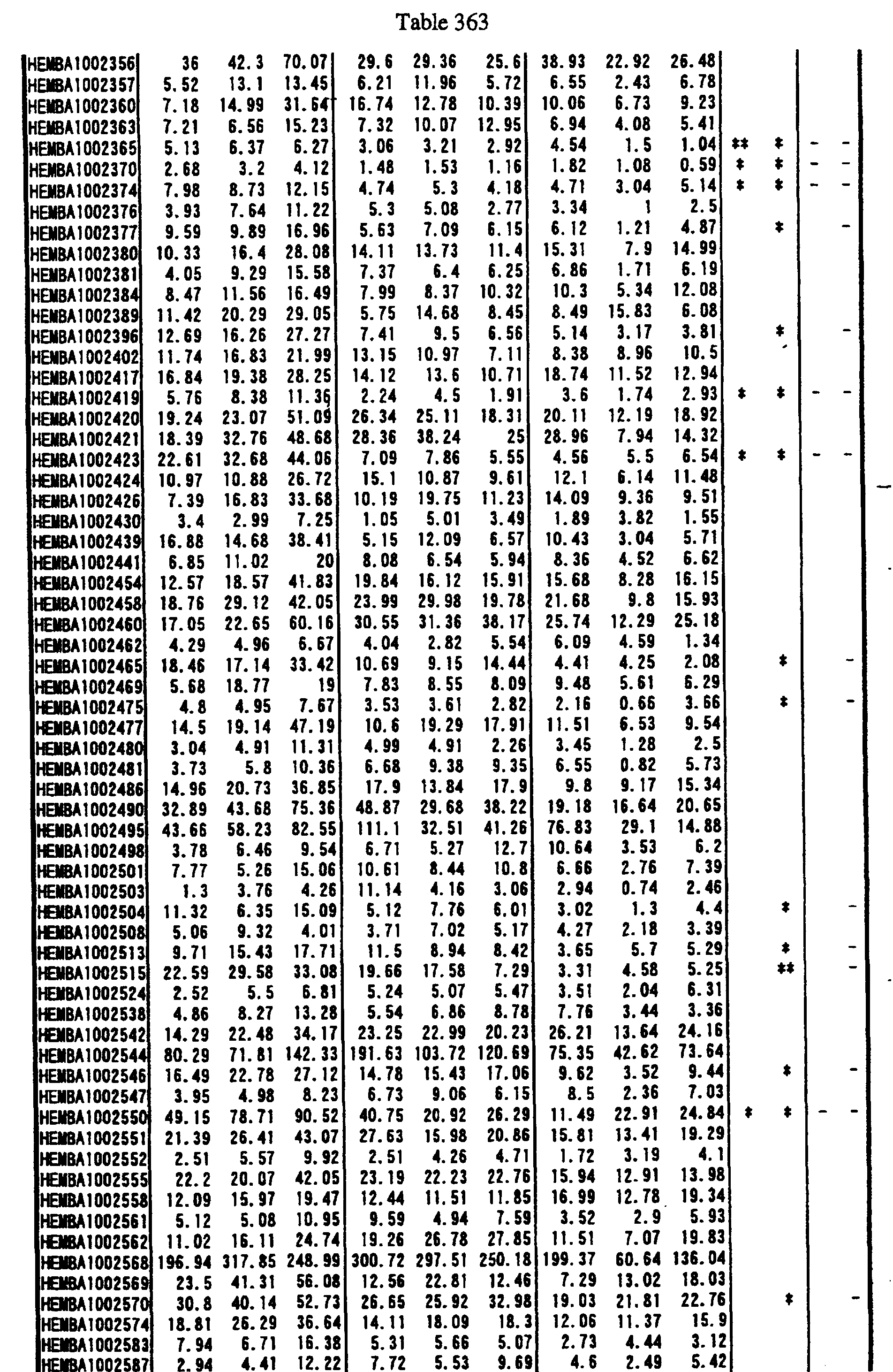

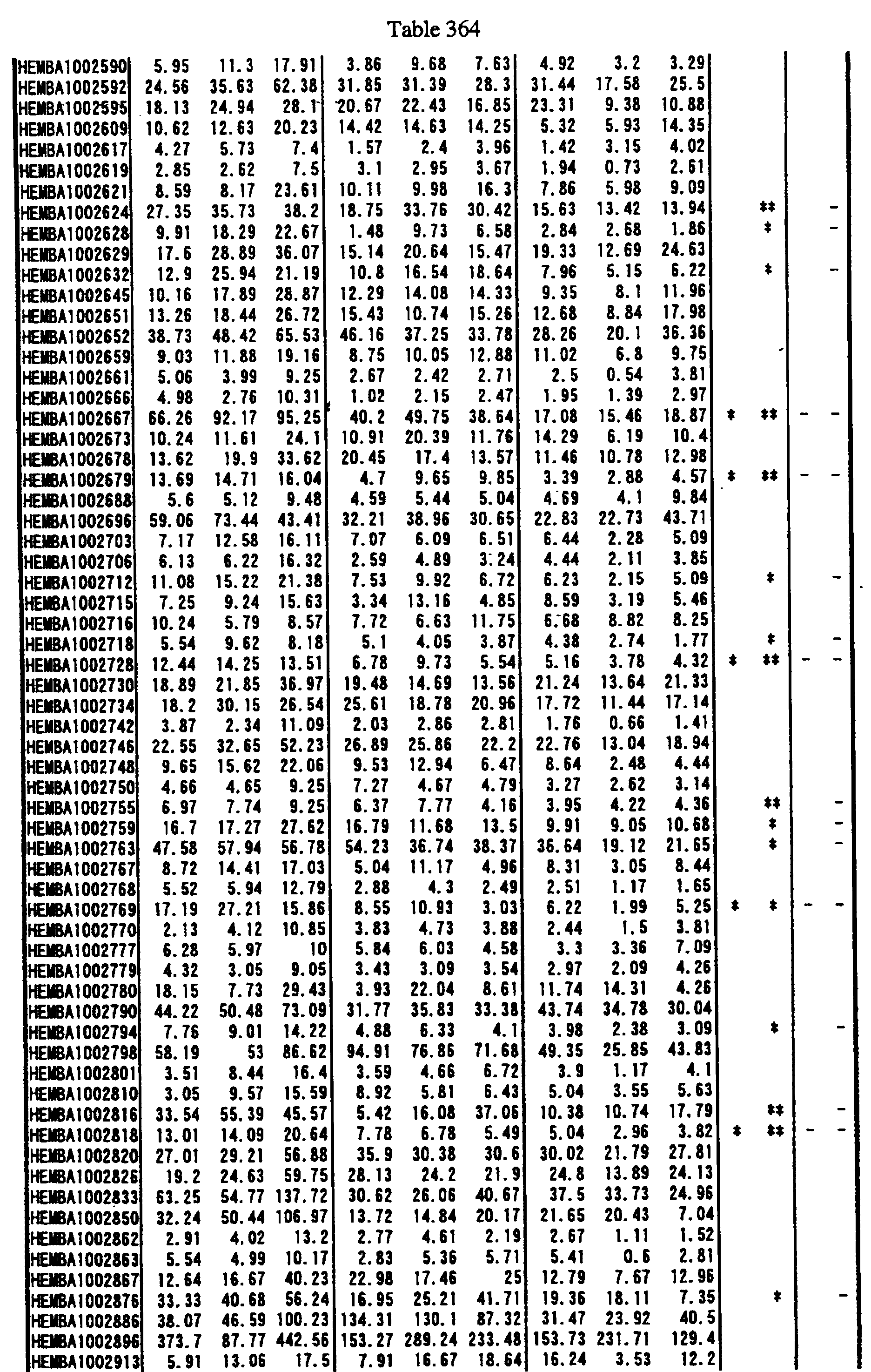

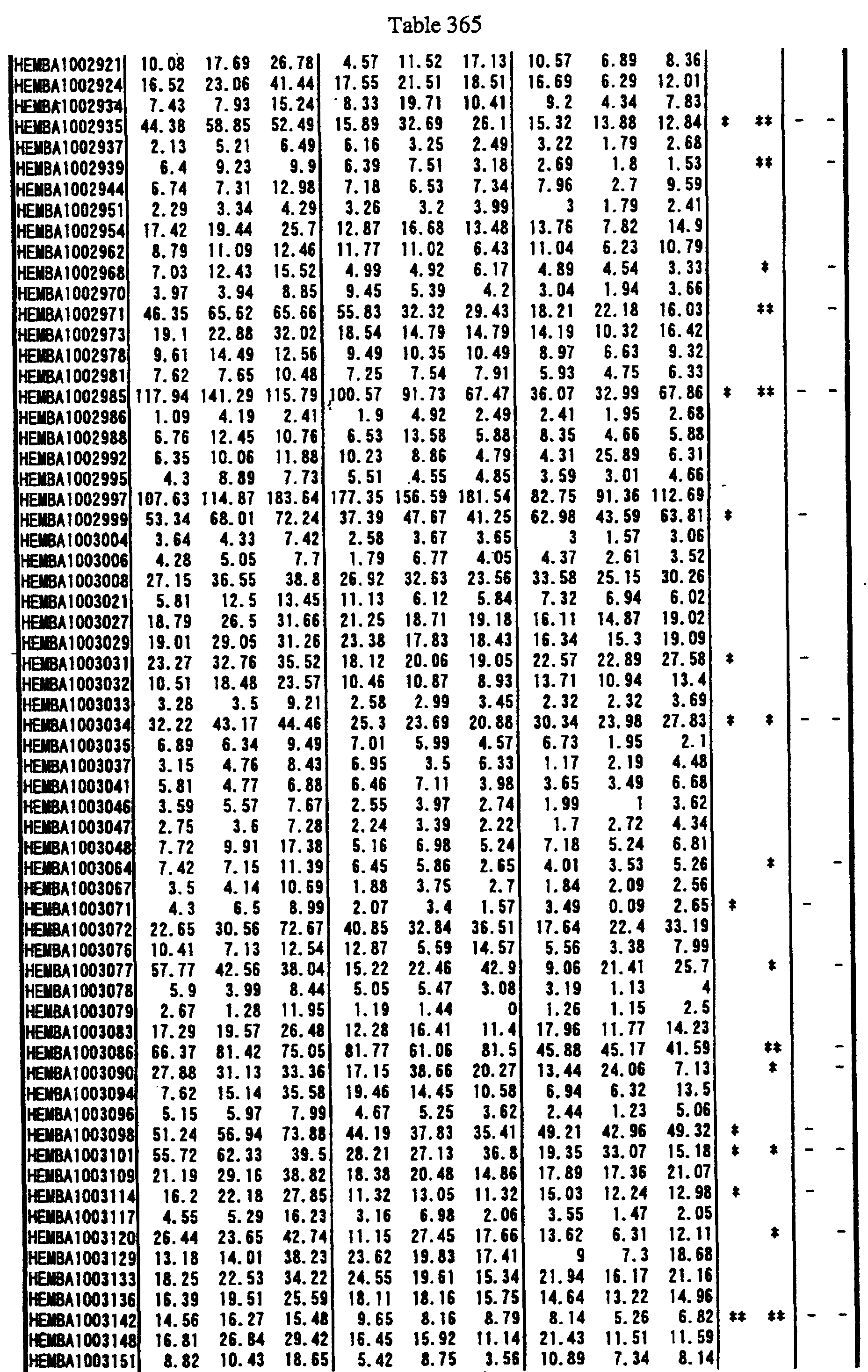

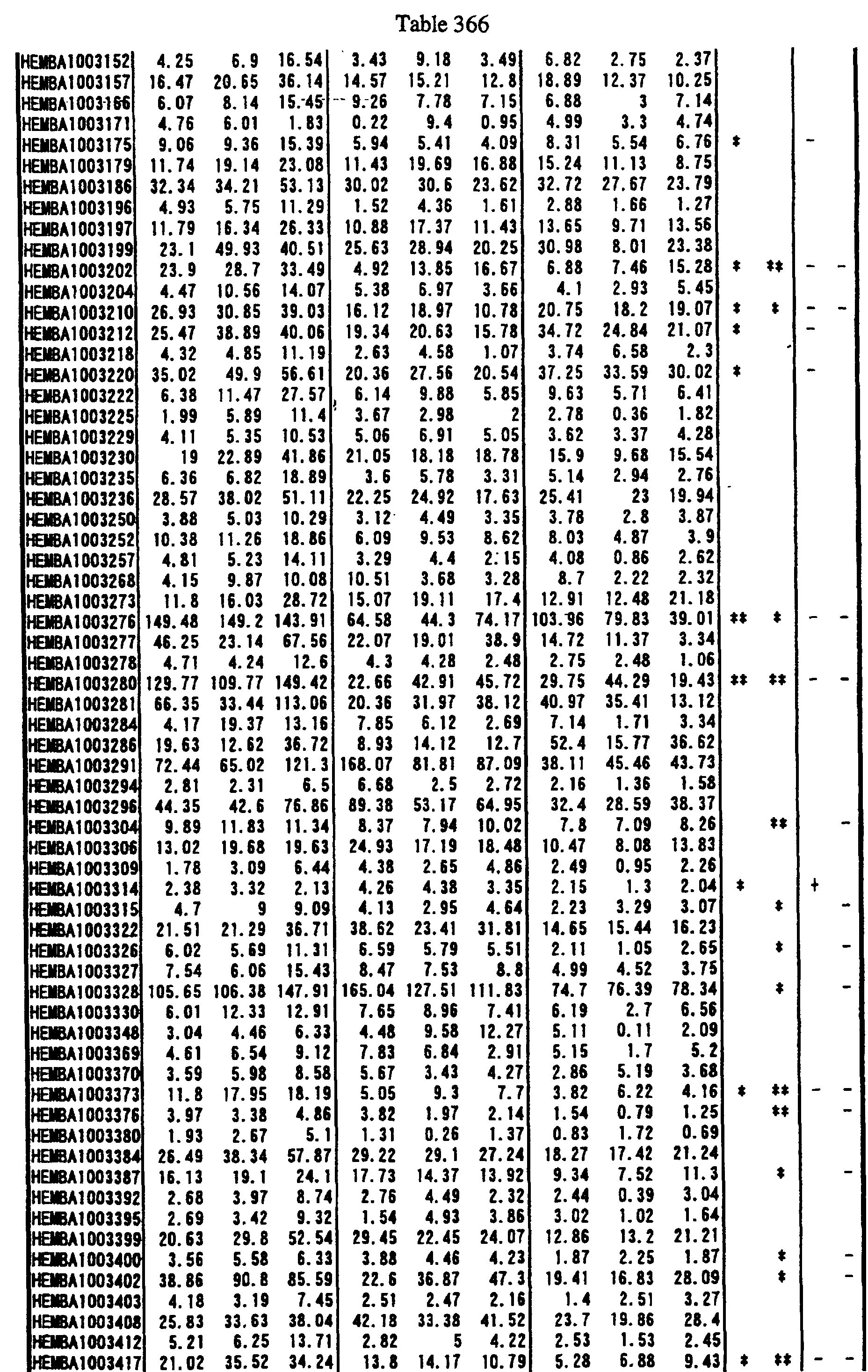

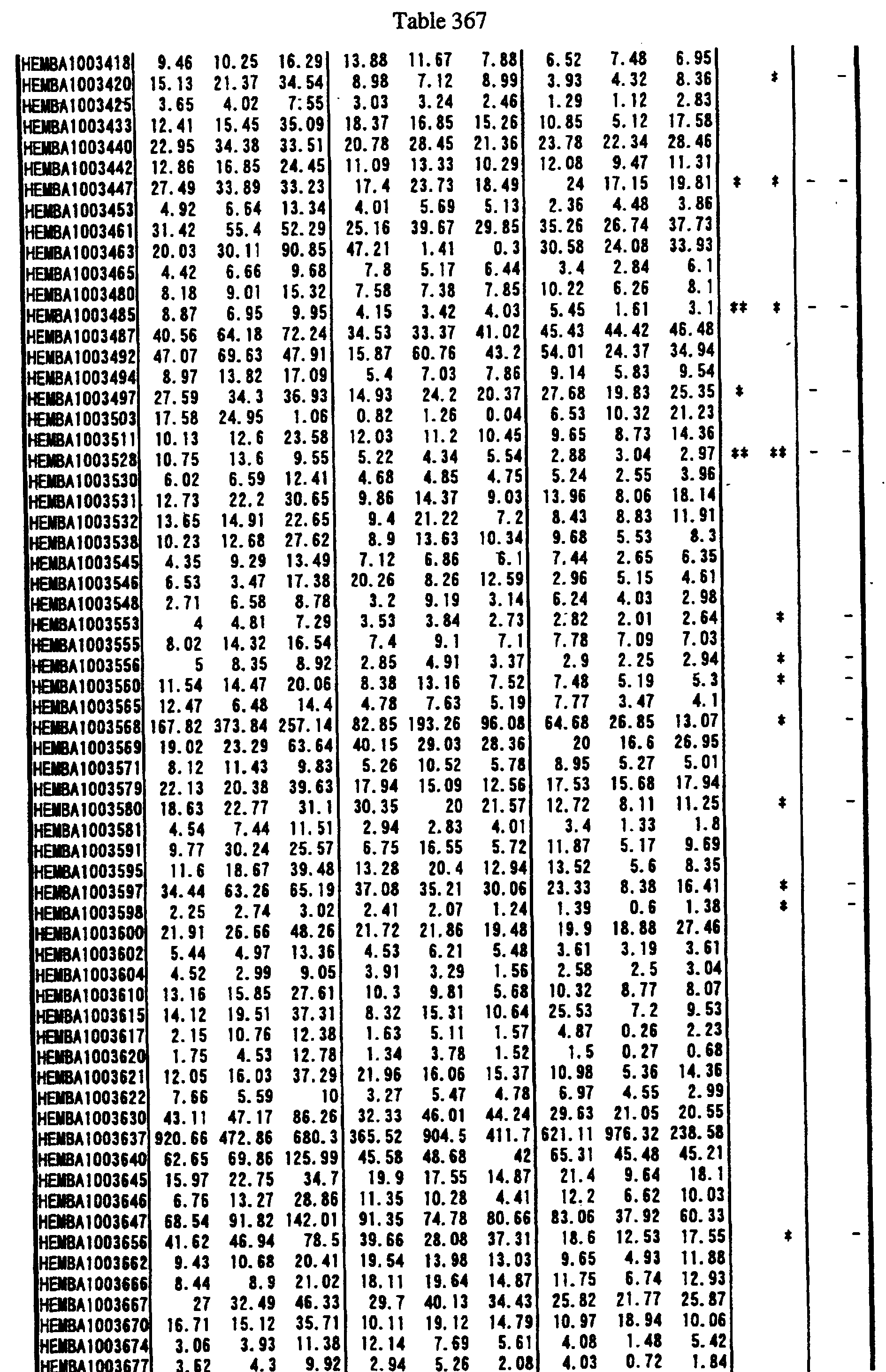

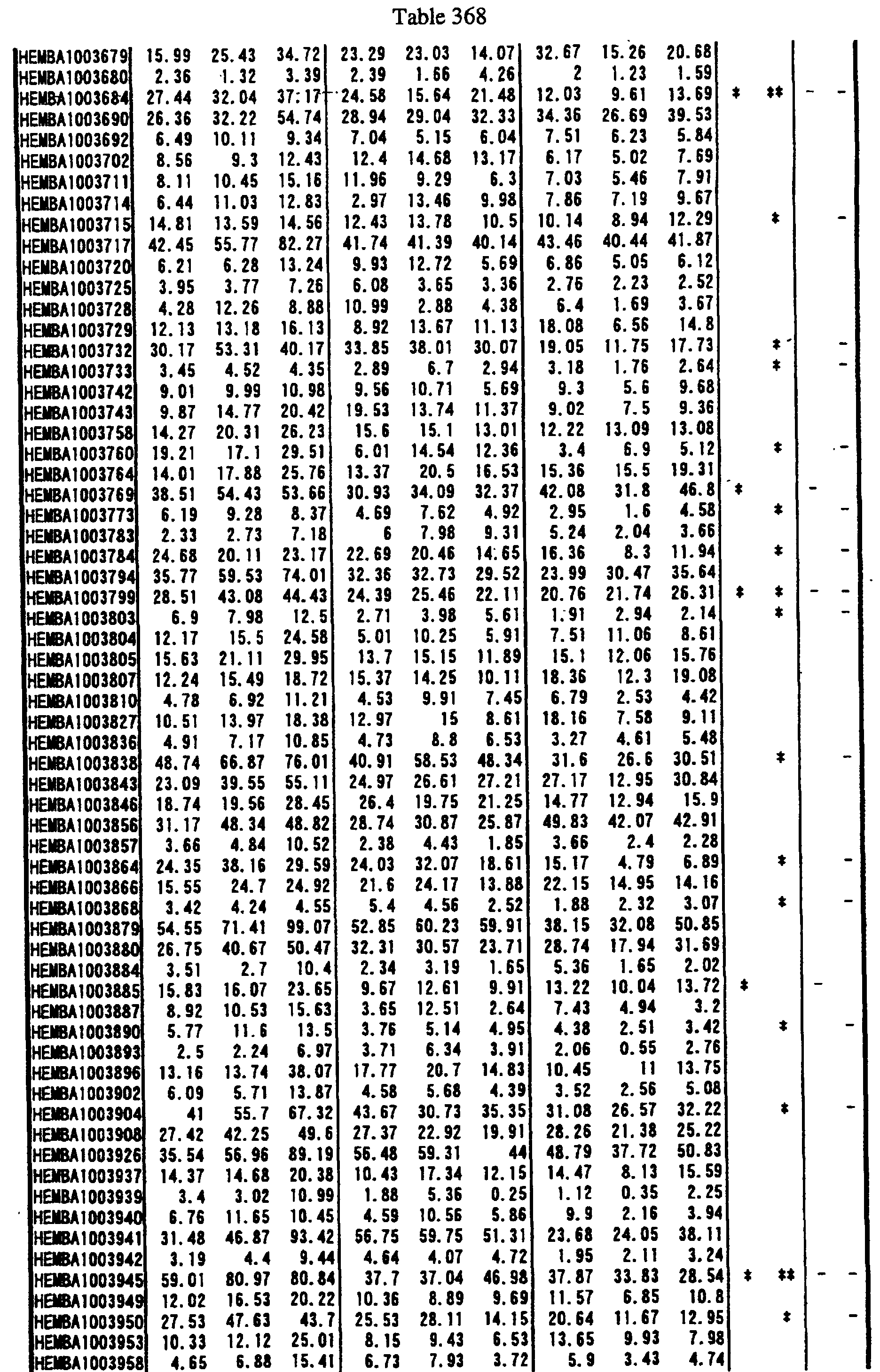

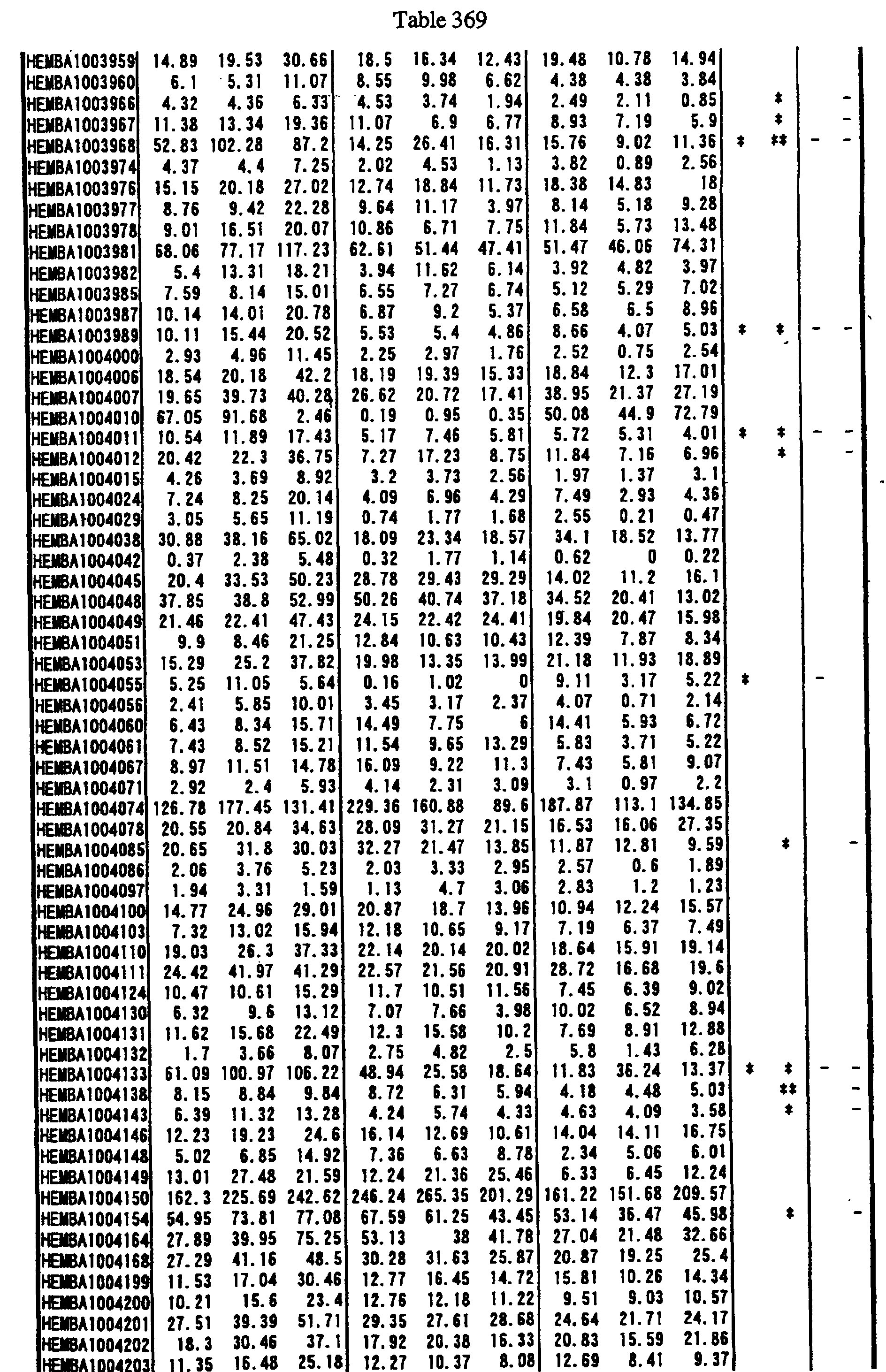

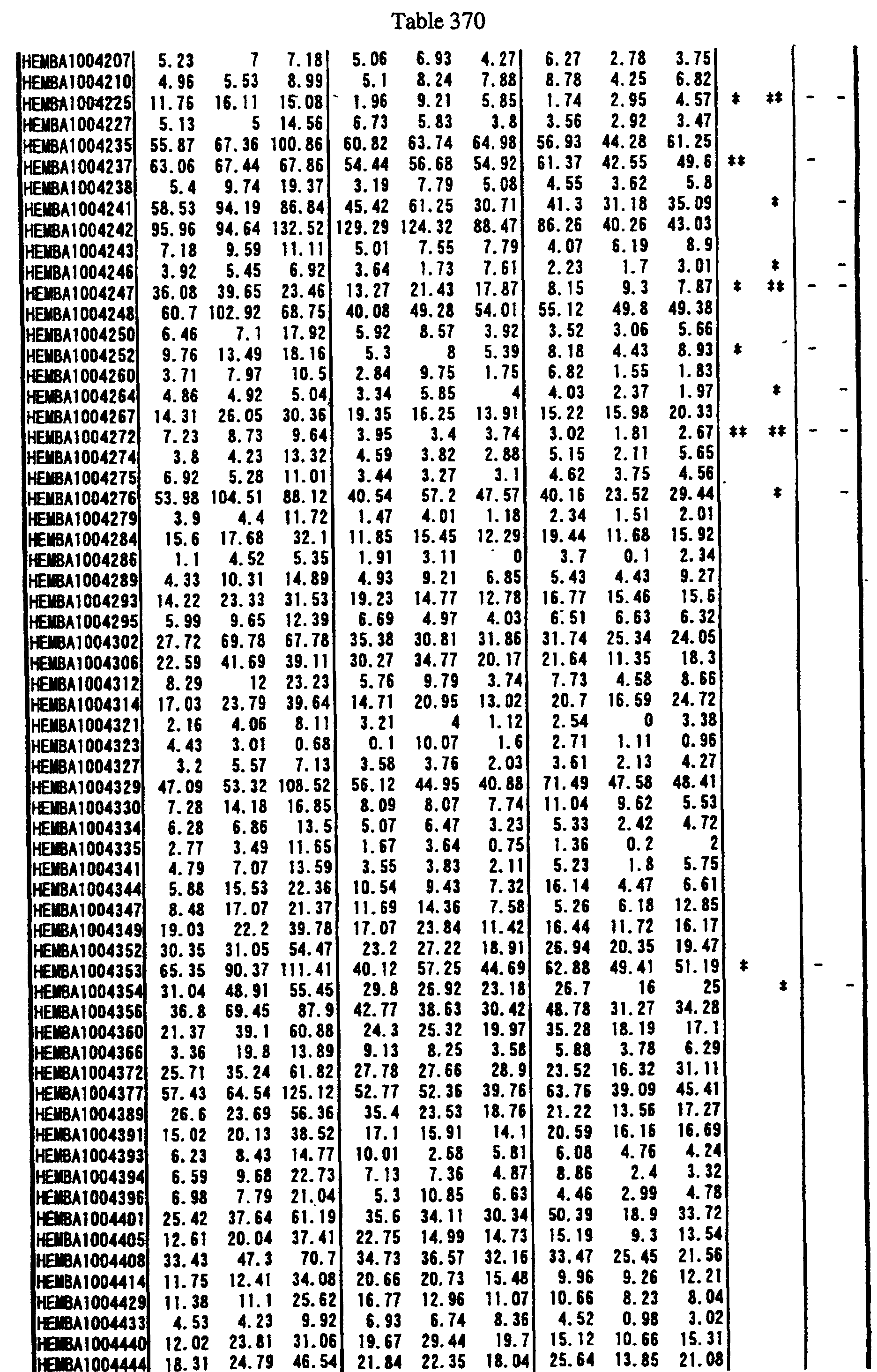

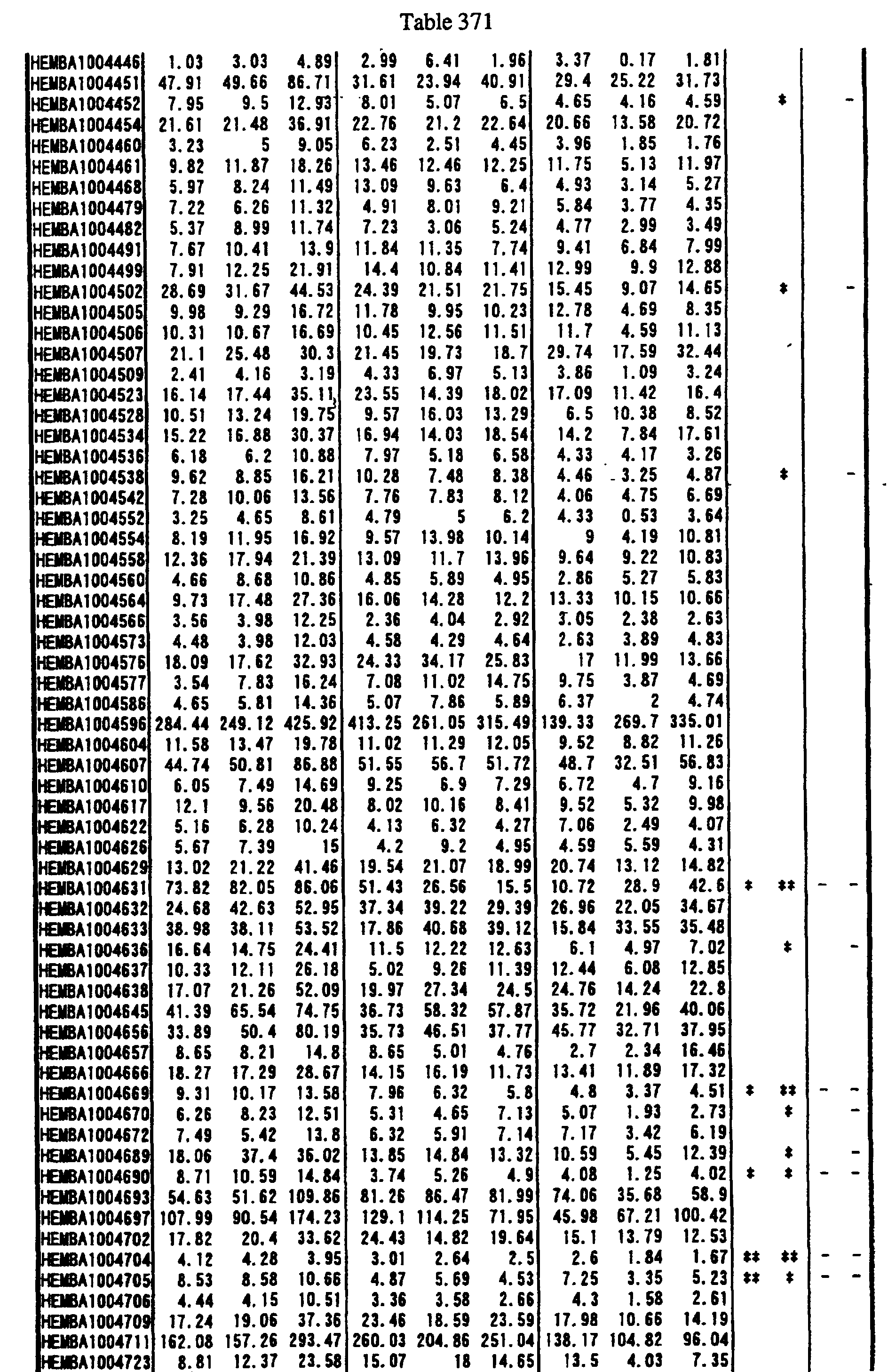

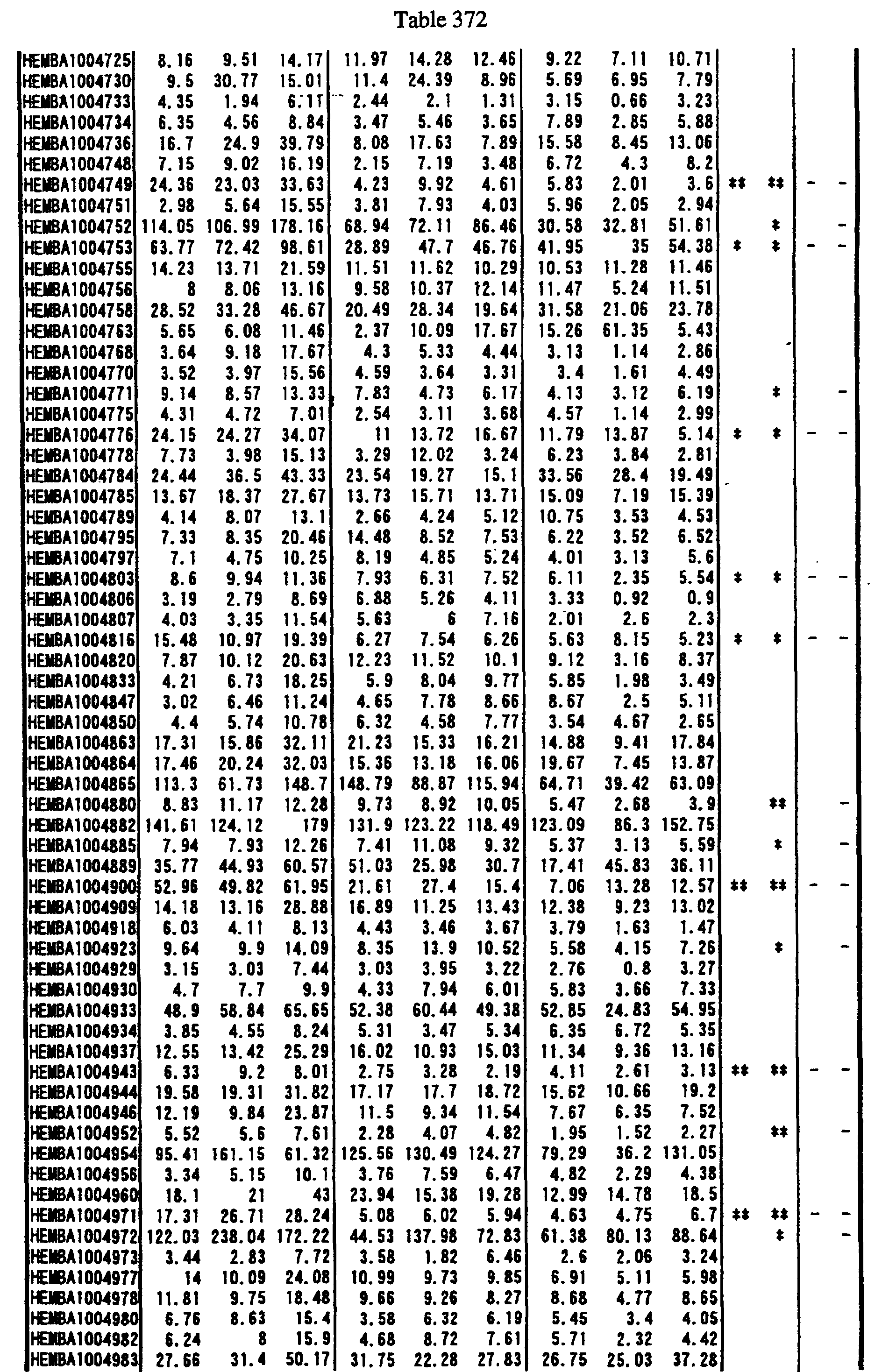

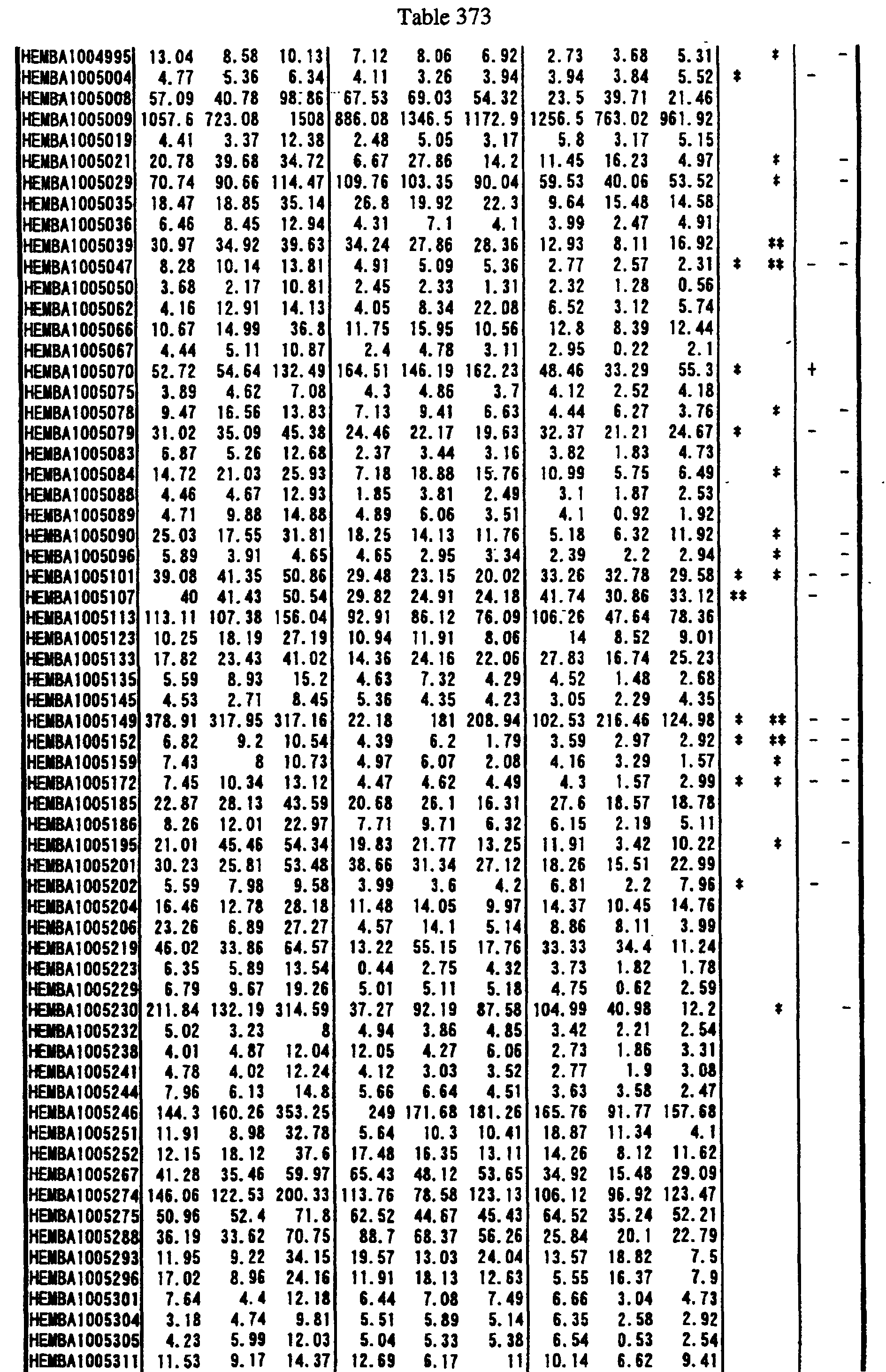

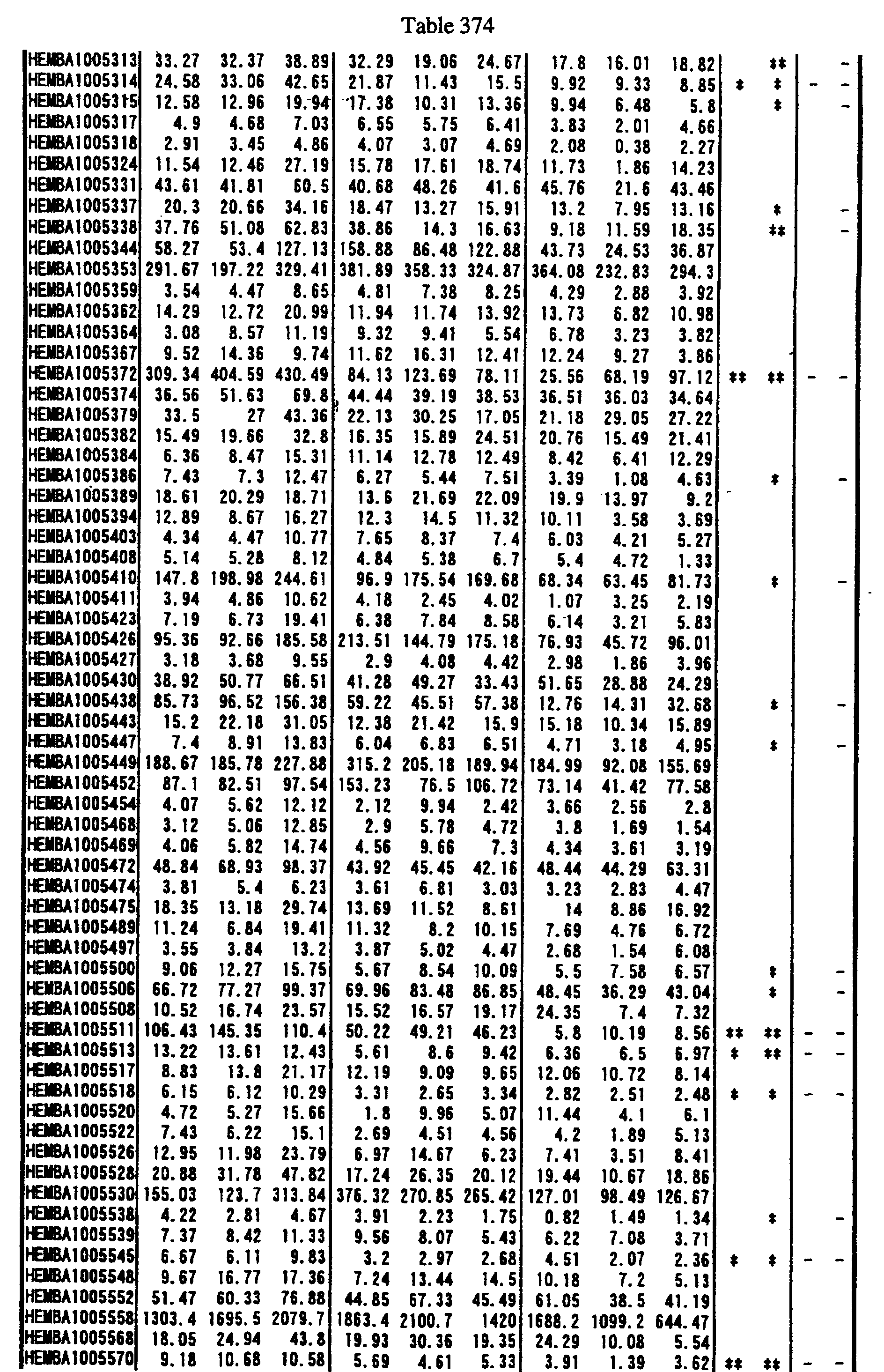

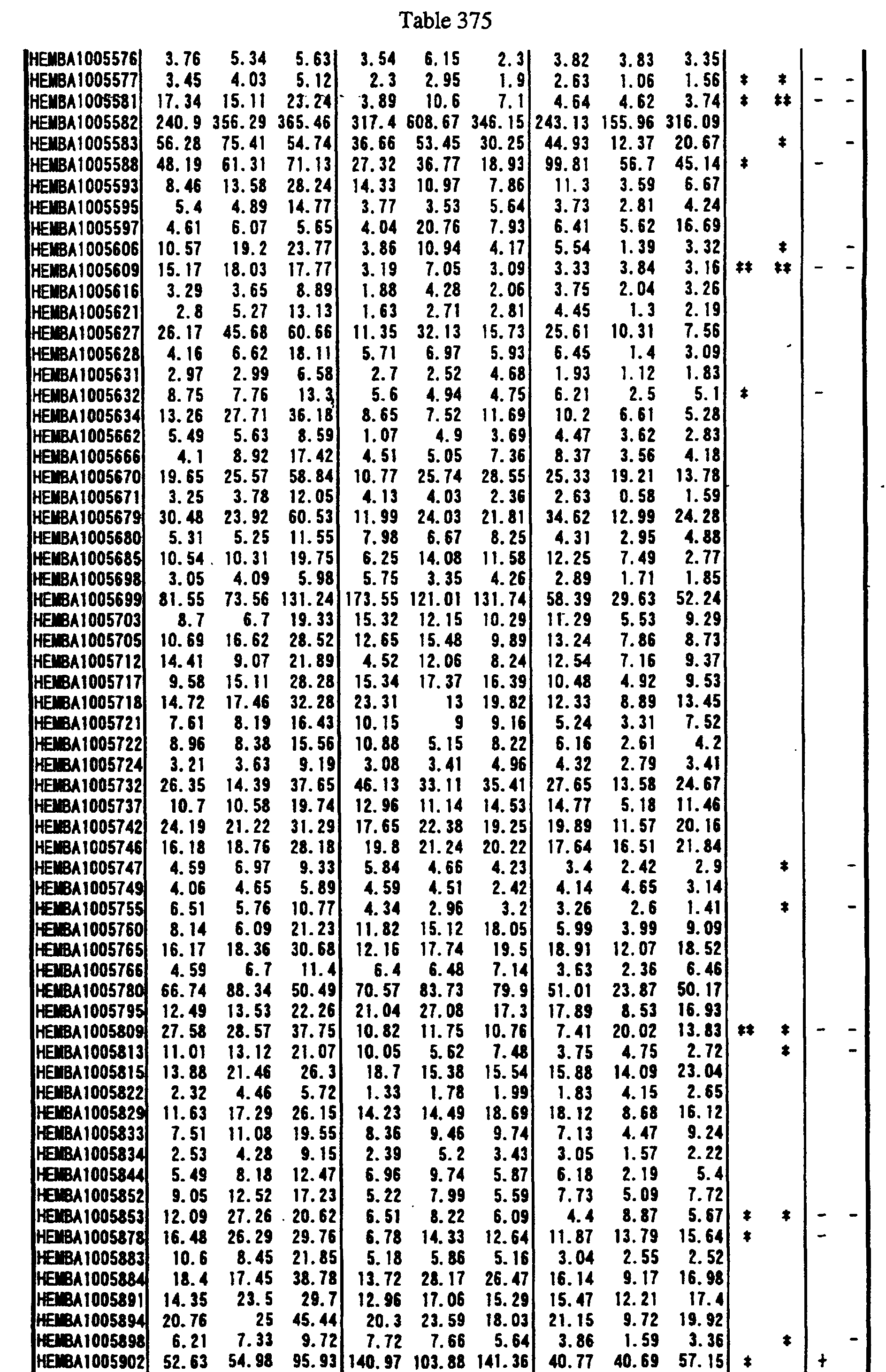

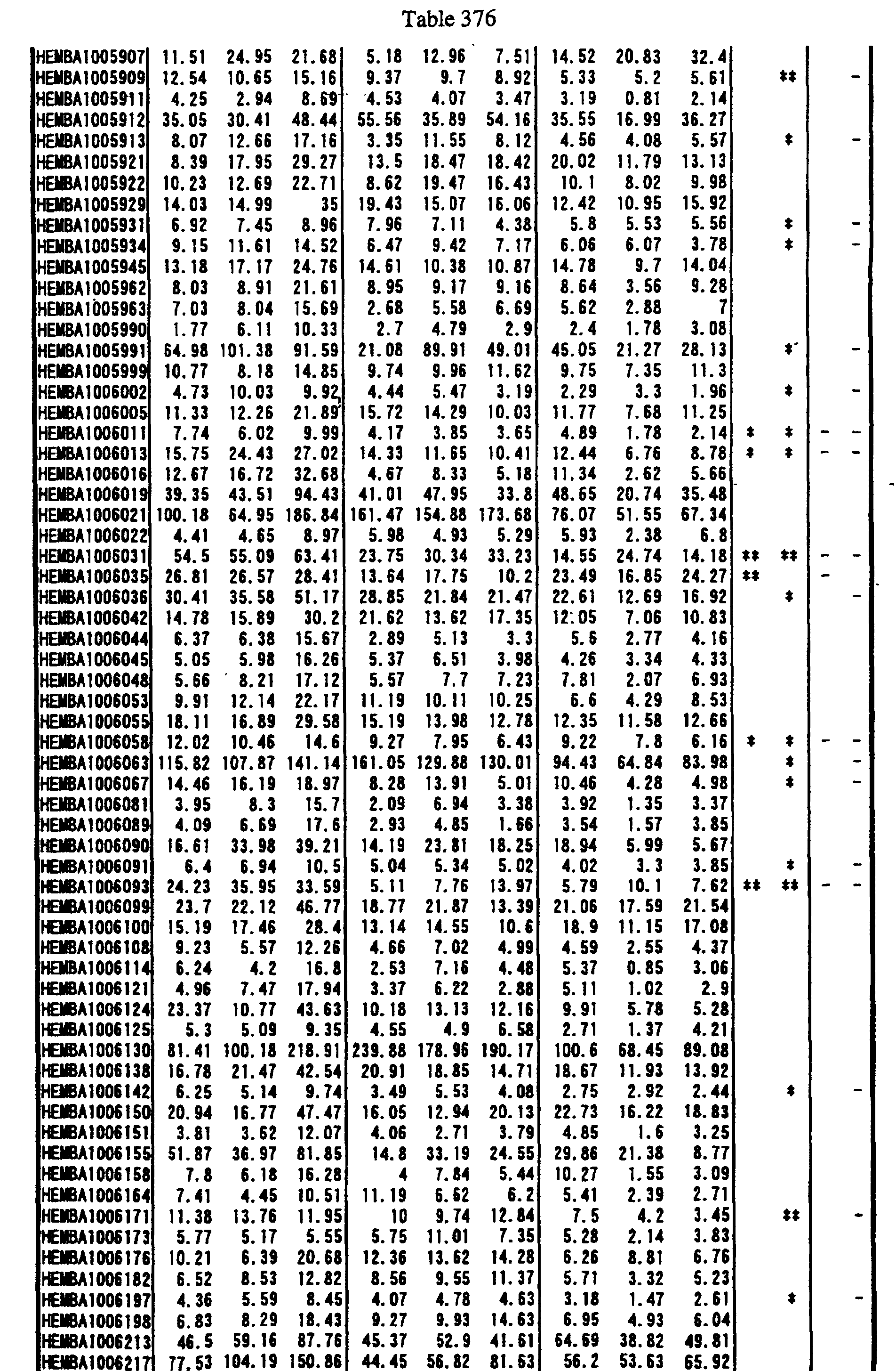

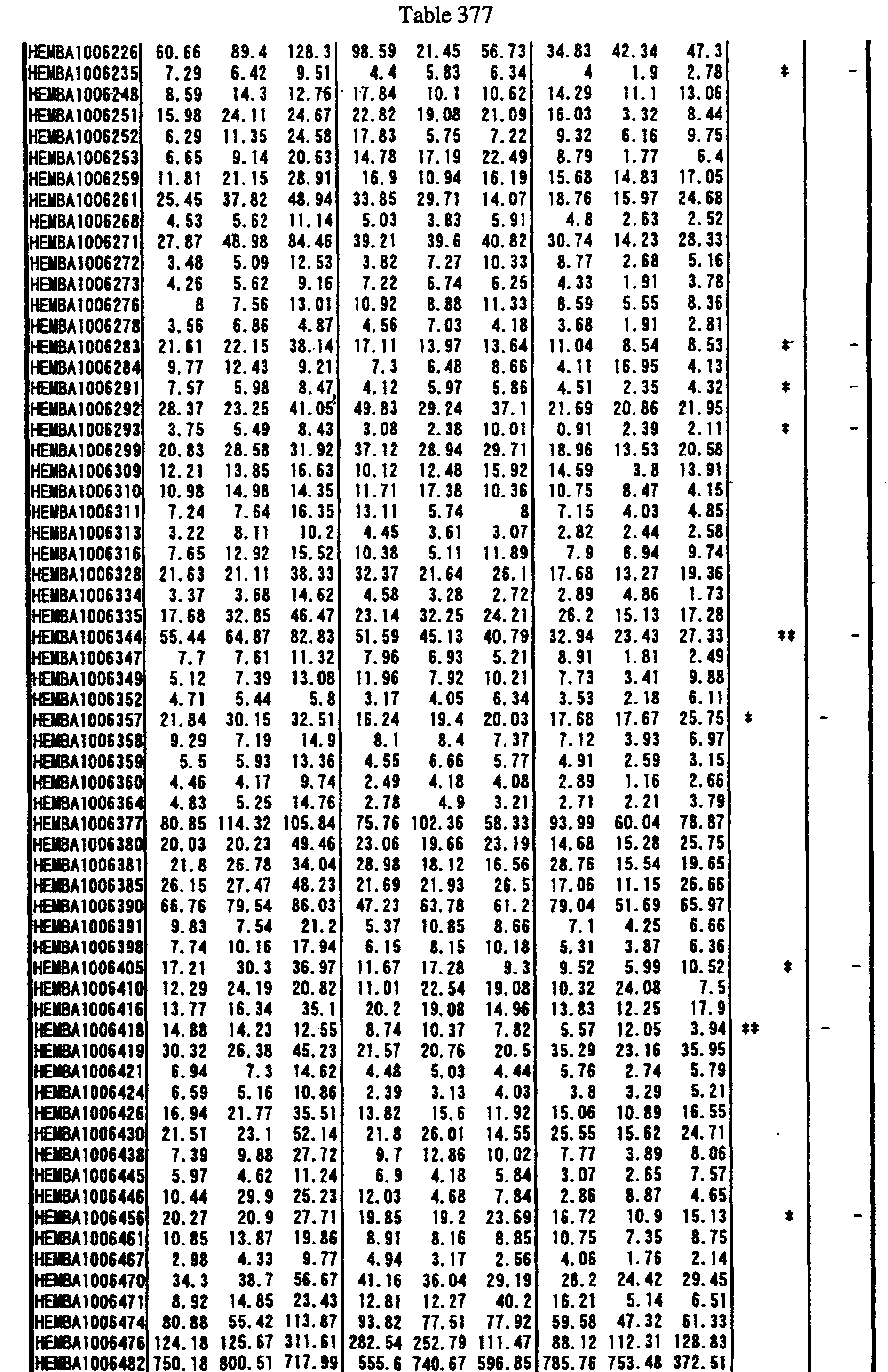

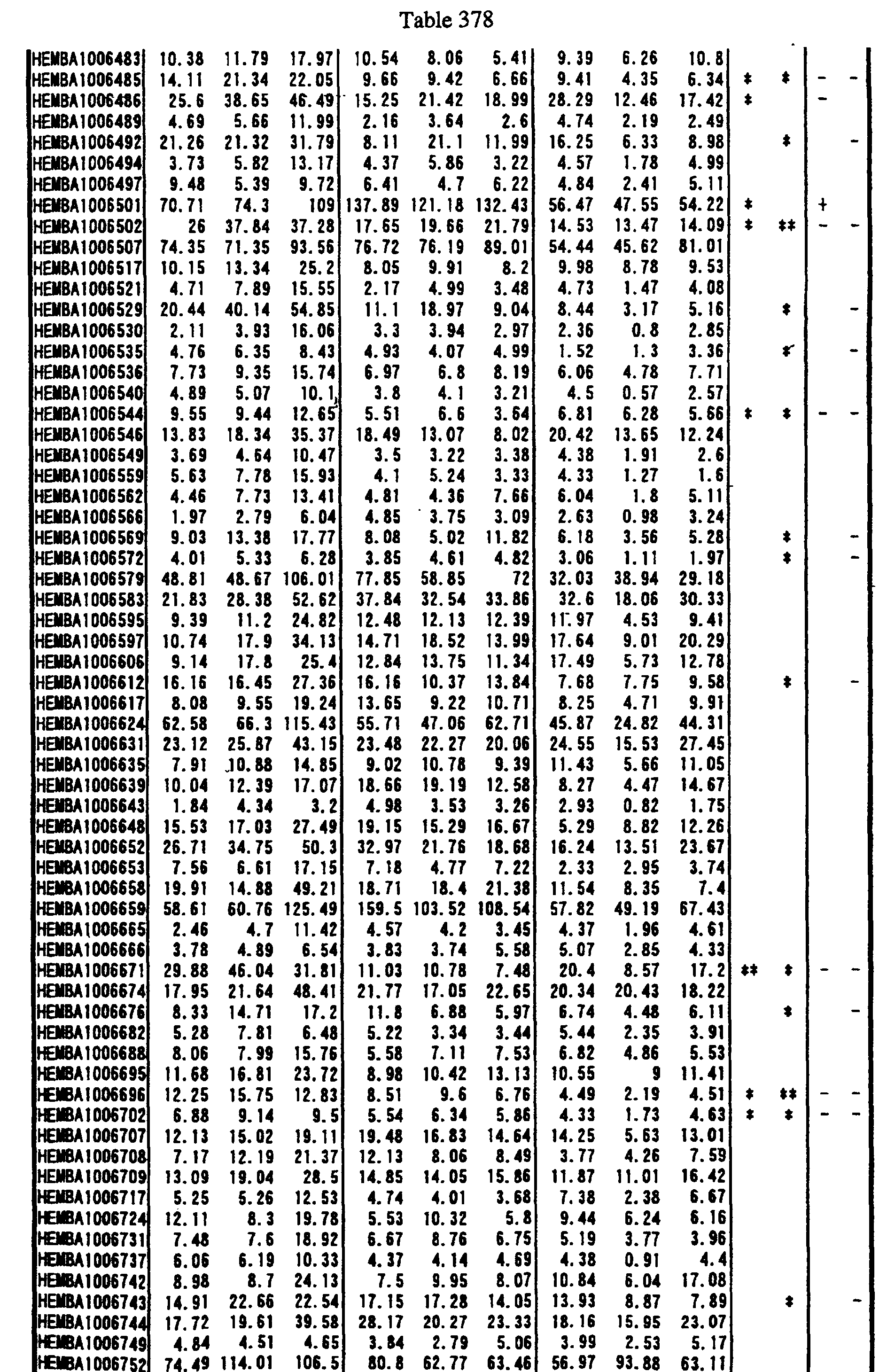

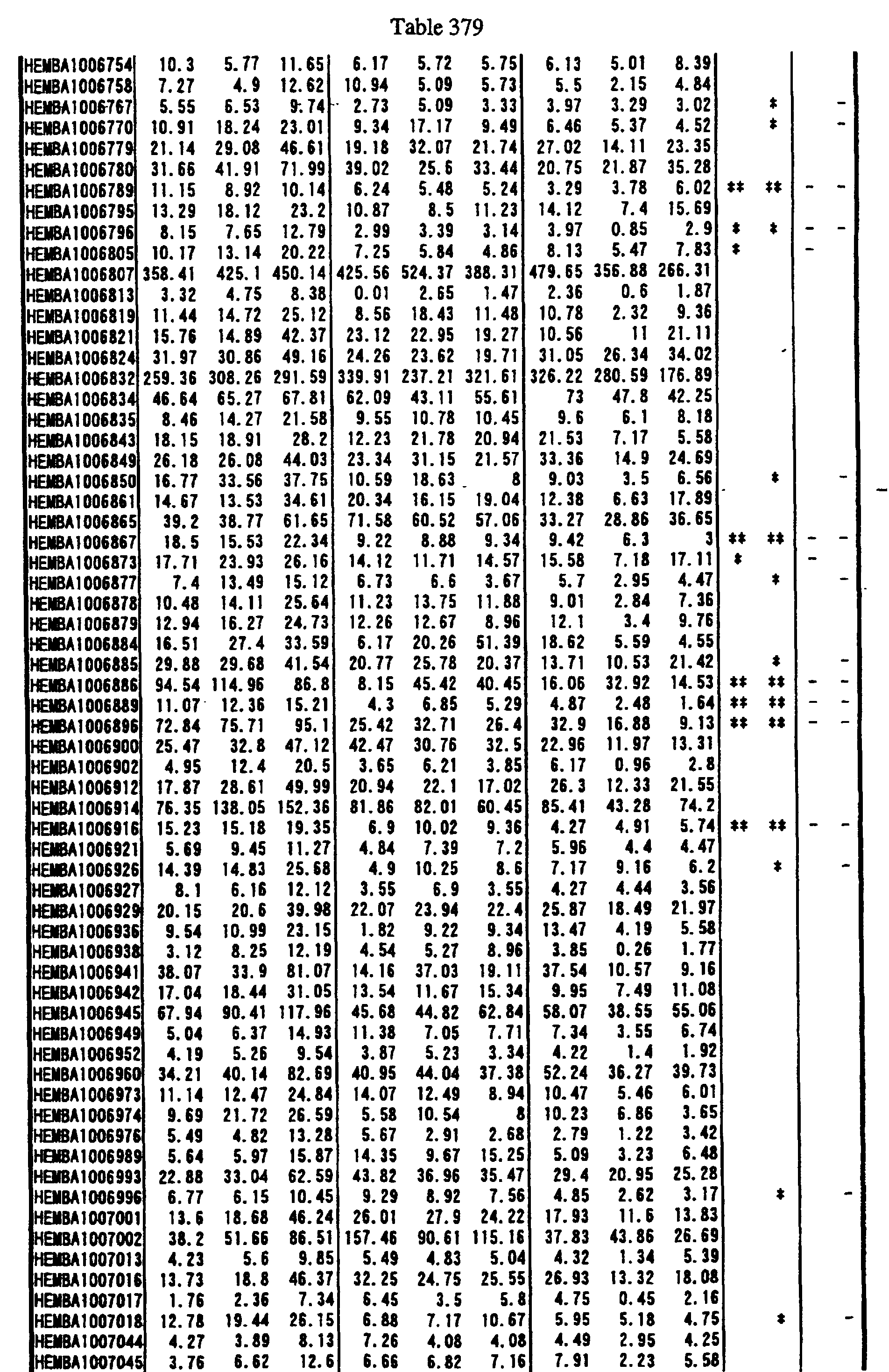

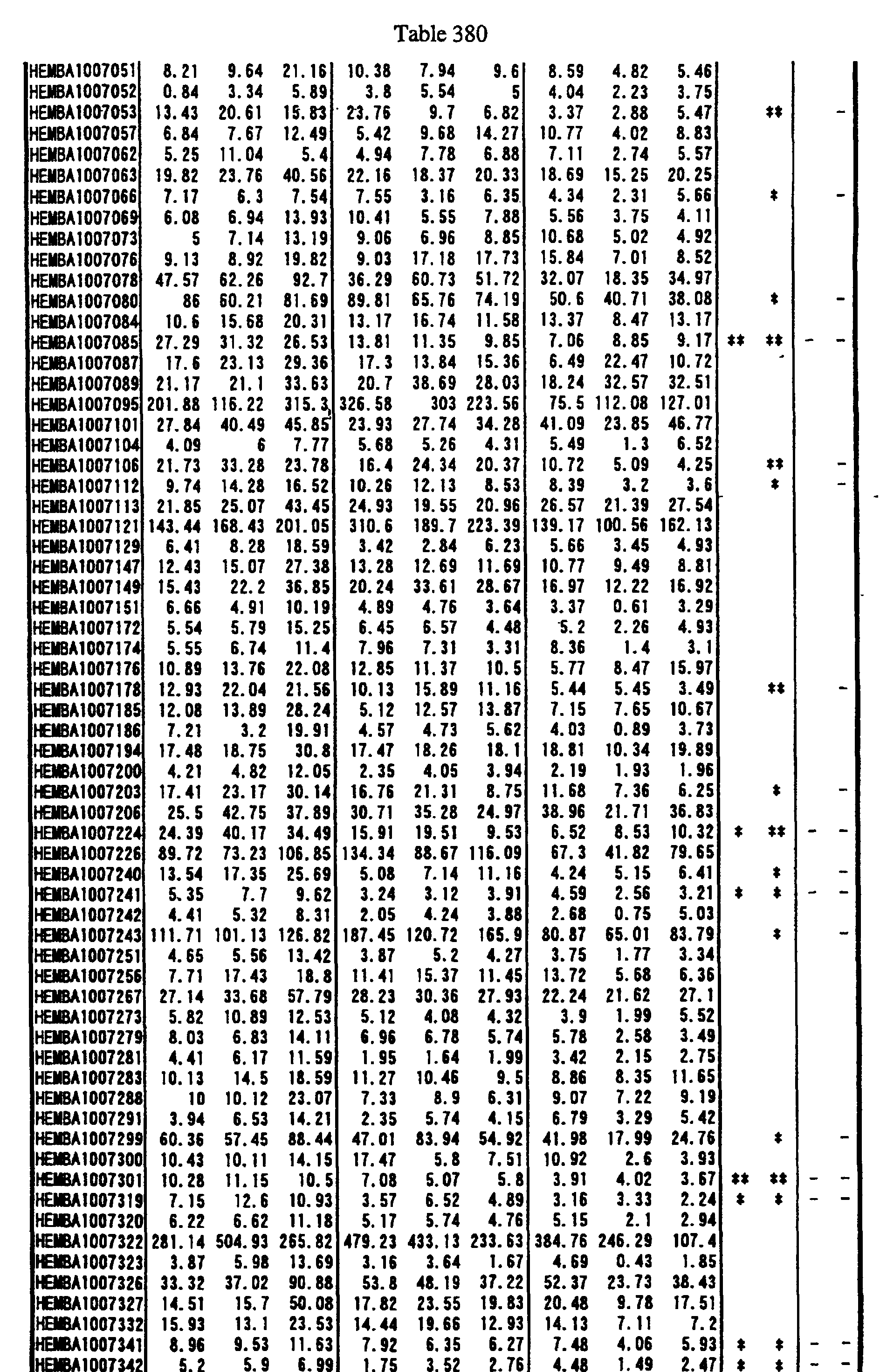

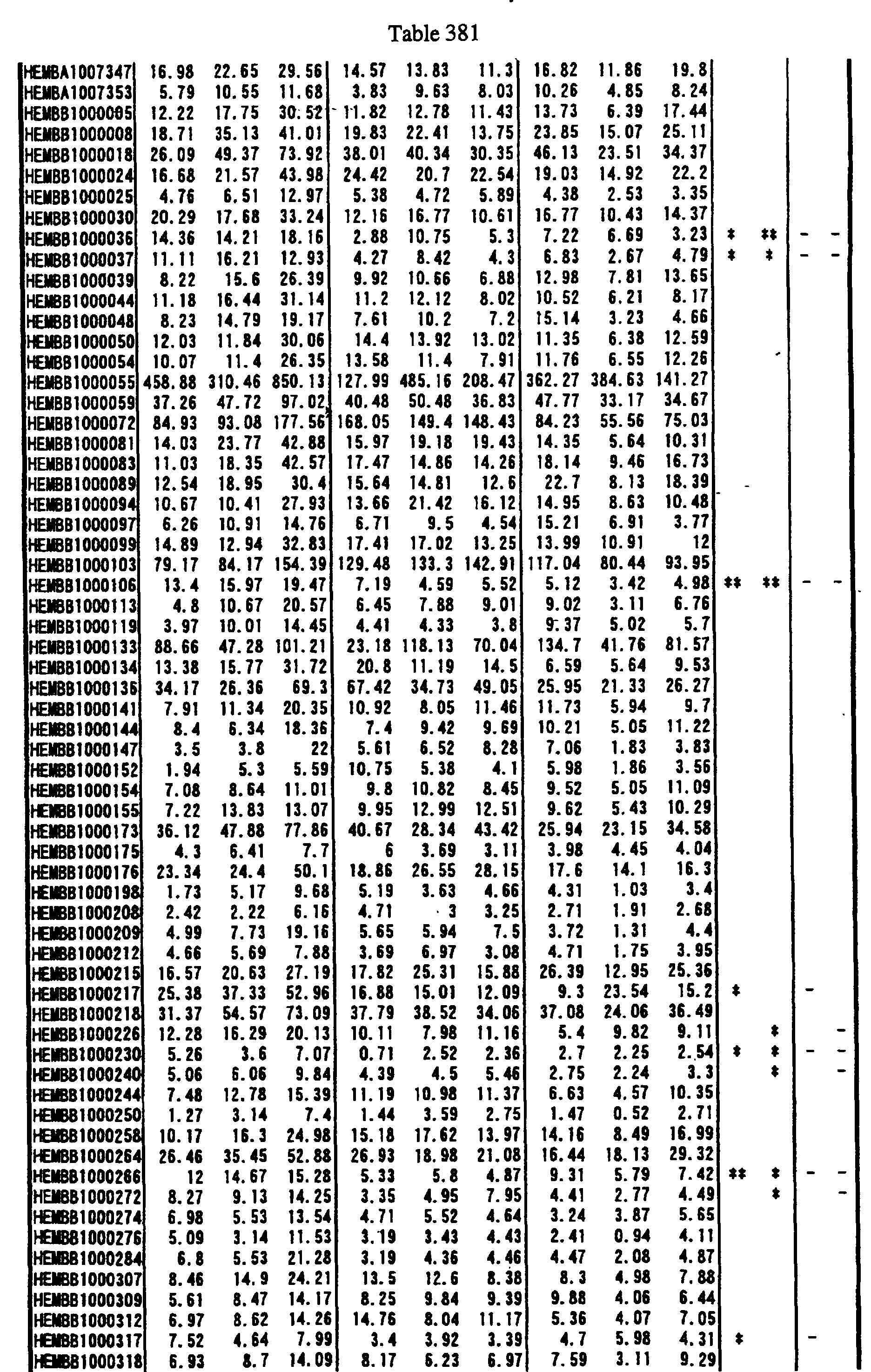

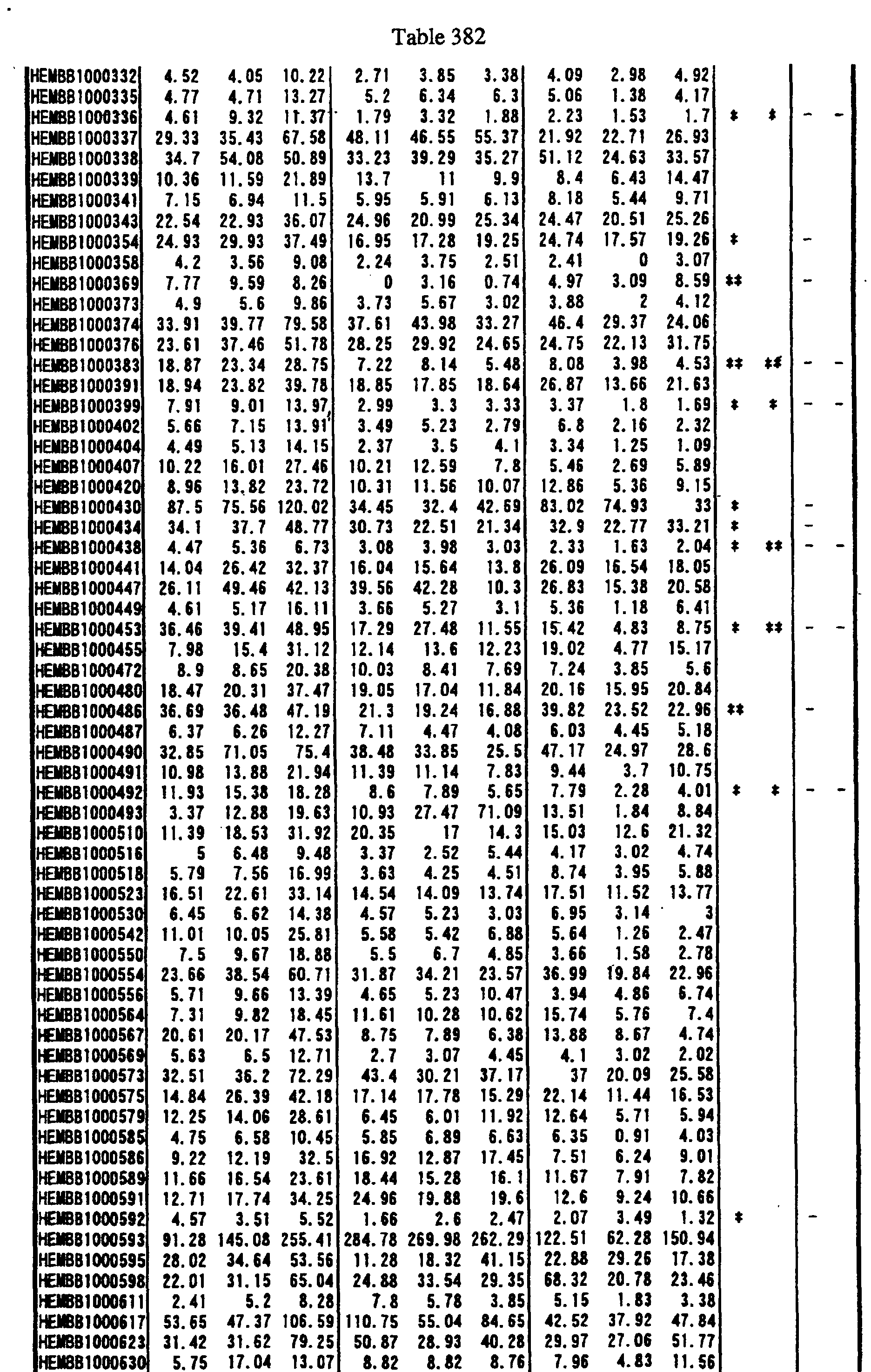

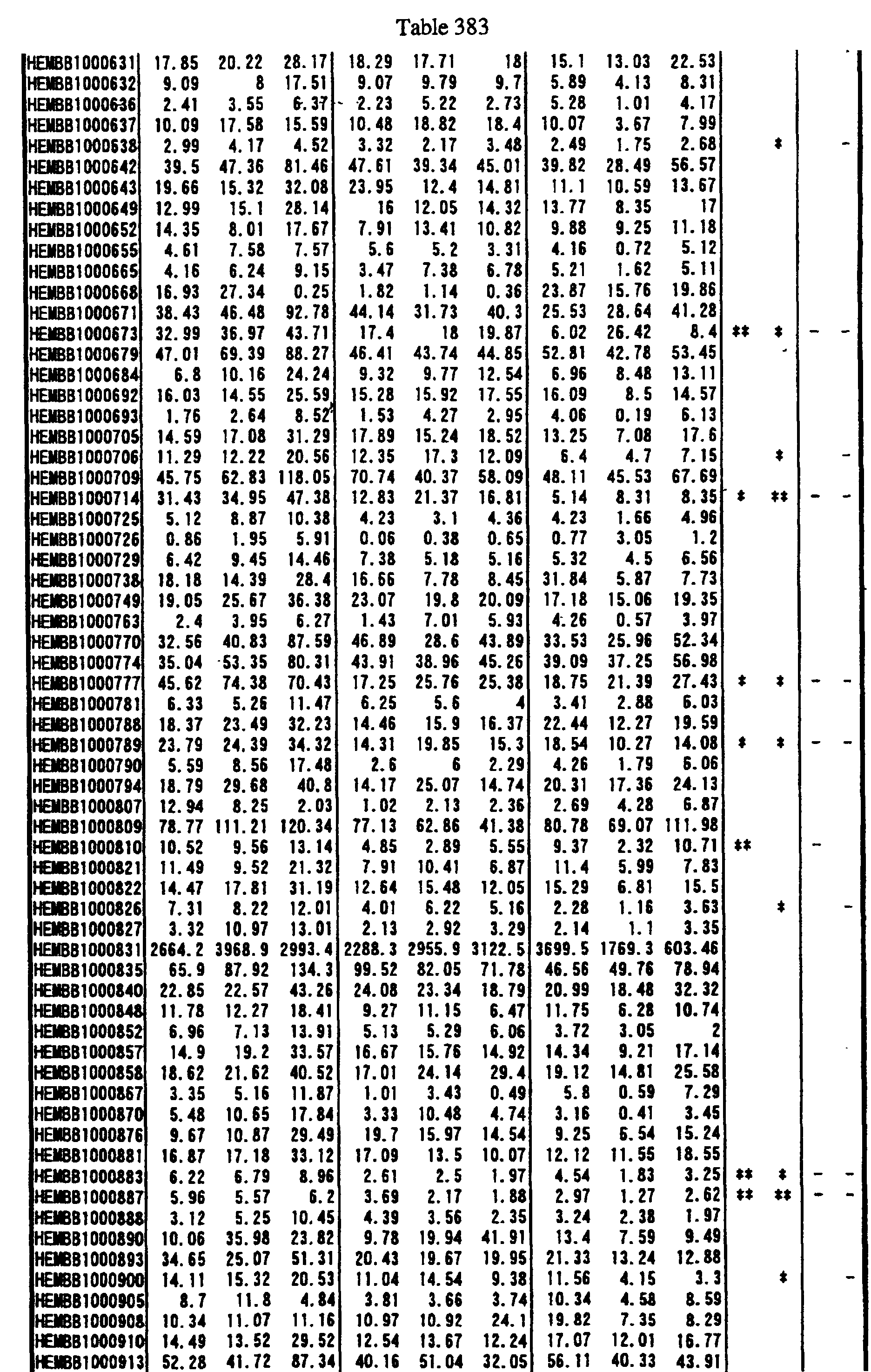

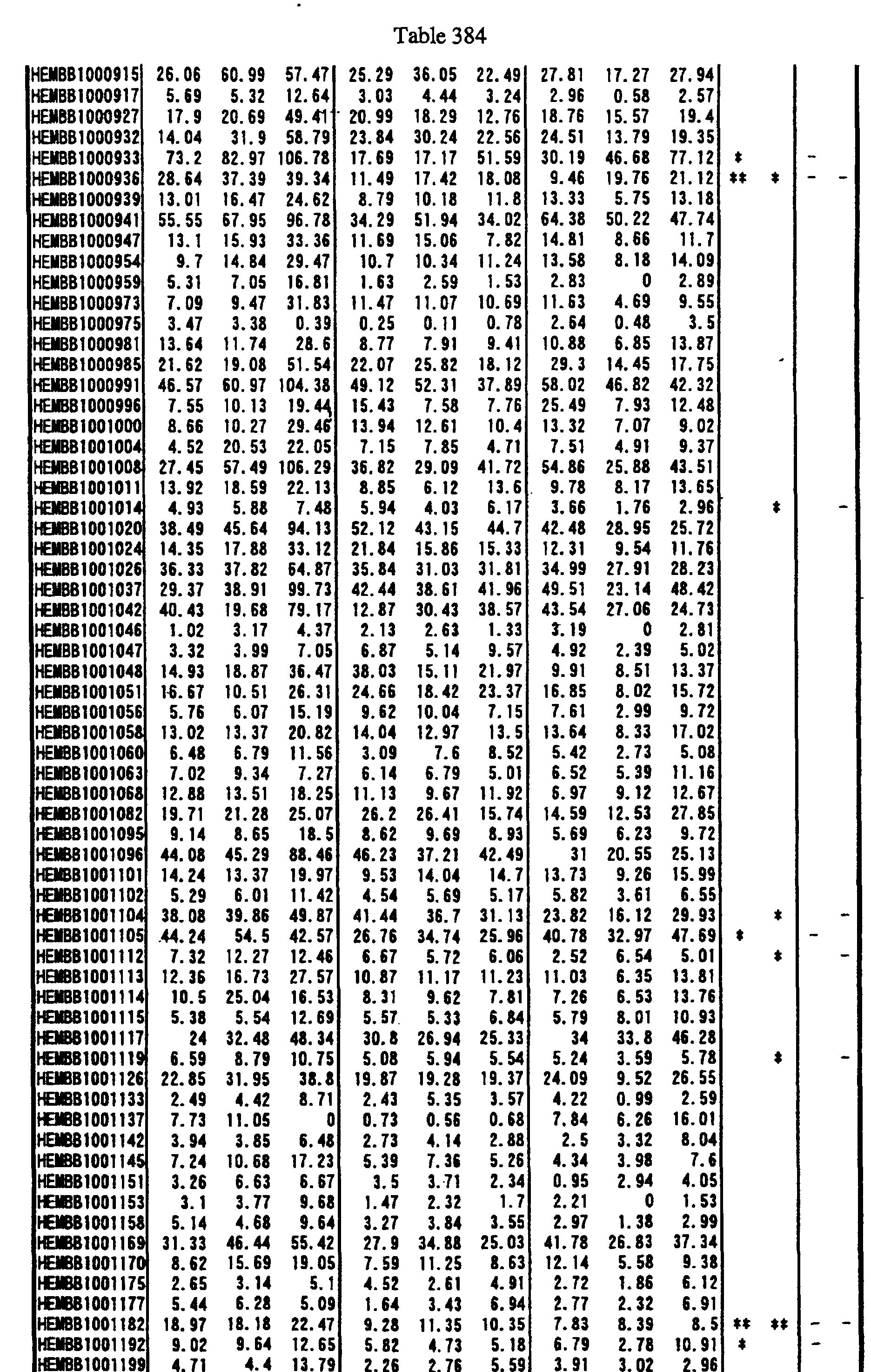

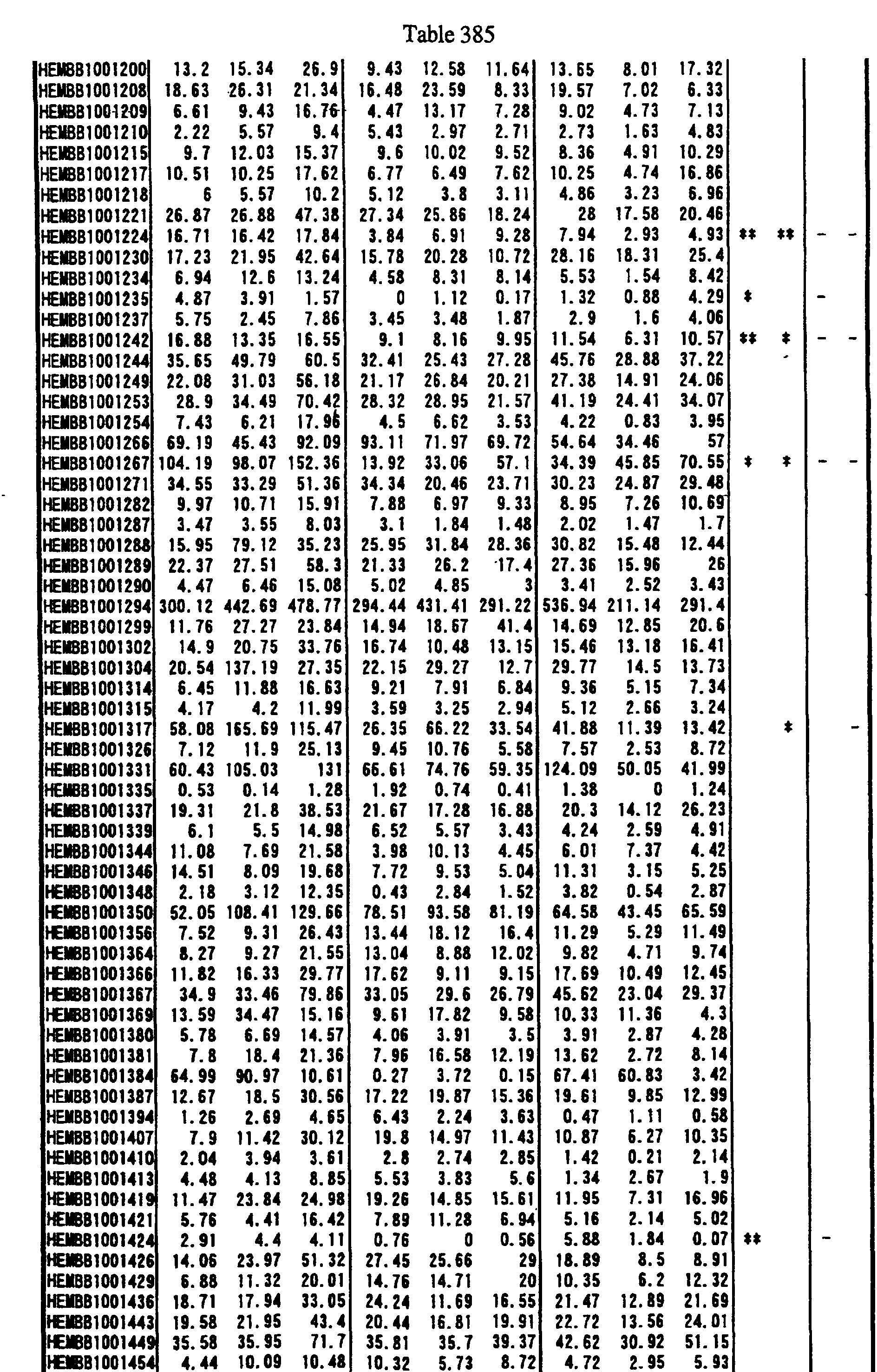

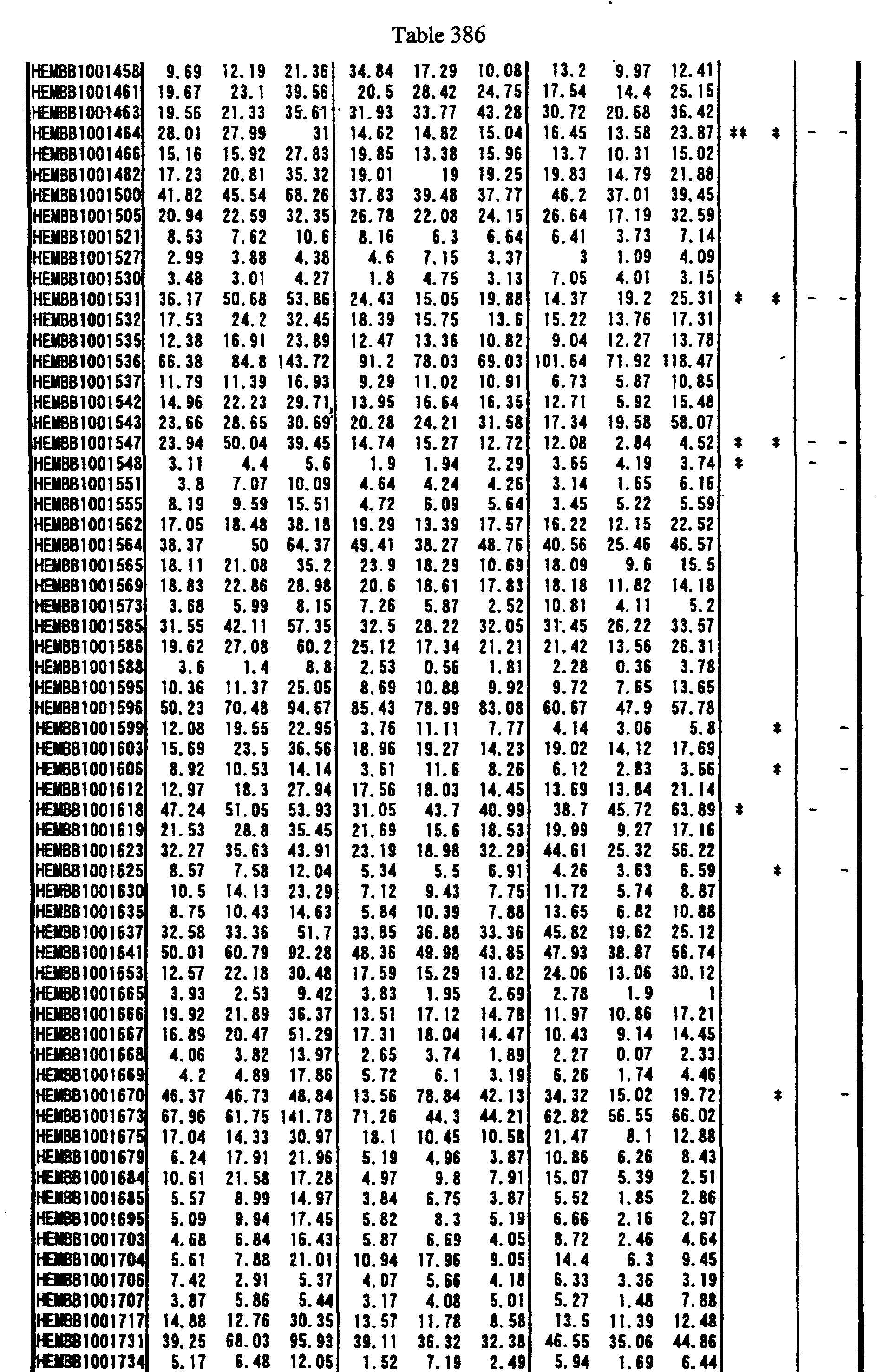

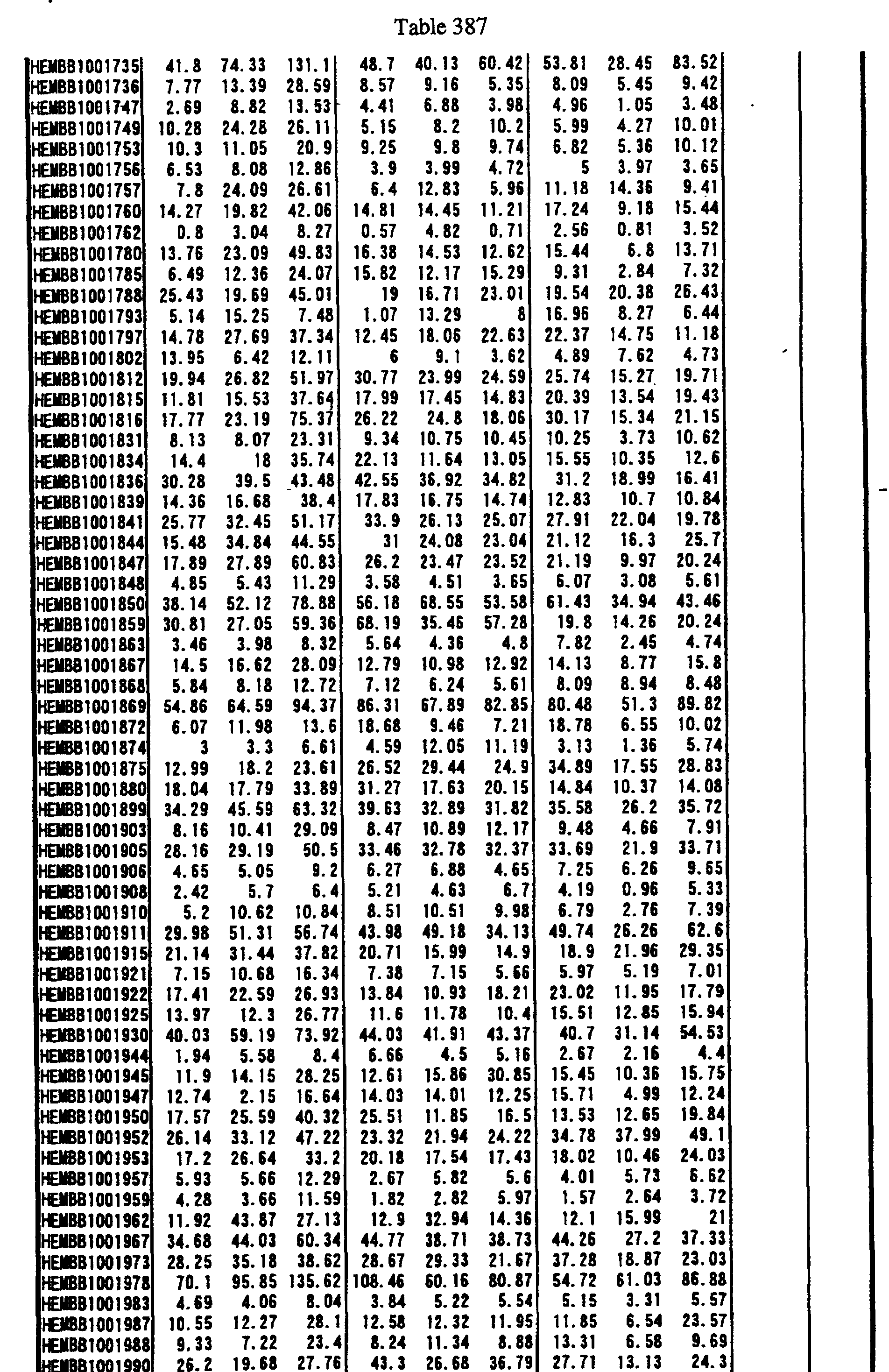

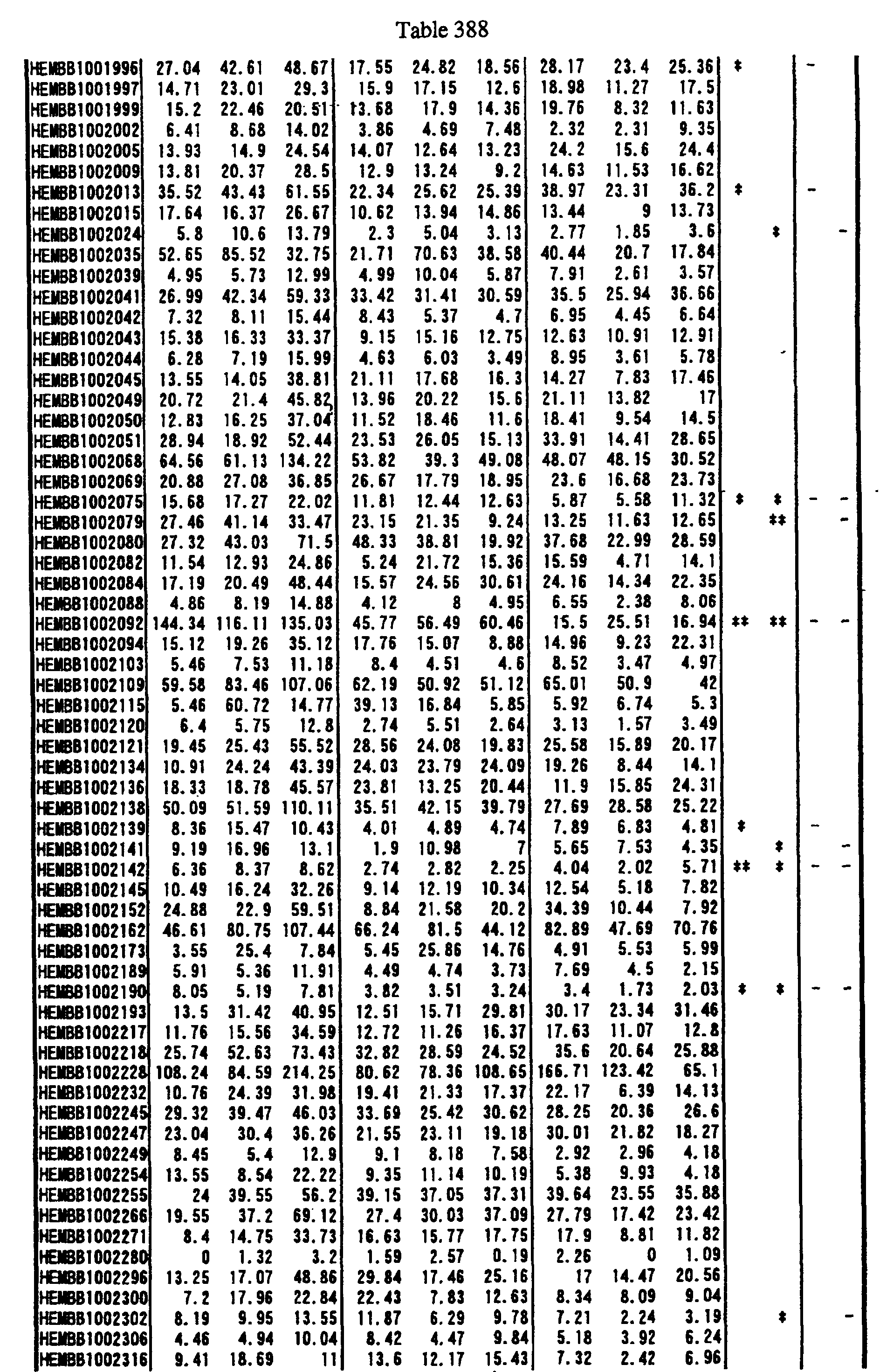

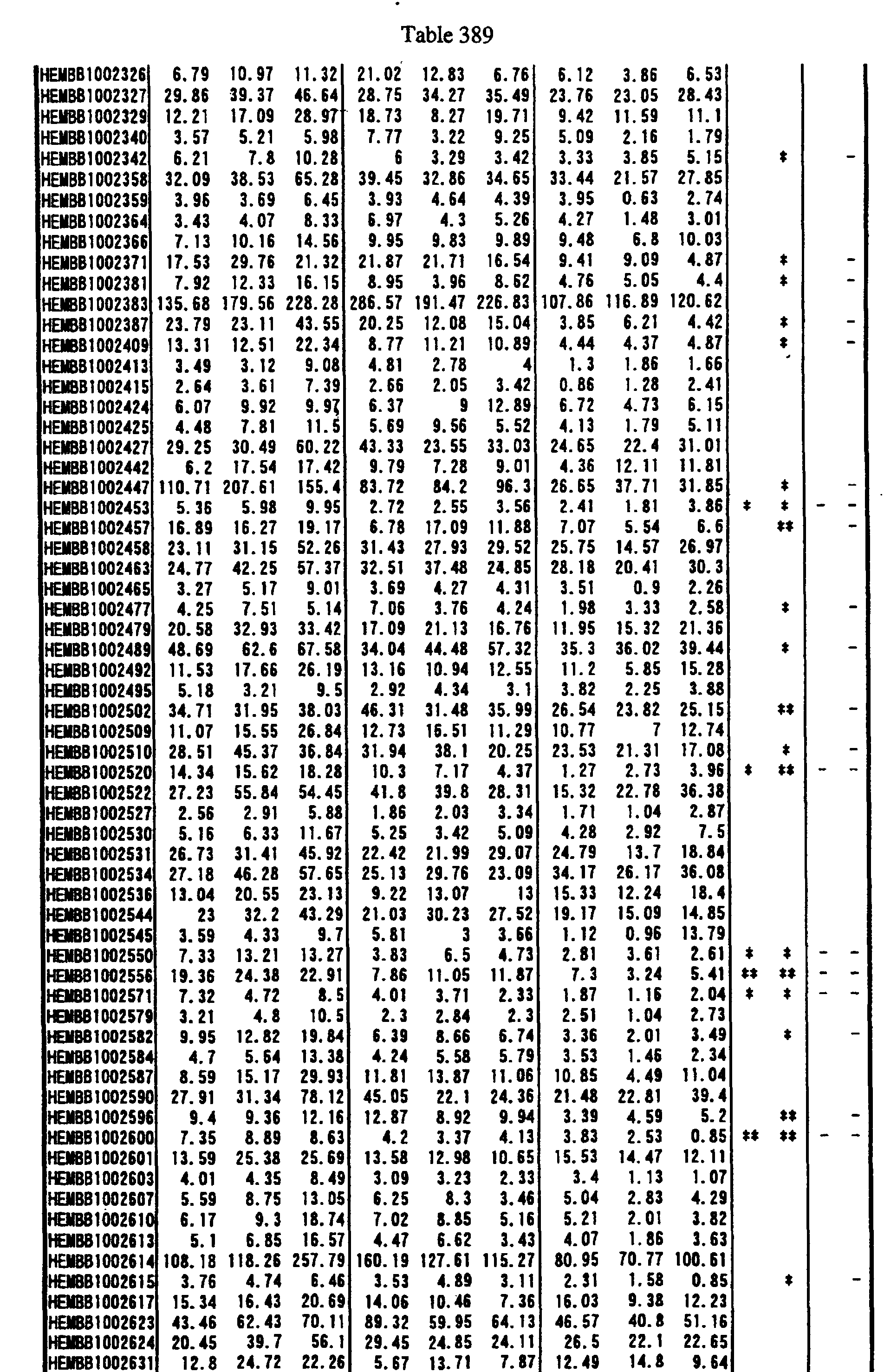

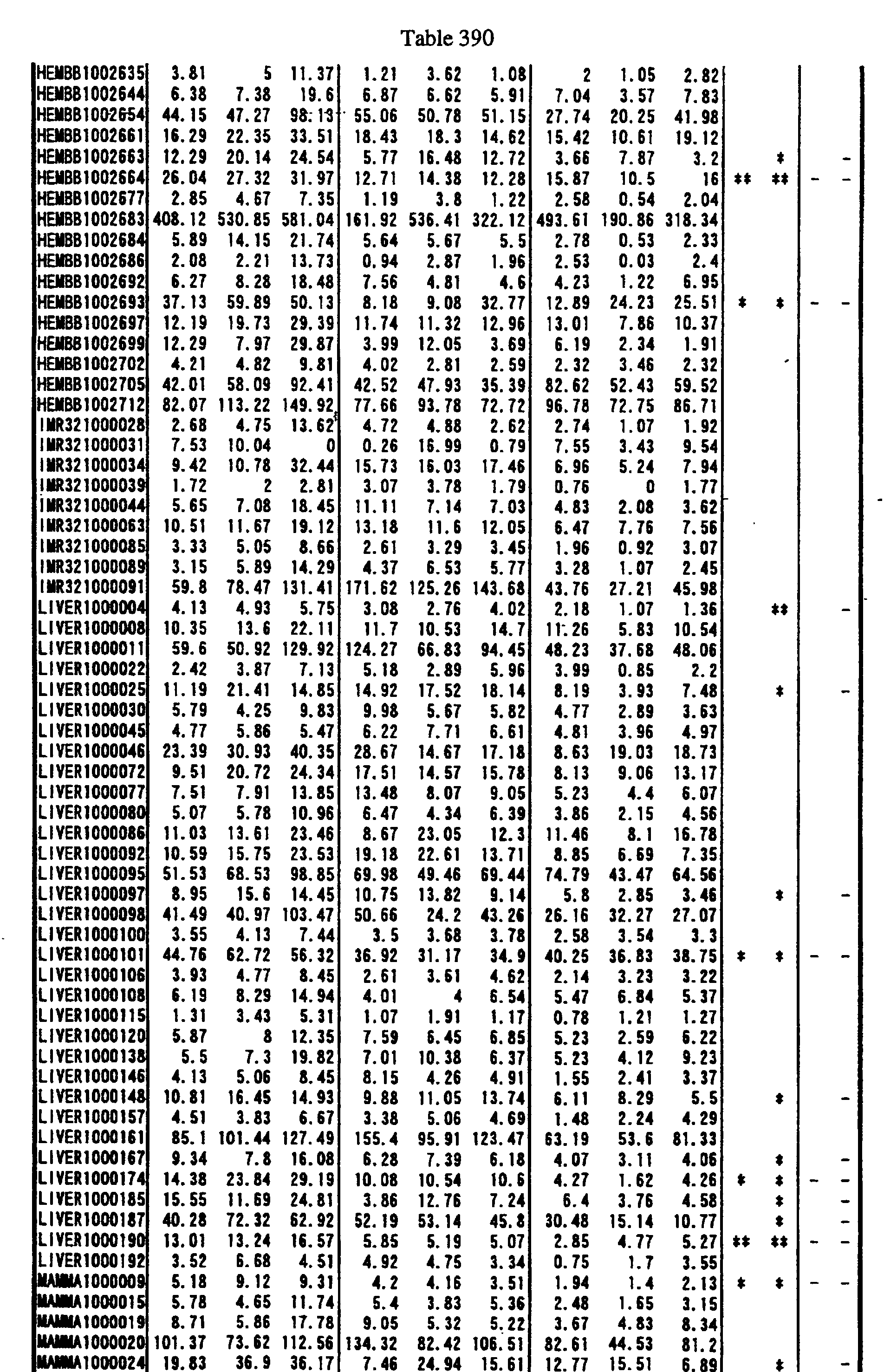

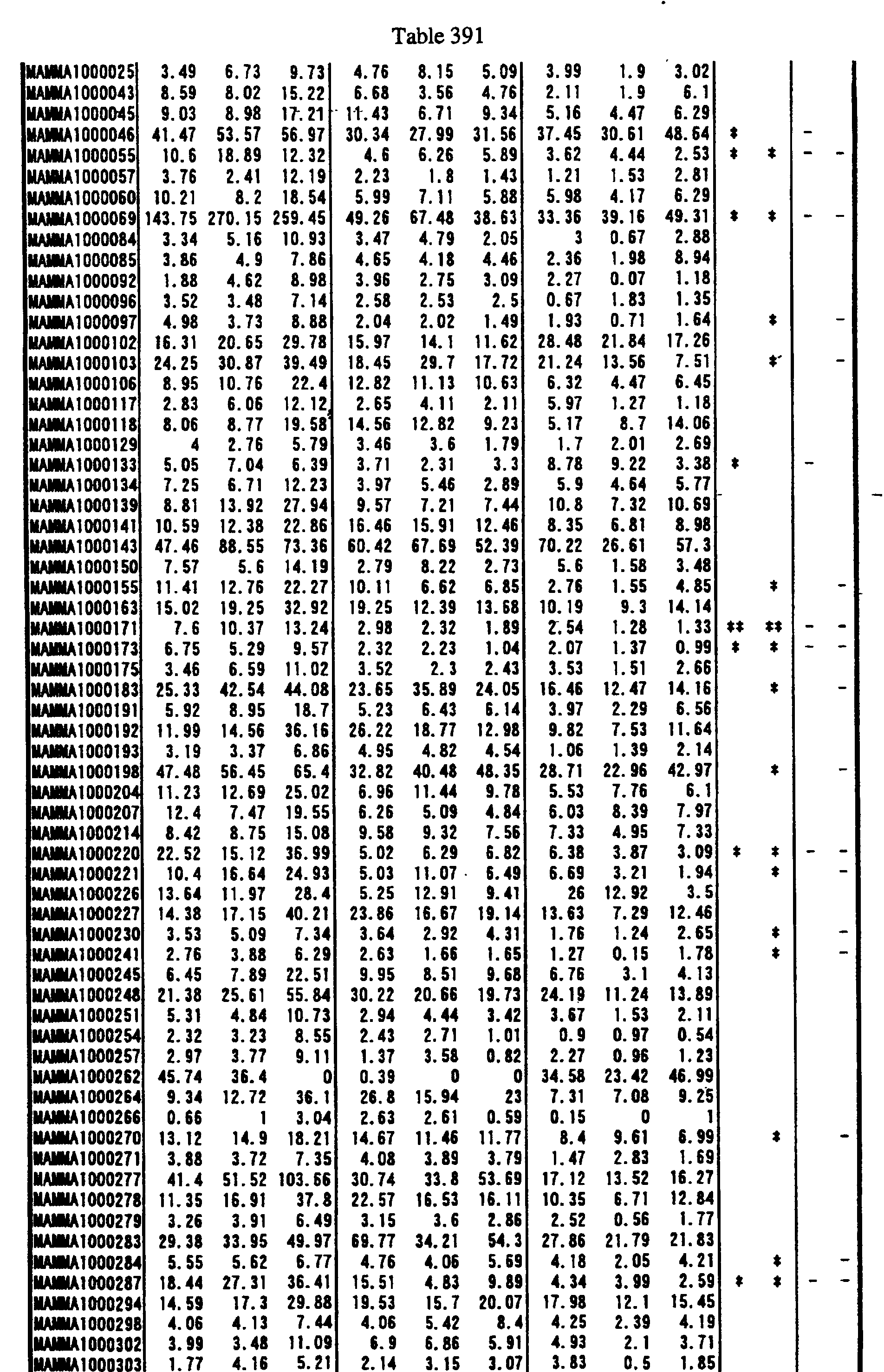

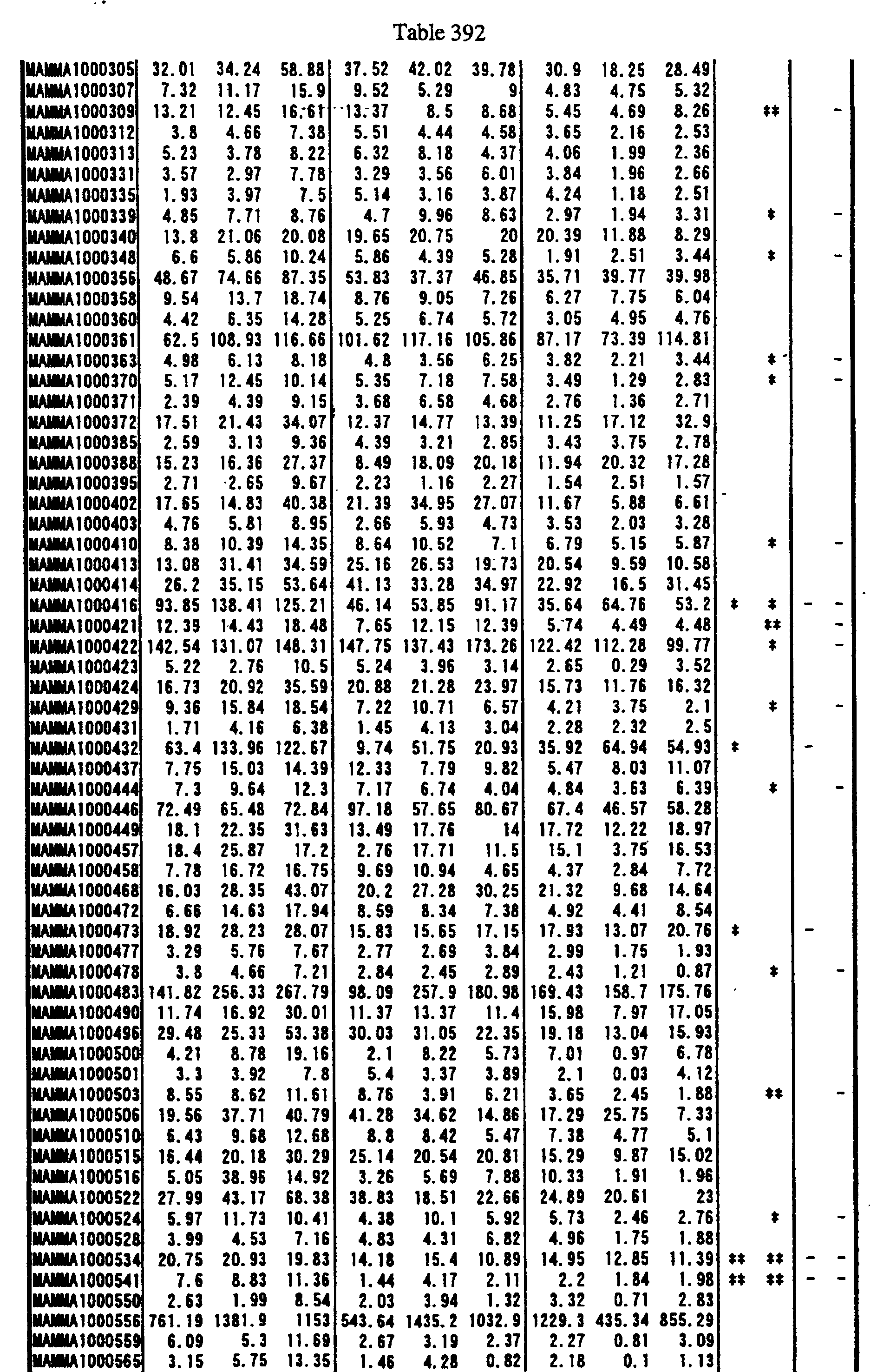

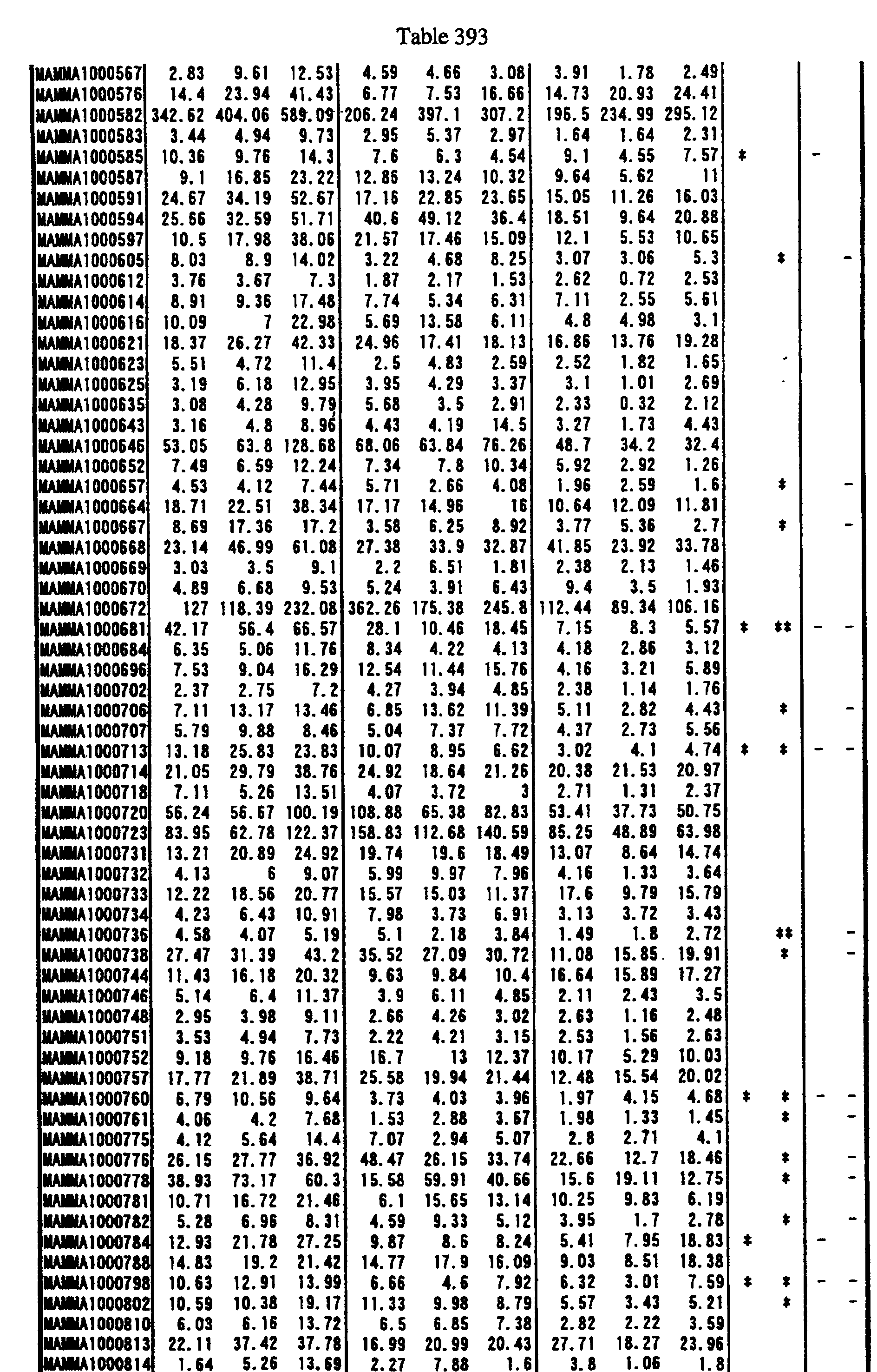

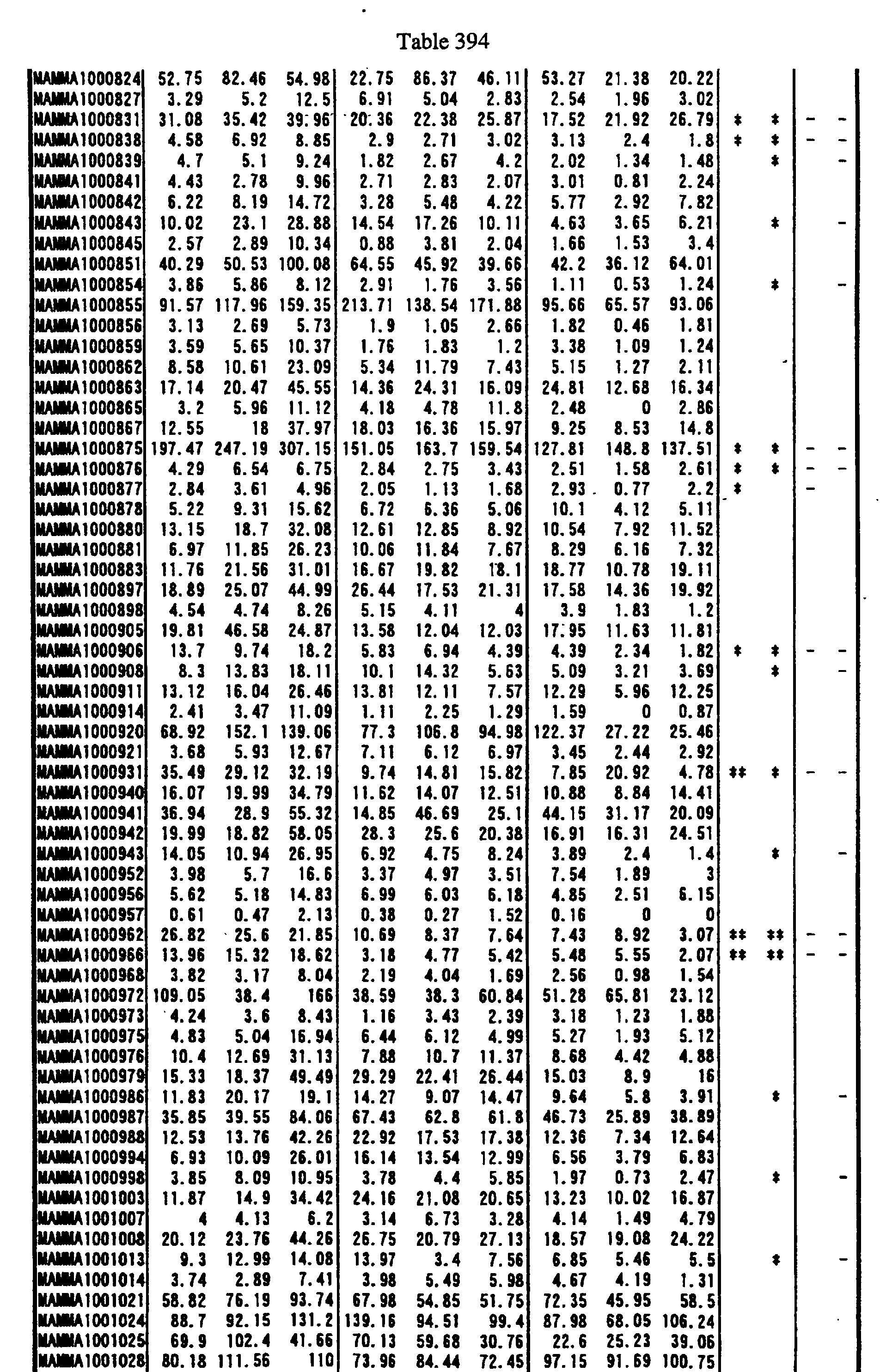

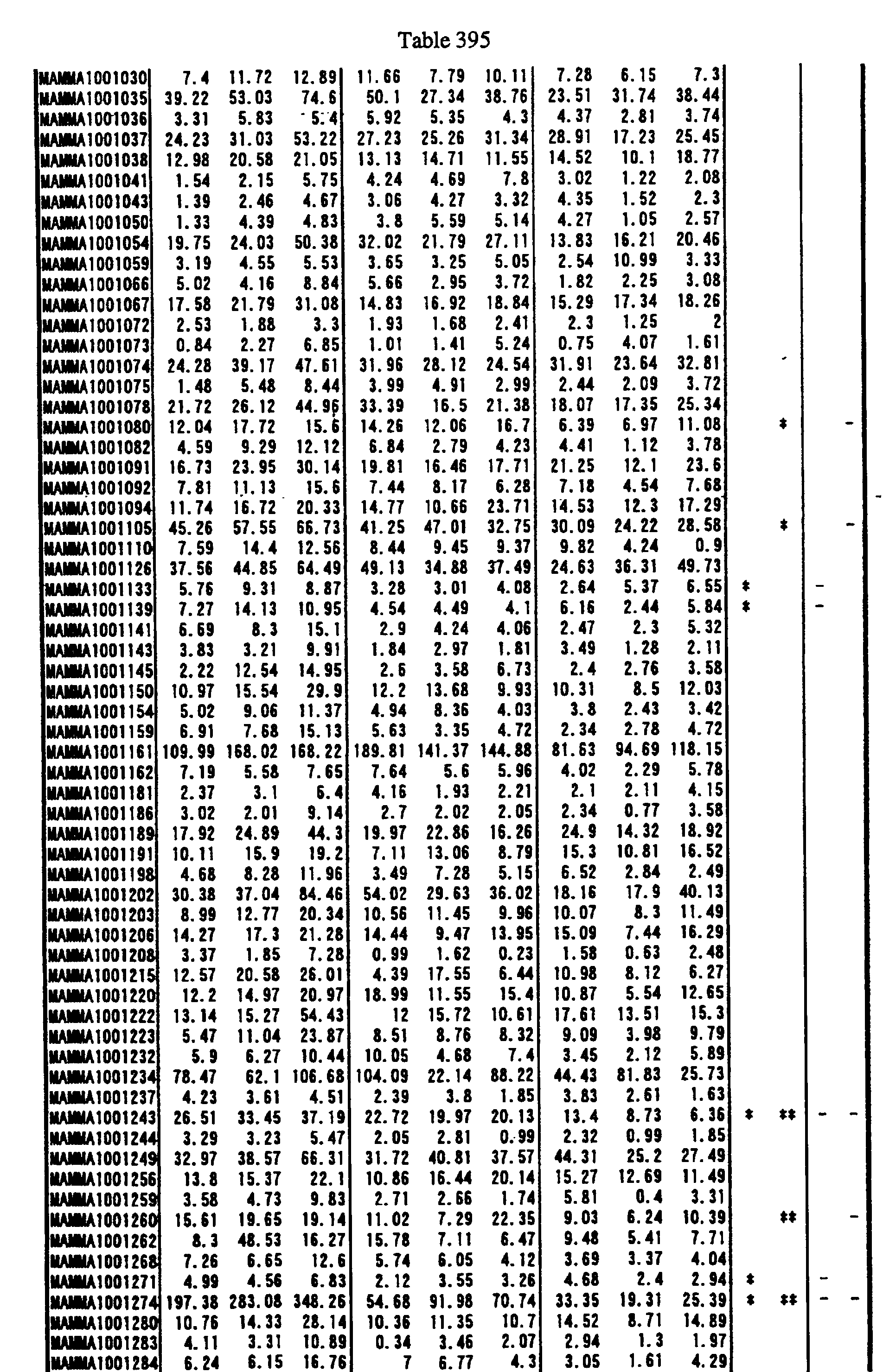

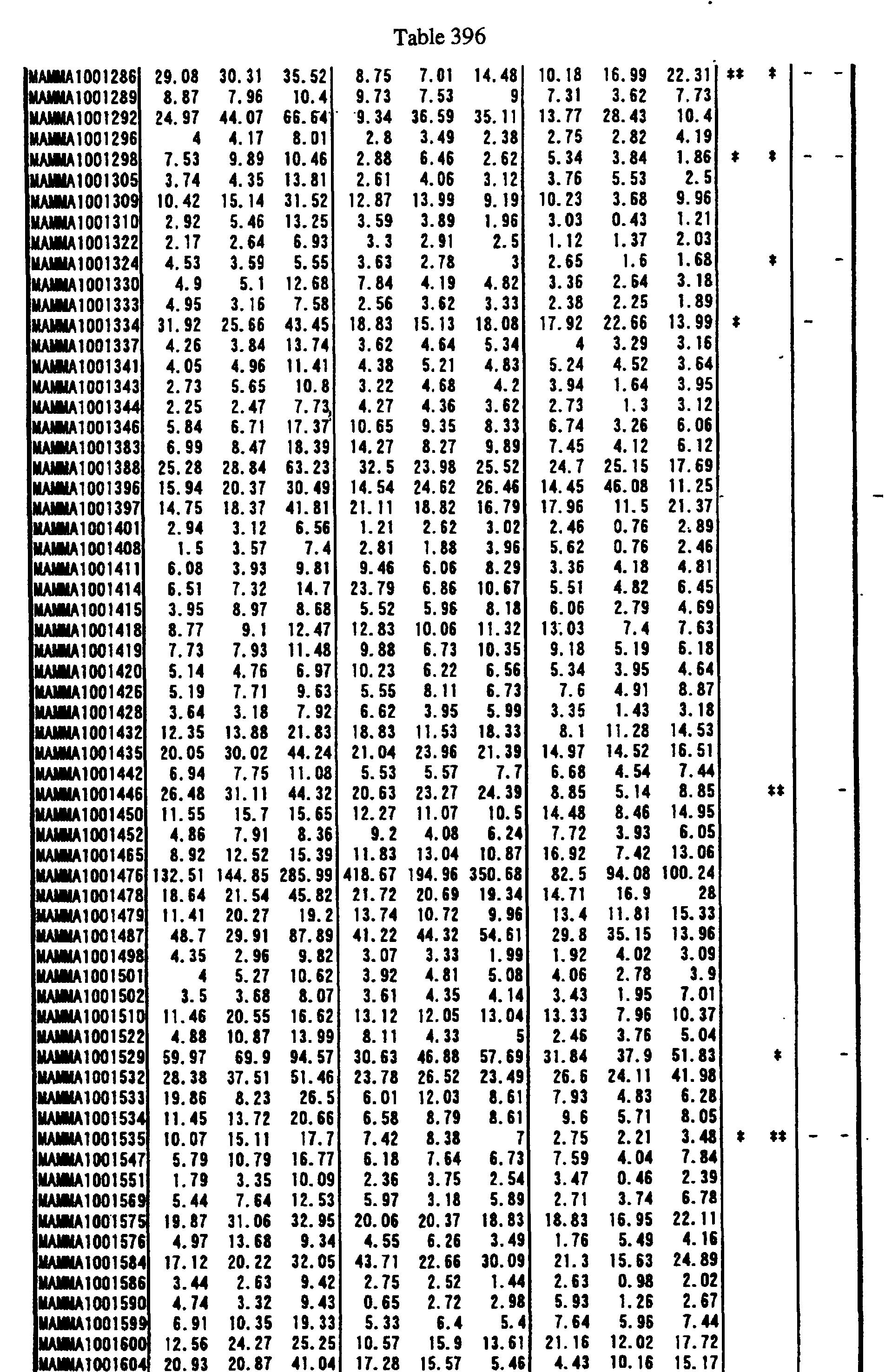

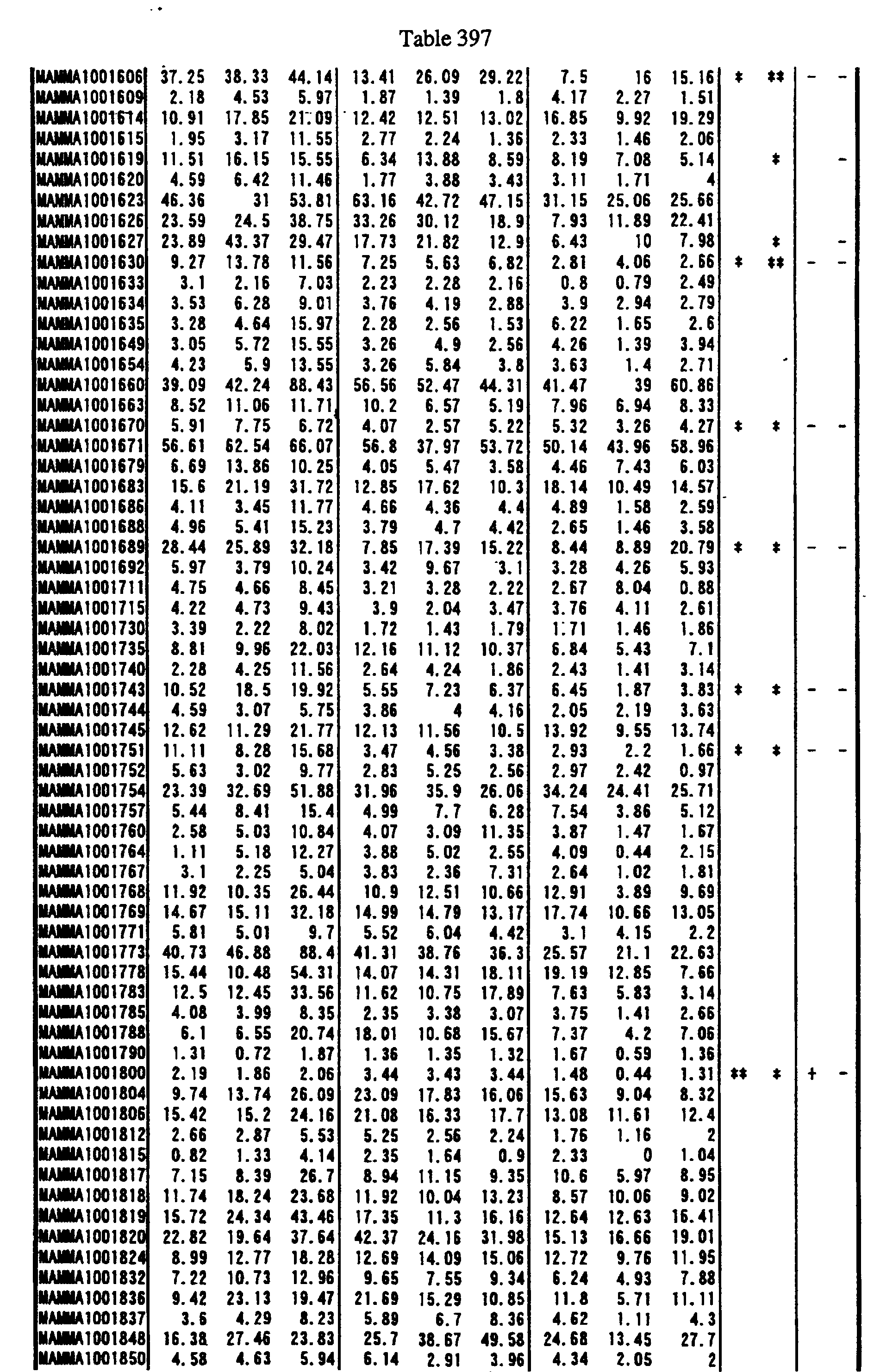

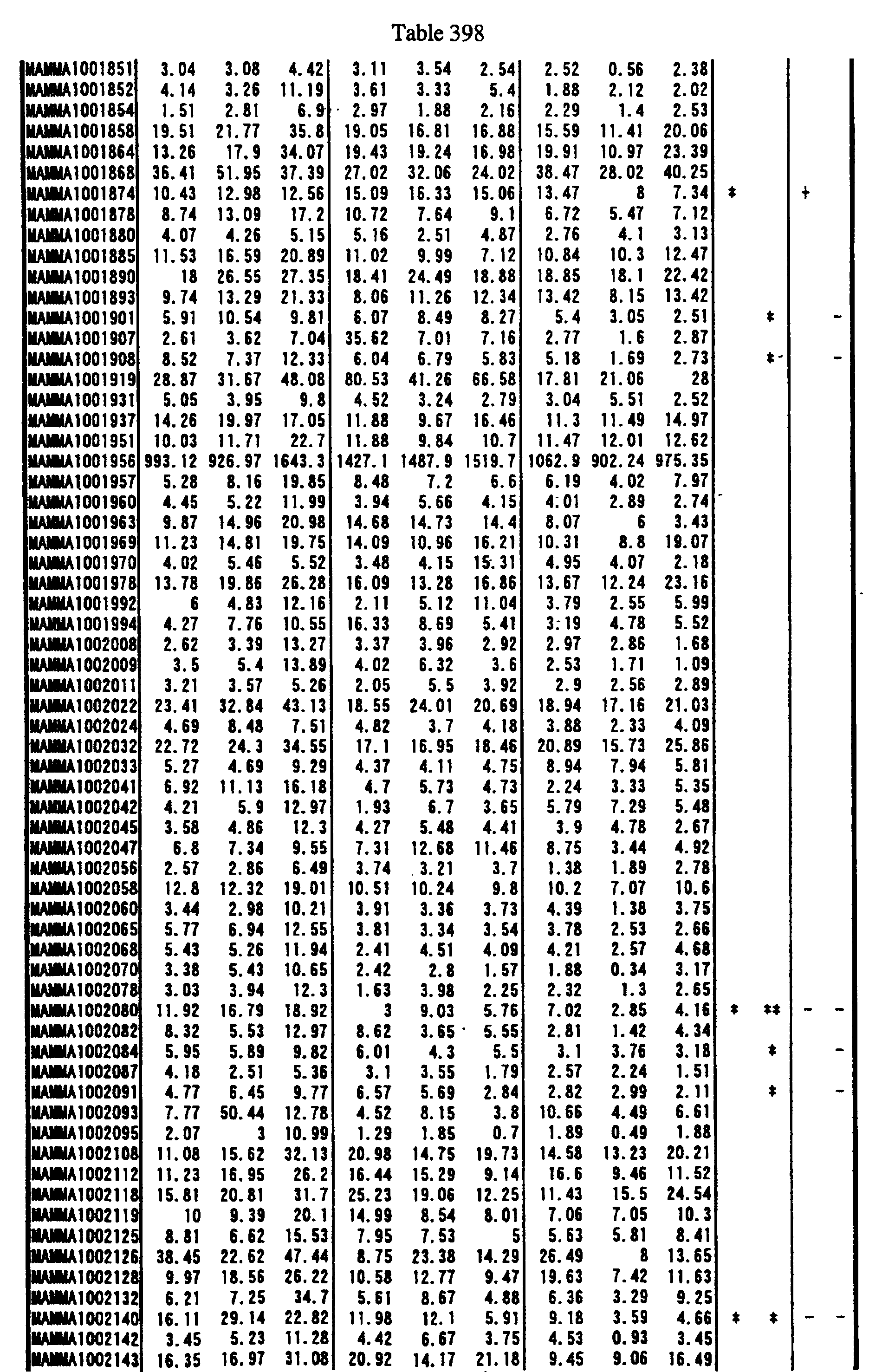

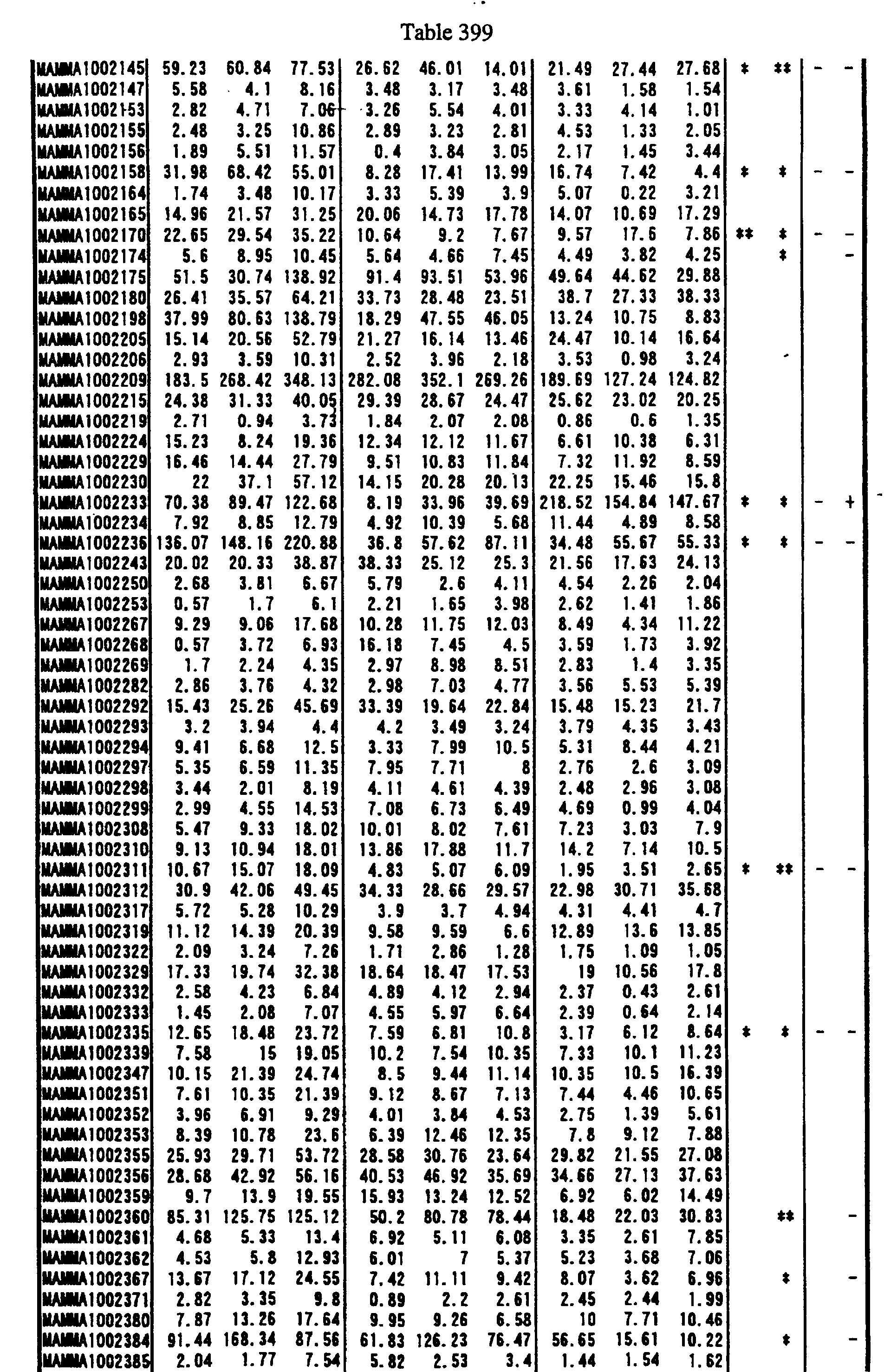

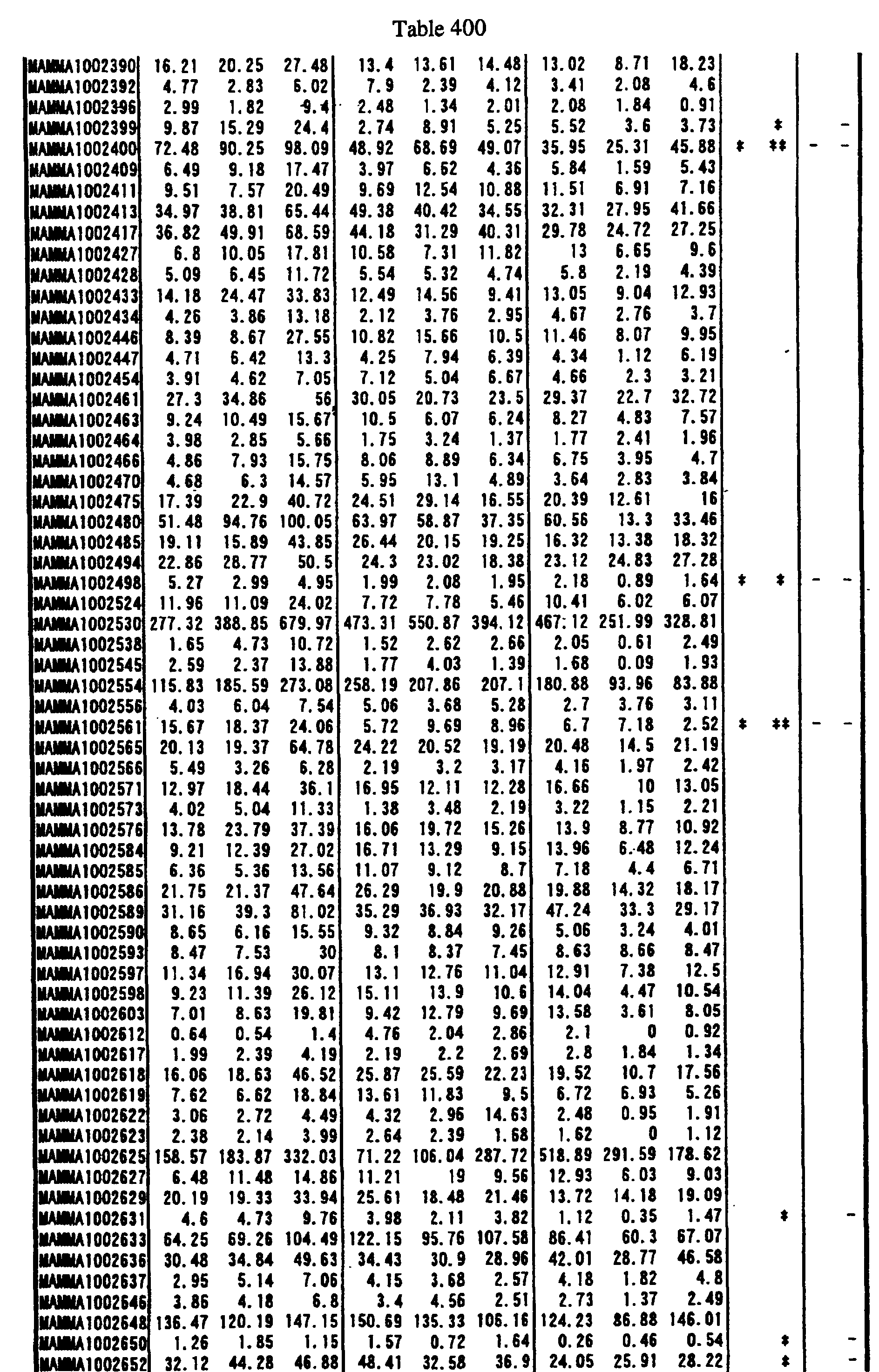

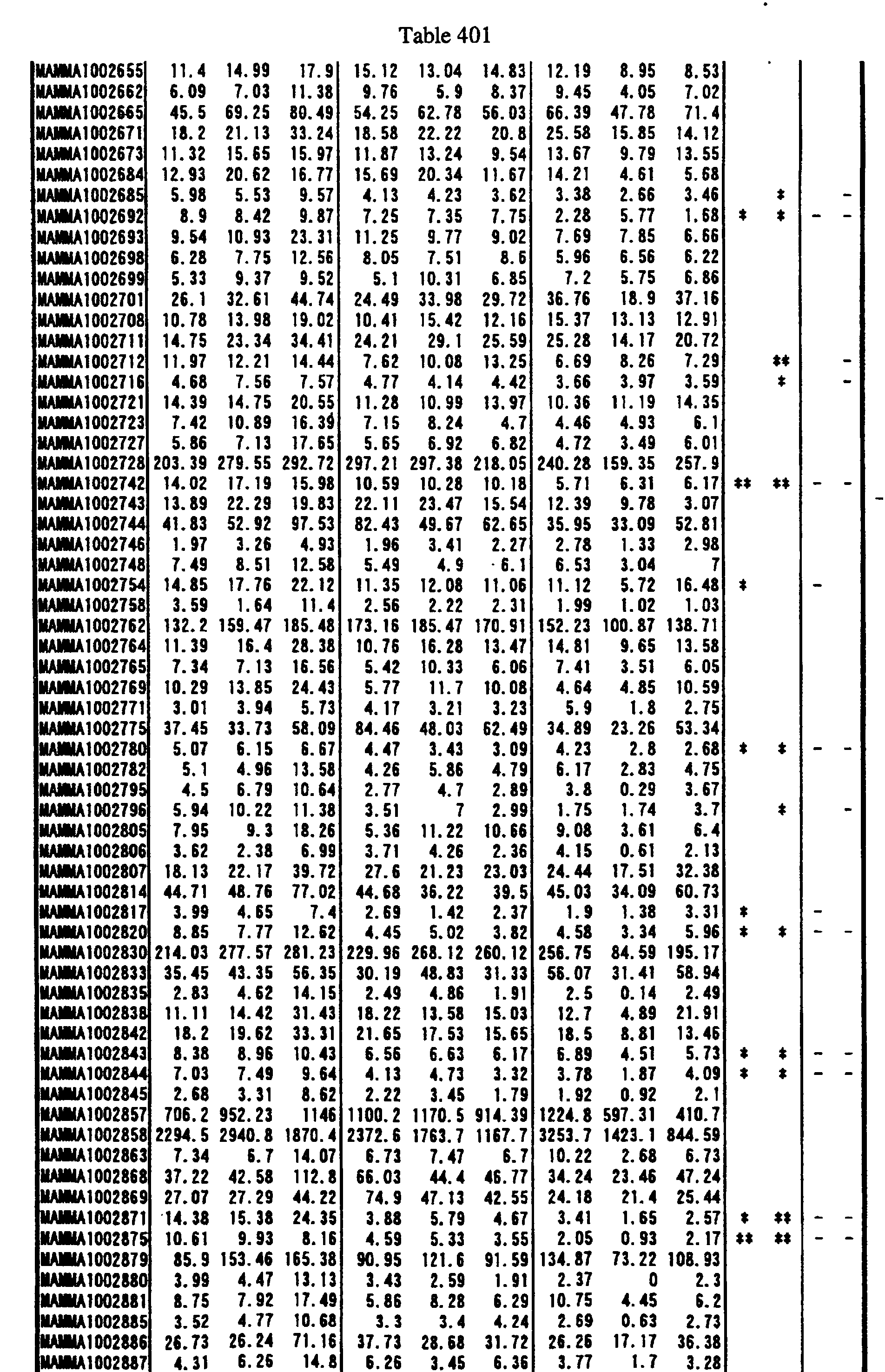

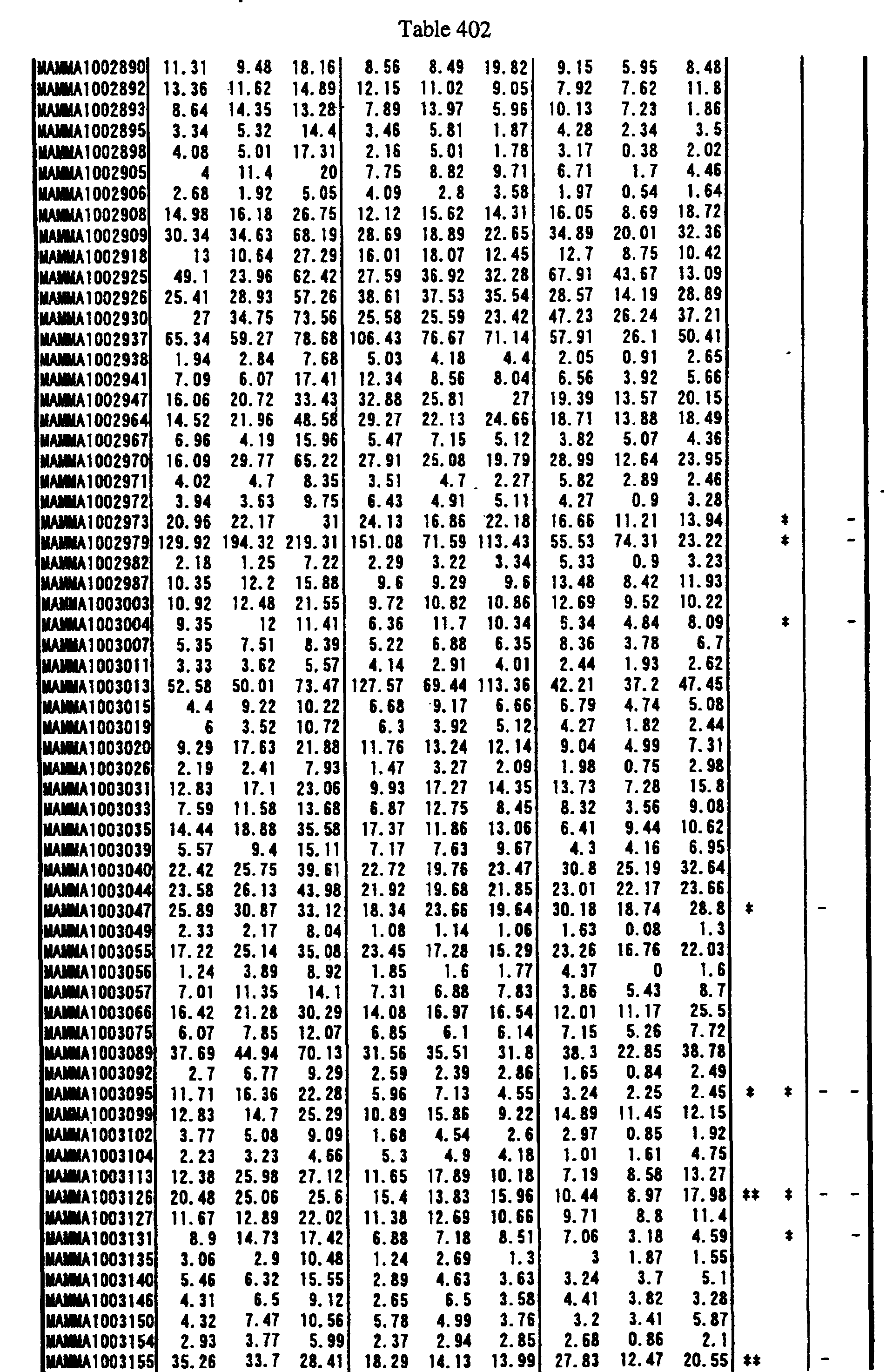

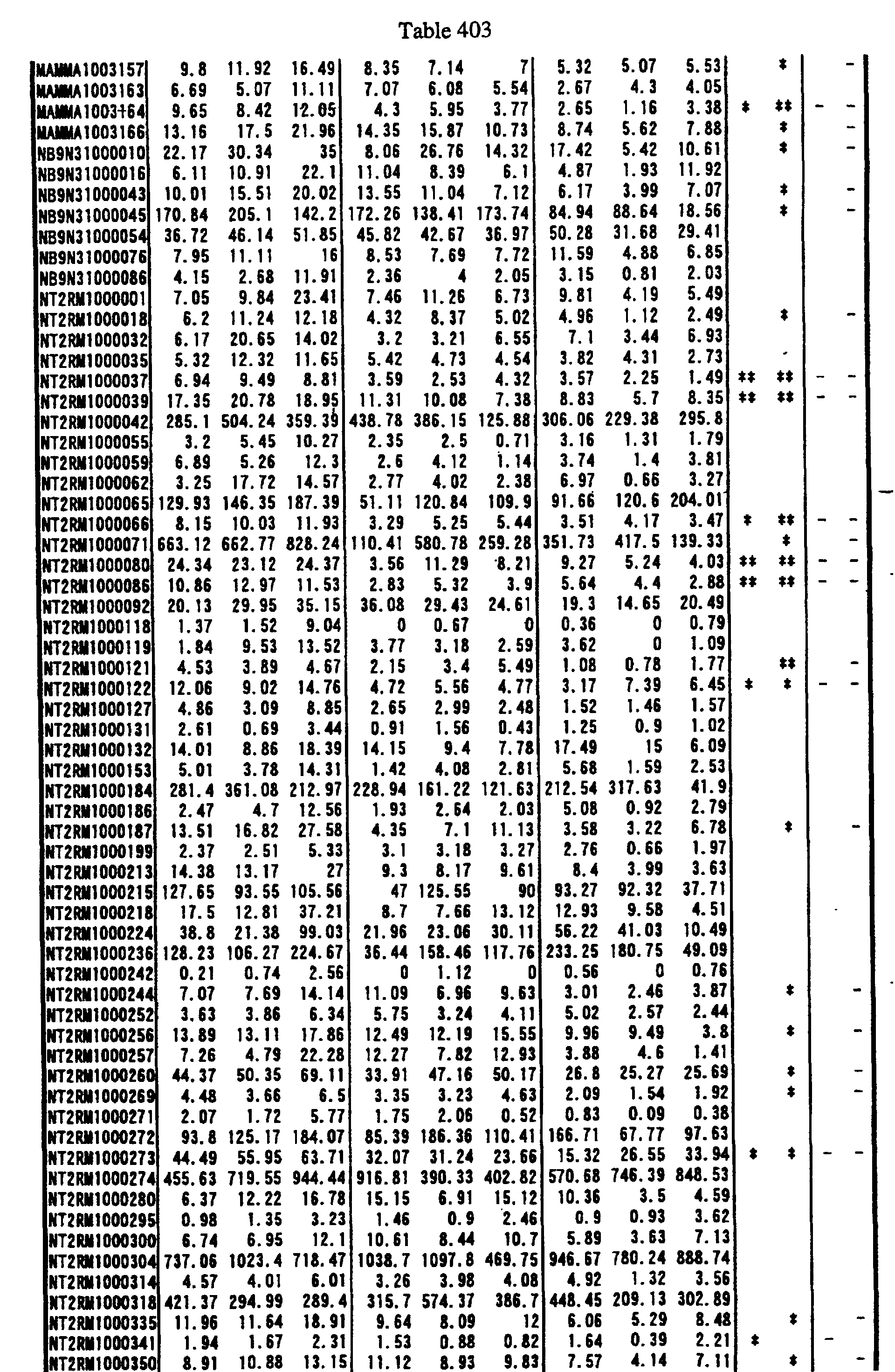

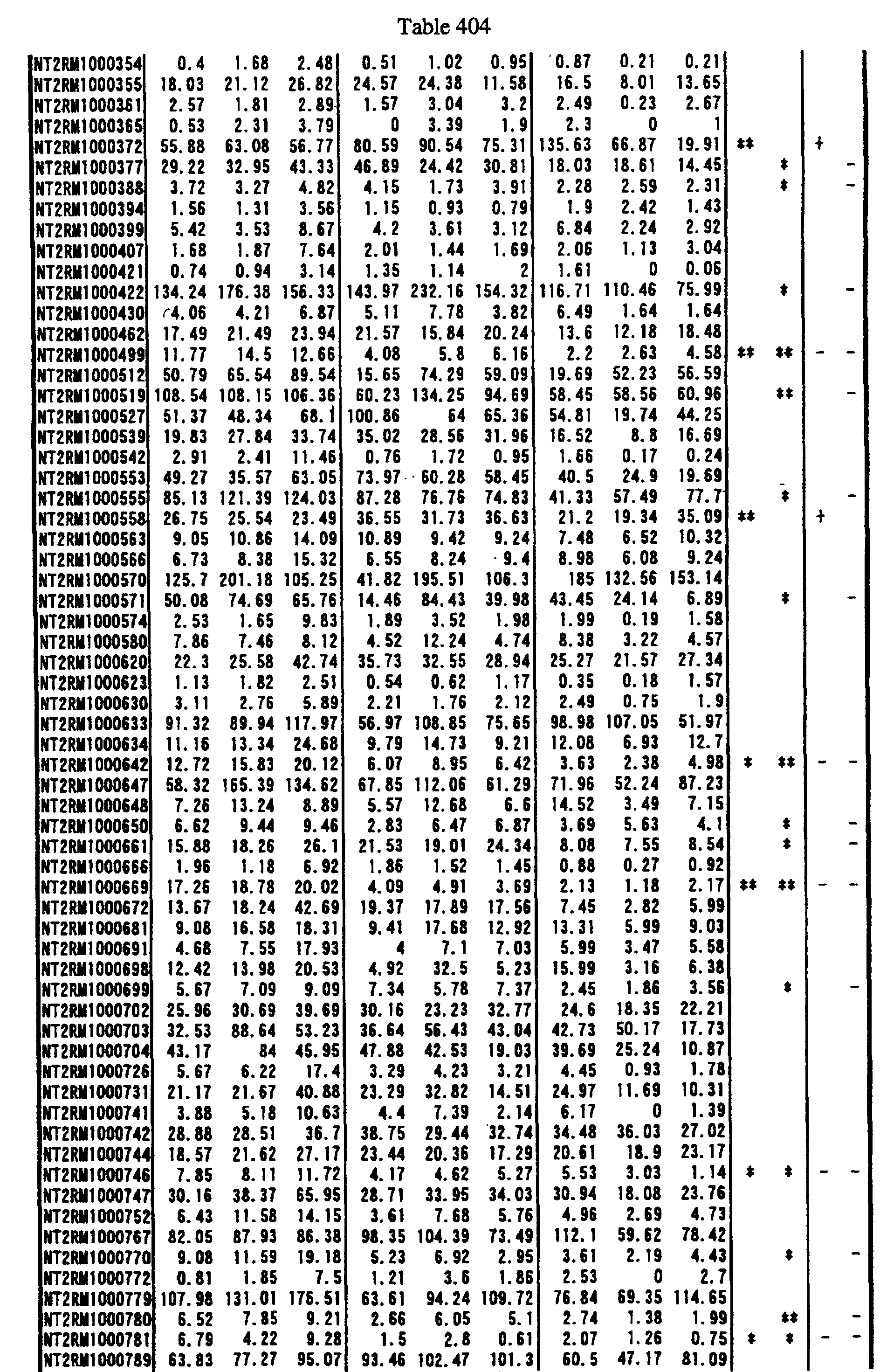

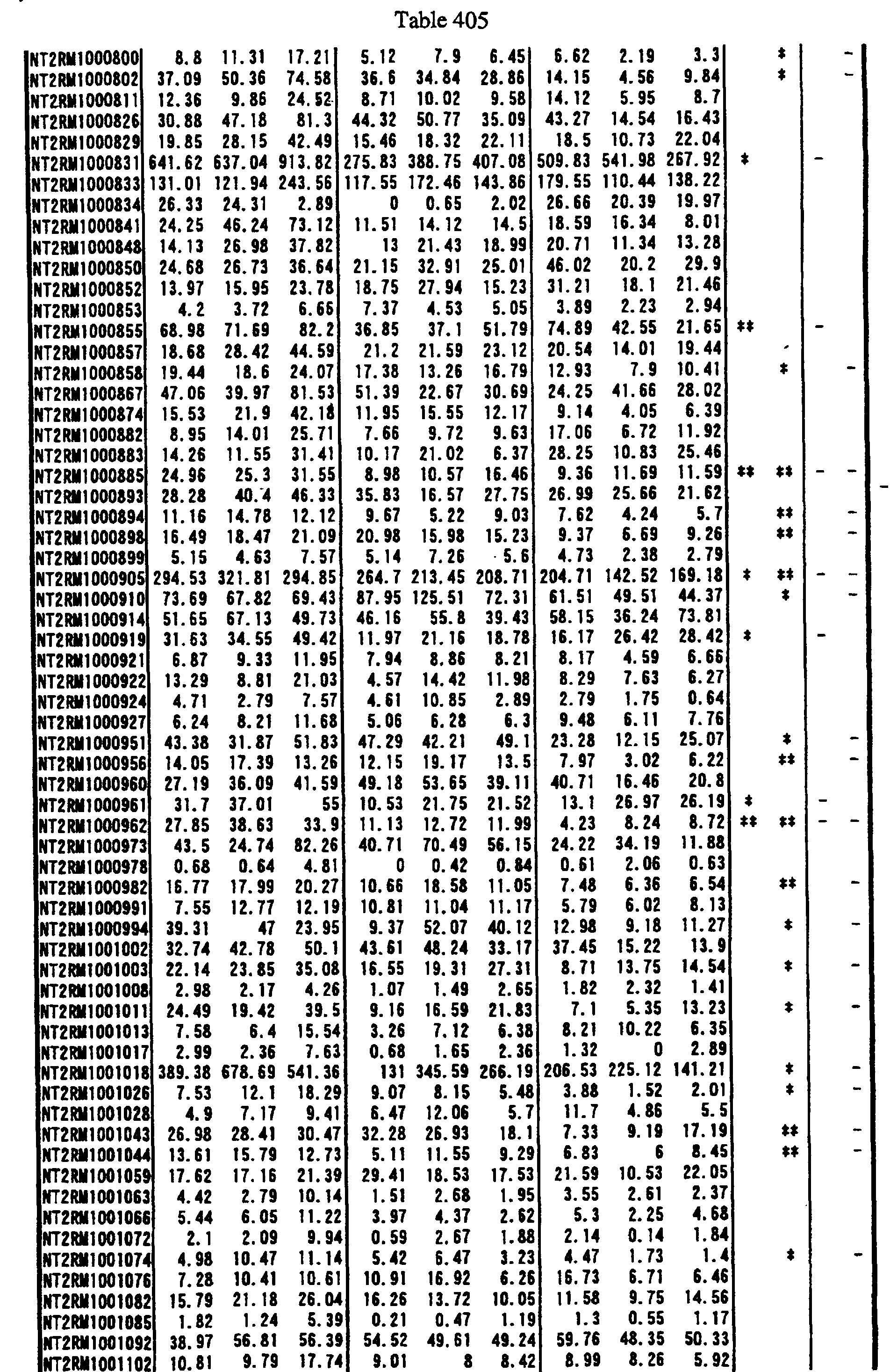

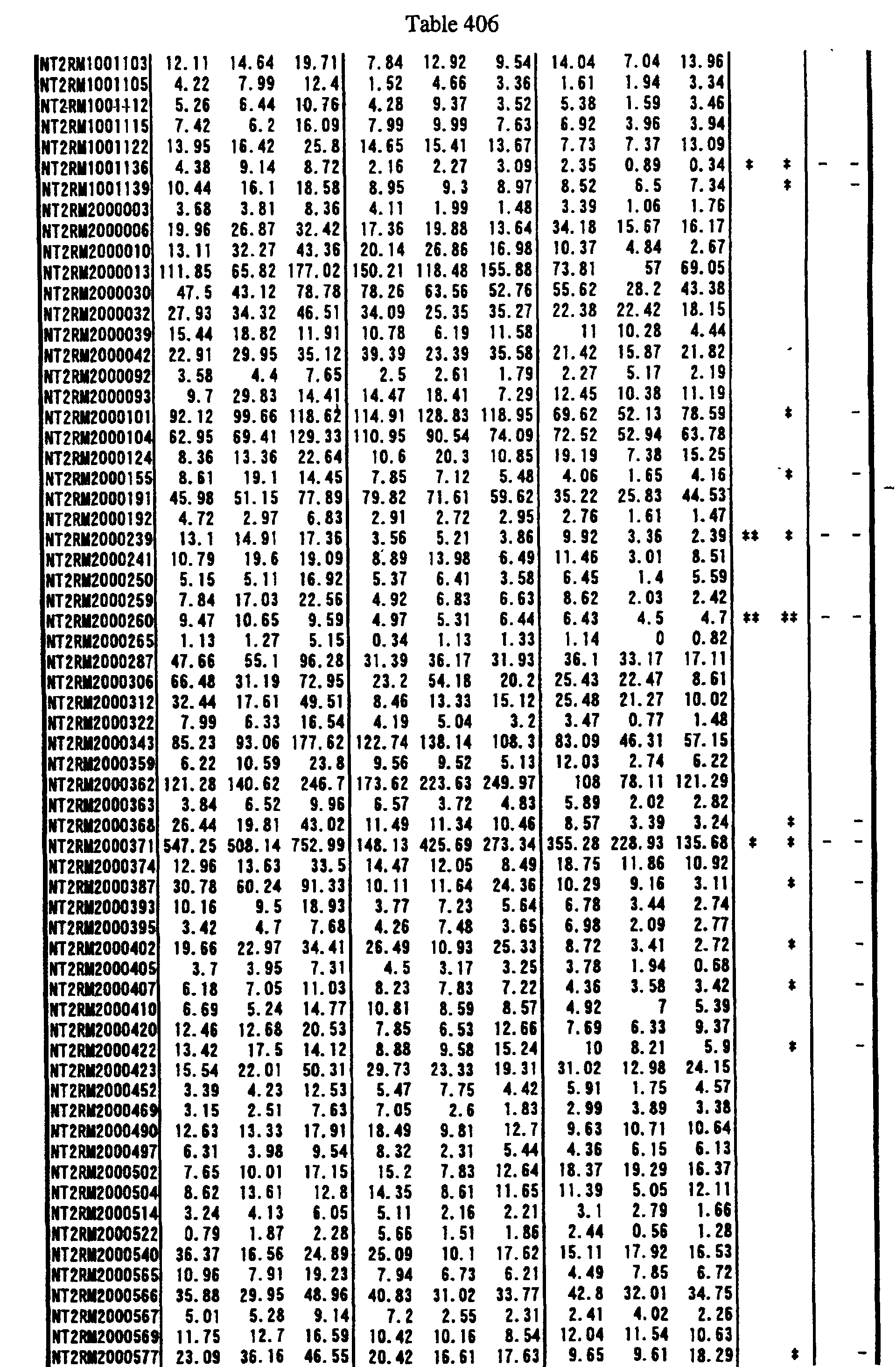

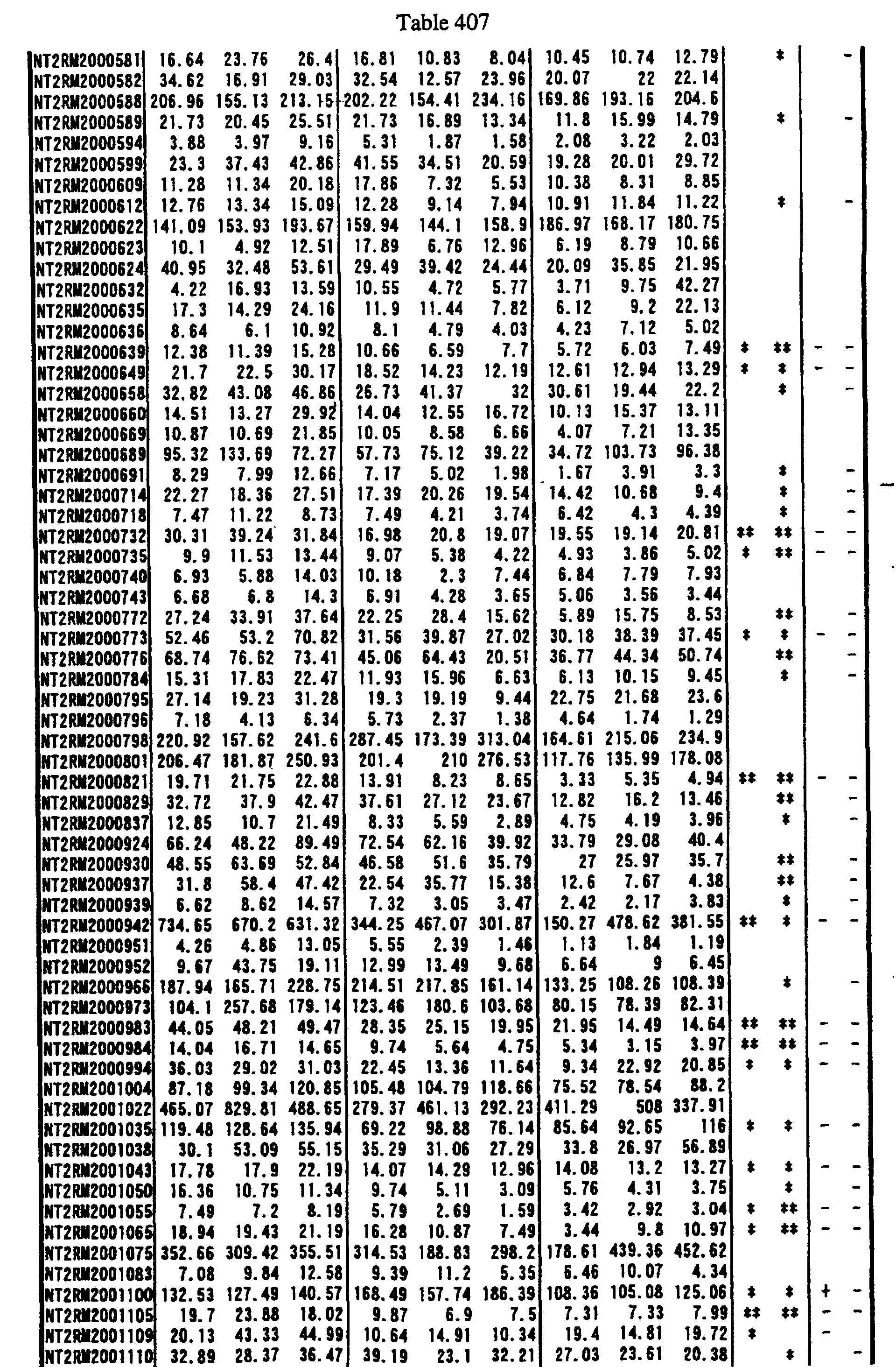

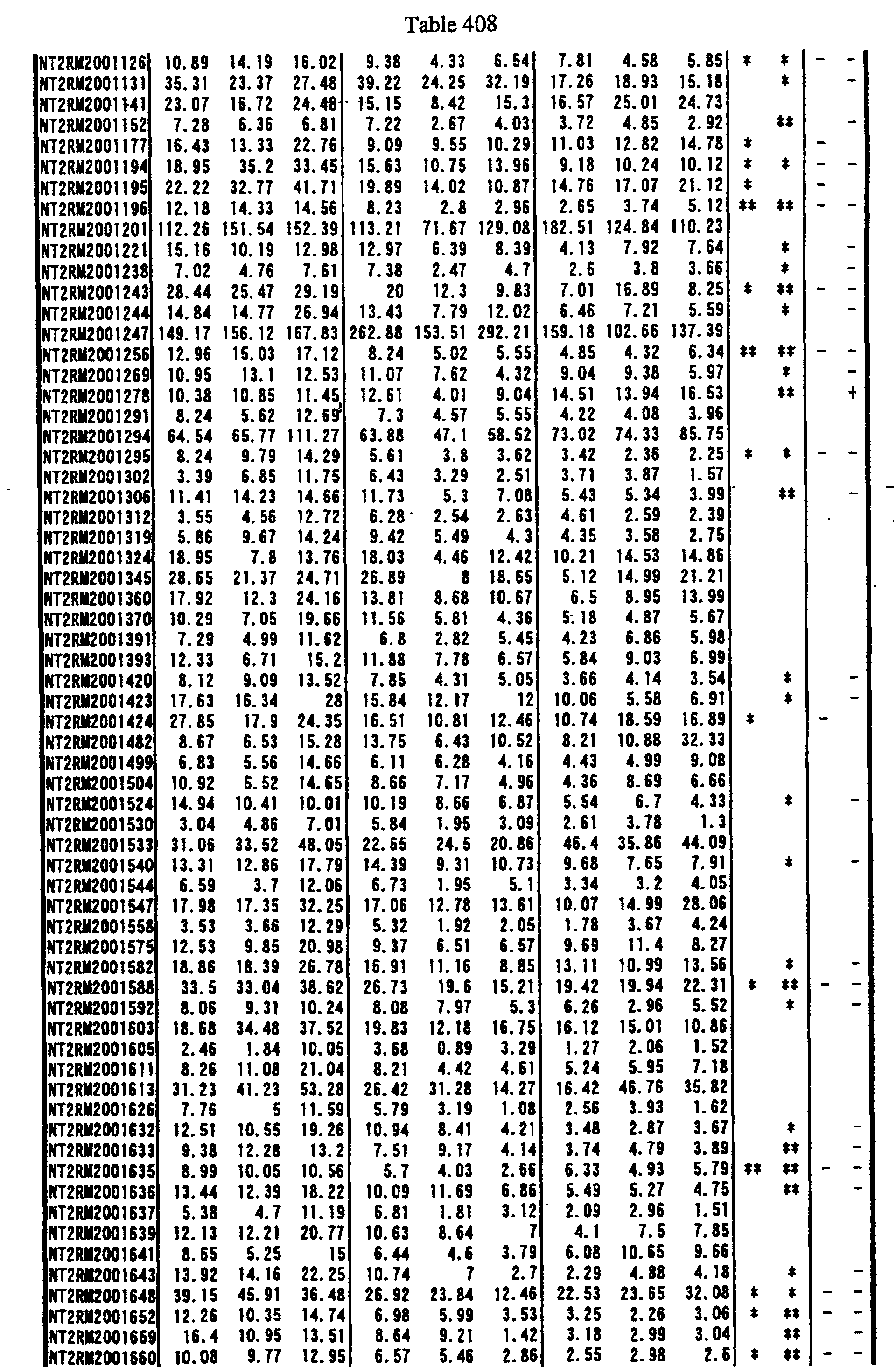

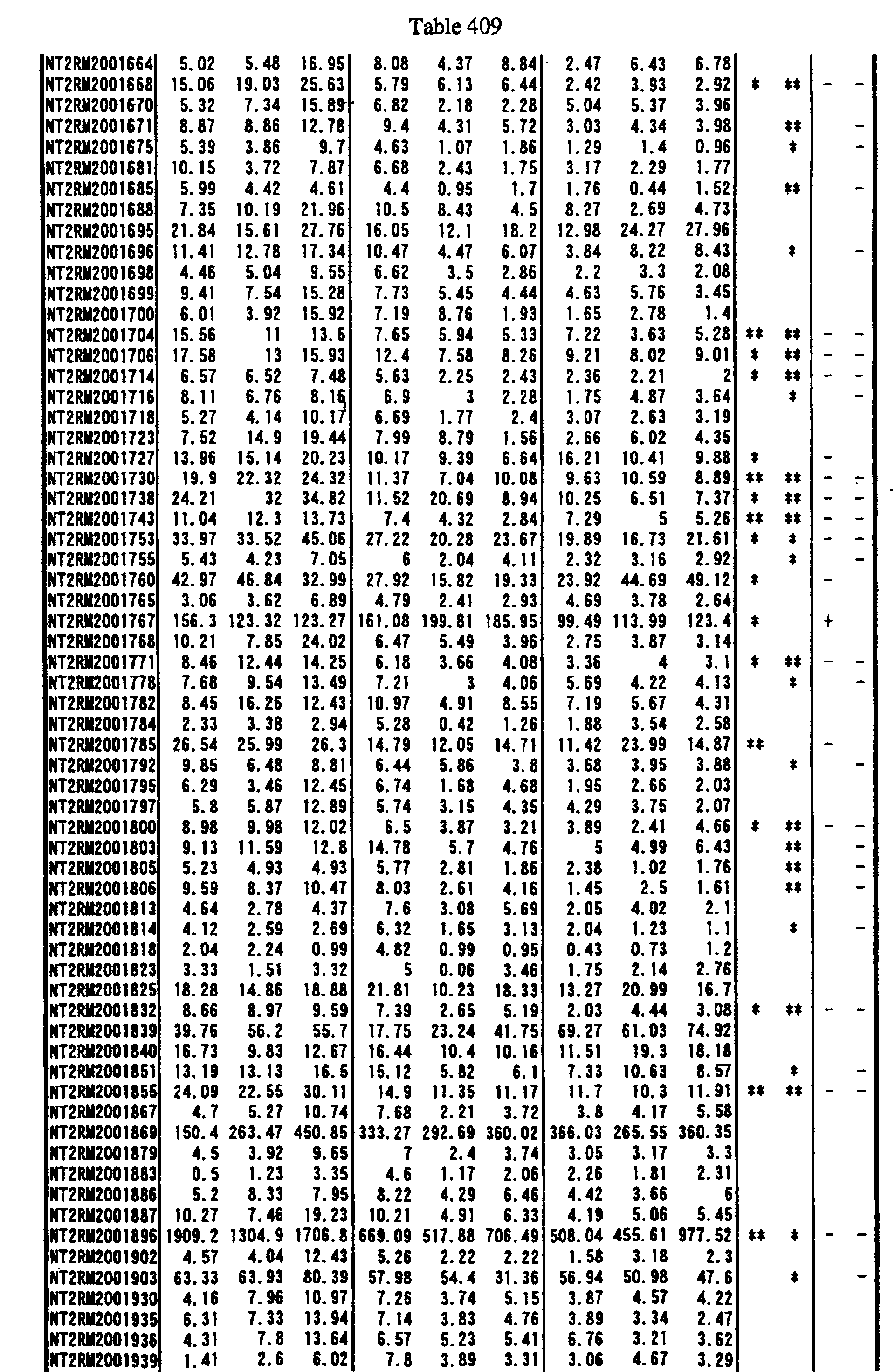

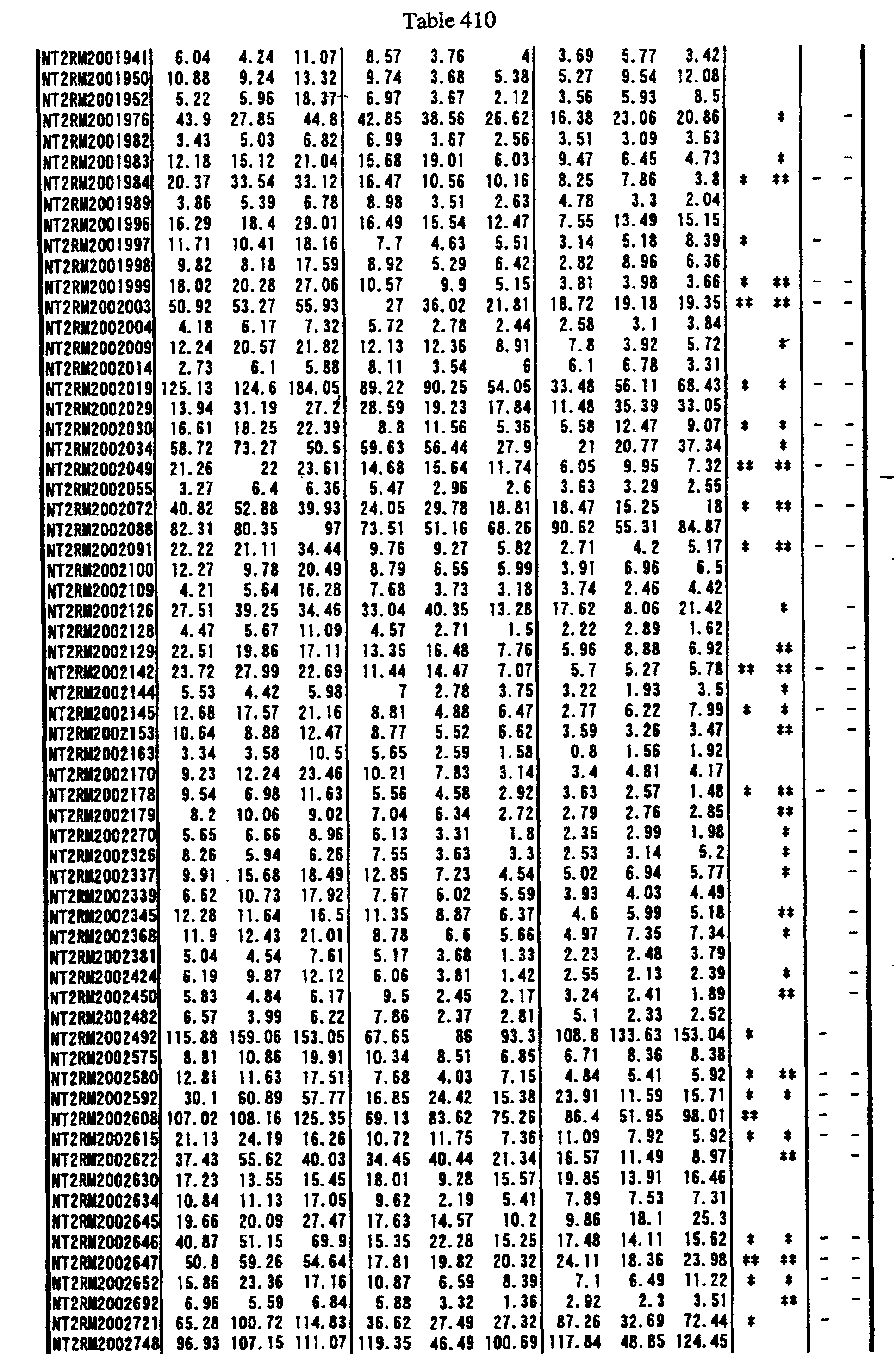

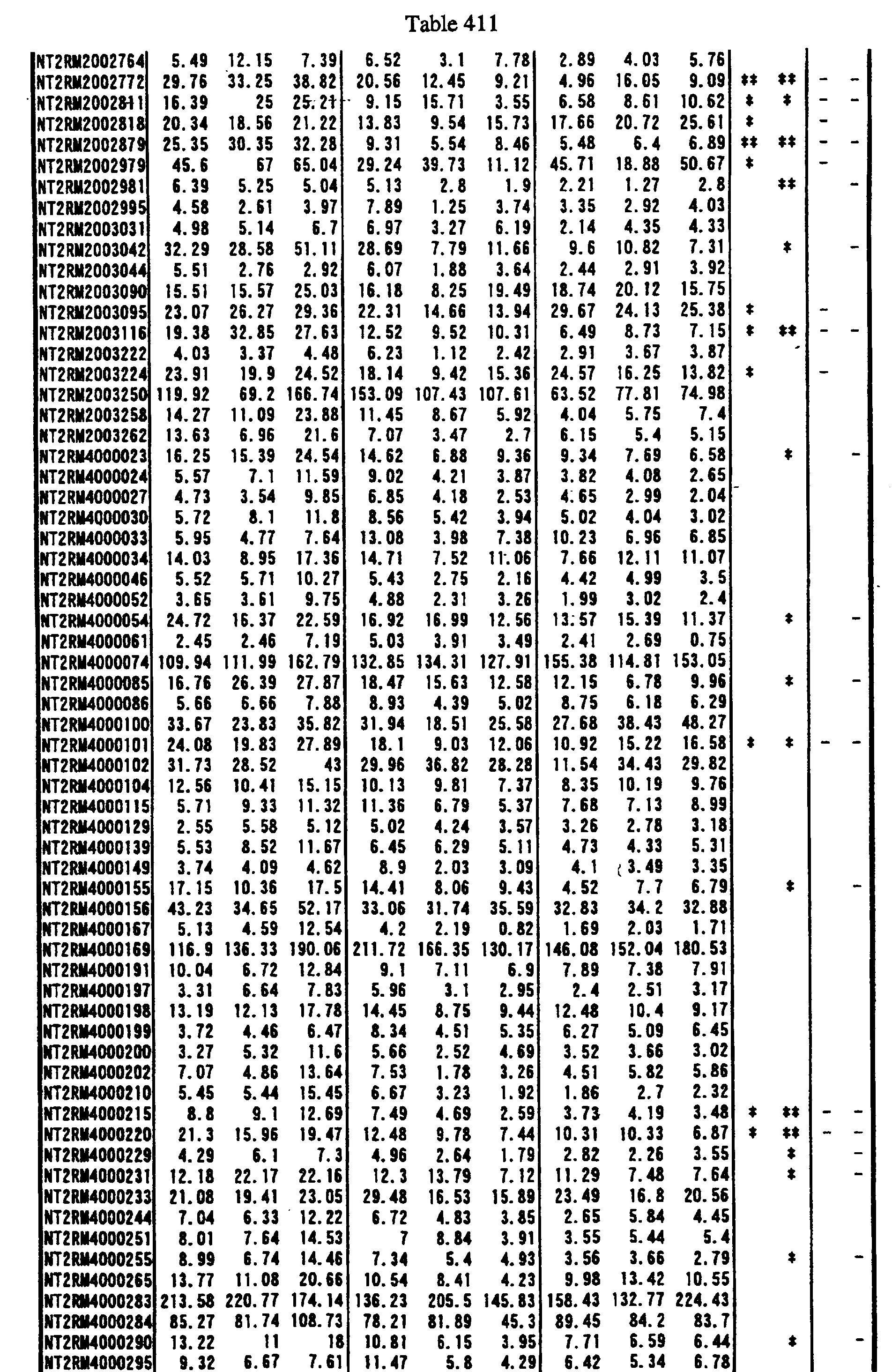

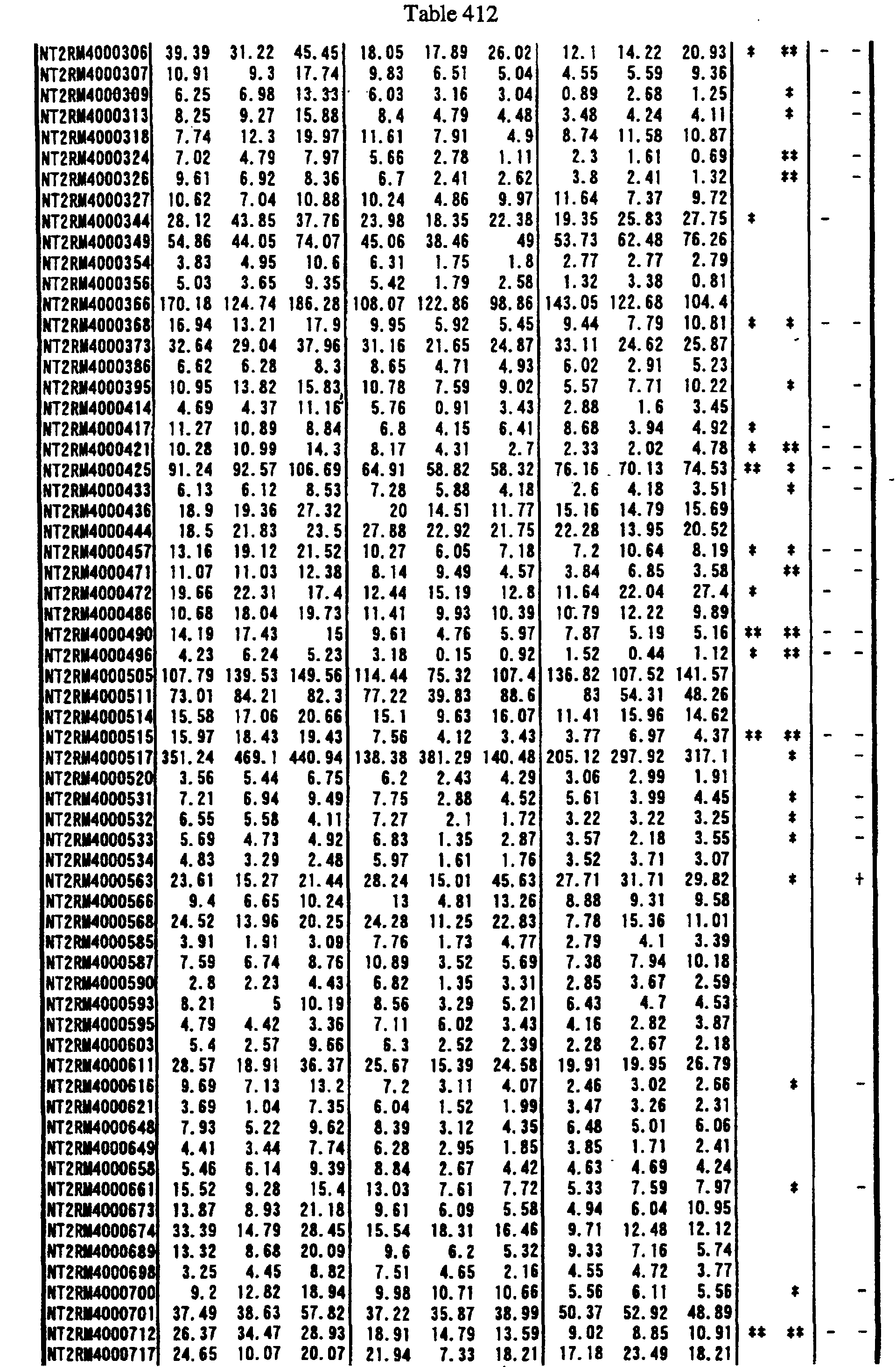

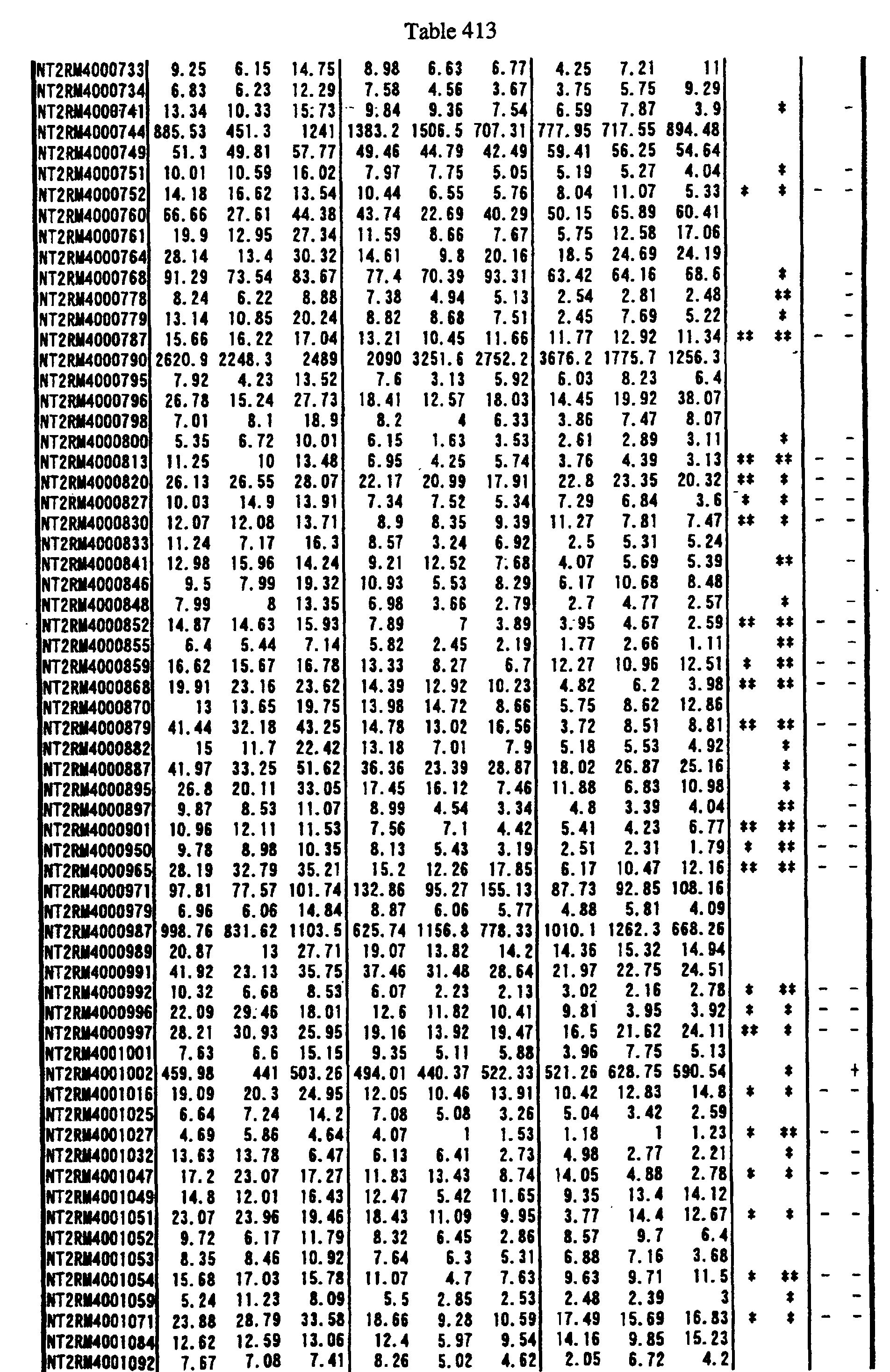

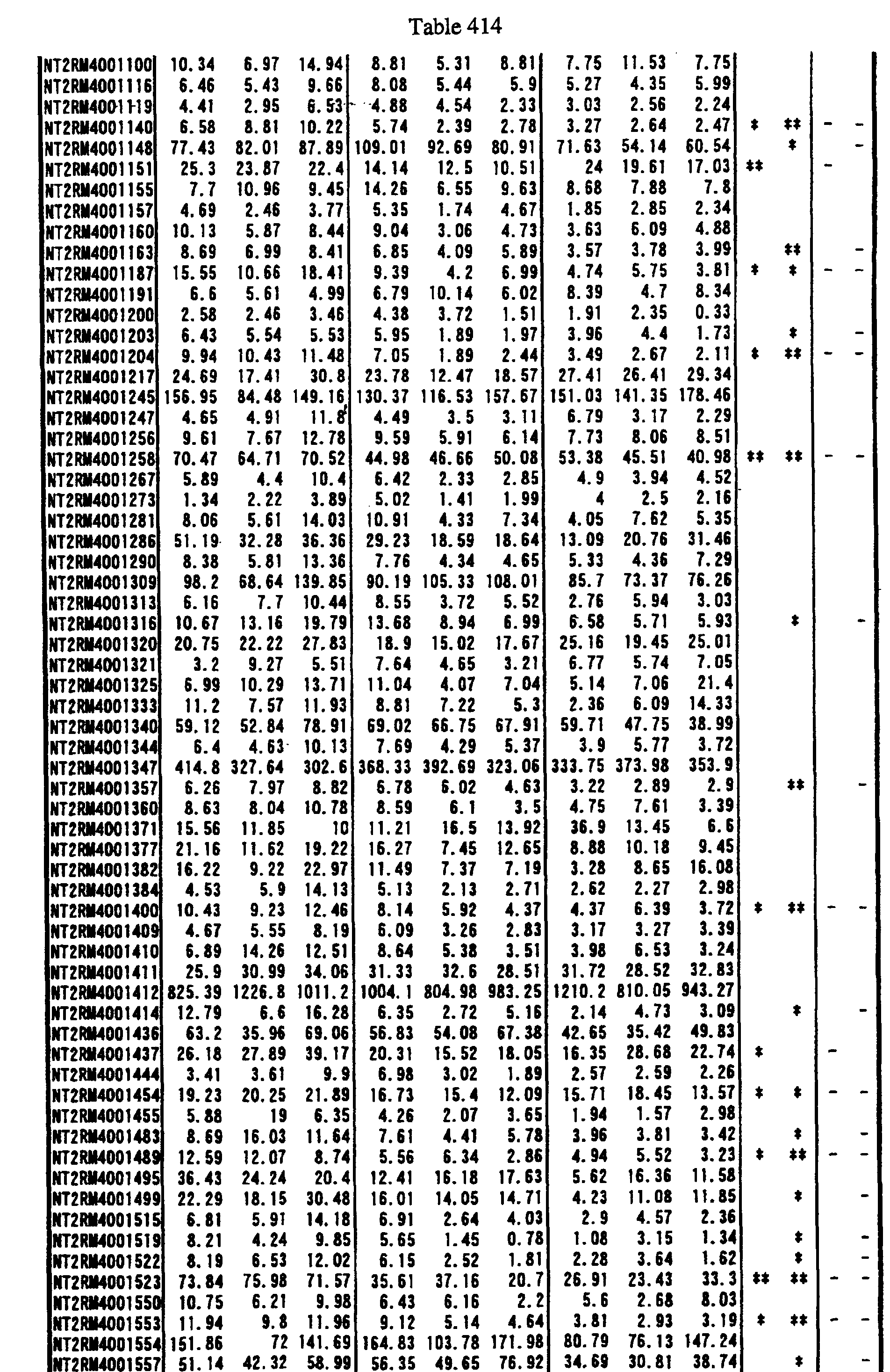

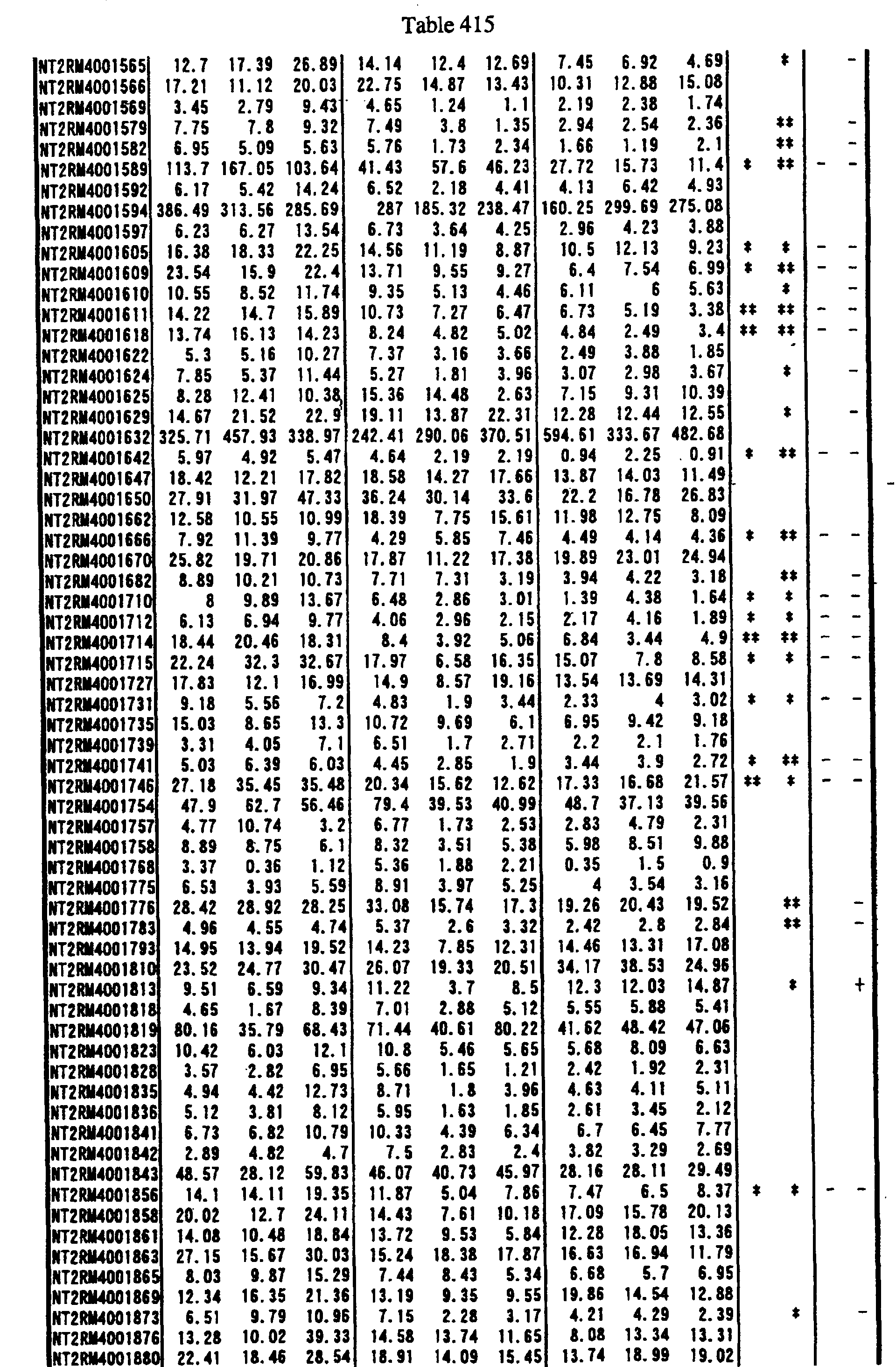

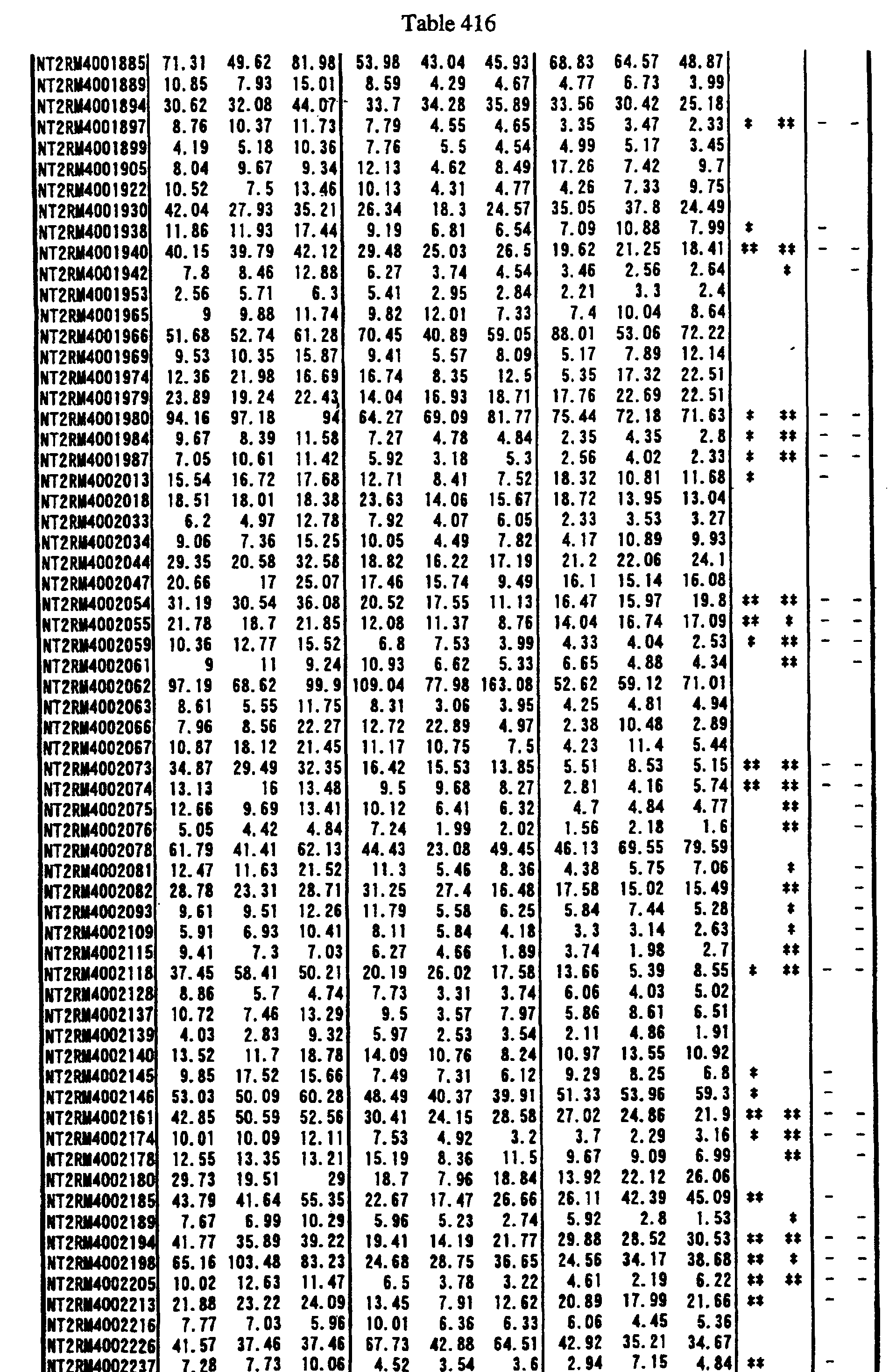

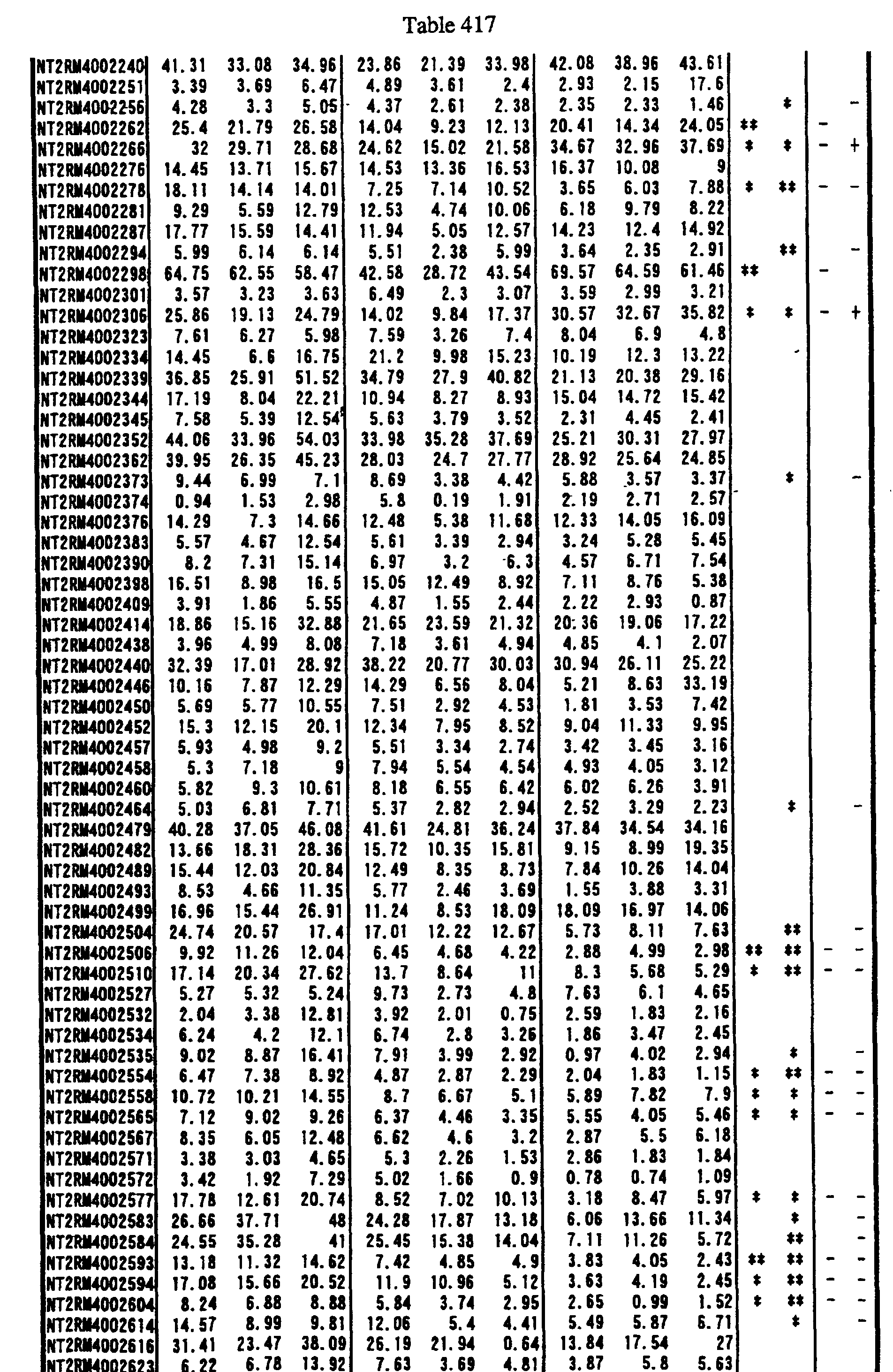

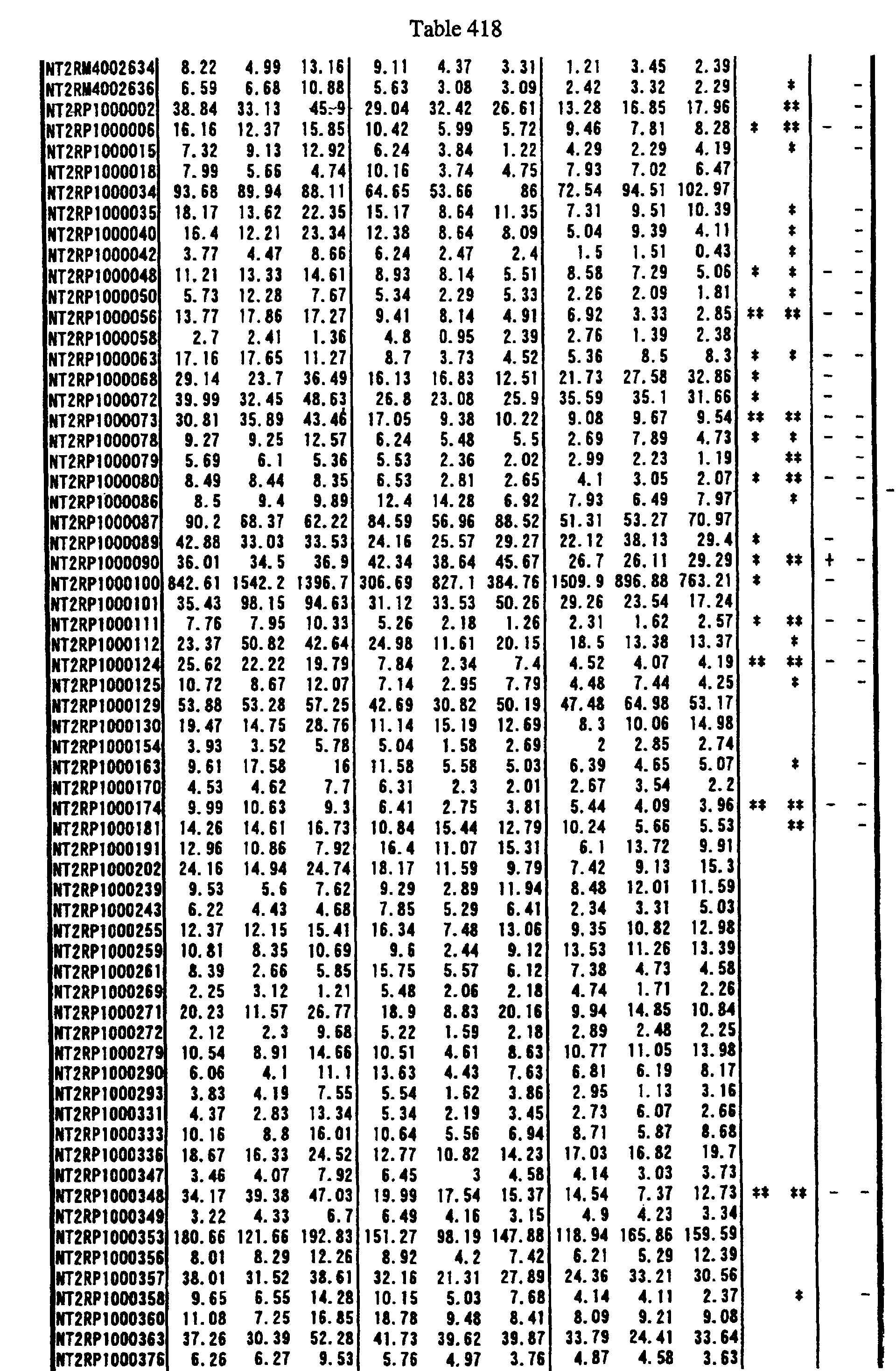

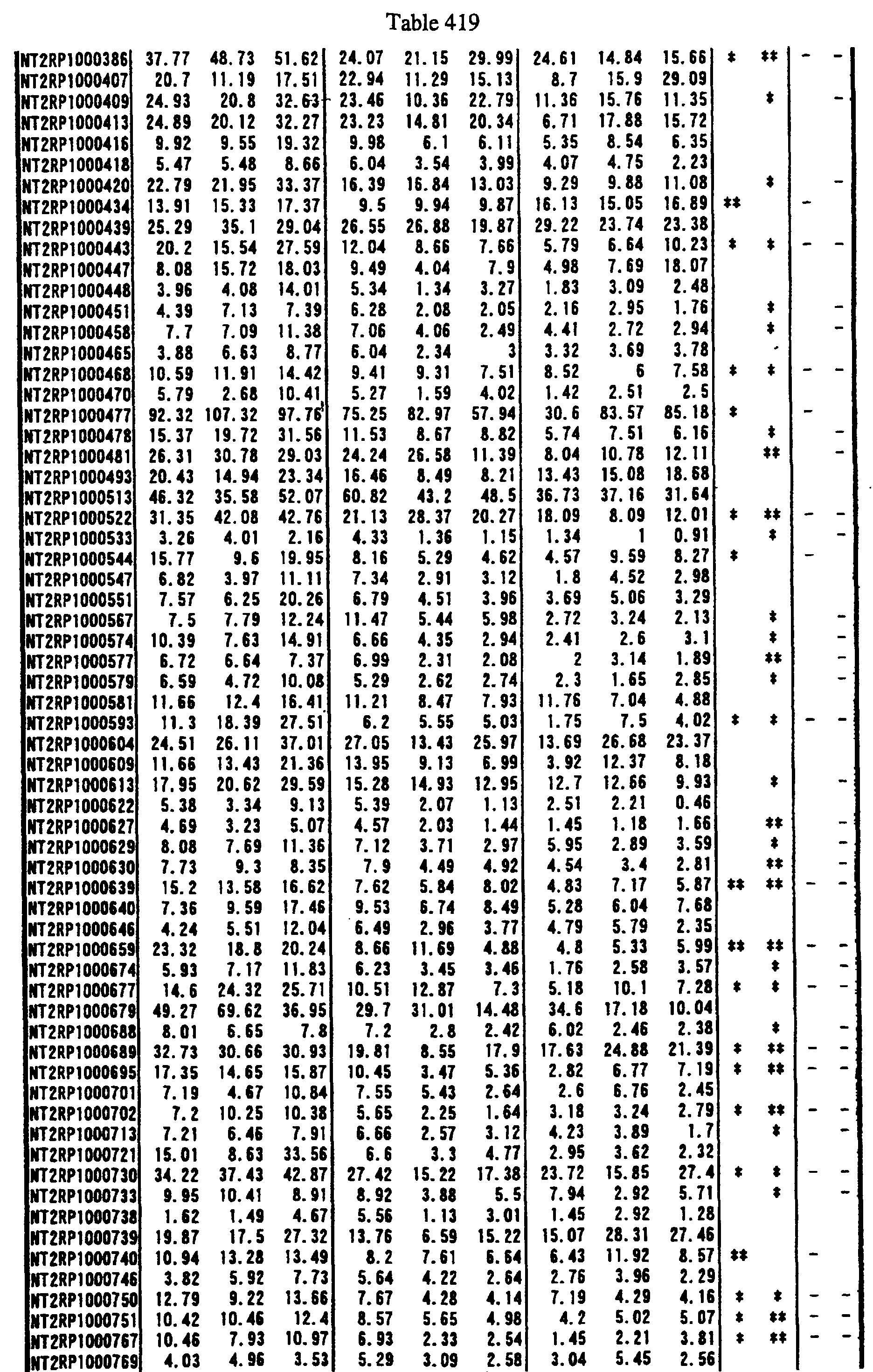

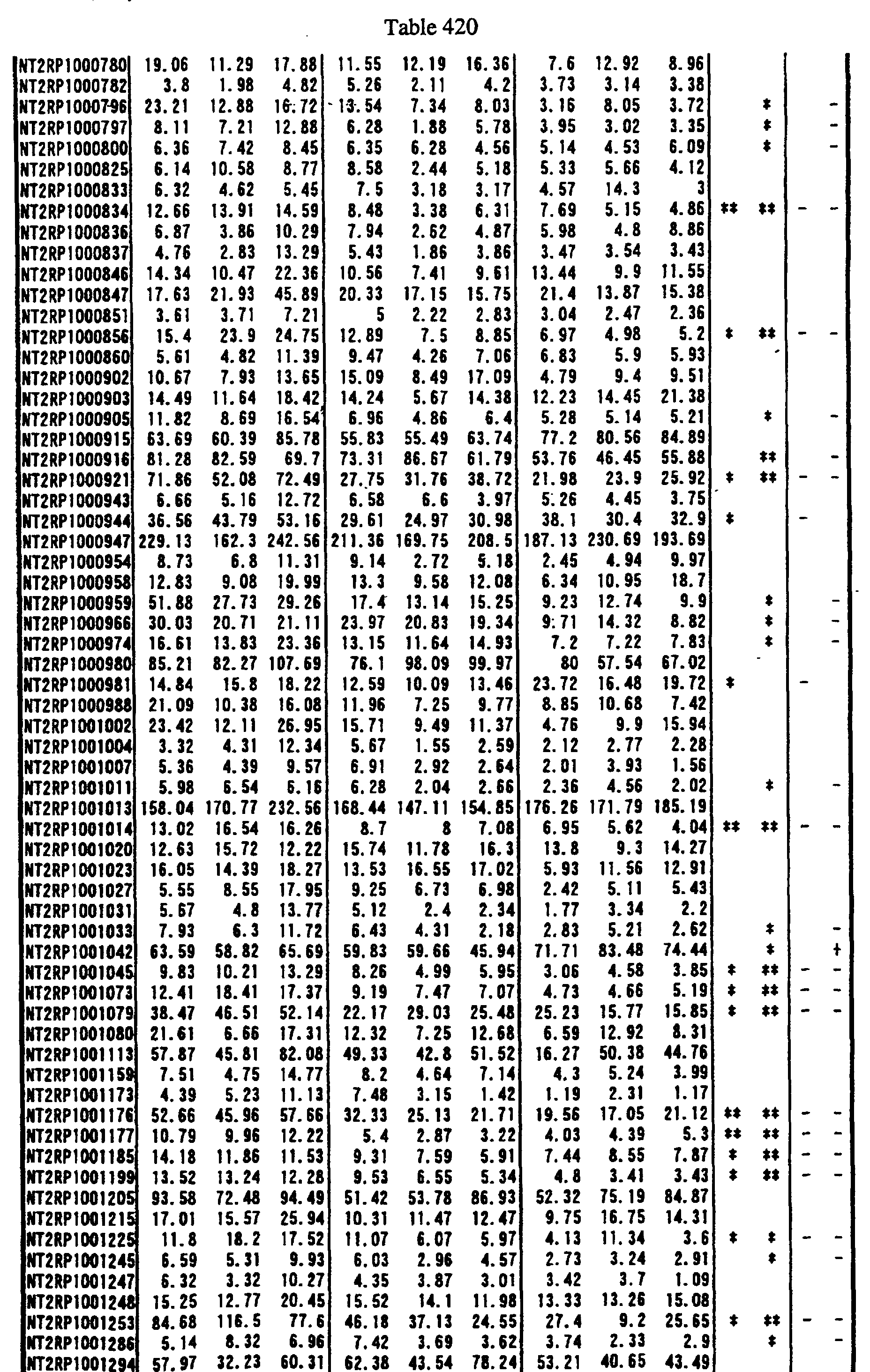

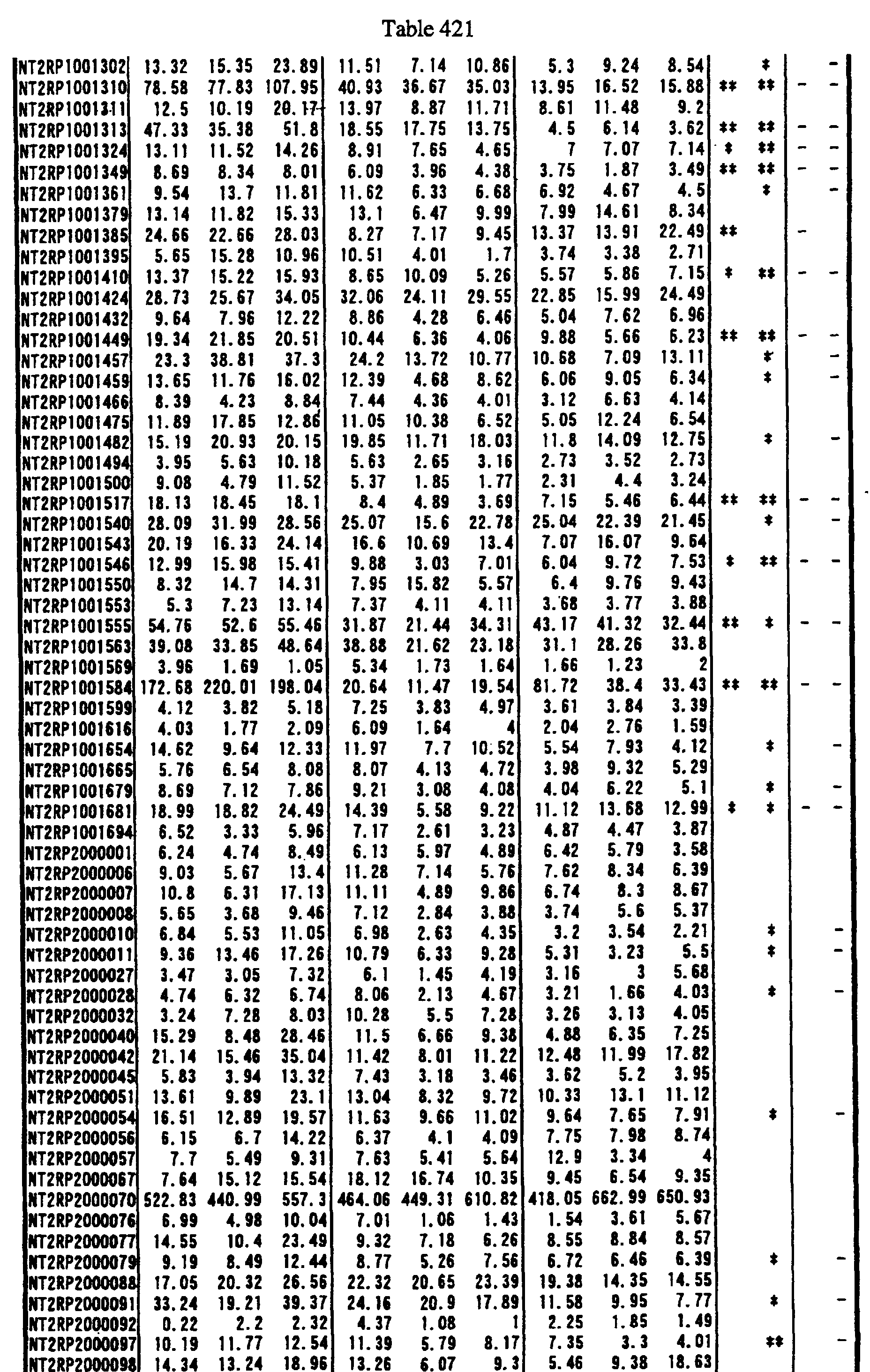

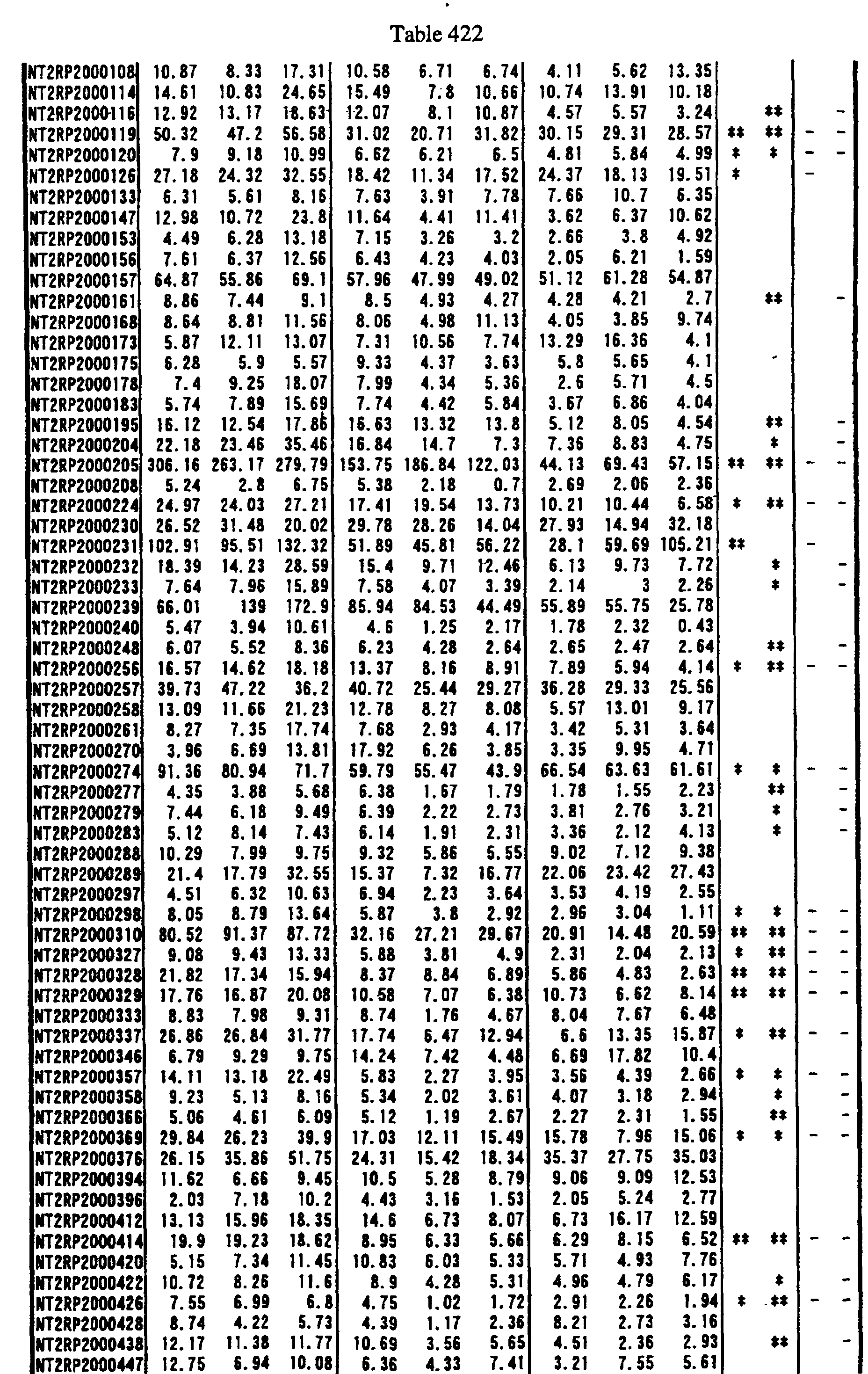

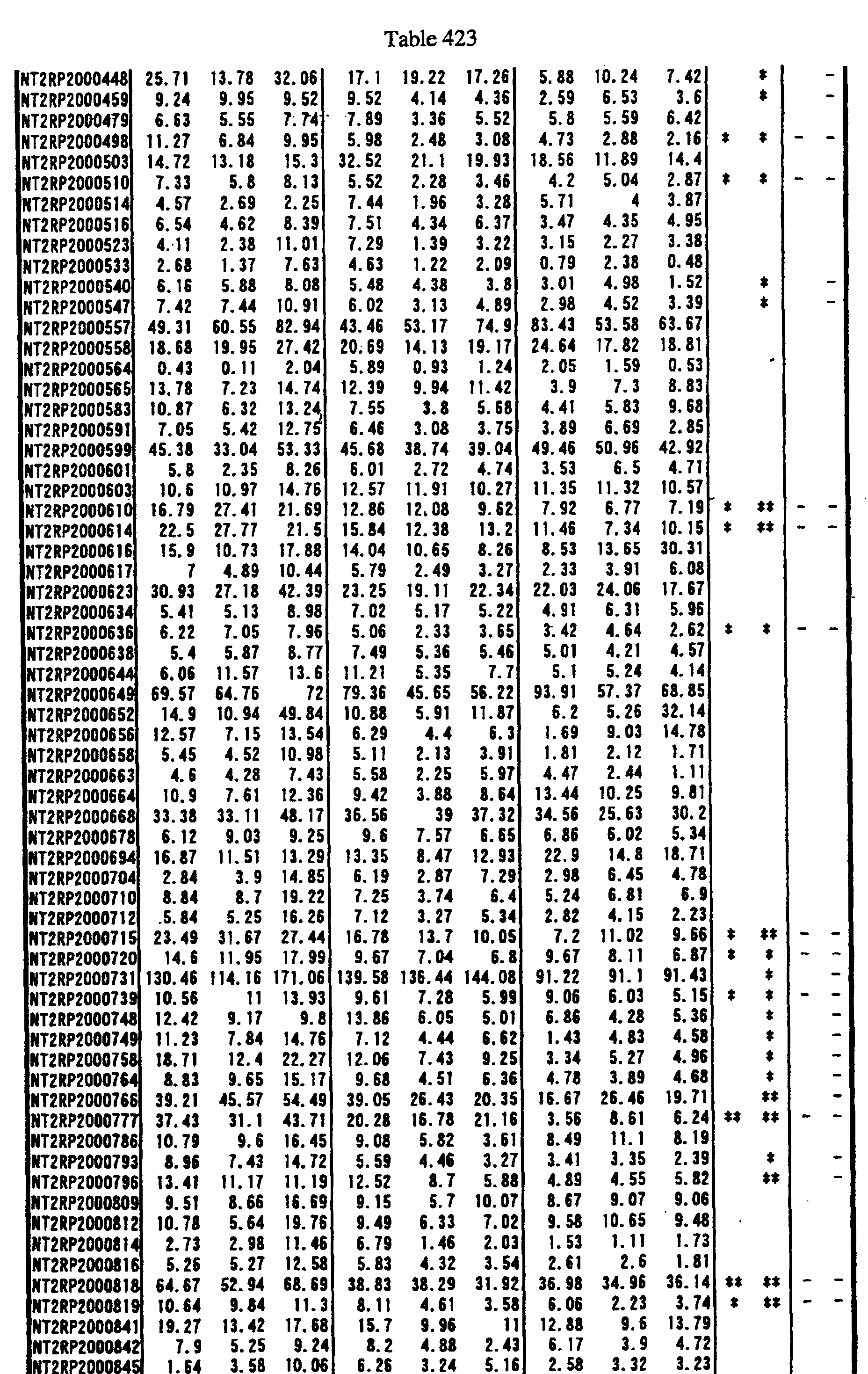

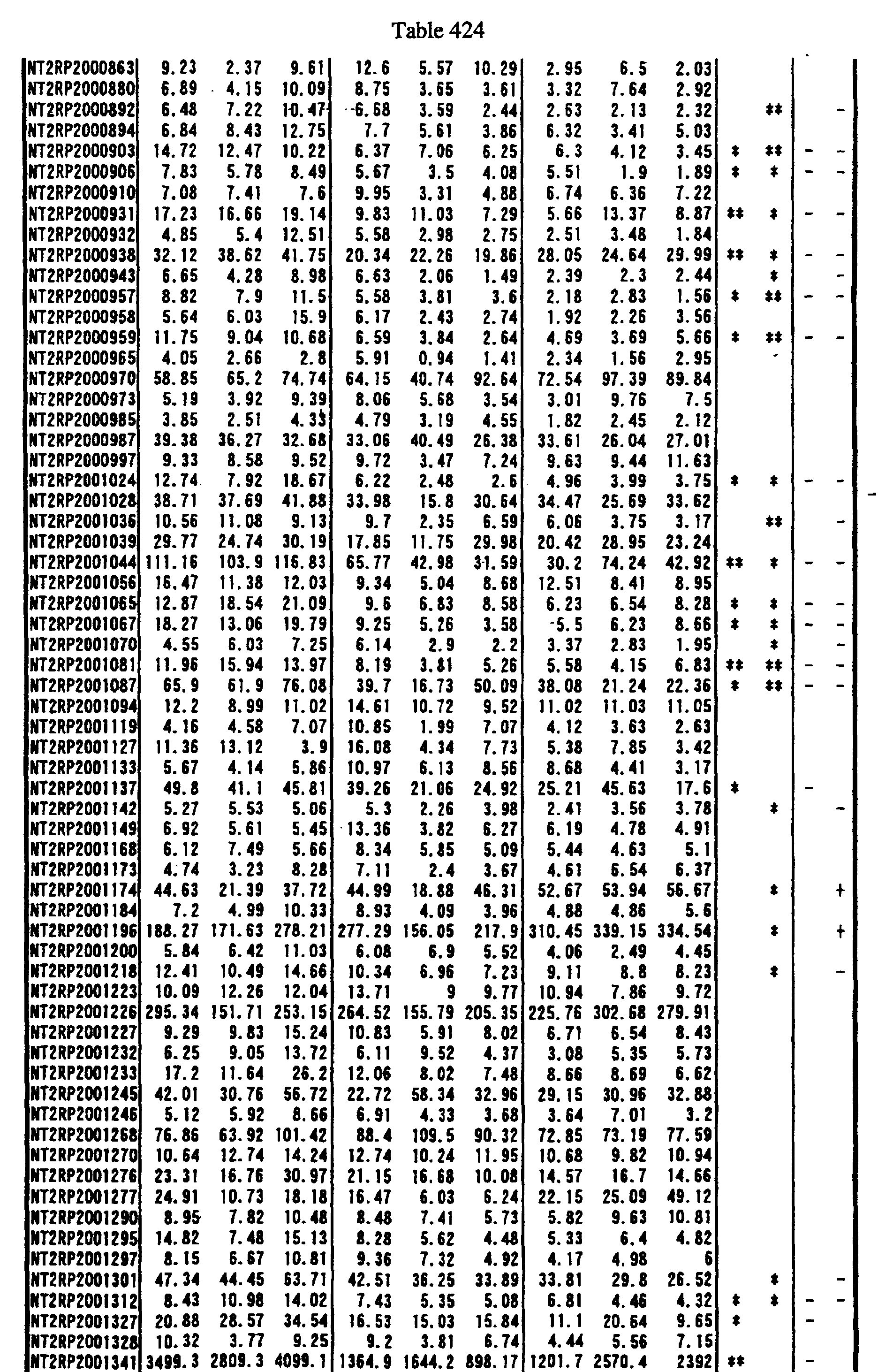

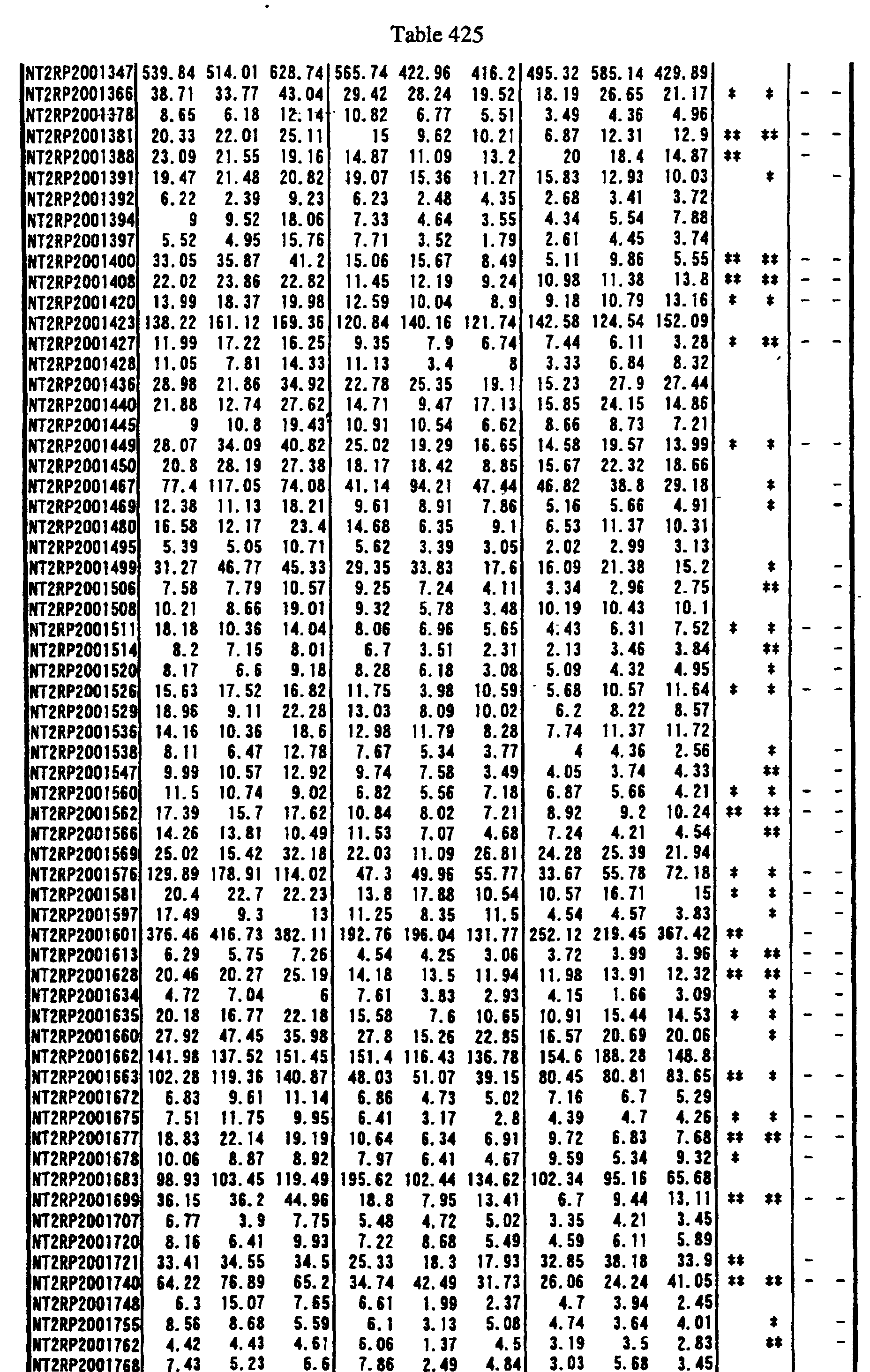

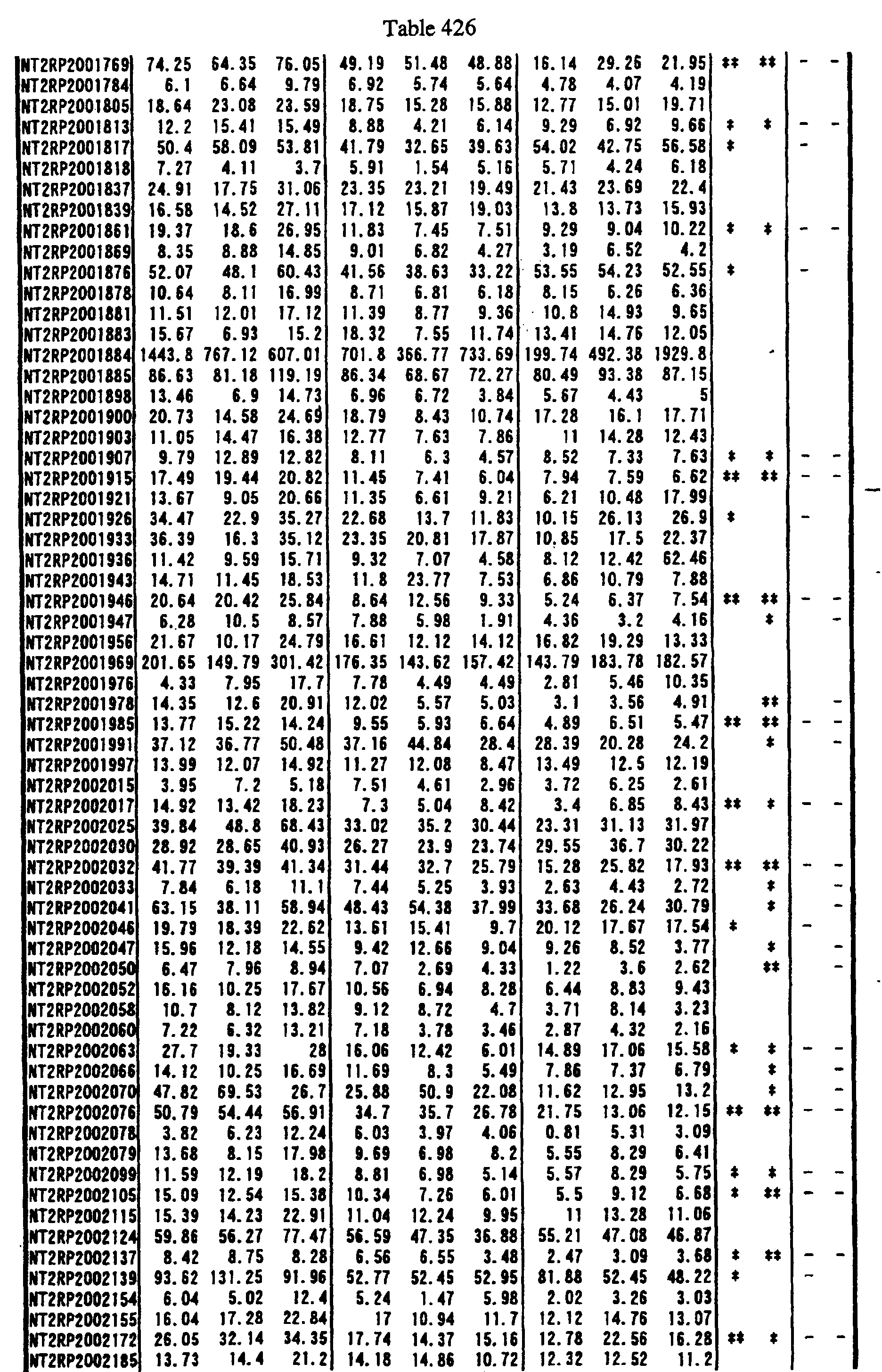

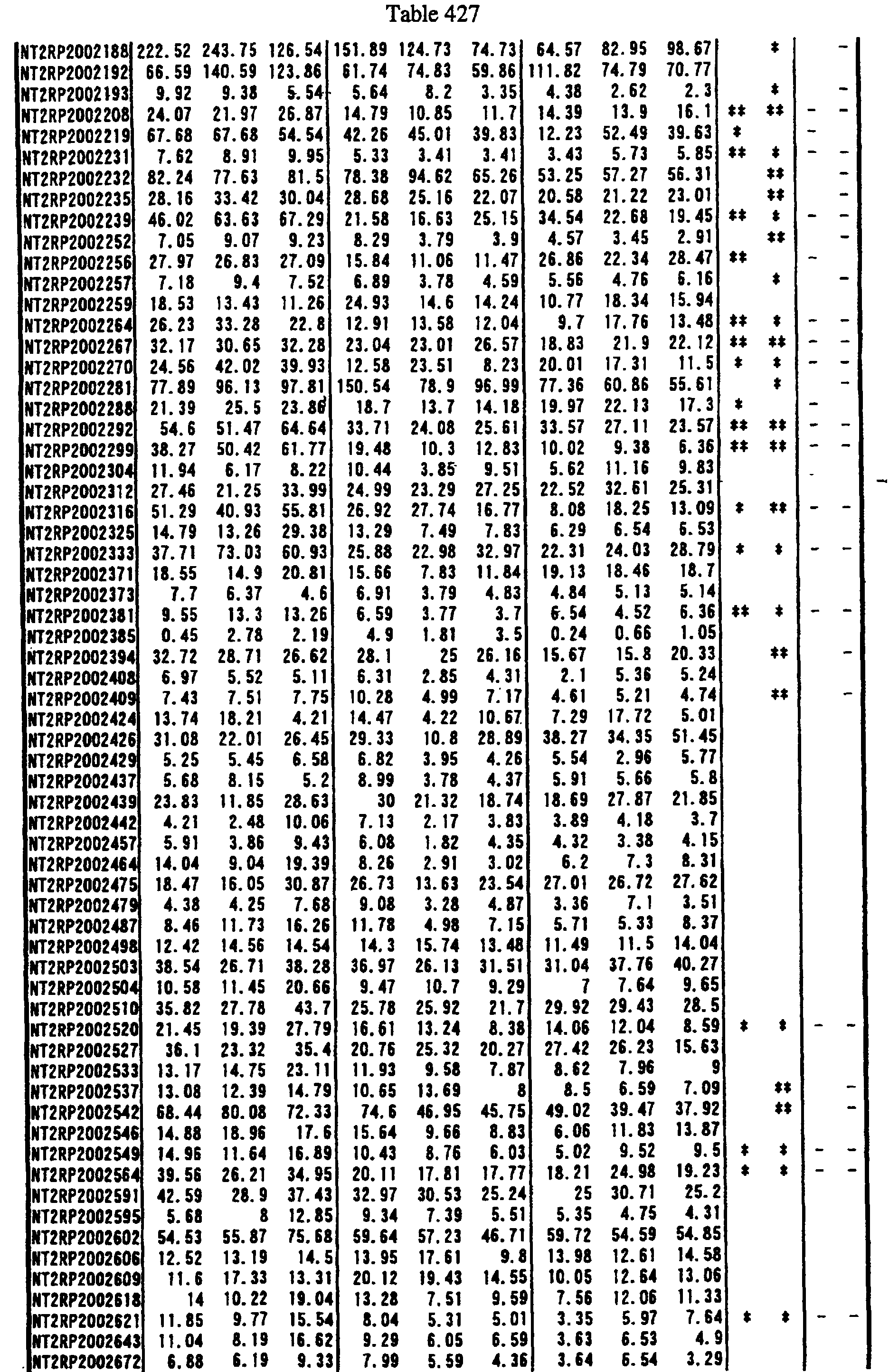

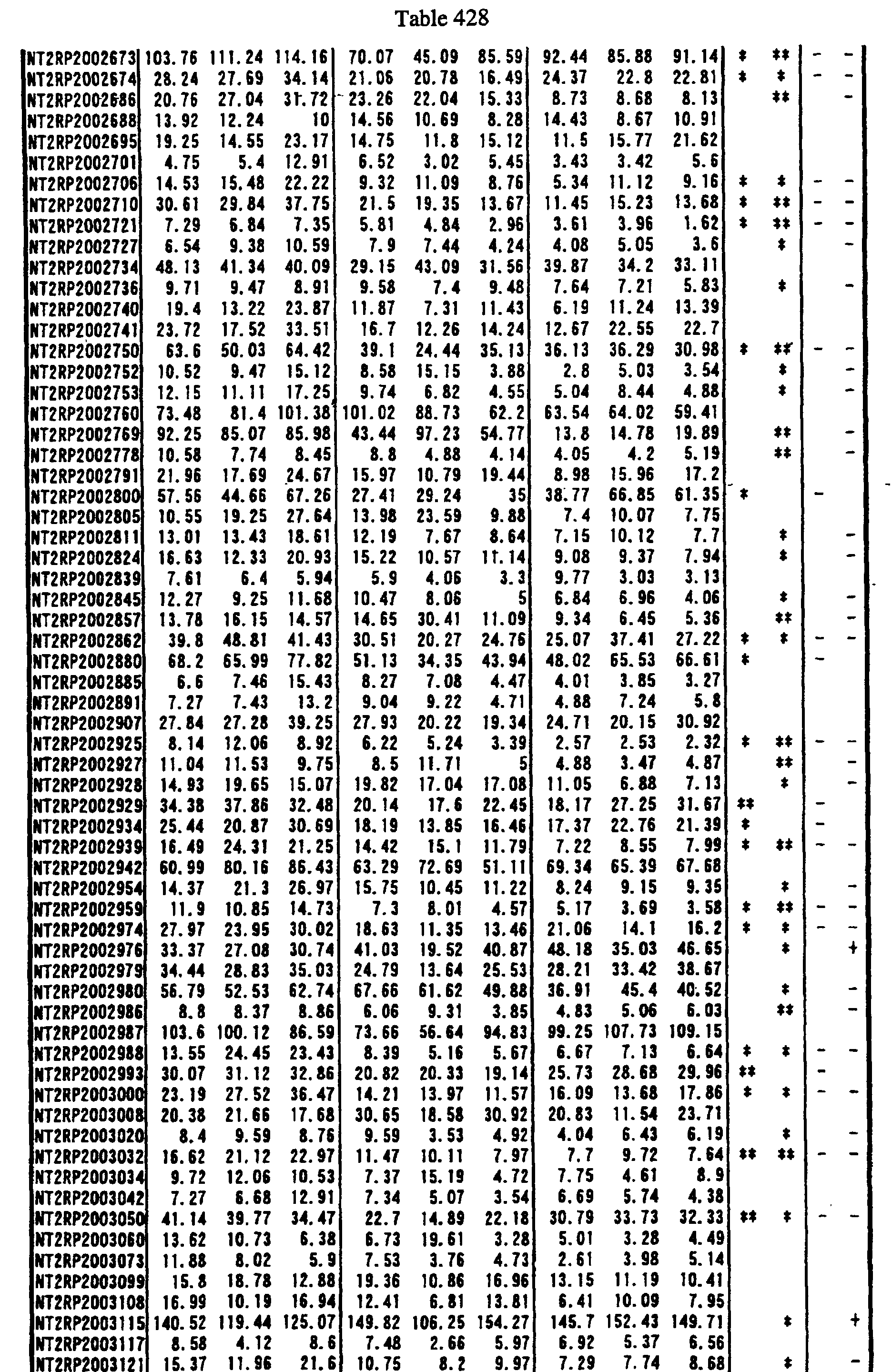

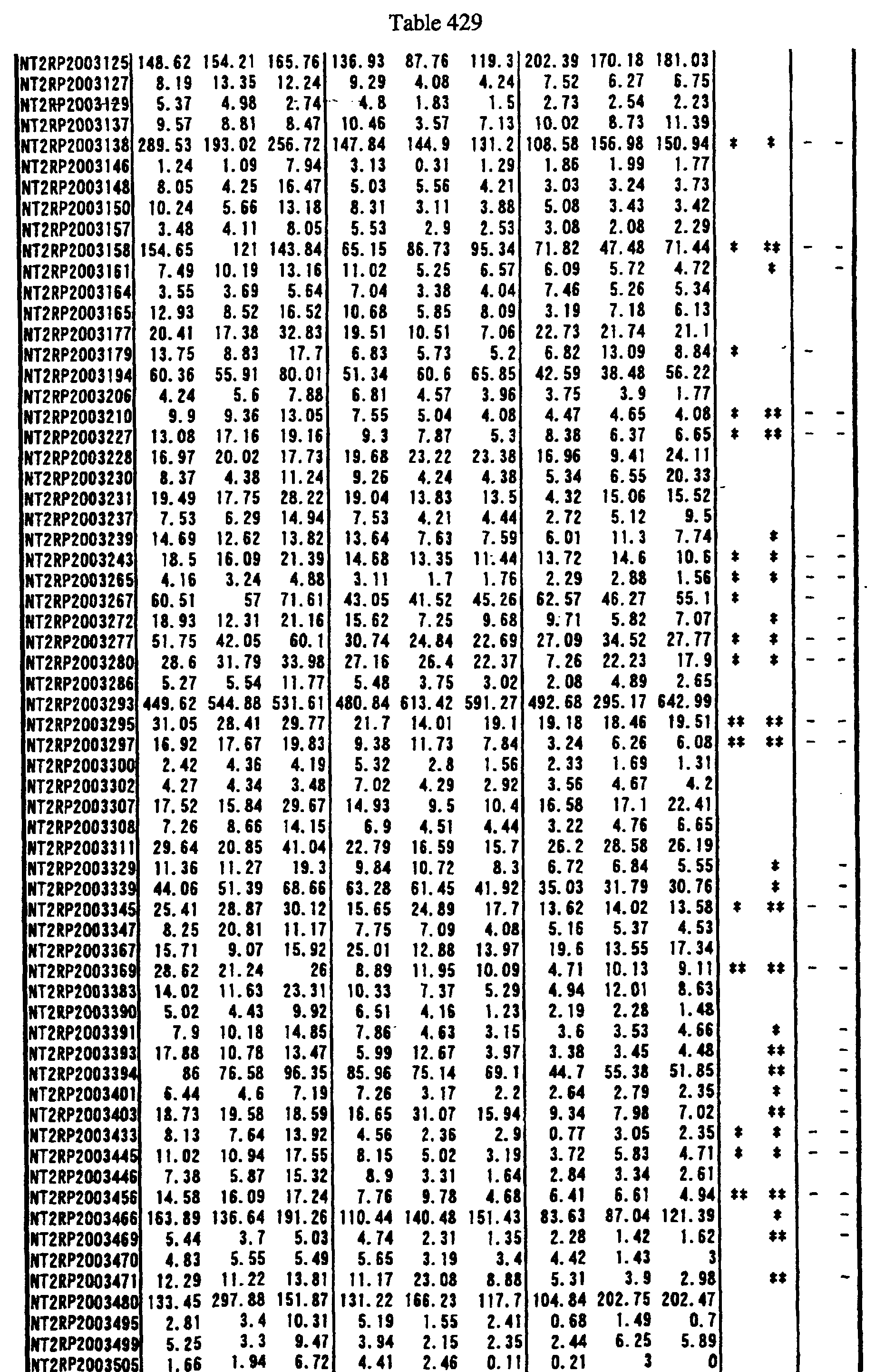

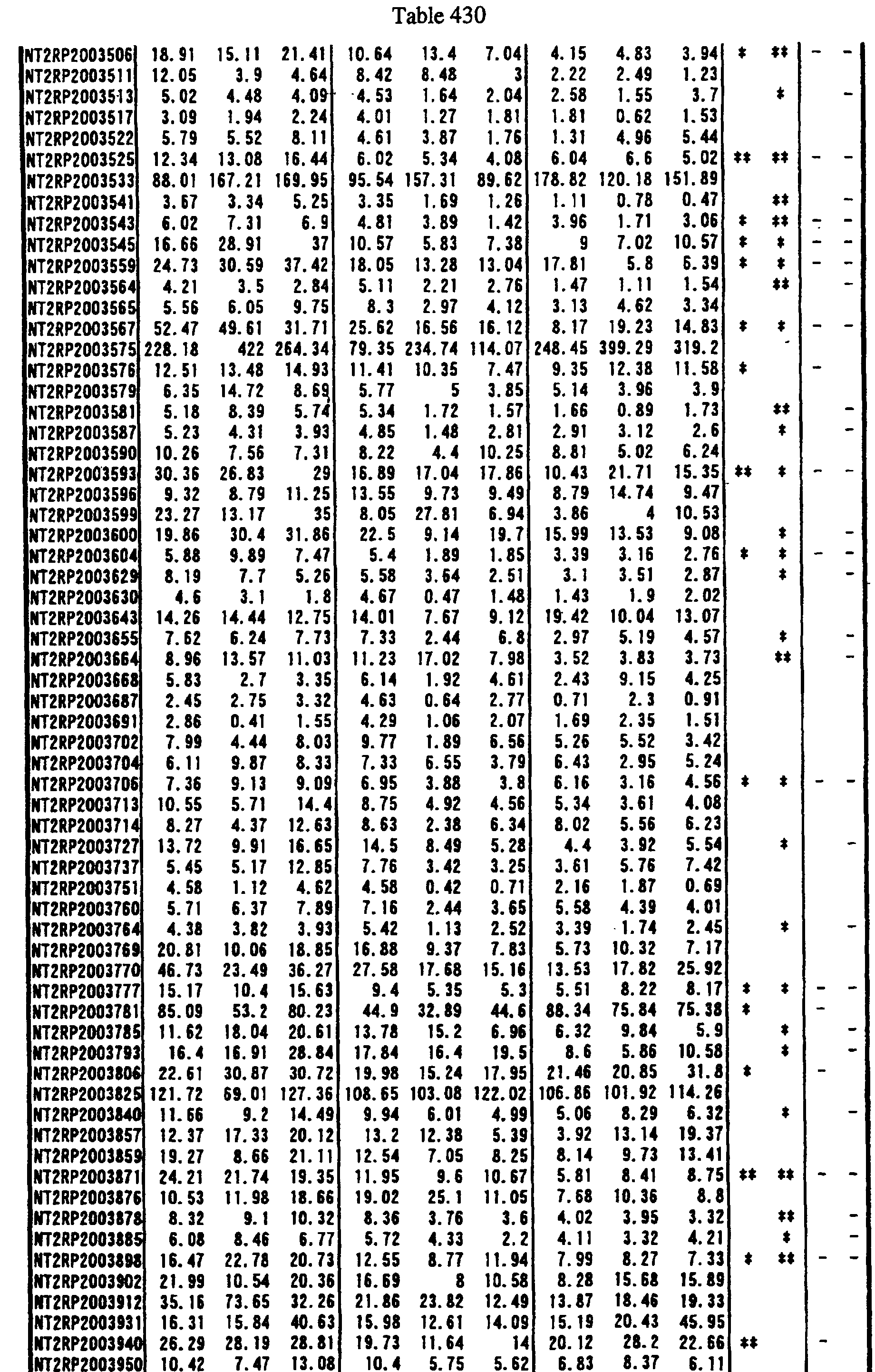

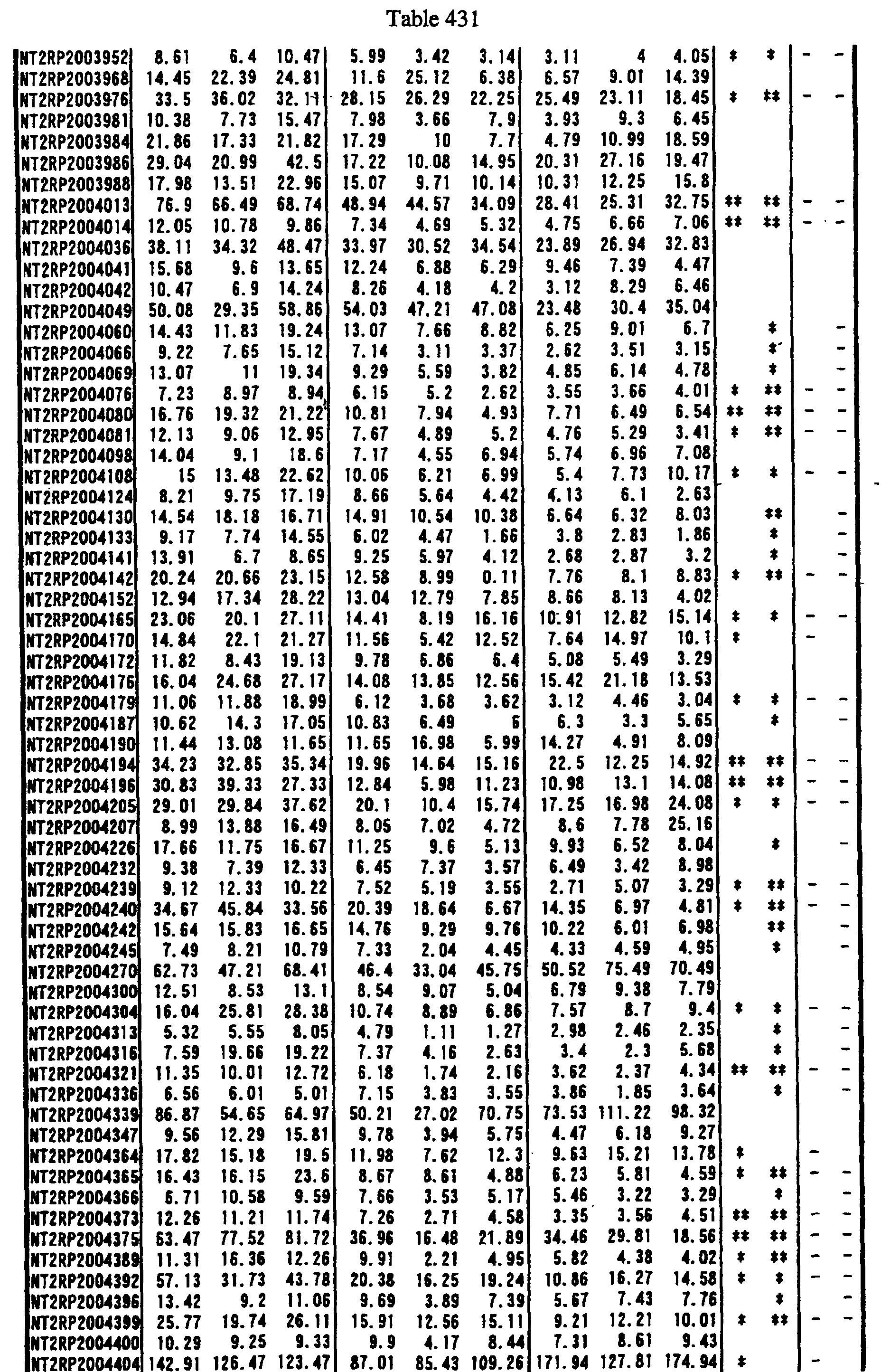

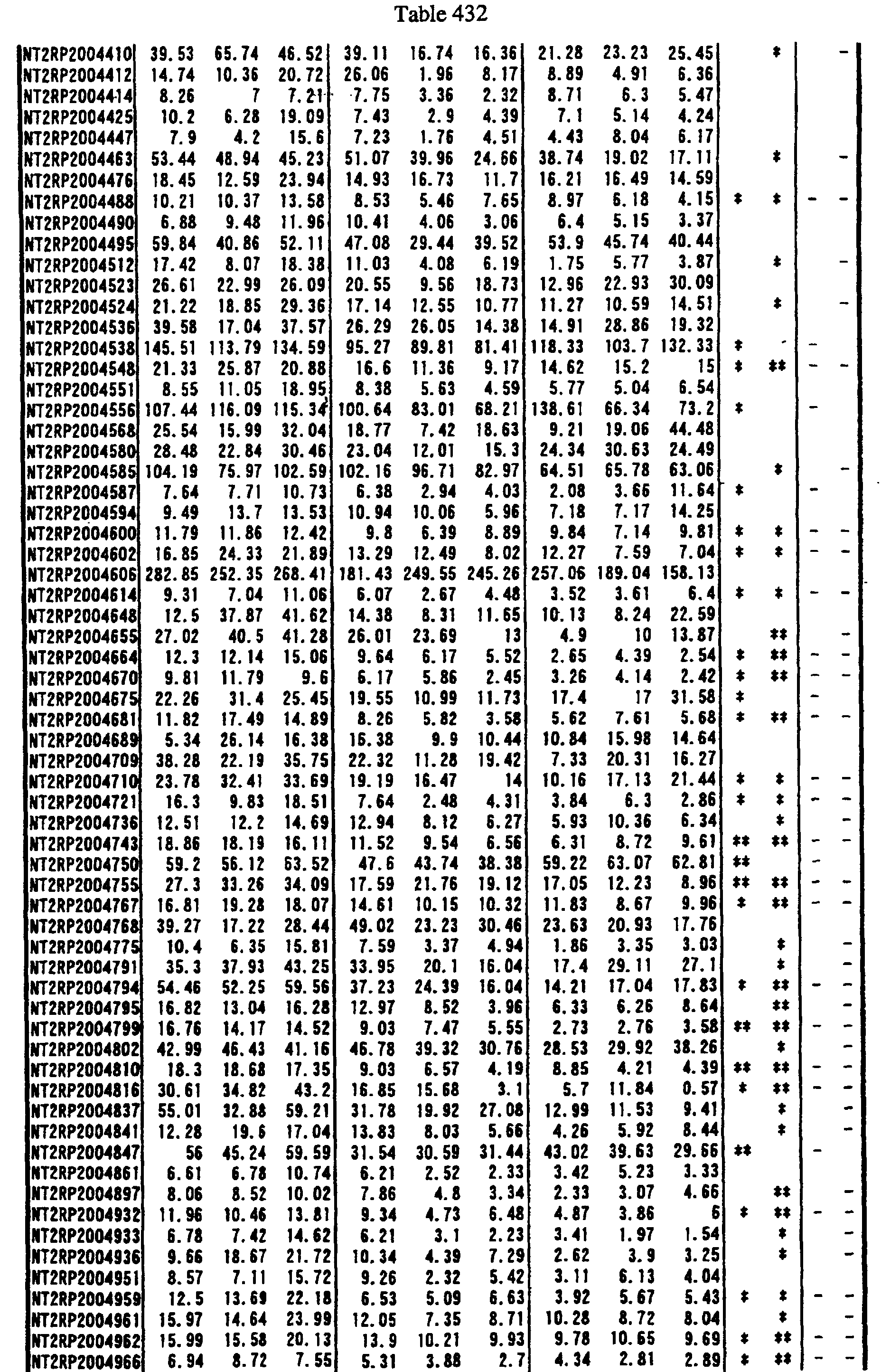

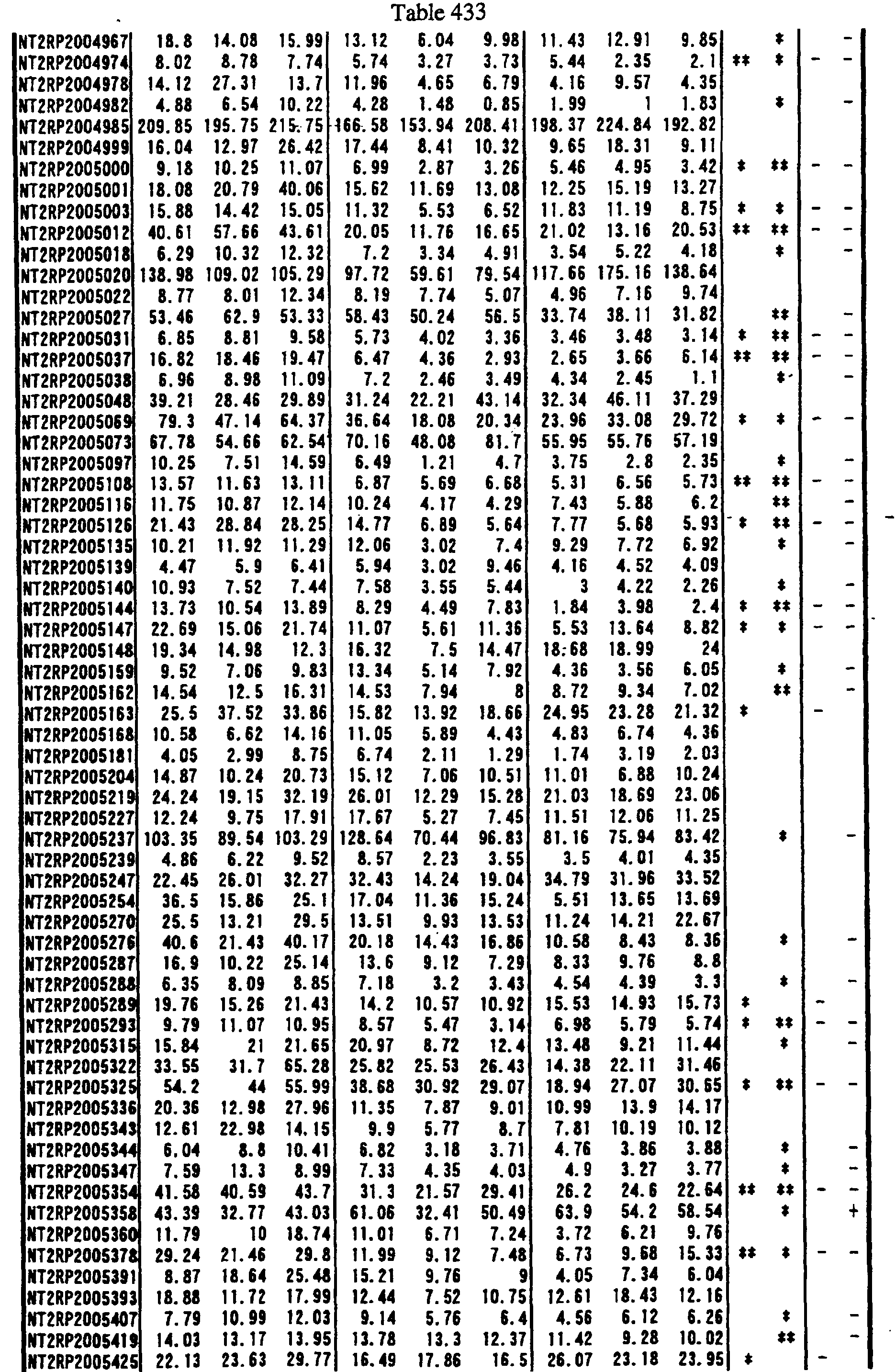

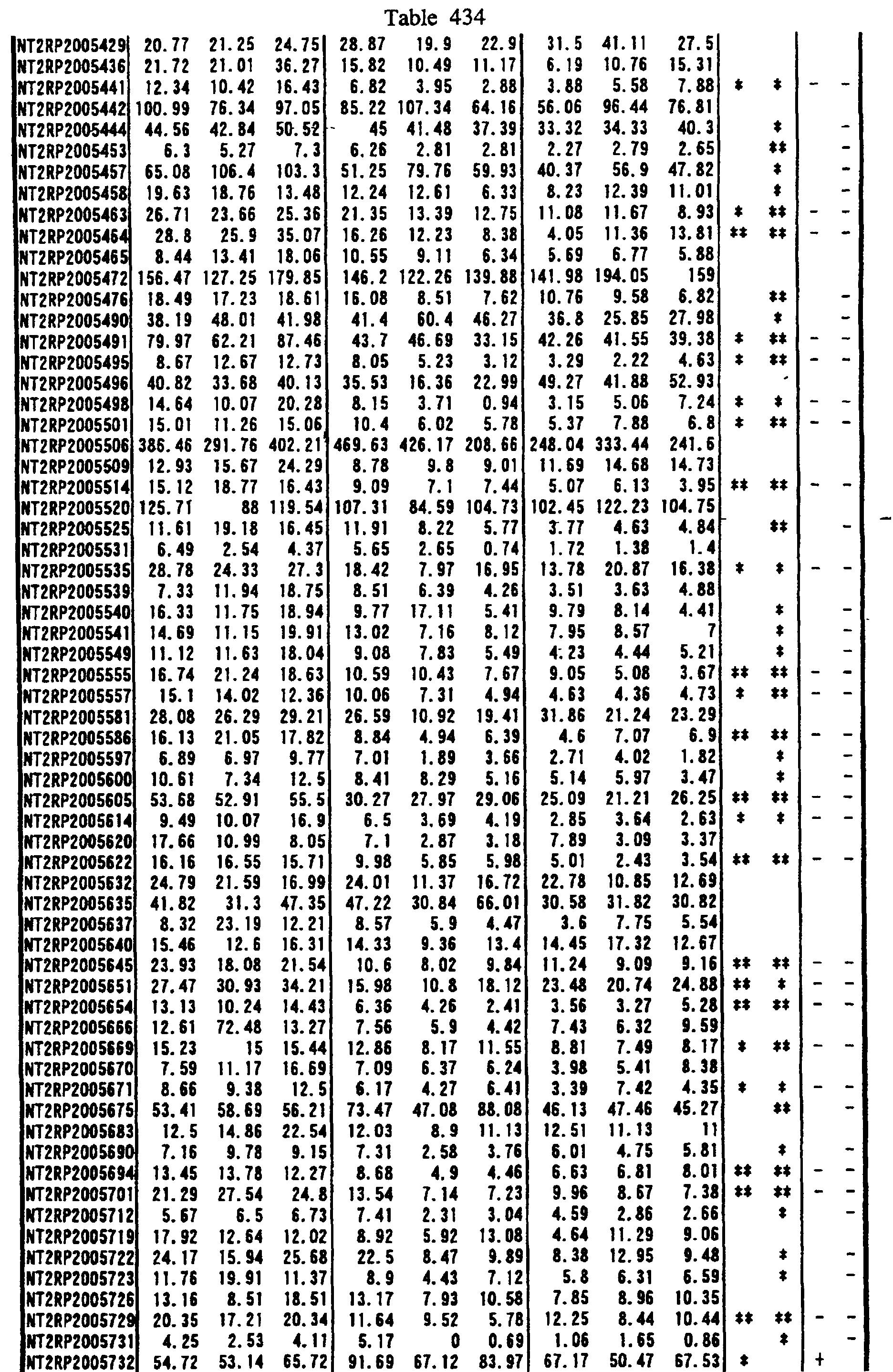

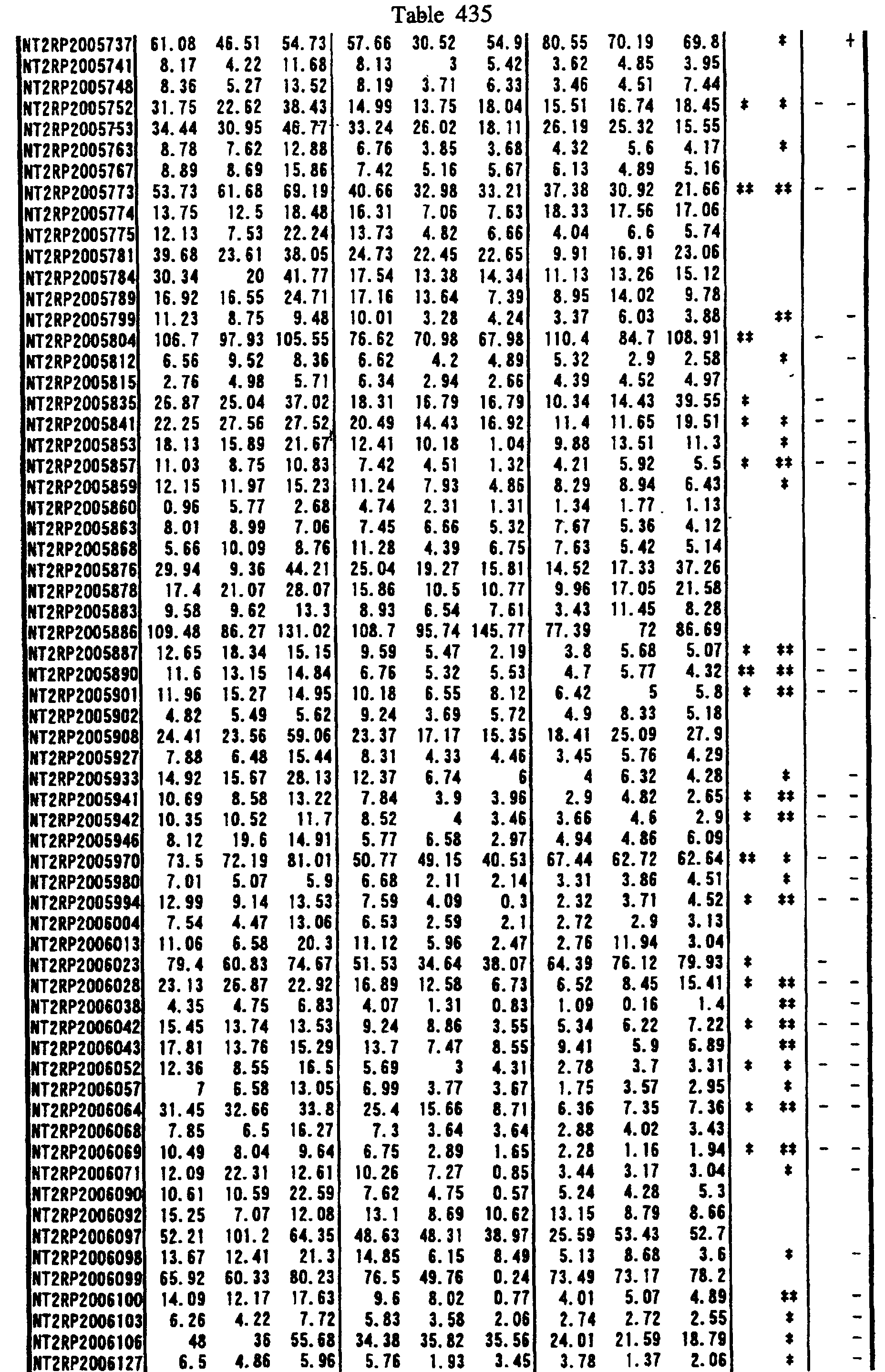

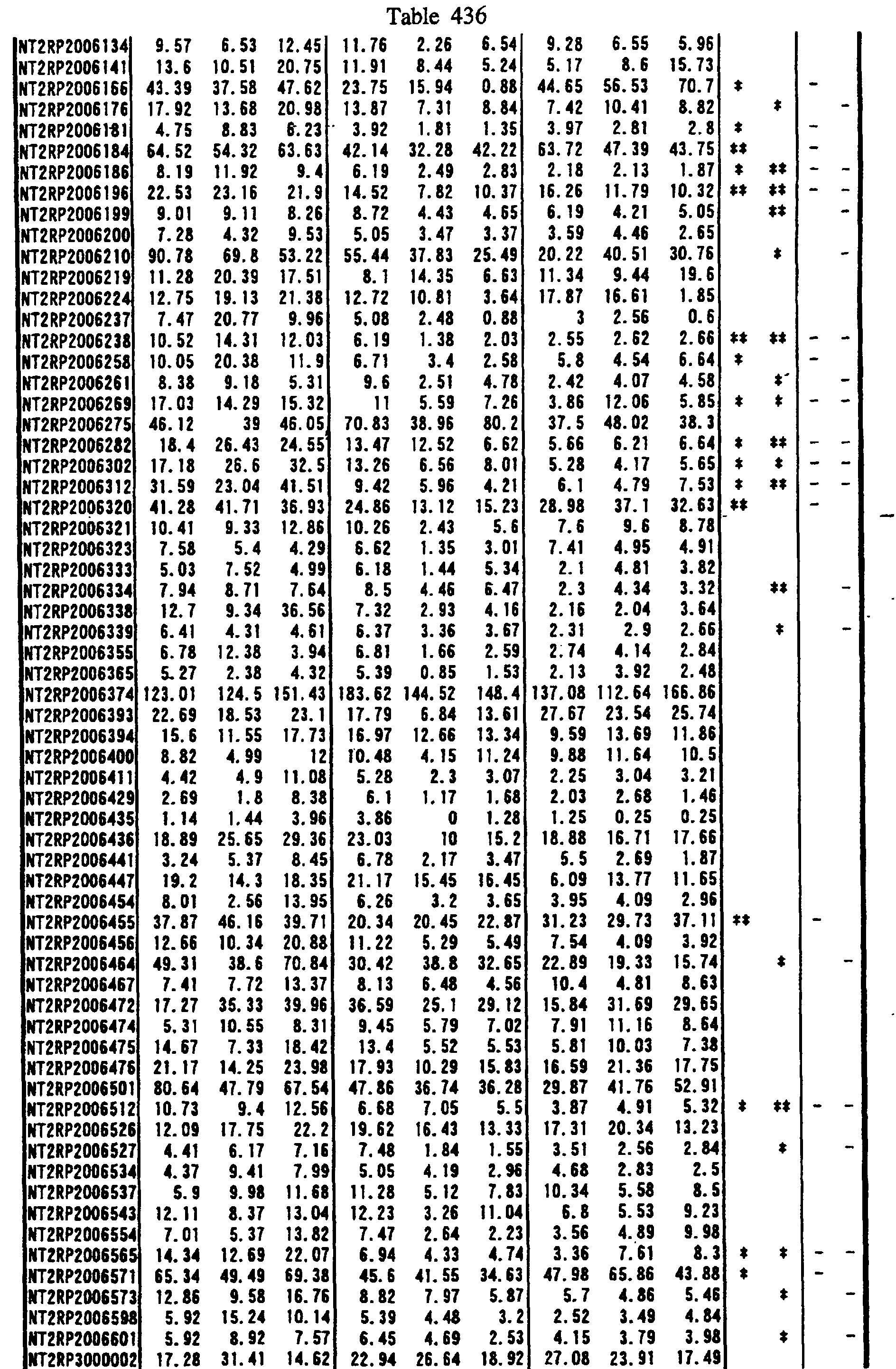

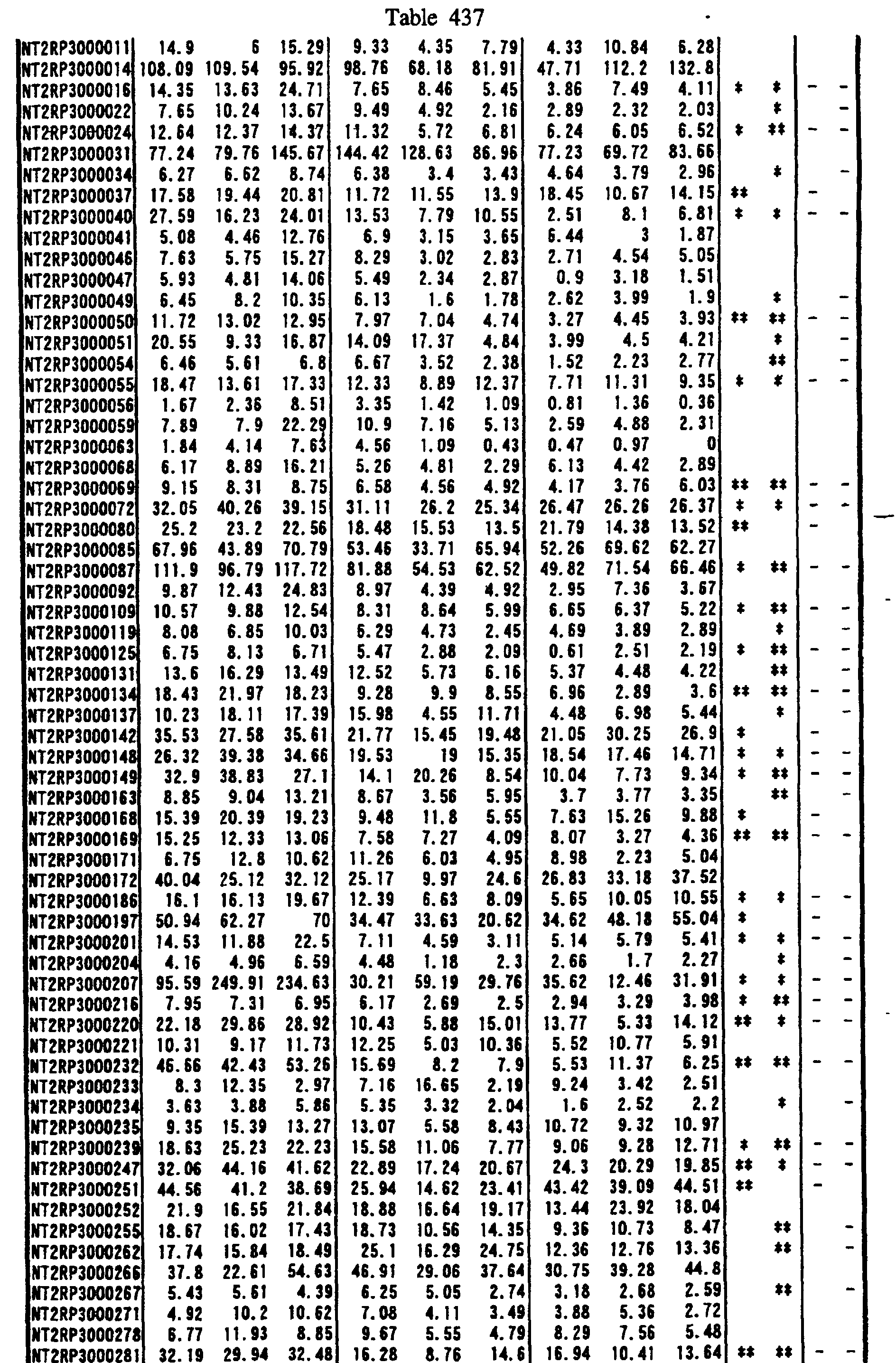

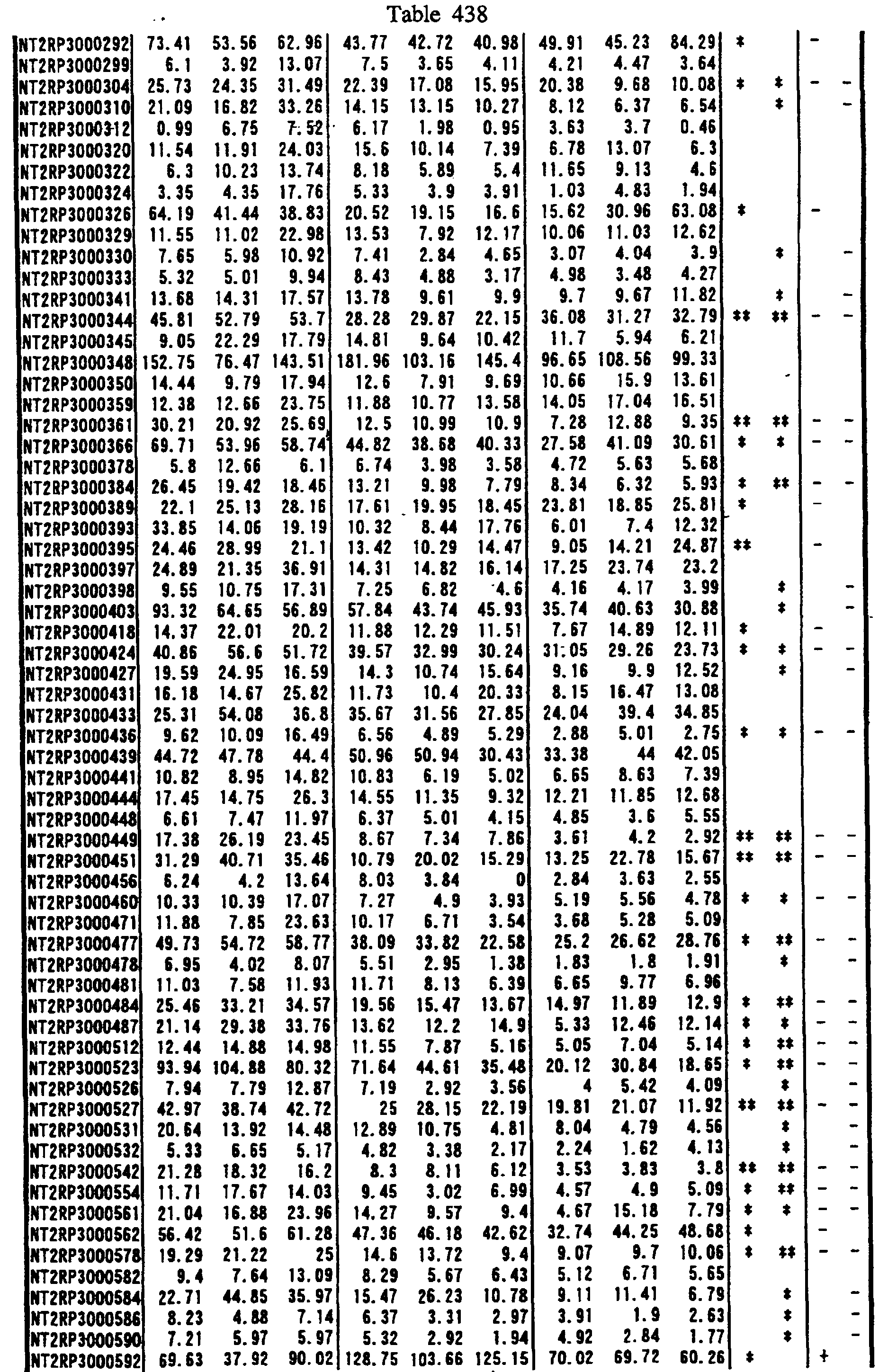

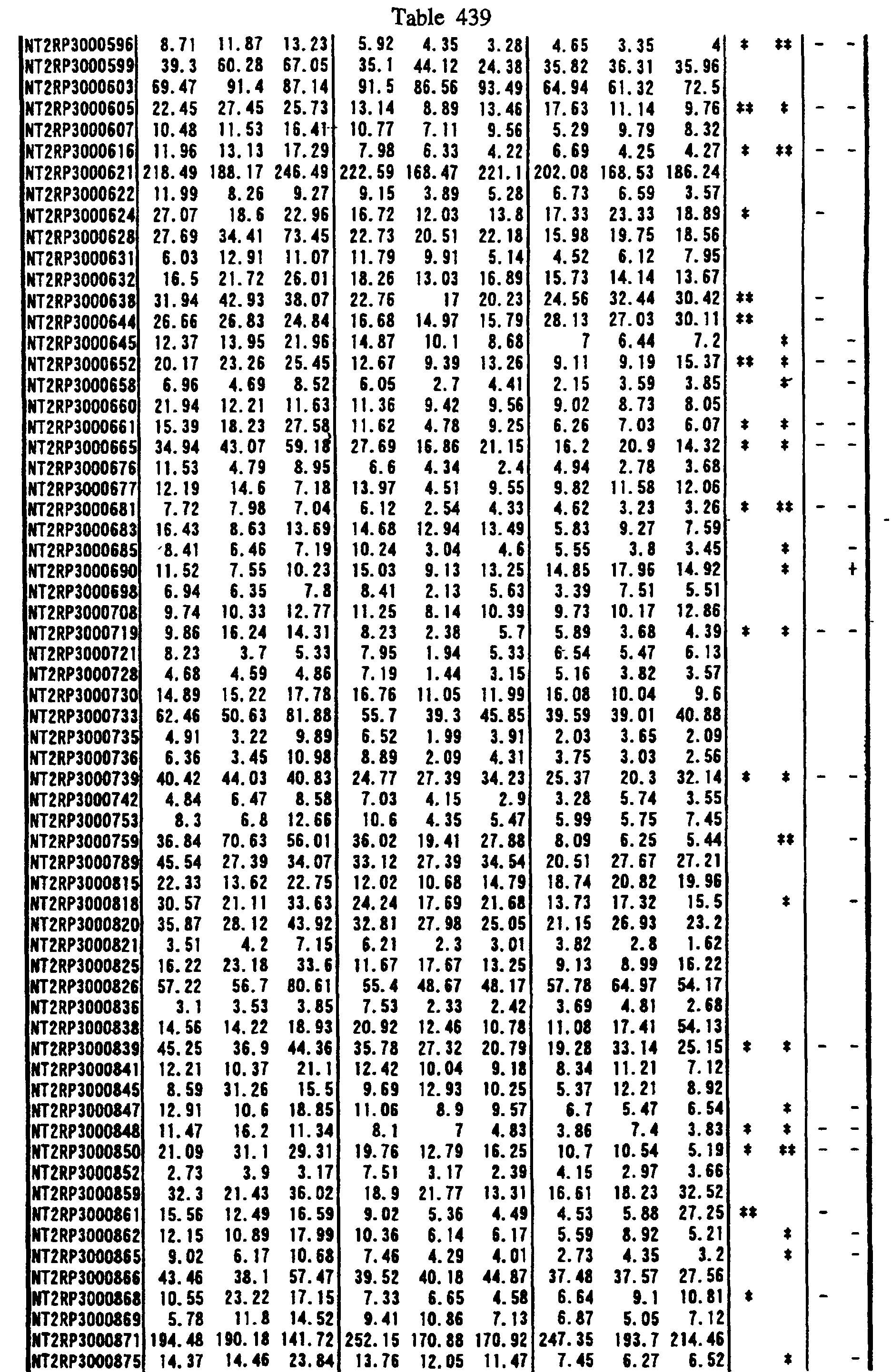

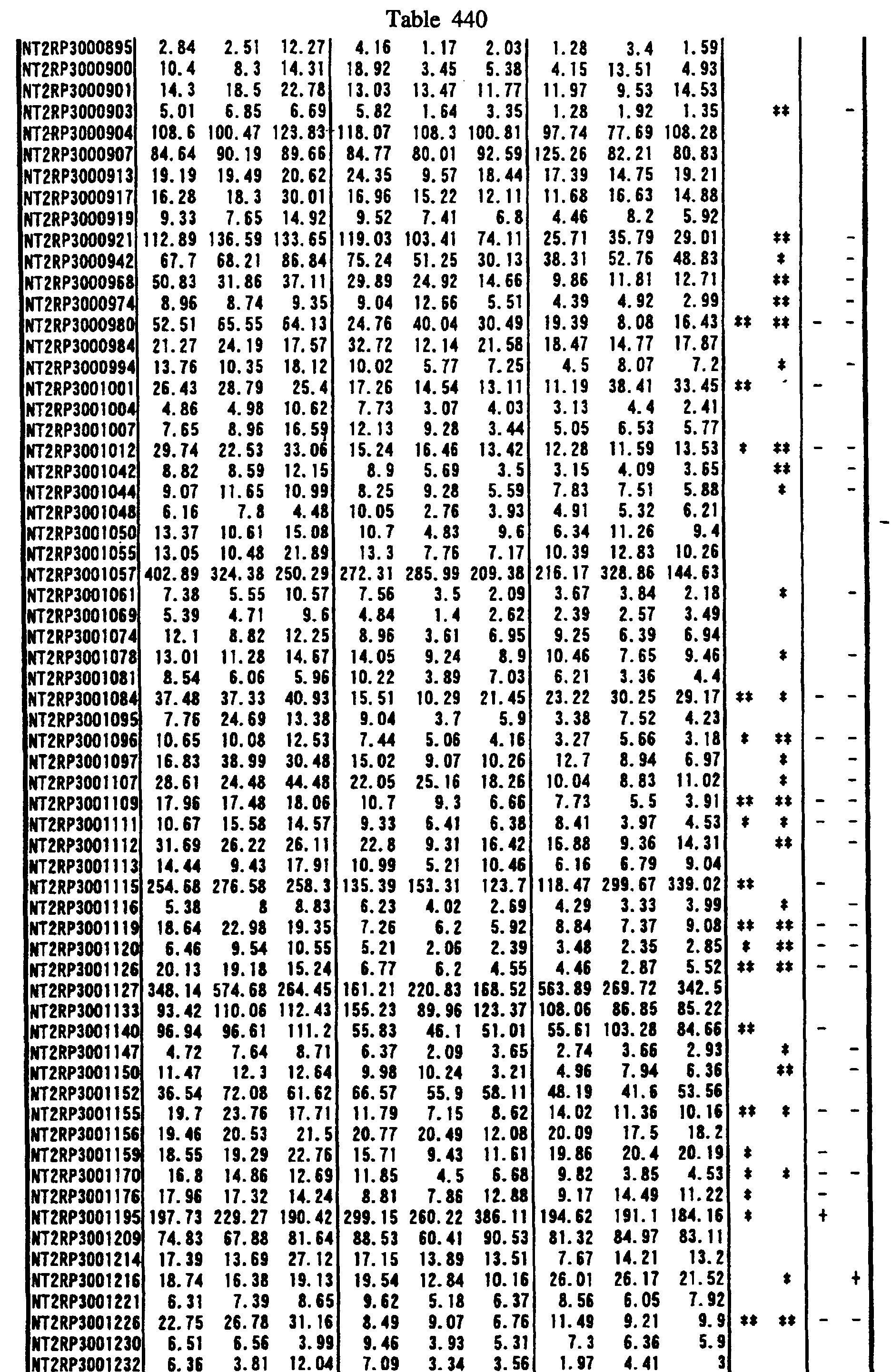

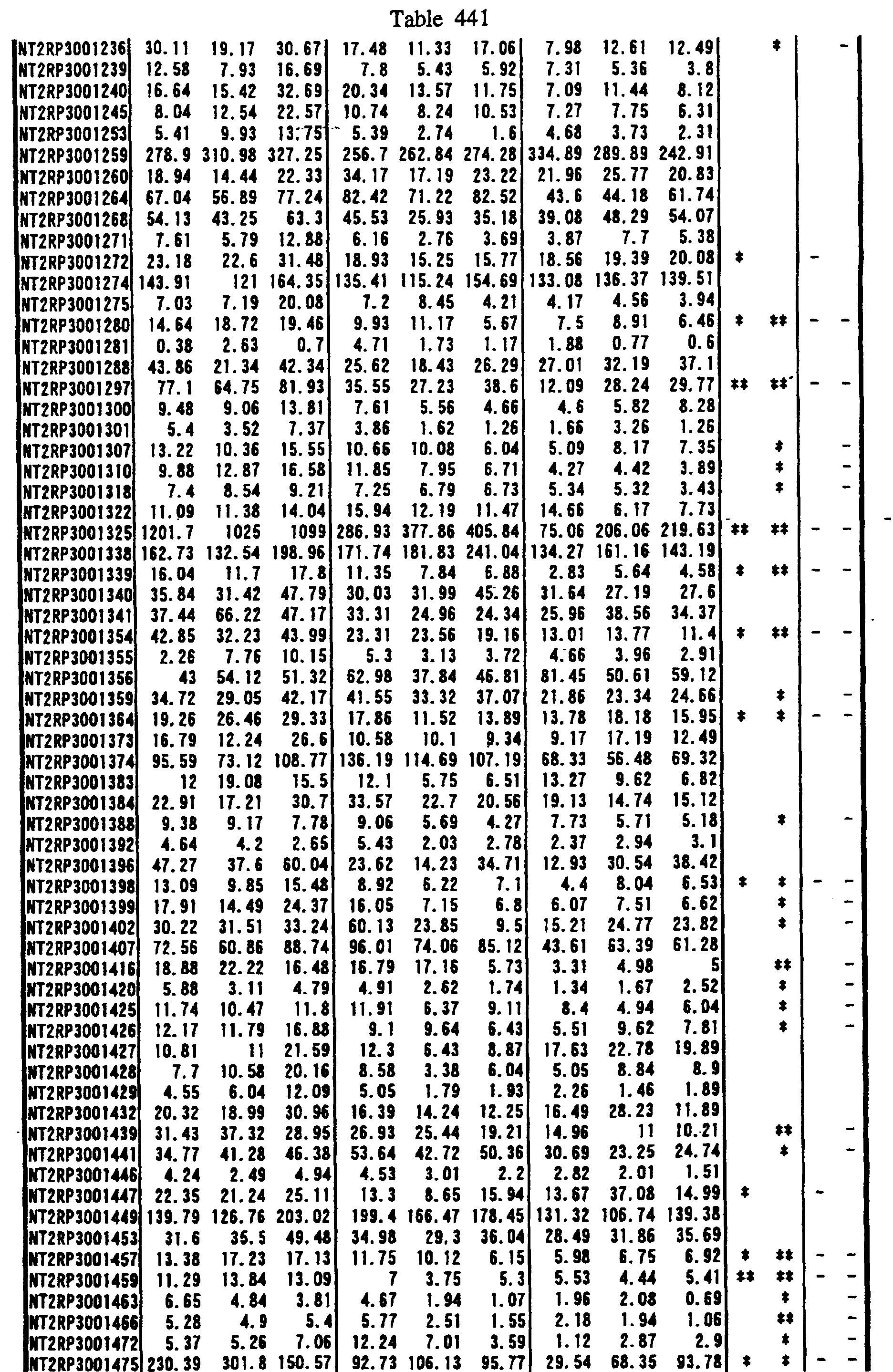

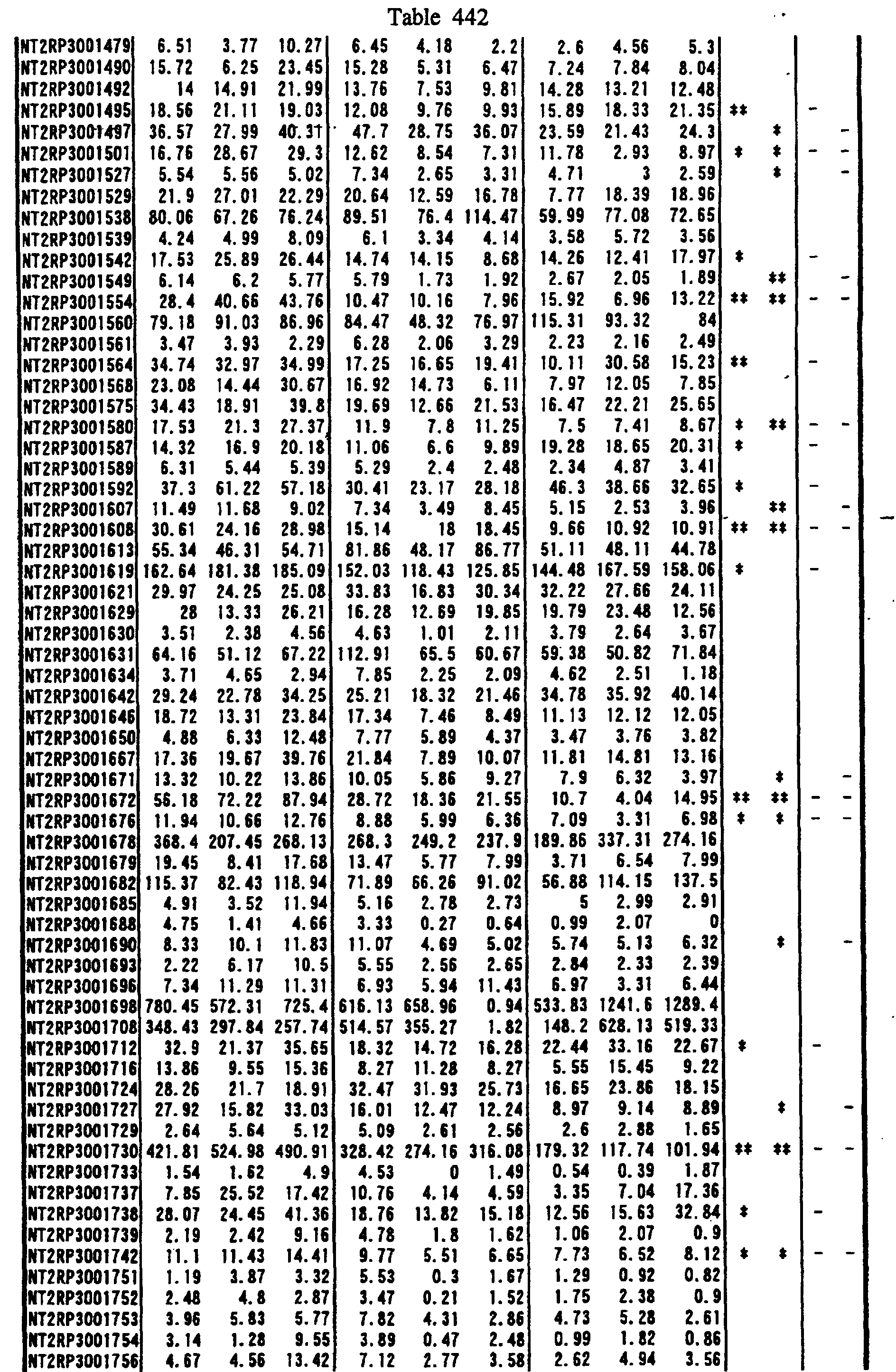

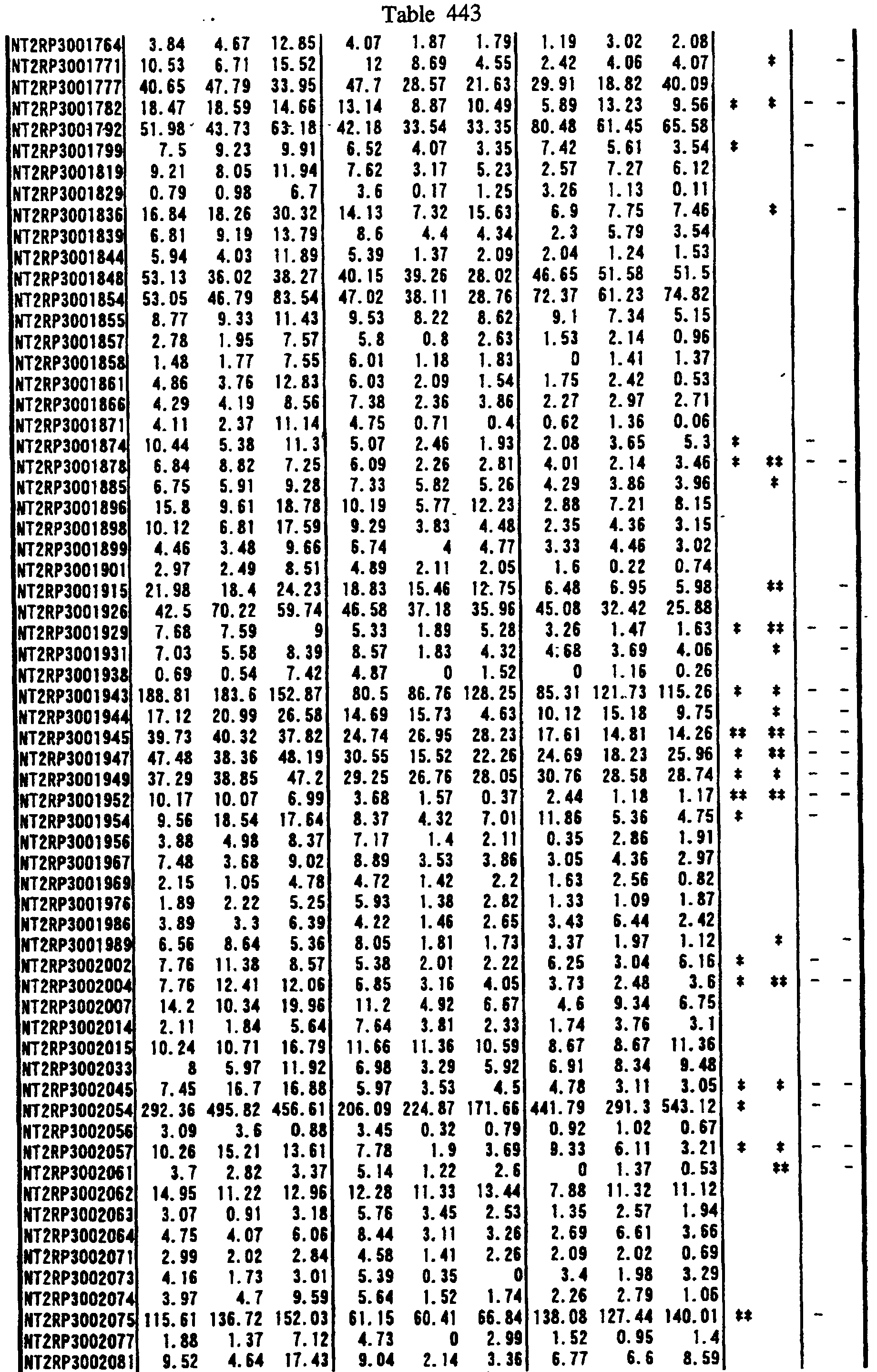

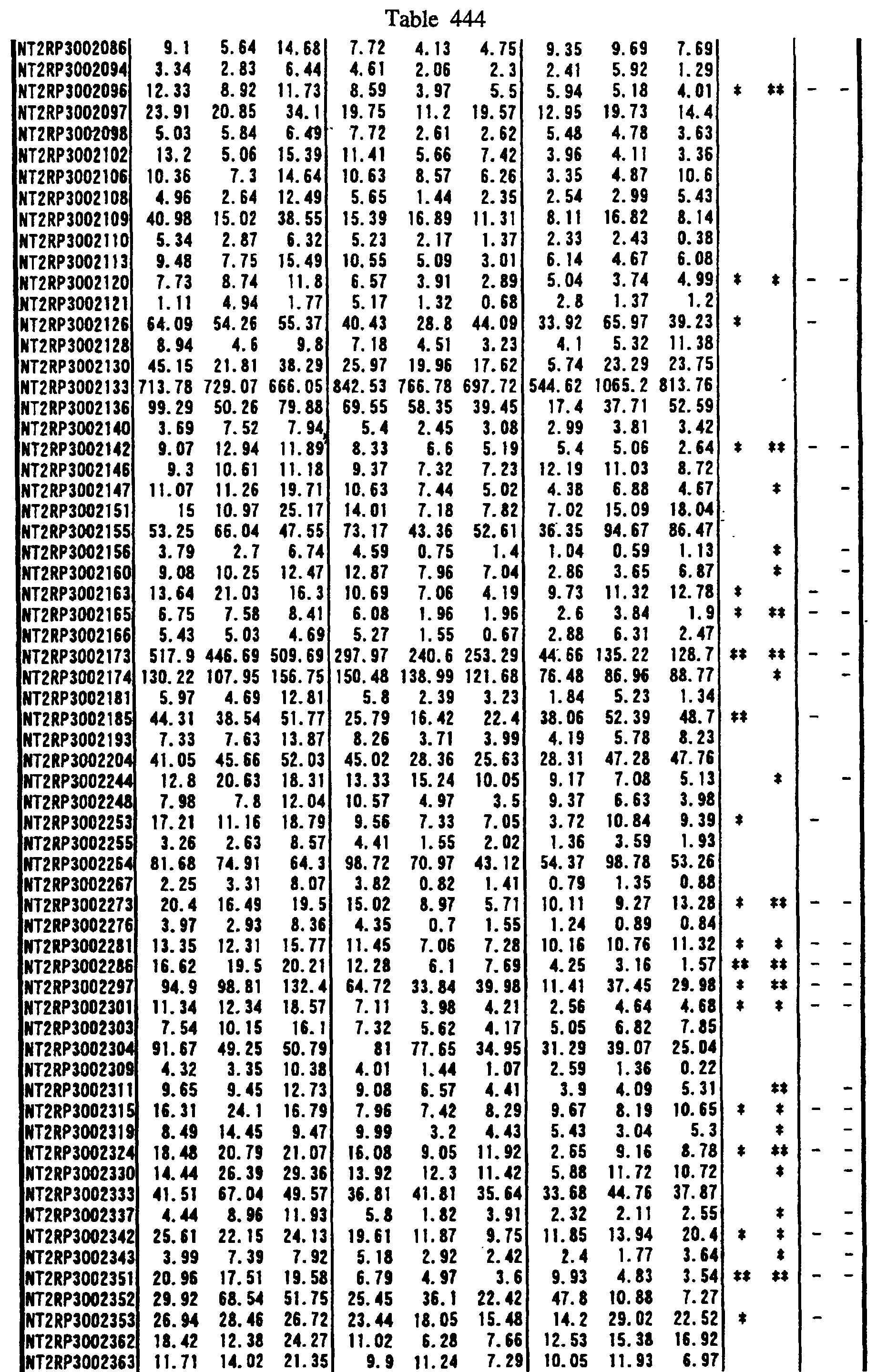

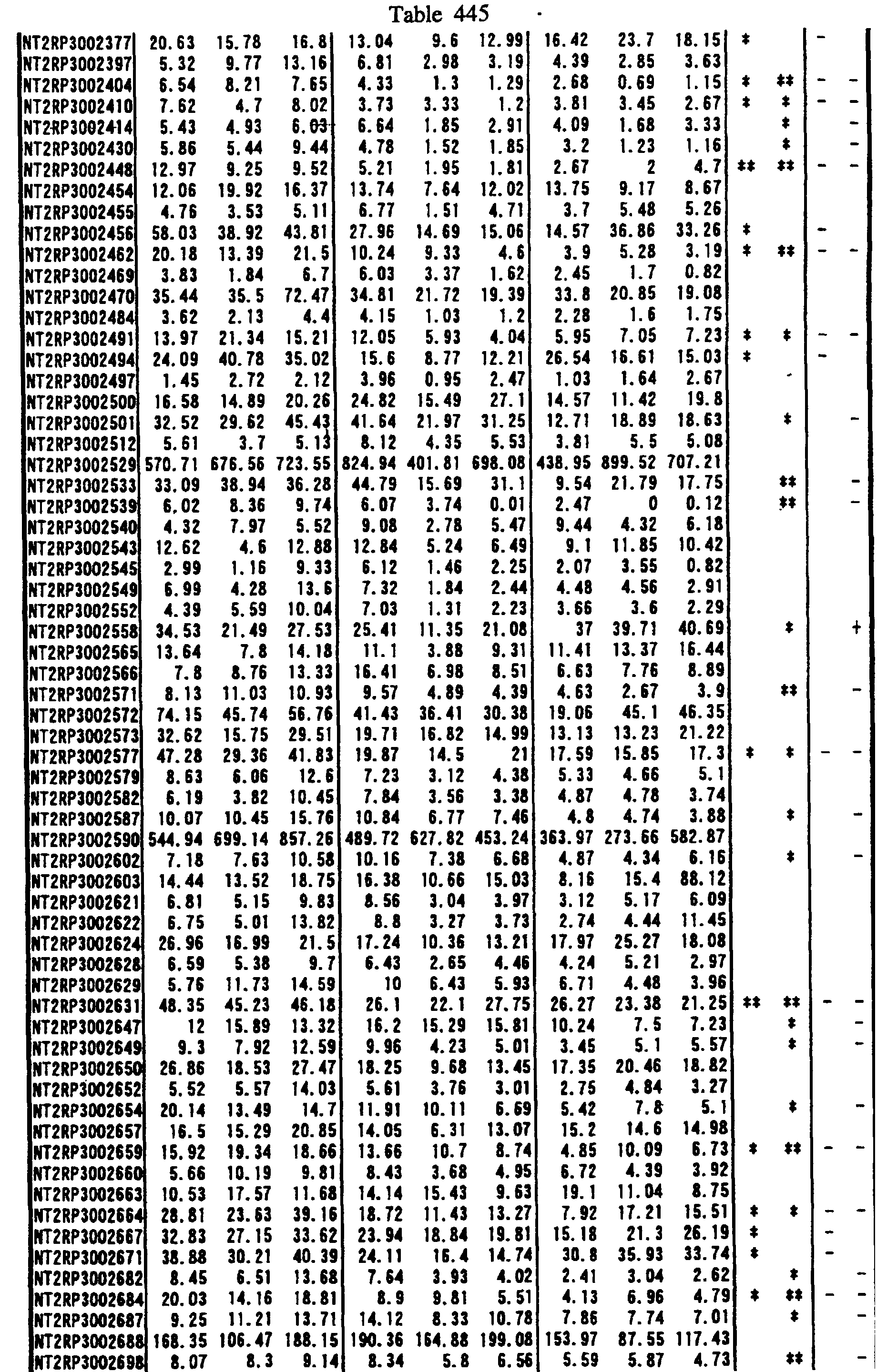

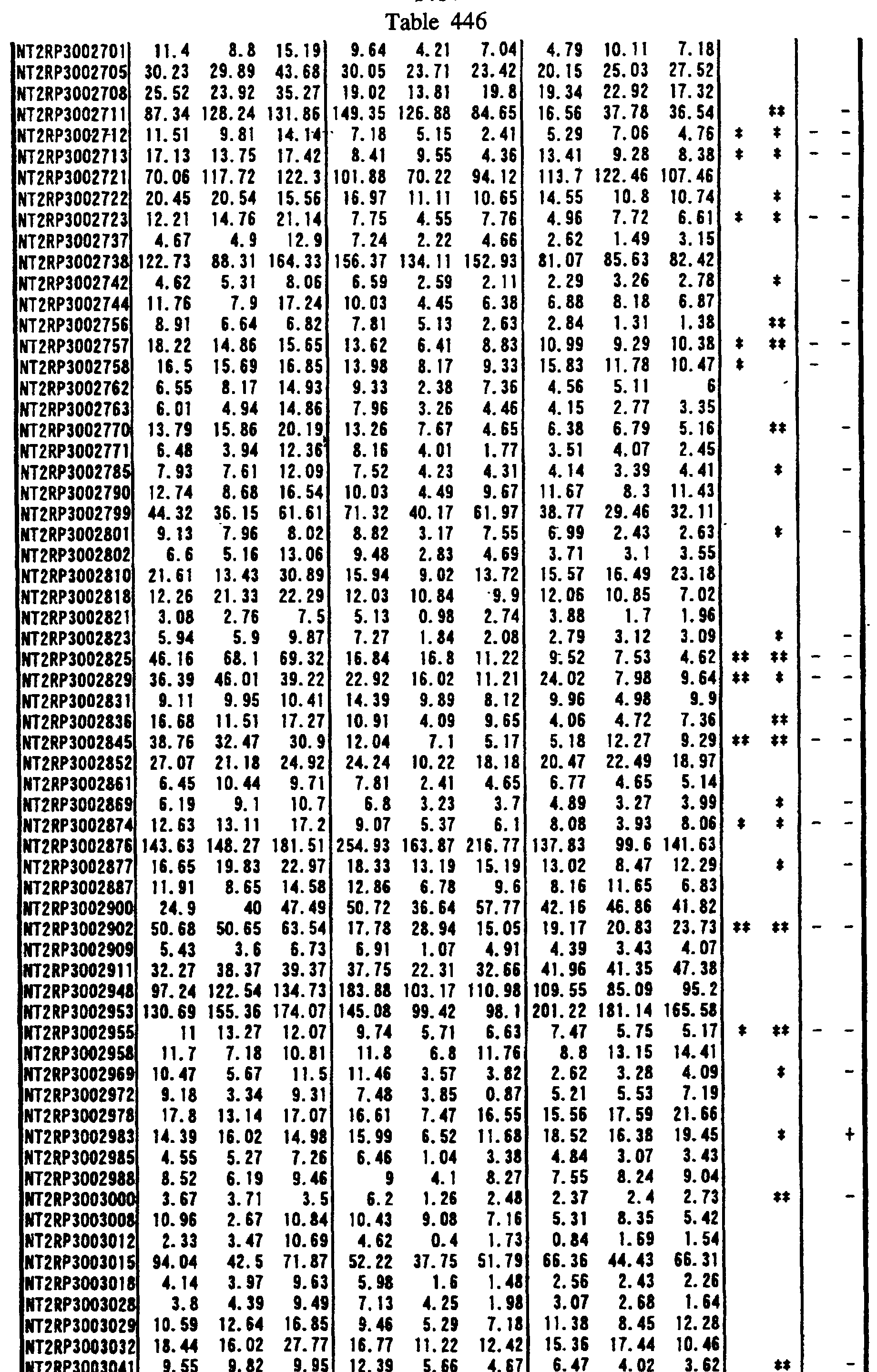

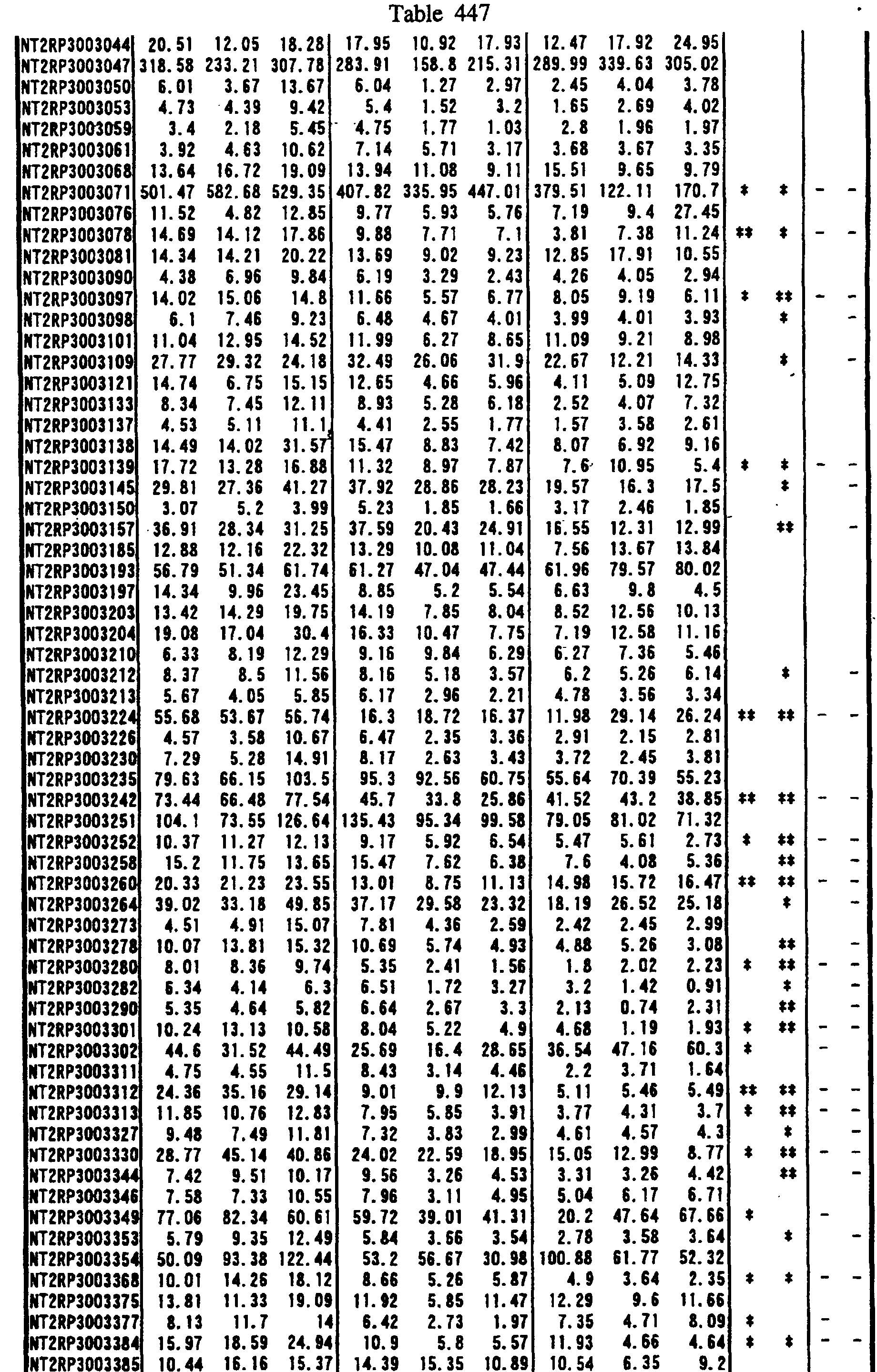

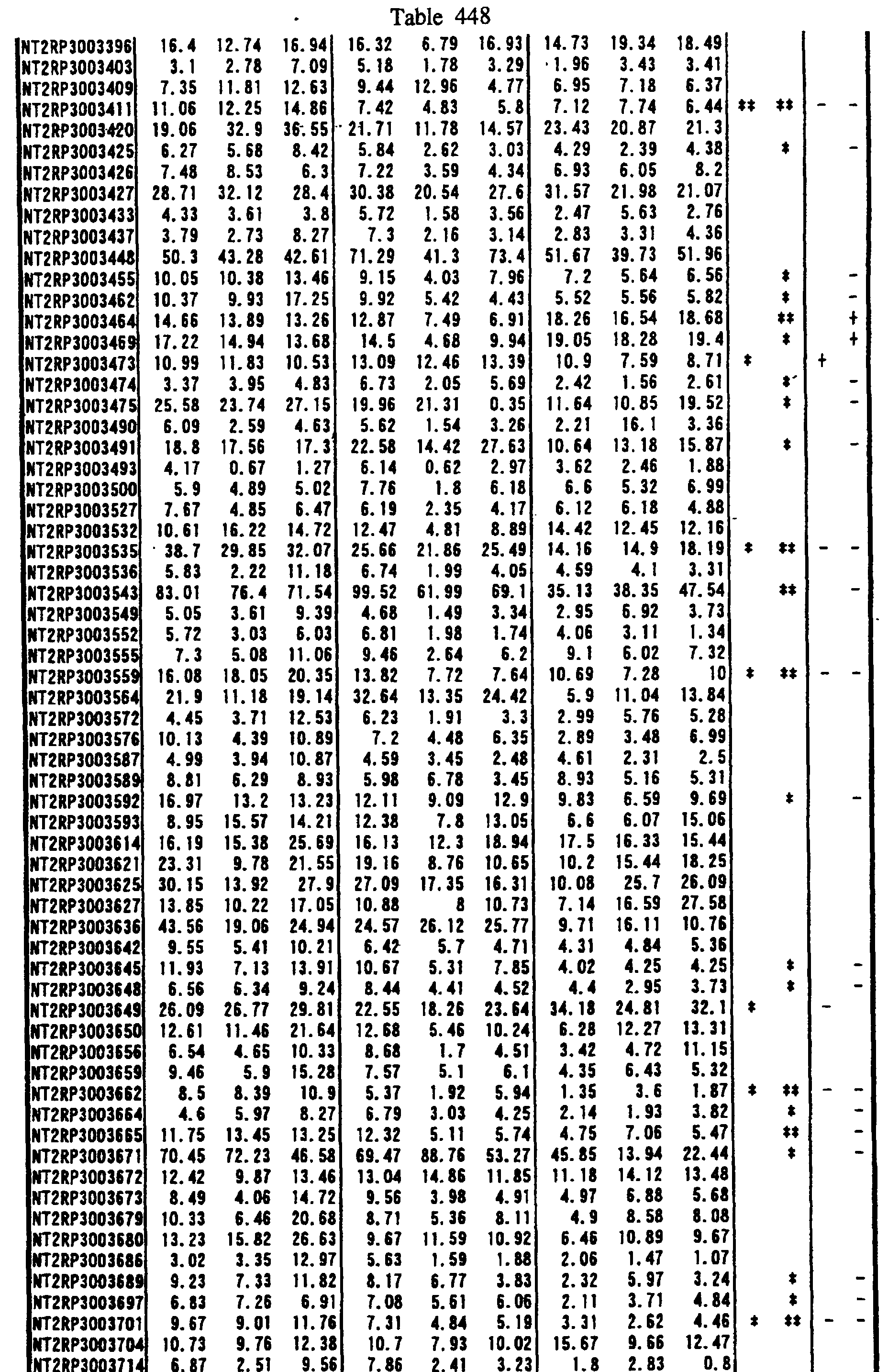

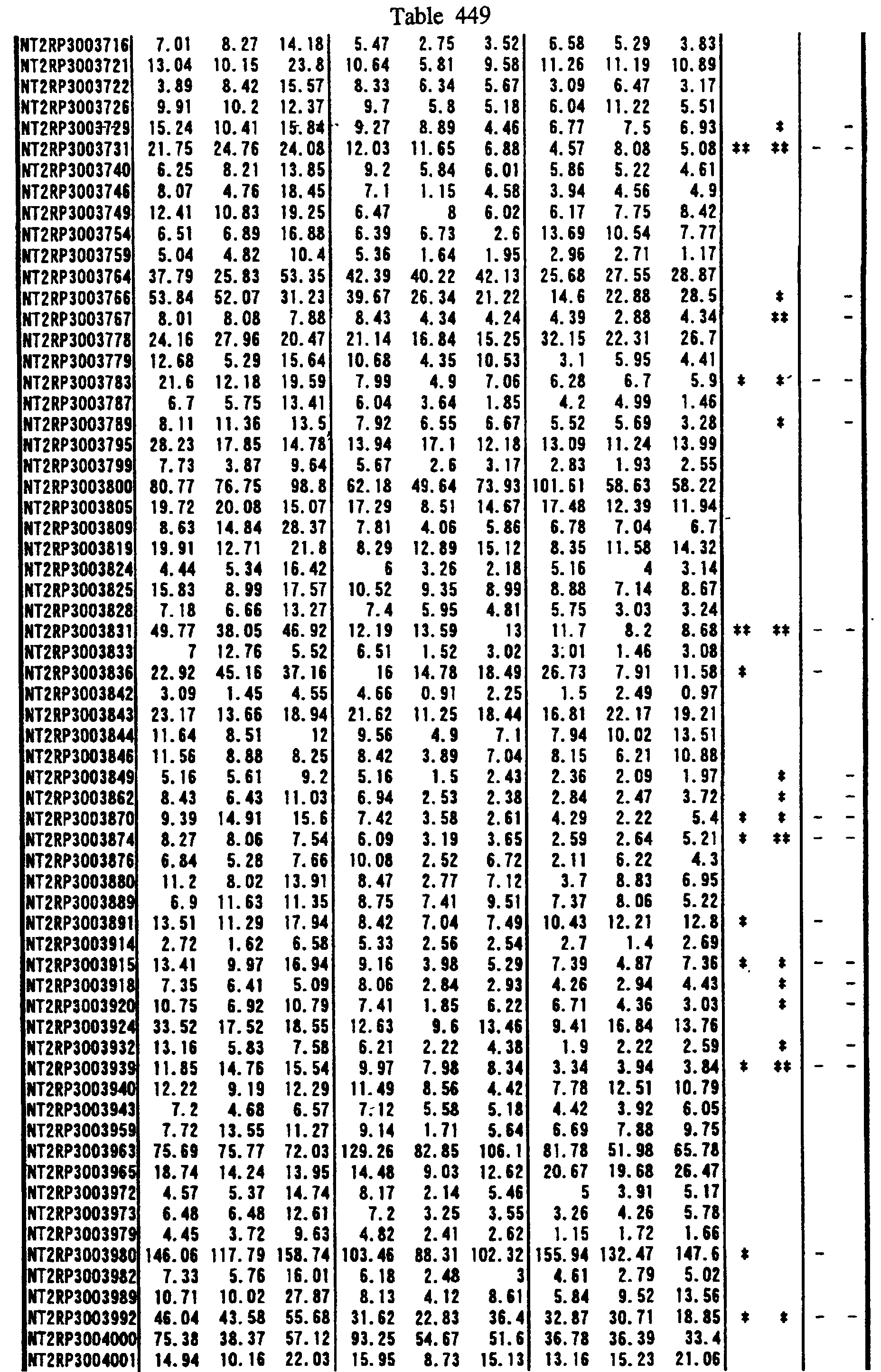

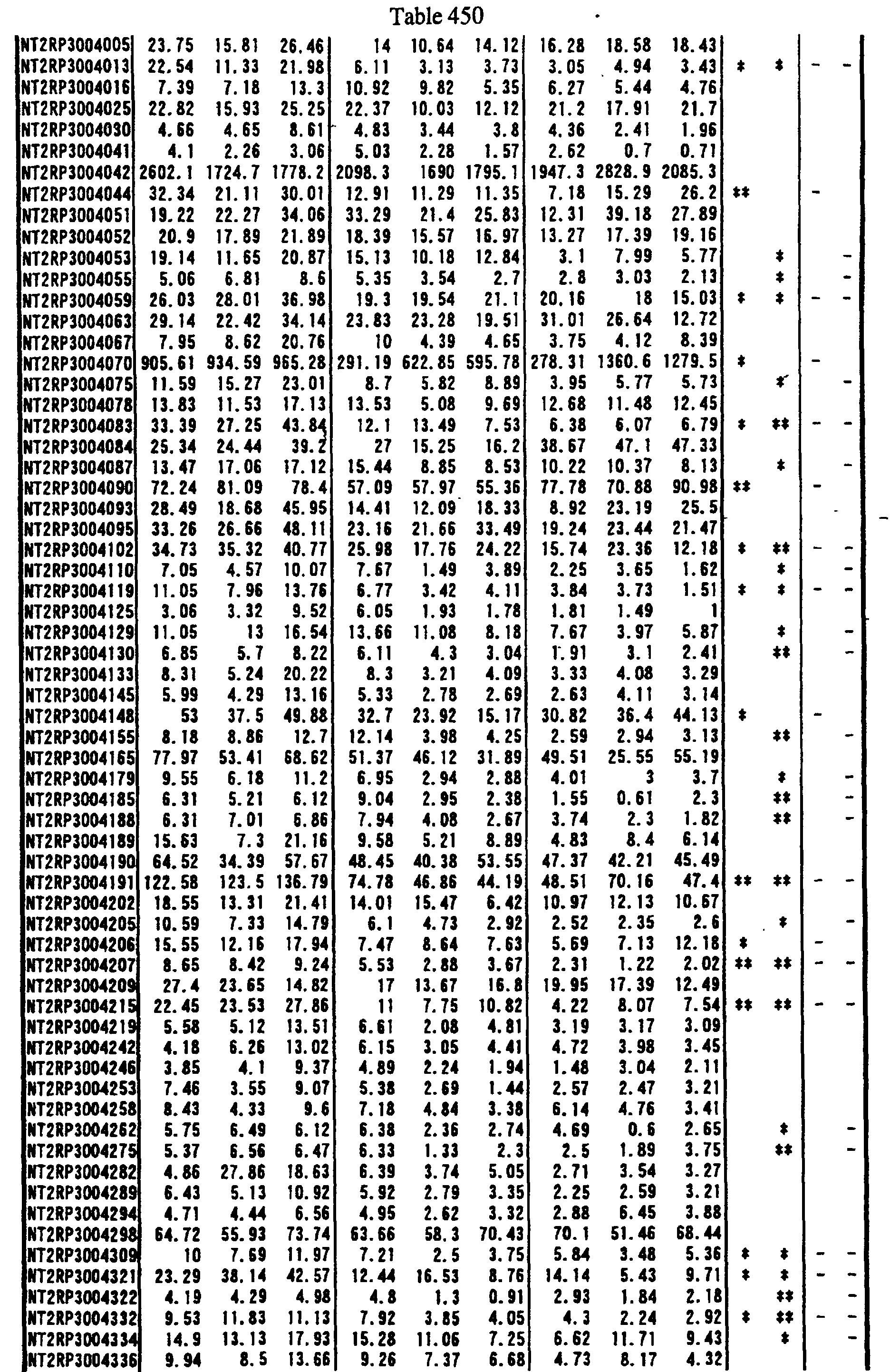

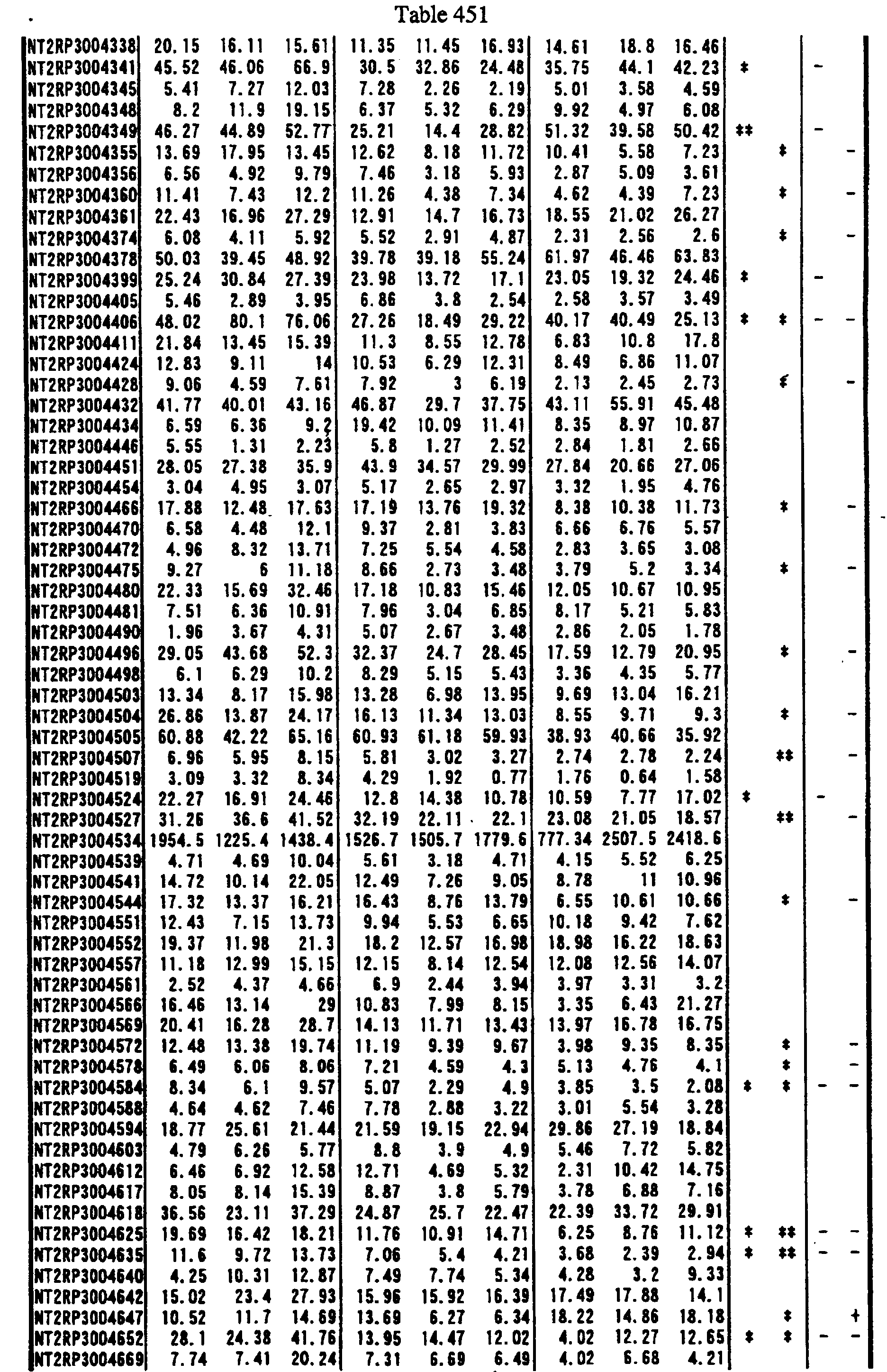

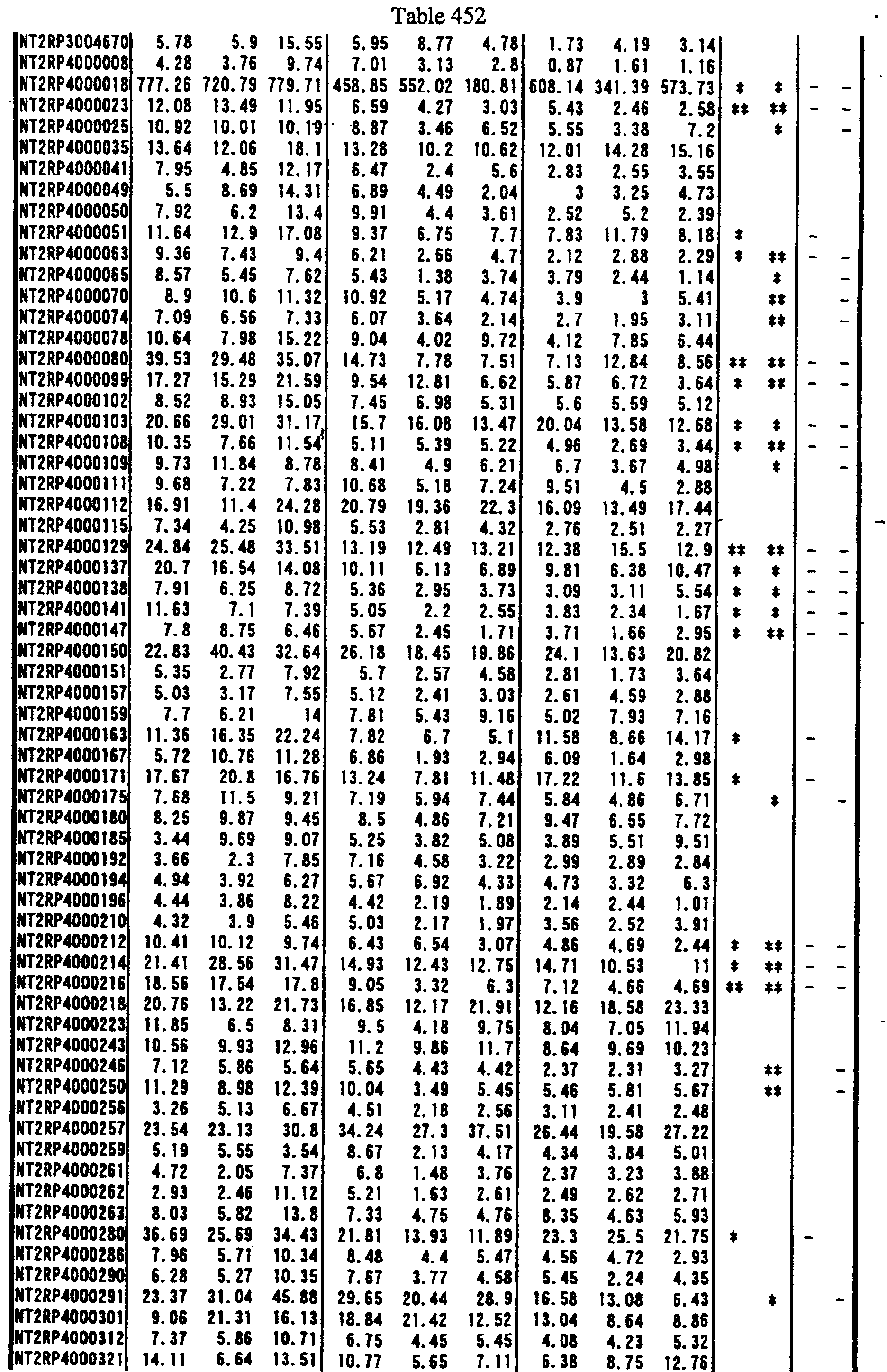

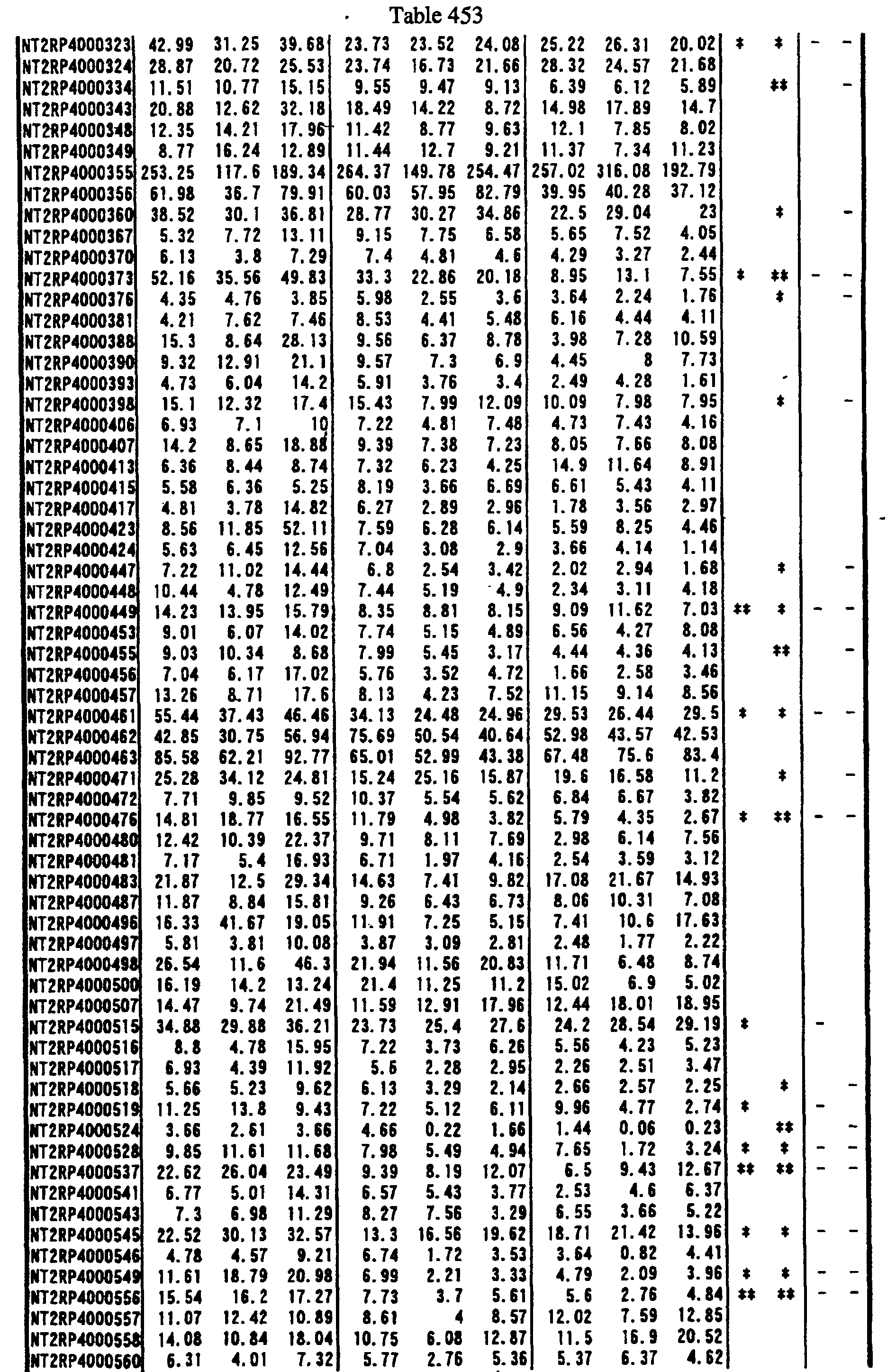

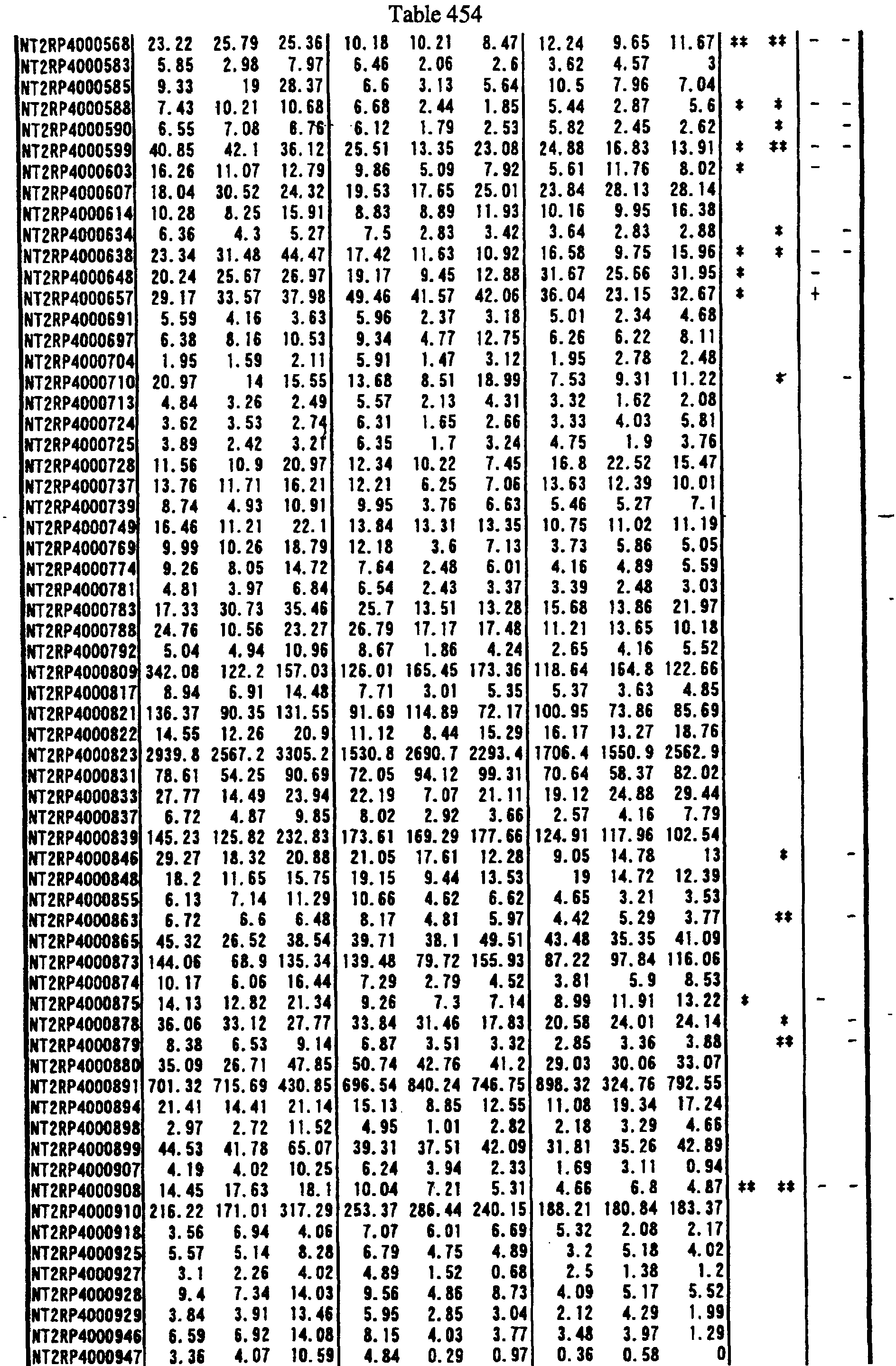

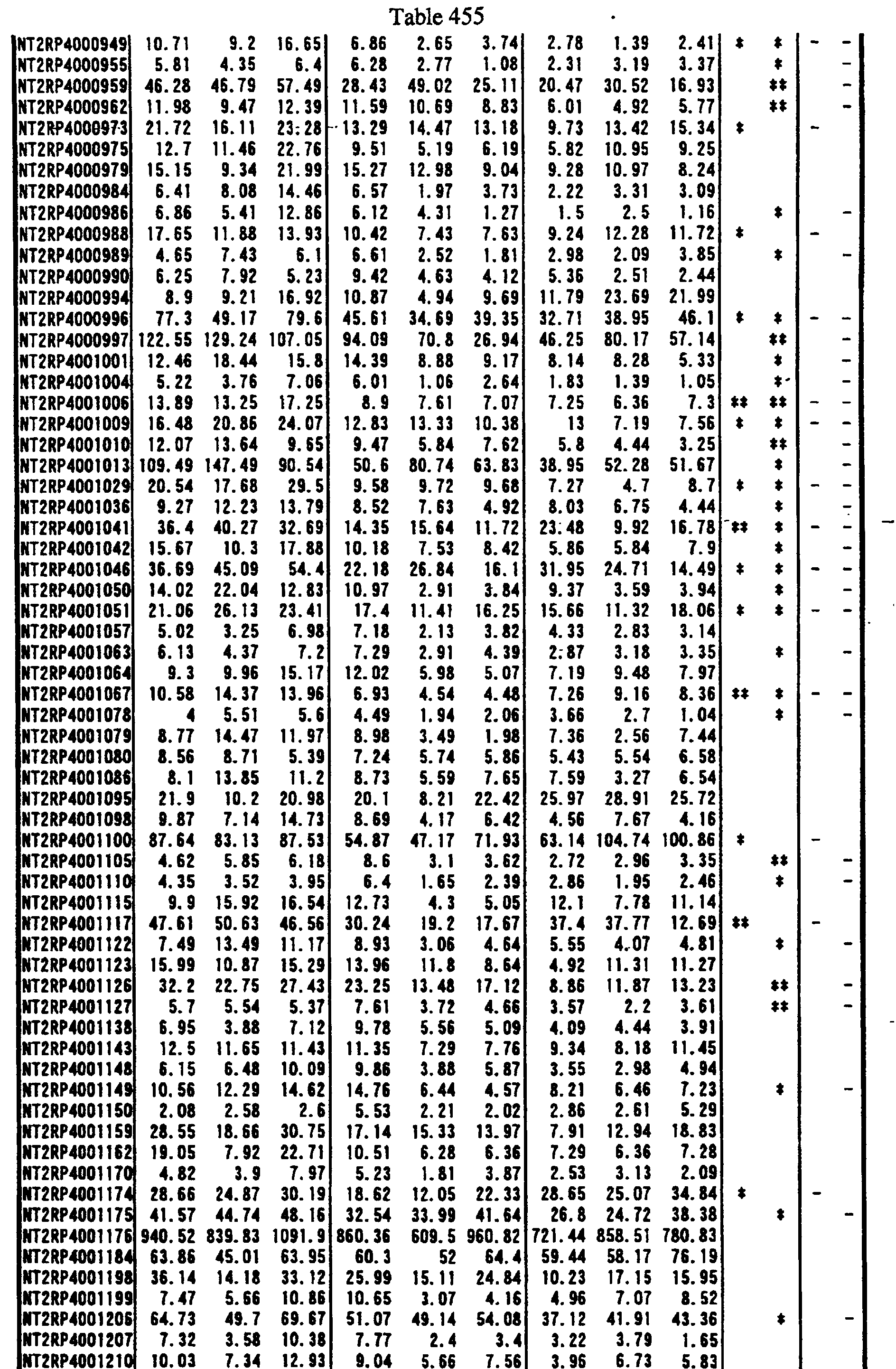

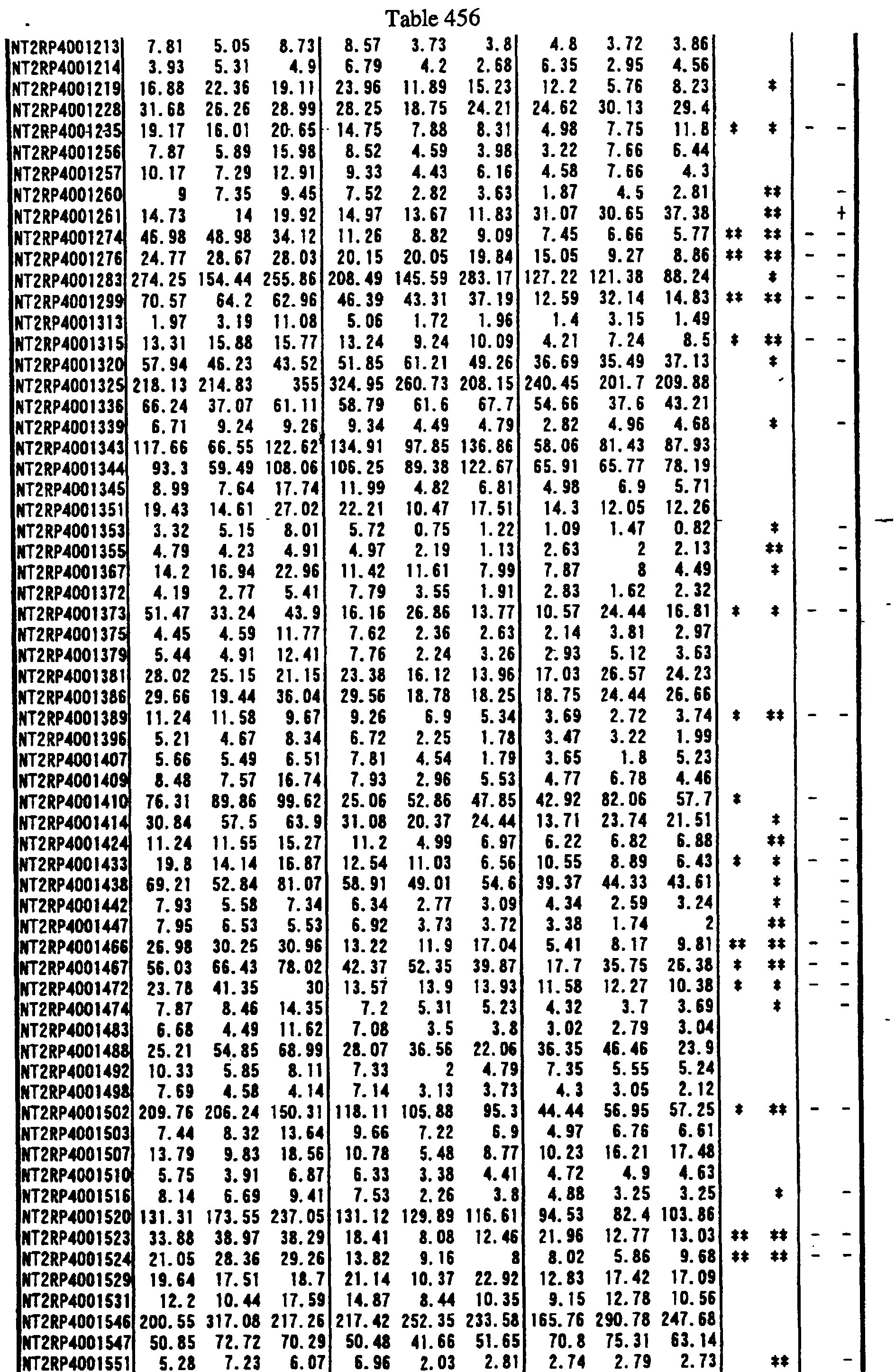

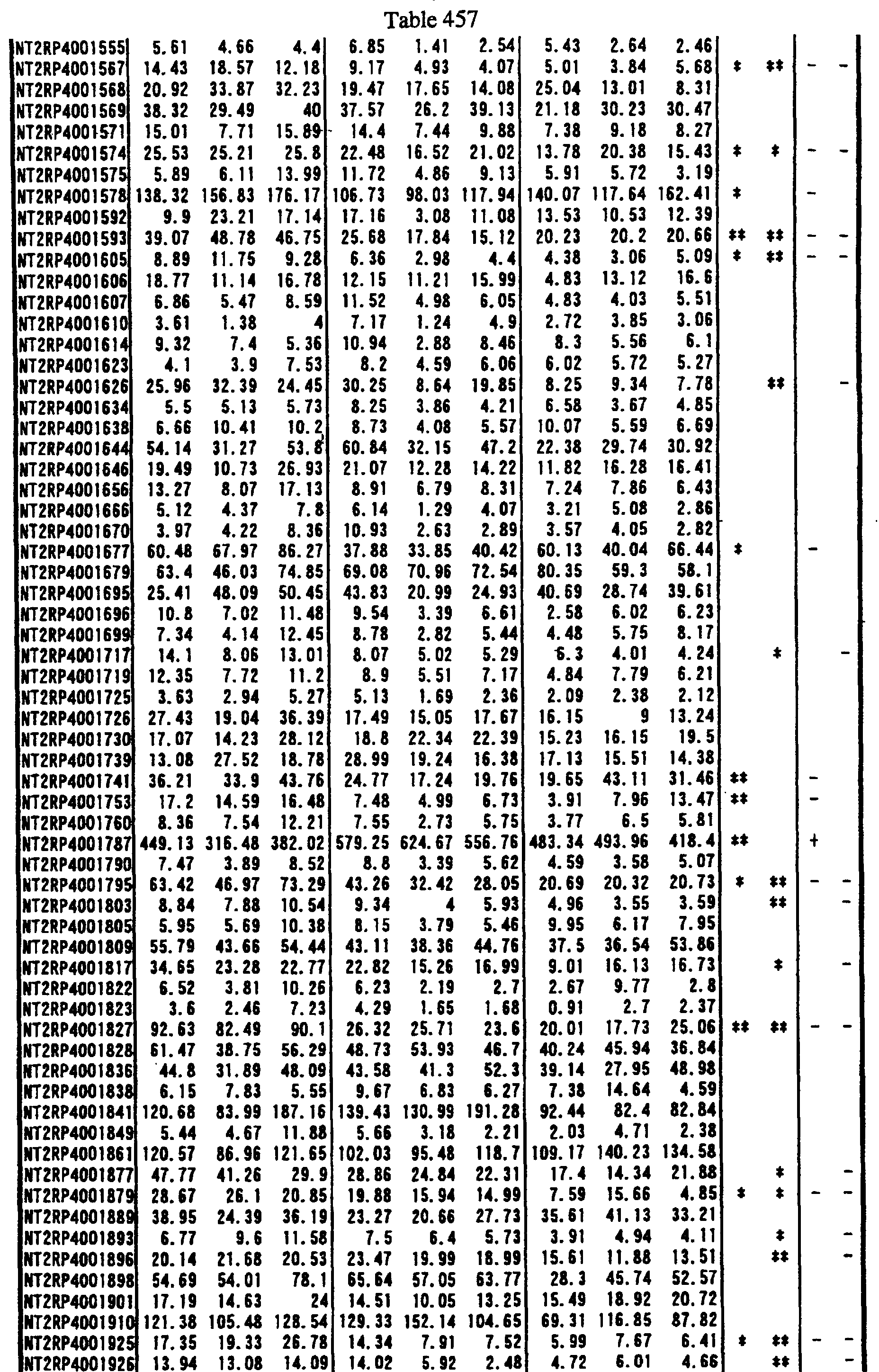

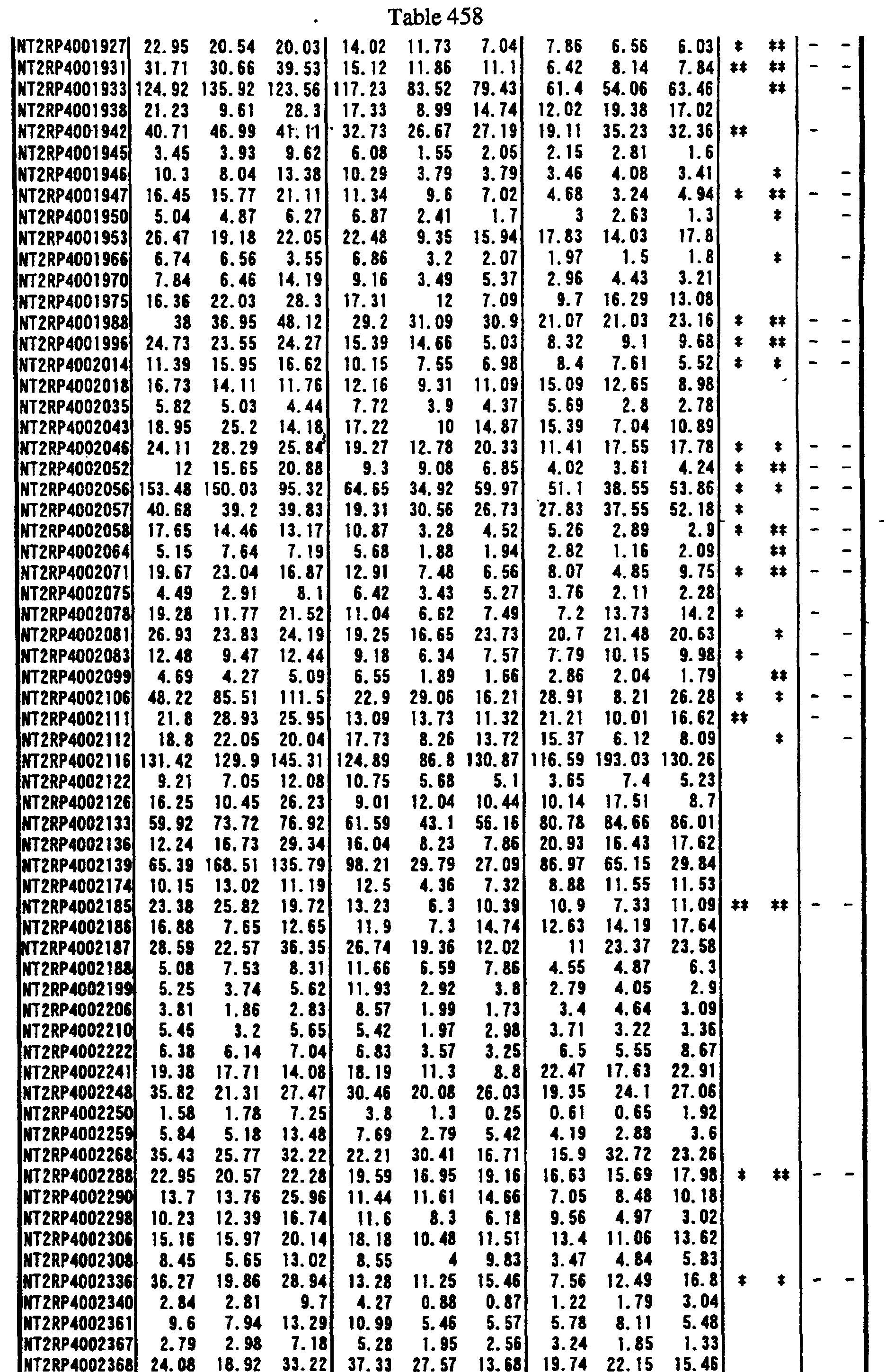

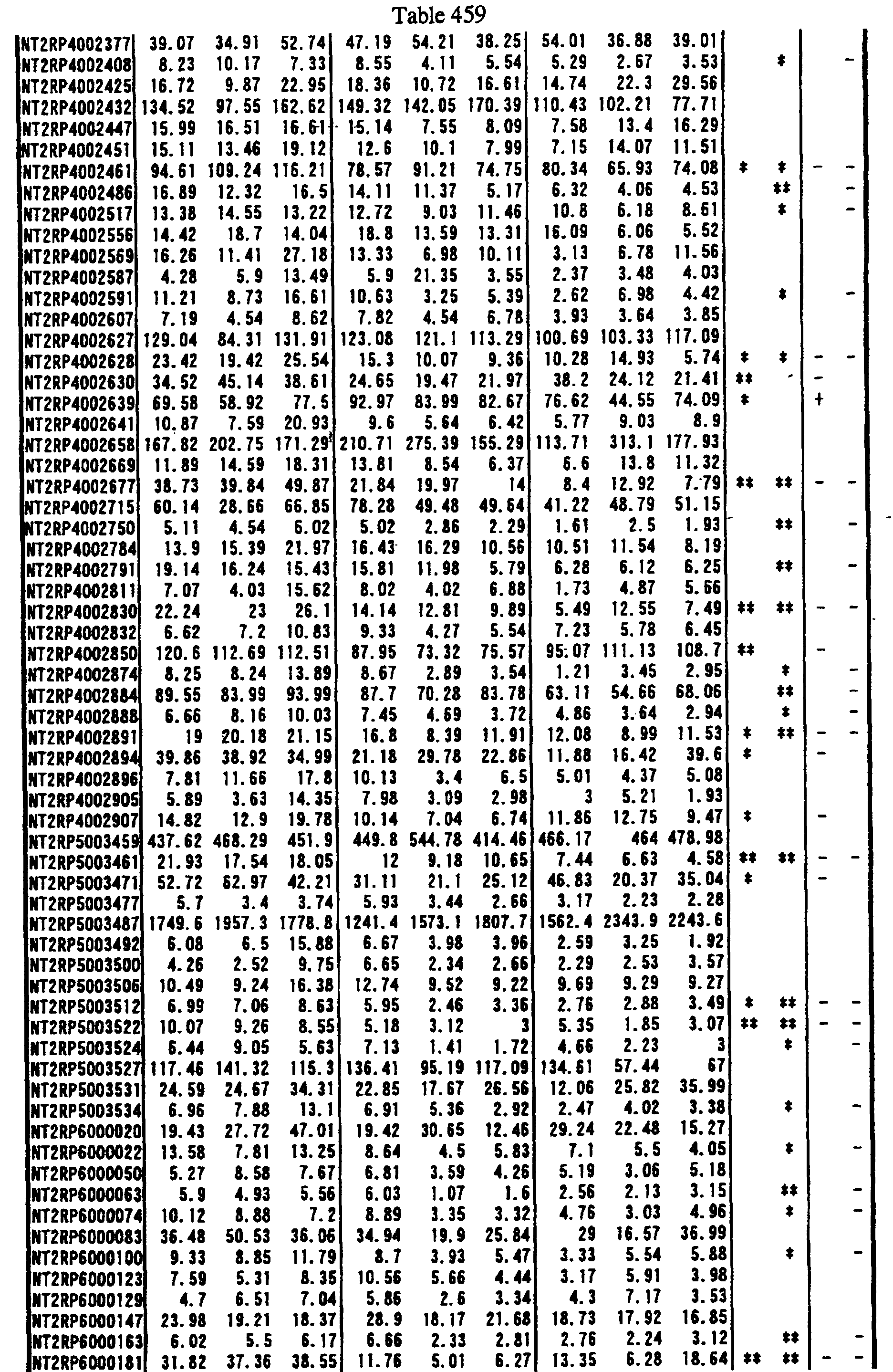

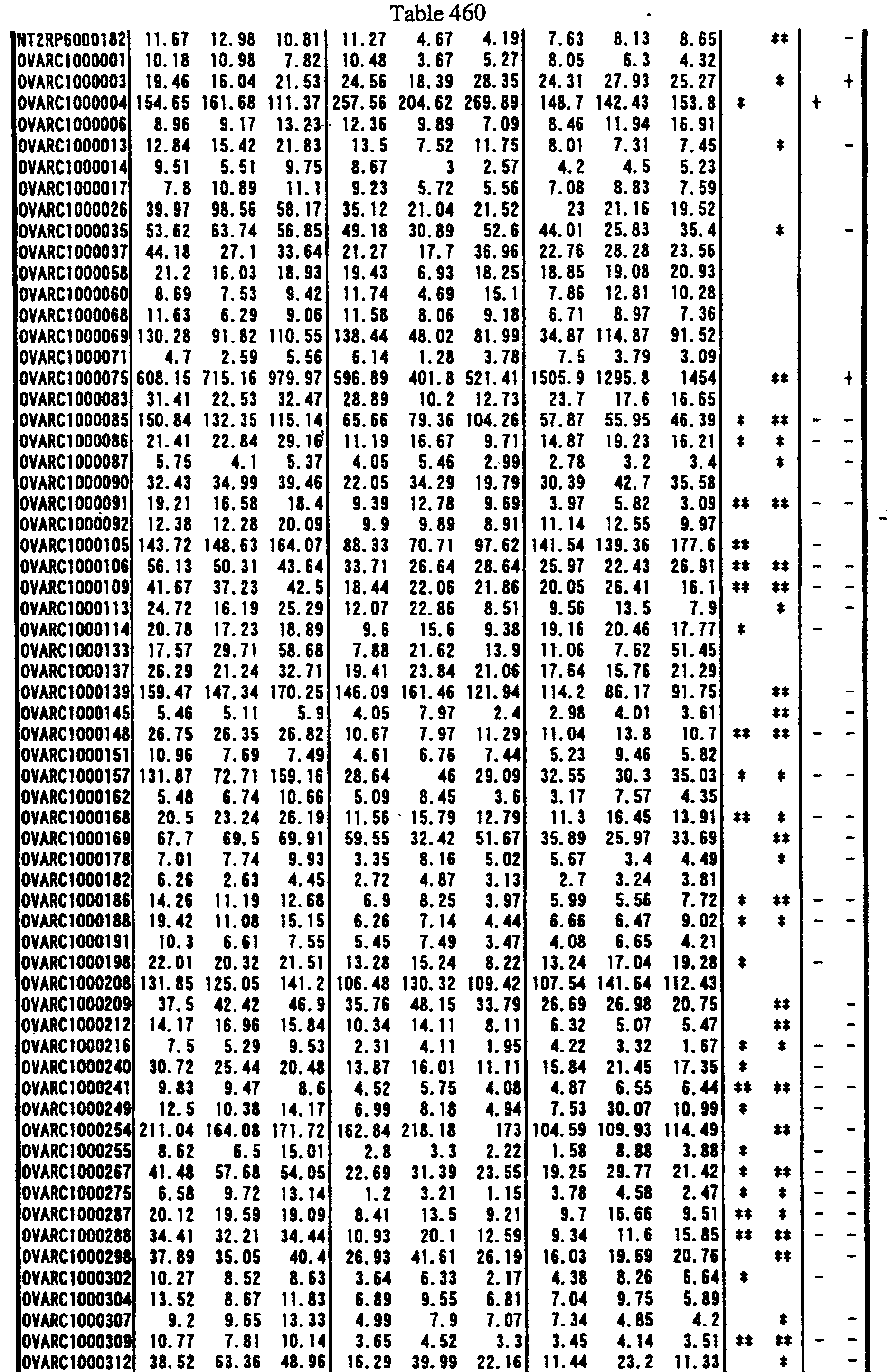

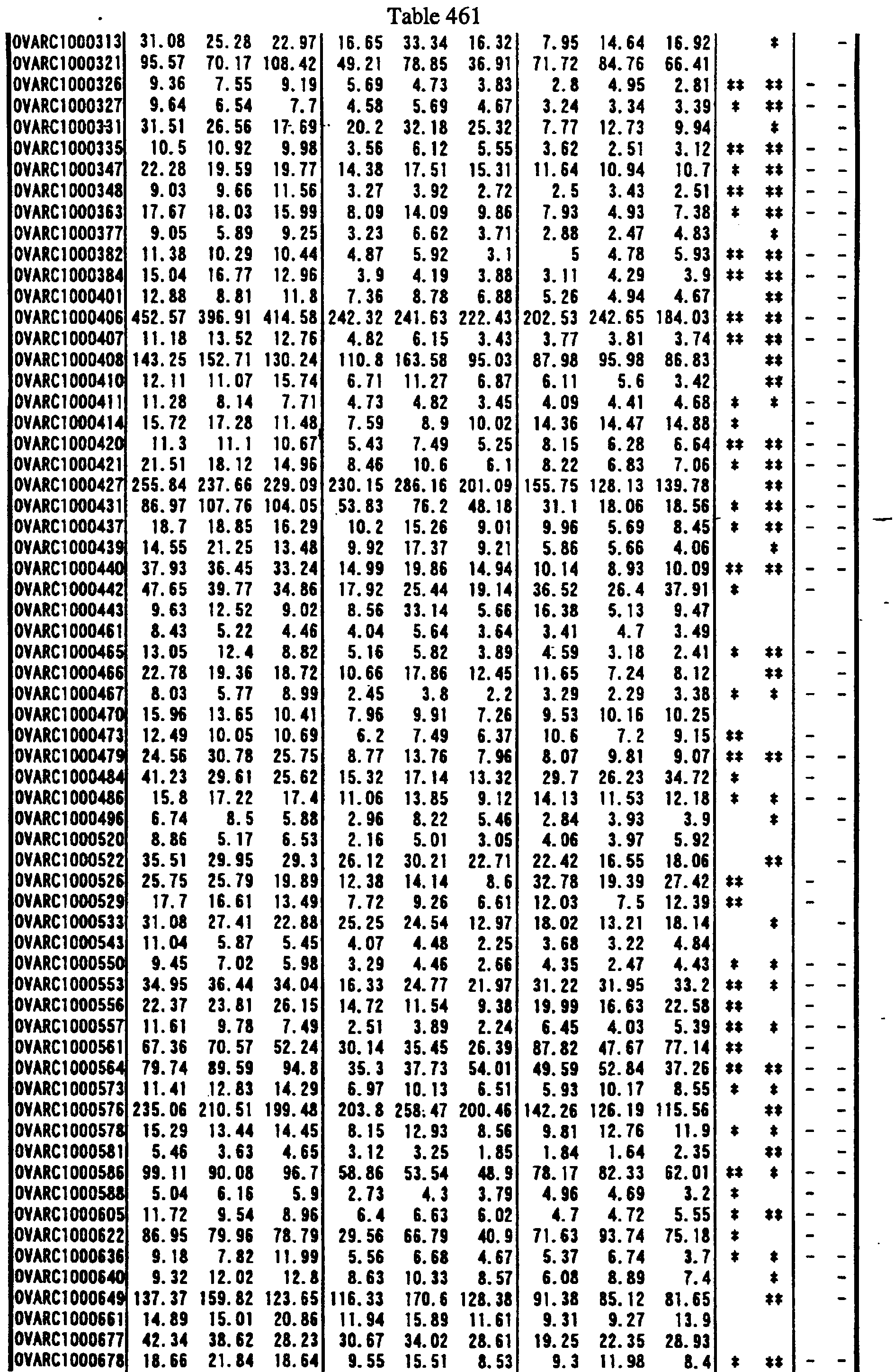

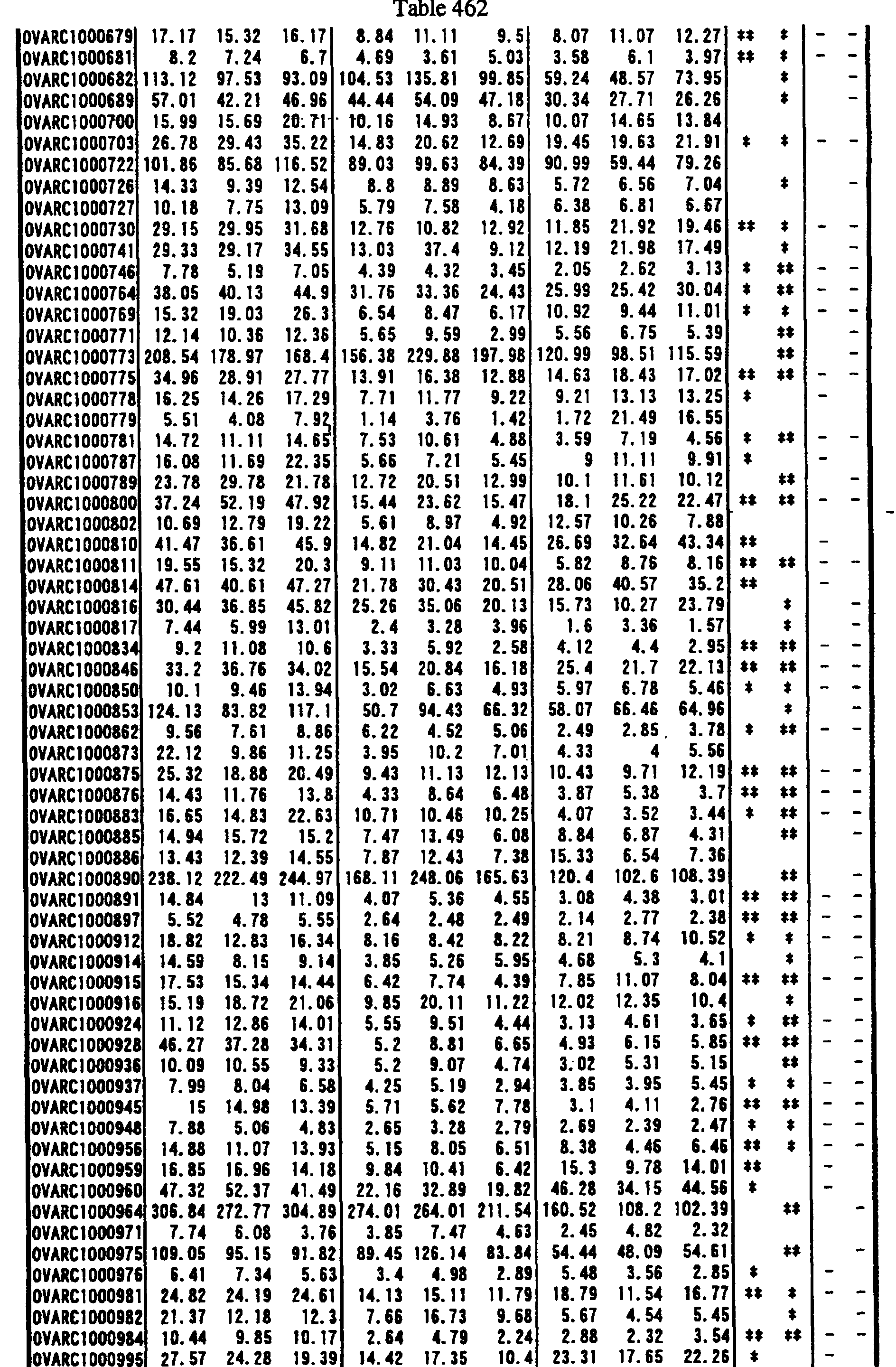

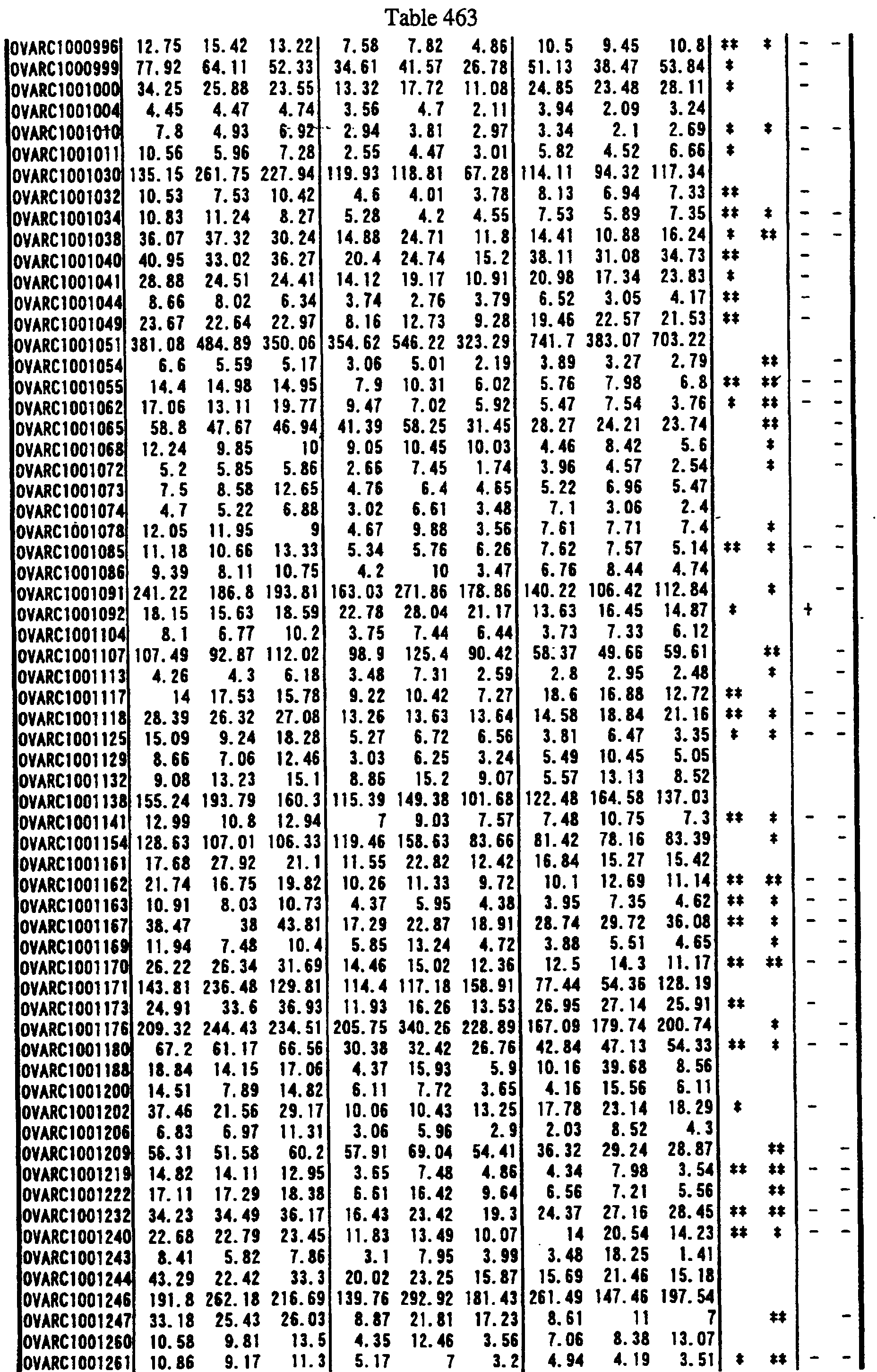

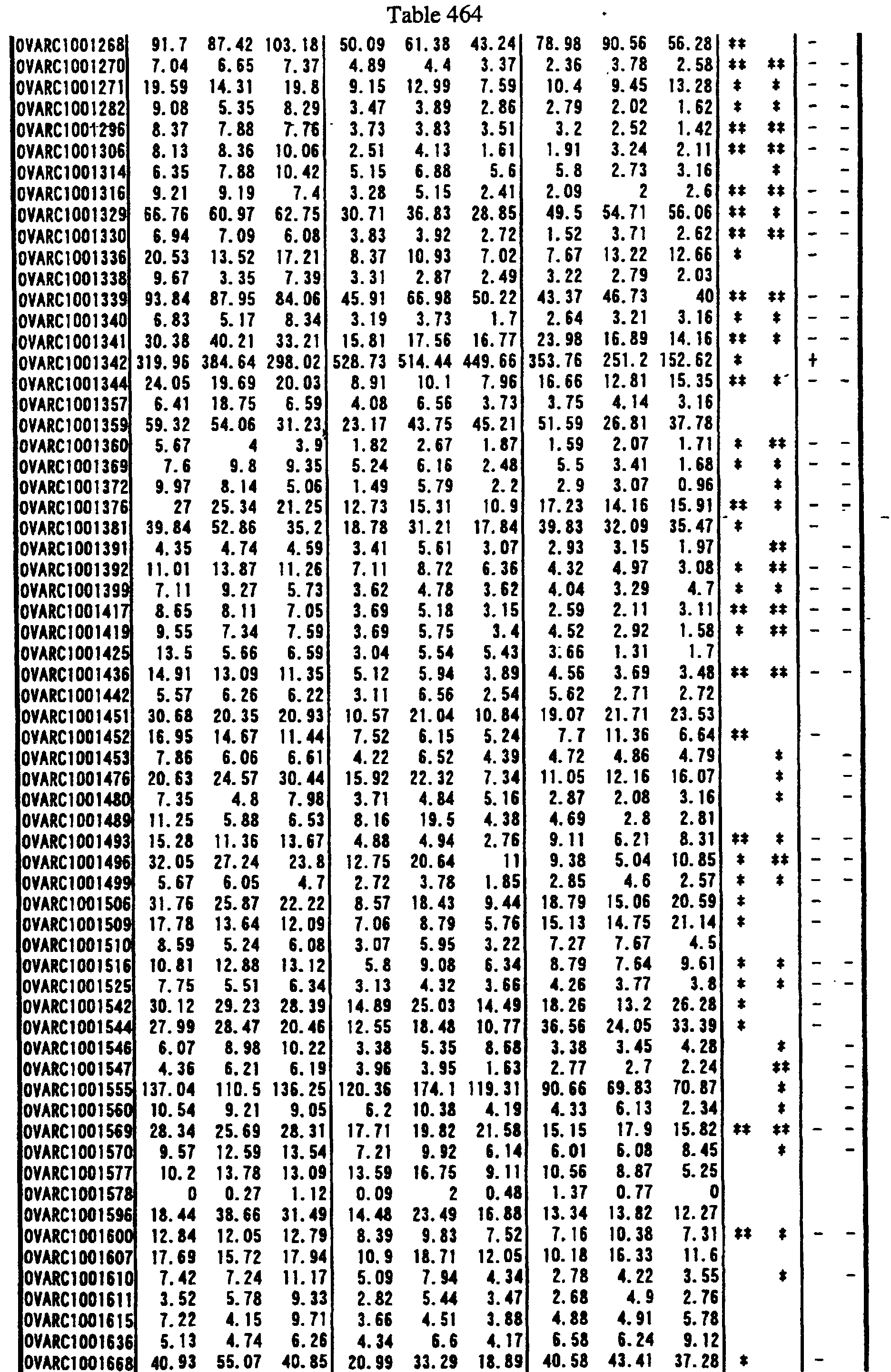

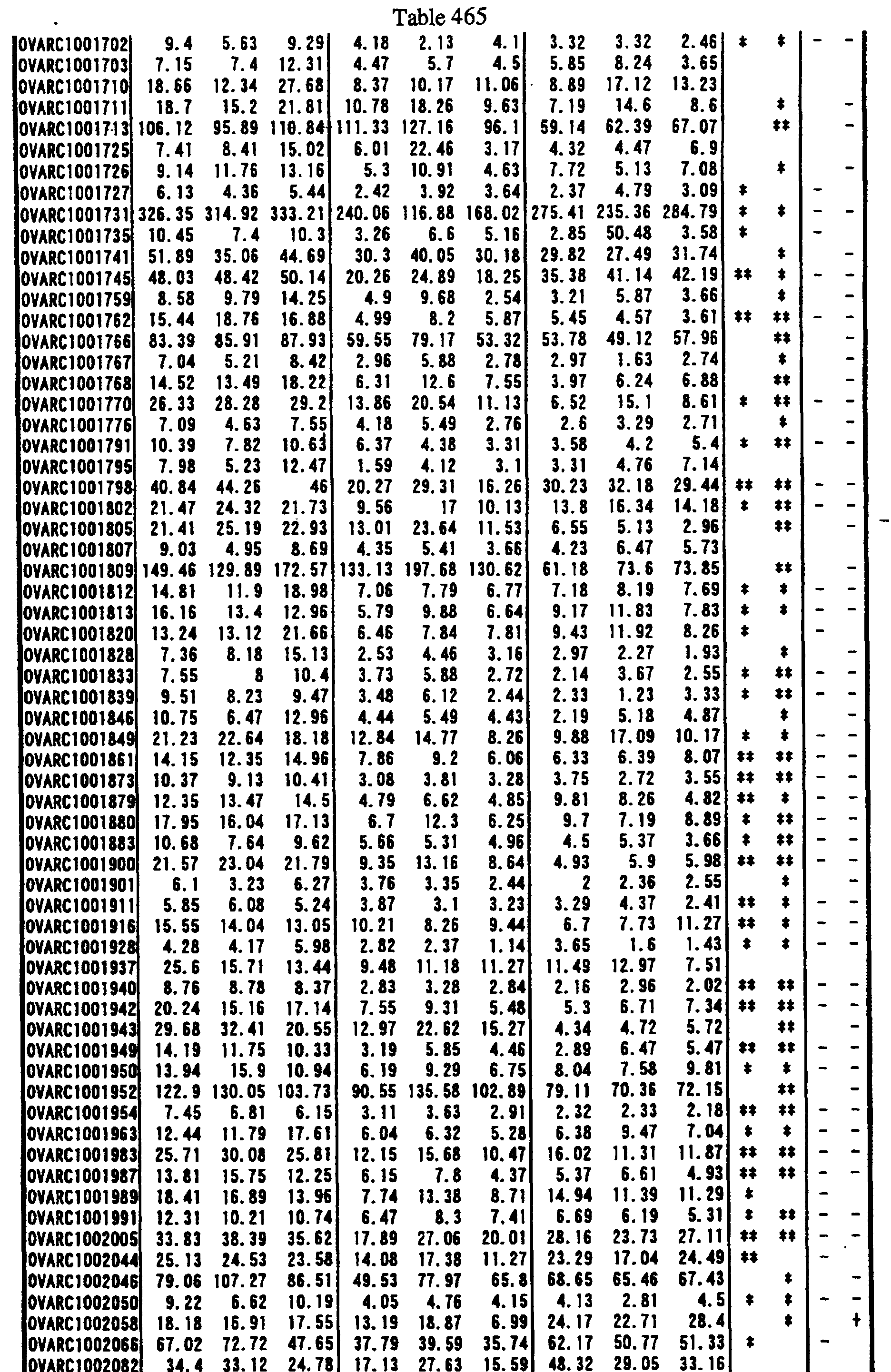

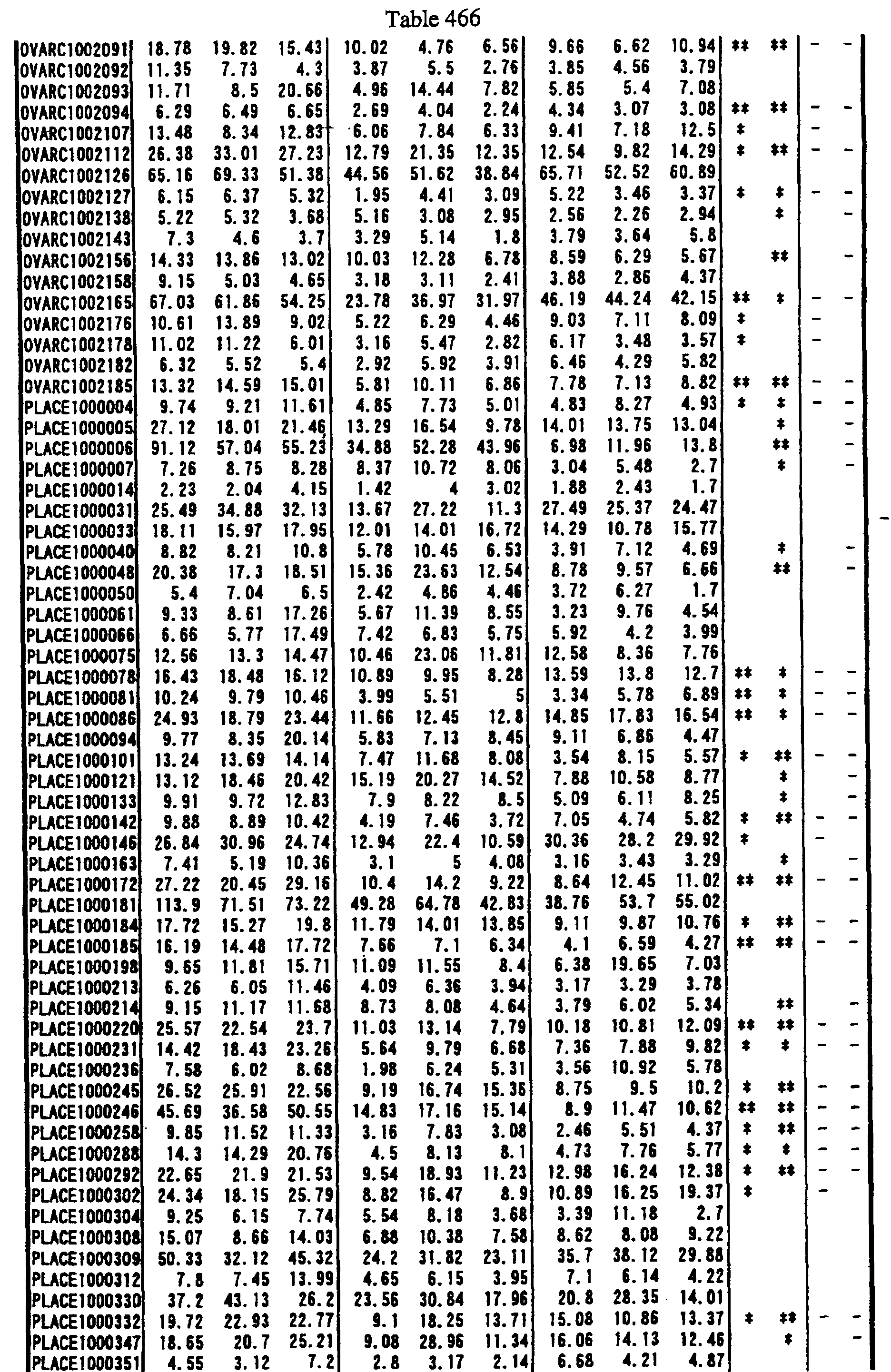

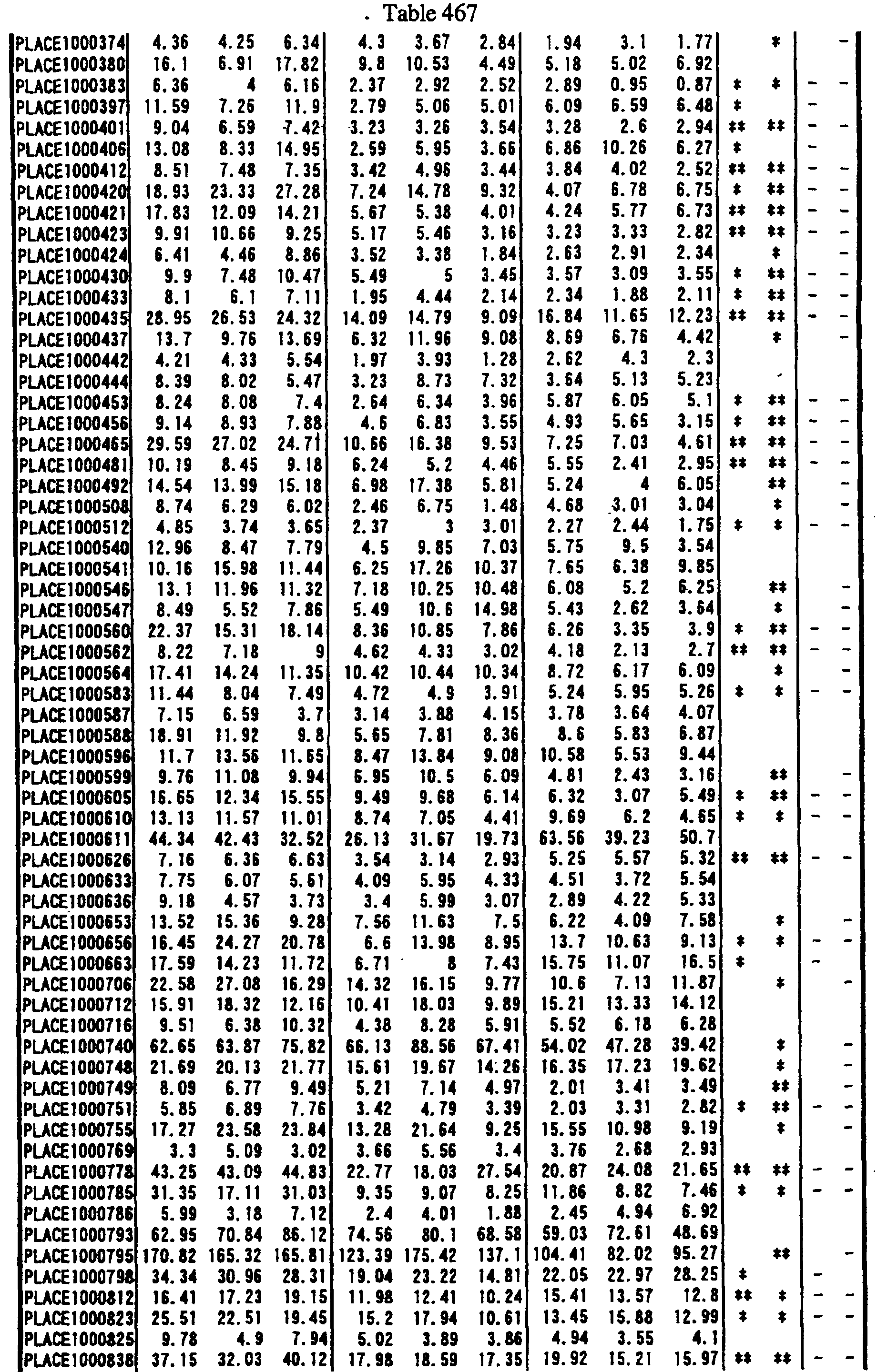

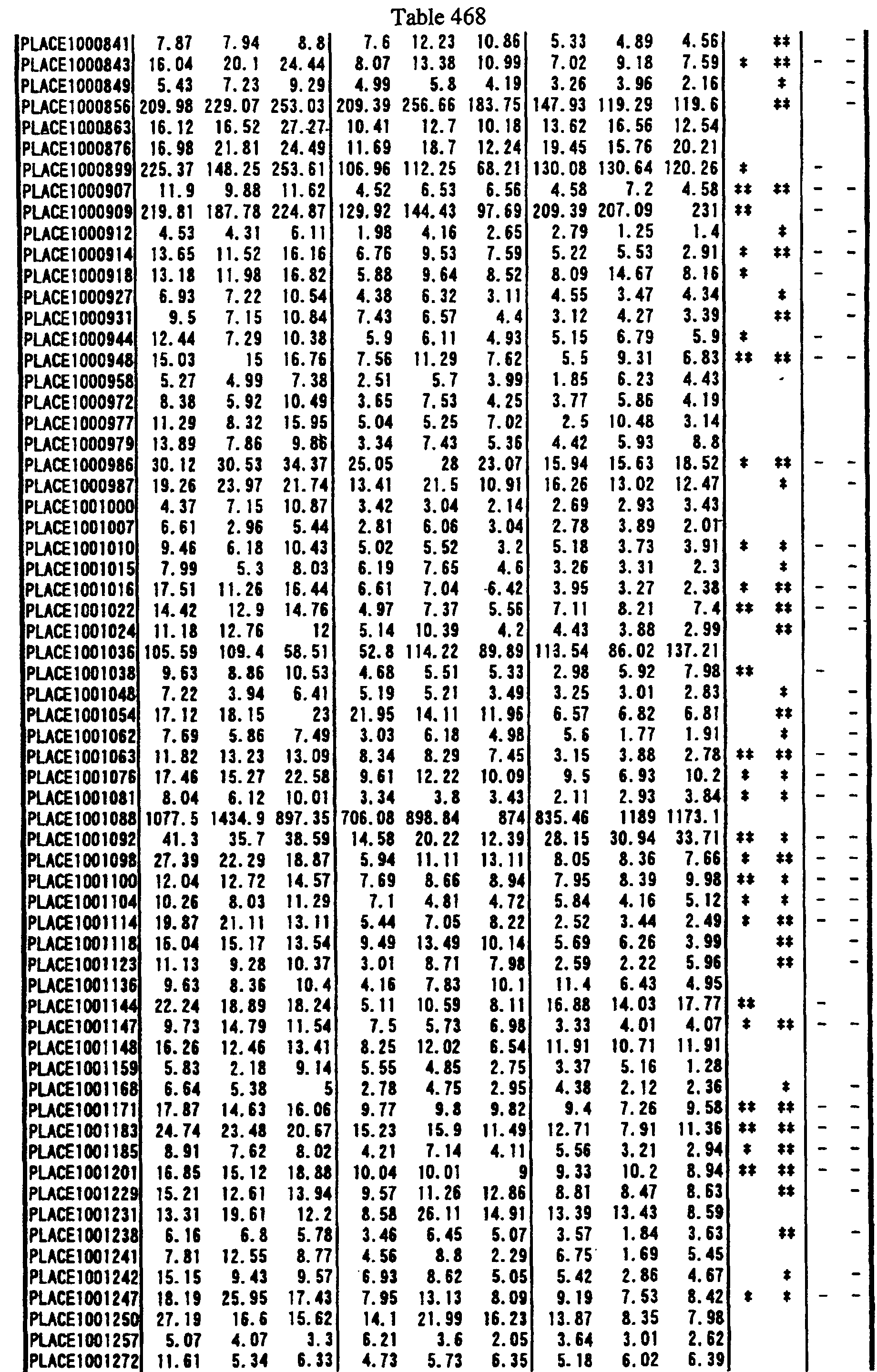

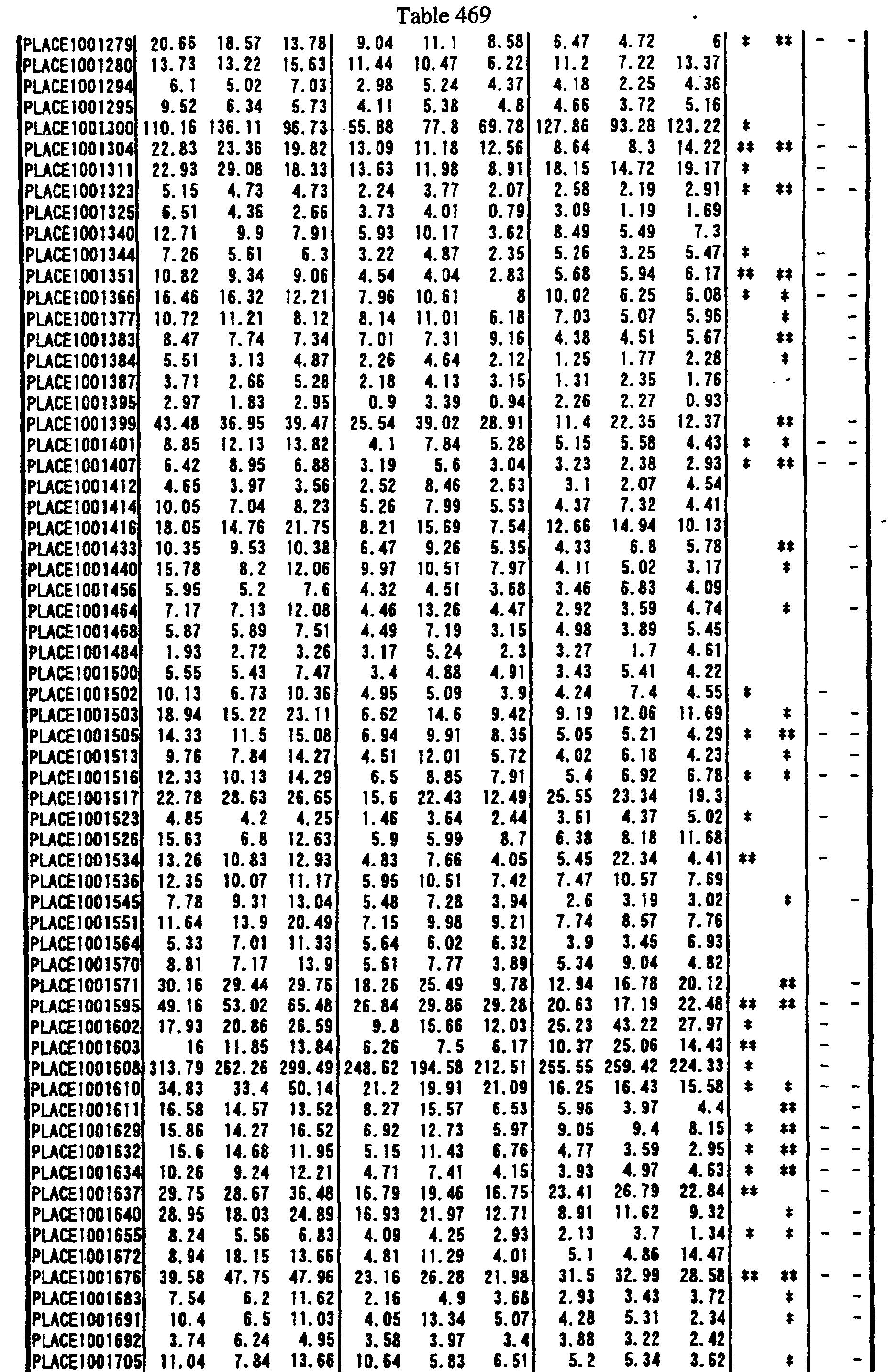

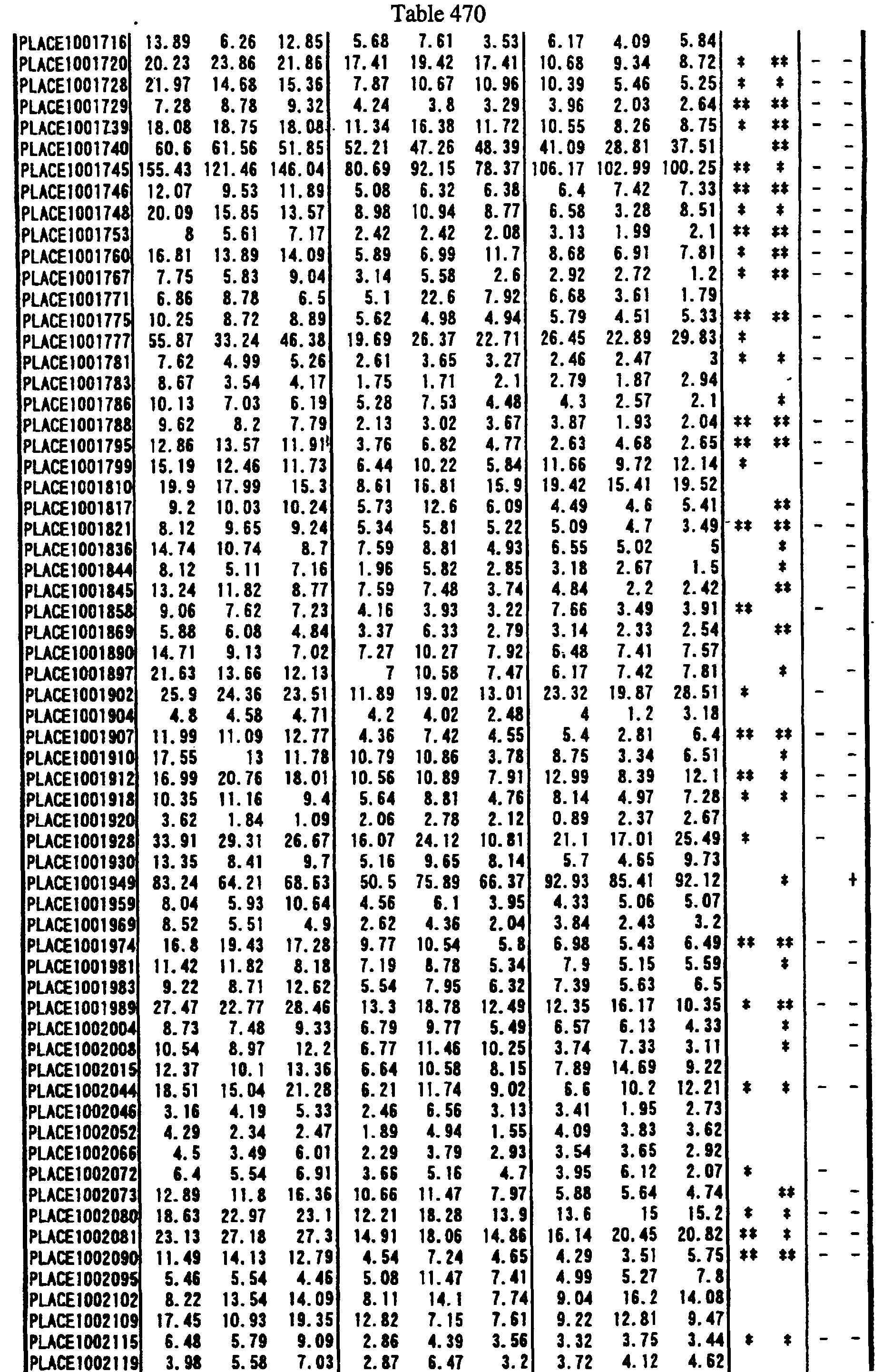

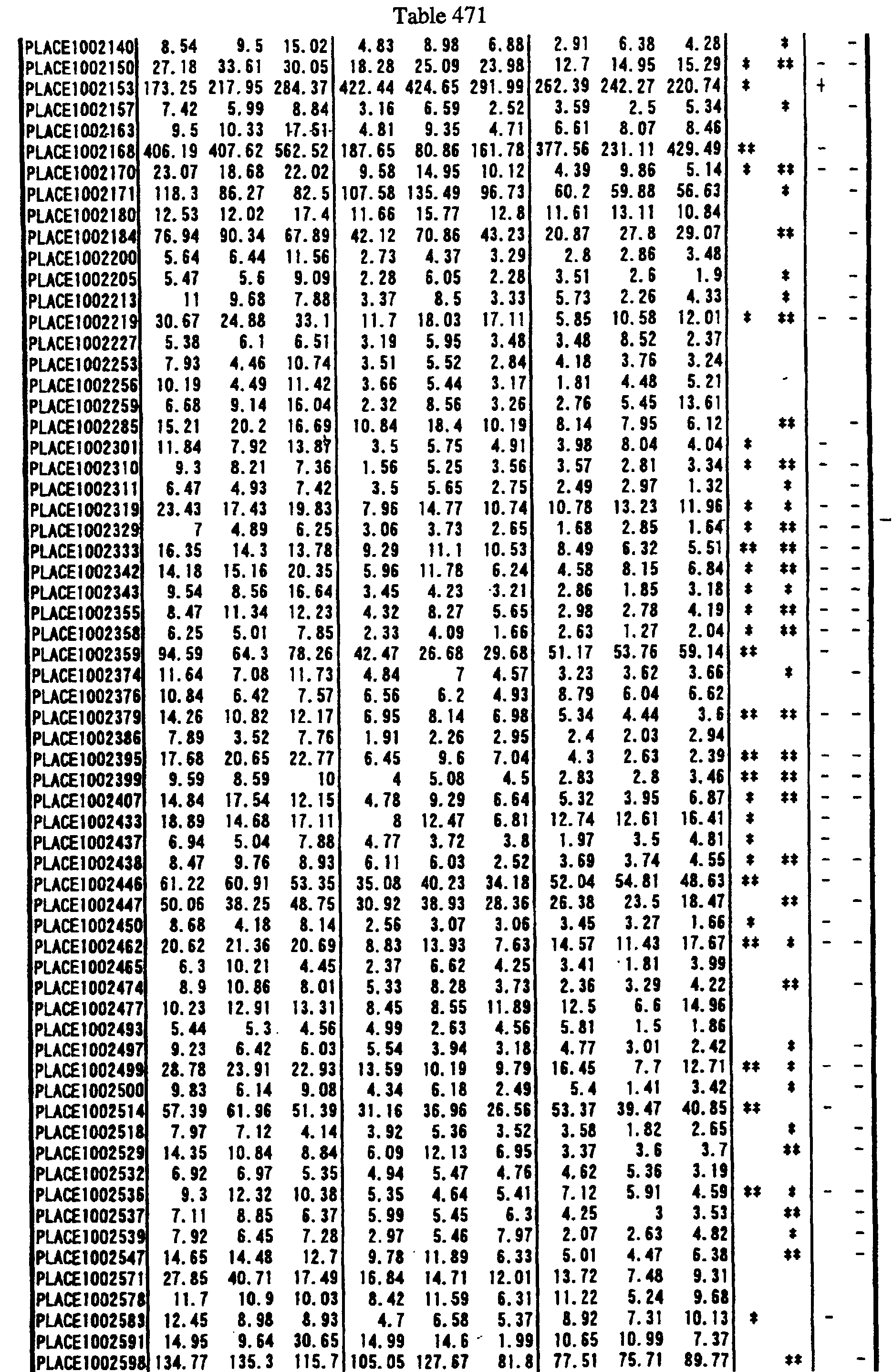

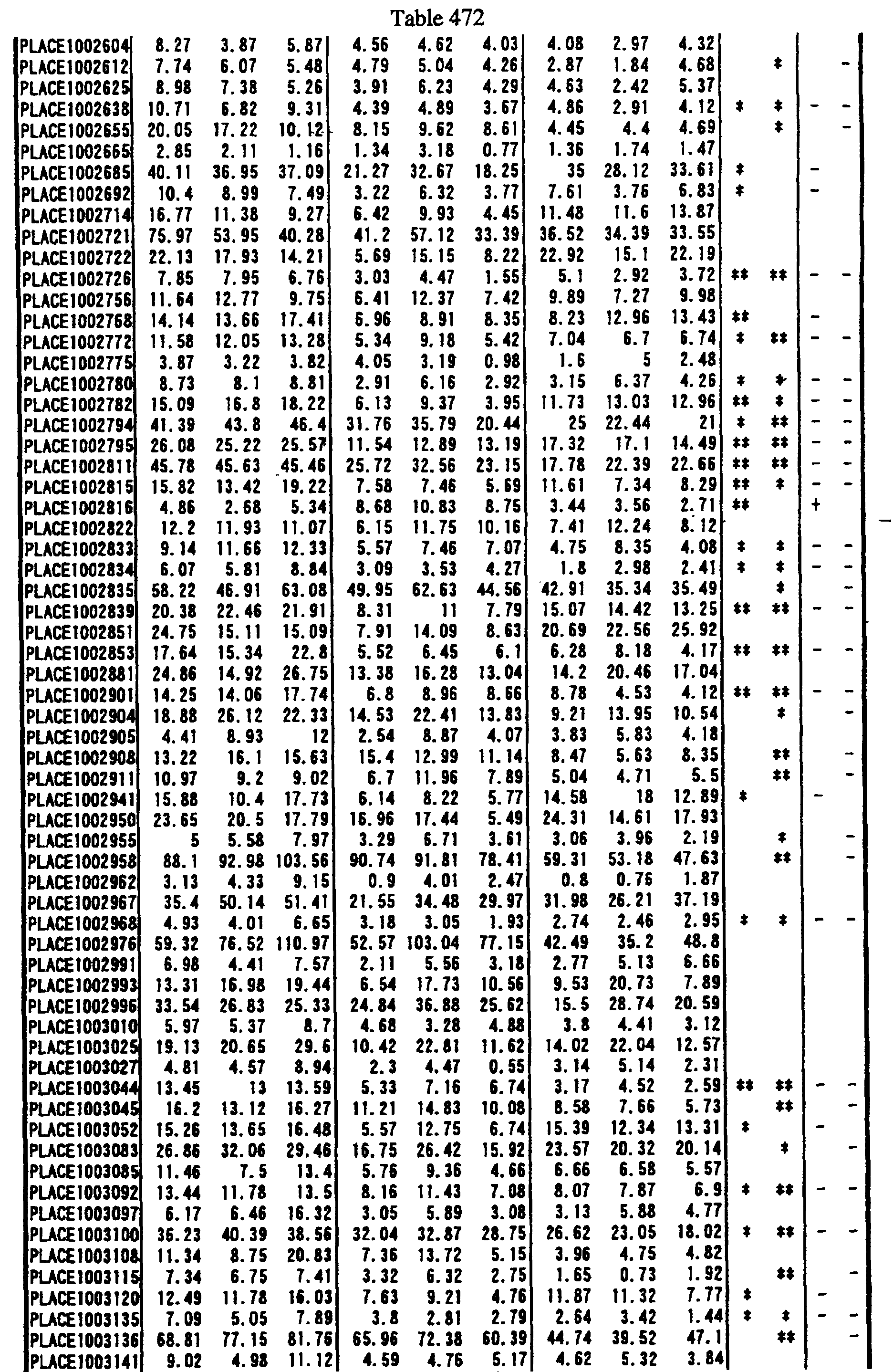

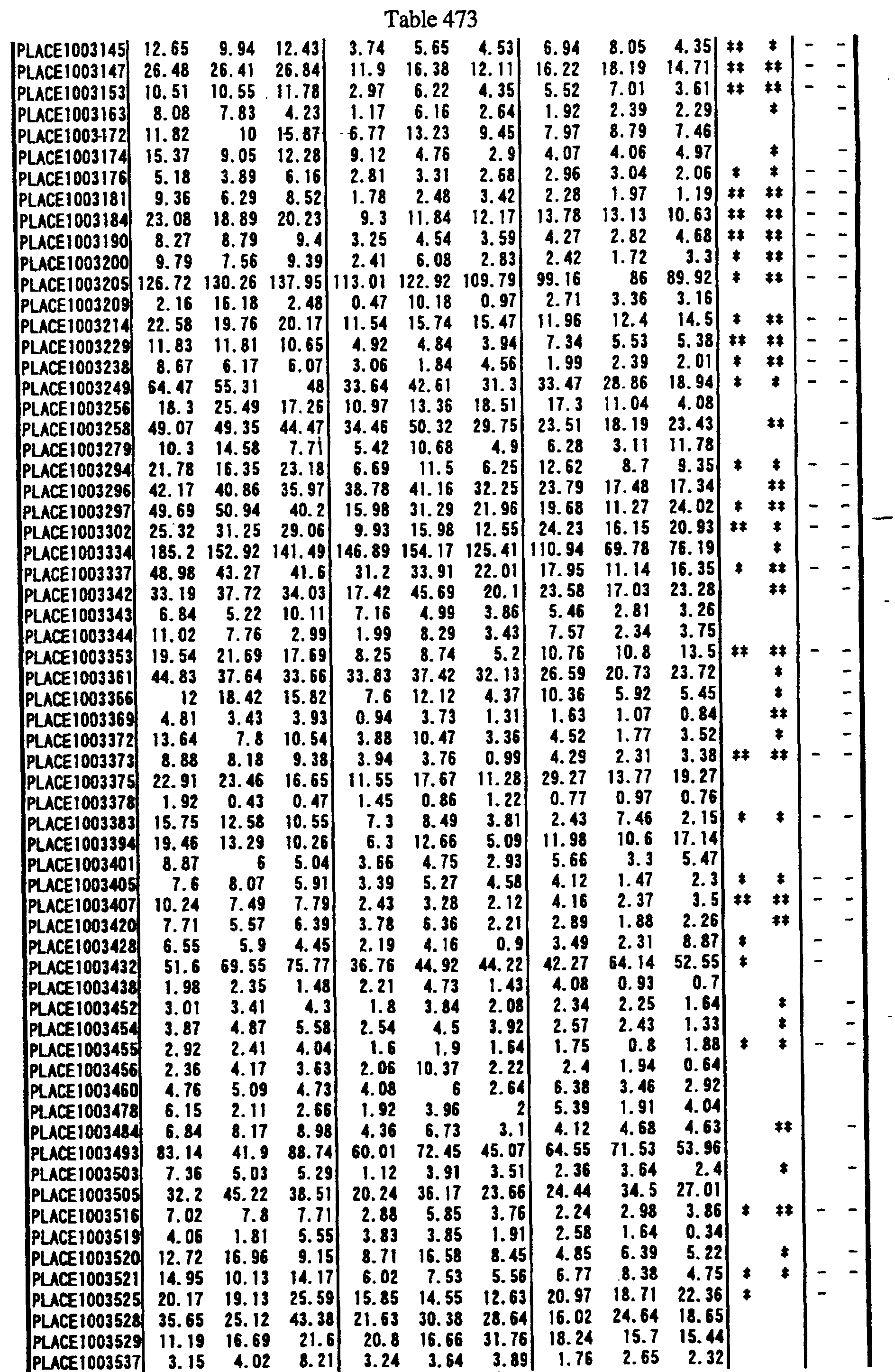

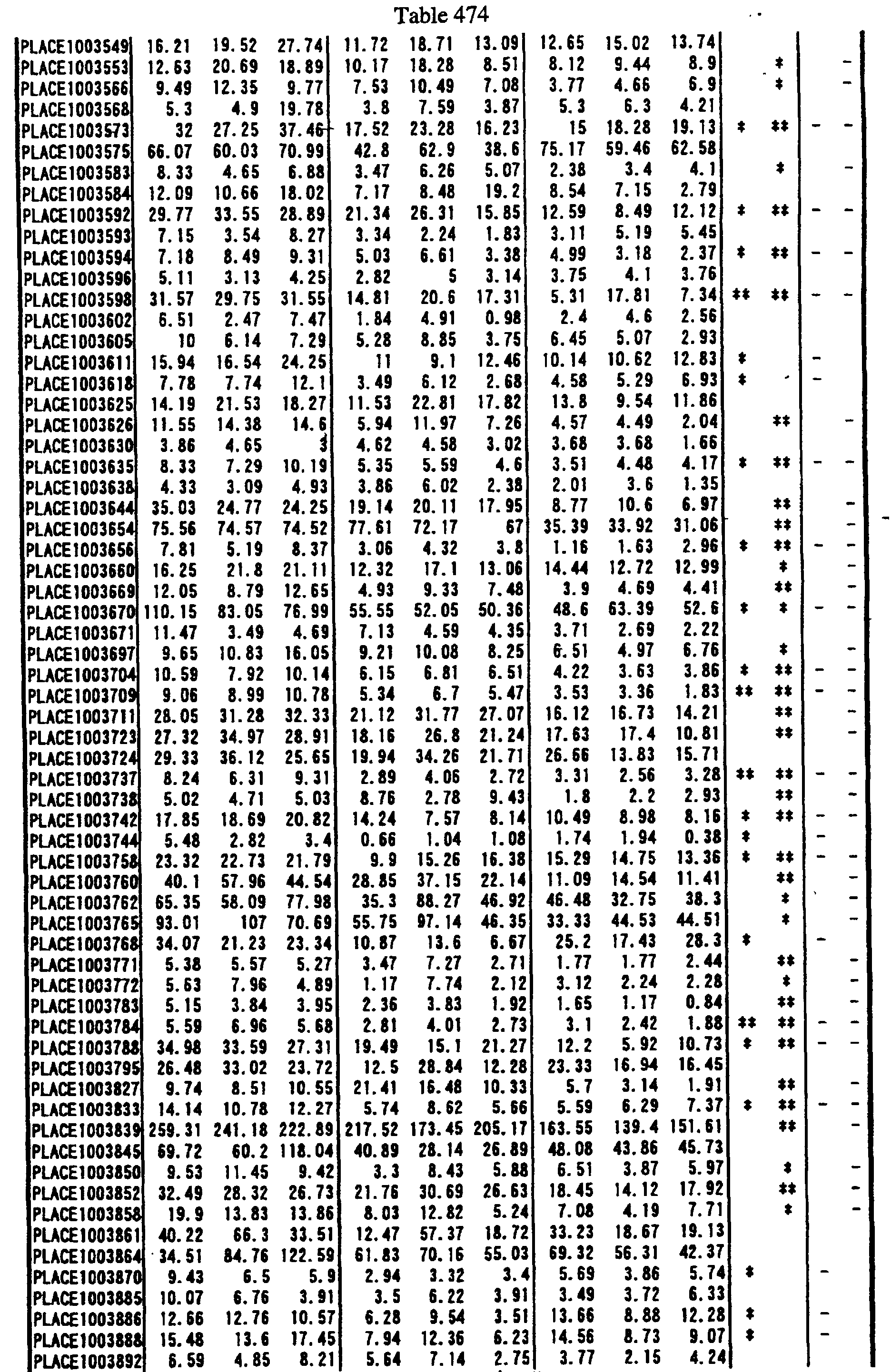

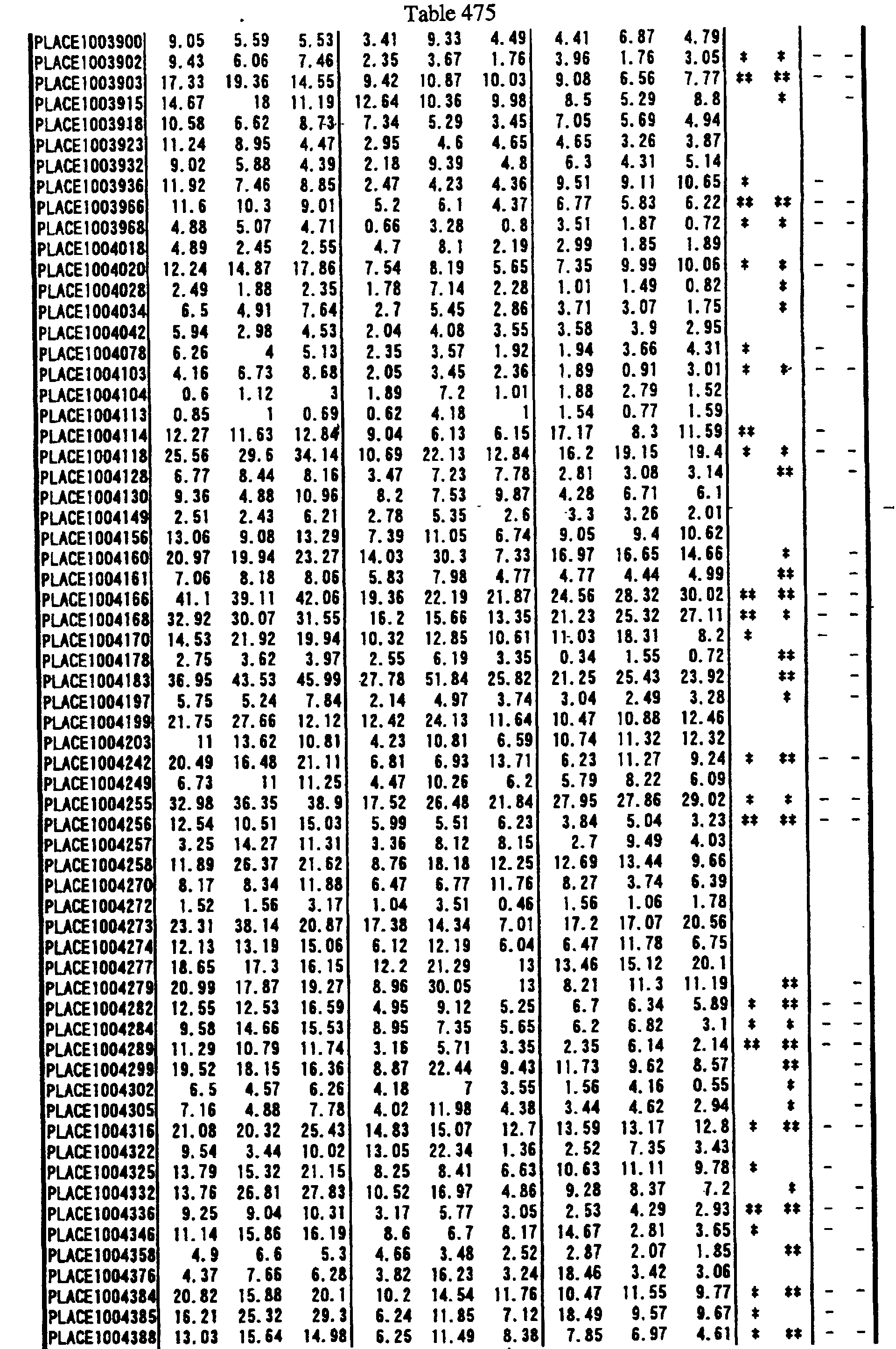

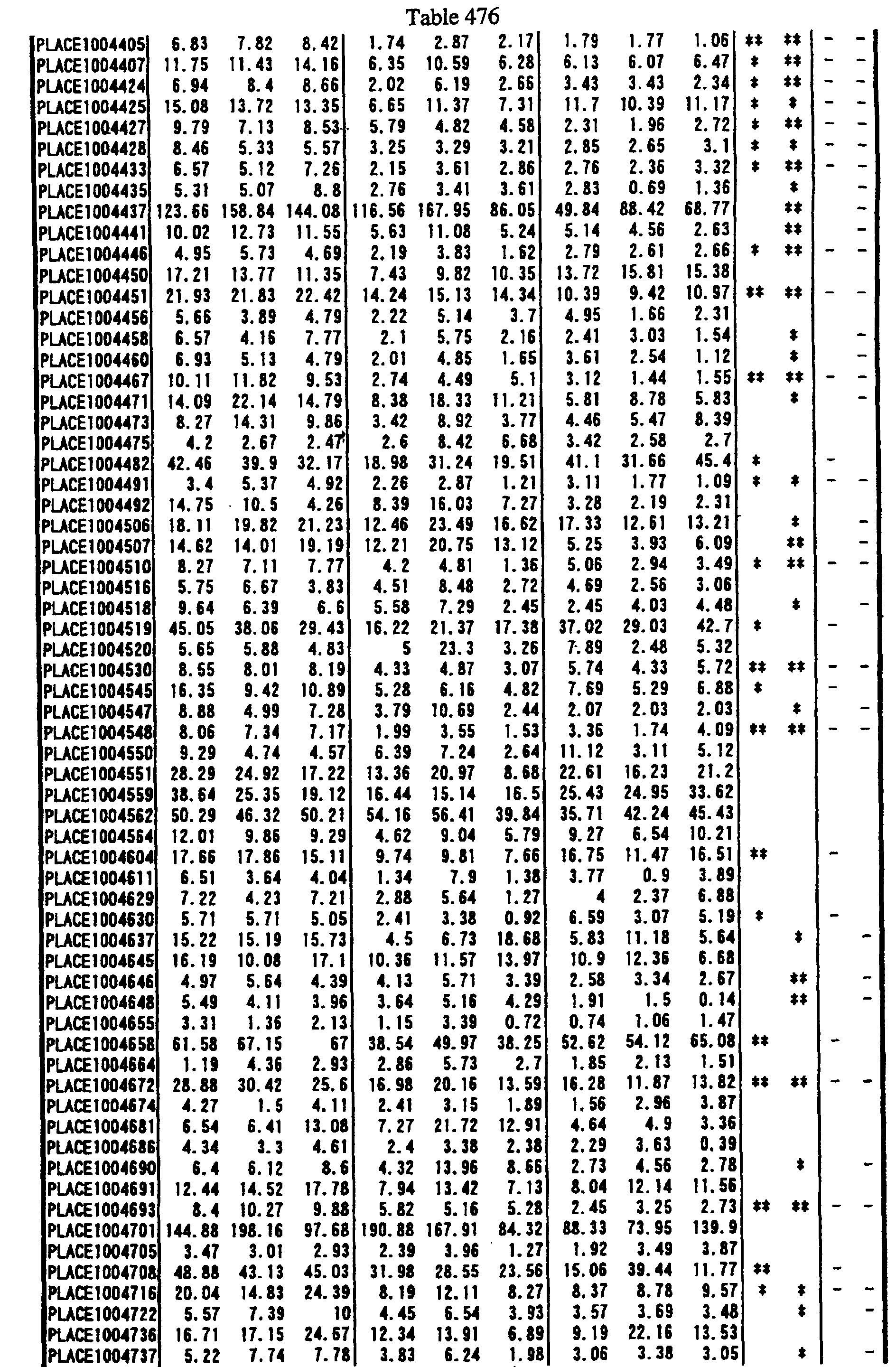

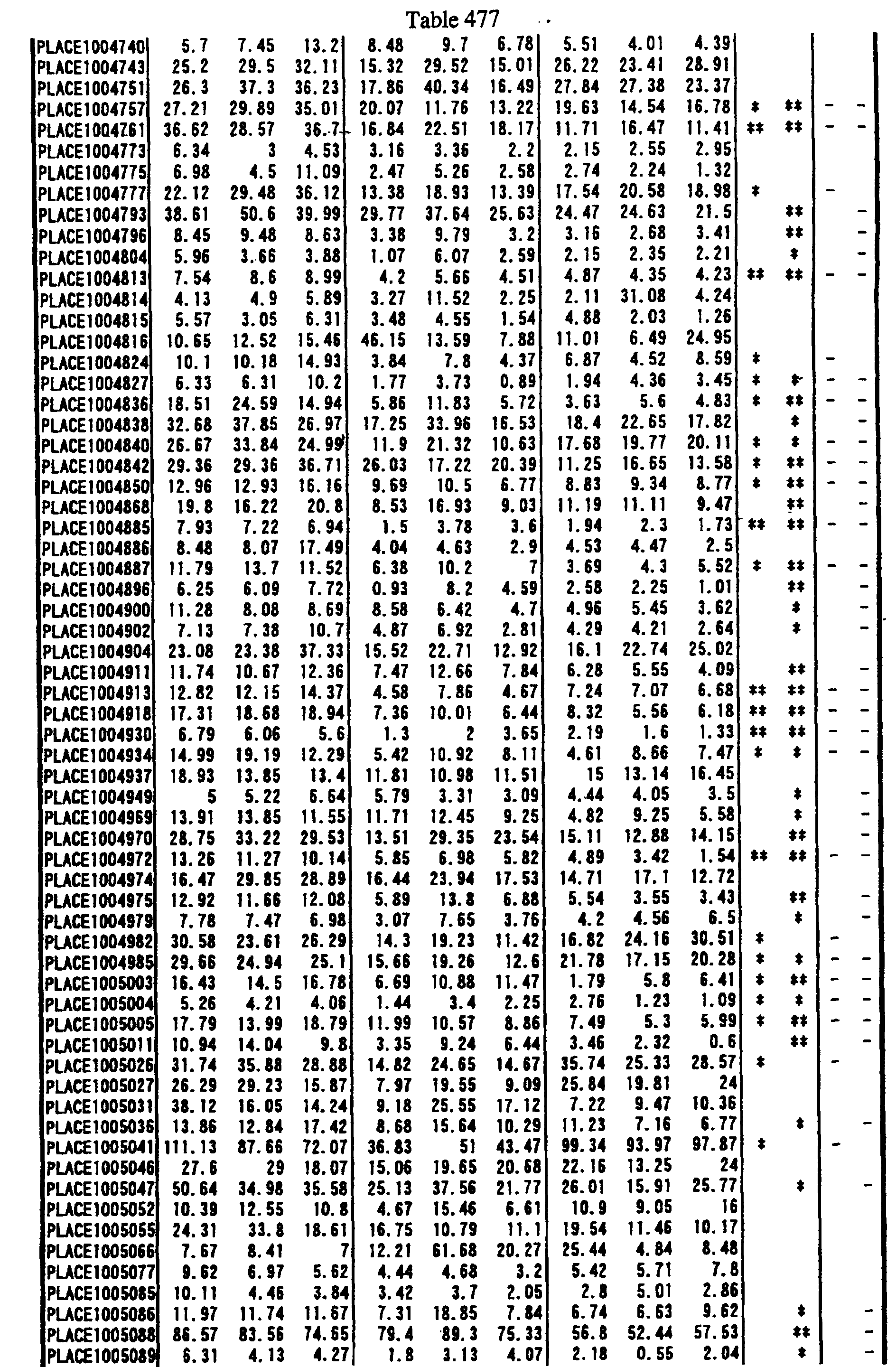

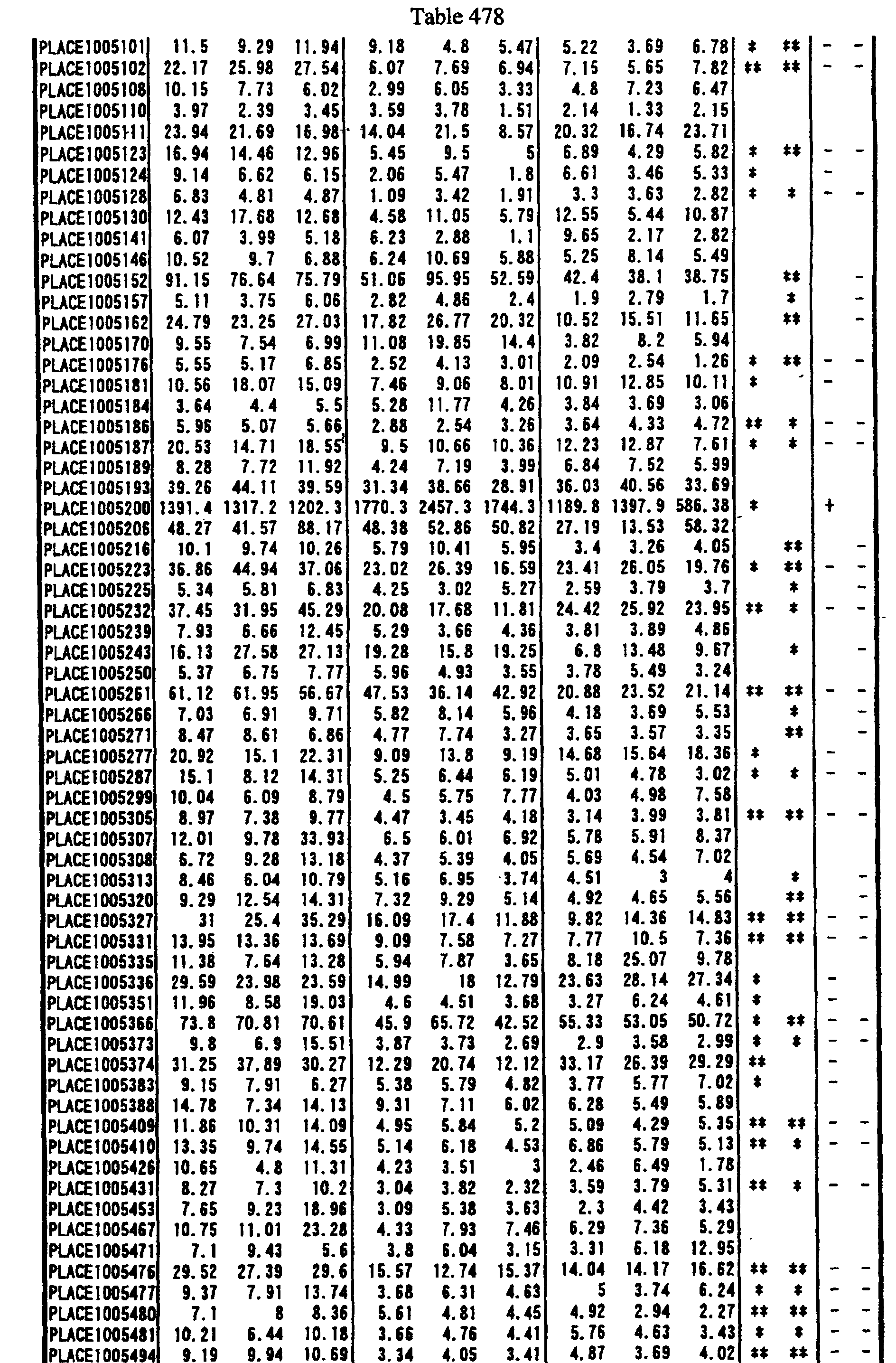

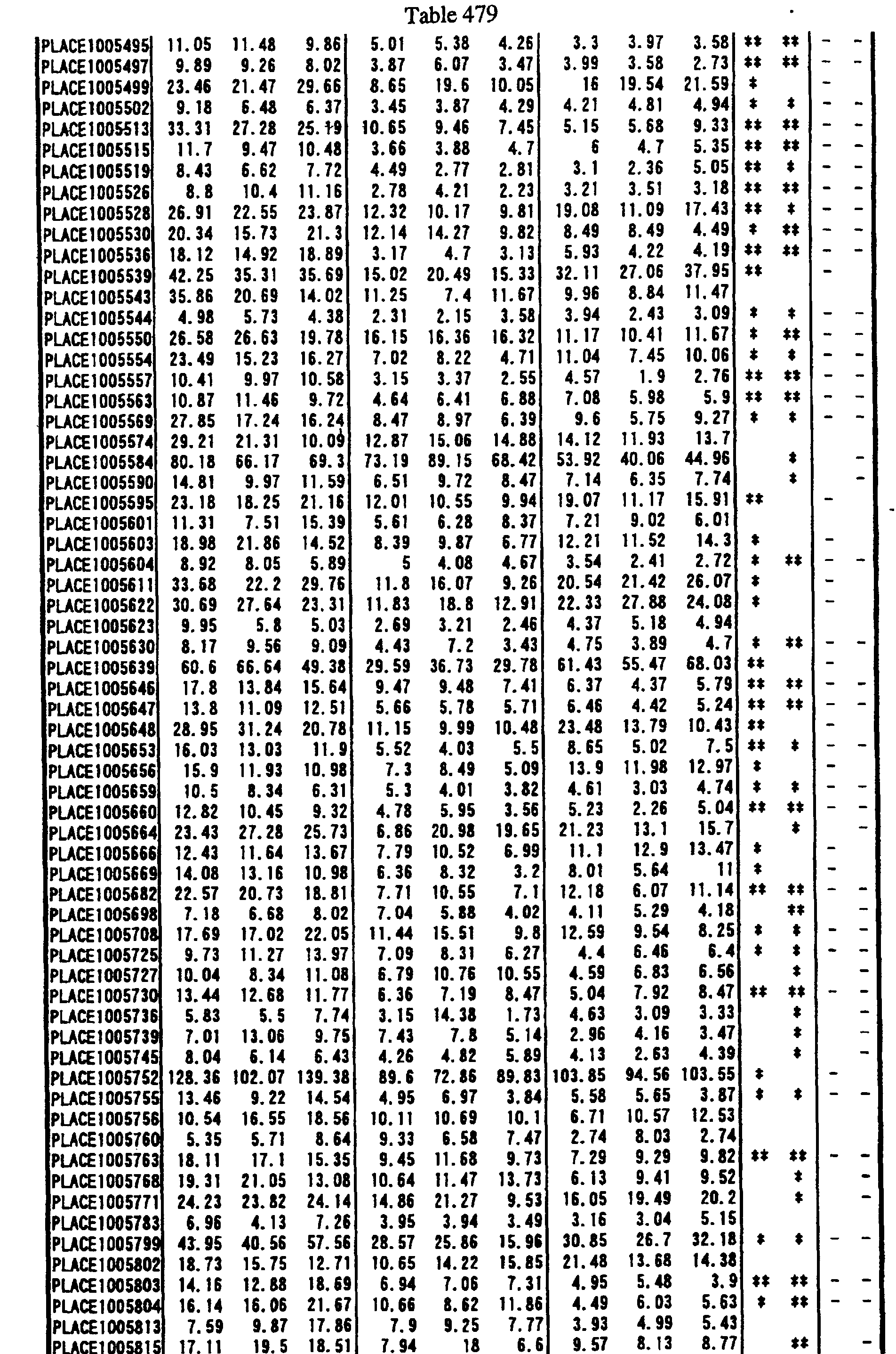

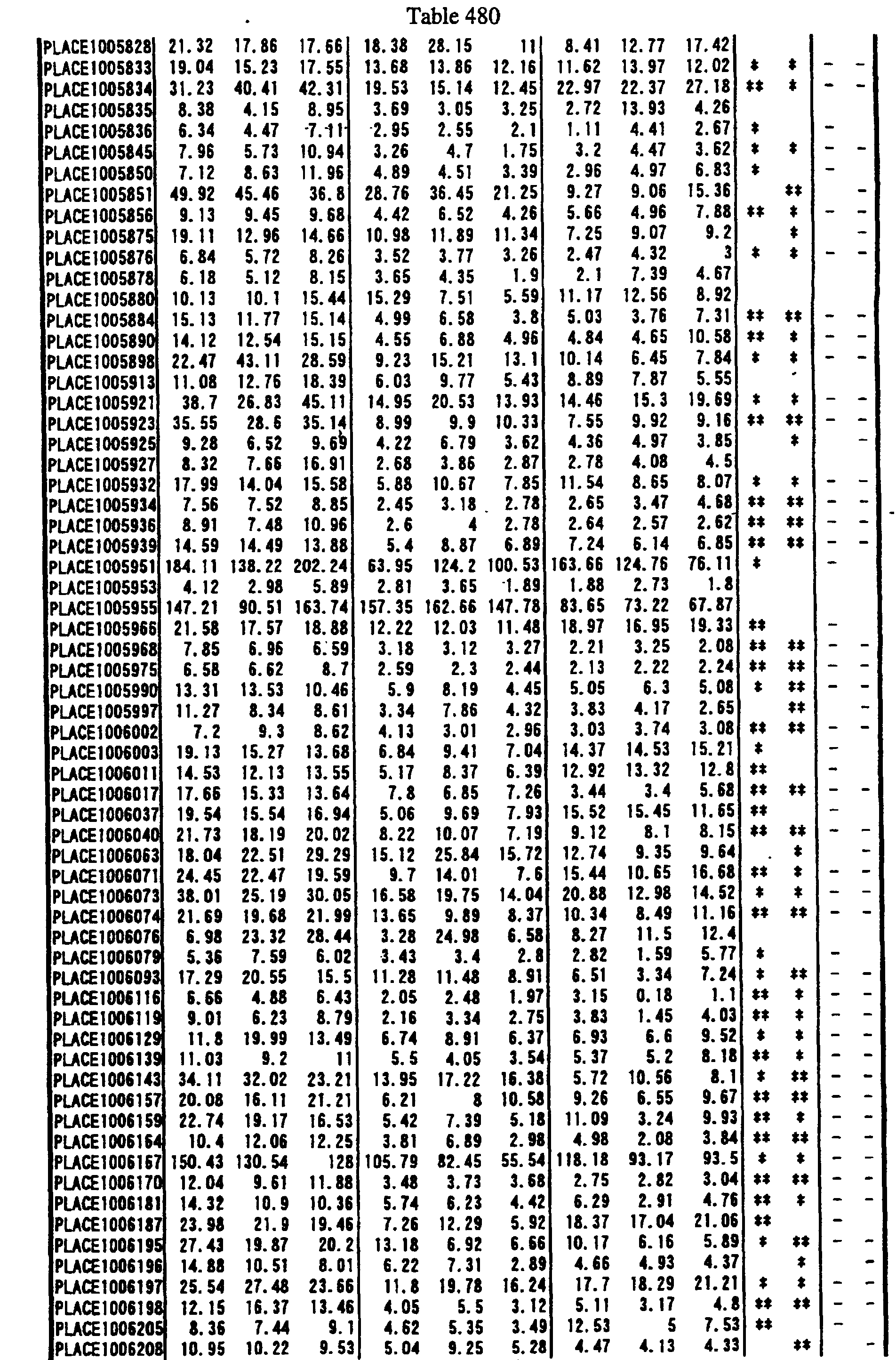

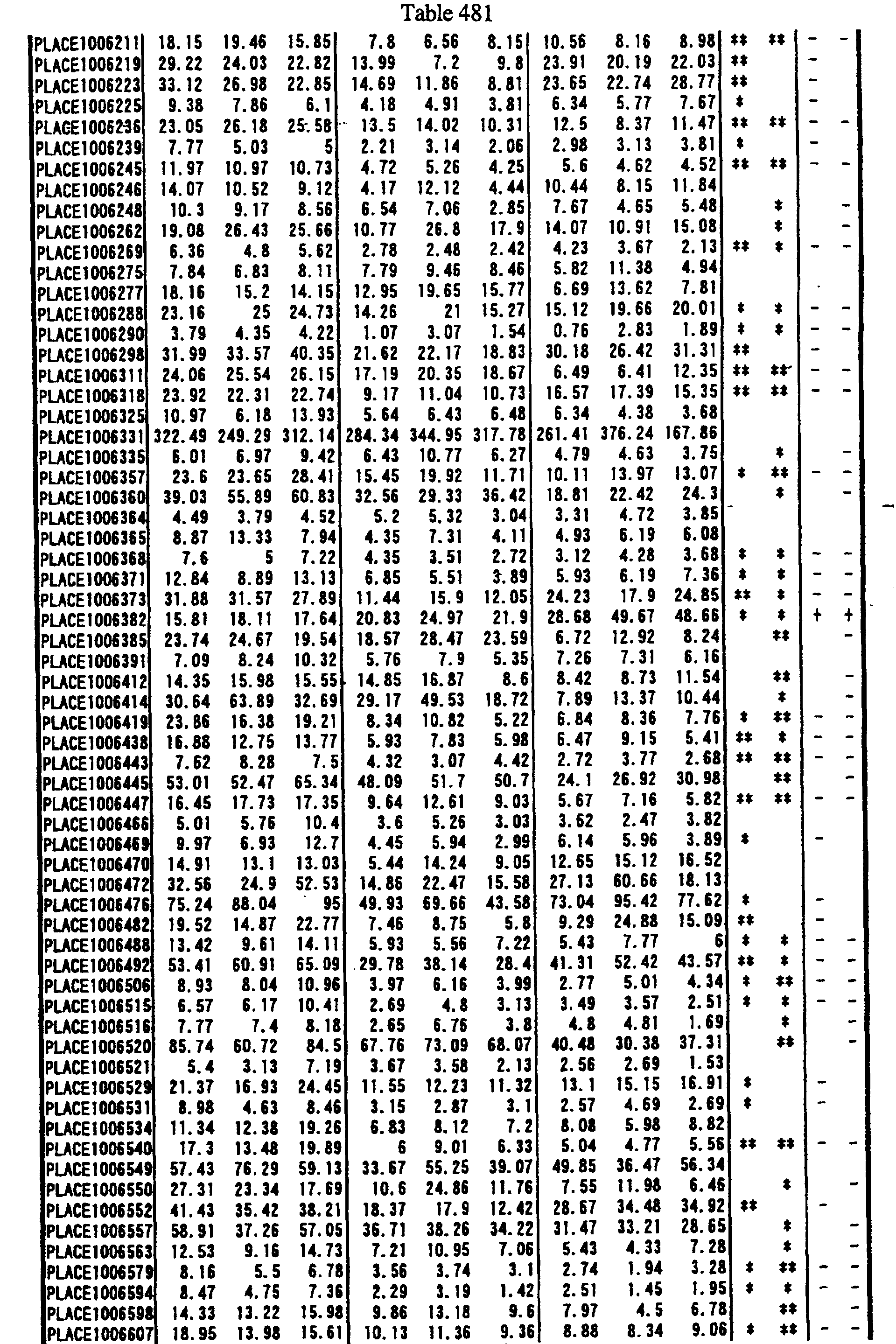

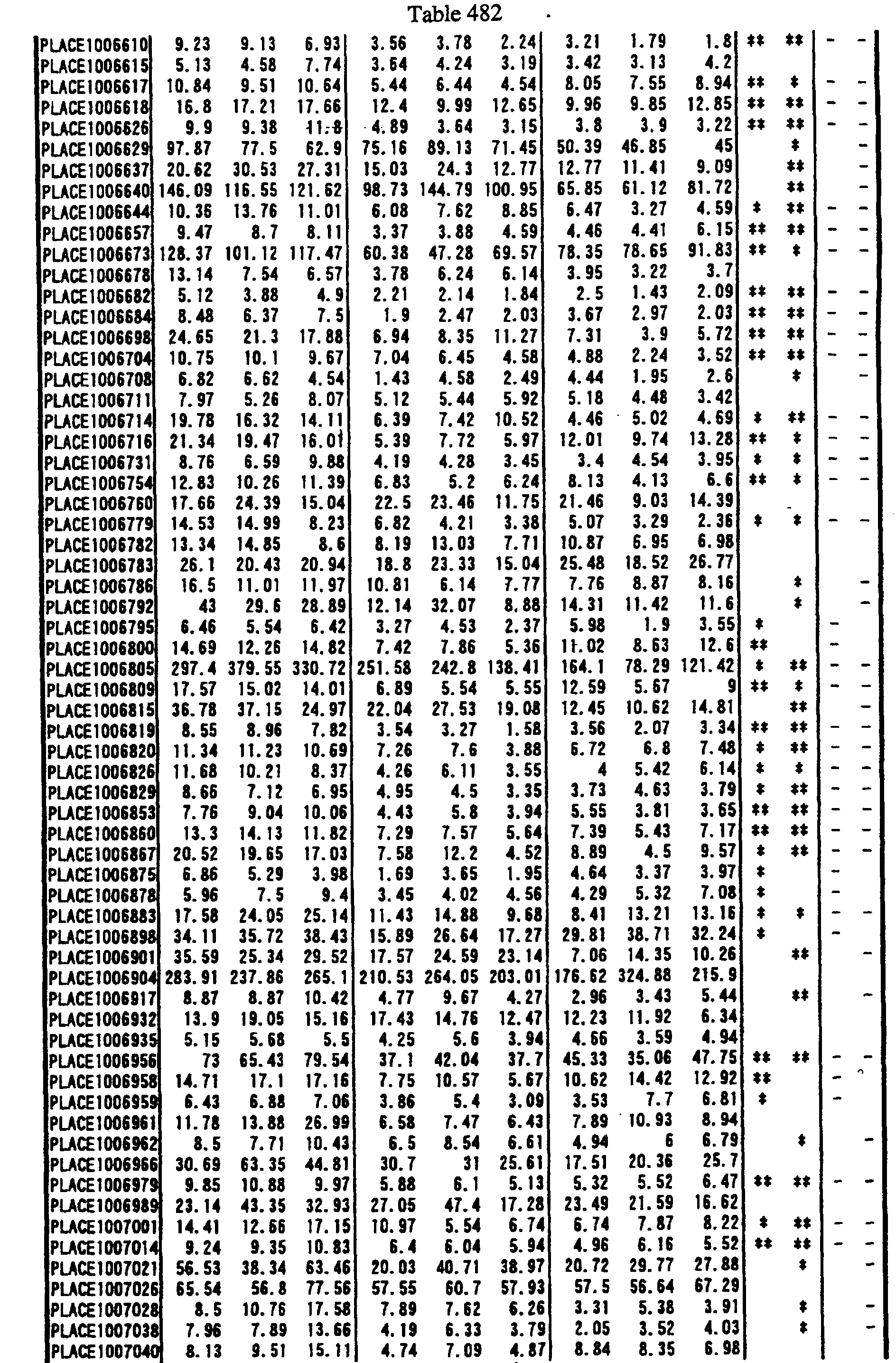

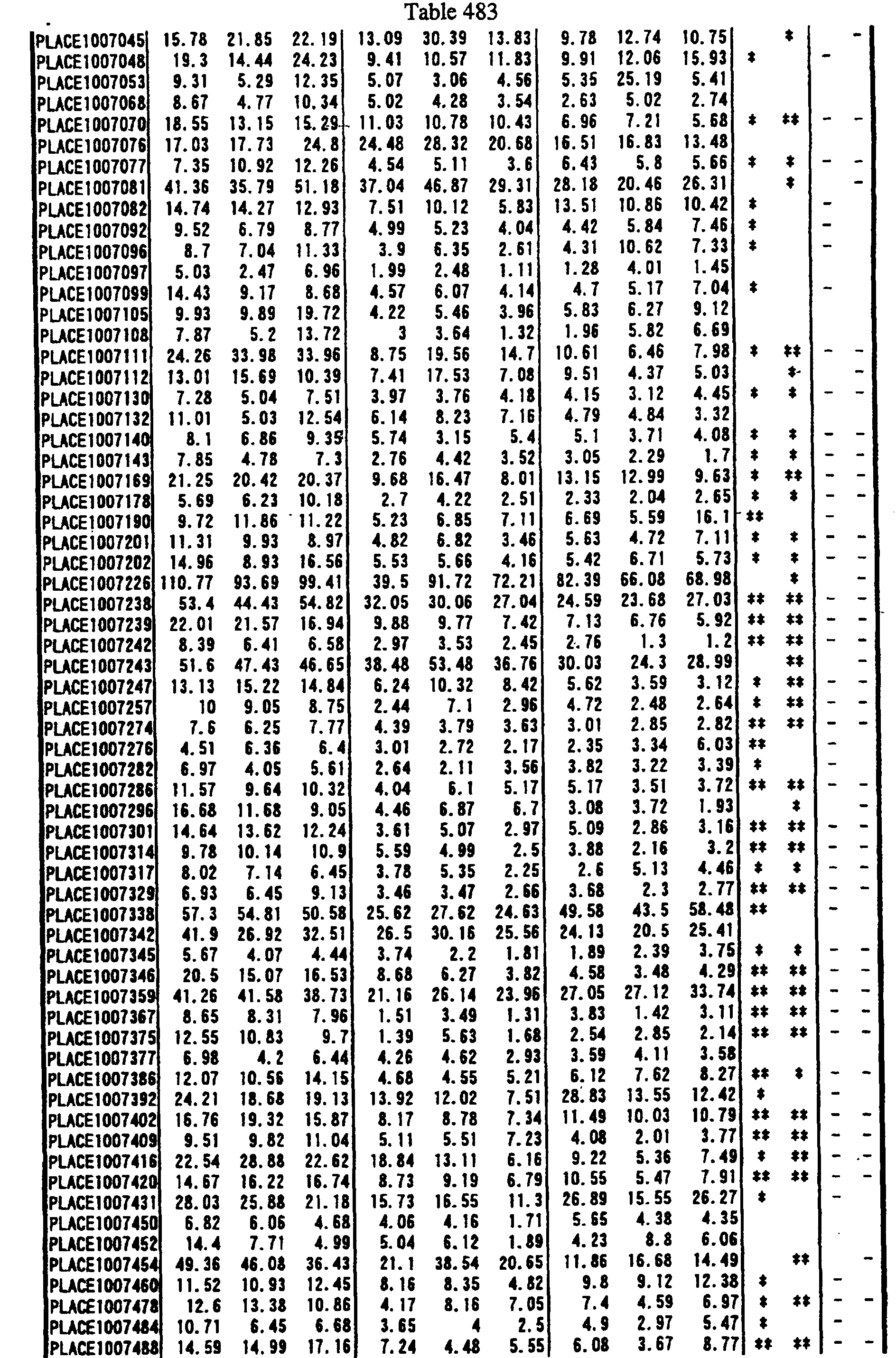

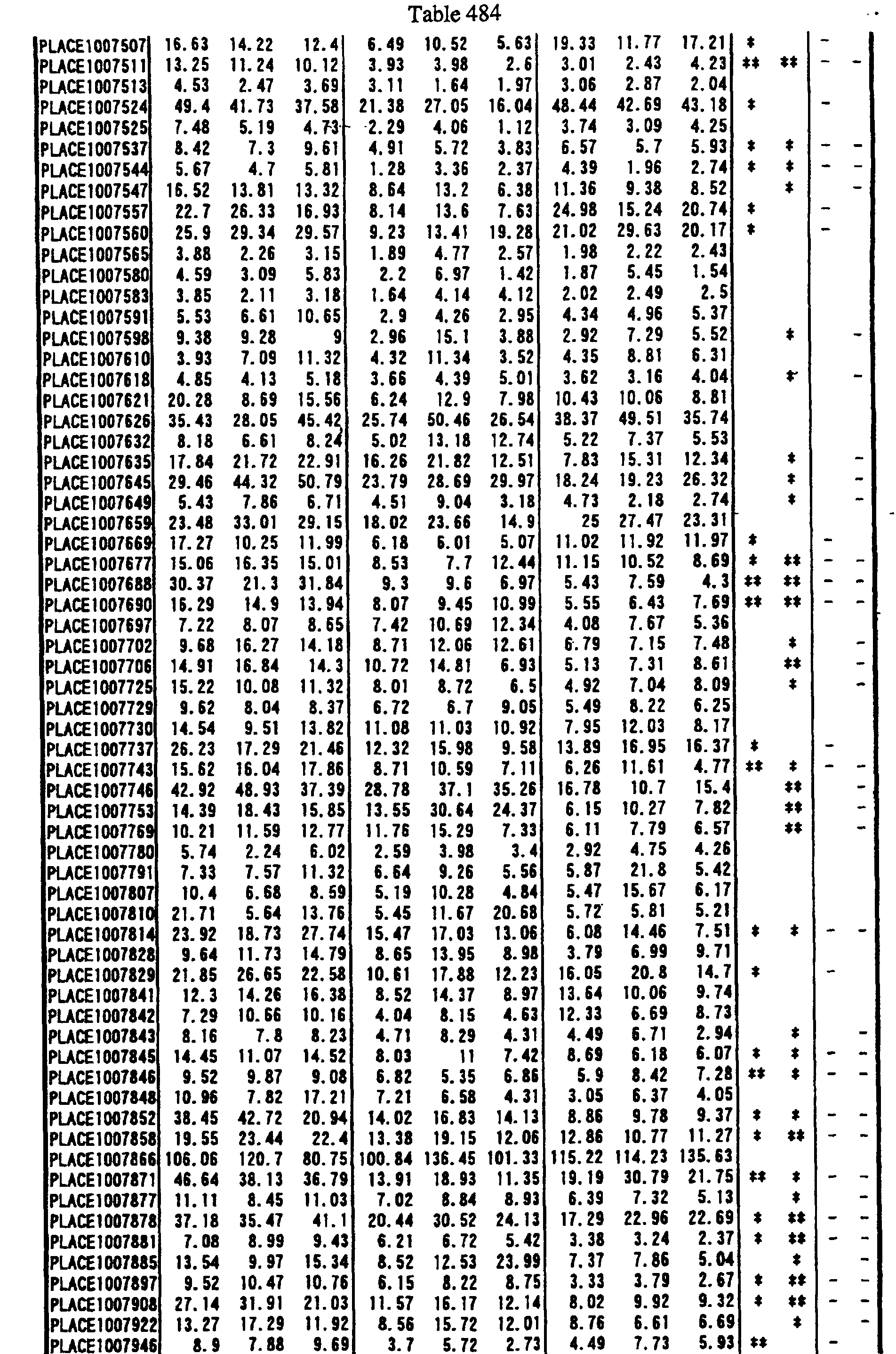

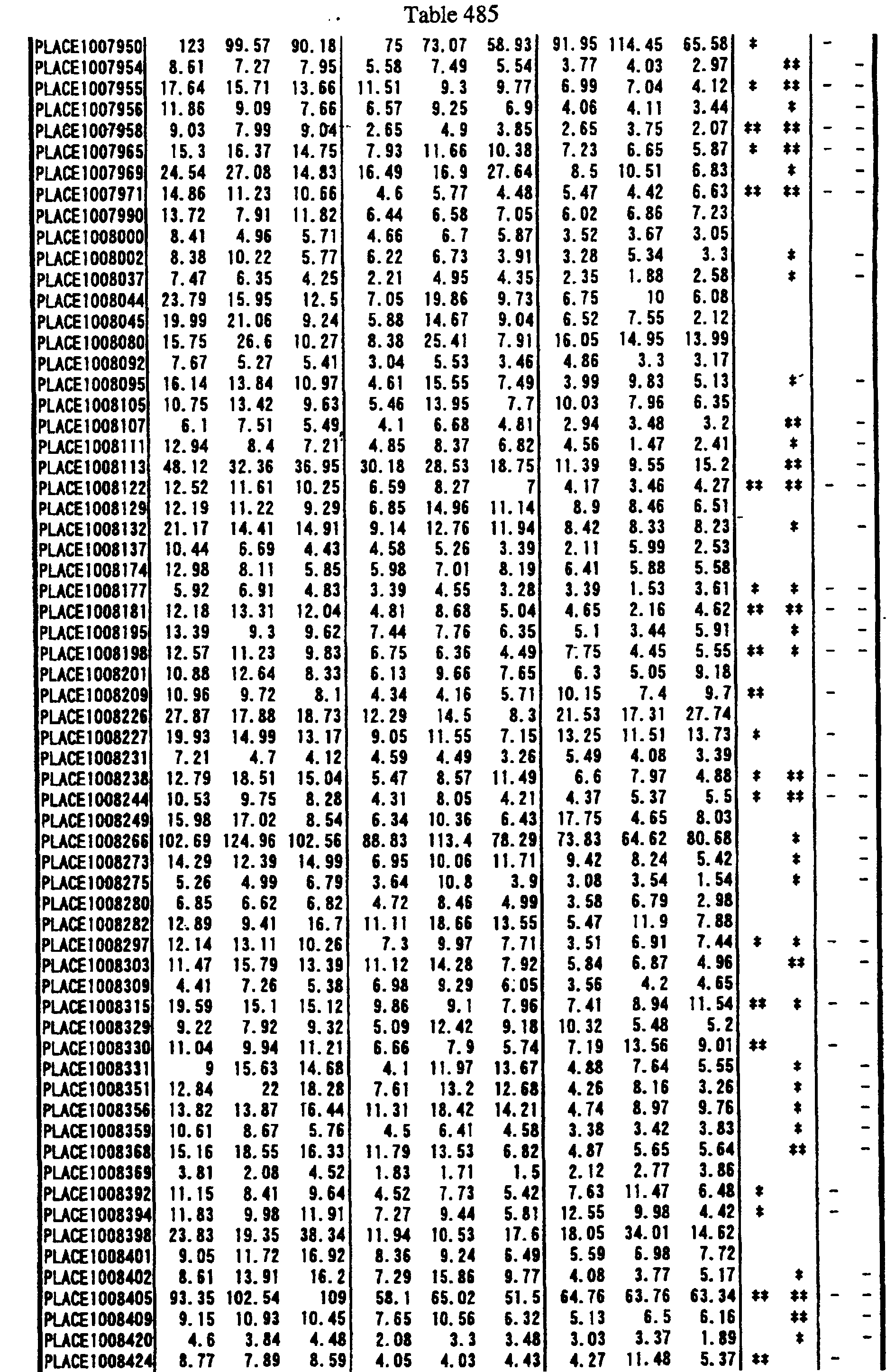

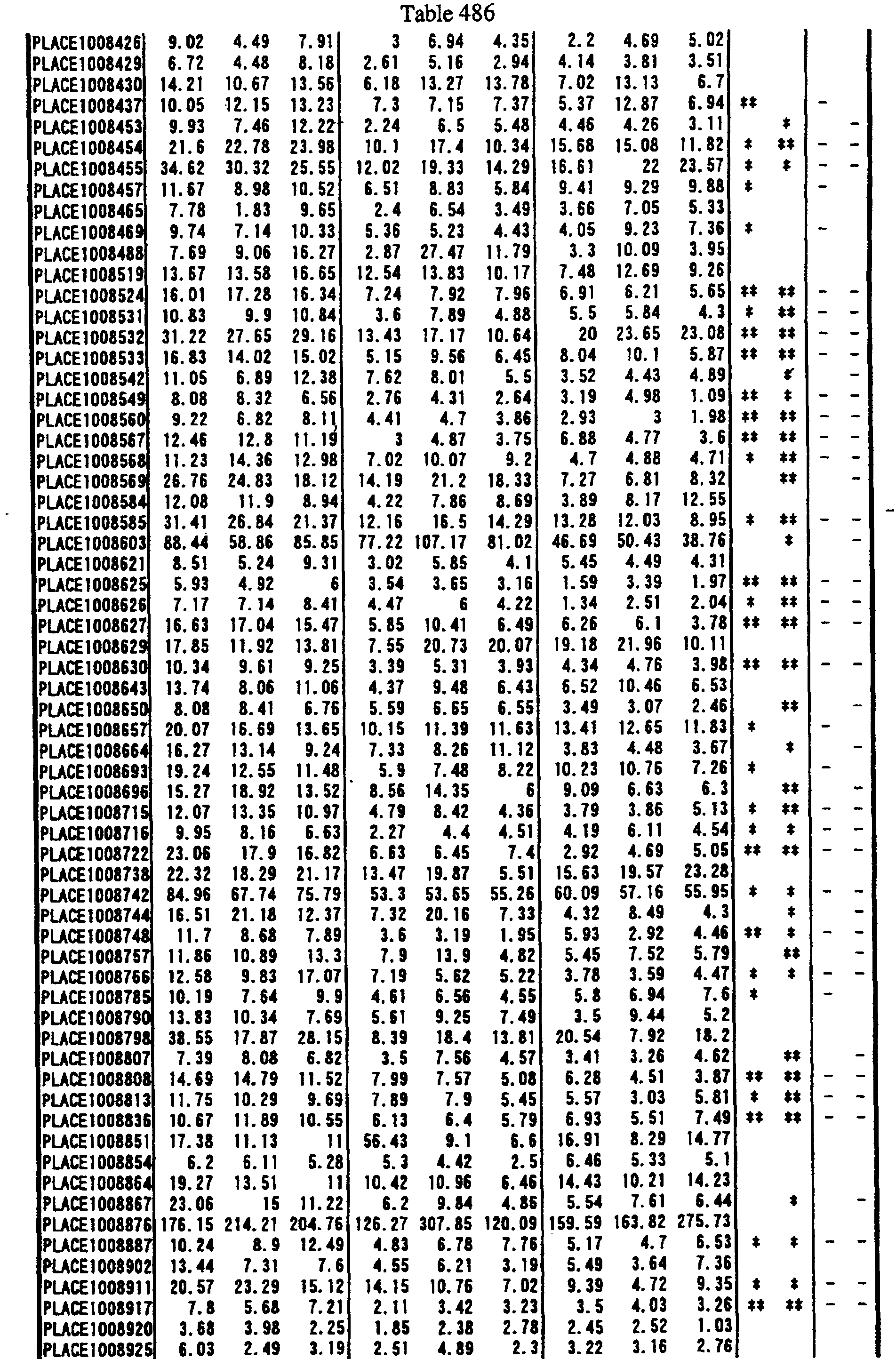

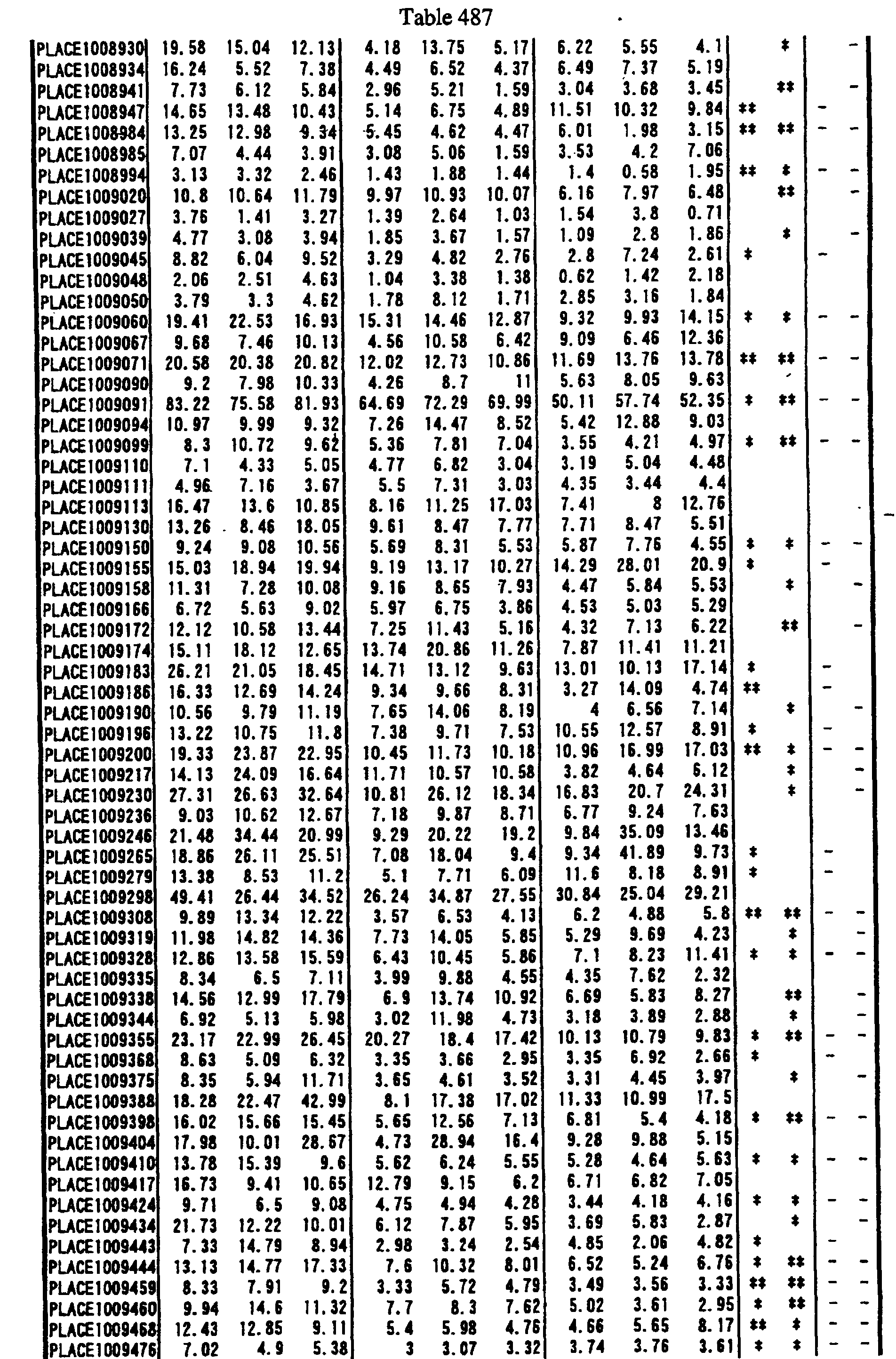

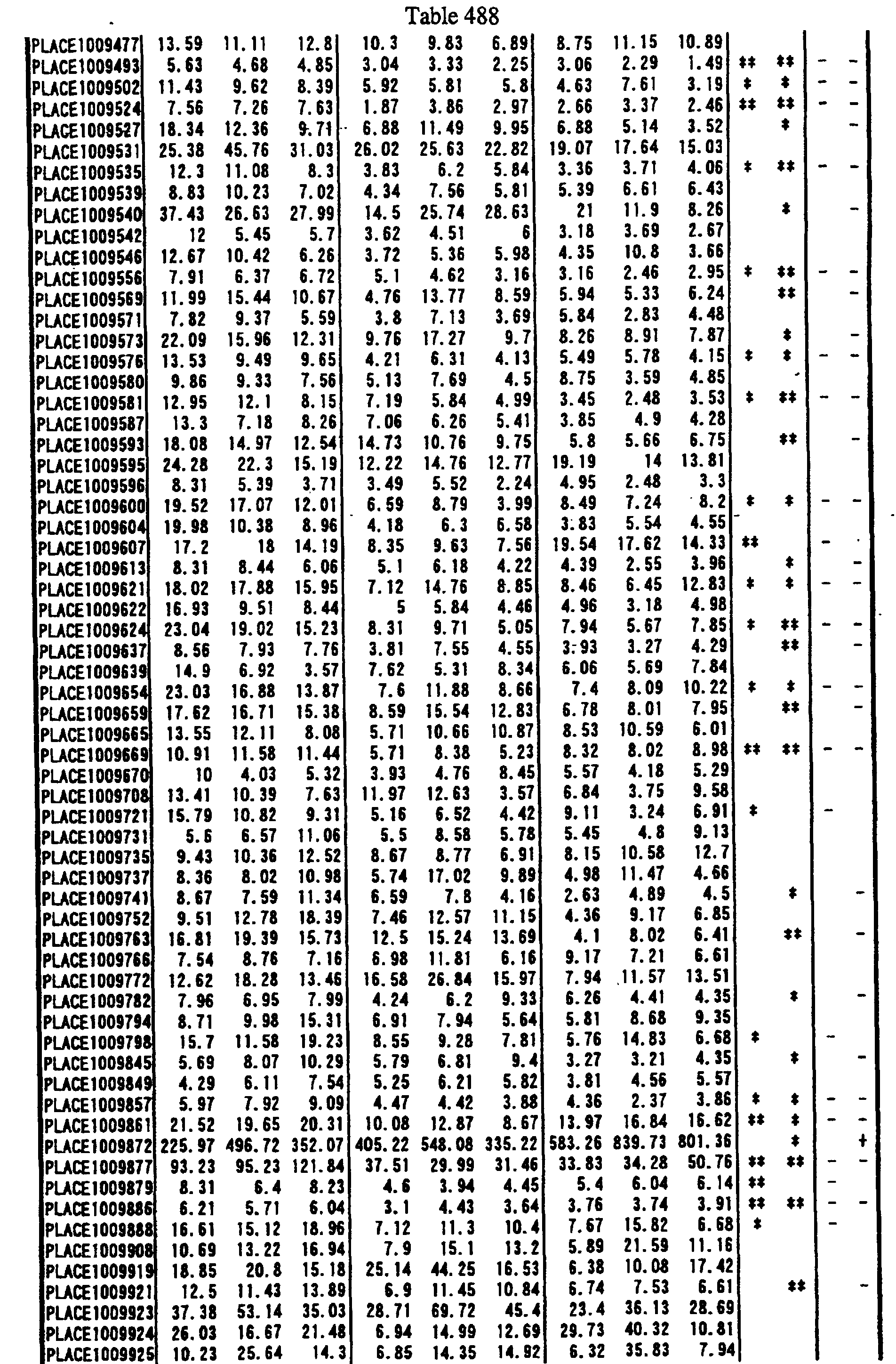

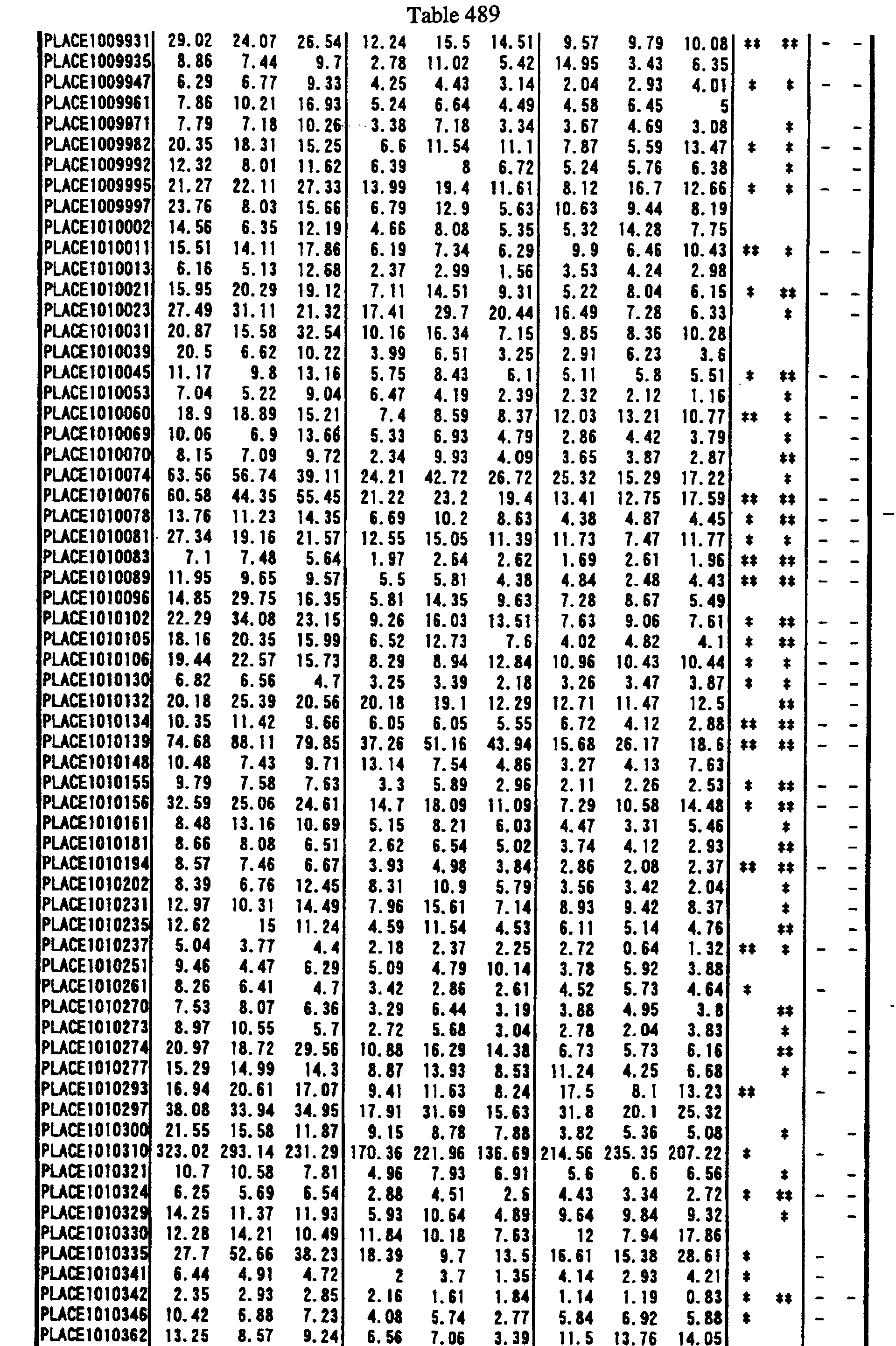

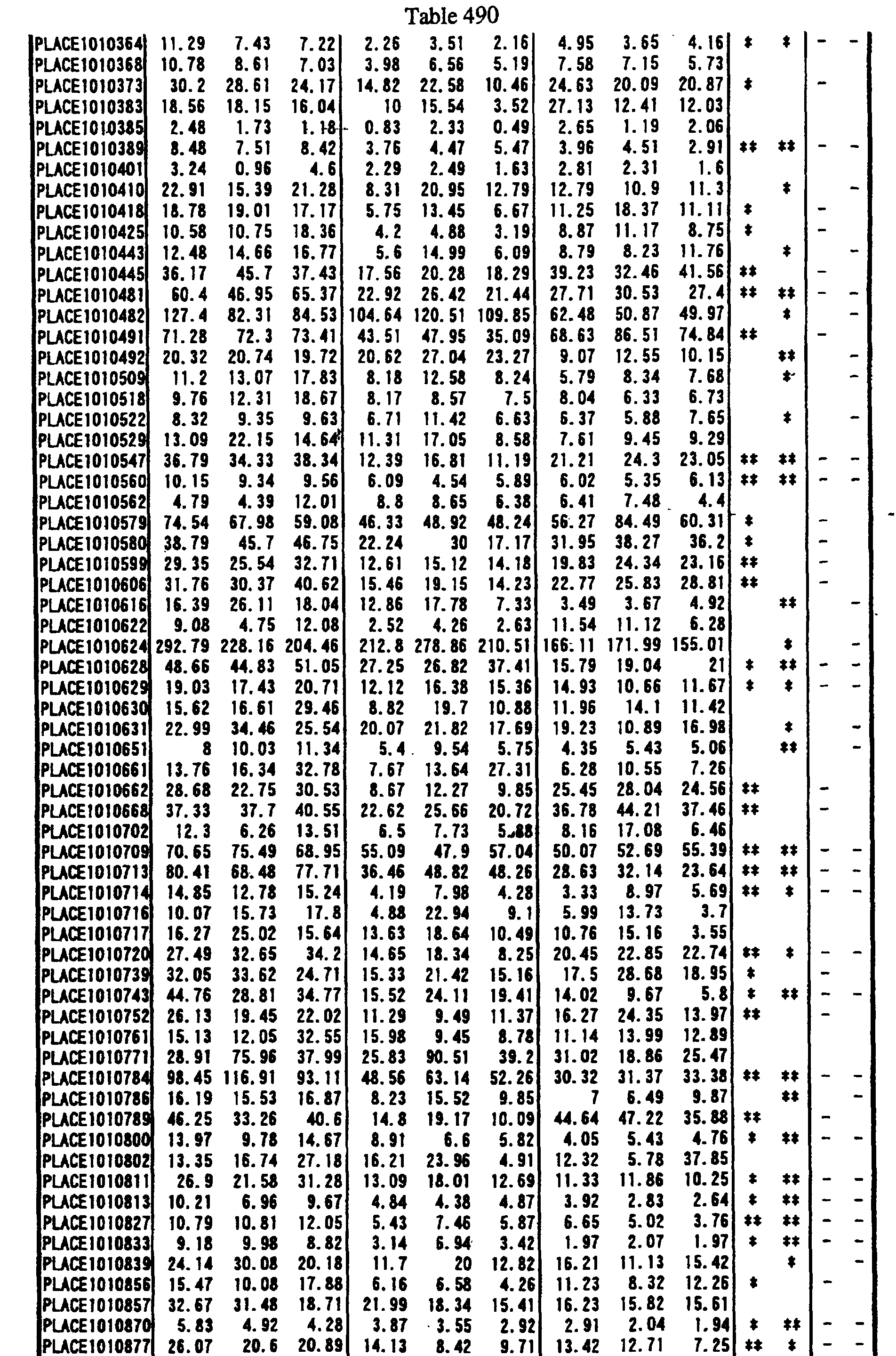

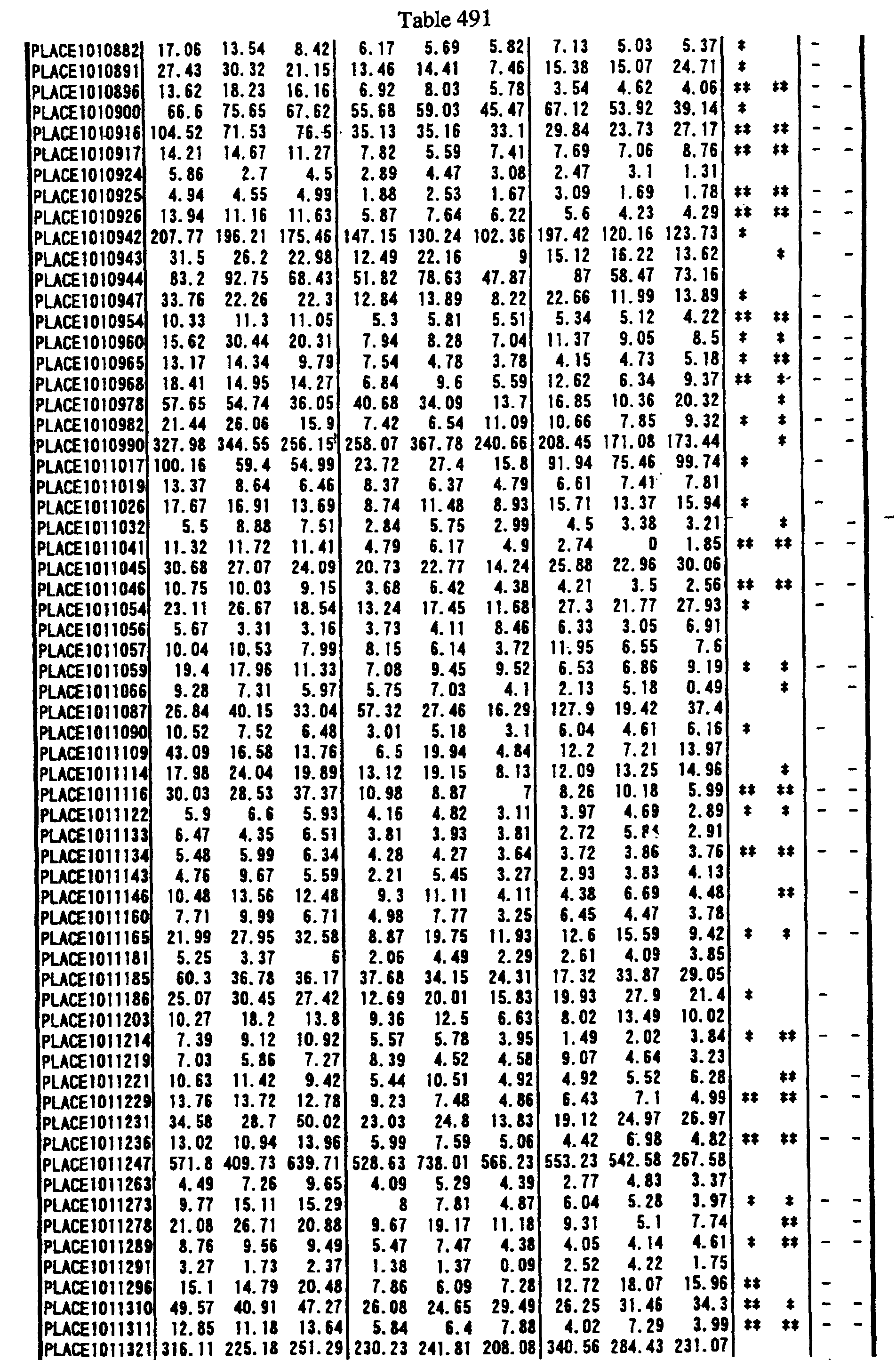

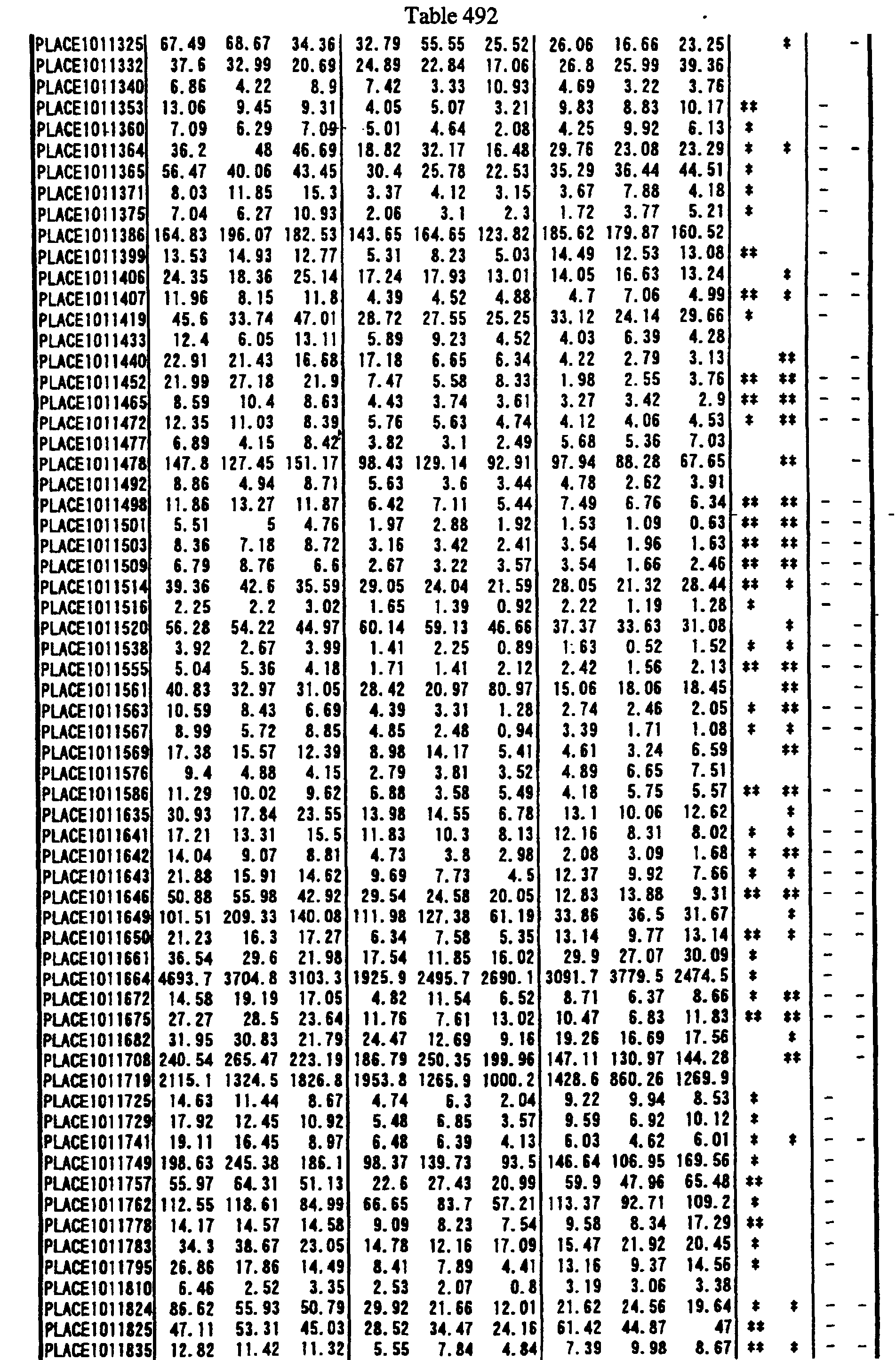

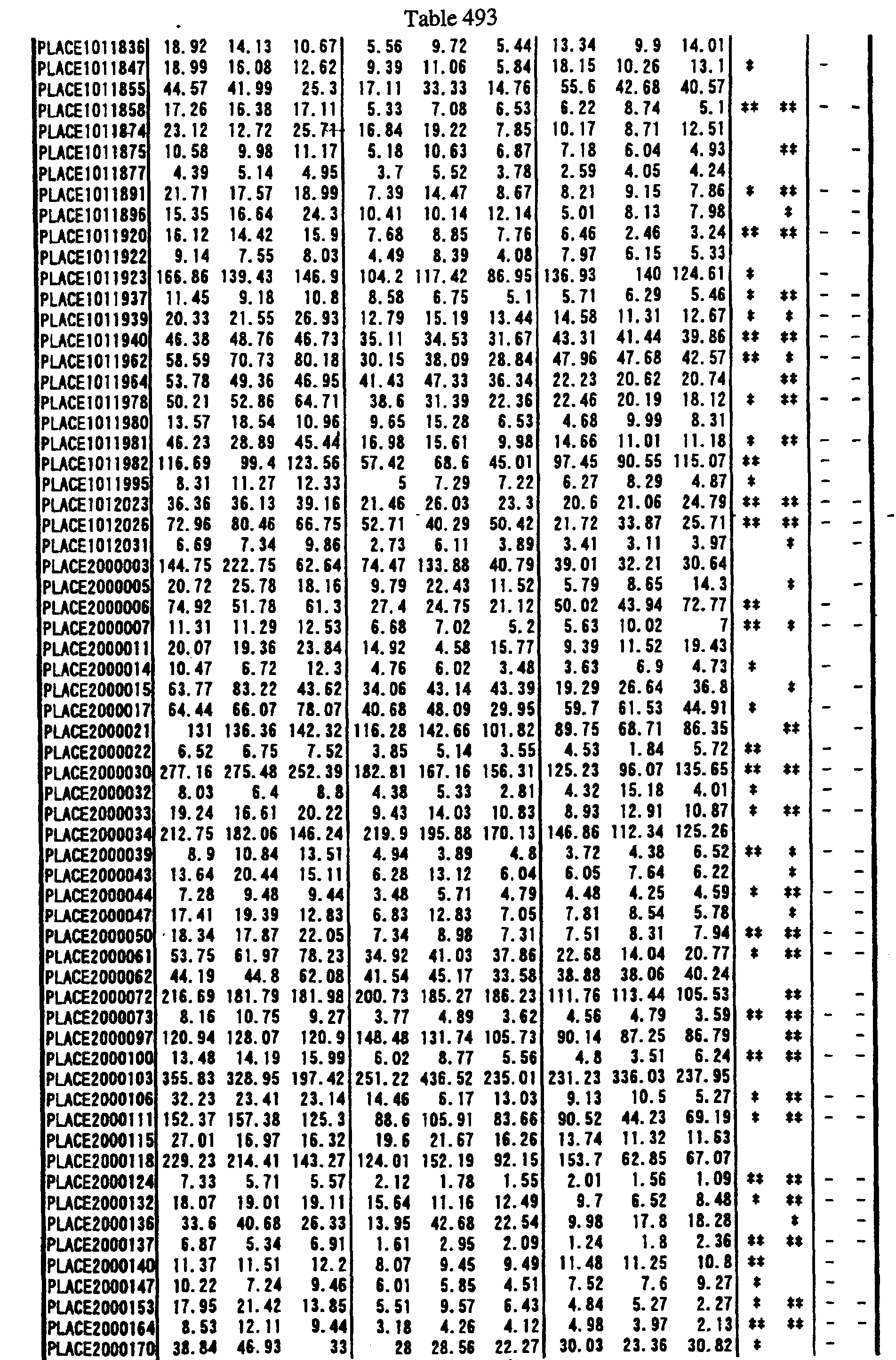

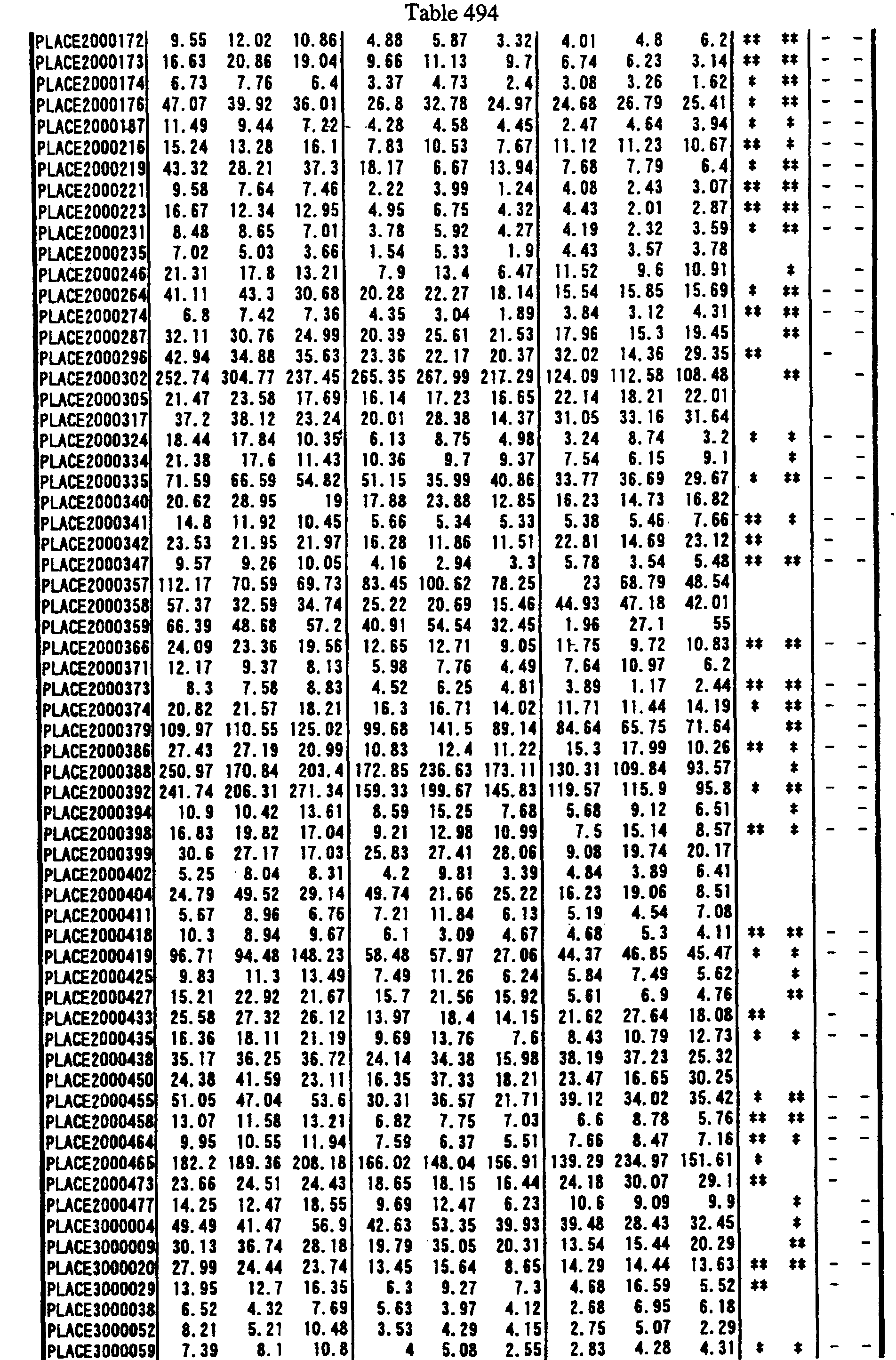

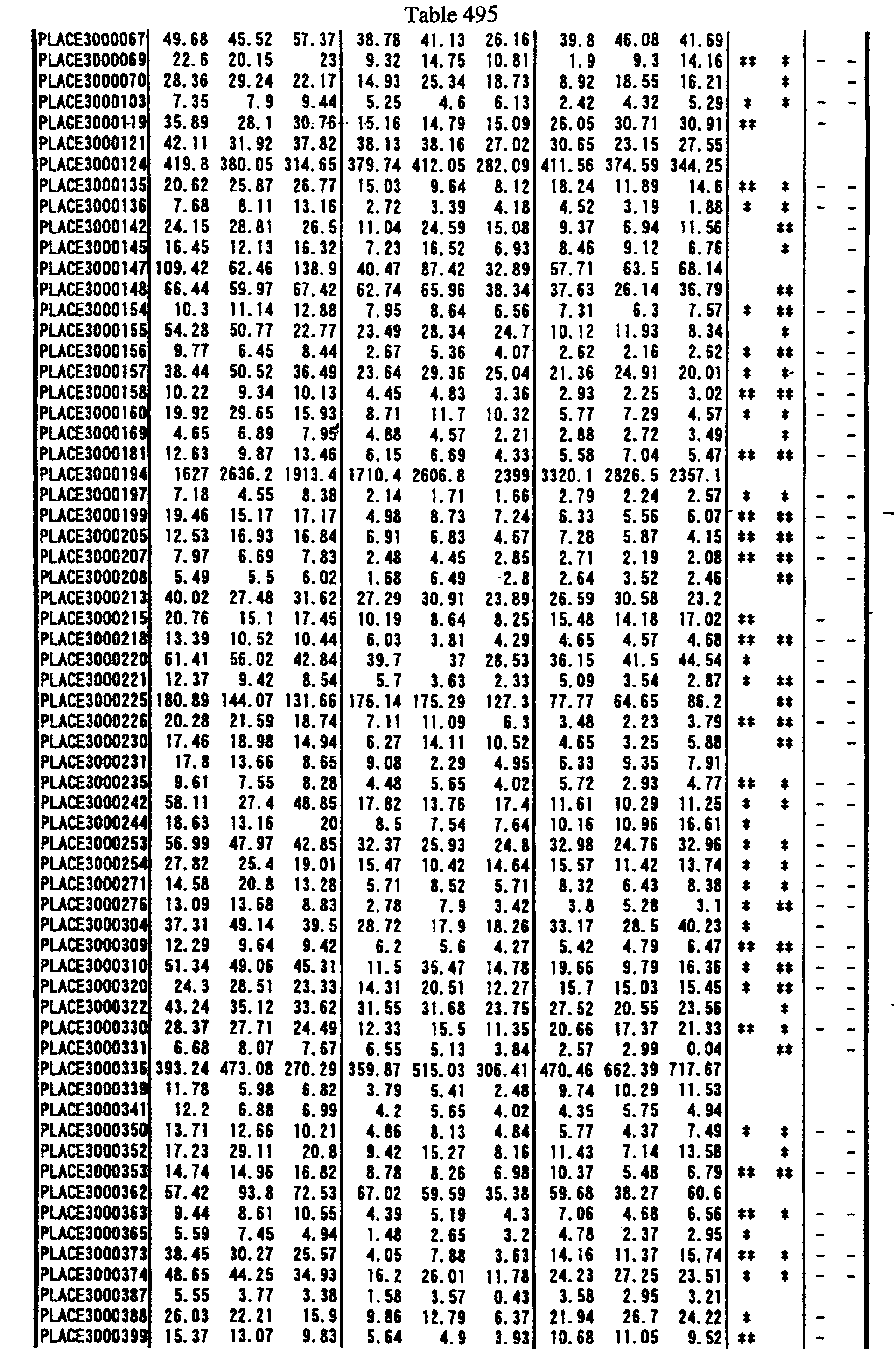

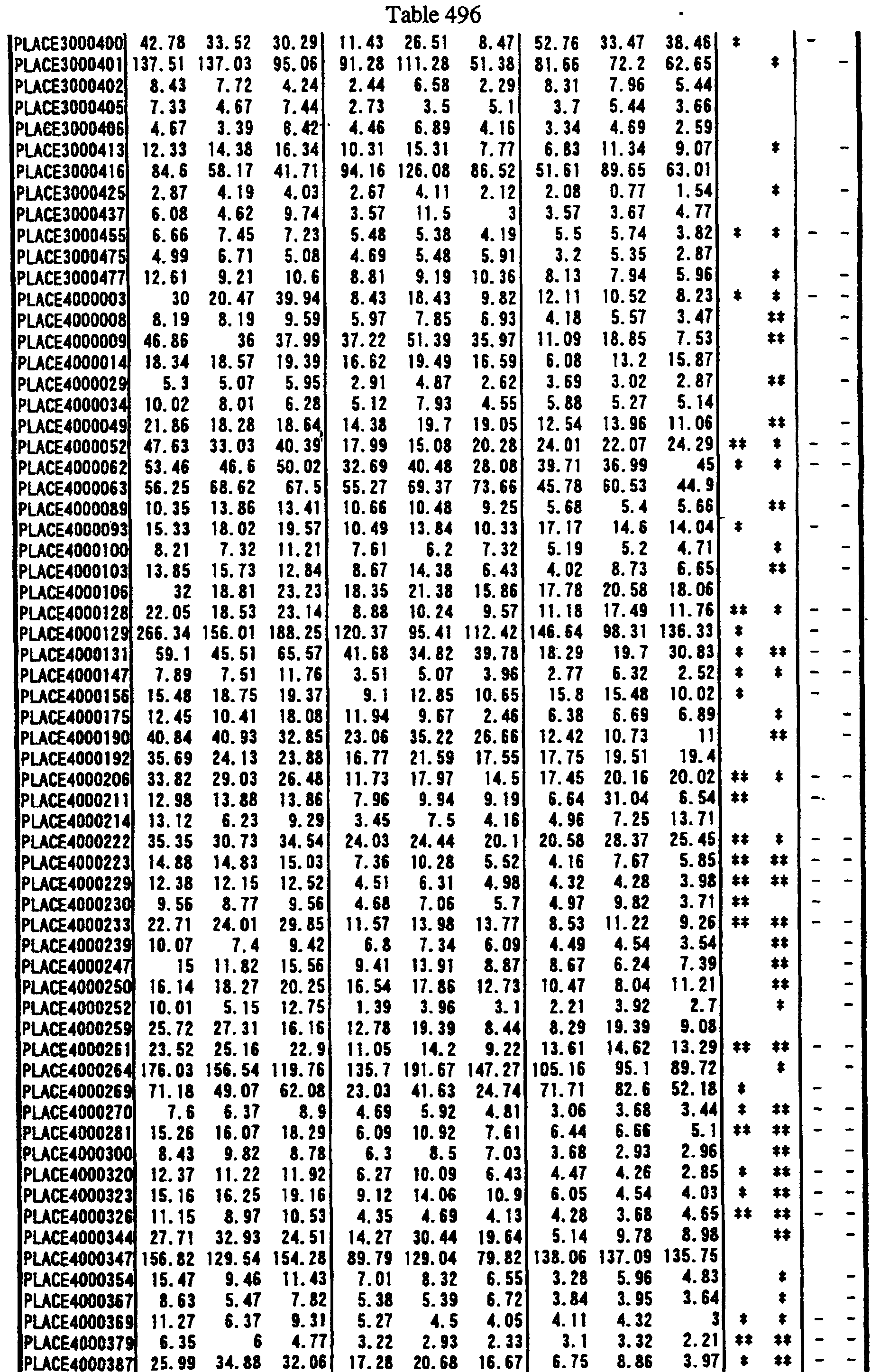

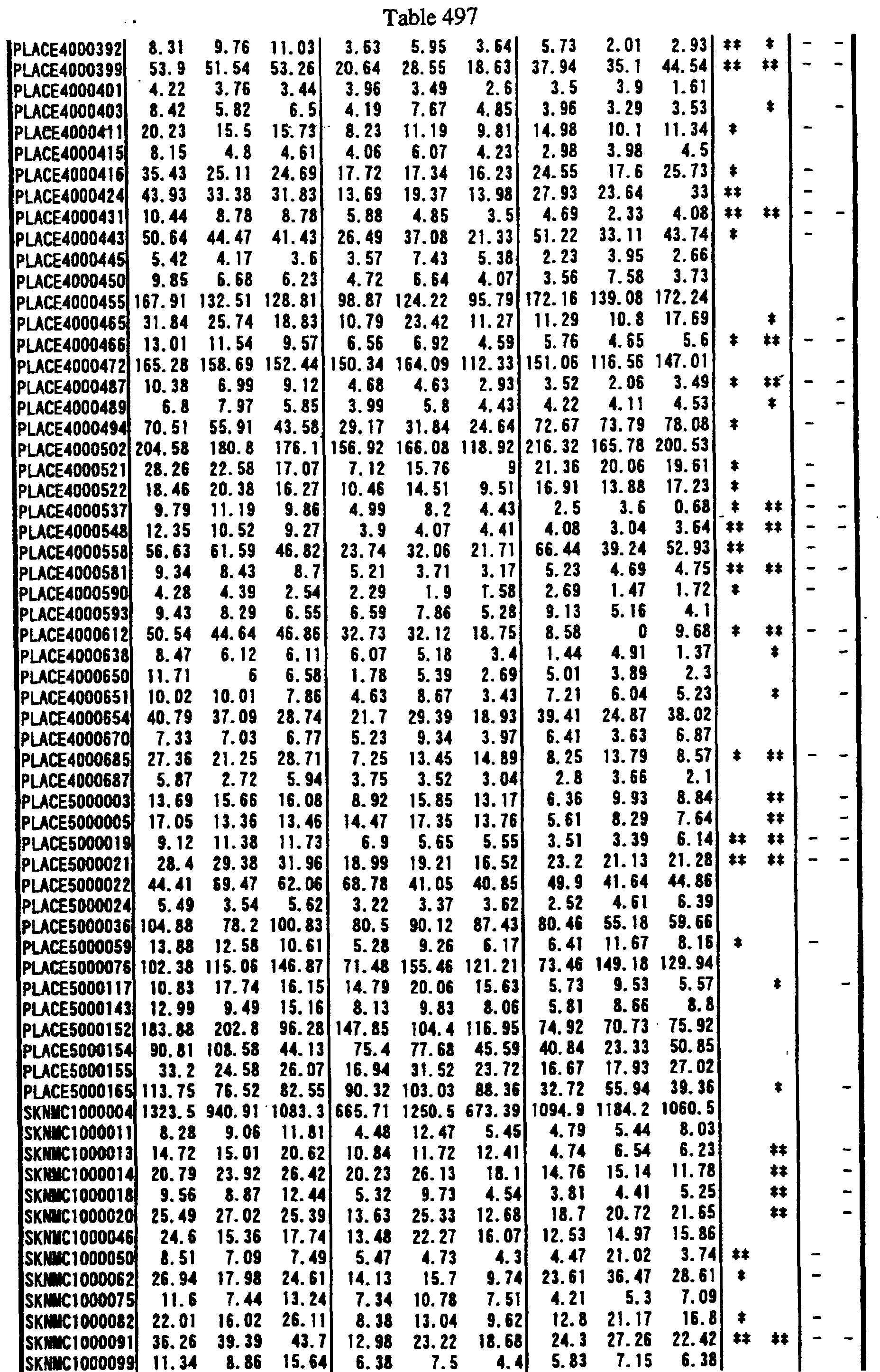

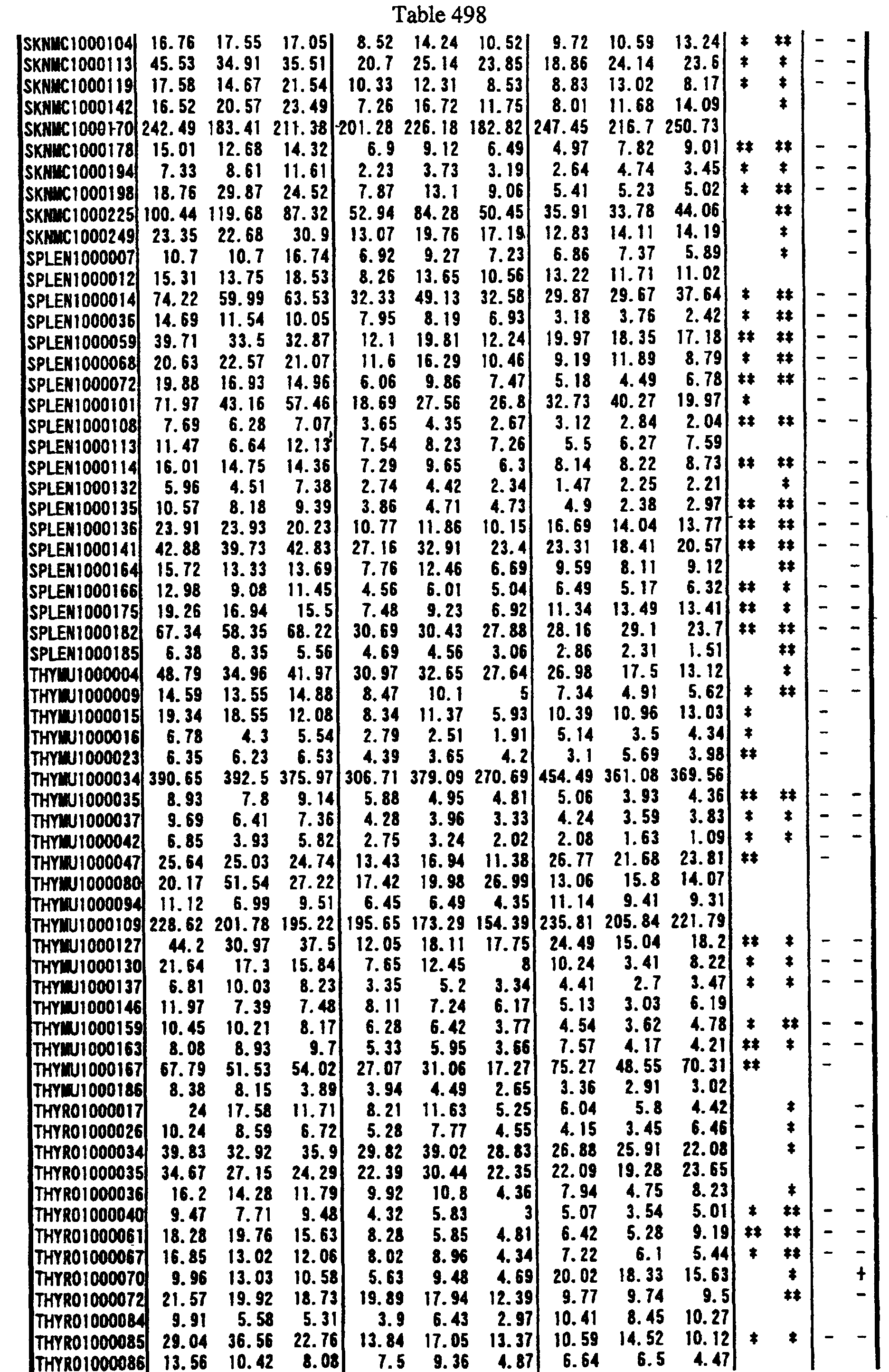

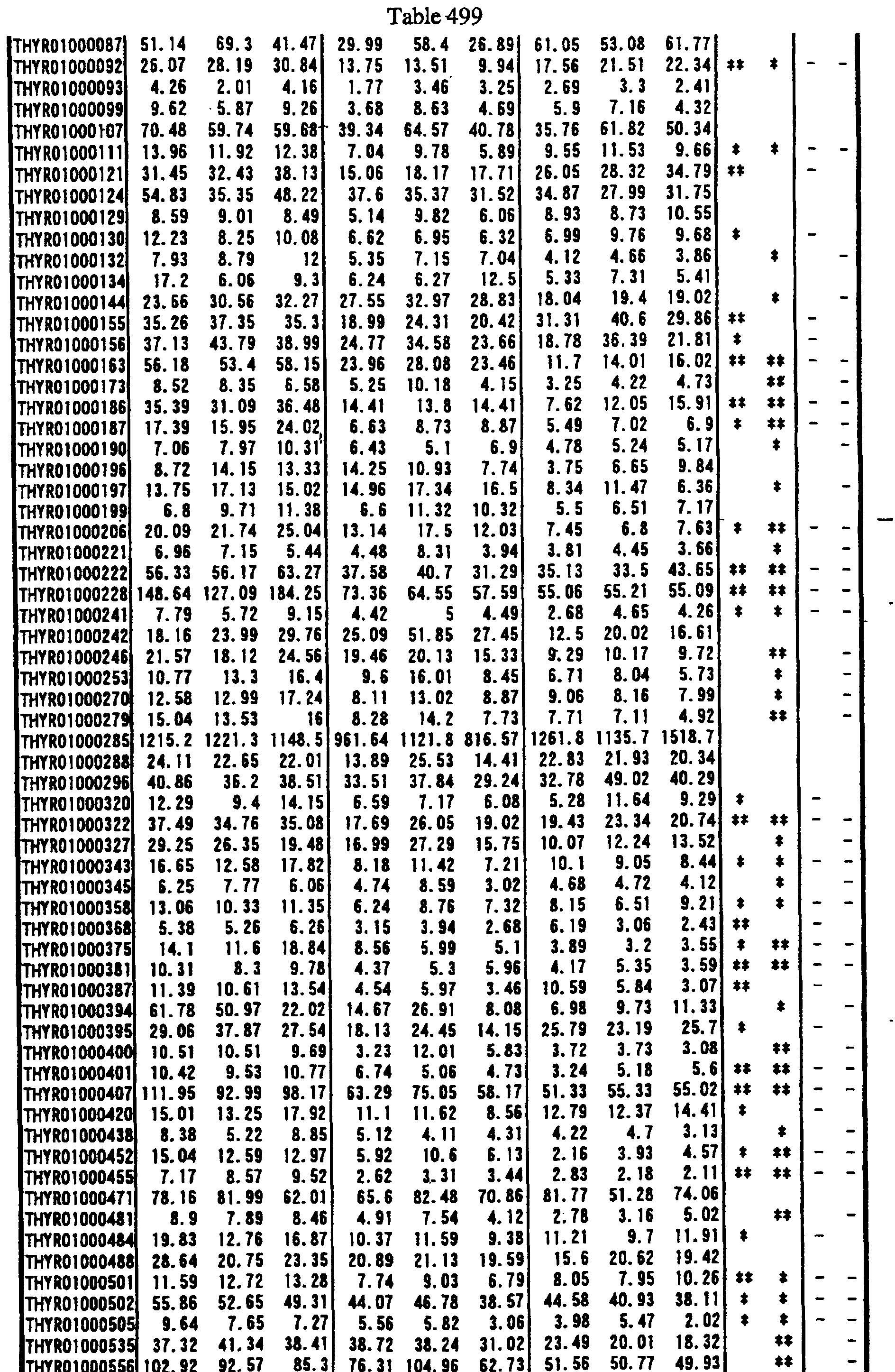

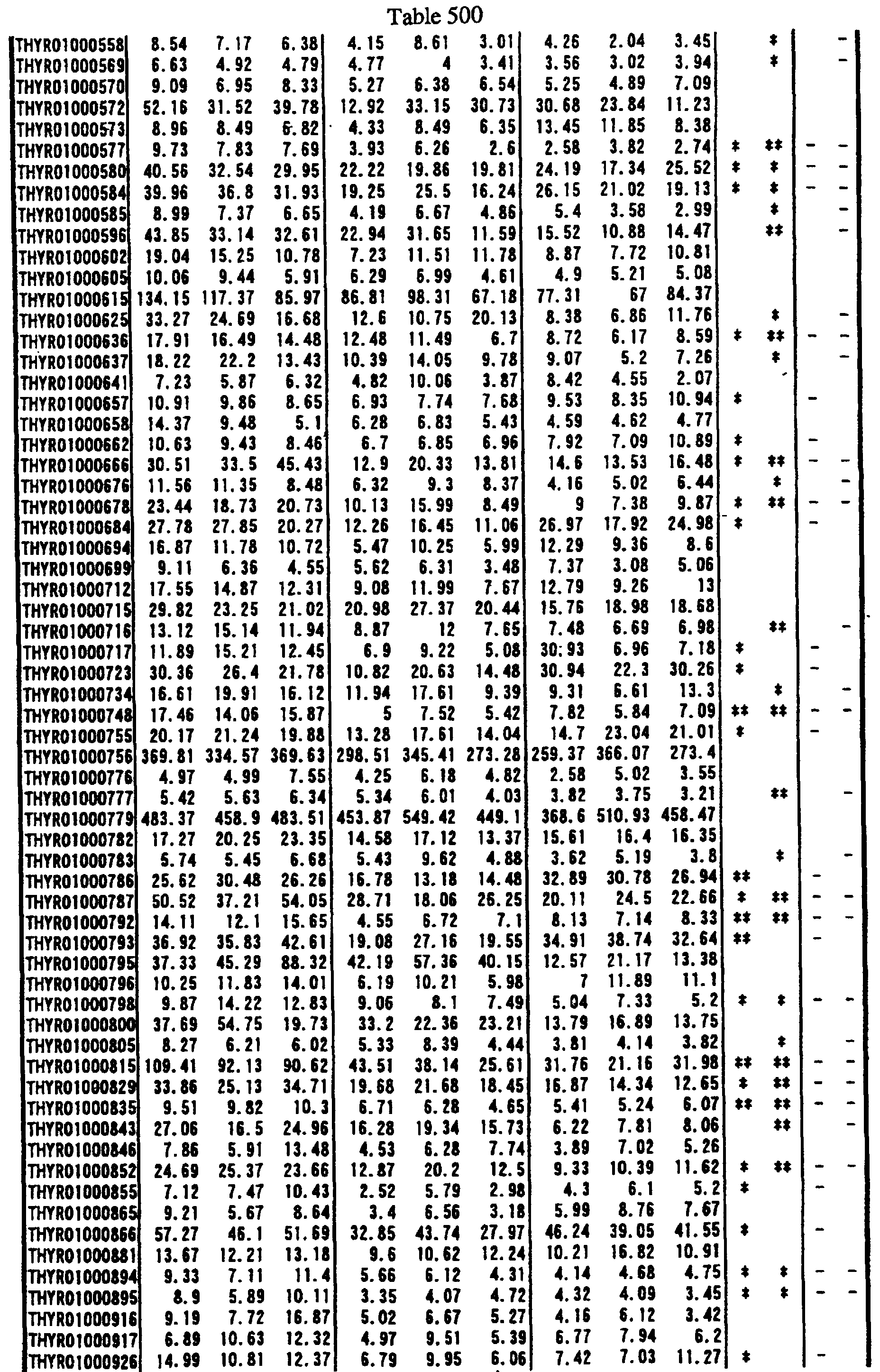

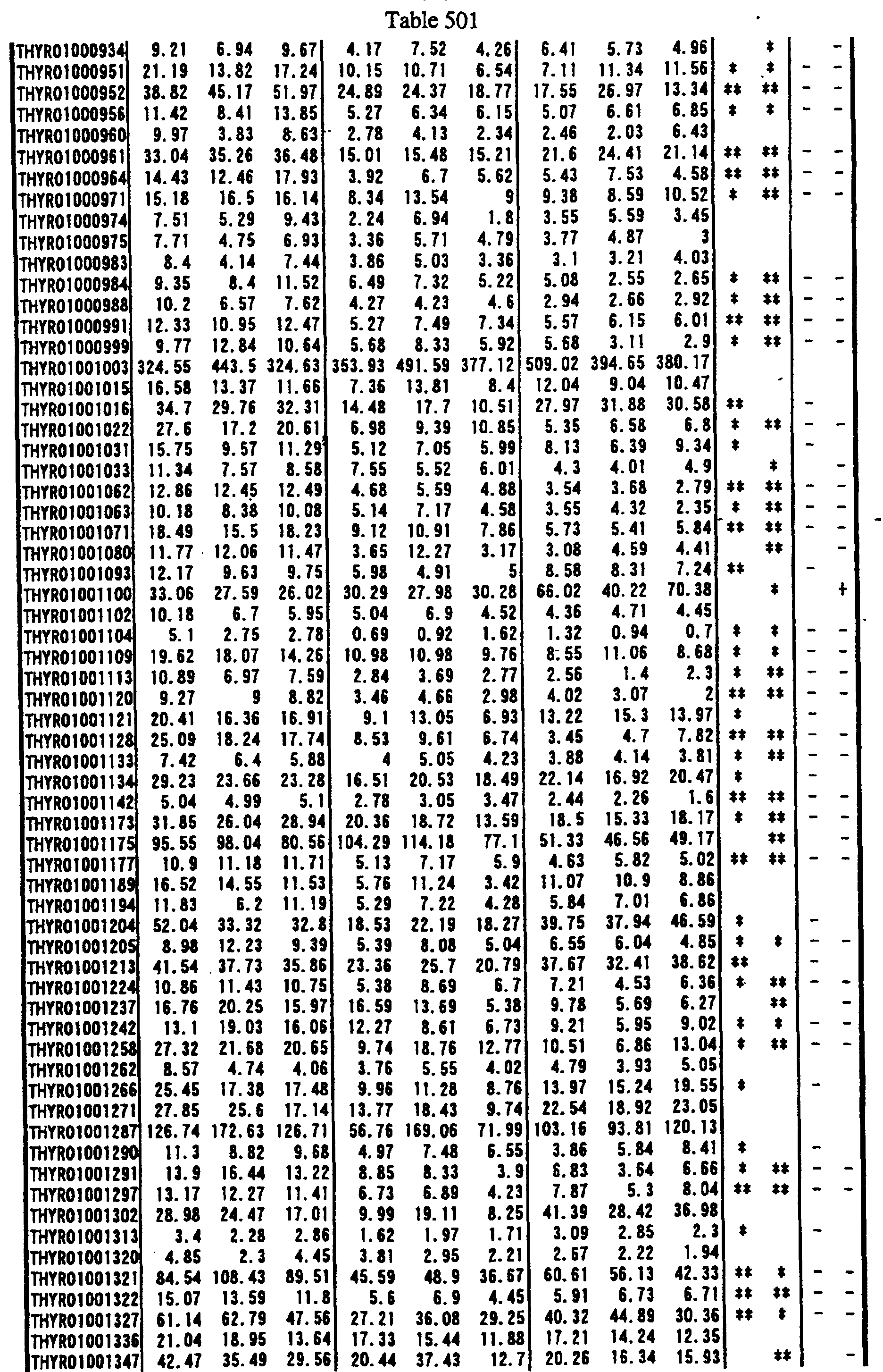

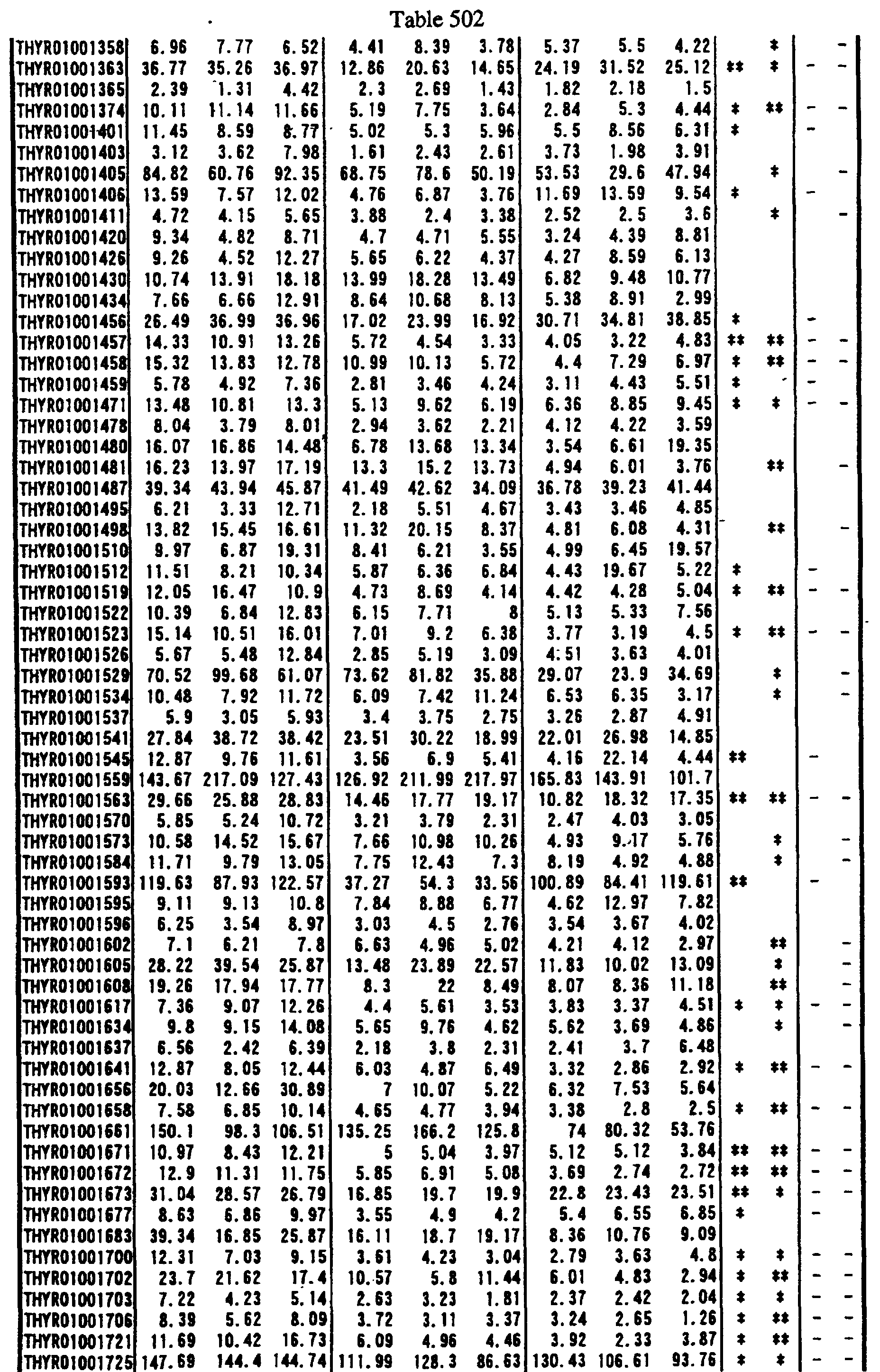

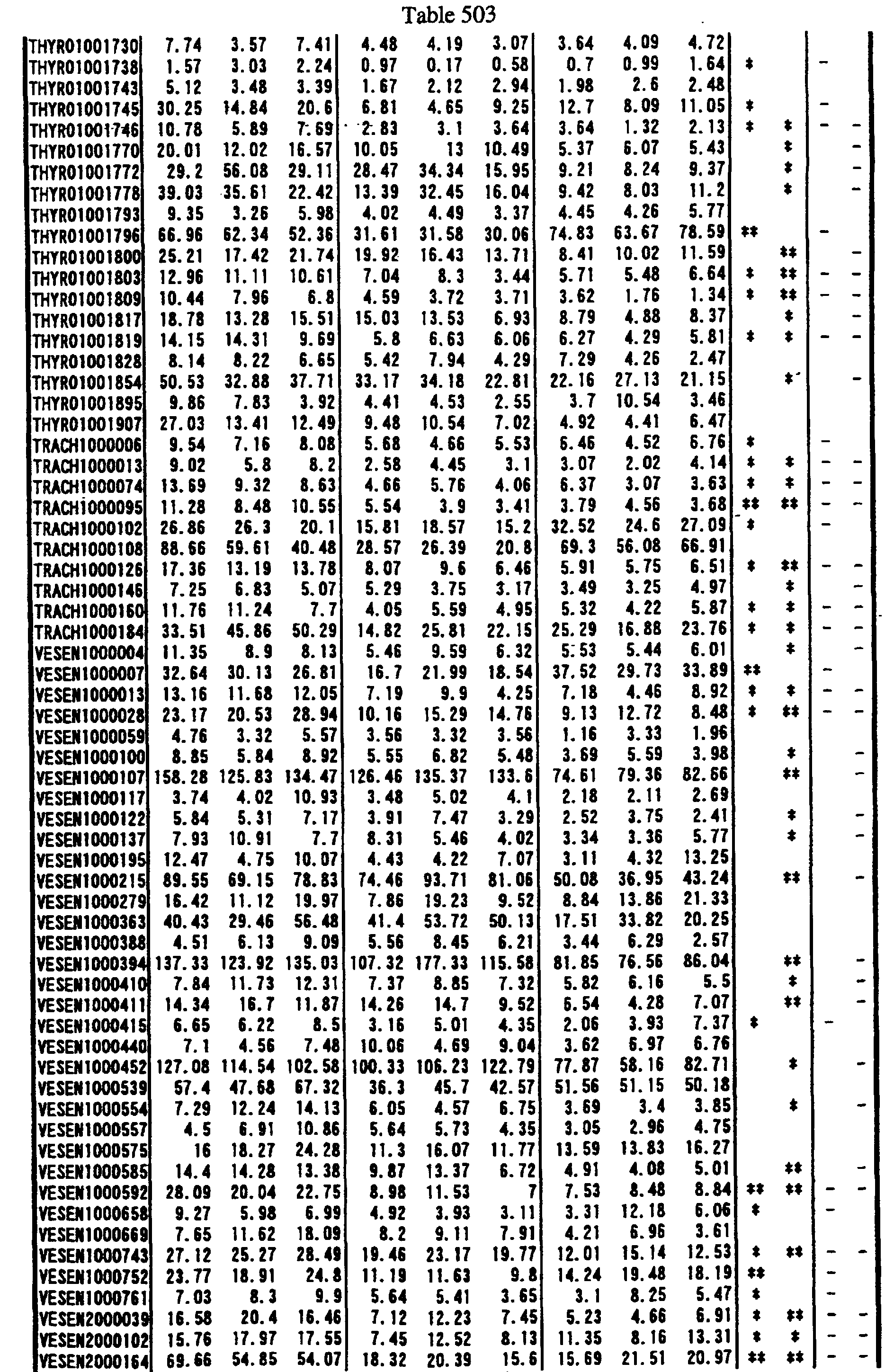

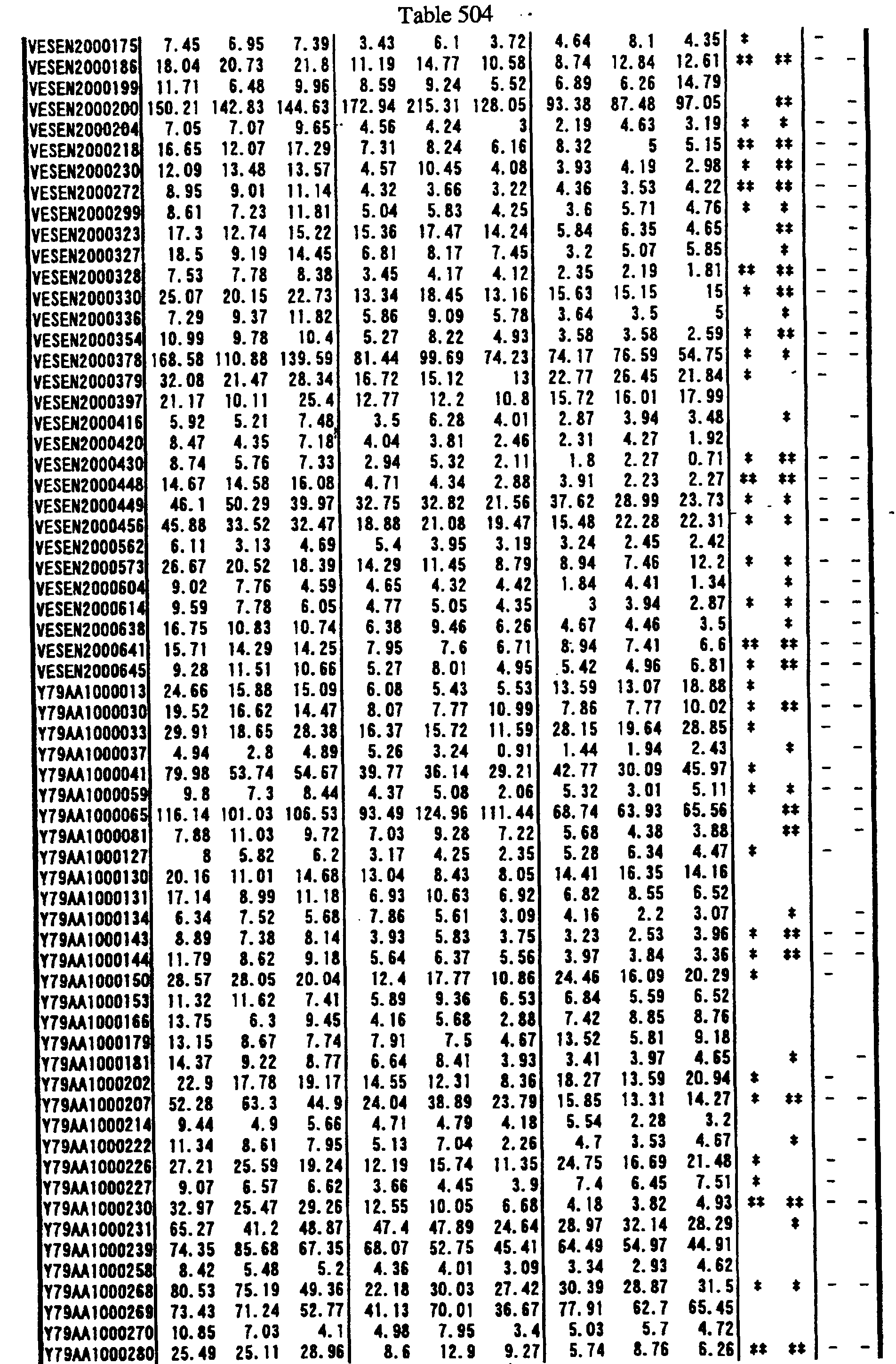

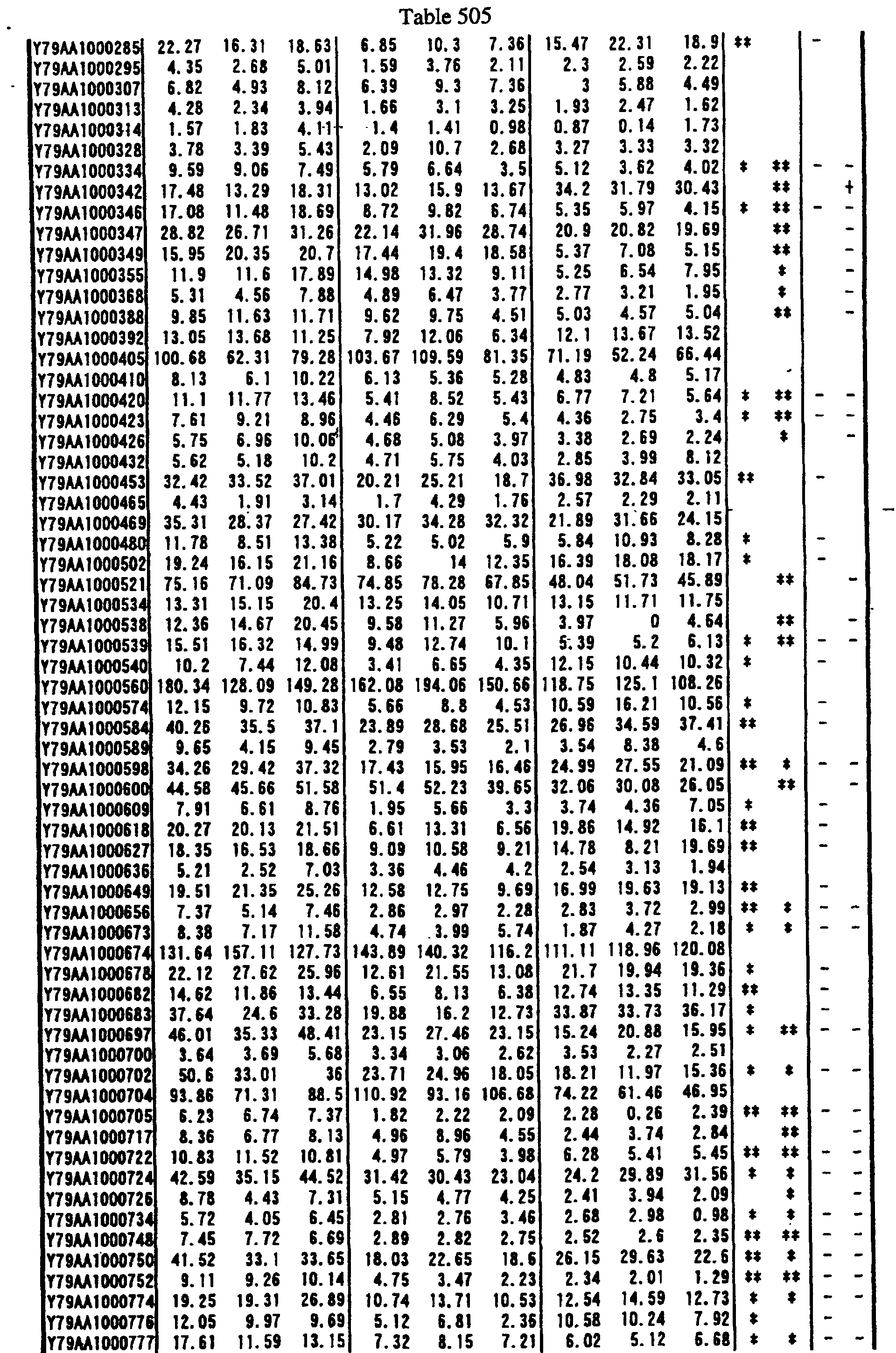

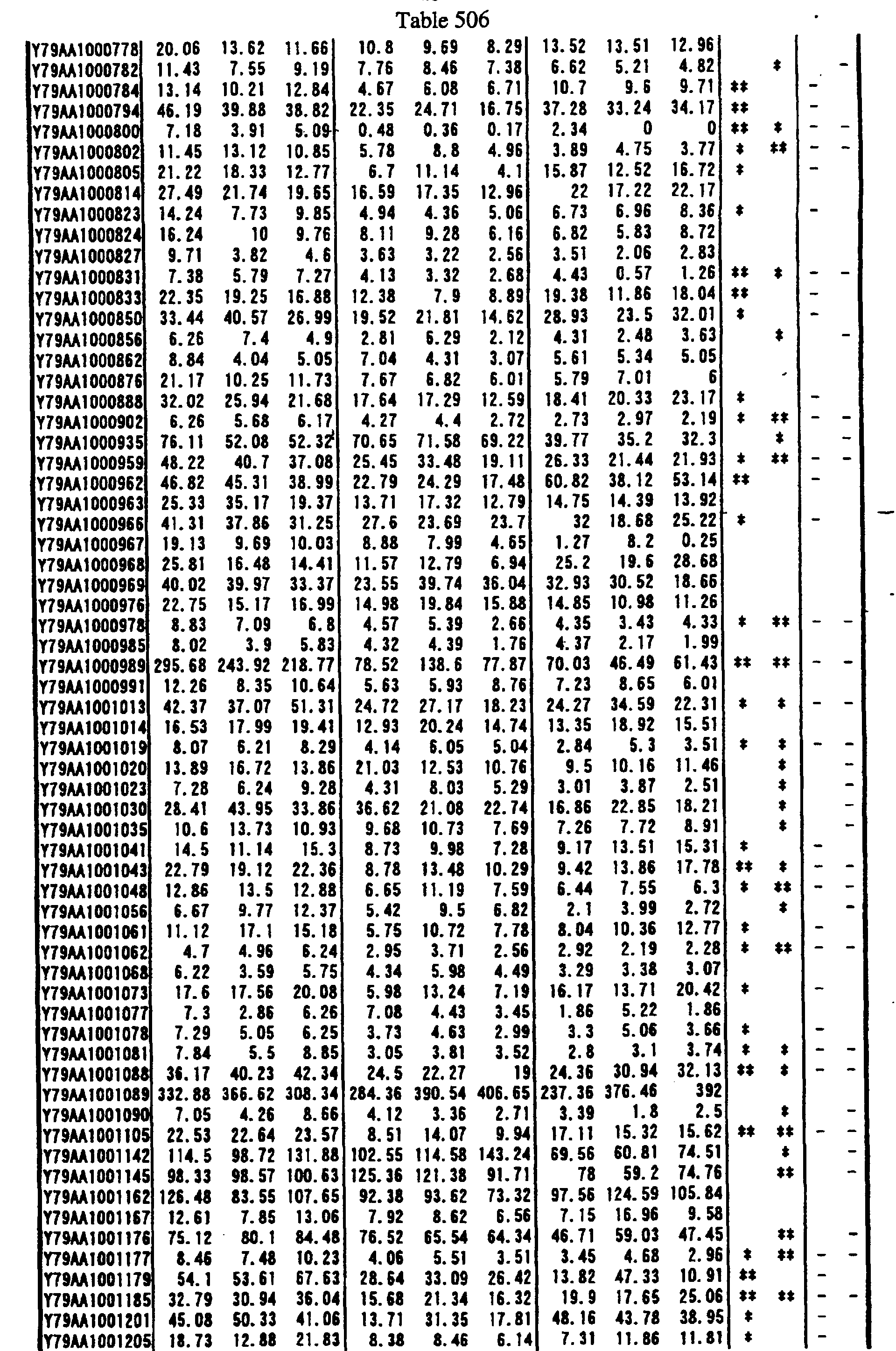

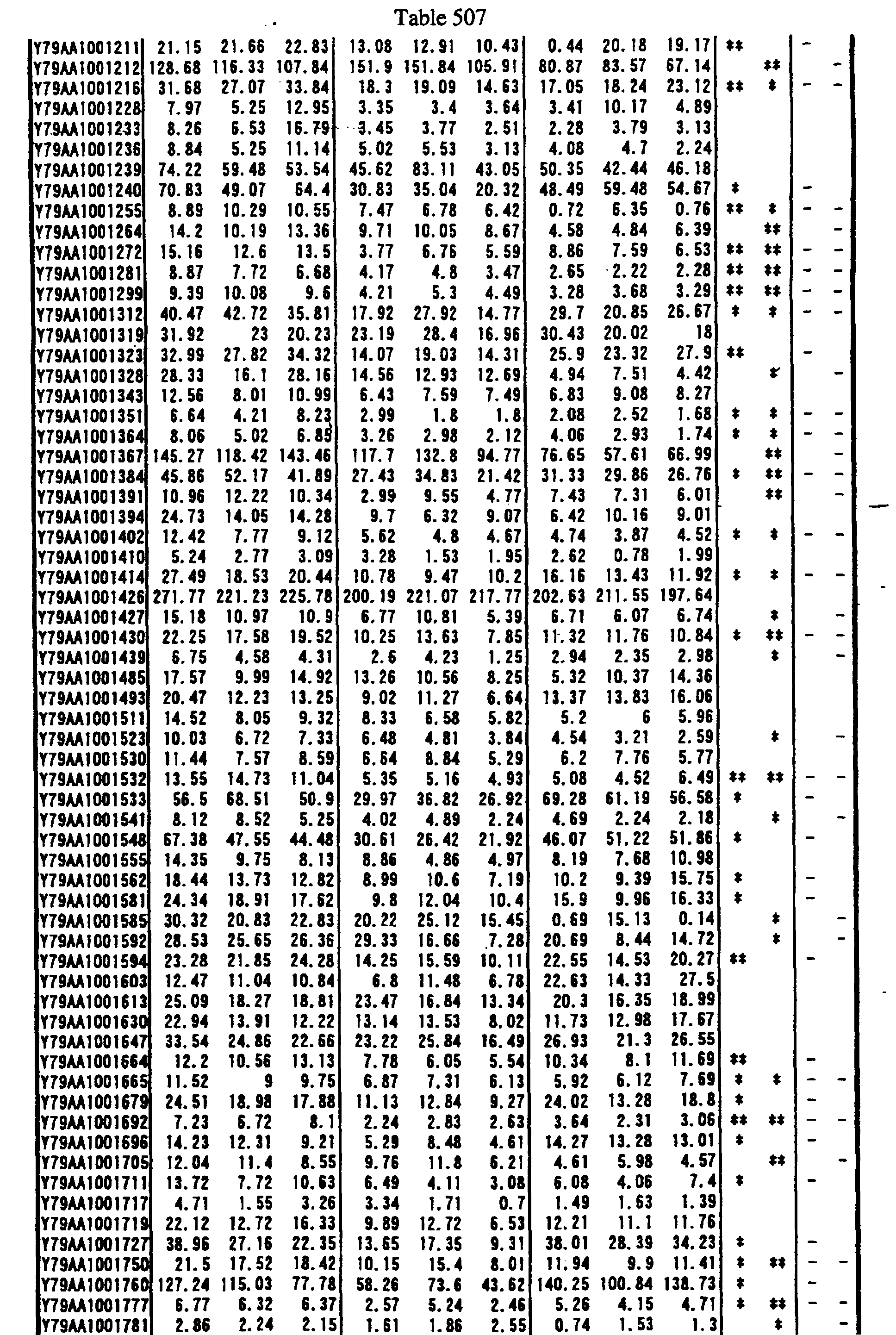

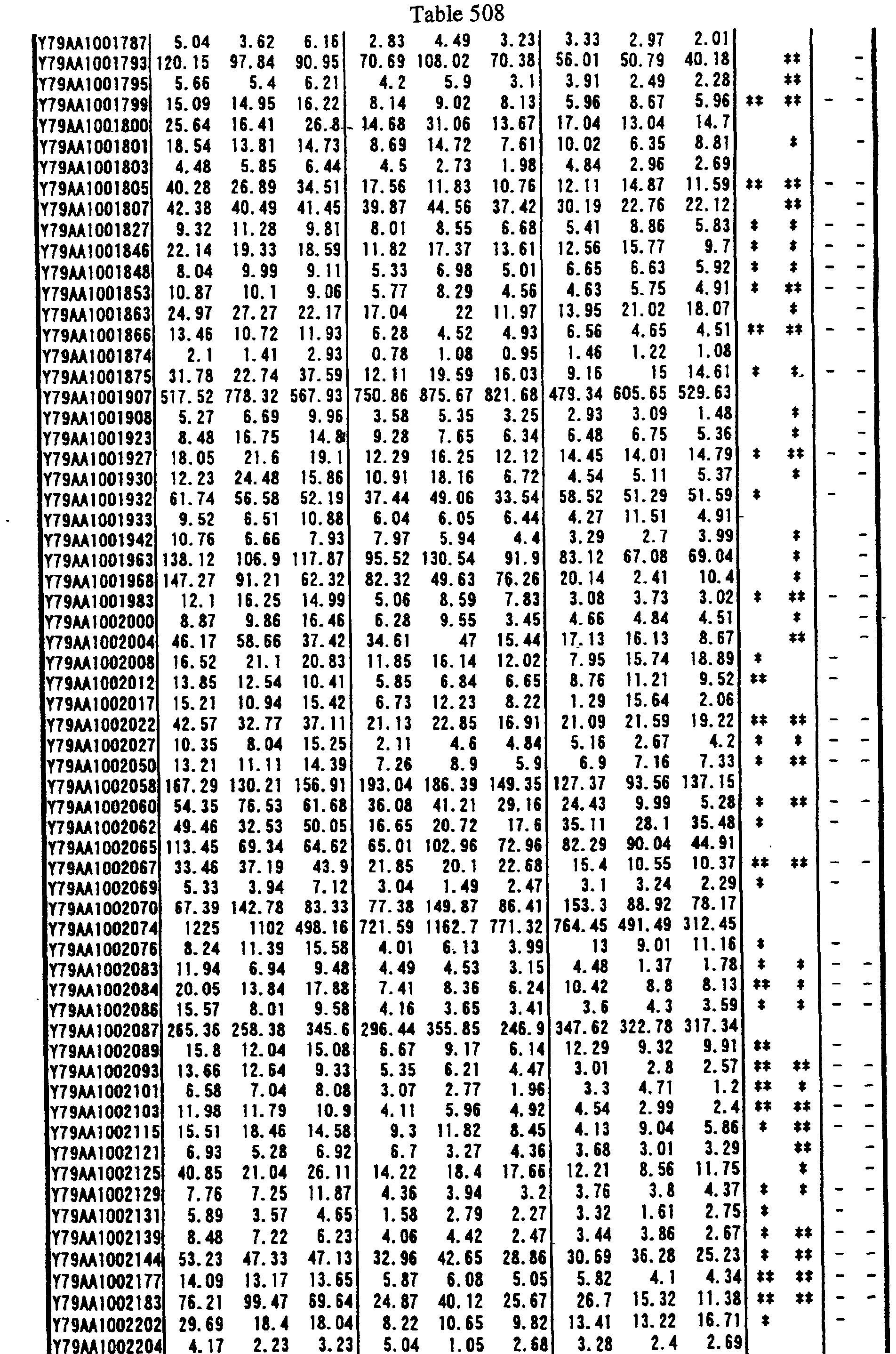

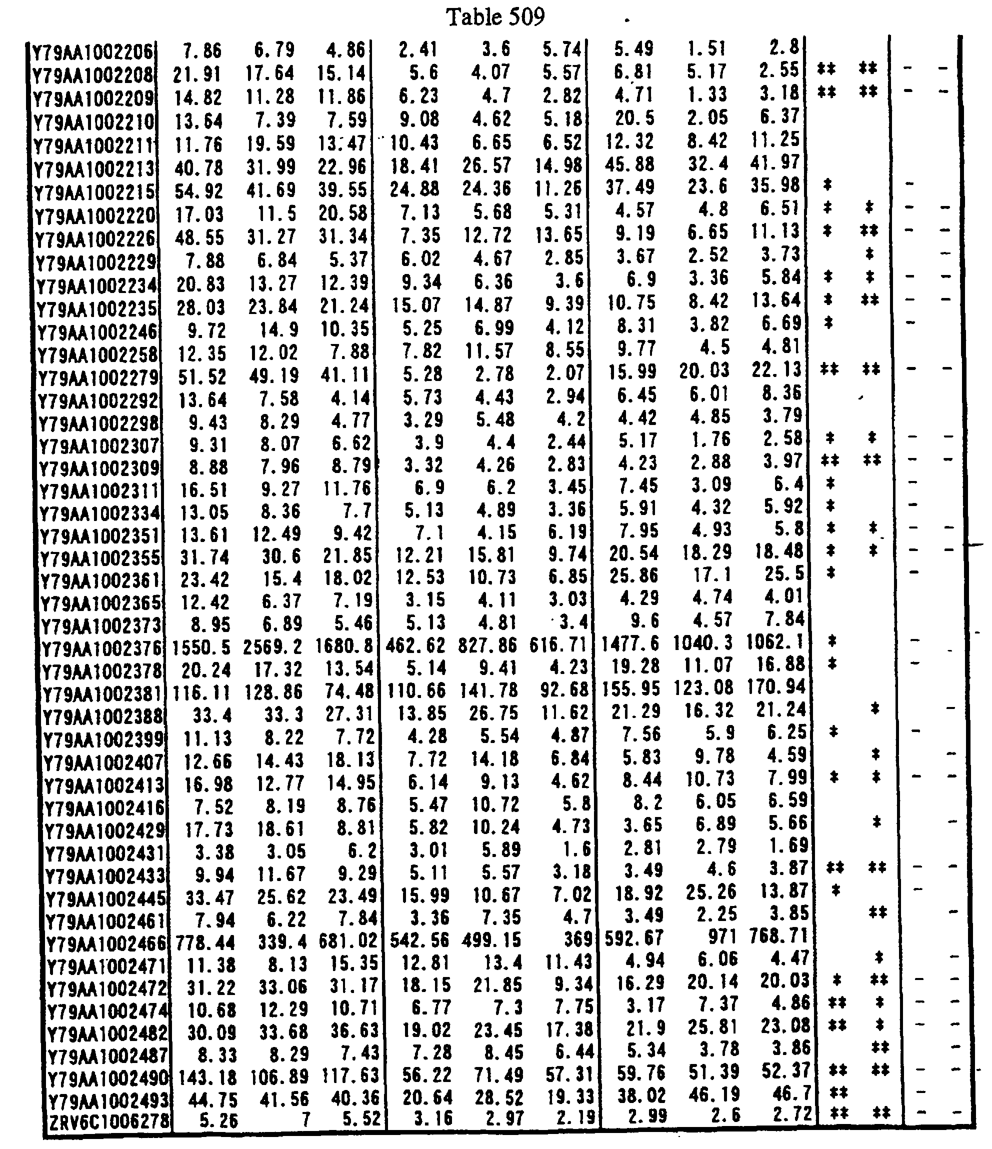

### EXAMPLE 16

### Selection of novel cDNA clones from cDNA libraries prepared by oligo-capping method

The following 54 clones were newly selected from cDNA libraries prepared by oligo-capping method, based on the criterion that the 5'-end sequence of a cDNA clone contained a coding region which was initiated with ATG codon and which encoded 50 amino acids or more:
HEMBA1000497, HEMBA1001750, HEMBA1003854, HEMBA1004193, HEMBA1004860, HEMBA1005572, HEMBA1006038, HEMBA1006092, HEMBA1006406, HEMBA1006650, HEMBA1006812, HEMBB1000672, HEMBB1001197, HEMBB1001871, MAMMA1001252, MAMMA1002094, NT2RM4000634, NT2RM4000657, NT2RM4000783, NT2RM4000857, NT2RM4001178, NT2RM4002420, NT2RP2000198, NT2RP2000551, NT2RP2000660,
NT2RP2001214, NT2RP2001460, NT2RP2001756, NT2RP2002056, NT2RP2002677, NT2RP2002755, NT2RP2002843, NT2RP2003101, NT2RP2003799, NT2RP2004095, NT2RP2004732, NT2RP2004920, NT2RP2005454, NT2RP2005776, NT2RP2005806, NT2RP2005882, NT2RP3001282, NT2RP3001723, NT2RP3002099, NT2RP3003155, NT2RP3004028, OVARC1000008, OVARC1000724, OVARC1000751, OVARC1001029, PLACE1000814, PLACE1003030, PLACE1005549, PLACE1007218.
Among them, the following 23 clones was predicted to contain a coding region encoding 100 amino acids or more: HEMBA1000497, HEMBA1003854, HEMBA1004193, HEMBA1006812, HEMBB1001871, NT2RM4000657, NT2RM4001178, NT2RP2001756, NT2RP2002677, NT2RP2002755, NT2RP2002843, NT2RP2004095, NT2RP2004920, NT2RP2005806, NT2RP3001282, NT2RP3002099, NT2RP3003155, OVARC1000724, OVARC1001029, PLACE1000814, PLACE1003030, PLACE1005549, PLACE1007218. This indicates that the clones encode proteins.

Table 510 shows maximal ATGprl value determined for each clone. Since the respective maximal ATGprl values for HEMBA1006812, HEMBB1001871 and NT2RRP3001282 are higher than 0.3, the clones would be full-length. Other clones indicated below have maximal ATGprl values of 0.3 or less, and this means that the fullness ratios of the clones are low.
However, the sequences can still be full-length:HEMBA1000497, HEMBA1001750, HEMBA1003854, HEMBA1004193, HEMBA1004860, HEMBA1005572, HEMBA1006038, HEMBA1006092, HEMBA1006406, HEMBA1006650, HEMBB1000672, HEMBB1001197, MAMMA1001252, MAMMA1002094, NT2RM4000634, NT2RM4000657, NT2RM4000783, NT2RM4000857, NT2RM4001178, NT2RM4002420, NT2RP2000198, NT2RP2000551, NT2RP2000660, NT2RP2001214, NT2RP2001460, NT2RP2001756, NT2RP2002056, NT2RP2002677, NT2RP2002755, NT2RP2002843, NT2RP2003101, NT2RP2003799, NT2RP2004095, NT2RP2004732, NT2RP2004920, NT2RP2005454, NT2RP2005776, NT2RP2005806, NT2RP2005882, NT2RP3001723, NT2RP3002099, NT2RP3003155, NT2RP3004028, OVARC1000008, OVARC1000724, OVARC1000751, OVARC1001029, PLACE1000814, PLACE1003030, PLACE1005549, PLACE1007218

Table 511 (same as Table 2) shows SEQ ID NOs of the nucleotide sequences located at the 5'-end and 3'-end of each of the 54 clones and the corresponding plasmid clone, which was obtained herein, containing a polynucleotide as an insert. SEQ ID NO for a 5'-end sequence is indicated on the right side of the corresponding Sequence name of 5'-end sequence, and SEQ ID NO for a 3'-end sequence is indicated on the right side of the corresponding Sequence name of 3'-end sequence.

Swiss-Prot was searched for data homologous to the 5' -end sequences of the selected 54 clones, and GenBank and UniGene were searched for data homologous to the 5' -end and 3'-end sequences of the same clones. The search results are indicated as Homology search results 1-7 in the last part of this SPECIFICATION.

Based on the matching data obtained by the search, 7 clones presumably encode proteins belonging to any of the categories of secretory or membrane proteins, glycoproteins, signal transduction-associated proteins, transcription-associated proteins, disease-associated proteins, and protein synthesis- and/or protein transport-associated proteins. These were clones exhibiting relatively low homology to any of known proteins belonging to said categories. Here, the term "relatively low homology" means that a nucleotide sequence does not satisfy the conditions under which the nucleotide sequence exhibits "relatively high homology" (which means that, when the nucleotide sequence is compared with the known sequences in Swiss-Prot database, the sequence identity is 60% or higher and the P value is 10⁻¹⁰ or less) and that, when the nucleotide sequence is compared with the known sequences in Swiss-Prot database, the sequence to be compared contains 55 nucleotides or more, the sequence identity is 25% or higher, and the P value is 10⁻⁶ or less.

Among the 7 clones, clones presumably encoding proteins belonging to the category of secretory or membrane proteins are the two clones, HEMBB1001871 and NT2RM4000857 (which also belong to other categories); clones presumably encoding proteins belonging to the category of glycoproteins are the two clones, HEMBB1001871 and NT2RM4000857 (which also belong to other categories);- a clone presumably encoding a protein belonging to the category of signal transduction-associated proteins is PLACE1005549; clones presumably encoding proteins belonging to the category of transcription-associated proteins are the three clones, HEMBA1005572, NT2RP2001756, and NT2RP2005776; a clone presumably encoding a protein belonging to the category of disease-associated proteins is NT2RM4000857 (which also belong to other categories); a clone presumably encoding a protein belonging to the category of protein synthesis- and/or protein transport-associated proteins is HEMBA1001750 (see Examples 12).

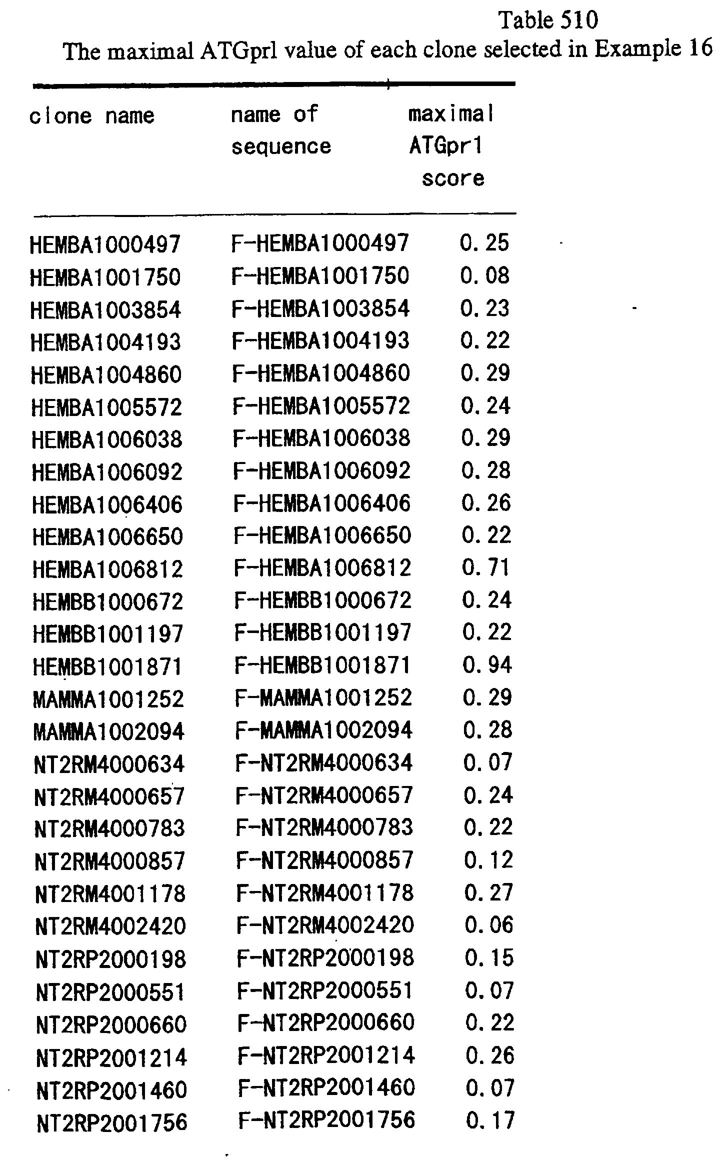

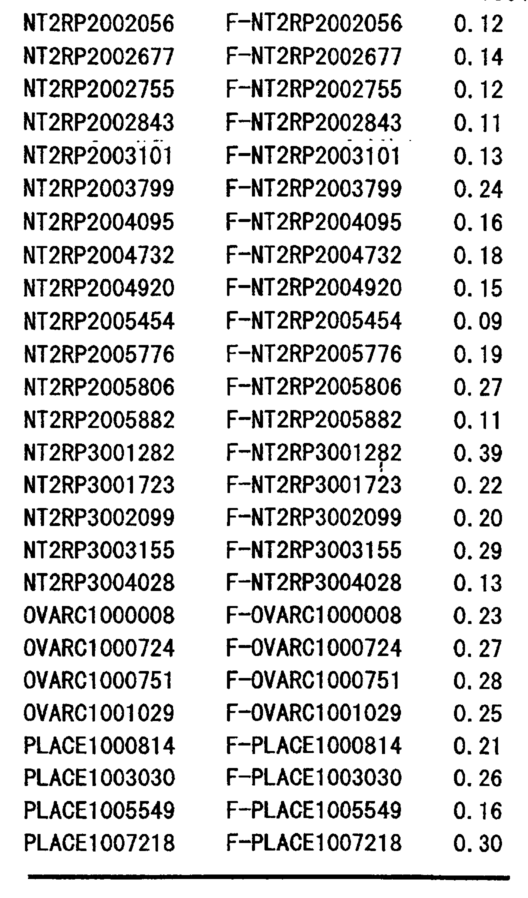

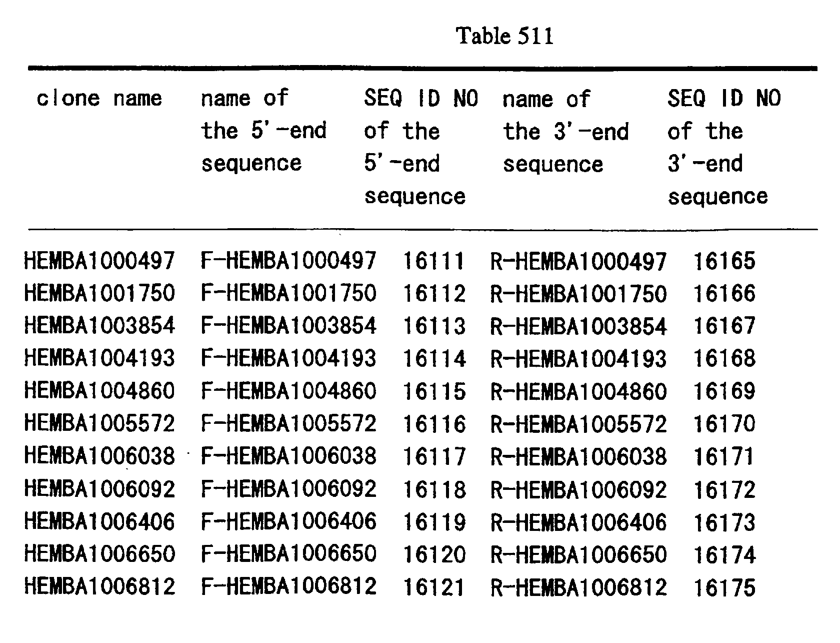

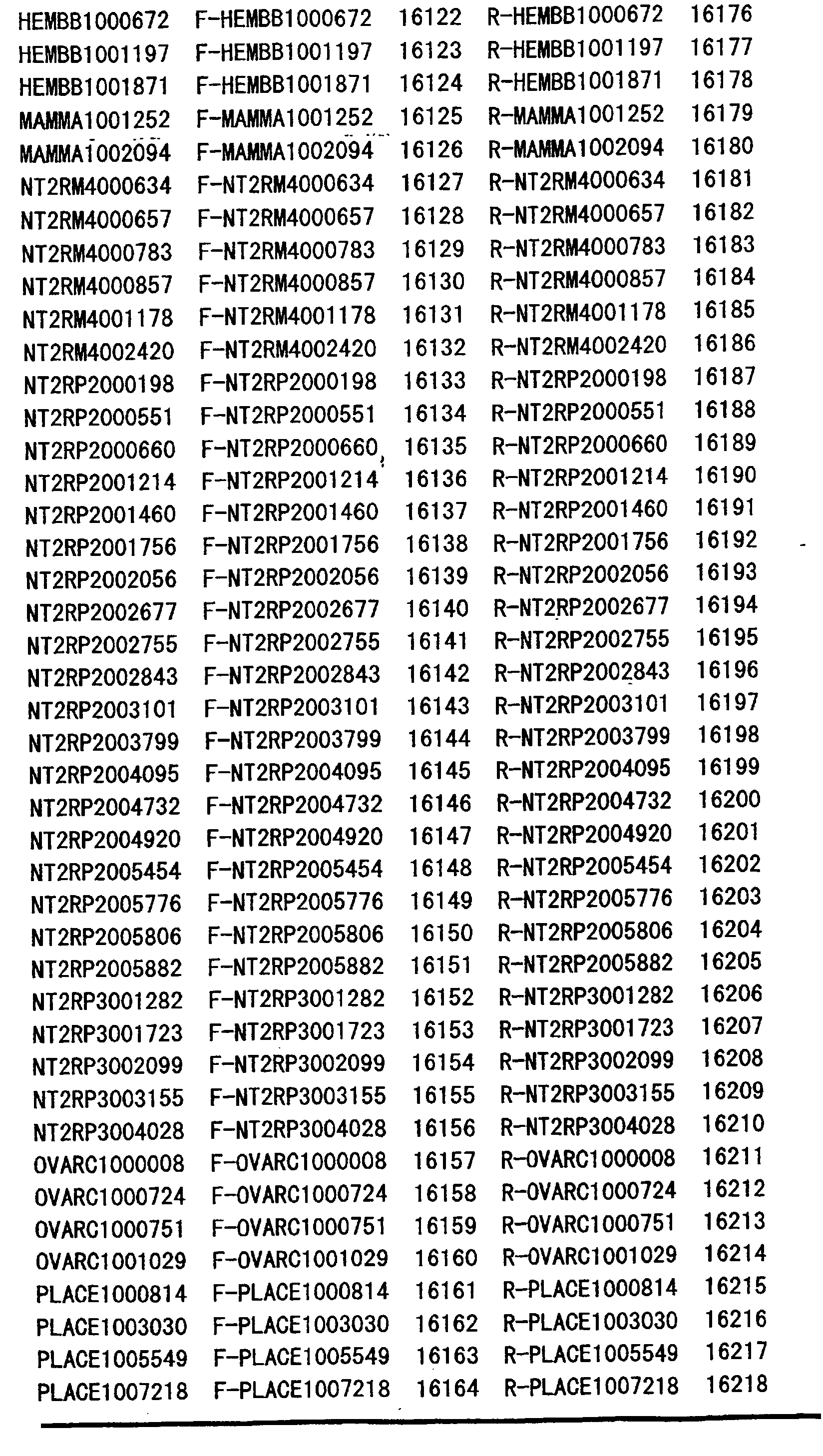

### EXAMPLE 17

### Search for a signal sequence, transmembrane region and functional domain in deduced amino acid sequences

The deduced amino acid sequences from the full-length nucleotide sequences were examined to predict the presence of a signal sequence in their amino-termini as well as the presence of a transmembrane region. The amino acid sequences were also searched for a protein functional domain (motif). The examinations for a signal sequence in the amino-terminus, for a transmembrane region and for a functional domain were performed by using PSORT [K. Nakai & M. Kanehisa, Genomics, 14:897-911 (1992)], SOSUI [T. Hirokawa et al., Bioinformatics, 14:378-379 (1998)] (Mitsui Knowledge Industry Co., Ltd.) and Pfam (http://www.sanger.ac.uk/Software/Pfam/index.shtml), respectively. When the presence of a signal sequence or a transmembrane region in the amino-terminus was predicted in the amino acid sequence by PSORT or SOSUI, the protein was predicted to be a secretory protein or a membrane protein. When the amino acid sequence matched a functional domain in the Pfam search for a functional domain, the function of the protein is predictable based on the matching data, for example, by referring to the functional categories in PROSITE (http://www.expasy.ch/cgi-bin/prosite-list.pl). The functional domain search can be performed by using PROSITE instead of Pfam.

Search results obtained by using the respective software programs are indicated below.

Clones whose deduced amino acid sequences were predicted to have signal sequences by PSORT search are as follows:
HEMBA1001052, HEMBA1001407, HEMBA1002486, HEMBA1002661, HEMBA1002818, HEMBA1002876, HEMBA1003086, HEMBA1003711, HEMBA1004752, HEMBA1005991, HEMBA1006067, HEMBA1006173, HEMBA1006198, HEMBA1006789, HEMBA1006921, HEMBB1000054, HEMBB1000175, HEMBB1002692, MAMMA1000798, MAMMA1002427, MAMMA1002881, MAMMA1003035, NT2RM1000035, NT2RM1000742, NT2RM1000811, NT2RM1000905, NT2RM1001008, NT2RM2000287, NT2RM2000609, NT2RM2001613, NT2RM4000634, NT2RM4000778, NT2RM4002339, NT2RM4002460, NT2RP1000782, NT2RP1000856, NT2RP1001247, NT2RP1001546, NT2RP1001569, NT2RP2001597, NT2RP2002537, NT2RP2004142, NT2RP2005752, NT2RP2005812, NT2RP3001084, NT2RP3001589, NT2RP3002163, NT2RP3002650, NT2RP3003145, NT2RP3003242, NT2RP3003621, NT2RP3004282, NT2RP3004503, NT2RP4000051, NT2RP4000151, NT2RP4000243, NT2RP4000259, NT2RP4000323, NT2RP4000417, NT2RP4001064, NT2RP4001117, NT2RP4001730, NT2RP4001739, NT2RP4002075, NT2RP5003500, OVARC1001154, PLACE1000611, PLACE1003030, PLACE1003044, PLACE1003369, PLACE1003596, PLACE1004258, PLACE1005086, PLACE1006239, PLACE1006754, PLACE1006829, PLACE1007954, PLACE1008424, PLACE1008533, PLACE1008693, PLACE1010622, PLACE1010942, PLACE2000176, PLACE2000341, PLACE2000379, PLACE2000427, PLACE2000477, PLACE4000431, PLACE4000593, THYRO1000156, THYRO1001134, THYRO1001287, Y79AA1000258, Y79AA1001874, Y79AA1002399 HEMBB1001871, HEMBB1001925, MAMMA1000778, MAMMA1000897, MAMMA1001080, NT2RP2004300, NT2RP3002985, NT2RP3003059, OVARC1000689, OVARC1000890, PLACE1005162, PLACE3000399, PLACE3000455, PLACE4000247, PLACE4000259, PLACE4000494

Clones whose deduced amino acid sequences were predicted to have transmembrane regions by SOSUI search are as follows:
HEMBA1000005, HEMBA1000356, HEMBA1000518, HEMBA1000531, HEMBA1000637, HEMBA1000719, HEMBA1000817, HEMBA1000822, HEMBA1000870, HEMBA1000991, HEMBA1001052, HEMBA1001085, HEMBA1001286, HEMBA1001351, HEMBA1001407, HEMBA1001446, HEMBA1001510, HEMBA1001515, HEMBA1001557, HEMBA1001746, HEMBA1002092, HEMBA1002125, HEMBA1002150, HEMBA1002166, HEMBA1002462, HEMBA1002477, HEMBA1002486, HEMBA1002609, HEMBA1002659, HEMBA1002661, HEMBA1002780, HEMBA1002818, HEMBA1002876, HEMBA1002921, HEMBA1003077, HEMBA1003079, HEMBA1003086, HEMBA1003096, HEMBA1003281, HEMBA1003286, HEMBA1003711, HEMBA1003742, HEMBA1003803, HEMBA1004143, HEMBA1004146, HEMBA1004341, HEMBA1004461, HEMBA1004577, HEMBA1004637, HEMBA1004752, HEMBA1004756, HEMBA1004850, HEMBA1004889, HEMBA1004923, HEMBA1004930, HEMBA1005029, HEMBA1005035, HEMBA1005050, HEMBA1005552, HEMBA1005588, HEMBA1005616, HEMBA1005991, HEMBA1006036, HEMBA1006067, HEMBA1006293, HEMBA1006492, HEMBA1006502, HEMBA1006659, HEMBA1006758, HEMBA1006789, HEMBA1006921, HEMBA1006926, HEMBA1007203, HEMBB1000050, HEMBB1000054, HEMBB1000556, HEMBB1000593, HEMBB1000631, HEMBB1000763, HEMBB1000827, HEMBB1000915, HEMBB1000975, HEMBB1001112, HEMBB1001177, HEMBB1001302, HEMBB1001348, HEMBB1001962, HEMBB1002142, HEMBB1002190, HEMBB1002247, HEMBB1002387, HEMBB1002550, HEMBB1002600, HEMBB1002692, MAMMA1000129, MAMMA1000133, MAMMA1000277, MAMMA1000278, MAMMA1000410, MAMMA1000416, MAMMA1000472, MAMMA1000714, MAMMA1000731, MAMMA1000734, MAMMA1000798, MAMMA1000842, MAMMA1000956, MAMMA1001008, MAMMA1001030, MAMMA1001139, MAMMA1001154, MAMMA1001388, MAMMA1001411, MAMMA1001487, MAMMA1001751, MAMMA1001771, MAMMA1002461, MAMMA1002524, MAMMA1002598, MAMMA1002684, MAMMA1002769, MAMMA1002890, MAMMA1002938, MAMMA1003146, NT2RM1000035, NT2RM1000037, NT2RM1000062, NT2RM1000131, NT2RM1000257, NT2RM1000260, NT2RM1000355, NT2RM1000648, NT2RM1000742, NT2RM1000800, NT2RM1000811, NT2RM1000857, NT2RM1000867, NT2RM1000882, NT2RM1001008, NT2RM1001115, NT2RM1001139, NT2RM2000259, NT2RM2000395, NT2RM2000402, NT2RM2000407, NT2RM2000422, NT2RM2000566, NT2RM2000581, NT2RM2000609, NT2RM2001370, NT2RM2001393, NT2RM2001499, NT2RM2001613, NT2RM2001648, NT2RM2001659, NT2RM2001671, NT2RM2001718, NT2RM2001760, NT2RM2001785, NT2RM2001823, NT2RM2001930, NT2RM2001950, NT2RM2001998, NT2RM2002049, NT2RM4000046, NT2RM4000233, NT2RM4000433, NT2RM4000520, NT2RM4000634, NT2RM4000674, NT2RM4000700, NT2RM4000764, NT2RM4000795, NT2RM4000820, NT2RM4000857, NT2RM4001032, NT2RM4001054, NT2RM4001455, NT2RM4001813, NT2RM4001930, NT2RM4001987, NT2RM4002054, NT2RM4002073, NT2RM4002145, NT2RM4002146, NT2RM4002194, NT2RM4002339, NT2RM4002438, NT2RM4002446, NT2RM4002452, NT2RM4002460, NT2RM4002493, NT2RM4002571, NT2RP1000191, NT2RP1000358, NT2RP1000418, NT2RP1000547, NT2RP1000609, NT2RP1000677, NT2RP1000767, NT2RP1000782, NT2RP1000856, NT2RP1001113, NT2RP1001247, NT2RP1001286, NT2RP1001310, NT2RP1001311, NT2RP1001313, NT2RP1001385, NT2RP1001449, NT2RP1001546, NT2RP1001569, NT2RP2000032, NT2RP2000040, NT2RP2000070, NT2RP2000091, NT2RP2000114, NT2RP2000120, NT2RP2000173, NT2RP2000175, NT2RP2000195, NT2RP2000248, NT2RP2000270, NT2RP2000283, NT2RP2000289, NT2RP2000459, NT2RP2000516, NT2RP2000842, NT2RP2000892, NT2RP2001081, NT2RP2001268, NT2RP2001295, NT2RP2001366, NT2RP2001576, NT2RP2001581, NT2RP2001597, NT2RP2001947, NT2RP2001991, NT2RP2002025, NT2RP2002312, NT2RP2002385, NT2RP2002479, NT2RP2002537, NT2RP2002643, NT2RP2002701, NT2RP2002740, NT2RP2002857, NT2RP2003125, NT2RP2003297, NT2RP2003433, NT2RP2003446, NT2RP2003466, NT2RP2003629, NT2RP2003777, NT2RP2003781, NT2RP2004041, NT2RP2004194, NT2RP2004270, NT2RP2004681, NT2RP2004775, NT2RP2004799, NT2RP2004936, NT2RP2005012, NT2RP2005159, NT2RP2005227, NT2RP2005270, NT2RP2005344, NT2RP2005509, NT2RP2005752, NT2RP2005781, NT2RP2005784, NT2RP2005812, NT2RP2006069, NT2RP2006100, NT2RP2006141, NT2RP2006261, NT2RP2006571, NT2RP3000092, NT2RP3000134, NT2RP3000333, NT2RP3000393, NT2RP3000439, NT2RP3000441, NT2RP3000531, NT2RP3000685, NT2RP3000826, NT2RP3000852, NT2RP3001126, NT2RP3001176, NT2RP3001260, NT2RP3001355, NT2RP3001383, NT2RP3001426, NT2RP3001453, NT2RP3001497, NT2RP3001538, NT2RP3001716, NT2RP3001727, NT2RP3001739, NT2RP3001799, NT2RP3001943, NT2RP3001944, NT2RP3002002, NT2RP3002014, NT2RP3002054, NT2RP3002108, NT2RP3002163, NT2RP3002351, NT2RP3002455, NT2RP3002549, NT2RP3002628, NT2RP3002650, NT2RP3002687, NT2RP3002701, NT2RP3002869, NT2RP3002969, NT2RP3003008, NT2RP3003071, NT2RP3003101, NT2RP3003145, NT2RP3003302, NT2RP3003353, NT2RP3003409, NT2RP3003716, NT2RP3003918, NT2RP3004207, NT2RP3004454, NT2RP3004503, NT2RP4000051, NT2RP4000151, NT2RP4000243, NT2RP4000259, NT2RP4000323, NT2RP4000500, NT2RP4000560, NT2RP4000588, NT2RP4000713, NT2RP4000724, NT2RP4000833, NT2RP4000878, NT2RP4000907, NT2RP4000925, NT2RP4000928, NT2RP4000973, NT2RP4000989, NT2RP4001057, NT2RP4001064, NT2RP4001079, NT2RP4001117, NT2RP4001138, NT2RP4001150, NT2RP4001174, NT2RP4001274, NT2RP4001345, NT2RP4001372, NT2RP4001373, NT2RP4001379, NT2RP4001498, NT2RP4001547, NT2RP4001571, NT2RP4001644, NT2RP4001677, NT2RP4001803, NT2RP4001822, NT2RP4001975, NT2RP4002052, NT2RP4002075, NT2RP5003500, NT2RP5003506, NT2RP5003522, NT2RP5003534, OVARC1000151, OVARC1000241, OVARC1000335, OVARC1000700, OVARC1000722, OVARC1000751, OVARC1000850, OVARC1000924, OVARC1000936, OVARC1000959, OVARC1000984, OVARC1001034, OVARC1001129, OVARC1001381, OVARC1001391, OVARC1001453, OVARC1001476, OVARC1001506, OVARC1001610, OVARC1001702, OVARC1001703, OVARC1001713, OVARC1001745, OVARC1001767, OVARC1002127, OVARC1002158, OVARC1002165, PLACE1000014, PLACE1000401, PLACE1000562, PLACE1000611, PLACE1000656, PLACE1000712, PLACE1000909, PLACE1000948, PLACE1001241, PLACE1001257, PLACE1001377, PLACE1001517, PLACE1001610, PLACE1001771, PLACE1001817, PLACE1001983, PLACE1002213, PLACE1002395, PLACE1002500, PLACE1002714, PLACE1002722, PLACE1002794, PLACE1002851, PLACE1002908, PLACE1003045, PLACE1003238, PLACE1003296, PLACE1003369, PLACE1003493, PLACE1003537, PLACE1003553, PLACE1003768, PLACE1003771, PLACE1003903, PLACE1004197, PLACE1004258, PLACE1004270, PLACE1004289, PLACE1004473, PLACE1004743, PLACE1004840, PLACE1004969, PLACE1005086, PLACE1005206, PLACE1005313, PLACE1005530, PLACE1005595, PLACE1005623, PLACE1005763, PLACE1005884, PLACE1005934, PLACE1006225, PLACE1006754, PLACE1006901, PLACE1006935, PLACE1006956, PLACE1007014, PLACE1007111, PLACE1007243, PLACE1007274, PLACE1007282, PLACE1007317, PLACE1007375, PLACE1007386, PLACE1007409, PLACE1007484, PLACE1007583, PLACE1007632, PLACE1007645, PLACE1007852, PLACE1007877, PLACE1008331, PLACE1008424, PLACE1008531, PLACE1008532, PLACE1008568, PLACE1008715, PLACE1009045, PLACE1009319, PLACE1009338, PLACE1009368, PLACE1009493, PLACE1009639, PLACE1009708, PLACE1009731, PLACE1010089, PLACE1010231, PLACE1010321, PLACE1010622, PLACE1010811, PLACE1010917, PLACE1010954, PLACE1011090, PLACE1011214, PLACE1011221, PLACE1011399, PLACE1011492, PLACE1011646, PLACE1011749, PLACE2000034, PLACE2000111, PLACE2000176, PLACE2000187, PLACE2000341, PLACE2000379, PLACE2000425, PLACE2000458, PLACE3000020, PLACE3000218, PLACE3000226, PLACE3000244, PLACE3000413, PLACE4000052, PLACE4000129, PLACE4000300, PLACE4000387, PLACE4000581, PLACE4000593, PLACE4000650, THYRO1000394, THYRO1000395, THYRO1000570, THYRO1000748, THYRO1000756, THYRO1001134, THYRO1001271, THYRO1001401, THYRO1001534, THYRO1001541, THYRO1001809, Y79AA1000258, Y79AA1000420, Y79AA1000469, Y79AA1000734, Y79AA1000800, Y79AA1000976, Y79AA1001023, Y79AA1001177, Y79AA1001394, Y79AA1001603, Y79AA1001647, Y79AA1001846, Y79AA1001874, Y79AA1002139, Y79AA1002351, Y79AA1002399, Y79AA1002416 HEMBA1004055, HEMBB1001630, HEMBB1001872, HEMBB1002044, HEMBB1002383, MAMMA1000778, MAMMA1000859, MAMMA1000897, MAMMA1001073, MAMMA1002009, MAMMA1002844, MAMMA1002947, MAMMA1003089, NT2RM1000092, NT2RM1000833, NT2RP2002105, NT2RP2003668, NT2RP2006184, NT2RP3001282, NT2RP3002810, NT2RP3002985, NT2RP3003059, NT2RP3003576, NT2RP3003665, NT2RP3003799, NT2RP3003828, NT2RP3003992, NT2RP3004051, NT2RP3004155, OVARC1000890, OVARC1001117, OVARC1001329, PLACE1001761, PLACE1002437, PLACE1004793, PLACE1005611, PLACE1005898, PLACE1009935, PLACE1011896, PLACE2000132, PLACE2000335, PLACE3000373, PLACE3000406, PLACE4000250, PLACE4000487, PLACE4000494, THYRO1001320, THYRO1001537, THYRO1001828, Y79AA1001384

Names of clones whose deduced amino acid sequences were predicted to have functional domains by Pfam search, and names of the matched functional domains are shown below.
When multiple functional domains matched a clone, each domain name was indicated, separated by a double-slash mark, //.
HEMBA1000005//DnaJ, prokaryotic heat shock protein
HEMBA1000020//Tubulin
HEMBA1000129//Helicases conserved C-terminal domain
HEMBA1000156//RNA recognition motif. (aka RRM, RBD, or RNP domain)
HEMBA1000158//Fork head domain, eukaryotic transcription factors //Zinc finger, C2H2 type
HEMBA1000303//Src homology domain 3 //Zinc finger, C3HC4 type (RING finger)
HEMBA1000411//Ank repeat
HEMBA1000491//Ras family (contains ATP/GTP binding P-loop)
HEMBA1000531//Heat shock hsp70 proteins
HEMBA1000561//Zinc finger, C2H2 type
HEMBA1000608//Src homology domain 3
HEMBA1000919//WD domain, G-beta repeats
HEMBA1001043//Ank repeat
HEMBA1001088//LIM domain containing proteins
HEMBA1001137//Zinc finger, C2H2 type
HEMBA1001174//ADP-ribosylation factors (Arf family) (contains ATP/GTP binding P-loop)
HEMBA1001247//WW/rsp5/WWP domain containing proteins
HEMBA1001286//Sushi domain
HEMBA1001510//Basic region plus leucine zipper transcription factors
HEMBA1001515//Reverse transcriptase (RNA-dependent DNA polymerase)
HEMBA1001661//Cadherin
HEMBA1001723//WD domain, G-beta repeats
HEMBA1001744//Eukaryotic protein kinase domain
HEMBA1001804//Zinc finger, C2H2 type
HEMBA1001819//Zinc finger, C2H2 type
HEMBA1001847//Zinc finger, C2H2 type
HEMBA1002035//Bromodomain
HEMBA1002102//Ank repeat
HEMBA1002161//Myosin head (motor domain) (contains ATP/GTP binding P-loop)
HEMBA1002177//GATA family of transcription factors //Zinc finger, C2H2 type
HEMBA1002212//Eukaryotic protein kinase domain
HEMBA1002215//LIM domain containing proteins
HEMBA1002419//RNA recognition motif. (aka RRM, RBD, or RNP domain)
HEMBA1002547//Kazal-type serine protease inhibitor domain //Laminin EGF-like (Domains III and V)
HEMBA1002768//Src homology domain 3
HEMBA1002810//WW/rsp5/WWP domain containing proteins
HEMBA1002818//EGF-like domain
HEMBA1002935//Zinc finger, C2H2 type
HEMBA1002939//Ank repeat
HEMBA1002973//3'5'-cyclic nucleotide phosphodiesterases
HEMBA1003077//Fibronectin type III domain
HEMBA1003250//Eukaryotic protein kinase domain
HEMBA1003257//Zinc finger, C2H2 type
HEMBA1003281//IG superfamily
HEMBA1003291//Eukaryotic protein kinase domain
HEMBA1003433//Forkhead-associated (FHA) domain
HEMBA1003545//Homeobox domain //LIM domain containing proteins
HEMBA1003591//RNA recognition motif. (aka RRM, RBD, or RNP domain)
HEMBA1003684//Zinc finger, C2H2 type
HEMBA1003953//Zinc finger, C2H2 type
HEMBA1004202//Ras family (contains ATP/GTP binding P-loop)
HEMBA1004227//Protein phosphatase 2C
HEMBA1004321//Zinc finger, C2H2 type
HEMBA1004356//RNA recognition motif. (aka RRM, RBD, or RNP domain)
HEMBA1004408//Peptidyl-prolyl cis-trans isomerases
HEMBA1004596//RNA recognition motif. (aka RRM, RBD, or RNP domain)
HEMBA1004734//Ubiquitin-conjugating enzymes
HEMBA1004973//Fibronectin type III domain
HEMBA1005009//Actins
HEMBA1005101//RNA recognition motif. (aka RRM, RBD, or RNP domain)
HEMBA1005581//EGF-like domain //Laminin G domain
HEMBA1005732//Polyprenyl synthetases
HEMBA1005737//EF hand
HEMBA1006248//Zinc finger, C2H2 type
HEMBA1006284//Ubiquitin family
HEMBA1006293//IG superfamily
HEMBA1006344//Band 4.1 family
HEMBA1006445//Ras family (contains ATP/GTP binding P-loop)
HEMBA1006492//Ank repeat
HEMBA1006559//Zinc finger, C3HC4 type (RING finger)
HEMBA1006708//WD domain, G-beta repeats
HEMBA1006737//Ank repeat
HEMBA1006758//Cadherin
HEMBA1006941//Thioredoxins
HEMBA1007243//Purine/pyrimidine phosphoribosyl transferases
HEMBA1007300//3'5'-cyclic nucleotide phosphodiesterases
HEMBB1000083//IG superfamily
HEMBB1000317//EGF-like domain //Thrombospondin type 1 domain
HEMBB1000556//Actinin-type actin-binding domain containing proteins //LIM domain containing proteins
HEMBB1000725//Ras family (contains ATP/GTP binding P-loop)
HEMBB1000781//Eukaryotic protein kinase domain
HEMBB1000915//Thrombospondin type 1 domain
HEMBB1000927//EF hand
HEMBB1000947//Double-stranded RNA binding motif
HEMBB1001112//eubacterial secY protein
HEMBB1001175//Ank repeat
HEMBB1001234//WW/rsp5/WWP domain containing proteins
HEMBB1001282//Ank repeat
HEMBB1001294//Ras family (contains ATP/GTP binding P-loop)
HEMBB1001339//Forkhead-associated (FHA) domain
HEMBB1001673//Forkhead-associated (FHA) domain //Zinc finger, C3HC4 type (RING finger)
HEMBB1001802//Intermediate filament proteins
HEMBB1001839//Zinc finger, C2H2 type
HEMBB1002217//Zinc finger, C2H2 type
HEMBB1002342//Thioredoxins
HEMBB1002600//4 transmembrane segments integral membrane proteins
MAMMA1000173//Src homology domain 3
MAMMA1000388//Zinc finger, C2H2 type
MAMMA1000402//Reverse transcriptase (RNA-dependent DNA polymerase)
MAMMA1000612//WD domain, G-beta repeats
MAMMA1000672//Serine carboxypeptidases
MAMMA1000731//SNF2 and others N-terminal domain
MAMMA1001008//Eukaryotic aspartyl proteases
MAMMA1001041//Actinin-type actin-binding domain containing proteins
MAMMA1001059//DEAD and DEAH box helicases //Helicases conserved C-terminal domain
MAMMA1001105//Zinc finger, C2H2 type
MAMMA1001260//Zinc finger, C3HC4 type (RING finger)
MAMMA1001576//Tubulin
MAMMA1001735//Tubulin
MAMMA1001768//ATPases associated with various cellular activities (AAA)
MAMMA1001837//Zinc finger, C2H2 type
MAMMA1002170//Ribosomal protein S5
MAMMA1002385//RNA recognition motif. (aka RRM, RBD, or RNP domain)
MAMMA1002619//Ubiquitin carboxyl-terminal hydrolases family 2
MAMMA1002637//Kinesin light chain repeat
MAMMA1002650//Zinc finger, C2H2 type
MAMMA1002671//AMP-binding enzymes
MAMMA1002869//LIM domain containing proteins
MAMMA1002881//SCP-like extracellular Proteins
MAMMA1002937//Zinc finger, C2H2 type
MAMMA1002938//Multicopper oxidases
MAMMA1003011//Core histones H2A, H2B, H3 and H4
MAMMA1003057//WD domain, G-beta repeats
MAMMA1003127//Myosin head (motor domain) (contains ATP/GTP binding P-loop)
NT2RM1000086//Zinc finger, C3HC4 type (RING finger)
NT2RM1000199//CUB domain //Sushi domain
NT2RM1000256//Glutamine amidotransferases class-II
NT2RM1000499//Ank repeat
NT2RM1000555//'Cold-shock' DNA-binding domain containing proteins
NT2RM1000666//'Cold-shock' DNA-binding domain containing proteins //Zinc finger, CCHC class
NT2RM1000772//WD domain, G-beta repeats
NT2RM1000826//'Cold-shock' DNA-binding domain containing proteins
NT2RM1000850//Ank repeat //Eukaryotic protein kinase domain
NT2RM1000852//DEAD and DEAH box helicases //Helicases conserved C-terminal domain
NT2RM1000882//Heme-binding domain in cytochrome b5 and oxidoreductases
NT2RM1000885//Zinc finger, C3HC4 type (RING finger)
NT2RM1001059//RNA recognition motif. (aka RRM, RBD, or RNP domain)
NT2RM1001072//C2 domain //Phosphatidylinositol-specific phospholipase C, X domain //Phosphatidylinositol-specific phospholipase C, Y domain
NT2RM2000092//Ubiquitin carboxyl-terminal hydrolases family 2
NT2RM2000101//Zinc finger, C3HC4 type (RING finger)
NT2RM2000191//3'5'-cyclic nucleotide phosphodiesterases
NT2RM2000422//Sodium:neurotransmitter symporter family
NT2RM2000490//C2 domain
NT2RM2000566//Integrins alpha chain
NT2RM2000577//tRNA synthetases class I
NT2RM2000594//C-5 cytosine-specific DNA methylases
NT2RM2000691//Actins
NT2RM2000735//Zinc finger, C2H2 type
NT2RM2000740//Helicases conserved C-terminal domain
NT2RM2000951//FGGY family of carbohydrate kinases
NT2RM2001324//LIM domain containing proteins
NT2RM2001499//Amino acid permeases
NT2RM2001547//DnaJ, prokaryotic heat shock protein //Thioredoxins
NT2RM2001613//eubacterial secY protein
NT2RM2001670//Zinc finger, C2H2 type
NT2RM2001700//Acyl-CoA dehydrogenases
NT2RM2001730//Ubiquitin carboxyl-terminal hydrolases family 2
NT2RM2001813//WD domain, G-beta repeats
NT2RM2001823//Helicases conserved C-terminal domain //SNF2 and others N-terminal domain
NT2RM2001896//Cytochrome C oxidase subunit II
NT2RM2001989//RNA recognition motif. (aka RRM, RBD, or RNP domain)
NT2RM2001997//Thioredoxins
NT2RM2002088//KH domain family of RNA binding proteins
NT2RM2002100//DEAD and DEAH box helicases //Helicases conserved C-terminal domain
NT2RM2002109//IG superfamily
NT2RM4000046//Zinc finger, C3HC4 type (RING finger)
NT2RM4000104//Zinc finger, C2H2 type
NT2RM4000167//Kinesin motor domain
NT2RM4000191//DEAD and DEAH box helicases //Helicases conserved C-terminal domain
NT2RM4000202//Zinc finger, C2H2 type
NT2RM4000229//PH (pleckstrin homology) domain
NT2RM4000344//ATPases associated with various cellular activities (AAA)
NT2RM4000356//Ras family (contains ATP/GTP binding P-loop)
NT2RM4000471//Aminotransferases class-V
NT2RM4000496//ATPases associated with various cellular activities (AAA)
NT2RM4000611//WD domain, G-beta repeats
NT2RM4000657//C2 domain //Phosphatidylinositol-specific phospholipase C, Y domain
NT2RM4000712//Ubiquitin carboxyl-terminal hydrolases family 2 //Ubiquitin carboxyl-terminal hydrolases family 2
NT2RM4000733//Forkhead-associated (FHA) domain
NT2RM4000734//Zinc finger, C2H2 type
NT2RM4000751//Zinc finger, C2H2 type
NT2RM4000795//Carboxylesterases
NT2RM4000996//Zinc finger, C2H2 type
NT2RM4001054//eubacterial secY protein
NT2RM4001140//Homeobox domain
NT2RM4001178//DEAD and DEAH box helicases
NT2RM4001200//Zinc finger, C2H2 type
NT2RM4001313//Phosphatidylinositol 3- and 4-kinases
NT2RM4001316//Acyl-CoA dehydrogenases
NT2RM4001320//Src homology domain 3
NT2RM4001411//PH (pleckstrin homology) domain //Src homology domain 2
NT2RM4001454//PH (pleckstrin homology) domain
NT2RM4001483//Zinc finger, C2H2 type
NT2RM4001629//Src homology domain 3
NT2RM4001758//Eukaryotic protein kinase domain
NT2RM4001810//Zinc finger, C2H2 type
NT2RM4001813//Lectin C-type domain short and long forms
NT2RM4001823//Zinc finger, C2H2 type
NT2RM4001828//Zinc finger, C2H2 type
NT2RM4001979//Zinc finger, C2H2 type
NT2RM4001987//IG superfamily
NT2RM4002013//WD domain, G-beta repeats
NT2RM4002073//AMP-binding enzymes
NT2RM4002093//RNA recognition motif. (aka RRM, RBD, or RNP domain)
NT2RM4002145//IG superfamily
NT2RM4002287//Fibronectin type III domain
NT2RM4002527//WD domain, G-beta repeats
NT2RM4002623//tRNA synthetases class II
NT2RP1000101//Zinc finger, C2H2 type
NT2RP1000202//Ank repeat
NT2RP1000272//RNA recognition motif. (aka RRM, RBD, or RNP domain)
NT2RP1000363//PH (pleckstrin homology) domain
NT2RP1000376//Ank repeat
NT2RP1000470//DEAD and DEAH box helicases
NT2RP1000478//Tubulin
NT2RP1000522//Ubiquitin carboxyl-terminal hydrolases family 2 //Ubiquitin carboxyl-terminal hydrolases family 2
NT2RP1000677//Kazal-type serine protease inhibitor domain
NT2RP1000701//WD domain, G-beta repeats
NT2RP1000733//Elongation factor Tu family (contains ATP/GTP binding P-loop)
NT2RP1000782//4 transmembrane segments integral membrane proteins
NT2RP1000833//3'5'-cyclic nucleotide phosphodiesterases
NT2RP1000856//4 transmembrane segments integral membrane proteins
NT2RP1000947//Ubiquitin-conjugating enzymes
NT2RP1000959//60s Acidic ribosomal protein
NT2RP1000966//RNA recognition motif. (aka RRM, RBD, or RNP domain)
NT2RP1001033//Tubulin
NT2RP1001080//DEAD and DEAH box helicases //Helicases conserved C-terminal domain
NT2RP1001177//Core histones H2A, H2B, H3 and H4
NT2RP1001247//Transforming growth factor beta like domain
NT2RP1001294//WD domain, G-beta repeats
NT2RP1001302//WD domain, G-beta repeats
NT2RP1001313//Heme-binding domain in cytochrome b5 and oxidoreductases
NT2RP1001457//WD domain, G-beta repeats
NT2RP1001546//4 transmembrane segments integral membrane proteins
NT2RP2000008//Zinc finger, C2H2 type
NT2RP2000040//C2 domain
NT2RP2000045//DnaJ, prokaryotic heat shock protein
NT2RP2000054//Zinc finger, C3HC4 type (RING finger)
NT2RP2000070//Cadherin
NT2RP2000126//Helicases conserved C-terminal domain //SNF2 and others N-terminal domain
NT2RP2000153//RNA recognition motif. (aka RRM, RBD, or RNP domain)
NT2RP2000224//PH (pleckstrin, homology) domain
NT2RP2000257//Mitochondrial carrier proteins
NT2RP2000329//Adenylate kinases
NT2RP2000414//RNA recognition motif. (aka RRM, RBD, or RNP domain)
NT2RP2000448//PH (pleckstrin homology) domain
NT2RP2000660//ATPases associated with various cellular activities (AAA)
NT2RP2000668//Eukaryotic protein kinase domain
NT2RP2000710//tRNA synthetases class II
NT2RP2000764//Aminotransferases class-V
NT2RP2000842//7 transmembrane receptor (rhodopsin family)
NT2RP2000880//Elongation factor Tu family (contains ATP/GTP binding P-loop)
NT2RP2000931//RNA recognition motif. (aka RRM, RBD, or RNP domain)
NT2RP2000932//Ank repeat
NT2RP2001081//C2 domain
NT2RP2001174//Zinc finger, C2H2 type
NT2RP2001397//Cyclins
NT2RP2001520//Mitochondrial carrier proteins
NT2RP2001597//Zinc finger, C3HC4 type (RING finger)
NT2RP2001740//Ubiquitin carboxyl-terminal hydrolases family 2
NT2RP2001748//Polyprenyl synthetases
NT2RP2001756//Zinc finger, C2H2 type
NT2RP2001839//Eukaryotic protein kinase domain
NT2RP2001900//Actins
NT2RP2001991//Sodium:neurotransmitter symporter family
NT2RP2002058//WD domain, G-beta repeats
NT2RP2002124//Ubiquitin carboxyl-terminal hydrolases family 2 //Ubiquitin carboxyl-terminal hydrolases family 2
NT2RP2002185//Ubiquitin family
NT2RP2002208//Zinc finger, C3HC4 type (RING finger)
NT2RP2002256//Cytochrome P450
NT2RP2002479//ABC transporters
NT2RP2002503//Zinc finger, C2H2 type
NT2RP2002520//Ank repeat
NT2RP2002591//Zinc finger, C2H2 type
NT2RP2002741//Src homology domain 3
NT2RP2002929//WD domain, G-beta repeats
NT2RP2002939//Zinc finger, C2H2 type
NT2RP2002959//Ubiquitin-conjugating enzymes
NT2RP2002980//Ribosomal protein S10
NT2RP2003137//Ubiquitin family
NT2RP2003164//Eukaryotic protein kinase domain
NT2RP2003228//MCM2/3/5 family
NT2RP2003243//Fibronectin type III domain
NT2RP2003272//Ubiquitin family
NT2RP2003307//Kinesin light chain repeat
NT2RP2003401//Ubiquitin carboxyl-terminal hydrolases family 2 //Ubiquitin carboxyl-terminal hydrolases, family 2
NT2RP2003433//eubacterial secY protein
NT2RP2003480//Zinc finger, C2H2 type
NT2RP2003713//Ubiquitin carboxyl-terminal hydrolases family 2
NT2RP2003737//Ubiquitin-conjugating enzymes
NT2RP2003777//Zinc finger, C3HC4 type (RING finger)
NT2RP2003840//Ubiquitin-conjugating enzymes
NT2RP2003857//Ank repeat
NT2RP2003981//Zinc finger, C3HC4 type (RING finger)
NT2RP2004170//WD domain, G-beta repeats
NT2RP2004187//Zinc finger, C2H2 type
NT2RP2004232//Phorbol esters / diacylglycerol binding domain //PH (pleckstrin homology) domain //Eukaryotic protein kinase domain
NT2RP2004389//Ribosomal protein S9
NT2RP2004538//PH (pleckstrin homology) domain
NT2RP2004568//DEAD and DEAH box helicases //Helicases conserved C-terminal domain
NT2RP2004710//WW/rsp5/WWP domain containing proteins
NT2RP2004768//Eukaryotic protein kinase domain
NT2RP2004933//Eukaryotic protein kinase domain
NT2RP2004961//Zinc finger, C2H2 type
NT2RP2005003//Zinc finger, C3HC4 type (RING finger)
NT2RP2005012//DnaJ, prokaryotic heat shock protein
NT2RP2005126//DEAD and DEAH box helicases //Helicases conserved C-terminal domain
NT2RP2005139//Ank repeat
NT2RP2005140//PH (pleckstrin homology) domain
NT2RP2005239//Aminotransferases class-V
NT2RP2005288//Regulator of chromosome condensation (RCC1)
NT2RP2005293//PH (pleckstrin homology) domain
NT2RP2005325//Homeobox domain //LIM domain containing proteins
NT2RP2005344//E1-E2 ATPases
NT2RP2005465//Mitochondrial carrier proteins
NT2RP2005525//Forkhead-associated (FHA) domain
NT2RP2005531//Band 4.1 family
NT2RP2005557//Bacterial mutT protein
NT2RP2005654//DnaJ, prokaryotic heat shock protein
NT2RP2005701//Zinc finger, C3HC4 type (RING finger)
NT2RP2005722//Zinc finger, C2H2 type
NT2RP2005752//TNFR/NGFR cysteine-rich region
NT2RP2005763//DEAD and DEAH box helicases //Helicases conserved C-terminal domain
NT2RP2005767//HMG (high mobility group) box
NT2RP2006312//HMG (high mobility group) box
NT2RP2006464//HMG (high mobility group) box
NT2RP2006571//Cytochrome P450
NT2RP3000050//Zinc finger, C2H2 type
NT2RP3000068//PH (pleckstrin homology) domain
NT2RP3000085//Biotin-requiring enzymes //Carbamoyl-phosphate synthase (CPSase)
NT2RP3000299//Src homology domain 3
NT2RP3000359//Adenylate kinases
NT2RP3000366//Ras family (contains ATP/GTP binding P-loop)
NT2RP3000403//WW/rsp5/WWP domain containing proteins
NT2RP3000487//WW/rsp5/WWP domain containing proteins
NT2RP3000512//Homeobox domain
NT2RP3000527//Zinc finger, C2H2 type
NT2RP3000531//IG superfamily
NT2RP3000590//Zinc finger, C3HC4 type (RING finger)
NT2RP3000603//Helix-loop-helix DNA-binding domain
NT2RP3000605//Zinc finger, C2H2 type
NT2RP3000632//Zinc finger, C2H2 type
NT2RP3000742//Phosphatidylinositol-specific phospholipase C, X domain //Phosphatidylinositol-specific phospholipase C, Y domain
NT2RP3000759//ADP-ribosylation factors (Arf family) (contains ATP/GTP binding P-loop)
NT2RP3000825//EGF-like domain
NT2RP3000869//ATPases associated with various cellular activities (AAA)
NT2RP3000994//Double-stranded RNA binding motif
NT2RP3001057//Zinc finger, C2H2 type
NT2RP3001084//PH (pleckstrin homology) domain
NT2RP3001120//Zinc finger, C2H2 type
NT2RP3001140//Thrombospondin type 1 domain
NT2RP3001150//Forkhead-associated (FHA) domain
NT2RP3001155//HMG (high mobility group) box
NT2RP3001214//Zinc finger, C2H2 type
NT2RP3001268//Zinc finger, C2H2 type
NT2RP3001338//Zinc finger, C2H2 type
NT2RP3001355//Mitochondrial carrier proteins
NT2RP3001398//Zinc finger, C2H2 type
NT2RP3001426//DnaJ, prokaryotic heat shock protein
NT2RP3001453//ABC transporters
NT2RP3001457//PH (pleckstrin homology) domain
NT2RP3001472//HMG (high mobility group) box
NT2RP3001495//Alcohol/other dehydrogenases, short chain type //WW/rsp5/WWP domain containing proteins
NT2RP3001497//Zinc finger, C3HC4 type (RING finger)
NT2RP3001724//Helicases conserved C-terminal domain
NT2RP3001792//RNA recognition motif. (aka RRM, RBD, or RNP domain)
NT2RP3001943//Zinc finger, C3HC4 type (RING finger)
NT2RP3001944//Zinc finger, C3HC4 type (RING finger)
NT2RP3002007//ATPases associated with various cellular activities (AAA)
NT2RP3002054//Low-density lipoprotein receptor domain class A
NT2RP3002151//Elongation factor Tu family (contains ATP/GTP binding P-loop)
NT2RP3002399//MCM2/3/5 family
NT2RP3002501//Serine/threonine dehydratases
NT2RP3002602//Thioredoxins
NT2RP3002628//DnaJ, prokaryotic heat shock protein //Thioredoxins
NT2RP3002663//PH (pleckstrin homology) domain
NT2RP3002909//Ank repeat
NT2RP3002953//Cadherin
NT2RP3002969//AMP-binding enzymes
NT2RP3003061//Ank repeat
NT2RP3003145//Zinc carboxypeptidases
NT2RP3003230//WD domain, G-beta repeats
NT2RP3003251//Zinc finger, C3HC4 type (RING finger)
NT2RP3003278//Ank repeat //Zinc finger, C2H2 type
NT2RP3003282//PH (pleckstrin homology) domain
NT2RP3003311//PH (pleckstrin homology) domain
NT2RP3003385//Ank repeat //Chaperonins clpA/B
NT2RP3003589//Ras family (contains ATP/GTP binding P-loop)
NT2RP3003621//CUB domain //Kringle domain
NT2RP3003701//Thrombospondin type 1 domain
NT2RP3003716//Fibronectin type III domain
NT2RP3003809//ATPases associated with various cellular activities (AAA)
NT2RP3004016//Zinc finger, C3HC4 type (RING finger)
NT2RP3004207//CUB domain //Sushi domain
NT2RP3004209//Ubiquitin carboxyl-terminal hydrolases family 2 //Ubiquitin carboxyl-terminal hydrolases family 2
NT2RP3004242//PH (pleckstrin homology) domain
NT2RP3004262//DnaJ, prokaryotic heat shock protein
NT2RP3004566//Zinc finger, C2H2 type
NT2RP3004569//Ank repeat
NT2RP3004594//HMG (high mobility group) box
NT2RP3004617//Zinc finger, C3HC4 type (RING finger)
NT2RP4000259//Glutathione peroxidases
NT2RP4000370//Prokaryotic-type class I peptide chain release factors
NT2RP4000376//WD domain, G-beta repeats
NT2RP4000398//Zinc finger, C2H2 type
NT2RP4000455//Forkhead-associated (FHA) domain //Zinc finger, C3HC4 type (RING finger)
NT2RP4000457//Ubiquitin carboxyl-terminal hydrolases family 2
NT2RP4000518//DEAD and DEAH box helicases //Helicases conserved C-terminal domain
NT2RP4000588//Actinin-type actin-binding domain containing proteins
NT2RP4000614//RNA recognition motif. (aka RRM, RBD, or RNP domain)
NT2RP4000648//Forkhead-associated (FHA) domain //Zinc finger, C3HC4 type (RING finger)
NT2RP4000837//Zinc finger, C2H2 type
NT2RP4000839//WD domain, G-beta repeats
NT2RP4000865//Zinc finger, C2H2 type
NT2RP4000907//Fibronectin type III domain //IG superfamily
NT2RP4000925//Fibronectin type III domain
NT2RP4000927//Ubiquitin carboxyl-terminal hydrolases family 2 //Ubiquitin carboxyl-terminal hydrolases family 2
NT2RP4000973//DnaJ, prokaryotic heat shock protein //Thioredoxins
NT2RP4001079//E1-E2 ATPases
NT2RP4001080//RNA recognition motif. (aka RRM, RBD, or RNP domain)
NT2RP4001117//eubacterial secY protein
NT2RP4001150//Fibronectin type III domain
NT2RP4001213//Zinc finger, C2H2 type
NT2RP4001219//Thioredoxins
NT2RP4001235//Zinc finger, CCHC class
NT2RP4001433//Zinc finger, C2H2 type
NT2RP4001498//Ank repeat
NT2RP4001568//Ank repeat
NT2RP4001644//Eukaryotic protein kinase domain
NT2RP4001725//WD domain, G-beta repeats
NT2RP4001753//Zinc finger, C2H2 type
NT2RP4001790//Zinc finger, C2H2 type
NT2RP4001822//4 transmembrane segments integral membrane proteins
NT2RP4001823//Fibrinogen beta and gamma chains, C-terminal globular domain
NT2RP4001893//Ank repeat
NT2RP4001896//WD domain, G-beta repeats
NT2RP4001927//WD domain, G-beta repeats
NT2RP4001938//Zinc finger, C2H2 type
NT2RP4002047//Elongation factor Tu family (contains ATP/GTP binding P-loop)
NT2RP4002078//Zinc finger, C2H2 type
NT2RP4002408//Eukaryotic protein kinase domain
NT2RP4002905//Cyclins
NT2RP5003477//WD domain, G-beta repeats
OVARC1000006//Core histones H2A, H2B, H3 and H4
OVARC1000085//Proteasome A-type and B-type
OVARC1000148//RNA recognition motif. (aka RRM, RBD, or RNP domain)
OVARC1000556//Eukaryotic protein kinase domain
OVARC1000649//PH (pleckstrin homology) domain //Src homology domain 2
OVARC1000746//Double-stranded RNA binding motif
OVARC1000885//Alcohol/other dehydrogenases, short chain type
OVARC1000937//Cyclins
OVARC1000999//Ank repeat
OVARC1001154//Granulins
OVARC1001180//Ubiquitin family
OVARC1001306//Helix-loop-helix DNA-binding domain
OVARC1001577//RNA recognition motif. (aka RRM, RBD, or RNP domain)
OVARC1001731//Tropomyosins
OVARC1001943//Zinc finger, C2H2 type
OVARC1002050//Spectrin alpha chain, repeated domain
OVARC1002112//Core histones H2A, H2B, H3 and H4
OVARC1002138//ATPases associated with various cellular activities (AAA)
OVARC1002182//WD domain, G-beta repeats
PLACE1000014//Zinc finger, C3HC4 type (RING finger)
PLACE1000040//Ras family (contains ATP/GTP binding P-loop)
PLACE1000050//Zinc finger, C2H2 type
PLACE1000081//PH (pleckstrin homology) domain
PLACE1000142//Enoyl-CoA hydratase/isomerase
PLACE1000401//IG superfamily
PLACE1000406//RNA recognition motif. (aka RRM, RBD, or RNP domain)
PLACE1000420//Bacterial mutT protein
PLACE1000706//Bromodomain
PLACE1000769//KH domain family of RNA binding proteins
PLACE1000786//PH (pleckstrin homology) domain
PLACE1000863//Ribosomal protein S4
PLACE1000909//Ank repeat
PLACE1000972//Src homology domain 3
PLACE1000979//Zinc finger, C2H2 type
PLACE1001304//Zinc finger, C2H2 type
PLACE1001387//Src homology domain 3
PLACE1001632//Zinc finger, C2H2 type
PLACE1001672//Aminotransferases class-III pyridoxal-phosphate
PLACE1001716//Zinc finger, CCHC class
PLACE1001739//DEAD and DEAH box helicases //Helicases conserved C-terminal domain
PLACE1001781//Phosphoglucomutase and phosphomannomutase phosphoserine
PLACE1001869//FGGY family of carbohydrate kinases
PLACE1002438//Zinc finger, C2H2 type
PLACE1002450//Zinc finger, C2H2 type
PLACE1002474//EGF-like domain //von Willebrand factor type A domain
PLACE1002499//Zinc finger, C3HC4 type (RING finger)
PLACE1002532//Homeobox domain
PLACE1002571//Actins
PLACE1002685//Src homology domain 2
PLACE1002722//7 transmembrane receptor (rhodopsin family)
PLACE1002775//Bromodomain
PLACE1002834//Zinc finger, C2H2 type
PLACE1003100//Alcohol/other dehydrogenases, short chain type
PLACE1003174//Ubiquitin-conjugating enzymes
PLACE1003238//7 transmembrane receptor (rhodopsin family)
PLACE1003302//Zinc finger, C2H2 type
PLACE1003334//RNA recognition motif. (aka RRM, RBD, or RNP domain)
PLACE1003366//C2 domain
PLACE1003394//Ras family (contains ATP/GTP binding P-loop)
PLACE1003420//Mitochondrial carrier proteins
PLACE1003493//C1q domain
PLACE1003519//KH domain family of RNA binding-proteins
PLACE1003723//Src homology domain 2
PLACE1003738//Zinc finger, C2H2 type
PLACE1003888//C2 domain //Phosphatidylinositol-specific phospholipase C, X domain //Phosphatidylinositol-specific phospholipase C, Y domain
PLACE1004128//WD domain, G-beta repeats
PLACE1004358//PH (pleckstrin homology) domain
PLACE1004428//Acyl-CoA dehydrogenases
PLACE1004437//Isocitrate and isopropylmalate dehydrogenases
PLACE1004506//LIM domain containing proteins
PLACE1004674//EF hand
PLACE1004918//L-lactate dehydrogenases
PLACE1005243//Eukaryotic protein kinase domain
PLACE1005305//Adenylate kinases
PLACE1005327//Src homology domain 3
PLACE1005530//Zinc finger, C3HC4 type (RING finger)
PLACE1005646//Helicases conserved C-terminal domain
PLACE1005656//Ribonucleotide reductases
PLACE1005966//WD domain, G-beta repeats
PLACE1006157//Sushi domain
PLACE1006196//DEAH and DEAR box helicases //Helicases conserved C-terminal domain
PLACE1006438//Zinc finger, C2H2 type
PLACE1006626//Double-stranded RNA binding motif
PLACE1006754//IG superfamily
PLACE1006829//Ubiquitin carboxyl-terminal hydrolases family 2 //Ubiquitin carboxyl-terminal hydrolases family 2
PLACE1006917//RNA recognition motif. (aka RRM, RBD, or RNP domain)
PLACE1006956//ABC transporters
PLACE1006958//Heat shock hsp70 proteins
PLACE1007375//C2 domain
PLACE1007488//PH (pleckstrin homology) domain
PLACE1007511//Intermediate filament proteins
PLACE1007537//Ank repeat
PLACE1007544//Zinc finger, C2H2 type
PLACE1007547//Zinc finger, C3HC4 type (RING finger)
PLACE1007598//Zinc finger, C2H2 type
PLACE1007697//ABC transporters
PLACE1007958//3'5'-cyclic nucleotide phosphodiesterases
PLACE1007969//RNA recognition motif. (aka RRM, RBD, or RNP domain)
PLACE1008201//Zinc finger, C2H2 type
PLACE1008429//Ank repeat
PLACE1008465//Zinc finger, C2H2 type
PLACE1008650//WD domain, G-beta repeats
PLACE1009020//Aminotransferases class-V
PLACE1009094//von Willebrand factor type C domain
PLACE1009099//Zinc finger, C2H2 type
PLACE1009246//LIM domain containing proteins
PLACE1009468//WD domain, G-beta repeats
PLACE1009476//DEAD and DEAH box helicases //Helicases conserved C-terminal domain
PLACE1009524//PH (pleckstrin homology) domain
PLACE1009596//WD domain, G-beta repeats
PLACE1009622//Double-stranded RNA binding motif
PLACE1009861//Cysteine proteases
PLACE1009925//Helicases conserved C-terminal domain
PLACE1009992//CUB domain //EGF-like domain //Sushi domain //Trypsin
PLACE1010053//Double-stranded RNA binding motif
PLACE1010089//Ubiquitin carboxyl-terminal hydrolases family 2
PLACE1010702//Zinc finger, C2H2 type
PLACE1010833//EF hand
PLACE1010926//Src homology domain 3
PLACE1010960//Actins
PLACE1011041//Src homology domain 3
PLACE1011046//C2 domain //Phosphatidylinositol-specific phospholipase C, X domain //Phosphatidylinositol-specific phospholipase C, Y domain
PLACE1011114//Helicases conserved C-terminal domain
PLACE1011160//Zinc finger, C3HC4 type (RING finger)
PLACE1011263//Ank repeat
PLACE1011433//Zinc finger, C2H2 type
PLACE1011576//Zinc finger, C2H2 type
PLACE1011923//Eukaryotic protein kinase domain
PLACE2000034//Fibronectin type III domain //IG superfamily
PLACE2000072//Zinc finger, C2H2 type
PLACE2000111//IG superfamily
PLACE2000164//WD domain, G-beta repeats
PLACE2000216//PH (pleckstrin homology) domain
PLACE2000341//Sodium:solute symporter family
PLACE2000371//Src homology domain 2
PLACE2000373//Thrombospondin type 1 domain
PLACE2000398//IG superfamily
PLACE2000427//Helicases conserved C-terminal domain
PLACE2000458//Cadherin
PLACE3000020//Guanylate cyclases
PLACE3000169//Zinc finger, C2H2 type
PLACE4000014//Helicases conserved C-terminal domain
PLACE4000052//ABC transporters
PLACE4000192//Zinc finger, C2H2 type
PLACE4000211//Bromodomain
PLACE4000431//Helicases conserved C-terminal domain
PLACE4000522//Ank repeat
PLACE4000581//EGF-like domain //Sushi domain
PLACE4000654//Ubiquitin-conjugating enzymes
THYRO1000072//IG superfamily
THYRO1000242//Zinc finger, C2H2 type
THYRO1000288//Zinc-binding metalloprotease domain
THYRO1000488//Zinc finger, C3HC4 type (RING finger)
THYRO1000501//Zinc finger, C3HC4 type (RING finger)
THYRO1000666//Kinesin motor domain
THYRO1000748//Src homology domain 3
THYRO1000926//3' 5'-cyclic nucleotide phosphodiesterases
THYRO1001661//RNA recognition motif. (aka RRM, RBD, or RNP domain)
THYRO1001671//Ubiquitin family
Y79AA1000037//Zinc finger, C3HC4 type (RING finger)
Y79AA1000214//Core histones H2A, H2B, H3 and H4
Y79AA1000342//Zinc finger, C2H2 type
Y79AA1000349//Double-stranded RNA binding motif
Y79AA1000627//Zinc finger, C2H2 type
Y79AA1000705//Helicases conserved C-terminal domain
Y79AA1000752//KH domain family of RNA binding proteins
Y79AA1000833//Tubulin
Y79AA1001048//Acyl-CoA dehydrogenases
Y79AA1001391//Homeobox domain
Y79AA1001394//ATPases associated with various cellular activities (AAA)
Y79AA1001493//Ubiquitin-conjugating enzymes
Y79AA1001613//Zinc finger, C2H2 type
Y79AA1001874//TNFR/NGFR cysteine-rich region
Y79AA1002027//Ubiquitin-conjugating enzymes
Y79AA1002139//DnaJ, prokaryotic heat shock protein
Y79AA1002208//Ank repeat
Y79AA1002246//C2 domain
Y79AA1002307//Fibronectin type III domain
Y79AA1002472//Zinc finger, C2H2 type
HEMBA1003538//CUB domain HEMBA1003645//WD domain, G-beta repeats //Src homology domain 3 HEMBA1005206//Glutathione S-transferases.
HEMBA1006521//Alcohol/other dehydrogenases, short chain type
HEMBB1001482//Zinc finger, C2H2 type HEMBB1001915//Ubiquitin carboxyl-terminal hydrolases family 2 //Ubiquitin carboxyl-terminal hydrolases family 2 HEMBB1002044//Cadherin MAMMA1000183//Zinc finger, C2H2 type
MAMMA1000897//von Willebrand factor type A domain MAMMA1001080//IG superfamily MAMMA1002498//IG superfamily MAMMA1002573//KH domain family of RNA binding proteins MAMMA1002617//Zinc finger, C2H2 type
NT2RM1000833//eubacterial secY protein NT2RM2001797//Zinc finger, C2H2 type
NT2RP1001013//Zinc finger, C2H2 type NT2RP2001233//Zinc finger, C2H2 type
NT2RP2001440//14-3-3 proteins NT2RP2002105//7 transmembrane receptor (rhodopsin family) NT2RP3001723//Laminin G domain NT2RP3001938//Eukaryotic protein kinase domain NT2RP3002330//Elongation factor Tu family (contains ATP/GTP binding P-loop) NT2RP3003133//Zinc finger, C2H2 type
NT2RP3003500//Eukaryotic protein kinase domain NT2RP3003799//C2 domain
NT2RP3003800//Eukaryotic protein kinase domain NT2RP3004013//Double-stranded RNA binding motif NT2RP3004125//Zinc finger, C2H2 type
OVARC1001244//Bromodomain OVARC1001496//D-isomer specific 2-hydroxyacid dehydrogenases PLACE1000007//Ubiquitin carboxyl-terminal hydrolases family 2 //Ubiquitin carboxyl-terminal hydrolases family 2 PLACE1001118//Zinc finger, C2H2 type PLACE1010310//Zinc finger, C2H2 type PLACE1011896//wnt family of developmental signaling proteins PLACE3000124//Src homology domain 2
PLACE4000100//D-isomer specific 2-hydroxyacid dehydrogenases
PLACE4000259//Helicases conserved C-terminal domain PLACE4000261//Bromodomain SKNMC1000013//ABC transporters SKNMC1000091//Basic region plus leucine zipper transcription factors THYRO1000343//Src homology domain 3 THYRO1000569//Zinc finger, C2H2 type THYRO1001189//Zinc finger, C2H2 type Y79AA1002103//Zinc finger, C2H2 type PLACE3000350//Eukaryotic protein kinase domain
PLACE4000156//Zinc finger, C2H2 type

### EXAMPLE 18

### Classification of cDNA clones into functional categories based on the full-length nucleotide sequences

Prediction of functions of proteins encoded by the clones and the categorization thereof were performed based on the results of homology search (see Homology search results 6, 12, 13 and 14) of the databases, GenBank, Swiss-Prot and UniGene, for the full-length nucleotide sequences of 4997 clones and based on the results of domain search (see Example 17) of the deduced amino acid sequences encoded by the full-length nucleotide sequences. The target 4997 clones are listed below:
HEMBA1000005, HEMBA1000012, HEMBA1000020, HEMBA1000030, HEMBA1000042, HEMBA1000046, HEMBA1000050, HEMBA1000076, HEMBA1000129, HEMBA1000141, HEMBA1000150, HEMBA1000156, HEMBA1000158, HEMBA1000168, HEMBA1000185, HEMBA1000193, HEMBA1000201, HEMBA1000213, HEMBA1000216, HEMBA1000227, HEMBA1000231, HEMBA1000243, HEMBA1000244, HEMBA1000251, HEMBA1000264, HEMBA1000280, HEMBA1000282, HEMBA1000288, HEMBA1000290, HEMBA1000302, HEMBA1000303, HEMBA1000304, HEMBA1000307, HEMBA1000327, HEMBA1000333, HEMBA1000338, HEMBA1000351, HEMBA1000356, HEMBA1000357, HEMBA1000369, HEMBA1000376, HEMBA1000387, HEMBA1000392, HEMBA1000396, HEMBA1000411, HEMBA1000428, HEMBA1000442, HEMBA1000456, HEMBA1000459, HEMBA1000460, HEMBA1000469, HEMBA1000488, HEMBA1000491, HEMBA1000497, HEMBA1000501, HEMBA1000504, HEMBA1000505, HEMBA1000508, HEMBA1000518, HEMBA1000519, HEMBA1000520, HEMBA1000523, HEMBA1000531, HEMBA1000534, HEMBA1000542, HEMBA1000545, HEMBA1000555, HEMBA1000557, HEMBA1000561, HEMBA1000568, HEMBA1000569, HEMBA1000575, HEMBA1000588, HEMBA1000591, HEMBA1000592, HEMBA1000594, HEMBA1000604, HEMBA1000608, HEMBA1000622, HEMBA1000636, HEMBA1000637, HEMBA1000655, HEMBA1000657, HEMBA1000673, HEMBA1000682, HEMBA1000686, HEMBA1000702, HEMBA1000719, HEMBA1000722, HEMBA1000726, HEMBA1000727, HEMBA1000749, HEMBA1000752, HEMBA1000769, HEMBA1000773, HEMBA1000774, HEMBA1000817, HEMBA1000822, HEMBA1000843, HEMBA1000851, HEMBA1000852, HEMBA1000867, HEMBA1000869, HEMBA1000870, HEMBA1000872, HEMBA1000876, HEMBA1000908, HEMBA1000910, HEMBA1000918, HEMBA1000919, HEMBA1000934, HEMBA1000942, HEMBA1000943, HEMBA1000946, HEMBA1000960, HEMBA1000968, HEMBA1000971, HEMBA1000972, HEMBA1000975, HEMBA1000985, HEMBA1000986, HEMBA1000991, HEMBA1001008, HEMBA1001009, HEMBA1001019, HEMBA1001020, HEMBA1001022, HEMBA1001024, HEMBA1001026, HEMBA1001043, HEMBA1001051, HEMBA1001052, HEMBA1001059, HEMBA1001060, HEMBA1001071, HEMBA1001077, HEMBA1001080, HEMBA1001085, HEMBA1001088, HEMBA1001094, HEMBA1001099, HEMBA1001109, HEMBA1001121, HEMBA1001122, HEMBA1001123, HEMBA1001133, HEMBA1001137, HEMBA1001140, HEMBA1001174, HEMBA1001197, HEMBA1001208, HEMBA1001213, HEMBA1001226, HEMBA1001235, HEMBA1001247, HEMBA1001257, HEMBA1001281, HEMBA1001286, HEMBA1001299, HEMBA1001302, HEMBA1001303, HEMBA1001310, HEMBA1001319, HEMBA1001323, HEMBA1001326, HEMBA1001327, HEMBA1001330, HEMBA1001351, HEMBA1001361, HEMBA1001375, HEMBA1001377, HEMBA1001383, HEMBA1001387, HEMBA1001388, HEMBA1001391, HEMBA1001398, HEMBA1001405, HEMBA1001407, HEMBA1001411, HEMBA1001413, HEMBA1001415, HEMBA1001432, HEMBA1001433, HEMBA1001435, HEMBA1001442, HEMBA1001446, HEMBA1001450, HEMBA1001455, HEMBA1001463, HEMBA1001497, HEMBA1001510, HEMBA1001515, HEMBA1001522, HEMBA1001526, HEMBA1001533, HEMBA1001557, HEMBA1001566, HEMBA1001569, HEMBA1001570, HEMBA1001579, HEMBA1001581, HEMBA1001589, HEMBA1001595, HEMBA1001608, HEMBA1001620, HEMBA1001635, HEMBA1001636, HEMBA1001640, HEMBA1001647, HEMBA1001651, HEMBA1001655, HEMBA1001658, HEMBA1001661, HEMBA1001672, HEMBA1001675, HEMBA1001702, HEMBA1001711, HEMBA1001712, HEMBA1001714, HEMBA1001723, HEMBA1001731, HEMBA1001734, HEMBA1001744, HEMBA1001745, HEMBA1001746, HEMBA1001750, HEMBA1001781, HEMBA1001784, HEMBA1001791, HEMBA1001803, HEMBA1001804, HEMBA1001809, HEMBA1001815, HEMBA1001819, HEMBA1001820, HEMBA1001822, HEMBA1001824, HEMBA1001835, HEMBA1001847, HEMBA1001864, HEMBA1001866, HEMBA1001869, HEMBA1001888, HEMBA1001896, HEMBA1001910, HEMBA1001912, HEMBA1001913, HEMBA1001915, HEMBA1001918, HEMBA1001921, HEMBA1001939, HEMBA1001940, HEMBA1001942, HEMBA1001950, HEMBA1001964, HEMBA1001967, HEMBA1001987, HEMBA1002018, HEMBA1002022, HEMBA1002035, HEMBA1002039, HEMBA1002049, HEMBA1002084, HEMBA1002092, HEMBA1002100, HEMBA1002102, HEMBA1002113, HEMBA1002119, HEMBA1002125, HEMBA1002139, HEMBA1002150, HEMBA1002151, HEMBA1002160, HEMBA1002161, HEMBA1002162, HEMBA1002166, HEMBA1002177, HEMBA1002185, HEMBA1002189, HEMBA1002191, HEMBA1002199, HEMBA1002204, HEMBA1002212, HEMBA1002215, HEMBA1002229, HEMBA1002237, HEMBA1002241, HEMBA1002265, HEMBA1002267, HEMBA1002328, HEMBA1002337, HEMBA1002341, HEMBA1002348, HEMBA1002349, HEMBA1002363, HEMBA1002381, HEMBA1002417, HEMBA1002419, HEMBA1002430, HEMBA1002439, HEMBA1002458, HEMBA1002460, HEMBA1002462, HEMBA1002469, HEMBA1002475, HEMBA1002477, HEMBA1002486, HEMBA1002495, HEMBA1002498, HEMBA1002503, HEMBA1002508, HEMBA1002513, HEMBA1002515, HEMBA1002538, HEMBA1002542, HEMBA1002547, HEMBA1002552, HEMBA1002555, HEMBA1002558, HEMBA1002569, HEMBA1002583, HEMBA1002609, HEMBA1002621, HEMBA1002624, HEMBA1002629, HEMBA1002645, HEMBA1002659, HEMBA1002661, HEMBA1002666, HEMBA1002678, HEMBA1002679, HEMBA1002688, HEMBA1002696, HEMBA1002703, HEMBA1002712, HEMBA1002716, HEMBA1002742, HEMBA1002746, HEMBA1002748, HEMBA1002750, HEMBA1002768, HEMBA1002770, HEMBA1002777, HEMBA1002779, HEMBA1002780, HEMBA1002794, HEMBA1002801, HEMBA1002810, HEMBA1002816, HEMBA1002818, HEMBA1002826, HEMBA1002833, HEMBA1002850, HEMBA1002863, HEMBA1002876, HEMBA1002921, HEMBA1002934, HEMBA1002935, HEMBA1002937, HEMBA1002939, HEMBA1002944, HEMBA1002951, HEMBA1002954, HEMBA1002968, HEMBA1002970, HEMBA1002971, HEMBA1002973, HEMBA1002997, HEMBA1002999, HEMBA1003021, HEMBA1003033, HEMBA1003034, HEMBA1003035, HEMBA1003037, HEMBA1003041, HEMBA1003046, HEMBA1003067, HEMBA1003071, HEMBA1003077, HEMBA1003078, HEMBA1003079, HEMBA1003083, HEMBA1003086, HEMBA1003096, HEMBA1003098, HEMBA1003117, HEMBA1003129, HEMBA1003133, HEMBA1003136, HEMBA1003142, HEMBA1003148, HEMBA1003166, HEMBA1003175, HEMBA1003179, HEMBA1003197, HEMBA1003199, HEMBA1003202, HEMBA1003220, HEMBA1003222, HEMBA1003229, HEMBA1003235, HEMBA1003250, HEMBA1003257, HEMBA1003273, HEMBA1003276, HEMBA1003278, HEMBA1003281, HEMBA1003286, HEMBA1003291, HEMBA1003304, HEMBA1003309, HEMBA1003322, HEMBA1003327, HEMBA1003328, HEMBA1003369, HEMBA1003370, HEMBA1003373, HEMBA1003376, HEMBA1003380, HEMBA1003384, HEMBA1003395, HEMBA1003402, HEMBA1003408, HEMBA1003417, HEMBA1003418, HEMBA1003433, HEMBA1003447, HEMBA1003461, HEMBA1003463, HEMBA1003480, HEMBA1003528, HEMBA1003531, HEMBA1003538, HEMBA1003545, HEMBA1003548, HEMBA1003555, HEMBA1003556, HEMBA1003560, HEMBA1003568, HEMBA1003569, HEMBA1003571, HEMBA1003579, HEMBA1003581, HEMBA1003591, HEMBA1003597, HEMBA1003598, HEMBA1003615, HEMBA1003617, HEMBA1003621, HEMBA1003645, HEMBA1003646, HEMBA1003656, HEMBA1003662, HEMBA1003667, HEMBA1003679, HEMBA1003680, HEMBA1003684, HEMBA1003690, HEMBA1003692, HEMBA1003711, HEMBA1003720, HEMBA1003725, HEMBA1003729, HEMBA1003733, HEMBA1003742, HEMBA1003758, HEMBA1003760, HEMBA1003773, HEMBA1003783, HEMBA1003799, HEMBA1003803, HEMBA1003804, HEMBA1003805, HEMBA1003807, HEMBA1003827, HEMBA1003836, HEMBA1003838, HEMBA1003854, HEMBA1003856, HEMBA1003864, HEMBA1003866, HEMBA1003879, HEMBA1003880, HEMBA1003893, HEMBA1003908, HEMBA1003926, HEMBA1003937, HEMBA1003939, HEMBA1003942, HEMBA1003953, HEMBA1003958, HEMBA1003959, HEMBA1003976, HEMBA1003978, HEMBA1003985, HEMBA1003987, HEMBA1003989, HEMBA1004011, HEMBA1004012, HEMBA1004015, HEMBA1004024, HEMBA1004038, HEMBA1004045, HEMBA1004048, HEMBA1004055, HEMBA1004056, HEMBA1004074, HEMBA1004086, HEMBA1004097, HEMBA1004111, HEMBA1004131, HEMBA1004138, HEMBA1004143, HEMBA1004146, HEMBA1004150, HEMBA1004168, HEMBA1004193, HEMBA1004199, HEMBA1004200, HEMBA1004202, HEMBA1004203, HEMBA1004207, HEMBA1004225, HEMBA1004227, HEMBA1004238, HEMBA1004241, HEMBA1004246, HEMBA1004248, HEMBA1004267, HEMBA1004272, HEMBA1004274, HEMBA1004275, HEMBA1004276, HEMBA1004286, HEMBA1004289, HEMBA1004295, HEMBA1004312, HEMBA1004321, HEMBA1004323, HEMBA1004327, HEMBA1004330, HEMBA1004335, HEMBA1004341, HEMBA1004353, HEMBA1004354, HEMBA1004356, HEMBA1004366, HEMBA1004372, HEMBA1004389, HEMBA1004394, HEMBA1004396, HEMBA1004405, HEMBA1004408, HEMBA1004429, HEMBA1004433, HEMBA1004460, HEMBA1004461, HEMBA1004479, HEMBA1004499, HEMBA1004502, HEMBA1004507, HEMBA1004509, HEMBA1004534, HEMBA1004538, HEMBA1004542, HEMBA1004554, HEMBA1004560, HEMBA1004573, HEMBA1004577, HEMBA1004596, HEMBA1004604, HEMBA1004610, HEMBA1004617, HEMBA1004629, HEMBA1004631, HEMBA1004632, HEMBA1004637, HEMBA1004638, HEMBA1004669, HEMBA1004670, HEMBA1004672, HEMBA1004693, HEMBA1004697, HEMBA1004705, HEMBA1004709, HEMBA1004711, HEMBA1004725, HEMBA1004730, HEMBA1004733, HEMBA1004734, HEMBA1004736, HEMBA1004748, HEMBA1004751, HEMBA1004752, HEMBA1004753, HEMBA1004756, HEMBA1004758, HEMBA1004763, HEMBA1004768, HEMBA1004771, HEMBA1004776, HEMBA1004778, HEMBA1004795, HEMBA1004803, HEMBA1004806, HEMBA1004807, HEMBA1004820, HEMBA1004847, HEMBA1004850, HEMBA1004860, HEMBA1004863, HEMBA1004865, HEMBA1004880, HEMBA1004889, HEMBA1004900, HEMBA1004909, HEMBA1004923, HEMBA1004929, HEMBA1004930, HEMBA1004933, HEMBA1004934, HEMBA1004944, HEMBA1004954, HEMBA1004960, HEMBA1004972, HEMBA1004973, HEMBA1004977, HEMBA1004978, HEMBA1004980, HEMBA1004983, HEMBA1004995, HEMBA1005009, HEMBA1005019, HEMBA1005029, HEMBA1005035, HEMBA1005039, HEMBA1005047, HEMBA1005050, HEMBA1005062, HEMBA1005066, HEMBA1005075, HEMBA1005079, HEMBA1005083, HEMBA1005101, HEMBA1005113, HEMBA1005123, HEMBA1005133, HEMBA1005149, HEMBA1005152, HEMBA1005185, HEMBA1005201, HEMBA1005202, HEMBA1005206, HEMBA1005219, HEMBA1005223, HEMBA1005232, HEMBA1005241, HEMBA1005252, HEMBA1005275, HEMBA1005293, HEMBA1005296, HEMBA1005311, HEMBA1005314, HEMBA1005331, HEMBA1005338, HEMBA1005359, HEMBA1005367, HEMBA1005374, HEMBA1005382, HEMBA1005394, HEMBA1005403, HEMBA1005411, HEMBA1005423, HEMBA1005426, HEMBA1005443, HEMBA1005447, HEMBA1005468, HEMBA1005469, HEMBA1005472, HEMBA1005474, HEMBA1005475, HEMBA1005497, HEMBA1005500, HEMBA1005506, HEMBA1005508, HEMBA1005513, HEMBA1005517, HEMBA1005518, HEMBA1005526, HEMBA1005528, HEMBA1005530, HEMBA1005548, HEMBA1005552, HEMBA1005558, HEMBA1005568, HEMBA1005572, HEMBA1005576, HEMBA1005581, HEMBA1005582, HEMBA1005583, HEMBA1005588, HEMBA1005593, HEMBA1005595, HEMBA1005606, HEMBA1005609, HEMBA1005616, HEMBA1005621, HEMBA1005627, HEMBA1005666, HEMBA1005670, HEMBA1005679, HEMBA1005680, HEMBA1005685, HEMBA1005699, HEMBA1005705, HEMBA1005732, HEMBA1005737, HEMBA1005746, HEMBA1005755, HEMBA1005780, HEMBA1005813, HEMBA1005815, HEMBA1005822, HEMBA1005834, HEMBA1005852, HEMBA1005884, HEMBA1005891, HEMBA1005894, HEMBA1005909, HEMBA1005911, HEMBA1005921, HEMBA1005931, HEMBA1005963, HEMBA1005990, HEMBA1005991, HEMBA1006005, HEMBA1006031, HEMBA1006035, HEMBA1006036, HEMBA1006038, HEMBA1006067, HEMBA1006081, HEMBA1006090, HEMBA1006091, HEMBA1006092, HEMBA1006100, HEMBA1006108, HEMBA1006121, HEMBA1006124, HEMBA1006130, HEMBA1006138, HEMBA1006155, HEMBA1006158, HEMBA1006173, HEMBA1006182, HEMBA1006198, HEMBA1006235, HEMBA1006248, HEMBA1006252, HEMBA1006253, HEMBA1006259, HEMBA1006268, HEMBA1006272, HEMBA1006278, HEMBA1006283, HEMBA1006284, HEMBA1006291, HEMBA1006293, HEMBA1006309, HEMBA1006310, HEMBA1006344, HEMBA1006347, HEMBA1006349, HEMBA1006359, HEMBA1006364, HEMBA1006377, HEMBA1006380, HEMBA1006381, HEMBA1006398, HEMBA1006406, HEMBA1006416, HEMBA1006421, HEMBA1006424, HEMBA1006426, HEMBA1006445, HEMBA1006446, HEMBA1006461, HEMBA1006467, HEMBA1006474, HEMBA1006483, HEMBA1006485, HEMBA1006486, HEMBA1006492, HEMBA1006494, HEMBA1006497, HEMBA1006502, HEMBA1006507, HEMBA1006521, HEMBA1006530, HEMBA1006535, HEMBA1006546, HEMBA1006559, HEMBA1006562, HEMBA1006566, HEMBA1006579, HEMBA1006583, HEMBA1006595, HEMBA1006597, HEMBA1006612, HEMBA1006617, HEMBA1006624, HEMBA1006631, HEMBA1006639, HEMBA1006643, HEMBA1006650, HEMBA1006652, HEMBA1006659, HEMBA1006665, HEMBA1006674, HEMBA1006676, HEMBA1006682, HEMBA1006695, HEMBA1006708, HEMBA1006709, HEMBA1006717, HEMBA1006737, HEMBA1006744, HEMBA1006754, HEMBA1006758, HEMBA1006767, HEMBA1006779, HEMBA1006780, HEMBA1006789, HEMBA1006795, HEMBA1006796, HEMBA1006807, HEMBA1006812, HEMBA1006824, HEMBA1006832, HEMBA1006865, HEMBA1006877, HEMBA1006885, HEMBA1006900, HEMBA1006914, HEMBA1006921, HEMBA1006926, HEMBA1006936, HEMBA1006941, HEMBA1006949, HEMBA1006973, HEMBA1006976, HEMBA1006993, HEMBA1007002, HEMBA1007018, HEMBA1007051, HEMBA1007052, HEMBA1007062, HEMBA1007066, HEMBA1007073, HEMBA1007078, HEMBA1007080, HEMBA1007085, HEMBA1007087, HEMBA1007112, HEMBA1007113, HEMBA1007121, HEMBA1007129, HEMBA1007147, HEMBA1007151, HEMBA1007174, HEMBA1007178, HEMBA1007194, HEMBA1007203, HEMBA1007206, HEMBA1007224, HEMBA1007243, HEMBA1007251, HEMBA1007256, HEMBA1007267, HEMBA1007281, HEMBA1007288, HEMBA1007300, HEMBA1007301, HEMBA1007319, HEMBA1007320, HEMBA1007322, HEMBA1007327, HEMBA1007341, HEMBA1007342, HEMBA1007347, HEMBB1000005, HEMBB1000008, HEMBB1000018, HEMBB1000024, HEMBB1000025, HEMBB1000030, HEMBB1000036, HEMBB1000037, HEMBB1000048, HEMBB1000050, HEMBB1000054, HEMBB1000059, HEMBB1000083, HEMBB1000089, HEMBB1000099, HEMBB1000103, HEMBB1000113, HEMBB1000119, HEMBB1000136, HEMBB1000141, HEMBB1000144, HEMBB1000173, HEMBB1000175, HEMBB1000198, HEMBB1000215, HEMBB1000217, HEMBB1000218, HEMBB1000226, HEMBB1000240, HEMBB1000244, HEMBB1000264, HEMBB1000266, HEMBB1000272, HEMBB1000274, HEMBB1000312, HEMBB1000317, HEMBB1000318, HEMBB1000335, HEMBB1000336, HEMBB1000337, HEMBB1000338, HEMBB1000339, HEMBB1000341, HEMBB1000343, HEMBB1000354, HEMBB1000374, HEMBB1000391, HEMBB1000402, HEMBB1000420, HEMBB1000434, HEMBB1000438, HEMBB1000441, HEMBB1000449, HEMBB1000480, HEMBB1000491, HEMBB1000493, HEMBB1000510, HEMBB1000530, HEMBB1000550, HEMBB1000554, HEMBB1000556, HEMBB1000573, HEMBB1000586, HEMBB1000589, HEMBB1000591, HEMBB1000592, HEMBB1000593, HEMBB1000623, HEMBB1000630, HEMBB1000631, HEMBB1000632, HEMBB1000637, HEMBB1000649, HEMBB1000652, HEMBB1000671, HEMBB1000672, HEMBB1000673, HEMBB1000684, HEMBB1000693, HEMBB1000705, HEMBB1000706, HEMBB1000709, HEMBB1000725, HEMBB1000726, HEMBB1000749, HEMBB1000763, HEMBB1000770, HEMBB1000774, HEMBB1000781, HEMBB1000789, HEMBB1000790, HEMBB1000807, HEMBB1000810, HEMBB1000822, HEMBB1000826, HEMBB1000827, HEMBB1000831, HEMBB1000835, HEMBB1000848, HEMBB1000852, HEMBB1000870, HEMBB1000883, HEMBB1000887, HEMBB1000888, HEMBB1000890, HEMBB1000893, HEMBB1000908, HEMBB1000913, HEMBB1000915, HEMBB1000917, HEMBB1000927, HEMBB1000947, HEMBB1000973, HEMBB1000975, HEMBB1000985, HEMBB1000991, HEMBB1000996, HEMBB1001004, HEMBB1001008, HEMBB1001011, HEMBB1001014, HEMBB1001020, HEMBB1001024, HEMBB1001047, HEMBB1001051, HEMBB1001056, HEMBB1001058, HEMBB1001060, HEMBB1001068, HEMBB1001096, HEMBB1001105, HEMBB1001112, HEMBB1001114, HEMBB1001117, HEMBB1001119, HEMBB1001126, HEMBB1001133, HEMBB1001137, HEMBB1001142, HEMBB1001151, HEMBB1001153, HEMBB1001169, HEMBB1001175, HEMBB1001177, HEMBB1001182, HEMBB1001197, HEMBB1001199, HEMBB1001208, HEMBB1001209, HEMBB1001210, HEMBB1001221, HEMBB1001234, HEMBB1001242, HEMBB1001249, HEMBB1001253, HEMBB1001254, HEMBB1001271, HEMBB1001282, HEMBB1001288, HEMBB1001289. HEMBB1001294, HEMBB1001302, HEMBB1001304, HEMBB1001314, HEMBB1001315, HEMBB1001317, HEMBB1001331, HEMBB1001335, HEMBB1001337, HEMBB1001339, HEMBB1001346, HEMBB1001348, HEMBB1001356, HEMBB1001364, HEMBB1001366, HEMBB1001367, HEMBB1001369, HEMBB1001384, HEMBB1001387, HEMBB1001394, HEMBB1001410, HEMBB1001424, HEMBB1001426, HEMBB1001429, HEMBB1001436, HEMBB1001443, HEMBB1001449, HEMBB1001458, HEMBB1001482, HEMBB1001500, HEMBB1001521, HEMBB1001527, HEMBB1001531, HEMBB1001535, HEMBB1001536, HEMBB1001537, HEMBB1001562, HEMBB1001564, HEMBB1001565, HEMBB1001585, HEMBB1001588, HEMBB1001603, HEMBB1001618, HEMBB1001619, HEMBB1001630, HEMBB1001635, HEMBB1001653, HEMBB1001665, HEMBB1001668, HEMBB1001673, HEMBB1001684, HEMBB1001685, HEMBB1001695, HEMBB1001707, HEMBB1001735, HEMBB1001736, HEMBB1001747, HEMBB1001749, HEMBB1001753, HEMBB1001756, HEMBB1001760, HEMBB1001785, HEMBB1001797, HEMBB1001802, HEMBB1001812, HEMBB1001816, HEMBB1001831, HEMBB1001834, HEMBB1001839, HEMBB1001850, HEMBB1001863, HEMBB1001868, HEMBB1001869, HEMBB1001871, HEMBB1001872, HEMBB1001874, HEMBB1001880, HEMBB1001899, HEMBB1001905, HEMBB1001906, HEMBB1001908, HEMBB1001910, HEMBB1001911, HEMBB1001915, HEMBB1001921, HEMBB1001922, HEMBB1001925, HEMBB1001930, HEMBB1001944, HEMBB1001945, HEMBB1001947, HEMBB1001950, HEMBB1001952, HEMBB1001957, HEMBB1001962, HEMBB1001967, HEMBB1001983, HEMBB1001990, HEMBB1001996, HEMBB1002002, HEMBB1002005, HEMBB1002042, HEMBB1002043, HEMBB1002044, HEMBB1002045, HEMBB1002049, HEMBB1002050, HEMBB1002068, HEMBB1002092, HEMBB1002134, HEMBB1002139, HEMBB1002142, HEMBB1002152, HEMBB1002190, HEMBB1002193, HEMBB1002217, HEMBB1002218, HEMBB1002232, HEMBB1002247, HEMBB1002249, HEMBB1002266, HEMBB1002280, HEMBB1002300, HEMBB1002327, HEMBB1002329, HEMBB1002342, HEMBB1002358, HEMBB1002359, HEMBB1002371, HEMBB1002381, HEMBB1002383, HEMBB1002387, HEMBB1002409, HEMBB1002415, HEMBB1002425, HEMBB1002442, HEMBB1002453, HEMBB1002457, HEMBB1002458, HEMBB1002477, HEMBB1002489, HEMBB1002492, HEMBB1002495, HEMBB1002502, HEMBB1002510, HEMBB1002520, HEMBB1002522, HEMBB1002534, HEMBB1002545, HEMBB1002550, HEMBB1002579, HEMBB1002582, HEMBB1002596, HEMBB1002600, HEMBB1002603, HEMBB1002607, HEMBB1002610, HEMBB1002613, HEMBB1002617, HEMBB1002623, HEMBB1002635, HEMBB1002677, HEMBB1002683, HEMBB1002684, HEMBB1002692, HEMBB1002697, HEMBB1002699, HEMBB1002702, HEMBB1002705, MAMMA1000009, MAMMA1000019, MAMMA1000020, MAMMA1000025, MAMMA1000043, MAMMA1000045, MAMMA1000055, MAMMA1000057, MAMMA1000069, MAMMA1000084, MAMMA1000085, MAMMA1000092, MAMMA1000103, MAMMA1000117, MAMMA1000129, MAMMA1000133, MAMMA1000139, MAMMA1000143, MAMMA1000155, MAMMA1000163, MAMMA1000171, MAMMA1000173, MAMMA1000175, MAMMA1000183, MAMMA1000198, MAMMA1000227, MAMMA1000241, MAMMA1000251, MAMMA1000254, MAMMA1000257, MAMMA1000264, MAMMA1000266, MAMMA1000270, MAMMA1000277, MAMMA1000278, MAMMA1000279, MAMMA1000284, MAMMA1000287, MAMMA1000307, MAMMA1000309, MAMMA1000312, MAMMA1000313, MAMMA1000331, MAMMA1000339, MAMMA1000340, MAMMA1000348, MAMMA1000356, MAMMA1000360, MAMMA1000361, MAMMA1000372, MAMMA1000388, MAMMA1000395, MAMMA1000402, MAMMA1000410, MAMMA1000414, MAMMA1000416, MAMMA1000421, MAMMA1000422, MAMMA1000431, MAMMA1000444, MAMMA1000458, MAMMA1000468, MAMMA1000472, MAMMA1000490, MAMMA1000500, MAMMA1000522, MAMMA1000524, MAMMA1000559, MAMMA1000567, MAMMA1000576, MAMMA1000583, MAMMA1000594, MAMMA1000605, MAMMA1000612, MAMMA1000616, MAMMA1000623, MAMMA1000625, MAMMA1000643, MAMMA1000664, MAMMA1000670, MAMMA1000672, MAMMA1000684, MAMMA1000696, MAMMA1000707, MAMMA1000713, MAMMA1000714, MAMMA1000720, MAMMA1000731, MAMMA1000734, MAMMA1000738, MAMMA1000744, MAMMA1000746, MAMMA1000752, MAMMA1000760, MAMMA1000761, MAMMA1000775, MAMMA1000776, MAMMA1000778, MAMMA1000798, MAMMA1000802, MAMMA1000824, MAMMA1000831, MAMMA1000839, MAMMA1000841, MAMMA1000842, MAMMA1000843, MAMMA1000851, MAMMA1000855, MAMMA1000856, MAMMA1000859, MAMMA1000863, MAMMA1000865, MAMMA1000867, MAMMA1000875, MAMMA1000876, MAMMA1000880, MAMMA1000883, MAMMA1000897, MAMMA1000906, MAMMA1000908, MAMMA1000914, MAMMA1000921, MAMMA1000931, MAMMA1000940, MAMMA1000941, MAMMA1000956, MAMMA1000957, MAMMA1000962, MAMMA1000968, MAMMA1000975, MAMMA1000979, MAMMA1000987, MAMMA1001003, MAMMA1001008, MAMMA1001021, MAMMA1001030, MAMMA1001038, MAMMA1001041, MAMMA1001059, MAMMA1001073, MAMMA1001075, MAMMA1001078, MAMMA1001080, MAMMA1001082, MAMMA1001091, MAMMA1001105, MAMMA1001110, MAMMA1001126, MAMMA1001139, MAMMA1001143, MAMMA1001154, MAMMA1001162, MAMMA1001181, MAMMA1001186, MAMMA1001191, MAMMA1001198, MAMMA1001202, MAMMA1001206, MAMMA1001215, MAMMA1001220, MAMMA1001222, MAMMA1001243, MAMMA1001244, MAMMA1001249, MAMMA1001252, MAMMA1001256, MAMMA1001259, MAMMA1001260, MAMMA1001268, MAMMA1001271, MAMMA1001274, MAMMA1001292, MAMMA1001296, MAMMA1001305, MAMMA1001322, MAMMA1001324, MAMMA1001341, MAMMA1001343, MAMMA1001388, MAMMA1001397, MAMMA1001408, MAMMA1001411, MAMMA1001419, MAMMA1001420, MAMMA1001442, MAMMA1001452, MAMMA1001465, MAMMA1001476, MAMMA1001487, MAMMA1001501, MAMMA1001502, MAMMA1001510, MAMMA1001522, MAMMA1001547, MAMMA1001551, MAMMA1001575, MAMMA1001576, MAMMA1001590, MAMMA1001600, MAMMA1001604, MAMMA1001606, MAMMA1001620, MAMMA1001627, MAMMA1001630, MAMMA1001633, MAMMA1001635, MAMMA1001649, MAMMA1001663, MAMMA1001670, MAMMA1001671, MAMMA1001679, MAMMA1001683, MAMMA1001686, MAMMA1001692, MAMMA1001711, MAMMA1001715, MAMMA1001730, MAMMA1001735, MAMMA1001743, MAMMA1001744, MAMMA1001745, MAMMA1001751, MAMMA1001754, MAMMA1001757, MAMMA1001760, MAMMA1001764, MAMMA1001768, MAMMA1001769, MAMMA1001771, MAMMA1001783, MAMMA1001785, MAMMA1001788, MAMMA1001790, MAMMA1001806, MAMMA1001812, MAMMA1001815, MAMMA1001817, MAMMA1001818, MAMMA1001820, MAMMA1001824, MAMMA1001837, MAMMA1001848, MAMMA1001851, MAMMA1001854, MAMMA1001858, MAMMA1001864, MAMMA1001868, MAMMA1001874, MAMMA1001878, MAMMA1001890, MAMMA1001907, MAMMA1001908, MAMMA1001931, MAMMA1001956, MAMMA1001969, MAMMA1001970, MAMMA1002009, MAMMA1002011, MAMMA1002032, MAMMA1002033, MAMMA1002041, MAMMA1002042, MAMMA1002047, MAMMA1002056, MAMMA1002058, MAMMA1002068, MAMMA1002078, MAMMA1002082, MAMMA1002084, MAMMA1002093, MAMMA1002094, MAMMA1002118, MAMMA1002125, MAMMA1002132, MAMMA1002140, MAMMA1002143, MAMMA1002145, MAMMA1002153, MAMMA1002155, MAMMA1002156, MAMMA1002170, MAMMA1002174, MAMMA1002198, MAMMA1002209, MAMMA1002215, MAMMA1002219, MAMMA1002230, MAMMA1002236, MAMMA1002243, MAMMA1002250, MAMMA1002268, MAMMA1002269, MAMMA1002282, MAMMA1002292, MAMMA1002293, MAMMA1002294, MAMMA1002297, MAMMA1002298, MAMMA1002299, MAMMA1002308, MAMMA1002310, MAMMA1002311, MAMMA1002312, MAMMA1002317, MAMMA1002319, MAMMA1002322, MAMMA1002329, MAMMA1002333, MAMMA1002339, MAMMA1002351, MAMMA1002352, MAMMA1002353, MAMMA1002355, MAMMA1002356, MAMMA1002359, MAMMA1002360, MAMMA1002362, MAMMA1002380, MAMMA1002384, MAMMA1002385, MAMMA1002392, MAMMA1002411, MAMMA1002413, MAMMA1002417, MAMMA1002427, MAMMA1002428, MAMMA1002434, MAMMA1002446, MAMMA1002454, MAMMA1002461, MAMMA1002470, MAMMA1002475, MAMMA1002485, MAMMA1002494, MAMMA1002498, MAMMA1002524, MAMMA1002530, MAMMA1002545, MAMMA1002554, MAMMA1002556, MAMMA1002566, MAMMA1002571, MAMMA1002573, MAMMA1002585, MAMMA1002590, MAMMA1002597, MAMMA1002598, MAMMA1002612, MAMMA1002617, MAMMA1002618, MAMMA1002619, MAMMA1002622, MAMMA1002636, MAMMA1002637, MAMMA1002646, MAMMA1002650, MAMMA1002655, MAMMA1002665, MAMMA1002671, MAMMA1002673, MAMMA1002684, MAMMA1002699, MAMMA1002708, MAMMA1002711, MAMMA1002721, MAMMA1002727, MAMMA1002728, MAMMA1002744, MAMMA1002748, MAMMA1002758, MAMMA1002764, MAMMA1002765, MAMMA1002769, MAMMA1002775, MAMMA1002780, MAMMA1002782, MAMMA1002796, MAMMA1002807, MAMMA1002820, MAMMA1002830, MAMMA1002833, MAMMA1002838, MAMMA1002842, MAMMA1002843, MAMMA1002844, MAMMA1002858, MAMMA1002868, MAMMA1002869, MAMMA1002880, MAMMA1002881, MAMMA1002886, MAMMA1002890, MAMMA1002892, MAMMA1002895, MAMMA1002909, MAMMA1002937, MAMMA1002938, MAMMA1002941, MAMMA1002947, MAMMA1002964, MAMMA1002972, MAMMA1002973, MAMMA1002987, MAMMA1003003, MAMMA1003004, MAMMA1003011, MAMMA1003013, MAMMA1003015, MAMMA1003019, MAMMA1003026, MAMMA1003031, MAMMA1003035, MAMMA1003039, MAMMA1003044, MAMMA1003047, MAMMA1003049, MAMMA1003056, MAMMA1003057, MAMMA1003066, MAMMA1003089, MAMMA1003099, MAMMA1003104, MAMMA1003113, MAMMA1003127, MAMMA1003135, MAMMA1003146, MAMMA1003150, MAMMA1003166, NT2RM1000001, NT2RM1000018, NT2RM1000032, NT2RM1000035, NT2RM1000037, NT2RM1000039, NT2RM1000055, NT2RM1000059, NT2RM1000062, NT2RM1000080, NT2RM1000086, NT2RM1000092, NT2RM1000118, NT2RM1000119, NT2RM1000127, NT2RM1000131, NT2RM1000132, NT2RM1000153, NT2RM1000186, NT2RM1000187, NT2RM1000199, NT2RM1000242, NT2RM1000244, NT2RM1000252, NT2RM1000256, NT2RM1000257, NT2RM1000260, NT2RM1000271, NT2RM1000272, NT2RM1000280, NT2RM1000300, NT2RM1000314, NT2RM1000341, NT2RM1000354, NT2RM1000355, NT2RM1000365, NT2RM1000377, NT2RM1000388, NT2RM1000399, NT2RM1000421, NT2RM1000430, NT2RM1000499, NT2RM1000539, NT2RM1000553, NT2RM1000555, NT2RM1000563, NT2RM1000623, NT2RM1000648, NT2RM1000661, NT2RM1000666, NT2RM1000669, NT2RM1000672, NT2RM1000691,
NT2RM1000699, NT2RM1000702, NT2RM1000741, NT2RM1000742, NT2RM1000746, NT2RM1000770, NT2RM1000772, NT2RM1000780, NT2RM1000781, NT2RM1000800, NT2RM1000802, NT2RM1000811, NT2RM1000826, NT2RM1000829, NT2RM1000833, NT2RM1000850, NT2RM1000852, NT2RM1000857, NT2RM1000867, NT2RM1000874, NT2RM1000882, NT2RM1000883, NT2RM1000885, NT2RM1000894, NT2RM1000898, NT2RM1000905, NT2RM1000924, NT2RM1000927, NT2RM1000962, NT2RM1000978, NT2RM1001003, NT2RM1001008, NT2RM1001043, NT2RM1001044, NT2RM1001059, NT2RM1001066, NT2RM1001072, NT2RM1001074, NT2RM1001082, NT2RM1001085, NT2RM1001092, NT2RM1001102, NT2RM1001105, NT2RM1001112, NT2RM1001115, NT2RM1001139, NT2RM2000006, NT2RM2000013, NT2RM2000030, NT2RM2000032, NT2RM2000042, NT2RM2000092, NT2RM2000093, NT2RM2000101, NT2RM2000191, NT2RM2000192, NT2RM2000239, NT2RM2000250, NT2RM2000259, NT2RM2000260, NT2RM2000287, NT2RM2000322, NT2RM2000359, NT2RM2000363, NT2RM2000368, NT2RM2000371, NT2RM2000374, NT2RM2000395, NT2RM2000402, NT2RM2000407, NT2RM2000420, NT2RM2000422, NT2RM2000452, NT2RM2000469, NT2RM2000490, NT2RM2000502, NT2RM2000504, NT2RM2000522, NT2RM2000540, NT2RM2000566, NT2RM2000567, NT2RM2000569, NT2RM2000577, NT2RM2000581, NT2RM2000588, NT2RM2000594, NT2RM2000599, NT2RM2000609, NT2RM2000612, NT2RM2000624, NT2RM2000635, NT2RM2000636, NT2RM2000639, NT2RM2000649, NT2RM2000669, NT2RM2000691, NT2RM2000714, NT2RM2000718, NT2RM2000735, NT2RM2000740, NT2RM2000795, NT2RM2000821, NT2RM2000837, NT2RM2000951, NT2RM2000952, NT2RM2000984, NT2RM2001004, NT2RM2001035, NT2RM2001065, NT2RM2001100, NT2RM2001105, NT2RM2001131, NT2RM2001141, NT2RM2001152, NT2RM2001177, NT2RM2001194, NT2RM2001196, NT2RM2001201, NT2RM2001221, NT2RM2001238, NT2RM2001243, NT2RM2001247, NT2RM2001256, NT2RM2001291, NT2RM2001306, NT2RM2001312, NT2RM2001319, NT2RM2001324, NT2RM2001345, NT2RM2001360, NT2RM2001370, NT2RM2001393, NT2RM2001420, NT2RM2001424, NT2RM2001499, NT2RM2001504, NT2RM2001524, NT2RM2001544, NT2RM2001547, NT2RM2001575, NT2RM2001582, NT2RM2001588, NT2RM2001592, NT2RM2001605, NT2RM2001613, NT2RM2001632, NT2RM2001635, NT2RM2001637, NT2RM2001641, NT2RM2001648, NT2RM2001652, NT2RM2001659, NT2RM2001664, NT2RM2001668, NT2RM2001670, NT2RM2001671, NT2RM2001675, NT2RM2001681, NT2RM2001688, NT2RM2001695, NT2RM2001696, NT2RM2001698, NT2RM2001699, NT2RM2001700, NT2RM2001706, NT2RM2001716, NT2RM2001718, NT2RM2001723, NT2RM2001727, NT2RM2001730, NT2RM2001743, NT2RM2001753, NT2RM2001760, NT2RM2001768, NT2RM2001771, NT2RM2001782, NT2RM2001784, NT2RM2001785, NT2RM2001797, NT2RM2001803, NT2RM2001805, NT2RM2001813, NT2RM2001823, NT2RM2001839, NT2RM2001840, NT2RM2001855, NT2RM2001867, NT2RM2001879, NT2RM2001886, NT2RM2001896, NT2RM2001903, NT2RM2001930, NT2RM2001935, NT2RM2001936, NT2RM2001950, NT2RM2001982, NT2RM2001983, NT2RM2001989, NT2RM2001997, NT2RM2001998, NT2RM2002004, NT2RM2002014, NT2RM2002030, NT2RM2002049, NT2RM2002055, NT2RM2002088, NT2RM2002091, NT2RM2002100, NT2RM2002109, NT2RM2002128, NT2RM2002142, NT2RM2002145, NT2RM2002178, NT2RM4000024, NT2RM4000027, NT2RM4000030, NT2RM4000046, NT2RM4000061, NT2RM4000086, NT2RM4000104, NT2RM4000139, NT2RM4000155, NT2RM4000156, NT2RM4000167, NT2RM4000169, NT2RM4000191, NT2RM4000197, NT2RM4000199, NT2RM4000200, NT2RM4000202, NT2RM4000210, NT2RM4000215, NT2RM4000229, NT2RM4000233, NT2RM4000244, NT2RM4000251, NT2RM4000265, NT2RM4000290, NT2RM4000324, NT2RM4000327, NT2RM4000344, NT2RM4000349, NT2RM4000354, NT2RM4000356, NT2RM4000386, NT2RM4000395, NT2RM4000414, NT2RM4000421, NT2RM4000425, NT2RM4000433, NT2RM4000457, NT2RM4000471, NT2RM4000486, NT2RM4000496, NT2RM4000511, NT2RM4000514, NT2RM4000515, NT2RM4000520, NT2RM4000531, NT2RM4000532, NT2RM4000534, NT2RM4000585, NT2RM4000590, NT2RM4000595, NT2RM4000603, NT2RM4000611, NT2RM4000616, NT2RM4000634, NT2RM4000657, NT2RM4000674, NT2RM4000689, NT2RM4000698, NT2RM4000700, NT2RM4000712, NT2RM4000717, NT2RM4000733, NT2RM4000734, NT2RM4000741, NT2RM4000751, NT2RM4000764, NT2RM4000778, NT2RM4000783, NT2RM4000787, NT2RM4000790, NT2RM4000795, NT2RM4000796, NT2RM4000798, NT2RM4000813, NT2RM4000820, NT2RM4000833, NT2RM4000848, NT2RM4000852, NT2RM4000855, NT2RM4000857, NT2RM4000887, NT2RM4000895, NT2RM4000950, NT2RM4000971, NT2RM4000979, NT2RM4000996,

NT2RM4001002, NT2RM4001032, NT2RM4001047, NT2RM4001054, NT2RM4001084, NT2RM4001092, NT2RM4001116, NT2RM4001140, NT2RM4001151, NT2RM4001155, NT2RM4001160, NT2RM4001178, NT2RM4001187, NT2RM4001191, NT2RM4001200, NT2RM4001203, NT2RM4001204, NT2RM4001217, NT2RM4001256, NT2RM4001258, NT2RM4001309, NT2RM4001313, NT2RM4001316, NT2RM4001320, NT2RM4001340, NT2RM4001344, NT2RM4001347, NT2RM4001371, NT2RM4001382, NT2RM4001384, NT2RM4001410, NT2RM4001411, NT2RM4001412, NT2RM4001414, NT2RM4001437, NT2RM4001444, NT2RM4001454, NT2RM4001455, NT2RM4001483, NT2RM4001489, NT2RM4001522, NT2RM4001557, NT2RM4001565, NT2RM4001566, NT2RM4001569, NT2RM4001582, NT2RM4001592, NT2RM4001594, NT2RM4001597, NT2RM4001611, NT2RM4001629, NT2RM4001650, NT2RM4001662, NT2RM4001666, NT2RM4001682, NT2RM4001710, NT2RM4001714, NT2RM4001715, NT2RM4001731, NT2RM4001746, NT2RM4001754, NT2RM4001758, NT2RM4001783, NT2RM4001810, NT2RM4001813, NT2RM4001819, NT2RM4001823, NT2RM4001828, NT2RM4001836, NT2RM4001841, NT2RM4001842, NT2RM4001856, NT2RM4001858, NT2RM4001865, NT2RM4001876, NT2RM4001880, NT2RM4001905, NT2RM4001922, NT2RM4001930, NT2RM4001938, NT2RM4001940, NT2RM4001953, NT2RM4001965, NT2RM4001969, NT2RM4001979, NT2RM4001984, NT2RM4001987, NT2RM4002013, NT2RM4002018, NT2RM4002034, NT2RM4002044, NT2RM4002054, NT2RM4002062, NT2RM4002063, NT2RM4002066, NT2RM4002073, NT2RM4002075, NT2RM4002093, NT2RM4002109, NT2RM4002128, NT2RM4002140, NT2RM4002145, NT2RM4002146, NT2RM4002161, NT2RM4002174, NT2RM4002189, NT2RM4002194, NT2RM4002205, NT2RM4002213, NT2RM4002226, NT2RM4002251, NT2RM4002256, NT2RM4002266, NT2RM4002281, NT2RM4002287, NT2RM4002294, NT2RM4002301, NT2RM4002323, NT2RM4002339, NT2RM4002344, NT2RM4002373, NT2RM4002374, NT2RM4002383, NT2RM4002390, NT2RM4002398, NT2RM4002409, NT2RM4002420, NT2RM4002438, NT2RM4002446, NT2RM4002452, NT2RM4002457, NT2RM4002460, NT2RM4002493, NT2RM4002504, NT2RM4002527, NT2RM4002532, NT2RM4002534, NT2RM4002558, NT2RM4002565, NT2RM4002567, NT2RM4002571, NT2RM4002593, NT2RM4002594, NT2RM4002623, NT2RP1000018, NT2RP1000035, NT2RP1000040, NT2RP1000063, NT2RP1000086, NT2RP1000101, NT2RP1000111, NT2RP1000112, NT2RP1000124, NT2RP1000130, NT2RP1000163, NT2RP1000170, NT2RP1000191, NT2RP1000202, NT2RP1000243, NT2RP1000259, NT2RP1000272, NT2RP1000324, NT2RP1000326, NT2RP1000333, NT2RP1000348, NT2RP1000357, NT2RP1000358, NT2RP1000363, NT2RP1000376, NT2RP1000409, NT2RP1000413, NT2RP1000416, NT2RP1000418, NT2RP1000439, NT2RP1000443, NT2RP1000460, NT2RP1000470, NT2RP1000478, NT2RP1000481, NT2RP1000493, NT2RP1000513, NT2RP1000522, NT2RP1000547, NT2RP1000574, NT2RP1000581, NT2RP1000609, NT2RP1000630, NT2RP1000677, NT2RP1000688, NT2RP1000695, NT2RP1000701, NT2RP1000721, NT2RP1000730, NT2RP1000733, NT2RP1000738, NT2RP1000746, NT2RP1000767, NT2RP1000782, NT2RP1000796, NT2RP1000825, NT2RP1000833, NT2RP1000834, NT2RP1000836, NT2RP1000846, NT2RP1000851, NT2RP1000856, NT2RP1000860, NT2RP1000902, NT2RP1000915, NT2RP1000916, NT2RP1000943, NT2RP1000944, NT2RP1000947, NT2RP1000954, NT2RP1000958, NT2RP1000959, NT2RP1000966, NT2RP1000980, NT2RP1000988, NT2RP1001011, NT2RP1001013, NT2RP1001014, NT2RP1001033, NT2RP1001073, NT2RP1001079, NT2RP1001080, NT2RP1001113, NT2RP1001173, NT2RP1001177, NT2RP1001185, NT2RP1001199, NT2RP1001247, NT2RP1001248, NT2RP1001253, NT2RP1001286, NT2RP1001294, NT2RP1001302, NT2RP1001310, NT2RP1001311, NT2RP1001313, NT2RP1001361, NT2RP1001385, NT2RP1001395, NT2RP1001410, NT2RP1001424, NT2RP1001432, NT2RP1001449, NT2RP1001457, NT2RP1001466, NT2RP1001475, NT2RP1001482, NT2RP1001494, NT2RP1001543, NT2RP1001546, NT2RP1001569, NT2RP1001616, NT2RP1001665, NT2RP2000001, NT2RP2000006, NT2RP2000007, NT2RP2000008, NT2RP2000027, NT2RP2000032, NT2RP2000040, NT2RP2000045, NT2RP2000054, NT2RP2000056, NT2RP2000067, NT2RP2000070, NT2RP2000076, NT2RP2000079, NT2RP2000088, NT2RP2000091, NT2RP2000097, NT2RP2000098, NT2RP2000108, NT2RP2000114, NT2RP2000120, NT2RP2000126, NT2RP2000133, NT2RP2000147, NT2RP2000153, NT2RP2000157, NT2RP2000161, NT2RP2000173, NT2RP2000175, NT2RP2000183, NT2RP2000195, NT2RP2000198, NT2RP2000205, NT2RP2000208, NT2RP2000224, NT2RP2000232, NT2RP2000233, NT2RP2000239, NT2RP2000248, NT2RP2000257, NT2RP2000258, NT2RP2000270, NT2RP2000274, NT2RP2000283, NT2RP2000288, NT2RP2000289, NT2RP2000297, NT2RP2000298, NT2RP2000310, NT2RP2000327, NT2RP2000328, NT2RP2000329, NT2RP2000337, NT2RP2000346, NT2RP2000369, NT2RP2000412, NT2RP2000414, NT2RP2000420, NT2RP2000422, NT2RP2000438, NT2RP2000448, NT2RP2000459, NT2RP2000498, NT2RP2000503, NT2RP2000510, NT2RP2000516, NT2RP2000523, NT2RP2000551, NT2RP2000603, NT2RP2000617, NT2RP2000634, NT2RP2000644, NT2RP2000656, NT2RP2000658, NT2RP2000660, NT2RP2000668, NT2RP2000678, NT2RP2000704, NT2RP2000710, NT2RP2000715, NT2RP2000758, NT2RP2000764, NT2RP2000809, NT2RP2000812, NT2RP2000814, NT2RP2000816, NT2RP2000819, NT2RP2000841, NT2RP2000842, NT2RP2000845, NT2RP2000863, NT2RP2000880, NT2RP2000892, NT2RP2000931, NT2RP2000932, NT2RP2000938, NT2RP2000943, NT2RP2000965, NT2RP2000970, NT2RP2000985, NT2RP2001036, NT2RP2001044, NT2RP2001056, NT2RP2001065, NT2RP2001070, NT2RP2001081, NT2RP2001094, NT2RP2001119, NT2RP2001127, NT2RP2001137, NT2RP2001149, NT2RP2001168, NT2RP2001173, NT2RP2001174, NT2RP2001196, NT2RP2001214, NT2RP2001218, NT2RP2001226, NT2RP2001233, NT2RP2001245, NT2RP2001268, NT2RP2001290, NT2RP2001295, NT2RP2001312, NT2RP2001327, NT2RP2001328, NT2RP2001347, NT2RP2001366, NT2RP2001378, NT2RP2001381, NT2RP2001392, NT2RP2001394, NT2RP2001397, NT2RP2001420, NT2RP2001427, NT2RP2001440, NT2RP2001450, NT2RP2001460, NT2RP2001467, NT2RP2001506, NT2RP2001511, NT2RP2001520, NT2RP2001536, NT2RP2001560, NT2RP2001576, NT2RP2001581, NT2RP2001597, NT2RP2001601, NT2RP2001613, NT2RP2001628, NT2RP2001634, NT2RP2001660, NT2RP2001663, NT2RP2001675, NT2RP2001677, NT2RP2001678, NT2RP2001720, NT2RP2001721, NT2RP2001740, NT2RP2001748, NT2RP2001756, NT2RP2001813, NT2RP2001839, NT2RP2001861, NT2RP2001869, NT2RP2001876, NT2RP2001883, NT2RP2001898, NT2RP2001900, NT2RP2001907, NT2RP2001936, NT2RP2001943, NT2RP2001946, NT2RP2001947, NT2RP2001969, NT2RP2001976, NT2RP2001985, NT2RP2001991, NT2RP2002025, NT2RP2002032, NT2RP2002033, NT2RP2002041, NT2RP2002046, NT2RP2002047, NT2RP2002056, NT2RP2002058, NT2RP2002066, NT2RP2002070, NT2RP2002076, NT2RP2002078, NT2RP2002079, NT2RP2002099, NT2RP2002105, NT2RP2002124, NT2RP2002154, NT2RP2002172, NT2RP2002185, NT2RP2002193, NT2RP2002208, NT2RP2002219, NT2RP2002231, NT2RP2002235, NT2RP2002252, NT2RP2002256, NT2RP2002270, NT2RP2002292, NT2RP2002312, NT2RP2002316, NT2RP2002325, NT2RP2002333, NT2RP2002373, NT2RP2002385, NT2RP2002408, NT2RP2002426, NT2RP2002439, NT2RP2002442, NT2RP2002464, NT2RP2002475, NT2RP2002479, NT2RP2002498, NT2RP2002503, NT2RP2002520, NT2RP2002537, NT2RP2002546, NT2RP2002549, NT2RP2002591, NT2RP2002595, NT2RP2002606, NT2RP2002609, NT2RP2002618, NT2RP2002621, NT2RP2002643, NT2RP2002672, NT2RP2002677, NT2RP2002701, NT2RP2002706, NT2RP2002710, NT2RP2002727, NT2RP2002736, NT2RP2002740, NT2RP2002741, NT2RP2002752, NT2RP2002753, NT2RP2002755, NT2RP2002769, NT2RP2002800, NT2RP2002843, NT2RP2002857, NT2RP2002862, NT2RP2002880, NT2RP2002891, NT2RP2002928, NT2RP2002929, NT2RP2002939, NT2RP2002954, NT2RP2002959, NT2RP2002979, NT2RP2002980, NT2RP2002986, NT2RP2002993, NT2RP2003000, NT2RP2003034, NT2RP2003073, NT2RP2003099, NT2RP2003101, NT2RP2003108, NT2RP2003117, NT2RP2003121, NT2RP2003125, NT2RP2003137, NT2RP2003157, NT2RP2003158, NT2RP2003161, NT2RP2003164, NT2RP2003165, NT2RP2003177, NT2RP2003194, NT2RP2003206, NT2RP2003228, NT2RP2003230, NT2RP2003237, NT2RP2003243, NT2RP2003265, NT2RP2003272, NT2RP2003277, NT2RP2003280, NT2RP2003286, NT2RP2003293, NT2RP2003295, NT2RP2003297, NT2RP2003307, NT2RP2003308, NT2RP2003329, NT2RP2003339, NT2RP2003347, NT2RP2003367, NT2RP2003391, NT2RP2003393, NT2RP2003394, NT2RP2003401, NT2RP2003433, NT2RP2003445, NT2RP2003446, NT2RP2003456, NT2RP2003466, NT2RP2003480, NT2RP2003506, NT2RP2003511, NT2RP2003513, NT2RP2003517, NT2RP2003522, NT2RP2003533, NT2RP2003543, NT2RP2003559, NT2RP2003564, NT2RP2003567, NT2RP2003581, NT2RP2003596, NT2RP2003604, NT2RP2003629, NT2RP2003643, NT2RP2003668, NT2RP2003687, NT2RP2003691, NT2RP2003702, NT2RP2003704, NT2RP2003713, NT2RP2003714, NT2RP2003727, NT2RP2003737, NT2RP2003751, NT2RP2003760, NT2RP2003764, NT2RP2003769, NT2RP2003777, NT2RP2003781, NT2RP2003793, NT2RP2003799, NT2RP2003825, NT2RP2003840, NT2RP2003857, NT2RP2003871, NT2RP2003885, NT2RP2003912, NT2RP2003952, NT2RP2003976, NT2RP2003981, NT2RP2003984, NT2RP2003986, NT2RP2003988, NT2RP2004013, NT2RP2004041, NT2RP2004042, NT2RP2004066, NT2RP2004081, NT2RP2004095, NT2RP2004098, NT2RP2004124, NT2RP2004142, NT2RP2004152, NT2RP2004165, NT2RP2004170, NT2RP2004187, NT2RP2004194, NT2RP2004207, NT2RP2004226, NT2RP2004232, NT2RP2004239, NT2RP2004242, NT2RP2004245, NT2RP2004270, NT2RP2004300, NT2RP2004316, NT2RP2004321, NT2RP2004339, NT2RP2004347, NT2RP2004364, NT2RP2004365, NT2RP2004366, NT2RP2004373, NT2RP2004389, NT2RP2004392, NT2RP2004396, NT2RP2004399, NT2RP2004400, NT2RP2004412, NT2RP2004425, NT2RP2004463, NT2RP2004476, NT2RP2004490, NT2RP2004523, NT2RP2004538, NT2RP2004551, NT2RP2004568, NT2RP2004580, NT2RP2004587, NT2RP2004594, NT2RP2004600, NT2RP2004602, NT2RP2004614, NT2RP2004655, NT2RP2004664, NT2RP2004675, NT2RP2004681, NT2RP2004689, NT2RP2004709, NT2RP2004710, NT2RP2004732, NT2RP2004743, NT2RP2004767, NT2RP2004768, NT2RP2004775, NT2RP2004791, NT2RP2004799, NT2RP2004802, NT2RP2004816, NT2RP2004841, NT2RP2004861, NT2RP2004897, NT2RP2004920, NT2RP2004933, NT2RP2004936, NT2RP2004959, NT2RP2004961, NT2RP2004962, NT2RP2004978, NT2RP2004982, NT2RP2004999, NT2RP2005000, NT2RP2005001, NT2RP2005003, NT2RP2005012, NT2RP2005018, NT2RP2005020, NT2RP2005022, NT2RP2005031, NT2RP2005037, NT2RP2005038, NT2RP2005116, NT2RP2005126, NT2RP2005139, NT2RP2005140, NT2RP2005144, NT2RP2005147, NT2RP2005159, NT2RP2005162, NT2RP2005168, NT2RP2005204, NT2RP2005227, NT2RP2005239, NT2RP2005254, NT2RP2005270, NT2RP2005276, NT2RP2005287, NT2RP2005288, NT2RP2005293, NT2RP2005315, NT2RP2005325, NT2RP2005336, NT2RP2005344, NT2RP2005358, NT2RP2005360, NT2RP2005393, NT2RP2005407, NT2RP2005436, NT2RP2005441, NT2RP2005453, NT2RP2005454, NT2RP2005457, NT2RP2005464, NT2RP2005465, NT2RP2005472, NT2RP2005476, NT2RP2005490, NT2RP2005491, NT2RP2005495, NT2RP2005496, NT2RP2005498, NT2RP2005501, NT2RP2005509, NT2RP2005520, NT2RP2005525, NT2RP2005531, NT2RP2005539, NT2RP2005540, NT2RP2005549, NT2RP2005555, NT2RP2005557, NT2RP2005600, NT2RP2005605, NT2RP2005620, NT2RP2005622, NT2RP2005635, NT2RP2005637, NT2RP2005640, NT2RP2005645, NT2RP2005654, NT2RP2005669, NT2RP2005675, NT2RP2005683, NT2RP2005690, NT2RP2005694, NT2RP2005701, NT2RP2005712, NT2RP2005719, NT2RP2005722, NT2RP2005723, NT2RP2005726, NT2RP2005732, NT2RP2005741, NT2RP2005748, NT2RP2005752, NT2RP2005753, NT2RP2005763, NT2RP2005767, NT2RP2005773, NT2RP2005775, NT2RP2005776, NT2RP2005781, NT2RP2005784, NT2RP2005804, NT2RP2005806, NT2RP2005812, NT2RP2005815, NT2RP2005835, NT2RP2005841, NT2RP2005853, NT2RP2005859, NT2RP2005868, NT2RP2005882, NT2RP2005886, NT2RP2005890, NT2RP2005901, NT2RP2005933, NT2RP2005942, NT2RP2005980, NT2RP2006023, NT2RP2006038, NT2RP2006043, NT2RP2006052, NT2RP2006069, NT2RP2006071, NT2RP2006100, NT2RP2006103, NT2RP2006106, NT2RP2006141, NT2RP2006166, NT2RP2006184, NT2RP2006186, NT2RP2006196, NT2RP2006200, NT2RP2006219, NT2RP2006237, NT2RP2006238, NT2RP2006258, NT2RP2006261, NT2RP2006275, NT2RP2006312, NT2RP2006321, NT2RP2006333, NT2RP2006334, NT2RP2006365, NT2RP2006393, NT2RP2006436, NT2RP2006441, NT2RP2006454, NT2RP2006456, NT2RP2006464, NT2RP2006467, NT2RP2006472, NT2RP2006534, NT2RP2006554, NT2RP2006565, NT2RP2006571, NT2RP2006573, NT2RP2006598, NT2RP3000002, NT2RP3000031, NT2RP3000046, NT2RP3000047, NT2RP3000050, NT2RP3000055, NT2RP3000068, NT2RP3000072, NT2RP3000080, NT2RP3000085, NT2RP3000092, NT2RP3000109, NT2RP3000134, NT2RP3000142, NT2RP3000149, NT2RP3000197, NT2RP3000207, NT2RP3000220, NT2RP3000233, NT2RP3000235, NT2RP3000247, NT2RP3000251, NT2RP3000252, NT2RP3000267, NT2RP3000299, NT2RP3000312, NT2RP3000320, NT2RP3000324, NT2RP3000333, NT2RP3000341, NT2RP3000348, NT2RP3000350, NT2RP3000359, NT2RP3000361, NT2RP3000366, NT2RP3000393, NT2RP3000397, NT2RP3000403, NT2RP3000418, NT2RP3000439, NT2RP3000441, NT2RP3000449, NT2RP3000451, NT2RP3000456, NT2RP3000484, NT2RP3000487, NT2RP3000512, NT2RP3000526, NT2RP3000527, NT2RP3000531, NT2RP3000542, NT2RP3000561, NT2RP3000562, NT2RP3000578, NT2RP3000584, NT2RP3000590, NT2RP3000592, NT2RP3000596, NT2RP3000599, NT2RP3000603, NT2RP3000605, NT2RP3000622, NT2RP3000624, NT2RP3000628, NT2RP3000632, NT2RP3000644, NT2RP3000661, NT2RP3000665, NT2RP3000685, NT2RP3000690, NT2RP3000736, NT2RP3000739, NT2RP3000742, NT2RP3000753, NT2RP3000759, NT2RP3000825, NT2RP3000826, NT2RP3000836, NT2RP3000841, NT2RP3000845, NT2RP3000850, NT2RP3000852, NT2RP3000859, NT2RP3000865, NT2RP3000868, NT2RP3000869, NT2RP3000875, NT2RP3000901, NT2RP3000917, NT2RP3000919, NT2RP3000968, NT2RP3000980, NT2RP3000994, NT2RP3001004, NT2RP3001007, NT2RP3001055, NT2RP3001057, NT2RP3001081, NT2RP3001084, NT2RP3001096, NT2RP3001107, NT2RP3001109, NT2RP3001111, NT2RP3001113, NT2RP3001115, NT2RP3001116, NT2RP3001119, NT2RP3001120, NT2RP3001126, NT2RP3001133, NT2RP3001140, NT2RP3001150, NT2RP3001155, NT2RP3001176, NT2RP3001214, NT2RP3001216, NT2RP3001221, NT2RP3001232, NT2RP3001236, NT2RP3001239, NT2RP3001245, NT2RP3001253, NT2RP3001260, NT2RP3001268, NT2RP3001272, NT2RP3001274, NT2RP3001281, NT2RP3001282, NT2RP3001297, NT2RP3001307, NT2RP3001318, NT2RP3001325, NT2RP3001338, NT2RP3001355, NT2RP3001356, NT2RP3001374, NT2RP3001383, NT2RP3001384, NT2RP3001392, NT2RP3001396, NT2RP3001398, NT2RP3001399, NT2RP3001407, NT2RP3001420, NT2RP3001426, NT2RP3001427, NT2RP3001428, NT2RP3001432, NT2RP3001447, NT2RP3001449, NT2RP3001453, NT2RP3001457, NT2RP3001459, NT2RP3001472, NT2RP3001495, NT2RP3001497, NT2RP3001527, NT2RP3001529, NT2RP3001538, NT2RP3001554, NT2RP3001580, NT2RP3001587, NT2RP3001589, NT2RP3001607, NT2RP3001608, NT2RP3001621, NT2RP3001629, NT2RP3001642, NT2RP3001646, NT2RP3001671, NT2RP3001672, NT2RP3001676, NT2RP3001678, NT2RP3001679, NT2RP3001688, NT2RP3001690, NT2RP3001698, NT2RP3001708, NT2RP3001712, NT2RP3001716, NT2RP3001723, NT2RP3001724, NT2RP3001727, NT2RP3001730, NT2RP3001739, NT2RP3001752, NT2RP3001777, NT2RP3001792, NT2RP3001799, NT2RP3001819, NT2RP3001844, NT2RP3001854, NT2RP3001855, NT2RP3001857, NT2RP3001896, NT2RP3001898, NT2RP3001915, NT2RP3001929, NT2RP3001931, NT2RP3001938, NT2RP3001943, NT2RP3001944, NT2RP3001969, NT2RP3002002, NT2RP3002004, NT2RP3002007, NT2RP3002014,

NT2RP3002033, NT2RP3002045, NT2RP3002054, NT2RP3002056, NT2RP3002062, NT2RP3002063, NT2RP3002081, NT2RP3002097, NT2RP3002099, NT2RP3002102, NT2RP3002108, NT2RP3002142, NT2RP3002146, NT2RP3002147, NT2RP3002151, NT2RP3002163, NT2RP3002165, NT2RP3002166, NT2RP3002173, NT2RP3002181, NT2RP3002244, NT2RP3002248, NT2RP3002255, NT2RP3002273, NT2RP3002276, NT2RP3002303, NT2RP3002304, NT2RP3002330, NT2RP3002343, NT2RP3002351, NT2RP3002399, NT2RP3002402, NT2RP3002455, NT2RP3002484, NT2RP3002501, NT2RP3002512, NT2RP3002529, NT2RP3002545, NT2RP3002549, NT2RP3002566, NT2RP3002587, NT2RP3002590, NT2RP3002602, NT2RP3002603, NT2RP3002628, NT2RP3002631, NT2RP3002650, NT2RP3002659, NT2RP3002660, NT2RP3002663, NT2RP3002671, NT2RP3002682, NT2RP3002687, NT2RP3002688, NT2RP3002701, NT2RP3002713, NT2RP3002763, NT2RP3002770, NT2RP3002785, NT2RP3002799, NT2RP3002810, NT2RP3002818, NT2RP3002861, NT2RP3002869, NT2RP3002876, NT2RP3002877, NT2RP3002909, NT2RP3002911, NT2RP3002948, NT2RP3002953, NT2RP3002955, NT2RP3002969, NT2RP3002972, NT2RP3002985, NT2RP3002988, NT2RP3003008, NT2RP3003032, NT2RP3003059, NT2RP3003061, NT2RP3003071, NT2RP3003078, NT2RP3003101, NT2RP3003121, NT2RP3003133, NT2RP3003138, NT2RP3003139, NT2RP3003145, NT2RP3003150, NT2RP3003155, NT2RP3003157, NT2RP3003185, NT2RP3003193, NT2RP3003197, NT2RP3003203, NT2RP3003204, NT2RP3003210, NT2RP3003212, NT2RP3003230, NT2RP3003242, NT2RP3003251, NT2RP3003264, NT2RP3003278, NT2RP3003282, NT2RP3003290, NT2RP3003301, NT2RP3003302, NT2RP3003311, NT2RP3003313, NT2RP3003327, NT2RP3003344, NT2RP3003346, NT2RP3003353, NT2RP3003377, NT2RP3003385, NT2RP3003403, NT2RP3003409, NT2RP3003411, NT2RP3003427, NT2RP3003433, NT2RP3003490, NT2RP3003491, NT2RP3003500, NT2RP3003543, NT2RP3003552, NT2RP3003555, NT2RP3003564, NT2RP3003572, NT2RP3003576, NT2RP3003589, NT2RP3003621, NT2RP3003625, NT2RP3003656, NT2RP3003659, NT2RP3003665, NT2RP3003672, NT2RP3003680, NT2RP3003686, NT2RP3003701, NT2RP3003716, NT2RP3003726, NT2RP3003795, NT2RP3003799, NT2RP3003800, NT2RP3003805, NT2RP3003809, NT2RP3003819, NT2RP3003825, NT2RP3003828, NT2RP3003831, NT2RP3003833, NT2RP3003842, NT2RP3003846, NT2RP3003870, NT2RP3003876, NT2RP3003914, NT2RP3003918, NT2RP3003932, NT2RP3003989, NT2RP3003992, NT2RP3004013, NT2RP3004016, NT2RP3004028, NT2RP3004041, NT2RP3004051, NT2RP3004070, NT2RP3004078, NT2RP3004093, NT2RP3004095, NT2RP3004125, NT2RP3004145, NT2RP3004148, NT2RP3004155, NT2RP3004189, NT2RP3004206, NT2RP3004207, NT2RP3004209, NT2RP3004215, NT2RP3004242, NT2RP3004246, NT2RP3004253, NT2RP3004258, NT2RP3004262, NT2RP3004282, NT2RP3004332, NT2RP3004341, NT2RP3004348, NT2RP3004349, NT2RP3004378, NT2RP3004424, NT2RP3004428, NT2RP3004451, NT2RP3004454, NT2RP3004466, NT2RP3004470, NT2RP3004472, NT2RP3004480, NT2RP3004490, NT2RP3004498, NT2RP3004503, NT2RP3004504, NT2RP3004507, NT2RP3004534, NT2RP3004539, NT2RP3004544, NT2RP3004566, NT2RP3004569, NT2RP3004572, NT2RP3004578, NT2RP3004594, NT2RP3004617, NT2RP3004618, NT2RP3004669, NT2RP3004670, NT2RP4000008, NT2RP4000023, NT2RP4000035, NT2RP4000049, NT2RP4000051, NT2RP4000078, NT2RP4000102, NT2RP4000109, NT2RP4000111, NT2RP4000129, NT2RP4000147, NT2RP4000150, NT2RP4000151, NT2RP4000159, NT2RP4000167, NT2RP4000185, NT2RP4000210, NT2RP4000212, NT2RP4000218, NT2RP4000243, NT2RP4000246, NT2RP4000259, NT2RP4000290, NT2RP4000312, NT2RP4000323, NT2RP4000355, NT2RP4000360, NT2RP4000367, NT2RP4000370, NT2RP4000376, NT2RP4000381, NT2RP4000398, NT2RP4000415, NT2RP4000417, NT2RP4000424, NT2RP4000448, NT2RP4000449, NT2RP4000455, NT2RP4000457, NT2RP4000480, NT2RP4000481, NT2RP4000498, NT2RP4000500, NT2RP4000515, NT2RP4000517, NT2RP4000518, NT2RP4000519, NT2RP4000524, NT2RP4000528, NT2RP4000541, NT2RP4000556, NT2RP4000560, NT2RP4000588, NT2RP4000614, NT2RP4000638, NT2RP4000648, NT2RP4000657, NT2RP4000704, NT2RP4000713, NT2RP4000724, NT2RP4000728, NT2RP4000737, NT2RP4000739, NT2RP4000781, NT2RP4000817, NT2RP4000833, NT2RP4000837, NT2RP4000839, NT2RP4000855, NT2RP4000865, NT2RP4000878, NT2RP4000879, NT2RP4000907, NT2RP4000925, NT2RP4000927, NT2RP4000928, NT2RP4000929, NT2RP4000955, NT2RP4000973, NT2RP4000975, NT2RP4000979, NT2RP4000984, NT2RP4000989, NT2RP4000997, NT2RP4001004, NT2RP4001006, NT2RP4001010, NT2RP4001029, NT2RP4001041, NT2RP4001057, NT2RP4001064, NT2RP4001079, NT2RP4001080, NT2RP4001086, NT2RP4001095, NT2RP4001100, NT2RP4001117, NT2RP4001122, NT2RP4001126, NT2RP4001138, NT2RP4001143, NT2RP4001148, NT2RP4001149, NT2RP4001150, NT2RP4001174, NT2RP4001206, NT2RP4001207, NT2RP4001210, NT2RP4001213, NT2RP4001219, NT2RP4001228, NT2RP4001235, NT2RP4001256, NT2RP4001260, NT2RP4001274, NT2RP4001276, NT2RP4001313, NT2RP4001315, NT2RP4001336, NT2RP4001339, NT2RP4001343, NT2RP4001345, NT2RP4001351, NT2RP4001353, NT2RP4001372, NT2RP4001373, NT2RP4001375, NT2RP4001379, NT2RP4001389, NT2RP4001407, NT2RP4001414, NT2RP4001433, NT2RP4001442, NT2RP4001447, NT2RP4001474, NT2RP4001483, NT2RP4001498, NT2RP4001502, NT2RP4001507, NT2RP4001524, NT2RP4001529, NT2RP4001547, NT2RP4001551, NT2RP4001555, NT2RP4001567, NT2RP4001568, NT2RP4001571, NT2RP4001574, NT2RP4001575, NT2RP4001592, NT2RP4001610, NT2RP4001614, NT2RP4001634, NT2RP4001638, NT2RP4001644, NT2RP4001656, NT2RP4001677, NT2RP4001679, NT2RP4001696, NT2RP4001725, NT2RP4001730, NT2RP4001739, NT2RP4001753, NT2RP4001760, NT2RP4001790, NT2RP4001803, NT2RP4001822, NT2RP4001823, NT2RP4001828, NT2RP4001838, NT2RP4001841, NT2RP4001849, NT2RP4001861, NT2RP4001893, NT2RP4001896, NT2RP4001901, NT2RP4001927, NT2RP4001938, NT2RP4001946, NT2RP4001950, NT2RP4001953, NT2RP4001966, NT2RP4001975, NT2RP4002018, NT2RP4002047, NT2RP4002052, NT2RP4002058, NT2RP4002071, NT2RP4002075, NT2RP4002078, NT2RP4002081, NT2RP4002083, NT2RP4002298, NT2RP4002408, NT2RP4002791, NT2RP4002888, NT2RP4002905, NT2RP5003461, NT2RP5003477, NT2RP5003492, NT2RP5003500, NT2RP5003506, NT2RP5003512, NT2RP5003522, NT2RP5003524, NT2RP5003534, OVARC1000001, OVARC1000006, OVARC1000008, OVARC1000013, OVARC1000014, OVARC1000017, OVARC1000035, OVARC1000058, OVARC1000060, OVARC1000068, OVARC1000071, OVARC1000085, OVARC1000087, OVARC1000091, OVARC1000092, OVARC1000106, OVARC1000109, OVARC1000113, OVARC1000114, OVARC1000139, OVARC1000145, OVARC1000148, OVARC1000151, OVARC1000168, OVARC1000198, OVARC1000209, OVARC1000212, OVARC1000240, OVARC1000241, OVARC1000288, OVARC1000302, OVARC1000304, OVARC1000309, OVARC1000321, OVARC1000326, OVARC1000335, OVARC1000347, OVARC1000384, OVARC1000408, OVARC1000411, OVARC1000414, OVARC1000420, OVARC1000437, OVARC1000440, OVARC1000442, OVARC1000443, OVARC1000461, OVARC1000465, OVARC1000466, OVARC1000473, OVARC1000479, OVARC1000496, OVARC1000520, OVARC1000533, OVARC1000556, OVARC1000557, OVARC1000564, OVARC1000576, OVARC1000578, OVARC1000588, OVARC1000605, OVARC1000622, OVARC1000640, OVARC1000649, OVARC1000661, OVARC1000678, OVARC1000679, OVARC1000681, OVARC1000682, OVARC1000689, OVARC1000700, OVARC1000703, OVARC1000722, OVARC1000724, OVARC1000730, OVARC1000746, OVARC1000751, OVARC1000771, OVARC1000781, OVARC1000787, OVARC1000800, OVARC1000802, OVARC1000834, OVARC1000846, OVARC1000850, OVARC1000862, OVARC1000876, OVARC1000883, OVARC1000885, OVARC1000886, OVARC1000890, OVARC1000891, OVARC1000912, OVARC1000915, OVARC1000924, OVARC1000936, OVARC1000937, OVARC1000945, OVARC1000959, OVARC1000960, OVARC1000964, OVARC1000971, OVARC1000984, OVARC1000999, OVARC1001000, OVARC1001004, OVARC1001010, OVARC1001011, OVARC1001029, OVARC1001032, OVARC1001034, OVARC1001038, OVARC1001040, OVARC1001044, OVARC1001051, OVARC1001055, OVARC1001065, OVARC1001068, OVARC1001072, OVARC1001074, OVARC1001092, OVARC1001107, OVARC1001113, OVARC1001117, OVARC1001118, OVARC1001129, OVARC1001154, OVARC1001161, OVARC1001162, OVARC1001167, OVARC1001169, OVARC1001170, OVARC1001171, OVARC1001173, OVARC1001176, OVARC1001180, OVARC1001188, OVARC1001200, OVARC1001232, OVARC1001240, OVARC1001243, OVARC1001244, OVARC1001261, OVARC1001270, OVARC1001271, OVARC1001296, OVARC1001306, OVARC1001329, OVARC1001339, OVARC1001341, OVARC1001342, OVARC1001344, OVARC1001357, OVARC1001360, OVARC1001369, OVARC1001372, OVARC1001376, OVARC1001381, OVARC1001391, OVARC1001399, OVARC1001417, OVARC1001419, OVARC1001425, OVARC1001436, OVARC1001442, OVARC1001453, OVARC1001476, OVARC1001480, OVARC1001489, OVARC1001496, OVARC1001506, OVARC1001525, OVARC1001555, OVARC1001577, OVARC1001600, OVARC1001610, OVARC1001611, OVARC1001702, OVARC1001703, OVARC1001711, OVARC1001713, OVARC1001726, OVARC1001731, OVARC1001745, OVARC1001762, OVARC1001766, OVARC1001767, OVARC1001768, OVARC1001791, OVARC1001795, OVARC1001802, OVARC1001809, OVARC1001813, OVARC1001828, OVARC1001846, OVARC1001861, OVARC1001873, OVARC1001879, OVARC1001880, OVARC1001883, OVARC1001916, OVARC1001928, OVARC1001942, OVARC1001943, OVARC1001950, OVARC1001987, OVARC1002050, OVARC1002082, OVARC1002107, OVARC1002112, OVARC1002127, OVARC1002138, OVARC1002143, OVARC1002156, OVARC1002158, OVARC1002165, OVARC1002182, PLACE1000004, PLACE1000005, PLACE1000007, PLACE1000014, PLACE1000040, PLACE1000048, PLACE1000050, PLACE1000061, PLACE1000066, PLACE1000078, PLACE1000081, PLACE1000094, PLACE1000133, PLACE1000142, PLACE1000184, PLACE1000185, PLACE1000213, PLACE1000214, PLACE1000236, PLACE1000246, PLACE1000292, PLACE1000308, PLACE1000332, PLACE1000347, PLACE1000374, PLACE1000380, PLACE1000383, PLACE1000401, PLACE1000406, PLACE1000420, PLACE1000435, PLACE1000444, PLACE1000453, PLACE1000492, PLACE1000547, PLACE1000562, PLACE1000564, PLACE1000583, PLACE1000588, PLACE1000596, PLACE1000599, PLACE1000610, PLACE1000611, PLACE1000636, PLACE1000653, PLACE1000656, PLACE1000706, PLACE1000712, PLACE1000716, PLACE1000748, PLACE1000749, PLACE1000755, PLACE1000769, PLACE1000785, PLACE1000786, PLACE1000793, PLACE1000798, PLACE1000814, PLACE1000849, PLACE1000856, PLACE1000863, PLACE1000909, PLACE1000931, PLACE1000948, PLACE1000972, PLACE1000977, PLACE1000979, PLACE1000987, PLACE1001000, PLACE1001007, PLACE1001010, PLACE1001015, PLACE1001024, PLACE1001036, PLACE1001054, PLACE1001062, PLACE1001076, PLACE1001088, PLACE1001092, PLACE1001104, PLACE1001118, PLACE1001136, PLACE1001168, PLACE1001171, PLACE1001185, PLACE1001238, PLACE1001241, PLACE1001257, PLACE1001280, PLACE1001294, PLACE1001304, PLACE1001311, PLACE1001323, PLACE1001351, PLACE1001366, PLACE1001377, PLACE1001383, PLACE1001387, PLACE1001395, PLACE1001399, PLACE1001412, PLACE1001414, PLACE1001440, PLACE1001456, PLACE1001484, PLACE1001503, PLACE1001517, PLACE1001545, PLACE1001570, PLACE1001602, PLACE1001608, PLACE1001610, PLACE1001632, PLACE1001634, PLACE1001640, PLACE1001672, PLACE1001692, PLACE1001705, PLACE1001716, PLACE1001720, PLACE1001729, PLACE1001739, PLACE1001740, PLACE1001745, PLACE1001746, PLACE1001748, PLACE1001761, PLACE1001771, PLACE1001781, PLACE1001799, PLACE1001810, PLACE1001817, PLACE1001845, PLACE1001869, PLACE1001897, PLACE1001912, PLACE1001920, PLACE1001928, PLACE1001983, PLACE1001989, PLACE1002004, PLACE1002046, PLACE1002066, PLACE1002072, PLACE1002073, PLACE1002090, PLACE1002115, PLACE1002140, PLACE1002157, PLACE1002163, PLACE1002170, PLACE1002171, PLACE1002213, PLACE1002227, PLACE1002256, PLACE1002259, PLACE1002319, PLACE1002342, PLACE1002395, PLACE1002433, PLACE1002437, PLACE1002438, PLACE1002450, PLACE1002465, PLACE1002474, PLACE1002477, PLACE1002493, PLACE1002499, PLACE1002500, PLACE1002514, PLACE1002529, PLACE1002532, PLACE1002537, PLACE1002571, PLACE1002578, PLACE1002583, PLACE1002591, PLACE1002598, PLACE1002625, PLACE1002655, PLACE1002665, PLACE1002685, PLACE1002714, PLACE1002722, PLACE1002768, PLACE1002772, PLACE1002775, PLACE1002782, PLACE1002794, PLACE1002815, PLACE1002816, PLACE1002834, PLACE1002839, PLACE1002851, PLACE1002853, PLACE1002908, PLACE1002941, PLACE1002962, PLACE1002968, PLACE1002991, PLACE1002993, PLACE1002996, PLACE1003025, PLACE1003027, PLACE1003030, PLACE1003044, PLACE1003045, PLACE1003092, PLACE1003100, PLACE1003108, PLACE1003145, PLACE1003174, PLACE1003176, PLACE1003190, PLACE1003200, PLACE1003205, PLACE1003238, PLACE1003249, PLACE1003256, PLACE1003258, PLACE1003296, PLACE1003302, PLACE1003334, PLACE1003342, PLACE1003343, PLACE1003353, PLACE1003361, PLACE1003366, PLACE1003369, PLACE1003373, PLACE1003375, PLACE1003383, PLACE1003394, PLACE1003420, PLACE1003454, PLACE1003478, PLACE1003493, PLACE1003516, PLACE1003519, PLACE1003521, PLACE1003528, PLACE1003537, PLACE1003553, PLACE1003566, PLACE1003584, PLACE1003592, PLACE1003593, PLACE1003596, PLACE1003602, PLACE1003605, PLACE1003611, PLACE1003618, PLACE1003625, PLACE1003638, PLACE1003669, PLACE1003704, PLACE1003709, PLACE1003711, PLACE1003723, PLACE1003738, PLACE1003760, PLACE1003762, PLACE1003768, PLACE1003771, PLACE1003784, PLACE1003795, PLACE1003864, PLACE1003870, PLACE1003885, PLACE1003886, PLACE1003888, PLACE1003892, PLACE1003900, PLACE1003903, PLACE1003915, PLACE1003923, PLACE1003936, PLACE1003968, PLACE1004104, PLACE1004114, PLACE1004118, PLACE1004128, PLACE1004149, PLACE1004156, PLACE1004161, PLACE1004183, PLACE1004197, PLACE1004203, PLACE1004256, PLACE1004258, PLACE1004270, PLACE1004274, PLACE1004277, PLACE1004284, PLACE1004289, PLACE1004302, PLACE1004316, PLACE1004336, PLACE1004358, PLACE1004376, PLACE1004384, PLACE1004388, PLACE1004405, PLACE1004425, PLACE1004428, PLACE1004437, PLACE1004451, PLACE1004460, PLACE1004471, PLACE1004473, PLACE1004491, PLACE1004506, PLACE1004510, PLACE1004516, PLACE1004518, PLACE1004548, PLACE1004550, PLACE1004564, PLACE1004629, PLACE1004645, PLACE1004646, PLACE1004664, PLACE1004672, PLACE1004674, PLACE1004681, PLACE1004686, PLACE1004691, PLACE1004693, PLACE1004716, PLACE1004722, PLACE1004736, PLACE1004740, PLACE1004743, PLACE1004751, PLACE1004777, PLACE1004793, PLACE1004804, PLACE1004814, PLACE1004815, PLACE1004824, PLACE1004836, PLACE1004838, PLACE1004840, PLACE1004868, PLACE1004885, PLACE1004900, PLACE1004902, PLACE1004913, PLACE1004918, PLACE1004930, PLACE1004934, PLACE1004937, PLACE1004969, PLACE1004979, PLACE1004982, PLACE1004985, PLACE1005026, PLACE1005027, PLACE1005046, PLACE1005052, PLACE1005055, PLACE1005077, PLACE1005085, PLACE1005086, PLACE1005101, PLACE1005102, PLACE1005108, PLACE1005111, PLACE1005128, PLACE1005146, PLACE1005162, PLACE1005176, PLACE1005181, PLACE1005187, PLACE1005206, PLACE1005232, PLACE1005243, PLACE1005261, PLACE1005266, PLACE1005277, PLACE1005287, PLACE1005305, PLACE1005308, PLACE1005313, PLACE1005327, PLACE1005331, PLACE1005335, PLACE1005373, PLACE1005374, PLACE1005409, PLACE1005453, PLACE1005467, PLACE1005477, PLACE1005480, PLACE1005481, PLACE1005494, PLACE1005530, PLACE1005549, PLACE1005550, PLACE1005554, PLACE1005557, PLACE1005574, PLACE1005584, PLACE1005595, PLACE1005603, PLACE1005611, PLACE1005623, PLACE1005639, PLACE1005646, PLACE1005656, PLACE1005727, PLACE1005730, PLACE1005739, PLACE1005755, PLACE1005763, PLACE1005799, PLACE1005802, PLACE1005803, PLACE1005804, PLACE1005813, PLACE1005828, PLACE1005850, PLACE1005851, PLACE1005876, PLACE1005884, PLACE1005890, PLACE1005898, PLACE1005921, PLACE1005923, PLACE1005925, PLACE1005932, PLACE1005934, PLACE1005936, PLACE1005951, PLACE1005953, PLACE1005955, PLACE1005966, PLACE1005968, PLACE1005990, PLACE1006002, PLACE1006003, PLACE1006011, PLACE1006017, PLACE1006037, PLACE1006040, PLACE1006076, PLACE1006119, PLACE1006129, PLACE1006139, PLACE1006143, PLACE1006157, PLACE1006159, PLACE1006167, PLACE1006170, PLACE1006195, PLACE1006196, PLACE1006225, PLACE1006236, PLACE1006239, PLACE1006246, PLACE1006248, PLACE1006288, PLACE1006318, PLACE1006325, PLACE1006335, PLACE1006357, PLACE1006360, PLACE1006368, PLACE1006371, PLACE1006382, PLACE1006385, PLACE1006412, PLACE1006414, PLACE1006438, PLACE1006445, PLACE1006469, PLACE1006470, PLACE1006482, PLACE1006488, PLACE1006492, PLACE1006506, PLACE1006521, PLACE1006531, PLACE1006534, PLACE1006552, PLACE1006598, PLACE1006615, PLACE1006617, PLACE1006626, PLACE1006640, PLACE1006673, PLACE1006678, PLACE1006704, PLACE1006731, PLACE1006754, PLACE1006760, PLACE1006779, PLACE1006782, PLACE1006792, PLACE1006795, PLACE1006805, PLACE1006815, PLACE1006819, PLACE1006829, PLACE1006867, PLACE1006878, PLACE1006883, PLACE1006901, PLACE1006917, PLACE1006932, PLACE1006935, PLACE1006956, PLACE1006958, PLACE1006961, PLACE1006962, PLACE1006966, PLACE1007014, PLACE1007021, PLACE1007045, PLACE1007053, PLACE1007068, PLACE1007097, PLACE1007105, PLACE1007111, PLACE1007112, PLACE1007140, PLACE1007178, PLACE1007218, PLACE1007226, PLACE1007238, PLACE1007239, PLACE1007242, PLACE1007243, PLACE1007257, PLACE1007274, PLACE1007282, PLACE1007301, PLACE1007317, PLACE1007342, PLACE1007346, PLACE1007367, PLACE1007375, PLACE1007386, PLACE1007402, PLACE1007409, PLACE1007416, PLACE1007450, PLACE1007452, PLACE1007454, PLACE1007460, PLACE1007478, PLACE1007484, PLACE1007488, PLACE1007507, PLACE1007511, PLACE1007524, PLACE1007537, PLACE1007544, PLACE1007547, PLACE1007557, PLACE1007583, PLACE1007598, PLACE1007618, PLACE1007621, PLACE1007632, PLACE1007645, PLACE1007649, PLACE1007677, PLACE1007688, PLACE1007690, PLACE1007697, PLACE1007705, PLACE1007706, PLACE1007725, PLACE1007729, PLACE1007730, PLACE1007737, PLACE1007743, PLACE1007746, PLACE1007791, PLACE1007807, PLACE1007810, PLACE1007843, PLACE1007846, PLACE1007852, PLACE1007858, PLACE1007866, PLACE1007877, PLACE1007897, PLACE1007946, PLACE1007954, PLACE1007955, PLACE1007958, PLACE1007969, PLACE1007990, PLACE1008000, PLACE1008002, PLACE1008044, PLACE1008045, PLACE1008080, PLACE1008095, PLACE1008111, PLACE1008122, PLACE1008129, PLACE1008132, PLACE1008177, PLACE1008181, PLACE1008201, PLACE1008209, PLACE1008231, PLACE1008244, PLACE1008273, PLACE1008275, PLACE1008280, PLACE1008309, PLACE1008329, PLACE1008330, PLACE1008331, PLACE1008356, PLACE1008368, PLACE1008369, PLACE1008392, PLACE1008398, PLACE1008401, PLACE1008402, PLACE1008405, PLACE1008424, PLACE1008426, PLACE1008429, PLACE1008455, PLACE1008457, PLACE1008465, PLACE1008488, PLACE1008524, PLACE1008531, PLACE1008532, PLACE1008533, PLACE1008568, PLACE1008584, PLACE1008603, PLACE1008621, PLACE1008625, PLACE1008626, PLACE1008627, PLACE1008629, PLACE1008630, PLACE1008643, PLACE1008650, PLACE1008693, PLACE1008696, PLACE1008715, PLACE1008748, PLACE1008757, PLACE1008790, PLACE1008798, PLACE1008808, PLACE1008813, PLACE1008851, PLACE1008854, PLACE1008867, PLACE1008887, PLACE1008902, PLACE1008925, PLACE1008941, PLACE1008947, PLACE1009020, PLACE1009027, PLACE1009039, PLACE1009045, PLACE1009048, PLACE1009050, PLACE1009060, PLACE1009090, PLACE1009091, PLACE1009094, PLACE1009099, PLACE1009110, PLACE1009111, PLACE1009113, PLACE1009130, PLACE1009150, PLACE1009158, PLACE1009166, PLACE1009174, PLACE1009186, PLACE1009190, PLACE1009200, PLACE1009230, PLACE1009246, PLACE1009298, PLACE1009308, PLACE1009319, PLACE1009328, PLACE1009335, PLACE1009338, PLACE1009368, PLACE1009375, PLACE1009388, PLACE1009398, PLACE1009404, PLACE1009410, PLACE1009434, PLACE1009443, PLACE1009444, PLACE1009459, PLACE1009468, PLACE1009476, PLACE1009477, PLACE1009493, PLACE1009524, PLACE1009539, PLACE1009542, PLACE1009571, PLACE1009581, PLACE1009595, PLACE1009596, PLACE1009607, PLACE1009621, PLACE1009622, PLACE1009637, PLACE1009639, PLACE1009659, PLACE1009665, PLACE1009670, PLACE1009708, PLACE1009721, PLACE1009731, PLACE1009763, PLACE1009794, PLACE1009798, PLACE1009845, PLACE1009861, PLACE1009886, PLACE1009388, PLACE1009908, PLACE1009921, PLACE1009925, PLACE1009935, PLACE1009947, PLACE1009971, PLACE1009992, PLACE1009995, PLACE1009997, PLACE1010023, PLACE1010031, PLACE1010053, PLACE1010069, PLACE1010074, PLACE1010076, PLACE1010089, PLACE1010096, PLACE1010102, PLACE1010105, PLACE1010106, PLACE1010134, PLACE1010148, PLACE1010194, PLACE1010202, PLACE1010231, PLACE1010261, PLACE1010270, PLACE1010274, PLACE1010293, PLACE1010310, PLACE1010321, PLACE1010324, PLACE1010329, PLACE1010362, PLACE1010364, PLACE1010383, PLACE1010401, PLACE1010481, PLACE1010491, PLACE1010492, PLACE1010522, PLACE1010529, PLACE1010547, PLACE1010562, PLACE1010579, PLACE1010599, PLACE1010616, PLACE1010622, PLACE1010624, PLACE1010628, PLACE1010629, PLACE1010630, PLACE1010661, PLACE1010662, PLACE1010702, PLACE1010714, PLACE1010720, PLACE1010743, PLACE1010761, PLACE1010771, PLACE1010786, PLACE1010800, PLACE1010802, PLACE1010811, PLACE1010833, PLACE1010856, PLACE1010857, PLACE1010870, PLACE1010877, PLACE1010896, PLACE1010900, PLACE1010916, PLACE1010917, PLACE1010925, PLACE1010926, PLACE1010942, PLACE1010944, PLACE1010947, PLACE1010954, PLACE1010960, PLACE1010965, PLACE1011026, PLACE1011032, PLACE1011041, PLACE1011046, PLACE1011054, PLACE1011056, PLACE1011057, PLACE1011090, PLACE1011109, PLACE1011114, PLACE1011133, PLACE1011143, PLACE1011160, PLACE1011165, PLACE1011185, PLACE1011214, PLACE1011219, PLACE1011221, PLACE1011229, PLACE1011263, PLACE1011273, PLACE1011291, PLACE1011310, PLACE1011325, PLACE1011332, PLACE1011340, PLACE1011371, PLACE1011399, PLACE1011433, PLACE1011452, PLACE1011465, PLACE1011472, PLACE1011477, PLACE1011492, PLACE1011503, PLACE1011520, PLACE1011563, PLACE1011567, PLACE1011576, PLACE1011586, PLACE1011635, PLACE1011643, PLACE1011646, PLACE1011649, PLACE1011650, PLACE1011664, PLACE1011675, PLACE1011682, PLACE1011719, PLACE1011725, PLACE1011729, PLACE1011749, PLACE1011858, PLACE1011874, PLACE1011875, PLACE1011891, PLACE1011896, PLACE1011922, PLACE1011923, PLACE1011982, PLACE1012031, PLACE2000003, PLACE2000006, PLACE2000007, PLACE2000014, PLACE2000015, PLACE2000017, PLACE2000021, PLACE2000034, PLACE2000039, PLACE2000047, PLACE2000050, PLACE2000061, PLACE2000062, PLACE2000072, PLACE2000097, PLACE2000100, PLACE2000103, PLACE2000111, PLACE2000115, PLACE2000124, PLACE2000132, PLACE2000140, PLACE2000164, PLACE2000170, PLACE2000172, PLACE2000176, PLACE2000187, PLACE2000216, PLACE2000223, PLACE2000235, PLACE2000246, PLACE2000274, PLACE2000302, PLACE2000317, PLACE2000335, PLACE2000341, PLACE2000347, PLACE2000359, PLACE2000366, PLACE2000371, PLACE2000373, PLACE2000379, PLACE2000394, PLACE2000398, PLACE2000399, PLACE2000404, PLACE2000411, PLACE2000425, PLACE2000427, PLACE2000433, PLACE2000438, PLACE2000450, PLACE2000455, PLACE2000458, PLACE2000477, PLACE3000009, PLACE3000020, PLACE3000059, PLACE3000070, PLACE3000103, PLACE3000119, PLACE3000121, PLACE3000124, PLACE3000136, PLACE3000142, PLACE3000145, PLACE3000147, PLACE3000148, PLACE3000155, PLACE3000156, PLACE3000157, PLACE3000158, PLACE3000160, PLACE3000169, PLACE3000194, PLACE3000197, PLACE3000199, PLACE3000207, PLACE3000208, PLACE3000218, PLACE3000221, PLACE3000226, PLACE3000230, PLACE3000242, PLACE3000244, PLACE3000254, PLACE3000271, PLACE3000276, PLACE3000304, PLACE3000310, PLACE3000320, PLACE3000322, PLACE3000331, PLACE3000339, PLACE3000341, PLACE3000350, PLACE3000352, PLACE3000353, PLACE3000362, PLACE3000363, PLACE3000365, PLACE3000373, PLACE3000388, PLACE3000399, PLACE3000401, PLACE3000402, PLACE3000405, PLACE3000406, PLACE3000413, PLACE3000416, PLACE3000425, PLACE3000455, PLACE3000475, PLACE3000477, PLACE4000009, PLACE4000014, PLACE4000052, PLACE4000063, PLACE4000089, PLACE4000093, PLACE4000100, PLACE4000106, PLACE4000128, PLACE4000129, PLACE4000131, PLACE4000147, PLACE4000156, PLACE4000192, PLACE4000211, PLACE4000222, PLACE4000230, PLACE4000247, PLACE4000250, PLACE4000252, PLACE4000259, PLACE4000261, PLACE4000269, PLACE4000270, PLACE4000300, PLACE4000320, PLACE4000323, PLACE4000326, PLACE4000344, PLACE4000367, PLACE4000369, PLACE4000387, PLACE4000392, PLACE4000401, PLACE4000411, PLACE4000431, PLACE4000445, PLACE4000450, PLACE4000465, PLACE4000487, PLACE4000489, PLACE4000494, PLACE4000521, PLACE4000522, PLACE4000548, PLACE4000558, PLACE4000581, PLACE4000590, PLACE4000593, PLACE4000612, PLACE4000638, PLACE4000650, PLACE4000654, PLACE4000670, SKNMC1000011, SKNMC1000013, SKNMC1000046, SKNMC1000050, SKNMC1000091, THYRO1000026, THYRO1000034, THYRO1000070, THYRO1000072, THYRO1000085, THYRO1000092, THYRO1000107, THYRO1000111, THYRO1000121, THYRO1000124, THYRO1000132, THYRO1000156, THYRO1000173, THYRO1000186, THYRO1000187, THYRO1000197, THYRO1000199, THYRO1000206, THYRO1000241, THYRO1000242, THYRO1000253, THYRO1000270, THYRO1000279, THYRO1000288, THYRO1000320, THYRO1000327, THYRO1000343, THYRO1000358, THYRO1000368, THYRO1000381, THYRO1000387, THYRO1000394, THYRO1000395, THYRO1000401, THYRO1000452, THYRO1000471, THYRO1000484, THYRO1000488, THYRO1000501, THYRO1000502, THYRO1000505, THYRO1000558, THYRO1000569, THYRO1000570, THYRO1000585, THYRO1000596, THYRO1000605, THYRO1000625, THYRO1000637, THYRO1000662, THYRO1000666, THYRO1000676, THYRO1000684, THYRO1000712, THYRO1000715, THYRO1000734, THYRO1000748, THYRO1000756, THYRO1000777, THYRO1000783, THYRO1000787, THYRO1000793, THYRO1000796, THYRO1000805, THYRO1000815, THYRO1000843, THYRO1000852, THYRO1000855, THYRO1000865, THYRO1000895, THYRO1000916, THYRO1000926, THYRO1000934, THYRO1000951, THYRO1000952, THYRO1000983, THYRO1000988, THYRO1001003, THYRO1001031, THYRO1001033, THYRO1001062, THYRO1001100, THYRO1001120, THYRO1001133, THYRO1001134, THYRO1001142, THYRO1001173, THYRO1001189, THYRO1001204, THYRO1001213, THYRO1001262, THYRO1001271, THYRO1001287, THYRO1001313, THYRO1001320, THYRO1001321, THYRO1001322, THYRO1001347, THYRO1001363, THYRO1001365, THYRO1001374, THYRO1001401, THYRO1001403, THYRO1001405, THYRO1001406, THYRO1001411, THYRO1001426, THYRO1001434, THYRO1001458, THYRO1001480, THYRO1001487, THYRO1001534, THYRO1001537, THYRO1001541, THYRO1001559, THYRO1001570, THYRO1001584, THYRO1001595, THYRO1001602, THYRO1001605, THYRO1001617, THYRO1001637, THYRO1001656, THYRO1001661, THYRO1001671, THYRO1001673, THYRO1001703, THYRO1001706, THYRO1001721, THYRO1001738, THYRO1001745, THYRO1001746, THYRO1001772, THYRO1001793, THYRO1001809, THYRO1001828, THYRO1001854, THYRO1001895, THYRO1001907, VESEN1000122, Y79AA1000013, Y79AA1000033, Y79AA1000037, Y79AA1000059, Y79AA1000065, Y79AA1000131, Y79AA1000181, Y79AA1000202, Y79AA1000214, Y79AA1000230, Y79AA1000231, Y79AA1000258, Y79AA1000268, Y79AA1000313, Y79AA1000328, Y79AA1000342, Y79AA1000346, Y79AA1000349, Y79AA1000355, Y79AA1000368, Y79AA1000410, Y79AA1000420, Y79AA1000469, Y79AA1000480, Y79AA1000539, Y79AA1000540, Y79AA1000560, Y79AA1000574, Y79AA1000589, Y79AA1000627, Y79AA1000705, Y79AA1000734, Y79AA1000748, Y79AA1000752, Y79AA1000774, Y79AA1000782, Y79AA1000784, Y79AA1000794, Y79AA1000800, Y79AA1000802, Y79AA1000805, Y79AA1000824, Y79AA1000827, Y79AA1000833, Y79AA1000850, Y79AA1000962, Y79AA1000966, Y79AA1000968, Y79AA1000969, Y79AA1000976, Y79AA1000985, Y79AA1001023, Y79AA1001041, Y79AA1001048, Y79AA1001061, Y79AA1001068, Y79AA1001077, Y79AA1001078, Y79AA1001145, Y79AA1001167, Y79AA1001177, Y79AA1001185, Y79AA1001211, Y79AA1001216, Y79AA1001228, Y79AA1001233, Y79AA1001236, Y79AA1001281, Y79AA1001299, Y79AA1001312, Y79AA1001323, Y79AA1001384, Y79AA1001391, Y79AA1001394, Y79AA1001402, Y79AA1001493, Y79AA1001511, Y79AA1001533, Y79AA1001541, Y79AA1001548, Y79AA1001555, Y79AA1001581, Y79AA1001585, Y79AA1001594, Y79AA1001603, Y79AA1001613, Y79AA1001647, Y79AA1001665, Y79AA1001679, Y79AA1001692, Y79AA1001696, Y79AA1001705, Y79AA1001711, Y79AA1001781, Y79AA1001805, Y79AA1001827, Y79AA1001846, Y79AA1001866, Y79AA1001874, Y79AA1001875, Y79AA1001923, Y79AA1001963, Y79AA1002027, Y79AA1002083, Y79AA1002089, Y79AA1002103, Y79AA1002115, Y79AA1002125, Y79AA1002139, Y79AA1002204, Y79AA1002208, Y79AA1002209, Y79AA1002210, Y79AA1002211, Y79AA1002220, Y79AA1002229, Y79AA1002234, Y79AA1002246, Y79AA1002258, Y79AA1002298, Y79AA1002307, Y79AA1002311, Y79AA1002351, Y79AA1002361, Y79AA1002399, Y79AA1002407, Y79AA1002416, Y79AA1002431, Y79AA1002433, Y79AA1002472, Y79AA1002482, Y79AA1002487,

Among the 4997 clones, there are 2189 clones that presumably encode proteins belonging to any of the categories of secretory or membrane proteins, glycoprotein-associated proteins, signal transduction-associated proteins, transcription-associated proteins, disease-associated proteins, enzymes and/or metabolism-associated proteins, ATP- and/or GTP-binding proteins, nuclear proteins, DNA- and/or RNA-binding proteins, RNA synthesis-associated proteins, protein synthesis- and/or protein transport-associated proteins, cytoskeleton-associated proteins, cell division- and/or cell proliferation-associated proteins, embryogenesis- and/or development-associated proteins, or cellular defense-associated proteins.

The clones that presumably encode proteins belonging to the category of secretory or membrane proteins are those which matched the full-length sequences of Swiss-Prot database with the keywords "growth factor", "cytokine", "hormone", "signal", "transmembrane", "membrane", "extracellular matrix", "receptor", "G-protein coupled receptor", "ionic channel", "voltage-gated channel", "calcium channel" , "cell adhesion" , "collagen", or "connective tissue"; those which matched the data, suggesting that the proteins are secretory or membrane proteins; or those which matched the full-length sequences of GenBank or UniGene database with similar description; and, further, those predicted to have an N-terminal signal sequence or a transmembrane region as a result of domain search for the amino acid sequences deduced from the full-length nucleotide sequences.

The clones that presumably encode proteins belonging to the category of glycoprotein-associated proteins are those which matched the full-length sequences of Swiss-Prot database with the keywords "glycoprotein"; those which matched the data, suggesting that the proteins are glycoprotein; or those which matched the full-length sequences of GenBank or UniGene database with similar description.

The clones that presumably encode proteins belonging to the category of signal transduction-associated proteins are those which matched the full-length sequences of Swiss-Prot database with the keywords "serine/threonine-protein kinase", "tyrosine-protein kinase", or "SH3 domain"; those which matched the data, suggesting that the proteins are signal transduction-associated proteins (for example, "ADP-ribosylation factor"); or those which matched the full-length sequences of GenBank or UniGene database with similar description.

The clones that presumably encode proteins belonging to the category of transcription-associated proteins are those which matched the full-length sequences of Swiss-Prot database with the keywords "transcription regulation", "zinc finger", or "homeobox"; those which matched the data, suggesting that the proteins are transcription-associated proteins; or those which matched the full-length sequences of GenBank or UniGene database with similar description.

The clones that presumably encode proteins belonging to the category of disease-associated proteins are those which matched the full-length sequences of Swiss-Prot database with the keywords "disease mutation" or "syndrome"; those which matched the data, suggesting that the proteins are disease-associated proteins; or those which matched the full-length sequences of Swiss-Prot database and GenBank or UniGene database where the matched sequences of genes or proteins which had been registered in the database of Online Mendelian Inheritance in Man (OMIM) (http://www.ncbi.nlm.nih.gov/Omim/), which is a database of human genes and diseases.

The clones that presumably encode proteins belonging to the category of enzymes and/or metabolism-associated proteins are those which showed the terms " metabolism", "oxidoreductase", or "E.C. No. (Enzyme commission number)" in the matching data.

The clones that presumably encode proteins belonging to the category of ATP- and/or GTP-binding proteins are those which matched the data with the terms "ATP-binding" or "GTP-binding".

The clones that presumably encode proteins belonging to the category of nuclear proteins are those which matched the data with the terms "nuclear protein".

The clones that presumably encode proteins belonging to the category of DNA- and/or RNA-binding proteins are those which matched the data with the terms "DNA-binding" or "RNA-binding".

The clones that presumably encode proteins belonging to the category of RNA synthesis-associated proteins are those which matched the data with the terms "RNA splicing", "RNA processing", "RNA helicase", or "polyadenylation".

The clones that presumably encode proteins belonging to the category of protein synthesis- and/or protein transport-associated proteins are those which matched the data with the terms "translation regulation", "protein biosynthesis", "amino-acid biosynthesis", "ribosomal protein", "protein transport", or "signal recognition particle".

The clones that presumably encode proteins belonging to the category of cytoskeleton-associated proteins are those which matched the data with the terms "structural protein", "cytoskeleton", "actin-binding", or "microtubules".

The clones that presumably encode proteins belonging to the category of cell division- and/or cell proliferation-associated proteins are those which matched the data with the terms "cell division", "cell cycle", "mitosis", "chromosomal protein", "cell growth", or "apoptosis" .

The clones that presumably encode proteins belonging to the category of embryogenesis- and/or development-associated proteins are those which matched the data with the terms "developmental protein".

The clones that presumably encode proteins belonging to the category of cellular defense-associated proteins are those which matched the data with the terms "heat shock", "DNA repair", or "DNA damage".

When a clone belonged to the above-mentioned multiple functional categories, the clone was classified into the multiple categories. However, the functions of the protein encoded by the clone are not limited to the functions of the categories into which the clone was classified, and therefore, additional functions can be found for the protein by further analyses.

The following 796 clones are categorized into secretory or membrane proteins.
HEMBA1000356, HEMBA1000518, HEMBA1000531, HEMBA1000637, HEMBA1000719, HEMBA1000817, HEMBA1000822, HEMBA1000852, HEMBA1000870, HEMBA1000991, HEMBA1001052, HEMBA1001071, HEMBA1001085, HEMBA1001286, HEMBA1001351, HEMBA1001407, HEMBA1001446, HEMBA1001515, HEMBA1001557, HEMBA1001569, HEMBA1001661, HEMBA1001734, HEMBA1001746, HEMBA1001866, HEMBA1002125, HEMBA1002150, HEMBA1002166, HEMBA1002417, HEMBA1002462, HEMBA1002475, HEMBA1002477, HEMBA1002486, HEMBA1002609, HEMBA1002659, HEMBA1002661, HEMBA1002780, HEMBA1002818, HEMBA1002876, HEMBA1002921, HEMBA1003071, HEMBA1003077, HEMBA1003079, HEMBA1003086, HEMBA1003096, HEMBA1003281, HEMBA1003286, HEMBA1003538, HEMBA1003711, HEMBA1003742, HEMBA1003803, HEMBA1004055, HEMBA1004143, HEMBA1004146, HEMBA1004207, HEMBA1004341, HEMBA1004461, HEMBA1004577, HEMBA1004637, HEMBA1004752, HEMBA1004756, HEMBA1004850, HEMBA1004889, HEMBA1004923, HEMBA1004930, HEMBA1005029, HEMBA1005035, HEMBA1005050, HEMBA1005552, HEMBA1005576, HEMBA1005581, HEMBA1005588, HEMBA1005616, HEMBA1005699, HEMBA1005991, HEMBA1006036, HEMBA1006038, HEMBA1006067, HEMBA1006173, HEMBA1006198, HEMBA1006293, HEMBA1006310, HEMBA1006492, HEMBA1006502, HEMBA1006583, HEMBA1006659, HEMBA1006758, HEMBA1006789, HEMBA1006921, HEMBA1006926, HEMBA1006976, HEMBA1007203, HEMBA1007301, HEMBB1000037, HEMBB1000050, HEMBB1000054, HEMBB1000175, HEMBB1000317, HEMBB1000556, HEMBB1000593, HEMBB1000631, HEMBB1000763, HEMBB1000827, HEMBB1000915, HEMBB1000975, HEMBB1001112, HEMBB1001151, HEMBB1001177, HEMBB1001302, HEMBB1001348, HEMBB1001564, HEMBB1001630, HEMBB1001871, HEMBB1001872, HEMBB1001925, HEMBB1001962, HEMBB1002042, HEMBB1002044, HEMBB1002142, HEMBB1002190, HEMBB1002193, HEMBB1002247, HEMBB1002383, HEMBB1002387, HEMBB1002550, HEMBB1002600, HEMBB1002692, MAMMA1000045, MAMMA1000129, MAMMA1000133, MAMMA1000277, MAMMA1000278, MAMMA1000410, MAMMA1000416, MAMMA1000472, MAMMA1000672, MAMMA1000684, MAMMA1000714, MAMMA1000734, MAMMA1000778, MAMMA1000798, MAMMA1000842, MAMMA1000859, MAMMA1000897, MAMMA1000956, MAMMA1001008, MAMMA1001030, MAMMA1001041, MAMMA1001073, MAMMA1001080, MAMMA1001139, MAMMA1001154, MAMMA1001322, MAMMA1001388, MAMMA1001411, MAMMA1001487, MAMMA1001751, MAMMA1001754, MAMMA1001771, MAMMA1002009, MAMMA1002427, MAMMA1002428, MAMMA1002461, MAMMA1002524, MAMMA1002573, MAMMA1002598, MAMMA1002655, MAMMA1002684, MAMMA1002769, MAMMA1002844, MAMMA1002881, MAMMA1002890, MAMMA1002938, MAMMA1002947, MAMMA1003035, MAMMA1003089, MAMMA1003146, MAMMA1003150, NT2RM1000035, NT2RM1000037, NT2RM1000062, NT2RM1000080, NT2RM1000092, NT2RM1000131, NT2RM1000199, NT2RM1000257, NT2RM1000260, NT2RM1000355, NT2RM1000430, NT2RM1000563, NT2RM1000648, NT2RM1000742, NT2RM1000770, NT2RM1000800, NT2RM1000811, NT2RM1000833, NT2RM1000857, NT2RM1000867, NT2RM1000882, NT2RM1000905, NT2RM1001008,
NT2RM1001115, NT2RM1001139, NT2RM2000259, NT2RM2000260, NT2RM2000287, NT2RM2000395, NT2RM2000402, NT2RM2000407, NT2RM2000422, NT2RM2000490, NT2RM2000522, NT2RM2000566, NT2RM2000581, NT2RM2000609, NT2RM2000821, NT2RM2001370, NT2RM2001393, NT2RM2001499, NT2RM2001547, NT2RM2001613, NT2RM2001648, NT2RM2001659, NT2RM2001671, NT2RM2001688, NT2RM2001698, NT2RM2001718, NT2RM2001753, NT2RM2001760, NT2RM2001785, NT2RM2001930, NT2RM2001950, NT2RM2001997, NT2RM2001998, NT2RM2002049, NT2RM2002145, NT2RM4000233, NT2RM4000433, NT2RM4000457, NT2RM4000486, NT2RM4000496, NT2RM4000520, NT2RM4000634, NT2RM4000674, NT2RM4000700, NT2RM4000764, NT2RM4000778, NT2RM4000795, NT2RM4000820, NT2RM4000857, NT2RM4001032, NT2RM4001054, NT2RM4001116, NT2RM4001455, NT2RM4001666, NT2RM4001810, NT2RM4001813, NT2RM4001930, NT2RM4001987, NT2RM4002054, NT2RM4002073, NT2RM4002145, NT2RM4002146, NT2RM4002189, NT2RM4002194, NT2RM4002251, NT2RM4002339, NT2RM4002438, NT2RM4002446, NT2RM4002452, NT2RM4002460, NT2RM4002493, NT2RM4002558, NT2RM4002565, NT2RM4002571, NT2RM4002594, NT2RP1000130, NT2RP1000191, NT2RP1000326, NT2RP1000358, NT2RP1000413, NT2RP1000418, NT2RP1000547, NT2RP1000609, NT2RP1000677, NT2RP1000767, NT2RP1000782, NT2RP1000856, NT2RP1001113, NT2RP1001247, NT2RP1001286, NT2RP1001310, NT2RP1001311, NT2RP1001313, NT2RP1001385, NT2RP1001449, NT2RP1001546, NT2RP1001569, NT2RP2000032, NT2RP2000040, NT2RP2000056, NT2RP2000070, NT2RP2000091, NT2RP2000114, NT2RP2000120, NT2RP2000173, NT2RP2000175, NT2RP2000195, NT2RP2000257, NT2RP2000270, NT2RP2000283, NT2RP2000288, NT2RP2000289, NT2RP2000459, NT2RP2000516, NT2RP2000660, NT2RP2000842, NT2RP2000892, NT2RP2001081, NT2RP2001268, NT2RP2001295, NT2RP2001366, NT2RP2001378, NT2RP2001576, NT2RP2001581, NT2RP2001597, NT2RP2001613, NT2RP2001947, NT2RP2001991, NT2RP2002025, NT2RP2002066, NT2RP2002078, NT2RP2002105, NT2RP2002312, NT2RP2002325, NT2RP2002385, NT2RP2002479, NT2RP2002537, NT2RP2002643, NT2RP2002701, NT2RP2002740, NT2RP2002857, NT2RP2003125, NT2RP2003297, NT2RP2003433, NT2RP2003446, NT2RP2003466, NT2RP2003506, NT2RP2003513, NT2RP2003629, NT2RP2003668, NT2RP2003760, NT2RP2003777, NT2RP2003781, NT2RP2004041, NT2RP2004142, NT2RP2004194, NT2RP2004270, NT2RP2004300, NT2RP2004392, NT2RP2004655, NT2RP2004681, NT2RP2004775, NT2RP2004799, NT2RP2004936, NT2RP2004959, NT2RP2005012, NT2RP2005159, NT2RP2005227, NT2RP2005270, NT2RP2005344, NT2RP2005465, NT2RP2005509, NT2RP2005752, NT2RP2005781, NT2RP2005784, NT2RP2005812, NT2RP2006069, NT2RP2006100, NT2RP2006141, NT2RP2006184, NT2RP2006261, NT2RP2006565, NT2RP2006571, NT2RP2006573, NT2RP3000092, NT2RP3000109, NT2RP3000134, NT2RP3000207, NT2RP3000333, NT2RP3000341, NT2RP3000393, NT2RP3000439, NT2RP3000441, NT2RP3000531, NT2RP3000685, NT2RP3000825, NT2RP3000826, NT2RP3000852, NT2RP3000919, NT2RP3001084, NT2RP3001096, NT2RP3001126, NT2RP3001140, NT2RP3001176, NT2RP3001260, NT2RP3001282, NT2RP3001355, NT2RP3001383, NT2RP3001426, NT2RP3001453, NT2RP3001497, NT2RP3001538, NT2RP3001589, NT2RP3001642, NT2RP3001708, NT2RP3001716, NT2RP3001727, NT2RP3001739, NT2RP3001799, NT2RP3001943, NT2RP3001944, NT2RP3002002, NT2RP3002007, NT2RP3002014, NT2RP3002054, NT2RP3002108, NT2RP3002163, NT2RP3002351, NT2RP3002455, NT2RP3002549, NT2RP3002602, NT2RP3002628, NT2RP3002650, NT2RP3002687, NT2RP3002701, NT2RP3002810, NT2RP3002869, NT2RP3002969, NT2RP3002985, NT2RP3003008, NT2RP3003059, NT2RP3003071, NT2RP3003101, NT2RP3003145, NT2RP3003197, NT2RP3003203, NT2RP3003242, NT2RP3003302, NT2RP3003353, NT2RP3003409, NT2RP3003576, NT2RP3003621, NT2RP3003665, NT2RP3003672, NT2RP3003701, NT2RP3003716, NT2RP3003799, NT2RP3003828, NT2RP3003914, NT2RP3003918, NT2RP3003992, NT2RP3004051, NT2RP3004148, NT2RP3004155, NT2RP3004207, NT2RP3004282, NT2RP3004454, NT2RP3004480, NT2RP3004503, NT2RP4000008, NT2RP4000051, NT2RP4000151, NT2RP4000212, NT2RP4000243, NT2RP4000259, NT2RP4000323, NT2RP4000417, NT2RP4000500, NT2RP4000524, NT2RP4000556, NT2RP4000560, NT2RP4000588, NT2RP4000713, NT2RP4000724, NT2RP4000817, NT2RP4000833, NT2RP4000878, NT2RP4000907, NT2RP4000925, NT2RP4000928, NT2RP4000973, NT2RP4000989, NT2RP4001057, NT2RP4001064, NT2RP4001079, NT2RP4001117, NT2RP4001138, NT2RP4001149, NT2RP4001150, NT2RP4001174, NT2RP4001219, NT2RP4001274, NT2RP4001313, NT2RP4001345, NT2RP4001372, NT2RP4001373, NT2RP4001379, NT2RP4001498, NT2RP4001547, NT2RP4001571, NT2RP4001574, NT2RP4001644, NT2RP4001656, NT2RP4001677, NT2RP4001730, NT2RP4001739, NT2RP4001803, NT2RP4001822, NT2RP4001823, NT2RP4001950, NT2RP4001975, NT2RP4002052, NT2RP4002075, NT2RP5003500, NT2RP5003506, NT2RP5003522, NT2RP5003534, OVARC1000060, OVARC1000335, OVARC1000682, OVARC 1000689, OVARC 1000700, OVARC 1000722, OVARC1000751, OVARC 1000850, OVARC1000890, OVARC1000924, OVARC1000936, OVARC1000959, OVARC1000984, OVARC1000999, OVARC1001034, OVARC1001055, OVARC1001117, OVARC1001129, OVARC1001154, OVARC1001329, OVARC1001381, OVARC1001391, OVARC1001453, OVARC1001476, OVARC1001506, OVARC1001610, OVARC1001702, OVARC1001703, OVARC1001713, OVARC1001745, OVARC1001767, OVARC1002127, OVARC1002138, OVARC1002158, OVARC1002165, PLACE1000014, PLACE1000213, PLACE1000401, PLACE1000562, PLACE1000611, PLACE1000656, PLACE1000712, PLACE1 000793, PLACE1000909, PLACE1000948, PLACE1000977, PLACE1001241, PLACE1001257, PLACE1001377, PLACE1001517, PLACE1001610, PLACE1001761, PLACE1001771, PLACE1001817, PLACE1001983, PLACE1002046, PLACE1002140, PLACE1002213, PLACE1002395, PLACE1002437, PLACE1002500, PLACE1002583, PLACE1002714, PLACE1002722, PLACE1002782, PLACE1002794, PLACE1002851, PLACE1002908, PLACE1003030, PLACE1003044, PLACE1003045, PLACE1003238, PLACE1003296, PLACE1003369, PLACE1003420, PLACE1003493, PLACE1003537, PLACE1003553, PLACE1003596, PLACE1003760, PLACE1003768, PLACE1003771, PLACE1003903, PLACE1004149, PLACE1004197, PLACE1004203, PLACE1004258, PLACE1004270, PLACE1004277, PLACE1004289, PLACE1004473, PLACE1004629, PLACE1004646, PLACE1004743, PLACE1004751, PLACE1004793, PLACE1004840, PLACE1004969, PLACE1005086, PLACE1005162, PLACE1005206, PLACE1005313, PLACE1005467, PLACE1005530, PLACE1005595, PLACE1005611, PLACE1005623, PLACE1005763, PLACE1005884, PLACE1005890, PLACE1005898, PLACE1005934, PLACE1005953, PLACE1006157, PLACE1006225, PLACE1006239, PLACE1006288, PLACE1006492, PLACE1006534, PLACE1006678, PLACE1006754, PLACE1006901, PLACE1006935, PLACE1006956, PLACE1007111, PLACE1007243, PLACE1007274, PLACE1007282, PLACE1007317, PLACE1007375, PLACE1007386, PLACE1007409, PLACE1007416, PLACE1007484, PLACE1007583, PLACE1007632, PLACE1007645, PLACE1007649, PLACE1007852, PLACE1007877, PLACE1007954, PLACE1008273, PLACE1008309, PLACE1008331, PLACE1008402, PLACE1008424, PLACE1008429, PLACE1008531, PLACE1008532, PLACE1008533, PLACE1008568, PLACE1008643, PLACE1008693, PLACE1008715, PLACE1009045, PLACE1009094, PLACE1009298, PLACE1009319, PLACE1009338, PLACE1009368, PLACE1009493, PLACE1009639, PLACE1009659, PLACE1009708, PLACE1009731, PLACE1009845, PLACE1009861, PLACE1009935, PLACE1009992, PLACE1010089, PLACE1010231, PLACE1010321, PLACE1010362, PLACE1010599, PLACE1010622, PLACE1010662, PLACE1010811, PLACE1010917, PLACE1010942, PLACE1010954, PLACE1011090, PLACE1011214, PLACE1011221, PLACE1011371, PLACE1011399, PLACE1011492, PLACE1011646, PLACE1011749, PLACE1011896, PLACE2000034, PLACE2000062, PLACE2000111, PLACE2000132, PLACE2000176, PLACE2000187, PLACE2000216, PLACE2000335, PLACE2000341, PLACE2000373, PLACE2000379, PLACE2000398, PLACE2000399, PLACE2000425, PLACE2000438, PLACE2000458, PLACE2000477, PLACE3000020, PLACE3000218, PLACE3000226, PLACE3000242, PLACE3000244, PLACE3000339, PLACE3000373, PLACE3000399, PLACE3000406, PLACE3000413, PLACE3000455, PLACE4000052, PLACE4000063, PLACE4000129, PLACE4000247, PLACE4000250, PLACE4000259, PLACE4000300, PLACE4000387, PLACE4000431, PLACE4000487, PLACE4000494, PLACE4000522, PLACE4000548, PLACE4000581, PLACE4000593, PLACE4000650, THYRO1000156, THYRO1000327, THYRO1000394, THYRO1000395, THYRO1000570, THYRO1000748, THYRO1000756, THYRO1000783, THYRO1001134, THYRO1001271, THYRO1001287, THYRO1001320, THYRO1001401, THYRO1001534, THYRO1001537, THYRO1001541, THYRO1001828, Y79AA1000258, Y79AA1000420, Y79AA1000469, Y79AA1000734, Y79AA1000800, Y79AA1000976, Y79AA1001023, Y79AA1001177, Y79AA1001384, Y79AA1001394, Y79AA1001603, Y79AA1001647, Y79AA1001846, Y79AA1001874, Y79AA1002139, Y79AA1002246, Y79AA1002351, Y79AA1002399, Y79AA1002416,

The following 141 clones are categorized into glycoproteins-associated proteins.
HEMBA1000156, HEMBA1000518, HEMBA1000852, HEMBA1001071, HEMBA1001286, HEMBA1001661, HEMBA1001734, HEMBA1001866, HEMBA1003071, HEMBA1003077, HEMBA1003281, HEMBA1003538, HEMBA1003679, HEMBA1003866, HEMBA1005576, HEMBA1005581, HEMBA1005699, HEMBA1006038, HEMBA1006976, HEMBA1007301, HEMBB1000317, HEMBB1000915, HEMBB1001871, HEMBB1001872, HEMBB1002193, MAMMA1000672, MAMMA1000897, MAMMA1001030, MAMMA1001388, MAMMA1002329, MAMMA1002428, MAMMA1002573, MAMMA1003150, NT2RM1000648, NT2RM1001115, NT2RM2000260, NT2RM2000407, NT2RM2000422, NT2RM2000490, NT2RM2001499, NT2RM2001659, NT2RM2001930, NT2RM4000820, NT2RM4000857, NT2RM4001810, NT2RM4001813, NT2RM4001987, NT2RM4002145, NT2RM4002189, NT2RM4002251, NT2RM4002460, NT2RM4002558, NT2RP1000677, NT2RP1000782, NT2RP1000856, NT2RP1001546, NT2RP2000056, NT2RP2000070, NT2RP2001295, NT2RP2001378, NT2RP2001597, NT2RP2001991, NT2RP2002025, NT2RP2002078, NT2RP2002385, NT2RP2004587, NT2RP2004732, NT2RP2005531, NT2RP3000207, NT2RP3000531, NT2RP3000825, NT2RP3001140, NT2RP3002810, NT2RP3003672, NT2RP3003701, NT2RP3003716, NT2RP3003914, NT2RP3004148, NT2RP4000212, NT2RP4000417, NT2RP4000724, NT2RP4000817, NT2RP4000925, NT2RP4001150, NT2RP4001372, NT2RP4001730, NT2RP4001822, NT2RP4001823, NT2RP5003522, OVARC1000091, OVARC1000288, OVARC1000682, OVARC1001055, OVARC1001506, OVARC1001713, OVARC1002127, PLACE1000213, PLACE1000401, PLACE1002437, PLACE1002583,
PLACE1002722, PLACE1003045, PLACE1003238, PLACE1003258, PLACE1003493, PLACE1004197, PLACE1004793, PLACE1005953, PLACE1005955, PLACE1006157, PLACE1006239, PLACE1006368, PLACE1006534, PLACE1006754, PLACE1006956, PLACE1007416, PLACE1007632, PLACE1007649, PLACE1008643, PLACE1009094, PLACE1009992, PLACE1010231, PLACE1010662, PLACE1011371, PLACE2000034, PLACE2000373, PLACE2000398, PLACE2000399, PLACE2000438, PLACE2000458, PLACE3000339, PLACE4000063, PLACE4000230, PLACE4000522, PLACE4000548, PLACE4000581, THYRO1000327, THYRO1000756, THYRO1001287, Y79AA1001603, Y79AA1001874

The following 129 clones are categorized into signal transduction-associated proteins.
HEMBA1000303, HEMBA1000369, HEMBA1000608, HEMBA1000657, HEMBA1000919, HEMBA1001019, HEMBA1001174, HEMBA1001822, HEMBA1001921, HEMBA1002139, HEMBA1002212, HEMBA1002341, HEMBA1002417, HEMBA1002768, HEMBA1003250, HEMBA1003291, HEMBA1003645, HEMBA1004286, HEMBA1005737, HEMBA1006130, HEMBA1006708, HEMBB1000083, HEMBB1000266, HEMBB1000632, HEMBB1000781, HEMBB1000831, HEMBB1002193, MAMMA1000173, MAMMA1001038, MAMMA1001198, MAMMA1002842, MAMMA1003057, NT2RM1000702, NT2RM1000772, NT2RM1001072, NT2RM2000030, NT2RM2000469, NT2RM2000612, NT2RM2001221, NT2RM2001345, NT2RM2002128, NT2RM4000229, NT2RM4000354, NT2RM4000611, NT2RM4000798, NT2RM4001411, NT2RM4001412, NT2RM4001629, NT2RM4001758, NT2RM4002013, NT2RM4002527, NT2RP1000018, NT2RP1000701, NT2RP1001294, NT2RP1001302, NT2RP2000668, NT2RP2001440, NT2RP2001560, NT2RP2002058, NT2RP2002193, NT2RP2002408, NT2RP2002710, NT2RP2002929, NT2RP2003164, NT2RP2003912, NT2RP2004232, NT2RP2004768, NT2RP2006071, NT2RP2006534, NT2RP3000759, NT2RP3000845, NT2RP3001646, NT2RP3001857, NT2RP3001938, NT2RP3002004, NT2RP3002785, NT2RP3002909, NT2RP3002988, NT2RP3003800, NT2RP3004189, NT2RP3004544, NT2RP4000147, NT2RP4000839, NT2RP4001122, NT2RP4001148, NT2RP4001336, NT2RP4001375, NT2RP4001644, NT2RP4001725, NT2RP4001849, NT2RP4001896, NT2RP4001927, NT2RP4002408, NT2RP5003477, OVARC1000013, OVARC1000437, OVARC1000556, OVARC1000649, OVARC 1000945, OVARC1001200, OVARC1002182, PLACE1000977, PLACE1001387, PLACE1002493, PLACE1002591, PLACE1003190, PLACE1003353, PLACE1004128, PLACE1004302, PLACE1004937, PLACE1005243, PLACE1008000, PLACE1008244, PLACE1008650, PLACE1009468, PLACE1009596, PLACE1009708, PLACE1009845, PLACE1010926, PLACE1011041, PLACE2000164, PLACE2000371, PLACE3000145, PLACE3000350, THYRO1000072, THYRO1000748, THYRO1001120, Y79AA1000328, Y79AA1002431

The following 309 clones are categorized into transcription -associated proteins.
HEMBA1000158, HEMBA1000201, HEMBA1000216, HEMBA1000555, HEMBA1000561, HEMBA1000851, HEMBA1001077, HEMBA1001137, HEMBA1001405, HEMBA1001510, HEMBA1001635, HEMBA1001804, HEMBA1001809, HEMBA1001819, HEMBA1001847, HEMBA1001869, HEMBA1002035, HEMBA1002092, HEMBA1002177, HEMBA1002770, HEMBA1002935, HEMBA1003408, HEMBA1003545, HEMBA1003568, HEMBA1003662, HEMBA1003684, HEMBA1003760, HEMBA1003953, HEMBA1004097, HEMBA1004321, HEMBA1004353, HEMBA1004389, HEMBA1004479, HEMBA1004758, HEMBA1004973, HEMBA1005219, HEMBA1005359, HEMBA1005513, HEMBA1005528, HEMBA1005548, HEMBA1005558, HEMBA1005931, HEMBA1006158, HEMBA1006248, HEMBA1006278, HEMBA1006283, HEMBA1006347, HEMBA1006359, HEMBA1006559, HEMBA1006941, HEMBB1000789, HEMBB1001011, HEMBB1001314, HEMBB1001482, HEMBB1001673, HEMBB1001749, HEMBB1001839, HEMBB1001908, HEMBB1002134, HEMBB1002217, HEMBB1002342, HEMBB1002607, MAMMA1000183, MAMMA1000388, MAMMA1001105, MAMMA1001222, MAMMA1001260, MAMMA1001627, MAMMA1001633, MAMMA1001743, MAMMA1001820, MAMMA1001837, MAMMA1002617, MAMMA1002650, MAMMA1002937, NT2RM1000055, NT2RM1000086, NT2RM1000746, NT2RM1000885, NT2RM1000894, NT2RM1001092, NT2RM2000013, NT2RM2000452, NT2RM2000735, NT2RM2000740, NT2RM2001035, NT2RM2001105, NT2RM2001575, NT2RM2001670, NT2RM2001716, NT2RM2001771, NT2RM2002091, NT2RM4000024, NT2RM4000046, NT2RM4000104, NT2RM4000202, NT2RM4000531, NT2RM4000595, NT2RM4000733, NT2RM4000734,
NT2RM4000741, NT2RM4000751, NT2RM4000996, NT2RM4001092, NT2RM4001140, NT2RM4001200, NT2RM4001483, NT2RM4001592, NT2RM4001783, NT2RM4001823, NT2RM4001828, NT2RM4001858, NT2RM4001979, NT2RM4002066, NT2RP1000086, NT2RP1000111, NT2RP1000574, NT2RP1000902, NT2RP1001013, NT2RP2000008, NT2RP2000126, NT2RP2000297, NT2RP2000420, NT2RP2001174, NT2RP2001233, NT2RP2001756, NT2RP2001869, NT2RP2002046, NT2RP2002252, NT2RP2002270, NT2RP2002464, NT2RP2002503, NT2RP2002520, NT2RP2002591, NT2RP2002880, NT2RP2002939, NT2RP2002993, NT2RP2003243, NT2RP2003329, NT2RP2003347, NT2RP2003480, NT2RP2003522, NT2RP2003564, NT2RP2003714, NT2RP2004013, NT2RP2004066, NT2RP2004187, NT2RP2004920, NT2RP2004961, NT2RP2005003, NT2RP2005139, NT2RP2005325, NT2RP2005496, NT2RP2005701, NT2RP2005722, NT2RP2005776, NT2RP2005942, NT2RP2006238, NT2RP2006436, NT2RP3000050, NT2RP3000320, NT2RP3000512, NT2RP3000527, NT2RP3000590, NT2RP3000603, NT2RP3000605, NT2RP3000632, NT2RP3001057, NT2RP3001107, NT2RP3001111, NT2RP3001120, NT2RP3001150, NT2RP3001268, NT2RP3001338, NT2RP3001398, NT2RP3001527, NT2RP3001688, NT2RP3001855, NT2RP3002165, NT2RP3002399, NT2RP3002876, NT2RP3003133, NT2RP3003193, NT2RP3003251, NT2RP3003313, NT2RP3003327, NT2RP3003555, NT2RP3004016, NT2RP3004125, NT2RP3004242, NT2RP3004428, NT2RP3004498, NT2RP3004566, NT2RP3004617, NT2RP4000210, NT2RP4000398, NT2RP4000455, NT2RP4000648, NT2RP4000837, NT2RP4000865, NT2RP4000997, NT2RP4001029, NT2RP4001080, NT2RP4001213, NT2RP4001433, NT2RP4001529, NT2RP4001551, NT2RP4001568, NT2RP4001638, NT2RP4001753, NT2RP4001760, NT2RP4001790, NT2RP4001838, NT2RP4001938, NT2RP4002078, NT2RP4002081, NT2RP5003461, OVARC1000151, OVARC1000241, OVARC1000479, OVARC1001271, OVARC1001417, OVARC1001436, PLACE1000133, PLACE1000583, PLACE1000706, PLACE1000786, PLACE1000979, PLACE1001118, PLACE1001238, PLACE1001294, PLACE1001304, PLACE1001383, PLACE1001602, PLACE1001632, PLACE1002171, PLACE1002438, PLACE1002450, PLACE1002532, PLACE1002775, PLACE1002834, PLACE1003302, PLACE1003605, PLACE1003738, PLACE1003885, PLACE1004471, PLACE1005584, PLACE1005803, PLACE1005966, PLACE1006167, PLACE1006318, PLACE1006438, PLACE1006482, PLACE1007239, PLACE1007346, PLACE1007488, PLACE1007547, PLACE1007598, PLACE1007955, PLACE1008132, PLACE1008201, PLACE1009099, PLACE1009246, PLACE1009308, PLACE1009398, PLACE1009798, PLACE1010134, PLACE1010702, PLACE1010771, PLACE1010870, PLACE1011160, PLACE1011433, PLACE1011576, PLACE3000009, PLACE3000169, PLACE3000254, PLACE4000128, PLACE4000156, PLACE4000192, PLACE4000211, PLACE4000261, PLACE4000450, PLACE4000489, THYRO1000085, THYRO1000121, THYRO1000242, THYRO1000488, THYRO1000501, THYRO1000569, THYRO 1001100, THYRO1001189, THYRO1001809, Y79AA1000013, Y79AA1000033, Y79AA1000037, Y79AA1000342, Y79AA1000627, Y79AA1000705, Y79AA1001299, Y79AA1001312, Y79AA1001391, Y79AA1001533, Y79AA1001613, Y79AA1001866, Y79AA1002103, Y79AA1002229, Y79AA1002433, Y79AA1002472, Y79AA1002482,

The following 392 clones are categorized into disease-associated proteins.
HEMBA1000020, HEMBA1000216, HEMBA1000304, HEMBA1000561, HEMBA1000569, HEMBA1000910, HEMBA1001043, HEMBA1001059, HEMBA1001071, HEMBA1001088, HEMBA1001569, HEMBA1001661, HEMBA1001672, HEMBA1001819, HEMBA1001921, HEMBA1002267, HEMBA1002419, HEMBA1002469, HEMBA1002547, HEMBA1002555, HEMBA1002810, HEMBA1002939, HEMBA1002997, HEMBA1003148, HEMBA1003369, HEMBA1003417, HEMBA1003418, HEMBA1003433, HEMBA1003538, HEMBA1003555, HEMBA1003568, HEMBA1003569, HEMBA1003581, HEMBA1004168, HEMBA1004202, HEMBA1004248, HEMBA1004275, HEMBA1004321, HEMBA1004353, HEMBA1004356, HEMBA1004479, HEMBA1004509, HEMBA1004669, HEMBA1005009, HEMBA1005338, HEMBA1005367, HEMBA1005423, HEMBA1005528, HEMBA1005581, HEMBA1005621, HEMBA1005699, HEMBA1006507, HEMBA1006650, HEMBA1006652, HEMBA1006737, HEMBA1006807, HEMBA1006877, HEMBA1007121, HEMBA1007243, HEMBB1000119, HEMBB1000693, HEMBB1000927, HEMBB1000985, HEMBB1001068, HEMBB1001282, HEMBB1001339, HEMBB1001482, HEMBB1001564, HEMBB1001802, HEMBB1001905, HEMBB1001908, HEMBB1002217, HEMBB1002477, MAMMA1000388, MAMMA1000731, MAMMA1001305, MAMMA1001633, MAMMA1001868, MAMMA1002170, MAMMA1002198, MAMMA1002268, MAMMA1002485, MAMMA1002530, MAMMA1002858, MAMMA1002869, MAMMA1002881, MAMMA1003047, MAMMA1003146, MAMMA1003166, NT2RM1000001, NT2RM1000153, NT2RM1000252, NT2RM1000555, NT2RM1000770, NT2RM1000826, NT2RM1000850, NT2RM1001003, NT2RM1001092, NT2RM1001102, NT2RM2000191,
NT2RM2000363, NT2RM2000594, NT2RM2000624, NT2RM2000714, NT2RM2000821, NT2RM2001035, NT2RM2001575, NT2RM2001652, NT2RM2001664, NT2RM2001668, NT2RM2001698, NT2RM2001803, NT2RM2001839, NT2RM4000155, NT2RM4000471, NT2RM4000486, NT2RM4000657, NT2RM4000751, NT2RM4000996, NT2RM4001629, NT2RM4001810, NT2RM4001819, NT2RM4001865, NT2RM4001876, NT2RM4001940, NT2RM4002066, NT2RM4002093, NT2RM4002146, NT2RM4002161, NT2RM4002323, NT2RM4002558, NT2RM4002571, NT2RP1000086, NT2RP1000574, NT2RP1000738, NT2RP1000825, NT2RP1000833, NT2RP1000959, NT2RP1000966, NT2RP1001013, NT2RP1001185, NT2RP1001482, NT2RP1001665, NT2RP2000070, NT2RP2000147, NT2RP2000224, NT2RP2000248, NT2RP2000297, NT2RP2000310, NT2RP2000414, NT2RP2000420, NT2RP2000523, NT2RP2000809, NT2RP2000812, NT2RP2001233, NT2RP2001327, NT2RP2001378, NT2RP2001394, NT2RP2001397, NT2RP2001460, NT2RP2001520, NT2RP2001536, NT2RP2001876, NT2RP2001898, NT2RP2002025, NT2RP2002058, NT2RP2002124, NT2RP2002325, NT2RP2002503, NT2RP2002959, NT2RP2003000, NT2RP2003157, NT2RP2003164, NT2RP2003228, NT2RP2003295, NT2RP2003517, NT2RP2003564, NT2RP2003604, NT2RP2003714, NT2RP2003737, NT2RP2003952, NT2RP2004013, NT2RP2004170, NT2RP2004587, NT2RP2004732, NT2RP2004933, NT2RP2005003, NT2RP2005144, NT2RP2005239, NT2RP2005276, NT2RP2005288, NT2RP2005315, NT2RP2005325, NT2RP2005336, NT2RP2005358, NT2RP2005407, NT2RP2005436, NT2RP2005476, NT2RP2005525, NT2RP2005694, NT2RP2005719, NT2RP2006043, NT2RP2006071, NT2RP2006219, NT2RP2006312, NT2RP2006456, NT2RP3000050, NT2RP3000068, NT2RP3000085, NT2RP3000299, NT2RP3000403, NT2RP3000596, NT2RP3000739, NT2RP3000753, NT2RP3000875, NT2RP3001057, NT2RP3001081, NT2RP3001216, NT2RP3001307, NT2RP3001338, NT2RP3001427, NT2RP3001428, NT2RP3001679, NT2RP3001723, NT2RP3001855, NT2RP3001898, NT2RP3001969, NT2RP3002056, NT2RP3002062, NT2RP3002151, NT2RP3002351, NT2RP3002399, NT2RP3002953, NT2RP3002988, NT2RP3003078, NT2RP3003251, NT2RP3003282, NT2RP3003313, NT2RP3003327, NT2RP3003409, NT2RP3003672, NT2RP3003831, NT2RP3004016, NT2RP3004078, NT2RP3004209, NT2RP3004258, NT2RP3004490, NT2RP3004534, NT2RP3004569, NT2RP3004572, NT2RP4000109, NT2RP4000367, NT2RP4000376, NT2RP4000449, NT2RP4000855, NT2RP4000879, NT2RP4000925, NT2RP4001086, NT2RP4001126, NT2RP4001150, NT2RP4001213, NT2RP4001276, NT2RP4001407, NT2RP4001433, NT2RP4001483, NT2RP4001575, NT2RP4001760, NT2RP4001861, NT2RP4002078, NT2RP4002791, OVARC1000014, OVARC1000139, OVARC1000520, OVARC1000722, OVARC1000771, OVARC1000834, OVARC1001051, OVARC1001113, OVARC1001244, OVARC1001372, OVARC1001417, OVARC1001496, OVARC1001506, OVARC1001577, OVARC1001726, OVARC1001766, OVARC1001809, OVARC1002165, PLACE1000133, PLACE1000383, PLACE1000420, PLACE1000583, PLACE1000588, PLACE1001171, PLACE1001387, PLACE1001602, PLACE1002046, PLACE1002140, PLACE1002437, PLACE1002474, PLACE1002685, PLACE1002782, PLACE1002834, PLACE1002908, PLACE1003045, PLACE1003302, PLACE1003353, PLACE1003366, PLACE1003493, PLACE1003669, PLACE1003704, PLACE1003903, PLACE1003968, PLACE1004183, PLACE1004197, PLACE1004277, PLACE1004316, PLACE1004358, PLACE1004471, PLACE1004506, PLACE1004510, PLACE1004674, PLACE1004777, PLACE1004814, PLACE1005494, PLACE1006040, PLACE1006170, PLACE1006438, PLACE1006615, PLACE1007140, PLACE1007239, PLACE1007257, PLACE1007511, PLACE1007598, PLACE1008177, PLACE1008356, PLACE1008402, PLACE1008696, PLACE1009027, PLACE1009113, PLACE1009158, PLACE1009444, PLACE1009524, PLACE1010529, PLACE1010870, PLACE1010896, PLACE1011635, PLACE1011858, PLACE1011922, PLACE2000015, PLACE2000072, PLACE2000216, PLACE2000399, PLACE2000438, PLACE2000458, PLACE3000242, PLACE4000009, PLACE4000014, PLACE4000156, PLACE4000369, SKNMC1000046, SKNMC1000050, THYRO1000034, THYRO1000327, THYRO1000343, THYRO1000358, THYRO1000501, THYRO1000662, THYRO1000684, THYRO1000748, THYRO1000934, THYRO1001120, THYRO1001189, THYRO1001204, THYRO1001458, THYRO1001617, THYRO1001671, Y79AA1000346, Y79AA1000469, Y79AA1000560, Y79AA1000734, Y79AA1000782, Y79AA1001391, Y79AA1001548, Y79AA1001594, Y79AA1001711, Y79AA1001874, Y79AA1002204, Y79AA1002210, Y79AA1002258, Y79AA1002472, Y79AA1002482,

Among them, Swiss-Prot database search and GenBank or UniGene database search revealed that the following 380 clones matched the data of genes or proteins which had been registered in the database of Online Mendelian Inheritance in Man (OMIM) (http://www.ncbi.nlm.nih.gov/Omim/), which is a database of human genes and diseases. (The corresponding OMIM numbers are parenthetically indicated following the clone names.)
HEMBB1000985(147485), HEMBB1001068(603142), HEMBB1001282(182900), HEMBB1001339(300080), HEMBB1001482(603971), HEMBB1001564(603931), HEMBB1001802(125660), HEMBB1001905(190370), HEMBB1001908(601408), HEMBB1002217(603971), HEMBB1002477(604439), MAMMA1000388(604865), MAMMA1000731(602118), MAMMA1001305(602732), MAMMA1001633(600834), MAMMA1001868(190370), MAMMA1002170(603624), MAMMA1002198(600538), MAMMA1002268(603730), MAMMA1002485(603665), MAMMA1002530(603602), MAMMA1002858(601064), MAMMA1002869(602567), MAMMA1002881(602692), MAMMA1003047(603566), MAMMA1003146(603094), MAMMA1003166(604061), NT2RM1000001(601169), NT2RM1000153(600417), NT2RM1000252(604108), NT2RM1000555(191510), NT2RM1000770(300061), NT2RM1000826(191510), NT2RM1000850(182900), NT2RM1001003(604785), NT2RM1001092(603971), NT2RM1001102(604533), NT2RM2000191(602973), NT2RM2000363(151410), NT2RM2000594(602900), NT2RM2000624(601940), NT2RM2000714(179555), NT2RM2000821(600959), NT2RM2001035(604913), NT2RM2001575(109092), NT2RM2001652(604141), NT2RM2001664(603722), NT2RM2001668(602952), NT2RM2001698(604327), NT2RM2001803(603722), NT2RM2001839(603420), NT2RM4000155(187790), NT2RM4000471(603485), NT2RM4000486(168730), NT2RM4000657(602142), NT2RM4000751(602277), NT2RM4000996(603971), NT2RM4001629(601114), NT2RM4001810(155760), NT2RM4001819(176873), NT2RM4001876(179555), NT2RM4001940(603887), NT2RM4002066(300188), NT2RM4002093(600693), NT2RM4002146(602603), NT2RM4002161(254780), NT2RM4002558(604194), NT2RM4002571(602274), NT2RP1000086(602219), NT2RP1000574(601740), NT2RP1000825(602732), NT2RP1000833(602973), NT2RP1000959(180510), NT2RP1000966(164035), NT2RP1001013(194558), NT2RP1001185(243500), NT2RP1001482(600586), NT2RP1001665(114180), NT2RP2000070(600976), NT2RP2000147(603535), NT2RP2000248(603367), NT2RP2000297(602277), NT2RP2000310(239500), NT2RP2000414(601037), NT2RP2000420(600834), NT2RP2000523(600130), NT2RP2000809(603885), NT2RP2000812(160777), NT2RP2001233(603971), NT2RP2001327(191161), NT2RP2001378(158370), NT2RP2001394(300208), NT2RP2001397(602755), NT2RP2001460(190370), NT2RP2001520(603667), NT2RP2001536(600675), NT2RP2001876(601833), NT2RP2001898(147264), NT2RP2002025(601581), NT2RP2002058(604737),
NT2RP2002124(603486), NT2RP2002325(603866), NT2RP2002503(601781), NT2RP2002959(602962), NT2RP2003000(191161), NT2RP2003157(601940), NT2RP2003164(604746), NT2RP2003228(602638), NT2RP2003295(603494), NT2RP2003517(190040), NT2RP2003564(109092), NT2RP2003604(604785), NT2RP2003714(603971), NT2RP2003737(602962), NT2RP2003952(602675), NT2RP2004013(602542), NT2RP2004170(300196), NT2RP2004587(162250), NT2RP2004732(162250), NT2RP2004933(603289), NT2RP2005003(109092), NT2RP2005144(604730), NT2RP2005239(603485), NT2RP2005276(602371), NT2RP2005288(603524), NT2RP2005315(604039), NT2RP2005325(603759), NT2RP2005336(190370), NT2RP2005358(603573), NT2RP2005407(167040), NT2RP2005436(601940), NT2RP2005476(602680), NT2RP2005525(602655), NT2RP2005719(601178), NT2RP2006043(601940), NT2RP2006071(604299), NT2RP2006219(601279), NT2RP2006312(603111), NT2RP2006456(604619), NT2RP3000050(603971), NT2RP3000068(182530), NT2RP3000085(300032), NT2RP3000299(602941), NT2RP3000403(604981), NT2RP3000596(190370), NT2RP3000739(125370), NT2RP3000753(162230), NT2RP3001057(603971), NT2RP3001081(603524), NT2RP3001216(603121), NT2RP3001307(180069), NT2RP3001338(314998), NT2RP3001428(189940), NT2RP3001723(604569), NT2RP3001855(602100), NT2RP3001898(604561), NT2RP3001969(190370), NT2RP3002056(180201), NT2RP3002062(603885), NT2RP3002151(139259), NT2RP3002351(604887), NT2RP3002399(602638), NT2RP3002953(604967), NT2RP3002988(603258), NT2RP3003251(109092), NT2RP3003282(602378), NT2RP3003313(603810), NT2RP3003409(604533), NT2RP3003672(313470), NT2RP3003831(604051), NT2RP3004016(601742), NT2RP3004078(142765), NT2RP3004209(125255), NT2RP3004258(604347), NT2RP3004490(603328), NT2RP3004534(600586), NT2RP3004569(106410), NT2RP3004572(604912), NT2RP4000109(603745), NT2RP4000367(603722), NT2RP4000376(603873), NT2RP4000449(604479), NT2RP4000855(602675), NT2RP4000879(314370), NT2RP4000925(600245), NT2RP4001086(162230), NT2RP4001126(190370), NT2RP4001150(601581), NT2RP4001213(602277), NT2RP4001276(190370), NT2RP4001407(190370), NT2RP4001433(602277), NT2RP4001483(203740), NT2RP4001575(603443), NT2RP4001760(305400), NT2RP4001861(190370), NT2RP4002078(603971), NT2RP4002791(189940), OVARC1000014(603371), OVARC1000139(603486),
OVARC1000520(604126), OVARC1000722(604014), OVARC1000771(179509), OVARC1001051(600051), OVARC1001113(602121), OVARC1001244(601540), OVARC1001372(603145), OVARC1001417(300182), OVARC1001496(602619), OVARC1001506(601313), OVARC1001577(603269), OVARC1001726(300103), OVARC1001766(603910), OVARC1001809(603730), PLACE1000133(602542), PLACE1000383(300171), PLACE1000420(600312), PLACE1000583(194558), PLACE1000588(600411), PLACE1001171(310400), PLACE1001387(600206), PLACE1001602(604913), PLACE1002046(151625), PLACE1002140(603748), PLACE1002437(600046), PLACE1002474(602108), PLACE1002685(604515), PLACE1002782(602095), PLACE1002834(194558), PLACE1002908(604327), PLACE1003045(173910), PLACE1003302(194558), PLACE1003353(604704), PLACE1003366(603681), PLACE1003493(601456), PLACE1003669(190370), PLACE1003704(601940), PLACE1003903(123860), PLACE1003968(602742), PLACE1004183(604701), PLACE1004197(601610), PLACE1004277(603493), PLACE1004316(604261), PLACE1004358(603272), PLACE1004471(194558), PLACE1004506(603450), PLACE1004510(604912), PLACE1004674(601057), PLACE1004777(118423), PLACE1004814(601940), PLACE1005494(603652), PLACE1006040(603061), PLACE1006170(601026), PLACE1006438(600834), PLACE1006615(603910), PLACE1007140(190370), PLACE1007239(604784), PLACE1007257(300108), PLACE1007511(148020), PLACE1007598(602277), PLACE1008177(190370), PLACE1008356(604039), PLACE1008402(603344), PLACE1008696(602141), PLACE1009027(300121), PLACE1009113(600675), PLACE1009158(604140), PLACE1009444(600286), PLACE1009524(602488), PLACE1010529(604834), PLACE1010870(603971), PLACE1010896(160776), PLACE1011635(604058), PLACE1011858(603882), PLACE1011922(160776), PLACE2000015(600051), PLACE2000072(603430), PLACE2000216(182790), PLACE2000399(313470), PLACE2000438(602273), PLACE2000458(600976), PLACE3000242(300132), PLACE4000009(160776), PLACE4000014(300032), PLACE4000156(603971), PLACE4000369(603808), SKNMC1000046(603144), SKNMC1000050(114230), THYRO1000034(190370), THYRO1000327(603243), THYRO1000343(125370), THYRO1000358(604188), THYRO1000501(109092), THYRO1000662(278750), THYRO1000684(603885), THYRO1000748(300023), THYRO1000934(179035), THYRO1001120(602582), THYRO1001189(603971), THYRO 1001204(603169),
THYRO1001458(160776), THYRO1001617(602744), THYRO1001671(603281), Y79AA1000346(604355), Y79AA1000469(602434), Y79AA1000560(601026), Y79AA1000734(603867), Y79AA1000782(600417), Y79AA1001391(142959), Y79AA1001548(600286), Y79AA1001594(600936), Y79AA1001711(600063), Y79AA1001874(600315), Y79AA1002204(605033), Y79AA1002210(191161), Y79AA1002472(603971), Y79AA1002482(603971),

The following 425 clones presumably belong to enzymes and/or metabolism-associated proteins.
HEMBA1000012, HEMBA1000129, HEMBA1000141, HEMBA1000150, HEMBA1000542, HEMBA1000852, HEMBA1001019, HEMBA1001257, HEMBA1001526, HEMBA1001620, HEMBA1001866, HEMBA1001896, HEMBA1002212, HEMBA1002513, HEMBA1002746, HEMBA1002973, HEMBA1003046, HEMBA1003136, HEMBA1003179, HEMBA1003250, HEMBA1003291, HEMBA1003408, HEMBA1003538, HEMBA1003679, HEMBA1003680, HEMBA1004199, HEMBA1004227, HEMBA1004408, HEMBA1004509, HEMBA1004734, HEMBA1004768, HEMBA1005394, HEMBA1005513, HEMBA1005737, HEMBA1005815, HEMBA1006031, HEMBA1006272, HEMBA1006278, HEMBA1006291, HEMBA1006309, HEMBA1006347, HEMBA1006485, HEMBA1006521, HEMBA1006624, HEMBA1006885, HEMBA1006976, HEMBA1007121, HEMBA1007224, HEMBA1007243, HEMBA1007300, HEMBB1000083, HEMBB1000217, HEMBB1000915, HEMBB1000947, HEMBB1001137, HEMBB1001346, HEMBB1001429, HEMBB1001443, HEMBB1001915, HEMBB1001950, HEMBB1002042, MAMMA1000020, MAMMA1000085, MAMMA1000672, MAMMA1000713, MAMMA1000841, MAMMA1000897, MAMMA1001008, MAMMA1001038, MAMMA1001059, MAMMA1001476, MAMMA1001501, MAMMA1002268, MAMMA1002470, MAMMA1002530, MAMMA1002573, MAMMA1002619, MAMMA1002655, MAMMA1002671, MAMMA1003013, MAMMA1003035, NT2RM1000039, NT2RM1000132, NT2RM1000153, NT2RM1000256, NT2RM1000280, NT2RM1000377, NT2RM1000553, NT2RM1000648, NT2RM1000702, NT2RM1000894, NT2RM1001072, NT2RM1001115, NT2RM2000013, NT2RM2000092, NT2RM2000322, NT2RM2000368, NT2RM2000371, NT2RM2000469, NT2RM2000504, NT2RM2000577, NT2RM2000594, NT2RM2000951, NT2RM2001238, NT2RM2001547, NT2RM2001632, NT2RM2001664, NT2RM2001698, NT2RM2001700, NT2RM2001730, NT2RM2001782, NT2RM2001803, NT2RM2001886, NT2RM2001935, NT2RM2001997, NT2RM2002030, NT2RM2002128, NT2RM4000024, NT2RM4000155, NT2RM4000344, NT2RM4000471, NT2RM4000616, NT2RM4000657, NT2RM4000712, NT2RM4000820, NT2RM4001313, NT2RM4001316, NT2RM4001444, NT2RM4001592, NT2RM4001758, NT2RM4001819, NT2RM4001880, NT2RM4002062, NT2RM4002063, NT2RM4002189, NT2RM4002213, NT2RM4002251, NT2RM4002409, NT2RM4002532, NT2RM4002623, NT2RP1000376, NT2RP1000443, NT2RP1000522, NT2RP1000834, NT2RP1000947, NT2RP1001079, NT2RP1001185, NT2RP1001253, NT2RP1001361, NT2RP1001543, NT2RP2000056, NT2RP2000114, NT2RP2000183, NT2RP2000248, NT2RP2000329, NT2RP2000422, NT2RP2000448, NT2RP2000668, NT2RP2000710, NT2RP2000816, NT2RP2001070, NT2RP2001392, NT2RP2001601, NT2RP2001663, NT2RP2001740, NT2RP2001748, NT2RP2001898, NT2RP2002124, NT2RP2002256, NT2RP2002609, NT2RP2002618, NT2RP2002959, NT2RP2002993, NT2RP2003230, NT2RP2003286, NT2RP2003401, NT2RP2003506, NT2RP2003543, NT2RP2003643, NT2RP2003702, NT2RP2003704, NT2RP2003713, NT2RP2003737, NT2RP2003840, NT2RP2003912, NT2RP2003952, NT2RP2004098, NT2RP2004239, NT2RP2004245, NT2RP2004768, NT2RP2004791, NT2RP2004799, NT2RP2004933, NT2RP2005038, NT2RP2005139, NT2RP2005162, NT2RP2005204, NT2RP2005239, NT2RP2005276, NT2RP2005344, NT2RP2005360, NT2RP2005457, NT2RP2005498, NT2RP2005549, NT2RP2005557, NT2RP2005605, NT2RP2005635, NT2RP2005723, NT2RP2005773, NT2RP2005775, NT2RP2005776, NT2RP2005784, NT2RP2005835, NT2RP2005942, NT2RP2006534, NT2RP2006571, NT2RP2006573, NT2RP3000031, NT2RP3000085, NT2RP3000207, NT2RP3000359, NT2RP3000578, NT2RP3000742, NT2RP3000845, NT2RP3000875, NT2RP3000917, NT2RP3001055, NT2RP3001221, NT2RP3001495, NT2RP3001898, NT2RP3001938, NT2RP3002303, NT2RP3002351, NT2RP3002501, NT2RP3002602, NT2RP3002628, NT2RP3002663, NT2RP3003301, NT2RP3003385, NT2RP3003490, NT2RP3003659, NT2RP3003825, NT2RP3003831, NT2RP3003846, NT2RP3003914, NT2RP3004148, NT2RP3004209, NT2RP3004378, NT2RP3004669, NT2RP3004670, NT2RP4000259, NT2RP4000312, NT2RP4000367, NT2RP4000417, NT2RP4000457, NT2RP4000657, NT2RP4000817, NT2RP4000855, NT2RP4000879, NT2RP4000927, NT2RP4000973, NT2RP4000997, NT2RP4001041, NT2RP4001079, NT2RP4001095, NT2RP4001143, NT2RP4001219, NT2RP4001375, NT2RP4001389, NT2RP4001483, NT2RP4001555, NT2RP4001592, NT2RP4001644, NT2RP4001730, NT2RP4001946, NT2RP4002408, NT2RP5003500, NT2RP5003522, OVARC1000013, OVARC1000060, OVARC1000139, OVARC1000288, OVARC1000309, OVARC1000473, OVARC1000556, OVARC1000682, OVARC1000722, OVARC1000751, OVARC1000885, OVARC1000915, OVARC1001107, OVARC1001713, OVARC1001762, OVARC1001809, OVARC1001942, OVARC1002156, OVARC1002165, PLACE1000007, PLACE1000142, PLACE1000185, PLACE1000213, PLACE1000383, PLACE1000420, PLACE1000547, PLACE1000653, PLACE1000755, PLACE1001054, PLACE1001062, PLACE1001672, PLACE1001692, PLACE1001748, PLACE1001781, PLACE1001817, PLACE1001869, PLACE1001989, PLACE1002073, PLACE1002598, PLACE1002908, PLACE1002991, PLACE1003174, PLACE1003176, PLACE1003709, PLACE1003885, PLACE1003888, PLACE1003903, PLACE1003915, PLACE1004270, PLACE1004428, PLACE1004437, PLACE1004751, PLACE1004804, PLACE1004918, PLACE1005243, PLACE1005305, PLACE1005373, PLACE1005656, PLACE1005763, PLACE1005804, PLACE1005953, PLACE1005955, PLACE1006011, PLACE1006469, PLACE1006534, PLACE1006626, PLACE1006731, PLACE1006819, PLACE1006829, PLACE1006878, PLACE1007226, PLACE1007416, PLACE1007649, PLACE1007706, PLACE1007729, PLACE1007954, PLACE1007958, PLACE1008111, PLACE1008275, PLACE1008330, PLACE1008643, PLACE1009094, PLACE1009130, PLACE1009444, PLACE1009763, PLACE1009861, PLACE1009992, PLACE1009997, PLACE1010096, PLACE1010362, PLACE1010481, PLACE1010662, PLACE1011046, PLACE1011219, PLACE1011229, PLACE1011332, PLACE1011635, PLACE1011923, PLACE2000021, PLACE2000034, PLACE2000398, PLACE2000404, PLACE2000438, PLACE3000009, PLACE3000020, PLACE3000059, PLACE3000147, PLACE3000339, PLACE3000350, PLACE4000063, PLACE4000100, PLACE4000401, PLACE4000548, PLACE4000654, SKNMC1000050, THYRO1000072, THYRO1000197, THYRO1000288, THYRO1000605, THYRO1000662, THYRO1000756, THYRO1000852, THYRO1000926, THYRO 1000934, THYRO1000951, THYRO 1000983, THYRO1001003, THYRO1001287, THYRO1001374, THYRO1001406, THYRO1001617, THYRO1001671, THYRO1001738, Y79AA1000782, Y79AA1001048, Y79AA1001233, Y79AA1001394, Y79AA1001493, Y79AA1001548, Y79AA1001581, Y79AA1001603, Y79AA1001827, Y79AA1002027, Y79AA1002209, Y79AA1002211, Y79AA1002361, Y79AA1002416,

The following 217 clones presumably belong to a group of cDNAs encoding ATP- and/or GTP-binding proteins.
HEMBA1000012, HEMBA1000129, HEMBA1000185, HEMBA1000491, HEMBA1000531, HEMBA1001019, HEMBA1001174, HEMBA1001387, HEMBA1001595, HEMBA1001723, HEMBA1001913, HEMBA1002161, HEMBA1002212, HEMBA1002876, HEMBA1002997, HEMBA1003250, HEMBA1003291, HEMBA1003369, HEMBA1003555, HEMBA1003560, HEMBA1004131, HEMBA1004199, HEMBA1004202, HEMBA1004354, HEMBA1004697, HEMBA1005047, HEMBA1005595, HEMBA1007018, HEMBA1007151, HEMBB1000083, HEMBB1000226, HEMBB1000264, HEMBB1000632, HEMBB1000725, HEMBB1001294, HEMBB1002193, MAMMA1000085, MAMMA1000612, MAMMA1000731, MAMMA1000738, MAMMA1001038, MAMMA1001735, MAMMA1001768, MAMMA1003127, NT2RM1000187, NT2RM1000388, NT2RM1000702, NT2RM1000772, NT2RM1000924, NT2RM2000469, NT2RM2000577, NT2RM2000740, NT2RM2001100, NT2RM2001201, NT2RM2001345, NT2RM2001823, NT2RM2002128, NT2RM4000155, NT2RM4000191, NT2RM4000356, NT2RM4000496, NT2RM4000611, NT2RM4000733, NT2RM4000820, NT2RM4001084, NT2RM4001178, NT2RM4001344, NT2RM4001444, NT2RM4001592, NT2RM4001714, NT2RM4001758, NT2RM4001880, NT2RM4002062, NT2RM4002174, NT2RM4002205, NT2RM4002527, NT2RM4002594, NT2RM4002623, NT2RP1000470, NT2RP1000478, NT2RP1000915, NT2RP1000958, NT2RP1001080, NT2RP1001410, NT2RP1001569, NT2RP2000126, NT2RP2000258, NT2RP2000329, NT2RP2000660, NT2RP2000668, NT2RP2000710, NT2RP2000812, NT2RP2000880, NT2RP2001245, NT2RP2001392, NT2RP2002606, NT2RP2003277, NT2RP2003912, NT2RP2004538, NT2RP2004568,
NT2RP2004689, NT2RP2004768, NT2RP2004791, NT2RP2004920, NT2RP2005344, NT2RP2005393, NT2RP2005763, NT2RP2006534, NT2RP3000046, NT2RP3000252, NT2RP3000350, NT2RP3000359, NT2RP3000366, NT2RP3000397, NT2RP3000759, NT2RP3000845, NT2RP3000875, NT2RP3001150, NT2RP3001427, NT2RP3001453, NT2RP3001529, NT2RP3001730, NT2RP3001799, NT2RP3001857, NT2RP3001938, NT2RP3002007, NT2RP3002151, NT2RP3002330, NT2RP3002399, NT2RP3002671, NT2RP3003301, NT2RP3003353, NT2RP3003589, NT2RP3003809, NT2RP3003876, NT2RP3004189, NT2RP3004428, NT2RP3004578, NT2RP4000290, NT2RP4000481, NT2RP4000518, NT2RP4000781, NT2RP4000839, NT2RP4000929, NT2RP4001041, NT2RP4001079, NT2RP4001375, NT2RP4001414, NT2RP4001592, NT2RP4001634, NT2RP4001644, NT2RP4001656, NT2RP4001896, NT2RP4002047, NT2RP4002058, NT2RP4002408, NT2RP5003477, OVARC1000013, OVARC1000304, OVARC1000556, OVARC1000771, OVARC1000800, OVARC1001068, OVARC1002138, PLACE1000040, PLACE1000588, PLACE1001104, PLACE1001739, PLACE1002433, PLACE1002437, PLACE1002714, PLACE1003394, PLACE1003521, PLACE1003915, PLACE1004902, PLACE1005243, PLACE1005305; PLACE1005549, PLACE1005739, PLACE1005921, PLACE1006119, PLACE1006196, PLACE1006552, PLACE1006956, PLACE1007409, PLACE1007697, PLACE1007946, PLACE1008244, PLACE1009404, PLACE1009476, PLACE1009596, PLACE1009908, PLACE1010134, PLACE1010720, PLACE1010896, PLACE1011109, PLACE1011114, PLACE1011310, PLACE1011922, PLACE2000014, PLACE2000039, PLACE2000274, PLACE2000404, PLACE2000427, PLACE3000350, PLACE4000009, PLACE4000014, PLACE4000326, SKNMC1000013, THYRO1000072, THYRO1001458, Y79AA1000833, Y79AA1000962, Y79AA1001394, Y79AA1001875, Y79AA1001963, Y79AA1002209,

The following 320 clones presumably belong to nuclear proteins.
HEMBA1000005, HEMBA1000158, HEMBA1000216, HEMBA1000561, HEMBA1000591, HEMBA1001088, HEMBA1001137, HEMBA1001405, HEMBA1001510, HEMBA1001579, HEMBA1001809, HEMBA1001819, HEMBA1001824, HEMBA1001847, HEMBA1001869, HEMBA1002177, HEMBA1002241, HEMBA1002495, HEMBA1002569, HEMBA1002935, HEMBA1002951, HEMBA1002999, HEMBA1003408, HEMBA1003545, HEMBA1003662, HEMBA1003684, HEMBA1003690, HEMBA1003760, HEMBA1004203, HEMBA1004321, HEMBA1004353, HEMBA1004479, HEMBA1004973, HEMBA1005219, HEMBA1005359, HEMBA1005558, HEMBA1005931, HEMBA1006278, HEMBA1006283, HEMBA1006359, HEMBA1006485, HEMBA1007087, HEMBB1000226, HEMBB1000789, HEMBB1001011, HEMBB1001056, HEMBB1001242, HEMBB1001482, HEMBB1001915, HEMBB1002134, HEMBB1002217, MAMMA1000183, MAMMA1000731, MAMMA1001105, MAMMA1001222, MAMMA1001260, MAMMA1001633, MAMMA1001743, MAMMA1001837, MAMMA1002617, MAMMA1002869, MAMMA1002937, MAMMA1003011, NT2RM1000086, NT2RM1000187, NT2RM1000666, NT2RM1000885, NT2RM1000894, NT2RM1001059, NT2RM1001092, NT2RM2000013, NT2RM2000588, NT2RM2000624, NT2RM2000735, NT2RM2000740, NT2RM2001105, NT2RM2001635, NT2RM2001670, NT2RM2001771, NT2RM2001823, NT2RM2001936, NT2RM2001989, NT2RM2002004, NT2RM2002088, NT2RM2002091, NT2RM4000024, NT2RM4000046, NT2RM4000104, NT2RM4000202, NT2RM4000215, NT2RM4000290, NT2RM4000531, NT2RM4000751, NT2RM4000996, NT2RM4001092, NT2RM4001140, NT2RM4001200, NT2RM4001483, NT2RM4001566, NT2RM4001592,
NT2RM4001597, NT2RM4001783, NT2RM4001823, NT2RM4001828, NT2RM4001858, NT2RM4001979, NT2RP1000035, NT2RP1000111, NT2RP1000493, NT2RP1000574, NT2RP1000630, NT2RP1000902, NT2RP1000915, NT2RP1000958, NT2RP1000966, NT2RP1001013, NT2RP1001177, NT2RP2000008, NT2RP2000076, NT2RP2000126, NT2RP2000153, NT2RP2000161, NT2RP2000248, NT2RP2000258, NT2RP2000297, NT2RP2000420, NT2RP2000931, NT2RP2001233, NT2RP2001420, NT2RP2001756, NT2RP2001869, NT2RP2002079, NT2RP2002270, NT2RP2002503, NT2RP2002591, NT2RP2002880, NT2RP2002939, NT2RP2002993, NT2RP2003137, NT2RP2003157, NT2RP2003277, NT2RP2003286, NT2RP2003308, NT2RP2003347, NT2RP2003714, NT2RP2003912, NT2RP2004013, NT2RP2004187, NT2RP2004689, NT2RP2004920, NT2RP2005393, NT2RP2005436, NT2RP2005496, NT2RP2005539, NT2RP2005701, NT2RP2005767, NT2RP2005776, NT2RP2005933, NT2RP2005942, NT2RP2006043, NT2RP2006436, NT2RP3000031, NT2RP3000050, NT2RP3000397, NT2RP3000512, NT2RP3000527, NT2RP3000590, NT2RP3000603, NT2RP3000632, NT2RP3000917, NT2RP3001057, NT2RP3001107, NT2RP3001120, NT2RP3001253, NT2RP3001338, NT2RP3001384, NT2RP3001398, NT2RP3001427, NT2RP3001428, NT2RP3001472, NT2RP3001646, NT2RP3001671, NT2RP3001792, NT2RP3001855, NT2RP3002056, NT2RP3002165, NT2RP3002399, NT2RP3002876, NT2RP3003193, NT2RP3003212, NT2RP3003555, NT2RP3004016, NT2RP3004206, NT2RP3004424, NT2RP3004428, NT2RP3004566, NT2RP3004617, NT2RP4000078, NT2RP4000111, NT2RP4000210, NT2RP4000398, NT2RP4000481, NT2RP4000518, NT2RP4000997, NT2RP4001148, NT2RP4001206, NT2RP4001213, NT2RP4001433, NT2RP4001568, NT2RP4001638, NT2RP4001696, NT2RP4001753, NT2RP4001938, NT2RP4002058, NT2RP4002078, NT2RP4002081, NT2RP4002791, OVARC1000006, OVARC1000087, OVARC1000091, OVARC1000241, OVARC1000326, OVARC1000556, OVARC1000846, OVARC1001038, OVARC1001180, OVARC1001232, OVARC1001271, OVARC1001306, OVARC1001436, OVARC1002112, PLACE1000133, PLACE1000184, PLACE1000406, PLACE1000583, PLACE1000596, PLACE1000979, PLACE1001118, PLACE1001383, PLACE1001632, PLACE1002171, PLACE1002433, PLACE1002438, PLACE1002532, PLACE1002775, PLACE1002816, PLACE1002834, PLACE1003100, PLACE1003190, PLACE1003302, PLACE1003519, PLACE1003521, PLACE1003605, PLACE1003704, PLACE1003738, PLACE1003885, PLACE1003923, PLACE1004302, PLACE1004471, PLACE1004564, PLACE1004814, PLACE1004902, PLACE1005287, PLACE1005876, PLACE1005966, PLACE1006167, PLACE1006438, PLACE1006482, PLACE1006829, PLACE1006878, PLACE1006917, PLACE1007014, PLACE1007547, PLACE1007598, PLACE1007688, PLACE1007969, PLACE1008044, PLACE1008132, PLACE1008603, PLACE1009099, PLACE1009130, PLACE1009308, PLACE1009398, PLACE1010134, PLACE1010194, PLACE1010702, PLACE1010720, PLACE1010870, PLACE1011056, PLACE1011433, PLACE1011664, PLACE2000014, PLACE2000427, PLACE3000009, PLACE3000169, PLACE4000014, PLACE4000156, PLACE4000192, PLACE4000261, PLACE4000326, PLACE4000489, SKNMC1000011, THYRO1000085, THYRO1000242, THYRO1000585, THYRO1001100, THYRO1001189, THYRO1001809, Y79AA1000037, Y79AA1000214, Y79AA1000231, Y79AA1000589, Y79AA1000752, Y79AA1001391, Y79AA1001613, Y79AA1001705, Y79AA1001963, Y79AA1002431, Y79AA1002472, Y79AA1002482

The following 292 clones presumably belong to DNA- and/or RNA-binding proteins.
HEMBA1000158, HEMBA1000216, HEMBA1000561, HEMBA1000591, HEMBA1000851, HEMBA1001088, HEMBA1001137, HEMBA1001405, HEMBA1001510, HEMBA1001804, HEMBA1001809, HEMBA1001819, HEMBA1001847, HEMBA1001869, HEMBA1002177, HEMBA1002935, HEMBA1003408, HEMBA1003545, HEMBA1003568, HEMBA1003591, HEMBA1003662, HEMBA1003684, HEMBA1003760, HEMBA1003783, HEMBA1003805, HEMBA1003953, HEMBA1004321, HEMBA1004354, HEMBA1004389, HEMBA1004479, HEMBA1004669, HEMBA1004847, HEMBA1004973, HEMBA1005202, HEMBA1005359, HEMBA1005931, HEMBA1006248, HEMBA1006278, HEMBA1006283, HEMBA1006359, HEMBA1006652, HEMBA1007087, HEMBA1007194, HEMBB1000264, HEMBB1000789, HEMBB1001011, HEMBB1001482, HEMBB1001736, HEMBB1001749, HEMBB1001839, HEMBB1002217, MAMMA1000183, MAMMA1000284, MAMMA1000731, MAMMA1001105, MAMMA1001222, MAMMA1001260, MAMMA1001743, MAMMA1001837, MAMMA1002385, MAMMA1002617, MAMMA1002869, MAMMA1002937, MAMMA1003011, NT2RM1000086, NT2RM1000539, NT2RM1000555, NT2RM1000666, NT2RM1000691, NT2RM1000826, NT2RM1000885, NT2RM1001059, NT2RM1001092, NT2RM2000371, NT2RM2000624, NT2RM2000735, NT2RM2001105, NT2RM2001424, NT2RM2001575, NT2RM2001605, NT2RM2001670, NT2RM2001771, NT2RM2001823, NT2RM2001989, NT2RM2002004, NT2RM2002014, NT2RM2002088, NT2RM2002091, NT2RM4000046, NT2RM4000104, NT2RM4000167, NT2RM4000191, NT2RM4000202, NT2RM4000531, NT2RM4000595, NT2RM4000733, NT2RM4000751, NT2RM4000996, NT2RM4001092, NT2RM4001140,
NT2RM4001178, NT2RM4001200, NT2RM4001483, NT2RM4001592, NT2RM4001783, NT2RM4001823, NT2RM4001828, NT2RM4001858, NT2RM4001880, NT2RM4001979, NT2RM4002093, NT2RM4002109, NT2RP1000470, NT2RP1000493, NT2RP1000574, NT2RP1000902, NT2RP1000966, NT2RP1001013, NT2RP1001073, NT2RP1001080, NT2RP2000008, NT2RP2000153, NT2RP2000258, NT2RP2000297, NT2RP2001127, NT2RP2001174, NT2RP2001233, NT2RP2001511, NT2RP2001756, NT2RP2001869, NT2RP2002079, NT2RP2002099, NT2RP2002503, NT2RP2002591, NT2RP2002939, NT2RP2003157, NT2RP2003329, NT2RP2003347, NT2RP2003480, NT2RP2003522, NT2RP2003564, NT2RP2003714, NT2RP2004187, NT2RP2004568, NT2RP2004920, NT2RP2005003, NT2RP2005139, NT2RP2005168, NT2RP2005436, NT2RP2005496, NT2RP2005701, NT2RP2005763, NT2RP2005776, NT2RP2005942, NT2RP2006043, NT2RP2006436, NT2RP2006464, NT2RP3000050, NT2RP3000512, NT2RP3000527, NT2RP3000562, NT2RP3000590, NT2RP3000603, NT2RP3000624, NT2RP3000632, NT2RP3000994, NT2RP3001057, NT2RP3001107, NT2RP3001120, NT2RP3001150, NT2RP3001155, NT2RP3001338, NT2RP3001398, NT2RP3001472, NT2RP3001672, NT2RP3001688, NT2RP3001724, NT2RP3001792, NT2RP3001855, NT2RP3002165, NT2RP3002399, NT2RP3002876, NT2RP3003138, NT2RP3003193, NT2RP3003251, NT2RP3003327, NT2RP3003555, NT2RP3004013, NT2RP3004078, NT2RP3004428, NT2RP3004490, NT2RP3004566, NT2RP3004594, NT2RP3004617, NT2RP3004618, NT2RP4000111, NT2RP4000398, NT2RP4000455, NT2RP4000518, NT2RP4000648, NT2RP4000865, NT2RP4000929, NT2RP4001080, NT2RP4001095, NT2RP4001213, NT2RP4001433, NT2RP4001568, NT2RP4001696, NT2RP4001753, NT2RP4001838, NT2RP4001938, NT2RP4002078, OVARC1000006, OVARC1000087, OVARC1000241, OVARC1000746, OVARC1000846, OVARC1001232, OVARC1001271, OVARC1001306, OVARC1001987, OVARC1002112, PLACE1000406, PLACE1000583, PLACE1000979, PLACE1001118, PLACE1001632, PLACE1001739, PLACE1002438, PLACE1002532, PLACE1002775, PLACE1002834, PLACE1003302, PLACE1003519, PLACE1003605, PLACE1003704, PLACE1003738, PLACE1003885, PLACE1004471, PLACE1004564, PLACE1004814, PLACE1005584, PLACE1005876, PLACE1005951, PLACE1006196, PLACE1006482, PLACE1006488, PLACE1006531, PLACE1006917, PLACE1007346, PLACE1007547, PLACE1007598, PLACE1007688, PLACE1007969, PLACE1008132, PLACE1009099, PLACE1009246, PLACE1009398, PLACE1009476, PLACE1009622, PLACE1010053, PLACE1010194, PLACE1010702, PLACE1010870, PLACE1011056, PLACE1011114, PLACE1011433, PLACE2000427, PLACE3000009, PLACE3000169, PLACE4000014, PLACE4000156, PLACE4000192, PLACE4000261, PLACE4000489, SKNMC1000091, THYRO1000085, THYRO1000242, THYRO1000501, THYRO1001100, THYRO1001189, THYRO1001809, Y79AA1000037, Y79AA1000349, Y79AA1000752, Y79AA1001211, Y79AA1001312, Y79AA1001391, Y79AA1001613, Y79AA1002103, Y79AA1002472, Y79AA1002482,

The following 66 clones presumably belong to the category of RNA synthesis-associated proteins.
HEMBA1000591, HEMBA1001579, HEMBA1003179, HEMBA1003591, HEMBA1006278, HEMBB1000226, NT2RM1000187, NT2RM1000852, NT2RM2000624, NT2RM2001989, NT2RM2002100, NT2RM4000191, NT2RM4001178, NT2RM4002093, NT2RP1000035, NT2RP1000272, NT2RP1000470, NT2RP1001080, NT2RP2000153, NT2RP2002928, NT2RP2003157, NT2RP2004568, NT2RP2005126, NT2RP2005436, NT2RP2005539, NT2RP2005605, NT2RP2005776, NT2RP2005942, NT2RP2006043, NT2RP2006238, NT2RP3000361, NT2RP3000397, NT2RP3001671, NT2RP3004504, NT2RP4000078, NT2RP4000111, NT2RP4000481, NT2RP4000518, NT2RP4000614, NT2RP4000929, NT2RP4001696, NT2RP4002058, OVARC1001232, OVARC1001577, PLACE1000406, PLACE1000596, PLACE1000755, PLACE1001739, PLACE1003704, PLACE1003885, PLACE1004564, PLACE1004814, PLACE1004902, PLACE1005373, PLACE1005646, PLACE1005876, PLACE1006196, PLACE1006626, PLACE1006878, PLACE1006917, PLACE1009476, PLACE1009925, PLACE1010194, PLACE1011114, THYRO1000121, Y79AA1001963,

The following 183 clones presumably belong to protein synthesis-associated and/or protein transport-associated proteins.
HEMBA1000012, HEMBA1000141, HEMBA1000592, HEMBA1003617, HEMBA1003773, HEMBA1004202, HEMBA1004276, HEMBA1004734, HEMBA1004847, HEMBA1004929, HEMBA1004930, HEMBA1005047, HEMBA1005202, HEMBA1006031, HEMBA1006272, HEMBA1006474, HEMBA1006652, HEMBA1006914, HEMBA1006973, HEMBA1007224, HEMBB1000915, HEMBB1001112, HEMBB1001137, HEMBB1001736, HEMBB1001831, HEMBB1001915, MAMMA1000085, MAMMA1000734, MAMMA1001008, MAMMA1002170, MAMMA1002219, MAMMA1002236, MAMMA1002619, NT2RM1000661, NT2RM1000833, NT2RM2000092, NT2RM2000504, NT2RM2000577, NT2RM2000821, NT2RM2001201, NT2RM2001592, NT2RM2001613, NT2RM2001648, NT2RM2001730, NT2RM2001760, NT2RM2002055, NT2RM4000155, NT2RM4000169, NT2RM4000344, NT2RM4000356, NT2RM4000421, NT2RM4000712, NT2RM4001054, NT2RM4001203, NT2RM4001382, NT2RM4001444, NT2RM4002062, NT2RM4002205, _ NT2RM4002623, NT2RP1000326, NT2RP1000522, NT2RP1000547, NT2RP1000746, NT2RP1000947, NT2RP1001569, NT2RP2000147, NT2RP2000710, NT2RP2000880, NT2RP2000943, NT2RP2001290, NT2RP2001392, NT2RP2001601, NT2RP2001613, NT2RP2001660, NT2RP2001740, NT2RP2002124, NT2RP2002606, NT2RP2002862, NT2RP2002959, NT2RP2002980, NT2RP2003137, NT2RP2003158, NT2RP2003391, NT2RP2003394, NT2RP2003401, NT2RP2003433, NT2RP2003704, NT2RP2003713, NT2RP2003737, NT2RP2003760, NT2RP2003981, NT2RP2004366, NT2RP2004389, NT2RP2004791, NT2RP2005012, NT2RP2005116, NT2RP2005360, NT2RP2005763, NT2RP2005784, NT2RP3000366,
NT2RP3000759, NT2RP3000968, NT2RP3001113, NT2RP3001690, NT2RP3002045, NT2RP3002151, NT2RP3002529, NT2RP3002671, NT2RP3003301, NT2RP3003846, NT2RP3003876, NT2RP3004209, NT2RP4000370, NT2RP4000457, NT2RP4000879, NT2RP4000927, NT2RP4001041, NT2RP4001117, NT2RP4001313, NT2RP4001315, NT2RP4001574, NT2RP4001592, OVARC1000013, OVARC1000071, OVARC1000085, OVARC1000465, OVARC1000564, OVARC1000771, OVARC1000862, OVARC1001171, OVARC1001180, OVARC1001342, PLACE1000007, PLACE1000061, PLACE1000081, PLACE1000492, PLACE1000863, PLACE1001092, PLACE1001748, PLACE1002090, PLACE1003174, PLACE1003915, PLACE1004104, PLACE1004270, PLACE1004743, PLACE1005557, PLACE1005813, PLACE1006170, PLACE1006488, PLACE1006829, PLACE1007706, PLACE1007729, PLACE1008273, PLACE1008402, PLACE1008790, PLACE1008813, PLACE1009094, PLACE1009130, PLACE1009477, PLACE1009721, PLACE1009845, PLACE1010074, PLACE1010547, PLACE1011109, PLACE1011229, PLACE1011477, PLACE1012031, PLACE2000404, PLACE3000059, PLACE3000121, PLACE4000269, PLACE4000654, SKNMC1000011, THYRO1000983, THYRO1001003, THYRO1001313, Y79AA1000560, Y79AA1000784, Y79AA1000968, Y79AA1001493, Y79AA1001875, Y79AA1002027, Y79AA1002209,

The following 130 clones presumably belong to cytoskeletal-associated proteins.
HEMBA1000156, HEMBA1000168, HEMBA1000411, HEMBA1000588, HEMBA1001043, HEMBA1001651, HEMBA1001661, HEMBA1002102, HEMBA1002161, HEMBA1002939, HEMBA1003235, HEMBA1003581, HEMBA1004499, HEMBA1004534, HEMBA1004697, HEMBA1004929, HEMBA1004972, HEMBA1005582, HEMBA1005595, HEMBA1006344, HEMBA1006737, HEMBB1001175, HEMBB1001282, HEMBB1001562, HEMBB1001802, MAMMA1000824, MAMMA1001041, MAMMA1001576, MAMMA1001679, MAMMA1001735, MAMMA1002297, MAMMA1002351, MAMMA1002622, MAMMA1002637, MAMMA1003127, NT2RM1000850, NT2RM1000898, NT2RM2000030, NT2RM2000260, NT2RM2000691, NT2RM2001324, NT2RM4000169, NT2RM4000229, NT2RM4000515, NT2RM4001217, NT2RP1000202, NT2RP1000348, NT2RP1000460, NT2RP1000478, NT2RP1001033, NT2RP1001294, NT2RP1001302, NT2RP2000070, NT2RP2000812, NT2RP2000814, NT2RP2001168, NT2RP2001245, NT2RP2001634, NT2RP2001900, NT2RP2003307, NT2RP2003394, NT2RP2004041, NT2RP2004242, NT2RP2004538, NT2RP2004587, NT2RP2004681, NT2RP2004732, NT2RP2004978, NT2RP2005491, NT2RP2005531, NT2RP2005712, NT2RP2006275, NT2RP3000753, NT2RP3001113, NT2RP3001216, NT2RP3001239, NT2RP3001272, NT2RP3001554, NT2RP3001690, NT2RP3001799, NT2RP3002688, NT2RP3003061, NT2RP3003185, NT2RP3003230, NT2RP3004569, NT2RP3004578, NT2RP4001004, NT2RP4001086, NT2RP4001256, NT2RP4001567, NT2RP4001927, OVARC1000001, OVARC1000106, OVARC1000437, OVARC1000520, OVARC1000679, OVARC1001731, OVARC1002050, PLACE1001104, PLACE1002571,
PLACE1002591, PLACE1002655, PLACE1002714, PLACE1003625, PLACE1005287, PLACE1006552, PLACE1007946, PLACE1008426, PLACE1010148, PLACE1010547, PLACE1010743, PLACE1010896, PLACE1010960, PLACE1011310, PLACE1011922, PLACE2000216, PLACE2000274, PLACE2000371, PLACE2000458, PLACE3000145, PLACE3000416, PLACE4000009, THYRO1000132, THYRO1001405, THYRO1001458, Y79AA1000368, Y79AA1000794, Y79AA1000833, Y79AA1000962, Y79AA1002208,

The following 54 clones presumably belong to cell division-associated and/or cell proliferation-associated proteins.
HEMBA1001019, HEMBA1001595, HEMBA1002363, HEMBA1002997, HEMBA1003136, HEMBA1003369, HEMBA1004131, HEMBA1004354, HEMBA1005621, HEMBB1000037, HEMBB1000264, MAMMA1001768, MAMMA1002769, NT2RM1000354, NT2RM1000430, NT2RM1000874, NT2RM2001256, NT2RM2001743, NT2RM2001896, NT2RM2002145, NT2RM4000215, NT2RM4001714, NT2RP1000163, NT2RP1000333, NT2RP1000439, NT2RP2000346, NT2RP2001397, NT2RP2002595, NT2RP2003177, NT2RP2003596, NT2RP2003912, NT2RP2004396, NT2RP2005037, NT2RP2005520, NT2RP2005669, NT2RP2005835, NT2RP3001730, NT2RP3002081, NT2RP4000210, NT2RP4000415, NT2RP4001414, NT2RP4001634, OVARC1000013, OVARC1000937, PLACE1001383, PLACE1002433, PLACE1004316, PLACE1005287, PLACE1008808, PLACE1010720, PLACE1010833, Y79AA1000748, Y79AA1001236, Y79AA1001394,

The following 36 clones presumably belong to the category of embryogenesis- and/or development-associated proteins.
HEMBA1000518, HEMBA1001847, HEMBA1001869, HEMBA1003545, HEMBA1004973, HEMBB1002442, MAMMA1001837, NT2RM2001670, NT2RM4000046, NT2RM4000531, NT2RM4001140, NT2RM4001858, NT2RP2002078, NT2RP2004187, NT2RP2006436, NT2RP3000603, NT2RP3000994, NT2RP3001580, NT2RP3001708, NT2RP3003071, NT2RP3004472, NT2RP3004617, NT2RP4000246, NT2RP4001567, OVARC1000304, OVARC1000746, PLACE1000793, PLACE1002532, PLACE1003258, PLACE1003625, PLACE1004460, PLACE1009622, PLACE4000558, THYRO1000085, Y79AA1001391, Y79AA1001692,

The following 30 clones presumably belong to cellular defense-associated proteins.
HEMBA1000005, HEMBA1000531, HEMBA1003417, HEMBA1006253, NT2RM4000354, NT2RM4001880, NT2RP1000333, NT2RP1000493, NT2RP2000006, NT2RP2000045, NT2RP2000809, NT2RP2001536, NT2RP2002464, NT2RP2004920, NT2RP2005037, NT2RP3000590, NT2RP3001426, NT2RP3002062, NT2RP3002785, NT2RP3004262, NT2RP4001555, NT2RP4001638, PLACE1006958, PLACE1008275, PLACE1009113, PLACE1011858, PLACE4000014, THYRO1000684, Y79AA1002139, Y79AA1002229,

Although it is unclear whether or not 261 clones out of clones other than the above-mentioned clones belong to any of the above-described categories, these clones are predicted to have some functions, based on the homology search using the full-length sequences thereof. The clone names and the gene definitions found in the result of homology search are shown below, separated with a double-slash mark, //.
HEMBA1000030//Homo sapiens ARF GTPase-activating protein GlT1 mRNA, complete cds.
HEMBA1000307//CARNITINE DEFICIENCY-ASSOCIATED PROTEIN EXPRESSED IN VENTRICLE 1

HEMBA1000333//Homo sapiens F-box protein Fbx21 (FBX21) mRNA, complete cds.
HEMBA1000488//RING CANAL PROTEIN (KELCH PROTEIN).
HEMBA1000523//TESTIS-SPECIFIC PROTEIN PBS13.
HEMBA1001197//Homo sapiens rap2 interacting protein x mRNA, complete cds.
HEMBA1001302//Homo sapiens calcium binding protein precursor, mRNA, complete cds.
HEMBA1001455//Mus musculus transposon-derived Buster2 transposase-like protein gene, partial cds.
HEMBA1001675//VACUOLAR PROTEIN SORTING-ASSOCIATED PROTEIN VPS9.
HEMBA1001714//Homo sapiens mRNA for ATPase inhibitor precursor, complete cds.
HEMBA1001744//SCY1 PROTEIN.
HEMBA1001967//Homo sapiens NY-REN-57 antigen mRNA, partial cds.
HEMBA1002151//Rattus norvegicus p34 mRNA, complete cds.
HEMBA1002215//TESTIN 2 (TES2) [CONTAINS: TESTIN 1 (TES1)].
HEMBA1002458//OVARIAN GRANULOSA CELL 13.0 KD PROTEIN HGR74.
HEMBA1002777//Fugu rubripes BAW (BAW) mRNA, complete cds.
HEMBA1003098//Homo sapiens NY-REN-6 antigen mRNA, partial cds.
HEMBA1003199//Homo sapiens chromosome 5 F-box protein Fbx4 (FBX4) mRNA, complete cds.
HEMBA1003615//Homo sapiens ART-4 mRNA, complete cds.
HEMBA1003836//MOB1 PROTEIN (MPS1 BINDER 1).
HEMBA1004295//Homo sapiens NY-REN-25 antigen mRNA, partial cds.
HEMBA1004573//Homo sapiens mRNA for HELG protein.
HEMBA1004604//Homo sapiens COP9 complex subunit 7a mRNA, complete cds.
HEMBA1004795//CDC4-LIKE PROTEIN (FRAGMENT).
HEMBA1005101//Homo sapiens SYT interacting protein SIP mRNA, complete cds.
HEMBA1005201//Homo sapiens CGI-07 protein mRNA, complete cds.
HEMBA1005206//Drosophila simulans anon73B1 gene and Su(P) gene.
HEMBA1005530//Homo sapiens anaphase-promoting complex subunit 7 (APC7) mRNA, complete cds.
HEMBA1005666//Homo sapiens mRNA for DIPB protein.
HEMBA1005990//Homo sapiens I-1 receptor candidate protein mRNA, complete cds.
HEMBA1006268//Homo sapiens HQOO24c mRNA, complete cds.
HEMBA1006398//Human L1 element L1.6 putative p150 gene, complete cds.
HEMBA1006445//Homo sapiens putative tumor supressor NOEY2 mRNA, complete cds.
HEMBA1007174//Homo sapiens epsin 2b mRNA, complete cds.
HEMBA1007251//Homo sapiens F-box protein FBX29 (FBX29) mRNA, partial cds. HEMBB1000036//Homo sapiens CGI-51 protein mRNA, complete cds.
HEMBB1000144//GUANYLATE CYCLASE ACTIVATING PROTEIN 2 (GCAP 2) (RETINAL
GUANYLYL CYCLASE ACTIVATOR PROTEIN P24).
HEMBB1000973//Mus musculus schlafen3 (Slfn3) mRNA, complete cds.
HEMBB1001058//Homo sapiens neuronal thread protein AD7c-NTP mRNA, complete cds
HEMBB1001234//65 KD YES-ASSOCIATED PROTEIN (YAP65).
HEMBB1001288//COPPER HOMEOSTASIS PROTEIN CUTC.
HEMBB1001331//Mus musculus mRNA for hepatoma-derived growth factor, complete cds, strain:BALB/c.
HEMBB1001384//Homo sapiens COP9 complex subunit 4 mRNA, complete cds.
HEMBB1002266//NEURONAL PROTEIN.
HEMBB1002510//GYP7 PROTEIN.
HEMBB1002705//Homo sapiens CGI-27 protein mRNA, complete cds.
MAMMA1000055//TESTIN 2 (TES2) [CONTAINS: TESTIN 1 (TEST)].
MAMMA1000625//GYP7 PROTEIN.
MAMMA1001075//Homo sapiens CGI-72 protein mRNA, complete cds.
MAMMA1001181//ABC1 PROTEIN HOMOLOG PRECURSOR.
MAMMA1001259//Mus musculus F-box protein FBX18 mRNA, partial cds.
MAMMA1001730//Homo sapiens brain and nasopharyngeal carcinoma susceptibility protein NSG-x mRNA, partial cds.
MAMMA1002143//Homo sapiens Cdc42 effector protein 4 mRNA, complete cds.
MAMMA1002699//Rattus norvegicus EH domain binding protein Epsin mRNA, complete cds.
MAMMA1002972//VACUOLAR PROTEIN SORTING-ASSOCIATED PROTEIN VPS27.
MAMMA1003113//Mus musculus COP9 complex subunit 7a (COPS7a) mRNA, complete cds.
NT2RM1000118//CALCINEURIN B SUBUNIT (PROTEIN PHOSPHATASE 2B REGULATORY SUBUNIT) (CALCINEURIN REGULATORY SUBUNIT).
NT2RM1000186//CALCINEURIN B SUBUNIT (PROTEIN PHOSPHATASE 2B REGULATORY SUBUNIT) (CALCINEURIN REGULATORY SUBUNIT).
NT2RM1000244//Homo sapiens TRAF4 associated factor 1 mRNA, partial cds.
NT2RM1000421//RIBONUCLEASE INHIBITOR.
NT2RM1000499//Caenorhabditis elegans mRNA for centaurin gamma 1A.
NT2RM1000623//RIBONUCLEASE INHIBITOR.
NT2RM1000883//Homo sapiens I-1 receptor candidate protein mRNA, complete cds.
NT2RM2000502//Rattus norvegicus W3O7 mRNA, complete cds.
NT2RM2000599//Homo sapiens F-box protein Lilina (LILINA) mRNA, complete cds.
NT2RM2000718//Homo sapiens endocrine regulator mRNA, complete cds.
NT2RM2001065//Homo sapiens COP9 complex subunit 4 mRNA, complete cds.
NT2RM2001196//PROLINE-RICH PROTEIN MP-3 (FRAGMENT).
NT2RM2001983//Homo sapiens RGS-GAIP interacting protein GIPC mRNA, complete cds.
NT2RM2002109//Homo sapiens glioma amplified on chromosome 1 protein (GAC1) mRNA, complete cds.
NT2RM2002142//GASTRULATION SPECIFIC PROTEIN G12.
NT2RM4000030//LAS1 PROTEIN.
NT2RM4000139//R.norvegicus trg mRNA.
NT2RM4000156//H. sapiens HPBRII-7 gene.
NT2RM4000386//Mus musculus ODZ3 (Odz3) mRNA, partial cds.
NT2RM4000590//RING CANAL PROTEIN (KELCH PROTEIN).
NT2RM4001047//MO25 PROTEIN.
NT2RM4001155//ADRENAL MEDULLA 50 KD PROTEIN.
NT2RM4001256//Xenopus laevis putative Zic3 binding protein mRNA, complete cds.
NT2RM4001320//Homo sapiens mRNA for Neuroblastoma, complete cds.
NT2RM4001340//UTR4 PROTEIN (UNKNOWN TRANSCRIPT 4 PROTEIN).
NT2RM4001347//Homo sapiens NY-REN-25 antigen mRNA, partial cds.
NT2RM4001371//Homo sapiens IDN3 mRNA, partial cds.
NT2RM4001582//Mus musculus COP9 complex subunit 7b (COPS7b) mRNA, complete cds.
NT2RM4001611//SIS2 PROTEIN (HALOTOLERANCE PROTEIN HAL3).
NT2RM4001731//Homo sapiens F-box protein Lilina (LILINA) mRNA, complete cds.
NT2RM4001969//R.norvegicus mRNA for IP63 protein.
NT2RM4002034//Homo sapiens hiwi mRNA, partial cds.
NT2RM4002075//RING CANAL PROTEIN (KELCH PROTEIN).
NT2RM4002226//GTPASE ACTIVATING PROTEIN ROTUND.
NT2RP1000040//Mus musculus donson protein (Donson) mRNA, partial cds.
NT2RP1000363//R.norvegicus LL5 mRNA.
NT2RP1000481//Homo sapiens antigen NY-CO-3 (NY-CO-3) mRNA, partial cds.
NT2RP1000513//Human NifU-like protein (hNifU) mRNA, partial cds.
NT2RP1000733//Human mRNA for GSPT1-TK protein,complete cds.
NT2RP1000860//Homo sapiens KLO4P mRNA, complete cds.
NT2RP1000954//RING CANAL PROTEIN (KELCH PROTEIN).
NT2RP1001011//Drosophila melanogaster putative 43 kDa protein (TH1) mRNA, complete cds.
NT2RP1001395//Homo sapiens COP9 complex subunit 7a mRNA, complete cds.
NT2RP1001457//Homo sapiens partial mRNA for beta-transducin family protein (putative).
NT2RP1001494//MALE STERILITY PROTEIN 2.
NT2RP2000054//Homo sapiens putative ring zinc finger protein NY-REN-43 antigen mRNA, complete cds.
NT2RP2000067//Mus musculus ODZ3 (Odz3) mRNA, partial cds.
NT2RP2000133//Homo sapiens Leman coiled-coil protein (LCCP) mRNA, complete cds.
NT2RP2000157//MLO2 PROTEIN.
NT2RP2000764//NIFS PROTEIN.
NT2RP2000965//Homo sapiens mRNA for fls353, complete cds.
NT2RP2001839//SCY1 PROTEIN.
NT2RP2001883//Homo sapiens CGI-01 protein mRNA, complete cds.
NT2RP2001976//Mus musculus calmodulin-binding protein SHA1 (Sha1) mRNA, complete cds.
NT2RP2001985//Homo sapiens high-risk human papilloma viruses E6 oncoproteins targeted protein E6TP1 alpha mRNA, complete cds.
NT2RP2002185//Homo sapiens ubiquilin mRNA, complete cds.
NT2RP2002442//HESA PROTEIN.
NT2RP2002727//Rattus norvegicus tulip 2 mRNA, complete cds.
NT2RP2002741//Homo sapiens mRNA for Neuroblastoma, complete cds.
NT2RP2002986//Homo sapiens mRNA for Kelch motif containing protein, complete cds.
NT2RP2003121//Mus musculus enhancer of polycomb (Epc1) mRNA, complete cds.
NT2RP2003265//Homo sapiens CGI-53 protein mRNA, complete cds.
NT2RP2003272//Homo sapiens ubiquilin mRNA, complete cds.
NT2RP2003857//MYOTROPHIN (V-1 PROTEIN) (GRANULE CELL DIFFERENTIATION PROTEIN).
NT2RP2003871//Homo sapiens transposon-derived Buster1 transposase-like protein gene, complete cds.
NT2RP2004425//Mus musculus axotrophin mRNA, complete cds.
NT2RP2004476//Homo sapiens cyclin L ania-6a mRNA, complete cds.
NT2RP2004710//Mus musculus formin binding protein 30 mRNA, complete cds.
NT2RP2004816//H58 PROTEIN.
NT2RP2005441//Homo sapiens hypothalamus protein HT002 mRNA, complete cds.
NT2RP2005490//Mus musculus D3Mm3e (D3Mm3e) mRNA, complete cds.
NT2RP2005620//Homo sapiens epsin 2a mRNA, complete cds.
NT2RP2005654//CYSTEINE STRING PROTEIN (CCCS1).
NT2RP2005675//Homo sapiens growth suppressor related (DOC-1R) mRNA, complete cds.
NT2RP2005753//Homo sapiens I-1 receptor candidate protein mRNA, complete cds.
NT2RP2005841//Homo sapiens mRNA for ALEX3, complete cds.
NT2RP2006598//Homo sapiens retinoid x receptor interacting protein mRNA, complete cds.
NT2RP3000047//NPL4 PROTEIN.
NT2RP3000233//RING CANAL PROTEIN (KELCH PROTEIN).
NT2RP3000868//Human ovarian cancer downregulated myosin heavy chain homolog (Doc1) mRNA, complete cds.
NT2RP3000869//Drosophila melanogaster AAA family protein Bor (bor) mRNA, complete cds.
NT2RP3001399//SSU72 PROTEIN.
NT2RP3001407//SCY1 PROTEIN.
NT2RP3001457//Drosophila melanogaster Melted (melt) mRNA, partial cds.
NT2RP3001587//Human anthracycline-associated resistance ARX mRNA, complete cds.
NT2RP3001712//Homo sapiens HP1-BP74 protein mRNA, complete cds.
NT2RP3001819//RING CANAL PROTEIN (KELCH PROTEIN).
NT2RP3001854//Homo sapiens novel retinal pigment epithelial cell protein (NORPEG) mRNA, complete cds.
NT2RP3001931//Rattus norvegicus clone C48 CDK5 activator-binding protein mRNA, complete cds.
NT2RP3002273//SCD6 PROTEIN.
NT2RP3002631//Homo sapiens Ran binding protein 11 mRNA, complete cds.
NT2RP3002682//Homo sapiens CGI-145 protein mRNA, complete cds.
NT2RP3002770//MYELOID DIFFERENTIATION PRIMARY RESPONSE PROTEIN MYD116.
NT2RP3002818//INSERTION ELEMENT IS2A HYPOTHETICAL 48.2 KD PROTEIN.
NT2RP3002948//RING CANAL PROTEIN (KELCH PROTEIN).
NT2RP3002972//Halocynthia roretzi mRNA for HrPET-1, complete cds.
NT2RP3003032//Homo sapiens okadaic acid-inducible and cAMP-regulated phosphoprotein 19 (ARPP-19) mRNA, complete cds.
NT2RP3003290//Mus musculus mRNA for Ndr1 related protein Ndr3, complete cds.
NT2RP3003411//Mus musculus COP9 complex subunit 7b (COPS7b) mRNA, complete cds.
NT2RP3003491//Drosophila melanogaster Pelle associated protein Pellino (PIi) mRNA, complete cds.
NT2RP3003500//SCY1 PROTEIN.
NT2RP3003726//Homo sapiens spermatogenesis associated PD1 mRNA, complete cds.
NT2RP3004348//R. norvegicus mRNA for cytosolic resiniferatoxin-binding protein.
NT2RP3004507//MOB1 PROTEIN (MPS1 BINDER 1).
NT2RP4000129//Xenopus laevis F-box protein 28 (Fbx28) mRNA, partial cds.
NT2RP4000498//MOB1 PROTEIN (MPS1 BINDER 1).
NT2RP4000528//NPL4 PROTEIN.
NT2RP4000737//Mus musculus F-box protein FBL10 mRNA, partial cds.
NT2RP4000979//Homo sapiens putative HIV-1 infection related protein mRNA, partial cds.
NT2RP4001010//Rattus norvegicus PSD-95/SAP90-associated protein-4 mRNA, complete cds.
NT2RP4001207//Homo sapiens Ran binding protein 11 mRNA, complete cds.
NT2RP4001228//RING CANAL PROTEIN (KELCH PROTEIN).
NT2RP4001260//Homo sapiens F-box protein Fbx21 (FBX21) mRNA, complete cds.
NT2RP4001339//Homo sapiens mRNA for AMMERC1 protein.
NT2RP4001351//Human ovarian cancer downregulated myosin heavy chain homolog (Doc1) mRNA, complete cds.
NT2RP4001474//Xenopus laevis putative Zic3 binding protein mRNA, complete cds.
NT2RP4001966//Mus musculus ODZ3 (Odz3) mRNA, partial cds.
NT2RP4002018//RING CANAL PROTEIN (KELCH PROTEIN).
OVARC1000209//Oryza sativa submergence induced protein 2A mRNA, complete cds.
OVARC1000876//MOB1 PROTEIN (MPS1 BINDER 1).
OVARC1001065//Homo sapiens CGI-12 protein mRNA, complete cds.
OVARC1001092//Homo sapiens mRNA for JM5 protein, complete CDS (clone IMAGE 53337, LLNLc110F1857O7 (RZPD Berlin) and LLNLc110G0913Q7 (RZPD Berlin)).
OVARC1001419//Homo sapiens GOK (STIM1) mRNA, complete cds.
OVARC1001555//NGG1-INTERACTING FACTOR 3.
OVARC1001711//CORNIFIN B (SMALL PROLINE-RICH PROTEIN 1B) (SPR1B) (SPR1 B).
OVARG1001943//Mus musculus DEBT-91 mRNA, complete cds.
PLACE1000004//Homo sapiens IDN3-B mRNA, complete cds.
PLACE1000066//SSU72 PROTEIN.
PLACE1000610//MSN5 PROTEIN.
PLACE1000636//MALE STERILITY PROTEIN 2.
PLACE1000769//Homo sapiens CGI-18 protein mRNA, complete cds.
PLACE1000987//Rattus norvegicus late gestation lung 2 protein (Lgl2) mRNA, complete cds.
PLACE1001036//Homo sapiens mRNA for alpha integrin binding protein 63, partial.
PLACE1001845//Mus musculus cyclin ania-6a mRNA, complete cds.
PLACE1001920//Homo sapiens MDC-3.13 isoform 2 mRNA, complete cds.
PLACE1002665//Mus musculus enhancer of polycomb (Epc1) mRNA, complete cds.
PLACE1003602//Homo sapiens mRNA expressed in placenta.
PLACE1003611//Homo sapiens anaphase-promoting complex subunit 4 (APC4) mRNA, complete cds.
PLACE1004256//Mus musculus short coiled coil protein SCOCO (Scoc) mRNA, complete cds.
PLACE1004550//Homo sapiens CGI-20 protein mRNA, complete cds.
PLACE1004868//MALE STERILITY PROTEIN 2.
PLACE1004930//Homo sapiens MDC-3.13 isoform 2 mRNA, complete cds
PLACE1005052//Homo sapiens CGI-16 protein mRNA, complete cds.
PLACE1005102//RING CANAL PROTEIN (KELCH PROTEIN).
PLACE1005176//Homo sapiens hypothalamus protein HT001 mRNA, complete cds.
PLACE1005187//APAG PROTEIN.
PLACE1005331//Homo sapiens 7h3 protein mRNA, partial cds.
PLACE1005727//Homo sapiens STRIN protein (STRIN) mRNA, complete cds.
PLACE1006003//Homo sapiens CGI-94 protein mRNA, complete cds.
PLACE1006335//Homo sapiens NY-REN-50 antigen mRNA, partial cds.
PLACE1006385//Homo sapiens epsin 2a mRNA, complete cds.
PLACE1006506//Homo sapiens anaphase-promoting complex subunit 4 (APC4) mRNA, complete cds.
PLACE1007105//Homo sapiens muskelin (MKLN1) mRNA, complete cds.
PLACE1007537//Homo sapiens ankyrin repeat-containing protein ASB-2 mRNA, complete cds.
PLACE1007705//Mus musculus mRNA for Ndr1 related protein Ndr3, complete cds.
PLACE1007791//Homo sapiens IDN3-B mRNA, complete cds.
PLACE1007897//Homo sapiens FLASH mRNA, complete cds.
PLACE1008080//Homo sapiens mRNA for HEXIM1 protein, complete cds.
PLACE1008368//RING CANAL PROTEIN (KELCH PROTEIN).
PLACE1008398//GENE 33 POLYPEPTIDE.
PLACE1008465//Homo sapiens mRNA for rapa-1 (rapa gene).
PLACE1008627//Homo sapiens mRNA for cysteine-rich protein.
PLACE1009020//NIFS PROTEIN.
PLACE1009060//BRO1 PROTEIN.
PLACE1009186//Homo sapiens small zinc finger-like protein (TlM9b) mRNA, complete cds.
PLACE1009443//Mus musculus F-box protein FBL8 mRNA, complete cds.
PLACE1009571//Homo sapiens PTD002 mRNA, complete cds.
PLACE1009670//Homo sapiens genethonin 1 mRNA, complete cds.
PLACE1010105//RING CANAL PROTEIN (KELCH PROTEIN).
PLACE1010261//SEGREGATION DISTORTER PROTEIN.
PLACE1010310//SPIDROIN 2 (DRAGLINE SILK FIBROIN 2) (FRAGMENT).
PLACE1010522//Homo sapiens mRNA for DEPP (decidual protein induced by progesterone), complete cds.
PLACE1010579//Homo sapiens CED-6 protein (CED-6) mRNA, complete cds.
PLACE1010628//Homo sapiens S164 gene, partial cds; PS1 and hypothetical protein genes, complete cds; and S171 gene, partial cds.
PLACE1010661//TESTIS-SPECIFIC PROTEIN PBS13.
PLACE1010761//Homo sapiens mRNA for cisplatin resistance-associated overexpressed protein, complete cds.
PLACE1011185//INSERTION ELEMENT IS1 PROTEIN INSB.
PLACE1011340//Homo sapiens IDN3-B mRNA, complete cds.
PLACE1011586//Rattus norvegicus clone C53 CDK5 activator-binding protein mRNA, complete cds.
PLACE2000246//RING CANAL PROTEIN (KELCH PROTEIN).
PLACE2000411//Homo sapiens epsin 2b mRNA, complete cds.
PLACE3000477//Homo sapiens phosphoprotein pp75 mRNA, partial cds.
THYRO1000173//Homo sapiens AP-mu chain family member mu1B (HSMU1B) mRNA, complete cds.
THYRO1000401//Human TcD37 homolog (HTcD37) mRNA, partial cds.
THYRO1000666//Mus musculus mRNA for kinesin like protein 9.
THYRO1001033//TRANSFORMATION-SENSITIVE PROTEIN IEF SSP 3521.
THYRO1001347//Homo sapiens RAN binding protein 16 mRNA, complete cds.
THYRO1001656//Homo sapiens Leman coiled-coil protein (LCCP) mRNA, complete cds.
THYRO1001703//NIFR3-LIKE PROTEIN.
THYRO1001721//RING CANAL PROTEIN (KELCH PROTEIN).
Y79AA1000059//Homo sapiens aryl-hydrocarbon interacting protein-like 1 (AIPL1) gene, complete cds.
Y79AA1000181//Homo sapiens CGI-01 protein mRNA, complete cds.
Y79AA1000268//Mus musculus Nip2I mRNA, complete cds.
Y79AA1000313//CALPHOTIN.
Y79AA1000540//CELL POLARITY PROTEIN TEA1.
Y79AA1000966//Homo sapiens COP9 complex subunit 4 mRNA, complete cds.
Y79AA1000985//Human centrosomal protein kendrin mRNA, complete cds.
Y79AA1001323//Mus musculus mRNA for GSG1, complete cds.
Y79AA1001402//Homo sapiens paraneoplastic cancer-testis-brain antigen (MA4) mRNA, partial cds.
Y79AA1001679//Homo sapiens lambda-crystallin mRNA, complete cds.
Y79AA1001923//Homo sapiens F-box protein Fbx22 (FBX22) gene, partial cds. Y79AA1002083//H. sapiens mRNA for MUF1 protein.
Y79AA1002307//Homo sapiens astrotactin2 (ASTN2) mRNA, complete cds.
Y79AA1002311//R. norvegicus mRNA for cytosolic resiniferatoxin-binding protein.
Y79AA1002487//Homo sapiens chromosome 5 F-box protein Fbx4 (FBX4) mRNA, complete cds.

Among the clones other than the above-mentioned, there were 36 clones that were similarly classified into the functional categories based on the results of functional domain search using the Pfam program. These clones were categorized as follows.

Clones presumably belonging to the category of secretory or membrane proteins are two clones, MAMMA1002498 and NT2RM4002287; a clone presumably belonging to the category of glycoproteins-associated proteins is a clone MAMMA1002498; clones presumably belonging to the category of signal transduction-associated proteins are 11 clones, HEMBA1001247, NT2RM2001813, NT2RM4001454, NT2RP2005140, NT2RP2005293, NT2RP3000487, NT2RP3003311, PLACE1000972, PLACE1003723, PLACE1005327, and PLACE3000124; clones presumably belonging to the category of transcription-associated proteins are 12 clones, HEMBA1003257, NT2RM2000101, NT2RM2001797, NT2RP1000101, NT2RP2002208, NT2RP3001214, NT2RP3003278, NT2RP4001235, PLACE1000050, PLACE1001716, PLACE1002499, and PLACE1007544; clones presumably belonging to the category of enzymes and/or metabolism-associated proteins are 2 clones, HEMBA1005732 and MAMMA1000402; clones presumably belonging to the category of DNA- and/or RNA-binding proteins are 4 clones, HEMBA1004596, OVARC1000148, PLACE1003334, and THYRO1001661; a clone presumably belonging to the category of protein synthesis- and/or protein transport-associated proteins is a clone, HEMBA1006284.

So far, useful information for presuming the functions is unavailable for the remaining 2511 clones. Their functions will possibly be revealed by further analyses. Names of the clones are listed below.

So far, useful information for presuming the functions are unavailable for the remaining 2511 clones. Their functions will possibly be revealed by further analyses. Names of the clones are listed below.
HEMBA1000042, HEMBA1000046, HEMBA1000050, HEMBA1000076, HEMBA1000193, HEMBA1000213, HEMBA1000227, HEMBA1000231, HEMBA1000243, HEMBA1000244, HEMBA1000251, HEMBA1000264, HEMBA1000280, HEMBA1000282, HEMBA1000288, HEMBA1000290, HEMBA1000302, HEMBA1000327, HEMBA1000338, HEMBA1000351, HEMBA1000357, HEMBA1000376, HEMBA1000387, HEMBA1000392, HEMBA1000396, HEMBA1000428, HEMBA1000442, HEMBA1000456, HEMBA1000459, HEMBA1000460, HEMBA1000469, HEMBA1000497, HEMBA1000501, HEMBA1000504, HEMBA1000505, HEMBA1000508, HEMBA1000519, HEMBA1000520, HEMBA1000534, HEMBA1000545, HEMBA1000557, HEMBA1000568, HEMBA1000575, HEMBA1000594, HEMBA1000604, HEMBA1000622, HEMBA1000636, HEMBA1000655, HEMBA1000673, HEMBA1000682, HEMBA1000686, HEMBA1000702, HEMBA1000722, HEMBA1000726, HEMBA1000727, HEMBA1000749, HEMBA1000752, HEMBA1000769, HEMBA1000773, HEMBA1000774, HEMBA1000843, HEMBA1000867, HEMBA1000869, HEMBA1000872, HEMBA1000876, HEMBA1000908, HEMBA1000918, HEMBA1000934, HEMBA1000942, HEMBA1000943, HEMBA1000946, HEMBA1000960, HEMBA1000968, HEMBA1000971, HEMBA1000972, HEMBA1000975, HEMBA1000985, HEMBA1000986, HEMBA1001008, HEMBA1001009, HEMBA1001020, HEMBA1001022, HEMBA1001024, HEMBA1001026, HEMBA1001051, HEMBA1001060, HEMBA1001080, HEMBA1001094, HEMBA1001099, HEMBA1001109, HEMBA1001121, HEMBA1001122, HEMBA1001123, HEMBA1001133, HEMBA1001140, HEMBA1001208, HEMBA1001213, HEMBA1001226, HEMBA1001235, HEMBA1001281, HEMBA1001299, HEMBA1001303, HEMBA1001310, HEMBA1001319, HEMBA1001323, HEMBA1001326, HEMBA1001327, HEMBA1001330, HEMBA1001361, HEMBA1001375, HEMBA1001377, HEMBA1001383, HEMBA1001388, HEMBA1001391, HEMBA1001398, HEMBA1001411, HEMBA1001413, HEMBA1001415, HEMBA1001432, HEMBA1001433, HEMBA1001435, HEMBA1001442, HEMBA1001450, HEMBA1001463, HEMBA1001497, HEMBA1001522, HEMBA1001533, HEMBA1001566, HEMBA1001570, HEMBA1001581, HEMBA1001589, HEMBA1001608, HEMBA1001636, HEMBA1001640, HEMBA1001647, HEMBA1001655, HEMBA1001658, HEMBA1001702, HEMBA1001711, HEMBA1001712, HEMBA1001731, HEMBA1001745, HEMBA1001750, HEMBA1001781, HEMBA1001784, HEMBA1001791, HEMBA1001803, HEMBA1001815, HEMBA1001820, HEMBA1001835, HEMBA1001864, HEMBA1001888, HEMBA1001910, HEMBA1001912, HEMBA1001915, HEMBA1001918, HEMBA1001939, HEMBA1001940, HEMBA1001942, HEMBA1001950, HEMBA1001964, HEMBA1001987, HEMBA1002018, HEMBA1002022, HEMBA1002039, HEMBA1002049, HEMBA1002084, HEMBA1002100, HEMBA1002113, HEMBA1002119, HEMBA1002160, HEMBA1002162, HEMBA1002185, HEMBA1002189, HEMBA1002191, HEMBA1002199, HEMBA1002204, HEMBA1002229, HEMBA1002237, HEMBA1002265, HEMBA1002328, HEMBA1002337, HEMBA1002348, HEMBA1002349, HEMBA1002381, HEMBA1002430, HEMBA1002439, HEMBA1002460, HEMBA1002498, HEMBA1002503, HEMBA1002508, HEMBA1002515, HEMBA1002538, HEMBA1002542, HEMBA1002552, HEMBA1002558, HEMBA1002583, HEMBA1002621, HEMBA1002624, HEMBA1002629, HEMBA1002645, HEMBA1002666, HEMBA1002678, HEMBA1002679, HEMBA1002688, HEMBA1002696, HEMBA1002703, HEMBA1002712, HEMBA1002716, HEMBA1002742, HEMBA1002748, HEMBA1002750, HEMBA1002779, HEMBA1002794, HEMBA1002801, HEMBA1002816, HEMBA1002826, HEMBA1002833, HEMBA1002850, HEMBA1002863, HEMBA1002934, HEMBA1002937, HEMBA1002944, HEMBA1002954, HEMBA1002968, HEMBA1002970, HEMBA1002971, HEMBA1003021, HEMBA1003033, HEMBA1003034, HEMBA1003035, HEMBA1003037, HEMBA1003041, HEMBA1003067, HEMBA1003078, HEMBA1003083, HEMBA1003117, HEMBA1003129, HEMBA1003133, HEMBA1003142, HEMBA1003166, HEMBA1003175, HEMBA1003197, HEMBA1003202, HEMBA1003220, HEMBA1003222, HEMBA1003229, HEMBA1003273, HEMBA1003276, HEMBA1003278, HEMBA1003304, HEMBA1003309, HEMBA1003322, HEMBA1003327, HEMBA1003328, HEMBA1003370, HEMBA1003373, HEMBA1003376, HEMBA1003380, HEMBA1003384, HEMBA1003395, HEMBA1003402, HEMBA1003447, HEMBA1003461, HEMBA1003463, HEMBA1003480, HEMBA1003528, HEMBA1003531, HEMBA1003548, HEMBA1003556, HEMBA1003571, HEMBA1003579, HEMBA1003597, HEMBA1003598, HEMBA1003621, HEMBA1003646, HEMBA1003656, HEMBA1003667, HEMBA1003692, HEMBA1003720, HEMBA1003725, HEMBA1003729, HEMBA1003733, HEMBA1003758, HEMBA1003799, HEMBA1003804, HEMBA1003807, HEMBA1003827, HEMBA1003838, HEMBA1003854, HEMBA1003856, HEMBA1003864, HEMBA1003879, HEMBA1003880, HEMBA1003893, HEMBA1003908, HEMBA1003926, HEMBA1003937, HEMBA1003939, HEMBA1003942, HEMBA1003958, HEMBA1003959, HEMBA1003976, HEMBA1003978, HEMBA1003985, HEMBA1003987, HEMBA1003989, HEMBA1004011, HEMBA1004012, HEMBA1004015, HEMBA1004024, HEMBA1004038, HEMBA1004045, HEMBA1004048, HEMBA1004056, HEMBA1004074, HEMBA1004086, HEMBA1004111, HEMBA1004138, HEMBA1004150, HEMBA1004193, HEMBA1004200, HEMBA1004225, HEMBA1004238, HEMBA1004241, HEMBA1004246, HEMBA1004267, HEMBA1004272, HEMBA1004274, HEMBA1004289, HEMBA1004312, HEMBA1004323, HEMBA1004327, HEMBA1004330, HEMBA1004335, HEMBA1004366, HEMBA1004372, HEMBA1004394, HEMBA1004396, HEMBA1004405, HEMBA1004429, HEMBA1004433, HEMBA1004460, HEMBA1004502, HEMBA1004507, HEMBA1004538, HEMBA1004542, HEMBA1004554, HEMBA1004560, HEMBA1004610, HEMBA1004617, HEMBA1004629, HEMBA1004631, HEMBA1004632, HEMBA1004638, HEMBA1004670, HEMBA1004672, HEMBA1004693, HEMBA1004705, HEMBA1004709, HEMBA1004711, HEMBA1004725, HEMBA1004730, HEMBA1004733, HEMBA1004736, HEMBA1004748, HEMBA1004751, HEMBA1004753, HEMBA1004763, HEMBA1004771, HEMBA1004776, HEMBA1004778, HEMBA1004803, HEMBA1004806, HEMBA1004807, HEMBA1004820, HEMBA1004860, HEMBA1004863, HEMBA1004865, HEMBA1004880, HEMBA1004900, HEMBA1004909, HEMBA1004933, HEMBA1004934, HEMBA1004944, HEMBA1004954, HEMBA1004960, HEMBA1004977, HEMBA1004978, HEMBA1004980, HEMBA1004983, HEMBA1004995, HEMBA1005019, HEMBA1005039, HEMBA1005062, HEMBA1005066, HEMBA1005075, HEMBA1005079, HEMBA1005083, HEMBA1005113, HEMBA1005123, HEMBA1005133, HEMBA1005149, HEMBA1005152, HEMBA1005185, HEMBA1005223, HEMBA1005232, HEMBA1005241, HEMBA1005252, HEMBA1005275, HEMBA1005293, HEMBA1005296, HEMBA1005311, HEMBA1005314, HEMBA1005331, HEMBA1005374, HEMBA1005382, HEMBA1005403, HEMBA1005411, HEMBA1005426, HEMBA1005443, HEMBA1005447, HEMBA1005468, HEMBA1005469, HEMBA1005472, HEMBA1005474, HEMBA1005475, HEMBA1005497, HEMBA1005500, HEMBA1005506, HEMBA1005508, HEMBA1005517, HEMBA1005518, HEMBA1005526, HEMBA1005568, HEMBA1005572, HEMBA1005583, HEMBA1005593, HEMBA1005606, HEMBA1005609, HEMBA1005627, HEMBA1005670, HEMBA1005679, HEMBA1005680, HEMBA1005685, HEMBA1005705, HEMBA1005746, HEMBA1005755, HEMBA1005780, HEMBA1005813, HEMBA1005822, HEMBA1005834, HEMBA1005852, HEMBA1005884, HEMBA1005891, HEMBA1005894, HEMBA1005909, HEMBA1005911, HEMBA1005921, HEMBA1005963, HEMBA1006005, HEMBA1006035, HEMBA1006081, HEMBA1006090, HEMBA1006091, HEMBA1006092, HEMBA1006100, HEMBA1006108, HEMBA1006121, HEMBA1006124, HEMBA1006138, HEMBA1006155, HEMBA1006182, HEMBA1006235, HEMBA1006252, HEMBA1006259, HEMBA1006349, HEMBA1006364, HEMBA1006377, HEMBA1006380, HEMBA1006381, HEMBA1006406, HEMBA1006416, HEMBA1006421, HEMBA1006424, HEMBA1006426, HEMBA1006446, HEMBA1006461, HEMBA1006467, HEMBA1006483, HEMBA1006486, HEMBA1006494, HEMBA1006497, HEMBA1006530, HEMBA1006535, HEMBA1006546, HEMBA1006562, HEMBA1006566, HEMBA1006579, HEMBA1006595, HEMBA1006597, HEMBA1006612, HEMBA1006617, HEMBA1006631, HEMBA1006639, HEMBA1006643, HEMBA1006665, HEMBA1006674, HEMBA1006676, HEMBA1006682, HEMBA1006695, HEMBA1006709, HEMBA1006717, HEMBA1006744, HEMBA1006754, HEMBA1006767, HEMBA1006779, HEMBA1006780, HEMBA1006795, HEMBA1006796, HEMBA1006812, HEMBA1006824, HEMBA1006832, HEMBA1006865, HEMBA1006900, HEMBA1006936, HEMBA1006949, HEMBA1006993, HEMBA1007002, HEMBA1007051, HEMBA1007052, HEMBA1007062, HEMBA1007066, HEMBA1007073, HEMBA1007078, HEMBA1007080, HEMBA1007085, HEMBA1007112, HEMBA1007113, HEMBA1007129, HEMBA1007147, HEMBA1007178, HEMBA1007206, HEMBA1007256, HEMBA1007267, HEMBA1007281, HEMBA1007288, HEMBA1007319, HEMBA1007320, HEMBA1007322, HEMBA1007327, HEMBA1007341, HEMBA1007342, HEMBA1007347, HEMBB1000005, HEMBB1000008, HEMBB1000018, HEMBB1000024, HEMBB1000025, HEMBB1000030, HEMBB1000048, HEMBB1000059, HEMBB1000089, HEMBB1000099, HEMBB1000103, HEMBB1000113, HEMBB1000136, HEMBB1000141, HEMBB1000173, HEMBB1000198, HEMBB1000215, HEMBB1000218, HEMBB1000240, HEMBB1000244, HEMBB1000272, HEMBB1000274, HEMBB1000312, HEMBB1000318, HEMBB1000335, HEMBB1000336, HEMBB1000337, HEMBB1000338, HEMBB1000339, HEMBB1000341, HEMBB1000343, HEMBB1000354, HEMBB1000374, HEMBB1000391, HEMBB1000402, HEMBB1000420, HEMBB1000434, HEMBB1000438, HEMBB1000441, HEMBB1000449, HEMBB1000480, HEMBB1000491, HEMBB1000493, HEMBB1000510, HEMBB1000530, HEMBB1000550, HEMBB1000554, HEMBB1000573, HEMBB1000586, HEMBB1000589, HEMBB1000591, HEMBB1000592, HEMBB1000623, HEMBB1000630, HEMBB1000637, HEMBB1000649, HEMBB1000652, HEMBB1000671, HEMBB1000672, HEMBB1000673, HEMBB1000684, HEMBB1000705, HEMBB1000706, HEMBB1000709, HEMBB1000726, HEMBB1000749, HEMBB1000770, HEMBB1000774, HEMBB1000790, HEMBB1000807, HEMBB1000810, HEMBB1000822, HEMBB1000826, HEMBB1000835, HEMBB1000848, HEMBB1000852, HEMBB1000870, HEMBB1000883, HEMBB1000887, HEMBB1000888, HEMBB1000890, HEMBB1000893, HEMBB1000908, HEMBB1000913, HEMBB1000917, HEMBB1000991, HEMBB1000996, HEMBB1001004, HEMBB1001008, HEMBB1001014, HEMBB1001020, HEMBB1001024, HEMBB1001047, HEMBB1001051, HEMBB1001060, HEMBB1001096, HEMBB1001105, HEMBB1001114, HEMBB1001117, HEMBB1001119, HEMBB1001126, HEMBB1001133, HEMBB1001142, HEMBB1001153, HEMBB1001169, HEMBB1001182, HEMBB1001197, HEMBB1001199, HEMBB1001208, HEMBB1001209, HEMBB1001210, HEMBB1001221, HEMBB1001249, HEMBB1001253, HEMBB1001254, HEMBB1001271, HEMBB1001289, HEMBB1001304, HEMBB1001315, HEMBB1001317, HEMBB1001335, HEMBB1001337, HEMBB1001356, HEMBB1001364, HEMBB1001366, HEMBB1001367, HEMBB1001369, HEMBB1001387, HEMBB1001394, HEMBB1001410, HEMBB1001424, HEMBB1001426, HEMBB1001436, HEMBB1001449, HEMBB1001458, HEMBB1001500, HEMBB1001521, HEMBB1001527, HEMBB1001531, HEMBB1001535, HEMBB1001536, HEMBB1001537, HEMBB1001565, HEMBB1001585, HEMBB1001588, HEMBB1001603, HEMBB1001618, HEMBB1001619, HEMBB1001635, HEMBB1001653, HEMBB1001665, HEMBB1001668, HEMBB1001684, HEMBB1001685, HEMBB1001695, HEMBB1001707, HEMBB1001735, HEMBB1001747, HEMBB1001753, HEMBB1001756, HEMBB1001760, HEMBB1001785, HEMBB1001797, HEMBB1001812, HEMBB1001816, HEMBB1001834, HEMBB1001850, HEMBB1001863, HEMBB1001868, HEMBB1001869, HEMBB1001874, HEMBB1001880, HEMBB1001899, HEMBB1001906, HEMBB1001910, HEMBB1001911, HEMBB1001921, HEMBB1001922, HEMBB1001930, HEMBB1001944, HEMBB1001945, HEMBB1001947, HEMBB1001952, HEMBB1001957, HEMBB1001967, HEMBB1001983, HEMBB1001990, HEMBB1001996, HEMBB1002002, HEMBB1002005, HEMBB1002043, HEMBB1002045, HEMBB1002049, HEMBB1002050, HEMBB1002068, HEMBB1002092, HEMBB1002139, HEMBB1002152, HEMBB1002218, HEMBB1002232, HEMBB1002249, HEMBB1002280, HEMBB1002300, HEMBB1002327, HEMBB1002329, HEMBB1002358, HEMBB1002359, HEMBB1002371, HEMBB1002381, HEMBB1002409, HEMBB1002415, HEMBB1002425, HEMBB1002453, HEMBB1002457, HEMBB1002458, HEMBB1002489, HEMBB1002492, HEMBB1002495, HEMBB1002502, HEMBB1002520, HEMBB1002522, HEMBB1002534, HEMBB1002545, HEMBB1002579, HEMBB1002582, HEMBB1002596, HEMBB1002603, HEMBB1002610, HEMBB1002613, HEMBB1002617, HEMBB1002623, HEMBB1002635, HEMBB1002677, HEMBB1002683, HEMBB1002684, HEMBB1002697, HEMBB1002699, HEMBB1002702, MAMMA1000009, MAMMA1000019, MAMMA1000025, MAMMA1000043, MAMMA1000057, MAMMA1000069, MAMMA1000084,
MAMMA1000092,
MAMMA1000103, MAMMA1000117, MAMMA1000139, MAMMA1000143, MAMMA1000155, MAMMA1000163, MAMMA1000171, MAMMA1000175, MAMMA1000198, MAMMA1000227, MAMMA1000241, MAMMA1000251, MAMMA1000254, MAMMA1000257, MAMMA1000264, MAMMA1000266, MAMMA1000270, MAMMA1000279, MAMMA1000287, MAMMA1000307, MAMMA1000309, MAMMA1000312, MAMMA1000313, MAMMA1000331, MAMMA1000339, MAMMA1000340, MAMMA1000348, MAMMA1000356, MAMMA1000360, MAMMA1000361, MAMMA1000372, MAMMA1000395, MAMMA1000414, MAMMA1000421, MAMMA1000422, MAMMA1000431, MAMMA1000444, MAMMA1000458, MAMMA1000468, MAMMA1000490, MAMMA1000500, MAMMA1000522, MAMMA1000524, MAMMA1000559, MAMMA1000567, MAMMA1000576, MAMMA1000583, MAMMA1000594, MAMMA1000605, MAMMA1000616, MAMMA1000623, MAMMA1000643, MAMMA1000664, MAMMA1000670, MAMMA1000696, MAMMA1000707, MAMMA1000720, MAMMA1000744, MAMMA1000746, MAMMA1000752, MAMMA1000760, MAMMA1000761, MAMMA1000775, MAMMA1000776, MAMMA1000802, MAMMA1000831, MAMMA1000839, MAMMA1000843, MAMMA1000851, MAMMA1000855, MAMMA1000856, MAMMA1000863, MAMMA1000865, MAMMA1000867, MAMMA1000875, MAMMA1000876, MAMMA1000880, MAMMA1000883, MAMMA1000906, MAMMA1000908, MAMMA1000914, MAMMA1000921, MAMMA1000931, MAMMA1000940, MAMMA1000941, MAMMA1000957, MAMMA1000962, MAMMA1000968, MAMMA1000975, MAMMA1000979, MAMMA1000987, MAMMA1001003, MAMMA1001021, MAMMA1001078, MAMMA1001082, MAMMA1001091, MAMMA1001110, MAMMA1001126, MAMMA1001143, MAMMA1001162, MAMMA1001186, MAMMA1001191, MAMMA1001202, MAMMA1001206, MAMMA1001215, MAMMA1001220, MAMMA1001243, MAMMA1001244, MAMMA1001249, MAMMA1001252, MAMMA1001256, MAMMA1001268, MAMMA1001271, MAMMA1001274, MAMMA1001292, MAMMA1001296, MAMMA1001324, MAMMA1001341, MAMMA1001343, MAMMA1001397, MAMMA1001408, MAMMA1001419, MAMMA1001420, MAMMA1001442, MAMMA1001452, MAMMA1001465, MAMMA1001502, MAMMA1001510, MAMMA1001522, MAMMA1001547, MAMMA1001551, MAMMA1001575, MAMMA1001590, MAMMA1001600, MAMMA1001604, MAMMA1001606, MAMMA1001620, MAMMA1001630, MAMMA1001635, MAMMA1001649, MAMMA1001663, MAMMA1001670, MAMMA1001671, MAMMA1001683, MAMMA1001686, MAMMA1001692, MAMMA1001711, MAMMA1001715, MAMMA1001744, MAMMA1001745, MAMMA1001757, MAMMA1001760, MAMMA1001764, MAMMA1001769, MAMMA1001783, MAMMA1001785, MAMMA1001788, MAMMA1001790, MAMMA1001806, MAMMA1001812, MAMMA1001815, MAMMA1001817, MAMMA1001818, MAMMA1001824, MAMMA1001848, MAMMA1001851, MAMMA1001854, MAMMA1001858, MAMMA1001864, MAMMA1001874, MAMMA1001878, MAMMA1001890, MAMMA1001907, MAMMA1001908, MAMMA1001931, MAMMA1001956, MAMMA1001969, MAMMA1001970, MAMMA1002011, MAMMA1002032, MAMMA1002033, MAMMA1002041, MAMMA1002042, MAMMA1002047, MAMMA1002056, MAMMA1002058, MAMMA1002068, MAMMA1002078, MAMMA1002082, MAMMA1002084, MAMMA1002093, MAMMA1002094, MAMMA1002118, MAMMA1002125, MAMMA1002132, MAMMA1002140, MAMMA1002145, MAMMA1002153, MAMMA1002155, MAMMA1002156, MAMMA1002174, MAMMA1002209, MAMMA1002230, MAMMA1002243, MAMMA1002250, MAMMA1002269, MAMMA1002282, MAMMA1002292, MAMMA1002293, MAMMA1002294, MAMMA1002298, MAMMA1002299, MAMMA1002308, MAMMA1002310, MAMMA1002311, MAMMA1002312, MAMMA1002317, MAMMA1002319, MAMMA1002322, MAMMA1002333, MAMMA1002339, MAMMA1002352, MAMMA1002353, MAMMA1002355, MAMMA1002356, MAMMA1002359, MAMMA1002360, MAMMA1002362, MAMMA1002380, MAMMA1002384, MAMMA1002392, MAMMA1002411, MAMMA1002413, MAMMA1002417, MAMMA1002434, MAMMA1002446, MAMMA1002454, MAMMA1002475, MAMMA1002494, MAMMA1002545, MAMMA1002554, MAMMA1002556, MAMMA1002566, MAMMA1002571, MAMMA1002585, MAMMA1002590, MAMMA1002597, MAMMA1002612, MAMMA1002618, MAMMA1002636, MAMMA1002646, MAMMA1002665, MAMMA1002673, MAMMA1002708, MAMMA1002711, MAMMA1002727, MAMMA1002728, MAMMA1002744, MAMMA1002748, MAMMA1002758, MAMMA1002764, MAMMA1002765, MAMMA1002775, MAMMA1002780, MAMMA1002782, MAMMA1002796, MAMMA1002807, MAMMA1002820, MAMMA1002830, MAMMA1002833, MAMMA1002838, MAMMA1002843, MAMMA1002868, MAMMA1002880, MAMMA1002886, MAMMA1002892, MAMMA1002895, MAMMA1002909, MAMMA1002941, MAMMA1002964, MAMMA1002973, MAMMA1002987, MAMMA1003003, MAMMA1003004, MAMMA1003015, MAMMA1003019, MAMMA1003026, MAMMA1003031, MAMMA1003039, MAMMA1003044, MAMMA1003049, MAMMA1003056, MAMMA1003066, MAMMA1003099, MAMMA1003104, MAMMA1003135, NT2RM1000018, NT2RM1000032, NT2RM1000059, NT2RM1000119, NT2RM1000127, NT2RM1000242, NT2RM1000271, NT2RM1000272, NT2RM1000300, NT2RM1000314, NT2RM1000341, NT2RM1000365, NT2RM1000399, NT2RM1000669, NT2RM1000672, NT2RM1000699, NT2RM1000741, NT2RM1000780, NT2RM1000781, NT2RM1000802, NT2RM1000829, NT2RM1000927, NT2RM1000962, NT2RM1000978, NT2RM1001043, NT2RM1001044, NT2RM1001066, NT2RM1001074, NT2RM1001082, NT2RM1001085, NT2RM1001105, NT2RM1001112, NT2RM2000006, NT2RM2000032, NT2RM2000042, NT2RM2000093, NT2RM2000192, NT2RM2000239, NT2RM2000250, NT2RM2000359, NT2RM2000374, NT2RM2000420, NT2RM2000540, NT2RM2000567, NT2RM2000569, NT2RM2000635, NT2RM2000636, NT2RM2000639, NT2RM2000649, NT2RM2000669, NT2RM2000795, NT2RM2000837, NT2RM2000952, NT2RM2000984, NT2RM2001004, NT2RM2001131, NT2RM2001141, NT2RM2001152, NT2RM2001177, NT2RM2001194, NT2RM2001243, NT2RM2001247, NT2RM2001291, NT2RM2001306, NT2RM2001312, NT2RM2001319, NT2RM2001360, NT2RM2001420, NT2RM2001504, NT2RM2001524, NT2RM2001544, NT2RM2001582, NT2RM2001588, NT2RM2001637, NT2RM2001641, NT2RM2001675, NT2RM2001681, NT2RM2001695, NT2RM2001696, NT2RM2001699, NT2RM2001706, NT2RM2001723, NT2RM2001727, NT2RM2001768, NT2RM2001784, NT2RM2001805, NT2RM2001840, NT2RM2001855, NT2RM2001867, NT2RM2001879, NT2RM2001903, NT2RM2001982, NT2RM2002178, NT2RM4000027, NT2RM4000061, NT2RM4000086, NT2RM4000197, NT2RM4000199, NT2RM4000200, NT2RM4000210, NT2RM4000244, NT2RM4000251, NT2RM4000265, NT2RM4000324, NT2RM4000327, NT2RM4000349, NT2RM4000395, NT2RM4000414, NT2RM4000425, NT2RM4000511, NT2RM4000514, NT2RM4000532, NT2RM4000534, NT2RM4000585, NT2RM4000603, NT2RM4000689, NT2RM4000698, NT2RM4000717, NT2RM4000783, NT2RM4000787, NT2RM4000790, NT2RM4000796, NT2RM4000813, NT2RM4000833, NT2RM4000848, NT2RM4000852, NT2RM4000855, NT2RM4000887, NT2RM4000895, NT2RM4000950, NT2RM4000971, NT2RM4000979, NT2RM4001002, NT2RM4001151, NT2RM4001160, NT2RM4001187, NT2RM4001191, NT2RM4001204, NT2RM4001258, NT2RM4001309, NT2RM4001384, NT2RM4001410, NT2RM4001414, NT2RM4001437, NT2RM4001489, NT2RM4001522, NT2RM4001557, NT2RM4001565, NT2RM4001569, NT2RM4001594, NT2RM4001650, NT2RM4001662, NT2RM4001682, NT2RM4001710, NT2RM4001715, NT2RM4001746, NT2RM4001754, NT2RM4001836, NT2RM4001841, NT2RM4001842, NT2RM4001856, NT2RM4001905, NT2RM4001922, NT2RM4001938, NT2RM4001953, NT2RM4001965, NT2RM4001984, NT2RM4002018, NT2RM4002044, NT2RM4002128, NT2RM4002140, NT2RM4002256, NT2RM4002266, NT2RM4002281, NT2RM4002294, NT2RM4002301, NT2RM4002344, NT2RM4002373, NT2RM4002374, NT2RM4002383, NT2RM4002390, NT2RM4002398, NT2RM4002420, NT2RM4002457, NT2RM4002504, NT2RM4002534, NT2RM4002567, NT2RM4002593, NT2RP1000063, NT2RP1000112, NT2RP1000124, NT2RP1000170, NT2RP1000243, NT2RP1000259, NT2RP1000324, NT2RP1000357, NT2RP1000409, NT2RP1000416, NT2RP1000581, NT2RP1000688, NT2RP1000695, NT2RP1000721, NT2RP1000730, NT2RP1000796, NT2RP1000836, NT2RP1000846, NT2RP1000851, NT2RP1000916, NT2RP1000943, NT2RP1000944, NT2RP1000980, NT2RP1000988, NT2RP1001014, NT2RP1001173, NT2RP1001199, NT2RP1001248, NT2RP1001424, NT2RP1001432, NT2RP1001466, NT2RP1001475, NT2RP1001616, NT2RP2000001, NT2RP2000007, NT2RP2000027, NT2RP2000079, NT2RP2000088, NT2RP2000097, NT2RP2000098, NT2RP2000108, NT2RP2000198, NT2RP2000205, NT2RP2000208, NT2RP2000232, NT2RP2000233, NT2RP2000239, NT2RP2000274, NT2RP2000298, NT2RP2000327, NT2RP2000328, NT2RP2000337, NT2RP2000369, NT2RP2000412, NT2RP2000438, NT2RP2000498, NT2RP2000503, NT2RP2000510, NT2RP2000551, NT2RP2000603, NT2RP2000617, NT2RP2000634, NT2RP2000644, NT2RP2000656, NT2RP2000658, NT2RP2000678, NT2RP2000704, NT2RP2000715, NT2RP2000758, NT2RP2000819, NT2RP2000841, NT2RP2000845, NT2RP2000863, NT2RP2000938, NT2RP2000970, NT2RP2000985, NT2RP2001036, NT2RP2001044, NT2RP2001056, NT2RP2001065, NT2RP2001094, NT2RP2001119, NT2RP2001137, NT2RP2001149, NT2RP2001173, NT2RP2001196, NT2RP2001214, NT2RP2001218, NT2RP2001226, NT2RP2001312, NT2RP2001328, NT2RP2001347, NT2RP2001381, NT2RP2001427, NT2RP2001450, NT2RP2001467, NT2RP2001506, NT2RP2001628, NT2RP2001675, NT2RP2001677, NT2RP2001678, NT2RP2001720, NT2RP2001721, NT2RP2001813, NT2RP2001861, NT2RP2001907, NT2RP2001936, NT2RP2001943, NT2RP2001946, NT2RP2001969, NT2RP2002032, NT2RP2002033, NT2RP2002041, NT2RP2002047, NT2RP2002056, NT2RP2002076, NT2RP2002154, NT2RP2002172, NT2RP2002219, NT2RP2002231, NT2RP2002235, NT2RP2002292, NT2RP2002316, NT2RP2002333, NT2RP2002373, NT2RP2002426, NT2RP2002439, NT2RP2002475, NT2RP2002498, NT2RP2002546, NT2RP2002549, NT2RP2002621, NT2RP2002672, NT2RP2002677, NT2RP2002706, NT2RP2002736, NT2RP2002752, NT2RP2002753, NT2RP2002755, NT2RP2002769, NT2RP2002800, NT2RP2002843, NT2RP2002891, NT2RP2002954, NT2RP2002979, NT2RP2003034, NT2RP2003073, NT2RP2003099, NT2RP2003101, NT2RP2003108, NT2RP2003117, NT2RP2003161, NT2RP2003165, NT2RP2003194, NT2RP2003206, NT2RP2003237, NT2RP2003280, NT2RP2003293, NT2RP2003339, NT2RP2003367, NT2RP2003393, NT2RP2003445, NT2RP2003456, NT2RP2003511, NT2RP2003533, NT2RP2003559, NT2RP2003567, NT2RP2003581, NT2RP2003687, NT2RP2003691, NT2RP2003727, NT2RP2003751, NT2RP2003764, NT2RP2003769, NT2RP2003793, NT2RP2003799, NT2RP2003825, NT2RP2003885, NT2RP2003976, NT2RP2003984, NT2RP2003986, NT2RP2003988, NT2RP2004042, NT2RP2004081, NT2RP2004095, NT2RP2004124, NT2RP2004152, NT2RP2004165, NT2RP2004207, NT2RP2004226, NT2RP2004316, NT2RP2004321, NT2RP2004339, NT2RP2004347, NT2RP2004364, NT2RP2004365, NT2RP2004373, NT2RP2004399, NT2RP2004400, NT2RP2004412, NT2RP2004463, NT2RP2004490, NT2RP2004523, NT2RP2004551, NT2RP2004580, NT2RP2004594, NT2RP2004600, NT2RP2004602, NT2RP2004614, NT2RP2004664, NT2RP2004675, NT2RP2004709, NT2RP2004743, NT2RP2004767, NT2RP2004802, NT2RP2004841, NT2RP2004861, NT2RP2004897, NT2RP2004962, NT2RP2004982, NT2RP2004999, NT2RP2005000, NT2RP2005001, NT2RP2005018, NT2RP2005020, NT2RP2005022, NT2RP2005031, NT2RP2005147, NT2RP2005254, NT2RP2005287, NT2RP2005453, NT2RP2005454, NT2RP2005464, NT2RP2005472, NT2RP2005495, NT2RP2005501, NT2RP2005540, NT2RP2005555, NT2RP2005600, NT2RP2005622, NT2RP2005637, NT2RP2005640, NT2RP2005645, NT2RP2005683, NT2RP2005690, NT2RP2005726, NT2RP2005732, NT2RP2005741, NT2RP2005748, NT2RP2005804, NT2RP2005806, NT2RP2005815, NT2RP2005853, NT2RP2005859, NT2RP2005868, NT2RP2005882, NT2RP2005886, NT2RP2005890, NT2RP2005901, NT2RP2005980, NT2RP2006023, NT2RP2006038, NT2RP2006052, NT2RP2006103, NT2RP2006106, NT2RP2006166, NT2RP2006186, NT2RP2006196, NT2RP2006200, NT2RP2006237, NT2RP2006258, NT2RP2006321, NT2RP2006333, NT2RP2006334, NT2RP2006365, NT2RP2006393, NT2RP2006441, NT2RP2006454, NT2RP2006467, NT2RP2006472, NT2RP2006554, NT2RP3000002, NT2RP3000055, NT2RP3000072, NT2RP3000080, NT2RP3000142, NT2RP3000149, NT2RP3000197, NT2RP3000220, NT2RP3000235, NT2RP3000247, NT2RP3000251, NT2RP3000267, NT2RP3000312, NT2RP3000324, NT2RP3000348, NT2RP3000418, NT2RP3000449, NT2RP3000451, NT2RP3000456, NT2RP3000484, NT2RP3000526, NT2RP3000542, NT2RP3000561, NT2RP3000584, NT2RP3000592, NT2RP3000599, NT2RP3000622, NT2RP3000628, NT2RP3000644, NT2RP3000661, NT2RP3000665, NT2RP3000690, NT2RP3000736, NT2RP3000836, NT2RP3000841, NT2RP3000850, NT2RP3000859, NT2RP3000865, NT2RP3000901, NT2RP3000980, NT2RP3001004, NT2RP3001007, NT2RP3001109, NT2RP3001115, NT2RP3001116, NT2RP3001119, NT2RP3001133, NT2RP3001232, NT2RP3001236, NT2RP3001245, NT2RP3001274, NT2RP3001281, NT2RP3001297, NT2RP3001318, NT2RP3001325, NT2RP3001356, NT2RP3001374, NT2RP3001392, NT2RP3001396, NT2RP3001420, NT2RP3001432, NT2RP3001447, NT2RP3001449, NT2RP3001459, NT2RP3001607, NT2RP3001608, NT2RP3001621, NT2RP3001629, NT2RP3001676, NT2RP3001678, NT2RP3001698, NT2RP3001752, NT2RP3001777, NT2RP3001844, NT2RP3001896, NT2RP3001915, NT2RP3001929, NT2RP3002033, NT2RP3002063, NT2RP3002097, NT2RP3002099, NT2RP3002102, NT2RP3002142, NT2RP3002146, NT2RP3002147, NT2RP3002166, NT2RP3002173, NT2RP3002181, NT2RP3002244, NT2RP3002248, NT2RP3002255, NT2RP3002276, NT2RP3002304, NT2RP3002343, NT2RP3002402, NT2RP3002484, NT2RP3002512, NT2RP3002545, NT2RP3002566, NT2RP3002587, NT2RP3002590, NT2RP3002603, NT2RP3002659, NT2RP3002660, NT2RP3002713, NT2RP3002763, NT2RP3002799, NT2RP3002861, NT2RP3002877, NT2RP3002911, NT2RP3002955, NT2RP3003121, NT2RP3003139, NT2RP3003150, NT2RP3003155, NT2RP3003157, NT2RP3003204, NT2RP3003210, NT2RP3003264, NT2RP3003344, NT2RP3003346, NT2RP3003377, NT2RP3003403, NT2RP3003427, NT2RP3003433, NT2RP3003543, NT2RP3003552, NT2RP3003564, NT2RP3003572, NT2RP3003625, NT2RP3003656, NT2RP3003680, NT2RP3003686, NT2RP3003795, NT2RP3003805, NT2RP3003819, NT2RP3003833, NT2RP3003842, NT2RP3003870, NT2RP3003932, NT2RP3003989, NT2RP3004028, NT2RP3004041, NT2RP3004070, NT2RP3004093, NT2RP3004095, NT2RP3004145, NT2RP3004215, NT2RP3004246, NT2RP3004253, NT2RP3004332, NT2RP3004341, NT2RP3004349, NT2RP3004451, NT2RP3004466, NT2RP3004470, NT2RP3004539, NT2RP4000023, NT2RP4000035, NT2RP4000049, NT2RP4000102, NT2RP4000150, NT2RP4000159, NT2RP4000167, NT2RP4000185, NT2RP4000218, NT2RP4000355, NT2RP4000360, NT2RP4000381, NT2RP4000424, NT2RP4000448, NT2RP4000480, NT2RP4000515, NT2RP4000517, NT2RP4000519, NT2RP4000541, NT2RP4000638, NT2RP4000704, NT2RP4000728, NT2RP4000739, NT2RP4000955, NT2RP4000975, NT2RP4000984, NT2RP4001006, NT2RP4001100, NT2RP4001210, NT2RP4001343, NT2RP4001353, NT2RP4001442, NT2RP4001447, NT2RP4001502, NT2RP4001507, NT2RP4001524, NT2RP4001610, NT2RP4001614, NT2RP4001679, NT2RP4001828, NT2RP4001841, NT2RP4001901, NT2RP4001953, NT2RP4002071, NT2RP4002083, NT2RP4002298, NT2RP4002888, NT2RP5003492, NT2RP5003512, NT2RP5003524, OVARC1000008, OVARC1000017, OVARC1000035, OVARC1000058, OVARC1000068, OVARC1000092, OVARC1000109, OVARC1000113, OVARC1000114, OVARC1000145, OVARC1000168, OVARC1000198, OVARC1000212, OVARC1000240, OVARC1000302, OVARC1000321, OVARC1000347, OVARC1000384, OVARC1000408, OVARC1000411, OVARC1000414, OVARC1000420, OVARC1000440, OVARC1000442, OVARC1000443, OVARC1000461, OVARC1000466, OVARC1000496, OVARC1000533, OVARC1000557, OVARC1000576, OVARC1000578, OVARC1000588, OVARC1000605, OVARC1000622, OVARC1000640, OVARC1000661, OVARC1000678, OVARC1000681, OVARC1000703, OVARC1000724, OVARC1000730, OVARC1000781, OVARC1000787, OVARC1000802, OVARC1000883, OVARC1000886, OVARC1000891, OVARC1000912, OVARC1000960, OVARC1000964, OVARC1000971, OVARC1001000, OVARC1001004, OVARC1001010, OVARC1001011, OVARC1001029, OVARC1001032, OVARC1001040, OVARC1001044, OVARC1001072, OVARC1001074, OVARC1001118, OVARC1001161, OVARC1001162, OVARC1001167, OVARC1001169, OVARC1001170, OVARC1001173, OVARC1001176, OVARC1001188, OVARC1001240, OVARC1001243, OVARC1001261, OVARC1001270, OVARC1001296, OVARC1001339, OVARC1001341, OVARC1001344, OVARC1001357, OVARC1001360, OVARC1001369, OVARC1001376, OVARC1001399, OVARC1001425, OVARC1001442, OVARC1001480, OVARC1001489, OVARC1001525, OVARC1001600, OVARC1001611, OVARC1001768, OVARC1001791, OVARC1001795, OVARC1001802, OVARC1001813, OVARC1001828, OVARC1001846, OVARC1001861, OVARC1001873, OVARC1001879, OVARC1001880, OVARC1001883, OVARC1001916, OVARC1001928, OVARC1001950, OVARC1002082, OVARC1002107, OVARC1002143, PLACE1000005, PLACE1000048, PLACE1000078, PLACE1000094, PLACE1000214, PLACE1000236, PLACE1000246, PLACE1000292, PLACE1000308, PLACE1000332, PLACE1000347, PLACE1000374, PLACE1000380, PLACE1000435, PLACE1000444, PLACE1000453, PLACE1000564, PLACE1000599, PLACE1000716, PLACE1000748, PLACE1000749, PLACE1000785, PLACE1000798, PLACE1000814, PLACE1000849, PLACE1000856, PLACE1000931, PLACE1001000, PLACE1001007, PLACE1001010, PLACE1001015, PLACE1001024, PLACE1001076, PLACE1001088, PLACE1001136, PLACE1001168, PLACE1001185, PLACE1001280, PLACE1001311, PLACE1001323, PLACE1001351, PLACE1001366, PLACE1001395, PLACE1001399, PLACE1001412, PLACE1001414, PLACE1001440, PLACE1001456, PLACE1001484, PLACE1001503, PLACE1001545, PLACE1001570, PLACE1001608, PLACE1001634, PLACE1001640, PLACE1001705, PLACE1001720, PLACE1001729, PLACE1001740, PLACE1001745, PLACE1001746, PLACE1001799, PLACE1001810, PLACE1001897, PLACE1001912, PLACE1001928, PLACE1002004, PLACE1002066, PLACE1002072, PLACE1002115, PLACE1002157, PLACE1002163, PLACE1002170, PLACE1002227, PLACE1002256, PLACE1002259, PLACE1002319, PLACE1002342, PLACE1002465, PLACE1002477, PLACE1002514, PLACE1002529, PLACE1002537, PLACE1002578, PLACE1002625, PLACE1002768, PLACE1002772, PLACE1002815, PLACE1002839, PLACE1002853, PLACE1002941, PLACE1002962, PLACE1002968, PLACE1002993, PLACE1002996, PLACE1003025, PLACE1003027, PLACE1003092, PLACE1003108, PLACE1003145, PLACE1003200, PLACE1003205, PLACE1003249, PLACE1003256, PLACE1003342, PLACE1003343, PLACE1003361, PLACE1003373, PLACE1003375, PLACE1003383, PLACE1003454, PLACE1003478, PLACE1003516, PLACE1003528, PLACE1003566, PLACE1003584, PLACE1003592, PLACE1003593, PLACE1003618, PLACE1003638, PLACE1003711, PLACE1003762, PLACE1003784, PLACE1003795, PLACE1003864, PLACE1003870, PLACE1003886, PLACE1003892, PLACE1003900, PLACE1003936, PLACE1004114, PLACE1004118, PLACE1004156, PLACE1004161, PLACE1004274, PLACE1004284, PLACE1004336, PLACE1004376, PLACE1004384, PLACE1004388, PLACE1004405, PLACE1004425, PLACE1004451, PLACE1004491, PLACE1004516, PLACE1004518, PLACE1004548, PLACE1004645, PLACE1004664, PLACE1004672, PLACE1004681, PLACE1004686, PLACE1004691, PLACE1004693, PLACE1004716, PLACE1004722, PLACE1004736, PLACE1004740, PLACE1004815, PLACE1004824, PLACE1004836, PLACE1004838, PLACE1004885, PLACE1004900, PLACE1004913, PLACE1004934, PLACE1004979, PLACE1004982, PLACE1004985, PLACE1005026, PLACE1005027, PLACE1005046, PLACE1005055, PLACE1005077, PLACE1005085, PLACE1005101, PLACE1005108, PLACE1005111, PLACE1005128, PLACE1005146, PLACE1005181, PLACE1005232, PLACE1005261, PLACE1005266, PLACE1005277, PLACE1005308, PLACE1005335, PLACE1005374, PLACE1005409, PLACE1005453, PLACE1005477, PLACE1005480, PLACE1005481, PLACE1005550, PLACE1005554, PLACE1005574, PLACE1005603, PLACE1005639, PLACE1005730, PLACE1005755, PLACE1005799, PLACE1005802, PLACE1005828, PLACE1005850, PLACE1005851, PLACE1005923, PLACE1005925, PLACE1005932, PLACE1005936, PLACE1005968, PLACE1005990, PLACE1006002, PLACE1006017, PLACE1006037, PLACE1006076, PLACE1006129, PLACE1006139, PLACE1006143, PLACE1006159, PLACE1006195, PLACE1006236, PLACE1006246, PLACE1006248, PLACE1006325, PLACE1006357, PLACE1006360, PLACE1006371, PLACE1006382, PLACE1006412, PLACE1006414, PLACE1006445, PLACE1006470, PLACE1006521, PLACE1006598, PLACE1006617, PLACE1006640, PLACE1006673, PLACE1006704, PLACE1006760, PLACE1006779, PLACE1006782, PLACE1006792, PLACE1006795, PLACE1006805, PLACE1006815, PLACE1006867, PLACE1006883, PLACE1006932, PLACE1006961, PLACE1006962, PLACE1006966, PLACE1007021, PLACE1007045, PLACE1007053, PLACE1007068, PLACE1007097, PLACE1007112, PLACE1007178, PLACE1007218, PLACE1007238, PLACE1007242, PLACE1007301, PLACE1007342, PLACE1007367, PLACE1007402, PLACE1007450, PLACE1007452, PLACE1007454, PLACE1007460, PLACE1007478, PLACE1007507, PLACE1007524, PLACE1007557, PLACE1007618, PLACE1007621, PLACE1007677, PLACE1007690, PLACE1007725, PLACE1007730, PLACE1007737, PLACE1007743, PLACE1007746, PLACE1007807, PLACE1007810, PLACE1007843, PLACE1007846, PLACE1007858, PLACE1007866, PLACE1007990, PLACE1008002, PLACE1008045, PLACE1008095, PLACE1008122, PLACE1008129, PLACE1008181, PLACE1008209, PLACE1008231, PLACE1008280, PLACE1008329, PLACE1008369, PLACE1008392, PLACE1008401, PLACE1008405, PLACE1008455, PLACE1008457, PLACE1008488, PLACE1008524, PLACE1008584, PLACE1008621, PLACE1008625, PLACE1008626, PLACE1008629, PLACE1008630, PLACE1008748, PLACE1008757, PLACE1008798, PLACE1008851, PLACE1008854, PLACE1008867, PLACE1008887, PLACE1008902, PLACE1008925, PLACE1008941, PLACE1008947, PLACE1009039, PLACE1009048, PLACE1009050, PLACE1009090, PLACE1009091, PLACE1009110, PLACE1009111, PLACE1009150, PLACE1009166, PLACE1009174, PLACE1009190, PLACE1009200, PLACE1009230, PLACE1009328, PLACE1009335, PLACE1009375, PLACE1009388, PLACE1009410, PLACE1009434, PLACE1009459, PLACE1009539, PLACE1009542, PLACE1009581, PLACE1009595, PLACE1009607, PLACE1009621, PLACE1009637, PLACE1009665, PLACE1009794, PLACE1009886, PLACE1009888, PLACE1009921, PLACE1009947, PLACE1009971, PLACE1009995, PLACE1010023, PLACE1010031, PLACE1010069, PLACE1010076, PLACE1010102, PLACE1010106, PLACE1010202, PLACE1010270, PLACE1010274, PLACE1010293, PLACE1010324, PLACE1010329, PLACE1010364, PLACE1010383, PLACE1010401, PLACE1010491, PLACE1010492, PLACE1010562, PLACE1010616, PLACE1010624, PLACE1010629, PLACE1010630, PLACE1010714, PLACE1010786, PLACE1010800, PLACE1010802, PLACE1010856, PLACE1010857, PLACE1010877, PLACE1010900, PLACE1010916, PLACE1010925, PLACE1010944, PLACE1010947, PLACE1010965, PLACE1011026, PLACE1011032, PLACE1011054, PLACE1011057, PLACE1011133, PLACE1011143, PLACE1011165, PLACE1011273, PLACE1011291, PLACE1011325, PLACE1011452, PLACE1011465, PLACE1011472, PLACE1011503, PLACE1011520, PLACE1011563, PLACE1011567, PLACE1011643, PLACE1011649, PLACE1011650, PLACE1011675, PLACE1011682, PLACE1011719, PLACE1011725, PLACE1011729, PLACE1011874, PLACE1011875, PLACE1011891, PLACE1011982, PLACE2000003, PLACE2000006, PLACE2000007, PLACE2000017, PLACE2000047, PLACE2000050, PLACE2000061, PLACE2000097, PLACE2000100, PLACE2000103, PLACE2000115, PLACE2000124, PLACE2000140, PLACE2000170, PLACE2000172, PLACE2000223, PLACE2000235, PLACE2000302, PLACE2000317, PLACE2000347, PLACE2000359, PLACE2000366, PLACE2000394, PLACE2000433, PLACE2000450, PLACE2000455, PLACE3000070, PLACE3000103, PLACE3000119, PLACE3000136, PLACE3000142, PLACE3000148, PLACE3000155, PLACE3000156, PLACE3000157, PLACE3000158, PLACE3000160, PLACE3000194, PLACE3000197, PLACE3000199, PLACE3000207, PLACE3000208, PLACE3000221, PLACE3000230, PLACE3000271, PLACE3000276, PLACE3000304, PLACE3000310, PLACE3000320, PLACE3000322, PLACE3000331, PLACE3000341, PLACE3000352, PLACE3000353, PLACE3000362, PLACE3000363, PLACE3000365, PLACE3000388, PLACE3000401, PLACE3000402, PLACE3000405, PLACE3000425, PLACE3000475, PLACE4000089, PLACE4000093, PLACE4000106, PLACE4000131, PLACE4000147, PLACE4000222, PLACE4000252, PLACE4000270, PLACE4000320, PLACE4000323, PLACE4000344, PLACE4000367, PLACE4000392, PLACE4000411, PLACE4000445, PLACE4000465, PLACE4000521, PLACE4000590, PLACE4000612, PLACE4000638, PLACE4000670, THYRO1000026, THYRO1000070, THYRO1000092, THYRO1000107, THYRO1000111, THYRO1000124, THYRO1000186, THYRO1000187, THYRO1000199, THYRO1000206, THYRO1000241, THYRO1000253, THYRO1000270, THYRO1000279, THYRO1000320, THYRO1000368, THYRO1000381, THYRO1000387, THYRO1000452, THYRO1000471, THYRO1000484, THYRO1000502, THYRO1000505, THYRO1000558, THYRO1000596, THYRO1000625, THYRO1000637, THYRO 1000676, THYRO1000712, THYRO1000715, THYRO1000734, THYRO1000777, THYRO1000787, THYRO1000793, THYRO1000796, THYRO1000805, THYRO1000815, THYRO1000843, THYRO1000855, THYRO1000865, THYRO1000895, THYRO1000916, THYRO1000952, THYRO1000988, THYRO1001031, THYRO1001062, THYRO1001133, THYRO1001142, THYRO1001173, THYRO1001213, THYRO1001262, THYRO1001321, THYRO1001322, THYRO1001363, THYRO1001365, THYRO1001403, THYRO1001411, THYRO1001426, THYRO1001434, THYRO1001480, THYRO1001487, THYRO1001559, THYRO1001570, THYRO1001584, THYRO1001595, THYRO1001602, THYRO1001605, THYRO1001637, THYRO1001673, THYRO1001706, THYRO1001745, THYRO1001746, THYRO1001772, THYRO1001793, THYRO1001854, THYRO1001895, THYRO1001907, VESEN1000122, Y79AA1000065, Y79AA1000131, Y79AA1000202, Y79AA1000230, Y79AA1000355, Y79AA1000410, Y79AA1000480, Y79AA1000539, Y79AA1000574, Y79AA1000774, Y79AA1000802, Y79AA1000805, Y79AA1000824, Y79AA1000827, Y79AA1000850, Y79AA1000969, Y79AA1001041, Y79AA1001061, Y79AA1001068, Y79AA1001077, Y79AA1001078, Y79AA1001145, Y79AA1001167, Y79AA1001185, Y79AA1001216, Y79AA1001228, Y79AA1001281, Y79AA1001511, Y79AA1001541, Y79AA1001555, Y79AA1001585, Y79AA1001665, Y79AA1001696, Y79AA1001781, Y79AA1001805, Y79AA1002089, Y79AA1002115, Y79AA1002125, Y79AA1002220, Y79AA1002234, Y79AA1002298, Y79AA1002407, MAMMA1002215, MAMMA1002721, NT2RP2002070,

### Homology Search Result Data 1.

The result of the homology search of the SwissProt using the 5'-end sequence.

Data include
the name of clone,
definition of the top hit data,
the P-value: the length of the compared sequence: identity (%), and
the organism and the Accession No. of the top hit data, as in the order separated by //.

Data are not shown for the clones in which the P-value was higher than 1.

The P-value is a score obtained statistically by taking into account the possible similarity between two sequences. In general, the smaller P-value reflects the higher similarity. (Altschul, S.F., Gish, W., Miller, W., Myers, E.W. & Lipman, D.J. (1990) "Basic local alignment search tool." J. Mol. Biol. 215:403-410; Gish, W. & States, D.J. (1993) "Identification of protein coding regions by database similarity search." Nature Genet. 3:266-272).
F-HEMBA1000005//DNAJ PROTEIN HOMOLOG MTJ1.//1.8e-85:244:75//MUS MUSCULUS (MOUSE).//Q61712
F-HEMBA1000012//PROBABLE LEUCYL-TRNA SYNTHETASE (EC 6.1.1.4) (LEUCINETRNA LIGASE) (LEURS).//7.6e-57:231:53//CAENORHABDITIS ELEGANS.//Q09996
F-HEMBA1000020//TUBULIN BETA CHAIN.//1.0e-92:143:80//AJELLOMYCES CAPSULATA (HISTOPLASMA CAPSULATUM).//P41742
F-HEMBA1000030//CIRCUMSPOROZOITE PROTEIN PRECURSOR (CS).//0.021:136:33//PLASMODIUM KNOWLESI (STRAIN NURI).//P04922
F-HEMBA1000042//METALLOTHIONEIN 10-II (MT-10-II).//0.71:64:32//MYTILUS EDULIS (BLUE MUSSEL).//P80247
F-HEMBA1000046//PROTEIN Q300.//0.92:40:37//MUS MUSCULUS (MOUSE).//Q02722
F-HEMBA1000050//COMPETENCE PROTEIN S.//0.50:28:35//BACILLUS SUBTILIS.//P80355
F-HEMBA1000076//ATP SYNTHASE E CHAIN, MITOCHONDRIAL (EC 3.6.1.34).//0.86:41:41//HOMO SAPIENS (HUMAN).//P56385
F-HEMBA1000111
F-HEMBA1000129//UVSW PROTEIN (DAR PROTEIN).//0.023:68:33//BACTERIOPHAGE T4.//P20703
F-HEMBA1000141//YSY6 PROTEIN.//0.90:29:37//SACCHAROMYCES CEREVISIAE (BAKER'S YEAST).//P38374
F-HEMBA1000150//!!!! ALU SUBFAMILY SP WARNING ENTRY !!!!//8.4e-16:47:70//HOMO SAPIENS (HUMAN).//P39193
F-HEMBA1000156//IMMEDIATE-EARLY PROTEIN.//8.1e-07:143:28//HERPESVIRUS SAIMIRI (STRAIN 11).//Q01042
F-HEMBA1000158//HYPOTHETICAL PROTEIN KIAA0192 (FRAGMENT).//7.9e-11:129:40//HOMO SAPIENS (HUMAN).//Q93074
F-HEMBA1000168//INSULIN RECEPTOR SUBSTRATE-2 (IRS-2) (4PS).//0.00055:86:36//MUS MUSCULUS (MOUSE).//P81122
F-HEMBA1000180//VPU PROTEIN (U ORF PROTEIN).//0.22:73:28//CHIMPANZEE IMMUNODEFICIENCY VIRUS (SIV(CPZ)) (CIV).//P17286
F-HEMBA1000185//RAS-1 PROTEIN.//5.1e-10:121:29//NEUROSPORA CRASSA.//P22126
F-HEMBA1000193//PROLINE-RICH PEPTIDE P-B.//0.00078:56:41//HOMO SAPIENS (HUMAN).//P02814
F-HEMBA1000201//PROLINE-RICH PROTEIN MP-2 PRECURSOR.//0.00061:49:42//MUS MUSCULUS (MOUSE).//P05142
F-HEMBA1000213
F-HEMBA1000216//HYPOXIA-INDUCIBLE FACTOR 1 ALPHA (HIF-1 ALPHA) (ARNT INTERACTING PROTEIN).//1.6e-59:115:53//MUS MUSCULUS (MOUSE).//Q61221
F-HEMBA1000227//SUPPRESSOR PROTEIN SRP40.//0.00059:135:22//SACCHAROMYCES CEREVISIAE (BAKER'S YEAST).//P32583
F-HEMBA1000231//HYPOTHETICAL 60.7 KD PROTEIN C56F8.17C IN CHROMOSOME I.//0.024:60:38//SCHIZOSACCHAROMYCES POMBE (FISSION YEAST).//Q10264
F-HEMBA1000243//LINE-1 REVERSE TRANSCRIPTASE HOMOLOG.//0.0038:125:34//HOMO SAPIENS (HUMAN).//P08547
F-HEMBA1000244//HYPOTHETICAL 123.6 KD PROTEIN IN POR2-COX5B INTERGENIC REGION.//3.1e-17:149:36//SACCHAROMYCES CEREVISIAE (BAKER'S YEAST).//P40480
F-HEMBA1000251
F-HEMBA1000264//PROBABLE E5 PROTEIN.//1.0:49:36//HUMAN PAPILLOMAVIRUS TYPE 58.//P26552
F-HEMBA1000280//SHORT NEUROTOXIN 1 (TOXIN C-6).//0.98:58:31//NAJA NAJA KAOUTHIA (MONOCLED COBRA) (NAJA NAJA SIAMENSIS).//P14613
F-HEMBA1000282//!!!! ALU SUBFAMILY J WARNING ENTRY !!!!//0.14:26:65//HOMO SAPIENS (HUMAN).//P39188
F-HEMBA1000288
F-HEMBA1000290//HYPOTHETICAL 14 KD PROTEIN IN TVRI-6 REPETITIVE REGION.//3.8e-06:98:39//HOMO SAPIENS (HUMAN).//P10516
F-HEMBA1000302
F-HEMBA1000303//HYPOTHETICAL 104.4 KD PROTEIN F54G8.4 IN CHROMOSOME III.//1.3e-05:69:42//CAENORHABDITIS ELEGANS.//Q03601
F-HEMBA1000304//!!!! ALU SUBFAMILY SQ WARNING ENTRY !!!!//0.021:18:83//HOMO SAPIENS (HUMAN).//P39194
F-HEMBA1000307//MYOSIN HEAVY CHAIN, CLONE 203 (FRAGMENT).//7.1e-06:235:25//HYDRA ATTENUATA (HYDRA) (HYDRA VULGARIS).//P39922
F-HEMBA1000327
F-HEMBA1000333//SRP1 PROTEIN.//1.0:159:30//SCHIZOSACCHAROMYCES POMBE (FISSION YEAST).//Q10193
F-HEMBA1000338//!!!! ALU SUBFAMILY SP WARNING ENTRY !!!!//8.8e-26:36:83//HOMO SAPIENS (HUMAN).//P39193
F-HEMBA1000351
F-HEMBA1000355//BASIC PROLINE-RICH PEPTIDE P-E (IB-9).//0.99:22:50//HOMO SAPIENS (HUMAN).//P02811
F-HEMBA1000356//IMMEDIATE-EARLY PROTEIN IE180.//0.11:82:36//PSEUDORABIES VIRUS (STRAIN INDIANA-FUNKHAUSER / BECKER) (PRV).//P11675
F-HEMBA1000357//!!!! ALU SUBFAMILY SC WARNING ENTRY !!!!//2.1e-35:105:74//HOMO SAPIENS (HUMAN).//P39192
F-HEMBA1000366//HYPOTHETICAL TRANSCRIPTIONAL REGULATOR AF1627.//1.0:28:42//ARCHAEOGLOBUS FULGIDUS.//O28646
F-HEMBA1000369//PRESYNAPTIC DENSITY PROTEIN 95 (PSD-95).//0.013:140:26//HOMO SAPIENS (HUMAN).//P78352
F-HEMBA1000376//RETROVIRUS-RELATED POL POLYPROTEIN [CONTAINS: REVERSE TRANSCRIPTASE (EC 2.7.7.49); ENDONUCLEASE].//6.8e-08:66:42//MUS MUSCULUS (MOUSE).//P11369
F-HEMBA1000387//HYPOTHETICAL 63.2 KD PROTEIN C1F3.09 IN CHROMOSOME I.//1.5e-15:177:32//SCHIZOSACCHAROMYCES POMBE (FISSION YEAST).//Q10414
F-HEMBA1000390//PARATHYMOSIN.//0.0071:61:29//HOMO SAPIENS (HUMAN).//P20962
F-HEMBA1000392//!!!! ALU SUBFAMILY SQ WARNING ENTRY !!!!//2.1e-30:92:69//HOMO SAPIENS (HUMAN).//P39194
F-HEMBA1000396/LINE-1 REVERSE TRANSCRIPTASE HOMOLOG.//3.9e-23:64:57//HOMO SAPIENS (HUMAN).//P08547
F-HEMBA1000411
F-HEMBA1000418
F-HEMBA1000422//!!!! ALU SUBFAMILY J WARNING ENTRY !!!!//8.3e-10:90:53//HOMO SAPIENS (HUMAN).//P39188
F-HEMBA1000428//LINE-1 REVERSE TRANSCRIPTASE HOMOLOG.//3.1e-12:72:55//HOMO SAPIENS (HUMAN).//P08547
F-HEMBA1000434
F-HEMBA1000442//GENE 11 PROTEIN.//1.0:28:46//SPIROPLASMA VIRUS SPV1-R8A2 B.//P15902
F-HEMBA1000456//26S PROTEASOME REGULATORY SUBUNIT MTS4 (19S REGULATORY CAP REGION OF 26S PROTEASE SUBUNIT 2).//0.077:118:28//SCHIZOSACCHAROMYCES POMBE (FISSION YEAST).//P87048
F-HEMBA1000459//HEME-REGULATED EUKARYOTIC INITIATION FACTOR EIF-2-ALPHA KINASE (EC 2.7.1.-) (HRI).//4.8e-62:102:78//ORYCTOLAGUS CUNICULUS (RABBIT).//P33279
F-HEMBA1000460//LYSIS PROTEIN (E PROTEIN) (GPE).//1.0:24:50//BACTERIOPHAGE ALPHA-3.//P31280
F-HEMBA1000464
F-HEMBA1000469//PILI PROTEIN.//1.0:27:44//PSEUDOMONAS AERUGINOSA.//P43502
F-HEMBA1000488//ZINC FINGER PROTEIN 151 (MIZ-1 PROTEIN).//1.1e-07:90:38//HOMO SAPIENS (HUMAN).//Q13105
F-HEMBA1000490//PLECTIN.//0.74:254:25//RATTUS NORVEGICUS (RAT).//P30427
F-HEMBA1000491//RAS-RELATED PROTEIN M-RAS.//3.0e-14:100:36//RATTUS NORVEGICUS (RAT).//P97538
F-HEMBA1000501//!!!! ALU SUBFAMILY SQ WARNING ENTRY !!!!//1.5e-20:81:54//HOMO SAPIENS (HUMAN).//P39194
F-HEMBA1000504
F-HEMBA1000505//NEURON-SPECIFIC X11 PROTEIN (FRAGMENT).//0.00028:128:32//HOMO SAPIENS (HUMAN).//Q02410
F-HEMBA1000508//CHITIN SYNTHASE 3 (EC 2.4.1.16) (CHITIN-UDP ACETYL-GLUCOSAMINYL TRANSFERASE 3).//0.61:132:25//SACCHAROMYCES CEREVISIAE (BAKER'S YEAST).//P29465
F-HEMBA1000518
F-HEMBA1000519//!!!! ALU SUBFAMILY SB WARNING ENTRY !!!!//1.8e-37:68:75//HOMO SAPIENS (HUMAN).//P39189
F-HEMBA1000520//!!!! ALU SUBFAMILY SC WARNING ENTRY !!!!//5.2e-09:75:49//HOMO SAPIENS (HUMAN).//P39192
F-HEMBA1000523//TESTIS-SPECIFIC PROTEIN PBS13.//1.5e-35:257:36//MUS MUSCULUS (MOUSE).//Q01755
F-HEMBA1000531//HEAT SHOCK PROTEIN 70 B2.//1.6e-14:72:44//ANOPHELES ALBIMANUS (NEW WORLD MALARIA MOSQUITO).//P41827
F-HEMBA1000534//!!!! ALU SUBFAMILY SP WARNING ENTRY !!!!//9.7e-32:96:78//HOMO SAPIENS (HUMAN).//P39193
F-HEMBA1000540//LANTIBIOTIC LACTICIN 481 PRECURSOR (LACTOCOCCIN DR).//1.0:12:75//LACTOCOCCUS LACTIS (SUBSP. LACTIS) (STREPTOCOCCUS LACTIS).//P36499
F-HEMBA1000542//SPERM MITOCHONDRIAL CAPSULE SELENOPROTEIN (MCS).//0.0089:79:31//MUS MUSCULUS (MOUSE).//P15265
F-HEMBA1000545//LINE-1 REVERSE TRANSCRIPTASE HOMOLOG.//9.0e-83:256:66//HOMO SAPIENS (HUMAN).//P08547
F-HEMBA1000555//TRANSLATION INITIATION FACTOR IF-2.//3.6e-06:252:22//SACCHAROMYCES CEREVISIAE (BAKER'S YEAST).//P39730
F-HEMBA1000557
F-HEMBA1000561//ZINC FINGER PROTEIN 81 (FRAGMENT).//9.1 e-18:200:28//HOMO SAPIENS (HUMAN).//P51508
F-HEMBA1000563
F-HEMBA1000568
F-HEMBA1000569//GPI-ANCHORED PROTEIN P137.//1.0e-40:137:54//HOMO SAPIENS (HUMAN).//Q14444
F-HEMBA1000575
F-HEMBA1000588
F-HEMBA1000591//!!!! ALU SUBFAMILY SQ WARNING ENTRY !!!!//3.1e-17:41:92//HOMO SAPIENS (HUMAN).//P39194
F-HEMBA1000592//CENTROMERIC PROTEIN E (CENP-E PROTEIN).//0.18:128:23//HOMO SAPIENS (HUMAN).//Q02224
F-HEMBA1000594//HYPOTHETICAL 29.3 KD PROTEIN B0280.6 IN CHROMOSOME III.//0.93:24:54//CAENORHABDITIS ELEGANS.//P41997
F-HEMBA1000604//!!!! ALU SUBFAMILY J WARNING ENTRY !!!!//0.00010:49:55//HOMO SAPIENS (HUMAN).//P39188
F-HEMBA1000608//HYPOTHETICAL PROTEIN KIAA0411 (FRAGMENT).//1.8e-55:179:61//HOMO SAPIENS (HUMAN).//O43295
F-HEMBA1000622//!!!! ALU SUBFAMILY J WARNING ENTRY !!!!//1.1e-21:94:62//HOMO SAPIENS (HUMAN).//P39188
F-HEMBA1000636//SULFATED SURFACE GLYCOPROTEIN 185 (SSG 185).//0.34:73:36//VOLVOX CARTERI.//P21997
F-HEMBA1000637//BASIC PROLINE-RICH PEPTIDE IB-1.//0.0057:76:38//HOMO SAPIENS (HUMAN).//P04281
F-HEMBA1000655
F-HEMBA1000657//ZINC FINGER PROTEIN GCS1.//1.5e-07:66:37//SACCHAROMYCES CEREVISIAE (BAKER'S YEAST).//P35197
F-HEMBA1000662//METALLOTHIONEIN-II (MT-II).//0.79:33:39//CRICETULUS GRISEUS (CHINESE HAMSTER).//P02799
F-HEMBA1000673//!!!! ALU SUBFAMILY SP WARNING ENTRY !!!!//3.1e-17:86:59//HOMO SAPIENS (HUMAN).//P39193
F-HEMBA1000682//RETROVIRUS-RELATED POL POLYPROTEIN [CONTAINS: REVERSE TRANSCRIPTASE (EC 2.7.7.49); ENDONUCLEASE].//3.0e-13:45:44//MUS MUSCULUS (MOUSE).//P11369
F-HEMBA1000686//HYPOTHETICAL 48.0 KD PROTEIN C1B3.08 IN CHROMOSOME I.//4.5e-07:79:34//SCHIZOSACCHAROMYCES POMBE (FISSION YEAST).//O13873
F-HEMBA1000702
F-HEMBA1000705//PROTEIN Q300.//0.80:25:44//MUS MUSCULUS (MOUSE).//Q02722
F-HEMBA1000719//MYOSIN IC HEAVY CHAIN.//0.0026:115:44//ACANTHAMOEBA CASTELLANII (AMOEBA).//P10569
F-HEMBA1000722
F-HEMBA1000726//!!!! ALU SUBFAMILY SB2 WARNING ENTRY !!!!//7.4e-32:83:77//HOMO SAPIENS (HUMAN).//P39191
F-HEMBA1000727//ZINC FINGER PROTEIN CTH2 (YTIS11 PROTEIN).//0.73:26:46//SACCHAROMYCES CEREVISIAE (BAKER'S YEAST).//P47977
F-HEMBA1000747
F-HEMBA1000749//HYPOTHETICAL PROTEIN HI1484.//1.0:42:35//HAEMOPHILUS INFLUENZAE.//P44211
F-HEMBA1000752//RETROVIRUS-RELATED ENV POLYPROTEIN.//1.0e-08:84:39//HOMO SAPIENS (HUMAN).//P10267
F-HEMBA1000769
F-HEMBA1000773//PAIRED BOX PROTEIN PAX-4.//1.0:107:33//HOMO SAPIENS (HUMAN).//O43316
F-HEMBA1000774//!!!! ALU SUBFAMILY J WARNING ENTRY !!!!//1.3e-23:92:63//HOMO SAPIENS (HUMAN).//P39188
F-HEMBA1000791
F-HEMBA1000817//PROLACTIN RECEPTOR PRECURSOR (PRL-R).//0.079:87:29//CERVUS ELAPHUS (RED DEER).//Q28235
F-HEMBA1000822
F-HEMBA1000827//HYPOTHETICAL 8.4 KD PROTEIN.//0.98:48:39//VACCINIA VIRUS (STRAIN COPENHAGEN).//P20546
F-HEMBA1000843//HYPOTHETICAL 7.3 KD PROTEIN D1044.5 IN CHROMOSOME III.//0.92:46:34//CAENORHABDITIS ELEGANS.//P41953
F-HEMBA1000851//HOMEOBOX PROTEIN GBX-2 (GASTRULATION AND BRAIN-SPECIFIC HOMEOBOX PROTEIN 2).//0.048:39:51//HOMO SAPIENS (HUMAN).//P52951
F-HEMBA1000852//ARYLSULFATASE D PRECURSOR (EC 3.1.6.-) (ASD).//4.0e-24:29:100//HOMO SAPIENS (HUMAN).//P51689
F-HEMBA1000867
F-HEMBA1000869//PROBABLE E5 PROTEIN.//0.99:70:27//HUMAN PAPILLOMAVIRUS TYPE 18.//P06792
F-HEMBA1000870//MYOTOXIN 3 PRECURSOR (CROTAMINE 3).//0.79:43:32//CROTALUS DURISSUS TERRIFICUS (SOUTH AMERICAN RATTLESNAKE).//P24333
F-HEMBA1000872//GAR2 PROTEIN.//0.89:70:31//SCHIZOSACCHAROMYCES POMBE (FISSION YEAST).//P41891
F-HEMBA1000876//DEFENSIN.//0.89:34:38//ALLOMYRINA DICHOTOMA.//Q10745
F-HEMBA1000908//DNA-BINDING P52/P100 COMPLEX, 100 KD SUBUNIT (FRAGMENTS).//0.69:43:37//HOMO SAPIENS (HUMAN).//P30808
F-HEMBA1000910//MELANOMA-ASSOCIATED ANTIGEN B3 (MAGE-B3 ANTIGEN).//5.1e-08:44:38//HOMO SAPIENS (HUMAN).//O15480
F-HEMBA1000918//60S RIBOSOMAL PROTEIN L37-A (YL35) (FRAGMENT).//1.0:19:52//SCHIZOSACCHAROMYCES POMBE (FISSION YEAST).//P22667
F-HEMBA1000919//69 KD PARAFLAGELLAR ROD PROTEIN (69 KD PFR PROTEIN) (PFR-A/PFR-B).//0.29:116:30//TRYPANOSOMA BRUCEI BRUCEI.//P22225
F-HEMBA1000934
F-HEMBA1000942//!!!! ALU SUBFAMILY J WARNING ENTRY !!!!//0.85:27:59//HOMO SAPIENS (HUMAN).//P39188
F-HEMBA1000943
F-HEMBA1000946//STO-2 PROTEIN.//0.82:82:30//CAENORHABDITIS ELEGANS.//Q19958
F-HEMBA1000960//!!!! ALU SUBFAMILY SC WARNING ENTRY !!!!//0.0097:29:72//HOMO SAPIENS (HUMAN).//P39192
F-HEMBA1000968//METALLOTHIONEIN 20-III ISOFORMS A AND B (MT-20-IIIA AND MT-20-IIIB).//0.047:45:37//MYTILUS EDULIS (BLUE MUSSEL).//P80253
F-HEMBA1000971//HYPOTHETICAL BHLF1 PROTEIN.//0.038:172:31//EPSTEIN-BARR VIRUS (STRAIN B95-8) (HUMAN HERPESVIRUS 4).//P03181
F-HEMBA1000972
F-HEMBA1000974//HYPOTHETICAL PROTEIN MG441.//0.98:66:28//MYCOPLASMA GENITALIUM.//P47679
F-HEMBA1000975//COLLAGEN ALPHA 2(VIII) CHAIN (ENDOTHELIAL COLLAGEN) (FRAGMENT).//0.028:57:36//HOMO SAPIENS (HUMAN).//P25067
F-HEMBA1000985
F-HEMBA1000986//SUBMANDIBULAR GLAND SECRETORY GLX-RICH PROTEIN CB PRECURSOR (GRP-CB) (CONTIGUOUS REPEAT POLYPEPTIDE) (CRP).//0.13:91:34//RATTUS NORVEGICUS (RAT).//P08462
F-HEMBA1000991//HYPOTHETICAL 46.2 KD TRP-ASP REPEATS CONTAINING PROTEIN D2013.2 IN CHROMOSOME II.//5.6e-05:37:45//CAENORHABDITIS ELEGANS.//Q18964
F-HEMBA1001007//HYPOTHETICAL PROTEIN KIAA0179.//0.27:72:41//HOMO SAPIENS (HUMAN).//Q14684
F-HEMBA1001008//!!!! ALU SUBFAMILY SQ WARNING ENTRY !!!!//2.4e-25:61:70//HOMO SAPIENS (HUMAN).//P39194
F-HEMBA1001009//CUTICLE COLLAGEN 34.//0.044:214:29//CAENORHABDITIS ELEGANS.//P34687
F-HEMBA1001017//SYNDECAN-3 PRECURSOR (N-SYNDECAN) (NEUROGLYCAN).//5.0e-85:191:84//RATTUS NORVEGICUS (RAT).//P33671
F-HEMBA1001019
F-HEMBA1001020//!!!! ALU SUBFAMILY J WARNING ENTRY !!!!//6.7e-24:49:73//HOMO SAPIENS (HUMAN).//P39188
F-HEMBA1001022
F-HEMBA1001024//LINE-1 REVERSE TRANSCRIPTASE HOMOLOG.//3.0e-11:61:59//HOMO SAPIENS (HUMAN).//P08547
F-HEMBA1001026//HYPOTHETICAL PROTEIN BB0073.//0.94:63:34//BORRELIA BURGDORFERI (LYME DISEASE SPIROCHETE).//O51100
F-HEMBA1001043//INVOLUCRIN.//0.0036:238:25//SAGUINUS OEDIPUS (COTTON-TOP TAMARIN).//P24712
F-HEMBA1001051//!!!! ALU SUBFAMILY SB WARNING ENTRY !!!!//3.3e-32:95:75//HOMO SAPIENS (HUMAN).//P39189
F-HEMBA1001052//CURROMYCIN RESISTANCE PROTEIN.//1.0:31:38//STREPTOMYCES HYGROSCOPICUS.//P16961
F-HEMBA1001059//N-ACETYLGALACTOSAMINE-6-SULFATASE PRECURSOR (EC 3.1.6.4) (N- ACETYLGALACTOSAMINE-6-SULFATE SULFATASE) (GALACTOSE-6-SULFATE SULFATASE) (GALNAC6S SULFATASE) (CHONDROITINSULFATASE) (CHONDROITINASE).//3.2e-132:249:94//HOMO SAPIENS (HUMAN).//P34059
F-HEMBA1001060
F-HEMBA1001071//PROCOLLAGEN ALPHA 1(III) CHAIN PRECURSOR.//8.3e-23:51:96//HOMO SAPIENS (HUMAN).//P02461
F-HEMBA1001077//AUTOIMMUNE REGULATOR (APECED PROTEIN).//3.4e-06:37:56//HOMO SAPIENS (HUMAN).//O43918
F-HEMBA1001080//INFECTED CELL PROTEIN ICP34.5 (NEUROVIRULENCE FACTOR ICP34.5).//0.0012:70:38//HERPES SIMPLEX VIRUS (TYPE 1 / STRAW MGH-10).//P37319
F-HEMBA1001085//SERINE/THREONINE PROTEIN PHOSPHATASE 5 (EC 3.1.3.16) (PPS) (PROTEIN PHOSPHATASE T) (PPT) (FRAGMENT).//0.00018:76:32//MUS MUSCULUS (MOUSE).//Q60676
F-HEMBA1001088//PINCH PROTEIN (PARTICULARY INTERESTING NEW CYS-HIS PROTEIN).//3.5e-50:176:57//HOMO SAPIENS (HUMAN).//P48059
F-HEMBA1001094
F-HEMBA1001099//LIGHT-HARVESTING PROTEIN B800/850/890, ALPHA-2 CHAIN (EHA-ALPHA-2) (ANTENNA PIGMENT PROTEIN, ALPHA-2 CHAIN) (FRAGMENT).//1.0:15:60//ECTOTHIORHODOSPIRA HALOPHILA.//P80101
F-HEMBA1001109//!!!! ALU SUBFAMILY SB WARNING ENTRY !!!!//6.7e-37:102:82//HOMO SAPIENS (HUMAN).//P39189
F-HEMBA1001121//LINE-1 REVERSE TRANSCRIPTASE HOMOLOG.//0.036:49:46//HOMO SAPIENS (HUMAN).//P08547
F-HEMBA1001122
F-HEMBA1001123
F-HEMBA1001133//HYPOTHETICAL 9.4 KD PROTEIN (ORF2).//0.86:29:41//FELINE IMMUNODEFICIENCY VIRUS (ISOLATE SAN DIEGO) (FIV), AND FELINE IMMUNODEFICIENCY VIRUS (ISOLATE PETALUMA) (FIV).//P19033
F-HEMBA1001137//ZINC FINGER PROTEIN 84 (ZINC FINGER PROTEIN HPF2).//2.0e-22:103:52//HOMO SAPIENS (HUMAN).//P51523
F-HEMBA1001140//COLLAGEN ALPHA 4(IV) CHAIN PRECURSOR.//0.032:94:36//HOMO SAPIENS (HUMAN).//P53420
F-HEMBA1001172
F-HEMBA1001174//ADP-RIBOSYLATION FACTOR-LIKE PROTEIN 5.//2.9e-78:179:79//RATTUS NORVEGICUS (RAT).//P51646
F-HEMBA1001197//MAJOR PRION PROTEIN PRECURSOR (PRP) (PRP27-30) (PRP33-35C) (FRAGMENT).//0.051:96:32//CERCOCEBUS ATERRIMUS, AND MACACA SYLVANUS (BARBARY APE).//Q95145
F-HEMBA1001208
F-HEMBA1001213
F-HEMBA1001226//PROTEASOME COMPONENT C8 (EC 3.4.99.46) (MACROPAIN SUBUNIT C8) (MULTICATALYTIC ENDOPEPTIDASE COMPLEX SUBUNIT C8).//1.5e-08:24:91//HOMO SAPIENS (HUMAN).//P25788
F-HEMBA1001235//FIBRONECTIN (FN) (FRAGMENT).//0.76:50:38//ORYCTOLAGUS CUNICULUS (RABBIT).//Q28749
F-HEMBA1001247//SULFATED SURFACE GLYCOPROTEIN 185 (SSG 185).//0.00052:16:81//VOLVOX CARTERI.//P21997
F-HEMBA1001257//2-ARYLPROPIONYL-COA EPIMERASE (EC 5.-.-.-).//1.6e-68:178:77//RATTUS NORVEGICUS (RAT).//P70473
F-HEMBA1001265//MANNAN ENDO-1,4-BETA-MANNOSIDASE A PRECURSOR (EC 3.2.1.78) (BETA- MANNANASE A) (1,4-BETA-D-MANNAN MANNANOHYDROLASE A).//0.67:23:60//PIROMYCES SP.//P55296
F-HEMBA1001281//HYPOTHETICAL 8.9 KD PROTEIN YCF34 (ORF76).//0.83:48:35//PORPHYRA PURPUREA.//P51229
F-HEMBA1001286//COMPLEMENT DECAY-ACCELERATING FACTOR PRECURSOR.//1.3e-07:185:29//CAVIA PORCELLUS (GUINEA PIG).//Q60401
F-HEMBA1001289//METABOTROPIC GLUTAMATE RECEPTOR 3 PRECURSOR.//0.00018:159:30//RATTUS NORVEGICUS (RAT).//P31422
F-HEMBA1001294
F-HEMBA1001299//!!!! ALU SUBFAMILY SX WARNING ENTRY !!!!//5.3e-07:27:77//HOMO SAPIENS (HUMAN).//P39195
F-HEMBA1001302//45 KD CALCIUM-BINDING PROTEIN PRECURSOR (STROMAL CELL-DERIVED FACTOR 4) (SDF-4).//3.3e-61:150:76//MUS MUSCULUS (MOUSE).//Q61112
F-HEMBA1001303
F-HEMBA1001310//HYPOTHETICAL PROTEIN KIAA0161.//2.7e-10:170:27//HOMO SAPIENS (HUMAN).//P50876
F-HEMBA1001319
F-HEMBA1001323
F-HEMBA1001326//HYPOTHETICAL 55.1 KD PROTEIN IN FAB1-PES4 INTERGENIC REGION.//1.1e-39:144:38//SACCHAROMYCES CEREVISIAE (BAKER'S YEAST).//P43601
F-HEMBA1001327
F-HEMBA1001330
F-HEMBA1001351//VESICLE-ASSOCIATED MEMBRANE PROTEIN/SYNAPTOBREVIN BINDING PROTEIN (VAP-33).//1.9e-37:155:46//APLYSIA CALIFORNICA (CALIFORNIA SEA HARE).//Q16943
F-HEMBA1001361//RUBREDOXIN (RD).//0.95:44:29//ALCALIGENES EUTROPHUS.//P31912
F-HEMBA1001375//AEROLYSIN REGULATORY PROTEIN.//0.013:45:33//AEROMONAS SOBRIA.//P09165
F-HEMBA1001377//SPERM PROTAMINE P1.//1.0:22:40//PLANIGALE MACULATA SINUALIS (COMMON PLANIGALE).//O18746
F-HEMBA1001383//TAT PROTEIN (TRANSACTIVATING REGULATORY PROTEIN).//0.60:37:29//HUMAN IMMUNODEFICIENCY VIRUS TYPE 1 (Z2/CDC-Z34 ISOLATE) (HIV-1).//P12506
F-HEMBA1001387//GTP-BINDING PROTEIN TC10.//6.6e-43:83:92//HOMO SAPIENS (HUMAN).//P17081
F-HEMBA1001388//HYPOTHETICAL PROTEIN KIAA0136 (FRAGMENT).//0.00088:46:45//HOMO SAPIENS (HUMAN).//Q14149
F-HEMBA1001391
F-HEMBA1001398//CLOACIN (EC 3.1.-.-) (RIBONUCLEASE).//1.0:59:37//ESCHERICHIA COLI.//P00645
F-HEMBA1001405//SMALL PROLINE RICH PROTEIN II (SPR-II) (CLONE 930).//0.25:41:34//HOMO SAPIENS (HUMAN).//P22531
F-HEMBA1001407//SALIVARY PROLINE-RICH PROTEIN PRECURSOR (CLONES CP3, CP4 AND CP5) [CONTAINS: BASIC PEPTIDE IB-6; PEPTIDE P-H].//4.0e-09:129:40//HOMO SAPIENS (HUMAN).//P04280
F-HEMBA1001411//HYPOTHETICAL 34.9 KD PROTEIN IN CYSJ-ENO INTERGENIC REGION (O313).//0.95:88:31//ESCHERICHIA COLI.//P55140
F-HEMBA1001413//SOX-12 PROTEIN (FRAGMENT).//0.95:46:32//MUS MUSCULUS (MOUSE).//Q04890
F-HEMBA1001415//HISTONE H5.//0.43:95:29//GALLUS GALLUS (CHICKEN).//P02259
F-HEMBA1001432//LANTIBIOTIC NISIN A PRECURSOR.//0.77:46:32//LACTOCOCCUS LACTIS (SUBSP. LACTIS) (STREPTOCOCCUS LACTIS).//P13068
F-HEMBA1001433//LINE-1 REVERSE TRANSCRIPTASE HOMOLOG.//4.8e-09:132:31//NYCTICEBUS COUCANG (SLOW LORIS).//P08548
F-HEMBA1001435//!!!! ALU SUBFAMILY SB WARNING ENTRY !!!!//1.2e-31:84:77//HOMO SAPIENS (HUMAN).//P39189
F-HEMBA1001442
F-HEMBA1001446//ANTIFREEZE PEPTIDE 4 PRECURSOR.//0.71:41:39//PSEUDOPLEURONECTA AMERICANUS (WINTER FLOUNDER).//P02734
F-HEMBA1001450//PROLINE-RICH PROTEIN LAS17.//0.13:127:27//SACCHAROMYCES CEREVISIAE (BAKER'S YEAST).//Q12446
F-HEMBA1001454//ATP SYNTHASE PROTEIN 8 (EC 3.6.1.34) (A6L).//0.57:38:47//HANSENULA WINGEI (YEAST).//P48882
F-HEMBA1001455//CHEMOTAXIS PROTEIN CHEA (EC 2.7.3.-).//0.98:124:25//BORRELIA BURGDORFERI (LYME DISEASE SPIROCHETE).//Q44737
F-HEMBA1001463//!!!! ALU SUBFAMILY SQ WARNING ENTRY !!!!//2.8e-32:62:67//HOMO SAPIENS (HUMAN).//P39194
F-HEMBA1001476//NUCLEOPORIN NUP159 (NUCLEAR PORE PROTEIN NUP159).//6.8e-09:252:36//SACCHAROMYCES CEREVISIAE (BAKER'S YEAST).//P40477
F-HEMBA1001478
F-HEMBA1001497//!!!! ALU SUBFAMILY SQ WARNING ENTRY !!!!//1.2e-33:105:72//HOMO SAPIENS (HUMAN).//P39194
F-HEMBA1001510//!!!! ALU SUBFAMILY SB WARNING ENTRY !!!!//8.3e-37:54:81//HOMO SAPIENS (HUMAN).//P39189
F-HEMBA1001515//LINE-1 REVERSE TRANSCRIPTASE HOMOLOG.//2.0e-63:223:57//HOMO SAPIENS (HUMAN).//P08547
F-HEMBA1001517
F-HEMBA1001522//TROPOMYOSIN ALPHA CHAIN, SMOOTH MUSCLE.//0.78:150:22//COTURNIX COTURNIX JAPONICA (JAPANESE QUAIL).//P49437
F-HEMBA1001526//PERIPLASMIC [FE] HYDROGENASE 1 (EC 1.18.99.1).//1.6e-06:130:29//CLOSTRIDIUM PASTEURIANUM.//P29166
F-HEMBA1001533//PROBABLE E5A PROTEIN.//0.73:35:37//HUMAN PAPILLOMAVIRUS TYPE 6A.//Q84296
F-HEMBA1001557//HYPOTHETICAL 17.1 KD PROTEIN IN PUR5 3'REGION.//1.5e-07:99:36//SACCHAROMYCES CEREVISIAE (BAKER'S YEAST).//P38898
F-HEMBA1001566//HYPOTHETICAL PROTEIN BB0692.//0.91:27:44//BORRELIA BURGDORFERI (LYME DISEASE SPIROCHETE).//O51635
F-HEMBA1001569//SYNAPTOBREVIN 2 (VESICLE ASSOCIATED MEMBRANE PROTEIN 2) (VAMP-2).//2.2e-50:110:95//HOMO SAPIENS (HUMAN), AND BOS TAURUS (BOVINE).//P19065
F-HEMBA1001570//!!!! ALU SUBFAMILY SX WARNING ENTRY !!!!//3.3e-33:107:72//HOMO SAPIENS (HUMAN).//P39195
F-HEMBA1001579//RING CANAL PROTEIN (KELCH PROTEIN).//1.2e-14:111:39//DROSOPHILA MELANOGASTER (FRUIT FLY).//Q04652
F-HEMBA1001581
F-HEMBA1001585
F-HEMBA1001589//PROBABLE DNA-BINDING PROTEIN (AGNOPROTEIN).//0.98:51:33//HUMAN ADENOVIRUS TYPE 2.//P03263
F-HEMBA1001595//SEPTIN 2 HOMOLOG (FRAGMENT).//3.0e-124:274:85//HOMO SAPIENS (HUMAN).//Q14141
F-HEMBA1001608//RENAL SODIUM/DICARBOXYLATE COTRANSPORTER (NA(+)/DICARBOXYLATE COTRANSPORTER).//0.99:28:39//ORYCTOLAGUS CUNICULUS (RABBIT).//Q28615
F-HEMBA1001620//MYO-INOSITOL-1-PHOSPHATE SYNTHASE (EC 5.5.1.4) (IPS).//4.3e-45:222:46//SPIRODELA POLYRRHIZA.//P42803
F-HEMBA1001635//FIBRILLARIN.//0.10:72:38//CAENORHABDITIS ELEGANS.//Q22053
F-HEMBA1001636//PAIRED BOX PROTEIN PAX-8, ISOFORMS 8C/8D.//0.75:38:47//HOMO SAPIENS (HUMAN).//Q09155
F-HEMBA1001640//!!!! ALU SUBFAMILY J WARNING ENTRY !!!!/14.7e-06:80:41//HOMO SAPIENS (HUMAN).//P39188
F-HEMBA1001647//TRANSCRIPTION INITIATION FACTOR TFIID 135 KD SUBUNIT (TAFII-135) (TAFII135) (TAFII-130) (TAFII130).//0.075:165:32//HOMO SAPIENS (HUMAN).//O00268
F-HEMBA1001651//GOLGIN-95.//6.8e-05:141:24//HOMO-SAPIENS (HUMAN).//Q08379
F-HEMBA1001655//PROLINE-RICH PROTEIN LAS17.//0.19:97:30//SACCHAROMYCES CEREVISIAE (BAKER'S YEAST).//Q12446
F-HEMBA1001658//TETRAHYDROMETHANOPTERIN S-METHYLTRANSFERASE 12 KD SUBUNIT (EC 2.1.1.86) (N5-METHYLTETRAHYDROMETHANOPTERIN-COENZYME M METHYLTRANSFERASE 12 KD SUBUNIT).//1.0:29:44//METHANOBACTERIUM THERMOAUTOTROPHICUM (STRAIN MARBURG / DSM 2133).//Q50773
F-HEMBA1001661//CELLULOSE COMPLEMENTING PROTEIN.//0.35:87:33//ACETOBACTER XYLINUM (ACETOBACTER PASTEURIANUS).//P37697
F-HEMBA1001672//CIRCUMSPOROZOITE PROTEIN PRECURSOR (CS).//2.7e-10:216:35//PLASMODIUM CYNOMOLGI (STRAIN BEROK).//P08672
F-HEMBA1001675//NODULIN 20 PRECURSOR (N-20).//0.98:36:44//GLYCINE MAX (SOYBEAN).//P08960
F-HEMBA1001678//!!!! ALU SUBFAMILY SX WARNING ENTRY !!!!//8.2e-13:62:64//HOMO SAPIENS (HUMAN).//P39195
F-HEMBA1001681//HYPOTHETICAL 41.5 KD PROTEIN IN P6.5-VP48 INTERGENIC REGION (P40) (ORF3) (ORF102).//1.0:51:39//ORGYIA PSEUDOTSUGATA MULTICAPSID POLYHEDROSIS VIRUS (OPMNPV).//P24653
F-HEMBA1001702//ATP SYNTHASE A CHAIN (EC 3.6.1.34) (PROTEIN 6).//0.017:54:37//TRYPANOSOMA BRUCEI BRUCEI.//P24499
F-HEMBA1001709//HYPOTHETICAL 21.2 KD PROTEIN IN TOR2-MNN4 INTERGENIC REGION.//0.59:109:35//SACCHAROMYCES CEREVISIAE (BAKER'S YEAST).//P36042
F-HEMBA1001711
F-HEMBA1001712//HYPOTHETICAL 6.9 KD PROTEIN IN 100 KD PROTEIN REGION.//0.54:44:34//HUMAN ADENOVIRUS TYPE 41.//P23690
F-HEMBA1001714//ATPASE INHIBITOR, MITOCHONDRIAL PRECURSOR.//1.2e-19:60:75//RATTUS NORVEGICUS (RAT).//Q03344
F-HEMBA1001718//HYPOTHETICAL PROTEIN UL63.//1.0:54:37//HUMAN CYTOMEGALOVIRUS (STRAIN AD169).//P16820
F-HEMBA1001723//HYPOTHETICAL 34.0 KD TRP-ASP REPEATS CONTAINING PROTEIN IN SIS1-MRPL2 INTERGENIC REGION.//5.1e-26:90:53//SACCHAROMYCES CEREVISIAE (BAKER'S YEAST).//P41318
F-HEMBA1001731//HYPOTHETICAL 16.6 KD PROTEIN.//0.71:49:32//AVIAN INFECTIOUS BURSAL DISEASE VIRUS (STRAIN 52/70) (IBDV).//P25221
F-HEMBA1001734
F-HEMBA1001744//SCY1 PROTEIN.//2.1e-11:182:29//SACCHAROMYCES CEREVISIAE (BAKER'S YEAST).//P53009
F-HEMBA1001745//HYPOTHETICAL 11.6 KD PROTEIN IN NUT1-ARO2 INTERGENIC REGION PRECURSOR.//1.0:36:38//SACCHAROMYCES CEREVISIAE (BAKER'S YEAST).//P53116
F-HEMBA1001746//PROTEIN-EXPORT MEMBRANE PROTEIN SECG HOMOLOG.//0.94:48:35//MYCOBACTERIUM LEPRAE.//P38388
F-HEMBA1001761
F-HEMBA1001781//ZINC FINGER PROTEIN 19 (ZINC FINGER PROTEIN KOX12) (FRAGMENT).//0.028:47:40//HOMO SAPIENS (HUMAN).//P17023
F-HEMBA1001784//HYPOTHETICAL 6.1 KD PROTEIN C03B1.10 IN CHROMOSOME X.//0.00068:32:46//CAENORHABDITIS ELEGANS .//Q11116
F-HEMBA1001791//METALLOTHIONEIN (MT).//1.0:34:35//PLEURONECTES PLATESSA (PLAICE).//P07216
F-HEMBA1001800//ZINC FINGER PROTEIN MFG-1 (ZINC FINGER PROTEIN 58) (FRAGMENT).//1.5e-14:60:48//MUS MUSCULUS (MOUSE).//P16372
F-HEMBA1001803
F-HEMBA1001804//GLYCINE-RICH CELL WALL STRUCTURAL PROTEIN 1 PRECURSOR.//9.3e-17:56:57//ORYZA SATIVA (RICE).//P25074
F-HEMBA1001808//PARANEOPLASTIC ENCEPHALOMYELITIS ANTIGEN HUD HOMOLOG (HU-ANTIGEN D).//0.75:97:31//RATTUS NORVEGICUS (RAT).//O09032
F-HEMBA1001809//IMMEDIATE-EARLY PROTEIN IE180.//4.5e-11:206:36//PSEUDORABIES VIRUS (STRAIN INDIANA-FUNKHAUSER / BECKER) (PRV).//P11675
F-HEMBA1001815//60S RIBOSOMAL PROTEIN L37-B (YL27) (FRAGMENT).//0.34:30:30//SCHIZOSACCHAROMYCES POMBE (FISSION YEAST).//P05733
F-HEMBA1001819//ZINC FINGER PROTEIN 135.//2.6e-102:262:66//HOMO SAPIENS (HUMAN).//P52742
F-HEMBA1001820
F-HEMBA1001822//EPIDERMAL GROWTH FACTOR RECEPTOR SUBSTRATE SUBSTRATE 15 (PROTEIN EPS15).//1.2e-18:251:33//MUS MUSCULUS (MOUSE).//P42567
F-HEMBA1001824//KERATIN, ULTRA HIGH-SULFUR MATRIX PROTEIN (UHS KERATIN).//4.7e-11:124:37//OVIS ARIES (SHEEP).//P26372
F-HEMBA1001835
F-HEMBA1001844//!!!! ALU SUBFAMILY SX WARNING ENTRY !!!!//4.3e-14:36:63//HOMO SAPIENS (HUMAN).//P39195
F-HEMBA1001847//ZINC FINGER PROTEIN 29 (ZFP-29).//2.7e-36:135:51//MUS MUSCULUS (MOUSE).//Q07230
F-HEMBA1001861
F-HEMBA1001864//HEAT-STABLE ENTEROTOXIN A3/A4 PRECURSOR (STA3/STA4) (ST-IB) (ST-H).//1.0:31:38//ESCHERICHIA COLI.//P07965
F-HEMBA1001866//UDP-GLUCOSE:GLYCOPROTEIN GLUCOSYLTRANSFERASE PRECURSOR (EC 2.4.1.-) (DUGT).//9.7e-42:234:41//DROSOPHILA MELANOGASTER (FRUIT FLY).//Q09332
F-HEMBA1001869//HYPOTHETICAL 94.9 KD PROTEIN C22E12.11C IN CHROMOSOME I.//5.3e-13:65.47//SCHIZOSACCHAROMYCES POMBE (FISSION YEAST).//Q10362
F-HEMBA1001888//HYPOTHETICAL 11.4 KD PROTEIN (ORF1).//0.85:62:37//STREPTOMYCES FRADIAE.//P26800
F-HEMBA1001896//DIMETHYLGLYCINE DEHYDROGENASE PRECURSOR (EC 1.5.99.2) (ME2GLYDH).//9.8e-20:250:29//RATTUS NORVEGICUS (RAT).//Q63342
F-HEMBA1001910//EUKARYOTIC TRANSLATION INITIATION FACTOR 4E (EIF-4E) (EIF4E) (MRNA CAP-BINDING PROTEIN) (EIF-4F 25 KD SUBUNIT).//0.94:44:38//CAENORHABDITIS ELEGANS.//O61955
F-HEMBA1001912//!!!! ALU SUBFAMILY J WARNING ENTRY !!!!//8.7e-07:53:62//HOMO SAPIENS (HUMAN).//P39188
F-HEMBA1001913//GCN20 PROTEIN.//1.8e-21:68:60//SACCHAROMYCES CEREVISIAE (BAKER'S YEAST).//P43535
F-HEMBA1001915//KLEE PROTEIN (KCRB3 PROTEIN).//0.94:64:21//ESCHERICHIA COLI.//Q52280
F-HEMBA1001918
F-HEMBA1001921
F-HEMBA1001939//CHLOROPLAST 50S RIBOSOMAL PROTEIN L24.//1.0:47:31//ODONTELLA SINENSIS.//P49560
F-HEMBA1001940//!!!! ALU SUBFAMILY J WARNING ENTRY !!!!//0.0017:31:77//HOMO SAPIENS (HUMAN).//P39188
F-HEMBA1001942//HIBERNATION-ASSOCIATED PLASMA PROTEIN HP-27 PRECURSOR (HIBERNATOR- SPECIFIC BLOOD COMPLEX, 27 KD SUBUNIT).//1.0:77:28//TAMIAS ASIATICUS (CHIPMUNK).//Q06577
F-HEMBA1001945//HYPOTHETICAL 4.6 KD PROTEIN IN GP47-AGT INTERGENIC REGION (ORF E).//1.0:35:37//BACTERIOPHAGE T4.//P32269
F-HEMBA1001950//ATP SYNTHASE PROTEIN 8 (EC 3.6.1.34) (A6L).//0.43:18:44//DROSOPHILA YAKUBA (FRUIT FLY).//P03933
F-HEMBA1001960//HOMEOBOX PROTEIN HOX-C5 (HOX-3D) (CP11).//0.17:12:66//HOMO SAPIENS (HUMAN).//Q00444
F-HEMBA1001962//HYPOTHETICAL 9.0 KD PROTEIN IN ADH4 5'REGION.//1.0:30:36//SACCHAROMYCES CEREVISIAE (BAKER'S YEAST).//P53056
F-HEMBA1001964
F-HEMBA1001967//HYPOTHETICAL PROTEIN UL61.//0.027:111:36//HUMAN CYTOMEGALOVIRUS (STRAIN AD169).//P16818
F-HEMBA1001979
F-HEMBA1001987//HYPOTHETICAL 11.2 KD PROTEIN (ORF117).//1.0:83:32//ORGYIA PSEUDOTSUGATA MULTICAPSID POLYHEDROSIS VIRUS (OPMNPV).//O10356
F-HEMBA1001991//NEUROTOXIN 1 (TOXIN ATX-I).//0.99:31:45//ANEMONIA SULCATA (SNAKE-LOCKS SEA ANEMONE).//P01533
F-HEMBA1002003//GLYCERALDEHYDE 3-PHOSPHATE DEHYDROGENASE, TESTIS-SPECIFIC (EC 1.2.1.12) (GAPDH).//5.5e-07:109:32//MUS MUSCULUS (MOUSE).//Q64467
F-HEMBA1002008
F-HEMBA1002018//EC PROTEIN HOMOLOG 2 (FRAGMENT).//0.83:66:33//ARABIDOPSIS THALIANA (MOUSE-EAR CRESS).//Q42377
F-HEMBA1002022//INSULIN.//1.0:59:32//SQUALUS ACANTHIAS (SPINY DOGFISH).//P12704
F-HEMBA1002035//MONOCYTIC LEUKEMIA ZINC FINGER PROTEIN.//8.3e-15:64:40//HOMO SAPIENS (HUMAN).//Q92794
F-HEMBA1002039//HYPOTHETICAL PROLINE-RICH PROTEIN KIAA0269.//0.0070:70:40//HOMO SAPIENS (HUMAN).//Q92558
F-HEMBA1002049//!!!! ALU SUBFAMILY J WARNING ENTRY !!!!//1.5e-07:37:75//HOMO SAPIENS (HUMAN).//P39188
F-HEMBA1002084
F-HEMBA1002092//SPT23 PROTEIN.//0.12:208:26//SACCHAROMYCES CEREVISIAE (BAKER'S YEAST).//P35210
F-HEMBA1002100
F-HEMBA1002102//ANKYRIN.//1.4e-12:106:35//MUS MUSCULUS (MOUSE).//Q02357
F-HEMBA1002113//EARLY NODULIN 20 PRECURSOR (N-20).//0.073:155:32//MEDICAGO TRUNCATULA (BARREL MEDIC).//P93329
F-HEMBA1002119//MALE SPECIFIC SPERM PROTEIN MST84DB.//0.85:22:36//DROSOPHILA MELANOGASTER (FRUIT FLY).//Q01643
F-HEMBA1002125//GAG POLYPROTEIN [CONTAINS: CORE PROTEINS P15, P12, P30].//0.35:111:33//FELINE SARCOMA VIRUS (STRAIN SNYDER-THEILEN).//P03338
F-HEMBA1002139//HYPOTHETICAL 12.4 KD PROTEIN IN SEC17-QCR1 INTERGENIC REGION.//0.88:72:25//SACCHAROMYCES CEREVISIAE (BAKER'S YEAST).//P38192
F-HEMBA1002144
F-HEMBA1002150//THROMBOMODULIN (FETOMODULIN) (TM) (FRAGMENT).//4.8e-10:65:46//BOS TAURUS (BOVINE).//P06579
F-HEMBA1002151//A-AGGLUTININ ATTACHMENT SUBUNIT PRECURSOR.//0.24:146:28//SACCHAROMYCES CEREVISIAE (BAKER'S YEAST).//P32323
F-HEMBA1002153//NADH-UBIQUINONE OXIDOREDUCTASE CHAIN 4L (EC 1.6.5.3).//0.93:58:25//APIS MELLIFERA (HONEYBEE).//P34859
F-HEMBA1002160//!!!! ALU SUBFAMILY SP WARNING ENTRY !!!!//5.1e-21:94:65//HOMO SAPIENS (HUMAN).//P39193
F-HEMBA1002161//MYOSIN HEAVY CHAIN, CARDIAC MUSCLE BETA ISOFORM.//1.4e-51:180:56//SUS SCROFA (PIG).//P79293
F-HEMBA1002162//!!!! ALU SUBFAMILY SP WARNING ENTRY !!!!//4.1e-40:102:75//HOMO SAPIENS (HUMAN).//P39193
F-HEMBA1002166//!!!! ALU SUBFAMILY J WARNING ENTRY !!!!//1.8e-13:133:45//HOMO SAPIENS (HUMAN).//P39188
F-HEMBA1002177//ZINC FINGER PROTEIN 142 (KIAA0236) (HA4654).//0.0014:153:26//HOMO SAPIENS (HUMAN).//P52746
F-HEMBA1002185
F-HEMBA1002189//!!!! ALU SUBFAMILY SQ WARNING ENTRY !!!!//0.86:46:45//HOMO SAPIENS (HUMAN).//P39194
F-HEMBA1002191//MALE SPECIFIC SPERM PROTEIN MST84DC.//0.037:14:57//DROSOPHILA MELANOGASTER (FRUIT FLY).//Q01644
F-HEMBA1002199
F-HEMBA1002204
F-HEMBA1002212//DUAL SPECIFICITY MITOGEN-ACTIVATED PROTEIN KINASE
KINASE DSOR1 (EC 2.7.1.-) (DOWNSTREAM OF RAF) (MAPKK).//3.2e-13:201:30//DROSOPHILA MELANOGASTER (FRUIT FLY).//Q24324
F-HEMBA1002215//TESTIN 2 (TES2) [CONTAINS: TESTIN 1 (TES1)].//1.1e-62:147:84//MUS MUSCULUS (MOUSE).//P47226
F-HEMBA1002226//!!!! ALU SUBFAMILY J WARNING ENTRY !!!!//1.6e-26:168:44//HOMO SAPIENS (HUMAN).//P39188
F-HEMBA1002229//!!!! ALU SUBFAMILY SB1 WARNING ENTRY !!!!//6.8e-18:68:72//HOMO SAPIENS (HUMAN).//P39190
F-HEMBA1002237//EAMZP30-47 PROTEIN (FRAGMENT).//0.96:21:61//EIMERIA ACERVULINA.//P21959
F-HEMBA1002241//METALLOTHIONEIN (MT).//0.95:25:48//PARACENTROTUS LIVIDUS (COMMON SEA URCHIN).//P80367
F-HEMBA1002253//METALLOTHIONEIN-II (MT-II).//0.97:27:48//MESOCRICETUS AURATUS (GOLDEN HAMSTER).//P17808
F-HEMBA1002257
F-HEMBA1002265//MALE SPECIFIC SPERM PROTEIN MST84DC.//0.95:24:50//DROSOPHILA MELANOGASTER (FRUIT FLY).//Q01644
F-HEMBA1002267//NEURONAL PROTEIN 3.1 (P311 PROTEIN).//0.94:33:33//GALLUS GALLUS (CHICKEN).//Q90667
F-HEMBA1002270
F-HEMBA1002321//HYPOTHETICAL IMMUNITY REGION PROTEIN 14.//0.99:22:40//BACTERIOPHAGE PHI-105.//P10437
F-HEMBA1002328
F-HEMBA1002337
F-HEMBA1002341//P53-BINDING PROTEIN 53BP2 (FRAGMENT).//3.7e-55:109:96//MUS MUSCULUS (MOUSE).//Q62415
F-HEMBA1002348//PROBABLE E5 PROTEIN.//0.43:30:50//HUMAN PAPILLOMAVIRUS TYPE 35.//P27226
F-HEMBA1002349
F-HEMBA1002363//CHROMOSOME ASSEMBLY PROTEIN XCAP-E.//5.7e-105:278:71//XENOPUS LAEVIS (AFRICAN CLAWED FROG).//P50533
F-HEMBA1002381//!!!! ALU SUBFAMILY J WARNING ENTRY !!!!//3.3e-24:69:73//HOMO SAPIENS (HUMAN).//P39188
F-HEMBA1002389//EARLY NODULIN 20 PRECURSOR (N-20).//0.16:110:31//MEDICAGO TRUNCATULA (BARREL MEDIC).//P93329
F-HEMBA1002417//TIGHT JUNCTION PROTEIN ZO-1 (TIGHT JUNCTION PROTEIN 1).//2.6e-51:187:56//MUS MUSCULUS (MOUSE).//P39447
F-HEMBA1002419//PROLINE-RICH PEPTIDE P-B.//1.0:18:61//HOMO SAPIENS (HUMAN).//P02814
F-HEMBA1002430//HYPOTHETICAL 12.3 KD PROTEIN IN GAP1-NAP1 INTERGENIC REGION.//0.042:41:46//SACCHAROMYCES CEREVISIAE (BAKER'S YEAST).//P36140
F-HEMBA1002439//CHLOROPLAST 50S RIBOSOMAL PROTEIN L27 (FRAGMENT).//0.99:47:29//CALYPTROSPHAERA SPHAEROIDEA.//P41548
F-HEMBA1002458//OVARIAN GRANULOSA CELL 13.0 KD PROTEIN HGR74.//4.1e-24:109:55//HOMO SAPIENS (HUMAN).//Q00994
F-HEMBA1002460
F-HEMBA1002462//SALIVARY PROLINE-RICH PROTEIN II-1 (FRAGMENT).//0.00025:80:30//HOMO SAPIENS (HUMAN).//P81489
F-HEMBA1002469//PUTATIVE TUMOR SUPPRESSOR LUCA15.//0.0012:110:33//HOMO SAPIENS (HUMAN).//P52756
F-HEMBA1002475//PROLINE-RICH PROTEIN MP-3 (FRAGMENT).//0.37:106:33//MUS MUSCULUS (MOUSE).//P05143
F-HEMBA1002477//!!!! ALU SUBFAMILY SQ WARNING ENTRY !!!!//3.3e-34:96:71//HOMO SAPIENS (HUMAN).//P39194
F-HEMBA1002486
F-HEMBA1002495//LIGHT-MEDIATED DEVELOPMENT PROTEIN DET1.//2.9e-31:110:39//ARABIDOPSIS THALIANA (MOUSE-EAR CRESS).//P48732
F-HEMBA1002498//SFT2 PROTEIN.//1.0:54:35//SACCHAROMYCES CEREVISIAE (BAKER'S YEAST).//P38166
F-HEMBA1002503//!!!! ALU SUBFAMILY J WARNING ENTRY !!!!//3.0e-06:49:63//HOMO SAPIENS (HUMAN).//P39188
F-HEMBA1002508//!!!! ALU SUBFAMILY SX WARNING ENTRY !!!!//1.6e-22:169:44//HOMO SAPIENS (HUMAN).//P39195
F-HEMBA1002513//HYPOTHETICAL 89.8 KD PROTEIN F41H10.6 IN CHROMOSOME IV.//0.00017:79:35//CAENORHABDITIS ELEGANS.//Q20296
F-HEMBA1002515
F-HEMBA1002538//ATP SYNTHASE E CHAIN, MITOCHONDRIAL (EC 3.6.1.34).//1.0:53:37//SACCHAROMYCES CEREVISIAE (BAKER'S YEAST).//P81449
F-HEMBA1002542//!!!! ALU SUBFAMILY J WARNING ENTRY !!!!//5.7e-32:96:75//HOMO SAPIENS (HUMAN).//P39188
F-HEMBA1002547//AGRIN PRECURSOR.//2.5e-100:218:80//RATTUS NORVEGICUS (RAT).//P25304
F-HEMBA1002552//HEP27 PROTEIN (PROTEIN D).//9.5e-12:29:82//HOMO SAPIENS (HUMAN).//Q13268
F-HEMBA1002555//COLLAGEN ALPHA 1(III) CHAIN.//2.4e-15:207:36//BOS TAURUS (BOVINE).//P04258
F-HEMBA1002558//!!!! ALU SUBFAMILY SP WARNING ENTRY !!!!//1.0:34:50//HOMO SAPIENS (HUMAN).//P39193
F-HEMBA1002561//LINE-1 REVERSE TRANSCRIPTASE HOMOLOG.//1.5e-05:49:46//NYCTICEBUS COUCANG (SLOW LORIS).//P08548
F-HEMBA1002569//SINGLE-STRANDED DNA-BINDING PROTEIN P12.//0.97:60:33//BACTERIOPHAGE PRD1.//P17637
F-HEMBA1002583
F-HEMBA1002590//!!!! ALU SUBFAMILY J WARNING ENTRY !!!!//5.6e-15:54:55//HOMO SAPIENS (HUMAN).//P39188
F-HEMBA1002592//HISTIDINE-RICH PROTEIN.//0.99:39:28//PLASMODIUM FALCIPARUM (ISOLATE FCM17 / SENEGAL).//P14586
F-HEMBA1002609//SSM4 PROTEIN.//1.9e-12:135:29//SACCHAROMYCES CEREVISIAE (BAKER'S YEAST).//P40318
F-HEMBA1002621//CYTOCHROME B6-F COMPLEX 3.5 KD SUBUNIT (CYTOCHROME B6-F COMPLEX SUBUNIT 6).//1.0:20:55//ZEA MAYS (MAIZE).//P19445
F-HEMBA1002624//EXTENSIN PRECURSOR (CELL WALL HYDROXYPROLINE-RICH GLYCOPROTEIN).//0.0035:124:33//NICOTIANA TABACUM (COMMON TOBACCO).//P13983
F-HEMBA1002628
F-HEMBA1002629//IMMEDIATE-EARLY PROTEIN IE180.//0.84:80:36//PSEUDORABIES VIRUS (STRAIN KAPLAN) (PRV).//P33479
F-HEMBA1002645//!!!! ALU SUBFAMILY SP WARNING ENTRY !!!!//1.8e-16:57:68//HOMO SAPIENS (HUMAN).//P39193
F-HEMBA1002651
F-HEMBA1002659//CUTICLE COLLAGEN 2.//0.0077:77:38//CAENORHABDITIS ELEGANS.//P17656
F-HEMBA1002661//LINE-1 REVERSE TRANSCRIPTASE HOMOLOG.//2.3e-89:116:72//HOMO SAPIENS (HUMAN).//P08547
F-HEMBA1002666//BETA CRYSTALLIN A4.//0.18:58:44//GALLUS GALLUS (CHICKEN).//P49152
F-HEMBA1002678
F-HEMBA1002679//GLUTAMIC ACID-RICH PROTEIN PRECURSOR.//5.7e-06:219:27//PLASMODIUM FALCIPARUM (ISOLATE FC27 / PAPUA NEW GUINEA).//P13816
F-HEMBA1002688//SPIDROIN 2 (DRAGLINE SILK FIBROIN 2) (FRAGMENT).//1.1e-07:198:32//NEPHILA CLAVIPES (ORB SPIDER).//P46804
F-HEMBA1002696//COLLAGEN ALPHA 1(VII) CHAIN PRECURSOR (LONG-CHAIN COLLAGEN) (LC COLLAGEN).//0.16:158:33//HOMO SAPIENS (HUMAN).//Q02388
F-HEMBA1002703//HYPOTHETICAL BHLF1 PROTEIN.//0.78:147:29//EPSTEIN-BARR VIRUS (STRAIN B95-8) (HUMAN HERPESVIRUS 4).//P03181
F-HEMBA1002712//11.2 KD PROTEIN (ORF 103).//0.029:75:34//BACTERIOPHAGE PF1.//P25133
F-HEMBA1002716//50S RIBOSOMAL PROTEIN L28.//1.0:44:27//BACILLUS SUBTILIS.//P37807
F-HEMBA1002728//!!!! ALU SUBFAMILY SX WARNING ENTRY !!!!//5.4e-18:56:75//HOMO SAPIENS (HUMAN).//P39195
F-HEMBA1002730//HYPOTHETICAL PROTEIN MJ0316.//0.097:84:35//METHANOCOCCUS JANNASCHII.//Q57764
F-HEMBA1002742//APOLIPOPROTEIN C-III PRECURSOR (APO-CIII).//0.97:26:50//SUS SCROFA (PIG).//P27917
F-HEMBA1002746//CALPHOTIN.//0.35:65:35//DROSOPHILA MELANOGASTER (FRUIT FLY).//Q02910
F-HEMBA1002748//PLATELET GLYCOPROTEIN IB BETA CHAIN PRECURSOR (GP-IB BETA) (GPIBB).//1.0:74:32//MUS MUSCULUS (MOUSE).//P56400
F-HEMBA1002750//!!!! ALU SUBFAMILY J WARNING ENTRY !!!!//7.0e-15:49:75//HOMO SAPIENS (HUMAN).//P39188
F-HEMBA1002768//HYPOTHETICAL 72.2 KD PROTEIN C12C2.05C IN CHROMOSOME II.//0.00036:197:26//SCHIZOSACCHAROMYCES POMBE (FISSION YEAST).//Q09746
F-HEMBA1002770//UTEROGLOBIN PRECURSOR (BLASTOKININ).//023:88:27//ORYCTOLAGUS CUNICULUS (RABBIT).//P02779
F-HEMBA1002777//HOMEOBOX PROTEIN HOX-A4 (HOX-1.4) (MH-3).//0.00018:67:43//MUS MUSCULUS (MOUSE).//P06798
F-HEMBA1002779//HYPOTHETICAL 17.6 KD PROTEIN IN NPR1-RPS3 INTERGENIC REGION.//0.70:30:53//SACCHAROMYCES CEREVISIAE (BAKER'S YEAST).//P53880
F-HEMBA1002780//OLFACTORY RECEPTOR 3 (K10) (FRAGMENT).//1.0:31:45//MUS MUSCULUS (MOUSE).//Q60879
F-HEMBA1002794//HMG-Y RELATED PROTEIN B (SB16B PROTEIN) (FRAGMENT).//0.0044:66:37//GLYCINE MAX (SOYBEAN).//Q10370
F-HEMBA1002801
F-HEMBA1002810//HYPOTHETICAL 25.9 KD PROTEIN AH6.3 IN CHROMOSOME II.//0.0033:116:31//CAENORHABDITIS ELEGANS.//Q09202
F-HEMBA1002816//HYPOTHETICAL 47.1 KD PROTEIN C9G1.13C IN CHROMOSOME I.//1.0e-17:68:48//SCHIZOSACCHAROMYCES POMBE (FISSION YEAST).//O14308
F-HEMBA1002818//FIBULIN-2 PRECURSOR.//2.1e-27:92:44//MUS MUSCULUS (MOUSE).//P37889
F-HEMBA1002826//DNA-BINDING PROTEIN 65 (PROTEIN GP65).//0.28:46:34//BACTERIOPHAGE T4.//P16012
F-HEMBA1002833
F-HEMBA1002850//ATP SYNTHASE PROTEIN 8 (EC 3.6.1.34) (A6L).//1.0:27:37//METRIDIUM SENILE (BROWN SEA ANEMONE) (FRILLED SEA ANEMONE).//O47493
F-HEMBA1002863//PHOTOSYSTEM I REACTION CENTRE SUBUNIT IV (PHOTOSYSTEM I 8.1 KD PROTEIN) (P30 PROTEIN) (PSI-E).//0.84:37:43//SYNECHOCYSTIS SP. (STRAIN PCC 6803).//P12975
F-HEMBA1002876//OCTAPEPTIDE-REPEAT PROTEIN T2.//0.74:58:34//MUS MUSCULUS (MOUSE).//Q06666
F-HEMBA1002886
F-HEMBA1002896//HOMEOBOX PROTEIN HOX-B3 (HOX-2G) (HOX-2.7).//4.7e-05:84:35//HOMO SAPIENS (HUMAN).//P14651
F-HEMBA1002921//TAT PROTEIN (TRANSACTIVATING REGULATORY PROTEIN).//0.21:42:42//HUMAN IMUNODEFICIENCY VIRUS TYPE 1 (RF/HAT ISOLATE) (HIV-1).//P05908
F-HEMBA1002924//EC PROTEIN HOMOLOG 2 (FRAGMENT).//0.85:75:22//ARABIDOPSIS THALIANA (MOUSE-EAR CRESS).//Q42377
F-HEMBA1002934//!!!! ALU SUBFAMILY J WARNING ENTRY !!!!//3.4e-31:92:72//HOMO SAPIENS (HUMAN).//P39188
F-HEMBA1002935//GASTRULA ZINC FINGER PROTEIN XLCGF58.1 (FRAGMENT).//7.7e-06:187:29//XENOPUS LAEVIS (AFRICAN CLAWED FROG).//P18730
F-HEMBA1002937//SUPPRESSOR PROTEIN SRP40.//0.00031:150:24//SACCHAROMYCES CEREVISIAE (BAKER'S YEAST).//P32583
F-HEMBA1002939//ANKYRIN R (ANKYRINS 2.1 AND 2.2) (ERYTHROCYTE ANKYRIN).//5.2e-25:225:33//HOMO SAPIENS (HUMAN).//P16157
F-HEMBA1002944
F-HEMBA1002951//TRICHOHYALIN.//0.0011:220:24//HOMO SAPIENS (HUMAN).//Q07283
F-HEMBA1002954//PROBABLE E8 PROTEIN.//0.98:49:32//BOVINE PAPILLOMAVIRUS TYPE 4.//P08352
F-HEMBA1002968//ACCESSORY GLAND PEPTIDE PRECURSOR (PARAGONIAL PEPTIDE B).//0.93:41:34//DROSOPHILA SECHELLIA (FRUIT FLY).//O18417
F-HEMBA1002970//!!!! ALU SUBFAMILY SP WARNING ENTRY !!!!//0.00010:35:62//HOMO SAPIENS (HUMAN).//P39193
F-HEMBA1002971//INSULIN.//1.0:31:35//HYDROLAGUS COLLIEI (SPOTTED RATFISH) (PACIFIC RATFISH), AND CHIMAERA MONSTROSA (RABBIT FISH).//P09536 F-HEMBA1002973//CAMP-DEPENDENT 3',5'-CYCLIC PHOSPHODIESTERASE 4B (EC 3.1.4.17) (DPDE4).//3.0e-29:63:100//RATTUS NORVEGICUS (RAT).//P14646
F-HEMBA1002997//HYPOTHETICAL 106.5 KD PROTEIN IN CTT1-PRP31 INTERGENIC REGION.//1.0e-08:211:28//SACCHAROMYCES CEREVISIAE (BAKER'S YEAST).//P53253
F-HEMBA1002999//SUPPRESSOR PROTEIN SRP40.//0.026:175:23//SACCHAROMYCES CEREVISIAE (BAKER'S YEAST).//P32583
F-HEMBA1003021//!!!! ALU SUBFAMILY SQ WARNING ENTRY !!!!//1.3e-36:102:70//HOMO SAPIENS (HUMAN).//P39194
F-HEMBA1003033//HYPOTHETICAL 23.1 KD PROTEIN CY277.20C.//0.029:75:29//MYCOBACTERIUM TUBERCULOSIS.//P71779
F-HEMBA1003034//!!!! ALU SUBFAMILY SC WARNING ENTRY !!!!//6.3e-23:144:46//HOMO SAPIENS (HUMAN).//P39192
F-HEMBA1003035//HYPOTHETICAL 13.3 KD PROTEIN IN AROD-COMER INTERGENIC REGION.//0.99:55:30//BACILLUS SUBTILIS.//P54457
F-HEMBA1003037//DNA-BINDING PROTEIN INHIBITOR ID-4.//0.17:42:40//HOMO SAPIENS (HUMAN).//P47928
F-HEMBA1003041//DNA-BINDING P52/P100 COMPLEX, 100 KD SUBUNIT (FRAGMENTS).//0.69:28:46//HOMO SAPIENS (HUMAN).//P30808
F-HEMBA1003046//MITOCHONDRIAL PROCESSING PROTEASE BETA SUBUNIT PRECURSOR (EC 3.4.24.64) (BETA-MPP) (P-52).//7.9e-124:253:96//HOMO SAPIENS (HUMAN).//O75439
F-HEMBA1003064//NADH-UBIQUINONE OXIDOREDUCTASE CHAIN 5 (EC 1.6.5.3).//0.25:263:22//TRYPANOSOMA BRUCEI BRUCEI.//P04540
F-HEMBA1003067//HYPOTHETICAL 57.5 KD PROTEIN IN VMA7-RPS25A INTERGENIC REGION.//4.1e-05:189:30//SACCHAROMYCES CEREVISIAE (BAKER'S YEAST).//P53214
F-HEMBA1003071//CUTICLE COLLAGEN 40.//6.0e-07:126:38//CAENORHABDITIS ELEGANS.//P34804
F-HEMBA1003077//FIBROMODULIN PRECURSOR (FM) (COLLAGEN-BINDING 59 KD PROTEIN).//2.4e-12:139:34//HOMO SAPIENS (HUMAN).//Q06828
F-HEMBA1003078//RETROVIRUS-RELATED POL POLYPROTEIN [CONTAINS: REVERSE TRANSCRIPTASE (EC 2.7.7.49); ENDONUCLEASE].//7.2e-05:60:40//MUS MUSCULUS (MOUSE).//P11369
F-HEMBA1003079//PROTEIN Q300.//0.0012:16:87//MUS MUSCULUS (MOUSE).//Q02722
F-HEMBA1003083//!!!! ALU SUBFAMILY SB WARNING ENTRY !!!!//3.3e-32:95:75//HOMO SAPIENS (HUMAN).//P39189
F-HEMBA1003086
F-HEMBA1003096//PROTAMINE IA (IRIDINE IA).//0.36:20:40//SALMO IRIDEUS (RAINBOW TROUT).//P02328
F-HEMBA1003098//!!!! ALU SUBFAMILY J WARNING ENTRY !!!!//4.4e-09:43:72//HOMO SAPIENS (HUMAN).//P39188
F-HEMBA1003117//PUTATIVE CUTICLE COLLAGEN C09G5.5.//1.0:88:38//CAENORHABDITIS ELEGANS.//Q09456
F-HEMBA1003129//NADH-UBIQUINONE OXIDOREDUCTASE CHAIN 4L (EC 1.6.5.3).//0.61:63:25//APIS MELLIFERA (HONEYBEE).//P34859
F-HEMBA1003133//COLLAGEN ALPHA 2(VIII) CHAIN (ENDOTHELIAL COLLAGEN) (FRAGMENT).//0.48:79:37//HOMO SAPIENS (HUMAN).//P25067
F-HEMBA1003136//MANNOSE-1-PHOSPHATE GUANYLTRANSFERASE (EC 2.7.7.13) (ATP-MANNOSE-1- PHOSPHATE GUANYLYLTRANSFERASE) (NDP-HEXOSE PYROPHOSPHORYLASE).//3.6e-25:190:34//SACCHAROMYCES CEREVISIAE (BAKER'S YEAST).//P41940
F-HEMBA1003142
F-HEMBA1003148//HYPOTHETICAL 56.4 KD PROTEIN IN RPL30-CWH41 INTERGENIC REGION PRECURSOR.//0.068:171:23//SACCHAROMYCES CEREVISIAE (BAKER'S YEAST).//P53189
F-HEMBA1003166//!!!! ALU SUBFAMILY SC WARNING ENTRY !!!!//1.8e-13:54:66//HOMO SAPIENS (HUMAN).//P39192
F-HEMBA1003175//HYPOTHETICAL 57.5 KD PROTEIN IN VMA7-RPS25A INTERGENIC REGION.//0.015:147:31//SACCHAROMYCES CEREVISIAE (BAKER'S YEAST).//P53214
F-HEMBA1003179//PROBABLE TRNA (5-METHYLAMINOMETHYL-2-THIOURIDYLATE)-METHYLTRANSFERASE (EC 2.1.1.61).//2.6e-51:164:47//BACILLUS SUBTILIS.//O35020
F-HEMBA1003197
F-HEMBA1003199//HOMEOBOX PROTEIN HOX-A4 (HOX-1D) (HOX-1.4).//0.00049:83:38//HOMO SAPIENS (HUMAN).//Q00056
F-HEMBA1003202//SPERM PROTAMINE P1.//0.98:53:28//PLANIGALE GILESI (FLAT-SKULLED MARSUPIAL MOUSE).//O18747
F-HEMBA1003204//!!!! ALU SUBFAMILY SQ WARNING ENTRY !!!!//5.2e-22:42:80//HOMO SAPIENS (HUMAN).//P39194
F-HEMBA1003212//!!!! ALU SUBFAMILY SP WARNING ENTRY !!!!//1.6e-18:74:71//HOMO SAPIENS (HUMAN).//P39193
F-HEMBA1003220//!!!! ALU SUBFAMILY SQ WARNING ENTRY !!!!//3.3e-18:56:78//HOMO SAPIENS (HUMAN).//P39194
F-HEMBA1003222//HYPOTHETICAL 37.5 KD PROTEIN IN GNTR-HTPG INTERGENIC REGION.//0.0018:159:27//BACILLUS SUBTILIS.//P46327
F-HEMBA1003229//DIHYDRODIPICOLINATE SYNTHASE 1 PRECURSOR (EC 4.2.1.52) (DHDPS).//1.0:85:28//TRITICUM AESTIVUM (WHEAT).//P24846
F-HEMBA1003235//TROPOMYOSIN://8.3e-07:109:33//SCHIZOSACCHAROMYCES POMBE (FISSION YEAST).//Q02088
F-HEMBA1003250
F-HEMBA1003257//HYPOTHETICAL PROLINE-RICH PROTEIN (FRAGMENT).//1.5e-07:27:74//OWENIA FUSIFORMIS.//P21260
F-HEMBA1003273
F-HEMBA1003276
F-HEMBA1003278
F-HEMBA1003281//HOMEOBOX PROTEIN HOX-A4 (CHOX-1.4).//0.0053:116:36//GALLUS GALLUS (CHICKEN).//P17277
F-HEMBA1003286//DNA-DIRECTED RNA POLYMERASE SUBUNIT N (EC 2.7.7.6).//0.96:37:35//SULFOLOBUS ACIDOCALDARIUS.//P39472
F-HEMBA1003291//5'-AMP-ACTIVATED PROTEIN KINASE, CATALYTIC ALPHA-2 CHAIN (EC 2.7.1.-) (AMPK ALPHA-2 CHAIN) (FRAGMENT).//3.3e-15:68:39//SUS SCROFA (PIG).//Q28948
F-HEMBA1003296//PULMONARY SURFACTANT-ASSOCIATED PROTEIN B (SP-B) (6 KD PROTEIN) (PULMONARY SURFACTANT-ASSOCIATED PROTEOLIPID SPL(PHE)).//0.98:49:28//BOS TAURUS (BOVINE).//P15781
F-HEMBA1003304//MITOCHONDRIAL RIBOSOMAL PROTEIN S19.//0.99:36:30//PROTOTHECA WICKERHAMII.//P46750
F-HEMBA1003309//HYPOTHETICAL 7.9 KD PROTEIN.//0.69:54:37//VACCINIA VIRUS (STRAIN WR), AND VACCINIA VIRUS (STRAIN COPENHAGEN).//P04306
F-HEMBA1003314//MIXED LINEAGE KINASE 2 (EC 2.7.1.-) (FRAGMENT).//2.3e-06:143:22//HOMO SAPIENS (HUMAN).//Q02779
F-HEMBA1003322//!!!! ALU SUBFAMILY SQ WARNING ENTRY !!!!//1.5e-30:53:77//HOMO SAPIENS (HUMAN).//P39194
F-HEMBA1003327
F-HEMBA1003328//TAT PROTEIN (TRANSACTIVATING REGULATORY PROTEIN) (FRAGMENT).//0.53:21:42//HUMAN IMMUNODEFICIENCY VIRUS TYPE 1 (BH5 ISOLATE) (HIV-1).//P04612
F-HEMBA1003330//LONG NEUROTOXIN 3 (TOXIN VN2).//1.0:26:34//DENDROASPIS POLYLEPIS POLYLEPIS (BLACK MAMBA).//P25667
F-HEMBA1003348//!!!! ALU SUBFAMILY SQ WARNING ENTRY !!!!//6.5e-09:56:66//HOMO SAPIENS (HUMAN).//P39194
F-HEMBA1003369//ANTER-SPECIFIC PROLINE-RICH PROTEIN APG PRECURSOR.//0.0042:97:36//ARABIDOPSIS THALIANA (MOUSE-EAR CRESS).//P40602
F-HEMBA1003370//!!!! ALU SUBFAMILY J WARNING ENTRY !!!!//7.0e-18:99:53//HOMO SAPIENS (HUMAN).//P39188
F-HEMBA1003373
F-HEMBA1003376//!!!! ALU SUBFAMILY SB WARNING ENTRY !!!!//4.7e-16:60:75//HOMO SAPIENS (HUMAN).//P39189
F-HEMBA1003380//!!!! ALU SUBFAMILY J WARNING ENTRY !!!!//2.8e-10:50:68//HOMO SAPIENS (HUMAN).//P39188
F-HEMBA1003384
F-HEMBA1003395//PROBABLE E5 PROTEIN.//0.62:64:29//HUMAN PAPILLOMAVIRUS TYPE 16.//P06927
F-HEMBA1003402//HYPOTHETICAL 12.0 KD PROTEIN IN TUB1-CPR3 INTERGENIC REGION PRECURSOR.//0.89:74:33//SACCHAROMYCES CEREVISIAE (BAKER'S YEAST).//Q04521
F-HEMBA1003403//ACIDIC PROLINE-RICH PROTEIN PRECURSOR (CLONE PRP25) (FRAGMENT).//0.0010:69:33//RATTUS NORVEGICUS (RAT).//P10164
F-HEMBA1003408//WEB1 PROTEIN (PROTEIN TRANSPORT PROTEIN SEC31).//4.8e-06:93:25//SACCHAROMYCES CEREVISIAE (BAKER'S YEAST).//P38968
F-HEMBA1003417//PROCOLLAGEN ALPHA 1(II) CHAIN PRECURSOR [CONTAINS: CHONDROCALCIN].//0.0021:140:34//MUS MUSCULUS (MOUSE).//P28481
F-HEMBA1003418//SPLICING FACTOR, ARGININE/SERINE-RICH 4 (PRE-MRNA SPLICING FACTOR SRP75).//1.7e-14:188:33//HOMO SAPIENS (HUMAN).//Q08170
F-HEMBA1003433//DNA REPAIR PROTEIN XRS2.//1.0:88:35//SACCHAROMYCES CEREVISIAE (BAKER'S YEAST).//P33301
F-HEMBA1003447//ACIDIC PROLINE-RICH PROTEIN PRECURSOR (CLONE PRP25) (FRAGMENT).//0.0061:69:33//RATTUS NORVEGICUS (RAT).//P10164
F-HEMBA1003461//SPIDROIN 1 (DRAGLINE SILK FIBROIN 1) (FRAGMENT).//2.3e-09:239:33//NEPHILA CLAVIPES (ORB SPIDER).//P19837
F-HEMBA1003463//METALLOTHIONEIN-A (MTA) (FRAGMENT).//1.0:40:35//SPHAERECHINUS GRANULARIS (PURPLE SEA URCHIN).//Q26497
F-HEMBA1003480//FUSARIC ACID RESISTANCE PROTEIN FUSB.//0.0043:96:32//BURKHOLDERIA CEPACIA (PSEUDOMONAS CEPACIA).//P24127.
F-HEMBA1003528//36.4 KD PROLINE-RICH PROTEIN.//6.4e-15:167:33//LYCOPERSICON ESCULENTUM (TOMATO).//Q00451
F-HEMBA1003531//!!!! ALU SUBFAMILY SB WARNING ENTRY !!!!//1.2e-18:56:78//HOMO SAPIENS (HUMAN).//P39189
F-HEMBA1003538//COMPLEMENT C1R COMPONENT PRECURSOR (EC 3.4.21.41).//2.5e-28:136:47//HOMO SAPIENS (HUMAN).//P00736
F-HEMBA1003545//INSULIN GENE ENHANCER PROTEIN ISL-2 (ISLET-2).//9.2e-105:217:85//RATTUS NORVEGICUS (RAT).//P50480
F-HEMBA1003548
F-HEMBA1003555//HYPOTHETICAL 31.9 KD PROTEIN IN BET1-PAN1 INTERGENIC REGION.//8.7e-57:180:55//SACCHAROMYCES CEREVISIAE (BAKER'S YEAST).//P40558
F-HEMBA1003556//HYPOTHETICAL 19.2 KD PROTEIN IN COX-REP INTERGENIC REGION (ORF5) (ORF21).//0.53:97:25//BACTERIOPHAGE HP1.//P51706
F-HEMBA1003560//GUANINE NUCLEOTIDE-BINDING PROTEIN G(I)/G(S)/G(O) GAMMA-2 SUBUNIT (G GAMMA-I).//1.8e-32:71:100//BOS TAURUS (BOVINE).//P16874
F-HEMBA1003568//ZINC-FINGER PROTEIN RFP (RET FINGER PROTEIN).//4.1e-19:126:31//HOMO SAPIENS (HUMAN).//P14373
F-HEMBA1003569//METASTASIS-ASSOCIATED PROTEIN MTA1.//3.9e-83:143:74//HOMO SAPIENS (HUMAN).//Q13330
F-HEMBA1003571//HYPOTHETICAL 8.7 KD PROTEIN (READING FRAME D).//1.0:64:25//STAPHYLOCOCCUS AUREUS.//P03860
F-HEMBA1003579//CYTOTOXIN 1 (CYTOTOXIN V-II-1) (TOXIN V(II)1).//1.0:41:29//NAJA MELANOLEUCA (FOREST COBRA) (BLACK-LIPPED COBRA).//P01448
F-HEMBA1003581//TALIN.//3.7e-36:52:98//MUS MUSCULUS (MOUSE).//P26039
F-HEMBA1003591//CHLOROPLAST 28 KD RIBONUCLEOPROTEIN PRECURSOR (28RNP).//1.6e-05:91:31//NICOTIANA SYLVESTRIS (WOOD TOBACCO).//P19682
F-HEMBA1003595//HYPOTHETICAL 12.0 KD PROTEIN IN DST1-HEM2 INTERGENIC REGION.//1.0:55:32//SACCHAROMYCES CEREVISIAE (BAKER'S YEAST).//P53182
F-HEMBA1003597
F-HEMBA1003598//T-CELL RECEPTOR BETA CHAIN PRECURSOR (ANA 11)//4.9e-10:85:41//ORYCTOLAGUS CUNICULUS (RABBIT).//P06333
F-HEMBA1003615//PUTATIVE MINOR COAT PROTEIN (ORF43).//0.086:10:70//BACTERIOPHAGE PHI-LF.//Q07482
F-HEMBA1003617//HYPOTHETICAL 36.8 KD PROTEIN C26A3.16 IN CHROMOSOME I.//4.4e-13:58:48//SCHIZOSACCHAROMYCES POMBE (FISSION YEAST).//Q10169
F-HEMBA1003621//LONG NEUROTOXIN 1 (NEUROTOXIN A).//0.096:40:37//OPHIOPHAGUS HANNAH (KING COBRA) (NAJA HANNAH).//P01387
F-HEMBA1003622
F-HEMBA1003630
F-HEMBA1003637//!!!! ALU SUBFAMILY J WARNING ENTRY !!!!//2.4e-13:47:74//HOMO SAPIENS (HUMAN).//P39188
F-HEMBA1003640//!!!! ALU SUBFAMILY SP WARNING ENTRY !!!!//0.87:25:64//HOMO SAPIENS (HUMAN).//P39193
F-HEMBA1003645//HYPOTHETICAL 40.4 KD TRP-ASP REPEATS CONTAINING PROTEIN C14B1.4 IN CHROMOSOME III.//1.8e-10:157:26//CAENORHABDITIS ELEGANS.//Q17963
F-HEMBA1003646//SERINE-ARGININE PROTEIN 55 (SRP55) (ENHANCER OF DEFORMED) (52-KD BRACKETING PROTEIN) (B52 PROTEIN).//4.9e-05:207:27//DROSOPHILA MELANOGASTER (FRUIT FLY).//P26686
F-HEMBA1003656
F-HEMBA1003662//PROLINE-RICH PEPTIDE P-B.//0.57:17:52//HOMO SAPIENS (HUMAN).//P02814
F-HEMBA1003667//!!!! ALU SUBFAMILY SQ WARNING ENTRY !!!!//6.0e-16:43:72//HOMO SAPIENS (HUMAN).//P39194
F-HEMBA1003679
F-HEMBA1003680//PUTATIVE AMINOPEPTIDASE ZK353.6 IN CHROMOSOME III (EC 3.4.11.-).//3.9e-08:137:27//CAENORHABDITIS ELEGANS.//P34629
F-HEMBA1003684//ZINC FINGER PROTEIN 151 (POLYOMAVIRUS LATE INITIATOR PROMOTER BINDING PROTEIN) (LP-1) (ZINC FINGER PROTEIN Z13).//2.1e-20:127:40//MUS MUSCULUS (MOUSE).//Q60821
F-HEMBA1003690//HYPOTHETICAL PROTEIN KIAA0288 (HA6116).//3.0e-85:201:78//HOMO SAPIENS (HUMAN).//P56524
F-HEMBA1003692//CELL DIVISION CONTROL PROTEIN 1.//0.13:69:30//SACCHAROMYCES CEREVISIAE (BAKER'S YEAST).//P40986
F-HEMBA1003711//CARCINOEMBRYONIC ANTIGEN PRECURSOR (CEA) (MECONIUM ANTIGEN 100) (CD66E ANTIGEN).//0.021:153:26//HOMO SAPIENS (HUMAN).//P06731
F-HEMBA1003714//ABAECIN.//0.99:34:32//BOMBUS PASCUORUM.//P81463
F-HEMBA1003715
F-HEMBA1003720//LINE-1 REVERSE TRANSCRIPTASE HOMOLOG.//5.4e-34:155:56//HOMO SAPIENS (HUMAN).//P08547
F-HEMBA1003725//LINE-1 REVERSE TRANSCRIPTASE HOMOLOG.//1.3e-27:181:41//HOMO SAPIENS (HUMAN).//P08547
F-HEMBA1003729//PTB-ASSOCIATED SPLICING FACTOR (PSF).//0.0037:103:33//HOMO SAPIENS (HUMAN).//P23246
F-HEMBA1003733//LINE-1 REVERSE TRANSCRIPTASE HOMOLOG.//5.0e-54:210:58//HOMO SAPIENS (HUMAN).//P08547
F-HEMBA1003742//MALE SPECIFIC SPERM PROTEIN MST84DB.//0.066:72:33//DROSOPHILA MELANOGASTER (FRUIT FLY).//Q01643
F-HEMBA1003758
F-HEMBA1003760//HYPOXIA-INDUCIBLE FACTOR 1 ALPHA (HIF-1 ALPHA) (ARNT INTERACTING PROTEIN).//1.5e-51:220:52//MUS MUSCULUS (MOUSE).//Q61221
F-HEMBA1003773
F-HEMBA1003783
F-HEMBA1003784
F-HEMBA1003799//SHORT NEUROTOXIN 1 (TOXIN AA C).//0.95:27:37//ACANTHOPHIS ANTARCTICUS (COMMON DEATH ADDER).//P01434
F-HEMBA1003803//GAG POLYPROTEIN [CONTAINS: CORE PROTEINS P15, P12, P30].//0.46:96:34//FELINE SARCOMA VIRUS (STRAIN SNYDER-THEILEN).//P03338
F-HEMBA1003804//DNA-BINDING P52/P100 COMPLEX, 100 KD SUBUNIT (FRAGMENTS).//0.019:30:50//HOMO SAPIENS (HUMAN).//P30808
F-HEMBA1003805//HYPOTHETICAL 75.0 KD PROTEIN B0280.11 IN CHROMOSOME III.//1.8e-20:109:47//CAENORHABDITIS ELEGANS.//P42083
F-HEMBA1003807
F-HEMBA1003827//HYPOTHETICAL PROLINE-RICH PROTEIN (FRAGMENT).//2.1e-09:23:78//OWENIA FUSIFORMIS.//P21260
F-HEMBA1003836//MOB1 PROTEIN (MPS1 BINDER 1).//2.0e-31:134:52//SACCHAROMYCES CEREVISIAE (BAKER'S YEAST).//P40484
F-HEMBA1003838//!!!! ALU SUBFAMILY SC WARNING ENTRY !!!!//3.9e-22:39:76//HOMO SAPIENS (HUMAN).//P39192
F-HEMBA1003856
F-HEMBA1003864//HYPOTHETICAL 39.4 KD PROTEIN IN MET1-SIS2 INTERGENIC REGION.//1.5e-15:194:30//SACCHAROMYCES CEREVISIAE (BAKER'S YEAST).//P36151
F-HEMBA1003866//PROTEIN A39.//0.0027:72:33//VACCINIA VIRUS (STRAIN COPENHAGEN).//P21062
F-HEMBA1003879//80 KD NUCLEAR CAP BINDING PROTEIN (NCBP 80 KD SUBUNIT) (CBP80).//2.9e-16:22:100//HOMO SAPIENS (HUMAN).//Q09161
F-HEMBA1003880//ATP SYNTHASE PROTEIN 8 (EC 3.6.1.34) (A6L).//0.99:39:38//FELIS SILVESTRIS CATUS (CAT).//P48896
F-HEMBA1003885//!!!! ALU SUBFAMILY SP WARNING ENTRY !!!!//3.5e-28:47:76//HOMO SAPIENS (HUMAN).//P39193
F-HEMBA1003893//HYPOTHETICAL 27.8 KD PROTEIN IN VMA7-RPS25A INTERGENIC REGION.//1.7e-57:215:51//SACCHAROMYCES CEREVISIAE (BAKER'S YEAST).//P53215
F-HEMBA1003902
F-HEMBA1003908
F-HEMBA1003926//!!!! ALU SUBFAMILY J WARNING ENTRY !!!!//4.3e-10:60:63//HOMO SAPIENS (HUMAN).//P39188
F-HEMBA1003937//!!!! ALU SUBFAMILY SQ WARNING ENTRY !!!!//8.1e-29:68:64//HOMO SAPIENS (HUMAN).//P39194
F-HEMBA1003939//PROTEIN Q300.//0.0025:24:62//MUS MUSCULUS (MOUSE).//Q02722
F-HEMBA1003942//EXCITATORY INSECT TOXIN BJXTR-IT PRECURSOR (BJ-XTRIT).//0.084:67:31//BUTHOTUS JUDAICUS (SCORPION) (HOTTENTOTTA JUDAICA).//P56637
F-HEMBA1003950//HYPOTHETICAL 8.1 KD PROTEIN IN SPEA-METK INTERGENIC REGION (O71).//0.95:26:34//ESCHERICHIA COLI.//P46878
F-HEMBA1003953//ZINC FINGER PROTEIN MFG-1 (ZINC FINGER PROTEIN 58) (FRAGMENT).//2.5e-17:89:46//MUS MUSCULUS (MOUSE).//P16372
F-HEMBA1003958//LINE-1 REVERSE TRANSCRIPTASE HOMOLOG.//4.2e-23:43:76//HOMO SAPIENS (HUMAN).//P08547
F-HEMBA1003959
F-HEMBA1003976//HYPOTHETICAL PROTEIN KIAA0076 (HA0936).//0.99:88:28//HOMO SAPIENS (HUMAN).//Q14999
F-HEMBA1003978//SMALL PROLINE RICH PROTEIN II (SPR-II) (CLONE 930).//0.98:19:57//HOMO SAPIENS (HUMAN).//P22531
F-HEMBA1003985//LYSYL-TRNA SYNTHETASE (EC 6.1.1.6) (LYSINE--TRNA LIGASE) (LYSRS) (FRAGMENT).//1.0:40:32//MYCOBACTERIUM LEPRAE.//P46861
F-HEMBA1003987//HYPOTHETICAL PROTEIN UL66.//0.27:65:33//HUMAN CYTOMEGALOVIRUS (STRAIN AD169).//P16822
F-HEMBA1003989//MALE SPECIFIC SPERM PROTEIN MST84DB.//5.2e-05:64:40//DROSOPHILA MELANOGASTER (FRUIT FLY).//Q01643
F-HEMBA1004000//PROTEIN Q300.//0.00042:17:82//MUS MUSCULUS (MOUSE).//Q02722
F-HEMBA1004011//ALPHA-TYPE CALCITONIN GENE-RELATED PEPTIDE PRECURSOR (CGRP-1).//0.47:106:32//HOMO SAPIENS (HUMAN).//P06881
F-HEMBA1004012//ATP SYNTHASE PROTEIN 9, MITOCHONDRIAL (EC 3.6.1.34) (LIPID-BINDING PROTEIN).//0.96:36:33//PARAMECIUM TETRAURELIA.//P16001
F-HEMBA1004015//HYPOTHETICAL 29.3 KD PROTEIN B0280.6 IN CHROMOSOME III.//0.00018:90:34//CAENORHABDITIS ELEGANS.//P41997
F-HEMBA1004024//!!!! ALU SUBFAMILY SQ WARNING ENTRY !!!!//5.1e-34:75:80//HOMO SAPIENS (HUMAN).//P39194
F-HEMBA1004038
F-HEMBA1004042
F-HEMBA1004045//40S RIBOSOMAL PROTEIN S27A.//1.0:20:55//ASPARAGUS OFFICINALIS (GARDEN ASPARAGUS).//P31753
F-HEMBA1004048//PROLINE-RICH PROTEIN MP-3 (FRAGMENT).//1.3e-06:158:35//MUS MUSCULUS (MOUSE).//P05143
F-HEMBA1004049//32 KD HEAT SHOCK PROTEIN (4-1 PROTEIN).//0.098:106:32//DICTYOSTELIUM DISCOIDEUM (SLIME MOLD).//P54658
F-HEMBA1004055//HYPOTHETICAL PROTEIN HI0258/259.//0.87:133:23//HAEMOPHILUS INFLUENZAE.//P43974
F-HEMBA1004056//!!!! ALU SUBFAMILY SB2 WARNING ENTRY !!!!//3.3e-25:39:64//HOMO SAPIENS (HUMAN).//P39191
F-HEMBA1004074//!!!! ALU SUBFAMILY J WARNING ENTRY !!!!//9.9e-08:35:68//HOMO SAPIENS (HUMAN).//P39188
F-HEMBA1004086
F-HEMBA1004097//IMMEDIATE-EARLY PROTEIN IE4 (IE68) (FRAGMENT)//0.71:95:35//HERPES SIMPLEX VIRUS (TYPE 2).//P14379
F-HEMBA1004111//!!!! ALU SUBFAMILY J WARNING ENTRY !!!!//4.7e-26:84:64//HOMO SAPIENS (HUMAN).//P39188
F-HEMBA1004131//SEPTIN 2 HOMOLOG (FRAGMENT).//2.8e-34:108:63//HOMO SAPIENS (HUMAN).//Q14141
F-HEMBA1004132//HYPOTHETICAL PROTEIN HI1736.//1.0:44:34//HAEMOPHILUS INFLUENZAE.//P44300
F-HEMBA1004133//HYPOTHETICAL 8.5 KD PROTEIN CY274.40C.//0.89:21:57//MYCOBACTERIUM TUBERCULOSIS.//Q10826
F-HEMBA1004138//EARLY NODULIN 75 (N-75) (NGM-75) (FRAGMENT).//0.016:39:41//MEDICAGO SATIVA (ALFALFA).//P11728
F-HEMBA1004143//CYTOCHROME C OXIDASE POLYPEPTIDE VIII PRECURSOR (EC 1.9.3.1).//0.93:34:29//SACCHAROMYCES CEREVISIAE (BAKER'S YEAST).//P04039
F-HEMBA1004146//BASIC PROLINE-RICH PEPTIDE P-E (IB-9).//0.63:52:36//HOMO SAPIENS (HUMAN).//P02811
F-HEMBA1004150//METALLOTHIONEIN-II (MT-II).//1.0:20:45//MUS MUSCULUS (MOUSE).//P02798
F-HEMBA1004164//!!!! ALU SUBFAMILY SX WARNING ENTRY !!!!//3.0e-13:57:71//HOMO SAPIENS (HUMAN).//P39195
F-HEMBA1004168//V-TYPE SODIUM ATP SYNTHASE SUBUNIT F (EC 3.6.1.34) (NA(+)-TRANSLOCATING ATPASE SUBUNIT F).//0.00035:90:34//ENTEROCOCCUS HIRAE.//P43437
F-HEMBA1004199//HYPOTHETICAL HELICASE K12H4.8 IN CHROMOSOME III.//5.1e-14:115:31//CAENORHABDITIS ELEGANS.//P34529
F-HEMBA1004200
F-HEMBA1004202//YPT1-RELATED PROTEIN 1.//2.5e-24:96:52//SCHIZOSACCHAROMYCES POMBE (FISSION YEAST).//P11620
F-HEMBA1004203//!!!! ALU SUBFAMILY SP WARNING ENTRY !!!!//2.2e-09:48:64//HOMO SAPIENS (HUMAN).//P39193
F-HEMBA1004207//HYPOTHETICAL 8.7 KD PROTEIN IN RPL22-RPL23 INTERGENIC REGION (ORF70).//0.98:51:33//ASTASIA LONGA (EUGLENOPHYCEAN ALGA).//P34779
F-HEMBA1004225//METALLOTHIONEIN-II.//1.0:30:33//CANDIDA GLABRATA (YEAST) (TORULOPSIS GLABRATA).//P15114
F-HEMBA1004227//PUTATIVE PROTEIN PHOSPHATASE 2C (EC 3.1.3.16) (PP2C) (KIAA0015).//5.9e-06:109:33//HOMO SAPIENS (HUMAN).//P49593
F-HEMBA1004238//VERY HYPOTHETICAL XYLU PROTEIN.//0.98:39:38//ESCHERICHIA COLI.//P05056
F-HEMBA1004241//SOX-13 PROTEIN (FRAGMENT).//0.66:36:38//MUS MUSCULUS (MOUSE).//Q04891
F-HEMBA1004246
F-HEMBA1004248//INSULIN-INDUCED GROWTH RESPONSE PROTEIN CL-6 (IMMEDIATE-EARLY PROTEIN CL-6).//1.0e-43:98:84//RATTUS NORVEGICUS (RAT).//Q08755
F-HEMBA1004264//SPIDROIN 2 (DRAGLINE SILK FIBROIN 2) (FRAGMENT).//0.014:160:28//NEPHILA CLAVIPES (ORB SPIDER).//P46804
F-HEMBA1004267//!!!! ALU SUBFAMILY SB WARNING ENTRY !!!!//1.8e-52:56:83//HOMO SAPIENS (HUMAN).//P39189
F-HEMBA1004272
F-HEMBA1004274//HYPOTHETICAL 13.0 KD PROTEIN F59B2.10 IN CHROMOSOME III.//0.00084:33:54//CAENORHABDITIS ELEGANS.//P34485
F-HEMBA1004275//HYPOTHETICAL 56.5 KD PROTEIN IN CAJ1-HOM3 INTERGENIC REGION)/9.3e-06:125:27//SACCHAROMYCES CEREVISIAE (BAKER'S YEAST).//P40034
F-HEMBA1004276//BETA-ADAPTIN 1 (PLASMA MEMBRANE ADAPTOR HA2/AP2 ADAPTIN BETA SUBUNIT) (CLATHRIN ASSEMBLY PROTEIN COMPLEX 2 BETA LARGE CHAIN) (AP105A).//3.7e-30:239:32//HOMO SAPIENS (HUMAN).//Q10567
F-HEMBA1004286//CUTICLE COLLAGEN 34.//0.0027:71:38//CAENORHABDITIS ELEGANS.//P34687
F-HEMBA1004289//PTR3 PROTEIN (SSY3 PROTEIN).//1.0:76:28//SACCHAROMYCES CEREVISIAE (BAKER'S YEAST).//P43606
F-HEMBA1004295//DNA-BINDING P52/P100 COMPLEX, 100 KD SUBUNIT (FRAGMENTS).//0.075:58:39//HOMO SAPIENS (HUMAN).//P30808
F-HEMBA1004306//HYPOTHETICAL 29.3 KD PROTEIN (ORF92).//0.020:132:30//ORGYIA PSEUDOTSUGATA MULTICAPSID POLYHEDROSIS VIRUS (OPMNPV).//O10341
F-HEMBA1004312//EARLY PROTEIN I73R.//0.99:65:32//AFRICAN SWINE FEVER VIRUS (STRAIN BA71V) (ASFV).//P27946
F-HEMBA1004321//ZINC FINGER PROTEIN 90 (ZFP-90) (ZINC FINGER PROTEIN NK10).//4.3e-43:133:44//MUS MUSCULUS (MOUSE).//Q61967
F-HEMBA1004323
F-HEMBA1004327//SMALL PROLINE-RICH PROTEIN 2-1.//0.027:48:43//HOMO SAPIENS (HUMAN).//P35326
F-HEMBA1004330//HOMEOBOX PROTEIN ENGRAILED-1 (HU-EN-1).//0.46:70:34//HOMO SAPIENS (HUMAN).//Q05925
F-HEMBA1004334//LINE-1 REVERSE TRANSCRIPTASE HOMOLOG.//7.7e-05:83:34//HOMO SAPIENS (HUMAN).//P08547
F-HEMBA1004335//!!!! ALU SUBFAMILY SX WARNING ENTRY !!!!//2.0e-24:41:80//HOMO SAPIENS (HUMAN).//P39195
F-HEMBA1004341//PROLINE-RICH PROTEIN MP-3 (FRAGMENT).//2.8e-06:148:35//MUS MUSCULUS (MOUSE).//P05143
F-HEMBA1004353//!!!! ALU SUBFAMILY SX WARNING ENTRY !!!!//2.2e-29:57:80//HOMO SAPIENS (HUMAN).//P39195
F-HEMBA1004354//CHL1 PROTEIN.//0.017:40:40//SACCHAROMYCES CEREVISIAE (BAKER'S YEAST).//P22516
F-HEMBA1004356
F-HEMBA1004366//LINE-1 REVERSE TRANSCRIPTASE HOMOLOG.//0.00045:49:46//HOMO SAPIENS (HUMAN).//P08547
F-HEMBA1004372//VERY HYPOTHETICAL 20.6 KD PROTEIN C56F8.15 IN CHROMOSOME I.//1.0:125:28//SCHIZOSACCHAROMYCES POMBE (FISSION YEAST).//Q10263
F-HEMBA1004389//HYPOTHETICAL 113.1 KD PROTEIN IN PRE5-FET4 INTERGENIC REGION.//0.76:170:25//SACCHAROMYCES CEREVISIAE (BAKER'S YEAST).//Q04893
F-HEMBA1004394
F-HEMBA1004396//LINE-1 REVERSE TRANSCRIPTASE HOMOLOG.//1.2e-10:72:51//HOMO SAPIENS (HUMAN).//P08547
F-HEMBA1004405
F-HEMBA1004408//PEPTIDYL-PROLYL CIS-TRANS ISOMERASE 10 (EC 5.2.1.8) (PPIASE) (ROTAMASE) (CYCLOPHILIN-10).//2.7e-29:146:48//CAENORHABDITIS ELEGANS.//P52017
F-HEMBA1004429//!!!! ALU SUBFAMILY SB2 WARNING ENTRY !!!!//0.0019:47:59//HOMO SAPIENS (HUMAN).//P39191
F-HEMBA1004433//!!!! ALU SUBFAMILY SC WARNING ENTRY !!!!//1.1e-20:47:68//HOMO SAPIENS (HUMAN).//P39192
F-HEMBA1004460//!!!! ALU SUBFAMILY SP WARNING ENTRY !!!!//6.2e-64:134:69//HOMO SAPIENS (HUMAN).//P39193
F-HEMBA1004461//METALLOTHIONEIN-LIKE PROTEIN 1.//1.0:39:35//PISUM SATIVUM (GARDEN PEA).//P20830
F-HEMBA1004479//HYPOXIA-INDUCIBLE FACTOR 1 ALPHA (HIF-1 ALPHA) (ARNT INTERACTING PROTEIN).//9.7e-43:101:48//MUS MUSCULUS (MOUSE).//Q61221
F-HEMBA1004482//ATP SYNTHASE PROTEIN 8 (EC 3.6.1.34).//1.0:41:36//CANDIDA GLABRATA (YEAST) (TORULOPSIS GLABRATA).//P05040
F-HEMBA1004499//TUBULIN BETA CHAIN.//0.00021:55:36//CAENORHABDITIS ELEGANS.//P52275
F-HEMBA1004502
F-HEMBA1004506//HYPOTHETICAL PROTEIN ORF-1137.//5.3-11:119:35//MUS MUSCULUS (MOUSE).//P11260
F-HEMBA1004507//SPLICEOSOME ASSOCIATED PROTEIN 62 (SAP 62) (SF3A66).//0.00072:90:37//HOMO SAPIENS (HUMAN).//Q15428
F-HEMBA1004509//HYPOTHETICAL 52.2 KD PROTEIN IN MPR1-GCN20 INTERGENIC REGION.//6.3e-28:169:42//SACCHAROMYCES CEREVISIAE (BAKER'S YEAST).//P43589
F-HEMBA1004534//ENDOTHELIAL ACTIN-BINDING PROTEIN (ABP-280) (NONMUSCLE FILAMIN) (FILAMIN 1).//1.3e-80:226:66//HOMO SAPIENS (HUMAN).//P21333
F-HEMBA1004538//HYPOTHETICAL PROTEIN MJ0764.//0.96:28:35//METHANOCOCCUS JANNASCHII.//Q58174
F-HEMBA1004542//METALLOTHIONEIN (MT).//0.78:36:41//GADUS MORHUA (ATLANTIC COD).//P51902
F-HEMBA1004554
F-HEMBA1004560//HYPOTHETICAL PROTEIN KIAA0281 (HA6725).//4.2e-15:56:69//HOMO SAPIENS (HUMAN).//Q92556
F-HEMBA1004573//CIRCUMSPOROZOITE PROTEIN PRECURSOR (CS).//0.65:31:58//PLASMODIUM BERGHEI.//P06915
F-HEMBA1004577//!!!! ALU SUBFAMILY SX WARNING ENTRY !!!!//3.9e-08:35:80//HOMO SAPIENS (HUMAN).//P39195
F-HEMBA1004586//!!!! ALU SUBFAMILY SQ WARNING ENTRY !!!!//6.6e-08:64:54//HOMO SAPIENS (HUMAN).//P39194
F-HEMBA1004596//HETEROGENEOUS NUCLEAR RIBONUCLEOPROTEIN C (HNRNP C) (HNRNP CORE PROTEIN C) (FRAGMENT).//0.00057:88:31//RATTUS NORVEGICUS (RAT).//P17132
F-HEMBA1004604//COLLAGEN ALPHA 2(XI) CHAIN PRECURSOR (FRAGMENT).//0.045:37:45//MUS MUSCULUS (MOUSE).//Q64739
F-HEMBA1004610//!!!! ALU SUBFAMILY J WARNING ENTRY !!!!//4.3e-11:73:54//HOMO SAPIENS (HUMAN).//P39188
F-HEMBA1004617
F-HEMBA1004629
F-HEMBA1004631//HYPOTHETICAL 7.8 KD PROTEIN IN WAPA-LICT INTERGENIC REGION.//1.0:36:38//BACILLUS SUBTILIS.//P42303
F-HEMBA1004632//PHOTOSYSTEM I REACTION CENTRE SUBUNIT X PRECURSOR (LIGHT-HARVESTING 8.0 KD POLYPEPTIDE).//0.86:48:35//SYNECHOCOCCUS ELONGATUS NAEGELI.//P20453
F-HEMBA1004637//HYPOTHETICAL 83.6 KD PROTEIN R05D3.2 IN CHROMOSOME III.//1.7e-32:159:42//CAENORHABDITIS ELEGANS.//P34535
F-HEMBA1004638//HYPOTHETICAL PROLINE-RICH PROTEIN (FRAGMENT).//2.8e-06:50:46//OWENIA FUSIFORMIS.//P21260
F-HEMBA1004666//TOXIN S6C4.//1.0:36:30//DENDROASPIS JAMESONI KAIMOSAE (EASTERN JAMESON'S MAMBA).//P25682
F-HEMBA1004669//SPLICING FACTOR, ARGININE/SERINE-RICH 4 (PRE-MRNA SPLICING FACTOR SRP75).//1.6e-12:105:42//HOMO SAPIENS (HUMAN).//Q08170
F-HEMBA1004670//PROCOLLAGEN ALPHA 1(I) CHAIN PRECURSOR.//2.5e-06:62:45//HOMO SAPIENS (HUMAN).//P02452
F-HEMBA1004672//HYPOTHETICAL PROTEIN MJ0437.//0.95:37:29//METHANOCOCCUS JANNASCHII.//Q57879
F-HEMBA1004693//MYOSIN HEAVY CHAIN, NONMUSCLE TYPE B (CELLULAR MYOSIN HEAVY CHAIN, TYPE B) (NMMHC-B).//0.00035:217:23//HOMO SAPIENS (HUMAN).//P35580
F-HEMBA1004697//IMMUNOGLOBULIN G BINDING PROTEIN H PRECURSOR (PROTEIN H).//0.058:118:30//STREPTOCOCCUS PYOGENES.//P50470
F-HEMBA1004705//!!!! ALU SUBFAMILY J WARNING ENTRY !!!!//6.8e-09:43:72//HOMO SAPIENS (HUMAN).//P39188
F-HEMBA1004709//!!!! ALU SUBFAMILY SB WARNING ENTRY !!!!//8.8e-18:50:84//HOMO SAPIENS (HUMAN).//P39189
F-HEMBA1004711//ETS-RELATED PROTEIN 71 (ETS TRANSLOCATION VARIANT 2).//0.0027:148:30//HOMO SAPIENS (HUMAN).//000321
F-HEMBA1004725//CUTICLE COLLAGEN 2.//0.0051:41:41//CAENORHABDITIS ELEGANS.//P17656
F-HEMBA1004730//LINE-1 REVERSE TRANSCRIPTASE HOMOLOG.//1.4e-22:210:37//HOMO SAPIENS (HUMAN).//P08547
F-HEMBA1004733//!!!! ALU SUBFAMILY J WARNING ENTRY !!!!//4.7e-07:50:62//HOMO SAPIENS (HUMAN).//P39188
F-HEMBA1004734//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//9.9e-39:143:52//ARABIDOPSIS THALIANA (MOUSE-EAR CRESS).//P42743
F-HEMBA1004736//LINE-1 REVERSE TRANSCRIPTASE HOMOLOG.//4.1e-60:210:61//HOMO SAPIENS (HUMAN).//P08547
F-HEMBA1004748
F-HEMBA1004751//!!!! ALU SUBFAMILY J WARNING ENTRY !!!!//4.8e-20:88:63//HOMO SAPIENS (HUMAN).//P39188
F-HEMBA1004752//SKIN SECRETORY PROTEIN XP2 PRECURSOR (APEG PROTEIN).//0.0043:126:34//XENOPUS LAEVIS (AFRICAN CLAWED FROG).//P17437
F-HEMBA1004753//!!!! ALU SUBFAMILY SP WARNING ENTRY !!!!//7.8e-28:47:78//HOMO SAPIENS (HUMAN).//P39193
F-HEMBA1004756//HYPOTHETICAL 53.3 KD PROTEIN IN HXT8-CAN1 INTERGENIC REGION.//0.22:77:27//SACCHAROMYCES CEREVISIAE (BAKER'S YEAST).//P39981
F-HEMBA1004758
F-HEMBA1004763//HYPOTHETICAL PROLINE-RICH PROTEIN (FRAGMENT).//1.1e-06:58:43//OWENIA FUSIFORMIS.//P21260
F-HEMBA1004768//LINE-1 REVERSE TRANSCRIPTASE HOMOLOG.//4.7e-65:298:53//HOMO SAPIENS (HUMAN).//P08547
F-HEMBA1004770
F-HEMBA1004771
F-HEMBA1004776//GRANULIN 1.//0.78:28:42//CYPRINUS CARPIO (COMMON CARP).//P81013
F-HEMBA1004778
F-HEMBA1004795//CDC4-LIKE PROTEIN (FRAGMENT).//6.9e-20:74:63//HOMO SAPIENS (HUMAN).//P50851
F-HEMBA1004803//LINE-1 REVERSE TRANSCRIPTASE HOMOLOG.//1.4e-22:58:86//HOMO SAPIENS (HUMAN).//P08547
F-HEMBA1004806//HYPOTHETICAL 24.3 KD PROTEIN IN PSBH-RPL11 INTERGENIC REGION (ORF182).//0.72:75:33//CYANOPHORA PARADOXA.//P48324
F-HEMBA1004807
F-HEMBA1004816
F-HEMBA1004820//HEMOLYMPH TRYPSIN INHIBITOR A (BPI-TYPE) (FRAGMENT).//1.0:50:38//MANDUCA SEXTA (TOBACCO HAWKMOTH) (TOBACCO HORNWORM).//P26226
F-HEMBA1004847//SIGNAL RECOGNITION PARTICLE 68 KD PROTEIN (SRP68).//3.0e-76:171:91//CANIS FAMILIARIS (DOG).//Q00004
F-HEMBA1004850//COLLAGEN ALPHA 1(XII) CHAIN (FRAGMENTS).//3.0e-05:64:43//BOS TAURUS (BOVINE).//P25508
F-HEMBA1004863//TOXIN C13S1C1 PRECURSOR.//0.38:52:30//DENDROASPIS ANGUSTICEPS (EASTERN GREEN MAMBA).//P18329
F-HEMBA1004864//TAT PROTEIN (TRANSACTIVATING REGULATORY PROTEIN) (FRAGMENT).//0.89:24:50//HUMAN IMMUNODEFICIENCY VIRUS TYPE 1 (BH5 ISOLATE) (HIV-1).//P04612
F-HEMBA1004865
F-HEMBA1004880
F-HEMBA1004889//SMALL PROLINE-RICH PROTEIN II (SPR-II) (CLONE 174N).//0.66:23:47//HOMO SAPIENS (HUMAN).//P22532
F-HEMBA1004900
F-HEMBA1004909
F-HEMBA1004918//CHLOROPLAST 30S RIBOSOMAL PROTEIN S8 (FRAGMENT).//0.56:37:32//SPINACIA OLERACEA (SPINACH).//P09597
F-HEMBA1004923//!!!! ALU SUBFAMILY J WARNING ENTRY !!!!//3,5e-24:44:68//HOMO SAPIENS (HUMAN).//P39188
F-HEMBA1004929//ATP SYNTHASE PROTEIN 8 (EC 3.6.1.34) (A6L).//0.97:39:38//STRONGYLOCENTROTUS PURPURATUS (PURPLE SEA URCHIN).//P15997
F-HEMBA1004930//LINE-1 REVERSE TRANSCRIPTASE HOMOLOG.//6.6e-15:64:59//HOMO SAPIENS (HUMAN).//P08547
F-HEMBA1004933//VASODILATOR-STIMULATED PHOSPHOPROTEIN (VASP).//0.34:58:41//HOMO SAPIENS (HUMAN).//P50552
F-HEMBA1004934
F-HEMBA1004944
F-HEMBA1004954//NADH-UBIQUINONE OXIDOREDUCTASE CHAIN 3 (EC 1.6.5.3).//0.58:78:30//PARAMECIUM TETRAURELIA.//P15579
F-HEMBA1004956//HYPOTHETICAL 18.8 KD PROTEIN (ORF4).//0.98:57:31//PARAMECIUM TETRAURELIA.//P15605
F-HEMBA1004960//HYPOTHETICAL 12.6 KD PROTEIN-(ORFJ) (RETRON EC67).//1.0:58:27//ESCHERICHIA COLI.//P21324
F-HEMBA1004972
F-HEMBA1004973//SMALL PROLINE RICH PROTEIN II (SPR-II) (CLONE 930).//0.90:55:30//HOMO SAPIENS (HUMAN).//P22531
F-HEMBA1004977
F-HEMBA1004978
F-HEMBA1004980//MOTILIN PRECURSOR.//0.088:79:31//MACACA MULATTA (RHESUS MACAQUE).//018811
F-HEMBA1004983//10 KD CHAPERONIN (PROTEIN CPN10) (PROTEIN GROES).//0.87:51:31//BUCHNERA APHIDICOLA.//Q59176
F-HEMBA1004995//MYOCYTE-SPECIFIC ENHANCER FACTOR 2B (SERUM RESPONSE FACTOR-LIKE PROTEIN 2) (XMEF2) (RSRFR2).//0.17:52:40//HOMO SAPIENS (HUMAN).//Q02080
F-HEMBA1005008//METALLOTHIONEIN (MT).//1.0:52:32//CRASSOSTREA VIRGINICA (EASTERN OYSTER).//P23038
F-HEMBA1005009//ACTIN.//3.5e-27:171:38//CANDIDA ALBICANS (YEAST).//P14235
F-HEMBA1005019//HYPOTHETICAL PROTEIN HI1222.//0.13:58:31//HAEMOPHILUS INFLUENZAE.//P44129
F-HEMBA1005029//P2Y PURINOCEPTOR 5 (P2Y5) (PURINERGIC RECEPTOR 5) (6H1).//0.76:72:31//GALLUS GALLUS (CHICKEN).//P32250
F-HEMBA1005035//HOMEOBOX PROTEIN HB9.//0.0086:60:40//HOMO SAPIENS (HUMAN).//P50219
F-HEMBA1005039//SMALL PROLINE-RICH PROTEIN II (SPR-II) (CLONE 174N).//0.47:49:32//HOMO SAPIENS (HUMAN).//P22532
F-HEMBA1005047//RAS-RELATED PROTEIN RAB-24 (RAB-16).//1.5e-19:39:100//MUS MUSCULUS (MOUSE).//P35290
F-HEMBA1005050//COLLAGEN ALPHA 1(XII) CHAIN (FRAGMENTS).//0.074:34:44//BOS TAURUS (BOVINE).//P25508
F-HEMBA1005062
F-HEMBA1005066//LINE-1 REVERSE TRANSCRIPTASE HOMOLOG.//2.1e-44:126:65//HOMO SAPIENS (HUMAN).//P08547
F-HEMBA1005075//SUPPRESSOR PROTEIN SRP40.//0.35:96:31//SACCHAROMYCES CEREVISIAE (BAKER'S YEAST).//P32583
F-HEMBA1005079//!!!! ALU SUBFAMILY SB2 WARNING ENTRY !!!!//3.6e-20:75:64//HOMO SAPIENS (HUMAN).//P39191
F-HEMBA1005083//COLLAGEN ALPHA 1(XII) CHAIN (FRAGMENTS).//0.00015:72:34//BOS TAURUS (BOVINE).//P25508
F-HEMBA1005101//HETEROGENEOUS NUCLEAR RIBONUCLEOPROTEIN 27C (HNRNP 48) (HRP48.1).//4.8e-10:176:25//DROSOPHILA MELANOGASTER (FRUIT FLY).//P48809
F-HEMBA1005113
F-HEMBA1005123//!!!! ALU SUBFAMILY SQ WARNING ENTRY !!!!//3.6e-24:99:60//HOMO SAPIENS (HUMAN).//P39194
F-HEMBA1005133//HYPOTHETICAL 13.5 KD PROTEIN IN MOB1-SGA1 INTERGENIC REGION.//0.11:22:54//SACCHAROMYCES CEREVISIAE (BAKER'S YEAST).//P40490
F-HEMBA1005149//!!!! ALU SUBFAMILY J WARNING ENTRY !!!!//3.7e-16:59:71//HOMO SAPIENS (HUMAN).//P39188
F-HEMBA1005152//GENOME POLYPROTEIN 2 [CONTAINS: HELPER COMPONENT PROTEINASE (EC 3.4.22.-) (HC-PRO); 70 KD PROTEIN].//1.0:77:27//BARLEY YELLOW MOSAIC VIRUS (JAPANESE STRAIN II-1) (BAYMV).//Q01207
F-HEMBA1005159//NADH-UBIQUINONE OXIDOREDUCTASE CHAIN 4L (EC 1.6.5.3).//0.40:53:33//APIS MELLIFERA (HONEYBEE).//P34859
F-HEMBA1005185//MYOSIN IB HEAVY CHAIN.//0.011:58:48//DICTYOSTELIUM DISCOIDEUM (SLIME MOLD).//P34092
F-HEMBA1005201//HYPOTHETICAL 56.6 KD PROTEIN C16C9.03 IN CHROMOSOME I.//3.9e-67:241:53//SCHIZOSACCHAROMYCES POMBE (FISSION YEAST).//Q09817
F-HEMBA1005202//SIGNAL RECOGNITION PARTICLE 68 KD PROTEIN (SRP68).//3.8e-124:257:95//CANIS FAMILIARIS (DOG).//Q00004
F-HEMBA1005206//CUTICLE COLLAGEN 1.//0.010:118:33//CAENORHABDITIS ELEGANS.//P08124
F-HEMBA1005219//PTB-ASSOCIATED SPLICING FACTOR (PSF).//0.99:85:40//HOMO SAPIENS (HUMAN).//P23246
F-HEMBA1005223//HYPOTHETICAL GENE 1.05 PROTEIN.//0.31:75:28//BACTERIOPHAGE T3.//P07715
F-HEMBA1005232//HYPOTHETICAL 7.8 KD PROTEIN.//0.99:48:29//VACCINIA VIRUS (STRAIN WR), AND VACCINIA VIRUS (STRAIN COPENHAGEN).//P20544
F-HEMBA1005241//!!!! ALU SUBFAMILY SP WARNING ENTRY !!!!//1.4e-28:138:55//HOMO SAPIENS (HUMAN).//P39193
F-HEMBA1005244//SMALL PROLINE RICH PROTEIN II (SPR-II) (CLONE 930).//0.014:39:41//HOMO SAPIENS (HUMAN).//P22531
F-HEMBA1005251//ATP SYNTHASE PROTEIN 8 (EC 3.6.1.34) (A6L).//0.55:15:46//DICENTRARCHUS LABRAX (EUROPEAN SEA BASS).//Q36362
F-HEMBA1005252//EC PROTEIN HOMOLOG (ZINC-METALLOTHIONEIN CLASS II).//0.088:33:42//ZEA MAYS (MAIZE).//P43401
F-HEMBA1005274
F-HEMBA1005275//!!!! ALU SUBFAMILY J WARNING ENTRY !!!!//0.96:42:45//HOMO SAPIENS (HUMAN).//P39188
F-HEMBA1005293//PROBABLE COATOMER BETA' SUBUNIT (BETA'-COAT PROTEIN) (BETA'-COP).//0.55:98:30//CAENORHABDITIS ELEGANS.//Q20168
F-HEMBA1005296//MUCIN 2 PRECURSOR (INTESTINAL MUCIN 2).//0.095:75:34//HOMO SAPIENS (HUMAN).//Q02817
F-HEMBA1005304//!!!! ALU SUBFAMILY SB WARNING ENTRY !!!!//5.4e-33:103:74//HOMO SAPIENS (HUMAN).//P39189
F-HEMBA1005311//PERIOD CLOCK PROTEIN (FRAGMENT).//0.99:45:31//DROSOPHILA SALTANS (FRUIT FLY).//Q04536
F-HEMBA1005314//HYPOTHETICAL 6.3 KD PROTEIN T19C3.3 IN CHROMOSOME III.//0.98:30:30//CAENORHABDITIS ELEGANS.//Q10009
F-HEMBA1005315//LINE-1 REVERSE TRANSCRIPTASE HOMOLOG.//1.1e-05:35:51//HOMO SAPIENS (HUMAN).//P08547
F-HEMBA1005318//OLFACTORY RECEPTOR-LIKE PROTEIN COR8 (FRAGMENT).//0.57:44:38//GALLUS GALLUS (CHICKEN).//Q98913
F-HEMBA1005331//IMMEDIATE-EARLY PROTEIN IE180.//0.57:106:33//PSEUDORABIES VIRUS (STRAIN INDIANA-FUNKHAUSER / BECKER) (PRV).//P11675
F-HEMBA1005338//CARTIAGE MATRIX PROTEIN PRECURSOR (MATRILIN-1).//1.8e-55:199:59//GALLUS GALLUS (CHICKEN).//P05099
F-HEMBA1005353//CHLOROPLAST 30S RIBOSOMAL PROTEIN S17.//0.88:33:36//PORPHYRA PURPUREA.//P51305
F-HEMBA1005359//ZINC FINGER PROTEIN 83 (ZINC FINGER PROTEIN HPF1).//1.1e-68:255:48//HOMO SAPIENS (HUMAN).//P51522
F-HEMBA1005367//ALPHA-AMYLASE INHIBITOR AAI.//1.0:25:40//AMARANTHUS HYPOCHONDRIACUS (PRINCE'S FEATHER).//P80403
F-HEMBA1005372
F-HEMBA1005374//!!!! ALU SUBFAMILY SQ WARNING ENTRY !!!!//2.0e-34:92:75//HOMO SAPIENS (HUMAN).//P39194
F-HEMBA1005382//APOLIPOPROTEIN C-II (APO-CII).//0.99:39:33//BOS TAURUS (BOVINE).//P19034
F-HEMBA1005389//HYPOTHETICAL 70.0 KD PROTEIN IN DNAK 3'REGION (ORF4).//0.82:164:31//LACTOCOCCUS LACTIS (SUBSP. LACTIS) (STREPTOCOCCUS LACTIS).//P42377
F-HEMBA1005394//HYPOTHETICAL 8.9 KD PROTEIN IN IE0-IE1 INTERGENIC REGION.//0.98:44:38//AUTOGRAPHA CALIFORNICA NUCLEAR POLYHEDROSIS VIRUS (ACMNPV).//P41703
F-HEMBA1005403//SPERM HISTONE P2 PRECURSOR (PROTAMINE MP2).//0.066:64:29//MUS MUSCULUS (MOUSE).//P07978
F-HEMBA1005408//50S RIBOSOMAL PROTEIN L33.//0.77:32:25//BACILLUS SUBTILIS.//Q06798
F-HEMBA1005410//RETROVIRUS-RELATED POL POLYPROTEIN [CONTAINS: REVERSE TRANSCRIPTASE (EC 2.7.7.49); ENDONUCLEASE].//0.0065:38:52//MUS MUSCULUS (MOUSE).//P11369
F-HEMBA1005411//TOXIN S4C8.//0.16:46:28//DENDROASPIS JAMESONI KAIMOSAE (EASTERN JAMESON'S MAMBA).//P25683
F-HEMBA1005423//CYCLIN-DEPENDENT KINASE 6 INHIBITOR (P18-INK6) (CYCLIN-DEPENDENT KINASE 4 INHIBITOR C) (P18-INK4C).//4.3e-09:29:96//HOMO SAPIENS (HUMAN).//P42773
F-HEMBA1005426//TOXIN C10S2C2.//0.99:49:34//DENDROASPIS ANGUSTICEPS (EASTERN GREEN MAMBA).//P25684
F-HEMBA1005443//!!!! ALU SUBFAMILY J WARNING ENTRY !!!!//2.9e-16:78:60//HOMO SAPIENS (HUMAN).//P39188
F-HEMBA1005447//ATP SYNTHASE PROTEIN 8 (EC 3.6.1.34) (A6L).//0.99:57:31//DASYPUS NOVEMCINCTUS (NINE-BANDED ARMADILLO).//O21329
F-HEMBA1005468//NADH-UBIQUINONE OXIDOREDUCTASE CHAIN 1 (EC 1.6.5.3) (FRAGMENTS).//0.68:41:31//ARTEMIA SALINA (BRINE SHRIMP).//P19040
F-HEMBA1005469
F-HEMBA1005472//LINE-1 REVERSE TRANSCRIPTASE HOMOLOG.//1.5e-39:142:70//HOMO SAPIENS (HUMAN).//P08547
F-HEMBA1005474//!!!! ALU SUBFAMILY SQ WARNING ENTRY !!!!//5.8e-10:44:68//HOMO SAPIENS (HUMAN).//P39194
F-HEMBA1005475//U1 SMALL NUCLEAR RIBONUCLEOPROTEIN 70 KD (U1 SNRNP 70 KD) (SNRP70).//9.2e-14:179:33//HOMO SAPIENS (HUMAN).//P08621
F-HEMBA1005497
F-HEMBA1005500//60S RIBOSOMAL PROTEIN L37.//0.11:53:33//SCHISTOSOMA MANSONI (BLOOD FLUKE).//044125
F-HEMBA1005506
F-HEMBA1005508
F-HEMBA1005511//!!!! ALU SUBFAMILY SQ WARNING ENTRY !!!!//3.5e-30:92:73//HOMO SAPIENS (HUMAN).//P39194
F-HEMBA1005513//MALES-ABSENT ON THE FIRST PROTEIN (EC 2.3.1.-).//2.0e-39:95:61//DROSOPHILA MELANOGASTER (FRUIT FLY).//O02193
F-HEMBA1005517//PROLINE-RICH PROTEIN MP-2 PRECURSOR.//2.1e-06:56:44//MUS MUSCULUS (MOUSE).//P05142
F-HEMBA1005518//COLLAGEN ALPHA 1(I) CHAIN (FRAGMENTS).//5.8e-05:192:33//BOS TAURUS (BOVINE).//P02453
F-HEMBA1005520//!!!! ALU SUBFAMILY J WARNING ENTRY !!!!/2.0e-18:87:57//HOMO SAPIENS (HUMAN).//P39188
F-HEMBA1005526//!!!! ALU SUBFAMILY SB2 WARNING ENTRY !!!!/5.1e-22:77:54//HOMO SAPIENS (HUMAN).//P39191
F-HEMBA1005528//CCR4-ASSOCIATED FACTOR 1 (CAF1).//1.2e-81:157:98//MUS MUSCULUS (MOUSE).//Q60809
F-HEMBA1005530//POLLEN ALLERGEN AMB P 5-A PRECURSOR (AMB P V-A).//0.98:19:47//AMBROSIA PSILOSTACHYA (WESTERN RAGWEED).//P43174
F-HEMBA1005548//TRANSCRIPTION FACTOR MAF1.//1.4e-72:137:97//RATTUS NORVEGICUS (RAT).//P54842
F-HEMBA1005552//!!!! ALU SUBFAMILY SP WARNING ENTRY !!!!//1.7e-29:47:78//HOMO SAPIENS (HUMAN).//P39193
F-HEMBA1005558//HYPOTHETICAL 25.6 KD PROTEIN IN ABF2-CHL12 INTERGENIC REGION.//1.6e-20:202:30//SACCHAROMYCES CEREVISIAE (BAKER'S YEAST).//Q04272
F-HEMBA1005568
F-HEMBA1005570//NADH-UBIQUINONE OXIDOREDUCTASE CHAIN 6 (EC 1.6.5.3).//1.0:80:31//CAENORHABDITIS ELEGANS.//P24885
F-HEMBA1005576//TRANSMEMBRANE PROTEIN SEX PRECURSOR.//8.5e-58:152:75//HOMO SAPIENS (HUMAN).//P51805
F-HEMBA1005577//KERATIN, HIGH-SULFUR MATRIX PROTEIN, B2A.//0.98:57:36//OVIS ARIES (SHEEP).//P02438
F-HEMBA1005581//SLIT PROTEIN PRECURSOR.//1.1e-62:254:41//DROSOPHILA MELANOGASTER (FRUIT FLY).//P24014
F-HEMBA1005582//DYNACTIN, 150 KD ISOFORM (150 KD DYNEIN-ASSOCIATED POLYPEPTIDE) (DP-150) (DAP-150) (P150-GLUED).//0.0091:189:29//RATTUS NORVEGICUS (RAT).//P28023
F-HEMBA1005583//HYPOTHETICAL 41.2 KD PROTEIN IN CPS REGION (ORF7).//0.83:119:23//KLEBSIELLA PNEUMONIAE.//Q48453
F-HEMBA1005588//!!!! ALU SUBFAMILY J WARNING ENTRY !!!!//1.9e-17:108:53//HOMO SAPIENS (HUMAN).//P39188
F-HEMBA1005593//SMALL PROLINE-RICH PROTEIN II (SPR-II) (CLONE 174N).//0.23:24:54//HOMO SAPIENS (HUMAN).//P22532
F-HEMBA1005595//DYNEIN HEAVY CHAIN, CYTOSOUC (DYHC).//2.7e-39:257:39//DICTYOSTELIUM DISCOIDEUM (SLIME MOLD).//P34036
F-HEMBA1005606
F-HEMBA1005609//!!!! ALU SUBFAMILY SC WARNING ENTRY !!!!//3.2e-20:27:96//HOMO SAPIENS (HUMAN).//P39192
F-HEMBA1005616//LATE CONTROL GENE B PROTEIN (GPB).//0.48:51:33//BACTERIOPHAGE 186.//P08711
F-HEMBA1005621//MITOTIC MAD2 PROTEIN.//1.2e-06:137:32//SACCHAROMYCES CEREVISIAE (BAKER'S YEAST).//P40958
F-HEMBA1005627//HYPOTHETICAL 17.1 KD PROTEIN IN PUBS 3'REGION.//0.18:100:32//SACCHAROMYCES CEREVISIAE (BAKER'S YEAST).//P38898
F-HEMBA1005631
F-HEMBA1005632//HYPOTHETICAL 7.4 KD PROTEIN.//0.32:59:32//VACCINIA VIRUS (STRAIN WR).//P04309
F-HEMBA1005634//!!!! ALU SUBFAMILY J WARNING ENTRY !!!!//2.6e-14:93:58//HOMO SAPIENS (HUMAN).//P39188
F-HEMBA1005666//HYPOTHETICAL PROTEIN KIAA0129.//2.1e-05:126:25//HOMO SAPIENS (HUMAN).//Q14142
F-HEMBA1005670
F-HEMBA1005679//LINE-1 REVERSE TRANSCRIPTASE HOMOLOG.//2.2e-08:40:72//HOMO SAPIENS (HUMAN).//P08547
F-HEMBA1005680//SMALL PROLINE-RICH PROTEIN 2-1.//0.015:19:47//HOMO SAPIENS (HUMAN).//P35326
F-HEMBA1005685
F-HEMBA1005699//EPHRIN-B3 PRECURSOR (EPH-RELATED RECEPTOR TYROSINE KINASE LIGAND 8) (LERK-8) (EPH-RELATED RECEPTOR TRANSMEMBRANE LIGAND ELK-L3).//4.2e-38:98:81//HOMO SAPIENS (HUMAN).//Q15768
F-HEMBA1005705//PROTEIN Q300.//0.11:23:56//MUS MUSCULUS (MOUSE).//Q02722
F-HEMBA1005717
F-HEMBA1005732//BACTENECIN 7 PRECURSOR (BAC7).//0.22:55:41//OVIS ARIES (SHEEP).//P50415
F-HEMBA1005737//CALCINEURIN B SUBUNIT (PROTEIN PHOSPHATASE 2B REGULATORY SUBUNIT).//4.5e-18:167:34//SACCHAROMYCES CEREVISIAE (BAKER'S YEAST).//P25296
F-HEMBA1005746
F-HEMBA1005755//LINE-1 REVERSE TRANSCRIPTASE HOMOLOG.//7.4e-30:69:65//HOMO SAPIENS (HUMAN).//P08547
F-HEMBA1005765//!!!! ALU SUBFAMILY SQ WARNING ENTRY !!!!//2.8e-19:60:63//HOMO SAPIENS (HUMAN).//P39194
F-HEMBA1005780//METALLOTHIONEIN-I (MT-1).//1.0:31:38//COLUMBA LIVIA (DOMESTIC PIGEON).//P15786
F-HEMBA1005813
F-HEMBA1005815//CALPAIN, LARGE [CATALYTIC] SUBUNIT (EC 3.4.22.17) (CALCIUM- ACTIVATED NEUTRAL PROTEINASE) (CANP) (MU/M-TYPE).//1.0e-23:200:31//GALLUS GALLUS (CHICKEN).//P00789
F-HEMBA1005822//PROTEIN Q300.//0.0016:21:80//MUS MUSCULUS (MOUSE).//Q02722 F-HEMBA1005829//!!!! ALU SUBFAMILY SQ WARNING ENTRY !!!!//9.6e-33:96:73//HOMO SAPIENS (HUMAN).//P39194
F-HEMBA1005834//LINE-1 REVERSE TRANSCRIPTASE HOMOLOG.//1.6e-22:103:46//NYCTICEBUS COUCANG (SLOW LORIS).//P08548
F-HEMBA1005852//PROLINE-RICH PROTEIN MP-3 (FRAGMENT).//8.8e-06:95:35//MUS MUSCULUS (MOUSE).//P05143
F-HEMBA1005853//HYPOTHETICAL PROTEIN
MJ0647.//0.39:28:39//METHANOCOCCUS JANNASCHII.//Q58063
F-HEMBA1005884
F-HEMBA1005891//HYPOTHETICAL PROTEIN MTH137.//0.95:51:27//METHANOBACTERIUM THERMOAUTOTROPHICUM.//O26240
F-HEMBA1005894//!!!! ALU SUBFAMILY SX WARNING ENTRY !!!!//2.6e-29:81:71//HOMO SAPIENS (HUMAN).//P39195
F-HEMBA1005909//HYPOTHETICAL 8.2 KD PROTEIN B0353.1 IN CHROMOSOME III.//0.98:19:52//CAENORHABDITIS ELEGANS.//Q10958
F-HEMBA1005911//!!!! ALU SUBFAMILY J WARNING ENTRY !!!!//1.9e-27:86:70//HOMO SAPIENS (HUMAN).//P39188
F-HEMBA1005921//!!!! ALU SUBFAMILY SQ WARNING ENTRY !!!!//1.3e-38:99:81//HOMO SAPIENS (HUMAN).//P39194
F-HEMBA1005931//ZINC FINGER PROTEIN 83 (ZINC FINGER PROTEIN HPF1).//2.3e-17:76:51//HOMO SAPIENS (HUMAN).//P51522
F-HEMBA1005934//!!!! ALU SUBFAMILY SB WARNING ENTRY !!!!//0.024:54:40//HOMO SAPIENS (HUMAN).//P39189
F-HEMBA1005962
F-HEMBA1005963//COATOMER GAMMA SUBUNIT (GAMMA-COAT PROTEIN) (GAMMA-COP).//1.7e-32:89:79//BOS TAURUS (BOVINE).//P53620
F-HEMBA1005990//HYPOTHETICAL BHLF1 PROTEIN.//3.0e-09:180:36//EPSTEIN-BARR VIRUS (STRAIN B95-8) (HUMAN HERPESVIRUS 4).//P03181
F-HEMBA1005991//HYPOTHETICAL PROTEIN KIAA0032.//3.0e-17:107:43//HOMO SAPIENS (HUMAN).//Q15034
F-HEMBA1005999
F-HEMBA1006002
F-HEMBA1006005//CORNIFIN B (SMALL PROLINE-RICH PROTEIN 1B) (SPR1B) (SPR1 B).//0.0017:45:44//MUS MUSCULUS (MOUSE).//Q62267
F-HEMBA1006031//BASIC PROLINE-RICH PEPTIDE IB-1.//0.00016:84:39//HOMO SAPIENS (HUMAN).//P04281
F-HEMBA1006035//DNAK PROTEIN 1 (HEAT SHOCK PROTEIN 70) (HSP70).//0.43:100:27//SYNECHOCYSTIS SP. (STRAIN PCC 6803).//Q55154
F-HEMBA1006036//!!!! ALU SUBFAMILY SQ WARNING ENTRY !!!!//6.2e-64:150:74//HOMO SAPIENS (HUMAN).//P39194
F-HEMBA1006042
F-HEMBA1006067//METALLOTHIONEIN A (MT-A).//0.86:34:41//THERMARCES CERBERUS.//P52721
F-HEMBA1006081
F-HEMBA1006090//SODIUM/GLUCOSE COTRANSPORTER 3 (NA(+)/GLUCOSE COTRANSPORTER 3) (LOW AFFINITY SODIUM-GLUCOSE COTRANSPORTER).//0.87:35:54//SUS SCROFA (PIG).//P31636
F-HEMBA1006091//EARLY NODULIN 20 PRECURSOR (N-20).//0.027:87:32//MEDICAGO TRUNCATULA (BARREL MEDIC).//P93329
F-HEMBA1006100//!!!! ALU SUBFAMILY SX WARNING ENTRY !!!!//8.1e-09:58:60//HOMO SAPIENS (HUMAN).//P39195
F-HEMBA1006108//HYPOTHETICAL 56.6 KD PROTEIN IN URE2-SSU72 INTERGENIC REGION.//5.6e-16:88:36//SACCHAROMYCES CEREVISIAE (BAKER'S YEAST).//P53867
F-HEMBA1006121//HOMEOBOX PROTEIN CDX-1 (CAUDAL-TYPE HOMEOBOX PROTEIN 1).//3.4e-05:106:37//HOMO SAPIENS (HUMAN).//P47902
F-HEMBA1006124//50S RIBOSOMAL PROTEIN L33.//1.0:12:83//BACILLUS STEAROTHERMOPHILUS.//P23375
F-HEMBA1006130//SEL-10 PROTEIN.//7.7e-05:129:28//CAENORHABDITIS ELEGANS.//Q93794
F-HEMBA1006138//!!!! ALU SUBFAMILY SQ WARNING ENTRY !!!!//7.8e-13:41:73//HOMO SAPIENS (HUMAN).//P39194
F-HEMBA1006142//!!!! ALU SUBFAMILY SC WARNING ENTRY !!!!//2.3e-39:101:77//HOMO SAPIENS (HUMAN).//P39192
F-HEMBA1006155//GENE 33 POLYPEPTIDE.//0.21:70:31//RATTUS NORVEGICUS (RAT).//P05432
F-HEMBA1006158
F-HEMBA1006173//PROTEIN-TYROSINE PHOSPHATASE STRIATUM-ENRICHED (EC 3.1.3.48) (STEP) (NEURAL-SPECIFIC PROTEIN-TYROSINE PHOSPHATASE) (FRAGMENT).//0.017:20:95//HOMO SAPIENS (HUMAN).//P54829
F-HEMBA1006182//!!!! ALU SUBFAMILY J WARNING ENTRY !!!!//0.37:31:61//HOMO SAPIENS (HUMAN).//P39188
F-HEMBA1006198//HOMEOBOX PROTEIN HOX-B3 (HOX-2.7) (MH-23).//0.85:61:29//MUS MUSCULUS (MOUSE).//P09026
F-HEMBA1006235//50S RIBOSOMAL PROTEIN L33.//1.0:26:38//AQUIFEX AEOLICUS.//O67756
F-HEMBA1006248//MALE SPECIFIC SPERM PROTEIN MST84DB.//0.0041:64:37//DROSOPHILA MELANOGASTER (FRUIT FLY).//Q01643
F-HEMBA1006252//BOWMAN-BIRK TYPE PROTEINASE INHIBITOR DE-3.//1.0:22:40//DOLICHOS AXILLARIS (MACROTYLOMA AXILLARE).//P01057
F-HEMBA1006253//DISINTEGRIN ERISTICOPHIN (PLATELET AGGREGATION ACTIVATION INHIBITOR).//0.95:19:47//ERISTOCOPHIS MACMAHONI (LEAF-NOSED VIPER).//P22826
F-HEMBA1006259
F-HEMBA1006268//!!!! ALU SUBFAMILY SC WARNING ENTRY !!!!//7.0e-05:32:65//HOMO SAPIENS (HUMAN).//P39192
F-HEMBA1006272//RETROVIRUS-RELATED GAG POLYPROTEIN (VERSION 2).//4.8e-112:248:78//HOMO SAPIENS (HUMAN).//P10264
F-HEMBA1006278//POLY(A) POLYMERASE (EC 2.7.7.19) (PAP) (POLYNUCLEOTIDE ADENYLYLTRANSFERASE) (FRAGMENT).//2.5e-71:164:75//HOMO SAPIENS (HUMAN).//P51003
F-HEMBA1006283//50S RIBOSOMAL PROTEIN L32.//0.81:27:44//THERMUS AQUATICUS (SUBSP. THERMOPHILUS).//P80339
F-HEMBA1006284//CUTICLE COLLAGEN 2.//0.36:42:40//CAENORHABDITIS ELEGANS.//P17656
F-HEMBA1006291//HYPOTHETICAL 43.3 KD PROTEIN IN EVGS-GLK INTERGENIC REGION.//2.4e-37:143:31//ESCHERICHIA COLI.//P76518
F-HEMBA1006293//MYELIN-OLIGODENDROCYTE GLYCOPROTEIN PRECURSOR.//0.20:134:29//RATTUS NORVEGICUS (RAT).//Q63345 F-HEMBA1006309//HYPOTHETICAL 54.2 KD PROTEIN IN ERP5-ORC6 INTERGENIC REGION.//2.1e-43:187:48//SACCHAROMYCES CEREVISIAE (BAKER'S YEAST).//P38821
F-HEMBA1006310//SIGNAL TRANSDUCER CD24 PRECURSOR (HEAT STABLE ANTIGEN) (HSA) (NECTADRIN).//0.71:46:39//RATTUS NORVEGICUS (RAT).//Q07490
F-HEMBA1006328//RNA POLYMERASE ALPHA SUBUNIT (EC 2.7.7.48) (NUCLEOCAPSID PHOSPHOPROTEIN).//0.44:141:24//HUMAN PARAINFLUENZA 1 VIRUS (STRAIN CI-5/73).//P32531
F-HEMBA1006334//HYPOTHETICAL TRANSCRIPTIONAL REGULATOR AF1627.//0.98:26:46//ARCHAEOGLOBUS FULGIDUS.//028646
F-HEMBA1006344//EZRIN (P81) (CYTOVILLIN) (VILLIN-2).//8.8e-08:91:36//MUS MUSCULUS (MOUSE).//P26040
F-HEMBA1006347//MALES-ABSENT ON THE FIRST PROTEIN (EC 2.3.1.-).//9.1e-48:149:50//DROSOPHILA MELANOGASTER (FRUIT FLY).//O2193
F-HEMBA1006349//METALLOTHIONEIN-LIKE PROTEIN 1.//0.015:59:33//CASUARINA GLAUCA (SWAMP OAK).//Q39511
F-HEMBA1006359//ZINC FINGER PROTEIN 43 (ZINC PROTEIN HTF6).//6.8e-96:261:66//HOMO SAPIENS (HUMAN).//P28160
F-HEMBA1006364//PUTATIVE ENDONUCLEASE C1F12.06C (EC 3.1.-.-).//0.97:60:35//SCHIZOSACCHAROMYCES POMBE (FISSION YEAST).//Q10348
F-HEMBA1006377//EARLY NODULIN 20 PRECURSOR (N-20).//0.00023:110:35//MEDICAGO TRUNCATULA (BARREL MEDIC).//P93329
F-HEMBA1006380
F-HEMBA1006381//METALLOTHIONEIN-II.//1.0:26:38//CANDIDA GLABRATA (YEAST) (TORULOPSIS GLABRATA).//P15114
F-HEMBA1006398//LINE-1 REVERSE TRANSCRIPTASE HOMOLOG.//3.3e-26:123:52//HOMO SAPIENS (HUMAN).//P08547
F-HEMBA1006416
F-HEMBA1006419//!!!! ALU SUBFAMILY SB WARNING ENTRY !!!!//1.2e-24:102:50//HOMO SAPIENS (HUMAN).//P39189
F-HEMBA1006421//!!!! ALU SUBFAMILY J WARNING ENTRY !!!!//3.1e-21:101:57//HOMO SAPIENS (HUMAN).//P39188
F-HEMBA1006424//HYPOTHETICAL PROTEIN IORF1.//0.85:55:30//BOVINE CORONAVIRUS (STRAIN MEBUS), AND BOVINE CORONAVIRUS (STRAIN QUEBEC).//P22053
F-HEMBA1006426//!!!! ALU SUBFAMILY SX WARNING ENTRY !!!!//1.8e-36:78:74//HOMO SAPIENS (HUMAN).//P39195
F-HEMBA1006438//HYPOTHETICAL 8.1 KD PROTEIN (ORF65).//1.0:38:36//GUILLARDIA THETA (CRYPTOMONAS PHI).//O78421
F-HEMBA1006445//RAS-LIKE PROTEIN 3.//1.9e-06:40:47//RHIZOMUCOR RACEMOSUS (MUCOR CIRCINELLOIDES F. LUSITANICUS).//P22280
F-HEMBA1006446
F-HEMBA1006461//!!!! ALU SUBFAMILY SC WARNING ENTRY !!!!//4.1e-18:68:67//HOMO SAPIENS (HUMAN).//P39192
F-HEMBA1006467
F-HEMBA1006471
F-HEMBA1006474//40 KD PROTEIN.//1.1e-37:231:38//BORNA DISEASE VIRUS (BDV).//Q01552
F-HEMBA1006483//!!!! ALU SUBFAMILY SC WARNING ENTRY !!!!//6.1e-38:77:74//HOMO SAPIENS (HUMAN).//P39192
F-HEMBA1006485//HYPOTHETICAL 9.3 KD PROTEIN IN NAD3-NAD7 INTERGENIC REGION (ORF 79).//0.91:30:40//MARCHANTIA POLYMORPHA (LIVERWORT).//P38465
F-HEMBA1006486//LINE-1 REVERSE TRANSCRIPTASE HOMOLOG.//1.1e-12:78:51//HOMO SAPIENS (HUMAN).//P08547
F-HEMBA1006489//FUN34 PROTEIN.//0.94:58:37//SACCHAROMYCES CEREVISIAE (BAKER'S YEAST).//P32907
F-HEMBA1006492//NADH-UBIQUINONE OXIDOREDUCTASE MWFE SUBUNIT (EC 1.6.5.3) (EC 1.6.99.3) (COMPLEX I-MWFE) (CI-MWFE).//0.87:44:36//HOMO SAPIENS (HUMAN).//O15239
F-HEMBA1006494//FERREDOXIN-LIKE PROTEIN IN NIF REGION.//0.11:46:26//RHIZOBIUM LEGUMINOSARUM (BIOVAR TRIFOLII).//P42711
F-HEMBA1006497
F-HEMBA1006502//!!!! ALU SUBFAMILY J WARNING ENTRY !!!!//0.15:26:73//HOMO SAPIENS (HUMAN).//P39188
F-HEMBA1006507//DIAPHANOUS PROTEIN.//0.0055:129:28//DROSOPHILA MELANOGASTER (FRUIT FLY).//P48608
F-HEMBA1006521//3-OXOACYL-[ACYL-CARRIER PROTEIN] REDUCTASE (EC 1.1.1.100) (3-KETOACYL- ACYL CARRIER PROTEIN REDUCTASE).//1.1e-32:177:41//ESCHERICHIA COLI.//P25716
F-HEMBA1006530//NADH-UBIQUINONE OXIDOREDUCTASE CHAIN 5 (EC 1.6.5.3) (FRAGMENT).//0.052:84:26//LEISHMANIA TARENTOLAE (SAUROLEISHMANIA TARENTOLAE).//P15583
F-HEMBA1006535//INHIBITOR OF APOPTOSIS PROTEIN 1 (MIAP1) (MIAP-1).//6.6e-05:53:39//MUS MUSCULUS (MOUSE).//O08863
F-HEMBA1006540//PRESYNAPTIC PROTEIN SAP97 (SYNAPSE-ASSOCIATED PROTEIN 97) (DISCS, LARGE HOMOLOG 1).//2.1e-07:206:23//RATTUS NORVEGICUS (RAT).//Q62696
F-HEMBA1006546//PROBABLE E5 PROTEIN.//0.11:70:32//HUMAN PAPILLOMAVIRUS TYPE 51.//P26553
F-HEMBA1006559//SUPPRESSOR PROTEIN SRP40.//0.015:221:20//SACCHAROMYCES CEREVISIAE (BAKER'S YEAST).//P32583
F-HEMBA1006562//SALIVARY PROLINE-RICH PROTEIN PO PRECURSOR (ALLELE S).//1.5e-07:122:33//HOMO SAPIENS (HUMAN).//P10163
F-HEMBA1006566//CELL DIVISION PROTEIN KINASE 2 (EC 2.7.1.-) (CDC2 HOMOLOG EG1 PROTEIN KINASE).//0.63:53:37//XENOPUS LAEVIS (AFRICAN CLAWED FROG).//P23437
F-HEMBA1006569//COLLAGEN ALPHA 2(I) CHAIN (FRAGMENT).//4.4e-06:88:39//BOS TAURUS (BOVINE).//P02465
F-HEMBA1006579
F-HEMBA1006583//PROLINE-RICH PROTEIN MP-2 PRECURSOR.//0.011:61:40//MUS MUSCULUS (MOUSE).//P05142
F-HEMBA1006595//!!!! ALU SUBFAMILY SQ WARNING ENTRY !!!!//5.6e-34:93:77//HOMO SAPIENS (HUMAN).//P39194
F-HEMBA1006597//!!!! ALU SUBFAMILY SX WARNING ENTRY !!!!//1.9e-26:75:74//HOMO SAPIENS (HUMAN).//P39195
F-HEMBA1006612//SUPPRESSOR PROTEIN SRP40.//0.026:221:22//SACCHAROMYCES CEREVISIAE (BAKER'S YEAST).//P32583
F-HEMBA1006617//!!!! ALU SUBFAMILY J WARNING ENTRY !!!!//6.6e-20:73:63//HOMO SAPIENS (HUMAN).//P39188
F-HEMBA1006624//HYPOTHETICAL 41.9 KD PROTEIN IN SDS3-THS1 INTERGENIC REGION.//2.6e-31:209:44//SACCHAROMYCES CEREVISIAE (BAKER'S YEAST).//P40506
F-HEMBA1006631//HYPOTHETICAL 62.8 KD PROTEIN IN TAF145-YOR1 INTERGENIC REGION.//1.5e-15:131:41//SACCHAROMYCES CEREVISIAE (BAKER'S YEAST).//P53331
F-HEMBA1006635
F-HEMBA1006639//POLYADENYLATE-BINDING PROTEIN 1 (POLY(A) BINDING PROTEIN 1) (PABP 1).//2.2e-11:48:75//MUS MUSCULUS (MOUSE).//P29341
F-HEMBA1006643//LONG NEUROTOXIN CR1 PRECURSOR (KAPPA NEUROTOXIN).//0.28:48:27//BUNGARUS MULTICINCTUS (MANY-BANDED KRAIT).//P15817
F-HEMBA1006648//ZINC FINGER PROTEIN 12 (ZINC FINGER PROTEIN KOX3) (FRAGMENT).//0.26:17:47//HOMO SAPIENS (HUMAN).//P17014
F-HEMBA1006652//60S RIBOSOMAL PROTEIN L7.//2.4e-44:206:47//MUS MUSCULUS (MOUSE).//P14148
F-HEMBA1006653
F-HEMBA1006659
F-HEMBA1006665//LINE-1 REVERSE TRANSCRIPTASE HOMOLOG.//0.018:43:58//HOMO SAPIENS (HUMAN).//P08547
F-HEMBA1006674//TRANSCRIPTION INITIATION FACTOR TFIID 135 KD SUBUNIT (TAFII-135) (TAFII135) (TAFII-130) (TAFII130).//2.9e-05:154:33//HOMO SAPIENS (HUMAN).//O00268
F-HEMBA1006676//HYPOTHETICAL PROLINE-RICH PROTEIN (FRAGMENT).//3.6e-09:52:51//OWENIA FUSIFORMIS.//P21260
F-HEMBA1006682
F-HEMBA1006695//!!!! ALU SUBFAMILY J WARNING ENTRY !!!!//2.1e-06:35:65//HOMO SAPIENS (HUMAN).//P39188
F-HEMBA1006696
F-HEMBA1006708//HYPOTHETICAL 46.4 KD TRP-ASP REPEATS CONTAINING PROTEIN IN PMC1-TFG2 INTERGENIC REGION.//3.4e-19:104:45//SACCHAROMYCES CEREVISIAE (BAKER'S YEAST).//P53196
F-HEMBA1006709//RETINOIC ACID RECEPTOR RXR-BETA.//0.24:111:36//HOMO SAPIENS (HUMAN).//P28702
F-HEMBA1006717
F-HEMBA1006737//ANKYRIN, BRAIN VARIANT 2 (ANKYRIN B) (ANKYRIN, NONERYTHROID) (FRAGMENT).//5.8e-09:111:40//HOMO SAPIENS (HUMAN).//Q01485
F-HEMBA1006744//!!!! ALU SUBFAMILY SB2 WARNING ENTRY !!!!//1.8e-32:84:78//HOMO SAPIENS (HUMAN).//P39191
F-HEMBA1006754//LINE-1 REVERSE TRANSCRIPTASE HOMOLOG.//1.3e-75:220:62//HOMO SAPIENS (HUMAN).//P08547
F-HEMBA1006758//VASCULAR ENDOTHELIAL-CADHERIN PRECURSOR (VECADHERIN) (CADHERIN-5) (7B4 ANTIGEN) (CD144 ANTIGEN).//0.024:110:29//HOMO SAPIENS (HUMAN).//P33151
F-HEMBA1006767
F-HEMBA1006779//MITOCHONDRIAL RIBOSOMAL PROTEIN S12.//0.67:19:42//LEISHMANIA TARENTOLAE (SAUROLEISHMANIA TARENTOLAE).//Q34940
F-HEMBA1006780
F-HEMBA1006789//PROLINE-RICH PROTEIN MP-3 (FRAGMENT).//0.056:98:30//MUS MUSCULUS (MOUSE).//P05143
F-HEMBA1006795//LINE-1 REVERSE TRANSCRIPTASE HOMOLOG.//2.9e-11:143:30//NYCTICEBUS COUCANG (SLOW LORIS).//P08548
F-HEMBA1006796//WISKOTT-ALDRICH SYNDROME PROTEIN HOMOLOG (WASP).//0.16:38:42//MUS MUSCULUS (MOUSE).//P70315
F-HEMBA1006807//HYPOTHETICAL 46.4 KD PROTEIN T16H12.5 IN CHROMOSOME III.//4.4e-75:184:77//CAENORHABDITIS ELEGANS.//P34568
F-HEMBA1006821//!!!! ALU SUBFAMILY SQ WARNING ENTRY !!!!//0.011:20:85//HOMO SAPIENS (HUMAN).//P39194
F-HEMBA1006824//PROTEIN B11.//0.44:27:44//VACCINIA VIRUS (STRAIN WR).//Q01229
F-HEMBA1006832//HYPOTHETICAL 34.6 KD PROTEIN C13G5.2 IN CHROMOSOME III.//1.0:46:36//CAENORHABDITIS ELEGANS.//P34327
F-HEMBA1006849
F-HEMBA1006865//ACROSIN INHIBITORS IIA AND IIB (BUSI-II).//1.0:41:31//BOS TAURUS (BOVINE).//P01001
F-HEMBA1006877//OXYSTEROL-BINDING PROTEIN.//3.7e-26:239:36//ORYCTOLAGUS CUNICULUS (RABBIT).//P16258
F-HEMBA1006885//HYPOTHETICAL 27.2 KD PROTEIN F09E5.8 IN CHROMOSOME II.//4.5e-38:185:43//CAENORHABDITIS ELEGANS.//P52057
F-HEMBA1006900
F-HEMBA1006914//UBIQUITIN-ACTIVATING ENZYME E1-LIKE (POLYMERASE-INTERACTING PROTEIN 2).//5.2e-27:269:33//SACCHAROMYCES CEREVISIAE (BAKER'S YEAST).//P52488
F-HEMBA1006921//CYTOTOXIN 3 (COMPONENT 3.20).//0.99:32:37//NAJA MELANOLEUCA (FOREST COBRA) (BLACK-LIPPED COBRA).//P01473
F-HEMBA1006926//ATROPHIN-1 (DENTATORUBRAL-PALLIDOLUYSIAN ATROPHY PROTEIN).//0.0024:148:33//RATTUS NORVEGICUS (RAT).//P54258
F-HEMBA1006929//HYPOTHETICAL PROTEIN MJ0525.//0.95:35:20//METHANOCOCCUS JANNASCHII.//Q57945
F-HEMBA1006936//SALIVARY ACIDIC PROLINE-RICH PHOSPHOPROTEIN 1/2 PRECURSOR (PRP-1 / PRP-3) (PRP-2 / PRP-4) (PIF-F / PIF-S) (PROTEIN A / PROTEIN C) [CONTAINS: PEPTIDE P-C].//0.074:116:31//HOMO SAPIENS (HUMAN).//P02810
F-HEMBA1006938
F-HEMBA1006941//THIOREDOXIN H-TYPE 1 (TRX-H1).//2.1e-13:90:33//NICOTIANA TABACUM (COMMON TOBACCO).//P29449
F-HEMBA1006949
F-HEMBA1006973//COLLAGEN ALPHA 1(XII) CHAIN (FRAGMENTS).//0.75:29:55//BOS TAURUS (BOVINE).//P25508
F-HEMBA1006976//CMP-N-ACETYLNEURAMINATE-BETA-GALACTOSAMIDE-ALPHA-2,3-SIALYLTRANSFERASE (EC 2.4.99.-) (BETA-GALACTOSIDE ALPHA-2,3-SIALYLTRANSFERASE) (ST3GALIII) (ALPHA 2,3-ST) (GAL-NAC6S) (STZ) (SIAT4-C) (SAT-3) (ST-4).//3.9e-108:117:95//HOMO SAPIENS (HUMAN).//Q11206
F-HEMBA1006993
F-HEMBA1006996//HYPOTHETICAL 8.7 KD PROTEIN IN RPL22-RPL23 INTERGENIC REGION (ORF70).//0.12:51:33//ASTASIA LONGA (EUGLENOPHYCEAN ALGA).//P34779
F-HEMBA1007002//PLATELET GLYCOPROTEIN IX PRECURSOR (GPIX) (CD42A).//0.00096:60:33//HOMO SAPIENS (HUMAN).//P14770
F-HEMBA1007017//HYPOTHETICAL 7.2 KD PROTEIN IN CYAY-DAPF INTERGENIC REGION.//1.0:25:56//ESCHERICHIA COLI.//P39166
F-HEMBA1007018//DYNEIN LIGHT INTERMEDIATE CHAIN 1, CYTOSOLIC (LIC57/59) (DYNEIN LIGHT CHAIN A) (DLC-A).//8.5e-120:278:80//GALLUS GALLUS (CHICKEN).//Q90828
F-HEMBA1007045//SKIN SECRETORY PROTEIN XP2 PRECURSOR (APEG PROTEIN).//2.1e-12:158:29//XENOPUS LAEVIS (AFRICAN CLAWED FROG).//P17437
F-HEMBA1007051
F-HEMBA1007052//60S RIBOSOMAL PROTEIN L37-B (L35) (YP55).//0.94:37:35//SACCHAROMYCES CEREVISIAE (BAKER'S YEAST).//P51402
F-HEMBA1007062//ATP SYNTHASE PROTEIN 8 (EC 3.6.1.34) (A6L).//0.93:55:29//RHINOCEROS UNICORNIS (GREATER INDIAN RHINOCEROS).//Q96063
F-HEMBA1007066//ECLOSION HORMONE PRECURSOR (ECDYSIS ACTIVATOR) (EH).//0.58:49:38//BOMBYX MORI (SILK MOTH).//P25331
F-HEMBA1007073//PUTATTVE SMALL MEMBRANE PROTEIN (ORF 4).//0.86:46:34//CANINE ENTERIC CORONAVIRUS (STRAIN INSAVC-1) (CCV).//P36696
F-HEMBA1007078//!!!! ALU SUBFAMILY SP WARNING ENTRY !!!!//8.6e-29:56:67//HOMO SAPIENS (HUMAN).//P39193
F-HEMBA1007080//SKIN SECRETORY PROTEIN XP2 PRECURSOR (APEG PROTEIN).//0.028:122:30//XENOPUS LAEVIS (AFRICAN CLAWED FROG).//P17437
F-HEMBA1007085//RTOA PROTEIN (RATIO-A).//7.4e-11:221:31//DICTYOSTELIUM DISCOIDEUM (SLIME MOLD).//P54681
F-HEMBA1007087//HYPOTHETICAL PROTEIN MJ0162.//3.3e-29:173:36//METHANOCOCCUS JANNASCHII.//Q57626
F-HEMBA1007112
F-HEMBA1007113
F-HEMBA1007121//INOSITOL POLYPHOSPHATE 1-PHOSPHATASE (EC 3.1.3.57) (IPP).//5.4e-07:90:28//HOMO SAPIENS (HUMAN).//P49441
F-HEMBA1007129//HIRUSTASIN.//0.88:37:32//HIRUDO MEDICINALIS (MEDICINAL LEECH) .//P80302
F-HEMBA1007147//HYPOTHETICAL 12.0 KD PROTEIN IN DST1-HEM2 INTERGENIC REGION.//0.92:23:34//SACCHAROMYCES CEREVISIAE (BAKER'S YEAST).//P53182
F-HEMBA1007149//BACTERIOCIN MICROCIN B17 PRECURSOR (MCB17).//0.0078:17:70//ESCHERICHIA COLI.//P05834
F-HEMBA1007151//WDNM1 PROTEIN PRECURSOR.//0.25:45:37//MUS MUSCULUS (MOUSE).//Q62477
F-HEMBA1007174//HYPOTHETICAL 45.1 KD PROTEIN IN RPS5-ZMS1 INTERGENIC REGION.//6.9e-18:97:47//SACCHAROMYCES CEREVISIAE (BAKER'S YEAST).//P47160
F-HEMBA1007178//!!!! ALU SUBFAMILY SX WARNING ENTRY !!!!/9.8e-06:38:65//HOMO SAPIENS (HUMAN).//P39195
F-HEMBA1007194//GLUCOSE-6-PHOSPHATE 1-DEHYDROGENASE, CHOLOROPLAST ISOFORM PRECURSOR (EC 1.1.1.49) (G6PD).//1.0:80:32//NICOTIANA TABACUM (COMMON TOBACCO).//Q43793
F-HEMBA1007203//PROTEIN A22.//1.0:115:26//VARIOLA VIRUS.//P33845
F-HEMBA1007206
F-HEMBA1007224//HYPOTHETICAL 35.7 KD PROTEIN C41C4.6 IN CHROMOSOME II.//2.4e-05:92:30//CAENORHABDITIS ELEGANS.//Q09275
F-HEMBA1007243//HYPOXANTHINE-GUANINE PHOSPHORIBOSYLTRANSFERASE (EC 2.4.2.8) (HGPRT) (HGPRTASE) (HPRT B).//3.1e-74:205:67//MUS MUSCULUS (MOUSE).//P00493
F-HEMBA1007251//VITELLINE MEMBRANE PROTEIN VM26AB PRECURSOR (PROTEIN TU-4) (PROTEIN SV23).//0.52:108:30//DROSOPHILA MELANOGASTER (FRUIT FLY).//P13238
F-HEMBA1007256
F-HEMBA1007267//CALICIN (FRAGMENT).//0.060:88:31//HOMO SAPIENS (HUMAN).//Q13939
F-HEMBA1007273//HYPOTHETICAL 8.1 KD PROTEIN (ORF65).//0.95:40:37//GUILLARDIA THETA (CRYPTOMONAS PHI).//O78421
F-HEMBA1007279//!!!! ALU SUBFAMILY J WARNING ENTRY !!!!//4.6e-24:98:64//HOMO SAPIENS (HUMAN).//P39188
F-HEMBA1007281
F-HEMBA1007288//HYPOTHETICAL 13.5 KD PROTEIN IN ZMS1-MNS1 INTERGENIC REGION.//0.88:11:54//SACCHAROMYCES CEREVISIAE (BAKER'S YEAST).//P47162
F-HEMBA1007300//CGMP-SPECIFIC 3',5'-CYCLIC PHOSPHODIESTERASE (EC 3.1.4.17) (CGB-PDE).//2.7e-43:220:41//BOS TAURUS (BOVINE).//Q28156
F-HEMBA1007301//PROCOLLAGEN ALPHA 1(III) CHAIN PRECURSOR.//3.3e-22:115:33//HOMO SAPIENS (HUMAN).//P02461
F-HEMBA1007319
F-HEMBA1007320//HYPOTHETICAL 28.0 KD PROTEIN IN GLOB-RNHA INTERGENIC REGION.//1.0:48:37//ESCHERICHIA COLI.//P75672
F-HEMBA1007322//THREONINE DEHYDRATASE OPERON ACTIVATOR PROTEIN.//1.0:59:33//ESCHERICHIA COLI.//P11866
F-HEMBA1007327
F-HEMBA1007341//!!!! ALU SUBFAMILY J WARNING ENTRY !!!!//9.1e-12:37:62//HOMO SAPIENS (HUMAN).//P39188
F-HEMBA1007342//PROBABLE E5 PROTEIN.//0.89:96:29//PYGMY CHIMPANZEE PAPILLOMAVIRUS TYPE 1.//Q02268
F-HEMBA1007347//INSULIN-LIKE GROWTH FACTOR BINDING PROTEIN 2 PRECURSOR (IGFBP-2) (IBP-2) (IGF-BINDING PROTEIN 2).//0.92:62:43//OVIS ARIES (SHEEP).//Q29400
F-HEMBB1000005//WEAK NEUROTOXIN 5.//0.98:30:33//NAJA NAJA (INDIAN COBRA).//P29179
F-HEMBB1000008//!!!! ALU SUBFAMILY SX WARNING ENTRY !!!!//2.7e-35:73:84//HOMO SAPIENS (HUMAN).//P39195
F-HEMBB1000018//HYPOTHETICAL BHLF1 PROTEIN.//0.39:90:37//EPSTEIN-BARR VIRUS (STRAIN B95-8) (HUMAN HERPESVIRUS 4).//P03181
F-HEMBB1000024//VIRE LOCUS 9 KD VIRULENCE PROTEIN.//0.66:36:41//AGROBACTERIUM TUMEFACIENS.//P08061
F-HEMBB1000025//MUSCARINIC TOXIN ALPHA (MT-ALPHA).//0.46:32:40//DENDROASPIS POLYLEPIS POLYLEPIS (BLACK MAMBA).//P80494
F-HEMBB1000030//SUPPRESSOR PROTEIN SRP40.//6.7e-07:50:52//SACCHAROMYCES CEREVISIAE (BAKER'S YEAST).//P32583
F-HEMBB1000036//HYPOTHETICAL 43.2 KD PROTEIN C34E10.1 IN CHROMOSOME III.//2.5e-07:120:29//CAENORHABDITIS ELEGANS.//P46576
F-HEMBB1000037//HYPOTHETICAL 59.9 KD PROTEIN-IN SGA1-KTR7 INTERGENIC REGION.//1.7e-05:71:29//SACCHAROMYCES CEREVISIAE (BAKER'S YEAST).//P40492
F-HEMBB1000039//VERY HYPOTHETICAL 11.9 KD PROTEIN C4H3.12C IN CHROMOSOME I.//1.0:61:21//SCHIZOSACCHAROMYCES POMBE (FISSION YEAST).//Q10219
F-HEMBB1000044
F-HEMBB1000048//HYPOTHETICAL 15.7 KD PROTEIN IN IDH-DEOR INTERGENIC REGION.//1.0:63:31//BACILLUS SUBTILIS.//P54942
F-HEMBB1000050//!!!! ALU SUBFAMILY SQ WARNING ENTRY !!!!//9.0e-14:34:79//HOMO SAPIENS (HUMAN).//P39194
F-HEMBB1000054//!!!! ALU SUBFAMILY SP WARNING ENTRY !!!!//5.9e-31:45:73//HOMO SAPIENS (HUMAN).//P39193
F-HEMBB1000055//MUSCARINIC TOXIN ALPHA (MT-ALPHA).//1.0:14:57//DENDROASPIS POLYLEPIS POLYLEPIS (BLACK MAMBA).//P80494
F-HEMBB1000059//!!!! ALU SUBFAMILY SX WARNING ENTRY !!!!//1.0e-21:82:59//HOMO SAPIENS (HUMAN).//P39195
F-HEMBB1000083//CHROMOGRANIN A PRECURSOR (CGA) [CONTAINS: PANCREASTATIN; BETA-GRANIN; WE-14].//0.87:172:28//RATTUS NORVEGICUS (RAT).//P10354
F-HEMBB1000089//HYPOTHETICAL 9.5 KD PROTEIN IN SPEA-METK INTERGENIC REGION (F83).//1.0:42:33//ESCHERICHIA COLI.//P46879
F-HEMBB1000099//!!!! ALU SUBFAMILY SB WARNING ENTRY !!!!//7.7e-08:31:87//HOMO SAPIENS (HUMAN).//P39189
F-HEMBB1000103/LINE-1 REVERSE TRANSCRIPTASE HOMOLOG.//1.4e-38:136:58//HOMO SAPIENS (HUMAN).//P08547
F-HEMBB1000113//!!!! ALU SUBFAMILY J WARNING ENTRY !!!!//3.9e-13:57:64//HOMO SAPIENS (HUMAN).//P39188
F-HEMBB1000119//MAF PROTEIN.//3.6e-32:195:43//BACILLUS SUBTILIS.//Q02169
F-HEMBB1000136//HYPOTHETICAL 12.7 KD PROTEIN IN PCS60-ABD1 INTERGENIC REGION.//0.65:71:32//SACCHAROMYCES CEREVISIAE (BAKER'S YEAST).//P38327
F-HEMBB1000141//B-CELL GROWTH FACTOR PRECURSOR (BCGF-12 KD).//0.00014:34:64//HOMO SAPIENS (HUMAN).//P20931
F-HEMBB1000144//!!!! ALU SUBFAMILY SB2 WARNING ENTRY !!!!//2.0e-26:81:69//HOMO SAPIENS (HUMAN).//P39191
F-HEMBB1000173//!!!! ALU SUBFAMILY J WARNING ENTRY !!!!//9.2e-29:91:71//HOMO SAPIENS (HUMAN).//P39188
F-HEMBB1000175//ANTIMICROBIAL PEPTIDE ENAP-1 (FRAGMENT).//0.97:41:36//EQUUS CABALLUS (HORSE).//P80930
F-HEMBB1000198//HYPOTHETICAL 7.7 KD PROTEIN YCF33 (ORF67).//0.91:21:52//PORPHYRA PURPUREA.//P51329
F-HEMBB1000215//!!!! ALU SUBFAMILY SC WARNING ENTRY !!!!//3.4e-08:39:76//HOMO SAPIENS (HUMAN).//P39192
F-HEMBB1000217//DNA DAMAGE TOLERANCE PROTEIN RHC31 (RAD31 HOMOLOG).//2.9e-32:174:40//SACCHAROMYCES CEREVISIAE (BAKER'S YEAST).//Q06624
F-HEMBB1000218//ATP SYNTHASE PROTEIN 8 (EC 3.6.1.34) (A6L).//0.73:31:38//MICROTUS PENNSYLVANICUS (MEADOW VOLE).//P24949
F-HEMBB1000226//HYPOTHETICAL 37.0 KD PROTEIN B0495.8 IN CHROMOSOME II.//6.5e-26:191:34//CAENORHABDITIS ELEGANS.//Q09217
F-HEMBB1000240
F-HEMBB1000244//!!!! ALU SUBFAMILY J WARNING ENTRY !!!!//1.9e-05:44:61//HOMO SAPIENS (HUMAN).//P39188
F-HEMBB1000250
F-HEMBB1000258
F-HEMBB1000264//CUTICLE COLLAGEN SQT-1.//0.15:89:33//CAENORHABDITIS ELEGANS.//P12114
F-HEMBB1000266//TRANSLATION INITIATION FACTOR IF-2.//2.7e-06:167:22//SACCHAROMYCES CEREVISIAE (BAKER'S YEAST).//P39730
F-HEMBB1000272//CYTOCHROME C OXIDASE POLYPEPTIDE VIB (EC 1.9.3.1) (AED).//0.75:30:43//BOS TAURUS (BOVINE).//P00429
F-HEMBB1000274//CORNIFIN (SMALL PROLINE-RICH PROTEIN I) (SPR-I) (SMALL PROLINE-RICH SQUAMOUS CELL MARKER) (SPRP).//1.0:38:36//SUS SCROFA (PIG).//P35323
F-HEMBB1000284//CALTRIN (CALCIUM TRANSPORT INHIBITOR).//1.0:56:30//MUS MUSCULUS (MOUSE).//Q09098
F-HEMBB1000307
F-HEMBB1000312
F-HEMBB1000317//THROMBOSPONDIN 1 PRECURSOR.//3.2e-32:135:43//HOMO SAPIENS (HUMAN).//P07996
F-HEMBB1000318//PUTATIVE SMALL MEMBRANE PROTEIN (NONSTRUCTURAL PROTEIN NS3) (NONSTRUCTURAL 9.5 KD PROTEIN).//0.41:51:31//HUMAN CORONAVIRUS (STRAIN OC43).//Q04854
F-HEMBB1000335//ZINC FINGER PROTEIN 13 (ZFP-13) (KROX-8 PROTEIN) (FRAGMENT).//0.82:33:45//MUS MUSCULUS (MOUSE).//P10754
F-HEMBB1000336//ALDEHYDE OXIDASE (EC 1.2.3.1) (FRAGMENTS).//0.80:44:40//ORYCTOLAGUS CUNICULUS (RABBIT).//P80456
F-HEMBB1000337//SPLICING FACTOR, ARGININE/SERINE-RICH 4 (PRE-MRNA SPLICING FACTOR SRP75).//0.94:118:22//HOMO SAPIENS (HUMAN).//Q08170
F-HEMBB1000338//MALE SPECIFIC SPERM PROTEIN MST84DA.//0.042:33:39//DROSOPHILA MELANOGASTER (FRUIT FLY).//Q01642
F-HEMBB1000339//!!!! ALU SUBFAMILY J WARNING ENTRY !!!!//2.2e-14:54:55//HOMO SAPIENS (HUMAN).//P39188
F-HEMBB1000341//GENE 74 PROTEIN (GP74).//1.0:39:33//MYCOBACTERIOPHAGE L5.//Q05289
F-HEMBB1000343
F-HEMBB1000354//!!!! ALU SUBFAMILY J WARNING ENTRY !!!!//1.1e-15:83:56//HOMO SAPIENS (HUMAN).//P39188
F-HEMBB1000369//PROTEIN Q300.//0.99:27:40//MUS MUSCULUS (MOUSE).//Q02722
F-HEMBB1000374//!!!! ALU SUBFAMILY SB WARNING ENTRY !!!!//4.7e-34:56:78//HOMO SAPIENS (HUMAN).//P39189
F-HEMBB1000376
F-HEMBB1000391//COLLAGEN ALPHA 1(XII) CHAIN (FRAGMENTS).//0.0013:79:35//BOS TAURUS (BOVINE).//P25508
F-HEMBB1000399//CHECKPOINT PROTEIN RAD17.//2.8e-15:187:31//SCHIZOSACCHAROMYCES POMBE (FISSION YEAST).//P50531
F-HEMBB1000402//NADH-UBIQUINONE OXIDOREDUCTASE CHAIN 5 (EC 1.6.5.3) (FRAGMENT).//0.027:60:38//LEISHMANIA TARENTOLAE (SAUROLEISHMANIA TARENTOLAE).//P15583
F-HEMBB1000404//CYANELLE 50S RIBOSOMAL PROTEIN L28.//0.94:29:27//CYANOPHORA PARADOXA.//P48129
F-HEMBB1000420//SPLICEOSOME ASSOCIATED PROTEIN 49 (SAP 49) (SF3B53).//0.023:97:35//HOMO SAPIENS (HUMAN).//Q15427
F-HEMBB1000434//!!!! ALU SUBFAMILY SQ WARNING ENTRY !!!!//4.8e-20:111:54//HOMO SAPIENS (HUMAN).//P39194
F-HEMBB1000438//HYPOTHETICAL 7.9 KD PROTEIN IN GP55-NRDG INTERGENIC REGION.//0.93:24:50//BACTERIOPHAGE T4.//P07076
F-HEMBB1000441//!!!! ALU SUBFAMILY J WARNING ENTRY !!!!//4.4e-23:85:70//HOMO SAPIENS (HUMAN).//P39188
F-HEMBB1000449//!!!! ALU SUBFAMILY SX WARNING ENTRY !!!!//0.88:27:51//HOMO SAPIENS (HUMAN).//P39195
F-HEMBB1000455
F-HEMBB1000472
F-HEMBB1000480//PROTEIN STBC.//1.0:52:30//ESCHERICHIA COLI.//P11905
F-HEMBB1000487//SHORT NEUROTOXIN 1 (NEUROTOXIN ALPHA) (NEUROTOXIN II).//0.93:29:34//NAJA OXIANA (CENTRAL ASIAN COBRA) (OXUS COBRA).//P01427
F-HEMBB1000490//!!!! ALU SUBFAMILY SX WARNING ENTRY !!!!//2.3e-16:50:80//HOMO SAPIENS (HUMAN).//P39195
F-HEMBB1000491
F-HEMBB1000493//3A PROTEIN.//1.0:51:35//AVIAN INFECTIOUS BRONCHITIS VIRUS (STRAIN BEAUDETTE) (IBV).//P30237
F-HEMBB1000510//LINE-1 REVERSE TRANSCRIPTASE HOMOLOG.//9.7e-27:132:45//HOMO SAPIENS (HUMAN).//P08547
F-HEMBB1000518//CYTOCHROME C OXIDASE POLYPEPTIDE III (EC 1.9.3.1).//0.021:47:40//LEISHMANIA TARENTOLAE (SAUROLEISHMANIA TARENTOLAE).//P14546
F-HEMBB1000523
F-HEMBB1000530//COLLAGEN ALPHA 1(XIV) CHAIN PRECURSOR (UNDULIN).//9.8e-14:43:83//GALLUS GALLUS (CHICKEN).//P32018
F-HEMBB1000550//NADH-UBIQUINONE OXIDOREDUCTASE CHAIN 5 (EC 1.6.5.3).//0.19:97:30//TRYPANOSOMA BRUCEI BRUCEI.//P04540
F-HEMBB1000554//MATERNAL B9.10 PROTEIN (P30 B9.10).//0.94:82:25//XENOPUS LAEVIS (AFRICAN CLAWED FROG).//P40744
F-HEMBB1000556//TRANSCRIPTION INITIATION FACTOR TFIID 135 KD SUBUNIT (TAFII-135) (TAFII135) (TAFII-130) (TAFII130).//0.043:201:29//HOMO SAPIENS (HUMAN).//000268
F-HEMBB1000564//ATP SYNTHASE PROTEIN 8 (EC 3.6.1.34) (A6L).//1.0:5:2:34//METRIDIUM SENILE (BROWN SEA ANEMONE) (FRILLED SEA ANEMONE).//O47493
F-HEMBB1000573//!!!! ALU SUBFAMILY SB2 WARNING ENTRY !!!!//2.3e-10:52:73//HOMO SAPIENS (HUMAN).//P39191
F-HEMBB1000575//!!!! ALU SUBFAMILY SC WARNING ENTRY !!!!//1.8e-26:76:76//HOMO SAPIENS (HUMAN).//P39192
F-HEMBB1000586//NADH-UBIQUINONE OXIDOREDUCTASE MLRQ SUBUNIT (EC 1.6.5.3) (EC 1.6.99.3) (COMPLEX I-MLRQ) (CI-MLRQ).//0.74:23:52//HOMO SAPIENS (HUMAN).//O00483
F-HEMBB1000589//!!!! ALU SUBFAMILY SP WARNING ENTRY !!!!//2.9e-25:61:75//HOMO SAPIENS (HUMAN).//P39193
F-HEMBB1000591//ATP SYNTHASE PROTEIN 8 (EC 3.6.1.34) (A6L).//1.0:34:35//PETROMYZON MARINUS (SEA LAMPREY).//Q35537
F-HEMBB1000592//SMALL PROLINE-RICH PROTEIN 2-1.//0.0016:49:42//HOMO SAPIENS (HUMAN).//P35326
F-HEMBB1000593//COLLAGEN ALPHA 1(III) CHAIN (FRAGMENTS).//0.0070:189:32//GALLUS GALLUS (CHICKEN).//P12105
F-HEMBB1000598//LINE-1 REVERSE TRANSCRIPTASE HOMOLOG.//5.7e-10:110:41//NYCTICEBUS COUCANG (SLOW LORIS).//P08548
F-HEMBB1000623//HYPOTHETICAL 54.9 KD PROTEIN C02F5.7 IN CHROMOSOME III.//0.0022:98:28//CAENORHABDITIS ELEGANS.//P34284
F-HEMBB1000630
F-HEMBB1000631//ALPHA-2C-1 ADRENERGIC RECEPTOR (ALPHA-2C-1 ADRENOCEPTOR) (SUBTYPE C4).//8.8e-06:59:40//HOMO SAPIENS (HUMAN).//P18825
F-HEMBB1000632//GUANINE NUCLEOTIDE RELEASING PROTEIN (GNRP).//7.3e-13:173:28//MUS MUSCULUS (MOUSE).//P27671
F-HEMBB1000637//!!!! ALU SUBFAMILY SP WARNING ENTRY !!!!//4.6e-41:94:82//HOMO SAPIENS (HUMAN).//P39193
F-HEMBB1000638//INVOLUCRIN.//1.9e-06:144:29//HOMO SAPIENS (HUMAN).//P07476
F-HEMBB1000643//!!!! ALU SUBFAMILY SX WARNING ENTRY !!!!//8.3e-30:77:76//HOMO SAPIENS (HUMAN).//P39195
F-HEMBB1000649//!!!! ALU SUBFAMILY SB WARNING ENTRY !!!!//2.5e-37:58:81//HOMO SAPIENS (HUMAN).//P39189
F-HEMBB1000652//!!!! ALU SUBFAMILY SP WARNING ENTRY !!!!//2.2e-37:61:77//HOMO SAPIENS (HUMAN).//P39193
F-HEMBB1000665//HYPOTHETICAL PROTEIN BBD24.//0.83:38:36//BORRELIA BURGDORFERI (LYME DISEASE SPIROCHETE).//P70845
F-HEMBB1000671//LINE-1 REVERSE TRANSCRIPTASE HOMOLOG.//6.8e-51:74:71//HOMO SAPIENS (HUMAN).//P08547
F-HEMBB1000673//HEAT-STABLE ENTEROTOXIN A3/A4 PRECURSOR (STA3/STA4) (ST-IB) (ST-H).//0.012:37:37//ESCHERICHIA COLI.//P07965
F-HEMBB1000684//!!!! ALU SUBFAMILY SP WARNING ENTRY !!!!//3.1e-21:66:72//HOMO SAPIENS (HUMAN).//P39193
F-HEMBB1000693//HUNTINGTIN ASSOCIATED PROTEIN 1 (HAP1).//5.2e-26:121:49//RATTUS NORVEGICUS (RAT).//P54256
F-HEMBB1000705
F-HEMBB1000706
F-HEMBB1000709//HYPOTHETICAL 5.8 KD PROTEIN.//1.0:29:44//CLOVER YELLOW MOSAIC VIRUS (CYMV).//P16485
F-HEMBB1000725//RAS-RELATED PROTEIN RAB-8B.//7.4e-105:205:98//RATTUS NORVEGICUS (RAT).//P70550
F-HEMBB1000726//!!!! ALU SUBFAMILY SQ WARNING ENTRY !!!!//1.4e-25:85:70//HOMO SAPIENS (HUMAN).//P39194
F-HEMBB1000738//50S RIBOSOMAL PROTEIN L33.//1.0:41:31//THERMUS AQUATICUS (SUBSP. THERMOPHILUS).//P35871
F-HEMBB1000749//!!!! ALU SUBFAMILY SQ WARNING ENTRY !!!!//1.3e-29:42:85//HOMO SAPIENS (HUMAN).//P39194
F-HEMBB1000763//NIFU PROTEIN.//0.089:63:36//FRANKIA ALNI.//P46045
F-HEMBB1000770//CALTRIN-LIKE PROTEIN II.//0.98:13:69//CAVIA PORCELLUS (GUINEA PIG).//P22075
F-HEMBB1000774//HIGH MOBILITY GROUP PROTEIN HMG-Y.//0.029:53:32//MUS MUSCULUS (MOUSE).//P17095
F-HEMBB1000781//MAPK/ERK KINASE KINASE 2 (EC 2.7.1.-) (MEK KINASE 2) (MEKK 2).//3.5e-75:144:98//MUS MUSCULUS (MOUSE).//Q61083
F-HEMBB1000789//PUTATIVE 90.2 KD ZINC FINGER PROTEIN IN CCA1-ADK2 INTERGENIC REGION.//2.6e-49:232:43//SACCHAROMYCES CEREVISIAE (BAKER'S YEAST).//P39956
F-HEMBB1000790//!!!! ALU SUBFAMILY J WARNING ENTRY !!!!//2.9e-16:93:51//HOMO SAPIENS (HUMAN).//P39188
F-HEMBB1000794
F-HEMBB1000807//MUSCARINIC ACETYLCHOLINE RECEPTOR M3.//0.54:111:27//GALLUS GALLUS (CHICKEN).//P49578
F-HEMBB1000810
F-HEMBB1000821
F-HEMBB1000822//HYPOTHETICAL 10 KD PROTEIN (ORF 6).//0.10:50:34//NARCISSUS MOSAIC VIRUS (NMV).//P15099
F-HEMBB1000826//B-CELL GROWTH FACTOR PRECURSOR (BCGF-12 KD).//0.00025:73:39//HOMO SAPIENS (HUMAN).//P20931
F-HEMBB1000827//HYPOTHETICAL 7.4 KD PROTEIN.//0.89:23:52//THERMOPROTEUS TENAX VIRUS 1 (STRAIN KRA1) (TTV1).//P19302
F-HEMBB1000831//MALE SPECIFIC SPERM PROTEIN MST87F.//0.98:35:40//DROSOPHILA MELANOGASTER (FRUIT FLY).//P08175
F-HEMBB1000835//LINE-1 REVERSE TRANSCRIPTASE HOMOLOG.//7.8e-31:96:46//HOMO SAPIENS (HUMAN).//P08547
F-HEMBB1000840//LINE-1 REVERSE TRANSCRIPTASE HOMOLOG.//0.00012:102:36//NYCTICEBUS COUCANG (SLOW LORIS).//P08548
F-HEMBB1000848//LINE-1 REVERSE TRANSCRIPTASE HOMOLOG.//7.3e-97:239:70//HOMO SAPIENS (HUMAN).//P08547
F-HEMBB1000852
F-HEMBB1000870
F-HEMBB1000876//METALLOTHIONEIN (MT).//0.99:14:64//PERCA FLUVIATILIS (PERCH).//P52725
F-HEMBB1000883//HYPOTHETICAL 7.8 KD PROTEIN (ORF62).//0.34:60:33//GUILLARDIA THETA (CRYPTOMONAS PHI).//O78459
F-HEMBB1000887//HISTIDINE-RICH, METAL BINDING POLYPEPTIDE.//1.0:26:42//HELICOBACTER PYLORI (CAMPYLOBACTER PYLORI).//Q48251
F-HEMBB1000888
F-HEMBB1000890
F-HEMBB1000893
F-HEMBB1000908//!!!! ALU SUBFAMILY J WARNING ENTRY !!!!//0.0074:45:51//HOMO SAPIENS (HUMAN).//P39188
F-HEMBB1000910//PROBABLE E5 PROTEIN.//1.0:49:36//HUMAN PAPILLOMAVIRUS TYPE 58.//P26552
F-HEMBB1000913//!!!! ALU SUBFAMILY SX WARNING ENTRY !!!!//0.29:56:46//HOMO SAPIENS (HUMAN).//P39195
F-HEMBB1000915//CYTOCHROME B (EC 1.10.2.2).//2.5e-24:62:90//HOMO SAPIENS (HUMAN).//P00156
F-HEMBB1000917//!!!! ALU SUBFAMILY SP WARNING ENTRY !!!!//5.9e-26:53:66//HOMO SAPIENS (HUMAN).//P39193
F-HEMBB1000927//NEURONAL CALCIUM SENSOR 1 (NCS-1) (FREQUENIN).//3.9e-44:182:45//XENOPUS LAEVIS (AFRICAN CLAWED FROG).//Q91614
F-HEMBB1000947//SMALL PROLINE-RICH PROTEIN 2-1.//0.24:69:27//HOMO SAPIENS (HUMAN).//P35326
F-HEMBB1000959//!!!! ALU SUBFAMILY SX WARNING ENTRY !!!!//3.0e-31:89:68//HOMO SAPIENS (HUMAN).//P39195
F-HEMBB1000973//CONNECTIVE TISSUE GROWTH FACTOR PRECURSOR.//0.96:66:36//BOS TAURUS (BOVINE).//018739
F-HEMBB1000975//HISTIDINE-RICH GLYCOPROTEIN PRECURSOR (HISTIDINE-PROLINE RICH GLYCOPROTEIN) (HPRG).//0.00042:77:41//HOMO SAPIENS (HUMAN).//P04196
F-HEMBB1000981
F-HEMBB1000985//MIPP PROTEIN (MURINE IAP-PROMOTED PLACENTA-EXPRESSED PROTEIN).//1.0e-18:178:30//MUS MUSCULUS (MOUSE).//P28575
F-HEMBB1000991
F-HEMBB1000996//HYPOTHETICAL 10.1 KD PROTEIN IN RHSD-GCL INTERGENIC REGION (ORFD3).//0.58:34:35//ESCHERICHIA COLI.//P33669
F-HEMBB1001004//PROBABLE E4 PROTEIN.//0.24:110:35//HUMAN PAPILLOMAVIRUS TYPE 5B.//P26550
F-HEMBB1001008
F-HEMBB1001011//ZINC FINGER PROTEIN 7 (ZINC FINGER PROTEIN KOX4) (ZINC FINGER PROTEIN HF.16).//3.2e-17:104:47//HOMO SAPIENS (HUMAN).//P17097
F-HEMBB1001014//EOTAXIN PRECURSOR (EOSINOPHIL CHEMOTACTIC PROTEIN).//1.0:58:39//RATTUS NORVEGICUS (RAT).//P97545
F-HEMBB1001020//!!!! ALU SUBFAMILY SB WARNING ENTRY !!!!//1.4e-07:36:75//HOMO SAPIENS (HUMAN).//P39189
F-HEMBB1001024
F-HEMBB1001037//FERREDOXIN.//1.0:52:25//MOORELLA THERMOACETICA (CLOSTRIDIUM THERMOACETICUM).//P00203
F-HEMBB1001047
F-HEMBB1001051//PROTEIN FAN (FACTOR ASSOCIATED WITH N-SMASE ACTIVATION).//3.4e-21:50:100//HOMO SAPIENS (HUMAN).//Q92636
F-HEMBB1001056//HYPOTHETICAL 29.3 KD PROTEIN (ORF92).//0.0099:115:35//ORGYIA PSEUDOTSUGATA MULTICAPSID POLYHEDROSIS VIRUS (OPMNPV).//O10341
F-HEMBB1001058//!!!! ALU SUBFAMILY SC WARNING ENTRY !!!!//1.1e-33:95:76//HOMO SAPIENS (HUMAN).//P39192
F-HEMBB1001060//HYPOTHETICAL 8.2 KD PROTEIN ZC21.7 IN CHROMOSOME III.//1.0:38:36//CAENORHABDITIS ELEGANS.//P34591
F-HEMBB1001063
F-HEMBB1001068
F-HEMBB1001096//NOXIUSTOXIN (NTX) (TOXIN II.11).//0.99:36:38//CENTRUROIDES NOXIUS (MEXICAN SCORPION).//P08815
F-HEMBB1001102//HYPOTHETICAL 72.5 KD PROTEIN C2F7.10 IN CHROMOSOME I.//1.1e-27:115:36//SCHIZOSACCHAROMYCES POMBE (FISSION YEAST).//Q09701
F-HEMBB1001105//CLASS II HISTOCOMPATIBILITY ANTIGEN, M ALPHA CHAIN PRECURSOR.//0.80:70:40//HOMO SAPIENS (HUMAN).//P28067
F-HEMBB1001112//PROTEIN TRANSPORT PROTEIN SEC61 ALPHA SUBUNIT.//1.1e-126:287:85//RATTUS NORVEGICUS (RAT).//P38378
F-HEMBB1001114//HYPOTHETICAL 9.6 KD PROTEIN (ORF2).//0.84:62:27//BACTERIOPHAGE L2.//P42537
F-HEMBB1001117
F-HEMBB1001119//COLLAGEN ALPHA 1(XII) CHAIN PRECURSOR.//1.6e-21:50:98//HOMO SAPIENS (HUMAN).//Q99715
F-HEMBB1001126//HYPOTHETICAL 55.9 KD PROTEIN EEED8.6 IN CHROMOSOME II.//1.7e-50:184:53//CAENORHABDITIS ELEGANS.//Q09296
F-HEMBB1001133//!!!! ALU SUBFAMILY SC WARNING ENTRY !!!!//1.4e-09:53:62//HOMO SAPIENS (HUMAN).//P39192
F-HEMBB1001137//DNA-DIRECTED RNA POLYMERASE II LARGEST SUBUNIT (EC 2.7.7.6) (RPB1) (FRAGMENT).//2.0e-05:206:27//CRICETULUS GRISEUS (CHINESE HAMSTER).//P11414
F-HEMBB1001142//!!!! ALU SUBFAMILY SP WARNING ENTRY !!!!//4.1e-05:46:56//HOMO SAPIENS (HUMAN).//P39193
F-HEMBB1001151//HYPOTHETICAL 33.5 KD PROTEIN C1D4.02C IN CHROMOSOME I.//2.3e-23:109:44//SCHIZOSACCHAROMYCES POMBE (FISSION YEAST).//Q10149
F-HEMBB1001153//PROCOLLAGEN ALPHA 2(IV) CHAIN PRECURSOR.//0.75:76:34//ASCARIS SUUM (PIG ROUNDWORM) (ASCARIS LUMBRICOIDES).//P27393
F-HEMBB1001169//!!!! ALU SUBFAMILY SX WARNING ENTRY !!!!//1.4e-16:71:59//HOMO SAPIENS (HUMAN).//P39195
F-HEMBB1001175//ANKYRIN.//3.2e-12:169:31//MUS MUSCULUS (MOUSE).//Q02357
F-HEMBB1001177//PERIODIC TRYPTOPHAN PROTEIN 2 HOMOLOG.//9.4e-07:148:27//HOMO SAPIENS (HUMAN).//Q15269
F-HEMBB1001182//HYPOTHETICAL 36.0 KD PROTEIN.//1.3e-09:110:31//SACCHAROMYCES CEREVISIAE (BAKER'S YEAST).//P54858
F-HEMBB1001199
F-HEMBB1001208//HYPOTHETICAL PROTEIN LAMBDA-SP5.//0.053:23:47//MUS MUSCULUS (MOUSE).//P15974
F-HEMBB1001209
F-HEMBB1001210//HYPOTHETICAL PROTEIN LAMBDA-SP5.//0.14:40:37//MUS MUSCULUS (MOUSE).//P15974
F-HEMBB1001218//!!!! ALU SUBFAMILY SQ WARNING ENTRY !!!!//3.4e-19:49:67//HOMO SAPIENS (HUMAN).//P39194
F-HEMBB1001221//CYTOCHROME C OXIDASE POLYPEPTIDE VIIA-LIVER PRECURSOR (EC 1.9.3.1).//0.11:44:38//HOMO SAPIENS (HUMAN).//P14406
F-HEMBB1001234//65 KD YES-ASSOCIATED PROTEIN (YAP65).//2.0e-45:192:53//MUS MUSCULUS (MOUSE).//P46938
F-HEMBB1001242//HYPOTHETICAL 143.3 KD TRP-ASP REPEATS CONTAINING PROTEIN C12G12.13C IN CHROMOSOME I.//5.5e-37:226:41//SCHIZOSACCHAROMYCES POMBE (FISSION YEAST).//Q09876
F-HEMBB1001249//OXALOACETATE DECARBOXYLASE GAMMA CHAIN (EC 4.1.1.3).//1.0:23:43//KLEBSIELLA PNEUMONIAE.//P13155
F-HEMBB1001253//METALLOTHIONEIN-IH (MT-1H) (METALLOTHIONEIN-0) (MT-0).//0.14:16:43//HOMO SAPIENS (HUMAN).//P80294
F-HEMBB1001254//!!!! ALU SUBFAMILY SX WARNING ENTRY !!!!//1.4e-12:40:75//HOMO SAPIENS (HUMAN).//P39195
F-HEMBB1001267//!!!! ALU SUBFAMILY SP WARNING ENTRY !!!!//1.0e-12:33:78//HOMO SAPIENS (HUMAN).//P39193
F-HEMBB1001271//HYPOTHETICAL 25.1 KD PROTEIN B0302.5 IN CHROMOSOME X.//1.0:58:37//CAENORHABDITIS ELEGANS.//Q10928
F-HEMBB1001282//ANKYRIN HOMOLOG PRECURSOR.//9.5e-13:206:31//CHROMATIUM VINOSUM.//Q06527
F-HEMBB1001288//COPPER HOMEOSTASIS PROTEIN CUTC.//4.6e-42:163:51//ESCHERICHIA COLI.//P46719
F-HEMBB1001289//HYPOTHETICAL PROTEIN ORF-1137.//1.0e-05:106:26//MUS MUSCULUS (MOUSE).//P11260
F-HEMBB1001294//GTP-BINDING PROTEIN TC10.//1.3e-34:58:94//HOMO SAPIENS (HUMAN).//P17081
F-HEMBB1001302//HOMEOBOX PROTEIN CDX-2 (CAUDAL-TYPE HOMEOBOX PROTEIN 2) (CDX-3).//0.24:49:46//HOMO SAPIENS (HUMAN).//Q99626
F-HEMBB1001304//GLYCINE-RICH CELL WALL STRUCTURAL PROTEIN (CLONE W10-1) (FRAGMENT).//1.0:17:70//LYCOPERSICON ESCULENTUM (TOMATO).//Q01157
F-HEMBB1001314//SALIVARY GLUE PROTEIN SGS-3 PRECURSOR.//0.21:104:27//DROSOPHILA ERECTA (FRUIT FLY).//P13730
F-HEMBB1001315//!!!! ALU SUBFAMILY SX WARNING ENTRY !!!!//1.3e-24:53:71//HOMO SAPIENS (HUMAN).//P39195
F-HEMBB1001317//HYPOTHETICAL 85.7 KD PROTEIN C13G6.03 IN CHROMOSOME I.//0.24:90:31//SCHIZOSACCHAROMYCES POMBE (FISSION YEAST).//Q09782
F-HEMBB1001326//HYPOTHETICAL PROTEIN LAMBDA-SP5.//0.36:26:50//MUS MUSCULUS (MOUSE).//P15974
F-HEMBB1001331//HYPOTHETICAL BHLF1 PROTEIN.//1.0:127:33//EPSTEIN-BARR VIRUS (STRAIN B95-8) (HUMAN HERPESVIRUS 4).//P03181
F-HEMBB1001335//ESCARGOT/SNAIL PROTEIN HOMOLOG (FRAGMENT).//0.85:44:29//SCIARA COPROPHILA (FUNGUS GNAT).//Q01799
F-HEMBB1001337//!!!! ALU SUBFAMILY SQ WARNING ENTRY !!!!//4.2e-20:62:62//HOMO SAPIENS (HUMAN).//P39194
F-HEMBB1001339//HYPOTHETICAL 17.3 KD PROTEIN CY1A11.16C.//8.2e-07:123:34//MYCOBACTERIUM TUBERCULOSIS.//Q50606
F-HEMBB1001346//LINE-1 REVERSE TRANSCRIPTASE HOMOLOG.//4.2e-14:60:45//HOMO SAPIENS (HUMAN).//P08547
F-HEMBB1001348//!!!! ALU SUBFAMILY J WARNING ENTRY !!!!//5.6e-14:61:62//HOMO SAPIENS (HUMAN).//P39188
F-HEMBB1001356
F-HEMBB1001364
F-HEMBB1001366/HISTIDINE-RICH PROTEIN.//0.87:26:42//PLASMODIUM FALCIPARUM (ISOLATE FCM17 / SENEGAL).//P14586
F-HEMBB1001367//!!!! ALU SUBFAMILY SC WARNING ENTRY !!!!//8.6e-40:146:61//HOMO SAPIENS (HUMAN).//P39192
F-HEMBB1001369
F-HEMBB1001380//!!!! ALU SUBFAMILY SP WARNING ENTRY !!!!/1.9e-25:49:83//HOMO SAPIENS (HUMAN).//P39193
F-HEMBB1001384//BH3 INTERACTING DOMAIN DEATH AGONIST (BID).//0.80:95:29//MUS MUSCULUS (MOUSE).//P70444
F-HEMBB1001387//PEA2 PROTEIN (PPF2 PROTEIN).//0.022:117:34//SACCHAROMYCES CEREVISIAE (BAKER'S YEAST).//P40091
F-HEMBB1001394//ALPHA-ADAPTIN A (CLATHRIN ASSEMBLY PROTEIN COMPLEX 2 ALPHA-A LARGE CHAIN) (100 KD COATED VESICLE PROTEIN A) (PLASMA MEMBRANE ADAPTOR HA2/AP2 ADAPTIN ALPHA A SUBUNIT).//0.38:85:31//MUS MUSCULUS (MOUSE).//P17426
F-HEMBB1001410
F-HEMBB1001424//PHOTOSYSTEM II 4 KD REACTION CENTRE PROTEIN PRECURSOR.//0.99:37:21//ORYZA SATIVA (RICE).//P12162
F-HEMBB1001426//!!!! ALU SUBFAMILY SX WARNING ENTRY !!!!//0.0035:40:60//HOMO SAPIENS (HUMAN).//P39195
F-HEMBB1001429//CYTOSOL AMINOPEPTIDASE (EC 3.4.11.1) (LEUCINE AMINOPEPTIDASE) (LAP) (LEUCYL AMINOPEPTIDASE) (PROLINE AMINOPEPTIDASE) (EC 3.4.11.5) (PROLYL AMINOPEPTIDASE).//1.1e-99:21:86//BOS TAURUS (BOVINE).//P00727
F-HEMBB1001436//!!!! ALU SUBFAMILY SX WARNING ENTRY !!!!//3.4e-30:57:78//HOMO SAPIENS (HUMAN).//P39195
F-HEMBB1001443//[PYRUVATE DEHYDROGENASE (LIPOAMIDE)]-PHOSPHATASE PRECURSOR (PDP) (EC 3.1.3.43) (PYRUVATE DEHYDROGENASE PHOSPHATASE, CATALYTIC SUBUNIT (PDPC).//2.5e-79:155:97//BOS TAURUS (BOVINE).//P35816
F-HEMBB1001449
F-HEMBB1001454//SALIVARY PROLINE-RICH PROTEIN PO (ALLELE M) [CONTAINS: PEPTIDE P-D] (FRAGMENT).//1.1e-05:196:31//HOMO SAPIENS (HUMAN).//P10161
F-HEMBB1001458//24 KD ANTIGEN (FRAGMENT).//0.94:18:50//PLASMODIUM CHABAUDI.//P14592
F-HEMBB1001463
F-HEMBB1001464//PPF2L ANTIGEN (FRAGMENT).//1.0:45:28//PLASMODIUM FALCIPARUM (ISOLATE PALO ALTO / UGANDA).//P07765
F-HEMBB1001482//GASTRULA ZINC FINGER PROTEIN XLCGF16.1 (FRAGMENT).//4.2e-10:37:43//XENOPUS LAEVIS (AFRICAN CLAWED FROG).//P18712 F-HEMBB1001500
F-HEMBB1001521//!!!! ALU SUBFAMILY J WARNING ENTRY !!!!//2.4e-39:59:72//HOMO SAPIENS (HUMAN).//P39188
F-HEMBB1001527//HOMEOBOX PROTEIN HOX-B5 (XLHBOX-4) (XHOX-1B) (FRAGMENT).//0.21:131:25//XENOPUS LAEVIS (AFRICAN CLAWED FROG).//P09019
F-HEMBB1001531//GENE 32 PROTEIN (GP32).//0.88:95:30//MYCOBACTERIOPHAGE L5.//Q05241
F-HEMBB1001535//ATP SYNTHASE PROTEIN 8 (EC 3.6.1.34) (A6L).//1.0:31:38//LUMBRICUS TERRESTRIS (COMMON EARTHWORM).//Q34942
F-HEMBB1001536
F-HEMBB1001537//!!!! ALU SUBFAMILY SB2 WARNING ENTRY !!!!//0.0063:52:50//HOMO SAPIENS (HUMAN).//P39191
F-HEMBB1001555//!!!! ALU SUBFAMILY J WARNING ENTRY !!!!//4.9e-23:69:63//HOMO SAPIENS (HUMAN).//P39188
F-HEMBB1001562//RABPHILIN-3A.//0.087:147:27//RATTUS NORVEGICUS (RAT).//P47709
F-HEMBB1001564//LINE-1 REVERSE TRANSCRIPTASE HOMOLOG.//5.9e-27:107:54//HOMO SAPIENS (HUMAN).//P08547
F-HEMBB1001565//!!!! ALU SUBFAMILY SQ WARNING ENTRY !!!!//1.9e-12:51:54//HOMO SAPIENS (HUMAN).//P39194
F-HEMBB1001585
F-HEMBB1001586
F-HEMBB1001588//HYPOTHETICAL 12.3 KD PROTEIN IN GAP1-NAP1 INTERGENIC REGION.//0.0031:31:48//SACCHAROMYCES CEREVISIAE (BAKER'S YEAST).//P36140
F-HEMBB1001603
F-HEMBB1001618//RETROVIRUS-RELATED POL POLYPROTEIN [CONTAINS: REVERSE TRANSCRIPTASE (EC 2.7.7.49); ENDONUCLEASE].//0.00076:47:44//MUS MUSCULUS (MOUSE).//P11369
F-HEMBB1001619//SMALL PROLINE RICH PROTEIN II (SPR-II) (CLONE 930).//1.0:52:32//HOMO SAPIENS (HUMAN).//P22531
F-HEMBB1001630
F-HEMBB1001635//METALLOTHIONEIN-LIKE PROTEIN TYPE 2 A.//1.0:27:44//LYCOPERSICON ESCULENTUM (TOMATO).//Q40157
F-HEMBB1001637//!!!! ALU SUBFAMILY J WARNING ENTRY !!!!//0.0042:26:73//HOMO SAPIENS (HUMAN).//P39188
F-HEMBB1001641
F-HEMBB1001653//SURVIVAL MOTOR NEURON PROTEIN 1.//0.51:36:47//CANIS FAMILIARIS (DOG).//O02771
F-HEMBB1001665//HOMEOBOX PROTEIN ENGRAILED-1 (HU-EN-1).//0.0030:135:34//HOMO SAPIENS (HUMAN).//Q05925
F-HEMBB1001668//PROBABLE 60S RIBOSOMAL PROTEIN L39.//0.99:25:44//CAENORHABDITIS ELEGANS.//P52814
F-HEMBB1001673//HYPOTHETICAL 46.1 KD PROTEIN IN ERP5-ORC6 INTERGENIC REGION.//0.0054:128:34//SACCHAROMYCES CEREVISIAE (BAKER'S YEAST).//P38823.
F-HEMBB1001684//SUPPRESSOR PROTEIN SRP40.//0.56:81:34//SACCHAROMYCES CEREVISIAE (BAKER'S YEAST).//P32583
F-HEMBB1001685//CYTOCHROME C OXIDASE POLYPEPTIDE VIII-HEART PRECURSOR (EC 1.9.3.1) (VIIIB) (IX).//1.0:21:47//BOS TAURUS (BOVINE).//P10175
F-HEMBB1001695//MYOSIN IC HEAVY CHAIN.//8.9e-05:86:40//ACANTHAMOEBA CASTELLANII (AMOEBA).//P10569
F-HEMBB1001704//!!!! ALU SUBFAMILY SX WARNING ENTRY !!!!//9.0e-08:35:71//HOMO SAPIENS (HUMAN).//P39195
F-HEMBB1001706//CORNIFIN (SMALL PROLINE-RICH PROTEIN I) (SPR-I) (SMALL PROLINE-RICH SQUAMOUS CELL MARKER) (SPRP).//0.91:39:41//SUS SCROFA (PIG).//P35323
F-HEMBB1001707//FERREDOXIN-LIKE PROTEIN IN NIF REGION.//1.0:43:23//BRADYRHIZOBIUM JAPONICUM.//P27394
F-HEMBB1001717//NADH-UBIQUINONE OXIDOREDUCTASE CHAIN 4 (EC 1.6.5.3) (FRAGMENT).//1.0:71:25//LEMUR CATTA (RING-TAILED LEMUR).//Q34878
F-HEMBB1001735//!!!! ALU SUBFAMILY SQ WARNING ENTRY !!!!//9.0e-35:97:74//HOMO SAPIENS (HUMAN).//P39194
F-HEMBB1001736//EUKARYOTIC TRANSLATION INITIATION FACTOR 3 BETA SUBUNIT (EIF-3 BETA) (EIF3 P116) (EIF3 P110).//0.00069:180:28//HOMO SAPIENS (HUMAN).//P55884
F-HEMBB1001747
F-HEMBB1001749//!!!! ALU SUBFAMILY SX WARNING ENTRY !!!!//1.8e-43:75:70//HOMO SAPIENS (HUMAN).//P39195
F-HEMBB1001753//PROTEIN Q300.//0.00091:16:81//MUS MUSCULUS (MOUSE).//Q02722
F-HEMBB1001756//CYCLIN-DEPENDENT KINASES REGULATORY SUBUNIT 2 (XE-P9).//0.94:35:42//XENOPUS LAEVIS (AFRICAN CLAWED FROG).//Q91879
F-HEMBB1001760
F-HEMBB1001762//GENE 35 PROTEIN (GP35).//0.76:21:47//MYCOBACTERIOPHAGE L5.//Q05245
F-HEMBB1001785
F-HEMBB1001797//CHLOROPLAST 50S RIBOSOMAL PROTEIN L35.//0.99:41:31//PORPHYRA PURPUREA.//P51270
F-HEMBB1001802
F-HEMBB1001812//!!!! ALU SUBFAMILY SP WARNING ENTRY !!!!//2.2e-39:54:77//HOMO SAPIENS (HUMAN).//P39193
F-HEMBB1001816//!!!! ALU SUBFAMILY SQ WARNING ENTRY !!!!//1.1e-19:97:57//HOMO SAPIENS (HUMAN).//P39194
F-HEMBB1001831//HYPOTHETICAL 45.6 KD PROTEIN IN COX5A-ALG11 INTERGENIC REGION.//0.62:204:23//SACCHAROMYCES CEREVISIAE (BAKER'S YEAST).//P53951
F-HEMBB1001834//GLYCINE-RICH RNA-BINDING PROTEIN 1 (FRAGMENT).//0.0014:40:45//SORGHUM VULGARE (SORGHUM).//Q99069
F-HEMBB1001836//!!!! ALU SUBFAMILY SB2 WARNING ENTRY !!!!//7.1e-14:85:61//HOMO SAPIENS (HUMAN).//P39191
F-HEMBB1001839//PROBABLE E4 PROTEIN.//0.61:49:34//HUMAN PAPILLOMAVIRUS TYPE 6C.//P20969
F-HEMBB1001850
F-HEMBB1001863//!!!! ALU SUBFAMILY SQ WARNING ENTRY !!!!//2.7e-30:57:68//HOMO SAPIENS (HUMAN).//P39194
F-HEMBB1001867
F-HEMBB1001868//EXTENSIN PRECURSOR (CELL WALL HYDROXYPROLINE-RICH GLYCOPROTEIN).//0.00036:47:53//NICOTIANA TABACUM (COMMON TOBACCO).//P13983
F-HEMBB1001869//!!!! ALU SUBFAMILY J WARNING ENTRY !!!!//7.0e-11:95:45//HOMO SAPIENS (HUMAN).//P39188
F-HEMBB1001872//HYPOTHETICAL 8.2 KD PROTEIN IN LEF8-FP INTERGENIC REGION.//1.0:34:38//AUTOGRAPHA CALIFORNICA NUCLEAR POLYHEDROSIS VIRUS (ACMNPV).//P41459
F-HEMBB1001874
F-HEMBB1001875
F-HEMBB1001880
F-HEMBB1001899//GENE 11 PROTEIN.//1.0:45:31//SPIROPLASMA VIRUS SPV1-R8A2 B.//P15902
F-HEMBB1001905//HYPOTHETICAL 81.7 KD PROTEIN IN MOL1-NAT2 INTERGENIC REGION.//8.8e-54:216:51//SACCHAROMYCES CEREVISIAE (BAKER'S YEAST).//P48234
F-HEMBB1001906
F-HEMBB1001908//MONOCYTIC LEUKEMIA ZINC FINGER PROTEIN.//6.3e-51:138:80//HOMO SAPIENS (HUMAN).//Q92794
F-HEMBB1001910
F-HEMBB1001911
F-HEMBB1001915//UBIQUITIN CARBOXYL-TERMINAL HYDROLASE 64E (EC 3.1.2.15) (UBIQUITIN THIOLESTERASE 64E) (UBIQUITIN-SPECIFIC PROCESSING PROTEASE 64E) (DEUBIQUITINATING ENZYME 64E).//2.3e-27:71:70//DROSOPHILA MELANOGASTER (FRUIT FLY).//Q24574
F-HEMBB1001921//LINE-1 REVERSE TRANSCRIPTASE HOMOLOG.//9.8e-13:75:53//HOMO SAPIENS (HUMAN).//P08547
F-HEMBB1001922
F-HEMBB1001925//EPITHELIAL MEMBRANE PROTEIN-1 (EMP-1) (TUMOR-ASSOCIATED MEMBRANE PROTEIN).//1.0:55:30//MUS MUSCULUS (MOUSE).//P47801
F-HEMBB1001930//HYPOTHETICAL 9.6 KD PROTEIN K10D2.7 IN CHROMOSOME III.//0.43:49:26//CAENORHABDITIS ELEGANS.//Q09412
F-HEMBB1001944//!!!! ALU SUBFAMILY SB WARNING ENTRY !!!!//5.1e-34:63:85//HOMO SAPIENS (HUMAN).//P39189
F-HEMBB1001945//NONSPECIFIC LIPID-TRANSFER PROTEIN (LTP) (PHOSPHOLIPID TRANSFER PROTEIN) (PLTP).//0.28:45:40//AMARANTHUS CAUDATUS (LOVE-LIES-BLEEDING) (INCA-WHEAT).//P80450
F-HEMBB1001947//PROTEIN UL24.//0.48:42:47//HERPES SIMPLEX VIRUS (TYPE 1 / STRAIN 17).//P10208
F-HEMBB1001950//HYPOTHETICAL 42.6 KD PROTEIN IN GSHB-ANSB INTERGENIC REGION (O378).//1.6e-24:162:36//ESCHERICHIA COLI.//P52062
F-HEMBB1001952
F-HEMBB1001953
F-HEMBB1001957//!!!! ALU SUBFAMILY J WARNING ENTRY !!!!//2.7e-11:51:60//HOMO SAPIENS (HUMAN).//P39188
F-HEMBB1001962//!!!! ALU SUBFAMILY J WARNING ENTRY !!!!//7.6e-24:163:42//HOMO SAPIENS (HUMAN).//P39188
F-HEMBB1001967//!!!! ALU SUBFAMILY SB WARNING ENTRY !!!!//2.1e-35:55:80//HOMO SAPIENS (HUMAN).//P39189
F-HEMBB1001973//!!!! ALU SUBFAMILY SC WARNING ENTRY !!!!//2.1e-37:108:75//HOMO SAPIENS (HUMAN).//P39192
F-HEMBB1001983//LYSIS PROTEIN (E PROTEIN) (GPE).//0.84:45:37//BACTERIOPHAGE ALPHA-3.//P31280
F-HEMBB1001988
F-HEMBB1001990
F-HEMBB1001996//LINE-1 REVERSE TRANSCRIPTASE HOMOLOG.//1.4e-14:98:40//HOMO SAPIENS (HUMAN).//P08547
F-HEMBB1001997//!!!! ALU SUBFAMILY J WARNING ENTRY !!!!//4.1e-19:38:73//HOMO SAPIENS (HUMAN).//P39188
F-HEMBB1002002//CYTOCHROME C BIOGENESIS PROTEIN CCSA.//1.0:150:25//PORPHYRA PURPUREA.//P51369
F-HEMBB1002005//!!!! ALU SUBFAMILY SX WARNING ENTRY !!!!//7.6e-12:94:40//HOMO SAPIENS (HUMAN).//P39195
F-HEMBB1002009
F-HEMBB1002015//HYPOTHETICAL 7.7 KD PROTEIN IN MRR-TSR INTERGENIC REGION (F67).//1.0:17:47//ESCHERICHIA COLI.//P39395
F-HEMBB1002042//CYTOCHROME P450 4C1 (EC 1.14.14.1) (CYPIVC1).//2.4e-50:139:55//BLABERUS DISCOIDALIS (TROPICAL COCKROACH).//P29981
F-HEMBB1002043//HYPOTHETICAL 9.5 KD PROTEIN IN DHFR 3'REGION (ORF3).//0.052:40:42//HERPESVIRUS SAIMIRI (SUBGROUP C / STRAIN 488).//P22577
F-HEMBB1002044//CELLULOSE COMPLEMENTING PROTEIN.//0.45:87:33//ACETOBACTER XYLINUM (ACETOBACTER PASTEURIANUS).//P37697
F-HEMBB1002045//ATP SYNTHASE PROTEIN 8 (EC 3.6.1.34) (A6L).//0.78:18:55//HOMO SAPIENS (HUMAN).//P03928
F-HEMBB1002049
F-HEMBB1002050//SALIVARY PROLINE-RICH PROTEIN PRECURSOR (CLONE CP7) [CONTAINS: BASIC PEPTIDE P-F] (FRAGMENT).//1.0e-06:188:27//HOMO SAPIENS (HUMAN).//P02812
F-HEMBB1002068//HOMEOBOX PROTEIN HOX-A4 (CHOX-1.4).//0.0023:56:44//GALLUS GALLUS (CHICKEN).//P17277
F-HEMBB1002069//EXTENSIN PRECURSOR (CELL WALL HYDROXYPROLINE-RICH GLYCOPROTEIN).//0.0074:134:33//NICOTIANA TABACUM (COMMON TOBACCO).//P13983
F-HEMBB1002092//ENV POLYPROTEIN PRECURSOR (COAT POLYPROTEIN) [CONTAINS: OUTER MEMBRANE PROTEIN GP70; TRANSMEMBRANE PROTEIN P20E].//2.4e-07:75:40//BABOON ENDOGENOUS VIRUS (STRAIN M7).//P10269
F-HEMBB1002094//!!!! ALU SUBFAMILY SB2 WARNING ENTRY !!!!//1.9e-24:63:82//HOMO SAPIENS (HUMAN).//P39191
F-HEMBB1002115//EC PROTEIN HOMOLOG (ZINC-METALLOTHIONEIN CLASS II).//0.94:26:42//ZEA MAYS (MAIZE).//P43401
F-HEMBB1002134//ZINC-FINGER PROTEIN NEURO-D4.//4.6e-57:176:67//RATTUS NORVEGICUS (RAT).//P56163
F-HEMBB1002139//CHLOROPLAST 50S RIBOSOMAL PROTEIN L35.//1.0:17:52//PORPHYRA PURPUREA.//P51270
F-HEMBB1002142//EARLY NODULIN 20 PRECURSOR (N-20).//0.087:52:36//MEDICAGO TRUNCATULA (BARREL MEDIC).//P93329
F-HEMBB1002152//HYPOTHETICAL 12.3 KD PROTEIN IN RPL3-RPL33 INTERGENIC REGION (ORF102).//5.8e-05:61:37//CYANOPHORA PARADOXA.//P15811
F-HEMBB1002189//HYPOTHETICAL PROTEIN UL125.//1.0:77:32//HUMAN CYTOMEGALOVIRUS (STRAIN AD169).//P16835
F-HEMBB1002190
F-HEMBB1002193//TYROSINE-PROTEIN KINASE RECEPTOR TYRO3 PRECURSOR (TYROSINE-PROTEIN KINASE RSE) (TYROSINE-PROTEIN KINASE SKY) (TYROSINE-PROTEIN KINASE DTK).//1.2e-27:59:100//HOMO SAPIENS (HUMAN).//Q06418
F-HEMBB1002217//ZINC FINGER PROTEIN 184 (FRAGMENT).//6.6e-22:106:50//HOMO SAPIENS (HUMAN).//Q99676
F-HEMBB1002218//PROTEIN Q300.//0.85:19:52//MUS MUSCULUS (MOUSE).//Q02722
F-HEMBB1002232//!!!! ALU SUBFAMILY SX WARNING ENTRY !!!!//9.6e-21:56:71//HOMO SAPIENS (HUMAN).//P39195
F-HEMBB1002247
F-HEMBB1002249//!!!! ALU SUBFAMILY SQ WARNING ENTRY !!!!//7.2e-29:93:69//HOMO SAPIENS (HUMAN).//P39194
F-HEMBB002254//!!!! ALU SUBFAMILY SQ WARNING ENTRY !!!!//2.0e-29:101:67//HOMO SAPIENS (HUMAN).//P39194
F-HEMBB1002255//NADH-UBIQUINONE OXIDOREDUCTASE CHAIN 3 (EC 1.6.5.3).//1.0:73:28//PARAMECIUM TETRAURELIA.//P15579
F-HEMBB1002266//GLUTAMIC ACID-RICH PROTEIN PRECURSOR.//0.0079:151:26//PLASMODIUM FALCIPARUM (ISOLATE FC27 / PAPUA NEW GUINEA).//P13816
F-HEMBB1002280//LINE-1 REVERSE TRANSCRIPTASE HOMOLOG.//5.2e-15:182:36//NYCTICEBUS COUCANG (SLOW LORIS).//P08548
F-HEMBB1002300
F-HEMBB1002306//!!!! ALU SUBFAMILY SX WARNING ENTRY !!!!//0.00011:26:84//HOMO SAPIENS (HUMAN).//P39195
F-HEMBB1002327//!!!! ALU SUBFAMILY SB WARNING ENTRY !!!!//4.1e-11:41:85//HOMO SAPIENS (HUMAN).//P39189
F-HEMBB1002329//HYPOTHETICAL 74.0 KD PROTEIN IN CAJ1-HOM3 INTERGENIC REGION.//9.9e-17:232:30//SACCHAROMYCES CEREVISIAE (BAKER'S YEAST).//P40032
F-HEMBB1002340
F-HEMBB1002342//HYPOTHETICAL 32.5 KD PROTEIN IN MSH6-BMH2 INTERGENIC REGION.//3.6e-40:102:57//SACCHAROMYCES CEREVISIAE (BAKER'S YEAST).//Q03835
F-HEMBB1002358//THYMIDYLATE KINASE (EC 2.7.4.9) (DTMP KINASE).//6.1e-30:63:96//HOMO SAPIENS (HUMAN).//P23919
F-HEMBB1002359//HYPOTHETICAL 7.1 KD PROTEIN C6G9.01C IN CHROMOSOME I.//0.97:28:46//SCHIZOSACCHAROMYCES POMBE (FISSION YEAST).//Q92346
F-HEMBB1002364//RETROVIRUS-RELATED POL POLYPROTEIN (FRAGMENT).//0.47:119:25//HOMO SAPIENS (HUMAN).//P12895
F-HEMBB1002371//HYPOTHETICAL 15.5 KD PROTEIN C2F7.12 IN CHROMOSOME I PRECURSOR.//3.0e-05:111:30//SCHIZOSACCHAROMYCES POMBE (FISSION YEAST).//Q09703
F-HEMBB1002381//PUTATIVE CUTICLE COLLAGEN C09G5.4.//0.34:105:34//CAENORHABDITIS ELEGANS.//Q09455
F-HEMBB1002383//ATP SYNTHASE A CHAIN (EC 3.6.1.34) (PROTEIN 6).//0.049:103:32//AQUIFEX AEOLICUS.//066566
F-HEMBB1002387//10 KD CHAPERONIN (PROTEIN CPN10) (PROTEIN GROES) (HEAT SHOCK PROTEIN 11).//0.18:75:28//RICKETTSIA TSUTSUGAMUSHI.//P16626
F-HEMBB1002409//HIGH MOBILITY GROUP PROTEIN HMG-Y.//0.014:61:36//MUS MUSCULUS (MOUSE).//P17095
F-HEMBB1002415
F-HEMBB1002425//!!!! ALU SUBFAMILY SQ WARNING ENTRY !!!!//3.8e-18:55:70//HOMO SAPIENS (HUMAN).//P39194
F-HEMBB1002442//LIN-10 PROTEIN.//5.1e-15:121:31//CAENORHABDITIS ELEGANS.//P34692
F-HEMBB1002453//!!!! ALU SUBFAMILY SB WARNING ENTRY !!!!//1.2e-32:54:75//HOMO SAPIENS (HUMAN).//P39189
F-HEMBB1002457//!!!! ALU SUBFAMILY J WARNING ENTRY !!!!//1.5e-07:31:64//HOMO SAPIENS (HUMAN).//P39188
F-HEMBB1002458//MALE SPECIFIC SPERM PROTEIN MST84DA.//0.92:28:53//DROSOPHILA MELANOGASTER (FRUIT FLY).//Q01642
F-HEMBB1002477//DNA-DIRECTED RNA POLYMERASE II LARGEST SUBUNIT (EC 2.7.7.6) (RPB1) (FRAGMENT).//0.0066:198:27//CRICETULUS GRISEUS (CHINESE HAMSTER).//P11414
F-HEMBB1002489//SPLICEOSOME ASSOCIATED PROTEIN 49 (SAP 49) (SF3B53).//0.030:182:28//HOMO SAPIENS (HUMAN).//Q15427
F-HEMBB1002492
F-HEMBB1002495//!!!! ALU SUBFAMILY SC WARNING ENTRY !!!!//2.1e-08:41:75//HOMO SAPIENS (HUMAN).//P39192
F-HEMBB1002502//RETROVIRUS-RELATED POL POLYPROTEIN (FRAGMENT).//0.00030:31:77//HOMO SAPIENS (HUMAN).//P12895
F-HEMBB1002509
F-HEMBB1002510
F-HEMBB1002520//LINE-1 REVERSE TRANSCRIPTASE HOMOLOG.//6.8e-36:162:50//NYCTICEBUS COUCANG (SLOW LORIS).//P08548
F-HEMBB1002522//7 KD PROTEIN (ORF 4).//0.77:32:40//CHRYSANTHEMUM VIRUS B (CVB).//P37990
F-HEMBB1002531
F-HEMBB1002534//!!!! ALU SUBFAMILY SX WARNING ENTRY !!!!//3.1e-36:80:73//HOMO SAPIENS (HUMAN).//P39195
F-HEMBB1002545
F-HEMBB1002550//HOMEOBOX PROTEIN HOX-D11 (HOX-4.6) (HOX-5.5).//3.8e-05:83:34//MUS MUSCULUS (MOUSE).//P23813
F-HEMBB1002556
F-HEMBB1002579//SPLICING FACTOR U2AF 35 KD SUBUNIT (U2 AUXILIARY FACTOR 35 KD SUBUNIT) (U2 SNRNP AUXILIARY FACTOR SMALL SUBUNIT) (FRAGMENT).//5.0e-06:27:77//SUS SCROFA (PIG).//Q29350
F-HEMBB1002582//PROTEINASE INHIBITOR.//1.0:27:40//SOLANUM MELONGENA (EGGPLANT) (AUBERGINE).//P01078
F-HEMBB1002590//HYPOTHETICAL PROTEIN IN MMSB 3'REGION (ORF1) (FRAGMENT).//1.9e-20:90:54//PSEUDOMONAS AERUGINOSA.//P28812
F-HEMBB1002596
F-HEMBB1002600//NOVEL ANTIGEN 2 (NAG-2).//1.9e-60:187:59//HOMO SAPIENS (HUMAN).//O14817
F-HEMBB1002601//M PROTEIN, SEROTYPE 6 PRECURSOR.//1.0:71:35//STREPTOCOCCUS PYOGENES.//P08089
F-HEMBB1002603
F-HEMBB1002607//SALIVARY PROLINE-RICH PROTEIN PO (ALLELE K) [CONTAINS: PEPTIDE P-D] (FRAGMENT).//0.0032:142:33//HOMO SAPIENS (HUMAN).//P10162
F-HEMBB1002610//LINE-1 REVERSE TRANSCRIPTASE HOMOLOG.//2.0e-11:79:49//HOMO SAPIENS (HUMAN).//P08547
F-HEMBB1002613//!!!! ALU SUBFAMILY J WARNING ENTRY !!!!//3.9e-08:41:60//HOMO SAPIENS (HUMAN).//P39188
F-HEMBB1002614//HYPOTHETICAL 9.5 KD PROTEIN.//1.0:40:35//VACCINIA VIRUS (STRAIN COPENHAGEN).//P20553
F-HEMBB1002617//INSECT TOXIN 1 (BOT IT1).//1.0:44:29//BUTHUS OCCITANUS TUNETANUS (COMMON EUROPEAN SCORPION).//P55902
F-HEMBB1002623//HYPOTHETICAL 9.7 KD PROTEIN (ORF88) (PUTATIVE DNA-BINDING PROTEIN).//0.42:31:54//BACTERIOPHAGE P4.//P12552
F-HEMBB1002635//STRESS-ACTIVATED PROTEIN KINASE JNK3 (EC 2.7.1.-) (C-JUN N-TERMINAL KINASE 3) (MAP KINASE P49 3F12).//6.2e-17:44:95//HOMO SAPIENS (HUMAN).//P53779
F-HEMBB1002664//SMALL NUCLEAR RIBONUCLEOPROTEIN ASSOCIATED PROTEIN B (SM-B) (SNRNP-B) (SM11) (FRAGMENT).//1.0:57:36//RATTUS NORVEGICUS (RAT).//P17136
F-HEMBB1002677//LINE-1 REVERSE TRANSCRIPTASE HOMOLOG.//1.9e-06:194:34//NYCTICEBUS COUCANG (SLOW LORIS).//P08548
F-HEMBB1002683//NADH-UBIQUINONE OXIDOREDUCTASE CHAIN 5 (EC 1.6.5.3) (FRAGMENT).//0.96:56:35//LEMUR CATTA (RING-TAILED LEMUR).//Q34879
F-HEMBB1002684//SILLUCIN.//1.0:16:50//RHIZOMUCOR PUSILLUS.//P02885
F-HEMBB1002686
F-HEMBB1002692
F-HEMBB1002697//HELIX-DESTABILIZING PROTEIN (SINGLE-STRANDED DNA BINDING PROTEIN) (GPV).//0.57:36:38//BACTERIOPHAGE FD, BACTERIOPHAGE F1, AND BACTERIOPHAGE M13.//P03669
F-HEMBB1002699
F-HEMBB1002702
F-HEMBB1002705//HYPOTHETICAL 34.8 KD PROTEIN C4H3.04C IN CHROMOSOME I.//3.6e-40:180:37//SCHIZOSACCHAROMYCES POMBE (FISSION YEAST).//Q10212
F-HEMBB1002712
F-MAMMA1000009//!!!! ALU SUBFAMILY SB WARNING ENTRY !!!!//1.2e-32:95:75//HOMO SAPIENS (HUMAN).//P39189
F-MAMMA1000019
F-MAMMA1000020//DIMETHYLANILINE MONOOXYGENASE [N-OXIDE FORMING] 5 (EC 1.14.13.8) (HEPATIC FLAVIN-CONTAINING MONOOXYGENASE 5) (FMO 5) (DIMETHYLANILINE OXIDASE 5).//5.2e-12:24:100//HOMO SAPIENS (HUMAN).//P49326
F-MAMMA1000025//BETA-2-MICROGLOBULIN PRECURSOR.//1.0:73:26//BRACHYDANIO RERIO (ZEBRAFISH) (ZEBRA DANIO).//Q04475
F-MAMMA1000043//HYPOTHETICAL PXBL-I PROTEIN (FRAGMENT).//0.057:130:31//BOVINE LEUKEMIA VIRUS (JAPANESE ISOLATE BLV-1) (BLV).//P03412
F-MAMMA1000045
F-MAMMA1000055//TESTIN 2 (TES2) [CONTAINS: TESTIN 1 (TES1)].//7.5e-44:138:55//MUS MUSCULUS (MOUSE).//P47226
F-MAMMA1000057//!!!! ALU SUBFAMILY SQ WARNING ENTRY !!!!//1.2e-39:92:69//HOMO SAPIENS (HUMAN).//P39194
F-MAMMA1000069//HYPOTHETICAL 29.3 KD PROTEIN (ORF92).//0.0044:96:34//ORGYIA PSEUDOTSUGATA MULTICAPSID POLYHEDROSIS VIRUS (OPMNPV).//O10341
F-MAMMA1000084//!!!! ALU SUBFAMILY SX WARNING ENTRY !!!!//5.4e-28:94:73//HOMO SAPIENS (HUMAN).//P39195
F-MAMMA1000085//PUTATIVE CYSTEINYL-TRNA SYNTHETASE C29E6.06C (EC 6.1.1.16) (CYSTEINE-- TRNA LIGASE) (CYSRS).//6.6e-38:90:51//SCHIZOSACCHAROMYCES POMBE (FISSION YEAST).//Q09860
F-MAMMA1000092//!!!! ALU SUBFAMILY SC WARNING ENTRY !!!!//6.4e-30:43:86//HOMO SAPIENS (HUMAN).//P39192
F-MAMMA1000103//SMALL PROLINE RICH PROTEIN II (SPR-II) (CLONE 930).//0.038:17:52//HOMO SAPIENS (HUMAN).//P22531
F-MAMMA1000117//50S RIBOSOMAL PROTEIN L24E (HL21/HL22).//0.90:25:48//HALOARCULA MARISMORTUI (HALOBACTERIUM MARISMORTUI).//P14116
F-MAMMA1000129//HYPOTHETICAL BHLF1 PROTEIN.//0.0016:75:40//EPSTEIN-BARR VIRUS (STRAIN B95-8) (HUMAN HERPESVIRUS 4).//P03181
F-MAMMA1000133
F-MAMMA1000134//HYPOTHETICAL PROTEIN MJ0647.//1.0:41:41//METHANOCOCCUS JANNASCHII.//Q58063
F-MAMMA1000139//GUANINE NUCLEOTIDE-BINDING PROTEIN G(I)/G(S)/G(O) GAMMA-3 SUBUNIT.//0.99:69:28//BOS TAURUS (BOVINE), AND MUS MUSCULUS (MOUSE).//P29798
F-MAMMA1000143//CALPAIN INHIBITOR (CALPASTATIN) (FRAGMENT).//0.023:111:27//MUS MUSCULUS (MOUSE).//P51125
F-MAMMA1000155//PUTATIVE CUTICLE COLLAGEN C09G5.5.//0.018:125:34//CAENORHABDITIS ELEGANS.//Q09456
F-MAMMA1000163//MERCURIC TRANSPORT PROTEIN PERIPLASMIC COMPONENT PRECURSOR (PERIPLASMIC MERCURY ION BINDING PROTEIN) (MERCURY SCAVENGER PROTEIN).//0.11:88:25//SHEWANELLA PUTREFACIENS (PSEUDOMONAS PUTREFACIENS).//Q54463
F-MAMMA1000171
F-MAMMA1000173//DREBRIN E.//7.6e-41:197:43//HOMO SAPIENS (HUMAN).//Q16643
F-MAMMA1000175//GAMMA-THIONIN HOMOLOG PPT PRECURSOR.//0.92:39:38//PETUNIA INTEGRIFOLIA (VIOLET-FLOWERED PETUNIA) (PETUNIA INFLATA).//Q40901
F-MAMMA1000183//ZINC FINGER PROTEIN 84 (ZINC FINGER PROTEIN HPF2).//2.4e-106:249:61//HOMO SAPIENS (HUMAN).//P51523
F-MAMMA1000198//MALE SPECIFIC SPERM PROTEIN MST84DD.//0.0014:35:42//DROSOPHILA MELANOGASTER (FRUIT FLY).//Q01645
F-MAMMA1000221
F-MAMMA1000227//6.8 KD MITOCHONDRIAL PROTEOLIPID.//1.0:30:40//MUS MUSCULUS (MOUSE).//P56379
F-MAMMA1000241//PHOTOSYSTEM I REACTION CENTRE SUBUNIT X (PSI-K).//1.0:40:37//PORPHYRA PURPUREA.//P51370
F-MAMMA1000251//HYPOTHETICAL 6.8 KD PROTEIN IN FIC-PPIA INTERGENIC REGION.//0.99:29:48//SALMONELLA TYPHIMURIUM.//P37771
F-MAMMA1000254//HYPOTHETICAL 6.0 KD PROTEIN IN THI12 5'REGION.//1.0:20:50//SACCHAROMYCES CEREVISIAE (BAKER'S YEAST).//P53820
F-MAMMA1000257//HYPOTHETICAL 50.0 KD PROTEIN IN HEML 3'REGION (ORF2).//0.22:50:44//PSEUDOMONAS AERUGINOSA.//Q51470
F-MAMMA1000264//GASTRIN-RELEASING PEPTIDE RECEPTOR (GRP-R) (GRP-PREFERRING BOMBESIN RECEPTOR).//0.80:39:43//HOMO SAPIENS (HUMAN).//P30550
F-MAMMA1000266
F-MAMMA1000270//!!!! ALU SUBFAMILY SB WARNING ENTRY !!!!//9.5e-42:95:84//HOMO SAPIENS (HUMAN).//P39189
F-MAMMA1000277//PROCOLLAGEN ALPHA 1(II) CHAIN PRECURSOR [CONTAINS: CHONDROCALCIN].//0.0062:90:34//MUS MUSCULUS (MOUSE).//P28481
F-MAMMA1000278//C-HORDEIN (CLONE PC HOR1-3) (FRAGMENT).//0.00096:59:33//HORDEUM VULGARE (BARLEY).//P17991
F-MAMMA1000279//!!!! ALU SUBFAMILY SX WARNING ENTRY !!!!//8.4e-17:56:76//HOMO SAPIENS (HUMAN).//P39195
F-MAMMA1000284//ARYL HYDROCARBON RECEPTOR NUCLEAR TRANSLOCATOR 2 (ARNT PROTEIN 2).//0.017:146:30//MUS MUSCULUS (MOUSE).//Q61324
F-MAMMA1000287//!!!! ALU SUBFAMILY SB WARNING ENTRY !!!!//1.5e-32:84:58//HOMO SAPIENS (HUMAN).//P39189
F-MAMMA1000302//C-HORDEIN (CLONE PC-919) (FRAGMENT).//1.0:42:33//HORDEUM VULGARE (BARLEY).//P17992
F-MAMMA1000307//PROBABLE E4 PROTEIN.//0.21:71:30//RHESUS PAPILLOMAVIRUS TYPE 1 (RHPV 1).//P24832
F-MAMMA1000309//COLLAGEN ALPHA 1(VIII) CHAIN PRECURSOR (ENDOTHELIAL COLLAGEN).//0.0026:141:36//HOMO SAPIENS (HUMAN).//P27658
F-MAMMA1000312
F-MAMMA1000313//DNA REPAIR PROTEIN RADC HOMOLOG (25 KD PROTEIN) (FRAGMENT).//0.76:52:32//STAPHYLOCOCCUS AUREUS.//P31337
F-MAMMA1000331
F-MAMMA1000339//50S RIBOSOMAL PROTEIN L29P.//0.78:32:46//METHANOBACTERIUM THERMOAUTOTROPHICUM.//O26117
F-MAMMA1000340//HYPOTHETICAL 29.4 KD PROTEIN IN STE6-LOS1 INTERGENIC REGION.//1.0:29:58//SACCHAROMYCES CEREVISIAE (BAKER'S YEAST).//P36039
F-MAMMA1000348//!!!! ALU SUBFAMILY J WARNING ENTRY !!!!//7.5e-09:63:60//HOMO SAPIENS (HUMAN).//P39188
F-MAMMA1000356//!!!! ALU SUBFAMILY J WARNING ENTRY !!!!//3.3e-05:42:52//HOMO SAPIENS (HUMAN).//P39188
F-MAMMA1000360
F-MAMMA1000361//!!!! ALU SUBFAMILY SB WARNING ENTRY !!!!//4.4e-33:84:72//HOMO SAPIENS (HUMAN).//P39189
F-MAMMA1000372//!!!! ALU SUBFAMILY SP WARNING ENTRY !!!!//6.6e-21:53:71//HOMO SAPIENS (HUMAN).//P39193
F-MAMMA1000385
F-MAMMA1000388//OX40L RECEPTOR PRECURSOR (ACT35 ANTIGEN) (TAX-TRANSCRIPTIONALLY ACTIVATED GLYCOPROTEIN 1 RECEPTOR) (CD134 ANTIGEN).//0.40:72:36//HOMO SAPIENS (HUMAN).//P43489
F-MAMMA1000395//RABPHILIN-3A (FRAGMENT).//0.032:125:25//MUS MUSCULUS (MOUSE).//P47708
F-MAMMA1000402//LINE-1 REVERSE TRANSCRIPTASE HOMOLOG.//3.1e-28:266:40//HOMO SAPIENS (HUMAN).//P08547
F-MAMMA1000410//NADH-UBIQUINONE OXIDOREDUCTASE 13 KD-B SUBUNIT (EC 1.6.5.3) (EC 1.6.99.3) (COMPLEX I-13KD-B) (CI-13KD-B) (B13).//5.9e-06:32:68//HOMO SAPIENS (HUMAN).//Q16718
F-MAMMA1000413//RETROVIRUS-RELATED POL POLYPROTEIN [CONTAINS: REVERSE TRANSCRIPTASE (EC 2.7.7.49); ENDONUCLEASE].//6.7e-05:93:31//MUS MUSCULUS (MOUSE).//P11369
F-MAMMA1000414
F-MAMMA1000416//HYPOTHETICAL 32.0 KD PROTEIN C09F5.2 IN CHROMOSOME III.//4.1e-28:119:53//CAENORHABDITIS ELEGANS.//Q09232
F-MAMMA1000421//!!!! ALU SUBFAMILY SQ WARNING ENTRY !!!!//2.7e-23:68:76//HOMO SAPIENS (HUMAN).//P39194
F-MAMMA1000422//METALLOTHIONEIN (MT).//0.037:42:42//GADUS MORHUA (ATLANTIC COD).//P51902
F-MAMMA1000423
F-MAMMA1000424//!!!! ALU SUBFAMILY SB WARNING ENTRY !!!!//0.048:23:73//HOMO SAPIENS (HUMAN).//P39189
F-MAMMA1000429//VACUOLAR PROTEIN SORTING-ASSOCIATED PROTEIN VPS5.//2.7e-05:110:30//SACCHAROMYCES CEREVISIAE (BAKER'S YEAST).//Q92331
F-MAMMA1000431//!!!! ALU SUBFAMILY SQ WARNING ENTRY !!!!//9.4e-15:85:58//HOMO SAPIENS (HUMAN).//P39194
F-MAMMA1000444//!!!! ALU SUBFAMILY SQ WARNING ENTRY !!!!//4.3e-25:65:76//HOMO SAPIENS (HUMAN).//P39194
F-MAMMA1000446//ZYXIN.//0.79:155:29//GALLUS GALLUS (CHICKEN).//Q04584
F-MAMMA1000458//HYPOTHETICAL 37.7 KD PROTEIN C18B11.06 IN CHROMOSOME I.//0.0048:46:43//SCHIZOSACCHAROMYCES POMBE (FISSION YEAST).//Q09713
F-MAMMA1000468//PERIOD CLOCK PROTEIN (FRAGMENT).//0.50:20:55//DROSOPHILA ROBUSTA (FRUIT FLY).//Q03296
F-MAMMA1000472//!!!! ALU SUBFAMILY J WARNING ENTRY !!!!//1.5e-17:106:55//HOMO SAPIENS (HUMAN).//P39188
F-MAMMA1000478//!!!! ALU SUBFAMILY SX WARNING ENTRY !!!!//3.9e-35:80:68//HOMO SAPIENS (HUMAN).//P39195
F-MAMMA1000483//!!!! ALU SUBFAMILY SP WARNING ENTRY !!!!//2.8e-24:74:77//HOMO SAPIENS (HUMAN).//P39193
F-MAMMA1000490//TYROSINE-PROTEIN KINASE TXK (EC 2.7.1.112) (PTK-RL-18) (RESTING LYMPHOCYTE KINASE).//0.43:21:57//MUS MUSCULUS (MOUSE).//P42682
F-MAMMA1000500//TAT PROTEIN (TRANSACTIVATING REGULATORY PROTEIN).//0.61:33:54//HUMAN IMMUNODEFICIENCY VIRUS TYPE 1 (Z2/CDC-Z34 ISOLATE) (HIV-1).//P12506
F-MAMMA1000501//!!!! ALU SUBFAMILY SQ WARNING ENTRY !!!!//1.1e-32:43:83//HOMO SAPIENS (HUMAN).//P39194
F-MAMMA1000516
F-MAMMA1000522//LINE-1 REVERSE TRANSCRIPTASE HOMOLOG.//0.0015:113:32//HOMO SAPIENS (HUMAN).//P08547
F-MAMMA1000524//HYPOTHETICAL HOST RANGE 8.5 KD PROTEIN.//1.0:63:31//VACCINIA VIRUS (STRAIN WR).//P17359
F-MAMMA1000559//METALLOTHIONEIN-I (MT-I) (MT-IB/MT-IA).//0.31:16:50//CALLINECTES SAPIDUS (BLUE CRAB).//P55949
F-MAMMA1000565//FERREDOXIN-TYPE PROTEIN NAPF.//0.98:37:35//ESCHERICHIA COLI.//P33939
F-MAMMA1000567//!!!! ALU SUBFAMILY SX WARNING ENTRY !!!!//5.5e-37:95:76//HOMO SAPIENS (HUMAN).//P39195
F-MAMMA1000576//!!!! ALU SUBFAMILY SB2 WARNING ENTRY !!!!//4.1e-07:34:64//HOMO SAPIENS (HUMAN).//P39191
F-MAMMA1000583
F-MAMMA1000585//!!!! ALU SUBFAMILY SQ WARNING ENTRY !!!!//1.5e-28:89:75//HOMO SAPIENS (HUMAN).//P39194
F-MAMMA1000594//!!!! ALU SUBFAMILY SX WARNING ENTRY !!!!//4.8e-24:38:71//HOMO SAPIENS (HUMAN).//P39195
F-MAMMA1000597//!!!! ALU SUBFAMILY SX WARNING ENTRY !!!!//1.1e-25:74:77//HOMO SAPIENS (HUMAN).//P39195
F-MAMMA1000605//!!!! ALU SUBFAMILY SX WARNING ENTRY !!!!//2.1e-18:83:50//HOMO SAPIENS (HUMAN).//P39195
F-MAMMA1000612//HYPOTHETICAL 34.0 KD TRP-ASP REPEATS CONTAINING PROTEIN IN SIS1-MRPL2 INTERGENIC REGION.//4.0e-42:166:48//SACCHAROMYCES CEREVISIAE (BAKER'S YEAST).//P41318
F-MAMMA1000616
F-MAMMA1000621
F-MAMMA1000623//METALLOTHIONEIN-IK (MT-1K).//0.0045:25:48//HOMO SAPIENS (HUMAN).//P80296
F-MAMMA1000625//PROLINE-RICH PROTEIN MP-3 (FRAGMENT).//0.00078:79:35//MUS MUSCULUS (MOUSE).//P05143
F-MAMMA1000643//HYPOTHETICAL 9.3 KD PROTEIN.//1.0:25:28//MAGUARI VIRUS.//P16607
F-MAMMA1000664
F-MAMMA1000669//LINE-1 REVERSE TRANSCRIPTASE HOMOLOG.//4.2e-05:186:30//HOMO SAPIENS (HUMAN).//P08547
F-MAMMA1000670//PROLINE-RICH PROTEIN MP-3 (FRAGMENT).//1.6e-06:195:30//MUS MUSCULUS (MOUSE).//P05143
F-MAMMA1000672//VITELLOGENIC CARBOXYPEPTIDASE PRECURSOR (EC 3.4.16.-).//3.8e-28:184:35//AEDES AEGYPTI (YELLOWFEVER MOSQUITO).//P42660
F-MAMMA1000684//DNA-BINDING PROTEIN (VMW21).//1.1e-07:55:56//HERPES SIMPLEX VIRUS (TYPE 1 / STRAIN 17).//P04487
F-MAMMA1000696//!!!! ALU SUBFAMILY SQ WARNING ENTRY !!!!/1.2e-31:97:74//HOMO SAPIENS (HUMAN).//P39194
F-MAMMA1000707//METALLOTHIONEIN-II (MT-II) (MT-IIB/MT-IIA).//0.31:19:42//CALLINECTES SAPIDUS (BLUE CRAB).//P55950
F-MAMMA1000713//XYLULOSE KINASE (EC 2.7.1.17) (XYLULOKINASE).//1.6e-05:88:35//LACTOBACILLUS PENTOSUS.//P21939
F-MAMMA1000714//PROTEIN-LYSINE 6-OXIDASE PRECURSOR (EC 1.4.3.13) (LYSYL OXIDASE).//0.44:126:30//RATTUS NORVEGICUS (RAT).//P16636
F-MAMMA1000718//METALLOTHIONEIN-IIE (MT-2E).//1.0:51:31//ORYCTOLAGUS CUNICULUS (RABBIT).//P80292
F-MAMMA1000720//!!!! ALU SUBFAMILY SP WARNING ENTRY !!!!//3.3e-28:60:71//HOMO SAPIENS (HUMAN).//P39193
F-MAMMA1000723//LINE-1 REVERSE TRANSCRIPTASE HOMOLOG.//3.7e-14:63:53//HOMO SAPIENS (HUMAN).//P08547
F-MAMMA1000731//CHROMODOMAIN-HELICASE-DNA-BINDING PROTEIN 2 (CHD-2).//1.8e-43:258:43//HOMO SAPIENS (HUMAN).//O14647
F-MAMMA1000732//!!!! ALU SUBFAMILY J WARNING ENTRY !!!!//9.9e-12:76:55//HOMO SAPIENS (HUMAN).//P39188
F-MAMMA1000733
F-MAMMA1000734//NPL1 PROTEIN (SEC63 PROTEIN).//2.5e-18:181:39//SACCHAROMYCES CEREVISIAE (BAKER'S YEAST).//P14906
F-MAMMA1000738//HYPOTHETICAL 116.5 KD PROTEIN C20G8.09C IN CHROMOSOME I.//5.4e-52:196:58//SCHIZOSACCHAROMYCES POMBE (FISSION YEAST).//P87115
F-MAMMA1000744//!!!! ALU SUBFAMILY SB1 WARNING ENTRY !!!!//6.3e-36:144:47//HOMO SAPIENS (HUMAN).//P39190
F-MAMMA1000746
F-MAMMA1000752
F-MAMMA1000760//!!!! ALU SUBFAMILY SX WARNING ENTRY !!!!//6.6e-29:75:72//HOMO SAPIENS (HUMAN).//P39195
F-MAMMA1000761//!!!! ALU SUBFAMILY SQ WARNING ENTRY !!!!//3.6e-09:59:64//HOMO SAPIENS (HUMAN).//P39194
F-MAMMA1000775
F-MAMMA1000776//!!!! ALU SUBFAMILY SP WARNING ENTRY !!!!//3.3e-35:99:74//HOMO SAPIENS (HUMAN).//P39193
F-MAMMA1000778//!!!! ALU SUBFAMILY SX WARNING ENTRY !!!!//2.1e-19:65:70//HOMO SAPIENS (HUMAN).//P39195
F-MAMMA1000782
F-MAMMA1000798//HYPOTHETICAL PROTEIN ORF-1137.//0.015:59:37//MUS MUSCULUS (MOUSE).//P11260
F-MAMMA1000802//MYOSIN IC HEAVY CHAIN.//0.35:94:41//ACANTHAMOEBA CASTELLANII (AMOEBA).//P10569
F-MAMMA1000824//ACTIN 1.//0.046:60:31//ZEA MAYS (MAIZE).//P02582
F-MAMMA1000831//PROBABLE NI/FE-HYDROGENASE 1 B-TYPE CYTOCHROME SUBUNIT.//1.0:30:46//ESCHERICHIA COLI.//P19929
F-MAMMA1000839//!!!! ALU SUBFAMILY J WARNING ENTRY !!!!//1.1e-28:80:58//HOMO SAPIENS (HUMAN).//P39188
F-MAMMA1000841//PUTATIVE AMIDASE (EC 3.5.1.4).//1.5e-39:130:36//METHANOBACTERIUM THERMOAUTOTROPHICUM.//O27540
F-MAMMA1000842//C-HORDEIN (CLONE PC-919) (FRAGMENT).//0.064:43:41//HORDEUM VULGARE (BARLEY).//P17992
F-MAMMA1000843
F-MAMMA1000845//NADH-UBIQUINONE OXIDOREDUCTASE CHAIN 2 (EC 1.6.5.3).//0.43:58:34//DROSOPHILA YAKUBA (FRUIT FLY).//P03895
F-MAMMA1000851//CUTICLE COLLAGEN 34.//0.019:107:29//CAENORHABDITIS ELEGANS.//P34687
F-MAMMA1000855//SPLICEOSOME ASSOCIATED PROTEIN 62 (SAP 62) (SF3A66).//0.00098:149:32//HOMO SAPIENS (HUMAN).//Q15428
F-MAMMA1000856//METALLOTHIONEIN (MT).//0.63:39:41//POTAMON POTAMIOS.//P55952
F-MAMMA1000859//GLYCOPROTEIN X PRECURSOR.//0.014:192:28//EQUINE HERPESVIRUS TYPE 1 (STRAIN AB4P) (EHV-1).//P28968
F-MAMMA1000862//DISINTEGRIN KISTRIN (PLATELET AGGREGATION ACTIVATION INHIBITOR).//1.0:66:27//AGKISTRODON RHODOSTOMA (MALAYAN PIT VIPER) (CALLOSELASMA RHODOSTOMA).//P17494
F-MAMMA1000863//!!!! ALU SUBFAMILY J WARNING ENTRY !!!!//5.4e-16:41:68//HOMO SAPIENS (HUMAN).//P39188
F-MAMMA1000865//SALIVARY PROUNE-RICH PROTEIN II-1 (FRAGMENT).//0.030:100:32//HOMO SAPIENS (HUMAN).//P81489
F-MAMMA1000867//APTOTOXIN IX (PARALYTIC PEPTIDE IX) (PP IX).//0.98:43:32//APTOSTICHUS SCHLINGERI (TRAP-DOOR SPIDER).//P49272
F-MAMMA1000875//PROLINE-RICH PEPTIDE P-B.//0.18:21:47//HOMO SAPIENS (HUMAN).//P02814
F-MAMMA1000876//!!!! ALU SUBFAMILY SB WARNING ENTRY !!!!//1.5e-22:85:71//HOMO SAPIENS (HUMAN).//P39189
F-MAMMA1000877//!!!! ALU SUBFAMILY J WARNING ENTRY !!!!//1.2e-38:62:74//HOMO SAPIENS (HUMAN).//P39188
F-MAMMA1000880//COLLAGEN ALPHA 1(XII) CHAIN (FRAGMENTS).//0.49:79:32//BOS TAURUS (BOVINE).//P25508
F-MAMMA1000883//HYPOTHETICAL 6.1 KD PROTEIN C03B1.10 IN CHROMOSOME X.//0.87:15:60//CAENORHABDITIS ELEGANS.//Q11116
F-MAMMA1000897//INTER-ALPHA-TRYPSIN INHIBITOR HEAVY CHAIN H4 PRECURSOR (ITI HEAVY CHAIN H4) (INTER-ALPHA-TRYPSIN INHIBITOR FAMILY HEAVY CHAIN-RELATED PROTEIN) (PLASMA KALLIKREIN SENSITIVE GLYCOPROTEIN 120) (PK-120).//5.3e-17:130:40//HOMO SAPIENS (HUMAN).//Q14624
F-MAMMA1000905
F-MAMMA1000906
F-MAMMA1000908//LINE-1 REVERSE TRANSCRIPTASE HOMOLOG.//8.0e-17:70:62//HOMO SAPIENS (HUMAN).//P08547
F-MAMMA1000914//HYPOTHETICAL 6.2 KD PROTEIN.//0.97:36:36//THERMOPROTEUS TENAX VIRUS 1 (STRAIN KRA1) (TTV1).//P19299
F-MAMMA1000921
F-MAMMA1000931//!!!! ALU SUBFAMILY J WARNING ENTRY !!!!//5.6e-10:49:65//HOMO SAPIENS (HUMAN).//P39188
F-MAMMA1000940//MITOCHONDRIAL 60S RIBOSOMAL PROTEIN L32.//0.42:22:54//RECLINOMONAS AMERICANA.//O21281
F-MAMMA1000941//!!!! ALU SUBFAMILY J WARNING ENTRY !!!!//2.3e-25:55:69//HOMO SAPIENS (HUMAN).//P39188
F-MAMMA1000942//!!!! ALU SUBFAMILY SQ WARNING ENTRY !!!!//3.3e-08:36:75//HOMO SAPIENS (HUMAN).//P39194
F-MAMMA1000943
F-MAMMA1000956//SMALL HISTIDINE-ALANINE-RICH PROTEIN PRECURSOR (SHARP) (ANTIGEN 57).//0.041:122:25//PLASMODIUM FALCIPARUM (ISOLATE FC27 / PAPUA NEW GUINEA).//P04930
F-MAMMA1000957//HEAT-STABLE ENTEROTOXIN A2 PRECURSOR (STA2).//0.024:37:37//ESCHERICHIA COLI.//Q47185
F-MAMMA1000962//!!!! ALU SUBFAMILY SB WARNING ENTRY !!!!//6.0e-39:61:78//HOMO SAPIENS (HUMAN).//P39189
F-MAMMA1000968//!!!! ALU SUBFAMILY SQ WARNING ENTRY !!!!//0.0054:29:72//HOMO SAPIENS (HUMAN).//P39194
F-MAMMA1000975//CUTICLE COLLAGEN DPY-2 PRECURSOR.//1.0:93:30//CAENORHABDITIS ELEGANS.//P35799
F-MAMMA1000979//PROLINE-RICH PEPTIDE P-B.//0.012:12:66//HOMO SAPIENS (HUMAN).//P02814
F-MAMMA1000987//HYPOTHETICAL PROTEIN LAMBDA-SP34.//1.0:47:40//MUS MUSCULUS (MOUSE).//P15973
F-MAMMA1000998
F-MAMMA1001003//PROBABLE E5 PROTEIN.//1.0:52:42//HUMAN PAPILLOMAVIRUS TYPE 33.//P06426
F-MAMMA1001008//PROGASTRICSIN PRECURSOR (EC 3.4.23.3) (PEPSINOGEN C) (FRAGMENT).//3.2e-14:131:35//MACACA FUSCATA FUSCATA (JAPANESE MACAQUE).//P03955
F-MAMMA1001021//HYPOTHETICAL 35.5 KD PROTEIN IN TRANSPOSON TN4556.//0.016:61:42//STREPTOMYCES FRADIAE.//P20186
F-MAMMA1001024
F-MAMMA1001030//LUTROPIN-CHORIOGONADOTROPIC HORMONE RECEPTOR (LH/CG-R) (LSH-R) (LUTEINIZING HOROMINE RECEPTOR) (FRAGMENT).//2.4e-20:234:29//GALLUS GALLUS (CHICKEN).//Q90674
F-MAMMA1001035//!!!! ALU SUBFAMILY SP WARNING ENTRY !!!!//1.7e-15:52:78//HOMO SAPIENS (HUMAN).//P39193
F-MAMMA1001038//NEUROTOXIN II (TOXIN RP-II) (SODIUM CHANNEL TOXIN II).//0.53:25:48//RADIANTHUS PAUMOTENSIS (SEA ANEMONE) (HETERACTIS PAUMOTENSIS).//P01534
F-MAMMA1001041//SPECTRIN BETA CHAIN, ERYTHROCYTE.//6.3e-18:112:43//MUS MUSCULUS (MOUSE).//P15508
F-MAMMA1001050
F-MAMMA1001059//PUTATIVE ATP-DEPENDENT RNA HELICASE C12C2.06.//1.3e-34:187:47//SCHIZOSACCHAROMYCES POMBE (FISSION YEAST).//Q09747
F-MAMMA1001067//PROTEIN Q300.//0.36:12:75//MUS MUSCULUS (MOUSE).//Q02722
F-MAMMA1001073//HEPATOCYTE NUCLEAR FACTOR 3 FORKHEAD HOMOLOG 1 (HFH-1).//1.0:70:37//RATTUS NORVEGICUS (RAT).//Q63244
F-MAMMA1001074//LINE-1 REVERSE TRANSCRIPTASE HOMOLOG.//0.00067:163:32//HOMO SAPIENS (HUMAN).//P08547
F-MAMMA1001075//RETINOBLASTOMA BINDING PROTEIN 1 (RBBP-1).//0.53:72:34//HOMO SAPIENS (HUMAN).//P29374
F-MAMMA1001078//LINE-1 REVERSE TRANSCRIPTASE HOMOLOG.//5.0e-79:184:73//HOMO SAPIENS (HUMAN).//P08547
F-MAMMA1001080//IG HEAVY CHAIN PRECURSOR V-III REGION (VH26).//1.7e-27:82:71//HOMO SAPIENS (HUMAN).//P01764
F-MAMMA1001082
F-MAMMA1001091//HYPOTHETICAL BHLF1 PROTEIN.//3.1e-05:198:32//EPSTEIN-BARR VIRUS (STRAIN B95-8) (HUMAN HERPESVIRUS 4).//P03181
F-MAMMA1001092//LINE-1 REVERSE TRANSCRIPTASE HOMOLOG.//7.1e-21:65:72//HOMO SAPIENS (HUMAN).//P08547
F-MAMMA1001105//OVO PROTEIN (SHAVEN BABY PROTEIN).//1.0e-18:68:48//DROSOPHILA MELANOGASTER (FRUIT FLY).//P51521
F-MAMMA1001110//PROCOLLAGEN ALPHA 1(IV) CHAIN PRECURSOR.//0.080:108:37//MUS MUSCULUS (MOUSE).//P02463
F-MAMMA1001126//!!!! ALU SUBFAMILY SB WARNING ENTRY !!!!//2.3e-07:66:45//HOMO SAPIENS (HUMAN).//P39189
F-MAMMA1001133//HYPOTHETICAL 13.2 KD PROTEIN IN RPS4A-BAT2 INTERGENIC REGION.//0.96:43:25//SACCHAROMYCES CEREVISIAE (BAKER'S YEAST).//P47174
F-MAMMA1001139//HYPOTHETICAL 36.7 KD PROTEIN AH6.2 IN CHROMOSOME II.//5.4e-42:81:62//CAENORHABDITIS ELEGANS.//Q09201
F-MAMMA1001143//!!!! ALU SUBFAMILY J WARNING ENTRY !!!!//0.00014:36:66//HOMO SAPIENS (HUMAN).//P39188
F-MAMMA1001145
F-MAMMA1001154//CSBA PROTEIN.//1.0:39:38//BACILLUS SUBTILIS.//P37953
F-MAMMA1001161//!!!! ALU SUBFAMILY J WARNING ENTRY !!!!//5.2e-23:53:64//HOMO SAPIENS (HUMAN).//P39188
F-MAMMA1001162//CD27L RECEPTOR PRECURSOR (T-CELL ACTIVATION ANTIGEN CD27).//0.69:86:31//MUS MUSCULUS (MOUSE).//P41272
F-MAMMA1001181//HYPOTHETICAL 81.0 KD PROTEIN C35D10.4 IN CHROMOSOME III.//0.00010:74:47//CAENORHABDITIS ELEGANS.//Q18486
F-MAMMA1001186//!!!! ALU SUBFAMILY SQ WARNING ENTRY !!!!//9.0e-32:44:86//HOMO SAPIENS (HUMAN).//P39194
F-MAMMA1001191//OCTAMER-BINDING TRANSCRIPTION FACTOR 1 (OTF-1) (NF-A1) (FRAGMENT).//0.096:40:40//MACROPUS EUGENII (TAMMAR WALLABY).//Q28466
F-MAMMA1001198//EPIDERMAL GROWTH FACTOR RECEPTOR SUBSTRATE SUBSTRATE 15 (PROTEIN EPS15) (AF-1P PROTEIN).//2.5e-75:204:70//HOMO SAPIENS (HUMAN).//P42566
F-MAMMA1001202//METALLOTHIONEIN-II (MT-II) (MT-IIB/MT-IIA).//0.52:46:32//CALLINECTES SAPIDUS (BLUE CRAB).//P55950
F-MAMMA1001203//!!!! ALU SUBFAMILY SC WARNING ENTRY !!!!//7.3e-11:82:58//HOMO SAPIENS (HUMAN).//P39192
F-MAMMA1001206//!!!! ALU SUBFAMILY J WARNING ENTRY !!!!//1.9e-17:67:71//HOMO SAPIENS (HUMAN).//P39188
F-MAMMA1001215//9 KD PROTEIN.//1.0:51:33//HOMO SAPIENS (HUMAN).//P13994
F-MAMMA1001220//!!!! ALU SUBFAMILY SB WARNING ENTRY !!!!//3.4e-37:55:87//HOMO SAPIENS (HUMAN).//P39189
F-MAMMA1001222//HYPOTHETICAL 73.6 KD PROTEIN CY49.21.//3.7e-06:168:38//MYCOBACTERIUM TUBERCULOSIS.//Q10690
F-MAMMA1001243
F-MAMMA1001244//TRP OPERON LEADER PEPTIDE.//1.0:18:55//SERRATIA MARCESCENS.//P03055
F-MAMMA1001249//HYPOTHETICAL 7.2 KD PROTEIN IN RPS2 3'REGION (ORF57).//0.57:23:34//ASTASIA LONGA (EUGLENOPHYCEAN ALGA).//P34774
F-MAMMA1001256//!!!! ALU SUBFAMILY J WARNING ENTRY !!!!//2.3e-07:79:44//HOMO SAPIENS (HUMAN).//P39188
F-MAMMA1001259//PUTATIVE DNA HELICASE II HOMOLOG (EC 3.6.1.-).//0.046:86:32//MYCOPLASMA GENITALIUM.//P47486
F-MAMMA1001260//MYOSIN HEAVY CHAIN, PERINATAL SKELETAL MUSCLE.//2.7e-05:219:27//HOMO SAPIENS (HUMAN).//P13535
F-MAMMA1001268//LINE-1 REVERSE TRANSCRIPTASE HOMOLOG.//9.7e-27:89:67//HOMO SAPIENS (HUMAN).//P08547
F-MAMMA1001271//ATROPHIN-1 (DENTATORUBRAL-PALLIDOLUYSIAN ATROPHY PROTEIN).//4.0e-06:126:38//HOMO SAPIENS (HUMAN).//P54259
F-MAMMA1001274/!!!! ALU SUBFAMILY SQ WARNING ENTRY !!!!//7.4e-29:57:66//HOMO SAPIENS (HUMAN).//P39194
F-MAMMA1001280//BACTERIOCIN MICROCIN B17 PRECURSOR (MCB17).//0.27:24:54//ESCHERICHIA COLI.//P05834
F-MAMMA1001292//HYPOTHETICAL PROTEIN KIAA0176 (FRAGMENT).//1.3e-73:208:69//HOMO SAPIENS (HUMAN).//Q14681
F-MAMMA1001296//!!!! ALU SUBFAMILY SP WARNING ENTRY !!!!//6.9e-22:41:80//HOMO SAPIENS (HUMAN).//P39193
F-MAMMA1001298//HYPOTHETICAL PROTEIN HI0371.//0.99:29:37//HAEMOPHILUS INFLUENZAE.//P44668
F-MAMMA1001305//GTPASE-ACTIVATING PROTEIN RHOGAP (RHO-RELATED SMALL GTPASE PROTEIN ACTIVATOR) (CDC42 GTPASE-ACTIVATING PROTEIN) (P50-RHOGAP).//9.9e-62:222:54//HOMO SAPIENS (HUMAN).//Q07960
F-MAMMA1001322//B-CELL GROWTH FACTOR PRECURSOR (BCGF-12 KD).//2.1e-09:46:60//HOMO SAPIENS (HUMAN).//P20931
F-MAMMA1001324//POL POLYPROTEIN [CONTAINS: PROTEASE (EC 3.4.23.-); REVERSE TRANSCRIPTASE (EC 2.7.7.49); RIBONUCLEASE H (EC 3.1.26.4)].//2.5e-43:128:50//FRIEND MURINE LEUKEMIA VIRUS (ISOLATE PVC-211) (F-MULV).//P26808
F-MAMMA1001330//HEMOGLOBIN ZETA CHAIN (FRAGMENTS).//0.30:51:37//MACROPUS EUGENII (TAMMAR WALLABY).//P81044
F-MAMMA1001341//TRISTETRAPROLINE (TTP) (TIS11A) (TIS11) (ZFP-36) (GROWTH FACTOR- INDUCIBLE NUCLEAR PROTEIN NUP475).//0.024:89:39//HOMO SAPIENS (HUMAN).//P26651
F-MAMMA1001343//PROBABLE E5 PROTEIN.//0.60:64:29//HUMAN PAPILLOMAVIRUS TYPE 16.//P06927
F-MAMMA1001346//PROTEINASE INHIBITOR IIB (FRAGMENTS).//0.97:33:45//SOLANUM TUBEROSUM (POTATO).//P01082
F-MAMMA1001383//!!!! ALU SUBFAMILY SQ WARNING ENTRY !!!!//1.2e-30:86:77//HOMO SAPIENS (HUMAN).//P39194
F-MAMMA1001388//LEUCINE-RICH ALPHA-2-GLYCOPROTEIN (LRG).//9.2e-91:195:92//HOMO SAPIENS (HUMAN).//P02750
F-MAMMA1001397//!!!! ALU SUBFAMILY J WARNING ENTRY !!!!//3.5e-19:55:69//HOMO SAPIENS (HUMAN).//P39188
F-MAMMA1001408//SALIVARY GLUE PROTEIN SGS-7 PRECURSOR.//0.60:45:35//DROSOPHILA MELANOGASTER (FRUIT FLY).//P02841
F-MAMMA1001411//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//5.8e-06:153:26//SACCHAROMYCES CEREVISIAE (BAKER'S YEAST).//P08640
F-MAMMA1001419//!!!! ALU SUBFAMILY SQ WARNING ENTRY !!!!//2.3e-16:99:51//HOMO SAPIENS (HUMAN).//P39194
F-MAMMA1001420//!!!! ALU SUBFAMILY SB1 WARNING ENTRY !!!!//0.0018:23:65//HOMO SAPIENS (HUMAN).//P39190
F-MAMMA1001435//!!!! ALU SUBFAMILY SX WARNING ENTRY !!!!//1.7e-22:60:58//HOMO SAPIENS (HUMAN).//P39195
F-MAMMA1001442
F-MAMMA1001446//!!!! ALU SUBFAMILY SQ WARNING ENTRY !!!!//1.2e-23:48:75//HOMO SAPIENS (HUMAN).//P39194
F-MAMMA1001452//GENE 35 PROTEIN (GP35).//0.61:31:45//MYCOBACTERIOPHAGE L5.//Q05245
F-MAMMA1001465//HYPOTHETICAL PROTEIN E-115.//0.0026:68:38//HUMAN ADENOVIRUS TYPE 2.//P03290
F-MAMMA1001476//URIDINE KINASE (EC 2.7.1.48) (URIDINE MONOPHOSPHOKINASE) (FRAGMENT).//3.7e-94:201:92//MUS MUSCULUS (MOUSE).//P52623
F-MAMMA1001487//LINE-1 REVERSE TRANSCRIPTASE HOMOLOG.//4.6e-16:89:41//NYCTICEBUS COUCANG (SLOW LORIS).//P08548
F-MAMMA1001501//CALPAIN 1, LARGE [CATALYTIC] SUBUNIT (EC 3.4.22.17) (CALCIUM-ACTIVATED NEUTRAL PROTEINASE) (CANP) (MU-TYPE).//6.2e-59:86:97//HOMO SAPIENS (HUMAN).//P07384
F-MAMMA1001502//HYPOTHETICAL 11.4 KD PROTEIN (ORF1).//0.21:79:30//STREPTOMYCES FRADIAE.//P26800
F-MAMMA1001510
F-MAMMA1001522//HYPOTHETICAL 35.5 KD PROTEIN IN TRANSPOSON TN4556.//0.67:98:31//STREPTOMYCES FRADIAE.//P20186
F-MAMMA1001547//PROBABLE MOLYBDENUM-PTERIN BINDING PROTEIN.//0.97:35:42//HAEMOPHILUS INFLUENZAE.//P45183
F-MAMMA1001551//HYPOTHETICAL PROTEIN MJ0458.1.//0.038:31:41//METHANOCOCCUS JANNASCHII.//P81308
F-MAMMA1001575
F-MAMMA1001576//TUBULIN GAMMA CHAIN.//1.6e-86:162:99//XENOPUS LAEVIS (AFRICAN CLAWED FROG).//P23330
F-MAMMA1001590//!!!! ALU SUBFAMILY SX WARNING ENTRY !!!!//0.0035:38:55//HOMO SAPIENS (HUMAN).//P39195
F-MAMMA1001600//CONNECTIVE TISSUE GROWTH FACTOR PRECURSOR.//0.85:53:33//HOMO SAPIENS (HUMAN).//P29279
F-MAMMA1001604//HYPOTHETICAL 11.1 KD PROTEIN C30D11.02C IN CHROMOSOME I.//0.14:82:29//SCHIZOSACCHAROMYCES POMBE (FISSION YEAST).//Q09902
F-MAMMA1001606//HIGH MOBILITY GROUP PROTEIN HMGI-C.//8.2e-05:77:37//HOMO SAPIENS (HUMAN).//P52926
F-MAMMA1001620//!!!! ALU SUBFAMILY SX WARNING ENTRY !!!!//5.5e-05:24:66//HOMO SAPIENS (HUMAN).//P39195
F-MAMMA1001627//CUTICLE COLLAGEN 40.//0.82:131:31//CAENORHABDITIS ELEGANS.//P34804
F-MAMMA1001630//!!!! ALU SUBFAMILY SQ WARNING ENTRY !!!!//8.6e-26:57:78//HOMO SAPIENS (HUMAN).//P39194
F-MAMMA1001633//ZINC FINGER PROTEIN 165.//6.9e-38:160:55//HOMO SAPIENS (HUMAN).//P49910
F-MAMMA1001635
F-MAMMA1001649//SPERM PROTAMINE P1.//0.39:31:41//TACHYGLOSSUS ACULEATUS ACULEATUS (AUSTRALIAN ECHIDNA).//P35311
F-MAMMA1001654//NON-RECEPTOR TYROSINE KINASE SPORE LYSIS A (EC 2.7.1.112) (TYROSINE- PROTEIN KINASE 1).//5.6e-06:99:28//DICTYOSTELIUM DISCOIDEUM (SLIME MOLD).//P18160
F-MAMMA1001663//VERY HYPOTHETICAL XYLU PROTEIN.//0.99:27:37//ESCHERICHIA COLI.//P05056
F-MAMMA1001670//CUTICLE COLLAGEN 1.//0.033:97:37//CAENORHABDITIS ELEGANS.//P08124
F-MAMMA1001671
F-MAMMA1001679//PROCOLLAGEN ALPHA 2(IV) CHAIN PRECURSOR.//0.92:32:50//HOMO SAPIENS (HUMAN).//P08572
F-MAMMA1001683//HYPOTHETICAL 35.5 KD PROTEIN IN TRANSPOSON TN4556.//0.00026:147:34//STREPTOMYCES FRADIAE.//P20186
F-MAMMA1001686
F-MAMMA1001692//SMALL HYDROPHOBIC PROTEIN (SMALL PROTEIN 1A).//1.0:34:26//BOVINE RESPIRATORY SYNCYTIAL VIRUS (STRAIN A51908) (BRS).//P24616
F-MAMMA1001711//!!!! ALU SUBFAMILY SQ WARNING ENTRY !!!!//1.1e-28:56:69//HOMO SAPIENS (HUMAN).//P39194
F-MAMMA1001715//!!!! ALU SUBFAMILY J WARNING ENTRY !!!!//1.6e-08:39:71//HOMO SAPIENS (HUMAN).//P39188
F-MAMMA1001730//METALLOTHIONEIN-B (MTB).//1.0:17:64//STRONGYLOCENTROTUS PURPURATUS (PURPLE SEA URCHIN).//Q27287
F-MAMMA1001735//TUBULIN BETA-5 CHAIN (CLASS-V).//5.1e-121:213:97//GALLUS GALLUS (CHICKEN).//P09653
F-MAMMA1001740
F-MAMMA1001743//!!!! ALU SUBFAMILY SX WARNING ENTRY !!!!//1.3e-09:100:42//HOMO SAPIENS (HUMAN).//P39195
F-MAMMA1001744//POU DOMAIN PROTEIN 2.//0.97:59:38//BRACHYDANIO RERIO (ZEBRAFISH) (ZEBRA DANIO).//Q90270
F-MAMMA1001745//LINE-1 REVERSE TRANSCRIPTASE HOMOLOG.//2.1e-43:199:42//HOMO SAPIENS (HUMAN).//P08547
F-MAMMA1001751//TWK-8 PROTEIN.//2.9e-15:77:36//CAENORHABDITIS ELEGANS.//P34410
F-MAMMA1001754//MALE SPECIFIC SPERM PROTEIN MST84DD.//0.019:20:45//DROSOPHILA MELANOGASTER (FRUIT FLY).//Q01645
F-MAMMA1001757//HYPOTHETICAL 9.2 KD PROTEIN IN RNPA 3'REGION.//0.94:30:43//PSEUDOMONAS PUTIDA.//P25753
F-MAMMA1001760//!!!! ALU SUBFAMILY SB2 WARNING ENTRY !!!!//4.6e-34:103:59//HOMO SAPIENS (HUMAN).//P39191
F-MAMMA1001764
F-MAMMA1001768//HYPOTHETICAL PROTEIN UL61.//0.042:167:33//HUMAN CYTOMEGALOVIRUS (STRAIN AD169).//P16818
F-MAMMA1001769//!!!! ALU SUBFAMILY SQ WARNING ENTRY !!!!//2.0e-29:97:69//HOMO SAPIENS (HUMAN).//P39194
F-MAMMA1001771//TRANSMEMBRANE PROTEIN SEX PRECURSOR.//3.3e-09:123:32//HOMO SAPIENS (HUMAN).//P51805
F-MAMMA1001783//!!!! ALU SUBFAMILY J WARNING ENTRY !!!!//1.1e-09:55:61//HOMO SAPIENS (HUMAN).//P39188
F-MAMMA1001785//RAS-RELATED PROTEIN RABC.//1.9e-06:120:25//DICTYOSTELIUM DISCOIDEUM (SLIME MOLD).//P34143
F-MAMMA1001788//LINE-1 REVERSE TRANSCRIPTASE HOMOLOG.//9.3e-29:46:76//HOMO SAPIENS (HUMAN).//P08547
F-MAMMA1001790//!!!! ALU SUBFAMILY J WARNING ENTRY !!!!//4.7e-24:69:69//HOMO SAPIENS (HUMAN).//P39188
F-MAMMA1001806//HYPOTHETICAL 21.2 KD PROTEIN IN TOR2-MNN4 INTERGENIC REGION.//0.95:58:36//SACCHAROMYCES CEREVISIAE (BAKER'S YEAST).//P36042
F-MAMMA1001812//!!!! ALU SUBFAMILY SX WARNING ENTRY !!!!//8.8e-12:53:69//HOMO SAPIENS (HUMAN).//P39195
F-MAMMA1001815//LINE-1 REVERSE TRANSCRIPTASE HOMOLOG.//0.11:30:70//HOMO SAPIENS (HUMAN).//P08547
F-MAMMA1001817//!!!! ALU SUBFAMILY J WARNING ENTRY !!!!//2.9e-16:86:55//HOMO SAPIENS (HUMAN).//P39188
F-MAMMA1001818
F-MAMMA1001820//VITTELLINE MEMBRANE PROTEIN VM26AB PRECURSOR (PROTEIN TU-4) (PROTEIN SV23).//0.0030:63:42//DROSOPHILA MELANOGASTER (FRUIT FLY).//P13238
F-MAMMA1001824//APTOTOXIN VII (PARALYTIC PEPTIDE VII) (PP VII).//0.99:26:34//APTOSTICHUS SCHLINGERI (TRAP-DOOR SPIDER).//P49271
F-MAMMA1001836//!!!! ALU SUBFAMILY SX WARNING ENTRY !!!!//2.6e-35:77:88//HOMO SAPIENS (HUMAN).//P39195
F-MAMMA1001837//ZINC FINGER PROTEIN 191.//1.3e-27:106:58//HOMO SAPIENS (HUMAN).//O14754
F-MAMMA1001848//!!!! ALU SUBFAMILY J WARNING ENTRY !!!!//1.0e-19:92:58//HOMO SAPIENS (HUMAN).//P39188
F-MAMMA1001851
F-MAMMA1001854
F-MAMMA1001858//ISOTOCIN-NEUROPHYSIN IT 1 PRECURSOR.//0.93:42:38//CATOSTOMUS COMMERSONI (WHITE SUCKER).//P15210
F-MAMMA1001864//PROBABLE ABC TRANSPORTER PERMEASE PROTEIN MG189.//0.77:161:27//MYCOPLASMA GENITALIUM.//P47435
F-MAMMA1001868//FK506-BINDING NUCLEAR PROTEIN (PEPTIDYL-PROLYL CIS-TRANS ISOMERASE) (PPIASE) (EC 5.2.1.8) (PROLINE ROTAMASE) (NUCLEOLAR PROLINE ISOMERASE) (FKBP-70).//0.00013:219:26//SACCHAROMYCES CEREVISIAE (BAKER'S YEAST).//P38911
F-MAMMA1001874//SPERM HISTONE P2 PRECURSOR (PROTAMINE MP2).//0.0075:76:31//MUS MUSCULUS (MOUSE).//P07978
F-MAMMA1001878//GLYCINE-RICH CELL WALL STRUCTURAL PROTEIN (CLONE W10-1) (FRAGMENT).//0.020:10:80//LYCOPERSICON ESCULENTUM (TOMATO).//Q01157
F-MAMMA1001880
F-MAMMA1001890//!!!! ALU SUBFAMILY SC WARNING ENTRY !!!!//5.1e-34:56:83//HOMO SAPIENS (HUMAN).//P39192
F-MAMMA1001907//!!!! ALU SUBFAMILY SQ WARNING ENTRY !!!!//2.7e-12:44:68//HOMO SAPIENS (HUMAN).//P39194
F-MAMMA1001908//HYPOTHETICAL 16.2 KD PROTEIN IN PRP24-RRN9 INTERGENIC REGION.//0.00013:77:37//SACCHAROMYCES CEREVISIAE (BAKER'S YEAST).//Q03525
F-MAMMA1001931//HYPOTHETICAL 118.2 KD PROTEIN F43C1.1 IN CHROMOSOME III.//0.41:106:29//CAENORHABDITIS ELEGANS.//Q09564
F-MAMMA1001956//OCTAPEPTIDE-REPEAT PROTEIN T2.//0.00053:149:30//MUS MUSCULUS (MOUSE).//Q06666
F-MAMMA1001963//HYPOTHETICAL PROTEIN IN NAC 5'REGION (ORF X) (FRAGMENT).//1.0:46:28//KLEBSIELLA AEROGENES.//Q08600
F-MAMMA1001969//LINE-1 REVERSE TRANSCRIPTASE HOMOLOG.//1.7e-34:97:68//HOMO SAPIENS (HUMAN).//P08547
F-MAMMA1001970//LINE-1 REVERSE TRANSCRIPTASE HOMOLOG.//6.2e-07:67:37//HOMO SAPIENS (HUMAN).//P08547
F-MAMMA1001992//PROTEIN Q300.//0.53:14:71//MUS MUSCULUS (MOUSE).//Q02722
F-MAMMA1002009//PROBABLE E5 PROTEIN.//0.17:56:32//HUMAN PAPILLOMAVIRUS TYPE 31.//P17385
F-MAMMA1002011//MYRISTOYLATED ALANINE-RICH C-KINASE SUBSTRATE (MARCKS) (PROTEIN KINASE C SUBSTRATE, 80 KD PROTEIN, LIGHT CHAIN) (PKCSL) (80K-L PROTEIN).//1.0:100:31//HOMO SAPIENS (HUMAN).//P29966
F-MAMMA1002032//!!!! ALU SUBFAMILY J WARNING ENTRY !!!!//3.1e-21:86:65//HOMO SAPIENS (HUMAN).//P39188
F-MAMMA1002033//!!!! ALU SUBFAMILY J WARNING ENTRY !!!!//8.5e-20:67:58//HOMO SAPIENS (HUMAN).//P39188
F-MAMMA1002041//MALE SPECIFIC SPERM PROTEIN MST84DC.//1.0:17:52//DROSOPHILA MELANOGASTER (FRUIT FLY).//Q01644
F-MAMMA1002042//!!!! ALU SUBFAMILY SC WARNING ENTRY !!!!//0.19:45:46//HOMO SAPIENS (HUMAN).//P39192
F-MAMMA1002047//TYROSINE AMINOTRANSFERASE (EC 2.6.1.5) (L-TYROSINE:2-OXOGLUTARATE AMINOTRANSFERASE) (TAT).//0.0017:50:46//RATTUS NORVEGICUS (RAT).//P04694
F-MAMMA1002056//!!!! ALU SUBFAMILY SQ WARNING ENTRY !!!!//3.2e-37:70:77//HOMO SAPIENS (HUMAN).//P39194
F-MAMMA1002058//!!!! ALU SUBFAMILY J WARNING ENTRY !!!!//1.1e-08:26:76//HOMO SAPIENS (HUMAN).//P39188
F-MAMMA1002068//LINE-1 REVERSE TRANSCRIPTASE HOMOLOG.//2.0e-11:78:46//HOMO SAPIENS (HUMAN).//P08547
F-MAMMA1002078//ATP SYNTHASE PROTEIN 8 (EC 3.6.1.34) (A6L).//0.96:26:46//COTURNIX COTURNIX JAPONICA (JAPANESE QUAIL).//P50682
F-MAMMA1002082//SUPPRESSOR PROTEIN SRP40.//0.23:95:32//SACCHAROMYCES CEREVISIAE (BAKER'S YEAST).//P32583
F-MAMMA1002084//HYPOTHETICAL 7.5 KD PROTEIN.//1.0:40:35//VACCINIA VIRUS (STRAIN COPENHAGEN).//P20520
F-MAMMA1002093
F-MAMMA1002108//HYPOTHETICAL 35.5 KD PROTEIN IN TRANSPOSON TN4556.//0.00079:143:33//STREPTOMYCES FRADIAE.//P20186
F-MAMMA1002118//ATP SYNTHASE PROTEIN 8 (EC 3.6.1.34) (A6L).//1.0:43:34//METRIDIUM SENILE (BROWN SEA ANEMONE) (FRILLED SEA ANEMONE).//O47493
F-MAMMA1002125//!!!! ALU SUBFAMILY SC WARNING ENTRY !!!!//1.9e-14:60:68//HOMO SAPIENS (HUMAN).//P39192
F-MAMMA1002132
F-MAMMA1002140//!!!! ALU SUBFAMILY J WARNING ENTRY !!!!//1.4e-24:69:65//HOMO SAPIENS (HUMAN).//P39188
F-MAMMA1002143//SERUM PROTEIN MSE55.//2.1e-16:166:43//HOMO SAPIENS (HUMAN).//Q00587
F-MAMMA1002145//36.4 KD PROLINE-RICH PROTEIN.//0.00014:84:29//LYCOPERSICON ESCULENTUM (TOMATO).//Q00451
F-MAMMA1002153
F-MAMMA1002155
F-MAMMA1002156//METALLOPROTEINASE INHIBITOR PRECURSOR.//0.90:58:34//STREPTOMYCES NIGRESCENS.//P01077
F-MAMMA1002158
F-MAMMA1002170//40S RIBOSOMAL PROTEIN S2 (S4) (LLREP3 PROTEIN).//6.0e-66:157:70//HOMO SAPIENS (HUMAN).//P15880
F-MAMMA1002174//!!!! ALU SUBFAMILY J WARNING ENTRY !!!!//6.5e-25:56:64//HOMO SAPIENS (HUMAN).//P39188
F-MAMMA1002198//THIOREDOXIN PEROXIDASE 1 (THIOREDOXIN-DEPENDENT PEROXIDE REDUCTASE 1) (THIOL-SPECIFIC ANTIOXIDANT PROTEIN) (TSA) (PRP) (NATURAL KILLER CELL ENHANCING FACTOR B) (NKEF-B).//9.0e-09:28:100//HOMO SAPIENS (HUMAN).//P32119
F-MAMMA1002209//TRANSCRIPTION INITIATION FACTOR TFIID 135 KD SUBUNIT (TAFII-135) (TAFII135) (TAFII-130) (TAFII130).//0.0023:132:33//HOMO SAPIENS (HUMAN).//O00268
F-MAMMA1002215//PROCOLLAGEN ALPHA 1(I) CHAIN PRECURSOR.//0.00032:68:35//HOMO SAPIENS (HUMAN).//P02452
F-MAMMA1002219//INTRACELLULAR PROTEIN TRANSPORT PROTEIN USO1.//0.0079:224:24//SACCHAROMYCES CEREVISIAE (BAKER'S YEAST).//P25386
F-MAMMA1002230
F-MAMMA1002236//TRANSLATION INITIATION FACTOR EIF-2B GAMMA SUBUNIT (EIF-2B GDP-GTP EXCHANGE FACTOR).//1.4e-118:151:94//RATTUS NORVEGICUS (RAT).//P70541
F-MAMMA1002243//WISKOTT-ALDRICH SYNDROME PROTEIN HOMOLOG (WASP).//0.028:112:33//MUS MUSCULUS (MOUSE).//P70315
F-MAMMA1002250//T-CELL RECEPTOR BETA CHAIN PRECURSOR (ANA 11).//0.0012:80:32//ORYCTOLAGUS CUNICULUS (RABBIT).//P06333
F-MAMMA1002267//ATP SYNTHASE A CHAIN (EC 3.6.1.34) (PROTEIN 6).//0.17:139:28//TRYPANOSOMA BRUCEI BRUCEI.//P24499
F-MAMMA1002268//60S RIBOSOMAL PROTEIN L22.//0.00026:163:30//DROSOPHILA MELANOGASTER (FRUIT FLY).//P50887
F-MAMMA1002269//HISTIDINE-RICH, METAL BINDING POLYPEPTIDE.//0.35:14:57//HELICOBACTER PYLORI (CAMPYLOBACTER PYLORI).//Q48251
F-MAMMA1002282//!!!! ALU SUBFAMILY SC WARNING ENTRY !!!!//6.1e-05:32:65//HOMO SAPIENS (HUMAN).//P39192
F-MAMMA1002292//TROPOMYOSIN 2.//1.4e-05:100:30//SACCHAROMYCES CEREVISIAE (BAKER'S YEAST).//P40414
F-MAMMA1002293//!!!! ALU SUBFAMILY J WARNING ENTRY !!!!//6.8e-25:127:44//HOMO SAPIENS (HUMAN).//P39188
F-MAMMA1002294//ALPHA TRANS-INDUCING PROTEIN (ALPHA-TIF).//0.00011:138:38//BOVINE HERPESVIRUS TYPE 1 (STRAIN P8-2).//P30020
F-MAMMA1002297//A-AGGLUTININ ATTACHMENT SUBUNIT PRECURSOR.//0.15:144:30//SACCHAROMYCES CEREVISIAE (BAKER'S YEAST).//P32323
F-MAMMA1002298//PROLINE-RICH PROTEIN MP-3 (FRAGMENT).//1.0e-05:40:50//MUS MUSCULUS (MOUSE).//P05143
F-MAMMA1002299//NADH-UBIQUINONE OXIDOREDUCTASE CHAIN 4L (EC 1.6.5.3).//0.84:65:32//STRUTHIO CAMELUS (OSTRICH).//O21405
F-MAMMA1002308//!!!! ALU SUBFAMILY J WARNING ENTRY !!!!//4.3e-29:61:73//HOMO SAPIENS (HUMAN).//P39188
F-MAMMA1002310//SPERM MITOCHONDRIAL CAPSULE SELENOPROTEIN (MCS).//0.00016:70:38//MUS MUSCULUS (MOUSE).//P15265
F-MAMMA1002311//!!!! ALU SUBFAMILY SB WARNING ENTRY !!!!//9.4e-09:84:54//HOMO SAPIENS (HUMAN).//P39189
F-MAMMA1002312//HYPOTHETICAL 10.8 KD PROTEIN IN GP30-RIII INTERGENIC REGION (URF Y).//0.48:48:33//BACTERIOPHAGE T4.//P33084
F-MAMMA1002317
F-MAMMA1002319//RETROVIRUS-RELATED POL POLYPROTEIN [CONTAINS: REVERSE TRANSCRIPTASE (EC 2.7.7.49); ENDONUCLEASE].//0.011:128:27//MUS MUSCULUS (MOUSE).//P11369
F-MAMMA1002322//!!!! ALU SUBFAMILY SX WARNING ENTRY !!!!//5.2e-20:92:57//HOMO SAPIENS (HUMAN).//P39195
F-MAMMA1002329//ATP SYNTHASE PROTEIN 8 (EC 3.6.1.34) (A6L).//0.051:33:36//XENOPUS LAEVIS (AFRICAN CLAWED FROG).//P03931
F-MAMMA1002332//LINE-1 REVERSE TRANSCRIPTASE HOMOLOG.//6.5e-20:116:51//HOMO SAPIENS (HUMAN).//P08547
F-MAMMA1002333//COLLAGEN ALPHA 1(I) CHAIN (FRAGMENTS).//0.0017:214:31//BOS TAURUS (BOVINE).//P02453
F-MAMMA1002339//COPPER-METALLOTHIONEIN (CU-MT).//0.59:42:38//HELIX POMATIA (ROMAN SNAIL) (EDIBLE SNAIL).//P55947
F-MAMMA1002347//!!!! ALU SUBFAMILY J WARNING ENTRY !!!!//0.43:26:61//HOMO SAPIENS (HUMAN).//P39188
F-MAMMA1002351//HYPOTHETICAL PROTEIN MJ0304.//2.3e-07:139:25//METHANOCOCCUS JANNASCHII.//Q57752
F-MAMMA1002352
F-MAMMA1002353//!!!! ALU SUBFAMILY J WARNING ENTRY !!!!//0.00028:31:80//HOMO SAPIENS (HUMAN).//P39188
F-MAMMA1002355//!!!! ALU SUBFAMILY SP WARNING ENTRY !!!!//4.2e-28:87:73//HOMO SAPIENS (HUMAN).//P39193
F-MAMMA1002356//RELAXIN.//0.95:31:35//SQUALUS ACANTHIAS (SPINY DOGFISH).//P11953
F-MAMMA1002359//CHLOROPLAST 50S RIBOSOMAL PROTEIN L33.//0.93:44:36//GUILLARDIA THETA (CRYPTOMONAS PHI).//O78487
F-MAMMA1002360//LATE L2 MU CORE PROTEIN PRECURSOR (PROTEIN X).//0.94:30:43//BOVINE ADENOVIRUS TYPE 2 (MASTADENOVIRUS BOS2).//Q96626
F-MAMMA1002361//!!!! ALU SUBFAMILY J WARNING ENTRY !!!!//2.0e-08:45:68//HOMO SAPIENS (HUMAN).//P39188
F-MAMMA1002362//ATP SYNTHASE PROTEIN 8 (EC 3.6.1.34) (A6L).//0.58:23:26//LUMBRICUS TERRESTRIS (COMMON EARTHWORM).//Q34942
F-MAMMA1002380//SALIVARY GLUE PROTEIN SGS-3 PRECURSOR.//0.23:100:27//DROSOPHILA SIMULANS (FRUIT FLY).//P13729
F-MAMMA1002384
F-MAMMA1002385//HYPOTHETICAL 40.9 KD PROTEIN IN ORC2-TIP1 INTERGENIC REGION.//3.8e-14:125:37//SACCHAROMYCES CEREVISIAE (BAKER'S YEAST).//P38241
F-MAMMA1002392//ATP SYNTHASE PROTEIN 8 (EC 3.6.1.34) (A6L).//1.0:17:58//BRANCHIOSTOMA LANCEOLATUM (COMMON LANCELET) (AMPHIOXUS).//O21003
F-MAMMA1002411//30S RIBOSOMAL PROTEIN S17.//0.85:49:32//SYNECHOCYSTIS SP. (STRAIN PCC 6803).//P73311
F-MAMMA1002413//NADH-UBIQUINONE OXIDOREDUCTASE CHAIN 1 (EC 1.6.5.3) (FRAGMENT).//0.97:41:39//DROSOPHILA AFFINIS (FRUIT FLY).//P51926
F-MAMMA1002417//RFBJ PROTEIN.//0.99:31:35//SHIGELLA FLEXNERI.//P37786
F-MAMMA1002427//!!!! ALU SUBFAMILY SQ WARNING ENTRY !!!!//1.6e-33:135:59//HOMO SAPIENS (HUMAN).//P39194
F-MAMMA1002428//HYPOTHETICAL PROTEIN C18.//0.97:34:44//SWINEPOX VIRUS (STRAIN KASZA) (SPV).//P32217
F-MAMMA1002434//!!!! ALU SUBFAMILY SB WARNING ENTRY !!!!//3.1e-36:56:78//HOMO SAPIENS (HUMAN).//P39189
F-MAMMA1002446
F-MAMMA1002454//EARLY NODULIN 20 PRECURSOR (N-20).//0.77:57:45//MEDICAGO TRUNCATULA (BARREL MEDIC).//P93329
F-MAMMA1002461//VASODILATOR-STIMULATED PHOSPHOPROTEIN (VASP).//1.3e-05:193:32//CANIS FAMILIARIS (DOG).//P50551
F-MAMMA1002470//HYPOTHETICAL 80.7 KD PROTEIN IN ERG7-NMD2 INTERGENIC REGION.//1.0e-75:231:60//SACCHAROMYCES CEREVISIAE (BAKER'S YEAST).//P38795
F-MAMMA1002475//POSSIBLE GLOBAL TRANSCRIPTION ACTIVATOR SNF2L4 (SNF2-BETA) (BRG-1 PROTEIN) (MITOTIC GROWTH AND TRANSCRIPTION ACTIVATOR) (BRAHMA PROTEIN HOMOLOG 1).//0.013:99:30//HOMO SAPIENS (HUMAN).//P51532
F-MAMMA1002480//NONSTRUCTURAL PROTEIN 5B.//1.0:23:43//HUMAN CORONAVIRUS (STRAIN 229E).//P19741
F-MAMMA1002485//STANNIOCALCIN PRECURSOR.//2.1e-23:88:46//HOMO SAPIENS (HUMAN).//P52823
F-MAMMA1002494//MOLT-INHIBITING HORMONE (MIH).//1.0:32:37//PROCAMBARUS CLARKII (RED SWAMP CRAYFISH).//P55848
F-MAMMA1002498//6.7 KD PROTEIN (ORF 5).//1.0:26:42//BARLEY YELLOW DWARF VIRUS (ISOLATE PAV) (BYDV).//P09517
F-MAMMA1002524//HYPOTHETICAL 117.8 KD PROTEIN IN STE2-FRS2 INTERGENIC REGION.//5.0e-26:222:35//SACCHAROMYCES CEREVISIAE (BAKER'S YEAST).//P43571
F-MAMMA1002530//CYTOSOLIC PHOSPHOLIPASE A2 (EC 3.1.1.4) (CPLA2) (PHOSPHATIDYLCHOLINE 2-ACYLHYDROLASE) / LYSOPHOSPHOLIPASE (EC 3.1.1.5).//4.5e-12:88:44//HOMO SAPIENS (HUMAN).//P47712
F-MAMMA1002545//!!!! ALU SUBFAMILY SX WARNING ENTRY !!!!//4.3e-29:97:71//HOMO SAPIENS (HUMAN).//P39195
F-MAMMA1002554//DNA-DIRECTED RNA POLYMERASE II LARGEST SUBUNIT (EC 2.7.7.6) (RPB1) (FRAGMENT).//0.46:54:40//CRICETULUS GRISEUS (CHINESE HAMSTER).//P11414
F-MAMMA1002556//METALLOTHIONEIN 20-I ISOFORMS A AND B (MT-20-IA AND MT-20-IB).//0.99:21:47//MYTILUS EDULIS (BLUE MUSSEL).//P80251
F-MAMMA1002566//TRANSCRIPTION FACTOR P65 (NUCLEAR FACTOR NF-KAPPA-B P65 SUBUNIT).//0.70:130:30//MUS MUSCULUS (MOUSE).//Q04207
F-MAMMA1002571//DNA-DIRECTED RNA POLYMERASE II LARGEST SUBUNIT (EC 2.7.7.6) (FRAGMENT).//0.54:45:51//DICTYOSTELIUM DISCOIDEUM (SLIME MOLD).//P35084
F-MAMMA1002573//PARATHYMOSIN.//1.5e-07:69:46//HOMO SAPIENS (HUMAN).//P20962
F-MAMMA1002585//MYOSIN LIGHT CHAIN 1, SLOW-TWITCH MUSCLE B/VENTRICULAR ISOFORM (FRAGMENT).//0.38:36:36//MUS MUSCULUS (MOUSE).//P09542
F-MAMMA1002590//!!!! ALU SUBFAMILY SX WARNING ENTRY !!!!//0.99:22:77//HOMO SAPIENS (HUMAN).//P39195
F-MAMMA1002597//!!!! ALU SUBFAMILY SQ WARNING ENTRY !!!!//2.1e-18:44:70//HOMO SAPIENS (HUMAN).//P39194
F-MAMMA1002598//60S RIBOSOMAL PROTEIN L7.//1.8e-16:40:100//HOMO SAPIENS (HUMAN).//P18124
F-MAMMA1002603
F-MAMMA1002612//30S RIBOSOMAL PROTEIN S16 (FRAGMENT).//1.0:29:37//THERMUS AQUATICUS.//O07348
F-MAMMA1002617//ACIDIC PROLINE-RICH PROTEIN PRECURSOR (CLONE PRP25) (FRAGMENT).//0.00041:81:34//RATTUS NORVEGICUS (RAT).//P10164
F-MAMMA1002618//ESCARGOT/SNAIL PROTEIN HOMOLOG (FRAGMENT).//0.11:18:50//PSYCHODA CINEREA.//Q02027
F-MAMMA1002619//PROBABLE UBIQUITIN CARBOXYL-TERMINAL HYDROLASE K02C4.3 (EC 3.1.2.15) (UBIQUITIN THIOLESTERASE) (UBIQUITIN-SPECIFIC PROCESSING PROTEASE) (DEUBIQUITINATING ENZYME).//1.8e-13:110:40//CAENORHABDITIS ELEGANS.//Q09931
F-MAMMA1002622//!!!! ALU SUBFAMILY J WARNING ENTRY !!!!//8.4e-05:53:58//HOMO SAPIENS (HUMAN).//P39188
F-MAMMA1002623//PEPTIDYL-GLYCINE ALPHA-AMIDATING MONOOXYGENASE PRECURSOR (EC 1.14.17.3) (PAM).//2.6e-07:37:78//HOMO SAPIENS (HUMAN).//P19021
F-MAMMA1002625
F-MAMMA1002629//!!!! ALU SUBFAMILY J WARNING ENTRY !!!!//4.4e-19:49:73//HOMO SAPIENS (HUMAN).//P39188
F-MAMMA1002636//COLLAGEN ALPHA 2(VI) CHAIN (FRAGMENT).//1.7e-07:189:32//HOMO SAPIENS (HUMAN).//P12110
F-MAMMA1002637//KINESIN LIGHT CHAIN (KLC).//7.7e-54:227:52//RATTUS NORVEGICUS (RAT).//P37285
F-MAMMA1002646//NEUROFILAMENT TRIPLET H PROTEIN (200 KD NEUROFILAMENT PROTEIN) (NF-H).//0.034:199:25//MUS MUSCULUS (MOUSE).//P19246
F-MAMMA1002650//TRANSCRIPTION REGULATOR PROTEIN BACH2 (BTB AND CNC HOMOLOG 2).//1.7e-07:104:32//MUS MUSCULUS (MOUSE).//P97303
F-MAMMA1002655//SMALL PROLINE-RICH PROTEIN II (SPR-II) (CLONE 174N).//1.0:25:44//HOMO SAPIENS (HUMAN).//P22532
F-MAMMA1002662
F-MAMMA1002665//!!!! ALU SUBFAMILY SQ WARNING ENTRY !!!!//2.3e-07:54:57//HOMO SAPIENS (HUMAN).//P39194
F-MAMMA1002671//ACETYL-COENZYME A SYNTHETASE (EC 6.2.1.1) (ACETATE--COA LIGASE) (ACYL- ACTIVATING ENZYME).//1.4e-10:144:31//ESCHERICHIA COLI.//P27550
F-MAMMA1002673//BREVICAN CORE PROTEIN PRECURSOR.//0.76:64:39//BOS TAURUS (BOVINE).//Q28062
F-MAMMA1002684//HYPOTHETICAL 11.8 KD PROTEIN IN GP55-NRDG INTERGENIC REGION.//0.094:77:27/BACTERIOPHAGE T4.//P07079
F-MAMMA1002685//COLLAGEN ALPHA 1(I) CHAIN (FRAGMENTS).//0.0017:177:34//RATTUS NORVEGICUS (RAT).//P02454
F-MAMMA1002698
F-MAMMA1002699//HYPOTHETICAL 45.1 KD PROTEIN IN RPS5-ZMS1 INTERGENIC REGION.//1.2e-28:127:47//SACCHAROMYCES CEREVISIAE (BAKER'S YEAST).//P47160
F-MAMMA1002701//!!!! ALU SUBFAMILY J WARNING ENTRY !!!!//1.0:14:92//HOMO SAPIENS (HUMAN).//P39188
F-MAMMA1002708//!!!! ALU SUBFAMILY SP WARNING ENTRY !!!!//7.9e-27:52:65//HOMO SAPIENS (HUMAN).//P39193
F-MAMMA1002711//!!!! ALU SUBFAMILY J WARNING ENTRY !!!!//3.7e-24:54:75//HOMO SAPIENS (HUMAN).//P39188
F-MAMMA1002721
F-MAMMA1002727//SOX-13 PROTEIN (FRAGMENT).//0.70:36:38//MUS MUSCULUS (MOUSE).//Q04891
F-MAMMA1002728//HYPOTHETICAL 6.0 KD PROTEIN.//1.0:25:44//THERMOPROTEUS TENAX VIRUS 1 (STRAIN KRA1) (TTV1).//P19305
F-MAMMA1002744//HYPOTHETICAL 13.4 KD PROTEIN IN ACT5-YCK1 INTERGENIC REGION.//1.0:52:34//SACCHAROMYCES CEREVISIAE (BAKER'S YEAST).//P38834
F-MAMMA1002746//HYPOTHETICAL 5.6 KD PROTEIN (ORF A-45).//1.0:22:40//SULFOLOBUS VIRUS-LIKE PARTICLE SSV1.//P20198
F-MAMMA1002748
F-MAMMA1002754//!!!! ALU SUBFAMILY J WARNING ENTRY !!!!//5.1e-21:56:64//HOMO SAPIENS (HUMAN).//P39188
F-MAMMA1002758//MALE SPECIFIC SPERM PROTEIN MST84DD.//0.37:14:64//DROSOPHILA MELANOGASTER (FRUIT FLY).//Q01645
F-MAMMA1002764//!!!! ALU SUBFAMILY SQ WARNING ENTRY !!!!//4.7e-32:79:60//HOMO SAPIENS (HUMAN).//P39194
F-MAMMA1002765//PARATHYMOSIN.//0.79:63:28//BOS TAURUS (BOVINE).//P08814
F-MAMMA1002769//GAR2 PROTEIN.//0.00037:192:27//SCHIZOSACCHAROMYCES POMBE (FISSION YEAST).//P41891
F-MAMMA1002775//HYPOTHETICAL 36.7 KD PROTEIN C2F7.14C IN CHROMOSOME I.//5.4e-54:240:49//SCHIZOSACCHAROMYCES POMBE (FISSION YEAST).//Q09704
F-MAMMA1002780
F-MAMMA1002782//MARGATOXIN (MGTX).//1.0:31:38//CENTRUROIDES MARGARITATUS (SCORPION).//P40755
F-MAMMA1002796//ICE NUCLEATION PROTEIN.//0.0018:100:41//PSEUDOMONAS FLUORESCENS.//P09815
F-MAMMA1002807//!!!! ALU SUBFAMILY J WARNING ENTRY !!!!//9.3e-23:100:59//HOMO SAPIENS (HUMAN).//P39188
F-MAMMA1002820//NEUROTOXIN IV (LQQ IV).//1.0:18:50//LEIURUS QUINQUESTRIATUS QUINQUESTRIATUS (EGYPTIAN SCORPION).//P01489
F-MAMMA1002830//!!!! ALU SUBFAMILY SX WARNING ENTRY !!!!//4.7e-24:55:74//HOMO SAPIENS (HUMAN).//P39195
F-MAMMA1002833//!!!! ALU SUBFAMILY SB WARNING ENTRY !!!!//2.6e-31:95:73//HOMO SAPIENS (HUMAN).//P39189
F-MAMMA1002835//HYPOTHETICAL 42.1 KD PROTEIN F13G3.3 IN CHROMOSOME I.//1.0:54:37//CAENORHABDITIS ELEGANS.//Q19417
F-MAMMA1002838//!!!! ALU SUBFAMILY SP WARNING ENTRY !!!!//2.5e-27:99:70//HOMO SAPIENS (HUMAN).//P39193
F-MAMMA1002842//!!!! ALU SUBFAMILY SX WARNING ENTRY !!!!//2.3e-13:65:63//HOMO SAPIENS (HUMAN).//P39195
F-MAMMA1002843//METALLOTHIONEIN-II (MT-II).//0.97:19:47//MUS MUSCULUS (MOUSE).//P02798
F-MAMMA1002844//HYPOTHETICAL 24.1 KD PROTEIN IN LEF4-P33 INTERGENIC REGION.//4.9e-08:119:36//AUTOGRAPHA CALIFORNICA NUCLEAR POLYHEDROSIS VIRUS (ACMNPV).//P41479
F-MAMMA1002858//ATP SYNTHASE PROTEIN 8 (EC 3.6.1.34) (A6L).//0.98:37:37//PAN TROGLODYTES (CHIMPANZEE).//Q35647
F-MAMMA1002868//!!!! ALU SUBFAMILY J WARNING ENTRY !!!!//3.8e-10:51:62//HOMO SAPIENS (HUMAN).//P39188
F-MAMMA1002869//PINCH PROTEIN (PARTICULARY INTERESTING NEW CYS-HIS PROTEIN).//1.8e-95:194:78//HOMO SAPIENS (HUMAN).//P48059
F-MAMMA1002871//G-PROTEIN COUPLED RECEPTOR HOMOLOG R33.//1.0:51:35//RAT CYTOMEGALOVIRUS (STRAIN MAASTRICHT).//O12000
F-MAMMA1002880
F-MAMMA1002881//GLIOMA PATHOGENESIS-RELATED PROTEIN (RTVP-1 PROTEIN).//3.3e-22:180:35//HOMO SAPIENS (HUMAN).//P48060
F-MAMMA1002886//MYOSIN HEAVY CHAIN IB (MYOSIN HEAVY CHAIN IL).//0.00011:148:39//ACANTHAMOEBA CASTELLANII (AMOEBA).//P19706
F-MAMMA1002887
F-MAMMA1002890//A-AGGLUTININ ATTACHMENT SUBUNIT PRECURSOR.//0.030:142:25//SACCHAROMYCES CEREVISIAE (BAKER'S YEAST).//P32323
F-MAMMA1002892
F-MAMMA1002895//HYPOTHETICAL PROTEIN UL61.//0.00099:143:35//HUMAN CYTOMEGALOVIRUS (STRAIN AD169).//P16818
F-MAMMA1002908//T-CELL RECEPTOR BETA CHAIN PRECURSOR (ANA 11).//0.12:44:43//ORYCTOLAGUS CUNICULUS (RABBIT).//P06333
F-MAMMA1002909//!!!! ALU SUBFAMILY J WARNING ENTRY !!!!//0.00011:28:75//HOMO SAPIENS (HUMAN).//P39188
F-MAMMA1002930//BOMBYXIN A-7 PRECURSOR (BBX-A7) (4K-PROTHORACICOTROPIC HORMONE) (4K-PTTH).//0.99:45:46//BOMBYX MORI (SILK MOTH).//P26730
F-MAMMA1002937//ZINC FINGER PROTEIN 42 (MYELOID ZINC FINGER 1) (MZF-1).//6.5e-24:147:34//HOMO SAPIENS (HUMAN).//P28698
F-MAMMA1002938//CERULOPLASMIN PRECURSOR (EC 1.16.3.1) (FERROXIDASE).//4.7e-11:44:68//MUS MUSCULUS (MOUSE).//Q61147
F-MAMMA1002941//PROTEIN Q300.//0.0076:21:61//MUS MUSCULUS (MOUSE).//Q02722
F-MAMMA1002947//HYPOTHETICAL 35.5 KD PROTEIN IN TRANSPOSON TN4556.//1.9e-08:152:38//STREPTOMYCES FRADIAE.//P20186
F-MAMMA1002964
F-MAMMA1002970//!!!! ALU SUBFAMILY SB WARNING ENTRY !!!!//0.0057:55:43//HOMO SAPIENS (HUMAN).//P39189
F-MAMMA1002972//BRAIN-SPECIFIC HOMEOBOX/POU DOMAIN PROTEIN 3A (BRN-3A) (OCT-T1) (HOMEOBOX/POU DOMAIN PROTEIN RDC-1).//0.84:53:41//HOMO SAPIENS (HUMAN).//Q01851
F-MAMMA1002973//!!!! ALU SUBFAMILY SC WARNING ENTRY !!!!//4.6e-11:54:68//HOMO SAPIENS (HUMAN).//P39192
F-MAMMA1002982
F-MAMMA1002987//HYPOTHETICAL 11.9 KD PROTEIN IN RPC8-MFA2 INTERGENIC REGION.//0.17:47:29//SACCHAROMYCES CEREVISIAE (BAKER'S YEAST).//P53906
F-MAMMA1003003//!!!! ALU SUBFAMILY SX WARNING ENTRY !!!!//8.6e-09:30:73//HOMO SAPIENS (HUMAN).//P39195
F-MAMMA1003004//!!!! ALU SUBFAMILY SX WARNING ENTRY !!!!//0.0071:41:58//HOMO SAPIENS (HUMAN).//P39195
F-MAMMA1003007//SPERM PROTAMINE P1.//0.0076:51:37//TACHYGLOSSUS ACULEATUS ACULEATUS (AUSTRALIAN ECHIDNA).//P35311
F-MAMMA1003011//HISTONE MACRO-H2A.1.//1.8e-60:175:70//RATTUS NORVEGICUS (RAT).//Q02874
F-MAMMA1003013//ACTIN BINDING PROTEIN.//0.097:83:31//SACCHAROMYCES EXIGUUS (YEAST).//P38479
F-MAMMA1003015
F-MAMMA1003019//MYOTUBULARIN.//0.022:56:37//HOMO SAPIENS (HUMAN).//Q13496
F-MAMMA1003026//HYPOTHETICAL 29.3 KD PROTEIN (ORF92).//0.0014:208:27//ORGYIA PSEUDOTSUGATA MULTICAPSID POLYHEDROSIS VIRUS (OPMNPV).//O10341
F-MAMMA1003031//PROBABLE E4 PROTEIN (E1^E4).//0.14:49:32//HUMAN PAPILLOMAVIRUS TYPE 6B.//P06459
F-MAMMA1003035//HYPOTHETICAL 24.4 KD PROTEIN IN LPD 3'REGION (ORF4).//5.1e-12:112:34//ZYMOMONAS MOBILIS.//O66114
F-MAMMA1003039//!!!! ALU SUBFAMILY J WARNING ENTRY !!!!//2.4e-07:68:54//HOMO SAPIENS (HUMAN).//P39188
F-MAMMA1003040//!!!! ALU SUBFAMILY SB1 WARNING ENTRY !!!!//2.8e-39:90:57//HOMO SAPIENS (HUMAN).//P39190
F-MAMMA1003044
F-MAMMA1003047//SPERM HISTONE P2 PRECURSOR (PROTAMINE 2).//0.18:25:44//BOS TAURUS (BOVINE).//P19782
F-MAMMA1003049//PROBABLE E4 PROTEIN.//0.50:67:29//HUMAN PAPILLOMAVIRUS TYPE 6C.//P20969
F-MAMMA1003055//WEAK TOXIN CM-2.//0.99:23:30//NAJA HAJE HAJE (EGYPTIAN COBRA).//P01415
F-MAMMA1003056//EXPORTED PROTEIN 7 (FRAGMENT).//1.0:52:32//STREPTOCOCCUS PNEUMONIAE.//P35597
F-MAMMA1003057//MD6 PROTEIN.//1.5e-85:168:95//MUS MUSCULUS (MOUSE).//Q60584
F-MAMMA1003066//REGB PROTEIN.//1.0:62:27//PSEUDOMONAS AERUGINOSA.//Q03381
F-MAMMA1003089//!!!! ALU SUBFAMILY SB1 WARNING ENTRY !!!!//5.1e-15:44:77//HOMO SAPIENS (HUMAN).//P39190
F-MAMMA1003099//ENDOTHELIAL ACTIN-BINDING PROTEIN (ABP-280) (NONMUSCLE FILAMIN) (FILAMIN 1).//4.8e-20:80:62//HOMO SAPIENS (HUMAN).//P21333
F-MAMMA1003104//PHOTOSYSTEM I REACTION CENTRE SUBUNIT VIII.//0.98:22:40//SYNECHOCOCCUS ELONGATUS NAEGELI.//P25900
F-MAMMA1003113//PROCOLLAGEN ALPHA 2(I) CHAIN PRECURSOR (FRAGMENTS).//0.67:35:45//GALLUS GALLUS (CHICKEN).//P02467
F-MAMMA1003127//MYOSIN I ALPHA (MMI-ALPHA).//5.2e-34:141:56//MUS MUSCULUS (MOUSE).//P46735
F-MAMMA1003135//HYPOTHETICAL 182.0 KD PROTEIN IN NMD5-HOM6 INTERGENIC REGION.//3.6e-05:91:34//SACCHAROMYCES CEREVISIAE (BAKER'S YEAST).//P47170
F-MAMMA1003140
F-MAMMA1003146//MALE SPECIFIC SPERM PROTEIN MST87F.//1.0:33:36//DROSOPHILA MELANOGASTER (FRUIT FLY).//P08175
F-MAMMA1003150//HYPOTHETICAL 84.3 KD PROTEIN ZK945.10 IN CHROMOSOME II.//4.4e-10:254:30//CAENORHABDITIS ELEGANS.//Q09625
F-MAMMA1003166//BRAIN PROTEIN H5.//4.0e-42:182:48//HOMO SAPIENS (HUMAN).//O43236
F-NT2RM1000001//HYPOTHETICAL 8.7 KD PROTEIN IN RPL22-RPL23 INTERGENIC REGION (ORF70).//0.15:38:34//ASTASIA LONGA (EUGLENOPHYCEAN ALGA).//P34779
F-NT2RM1000018
F-NT2RM1000032//ATP SYNTHASE PROTEIN 8 (EC 3.6.1.34) (A6L).//0.51:17:41//CYPRINUS CARPIO (COMMON CARP).//P24948
F-NT2RM1000035//3-HYDROXY-3-METHYLGLUTARYL-COENZYME A REDUCTASE (EC 1.1.1.34) (HMG-COA REDUCTASE).//0.00011:114:27//BLATTELLA GERMANICA (GERMAN COCKROACH).//P54960
F-NT2RM1000037//METALLOTHIONEIN-II (MT-II).//0.025:19:47//SCYLLA SERRATA (MUD CRAB).//P02806
F-NT2RM1000039//VITELLINE MEMBRANE VM34CA PROTEIN PRECURSOR.//0.00083:84:33//DROSOPHILA MELANOGASTER (FRUIT FLY).//Q06521
F-NT2RM1000055//HISTIDINE-RICH GLYCOPROTEIN PRECURSOR.//1.1e-07:34:55//PLASMODIUM LOPHURAE.//P04929
F-NT2RM1000059//MYOCYTE-SPECIFIC ENHANCER FACTOR 2B (SERUM RESPONSE FACTOR-LIKE PROTEIN 2) (XMEF2) (RSRFR2).//0.18:83:36//HOMO SAPIENS (HUMAN).//Q02080
F-NT2RM1000062//PROLINE-RICH PEPTIDE P-B.//0.54:34:44//HOMO SAPIENS (HUMAN).//P02814
F-NT2RM1000080//HYPOTHETICAL 35.7 KD PROTEIN SLR1128.//2.1e-20:119:40//SYNECHOCYSTIS SP. (STRAIN PCC 6803).//P72655
F-NT2RM1000086//SALIVARY PROLINE-RICH PROTEIN PO (ALLELE K) [CONTAINS: PEPTIDE P-D] (FRAGMENT).//0.20:56:35//HOMO SAPIENS (HUMAN).//P10162
F-NT2RM1000092//COLLAGEN-LIKE PROTEIN.//0.0017:44:45//HERPESVIRUS SAIMIRI (SUBGROUP C / STRAIN 488).//P22576
F-NT2RM1000118//CALCINEURIN B SUBUNIT (PROTEIN PHOSPHATASE 2B REGULATORY SUBUNIT) (CALCINEURIN REGULATORY SUBUNIT).//5.7e-07:109:28//NEUROSPORA CRASSA.//P87072
F-NT2RM1000119//TRANSCRIPTIONAL REGULATOR IE63 (VMW63) (ICP27).//0.0050:135:32//HERPES SIMPLEX VIRUS (TYPE 2 / STRAIN HG52).//P28276
F-NT2RM1000127//EXTENSIN PRECURSOR (PROLINE-RICH GLYCOPROTEIN).//0.032:68:32//SORGHUM VULGARE (SORGHUM).//P24152
F-NT2RM1000131//METALLOTHIONEIN-III (MT-III) (GROWTH INHIBITORY FACTOR) (GIF).//0.82:33:39//BOS TAURUS (BOVINE).//P37359
F-NT2RM1000132//NADH-UBIQUINONE OXIDOREDUCTASE 13 KD-A SUBUNIT PRECURSOR (EC 1.6.5.3) (EC 1.6.99.3) (COMPLEX I-13KD-A) (CI-13KD-A).//2.7e-59:124:91//HOMO SAPIENS (HUMAN).//O75380
F-NT2RM1000153//CYTOSOLIC PURINE 5'-NUCLEOTIDASE (EC 3.1.3.5).//2.5e-08:148:29//HOMO SAPIENS (HUMAN).//P49902
F-NT2RM1000186//CALCINEURIN B SUBUNIT (PROTEIN PHOSPHATASE 2B REGULATORY SUBUNIT) (CALCINEURIN REGULATORY SUBUNIT).//1.9e-07:109:27//NEUROSPORA CRASSA.//P87072
F-NT2RM1000187//PUTATIVE PRE-MRNA SPLICING FACTOR ATP-DEPENDENT RNA HELICASE SPAC10F6.02C.//1.0e-12:94:46//SCHIZOSACCHAROMYCES POMBE (FISSION YEAST).//O42643
F-NT2RM1000199//CUTICLE COLLAGEN 12 PRECURSOR.//0.46:130:33//CAENORHABDITIS ELEGANS.//P20630
F-NT2RM1000242//PUTATIVE ATP SYNTHASE J CHAIN, MITOCHONDRIAL (EC 3.6.1.34).//0.85:38:36//SCHIZOSACCHAROMYCES POMBE (FISSION YEAST).//O13931
F-NT2RM1000244//HYPOTHETICAL 131.5 KD PROTEIN C02F12.7 IN CHROMOSOME X.//0.0055:98:36//CAENORHABDITIS ELEGANS.//Q11102
F-NT2RM1000252//TRICHOHYALIN.//2.9e-06:88:36//OVIS ARIES (SHEEP).//P22793
F-NT2RM1000256//GLUCOSAMINE--FRUCTOSE-6-PHOSPHATE AMINOTRANSFERASE [ISOMERIZING] (EC 2.6.1.16) (HEXOSEPHOSPHATE AMINOTRANSFERASE) (D-FRUCTOSE-6- PHOSPHATE AMIDOTRANSFERASE) (GFAT).//2.9e-54:153:67//MUS MUSCULUS (MOUSE).//P47856
F-NT2RM1000257//MAGO NASHI PROTEIN.//5.9e-64:136:89//DROSOPHILA MELANOGASTER (FRUIT FLY).//P49028
F-NT2RM1000260
F-NT2RM1000271//GALACTOKINASE (EC 2.7.1.6).//0.99:41:39//BACILLUS SUBTILIS.//P39574
F-NT2RM1000272//HYPOTHETICAL 55.5 KD PROTEIN ZK1128.2 IN CHROMOSOME III.//8.8e-25:131:45//CAENORHABDITIS ELEGANS.//Q09357
F-NT2RM1000280//VACUOLAR ATP SYNTHASE SUBUNIT D (EC 3.6.1.34) (V-ATPASE D SUBUNIT) (V- ATPASE 28 KD ACCESSORY PROTEIN).//2.5e-63:121:94//BOS TAURUS (BOVINE).//P39942
F-NT2RM1000300//TREACLE PROTEIN (TREACHER COLLINS SYNDROME PROTEIN).//0.51:145:26//HOMO SAPIENS (HUMAN).//Q13428
F-NT2RM1000314
F-NT2RM1000318//50S RIBOSOMAL PROTEIN L23.//0.83:28:35//AQUIFEX AEOLICUS.//O66433
F-NT2RM1000341
F-NT2RM1000354//HYPOTHETICAL 5.8 KD PROTEIN IN PUHA 5'REGION (ORF55).//0.95:43:37//RHODOBACTER CAPSULATUS (RHODOPSEUDOMONAS CAPSULATA).//P26159
F-NT2RM1000355//SPERM-SPECIFIC PROTEIN PHI-1.//0.0016:73:43//MYTILUS EDULIS (BLUE MUSSEL).//Q04621
F-NT2RM1000365//HYPOTHETICAL PROTEIN KIAA0140.//3.5e-10:83:49//HOMO SAPIENS (HUMAN).//Q14153
F-NT2RM1000377//DUAL SPECIFICITY PROTEIN PHOSPHATASE 9 (EC 3.1.3.48) (EC 3.1.3.16) (MITOGEN-ACTIVATED PROTEIN KINASE PHOSPHATASE 4) (MAP KINASE PHOSPHATASE 4) (MKP-4).//4.9e-18:113:38//HOMO SAPIENS (HUMAN).//Q99956
F-NT2RM1000388//HYPOTHETICAL 27.7 KD PROTEIN IN CPT1-SPC98 INTERGENIC REGION.//0.00023:67:31//SACCHAROMYCES CEREVISIAE (BAKER'S YEAST).//P53915
F-NT2RM1000394//HISTONE H3.3 (H3.B) (H3.3Q).//4.7e-52:71:91//HOMO SAPIENS (HUMAN), MUS MUSCULUS (MOUSE), RATTUS NORVEGICUS (RAT), ORYCTOLAGUS CUNICULUS (RABBIT), GALLUS GALLUS (CHICKEN), SPISULA SOLIDISSIMA (ATLANTIC SURF-CLAM), DROSOPHILA MELANOGASTER (FRUIT FLY), AND DROSOPHILA HYDEI (FRUIT FLY).//P06351
F-NT2RM1000399//ENDOTHELIN-2 PRECURSOR (ET-2) (FRAGMENT).//0.92:24:45//CANIS FAMILIARIS (DOG).//P12064
F-NT2RM1000421//CUTICLE COLLAGEN 2C (FRAGMENT).//0.12:93:33//HAEMONCHUS CONTORTUS.//P16252
F-NT2RM1000430//PISTIL-SPECIFIC EXTENSIN-LIKE PROTEIN PRECURSOR (PELP).//0.13:86:31//NICOTIANA TABACUM (COMMON TOBACCO).//Q03211
F-NT2RM1000499//HYPOTHETICAL PROTEIN KIAA0041 (FRAGMENT).//2.9e-17:75:49//HOMO SAPIENS (HUMAN).//Q15057
F-NT2RM1000539//HYPOTHETICAL 10.4 KD PROTEIN IN FTR1-SPT15 INTERGENIC REGION.//2.9e-16:82:51//SACCHAROMYCES CEREVISIAE (BAKER'S YEAST).//P40089
F-NT2RM1000553//GLYCOLIPID TRANSFER PROTEIN (GLTP).//6.4e-06:103:33//SUS SCROFA (PIG).//P17403
F-NT2RM1000555//UNR PROTEIN.//8.7e-77:105:95//RATTUS NORVEGICUS (RAT).//P18395
F-NT2RM1000563//DNA-BINDING P52/P100 COMPLEX, 100 KD SUBUNIT (FRAGMENTS).//0.15:20:50//HOMO SAPIENS (HUMAN).//P30808
F-NT2RM1000623//CLARA CELL PHOSPHOLIPID-BINDING PROTEIN PRECURSOR (CCPBP) (CLARA CELLS 10 KD SECRETORY PROTEIN) (CC10).//0.17:70:34//HOMO SAPIENS (HUMAN).//P11684
F-NT2RM1000648//GLYCOSYLTRANSFERASE ALG2 (EC 2.4.1.-).//2.0e-22:133:42//SACCHAROMYCES CEREVISIAE (BAKER'S YEAST).//P43636
F-NT2RM1000661//METALLOTHIONEIN-III (MT-III) (GROWTH INHIBITORY FACTOR) (GIF) (GIFB).//0.0060:24:33//HOMO SAPIENS (HUMAN).//P25713
F-NT2RM1000666//COLD SHOCK PROTEIN SCOF.//9.1e-07:67:41//STREPTOMYCES COELICOLOR.//P48859
F-NT2RM1000669//CHLOROPLAST 50S RIBOSOMAL PROTEIN L31.//0.071:69:31//PORPHYRA PURPUREA.//P51290
F-NT2RM1000672//SIGNAL RECOGNITION PARTICLE SEC65 SUBUNIT (FRAGMENT).//0.27:42:42//KLUYVEROMYCES LACTIS (YEAST).//O13475
F-NT2RM1000691//RETINOBLASTOMA BINDING PROTEIN 2 (RBBP-2).//4.3e-42:241:42//HOMO SAPIENS (HUMAN).//P29375
F-NT2RM1000699//N2,N2-DIMETHYLGUANOSINE TRNA METHYLTRANSFERASE PRECURSOR (EC 2.1.1.32).//0.94:48:37//SACCHAROMYCES CEREVISIAE (BAKER'S YEAST).//P15565
F-NT2RM1000702//GUANINE NUCLEOTIDE-BINDING PROTEIN BETA SUBUNIT 1.//0.0013:139:25//DROSOPHILA MELANOGASTER (FRUIT FLY).//P26308
F-NT2RM1000725//BASIC PROLINE-RICH PEPTIDE P-E (IB-9).//1.0:15:60//HOMO SAPIENS (HUMAN).//P02811
F-NT2RM1000741//STATHMIN (CLONE XO20) (FRAGMENT).//1.0:53:32//XENOPUS LAEVIS (AFRICAN CLAWED FROG).//Q09005
F-NT2RM1000742//HYPOTHETICAL 24.1 KD PROTEIN IN DHFR 3'REGION (ORF2).//1.0:54:42//HERPESVIRUS SAIMIRI (STRAIN 484-77).//P25049
F-NT2RM1000746//HYPOTHETICAL 16.8 KD PROTEIN C29E6.04 IN CHROMOSOME I.//0.11:87:21//SCHIZOSACCHAROMYCES POMBE (FISSION YEAST).//Q09858
F-NT2RM1000770//DXS6673E PROTEIN.//2.0e-38:190:48//HOMO SAPIENS (HUMAN).//Q14202
F-NT2RM1000772//VEGETATIBLE INCOMPATIBILITY PROTEIN HET-E-1.//4.3e-12:141:30//PODOSPORA ANSERINA.//Q00808
F-NT2RM1000780//MALE SPECIFIC SPERM PROTEIN MST87F.//0.98:34:38//DROSOPHILA MELANOGASTER (FRUIT FLY).//P08175
F-NT2RM1000781
F-NT2RM1000800//24.1 KD PROTEIN IN VMA12-APN1 INTERGENIC REGION.//7.9e-11:135:34//SACCHAROMYCES CEREVISIAE (BAKER'S YEAST).//P28707
F-NT2RM1000802//ALPHA-AMYLASE INHIBITOR PAIM I (PIG PANCREATIC ALPHA-AMYLASE INHIBITOR OF MICROBES I).//0.43:62:35//STREPTOMYCES OLIVACEOVIRIDIS (STREPTOMYCES CORCHORUSII).//P09921
F-NT2RM1000811
F-NT2RM1000826//UNR PROTEIN.//1.1e-110:144:83//RATTUS NORVEGICUS (RAT).//P18395
F-NT2RM1000829//ATP SYNTHASE PROTEIN 8 (EC 3.6.1.34) (A6L).//1.0:38:34//DROSOPHILA SIMULANS (FRUIT FLY).//P50270
F-NT2RM1000833//PROTEIN TRANSPORT PROTEIN SEC61 ALPHA SUBUNIT.//1.4e-62:145:841/CANIS FAMILIARIS (DOG).//P38377
F-NT2RM1000850//TESTIS-SPECIFIC PROTEIN KINASE 1 (EC 2.7.1.-).//6.1e-08:136:33//RATTUS NORVEGICUS (RAT).//Q63572
F-NT2RM1000852//ATP-DEPENDENT RNA HELICASE ROK1.//1.6e-34:212:43//SACCHAROMYCES CEREVISIAE (BAKER'S YEAST).//P45818
F-NT2RM1000857//HISTONE H1.M6.1.//0.76:31:48//TRYPANOSOMA CRUZI.//P40273
F-NT2RM1000867//MICROSOMAL SIGNAL PEPTIDASE 10.8 KD SUBUNIT (EC 3.4.-.-).//0.0082:76:25//SACCHAROMYCES CEREVISIAE (BAKER'S YEAST).//P46965
F-NT2RM1000874//DNA-BINDING P52/P100 COMPLEX, 100 KD SUBUNIT (FRAGMENTS).//0.38:12:58//HOMO SAPIENS (HUMAN).//P30808
F-NT2RM1000882//CYTOCHROME B5.//9.0e-13:92:38//SACCHAROMYCES CEREVISIAE (BAKER'S YEAST).//P40312
F-NT2RM1000883//DNA-BINDING P52/P100 COMPLEX, 100 KD SUBUNIT (FRAGMENTS).//0.79:22:59//HOMO SAPIENS (HUMAN).//P30808
F-NT2RM1000885//HYPOTHETICAL 5.8 KD PROTEIN.//0.76:18:38//CLOVER YELLOW MOSAIC VIRUS (CYMV).//P16485
F-NT2RM1000894//DNA-DIRECTED RNA POLYMERASE I135 KD POLYPEPTIDE (EC 2.7.7.6) (RNA POLYMERASE I SUBUNIT 2) (RPA135) (RNA POLYMERASE I 127 KD SUBUNIT).//6.2e-70:153:88//RATTUS NORVEGICUS (RAT).//O54888
F-NT2RM1000898//ACTIN, CYTOPLASMIC (ACTIN, MICRONUCLEAR).//4.3e-12:159:28//OXYTRICHA FALLAX.//P02583
F-NT2RM1000905//GLUTATHIONE S-TRANSFERASE 1-1 (EC 2.5.1.18) (CLASS-THETA).//0.98:39:35//LUCILIA CUPRINA (GREENBOTTLE FLY) (AUSTRALIAN SHEEP BLOWFLY).//P42860
F-NT2RM1000924//HYPOTHETICAL 39.7 KD PROTEIN C34E10.2 IN CHROMOSOME III.//1.3e-11:169:28//CAENORHABDITIS ELEGANS.//P46577
F-NT2RM1000927//CUTICLE COLLAGEN 1.//0.00048:141:31//CAENORHABDITIS ELEGANS.//P08124
F-NT2RM1000962//HYPOTHETICAL 35.8 KD PROTEIN C4F8.04 IN CHROMOSOME I.//7.1e-13:169:31//SCHIZOSACCHAROMYCES POMBE (FISSION YEAST).//O14180
F-NT2RM1000978//HYPOTHETICAL 20.2 KD PROTEIN IN MNN4-PTK1 INTERGENIC REGION.//0.61:82:34//SACCHAROMYCES CEREVISIAE (BAKER'S YEAST).//P36045
F-NT2RM1001003//ALPHA-2 CATENIN (ALPHA N-CATENIN) (NEURAL ALPHA-CATENIN).//1.6e-21:211:31//GALLUS GALLUS (CHICKEN).//P30997
F-NT2RM1001008//HYPOTHETICAL 72.5 KD PROTEIN C2F7.10 IN CHROMOSOME I.//3.2e-15:119:36//SCHIZOSACCHAROMYCES POMBE (FISSION YEAST).//Q09701
F-NT2RM1001043//ENDOTHELIN-1 (ET-1) (FRAGMENT).//0.78:32:34//MACACA FASCICULARIS (CRAB EATING MACAQUE) (CYNOMOLGUS MONKEY).//Q28469
F-NT2RM1001044
F-NT2RM1001059//LORICRIN.//8.6e-08:108:39//HOMO SAPIENS (HUMAN).//P23490
F-NT2RM1001066//METALLOTHIONEIN-LIKE PROTEIN TYPE 2.//0.99:24:50//LYCOPERSICON ESCULENTUM (TOMATO).//Q43513
F-NT2RM1001072//1-PHOSPHATIDYLINOSITOL-4,5-BISPHOSPHATE PHOSPHODIESTERASE GAMMA 1 (EC 3.1.4.11) (PLC-GAMMA-1) (PHOSPHOLIPASE C-GAMMA-1) (PLC-II) (PLC-148).//4.7e-15:148:33//HOMO SAPIENS (HUMAN).//P19174
F-NT2RM1001074//HYPOTHETICAL PROTEIN F-215.//8.6e-05:126:30//HUMAN ADENOVIRUS TYPE 2.//P03291
F-NT2RM1001082//!!!! ALU SUBFAMILY SX WARNING ENTRY !!!!//6.5e-19:75:54//HOMO SAPIENS (HUMAN).//P39195
F-NT2RM1001085//MALE SPECIFIC SPERM PROTEIN MST84DB.//0.49:29:41//DROSOPHILA MELANOGASTER (FRUIT FLY).//Q01643
F-NT2RM1001092//ZINC FINGER PROTEIN 83 (ZINC FINGER PROTEIN HPF1).//2.8e-42:200:38//HOMO SAPIENS (HUMAN).//P51522
F-NT2RM1001102//HYPOTHETICAL 62.8 KD PROTEIN IN TAF145-YOR1 INTERGENIC REGION.//1.7e-18:161:36//SACCHAROMYCES CEREVISIAE (BAKER'S YEAST).//P53331
F-NT2RM1001105//HYPOTHETICAL 35.5 KD PROTEIN IN TRANSPOSON TN4556.//4.0e-05:157:35//STREPTOMYCES FRADIAE.//P20186
F-NT2RM1001112//NONHISTONE CHROMOSOMAL PROTEIN HMG-17.//0.18:20:55//BOS TAURUS (BOVINE).//P02313
F-NT2RM1001115
F-NT2RM1001139//GLYCINE-RICH CELL WALL STRUCTURAL PROTEIN 1.8 PRECURSOR (GRP 1.8).//2.0e-25:156:46//PHASEOLUS VULGARIS (KIDNEY BEAN) (FRENCH BEAN).//P10496
F-NT2RM2000006//MITOCHONDRIAL RIBOSOMAL PROTEIN S12.//0.76:45:35//LEISHMANIA TARENTOLAE (SAUROLEISHMANIA TARENTOLAE).//Q34940
F-NT2RM2000013//DNA-DIRECTED RNA POLYMERASE III 128 KD POLYPEPTIDE (EC 2.7.7.6) (RNA POLYMERASE III SUBUNIT 2).//3.9e-87:238:65//DROSOPHILA MELANOGASTER (FRUIT FLY).//P25167
F-NT2RM2000030//TOXINS 1 AND 2.//0.98:21:42//TRIMERESURUS WAGLERI (WAGLER'S PIT VIPER) (TROPIDOLAEMUS WAGLERI).//P24335
F-NT2RM2000032//!!!! ALU SUBFAMILY J WARNING ENTRY !!!!//0.00059:53:49//HOMO SAPIENS (HUMAN).//P39188
F-NT2RM2000042//SMALL PROLINE-RICH PROTEIN II (SPR-II) (CLONE 174N).//1.0:68:26//HOMO SAPIENS (HUMAN).//P22532
F-NT2RM2000092//HYPOTHETICAL 67.5 KD PROTEIN IN PRPS4-STE20 INTERGENIC REGION.//7.0e-11:80:40//SACCHAROMYCES CEREVISIAE (BAKER'S YEAST).//P38748
F-NT2RM2000093//OVARY MATURATING PARSIN (OMP).//1.0:26:38//LOCUSTA MIGRATORIA (MIGRATORY LOCUST).//P80045
F-NT2RM2000101//HYPOTHETICAL 39.3 KD PROTEIN C02B8.6 IN CHROMOSOME X.//3.3e-09:56:35//CAENORHABDITIS ELEGANS.//Q11096
F-NT2RM2000124//CAMP-DEPENDENT PROTEIN KINASE, ALPHA-CATALYTIC SUBUNIT (EC 2.7.1.37) (PKA C-ALPHA).//3.1e-35:77:96//MUS MUSCULUS (MOUSE).//P05132
F-NT2RM2000191//3',5'-CYCLIC-NUCLEOTIDE PHOSPHODIESTERASE REGA (EC 3.1.4.17) (PDEASE REGA).//3.3e-05:181:27//DICTYOSTELIUM DISCOIDEUM (SLIME MOLD).//Q23917
F-NT2RM2000192//REPLICATION PROTEIN E1 (FRAGMENTS).//0.019:148:25//COTTONTAIL RABBIT (SHOPE) PAPILLOMAVIRUS (STRAIN WASHINGTON B) (CRPV).//P51894
F-NT2RM2000239//PROLINE-RICH PROTEIN MP-3 (FRAGMENT).//0.00032:111:32//MUS MUSCULUS (MOUSE).//P05143
F-nnnnnnnnnnnn//METALLOTHIONEIN-LIKE PROTEIN TYPE 2.//0.046:59:33//LYCOPERSICON ESCULENTUM (TOMATO).//Q43512
F-NT2RM2000250//GALECTIN-3 (GALACTOSE-SPECIFIC LECTIN 3) (MAC-2 ANTIGEN) (IGE-BINDING PROTEIN) (35 KD LECTIN) (CARBOHYDRATE BINDING PROTEIN 35) (CBP 35) (LAMININ-BINDING PROTEIN) (LECTIN L-29).//0.054:46:34//RATTUS NORVEGICUS (RAT).//P08699
F-NT2RM2000259//TRANS-ACTING TRANSCRIPTIONAL PROTEIN ICP0 (P135 PROTEIN) (IER 2.9/ER2.6).//0.27:112:33//BOVINE HERPES VIRUS TYPE 1 (STRAIN JURA).//P29128
F-NT2RM2000260//PROLINE-RICH PROTEIN MP-3 (FRAGMENT).//4.7e-22:191:35//MUS MUSCULUS (MOUSE).//P05143
F-NT2RM2000287//HYPOTHETICAL 11.8 KD PROTEIN C1B3.02C IN CHROMOSOME I.//5.0e-19:83:53//SCHIZOSACCHAROMYCES POMBE (FISSION YEAST).//O13868
F-NT2RM2000322//DIAMINOPIMELATE DECARBOXYLASE (EC 4.1.1.20) (DAP DECARBOXYLASE).//0.47:117:29//HELICOBACTER PYLORI (CAMPYLOBACTER PYLORI).//P56129
F-NT2RM2000359//SPORE GERMINATION PROTEIN 270-11.//0.12:83:36//DICTYOSTELIUM DISCOIDEUM (SLIME MOLD).//P22698
F-NT2RM2000363//BREAKPOINT CLUSTER REGION PROTEIN.//1.3e-16:203:30//HOMO SAPIENS (HUMAN).//P11274
F-NT2RM2000368//DEK PROTEIN.//0.00027:100:32//HOMO SAPIENS (HUMAN).//P35659
F-NT2RM2000371//POLYRIBONUCLEOTIDE NUCLEOTIDYLTRANSFERASE (EC 2.7.7.8) (POLYNUCLEOTIDE PHOSPHORYLASE) (PNPASE).//6.8e-36:170:47//ESCHERICHIA COLI.//P05055
F-NT2RM2000374//NODAL PRECURSOR.//1.1e-32:64:95//MUS MUSCULUS (MOUSE).//P43021
F-NT2RM2000395//IMMEDIATE-EARLY PROTEIN IE180.//0.31:41:43//PSEUDORABIES VIRUS (STRAIN INDIANA-FUNKHAUSER / BECKER) (PRV).//P11675
F-NT2RM2000402//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//1.2e-30:228:32//SACCHAROMYCES CEREVISIAE (BAKER'S YEAST).//P32802
F-NT2RM2000407//TRANSMEMBRANE PROTEIN SEX PRECURSOR.//0.032:105:30//HOMO SAPIENS (HUMAN).//P51805
F-NT2RM2000420//METALLOTHIONEIN (MT).//0.88:42:38//PLEURONECTES PLATESSA (PLAICE).//P07216
F-NT2RM2000422//SODIUM- AND CHLORIDE-DEPENDENT TRANSPORTER NTT73.//2.0e-117:237:87//RATTUS NORVEGICUS (RAT).//Q08469
F-NT2RM2000452//HYPOTHETICAL 63.6 KD PROTEIN IN YPT52-GCN3 INTERGENIC REGION.//1.1e-08:157:28//SACCHAROMYCES CEREVISIAE (BAKER'S YEAST).//P36113
F-NT2RM2000469//70 KD ANTIGEN.//0.050:207:23//SHIGELLA FLEXNERI.//P18010
F-NT2RM2000490//BASIC PROLINE-RICH PEPTIDE P-E (IB-9).//0.022:25:44//HOMO SAPIENS (HUMAN).//P02811
F-NT2RM2000502//MALE SPECIFIC SPERM PROTEIN MST84DD.//0.0037:17:58//DROSOPHILA MELANOGASTER (FRUIT FLY).//Q01645
F-NT2RM2000504//HYPOTHETICAL 99.0 KD PROTEIN SPBC119.17.//1.7e-22:195:27//SCHIZOSACCHAROMYCES POMBE (FISSION YEAST).//O42908
F-NT2RM2000522//RAS-RELATED PROTEIN RABA (FRAGMENT).//3.6e-05:67:29//DICTYOSTELIUM DISCOIDEUM (SLIME MOLD).//P34141
F-NT2RM2000540//HYPOTHETICAL 83.8 KD PROTEIN C27F2.7 IN CHROMOSOME III.//8.4e-33:214:38//CAENORHABDITIS ELEGANS.//Q18262
F-NT2RM2000556//HYPOTHETICAL PROTEIN KIAA0288 (HA6116).//1.7e-09:133:36//HOMO SAPIENS (HUMAN).//P56524
F-NT2RM2000566//INTEGRIN ALPHA-6 PRECURSOR (VLA-6) (CD49F).//2.2e-60:244:51//HOMO SAPIENS (HUMAN).//P23229
F-NT2RM2000567//PROLINE-RICH PROTEIN MP-3 (FRAGMENT).//2.3e-09:192:34//MUS MUSCULUS (MOUSE).//P05143
F-NT2RM2000569//!!!! ALU SUBFAMILY J WARNING ENTRY !!!!//9.0e-08:43:72//HOMO SAPIENS (HUMAN).//P39188
F-NT2RM2000577//ISOLEUCYL-TRNA SYNTHETASE (EC 6.1.1.5) (ISOLEUCINE--TRNA LIGASE) (ILERS).//9.1e-54:225:45//SYNECHOCYSTIS SP. (STRAIN PCC 6803).//P73505
F-NT2RM2000581//SPLICEOSOME ASSOCIATED PROTEIN 49 (SAP 49) (SF3B53).//0.079:111:34//HOMO SAPIENS (HUMAN).//Q15427
F-NT2RM2000588//HYPOTHETICAL PROTEIN KIAA0288 (HA6116).//2.3e-09:193:32//HOMO SAPIENS (HUMAN).//P56524
F-NT2RM2000594//BASIC PROLINE-RICH PEPTIDE P-E (IB-9).//0.18:33:42//HOMO SAPIENS (HUMAN).//P02811
F-NT2RM2000599//DNA (CYTOSINE-5)-METHYLTRANSFERASE (EC 2.1.1.37) (DNA METHYLTRANSFERASE) (DNA METASE) (MCMT) (M.MMUI).//1.5e-09:68:45//MUS MUSCULUS (MOUSE).//P13864
F-NT2RM2000609//GRANULIN 2.//0.83:42:35//CYPRINUS CARPIO (COMMON CARP).//P81014
F-NT2RM2000612//ZINC FINGER PROTEIN GCS1.//7.2e-05:155:29//SACCHAROMYCES CEREVISIAE (BAKER'S YEAST).//P35197
F-NT2RM2000623//A-AGGLUTININ ATTACHMENT SUBUNIT PRECURSOR.//1.8e-09:196:33//SACCHAROMYCES CEREVISIAE (BAKER'S YEAST).//P32323
F-NT2RM2000624//SALIVARY GLUE PROTEIN SGS-3 PRECURSOR.//0.070:113:27//DROSOPHILA ERECTA (FRUIT FLY).//P13730
F-NT2RM2000635//SPERM PROTAMINE P1.//0.54:47:38//ANTECHINUS STUARTII.//P42129
F-NT2RM2000636//OUTER MEMBRANE PROTEIN H.8 PRECURSOR.//0.096:62:35//NEISSERIA GONORRHOEAE.//P11910
F-NT2RM2000639//HYPOTHETICAL PROTEIN MJ0243.//0.99:32:34//METHANOCOCCUS JANNASCHII.//Q57694
F-NT2RM2000649//NEURONAL CALCIUM SENSOR 1 (NCS-1).//0.00049:70:35//RATTUS NORVEGICUS (RAT), AND GALLUS GALLUS (CHICKEN).//P36610
F-NT2RM2000669//50S RIBOSOMAL PROTEIN L34.//1.0:34:44//BACILLUS SUBTILIS.//P05647
F-NT2RM2000691//ACTIN-LIKE PROTEIN 3 (ACTIN-2).//7.0e-116:243:87//HOMO SAPIENS (HUMAN), AND BOS TAURUS (BOVINE).//P32391
F-NT2RM2000714//RAS SUPPRESSOR PROTEIN 1 (RSU-1) (RSP-1 PROTEIN) (RSP-l).//3.8e-21:174:35//HOMO SAPIENS (HUMAN).//Q15404
F-NT2RM2000718//HYPOTHETICAL 52.9 KD SERINE-RICH PROTEIN C11G7.01 IN CHROMOSOME I.//0.0022:174:29//SCHIZOSACCHAROMYCES POMBE (FISSION YEAST).//O13695
F-NT2RM2000735//ZINC FINGER PROTEIN 43 (ZINC PROTEIN HTF6).//1.6e-102:246:74//HOMO SAPIENS (HUMAN).//P28160
F-NT2RM2000740//HYPOTHETICAL 131.1 KD HELICASE IN ALG7-ENP1 INTERGENIC REGION.//8.5e-51:212:49//SACCHAROMYCES CEREVISIAE (BAKER'S YEAST).//P38144
F-NT2RM2000795//!!!! ALU SUBFAMILY SB WARNING ENTRY !!!!//9.0e-41:125:53//HOMO SAPIENS (HUMAN).//P39189
F-NT2RM2000821//COATOMER BETA SUBUNIT (BETA-COAT PROTEIN) (BETA-COP).//1.1e-128:291:89//RATTUS NORVEGICUS (RAT).//P23514
F-NT2RM2000837//CYCLIN-DEPENDENT KINASE INHIBITOR 1C (CYCLIN-DEPENDENT KINASE INHIBITOR P57) (P57KIP2).//3.9e-05:113:36//HOMO SAPIENS (HUMAN).//P49918
F-NT2RM2000951//HYPOTHETICAL 60.3 KD PROTEIN R08D7.7 IN CHROMOSOME III.//2.5e-49:273:39//CAENORHABDITIS ELEGANS.//P30646
F-NT2RM2000952//NEUROFILAMENT TRIPLET H PROTEIN (200 KD NEUROFILAMENT PROTEIN) (NF-H) (FRAGMENT).//0.037:234:23//RATTUS NORVEGICUS (RAT).//P16884
F-NT2RM2000984//HYPOTHETICAL 54.7 KD PROTEIN F37A4.1 IN CHROMOSOME III.//6.3e-44:216:43//CAENORHABDITIS ELEGANS.//P41879
F-NT2RM2001004//SYNAPSINS IA AND IB.//0.15:178:32//RATTUS NORVEGICUS (RAT).//P09951
F-NT2RM2001035//CCR4-ASSOCIATED FACTOR 1 (CAF1).//1.4e-87:188:90//MUS MUSCULUS (MOUSE).//Q60809
F-NT2RM2001065//ATP SYNTHASE A CHAIN (EC 3.6.1.34) (PROTEIN 6).//0.53:122:31//TRYPANOSOMA BRUCEI BRUCEI.//P24499
F-NT2RM2001100//HYPOTHETICAL 39.7 KD PROTEIN C34E10.2 IN CHROMOSOME III.//3.4e-13:171:30//CAENORHABDITIS ELEGANS.//P46577
F-NT2RM2001105//SPORE COAT PROTEIN SP96.//7.8e-06:141:34//DICTYOSTELIUM DISCOIDEUM (SLIME MOLD).//P14328
F-NT2RM2001131//PROBABLE EUKARYOTIC INITIATION FACTOR C17C9.03.//2.3e-18:249:31//SCHIZOSACCHAROMYCES POMBE (FISSION YEAST).//Q10475
F-NT2RM2001141//HYPOTHETICAL 115.4 KD PROTEIN ZK757.3 IN CHROMOSOME III.//0.050:134:26//CAENORHABDITIS ELEGANS.//P34681
F-NT2RM2001152
F-NT2RM2001177//COLLAGEN ALPHA 1(XIV) CHAIN PRECURSOR (UNDULIN).//0.86:42:40//GALLUS GALLUS (CHICKEN).//P32018
F-NT2RM2001194//SMOOTHELIN.//4.7e-05:77:32//HOMO SAPIENS (HUMAN).//P53814
F-NT2RM2001196//PROLINE-RICH PROTEIN MP-3 (FRAGMENT).//1.7e-18:218:35//MUS MUSCULUS (MOUSE).//P05143
F-NT2RM2001201//CYSTEINE STRING PROTEIN (CCCS1).//0.041:22:59//TORPEDO CALIFORNICA (PACIFIC ELECTRIC RAY).//P56101
F-NT2RM2001221//KALIRIN (PAM COOH-TERMINAL INTERACTOR PROTEIN 10) (P-CIP10).//1.3e-13:183:32//RATTUS NORVEGICUS (RAT).//P97924
F-NT2RM2001238//GLUTAMINASE, KIDNEY ISOFORM PRECURSOR (EC 3.5.1.2) (GLS) (L-GLUTAMINE AMIDOHYDROLASE).//6.5e-121:218:98//RATTUS NORVEGICUS (RAT).//P13264
F-NT2RM2001243//HYPOTHETICAL 200.0 KD PROTEIN IN GZF3-IME2 INTERGENIC REGION.//0.00019:177:27//SACCHAROMYCES CEREVISIAE (BAKER'S YEAST).//P42945
F-NT2RM2001247//LEGUMIN B (FRAGMENT).//0.22:54:35//PISUM SATIVUM (GARDEN PEA).//P14594
F-NT2RM2001256//PROTEIN TSG24 (MEIOTIC CHECK POINT REGULATOR).//1.8e-109:207:98//MUS MUSCULUS (MOUSE).//P53995
F-NT2RM2001291//SMALL PROLINE RICH PROTEIN II (SPR-II) (CLONE 930).//0.016:22:40//HOMO SAPIENS (HUMAN).//P22531
F-NT2RM2001306//REF(2)P PROTEIN.//0.61:51:33//DROSOPHILA MELANOGASTER (FRUIT FLY).//P14199
F-NT2RM2001312//!!!! ALU SUBFAMILY SX WARNING ENTRY !!!!//7.2e-11:33:72//HOMO SAPIENS (HUMAN).//P39195
F-NT2RM2001319
F-NT2RM2001324//ZYXIN.//5.1e-22:91:38//GALLUS GALLUS (CHICKEN).//Q04584
F-NT2RM2001345//VEGETATIBLE INCOMPATIBILITY PROTEIN HET-E-1.//7.4e-10:159:27//PODOSPORA ANSERINA.//Q00808
F-NT2RM2001360//ACCESSORY GLAND PEPTIDE PRECURSOR (PARAGONIAL PEPTIDE B).//1.0:27:48//DROSOPHILA MELANOGASTER (FRUIT FLY).//P05623
F-NT2RM2001370//NAPE PROTEIN.//0.98:44:31//PARACOCCUS DENITRIFICANS (SUBSP. THIOSPHAERA PANTOTROPHA).//Q56348
F-NT2RM2001393//VITELLOGENIN PRECURSOR (VTG) [CONTAINS: LIPOVITELLIN LV-1N; LIPOVITELLIN LV-1C; LIPOVITELLIN LV-2].//0.0024:163:31//ICHTHYOMYZON UNICUSPUS (SILVER LAMPREY).//Q91062
F-NT2RM2001420
F-NT2RM2001424//HETEROGENOUS NUCLEAR RIBONUCLEOPROTEIN U (HNRNP U).//2.4e-41:140:59//HOMO SAPIENS (HUMAN).//Q00839
F-NT2RM2001499//HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).//3.7e-71:201:68//HOMO SAPIENS (HUMAN).//P30825
F-NT2RM2001504//CUTICLE COLLAGEN 2.//0.028:41:39//CAENORHABDITIS ELEGANS.//P17656
F-NT2RM2001524//HYPOTHETICAL 61.3 KD PROTEIN F25B5.5 IN CHROMOSOME III.//6.7e-47:190:42//CAENORHABDITIS ELEGANS.//Q09316
F-NT2RM2001544//TELOMERE-BINDING PROTEIN 51 KD SUBUNIT.//0.0027:136:33//EUPLOTES CRASSUS.//Q06184
F-NT2RM2001547//HYPOTHETICAL 48.6 KD PROTEIN IN BET1-PAN1 INTERGENIC REGION.//8.5e-18:91:50//SACCHAROMYCES CEREVISIAE (BAKER'S YEAST).//P40564
F-NT2RM2001575//52 KD RO PROTEIN (SJOGREN SYNDROME TYPE A ANTIGEN (SS-A)) (RO(SS-A)).//3.9e-35:212:41//HOMO SAPIENS (HUMAN).//P19474
F-NT2RM2001582//RESA PROTEIN.//0.0033:72:27//BACILLUS SUBTILIS.//P35160
F-NT2RM2001588//EXTENSIN PRECURSOR (PROLINE-RICH GLYCOPROTEIN).//1.0e-06:115:32//ZEA MAYS (MAIZE).//P14918
F-NT2RM2001592//KERATIN, ULTRA HIGH-SULFUR MATRIX PROTEIN (UHS KERATIN).//0.033:156:23//HOMO SAPIENS (HUMAN).//P26371
F-NT2RM2001605//RETINOBLASTOMA BINDING PROTEIN 2 (RBBP-2).//1.1e-116:249:82//HOMO SAPIENS (HUMAN).//P29375
F-NT2RM2001613//PROTEIN TRANSPORT PROTEIN SEC61 ALPHA SUBUNIT.//1.2e-97:192:100//RATTUS NORVEGICUS (RAT).//P38378
F-NT2RM2001632//A-AGGLUTININ ATTACHMENT SUBUNIT PRECURSOR.//0.00068:145:28//SACCHAROMYCES CEREVISIAE (BAKER'S YEAST).//P32323
F-NT2RM2001635//NUCLEAR ENVELOPE PORE MEMBRANE PROTEIN POM 121 (PORE MEMBRANE PROTEIN OF 121 KD) (P145).//1.1e-39:235:47//RATTUS NORVEGICUS (RAT).//P52591
F-NT2RM2001637//HYPOTHETICAL BHLF1 PROTEIN.//0.075:197:29//EPSTEIN-BARR VIRUS (STRAIN B95-8) (HUMAN HERPESVIRUS 4).//P03181
F-NT2RM2001641//NADH-CYTOCHROME B5 REDUCTASE (EC 1.6.2.2) (B5R).//0.013:29:68//HOMO SAPIENS (HUMAN).//P00387
F-NT2RM2001648//PROTEIN TRANSPORT PROTEIN SEC61 ALPHA SUBUNIT.//3.2e-65:132:100//CANIS FAMILIARIS (DOG).//P38377
F-NT2RM2001652//PROTEIN TRANSPORT PROTEIN SEC7.//1.6e-32:261:32//SACCHAROMYCES CEREVISIAE (BAKER'S YEAST).//P11075
F-NT2RM2001659//CARBOXYPEPTIDASE A INHIBITOR.//0.83:30:46//ASCARIS SUUM (PIG ROUNDWORM) (ASCARIS LUMBRICOIDES).//P19399
F-NT2RM2001664//IKI3 PROTEIN.//1.3e-31:265:34//SACCHAROMYCES CEREVISIAE (BAKER'S YEAST).//Q06706
F-NT2RM2001668//TONB PROTEIN.//0.32:39:41//XANTHOMONAS CAMPESTRIS (PV. CAMPESTRIS).//O34261
F-NT2RM2001670//ZINC FINGER PROTEIN 174.//3.6e-21:172:39//HOMO SAPIENS (HUMAN).//Q15697
F-NT2RM2001671//HYPOTHETICAL 118.6 KD PROTEIN C29E6.03C IN CHROMOSOME I.//1.6e-10:229:24//SCHIZOSACCHAROMYCES POMBE (FISSION YEAST).//Q09857
F-NT2RM2001675//DIHYDRODIPICOLINATE SYNTHASE (EC 4.2.1.52) (DHDPS).//1.0:184:21//METHANOCOCCUS JANNASCHII.//Q57695
F-NT2RM2001681//PROTEIN DISULFIDE ISOMERASE PRECURSOR (PDI) (EC 5.3.4.1).//0.0039:199:22//DROSOPHILA MELANOGASTER (FRUIT FLY).//P54399
F-NT2RM2001688//HYPOTHETICAL 28.1 KD PROTEIN IN SIPU-PBPC INTERGENIC REGION.//2.6e-21:162:33//BACILLUS SUBTILIS.//P42966
F-NT2RM2001695//!!!! ALU SUBFAMILY SQ WARNING ENTRY !!!!//4.9e-41:60:81//HOMO SAPIENS (HUMAN).//P39194
F-NT2RM2001696//HYPOTHETICAL 24.1 KD PROTEIN IN LEF4-P33 INTERGENIC REGION.//9.8e-16:126:38//AUTOGRAPHA CALIFORNICA NUCLEAR POLYHEDROSIS VIRUS (ACMNPV).//P41479
F-NT2RM2001698//PENAEIDIN-3B PRECURSOR (P3-B).//0.36:52:34//PENAEUS VANNAMEI (PENOEID SHRIMP) (EUROPEAN WHITE SHRIMP).//P81059
F-NT2RM2001699//TRANSCRIPTION INITIATION FACTOR TFIID 30 KD SUBUNIT (TAFII-30) (TAFII30).//0.0012:79:40//HOMO SAPIENS (HUMAN).//Q12962
F-NT2RM2001700//ACYL-COA DEHYDROGENASE, VERY-LONG-CHAIN SPECIFIC (EC 1.3.99.-) (VLCAD) (FRAGMENT).//1.0e-30:140:53//MUS MUSCULUS (MOUSE).//P50544
F-NT2RM2001706//!!!! ALU SUBFAMILY SX WARNING ENTRY !!!!//1.5e-33:95:75//HOMO SAPIENS (HUMAN).//P39195
F-NT2RM2001716//HYPOTHETICAL 118.4 KD PROTEIN IN BAT2-DAL5 INTERGENIC REGION PRECURSOR.//0.010:116:30//SACCHAROMYCES CEREVISIAE (BAKER'S YEAST).//P47179
F-NT2RM2001718//METHYL-ACCEPTING CHEMOTAXIS PROTEIN TLPB.//0.00029:77:37//BACILLUS SUBTILIS.//P39217
F-NT2RM2001723//POSTERIOR PITUITARY PEPTIDE.//0.94:26:53//BOS TAURUS (BOVINE).//P01154
F-NT2RM2001727//E7 PROTEIN.//0.91:46:34//HUMAN PAPILLOMAVIRUS TYPE 23.//P50781
F-NT2RM2001730//PROBABLE UBIQUITIN CARBOXYL-TERMINAL HYDROLASE K02C4.3 (EC 3.1.2.15) (UBIQUITIN THIOLESTERASE) (UBIQUITIN-SPECIFIC PROCESSING PROTEASE) (DEUBIQUITINATING ENZYME).//4.9e-07:139:29//CAENORHABDITIS ELEGANS.//Q09931
F-NT2RM2001743//PROENKEPHALIN A PRECURSOR.//0.75:65:35//CAVIA PORCELLUS (GUINEA PIG).//P47969
F-NT2RM2001753//HYPOTHETICAL PROTEIN KIAA0210.//1.5e-14:119:36//HOMO SAPIENS (HUMAN).//Q92609
F-NT2RM2001760//PROTEIN TRANSPORT PROTEIN SEC61 ALPHA SUBUNIT.//8.3e-58:119:99//CANIS FAMILIARIS (DOG).//P38377
F-NT2RM2001768//HYPOTHETICAL PROTEIN UL25.//0.45:77:32//HUMAN CYTOMEGALOVIRUS (STRAIN AD169).//P16761
F-NT2RM2001771//ZINC FINGER PROTEIN 135.//4.6e-80:224:60//HOMO SAPIENS (HUMAN).//P52742
F-NT2RM2001782//MANNOSE-1-PHOSPHATE GUANYLTRANSFERASE (EC 2.7.7.13) (ATP-MANNOSE-1- PHOSPHATE GUANYLYLTRANSFERASE) (NDP-HEXOSE PYROPHOSPHORYLASE).//7.0e-06:61:45//SACCHAROMYCES CEREVISIAE (BAKER'S YEAST).//P41940
F-NT2RM2001784//HYPOTHETICAL PROTEIN UL61.//0.00070:145:33//HUMAN CYTOMEGALOVIRUS (STRAIN AD169).//P16818
F-NT2RM2001785//LINOLEOYL-COA DESATURASE (EC 1.14.99.25) (DELTA(6)-DESATURASE).//1.5e-08:127:32//SYNECHOCYSTIS SP. (STRAIN PCC 6803).//Q08871
F-NT2RM2001797//ZINC FINGER PROTEIN 135.//1.6e-73:267:49//HOMO SAPIENS (HUMAN).//P52742
F-NT2RM2001800//HYPOTHETICAL HELICASE MG018/MG017/MG016 HOMOLOG.//3.9e-12:171:33//MYCOPLASMA PNEUMONIAE.//P75093
F-NT2RM2001803//IKI3 PROTEIN.//1.6e-38:283:36//SACCHAROMYCES CEREVISIAE (BAKER'S YEAST).//Q06706
F-NT2RM2001805//COLD SHOCK-LIKE PROTEIN CSPH.//0.51:46:32//SALMONELLA TYPHIMURIUM.//O33793
F-NT2RM2001813//HYPOTHETICAL 40.4 KD TRP-ASP REPEATS CONTAINING PROTEIN C14B1.4 IN CHROMOSOME III.//5.0e-05:82:32//CAENORHABDITIS ELEGANS.//Q17963
F-NT2RM2001823//CHROMODOMAIN-HELICASE-DNA-BINDING PROTEIN 2 (CHD-2).//3.6e-49:233:45//HOMO SAPIENS (HUMAN).//O14647
F-NT2RM2001839//RETICULOCALBIN 1 PRECURSOR.//5.2e-65:222:56//HOMO SAPIENS (HUMAN).//Q15293
F-NT2RM2001840//!!!! ALU SUBFAMILY SQ WARNING ENTRY !!!!//9.6e-33:102:68//HOMO SAPIENS (HUMAN).//P39194
F-NT2RM2001855//BASP1 PROTEIN.//0.054:120:30//HOMO SAPIENS (HUMAN).//P80723
F-NT2RM2001867//HYPOTHETICAL 56.6 KD PROTEIN IN URE2-SSU72 INTERGENIC REGION.//4.1e-19:88:36//SACCHAROMYCES CEREVISIAE (BAKER'S YEAST).//P53867
F-NT2RM2001879//HYPOTHETICAL 47.3 KD PROTEIN C22G7.07C IN CHROMOSOME I.//5.9e-15:76:38//SCHIZOSACCHAROMYCES POMBE (FISSION YEAST).//Q09800
F-NT2RM2001886//HYPOTHETICAL 126.9 KD PROTEIN C22G7.04 IN CHROMOSOME I.//1.4e-41:249:38//SCHIZOSACCHAROMYCES POMBE (FISSION YEAST).//Q09798
F-NT2RM2001896//HYPOTHETICAL 83.2 KD PROTEIN IN KAR4-PBN1 INTERGENIC REGION.//2.1e-59:197:56//SACCHAROMYCES CEREVISIAE (BAKER'S YEAST).//P25582
F-NT2RM2001903//HYPOTHETICAL PROTEIN MJ0263.//0.070:132:31//METHANOCOCCUS JANNASCHII.//O06917
F-NT2RM2001930//THROMBOSPONDIN 2 PRECURSOR.//7.1e-05:53:47//MUS MUSCULUS (MOUSE).//Q03350
F-NT2RM2001935//PUTATIVE CUTICLE COLLAGEN F55C10.3.//0.00046:116:35//CAENORHABDITIS ELEGANS.//Q21184
F-NT2RM2001936//32.3 KD PROTEIN IN CWP1-MBR1 INTERGENIC REGION.//4.5e-27:216:34//SACCHAROMYCES CEREVISIAE (BAKER'S YEAST).//P28320
F-NT2RM2001950//HIRUDIN HV1 (BUFRUDIN).//0.59:43:34//HIRUDINARIA MANILLENSIS (BUFFALO LEECH).//P81492
F-NT2RM2001982//GUANINE NUCLEOTIDE-BINDING PROTEIN G(I)/G(S)/G(O) GAMMA-8 SUBUNIT (G GAMMA-C).//0.72:35:42//BOS TAURUS (BOVINE).//P50154
F-NT2RM2001983//PROLINE-RICH PEPTIDE P-B.//0.00035:23:52//HOMO SAPIENS (HUMAN).//P02814
F-NT2RM2001989//NUCLEOLAR PROTEIN NOP4 (NUCLEOLAR PROTEIN NOP77).//8.6e-24:197:37//SACCHAROMYCES CEREVISIAE (BAKER'S YEAST).//P37838
F-NT2RM2001997
F-NT2RM2001998//IMMEDIATE-EARLY PROTEIN IE180.//0.076:92:27//PSEUDORABIES VIRUS (STRAIN INDIANA-FUNKHAUSER / BECKER) (PRV).//P11675
F-NT2RM2002004//SLF1 PROTEIN.//3.5e-06:235:27//SACCHAROMYCES CEREVISIAE (BAKER'S YEAST).//Q12034
F-NT2RM2002014//HYPOTHETICAL PROTEIN HI0568.//2.1e-17:235:29//HAEMOPHILUS INFLUENZAE.//P71353
F-NT2RM2002030//GLUCOSAMINE-FRUCTOSE-6-PHOSPHATE AMINOTRANSFERASE [ISOMERIZING] (EC 2.6.1.16) (HEXOSEPHOSPHATE AMINOTRANSFERASE) (D-FRUCTOSE-6- PHOSPHATE AMIDOTRANSFERASE) (GFAT).//9.5e-105:271:76//MUS MUSCULUS (MOUSE).//P47856
F-NT2RM2002049//SMALL PROLINE-RICH PROTEIN 2-1.//0.099:41:41//HOMO SAPIENS (HUMAN).//P35326
F-NT2RM2002055//VACUOLAR PROTEIN SORTING-ASSOCIATED PROTEIN VPS13.//0.012:217:24//SACCHAROMYCES CEREVISIAE (BAKER'S YEAST).//Q07878
F-NT2RM2002088//PUTATIVE HETEROGENEOUS NUCLEAR RIBONUCLEOPROTEIN X (HNRNP X) (CBP).//1.1e-09:65:53//MUS MUSCULUS (MOUSE).//Q61990
F-NT2RM2002091//HYPOTHETICAL 57.5 KD PROTEIN IN VMA7-RPS25A INTERGENIC REGION.//0.072:74:37//SACCHAROMYCES CEREVISIAE (BAKER'S YEAST).//P53214
F-NT2RM2002100//ATP-DEPENDENT RNA HELICASE ROK1.//4.5e-50:289:41//SACCHAROMYCES CEREVISIAE (BAKER'S YEAST).//P45818
F-NT2RM2002109//NT-3 GROWTH FACTOR RECEPTOR PRECURSOR (EC 2.7.1.112) (TRKC TYROSINE KINASE) (GP145-TRKC) (TRK-C).//1.4e-14:203:32//RATTUS NORVEGICUS (RAT).//Q03351
F-NT2RM2002128//SKIN SECRETORY PROTEIN XP2 PRECURSOR (APEG PROTEIN).//0.0025:139:31//XENOPUS LAEVIS (AFRICAN CLAWED FROG).//P17437
F-NT2RM2002142//GASTRULATION SPECIFIC PROTEIN G12.//9.2e-20:42:73//BRACHYDANIO RERIO (ZEBRAFISH) (ZEBRA DANIO).//P47805
F-NT2RM2002145//GLUTENIN, HIGH MOLECULAR WEIGHT SUBUNIT 12 PRECURSOR.//0.0085:200:26//TRITICUM AESTIVUM (WHEAT).//P08488
F-NT2RM2002178//COLLAGEN ALPHA 1(XII) CHAIN (FRAGMENTS).//5.8e-05:56:39//BOS TAURUS (BOVINE).//P25508
F-NT2RM2002580//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//2.9e-14:96:37//PETROMYZON MARINUS (SEA LAMPREY).//P25210
F-NT2RM4000024//DNA-DIRECTED RNA POLYMERASE III 128 KD POLYPEPTIDE (EC 2.7.7.6) (RNA POLYMERASE III SUBUNIT 2).//8.6e-95:271:67//DROSOPHILA MELANOGASTER (FRUIT FLY).//P25167
F-NT2RM4000027//INTERFERON-ACTIVATABLE PROTEIN 202 (IFI-202).//0.99:72:31//MUS MUSCULUS (MOUSE).//P15091
F-NT2RM4000030//LAS1 PROTEIN.//1.4e-14:184:32//SACCHAROMYCES CEREVISIAE (BAKER'S YEAST).//P36146
F-NT2RM4000046//COLLAGEN ALPHA 1(III) CHAIN (FRAGMENT).//0.99:120:28//RATTUS NORVEGICUS (RAT).//P13941
F-NT2RM4000061
F-NT2RM4000085//ATP-DEPENDENT RNA HELICASE A (NUCLEAR DNA HELICASE II) (NDH II) (DEAD BOX PROTEIN 9) (MHEL-5).//8.5e-40:263:38//MUS MUSCULUS (MOUSE).//O70133
F-NT2RM4000086//HYPOTHETICAL PROTEIN HI1497.//1.0:27:37//HAEMOPHILUS INFLUENZAE.//P44221
F-NT2RM4000104//ZINC FINGER PROTEIN 134.//1.0e-26:64:56//HOMO SAPIENS (HUMAN).//P52741
F-NT2RM4000139//PREPROTEIN TRANSLOCASE SECE SUBUNIT.//0.99:38:42//THERMOTOGA MARITIMA.//P35874
F-NT2RM4000155//THREONYL-TRNA SYNTHETASE, CYTOPLASMIC (EC 6.1.1.3) (THREONINE--TRNA LIGASE) (THRRS).//6.3e-34:181:40//HOMO SAPIENS (HUMAN).//P26639
F-NT2RM4000156//EXTENSIN PRECURSOR (CELL WALL HYDROXYPROLINE-RICH GLYCOPROTEIN).//4.6e-12:142:33//NICOTIANA TABACUM (COMMON TOBACCO).//P13983
F-NT2RM4000167//KINESIN-LIKE PROTEIN KIF4.//3.4e-123:269:91//MUS MUSCULUS (MOUSE).//P33174
F-NT2RM4000169//M PROTEIN, SEROTYPE 2.2 PRECURSOR.//9.7e-10:229:26//STREPTOCOCCUS PYOGENES.//P50469
F-NT2RM4000191//P68-LIKE PROTEIN.//2.1e-11:104:40//SACCHAROMYCES CEREVISIAE (BAKER'S YEAST).//P24783
F-NT2RM4000197//CUTICLE PROTEIN CP463 (CPCP463).//0.84:29:37//CANCER PAGURUS (ROCK CRAB).//P81587
F-NT2RM4000199//SALIVARY PROLINE-RICH PROTEIN PO (ALLELE K) [CONTAINS: PEPTIDE P-D] (FRAGMENT).//1.8e-06:187:34//HOMO SAPIENS (HUMAN).//P10162
F-NT2RM4000200//HYPOTHETICAL 9.4 KD PROTEIN IN FLAL 3'REGION (ORF3).//0.52:42:40//BACILLUS LICHENIFORMIS.//P22754
F-NT2RM4000202//COLLAGEN ALPHA 1(VIII) CHAIN PRECURSOR (ENDOTHELIAL COLLAGEN).//0.00044:168:32//ORYCTOLAGUS CUNICULUS (RABBIT).//P14282
F-NT2RM4000210//EXTENSIN PRECURSOR.//0.27:129:27//DAUCUS CAROTA (CARROT).//P06599
F-NT2RM4000215//MAK16 PROTEIN.//2.0e-65:234:52//SACCHAROMYCES CEREVISIAE (BAKER'S YEAST).//P10962
F-NT2RM4000229//GAR2 PROTEIN.//0.13:217:26//SCHIZOSACCHAROMYCES POMBE (FISSION YEAST).//P41891
F-NT2RM4000233//TRANSMEMBRANE PROTEIN SEX PRECURSOR.//0.047:108:30//HOMO SAPIENS (HUMAN).//P51805
F-NT2RM4000244//ATP SYNTHASE PROTEIN 8 (EC 3.6.1.34) (A6L).//0.67:59:27//BALAENOPTERA PHYSALUS (FINBACK WHALE) (COMMON RORQUAL).//P24947
F-NT2RM4000251//PROLINE-RICH PROTEIN MP-3 (FRAGMENT).//0.0059:108:35//MUS MUSCULUS (MOUSE).//P05143
F-NT2RM4000265//!!!! ALU SUBFAMILY J WARNING ENTRY !!!!//8.1e-38:70:70//HOMO SAPIENS (HUMAN).//P39188
F-NT2RM4000290//TRANSDUCIN-LIKE ENHANCER PROTEIN 3 (ESG3).//1.6e-115:209:94//HOMO SAPIENS (HUMAN).//Q04726
F-NT2RM4000324//PRESPORE PROTEIN DP87 PRECURSOR.//0.14:136:30//DICTYOSTELIUM DISCOIDEUM (SLIME MOLD).//Q04503
F-NT2RM4000327//HYPOTHETICAL 8.9 KD PROTEIN IN IE0-IE1 INTERGENIC REGION.//0.91:73:28//AUTOGRAPHA CALIFORNICA NUCLEAR POLYHEDROSIS VIRUS (ACMNPV).//P41703
F-NT2RM4000344//YME1 PROTEIN HOMOLOG (EC 3.4.24.-).//9.4e-78:241:55//CAENORHABDITIS ELEGANS.//P54813
F-NT2RM4000349//CYSTEINE STRING PROTEIN (CCCS1).//0.055:22:59//TORPEDO CALIFORNICA (PACIFIC ELECTRIC RAY).//P56101
F-NT2RM4000354//LETHAL(2)DENTICLELESS PROTEIN (DTL83 PROTEIN).//4.6e-26:208:35//DROSOPHILA MELANOGASTER (FRUIT FLY).//Q24371
F-NT2RM4000356//COAT PROTEIN.//0.11:105:36//SATELLITE TOBACCO MOSAIC VIRUS (STMV).//P17574
F-NT2RM4000366//IMMEDIATE-EARLY PROTEIN.//1.2e-05:215:24//HERPES VIRUS SAIMIRI (STRAIN 11).//Q01042
F-NT2RM4000368//HYPOTHETICAL 7.3 KD PROTEIN IN RPBA-GP46 INTERGENIC REGION.//0.54:46:36//BACTERIOPHAGE RB69.//O64300
F-NT2RM4000386//RHSC PROTEIN PRECURSOR.//0.0096:162:29//ESCHERICHIA COLI.//P16918
F-NT2RM4000395//HYPOTHETICAL 52.9 KD PROTEIN IN SAP155-YMR31 INTERGENIC REGION.//4.5e-66:256:53//SACCHAROMYCES CEREVISIAE (BAKER'S YEAST).//P43616
F-NT2RM4000414//HYPOTHETICAL 6.0 KD PROTEIN IN THI12 5'REGION.//0.13:33:48//SACCHAROMYCES CEREVISIAE (BAKER'S YEAST).//P53820
F-NT2RM4000421//MRNA TRANSPORT REGULATOR MTR10.//5.0e-13:171:29//SACCHAROMYCES CEREVISIAE (BAKER'S YEAST).//Q99189
F-NT2RM4000425//!!!! ALU SUBFAMILY SP WARNING ENTRY !!!!//2.1e-25:46:80//HOMO SAPIENS (HUMAN).//P39193
F-NT2RM4000433//CUTICLE COLLAGEN 3A3.//2.5e-06:77:38//HAEMONCHUS CONTORTUS.//P16253
F-NT2RM4000457//HYPOTHETICAL 111.9 KD PROTEIN C22H10.03C IN CHROMOSOME I.//4.3e-09:215:22//SCHIZOSACCHAROMYCES POMBE (FISSION YEAST).//Q10297
F-NT2RM4000471//TRNA SPLICING PROTEIN SPL1.//6.7e-73:163:65//CANDIDA ALBICANS (YEAST).//P87185
F-NT2RM4000486//COLLAGEN ALPHA 2(VI) CHAIN PRECURSOR.//0.0012:121:34//GALLUS GALLUS (CHICKEN).//P15988
F-NT2RM4000496//DNA-DIRECTED RNA POLYMERASE II LARGEST SUBUNIT (EC 2.7.7.6) (RNA POLYMERASE II SUBUNIT 1).//5.9e-09:175:35//SCHIZOSACCHAROMYCES POMBE (FISSION YEAST).//P36594
F-NT2RM4000511//SALIVARY GLUE PROTEIN SGS-3 PRECURSOR.//0.020:122:31//DROSOPHILA SIMULANS (FRUIT FLY).//P13729
F-NT2RM4000514//ATP SYNTHASE A CHAIN (EC 3.6.1.34) (PROTEIN 6).//0.46:68:32//ARTEMIA SANFRANCISCANA (BRINE SHRIMP) (ARTEMIA FRANCISCANA).//Q37708
F-NT2RM4000515//GAR2 PROTEIN.//3.2e-05:198:27//SCHIZOSACCHAROMYCES POMBE (FISSION YEAST).//P41891
F-NT2RM4000520//HYPOTHETICAL 7.5 KD PROTEIN (ORF 63).//0.011:55:38//SPINACIA OLERACEA (SPINACH).//P08974
F-NT2RM4000531//ZINC FINGER PROTEIN 169 (FRAGMENT).//3.6e-44:244:42//HOMO SAPIENS (HUMAN).//Q14929
F-NT2RM4000532//PUTATIVE MEMBRANE PROTEIN 53.//1.0:47:34//HERPES VIRUS SAIMIRI (STRAIN 11).//Q01049
F-NT2RM4000534//HYPOTHETICAL 5.9 KD PROTEIN IN WRBA-PUTA INTERGENIC REGION.//0.75:26:46//ESCHERICHIA COLI.//P56614
F-NT2RM4000585//GAG POLYPROTEIN [CONTAINS: CORE PROTEIN P16; CORE PROTEIN P26].//0.019:86:34//HUMAN IMMUNODEFICIENCY VIRUS TYPE 2 (ISOLATE SBLIS Y) (HIV-2).//P12450
F-NT2RM4000590//RING CANAL PROTEIN (KELCH PROTEIN).//5.0e-23:224:29//DROSOPHILA MELANOGASTER (FRUIT FLY).//Q04652
F-NT2RM4000595//HYPOTHETICAL 54.9 KD PROTEIN C02F5.7 IN CHROMOSOME III.//3.8e-62:226:50//CAENORHABDITIS ELEGANS.//P34284
F-NT2RM4000603//SRC SUBSTRATE CORTACTIN (AMPLAXIN) (EMS1 ONCOGENE).//0.077:132:22//HOMO SAPIENS (HUMAN).//Q14247
F-NT2RM4000611//HYPOTHETICAL 40.4 KD TRP-ASP REPEATS CONTAINING PROTEIN C14B1.4 IN CHROMOSOME III.//1.9e-06:82:32//CAENORHABDITIS ELEGANS.//Q17963
F-NT2RM4000616//ACETYL-COENZYME A SYNTHETASE (EC 6.2.1.1) (ACETATE--COA LIGASE) (ACYL- ACTIVATING ENZYME).//5.3e-79:213:62//ESCHERICHIA COLI.//P27550
F-NT2RM4000674//HYPOTHETICAL SYMPORTER SLL1374.//1.3e-11:147:32//SYNECHOCYSTIS SP. (STRAIN PCC 6803).//P74168
F-NT2RM4000689
F-NT2RM4000698//CHORION CLASS HIGH-CYSTEINE HCA PROTEIN 12 PRECURSOR (HC-A.12).//0.26:45:33//BOMBYX MORI (SILK MOTH).//P05687
F-NT2RM4000700//THIOPHENE AND FURAN OXIDATION PROTEIN THDF.//0.95:165:25//BORRELIA BURGDORFERI (LYME DISEASE SPIROCHETE).//P53364
F-NT2RM4000712//PROBABLE UBIQUITIN CARBOXYL-TERMINAL HYDROLASE R10E11.3 (EC 3.1.2.15) (UBIQUITIN THIOLESTERASE) (UBIQUITIN-SPECIFIC PROCESSING PROTEASE) (DEUBIQUITINATING ENZYME).//2.2e-82:152:63//CAENORHABDITIS ELEGANS.//P34547
F-NT2RM4000717//SALIVARY GLUE PROTEIN SGS-3 PRECURSOR.//0.80:54:40//DROSOPHILA SIMULANS (FRUIT FLY).//P13729
F-NT2RM4000733//OCTAPEPTIDE-REPEAT PROTEIN T2.//1.5e-08:139:28//MUS MUSCULUS (MOUSE).//Q06666
F-NT2RM4000734//GASTRULA ZINC FINGER PROTEIN XLCGF26.1 (FRAGMENT).//7.2e-20:205:28//XENOPUS LAEVIS (AFRICAN CLAWED FROG).//P18715
F-NT2RM4000741//SPERM PROTAMINE P1.//0.89:52:38//ISOODON MACROURUS (SHORT-NOSED BANDICOOT).//P42136
F-NT2RM4000751//ZINC FINGER PROTEIN 26 (ZFP-26) (MKR3 PROTEIN) (FRAGMENT).//5.2e-77:246:52//MUS MUSCULUS (MOUSE).//P10076
F-NT2RM4000764//KERATIN, GLYCINE/TYROSINE-RICH OF HAIR.//0.062:33:42//OVIS ARIES (SHEEP).//Q02958
F-NT2RM4000778
F-NT2RM4000779//SULFATED SURFACE GLYCOPROTEIN 185 (SSG 185).//0.014:53:45//VOLVOX CARTERI.//P21997
F-NT2RM4000787//BONE MORPHOGENETIC PROTEIN 1 PRECURSOR (EC 3.4.24.-) (BMP-1).//0.00011:73:39//MUS MUSCULUS (MOUSE).//P98063
F-NT2RM4000790//SPORE COAT PROTEIN SP96.//0.00083:157:29//DICTYOSTELIUM DISCOIDEUM (SLIME MOLD).//P14328
F-NT2RM4000795//CHOLINESTERASE PRECURSOR (EC 3.1.1.8) (ACYLCHOLINE ACYLHYDROLASE) (CHOLINE ESTERASE II) (BUTYRYLCHOLINE ESTERASE) (PSEUDOCHOLINESTERASE).//7.4e-41:271:36//HOMO SAPIENS (HUMAN).//P06276
F-NT2RM4000796//5-METHYLCYTOSINE-SPECIFIC RESTRICTION ENZYME B (EC 3.1.21.-).//0.28:82:30//ESCHERICHIA COLI.//P15005
F-NT2RM4000798//PROTEIN TRANSPORT PROTEIN SEC7.//4.7e-38:165:48//SACCHAROMYCES CEREVISIAE (BAKER'S YEAST).//P11075
F-NT2RM4000813//METALLOTHIONEIN-IB.//0.0025:25:44//OVIS ARIES (SHEEP).//P09577
F-NT2RM4000820
F-NT2RM4000833//HYPOTHETICAL PROTEIN MJ1136.//6.5e-42:206:41//METHANOCOCCUS JANNASCHII.//Q58536
F-NT2RM4000848//BRAIN-SPECIFIC HOMEOBOX/POU DOMAIN PROTEIN 3A (BRN-3A) (BRN-3.0).//0.00060:159:33//MUS MUSCULUS (MOUSE).//P17208
F-NT2RM4000852//SMALL PROLINE-RICH PROTEIN 2B (SPR-2B).//0.0076:13:69//HOMO SAPIENS (HUMAN).//P35325
F-NT2RM4000855//!!!! ALU SUBFAMILY SQ WARNING ENTRY !!!!//0.0060:68:44//HOMO SAPIENS (HUMAN).//P39194
F-NT2RM4000887//RTS1 PROTEIN (SCS1 PROTEIN).//0.23:153:24//SACCHAROMYCES CEREVISIAE (BAKER'S YEAST).//P38903
F-NT2RM4000895//HYPOTHETICAL 53.5 KD PROTEIN IN PHO2-POL3 INTERGENIC REGION.//3.3e-09:80:46//SACCHAROMYCES CEREVISIAE (BAKER'S YEAST).//P43123
F-NT2RM4000950//HYPOTHETICAL PROTEIN MJ0572.//0.090:68:29//METHANOCOCCUS JANNASCHII.//Q57992
F-NT2RM4000971//KINESIN LIGHT CHAIN (KLC).//0.79:201:24//LOLIGO PEALEII (LONGFIN SQUID).//P46825
F-NT2RM4000979//MYOSIN REGULATORY LIGHT CHAIN 2, NONSARCOMERIC (MYOSIN RLC).//1.2e-07:25:96//HOMO SAPIENS (HUMAN).//P19105
F-NT2RM4000996//ZINC FINGER PROTEIN 37 (ZFP-37) (MALE GERM CELL SPECIFIC ZINC FINGER PROTEIN).//1.4e-56:253:46//MUS MUSCULUS (MOUSE).//P17141
F-NT2RM4001002
F-NT2RM4001016//GAG POLYPROTEIN [CONTAINS: CORE PROTEIN P15; INNER COAT PROTEIN P12; CORE SHELL PROTEIN P30].//0.25:101:31//FBR MURINE OSTEOSARCOMA VIRUS.//P29175
F-NT2RM4001032//CUTICLE COLLAGEN 2.//2.6e-07:130:39//CAENORHABDITIS ELEGANS.//P17656
F-NT2RM4001047//MO25 PROTEIN.//5.6e-107:252:80//MUS MUSCULUS (MOUSE).//Q06138
F-NT2RM4001054//PROTEIN TRANSPORT PROTEIN SEC61 ALPHA SUBUNIT.//9.0e-109:209:94//CANIS FAMILIARIS (DOG).//P38377
F-NT2RM4001084//HYPOTHETICAL TRANSCRIPTIONAL REGULATOR IN UXUR-IADA INTERGENIC REGION.//0.57:95:30//ESCHERICHIA COLI.//P39376
F-NT2RM4001092//HYPOTHETICAL 127.4 KD PROTEIN F07F6.4 IN CHROMOSOME III.//2.5e-47:231:47//CAENORHABDITIS ELEGANS.//Q09531
F-NT2RM4001116//HYPOTHETICAL 216.3 KD PROTEIN R06F6.8 IN CHROMOSOME II.//1.3e-08:243:23//CAENORHABDITIS ELEGANS.//Q09417
F-NT2RM4001140//HOMEOBOX PROTEIN MSH-D.//7.1e-13:103:38//BRACHYDANIO RERIO (ZEBRAFISH) (ZEBRA DANIO).//Q01704
F-NT2RM4001151//SYNAPSINS IA AND IB (BRAIN PROTEIN 4.1).//0.26:96:34//HOMO SAPIENS (HUMAN).//P17600
F-NT2RM4001155//ADRENAL MEDULLA 50 KD PROTEIN.//3.6e-103:201:91//BOS TAURUS (BOVINE).//Q27969
F-NT2RM4001160//GLUTATHIONE S-TRANSFERASE (EC 2.5.1.18) (CLASS-PHI) (FRAGMENTS).//1.0:33:36//BRASSICA OLERACEA (CAULIFLOWER).//P48438 F-NT2RM4001187//PREPROTEIN TRANSLOCASE SECA SUBUNIT.//0.44:158:27//MYCOPLASMA GENITALIUM.//P47318
F-NT2RM4001191//LONG NEUROTOXIN 2 (TOXIN C).//0.99:44:43//ASTROTIA STOKESI (STOKES'S SEA SNAKE) (DISTEIRA STOKESI).//P01381
F-NT2RM4001200//ZINC FINGER PROTEIN 135.//2.2e-82:245:59//HOMO SAPIENS (HUMAN).//P52742
F-NT2RM4001203//HYPOTHETICAL 57.5 KD PROTEIN IN VMA7-RPS25A INTERGENIC REGION.//0.028:94:40//SACCHAROMYCES CEREVISIAE (BAKER'S YEAST).//P53214
F-NT2RM4001204//SPLICEOSOME ASSOCIATED PROTEIN 62 (SAP 62) (SF3A66).//0.0096:182:34//HOMO SAPIENS (HUMAN).//Q15428
F-NT2RM4001217//RING CANAL PROTEIN (KELCH PROTEIN).//2.1e-21:221:29//DROSOPHILA MELANOGASTER (FRUIT FLY).//Q04652
F-NT2RM4001256//CBP3 PROTEIN PRECURSOR.//0.30:55:32//SACCHAROMYCES CEREVISIAE (BAKER'S YEAST).//P21560
F-NT2RM4001258//HYPOTHETICAL 35.5 KD PROTEIN IN TRANSPOSON TN4556.//0.00031:132:39//STREPTOMYCES FRADIAE.//P20186
F-NT2RM4001309//SALIVARY PROLINE-RICH PROTEIN PRECURSOR (CLONE CP7) [CONTAINS: BASIC PEPTIDE P-F] (FRAGMENT).//0.048:132:28//HOMO SAPIENS (HUMAN).//P02812
F-NT2RM4001313//PHOSPHATIDYLINOSITOL 3-KINASE VPS34-LIKE (EC 2.7.1.137) (PI3-KINASE) (PTDINS-3-KINASE) (PI3K).//2.6e-37:124:65//DICTYOSTELIUM DISCOIDEUM (SLIME MOLD).//P54676
F-NT2RM4001316//ACYL-COA DEHYDROGENASE, MEDIUM-CHAIN SPECIFIC PRECURSOR (EC 1.3.99.3) (MCAD).//1.7e-10:185:30//RATTUS NORVEGICUS (RAT).//P08503
F-NT2RM4001320//PUTATIVE RHO/RAC GUANINE NUCLEOTIDE EXCHANGE FACTOR (RHO/RAC GEF) (FACIOGENITAL DYSPLASIA PROTEIN HOMOLOG).//1.5e-08:197:26//MUS MUSCULUS (MOUSE).//P52734
F-NT2RM4001340//UTR4 PROTEIN (UNKNOWN TRANSCRIPT 4 PROTEIN).//7.7e-14:82:36//SACCHAROMYCES CEREVISIAE (BAKER'S YEAST).//P32626
F-NT2RM4001344//HYPOTHETICAL GTP-BINDING PROTEIN IN POP2-HOL1 INTERGENIC REGION.//3.3e-16:128:35//SACCHAROMYCES CEREVISIAE (BAKER'S YEAST).//P53742
F-NT2RM4001347//HYPOTHETICAL 76.9 KD PROTEIN IN RPM2-TUB1 INTERGENIC REGION.//0.067:111:33//SACCHAROMYCES CEREVISIAE (BAKER'S YEAST).//Q04511
F-NT2RM4001371
F-NT2RM4001382//HISTIDINE-RICH GLYCOPROTEIN PRECURSOR.//1.0e-08:82:39//PLASMODIUM LOPHURAE.//P04929
F-NT2RM4001384
F-NT2RM4001410//A-AGGLUTININ ATTACHMENT SUBUNIT PRECURSOR.//2.1e-08:185:31//SACCHAROMYCES CEREVISIAE (BAKER'S YEAST).//P32323
F-NT2RM4001411//EARLY NODULIN 20 PRECURSOR (N-20).//5.3e-05:105:38//MEDICAGO TRUNCATULA (BARREL MEDIC).//P93329
F-NT2RM4001412//GTPASE-ACTIVATING PROTEIN (GAP) (RAS P21 PROTEIN ACTIVATOR) (P120GAP) (RASGAP).//6.2e-17:109:41//RATTUS NORVEGICUS (RAT).//P50904
F-NT2RM4001414//ZINC FINGER PROTEIN 177.//8.3e-06:54:50//HOMO SAPIENS (HUMAN).//Q13360
F-NT2RM4001437//!!!! ALU SUBFAMILY SC WARNING ENTRY !!!!//2.1e-24:87:65//HOMO SAPIENS (HUMAN).//P39192
F-NT2RM4001444//PROBABLE ISOLEUCYL-TRNA SYNTHETASE (EC 6.1.1.5) (ISOLEUCINE--TRNA LIGASE) (ILERS) (FRAGMENT).//2.6e-45:197:47//CIONA INTESTINALIS.//Q94425
F-NT2RM4001454//HYPOTHETICAL PROTEIN KIAA0041 (FRAGMENT).//0.0060:95:29//HOMO SAPIENS (HUMAN).//Q15057
F-NT2RM4001455//PROBABLE E5B PROTEIN.//0.41:44:36//HUMAN PAPILLOMAVIRUS TYPE 6B.//P06461
F-NT2RM4001483//ZINC FINGER PROTEIN 136.//1.7e-28:85:64//HOMO SAPIENS (HUMAN).//P52737
F-NT2RM4001489//PTB-ASSOCIATED SPLICING FACTOR (PSF).//0.086:111:34//HOMO SAPIENS (HUMAN).//P23246
F-NT2RM4001519//ACID UREASE ALPHA SUBUNIT (EC 3.5.1.5) (UREA AMIDOHYDROLASE).//0.82:51:47//LACTOBACILLUS FERMENTUM.//P26929
F-NT2RM4001522//TROPOMYOSIN.//0.030:117:23//SCHIZOSACCHAROMYCES POMBE (FISSION YEAST).//Q02088
F-NT2RM4001557
F-NT2RM4001565//HYPOTHETICAL 44.3 KD PROTEIN C1F7.07C IN CHROMOSOME I.//0.99:42:40//SCHIZOSACCHAROMYCES POMBE (FISSION YEAST).//Q09919
F-NT2RM4001566//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//0.054:190:23//SACCHAROMYCES CEREVISIAE (BAKER'S YEAST).//P08640
F-NT2RM4001569//GUANINE NUCLEOTIDE-BINDING PROTEIN BETA SUBUNIT-LIKE PROTEIN (ACTIVATED PROTEIN KINASE C RECEPTOR HOMOLOG).//0.72:64:31//TRYPANOSOMA BRUCEI BRUCEI.//Q94775
F-NT2RM4001582
F-NT2RM4001592//DNA REPAIR PROTEIN RAD9.//0.00037:198:31//SACCHAROMYCES CEREVISIAE (BAKER'S YEAST).//P14737
F-NT2RM4001594//IMMEDIATE-EARLY PROTEIN IE180.//1.9e-05:147:34//PSEUDORABIES VIRUS (STRAIN KAPLAN) (PRV).//P33479
F-NT2RM4001597//THIOL:DISULFIDE INTERCHANGE PROTEIN TLPA (CYTOCHROME C BIOGENESIS PROTEIN TLPA).//5.7e-06:122:29//BRADYRHIZOBIUM JAPONICUM.//P43221
F-NT2RM4001605//NUCLEAR PORE COMPLEX PROTEIN NUP155 (NUCLEOPORIN NUP155) (155 KD NUCLEOPORIN) (P140).//1.7e-128:249:96//RATTUS NORVEGICUS (RAT).//P37199
F-NT2RM4001611//SIS2 PROTEIN (HALOTOLERANCE PROTEIN HAL3).//1.5e-35:128:47//SACCHAROMYCES CEREVISIAE (BAKER'S YEAST).//P36024
F-NT2RM4001629//MAGUK P55 SUBFAMILY MEMBER 3 (MPP3 PROTEIN) (DISCS, LARGE HOMOLOG 3).//5.8e-42:254:37//HOMO SAPIENS (HUMAN).//Q13368
F-NT2RM4001650//HOMEOBOX PROTEIN HOX-A4 (CHOX-1.4).//0.62:19:57//GALLUS GALLUS (CHICKEN).//P17277
F-NT2RM4001662//PROTEIN KINASE C, ALPHA TYPE (EC 2.7.1.-) (PKC-ALPHA).//0.29:90:32//HOMO SAPIENS (HUMAN).//P17252
F-NT2RM4001666//HYPOTHETICAL 48.6 KD PROTEIN IN ALPA-GABP INTERGENIC REGION.//1.1e-31:137:44//ESCHERICHIA COLI.//P37339
F-NT2RM4001682//PROBABLE 60S RIBOSOMAL PROTEIN L22.//0.98:55:29//CAENORHABDITIS ELEGANS.//P52819
F-NT2RM4001710//HYPOTHETICAL PROTEIN KIAA0039 (FRAGMENT).//0.56:113:28//HOMO SAPIENS (HUMAN).//Q15054
F-NT2RM4001714//SEPTIN 2 HOMOLOG (FRAGMENT).//1.4e-108:255:77//HOMO SAPIENS (HUMAN).//Q14141
F-NT2RM4001715//HYPOTHETICAL PROTEIN C19G10.16 IN CHROMOSOME I (FRAGMENT).//2.1e-36:148:38//SCHIZOSACCHAROMYCES POMBE (FISSION YEAST).//Q10342
F-NT2RM4001731//HYPOTHETICAL 54.9 KD PROTEIN C02F5.7 IN CHROMOSOME III.//1.1e-05:90:33//CAENORHABDITIS ELEGANS.//P34284
F-NT2RM4001741//TALIN.//1.1e-106:208:99//MUS MUSCULUS (MOUSE).//P26039
F-NT2RM4001746//EBNA-1 NUCLEAR PROTEIN.//1.6e-09:155:38//EPSTEIN-BARR VIRUS (STRAIN B95-8) (HUMAN HERPESVIRUS 4).//P03211
F-NT2RM4001754//COLLAGEN ALPHA 5(IV) CHAIN PRECURSOR.//0.93:158:33//HOMO SAPIENS (HUMAN).//P29400
F-NT2RM4001758//PUTATIVE SERINE/THREONINE-PROTEIN KINASE P78 (EC 2.7.1.-).//5.1e-113:277:79//HOMO SAPIENS (HUMAN).//P27448
F-NT2RM4001776//MYOSIN I ALPHA (MMI-ALPHA).//2.2e-73:262:54//MUS MUSCULUS (MOUSE).//P46735
F-NT2RM4001783//ZINC FINGER PROTEIN HRX (ALL-1) (FRAGMENT).//5.3e-26:169:39//MUS MUSCULUS (MOUSE).//P55200
F-NT2RM4001810//MALE SPECIFIC SPERM PROTEIN MST84DB.//2.3e-05:68:42//DROSOPHILA MELANOGASTER (FRUIT FLY).//Q01643
F-NT2RM4001813//RHODOCETIN ALPHA SUBUNIT.//2.3e-05:115:34//AGKISTRODON RHODOSTOMA (MALAYAN PIT VIPER) (CALLOSELASMA RHODOSTOMA).//P81397
F-NT2RM4001819//CELL SURFACE GLYCOPROTEIN EMR1 PRECURSOR (EMR1 HORMONE RECEPTOR) (CELL SURFACE GLYCOPROTEIN F4/80).//1.7e-06:159:25//MUS MUSCULUS (MOUSE).//Q61549
F-NT2RM4001823//ZINC FINGER PROTEIN ZIC1 (ZINC FINGER PROTEIN OF THE CEREBELLUM 1).//2.6e-18:114:40//MUS MUSCULUS (MOUSE).//P46684
F-NT2RM4001828//ZINC FINGER PROTEIN 84 (ZINC FINGER PROTEIN HPF2).//4.0e-81:253:59//HOMO SAPIENS (HUMAN).//P51523
F-NT2RM4001836//SPIDROIN 2 (DRAGLINE SILK FIBROIN 2) (FRAGMENT).//0.21:176:30//NEPHILA CLAVIPES (ORB SPIDER).//P46804
F-NT2RM4001841//PROLINE-RICH PEPTIDE P-B.//0.046:27:40//HOMO SAPIENS (HUMAN).//P02814
F-NT2RM4001842//HYPOTHETICAL 7.0 KD PROTEIN B03B8.1 IN CHROMOSOME III.//0.98:35:42//CAENORHABDITIS ELEGANS.//Q11104
F-NT2RM4001856//HYPOTHETICAL 75.2 KD PROTEIN IN ACS1-GCV3 INTERGENIC REGION.//2.3e-37:242:37//SACCHAROMYCES CEREVISIAE (BAKER'S YEAST).//P39722
F-NT2RM4001858//T-BOX PROTEIN VEGT (T-BOX PROTEIN BRAT) (T-BOX PROTEIN ANTIPODEAN).//1.8e-23:78:64//XENOPUS LAEVIS (AFRICAN CLAWED FROG).//P87377
F-NT2RM4001865//NEURONAL CALCIUM SENSOR 2 (NCS-2).//0.012:83:28//CAENORHABDITIS ELEGANS.//P36609
F-NT2RM4001876//HYPOTHETICAL 118.4 KD PROTEIN IN BAT2-DAL5 INTERGENIC REGION PRECURSOR.//3.8e-10:242:32//SACCHAROMYCES CEREVISIAE (BAKER'S YEAST).//P47179
F-NT2RM4001880//EC PROTEIN HOMOLOG.//0.22:59:32//ARABIDOPSIS THALIANA (MOUSE-EAR CRESS).//P93746
F-NT2RM4001905//60S RIBOSOMAL PROTEIN L40 (CEP52).//0.57:20:60//HOMO SAPIENS (HUMAN), RATTUS NORVEGICUS (RAT), AND GALLUS GALLUS (CHICKEN).//P14793
F-NT2RM4001922
F-NT2RM4001930//PUTATIVE GLUCOSYLTRANSFERASE C08B11.8 (EC 2.4.1.-).//5.5e-45:167:53//CAENORHABDITIS ELEGANS.//Q09226
F-NT2RM4001938//RTOA PROTEIN (RATIO-A).//0.0036:120:32//DICTYOSTELIUM DISCOIDEUM (SLIME MOLD).//P54681
F-NT2RM4001940//IROQUOIS-CLASS HOMEODOMAIN PROTEIN IRX-1 (FRAGMENT).//0.32:31:48//HOMO SAPIENS (HUMAN).//P78415
F-NT2RM4001953//!!!! ALU SUBFAMILY SC WARNING ENTRY !!!!//2.2e-43:56:85//HOMO SAPIENS (HUMAN).//P39192
F-NT2RM4001965//IG ALPHA-1 CHAIN C REGION.//0.56:73:34//GORILLA GORILLA GORILLA (LOWLAND GORILLA).//P20758
F-NT2RM4001969//SALIVARY PROLINE-RICH PROTEIN PRECURSOR (CLONES CP3, CP4 AND CP5) [CONTAINS: BASIC PEPTIDE IB-6; PEPTIDE P-H].//0.0016:140:27//HOMO SAPIENS (HUMAN).//P04280
F-NT2RM4001979//ZINC FINGER PROTEIN 84 (ZINC FINGER PROTEIN HPF2).//3.9e-21:103:51//HOMO SAPIENS (HUMAN).//P51523
F-NT2RM4001984//HYPOTHETICAL PROTEIN LAMBDA-SP5.//0.0034:50:40//MUS MUSCULUS (MOUSE).//P15974
F-NT2RM4001987//IRREGULAR CHIASM C-ROUGHEST PROTEIN PRECURSOR (IRREC PROTEIN).//6.9e-17:115:31//DROSOPHILA MELANOGASTER (FRUIT FLY).//Q08180
F-NT2RM4002013//HYPOTHETICAL 54.5 KD TRP-ASP REPEATS CONTAINING PROTEIN ZC302.2 IN CHROMOSOME V.//0.0062:117:28//CAENORHABDITIS ELEGANS.//Q23256
F-NT2RM4002018//SPORE COAT PROTEIN SP96.//4.3e-06:203:28//DICTYOSTELIUM DISCOIDEUM (SLIME MOLD).//P14328
F-NT2RM4002034//RHO-GAP HEMATOPOIETIC PROTEIN C1 (P115) (KIAA0131).//0.78:132:25//HOMO SAPIENS (HUMAN).//P98171
F-NT2RM4002044//VITELLOGENIN I PRECURSOR (MINOR VITELLOGENIN) [CONTAINS: LIPOVITELLIN I (LVI); PHOSVITIN (PV); LIPOVITELLIN II (LVII); YGP42].//0.062:201:24//GALLUS GALLUS (CHICKEN).//P87498
F-NT2RM4002054//DUPLICATE PROCYCLIN.//0.0079:44:52//TRYPANOSOMA BRUCEI BRUCEI.//P14044
F-NT2RM4002055//PUTATIVE Z PROTEIN.//0.82:39:30//OVIS ARIES (SHEEP).//P08105
F-NT2RM4002062//ASPARTYL-TRNA SYNTHETASE (EC 6.1.1.12) (ASPARTATE--TRNA LIGASE) (ASPRS).//7.0e-37:80:52//THERMUS AQUATICUS (SUBSP. THERMOPHILUS).//P36419
F-NT2RM4002063//SARCOSINE OXIDASE (EC 1.5.3.1).//2.2e-25:216:31//BACILLUS SP. (STRAIN NS-129).//P23342
F-NT2RM4002066//HYPOTHETICAL PROTEIN KIAA0192 (FRAGMENT).//1.1e-94:260:71//HOMO SAPIENS (HUMAN).//Q93074
F-NT2RM4002067//!!!! ALU SUBFAMILY J WARNING ENTRY !!!!//1.5e-15:51:70//HOMO SAPIENS (HUMAN).//P39188
F-NT2RM4002073//ELASTIN PRECURSOR (TROPOELASTIN).//4.9e-05:88:36//HOMO SAPIENS (HUMAN).//P15502
F-NT2RM4002075//RING CANAL PROTEIN (KELCH PROTEIN).//7.2e-43:220:41//DROSOPHILA MELANOGASTER (FRUIT FLY).//Q04652
F-NT2RM4002093//POLYPYRIMIDINE TRACT-BINDING PROTEIN (PTB) (HETEROGENEOUS NUCLEAR RIBONUCLEOPROTEIN I) (HNRNP I) (57 KD RNA-BINDING PROTEIN PPTB-1).//1.8e-93:255:72//HOMO SAPIENS (HUMAN).//P26599
F-NT2RM4002109//KINESIN-LIKE PROTEIN KIF4.//3.7e-101:260:78//MUS MUSCULUS (MOUSE).//P33174
F-NT2RM4002128//HYPOTHETICAL PROTEIN IN CYCB 3'REGION PRECURSOR (ORF2) (FRAGMENT).//0.91:49:32//PARACOCCUS DENITRIFICANS.//P29969
F-NT2RM4002140//GROUCHO PROTEIN (ENHANCER OF SPLIT M9/10).//0.36:104:22//DROSOPHILA MELANOGASTER (FRUIT FLY).//P16371
F-NT2RM4002145//SLIT PROTEIN PRECURSOR.//8.6e-13:127:33//DROSOPHILA MELANOGASTER (FRUIT FLY).//P24014
F-NT2RM4002146//MAGO NASHI PROTEIN.//7.9e-69:143:91//DROSOPHILA MELANOGASTER (FRUIT FLY).//P49028
F-NT2RM4002161//DUAL SPECIFICITY PROTEIN PHOSPHATASE (EC 3.1.3.48) (EC 3.1.3.16).//0.0062:99:26//CHLAMYDOMONAS EUGAMETOS.//Q39491
F-NT2RM4002174//MRP PROTEIN.//4.5e-50:183:55//ESCHERICHIA COLI.//P21590
F-NT2RM4002189//MUCIN 2 PRECURSOR (INTESTINAL MUCIN 2).//2.6e-14:233:29//HOMO SAPIENS (HUMAN).//Q02817
F-NT2RM4002194//TRANSMEMBRANE PROTEIN SEX PRECURSOR.//0.92:108:28//HOMO SAPIENS (HUMAN).//P51805
F-NT2RM4002205//ELONGATION FACTOR G, MITOCHONDRIAL PRECURSOR (MEF-G).//5.8e-39:122:72//RATTUS NORVEGICUS (RAT).//Q07803
F-NT2RM4002213//HYPOTHETICAL 88.4 KD PROTEIN B0464.7 IN CHROMOSOME III.//9.9e-27:110:43//CAENORHABDITIS ELEGANS.//Q03565
F-NT2RM4002226//GTPASE ACTIVATING PROTEIN ROTUND.//1.3e-21:147:41//DROSOPHILA MELANOGASTER (FRUIT FLY).//P40809
F-NT2RM4002251//PROTEIN EF-7 (FRAGMENT).//0.00082:45:42//MUS MUSCULUS (MOUSE).//P97805
F-NT2RM4002256//COLD-REGULATED PROTEIN 1 (FRAGMENT).//0.00015:114:42//HORDEUM VULGARE (BARLEY).//P23251
F-NT2RM4002266//CUTICLE COLLAGEN 2.//0.00013:142:33//CAENORHABDITIS ELEGANS.//P17656
F-NT2RM4002278//HYPOTHETICAL 22.2 KD PROTEIN IN NSR1-TIF4631 INTERGENIC REGION.//1.0:40:52//SACCHAROMYCES CEREVISIAE (BAKER'S YEAST).//P53288
F-NT2RM4002281
F-NT2RM4002287//GAR2 PROTEIN.//0.00055:225:23//SCHIZOSACCHAROMYCES POMBE (FISSION YEAST).//P41891
F-NT2RM4002294//HYPOTHETICAL PROTEIN KIAA0281 (HA6725).//1.1e-60:152:75//HOMO SAPIENS (HUMAN).//Q92556
F-NT2RM4002301//GENERAL STRESS PROTEIN CTC (FRAGMENT).//0.56:43:39//BACILLUS CALDOLYTICUS.//P42832
F-NT2RM4002323//NONHISTONE CHROMOSOMAL PROTEIN HMG-17.//0.0080:73:35//BOS TAURUS (BOVINE).//P02313
F-NT2RM4002339//METALLOTHIONEIN 10-III (MT-10-III).//0.67:34:38//MYTILUS EDULIS (BLUE MUSSEL).//P80248
F-NT2RM4002344//METALLOTHIONEIN-I (MT-I).//0.84:41:31//MUS MUSCULUS (MOUSE).//P02802
F-NT2RM4002373//GLUTENIN, HIGH MOLECULAR WEIGHT SUBUNIT DY10 PRECURSOR.//0.0019:190:28//TRITICUM AESTIVUM (WHEAT).//P10387
F-NT2RM4002374//5E5 ANTIGEN.//0.0059:170:32//RATTUS NORVEGICUS (RAT).//Q63003
F-NT2RM4002383//!!!! ALU SUBFAMILY SP WARNING ENTRY !!!!//0.13:17:88//HOMO SAPIENS (HUMAN).//P39193
F-NT2RM4002390
F-NT2RM4002398//HNRNP ARGININE N-METHYLTRANSFERASE (EC 2.1.1.-) (ODP1 PROTEIN).//0.034:110:27//SACCHAROMYCES CEREVISIAE (BAKER'S YEAST).//P38074
F-NT2RM4002409//ACETYL-COENZYME A SYNTHETASE (EC 6.2.1.1) (ACETATE--COA LIGASE) (ACYL- ACTIVATING ENZYME).//4.0e-20:179:31//METHANOTHRIX SOEHNGENII.//P27095
F-NT2RM4002438//!!!! ALU SUBFAMILY SQ WARNING ENTRY !!!!//1.7e-15:41:95//HOMO SAPIENS (HUMAN).//P39194
F-NT2RM4002446//CRYPTDIN-RELATED PROTEIN 4C-1 PRECURSOR (CRS4C).//0.0058:24:50//MUS MUSCULUS (MOUSE).//P17534
F-NT2RM4002452//METALLOTHIONEIN 10-II (MT-10-II).//0.83:48:37//MYTILUS EDULIS (BLUE MUSSEL).//P80247
F-NT2RM4002457//!!!! ALU SUBFAMILY SC WARNING ENTRY !!!!//4.9e-07:52:63//HOMO SAPIENS (HUMAN).//P39192
F-NT2RM4002460//C-HORDEIN (CLONE PC-919) (FRAGMENT).//0.92:43:30//HORDEUM VULGARE (BARLEY).//P17992
F-NT2RM4002479//RNA HELICASE-LIKE PROTEIN DB10.//1.7e-28:200:41//NICOTIANA SYLVESTRIS (WOOD TOBACCO).//P46942
F-NT2RM4002482//HYPOTHETICAL 65.9 KD PROTEIN YPR065W.//8.8e-26:123:49//SACCHAROMYCES CEREVISIAE (BAKER'S YEAST).//Q12514 F-NT2RM4002493//LARVAL CUTICLE PROTEIN I PRECURSOR.//0.17:126:27//DROSOPHILA MIRANDA (FRUIT FLY).//P91627
F-NT2RM4002499//!!!! ALU SUBFAMILY SQ WARNING ENTRY !!!!//9.4e-34:92:80//HOMO SAPIENS (HUMAN).//P39194
F-NT2RM4002504//!!!! ALU SUBFAMILY SB WARNING ENTRY !!!!//3.4e-19:55:83//HOMO SAPIENS (HUMAN).//P39189
F-NT2RM4002527//WD-40 REPEAT PROTEIN MSI2.//3.0e-07:193:27//ARABIDOPSIS THALIANA (MOUSE-EAR CRESS).//O22468
F-NT2RM4002532//AEROLYSIN REGULATORY PROTEIN.//0.97:19:47//AEROMONAS SOBRIA.//P09165
F-NT2RM4002534//MITOCHONDRIAL 60S RIBOSOMAL PROTEIN L32 PRECURSOR (YML32).//0.76:86:22//SACCHAROMYCES CEREVISIAE (BAKER'S YEAST).//P25348
F-NT2RM4002558//LONG-CHAIN FATTY ACID TRANSPORT PROTEIN (FATP).//4.2e-55:204:50//MUS MUSCULUS (MOUSE).//Q60714
F-NT2RM4002565//CHYMOTRYPSIN/ELASTASE ISOINHIBITORS 2 TO 5.//1.0:16:62//ASCARIS SUUM (PIG ROUNDWORM) (ASCARIS LUMBRICOIDES).//P07852
F-NT2RM4002567//HYPOTHETICAL 74.0 KD PROTEIN IN CAJ1-HOM3 INTERGENIC REGION.//2.7e-10:184:29//SACCHAROMYCES CEREVISIAE (BAKER'S YEAST).//P40032
F-NT2RM4002571//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N- ACETYLGALACTOSAMINYLTRANSFERASE) (GALNAC-T1).//2.4e-25:124:47//HOMO SAPIENS (HUMAN).//Q10472
F-NT2RM4002593//HYPOTHETICAL 9.1 KD PROTEIN IN TETB-EXOA INTERGENIC REGION.//0.95:36:38//BACILLUS SUBTILIS.//P37509
F-NT2RM4002594//MSP1 PROTEIN HOMOLOG.//9.0e-68:227:60//CAENORHABDITIS ELEGANS.//P54815
F-NT2RM4002623//ASPARTYL-TRNA SYNTHETASE (EC 6.1.1.12) (ASPARTATE--TRNA LIGASE) (ASPRS).//3.3e-54:243:47//SYNECHOCYSTIS SP. (STRAIN PCC 6803).//P73851
F-NT2RP1000018//SUPPRESSOR PROTEIN SRP40.//0.0023:131:25//SACCHAROMYCES CEREVISIAE (BAKER'S YEAST).//P32583
F-NT2RP1000035//RING CANAL PROTEIN (KELCH PROTEIN).//1.0e-06:63:34//DROSOPHILA MELANOGASTER (FRUIT FLY).//Q04652
F-NT2RP1000040//LETHAL NEUROTOXIN TX1.//0.69:21:47//PHONEUTRIA NIGRIVENTER (BRAZILIAN ARMED SPIDER).//P17727
F-NT2RP1000063//HYPOTHETICAL 25.1 KD PROTEIN IN SMC3-MRPL8 INTERGENIC REGION.//3.8e-14:130:30//SACCHAROMYCES CEREVISIAE (BAKER'S YEAST).//P40359
F-NT2RP1000086//HYPOTHETICAL 9.4 KD PROTEIN IN RNPA-THDF INTERGENIC REGION.//0.16:44:40//ESCHERICHIA COLI.//P22847
F-NT2RP1000101//45.8 KD PROTEIN IN SHM1-MRPL37 INTERGENIC REGION.//1.9e-06:74:32//SACCHAROMYCES CEREVISIAE (BAKER'S YEAST).//P38344
F-NT2RP1000111//COP1 REGULATORY PROTEIN (FUSCA PROTEIN FUS1).//2.7e-19:135:36//ARABIDOPSIS THALIANA (MOUSE-EAR CRESS).//P43254
F-NT2RP1000112//DUAL SPECIFICITY PROTEIN KINASE TTK (EC 2.7.1.-) (PYT).//1.2e-39:91:62//HOMO SAPIENS (HUMAN).//P33981
F-NT2RP1000124//ATP-DEPENDENT PROTEASE LA 2 (EC 3.4.21.53).//0.074:131:24//MYXOCOCCUS XANTHUS.//P36774
F-NT2RP1000130//HEPATOMA-DERIVED GROWTH FACTOR (HDGF).//1.5e-49:186:56//MUS MUSCULUS (MOUSE).//P51859
F-NT2RP1000163//METALLOTHIONEIN (MT).//0.98:41:34//PLEURONECTES PLATESSA (PLAICE).//P07216
F-NT2RP1000170//SALIVARY PROLINE-RICH PROTEIN PO (ALLELE K) [CONTAINS: PEPTIDE P-D] (FRAGMENT).//0.85:64:35//HOMO SAPIENS (HUMAN).//P10162
F-NT2RP1000174//IMMEDIATE-EARLY PROTEIN IE180.//0.00056:89:37//PSEUDORABIES VIRUS (STRAIN KAPLAN) (PRV).//P33479
F-NT2RP1000191//NIFU PROTEIN.//0.53:78:35//FRANKIA ALNI.//P46045
F-NT2RP1000202//ANKYRIN, BRAIN VARIANT 2 (ANKYRIN B) (ANKYRIN, NONERYTHROID) (FRAGMENT).//9.1e-21:148:39//HOMO SAPIENS (HUMAN).//Q01485
F-NT2RP1000243//HYPOTHETICAL PROTEIN MJ1136.//1.4e-37:219:36//METHANOCOCCUS JANNASCHII.//Q58536
F-NT2RP1000259//HYPOTHETICAL PROTEIN TP0318.//0.18:25:44//TREPONEMA PALLIDUM.//O83338
F-NT2RP1000272//SPLICING FACTOR, ARGININE/SERINE-RICH 3 (PRE-MRNA SPLICING FACTOR SRP20) (X16 PROTEIN).//1.6e-18:133:36//HOMO SAPIENS (HUMAN), AND MUS MUSCULUS (MOUSE).//P23152
F-NT2RP1000324
F-NT2RP1000326//HYPOTHETICAL 29.8 KD PROTEIN ZC97.1 IN CHROMOSOME III.//1.0e-23:129:36//CAENORHABDITIS ELEGANS.//P34599
F-NT2RP1000333//ANTI-SILENCING PROTEIN 1.//2.5e-45:147:57//SACCHAROMYCES CEREVISIAE (BAKER'S YEAST).//P32447
F-NT2RP1000348//REDUCED VIABILITY UPON STARVATION PROTEIN 161.//4.8e-14:119:34//SACCHAROMYCES CEREVISIAE (BAKER'S YEAST).//P25343
F-NT2RP1000357//TRYPOMASTIGOTE DECAY-ACCELERATING FACTOR (T-DAF) (FRAGMENT).//1.0:43:32//TRYPANOSOMA CRUZI.//Q26327
F-NT2RP1000358//HYPOTHETICAL 84.4 KD PROTEIN IN RPC2/RET1 3'REGION.//7.9e-28:244:35//SACCHAROMYCES CEREVISIAE (BAKER'S YEAST).//P39744
F-NT2RP1000363//SKIN SECRETORY PROTEIN XP2 PRECURSOR (APEG PROTEIN).//2.2e-07:178:30//XENOPUS LAEVIS (AFRICAN CLAWED FROG).//P17437
F-NT2RP1000376//ANKYRIN R (ANKYRINS 2.1 AND 2.2) (ERYTHROCYTE ANKYRIN).//1.5e-20:254:31//HOMO SAPIENS (HUMAN).//P16157
F-NT2RP1000409//CYTOCHROME C3 (CYTOCHROME C7) (C551.5).//1.0:34:26//DESULFUROMONAS ACETOXIDANS (CHLOROPSEUDOMONAS ETHYLICA).//P00137
F-NT2RP1000413//MEMBRANE-ASSOCIATED PROTEIN HEM-2 (NAP1 PROTEIN).//3.7e-131:230:97//RATTUS NORVEGICUS (RAT).//P55161
F-NT2RP1000416//SALIVARY GLUE PROTEIN SGS-3 PRECURSOR.//0.83:54:40//DROSOPHILA SIMULANS (FRUIT FLY).//P13729
F-NT2RP1000418//HYPOTHETICAL 9.9 KD PROTEIN IN GCVT-SPOIIIAA INTERGENIC REGION.//0.24:91:35//BACILLUS SUBTILIS.//P49779
F-NT2RP1000439//HYPOTHETICAL 100.5 KD PROTEIN C1B9.04 IN CHROMOSOME I.//0.13:172:22//SCHIZOSACCHAROMYCES POMBE (FISSION YEAST).//Q10429
F-NT2RP1000443//QUINONE OXIDOREDUCTASE (EC 1.6.5.5) (NADPH:QUINONE REDUCTASE) (ZETA- CRYSTALLIN).//1.9e-08:167:24//HOMO SAPIENS (HUMAN).//Q08257
F-NT2RP1000460//NUCLEAR MOVEMENT PROTEIN NUDC.//1.0e-18:149:34//EMERICELLA NIDULANS (ASPERGILLUS NIDULANS).//P17624
F-NT2RP1000470//PUTATIVE ATP-DEPENDENT RNA HELICASE T26G10.1 IN CHROMOSOME III.//1.3e-43:180:47//CAENORHABDITIS ELEGANS.//P34580
F-NT2RP1000478//TUBULIN BETA-6 CHAIN (CLASS-VI).//1.5e-45:85:63//GALLUS GALLUS (CHICKEN).//P09207
F-NT2RP1000481//HYPOTHETICAL 5.8 KD PROTEIN IN PUHA 5'REGION (ORF55).//0.083:21:47//RHODOBACTER CAPSULATUS (RHODOPSEUDOMONAS CAPSULATA).//P26159
F-NT2RP1000493//POSSIBLE DNA-REPAIR PROTEIN XP-E (POSSIBLE XERODERMA PIGMENTOSUM GROUP E PROTEIN) (UV-DAMAGED DNA-BINDING PROTEIN) (UV-DDB).//6.6e-11:139:31//CERCOPITHECUS AETHIOPS (GREEN MONKEY) (GRIVET).//P33194
F-NT2RP1000513//60S RIBOSOMAL PROTEIN L22.//0.017:92:30//DROSOPHILA MELANOGASTER (FRUIT FLY).//P50887
F-NT2RP1000522//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//0.0055:86:36//MUS MUSCULUS (MOUSE).//Q61068
F-NT2RP1000547//COP-COATED VESICLE MEMBRANE PROTEIN P24 PRECURSOR (FRAGMENT).//1.2e-09:69:36//CRICETULUS GRISEUS (CHINESE HAMSTER).//P49020
F-NT2RP1000574//HOMEOBOX PROTEIN MEIS2 (MEIS1-RELATED PROTEIN 1).//6.0e-39:141:65//MUS MUSCULUS (MOUSE).//P97367
F-NT2RP1000577//PUTATIVE ATP-DEPENDENT RNA HELICASE YDL031W.//0.00016:48:45//SACCHAROMYCES CEREVISIAE (BAKER'S YEAST).//Q12389
F-NT2RP1000581//VON WILLEBRAND FACTOR PRECURSOR.//0.00017:61:50//HOMO SAPIENS (HUMAN).//P04275
F-NT2RP1000609//LINOLEOYL-COA DESATURASE (EC 1.14.99.25) (DELTA(6)-DESATURASE).//4.4e-07:128:31//SYNECHOCYSTIS SP. (STRAIN PCC 6803).//Q08871
F-NT2RP1000629//CLATHRIN COAT ASSEMBLY PROTEIN AP47 (CLATHRIN COAT ASSOCIATED PROTEIN AP47) (GOLGI ADAPTOR AP-1 47 KD PROTEIN) (HA1 47 KD SUBUNIT) (CLATHRIN ASSEMBLY PROTEIN ASSEMBLY PROTEIN COMPLEX 1 MEDIUM CHAIN).//4.2e-70:167:86//MUS MUSCULUS (MOUSE).//P35585
F-NT2RP1000630//HYPOTHETICAL 118.4 KD PROTEIN IN BAT2-DAL5 INTERGENIC REGION PRECURSOR.//0.0011:238:21//SACCHAROMYCES CEREVISIAE (BAKER'S YEAST).//P47179
F-NT2RP1000677//COLLAGEN ALPHA 1(XVI) CHAIN PRECURSOR.//0.99:71:33//HOMO SAPIENS (HUMAN).//Q07092
F-NT2RP1000688//!!!! ALU SUBFAMILY SP WARNING ENTRY !!!!//0.0024:19:94//HOMO SAPIENS (HUMAN).//P39193
F-NT2RP1000695//HYPOTHETICAL 83.8 KD PROTEIN C27F2.7 IN CHROMOSOME III.//2.2e-30:185:37//CAENORHABDITIS ELEGANS.//Q18262
F-NT2RP1000701//PHOSPHOLIPASE A-2-ACTIVATING PROTEIN (PLAP).//3.2e-65:128:93//RATTUS NORVEGICUS (RAT).//P54319
F-NT2RP1000721//TRANSCRIPTION INITIATION FACTOR TFIID 135 KD SUBUNIT (TAFII-135) (TAFII135) (TAFII-130) (TAFII130).//2.3e-06:139:34//HOMO SAPIENS (HUMAN).//O00268
F-NT2RP1000730//MYOSIN LIGHT CHAIN 1, SLOW-TWITCH MUSCLE B/VENTRICULAR ISOFORM (FRAGMENT).//0.89:40:40//MUS MUSCULUS (MOUSE).//P09542
F-NT2RP1000733//METALLOTHIONEIN-LIKE PROTEIN CRS5.//0.024:24:45//SACCHAROMYCES CEREVISIAE (BAKER'S YEAST).//P41902
F-NT2RP1000738//SALIVARY ACIDIC PROLINE-RICH PHOSPHOPROTEIN 1/2 PRECURSOR (PRP-1 / PRP-3) (PRP-2 / PRP-4) (PIF-F / PIF-S) (PROTEIN A / PROTEIN C) [CONTAINS: PEPTIDE P-C].//0.040:82:36//HOMO SAPIENS (HUMAN).//P02810
F-NT2RP1000746//HYPOTHETICAL 27.1 KD PROTEIN UFD4-CAP1 INTERGENIC REGION.//2.0e-30:170:37//SACCHAROMYCES CEREVISIAE (BAKER'S YEAST).//P33201
F-NT2RP1000767//PSEUDOMONAPEPSIN PRECURSOR (EC 3.4.23.37) (PEPSTATIN-INSENSITIVE CARBOXYL PROTEINASE).//0.99:75:34//PSEUDOMONAS SP. (STRAIN 101).//P42790
F-NT2RP1000782//CELL SURFACE GLYCOPROTEIN A15 (T-CELL ACUTE LYMPHOBLASTIC LEUKEMIA ASSOCIATED ANTIGEN 1) (TALLA-1) (MEMBRANE COMPONENT, X CHROMOSOME, SURFACE MARKER 1).//2.3e-23:159:35//HOMO SAPIENS (HUMAN).//P41732
F-NT2RP1000796//CORNIFIN (SMALL PROLINE-RICH PROTEIN I) (SPR-I) (SMALL PROLINE-RICH SQUAMOUS CELL MARKER) (SPRP).//0.00018:79:32//SUS SCROFA (PIG).//P35323
F-NT2RP1000825//GTPASE-ACTIVATING PROTEIN RHOGAP (RHO-RELATED SMALL GTPASE PROTEIN ACTIVATOR) (CDC42 GTPASE-ACTIVATING PROTEIN) (P50-RHOGAP).//3.1e-37:89:64//HOMO SAPIENS (HUMAN).//Q07960
F-NT2RP1000833//SMALL PROLINE RICH PROTEIN II (SPR-II) (CLONE 930).//0.32:29:48//HOMO SAPIENS (HUMAN).//P22531
F-NT2RP1000834//2-ARYLPROPIONYL-COA EPIMERASE (EC 5.-.-.-).//6.4e-67:202:68//RATTUS NORVEGICUS (RAT).//P70473
F-NT2RP1000836//HYPOTHETICAL 7.3 KD PROTEIN IN 100 KD PROTEIN REGION.//1.0:35:54//HUMAN ADENOVIRUS TYPE 41.//P23691
F-NT2RP1000846//SMALL PROLINE-RICH PROTEIN 2-1.//0.013:35:48//HOMO SAPIENS (HUMAN).//P35326
F-NT2RP1000851//PERIOD CLOCK PROTEIN (FRAGMENT).//0.082:28:57//DROSOPHILA SALTANS (FRUIT FLY).//Q04536
F-NT2RP1000856//PLATELET-ENDOTHELIAL TETRASPAN ANTIGEN 3 (PETA-3) (GP27) (MEMBRANE GLYCOPROTEIN SFA-1) (CD151 ANTIGEN).//2.5e-26:190:30//MUS MUSCULUS (MOUSE).//O35566
F-NT2RP1000860//POTENTIAL TRANSCRIPTIONAL ADAPTOR.//0.13:86:36//SACCHAROMYCES CEREVISIAE (BAKER'S YEAST).//Q02336
F-NT2RP1000902//HYPOTHETICAL 127.4 KD PROTEIN F07F6.4 IN CHROMOSOME III.//7.6e-11:200:35//CAENORHABDITIS ELEGANS.//Q09531
F-NT2RP1000915//HYPOTHETICAL GTP-BINDING PROTEIN IN PMI40-PAC2 INTERGENIC REGION.//1.4e-06:88:35//SACCHAROMYCES CEREVISIAE (BAKER'S YEAST).//P40010
F-NT2RP1000916//SUPPRESSOR PROTEIN SRP40.//0.40:90:35//SACCHAROMYCES CEREVISIAE (BAKER'S YEAST).//P32583
F-NT2RP1000943//MUCIN 2 PRECURSOR (INTESTINAL MUCIN 2).//0.099:75:34//HOMO SAPIENS (HUMAN).//Q02817
F-NT2RP1000944//HYPOTHETICAL 29.3 KD PROTEIN (ORF92).//7.6e-06:65:41//ORGYIA PSEUDOTSUGATA MULTICAPSID POLYHEDROSIS VIRUS (OPMNPV).//O10341
F-NT2RP1000947//UBIQUITIN-CONJUGATING ENZYME E2-17 KD 2 (EC 6.3.2.19) (UBIQUITIN- PROTEIN LIGASE) (UBIQUITIN CARRIER PROTEIN) (E2(17)KB 2).//3.6e-12:27:77//HOMO SAPIENS (HUMAN), MUS MUSCULUS (MOUSE), RATTUS NORVEGICUS (RAT), AND XENOPUS LAEVIS (AFRICAN CLAWED FROG).//P51669
F-NT2RP1000954//RING CANAL PROTEIN (KELCH PROTEIN).//2.8e-15:169:28//DROSOPHILA MELANOGASTER (FRUIT FLY).//Q04652
F-NT2RP1000958//HYPOTHETICAL GTP-BINDING PROTEIN IN PMI40-PAC2 INTERGENIC REGION.//4.2e-16:162:32//SACCHAROMYCES CEREVISIAE (BAKER'S YEAST).//P40010
F-NT2RP1000959//CORNIFIN A (SMALL PROLINE-RICH PROTEIN IA) (SPR-IA) (SPRK).//0.0031:34:44//HOMO SAPIENS (HUMAN).//P35321
F-NT2RP1000966//NUCLEOLIN (PROTEIN C23).//1.5e-52:110:95//HOMO SAPIENS (HUMAN).//P19338
F-NT2RP1000980//LIGHT-HARVESTING PROTEIN B-1015, ALPHA CHAIN PRECURSOR (ANTENNA PIGMENT PROTEIN, ALPHA CHAIN).//0.87:37:45//RHODOPSEUDOMONAS VIRIDIS.//P04123
F-NT2RP1000988
F-NT2RP1001011//PROTEIN P19.//0.96:30:50//BACTERIOPHAGE PRD1.//P17638
F-NT2RP1001013//DNA-BINDING PROTEIN 65 (PROTEIN GP65).//1.0:20:45//BACTERIOPHAGE T4.//P16012
F-NT2RP1001014
F-NT2RP1001033//TUBULIN GAMMA CHAIN.//2.5e-16:112:42//SCHIZOSACCHAROMYCES POMBE (FISSION YEAST).//P25295
F-NT2RP1001073//HYPOTHETICAL 10.4 KD PROTEIN IN FTR1-SPT15 INTERGENIC REGION.//7.6e-16:82:51//SACCHAROMYCES CEREVISIAE (BAKER'S YEAST).//P40089
F-NT2RP1001079//SARCOSINE OXIDASE (EC 1.5.3.1).//4.8e-15:95:40//ARTHROBACTER SP. (STRAIN TE1826).//P40873
F-NT2RP1001080//PROBABLE ATP-DEPENDENT RNA HELICASE DBP9.//2.4e-29:126:46//SACCHAROMYCES CEREVISIAE (BAKER'S YEAST).//Q06218
F-NT2RP1001113//SMALL PROLINE-RICH PROTEIN 2-1.//0.49:38:39//HOMO SAPIENS (HUMAN).//P35326
F-NT2RP1001173//RHOMBOTIN-1 (CYSTEINE RICH PROTEIN TTG-1) (T-CELL TRANSLOCATION PROTEIN 1) (LIM-ONLY PROTEIN 1).//0.99:54:37//HOMO SAPIENS (HUMAN).//P25800
F-NT2RP1001177//HISTONE MACRO-H2A.1.//1.6e-29:85:76//RATTUS NORVEGICUS (RAT).//Q02874
F-NT2RP1001185
F-NT2RP1001199//NEUROTOXIN I.//1.0:23:47//CENTRUROIDES SCULPTURATUS (BARK SCORPION).//P01491
F-NT2RP1001247//TRANSFORMING GROWTH FACTOR BETA 4 PRECURSOR (TGF-BETA 4) (ENDOMETRIAL BLEEDING-ASSOCIATED FACTOR).//3.3e-08:28:89//HOMO SAPIENS (HUMAN).//O00292
F-NT2RP1001248//TAT PROTEIN (TRANSACTIVATING REGULATORY PROTEIN).//0.33:49:28//HUMAN IMMUNODEFICIENCY VIRUS TYPE 1 (NDK ISOLATE) (HIV-1).//P18804
F-NT2RP1001253//GLUCOSAMINE-6-PHOSPHATE ISOMERASE (EC 5.3.1.10) (GLUCOSAMINE-6- PHOSPHATE DEAMINASE) (GNPDA) (OSCILLIN) (KIAA0060).//3.8e-46:115:81//HOMO SAPIENS (HUMAN).//P46926
F-NT2RP1001286//GALECTIN-3 (GALACTOSE-SPECIFIC LECTIN 3) (MAC-2 ANTIGEN) (IGE-BINDING PROTEIN) (35 KD LECTIN) (CARBOHYDRATE BINDING PROTEIN 35) (CBP 35) (LAMININ-BINDING PROTEIN) (LECTIN L-29) (L-34 GALACTOSIDE-BINDING LECTIN).//0.16:48:37//MUS MUSCULUS (MOUSE).//P16110
F-NT2RP1001294//MICROTUBULE-ASSOCIATED PROTEIN YTM1.//6.1e-05:92:34//SACCHAROMYCES CEREVISIAE (BAKER'S YEAST).//Q12024
F-NT2RP1001302//MICROTUBULE-ASSOCIATED PROTEIN YTM1.//1.2e-05:92:33//SACCHAROMYCES CEREVISIAE (BAKER'S YEAST).//Q12024
F-NT2RP1001310//PROBABLE E4 PROTEIN.//0.99:109:26//HUMAN PAPILLOMAVIRUS TYPE 5.//P06924
F-NT2RP1001311//SODIUM/HYDROGEN EXCHANGER 5 (NA(+)/H(+) EXCHANGER 5) (NHE-5) (FRAGMENT).//0.99:94:31//HOMO SAPIENS (HUMAN).//Q14940
F-NT2RP1001313//CYTOCHROME B5.//9.0e-13:92:38//SACCHAROMYCES CEREVISIAE (BAKER'S YEAST).//P40312
F-NT2RP1001361//NADH-UBIQUINONE OXIDOREDUCTASE SUBUNIT B14.5B (EC 1.6.5.3) (EC 1.6.99.3) (COMPLEX I-B14.5B) (CI-B14.5B).//1.2e-47:117:74//BOS TAURUS (BOVINE).//Q02827
F-NT2RP1001385//CELL DIVISION PROTEIN FTSN.//0.64:107:28//ESCHERICHIA COLI.//P29131
F-NT2RP1001395//PROCOLLAGEN ALPHA 2(I) CHAIN PRECURSOR (FRAGMENTS).//0.25:35:45//GALLUS GALLUS (CHICKEN).//P02467
F-NT2RP1001410//PUTATIVE GTP-BINDING PROTEIN W08E3.3.//2.2e-41:129:67//CAENORHABDITIS ELEGANS.//P91917
F-NT2RP1001424//UREASE ACCESSORY PROTEIN UREF (FRAGMENT).//0.87:24:45//ESCHERICHIA COLI.//Q03286
F-NT2RP1001432//CYSTEINE PROTEINASE INHIBITOR B (CYSTATIN B) (SCB).//1.0:35:42//HELIANTHUS ANNUUS (COMMON SUNFLOWER).//Q10993
F-NT2RP1001449//KERATIN, ULTRA HIGH-SULFUR MATRIX PROTEIN (UHS KERATIN).//0.053:37:37//OVIS ARIES (SHEEP).//P26372
F-NT2RP1001457//HYPOTHETICAL 57.0 KD TRP-ASP REPEATS CONTAINING PROTEIN IN CPR4-SSK22 INTERGENIC REGION.//2.9e-16:159:30//SACCHAROMYCES CEREVISIAE (BAKER'S YEAST).//P25382
F-NT2RP1001466//HYPOTHETICAL PROTEIN MJ0284.//5.3e-15:162:35//METHANOCOCCUS JANNASCHII.//Q57732
F-NT2RP1001475//HYPOTHETICAL 195.1 KD PROTEIN IN DNA43-UBI1 INTERGENIC REGION.//0.69:119:27//SACCHAROMYCES CEREVISIAE (BAKER'S YEAST).//P40457
F-NT2RP1001482//PROTEASOME COMPONENT C9 (EC 3.4.99.46) (MACROPAIN SUBUNIT C9) (MULTICATALYTIC ENDOPEPTIDASE COMPLEX SUBUNIT C9).//1.0:58:32//HOMO SAPIENS (HUMAN).//P25789
F-NT2RP1001494//MALE STERILITY PROTEIN 2.//2.4e-12:84:42//ARABIDOPSIS THALIANA (MOUSE-EAR CRESS).//Q08891
F-NT2RP1001543//MYO-INOSITOL-1-PHOSPHATE SYNTHASE (EC 5.5.1.4) (IPS).//6.3e-37:94:52//SPIRODELA POLYRRHIZA.//P42803
F-NT2RP1001546//LEUKOCYTE SURFACE ANTIGEN CD53 (CELL SURFACE GLYCOPROTEIN CD53).//9.3e-11:98:29//HOMO SAPIENS (HUMAN).//P19397
F-NT2RP1001569//SIGNAL RECOGNITION PARTICLE RECEPTOR BETA SUBUNIT (SR-BETA).//2.2e-64:159:84//MUS MUSCULUS (MOUSE).//P47758
F-NT2RP1001616//HYPOTHETICAL 13.5 KD PROTEIN C45G9.7 IN CHROMOSOME III.//9.2e-05:49:42//CAENORHABDITIS ELEGANS.//Q09506
F-NT2RP1001665//REGB PROTEIN.//0.99:29:37//PSEUDOMONAS AERUGINOSA.//Q03381
F-NT2RP2000001//SMALL PROLINE-RICH PROTEIN 2-1.//0.64:36:41//HOMO SAPIENS (HUMAN).//P35326
F-NT2RP2000006//DNAJ PROTEIN HOMOLOG 1 (HDJ-1) (HEAT SHOCK PROTEIN 40) (HSP40).//1.7e-19:74:52//HOMO SAPIENS (HUMAN).//P25685
F-NT2RP2000007//TROPOMYOSIN, FIBROBLAST AND EPITHELIAL MUSCLE-TYPE (TM36) (TME1) (TM1).//0.93:126:23//HOMO SAPIENS (HUMAN).//P06468
F-NT2RP2000008//ZINC FINGER PROTEIN 33A (ZINC FINGER PROTEIN KOX31) (KIAA0065) (HA0946) (FRAGMENT).//4.2e-35:156:54//HOMO SAPIENS (HUMAN).//Q06730
F-NT2RP2000027//NADH-UBIQUINONE OXIDOREDUCTASE CHAIN 5 (EC 1.6.5.3) (FRAGMENT).//0.95:41:39//MACACA FASCICULARIS (CRAB EATING MACAQUE) (CYNOMOLGUS MONKEY).//P50665
F-NT2RP2000032//BAX PROTEIN, CYTOPLASMIC ISOFORM GAMMA.//1.0:35:34//HOMO SAPIENS (HUMAN).//Q07815
F-NT2RP2000040//BASIC PROLINE-RICH PEPTIDE IB-1.//0.0024:58:36//HOMO SAPIENS (HUMAN).//P04281
F-NT2RP2000045//DNAJ PROTEIN.//1.1e-12:42:66//THERMUS AQUATICUS (SUBSP. THERMOPHILUS).//Q56237
F-NT2RP2000054//GONADOLIBERIN III PRECURSOR (GONADOTROPIN-RELEASING HORMONE III) (GNRH-III) (LH-RH III) (LULIBERIN III).//0.20:46:36//ONCORHYNCHUS MASOU (CHERRY SALMON) (MASU SALMON).//P30973
F-NT2RP2000056//PROTEIN-TYROSINE PHOSPHATASE EPSILON PRECURSOR (EC 3.1.3.48) (R-PTP- EPSILON).//1.3e-18:45:100//MUS MUSCULUS (MOUSE).//P49446
F-NT2RP2000067//HOMEOBOX PROTEIN HOX-A5 (S12-B) (FRAGMENT).//0.71:44:40//SALMO SALAR (ATLANTIC SALMON).//P09637
F-NT2RP2000070//INSULIN.//0.94:30:43//HYSTRIX CRISTATA (CRESTED PORCUPINE).//P01328
F-NT2RP2000076//ETS-LIKE PROTEIN POINTED P1 (D-ETS-2).//0.0013:76:40//DROSOPHILA MELANOGASTER (FRUIT FLY).//P51022
F-NT2RP2000077//U1 SMALL NUCLEAR RIBONUCLEOPROTEIN C (U1-C).//0.24:49:40//HOMO SAPIENS (HUMAN).//P09234
F-NT2RP2000079//PLATELET FACTOR 4 (PF-4).//0.15:52:30//SUS SCROFA (PIG).//P30034
F-NT2RP2000088//HYPOTHETICAL 13.6 KD PROTEIN IN SPT4-ROM1 INTERGENIC REGION.//1.0:36:44//SACCHAROMYCES CEREVISIAE (BAKER'S YEAST).//P53245
F-NT2RP2000091//HYPOTHETICAL PROTEIN HI0149 PRECURSOR.//0.22:38:47//HAEMOPHILUS INFLUENZAE.//P43953
F-NT2RP2000097//VIRUS ATTACHMENT PROTEIN (O61R).//0.75:33:36//AFRICAN SWINE FEVER VIRUS (STRAIN BA71V) (ASFV).//P32510
F-NT2RP2000098
F-NT2RP2000108//!!!! ALU SUBFAMILY SX WARNING ENTRY !!!!//2.4e-09:50:70//HOMO SAPIENS (HUMAN).//P39195
F-NT2RP2000114//WISKOTT-ALDRICH SYNDROME PROTEIN (WASP).//0.024:52:44//HOMO SAPIENS (HUMAN).//P42768
F-NT2RP2000120//5.8 KD PROTEIN IN HMC OPERON (ORF 4).//0.67:37:32//DESULFOVIBRIO VULGARIS (STRAIN HILDENBOROUGH).//P33391
F-NT2RP2000126//CHROMODOMAIN-HELICASE-DNA-BINDING PROTEIN 1 (CHD-1).//1.5e-23:94:47//HOMO SAPIENS (HUMAN).//O14646
F-NT2RP2000133//SPLICEOSOME ASSOCIATED PROTEIN 49 (SAP 49) (SF3B53).//5.6e-10:82:39//HOMO SAPIENS (HUMAN).//Q15427
F-NT2RP2000147//CLATHRIN COAT ASSEMBLY PROTEIN AP47 (CLATHRIN COAT ASSOCIATED PROTEIN AP47) (GOLGI ADAPTOR AP-1 47 KD PROTEIN) (HA1 47 KD SUBUNIT) (CLATHRIN ASSEMBLY PROTEIN ASSEMBLY PROTEIN COMPLEX 1 MEDIUM CHAIN).//6.7e-89:96:98//MUS MUSCULUS (MOUSE).//P35585
F-NT2RP2000153//PEPTIDYLPROLYL ISOMERASE CYP-1 (EC 5.2.1.8) (PEPTIDYLPROLYL CIS-TRANS ISOMERASE) (CYCLOPHILIN) (PPIASE).//1.7e-05:136:33//BRUGIA MALAYI.//Q27450
F-NT2RP2000157//MLO2 PROTEIN.//2.7e-06:62:40//SCHIZOSACCHAROMYCES POMBE (FISSION YEAST).//Q09329
F-NT2RP2000161//DIS3 PROTEIN HOMOLOG.//2.7e-33:173:45//CAENORHABDITIS ELEGANS.//Q17632
F-NT2RP2000173//HYPOTHETICAL 10.5 KD PROTEIN IN SODA-COMGA INTERGENIC REGION.//0.99:62:25//BACILLUS SUBTILIS.//P54499
F-NT2RP2000175//MALE SPECIFIC SPERM PROTEIN MST84DB.//0.19:41:43//DROSOPHILA MELANOGASTER (FRUIT FLY).//Q01643
F-NT2RP2000183//DIHYDROPYRIMIDINASE RELATED PROTEIN-2 (DRP-2) (NEURAL SPECIFIC PROTEIN NSP60).//4.1e-19:114:44//BOS TAURUS (BOVINE).//O02675
F-NT2RP2000195//ATP SYNTHASE PROTEIN 8 (EC 3.6.1.34) (A6L).//0.99:30:33//MICROTUS PENNSYLVANICUS (MEADOW VOLE).//P24949
F-NT2RP2000205//MERCURIC TRANSPORT PROTEIN PERIPLASMIC COMPONENT PRECURSOR (PERIPLASMIC MERCURY ION BINDING PROTEIN) (MERCURY SCAVENGER PROTEIN).//0.098:88:25//SHEWANELLA PUTREFACIENS (PSEUDOMONAS PUTREFACIENS).//Q54463
F-NT2RP2000208//MALE SPECIFIC SPERM PROTEIN MST84DD.//0.020:19:57//DROSOPHILA MELANOGASTER (FRUIT FLY).//Q01645
F-NT2RP2000224//PUTATIVE CUTICLE COLLAGEN C09G5.4.//0.0058:159:32//CAENORHABDITIS ELEGANS.//Q09455
F-NT2RP2000232//P55-C-FOS PROTO-ONCOGENE PROTEIN (FRAGMENT).//1.0:44:38//OVIS ARIES (SHEEP).//O02761
F-NT2RP2000233//GASTRIN/CHOLECYSTOKININ TYPE B RECEPTOR (CCK-B RECEPTOR) (CCK-BR).//0.34:53:43//CANIS FAMILIARIS (DOG).//P30552
F-NT2RP2000239//ACIDIC PROLINE-RICH PROTEIN PRECURSOR (CLONE PRP25) (FRAGMENT).//0.019:69:33//RATTUS NORVEGICUS (RAT).//P10164
F-NT2RP2000248//OVOMUCOID (FRAGMENT).//0.88:18:55//POLYPLECTRON EMPHANUM (PALAWAN PEACOCK-PHEASANT).//P52250
F-NT2RP2000257//PUTATIVE MITOCHONDRIAL CARRIER YIL006W.//6.4e-09:83:37//SACCHAROMYCES CEREVISIAE (BAKER'S YEAST).//P40556
F-NT2RP2000258//MYOSIN II HEAVY CHAIN, NON MUSCLE.//0.081:217:28//DICTYOSTELIUM DISCOIDEUM (SLIME MOLD).//P08799
F-NT2RP2000270//!!!! ALU SUBFAMILY J WARNING ENTRY !!!!//2.4e-17:80:57//HOMO SAPIENS (HUMAN).//P39188
F-NT2RP2000274//HYPOTHETICAL 5.8 KD PROTEIN.//0.082:22:45//CLOVER YELLOW MOSAIC VIRUS (CYMV).//P16485
F-NT2RP2000283//HYPOTHETICAL 83.6 KD PROTEIN R05D3.2 IN CHROMOSOME III.//0.39:38:34//CAENORHABDITIS ELEGANS.//P34535
F-NT2RP2000288
F-NT2RP2000289//HYPOTHETICAL 9.4 KD PROTEIN IN RNPA-THDF INTERGENIC REGION.//0.40:38:42//ESCHERICHIA COLI.//P22847
F-NT2RP2000297//ZINC FINGER PROTEIN 85 (ZINC FINGER PROTEIN HPF4) (HTF1).//2.3e-62:206:47//HOMO SAPIENS (HUMAN).//Q03923
F-NT2RP2000298//CUTICLE COLLAGEN 12 PRECURSOR.//0.55:81:40//CAENORHABDITIS ELEGANS.//P20630
F-NT2RP2000310//RUBREDOXIN (RD).//0.13:43:41//TREPONEMA PALLIDUM.//O83956
F-NT2RP2000327//ATP SYNTHASE PROTEIN 8 (EC 3.6.1.34) (A6L).//1.0:46:30//GADUS MORHUA (ATLANTIC COD).//P15996
F-NT2RP2000328//HYPOTHETICAL 86.6 KD PROTEIN IN PFK1-TDS4 INTERGENIC REGION.//2.0e-21:198:31//SACCHAROMYCES CEREVISIAE (BAKER'S YEAST).//P53313
F-NT2RP2000329//GTP:AMP PHOSPHOTRANSFERASE MITOCHONDRIAL (EC 2.7.4.10) (AK3).//1.8e-91:155:92//BOS TAURUS (BOVINE).//P08760
F-NT2RP2000337//PROTEIN A54.//0.75:48:35//VACCINIA VIRUS (STRAIN WR), AND VACCINIA VIRUS (STRAIN COPENHAGEN).//P21072
F-NT2RP2000346//MYELOID DIFFERENTIATION PRIMARY RESPONSE PROTEIN MYD116.//9.7e-13:114:42//MUS MUSCULUS (MOUSE).//P17564
F-NT2RP2000369//CALTRIN (CALCIUM TRANSPORT INHIBITOR).//0.98:47:34//MUS MUSCULUS (MOUSE).//Q09098
F-NT2RP2000412//SHORT NEUROTOXIN D PRECURSOR.//0.66:57:36//AIPYSURUS LAEVIS (OLIVE SEA SNAKE).//P19960
F-NT2RP2000414//HETEROGENEOUS NUCLEAR RIBONUCLEOPROTEIN F (HNRNP F).//1.0e-27:96:67//HOMO SAPIENS (HUMAN).//P52597
F-NT2RP2000420//ZINC FINGER PROTEIN 191.//0.16:47:38//HOMO SAPIENS (HUMAN).//O14754
F-NT2RP2000422//PUTATIVE PHOSPHOACETYLGLUCOSAMINE MUTASE (EC 5.4.2.3) (ACETYLGLUCOSAMINE PHOSPHOMUTASE) (N-ACETYLGLUCOSAMINE-PHOSPHATE MUTASE).//3.6e-19:148:36//SCHIZOSACCHAROMYCES POMBE (FISSION YEAST).//Q09687
F-NT2RP2000438//TUBULIN GAMMA CHAIN.//0.86:190:27//RETICULOMYXA FILOSA.//P54405
F-NT2RP2000448//OXYSTEROL-BINDING PROTEIN.//3.7e-13:140:42//HOMO SAPIENS (HUMAN).//P22059
F-NT2RP2000459//NEURONAL PROTEIN 3.1 (P311 PROTEIN).//1.0:45:35//HOMO SAPIENS (HUMAN).//Q16612
F-NT2RP2000498//!!!! ALU SUBFAMILY SQ WARNING ENTRY !!!!//0.062:25:68//HOMO SAPIENS (HUMAN).//P39194
F-NT2RP2000503
F-NT2RP2000510//TOXIN IV-5.//1.0:51:33//TITYUS BAHIENSIS (BRAZILIAN SCORPION).//P56608
F-NT2RP2000516//SLYX PROTEIN.//1.0:52:32//ESCHERICHIA COLI.//P30857
F-NT2RP2000523//PHORBOLIN I (FRAGMENTS).//1.4e-06:36:47//HOMO-SAPIENS (HUMAN).//P31941
F-NT2RP2000603//ALPHA/BETA-GLIADIN PRECURSOR (PROLAMIN) (CLASS A-III).//0.93:119:26//TRITICUM AESTIVUM (WHEAT).//P04723
F-NT2RP2000617//SPERM PROTAMINE P1 (CYSTEINE-RICH PROTAMINE).//0.056:16:62//OVIS ARIES (SHEEP), AND CAPRA HIRCUS (GOAT).//P04102
F-NT2RP2000634//NEDD-4 PROTEIN (EC 6.3.2.-) (KIAA0093) (FRAGMENT).//1.8e-05:128:28//HOMO SAPIENS (HUMAN).//P46934
F-NT2RP2000644//HYPOTHETICAL PROTEIN HI1566 PRECURSOR.//0.85:48:39//HAEMOPHILUS INFLUENZAE.//P44257
F-NT2RP2000656//EARLY GROWTH RESPONSE PROTEIN 1 (EGR-1) (NERVE GROWTH FACTOR-INDUCED PROTEIN A) (NGFI-A).//1.0:111:24//RATTUS NORVEGICUS (RAT).//P08154
F-NT2RP2000658//URONATE ISOMERASE (EC 5.3.1.12) (GLUCURONATE ISOMERASE) (URONIC ISOMERASE).//0.49:79:31//ESCHERICHIA COLI.//P42607
F-NT2RP2000668//MEROZOITE SURFACE ANTIGEN 2 PRECURSOR (MSA-2) (45 KD MEROZOITE SURFACE ANTIGEN).//0.020:115:30//PLASMODIUM FALCIPARUM (ISOLATE 3D7).//P50498
F-NT2RP2000678//!!!! ALU SUBFAMILY J WARNING ENTRY !!!!//0.00085:38:68//HOMO SAPIENS (HUMAN).//P39188
F-NT2RP2000704//!!!! ALU SUBFAMILY J WARNING ENTRY !!!!//2.2e-17:55:74//HOMO SAPIENS (HUMAN).//P39188
F-NT2RP2000710//ASPARTYL-TRNA SYNTHETASE (EC 6.1.1.12) (ASPARTATE--TRNA LIGASE) (ASPRS).//8.9e-47:106:59//TREPONEMA PALLIDUM.//O83950
F-NT2RP2000715
F-NT2RP2000731//CONIDIATION-SPECIFIC PROTEIN 10.//0.094:31:41//NEUROSPORA CRASSA.//P10713
F-NT2RP2000758//!!!! ALU SUBFAMILY J WARNING ENTRY !!!!//0.00027:31:74//HOMO SAPIENS (HUMAN).//P39188
F-NT2RP2000764//NIFS PROTEIN.//2.7e-27:175:47//ANABAENA SP. (STRAIN PCC 7120).//P12623
F-NT2RP2000809//HYPOTHETICAL PROTEIN MG381 HOMOLOG.//0.91:85:25//MYCOPLASMA PNEUMONIAE.//P75219
F-NT2RP2000812//DILUTE MYOSIN HEAVY CHAIN, NON-MUSCLE (MYOSIN 5A).//2.8e-07:133:31//MUS MUSCULUS (MOUSE).//Q99104
F-NT2RP2000814//40S RIBOSOMAL PROTEIN S27A.//0.93:44:38//LYCOPERSICON ESCULENTUM (TOMATO), AND SOLANUM TUBEROSUM (POTATO).//P27083
F-NT2RP2000816//HYPOTHETICAL 88.4 KD PROTEIN B0464.7 IN CHROMOSOME III.//3.3e-21:123:39//CAENORHABDITIS ELEGANS.//Q03565
F-NT2RP2000819//TROPOMYOSIN 5, CYTOSKELETAL TYPE.//1.0:71:30//MUS MUSCULUS (MOUSE).//P21107
F-NT2RP2000841//GUANINE NUCLEOTIDE RELEASING PROTEIN (GNRP).//0.0011:133:26//MUS MUSCULUS (MOUSE).//P27671
F-NT2RP2000842//LYSOPHOSPHATIDIC ACID RECEPTOR (EDG-2).//6.4e-13:22:95//HOMO SAPIENS (HUMAN).//Q92633
F-NT2RP2000845//BOWMAN-BIRK TYPE PROTEINASE INHIBITOR (MSTI).//0.92:24:41//MEDICAGO SCUTELLATA (SNAIL MEDIC).//P80321
F-NT2RP2000863//N-MYC PROTO-ONCOGENE PROTEIN.//0.010:148:27//XENOPUS LAEVIS (AFRICAN CLAWED FROG).//P24793
F-NT2RP2000880//PROBABLE TRANSLATION INITIATION FACTOR IF-2.//4.0e-100:199:94//HOMO SAPIENS (HUMAN).//O60841
F-NT2RP2000892//PROCOLLAGEN ALPHA 1(II) CHAIN PRECURSOR [CONTAINS: CHONDROCALCIN].//0.43:45:44//MUS MUSCULUS (MOUSE).//P28481
F-NT2RP2000931//MATRIN 3.//2.8e-46:104:92//RATTUS NORVEGICUS (RAT).//P43244
F-NT2RP2000932//2-5A-DEPENDENT RIBONUCLEASE (EC 3.1.26.-) (2-5A-DEPENDENT RNAASE) (RNASE L) (RIBONUCLEASE 4) (FRAGMENT).//3.9e-07:113:31//MUS MUSCULUS (MOUSE).//Q05921
F-NT2RP2000938//VOLTAGE-GATED POTASSIUM CHANNEL PROTEIN KV3.3 (KSHIIID).//0.026:59:45//RATTUS NORVEGICUS (RAT).//Q01956
F-NT2RP2000943//HYPOTHETICAL PROTEIN KIAA0079 (HA3543).//5.9e-18:161:42//HOMO SAPIENS (HUMAN).//P53992
F-NT2RP2000965//INNER CENTROMERE PROTEIN (INCENP).//0.062:156:25//GALLUS GALLUS (CHICKEN).//P53352
F-NT2RP2000970//EC PROTEIN HOMOLOG.//1.0:50:30//ARABIDOPSIS THALIANA (MOUSE-EAR CRESS).//P93746
F-NT2RP2000985//HYPOTHETICAL 96.8 KD PROTEIN IN SIS2-MTD1 INTERGENIC REGION.//2.5e-06:53:47//SACCHAROMYCES CEREVISIAE (BAKER'S YEAST).//P36159
F-NT2RP2000987//INSECT TOXIN 4 (INSECT TOXIN AAH IT4).//1.0:32:34//ANDROCTONUS AUSTRALIS HECTOR (SAHARA SCORPION).//P21150
F-NT2RP2001036//!!!! ALU SUBFAMILY SP WARNING ENTRY !!!!//1.2e-33:65:81//HOMO SAPIENS (HUMAN).//P39193
F-NT2RP2001044//HIRUSTASIN.//0.97:15:66//HIRUDO MEDICINALIS (MEDICINAL LEECH).//P80302
F-NT2RP2001056//!!!! ALU SUBFAMILY SQ WARNING ENTRY !!!!//1.0e-24:85:65//HOMO SAPIENS (HUMAN).//P39194
F-NT2RP2001065//BOWMAN-BIRK TYPE SEED TRYPSIN AND CHYMOTRYPSIN INHIBITOR (BTCI).//0.41:50:32//VIGNA UNGUICULATA (COWPEA).//P17734
F-NT2RP2001070//PROBABLE PYRIDOXAMINE 5'-PHOSPHATE OXIDASE (EC 1.4.3.5) (PNP/PMP OXIDASE) (FPRA PROTEIN).//6.2e-18:64:48//MYXOCOCCUS XANTHUS.//P21159
F-NT2RP2001081//SYNAPTOTAGMIN IV.//7.8e-16:94:46//RATTUS NORVEGICUS (RAT).//P50232
F-NT2RP2001094//METALLOTHIONEIN-I (MT-I).//1.0:24:33//RATTUS NORVEGICUS (RAT).//P02803
F-NT2RP2001119//!!!! ALU SUBFAMILY SX WARNING ENTRY !!!!//7.5e-11:61:63//HOMO SAPIENS (HUMAN).//P39195
F-NT2RP2001127//XE169 PROTEIN (SMCX PROTEIN) (FRAGMENTS).//1.0e-47:155:58//MUS MUSCULUS (MOUSE).//P41230
F-NT2RP2001137//COLLAGEN ALPHA 1(XII) CHAIN (FRAGMENTS).//0.10:68:39//BOS TAURUS (BOVINE).//P25508
F-NT2RP2001149//!!!! ALU SUBFAMILY J WARNING ENTRY!!!!//1.1e-13:81:59//HOMO SAPIENS (HUMAN).//P39188
F-NT2RP2001168//PROTEIN KINASE C SUBSTRATE 80 KD PROTEIN (FRAGMENTS).//0.0071:77:33//RATTUS NORVEGICUS (RAT).//P20468
F-NT2RP2001173//CYTOSKELETON-ASSOCIATED PROTEIN CKAPI (TUBULIN FOLDING COFACTOR B).//1.0:36:41//HOMO SAPIENS (HUMAN).//Q99426
F-NT2RP2001174//ZINC FINGER PROTEIN 137.//7.2e-11:65:43//HOMO SAPIENS (HUMAN).//P52743
F-NT2RP2001196//NADH-UBIQUINONE OXIDOREDUCTASE CHAIN 2 (EC 1.6.5.3).//1.0:95:26//CAPRA HIRCUS (GOAT).//Q36346
F-NT2RP2001218//HYPOTHETICAL 59.2 KD PROTEIN IN MOB1-SGA1 INTERGENIC REGION.//0.00024:80:23//SACCHAROMYCES CEREVISIAE (BAKER'S YEAST).//P40486
F-NT2RP2001226//RABPHILIN-3A (FRAGMENT).//4.6e-05:121:39//MUS MUSCULUS (MOUSE).//P47708
F-NT2RP2001233//ZINC FINGER PROTEIN ZFP-36 (FRAGMENT).//3.2e-61:153:56//HOMO SAPIENS (HUMAN).//P16415
F-NT2RP2001245//SYNAPTONEMAL COMPLEX PROTEIN 1 (SCP-1 PROTEIN).//4.9e-05:230:21//HOMO SAPIENS (HUMAN).//Q15431
F-NT2RP2001268//HOMEOBOX PROTEIN CEH-32.//0.23:159:25//CAENORHABDITIS ELEGANS.//Q23175
F-NT2RP2001277
F-NT2RP2001290//BETA-SOLUBLE NSF ATTACHMENT PROTEIN (SNAP-BETA) (SNAP-ALPHA HOMOLOG) (BRAIN PROTEIN I47) (FRAGMENT).//1.0e-86:131:97//MUS MUSCULUS (MOUSE).//P28663
F-NT2RP2001295
F-NT2RP2001312//N-ACETYLGLUCOSAMINE-6-SULFATASE PRECURSOR (EC 3.1.6.14) (G6S) (GLUCOSAMINE-6-SULFATASE).//0.64:80:33//CAPRA HIRCUS (GOAT).//P50426
F-NT2RP2001327//TUMOR NECROSIS FACTOR, ALPHA-INDUCED PROTEIN 1, ENDOTHELIAL (B12 PROTEIN).//1.0e-36:118:65//HOMO SAPIENS (HUMAN).//Q13829
F-NT2RP2001328//PROBABLE E5 PROTEIN.//1.0:46:41//HUMAN PAPILLOMAVIRUS TYPE 33.//P06426
F-NT2RP2001347//!!!! ALU SUBFAMILY SP WARNING ENTRY !!!!//4.5e-19:66:62//HOMO SAPIENS (HUMAN).//P39193
F-NT2RP2001366//SPERM-SPECIFIC PROTEIN PHI-1.//0.66:55:32//MYTILUS EDULIS (BLUE MUSSEL).//Q04621
F-NT2RP2001378//VOLTAGE-GATED POTASSIUM CHANNEL PROTEIN KV3.3 (KSHIIID) (FRAGMENT).//0.060:78:33//HOMO SAPIENS (HUMAN).//Q14003
F-NT2RP2001381//26S PROTEASE REGULATORY SUBUNIT 8 (SUG1 HOMOLOG) (XSUG1).//1.0:167:26//XENOPUS LAEVIS (AFRICAN CLAWED FROG).//P46470
F-NT2RP2001392//KERATIN, HIGH-SULFUR MATRIX PROTEIN, IIIA3.//0.0080:82:32//OVIS ARIES (SHEEP).//P02441
F-NT2RP2001394//POLYHOMEOTIC-PROXIMAL CHROMATIN PROTEIN.//0.024:39:53//DROSOPHILA MELANOGASTER (FRUIT FLY).//P39769
F-NT2RP2001397//G2/MITOTIC-SPECIFIC CYCLIN B2.//1.4e-46:125:78//MESOCRICETUS AURATUS (GOLDEN HAMSTER).//P37883
F-NT2RP2001420//SALIVARY PROLINE-RICH PROTEIN PRECURSOR (CLONES CP3, CP4 AND CP5) [CONTAINS: BASIC PEPTIDE IB-6; PEPTIDE P-H].//0.00018:113:38//HOMO SAPIENS (HUMAN).//P04280
F-NT2RP2001423//HYPOTHETICAL 9.4 KD PROTEIN IN GP31-CD INTERGENIC REGION (ORF A).//0.90:23:43//BACTERIOPHAGE T4.//P17307
F-NT2RP2001427//!!!! ALU SUBFAMILY J WARNING ENTRY !!!!//2.2e-11:38:68//HOMO SAPIENS (HUMAN).//P39188
F-NT2RP2001436//DYNEIN LIGHT INTERMEDIATE CHAIN 2, CYTOSOLIC (LIC53/55) (LIC-2).//0.25:124:28//RATTUS NORVEGICUS (RAT).//Q62698
F-NT2RP2001440//14-3-3 PROTEIN GAMMA (PROTEIN KINASE C INHIBITOR PROTEIN-1) (KCIP-1).//4.8e-62:145:90//RATTUS NORVEGICUS (RAT).//P35214
F-NT2RP2001445
F-NT2RP2001449//CLEAVAGE AND POLYADENYLATION SPECIFICITY FACTOR, 100 KD SUBUNIT (CPSF 100 KD SUBUNIT).//9.5e-118:226:95//BOS TAURUS (BOVINE).//Q10568
F-NT2RP2001450
F-NT2RP2001467//SHORT NEUROTOXIN 1 (TOXIN V-II-1).//1.0:25:40//BUNGARUS FASCIATUS (BANDED KRAIT).//P10808
F-NT2RP2001506
F-NT2RP2001511//HYPOTHETICAL 115.4 KD PROTEIN ZK757.3 IN CHROMOSOME III.//0.49:124:29//CAENORHABDITIS ELEGANS.//P34681
F-NT2RP2001520//VITAMIN D-DEPENDENT CALCIUM-BINDING PROTEIN, INTESTINAL (CABP) (CALBINDIN D9K).//0.035:71:33//HOMO SAPIENS (HUMAN).//P29377
F-NT2RP2001526
F-NT2RP2001536//METALLOTHIONEIN-I (MT-1).//1.0:19:42//COLUMBA LIVIA (DOMESTIC PIGEON).//P15786
F-NT2RP2001560//CUTICLE COLLAGEN 12 PRECURSOR.//0.0018:144:35//CAENORHABDITIS ELEGANS.//P20630
F-NT2RP2001569//!!!! ALU SUBFAMILY SQ WARNING ENTRY !!!!//2.7e-31:102:67//HOMO SAPIENS (HUMAN).//P39194
F-NT2RP2001576//SMP3 PROTEIN.//0.00016:75:36//SACCHAROMYCES CEREVISIAE (BAKER'S YEAST).//Q04174
F-NT2RP2001581//TRANSMEMBRANE PROTEIN SEX PRECURSOR.//0.040:46:36//HOMO SAPIENS (HUMAN).//P51805
F-NT2RP2001597//PROBABLE E4 PROTEIN.//0.00042:113:34//HUMAN PAPILLOMAVIRUS TYPE 5.//P06924
F-NT2RP2001601
F-NT2RP2001613//HOMEOBOX PROTEIN SAX-1 (CHOX-3) (FRAGMENT).//0.14:59:32//GALLUS GALLUS (CHICKEN).//P19601
F-NT2RP2001628//A-AGGLUTININ ATTACHMENT SUBUNIT PRECURSOR.//0.056:140:33//SACCHAROMYCES CEREVISIAE (BAKER'S
YEAST).//P32323
F-NT2RP2001634//ALPHA-CATENIN.//7.1e-12:152:35//DROSOPHILA MELANOGASTER (FRUIT FLY).//P35220
F-NT2RP2001660//HYPOTHETICAL 80.4 KD PROTEIN IN SMC3-MRPL8 INTERGENIC REGION.//0.43:119:26//SACCHAROMYCES CEREVISIAE (BAKER'S YEAST).//P40358
F-NT2RP2001663//ALPHA ENOLASE (EC 4.2.1.11) (2-PHOSPHO-D-GLYCERATE HYDRO-LYASE) (NON- NEURAL ENOLASE) (NNE) (PHOSPHOPYRUVATE HYDRATASE).//1.2e-26:126:56//HOMO SAPIENS (HUMAN).//P06733
F-NT2RP2001675//HYPOTHETICAL 107.7 KD PROTEIN IN RPSO 5'REGION (ORF1).//0.25:148:25//CAMPYLOBACTER JEJUNI.//Q46089
F-NT2RP2001677//ACIDIC PROLINE-RICH PROTEIN PRECURSOR (CLONE PRP25) (FRAGMENT).//0.010:101:31//RATTUS NORVEGICUS (RAT).//P10164
F-NT2RP2001678//!!!! ALU SUBFAMILY J WARNING ENTRY !!!!//2.6e-18:83:61//HOMO SAPIENS (HUMAN).//P39188
F-NT2RP2001699//PROTEIN C14.//0:98:51:31//VACCINIA VIRUS (STRAIN COPENHAGEN).//P21045
F-NT2RP2001720//MEROZOITE SURFACE ANTIGEN 2 PRECURSOR (MSA-2) (ALLELIC FORM 1).//0.16:145:30//PLASMODIUM FALCIPARUM (ISOLATE CAMP / MALAYSIA).//Q99317
F-NT2RP2001721//MALE-SPECIFIC LETHAL-2 PROTEIN.//0.00090:48:39//DROSOPHILA MELANOGASTER (FRUIT FLY).//P50534
F-NT2RP2001740//ANNEXIN VII (SYNEXIN) (FRAGMENT).//0.50:43:25//BOS TAURUS (BOVINE).//P20072
F-NT2RP2001748//SALIVARY PROLINE-RICH PROTEIN PO (ALLELE K) [CONTAINS: PEPTIDE P-D] (FRAGMENT).//0.77:111:28//HOMO SAPIENS (HUMAN).//P10162
F-NT2RP2001762
F-NT2RP2001813//PHOTOSYSTEM I REACTION CENTRE SUBUNIT VIII (PSI-I).//1.0:22:40//PICEA ABIES (NORWAY SPRUCE) (PICEA EXCELSA).//O47040
F-NT2RP2001839//SCY1 PROTEIN.//6.8e-17:204:27//SACCHAROMYCES CEREVISIAE (BAKER'S YEAST).//P53009
F-NT2RP2001861//D15KZ1 PROTEIN (FRAGMENT).//0.31:56:39//MUS MUSCULUS (MOUSE).//Q61466
F-NT2RP2001869//CORNEODESMOSIN (S PROTEIN) (FRAGMENT).//0.97:78:30//SUS SCROFA (PIG).//O19084
F-NT2RP2001876//ALLOGRAFT INFLAMMATORY FACTOR-1 (AIF-1) (IONIZED CALCIUM BINDING ADAPTER MOLECULE 1).//3.5e-36:106:66//HOMO SAPIENS (HUMAN).//P55008
F-NT2RP2001883//CATHEPSIN L (EC 3.4.22.15).//0.95:29:41//OVIS ARIES (SHEEP).//Q10991
F-NT2RP2001898//TYPE II INOSITOL-1,4,5-TRISPHOSPHATE 5-PHOSPHATASE PRECURSOR (EC 3.1.3.56) (5PTASE) (FRAGMENT).//1.6e-84:185:88//HOMO SAPIENS (HUMAN).//P32019
F-NT2RP2001900//ACTIN-LIKE PROTEIN ARP5.//1.1e-17:180:34//SACCHAROMYCES CEREVISIAE (BAKER'S YEAST).//P53946
F-NT2RP2001907//HYPHAL WALL PROTEIN 1 (CELL ELONGATION PROTEIN 2).//0.13:108:27//CANDIDA ALBICANS (YEAST).//P46593
F-NT2RP2001926//HYPOTHETICAL 7.6 KD PROTEIN YCF33.//0.55:57:26//CYANOPHORA PARADOXA.//P48273
F-NT2RP2001936
F-NT2RP2001943//HYPOTHETICAL 57.7 KD PROTEIN IN AIP1-CTF13 INTERGENIC REGION.//1.8e-13:208:22//SACCHAROMYCES CEREVISIAE (BAKER'S YEAST).//Q04305
F-NT2RP2001946//HYPOTHETICAL 13.0 KD PROTEIN IN ALGR3 3'REGION.//0.59:76:28//PSEUDOMONAS AERUGINOSA.//P21485
F-NT2RP2001947//ZINC FINGER PROTEIN DAN (N03).//0.53:68:29//RATTUS NORVEGICUS (RAT).//Q06880
F-NT2RP2001969//CHLOROPLAST 30S RIBOSOMAL PROTEIN S18.//0.0015:52:34//CHLORELLA VULGARIS.//P56353
F-NT2RP2001976//DILUTE MYOSIN HEAVY CHAIN, NON-MUSCLE (MYOSIN 5A).//9.5e-07:201:22//MUS MUSCULUS (MOUSE).//Q99104
F-NT2RP2001985//PROLINE-RICH PROTEIN MP-2 PRECURSOR.//0.016:90:32//MUS MUSCULUS (MOUSE).//P05142
F-NT2RP2001991//SODIUM- AND CHLORIDE-DEPENDENT TRANSPORTER NTT73.//8.0e-14:47:76//RATTUS NORVEGICUS (RAT).//Q08469
F-NT2RP2002025//NG-CAM RELATED CELL ADHESION MOLECULE PRECURSOR (NR-CAM) (BRAVO).//2.9e-30:211:42//GALLUS GALLUS (CHICKEN).//P35331
F-NT2RP2002032//FLOCCULANT-ACTIVE PROTEINS MO2.1 AND MO2.2.//0.23:20:40//MORINGA OLEIFERA (HORSERADISH TREE) (MORINGA PTERYGOSPERMA).//P24303
F-NT2RP2002033//!!!! ALU SUBFAMILY SP WARNING ENTRY !!!!//0.88:27:62//HOMO SAPIENS (HUMAN).//P39193
F-NT2RP2002041
F-NT2RP2002046//MATING PROCESS PROTEIN MID2 (SERINE-RICH PROTEIN SMS1) (PROTEIN KINASE A INTERFERENCE PROTEIN).//1.0:85:31//SACCHAROMYCES CEREVISIAE (BAKER'S YEAST).//P36027
F-NT2RP2002047
F-NT2RP2002058//DOM34 INTERACTING PROTEIN 2.//9.4e-25:165:34//SACCHAROMYCES CEREVISIAE (BAKER'S YEAST).//Q12220
F-NT2RP2002066//TIGHT JUNCTION PROTEIN ZO-1 (TIGHT JUNCTION PROTEIN 1).//5.7e-12:108:41//HOMO SAPIENS (HUMAN).//Q07157
F-NT2RP2002070//CYTOCHROME C OXIDASE POLYPEPTIDE II (EC 1.9.3.1) (FRAGMENT).//0.88:28:50//ASTERINA PECTINIFERA (STARFISH).//P11958
F-NT2RP2002076//TRP-ASP REPEATS CONTAINING PROTEIN RBA-2.//0.0031:124:27//CAENORHABDITIS ELEGANS.//P90916
F-NT2RP2002078//KERATIN, GLYCINE/TYROSINE-RICH OF HAIR.//0.82:30:40//OVIS ARIES (SHEEP).//Q02958
F-NT2RP2002079//OUTER DENSE FIBER PROTEIN.//0.34:41:39//HOMO SAPIENS (HUMAN).//Q14990
F-NT2RP2002099//HETEROGENOUS NUCLEAR RIBONUCLEOPROTEIN U (HNRNP U).//5.2e-08:81:48//HOMO SAPIENS (HUMAN).//Q00839
F-NT2RP2002105//COLLAGEN 1(X) CHAIN PRECURSOR.//0.0012:100:34//BOS TAURUS (BOVINE).//P23206
F-NT2RP2002124//EARLY GROWTH RESPONSE PROTEIN 1 (EGR-1) (KROX24) (TRANSCRIPTION FACTOR ETR103) (ZINC FINGER PROTEIN 225) (AT225).//0.74:72:31//HOMO SAPIENS (HUMAN).//P18146
F-NT2RP2002137//NEUROTOXIN B-II.//1.0:27:44//CEREBRATULUS LACTEUS (MILKY RIBBON WORM).//P01526
F-NT2RP2002154//GALECTIN-3 (GALACTOSE-SPECIFIC LECTIN 3) (MAC-2 ANTIGEN) (IGE-BINDING PROTEIN) (35 KD LECTIN) (CARBOHYDRATE BINDING PROTEIN 35) (CBP 35) (LAMININ-BINDING PROTEIN) (LECTIN L-29) (L-34 GALACTOSIDE-BINDING LECTIN).//0.0029:112:34//MUS MUSCULUS (MOUSE).//P16110
F-NT2RP2002172
F-NT2RP2002185//UBIQUITIN-LIKE PROTEIN DSK2.//1.8e-07:87:40//SACCHAROMYCES CEREVISIAE (BAKER'S YEAST).//P48510
F-NT2RP2002192
F-NT2RP2002193//CUTICLE COLLAGEN 40.//0.0062:70:37//CAENORHABDITIS ELEGANS.//P34804
F-NT2RP2002208//PEROXISOME ASSEMBLY PROTEIN PEX10 (PEROXIN-10).//0.00011:45:40//HOMO SAPIENS (HUMAN).//060683
F-NT2RP2002219
F-NT2RP2002231//V-TYPE SODIUM ATP SYNTHASE SUBUNIT E (EC 3.6.1.34) (NA(+)-TRANSLOCATING ATPASE SUBUNIT E).//1.0:68:32//ENTEROCOCCUS HIRAE.//P43436
F-NT2RP2002235//INFECTED CELL PROTEIN ICP34.5 (NEUROVIRULENCE FACTOR ICP34.5).//0.0022:66:45//HERPES SIMPLEX VIRUS (TYPE 1 / STRAIN CVG-2).//P37318
F-NT2RP2002252//DNA-DIRECTED RNA POLYMERASE II LARGEST SUBUNIT (EC 2.7.7.6) (RPB1) (FRAGMENT).//0.071:110:31//CRICETULUS GRISEUS (CHINESE HAMSTER).//P11414
F-NT2RP2002256//CYTOCHROME P450 26 (EC 1.14.-.-) (RETINOIC ACID-METABOLIZING CYTOCHROME) (P450RAI) (RETINOIC ACID 4-HYDROXYLASE).//3.1e-31:75:84//MUS MUSCULUS (MOUSE).//O55127
F-NT2RP2002259//L-MYC-1 PROTO-ONCOGENE PROTEIN.//1.9e-17:41:90//HOMO SAPIENS (HUMAN).//P12524
F-NT2RP2002270//HYPOTHETICAL 26.0 KD PROTEIN IN CYB5-LEU4 INTERGENIC REGION.//2.1e-27:164:36//SACCHAROMYCES CEREVISIAE (BAKER'S YEAST).//P53930
F-NT2RP2002292//IMMEDIATE-EARLY PROTEIN RSP40.//0.018:107:23//PSEUDORABIES VIRUS (STRAIN KAPLAN) (PRV).//P24827
F-NT2RP2002312//PHOSPHATIDATE CYTIDYLYLTRANSFERASE (EC 2.7.7.41) (CDP-DIGLYCERIDE SYNTHETASE) (CDP-DIGLYCERIDE PYROPHOSPHORYLASE) (CDP-DIACYLGLYCEROL SYNTHASE) (CDS) (CTP:PHOSPHATIDATE CYTIDYLYLTRANSFERASE) (CDP-DAG SYNTHASE).//1.4e-52:174:55//HOMO SAPIENS (HUMAN).//Q92903
F-NT2RP2002316//HISTONE H1.C6/H1.C9.//1.0:40:40//TRYPANOSOMA CRUZI.//P40269
F-NT2RP2002325//PEROXISOMAL MEMBRANE PROTEIN PMP30A (PMP31) (PEROXIN 11A).//2.2e-06:145:26//CANDIDA BOIDINII (YEAST).//Q00316
F-NT2RP2002333//HYPOTHETICAL 39.1 KD PROTEIN IN RNPB-SOHA INTERGENIC REGION (ORF 3).//0.30:86:32//ESCHERICHIA COLI.//P23524
F-NT2RP2002373//SYNAPSINS IA AND IB.//0.080:145:31//BOS TAURUS (BOVINE).//P17599
F-NT2RP2002385//ENV POLYPROTEIN PRECURSOR (COAT POLYPROTEIN) [CONTAINS: KNOB PROTEIN GP70; SPIKE PROTEIN P15E; R PROTEIN].//0.021:66:28//MINK CELL FOCUS-FORMING MURINE LEUKEMIA VIRUS (ISOLATE CI-3).//P03388
F-NT2RP2002394
F-NT2RP2002408//COLLAGEN ALPHA 1(I) CHAIN (FRAGMENTS).//0.00030:107:37//BOS TAURUS (BOVINE).//P02453
F-NT2RP2002426
F-NT2RP2002439//CIRCUMSPOROZOITE PROTEIN PRECURSOR (CS).//0.00032:79:32//PLASMODIUM BERGHEI (STRAIN ANKA).//P23093
F-NT2RP2002442//HESA PROTEIN.//6.0e-16:163:30//PLECTONEMA BORYANUM.//P46037
F-NT2RP2002457
F-NT2RP2002464//HYPOTHETICAL 60.7 KD PROTEIN C56F8.17C IN CHROMOSOME I.//9.3e-18:165:32//SCHIZOSACCHAROMYCES POMBE (FISSION YEAST).//Q10264
F-NT2RP2002475//CYSTEINE-RICH HEART PROTEIN (HCRHP).//0.91:45:35//HOMO SAPIENS (HUMAN).//P50238
F-NT2RP2002479//ATP-BINDING CASSETTE TRANSPORTER 7 PRECURSOR (ABC TRANSPORTER 7 PROTEIN).//6.8e-96:186:94//HOMO SAPIENS (HUMAN).//O75027
F-NT2RP2002498//HYPOTHETICAL MERCURIC RESISTANCE PROTEIN MERC.//0.65:37:45//PSEUDOMONAS AERUGINOSA.//P04139
F-NT2RP2002503//ZINC FINGER PROTEIN 45 (BRC1744).//1.3e-31:124:59//HOMO SAPIENS (HUMAN).//Q02386
F-NT2RP2002504//NUCLEAR PORE COMPLEX PROTEIN NUP155 (NUCLEOPORIN NUP155) (155 KD NUCLEOPORIN) (P140).//1.2e-123:240:92//RATTUS NORVEGICUS (RAT).//P37199
F-NT2RP2002520//ACIDIC PROLINE-RICH PROTEIN HP43A PRECURSOR.//0.94:83:28//MESOCRICETUS AURATUS (GOLDEN HAMSTER).//P06680
F-NT2RP2002537//HYPOTHETICAL 55.1 KD PROTEIN B0416.5 IN CHROMOSOME X.//4.0e-10:194:23//CAENORHABDITIS ELEGANS.//Q11073
F-NT2RP2002546
F-NT2RP2002549//G2/MITOTIC-SPECIFIC CYCLIN C13-1 (A-LIKE CYCLIN) (FRAGMENT).//0.98:65:30//DAUCUS CAROTA (CARROT).//P25010
F-NT2RP2002591//ZINC FINGER PROTEIN 84 (ZINC FINGER PROTEIN HPF2).//2.6e-19:60:61//HOMO SAPIENS (HUMAN).//P51523
F-NT2RP2002595//ANNEXIN VII (SYNEXIN).//1.2e-15:121:49//XENOPUS LAEVIS (AFRICAN CLAWED FROG).//Q92125
F-NT2RP2002606//PROTEIN TRANSPORT PROTEIN SEC2.//0.00034:98:31//SACCHAROMYCES CEREVISIAE (BAKER'S YEAST).//P17065
F-NT2RP2002609//HYPOTHETICAL 52.0 KD PROTEIN IN CLB6-SPT6 INTERGENIC REGION.//0.00022:79:39//SACCHAROMYCES CEREVISIAE (BAKER'S YEAST).//P53264
F-NT2RP2002618//PROTEIN ARGININE N-METHYLTRANSFERASE 1 (EC 2.1.1.-).//6.2e-37:180:44//RATTUS NORVEGICUS (RAT).//Q63009
F-NT2RP2002621//NADH-UBIQUINONE OXIDOREDUCTASE CHAIN 5 (EC 1.6.5.3) (FRAGMENT).//0.98:37:35//LEMUR CATTA (RING-TAILED LEMUR).//Q34879
F-NT2RP2002643//INFECTED CELL PROTEIN ICP34.5 (NEUROVIRULENCE FACTOR ICP34.5).//0.042:77:32//HERPES SIMPLEX VIRUS (TYPE 1 / STRAIN MGH-10).//P37319
F-NT2RP2002672//PROTEIN Q300.//0.0018:41:43//MUS MUSCULUS (MOUSE).//Q02722
F-NT2RP2002701//HYPOTHETICAL 72.5 KD PROTEIN C2F7.10 IN CHROMOSOME I.//3.6e-17:100:42//SCHIZOSACCHAROMYCES POMBE (FISSION YEAST).//Q09701
F-NT2RP2002706//IMMEDIATE-EARLY PROTEIN IE180.//0.00027:139:33//PSEUDORABIES VIRUS (STRAIN KAPLAN) (PRV).//P33479
F-NT2RP2002710//SH3-BINDING PROTEIN 3BP-1.//6.9e-09:96:40//MUS MUSCULUS (MOUSE).//P55194
F-NT2RP2002727//TUBERIN (TUBEROUS SCLEROSIS 2 HOMOLOG PROTEIN).//3.6e-20:160:36//RATTUS NORVEGICUS (RAT).//P49816
F-NT2RP2002736
F-NT2RP2002740
F-NT2RP2002741//RHO1 GDP-GTP EXCHANGE PROTEIN 2.//2.0e-07:178:28//SACCHAROMYCES CEREVISIAE (BAKER'S YEAST).//P51862
F-NT2RP2002750//!!!! ALU SUBFAMILY SB2 WARNING ENTRY !!!!//1.6e-09:43:72//HOMO SAPIENS (HUMAN).//P39191
F-NT2RP2002752//LOW CALCIUM RESPONSE LOCUS PROTEIN T.//0.95:33:39//YERSINIA PSEUDOTUBERCULOSIS.//Q00932
F-NT2RP2002753//ENDOGLUCANASE EG-1 PRECURSOR (EC 3.2.1.4) (ENDO-1,4-BETA-GLUCANASE) (CELLULASE).//0.71:78:33//TRICHODERMA LONGIBRACHIATUM.//Q12714
F-NT2RP2002769//50 KD SPICULE MATRIX PROTEIN PRECURSOR.//0.44:76:32//STRONGYLOCENTROTUS PURPURATUS (PURPLE SEA URCHIN).//P11994
F-NT2RP2002778
F-NT2RP2002800//CRAMBIN.//0.99:20:50//CRAMBE ABYSSINICA (ABYSSINIAN CRAMBE).//P01542
F-NT2RP2002839//SALIVARY PROLINE-RICH PROTEIN PRECURSOR (CLONE CP7) [CONTAINS: BASIC PEPTIDE P-F] (FRAGMENT).//0.010:87:31//HOMO SAPIENS (HUMAN).//P02812
F-NT2RP2002857//ACIDIC PROLINE-RICH PROTEIN PRECURSOR (CLONE PRP33).//0.00018:57:45//RATTUS NORVEGICUS (RAT).//P04474
F-NT2RP2002862//HYPOTHETlCAL 27.1 KD PROTEIN UFD4-CAP1 INTERGENIC REGION.//7.2e-27:140:40//SACCHAROMYCES CEREVISIAE (BAKER'S YEAST).//P33201
F-NT2RP2002880//DNA REPAIR PROTEIN RAD32.//0.83:67:28//SCHIZOSACCHAROMYCES POMBE (FISSION YEAST).//Q09683
F-NT2RP2002891//HOMEOBOX PROTEIN DLX-2 (DLX-5) (FRAGMENT).//0.99:70:24//RATTUS NORVEGICUS (RAT).//Q64204
F-NT2RP2002925//ALPHA-1D ADRENERGIC RECEPTOR (ALPHA 1D-ADRENOCEPTOR) (ALPHA-1A ADRENERGIC RECEPTOR).//0.31:48:43//HOMO SAPIENS (HUMAN).//P25100
F-NT2RP2002928//CELL DIVISION CONTROL PROTEIN 40.//2.8e-26:142:42//SACCHAROMYCES CEREVISIAE (BAKER'S YEAST).//P40968
F-NT2RP2002929//HYPOTHETICAL 46.2 KD TRP-ASP REPEATS CONTAINING PROTEIN D2013.2 IN CHROMOSOME II.//2.0e-31:186:35//CAENORHABDITIS ELEGANS.//Q18964
F-NT2RP2002939//ADENYLATE CYCLASE, TYPE V (EC 4.6.1.1) (ATP PYROPHOSPHATE-LYASE) (CA(2+)-INHIBITABLE ADENYLYL CYCLASE).//0.0022:98:39//CANIS FAMILIARIS (DOG).//P30803
F-NT2RP2002954//U2 SMALL NUCLEAR RIBONUCLEOPROTEIN A' (U2 SNRNP-A').//0.0019:107:30//ARABIDOPSIS THALIANA (MOUSE-EAR CRESS).//P43333
F-NT2RP2002959//UBIQUITIN-CONJUGATING ENZYME E2-17 KD 2 (EC 6.3.2.19) (UBIQUITIN- PROTEIN LIGASE) (UBIQUITIN CARRIER PROTEIN) (E2(17)KB 2).//2.8e-11:33:81//HOMO SAPIENS (HUMAN), MUS MUSCULUS (MOUSE), RATTUS NORVEGICUS (RAT), AND XENOPUS LAEVIS (AFRICAN CLAWED FROG).//P51669
F-NT2RP2002979
F-NT2RP2002980//30S RIBOSOMAL PROTEIN S10.//1.1e-09:98:36//MYCOPLASMA CAPRICOLUM.//P10129
F-NT2RP2002986//RING CANAL PROTEIN (KELCH PROTEIN).//1.1e-19:141:39//DROSOPHILA MELANOGASTER (FRUIT FLY).//Q04652
F-NT2RP2002987//!!!! ALU SUBFAMILY SC WARNING ENTRY !!!!//1.3e-07:78:47//HOMO SAPIENS (HUMAN).//P39192
F-NT2RP2002993//DNA-DIRECTED RNA POLYMERASE I 135 KD POLYPEPTIDE (EC 2.7.7.6) (RNA POLYMERASE I SUBUNIT 2) (RPA135) (RNA POLYMERASE I 127 KD SUBUNIT).//8.0e-77:165:85//RATTUS NORVEGICUS (RAT).//O54888
F-NT2RP2003000//!!!! ALU SUBFAMILY SQ WARNING ENTRY !!!!//2.8e-19:62:64//HOMO SAPIENS (HUMAN).//P39194
F-NT2RP2003034//HYPOTHETICAL PROTEIN HI1458.//1.0:42:35//HAEMOPHILUS INFLUENZAE.//P44204
F-NT2RP2003073//!!!! ALU SUBFAMILY SB WARNING ENTRY !!!!//0.0051:16:87//HOMO SAPIENS (HUMAN).//P39189
F-NT2RP2003099
F-NT2RP2003108//BASIC PROLINE-RICH PEPTIDE IB-1.//0.84:47:34//HOMO SAPIENS (HUMAN).//P04281
F-NT2RP2003117
F-NT2RP2003121//HYPOTHETICAL 96.7 KD PROTEIN IN STE2-FRS2 INTERGENIC REGION.//9.0e-08:99:29//SACCHAROMYCES CEREVISIAE (BAKER'S YEAST).//P43572
F-NT2RP2003125//TRANSCRIPTION REGULATOR PROTEIN BACH2 (BTB AND CNC HOMOLOG 2).//9.2e-08:134:28//MUS MUSCULUS (MOUSE).//P97303
F-NT2RP2003129
F-NT2RP2003137//UBIQUITIN.//3.4e-06:70:30//NEUROSPORA CRASSA.//P13117
F-NT2RP2003157//HYPOTHETICAL 37.0 KD PROTEIN B0495.8 IN CHROMOSOME II.//7.8e-13:84:40//CAENORHABDITIS ELEGANS.//Q09217
F-NT2RP2003158//26S PROTEASOME REGULATORY SUBUNIT S3 (PROTEASOME SUBUNIT P58).//3.1e-65:155:84//HOMO SAPIENS (HUMAN).//O43242
F-NT2RP2003161//PROLINE-RICH PROTEIN MP-2 PRECURSOR.//0.0011:59:42//MUS MUSCULUS (MOUSE).//P05142
F-NT2RP2003164//ZYXIN.//0.0037:85:36//MUS MUSCULUS (MOUSE).//Q62523
F-NT2RP2003165//!!!! ALU SUBFAMILY SQ WARNING ENTRY !!!!//1.2e-24:77:64//HOMO SAPIENS (HUMAN).//P39194
F-NT2RP2003177//MALE SPECIFIC SPERM PROTEIN MST84DB.//0.55:38:39//DROSOPHILA MELANOGASTER (FRUIT FLY).//Q01643
F-NT2RP2003194//HYPOTHETICAL 12.5 KD PROTEIN ZK637.2 IN CHROMOSOME III.//2.3e-14:87:37//CAENORHABDITIS ELEGANS.//P30629
F-NT2RP2003206//NADH-UBIQUINONE OXIDOREDUCTASE CHAIN 3 (EC 1.6.5.3).//1.0:100:28//DIDELPHIS MARSUPIALIS VIRGINIANA (NORTH AMERICAN OPOSSUM).//P41306
F-NT2RP2003228//DNA REPLICATION LICENSING FACTOR MCM4 (CDC21 HOMOLOG) (P1-CDC21).//9.3e-82:211:81//HOMO SAPIENS (HUMAN).//P33991
F-NT2RP2003230//SEC14 CYTOSOLIC FACTOR (PHOSPHATIDYLINOSITOL/PHOSPHATIDYLCHOLINE TRANSFER PROTEIN) (PI/PC TP).//1.0:51:31//CANDIDA GLABRATA (YEAST) (TORULOPSIS GLABRATA).//P53989
F-NT2RP2003237//!!!! ALU SUBFAMILY SQ WARNING ENTRY !!!!//5.1e-44:66:84//HOMO SAPIENS (HUMAN).//P39194
F-NT2RP2003243//M PROTEIN, SEROTYPE 5 PRECURSOR.//0.027:204:23//STREPTOCOCCUS PYOGENES.//P02977
F-NT2RP2003265//BP4A PROTEIN.//0.95:35:34//BRASSICA NAPUS (RAPE).//P41505
F-NT2RP2003272//ANTER-SPECIFIC PROLINE-RICH PROTEIN APG (PROTEIN CEX) (FRAGMENT).//5.5e-06:78:35//BRASSICA NAPUS (RAPE).//P40603
F-NT2RP2003277//NAM7 PROTEIN (NONSENSE-MEDIATED MRNA DECAY PROTEIN 1) (UP-FRAMESHIFT SUPPRESSOR 1).//1.9e-19:145:43//SACCHAROMYCES CEREVISIAE (BAKER'S YEAST).//P30771
F-NT2RP2003280
F-NT2RP2003286//RNA 3'-TERMINAL PHOSPHATE CYCLASE (EC 6.5.1.4) (RNA-3'-PHOSPHATE CYCLASE) (RNA CYCLASE).//2.1e-32:137:42//SACCHAROMYCES CEREVISIAE (BAKER'S YEAST).//Q08096
F-NT2RP2003293//ZINC FINGER PROTEIN 83 (ZINC FINGER PROTEIN HPF1).//7.7e-12:175:33//HOMO SAPIENS (HUMAN).//P51522
F-NT2RP2003295//PTB-ASSOCIATED SPLICING FACTOR (PSF).//0.055:44:45//HOMO SAPIENS (HUMAN).//P23246
F-NT2RP2003297
F-NT2RP2003307//KINESIN LIGHT CHAIN (KLC).//2.0e-18:87:49//RATTUS NORVEGICUS (RAT).//P37285
F-NT2RP2003308//CROOKED NECK PROTEIN.//2.1e-91:244:67//DROSOPHILA MELANOGASTER (FRUIT FLY).//P17886
F-NT2RP2003329//HYPOTHETICAL 54.9 KD PROTEIN C02F5.7 IN CHROMOSOME III.//5.8e-57:186:55//CAENORHABDITIS ELEGANS.//P34284
F-NT2RP2003339//SHORT NEUROTOXIN 1 (NEUROTOXIN ALPHA).//0.98:11:72//DENDROASPIS POLYLEPIS POLYLEPIS (BLACK MAMBA).//P01416
F-NT2RP2003347//60S RIBOSOMAL PROTEIN L38.//0.83:42:33//OSTERTAGIA OSTERTAGI.//O61570
F-NT2RP2003367//SYNERGISTIC-TYPE VENOM PROTEIN C9S3, CHAIN 1.//1.0:37:35//DENDROASPIS ANGUSTICEPS (EASTERN GREEN MAMBA).//P01408
F-NT2RP2003391//MRNA TRANSPORT REGULATOR MTR10.//3.3e-11:229:24//SACCHAROMYCES CEREVISIAE (BAKER'S YEAST).//Q99189
F-NT2RP2003393//PROTOCHLOROPHYLLIDE REDUCTASE CHLB SUBUNIT (EC 1.3.1.33) (NADPH- PROTOCHLOROPHYLLIDE OXIDOREDUCTASE CHLB SUBUNIT) (FRAGMENT).//0.94:29:34//ARAUCARIA HETEROPHYLLA.//P37843
F-NT2RP2003394
F-NT2RP2003401//60 KD CHAPERONIN (PROTEIN CPN60) (GROEL PROTEIN).//0.95:125:28//THERMUS AQUATICUS (SUBSP. THERMOPHILUS).//P45746
F-NT2RP2003433//PROTEIN TRANSPORT PROTEIN SEC61 ALPHA SUBUNIT.//9.8e-78:178:84//RATTUS NORVEGICUS (RAT).//P38378
F-NT2RP2003445
F-NT2RP2003446//HYPOTHETICAL PROTEIN E-115.//0.00030:106:33//HUMAN ADENOVIRUS TYPE 2.//P03290
F-NT2RP2003456//PHOTOSYSTEM II REACTION CENTRE M PROTEIN.//1.0:27:51//MARCHANTIA POLYMORPHA (LIVERWORT).//P12168
F-NT2RP2003466//LINOLEOYL-COA DESATURASE (EC 1.14.99.25) (DELTA(6)-DESATURASE).//6.7e-06:108:32//SYNECHOCYSTIS SP. (STRAIN PCC 6803).//Q08871
F-NT2RP2003480//TRANSCRIPTION FACTOR BF-2 (BRAIN FACTOR 2) (BF2) (CBF-2) (T-14-6).//7.2e-15:38:50//GALLUS GALLUS (CHICKEN).//Q98937
F-NT2RP2003499//5E5 ANTIGEN.//0.090:114:32//RATTUS NORVEGICUS (RAT).//Q63003
F-NT2RP2003506//NADPH-CYTOCHROME P450 REDUCTASE (EC 1.6.2.4) (CPR).//2.0e-11:91:43//SUS SCROFA (PIG).//P04175
F-NT2RP2003511//PARAMYOSIN, SHORT FORM (MIMIPARAMYOSIN).//0.0020:108:25//DROSOPHILA MELANOGASTER (FRUIT FLY).//P35416
F-NT2RP2003513//PTB-ASSOCIATED SPLICING FACTOR (PSF).//1.2e-05:96:36//HOMO SAPIENS (HUMAN).//P23246
F-NT2RP2003517//HYPOTHETICAL 12.9 KD PROTEIN CY49.27.//0.0059:22:31//MYCOBACTERIUM TUBERCULOSIS.//Q10696
F-NT2RP2003522//HYPOTHETICAL 10.0 KD PROTEIN.//1.0:65:30//THERMOPROTEUS TENAX VIRUS 1 (STRAIN KRA1) (TTV1).//P19283
F-NT2RP2003533//LINE-1 REVERSE TRANSCRIPTASE HOMOLOG.//8.7e-18:94:54//HOMO SAPIENS (HUMAN).//P08547
F-NT2RP2003543//SYNAPSINS IA AND IB.//0.045:101:35//RATTUS NORVEGICUS (RAT).//P09951
F-NT2RP2003559//ITBA2 PROTEIN (DXS9879E).//0.98:37:37//HOMO SAPIENS (HUMAN).//Q14657
F-NT2RP2003564//52 KD RO PROTEIN (SJOGREN SYNDROME TYPE A ANTIGEN (SS-A)) (RO(SS-A)).//6.4e-35:175:44//HOMO SAPIENS (HUMAN).//P19474
F-NT2RP2003567//HYPOTHETICAL 11.2 KD PROTEIN T18D3.7 IN CHROMOSOME X.//0.72:82:34//CAENORHABDITIS ELEGANS.//Q22544
F-NT2RP2003581//HOMEOBOX PROTEIN OTX1.//0.90:61:37//MUS MUSCULUS (MOUSE).//P80205
F-NT2RP2003596//ELONGATION FACTOR P (EF-P).//0.83:61:32//MYCOPLASMA GENITALIUM.//P47272
F-NT2RP2003604//ALPHA-CATENIN.//1.5e-11:152:33//DROSOPHILA MELANOGASTER (FRUIT FLY).//P35220
F-NT2RP2003629//PHOSPHOLIPASE A2 ALPHA (EC 3.1.1.4) (PHOSPHATIDYLCHOLINE 2-ACYLHYDROLASE).//0.97:85:27//CROTALUS ADAMANTEUS (EASTERN DIAMONDBACK RATTLESNAKE).//P00623
F-NT2RP2003643//ACYLNEURAMINATE CYTIDYLYLTRANSFERASE (EC 2.7.7.43) (CMP-N- ACETYLNEURAMINIC ACID SYNTHETASE) (CMP-NEUNAC SYNTHETASE) (CMP-SIALIC ACID SYNTHETASE).//3.9e-12:84:40//NEISSERIA MENINGITIDIS.//Q57385
F-NT2RP2003668//!!!! ALU-SUBFAMILY SX WARNING ENTRY !!!!//5.0e-33:74:81//HOMO SAPIENS (HUMAN).//P39195
F-NT2RP2003687//!!!! ALU SUBFAMILY J WARNING ENTRY !!!!//5.7e-05:40:67//HOMO SAPIENS (HUMAN).//P39188
F-NT2RP2003691//!!!! ALU SUBFAMILY SQ WARNING ENTRY !!!!//2.5e-37:56:67//HOMO SAPIENS (HUMAN).//P39194
F-NT2RP2003702//HYPOTHETICAL OXIDOREDUCTASE IN INLA 5'REGION (EC 1.-.-.-) (ORFA).//1.3e-07:98:37//LISTERIA MONOCYTOGENES.//P25145
F-NT2RP2003704//GAMMA-GLUTAMYLTRANSPEPTIDASE 5 PRECURSOR (EC 2.3.2.2) (GAMMA- GLUTAMYLTRANSFERASE 5) (GGT-REL).//0.66:23:52//HOMO SAPIENS (HUMAN).//P36269
F-NT2RP2003706//GLUTAMYL AMINOPEPTIDASE (EC 3.4.11.7) (EAP) (AMINOPEPTIDASE A) (APA) (DIFFERENTIATION ANTIGEN GP160).//1.2e-22:187:35//HOMO SAPIENS (HUMAN).//Q07075
F-NT2RP2003713//UBIQUITIN CARBOXYL-TERMINAL HYDROLASE 6 (EC 3.1.2.15) (UBIQUITIN THIOLESTERASE 6) (UBIQUITIN-SPECIFIC PROCESSING PROTEASE 6) (DEUBIQUITINATING ENZYME 6) (PROTO-ONCOGENE TRE-2).//2.7e-06:119:34//HOMO SAPIENS (HUMAN).//P35125
F-NT2RP2003714//ZINC FINGER PROTEIN 91 (ZINC FINGER PROTEIN HTF10) (HPF7).//6.7e-27:68:75//HOMO SAPIENS (HUMAN).//Q05481
F-NT2RP2003727//HYPOTHETICAL PROTEIN MG007 HOMOLOG.//0.64:110:30//MYCOPLASMA PNEUMONIAE.//P75105
F-NT2RP2003737//UBIQUITIN-CONJUGATING ENZYME E2-17 KD 2 (EC 6.3.2.19) (UBIQUITIN- PROTEIN LIGASE) (UBIQUITIN CARRIER PROTEIN) (E2(17)KB 2).//1.2e-72:147:90//HOMO SAPIENS (HUMAN), MUS MUSCULUS (MOUSE), RATTUS NORVEGICUS (RAT), AND XENOPUS LAEVIS (AFRICAN CLAWED FROG).//P51669
F-NT2RP2003751//EXTRACELLULAR GLOBIN PRECURSOR.//0.67:68:30//PSEUDOTERRANOVA DECIPIENS (COD WORM).//P26914
F-NT2RP2003760//COATOMER GAMMA SUBUNIT (GAMMA-COAT PROTEIN) (GAMMA-COP).//1.0e-98:235:82//BOS TAURUS (BOVINE).//P53620
F-NT2RP2003764//HYPOTHETICAL 29.3 KD PROTEIN (ORF92).//0.011:69:34//ORGYIA PSEUDOTSUGATA MULTICAPSID POLYHEDROSIS VIRUS (OPMNPV).//O10341
F-NT2RP2003769//ATP SYNTHASE PROTEIN 8 (EC 3.6.1.34) (A6L).//1.0:33:36//EQUUS CABALLUS (HORSE).//P48663
F-NT2RP2003770//PHOSPHATE REGULON SENSOR PROTEIN PHOR (EC 2.7.3.-) (FRAGMENT).//0.029:35:42//PSEUDOMONAS AERUGINOSA.//P23621
F-NT2RP2003777/HYPOTHETICAL 82 KD AVIRULENCE PROTEIN IN AVRBS3 REGION.//0.041:67:34//XANTHOMONAS CAMPESTRIS (PV. VESICATORIA).//P14728
F-NT2RP2003781//HYPOTHETICAL 36.7 KD PROTEIN AH6.2 IN CHROMOSOME II.//4.7e-54:204:47//CAENORHABDITIS ELEGANS.//Q09201
F-NT2RP2003793//PSEUDO-HEVEIN (MINOR HEVEIN).//0.61:30:36//HEVEA BRASILIENSIS (PARA RUBBER TREE).//P80359
F-NT2RP2003825//ENDOTHELIN-1 PRECURSOR (ET-1) (FRAGMENT).//1.0:35:37//CANIS FAMILIARIS (DOG).//P13206
F-NT2RP2003840//HYPOTHETICAL 48.1 KD PROTEIN B0403.2 IN CHROMOSOME X.//2.5e-05:80:38//CAENORHABDITIS ELEGANS.//Q11076
F-NT2RP2003857//BACTERIOCIN MICROCIN B17 PRECURSOR (MCB17).//0.54:28:50//ESCHERICHIA COLI.//P05834
F-NT2RP2003859//DROSOCIN PRECURSOR.//1.0:37:35//DROSOPHILA MELANOGASTER (FRUIT FLY).//P36193
F-NT2RP2003871
F-NT2RP2003885//CUTICLE PROTEIN 32 (LM-32) (LM-ACP 32) (FRAGMENT).//1.0:28:50//LOCUSTA MIGRATORIA (MIGRATORY LOCUST).//P11736
F-NT2RP2003912//SERINE/THREONINE-PROTEIN KINASE NEK1 (EC 2.7.1.-) (NIMA-RELATED PROTEIN KINASE 1).//4.8e-110:268:80//MUS MUSCULUS (MOUSE).//P51954
F-NT2RP2003952//AMINOPEPTIDASE B (EC 3.4.11.6) (ARGINYL AMINOPEPTIDASE) (ARGININE AMINOPEPTIDASE) (CYTOSOL AMINOPEPTIDASE IV) (AP-B).//0.00024:92:31//RATTUS NORVEGICUS (RAT).//O09175
F-NT2RP2003968//SKIN SECRETORY PROTEIN XP2 PRECURSOR (APEG PROTEIN).//9.2e-05:101:36//XENOPUS LAEVIS (AFRICAN CLAWED FROG).//P17437
F-NT2RP2003976//!!!! ALU SUBFAMILY J WARNING ENTRY !!!!//1.7e-21:62:62//HOMO SAPIENS (HUMAN).//P39188
F-NT2RP2003981//VACUOLAR PROTEIN SORTING-ASSOCIATED PROTEIN VPS8.//2.7e-08:165:22//SACCHAROMYCES CEREVISIAE (BAKER'S YEAST).//P39702
F-NT2RP2003984//UNC-87 PROTEIN.//0.75:71:28//CAENORHABDITIS ELEGANS.//P37806
F-NT2RP2003986//!!!! ALU SUBFAMILY SP WARNING ENTRY !!!!//5.3e-19:47:70//HOMO SAPIENS (HUMAN).//P39193
F-NT2RP2003988//!!!! ALU SUBFAMILY SX WARNING ENTRY !!!!//2.2e-18:80:58//HOMO SAPIENS (HUMAN).//P39195
F-NT2RP2004013//TRANSCRIPTION FACTOR BTF3 (RNA POLYMERASE B TRANSCRIPTION FACTOR 3).//1.0e-52:141:77//HOMO SAPIENS (HUMAN).//P20290
F-NT2RP2004014//MACROPHAGE INFLAMMATORY PROTEIN-2-ALPHA (MIP2-ALPHA) (CINC-2-ALPHA).//0.99:45:26//RATTUS NORVEGICUS (RAT).//Q10746
F-NT2RP2004041//SYNAPSINS IA AND IB.//0.0022:51:37//BOS TAURUS (BOVINE).//P17599
F-NT2RP2004042//CRUSTACEAN HYPERGLYCEMIC HORMONE PRECURSOR (CHH) (FRAGMENT).//1.0:49:28//PENAEUS VANNAMEI (PENOEID SHRIMP) (EUROPEAN WHITE SHRIMP).//Q26181
F-NT2RP2004066//CALDESMON (CDM).//2.9e-05:175:21//GALLUS GALLUS (CHICKEN).//P12957
F-NT2RP2004081//CADMIUM-METALLOTHIONEIN (CD-MT).//0.93:59:23//HELIX POMATIA (ROMAN SNAIL) (EDIBLE SNAIL).//P33187
F-NT2RP2004098//RAS SUPPRESSOR PROTEIN 1 (RSU-1) (RSP-1 PROTEIN) (RSP-1).//4.6e-09:121:30//HOMO SAPIENS (HUMAN).//Q15404
F-NT2RP2004124//NONHISTONE CHROMOSOMAL PROTEIN HMG-17.//0.068:63:31//GALLUS GALLUS (CHICKEN).//P02314
F-NT2RP2004142//HYPOTHETICAL 59.1 KD PROTEIN IN VPS15-YMC2 INTERGENIC REGION.//7.9e-05:94:28//SACCHAROMYCES CEREVISIAE (BAKER'S YEAST).//P38262
F-NT2RP2004152//LAMIN L(I).//0.25:167:19//XENOPUS LAEVIS (AFRICAN CLAWED FROG).//P09010
F-NT2RP2004165//HYPOTHETICAL 57.5 KD PROTEIN IN VMA7-RPS25A INTERGENIC REGION.//0.0014:124:31//SACCHAROMYCES CEREVISIAE (BAKER'S YEAST).//P53214
F-NT2RP2004170//PROLINE-RICH PROTEIN MP-3 (FRAGMENT).//0.012:125:30//MUS MUSCULUS (MOUSE).//P05143
F-NT2RP2004172//HYPOTHETICAL 105.7 KD PROTEIN IN TPK3-PIR1 INTERGENIC REGION.//4.1e-26:214:35//SACCHAROMYCES CEREVISIAE (BAKER'S YEAST).//P36051
F-NT2RP2004187//ZINC FINGER PROTEIN 174.//3.7e-12:76:47//HOMO SAPIENS (HUMAN).//Q15697
F-NT2RP2004194//HYPOTHETICAL 10.5 KD PROTEIN C31A2.13C IN CHROMOSOME I.//0.0013:92:23//SCHIZOSACCHAROMYCES POMBE (FISSION YEAST).//Q09730
F-NT2RP2004196//METALLOTHIONEIN 10-II (MT-10-II).//0.92:36:36//MYTILUS EDULIS (BLUE MUSSEL).//P80247
F-NT2RP2004207//MALE ACCESSORY GLAND SECRETORY PROTEIN 355A PRECURSOR.//0.92:62:35//DROSOPHILA SIMULANS (FRUIT FLY).//P33737
F-NT2RP2004226//66 KD STRESS PROTEIN (P66).//0.030:113:26//PHYSARUM POLYCEPHALUM (SLIME MOLD).//P90587
F-NT2RP2004232//PROTEIN KINASE C, MU TYPE (EC 2.7.1.-) (NPKC-MU).//2.0e-48:211:51//HOMO SAPIENS (HUMAN).//Q15139
F-NT2RP2004239//GLUTENIN, HIGH MOLECULAR WEIGHT SUBUNIT PW212 PRECURSOR.//0.00038:111:36//TRITICUM AESTIVUM (WHEAT).//P08489
F-NT2RP2004240//METALLOTHIONEIN-II (MT-II) (METALLOTHIONEIN-LIKE PROTEIN) (MT-CE).//1.0:39:28//CAENORHABDITIS ELEGANS.//P17512
F-NT2RP2004242//RAS-RELATED PROTEIN RGP1 (GTP-BINDING REGULATORY PROTEIN RGP1).//0.0036:64:28//ORYZA SATIVA (RICE).//P25766
F-NT2RP2004245//ATP SYNTHASE PROTEIN 8 (EC 3.6.1.34) (A6L).//1.0:21:42//PONGO PYGMAEUS PYGMAEUS (BORNEAN ORANGUTAN).//P92896
F-NT2RP2004270//SPIDROIN 2 (DRAGLINE SILK FIBROIN 2) (FRAGMENT).//0.00023:118:33//NEPHILA CLAVIPES (ORB SPIDER).//P46804
F-NT2RP2004300//PROBABLE E4 PROTEIN.//0.18:77:40//HUMAN PAPILLOMAVIRUS TYPE 8.//P06425
F-NT2RP2004316
F-NT2RP2004321//HYPOTHETICAL 10.8 KD PROTEIN SSR2439.//1.0:50:28//SYNECHOCYSTIS SP. (STRAIN PCC 6803).//Q01904
F-NT2RP2004339//!!!! ALU SUBFAMILY SX WARNING ENTRY !!!!//5.0e-33:84:77//HOMO SAPIENS (HUMAN).//P39195
F-NT2RP2004347//HYPOTHETICAL 40.9 KD PROTEIN F33H1.3 FROM CHROMOSOME II.//0.78:96:30//CAENORHABDITIS ELEGANS.//Q09556
F-NT2RP2004364//MINOR OUTER CAPSID PROTEIN (NS26) (NONSTRUCTURAL PROTEIN VP9).//0.059:143:30//BOVINE ROTAVIRUS (STRAIN UK).//P04515
F-NT2RP2004365//EAMZP30-47 PROTEIN (FRAGMENT).//0.27:38:39//EIMERIA ACERVULINA.//P21959
F-NT2RP2004366//GLYCOPROTEIN L PRECURSOR.//0.64:71:28//MAREK'S DISEASE HERPESVIRUS (STRAIN GA) (MDHV).//P52510
F-NT2RP2004373//HISTIDINE-RICH GLYCOPROTEIN PRECURSOR (HISTIDINE-PROLINE RICH GLYCOPROTEIN) (HPRG) (FRAGMENT).//0.59:50:40//ORYCTOLAGUS CUNICULUS (RABBIT).//Q28640
F-NT2RP2004389//HYPOTHETICAL 70.7 KD PROTEIN F09G8.3 IN CHROMOSOME III.//4.0e-16:89:43//CAENORHABDITIS ELEGANS.//P34388
F-NT2RP2004392
F-NT2RP2004396//SINGLE-STRANDED NUCLEIC ACID-BINDING PROTEIN.//0.42:89:29//SACCHAROMYCES CEREVISIAE (BAKER'S YEAST).//P10080
F-NT2RP2004399//SOMATOTROPIN PRECURSOR (GROWTH HORMONE).//1.0:72:34//MESOCRICETUS AURATUS (GOLDEN HAMSTER).//P37886
F-NT2RP2004400
F-NT2RP2004412//SPERM PROTAMINE P1.//0.24:38:31//NOTORYCTES TYPHLOPS (MARSUPIAL MOLE).//P42143
F-NT2RP2004425//SUPPRESSOR PROTEIN SRP40.//0.0087:197:22//SACCHAROMYCES CEREVISIAE (BAKER'S YEAST).//P32583
F-NT2RP2004463//ALPHA-2A ADRENERGIC RECEPTOR (ALPHA-2A ADRENOCEPTOR) (ALPHA-2AAR).//1.3e-05:121:37//MUS MUSCULUS (MOUSE).//Q01338
F-NT2RP2004476//NICKEL-SENSITIVE T-TYPE CALCIUM CHANNEL ALPHA-1 SUBUNIT (RBE-II).//0.20:68:36//RATTUS NORVEGICUS (RAT).//Q07652
F-NT2RP2004490//FOS-RELATED ANTIGEN 1.//0.94:59:33//HOMO SAPIENS (HUMAN).//P15407
F-NT2RP2004512//NADH-UBIQUINONE OXIDOREDUCTASE CHAIN 4 (EC 1.6.5.3) (FRAGMENTS).//1.0:37:32//PISASTER OCHRACEUS (SEA STAR).//P24998
F-NT2RP2004523//!!!! ALU SUBFAMILY SQ WARNING ENTRY !!!!//2.1e-15:57:71//HOMO SAPIENS (HUMAN).//P39194
F-NT2RP2004538//KINESIN-LIKE PROTEIN KIF1A (AXONAL TRANSPORTER OF SYNAPTIC VESICLES).//1.2e-48:121:60//HOMO SAPIENS (HUMAN).//Q12756
F-NT2RP2004551//HYPOTHETICAL 7.6 KD PROTEIN (ORF 65).//1.0:20:50//EUGLENA GRACILIS.//P32095
F-NT2RP2004568//PUTATIVE ATP-DEPENDENT RNA HELICASE C30D11.03.//5.2e-07:150:30//SCHIZOSACCHAROMYCES POMBE (FISSION YEAST).//Q09903
F-NT2RP2004580//!!!! ALU SUBFAMILY SC WARNING ENTRY !!!!//3.7e-37:100:78//HOMO SAPIENS (HUMAN).//P39192
F-NT2RP2004587//HYPOTHETICAL 57.5 KD PROTEIN IN VMA7-RPS25A INTERGENIC REGION.//8.2e-06:150:32//SACCHAROMYCES CEREVISIAE (BAKER'S YEAST).//P53214
F-NT2RP2004594//HYPOTHETICAL 45.3 KD PROTEIN C09F5.7 IN CHROMOSOME II.//0.84:105:24//CAENORHABDITIS ELEGANS.//Q09458
F-NT2RP2004600//MYRISTOYLATED ALANINE-RICH C-KINASE SUBSTRATE (MARCKS).//0.17:127:29//RATTUS NORVEGICUS (RAT).//P30009
F-NT2RP2004602//!!!! ALU SUBFAMILY J WARNING ENTRY !!!!//1.1e-05:50:58//HOMO SAPIENS (HUMAN).//P39188
F-NT2RP2004614//HYPOTHETICAL 11.6 KD PROTEIN.//1.0:68:33//VACCINIA VIRUS (STRAIN COPENHAGEN).//P20561
F-NT2RP2004655//GLYCINE-RICH RNA-BINDING PROTEIN 7.//7.0e-05:70:42//ARABIDOPSIS THALIANA (MOUSE-EAR CRESS).//Q03250
F-NT2RP2004664//HYPOTHETICAL 104.0 KD PROTEIN C32A11.03C IN CHROMOSOME I.//0.30:78:38//SCHIZOSACCHAROMYCES POMBE (FISSION YEAST).//Q10328
F-NT2RP2004675
F-NT2RP2004681
F-NT2RP2004689//HYPOTHETICAL 78.3 KD PROTEIN IN RAM2-ATP7 INTERGENIC REGION.//0.021:179:24//SACCHAROMYCES CEREVISIAE (BAKER'S YEAST).//P34243
F-NT2RP2004709//HYPOTHETICAL PROTEIN MJ0647.//0.90:39:43//METHANOCOCCUS JANNASCHII.//Q58063
F-NT2RP2004710//GAR2 PROTEIN.//0.085:60:30//SCHIZOSACCHAROMYCES POMBE (FISSION YEAST).//P41891
F-NT2RP2004736//!!!! ALU SUBFAMILY J WARNING ENTRY !!!!//4.4e-15:97:49//HOMO SAPIENS (HUMAN).//P39188
F-NT2RP2004743//MALE SPECIFIC SPERM PROTEIN MST87F.//0.43:24:41//DROSOPHILA MELANOGASTER (FRUIT FLY).//P08175
F-NT2RP2004767//36.4 KD PROLINE-RICH PROTEIN.//0.0051:88:27//LYCOPERSICON ESCULENTUM (TOMATO).//Q00451
F-NT2RP2004768//SERINE/THREONINE-PROTEIN KINASE NRK1 (EC 2.7.1.-) (N-RICH KINASE 1).//9.0e-29:166:43//SACCHAROMYCES CEREVISIAE (BAKER'S YEAST).//P38692
F-NT2RP2004775
F-NT2RP2004791//PROBABLE LEUCYL-TRNA SYNTHETASE (EC 6.1.1.4) (LEUCINE--TRNA LIGASE) (LEURS).//7.4e-60:226:53//CAENORHABDITIS ELEGANS.//Q09996
F-NT2RP2004799//SUCCINYL-COA LIGASE [GDP-FORMING], BETA-CHAIN PRECURSOR (EC 6.2.1.4) (SUCCINYL-COA SYNTHETASE, BETA CHAIN) (SCS-BETA).//2.2e-42:133:57//NEOCALLIMASTIX FRONTALIS (RUMEN FUNGUS).//P53587
F-NT2RP2004802//HYPOTHETlCAL 17.1 KD PROTEIN IN PUR5 3'REGION.//0.018:86:32//SACCHAROMYCES CEREVISIAE (BAKER'S YEAST).//P38898
F-NT2RP2004816//H<BETA>58 PROTEIN.//1.0e-68:145:93//MUS MUSCULUS (MOUSE).//P40336
F-NT2RP2004841//DSRD PROTEIN.//0.83:33:39//ARCHAEOGLOBUS FULGIDUS.//P70742
F-NT2RP2004861//KERATIN, HIGH-SULFUR MATRIX PROTEIN, IIIA3A.//0.0072:41:39//OVIS ARIES (SHEEP).//P02443
F-NT2RP2004897//METALLOTHIONEIN-LIKE PROTEIN 1.//0.99:41:41//CASUARINA GLAUCA (SWAMP OAK).//Q39511
F-NT2RP2004933//DEATH-ASSOCIATED PROTEIN KINASE 1 (EC 2.7.1.-) (DAP KINASE 1).//8.4e-34:102:67//HOMO SAPIENS (HUMAN).//P53355
F-NT2RP2004936//HIGH POTENTIAL IRON-SULFUR PROTEIN, ISOZYME 2 (HIPIP 2).//0.87:36:33//ECTOTHIORHODOSPIRA VACUOLATA.//P38524
F-NT2RP2004959//STEM CELL FACTOR PRECURSOR (SCF) (MAST CELL GROWTH FACTOR) (MGF) (C-KIT LIGAND).//1.0:69:28//CANIS FAMILIARIS (DOG).//Q06220
F-NT2RP2004961//ZINC FINGER PROTEIN 33A (ZINC FINGER PROTEIN KOX31) (KIAA0065) (HA0946) (FRAGMENT).//2.1e-21:73:58//HOMO SAPIENS (HUMAN).//Q06730
F-NT2RP2004962//!!!! ALU SUBFAMILY SB WARNING ENTRY !!!!//0.17:28:57//HOMO SAPIENS (HUMAN).//P39189
F-NT2RP2004967//HYPOTHETlCAL 7.3 KD PROTEIN.//0.76:41:31//THERMOPROTEUS TENAX VIRUS 1 (STRAIN KRA1) (TTV1).//P19301
F-NT2RP2004978//SPERMATID-SPECIFIC PROTEIN T2 [CONTAINS: SPERM PROTAMINE SP2].//0.44:40:45//SEPIA OFFICINALIS (COMMON CUTTLEFISH).//P80002
F-NT2RP2004982
F-NT2RP2004985//HYPOTHETICAL PROTEIN KIAA0144.//1.2e-51:204:57//HOMO SAPIENS (HUMAN).//Q14157
F-NT2RP2004999//LONG NEUROTOXIN 1 (ALPHA-BUNGAROTOXIN) (BGTX).//0.23:73:26//BUNGARUS MULTICINCTUS (MANY-BANDED KRAIT).//P01378
F-NT2RP2005000//ATPASE STABILIZING FACTOR 15 KD PROTEIN.//0.12:37:32//SACCHAROMYCES CEREVISIAE (BAKER'S YEAST).//P16965
F-NT2RP2005001//SMALL PROLINE RICH PROTEIN II (SPR-II) (CLONE 930).//0.90:54:31//HOMO SAPIENS (HUMAN).//P22531
F-NT2RP2005003//DOWN REGULATORY PROTEIN OF INTERLEUKIN 2 RECEPTOR.//1.6e-30:78:56//MUS MUSCULUS (MOUSE).//P15533
F-NT2RP2005012//NPL1 PROTEIN (SEC63 PROTEIN).//0.00024:94:35//SACCHAROMYCES CEREVISIAE (BAKER'S YEAST).//P14906
F-NT2RP2005018//GAG POLYPROTEIN (CORE POLYPROTEIN) [CONTAINS: CORE PROTEINS P19, P10] (FRAGMENT).//1.0:91:28//AVIAN ENDOGENOUS ROUS-ASSOCIATED VIRUS-0 (EV-2) (AVIAN RETROVIRUS RAV-0).//P06937
F-NT2RP2005020
F-NT2RP2005022//VEGETATIBLE INCOMPATIBILITY PROTEIN HET-E-1.//4.9e-11:106:35//PODOSPORA ANSERINA.//Q00808
F-NT2RP2005031
F-NT2RP2005037//ANTI-SILENCING PROTEIN 1.//2.2e-32:117:55//SACCHAROMYCES CEREVISIAE (BAKER'S YEAST).//P32447
F-NT2RP2005038//DNA NUCLEOTIDYLEXOTRANSFERASE (EC 2.7.7.31) (TERMINAL ADDITION ENZYME) (TERMINAL DEOXYNUCLEOTIDYLTRANSFERASE) (TERMINAL TRANSFERASE).//9.3e-28:187:40//AMBYSTOMA MEXICANUM (AXOLOTL).//O57486
F-NT2RP2005108//CUTICLE COLLAGEN 2.//0.33:62:38//CAENORHABDITIS ELEGANS.//P17656
F-NT2RP2005116//PUTATIVE EUKARYOTIC TRANSLATION INITIATION FACTOR 3 ALPHA SUBUNIT (EIF-3 ALPHA).//4.0e-54:161:63//CAENORHABDITIS ELEGANS.//P34466
F-NT2RP2005126//CHLOROPLAST 50S RIBOSOMAL PROTEIN L27 (FRAGMENT).//0.23:46:39//PLEUROCHRYSIS HAPTONEMOFERA.//P41552
F-NT2RP2005139//COLLAGEN ALPHA 1(XII) CHAIN (FRAGMENTS).//0.016:43:37//BOS TAURUS (BOVINE).//P25508
F-NT2RP2005140//HYPOTHETICAL 7.4 KD PROTEIN YCF33.//0.96:51:39//GUILLARDIA THETA (CRYPTOMONAS PHI).//O78517
F-NT2RP2005144//TUBBY PROTEIN.//5.6e-08:66:45//MUS MUSCULUS (MOUSE).//P50586
F-NT2RP2005147
F-NT2RP2005159//PHOTOSYSTEM II 4 KD REACTION CENTRE PROTEIN PRECURSOR.//0.94:57:29//NICOTIANA TABACUM (COMMON TOBACCO), AND SPINACIA OLERACEA (SPINACH).//P12164
F-NT2RP2005162//HYPOTHETICAL 54.2 KD PROTEIN IN ERP5-ORC6 INTERGENIC REGION.//1.2e-33:139:51//SACCHAROMYCES CEREVISIAE (BAKER'S YEAST).//P38821
F-NT2RP2005168//HETEROGENOUS NUCLEAR RIBONUCLEOPROTEIN U (HNRNP U).//2.8e-33:102:61//HOMO SAPIENS (HUMAN).//Q00839
F-NT2RP2005204//DNA DAMAGE TOLERANCE PROTEIN RHC31 (RAD31 HOMOLOG).//3.9e-28:141:42//SACCHAROMYCES CEREVISIAE (BAKER'S YEAST).//Q06624
F-NT2RP2005227
F-NT2RP2005239//TRNA SPLICING PROTEIN SPL1.//2.0e-38:117:64//CANDIDA ALBICANS (YEAST).//P87185
F-NT2RP2005254//OMEGA-AGATOXIN IB (OMEGA-AGA-IB) (FRAGMENT).//0.26:29:48//AGELENOPSIS APERTA (FUNNEL-WEB SPIDER).//P15970
F-NT2RP2005270//HOMEOBOX PROTEIN HOX-A4 (CHOX-1.4).//0.037:82:34//GALLUS GALLUS (CHICKEN).//P17277
F-NT2RP2005276//LONG-CHAIN-FATTY-ACID--COA LIGASE 4 (EC 6.2.1.3) (LONG-CHAIN ACYL-COA SYNTHETASE 4) (LACS 4).//2.0e-59:174:61//RATTUS NORVEGICUS (RAT).//O35547
F-NT2RP2005287//ZINC FINGER PROTEIN 26 (ZINC FINGER PROTEIN KOX20) (FRAGMENT).//1.5e-05:27:70//HOMO SAPIENS (HUMAN).//P17031
F-NT2RP2005288//PROBABLE RUBREDOXIN HUPI.//1.0:42:28//RHIZOBIUM LEGUMINOSARUM (BIOVAR VICIAE).//P28151
F-NT2RP2005289//!!!! ALU SUBFAMILY SP WARNING ENTRY !!!!//1.1e-21:75:70//HOMO SAPIENS (HUMAN).//P39193
F-NT2RP2005293//TRANSLATION INITIATION FACTOR IF-2.//0.58:170:24//HELICOBACTER PYLORI (CAMPYLOBACTER PYLORI).//P55972
F-NT2RP2005315//CUTICLE COLLAGEN 7 (FRAGMENT).//0.091:65:38//CAENORHABDITIS ELEGANS.//P18832
F-NT2RP2005325//CHROMOGRANIN A PRECURSOR (CGA) (PITUITARY SECRETORY PROTEIN I) (SP-I) [CONTAINS: PANCREASTATIN; WE-14].//9.5e-09:98:39//HOMO SAPIENS (HUMAN).//P10645
F-NT2RP2005336//HYPOTHETICAL 68.7 KD PROTEIN IN STB1-MCK1 INTERGENIC REGION.//0.00011:124:28//SACCHAROMYCES CEREVISIAE (BAKER'S YEAST).//P42846
F-NT2RP2005344//PROBABLE CALCIUM-TRANSPORTING ATPASE 4 (EC 3.6.1.38).//4.7e-21:92:52//SACCHAROMYCES CEREVISIAE (BAKER'S YEAST).//Q12675
F-NT2RP2005354
F-NT2RP2005358//MYOSIN IC HEAVY CHAIN.//0.012:91:39//ACANTHAMOEBA CASTELLANII (AMOEBA).//P10569
F-NT2RP2005360//ACROSIN PRECURSOR (EC 3.4.21.10).//0.0022:73:36//ORYCTOLAGUS CUNICULUS (RABBIT).//P48038
F-NT2RP2005393//HYPOTHETICAL 25.9 KD PROTEIN AH6.3 IN CHROMOSOME II.//0.00085:135:28//CAENORHABDITIS ELEGANS .//Q09202
F-NT2RP2005407//SQUALENE MONOOXYGENASE (EC 1.14.99.7) (SQUALENE EPOXIDASE) (SE).//0.96:109:26//SACCHAROMYCES CEREVISIAE (BAKER'S YEAST).//P32476
F-NT2RP2005436//EXTENSIN PRECURSOR (PROLINE-RICH GLYCOPROTEIN).//0.0011:54:42//ZEA MAYS (MAIZE).//P14918
F-NT2RP2005441//PROLINE-RICH PROTEIN MP-2 PRECURSOR.//0.039:182:29//MUS MUSCULUS (MOUSE).//P05142
F-NT2RP2005453
F-NT2RP2005457//NADH-UBIQUINONE OXIDOREDUCTASE SUBUNIT B14.5B (EC 1.6.5.3) (EC 1.6.99.3) (COMPLEX I-B14.5B) (CI-B14.5B).//4.0e-10:124:37//BOS TAURUS (BOVINE).//Q02827
F-NT2RP2005464//HYPOTHETICAL 9.5 KD PROTEIN.//0.96:42:33//VACCINIA VIRUS (STRAIN COPENHAGEN).//P20553
F-NT2RP2005465//MITOCHONDRIAL CARRIER PROTEIN RIM2.//4.6e-09:92:42//SACCHAROMYCES CEREVISIAE (BAKER'S YEAST).//P38127
F-NT2RP2005472//HYPOTHETICAL PROTEIN BB0129.//0.76:80:32//BORRELIA BURGDORFERI (LYME DISEASE SPIROCHETE).//O51155
F-NT2RP2005476//!!!! ALU SUBFAMILY SP WARNING ENTRY !!!!//1.0e-31:39:89//HOMO SAPIENS (HUMAN).//P39193
F-NT2RP2005490//METALLOTHIONEIN-II (MT-II).//0.14:27:33//SCYLLA SERRATA (MUD CRAB).//P02806
F-NT2RP2005491//DNA-DIRECTED RNA POLYMERASE SUBUNIT I (EC 2.7.7.6).//0.95:45:31//METHANOCOCCUS JANNASCHII.//Q58785
F-NT2RP2005495//HYPOTHETICAL 10.8 KD PROTEIN IN GP30-RIII INTERGENIC REGION.//0.99:68:30//BACTERIOPHAGE T4.//Q02407
F-NT2RP2005496//ZINC FINGER PROTEIN 135.//1.4e-54:120:59//HOMO SAPIENS (HUMAN).//P52742
F-NT2RP2005498//PROTEIN PHOSPHATASE PP2A, 55 KD REGULATORY SUBUNIT, ALPHA ISOFORM (PROTEIN PHOSPHATASE PP2A B SUBUNIT ALPHA ISOFORM) (ALPHA-PR55).//9.5e-76:146:86//RATTUS NORVEGICUS (RAT).//P36876
F-NT2RP2005501//GALECTIN-3 (GALACTOSE-SPECIFIC LECTIN 3) (MAC-2 ANTIGEN) (IGE-BINDING PROTEIN) (35 KD LECTIN) (CARBOHYDRATE BINDING PROTEIN 35) (CBP 35) (LAMININ-BINDING PROTEIN) (LECTIN L-29) (L-31) (GALACTOSIDE-BINDING PROTEIN) (GALBP).//0.025:70:40//HOMO SAPIENS (HUMAN).//P17931
F-NT2RP2005509//PROCOLLAGEN ALPHA 1(I) CHAIN PRECURSOR.//1.0:166:27//GALLUS GALLUS (CHICKEN).//P02457
F-NT2RP2005520//CHROMOSOME ASSEMBLY PROTEIN XCAP-E.//7.9e-45:118:79//XENOPUS LAEVIS (AFRICAN CLAWED FROG).//P50533
F-NT2RP2005525//50S RIBOSOMAL PROTEIN L11.//1.0:47:27//BORRELIA BURGDORFERI (LYME DISEASE SPIROCHETE).//O51354
F-NT2RP2005531//PROTEIN-TYROSINE PHOSPHATASE MEG1 (EC 3.1.3.48) (PTPASE-MEG1) (MEG).//9.8e-13:84:45//HOMO SAPIENS (HUMAN).//P29074
F-NT2RP2005539//RING CANAL PROTEIN (KELCH PROTEIN).//4.9e-10:90:33//DROSOPHILA MELANOGASTER (FRUIT FLY).//Q04652
F-NT2RP2005540//NUCLEOTIDE BINDING PROTEIN EXPZ.//0.36:119:21//BACILLUS SUBTILIS.//P39115
F-NT2RP2005549//HYPOTHETICAL 32.0 KD PROTEIN C16C10.10 IN CHROMOSOME III.//6.0e-39:179:46//CAENORHABDITIS ELEGANS.//Q09253
F-NT2RP2005555
F-NT2RP2005557//HYPOTHETICAL 23.7 KD PROTEIN C13G6.14 IN CHROMOSOME I.//4.9e-06:90:35//SCHIZOSACCHAROMYCES POMBE (FISSION YEAST).//Q09790
F-NT2RP2005581
F-NT2RP2005600//BASIC PROLINE-RICH PEPTIDE P-E (IB-9).//0.014:37:40//HOMO SAPIENS (HUMAN).//P02811
F-NT2RP2005605//GONADOLIBERIN I PRECURSOR (LHRH I) (LUTEINIZING HORMONE RELEASING HORMONE I) (GONADOTROPIN RELEASING HORMONE I) (GNRH I) (LULIBERIN I) (FRAGMENT).//0.64:26:42//MACACA MULATTA (RHESUS MACAQUE).//P55247
F-NT2RP2005620//HYPOTHETICAL 45.1 KD PROTEIN IN RPS5-ZMS1 INTERGENIC REGION.//8.7e-31:138:49//SACCHAROMYCES CEREVISIAE (BAKER'S YEAST).//P47160
F-NT2RP2005622//NEUROTOXIN-LIKE PROTEIN STR1 (ANATOXIN AAH STR1).//0.39:22:40//ANDROCTONUS AUSTRALIS HECTOR (SAHARA SCORPION).//P80950
F-NT2RP2005635//HYPOTHETICAL 80.7 KD PROTEIN IN ERG7-NMD2 INTERGENIC REGION.//5.8e-43:144:56//SACCHAROMYCES CEREVISIAE (BAKER'S YEAST).//P38795
F-NT2RP2005637//VPU PROTEIN (U ORF PROTEIN).//0.91:33:45//CHIMPANZEE IMMUNODEFICIENCY VIRUS (SIV(CPZ)) (CIV).//P17286
F-NT2RP2005640//METALLOTHIONEIN-LIKE PROTEIN LSC54.//0.63:41:31//BRASSICA NAPUS (RAPE).//P43402
F-NT2RP2005645
F-NT2RP2005651//OCTAMER-BINDING TRANSCRIPTION FACTOR 3A (OCT-3A) (OCT-4).//0.0023:50:42//HOMO SAPIENS (HUMAN).//Q01860
F-NT2RP2005654//HYPOTHETICAL 48.6 KD PROTEIN IN BET1-PAN1 INTERGENIC REGION.//6.1e-16:76:44//SACCHAROMYCES CEREVISIAE (BAKER'S YEAST).//P40564
F-NT2RP2005669//METALLOTHIONEIN-II (MT-II).//0.76:16:50//SCYLLA SERRATA (MUD CRAB).//P02806
F-NT2RP2005675//PUTATIVE ORAL CANCER SUPPRESSOR (DELETED IN ORAL CANCER-1).//6.5e-26:116:54//MESOCRICETUS AURATUS (GOLDEN HAMSTER).//P49119
F-NT2RP2005683//HYPOTHETICAL PROTEIN HI0275.//0.17:50:40//HAEMOPHILUS INFLUENZAE.//P43975
F-NT2RP2005690//PYRROLINE-5-CARBOXYLATE REDUCTASE (EC 1.5.1.2) (P5CR) (P5C REDUCTASE).//1.3e-16:75:30//PISUM SATIVUM (GARDEN PEA).//Q04708
F-NT2RP2005694//HYPOTHETICAL PROTEIN KIAA0032.//9.6e-11:135:34//HOMO SAPIENS (HUMAN).//Q15034
F-NT2RP2005701//SALIVARY PROLINE-RICH PROTEIN PO (ALLELE M) [CONTAINS: PEPTIDE P-D] (FRAGMENT).//0.084:158:32//HOMO SAPIENS (HUMAN).//P10161
F-NT2RP2005712//METALLOTHIONEIN-II (MT-II).//0.19:14:50//STENELLA COERULEOALBA (STRIPED DOLPHIN).//P14425
F-NT2RP2005719//COLLAGEN ALPHA 1(I) CHAIN (FRAGMENT).//1.0:36:41//ORYCTOLAGUS CUNICULUS (RABBIT).//P02456
F-NT2RP2005722//ZINC FINGER PROTEIN ZFP-36 (FRAGMENT).//7.8e-37:131:62//HOMO SAPIENS (HUMAN).//P16415
F-NT2RP2005723//!!!! ALU SUBFAMILY SC WARNING ENTRY !!!!//0.98:23:60//HOMO SAPIENS (HUMAN).//P39192
F-NT2RP2005726//HYPOTHETICAL PROTEIN TP0375.//0.98:30:43//TREPONEMA PALLIDUM.//O83390
F-NT2RP2005732//PERIOD CLOCK PROTEIN (FRAGMENT).//0.41:20:55//DROSOPHILA ROBUSTA (FRUIT FLY).//Q03296
F-NT2RP2005741//SMR1 PROTEIN PRECURSOR (VCS-ALPHA 1).//0.38:58:36//RATTUS NORVEGICUS (RAT).//P13432
F-NT2RP2005748//ZINC FINGER PROTEIN KOX23 (FRAGMENT).//0.026:19:68//HOMO SAPIENS (HUMAN).//P17034
F-NT2RP2005752//PROCOLLAGEN ALPHA 1(III) CHAIN PRECURSOR.//0.90:101:31//HOMO SAPIENS (HUMAN).//P02461
F-NT2RP2005753//DNA-BINDING P52/P100 COMPLEX, 100 KD SUBUNIT (FRAGMENTS).//0.50:22:59//HOMO SAPIENS (HUMAN).//P30808
F-NT2RP2005763//PUTATIVE ATP-DEPENDENT RNA HELICASE STE13.//4.7e-14:108:37//SCHIZOSACCHAROMYCES POMBE (FISSION YEAST).//Q09181
F-NT2RP2005767//NONHISTONE CHROMOSOMAL PROTEIN 6B.//4.1e-08:65:32//SACCHAROMYCES CEREVISIAE (BAKER'S YEAST).//P11633
F-NT2RP2005773//PYRROLINE-5-CARBOXYLATE REDUCTASE (EC 1.5.1.2) (P5CR) (P5C REDUCTASE).//1.2e-14:65:61//HOMO SAPIENS (HUMAN).//P32322
F-NT2RP2005775//NEUROLYSIN PRECURSOR (EC 3.4.24.16) (NEUROTENSIN ENDOPEPTIDASE) (MITOCHONDRIAL OLIGOPEPTIDASE M) (MICROSOMAL ENDOPEPTIDASE) (MEP).//1.3e-103:199:90//ORYCTOLAGUS CUNICULUS (RABBIT).//P42675
F-NT2RP2005781//SALIVARY ACIDIC PROLINE-RICH PHOSPHOPROTEIN 1/2 PRECURSOR (PRP-1 / PRP-3) (PRP-2 / PRP-4) (PIF-F / PIF-S) (PROTEIN A / PROTEIN C) [CONTAINS: PEPTIDE P-C].//0.090:73:36//HOMO SAPIENS (HUMAN).//P02810
F-NT2RP2005784//TRANS-ACTING TRANSCRIPTIONAL PROTEIN ICP0 (IMMEDIATE-EARLY PROTEIN IE110) (VMW110) (ALPHA-0 PROTEIN).//3.5e-06:79:37//HERPES SIMPLEX VIRUS (TYPE 1 / STRAIN 17).//P08393
F-NT2RP2005804//HYPOTHETICAL PROLINE-RICH PROTEIN (FRAGMENT).//1.8e-07:43:55//OWENIA FUSIFORMIS.//P21260
F-NT2RP2005812//HYPOTHETICAL 39.3 KD PROTEIN IN GCN4-WBP1 INTERGENIC REGION.//6.3e-14:143:31//SACCHAROMYCES CEREVISIAE (BAKER'S YEAST).//P40004
F-NT2RP2005815//FERROCHELATASE (EC 4.99.1.1) (PROTOHEME FERRO-LYASE) (HEME SYNTHETASE).//0.0017:123:37//MYCOBACTERIUM AVIUM.//O07401
F-NT2RP2005835//SHP1 PROTEIN.//1.2e-08:135:26//SACCHAROMYCES CEREVISIAE (BAKER'S YEAST).//P34223
F-NT2RP2005841//SMALL PROLINE-RICH PROTEIN II (SPR-II) (CLONE 174N).//0.23:28:53//HOMO SAPIENS (HUMAN).//P22532
F-NT2RP2005853//HYPOTHETICAL 8.5 KD PROTEIN IN ASIA-MOTA INTERGENIC REGION.//0.99:33:48//BACTERIOPHAGE T4.//P22917
F-NT2RP2005857//CHROMOSOME ASSEMBLY PROTEIN XCAP-C.//8.6e-84:235:66//XENOPUS LAEVIS (AFRICAN CLAWED FROG).//P50532
F-NT2RP2005859//MALE SPECIFIC SPERM PROTEIN MST84DB.//0.017:60:40//DROSOPHILA MELANOGASTER (FRUIT FLY).//Q01643
F-NT2RP2005868//ATP SYNTHASE B' CHAIN PRECURSOR (EC 3.6.1.34) (SUBUNIT II).//0.28:121:28//SPINACIA OLERACEA (SPINACH).//P31853
F-NT2RP2005886//MICRONUCLEAR LINKER HISTONE POLYPROTEIN (MIC LH) [CONTAINS: LINKER HISTONE PROTEINS ALPHA, BETA, DELTA AND GAMMA].//0.80:130:28//TETRAHYMENA THERMOPHILA.//P40631
F-NT2RP2005890
F-NT2RP2005901//ATP SYNTHASE PROTEIN 8 (EC 3.6.1.34) (A6L).//0.35:18:44//DROSOPHILA YAKUBA (FRUIT FLY).//P03933
F-NT2RP2005908//!!!! ALU SUBFAMILY SQ WARNING ENTRY !!!!//1.0e-28:61:65//HOMO SAPIENS (HUMAN).//P39194
F-NT2RP2005933//PERIOD CLOCK PROTEIN (P230) (FRAGMENT).//1.7e-11:85:49//ACETABULARIA MEDITERRANEA (MERMAID'S WINE GLASS).//P12347
F-NT2RP2005942//POLY(A) POLYMERASE (EC 2.7.7.19) (PAP) (POLYNUCLEOTIDE ADENYLYLTRANSFERASE).//7.2e-59:216:58//BOS TAURUS (BOVINE).//P25500
F-NT2RP2005980//HYPOTHETICAL 11.5 KD PROTEIN IN RSP8A-AST1 INTERGENIC REGION.//1.0:49:34//SACCHAROMYCES CEREVISIAE (BAKER'S YEAST).//P38185
F-NT2RP2006023//DNA REPAIR PROTEIN RECN (RECOMBINATION PROTEIN N) (FRAGMENT).//1.0:40:45//VIBRIO CHOLERAE.//P52118
F-NT2RP2006038//HYPOTHETICAL 30.2 KD PROTEIN C02F5.4 IN CHROMOSOME III.//4.0e-11:90:34//CAENORHABDITIS ELEGANS.//P34281
F-NT2RP2006043//LAMININ BETA-1 CHAIN VARIANT (LAMININ BETA-1-2 CHAIN) (FRAGMENT).//0.00067:73:38//GALLUS GALLUS (CHICKEN).//Q01636
F-NT2RP2006052//METALLOTHIONEIN-I (MT-I).//0.19:31:38//CERCOPITHECUS AETHIOPS (GREEN MONKEY) (GRIVET).//P02797
F-NT2RP2006069//COLLAGEN ALPHA 2(I) CHAIN (FRAGMENTS).//1.0:66:34//RATTUS NORVEGICUS (RAT).//P02466
F-NT2RP2006071//RESTIN.//0.40:156:29//GALLUS GALLUS (CHICKEN).//O42184
F-NT2RP2006098//HYPOTHETICAL 21.7 KD PROTEIN IN TUP1-ABP1 INTERGENIC REGION.//0.99:95:20//SACCHAROMYCES CEREVISIAE (BAKER'S YEAST).//P25651
F-NT2RP2006100//LONG NEUROTOXIN 4 (ALPHA-NEUROTOXIN).//0.94:43:34//OPHIOPHAGUS HANNAH (KING COBRA) (NAJA HANNAH).//P80156
F-NT2RP2006103//50S RIBOSOMAL PROTEIN L32.//0.40:36:38//SYNECHOCYSTIS SP. (STRAIN PCC 6803).//P73014
F-NT2RP2006106//CUTICLE COLLAGEN 1.//0.28:85:29//CAENORHABDITIS ELEGANS.//P08124
F-NT2RP2006141//HYPOTHETICAL 72.5 KD PROTEIN C2F7.10 IN CHROMOSOME I.//1.9e-08:57:42//SCHIZOSACCHAROMYCES POMBE (FISSION YEAST).//Q09701
F-NT2RP2006166
F-NT2RP2006184//HYPOTHETICAL 11.2 KD PROTEIN IN CSGC-MDOG INTERGENIC REGION PRECURSOR.//0.95:87:26//ESCHERICHIA COLI.//P75917
F-NT2RP2006186//MICROTUBULE-ASSOCIATED PROTEIN 2.//0.088:124:33//MUS MUSCULUS (MOUSE).//P20357
F-NT2RP2006196//!!!! ALU SUBFAMILY SP WARNING ENTRY !!!!//4.0e-05:49:61//HOMO SAPIENS (HUMAN).//P39193
F-NT2RP2006200//PROCOLLAGEN ALPHA 2(V) CHAIN PRECURSOR.//0.0013:205:32//HOMO SAPIENS (HUMAN).//P05997
F-NT2RP2006219//GONADAL PROTEIN GDL.//3.5e-18:158:37//DROSOPHILA MELANOGASTER (FRUIT FLY).//P22468
F-NT2RP2006237//FIBRINOGEN- AND IG-BINDING PROTEIN PRECURSOR (MRP PROTEIN).//0.79:103:28//STREPTOCOCCUS PYOGENES.//P30141
F-NT2RP2006238//PROLINE-RICH PROTEIN MP-3 (FRAGMENT).//4.7e-07:127:39//MUS MUSCULUS (MOUSE).//P05143
F-NT2RP2006258//PROBABLE E5 PROTEIN.//0.78:47:34//RHESUS PAPILLOMAVIRUS TYPE 1 (RHPV 1).//P24834
F-NT2RP2006261//PENAEIDIN-3A PRECURSOR (P3-A).//0.61:35:40//PENAEUS VANNAMEI (PENOEID SHRIMP) (EUROPEAN WHITE SHRIMP).//P81058
F-NT2RP2006275//ELECTROMOTOR NEURON-ASSOCIATED PROTEIN 2 (FRAGMENT).//1.2e-28:59:57//TORPEDO CALIFORNICA (PACIFIC ELECTRIC RAY).//P14401
F-NT2RP2006312//HIGH-MOBILITY-GROUP PROTEIN (NONHISTONE CHROMOSOMAL PROTEIN).//1.6e-06:53:35//TETRAHYMENA PYRIFORMIS.//P40625
F-NT2RP2006320//TAT PROTEIN (TRANSACTIVATING REGULATORY PROTEIN) (FRAGMENT).//0.90:24:41//HUMAN IMMUNODEFICIENCY VIRUS TYPE 1 (BH5 ISOLATE) (HIV-1).//P04612
F-NT2RP2006321//!!!! ALU SUBFAMILY SP WARNING ENTRY !!!!//0.0051:25:76//HOMO SAPIENS (HUMAN).//P39193
F-NT2RP2006323//WISKOTT-ALDRICH SYNDROME PROTEIN (WASP).//0.84:33:39//HOMO SAPIENS (HUMAN).//P42768
F-NT2RP2006333//MYOTOXIN 3 PRECURSOR (CROTAMINE 3).//0.56:37:40//CROTALUS DURISSUS TERRIFICUS (SOUTH AMERICAN RATTLESNAKE).//P24333
F-NT2RP2006334//SUCCINYL-COA LIGASE [GDP-FORMING], ALPHA-CHAIN 3 PRECURSOR (EC 6.2.1.4) (SUCCINYL-COA SYNTHETASE, ALPHA CHAIN 3).//0.00097:46:41//TRICHOMONAS VAGINALIS.//P53401
F-NT2RP2006365//NONSPECIFIC LIPID-TRANSFER PROTEIN 4.3 PRECURSOR (LTP 4.3).//0.18:75:29//HORDEUM VULGARE (BARLEY).//Q42842
F-NT2RP2006393//OMEGA-CONOTOXIN MVIIC PRECURSOR (FRAGMENT).//0.82:15:66//CONUS MAGUS (MAGUS CONE).//P37300
F-NT2RP2006436//ANTERIOR-RESTRICTED HOMEOBOX PROTEIN (RATHKE POUCH HOMEO BOX).//1.4e-08:50:50//MUS MUSCULUS (MOUSE).//Q61658
F-NT2RP2006441//METALLOTHIONEIN-LIKE PROTEIN 1.//0.99:22:54//MIMULUS GUTTATUS (SPOTTED MONKEY FLOWER) (YELLOW MONKEY FLOWER).//P20238
F-NT2RP2006454//SPERM PROTAMINE P1.//0.60:47:36//TACHYGLOSSUS ACULEATUS ACULEATUS (AUSTRALIAN ECHIDNA).//P35311
F-NT2RP2006456
F-NT2RP2006464//PHOTOSYSTEM I IRON-SULFUR CENTER (PHOTOSYSTEM I SUBUNIT VII) (9 KD POLYPEPTIDE) (PSI-C).//0.91:79:30//SYNECHOCOCCUS SP. (STRAIN PCC 7002) (AGMENELLUM QUADRUPLICATUM).//P31087
F-NT2RP2006467//PUTATIVE CUTICLE COLLAGEN F55C10.3.//0.15:53:35//CAENORHABDITIS ELEGANS.//Q21184
F-NT2RP2006472//HYPOTHETICAL 19 KD PROTEIN (ORF 167).//0.33:98:26//MARCHANTIA POLYMORPHA (LIVERWORT).//P12202
F-NT2RP2006534
F-NT2RP2006554//ANTI-SIGMA F FACTOR ANTAGONIST (STAGE II SPORULATION PROTEIN AA).//0.91:50:34//BACILLUS SPHAERICUS.//O32723
F-NT2RP2006565//SECRETORY CARRIER-ASSOCIATED MEMBRANE PROTEIN 1 (SCAMP 37).//6.0e-66:93:96//RATTUS NORVEGICUS (RAT).//P56603
F-NT2RP2006571//CYTOCHROME P450 2B10 (EC 1.14.14.1) (CYPIIB10) (TESTOSTERONE 16-ALPHA HYDROXYLASE) (P450-16-ALPHA) (CLONE PF3/46).//4.5e-40:138:57//MUS MUSCULUS (MOUSE).//P12791
F-NT2RP2006573//SPERM PROTAMINE P1 (CYSTEINE-RICH PROTAMINE).//0.53:46:39//BOS TAURUS (BOVINE).//P02318
F-NT2RP2006598//!!!! ALU SUBFAMILY SX WARNING ENTRY !!!!//1.3e-12:44:77//HOMO SAPIENS (HUMAN).//P39195
F-NT2RP3000002//!!!! ALU SUBFAMILY SC WARNING ENTRY !!!!//1.4e-19:60:63//HOMO SAPIENS (HUMAN).//P39192
F-NT2RP3000031//HYPOTHETICAL 89.8 KD PROTEIN F41H10.6 IN CHROMOSOME IV.//2.1e-39:210:42//CAENORHABDITIS ELEGANS.//Q20296
F-NT2RP3000046//POSSIBLE THIOPHENE AND FURAN OXIDATION PROTEIN THDF.//1.4e-25:149:44//PSEUDOMONAS PUTIDA.//P25755
F-NT2RP3000047//NPL4 PROTEIN.//4.7e-48:275:38//SACCHAROMYCES CEREVISIAE (BAKER'S YEAST).//P33755
F-NT2RP3000050//ZINC FINGER PROTEIN 83 (ZINC FINGER PROTEIN HPF1).//3.2e-72:232:59//HOMO SAPIENS (HUMAN).//P51522
F-NT2RP3000055//MALE SPECIFIC SPERM PROTEIN MST84DB.//0.26:57:36//DROSOPHILA MELANOGASTER (FRUIT FLY).//Q01643
F-NT2RP3000068//HYPOTHETICAL 182.0 KD PROTEIN IN NMD5-HOM6 INTERGENIC REGION.//0.0014:66:34//SACCHAROMYCES CEREVISIAE (BAKER'S YEAST).//P47170
F-NT2RP3000072//HYPOTHETICAL 6.7 KD PROTEIN IN NOHA-CSPI INTERGENIC REGION.//0.95:49:30//ESCHERICHIA COLI.//P77695
F-NT2RP3000080//!!!! ALU SUBFAMILY J WARNING ENTRY !!!!//5.1e-17:64:68//HOMO SAPIENS (HUMAN).//P39188
F-NT2RP3000085//BIOTIN CARBOXYLASE (EC 6.3.4.14) (A SUBUNIT OF ACETYL-COA CARBOXYLASE (EC 6.4.1.2)) (ACC).//4.4e-43:169:51//BACILLUS SUBTILIS.//P49787
F-NT2RP3000092//CELL DIVISION CONTROL PROTEIN 1.//0.00016:103:31//SACCHAROMYCES CEREVISIAE (BAKER'S YEAST).//P40986
F-NT2RP3000109//ACYL CARRIER PROTEIN HOMOLOG (ACP).//0.76:83:28//MYCOPLASMA GENITALIUM.//P47529
F-NT2RP3000134
F-NT2RP3000142//GAR2 PROTEIN.//0.00098:241:20//SCHIZOSACCHAROMYCES POMBE (FISSION YEAST).//P41891
F-NT2RP3000149//ATP SYNTHASE PROTEIN 8 (EC 3.6.1.34) (A6L).//0.0014:33:36//PONGO PYGMAEUS ABELII (SUMATRAN ORANGUTAN).//P92694
F-NT2RP3000186//!!!! ALU SUBFAMILY J WARNING ENTRY !!!!//8.3e-15:36:83//HOMO SAPIENS (HUMAN).//P39188
F-NT2RP3000197//HYPOTHETICAL 6.0 KD PROTEIN IN THI12 5'REGION.//0.91:21:52//SACCHAROMYCES CEREVISIAE (BAKER'S YEAST).//P53820
F-NT2RP3000207//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//0.026:209:27//SACCHAROMYCES CEREVISIAE (BAKER'S YEAST).//P08640
F-NT2RP3000220//DNA-BINDING P52/P100 COMPLEX, 100 KD SUBUNIT (FRAGMENTS).//1.0:26:42//HOMO SAPIENS (HUMAN).//P30808
F-NT2RP3000233//RING CANAL PROTEIN (KELCH PROTEIN).//2.1e-42:249:39//DROSOPHILA MELANOGASTER (FRUIT FLY).//Q04652
F-NT2RP3000235//HOMEOBOX PROTEIN H40 (FRAGMENT).//0.55:45:40//APIS MELLIFERA (HONEYBEE).//P15858
F-NT2RP3000247//HYPOTHETICAL PROTEIN KIAA0218.//1.7e-82:123:69//HOMO SAPIENS (HUMAN).//Q93075
F-NT2RP3000251//SERINE PROTEINASE STUBBLE (EC 3.4.21.-) (STUBBLE-STUBBLOID PROTEIN).//1.0:53:33//DROSOPHILA MELANOGASTER (FRUIT FLY).//Q05319
F-NT2RP3000252//HYPOTHETICAL 40 KD GTP-BINDING PROTEIN IN RIBOSOMAL PROTEIN GENE CLUSTER 5'REGION.//2.2e-06:96:32//HALOBACTERIUM CUTIRUBRUM.//P17103
F-NT2RP3000255//HISTONE H1.1 (FRAGMENT).//0.95:71:33//BOS TAURUS (BOVINE).//P02253
F-NT2RP3000267//HYPOTHETICAL 21.1 KD PROTEIN IN SSR-SERA INTERGENIC REGION (O182).//0.38:77:33//ESCHERICHIA COLI.//P09160
F-NT2RP3000299//MYOSIN IC HEAVY CHAIN.//1.2e-11:147:34//ACANTHAMOEBA CASTELLANII (AMOEBA).//P10569
F-NT2RP3000312//HYPOTHETICAL 57.5 KD PROTEIN IN VMA7-RPS25A INTERGENIC REGION.//0.64:216:29//SACCHAROMYCES CEREVISIAE (BAKER'S YEAST).//P53214
F-NT2RP3000320//TRANSLATION INITIATION FACTOR IF-2.//5.2e-05:184:22//AQUIFEX AEOLICUS.//O67825
F-NT2RP3000324//HYPOTHETICAL PROTEIN HI1036.//0.69:64:35//HAEMOPHILUS INFLUENZAE.//P44097
F-NT2RP3000333//WIR1A PROTEIN.//0.35:51:41//TRITICUM AESTIVUM (WHEAT).//Q01482
F-NT2RP3000341//!!!! ALU SUBFAMILY SB WARNING ENTRY !!!!//2.1e-30:57:80//HOMO SAPIENS (HUMAN).//P39189
F-NT2RP3000348
F-NT2RP3000350//HYPOTHETICAL 40 KD GTP-BINDING PROTEIN IN RIBOSOMAL PROTEIN GENE CLUSTER 5'REGION.//0.0011:77:35//HALOBACTERIUM CUTIRUBRUM.//P17103
F-NT2RP3000359//GTP:AMP PHOSPHOTRANSFERASE MITOCHONDRIAL (EC 2.7.4.10) (AK3).//1.2e-97:222:84//BOS TAURUS (BOVINE).//P08760
F-NT2RP3000361//PRE-MRNA SPLICING FACTOR PRP6.//2.2e-08:128:28//SACCHAROMYCES CEREVISIAE (BAKER'S YEAST).//P19735
F-NT2RP3000366//RAS-RELATED PROTEIN RAB-18.//2.1e-107:206:99//MUS MUSCULUS (MOUSE).//P35293
F-NT2RP3000393//HOMEOBOX PROTEIN HOX-C4 (HOX-3E) (CP19).//0.0023:36:52//HOMO SAPIENS (HUMAN).//P09017
F-NT2RP3000397//PUTATIVE PRE-MRNA SPLICING FACTOR RNA HELICASE (DEAH BOX PROTEIN 13).//5.5e-27:116:44//MUS MUSCULUS (MOUSE).//O35286
F-NT2RP3000403//PRE-MRNA PROCESSING PROTEIN PRP40.//0.00044:67:34//SACCHAROMYCES CEREVISIAE (BAKER'S YEAST).//P33203
F-NT2RP3000418//RETROVIRUS-RELATED POL POLYPROTEIN [CONTAINS: REVERSE TRANSCRIPTASE (EC 2.7.7.49); ENDONUCLEASE].//2.2e-16:228:34//MUS MUSCULUS (MOUSE).//P11369
F-NT2RP3000433//!!!! ALU SUBFAMILY J WARNING ENTRY !!!!//1.7e-17:79:55//HOMO SAPIENS (HUMAN).//P39188
F-NT2RP3000439//HYPOTHETICAL 46.4 KD PROTEIN IN FFH-GRPE INTERGENIC REGION.//9.8e-10:201:26//ESCHERICHIA COLI.//P37908
F-NT2RP3000441//PROTEIN-EXPORT MEMBRANE PROTEIN SECG HOMOLOG.//0.91:48:35//MYCOBACTERIUM LEPRAE.//P38388
F-NT2RP3000449//HOMEOBOX PROTEIN HOX-B8 (CHOX-2.4) (FRAGMENT).//1.0:42:33//GALLUS GALLUS (CHICKEN).//P23681
F-NT2RP3000451
F-NT2RP3000456//COLLAGEN ALPHA 1(I) CHAIN
(FRAGMENTS).//0.00018:178:36//RATTUS NORVEGICUS (RAT).//P02454
F-NT2RP3000484//METALLOTHIONEIN-III (MT-III) (GROWTH INHIBITORY FACTOR) (GIF).//0.098:40:27//BOS TAURUS (BOVINE).//P37359
F-NT2RP3000487//SULFATED SURFACE GLYCOPROTEIN 185 (SSG 185).//0.00037:16:81//VOLVOX CARTERI.//P21997
F-NT2RP3000512
F-NT2RP3000526//HYPOTHETICAL NIN REGION PROTEIN ORF56.//0.51:37:43//BACTERIOPHAGE LAMBDA.//P03769
F-NT2RP3000527//ZINC FINGER PROTEIN 83 (ZINC FINGER PROTEIN HPF1).//1.0e-16:234:30//HOMO SAPIENS (HUMAN).//P51522
F-NT2RP3000531//POLIOVIRUS RECEPTOR PRECURSOR (CD155 ANTIGEN).//3.4e-15:192:30//HOMO SAPIENS (HUMAN).//P15151
F-NT2RP3000542//CYTOCHROME C OXIDASE POLYPEPTIDE II (EC 1.9.3.1) (FRAGMENT).//0.60:51:39//ASTERINA PECTINIFERA (STARFISH).//P11958
F-NT2RP3000561//HYPOTHETICAL ATP-BINDING PROTEIN MJ0423.//0.79:53:32//METHANOCOCCUS JANNASCHII.//Q57866
F-NT2RP3000562//ACCESSORY GLAND PEPTIDE PRECURSOR (PARAGONIAL PEPTIDE B).//0.99:26:34//DROSOPHILA MAURITIANA (FRUIT FLY), AND DROSOPHILA SIMULANS (FRUIT FLY).//O18666
F-NT2RP3000578//HYPOTHETICAL 49.8 KD PROTEIN IN RPL14B-GPA1 INTERGENIC REGION.//1.5e-26:127:37//SACCHAROMYCES CEREVISIAE (BAKER'S YEAST).//P38755
F-NT2RP3000582
F-NT2RP3000584//METALLOTHIONEIN-II (MT-II).//0.28:27:29//MUS MUSCULUS (MOUSE).//P02798
F-NT2RP3000590//UVS-2 PROTEIN.//4.8e-10:113:33//NEUROSPORA CRASSA.//P33288
F-NT2RP3000592//TRANSCRIPTION INITIATION FACTOR TFIID 135 KD SUBUNIT (TAFII-135) (TAFII135) (TAFII-130) (TAFII130).//0.00087:178:31//HOMO SAPIENS (HUMAN).//O00268
F-NT2RP3000596//YEMANUCLEIN-ALPHA.//1.8e-05:98:34//DROSOPHILA MELANOGASTER (FRUIT FLY).//P25992
F-NT2RP3000599//SPLICEOSOME ASSOCIATED PROTEIN 62 (SAP 62) (SF3A66).//0.00095:90:37//HOMO SAPIENS (HUMAN).//Q15428
F-NT2RP3000603//5E5 ANTIGEN.//1.0e-09:181:34//RATTUS NORVEGICUS (RAT).//Q63003
F-NT2RP3000605//STEROL REGULATORY ELEMENT BINDING PROTEIN-1 (SREBP-1) (STEROL REGULATORY ELEMENT-BINDING TRANSCRIPTION FACTOR 1).//0.00098:76:34//HOMO SAPIENS (HUMAN).//P36956
F-NT2RP3000622//HYPOTHETICAL PROTEIN MG096 HOMOLOG 5 (P02_ORF427).//0.15:52:36//MYCOPLASMA PNEUMONIAE.//P75277
F-NT2RP3000624//HYPOTHETICAL PROTEIN KIAA0256.//5.4e-16:222:31//HOMO SAPIENS (HUMAN).//Q93073
F-NT2RP3000628
F-NT2RP3000632//ZINC FINGER PROTEIN 90 (ZFP-90) (ZINC FINGER PROTEIN NK10).//2.0e-16:52:63//MUS MUSCULUS (MOUSE).//Q61967
F-NT2RP3000644//!!!! ALU SUBFAMILY SQ WARNING ENTRY !!!!//6.7e-40:102:79//HOMO SAPIENS (HUMAN).//P39194
F-NT2RP3000661//HYPOTHETICAL 139.1 KD PROTEIN C08B11.3 IN CHROMOSOME II.//6.0e-08:83:36//CAENORHABDITIS ELEGANS.//Q09441
F-NT2RP3000665//HOMEOBOX PROTEIN PROPHET OF PIT-1 (PROP-1) (PITUITARY SPECIFIC HOMEODOMAIN FACTOR).//0.13:48:35//HOMO SAPIENS (HUMAN).//O75360
F-NT2RP3000685//HYPOTHETICAL 33.5 KD PROTEIN IN CAT1 5'REGION (ORFY).//0.26:202:23//CLOSTRIDIUM KLUYVERI.//P38943
F-NT2RP3000690//INORGANIC PYROPHOSPHATASE (EC 3.6.1.1) (PYROPHOSPHATE PHOSPHO- HYDROLASE) (PPASE).//0.99:131:26//SACCHAROMYCES CEREVISIAE (BAKER'S YEAST).//P00817
F-NT2RP3000736//HYPOTHETICAL 28.7 KD PROTEIN IN RNR3-ARC15 INTERGENIC REGION.//3.5e-27:211:34//SACCHAROMYCES CEREVISIAE (BAKER'S YEAST).//P40516
F-NT2RP3000739//HYPOTHETICAL 33.5 KD PROTEIN C1D4.02C IN CHROMOSOME I.//6.0e-23:114:42//SCHIZOSACCHAROMYCES POMBE (FISSION YEAST).//Q10149
F-NT2RP3000742//1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III).//6.7e-12:85:36//RATTUS NORVEGICUS (RAT).//P10688
F-NT2RP3000753//CELL SURFACE GLYCOPROTEIN 1 PRECURSOR (OUTER LAYER PROTEIN B) (S-LAYER PROTEIN 1).//0.00011:208:28//CLOSTRIDIUM THERMOCELLUM.//Q06852
F-NT2RP3000759//ADP-RIBOSYLATION FACTOR 6.//8.1e-28:141:38//GALLUS GALLUS (CHICKEN).//P26990
F-NT2RP3000815//CYTOCHROME C-551 (C551) (CYTOCHROME C8).//0.24:45:37//PSEUDOMONAS DENITRIFICANS.//P00103
F-NT2RP3000825//ALPHA-LACTALBUMIN (LACTOSE SYNTHASE B PROTEIN (EC 2.4.1.22)).//0.82:51:39//MACROPUS RUFOGRISEUS (RED-NECKED WALLABY).//P07458
F-NT2RP3000826//COLLAGEN ALPHA 1(XII) CHAIN (FRAGMENTS).//0.025:79:37//BOS TAURUS (BOVINE).//P25508
F-NT2RP3000836//HYPOTHETICAL PROTEIN IN KSGA 3'REGION (ORF L5) (FRAGMENT).//0.85:36:47//MYCOPLASMA CAPRICOLUM.//P43040
F-NT2RP3000841//UDP-GLUCURONOSYLTRANSFERASE 1-7 PRECURSOR, MICROSOMAL (EC 2.4.1.17) (UDPGT) (UGT1*7) (UGT1-07) (UGT1.7) (UGT1A7) (UGTP4) (FRAGMENT).//1.0:70:34//MUS MUSCULUS (MOUSE).//Q62452
F-NT2RP3000845//PUTATIVE SERINE/THREONINE-PROTEIN KINASE P78 (EC 2.7.1.-).//5.2e-72:247:61//HOMO SAPIENS (HUMAN).//P27448
F-NT2RP3000847//HYPOTHETICAL PROTEIN KIAA0161.//0.037:55:30//HOMO SAPIENS (HUMAN).//P50876
F-NT2RP3000850//!!!! ALU SUBFAMILY SQ WARNING ENTRY !!!!//7.4e-31:90:75//HOMO SAPIENS (HUMAN).//P39194
F-NT2RP3000852//HYDROPHOBIC SEED PROTEIN (HPS).//0.33:23:69//GLYCINE MAX (SOYBEAN).//P24337
F-NT2RP3000859//IMMEDIATE-EARLY PROTEIN.//3.6e-07:189:25//HERPESVIRUS SAIMIRI (STRAIN 11).//Q01042
F-NT2RP3000865
F-NT2RP3000868//MYOSIN HEAVY CHAIN, CARDIAC MUSCLE ISOFORM (FRAGMENT).//1.4e-09:232:28//GALLUS GALLUS (CHICKEN).//P29616
F-NT2RP3000869//CUTICLE COLLAGEN 2.//4.5e-08:58:46//CAENORHABDITIS ELEGANS.//P17656
F-NT2RP3000875//HOMEOBOX PROTEIN CDX-2 (CAUDAL-TYPE HOMEOBOX PROTEIN 2).//0.90:62:37//MUS MUSCULUS (MOUSE).//P43241
F-NT2RP3000901//COLLAGEN ALPHA 1(I) CHAIN (FRAGMENTS).//0.99:124:33//BOS TAURUS (BOVINE).//P02453
F-NT2RP3000904
F-NT2RP3000917//DHP1 PROTEIN.//6.5e-60:229:55//SCHIZOSACCHAROMYCES POMBE (FISSION YEAST).//P40848
F-NT2RP3000919//HYPOTHETICAL 33.5 KD PROTEIN C1D4.02C IN CHROMOSOME I.//2.4e-19:159:34//SCHIZOSACCHAROMYCES POMBE (FISSION YEAST).//Q10149
F-NT2RP3000968//40S RIBOSOMAL PROTEIN S15A.//3.7e-48:73:98//HOMO SAPIENS (HUMAN), AND RATTUS NORVEGICUS (RAT).//P39027
F-NT2RP3000980//COPA/INCA PROTEIN (REPA3 PROTEIN).//0.24:19:47//ESCHERICHIA COLI.//P13946
F-NT2RP3000994//MATERNAL EFFECT PROTEIN STAUFEN.//1.4e-10:78:48//DROSOPHILA MELANOGASTER (FRUIT FLY).//P25159
F-NT2RP3001004//HYPOTHETICAL 7.6 KD PROTEIN B0563.8 IN CHROMOSOME X.//0.70:50:32//CAENORHABDITIS ELEGANS.//Q11084
F-NT2RP3001007
F-NT2RP3001055//N-TERMINAL ACETYLTRANSFERASE COMPLEX ARD1 SUBUNIT HOMOLOG.//1.3e-05:138:28//DICTYOSTELIUM DISCOIDEUM (SLIME MOLD).//P36416
F-NT2RP3001057//ZINC FINGER PROTEIN 45 (BRC1744).//4.0e-28:141:51//HOMO SAPIENS (HUMAN).//Q02386
F-NT2RP3001081//HYPOTHETICAL 46.4 KD PROTEIN T16H12.5 IN CHROMOSOME III.//3.8e-08:144:29//CAENORHABDITIS ELEGANS.//P34568
F-NT2RP3001084//SPIDROIN 2 (DRAGLINE SILK FIBROIN 2) (FRAGMENT).//3.4e-06:217:32//NEPHILA CLAVIPES (ORB SPIDER).//P46804
F-NT2RP3001096//SYNAPTONEMAL COMPLEX PROTEIN SC65.//1.1e-30:244:33//RATTUS NORVEGICUS (RAT).//Q64375
F-NT2RP3001107//ARYLSULFATASE F (EC 3.1.6.-) (ASF) (FRAGMENT).//0.041:47:44//HOMO SAPIENS (HUMAN).//P54793
F-NT2RP3001109
F-NT2RP3001111//MALE SPECIFIC SPERM PROTEIN MST84DC.//0.17:28:39//DROSOPHILA MELANOGASTER (FRUIT FLY).//Q01644
F-NT2RP3001113//INVOLUCRIN.//0.00036:192:23//MUS MUSCULUS (MOUSE).//P48997
F-NT2RP3001115
F-NT2RP3001116//AMINOPEPTIDASE G (EC 3.4.11.-) (FRAGMENT).//0.99:29:51//STREPTOMYCES LIVIDANS.//Q54340
F-NT2RP3001119//COLLAGEN ALPHA 4(IV) CHAIN (FRAGMENT).//0.0015:73:39//BOS TAURUS (BOVINE).//Q29442
F-NT2RP3001120//ZINC FINGER PROTEIN ZFP-36 (FRAGMENT).//1.3e-57:229:52//HOMO SAPIENS (HUMAN).//P16415
F-NT2RP3001126//HYPOTHETICAL 91.2 KD PROTEIN IN RPS4B-SCH9 INTERGENIC REGION.//2.8e-07:83:34//SACCHAROMYCES CEREVISIAE (BAKER'S YEAST).//P38888
F-NT2RP3001133//CALCIUM BINDING PROTEIN.//2.0e-08:171:32//DICTYOSTELIUM DISCOIDEUM (SLIME MOLD).//P35085
F-NT2RP3001140//F-SPONDIN PRECURSOR.//2.0e-147:244:97//RATTUS NORVEGICUS (RAT).//P35446
F-NT2RP3001147//TROPOMYOSIN 2 (TMII).//0.11:159:23//SCHISTOSOMA MANSONI (BLOOD FLUKE).//P42638
F-NT2RP3001150//OCTAPEPTIDE-REPEAT PROTEIN T2.//6.2e-09:163:25//MUS MUSCULUS (MOUSE).//Q06666
F-NT2RP3001155//DNA POLYMERASE ALPHA-BINDING PROTEIN (POB1/CTF4 PROTEIN) (CHROMOSOME REPLICATION PROTEIN CHL15).//4.1e-05:244:23//SACCHAROMYCES CEREVISIAE (BAKER'S YEAST).//Q01454
F-NT2RP3001176//LEUKOSIALIN PRECURSOR (LEUCOCYTE SIALOGLYCOPROTEIN) (SIALOPHORIN) (CD43) (LY 48) (B CELL DIFFERENTIATION ANTIGEN LP-3).//0.21:136:26//MUS MUSCULUS (MOUSE).//P15702
F-NT2RP3001214//SAP1 PROTEIN.//0.058:133:30//SACCHAROMYCES CEREVISIAE (BAKER'S YEAST).//P39955
F-NT2RP3001216//CYLICIN I (MULTIPLE-BAND POLYPEPTIDE I) (FRAGMENT).//2.1e-08:137:33//HOMO SAPIENS (HUMAN).//P35663
F-NT2RP3001221//GAMMA-BUTYROBETAINE,2-OXOGLUTARATE DIOXYGENASE (EC 1.14.11.1) (GAMMA-BUTYROBETAINE HYDROXYLASE).//4.2e-05:131:26//PSEUDOMONAS SP. (STRAIN AK-1).//P80193
F-NT2RP3001232//HYPOTHETICAL PROTEIN PRECURSOR IN CS5 3'REGION (FRAGMENT).//0.75:57:31//ESCHERICHIA COLI.//P33792
F-NT2RP3001236//TRANSFORMING PROTEIN MAF.//0.017:136:30//AVIAN MUSCULOAPONEUROTIC FIBROSARCOMA VIRUS AS42.//P23091
F-NT2RP3001239//ELECTROMOTOR NEURON-ASSOCIATED PROTEIN 1 (FRAGMENT).//4.2e-55:221:49//TORPEDO CALIFORNICA (PACIFIC ELECTRIC RAY).//P14400
F-NT2RP3001245
F-NT2RP3001253//TROPOMYOSIN 2, MUSCLE THORACIC ISOFORM (TROPOMYOSIN I).//0.0042:142:24//DROSOPHILA MELANOGASTER (FRUIT FLY).//P09491
F-NT2RP3001260//COLLAGEN ALPHA 4(IV) CHAIN PRECURSOR.//0.0011:89:43//HOMO SAPIENS (HUMAN).//P53420
F-NT2RP3001268//ZINC FINGER PROTEIN 45 (BRC1744).//9.0e-29:194:44//HOMO SAPIENS (HUMAN).//Q02386
F-NT2RP3001272//HYPOTHETICAL 75.2 KD PROTEIN C13F4.08C IN CHROMOSOME I.//8.2e-17:183:26//SCHIZOSACCHAROMYCES POMBE (FISSION YEAST).//Q10199
F-NT2RP3001274//SERINE/THREONINE PROTEIN PHOSPHATASE 5 (EC 3.1.3.16) (PP5) (PROTEIN PHOSPHATASE T) (PPT) (FRAGMENT).//1.7e-09:78:39//MUS MUSCULUS (MOUSE).//Q60676
F-NT2RP3001281//!!!! ALU SUBFAMILY J WARNING ENTRY !!!!//7.7e-08:38:71//HOMO SAPIENS (HUMAN).//P39188
F-NT2RP3001297//HYPOTHETICAL PROTEIN KIAA0281 (HA6725).//2.2e-57:159:70//HOMO SAPIENS (HUMAN).//Q92556
F-NT2RP3001307//SPERM PROTAMINE P1.//0.21:46:39//ORNITHORHYNCHUS ANATINUS (DUCKBILL PLATYPUS).//P35307
F-NT2RP3001318
F-NT2RP3001325//ENHANCER OF RUDIMENTARY HOMOLOG.//1.0:73:24//BRACHYDANIO RERIO (ZEBRAFISH) (ZEBRA DANIO).//Q98874
F-NT2RP3001338//ZINC FINGER PROTEIN 29 (ZINC FINGER PROTEIN KOX26) (FRAGMENT).//0.0021:56:35//HOMO SAPIENS (HUMAN).//P17037
F-NT2RP3001339//CITRON PROTEIN.//3.6e-06:90:33//MUS MUSCULUS (MOUSE).//P49025
F-NT2RP3001340//HYPOTHETICAL PROTEIN UL61.//7.2e-11:202:34//HUMAN CYTOMEGALOVIRUS (STRAIN AD169).//P16818
F-NT2RP3001355//TRICARBOXYLATE TRANSPORT PROTEIN PRECURSOR (CITRATE TRANSPORT PROTEIN) (CTP) (TRICARBOXYLATE CARRIER PROTEIN).//7.7e-16:129:33//HOMO SAPIENS (HUMAN).//P53007
F-NT2RP3001356//RAS-RELATED PROTEIN RABA (FRAGMENT).//0.00041:66:28//DICTYOSTELIUM DISCOIDEUM (SLIME MOLD).//P34141
F-NT2RP3001374
F-NT2RP3001383//PTB-ASSOCIATED SPLICING FACTOR (PSF).//2.5e-06:190:32//HOMO SAPIENS (HUMAN).//P23246
F-NT2RP3001384//CHORION PROTEIN S15.//0.00079:94:37//DROSOPHILA VIRILIS (FRUIT FLY).//P13424
F-NT2RP3001392//VPU PROTEIN (ORF-X PROTEIN) (UPX PROTEIN).//1.0:22:45//CAPRINE ARTHRITIS ENCEPHALITIS VIRUS (CAEV).//P31834
F-NT2RP3001396//HYPOTHETICAL 8.1 KD PROTEIN (ORF4).//1.0:37:32//STRAWBERRY MILD YELLOW EDGE-ASSOCIATED VIRUS (SMYEAV).//Q00848
F-NT2RP3001398//KRUEPPEL-RELATED ZINC FINGER PROTEIN 2 (HKR2 PROTEIN) (FRAGMENT).//1.9e-08:45:37//HOMO SAPIENS (HUMAN).//P10073
F-NT2RP3001399//SSU72 PROTEIN.//7.3e-18:84:52//SACCHAROMYCES CEREVISIAE (BAKER'S YEAST).//P53538
F-NT2RP3001407//SCY1 PROTEIN.//1.5e-08:143:25//SACCHAROMYCES CEREVISIAE (BAKER'S YEAST).//P53009
F-NT2RP3001420//HYPOTHETICAL 7.9 KD PROTEIN.//0.25:41:26//VACCINIA VIRUS (STRAIN COPENHAGEN).//P20542
F-NT2RP3001426//DNAJ PROTEIN.//7.5e-15:78:43//HAEMOPHILUS INFLUENZAE.//P43735
F-NT2RP3001427//WERNER SYNDROME HELICASE.//3.6e-13:159:33//HOMO SAPIENS (HUMAN).//Q14191
F-NT2RP3001428//NUCLEOPROTEIN TPR.//1.8e-53:117:99//HOMO SAPIENS (HUMAN).//P12270
F-NT2RP3001432//NADH-UBIQUINONE OXIDOREDUCTASE CHAIN 5 (EC 1.6.5.3) (FRAGMENT).//0.96:52:21//TARSIUS SYRICHTA (TARSIER).//Q36151
F-NT2RP3001447//HYPOTHETICAL 5.5 KD PROTEIN IN REPLICATION ORIGIN REGION (ORF1).//0.96:45:35//ESCHERICHIA COLI.//P14505
F-NT2RP3001449//HOMEOBOX PROTEIN SAX-1 (CHOX-3) (FRAGMENT).//0.0043:53:43//GALLUS GALLUS (CHICKEN).//P19601
F-NT2RP3001453//MALE SPECIFIC SPERM PROTEIN MST84DB.//0.0048:65:40//DROSOPHILA MELANOGASTER (FRUIT FLY).//Q01643
F-NT2RP3001457//VACUOLAR PROTEIN SORTING-ASSOCIATED PROTEIN VPS28.//0.55:121:20//SACCHAROMYCES CEREVISIAE (BAKER'S YEAST).//Q02767
F-NT2RP3001459//MYOSIN IC HEAVY CHAIN.//0.10:126:34//ACANTHAMOEBA CASTELLANII (AMOEBA).//P10569
F-NT2RP3001472//NONHISTONE CHROMOSOMAL PROTEIN 6A.//3.0e-14:87:43//SACCHAROMYCES CEREVISIAE (BAKER'S YEAST).//P11632
F-NT2RP3001490//METALLOTHIONEIN-LIKE PROTEIN LSC54.//1.0:39:35//BRASSICA NAPUS (RAPE).//P43402
F-NT2RP3001495//UBIQUITIN--PROTEIN LIGASE RSP5 (EC 6.3.2.-).//3.3e-14:148:35//SACCHAROMYCES CEREVISIAE (BAKER'S YEAST).//P39940
F-NT2RP3001497//COLLAGEN ALPHA 1(XII) CHAIN (FRAGMENTS).//0.13:44:38//BOS TAURUS (BOVINE).//P25508
F-NT2RP3001527//SPERM PROTAMINE P1.//0.35:29:37//DIDELPHIS MARSUPIALIS VIRGINIANA (NORTH AMERICAN OPOSSUM), AND MONODELPHIS DOMESTICA (SHORT-TAILED GREY OPOSSUM).//P35305
F-NT2RP3001529//HYPOTHETICAL 43.3 KD GTP-BINDING PROTEIN IN DACB-RPMA INTERGENIC REGION.//3.3e-21:125:37//ESCHERICHIA COLI.//P42641
F-NT2RP3001538//HNF3/FH TRANSCRIPTION FACTOR GENESIS (WINGED HELIX PROTEIN CWH-3).//0.13:53:39//GALLUS GALLUS (CHICKEN).//P79772
F-NT2RP3001554//ELECTROMOTOR NEURON-ASSOCIATED PROTEIN 2 (FRAGMENT).//2.3e-48:137:52//TORPEDO CALIFORNICA (PACIFIC ELECTRIC RAY).//P14401
F-NT2RP3001580//GERM CELL-LESS PROTEIN.//8.2e-18:100:42//DROSOPHILA MELANOGASTER (FRUIT FLY).//Q01820
F-NT2RP3001587//UBIQUITIN-ACTIVATING ENZYME E1-LIKE (POLYMERASE-INTERACTING PROTEIN 2).//2.0e-47:188:51//SACCHAROMYCES CEREVISIAE (BAKER'S YEAST).//P52488
F-NT2RP3001589//!!!! ALU SUBFAMILY SP WARNING ENTRY !!!!//7.4e-41:87:80//HOMO SAPIENS (HUMAN).//P39193
F-NT2RP3001607//ATP SYNTHASE PROTEIN 8 (EC 3.6.1.34) (A6L).//1.0:49:32//DICENTRARCHUS LABRAX (EUROPEAN SEA BASS).//Q36362
F-NT2RP3001608//EXTENSIN PRECURSOR (PROLINE-RICH GLYCOPROTEIN).//0.0013:177:25//ZEA MAYS (MAIZE).//P14918
F-NT2RP3001621//MALE SPECIFIC SPERM PROTEIN MST84DD.//0.84:29:37//DROSOPHILA MELANOGASTER (FRUIT FLY).//Q01645
F-NT2RP3001629//RAS-RELATED C3 BOTULINUM TOXIN SUBSTRATE 1 (P21-RAC1) (FRAGMENTS).//0.91:57:24//CAVIA PORCELLUS (GUINEA PIG).//P80236
F-NT2RP3001634//!!!! ALU SUBFAMILY SB WARNING ENTRY !!!!//8.9e-11:73:54//HOMO SAPIENS (HUMAN).//P39189
F-NT2RP3001642//HYPOTHETICAL PROTEIN KIAA0210.//1.1e-12:117:29//HOMO SAPIENS (HUMAN).//Q92609
F-NT2RP3001646//HYPOTHETICAL 29.3 KD PROTEIN (ORF92).//0.0092:69:34//ORGYIA PSEUDOTSUGATA MULTICAPSID POLYHEDROSIS VIRUS (OPMNPV).//O10341
F-NT2RP3001671//RING CANAL PROTEIN (KELCH PROTEIN).//0.0042:55:41//DROSOPHILA MELANOGASTER (FRUIT FLY).//Q04652
F-NT2RP3001672
F-NT2RP3001676//GTP-BINDING PROTEIN LEPA (FRAGMENT).//1.2e-15:56:62//PSEUDOMONAS FLUORESCENS.//P26843
F-NT2RP3001678//SPIDROIN 2 (DRAGLINE SILK FIBROIN 2) (FRAGMENT).//0.054:187:31//NEPHILA CLAVIPES (ORB SPIDER).//P46804
F-NT2RP3001679//HYPOTHETICAL 68.7 KD PROTEIN ZK757.1 IN CHROMOSOME III.//1.5e-07:63:44//CAENORHABDITIS ELEGANS.//P34679
F-NT2RP3001688//GLUCOAMYLASE S1 PRECURSOR (EC 3.2.1.3) (GLUCAN 1,4-ALPHA-GLUCOSIDASE) (1,4-ALPHA-D-GLUCAN GLUCOHYDROLASE) (GAI).//1.0:83:28//SACCHAROMYCES DIASTATICUS (YEAST).//P04065
F-NT2RP3001690//MYOSIN HEAVY CHAIN, CARDIAC MUSCLE BETA ISOFORM.//0.021:247:24//HOMO SAPIENS (HUMAN).//P12883
F-NT2RP3001698
F-NT2RP3001708//TWISTED GASTRULATION PROTEIN PRECURSOR.//7.7e-12:73:43//DROSOPHILA MELANOGASTER (FRUIT FLY).//P54356
F-NT2RP3001712//CEC-1 PROTEIN.//1.9e-07:121:29//CAENORHABDITIS ELEGANS.//P34618
F-NT2RP3001716//SALIVARY GLUE PROTEIN SGS-3 PRECURSOR.//0.89:54:40//DROSOPHILA SIMULANS (FRUIT FLY).//P13729
F-NT2RP3001724//CHROMODOMAIN-HELICASE-DNA-BINDING PROTEIN 1 (CHD-1).//7.5e-41:164:48//HOMO SAPIENS (HUMAN).//O14646
F-NT2RP3001727//HYPOTHETICAL 37.7 KD PROTEIN ZK686.3 IN CHROMOSOME III.//1.5e-51:240:41//CAENORHABDITIS ELEGANS.//P34669
F-NT2RP3001730//SEPTIN 2 HOMOLOG (FRAGMENT).//2.4e-122:267:86//HOMO SAPIENS (HUMAN).//Q14141
F-NT2RP3001739//INTESTINAL SODIUM/DICARBOXYLATE COTRANSPORTER (NA(+)/DICARBOXYLATE COTRANSPORTER).//0.99:63:34//RATTUS NORVEGICUS (RAT).//P70545
F-NT2RP3001752//!!!! ALU SUBFAMILY SP WARNING ENTRY !!!!//4.0e-21:60:85//HOMO SAPIENS (HUMAN).//P39193
F-NT2RP3001753//HYPOTHETICAL PROTEIN KIAA0127.//7.9e-12:83:44//HOMO SAPIENS (HUMAN).//Q14140
F-NT2RP3001764//DUAL SPECIFICITY PROTEIN PHOSPHATASE 6 (EC 3.1.3.48) (EC 3.1.3.16) (DUAL SPECIFICITY PROTEIN PHOSPHATASE PYST1).//7.7e-25:146:36//HOMO SAPIENS (HUMAN).//Q16828
F-NT2RP3001777//SERINE/THREONINE-PROTEIN KINASE STE20 HOMOLOG (EC 2.7.1.-).//0.0096:204:25//CANDIDA ALBICANS (YEAST).//Q92212
F-NT2RP3001782//ATP SYNTHASE PROTEIN 8 (EC 3.6.1.34) (A6L).//0.91:34:44//PONGO PYGMAEUS ABELII (SUMATRAN ORANGUTAN).//P92694
F-NT2RP3001792//HETEROGENEOUS NUCLEAR RIBONUCLEOPROTEIN M (HNRNP M).//1.8e-33:159:53//HOMO SAPIENS (HUMAN).//P52272
F-NT2RP3001799//LIGHT-HARVESTING PROTEIN B800/830/1020, ALPHA-2 CHAIN (EHS-ALPHA-2) (ANTENNA PIGMENT PROTEIN, ALPHA-2 CHAIN).//0.14:46:28//ECTOTHIORHODOSPIRA HALOCHLORIS.//P80103
F-NT2RP3001819//PROCOLLAGEN ALPHA 2(I) CHAIN PRECURSOR.//0.00030:77:36//HOMO SAPIENS (HUMAN).//P08123
F-NT2RP3001844//OCTAMER-BINDING TRANSCRIPTION FACTOR 1 (OTF-1) (NF-A1) (FRAGMENT).//0.99:43:34//MACROPUS EUGENII (TAMMAR WALLABY).//Q28466
F-NT2RP3001854//FIBRINOGEN- AND IG-BINDING PROTEIN PRECURSOR (MRP PROTEIN).//9.3e-10:213:24//STREPTOCOCCUS PYOGENES.//P30141
F-NT2RP3001855//HOMEOBOX PROTEIN PKNOX1 (HOMEOBOX PROTEIN PREP-1).//2.6e-61:220:60//HOMO SAPIENS (HUMAN).//P55347
F-NT2RP3001857//VEGETATIBLE INCOMPATIBILITY PROTEIN HET-E-1.//1.0e-13:213:24//PODOSPORA ANSERINA.//Q00808
F-NT2RP3001896//SPLICEOSOME ASSOCIATED PROTEIN 62 (SAP 62) (SF3A66).//0.074:124:34//HOMO SAPIENS (HUMAN).//Q15428
F-NT2RP3001898//REGULATORY PROTEIN E2.//0.36:131:29//CANINE ORAL PAPILLOMAVIRUS (COPV).//Q89420
F-NT2RP3001915//CHITIN BIOSYNTHESIS PROTEIN CHS5 (CAL3 PROTEIN).//0.0021:237:23//SACCHAROMYCES CEREVISIAE (BAKER'S YEAST).//Q12114
F-NT2RP3001926//HYPOTHETICAL 14.0 KD PROTEIN IN RPL15B-GCR3 INTERGENIC REGION.//1.0:63:34//SACCHAROMYCES CEREVISIAE (BAKER'S YEAST).//Q03880
F-NT2RP3001929//!!!! ALU SUBFAMILY SX WARNING ENTRY !!!!//1.4e-14:35:60//HOMO SAPIENS (HUMAN).//P39195
F-NT2RP3001931//HYPOTHETICAL 59.3 KD PROTEIN IN TAP42-ARP9 INTERGENIC REGION.//0.86:162:24//SACCHAROMYCES CEREVISIAE (BAKER'S YEAST).//Q05040
F-NT2RP3001938//GLYCOPROTEIN GP50.//0.0036:54:40//PSEUDORABIES VIRUS (STRAIN RICE) (PRV).//P07645
F-NT2RP3001943//33.2 KD PROTEIN IN DIND-RPH INTERGENIC REGION (ORF X).//1.0:113:27//ESCHERICHIA COLI.//P23839
F-NT2RP3001944//HYPOTHETICAL 47.6 KD PROTEIN C16C10.5 IN CHROMOSOME III.//4.1e-56:208:47//CAENORHABDITIS ELEGANS.//Q09251
F-NT2RP3001969//PUFF II/9-2 PROTEIN PRECURSOR.//0.0078:149:26//SCIARA COPROPHILA (FUNGUS GNAT).//P22312
F-NT2RP3001989//SPERM PROTAMINE P1 (CYSTEINE-RICH PROTAMINE).//1.0:41:31//MUS MUSCULUS (MOUSE).//P02319
F-NT2RP3002002//!!!! ALU SUBFAMILY SX WARNING ENTRY !!!!//1.2e-44:69:79//HOMO SAPIENS (HUMAN).//P39195
F-NT2RP3002004//TRANSCRIPTION FACTOR BF-2 (BRAIN FACTOR 2) (BF2).//0.00024:45:40//MUS MUSCULUS (MOUSE).//Q61345
F-NT2RP3002007//TENASCIN PRECURSOR (TN) (HEXABRACHION) (CYTOTACTIN) (NEURONECTIN) (GMEM) (JI) (MIOTENDINOUS ANTIGEN) (GLIOMA-ASSOCIATED-EXTRACELLULAR MATRIX ANTIGEN) (GP 150-225) (TENASCIN-C).//0.21:115:28//HOMO SAPIENS (HUMAN).//P24821
F-NT2RP3002014//HYPOTHETICAL 32.0 KD PROTEIN C09F5.2 IN CHROMOSOME III.//1.7e-25:139:48//CAENORHABDITIS ELEGANS.//Q09232
F-NT2RP3002033//ACTIVATOR OF APOPTOSIS HARAKIRI (NEURONAL DEATH PROTEIN DP5).//0.14:65:41//HOMO SAPIENS (HUMAN).//O00198
F-NT2RP3002045//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT).//8.1e-108:192:98//MUS MUSCULUS (MOUSE).//P17427
F-NT2RP3002054//HYPOTHETICAL 35.5 KD PROTEIN IN TRANSPOSON TN4556.//0.046:176:31//STREPTOMYCES FRADIAE.//P20186
F-NT2RP3002056//140 KD NUCLEOLAR PHOSPHOPROTEIN (NOPP140).//1.4e-07:245:25//RATTUS NORVEGICUS (RAT).//P41777
F-NT2RP3002057//SMALL HYDROPHOBIC PROTEIN.//1.0:12:66//SIMIAN VIRUS 5 (STRAIN W3) (SV5).//P07577
F-NT2RP3002062//PROTEASE A INHIBITOR 3 (PROTEINASE INHIBITOR I(A)3).//1.0:49:32//SACCHAROMYCES CEREVISIAE (BAKER'S YEAST).//P01094
F-NT2RP3002063//ACYL CARRIER PROTEIN (ACP).//0.99:38:31//HAEMOPHILUS INFLUENZAE.//P43709
F-NT2RP3002081//HYPOTHETICAL 100.5 KD PROTEIN C1B9.04 IN CHROMOSOME I.//5.8e-35:253:37//SCHIZOSACCHAROMYCES POMBE (FISSION YEAST).//Q10429
F-NT2RP3002097//HYPOTHETICAL 98.1 KD PROTEIN IN SPX19-GCR2 INTERGENIC REGION.//6.2e-06:99:31//SACCHAROMYCES CEREVISIAE (BAKER'S YEAST).//P40164
F-NT2RP3002102//HYPOTHETICAL 7.4 KD PROTEIN.//0.68:34:47//THERMOPROTEUS TENAX VIRUS 1 (STRAIN KRA1) (TTV1).//P19302
F-NT2RP3002108//HYPOTHETICAL 105.5 KD PROTEIN R13F6.10 IN CHROMOSOME III.//7.9e-19:179:34//CAENORHABDITIS ELEGANS.//Q21986
F-NT2RP3002142//!!!! ALU SUBFAMILY J WARNING ENTRY !!!!//2.6e-17:37:75//HOMO SAPIENS (HUMAN).//P39188
F-NT2RP3002146//CUTICLE COLLAGEN 40.//0.00034:90:37//CAENORHABDITIS ELEGANS.//P34804
F-NT2RP3002147//SALIVARY PROLINE-RICH PROTEIN PO PRECURSOR (ALLELE S).//0.011:166:28//HOMO SAPIENS (HUMAN).//P10163
F-NT2RP3002151//G1 TO S PHASE TRANSITION PROTEIN 1 HOMOLOG (GTP-BINDING PROTEIN GST1-HS).//4.8e-11:60:53//HOMO SAPIENS (HUMAN).//P15170
F-NT2RP3002163//TRANSCRIPTION INITIATION FACTOR TFIID 135 KD SUBUNIT (TAFII-135) (TAFII135) (TAFII-130) (TARII130).//0.028:191:29//HOMO SAPIENS (HUMAN).//O00268
F-NT2RP3002165//TRANSCRIPTIONAL REGULATOR PROTEIN HCNGP.//2.3e-131:223:91//MUS MUSCULUS (MOUSE).//Q02614
F-NT2RP3002166//D-ALANYL CARRIER PROTEIN (DCP).//1.0:65:33//LACTOBACILLUS CASEI.//P55153
F-NT2RP3002173//!!!! ALU SUBFAMILY SQ WARNING ENTRY !!!!//2.4e-26:114:62//HOMO SAPIENS (HUMAN).//P39194
F-NT2RP3002181//MALE SPECIFIC SPERM PROTEIN MST84DD.//0.25:31:38//DROSOPHILA MELANOGASTER (FRUIT FLY).//Q01645
F-NT2RP3002244//SPERM PROTAMINE P1 (CYSTEINE-RICH PROTAMINE).//0.069:16:62//OVIS ARIES (SHEEP), AND CAPRA HIRCUS (GOAT).//P04102
F-NT2RP3002248//MICROFIBRILLAR-ASSOCIATED PROTEIN 1 (ASSOCIATED MICROFIBRIL PROTEIN) (AMF).//0.0079:187:24//GALLUS GALLUS (CHICKEN).//P55080
F-NT2RP3002255//PROLINE-RICH PROTEIN MP-3 (FRAGMENT).//4.6e-10:168:34//MUS MUSCULUS (MOUSE).//P05143
F-NT2RP3002273//SCD6 PROTEIN.//1.5e-11:160:33//SACCHAROMYCES CEREVISIAE (BAKER'S YEAST).//P45978
F-NT2RP3002276//PROBABLE E4 PROTEIN.//0.91:54:29//HUMAN PAPILLOMAVIRUS TYPE 16.//P06922
F-NT2RP3002303//HYPOTHETICAL 30.2 KD PROTEIN C4D7.04C IN CHROMOSOME I.//1.7e-42:191:43//SCHIZOSACCHAROMYCES POMBE (FISSION YEAST).//O14171
F-NT2RP3002304
F-NT2RP3002330//NNP-1 PROTEIN.//0.52:140:18//MUS MUSCULUS (MOUSE).//P56183
F-NT2RP3002343//5E5 ANTIGEN.//0.0056:189:30//RATTUS NORVEGICUS (RAT).//Q63003
F-NT2RP3002351//NAD-DEPENDENT METHYLENETETRAHYDROFOLATE DEHYDROGENASE (EC 1.5.1.15) / METHENYLTETRAHYDROFOLATE CYCLOHYDROLASE (EC 3.5.4.9) MITOCHONDRIAL PRECURSOR.//1.0e-66:196:68//HOMO SAPIENS (HUMAN).//P13995
F-NT2RP3002352//PRESYNAPTIC PROTEIN SAP102 (SYNAPSE-ASSOCIATED PROTEIN 102) (NEUROENDOCRINE-DLG) (NE-DLG).//0.79:173:27//HOMO SAPIENS (HUMAN).//Q92796
F-NT2RP3002377//PUTATIVE HELICASE YGR271W.//1.0e-56:216:44//SACCHAROMYCES CEREVISIAE (BAKER'S YEAST).//P53327
F-NT2RP3002399//MINICHROMOSOME MAINTENANCE PROTEIN 6.//1.4e-19:136:31//SACCHAROMYCES CEREVISIAE (BAKER'S YEAST).//P53091
F-NT2RP3002402//EBNA-6 NUCLEAR PROTEIN (EBNA-3C) (EBNA-4B).//0.74:107:36//EPSTEIN-BARR VIRUS (STRAIN B95-8) (HUMAN HERPESVIRUS 4).//P03204
F-NT2RP3002455//DNAJ PROTEIN (FRAGMENT).//5.6e-06:57:42//AGROBACTERIUM TUMEFACIENS.//P50018
F-NT2RP3002484//HYPOTHETICAL 46.5 KD PROTEIN C12B10.04 IN CHROMOSOME I.//0.00032:52:48//SCHIZOSACCHAROMYCES POMBE (FISSION YEAST).//Q10438
F-NT2RP3002501//HYPOTHETICAL 34.9 KD PROTEIN IN FRE2-JEN1 INTERGENIC REGION.//9.4e-42:209:42//SACCHAROMYCES CEREVISIAE (BAKER'S YEAST).//P36007
F-NT2RP3002512//HYPOTHETICAL 37.4 KD PROTEIN IN GPM1-MCR1 INTERGENIC REGION.//7.7e-32:162:37//SACCHAROMYCES CEREVISIAE (BAKER'S YEAST).//P36059
F-NT2RP3002529//PUTATIVE VACUOLAR PROTEIN SORTING-ASSOCIATED PROTEIN C2G11.03C.//2.1e-45:241:43//SCHIZOSACCHAROMYCES POMBE (FISSION YEAST).//Q09805
F-NT2RP3002545
F-NT2RP3002549//HYPOTHETICAL 26.6 KD PROTEIN T19C3.4 IN CHROMOSOME III.//2.8e-41:161:52//CAENORHABDITIS ELEGANS.//Q10010
F-NT2RP3002566//IMMEDIATE-EARLY PROTEIN IE180.//0.56:130:24//PSEUDORABIES VIRUS (STRAIN KAPLAN) (PRV).//P33479
F-NT2RP3002587
F-NT2RP3002590
F-NT2RP3002602//PROTEIN DISULFIDE ISOMERASE PRECURSOR (PDI) (EC 5.3.4.1) (THIOREDOXIN- RELATED GLYCOPROTEIN 1).//0.00091:111:28//SACCHAROMYCES CEREVISIAE (BAKER'S YEAST).//P17967
F-NT2RP3002603//HYPOTHETICAL 14.2 KD PROTEIN IN BLAB 3'REGION.//1.0:65:40//STREPTOMYCES CACAOI.//P33654
F-NT2RP3002628//DNAJ-LIKE PROTEIN SLR0093.//2.4e-17:101:44//SYNECHOCYSTIS SP. (STRAIN PCC 6803).//P50027
F-NT2RP3002631//METALLOTHIONEIN-IB (MT-1B).//0.092:36:33//HOMO SAPIENS (HUMAN).//P07438
F-NT2RP3002650//DUALIN.//3.0e-21:184:37//GALLUS GALLUS (CHICKEN).//Q90830
F-NT2RP3002659//PROCOLLAGEN ALPHA 2(I) CHAIN PRECURSOR.//0.00016:223:33//HOMO SAPIENS (HUMAN).//P08123
F-NT2RP3002660//40S RIBOSOMAL PROTEIN S27A.//0.16:72:31//CAENORHABDITIS ELEGANS.//P37165
F-NT2RP3002663//OXYSTEROL-BINDING PROTEIN.//5.4e-23:168:41//HOMO SAPIENS (HUMAN).//P22059
F-NT2RP3002671//HYPOTHETICAL 124.5 KD PROTEIN IN SKO1-RPL44A INTERGENIC REGION.//6.0e-38:203:43//SACCHAROMYCES CEREVISIAE (BAKER'S YEAST).//P53893
F-NT2RP3002682//NADH-UBIQUINONE OXIDOREDUCTASE CHAIN 4L (EC 1.6.5.3).//0.25:63:31//ARTEMIA SALINA (BRINE SHRIMP).//P19049
F-NT2RP3002687//HYPOTHETICAL 30.4 KD PROTEIN IN LEF3-IAP2 INTERGENIC REGION.//0.029:60:36//AUTOGRAPHA CALIFORNICA NUCLEAR POLYHEDROSIS VIRUS (ACMNPV).//P41469
F-NT2RP3002688//KINESIN-LIKE PROTEIN KIF1B.//5.3e-61:130:88//MUS MUSCULUS (MOUSE).//Q60575
F-NT2RP3002701//SPERM MITOCHONDRIAL CAPSULE SELENOPROTEIN (MCS).//7.4e-05:109:33//MUS MUSCULUS (MOUSE).//P15265
F-NT2RP3002713//PROBABLE ATP-DEPENDENT RNA HELICASE DDX10 (DEAH BOX PROTEIN 10).//0.77:70:32//HOMO SAPIENS (HUMAN).//Q13206
F-NT2RP3002763//HYPOTHETICAL 11.3 KD PROTEIN C2C6.07 IN CHROMOSOME I.//6.7e-11:66:40//SCHIZOSACCHAROMYCES POMBE (FISSION YEAST).//O14056
F-NT2RP3002770//COLLAGEN ALPHA 1(IX) CHAIN (FRAGMENT).//0.33:87:34//MUS MUSCULUS (MOUSE).//Q05722
F-NT2RP3002785//LETHAL(2)DENTICLELESS PROTEIN (DTL83 PROTEIN).//9.7e-36:187:39//DROSOPHILA MELANOGASTER (FRUIT FLY).//Q24371
F-NT2RP3002799//!!!! ALU SUBFAMILY J WARNING ENTRY!!!!//5.6e-08:41:73//HOMO SAPIENS (HUMAN).//P39188
F-NT2RP3002810//!!!! ALU SUBFAMILY SP WARNING ENTRY !!!!//0.0034:35:65//HOMO SAPIENS (HUMAN).//P39193
F-NT2RP3002818//MAJOR CENTROMERE AUTOANTIGEN B (CENTROMERE PROTEIN B) (CENP-B).//3.2e-17:148:37//MUS MUSCULUS (MOUSE).//P27790
F-NT2RP3002861//HYPOTHETICAL 70.2 KD PROTEIN IN GSH1-CHS6 INTERGENIC REGION.//1.7e-05:95:31//SACCHAROMYCES CEREVISIAE (BAKER'S YEAST).//P42951
F-NT2RP3002869//TRYPSIN INHIBITOR II (BDTI-II).//0.97:23:39//BRYONIA DIOICA (RED BRYONY).//P11968
F-NT2RP3002876//ACIDIC PROLINE-RICH PROTEIN PRECURSOR (CLONE PRP33).//0.00017:140:31//RATTUS NORVEGICUS (RAT).//P04474
F-NT2RP3002877//!!!! ALU SUBFAMILY SQ WARNING ENTRY !!!!//2.5e-06:55:60//HOMO SAPIENS (HUMAN).//P39194
F-NT2RP3002909//P53-BINDING PROTEIN 53BP2 (BCL2-BINDING PROTEIN) (BBP).//4.6e-08:129:38//HOMO SAPIENS (HUMAN).//Q13625
F-NT2RP3002911//HYPOTHETICAL PROTEIN C18.//0.99:26:50//SWINEPOX VIRUS (STRAIN KASZA) (SPV).//P32217
F-NT2RP3002948//RING CANAL PROTEIN (KELCH PROTEIN).//1.2e-23:113:47//DROSOPHILA MELANOGASTER (FRUIT FLY).//Q04652
F-NT2RP3002953//CADHERIN-RELATED TUMOR SUPPRESSOR PRECURSOR (FAT PROTEIN).//0.55:116:27//DROSOPHILA MELANOGASTER (FRUIT FLY).//P33450
F-NT2RP3002955//HYPOTHETICAL 16.5 KD PROTEIN IN BLTR-SPOIIIC INTERGENIC REGION.//0.87:67:37//BACILLUS SUBTILIS.//P54445
F-NT2RP3002969//LONG-CHAIN-FATTY-ACID--COA LIGASE 4 (EC 6.2.1.3) (LONG-CHAIN ACYL-COA SYNTHETASE 4) (LACS 4).//6.7e-56:189:59//HOMO SAPIENS (HUMAN).//O60488
F-NT2RP3002972//HYPOTHETICAL 73.0 KD PROTEIN IN CLA4-MID1 INTERGENIC REGION.//0.0028:147:27//SACCHAROMYCES CEREVISIAE (BAKER'S YEAST).//P48566
F-NT2RP3002978//PROBABLE E5 PROTEIN.//0.15:55:36//HUMAN PAPILLOMAVIRUS TYPE 51.//P26553
F-NT2RP3002985//METALLOTHIONEIN (MT).//0.0031:49:42//PLEURONECTES PLATESSA (PLAICE).//P07216
F-NT2RP3002988//NEUROGENIC LOCUS NOTCH HOMOLOG PROTEIN 1 PRECURSOR (MOTCH PROTEIN).//1.0:111:29//MUS MUSCULUS (MOUSE).//Q01705
F-NT2RP3003008//HYPOTHETICAL 54.7 KD PROTEIN F37A4.1 IN CHROMOSOME III.//0.96:112:25//CAENORHABDITIS ELEGANS.//P41879
F-NT2RP3003032
F-NT2RP3003059//HYPOTHETICAL 52.3 KD PROTEIN C56F8.06C IN CHROMOSOME I PRECURSOR.//9.7e-27:216:37//SCHIZOSACCHAROMYCES POMBE (FISSION YEAST).//Q10254
F-NT2RP3003061//ANKYRIN R (ANKYRINS 2.1 AND 2.2) (ERYTHROCYTE ANKYRIN).//3.7e-25:167:34//HOMO SAPIENS (HUMAN).//P16157
F-NT2RP3003068//SERYL-TRNA SYNTHETASE (EC 6.1.1.11) (SERINE--TRNA LIGASE) (SERRS) (FRAGMENT).//0.074:82:39//SULFOLOBUS SOLFATARICUS.//O33780
F-NT2RP3003071//VASODILATOR-STIMULATED PHOSPHOPROTEIN (VASP).//0.0085:128:30//HOMO SAPIENS (HUMAN).//P50552
F-NT2RP3003078//SPERM ACROSOMAL PROTEIN FSA-ACR.1 PRECURSOR (FRAGMENT).//0.028:165:31//VULPES VULPES (RED FOX).//P53353
F-NT2RP3003101//TETRACYCLINE RESISTANCE PROTEIN, CLASS C (TETA(C)).//1.0e-14:243:25//ESCHERICHIA COLI.//P02981
F-NT2RP3003121//SUPPRESSOR PROTEIN SRP40.//7.4e-05:143:27//SACCHAROMYCES CEREVISIAE (BAKER'S YEAST).//P32583
F-NT2RP3003133//65 KD YES-ASSOCIATED PROTEIN (YAP65).//0.024:61:42//GALLUS GALLUS (CHICKEN).//P46936
F-NT2RP3003138//KINESIN-LIKE PROTEIN KIF4.//1.1e-118:151:93//MUS MUSCULUS (MOUSE).//P33174
F-NT2RP3003139//ATP-BINDING CASSETTE TRANSPORTER ABC1.//1.0:70:30//SCHIZOSACCHAROMYCES POMBE (FISSION YEAST).//Q92337
F-NT2RP3003145//MILK FAT GLOBULE-EGF FACTOR 8 PRECURSOR (MFG-E8) (HMFG) (BREAST EPITHELIAL ANTIGEN BA46) (MFGM).//2.0e-12:121:37//HOMO SAPIENS (HUMAN).//Q08431
F-NT2RP3003150
F-NT2RP3003157//ZINC FINGER PROTEIN 83 (ZINC FINGER PROTEIN HPF1).//4.0e-79:260:54//HOMO SAPIENS (HUMAN).//P51522
F-NT2RP3003185//TROPOMYOSIN.//0.077:122:27//SCHIZOSACCHAROMYCES POMBE (FISSION YEAST).//Q02088
F-NT2RP3003193//ZINC FINGER PROTEIN 135.//7.2e-91:239:65//HOMO SAPIENS (HUMAN).//P52742
F-NT2RP3003197//HYPOTHETICAL 28.1 KD PROTEIN IN SIPU-PBPC INTERGENIC REGION.//1.3e-07:117:34//BACILLUS SUBTILIS.//P42966
F-NT2RP3003203//HYPOTHETICAL 33.5 KD PROTEIN C1D4.02C IN CHROMOSOME I.//9.9e-23:132:39//SCHIZOSACCHAROMYCES POMBE (FISSION YEAST).//Q10149
F-NT2RP3003204//RAS-LIKE PROTEIN RASB.//0.92:103:27//DICTYOSTELIUM DISCOIDEUM (SLIME MOLD).//P32252
F-NT2RP3003210//VERY HYPOTHETICAL 13.2 KD PROTEIN IN PTC3-SAS3 INTERGENIC REGION.//0.23:106:33//SACCHAROMYCES CEREVISIAE (BAKER'S YEAST).//P38190
F-NT2RP3003212//SUPPRESSOR PROTEIN SRP40.//0.019:171:23//SACCHAROMYCES CEREVISIAE (BAKER'S YEAST).//P32583
F-NT2RP3003230//CORONIN-LIKE PROTEIN P57.//8.3e-74:183:73//BOS TAURUS (BOVINE).//Q92176
F-NT2RP3003242//STANNIOCALCIN PRECURSOR.//1.4e-21:127:37//HOMO SAPIENS (HUMAN).//P52823
F-NT2RP3003251//DOWN REGULATORY PROTEIN OF INTERLEUKIN 2 RECEPTOR.//3.1e-51:198:52//MUS MUSCULUS (MOUSE).//P15533
F-NT2RP3003264//E6 PROTEIN.//1.0:31:41//HUMAN PAPILLOMAVIRUS TYPE 48.//Q80920
F-NT2RP3003278//45.8 KD PROTEIN IN SHM1-MRPL37 INTERGENIC REGION.//8.6e-07:80:33//SACCHAROMYCES CEREVISIAE (BAKER'S YEAST).//P38344
F-NT2RP3003282//DYNAMIN 2 (DYNAMIN UDNM).//8.0e-108:226:88//MUS MUSCULUS (MOUSE).//P39054
F-NT2RP3003290//BIOH PROTEIN.//0.0055:107:30//ESCHERICHIA COLI.//P13001
F-NT2RP3003301//MITOCHONDRIAL LON PROTEASE HOMOLOG 1 PRECURSOR (EC 3.4.21.-).//1.3e-69:200:55//ARABIDOPSIS THALIANA (MOUSE-EAR CRESS).//O64948
F-NT2RP3003302//LINE-1 REVERSE TRANSCRIPTASE HOMOLOG.//6.4e-69:102:66//HOMO SAPIENS (HUMAN).//P08547
F-NT2RP3003311//MYOSIN II HEAVY CHAIN, NON MUSCLE.//0.18:225:26//ACANTHAMOEBA CASTELLANII (AMOEBA).//P05659
F-NT2RP3003313//SALIVARY PROLINE-RICH PROTEIN PO (ALLELE K) [CONTAINS: PEPTIDE P-D] (FRAGMENT).//0.0014:142:33//HOMO SAPIENS (HUMAN).//P10162
F-NT2RP3003327//52 KD RO PROTEIN (SJOGREN SYNDROME TYPE A ANTIGEN (SS-A)) (RO(SS-A)) (RO52).//8.8e-18:94:43//MUS MUSCULUS (MOUSE).//Q62191
F-NT2RP3003330//HYPOTHETICAL PROTEIN KIAA0176 (FRAGMENT).//1.3e-20:123:44//HOMO SAPIENS (HUMAN).//Q14681
F-NT2RP3003344//HYPOTHETICAL 8.8 KD PROTEIN IN ICDC-MINE INTERGENIC REGION.//1.0:28:42//ESCHERICHIA COLI.//P75991
F-NT2RP3003346//!!!! ALU SUBFAMILY SB2 WARNING ENTRY !!!!//6.9e-26:74:78//HOMO SAPIENS (HUMAN).//P39191
F-NT2RP3003353//HYPOTHETICAL 52.4 KD PROTEIN R08D7.2 IN CHROMOSOME III.//3.7e-10:118:33//CAENORHABDITIS ELEGANS.//P30641
F-NT2RP3003377//PUTATIVE CUTICLE COLLAGEN F09G8.6.//1.5e-05:102:37//CAENORHABDITIS ELEGANS.//P34391
F-NT2RP3003384
F-NT2RP3003385//SKD3 PROTEIN.//5.1e-83:210:69//MUS MUSCULUS (MOUSE).//Q60649
F-NT2RP3003403
F-NT2RP3003409//SOX-22 PROTEIN.//0.042:173:28//HOMO SAPIENS (HUMAN).//O15370
F-NT2RP3003411//PROBABLE E3 PROTEIN.//0.17:91:31//BOVINE PAPILLOMAVIRUS TYPE 2.//P11300
F-NT2RP3003427//HOLOTRICIN 3 PRECURSOR.//0.012:36:41//HOLOTRICHIA DIOMPHALIA.//Q25055
F-NT2RP3003433
F-NT2RP3003464//HYPOTHETICAL 57.5 KD PROTEIN IN VMA7-RPS25A INTERGENIC REGION.//0.0042:110:40//SACCHAROMYCES CEREVISIAE (BAKER'S YEAST).//P53214
F-NT2RP3003490
F-NT2RP3003491//10 KD CHAPERONIN (PROTEIN CPN10) (PROTEIN GROES) (HEAT SHOCK 10 KD PROTEIN).//0.99:49:34//LEPTOSPIRA INTERROGANS.//P35472
F-NT2RP3003500//SCY1 PROTEIN.//6.8e-14:192:26//SACCHAROMYCES CEREVISIAE (BAKER'S YEAST).//P53009
F-NT2RP3003543//COLLAGEN ALPHA 5(IV) CHAIN PRECURSOR.//0.0026:175:30//HOMO SAPIENS (HUMAN).//P29400
F-NT2RP3003552//ANNEXIN VII (SYNEXIN) (FRAGMENT).//0.19:21:47//BOS TAURUS (BOVINE).//P20072
F-NT2RP3003555//HYPOTHETICAL 32.6 KD PROTEIN IN MET30-PIG2 INTERGENIC REGION.//7.3e-27:159:43//SACCHAROMYCES CEREVISIAE (BAKER'S YEAST).//P40529
F-NT2RP3003564//RNA REPLICASE POLYPROTEIN (EC 2.7.7.48).//1.0:99:30//TURNIP YELLOW MOSAIC VIRUS.//P10358
F-NT2RP3003572//PUTATIVE CUTICLE COLLAGEN F09G8.6.//0.33:128:32//CAENORHABDITIS ELEGANS.//P34391
F-NT2RP3003576//!!!! ALU SUBFAMILY SX WARNING ENTRY !!!!//7.1e-28:58:77//HOMO SAPIENS (HUMAN).//P39195
F-NT2RP3003589//RAS-RELATED PROTEIN RAB-10.//5.4e-54:114:94//CANIS FAMILIARIS (DOG).//P24409
F-NT2RP3003621//COAGULATION FACTOR XII PRECURSOR (EC 3.4.21.38) (HAGEMAN FACTOR) (HAF).//2.0e-15:89:40//HOMO SAPIENS (HUMAN).//P00748
F-NT2RP3003625//MALE SPECIFIC SPERM PROTEIN MST84DD.//0.99:22:50//DROSOPHILA MELANOGASTER (FRUIT FLY).//Q01645
F-NT2RP3003656//HOMEOBOX PROTEIN OTX3 (ZOTX3).//0.30:111:25//BRACHYDANIO RERIO (ZEBRAFISH) (ZEBRA DANIO).//Q90267
F-NT2RP3003659//HYPOTHETICAL 49.8 KD PROTEIN IN RPL14B-GPA1 INTERGENIC REGION.//1.1e-20:127:37//SACCHAROMYCES CEREVISIAE (BAKER'S YEAST).//P38755
F-NT2RP3003665//PENAEIDIN-3C PRECURSOR (P3-C).//0.34:52:34//PENAEUS VANNAMEI (PENOEID SHRIMP) (EUROPEAN WHITE SHRIMP).//P81060
F-NT2RP3003672//T-CELL SURFACE GLYCOPROTEIN E2 PRECURSOR (E2 ANTIGEN) (CD99) (MIC2 PROTEIN) (12E7).//8.7e-15:146:42//HOMO SAPIENS (HUMAN).//P14209
F-NT2RP3003680//HYPOTHETICAL 55.1 KD PROTEIN IN FAB1-PES4 INTERGENIC REGION.//4.3e-25:159:40//SACCHAROMYCES CEREVISIAE (BAKER'S YEAST).//P43601
F-NT2RP3003686//NONHISTONE CHROMOSOMAL PROTEIN HMG-17.//0.067:63:31//GALLUS GALLUS (CHICKEN).//P02314
F-NT2RP3003701//F-SPONDIN PRECURSOR.//1.8e-13:193:27//RATTUS NORVEGICUS (RAT).//P35446
F-NT2RP3003716//SLIT PROTEIN PRECURSOR.//1.3e-12:150:34//DROSOPHILA MELANOGASTER (FRUIT FLY).//P24014
F-NT2RP3003726//INSERTION ELEMENT IS136 HYPOTHETICAL 16.9 KD PROTEIN).//0.47:109:28//AGROBACTERIUM TUMEFACIENS.//P05680
F-NT2RP3003746//HYPOTHETICAL 7.7 KD PROTEIN IN FIXX 3'REGION (ORF1).//0.57:34:38//AZORHIZOBIUM CAULINODANS.//P26486
F-NT2RP3003795//!!!! ALU SUBFAMILY SX WARNING ENTRY !!!!//4.3e-10:40:90//HOMO SAPIENS (HUMAN).//P39195
F-NT2RP3003799//MATING-TYPE PHEROMONE BBP1(3) PRECURSOR.//0.75:60:36//SCHIZOPHYLLUM COMMUNE (BRACKET FUNGUS).//P78744
F-NT2RP3003800//PROTO-ONCOGENE TYROSINE-PROTEIN KINASE SRC (EC 2.7.1.112) (P60-SRC).//4.2e-51:72:95//GALLUS GALLUS (CHICKEN).//P00523
F-NT2RP3003805//HYPOTHETICAL 32.1 KD PROTEIN IN DBP7-GCN3 INTERGENIC REGION.//0.00069:160:25//SACCHAROMYCES CEREVISIAE (BAKER'S YEAST).//P36121
F-NT2RP3003809//COLLAGEN ALPHA 1(III) CHAIN (FRAGMENTS).//0.028:135:35//GALLUS GALLUS (CHICKEN).//P12105
F-NT2RP3003819//C-HORDEIN (PCP387) (FRAGMENT).//0.0026:90:33//HORDEUM VULGARE (BARLEY).//P06472
F-NT2RP3003825//PHOSPHATIDYLCHOLINE TRANSFER PROTEIN (PC-TP).//5.6e-20:174:31//BOS TAURUS (BOVINE).//P02720
F-NT2RP3003828//ADENYLATE CYCLASE, TYPE V (EC 4.6.1.1) (ATP PYROPHOSPHATE-LYASE) (CA(2+)-INHIBITABLE ADENYLYL CYCLASE).//0.0017:111:38//CANIS FAMILIARIS (DOG).//P30803
F-NT2RP3003831//ENDONUCLEASE G PRECURSOR (EC 3.1.30.-) (ENDO G).//1.1e-37:187:42//MUS MUSCULUS (MOUSE).//O08600
F-NT2RP3003833//HYPOTHETICAL 6.4 KD PROTEIN IN INTE-PIN INTERGENIC REGION.//1.0:38:39//ESCHERICHIA COLI.//P75979
F-NT2RP3003842
F-NT2RP3003846//RETINAL DEGENERATION B PROTEIN (PROBABLE CALCIUM TRANSPORTER RDGB).//0.61:54:35//DROSOPHILA MELANOGASTER (FRUIT FLY).//P43125
F-NT2RP3003870//MALE SPECIFIC SPERM PROTEIN MST84DB.//0.83:51:37//DROSOPHILA MELANOGASTER (FRUIT FLY).//Q01643
F-NT2RP3003876//PROTEIN TRANSPORT PROTEIN SEC2.//0.0017:151:27//SACCHAROMYCES CEREVISIAE (BAKER'S YEAST).//P17065
F-NT2RP3003914//UDP-GLUCOSE:GLYCOPROTEIN GLUCOSYLTRANSFERASE PRECURSOR (EC 2.4.1.-) (DUGT).//3.3e-23:76:64//DROSOPHILA MELANOGASTER (FRUIT FLY).//Q09332
F-NT2RP3003918//VESICLE-ASSOCIATED MEMBRANE PROTEIN/SYNAPTOBREVIN BINDING PROTEIN (VAP-33).//5.5e-45:127:69//APLYSIA CALIFORNICA (CALIFORNIA SEA HARE).//Q16943
F-NT2RP3003932
F-NT2RP3003989//PREPROTEIN TRANSLOCASE SECE SUBUNIT.//0.96:46:32//THERMOTOGA MARITIMA.//P35874
F-NT2RP3003992//NUCLEAR LOCALIZATION SEQUENCE BINDING PROTEIN (P67).//0.0011:170:26//SACCHAROMYCES CEREVISIAE (BAKER'S YEAST).//P27476
F-NT2RP3004013//DOUBLE-STRANDED RNA-SPECIFIC EDITASE 1 (EC 3.5.-.-) (DSRNA ADENOSINE DEAMINASE) (RNA EDITING ENZYME 1).//3.6e-21:134:45//RATTUS NORVEGICUS (RAT).//P51400
F-NT2RP3004016//HYPOTHETICAL 24.1 KD PROTEIN IN LEF4-P33 INTERGENIC REGION.//0.00021:64:40//AUTOGRAPHA CALIFORNICA NUCLEAR POLYHEDROSIS VIRUS (ACMNPV).//P41479
F-NT2RP3004041//SPERM PROTAMINE P1.//0.0028:43:46//ORNITHORHYNCHUS ANATINUS (DUCKBILL PLATYPUS).//P35307
F-NT2RP3004051//MICROBIAL COLLAGENASE PRECURSOR (EC 3.4.24.3) (120 KD COLLAGENASE).//0.0079:194:24//CLOSTRIDIUM PERFRINGENS.//P43153
F-NT2RP3004070//!!!! ALU SUBFAMILY J WARNING ENTRY !!!!//3.4e-11:51:72//HOMO SAPIENS (HUMAN).//P39188
F-NT2RP3004078//DNA BINDING PROTEIN RFX2.//2.7e-114:243:87//MUS MUSCULUS (MOUSE).//P48379
F-NT2RP3004093//HYPOTHETICAL 32.3 KD PROTEIN IN RHSE-NARV INTERGENIC REGION (ORFB).//8.0e-13:111:41//ESCHERICHIA COLI.//P37757
F-NT2RP3004095//!!!! ALU SUBFAMILY J WARNING ENTRY !!!!//1.5e-17:72:65//HOMO SAPIENS (HUMAN).//P39188
F-NT2RP3004110//!!!! ALU SUBFAMILY SX WARNING ENTRY !!!!//2.6e-10:51:72//HOMO SAPIENS (HUMAN).//P39195
F-NT2RP3004125//ZINC FINGER PROTEIN 75.//1.1e-28:118:47//HOMO SAPIENS (HUMAN).//P51815
F-NT2RP3004145//AEROLYSIN REGULATORY PROTEIN.//0.012:45:33//AEROMONAS SOBRIA.//P09165
F-NT2RP3004148//METALLOTHIONEIN-I (MT-1).//0.055:18:50//COLUMBA LIVIA (DOMESTIC PIGEON).//P15786
F-NT2RP3004155//UBIQUINONE BIOSYNTHESIS PROTEIN COQ7 HOMOLOG.//1.7e-82:178:89//RATTUS NORVEGICUS (RAT).//Q63619
F-NT2RP3004189//VEGETATIBLE INCOMPATIBILITY PROTEIN HET-E-1.//1.7e-11:215:24//PODOSPORA ANSERINA.//Q00808
F-NT2RP3004206//CROOKED NECK PROTEIN.//3.8e-101:241:73//DROSOPHILA MELANOGASTER (FRUIT FLY).//P17886
F-NT2RP3004207//CUTICLE COLLAGEN 12 PRECURSOR.//0.13:130:33//CAENORHABDITIS ELEGANS.//P20630
F-NT2RP3004209//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN HOMOLOG).//6.5e-16:207:29//HOMO SAPIENS (HUMAN).//Q13107
F-NT2RP3004215//PROTEIN TRANSPORT PROTEIN SEC61 GAMMA SUBUNIT.//1.0:69:31//SACCHAROMYCES CEREVISIAE (BAKER'S YEAST).//P35179
F-NT2RP3004242//HYPOTHETICAL 30.2 KD PROTEIN ZK632.12 IN CHROMOSOME III.//1.1e-64:191:63//CAENORHABDITIS ELEGANS.//P34657
F-NT2RP3004246//RING3 PROTEIN (KIAA9001).//0.060:101:28//HOMO SAPIENS (HUMAN).//P25440
F-NT2RP3004253//COLLAGEN ALPHA 1(I) CHAIN (FRAGMENTS).//1.1e-07:184:35//BOS TAURUS (BOVINE).//P02453
F-NT2RP3004258//SUPPRESSOR PROTEIN SRP40.//4.9e-08:98:39//SACCHAROMYCES CEREVISIAE (BAKER'S YEAST).//P32583
F-NT2RP3004262//DNAJ PROTEIN HOMOLOG 1 (HDJ-1) (HEAT SHOCK PROTEIN 40) (HSP40).//1.6e-63:210:61//HOMO SAPIENS (HUMAN).//P25685
F-NT2RP3004282//HYPOTHETICAL PROTEIN F44G4.1 IN CHROMOSOME II (FRAGMENT).//1.6e-29:177:38//CAENORHABDITIS ELEGANS.//P54073
F-NT2RP3004332//DNA-DIRECTED RNA POLYMERASE II LARGEST SUBUNIT (EC 2.7.7.6) (RPB1) (FRAGMENT).//0.030:118:36//CRICETULUS GRISEUS (CHINESE HAMSTER).//P11414
F-NT2RP3004334
F-NT2RP3004341//ALPHA-INTERNEXIN (ALPHA-INX).//0.91:110:26//MUS MUSCULUS (MOUSE).//P46660
F-NT2RP3004348//HYPOTHETICAL 105.3 KD PROTEIN C01G6.5 IN CHROMOSOME III.//0.60:198:24//CAENORHABDITIS ELEGANS.//P46012
F-NT2RP3004349//!!!! ALU SUBFAMILY SP WARNING ENTRY !!!!//1.0e-37:60:76//HOMO SAPIENS (HUMAN).//P39193
F-NT2RP3004378//HYPOTHETICAL 18.8 KD PROTEIN IN GNTR-GGT INTERGENIC REGION (O162).//0.0026:76:28//ESCHERICHIA COLI.//P46854
F-NT2RP3004399//LEUCINE-RICH PRIMARY RESPONSE PROTEIN 1 (FOLLICLE-STIMULATING HORMONE PRIMARY RESPONSE PROTEIN).//4.4e-109:212:96//HOMO SAPIENS (HUMAN).//Q92674
F-NT2RP3004424//JTV-1 PROTEIN.//4.5e-18:60:70//HOMO SAPIENS (HUMAN).//Q13155 F-NT2RP3004428//METALLOTHIONEIN-A (MTA).//0.0010:36:47//STRONGYLOCENTROTUS PURPURATUS (PURPLE SEA URCHIN).//P04734
F-NT2RP3004451//MYOSIN IC HEAVY CHAIN.//0.00072:113:34//ACANTHAMOEBA CASTELLANII (AMOEBA).//P10569
F-NT2RP3004454//VERPROLIN.//3.3e-07:156:29//SACCHAROMYCES CEREVISIAE (BAKER'S YEAST).//P37370
F-NT2RP3004466//HYPOTHETICAL PROTEIN F-215.//0.0013:125:32//HUMAN ADENOVIRUS TYPE 2.//P03291
F-NT2RP3004470//HYPOTHETICAL 15.4 KD PROTEIN C16C10.11 IN CHROMOSOME III.//1.0:33:51//CAENORHABDITIS ELEGANS.//Q09254
F-NT2RP3004472//GERM CELL-LESS PROTEIN.//7.3e-33:170:40//DROSOPHILA MELANOGASTER (FRUIT FLY).//Q01820
F-NT2RP3004475//RHO-GAP HEMATOPOIETIC PROTEIN C1 (P115) (KIAA0131).//8.4e-54:214:46//HOMO SAPIENS (HUMAN).//P98171
F-NT2RP3004480//VACUOLAR PROTEIN SORTING-ASSOCIATED PROTEIN VPS35.//3.9e-47:199:49//SACCHAROMYCES CEREVISIAE (BAKER'S YEAST).//P34110
F-NT2RP3004490//SKIN SECRETORY PROTEIN XP2 PRECURSOR (APEG PROTEIN).//0.0013:121:33//XENOPUS LAEVIS (AFRICAN CLAWED FROG).//P17437
F-NT2RP3004498//HYPOTHETICAL 43.5 KD PROTEIN IN COTD-KDUD INTERGENIC REGION PRECURSOR.//0.066:87:35//BACILLUS SUBTILIS.//P50840
F-NT2RP3004503//!!!! ALU SUBFAMILY SQ WARNING ENTRY !!!!//2.0e-34:102:69//HOMO SAPIENS (HUMAN).//P39194
F-NT2RP3004504//SUPPRESSOR PROTEIN SRP40.//0.64:93:34//SACCHAROMYCES CEREVISIAE (BAKER'S YEAST).//P32583
F-NT2RP3004507//MOB1 PROTEIN (MPS1 BINDER 1).//2.2e-16:90:42//SACCHAROMYCES CEREVISIAE (BAKER'S YEAST).//P40484
F-NT2RP3004527
F-NT2RP3004534//S-PHASE ENTRY CYCLIN 6.//0.38:148:22//SACCHAROMYCES CEREVISIAE (BAKER'S YEAST).//P32943
F-NT2RP3004539//INSULIN-LIKE GROWTH FACTOR BINDING PROTEIN 1 PRECURSOR (IGFBP-1) (IBP-1) (IGF-BINDING PROTEIN 1).//0.38:89:38//RATTUS NORVEGICUS (RAT).//P21743
F-NT2RP3004544//CYTADHERENCE HIGH MOLECULAR WEIGHT PROTEIN 2 (CYTADHERENCE ACCESSORY PROTEIN 2).//0.0024:200:24//MYCOPLASMA PNEUMONIAE.//P75471
F-NT2RP3004566//GASTRULA ZINC FINGER PROTEIN XLCGF17.1 (FRAGMENT).//4.6e-25:126:43//XENOPUS LAEVIS (AFRICAN CLAWED FROG).//P18713
F-NT2RP3004569//ANKYRIN.//8.3e-07:150:28//MUS MUSCULUS (MOUSE).//Q02357
F-NT2RP3004572//TRANSCRIPTION INITIATION FACTOR TFIID 150 KD SUBUNIT (TAFII-150) (TAFII150).//1.6e-70:247:54//DROSOPHILA MELANOGASTER (FRUIT FLY).//Q24325
F-NT2RP3004578//CENTROMERIC PROTEIN E (CENP-E PROTEIN).//1.5e-10:210:26//HOMO SAPIENS (HUMAN).//Q02224
F-NT2RP3004594//P54 PROTEIN PRECURSOR.//0.0044:230:24//ENTEROCOCCUS FAECIUM (STREPTOCOCCUS FAECIUM).//P13692
F-NT2RP3004617//DOWN REGULATORY PROTEIN OF INTERLEUKIN 2 RECEPTOR.//1.5e-14:113:34//MUS MUSCULUS (MOUSE).//P15533
F-NT2RP3004618//HYPOTHETICAL 115.4 KD PROTEIN ZK757.3 IN CHROMOSOME III.//4.5e-08:149:30//CAENORHABDITIS ELEGANS.//P34681
F-NT2RP3004669//ETHANOLAMINE KINASE (EC 2.7.1.82) (EASILY SHOCKED PROTEIN).//1.0e-24:75:48//DROSOPHILA MELANOGASTER (FRUIT FLY).//P54352
F-NT2RP3004670//CUTICLE COLLAGEN 21/0.00090:159:29//CAENORHABDITIS ELEGANS.//P17656
F-NT2RP4000008//CHLORINE CHANNEL PROTEIN P64.//4.0e-79:243:62//BOS TAURUS (BOVINE).//P35526
F-NT2RP4000023
F-NT2RP4000035//!!!! ALU SUBFAMILY SQ WARNING ENTRY !!!!//3.6e-06:46:67//HOMO SAPIENS (HUMAN).//P39194
F-NT2RP4000049//CALDESMON (CDM).//0.41:63:34//GALLUS GALLUS (CHICKEN).//P12957
F-NT2RP4000051//DUALIN.//2.3e-23:195:37//GALLUS GALLUS (CHICKEN).//Q90830
F-NT2RP4000078//RING CANAL PROTEIN (KELCH PROTEIN).//1.2e-24:182:31//DROSOPHILA MELANOGASTER (FRUIT FLY).//Q04652
F-NT2RP4000102//XPAR7 PROTEIN.//1.0:54:33//BACILLUS LICHENIFORMIS.//Q99166
F-NT2RP4000109//SLIT PROTEIN PRECURSOR.//1.9e-60:230:46//DROSOPHILA MELANOGASTER (FRUIT FLY).//P24014
F-NT2RP4000111//CLEAVAGE AND POLYADENYLATION SPECIFICITY FACTOR, 100 KD SUBUNIT (CPSF 100 KD SUBUNIT).//1.4e-91:157:100//BOS TAURUS (BOVINE).//Q10568
F-NT2RP4000129//5E5 ANTIGEN.//0.00072:124:37//RATTUS NORVEGICUS (RAT).//Q63003
F-NT2RP4000147//ZINC FINGER PROTEIN GCS1.//1.5e-26:119:43//SACCHAROMYCES CEREVISIAE (BAKER'S YEAST).//P35197
F-NT2RP4000150
F-NT2RP4000151//HYPOTHETICAL 31.0 KD PROTEIN R107.2 IN CHROMOSOME III.//4.2e-31:180:47//CAENORHABDITIS ELEGANS .//P32740
F-NT2RP4000159//SPORE COAT PROTEIN SP96.//0.84:107:28//DICTYOSTELIUM DISCOIDEUM (SLIME MOLD).//P14328
F-NT2RP4000167//HYPOTHETICAL 98.1 KD PROTEIN IN SPX19-GCR2 INTERGENIC REGION.//2.4e-08:133:32//SACCHAROMYCES CEREVISIAE (BAKER'S YEAST).//P40164
F-NT2RP4000185//TRANS-ACTING TRANSCRIPTIONAL PROTEIN ICP0 (VMW118 PROTEIN).//5.4e-05:143:32//HERBES SIMPLEX VIRUS (TYPE 2 / STRAIN HG52).//P28284
F-NT2RP4000210//PAIRED AMPHIPATHIC HELIX PROTEIN.//1.8e-40:258:35//SACCHAROMYCES CEREVISIAE (BAKER'S YEAST).//P22579
F-NT2RP4000212//ATRIAL GLAND-SPECIFIC ANTIGEN PRECURSOR (AGSA).//1.4e-20:104:40//APLYSIA CALIFORNICA (CALIFORNIA SEA HARE).//P15287
F-NT2RP4000214//FERREDOXIN.//1.0:19:42//MOORELLA THERMOACETICA (CLOSTRIDIUM THERMOACETICUM).//P00203
F-NT2RP4000218//!!!! ALU SUBFAMILY J WARNING ENTRY !!!!//1.7e-15:48:60//HOMO SAPIENS (HUMAN).//P39188
F-NT2RP4000243//DUALIN.//5.8e-78:192:70//GALLUS GALLUS (CHICKEN).//Q90830
F-NT2RP4000246//NPC DERIVED PROLINE RICH PROTEIN 1 (NDPP-1).//3.1e-83:207:76//MUS MUSCULUS (MOUSE).//Q03173
F-NT2RP4000259//GLUTATHIONE PEROXIDASE 2 (EC 1.11.1.9).//5.5e-29:153:43//HELIANTHUS ANNUUS (COMMON SUNFLOWER).//O23968
F-NT2RP4000263//ANNEXIN VII (SYNEXIN) (FRAGMENT).//0.98:42:40//BOS TAURUS (BOVINE).//P20072
F-NT2RP4000290//HYPOTHETICAL 116.5 KD PROTEIN C20G8.09C IN CHROMOSOME I.//3.5e-71:209:66//SCHIZOSACCHAROMYCES POMBE (FISSION YEAST).//P87115
F-NT2RP4000312//RAS SUPPRESSOR PROTEIN 1 (RSU-1) (RSP-1 PROTEIN) (RSP-1).//8.9e-22:166:37//HOMO SAPIENS (HUMAN).//Q15404
F-NT2RP4000321//VERPROLIN.//0.00018:260:28//SACCHAROMYCES CEREVISIAE (BAKER'S YEAST).//P37370
F-NT2RP4000323//ANTHOPLEURIN B (TOXIN AP-B).//0.42:15:46//ANTHOPLEURA XANTHOGRAMMICA (GIANT GREEN SEA ANEMONE).//P01531
F-NT2RP4000355//HYPOTHETICAL 90.9 KD PROTEIN IN GCN20-CMK1 INTERGENIC REGION.//0.75:125:29//SACCHAROMYCES CEREVISIAE (BAKER'S YEAST).//P43596
F-NT2RP4000360//ACIDIC PROLINE-RICH PROTEIN PRECURSOR (CLONE PRP25) (FRAGMENT).//0.27:92:33//RATTUS NORVEGICUS (RAT).//P10164
F-NT2RP4000367//HYPOTHETICAL 7.3 KD PROTEIN IN 100 KD PROTEIN REGION.//0.99:52:32//HUMAN ADENOVIRUS TYPE 41.//P23691
F-NT2RP4000370//MITOCHONDRIAL PEPTIDE CHAIN RELEASE FACTOR 1 PRECURSOR (MRF-1).//4.1e-40:163:52//HOMO SAPIENS (HUMAN).//O75570
F-NT2RP4000376//PHOSPHOLIPASE A-2-ACTIVATING PROTEIN (PLAP).//4.2e-59:125:80//RATTUS NORVEGICUS (RAT).//P54319
F-NT2RP4000381//NEUROFILAMENT TRIPLET H PROTEIN (200 KD NEUROFILAMENT PROTEIN) (NF-H).//0.00058:194:30//MUS MUSCULUS (MOUSE).//P19246
F-NT2RP4000398//ZINC FINGER PROTEIN 184 (FRAGMENT).//1.2e-45:153:39//HOMO SAPIENS (HUMAN).//Q99676
F-NT2RP4000415//HYPOTHETICAL 118.4 KD PROTEIN IN BAT2-DAL5 INTERGENIC REGION PRECURSOR.//0.00066:201:27//SACCHAROMYCES CEREVISIAE (BAKER'S YEAST).//P47179
F-NT2RP4000417//PROCESSING ALPHA-1,2-MANNOSIDASE (EC 3.2.1.-) (ALPHA-1,2-MANNOSIDASE 1B).//1.8e-25:196:40//MUS MUSCULUS (MOUSE).//P39098
F-NT2RP4000424//!!!! ALU SUBFAMILY SX WARNING ENTRY !!!!//1.0e-15:72:61//HOMO SAPIENS (HUMAN).//P39195
F-NT2RP4000448//!!!! ALU SUBFAMILY SC WARNING ENTRY !!!!//7.0e-23:63:82//HOMO SAPIENS (HUMAN).//P39192
F-NT2RP4000449//REGULATORY PROTEIN SIR2 (SILENT INFORMATION REGULATOR 2).//1.3e-41:102:45//KLUYVEROMYCES LACTIS (YEAST).//P33294
F-NT2RP4000455//HOMEOBOX PROTEIN SAX-1 (CHOX-3) (FRAGMENT).//0.00014:92:30//GALLUS GALLUS (CHICKEN).//P19601
F-NT2RP4000457//UBIQUITIN CARBOXYL-TERMINAL HYDROLASE 7 (EC 3.1.2.15) (UBIQUITIN THIOLESTERASE 7) (UBIQUITIN-SPECIFIC PROCESSING PROTEASE 7) (DEUBIQUITINATING ENZYME 7) (HERPESVIRUS ASSOCIATED UBIQUITIN-SPECIFIC PROTEASE).//1.0e-29:218:38//HOMO SAPIENS (HUMAN).//Q93009
F-NT2RP4000480//TRANSCRIPTIONAL REGULATORY PROTEIN ALGP (ALGINATE REGULATORY PROTEIN ALGR3).//0.049:117:29//PSEUDOMONAS AERUGINOSA.//P15276
F-NT2RP4000481//HYPOTHETICAL HELICASE C28H8.3 IN CHROMOSOME III.//2.3e-05:152:23//CAENORHABDITIS ELEGANS.//Q09475
F-NT2RP4000498//MOB1 PROTEIN (MPS1 BINDER 1).//2.3e-48:172:52//SACCHAROMYCES CEREVISIAE (BAKER'S YEAST).//P40484
F-NT2RP4000500//HYPOTHETICAL 83.6 KD PROTEIN R05D3.2 IN CHROMOSOME III.//1.3e-23:165:35//CAENORHABDITIS ELEGANS.//P34535
F-NT2RP4000515//PHOSPHODIESTERASE I (EC 3.1.4.1) (5'-EXONUCLEASE) (5'-NUCLEOTIDE PHOSPHODIESTERASE) (FRAGMENT).//1.0:48:37//BOS TAURUS (BOVINE).//P15396
F-NT2RP4000517//METALLOTHIONEIN-LIKE PROTEIN TYPE 2.//1.0:41:36//VICIA FABA (BROAD BEAN).//Q41657
F-NT2RP4000518//ATP-DEPENDENT RNA HELICASE ROK1.//1.1e-11:93:36//SACCHAROMYCES CEREVISIAE (BAKER'S YEAST).//P45818
F-NT2RP4000519//COLLAGEN ALPHA 1(XII) CHAIN (FRAGMENTS).//0.68:55:40//BOS TAURUS (BOVINE).//P25508
F-NT2RP4000524//IGA FC RECEPTOR PRECURSOR (BETA ANTIGEN) (B ANTIGEN).//0.37:187:24//STREPTOCOCCUS AGALACTIAE.//P27951
F-NT2RP4000528//NPL4 PROTEIN.//2.1e-45:305:36//SACCHAROMYCES CEREVISIAE (BAKER'S YEAST).//P33755
F-NT2RP4000541//HOMEOBOX PROTEIN CHOX-1 (FRAGMENT).//0.23:28:50//GALLUS GALLUS (CHICKEN).//P13544
F-NT2RP4000556//HYPOTHETICAL 34.1 KD PROTEIN C40H1.4 IN CHROMOSOME III.//4.3e-14:174:34//CAENORHABDITIS ELEGANS.//Q03574
F-NT2RP4000560//HYPOTHETICAL 68.7 KD PROTEIN ZK757.1 IN CHROMOSOME III.//2.1e-19:155:36//CAENORHABDITIS ELEGANS.//P34679
F-NT2RP4000588//HYPOTHETICAL PROTEIN E-115.//0.014:64:35//HUMAN ADENOVIRUS TYPE 2.//P03290
F-NT2RP4000614//SPLICING FACTOR, ARGININE/SERINE-RICH 2 (SPLICING FACTOR SC35) (SC-35) (SPLICING COMPONENT, 35 KD) (PR264 PROTEIN).//2.7e-27:188:44//GALLUS GALLUS (CHICKEN).//P30352
F-NT2RP4000638//EARLY NODULIN 55-1 PRECURSOR (N-55-1) (FRAGMENT).//0.55:40:40//GLYCINE MAX (SOYBEAN).//Q05544
F-NT2RP4000648//!!!! ALU SUBFAMILY J WARNING ENTRY !!!!//2,4e-06:31:74//HOMO SAPIENS (HUMAN).//P39188
F-NT2RP46000657//HYPOTHETICAL PROTEIN MJ1065.//2.5e-40:237:40//METHANOCOCCUS JANNASCHII.//Q58465
F-NT2RP4000704
F-NT2RP4000713//HYPOTHETICAL 35.5 KD PROTEIN IN TRANSPOSON TN4556.//4.0e-07:134:40//STREPTOMYCES FRADIAE.//P20186
F-NT2RP4000724//RETROVIRUS-RELATED POL POLYPROTEIN [CONTAINS: REVERSE TRANSCRIPTASE (EC 2.7.7.49); ENDONUCLEASE].//1.1e-62:109:88//HOMO SAPIENS (HUMAN).//P10266
F-NT2RP4000728//A-AGGLUTININ ATTACHMENT SUBUNIT PRECURSOR.//0.0033:190:25//SACCHAROMYCES CEREVISIAE (BAKER'S YEAST).//P32323
F-NT2RP4000737//PTB-ASSOCIATED SPLICING FACTOR (PSF).//1.0e-05:114:34//HOMO SAPIENS (HUMAN).//P23246
F-NT2RP4000739//ATP SYNTHASE PROTEIN 8 (EC 3.6.1.34) (A6L).//1.0:20:50//ANAS PLATYRHYNCHOS (DOMESTIC DUCK).//P50655
F-NT2RP4000781//HYPOTHETICAL 27.7 KD PROTEIN IN CPT1-SPC98 INTERGENIC REGION.//0.0013:67:31//SACCHAROMYCES CEREVISIAE (BAKER'S YEAST).//P53915
F-NT2RP4000787//POLLEN SPECIFIC PROTEIN SF3.//1.3e-13:79:39//HELIANTHUS ANNUUS (COMMON SUNFLOWER).//P29675
F-NT2RP4000817//SUPPRESSOR PROTEIN SRP40.//1.3e-05:255:21//SACCHAROMYCES CEREVISIAE (BAKER'S YEAST).//P32583
F-NT2RP4000833
F-NT2RP4000837//MALE SPECIFIC SPERM PROTEIN MSTS4DB.//0.18:38:44//DROSOPHILA MELANOGASTER (FRUIT FLY).//Q01643
F-NT2RP4000839//TRANSCRIPTION INITIATION FACTOR TFIID 90 KD SUBUNIT (TAFII-90).//0.026:38:44//SACCHAROMYCES CEREVISIAE (BAKER'S YEAST).//P38129
F-NT2RP4000855//AMINOPEPTIDASE B (EC 3.4.11.6) (ARGINYL AMINOPEPTIDASE) (ARGININE AMINOPEPTIDASE) (CYTOSOL AMINOPEPTIDASE IV) (AP-B).//2.8e-64:229:53//RATTUS NORVEGICUS (RAT).//O09175
F-NT2RP4000865//ZINC FINGER PROTEIN ZFP-36 (FRAGMENT).//3.6e-84:174:54//HOMO SAPIENS (HUMAN).//P16415
F-NT2RP4000878//MYELOID UPREGULATED PROTEIN.//8.2e-88:227:74//MUS MUSCULUS (MOUSE).//O35682
F-NT2RP4000879//UBIQUITIN-ACTIVATING ENZYME E1 (A1S9 PROTEIN).//9.1e-55:268:43//HOMO SAPIENS (HUMAN).//P22314
F-NT2RP4000907//BDNF / NT-3 GROWTH FACTORS RECEPTOR PRECURSOR (EC 2.7.1.112) (TRKB TYROSINE KINASE) (GP145-TRKB) (TRK-B).//5.4e-10:220:25//HOMO SAPIENS (HUMAN).//Q16620
F-NT2RP4000915//60S ACIDIC RIBOSOMAL PROTEIN P2 (FRAGMENT).//0.46:23:60//ARABIDOPSIS THALIANA (MOUSE-EAR CRESS).//P51407
F-NT2RP4000918//METHYL-ACCEPTING CHEMOTAXIS PROTEIN TLPB.//0.00010:148:32//BACILLUS SUBTILIS.//P39217
F-NT2RP4000925//FIBROMODULIN PRECURSOR (FM) (COLLAGEN-BINDING 59 KD PROTEIN).//3.5e-27:220:36//HOMO SAPIENS (HUMAN).//Q06828
F-NT2RP4000927//TRANS-ACTING TRANSCRIPTIONAL PROTEIN ICP0 (P135 PROTEIN) (IER 2.9/ER2.6).//0.64:75:37//BOVINE HERPESVIRUS TYPE 1 (STRAIN JURA).//P29128
F-NT2RP4000928//PHOSPHATIDATE CYTIDYLYLTRANSFERASE (EC 2.7.7.41) (CDP-DIGLYCERIDE SYNTHETASE) (CDP-DIGLYCERIDE PYROPHOSPHORYLASE) (CDP-DIACYLGLYCEROL SYNTHASE) (CDS) (CTP:PHOSPHATIDATE CYTIDYLYLTRANSFERASE) (CDP-DAG SYNTHASE).//3.1e-104:263:66//HOMO SAPIENS (HUMAN).//Q92903
F-NT2RP4000929//HYPOTHETICAL 22.2 KD PROTEIN IN NSR1-TIF4631 INTERGENIC REGION.//0.93:107:28//SACCHAROMYCES CEREVISIAE (BAKER'S YEAST).//P53288
F-NT2RP4000955//PUTATIVE CUTICLE COLLAGEN F09G8.6.//2.0e-05:102:37//CAENORHABDITIS ELEGANS.//P34391
F-NT2RP4000973//HYPOTHETICAL 48.6 KD PROTEIN IN BET1-PAN1 INTERGENIC REGION.//2.3e-17:78:56//SACCHAROMYCES CEREVISIAE (BAKER'S YEAST).//P40564
F-NT2RP4000975//SALIVARY PROLINE-RICH PROTEIN PO (ALLELE K) [CONTAINS: PEPTIDE P-D] (FRAGMENT).//0.0041:142:33//HOMO SAPIENS (HUMAN).//P10162
F-NT2RP4000979//HYPOTHETICAL 14.5 KD PROTEIN.//0.77:106:33//VACCINIA VIRUS (STRAIN COPENHAGEN).//P20517
F-NT2RP4000984//HYPOTHETICAL 124.8 KD PROTEIN C29E4.4 IN CHROMOSOME III.//0.90:94:25//CAENORHABDITIS ELEGANS.//P34343
F-NT2RP4000989//ANTHOPLEURIN B (TOXIN AP-B).//0.76:41:41//ANTHOPLEURA XANTHOGRAMMICA (GIANT GREEN SEA ANEMONE).//P01531
F-NT2RP4000996//PROTEIN Q300.//0.00024:41:53//MUS MUSCULUS (MOUSE).//Q02722
F-NT2RP4000997//DNA-DIRECTED RNA POLYMERASE I135 KD POLYPEPTIDE (EC 2.7.7.6) (RNA POLYMERASE I SUBUNIT 2) (RPA135) (RNA POLYMERASE I 127 KD SUBUNIT).//8.7e-115:261:82//RATTUS NORVEGICUS (RAT).//O54888
F-NT2RP4001004//EC PROTEIN HOMOLOG 2 (FRAGMENT).//0.50:61:34//ARABIDOPSIS THALIANA (MOUSE-EAR CRESS).//Q42377
F-NT2RP4001006//HYPOTHETICAL 43.5 KD PROTEIN IN COTD-KDUD INTERGENIC REGION PRECURSOR.//0.010:152:29//BACILLUS SUBTILIS.//P50840
F-NT2RP4001010//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//9.9e-05:247:25//SACCHAROMYCES CEREVISIAE (BAKER'S YEAST).//P08640
F-NT2RP4001029//PROTEIN GRAINY-HEAD (DNA-BINDING PROTEIN ELF-1) (ELEMENT I-BINDING ACTIVITY) (TRANSCRIPTION FACTOR NTF-1).//1.1e-14:175:31//DROSOPHILA MELANOGASTER (FRUIT FLY).//P13002
F-NT2RP4001041//PROBABLE LEUCYL-TRNA SYNTHETASE (EC 6.1.1.4) (LEUCINE--TRNA LIGASE) (LEURS).//1.5e-74:272:55//CAENORHABDITIS ELEGANS.//Q09996
F-NT2RP4001057//HYPOTHETICAL 62.2 KD PROTEIN ZK652.6 IN CHROMOSOME III.//0.0064:76:38//CAENORHABDITIS ELEGANS.//P34664
F-NT2RP4001064//DUALIN.//2.5e-24:199:38//GALLUS GALLUS (CHICKEN).//Q90830
F-NT2RP4001078//TRANSCRIPTION INITIATION FACTOR TFIID 135 KD SUBUNIT (TAFII-135) (TAFII135) (TAFII-130) (TAFII130).//0.11:139:38//HOMO SAPIENS (HUMAN).//O00268
F-NT2RP4001079//CALCIUM-TRANSPORTING ATPASE 1 (EC 3.6.1.38) (GOLGI CA2+-ATPASE).//1.5e-22:242:31//SACCHAROMYCES CEREVISIAE (BAKER'S YEAST).//P13586
F-NT2RP4001080//POLYPYRIMIDINE TRACT-BINDING PROTEIN (PTB) (HETEROGENEOUS NUCLEAR RIBONUCLEOPROTEIN I) (HNRNP I).//1.7e-82:178:69//SUS SCROFA (PIG).//Q29099
F-NT2RP4001086//LEUCINE-RICH ACIDIC NUCLEAR PROTEIN.//0.00039:141:26//RATTUS NORVEGICUS (RAT).//P49911
F-NT2RP4001095//DOUBLE-STRANDED RNA-SPECIFIC EDITASE 1 (EC 3.5.-.-) (DSRNA ADENOSINE DEAMINASE) (RNA EDITING ENZYME 1).//9.9e-07:79:43//HOMO SAPIENS (HUMAN).//P78563
F-NT2RP4001100//HYPOTHETICAL 74.0 KD PROTEIN IN CAJ1-HOM3 INTERGENIC REGION.//4.4e-16:207:35//SACCHAROMYCES CEREVISIAE (BAKER'S YEAST).//P40032
F-NT2RP4001117//PROTEIN TRANSPORT PROTEIN SEC61 ALPHA SUBUNIT.//8.1e-115:224:99//RATTUS NORVEGICUS (RAT).//P38378
F-NT2RP4001122//TIPD PROTEIN://7.5e-11:129:31//DICTYOSTELIUM DISCOIDEUM (SLIME MOLD).//O15736
F-NT2RP4001126//TRICHOHYALIN.//1.4e-19:257:28//OVIS ARIES (SHEEP).//P22793
F-NT2RP4001138//PUTATIVE F420-DEPENDENT NADP REDUCTASE (EC 1.-.-.-).//0.00010:204:25//METHANOCOCCUS JANNASCHII.//Q58896
F-NT2RP4001143//HYPOTHETICAL 52.9 KD PROTEIN IN SAP155-YMR31 INTERGENIC REGION.//4.5e-34:168:44//SACCHAROMYCES CEREVISIAE (BAKER'S YEAST).//P43616
F-NT2RP4001148//SOF1 PROTEIN.//2.4e-41:158:41//SACCHAROMYCES CEREVISIAE (BAKER'S YEAST).//P33750
F-NT2RP4001149//SULFATED SURFACE GLYCOPROTEIN 185 (SSG 185).//1.3e-08:106:41//VOLVOX CARTERI.//P21997
F-NT2RP4001150//NG-CAM RELATED CELL ADHESION MOLECULE PRECURSOR (NR-CAM) (BRAVO).//3.6e-24:194:32//GALLUS GALLUS (CHICKEN).//P35331
F-NT2RP4001159//MEROZOITE SURFACE ANTIGEN 2 PRECURSOR (MSA-2).//0.0056:117:25//PLASMODIUM FALCIPARUM (ISOLATE K1 / THAILAND).//Q03643
F-NT2RP4001174//NON-GREEN PLASTID TRIOSE PHOSPHATE TRANSLOCATOR PRECURSOR (CTPT).//5.9e-24:184:34//BRASSICA OLERACEA (CAULIFLOWER).//P52178
F-NT2RP4001206//MEROZOITE SURFACE ANTIGEN 2 PRECURSOR (MSA-2).//0.0029:117:26//PLASMODIUM FALCIPARUM (ISOLATE K1 / THAILAND).//Q03643
F-NT2RP4001207//CHROMOSOME SEGREGATION PROTEIN CSE1.//1.0e-07:144:28//SACCHAROMYCES CEREVISIAE (BAKER'S YEAST).//P33307
F-NT2RP4001210//DERMORPHIN 1 PRECURSOR [CONTAINS: DELTORPHIN (DERMENKEPHALIN); DERMORPHIN].//0.019:130:30//PHYLLOMEDUSA SAUVAGEI (SAUVAGE'S LEAF FROG).//P05422
F-NT2RP4001213//ZINC FINGER PROTEIN 177.//3.2e-28:176:39//HOMO SAPIENS (HUMAN).//Q13360
F-NT2RP4001219//DISULFIDE ISOMERASE MPD1 PRECURSOR (EC 5.3.4.1).//2.4e-13:108:37//SACCHAROMYCES CEREVISIAE (BAKER'S YEAST).//Q12404
F-NT2RP4001228//RING CANAL PROTEIN (KELCH PROTEIN).//2.7e-56:242:40//DROSOPHILA MELANOGASTER (FRUIT FLY).//Q04652
F-NT2RP4001235//REGULATORY PROTEIN E2.//0.0080:100:38//HUMAN PAPILLOMAVIRUS TYPE 25.//P36787
F-NT2RP4001256//CUTICLE COLLAGEN 1.//0.014:104:31//CAENORHABDITIS ELEGANS.//P08124
F-NT2RP4001260//BACTERIOCIN MICROCIN B17 PRECURSOR (MCB17).//0.00077:16:68//ESCHERICHIA COLI.//P05834
F-NT2RP4001274//HISTONE H1.M6.1.//0.98:65:35//TRYPANOSOMA CRUZI.//P40273
F-NT2RP4001276//ELAV PROTEIN.//0.00054:134:33//DROSOPHILA VIRILIS (FRUIT FLY).//P23241
F-NT2RP4001313//EXTENSIN PRECURSOR (CELL WALL HYDROXYPROLINE-RICH GLYCOPROTEIN).//0.014:71:35//NICOTIANA TABACUM (COMMON TOBACCO).//P13983
F-NT2RP4001315//VACUOLAR PROTEIN SORTING-ASSOCIATED PROTEIN VPS9.//2.3e-12:190:27//SACCHAROMYCES CEREVISIAE (BAKER'S YEAST).//P54787
F-NT2RP4001336//VEGETATIBLE INCOMPATIBILITY PROTEIN HET-E-1.//0.0037:108:31//PODOSPORA ANSERINA.//Q00808
F-NT2RP4001339//HYPOTHETICAL PROTEIN MJ0810.//1.2e-09:150:34//METHANOCOCCUS JANNASCHII.//Q58220
F-NT2RP4001343//HYPOTHETICAL 85.2 KD PROTEIN F52C9.3 IN CHROMOSOME III.//1.4e-18:244:27//CAENORHABDITIS ELEGANS.//Q10123
F-NT2RP4001345//PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).//4.0e-49:212:50//GALLUS GALLUS (CHICKEN).//P53760
F-NT2RP4001351//INTRACELLULAR PROTEIN TRANSPORT PROTEIN USO1.//5.7e-11:229:26//SACCHAROMYCES CEREVISIAE (BAKER'S YEAST).//P25386
F-NT2RP4001353//RAS SUPPRESSOR PROTEIN 1 (RSU-1) (RSP-1 PROTEIN) (RSP-1).//0.00088:84:28//HOMO SAPIENS (HUMAN).//Q15404
F-NT2RP4001372//IRREGULAR CHIASM C-ROUGHEST PROTEIN PRECURSOR (IRREC PROTEIN).//1.0e-22:222:30//DROSOPHILA MELANOGASTER (FRUIT FLY).//Q08180
F-NT2RP4001373//OV-17 ANTIGEN PRECURSOR (IMMUNODOMINANT HYPODERMAL ANTIGEN).//0.51:92:26//ONCHOCERCA VOLVULUS.//P36991
F-NT2RP4001375//NON-RECEPTOR TYROSINE KINASE SPORE LYSIS A (EC 2.7.1.112) (TYROSINE- PROTEIN KINASE 1).//3.5e-13:146:35//DICTYOSTELIUM DISCOIDEUM (SLIME MOLD).//P18160
F-NT2RP4001379//HYPOTHETICAL 64.2 KD PROTEIN IN SLT2-PUT2 INTERGENIC REGION.//1.2e-14:207:28//SACCHAROMYCES CEREVISIAE (BAKER'S YEAST).//P38767
F-NT2RP4001389//DNA-DIRECTED RNA POLYMERASE II LARGEST SUBUNIT (EC 2.7.7.6) (RPB1) (FRAGMENT).//0.073:112:33//CRICETULUS GRISEUS (CHINESE HAMSTER).//P11414
F-NT2RP4001407//CENTROMERIC PROTEIN E (CENP-E PROTEIN).//0.0019:233:24//HOMO SAPIENS (HUMAN).//Q02224
F-NT2RP4001414//SEPTIN 2 HOMOLOG (FRAGMENT).//6.2e-89:195:81//HOMO SAPIENS (HUMAN).//Q14141
F-NT2RP4001433//ZINC FINGER PROTEIN 43 (ZINC PROTEIN HTF6).//1.5e-85:216:56//HOMO SAPIENS (HUMAN).//P28160
F-NT2RP4001442//DNA-DIRECTED RNA POLYMERASE II LARGEST SUBUNIT (EC 2.7.7.6) (VERSION 1).//0.012:107:35//ARABIDOPSIS THALIANA (MOUSE-EAR CRESS).//P18616
F-NT2RP4001447//60S ACIDIC RIBOSOMAL PROTEIN P2 (EL12).//0.0046:69:33//ARTEMIA SALINA (BRINE SHRIMP).//P02399
F-NT2RP4001474//CBP3 PROTEIN PRECURSOR.//0.0011:111:29//SACCHAROMYCES CEREVISIAE (BAKER'S YEAST).//P21560
F-NT2RP4001483//2-OXOGLUTARATE DEHYDROGENASE E1 COMPONENT PRECURSOR (EC 1.2.4.2) (ALPHA-KETOGLUTARATE DEHYDROGENASE).//6.2e-60:146:61//HOMO SAPIENS (HUMAN).//Q02218
F-NT2RP4001498//HYPOTHETICAL 72.5 KD PROTEIN C2F7.10 IN CHROMOSOME I.//2.3e-24:137:37//SCHIZOSACCHAROMYCES POMBE (FISSION YEAST).//Q09701
F-NT2RP4001502//HYPOTHETICAL 24.7 KD PROTEIN IN POM152-REC114 INTERGENIC REGION.//6.0e-22:148:34//SACCHAROMYCES CEREVISIAE (BAKER'S YEAST).//P40206
F-NT2RP4001507//CUTICLE COLLAGEN 40.//0.00029:166:31//CAENORHABDITIS ELEGANS.//P34804
F-NT2RP4001524//LACTOCOCCIN A IMMUNITY PROTEIN.//0.74:96:30//LACTOCOCCUS LACTIS (SUBSP. LACTIS) (STREPTOCOCCUS LACTIS), AND LACTOCOCCUS LACTIS (SUBSP. CREMORIS) (STREPTOCOCCUS CREMORIS).//Q00561
F-NT2RP4001529//PROTEIN GRAINY-HEAD (DNA-BINDING PROTEIN ELF-1) (ELEMENT I-BINDING ACTIVITY) (TRANSCRIPTION FACTOR NTF-1).//2.8e-06:79:41//DROSOPHILA MELANOGASTER (FRUIT FLY).//P13002
F-NT2RP4001547//HYPOTHETICAL 45.0 KD PROTEIN IN NOT1/CDC39-HMR INTERGENIC REGION.//5.4e-34:88:46//SACCHAROMYCES CEREVISIAE (BAKER'S YEAST).//P25656
F-NT2RP4001551//CELL DIVISION CONTROL PROTEIN 68.//1.5e-18:243:30//SACCHAROMYCES CEREVISIAE (BAKER'S YEAST).//P32558
F-NT2RP4001555//PUTATIVE ENDONUCLEASE VIII (EC 3.2.-.-).//0.00030:158:24//MYCOBACTERIUM TUBERCULOSIS.//P96902
F-NT2RP4001567//IMPORTIN ALPHA-1 SUBUNIT (KARYOPHERIN ALPHA-1 SUBUNIT).//0.00013:147:29//XENOPUS LAEVIS (AFRICAN CLAWED FROG).//P52170
F-NT2RP4001568//HYPOTHETICAL PROTEIN KIAA0041 (FRAGMENT).//8.0e-22:119:42//HOMO SAPIENS (HUMAN).//Q15057
F-NT2RP4001571//NEUROMODULIN (AXONAL MEMBRANE PROTEIN GAP-43) (PP46) (B-50) (PROTEIN F1) (CALMODULIN-BINDING PROTEIN P-57).//0.012:167:28//BOS TAURUS (BOVINE).//P06836
F-NT2RP4001574//COATOMER GAMMA SUBUNIT (GAMMA-COAT PROTEIN) (GAMMA-COP).//6.8e-115:208:98//BOS TAURUS (BOVINE).//P53620
F-NT2RP4001575//M-RELATED PROTEIN PRECURSOR.//0.22:184:25//STREPTOCOCCUS PYOGENES.//P16946
F-NT2RP4001592//ISOLEUCYL-TRNA SYNTHETASE (EC 6.1.1.5) (ISOLEUCINE--TRNA LIGASE) (ILERS).//7.4e-45:229:39//SYNECHOCYSTIS SP. (STRAIN PCC 6803).//P73505
F-NT2RP4001610//APOLIPOPROTEIN C-III PRECURSOR (APO-CIII).//0.41:74:28//SUS SCROFA (PIG).//P27917
F-NT2RP4001614//BASIC PROLINE-RICH PEPTIDE P-E (IB-9).//1.0:29:37//HOMO SAPIENS (HUMAN).//P02811
F-NT2RP4001634//MYOSIN HEAVY CHAIN, PERINATAL SKELETAL MUSCLE (FRAGMENT).//0.16:233:23//RATTUS NORVEGICUS (RAT).//P04462
F-NT2RP4001638//DNA REPAIR/TRANSCRIPTION PROTEIN MET18/34MS19.//4.2e-21:249:30//SACCHAROMYCES CEREVISIAE (BAKER'S YEAST).//P40469
F-NT2RP4001644//MYOSIN LIGHT CHAIN KINASE (EC 2.7.1.117) (MLCK).//4.5e-18:111:44//DICTYOSTELIUM DISCOIDEUM (SLIME MOLD).//P25323
F-NT2RP4001656//HYPOTHETICAL 108.5 KD PROTEIN R06F6.2 IN CHROMOSOME II.//3.4e-13:175:32//CAENORHABDITIS ELEGANS.//Q09600
F-NT2RP4001677//HYPOTHETICAL 73.6 KD PROTEIN CY49.21.//0.065:66:43//MYCOBACTERIUM TUBERCULOSIS.//Q10690
F-NT2RP4001679//!!!! ALU SUBFAMILY SQ WARNING ENTRY !!!!//1.3e-36:103:72//HOMO SAPIENS (HUMAN).//P39194
F-NT2RP4001696//PHOTOSYSTEM II REACTION CENTRE J PROTEIN.//0.93:37:37//CHLORELLA VULGARIS.//P56338
F-NT2RP4001725//GUANINE NUCLEOTIDE-BINDING PROTEIN BETA SUBUNIT.//4.3e-11:128:32//SCHIZOSACCHAROMYCES POMBE (FISSION YEAST).//Q10282
F-NT2RP4001730//UDP-GLUCOSE:GLYCOPROTEIN GLUCOSYLTRANSFERASE PRECURSOR (EC 2.4.1.-) (DUGT).//4.1e-22:201:27//DROSOPHILA MELANOGASTER (FRUIT FLY).//Q09332
F-NT2RP4001739//HOMEOBOX PROTEIN HOX-A10 (HOX-1H) (HOX-1.8) (PL).//1.0:67:34//HOMO SAPIENS (HUMAN).//P31260
F-NT2RP4001753//ZINC FINGER PROTEIN 10 (ZINC FINGER PROTEIN KOX1) (FRAGMENT).//1.2e-19:72:62//HOMO SAPIENS (HUMAN).//P21506
F-NT2RP4001760//BREAKPOINT CLUSTER REGION PROTEIN.//1.8e-13:179:28//HOMO SAPIENS (HUMAN).//P11274
F-NT2RP4001790//ZINC FINGER PROTEIN 38 (ZFP-38) (CTFIN51) (TRANSCRIPTION FACTOR RU49).//7.9e-38:147:49//MUS MUSCULUS (MOUSE).//Q07231
F-NT2RP4001803//CUTICLE COLLAGEN 12 PRECURSOR.//0.40:48:39//CAENORHABDITIS ELEGANS.//P20630
F-NT2RP4001822//NOVEL ANTIGEN 2 (NAG-2).//2.7e-27:173:36//HOMO SAPIENS (HUMAN).//O14817
F-NT2RP4001823//PUTATIVE CUTICLE COLLAGEN F09G8.6.//3.3e-16:152:42//CAENORHABDITIS ELEGANS.//P34391
F-NT2RP4001828//HOLIN.//0.99:33:36//BACTERIOPHAGE HP1.//P51727
F-NT2RP4001838//METASTASIS-ASSOCIATED PROTEIN MTA1.//1.2e-07:95:31//HOMO SAPIENS (HUMAN).//Q13330
F-NT2RP4001841//INTESTINAL MUCIN-LIKE PROTEIN (MLP) (FRAGMENT).//0.94:141:22//RATTUS NORVEGICUS (RAT).//P98089
F-NT2RP4001849//SH3-BINDING PROTEIN 3BP-1.//5.6e-52:276:45//MUS MUSCULUS (MOUSE).//P55194
F-NT2RP4001861//HYPOTHETICAL 10.6 KD PROTEIN IN GALE-PEPT INTERGENIC REGION.//0.92:39:51//BACILLUS SUBTILIS.//P55185
F-NT2RP4001889//HYPOTHETICAL BHLF1 PROTEIN.//0.32:97:31//EPSTEIN-BARR VIRUS (STRAIN B95-8) (HUMAN HERPESVIRUS 4).//P03181
F-NT2RP4001893//2-5A-DEPENDENT RIBONUCLEASE (EC 3.1.26.-) (2-5A-DEPENDENT RNAASE) (RNASE L) (RIBONUCLEASE 4) (FRAGMENT).//3.6e-07:124:29//MUS MUSCULUS (MOUSE).//Q05921
F-NT2RP4001896//HYPOTHETICAL 89.4 KD TRP-ASP REPEATS CONTAINING PROTEIN IN PMT6-PCT1 INTERGENIC REGION.//3.9e-10:210:28//SACCHAROMYCES CEREVISIAE (BAKER'S YEAST).//P42935
F-NT2RP4001901//ACROSIN PRECURSOR (EC 3.4.21.10).//2.4e-07:53:45//ORYCTOLAGUS CUNICULUS (RABBIT).//P48038
F-NT2RP4001927//MICROTUBULE-ASSOCIATED PROTEIN YTM1.//3.1e-19:170:32//SACCHAROMYCES CEREVISIAE (BAKER'S YEAST).//Q12024
F-NT2RP4001938//ZINC FINGER PROTEIN MOK-2.//1.3e-28:72:50//MUS MUSCULUS (MOUSE).//P24399
F-NT2RP4001946//PROTEIN-L-ISOASPARTATE O-METHYLTRANSFERASE (EC 2.1.1.77) (PROTEIN- BETA-ASPARTATE METHYLTRANSFERASE) (PIMT) (PROTEIN L-ISOASPARTYL METHYLTRANSFERASE) (L-ISOASPARTYL PROTEIN CARBOXYL METHYLTRANSFERASE).//4.8e-14:183:30//TRITICUM AESTIVUM (WHEAT).//Q43209
F-NT2RP4001950//HYPOTHETICAL PROTEIN ORF-1137.//3.7e-07:115:29//MUS MUSCULUS (MOUSE).//P11260
F-NT2RP4001953
F-NT2RP4001966//WALL-ASSOCIATED PROTEIN PRECURSOR.//0.13:151:27//BACILLUS SUBTILIS.//Q07833
F-NT2RP4001975//FIBRIL-FORMING COLLAGEN ALPHA CHAIN.//0.00031:190:31//RIFTIA PACHYPTILA (TUBE WORM).//P30754
F-NT2RP4002018//RING CANAL PROTEIN (KELCH PROTEIN).//3.5e-18:185:29//DROSOPHILA MELANOGASTER (FRUIT FLY).//Q04652
F-NT2RP4002047//GTP-BINDING PROTEIN GUF1 (GTPASE GUF1).//4.0e-49:158:65//SACCHAROMYCES CEREVISIAE (BAKER'S YEAST).//P46943
F-NT2RP4002052//HYPOTHETICAL 54.3 KD PROTEIN C23D3.03C IN CHROMOSOME I.//0.0047:148:27//SCHIZOSACCHAROMYCES POMBE (FISSION YEAST).//Q09844
F-NT2RP4002058//PUTATIVE PRE-MRNA SPLICING FACTOR ATP-DEPENDENT RNA HELICASE F56D2.6.//0.057:66:30//CAENORHABDITIS ELEGANS.//Q20875
F-NT2RP4002071//VERY HYPOTHETICAL 13.2 KD PROTEIN CY251.09.//0.94:45:46//MYCOBACTERIUM TUBERCULOSIS.//Q10888
F-NT2RP4002075//TAT PROTEIN (TRANSACTIVATING REGULATORY PROTEIN).//0.44:36:38//HUMAN IMMUNODEFICIENCY VIRUS TYPE 1 (NDK ISOLATE) (HIV-1).//P18804
F-NT2RP4002078//ZINC FINGER PROTEIN 91 (ZINC FINGER PROTEIN HTF10) (HPF7).//2.6e-19:46:76//HOMO SAPIENS (HUMAN).//Q05481
F-NT2RP4002081//MHC CLASS II REGULATORY FACTOR RFX1 (RFX) (ENHANCER FACTOR C) (EF-C).//2.8e-05:196:31//HOMO SAPIENS (HUMAN).//P22670
F-NT2RP4002083//HYPOTHETICAL PROLINE-RICH PROTEIN (FRAGMENT).//0.0064:29:55//OWENIA FUSIFORMIS.//P21260
F-NT2RP4002408//PROTEIN KINASE CEK1 (EC 2.7.1.-).//1.1e-37:159:53//SCHIZOSACCHAROMYCES POMBE (FISSION YEAST).//P38938
F-NT2RP4002791//30S RIBOSOMAL PROTEIN S20.//1.0:73:26//HELICOBACTER PYLORI (CAMPYLOBACTER PYLORI).//P56027
F-NT2RP4002888//HYPOTHETICAL PROTEIN TP0352.//0.98:52:26//TREPONEMA PALLIDUM.//O83371
F-NT2RP4002905//G2/MITOTIC-SPECIFIC CYCLIN S13-7 (B-LIKE CYCLIN) (FRAGMENT).//5.9e-05:138:27//GLYCINE MAX (SOYBEAN).//P25012
F-NT2RP5003459//HOMEOBOX PROTEIN HOX-A3 (HOX-1.5) (MO-10).//0.027:40:40//MUS MUSCULUS (MOUSE).//P02831
F-NT2RP5003461//HYPOTHETICAL PROTEIN C22F3.14C IN CHROMOSOME I (FRAGMENT).//1.1e-12:142:35//SCHIZOSACCHAROMYCES POMBE (FISSION YEAST).//Q09779
F-NT2RP5003477//VEGETATIBLE INCOMPATIBILITY PROTEIN HET-E-1.//5.3e-13:215:28//PODOSPORA ANSERINA.//Q00808
F-NT2RP5003492//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//0.0055:144:27//SACCHAROMYCES CEREVISIAE (BAKER'S YEAST).//P08640
F-NT2RP5003500//PROLINE-RICH PROTEIN MP-2 PRECURSOR.//9.0e-05:103:38//MUS MUSCULUS (MOUSE).//P05142
F-NT2RP5003506//MALE SPECIFIC SPERM PROTEIN MST87F.//0.53:21:38//DROSOPHILA MELANOGASTER (FRUIT FLY).//P08175
F-NT2RP5003512//HYPOTHETICAL PROTEIN IN CYCB 3'REGION PRECURSOR (ORF2) (FRAGMENT).//0.92:49:32//PARACOCCUS DENITRIFICANS.//P29969
F-NT2RP5003522//NADPH-CYTOCHROME P450 REDUCTASE (EC 1.6.2.4) (CPR).//2.7e-18:165:39//PHASEOLUS AUREUS (MUNG BEAN) (VIGNA RADIATA).//P37116
F-NT2RP5003524//COLLAGEN ALPHA 1(I) CHAIN (FRAGMENTS).//6.0e-08:125:41//RATTUS NORVEGICUS (RAT).//P02454
F-NT2RP5003534//ATP SYNTHASE, SUBUNIT F (EC 3.6.1.34).//0.88:37:45//HALOBACTERIUM VOLCANII (HALOFERAX VOLCANII).//Q48331
F-OVARC1000001//GAR22 PROTEIN.//1.9e-05:41:58//HOMO SAPIENS (HUMAN).//Q99501
F-OVARC1000004//70 KD EXOCYST COMPLEX PROTEIN.//3.7e-08:186:25//SACCHAROMYCES CEREVISIAE (BAKER'S YEAST).//P19658
F-OVARC1000006//HISTONE H2A.1.//4.7e-55:117:98//RATTUS NORVEGICUS (RAT).//P02262
F-OVARC1000013//WD-REPEAT PROTEIN POP1.//0.00022:126:28//SCHIZOSACCHAROMYCES POMBE (FISSION YEAST).//P87060
F-OVARC1000014//PROCOLLAGEN ALPHA 1(I) CHAIN PRECURSOR.//2.3e-05:220:30//GALLUS GALLUS (CHICKEN).//P02457
F-OVARC1000017//CUTICLE COLLAGEN DPY-13.//2.6e-05:97:30//CAENORHABDITIS ELEGANS.//P17657
F-OVARC1000035
F-OVARC1000058//RAS-RELATED PROTEIN RABC.//0.00015:110:24//DICTYOSTELIUM DISCOIDEUM (SLIME MOLD).//P34143
F-OVARC1000060//EXTRACELLULAR RIBONUCLEASE LE PRECURSOR (EC 3.1.27.1) (RNASE LE).//6.8e-09:60:45//LYCOPERSICON ESCULENTUM (TOMATO).//P80022
F-OVARC1000068//CYTOTOXIN 4 (CARDIOTOXIN V-II-4).//1.0:27:44//NAJA MOSSAMBICA (MOZAMBIQUE COBRA).//P01452
F-OVARC1000071//NUCLEAR TRANSPORT FACTOR 2 (NTF-2) (PLACENTAL PROTEIN 15) (PP15).//5.2e-06:115:29//HOMO SAPIENS (HUMAN), AND RATTUS NORVEGICUS (RAT).//P13662
F-OVARC1000085
F-OVARC1000087//HISTONE MACRO-H2A.1.//1.2e-13:174:26//RATTUS NORVEGICUS (RAT).//Q02874
F-OVARC1000091//OCTAPEPTIDE-REPEAT PROTEIN T2.//0.0013:137:32//MUS MUSCULUS (MOUSE).//Q06666
F-OVARC1000092//MITOCHONDRIAL RIBOSOMAL PROTEIN S7.//0.97:46:39//ACANTHAMOEBA CASTELLANII (AMOEBA).//P46756
F-OVARC1000106//HYPOTHETICAL 141.5 KD PROTEIN IN YPT53-RHO2 INTERGENIC REGION.//0.0012:165:29//SACCHAROMYCES CEREVISIAE (BAKER'S YEAST).//P53935
F-OVARC1000109//PROLINE RICH 33 KD EXTENSIN-RELATED PROTEIN PRECURSOR (FRAGMENT).//0.18:35:34//DAUCUS CAROTA (CARROT).//P06600
F-OVARC1000113//HYPOTHETICAL PROTEIN C18.//1.0:26:26//SWINEPOX VIRUS (STRAIN KASZA) (SPV).//P32217
F-OVARC1000114//!!!! ALU SUBFAMILY SQ WARNING ENTRY !!!!//3.6e-28:57:63//HOMO SAPIENS (HUMAN).//P39194
F-OVARC1000133
F-OVARC1000139//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN HOMOLOG).//1.9e-09:200:29//HOMO SAPIENS (HUMAN).//Q13107
F-OVARC1000145//HOMEOBOX PROTEIN DLX-3.//1.0:65:30//BRACHYDANIO RERIO (ZEBRAFISH) (ZEBRA DANIO).//Q01702
F-OVARC1000148//HYPHAL WALL PROTEIN 1 (CELL ELONGATION PROTEIN 2).//0.12:175:29//CANDIDA ALBICANS (YEAST).//P46593
F-OVARC1000151//HYPOTHETICAL PROTEIN KIAA0161.//5.6e-20:197:30//HOMO SAPIENS (HUMAN).//P50876
F-OVARC1000168//!!!! ALU SUBFAMILY J WARNING ENTRY !!!!//0.0030:77:38//HOMO SAPIENS (HUMAN).//P39188
F-OVARC1000191//COLANIC ACID BIOSYNTHESIS PROTEIN WCAH.//0.95:56:35//ESCHERICHIA COLI.//P32056
F-OVARC1000198//HISTONE H1.C2.//0.96:70:25//TRYPANOSOMA CRUZI.//P40268
F-OVARC1000209//HYPOTHETICAL 20.9 KD PROTEIN IN PLB1-HXT2 INTERGENIC REGION.//2.5e-33:178:44//SACCHAROMYCES CEREVISIAE (BAKER'S YEAST).//Q03677
F-OVARC1000212//PROLINE-RICH PROTEIN MP-2 PRECURSOR.//1.7e-05:66:46//MUS MUSCULUS (MOUSE).//P05142
F-OVARC1000240//!!!! ALU SUBFAMILY SP WARNING ENTRY !!!!//1.8e-10:41:78//HOMO SAPIENS (HUMAN).//P39193
F-OVARC1000241//ENDOTHELIAL PAS DOMAIN PROTEIN 1 (EPAS-1) (HIF-1 ALPHA-LIKE FACTOR) (MHLF) (HIF-RELATED FACTOR) (HRF).//7.4e-54:177:54//MUS MUSCULUS (MOUSE).//P97481
F-OVARC1000288//HYPOTHETICAL 54.2 KD PROTEIN IN ERP5-ORC6 INTERGENIC REGION.//2.9e-20:115:45//SACCHAROMYCES CEREVISIAE (BAKER'S YEAST).//P38821
F-OVARC1000302//CORTICOSTEROID-BINDING GLOBULIN PRECURSOR (CBG) (TRANSCORTIN).//1.0:79:25//MUS MUSCULUS (MOUSE).//Q06770
F-OVARC1000304//PROTEIN MOV-10.//1.6e-79:181:83//MUS MUSCULUS (MOUSE).//P23249
F-OVARC1000309//THREONINE SYNTHASE (EC 4.2.99.2).//6.9e-36:156:42//ASHBYA GOSSYPII (EREMOTHECIUM GOSSYPII).//Q00063
F-OVARC1000321//HYPOTHETICAL 28.1 KD PROTEIN C4F8.03 IN CHROMOSOME I.//5.2e-45:159:53//SCHIZOSACCHAROMYCES POMBE (FISSION YEAST).//O14179
F-OVARC1000326//BASIC PROLINE-RICH PEPTIDE IB-1.//0.036:67:35//HOMO SAPIENS (HUMAN).//P04281
F-OVARC1000335//HYPOTHETICAL 39.3 KD PROTEIN IN GCN4-WBP1 INTERGENIC REGION.//1.2e-16:200:27//SACCHAROMYCES CEREVISIAE (BAKER'S YEAST).//P40004
F-OVARC1000347//HYPOTHETICAL 7.6 KD PROTEIN YCF33.//0.69:41:43//CYANOPHORA PARADOXA.//P48273
F-OVARC1000384//ANTIFREEZE PEPTIDE 4 PRECURSOR.//0.98:49:34//PSEUDOPLEURONECTA AMERICANUS (WINTER FLOUNDER).//P02734
F-OVARC1000408//INTEGUMENTARY MUCIN C.1 (FIM-C.1) (FRAGMENT).//8.1e-05:115:33//XENOPUS LAEVIS (AFRICAN CLAWED FROG).//Q05049
F-OVARC1000411//DYNACTIN, 150 KD ISOFORM (150 KD DYNEIN-ASSOCIATED POLYPEPTIDE) (DP-150) (DAP-150) (P150-GLUED).//0.00076:100:29//RATTUS NORVEGICUS (RAT).//P28023
F-OVARC1000414//HYPOTHETICAL 7.0 KD PROTEIN IN BLTR-SPOIIIC INTERGENIC REGION.//1.0:46:34//BACILLUS SUBTILIS.//P54431
F-OVARC1000420//COLLAGEN ALPHA 2(VIII) CHAIN (ENDOTHELIAL COLLAGEN) (FRAGMENT).//0.0028:97:37//HOMO SAPIENS (HUMAN).//P25067
F-OVARC1000427//HYPOTHETICAL 13.9 KD PROTEIN IN PRFA-SPOIIR INTERGENIC REGION.//0.70:21:47//BACILLUS SUBTILIS.//P39150
F-OVARC1000431
F-OVARC1000437//TENSIN.//9.2e-42:195:52//GALLUS GALLUS (CHICKEN).//Q04205
F-OVARC1000440//PINCH PROTEIN (PARTICULARY INTERESTING NEW CYS-HIS PROTEIN).//3.4e-31:37:97//HOMO SAPIENS (HUMAN).//P48059
F-OVARC1000442
F-OVARC1000443//CUTICLE COLLAGEN 2C (FRAGMENT).//0.0056:163:34//HAEMONCHUS CONTORTUS.//P16252
F-OVARC1000461//FIXU PROTEIN.//0.36:36:44//RHIZOBIUM LEGUMINOSARUM (BIOVAR TRIFOLII).//P42710
F-OVARC1000465//PROTEIN TRANSPORT PROTEIN SEC7.//2.4e-14:222:26//SACCHAROMYCES CEREVISIAE (BAKER'S YEAST).//P11075
F-OVARC1000466//!!!! ALU SUBFAMILY SC WARNING ENTRY !!!!//2.3e-08:29:93//HOMO SAPIENS (HUMAN).//P39192
F-OVARC1000473//DUAL SPECIFICITY PROTEIN PHOSPHATASE 7 (EC 3.1.3.48) (EC 3.1.3.16) (DUAL SPECIFICITY PROTEIN PHOSPHATASE MKP-X) (FRAGMENT).//2.8e-06:96:36//RATTUS NORVEGICUS (RAT).//Q63340
F-OVARC1000479//PHOTOSYSTEM I REACTION CENTRE SUBUNIT X (PSI-K).//0.99:48:37//CYANIDIUM CALDARIUM (GALDIERIA SULPHURARIA).//P31567 F-OVARC1000486
F-OVARC1000496//HYPOTHETICAL PROTEIN MJ1213.//1.0:62:32//METHANOCOCCUS JANNASCHII.//Q58610
F-OVARC1000520//MEROZOITE SURFACE PROTEIN CMZ-8 (FRAGMENT).//0.0011:66:40//EIMERIA ACERVULINA.//P09125
F-OVARC1000526//PROTEIN Q300.//1.2e-05:51:43//MUS MUSCULUS (MOUSE).//Q02722
F-OVARC1000533//NEURONAL PROTEIN 3.1 (P311 PROTEIN).//0.74:43:41//HOMO SAPIENS (HUMAN).//Q16612
F-OVARC1000543//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N- ACETYLGALACTOSAMINYLTRANSFERASE) (GALNAC-T1).//2.3e-23:192:35//HOMO SAPIENS (HUMAN).//Q10472
F-OVARC1000556
F-OVARC1000557//!!!! ALU SUBFAMILY J WARNING ENTRY !!!!//1.6e-08:80:47//HOMO SAPIENS (HUMAN).//P39188
F-OVARC1000564//VPX PROTEIN (X ORF PROTEIN) (VIRAL ACCESSORY PROTEIN).//0.45:32:50//HUMAN IMMUNODEFICIENCY VIRUS TYPE 2 (ISOLATE D194) (HIV-2).//P17760
F-OVARC1000573
F-OVARC1000576//BETA-DEFENSIN 1 (BNDB-1).//0.47:29:41//BOS TAURUS (BOVINE).//P46159
F-OVARC1000578//COLLAGEN ALPHA 1(II) CHAIN (FRAGMENTS).//0.023:96:36//BOS TAURUS (BOVINE).//P02459
F-OVARC1000588//MITOCHONDRIAL 60S RIBOSOMAL PROTEIN L3.//0.75:57:29//HOMO SAPIENS (HUMAN).//P09001
F-OVARC1000605//AUTOLYSIN PRECURSOR (EC 3.4.24.38) (GAMETE LYTIC ENZYME) (GLE).//0.91:134:28//CHLAMYDOMONAS REINHARDTII.//P31178
F-OVARC1000622//!!!! ALU SUBFAMILY SB WARNING ENTRY !!!!//2.6e-36:100:80//HOMO SAPIENS (HUMAN).//P39189
F-OVARC1000640//HYPOTHETICAL 8.5 KD PROTEIN YCF40 (ORF73).//0.96:34:38//ODONTELLA SINENSIS.//P49535
F-OVARC1000649//ANTHER-SPECIFIC PROTEIN SF18 PRECURSOR (FRAGMENT).//0.0036:64:37//HELIANTHUS ANNUUS (COMMON SUNFLOWER).//P22357
F-OVARC1000661//COLLAGEN ALPHA 2(I) CHAIN (FRAGMENTS).//0.21:53:47//RATTUS NORVEGICUS (RAT).//P02466
F-OVARC1000678//BACTERIOCIN MICROCIN B17 PRECURSOR (MCB17).//1.0:17:58//ESCHERICHIA COLI.//P05834
F-OVARC1000679//DNA-DIRECTED RNA POLYMERASE OMEGA CHAIN (EC 2.7.7.6) (TRANSCRIPTASE OMEGA CHAIN) (RNA POLYMERASE OMEGA SUBUNIT).//0.096:67:29//ESCHERICHIA COLI.//P08374
F-OVARC1000681//PROTEIN Q300.//0.72:16:43//MUS MUSCULUS (MOUSE).//Q02722
F-OVARC1000682//PROCESSING ALPHA-1,2-MANNOSIDASE (EC 3.2.1.-) (ALPHA-1,2-MANNOSIDASE 1B).//7.6e-70:102:99//MUS MUSCULUS (MOUSE).//P39098
F-OVARC1000689//CADMIUM-METALLOTHIONEIN (CD-MT).//0.032:30:40//HELIX POMATIA (ROMAN SNAIL) (EDIBLE SNAIL).//P33187
F-OVARC1000700//BRAIN NEURON CYTOPLASMIC PROTEIN 2.//0.17:60:40//RATTUS NORVEGICUS (RAT).//P02684
F-OVARC1000703//BASIC PROLINE-RICH PEPTIDE P-E (IB-9).//0.57:42:42//HOMO SAPIENS (HUMAN).//P02811
F-OVARC1000722//N-ACETYLLACTOSAMINE SYNTHASE (EC 2.4.1.90) (N-ACETYLGLUCOSAMINE (BETA 1->4)GALACTOSYLTRANSFERASE) (EC 2.4.1.38) (LACTOSE SYNTHASE A PROTEIN (EC 2.4.1.22)) (GALACTOSYLTRANSFERASE) (GT).//1.1e-20:44:70//BOS TAURUS (BOVINE).//P08037
F-OVARC1000730//HYPOTHETICAL 83.8 KD PROTEIN C27F2.7 IN CHROMOSOME III.//5.2e-29:224:36//CAENORHABDITIS ELEGANS.//Q18262
F-OVARC1000746//MATERNAL EFFECT PROTEIN STAUFEN.//6.2e-12:78:48//DROSOPHILA MELANOGASTER (FRUIT FLY).//P25159
F-OVARC1000769
F-OVARC1000771//RAS-RELATED PROTEIN RAB-2.//1.1e-46:121:79//HOMO SAPIENS (HUMAN), AND CANIS FAMILIARIS (DOG).//P08886
F-OVARC1000781//HOMEOBOX PROTEIN GBX-2 (GASTRULATION AND BRAIN-SPECIFIC HOMEOBOX PROTEIN 2).//0.81:36:52//HOMO SAPIENS (HUMAN).//P52951
F-OVARC1000787//40S RIBOSOMAL PROTEIN S14 (FRAGMENT).//0.96:37:48//SUS SCROFA (PIG).//Q29303
F-OVARC1000800//!!!! ALU SUBFAMILY SB WARNING ENTRY !!!!//2.5e-31:47:82//HOMO SAPIENS (HUMAN).//P39189
F-OVARC1000802//HYPOTHETICAL 8.8 KD PROTEIN B0302.2 IN CHROMOSOME X.//0.16:55:40//CAENORHABDITIS ELEGANS.//Q10926
F-OVARC1000834//SERINE/THREONINE-PROTEIN KINASE PAK-ALPHA (EC 2.7.1.-) (P68-PAK) (P21- ACTIVATED KINASE) (ALPHA-PAK) (PROTEIN KINASE MUK2).//0.87:140:31//RATTUS NORVEGICUS (RAT).//P35465
F-OVARC1000846//NUCLEOLIN (PROTEIN C23).//7.0e-07:109:30//MESOCRICETUS AURATUS (GOLDEN HAMSTER).//P08199
F-OVARC1000850//HYPOTHETICAL 56.2 KD PROTEIN IN ERG8-UBP8 INTERGENIC REGION.//6.9e-09:180:28//SACCHAROMYCES CEREVISIAE (BAKER'S YEAST).//Q04991
F-OVARC1000862//UBIQUITIN-CONJUGATING ENZYME E2-17.5 KD (EC 6.3.2.19) (UBIQUITIN- PROTEIN LIGASE) (UBIQUITIN CARRIER PROTEIN).//0.0020:74:28//SACCHAROMYCES CEREVISIAE (BAKER'S YEAST).//P52490
F-OVARC1000876//MOB1 PROTEIN (MPS1 BINDER 1).//9.8e-39:154:55//SACCHAROMYCES CEREVISIAE (BAKER'S YEAST).//P40484
F-OVARC1000883//METALLOTHIONEIN-I.//0.87:38:36//CANDIDA GLABRATA (YEAST) (TORULOPSIS GLABRATA).//P15113
F-OVARC1000885//OXIDOREDUCTASE UCPA (EC 1.-.-.-).//2.8e-18:170:34//ESCHERICHIA COLI.//P37440
F-OVARC1000886//COLLAGEN ALPHA 2(I) CHAIN (FRAGMENT).//0.00033:60:45//BOS TAURUS (BOVINE).//P02465
F-OVARC1000890//PROBABLE E5 PROTEIN.//0.92:7:71//HUMAN PAPILLOMAVIRUS TYPE 70.//P50774
F-OVARC1000891//HYPOTHETICAL 8.3 KD PROTEIN (ORF5).//1.0:36:36//PARAMECIUM TETRAURELIA.//P15606
F-OVARC1000897//HYPOTHETICAL 6.1 KD PROTEIN PRECURSOR (ORF87).//1.0:34:44//ORGYIA PSEUDOTSUGATA MULTICAPSID POLYHEDROSIS VIRUS (OPMNPV).//O10337
F-OVARC1000912//PUTATIVE CUTICLE COLLAGEN C09G5.4.//4.0e-07:98:35//CAENORHABDITIS ELEGANS.//Q09455
F-OVARC1000915//HYPOTHETICAL PROTEIN KIAA0288 (HA6116).//1.7e-47:115:76//HOMO SAPIENS (HUMAN).//P56524
F-OVARC1000924//CYTOCHROME B (EC 1.10.2.2) (FRAGMENT).//0.99:54:24//BOA CONSTRICTOR (BOA).//P92848
F-OVARC1000936//HYPOTHETICAL 7.5 KD PROTEIN IN INAA-GLPQ INTERGENIC REGION.//1.0:48:33//ESCHERICHIA COLI.//P45505
F-OVARC1000937//PROCOLLAGEN ALPHA 1(I) CHAIN PRECURSOR.//1.0:135:31//HOMO SAPIENS (HUMAN).//P02452
F-OVARC1000945//EARLY E1A 11 KD PROTEIN.//0.087:81:24//MOUSE ADENOVIRUS TYPE 1 (MAV-1).//P12533
F-OVARC1000948
F-OVARC1000959//HYPOTHETICAL PROTEIN MJ0933.//0.99:67:28//METHANOCOCCUS JANNASCHII.//Q58343
F-OVARC1000960//!!!! ALU SUBFAMILY SP WARNING ENTRY !!!!//1.8e-32:56:75//HOMO SAPIENS (HUMAN).//P39193
F-OVARC1000964//MAMBIN (GLYCOPROTEIN IIB-IIA ANTAGONIST) (PLATELET AGGREGATION INHIBITOR) (DENDROASPIN).//1.0:30:36//DENDROASPIS JAMESONI KAIMOSAE (EASTERN JAMESON'S MAMBA).//P28375
F-OVARC1000971
F-OVARC1000984//HYPOTHETICAL 52.3 KD PROTEIN IN MRPL10-ERG24 INTERGENIC REGION PRECURSOR.//0.093:36:47//SACCHAROMYCES CEREVISIAE (BAKER'S YEAST).//P53832
F-OVARC1000996//MO25 PROTEIN.//1.9e-39:80:95//MUS MUSCULUS (MOUSE).//Q06138
F-OVARC1000999//BRAIN-SPECIFIC HOMEOBOX/POU DOMAIN PROTEIN 1 (BRN-1 PROTEIN).//0.00020:50:40//HOMO SAPIENS (HUMAN).//P20264
F-OVARC1001000//!!!! ALU SUBFAMILY SX WARNING ENTRY !!!!//1.4e-16:43:90//HOMO SAPIENS (HUMAN).//P39195
F-OVARC1001004//MALE SPECIFIC SPERM PROTEIN MST84DA.//0.95:33:42//DROSOPHILA MELANOGASTER (FRUIT FLY).//Q01642
F-OVARC1001010//HYPOTHETICAL PROTEIN MJ0926.//0.50:71:23//METHANOCOCCUS JANNASCHII.//Q58336
F-OVARC1001011//CORTISTATIN PRECURSOR.//0.81:45:37//RATTUS NORVEGICUS (RAT).//Q62949
F-OVARC1001032//FERREDOXIN LIKE PROTEIN.//1.0:26:46//RHIZOBIUM LEGUMINOSARUM (BIOVAR PHASEOLI).//Q05561
F-OVARC1001034//METALLOTHIONEIN-IG (MT-1G).//0.14:9:77//HOMO SAPIENS (HUMAN).//P13640
F-OVARC1001038//NUCLEOLIN (PROTEIN C23).//3.2e-07:36:80//HOMO SAPIENS (HUMAN).//P19338
F-OVARC1001040//!!!! ALU SUBFAMILY SQ WARNING ENTRY !!!!//1.5e-18:45:60//HOMO SAPIENS (HUMAN).//P39194
F-OVARC1001044//BIS(5'-NUCLEOSYL)-TETRAPHOSPHATASE (SYMMETRICAL) (EC 3.6.1.41) (DIADENOSINE TETRAPHOSPHATASE).//0.88:43:39//ESCHERICHIA COLI.//P05637
F-OVARC1001051//SERINE PROTEINASE STUBBLE (EC 3.4.21.-) (STUBBLE-STUBBLOID PROTEIN).//0.34:117:25//DROSOPHILA MELANOGASTER (FRUIT FLY).//Q05319
F-OVARC1001055//PRE-B CELL ENHANCING FACTOR PRECURSOR.//1.6e-33:43:97//HOMO SAPIENS (HUMAN).//P43490
F-OVARC1001062
F-OVARC1001065//METHIONYL-TRNA SYNTHETASE (EC 6.1.1.10) (METHIONINE--TRNA LIGASE) (METRS).//0.79:76:39//BORRELIA BURGDORFERI (LYME DISEASE SPIROCHETE).//Q44951
F-OVARC1001068//GTP-BINDING PROTEIN ERA HOMOLOG (FRAGMENT).//5.3e-15:100:44//BRADYRHIZOBIUM JAPONICUM.//O69162
F-OVARC1001072//!!!! ALU SUBFAMILY J WARNING ENTRY !!!!//0.0076:41:56//HOMO SAPIENS (HUMAN).//P39188
F-OVARC1001074//60S RIBOSOMAL PROTEIN L38.//1.0:32:40//LYCOPERSICON ESCULENTUM (TOMATO).//P46291
F-OVARC1001085//HYPOTHETICAL 126.5 KD PROTEIN C13F4.06 IN CHROMOSOME I.//0.73:135:25//SCHIZOSACCHAROMYCES POMBE (FISSION YEAST).//Q10197
F-OVARC1001092//HYPOTHETICAL 51.2 KD PROTEIN IN PET54-DIE2 INTERGENIC REGION.//5.6e-05:30:56//SACCHAROMYCES CEREVISIAE (BAKER'S YEAST).//P50079
F-OVARC1001107//SHK1 KINASE-BINDING PROTEIN 1.//1.8e-08:52:51//SCHIZOSACCHAROMYCES POMBE (FISSION YEAST).//P78963
F-OVARC1001113//DIAPHANOUS PROTEIN.//1.9e-33:218:35//DROSOPHILA MELANOGASTER (FRUIT FLY).//P48608
F-OVARC1001117//GENE 7 PROTEIN.//0.68:12:50//SPIROPLASMA VIRUS 4 (SPV4).//P11339
F-OVARC1001118
F-OVARC1001129//30S RIBOSOMAL PROTEIN S17.//0.15:57:22//AQUIFEX AEOLICUS.//O66439
F-OVARC1001154//GRANULINS PRECURSOR (ACROGRANIN).//2.3e-95:99:77//MUS MUSCULUS (MOUSE).//P28798
F-OVARC1001161//GUANINE NUCLEOTIDE-BINDING PROTEIN BETA SUBUNIT.//0.17:87:34//ARABIDOPSIS THALIANA (MOUSE-EAR CRESS).//P49177
F-OVARC1001162
F-OVARC1001167//TRBD PROTEIN.//0.92:24:45//ESCHERICHIA COLI.//P41070
F-OVARC1001169//FRUCTOSE-1,6-BISPHOSPHATASE (EC 3.1.3.11) (D-FRUCTOSE-1,6-BISPHOSPHATE 1-PHOSPHOHYDROLASE) (FBPASE) (FRAGMENT).//0.82:35:40//MUS MUSCULUS (MOUSE).//P97323
F-OVARC1001170//PROLINE-RICH PEPTIDE P-B.//0.17:27:37//HOMO SAPIENS (HUMAN).//P02814
F-OVARC1001171//!!!! ALU SUBFAMILY J WARNING ENTRY !!!!//0.00023:28:75//HOMO SAPIENS (HUMAN).//P39188
F-OVARC1001173
F-OVARC1001176//HYPOTHETICAL BHLF1 PROTEIN.//2.7e-05:158:31//EPSTEIN-BARR VIRUS (STRAIN B95-8) (HUMAN HERPESVIRUS 4).//P03181
F-OVARC1001180//UBIQUITIN-LIKE PROTEIN DSK2.//1.4e-12:208:25//SACCHAROMYCES CEREVISIAE (BAKER'S YEAST).//P48510
F-OVARC1001188//HYPOTHETICAL 27.8 KD PROTEIN IN VMA7-RPS25A INTERGENIC REGION.//3.3e-31:129:51//SACCHAROMYCES CEREVISIAE (BAKER'S YEAST).//P53215
F-OVARC1001200//HYPOTHETICAL 49.0 KD PROTEIN IN NSP1-KAR2 INTERGENIC REGION.//0.018:148:26//SACCHAROMYCES CEREVISIAE (BAKER'S YEAST).//P47057
F-OVARC1001232//HYPOTHETICAL PROTEIN MJ1236.//2.5e-27:141:39//METHANOCOCCUS JANNASCHII.//Q58633
F-OVARC1001240
F-OVARC1001243
F-OVARC1001244//RING3 PROTEIN (KIAA9001).//1.7e-13:37:91//HOMO SAPIENS (HUMAN).//P25440
F-OVARC1001261//OCTAPEPTIDE-REPEAT PROTEIN T2.//1.3e-07:109:35//MUS MUSCULUS (MOUSE).//Q06666
F-OVARC1001268//HYPOTHETICAL 57.4 KD PROTEIN IN PILT REGION (ORF4).//0.71:43:41//PSEUDOMONAS AERUGINOSA.//P24563
F-OVARC1001270//HYPOTHETICAL 9.0 KD PROTEIN IN UVSW-UVSY INTERGENIC REGION.//1.0:44:29//BACTERIOPHAGE T4.//P32281
F-OVARC1001271//HYPOTHETICAL 104.7 KD PROTEIN F23F12.8 IN CHROMOSOME III PRECURSOR.//0.00015:188:23//CAENORHABDITIS ELEGANS.//P46504
F-OVARC1001282
F-OVARC1001296//WEB1 PROTEIN (PROTEIN TRANSPORT PROTEIN SEC31).//0.022:101:31//SACCHAROMYCES CEREVISIAE (BAKER'S YEAST).//P38968
F-OVARC1001306//HYPOTHETICAL 52.9 KD SERINE-RICH PROTEIN C11G7.01 IN CHROMOSOME I.//0.023:134:26//SCHIZOSACCHAROMYCES POMBE (FISSION YEAST).//O13695
F-OVARC1001329//CHLOROPLAST TRIOSE PHOSPHATE TRANSLOCATOR PRECURSOR (CTPT).//1.3e-14:150:28//ZEA MAYS (MAIZE).//P49133
F-OVARC1001330
F-OVARC1001339//RIBONUCLEOPROTEIN RB97D.//0.0013:55:38//DROSOPHILA MELANOGASTER (FRUIT FLY).//Q02926
F-OVARC1001341//HYPOTHETICAL 74.0 KD PROTEIN IN CAJ1-HOM3 INTERGENIC REGION.//4.9e-17:110:43//SACCHAROMYCES CEREVISIAE (BAKER'S YEAST).//P40032
F-OVARC1001342
F-OVARC1001344//PREPROTEIN TRANSLOCASE SECE SUBUNIT.//0.99:39:23//STAPHYLOCOCCUS CARNOSUS.//P36253
F-OVARC1001357//METALLOTHIONEIN.//0.99:28:42//XENOPUS LAEVIS (AFRICAN CLAWED FROG).//Q05890
F-OVARC1001360//LARGE PROLINE-RICH PROTEIN BAT2 (HLA-B-ASSOCIATED TRANSCRIPT 2).//0.86:109:31//HOMO SAPIENS (HUMAN).//P48634
F-OVARC1001369//COLLAGEN ALPHA 2(I) CHAIN (FRAGMENT).//6.7e-05:124:36//BOS TAURUS (BOVINE).//P02465
F-OVARC1001372//HYPOTHETICAL 34.5 KD PROTEIN IN CLCB-CLCD INTERGENIC REGION PRECURSOR.//0.75:33:48//PSEUDOMONAS PUTIDA, AND PSEUDOMONAS SP. (STRAIN B13).//Q47100
F-OVARC1001376//!!!! ALU SUBFAMILY J WARNING ENTRY !!!!//2.8e-24:96:61//HOMO SAPIENS (HUMAN).//P39188
F-OVARC1001381//MEMBRANE-ASSOCIATED ATPASE EPSILON CHAIN (EC 3.6.1.34) (SUL-ATPASE EPSILON).//0.96:46:39//SULFOLOBUS ACIDOCALDARIUS.//P23039
F-OVARC1001391//SALIVARY PROLINE-RICH PROTEIN PO (ALLELE K) [CONTAINS: PEPTIDE P-D] (FRAGMENT).//0.00024:189:29//HOMO SAPIENS (HUMAN).//P10162
F-OVARC1001399//!!!! ALU SUBFAMILY SX WARNING ENTRY !!!!//0.062:18:77//HOMO SAPIENS (HUMAN).//P39195
F-OVARC1001417//HYPOTHETICAL 157.0 KD PROTEIN C38C10.5 IN CHROMOSOME III.//0.010:185:23//CAENORHABDITIS ELEGANS.//Q03570
F-OVARC1001419//A-TYPE INCLUSION PROTEIN (ATI).//0.50:135:28//CAMELPOX VIRUS (STRAIN CP-1).//Q05482
F-OVARC1001425//COLLAGEN ALPHA 1(X) CHAIN PRECURSOR.//0.43:85:40//HOMO SAPIENS (HUMAN).//Q03692
F-OVARC1001436//HYPOTHETICAL 11.4 KD PROTEIN (C4 PROTEIN).//0.031:100:30//TOMATO YELLOW LEAF CURL VIRUS (STRAIN AUSTRALIA) (TYLCV).//P36283
F-OVARC1001442//HOMEOBOX PROTEIN HTR-A2 (FRAGMENT).//1.0:32:34//HELOBDELLA TRISERIALIS (LEECH).//P17138
F-OVARC1001453//METALLOTHIONEIN-III (MT-III) (GROWTH INHIBITORY FACTOR) (GIF).//0.74:19:47//MUS MUSCULUS (MOUSE).//P28184
F-OVARC1001476//GTP-BINDING PROTEIN GTR2.//3.0e-12:114:34//SACCHAROMYCES CEREVISIAE (BAKER'S YEAST).//P53290
F-OVARC1001480//COLLAGEN ALPHA 2(VI) CHAIN PRECURSOR.//0.00019:134:32//MUS MUSCULUS (MOUSE).//Q02788
F-OVARC1001489//HYPOTHETICAL PROTEIN HI1270.//0.98:30:43//HAEMOPHILUS INFLUENZAE.//P44149
F-OVARC1001496//C-TERMINAL BINDING PROTEIN 2.//4.0e-65:132:100//HOMO SAPIENS (HUMAN).//P56545
F-OVARC1001506//POLYCYSTIN PRECURSOR (AUTOSOMAL DOMINANT POLYCYSTIC KIDNEY DISEASE PROTEIN 1).//3.2e-70:159:94//HOMO SAPIENS (HUMAN).//P98161
F-OVARC1001525//FIBROBLAST GROWTH FACTOR INDUCIBLE PROTEIN 14 (FIN14).//1.0:36:33//MUS MUSCULUS (MOUSE).//Q61077
F-OVARC1001542//SMALL PROLINE-RICH PROTEIN 2B (SPR-2B).//0.69:57:33//HOMO SAPIENS (HUMAN).//P35325
F-OVARC1001547
F-OVARC1001555//NGG1-INTERACTING FACTOR 3.//7.6e-16:148:34//SACCHAROMYCES CEREVISIAE (BAKER'S YEAST).//P53081
F-OVARC1001577//SPLICING FACTOR, ARGININE/SERINE-RICH 2 (SPLICING FACTOR SC35) (SC-35) (SPLICING COMPONENT, 35 KD) (PR264 PROTEIN).//8.8e-38:94:81//GALLUS GALLUS (CHICKEN).//P30352
F-OVARC1001600//GENE 7 PROTEIN.//0.80:38:39//SPIROPLASMA VIRUS SPV1-R8A2 B.//P15898
F-OVARC1001610//DIACYLGLYCEROL CHOLINEPHOSPHOTRANSFERASE (EC 2.7.8.2) (SN-1,2- DIACYLGLYCEROL CHOLINEPHOSPHOTRANSFERASE) (CHOPT).//1.6e-22:122:39//SACCHAROMYCES CEREVISIAE (BAKER'S YEAST).//P17898
F-OVARC1001611
F-OVARC1001615//HYPOTHETICAL 6.1 KD PROTEIN C03B1.10 IN CHROMOSOME X.//0.30:43:34//CAENORHABDITIS ELEGANS.//Q11116
F-OVARC1001668//!!!! ALU SUBFAMILY SC WARNING ENTRY !!!!//1.0e-19:45:82//HOMO SAPIENS (HUMAN).//P39192
F-OVARC1001702//SOX-20 PROTEIN.//2.4e-28:71:83//HOMO SAPIENS (HUMAN).//O60248
F-OVARC1001703//INTERFERON-INDUCED GUANYLATE-BINDING PROTEIN 1 (GUANINE NUCLEOTIDE- BINDING PROTEIN 1) (INTERFERON-GAMMA INDUCIBLE PROTEIN MAG-1).//0.00018:88:36//MUS MUSCULUS (MOUSE).//Q01514
F-OVARC1001711//CORNIFIN B (SMALL PROLINE-RICH PROTEIN 1B) (SPR1B) (SPR1 B).//2.7e-05:98:32//MUS MUSCULUS (MOUSE).//Q62267
F-OVARC1001713//ENDOZEPINE-RELATED PROTEIN PRECURSOR (MEMBRANE-ASSOCIATED DIAZEPAM BINDING INHIBITOR) (MA-DBI).//4.5e-20:46:67//BOS TAURUS (BOVINE).//P07106
F-OVARC1001726//ALPHA-AMYLASE INHIBITOR PAIM I (PIG PANCREATIC ALPHA-AMYLASE INHIBITOR OF MICROBES I).//0.59:23:56//STREPTOMYCES OLIVACEOVIRIDIS (STREPTOMYCES CORCHORUSII).//P09921
F-OVARC1001731//TROPOMYOSIN ALPHA CHAIN, SKELETAL MUSCLE.//2.1e-75:176:87//XENOPUS LAEVIS (AFRICAN CLAWED FROG).//Q01173
F-OVARC1001745//GENE 11 PROTEIN.//0.31:36:52//SPIROPLASMA VIRUS SPV1-R8A2 B.//P15902
F-OVARC1001762//N-TERMINAL ACETYLTRANSFERASE 1 (EC 2.3.1.88) (AMINO-TERMINAL, ALPHA- AMINO, ACETYLTRANSFERASE 1).//2.8e-23:197:35//SACCHAROMYCES CEREVISIAE (BAKER'S YEAST).//P12945
F-OVARC1001766//FK506-BINDING NUCLEAR PROTEIN (PEPTIDYL-PROLYL CIS-TRANS ISOMERASE) (PPIASE) (EC 5.2.1.8) (PROLINE ROTAMASE) (NUCLEOLAR PROLINE ISOMERASE) (FKBP-70).//2.2e-06:99:40//SACCHAROMYCES CEREVISIAE (BAKER'S YEAST).//P38911
F-OVARC1001767//33.2 KD PROTEIN IN DIND-RPH INTERGENIC REGION (ORF X).//0.99:113:27//ESCHERICHIA COLI.//P23839
F-OVARC1001768
F-OVARC1001791//HYPOTHETICAL 63.3 KD PROTEIN IN MPT5-SAE2 INTERGENIC REGION.//0.090:75:32//SACCHAROMYCES CEREVISIAE (BAKER'S YEAST).//P46945
F-OVARC1001795//HYPOTHETICAL 7.5 KD PROTEIN IN RPBA-GP46 INTERGENIC REGION.//0.81:21:38//BACTERIOPHAGE T4.//P07878
F-OVARC1001802//PLECTOXIN VIII (PLT-VIII) (PLTVIII).//0.41:19:36//PLECTREURYS TRISTIS (SPIDER).//P36984
F-OVARC1001805//60S RIBOSOMAL PROTEIN L40 (CEP52).//0.67:24:58//SACCHAROMYCES CEREVISIAE (BAKER'S YEAST).//P14796
F-OVARC1001809//COLLAGEN ALPHA 1(I) CHAIN (FRAGMENTS).//0.23:111:31//RATTUS NORVEGICUS (RAT).//P02454
F-OVARC1001812//ATP SYNTHASE PROTEIN 8 (EC 3.6.1.34) (A6L).//0.99:28:42//HALICHOERUS GRYPUS (GRAY SEAL).//P38592
F-OVARC1001813//HYPOTHETICAL 9.9 KD PROTEIN.//0.41:36:30//VACCINIA VIRUS (STRAIN COPENHAGEN).//P20562
F-OVARC1001820//HYPOTHETICAL PROTEIN ORF-1137.//0.80:58:29//MUS MUSCULUS (MOUSE).//P11260
F-OVARC1001828
F-OVARC1001846
F-OVARC1001861//METALLOTHIONEIN (MT).//0.18:11:54//PLEURONECTES PLATESSA (PLAICE).//P07216
F-OVARC1001873
F-OVARC1001879//HYPOTHETICAL 55.9 KD PROTEIN EEED8.6 IN CHROMOSOME II.//2.3e-05:73:31//CAENORHABDITIS ELEGANS.//Q09296
F-OVARC1001880//SALIVARY PROLINE-RICH PROTEIN PRECURSOR (CLONE CP7) [CONTAINS: BASIC PEPTIDE P-F] (FRAGMENT).//2.4e-11:203:32//HOMO SAPIENS (HUMAN).//P02812
F-OVARC1001883//!!!! ALU SUBFAMILY J WARNING ENTRY !!!!//2.3e-16:86:59//HOMO SAPIENS (HUMAN).//P39188
F-OVARC1001900//HYPOTHETICAL 105.9 KD PROTEIN F22B7.5 IN CHROMOSOME III.//0.0053:48:47//CAENORHABDITIS ELEGANS.//P34408
F-OVARC1001901
F-OVARC1001911//40S RIBOSOMAL PROTEIN S28.//1.0:33:36//ARABIDOPSIS THALIANA (MOUSE-EAR CRESS).//P34789
F-OVARC1001916//PUTATIVE RHO/RAC GUANINE NUCLEOTIDE EXCHANGE FACTOR (RHO/RAC GEF) (FACIOGENITAL DYSPLASIA PROTEIN).//0.00082:114:27//HOMO SAPIENS (HUMAN).//P98174
F-OVARC1001928//FERREDOXIN III (FDIII).//1.0:64:29//ANABAENA VARIABILIS.//P46050
F-OVARC1001942//N-TERMINAL ACETYLTRANSFERASE 1 (EC 2.3.1.88) (AMINO-TERMINAL, ALPHA- AMINO, ACETYLTRANSFERASE 1).//3.0e-07:93:37//SACCHAROMYCES CEREVISIAE (BAKER'S YEAST).//P12945
F-OVARC1001943//HYPOTHETICAL 62.2 KD PROTEIN ZK652.6 IN CHROMOSOME III.//1.7e-23:147:43//CAENORHABDITIS ELEGANS.//P34664
F-OVARC1001949//ZINC FINGER PROTEIN 177.//2.0e-23:56:66//HOMO SAPIENS (HUMAN).//Q13360
F-OVARC1001950//!!!! ALU SUBFAMILY J WARNING ENTRY !!!!//0.011:57:47//HOMO SAPIENS (HUMAN).//P39188
F-OVARC1001987//SPERM PROTAMINE P1 (CYSTEINE-RICH PROTAMINE).//0.39:14:64//MUS MUSCULUS (MOUSE).//P02319
F-OVARC1001989//!!!! ALU SUBFAMILY J WARNING ENTRY !!!!//2.4e-13:55:72//HOMO SAPIENS (HUMAN).//P39188
F-OVARC1002044
F-OVARC1002050//UTROPHIN (DYSTROPHIN-RELATED PROTEIN 1) (DRP1) (DRP).//3.6e-12:221:25//HOMO SAPIENS (HUMAN).//P46939
F-OVARC1002066
F-OVARC1002082
F-OVARC1002107//INTRACELLULAR PROTEIN TRANSPORT PROTEIN USO1.//0.99:149:24//SACCHAROMYCES CEREVISIAE (BAKER'S YEAST).//P25386
F-OVARC1002112//HISTONE MACRO-H2A.1.//2.8e-64:133:98//RATTUS NORVEGICUS (RAT).//Q02874
F-OVARC1002127//60S RIBOSOMAL PROTEIN L22.//0.0023:95:35//DROSOPHILA MELANOGASTER (FRUIT FLY).//P50887
F-OVARC1002138//PROBABLE 26S PROTEASE SUBUNIT YTA6 (TAT-BINDING HOMOLOG 6).//6.4e-51:198:56//SACCHAROMYCES CEREVISIAE (BAKER'S YEAST).//P40328
F-OVARC1002143
F-OVARC1002156//HYPOTHETICAL 27.7 KD PROTEIN IN CPT1-SPC98 INTERGENIC REGION.//0.00010:64:34//SACCHAROMYCES CEREVISIAE (BAKER'S YEAST).//P53915
F-OVARC1002158//HYPOTHETICAL 24.1 KD PROTEIN IN LEF4-P33 INTERGENIC REGION.//8.2e-07:119:35//AUTOGRAPHA CALIFORNICA NUCLEAR POLYHEDROSIS VIRUS (ACMNPV).//P41479
F-OVARC1002165//EBNA-6 NUCLEAR PROTEIN (EBNA-3C) (EBNA-4B).//0.00023:90:45//EPSTEIN-BARR VIRUS (STRAIN B95-8) (HUMAN HERPESVIRUS 4).//P03204
F-OVARC1002182//HYPOTHETICAL 46.2 KD TRP-ASP REPEATS CONTAINING PROTEIN D2013.2 IN CHROMOSOME II.//1.3e-34:165:35//CAENORHABDITIS ELEGANS.//Q18964
F-PLACE1000004//HYPOTHETICAL 180.2 KD PROTEIN C31A2.05C IN CHROMOSOME I.//8.8e-05:148:25//SCHIZOSACCHAROMYCES POMBE (FISSION YEAST).//Q09725
F-PLACE1000005//PROTEIN Q300.//0.30:10:100//MUS MUSCULUS (MOUSE).//Q02722
F-PLACE1000007//PROBABLE UBIQUITIN CARBOXYL-TERMINAL HYDROLASE R10E11.3 (EC 3.1.2.15) (UBIQUITIN THIOLESTERASE) (UBIQUITIN-SPECIFIC PROCESSING PROTEASE) (DEUBIQUITINATING ENZYME).//2.3e-39:134:62//CAENORHABDITIS ELEGANS.//P34547
F-PLACE1000014//52 KD RO PROTEIN (SJOGREN SYNDROME TYPE A ANTIGEN (SS-A)) (RO(SS-A)).//0.00036:63:39//HOMO SAPIENS (HUMAN).//P19474
F-PLACE1000031
F-PLACE1000040//!!!! ALU SUBFAMILY SQ WARNING ENTRY !!!!//4.4e-12:97:41//HOMO SAPIENS (HUMAN).//P39194
F-PLACE1000048//50S RIBOSOMAL PROTEIN L15 (FRAGMENT).//0.98:31:38//BACILLUS SP. (STRAIN C-125).//P38373
F-PLACE1000050//COLLAGEN ALPHA 1(III) CHAIN.//0.00062:190:33//BOS TAURUS (BOVINE).//P04258
F-PLACE1000061//60S RIBOSOMAL PROTEIN L37A.//6.4e-19:51:86//GALLUS GALLUS (CHICKEN).//P32046
F-PLACE1000066//SSU72 PROTEIN.//2.3e-39:165:49//SACCHAROMYCES CEREVISIAE (BAKER'S YEAST).//P53538
F-PLACE1000078//BAD PROTEIN (BCL-2 BINDING COMPONENT 6).//1.7e-06:21:95//HOMO SAPIENS (HUMAN).//Q92934
F-PLACE1000081//HOMEOBOX PROTEIN HOX-A4 (HOX-1.4) (MH-3).//0.0053:146:33//MUS MUSCULUS (MOUSE).//P06798
F-PLACE1000094
F-PLACE1000133//TRANSCRIPTION FACTOR BTF3 (RNA POLYMERASE B TRANSCRIPTION FACTOR 3).//1.8e-62:158:81//HOMO SAPIENS (HUMAN).//P20290
F-PLACE1000142//ENOYL-COA HYDRATASE, MITOCHONDRIAL PRECURSOR (EC 4.2.1.17) (SHORT CHAIN ENOYL-COA HYDRATASE) (SCEH) (ENOYL-COA HYDRATASE 1).//9.8e-12:104:34//HOMO SAPIENS (HUMAN).//P30084
F-PLACE1000184//AC PROTEIN.//0.44:31:29//BACTERIOPHAGE T4.//P18924
F-PLACE1000185//HYPOTHETICAL GLYCINE-RICH 49.6 KD PROTEIN CY130.10C PRECURSOR.//0.11:48:33//MYCOBACTERIUM TUBERCULOSIS.//Q10637
F-PLACE1000213//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//3.4e-05:194:26//SACCHAROMYCES CEREVISIAE (BAKER'S YEAST).//P08640
F-PLACE1000214
F-PLACE1000236//PROCOLLAGEN ALPHA 1(I) CHAIN PRECURSOR.//0.027:63:34//GALLUS GALLUS (CHICKEN).//P02457
F-PLACE1000246//TEGUMENT PROTEIN (GENE 11 PROTEIN).//0.78:100:26//EQUINE HERPESVIRUS TYPE 4 (STRAIN 1942) (EHV-4) (EQUINE HERPESVIRUS TYPE 1 SUBTYPE 2).//Q00039
F-PLACE1000292
F-PLACE1000308//EARLY NODULIN 75 (N-75) (NGM-75) (FRAGMENT).//0.049:28:42//MEDICAGO SATIVA (ALFALFA).//P11728
F-PLACE1000332
F-PLACE1000347//HYPOTHETICAL PROTEIN TP0420.//0.15:24:54//TREPONEMA PALLIDUM.//O83435
F-PLACE1000374//LYSOZYME C (EC 3.2.1.17) (1,4-BETA-N-ACETYLMURAMIDASE C).//1.0:63:25//ORYCTOLAGUS CUNICULUS (RABBIT).//P16973
F-PLACE1000380//MATING PROCESS PROTEIN MID2 (SERINE-RICH PROTEIN SMS1) (PROTEIN KINASE A INTERFERENCE PROTEIN).//0.018:169:28//SACCHAROMYCES CEREVISIAE (BAKER'S YEAST).//P36027
F-PLACE1000383//MYOTUBULARIN.//1.2e-65:215:57//HOMO SAPIENS (HUMAN).//Q13496
F-PLACE1000401//ELASTIN PRECURSOR (TROPOELASTIN).//0.00023:145:30//MUS MUSCULUS (MOUSE).//P54320
F-PLACE1000406//54 KD NUCLEAR RNA-BINDING PROTEIN (P54(NRB)).//3.4e-27:90:63//HOMO SAPIENS (HUMAN).//Q15233
F-PLACE1000420//7,8-DIHYDRO-8-OXOGUANINE TRIPHOSPHATASE (EC 3.1.6.-) (8-OXO-DGTPASE).//4.7e-07:134:29//MUS MUSCULUS (MOUSE).//P53368
F-PLACE1000421//HYPOTHETICAL 8.8 KD PROTEIN C11D3.01C IN CHROMOSOME I.//0.48:72:27//SCHIZOSACCHAROMYCES POMBE (FISSION YEAST).//Q10080
F-PLACE1000424
F-PLACE1000435
F-PLACE1000444//!!!! ALU SUBFAMILY SX WARNING ENTRY !!!!//2.0e-31:129:63//HOMO SAPIENS (HUMAN).//P39195
F-PLACE1000453//PROTEIN Q300.//0.013:16:68//MUS MUSCULUS (MOUSE).//Q02722
F-PLACE1000481//LINE-1 REVERSE TRANSCRIPTASE HOMOLOG.//0.14:63:36//HOMO SAPIENS (HUMAN).//P08547
F-PLACE1000492//BASP1 PROTEIN.//0.17:114:28//HOMO SAPIENS (HUMAN).//P80723
F-PLACE1000540
F-PLACE1000547//MANNOSE-1-PHOSPHATE GUANYLTRANSFERASE (EC 2.7.7.13) (ATP-MANNOSE-1-PHOSPHATE GUANYLYLTRANSFERASE) (NDP-HEXOSE PYROPHOSPHORYLASE).//1.8e-21:87:56//SACCHAROMYCES CEREVISIAE (BAKER'S YEAST).//P41940
F-PLACE1000562//HYPOTHETICAL PROTEIN MJ0562.//1.0:35:34//METHANOCOCCUS JANNASCHII.//Q57982
F-PLACE1000564//ADRENAL SPECIFIC 30 KD PROTEIN (CLONE PG2).//0.13:66:37//HOMO SAPIENS (HUMAN).//P15803
F-PLACE1000583//ZINC FINGER PROTEIN 83 (ZINC FINGER PROTEIN HPF1).//7.0e-45:192:47//HOMO SAPIENS (HUMAN).//P51522
F-PLACE1000588//INTERFERON-INDUCED GUANYLATE-BINDING PROTEIN 1 (GUANINE NUCLEOTIDE- BINDING PROTEIN 1).//5.3e-63:122:88//HOMO SAPIENS (HUMAN).//P32455
F-PLACE1000596//RING CANAL PROTEIN (KELCH PROTEIN).//2.6e-12:120:38//DROSOPHILA MELANOGASTER (FRUIT FLY).//Q04652
F-PLACE1000599//EARLY E3B 12.7 KD PROTEIN PRECURSOR.//0.83:53:32//HUMAN ADENOVIRUS TYPE 12.//P36707
F-PLACE1000610
F-PLACE1000611//HYPOTHETICAL 33.6 KD PROTEIN IN MCK1-RPS19B INTERGENIC REGION.//9.4e-07:64:28//SACCHAROMYCES CEREVISIAE (BAKER'S YEAST).//P48558
F-PLACE1000636//MALE STERILITY PROTEIN 2.//3.7e-09:83:43//ARABIDOPSIS THALIANA (MOUSE-EAR CRESS).//Q08891
F-PLACE1000653//PUTATIVE PHOSPHOACETYLGLUCOSAMINE MUTASE (EC 5.4.2.3) (ACETYLGLUCOSAMINE PHOSPHOMUTASE) (N-ACETYLGLUCOSAMINE-PHOSPHATE MUTASE).//1.9e-30:203:41//SCHIZOSACCHAROMYCES POMBE (FISSION YEAST).//Q09687
F-PLACE1000656//EXTENSIN PRECURSOR (CELL WALL HYDROXYPROLINE-RICH GLYCOPROTEIN).//0.0029:75:33//NICOTIANA TABACUM (COMMON TOBACCO).//P13983
F-PLACE1000706//TRANSCRIPTION INTERMEDIARY FACTOR 1-BETA (NUCLEAR COREPRESSOR KAP-1) (KRAB-ASSOCIATED PROTEIN 1).//1.1e-38:180:42//HOMO SAPIENS (HUMAN).//Q13263
F-PLACE1000712//VERY HYPOTHETICAL 8.9 KD PROTEIN CY441.05 PRECURSOR.//0.93:49:34//MYCOBACTERIUM TUBERCULOSIS.//P71934
F-PLACE1000716
F-PLACE1000748//HYPOTHETICAL 10.4 KD PROTEIN IN SPAT 3'REGION (ORF-11).//0.90:53:37//SHIGELLA FLEXNERI.//P55794
F-PLACE1000749//HYPOTHETICAL PROTEIN MG148.//0.0014:142:27//MYCOPLASMA GENITALIUM.//P47394
F-PLACE1000755//HYPOTHETICAL HELICASE K12H4.8 IN CHROMOSOME III.//1.1e-15:98:48//CAENORHABDITIS ELEGANS.//P34529
F-PLACE1000769//VIGILIN.//0.51:60:33//GALLUS GALLUS (CHICKEN).//P81021
F-PLACE1000785//PROBABLE COLD SHOCK PROTEIN CY15C10.04.//1.0:22:45//MYCOBACTERIUM TUBERCULOSIS.//O06360
F-PLACE1000786//HYPOTHETICAL 30.2 KD PROTEIN ZK632.12 IN CHROMOSOME III.//2.6e-38:159:51//CAENORHABDITIS ELEGANS.//P34657
F-PLACE1000793//VASODILATOR-STIMULATED PHOSPHOPROTEIN (VASP).//0.0097:128:30//HOMO SAPIENS (HUMAN).//P50552
F-PLACE1000798//!!!! ALU SUBFAMILY J WARNING ENTRY !!!!//9.4e-07:47:61//HOMO SAPIENS (HUMAN).//P39188
F-PLACE1000841
F-PLACE1000849//ELAV PROTEIN.//3.5e-05:140:35//DROSOPHILA VIRILIS (FRUIT FLY).//P23241
F-PLACE1000856//HYPOTHETICAL PROTEIN MJ0008.//0.95:100:23//METHANOCOCCUS JANNASCHII.//Q60319
F-PLACE1000863//PUTATIVE MITOCHONDRIAL 40S RIBOSOMAL PROTEIN YHR148W.//2.3e-46:172:54//SACCHAROMYCES CEREVISIAE (BAKER'S YEAST).//P32899
F-PLACE1000909//ANKYRIN R (ANKYRINS 2.1 AND 2.2) (ERYTHROCYTE ANKYRIN).//0.00022:105:35//HOMO SAPIENS (HUMAN).//P16157
F-PLACE1000931//KILLER TOXIN HM-1.//0.95:24:33//WILLIOPSIS MRAKII (YEAST) (HANSENULA MRAKII).//P10410
F-PLACE1000948//SL CYTOKINE PRECURSOR (FLT3 LIGAND).//0.97:52:40//HOMO SAPIENS (HUMAN).//P49771
F-PLACE1000972//MYOSIN ID HEAVY CHAIN.//1.9e-06:79:43//DICTYOSTELIUM DISCOIDEUM (SLIME MOLD).//P34109
F-PLACE1000977//HYPOTHETICAL 94.2 KD PROTEIN C38D4.5 IN CHROMOSOME III.//2.5e-23:105:41//CAENORHABDITIS ELEGANS.//P46941
F-PLACE1000979//ZINC FINGER PROTEIN 7 (ZINC FINGER PROTEIN KOX4) (ZINC FINGER PROTEIN HF.16).//0.91:83:30//HOMO SAPIENS (HUMAN).//P17097
F-PLACE1000987//HYPOTHETICAL 111.5 KD PROTEIN C22G7.02 IN CHROMOSOME I.//0.10:128:24//SCHIZOSACCHAROMYCES POMBE (FISSION YEAST).//Q09796
F-PLACE1001000
F-PLACE1001007//ZYXIN.//2.2e-05:135:30//GALLUS GALLUS (CHICKEN).//Q04584
F-PLACE1001010//BETA-1 BUNGAROTOXIN B CHAIN, MAJOR COMPONENT PRECURSOR (BUNGAROTOXIN, B1 CHAIN).//1.0:30:40//BUNGARUS MULTICINCTUS (MANY-BANDED KRAIT).//P00987
F-PLACE1001015
F-PLACE1001024
F-PLACE1001036
F-PLACE1001054//HOLOTRICIN 3 PRECURSOR.//0.0044:56:39//HOLOTRICHIA DIOMPHALIA.//Q25055
F-PLACE1001062//SACCHAROPINE DEHYDROGENASE [NADP+, L-GLUTAMATE FORMING] (EC 1.5.1.10).//0.0013:38:52//SACCHAROMYCES CEREVISIAE (BAKER'S YEAST).//P38999
F-PLACE1001076
F-PLACE1001088//EARLY NODULIN 75 (N-75) (NGM-75) (FRAGMENT).//0.95:32:50//MEDICAGO SATIVA (ALFALFA).//P11728
F-PLACE1001092//HYPOTHETICAL 49.0 KD PROTEIN IN NSP1-KAR2 INTERGENIC REGION.//0.0026:81:35//SACCHAROMYCES CEREVISIAE (BAKER'S YEAST).//P47057
F-PLACE1001104//HYPOTHETICAL 131.5 KD PROTEIN C02F12.7 IN CHROMOSOME X.//0.00063:125:32//CAENORHABDITIS ELEGANS.//Q11102
F-PLACE1001118//ZINC FINGER PROTEIN MLZ-4 (ZINC FINGER PROTEIN 46).//2.6e-77:209:63//MUS MUSCULUS (MOUSE).//Q03309
F-PLACE1001136//ALPHA-N-ACETYLGALACTOSAMINIDASE PRECURSOR (EC 3.2.1.49) (ALPHA- GALACTOSIDASE B).//0.99:107:30//HOMO SAPIENS (HUMAN).//P17050
F-PLACE1001168
F-PLACE1001171//RETROVIRUS-RELATED POL POLYPROTEIN (FRAGMENT).//0.00012:37:59//HOMO SAPIENS (HUMAN).//P12895
F-PLACE1001185//HYPOTHETICAL 56.6 KD PROTEIN IN URE2-SSU72 INTERGENIC REGION.//3.6e-12:88:36//SACCHAROMYCES CEREVISIAE (BAKER'S YEAST).//P53867
F-PLACE1001238
F-PLACE1001241//METALLOTHIONEIN B (MTB) (FRAGMENT).//0.13:30:53//COLINUS VIRGINIANUS (BOBWHITE QUAIL) (COMMON BOBWHITE).//P27087
F-PLACE1001257//RING CANAL PROTEIN (KELCH PROTEIN).//4.1e-24:125:46//DROSOPHILA MELANOGASTER (FRUIT FLY).//Q04652
F-PLACE1001272//HYPOTHETICAL PROTEIN IN KSGA 3'REGION (ORF L5) (FRAGMENT).//1.0:24:45//MYCOPLASMA CAPRICOLUM.//P43040
F-PLACE1001279//CYTOTOXIN 3 (CYTOTOXIN V-II-3).//0.98:31:41//NAJA MOSSAMBICA (MOZAMBIQUE COBRA).//P01470
F-PLACE1001280//PROCOLLAGEN ALPHA 1(II) CHAIN PRECURSOR [CONTAINS: CHONDROCALCIN].//0.0051:156:32//MUS MUSCULUS (MOUSE).//P28481
F-PLACE1001294//GAMETOGENESIS EXPRESSED PROTEIN GEG-154.//3.7e-56:109:93//MUS MUSCULUS (MOUSE).//P50636
F-PLACE1001304//ZINC FINGER PROTEIN 35 (ZFP-35).//3.2e-30:75:57//MUS MUSCULUS (MOUSE).//P15620
F-PLACE1001311//!!!! ALU SUBFAMILY SB WARNING ENTRY !!!!//2.7e-31:66:66//HOMO SAPIENS (HUMAN).//P39189
F-PLACE1001323
F-PLACE1001351//REV PROTEIN (ANTI-REPRESSION TRANSACTIVATOR PROTEIN) (ART/TRS).//0.11:66:27//SIMIAN IMMUNODEFICIENCY VIRUS (AGM155 ISOLATE) (SIV-AGM).//P27971
F-PLACE1001366//SHORT NEUROTOXIN 2 (TOXIN CM-14) (TOXIN V-N-I2).//0.070:18:33//NAJA HAJE ANNULIFERA (BANDED EGYPTIAN COBRA).//P01422
F-PLACE1001377//DISINTEGRIN TRIGRAMIN BETA (PLATELET AGGREGATION ACTIVATION INHIBITOR).//4.9e-06:50:46//TRIMERESURUS GRAMINEUS (INDIAN GREEN TREE VIPER) (GREEN HABU SNAKE).//P17495
F-PLACE1001383//M PROTEIN, SEROTYPE 49 PRECURSOR.//0.080:136:24//STREPTOCOCCUS PYOGENES.//P16947
F-PLACE1001384
F-PLACE1001387//EPIDERMAL GROWTH FACTOR RECEPTOR KINASE SUBSTRATE EPS8.//1.9e-22:142:39//HOMO SAPIENS (HUMAN).//Q12929
F-PLACE1001395//HYPOTHETICAL 8.5 KD PROTEIN IN ASIA-MOTA INTERGENIC REGION.//0.98:67:34//BACTERIOPHAGE T4.//P22917
F-PLACE1001399//!!!! ALU SUBFAMILY SQ WARNING ENTRY !!!!//3.1e-32:47:74//HOMO SAPIENS (HUMAN).//P39194
F-PLACE1001412//GLYCOPHORIN C (PAS-2') (GLYCOPROTEIN BETA) (GLPC) (GLYCOCONNECTIN) (SIALOGLYCOPROTEIN D) (GLYCOPHORIN D) (GPD).//0.00021:125:36//HOMO SAPIENS (HUMAN).//P04921
F-PLACE1001414//CHYMOTRYPSIN/ELASTASE ISOINHIBITORS 2 TO 5.//0.99:37:35//ASCARIS SUUM (PIG ROUNDWORM) (ASCARIS LUMBRICOIDES).//P07852
F-PLACE1001440//PROLINE-RICH PEPTIDE P-B.//0.35:16:50//HOMO SAPIENS (HUMAN).//P02814
F-PLACE1001456//RELAXIN.//0.48:38:36//BALAENOPTERA ACUTOROSTRATA (MINKE WHALE) (LESSER RORQUAL).//P11184
F-PLACE1001468//HYPOTHETICAL PROTEIN MJ0602.//0.10:86:32//METHANOCOCCUS JANNASCHII.//Q58019
F-PLACE1001484//HYPOTHETICAL 7.5 KD PROTEIN IN DNAC-RPLI INTERGENIC REGION.//1.0:47:34//BACILLUS SUBTILIS.//P37480
F-PLACE1001502//COLLAGEN 1(X) CHAIN PRECURSOR.//0.00029:118:34//BOS TAURUS (BOVINE).//P23206
F-PLACE1001503//HYPOTHETICAL 77.3 KD PROTEIN T05G5.8 IN CHROMOSOME III.//2.2e-07:107:30//CAENORHABDITIS ELEGANS.//P34561
F-PLACE1001517//SMALL PROTEIN INHIBITOR OF INSECT ALPHA-AMYLASES 2 (SI ALPHA-2).//0.56:22:45//SORGHUM BICOLOR MILO (SORGHUM).//P21924
F-PLACE1001534//PUTATIVE GENE PROTEIN 54.//0.43:44:40//BACTERIOPHAGE SP01.//O48408
F-PLACE1001545//HYPOTHETICAL 7.9 KD PROTEIN IN CELF-KATE INTERGENIC REGION.//0.99:70:32//ESCHERICHIA COLI.//P37795
F-PLACE1001551//CHLOROPLAST 50S RIBOSOMAL PROTEIN L32.//1.0:66:28//MARCHANTIA POLYMORPHA (LIVERWORT).//P12196
F-PLACE1001570//SYNAPTONEMAL COMPLEX PROTEIN 1 (SCP-1 PROTEIN).//0.024:120:27//HOMO SAPIENS (HUMAN).//Q15431
F-PLACE1001602//CCR4-ASSOCIATED FACTOR 1 (CAF1).//1.1e-30:90:78//MUS MUSCULUS (MOUSE).//Q60809
F-PLACE1001603//ACIDIC PROLINE-RICH PROTEIN PRECURSOR (CLONE PRP25) (FRAGMENT).//0.054:77:33//RATTUS NORVEGICUS (RAT).//P10164
F-PLACE1001608
F-PLACE1001610//PROBABLE E4 PROTEIN.//0.90:58:29//HUMAN PAPILLOMAVIRUS TYPE 28.//P51896
F-PLACE1001611//METALLOTHIONEIN-IG (MT-1G).//0.35:30:40//HOMO SAPIENS (HUMAN).//P13640
F-PLACE1001632//ZINC FINGER PROTEIN 84 (ZINC FINGER PROTEIN HPF2).//3.6e-28:144:43//HOMO SAPIENS (HUMAN).//P51523
F-PLACE1001634//PHOTOSYSTEM II REACTION CENTRE N PROTEIN.//1.0:36:41//CYANIDIUM CALDARIUM (GALDIERIA SULPHURARIA).//O19926
F-PLACE1001640//TAT PROTEIN (TRANSACTIVATING REGULATORY PROTEIN).//0.24:47:38//HUMAN IMMUNODEFICIENCY VIRUS TYPE 1 (NDK ISOLATE) (HIV-1).//P18804
F-PLACE1001672//!!!! ALU SUBFAMILY J WARNING ENTRY !!!!//1.0:27:66//HOMO SAPIENS (HUMAN).//P39188
F-PLACE1001691//HYPOTHETICAL 15.5 KD PROTEIN IN PIK1-POL2 INTERGENIC REGION.//0.40:81:33//SACCHAROMYCES CEREVISIAE (BAKER'S YEAST).//P53842
F-PLACE1001692//S-ACYL FATTY ACID SYNTHASE THIOESTERASE, MEDIUM CHAIN (EC 3.1.2.14) (THIOESTERASE II).//8.3e-41:103:55//RATTUS NORVEGICUS (RAT).//P08635
F-PLACE1001705
F-PLACE1001716//HYPOTHETICAL 138.5 KD PROTEIN C17H9.01 IN CHROMOSOME L//6.1e-07:157:29//SCHIZOSACCHAROMYCES POMBE (FISSION YEAST).//O13798
F-PLACE1001720
F-PLACE1001729//PROLINE-RICH PROTEIN MP-3 (FRAGMENT).//6.5e-05:196:32//MUS MUSCULUS (MOUSE).//P05143
F-PLACE1001739//NEUROFILAMENT TRIPLET M PROTEIN (160 KD NEUROFILAMENT PROTEIN) (NF-M).//0.00050:213:23//RATTUS NORVEGICUS (RAT).//P12839
F-PLACE1001740//!!!! ALU SUBFAMILY J WARNING ENTRY !!!!//2.4e-17:90:56//HOMO SAPIENS (HUMAN).//P39188
F-PLACE1001745//HYPOTHETICAL PROTEIN KIAA0125.//0.96:38:36//HOMO SAPIENS (HUMAN).//Q14138
F-PLACE1001746//CONGLUTIN DELTA-2 SMALL CHAIN.//0.98:23:43//LUPINUS ANGUSTIFOLIUS (NARROW-LEAVED BLUE LUPINE).//P09930
F-PLACE1001748//HYPOTHETICAL 99.0 KD PROTEIN SPBC119.17.//2.9e-28:167:38//SCHIZOSACCHAROMYCES POMBE (FISSION YEAST).//O42908
F-PLACE1001756//!!!! ALU SUBFAMILY SB WARNING ENTRY !!!!//9.2e-43:126:77//HOMO SAPIENS (HUMAN).//P39189
F-PLACE1001761//50S RIBOSOMAL PROTEIN L35.//0.26:42:38//HELICOBACTER PYLORI (CAMPYLOBACTER PYLORI).//P56057
F-PLACE1001771//TRANSIENT-RECEPTOR-POTENTIAL LIKE PROTEIN.//4.8e-35:223:40//DROSOPHILA MELANOGASTER (FRUIT FLY).//P48994
F-PLACE1001781//HYPOTHETICAL 71.1 KD PROTEIN IN DSK2-CAT8 INTERGENIC REGION.//9.5e-41:194:46//SACCHAROMYCES CEREVISIAE (BAKER'S YEAST).//Q03262
F-PLACE1001799
F-PLACE1001810
F-PLACE1001817//SUCCINYL-COA LIGASE [GDP-FORMING], BETA-CHAIN PRECURSOR (EC 6.2.1.4) (SUCCINYL-COA SYNTHETASE, BETA CHAIN) (SCS-BETA).//2.8e-40:115:61//NEOCALLIMASTIX FRONTALIS (RUMEN FUNGUS).//P53587
F-PLACE1001821
F-PLACE1001844//IG KAPPA CHAIN V-I REGION (HAU).//0.59:89:35//HOMO SAPIENS (HUMAN).//P01600
F-PLACE1001845
F-PLACE1001869//MPA43 PROTEIN.//3.5e-14:153:33//SACCHAROMYCES CEREVISIAE (BAKER'S YEAST).//P53583
F-PLACE1001897//LIGATOXIN A.//1.0:43:27//PHORADENDRON LIGA (ARGENTINE MISTLETOE).//P01540
F-PLACE1001912//LONG NEUROTOXIN 2 (TOXIN C).//0.57:44:45//ASTROTIA STOKESI (STOKES'S SEA SNAKE) (DISTEIRA STOKESI).//P01381
F-PLACE1001920//LATE GENES ACTIVATOR (EARLY PROTEIN GP4) (GPF).//0.89:75:29/BACTERIOPHAGE NF.//P09877
F-PLACE1001928
F-PLACE1001983//IMMEDIATE-EARLY PROTEIN IE180.//0.0049:51:45//PSEUDORABIES VIRUS (STRAIN KAPLAN) (PRV).//P33479
F-PLACE1001989//PUTATIVE AMIDASE (EC 3.5.1.4).//8.9e-08:125:36//MORAXELLA CATARRHALIS.//Q49091
F-PLACE1002004
F-PLACE1002046//LIGATIN (FRAGMENT).//1.6e-84:191:84//MUS MUSCULUS (MOUSE).//Q61211
F-PLACE1002052
F-PLACE1002066
F-PLACE1002072//ANTER-SPECIFIC PROLINE-RICH PROTEIN APG PRECURSOR.//0.16:77:31//ARABIDOPSIS THALIANA (MOUSE-EAR CRESS).//P40602
F-PLACE1002073//HYPOTHETICAL 118.2 KD PROTEIN F43C1.1 IN CHROMOSOME III.//4.0e-11:174:28//CAENORHABDITIS ELEGANS.//Q09564
F-PLACE1002090//SIGNAL RECOGNITION PARTICLE 72 KD PROTEIN (SRP72).//2.8e-57:112:99//HOMO SAPIENS (HUMAN).//O76094
F-PLACE1002115//P8 MTCP-1 PROTEIN (MATURE T-CELL PROLIFERATION-1 TYPE A) (MTCP-1 TYPE A) (P8MTCP1).//1.0:49:30//MUS MUSCULUS (MOUSE).//Q61908
F-PLACE1002119//T-LYMPHOCYTE ACTIVATED PROTEIN (CYCLOHEXIMIDE-INDUCED) (CHX1) (IMMEDIATE EARLY RESPONSE 2 PROTEIN).//2.7e-11:118:36//MUS MUSCULUS (MOUSE).//P17950
F-PLACE1002140//HYPOTHETICAL 12.3 KD PROTEIN IN MOBL 3'REGION (ORF 4).//0.0086:39:46//THIOBACILLUS FERROOXIDANS.//P20088
F-PLACE1002150
F-PLACE1002157//!!!! ALU SUBFAMILY SB WARNING ENTRY !!!!//2.4e-34:56:82//HOMO SAPIENS (HUMAN).//P39189
F-PLACE1002163//NEUROTOXIN 1.//1.0:17:52//CENTRUROIDES SCULPTURATUS (BARK SCORPION).//P01492
F-PLACE1002170
F-PLACE1002171//TRANSCRIPTION REGULATORY PROTEIN SWI3 (SWI/SNF COMPLEX COMPONENT SWI3) (TRANSCRIPTION FACTOR
TYE2).//0.00023:179:23//SACCHAROMYCES CEREVISIAE (BAKER'S YEAST).//P32591
F-PLACE1002205//HYPOTHETICAL 13.5 KD PROTEIN IN MOB1-SGA1 INTERGENIC REGION.//0.77:21:47//SACCHAROMYCES CEREVISIAE (BAKER'S YEAST).//P40490
F-PLACE1002213//HISTONE H4 (FRAGMENT).//0.62:31:32//BLEPHARISMA JAPONICUM.//P80738
F-PLACE1002227//HYPOTHETICAL 7.9 KD PROTEIN IN FIXW 5'REGION.//0.41:49:36//RHIZOBIUM LEGUMINOSARUM.//P14310
F-PLACE1002256//CYTOCHROME B (EC 1.10.2.2).//0.61:95:29//CAENORHABDITIS ELEGANS.//P24890
F-PLACE1002259//HYPOTHETICAL 9.2 KD PROTEIN IN SPS1-QCR7 INTERGENIC REGION.//0.99:22:45//SACCHAROMYCES CEREVISIAE (BAKER'S YEAST).//P56508
F-PLACE1002319//HYPOTHETICAL 56.6 KD PROTEIN IN URE2-SSU72 INTERGENIC REGION.//0.91:18:72//SACCHAROMYCES CEREVISIAE (BAKER'S YEAST).//P53867
F-PLACE1002342//HYPOTHETICAL PROTEIN C16.//1.0:53:32//SWINEPOX VIRUS (STRAIN KASZA) (SPV).//P32219
F-PLACE1002395//CIRCUMSPOROZOITE PROTEIN PRECURSOR (CS).//6.4e-05:127:37//PLASMODIUM VIVAX.//P08677
F-PLACE1002399
F-PLACE1002433//DYNACTIN, 150 KD ISOFORM (150 KD DYNEIN-ASSOCIATED POLYPEPTIDE) (DP-150) (DAP-150) (P150-GLUED).//0.00094:182:25//RATTUS NORVEGICUS (RAT).//P28023
F-PLACE1002437//ATP-BINDING CASSETTE TRANSPORTER 1.//4.5e-19:62:77//MUS MUSCULUS (MOUSE).//P41233
F-PLACE1002438//HYPOTHETICAL 141.5 KD ZINC FINGER PROTEIN IN TUB1-CPR3 INTERGENIC REGION.//0.014:63:34//SACCHAROMYCES CEREVISIAE (BAKER'S YEAST).//Q04545
F-PLACE1002450//OOCYTE ZINC FINGER PROTEIN XLCOF6 (FRAGMENT).//3.9e-28:159:38//XENOPUS LAEVIS (AFRICAN CLAWED FROG).//P18749
F-PLACE1002465//LARIAT DEBRANCHING ENZYME (EC 3.1.-.-).//0.0014:148:28//SCHIZOSACCHAROMYCES POMBE (FISSION YEAST).//O13765
F-PLACE1002474//FIBRILLIN 2 PRECURSOR.//2.1e-24:203:33//MUS MUSCULUS (MOUSE).//Q61555
F-PLACE1002477//!!!! ALU SUBFAMILY SP WARNING ENTRY !!!!//0.15:65:41//HOMO SAPIENS (HUMAN).//P39193
F-PLACE1002493//SEMENOGELIN II PRECURSOR (SGII).//1.0:72:31//MACACA MULATTA (RHESUS MACAQUE).//Q95196
F-PLACE1002499//HYPOTHETICAL 39.3 KD PROTEIN C02B8.6 IN CHROMOSOME X.//2.9e-11:67:35//CAENORHABDITIS ELEGANS.//Q11096
F-PLACE1002500//COBALT-ZINC-CADMIUM RESISTANCE PROTEIN CZCD (CATION EFFLUX SYSTEM PROTEIN CZCD).//8.4e-11:143:32//ALCALIGENES EUTROPHUS.//P13512
F-PLACE1002514//HYPOTHETICAL 8.1 KD PROTEIN IN SPEA-METK INTERGENIC REGION (O71).//1.0:15:60//ESCHERICHIA COLI.//P46878
F-PLACE1002529
F-PLACE1002532//HOMEOBOX PROTEIN DLX-5.//1.1e-76:183:81//MUS MUSCULUS (MOUSE).//P70396
F-PLACE1002537//!!!! ALU SUBFAMILY SX WARNING ENTRY !!!!//2.6e-18:51:86//HOMO SAPIENS (HUMAN).//P39195
F-PLACE1002571//ACTIN-LIKE PROTEIN 13E.//6.0e-56:140:47//DROSOPHILA MELANOGASTER (FRUIT FLY).//P45890
F-PLACE1002578
F-PLACE1002583
F-PLACE1002591//CORONIN-LIKE PROTEIN P57.//5.5e-26:78:69//BOS TAURUS (BOVINE).//Q92176
F-PLACE1002598
F-PLACE1002604
F-PLACE1002625//HYPOTHETICAL 180.2 KD PROTEIN IN FAA4-HOR7 INTERGENIC REGION.//6.4e-08:193:23//SACCHAROMYCES CEREVISIAE (BAKER'S YEAST).//Q04781
F-PLACE1002655//ADSEVERIN (GELSOLIN-LIKE PROTEIN).//7.1e-100:210:89//MUS MUSCULUS (MOUSE).//Q60604
F-PLACE1002665//MOBILIZATION PROTEIN MOBS.//0.35:60:30//THIOBACILLUS FERROOXIDANS.//P20086
F-PLACE1002685//ACTIN BINDING PROTEIN.//0.052:115:29//SACCHAROMYCES EXIGUUS (YEAST).//P38479
F-PLACE1002714//CIS-GOLGI MATRIX PROTEIN GM130.//1.8e-06:214:30//RATTUS NORVEGICUS (RAT).//Q62839
F-PLACE1002722//THROMBIN RECEPTOR PRECURSOR.//2.0e-19:134:38//XENOPUS LAEVIS (AFRICAN CLAWED FROG).//P47749
F-PLACE1002768//FOLLICLE STIMULATING HORMONE RECEPTOR PRECURSOR (FSH-R) (FOLLITROPIN RECEPTOR) (FRAGMENT).//0.43:40:35//MUS MUSCULUS (MOUSE).//P35378
F-PLACE1002772
F-PLACE1002775//CENTROMERE/MICROTUBULE BINDING PROTEIN CBF5 (CENTROMERE-BINDING FACTOR 5) (NUCLEOLAR PROTEIN CBF5).//4.8e-07:96:29//SCHIZOSACCHAROMYCES POMBE (FISSION YEAST).//O14007
F-PLACE1002782//COBALT-ZINC-CADMIUM RESISTANCE PROTEIN CZCD (CATION EFFLUX SYSTEM PROTEIN CZCD).//1.1e-07:114:35//ALCALIGENES EUTROPHUS.//P13512
F-PLACE1002794//CUTICLE COLLAGEN 12 PRECURSOR.//0.0068:98:39//CAENORHABDITIS ELEGANS.//P20630
F-PLACE1002811//CYCLIN-DEPENDENT KINASE 6 INHIBITOR (P18-INK6) (CYCLIN-DEPENDENT KINASE 4 INHIBITOR C) (P18-INK4C).//1.1e-09:137:34//MUS MUSCULUS (MOUSE).//Q60772
F-PLACE1002815//C-HORDEIN (CLONE PC HOR1-3) (FRAGMENT).//0.46:35:42//HORDEUM VULGARE (BARLEY).//P17991
F-PLACE1002816//HYPOTHETICAL PROTEIN KIAA0288 (HA6116).//1.0e-86:201:74//HOMO SAPIENS (HUMAN).//P56524
F-PLACE1002834//ZINC FINGER PROTEIN 83 (ZINC FINGER PROTEIN HPF1).//1.6e-30:54:96//HOMO SAPIENS (HUMAN).//P51522
F-PLACE1002839//METALLOTHIONEIN-I (MT-I).//1.0:43:37//MUS MUSCULUS (MOUSE).//P02802
F-PLACE1002851//BOWMAN-BIRK TYPE PROTEINASE INHIBITOR (VAI).//0.77:35:37//VICIA ANGUSTIFOLIA (COMMON VETCH).//P01065
F-PLACE1002853//HYPOTHETICAL 7.9 KD PROTEIN IN PE 5'REGION (ORF1).//1.0:18:55//LYMANTRIA DISPAR MULTICAPSID NUCLEAR POLYHEDROSIS VIRUS (LDMNPV).//P36866
F-PLACE1002881//!!!! ALU SUBFAMILY J WARNING ENTRY !!!!//4.1e-27:91:70//HOMO SAPIENS (HUMAN).//P39188
F-PLACE1002908//HYPOTHETICAL 33.8 KD PROTEIN R10E11.4 IN CHROMOSOME III.//2.0e-31:148:46//CAENORHABDITIS ELEGANS.//P34548
F-PLACE1002941//!!!! ALU SUBFAMILY SX WARNING ENTRY !!!!//2.6e-11:40:85//HOMO SAPIENS (HUMAN).//P39195
F-PLACE1002962//ENDOTHELIN-1 PRECURSOR (ET-1) (FRAGMENT).//0.90:38:36//CANIS FAMILIARIS (DOG).//P13206
F-PLACE1002968//TOXIN IV-5 PRECURSOR (TITYUSTOXIN) (FRAGMENT).//0.97:26:38//TITYUS SERRULATUS (BRAZILIAN SCORPION).//P01496
F-PLACE1002991//PUTATIVE AMIDASE (EC 3.5.1.4).//3.3e-20:120:41//METHANOCOCCUS JANNASCHII.//Q58560
F-PLACE1002993//HYPOTHETICAL 17.8 KD PROTEIN IN SMPA-SMPB INTERGENIC REGION (F158).//0.00045:93:23//ESCHERICHIA COLI.//P52121
F-PLACE1002996//PUTATIVE REGULATORY PROTEIN TSC-22 (TGFB STIMULATED CLONE 22 HOMOLOG).//0.17:91:29//GALLUS GALLUS (CHICKEN).//Q91012
F-PLACE1003025//SUPPRESSOR PROTEIN SRP40.//0.0079:214:24//SACCHAROMYCES CEREVISIAE (BAKER'S YEAST).//P32583
F-PLACE1003027//HYPOTHETICAL 128.6 KD PROTEIN ZK1098.10 IN CHROMOSOME III.//1.3e-49:167:63//CAENORHABDITIS ELEGANS.//P34609
F-PLACE1003044//SPORE COAT PROTEIN D.//0.97:24:45//BACILLUS SUBTILIS.//P07791
F-PLACE1003045
F-PLACE1003092
F-PLACE1003100//HEP27 PROTEIN (PROTEIN D).//3.9e-51:188:57//HOMO SAPIENS (HUMAN).//Q13268
F-PLACE1003108
F-PLACE1003136
F-PLACE1003145//BUTYROPHILIN PRECURSOR (BT).//0.00024:170:24//BOS TAURUS (BOVINE).//P18892
F-PLACE1003153//HUNCHBACK PROTEIN (FRAGMENT).//1.0:32:37//LOCUSTA MIGRATORIA (MIGRATORY LOCUST).//Q01777
F-PLACE1003174//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//6.3e-05:54:38//ARABIDOPSIS THALIANA (MOUSE-EAR CRESS).//P42743
F-PLACE1003176//HYPOTHETICAL 62.3 KD PROTEIN IN PCS60-ABD1 INTERGENIC REGION.//0.24:74:36//SACCHAROMYCES CEREVISIAE (BAKER'S YEAST).//P38319
F-PLACE1003190//SOF1 PROTEIN.//1.0e-52:158:41//SACCHAROMYCES CEREVISIAE (BAKER'S YEAST).//P33750
F-PLACE1003200
F-PLACE1003205//SPERM PROTAMINE P1.//0.074:20:45//CAENOLESTES FULIGINOSUS.//P42131
F-PLACE1003238//PROBABLE G PROTEIN-COUPLED RECEPTOR KIAA0001.//0.013:20:55//HOMO SAPIENS (HUMAN).//Q15391
F-PLACE1003249//HYPOTHETICAL PROTEIN KIAA0125.//0.98:48:37//HOMO SAPIENS (HUMAN).//Q14138
F-PLACE1003256//OMEGA-CONOTOXINS GVIA, GVIB AND GVIC PRECURSOR (SHAKER PEPTIDE).//0.84:53:30//CONUS GEOGRAPHUS (GEOGRAPHY CONE).//P01522
F-PLACE1003258//EARLY EMBRYOGENESIS ZYG-11 PROTEIN.//4.1e-18:70:47//CAENORHABDITIS ELEGANS.//P21541
F-PLACE1003296//SPECTRIN BETA CHAIN, ERYTHROCYTE.//0.063:160:24//HOMO SAPIENS (HUMAN).//P11277
F-PLACE1003302//ZINC FINGER PROTEIN 83 (ZINC FINGER PROTEIN HPF1).//9.4e-69:84:94//HOMO SAPIENS (HUMAN).//P51522
F-PLACE1003334//NUCLEOBINDIN PRECURSOR (NUCB1) (BONE 63 KD CALCIUM-BINDING PROTEIN).//0.029:125:24//RATTUS NORVEGICUS (RAT).//Q63083
F-PLACE1003342//MALE SPECIFIC SPERM PROTEIN MST84DB.//0.97:44:40//DROSOPHILA MELANOGASTER (FRUIT FLY).//Q01643
F-PLACE1003343//GENE 11 PROTEIN.//1.0:37:37//SPIROPLASMA VIRUS SPV1-R8A2 B.//P15902
F-PLACE1003353//SH2/SH3 ADAPTOR CRK (ADAPTER MOLECULE CRK) (CRK2).//6.4e-05:69:40//XENOPUS LAEVIS (AFRICAN CLAWED FROG).//P87378
F-PLACE1003361//!!!! ALU SUBFAMILY SC WARNING ENTRY !!!!//1.6e-23:66:75//HOMO SAPIENS (HUMAN).//P39192
F-PLACE1003366//SMALL PROLINE-RICH PROTEIN 2-1.//0.62:19:57//HOMO SAPIENS (HUMAN).//P35326
F-PLACE1003369//A-AGGLUTININ ATTACHMENT SUBUNIT PRECURSOR.//4.3e-06:102:42//SACCHAROMYCES CEREVISIAE (BAKER'S YEAST).//P32323
F-PLACE1003373//PROTEIN Q300.//0.042:29:37//MUS MUSCULUS (MOUSE).//Q02722
F-PLACE1003375//OLFACTORY RECEPTOR 11 (M49) (FRAGMENT).//0.99:46:34//MUS MUSCULUS (MOUSE).//Q60890
F-PLACE1003383
F-PLACE1003394//RAS-RELATED PROTEIN RAB-14.//2.8e-80:166:89//RATTUS NORVEGICUS (RAT).//P35287
F-PLACE1003401
F-PLACE1003420//PUTATIVE MITOCHONDRIAL CARRIER YIL006W.//8.1e-17:138:37//SACCHAROMYCES CEREVISIAE (BAKER'S YEAST).//P40556
F-PLACE1003454
F-PLACE1003478
F-PLACE1003493//ENDOTHELIAL CELL MULTIMERIN PRECURSOR.//3.4e-11:123:32//HOMO SAPIENS (HUMAN).//Q13201
F-PLACE1003516//LINE-1 REVERSE TRANSCRIPTASE HOMOLOG.//1.0e-32:68:76//HOMO SAPIENS (HUMAN).//P08547
F-PLACE1003519//!!!! ALU SUBFAMILY J WARNING ENTRY !!!!//9.2e-17:77:50//HOMO SAPIENS (HUMAN).//P39188
F-PLACE1003521//HYPOTHETICAL BAMHI-ORF9 PROTEIN.//1.0:38:42//FOWLPOX VIRUS (ISOLATE HP-438[MUNICH]).//P14366
F-PLACE1003528//ATP SYNTHASE PROTEIN 8 (EC 3.6.1.34) (A6L).//0.96:32:40//XENOPUS LAEVIS (AFRICAN CLAWED FROG).//P03931
F-PLACE1003537//CEF PROTEIN.//0.92:47:29//BACTERIOPHAGE T4.//Q01436
F-PLACE1003553
F-PLACE1003566//HYPOTHETICAL BAMHI-ORF9 PROTEIN.//1.0:32:34//FOWLPOX VIRUS (ISOLATE HP-438[MUNICH]).//P14366
F-PLACE1003575
F-PLACE1003583//PROBABLE E5 PROTEIN.//0.16:64:31//HUMAN PAPILLOMAVIRUS TYPE 35.//P27226
F-PLACE1003584
F-PLACE1003592//EXCISIONASE.//0.26:19:52//BACTERIOPHAGE PHI-80.//P05998
F-PLACE1003593//ATP SYNTHASE PROTEIN 8 (EC 3.6.1.34) (A6L).//1.0:42:30//OVIS ARIES (SHEEP).//O78751
F-PLACE1003596//OLIGOSACCHARYL TRANSFERASE STT3 SUBUNIT HOMOLOG.//6.3e-87:238:67//CAENORHABDITIS ELEGANS.//P46975
F-PLACE1003602//HYPOTHETICAL 11.0 KD PROTEIN IN FAA3-MAS3 INTERGENIC REGION.//8.4e-17:98:42//SACCHAROMYCES CEREVISIAE (BAKER'S YEAST).//P40554
F-PLACE1003605//HAP5 TRANSCRIPTIONAL ACTIVATOR.//2.0e-09:82:35//SACCHAROMYCES CEREVISIAE (BAKER'S YEAST).//Q02516
F-PLACE1003611//PANCREATIC SECRETORY TRYPSIN INHIBITOR.//0.99:32:43//CANIS FAMILIARIS (DOG).//P04542
F-PLACE1003618//LINE-1 REVERSE TRANSCRIPTASE HOMOLOG.//4.7e-65:229:58//HOMO SAPIENS (HUMAN).//P08547
F-PLACE1003625//30S RIBOSOMAL PROTEIN S20 (FRAGMENT).//1.0:56:26//PROTEUS MIRABILIS.//P42275
F-PLACE1003638//PROTEIN Q300.//0.079:41:39//MUS MUSCULUS (MOUSE).//Q02722
F-PLACE1003669//TRICHOHYALIN.//2.9e-07:180:30//OVIS ARIES (SHEEP).//P22793
F-PLACE1003704//SPLICING FACTOR, ARGININE/SERINE-RICH 4 (PRE-MRNA SPLICING FACTOR SRP75).//3.3e-16:98:40//HOMO SAPIENS (HUMAN).//Q08170
F-PLACE1003709//HYPOTHETICAL 59.5 KD PROTEIN IN CCT3-CCT8 INTERGENIC REGION.//2.8e-07:128:27//SACCHAROMYCES CEREVISIAE (BAKER'S YEAST).//P47074
F-PLACE1003711//ALPHA/BETA-GLIADIN PRECURSOR (PROLAMIN) (CLASS A-IV).//5.0e-05:88:30//TRITICUM AESTIVUM (WHEAT).//P04724
F-PLACE1003723//TYROSINE-PROTEIN KINASE SRM (EC 2.7.1.112) (PTK70).//6.0e-06:98:36//MUS MUSCULUS (MOUSE).//Q62270
F-PLACE1003738//OOCYTE ZINC FINGER PROTEIN XLCOF6 (FRAGMENT).//2.5e-45:147:46//XENOPUS LAEVIS (AFRICAN CLAWED FROG).//P18749
F-PLACE1003760//CYTOCHROME B (EC 1.10.2.2).//0.91:49:34//TRYPANOSOMA BRUCEI BRUCEI.//P00164
F-PLACE1003762//METALLOTHIONEIN-LIKE PROTEIN TYPE 2.//0.98:28:32//MALUS DOMESTICA (APPLE) (MALUS SYLVESTRIS).//O24058
F-PLACE1003768//LINE-1 REVERSE TRANSCRIPTASE HOMOLOG.//8.5e-19:123:37//HOMO SAPIENS (HUMAN).//P08547
F-PLACE1003771
F-PLACE1003783//SRY-RELATED PROTEIN ADW2 (FRAGMENT).//1.0:29:37//ALLIGATOR MISSISSIPPIENSIS (AMERICAN ALLIGATOR).//P40634
F-PLACE1003784//HYPOTHETICAL 98.1 KD PROTEIN IN SPX19-GCR2 INTERGENIC REGION.//1.2e-13:199:28//SACCHAROMYCES CEREVISIAE (BAKER'S YEAST).//P40164
F-PLACE1003795//EC PROTEIN I/II (ZINC-METALLOTHIONEIN CLASS II).//0.67:53:30//TRITICUM AESTIVUM (WHEAT).//P30569
F-PLACE1003833//METHIONYL-TRNA FORMYLTRANSFERASE (EC 2.1.2.9).//0.99:158:28//THERMUS AQUATICUS (SUBSP. THERMOPHILUS).//P43523
F-PLACE1003850
F-PLACE1003858//HUNCHBACK PROTEIN (FRAGMENT).//0.37:28:42//LITHOBIUS FORFICATUS.//Q02030
F-PLACE1003864//OUTER MEMBRANE LIPOPROTEIN LOLB PRECURSOR.//0.0046:116:31//ACTINOBACILLUS ACTINOMYCETEMCOMITANS (HAEMOPHILUS ACTINOMYCETEMCOMITANS).//O52727
F-PLACE1003870
F-PLACE1003885//POLY(A) POLYMERASE (EC 2.7.7.19) (PAP) (POLYNUCLEOTIDE ADENYLYLTRANSFERASE) (FRAGMENT).//1.6e-92:166:75//HOMO SAPIENS (HUMAN).//P51003
F-PLACE1003886//IMMEDIATE-EARLY PROTEIN IE180.//0.54:96:34//PSEUDORABIES VIRUS (STRAIN INDIANA-FUNKHAUSER / BECKER) (PRV).//P11675
F-PLACE1003888//1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III) (FRAGMENT).//8.8e-54:260:46//BOS TAURUS (BOVINE).//P10895
F-PLACE1003892//PROBABLE E5 PROTEIN.//1.0:13:61//HUMAN PAPILLOMAVIRUS TYPE 18.//P06792
F-PLACE1003900//BETA-FRUCTOFURANOSIDASE, SOLUBLE ISOENZYME I (EC 3.2.1.26) (SUCROSE-6- PHOSPHATE HYDROLASE) (INVERTASE) (FRAGMENTS).//0.58:49:36//DAUCUS CAROTA (CARROT).//P80065
F-PLACE1003903//CTP SYNTHASE (EC 6.3.4.2) (UTP--AMMONIA LIGASE) (CTP SYNTHETASE).//3.8e-52:92:85//HOMO SAPIENS (HUMAN).//P17812
F-PLACE1003915//PROBABLE ARGINYL-TRNA SYNTHETASE, CYTOPLASMIC (EC 6.1.1.19) (ARGININE- -TRNA LIGASE) (ARGRS).//2.6e-26:202:36//SACCHAROMYCES CEREVISIAE (BAKER'S YEAST).//Q05506
F-PLACE1003923//HISTIDYL-TRNA SYNTHETASE (EC 6.1.1.21) (HISTIDINE--TRNA LIGASE) (HISRS).//0.94:65:29//STREPTOCOCCUS EQUISIMILIS.//P30053
F-PLACE1003932//HYPOTHETICAL 17.3 KD PROTEIN IN SEC15-SAP4 INTERGENIC REGION.//0.098:79:31//SACCHAROMYCES CEREVISIAE (BAKER'S YEAST).//P53074
F-PLACE1003936
F-PLACE1003968//5'-AMP-ACTIVATED PROTEIN KINASE, GAMMA-1 SUBUNIT (AMPK GAMMA-1 CHAIN).//4.7e-68:164:78//RATTUS NORVEGICUS (RAT).//P80385
F-PLACE1004103//!!!! ALU SUBFAMILY SC WARNING ENTRY !!!!//1.9e-14:60:73//HOMO SAPIENS (HUMAN).//P39192
F-PLACE1004104//EXOCYST COMPLEX COMPONENT SEC5.//0.020:202:20//SACCHAROMYCES CEREVISIAE (BAKER'S YEAST).//P89102
F-PLACE1004114//!!!! ALU SUBFAMILY J WARNING ENTRY !!!!//2.1e-15:69:60//HOMO SAPIENS (HUMAN).//P39188
F-PLACE1004118//REGULATORY PROTEIN E2.//0.73:58:36//CANINE ORAL PAPILLOMAVIRUS (COPV).//Q89420
F-PLACE1004128//GUANINE NUCLEOTIDE-BINDING PROTEIN BETA SUBUNIT 4 (TRANSDUCIN BETA CHAIN 4).//7.7e-62:108:100//MUS MUSCULUS (MOUSE).//P29387
F-PLACE1004149//PROBABLE NUCLEAR ANTIGEN.//0.0011:73:42//PSEUDORABIES VIRUS (STRAIN KAPLAN) (PRV).//P33485
F-PLACE1004156//HYPOTHETICAL PROLINE-RICH PROTEIN (FRAGMENT).//0.00061:39:48//OWENIA FUSIFORMIS.//P21260
F-PLACE1004161//PLASMINOGEN-BINDING PROTEIN PAM PRECURSOR (FRAGMENT).//0.033:108:27//STREPTOCOCCUS PYOGENES.//P49054
F-PLACE1004183//HYPOTHETICAL 64.3 KD PROTEIN IN CDC12-ERP5 INTERGENIC REGION.//4.0e-07:146:35//SACCHAROMYCES CEREVISIAE (BAKER'S YEAST).//P38817
F-PLACE1004197//BUTYROPHILIN PRECURSOR (BT).//5.9e-11:208:27//MUS MUSCULUS (MOUSE).//Q62556
F-PLACE1004203//PROTEIN A39.//8.5e-18:139:33//VACCINIA VIRUS (STRAIN COPENHAGEN).//P21062
F-PLACE1004242//PHOTOSYSTEM II REACTION CENTRE J PROTEIN.//1.0:28:42//PISUM SATIVUM (GARDEN PEA).//P13555
F-PLACE1004256//MYOSIN HEAVY CHAIN D (MHC D).//0.73:134:25//CAENORHABDITIS ELEGANS.//P02567
F-PLACE1004257//HYPOTHETICAL PROTEIN HI0490.//0.13:75:29//HAEMOPHILUS INFLUENZAE.//P44006
F-PLACE1004258//COLLAGEN ALPHA 2(VIII) CHAIN (ENDOTHELIAL COLLAGEN) (FRAGMENT).//0.027:128:35//HOMO SAPIENS (HUMAN).//P25067
F-PLACE1004270//LARGE TEGUMENT PROTEIN.//1.8e-10:100:44//EPSTEIN-BARR VIRUS (STRAIN B95-8) (HUMAN HERPESVIRUS 4).//P03186
F-PLACE1004274//HYPOTHETICAL PROTEIN E-95.//0.44:61:42//HUMAN ADENOVIRUS TYPE 2.//P03286
F-PLACE1004277//COLLAGEN ALPHA 1(XII) CHAIN (FRAGMENTS).//0.0013:55:38//BOS TAURUS (BOVINE).//P25508
F-PLACE1004284//7 KD PROTEIN (ORF 4).//1.0:63:23//CHRYSANTHEMUM VIRUS B (CVB).//P37990
F-PLACE1004289//SPERM PROTAMINE P3.//0.00057:22:77//MUS MUSCULUS (MOUSE).//Q62100
F-PLACE1004302//SERINE/THREONINE PROTEIN KINASE AFSK (EC 2.7.1.-).//0.0065:148:29//STREPTOMYCES COELICOLOR.//P54741
F-PLACE1004316//AUTOPHAGY PROTEIN APG5.//8.8e-06:117:29//SACCHAROMYCES CEREVISIAE (BAKER'S YEAST).//Q12380
F-PLACE1004336//COLLAGEN ALPHA 4(IV) CHAIN PRECURSOR.//0.0027:83:36//HOMO SAPIENS (HUMAN).//P53420
F-PLACE1004358//PROCOLLAGEN ALPHA 1(I) CHAIN PRECURSOR.//2.9e-05:200:33//GALLUS GALLUS (CHICKEN).//P02457
F-PLACE1004376//AXONEME-ASSOCIATED PROTEIN MST101(2).//2.4e-05:179:29//DROSOPHILA HYDEI (FRUIT FLY).//Q08696
F-PLACE1004384//!!!! ALU SUBFAMILY SQ WARNING ENTRY !!!!//1.6e-28:46:76//HOMO SAPIENS (HUMAN).//P39194
F-PLACE1004388//HYPOTHETICAL 75.2 KD PROTEIN IN ACS1-GCV3 INTERGENIC REGION.//5.7e-34:202:37//SACCHAROMYCES CEREVISIAE (BAKER'S YEAST).//P39722
F-PLACE1004405//NEURAMINYLLACTOSE-BINDING HEMAGGLUTININ (N-ACETYLNEURAMINYLLACTOSE- BINDING FIBRILLAR HEMAGGLUTININ RECEPTOR-BINDING SUBUNIT) (NLBH) (FLAGELLAR SHEATH ADHESIN) (ADHESIN A) (FRAGMENT).//0.93:74:33//HELICOBACTER ACINONYX.//Q47947
F-PLACE1004425//!!!! ALU SUBFAMILY SX WARNING ENTRY !!!!//0.81:70:42//HOMO SAPIENS (HUMAN).//P39195
F-PLACE1004428//PRISTANOYL-COA OXIDASE (EC 1.3.3.-).//1.9e-31:203:39//RATTUS NORVEGICUS (RAT).//Q63448
F-PLACE1004437//ISOCITRATE DEHYDROGENASE [NAD], MITOCHONDRIAL SUBUNIT BETA PRECURSOR (EC 1.1.1.41) (ISOCITRIC DEHYDROGENASE) (NAD+-SPECIFIC ICDH) (FRAGMENT).//4.2e-93:140:100//MACACA FASCICULARIS (CRAB EATING MACAQUE) (CYNOMOLGUS MONKEY).//Q28479
F-PLACE1004451//!!!! ALU SUBFAMILY J WARNING ENTRY !!!!//0.00013:40:62//HOMO SAPIENS (HUMAN).//P39188
F-PLACE1004460//MATERNAL TUDOR PROTEIN.//0.0066:218:23//DROSOPHILA MELANOGASTER (FRUIT FLY).//P25823
F-PLACE1004467//!!!! ALU SUBFAMILY SP WARNING ENTRY !!!!//7.8e-10:33:87//HOMO SAPIENS (HUMAN).//P39193
F-PLACE1004471//ZINC FINGER PROTEIN 83 (ZINC FINGER PROTEIN HPF1).//7.0e-56:92:58//HOMO SAPIENS (HUMAN).//P51522
F-PLACE1004473//HYPOTHETICAL 54.3 KD PROTEIN C23D3.03C IN CHROMOSOME I.//0.019:136:27//SCHIZOSACCHAROMYCES POMBE (FISSION YEAST).//Q09844
F-PLACE1004491//LYSIS PROTEIN.//0.95:53:30//BACTERIOPHAGE FR.//P19903
F-PLACE1004506//AUTOIMMUNOGENIC CANCER/TESTIS ANTIGEN NY-ESO-1 (LAGE-1).//0.58:66:34//HOMO SAPIENS (HUMAN).//P78358
F-PLACE1004510//TRANSCRIPTION INITIATION FACTOR TFIID 150 KD SUBUNIT (TAFII-150) (TAFII150).//3.0e-07:63:46//DROSOPHILA MELANOGASTER (FRUIT FLY).//Q24325
F-PLACE1004516//HYPOTHETICAL PROTEIN 5' TO ASP-RICH AND HIS-RICH PROTEINS (FRAGMENT).//0.95:62:29//PLASMODIUM FALCIPARUM (ISOLATE FCM17 / SENEGAL).//P14587
F-PLACE1004518//METALLOTHIONEIN 10-III (MT-10-III).//0.91:28:42//MYTILUS EDULIS (BLUE MUSSEL).//P80248
F-PLACE1004548//DIHYDROPYRIDINE-SENSITIVE L-TYPE, SKELETAL MUSCLE CALCIUM CHANNEL GAMMA SUBUNIT.//0.94:75:32//ORYCTOLAGUS CUNICULUS (RABBIT).//P19518
F-PLACE1004550//CUTICLE COLLAGEN 2.//0.90:155:31//CAENORHABDITIS ELEGANS.//P17656
F-PLACE1004564//CLEAVAGE AND POLYADENYLATION SPECIFICITY FACTOR, 100 KD SUBUNIT (CPSF 100 KD SUBUNIT).//3.2e-70:121:100//BOS TAURUS (BOVINE).//Q10568
F-PLACE1004629//PROTEIN OS-9 PRECURSOR.//1.7e-10:132:36//HOMO SAPIENS (HUMAN).//Q13438
F-PLACE1004645//TRANSCRIPTION INITIATION FACTOR IIB HOMOLOG (TFIIB).//0.00036:100:30//PYROCOCCUS FURIOSUS.//Q51731
F-PLACE1004646//PROBABLE UDP-GALACTOPYRANOSE MUTASE (EC 5.4.99.9).//0.91:58:29//KLEBSIELLA PNEUMONIAE.//Q48481
F-PLACE1004658//GLUTAMATE [NMDA] RECEPTOR SUBUNIT EPSILON 4 PRECURSOR (N-METHYL D-ASPARTATE RECEPTOR SUBTYPE 2D) (NR2D) (NMDAR2D).//0.031:134:32//MUS MUSCULUS (MOUSE).//Q03391
F-PLACE1004664//HYPOTHETICAL 180.2 KD PROTEIN IN FAA4-HOR7 INTERGENIC REGION.//0.025:125:20//SACCHAROMYCES CEREVISIAE (BAKER'S YEAST).//Q04781
F-PLACE1004672//HYPOTHETICAL 36.7 KD PROTEIN C2F7:14C IN CHROMOSOME I.//7.6e-52:158:56//SCHIZOSACCHAROMYCES POMBE (FISSION YEAST).//Q09704
F-PLACE1004674//PROBABLE CALCIUM-BINDING PROTEIN ALG-2 (PMP41) (ALG-257).//1.4e-88:144:93//MUS MUSCULUS (MOUSE).//P12815
F-PLACE1004681//CCR4-ASSOCIATED FACTOR 1 (CAF1).//1.0e-34:70:100//MUS MUSCULUS (MOUSE).//Q60809
F-PLACE1004686//!!!! ALU SUBFAMILY SC WARNING ENTRY !!!!//3.4e-08:48:62//HOMO SAPIENS (HUMAN).//P39192
F-PLACE1004691//METALLOTHIONEIN (MT).//0.064:24:45//ARIANTA ARBUSTORUM.//P55946
F-PLACE1004693
F-PLACE1004716//ATP SYNTHASE PROTEIN 8 (EC 3.6.1.34) (A6L).//1.0:27:37//PAN PANISCUS (PYGMY CHIMPANZEE) (BONOBO).//Q35587
F-PLACE1004722//HYPOTHETICAL 61.5 KD PROTEIN IN CLA4-MID1 INTERGENIC REGION.//0.95:53:33//SACCHAROMYCES CEREVISIAE (BAKER'S YEAST).//P48565
F-PLACE1004736//NEURONAL AXONAL MEMBRANE PROTEIN NAP-22.//0.014:163:30//RATTUS NORVEGICUS (RAT).//Q05175
F-PLACE1004740//!!!! ALU SUBFAMILY SQ WARNING ENTRY !!!!//1.9e-09:37:70//HOMO SAPIENS (HUMAN).//P39194
F-PLACE1004743//HYPOTHETICAL 12.6 KD PROTEIN IN ALGR3 3'REGION.//0.99:72:33//PSEUDOMONAS AERUGINOSA.//P21484
F-PLACE1004751//CMP-N-ACETYLNEURAMINATE-BETA-GALACTOSAMIDE-ALPHA-2,3-SIALYLTRANSFERASE (EC 2.4.99.-) (BETA-GALACTOSIDE ALPHA-2,3-SIALYLTRANSFERASE) (ST3GALIII) (ALPHA 2,3-ST) (GAL-NAC6S) (STZ) (SIAT4-C) (SAT-3) (ST-4).//2.2e-08:90:38//HOMO SAPIENS (HUMAN).//Q11206
F-PLACE1004773//ANKYRIN R (ANKYRINS 2.1 AND 2.2) (ERYTHROCYTE ANKYRIN).//3.2e-25:233:32//HOMO SAPIENS (HUMAN).//P16157
F-PLACE1004777//N-CHIMAERIN (NC) (N-CHIMERIN) (ALPHA CHIMERIN) (A-CHIMAERIN).//8.1e-26:210:30//RATTUS NORVEGICUS (RAT).//P30337
F-PLACE1004793//ENV POLYPROTEIN [CONTAINS: COAT PROTEIN GP52; COAT PROTEIN GP36].//0.00062:106:25//MOUSE MAMMARY TUMOR VIRUS (STRAIN BR6).//P10259
F-PLACE1004804
F-PLACE1004813//HYPOTHETICAL PROTEIN UL12.//1.0:22:40//HUMAN CYTOMEGALOVIRUS (STRAIN AD169).//P16777
F-PLACE1004814//HYPOTHETICAL 37.0 KD PROTEIN B0495.8 IN CHROMOSOME II.//2.8e-06:136:25//CAENORHABDITIS ELEGANS.//Q09217
F-PLACE1004815
F-PLACE1004824//HYPOTHETICAL 106.7 KD PROTEIN IN MUP1-SPR3 INTERGENIC REGION.//2.3e-09:70:38//SACCHAROMYCES CEREVISIAE (BAKER'S YEAST).//P53236
F-PLACE1004827//HYPOTHETICAL 9.4 KD PROTEIN IN FLAL 3'REGION (ORF3).//0.54:25:56//BACILLUS LICHENIFORMIS.//P22754
F-PLACE1004836//ANNEXIN VII (SYNEXIN) (FRAGMENT).//0.0066:12:66//BOS TAURUS (BOVINE).//P20072
F-PLACE1004838
F-PLACE1004840
F-PLACE1004868//MALE STERILITY PROTEIN 2.//4.0e-16:172:30//ARABIDOPSIS THALIANA (MOUSE-EAR CRESS).//Q08891
F-PLACE1004885
F-PLACE1004900//MAST CELL DEGRANULATING PEPTIDE PRECURSOR (MCDP) (MCD) (PEPTIDE 401).//1.0:23:47//APIS MELLIFERA (HONEYBEE).//P01499
F-PLACE1004902//PUTATIVE PRE-MRNA SPLICING FACTOR ATP-DEPENDENT RNA HELICASE SPAC10F6.02C.//7.3e-15:94:47//SCHIZOSACCHAROMYCES POMBE (FISSION YEAST).//O42643
F-PLACE1004913//HYPOTHETICAL 7.2 KD PROTEIN IN BCSA-DEGR INTERGENIC REGION.//1.0:42:33//BACILLUS SUBTILIS.//P54165
F-PLACE1004918//HYPOTHETICAL 12.4 KD PROTEIN IN RPS21B-MRS3 INTERGENIC REGION.//0.98:50:34//SACCHAROMYCES CEREVISIAE (BAKER'S YEAST).//P47012
F-PLACE1004930//HYPOTHETICAL PROTEIN MJ0562.//0.82:44:36//METHANOCOCCUS JANNASCHII.//Q57982
F-PLACE1004934
F-PLACE1004937//HYPOTHETICAL 67.1 KD TRP-ASP REPEATS CONTAINING PROTEIN C57A10.05C IN CHROMOSOME I.//9.0e-10:87:33//SCHIZOSACCHAROMYCES POMBE (FISSION YEAST).//P87053
F-PLACE1004969//HYPOTHETICAL 55.1 KD PROTEIN B0416.5 IN CHROMOSOME X.//4.0e-14:184:25//CAENORHABDITIS ELEGANS.//Q11073
F-PLACE1004972//BROMELAIN INHIBITOR 2 (BI-II) (BROMELAIN INHIBITOR VI) (BI-VI).//1.0:35:37//ANANAS COMOSUS (PINEAPPLE).//P27478
F-PLACE1004979//!!!! ALU SUBFAMILY SC WARNING ENTRY !!!!//5.3e-30:55:72//HOMO SAPIENS (HUMAN).//P39192
F-PLACE1004982//M PROTEIN, SEROTYPE 12 PRECURSOR (FRAGMENT).//0.00049:124:27//STREPTOCOCCUS PYOGENES.//P19401
F-PLACE1004985//ATP SYNTHASE PROTEIN 8 (EC 3.6.1.34) (A6L).//1.0:26:34//LUMBRICUS TERRESTRIS (COMMON EARTHWORM).//Q34942
F-PLACE1005026//TELOMERE-BINDING PROTEIN HOMOLOG.//0.0011:179:27//EUPLOTES CRASSUS.//Q06183
F-PLACE1005027
F-PLACE1005046//ANNEXIN VII (SYNEXIN) (FRAGMENT).//0.082:44:36//BOS TAURUS (BOVINE).//P20072
F-PLACE1005052//MALE SPECIFIC SPERM PROTEIN MST84DD.//0.38:36:44//DROSOPHILA MELANOGASTER (FRUIT FLY).//Q01645
F-PLACE1005055
F-PLACE1005066//RING CANAL PROTEIN (KELCH PROTEIN).//2.9e-38:194:39//DROSOPHILA MELANOGASTER (FRUIT FLY).//Q04652
F-PLACE1005077
F-PLACE1005085//INSECT TOXIN 1 (BOT IT1).//0.85:36:33//BUTHUS OCCITANUS TUNETANUS (COMMON EUROPEAN SCORPION).//P55902
F-PLACE1005086//!!!! ALU SUBFAMILY SQ WARNING ENTRY !!!!//8.5e-38:93:76//HOMO SAPIENS (HUMAN).//P39194
F-PLACE1005101//HYPOTHETICAL PROTEIN ZAP128 (FRAGMENT).//1.6e-11:35:100//HOMO SAPIENS (HUMAN).//P49753
F-PLACE1005102//ZINC FINGER PROTEIN 151 (POLYOMAVIRUS LATE INITIATOR PROMOTER BINDING PROTEIN) (LP-1) (ZINC FINGER PROTEIN Z13).//3.0e-14:110:38//MUS MUSCULUS (MOUSE).//Q60821
F-PLACE1005108//METALLOTHIONEIN-III (MT-III) (GROWTH INHIBITORY FACTOR) (GIF).//0.41:35:34//BOS TAURUS (BOVINE).//P37359
F-PLACE1005111//ATP SYNTHASE PROTEIN 8 (EC 3.6.1.34) (A6L) (CHARGERIN II).//1.0:29:41//RATTUS NORVEGICUS (RAT).//P11608
F-PLACE1005128//RABPHILIN-3A (FRAGMENT).//5.9e-05:95:36//MUS MUSCULUS (MOUSE).//P47708
F-PLACE1005146//FIBROBLAST GROWTH FACTOR INDUCIBLE PROTEIN 15 (FIN15).//0.17:48:35//MUS MUSCULUS (MOUSE).//Q61075
F-PLACE1005162//!!!! ALU SUBFAMILY SB WARNING ENTRY !!!!//1.0e-31:60:76//HOMO SAPIENS (HUMAN).//P39189
F-PLACE1005176
F-PLACE1005181//HYPOTHETICAL 7 KD PROTEIN.//1.0:31:45//MEASLES VIRUS (STRAIN HALLE) (SUBACUTE SCLEROSE PANENCEPHALITIS VIRUS).//P06831
F-PLACE1005187//GLUCAN SYNTHASE-1 (EC 2.4.1.34) (1,3-BETA-GLUCAN SYNTHASE) (UDP-GLUCOSE-1,3-BETA-D-GLUCAN GLUCOSYLTRANSFERASE).//0.0025:58:34//NEUROSPORA CRASSA.//P38678
F-PLACE1005206//HYPOTHETICAL 10.7 KD PROTEIN.//0.34:57:42//VACCINIA VIRUS (STRAIN COPENHAGEN).//P20511
F-PLACE1005232//AMELOGENIN, Y ISOFORM PRECURSOR.//0.70:60:35//HOMO SAPIENS (HUMAN).//Q99218
F-PLACE1005243//SERINE/THREONINE PROTEIN KINASE PKPA (EC 2.7.1.-).//0.0017:114:27//PHYCOMYCES BLAKESLEEANUS.//Q01577
F-PLACE1005261//HYPOTHETICAL 90.8 KD PROTEIN T05H10.7 IN CHROMOSOME II.//1.2e-38:206:41//CAENORHABDITIS ELEGANS.//Q10003
F-PLACE1005266
F-PLACE1005277//PROTEIN GURKEN PRECURSOR.//0.58:95:29//DROSOPHILA MELANOGASTER (FRUIT FLY).//P42287
F-PLACE1005287//INNER CENTROMERE PROTEIN (INCENP).//2.0e-12:211:29//GALLUS GALLUS (CHICKEN).//P53352
F-PLACE1005305//GTP:AMP PHOSPHOTRANSFERASE MITOCHONDRIAL (EC 2.7.4.10) (AK3).//1.8e-78:205:78//BOS TAURUS (BOVINE).//P08760
F-PLACE1005308//WOUND-INDUCED BASIC PROTEIN.//0.99:40:40//PHASEOLUS VULGARIS (KIDNEY BEAN) (FRENCH BEAN).//Q09020
F-PLACE1005313//HYPOTHETICAL 8.7 KD PROTEIN IN LEUX-FECE INTERGENIC REGION (O67).//0.15:36:41//ESCHERICHIA COLI.//P39355
F-PLACE1005327//DNA-BINDING P52/P100 COMPLEX, 100 KD SUBUNIT (FRAGMENTS).//1.0:19:52//HOMO SAPIENS (HUMAN).//P30808
F-PLACE1005331//BREAKPOINT CLUSTER REGION PROTEIN.//0.00021:98:35//HOMO SAPIENS (HUMAN).//P11274
F-PLACE1005335//IROQUOIS-CLASS HOMEODOMAIN PROTEIN IRX-3.//0.37:98:33//MUS MUSCULUS (MOUSE).//P81067
F-PLACE1005373//PSEUDOURIDYLATE SYNTHASE 4 (EC 4.2.1.70) (PSEUDOURIDINE SYNTHASE 4) (TRNA PSEUDOURIDINE 55 SYNTHASE) (PSI55 SYNTHASE) (PSEUDOURIDYLATE SYNTHASE) (URACIL HYDROLYASE).//0.010:96:28//SACCHAROMYCES CEREVISIAE (BAKER'S YEAST).//P48567
F-PLACE1005374
F-PLACE1005409
F-PLACE1005453//LICHENASE PRECURSOR (EC 3.2.1.73) (ENDO-BETA-1,3-1,4 GLUCANASE).//1.0:50:32//NICOTIANA PLUMBAGINIFOLIA (LEADWORT-LEAVED TOBACCO).//P07979
F-PLACE1005467//KERATIN, FEATHER (F-KER).//0.0095:42:35//LARUS NOVAE-HOLLANDIAE (SILVER GULL).//P02451
F-PLACE1005471//NADH-UBIQUINONE OXIDOREDUCTASE CHAIN 4L (EC 1.6.5.3).//0.23:49:32//PHYTOPHTHORA INFESTANS (POTATO LATE BLIGHT FUNGUS).//Q37598
F-PLACE1005477//HYPOTHETICAL PROTEIN ORF-1137.//9.6e-13:115:38//MUS MUSCULUS (MOUSE).//P11260
F-PLACE1005480//C-HORDEIN (CLONE PC HOR1-3) (FRAGMENT).//0.97:33:30//HORDEUM VULGARE (BARLEY).//P17991
F-PLACE1005481//HUNCHBACK PROTEIN (FRAGMENT).//0.30:52:38//APIS MELLIFERA (HONEYBEE).//P31504
F-PLACE1005494//TRANSIENT-RECEPTOR-POTENTIAL PROTEIN.//3.9e-05:87:33//DROSOPHILA MELANOGASTER (FRUIT FLY).//P19334
F-PLACE1005502
F-PLACE1005526//IMMEDIATE-EARLY PROTEIN IE180.//4.6e-05:132:32//PSEUDORABIES VIRUS (STRAIN KAPLAN) (PRV).//P33479
F-PLACE1005528//!!!! ALU SUBFAMILY SX WARNING ENTRY !!!!//3.4e-09:31:74//HOMO SAPIENS (HUMAN).//P39195
F-PLACE1005530//HYPOTHETICAL 47.6 KD PROTEIN C16C10.5 IN CHROMOSOME III.//9.7e-50:148:58//CAENORHABDITIS ELEGANS.//Q09251
F-PLACE1005550//HYPOTHETICAL 40.2 KD PROTEIN K12H4.3 IN CHROMOSOME III.//3.0e-21:127:37//CAENORHABDITIS ELEGANS.//P34524
F-PLACE1005554//CYTOCHROME B (EC 1.10.2.2) (FRAGMENT).//0.84:38:31//DIPODOMYS CALIFORNICUS (KANGAROO RAT).//P16359
F-PLACE1005557//60S RIBOSOMAL PROTEIN L27.//4.8e-09:60:48//CRYPTOCOCCUS NEOFORMANS (FILOBASIDIELLA NEOFORMANS).//P46288
F-PLACE1005574//ATP SYNTHASE PROTEIN 8 (EC 3.6.1.34) (A6L).//0.89:44:29//BOS TAURUS (BOVINE).//P03929
F-PLACE1005584//MALE SPECIFIC SPERM PROTEIN MST87F.//0.00030:33:48//DROSOPHILA MELANOGASTER (FRUIT FLY).//P08175
F-PLACE1005595//IMMEDIATE-EARLY PROTEIN IE180.//0.00048:162:30//PSEUDORABIES VIRUS (STRAIN INDIANA-FUNKHAUSER / BECKER) (PRV).//P11675
F-PLACE1005603//HIGH-MOBILITY-GROUP PROTEIN (NONHISTONE CHROMOSOMAL PROTEIN).//0.00034:83:30//TETRAHYMENA PYRIFORMIS.//P40625
F-PLACE1005611//DNAJ PROTEIN.//8.6e-20:108:48//CLOSTRIDIUM ACETOBUTYLICUM.//P30725
F-PLACE1005623//EXTRACELLULAR SIGNAL-REGULATED KINASE 5 (EC 2.7.1.-) (ERK5) (ERK4) (BMK1 KINASE).//0.80:116:31//HOMO SAPIENS (HUMAN).//Q13164
F-PLACE1005630//INTERLEUKIN-14 PRECURSOR (IL-14) (HIGH MOLECULAR WEIGHT B-CELL GROWTH FACTOR) (HMW-BCGF).//0.0024:74:39//HOMO SAPIENS (HUMAN).//P40222
F-PLACE1005639//EXTRACELLULAR MATRIX PROTEIN 1 (SECRETORY COMPONENT P85) (FRAGMENT).//0.72:18:61//RATTUS NORVEGICUS (RAT).//Q62894
F-PLACE1005646//RNA HELICASE-LIKE PROTEIN DB10.//4.8e-29:172:45//NICOTIANA SYLVESTRIS (WOOD TOBACCO).//P46942
F-PLACE1005656//RIBONUCLEOSIDE-DIPHOSPHATE REDUCTASE M2 CHAIN (EC 1.17.4.1) (RIBONUCLEOTIDE REDUCTASE).//3.7e-64:133:75//MESOCRICETUS AURATUS (GOLDEN HAMSTER).//Q60561
F-PLACE1005666//CHLOROPLAST 50S RIBOSOMAL PROTEIN L28.//0.57:36.41//PORPHYRA PURPUREA.//P51224
F-PLACE1005698//HYPOTHETICAL PROTEIN IN SIGD 3'REGION (ORFC) (FRAGMENT).//0.50:61:29//BACILLUS SUBTILIS.//P40405
F-PLACE1005727//ANTER-SPECIFIC PROLINE-RICH PROTEIN APG (PROTEIN CEX) (FRAGMENT).//0.46:27:51//BRASSICA NAPUS (RAPE).//P40603
F-PLACE1005730//COLLAGEN ALPHA 1(I) CHAIN (FRAGMENT).//0.95:21:52//ORYCTOLAGUS CUNICULUS (RABBIT).//P02456
F-PLACE1005739//INTERFERON-GAMMA INDUCIBLE PROTEIN MG11.//3.4e-46:111:53//MUS MUSCULUS (MOUSE).//Q60710
F-PLACE1005755//HYPOTHETICAL 70.2 KD PROTEIN IN GSH1-CHS6 INTERGENIC REGION.//2.6e-12:66:51//SACCHAROMYCES CEREVISIAE (BAKER'S YEAST).//P42951
F-PLACE1005763//S-ACYL FATTY ACID SYNTHASE THIOESTERASE, MEDIUM CHAIN (EC 3.1.2.14) (THIOESTERASE II).//1.5e-26:69:57//RATTUS NORVEGICUS (RAT).//P08635
F-PLACE1005799//KERATIN, ULTRA HIGH-SULFUR MATRIX PROTEIN (UHS KERATIN).//0.028:96:32//HOMO SAPIENS (HUMAN).//P26371
F-PLACE1005802//PROTEIN PROSPERO.//0.86:64:42//DROSOPHILA MELANOGASTER (FRUIT FLY).//P29617
F-PLACE1005803//MYELOID DIFFERENTIATION PRIMARY RESPONSE PROTEIN MYD116.//1.0:95:25//MUS MUSCULUS (MOUSE).//P17564
F-PLACE1005804//PROCESSING ALPHA-1,2-MANNOSIDASE (EC 3.2.1.-) (ALPHA-1,2-MANNOSIDASE 1B).//2.8e-73:198:73//MUS MUSCULUS (MOUSE).//P39098
F-PLACE1005813//HYPOTHETICAL 49.0 KD PROTEIN IN NSP1-KAR2 INTERGENIC REGION.//0.022:78:38//SACCHAROMYCES CEREVISIAE (BAKER'S YEAST).//P47057
F-PLACE1005828//!!!! ALU SUBFAMILY SX WARNING ENTRY !!!!//1.8e-23:56:76//HOMO SAPIENS (HUMAN).//P39195
F-PLACE1005834//LATE CONTROL GENE B PROTEIN (GPB).//0.97:33:39//BACTERIOPHAGE 186.//P08711
F-PLACE1005845
F-PLACE1005850//!!!! ALU SUBFAMILY SQ WARNING ENTRY !!!!//5.5e-28:96:73//HOMO SAPIENS (HUMAN).//P39194
F-PLACE1005851
F-PLACE1005876//CLEAVAGE AND POLYADENYLATION SPECIFICITY FACTOR, 100 KD SUBUNIT (CPSF 100 KD SUBUNIT).//2.2e-99:155:95//BOS TAURUS (BOVINE).//Q10568
F-PLACE1005884
F-PLACE1005890//BEM46 PROTEIN (FRAGMENT).//1.8e-33:137:49//SCHIZOSACCHAROMYCES POMBE (FISSION YEAST).//P54069
F-PLACE1005898//NADH-UBIQUINONE OXIDOREDUCTASE MLRQ SUBUNIT (EC 1.6.5.3) (EC 1.6.99.3) (COMPLEX I-MLRQ) (CI-MLRQ).//0.77:58:34//HOMO SAPIENS (HUMAN).//O00483
F-PLACE1005921//AIG1 PROTEIN.//1.4e-23:165:38//ARABIDOPSIS THALIANA (MOUSE-EAR CRESS).//P54120
F-PLACE1005923//HYPOTHETICAL 22.4 KD PROTEIN (ORF16).//0.90:118:28//PARAMECIUM TETRAURELIA.//P15617
F-PLACE1005925//HYPOTHETICAL GENE 30 PROTEIN.//0.94:57:29//HERPESVIRUS SAIMIRI (STRAIN 11).//Q01010
F-PLACE1005932//EXTENSIN PRECURSOR (CELL WALL HYDROXYPROLINE-RICH GLYCOPROTEIN).//0.42:128:32//NICOTIANA TABACUM (COMMON TOBACCO).//P13983
F-PLACE1005934//DNA-DIRECTED RNA POLYMERASE II LARGEST SUBUNIT (EC 2.7.7.6)(RPB1) (FRAGMENT).//0.40:76:35//CRICETULUS GRISEUS (CHINESE HAMSTER).//P11414
F-PLACE1005936//TAT PROTEIN (TRANSACTIVATING REGULATORY PROTEIN).//0.50:15:66//HUMAN IMMUNODEFICIENCY VIRUS TYPE 1 (CLONE 12) (HIV-1).//P04326
F-PLACE1005951//EXTENSIN PRECURSOR (CELL WALL HYDROXYPROLINE-RICH GLYCOPROTEIN).//0.0025:135:32//NICOTIANA TABACUM (COMMON TOBACCO).//P13983
F-PLACE1005953//HIGH POTENTIAL IRON-SULFUR PROTEIN (HIPIP).//0.64:57:33//RHODOFERAX FERMENTANS.//P80882
F-PLACE1005955//HYPOTHETICAL 54.2 KD PROTEIN IN ERP5-ORC6 INTERGENIC REGION.//1.0e-32:110:50//SACCHAROMYCES CEREVISIAE (BAKER'S YEAST).//P38821
F-PLACE1005966//TACHYPLESIN II PRECURSOR.//0.97:31:35//TACHYPLEUS TRIDENTATUS (JAPANESE HORSESHOE CRAB).//P14214
F-PLACE1005968//GATA FACTOR SREP.//0.17:52:40//PENICILLIUM CHRYSOGENUM.//Q92259
F-PLACE1005990//CELL PATTERN FORMATION-ASSOCIATED PROTEIN.//0.36:55:36//EMERICELLA NIDULANS (ASPERGILLUS NIDULANS).//P36011
F-PLACE1006002//!!!! ALU SUBFAMILY SC WARNING ENTRY !!!!//1.5e-36:102:75//HOMO SAPIENS (HUMAN).//P39192
F-PLACE1006003//HYPOTHETICAL 6.8 KD PROTEIN IN COX3-NAD1 INTERGENIC REGION (ORF 61).//1.0:22:40//MARCHANTIA POLYMORPHA (LIVERWORT).//P38473
F-PLACE1006011//POLY [ADP-RIBOSE] POLYMERASE (EC 2.4.2.30) (PARP) (ADPRT) (NAD(+) ADP- RIBOSYLTRANSFERASE) (POLY[ADP-RIBOSE] SYNTHETASE).//2.8e-21:163:36//ARABIDOPSIS THALIANA (MOUSE-EAR CRESS).//Q11207
F-PLACE1006017//!!!! ALU SUBFAMILY SC WARNING ENTRY !!!!//1.1e-10:43:67//HOMO SAPIENS (HUMAN).//P39192
F-PLACE1006037//VITELLOGENIN I PRECURSOR (VTG I) [CONTAINS: LIPOVITELLIN 1 (LV1); PHOSVITIN (PV); LIPOVITELLIN 2 (LV2)].//0.00019:123:37//FUNDULUS HETEROCLITUS (KILLIFISH) (MUMMICHOG).//Q90508
F-PLACE1006040//CAMP-REGULATED PHOSPHOPROTEIN 19 (ARPP-19).//3.2e-40:110:76//HOMO SAPIENS (HUMAN).//P56211
F-PLACE1006076//BOWMAN-BIRK TYPE PROTEINASE INHIBITOR A-II.//0.99:30:40//ARACHIS HYPOGAEA (PEANUT).//P01066
F-PLACE1006119//IMPORTIN BETA-3 SUBUNIT (KARYOPHERIN BETA-3 SUBUNIT) (RAN-BINDING PROTEIN 5).//8.8e-94:218:76//HOMO SAPIENS (HUMAN).//O00410
F-PLACE1006129//A-AGGLUTININ ATTACHMENT SUBUNIT PRECURSOR.//0.00092:228:26//SACCHAROMYCES CEREVISIAE (BAKER'S YEAST).//P32323
F-PLACE1006139//HYPOTHETICAL 52.9 KD PROTEIN IN SAP155-YMR31 INTERGENIC REGION.//5.9e-55:128:50//SACCHAROMYCES CEREVISIAE (BAKER'S YEAST).//P43616
F-PLACE1006143//!!!! ALU SUBFAMILY SQ WARNING ENTRY !!!!//1.4e-25:107:63//HOMO SAPIENS (HUMAN).//P39194
F-PLACE1006157//E-SELECTIN PRECURSOR (ENDOTHELIAL LEUKOCYTE ADHESION MOLECULE 1) (ELAM-1) (LEUKOCYTE-ENDOTHELIAL CELL ADHESION MOLECULE 2) (LECAM2) (CD62E).//1.3e-21:168:32//SUS SCROFA (PIG).//P98110
F-PLACE1006159//COLD SHOCK INDUCED PROTEIN TIR1 PRECURSOR (SERINE-RICH PROTEIN 1).//0.46:98:29//SACCHAROMYCES CEREVISIAE (BAKER'S YEAST).//P10863
F-PLACE1006164//NADH-UBIQUINONE OXIDOREDUCTASE CHAIN 4L (EC 1.6.5.3).//0:70:28:42//ARTEMIA SALINA (BRINE SHRIMP).//P19049
F-PLACE1006167//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//8.9e-05:167:32//SACCHAROMYCES CEREVISIAE (BAKER'S YEAST).//P08640
F-PLACE1006170//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT).//1.1e-67:157:88//MUS MUSCULUS (MOUSE).//P17427
F-PLACE1006187//G1/S-SPECIFIC CYCLIN E.//5.6e-75:224:62//HOMO SAPIENS (HUMAN).//P24864
F-PLACE1006195//T-RELATED PROTEIN (TRP) (BRACHYENTERON PROTEIN).//0.99:177:29//DROSOPHILA MELANOGASTER (FRUIT FLY).//P55965
F-PLACE1006196//PUTATIVE ATP-DEPENDENT RNA HELICASE C12C2.06.//2.0e-33:183:46//SCHIZOSACCHAROMYCES POMBE (FISSION YEAST).//Q09747
F-PLACE1006205
F-PLACE1006223//SPERM MITOCHONDRIAL CAPSULE SELENOPROTEIN (MCS).//0.00015:22:50//MUS MUSCULUS (MOUSE).//P15265
F-PLACE1006225//VIRION INFECTIVITY FACTOR (SOR PROTEIN).//1.0:63:34//HUMAN IMMUNODEFICIENCY VIRUS TYPE 1 (NDK ISOLATE) (HIV-1).//P18805
F-PLACE1006236
F-PLACE1006239//60S ACIDIC RIBOSOMAL PROTEIN P2 (FRAGMENT).//0.48:23:52//ARABIDOPSIS THALIANA (MOUSE-EAR CRESS).//P51407
F-PLACE1006246//CMP-SIALIC ACID TRANSPORTER (CMP-SIA-TR).//0.012:84:30//MUS MUSCULUS (MOUSE).//Q61420
F-PLACE1006248//140 KD NUCLEOLAR PHOSPHOPROTEIN (NOPP140).//0.017:203:22//RATTUS NORVEGICUS (RAT).//P41777
F-PLACE1006262//L-FUCULOSE PHOSPHATE ALDOLASE (EC 4.1.2.17).//0.84:25:52//HAEMOPHILUS INFLUENZAE.//P44777
F-PLACE1006288
F-PLACE1006318//CYSTEINE-RICH ANTIFUNGAL PROTEIN 1 (AFP1) (M1).//1.0:29:48//SINAPIS ALBA (WHITE MUSTARD) (BRASSICA HIRTA).//P30231
F-PLACE1006325//CYCLIN-DEPENDENT KINASE INHIBITOR 1C (CYCLIN-DEPENDENT KINASE INHIBITOR P57) (P57KIP2).//0.99:97:32//HOMO SAPIENS (HUMAN).//P49918
F-PLACE1006335//PROLINE-RICH PEPTIDE P-B.//0.56:19:52//HOMO SAPIENS (HUMAN).//P02814
F-PLACE1006357
F-PLACE1006360
F-PLACE1006368//NUF1 PROTEIN (SPINDLE POLY BODY SPACER PROTEIN SPC110).//0.0057:122:31//SACCHAROMYCES CEREVISIAE (BAKER'S YEAST).//P32380
F-PLACE1006371//ARS BINDING PROTEIN 1.//0.00030:142:30//SCHIZOSACCHAROMYCES POMBE (FISSION YEAST).//P49777
F-PLACE1006382//NEUROTOXIN V.//0.85:28:39//ANDROCTONUS MAURETANICUS MAURETANICUS (SCORPION).//P01482
F-PLACE1006385//HYPOTHETICAL 45.1 KD PROTEIN IN RPS5-ZMS1 INTERGENIC REGION.//3.1e-35:165:47//SACCHAROMYCES CEREVISIAE (BAKER'S YEAST).//P47160
F-PLACE1006412//LINE-1 REVERSE TRANSCRIPTASE HOMOLOG.//4.3e-08:40:47//HOMO SAPIENS (HUMAN).//P08547
F-PLACE1006414//FORKHEAD-RELATED TRANSCRIPTION FACTOR 4 (FREAC-4).//3.8e-05:123:39//HOMO SAPIENS (HUMAN).//Q16676
F-PLACE1006438//ZINC FINGER PROTEIN 165.//2.8e-21:76:64//HOMO SAPIENS (HUMAN).//P49910
F-PLACE1006445//SUPPRESSOR OF HAIRY WING PROTEIN.//0.058:99:29//DROSOPHILA VIRILIS (FRUIT FLY).//Q08876
F-PLACE1006469//ACETYL-COENZYME A SYNTHETASE (EC 6.2.1.1) (ACETATE--COA LIGASE) (ACYL- ACTIVATING ENZYME).//1.8e-64:177:50//ESCHERICHIA COLI.//P27550
F-PLACE1006470
F-PLACE1006482//TRANSCRIPTION FACTOR MAFF.//2.0e-47:120:85//GALLUS GALLUS (CHICKEN).//Q90595
F-PLACE1006488//SIGNAL RECOGNITION PARTICLE 68 KD PROTEIN (SRP68).//1.8e-85:173:95//CANIS FAMILIARIS (DOG).//Q00004
F-PLACE1006492//VERY HYPOTHETICAL 11.2 KD PROTEIN C56F8.13 IN CHROMOSOME I.//0.75:32:56//SCHIZOSACCHAROMYCES POMBE (FISSION YEAST).//Q10261
F-PLACE1006506
F-PLACE1006521
F-PLACE1006531//HYPOTHETICAL 115.4 KD PROTEIN ZK757.3 IN CHROMOSOME III.//1.3e-53:167:61//CAENORHABDITIS ELEGANS.//P34681
F-PLACE1006534
F-PLACE1006540
F-PLACE1006552//MYOSIN HEAVY CHAIN, CLONE 203 (FRAGMENT).//1.3e-07:242:23//HYDRA ATTENUATA (HYDRA) (HYDRA VULGARIS).//P39922
F-PLACE1006598//!!!! ALU SUBFAMILY SB1 WARNING ENTRY !!!!//0.17:43:51//HOMO SAPIENS (HUMAN).//P39190
F-PLACE1006615//ACROSIN PRECURSOR (EC 3.4.21.10).//3.6e-05:66:43//ORYCTOLAGUS CUNICULUS (RABBIT).//P48038
F-PLACE1006617//HYPOTHETICAL 14.6 KD PROTEIN (READING FRAME C) (REPUCATION).//1.0:74:29//STAPHYLOCOCCUS AUREUS.//P03861
F-PLACE1006626//HYPOTHETICAL HELICASE K12H4.8 IN CHROMOSOME III.//2.9e-10:73:46//CAENORHABDITIS ELEGANS.//P34529
F-PLACE1006629//HYPOTHETICAL PROTEIN BB0410.//1.0:23:43//BORRELIA BURGDORFERI (LYME DISEASE SPIROCHETE).//O51371
F-PLACE1006640
F-PLACE1006673
F-PLACE1006678//COLLAGEN ALPHA 1(I) CHAIN (FRAGMENT).//1.0:36:41//ORYCTOLAGUS CUNICULUS (RABBIT).//P02456
F-PLACE1006704//BROAD-COMPLEX CORE-TNT1-Q1-Z1 PROTEIN (BRCORE-TNT1-Q1-Z1) [CONTAINS: BROAD-COMPLEX CORE-Q1-Z1
PROTEIN].//0.00062:157:26//DROSOPHILA MELANOGASTER (FRUIT FLY).//Q01295
F-PLACE1006731//RIBOFLAVIN KINASE (EC 2.7.1.26) (FLAVOKINASE) / FMN ADENYLYLTRANSFERASE (EC 2.7.7.2) (FAD PYROPHOSPHORYLASE) (FAD SYNTHETASE).//1.3e-07:127:36//CORYNEBACTERIUM AMMONIAGENES (BREVIBACTERIUM AMMONIAGENES).//Q59263
F-PLACE1006754//CARCINOEMBRYONIC ANTIGEN CGM1 PRECURSOR (CD66D ANTIGEN).//1.9e-19:78:53//HOMO SAPIENS (HUMAN).//P40198
F-PLACE1006760//COLLAGEN ALPHA 1(III) CHAIN (FRAGMENT).//0.21:107:30//RATTUS NORVEGICUS (RAT).//P13941
F-PLACE1006779//CYTOTOXIN 5 (CTX V).//1.0:20:30//NAJA MOSSAMBICA (MOZAMBIQUE COBRA).//P25517
F-PLACE1006782//ZINC FINGER PROTEIN 1.//0.00052:178:28//CANDIDA ALBICANS (YEAST).//P28875
F-PLACE1006792
F-PLACE1006795//VOLTAGE-GATED POTASSIUM CHANNEL PROTEIN SHAW (SHAW2).//1:0:80:30//DROSOPHILA MELANOGASTER (FRUIT FLY).//P17972
F-PLACE1006800//HYPOTHETICAL 9.4 KD PROTEIN.//0.99:62:33//VACCINIA VIRUS (STRAIN COPENHAGEN).//P20569
F-PLACE1006805
F-PLACE1006815//HYPOTHETICAL PROTEIN UL61.//0.038:146:32//HUMAN CYTOMEGALOVIRUS (STRAIN AD169).//P16818
F-PLACE1006819//LINE-1 REVERSE TRANSCRIPTASE HOMOLOG.//7.3e-98:239:76//HOMO SAPIENS (HUMAN).//P08547
F-PLACE1006829//UBIQUITIN CARBOXYL-TERMINAL HYDROLASE 8 (EC 3.1.2.15) (UBIQUITIN THIOLESTERASE 8) (UBIQUITIN-SPECIFIC PROCESSING PROTEASE 8) (DEUBIQUITINATING ENZYME 8).//0.061:34:58//SACCHAROMYCES CEREVISIAE (BAKER'S YEAST).//P50102
F-PLACE1006860
F-PLACE1006867
F-PLACE1006878//HYPOTHETICAL 8.2 KD PROTEIN IN MOBL 3'REGION (ORF 3).//0.85:27:37//THIOBACILLUS FERROOXIDANS.//P20087
F-PLACE1006883//VITAMIN D3 RECEPTOR (VDR) (1,25-DIHYDROXYVITAMIN D3 RECEPTOR).//0.78:51:37//MUS MUSCULUS (MOUSE).//P48281
F-PLACE1006901//HYPOTHETICAL 8.1 KD PROTEIN.//0.99:55:23//VACCINIA VIRUS (STRAIN COPENHAGEN).//P20567
F-PLACE1006904//MATING-TYPE LOCUS ALLELE B1 PROTEIN.//0.95:86:26//USTILAGO MAYDIS (SMUT FUNGUS).//P22015
F-PLACE1006917//HYPOTHETICAL 40.9 KD PROTEIN C08B11.5 IN CHROMOSOME II.//6.9e-15:101:45//CAENORHABDITIS ELEGANS.//Q09442
F-PLACE1006932//HISTIDINE-RICH, METAL BINDING POLYPEPTIDE.//0.089:28:39//HELICOBACTER PYLORI (CAMPYLOBACTER PYLORI).//Q48251
F-PLACE1006935//HYPOTHETICAL 95.2 KD PROTEIN R144.6 IN CHROMOSOME III.//0.93:35:48//CAENORHABDITIS ELEGANS.//Q10000
F-PLACE1006956//TRANSCRIPTION INITIATION FACTOR TFIID 135 KD SUBUNIT (TAFII-135) (TAFII135) (TAFII-130) (TAFII130).//0.00079:122:36//HOMO SAPIENS (HUMAN).//O00268
F-PLACE1006958//OSMOTIC STRESS PROTEIN 94 (HEAT SHOCK 70-RELATED PROTEIN APG-1).//8.8e-70:140:98//MUS MUSCULUS (MOUSE).//P48722
F-PLACE1006961
F-PLACE1006962//APOLIPOPROTEIN C-I PRECURSOR (APO-C1).//1.0:25:40//PAPIO HAMADRYAS (HAMADRYAS BABOON).//P34929
F-PLACE1006966//HYPOTHETICAL 49.1 KD PROTEIN IN SSB2-SPX18 INTERGENIC REGION.//1.6e-47:221:45//SACCHAROMYCES CEREVISIAE (BAKER'S YEAST).//P40160
F-PLACE1006989//HYPOTHETICAL 13.1 KD HIT-LIKE PROTEIN IN P37 5'REGION.//0.15:46:32//MYCOPLASMA HYORHINIS.//P32083
F-PLACE1007014//36 KD NUCLEOLAR PROTEIN HNP36 (DELAYED-EARLY RESPONSE PROTEIN 12) (DER12).//3.4e-09:120:29//HOMO SAPIENS (HUMAN).//Q14542
F-PLACE1007021//!!!! ALU SUBFAMILY J WARNING ENTRY !!!!//0.00046:42:59//HOMO SAPIENS (HUMAN).//P39188
F-PLACE1007045//HYPOTHETICAL PROTEIN ORF-1137.//8.1e-14:115:35//MUS MUSCULUS (MOUSE).//P11260
F-PLACE1007053//SMALL PROLINE RICH PROTEIN II (SPR-II) (CLONE 930).//0.038:48:39//HOMO SAPIENS (HUMAN).//P22531
F-PLACE1007068//PROTEIN-LYSINE 6-OXIDASE PRECURSOR (EC 1.4.3.13) (LYSYL OXIDASE).//0.0040:113:39//GALLUS GALLUS (CHICKEN).//Q05063
F-PLACE1007097//HYPOTHETICAL 6.8 KD PROTEIN IN HE65-PK2 INTERGENIC REGION.//0.97:47:29//AUTOGRAPHA CALIFORNICA NUCLEAR POLYHEDROSIS VIRUS (ACMNPV).//P41663
F-PLACE1007105//HYPOTHETICAL 83.6 KD PROTEIN C15A10.10 IN CHROMOSOME L//2.9e-33:219:37//SCHIZOSACCHAROMYCES POMBE (FISSION YEAST).//O13730
F-PLACE1007111
F-PLACE1007112//HYPOTHETICAL 9.2 KD PROTEIN.//0.47:75:28//ESCHERICHIA COLI.//P03853
F-PLACE1007132//!!!! ALU SUBFAMILY J WARNING ENTRY !!!!//1.8e-11:56:57//HOMO SAPIENS (HUMAN).//P39188
F-PLACE1007140//GAR2 PROTEIN.//0.72:185:24//SCHIZOSACCHAROMYCES POMBE (FISSION YEAST).//P41891
F-PLACE1007178//HYPOTHETICAL 8.5 KD PROTEIN CY274.40C.//0.97:79:30//MYCOBACTERIUM TUBERCULOSIS.//Q10826
F-PLACE1007226//HYPOTHETICAL 42.6 KD PROTEIN IN GSHB-ANSB INTERGENIC REGION (O378).//1.9e-15:123:32//ESCHERICHIA COLI.//P52062
F-PLACE1007238//MYOSIN HEAVY CHAIN IB (MYOSIN HEAVY CHAIN IL).//5.5e-10:98:44//ACANTHAMOEBA CASTELLANII (AMOEBA).//P19706
F-PLACE1007239//TRANSCRIPTION ELONGATION FACTOR S-II (TRANSCRIPTION ELONGATION FACTOR A).//3.9e-19:96:57//HOMO SAPIENS (HUMAN).//P23193
F-PLACE1007242//GUANINE NUCLEOTIDE DISSOCIATION STIMULATOR RALGDS FORM B (RALGEF).//1.0:132:30//RATTUS NORVEGICUS (RAT).//Q03386
F-PLACE1007243//HYPOTHETICAL 53.3 KD PROTEIN IN HXT8-CAN1 INTERGENIC REGION.//0.041:114:29//SACCHAROMYCES CEREVISIAE (BAKER'S YEAST).//P39981
F-PLACE1007257//DIAPHANOUS PROTEIN.//1.3e-42:205:46//DROSOPHILA MELANOGASTER (FRUIT FLY).//P48608
F-PLACE1007274//CADMIUM-METALLOTHIONEIN (CD-MT).//0.054:60:30//HELIX POMATIA (ROMAN SNAIL) (EDIBLE SNAIL).//P33187
F-PLACE1007276//BETA-DEFENSIN 1 PRECURSOR (RHBD-1) (DEFENSIN, BETA 1).//1.0:42:28//SUS SCROFA (PIG).//O62697
F-PLACE1007282//OUTER CAPSID PROTEIN VP4 (HEMAGGLUTININ) (OUTER LAYER PROTEIN VP4) [CONTAINS: OUTER CAPSID PROTEINS VP5 AND VP8].//0.070:126:27//HUMAN ROTAVIRUS (SEROTYPE 4 / STRAIN ST. THOMAS 3).//P11200
F-PLACE1007286
F-PLACE1007301//HYPOTHETICAL PROTEIN KIAA0168.//0.042:61:39//HOMO SAPIENS (HUMAN).//P50749
F-PLACE1007317
F-PLACE1007342//PROTEIN GRAINY-HEAD (DNA-BINDING PROTEIN ELF-1) (ELEMENT I-BINDING ACTIVITY) (TRANSCRIPTION FACTOR NTF-1).//1.7e-06:77:36//DROSOPHILA MELANOGASTER (FRUIT FLY).//P13002
F-PLACE1007346//TRANSCRIPTION INTERMEDIARY FACTOR 1-BETA (KRAB-A INTERACTING PROTEIN) (KRIP-1).//0.0026:147:27//MUS MUSCULUS (MOUSE).//Q62318
F-PLACE1007367//!!!! ALU SUBFAMILY SB WARNING ENTRY !!!!//1.3e-37:110:76//HOMO SAPIENS (HUMAN).//P39189
F-PLACE1007375//PHORBOL ESTER/DIACYLGLYCEROL-BINDING PROTEIN UNC-13.//4.7e-07:71:39//CAENORHABDITIS ELEGANS.//P27715
F-PLACE1007386//HYPOTHETICAL 7.6 KD PROTEIN IN FLO1-PHO11 INTERGENIC REGION.//0.74:48:29//SACCHAROMYCES CEREVISIAE (BAKER'S YEAST).//P39561
F-PLACE1007402//TRANSCRIPTIONAL REGULATORY PROTEIN ENTR (ENTERICIDIN R).//0.99:63:36//CITROBACTER FREUNDII.//O69280
F-PLACE1007409//WHITE PROTEIN.//7.9e-38:179:41//DROSOPHILA MELANOGASTER (FRUIT FLY).//P10090
F-PLACE1007416//DIPEPTIDYL PEPTIDASE IV (EC 3.4.14.5) (DPP IV) (T-CELL ACTIVATION ANTIGEN CD26) (TP103) (ADENOSINE DEAMINASE COMPLEXING PROTEIN-2) (ADABP).//0.031:159:23//HOMO SAPIENS (HUMAN).//P27487
F-PLACE1007450//ZINC FINGER PROTEIN 39 (ZINC FINGER PROTEIN KOX27) (FRAGMENT).//0.023:36:50//HOMO SAPIENS (HUMAN).//P17038
F-PLACE1007452//HYPOTHETICAL 22.1 KD PROTEIN IN CCP1-MET1 INTERGENIC REGION.//2.2e-18:85:54//SACCHAROMYCES CEREVISIAE (BAKER'S YEAST).//P36149
F-PLACE1007454//PHOTOSYSTEM II REACTION CENTRE N PROTEIN.//0.66:13:53//CHLAMYDOMONAS REINHARDTII.//Q06480
F-PLACE1007460//ATP SYNTHASE PROTEIN 8 (EC 3.6.1.34) (A6L).//0.93:45:33//SUS SCROFA (PIG).//Q35914
F-PLACE1007478//RETROVIRUS-RELATED POL POLYPROTEIN [CONTAINS: REVERSE TRANSCRIPTASE (EC 2.7.7.49); ENDONUCLEASE].//5.3e-08:50:56//MUS MUSCULUS (MOUSE).//P11369
F-PLACE1007484//HYPOTHETICAL 6.8 KD PROTEIN IN REPLICATION ORIGIN REGION.//0.87:43:37//ESCHERICHIA COLI.//P03849
F-PLACE1007488//PUTATIVE RHO/RAC GUANINE NUCLEOTIDE EXCHANGE FACTOR (RHO/RAC GEF) (FACIOGENITAL DYSPLASIA PROTEIN).//1.2e-25:202:31//HOMO SAPIENS (HUMAN).//P98174
F-PLACE1007507//HYPOTHETICAL 16.0 KD PROTEIN IN TAF60-G4P1 INTERGENIC REGION.//0.12:128:25//SACCHAROMYCES CEREVISIAE (BAKER'S YEAST).//P53139
F-PLACE1007511//KERATIN, TYPE I CYTOSKELETAL 19 (CYTOKERATIN 19) (K19) (CK 19).//2.1e-45:209:48//BOS TAURUS (BOVINE).//P08728
F-PLACE1007524//HYPOTHETICAL 9.2 KD PROTEIN.//0.74:80:30//VACCINIA VIRUS (STRAIN COPENHAGEN).//P20550
F-PLACE1007525
F-PLACE1007537//MYOTROPHIN (V-1 PROTEIN) (GRANULE CELL DIFFERENTIATION PROTEIN).//0.045:92:30//MUS MUSCULUS (MOUSE), AND RATTUS NORVEGICUS (RAT).//P80144
F-PLACE1007544//IMMEDIATE-EARLY PROTEIN IE180.//1.5e-07:59:50//PSEUDORABIES VIRUS (STRAIN KAPLAN) (PRV).//P33479
F-PLACE1007547//HYPOTHETICAL 97.1 KD PROTEIN R05D3.4 IN CHROMOSOME III.//2.5e-16:188:34//CAENORHABDITIS ELEGANS.//P34537
F-PLACE1007557
F-PLACE1007583//PROLINE RICH 33 KD EXTENSIN-RELATED PROTEIN PRECURSOR (FRAGMENT).//0.98:72:33//DAUCUS CAROTA (CARROT) .//P06600
F-PLACE1007598//ZINC FINGER PROTEIN 92 (ZINC FINGER PROTEIN HTF12) (FRAGMENT).//1.7e-11:88:43//HOMO SAPIENS (HUMAN).//Q03936
F-PLACE1007618//ANION EXCHANGE PROTEIN 2 (NON-ERYTHROID BAND 3-LIKE PROTEIN) (B3RP).//0.19:109:27//MUS MUSCULUS (MOUSE).//P13808
F-PLACE1007621//PHOSPHATE REGULON SENSOR PROTEIN PHOR (EC 2.7.3.-) (FRAGMENT).//0.98:34:41//PSEUDOMONAS AERUGINOSA.//P23621
F-PLACE1007632//COLLAGEN ALPHA 2(I) CHAIN (FRAGMENT).//0.70:110:34//BOS TAURUS (BOVINE).//P02465
F-PLACE1007645//ATP SYNTHASE PROTEIN 8 (EC 3.6.1.34) (A6L).//0.99:20:45//STRUTHIO CAMELUS (OSTRICH).//O21401
F-PLACE1007649//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//8.1e-06:197:26//SACCHAROMYCES CEREVISIAE (BAKER'S YEAST).//P08640
F-PLACE1007677//!!!! ALU SUBFAMILY SC WARNING ENTRY !!!!//1.0:47:46//HOMO SAPIENS (HUMAN).//P39192
F-PLACE1007688//LA PROTEIN HOMOLOG (LA RIBONUCLEOPROTEIN) (LA AUTOANTIGEN HOMOLOG).//2.7e-06:116:28//AEDES ALBOPICTUS (FOREST DAY MOSQUITO).//Q26457
F-PLACE1007690//SPERM PROTAMINE P1.//0.12:26:50//TACHYGLOSSUS ACULEATUS ACULEATUS (AUSTRALIAN ECHIDNA).//P35311
F-PLACE1007697//SPERM PROTAMINE P1.//0.19:34:52//DIDELPHIS MARSUPIALIS VIRGINIANA (NORTH AMERICAN OPOSSUM), AND MONODELPHIS DOMESTICA (SHORT-TAILED GREY OPOSSUM).//P35305
F-PLACE1007705//BIOH PROTEIN.//0.015:97:29//ESCHERICHIA COLI.//P13001
F-PLACE1007706//HYPOTHETICAL 112.2 KD PROTEIN IN TIF35-NPL3 INTERGENIC REGION (ORF1).//5.3e-55:190:56//SACCHAROMYCES CEREVISIAE (BAKER'S YEAST).//P32898
F-PLACE1007725
F-PLACE1007729//PROTEASE (EC 3.4.23.-).//1.8e-21:136:42//MOUSE MAMMARY TUMOR VIRUS (STRAIN BR6).//P10271
F-PLACE1007730//SALIVARY PROLINE-RICH PROTEIN II-1 (FRAGMENT).//0.0031:77:40//HOMO SAPIENS (HUMAN).//P81489
F-PLACE1007737//!!!! ALU SUBFAMILY SX WARNING ENTRY !!!!//0.78:39:56//HOMO SAPIENS (HUMAN).//P39195
F-PLACE1007743
F-PLACE1007746//RRP5 PROTEIN HOMOLOG (KIAA0185) (FRAGMENT).//0.0066:168:25//HOMO SAPIENS (HUMAN).//Q14690
F-PLACE1007791//KRUEPPEL PROTEIN (FRAGMENT).//0.62:17:41//LITHOBIUS FORFICATUS.//Q01872
F-PLACE1007807//HYPOTHETICAL 6.4 KD PROTEIN IN BLTR-SPOIIIC INTERGENIC REGION.//1.0:40:30//BACILLUS SUBTILIS.//P54446
F-PLACE1007810//ANTHOPLEURIN A (TOXIN AP-A).//0.79:28:46//ANTHOPLEURA XANTHOGRAMMICA (GIANT GREEN SEA ANEMONE).//P01530
F-PLACE1007829//SPORE COAT PROTEIN G.//1.0:65:38//BACILLUS SUBTILIS.//P39801
F-PLACE1007843
F-PLACE1007846//LINE-1 REVERSE TRANSCRIPTASE HOMOLOG.//1.5e-32:37:94//HOMO SAPIENS (HUMAN).//P08547
F-PLACE1007852//RHO-RELATED GTP-BINDING PROTEIN RHOH (GTP-BINDING PROTEIN TTF).//8.7e-05:138:30//HOMO SAPIENS (HUMAN).//Q15669
F-PLACE1007858//ANAPHASE SPINDLE ELONGATION PROTEIN.//0.0039:127:25//SACCHAROMYCES CEREVISIAE (BAKER'S YEAST).//P50275
F-PLACE1007866
F-PLACE1007877
F-PLACE1007897//CD44 ANTIGEN PRECURSOR (PHAGOCYTIC GLYCOPROTEIN I) (PGP-1) (HUTCH-I) (EXTRACELLULAR MATRIX RECEPTOR-III) (ECMR-III) (GP90 LYMPHOCYTE HOMING/ADHESION RECEPTOR) (HERMES ANTIGEN) (HYALURONATE RECEPTOR) (HEPARAN SULFATE PROTEOGLYCAN) (HAM1 ANTIGEN).//0.44:128:28//MESOCRICETUS AURATUS (GOLDEN HAMSTER).//Q60522
F-PLACE1007908//!!!! ALU SUBFAMILY SC WARNING ENTRY !!!!//5.5e-28:61:65//HOMO SAPIENS (HUMAN).//P39192
F-PLACE1007946//HYPOTHETICAL MERCURIC RESISTANCE PROTEIN MERC.//0.84:48:37//PSEUDOMONAS AERUGINOSA.//P04139
F-PLACE1007954//HYPOTHETICAL 45.5 KD PROTEIN IN FIG1-GIP1 INTERGENIC REGION.//0.00070:96:29//SACCHAROMYCES CEREVISIAE (BAKER'S YEAST).//P38226
F-PLACE1007955//HYPOTHETICAL 84.3 KD PROTEIN ZK945.10 IN CHROMOSOME II.//0.00027:255:23//CAENORHABDITIS ELEGANS.//Q09625
F-PLACE1007958//HIGH-AFFINITY CAMP-SPECIFIC 3',5'-CYCLIC PHOSPHODIESTERASE (EC 3.1.4.17).//1.7e-09:127:30//MUS MUSCULUS (MOUSE).//P70453
F-PLACE1007969//HYPOTHETICAL 24.1 KD PROTEIN IN LEF4-P33 INTERGENIC REGION.//2.4e-05:104:37//AUTOGRAPHA CALIFORNICA NUCLEAR POLYHEDROSIS VIRUS (ACMNPV).//P41479
F-PLACE1007990//SPERM PROTAMINE P1.//0.78:36:47//ORNITHORHYNCHUS ANATINUS (DUCKBILL PLATYPUS).//P35307
F-PLACE1008000//CHANNEL ASSOCIATED PROTEIN OF SYNAPSE-110 (CHAPSYN-110) (SYNAPTIC DENSITY PROTEIN PSD-93).//1.2e-16:128:39//RATTUS NORVEGICUS (RAT).//Q63622
F-PLACE1008002
F-PLACE1008044//NUCLEAR PORE COMPLEX PROTEIN NUP107 (NUCLEOPORIN NUP107) (107 KD NUCLEOPORIN) (P105).//3.9e-106:208:93//RATTUS NORVEGICUS (RAT).//P52590
F-PLACE1008045//COLLAGEN ALPHA 1(XII) CHAIN (FRAGMENTS).//3.9e-09:49:53//BOS TAURUS (BOVINE).//P25508
F-PLACE1008080//RNA REPLICASE POLYPROTEIN (EC 2.7.7.48).//0.00025:100:27//EGGPLANT MOSAIC VIRUS.//P20126
F-PLACE1008095//PROTOPORPHYRINOGEN OXIDASE (EC 1.3.3.4) (PPO).//0.90:74:25//MYCOBACTERIUM TUBERCULOSIS.//O53230
F-PLACE1008111//HYPOTHETICAL PROTEIN MJECS12.//0.30:38:42//METHANOCOCCUS JANNASCHII.//Q60311
F-PLACE1008122//PEA2 PROTEIN (PPF2 PROTEIN).//0.0085:117:34//SACCHAROMYCES CEREVISIAE (BAKER'S YEAST).//P40091
F-PLACE1008129//PROCOLLAGEN ALPHA 2(I) CHAIN PRECURSOR (FRAGMENTS).//1.8e-06:154:36//GALLUS GALLUS (CHICKEN).//P02467
F-PLACE1008132//HYPOTHETICAL 127.4 KD PROTEIN F07F6.4 IN CHROMOSOME III.//1.4e-13:227:36//CAENORHABDITIS ELEGANS.//Q09531
F-PLACE1008177//TRICHOHYALIN.//2.7e-10:230:26//OVIS ARIES (SHEEP).//P22793
F-PLACE1008181
F-PLACE1008198//SKIN SECRETORY PROTEIN XP2 PRECURSOR (APEG PROTEIN).//0.00044:121:34//XENOPUS LAEVIS (AFRICAN CLAWED FROG).//P17437
F-PLACE1008201//ZINC FINGER PROTEIN ZFMSA12A.//3.0e-05:82:37//MICROPTERUS SALMOIDES (LARGEMOUTH BASS).//P38621
F-PLACE1008209//METALLOTHIONEIN-I (MT-I).//0.95:39:35//CERCOPITHECUS AETHIOPS (GREEN MONKEY) (GRIVET).//P02797
F-PLACE1008231//PROCYCLIC FORM SPECIFIC POLYPEPTIDE B1-ALPHA PRECURSOR (PROCYCLIN) (PARP).//0.028:23:52//TRYPANOSOMA BRUCEI BRUCEI.//P08469
F-PLACE1008244//VEGETATIBLE INCOMPATIBILITY PROTEIN HET-E-1.//2.2e-23:148:38//PODOSPORA ANSERINA.//Q00808
F-PLACE1008273//COATOMER GAMMA SUBUNIT (GAMMA-COAT PROTEIN) (GAMMA-COP).//1.1e-97:222:81//BOS TAURUS (BOVINE).//P53620
F-PLACE1008275//DNA REPAIR PROTEIN REV1 (EC 2.7.7.-).//5.8e-20:161:37//SACCHAROMYCES CEREVISIAE (BAKER'S YEAST).//P12689
F-PLACE1008280//LINE-1 REVERSE TRANSCRIPTASE HOMOLOG.//6.1e-23:124:42//HOMO SAPIENS (HUMAN).//P08547
F-PLACE1008309//HYPOTHETICAL 98.3 KD PROTEIN C9G1.06C IN CHROMOSOME I.//0.47:99:37//SCHIZOSACCHAROMYCES POMBE (FISSION YEAST).//O14302
F-PLACE1008329//PUTATIVE Z PROTEIN.//0.73:52:28//OVIS ARIES (SHEEP).//P08105
F-PLACE1008330//!!!! ALU SUBFAMILY SQ WARNING ENTRY !!!!//9.0e-37:75:81//HOMO SAPIENS (HUMAN).//P39194
F-PLACE1008331//!!!! ALU SUBFAMILY J WARNING ENTRY !!!!//4.1e-08:70:50//HOMO SAPIENS (HUMAN).//P39188
F-PLACE1008356//FRUIT PROTEIN PKIWI501.//0.0037:148:29//ACTINIDIA CHINENSIS (KIWI) (YANGTAO).//P43393
F-PLACE1008368//RING CANAL PROTEIN (KELCH PROTEIN).//3.5e-18:205:30//DROSOPHILA MELANOGASTER (FRUIT FLY).//Q04652
F-PLACE1008369
F-PLACE1008392
F-PLACE1008398//GENE 33 POLYPEPTIDE.//1.5e-102:225:84//RATTUS NORVEGICUS (RAT).//P05432
F-PLACE1008401//PROLINE-RICH PROTEIN MP-3 (FRAGMENT).//2.9e-08:186:34//MUS MUSCULUS (MOUSE).//P05143
F-PLACE1008402//GENERAL VESICULAR TRANSPORT FACTOR P115 (TRANSCYTOSIS ASSOCIATED PROTEIN) (TAP).//9.4e-105:207:98//BOS TAURUS (BOVINE).//P41541
F-PLACE1008405
F-PLACE1008424//PROTEIN UL56.//1.0:65:33//HERPES SIMPLEX VIRUS (TYPE 1 / STRAIN HFEM).//P36297
F-PLACE1008426//MYOSIN HEAVY CHAIN, NON-MUSCLE (ZIPPER PROTEIN) (MYOSIN II).//4.4e-05:185:28//DROSOPHILA MELANOGASTER (FRUIT FLY).//Q99323
F-PLACE1008429//NEURONAL AXONAL MEMBRANE PROTEIN NAP-22.//0.00054:172:25//RATTUS NORVEGICUS (RAT).//Q05175
F-PLACE1008437//HYPOTHETICAL 115.4 KD PROTEIN ZK757.3 IN CHROMOSOME III.//1.9e-23:226:34//CAENORHABDITIS ELEGANS.//P34681
F-PLACE1008455//DNA-BINDING PROTEIN (AGNOPROTEIN).//0.97:23:52//BUDGERIGAR FLEDGLING DISEASE VIRUS (BFDV).//P13893
F-PLACE1008457//!!!! ALU SUBFAMILY J WARNING ENTRY !!!!//2.1e-12:89:47//HOMO SAPIENS (HUMAN).//P39188
F-PLACE1008465//ZINC FINGER PROTEIN 31 (ZINC FINGER PROTEIN KOX29) (FRAGMENT).//0.00017:23:43//HOMO SAPIENS (HUMAN).//P17040
F-PLACE1008488//HYPOTHETICAL PROTEIN UL61.//9.1e-05:204:30//HUMAN CYTOMEGALOVIRUS (STRAIN AD169).//P16818
F-PLACE1008524//HOMEOBOX PROTEIN HLX1 (HOMEOBOX PROTEIN HB24).//0.95:74:36//HOMO SAPIENS (HUMAN).//Q14774
F-PLACE1008531//!!!! ALU SUBFAMILY SC WARNING ENTRY !!!!//3.1e-05:86:45//HOMO SAPIENS (HUMAN).//P39192
F-PLACE1008532//HYPOTHETICAL 36.4 KD PROTEIN IN SMP1-MBA1 INTERGENIC REGION.//3.9e-21:62:45//SACCHAROMYCES CEREVISIAE (BAKER'S YEAST).//P38298
F-PLACE1008533//HYPOTHETICAL 86.2 KD PROTEIN C4G8.04 IN CHROMOSOME I.//3.5e-06:118:29//SCHIZOSACCHAROMYCES POMBE (FISSION YEAST).//Q09830
F-PLACE1008568//NEURONATIN.//0.046:34:52//HOMO SAPIENS (HUMAN).//Q16517
F-PLACE1008584//HUNCHBACK PROTEIN (FRAGMENT).//0.94:30:43//LITHOBIUS FORFICATUS.//Q02030
F-PLACE1008603//NUCLEAR PORE COMPLEX PROTEIN NUP155 (NUCLEOPORIN NUP155) (155 KD NUCLEOPORIN) (P140).//3.9e-123:224:96//RATTUS NORVEGICUS (RAT).//P37199
F-PLACE1008621//B-CELL GROWTH FACTOR PRECURSOR (BCGF-12 KD).//5.0e-05:31:67//HOMO SAPIENS (HUMAN).//P20931
F-PLACE1008625//DISAGREGIN (PLATELET AGGREGATION ACTIVATION INHIBITOR).//0.87:17:52//ORNITHODOROS MOUBATA (SOFT TICK).//P36235
F-PLACE1008626//METALLOTHIONEIN-I (MT-I).//0.77:33:36//SCYLLA SERRATA (MUD CRAB).//P02805
F-PLACE1008627//METALLOTHIONEIN-III (MT-III) (GROWTH INHIBITORY FACTOR) (GIF) (GIFB).//0.14:44:31//HOMO SAPIENS (HUMAN).//P25713
F-PLACE1008629
F-PLACE1008630//PROTAMINE Z3 (SCYLLIORHININE Z3).//0.78:33:36//SCYLIORHINUS CANICULA (SPOTTED DOGFISH) (SPOTTED CATSHARK).//P30258
F-PLACE1008643//INTER-ALPHA-TRYPSIN INHIBITOR HEAVY CHAIN H4 PRECURSOR (ITI HEAVY CHAIN H4) (INTER-ALPHA-TRYPSIN INHIBITOR FAMILY HEAVY CHAIN-RELATED PROTEIN) (PLASMA KALLIKREIN SENSITIVE GLYCOPROTEIN 120) (PK-120).//1.7e-30:220:41//HOMO SAPIENS (HUMAN).//Q14624
F-PLACE1008650//PP1/PP2A PHOSPHATASES PLEIOTROPIC REGULATOR PRL1.//2.5e-10:106:31//ARABIDOPSIS THALIANA (MOUSE-EAR CRESS).//Q42384
F-PLACE1008693//BOWMAN-BIRK TYPE PROTEINASE INHIBITOR (MSTI).//1.0:36:38//MEDICAGO SCUTELLATA (SNAIL MEDIC).//P80321
F-PLACE1008696//NADH-UBIQUINONE OXIDOREDUCTASE 23 KD SUBUNIT PRECURSOR (EC 1.6.5.3) (EC 1.6.99.3) (COMPLEX I-23KD) (CI-23KD) (TYKY SUBUNIT).//4.8e-14:47:80//HOMO SAPIENS (HUMAN).//O00217
F-PLACE1008715//HYPOTHETICAL 13.4 KD PROTEIN IN ACT5-YCK1 INTERGENIC REGION.//0.66:105:24//SACCHAROMYCES CEREVISIAE (BAKER'S YEAST).//P38834
F-PLACE1008748//HYPOTHETICAL 57.5 KD PROTEIN IN VMA7-RPS25A INTERGENIC REGION.//0.10:178:26//SACCHAROMYCES CEREVISIAE (BAKER'S YEAST).//P53214
F-PLACE1008757//HYPOTHETICAL 10.1 KD PROTEIN IN RHSD-GCL INTERGENIC REGION (ORFD3).//0.60:44:34//ESCHERICHIA COLI.//P33669
F-PLACE1008790//IMPORTIN ALPHA-6 SUBUNIT (KARYOPHERIN ALPHA-6 SUBUNIT) (IMPORTIN ALPHA S2).//3.0e-69:191:80//MUS MUSCULUS (MOUSE).//O35345
F-PLACE1008798//BACTERIOCIN LACTOBIN A.//1.0:34:41//LACTOBACILLUS AMYLOVORUS .//P80696
F-PLACE1008807//LINE-1 REVERSE TRANSCRIPTASE HOMOLOG.//0.91:77:36//HOMO SAPIENS (HUMAN).//P08547
F-PLACE1008808//REC1 PROTEIN.//0.45:39:30//USTILAGO MAYDIS (SMUT FUNGUS).//P14746
F-PLACE1008813
F-PLACE1008851//VERY HYPOTHETICAL 11.8 KD PROTEIN IN KTR3-DUR1,2 INTERGENIC REGION.//1.0:62:30//SACCHAROMYCES CEREVISIAE (BAKER'S YEAST).//P38309
F-PLACE1008854//HYPOTHETICAL 182.0 KD PROTEIN IN NMD5-HOM6 INTERGENIC REGION.//1.0:82:26//SACCHAROMYCES CEREVISIAE (BAKER'S YEAST).//P47170
F-PLACE1008867//PATATIN T5 PRECURSOR (POTATO TUBER PROTEIN).//0.65:61:36//SOLANUM TUBEROSUM (POTATO).//P15478
F-PLACE1008887//LINE-1 REVERSE TRANSCRIPTASE HOMOLOG.//1.5e-56:180:54//NYCTICEBUS COUCANG (SLOW LORIS).//P08548
F-PLACE1008902
F-PLACE1008920
F-PLACE1008925//HYPOTHETICAL 41.2 KD PROTEIN IN GAPA-RND INTERGENIC REGION.//0.90:77:33//ESCHERICHIA COLI.//P76242
F-PLACE1008934//HYPOTHETICAL PROTEIN IN ADHS 5'REGION (ORF3) (FRAGMENT).//0.14:77:45//GLUCONOBACTER SUBOXYDANS.//O05543
F-PLACE1008941//ZINC FINGER PROTEIN 141.//1.1e-17:45:95//HOMO SAPIENS (HUMAN).//Q15928
F-PLACE1008947//MAJOR CENTROMERE AUTOANTIGEN B (CENTROMERE PROTEIN B) (CENP-B).//4.1e-14:136:39//MUS MUSCULUS (MOUSE).//P27790
F-PLACE1009020//ANNEXIN VII (SYNEXIN) (FRAGMENT).//0.74:37:48//BOS TAURUS (BOVINE).//P20072
F-PLACE1009027//NADH-UBIQUINONE OXIDOREDUCTASE CHAIN 4L (EC 1.6.5.3).//0.39:57:36//BALAENOPTERA MUSCULUS (BLUE WHALE).//P41301
F-PLACE1009039
F-PLACE1009045//HYPOTHETICAL 9.5 KD PROTEIN IN SPEA-METK INTERGENIC REGION (F83).//0.48:32:43//ESCHERICHIA COLI.//P46879
F-PLACE1009048
F-PLACE1009050
F-PLACE1009060//HYPOTHETICAL 98.3 KD PROTEIN R10E12.1 IN CHROMOSOME III.//4.9e-23:244:31//CAENORHABDITIS ELEGANS.//P34552
F-PLACE1009090//50S RIBOSOMAL PROTEIN L35.//1.0:27:51//MYCOPLASMA GENITALIUM.//P47439
F-PLACE1009091
F-PLACE1009094//NEL-LIKE PROTEIN (FRAGMENT).//3.6e-15:180:30//HOMO SAPIENS (HUMAN).//Q92832
F-PLACE1009099//ZINC FINGER PROTEIN 27 (ZFP-27) (MKR4 PROTEIN) (FRAGMENT).//1.4e-94:228:71//MUS MUSCULUS (MOUSE).//P10077
F-PLACE1009110//HIRUDIN HV1 (BUFRUDIN).//1.0:49:34//HIRUDINARIA MANILLENSIS (BUFFALO LEECH).//P81492
F-PLACE1009111//!!!! ALU SUBFAMILY SX WARNING ENTRY !!!!//1.4e-05:30:83//HOMO SAPIENS (HUMAN).//P39195
F-PLACE1009113//ANNEXIN VII (SYNEXIN) (FRAGMENT).//0.032:40:52//BOS TAURUS (BOVINE).//P20072
F-PLACE1009130//HYPOTHETICAL PROTEIN KIAA0032.//3.3e-37:214:38//HOMO SAPIENS (HUMAN).//Q15034
F-PLACE1009150//!!!! ALU SUBFAMILY SX WARNING ENTRY !!!!//1.6e-32:56:76//HOMO SAPIENS (HUMAN).//P39195
F-PLACE1009155//!!!! ALU SUBFAMILY SQ WARNING ENTRY !!!!//1.2e-17:101:57//HOMO SAPIENS (HUMAN).//P39194
F-PLACE1009158//HYPOTHETICAL PROTEIN HKRFX (J1I).//0.0058:73:42//HUMAN CYTOMEGALOVIRUS (STRAIN AD169).//P09711
F-PLACE1009166//CYTOSOLIC PURINE 5'-NUCLEOTIDASE (EC 3.1.3.5).//0.0086:96:30//HOMO SAPIENS (HUMAN).//P49902
F-PLACE1009172//HYPOTHETICAL 8.7 KD PROTEIN IN GAPA-RND INTERGENIC REGION.//1.0:19:52//ESCHERICHIA COLI.//P76246
F-PLACE1009174//!!!! ALU SUBFAMILY SQ WARNING ENTRY !!!!//3.1e-17:47:82//HOMO SAPIENS (HUMAN).//P39194
F-PLACE1009183
F-PLACE1009186//HYPOTHETICAL 11.4 KD PROTEIN C13G6.04 IN CHROMOSOME I.//0.019:62:24//SCHIZOSACCHAROMYCES POMBE (FISSION YEAST).//Q09783
F-PLACE1009190//PALMITOYL-COA HYDROLASE (EC 3.1.2.2) (LONG-CHAIN FATTY-ACYL-COA HYDROLASE) (FRAGMENT).//0.027:53:28//RATTUS NORVEGICUS (RAT).//P80250
F-PLACE1009200//!!!! ALU SUBFAMILY SQ WARNING ENTRY !!!!//5.4e-28:84:71//HOMO SAPIENS (HUMAN).//P39194
F-PLACE1009230//!!!! ALU SUBFAMILY SB WARNING ENTRY !!!!//2.8e-12:50:74//HOMO SAPIENS (HUMAN).//P39189
F-PLACE1009246//UBIQUINOL-CYTOCHROME C REDUCTASE COMPLEX 7.8 KD PROTEIN (EC 1.10.2.2) (MITOCHONDRIAL HINGE PROTEIN) (CR7).//1.0:17:52//SOLANUM TUBEROSUM (POTATO).//P48504
F-PLACE1009298//VACUOLAR PROTEIN SORTING-ASSOCIATED PROTEIN VPS35.//6.6e-41:177:53//SACCHAROMYCES CEREVISIAE (BAKER'S YEAST).//P34110
F-PLACE1009308//KERATIN, ULTRA HIGH-SULFUR MATRIX PROTEIN (UHS KERATIN).//0.00034:108:33//HOMO SAPIENS (HUMAN).//P26371
F-PLACE1009319//PRESYNAPTIC DENSITY PROTEIN 95 (PSD-95).//5.3e-16:84:50//HOMO SAPIENS (HUMAN).//P78352
F-PLACE1009328//LINE-1 REVERSE TRANSCRIPTASE HOMOLOG.//6.9e-82:263:67//HOMO SAPIENS (HUMAN).//P08547
F-PLACE1009335//60S RIBOSOMAL PROTEIN L32.//0.95:71:36//HOMO SAPIENS (HUMAN), MUS MUSCULUS (MOUSE), AND RATTUS NORVEGICUS (RAT).//P02433
F-PLACE1009338//TRANSCRIPTION FACTOR HES-5 (HAIRY AND ENHANCER OF SPLIT 5).//0.90:42:40//MUS MUSCULUS (MOUSE).//P70120
F-PLACE1009368//BASIC PROLINE-RICH PEPTIDE IB-1.//0.013:33:48//HOMO SAPIENS (HUMAN).//P04281
F-PLACE1009375//HYPOTHETICAL 88.1 KD PROTEIN K02D10.1 IN CHROMOSOME III.//0.0022:135:21//CAENORHABDITIS ELEGANS.//P34492
F-PLACE1009388//!!!! ALU SUBFAMILY SX WARNING ENTRY !!!!//4.8e-22:73:65//HOMO SAPIENS (HUMAN).//P39195
F-PLACE1009398//ZINC FINGER PROTEIN 84 (ZINC FINGER PROTEIN HPF2).//8.1e-83:223:65//HOMO SAPIENS (HUMAN).//P51523
F-PLACE1009404//GLUTENIN, HIGH MOLECULAR WEIGHT SUBUNIT PW212 PRECURSOR.//0.047:145:29//TRITICUM AESTIVUM (WHEAT).//P08489
F-PLACE1009410//TOXIN C13S1C1 PRECURSOR.//0.22:21:47//DENDROASPIS ANGUSTICEPS (EASTERN GREEN MAMBA).//P18329
F-PLACE1009434//NADH-UBIQUINONE OXIDOREDUCTASE SUBUNIT K (EC 1.6.5.3) (FRAGMENT).//0.81:61:29//ANTHOCEROS FORMOSAE.//Q31791
F-PLACE1009443//SPLICEOSOME ASSOCIATED PROTEIN 62 (SAP 62) (SF3A66).//9.1e-05:93:32//MUS MUSCULUS (MOUSE).//Q62203
F-PLACE1009444//PHOSPHATIDYLINOSITOL 4-KINASE ALPHA (EC 2.7.1.67) (PI4-KINASE) (PTDINS-4-KINASE) (PI4K-ALPHA).//6.4e-15:41:97//HOMO SAPIENS (HUMAN).//P42356
F-PLACE1009459//HYPOTHETICAL 42.3 KD PROTEIN C12G12.11C IN CHROMOSOME I.//0.0011:119:31//SCHIZOSACCHAROMYCES POMBE (FISSION YEAST).//Q09874
F-PLACE1009468//PHOSPHOLIPASE A-2-ACTIVATING PROTEIN (PLAP).//4.2e-34:101:75//RATTUS NORVEGICUS (RAT).//P54319
F-PLACE1009476//DNA-BINDING P52/P100 COMPLEX, 100 KD SUBUNIT (FRAGMENTS).//0.086:21:52//HOMO SAPIENS (HUMAN).//P30808
F-PLACE1009477
F-PLACE1009493//HYPOTHETICAL 127.3 KD PROTEIN B0416.1 IN CHROMOSOME X.//1.4e-18:138:39//CAENORHABDITIS ELEGANS.//Q11069
F-PLACE1009524//ARF NUCLEOTIDE-BINDING SITE OPENER (ARNO PROTEIN) (ARF EXCHANGE FACTOR).//9.4e-80:155:85//HOMO SAPIENS (HUMAN).//Q99418
F-PLACE1009539//GTP-BINDING NUCLEAR PROTEIN RAN/TC4.//1.0:76:26//GIARDIA LAMBLIA (GIARDIA INTESTINALIS).//P38543
F-PLACE1009542//!!!! ALU SUBFAMILY J WARNING ENTRY !!!!//0.00016:31:77//HOMO SAPIENS (HUMAN).//P39188
F-PLACE1009571//ATP SYNTHASE B CHAIN (EC 3.6.1.34) (SUBUNIT I).//0.88:116:29//STREPTOCOCCUS PNEUMONIAE.//Q59952
F-PLACE1009581//50S RIBOSOMAL PROTEIN L32.//0.00023:37:51//RHODOBACTER CAPSULATUS (RHODOPSEUDOMONAS CAPSULATA).//P30788
F-PLACE1009595
F-PLACE1009596//HYPOTHETICAL 40.4 KD TRP-ASP REPEATS CONTAINING PROTEIN C14B1.4 IN CHROMOSOME III.//2.1e-36:116:49//CAENORHABDITIS ELEGANS.//Q17963
F-PLACE1009607//!!!! ALU SUBFAMILY J WARNING ENTRY !!!!//1.8e-43:73:69//HOMO SAPIENS (HUMAN).//P39188
F-PLACE1009613
F-PLACE1009621//TRANSCRIPTION FACTOR BTF3 HOMOLOG 2.//0.91:29:44//HOMO SAPIENS (HUMAN).//Q13891
F-PLACE1009622//MATERNAL EFFECT PROTEIN STAUFEN.//1.3e-22:132:47//DROSOPHILA MELANOGASTER (FRUIT FLY).//P25159
F-PLACE1009637//HYPOTHETICAL 18.1 KD PROTEIN IN CFXA 3'REGION.//0.30:28:57//BACTEROIDES VULGATUS.//P30905
F-PLACE1009639//LIPASE MODULATOR PRECURSOR (LIPASE HELPER PROTEIN).//0.23:79:31//PSEUDOMONAS AERUGINOSA.//Q04591
F-PLACE1009659//MEMBRANE-ASSOCIATED PROTEIN HEM-2 (BRAIN PROTEIN H19) (MH19) (FRAGMENT).//3.9e-126:227:96//MUS MUSCULUS (MOUSE).//P28660
F-PLACE1009665//IG KAPPA CHAIN V-I REGION (HAU).//0.52:89:35//HOMO SAPIENS (HUMAN).//P01600
F-PLACE1009670//CYCLOMALTODEXTRIN GLUCANOTRANSFERASE PRECURSOR (EC 2.4.1.19) (CYCLODEXTRIN-GLYCOSYLTRANSFERASE) (CGTASE).//0.16:114:29//PAENIBACILLUS MACERANS (BACILLUS MACERANS).//P31835
F-PLACE1009708//HYPOTHETICAL 143.3 KD TRP-ASP REPEATS CONTAINING PROTEIN C12G12.13C IN CHROMOSOME I.//9.6e-19:156:36//SCHIZOSACCHAROMYCES POMBE (FISSION YEAST).//Q09876
F-PLACE1009721//MSF1 PROTEIN.//7.7e-23:176:33//SACCHAROMYCES CEREVISIAE (BAKER'S YEAST).//P35200
F-PLACE1009731//AIG1 PROTEIN.//1.1e-09:91:43//ARABIDOPSIS THALIANA (MOUSE-EAR CRESS).//P54120
F-PLACE1009763//HYPOTHETICAL 48.9 KD PROTEIN C24H6.12C IN CHROMOSOME I.//8.3e-42:171:51//SCHIZOSACCHAROMYCES POMBE (FISSION YEAST).//Q09765
F-PLACE1009794//C-HORDEIN (CLONE PC HOR1-3) (FRAGMENT).//0.99:36:33//HORDEUM VULGARE (BARLEY).//P17991
F-PLACE1009798//HYPOTHETICAL PROTEIN C22F3.14C IN CHROMOSOME I (FRAGMENT).//2.6e-34:191:38//SCHIZOSACCHAROMYCES POMBE (FISSION YEAST).//Q09779
F-PLACE1009845//WEB1 PROTEIN (PROTEIN TRANSPORT PROTEIN SEC31).//2.2e-19:190:33//SACCHAROMYCES CEREVISIAE (BAKER'S YEAST).//P38968
F-PLACE1009861//CATHEPSIN B PRECURSOR (EC 3.4.22.1).//4.4e-20:171:33//BOS TAURUS (BOVINE).//P07688
F-PLACE1009879//HYPOTHETICAL 8.7 KD PROTEIN IN RPL22-RPL23 INTERGENIC REGION (ORF70).//0.99:30:33//ASTASIA LONGA (EUGLENOPHYCEAN ALGA).//P34779
F-PLACE1009886
F-PLACE1009888//NONSTRUCTURAL POLYPROTEIN [CONTAINS: NONSTRUCTURAL PROTEIN NSP4] (FRAGMENT).//1.0:33:42//WESTERN EQUINE ENCEPHALITIS VIRUS.//P13896
F-PLACE1009908//HYPOTHETICAL GTP-BINDING PROTEIN C3F10.16C IN CHROMOSOME I.//3.1e-42:205:46//SCHIZOSACCHAROMYCES POMBE (FISSION YEAST).//Q10190
F-PLACE1009921
F-PLACE1009924//ATP SYNTHASE A CHAIN (EC 3.6.1.34) (PROTEIN 6).//0.70:128:29//TRYPANOSOMA BRUCEI BRUCEI.//P24499
F-PLACE1009925//ATP SYNTHASE D CHAIN, MITOCHONDRIAL (EC 3.6.1.34).//0.99:111:27//SACCHAROMYCES CEREVISIAE (BAKER'S YEAST).//P30902
F-PLACE1009935//HYPOTHETICAL PROTEIN MJ0258.//0.063:75:32//METHANOCOCCUS JANNASCHII.//Q57706
F-PLACE1009947//NEUROGRANIN (NG) (P17) (B-50 IMMUNOREACTIVE C-KINASE SUBSTRATE) (BICKS) (FRAGMENT).//0.33:51:45//BOS TAURUS (BOVINE).//P35722
F-PLACE1009971//MIPP PROTEIN (MURINE IAP-PROMOTED PLACENTA-EXPRESSED PROTEIN).//0.022:84:27//MUS MUSCULUS (MOUSE).//P28575
F-PLACE1009992//BONE MORPHOGENETIC PROTEIN 1 PRECURSOR (EC 3.4.24.-) (BMP-1).//0.00011:35:51//HOMO SAPIENS (HUMAN).//P13497
F-PLACE1009995//TROPOMYOSIN, SMOOTH MUSCLE/FIBROBLAST CTM1.//0.052:185:22//CIONA INTESTINALIS.//Q07068
F-PLACE1009997//TRANSCRIPTION ELONGATION FACTOR S-II (RNA POLYMERASE II ELONGATION FACTOR DMS-II) (TFIIS).//0.68:98:28//DROSOPHILA MELANOGASTER (FRUIT FLY).//P20232
F-PLACE1010023//HYPOTHETICAL 83.8 KD PROTEIN C27F2.7 IN CHROMOSOME III.//6.6e-06:111:32//CAENORHABDITIS ELEGANS.//Q18262
F-PLACE1010031//HYPOTHETICAL 24.1 KD PROTEIN IN LEF4-P33 INTERGENIC REGION.//0.0024:72:33//AUTOGRAPHA CALIFORNICA NUCLEAR POLYHEDROSIS VIRUS (ACMNPV).//P41479
F-PLACE1010053//HYPOTHETICAL PROTEIN HI0593.//0.83:24:45//HAEMOPHILUS INFLUENZAE.//P44022
F-PLACE1010069
F-PLACE1010074//VACUOLAR PROTEIN SORTING-ASSOCIATED PROTEIN VPS5.//0.00027:192:28//SACCHAROMYCES CEREVISIAE (BAKER'S YEAST).//Q92331
F-PLACE1010076//HUNCHBACK PROTEIN (FRAGMENT).//0.80:39:30//SCIARA COPROPHILA (FUNGUS GNAT).//Q01790
F-PLACE1010083//RHO-GAP HEMATOPOIETIC PROTEIN C1 (P115) (KIAA0131).//2.7e-48:177:46//HOMO SAPIENS (HUMAN).//P98171
F-PLACE1010089//UBIQUITIN CARBOXYL-TERMINAL HYDROLASE 11 (EC 3.1.2.15) (UBIQUITIN THIOLESTERASE 11) (UBIQUITIN-SPECIFIC PROCESSING PROTEASE 13) (DEUBIQUITINATING ENZYME 11) (KIAA0055).//7.9e-07:55:43//HOMO SAPIENS (HUMAN).//P40818
F-PLACE1010096//100 KD PROTEIN (EC 6.3.2.-).//1.0e-107:232:90//RATTUS NORVEGICUS (RAT).//Q62671
F-PLACE1010102//DNA-DIRECTED RNA POLYMERASE SUBUNIT N (EC 2.7.7.6).//1.0:33:45//METHANOCOCCUS JANNASCHII.//Q57649
F-PLACE1010105//RING CANAL PROTEIN (KELCH PROTEIN).//1.2e-47:200:46//DROSOPHILA MELANOGASTER (FRUIT FLY).//Q04652
F-PLACE1010106//RETROVIRUS-RELATED POL POLYPROTEIN [CONTAINS: REVERSE TRANSCRIPTASE (EC 2.7.7.49); ENDONUCLEASE].//1.2e-14:94:41//MUS MUSCULUS (MOUSE).//P11369
F-PLACE1010134//HYPOTHETICAL 171.5 KD HELICASE IN NUT1-ARO2 INTERGENIC REGION.//4.0e-28:78:76//SACCHAROMYCES CEREVISIAE (BAKER'S YEAST).//P53115
F-PLACE1010148//GAR2 PROTEIN.//2.6e-05:180:26//SCHIZOSACCHAROMYCES POMBE (FISSION YEAST).//P41891
F-PLACE1010152//UBIQUITIN CARBOXYL-TERMINAL HYDROLASE 64E (EC 3.1.2.15) (UBIQUITIN THIOLESTERASE 64E) (UBIQUITIN-SPECIFIC PROCESSING PROTEASE 64E) (DEUBIQUITINATING ENZYME 64E).//2.1e-59:227:54//DROSOPHILA MELANOGASTER (FRUIT FLY).//Q24574
F-PLACE1010181//MALE SPECIFIC SPERM PROTEIN MST87F.//0.39:12:58//DROSOPHILA MELANOGASTER (FRUIT FLY).//P08175
F-PLACE1010194//SPLICING FACTOR, ARGININE/SERINE-RICH 2 (SPLICING FACTOR SC35) (SC-35) (SPLICING COMPONENT, 35 KD) (PR264 PROTEIN).//1.4e-07:95:43//GALLUS GALLUS (CHICKEN).//P30352
F-PLACE1010202//TRISTETRAPROLINE (TTP) (TIS11A) (TIS11) (ZFP-36).//0.094:109:29//RATTUS NORVEGICUS (RAT).//P47973
F-PLACE1010231//LANTIBIOTIC NISIN A PRECURSOR.//0.99:42:35//LACTOCOCCUS LACTIS (SUBSP. LACTIS) (STREPTOCOCCUS LACTIS).//P13068
F-PLACE1010261//SEGREGATION DISTORTER PROTEIN.//6.0e-71:201:62//DROSOPHILA MELANOGASTER (FRUIT FLY).//P25722
F-PLACE1010270
F-PLACE1010274//HYPOTHETICAL 16.2 KD PROTEIN C4F8.01 IN CHROMOSOME I.//4.4e-08:100:26//SCHIZOSACCHAROMYCES POMBE (FISSION YEAST).//O14177
F-PLACE1010293//!!!! ALU SUBFAMILY J WARNING ENTRY !!!!//3.9e-26:94:64//HOMO SAPIENS (HUMAN).//P39188
F-PLACE1010310//SYNAPSINS IA AND IB.//5.7e-09:89:37//RATTUS NORVEGICUS (RAT).//P09951
F-PLACE1010321//IMMEDIATE-EARLY PROTEIN IE180.//0.033:145:31//PSEUDORABIES VIRUS (STRAIN KAPLAN) (PRV).//P33479
F-PLACE1010324//MAST CELL DEGRANULATING PEPTIDE (MCDP) (MCD).//0.60:25:48//MEGABOMBUS PENNSYLVANICUS (AMERICAN COMMON BUMBLEBEE).//P04567
F-PLACE1010329//TOXIN S5C10.//1.0:39:33//DENDROASPIS JAMESONI KAIMOSAE (EASTERN JAMESON'S MAMBA).//P01419
F-PLACE1010341//!!!! ALU SUBFAMILY SB WARNING ENTRY !!!!//0.0049:49:55//HOMO SAPIENS (HUMAN).//P39189
F-PLACE1010362//VARIANT-SURFACE-GLYCOPROTEIN PHOSPHOLIPASE C (EC 3.1.4.47) (VSG LIPASE) (GLYCOSYLPHOSPHATIDYLINOSITOL-SPECIFIC PHOSPHOLIPASE C) (GPI-PLC).//0.0034:89:30//TRYPANOSOMA CRUZI.//015886
F-PLACE1010364//NADH-UBIQUINONE OXIDOREDUCTASE B17 SUBUNIT (EC 1.6.5.3) (EC 1.6.99.3) (COMPLEX I-B17) (CI-B17).//1.0:40:35//SUS SCROFA (PIG).//Q29259
F-PLACE1010383
F-PLACE1010401//140 KD NUCLEOLAR PHOSPHOPROTEIN (NOPP140).//0.10:174:22//RATTUS NORVEGICUS (RAT).//P41777
F-PLACE1010481//HYPOTHETICAL 71.9 KD PROTEIN B0285.5 IN CHROMOSOME III.//1.5e-21:170:35//CAENORHABDITIS ELEGANS.//P46555
F-PLACE1010491//HYPOTHETICAL 13.5 KD PROTEIN IN MOB1-SGA1 INTERGENIC REGION.//1.0:31:41//SACCHAROMYCES CEREVISIAE (BAKER'S YEAST).//P40490
F-PLACE1010492//HYPOTHETICAL 42.3 KD PROTEIN C12G12.11C IN CHROMOSOME I.//0.77:97:30//SCHIZOSACCHAROMYCES POMBE (FISSION YEAST).//Q09874
F-PLACE1010522//SMALL PROLINE RICH PROTEIN II (SPR-II) (CLONE 930).//0.74:45:37//HOMO SAPIENS (HUMAN).//P22531
F-PLACE1010529//DELTA 1-PYRROLINE-5-CARBOXYLATE SYNTHETASE (P5CS) [CONTAINS: GLUTAMATE 5-KINASE (EC 2.7.2.11) (GAMMA-GLUTAMYL KINASE) (GK); GAMMA-GLUTAMYL PHOSPHATE REDUCTASE (GPR) (EC 1.2.1.41) (GLUTAMATE-5-SEMIALDEHYDE DEHYDROGENASE) (GLUTAMYL-GAMMA-SEMIALDEHYDE DEHYDROGENASE)].//0.70:58:39//VIGNA ACONITIFOLIA (MOTHBEAN).//P32296
F-PLACE1010547//HYPOTHETICAL 31.0 KD PROTEIN IN BUD9-RME1 INTERGENIC REGION.//0.17:68:39//SACCHAROMYCES CEREVISIAE (BAKER'S YEAST).//P53227
F-PLACE1010562//CHLOROPLAST 50S RIBOSOMAL PROTEIN L33.//0.50:48:29//PORPHYRA PURPUREA.//P51255
F-PLACE1010579//HYPOTHETICAL PROTEIN HI1571.//0.29:37:43//HAEMOPHILUS INFLUENZAE.//P44260
F-PLACE1010580//PUTATIVE ATP-DEPENDENT RNA HELICASE C12C2.06.//3.3e-38:178:48//SCHIZOSACCHAROMYCES POMBE (FISSION YEAST).//Q09747
F-PLACE1010599//PEROXISOMAL MEMBRANE PROTEIN PER10 (PEROXIN-14).//4.6e-17:192:31//PICHIA ANGUSTA (YEAST) (HANSENULA POLYMORPHA).//P78723
F-PLACE1010616//HYPOTHETICAL 9.2 KD PROTEIN IN RNPA 3'REGION.//0.44:32:37//PSEUDOMONAS PUTIDA.//P25753
F-PLACE1010622//A-AGGLUTININ ATTACHMENT SUBUNIT PRECURSOR.//5.0e-06:102:42//SACCHAROMYCES CEREVISIAE (BAKER'S YEAST).//P32323
F-PLACE1010624//SALIVARY PROLINE-RICH PROTEIN PO (ALLELE K) [CONTAINS: PEPTIDE P-D] (FRAGMENT).//0.00036:134:321/HOMO SAPIENS (HUMAN).//P10162
F-PLACE1010628
F-PLACE1010629//!!!! ALU SUBFAMILY SQ WARNING ENTRY !!!!//2.7e-12:37:81//HOMO SAPIENS (HUMAN).//P39194
F-PLACE1010630
F-PLACE1010631//WNT-5B PROTEIN (FRAGMENT).//0.49:62:30//EUMECES SKILTONIANUS (WESTERN SKINK).//P28118
F-PLACE1010661//MATERNAL EXUPERANTIA 2 PROTEIN.//1.0:95:30//DROSOPHILA PSEUDOOBSCURA (FRUIT FLY).//Q24617
F-PLACE1010662//UDP-GLUCOSE:GLYCOPROTEIN GLUCOSYLTRANSFERASE PRECURSOR (EC 2.4.1.-) (DUGT).//3.2e-05:117:24//DROSOPHILA MELANOGASTER (FRUIT FLY).//Q09332
F-PLACE1010702//ZINC FINGER PROTEIN 195.//1.4e-62:117:62//HOMO SAPIENS (HUMAN).//O14628
F-PLACE1010714
F-PLACE1010720//CHROMOSOME ASSEMBLY PROTEIN XCAP-C.//1.1e-64:176:76//XENOPUS LAEVIS (AFRICAN CLAWED FROG).//P50532
F-PLACE1010739//TAT PROTEIN (TRANSACTIVATING REGULATORY PROTEIN) (FRAGMENT).//0.97:31:41//HUMAN IMMUNODEFICIENCY VIRUS TYPE 1 (BH5 ISOLATE) (HIV-1).//P04612
F-PLACE1010743//PROLINE-RICH PROTEIN MP-3 (FRAGMENT).//3.8e-05:253:30//MUS MUSCULUS (MOUSE).//P05143
F-PLACE1010761//HYPOTHETICAL 37.0 KD PROTEIN B0495.8 IN CHROMOSOME II.//1.5e-14:175:25//CAENORHABDITIS ELEGANS.//Q09217
F-PLACE1010771//TRANSCRIPTIONAL REGULATOR PROTEIN HCNGP.//1.3e-120:216:89//MUS MUSCULUS (MOUSE).//Q02614
F-PLACE1010786//CENTROSOMIN (ARROW PROTEIN).//0.97:133:24//DROSOPHILA MELANOGASTER (FRUIT FLY).//P54623
F-PLACE1010800//HYPOTHETICAL 31.7 KD PROTEIN IN TRAX-FINO INTERGENIC REGION (ORFC).//0.0060:111:31//ESCHERICHIA COLI.//Q99390
F-PLACE1010802//UREASE ACCESSORY PROTEIN UREI.//0.82:44:29//BACILLUS SP. (STRAIN TB-90).//Q07415
F-PLACE1010811//CYTOCHROME C-551 (C551).//0.99:42:38//ECTOTHIORHODOSPIRA HALOCHLORIS.//P38587
F-PLACE1010833//CALTRACTIN, ISOFORM 1 (CENTRIN).//2.8e-09:90:34//HOMO SAPIENS (HUMAN).//P41208
F-PLACE1010856//MOLT-INHIBITING HORMONE (MIH).//1.0:32:37//PROCAMBARUS CLARKII (RED SWAMP CRAYFISH).//P55848
F-PLACE1010857//IG ALPHA-1 CHAIN C REGION.//0.49:73:34//GORILLA GORILLA GORILLA (LOWLAND GORILLA).//P20758
F-PLACE1010870//ZINC FINGER PROTEIN 91 (ZINC FINGER PROTEIN HTF10) (HPF7).//1.2e-56:173:58//HOMO SAPIENS (HUMAN).//Q05481
F-PLACE1010877//HEAT SHOCK PROTEIN 82.//0.13:130:25//ZEA MAYS (MAIZE).//Q08277
F-PLACE1010891//HYPOTHETICAL 8.2 KD PROTEIN IN BLTR-SPOIIIC INTERGENIC REGION.//0.95:51:27//BACILLUS SUBTILIS.//P54436
F-PLACE1010896//SERINE/THREONINE-PROTEIN KINASE PTK1/STK1 (EC 2.7.1.).//0.98:71:30//SACCHAROMYCES CEREVISIAE (BAKER'S YEAST).//P36002
F-PLACE1010900//HYPOTHETICAL PROTEIN HI0840.//1.0:42:30//HAEMOPHILUS INFLUENZAE.//P44897
F-PLACE1010916//KERATIN, HIGH-SULFUR MATRIX PROTEIN, IIIB3.//0.060:59:35//OVIS ARIES (SHEEP).//P02444
F-PLACE1010917//E2 GLYCOPROTEIN PRECURSOR (SPIKE GLYCOPROTEIN) (PEPLOMER PROTEIN).//0.71:141:24//BOVINE CORONAVIRUS (STRAIN L9).//P25191
F-PLACE1010925//HYPOTHETICAL 8.1 KD PROTEIN.//1.0:17:58//THERMOPROTEUS TENAX VIRUS 1 (STRAIN KRA1) (TTV1).//P19285
F-PLACE1010926//HYPOTHETICAL PROLINE-RICH PROTEIN KIAA0269.//0.011:51:45//HOMO SAPIENS (HUMAN).//Q92558
F-PLACE1010942//EPIDERMAL GROWTH FACTOR RECEPTOR SUBSTRATE SUBSTRATE 15 (PROTEIN EPS15).//3.1e-09:64:37//MUS MUSCULUS (MOUSE).//P42567
F-PLACE1010944//GAP JUNCTION ALPHA-3 PROTEIN (CONNEXIN 44) (CX44).//0.17:71:38//BOS TAURUS (BOVINE).//P41987
F-PLACE1010947
F-PLACE1010954//TROPOMYOSIN ALPHA CHAIN, SKELETAL MUSCLE.//0.011:144:26//HOMO SAPIENS (HUMAN).//P09493
F-PLACE1010960//ACTIN-LIKE PROTEIN 13E.//1.1 e-60:136:52//DROSOPHILA MELANOGASTER (FRUIT FLY).//P45890
F-PLACE1010965
F-PLACE1011026//PERIOD CLOCK PROTEIN (FRAGMENT).//1.0:64:31//DROSOPHILA ANANASSAE (FRUIT FLY).//Q03293
F-PLACE1011032//RIBONUCLEASE HI (EC 3.1.26.4) (RNASE HI) (RIBONUCLEASE H) (RNASE H).//1.0:32:37//SALMONELLA TYPHIMURIUM.//P23329
F-PLACE1011041//HOMEOBOX PROTEIN VAB-7.//0.36:65:30//CAENORHABDITIS ELEGANS.//Q93899
F-PLACE1011046//1-PHOSPHATIDYLINOSITOL-4,5-BISPHOSPHATE PHOSPHODIESTERASE BETA 1 (EC 3.1.4.11) (PLC-BETA-1) (PHOSPHOLIPASE C-BETA-1) (PLC-I) (PLC-154).//1.3e-22:58:93//RATTUS NORVEGICUS (RAT).//P10687
F-PLACE1011054//!!!! ALU SUBFAMILY SX WARNING ENTRY !!!!//1.6e-07:38:73//HOMO SAPIENS (HUMAN).//P39195
F-PLACE1011056//HISTONE H1.//2.2e-10:109:41//PISUM SATIVUM (GARDEN PEA).//P08283
F-PLACE1011057
F-PLACE1011090//HYPOTHETICAL 33.8 KD PROTEIN IN TWT1-FLO5 INTERGENIC REGION.//1.8e-07:133:32//SACCHAROMYCES CEREVISIAE (BAKER'S YEAST).//P38892
F-PLACE1011109//ELONGATION FACTOR G, MITOCHONDRIAL PRECURSOR (MEF-G).//5.4e-25:63:88//RATTUS NORVEGICUS (RAT).//Q07803
F-PLACE1011114//PUTATIVE ATP-DEPENDENT RNA HELICASE C1F7.02C.//8.4e-31:157:45//SCHIZOSACCHAROMYCES POMBE (FISSION YEAST).//Q09916
F-PLACE1011133//SERUM AMYLOID P-COMPONENT PRECURSOR (SAP) (9.5S ALPHA-1-GLYCOPROTEIN).//0.92:58:31//HOMO SAPIENS (HUMAN).//P02743
F-PLACE1011143//PROBABLE E5 PROTEIN.//0.24:42:35//HUMAN PAPILLOMAVIRUS TYPE31.//P17385
F-PLACE1011160//EARLY NODULIN 55-2 PRECURSOR (N-55-2) (NODULIN-315).//0.88:98:27//GLYCINE MAX (SOYBEAN).//Q02917
F-PLACE1011165//HISTIDINE-RICH PROTEIN.//0.013:13:76//PLASMODIUM FALCIPARUM (ISOLATE FCM17 / SENEGAL).//P14586
F-PLACE1011185//!!!! ALU SUBFAMILY J WARNING ENTRY !!!!//1.4e-13:98:50//HOMO SAPIENS (HUMAN).//P39188
F-PLACE1011203
F-PLACE1011214//ATP SYNTHASE PROTEIN 8 (EC 3.6.1.34) (A6L).//1.0:48:27//EQUUS ASINUS (DONKEY).//P92479
F-PLACE1011219//PROBABLE OXIDOREDUCTASE (EC 1.-.-.-).//1.9e-15:162:31//STREPTOMYCES ANTIBIOTICUS.//Q03326
F-PLACE1011221//ANTITHROMBIN-III HOMOLOG.//0.84:74:33//FOWLPOX VIRUS (ISOLATE HP-438[MUNICH]).//P14369
F-PLACE1011229//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN HOMOLOG).//3.5e-86:218:68//HOMO SAPIENS (HUMAN).//Q13107
F-PLACE1011263//ANKYRIN, BRAIN VARIANT 2 (ANKYRIN B) (ANKYRIN, NONERYTHROID) (FRAGMENT).//3.0e-07:99:36//HOMO SAPIENS (HUMAN).//Q01485
F-PLACE1011273
F-PLACE1011291//PROTEIN KINASE C SUBSTRATE 80 KD PROTEIN (FRAGMENTS).//0.011:36:50//RATTUS NORVEGICUS (RAT).//P20468
F-PLACE1011296//HOMEOBOX PROTEIN DLX-6.//0.76:55:32//BRACHYDANIO RERIO (ZEBRAFISH) (ZEBRA DANIO).//Q98877
F-PLACE1011310//ATP SYNTHASE PROTEIN 9, MITOCHONDRIAL (EC 3.6.1.34) (LIPID-BINDING PROTEIN).//0.46:43:44//PETUNIA SP. (PETUNIA).//Q07060
F-PLACE1011325//HYPOTHETICAL 222.8 KD PROTEIN C1F3.06C IN CHROMOSOME I.//0.00021:171:27//SCHIZOSACCHAROMYCES POMBE (FISSION YEAST).//Q10411
F-PLACE1011332//DNA-DAMAGE-REPAIR/TOLERATION PROTEIN DRT101 PRECURSOR.//7.3e-27:113:52//ARABIDOPSIS THALIANA (MOUSE-EAR CRESS).//Q05211
F-PLACE1011340//!!!! ALU SUBFAMILY J WARNING ENTRY !!!!//2.6e-07:40:62//HOMO SAPIENS (HUMAN).//P39188
F-PLACE1011371//INTER-ALPHA-TRYPSIN INHIBITOR HEAVY CHAIN H2 PRECURSOR (ITI HEAVY CHAIN H2).//2.2e-54:227:44//MUS MUSCULUS (MOUSE).//Q61703
F-PLACE1011375//PROBABLE E5 PROTEIN.//0.93:28:57//HUMAN PAPILLOMAVIRUS TYPE 51.//P26553
F-PLACE1011399//HISTONE H2B-IV.//0.19:129:27//VOLVOX CARTERI.//P16868
F-PLACE1011419
F-PLACE1011433//ZINC FINGER PROTEIN GLI3 (FRAGMENT).//3.4e-05:133:24//GALLUS GALLUS (CHICKEN).//P55879
F-PLACE1011452//LINE-1 REVERSE TRANSCRIPTASE HOMOLOG.//3.9e-25:76:63//HOMO SAPIENS (HUMAN).//P08547
F-PLACE1011465//ECTODERMAL DYSPLASIA PROTEIN (EDA PROTEIN).//0.97:36:41//HOMO SAPIENS (HUMAN).//Q92838
F-PLACE1011472//METALLOTHIONEIN-1 (CUMT-1).//0.084:55:30//HOMARUS AMERICANUS (AMERICAN LOBSTER).//P29499
F-PLACE1011477//CELL SURFACE GLYCOPROTEIN 1 PRECURSOR (OUTER LAYER PROTEIN B) (S-LAYER PROTEIN 1).//0.028:129:34//CLOSTRIDIUM THERMOCELLUM.//Q06852
F-PLACE1011492//NON-GREEN PLASTID TRIOSE PHOSPHATE TRANSLOCATOR PRECURSOR (CTPT).//2.9e-13:147:31//BRASSICA OLERACEA (CAULIFLOWER).//P52178
F-PLACE1011503//PUTATIVE FERREDOXIN-LIKE PROTEIN IN PURL-DPJ INTERGENIC REGION (086).//0.66:32:40//ESCHERICHIA COLI.//P52102
F-PLACE1011520
F-PLACE1011563//LORICRIN.//0.00023:112:39//HOMO SAPIENS (HUMAN).//P23490
F-PLACE1011567//!!!! ALU SUBFAMILY SX WARNING ENTRY !!!!//9.2e-31:78:76//HOMO SAPIENS (HUMAN).//P39195
F-PLACE1011576//ZINC FINGER PROTEIN 91 (ZINC FINGER PROTEIN HTF10) (HPF7).//1.5e-32:45:86//HOMO SAPIENS (HUMAN).//Q05481
F-PLACE1011586//N-TYPE CALCIUM CHANNEL ALPHA-1B SUBUNIT (OMEGA-CONOTOXIN-SENSITIVE N- TYPE, BRAIN CALCIUM CHANNEL ALPHA-1 SUBUNIT).//0.26:81:37//HOMO SAPIENS (HUMAN).//Q00975
F-PLACE1011635//IMMEDIATE-EARLY PROTEIN IE180.//0.00045:170:30//PSEUDORABIES VIRUS (STRAIN INDIANA-FUNKHAUSER /BECKER) (PRV).//P11675
F-PLACE1011641
F-PLACE1011643//CUTICLE COLLAGEN 40.//1.0:128:32//CAENORHABDITIS ELEGANS.//P34804
F-PLACE1011646//!!!! ALU SUBFAMILY J WARNING ENTRY !!!!//1.1e-15:44:63//HOMO SAPIENS (HUMAN).//P39188
F-PLACE1011649//HYPOTHETICAL PROTEIN F-215.//0.48:106:34//HUMAN ADENOVIRUS TYPE 2.//P03291
F-PLACE1011650
F-PLACE1011664//CROOKED NECK PROTEIN.//1.2e-79:201:68//DROSOPHILA MELANOGASTER (FRUIT FLY).//P17886
F-PLACE1011675//FERREDOXIN.//1.0:44:29//METHANOCOCCUS THERMOLITHOTROPHICUS.//P21305
F-PLACE1011682//HYPOTHETICAL 7.0 KD PROTEIN IN RPS26A-COX4 INTERGENIC REGION.//1.0:40:22//SACCHAROMYCES CEREVISIAE (BAKER'S YEAST).//P53098
F-PLACE1011719//NEUROTOXIN TX2-6.//0.90:31:35//PHONEUTRIA NIGRIVENTER (BRAZILIAN ARMED SPIDER).//P29425
F-PLACE1011725//NUCLEOBINDIN PRECURSOR (NUCB1) (BONE 63 KD CALCIUM-BINDING PROTEIN).//0.0065:125:25//RATTUS NORVEGICUS (RAT).//Q63083
F-PLACE1011729//SRY-RELATED PROTEIN LG27 (FRAGMENT).//0.97:48:39//EUBLEPHARIS MACULARIUS.//P40654
F-PLACE1011749
F-PLACE1011762//D-BINDING PROTEIN (DBP) (ALBUMIN D BOX-BINDING PROTEIN).//0.028:91:39//MUS MUSCULUS (MOUSE).//Q60925
F-PLACE1011778
F-PLACE1011783//EMBRYONIC GROWTH/DIFFERENTIATION FACTOR 1 PRECURSOR (GDF-1).//0.97:48:43//MUS MUSCULUS (MOUSE).//P20863
F-PLACE1011858//COLLAGEN 1(X) CHAIN PRECURSOR.//0.0027:154:33//BOS TAURUS (BOVINE).//P23206
F-PLACE1011874//BACTERIOCHLOROPHYLL A PROTEIN (BCHL A PROTEIN) (BCP).//1.0:60:26//PROSTHECOCHLORIS AESTUARII.//P11741
F-PLACE1011875//HYPOTHETICAL 6.6 KD PROTEIN IN GP54-ALT INTERGENIC REGION.//0.99:34:35//ACTERIOPHAGE T4.//P39495
F-PLACE1011891//SMOOTHELIN.//0.018:122:31//HOMO SAPIENS (HUMAN).//P53814
F-PLACE1011896//SKIN SECRETORY PROTEIN XP2 PRECURSOR (APEG PROTEIN).//6.3e-09:203:35//XENOPUS LAEVIS (AFRICAN CLAWED FROG).//P17437
F-PLACE1011922//CRYPTDIN-RELATED PROTEIN 4C-2 PRECURSOR (CRS4C).//0.067:37:48//MUS MUSCULUS (MOUSE).//P50715
F-PLACE1011923//SERINE/THREONINE-PROTEIN KINASE SNK (EC 2.7.1.-) (SERUM INDUCIBLE KINASE).//1.5e-83:175:89//MUS MUSCULUS (MOUSE).//P53351
F-PLACE1011962//MATING-TYPE PHEROMONE BAP1(2) PRECURSOR.//0.50:46:41//SCHIZOPHYLLUM COMMUNE (BRACKET FUNGUS).//Q02593
F-PLACE1011964//LINE-1 REVERSE TRANSCRIPTASE HOMOLOG.//1.6e-05:47:51//NYCTICEBUS COUCANG (SLOW LORIS).//P08548
F-PLACE1011982//APICAL MEMBRANE ANTIGEN 1 PRECURSOR (MEROZOITE SURFACE ANTIGEN).//0.98:83:31//PLASMODIUM FRAGILE.//P22622
F-PLACE1011995
F-PLACE1012031//HYPOTHETICAL PROTEIN KIAA0254.//0.032:62:33//HOMO SAPIENS (HUMAN).//Q92543
F-PLACE2000003//!!!! ALU SUBFAMILY SP WARNING ENTRY !!!!//5.4e-18:63:73//HOMO SAPIENS (HUMAN).//P39193
F-PLACE2000006//ANNEXIN VII (SYNEXIN) (FRAGMENT).//0.14:20:50//BOS TAURUS (BOVINE).//P20072
F-PLACE2000007//PROLINE-RICH PROTEIN MP-3 (FRAGMENT).//0.0045:176:30//MUS MUSCULUS (MOUSE).//P05143
F-PLACE2000011//!!!! ALU SUBFAMILY SQ WARNING ENTRY !!!!//3.6e-25:57:78//HOMO SAPIENS (HUMAN).//P39194
F-PLACE2000014//HYPOTHETICAL HELICASE C28H8.3 IN CHROMOSOME III.//0.00013:237:27//CAENORHABDITIS ELEGANS.//Q09475
F-PLACE2000015//!!!! ALU SUBFAMILY SP WARNING ENTRY !!!!//1.4e-33:60:80//HOMO SAPIENS (HUMAN).//P39193
F-PLACE2000017//FOLATE RECEPTOR BETA PRECURSOR (FR-BETA) (FOLATE RECEPTOR 2) (FOLATE RECEPTOR, FETAL/PLACENTAL) (PLACENTAL FOLATE-BINDING PROTEIN) (FBP).//1.0:83:31//HOMO SAPIENS (HUMAN).//P14207
F-PLACE2000021//EPHRIN TYPE-A RECEPTOR 4 PRECURSOR (EC 2.7.1.112) (TYROSINE-PROTEIN KINASE RECEPTOR CEK8).//0.99:103:26//GALLUS GALLUS (CHICKEN).//Q07496
F-PLACE2000030//MALE SPECIFIC SPERM PROTEIN MST84DA.//0.69:29:44//DROSOPHILA MELANOGASTER (FRUIT FLY).//Q01642
F-PLACE2000033//PROBABLE OXIDOREDUCTASE (EC 1.-.-.-).//1.1e-05:74:41//STREPTOMYCES ANTIBIOTICUS.//Q03326
F-PLACE2000034//AXONIN-1 PRECURSOR (AXONAL GLYCOPROTEIN TAG-1) (TRANSIENT AXONAL GLYCOPROTEIN 1).//6.7e-18:191:35//HOMO SAPIENS (HUMAN).//Q02246
F-PLACE2000039//DYNEIN HEAVY CHAIN, CYTOSOLIC (DYHC) (MAP 1C).//4.7e-80:163:96//RATTUS NORVEGICUS (RAT).//P38650
F-PLACE2000047//!!!! ALU SUBFAMILY SB2 WARNING ENTRY !!!!//6.4e-06:63:49//HOMO SAPIENS (HUMAN).//P39191
F-PLACE2000050//!!!! ALU SUBFAMILY SC WARNING ENTRY !!!!//3.2e-22:74:64//HOMO SAPIENS (HUMAN).//P39192
F-PLACE2000061
F-PLACE2000062//GLUCOSE STARVATION-INDUCIBLE PROTEIN B (GENERAL STRESS PROTEIN B).//1.9e-06:108:37//BACILLUS SUBTILIS.//P26907
F-PLACE2000072//ZINC FINGER PROTEIN 165.//3.5e-34:175:49//HOMO SAPIENS (HUMAN).//P49910
F-PLACE2000097//RIBONUCLEASE PANCREATIC (EC 3.1.27.5) (RNASE 1) (RNASE A).//0.36:39:38//ONDATRA ZIBETHICUS (MUSKRAT).//P00681
F-PLACE2000100
F-PLACE2000103//TUBULIN ALPHA-4 CHAIN (FRAGMENTS).//0.18:32:37//ZEA MAYS (MAIZE).//P33626
F-PLACE2000111//CMRF35 ANTIGEN PRECURSOR.//0.056:107:27//HOMO SAPIENS (HUMAN).//Q08708
F-PLACE2000115//DIAMINOPIMELATE EPIMERASE (EC 5.1.1.7) (DAP EPIMERASE) (FRAGMENT).//1.0:21:52//CLOSTRIDIUM PERFRINGENS.//Q46185
F-PLACE2000124//!!!! ALU SUBFAMILY SQ WARNING ENTRY !!!!//3.4e-37:108:68//HOMO SAPIENS (HUMAN).//P39194
F-PLACE2000132//PROBABLE MEMBRANE ANTIGEN GP85.//0.99:133:29//EPSTEIN-BARR VIRUS (STRAIN B95-8) (HUMAN HERPESVIRUS 4).//P03224
F-PLACE2000136//VASOACTIVE INTESTINAL POLYPEPTIDE RECEPTOR 2 PRECURSOR (VIP-R-2) (PITUITARY ADENYLATE CYCLASE ACTIVATING POLYPEPTIDE TYPE III RECEPTOR) (PACAP TYPE III RECEPTOR) (PACAP-R-3).//0.83:65:32//MUS MUSCULUS (MOUSE).//P41588
F-PLACE2000140
F-PLACE2000164//TIPD PROTEIN.//5.7e-12:190:28//DICTYOSTELIUM DISCOIDEUM (SLIME MOLD).//O15736
F-PLACE2000170//BACTERIOCIN CARNOBACTERIOCIN BM1 PRECURSOR (CARNOBACTERIOCIN B1).//1.0:30:26//CARNOBACTERIUM PISCICOLA.//P38579
F-PLACE2000172
F-PLACE2000176//HYPOTHETICAL PROTEIN AF0526.//0.76:44:43//ARCHAEOGLOBUS FULGIDUS.//O29724
F-PLACE2000187//EM-LIKE PROTEIN GEA6.//0.84:42:35//ARABIDOPSIS THALIANA (MOUSE-EAR CRESS).//Q02973
F-PLACE2000216
F-PLACE2000223//NEUROTOXIN III (LQQ III).//0.99:38:34//LEIURUS QUINQUESTRIATUS QUINQUESTRIATUS (EGYPTIAN SCORPION).//P01487
F-PLACE2000235
F-PLACE2000246//RING CANAL PROTEIN (KELCH PROTEIN).//5.1e-37:121:42//DROSOPHILA MELANOGASTER (FRUIT FLY).//Q04652
F-PLACE2000264//!!!! ALU SUBFAMILY SB2 WARNING ENTRY !!!!//2.4e-05:77:42//HOMO SAPIENS (HUMAN).//P39191
F-PLACE2000274//DYNEIN BETA CHAIN, CILIARY.//5.3e-46:232:45//TRIPNEUSTES GRATILLA (HAWAIAN SEA URCHIN).//P23098
F-PLACE2000302//TRICHOHYALIN.//1.5e-06:215:29//ORYCTOLAGUS CUNICULUS (RABBIT).//P37709
F-PLACE2000305//!!!! ALU SUBFAMILY J WARNING ENTRY !!!!//5.3e-06:33:66//HOMO SAPIENS (HUMAN).//P39188
F-PLACE2000317//TOXIN C13S1C1 PRECURSOR.//0.44:45:33//DENDROASPIS ANGUSTICEPS (EASTERN GREEN MAMBA).//P18329
F-PLACE2000335//!!!! ALU SUBFAMILY SX WARNING ENTRY !!!!//7.9e-08:35:71//HOMO SAPIENS (HUMAN).//P39195
F-PLACE2000341//SODIUM/GLUCOSE COTRANSPORTER 1 (NA(+)/GLUCOSE COTRANSPORTER 1) (HIGH AFFINITY SODIUM-GLUCOSE COTRANSPORTER).//0.014:141:24//ORYCTOLAGUS CUNICULUS (RABBIT).//P11170
F-PLACE2000342//HYPOTHETICAL 24.1 KD PROTEIN IN LEF4-P33 INTERGENIC REGION.//5.7e-09:96:38//AUTOGRAPHA CALIFORNICA NUCLEAR POLYHEDROSIS VIRUS (ACMNPV).//P41479
F-PLACE2000347//ZINC FINGER PROTEIN 177.//5.9e-05:49:53//HOMO SAPIENS (HUMAN).//Q13360
F-PLACE2000359//!!!! ALU SUBFAMILY SQ WARNING ENTRY !!!!//7.5e-10:69:52//HOMO SAPIENS (HUMAN).//P39194
F-PLACE2000366
F-PLACE2000371//ATROPHIN-1 (DENTATORUBRAL-PALLIDOLUYSIAN ATROPHY PROTEIN).//1.5e-05:216:29//HOMO SAPIENS (HUMAN).//P54259
F-PLACE2000373//MAX BINDING PROTEIN MNT (ROX PROTEIN) (MYC ANTAGONIST MNT).//0.27:63:33//HOMO SAPIENS (HUMAN).//Q99583
F-PLACE2000379//HYPOTHETICAL GENE 1 PROTEIN.//0.72:120:31//EQUINE HERPESVIRUS TYPE 1 (STRAIN AB4P) (EHV-1).//P28978
F-PLACE2000394//BASIC PROLINE-RICH PEPTIDE P-E (IB-9).//0.95:40:42//HOMO SAPIENS (HUMAN).//P02811
F-PLACE2000398//RIBONUCLEASE PRECURSOR (EC 3.1.27.-).//0.88:88:31//AEROMONAS HYDROPHILA.//Q07465
F-PLACE2000399//T-CELL SURFACE GLYCOPROTEIN E2 PRECURSOR (E2 ANTIGEN) (CD99) (MIC2 PROTEIN) (12E7).//7.6e-16:180:39//HOMO SAPIENS (HUMAN).//P14209
F-PLACE2000404//PROBABLE LEUCYL-TRNA SYNTHETASE (EC 6.1.1.4) (LEUCINE-TRNA LIGASE) (LEURS).//1.7e-94:243:64//CAENORHABDITIS ELEGANS.//Q09996
F-PLACE2000411//SERINE/THREONINE PROTEIN PHOSPHATASE 5 (EC 3.1.3.16) (PP5) (PROTEIN PHOSPHATASE T) (PPT) (FRAGMENT).//1.2e-09:78:39//MUS MUSCULUS (MOUSE).//Q60676
F-PLACE2000419//!!!! ALU SUBFAMILY J WARNING ENTRY !!!!//2.6e-20:61:62//HOMO SAPIENS (HUMAN).//P39188
F-PLACE2000425//HYPOTHETICAL 11.9 KD PROTEIN IN MSB2-UGA1 INTERGENIC REGION.//0.98:75:32//SACCHAROMYCES CEREVISIAE (BAKER'S YEAST).//P53211
F-PLACE2000427//INSULIN PRECURSOR.//0.98:55:34//CERCOPITHECUS AETHIOPS (GREEN MONKEY) (GRIVET).//P30407
F-PLACE2000433//!!!! ALU SUBFAMILY J WARNING ENTRY !!!!//7.5e-07:65:50//HOMO SAPIENS (HUMAN).//P39188
F-PLACE2000435
F-PLACE2000438//HYPOTHETICAL 67.9 KD PROTEIN ZK688.8 IN CHROMOSOME III.//4.7e-66:178:47//CAENORHABDITIS ELEGANS.//P34678
F-PLACE2000450//!!!! ALU SUBFAMILY SX WARNING ENTRY !!!!//2.1e-23:88:62//HOMO SAPIENS (HUMAN).//P39195
F-PLACE2000455//TOXIN II (TOXIN II.10.9.2) (FRAGMENT).//0.093:18:44//CENTRUROIDES LIMPIDUS LIMPIDUS (MEXICAN SCORPION).//P45630
F-PLACE2000458//CADHERIN-RELATED TUMOR SUPPRESSOR PRECURSOR (FAT PROTEIN).//3.1e-23:165:40//DROSOPHILA MELANOGASTER (FRUIT FLY).//P33450
F-PLACE2000465//!!!! ALU SUBFAMILY J WARNING ENTRY !!!!//3.6e-23:73:63//HOMO SAPIENS (HUMAN).//P39188
F-PLACE2000477//!!!! ALU SUBFAMILY SQ WARNING ENTRY !!!!//4.4e-37:90:78//HOMO SAPIENS (HUMAN).//P39194
F-PLACE3000004//EYES ABSENT HOMOLOG 3.//1.1e-09:27:100//MUS MUSCULUS (MOUSE).//P97480
F-PLACE3000009//PUTATIVE CUTICLE COLLAGEN C09G5.6.//0.0061:148:34//CAENORHABDITIS ELEGANS.//Q09457
F-PLACE3000020//ADENYLATE CYCLASE, OLFACTIVE TYPE (EC 4.6.1.1) (TYPE III) (ATP PYROPHOSPHATE-LYASE) (ADENYLYL CYCLASE).//8.8e-93:193:92//RATTUS NORVEGICUS (RAT).//P21932
F-PLACE3000029//50S RIBOSOMAL PROTEIN L31E.//0.15:50:38//METHANOCOCCUS JANNASCHII.//P54009
F-PLACE3000059//TCP1-CHAPERONIN COFACTOR A.//0.96:50:34//BOS TAURUS (BOVINE).//P48427
F-PLACE3000070//HYPOTHETICAL 17.1 KD PROTEIN IN PUR5 3'REGION.//0.29:22:59//SACCHAROMYCES CEREVISIAE (BAKER'S YEAST).//P38898
F-PLACE3000103//LYSIS PROTEIN (E PROTEIN) (GPE).//0.99:53:32//BACTERIOPHAGE ALPHA-3.//P31280
F-PLACE3000119//!!!! ALU SUBFAMILY SB WARNING ENTRY !!!!//5.4e-41:87:78//HOMO SAPIENS (HUMAN).//P39189
F-PLACE3000121//VESICULAR TRAFFIC CONTROL PROTEIN SEC151/1.0e-07:269:22//SACCHAROMYCES CEREVISIAE (BAKER'S YEAST).//P22224
F-PLACE3000124//!!!! ALU SUBFAMILY J WARNING ENTRY !!!!//1.2e-29:97:73//HOMO SAPIENS (HUMAN).//P39188
F-PLACE3000136//PARS INTERCEREBRALIS MAJOR PEPTIDE D1 (PMP-D1).//0.77:26:42//LOCUSTA MIGRATORIA (MIGRATORY LOCUST).//P80059
F-PLACE3000142//HYPOTHETICAL 7.1 KD PROTEIN IN NAD2 3'REGION (ORF 63).//0.82:34:41//MARCHANTIA POLYMORPHA (LIVERWORT).//P38468
F-PLACE3000145//TENSIN.//3.5e-91:238:74//GALLUS GALLUS (CHICKEN).//Q04205
F-PLACE3000147//!!!! ALU SUBFAMILY SQ WARNING ENTRY !!!!//4.4e-30:61:65//HOMO SAPIENS (HUMAN).//P39194
F-PLACE3000148//POL POLYPROTEIN [CONTAINS: PROTEASE (EC 3.4.23.-); REVERSE TRANSCRIPTASE (EC 2.7.7.49); ENDONUCLEASE].//1.4e-18:226:34//GIBBON APE LEUKEMIA VIRUS.//P21414
F-PLACE3000155//EXTENSIN PRECURSOR (PROLINE-RICH GLYCOPROTEIN).//0.00014:107:33//ZEA MAYS (MAIZE).//P14918
F-PLACE3000156//POL POLYPROTEIN [CONTAINS: PROTEASE (EC 3.4.23.-); REVERSE TRANSCRIPTASE (EC 2.7.7.49); ENDONUCLEASE].//2.7e-19:169:30//BABOON ENDOGENOUS VIRUS (STRAIN M7).//P10272
F-PLACE3000157//PROBABLE SERINE/THREONINE-PROTEIN KINASE CY50.16 (EC 2.7.1.-).//0.0061:92:30//MYCOBACTERIUM TUBERCULOSIS.//Q11053
F-PLACE3000158//!!!! ALU SUBFAMILY SB WARNING ENTRY !!!!//5.7e-49:56:80//HOMO SAPIENS (HUMAN).//P39189
F-PLACE3000160//DNA TRANSFORMATION PROTEIN TFOX (COMPETENCE ACTIVATOR) (PROTEIN SXY).//0.39:94:34//HAEMOPHILUS INFLUENZAE.//P43779
F-PLACE3000169//!!!! ALU SUBFAMILY SP WARNING ENTRY !!!!//5.6e-28:99:59//HOMO SAPIENS (HUMAN).//P39193
F-PLACE3000194//PROLINE-RICH PROTEIN LAS17.//0.91:80:36//SACCHAROMYCES CEREVISIAE (BAKER'S YEAST).//Q12446
F-PLACE3000197//NEUROFILAMENT TRIPLET M PROTEIN (160 KD NEUROFILAMENT PROTEIN) (NF-M).//0.24:119:32//GALLUS GALLUS (CHICKEN).//P16053
F-PLACE3000199//EXTENSIN PRECURSOR (CELL WALL HYDROXYPROLINE-RICH GLYCOPROTEIN).//0.76:87:37//NICOTIANA TABACUM (COMMON TOBACCO).//P13983
F-PLACE3000207//!!!! ALU SUBFAMILY J WARNING ENTRY !!!!//4.5e-09:32:78//HOMO SAPIENS (HUMAN).//P39188
F-PLACE3000208
F-PLACE3000218//!!!! ALU SUBFAMILY SQ WARNING ENTRY !!!!//7.2e-34:96:70//HOMO SAPIENS (HUMAN).//P39194
F-PLACE3000220//OSTEOCALCIN (GAMMA-CARBOXYGLUTAMIC ACID-CONTAINING PROTEIN) (BONE GLA- PROTEIN) (BGP).//0.46:13:53//CANIS FAMILIARIS (DOG).//P81455
F-PLACE3000221//!!!! ALU SUBFAMILY J WARNING ENTRY !!!!//2.8e-24:178:45//HOMO SAPIENS (HUMAN).//P39188
F-PLACE3000226//30S RIBOSOMAL PROTEIN S18.//0.98:38:34//NEISSERIA GONORRHOEAE.//O07815
F-PLACE3000230//METALLOTHIONEIN (MT).//0.97:25:48//OREOCHROMIS MOSSAMBICUS (MOZAMBIQUE TILAPIA) (TILAPIA MOSSAMBICA).//P52726
F-PLACE3000242//MELANOMA-ASSOCIATED ANTIGEN 8 (MAGE-8 ANTIGEN).//8.0e-21:121:39//HOMO SAPIENS (HUMAN).//P43361
F-PLACE3000244//PROTEIN TSG24 (MEIOTIC CHECK POINT REGULATOR).//2.3e-125:264:87//MUS MUSCULUS (MOUSE).//P53995
F-PLACE3000254//RTOA PROTEIN (RATIO-A).//0.99:142:23//DICTYOSTELIUM DISCOIDEUM (SLIME MOLD).//P54681
F-PLACE3000271//!!!! ALU SUBFAMILY J WARNING ENTRY !!!!//2.2e-12:63:53//HOMO SAPIENS (HUMAN).//P39188
F-PLACE3000276//COLLAGEN ALPHA 1(VIII) CHAIN PRECURSOR (ENDOTHELIAL COLLAGEN).//1.0:55:38//HOMO SAPIENS (HUMAN).//P27658
F-PLACE3000304//DNA-BINDING P52/P100 COMPLEX, 100 KD SUBUNIT (FRAGMENTS).//0.0028:31:54//HOMO SAPIENS (HUMAN).//P30808
F-PLACE3000310//ATROPHIN-1 (DENTATORUBRAL-PALLIDOLUYSIAN ATROPHY PROTEIN).//0.98:82:34//RATTUS NORVEGICUS (RAT).//P54258
F-PLACE3000320
F-PLACE3000322//GLYCINE-RICH CELL WALL STRUCTURAL PROTEIN 1 PRECURSOR.//2.2e-22:61:52//ORYZA SATIVA (RICE).//P25074
F-PLACE3000331//SMALL PROLINE-RICH PROTEIN II (SPR-II) (CLONE 174N).//0.32:15:53//HOMO SAPIENS (HUMAN).//P22532
F-PLACE3000339//CHORION PROTEIN S19.//0.34:89:37//DROSOPHILA VIRILIS (FRUIT FLY).//P24516
F-PLACE3000341//NADH-UBIQUINONE OXIDOREDUCTASE CHAIN 1 (EC 1.6.5.3) (FRAGMENT).//1.0:47:38//COTURNIX COTURNIX JAPONICA (JAPANESE QUAIL).//P24968
F-PLACE3000350//SERINE/THREONINE-PROTEIN KINASE SULU (EC 2.7.1.-).//3.9e-50:168:60//CAENORHABDITIS ELEGANS .//P46549
F-PLACE3000352//!!!! ALU SUBFAMILY SQ WARNING ENTRY !!!!//7.8e-29:76:71//HOMO SAPIENS (HUMAN).//P39194
F-PLACE3000353//POLYPEPTTOE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N- ACETYLGALACTOSAMINYLTRANSFERASE) (GALNAC-T1).//3.0e-09:100:41//HOMO SAPIENS (HUMAN).//Q10472
F-PLACE3000362//HYPOTHETICAL PROTEIN TP0064.//1.0:75:26//TREPONEMA PALLIDUM.//O83103
F-PLACE3000363//METALLOTHIONEIN (MT).//0.067:42:33//ASTACUS FLUVIATILIS (BROAD-FINGERED CRAYFISH) (ASTACUS ASTACUS).//P55951
F-PLACE3000365//LYSIS PROTEIN (E PROTEIN) (GPE).//1.0:65:27//BACTERIOPHAGE PHI-K.//Q38040
F-PLACE3000373//RETROVIRUS-RELATED ENV POLYPROTEIN.//1.5e-18:90:47//HOMO SAPIENS (HUMAN).//P10267
F-PLACE3000388
F-PLACE3000399//!!!!ALU SUBFAMILY SP WARNING ENTRY !!!!//6.3e-45:60:75//HOMO SAPIENS (HUMAN).//P39193
F-PLACE3000400
F-PLACE3000401//!!!! ALU SUBFAMILY J WARNING ENTRY !!!!//3.6e-09:46:73//HOMO SAPIENS (HUMAN).//P39188
F-PLACE3000402//!!!! ALU SUBFAMILY J WARNING ENTRY !!!!//0.036:43:44//HOMO SAPIENS (HUMAN).//P39188
F-PLACE3000405//POSTERIOR PITUITARY PEPTIDE.//0.70:25:40//BOS TAURUS (BOVINE).//P01154
F-PLACE3000406//!!!! ALU SUBFAMILY SX WARNING ENTRY !!!!//4.3e-09:49:67//HOMO SAPIENS (HUMAN).//P39195
F-PLACE3000413//MALE SPECIFIC SPERM PROTEIN MST87F.//0.12:42:40//DROSOPHILA MELANOGASTER (FRUIT FLY).//P08175
F-PLACE3000416//CYLICIN I (MULTIPLE-BAND POLYPEPTIDE I).//0.67:236:21//BOS TAURUS (BOVINE).//P35662
F-PLACE3000425//PROLINE-RICH PEPTIDE P-B.//0.45:19:42//HOMO SAPIENS (HUMAN).//P02814
F-PLACE3000455//AMELOGENIN, CLASS I PRECURSOR.//0.0073:81:43//BOS TAURUS (BOVINE).//P02817
F-PLACE3000475//8.6 KD TRANSGLUTAMINASE SUBSTRATE.//1.0:53:32//TACHYPLEUS TRIDENTATUS (JAPANESE HORSESHOE CRAB).//P81281
F-PLACE3000477//MUSCARINIC TOXIN 7 (MT-7).//0.13:55:32//DENDROASPIS ANGUSTICEPS (EASTERN GREEN MAMBA).//P80970
F-PLACE4000009//MYOSIN HEAVY CHAIN, SMOOTH MUSCLE ISOFORM (SMMHC) (FRAGMENT).//7.0e-19:180:27//HOMO SAPIENS (HUMAN).//P35749
F-PLACE4000014//X-LINKED HELICASE II (X-LINKED NUCLEAR PROTEIN) (XNP).//3.2e-15:193:30//HOMO SAPIENS (HUMAN).//P46100
F-PLACE4000034//BRIDE OF SEVENLESS PROTEIN PRECURSOR.//0.0024:97:29//DROSOPHILA MELANOGASTER (FRUIT FLY).//P22815
F-PLACE4000049//!!!! ALU SUBFAMILY SQ WARNING ENTRY !!!!//3.8e-32:79:75//HOMO SAPIENS (HUMAN).//P39194
F-PLACE4000052//ATP-BINDING CASSETTE TRANSPORTER 1.//2.2e-99:178:97//MUS MUSCULUS (MOUSE).//P41233
F-PLACE4000063//IMMEDIATE-EARLY PROTEIN.//0.0017:159:25//HERPESVIRUS SAIMIRI (STRAIN 11).//Q01042
F-PLACE4000089
F-PLACE4000093
F-PLACE4000100//!!!! ALU SUBFAMILY J WARNING ENTRY !!!!//1.5e-14:68:60//HOMO SAPIENS (HUMAN).//P39188
F-PLACE4000106//1A PROTEIN [CONTAINS: HELICASE; METHYLTRANSFERASE].//1.0:46:41//BROAD BEAN MOTTLE VIRUS.//Q00020
F-PLACE4000128//HYPOTHETICAL PROTEIN E-115.//0.00020:101:30//HUMAN ADENOVIRUS TYPE 2.//P03290
F-PLACE4000129//CORNIFIN B (SMALL PROLINE-RICH PROTEIN IB) (SPR-IB) (14.9 KD PANCORNULIN).//0.15:57:31//HOMO SAPIENS (HUMAN).//P22528
F-PLACE4000131
F-PLACE4000147//COMPETENCE PHEROMONE PRECURSOR.//1.0:45:24//BACILLUS SUBTILIS.//P45453
F-PLACE4000156//ZINC FINGER PROTEIN 136.//2.1e-88:194:59//HOMO SAPIENS (HUMAN).//P52737
F-PLACE4000192//ZINC FINGER PROTEIN 142 (KIAA0236) (HA4654).//0.083:148:26//HOMO SAPIENS (HUMAN).//P52746
F-PLACE4000211//CALPHOTIN.//0.20:43:39//DROSOPHILA MELANOGASTER (FRUIT FLY).//Q02910
F-PLACE4000222//!!!! ALU SUBFAMILY J WARNING ENTRY !!!!//1.1e-05:20:85//HOMO SAPIENS (HUMAN).//P39188
F-PLACE4000230//DIHYDROFOLATE REDUCTASE (EC 1.5.1.3) / THYMIDYLATE SYNTHASE (EC 2.1.1.45) (DHFR-TS).//1.0:96:28//TRYPANOSOMA BRUCEI BRUCEI.//Q27783
F-PLACE4000233
F-PLACE4000247//METALLOTHIONEIN (MT).//1.0e-05:34:41//PLEURONECTES PLATESSA (PLAICE).//P07216
F-PLACE4000250//VPU PROTEIN (ORF-X PROTEIN) (UPX PROTEIN).//0.99:33:42//CAPRINE ARTHRITIS ENCEPHALITIS VIRUS (CAEV).//P31834
F-PLACE4000252//MALE SPECIFIC SPERM PROTEIN MST84DB.//0.42:24:45//DROSOPHILA MELANOGASTER (FRUIT FLY).//Q01643
F-PLACE4000259//PRE-MRNA SPLICING HELICASE BRR2 (EC 3.6.1.-).//3.5e-09:189:32//SACCHAROMYCES CEREVISIAE (BAKER'S YEAST).//P32639
F-PLACE4000261//PEREGRIN (BR140 PROTEIN).//5.0e-11:103:37//HOMO SAPIENS (HUMAN).//P55201
F-PLACE4000269//INTRACELLULAR PROTEIN TRANSPORT PROTEIN USO1.//0.037:181:25//SACCHAROMYCES CEREVISIAE (BAKER'S YEAST).//P25386
F-PLACE4000270//COAGULATION FACTOR VII PRECURSOR (EC 3.4.21.21).//1.0:46:39//MUS MUSCULUS (MOUSE).//P70375
F-PLACE4000300//50S RIBOSOMAL PROTEIN L32.//0.81:28:46//THERMUS AQUATICUS (SUBSP. THERMOPHILUS).//P80339
F-PLACE4000320//FKBP-RAPAMYCIN ASSOCIATED PROTEIN (FRAP) (RAPAMYCIN TARGET PROTEIN).//1.6e-29:44:93//HOMO SAPIENS (HUMAN).//P42345
F-PLACE4000323
F-PLACE4000326//PARATHYMOSIN.//0.0018:54:48//HOMO SAPIENS (HUMAN).//P20962
F-PLACE4000344//EPIDERMAL GROWTH FACTOR (EGF) (FRAGMENT).//0.97:28:42//SUS SCROFA (PIG).//Q00968
F-PLACE4000367//NEUROTOXIN 1 (TOXIN SHP-I) (SHNA) (NEUROTOXIN SHI).//1.0:33:36//STOICHACTIS HELIANTHUS (CARRIBEAN SEA ANEMONE) (STICHODACTYLA HELIANTHUS).//P19651
F-PLACE4000369//EXTENSIN PRECURSOR (PROLINE-RICH GLYCOPROTEIN).//0.071:42:42//SORGHUM VULGARE (SORGHUM).//P24152
F-PLACE4000379//!!!! ALU SUBFAMILY SP WARNING ENTRY !!!!//1.4e-16:54:77//HOMO SAPIENS (HUMAN).//P39193
F-PLACE4000387//PHOTOSYSTEM II 4 KD REACTION CENTRE PROTEIN PRECURSOR.//0.25:21:52//HORDEUM VULGARE (BARLEY), AND SECALE CEREALE (RYE).//P25877
F-PLACE4000392//FERROCHELATASE (EC 4.99.1.1) (PROTOHEME FERRO-LYASE) (HEME SYNTHETASE) (FRAGMENT).//0.91:36:50//YERSINIA PSEUDOTUBERCULOSIS.//Q05338
F-PLACE4000401//!!!! ALU SUBFAMILY SQ WARNING ENTRY !!!!//4.4e-29:96:67//HOMO SAPIENS (HUMAN).//P39194
F-PLACE4000411//!!!! ALU SUBFAMILY J WARNING ENTRY !!!!//2.3e-18:41:73//HOMO SAPIENS (HUMAN).//P39188
F-PLACE4000431//PRE-MRNA SPLICING HELICASE BRR2 (EC 3.6.1.-).//5.4e-21:237:33//SACCHAROMYCES CEREVISIAE (BAKER'S YEAST).//P32639
F-PLACE4000445//HYPOTHETICAL 99.7 KD PROTEIN IN SDL1 5'REGION PRECURSOR.//0.00081:210:26//SACCHAROMYCES CEREVISIAE (BAKER'S YEAST).//P40442
F-PLACE4000450//TRANSCRIPTION FACTOR HBP-1A (HISTONE-SPECIFIC TRANSCRIPTION FACTOR HBP1).//0.020:87:33//TRITICUM AESTIVUM (WHEAT).//P23922
F-PLACE4000465//METALLOTHIONEIN-IL (MT-1L) (MT1X).//0.20:18:38//HOMO SAPIENS (HUMAN).//P80297
F-PLACE4000487//!!!! ALU SUBFAMILY J WARNING ENTRY !!!!//1.5e-19:73:52//HOMO SAPIENS (HUMAN).//P39188
F-PLACE4000489
F-PLACE4000494//NPC DERIVED PROLINE RICH PROTEIN 1 (NDPP-1).//0.17:130:30//MUS MUSCULUS (MOUSE).//Q03173
F-PLACE4000521//RETROVIRUS-RELATED POL POLYPROTEIN [CONTAINS REVERSE TRANSCRIPTASE (EC 2.7.7.49); ENDONUCLEASE] (FRAGMENT).//3.0e-05:50:36//MUS MUSCULUS (MOUSE).//P10400
F-PLACE4000522//NEUROGENIC LOCUS NOTCH HOMOLOG PROTEIN 1 PRECURSOR.//1.8e-45:231:47//RATTUS NORVEGICUS (RAT).//Q07008
F-PLACE4000548//CYTOCHROME C-551 (C551).//0.96:50:34//ECTOTHIORHODOSPIRA HALOPHILA.//P00122
F-PLACE4000558//PROBABLE UBIQUITIN CARBOXYL-TERMINAL HYDROLASE FAF (EC 3.1.2.15) (UBIQUITIN THIOLESTERASE FAF) (UBIQUITIN-SPECIFIC PROCESSING PROTEASE FAF) (DEUBIQUITINATING ENZYME FAF) (FAT FACETS PROTEIN).//1.6e-28:223:36//DROSOPHILA MELANOGASTER (FRUIT FLY).//P55824
F-PLACE000581//P-SELECTIN PRECURSOR (GRANULE MEMBRANE PROTEIN 140) (GMP-140) (PADGEM) (CD62P) (LEUKOCYTE-ENDOTHELIAL CELL ADHESION MOLECULE 3) (LECAM3).//9.7e-11:166:281/HOMO SAPIENS (HUMAN).//P16109
F-PLACE4000590//POL POLYPROTEIN [CONTAINS: PROTEASE (EC 3.4.23.-); REVERSE TRANSCRIPTASE (EC 2.7.7.49); ENDONUCLEASE].//1.6e-17:134:35//GIBBON APE LEUKEMIA VIRUS.//P21414
F-PLACE4000593//GONADOTROPIN-RELEASING HORMONE RECEPTOR (GNRH-R).//1.0:54:29//RATTUS NORVEGICUS (RAT).//P30969
F-PLACE4000612//GAG POLYPROTEIN [CONTAINS: CORE PROTEIN P15; INNER COAT PROTEIN P12; CORE SHELL PROTEIN P30].//2.6e-14:221:32//MOLONEY MURINE SARCOMA VIRUS (STRAIN TS110).//P32594
F-PLACE4000638//HYPOTHETICAL 9.3 KD PROTEIN IN NRDB-INAA INTERGENIC REGION.//0.65:37:40//ESCHERICHIA COLI.//P37910
F-PLACE4000650//ZINC FINGER PROTEIN 16 (ZINC FINGER PROTEIN KOX9) (FRAGMENT).//1.0:33:33//HOMO SAPIENS (HUMAN).//P17020
F-PLACE4000654
F-PLACE4000670//HYPOTHETICAL 44.1 KD PROTEIN IN RPB5-CDC28 INTERGENIC REGION.//1.6e-07:161:25//SACCHAROMYCES CEREVISIAE (BAKER'S YEAST).//P33313
F-SKNMC1000011//PUTATIVE IMPORTIN BETA-4 SUBUNIT (KARYOPHERIN BETA-4 SUBUNIT).//7.4e-15:223:31//SCHIZOSACCHAROMYCES POMBE (FISSION YEAST).//O60100
F-SKNMC1000013//TRANSCRIPTION FACTOR BF-2 (BRAIN FACTOR 2) (BF2) (CBF-2) (T-14-6).//0.0013:128:35//GALLUS GALLUS (CHICKEN).//Q98937
F-SKNMC1000046//CUTICLE COLLAGEN 1.//0.0010:154:33//CAENORHABDITIS ELEGANS.//P08124
F-SKNMC1000050//CALPAIN 2, LARGE [CATALYTIC] SUBUNIT (EC 3.4.22.17) (CALCIUM-ACTIVATED NEUTRAL PROTEINASE) (CANP) (M-TYPE).//3.2e-41:87:98//HOMO SAPIENS (HUMAN).//P17655
F-SKNMC1000091//NTAK PROTEIN (NEURAL- AND THYMUS- DERIVED ACTIVATOR FOR ERBB KINASES).//0.0032:154:35//HOMO SAPIENS (HUMAN).//O14511
F-THYRO1000017//PUTATIVE PYRIDOXAMINE 5'-PHOSPHATE OXIDASE (EC 1.4.3.5) (PNP/PMP OXIDASE).//1.6e-23:124:37//CAENORHABDITIS ELEGANS.//Q20939
F-THYRO1000026//!!!! ALU SUBFAMILY SC WARNING ENTRY !!!!//3.0e-13:54:66//HOMO SAPIENS (HUMAN).//P39192
F-THYRO1000034//HYPOTHETICAL 10.4 KD PROTEIN.//0.16:44:34//HEPATITIS B VIRUS (SUBTYPE AYW).//P03163
F-THYRO1000035//CAMPATH-1 ANTIGEN PRECURSOR (CD52 ANTIGEN) (CDW52) (CAMBRIDGE PATHOLOGY 1 ANTIGEN).//0.83:59:37//MACACA FASCICULARIS (CRAB EATING MACAQUE) (CYNOMOLGUS MONKEY).//P32763
F-THYRO1000040//60S RIBOSOMAL PROTEIN L37 (FRAGMENT).//0.25:23:39//BOS TAURUS (BOVINE).//P79244
F-THYRO1000070//HYPOTHETICAL 29.3 KD PROTEIN (ORF92).//2.3e-11:133:36//ORGYIA PSEUDOTSUGATA MULTICAPSID POLYHEDROSIS VIRUS (OPMNPV).//O10341
F-THYRO1000072//C-PROTEIN, SKELETAL MUSCLE SLOW-ISOFORM.//1.5e-14:205:29//HOMO SAPIENS (HUMAN).//Q00872
F-THYRO1000085
F-THYRO1000092//SPERM MITOCHONDRIAL CAPSULE SELENOPROTEIN (MCS).//0.063:59:33//HOMO SAPIENS (HUMAN).//P49901
F-THYRO1000107
F-THYRO1000111//LINE-1 REVERSE TRANSCRIPTASE HOMOLOG.//5.0e-58:110:67//NYCTICEBUS COUCANG (SLOW LORIS).//P08548
F-THYRO1000121//SPLICEOSOME ASSOCIATED PROTEIN 62 (SAP 62) (SF3A66).//2.6e-06:134:35//MUS MUSCULUS (MOUSE).//Q62203
F-THYRO1000124//TENECIN 3 PRECURSOR.//0.047:76:35//TENEBRIO MOLITOR (YELLOW MEALWORM).//Q27270
F-THYRO1000129//FBROSIN (FRAGMENT).//0.35:43:34/MUS MUSCULUS (MOUSE).//Q60791
F-THYRO1000132//!!!! ALU SUBFAMILY J WARNING ENTRY !!!!//8.7e-14:104:42//HOMO SAPIENS (HUMAN).//P39188
F-THYRO1000156
F-THYRO1000163//!!!! ALU SUBFAMILY SB WARNING ENTRY !!!!//3.7e-20:71:71//HOMO SAPIENS (HUMAN).//P39189
F-THYRO1000173//CLATHRIN COAT ASSEMBLY PROTEIN AP47 (CLATHRIN COAT ASSOCIATED PROTEIN AP47) (GOLGI ADAPTOR AP-1 47 KD PROTEIN) (HA1 47 KD SUBUNIT) (CLATHRIN ASSEMBLY PROTEIN ASSEMBLY PROTEIN COMPLEX 1 MEDIUM CHAIN).//6.7e-88:216:76//MUS MUSCULUS (MOUSE).//P35585
F-THYRO1000186//!!!! ALU SUBFAMILY SC WARNING ENTRY !!!!//2.9e-24:72:77//HOMO SAPIENS (HUMAN).//P39192
F-THYRO1000187
F-THYRO1000190//PROTEIN TRANSPORT PROTEIN SEC61 BETA 2 SUBUNIT.//0.060:50:42//SACCHAROMYCES CEREVISIAE (BAKER'S YEAST).//P52871
F-THYRO1000197
F-THYRO1000199//HYPOTHETICAL 49.8 KD PROTEIN D2007.5 IN CHROMOSOME III.//2.0e-06:88:35//CAENORHABDITIS ELEGANS.//34379
F-THYRO1000206
F-THYRO1000221
F-THYRO1000241//HYPOTHETICAL 11.8 KD PROTEIN IN HE65-PK2 INTERGENIC REGION.//1.0:51:35//AUTOGRAPHA CALIFORNICA NUCLEAR POLYHEDROSIS VIRUS (ACMNPV).//P41661
F-THYRO1000242//ZINC FINGER PROTEIN 84 (ZINC FINGER PROTEIN HPF2).//7.4e-37:137:36//HOMO SAPIENS (HUMAN).//P51523
F-THYRO1000253//DNA-BINDING P52/P100 COMPLEX, 100 KD SUBUNIT (FRAGMENTS).//0.11:21:52//HOMO SAPIENS (HUMAN).//P30808
F-THYRO1000270//WDNM1 PROTEIN PRECURSOR.//0.40:52:32//MUS MUSCULUS (MOUSE).//Q62477
F-THYRO1000279//BETA CRYSTALLIN A4.//0.97:64:26//BOS TAURUS (BOVINE).//P11842
F-THYRO1000288//POTENTIAL CAAX PRENYL PROTEASE 1 (EC 3.4.24.-) (PRENYL PROTEIN- SPECIFIC ENDOPROTEASE 1) (PPSEP 1).//3.4e-48:142:42//SCHIZOSACCHAROMYCES POMBE (FISSION YEAST).//Q10071
F-THYRO1000320//ZINC FINGER PROTEIN 14 (ZFP-14) (KROX-9 PROTEIN) (FRAGMENT).//0.87:35:45//MUS MUSCULUS (MOUSE).//P10755
F-THYRO1000327//HYPOTHETICAL 64.7 KD PROTEIN F26E4.11 IN CHROMOSOME I.//0.00010:75:26//CAENORHABDITIS ELEGANS.//P90859
F-THYRO1000343//CHROMOGRANIN A PRECURSOR (CGA) [CONTAINS: PANCREASTATIN; BETA-GRANIN; WE-14].//0.88:107:26//MUS MUSCULUS (MOUSE).//P26339
F-THYRO1000358//SELENIUM-BINDING LIVER PROTEIN.//4.6e-25:49:81//MUS MUSCULUS (MOUSE).//P17563
F-THYRO1000368//LOCOMOTION-RELATED PROTEIN HIKARU GENKI PRECURSOR.//1.0:136:26//DROSOPHILA MELANOGASTER (FRUIT FLY).//Q09101
F-THYRO1000381//GAG POLYPROTEIN [CONTAINS: CORE PROTEIN P15; INNER COAT PROTEIN P12; CORE SHELL PROTEIN P30; NUCLEOPROTEIN P10].//0.032:99:35//SIMIAN SARCOMA VIRUS.//P03330
F-THYRO1000387//ATP SYNTHASE PROTEIN 8 (EC 3.6.1.34) (A6L).//0.90:46:30//HALICHOERUS GRYPUS (GRAY SEAL).//P38592
F-THYRO1000394//SMALL PROLINE RICH PROTEIN II (SPR-II) (CLONE 930).//0.00019:48:37//HOMO SAPIENS (HUMAN).//P22531
F-THYRO1000395//RING CANAL PROTEIN (KELCH PROTEIN).//1.2e-33:186:38//DROSOPHILA MELANOGASTER (FRUIT FLY).//Q04652
F-THYRO1000401//50S RIBOSOMAL PROTEIN L7/L12 (FRAGMENT).//0.57:67:31//STAPHYLOCOCCUS AUREUS.//P48860
F-THYRO1000438//ATP SYNTHASE PROTEIN 8 (EC 3.6.1.34) (A6L).//1.0:42:38//STRONGYLOCENTROTUS PURPURATUS (PURPLE SEA URCHIN).//P15997
F-THYRO1000452//BACTERIOCIN CARNOBACTERIOCIN A PRECURSOR (PISCICOLIN 61).//0.31:34:44//CARNOBACTERIUM PISCICOLA.//P38578
F-THYRO1000471//!!!! ALU SUBFAMILY SQ WARNING ENTRY !!!!//2,1e-31:94:72//HOMO SAPIENS (HUMAN).//P39194
F-THYRO1000484//!!!! ALU SUBFAMILY SX WARNING ENTRY !!!!//5.9e-08:30:86//HOMO SAPIENS (HUMAN).//P39195
F-THYRO1000488//EARLY NODULIN 55-2 PRECURSOR (N-55-2) (NODULIN-315).//0.93:98:27//GLYCINE MAX (SOYBEAN).//Q02917
F-THYRO1000501//DOWN REGULATORY PROTEIN OF INTERLEUKIN 2 RECEPTOR.//2.4e-51:198:50//MUS MUSCULUS (MOUSE).//P15533
F-THYRO1000502//HUNCHBACK PROTEIN (FRAGMENT).//0.84:41:43//APIS MELLIFERA (HONEYBEE).//P31504
F-THYRO1000505//HYPOTHETICAL BHLF1 PROTEIN.//0.99:231:33//EPSTEIN-BARR VIRUS (STRAIN B95-8)(HUMAN HERPESVIRUS 4).//P03181
F-THYRO1000558//ANTITHROMBIN-III PRECURSOR (ATIII) (FRAGMENT).//0.47:58:37//GALLUS GALLUS (CHICKEN).//Q03352
F-THYRO1000569//COLLAGEN ALPHA 1(I) CHAIN (FRAGMENTS).//0.00048:64:42//RATTUS NORVEGICUS (RAT).//P02454
F-THYRO1000570//HYPOTHETICAL 11.6 KD PROTEIN IN ACS1-GCV3 INTERGENIC REGION.//0.94:61:32//SACCHAROMYCES CEREVISIAE (BAKER'S YEAST).//P39725
F-THYRO1000585//SPLICING FACTOR, ARGININE/SERINE-RICH 6 (PRE-MRNA SPLICING FACTOR SRP55).//0.050:104:36//HOMO SAPIENS (HUMAN).//Q13247
F-THYRO1000596//INFECTED CELL PROTEIN ICP34.5 (NEUROVIRULENCE FACTOR ICP34.5).//0.99:37:40//HERPES SIMPLEX VIRUS (TYPE 1 / STRAIN MGH-10).//P37319
F-THYRO1000602//EAMZP30-47 PROTEIN (FRAGMENT).//0.88:61:34//EIMERIA ACERVULINA.//P21959
F-THYRO1000605//SUPPRESSOR PROTEIN SRP40.//0.0016:116:26//SACCHAROMYCES CEREVISIAE (BAKER'S YEAST).//P32583
F-THYRO1000625//!!!! ALU SUBFAMILY SQ WARNING ENTRY !!!!//3.4e-33:88:78//HOMO SAPIENS (HUMAN).//P39194
F-THYRO1000637//METALLOTHIONEIN A (MT A).//1.0:23:43//SPARUS AURATA (GILTHEAD SEA BREAM).//P52727
F-THYRO1000641//PHOTOSYSTEM II 10 KD PHOSPHOPROTEIN.//0.99:26:46//CYANIDIUM CALDARIUM (GALDIERIA SULPHURARIA).//O19925
F-THYRO1000658//!!!! ALU SUBFAMILY SB WARNING ENTRY !!!!//1.5e-49:116:69//HOMO SAPIENS (HUMAN).//P39189
F-THYRO1000662//DNA-DAMAGE-INDUCIBLE PROTEIN P.//3.7e-15:119:43//ESCHERICHIA COLI.//Q47155
F-THYRO1000666//KINESIN-LIKE PROTEIN KLP1.//1.0e-44:232:41//CHLAMYDOMONAS REINHARDTII.//P46870
F-THYRO1000676//!!!! ALU SUBFAMILY SP WARNING ENTRY !!!!//2.1e-15:144:39//HOMO SAPIENS (HUMAN).//P39193
F-THYRO1000684//HYPOTHETICAL 73.5 KD PROTEIN IN SCS3-RPS2 INTERGENIC REGION.//0.00033:84:30//SACCHAROMYCES CEREVISIAE (BAKER'S YEAST).//P53129
F-THYRO1000699//!!!! ALU SUBFAMILY SC WARNING ENTRY !!!!//0.97:20:85//HOMO SAPIENS (HUMAN).//P39192
F-THYRO1000712//!!!! ALU SUBFAMILY J WARNING ENTRY !!!!//4.2e-10:69:59//HOMO SAPIENS (HUMAN).//P39188
F-THYRO1000715//SALIVARY PROLINE-RICH PROTEIN PRECURSOR (CLONES CP3, CP4 AND CP5) [CONTAINS: BASIC PEPTIDE IB-6; PEPTIDE P-H].//4.6e-10:204:32//HOMO SAPIENS (HUMAN).//P04280
F-THYRO1000734
F-THYRO1000748//HYPOTHETICAL PROTEIN KIAA0411 (FRAGMENT).//1.8e-46:130:70//HOMO SAPIENS (HUMAN).//O43295
F-THYRO1000756//ALPHA-N-ACETYLGALACTOSAMINIDE ALPHA-2,6-SIALYLTRANSFERASE (EC 2.4.99.-) (ST6GALNACIII) (STY).//1.1e-06:95:31//RATTUS NORVEGICUS (RAT).//Q64686
F-THYRO1000777//CUTICLE COLLAGEN 2C (FRAGMENT).//0.0031:119:34//HAEMONCHUS CONTORTUS.//P16252
F-THYRO1000783//MYOSIN IC HEAVY CHAIN.//0.0014:121:37//ACANTHAMOEBA CASTELLANII (AMOEBA).//P10569
F-THYRO1000787//HUNCHBACK PROTEIN (FRAGMENT).//0.54:25:52//PHOLCUS PHALANGIOIDES.//Q02031
F-THYRO1000793//PRE-MRNA SPLICING FACTOR PRP9.//0.91:3 0:36//SACCHAROMYCES CEREVISIAE (BAKER'S YEAST).//P19736
F-THYRO1000796
F-THYRO1000805//HYPOTHETICAL 7.3 KD PROTEIN IN 100 KD PROTEIN REGION.//0.081:31:38//HUMAN ADENOVIRUS TYPE 41.//P23691
F-THYRO1000815//!!!! ALU SUBFAMILY SX WARNING ENTRY !!!!//6.0e-30:81:70//HOMO SAPIENS (HUMAN).//P39195
F-THYRO1000829//NEUROTOXIN III (BOM III).//0.022:32:34//BUTHUS OCCITANUS MARDOCHEI (MOROCCAN SCORPION).//P13488
F-THYRO1000843//HYPOTHETICAL 7.7 KD PROTEIN IN GENES 5-4 INTERGENIC REGION (ORF 109).//0.98:25:44//BACTERIOPHAGE P22.//P26750
F-THYRO1000852//SULFATED SURFACE GLYCOPROTEIN 185 (SSG 185).//7.3e-09:83:42//VOLVOX CARTERI.//P21997
F-THYRO1000855//ANTIFREEZE PEPTIDE 4 PRECURSOR.//1.0:54:35//PSEUDOPLEURONECTA AMERICANUS (WINTER FLOUNDER).//P02734
F-THYRO1000865//!!!! ALU SUBFAMILY J WARNING ENTRY!!!!//5.2e-17:66:57//HOMO SAPIENS (HUMAN).//P39188
F-THYRO1000895//!!!! ALU SUBFAMILY SB WARNING ENTRY !!!!//1.0e-12:58:62//HOMO SAPIENS (HUMAN).//P39189
F-THYRO1000916//!!!! ALU SUBFAMILY SB WARNING ENTRY !!!!//2.0e-32:101:69//HOMO SAPIENS (HUMAN).//P39189
F-THYRO1000926//NITROGEN FIXATION REGULATORY PROTEIN.//5.5e-05:108:27//KLEBSIELLA OXYTOCA.//P56267
F-THYRO1000934//PYRROLINE-5-CARBOXYLATE REDUCTASE (EC 1.5.1.2) (P5CR) (P5C REDUCTASE).//3.9e-50:147:40//HOMO SAPIENS (HUMAN).//P32322
F-THYRO1000951//DIHYDROXYACETONE KINASE (EC 2.7.1.29) (GLYCERONE KINASE).//1.8e-31:136:56//CITROBACTER FREUNDII.//P45510
F-THYRO1000952//HYPOTHETICAL 182.0 KD PROTEIN IN NMD5-HOM6 INTERGENIC REGION.//2.4e-05:91:34//SACCHAROMYCES CEREVISIAE (BAKER'S YEAST).//P47170
F-THYRO1000974//MITOCHONDRIAL ATP-DEPENDENT RNA HELICASE SUV3 PRECURSOR.//1.0:35:40//SACCHAROMYCES CEREVISIAE (BAKER'S YEAST).//P32580
F-THYRO1000975
F-THYRO1000983//HYPOTHETICAL 48.1 KD PROTEIN B0403.2 IN CHROMOSOME X.//1.3e-20:96:51//CAENORHABDITIS ELEGANS.//Q11076
F-THYRO1000984//GTP-BINDING ADP-RIBOSYLATION FACTOR HOMOLOG 1 PROTEIN.//0.011:76:34//DROSOPHILA MELANOGASTER (FRUIT FLY).//P25160
F-THYRO1000988
F-THYRO1001003//HYPOTHETICAL 8.1 KD PROTEIN IN MSCL-RPLQ INTERGENIC REGION.//0.97:60:31//ESCHERICHIA COLI.//P36675
F-THYRO1001031//!!!! ALU SUBFAMILY SX WARNING ENTRY !!!//9.5e-18:56:66//HOMO SAPIENS (HUMAN).//P39195
F-THYRO1001033//TRANSFORMATION-SENSITIVE PROTEIN IEF SSP 3521.//5.0e-13:126:35//HOMO SAPIENS (HUMAN).//P31948
F-THYRO1001062//!!!! ALU SUBFAMILY SQ WARNING ENTRY !!!!//1.1e-35:97:79//HOMO SAPIENS (HUMAN).//P39194
F-THYRO1001093//!!!! ALU SUBFAMILY SQ WARNING ENTRY !!!!//6.4e-13:70:57//HOMO SAPIENS (HUMAN).//P39194
F-THYRO1001100//ZINC FINGER X-LINKED PROTEIN ZXDA (FRAGMENT).//4.2e-63:219:63//HOMO SAPIENS (HUMAN).//P98168
F-THYRO1001120//SPLICEOSOME ASSOCIATED PROTEIN 49 (SAP 49) (SF3B53).//0.00068:160:31//HOMO SAPIENS (HUMAN).//Q15427
F-THYRO1001121//VERY HYPOTHETICAL 20.6 KD PROTEIN C56F8.15 IN CHROMOSOME I.//0.37:158:28//SCHIZOSACCHAROMYCES POMBE (FISSION YEAST).//Q10263
F-THYRO1001133//!!!! ALU SUBFAMILY J WARNING ENTRY !!!!//7.3e-15:59:66//HOMO SAPIENS (HUMAN).//P39188
F-THYRO1001134//SALIVARY PROLINE-RICH PROTEIN PO (ALLELE M) [CONTAINS: PEPTIDE P-D] (FRAGMENT).//0.00088:159:29//HOMO SAPIENS (HUMAN).//P10161
F-THYRO1001142//!!!! ALU SUBFAMILY SQ WARNING ENTRY !!!!//3.0e-29:81:71//HOMO SAPIENS (HUMAN).//P39194
F-THYRO1001173//CYTOCHROME C OXIDASE POLYPEPTIDE VIIS (EC 1.9.3.1).//0.88:51:35//DICTYOSTELIUM DISCOIDEUM (SLIME MOLD).//P20610
F-THYRO1001177//!!!! ALU SUBFAMILY SC WARNING ENTRY !!!!//3.0e-24:91:68//HOMO SAPIENS (HUMAN).//P39192
F-THYRO1001189//MKR2 PROTEIN (ZINC FINGER PROTEIN 2).//7.3e-27:165:39//MUS MUSCULUS (MOUSE).//P08043
F-THYRO1001204//BASIC PROLINE-RICH PEPTIDE P-E (IB-9).//0.67:42:42//HOMO SAPIENS (HUMAN).//P02811
F-THYRO1001213//!!!! ALU SUBFAMILY SQ WARNING ENTRY !!!!//2.9e-16:61:68//HOMO SAPIENS (HUMM).//P39194
F-THYRO1001262//!!!! ALU SUBFAMILY SP WARNING ENTRY !!!!//1.4e-36:50:84//HOMO SAPIENS (HUMAN).//P39193
F-THYRO1001271//HYPOTHETICAL 35.5 KD PROTEIN IN TRANSPOSON TN4556.//0.62: 126:30//STREPTOMYCES FRADIAE.//P20186
F-THYRO1001287//HYPOTHETICAL 91.2 KD PROTEIN IN RPS4B-SCH9 INTERGENIC REGION.//1.9e-26:208:37//SACCHAROMYCES CEREVISIAE (BAKER'S YEAST).//P38888
F-THYRO1001290//GIANT HEMOGLOBIN AIV CHAIN (FRAGMENT).//1.0:31:38//LAMELLIBRACHIA SP. (DEEP-SEA GIANT TUBE WORM).//P20413
F-THYRO1001313//VACUOLAR PROTEIN SORTING-ASSOCIATED PROTEIN VPS5.//0.00042:105:31//SACCHAROMYCES CEREVISIAE (BAKER'S YEAST).//Q92331
F-THYRO1001320//COLLAGEN ALPHA 1(III) CHAIN.//0.27:57:38//BOS TAURUS (BOVINE).//P04258
F-THYRO1001321//!!!! ALU SUBFAMILY J WARNING ENTRY !!!!//5.5e-20:74:64//HOMO SAPIENS (HUMAN).//P39188
F-THYRO1001322//HYPOTHETICAL 7.2 KD PROTEIN.//0.66:49:30//VACCINIA VIRUS (STRAIN COPENHAGEN).//P21123
F-THYRO1001347//TOXIN F-VIII PRECURSOR (TOXIN TA2) (TOXIN DAF8).//0.94:61:36//DENDROASPIS ANGUSTICEPS (EASTERN GREEN MAMBA).//P01404
F-THYRO1001363//!!!! ALU SUBFAMILY J WARNING ENTRY !!!!//0.0025:23:73//HOMO SAPIENS (HUMAN).//P39188
F-THYRO1001365//MERSACIDIN PRECURSOR.//0.35:38:42//BACILLUS SP. (STRAIN HIL-Y85/54728).//P43683
F-THYRO1001374//PROTEIN VDLD.//1.6e- 3:140:31//HELICOBACTER PYLORI (CAMPYLOBACTER PYLORI).//O05729
F-THYRO1001401//!!!! ALU SUBFAMILY SC WARNING ENTRY !!!!//0.047:43:48//HOMO SAPIENS (HUMAN).//P39192
F-THYRO1001403
F-THYRO1001405//SMALL PROLINE RICH PROTEIN II (SPR-II) (CLONE 930).//0.0068:26:42//HOMO SAPIENS (HUMAN).//P22531
F-THYRO1001406//PUTATIVE STEROID DEHYDROGENASE KIK-I (EC 1.1.1.-).//3.1e-81:97:83//MUS MUSCULUS (MOUSE).//O70503
F-THYRO1001411//!!!! ALU SUBFAMILY SP WARNING ENTRY !!!!//1.9e-26:89:74//HOMO SAPIENS (HUMAN).//P39193
F-THYRO1001426//!!!! ALU SUBFAMILY SP WARNING ENTRY !!!!//1.4e-09:55:61//HOMO SAPIENS (HUMAN).//P39193
F-THYRO1001434//BETA-DEFENSIN 4 PRECURSOR (BNDB-4).//0.68:44:34//BOS TAURUS (BOVINE).//P46162
F-THYRO1001458//MYOSIN HEAVY CHAIN, NONMUSCLE TYPE B (CELLULAR MYOSIN HEAVY CHAIN, TYPE B) (NMMHC-B).//3.8e-64:216:62//HOMO SAPIENS (HUMAN).//P35580
F-THYRO1001480//!!!! ALU SUBFAMILY SQ WARNING ENTRY !!!!//9.3e-29:88:75//HOMO SAPIENS (HUMAN).//P39194
F-THYRO1001487//HOMEOBOX PROTEIN HOX-B4 (HOX-2.6).//0.99:59:37//MUS MUSCULUS (MOUSE).//P10284
F-THYRO1001534//!!!! ALU SUBFAMILY SQ WARNING ENTRY !!!!//1.4e-14:40:82//HOMO SAPIENS (HUMAN).//P39194
F-THYRO1001537//HYPOTHETICAL 33.8 KD PROTEIN IN TWT1-FLO5 INTERGENIC REGION.//2.4e-07:142:32//SACCHAROMYCES CEREVISIAE (BAKER'S YEAST).//P38892
F-THYRO1001541//!!!! ALU SUBFAMILY SX WARNING ENTRY !!!!//0.98:26:61//HOMO SAPIENS (HUMAN).//P39195
F-THYRO1001559//PROTEIN Q300.//2.6e-05:20:75//MUS MUSCULUS (MOUSE).//Q02722
F-THYRO1001570
F-THYRO1001573//SPERM MITOCHONDRIAL CAPSULE SELENOPROTEIN (MCS).//0.033:71:36//MUS MUSCULUS (MOUSE).//P15265
F-THYRO1001584//SUPPRESSOR PROTEIN SRP40.//2.1e-05:188:27//SACCHAROMYCES CEREVISIAE (BAKER'S YEAST).//P32583
F-THYRO1001595//RAS SUPPRESSOR PROTEIN 1 (RSU-1) (RSP-1 PROTEIN) (RSP-1).//6.1e-21:35:91//HOMO SAPIENS (HUMAN).//Q15404
F-THYRO1001602//TRK SYSTEM POTASSIUM UPTAKE PROTEIN TRKH.//1.0:57:42//HAEMOPHILUS INFLUENZAE.//P44843
F-THYRO1001605//VENOM BASIC PROTEASE INHIBITORS IX AND VIIIB.//1.0:34:38//BUNGARUS FASCIATUS (BANDED KRAIT).//P25660
F-THYRO1001617//!!!! ALU SUBFAMILY SQ WARNING ENTRY !!!!//7.0e-18:55:81//HOMO SAPIENS (HUMAN).//P39194
F-THYRO1001637//!!!! ALU SUBFAMILY SX WARNING ENTRY !!!!//0.00020:25:80//HOMO SAPIENS (HUMAN).//P39195
F-THYRO1001656//PROLINE-RICH PROTEIN MP-2 PRECURSOR.//0.0091:54:42//MUS MUSCULUS (MOUSE).//P05142
F-THYRO1001661//HYPOTHETICAL 21.1 KD PROTEIN IN SSR-SERA INTERGENIC REGION (O182).//0.033:77:35//ESCHERICHIA COLI.//P09160
F-THYRO1001671//(2'-5')OLIGOADENYLATE SYNTHETASE 1 (EC 2.7.7.-) ((2-5')OLIGO(A) SYNTHETASE 1) (2-5A SYNTHETASE 1) (P46/P41) (E18/E16).//4.3e-34:207:34//HOMO SAPIENS (HUMAN).//P00973
F-THYRO1001673//!!!! ALU SUBFAMILY SQ WARNING ENTRY !!!!//3.9e-08:49:65//HOMO SAPIENS (HUMAN).//P39194
F-THYRO1001703//HYPOTHETICAL 69.8 KD PROTEIN IN BDF1-SFP1 INTERGENIC REGION.//6.4e-16:134:35//SACCHAROMYCES CEREVISIAE (BAKER'S YEAST).//Q06053
F-THYRO1001706
F-THYRO1001721//RING CANAL PROTEIN (KELCH PROTEIN).//2.7e-27:191:36//DROSOPHILA MELANOGASTER (FRUIT FLY).//Q04652
F-THYRO1001738//MATING PROCESS PROTEIN MID2 (SERINE-RICH PROTEIN SMS1) (PROTEIN KINASE A INTERFERENCE PROTEIN).//0.0032:105:32//SACCHAROMYCES CEREVISIAE (BAKER'S YEAST).//P36027
F-THYRO1001745
F-THYRO1001746//GENE 10 PROTEIN.//1.0:55:30//SPIROPLASMA VIRUS SPV1-R8A2 B.//P15901
F-THYRO1001772//!!!! ALU SUBFAMILY J WARNING ENTRY !!!!//1.2e-05:41:63//HOMO SAPIENS (HUMAN).//P39188
F-THYRO1001793//HYPOTHETICAL 21.6 KD PROTEIN F37A4.2 IN CHROMOSOME III.//1.5e-26:161:42//CAENORHABDITIS ELEGANS.//P41880
F-THYRO1001809//LATENCY-RELATED PROTEIN 2.//0.49:74:27//HERPES SIMPLEX VIRUS (TYPE 1 / STRAIN F).//P17589
F-THYRO1001828//PROTEINASE INHIBITOR.//0.11:34:50//SOLANUM MELONGENA (EGGPLANT) (AUBERGINE).//P01078
F-THYRO1001854//ACYL-COA-BINDING PROTEIN HOMOLOG (ACBP) (DIAZEPAM BINDING INHIBITOR HOMOLOG) (DBI).//0.63:50:38//RANA RIDIBUNDA (LAUGHING FROG) (MARSH FROG).//P45883
F-THYRO1001895//!!!! ALU SUBFAMILY J WARNING ENTRY !!!!//6.1e-09:72:47//HOMO SAPIENS (HUMAN).//P39188
F-THYRO1001907//TRYPOMASTIGOTE DECAY-ACCELERATING FACTOR (T-DAF) (FRAGMENT).//0.79:36:44//TRYPANOSOMA CRUZI.//Q26327
F-VESEN1000122//HOMEOBOX PROTEIN HB9.//0.57:64:32//HOMO SAPIENS (HUMAN).//P50219
F-Y79AA1000013//METALLOTHIONEIN B (MT-B).//0.034:35:48//SALMO SALAR (ATLANTIC SALMON).//P52720
F-Y79AA1000033//CHOLECYSTOKININ.//0.97:49:30//PSEUDEMYS SCRIPTA (SLIDER TURTLE).//P80345
F-Y79AA1000037//DNA-BINDING PROTEIN BMI-1.//1.4e-23:80:60//HOMO SAPIENS (HUMAN).//P35226
F-Y79AA1000059//HYPOTHETICAL 35.5 KD PROTEIN IN TRANSPOSON TN4556.//0.0075:127:36//STREPTOMYCES FRADIAE.//P20186
F-Y79AA1000065//SALIVARY PROLINE-RICH PROTEIN PO (ALLELE K) [CONTAINS: PEPTIDE P-D] (FRAGMENT).//0.022:135:29//HOMO SAPIENS (HUMAN).//P10162
F-Y79AA1000131//REGULATORY PROTEIN E2.//1.1e-05:175:26//HUMAN PAPILLOMAVIRUS TYPE 24.//P50770
F-Y79AA1000181//PROLINE-RICH PROTEIN MP-3 (FRAGMENT).//1.4e-06:187:29//MUS MUSCULUS (MOUSE).//P05143
F-Y79AA1000202//HYPOTHETICAL PROLINE-RICH PROTEIN (FRAGMENT).//6.2e-09:47:53//OWENIA FUSIFORMIS.//P21260
F-Y79AA1000214//HISTONE H2A VARIANT.//1.7e-50:107:100//GALLUS GALLUS (CHICKEN).//P02272
F-Y79AA1000230//GONADOLIBERIN I PRECURSOR (LHRH I) (LUTEINIZING HORMONE RELEASING HORMONE I) (GONADOTROPIN RELEASING HORMONE I) (GNRH I) (LULIBERIN I).//0.27:64:34//HOMO SAPIENS (HUMAN).//P01148
F-Y79AA1000231//HYPOTHETICAL 47.9 KD PROTEIN M021B04.12.//2.5e-72:277:53//ARABIDOPSIS THALIANA (MOUSE-EAR CRESS).//O04658
F-Y79AA1000258//PROLINE-RICH PROTEIN MP-2 PRECURSOR.//2.8e-08:174:35//MUS MUSCULUS (MOUSE).//P05142
F-Y79AA1000268//COLLAGEN ALPHA 1(III) CHAIN (FRAGMENT).//0.00020:176:33//RATTUS NORVEGICUS (RAT).//P13941
F-Y79AA1000313//HYPOTHETICAL 54.0 KD PROTEIN C32A3.1 IN CHROMOSOME III.//0.092:127:21//CAENORHABDITIS ELEGANS.//Q09260
F-Y79AA1000328//SEL-10 PROTEIN.//5.3e-05:129:28//CAENORHABDITIS ELEGANS.//Q93794
F-Y79AA1000342//KERATIN, ULTRA HIGH-SULFUR MATRIX PROTEIN (UHS KERATIN).//1.0:73:30//OVIS ARIES (SHEEP).//P26372
F-Y79AA1000346//COATOMER GAMMA SUBUNIT (GAMMA-COAT PROTEIN) (GAMMA-COP).//1.8e-95:205:83//BOS TAURUS (BOVINE).//P53620
F-Y79AA1000349//ANTIFREEZE PEPTIDE 4 PRECURSOR.//0.036:37:54//PSEUDOPLEURONECTA AMERICANUS (WINTER FLOUNDER).//P02734
F-Y79AA1000355//HYPOTHETICAL 18.2 KD PROTEIN ZK632.13 IN CHROMOSOME III.//0.0031:106:28//CAENORHABDITIS ELEGANS.//Q10120
F-Y79AA1000368//REDUCED VIABILITY UPON STARVATION PROTEIN 161.//1.4e-16:208:28//SACCHAROMYCES CEREVISIAE (BAKER'S YEAST).//P25343
F-Y79AA1000405//LIGHT-HARVESTING PROTEIN B-800-850, ALPHA CHAIN C (ANTENNA PIGMENT PROTEIN, ALPHA CHAIN C) (LH II-C ALPHA).//0.98:50:30//RHODOPSEUDOMONAS PALUSTRIS.//P35103
F-Y79AA1000410//!!!! ALU SUBFAMILY SQ WARNING ENTRY !!!!//7.9e-20:62:79//HOMO SAPIENS (HUMAN).//P39 194
F-Y79AA1000420//HYPOTHETICAL 27.7 KD PROTEIN IN UME3-HDA1 INTERGENIC REGION.//1.4e-06:86:38//SACCHAROMYCES CEREVISIAE (BAKER'S YEAST).//P53970
F-Y79AA1000469//HYPOTHETICAL 48.4 KD PROTEIN F44B9.5 IN CHROMOSOME III.//2.8e-34:211:40//CAENORHABDITIS ELEGANS.//P34426
F-Y79AA1000480//HYPOTHETICAL 63.2 KD PROTEIN C1F3.09 IN CHROMOSOME I.//3.9e-15:90:32//SCHIZOSACCHAROMYCES POMBE (FISSION YEAST).//Q10414
F-Y79AA1000538//!!!! ALU SUBFAMILY SX WARNING ENTRY !!!!//0.37:41:48//HOMO SAPIENS (HUMAN).//P39195
F-Y79AA1000539//SPLICING FACTOR, ARGININE/SERINE-RICH 4 (PRE-MRNA SPLICING FACTOR SRP75).//1.8e-21:190:37//HOMO SAPIENS (HUMAN).//Q08170
F-Y79AA1000540//SPERM PROTAMINE P1.//0.00045:66:45//DASYURUS VIVERRINUS (SOUTHEASTERN QUOLL), AND DASYURUS HALLUCATUS.//P42135
F-Y79AA1000560//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT).//1.6e-79:186:87//MUS MUSCULUS (MOUSE).//P17427
F-Y79AA1000574//AKLAVINONE C-11 HYDROXYLASE (EC 1.-.-.-) (FRAGMENT).//0.010:35:60//STREPTOMYCES PEUCETIUS.//P32009
F-Y79AA1000589//32.3 KD PROTEIN IN CWP1-MBR1 INTERGENIC REGION.//4.5e-27:197:36//SACCHAROMYCES CEREVISIAE (BAKER'S YEAST).//P28320
F-Y79AA1000627//ZINC FINGER PROTEIN 134.//1.6e-34:191:35//HOMO SAPIENS (HUMAN).//P52741
F-Y79AA1000705//HYPOTHETICAL 128.5 KD HELICASE IN ATS1-TPD3 INTERGENIC REGION.//8.7e-36:250:40//SACCHAROMYCES CEREVISIAE (BAKER'S YEAST).//P31380
F-Y79AA1000734//PEROXISOMAL MEMBRANE PROTEIN PMP30A (PMP31) (PEROXIN-11A).//0.00037:108:27//CANDIDA BOIDINII (YEAST).//Q00316
F-Y79AA1000748//HYPOTHETICAL 61.3 KD PROTEIN F25B5.5 IN CHROMOSOME III./1.0e-23:210:34//CAENORHABDITIS ELEGANS.//Q09316
F-Y79AA1000752//PUTATIVE HETEROGENEOUS NUCLEAR RIBONUCLEOPROTEIN X (HNRNP X) (CBP).//1.4e-53:156:68//MUS MUSCULUS (MOUSE).//Q61990
F-Y79AA1000774//HYPOTHETICAL 77.9 KD PROTEIN IN RRN10-MCM2 INTERGENIC REGION.//1.2e-11:231:26//SACCHAROMYCES CEREVISIAE (BAKER'S YEAST).//P38205
F-Y79AA1000782//CUTICLE COLLAGEN 2.//0.012:56:35//CAENORHABDITIS ELEGANS.//P17656
F-Y79AA1000784//HISTIDINE-RICH GLYCOPROTEIN PRECURSOR.//1.3e-08:82:39//PLASMODIUM LOPHURAE.//P04929
F-Y79AA1000794//DNA-BINDING P52/P100 COMPLEX, 100 KD SUBUNIT (FRAGMENTS).//0.043:13:53//HOMO SAPIENS (HUMAN).//P30808
F-Y79AA1000800//PRIA PROTEIN PRECURSOR.//0.031:94:34//LENTINULA EDODES (SHIITAKE MUSHROOM) (LENTINUS EDODES).//Q01200
F-Y79AA1000802//HYPOTHETICAL 67.4 KD PROTEIN IN RPS3-PSD1 INTERGENIC REGION.//0.26:186:23//SACCHAROMYCES CEREVISIAE (BAKER'S YEAST).//P53882
F-Y79AA1000805//AMP DEAMINASE (EC 3.5.4.6) (MYOADENYLATE DEAMINASE).//0.99:78:35//SCHIZOSACCHAROMYCES POMBE (FISSION YEAST).//P50998
F-Y79AA1000824//HYPOTHETICAL 81.7 KD PROTEIN IN MOL1-NAT2 INTERGENIC REGION.//3.4e-44:111:49//SACCHAROMYCES CEREVISIAE (BAKER'S YEAST).//P48234
F-Y79AA1000827//HYPOTHETICAL BHLF1 PROTEIN.//0.0046:187:33//EPSTEIN-BARR VIRUS (STRAIN B95-8) (HUMAN HERPESVIRUS 4).//P03181
F-Y79AA1000833//TUBULIN ALPHA-1 CHAIN.//1.0e-75:239:66//CRICETULUS GRISEUS (CHINESE HAMSTER).//P05209
F-Y79AA1000850//SMALL PROLINE-RICH PROTEIN II (SPR-II) (CLONE 174N).//0.0078:57:31//HOMO SAPIENS (HUMAN).//P22532
F-Y79AA1000962//MYOSIN HEAVY CHAIN, GIZZARD SMOOTH MUSCLE.//8.5e-11:241:26//GALLUS GALLUS (CHICKEN).//P10587
F-Y79AA1000966//ATP SYNTHASE A CHAIN (EC 3.6.1.34) (PROTEIN 6).//0.69:122:31//TRYPANOSOMA BRUCEI BRUCEI.//P24499
F-Y79AA1000968//TRANSLATION INITIATION FACTOR EIF-2B GAMMA SUBUNIT (EIF-2B GDP-GTP EXCHANGE FACTOR).//3.3e-102:211:93//RATTUS NORVEGICUS (RAT).//P70541
F-Y79AA1000969//PROCOLLAGEN ALPHA 1(I) CHAIN PRECURSOR.//1.0:67:38//GALLUS GALLUS (CHICKEN)://P02457
F-Y79AA1000976//INVOLUCRIN.//0.99:66:31//CEBUS ALBIFRONS (WHITE-FRONTED CAPUCHIN).//P24709
F-Y79AA1000985//PERICENTRIN.//1.1e-24:116:59//MUS MUSCULUS (MOUSE).//P48725
F-Y79AA1001023//HYPOTHETICAL 105.9 KD PROTEIN IN AAC3-RFC5 INTERGENIC REGION.//0.37:79:27//SACCHAROMYCES CEREVISIAE (BAKER'S YEAST).//P38250
F-Y79AA1001041//SPERMATID-SPECIFIC PROTEIN T1 [CONTAINS: SPERM PROTAMINE SP1].//0.93:43:39//SEPIA OFFICINALIS (COMMON CUTTLEFISH).//P80001
F-Y79AA1001048//ACYL-COA DEHYDROGENASE, VERY-LONG-CHAIN SPECIFIC PRECURSOR (EC 1.3.99.-) (VLCAD).//1.5e-51:211:52//BOS TAURUS (BOVINE).//P48818
F-Y79AA1001061//!!!! ALU SUBFAMILY SQ WARNING ENTRY !!!!.//3.8e-25:85:69//HOMO SAPIENS (HUMAN).//P39194
F-Y79AA1001068//PROCOLLAGEN ALPHA 1(II) CHAIN PRECURSOR [CONTAINS: CHONDROCALCIN].//0.0015:207:33//MUS MUSCULUS (MOUSE).//P28481
F-Y79AA1001077//ADULT-SPECIFIC RIGID CUTICULAR PROTEIN 11.9 (ACP 11.9).//0.99:36:41//ARANEUS DIADEMATUS (SPIDER).//P80515
F-Y79AA1001078//HYPOTHETICAL 88.1 KD PROTEIN K02D10.1 IN CHROMOSOME III.//1.0e-06:197:23//CAENORHABDITIS ELEGANS .//P34492
F-Y79AA1001105//HOMEOBOX PROTEIN OTX2.//2.9e-62:163:79//MUS MUSCULUS (MOUSE).//P80206
F-Y79AA1001145//!!!! ALU SUBFAMILY SX WARNING ENTRY !!!!//0.024:42:59//HOMO SAPIENS (HUMAN).//P39195
F-Y79AA1001167//HYPOTHETICAL 7.1 KD PROTEIN IN IAP2-VLF1 INTERGENIC REGION.//0.96:20:50//AUTOGRAPHA CALIFORNICA NUCLEAR POLYHEDROSIS VIRUS (ACMNPV).//P41471
F-Y79AA1001177//HYPOTHETICAL BHLF1 PROTEIN.//3.9e-05:135:34//EPSTEIN-BARR VIRUS (STRAIN B95-8) (HUMAN HERPESVIRUS 4).//P03181
F-Y79AA1001185//PUTATIVE CUTICLE COLLAGEN C09G5.5.//0.00017:93:38//CAENORHABDITIS ELEGANS.//Q09456
F-Y79AA1001211
F-Y79AA1001216//TENSIN.//0.012:134:32//GALLUS GALLUS (CHICKEN).//Q04205
F-Y79AA1001228//MUCIN 2 PRECURSOR (INTESTINAL MUCIN 2).//0.088:75:34//HOMO SAPIENS (HUMAN).//Q02817
F-Y79AA1001233//ESTRADIOL 17 BETA-DEHYDROGENASE 1 (EC 1.1.1.62) (17-BETA-HSD 1) (17-BETA-HYDROXYSTEROID DEHYDROGENASE 1).//1.1e-40:139:51//RATTUS NORVEGICUS (RAT).//P51657
F-Y79AA1001236//HYPOTHETICAL 34.7 KD PROTEIN IN ORC2-TIP1 INTERGENIC REGION.//2.0e-22:108:53//SACCHAROMYCES CEREVISIAE (BAKER'S YEAST).//P38238
F-Y79AA1001281
F-Y79AA1001299//PROLINE-RICH PROTEIN MP-3 (FRAGMENT).//0.0022:49:44//MUS MUSCULUS (MOUSE).//P05143
F-Y79AA1001312//50S RIBOSOMAL PROTEIN L24, CHLOROPLAST PRECURSOR.//0.98:117:25//ARABIDOPSIS THALIANA (MOUSE-EAR CRESS).//P92959
F-Y79AA1001323//CORNIFIN (SMALL PROLINE-RICH PROTEIN I) (SPR-I) (SMALL PROLINE-RICH SQUAMOUS CELL MARKER) (SPRP).//0.082:44:40//SUS SCROFA (PIG).//P35323
F-Y79AA1001384//APOLIPOPROTEIN C-III PRECURSOR (APO-CIII).//0.99:47:40//MUS MUSCULUS (MOUSE).//P33622
F-Y79AA1001391//HOMEOBOX PROTEIN HOX-A13 (HOX-1J).//9.8e-58:157:62//HOMO SAPIENS (HUMAN).//P31271
F-Y79AA1001394//TRICHOHYALIN.//4.7e-08:121:36//HOMO SAPIENS (HUMAN).//Q07283
F-Y79AA1001402//ETS-DOMAIN TRANSCRIPTION FACTOR ERF.//0.0087:81:33//MUS MUSCULUS (MOUSE).//P70459
F-Y79AA1001493//HYPOTHETICAL 48.1 KD PROTEIN B0403.2 IN CHROMOSOME X.//4.5e-21:125:44//CAENORHABDITIS ELEGANS.//Q11076
F-Y79AA1001511//HYPOTHETICAL 86.6 KD PROTEIN IN PFK1-TDS4 INTERGENIC REGION.//2.3e-17:249:31//SACCHAROMYCES CEREVISIAE (BAKER'S YEAST).//P53313
F-Y79AA1001533//DNA-DIRECTED RNA POLYMERASE I49 KD POLYPEPTIDE (EC 2.7.7.6) (A49).//0.0099:155:23//SACCHAROMYCES CEREVISIAE (BAKER'S YEAST).//Q01080
F-Y79AA1001541
F-Y79AA1001548//!!!! ALU SUBFAMILY SC WARNING ENTRY !!!!//1.1e-17:53:83//HOMO SAPIENS (HUMAN).//P39192
F-Y79AA1001555//MAJOR SURFACE ANTIGEN.//0.046:62:29//HEPATITIS B VIRUS.//P31873
F-Y79AA1001581//ACETYL-COENZYME A SYNTHETASE (EC 6.2.1.1) (ACETATE--COA LIGASE) (ACYL- ACTIVATING ENZYME).//8.6e-11:144:31//ESCHERICHIA COLI.//P27550
F-Y79AA1001585//SPERM MITOCHONDRIAL CAPSULE SELENOPROTEIN (MCS).//0.012:64:40//MUS MUSCULUS (MOUSE).//P15265
F-Y79AA1001594//CORNIFIN BETA.//0.61:88:31//MUS MUSCULUS (MOUSE).//O09116
F-Y79AA1001603//TRANSCRIPTION INITIATION FACTOR TFIID 135 KD SUBUNIT (TAFII-135) (TAFII135) (TAFII-130) (TAFII130).//0.024:170:30//HOMO SAPIENS (HUMAN).//O00268
F-Y79AA1001613//ZINC FINGER PROTEIN 42 (MYELOID ZINC FINGER 1) (MZF-1).//4.5e-09:136:27//HOMO SAPIENS (HUMAN).//P28698
F-Y79AA1001647//HYPOTHETICAL 23.1 KD PROTEIN CY277.20C.//0.093:94:26//MYCOBACTERIUM TUBERCULOSIS.//P71779
F-Y79AA1001665//HOMEOBOX PROTEIN DLX-2 (HOMEOBOX PROTEIN TES-1).//0.79:90:26//MUS MUSCULUS (MOUSE).//P40764
F-Y79AA1001679//LAMBDA-CRYSTALLIN.//1.6e-95:224:81//ORYCTOLAGUS CUNICULUS (RABBIT).//P14755
F-Y79AA1001692//GERM CELL-LESS PROTEIN.//3.5e-08:78:38//DROSOPHILA MELANOGASTER (FRUIT FLY).//Q01820
F-Y79AA1001696//INSULIN.//1.0:33:27//ANGUILLA ROSTRATA (AMERICAN EEL).//P42633
F-Y79AA1001705//HYPOTHETICAL BHLF1 PROTEIN.//0.0013:192:33//EPSTEIN-BARR VIRUS (STRAIN B95-8) (HUMAN HERPESVIRUS 4).//P03181
F-Y79AA1001711//PARATHYMOSIN (ZINC-BINDING 11.5 KD PROTEIN).//0.032:38:34//RATTUS NORVEGICUS (RAT).//P04550
F-Y79AA1001781
F-Y79AA1001805//VASODILATOR-STIMULATED PHOSPHOPROTEIN (VASP).//0.0063:128:30//HOMO SAPIENS (HUMAN).//P50552
F-Y79AA1001827//SPERM PROTAMINE P1.//0.015:45:40//DIDELPHIS MARSUPIALIS VIRGINIANA (NORTH AMERICAN OPOSSUM), AND MONODELPHIS DOMESTICA (SHORT-TAILED GREY OPOSSUM).//P35305
F-Y79AA1001846//!!!! ALU SUBFAMILY J WARNING ENTRY!!!!//2.4e-09:42:73//HOMO SAPIENS (HUMAN).//P39188
F-Y79AA1001848//KRUEPPEL PROTEIN (FRAGMENT).//1.8e-10:63:44//PSYCHODA CINEREA.//Q02035
F-Y79AA1001866//ZINC FINGER PROTEIN 90 (ZFP-90) (ZINC FINGER PROTEIN NK10).//0.00036:108:37//MUS MUSCULUS (MOUSE).//Q61967
F-Y79AA1001874//OX40L RECEPTOR PRECURSOR (ACT35 ANTIGEN) (TAX-TRANSCRIPTIONALLY ACTIVATED GLYCOPROTEIN-1 RECEPTOR) (CD134 ANTIGEN).//3.2e-07:100:35//HOMO SAPIENS (HUMAN).//P43489
F-Y79AA1001875//B-CELL GROWTH FACTOR PRECURSOR (BCGF-12 KD).//0.020:25:64//HOMO SAPIENS (HUMAN).//P20931
F-Y79AA1001923//SALIVARY PROLINE-RICH PROTEIN PO (ALLELE K) [CONTAINS: PEPTIDE P-D] (FRAGMENT).//0.016:83:36//HOMO SAPIENS (HUMAN).//P10162
F-Y79AA1001963//PUTATIVE PRE-MRNA SPLICING FACTOR ATP-DEPENDENT RNA HELICASE SPAC10F6.02C.//8.1e-13:94:47//SCHIZOSACCHAROMYCES POMBE (FISSION YEAST).//O42643
F-Y79AA1002027//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//9.8e-39:143:52//ARABIDOPSIS THALIANA (MOUSE-EAR CRESS).//P42743
F-Y79AA1002083//DNA-BINDING P52/P100 COMPLEX, 100 KD SUBUNIT (FRAGMENTS).//0.036:53:45//HOMO SAPIENS (HUMAN).//P30808
F-Y79AA1002089//HYPOTHETICAL 49.1 KD PROTEIN F02A9.4 IN CHROMOSOME III.//0.12:171:22//CAENORHABDITIS ELEGANS.//P34384
F-Y79AA1002093//MAX PROTEIN.//3.1e-07:111:29//BRACHYDANIO RERIO (ZEBRAFISH) (ZEBRA DANIO).//P52161
F-Y79AA1002103//SHORT NEUROTOXIN C.//0.040:21:47//AIPYSURUS LAEVIS (OLIVE SEA SNAKE).//P19958
F-Y79AA1002115//HYPOTHETICAL PROTEIN MJ0827.//0.84:68:30//METHANOCOCCUS JANNASCHII.//Q58237
F-Y79AA1002125//HYPOTHETICAL 24.7 KD PROTEIN IN POM152-REC114 INTERGENIC REGION.//3.4e-29:197:39//SACCHAROMYCES CEREVISIAE (BAKER'S YEAST).//P40206
F-Y79AA1002139//DNAJ PROTEIN HOMOLOG 1 (DROJ1).//1.9e-19:120:45//DROSOPHILA MELANOGASTER (FRUIT FLY).//Q24133
F-Y79AA1002204//TBX6 PROTEIN (T-BOX PROTEIN 6).//0.0011:162:32//MUS MUSCULUS (MOUSE).//P70327
F-Y79AA1002208//ANKYRIN.//2.9e-08:231:29//MUS MUSCULUS (MOUSE).//Q02357
F-Y79AA1002209//TYROSYL-TRNA SYNTHETASE, MITOCHONDRIAL PRECURSOR (EC 6.1.1.1) (TYROSINE--TRNA LIGASE) (TYRRS).//3.7e-23:170:32//SACCHAROMYCES CEREVISIAE (BAKER'S YEAST).//P48527
F-Y79AA1002210//CORNIFIN A (SMALL PROLINE-RICH PROTEIN IA) (SPR-IA) (SPRK).//0.0061:69:31//HOMO SAPIENS (HUMAN).//P35321
F-Y79AA1002211//!!!! ALU SUBFAMILY SP WARNING ENTRY !!!!//9.2e-10:43:62//HOMO SAPIENS (HUMAN).//P39193
F-Y79AA1002220
F-Y79AA1002229//HYPOTHETICAL 60.7 KD PROTEIN C56F8.17C IN CHROMOSOME I.//1.9e-21:147:40//SCHIZOSACCHAROMYCES POMBE (FISSION YEAST).//Q10264
F-Y79AA1002234
F-Y79AA1002246//MYOSIN IC HEAVY CHAIN.//0.00066:131:34//ACANTHAMOEBA CASTELLANII (AMOEBA).//P10569
F-Y79AA1002258//HYPOTHETICAL 103.9 KD PROTEIN ZK370.3 IN CHROMOSOME III.//4.3e-45:164:48//CAENORHABDITIS ELEGANS.//Q02328
F-Y79AA1002298//SALIVARY PROLINE-RICH PROTEIN PO (ALLELE M) [CONTAINS: PEPTIDE P-D] (FRAGMENT).//0.0063:99:31//HOMO SAPIENS (HUMAN).//P10161
F-Y79AA1002307
F-Y79AA1002311//HYPOTHETICAL 105.3 KD PROTEIN C01G6.5 IN CHROMOSOME III.//0.75:198:24//CAENORHABDITIS ELEGANS.//P46012
F-Y79AA1002351//CUTICLE COLLAGEN 34.//0.74:128:35//CAENORHABDITIS ELEGANS.//P34687
F-Y79AA1002361//GLC7-INTERACTING PROTEIN 2.//0.050:71:29//SACCHAROMYCES CEREVISIAE (BAKER'S YEAST).//P40036
F-Y79AA1002399//NEUROMODULIN (AXONAL MEMBRANE PROTEIN GAP-43) (PP46) (B-50) (PROTEIN F1) (CALMODULIN-BINDING PROTEIN P-57).//1.0:89:30//CARASSIUS AURATUS (GOLDFISH).//P17691
F-Y79AA1002407//HYPOTHETICAL 31.5 KD PROTEIN IN YGP1-YCK2 INTERGENIC REGION.//3.7e-16:232:28//SACCHAROMYCES CEREVISIAE (BAKER'S YEAST).//P53899
F-Y79AA1002416//CTP SYNTHASE (EC 6.3.4.2) (UTP--AMMONIA LIGASE) (CTP SYNTHETASE).//6.7e-72:162:84//HOMO SAPIENS (HUMAN).//P17812
F-Y79AA1002431//SMALL PROLINE RICH PROTEIN II (SPR-II) (CLONE 930).//0.81:34:41//HOMO SAPIENS (HUMAN).//P22531
F-Y79AA1002433//CELL DIVISION CONTROL PROTEIN 68.//0.00024:85:27//SACCHAROMYCES CEREVISIAE (BAKER'S YEAST).//P32558
F-Y79AA1002472//ZINC FINGER PROTEIN 35 (ZFP-35).//2.3e-60:217:44//MUS MUSCULUS (MOUSE).//P15620
F-Y79AA1002482//ZINC FINGER PROTEIN 141.//2.Oe-31:90:55//HOMO SAPIENS (HUMAN).//Q15928
F-Y79AA1002487//HYPOTHETICAL 67.1 KD TRP-ASP REPEATS CONTAINING PROTEIN C57A10.05C IN CHROMOSOME I.//0.18:41:36//SCHIZOSACCHAROMYCES POMBE (FISSION YEAST).//P87053

### Homology Search Result Data 2.

The result of the homology search of the GenBank using the clone sequence of 5'-end except EST and STS.

Data include
the name of clone,
definition of the top hit data,
the P-value: the length of the compared sequence: identity (%), and
the Accession No. of the top hit data, as in the order separated by *II*.

Data are not shown for the clones in which the P-value was higher than 1.
F-HEMBA1000005//Mouse tumor cell dnaJ-like protein 1 mRNA, complete cds.//3.4e-106:695:86//L16953
F-HEMBA1000012//Caenorhabditis-elegans cosmid C16C10, complete sequence.//1.5e-24:374:66//Z46787
F-HEMBA1000020//Homo sapiens beta 2 gene.//3.5e-112:529:90//X02344
F-HEMBA1000030//Rattus norvegicus G protein-coupled receptor kinase-associated ADP ribosylation factor GTPase-activating protein (GIT1) mRNA, complete cds.//5.6e-124:743:88//AF085693
F-HEMBA1000042//Human Chromosome 15q26.1 PAC clone pDJ460g16, WORKING DRAFT SEQUENCE, 3 unordered pieces.//1.1e-25:529:65//AC004581
F-HEMBA1000046//Human DNA sequence *** SEQUENCING IN PROGRESS *** from clone 125I3, WORKING DRAFT SEQUENCE.//3.2e-11:330:63//AL033528
F-HEMBA1000050//Homo sapiens DNA sequence from PAC 172K10 on chromosome 6q24. Contains STS, GSS and chromosome 6 fragment, complete sequence.//0.32:407:59//AL022477
F-HEMBA1000076//Homo sapiens full-length insert cDNA clone ZB97G06.//6.2e-135:594:98//AF086182
F-HEMBA1000111//CIT-HSP-2291M18.TF CIT-HSP Homo sapiens genomic clone 2291M18 genomic survey sequence.//2.8e-16:132:79//AQ004134
F-HEMBA1000129//Homo sapiens chromosome 17, clone HCIT48C15, complete sequence.//8.6e-98:230:93//AC003104
F-HEMBA1000141//Homo sapiens mRNA for KIAA0797 protein, partial cds.//2.1e-167:791:98//AB018340
F-HEMBA1000150//Homo sapiens mRNA for KIAA0788 protein, partial cds.//2.2e-44:242:96//AB018331
F-HEMBA1000156//Rattus norvegicus scaffold attachment factor B mRNA, complete cds.//1.1e-10:409:60//AF056324
F-HEMBA1000158//Homo sapiens CAGH44 mRNA, partial cds.//1.6e-35:365:73//U80741
F-HEMBA1000168//Human DNA sequence *** SEQUENCING IN PROGRESS *** from clone 321D2, WORKING DRAFT SEQUENCE.//0.99:290:61//AL031033
F-HEMBA1000180//rat u2 small nuclear rna gene and flanks.//3.7e-18:112:98//K00034
F-HEMBA1000185
F-HEMBA1000193//Human FMR1 gene, 5' end.//0.0012:191:67//L19476
F-HEMBA1000201//Human Ini1 mRNA, complete cds.//2.0e-73:440:92//U04847
F-HEMBA1000213//Plasmodium falciparum MAL3P7, complete sequence.//0.90:332:59//AL034559
F-HEMBA1000216//Mus musculus hypoxia inducible factor three alpha mRNA, complete cds.//4.8e-117:585:83//AF060194
F-HEMBA1000227//H.sapiens CpG island DNA genomic Mse1 fragment, clone 179h6, reverse read cpg179h6.rt1a.//1.9e-14:95:98//Z64921
F-HEMBA1000231//H.sapiens CpG island DNA genomic Mse1 fragment, clone 90a5, reverse read cpg90a5.rt1a.//5.1e-34:186:97//Z56144
F-HEMBA1000243//Human DNA sequence from PAC 440O21 on chromosome X contains ESTs and STS.//4.1e-67:291:82//Z84481
F-HEMBA1000244//M.musculus Ank-1 mRNA for erythroid ankydn.//0.029:316:59//X69065
F-HEMBA1000251//Homo sapiens PAC clone DJ0988L12 from 7q11.23-q21.1, complete sequence.//0.35:467:60//AC004454
F-HEMBA1000264
F-HEMBA1000280//Homo sapiens clone DJ0292L20, WORKING DRAFT SEQUENCE, 2 unordered pieces.//8.9e-20:218:78//AC004825
F-HEMBA1000282//Homo sapiens chromosome Y, clone 264,M,20, complete sequence.//4.2e-08:134:77//AC004617
F-HEMBA1000288//345L5.TPB CIT978SKA1 Homo sapiens genomic clone A-345L05, genomic survey sequence.//1.1e-06:152:73//B17459
F-HEMBA1000290//Human ornithine decarboxylase gene, complete cds.//3.2e-11:507:62//M33764
F-HEMBA1000302//CIT-HSP-2169N13.TF CIT-HSP Homo sapiens genomic clone 2169N13, genomic survey sequence.//5.4e-06:86:88//B90730
F-HEMBA1000303//Mus musculus Plenty of SH3s (POSH) mRNA, complete cds..//7.9e-111:701:86//AF030131
F-HEMBA1000304//HS_3006_A1_A09_T7 CIT Approved Human Genomic Sperm Library D Homo sapiens genomic clone Plate=3006 Col=17 Row=A, genomic survey sequence.//5.2e-40:240:92//AQ118226
F-HEMBA1000307//Mus musculus mRNA for CDV-1R protein.//7.9e-127:815:84//Y10495
F-HEMBA1000327//HS_3124_B2_H08_MR CIT Approved Human Genomic Sperm Library D Homo sapiens genomic clone Plate=3124 Col=16 Row=P, genomic survey sequence.//1.4e-11:87:96//AQ187492
F-HEMBA1000333
F-HEMBA1000338//Homo sapiens chromosome X, PAC 671D9, complete sequence.//4.0e-66:271:84//AF031078
F-HEMBA1000351//Homo sapiens PAC clone DJ0649P17 from 7q11.23-q21, complete sequence.//0.64:334:60//AC004848
F-HEMBA1000355//Pseudorabies virus serine/threonine kinase (ULPK) gene, partial cds and alkaline nuclease (AN) gene, complete cds.//0.017:313:63//U25056
F-HEMBA1000356//Oryctolagus cuniculus troponin T cardiac isoform mRNA, 3' end of cds.//0.87:198:61//L40178
F-HEMBA1000357//HS_3194_A1_D05_MR CIT Approved Human Genomic Sperm Library D Homo sapiens genomic clone Plate=3194 Col=9 Row=G, genomic survey sequence.//6.5e-90:436:98//AQ173748
F-HEMBA1000366//HS_3027_B2_G06_MF CIT Approved Human Genomic Sperm Library D Homo sapiens genomic clone Plate=3027 Col=12 Row=N, genomic survey sequence.//0.0074:192:64//AQ128843
F-HEMBA1000369//Human DNA sequence from clone 1039K5 on chromosome 22q12.3-13.2 Contains gene similar to PICK1 perinuclear binding protein, gene similar to monocarboxylate transporter (MCT3), ESTs, STS, GSS and a CpG island, complete sequence.//4.2e-106:133:99//AL031587
F-HEMBA1000376//Homo sapiens chromosome 19, BAC CIT-B-393i15 (BC301323), complete sequence.//1.6e-22:659:63//AC006116
F-HEMBA1000387//Homo sapiens chromosome 12p13.3 clone RPCI11-264F23, WORKING DRAFT SEQUENCE, 90 unordered pieces.//3.2e-06:136:75//AC006122
F-HEMBA1000390//Homo sapiens BAC clone RG119C02 from 7p15, complete sequence.//3.5e-111:284:95//AC004520
F-HEMBA1000392//Human DNA sequence *** SEQUENCING IN PROGRESS *** from clone 173D1, WORKING DRAFT SEQUENCE.//1.8e-39:332:80//AL031984
F-HEMBA1000396//Human Xq13 3' end of PAC 92E23 containing the X inactivation transcipt (XIST) gene, complete sequence.//9.5e-35:364:73//U80460
F-HEMBA1000411//Human Xp22 contig of 3 PACS (R7-39D12, R7-134G1, R7-185L21) from the Roswell Park Cancer Institute, complete sequence.//8.1e-18:424:64//U96409
F-HEMBA1000418//Drosophila melanogaster Oregon-R mitochondrial A+T region.//0.0026:564:59//U11584
F-HEMBA1000422//Human DNA from chromosome 19 specific cosmid R30292, genomic sequence, complete sequence.//9.2e-14:232:70//AC003112
F-HEMBA1000428//Homo sapiens Xp22 BAC GSHB-590J6 (Genome Systems Human BAC library) complete sequence.//3.8e-37:408:69//AC004554
F-HEMBA1000434//Caenorhabditis elegans cosmid Y48E1B, complete sequence.//0.73:454:57//Z93393
F-HEMBA1000442
F-HEMBA1000456//RPCI11-30J5.TV RPCI-11 Homo sapiens genomic clone RPCI-11-30J5, genomic survey sequence.//6.3e-06:62:96//B85188
F-HEMBA1000459//Mus musculus hemin-sensitive initiation factor 2 alpha kinase mRNA, complete cds.//6.8e-70:580:79//AF028808
F-HEMBA1000460//Homo sapiens PAC clone DJ0593H12 from 7p31, complete sequence.//2.8e-154:746:98//AC004839
F-HEMBA1000464//Homo sapiens, clone hRPK.15_A_1, complete sequence.//4.8e-25:397:72//AC006213
F-HEMBA1000469//CIT-HSP-2167P21.TF CIT-HSP Homo sapiens genomic clone 2167P21, genomic survey sequence.//4.0e-83:406:99//B94160
F-HEMBA1000488//Homo sapiens Chromosome 22q11.2 PAC Clone p_m11 In BCRL2-GGT Region, complete sequence.//4.2e-53:312:93//AC004033
F-HEMBA1000490//Campylobacter jejuni groES, groEL genes.//0.59:451:62//Y13334
F-HEMBA1000491//Murine sarcoma virus (Harvey-strain) H-ras transforming p21 gene.//8.6e-06:338:58//X00740
F-HEMBA1000501//Homo sapiens chromosome 17, clone hRPK.264_B_14, complete sequence.//9.4e-41:591:69//AC005884
F-HEMBA1000504//Homo sapiens mRNA for osteoblast specific factor 2 (OSF-2os).//4.0e-07:57:100//D13666
F-HEMBA1000505
F-HEMBA1000508//*** SEQUENCING IN PROGRESS *** Homo sapiens chromosome 4, BAC clone C0135005; HTGS phase 1, WORKING DRAFT SEQUENCE, 23 unordered pieces.//0.035:329:61//AC004661
F-HEMBA1000518//Caenorhabditis elegans cosmid C17H12.//0.96:425:58//AF045642
F-HEMBA1000519//Homo sapiens Xp22 BAC GSHB-536K7 (Genome Systems Human BAC library) complete sequence.//1.6e-53:300:89//AC004616
F-HEMBA1000520//Homo sapiens clone DJ0813F11, WORKING DRAFT SEQUENCE, 5 unordered pieces.//1.7e-10:117:86//AC006006
F-HEMBA1000523
F-HEMBA1000531//Mus musculus Hsp70-related NST-1 (hsr.1) mRNA, complete cds.//3.9e-35:290:80//U08215
F-HEMBA1000534//Homo sapiens chromosome 17, clone hRPK.177_H_5, WORKING DRAFT SEQUENCE, 2 ordered pieces.//1.7e-36:328:77//AC005973
F-HEMBA1000540//Arabidopsis thaliana DNA chromosome 4, BAC clone F7K2 (ESSAII project).//0.057:265:63//AL033545
F-HEMBA1000542//Rattus norvegicus mRNA for dipeptidyl peptidase III, complete cds.//1.2e-110:572:88//D89340
F-HEMBA1000545//Human DNA from cosmid L27h9, Huntington's Disease Region, chromosome 4p16.3 contains CpG island.//7.5e-130:780:89//Z49237
F-HEMBA1000555//Human DNA sequence *** SEQUENCING IN PROGRESS *** from clone 134O19, WORKING DRAFT SEQUENCE.//3.2e-175:838:98//AL034555
F-HEMBA1000557//CIT-HSP-2369F15.TF CIT-HSP Homo sapiens genomic clone 2369F15, genomic survey sequence.//2.8e-32:315:78//AQ074611
F-HEMBA1000561//Rattus norvegicus Olf-1/EBF associated Zn finger protein Roaz mRNA, alternatively spliced form, complete cds.//3.4e-69:665:72//U92564
F-HEMBA1000563//Plasmodium falciparum 3D7 chromosome 12 PFYAC1383 genomic sequence, WORKING DRAFT SEQUENCE, 3 unordered pieces.//0.59:261:61//AC005504
F-HEMBA1000568//HS_3243_B2_A12_MR CIT Approved Human Genomic Sperm Library D Homo sapiens genomic clone Plate=3243 Col=24 Row=B, genomic survey sequence.//3.1e-54:323:91//AQ219628
F-HEMBA1000569//M.musculus mRNA for GPI-anchored protein.//1.4e-19:440:61//X89571
F-HEMBA1000575//Plasmodium falciparum 3D7 chromosome 12 PFYAC357 genomic sequence, WORKING DRAFT SEQUENCE, 7 unordered pieces.//0.0016:557:57//AC005506
F-HEMBA1000588//Mus musculus FLI-LRR associated protein-1 mRNA, complete cds.//1.7e-11:132:79//AF045573
F-HEMBA1000591//Homo sapiens mRNA for E1B-55kDa-associated protein.//7.3e-43:228:97//AJ007509
F-HEMBA1000592//Mus musculus clone OST7314, genomic survey sequence.//7.3e-07:68:94//AF046733
F-HEMBA1000594//Human DNA sequence from PAC 306D1 on chromosome X contains ESTs.//8.7e-71:553:79//Z83822
F-HEMBA1000604//Human DNA sequence *** SEQUENCING IN PROGRESS *** from clone 237J2, WORKING DRAFT SEQUENCE.//2.9e-21:158:75//AL021394
F-HEMBA1000608//Homo sapiens mRNA for KIAA0456 protein, partial cds.//1.1e-118:561:99//AB007925
F-HEMBA1000622//Homo sapiens Chromosome 16 BAC clone CIT987SK-A-152E5, complete sequence.//2.2e-28:426:70//AC004382
F-HEMBA1000636//Human CpG island sequence, clone Q28B8.//1.0e-15:274:68//D85773
F-HEMBA1000637//Homo sapiens mRNA for KIAA0690 protein, partial cds.//6.7e-137:639:99//AB014590
F-HEMBA1000655//, complete sequence.//5.1e-83:685:80//AC005815
F-HEMBA1000657//Rattus norvegicus ADP-ribosylation factor-directed GTPase activating protein mRNA, complete cds.//1.1e-91:597:84//U35776
F-HEMBA1000662//Homo sapiens clone DJ0853H20, WORKING DRAFT SEQUENCE, 5 unordered pieces.//0.019:695:57//AC004907
F-HEMBA1000673//Human DNA sequence *** SEQUENCING IN PROGRESS *** from clone 229A8, WORKING DRAFT SEQUENCE.//1.5e-48:325:85//Z86090
F-HEMBA1000682//Homo sapiens (subclone 5_g5 from P1 H25) DNA sequence.//7.7e-61:615:74//L43411
F-HEMBA1000686
F-HEMBA1000702
F-HEMBA1000705//Plasmodium falciparum 3D7 chromosome 12 PFYACB8-628 genomic sequence, WORKING DRAFT SEQUENCE, 9 unordered pieces.//0.0037:569:57//AC005507
F-HEMBA1000719//Streptomyces coelicolor cosmid 1C2.//2.0e-09:483:62//AL031124
F-HEMBA1000722//Toxoplasma gondii chloroplast, complete genome.//0.00058:762:57//U87145
F-HEMBA1000726//H.sapiens HLA-DRB1*15 gene.//9.8e-49:189:89//X88791
F-HEMBA1000727//CIT-HSP-387P22.TRB CIT-HSP Homo sapiens genomic clone 387P22, genomic survey sequence.//0.0054:206:67//B60158
F-HEMBA1000747
F-HEMBA1000749//Human DNA sequence from clone 522P13 on chromosome 6p21.31-22.3. Contains a 60S Ribosomal Protein L21 pseudogene and an HNRNP A3 (Heterogenous Nuclear Riboprotein A3, FBRNP) pseudogene. Contains ESTs, STSs and GSSs, complete sequence.//3.3e-05:124:75//AL024509
F-HEMBA1000752//Human Chromosome X, complete sequence.//5.9e-48:502:75//AC004073
F-HEMBA1000769//Homo sapiens clone NH0576N21, WORKING DRAFT SEQUENCE, 5 unordered pieces.//0.011:179:67//AC005043
F-HEMBA1000773//Caenorhabditis elegans DNA *** SEQUENCING IN PROGRESS *** from clone Y59A8, WORKING DRAFT SEQUENCE.//0.070:231:63//Z98870
F-HEMBA1000774//Homo sapiens PAC clone DJ1059M17 from 7q21-q31.1, complete sequence.//6.2e-40:385:75//AC004953
F-HEMBA1000791
F-HEMBA1000817//Myrmecia pilosula HI87-135 mitochondrion cytochrome b gene, partial cds.//0.99:244:58//U15678
F-HEMBA1000822//Human DNA sequence from PAC 179D3, between markers DXS6791 and DXS8038 on chromosome X contains S10 GTP-binding protein, ESTs and CpG island.//0.033:294:62//Z81370
F-HEMBA1000827//Borrelia burgdorferi (section 50 of 70) of the complete genome.//9.7e-05:463:58//AE001164
F-HEMBA1000843//Homo sapiens DNA sequence from clone 511B24 on chromosome 20q11.2-12. Contains the TOP1 gene for Topoisomerase I, the PLCG1 gene for 1-Phosphatidylinositol-4,5-Bisphosphate Phosphodiesterase Gamma 1 (EC 3.1.4.11, PLC-Gamma-1, Phospholipase C-Gamma-1 PLC-II, PLC-148), the KIAA0395 gene for a probable Zinc Finger Homeobox protein and a 60S Ribosomal Protein L23 LIKE pseudogene. Contains a predicted CpG island, ESTs, STSs and GSSs, complete sequence.//3.0e-153:732:98//AL022394
F-HEMBA1000851//Rattus norvegicus glucocorticoid modulatory element binding protein 2 mRNA, complete cds.//1.6e-31:386:72//AF059273
F-HEMBA1000852//Homo sapiens Xp22 bins 3-5 PAC RPCI4-617A9 (Roswell Park Cancer Institute Human PAC Library) containing Arylsulfatase D and E genes, complete sequence.//8.5e-115:455:98//AC005295
F-HEMBA1000867
F-HEMBA1000869//Human DNA sequence from cosmid J138O17, between markers DXS6791 and DXS8038 on chromosome X contains EST CA repeat and an endogenous retroviral like element.//6.6e-41:424:75//Z72519
F-HEMBA1000870//Gnamptodon pumilio cytochrome oxidase II gene, partial cds; and tRNA-Asp, tRNA-His, and tRNA-Lys genes, complete sequence, mitochondrial genes for mitochondrial products.//0.0049:211:66//AF034598
F-HEMBA1000872//CIT-HSP-2355D20.TF CIT-HSP Homo sapiens genomic clone 2355D20, genomic survey sequence.//3.7e-33:180:98//AQ059583
F-HEMBA1000876//Human DNA sequence *** SEQUENCING IN PROGRESS *** from clone 473B4, WORKING DRAFT SEQUENCE.//5.6e-37:262:72//Z83826
F-HEMBA1000908//Triticum aestivum low-affinity cation transporter (LCT1) mRNA, complete cds.//1.0:304:59//AF015523
F-HEMBA1000910//M.musculus necdin mRNA, complete cds.//6.1e-08:256:61//M80840
F-HEMBA1000918//Tetrahymena thermophila micronuclear developmentally eliminated sequence region.//0.13:232:63//U88158
F-HEMBA1000919//Gallus domesticus filamin mRNA, complete cds.//1.0:213:65//U00147
F-HEMBA1000934//CIT-HSP-2053H24.TR CIT-HSP Homo sapiens genomic clone 2053H24, genomic survey sequence.//5.5e-11:275:64//B69224
F-HEMBA1000942//Homo sapiens clone DJ0754G14, WORKING DRAFT SEQUENCE, 15 unordered pieces.//9.7e-05:78:83//AC004878
F-HEMBA1000943//Homo sapiens chromosome 17, clone hRPK.640_I_15, complete sequence.//5.8e-140:661:99//AC005324
F-HEMBA1000946
F-HEMBA1000960//Homo sapiens clone DJ1111F22, WORKING DRAFT SEQUENCE, 12 unordered pieces.//8.3e-16:181:75//AC004967
F-HEMBA1000968//Human DNA sequence *** SEQUENCING IN PROGRESS *** from clone 69M21, WORKING DRAFT SEQUENCE.//4.4e-117:398:86//AL031735
F-HEMBA1000971//H.sapiens CpG island DNA genomic Mse1 fragment, clone 182f4, forward read cpg182f4 ft1a.//1.5e-20:126:96//Z57528
F-HEMBA1000972//Homo sapiens genomic DNA of 8p21.3-p22 anti-oncogene of hepatocellular colorectal and non-small cell lung cancer , segment 1/11.//0.34:642:59//AB020858
F-HEMBA1000974//Homo sapiens clone DA0091H08, complete sequence.//5.1e-183:865:98//AC004817
F-HEMBA1000975//Orf virus homologue of retroviral pseudoprotease gene, complete cds.//0.00065:391:62//M30023
F-HEMBA10009851/Human DNA sequence from clone 272E8 on chromosome Xp22.13-22.31. Contains a pseudogene similar to MDM2-Like P53-binding protein gene. Contains STSs, GSSs and a CA repeat polymorphism, complete sequence.//3.4e-05:243:65//Z93929
F-HEMBA1000986//Homo sapiens DNA from chromosome 19-cosmid R31491, genomic sequence.//6.6e-06:508:61//AD000813
F-HEMBA1000991//Homo sapiens mRNA for Hrs, complete cds.//1.2e-22:193:84//D84064 F-HEMBA1001007
F-HEMBA1001008//Human DNA sequence from clone 391O22 on chromosome 6p21.2-21.31 Contains pseudogenes similar to ribosomal protein, ESTs, GSSs, complete sequence.//7.8e-46:532:73//AL031577
F-HEMBA1001009//Human mRNA for IgM heavy chain complete sequence.//0.97:369:59//X17115
F-HEMBA1001017//Homo sapiens mRNA for KIAA0468 protein, complete cds.//4.4e-139:661:98//AB007937
F-HEMBA1001019//Homo sapiens, clone hRPK.15_A_1, complete sequence.//1.6e-16:521:64//AC006213
F-HEMBA1001020//Homo sapiens chromosome 17, clone hRPK.178_C_3, complete sequence.//3.8e-50:367:72//AC005702
F-HEMBA1001022
F-HEMBA1001024//Homo sapiens T-cell receptor alpha delta locus from bases 1 to 250529 (section 1 of 5) of the Complete Nucleotide Sequence.//5.0e-23:378:69//AE000658
F-HEMBA1001026//Homo sapiens DNA sequence from PAC 435D1 on chromosome Xq25. Contains ESTs and STS.//7.6e-19:867:60//Z86064
F-HEMBA1001043//HS_2219_B1_A10_MF CIT Approved Human Genomic Sperm Library D Homo sapiens genomic clone Plate=2219 Col=19 Row=B, genomic survey sequence.//3.0e-15:124:88//AQ301521
F-HEMBA1001051//Human Chromosome X clone bWXD342, complete sequence.//4.8e-79:308:84//AC004072
F-HEMBA1001052//Homo sapiens chromosome 17, clone hRPK.146_P_2, WORKING DRAFT SEQUENCE, 4 unordered pieces.//0.53:384:61//AC005341
F-HEMBA1001059//Human N-acetylgalactosamine 6-sulphatase (GALNS) gene, exon 10.//2.8e-26:397:71//U06084
F-HEMBA1001060//Homo sapiens chromosome 17, clone hRPK.855_D_21 complete sequence.//0.98:280:62//AC006079
F-HEMBA1001071//Human mRNA for pro alpha 1 (III) collagen C-terminal propeptide.//1.1e-31:181:96//X01742
F-HEMBA1001077//nuclear protein TIF1 [mice, mRNA, 3951 nt].//3.6e-13:338:65//S78219
F-HEMBA1001080//Streptomyces coelicolor cosmid 1A9.//0.00012:364:63//AL034446
F-HEMBA1001085//Human Chromosome 15q26.1 PAC clone pDJ290i21 containing fur, fes, and alpha mannosidase IIx genes, WORKING DRAFT SEQUENCE, 9 unordered pieces.//8.5e-134:476:96//AC004586
F-HEMBA1001088//Sequence 1 from patent US 5552529.//2.2e-71:303:78//I25863
F-HEMBA1001094//Homo sapiens clone RG491N20, complete sequence.//8.9e-119:609:96//AC005105
F-HEMBA1001099
F-HEMBA1001109//Homo sapiens BAC clone RG318M05 from 7q22-q31.1, complete sequence.//2.4e-58:347:87//AC005250
F-HEMBA1001121//Human DNA sequence *** SEQUENCING IN PROGRESS *** from clone 90G24, WORKING DRAFT SEQUENCE.//3.4e-21:226:65//AL008723
F-HEMBA1001122//Plasmodium falciparum chromosome 2, section 20 of 73 of the complete sequence.//9.2e-07:732:57//AE001383
F-HEMBA1001123//Homo sapiens full-length insert cDNA clone ZD38E12.//1.1e-11:231:68//AF086247
F-HEMBA1001133//Homo sapiens clone DJ0856O24, WORKING DRAFT SEQUENCE, 4 unordered pieces.//0.011:163:69//AC004909
F-HEMBA1001137//Homo sapiens mRNA for KIAA0798 protein, complete cds.//6.9e-72:527:77//AB018341
F-HEMBA1001140//Homo sapiens clone RG228D17, WORKING DRAFT SEQUENCE, 2 unordered pieces.//2.3e-120:578:98//AC005077
F-HEMBA1001172//Plasmodium falciparum 3D7 chromosome 12 PFYACB8-628 genomic sequence, WORKING DRAFT SEQUENCE, 9 unordered pieces.//0.010:520:59//AC005507
F-HEMBA1001174//R.norvegicus (Sprague Dawley) ARL5 mRNA for ARF-like protein 5.//1.0e-59:565:73//X78604
F-HEMBA1001197//Homo sapiens clone 82F9, WORKING DRAFT SEQUENCE, 4 unordered pieces.//0.0037:151:70//AC004815
F-HEMBA1001208//Human BAC clone RG264L19 from 7p15-p21, complete sequence.//7.4e-35:195:81//AC002410
F-HEMBA1001213//Homo sapiens clone DJ0892G19, complete sequence.//1.9e-171:826:98//AC004917
F-HEMBA1001226//Homo sapiens clone DJ0850101, WORKING DRAFT SEQUENCE, 1 unordered pieces.//0.00010:557:57//AC006009
F-HEMBA1001235//Homo sapiens chromosome 17, clone hRPK.601_N_13, complete sequence.//0.0086:372:58//AC005389
F-HEMBA1001247//H.sapiens CpG island DNA genomic Mse1 fragment, clone 11b11, reverse read cpg11b11.rt1a.//2.0e-24:154:93//Z64441
F-HEMBA1001257//Homo sapiens alpha-methylacyl-CoA racemase mRNA, complete cds.//1.9e-88:659:81//AF047020
F-HEMBA1001265//Human 18S ribosomal RNA.//1.0e-32:180:97//X03205
F-HEMBA1001281
F-HEMBA1001286//B.taurus mRNA for RF-36-DNA-binding protein.//7.7e-26:236:81//X15543
F-HEMBA1001289//Homo sapiens Chromosome 16 BAC clone CIT987SK-A-69G12, complete sequence.//5.5e-28:530:64//AC004131
F-HEMBA1001294//Yeast mitochondrial aapl gene for ATPase subunit 8.//2.8e-15:722:60//X00960
F-HEMBA1001299//Human DNA sequence from clone 422G23 on chromosome 6q24 Contains EST, STS, GSS, CpG island, complete sequence.//4.2e-24:288:76//AL031003
F-HEMBA1001302//cDNA encoding a human homologue of a mouse novel polypeptide derived from stromal cell.//7.2e-121:439:96//E12260
F-HEMBA1001303//Plasmodium falciparum 3D7 chromosome 12 PFYAC181 genomic sequence, WORKING DRAFT SEQUENCE, 8 unordered pieces.//0.011:637:56//AC005505
F-HEMBA1001310//HS_3252_B2_B12_T7 CIT Approved Human Genomic Sperm Library D Homo sapiens genomic clone Plate=3252 Col=24 Row=D, genomic survey sequence.//1.2e-16:166:82//AQ217054
F-HEMBA1001319//CIT-HSP-2034J6.TF CIT-HSP Homo sapiens genomic clone 2034J6, genomic survey sequence.//0.33:256:59//B79408
F-HEMBA1001323//Homo sapiens proto-oncogene (Wnt-5a) mRNA, complete cds.//7.8e-30:165:99//L20861
F-HEMBA1001326//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs(BAC end sequences) and a CA repeat polymorphism, complete sequence.//5.4e-19:347:68//AL021368
F-HEMBA1001327//CIT-HSP-2354E10.TR CIT-HSP Homo sapiens genomic clone 2354E10, genomic survey sequence.//0.012:152:65//AQ075713
F-HEMBA1001330//Plasmodium falciparum DNA *** SEQUENCING IN PROGRESS *** from contig 3-103, complete sequence.//0.0037:254:62//AL010208
F-HEMBA1001351//Homo sapiens VAMP-associated protein of 33 kDa (VAP-33) mRNA, complete cds.//1.1e-103:516:97//AF057358
F-HEMBA1001361//Homo sapiens chromosome 9, clone hRPK.202_H_3, complete sequence.//1.7e-150:706:99//AC006241
F-HEMBA1001375//Streptomyces coelicolor cosmid 1E6.//1.0:375:59//AL033505
F-HEMBA1001377//HS_3020_B1_D12_MR CIT Approved Human Genomic Sperm Library D Homo sapiens genomic clone Plate=3020 Col=23 Row=H, genomic survey sequence.//0.00022:63:77//AQ105297
F-HEMBA1001383//Plasmodium falciparum chromosome 2, section 68 of 73 of the complete sequence.//0.00035:317:60//AE001431
F-HEMBA1001387//HS_3039_B1_D01_MF CIT Approved Human Genomic Sperm Library D Homo sapiens genomic clone Plate=3039 Col=1 Row=H, genomic survey sequence.//5.0e-90:437:98//AQ155035
F-HEMBA1001388//Homo sapiens clone RG189J21, WORKING DRAFT SEQUENCE, 15 unordered pieces.//4.2e-47:159:89//AC005073
F-HEMBA1001391//Human DNA sequence from clone 409O10 on chromosome 20q12 Contains CA repeat, GSS, STS, complete sequence.//2.0e-06:495:60//AL031256
F-HEMBA1001398//H.sapiens CpG island DNA genomic Mse1 fragment, clone 70d11, forward read cpg70d11.ft1b.//0.018:46:97//Z62591
F-HEMBA1001405//Human DNA sequence *** SEQUENCING IN PROGRESS *** from clone 50024, WORKING DRAFT SEQUENCE.//2.3e-74:623:71//AL034380
F-HEMBA1001407//Mus musculus domesticus Torino (Sry) gene, complete cds.//0.36:363:57//U03645
F-HEMBA1001411//Homo sapiens genomic DNA, 21q region, clone: S39BG29, genomic survey sequence.//8.4e-12:516:60//AG001050
F-HEMBA1001413
F-HEMBA1001415//Human DNA sequence *** SEQUENCING IN PROGRESS *** from clone 410I8, WORKING DRAFT SEQUENCE.//0.98:177:64//AL031732
F-HEMBA1001432//Homo sapiens clone DJ0693M11, WORKING DRAFT SEQUENCE, 7 unordered pieces.//8.0e-177:859:97//AC006146
F-HEMBA1001433//Homo sapiens clone DJ0892G19, complete sequence.//2.0e-35:376:64//AC004917
F-HEMBA1001435//Homo sapiens chromosome 17, clone hRPK.63_A_1, complete sequence.//1.2e-74:284:84//AC005670
F-HEMBA1001442//Plasmodium falciparum DNA *** SEQUENCING IN PROGRESS *** from contig 3-66, complete sequence.//0.056:194:63//AL010138
F-HEMBA1001446//Homo sapiens chromosome 4 clone B150J4 map 4q25, complete sequence.//0.96:328:61//AC004047
F-HEMBA1001450
F-HEMBA1001454//Human DNA sequence from clone 598A24 on chromosome Xp11.1-11.23 Contains zinc finger X-linked proteins ZXDA, ZXDB, ESTs and STS, complete sequence.//2.0e-47:468:73//AL031115
F-HEMBA1001455//CIT978SK-32J2.TV CIT978SK Homo sapiens genomic clone 32J2, genomic survey sequence.//1.5e-05:223:65//B78859
F-HEMBA1001463//cSRL-69d1-u cSRL flow sorted Chromosome 11 specific cosmid Homo sapiens genomic clone cSRL-69d1, genomic survey sequence.//5.1e-66:564:77//B05652
F-HEMBA1001476//Homo sapiens mRNA for KIAA0572 protein, partial cds.//1.9e-102:489:99//AB011144
F-HEMBA1001478//HS_2228_A2_B03_MF CIT Approved Human Genomic Sperm Library D Homo sapiens genomic clone Plate=2228 Col=6 Row=C, genomic survey sequence.//4.5e-40:275:88//AQ032041
F-HEMBA1001497//Human DNA sequence from clone 281H8 on chromosome 6q25.1-25.3. Contains up to four novel genes, one with similarity to KIAA0323 and worm C30F12.1 and another with Ubiquitin-Like protein gene SMT3 (the latter in an intron of a novel gene). Contains ESTs, STSs, GSSs, a putative CpG island and genomic marker D6S1553, complete sequence.//7.7e-47:311:85//AL031133
F-HEMBA1001510//Human HLA class III region containing cAMP response element binding protein-related protein (CREB-RP) and tenascin X (tenascin-X) genes, complete cds, complete sequence.//2.0e-130:699:93//U89337
F-HEMBA1001515//Homo sapiens chromosome 19, cosmid F24866, complete sequence.//4.1e-114:711:85//AC005794
F-HEMBA1001517//Homo sapiens BAC clone RG459N13 from 7p15, complete sequence.//5.7e-162:769:98//AC004549
F-HEMBA1001522//Caenorhabditis elegans cosmid ZK328.//8.6e-17:498:61//U50193
F-HEMBA1001526//Human DNA sequence from cosmid 444G9 from a contig from the tip of the short arm of chromosome 16, spanning 2Mb of 16p13.3 Contains ESTs and CpG islands,.//0.31:120:69//Z98258
F-HEMBA1001533
F-HEMBA1001557//Chionoecetes opilio (clone COP41) DNA microsatellite repeat regions.//7.0e-25:303:72//L49136
F-HEMBA1001566//Homo sapiens DNA sequence from PAC 127D3 on chromosome 1q23-25. Contains FMO2 and FMO3 genes for Flavin-containing Monooxygenase 2 and Flavin-containing Monooxygenase 3 (Dimethylaniline Monooxygenase (N-Oxide 3, EC1.14.13.8, Dimethylaniline Oxidase 3, FMO II, FMO 3), and a gene for another, unknown, Flavin-containing Monooxygenase family protein. Contains ESTs and GSSs, complete sequence.//7.2e-18:805:60//AL021026
F-HEMBA1001569//Homo sapiens mRNA for vesicle associated membrane protein 2 (VAMP2).//1.1e-64:338:95//AJ225044
F-HEMBA1001570//Homo sapiens PAC clone DJ0844F09 from 7p12-p13, complete sequence.//2.1e-148:698:99//AC004453
F-HEMBA1001579//Homo sapiens mRNA for NS1-binding protein (NS1-BP).//2.2e-173:678:99//AJ012449
F-HEMBA1001581//Homo sapiens clone DJ1158B01, WORKING DRAFT SEQUENCE, 23 unordered pieces.//0.30:484:59//AC004980
F-HEMBA1001585
F-HEMBA1001589//Human BAC clone RG317G18 from 7q31, complete sequence.//0.98:197:63//AC002432
F-HEMBA1001595//Human mRNA for KIAA0128 gene, partial cds.//8.2e-109:855:78//D50918
F-HEMBA1001608//RPCI11-72E2.TJ RPCI11 Homo sapiens genomic clone R-72E2, genomic survey sequence.//3.8e-05:235:64//AQ267131
F-HEMBA1001620//Oryza sativa RINO1 mRNA for myo-inositol phosphate synthase, complete cds.//3.8e-40:719:64//AB012107
F-HEMBA1001635//HS_3208_A1_D07_MR CIT Approved Human Genomic Sperm Library D Homo sapiens genomic clone Plate=3208 Col=13 Row=G, genomic survey sequence.//1.4e-15:120:90//AQ176944
F-HEMBA1001636//Homo sapiens 12q24 PAC RPCI1-66E7 (Roswell Park Cancer Institute Human PAC library) complete sequence.//0.15:221:64//AC004216
F-HEMBA1001640//HS_3253_B2_D03_MR CIT Approved Human Genomic Sperm Library D Homo sapiens genomic clone Plate=3253 Col=6 Row=H, genomic survey sequence.//9.1e-52:278:95//AQ216058
F-HEMBA1001647//H.sapiens gene for plectin.//0.00052:629:61//Z54367
F-HEMBA1001651//Salmo salar DNA for a cryptic repeat.//7.9e-08:270:64//AJ012206
F-HEMBA1001655//Homo sapiens chromosome 5, BAC clone 194j18 (LBNL H158), complete sequence.//5.9e-164:802:97//AC005368
F-HEMBA1001658//M.musculus COL3A1 gene for collagen alpha-I.//2.4e-30:742:62//X52046
F-HEMBA1001661//Homo sapiens chromosome 5p, BAC clone 50g21 (LBNL H154), complete sequence.//2.2e-144:682:99//AC005740
F-HEMBA1001672//Homo sapiens methyl-CpG binding protein MBD3 (MBD3) mRNA, complete cds.//6.1e-152:725:98//AF072247
F-HEMBA1001675//RPCI11-54F8.TV RPCI11 Homo sapiens genomic clone R-54F8, genomic survey sequence.//5.3e-75:341:85//AQ082126
F-HEMBA1001678//Homo sapiens Xp22 PAC RPCI1-167A22 (from Roswell Park Cancer Center) complete sequence.//8.4e-54:551:74//AC002349
F-HEMBA1001681
F-HEMBA1001702//Plasmodium falciparum chromosome 2, section 35 of 73 of the complete sequence.//0.94:676:54//AE001398
F-HEMBA1001709//Human DNA sequence *** SEQUENCING IN PROGRESS *** from clone 702J19, WORKING DRAFT SEQUENCE.//0.74:659:58//AL033531
F-HEMBA1001711//Lysiphlebus melandriicola NADH dehydrogenase 1 gene, mitochondrial gene encoding mitochondrial protein, partial cds.//3.0e-07:413:60//AF069178
F-HEMBA1001712//Homo sapiens BAC clone RG041H04 from 7q21-q22, complete sequence.//0.091:315:61//AC004519
F-HEMBA1001714//Rattus norvegicus mitochondrial ATPase inhibitor gene, complete cds.//1.6e-28:218:75//U12250
F-HEMBA1001718//HS_3056_A2_H08_MF CIT Approved Human Genomic Sperm Library D Homo sapiens genomic clone Plate=3056 Col=16 Row=O, genomic survey sequence.//2.0e-79:383:99//AQ106367
F-HEMBA1001723//HS_2188_A2_D02_MF CIT Approved Human Genomic Sperm Library D Homo sapiens genomic clone Plate=2188 Col=4 Row=G, genomic survey sequence.//3.8e-28:174:94//AQ116793
F-HEMBA1001731//HS_3021_A1_A11_T7 CIT Approved Human Genomic Sperm Library D Homo sapiens genomic clone Plate=3021 Col=21 Row=A, genomic survey sequence.//2.5e-11:420:62//AQ154658
F-HEMBA1001734//Homo sapiens chromosome Y, clone 264,M,20, complete sequence.//0.00060:392:60//AC004617
F-HEMBA1001744//HS_3194_A1_D05_T7 CIT Approved Human Genomic Sperm Library D Homo sapiens genomic clone Plate=3194 Col=9 Row=G, genomic survey sequence.//5.8e-29:163:97//AQ252295
F-HEMBA1001745//Homo sapiens chromosome 9q34, clone 280C11, complete sequence.//0.66:627:59//AC002102
F-HEMBA1001746//HS_2163_B1_F04_MR CIT Approved Human Genomic Sperm Library D Homo sapiens genomic clone Plate=2163 Col=7 Row=L, genomic survey sequence.//1.4e-16:238:70//AQ085995
F-HEMBA1001761//Genomic sequence from Mouse 9, complete sequence.//3.5e-52:198:86//AC002109
F-HEMBA1001781
F-HEMBA1001784//Genomic sequence from Human 9q34, WORKING DRAFT SEQUENCE, 2 unordered-pieces.//5.5e-13:296:65//AC002099
F-HEMBA1001791//Homo sapiens DNA from chromosome 19-cosmids R31158, R31874, and R28125, genomic sequence, complete sequence.//0.18:534:59//AF038458
F-HEMBA1001800//CrT-HFP-2049N5.TF CIT-HSP Homo sapiens genomic clone 2049N5, genomic survey sequence.//2.2e-40:335:80//AQ009222
F-HEMBA1001803//M.musculus (Ba1b/C) P/L01 mRNA.//1.7e-25:286:74//Z31360
F-HEMBA1001804//Mouse interleukin 2 receptor (p55 IL-2R) mRNA, 5' end.//1.9e-58:358:89//M21977
F-HEMBA1001808//Homo sapiens mRNA, chromosome 1 specific transcript KIAA0500.//7.8e-174:809:98//AB007969
F-HEMBA1001809//Bovine herpesvirus 1 complete genome.//9.0e-09:639:57//AJ004801
F-HEMBA1001815
F-HEMBA1001819//HS_3079_B1_E04_T7 CIT Approved Human Genomic Sperm Library D Homo sapiens genomic clone Plate=3079 Col=7 Row=J, genomic survey sequence.//1.4e-79:396:97//AQ186616
F-HEMBA1001820//Homo sapiens BAC clone GS165L15 from 7p15, complete sequence.//0.00026:436:60//AC005013
F-HEMBA1001822//Homo sapiens intersectin short form mRNA, complete cds.//1.2e-40:510:65//AF064243
F-HEMBA1001824//Homo sapiens expanded SCA7 CAG repeat.//6.1e-20:344:68//AF020275
F-HEMBA1001835//Homo sapiens BAC clone RG017K18 from 7q31, complete sequence.//0.0094:553:58//AC005161
F-HEMBA1001844//Homo sapiens chromosome Xp22-135-136 clone GSHB-567I1, WORKING DRAFT SEQUENCE, 35 unordered pieces.//1.2e-22:316:70//AC005867
F-HEMBA1001847//M.musculus Zfp-29 gene for zinc finger protein.//5.3e-27:397:69//X55126
F-HEMBA1001861//Homo sapiens mRNA for KIAA0617 protein, complete cds.//8.8e-184:865:98//AB014517
F-HEMBA1001864//Arabidopsis thaliana chromosome II BAC F17H15 genomic sequence, complete sequence.//0.38:337:62//AC005395
F-HEMBA1001866//Caenorhabditis elegans cosmid F48E3.//1.4e-10:224:63//U28735
F-HEMBA1001869//Homo sapiens BAC clone RG114B19 from 7q31.1, complete sequence.//6.7e-98:288:91//AC005065
F-HEMBA1001888//Human Chromosome 11p15.5 PAC clone pDJ915f1 containing KvLQT1 gene, complete sequence.//4.9e-114:476:84//AC003693
F-HEMBA1001896//Bos taurus pyruvate dehydrogenase phosphatase regulatory subunit precursor, mRNA, complete cds.//2.2e-137:839:86//AF026954
F-HEMBA1001910//Homo sapiens Chromosome 2p13 BAC Clone h173, complete sequence.//0.90:221:63//AC003065
F-HEMBA1001912//HS_2237_A1_C10_MF CIT Approved Human Genomic Sperm Library D Homo sapiens genomic clone Plate=2237 Col=19 Row=E, genomic survey sequence.//9.7e-76:364:100//AQ033732
F-HEMBA1001913//Leishmania major chromosome 3 clone L4625 strain Friedlin, WORKING DRAFT SEQUENCE, 6 unordered pieces.//0.00063:219:65//AC005766
F-HEMBA1001915//Homo sapiens genomic DNA of 9q32 anti-oncogene of flat epitherium cancer , segment 5/10.//0.00011:366:63//AB020873
F-HEMBA1001918//Pneumocystis carinii gene for major surface glycoprotein MSG105, exon1-2, complete cds.//0.00024:562:58//D82031
F-HEMBA1001921//Homo sapiens germinal center kinase related protein kinase mRNA, complete cds.//2.1e-184:855:99//AF000145
F-HEMBA1001939//Human DNA sequence from clone 395P12 on chromosome 1q24-25. Contains the TXGP1 gene for tax-transcriptionally activated glycoprotein 1 (34kD) (OX40 ligand, OX40L) and a GOT2 (Aspartate Aminotransferase, mitochondrial precursor, EC 2.6.1.1, Transaminase A, Glutamate Oxaloacetate Transaminase-2) pseudogene. Contains ESTs, STSs and GSSs, complete sequence.//1.1e-42:380:80//AL022310
F-HEMBA1001940//Homo sapiens clone DJ1093I16, WORKING DRAFT SEQUENCE, 5 unordered pieces.//7.5e-175:861:97//AC005629
F-HEMBA1001942//Homo sapiens chromosome 12p13.3 clone RPCI1-96H9, WORKING DRAFT SEQUENCE, 66 unordered pieces.//0.097:107:71//AC006057
F-HEMBA1001945//Drosophila F family transposable element F12 3' region.//0.94:140:65//X01934
F-HEMBA1001950//H.sapiens CpG island DNA genomic Mse1 fragment, clone 15b5, forward read cpg15b5.ft1q.//1.4e-27:168:95//Z54728
F-HEMBA1001960//Locusta migratoria mRNA for nAChR alpha1 subunit.//0.010:108:71//AJ000390
F-HEMBA1001962//Plasmodium falciparum 3D7 chromosome 12 PFYACB8-628 genomic sequence, WORKING DRAFT SEQUENCE, 9 unordered pieces.//9.7e-05:494:60//AC005507
F-HEMBA1001964
F-HEMBA1001967//Human DNA sequence from clone 341E18 on chromosome 6p11.2-12.3. Contains a Serine/Threonine Protein Kinase gene (presumptive isolog of a Rat gene) and a novel alternatively spliced gene. Contains a putative CpG island, ESTs and GSSs, complete sequence.//9.6e-122:373:99//AL031178
F-HEMBA1001979//HS_3067_B1_A06_MR CIT Approved Human Genomic Sperm Library D Homo sapiens genomic clone Plate=3067 Col=11 Row=B, genomic survey sequence.//0.43:193:64//AQ143506
F-HEMBA1001987//Plasmodium falciparum MAL3P6, complete sequence.//1.0:428:56//Z98551
F-HEMBA1001991//HS_2237_A2_G09_MF CIT Approved Human Genomic Sperm Library D Homo sapiens genomic clone Plate=2237 Col=18 Row=M, genomic survey sequence.//4.3e-05:240:64//AQ067283
F-HEMBA1002003//protein phosphatase 2C isoform [rats, liver, mRNA, 1950 nt].//2.7e-33:364:74//S90449
F-HEMBA1002008//WORKING DRAFT SEQUENCE, 3 unordered pieces.//0.00032:214:68//AC005948
F-HEMBA1002018
F-HEMBA10020227/Human p37NB mRNA, complete cds.//0.014:58:96//U32907
F-HEMBA1002035//Mouse transcriptional control element.//7.8e-07:200:69//M17284
F-HEMBA1002039//Human DNA sequence from clone 267M20 on chromosome Xq22.2-22.3. Contains part of the DIAPH2 gene and a pseudogene, ESTs, STSs and GSSs, complete sequence.//0.31:497:58//AL031053
F-HEMBA1002049//Homo sapiens chromosome 5, BAC clone 282B7 (LBNL H192), complete sequence.//4.5e-42:532:63//AC005216
F-HEMBA1002084//Homo sapiens chromosome 19 cosmid F15386, genomic sequence, complete sequence.//0.81:435:59//AF025422
F-HEMBA1002092//Mus musculus Olf-1/EBF-like-3 transcription factor (O/E-3) mRNA, complete cds.//7.2e-130:769:87//U92703
F-HEMBA1002100//Homo sapiens PAC clone DJ0991G20, complete sequence.//1.3e-47:124:96//AC004943
F-HEMBA1002102//Xenopus laevis mRNA for xSox7 protein, complete cds.//2.7e-13:132:71//D83649
F-HEMBA1002113//F.rubripes GSS sequence, clone 063K10bB4, genomic survey sequence.//0.029:142:66//Z88840
F-HEMBA1002119//Human Chromosome 11 pac pDJ1173a5, complete sequence.//1.3e-14:515:62//AC000378
F-HEMBA1002125//Homo sapiens calcium-activated potassium channel (KCNN3) mRNA, complete cds.//0.98:222:61//AF031815
F-HEMBA1002139//Caenorhabditis elegans cosmid F55C9, complete sequence.//0.0081:371:60//Z81549
F-HEMBA1002144//Saccharomyces cerevisiae mitochondrion transfer RNA-Met (tRNA-Met) gene, oxil gene, and ORF1.//4.9e-06:341:61//L36888
F-HEMBA1002150//Homo sapiens mRNA for KIAA0720 protein, partial cds.//0.00017:353:62//AB018263
F-HEMBA1002151
F-HEMBA1002153//CITBI-E1-2519120.TR CITBI-E1 Homo sapiens genomic clone 2519I20, genomic survey sequence.//8.5e-61:334:94//AQ277613
F-HEMBA1002160//Homo sapiens clone DJ1189D06, complete sequence.//8.5e-44:385:77//AC005232
F-HEMBA1002161//Coturnix coturnix slow myosin heavy chain 2 (qmyhc2) mRNA, partial cds.//2.1e-59:571:74//AF006829
F-HEMBA1002162//Homo sapiens Xp22-150 BAC GSHB-309P15 (Genome Systems Human BAC Library) complete sequence.//5.3e-53:698:67//AC006210
F-HEMBA1002166//Human DNA sequence from PAC 84F12 on chromosome Xq25-Xq26.3. Contains glypican-3 precursor (intestinal protein OCI-5) (GTR2-2), ESTs and CA repeat.//1.2e-50:319:78//AL008712
F-HEMBA1002177//Homo sapiens BAC clone RG293F11 from 7q21-7q22, complete sequence.//2.5e-18:150:88//AC000066
F-HEMBA1002185//Homo sapiens clone DJ0292L20, WORKING DRAFT SEQUENCE, 2 unordered pieces.//0.00066:466:59//AC004825
F-HEMBA1002189//Homo sapiens clone GS166C05, WORKING DRAFT SEQUENCE, 7 unordered pieces.//3.3e-23:176:77//AC005015
F-HEMBA1002191//Homo sapiens mRNA for KIAA0689 protein, partial cds.//1.0:382:59//AB014589
F-HEMBA1002199//Homo sapiens chromosome 4 clone B55B24 map 4q25, complete sequence.//1.8e-20:368:66//AC005150
F-HEMBA1002204//HS_2055_A1_H09_T7 CIT Approved Human Genomic Sperm Library D Homo sapiens genomic clone Plate=2055 Col=17 Row=O, genomic survey sequence.//1.2e-06:178:65//AQ235350
F-HEMBA1002212//S.cerevisiae chromosome IV reading frame ORF YDL101c.//0.035:345:60//Z74149
F-HEMBA1002215//M.musculus mRNA for testin.//4.6e-80:504:87//X78989
F-HEMBA1002226//Homo sapiens Xp22 bins 87-93 PAC RPCI1-122K4 (Roswell Park Cancer Institute Human PAC Library) complete sequence.//5.7e-63:336:74//AC003035
F-HEMBA1002229//Homo sapiens BAC clone NH0539B24 from 7p15.1-p14, complete sequence.//2.6e-39:311:81//AC006044
F-HEMBA1002237//Homo sapiens PAC clone DJ0696N01 from 7p21-p22, complete sequence.//1.6e-12:397:64//AC004861
F-HEMBA1002241
F-HEMBA1002253
F-HEMBA1002257//Homo sapiens diacylglycerol kinase iota (DGKi) mRNA, complete cds.//3.5e-151:731:97//AF061936
F-HEMBA1002265//Human DNA sequence from cosmid N28H9 on chromosome 22q11.2-qter contains ESTs, STS and endogenous retrovirus.//1.3e-09:313:62//Z71183
F-HEMBA1002267
F-HEMBA1002270//Homo sapiens Xp22-150 BAC GSHB-309P15 (Genome Systems Human BAC Library) complete sequence.//0.069:495:58//AC006210
F-HEMBA1002321//Homo sapiens PAC clone DJ0991O23, complete sequence.//0.019:564:58//AC004944
F-HEMBA1002328//CIT-HSP-2387N15.TF.1 CIT-HSP Homo sapiens genomic clone 2387N15, genomic survey sequence.//1.8e-71:346:99//AQ240836
F-HEMBA1002337//Arabidopsis thaliana genomic DNA, chromosome 5, P1 clone: MYN8, complete sequence.//0.84:547:57//AB020754
F-HEMBA1002341//Homo sapiens mRNA for KIAA0771 protein, partial cds.//2.4e-185:872:98//AB018314
F-HEMBA1002348//CIT-HSP-2372K24.TR CIT-HSP Homo sapiens genomic clone 2372K24, genomic survey sequence.//9.1e-33:230:75//AQ110676
F-HEMBA1002349//Plasmodium falciparum histidine-rich protein II (HRP II) gene, complete cds.//9.4e-06:504:57//U69551
F-HEMBA1002363//Homo sapiens chromosome-associated protein-E (hCAP-E) mRNA, complete cds.//7.3e-188:872:99//AF092563
F-HEMBA1002381//Homo sapiens genomic DNA of 8p21.3-p22 anti-oncogene of hepatocellular colorectal and non-small cell lung cancer , segment 11/11.//2.1e-20:262:72//AB020868
F-HEMBA1002389//D.discoideum spore coat 60 (sp60) gene, 5' flank.//0.010:95:73//M34546
F-HEMBA1002417//Canis familiaris ZO-3 (zo-3) mRNA, complete cds.//6.2e-120:767:85//AF023617
F-HEMBA1002419//HS-1047-A1-F01-MF.abi CIT Human Genomic Sperm Library C Homo sapiens genomic clone Plate=CT 830 Col=1 Row=K, genomic survey sequence.//7.6e-06:111:76//B38165
F-HEMBA1002430//HS_3137_B2_F10_T7 CIT Approved Human Genomic Sperm Library D Homo sapiens genomic clone Plate=3137 Col=20 Row=L, genomic survey sequence.//1.6e-56:367:88//AQ148697
F-HEMBA1002439//Dictyostelium discoideum actin 8 gene, 3' UTR.//0.67:129:64//M25216
F-HEMBA1002458//Mus musculus REX-3 mRNA, complete cds.//1.1e-30:274:72//AF051347
F-HEMBA1002460//Homo sapiens clone DJ1137M13, complete sequence.//4.0e-173:822:98//AC005378
F-HEMBA1002462//Sequence 41 from patent US 5708157.//9.8e-51:519:73//I80067
F-HEMBA1002469//Human mRNA for KIAA0122 gene, partial cds.//4.0e-108:603:92//D50912
F-HEMBA1002475//Streptomyces coelicolor cosmid 2H4.//0.0068:626:57//AL031514
F-HEMBA1002477//Homo sapiens BAC clone NH0342K06 from 2, complete sequence.//1.5e-40:349:78//AC005034
F-HEMBA1002486
F-HEMBA1002495//HS_3218_B1_A12_T7 CIT Approved Human Genomic Sperm Library D Homo sapiens genomic clone Plate=3218 Col=23 Row=B, genomic survey sequence.//1.0:179:67//AQ181410
F-HEMBA1002498//Homo sapiens full-length insert cDNA clone ZD76B01.//1.4e-129:619:98//AF086404
F-HEMBA1002503//Homo sapiens clone DJ0742P04, WORKING DRAFT SEQUENCE, 6 unordered pieces.//1.9e-24:306:68//AC004873
F-HEMBA1002508//Homo sapiens chromosome 19, cosmid R33516, complete sequence.//2.9e-76:464:83//AC004799
F-HEMBA1002513//Homo sapiens mRNA for histone deacetylase-like protein (JM21).//2.8e-157:738:98//AJ011972
F-HEMBA1002515//Human DNA sequence *** SEQUENCING IN PROGRESS *** from clone 407F11, WORKING DRAFT SEQUENCE.//2.6e-07:307:64//AL022329
F-HEMBA1002538//HS_2185_B2_B04_MR CIT Approved Human Genomic Sperm Library D Homo sapiens genomic clone Plate=2185 Col=8 Row=D, genomic survey sequence.//4.7e-37:339:78//AQ298315
F-HEMBA1002542//HS_3197_B2_B10_T7 CIT Approved Human Genomic Sperm Library D Homo sapiens genomic clone Plate=3197 Col=20 Row=D, genomic survey sequence.//3.2e-70:372:95//AQ188792
F-HEMBA1002547//Homo sapiens agrin precursor mRNA, partial cds.//3.5e-137:655:98//AF016903
F-HEMBA1002552//Human Hep27 protein mRNA, complete cds.//8.8e-07:173:68//U31875
F-HEMBA1002555//*** SEQUENCING IN PROGRESS *** Homo sapiens chromosome 4, BAC clone C0190L06; HTGS phase 1, WORKING DRAFT SEQUENCE, 21 unordered pieces.//2.2e-15:628:60//AC004670
F-HEMBA1002558//Human Xp22 BAC CT-285I15 (from CalTech/Research Genetics) , PAC RPCI1-27C22 (from Roswell Park Cancer Center), and Cosmid U35B5 (from Lawrence Livermore), complete sequence.//2.3e-41:353:76//AC002366
F-HEMBA1002561//Homo sapiens chromosome 17, clone HRPC29G21, complete sequence.//1.1e-39:538:66//AC003687
F-HEMBA1002569//Homo sapiens protein associated with Myc mRNA, complete cds.//1.3e-140:457:99//AF075587
F-HEMBA1002583//CIT-HSP-2321D3.TR CIT-HSP Homo sapiens genomic clone 2321D3, genomic survey sequence.//5.1e-79:385:99//AQ038102
F-HEMBA1002590//Homo sapiens chromosome 17, clone hRPK.167_N_20, complete sequence.//1.9e-35:430:70//AC005940
F-HEMBA1002592//Human genomic DNA sequence from clone 308O1 on chromosome Xp11.3-11.4. Contains EST, CA repeat, STS, GSS, CpG island.//4Ae-19:303:71//Z93403
F-HEMBA1002609//Homo sapiens mRNA for KIAA0597 protein, partial cds.//4.4e-175:820:99//AB011169
F-HEMBA1002621//Homo sapiens PAC clone DJ0650P09 from 7q21, complete sequence.//0.14:353:58//AC004413
F-HEMBA1002624//Homo sapiens mRNA for KIAA0808 protein, complete cds.//2.9e-187:632:97//AB018351
F-HEMBA1002628//Plasmodium falciparum 3D7 chromosome 12 PFYAC812 genomic sequence, WORKING DRAFT SEQUENCE, 8 unordered pieces.//1.5e-05:792:58//AC004153
F-HEMBA1002629//Streptomyces coelicolor cosmid 1A9.//8.4e-08:576:58//AL034446
F-HEMBA1002645//Human DNA sequence *** SEQUENCING IN PROGRESS *** from clone 153G14, WORKING DRAFT SEQUENCE.//5.6e-47:222:86//AL031118
F-HEMBA1002651//Homo sapiens PAC clone DJ0593H12 from 7p31, complete sequence.//3.8e-182:859:99//AC004839
F-HEMBA1002659//Z.mobilis alcohol dehydrogenase I (adhA) gene, complete cds.//0.97:144:66//M32100
F-HEMBA1002661//Homo sapiens PAC clone DJ0698G21 from 7p21-p22, complete sequence.//1.3e-116:774:84//AC004535
F-HEMBA1002666
F-HEMBA1002678//Human DNA sequence *** SEQUENCING IN PROGRESS *** from clone 1137F22, WORKING DRAFT SEQUENCE.//5.7e-156:750:98//AL034421
F-HEMBA1002679//nbxb0002cC12r CUGI Rice BAC Library Oryza sativa genomic clone nbxb0002F23r, genomic survey sequence.//4.3e-09:517:58//AQ051621
F-HEMBA1002688//Herpes simplex virus type 2 (strain HG52), complete genome.//8.3e-20:651:61//Z86099
F-HEMBA1002696//Mus musculus proteasome regulator PA28 beta subunit gene, complete cds.//7.6e-62:306:81//AF060195
F-HEMBA1002703//Homo sapiens mRNA for KIAA0455 protein, complete cds.//1.9e-10:327:62//AB007924
F-HEMBA1002712
F-HEMBA1002716//HS_3064_A1_C10_MF CIT Approved Human Genomic Sperm Library D Homo sapiens genomic clone Plate=3064 Col=19 Row=E, genomic survey sequence.//8.4e-97:491:96//AQ142980
F-HEMBA1002728//Homo sapiens chromosome 5, BAC clone 205e20 (LBNL H170), complete sequence.//6.1e-21:217:77//AC004782
F-HEMBA1002730//Human platelet glycoprotein IIIa (GPIIIa) gene, exon 1.//0.57:125:67//M57481
F-HEMBA1002742//RPCI11-39J10.TP RPCI-11 Homo sapiens genomic clone RPCI-11-39J10, genomic survey sequence.//1.1e-86:414:99//AQ029102
F-HEMBA1002746//Mus musculus chromosome 19, clone CIT282B21, complete sequence.//7.1e-70:303:82//AC003694
F-HEMBA1002748//Human DNA sequence *** SEQUENCING IN PROGRESS *** from clone 410I8, WORKING DRAFT SEQUENCE.//0.096:212:62//AL031732
F-HEMBA1002750//Homo sapiens chromosome 5, PAC clone 170m10 (LBNL H89), complete sequence.//6.7e-40:232:70//AC004622
F-HEMBA1002768//Homo sapiens mRNA for KIAA0554 protein, partial cds.//9.0e-177:834:98//AB011126
F-HEMBA1002770//cDNA encoding novel rat protein TIP120 which is formed of complex with TBP (TATA binding protein).//1.3e-140:840:88//E12829
F-HEMBA1002777//F.rubripes GSS sequence, clone 189C06dB12, genomic survey sequence.//1.1e-28:263:77//AL007965
F-HEMBA1002779//CIT-HSP-2333I1.TF CIT-HSP Homo sapiens genomic clone 2333I1, genomic survey sequence.//1.8e-32:180:98//AQ036891
F-HEMBA1002780//Homo sapiens PAC clone DJ0244J05 from 5q31, complete sequence.//7.0e-06:199:67//AC004592
F-HEMBA1002794//H.sapiens mRNA for protein kinase C mu.//0.00015:244:67//X75756
F-HEMBA1002801//Plasmodium falciparum MAL3P2, complete sequence.//0.0010:534:57//AL034558
F-HEMBA1002810//Homo sapiens formin binding protein 21 mRNA, complete cds.//1.1e-167:820:97//AF071185
F-HEMBA1002816//Homo sapiens clone NH0576N21, WORKING DRAFT SEQUENCE, 5 unordered pieces.//3.1e-113:254:90//AC005043
F-HEMBA1002818//Cricetulus griseus H411 precursor (H411) mRNA, complete cds.//1.2e-122:760:86//AF046870
F-HEMBA1002826//Human DNA sequence from clone 23K20 on chromosome Xq25-26.2 Contains EST, STS, GSS, complete sequence.//0.0055:235:65//AL022153
F-HEMBA1002833//Homo sapiens chromosome 17, clone hRPC.117_B_12, complete sequence.//1.4e-170:744:99//AC004707
F-HEMBA1002850//Ephedrus persicae NADH dehydrogenase 1 gene, mitochondrial gene encoding mitochondrial protein, partial cds.//1.3e-05:334:59//AF069186
F-HEMBA1002863//CIT-HSP-2323A16.TF CIT-HSP Homo sapiens genomic clone 2323A16, genomic survey sequence.//2.9e-140:750:93//AQ028419
F-HEMBA1002876//HS_2270_B1_H03_MF CIT Approved Human Genomic Sperm Library D Homo sapiens genomic clone Plate=2270 Col=5 Row=P, genomic survey sequence.//0.44:163:64//AQ164031
F-HEMBA1002886
F-HEMBA1002896//Homo sapiens chromosome 5, P1 clone 793C5 (LBNL H58), complete sequence..//0.00015:277:61//AC005195
F-HEMBA1002921
F-HEMBA1002924//CIT-HSP-2171H4.TR CIT-HSP Homo sapiens genomic clone 2171H4, genomic survey sequence.//0.0016:175:66//B89715
F-HEMBA1002934//Human DNA sequence *** SEQUENCING IN PROGRESS *** from clone 862K6, WORKING DRAFT SEQUENCE.//1.2e-169:797:98//AL031681
F-HEMBA1002935//Homo sapiens mRNA for KIAA0576 protein, partial cds.//4.9e-173:803:99//AB011148
F-HEMBA1002937//Human DNA sequence *** SEQUENCING IN PROGRESS *** from clone 702J19, WORKING DRAFT SEQUENCE.//1.2e-163:411:99//AL033531
F-HEMBA1002939//RPCI11-74O14.TJ RPCI11 Homo sapiens genomic clone R-74O14, genomic survey sequence.//1.7e-41:215:99//AQ266676
F-HEMBA1002944//RPCI11-55C2.TV RPCI11 Homo sapiens genomic clone R-55C2, genomic survey sequence.//1.7e-37:375:74//AQ082240
F-HEMBA1002951//Homo sapiens chromosome 19, cosmid F20887, complete sequence.//0.00074:683:58//AC005578
F-HEMBA1002954//RPCI11-79F7.TV RPCI11 Homo sapiens genomic clone R-79F7, genomic survey sequence.//6.1e-24:250:78//AQ284146
F-HEMBA1002968//HS_2262_B2_G04_T7 CIT Approved Human Genomic Sperm Library D Homo sapiens genomic clone Plate=2262 Col=8 Row=N, genomic survey sequence.//0.99:270:60//AQ217059
F-HEMBA1002970//RPCI11-5L24.TV RPCI-11 Homo sapiens genomic clone RPCI-11-5L24, genomic survey sequence.//1.4e-10:189:71//B49289
F-HEMBA1002971//CIT-HSP-2363L16.TF CIT-HSP Homo sapiens genomic clone 2363L16, genomic survey sequence.//4.3e-21:181:80//AQ080538
F-HEMBA1002973//Rattus norvegicus Wistar 3',5'-cyclic AMP phosphodiesterase (PDE4-10) gene, exon 10.//2.5e-40:257:89//U01290
F-HEMBA1002997//CIT-HSP-2387H15.TF.1 CIT-HSP Homo sapiens genomic clone 2387H15, genomic survey sequence.//9.5e-17:128:92//AQ240797
F-HEMBA1002999//Rattus norvegicus lamina associated polypeptide 1C (LAP1C) mRNA, complete cds.//3.1e-62:713:73//U20286
F-HEMBA1003021//Homo sapiens clone DJ0847008, WORKING DRAFT SEQUENCE, 3 unordered pieces.//7.5e-50:331:85//AC005484
F-HEMBA1003033//Drosophila melanogaster, chromosome 3L, region 62A10-62B5, P1 clones DS02777, DS03222, DS02345, and DS04808, complete sequence.//2.6e-20:357:66//AC005557
F-HEMBA1003034//Human DNA sequence from 4PTEL, Huntington's Disease Region, chromosome 4p16.3.//4.5e-60:415:73//Z95704
F-HEMBA1003035//Homo sapiens chromosome Y, clone 264,M,20, complete sequence.//2.3e-05:591:57//AC004617
F-HEMBA1003037//RPCI11-88F2.TJ RPCI11 Homo sapiens genomic clone R-88F2, genomic survey sequence.//0.68:230:60//AQ286677
F-HEMBA1003041//Homo sapiens PAC clone DJ1163J12 from 7q21.2-q31.1, complete sequence.//8.1e-128:550:94//AC004983
F-HEMBA1003046//Homo sapiens mitochondrial processing peptidase beta-subunit mRNA, complete cds.//1.0e-164:777:98//AF054182
F-HEMBA1003064//Plasmodium falciparum 3D7 chromosome 12 PFYAC181 genomic sequence, WORKING DRAFT SEQUENCE, 8 unordered pieces.//1.5e-07:744:59//AC005505
F-HEMBA1003067//Rat dynorphin gene, exon 3.//1.0:140:63//M32783
F-HEMBA1003071//Homo sapiens alpha2-C4-adrenergic receptor gene, complete cds.//1.5e-20:595:65//U72648
F-HEMBA1003077//CIT-HSP-2366J21.TF CIT-HSP Homo sapiens genomic clone 2366J21, genomic survey sequence.//4.4e-33:176:99//AQ080257
F-HEMBA1003078//Homo sapiens DNA sequence from PAC 262D12 on chromosome 1q23.3-24.3. Contains a Tenascin (Hexabrachion, Cytotactin, Neuronectin, Myotendinous antigen)-LIKE gene and a mitochondrial/chloroplast 30S ribosomal protein S14-LIKE gene preceeded by a CpG island. Contains ESTs, genomic marker D1S2691 and STSs.//9.4e-43:478:70//Z99297
F-HEMBA1003079//Homo sapiens Xp22-132-134 BAC GSHB-590J15 (Genome Systems Human BAC library) complete sequence.//0.96:57:85//AC004673
F-HEMBA1003083//Homo sapiens PAC clone DJ1182N03 from 7q11.23-q21.1, complete sequence.//8.0e-74:359:81//AC004548
F-HEMBA1003086//Homo sapiens chromosome 16 BAC clone CIT987SK-334D11 complete sequence.//3.6e-11:734:58//AF001550
F-HEMBA1003096//Sequence 4 from patent US 5440017.//5.7e-56:594:71//I13750
F-HEMBA1003098//Human DNA sequence from cosmid SRL11M20, chromosome region 11p13. Contains EST and STS.//1.9e-09:230:69//Z83308
F-HEMBA1003117//Mouse TIS11 primary response gene, complete cds.//0.00054:480:60//M58564
F-HEMBA1003129//HS_3139_B2_F05_T7 CIT Approved Human Genomic Sperm Library D Homo sapiens genomic clone Plate=3139 Col=10 Row=L, genomic survey sequence.//2.3e-100:510:97//AQ187635
F-HEMBA1003133//Mouse BAC CitbCJ7 219m7, genomic sequence, complete sequence.//1.3e-78:370:90//AC005259
F-HEMBA1003136
F-HEMBA1003142//Homo sapiens full-length insert cDNA clone ZC39B06.//6.9e-121:563:100//AF086197
F-HEMBA1003148//Homo sapiens mRNA for dachshund protein.//6.7e-183:850:99//AJ005670
F-HEMBA1003166//Homo sapiens Chromosome 16 BAC clone CIT987-SKA-345G4 ∼complete genomic sequence, complete sequence.//3.8e-27:229:76//AC002302
F-HEMBA1003175//Homo sapiens genomic DNA for centromeric end of MHC class I region on chromosome 6, WORKING DRAFT SEQUENCE.//9.4e-09:837:58//AB000882
F-HEMBA1003179//Homo sapiens DNA sequence from Fosmid 27C3 on chromosome 22q11.2-qter. Contains two possibly alternatively spliced unknown genes, one with homology to a worm protein. Contains ESTs, complete sequence.//5.4e-115:174:98//AL022325
F-HEMBA1003197//Arabidopsis thaliana chromosome II BAC F15K20 genomic sequence, complete sequence.//1.1e-05:473:59//AC005824
F-HEMBA1003199//Rattus norvegicus Sprague-Dawley thyroid hormone receptor alpha gene, exon 1.//1.6e-05:367:61//U09302
F-HEMBA1003202//Homo sapiens BAC clone RG437L15 from 8q21, complete sequence.//9.0e-23:247:73//AC004003
F-HEMBA1003204//Human DNA sequence *** SEQUENCING IN PROGRESS *** from clone 409J21, WORKING DRAFT SEQUENCE.//4.7e-26:141:83//Z83824
F-HEMBA1003212//Human Chromosome 11 Overlapping Cosmids cSRL72g7 and cSRL140b8, complete sequence.//1.9e-31:158:86//AC002037
F-HEMBA1003220//Homo sapiens chromosome 17, clone hRPC.971_F_3, WORKING DRAFT SEQUENCE, 1 ordered pieces.//3.4e-24:284:75//AC004150
F-HEMBA1003222//RPCI11-47P17.TJ RPCI11 Homo sapiens genomic clone R-47P17, genomic survey sequence.//8.7e-39:202:99//AQ202885
F-HEMBA1003229//Arabidopsis thaliana genomic DNA, chromosome 3, P1 clone: MEB5, complete sequence.//0.86:227:62//AB019230
F-HEMBA1003235//Plasmodium falciparum chromosome 2, section 10 of 73 of the complete sequence.//8.6e-05:372:61//AE001373
F-HEMBA1003250//HS-1063-A1-H02-MR.abi CIT Human Genomic Sperm Library C Homo sapiens genomic clone Plate=CT 796 Col=3 Row=O, genomic survey sequence.//0.00032:57:96//B46142
F-HEMBA1003257//H.sapiens mRNA for RDC-1 POU domain containing protein.//2.2e-08:531:59//X64624
F-HEMBA1003273//H.sapiens flow-sorted chromosome 6 HindIII·fragment, SC6pA19H4.//0.070:267:64//Z78949
F-HEMBA1003276//CIT-HSP-2301B4.TF CIT-HSP Homo sapiens genomic clone 2301B4, genomic survey sequence.//5.2e-08:295:63//AQ015073
F-HEMBA1003278//HS_3075_A1_G09_MF CIT Approved Human Genomic Sperm Library D Homo sapiens genomic clone Plate=3075 Col=17 Row=M, genomic survey sequence.//0.98:399:58//AQ120599
F-HEMBA1003281//High throughput sequencing of human chromosome 12, WORKING DRAFT SEQUENCE, 1 ordered pieces.//4.8e-101:277:97//AC005840
F-HEMBA1003286//Homo sapiens chromosome 3q13 beta-1,4-galactosyltransferase mRNA, complete cds.//9.0e-145:539:97//AF038662
F-HEMBA1003291//Homo sapiens mRNA for KIAA0537 protein, complete cds.//5.0e-166:799:98//AB011109
F-HEMBA1003296//CITBI-E1-2507M8.TR CITBI-E1 Homo sapiens genomic clone 2507M8, genomic survey sequence.//1.9e-05:388:63//AQ262551
F-HEMBA1003304//Budworm mitochondrial partial transfer RNA-Met (tRNA-Met) gene, and partial 12S ribosomal RNA (12S rRNA) gene.//8.0e-05:388:62//L17343
F-HEMBA1003309//Crassostrea gigas clone CN20 microsatellite sequence.//0.0017:210:64//AF051177
F-HEMBA1003314//Homo sapiens mRNA for leucine zipper bearing kinase, complete cds.//4.6e-188:865:99//AB001872
F-HEMBA1003322//Human DNA sequence *** SEQUENCING IN PROGRESS *** from clone 169I5, WORKING DRAFT SEQUENCE.//2.4e-54:316:87//Z93015
F-HEMBA1003327//CIT-HSP-2024C24.TRB CIT-HSP Homo sapiens genomic clone 2024C24, genomic survey sequence.//8.4e-12:166:76//B67147
F-HEMBA1003328//HS_2230_B2_H08_MR CIT Approved Human Genomic Sperm Library D Homo sapiens genomic clone Plate=2230 Col=16 Row=P, genomic survey sequence.//0.026:128:71//AQ153313
F-HEMBA1003330//Homo sapiens wbscr1 (WBSCR1) and replication factor C subunit 2 (RFC2) genes, complete cds.//4.0e-160:745:99//AF045555
F-HEMBA1003348//HS_3194_A1_G05_MR CIT Approved Human Genomic Sperm Library D Homo sapiens genomic clone Plate=3194 Col=9 Row=M, genomic survey sequence.//5.0e-79:381:99//AQ173779
F-HEMBA1003369//H.vulgare GAA-satellite DNA.//0.12:89:71//Z50100
F-HEMBA1003370//Homo sapiens cosmid 123E15, complete sequence.//3.5e-32:199:80//AF024533
F-HEMBA1003373//Human DNA sequence *** SEQUENCING IN PROGRESS *** from clone 537K23, WORKING DRAFT SEQUENCE.//0.019:117:71//AL034405
F-HEMBA1003376//Human clone HS4.66 Alu-Ya5 sequence.//4.2e-30:196:85//U67229
F-HEMBA1003380//Homo sapiens DNA sequence from clone 394P21 on chromosome 1p36.12-36.13. Contains the PAX7 gene, locus D1S2644, ESTs and STSs, complete sequence.//4.6e-22:206:81//AL021528
F-HEMBA1003384//Homo sapiens clone GS096J14, WORKING DRAFT SEQUENCE, 3 unordered pieces.//0.00094:72:90//AC006026
F-HEMBA1003395//Plasmodium falciparum DNA *** SEQUENCING IN PROGRESS *** from MAL1P1, WORKING DRAFT SEQUENCE.//0.00041:826:57//AL031744
F-HEMBA1003402//CIT-HSP-2339K16.TR CIT-HSP Homo sapiens genomic clone 2339K16, genomic survey sequence.//2.4e-05:265:64//AQ056234
F-HEMBA1003403//Homo sapiens chromosome 4 clone B353C18 map 4q25, complete sequence.//4.3e-135:780:90//AC004066
F-HEMBA1003408
F-HEMBA1003417//Human DNA sequence from clone 496N17 on chromosome 6p11.2-12.3 Contains EST, GSS, complete sequence.//1.9e-41:239:95//AL031321
F-HEMBA1003418//Rattus norvegicus Wistar polymeric immunoglobulin receptor (PIGR) gene, 3'UTR and trinucleotide repeat microsatellites.//2.2e-06:247:64//U08273
F-HEMBA1003433//Homo sapiens nibrin (NBS) mRNA, complete cds.//1.4e-149:697:99//AF051334
F-HEMBA1003447//Homo sapiens chromosome 4 clone B353C18 map 4q25, complete sequence.//1.7e-77:461:90//AC004066
F-HEMBA1003461//Rhodobacter sphaeroides FliH (fliH) gene, partial cds, F1iI (fliI) and FliJ (fliJ) genes, complete cds.//8.6e-08:752:58//U31090
F-HEMBA1003463//Homo sapiens chromosome 17, clone HCIT305D20, complete sequence.//0.089:172:68//AC004098
F-HEMBA1003480//Homo sapiens clone NH0523H20, complete sequence.//4.5e-150:562:97//AC005041
F-HEMBA1003528//Streptomyces fradiae gene for trypsinogen precursor, complete cds.//4.7e-09:433:60//D16687
F-HEMBA1003531//Homo sapiens PAC clone DJ1185I07 from 7q11.23-q21, complete sequence.//2.3e-48:297:90//AC004990
F-HEMBA1003538//Human complement C1r mRNA, complete cds.//4.3e-22:474:63//M14058
F-HEMBA1003545//Rattus norvegicus (clone 1.6kB) islet-2 mRNA, complete cds.//3.5e-143:805:91//L35571
F-HEMBA1003548
F-HEMBA1003555//Human DNA sequence *** SEQUENCING IN PROGRESS *** from clone 447E6, WORKING DRAFT SEQUENCE.//3.4e-58:331:83//AL031724
F-HEMBA1003556//Homo sapiens Xp22-175-176 BAC GSHB-484017 (Genome Systems Human BAC Library) complete sequence.//6.0e-99:703:84//AC005913
F-HEMBA1003560//Bovine GTP-binding regulatory protein gamma-6 subunit mRNA, complete cds.//1.3e-99:587:89//J05071
F-HEMBA1003568//HS_3149_A1_C04_MR CIT Approved Human Genomic Sperm Library D Homo sapiens genomic clone Plate=3149 Col=7 Row=E, genomic survey sequence.//4.1e-05:389:57//AQ166810
F-HEMBA1003569//Homo sapiens BAC clone NH0335J18 from 2, complete sequence.//1.6e-102:669:85//AC005539
F-HEMBA1003571//Dictyostelium discoideum RegA (regA) gene, complete cds.//0.00033:649:58//U60170
F-HEMBA1003579//Plasmodium falciparum DNA *** SEQUENCING IN PROGRESS *** from MAL1P1, WORKING DRAFT SEQUENCE.//0.00034:623:56//AL031744
F-HEMBA1003581//Mouse mRNA for talin.//3.3e-41:181:86//X56123
F-HEMBA1003591//Homo sapiens chromosome 16, BAC clone RPCI-11_192K18, complete sequence.//4.4e-70:273:94//AC006075
F-HEMBA1003595//Plasmodium falciparum chromosome 2, section 32 of 73 of the complete sequence.//6.0e-17:768:58//AE001395
F-HEMBA1003597//Plasmodium falciparum chromosome 2, section 35 of 73 of the complete sequence.//4.0e-09:777:56//AE001398
F-HEMBA1003598//Homo sapiens PAC clone DJ0537P09 from 7p11.2-p12, complete sequence.//1.3e-146:692:98//AC005153
F-HEMBA1003615//HS_2010_A2_A07_MR CIT Approved Human Genomic Sperm Library D Homo sapiens genomic clone Plate=2010 Col=14 Row=A, genomic survey sequence.//1.1e-22:137:97//AQ226592
F-HEMBA1003617//Homo sapiens HRIHFB2157 mRNA, partial cds.//2.4e-169:501:97//AB015344
F-HEMBA1003621//Mus musculus PIAS3 mRNA, complete cds.//4.7e-37:165:92//AF034080
F-HEMBA1003622//Plasmodium falciparum 3D7 chromosome 12 PFYAC336 genomic sequence, WORKING DRAFT SEQUENCE, 5 unordered pieces.//0.0024:514:58//AC005139
F-HEMBA1003630//CIT-HSP-2168N15.TR CIT-HSP Homo sapiens genomic clone 2168N15, genomic survey sequence.//6.5e-15:358:63//B92984
F-HEMBA1003637//Homo sapiens clone RG228D17, WORKING DRAFT SEQUENCE, 2 unordered pieces.//5.0e-21:238:76//AC005077
F-HEMBA1003640//Human DNA sequence *** SEQUENCING IN PROGRESS *** from clone 112K5, WORKING DRAFT SEQUENCE.//2.3e-15:371:63//Z85987
F-HEMBA1003645//A.thaliana 81kb genomic sequence.//1.0:529:57//X98130
F-HEMBA1003646
F-HEMBA1003656
F-HEMBA1003662//Homo sapiens chromosome 17, clone hRPK.332_H_18, complete sequence.//1.6e-175:824:98//AC005746
F-HEMBA1003667//Homo sapiens chromosome 12p13.3, WORKING DRAFT SEQUENCE, 21 unordered pieces.//1.1e-24:190:87//AC004765
F-HEMBA1003679//Homo sapiens BAC clone RG114B19 from 7q31.1, complete sequence.//1.7e-162:579:99//AC005065
F-HEMBA1003680//H.sapiens DNA sequence.//7.3e-22:172:87//Z22322
F-HEMBA1003684//H.sapiens mRNA for Miz-1 protein.//0.0054:146:70//Y09723
F-HEMBA1003690//Homo sapiens antigen NY-CO-9 (NY-CO-9) mRNA, partial cds.//2.9e-72:606:77//AF039691
F-HEMBA1003692
F-HEMBA1003711//Homo sapiens chromosome 17, clone HRPC41C23, complete sequence.//0.55:450:60//AC003101
F-HEMBA1003714
F-HEMBA1003715//Human DNA sequence from clone 931E15 on chromosome Xq25. Contains STSs, GSSs and genomic marker DXS8098, complete sequence.//3.0e-16:316:68//AL023575
F-HEMBA1003720//Homo sapiens chromosome 4 clone B227H22 map 4q25, complete sequence.//1.3e-41:483:73//AC004056
F-HEMBA1003725//CIT-HSP-2351H9.TF CIT-HSP Homo sapiens genomic clone 2351H9, genomic survey sequence./1.1e-112:532:99//AQ079348
F-HEMBA1003729//HS_3043_A1_E07_MR CIT Approved Human Genomic Sperm Library D Homo sapiens genomic clone Plate=3043 Col=13 Row=I, genomic survey sequence.//1.6e-12:87:98//AQ129345
F-HEMBA1003733//Homo sapiens, clone hRPK.15_A_1, complete sequence.//4.7e-104:761:82//AC006213
F-HEMBA1003742//HS_3027_A2_B02_MF CIT Approved Human Genomic Sperm Library D Homo sapiens genomic clone Plate=3027 Col=4 Row=C, genomic survey sequence.//3.4e-08:67:97//AQ154731
F-HEMBA1003758//CIT-HSP-2379D18.TR CIT-HSP Homo sapiens genomic clone 2379D18, genomic survey sequence.//2.9e-10:310:63//AQ113513
F-HEMBA1003760//Mus musculus hypoxia inducible factor three alpha mRNA, complete cds.//6.4e-114:714:86//AF060194
F-HEMBA1003773//Plasmodium falciparum 3D7 chromosome 12 PFYAC336 genomic sequence, WORKING DRAFT SEQUENCE, 5 unordered pieces.//0.078:378:58//AC005139
F-HEMBA1003783//Human DNA sequence from PAC 509L4 on chromosome 6q22.1-6q22.33. Contains SSX3 like pseudogene, EST, STS.//9.0e-135:804:89//Z99496
F-HEMBA1003784//Caenorhabditis elegans cosmid C55B6.//0.054:463:58//U88181
F-HEMBA1003799//Homo sapiens Chromosome 22q11.2 Cosmid Clone 105a In DGCR Region, complete sequence.//1.9e-44:425:76//AC000070
F-HEMBA1003803//Oryctolagus cuniculus troponin T cardiac isoform mRNA, 3' end of cds.//0.95:198:62//L40178
F-HEMBA1003804//Homo sapiens chromosome 17, clone hCIT.175_E_5, complete sequence.//1.2e-138:275:99//AC004596
F-HEMBA1003805//Mus musculus quaking type I (QKI) mRNA, complete cds.//6.6e-148:753:95//U44940
F-HEMBA1003807//HS-1068-B1-G06-MR.abi CIT Human Genomic Sperm Library C Homo sapiens genomic clone Plate=CT 278 Col=11 Row=N, genomic survey sequence.//6.7e-07:241:67//B47212
F-HEMBA1003827//Homo sapiens mRNA for KIAA0616 protein, partial cds.//1.0e-83:586:87//AB014516
F-HEMBA1003836//S.cerevisiae chromosome IX cosmid 9150.//5.1e-16:368:63//Z38125
F-HEMBA1003838//CIT-HSP-384J15.TR CIT-HSP Homo sapiens genomic clone 384J15, genomic survey sequence.//1.4e-45:180:90//B54810
F-HEMBA1003856//Homo sapiens chromosome 10 clone CIT9875K-1188B12 map 10p12.1, complete sequence.//0.0014:574:58//AC005875
F-HEMBA1003864//, complete sequence.//2.1e-91:234:95//AC005300
F-HEMBA1003866//Mus musculus semaphorin VIa mRNA, complete cds.//5.9e-81:853:71//AF030430
F-HEMBA1003879//H.sapiens CBP80 mRNA.//2.0e-08:87:95//X80030
F-HEMBA1003880//Homo sapiens genomic DNA, chromosome 21q11.1, segment 7/28, WORKING DRAFT SEQUENCE.//1.7e-180:853:98//AP000036
F-HEMBA1003885//Homo sapiens PAC clone DJ0167F23 from 7p15, complete sequence.//4.5e-39:376:67//AC004079
F-HEMBA1003893//H.sapiens CpG island DNA genomic Mse1 fragment, clone 11b6, forward read cpg11b6.ft1a.//3.6e-32:173:99//Z59012
F-HEMBA1003902//RPCI11-26M20.TPB RPCI-11 Homo sapiens genomic clone RPCI-11-26M20, genomic survey sequence.//8.2e-12:422:61//AQ003455
F-HEMBA1003908//Plasmodium falciparum chromosome 2, section 38 of 73 of the complete sequence.//0.0063:468:58//AE001401
F-HEMBA1003926//Human DNA sequence *** SEQUENCING IN PROGRESS *** from clone 310O13, WORKING DRAFT SEQUENCE.//3.6e-27:278:76//AL031658
F-HEMBA1003937//Homo sapiens chromosome 3 subtelomeric region.//1.4e-55:315:81//AF109718
F-HEMBA1003939//HS-1047-A1-G04-MF.abi CIT Human Genomic Sperm Library C Homo sapiens genomic clone Plate=CT 830 Col=7 Row=M, genomic survey sequence.//6.1e-09:413:63//B38195
F-HEMBA1003942//Plasmodium falciparum 3D7 chromosome 12 PFYACB8-420 genomic sequence, WORKING DRAFT SEQUENCE, 14 unordered pieces.//0.42:205:65//AC005140
F-HEMBA1003950//M.capricolum DNA for CONTIG MC072.//0.029:458:58//Z33058
F-HEMBA1003953//HS_2268_A1_B04_MF CIT Approved Human Genomic Sperm Library D Homo sapiens genomic clone Plate=2268 Col=7 Row=C, genomic survey sequence.//9.0e-07:239:64//AQ085098
F-HEMBA1003958//Homo sapiens PAC clone DJ0808G16 from 7q11.23-q21, complete sequence.//2.8e-57:424:74//AC004894
F-HEMBA1003959//RPCI11-78E8.TV RPCI11 Homo sapiens genomic clone R-78E8, genomic survey sequence.//4.3e-86:441:9611AQ285498
F-HEMBA1003976//HS_3146_A1_H09_T7 CIT Approved Human Genomic Sperm Library D Homo sapiens genomic clone Plate=3146 Col=17 Row=O, genomic survey sequence.//6.3e-10:129:80//AQ141146
F-HEMBA1003978
F-HEMBA1003985//Caenorhabditis elegans DNA *** SEQUENCING IN PROGRESS *** from clone Y105C5, WORKING DRAFT SEQUENCE.//1.0:258:60//Z98855
F-HEMBA1003987
F-HEMBA1003989//Streptomyces coelicolor cosmid 1A9.//0.40:238:61//AL034446
F-HEMBA1004000//Rattus norvegicus satellite sequence d0Mco2.//2.0e-07:116:70//U19354
F-HEMBA1004011//Plasmodium falciparum 3D7 chromosome 12 PFYAC588 genomic sequence, WORKING DRAFT SEQUENCE, 2 unordered pieces.//0.098:286:60//AC004710
F-HEMBA1004012//Homo sapiens chromosome 17, clone hRPK.63_A_1, complete sequence.//2.8e-185:896:97//AC005670
F-HEMBA1004015//Homo sapiens chromosome 17, clone hRPK.721_K_1, complete sequence.//6.3e-68:417:80//AC005411
F-HEMBA1004024//Homo sapiens Xp22-83 BAC GSHB-324M7 (Genome Systems Human BAC Library) complete sequence.//2.0e-47:418:77//AC005859
F-HEMBA1004038//Homo sapiens genomic DNA, chromosome 21q11.1, segment 23/28, WORKING DRAFT SEQUENCE.//1.6e-51:564:74//AP000052
F-HEMBA1004042//Plasmodium falciparum chromosome 2, section 35 of 73 of the complete sequence.//1.2e-05:636:55//AE001398
F-HEMBA1004045//Homo sapiens (subclone 1_g7 from BAC H76) DNA sequence, complete sequence.//1.9e-31:373:76//AC002252
F-HEMBA1004048//Homo sapiens DNA for P35-related protein, exon 2.//0.039:234:63//D63393
F-HEMBA1004049//Homo sapiens Xp22 GS-524I1 (Genome Systems Human BAC library), complete sequence.//4.8e-135:780:89//AC003106
F-HEMBA1004055//Human chromosome 3p21.1 gene sequence.//4.7e-09:457:58//L13435
F-HEMBA1004056//Human DNA sequence *** SEQUENCING IN PROGRESS *** from clone 447C4, WORKING DRAFT SEQUENCE.//3.3e-25:246 :77//AL021977
F-HEMBA1004074//CIT-HSP-2053J5.TF CIT-HSP Homo sapiens genomic clone 2053J5, genomic survey sequence.//7.8e-24:233:76//B68555
F-HEMBA1004086//Saccharomyces douglasii mitochondrial tRNA-Ser and tRNA-Phe genes, partial sequence, and Var1p (var1) gene, mitochondrial gene encoding mitochondrial protein, complete cds.//4.5e-08:614:59//U49822
F-HEMBA1004097//Mus musculus putative transcription factor mRNA, complete cds.//5.9e-121:502:85//AF091234
F-HEMBA1004111//*** SEQUENCING IN PROGRESS *** Homo sapiens chromosome 4, BAC clone C0481P14; HTGS phase 1, WORKING DRAFT SEQUENCE, 7 unordered pieces.//2.0e-36:317:80//AC006160
F-HEMBA1004131//Mus musculus clone OST2067, genomic survey sequence.//8.7e-24:320:71//AF046393
F-HEMBA1004132//HS_3226_B1_D10_T7 CIT Approved Human Genomic Sperm Library D Homo sapiens genomic clone Plate=3226 Col=19 Row=H, genomic survey sequence.//9.7e-13:232:71//AQ182017
F-HEMBA1004133
F-HEMBA1004138//HS_3036_B1_G11_MR CIT Approved Human Genomic Sperm Library D Homo sapiens genomic clone Plate=3036 Col=21 Row=N, genomic survey sequence.//0.0035:165:64//AQ294763
F-HEMBA1004143
F-HEMBA1004146
F-HEMBA1004150//Human DNA sequence from PAC 52D1 on chromosome Xq21. Contains CA repeats, STS.//0.00011:618:60//Z96811
F-HEMBA1004164//Homo sapiens Xp22-175-176 BAC GSHB-484O17 (Genome Systems Human BAC Library) complete sequence.//2.9e-30:454:68//AC005913
F-HEMBA1004168//Homo sapiens geminin mRNA, complete cds.//4.5e-133:649:97//AF067855
F-HEMBA1004199
F-HEMBA1004200//HS_2015_A1_B05_T7 CIT Approved Human Genomic Sperm Library D Homo sapiens genomic clone Plate=2015 Col=9 Row=C, genomic survey sequence.//8.5e-34:236:87//AQ247957
F-HEMBA1004202//Mus musculus chromosome 11, clone mCIT.268_P_23, complete sequence.//7.8e-59:216:83//AC004807
F-HEMBA1004203//Homo sapiens clone NH0313P13, WORKING DRAFT SEQUENCE, 15 unordered pieces.//6.3e-98:173:98//AC005488
F-HEMBA1004207//Homo sapiens leptin receptor short form (db) mRNA, complete cds.//3.2e-166:791:98//U50748
F-HEMBA1004225//Plasmodium falciparum chromosome 2, section 61 of 73 of the complete sequence.//6.5e-08:584:60//AE001424
F-HEMBA1004227//Rattus norvegicus protein phosphatase 2C mRNA, complete cds.//8.0e-115:713:86//AF095927
F-HEMBA1004238
F-HEMBA1004241//CIC5B11.1 check: 4870 from: 1 to: 167234, complete sequence.//0.57:552:58//AC004708
F-HEMBA1004246//Human DNA sequence from clone 422F24 on chromosome 6q24.1-25.2. Contains a novel gene similar to C. elegans C02C2.5. Contains ESTs, STSs and GSSs, complete sequence.//6.1e-21:254:77//AL031010
F-HEMBA1004248//Rattus rattus insulin-induced growth-respons protein (CL-6) mRNA, complete cds.//1.7e-30:315 :74//L13619
F-HEMBA1004264//Homo sapiens cosmid clone LUCA20 from 3p21.3, complete sequence.//4.4e-07:674:60//AC004693
F-HEMBA1004267//Homo sapiens chromosome 17, clone hRPC.117_B_12, complete sequence.//3.1e-78:335:87//AC004707
F-HEMBA1004272//Homo sapiens 12p13.3 PAC RPCI5-1180D12 (Roswell Park Cancer Institute Human PAC Library) complete sequence.//1.4e-176:856:97//AC005831
F-HEMBA1004274//HS_3064_B2_A04_MF CIT Approved Human Genomic Sperm Library D Homo sapiens genomic clone Plate=3064 Col=8 Row=B, genomic survey sequence.//3.1e-28:153:100//AQ136993
F-HEMBA1004275//Human DNA sequence *** SEQUENCING IN PROGRESS *** from clone 501A4, WORKING DRAFT SEQUENCE.//5.2e-17:109:99//Z98051
F-HEMBA1004276//CIT-HSP-2387K6.TF.1 CIT-HSP Homo sapiens genomic clone 2387K6, genomic survey sequence.//5.0e-07:63:98//AQ240477
F-HEMBA1004286//Homo sapiens TGF beta receptor associated protein-1 mRNA, complete cds.//2.1e-185:868:99//AF022795
F-HEMBA1004289//Arabidopsis thaliana genomic DNA, chromosome 5, P1 clone: MQN23, complete sequence.//1.0:387:59//AB013395
F-HEMBA1004295//Homo sapiens DNA, anonymous heat-stable fragment RP11-3A.//7.8e-06:92:89//AB012254
F-HEMBA1004306//Homo sapiens clone DJ0811N16, complete sequence.//0.00037:413:59//AC004897
F-HEMBA1004312//Rickettsia prowazekii strain Madrid E, complete genome; segment 2/4.//0.28:522:57//AJ235271
F-HEMBA1004321//Homo sapiens chromosome 19, BAC CIT-B-191n6, complete sequence.//7.1e-136:548:92//AC006130
F-HEMBA1004323//Human DNA sequence from PAC 450C20 on chromosome X.//1.3e-32:320:65//Z84720
F-HEMBA1004327//Homo sapiens mRNA for KIAA0522 protein, partial cds.//0.93:222:62//AB011094
F-HEMBA1004330//Homo sapiens clone DJ1196H06, WORKING DRAFT SEQUENCE, 4 unordered pieces.//7.0e-168:895:93//AC004995
F-HEMBA1004334//Homo sapiens Xp22 BAC 620F15 (Genome Systems BAC library) complete sequence.//4.6e-73:713:75//AC002980
F-HEMBA1004335//Human DNA-sequence *** SEQUENCING IN PROGRESS *** from clone 417M14, WORKING DRAFT SEQUENCE.//1.3e-25:121:85//AL024498
F-HEMBA1004341
F-HEMBA1004353//***ALU WARNING: Human Alu-Sc subfamily consensus sequence.//6.4e-38:278:85//U14571
F-HEMBA1004354//Human clone C3 CHL1 protein (CHLR1) mRNA, alternatively spliced, complete cds.//4.1e-45:190:92//U75968
F-HEMBA1004356
F-HEMBA1004366//P.falciparum complete gene map of plastid-like DNA (IR-A).//2.2e-07:736:57//X95275
F-HEMBA1004372//H.sapiens dystrophin gene intron 44.//1.0:129:62//X77644
F-HEMBA1004389//Mouse interleukin 2 receptor (p55 IL-2R) mRNA, 5' end.//4.7e-42:237:94//M21977
F-HEMBA1004394//Plasmodium falciparum chromosome 2, section 39 of 73 of the complete sequence.//5.2e-05:519:59//AE001402
F-HEMBA1004396//Human BAC clone RG302F04 from 7q31, complete sequence.//4.0e-32:261:76//AC002463
F-HEMBA1004405//Plasmodium falciparum 3D7 chromosome 12 PFYACB8-628 genomic sequence, WORKING DRAFT SEQUENCE, 9 unordered pieces.//1.4e-07:693 :58//AC005507
F-HEMBA1004408//Homo sapiens clone NH0469M07, WORKING DRAFT SEQUENCE, 7 unordered pieces.//1.2e-69:195:100//AC005037
F-HEMBA1004429//HS_3193_A1_B06_T7 CIT Approved Human Genomic Sperm Library D Homo sapiens genomic clone Plate=3193 Col=11 Row=C, genomic survey sequence.//5.1e-67:386:91//AQ172942
F-HEMBA1004433//Human Chromosome 11p11.2 PAC clone pDJ404m15, complete sequence.//3.2e-27:242:82//AC002554
F-HEMBA1004460//Homo sapiens clone DJ0647C14, WORKING DRAFT SEQUENCE, 21 unordered pieces.//1.7e-75:590:81//AC004846
F-HEMBA1004461//Human DNA sequence from clone 657J8 on chromosome Xq26.1-26.3 Contains GSS, complete sequence.//0.045:215:66//AL034407
F-HEMBA1004479//Mus musculus hypoxia inducible factor three alpha mRNA, complete cds.//5.2e-43:364:79//AF060194
F-HEMBA1004482//Plasmodium falciparum 3D7 chromosome 12 PFYAC181 genomic sequence, WORKING DRAFT SEQUENCE, 8 unordered pieces.//6.8e-17:791:59//AC005505
F-HEMBA1004499//Homo sapiens chromosome 17, clone hRPC.1073_F_15, complete sequence.//4.4e-125:251:94//AC004686
F-HEMBA1004502//Plasmodium falciparum 3D7 chromosome 12 PFYAC1122 genomic sequence, WORKING DRAFT SEQUENCE, 3 unordered pieces.//0.012:635:57//AC004709
F-HEMBA1004506//Homo sapiens PAC clone DJ0844F09 from 7p12-p13, complete sequence.//2.8e-127:766:88//AC004453
F-HEMBA1004507
F-HEMBA1004509//Arabidopsis thaliana DNA chromosome 4, BAC clone T10I14 (ESSAII project).//1.0e-13:244:67//AL021712
F-HEMBA1004534//Human mRNA for actin-binding protein (filamin) (ABP-280).//1.6e-72:678:74//X53416
F-HEMBA1004538//Sequence 1 from patent US 5612190.//0.00015:416:59//I36871
F-HEMBA1004542//Homo sapiens clone NH0486I22, WORKING DRAFT SEQUENCE, 5 unordered pieces.//0.95:202:64//AC005038
F-HEMBA1004554//Arabidopsis thaliana BAC T26D22.//0.45:624:56//AFO58826
F-HEMBA1004560//Human mRNA for KIAA0281 gene, complete cds.//9.1e-10:173:70//D87457
F-HEMBA1004573//Human BAC clone RG114A06 from 7q31, complete sequence.//6.1e-23:134:73//AC002542
F-HEMBA1004577//Homo sapiens Chromosome 16 BAC clone CIT987SK-582J2, complete sequence.//1.6e-15:190:77//AC004525
F-HEMBA1004586//Homo sapiens clone DJ0810E06, WORKING DRAFT SEQUENCE, 8 unordered pieces.//3.1e-31:388:76//AC004895
F-HEMBA1004596//RPCI11-81O21.TJ RPCI11 Homo sapiens genomic clone R-81O21, genomic survey sequence.//2.2e-90:458:90//AQ285136
F-HEMBA1004604//Mus musculus COP9 complex subunit 7a (COPS7a) mRNA, complete cds.//8.6e-105:699:84//AF071316
F-HEMBA1004610//Homo sapiens PAC clone DJ1163J12 from 7q21.2-q31.1, complete sequence.//5.4e-20:267:72//AC004983
F-HEMBA1004617//CIT-HSP-2319H15.TF CIT-HSP Homo sapiens genomic clone 2319H15, genomic survey sequence.//6.2e-26:147:99//AQ034944
F-HEMBA1004629//Plasmodium falciparum 3D7 chromosome 12 PFYAC1383 genomic sequence, WORKING DRAFT SEQUENCE, 3 unordered pieces.//5.6e-06:766:56//AC005504
F-HEMBA1004631//Human DNA sequence from PAC 368A4 on chromosome X. Contains ESTs, CELLULAR NUCLEIC ACID BINDING PROTEIN (CNBP) like gene and STSs.//4.7e-73:412:92//Z83843
F-HEMBA1004632//Canine herpesvirus DNA for gene homolog of HSV1 UL16, EHV1 ORF 46, VZV ORF 44.//0.92:181:61//X90418
F-HEMBA1004637//G.gallus mRNA for LRP/alpha-2-macroglobulin receptor.//7.8e-47:784:65//X74904
F-HEMBA1004638//Rattus norvegicus homeodomain protein Nkx6.1 (nkx6.1) mRNA, complete cds.//6.4e-06:458:61//AF004431
F-HEMBA1004666//Caenorhabditis elegans DNA *** SEQUENCING IN PROGRESS *** from clone Y47D3, WORKING DRAFT SEQUENCE.//0.30:733:55//Z98865
F-HEMBA1004669//Human DNA sequence from clone 465N24 on chromosome 1p35.1-36.13. Contains two novel genes, ESTs, GSSs and CpG islands, complete sequence.//7.5e-136:521:98//AL031432
F-HEMBA1004670//Homo sapiens Chromosome 22q12 Cosmid Clone p90g5, complete sequence.//0.43:365 :59//AC000045
F-HEMBA1004672
F-HEMBA1004693//Plasmodium falciparum 3D7 chromosome 12 PFYAC492 genomic sequence, WORKING DRAFT SEQUENCE, 5 unordered pieces.//0.096:651:54//AC005308
F-HEMBA1004697//CIT-HSP-2326C13.TR CIT-HSP Homo sapiens genomic clone 2326C13, genomic survey sequence.//0.23:238:65//AQ040642
F-HEMBA1004705//Homo sapiens Xp22 Cosmid U151G1 (from Lawrence Livermore X library) and PAC RPCI1-93D11 (from Roswell Park Cancer Center) complete sequence.//2.1e-27:375:72//AC002357
F-HEMBA1004709//Homo sapiens Xp22-150 BAC GSHB-309P15 (Genome Systems Human BAC Library) complete sequence.//1.6e-36:191:91//AC006210
F-HEMBA1004711//Homo sapiens chromosome 17, clone hRPK.271_K_11, complete sequence.//1.1e-133:639:99//AC005562
F-HEMBA1004725//RPCI11-75013.TJ RPCI11 Homo sapiens genomic clone R-75O13, genomic survey sequence.//6.2e-32:169:100//AQ266512
F-HEMBA1004730//Human BAC clone RG035E18 from 7q31, complete sequence.//8.0e-68:732:72//AC004029
F-HEMBA1004733//CIT-HSP-2305M23.TF CIT-HSP Homo sapiens genomic clone 2305M23, genomic survey sequence.//4.9e-18:209:69//AQ017556
F-HEMBA1004734//Arabidopsis thaliana ubiquitin-conjugating enzyme 17 (UBC17) mRNA, complete cds.//1.8e-13:451:62//AF028340
F-HEMBA1004736//Human DNA Sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.//5.0e-87:646:78//Z94056
F-HEMBA1004748//Human BAC clone RG204I16 from 7q31, complete sequence.//0.24:526:57//AC002461
F-HEMBA1004751//Homo sapiens clone DJ0876A24, WORKING DRAFT SEQUENCE, 6 unordered pieces.//1.4e-25:268:76//AC004913
F-HEMBA1004752//R.norvegicus mRNA for leucocyte common antigen-related protein (3941 bp).//1.1e-07:503:61//X83546
F-HEMBA1004753//Homo sapiens Chromosome 12 Cosmid Clone 6e5, complete sequence.//4.5e-38:314:81//AC000028
F-HEMBA1004756//Homo sapiens, complete sequence.//1.4e-111:326:84//AC005854
F-HEMBA1004758//Sequence 29 from patent US 5534410.//3.9e-135:769:91//I23472
F-HEMBA1004763//Homo sapiens apoptosis inhibitor survivin gene, complete cds.//3.6e-47:404:79//U75285
F-HEMBA1004768//Homo sapiens PAC clone DJ0979P20 from 7q33-q35, complete sequence.//6.7e-107:890:78//AC004941
F-HEMBA1004770//Plasmodium falciparum 3D7 chromosome 12 PFYAC1122 genomic sequence, WORKING DRAFT SEQUENCE, 3 unordered pieces.//7.9e-09:806:59//AC004709
F-HEMBA1004771//G.muris ribosomal RNA operon DNA encoding 16S, 23S and 5.8S ribosomal RNA.//0.69:239:61//X65063
F-HEMBA1004776
F-HEMBA1004778
F-HEMBA1004795//Drosophila melanogaster A-kinase anchor protein DAKAP550 mRNA, partial cds.//3.4e-46:778:64//AF003622
F-HEMBA1004803//Homo sapiens chromosome Y, clone 264,M,20, complete sequence.//4.3e-82:580:82//AC004617
F-HEMBA1004806//Homo sapiens BAC clone RG281G05 from 7p15-p21, complete sequence.//5.4e-07:642:59//AC005083
F-HEMBA1004807//Human HIV1 tata element modulatory factor mRNA sequence from chromosome 3.//1.4e-46:171:92//L01042
F-HEMBA1004816//Homo sapiens calpastatin (CAST) gene, exons 10-14.//3.5e-31:546:66//M86257
F-HEMBA1004820//C.botulinum progenitor toxin complex genes.//0.0014:343:62//X87972
F-HEMBA1004847//Canine mRNA for 68kDA subunit of signal recognition particle (SRP68).//1.5e-85:512:88//X53744
F-HEMBA1004850//Homo sapiens TGF-beta type I receptor (TGFBR1) gene, exon 1.//0.0065:284:61//AF054590
F-HEMBA1004863//Genomic sequence from Mouse 11, complete sequence.//0.92:250:59//AC000400
F-HEMBA1004864
F-HEMBA1004865//Human DNA sequence from clone 459L4 on chromosome 6p22.3-24.1 Contains EST, STS, GSS, complete sequence.//3.6e-12:214:72//AL031120
F-HEMBA1004880//Homo sapiens Chromosome 16 BAC clone CIT987SK-A-319E8, complete sequence.//1.1e-08:255:69//AC004020
F-HEMBA1004889//Schistocerca americana Antennapedia homeotic protein (Antp) mRNA, complete cds.//0.062:155:69//U32943
F-HEMBA1004900//Plasmodium falciparum unidentified mRNA sequence.//0.00055:323:60//L12043
F-HEMBA1004909//Homo sapiens chromosome 17, clone 289A8, complete sequence.//9.6e-16:166:80//AC003051
F-HEMBA1004918//Turritella communis mitochondrial 16S ribosomal RNA gene, partial.//0.81:146:65//M94003
F-HEMBA1004923//Human DNA from overlapping chromosome 19-specific cosmids R32543,, and F15613 containing ZNF gene family member, genomic sequence, complete sequence.//1.4e-36:338:78//AC003006
F-HEMBA1004929//CIT-HSP-2373I16.TR CIT-HSP Homo sapiens genomic clone 2373I16, genomic survey sequence.//2.4e-86:443:96//AQ108676
F-HEMBA1004930//Homo sapiens PAC clone DJ0608H12 from 7q21, complete sequence.//4.6e-20:219:73//AC004109
F-HEMBA1004933//HS-1003-A1-E10-MF.abi CIT Human Genomic Sperm Library C Homo sapiens genomic clone Plate=CT 497 Col=19 Row=I, genomic survey sequence.//1.4e-28:216:85//B30726
F-HEMBA1004934//Homo sapiens chromosome 21q22.3 PAC 267O10, complete sequence.//0.53:222:61//AF042091
F-HEMBA1004944//Homo sapiens clone DJ0736H05, WORKING DRAFT SEQUENCE, 5 unordered pieces.//1.2e-58:509:78//AC005482
F-HEMBA1004954//HS_2033_A2_A08_T7 CIT Approved Human Genomic Sperm Library D Homo sapiens genomic clone Plate=2033 Col=16 Row=A, genomic survey sequence.//3.7e-47:243:99//AQ229758
F-HEMBA1004956//P.falciparum complete gene map of plastid-like DNA (IR-B).//0.048:421:58//X95276
F-HEMBA1004960//Arabidopsis thaliana DNA chromosome 4, ESSA I contig fragment No. 8.//0.89:333:58//Z97343
F-HEMBA1004972
F-HEMBA1004973//RPCI11-66P8.TK RPCI11 Homo sapiens genomic clone R-66P8, genomic survey sequence.//3.5e-22:245:77//AQ238471
F-HEMBA1004977//Homo sapiens full-length insert cDNA clone YZ83B08.//9.0e-11:84:98//AF086080
F-HEMBA1004978//CIT-HSP-2354E10.TR CIT-HSP Homo sapiens genomic clone 2354E10, genomic survey sequence.//0.0021:152:66//AQ075713
F-HEMBA1004980//HS_3018_A2_E04_MR CIT Approved Human Genomic Sperm Library D Homo sapiens genomic clone Plate=3018 Col=8 Row=I, genomic survey sequence.//1.9e-77:392:97//AQ071873
F-HEMBA1004983//Albinaria corrugata isolate cor. Prn1.1 16S ribosomal RNA gene, mitochondrial gene for mitochondrial RNA, partial sequence.//0.0030:276:61//AF031680
F-HEMBA1004995//Homo sapiens chromosome 16, cosmid bridge clone 306E6 (LANL), complete sequence.//4.2e-138:640:99//AC005590
F-HEMBA1005008//Human mariner1 transposase gene, complete consensus sequence.//6.8e-20:160:88//U52077
F-HEMBA1005009//Homo sapiens BAF53a (BAF53a) mRNA, complete cds.//2.0e-144:668:99//AF041474
F-HEMBA1005019//Homo sapiens mRNA for KIAA0648 protein, partial cds.//1.4e-146:693:98//AB014548
F-HEMBA1005029//Homo sapiens DNA sequence from PAC 97D16 on chromosome 6p21.3-22.2. Contains an unknown pseudogene, a 60S Ribosomal protein L24 (L30) LIKE pseudogene and histone genes H2BFC (H2B/c), H4FFP (H4/f pseudogene), H2AFC (H2A/c), H3F1K (H3.1/k) and a tRNA-Val pseudogene and tRNA-Thr gene. Contains ESTs, STSs, GSSs and genomic marker D6S464, complete sequence.//2.2e-115:668:90//AL009179
F-HEMBA1005035//Homo sapiens chromosome 17, clone hCIT.175_E_5, complete sequence.//4.6e-138:591:98//AC004596
F-HEMBA1005039//CIT-HSP-2338L5.TR CIT-HSP Homo sapiens genomic clone 2338L5, genomic survey sequence.//3.7e-61:271:88//AQ055486
F-HEMBA1005047//Mus musculus mRNA for Rab24 protein.//3.8e-17:218:73//Z22819
F-HEMBA1005050//Human Tis11d gene, complete cds.//0.079:251:63//U07802
F-HEMBA1005062//Plasmodium falciparum 3D7 chromosome 12 PFYAC69 genomic sequence, WORKING DRAFT SEQUENCE, 4 unordered pieces.//0.018:560:56//AC004688
F-HEMBA1005066//Human DNA sequence *** SEQUENCING IN PROGRESS *** from clone 774G10, WORKING DRAFT SEQUENCE.//3.4e-97:432:84//AL034410
F-HEMBA1005075//H.sapiens DNA 3' flanking simple sequence region clone wg2c3.//6.9e-07:176:68//X76589
F-HEMBA1005079//CIT-HSP-2325M21.TRB CIT-HSP Homo sapiens genomic clone 2325M21, genomic survey sequence.//2.1e-48:274:93//AQ038720
F-HEMBA1005083//HS_2248_B1_D05_MR CIT Approved Human Genomic Sperm Library D Homo sapiens genomic clone Plate=2248 Col=9 Row=H, genomic survey sequence.//3.4e-06:230:64//AQ129575
F-HEMBA1005101//Homo sapiens SYT interacting protein SIP mRNA, complete cds.//1.3e-161:762:98//AF080561
F-HEMBA1005113//L.esculentum microsatellite repeat DNA region.//0.0038:742:57//X90770
F-HEMBA1005123//Homo sapiens clone DJ0673M15, WORKING DRAFT SEQUENCE, 33 unordered pieces.//9.6e-83:479:78//AC004854
F-HEMBA1005133//Human DNA sequence *** SEQUENCING IN PROGRESS *** from clone Y313F4, WORKING DRAFT SEQUENCE.//3.9e-24:576:64//AL023808
F-HEMBA1005149//Homo sapiens PAC clone DJ430N08 from 22q12.1-qter, complete sequence.//4.7e-36:283:80//AC004542
F-HEMBA1005152//Homo sapiens chromosome Xp22-67-68, WORKING DRAFT SEQUENCE, 99 unordered pieces.//5.0e-10:332:64//AC004469
F-HEMBA1005159//Homo sapiens genomic DNA, chromosome 21q11.1, segment 1/5, WORKING DRAFT SEQUENCE.//4.0e-10:734:58//AP000023
F-HEMBA1005185//H.sapiens CpG island DNA genomic Mse1 fragment, clone 91b2, forward read cpg91b2.ft1a./12.2e-14:93:100//Z63847
F-HEMBA1005201//Drosophila melanogaster cosmid 152A3.//4.7e-35:679:64//AL009194
F-HEMBA1005202//Canine mRNA for 68kDA subunit of signal recognition particle (SRP68).//6.7e-138:778:90//X53744
F-HEMBA1005206//Drosophila melanogaster Su(P) and anon-73B1 genes and partial o25 gene and Pros26 gene.//7.1e-12:376:62//AJ011320
F-HEMBA1005219//Homo sapiens mRNA for KIAA0445 protein, complete cds.//7.1e-05:411:60//AB007914
F-HEMBA1005223//Homo sapiens PAC clone DJ430N08 from 22q12.1-qter, complete sequence.//3.5e-06:212:66//AC004542
F-HEMBA1005232//Plasmodium falciparum 3D7 chromosome 12 PFYAC492 genomic sequence, WORKING DRAFT SEQUENCE, 5 unordered pieces.//3.7e-07:625:57//AC005308
F-HEMBA1005241//Homo sapiens PAC clone DJ0777O23 from 7p14-p15, complete sequence.//8.7e-45:567:72//AC005154
F-HEMBA1005244//Homo sapiens chromosome X clone U177G4, U152H5, U168D5, 174A6, U172D6, and U186B3 from Xp22, complete sequence.//0.96:298:62//AC002365
F-HEMBA1005251
F-HEMBA1005252//Homo sapiens chromosome 17, clone hRPK.318_A_15, complete sequence.//4.5e-160:392:99//AC005837
F-HEMBA1005274//Homo sapiens BAC clone 255A7 from 8q21 containing NBS1 gene, complete sequence.//2.3e-05:496:60//AF069291
F-HEMBA1005275//Human DNA sequence from clone 444C7 on chromosome 6p22.3-23. Contains an EST, an STS and GSSs, complete sequence.//5.7e-05:220:64//AL033521
F-HEMBA1005293//Homo sapiens echinoderm microtubule-associated protein homolog HuEMAP mRNA, complete cds.//2.4e-20:338:65//U97018
F-HEMBA1005296
F-HEMBA1005304//Human DNA sequence from clone 364I22 on chromosome Xq21.31-22.3. Contains an STS and GSSs, complete sequence.//1.6e-51:381:78//AL031012
F-HEMBA1005311
F-HEMBA1005314//Homo sapiens genomic DNA, chromosome 21q11.1, segment 2/28, WORKING DRAFT SEQUENCE.//0.94:226:63//AP000031
F-HEMBA1005315//Homo sapiens BAC810, complete sequence.//9.5e-15:684:62//U85198
F-HEMBA1005318//Human DNA sequence from PAC 394F12 on chromosome X contains EST, STS, CpG island clone.//2.6e-05:472:59//Z83823
F-HEMBA1005331//Homo sapiens chromosome 17, clone hRPK.214_C_8, complete sequence.//3.3e-90:300:90//AC005803
F-HEMBA1005338//Homo sapiens mRNA for matrilin-4, partial.//1.4e-151:740:97//AJ007581
F-HEMBA1005353//CIT-HSP-2310N10.TR CIT-HSP Homo sapiens genomic clone 2310N10, genomic survey sequence.//2.1e-86:438:97//AQ016145
F-HEMBA1005359//Human zinc finger protein ZNF137 mRNA, complete cds.//1.8e-98:500:88//U09414
F-HEMBA1005367//Mus musculus melastatin mRNA, complete cds.//8.3e-72:577:73//AF047714
F-HEMBA1005372//Human DNA sequence from PAC 293E14 contains ESTs, STS.//1.3e-07:274:66//Z82900
F-HEMBA1005374//Homo sapiens clone 277F10, WORKING DRAFT SEQUENCE, 5 unordered pieces.//1.9e-48:611:69//AC004813
F-HEMBA1005382//HS_3063_B2_F11_MF CIT Approved Human Genomic Sperm Library D Homo sapiens genomic clone Plate=3063 Col=22 Row=L, genomic survey sequence.//1.6e-27:154:98//AQ103204
F-HEMBA1005389//Plasmodium falciparum telomere nucleotide sequence.//4.0e-07:443:61//M23175
F-HEMBA1005394//CIT-HSP-2368B11.TR CIT-HSP Homo sapiens genomic clone 2368B11, genomic survey sequence.//7.6e-17:225:71//AQ076749
F-HEMBA1005403//Human DNA sequence *** SEQUENCING IN PROGRESS *** from clone 423B22, WORKING DRAFT SEQUENCE.//4.5e-131:278:98//AL034379
F-HEMBA1005408//HS_3007_B2_G04_MR CIT Approved Human Genomic Sperm Library D Homo sapiens genomic clone Plate=3007 Col=8 Row=N, genomic survey sequence.//8.0e-06:218:66//AQ294366
F-HEMBA1005410//Human DNA sequence from cosmid cU120E2, on chromosome X contains Lowe oculocerebrorenal syndrome (OCRL) ESTs and STS.//1.5e-41:432:76//Z73496
F-HEMBA1005411
F-HEMBA1005423//Homo sapiens cyclin-dependent kinase inhibitor (CDKN2C) mRNA, complete cds.//1.0e-169:537:99//AF041248
F-HEMBA1005426
F-HEMBA1005443//Homo sapiens chromosome 19, BAC CIT-B-191n6, complete sequence.//7.1e-37:260:76//AC006130
F-HEMBA1005447//CIT-HSP-2173N7.TR CIT-HSP Homo sapiens genomic clone 2173N7, genomic survey sequence.//5.0e-133:631:98//B93234
F-HEMBA1005468//Human DNA sequence from clone 20J23 on chromosome Xq26.2-27.2 Contains ras-related C3 botulinum toxin substrate 1 (P21-RAC1) (ras-like protein TC25) EST, CA repeat, STS, CpG island, complete sequence.//1.5e-118:868:83//AL022576
F-HEMBA1005469//Homo sapiens chromosome 16, P1 clone 96-4B (LANL), complete sequence.//1.2e-179:838:99//AC005212
F-HEMBA1005472//Human DNA sequence *** SEQUENCING IN PROGRESS *** from clone 228H13, WORKING DRAFT SEQUENCE.//3.4e-20:187:74//AL031985
F-HEMBA1005474//Homo sapiens genomic DNA, chromosome 21q11.1, segment 12/28, WORKING DRAFT SEQUENCE.//4.1e-22:445:65//AP000041
F-HEMBA1005475//CIT-HSP-2322D14.TR CIT-HSP Homo sapiens genomic clone 2322D14, genomic survey sequence.//6.7e-51:269:97//AQ026941
F-HEMBA1005497//HS_3097_A2_G05_T7 CIT Approved Human Genomic Sperm Library D Homo sapiens genomic clone Plate=3097 Col=10 Row=M, genomic survey sequence.//1.4e-66:345:96//AQ103810
F-HEMBA1005500//Homo sapiens PAC clone DJ1093017 from 7q11.23-q21, complete sequence.//5.4e-178:818:98//AC004957
F-HEMBA1005506//Mus musculus (clone 0EBF17) early B-cell factor (EBF) mRNA, complete cds.//2.6e-06:73:98//L12147
F-HEMBA1005508//Homo sapiens clone hRPK.1_A_1, complete sequence.//0.00012:455:60//AC006196
F-HEMBA1005511//Homo sapiens MHC class 1 region.//3.3e-43:421:77//AF055066
F-HEMBA1005513//Drosophila melanogaster males-absent on the first (mof) gene, complete cds.//2.3e-20:352:69//U71219
F-HEMBA1005517//Homo sapiens DNA for (CGG)n trinucleotide repeat region, isolate E7.//2.5e-08:431:62//AJ001216
F-HEMBA1005518//M.musculus mRNA for paladin gene.//8.2e-90:651:81//X99384
F-HEMBA1005520//Homo sapiens clone DJ0876A24, WORKING DRAFT SEQUENCE, 6 unordered pieces.//7.8e-167:755:99//AC004913
F-HEMBA1005526//Homo sapiens chromosome 9, clone hRPK.202_H_3, complete sequence.//2.4e-42:475:73//AC006241
F-HEMBA1005528//Mus musculus mCAF1 protein mRNA, complete cds.//1.2e-94:512:92//U21855
F-HEMBA1005530
F-HEMBA1005548//Human DNA sequence *** SEQUENCING IN PROGRESS *** from clone 970A17, WORKING DRAFT SEQUENCE.//9.4e-87:422:99//AL034431
F-HEMBA1005552//Homo sapiens PAC clone DJ0807C15 from 7q34-q36, complete sequence.//6.1e-41:486:68//AC004743
F-HEMBA1005558//Drosophila melanogaster DNA sequence (P1 DS00837 (D87)), complete sequence.//2.9e-19:306:68//AC004377
F-HEMBA1005568//Plasmodium falciparum 3D7 chromosome 12 PFYAC812 genomic sequence, WORKING DRAFT SEQUENCE, 8 unordered pieces.//0.0093:345:60//AC004153
F-HEMBA1005570//Plasmodium falciparum chromosome 2, section 44 of 73 of the complete sequence.//4.2e-09:592:59//AE001407
F-HEMBA1005576//Homo sapiens mRNA for KIAA0463 protein, partial cds.//5.9e-127:610:98//AB007932
F-HEMBA1005577//HS-1004-A1-E11 -MR.abi CIT Human Genomic Sperm Library C Homo sapiens genomic clone Plate=CT 498 Col=21 Row=I, genomic survey sequence.//0.00034:254:64//B30971
F-HEMBA1005581//Rattus norvegicus mRNA for MEGF5, complete cds.//4.0e-57:826:65//AB011531
F-HEMBA1005582//HS_3242_A1_B07_MR CIT Approved Human Genomic Sperm Library D Homo sapiens genomic clone Plate=3242 Col=13 Row=C, genomic survey sequence.//1.1e-13:91:98//AQ211275
F-HEMBA1005583
F-HEMBA1005588//Homo sapiens PAC clone DJ1188N21 from 7q11.23-q21.1, complete sequence.//8.7e-31:283:75//AC006025
F-HEMBA1005593//Homo sapiens chromosome 17, clone hRPK.332_H_18, complete sequence.//8.3e-158:748:99//AC005746
F-HEMBA1005595//CIT-HSP-2309F14.TF CIT-HSP Homo sapiens genomic clone 2309F14, genomic survey sequence.//6.4e-30:194:91//AQ016527
F-HEMBA1005606//CIT-HSP-2326I6.TR CIT-HSP Homo sapiens genomic clone 2326I6, genomic survey sequence.//0.0014:132:70//AQ041484
F-HEMBA1005609//Homo sapiens clone RG315H11, WORKING DRAFT SEQUENCE, 5 unordered pieces.//2.9e-33:249:85//AC005089
F-HEMBA1005616//Homo sapiens DNA sequence from PAC 43C13 on chromosome Xq21.1-Xq21.3. rab proteins geranylgeranyltransferase component A 1 (rab escort protein 1) (REP-1) (choroideraemia protein) (TCD protein).//6.5e-29:279:69//AL009175
F-HEMBA1005621//Human DNA sequence *** SEQUENCING IN PROGRESS *** from clone 330012, WORKING DRAFT SEQUENCE.//6.4e-90:158:87//AL031731
F-HEMBA1005627//RPCI11-34P9 TJ RPCI-11 Homo sapiens genomic clone RPCI-11-34P9, genomic survey sequence.//0.014:168:67//AQ045110
F-HEMBA1005631//Homo sapiens PAC clone DJ1086D14, complete sequence.//1.0e-149:736:93//AC004460
F-HEMBA1005632
F-HEMBA1005634//Human DNA sequence from PAC 187N21 on chromosome 6p21.2-6p21.33. Contains ESTs.//6.6e-38:452:67//Z98036
F-HEMBA1005666
F-HEMBA1005670//Homo sapiens PAC clone DJ0665C04 from 7p14-p13, complete sequence.//5.1e-59:687:74//AC004850
F-HEMBA1005679//Homo sapiens clone DJ0425I02, WORKING DRAFT SEQUENCE, 5 unordered pieces.//1.0e-47:357:85//AC005478
F-HEMBA1005680
F-HEMBA1005685//RPCI11-23D19.TKBR RPCI-11 Homo sapiens genomic clone RPCI-11-23D19, genomic survey sequence.//0.99:228:63//AQ013742
F-HEMBA1005699//Human ligand for eph-related receptor tyrosine kinases (EPLG8) mRNA, complete cds.//1.4e-72:406:92//U57001
F-HEMBA1005705//Human (D21S172) DNA segment containing (CA) repeat.//0.00040:190:66//X56513
F-HEMBA1005717//Plasmodium falciparum MAL3P1, complete sequence.//0.0099:260:63//Z97348
F-HEMBA1005732//Human mRNA for KIAA0003 gene, complete cds.//8.1e-19:151:88//D14697
F-HEMBA1005737//Homo sapiens PAC clone DJ1099C19 from 7q21-q22, complete sequence.//5.6e-15:157:79//AC005156
F-HEMBA1005746//RPCI11-63N8.TK RPCI11 Homo sapiens genomic clone R-63N8, genomic survey sequence.//1.3e-18:113:100//AQ238535
F-HEMBA1005755//Homo sapiens DNA sequence from PAC 95C20 on chromosome Xp11.3-11.4. Contains STSs and the DXS7 locus with GT and GTG repeat polymorphisms, complete sequence.//3.6e-56:764:70//Z97181
F-HEMBA1005765//Human DNA sequence from PAC 288L1 on chromosome 22q12-qter contains ESTs and polymorphic CA repeat (D22S1152).//1.1e-30:275:77//Z82196
F-HEMBA1005780//RPCI11-74E19.TJ RPCI11 Homo sapiens genomic clone R-74E19, genomic survey sequence.//0.0011:283:62//AQ268432
F-HEMBA1005813//Homo sapiens PAC clone DJ0167F23 from 7p15, complete sequence.//0.14:326:61//AC004079
F-HEMBA1005815//M.musculus mRNA for skeletal muscle-specific calpain.//6.3e-10:706:59//X92523
F-HEMBA1005822//Mouse Bac 291G16, WORKING DRAFT SEQUENCE, 19 unordered pieces.//0.87:417:56//AC003020
F-HEMBA1005829//Homo sapiens Chromosome 22q11.2 Fosmid Clone f39e1 In DGCR Region, complete sequence.//8.8e-42:370:79//AC000094
F-HEMBA1005834//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//2.1e-42:690:67//AL022577
F-HEMBA1005852//F.rubripes GSS sequence, clone 163A22aE9, genomic survey sequence.//4.3e-07:253:59//AL018749
F-HEMBA1005853//CIT-HSP-2289L23.TR CIT-HSP Homo sapiens genomic clone-2289L23, genomic survey sequence.//2.2e-68:333:99//B98952
F-HEMBA1005884//Homo sapiens chromosome 5, BAC clone 78c6 (LBNL H191), complete sequence.//1.9e-57:331:87//AC005351
F-HEMBA1005891//Homo sapiens PAC clone DJ0997N05 from 7q11.23-q21.1, complete sequence.//5.1e-182:864:98//AC004945
F-HEMBA1005894//Homo sapiens, WORKING DRAFT SEQUENCE, 52 unordered pieces.//3.0e-44:340:80//AC004086
F-HEMBA1005909//Homo sapiens DNA sequence from PAC 127D3 on chromosome 1q23-25. Contains FMO2 and FMO3 genes for Flavin-containing Monooxygenase 2 and Flavin-containing Monooxygenase 3 (Dimethylaniline Monooxygenase (N-Oxide 3, EC1.14.13.8, Dimethylaniline Oxidase 3, FMO II, FMO 3), and a gene for another, unknown, Flavin-containing Monooxygenase family protein. Contains ESTs and GSSs, complete sequence.//8.3e-12:828:57//AL021026
F-HEMBA1005911//Human DNA sequence from clone 1158E12 on chromosome Xp11.23-11.4 Contains EST, STS, GSS, CpG island, complete sequence.//1.0e-44:328:77//AL031584
F-HEMBA1005921//Homo sapiens chromosome 17, clone hRPK.112_H_10, complete sequence.//1.3e-41:431:77//AC005666
F-HEMBA1005931//Homo sapiens chromosome 12p13.3 clone RPCI4-761J14, WORKING DRAFT SEQUENCE, 60 unordered pieces.//1.1e-29:394:70//AC006086
F-HEMBA1005934//Homo sapiens PAC clone DJ1140G11 from 14q24.3, complete sequence.//8.1e-06:115:80//AC004974
F-HEMBA1005962//RPCI11-17O15.TV RPCI-11 Homo sapiens genomic clone RPCI-11-17015, genomic survey sequence.//9.5e-36:315:84//B82821
F-HEMBA1005963//HS_3055_A1_E08_MR CIT Approved Human Genomic Sperm Library D Homo sapiens genomic clone Plate=3055 Col=15 Row=I, genomic survey sequence.//9.3e-73:372:97//AQ147357
F-HEMBA1005990//Homo sapiens I-1 receptor candidate protein mRNA, complete cds.//1.3e-149:697:99//AF082516
F-HEMBA1005991//Plasmodium falciparum chromosome 2, section 45 of 73 of the complete sequence.//6.3e-07:423:60//AE001408
F-HEMBA1005999//Homo sapiens chromosome 4 clone C0026P05 map 4P16, complete sequence.//3.8e-09:360:64//AC005599
F-HEMBA1006002
F-HEMBA1006005//Homo sapiens MLL (MLL) gene, exons 1-3, and partial cds.//4.5e-83:495:90//AF036405
F-HEMBA1006031
F-HEMBA1006035
F-HEMBA1006036//Human (lambda) DNA for immunogloblin light chain.//2.4e-59:652:74//D87009
F-HEMBA1006042//Homo sapiens chromosome 10 clone CIT987SK-1057L21 map 10q25, complete sequence.//2.1e-43:330:7011AC005386
F-HEMBA1006067//Plasmodium falciparum 3D7 chromosome 12 PFYAC812 genomic sequence, WORKING DRAFT SEQUENCE, 8 unordered pieces.//0.11:433:59//AC004153
F-HEMBA1006081
F-HEMBA1006090//, complete sequence.//4.5e-139:748:92//AC005500
F-HEMBA1006091//Homo sapiens gene encoding telethonin, exons 1 to 2, partial.//0.0091:346:62//AJ011098
F-HEMBA1006100//Homo sapiens chromosome 10 clone CIT987SK-1143A11 map 10q25, complete sequence.//2.8e-18:180:78//AC005880
F-HEMBA1006108//Human DNA sequence from clone 889N15 on chromosome Xq22.1-22.3. Contains part of the gene for a novel protein similar to X. laevis Cortical Thymocyte-Marker CTX, the possibly alternatively spliced gene for 26S Proteasome subunit p28 (Ankyrin repeat protein), a novel gene and exons 36 through 45 of the COL4A6 for Collagen Alpha 6(IV). Contains ESTs, STSs, GSSs and a putative CpG island, complete sequence.//0.26:84:71//AL031177
F-HEMBA1006121//Human DNA sequence *** SEQUENCING IN PROGRESS *** from clone 691N24, WORKING DRAFT SEQUENCE.//5.2e-18:147:87//AL031672
F-HEMBA1006124//CIT-HSP-2355B17.TF CIT-HSP Homo sapiens genomic clone 2355B17, genomic survey sequence.//0.044:225:61//AQ058966
F-HEMBA1006130//CIT-HSP-386A20.TF CIT-HSP Homo sapiens genomic clone 386A20, genomic survey sequence.//8.8e-07:173:69//B55085
F-HEMBA1006138//Homo sapiens DNA sequence from PAC 454M7 on chromosome Xq25-26.3. Contains the OCRL1 gene for Lowe Oculocerebrorenal Syndrome protein OCRL-1. Contains ESTs, STSs and GSSs, complete sequence.//7.5e-22:164:75//AL022162
F-HEMBA1006142//, complete sequence.//7.9e-125:586:99//AC005500
F-HEMBA1006155//H.sapiens CpG island DNA genomic Mse1 fragment, clone 119b6, forward read cpg119b6.ft1a.//1.0:85:72//Z64428
F-HEMBA1006158//Homo sapiens transcription factor forkhead-like 7 (FKHL7) gene, complete cds.//1.1e-185:852:99//AF048693
F-HEMBA1006173//striatum enriched phosphatase=protein-tyrosine-phosphatase [rat, striata, mRNA, 2815 nt].//8.4e-50:642:73//S49400
F-HEMBA1006182//Homo sapiens Chromosome 15q26.1 PAC clone pDJ105i19, complete sequence.//1.4e-22:194:74//AC005318
F-HEMBA1006198
F-HEMBA1006235//Homo sapiens clone 24422 mRNA sequence.//2.6e-175:836:98//AF070557
F-HEMBA1006248//Pinctada fucata mRNA for insoluble protein, complete cds.//8.2e-05:359:61//D86074
F-HEMBA1006252//Human DNA sequence *** SEQUENCING IN PROGRESS *** from clone 531H16, WORKING DRAFT SEQUENCE.//0.98:397:58//AL031664
F-HEMBA1006253
F-HEMBA1006259//HS_2231_A1_D10_MF CIT Approved Human Genomic Sperm Library D Homo sapiens genomic clone Plate=2231 Col=19 Row=G, genomic survey sequence.//1.2e-11:233:68//AQ152722
F-HEMBA1006268//Homo sapiens Xp22-132-134 BAC GSHB-590J15 (Genome Systems Human BAC library) complete sequence.//5.2e-27:156:85//AC004673
F-HEMBA1006272//Human endogenous retrovirus gag mRNA.//8.1e-115:847:80//X72791
F-HEMBA1006278//Mus musculus poly(A) polymerase VI mRNA, complete cds.//2.1e-57:665:70//U58134
F-HEMBA1006283
F-HEMBA1006284//Streptomyces fradiae tylactone synthase, starter module and modules 1-7, (tylG) gene, complete cds.//9.6e-06:623:60//U78289
F-HEMBA1006291//HS_2208_A1_C03_MR CIT Approved Human Genomic Sperm Library D Homo sapiens genomic clone Plate=2208 Col=5 Row=E, genomic survey sequence.//1.2e-13:105:92//AQ091804
F-HEMBA1006293//Sequence 8 from patent US 5721351.//5.6e-77:580:75//I89415
F-HEMBA1006309//Caenorhabditis elegans cosmid F01F1.//1.1e-21:420:63//U13070
F-HEMBA1006310//Rattus norvegicus cytosolic sorting protein PACS-1a (PACS-1) mRNA, complete cds.//6.8e-120:748:85//AF076183
F-HEMBA1006328//Homo sapiens fragile X mental retardation protein (FMR-1) gene (6 alternative splices), complete cds.//1.5e-46:485:73//L29074
F-HEMBA1006334//HS-1051-B2-F01-MR.abi CIT Human Genomic Sperm Library C Homo sapiens genomic clone Plate=CT 773 Col=2 Row=L, genomic survey sequence.//0.0032:61:91//B40563
F-HEMBA1006344//HS-1009-A2-B02-MF.abi CIT Human Genomic Sperm Library C Homo sapiens genomic clone Plate=CT 331 Col=4 Row=C, genomic survey sequence.//3.3e-09:218:66//B31420
F-HEMBA1006347//Drosophila melanogaster males-absent on the first (mof) gene, complete cds.//1.6e-31:484:68//U71219
F-HEMBA1006349//HS-1054-A1-G06-MR.abi CIT Human Genomic Sperm Library C Homo sapiens genomic clone Plate=CT 776 Col=11 Row=M, genomic survey sequence.//5.4e-15:95:100//B41671
F-HEMBA1006359//Human ZNF43 mRNA.//1.4e-115:823:81//X59244
F-HEMBA1006364//Mouse mRNA for transforming growth factor-beta2.//2.7e-10:247:71//X57413
F-HEMBA1006377//Mus musculus chromosome 7, clone 19K5, complete sequence.//3.0e-57:401:81//AC002327
F-HEMBA1006380//CIT-HSP-2172K18.TF CIT-HSP Homo sapiens genomic clone 2172K18, genomic survey sequence.//1.3e-110:525:99//B92570
F-HEMBA1006381//HS-1045-B2-F10-MF.abi CIT Human Genomic Sperm Library C Homo sapiens genomic clone Plate=CT 828 Col=20 Row=L, genomic survey sequence.//4.4e-05:163:70//B37813
F-HEMBA1006398//Homo sapiens 12q24.2 BAC RPCI11-360E11 (Roswell Park Cancer Institute Human BAC Library) complete sequence.//3.8e-62:370:86//AC004806
F-HEMBA1006416//Homo sapiens chromosome 5, P1 clone 1041F10 (LBNL H88), complete sequence.//3.7e-15:157:78//AC005179
F-HEMBA1006419//Human DNA sequence from clone 71L16 on chromosome Xp11. Contains a probable Zinc Finger protein (pseudo)gene, an unknown putative gene, a pseudogene with high similarity to part of antigen KI-67, a putative Chondroitin 6-Sulfotransferase LIKE gene and a KIAA0267 LIKE putative Na(+)/H(+) exchanger protein gene. Contains a predicted CpG island, ESTs, STSs and GSSs and genomic markers DXS1003 and DXS1055, complete sequence.//1.2e-39:752:63//AL022165
F-HEMBA1006421//Homo sapiens chromosome 14q24.3 clone BAC270M14 transforming growth factor-beta 3 (TGF-beta 3) gene, complete cds; and unknown genes.//2.4e-41:438:76//AF107885
F-HEMBA1006424//Human DNA sequence from clone 51J12 on chromosome 6q26-27. Contains the 3' part of the alternatively spliced gene for the human orthologs of mouse QKI-7 and QKI-7B (KH Domain RNA Binding proteins) and zebrafish ZKQ-1 (Quaking protein homolog). Contains ESTs, STSs and GSSs, complete sequence.//0.027:293:64//AL031781
F-HEMBA1006426//Human DNA sequence *** SEQUENCING IN PROGRESS *** from clone 292E10, WORKING DRAFT SEQUENCE.//1.7e-50:310:80//Z93930
F-HEMBA1006438//Liverwort Marchantia polymorpha chloroplast genome DNA.//0.051:440:59//X04465
F-HEMBA1006445//Felis catus ras p21 (H-ras) mRNA, partial cds.//1.0:238:59//U62088
F-HEMBA1006446//Plasmodium falciparum DNA *** SEQUENCING IN PROGRESS *** from MAL1P6, WORKING DRAFT SEQUENCE.//2.4e-05:702:58//AL031749
F-HEMBA1006461//Homo sapiens chromosome 19, cosmid R30676, complete sequence.//8.6e-55:409:83//AC004560
F-HEMBA1006467//Homo sapiens chromosome 17, clone hRPK.346_K_10, complete sequence.//1.0:293:59//AC006120
F-HEMBA1006471//Plasmodium falciparum 3D7 chromosome 12 PFYAC1122 genomic sequence, WORKING DRAFT SEQUENCE, 3 unordered pieces.//1.4e-05:731:59//AC004709
F-HEMBA1006474//CIT-HSP-2017H3.TF CIT-HSP Homo sapiens genomic clone 2017H3, genomic survey sequence.//5.2e-60:435:83//B54247
F-HEMBA1006483//Homo sapiens chromosome 5, BAC clone 8e5 (LBNL H167), complete sequence.//2.9e-48:286:84//AC004752
F-HEMBA1006485//Homo sapiens BAC clone NH0044G14 from 7q11.23-21.1, complete sequence.//0.96:283:59//AC006031
F-HEMBA1006486//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//1.8e-14:259:67//AL022577
F-HEMBA1006489//Human DNA sequence *** SEQUENCING IN PROGRESS *** from clone 467K16, WORKING DRAFT SEQUENCE.//6.6e-11:595:61//AL031283
F-HEMBA1006492//Homo sapiens chromosome 17, clone hRPK.269_G_24, complete sequence.//6.0e-122:337:100//AC005828
F-HEMBA1006494//Homo sapiens chromosome 7qtelo BAC E3, complete sequence.//3.8e-23:459:68//AF093117
F-HEMBA1006497//HS_3023_B2_H03_T7 CIT Approved Human Genomic Sperm Library D Homo sapiens genomic clone Plate=3023 Col=6 Row=P, genomic survey sequence.//2.3e-81:433:95//AQ093846
F-HEMBA1006502//H.sapiens 7SL repeat (clones 2-19b).//1.6e-13:86:87//X62364
F-HEMBA1006507//Homo sapiens mRNA for KIAA0666 protein, partial cds.//2.3e-139:470:98//AB014566
F-HEMBA1006521//Human BAC clone RG167B05 from 7q21, complete sequence.//4.3e-27:406:71//AC003991
F-HEMBA1006530//Human DNA sequence *** SEQUENCING IN PROGRESS *** from clone 1018D12, WORKING DRAFT SEQUENCE.//2.9e-27:408:65//AL031650
F-HEMBA1006535//Plasmodium falciparum DNA *** SEQUENCING IN PROGRESS *** from MAL4P1, WORKING DRAFT SEQUENCE.//0.028:599:60//AL034557
F-HEMBA1006540//Homo sapiens multi PDZ domain protein MUPP1 (MUPP1) mRNA, complete cds.//1.4e-171:654:98//AF093419
F-HEMBA1006546//Human DNA sequence from cosmid 232L22, between markers DXS366 and DXS87 on chromosome X contains ESTs glycerol kinase pseudogene.//3.8e-104:811:80//Z73986
F-HEMBA1006559//Mus musculus PRAJA1 (Praja1) mRNA, complete cds.//4.8e-99:386:82//U06944
F-HEMBA1006562//Human fructose-1,6-biphosphatase (FBP1) gene, exon 1.//0.012:322:60//U21925
F-HEMBA1006566//Plasmodium falciparum 3D7 chromosome 12 PFYAC1383 genomic sequence, WORKING DRAFT SEQUENCE, 3 unordered pieces.//0.0026:580:58//AC005504
F-HEMBA1006569//Ovis aries beta actin mRNA, complete cds.//6.3e-08:231:70//U39357
F-HEMBA1006579//CIT-HSP-2380A22.TR CIT-HSP Homo sapiens genomic clone 2380A22, genomic survey sequence.//0.036:250:62//AQ197107
F-HEMBA1006583//Mycobacterium tuberculosis H37Rv complete genome; segment 143/162.//1.0:225:63//AL021841
F-HEMBA1006595//Human DNA sequence *** SEQUENCING IN PROGRESS *** from clone 30A23, WORKING DRAFT SEQUENCE.//3.6e-50:689:69//AL022156
F-HEMBA1006597//Homo sapiens Chromosome 7 BAC Clone 239c10, WORKING DRAFT SEQUENCE, 9 unordered pieces.//1.9e-42:253:84//AC004166
F-HEMBA1006612//RPCI11-88F20.TJ RPCI11 Homo sapiens genomic clone R-88F20, genomic survey sequence.//1.1e-51:266:98//AQ286726
F-HEMBA1006617//HS_2193_B2_H07_MF CIT Approved Human Genomic Sperm Library D Homo sapiens genomic clone Plate=2193 Col=14 Row=P, genomic survey sequence.//1.1e-59:413:85//AQ299685
F-HEMBA1006624//Human DNA sequence from clone 406A7 on chromosome 6q23-24. Contains three pseudogenes similar to Elongation Factor 1-Alpha (EF-1-ALPHA, Statin S1), 60S Acidic Ribosomal Protein P1 and NADH-Ubiquinone Oxidoreductase 15 kDa subunit, and part of the Microtuble Associated Protein E-MAP-115 gene. Contains ESTs, STSs and GSSs, complete sequence.//1.4e-35:257:89//AL023284
F-HEMBA1006631//Homo sapiens Chromosome 11q23 PAC clone pDJ356d6, complete sequence.//9.6e-112:800:83//AC002036
F-HEMBA1006635//Plasmodium falciparum DNA *** SEQUENCING IN PROGRESS *** from MAL1P2, WORKING DRAFT SEQUENCE.//0.15:393:58//AL031745
F-HEMBA1006639//Petromyzon marinus polyadenylate binding protein (PABP) mRNA, complete cds.//9.6e-15:318:68//AF032896
F-HEMBA1006643//Homo sapiens clone DJ0902E20, WORKING DRAFT SEQUENCE, 1 unordered pieces.//0.58:254:65//AC006148
F-HEMBA1006648//Mus musculus integrin binding protein kinase mRNA, complete cds.//1.5e-37:108:88//U94479
F-HEMBA1006652//Homo sapiens chromosome 5, BAC clone 343g16 (LBNL H180), complete sequence.//1.3e-154:671:96//AC005601
F-HEMBA1006653
F-HEMBA1006659//Homo sapiens PAC clone DJ0905J08 from 7p12-p14, complete sequence.//5.2e-110:254:93//AC005189
F-HEMBA1006665//Homo sapiens Xp22 BAC GSHB-590J6 (Genome Systems Human BAC library) complete sequence.//1.4e-14:177:76//AC004554
F-HEMBA1006674//Homo sapiens mRNA for nucleolar protein hNop56.//5.5e-15:122:90//Y12065
F-HEMBA1006676//Homo sapiens chromosome 19, fosmid 37502, complete sequence.//0.098:218:63//AC004755
F-HEMBA1006682//Human DNA sequence *** SEQUENCING IN PROGRESS *** from clone 668J24, WORKING DRAFT SEQUENCE.//1.4e-05:719:57//AL034346
F-HEMBA1006695//Homo sapiens clone DJ0935K16, complete sequence.//3.1e-22:151:78//AC006011
F-HEMBA1006696//CITBI-E1-2522D16.TF CITBI-E1 Homo sapiens genomic clone 2522D16, genomic survey sequence.//5.6e-17:324:66//AQ280738
F-HEMBA1006708
F-HEMBA1006709
F-HEMBA1006717//Homo sapiens clone GS308H05, WORKING DRAFT SEQUENCE, 6 unordered pieces.//3.3e-08:136:79//AC005537
F-HEMBA1006737//Homo sapiens chromosome 17, clone hRPK.269_G_24, complete sequence.//5.8e-162:497:98//AC005828
F-HEMBA1006744//Homo sapiens Chromosome 11p14.3 PAC clone pDJ1034g4, complete sequence.//7.4e-48:320:87//AC004796
F-HEMBA1006754//Human DNA sequence from PAC 82J11 and cosmid U134E6 on chromosome Xq22. Contains NIK like and Thyroxin-binding globulin precursor (T4-binding globulin, TBG) genes, ESTs and STSs.//4.1e-129:804:85//Z83850
F-HEMBA1006758//Homo sapiens chromosome 5, BAC clone 182a8 (LBNL H161), complete sequence.//2.2e-162:766:99//AC005752
F-HEMBA1006767//Human Xq28 cosmid U247A3 from LLOXNC01 X chromosome library, complete sequence.//1.2e-19:326:69//U73465
F-HEMBA1006779//Human DNA sequence from clone 80I19 on chromosome 6p21.31-22.2 Contains genes and pseudogenes for olfactory receptor-like proteins, STS, GSS, complete sequence.//1.4e-103:355:87//AL022727
F-HEMBA1006780//CIT-HSP-2359P7.TR CIT-HSP Homo sapiens genomic clone 2359P7, genomic survey sequence.//0.072:147:68//AQ077208
F-HEMBA1006789//nbxb0037I13r CUGI Rice BAC Library Oryza sativa genomic clone nbxb0037I13r, genomic survey sequence.//0.00011:288:63//AQ290474
F-HEMBA1006795//CIT-HSP-2307E3.TF CIT-HSP Homo sapiens genomic clone 2307E3, genomic survey sequence.//5.1e-80:420:96//AQ020511
F-HEMBA1006796//Human clone 23803 mRNA, partial cds.//4.5e-06:202:68//U79298
F-HEMBA1006807//Homo sapiens mRNA for SPOP.//1.2e-66:651:73//AJ000644
F-HEMBA1006821//Homo sapiens chromosome 17, clone hRPC.62_O_9, complete sequence.//6.0e-116:541:99//AC004797
F-HEMBA1006824//Homo sapiens chromosome 19, cosmid R29368, complete sequence.//0.40:159:66//AC004262
F-HEMBA1006832//Homo sapiens (subclone 3_g8 from P1 H25) DNA sequence, complete sequence.//1.8e-24:323:71//AC002196
F-HEMBA1006849//Homo sapiens genomic DNA of 9q32 anti-oncogene of flat epitherium cancer, segment 4/10.//0.15:403:60//AB020872
F-HEMBA1006865//Plasmodium falciparum chromosome 2, section 6 of 73 of the complete sequence.//0.20:472:57//AE001369
F-HEMBA1006877//Mus musculus clone OST9241, genomic survey sequence.//3.4e-79:641:76//AF046757
F-HEMBA1006885//HS_2208_B2_G06_MR CIT Approved Human Genomic Sperm Library D Homo sapiens genomic clone Plate=2208 Col=12 Row=N, genomic survey sequence.//4.9e-18:206:76//AQ089246
F-HEMBA1006900//Human DNA sequence from clone 496N17 on chromosome 6p11.2-12.3 Contains EST, GSS, complete sequence.//5.4e-07:298:65//AL031321
F-HEMBA1006914//S.pombe chromosome II cosmid c16H5.//0.00040:194:66//AL022104
F-HEMBA1006921//Homo sapiens BAC clone GS114I09 from 7p14-p15, complete sequence.//1.1e-174:813:99//AC006027
F-HEMBA1006926//Caenorhabditis elegans cosmid ZK185.//0.0075:183:65//AF036704
F-HEMBA1006929//P.falciparum complete gene map of plastid-like DNA (IR-A).//4.0e-06:739:57//X95275
F-HEMBA1006936
F-HEMBA1006938//Plasmodium falciparum DNA *** SEQUENCING IN PROGRESS *** from MAL1P4, WORKING DRAFT SEQUENCE.//1.1e-05:733:57//AL031747
F-HEMBA1006941//Homo sapiens mRNA for putative thioredoxin-like protein.//1.3e-90:437:98//AJ010841
F-HEMBA1006949//Human DNA sequence from PAC 363L9 on chromosome X. contains STS and polymorphic CA repeat.//0.67:217:62//Z82205
F-HEMBA1006973//Homo sapiens rab3-GAP regulatory domain mRNA, complete cds.//5.6e-143:740:94//AF004828
F-HEMBA1006976//cDNA encoding alpha 2 to 3 sialyltransferase.//2.8e-101:338:89//E06058
F-HEMBA1006993//Homo sapiens BAC clone BK085E05 from 22q12.1-qter, complete sequence.//7.1e-31:536:66//AC003071
F-HEMBA1006996//Human DNA sequence from clone J428A131, WORKING DRAFT SEQUENCE://9.5e-07:285:60//Z82209
F-HEMBA1007002//Genomic sequence for Arabidopsis thaliana BAC F20N2, complete sequence.//0.99:388:58//AC002328
F-HEMBA1007017//Sequence 3 from Patent WO9416067.//0.96:220:62//A39358
F-HEMBA1007018//G.gallus mRNA for dynein light chain-A.//1.3e-124:838:83//X79088
F-HEMBA1007045
F-HEMBA1007051//Caenorhabditis elegans cosmid Y57G11C, complete sequence.//0.17:343:60//Z99281
F-HEMBA1007052//Homo sapiens FSHD-associated repeat DNA, proximal region.//4.3e-67:659:74//U85056
F-HEMBA1007062//Tubulin gene.//1.0:113:67//A18572
F-HEMBA1007066//HS_3116_A2_A03_T7 CIT Approved Human Genomic Sperm Library D Homo sapiens genomic clone Plate=3116 Col=6 Row=A, genomic survey sequence.//0.80:214:62//AQ140467
F-HEMBA1007073//Homo sapiens 12q13 PAC RPCI1-316M24 (Roswell Park Cancer Institute Human PAC library) complete sequence.//9.3e-54:519:68//AC004242
F-HEMBA1007078//CIT-HSP-2318N6.TF CIT-HSP Homo sapiens genomic clone 2318N6, genomic survey sequence.//8.7e-80:387:98//AQ044076
F-HEMBA1007080
F-HEMBA1007085//Streptomyces coelicolor cosmid 7A1.//3.5e-06:496:59//AL034447
F-HEMBA1007087//Plasmodium falciparum MAL3P6, complete sequence.//7.4e-07:553:56//Z98551
F-HEMBA1007112//HS_2171_A1_B01_T7 CIT Approved Human Genomic Sperm Library D Homo sapiens genomic clone Plate=2171 Col=1 Row=C, genomic survey sequence.//1.0:172:61//AQ091865
F-HEMBA1007113//Human DNA sequence from clone 1044O17 on chromosome Xp11.3-11.4 Contains GSS and STS, complete sequence.//0.54:502:56//AL023875
F-HEMBA1007121//Caenorhabditis elegans cosmid ZK430.//1.4e-08:265:64//U42833
F-HEMBA1007129//CITBI-E1-2504A5.TF CITBI-E1 Homo sapiens genomic clone 2504A5, genomic survey sequence.//0.97:267:62//AQ264035
F-HEMBA1007147//HS_3208_A2_C04_T7 CIT Approved Human Genomic Sperm Library D Homo sapiens genomic clone Plate=3208 Col=8 Row=E, genomic survey sequence.//9.1e-90:466:95//AQ176696
F-HEMBA1007149//Homo sapiens chromosome 19, cosmid F23149, complete sequence.//6.0e-138:524:98//AC005239
F-HEMBA1007151//CITBI-E1-2522H6.TF CITBI-E1 Homo sapiens genomic clone 2522H6, genomic survey sequence.//2.0e-20:157:87//AQ280780
F-HEMBA1007174//Homo sapiens epsin 2a mRNA, complete cds.//2.0e-62:318:97//AF062085
F-HEMBA1007178//Homo sapiens chromosome 12p13.3 clone RPCI11-372B4, WORKING DRAFT SEQUENCE, 129 ordered pieces.//1.6e-21:205:80//AC005911
F-HEMBA1007194//HS_3124_B2_H08_MR CIT Approved Human Genomic Sperm Library D Homo sapiens genomic clone Plate=3124 Col=16 Row=P, genomic survey sequence.//1.3e-11:87:96//AQ187492
F-HEMBA1007203//Homo sapiens mRNA for KIAA0214 protein, complete cds.//1.7e-156:478:98//D86987
F-HEMBA1007206//Homo sapiens chromosome 17, clone HRPC837J1, complete sequence.//0.024:342:63//AC004223
F-HEMBA1007224//Homo sapiens mRNA for KIAA0797 protein, partial cds.//5.0e-176:839:98//AB018340
F-HEMBA1007243//Chinese hamster hprt mRNA, complete cds.//4.3e-58:687:68//J00060
F-HEMBA1007251//Rabbit troponin T messenger fragment (aa 49 to 129).//0.084:177:62//V00899
F-HEMBA1007256//Human DNA sequence *** SEQUENCING IN PROGRESS *** from clone 328E19, WORKING DRAFT SEQUENCE.//1.3e-75:490:88//AL022240
F-HEMBA1007267//HS_3218_A1_F07_MR CIT Approved Human Genomic Sperm Library D Homo sapiens genomic clone Plate=3218 Col=13 Row=K, genomic survey sequence.//2.9e-62:393:87//AQ181128
F-HEMBA1007273//CIT-HSP-2171B10.TF CIT-HSP Homo sapiens genomic clone 2171B10, genomic survey sequence.//1.1e-63:314:99//B95401
F-HEMBA1007279//Homo sapiens Chromosome 16 BAC clone CIT987SK-A-116A10, complete sequence.//3.1e-31:401:72//AC004638
F-HEMBA1007281//HS_3115_A1_A11_T7 CIT Approved Human Genomic Sperm Library D Homo sapiens genomic clone Plate=3115 Col=21 Row=A, genomic survey sequence.//5.0e-70:372:96//AQ186691
F-HEMBA1007288//Human DNA sequence from clone 422G23 on chromosome 6q24 Contains EST, STS, GSS, CpG island, complete sequence.//1.2e-152:727:98//AL031003
F-HEMBA1007300//Canis familiaris PDE5 mRNA for 3',5'-Cyclic GMP Phosphodiesterase, complete cds.//2.1e-21:542:63//AB008467
F-HEMBA1007301//COL1A1=type I collagen pro alpha 1(I) chain propeptide {3' region} [human, fetal cells 86-237, 86-146, 88-251, mRNA Partial Mutant, 855 nt].//1.7e-08:388:61//S64596
F-HEMBA1007319//Genomic sequence from Mouse 9, complete sequence.//6.0e-84:390:75//AC000399
F-HEMBA1007320
F-HEMBA1007322//Homo sapiens BAC clone RG118E13 from 7p15-p21, complete sequence.//0.091:260:64//AC004485
F-HEMBA1007327//Plasmodium falciparum 3D7 chromosome 12 PFYACB8-420 genomic sequence, WORKING DRAFT SEQUENCE, 14 unordered pieces.//0.12:472:59//AC005140
F-HEMBA1007341//Homo sapiens chromosome 17, clone hRPK.346_K_10, complete sequence.//1.5e-18:408:64//AC006120
F-HEMBA1007342//Homo sapiens clone DJ1136G02, WORKING DRAFT SEQUENCE, 4 unordered pieces.//8.7e-25:500:62//AC005377
F-HEMBA1007347//Homo sapiens chromosome 5, BAC clone 7g12 (LBNL H126), complete sequence.//0.75:269:61//AC005738
F-HEMBB1000005//Homo sapiens chromosome Y, clone 264,M,20, complete sequence.//5.0e-05:441:60//AC004617
F-HEMBB1000008//Homo sapiens BAC clone RG139P11 from 7q11-q21, complete sequence.//1.0e-44:417:77//AC004491
F-HEMBB1000018//HS_2179_B2_E04_MF CIT Approved Human Genomic Sperm Library D Homo sapiens genomic clone Plate=2179 Col=8 Row=J, genomic survey sequence.//0.012:87:77//AQ023250
F-HEMBB1000024//Human DNA sequence from PAC 106I20 on chromosome 22q12-qter contains NADH pseudogene, ESTs, STS.//8.1e-11:461:61//Z81369
F-HEMBB1000025//CIT-HSP-2348F3.TR CIT-HSP Homo sapiens genomic clone 2348F3, genomic survey sequence.//0.96:198:62//AQ062938
F-HEMBB1000030//Homo sapiens DNA sequence from PAC 32F7 on chromosome X. Contains NUCLEOSOME ASSEMBLY PROTEIN 1-LIKE 3, ESTs.//0.00049:276:64//AL009173
F-HEMBB1000036//H.sapiens chromosome 22 CpG island DNA genomic Mse1 fragment, clone 302e2, reverse read 302e2.r.//0.0057:66:81//Z79857
F-HEMBB1000037//Homo sapiens erythroblast macrophage protein EMP mRNA, complete cds.//1.9e-100:450:98//AF084928
F-HEMBB1000039//HS_2167_B1_F12_T7 CIT Approved Human Genomic Sperm Library D Homo sapiens genomic clone Plate=2167 Col=23 Row=L, genomic survey sequence.//0.022:108:69//AQ092404
F-HEMBB1000044//Borrelia burgdorferi (section 50 of 70) of the complete genome.//1.0e-07:486:61//AE001164
F-HEMBB1000048//Plasmodium falciparum 3D7 chromosome 12 PFYACB8-628 genomic sequence, WORKING DRAFT SEQUENCE, 9 unordered pieces.//5.3e-05:585:58//AC005507
F-HEMBB1000050//Homo sapiens DNA sequence from clone 501N12 on chromosome 6p22.1-22.3 Contains a gene almost identical to four genes of unknown function, a pseudogene, three (pseudo?) genes similar to genes of unknown function, an unknown gene similar to a rat EST, a PX19 LIKE pseudogene and another unknown gene. Contains ESTs, STSs and GSSs, complete sequence.//5.8e-38:549:67//AL022170
F-HEMBB1000054//Homo sapiens Xp22 PAC RPCI1-167A22 (from Roswell Park Cancer Center) complete sequence.//7.0e-98:328:83//AC002349
F-HEMBB1000055//Homo sapiens genomic DNA for centromeric end of MHC class I region on chromosome 6, cosmid clone: TY2F10, WORKING DRAFT SEQUENCE.//3.7e-05:600:58//AB000880
F-HEMBB1000059//Homo sapiens clone RG339C12, WORKING DRAFT SEQUENCE, 10 unordered pieces.//1.3e-48:472:78//AC005096
F-HEMBB1000083
F-HEMBB1000089//Plasmodium falciparum DNA *** SEQUENCING IN PROGRESS *** from MAL1P1, WORKING DRAFT SEQUENCE.//0.0036:679:56//AL031744
F-HEMBB1000099//Homo sapiens chromosome 18 BAC RPCI11-128D14 (Roswell Park Cancer Institute Human BAC Library) complete sequence.//1.1e-15:312:68//AC005909
F-HEMBB1000103//Homo sapiens Xp22-150 BAC GSHB-309P15 (Genome Systems Human BAC Library) complete sequence.//1.0e-37:316:74//AC006210
F-HEMBB1000113//Homo sapiens chromosome 21q22.3 cosmid Q11M15, complete sequence.//3.1e-25:259:76//AF045450
F-HEMBB1000119//Homo sapiens ASMTL gene.//1.2e-137:654:98//Y15521
F-HEMBB1000136//Mycobacterium tuberculosis H37Rv complete genome; segment 127/162.//0.59:217:66//Z74697
F-HEMBB1000141//Homo sapiens DNA from choromosome 19q13.1 cosmid f14121 containing ATP4A and GADPH-2 genes, genomic sequence.//8.4e-31:113:88//AD000090
F-HEMBB1000144//Human BAC clone RG114A06 from 7q31, complete sequence.//4.4e-58:339:87//AC002542
F-HEMBB1000173//Homo sapiens 12q24 BAC RPCI11-162P23 (Roswell Park Cancer Institute Human BAC library) complete sequence.//9.4e-160:562:93//AC002996
F-HEMBB1000175
F-HEMBB1000198//HS_3071_A2_A10_MF CIT Approved Human Genomic Sperm Library D Homo sapiens genomic clone Plate=3071 Col=20 Row=A, genomic survey sequence.//0.99:261:61//AQ137388
F-HEMBB1000215//Homo sapiens chromosome 17, clone hRPK.481_C_4, complete sequence.//6.7e-17:138:86//AC005839
F-HEMBB1000217//Arabidopsis thaliana ubiquitin activating enzyme (UBA1) gene, complete cds.//0.00083:287:60//U80808
F-HEMBB1000218//Caenorhabditis elegans cosmid C52A11, complete sequence.//0.90:337:56//Z46792
F-HEMBB1000226//Human DNA sequence from cosmid RJ14 from a contig from the tip of the short arm of chromosome 16, spanning 2Mb of 16p13.3. Contains ESTs and CpG island.//1.7e-90:175:92//Z69890
F-HEMBB1000240//Human G-protein-coupled inwardly rectifying potassium channel (KCNJ3) gene, polymorphic repeat sequence.//0.16:171:62//U07918
F-HEMBB1000244//Homo sapiens clone DJ1129E22, WORKING DRAFT SEQUENCE, 7 unordered pieces.//4.8e-08:355:63//AC005522
F-HEMBB1000250//Homo sapiens protein associated with Myc mRNA, complete cds.//6.6e-155:735:98//AF075587
F-HEMBB1000258//Human adenosine monophosphate deaminase 1 (AMPD1) gene, exons 1-16.//0.58:396:59//M98818
F-HEMBB1000264//Human clone C3 CHL1 protein (CHLR1) mRNA, alternatively spliced, complete cds.//4.4e-32:100:100//U75968
F-HEMBB1000266//Homo sapiens Xp22 BAC GSHB-433024 (Genome Systems Human BAC library) complete sequence.//3.8e-16:176:78//AC004470
F-HEMBB1000272//Plasmodium falciparum chromosome 2, section 6 of 73 of the complete sequence.//0.011:379:58//AE001369
F-HEMBB1000274//Arabidopsis thaliana DNA chromosome 4, BAC clone T5K18 (ESSAII project).//0.92:272:61//AL022580
F-HEMBB1000284//Human Xp22 BAC CT-285I15 (from CalTech/Research Genetics) , PAC RPCI1-27C22 (from Roswell Park Cancer Center), and Cosmid U35B5 (from Lawrence Livermore), complete sequence.//0.00071:568:57//AC002366
F-HEMBB1000307//Human DNA sequence from PAC 29K1 on chromosome 6p21.3-22.2. Contains glutathione peroxidase-like; zinc finger, ESTs, mRNA, STS, tRNAs, olfactory receptor pseudogene.//3.0e-13:439:65//Z98745
F-HEMBB1000312//Homo sapiens clone GS051M12, complete sequence.//0.031:252:65//AC005007
F-HEMBB1000317//Fugu rubripes GSS sequence, clone 060J22aE10, genomic survey sequence.//0.00033:173:65//AL026242
F-HEMBB1000318//HS_3244_B2_H10_T7 CIT Approved Human Genomic Sperm Library D Homo sapiens genomic clone Plate=3244 Col=20 Row=P, genomic survey sequence.//3.9e-85:438:95//AQ252951
F-HEMBB1000335//Homo sapiens chromosome 18, clone hRPK.24_A_23, complete sequence.//0.63:285:61//AC005968
F-HEMBB1000336
F-HEMBB1000337//Homo sapiens chromosome 4 clone B208G5 map 4q25, complete sequence.//0.0014:309:64//AC004051
F-HEMBB1000338//HS_3108_A2_F07_T7 CIT Approved Human Genomic Sperm Library D Homo sapiens genomic clone Plate=3108 Col=14 Row=K, genomic survey sequence.//3.8e-09:331:63//AQ140356
F-HEMBB1000339//Homo sapiens 12q24 PAC RPCI1-46F2 (Roswell Park Cancer Institute Human PAC library) complete sequence.//1.2e-52:295:77//AC002351
F-HEMBB1000341
F-HEMBB1000343//Plasmodium falciparum MAL3P3, complete sequence.//0.00081:397:61//Z98547
F-HEMBB1000354//Human DNA sequence from clone 192P9 on chromosome Xp11.23-11.4. Contains a pseudogene similar to rat Plasmolipin, ESTs and GSSs, complete sequence.//9.1e-34:596:66//AL020989
F-HEMBB1000369//Genomic sequence from Human 17, complete sequence.//0.012:298:60//AC002090
F-HEMBB1000374//Human Xp22 contig of 3 PACS (R7-39D12, R7-134G1, R7-185L21) from the Roswell Park Cancer Institute, complete sequence.//9.3e-69:294:89//U96409
F-HEMBB1000376//Human DNA sequence from clone 751H9 on chromosome 6q13. Contains part of an unknown gene, ESTs, STSs and GSSs, complete sequence.//3.5e-54:352:88//AL034377
F-HEMBB1000391//Trichothecium roseum internal transcribed spacer 1, 5.8S ribosomal RNA gene; and internal transcribed spacer 2, complete sequence.//0.011:168:67//U51982
F-HEMBB1000399//Homo sapiens Rad17-like protein (RAD17) mRNA, complete cds.//2.6e-163:762:98//AF076838
F-HEMBB1000402//Homo sapiens Xq28 BAC PAC and cosmid clones containing FMR2 gene exons 1,2, and 3, complete sequence.//7.7e-15:466:63//AC002368
F-HEMBB1000404//Homo sapiens mRNA for myosin-IXA.//3.5e-65:324:98//AJ001714
F-HEMBB1000420//244Kb Contig from Human Chromsome 11p15.5 spanning D11S1 through D11S25, complete sequence.//0.013:399:62//AC001228
F-HEMBB1000434//Homo sapiens PAC clone 278C19 from 12q, complete sequence.//6.1e-83:571:84//AC004263
F-HEMBB1000438//RPCI11-21E14.TP RPCI-11 Homo sapiens genomic clone RPCI-11-21E14, genomic survey sequence.//0.0030:295:63//B83110
F-HEMBB1000441//Homo sapiens Chromosome 22q12 Cosmid Clone ll47g11, complete sequence.//2.5e-33:372:72//AC000035
F-HEMBB1000449//Human DNA sequence from PAC 296K21 on chromosome X contains cytokeratin exon, delta-aminolevulinate synthase (erythroid); 5-aminolevulinic acid synthase.(EC 2.3.1.37). 6-phosphofructo-2-kinase/fructose-2,6-bisphosphatase (EC 2.7.1.105, EC 3.1.3.46), ESTs and STS.//1.3e-51:534:72//Z83821
F-HEMBB1000455//Saccharomyces cerevisiae mitochondrion origin of replication (ori6) and oli1 gene, complete cds.//0.016:522:58//L36899
F-HEMBB1000472
F-HEMBB1000480
F-HEMBB1000487//Human DNA sequence *** SEQUENCING IN PROGRESS *** from clone 128O3, WORKING DRAFT SEQUENCE.//0.00013:314:64//Z98742
F-HEMBB1000490//Human DNA sequence *** SEQUENCING IN PROGRESS *** from clone 1185N5, WORKING DRAFT SEQUENCE.//4.1e-110:529:98//AL034423
F-HEMBB1000491//Plasmodium falciparum chromosome 2, section 25 of 73 of the complete sequence.//0.10:187:65//AE001388
F-HEMBB1000493//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//3.7e-06:637:58//AL022577
F-HEMBB1000510//Homo sapiens chromosome 17, clone hRPK.112_J_9, complete sequence.//3.1e-96:737:81//AC005553
F-HEMBB1000518//Homo Sapiens Chromosome X clone bWXD171, WORKING DRAFT SEQUENCE, 1 ordered pieces.//0.00014:163:68//AC004676
F-HEMBB1000523//Plasmodium falciparum DNA *** SEQUENCING IN PROGRESS *** from contig 3-105, complete sequence.//0.41:349:56//AL010212
F-HEMBB1000530//H.sapiens mRNA for extracellular matrix protein collagen type XIV, C-terminus.//6.6e-37:138:96//Y11710
F-HEMBB1000550//Homo sapiens genomic DNA of 8p21.3-p22 anti-oncogene of hepatocellular colorectal and non-small cell lung cancer , segment 3/11.//3.9e-56:683:71//AB020860
F-HEMBB1000554//Homo sapiens *** SEQUENCING IN PROGRESS ***, WORKING DRAFT SEQUENCE.//2.2e-51:282:84//AJ011929
F-HEMBB1000556//Homo sapiens mRNA for KIAA0750 protein, complete cds.//6.1e-32:537:65//AB018293
F-HEMBB1000564
F-HEMBB1000573//Homo sapiens clone RG228D17, WORKING DRAFT SEQUENCE, 2 unordered pieces.//8.2e-33:268:73//AC005077
F-HEMBB1000575//Human DNA sequence from clone 323M22 on chromosome 22q13.1-13.2. Contains the 5' part of the human ortholog of chicken P52 and mouse H74, and a novel gene coding for a protein similar to KIAA0173 and worm Tubulin Tyrosine Ligase. Contains ESTs, STSs, GSSs, genomic marker D22S418 and putative CpG islands, complete sequence.//5.8e-47:734:66//AL022476
F-HEMBB1000586//H.sapiens highly polymorphic microsatellite DNA.//0.030:147:67//X79883
F-HEMBB1000589//Homo sapiens Chromosome 16 BAC clone CIT987SK-A-279B10, complete sequence.//6.3e-41:278:83//AC002300
F-HEMBB1000591//Homo sapiens Xp22 bins 45-47 BAC GSHB-665N22 (Genome Systems Human BAC Library) complete sequence.//1.1e-182:871:98//AC005184
F-HEMBB1000592//Hepatitis C virus genomic RNA, 3' nonstranslated region, partial sequence. clone #19.//0.012:185:64//AF009074
F-HEMBB1000593//Homo sapiens chromosome 7q22 sequence, complete sequence.//1.2e-131:353:93//AF053356
F-HEMBB1000598//Homo sapiens 12p13.3 BAC RPCI3-488H23 (Roswell Park Cancer Institute Human BAC Library) complete sequence.//9.1e-58:600:72//AC006207
F-HEMBB1000623//cDNA encoding Coliolus manganese peroxidase.//0.89:284:62//E12284
F-HEMBB1000630//Mus musculus clone NSAT47 nonsatellite RNA sequence.//1.9e-15:129:87//U26231
F-HEMBB1000631//Sequence 26 from patent US 5708157.//3.2e-27:180:88//I80057
F-HEMBB1000632//Human mRNA for KIAA0351 gene, complete cds.//1.6e-48:811:65//AB002349
F-HEMBB1000637//Homo sapiens clone DJ0425I02, WORKING DRAFT SEQUENCE, 5 unordered pieces.//4.1e-58:649:73//AC005478
F-HEMBB1000638//HS_3051_A1_G01_MF CIT Approved Human Genomic Sperm Library D Homo sapiens genomic clone Plate=3051 Col=1 Row=M, genomic survey sequence.//0.0032:497:56//AQ155234
F-HEMBB1000643//Homo sapiens clone RG228D17, WORKING DRAFT SEQUENCE, 2 unordered pieces.//2.4e-50:791:68//AC005077
F-HEMBB1000649//Homo sapiens Chromosome 16 BAC clone CIT987-SK502C10, complete sequence.//5.2e-64:775:69//AC003009
F-HEMBB1000652//Homo sapiens chromosome 10 clone CRI-JC2048 map 10q22.1, WORKING DRAFT SEQUENCE, 4 unordered pieces.//2.7e-52:334:89//AC006186
F-HEMBB1000665//Human DNA sequence from clone 452M16 on chromosome Xq21.1-21.33 Contains capping protein alpha subunit isoform 1 pseudogene, STS, GSS, and CA repeat, complete sequence.//0.0062:426:60//AL024493
F-HEMBB1000671//Human DNA sequence from PAC 93H18 on chromosome 6 contains ESTs heterochromatin protein HP1Hs-gamma pseudogene, STS and CpG island.//9.6e-95:399:78//Z84488
F-HEMBB1000673//HS_3039_A2_C08_MF CIT Approved Human Genomic Sperm Library D Homo sapiens genomic clone Plate=3039 Col=16 Row=E, genomic survey sequence.//3.8e-50:293:92//AQ155121
F-HEMBB1000684//Human DNA sequence *** SEQUENCING IN PROGRESS *** from clone 222E13, WORKING DRAFT SEQUENCE.//8.0e-65 :282:83//Z93241
F-HEMBB1000693//Homo sapiens neuroan1 mRNA, complete cds.//1.6e-118:575:97//AF040723
F-HEMBB1000705//Plasmodium falciparum 3D7 chromosome 12 PFYACB8-628 genomic sequence, WORKING DRAFT SEQUENCE, 9 unordered pieces.//8.6e-07:251:61//AC005507
F-HEMBB1000706//Human DNA sequence *** SEQUENCING IN PROGRESS *** from clone 153G14, WORKING DRAFT SEQUENCE.//2.9e-20:434:64//AL031118
F-HEMBB1000709//Human DNA sequence *** SEQUENCING IN PROGRESS *** from clone 994L9, WORKING DRAFT SEQUENCE.//0.26:184:65//AL034554
F-HEMBB1000725//Rattus norvegicus GTPase Rab8b (Rab8b) mRNA, complete cds.//1.8e-129:692:93//U53475
F-HEMBB1000726//Human Chromosome 16 BAC clone CIT987SK-A-363E6, complete sequence.//2.7e-40:304:80//U91321
F-HEMBB1000738//Human Xq28 cosmids U126G1, U142F2, U69B6, U145C10, U169A5, U84H1, U24D12, U80A7, U153E6, L35485, and R7-163A8 containing iduronate 2-sulfatase gene and pseudogene, complete sequence.//8.9e-35:582:63//AF011889
F-HEMBB1000749//Homo sapiens chromosome 11 clone CIT-HSP-1337H24, WORKING DRAFT SEQUENCE, 9 unordered pieces.//6.2e-46:262:89//AC005849
F-HEMBB1000763//Human DNA sequence *** SEQUENCING IN PROGRESS *** from clone 537K23, WORKING DRAFT SEQUENCE.//1.6e-99:316:98//AL034405
F-HEMBB1000770//Human DNA sequence from clone 80I19 on chromosome 6p21.31-22.2 Contains genes and pseudogenes for olfactory receptor-like proteins, STS, GSS, complete sequence.//0.044:325:60//AL022727
F-HEMBB1000774
F-HEMBB1000781//Sequence 3 from patent US 5753446.//1.2e-92:599:86//AR008277
F-HEMBB1000789//Homo sapiens mRNA for KIAA0677 protein, complete cds.//9.3e-64:672:71//AB014577
F-HEMBB1000790//Homo sapiens 12q13.1 PAC RPCI1-228P16 (Roswell Park Cancer Institute Human PAC Library) complete sequence.//2.4e-41:460:74//AC004801
F-HEMBB1000794//HS_3034_B2_D12_MF CIT Approved Human Genomic Sperm Library D Homo sapiens genomic clone Plate=3034 Col=24 Row=H, genomic survey sequence.//1.8e-74:378:97//AQ117099
F-HEMBB1000807//H.sapiens CpG island DNA genomic Mse1 fragment, clone 39d7, reverse read cpg39d7.rt1a.//8.5e-14:95:97//Z58412
F-HEMBB1000810//H.sapiens chromosome 22 CpG island DNA genomic Mse1 fragment, clone 303a8, complete read.//3.2e-05:138:71//Z79983
F-HEMBB1000821//HS_2168_B1_A12_MF CIT Approved Human Genomic Sperm Library D Homo sapiens genomic clone Plate=2168 Col=23 Row=B, genomic survey sequence.//0.85 :208:60//AQ086361
F-HEMBB1000822//Human BAC clone GS113H23 from 5p15.2, complete sequence.//3.0e-06:361:60//AC003015
F-HEMBB1000826//Human BAC clone RG180F08 from 7q31, complete sequence.//1.1e-27:360:69//AC002431
F-HEMBB1000827
F-HEMBB1000831
F-HEMBB1000835//Human DNA sequence from clone 45I4 on chromosome 6q24.1-24.3. Contains two putative unknown genes, ESTs, STSs and GSSs, complete sequence.//0.00098:234:63//AL023581
F-HEMBB1000840//Human Chromosome 11 Cosmid cSRL97a6, complete sequence.//4.5e-61:328:79//U73649
F-HEMBB1000848//Homo sapiens DNA sequence from PAC 206D15 on chromosome 1q24. Contains a Reduced Folate Carrier protein (RFC) LIKE gene, a mitochondrial ATP Synthetase protein 8 (ATP8, MTATP8) LIKE pseudogene, an unknown gene and the last exon of the JEM1 gene coding for the Basic-Leucine Zipper nuclear factor JEM-1. Contains ESTs, an STS and a BAC end sequence (GSS), complete sequence.//9.7e-144:809:87//AL021068
F-HEMBB1000852//Plasmodium falciparum 3D7 chromosome 12 PFYAC293 genomic sequence, WORKING DRAFT SEQUENCE, 9 unordered pieces.//0.12:492:58//AC004157
F-HEMBB1000870//Plasmodium falciparum 3D7 chromosome 12 PFYAC293 genomic sequence, WORKING DRAFT SEQUENCE, 9 unordered pieces.//0.0024:212:67//AC004157
F-HEMBB1000876//Homo sapiens ELISC-1 mRNA, partial cds.//1.5e-32:200:94//AF085351
F-HEMBB1000883//HS_3065_B2_C04_MR CIT Approved Human Genomic Sperm Library D Homo sapiens genomic clone Plate=3065 Col=8 Row=F, genomic survey sequence.//0.0017:152:66//AQ137687
F-HEMBB1000887
F-HEMBB1000888//CIT-HSP-2329A10.TR CIT-HSP Homo sapiens genomic clone 2329A10, genomic survey sequence.//1.5e-31:172:98//AQ044369
F-HEMBB1000890
F-HEMBB1000893//Plasmodium falciparum MAL3P2, complete sequence.//9.5e-06:768:56//AL034558
F-HEMBB1000908//Homo sapiens clone DJ1119N05, complete sequence.//4.5e-21:199:82//AC004968
F-HEMBB1000910//Plasmodium falciparum DNA *** SEQUENCING IN PROGRESS *** from MAL4P1, WORKING DRAFT SEQUENCE.//0.72:366:59//AL034557
F-HEMBB1000913//HS_3078_B1_C02_MR CIT Approved Human Genomic Sperm Library D Homo sapiens genomic clone Plate=3078 Col=3 Row=F, genomic survey sequence.//9.9e-12:221:63//AQ144507
F-HEMBB1000915//Homo sapiens DNA for (CGG)n trinucleotide repeat region, isolate P4.//1.2e-49:252:99//AJ001215
F-HEMBB1000917//Homo sapiens chromosome 5, P1 clone 254f11 (LBNL H62), complete sequence.//2.3e-42:316:76//AC006077
F-HEMBB1000927//Human BDR-2 mRNA for hippocalcin, complete cds.//3.6e-30:528:65/D16593
F-HEMBB1000947//CpG0856B CpIOWAgDNA1 Cryptosporidium parvum genomic, genomic survey sequence.//0.81:262:62//AQ254493
F-HEMBB1000959//Human DNA sequence *** SEQUENCING IN PROGRESS *** from clone 346O6, WORKING DRAFT SEQUENCE.//1.2e-43:454:75//Z84487
F-HEMBB1000973//Mus musculus schlafen2 (Slfn2) mRNA, complete cds.//8.3e-42:458:72//AF099973
F-HEMBB1000975//Arabidopsis thaliana genomic DNA, chromosome 5, P1 clone: MBK5, complete sequence.//0.98:196:63//AB005234
F-HEMBB1000981
F-HEMBB1000985//Homo sapiens chromosome 19, cosmid R29388, complete sequence.//2.9e-06:566:57//AC004476
F-HEMBB1000991//Human DNA sequence from PAC 238J17 on chromosome 6q22. Contains EST and STS.//0.099:391:57//Z98753
F-HEMBB1000996//Human DNA sequence from BAC 999D10 on chromosome 22q13.3. Contains two BAC end-sequences (GSSs).//6.2e-33:227:80//Z94802
F-HEMBB1001004
F-HEMBB1001008//Human Chromosome 16 BAC clone CIT987SK-A-951C11, complete sequence.//4.0e-13:164:79//AC002551
F-HEMBB1001011//Human Chromosome 16 BAC clone CIT987SK-A-635H12, complete sequence.//7.5e-13:229:69//AC002310
F-HEMBB1001014//Homo sapiens chromosome 16, BAC clone 375G12 (LANL), complete sequence.//0.32:474:58//AC005751
F-HEMBB1001020//Homo sapiens BAC clone 255A7 from 8q21 containing NBS1 gene, complete sequence.//2.6e-39:218:80//AF069291
F-HEMBB1001024//Homo sapiens BAC clone 393I22 from 8q21, complete sequence.//5.3e-05:656:59//AF070717
F-HEMBB1001037//CIT-HSP-2358K16.TF CIT-HSP Homo sapiens genomic clone 2358K16, genomic survey sequence.//6.6e-05:228:64//AQ080539
F-HEMBB1001047//Homo sapiens cosmids Qc14E2, Qc12H12, Qc11F9, Qc10G9, LA1733 and Qc17B8 from Xq28, complete sequence.//4.0e-27:385:71//U82671
F-HEMBB1001051//H.sapiens mRNA for FAN protein.//1.2e-27:160:98//X96586
F-HEMBB1001056//Homo sapiens clone DJ0953A04, WORKING DRAFT SEQUENCE, 5 unordered pieces.//2.3e-89:180:91//AC006014
F-HEMBB1001058//Homo sapiens 3p22-8 PAC RPCI4-736H12 (Roswell Park Cancer Institute Human PAC Library) complete sequence.//1.2e-41:468:74//AC006060
F-HEMBB1001060//Human Tigger1 transposable element, complete consensus sequence.//4.3e-122:785:86//U49973
F-HEMBB1001063//Human DNA sequence *** SEQUENCING IN PROGRESS *** from clone 523G1, WORKING DRAFT SEQUENCE.//7.1e-162:770:99//AL034375
F-HEMBB1001068//Homo sapiens liprin-beta2 mRNA, partial cds.//3.1e-146:736:95//AF034803
F-HEMBB1001096//Buchnera aphidicola genomic fragment containing (chaperone Hsp60) groEL, DNA biosynthesis initiating protein (dnaA), ATP operon (atpCDGAHFEB), and putative chromosome replication protein (gidA) genes, complete cds; and termination factor Rho (rho) gene, partial cds.//0.00088:690:57//AF008210
F-HEMBB1001102//Homo sapiens huntingtin interacting protein HYPH mRNA, partial cds.//2.1e-76:368:99//AF049612
F-HEMBB1001105//CIT-HSP-2185N1.TR CIT-HSP Homo sapiens genomic clone 2185N1, genomic survey sequence.//1.0e-09:136:76//AQ002987
F-HEMBB1001112//Rattus rattus sec61 homologue mRNA, complete cds.//1.0e-108:909:76//M96630
F-HEMBB1001114//Homo sapiens chromosome 17, clone hRPK.795_F_17, complete sequence.//7.2e-07:459:59//AC005284
F-HEMBB1001117//HS_2178_B1_E12_MR CIT Approved-Human Genomic Sperm Library D Homo sapiens genomic clone Plate=2178 Col=23 Row=J, genomic survey sequence.//7.8e-50:331:86//AQ068244
F-HEMBB1001119//Human collagen type XII alpha-1 precursor (COL12A1) mRNA, complete cds.//1.6e-25:150:98//U73778
F-HEMBB1001126
F-HEMBB1001133//Homo sapiens Xp22-132-134 BAC GSHB-590J15 (Genome Systems Human BAC library) complete sequence.//2.8e-24:228:80//AC004673
F-HEMBB1001137
F-HEMBB1001142//Homo sapiens chromosome Y, clone 264,M,20, complete sequence.//1.0e-40:231:76//AC004617
F-HEMBB1001151//Rattus norvegicus golgi peripheral membrane protein p65 (GRASP65) mRNA, complete cds.//2.9e-47:640:67//AF015264
F-HEMBB1001153//CIT-HSP-2359K11.TR CIT-HSP Homo sapiens genomic clone 2359K11, genomic survey sequence.//0.76:136:67//AQ075724
F-HEMBB1001169//Human DNA sequence from PAC 84F12 on chromosome Xq25-Xq26.3. Contains glypican-3 precursor (intestinal protein OCI-5) (GTR2-2), ESTs and CA repeat.//9.9e-63:259:79//AL008712
F-HEMBB1001175//Human mRNA for ankyrin motif, complete cds.//2.2e-34:509:66//D78334
F-HEMBB1001177//CIT-HSP-2321I17.TR CIT-HSP Homo sapiens genomic clone 2321I17, genomic survey sequence.//5.9e-27:320:75//AQ036473
F-HEMBB1001182//RPCI11-30J5.TV RPCI-11 Homo sapiens genomic clone RPCI-11-30J5, genomic survey sequence.//5.7e-06:62:96//B85188
F-HEMBB1001199
F-HEMBB1001208//HS_2026_B1_C07_T7 CIT Approved Human Genomic Sperm Library D Homo sapiens genomic clone Plate=2026 Col=13 Row=F, genomic survey sequence.//0.00018:134:70//AQ229237
F-HEMBB1001209//CITBI-E1-2521F23.TF CITBI-E1 Homo sapiens genomic clone 2521F23, genomic survey sequence.//1.4e-95:464:98//AQ278357
F-HEMBB1001210//HS_3102_A2_F09_MF CIT Approved Human Genomic Sperm Library D Homo sapiens genomic clone Plate=3102 Col=18 Row=K, genomic survey sequence.//2.6e-90:446:98//AQ119196
F-HEMBB1001218//Human DNA sequence *** SEQUENCING IN PROGRESS *** from clone 796F18, WORKING DRAFT SEQUENCE.//1.0e-31:315:72//AL031291
F-HEMBB1001221//Plasmodium falciparum 3D7 chromosome 12 PFYAC1383 genomic sequence, WORKING DRAFT SEQUENCE, 3 unordered pieces.//9.7e-17:770:59//AC005504
F-HEMBB1001234//H.sapiens CpG island DNA genomic Mse1 fragment, clone 39f9, forward read cpg39f9.ft1e//4.0e-30:171:97//Z65435
F-HEMBB1001242//Homo sapiens mRNA for LAK-1, complete cds.//3.8e-30:458:67//AB005754
F-HEMBB1001249//CIT-HSP-2375N19.TF CIT-HSP Homo sapiens genomic clone 2375N19, genomic survey sequence.//0.0076:250:63//AQ109087
F-HEMBB1001253//Homo sapiens genomic DNA, chromosome 21q11.1, segment 3/28, WORKING DRAFT SEQUENCE.//0.0097:89:80//AP000032
F-HEMBB1001254//CIT-HSP-2320E5.TF CIT-HSP Homo sapiens genomic clone 2320E5, genomic survey sequence.//3.7e-54:284:97//AQ037173
F-HEMBB1001267//Homo sapiens chromosome 17, clone hRPK.488_L_1, complete sequence.//3.5e-30:236:78//AC005303
F-HEMBB1001271//HS_3011_A1_G02_T7 CIT Approved Human Genomic Sperm Library D Homo sapiens genomic clone Plate=3011 Col=3 Row=M, genomic survey sequence.//5.2e-07:364:62//AQ214217
F-HEMBB1001282//CIT-HSP-2356J20.TF CIT-HSP Homo sapiens genomic clone 2356J20; genomic survey sequence.//1.8e-16:109:97//AQ060969
F-HEMBB1001288//R.norvegicus mRNA for gephyrin.//3.4e-18:194:77//X66366
F-HEMBB1001289//Genomic sequence from Human 9q34, complete sequence.//4.8e-66:434:74//AC000387
F-HEMBB1001294//HS_3039_B1_D01_MF CIT Approved Human Genomic Sperm Library D Homo sapiens genomic clone Plate=3039 Col=1 Row=H, genomic survey sequence.//2.0e-90:437:99//AQ155035
F-HEMBB1001302
F-HEMBB1001304//CIT-HSP-2053E15.TF CIT-HSP Homo sapiens genomic clone 2053E15, genomic survey sequence.//2.2e-07:370:61//B69144
F-HEMBB1001314//Mus musculus Olf-1/EBF-like-3 transcription factor (O/E-3) mRNA, complete cds.//5.7e-116:663:85//U92703
F-HEMBB1001315//Homo sapiens chromosome 10 clone LA10NC01_40_G_3 map 10q26.1-10q26.2, WORKING DRAFT SEQUENCE, 1 ordered pieces.//2.5e-33:328:77//AC006096
F-HEMBB1001317//Homo sapiens Xp22-150 BAC GSHB-309P15 (Genome Systems Human BAC Library) complete sequence.//1.4e-122:680:91//AC006210
F-HEMBB1001326//Homo sapiens BAC clone RG136N17 from 7p15-p21, complete sequence.//2.8e-09:518:60//AC004129
F-HEMBB1001331//Mus musculus mRNA for hepatoma-derived growth factor, complete cds, strain:BALB/c.//3.7e-56:458:79//D63850
F-HEMBB1001335//HS_3055_A1_H10_MR CIT Approved Human Genomic Sperm Library D Homo sapiens genomic clone Plate=3055 Col=19 Row=O, genomic survey sequence.//1.0:222:63//AQ147384
F-HEMBB1001337//Human PAC clone DJ0093I03 from Xq23, complete sequence.//1.0e-74:319:85//AC003983
F-HEMBB1001339//Homo sapiens FSHD-associated repeat DNA, proximal region.//4.0e-135:856:87//U85056
F-HEMBB1001346//Human familial Alzheimer's disease (STM2) gene, complete cds.//3.3e-44:481:74//U50871
F-HEMBB1001348//Homo sapiens BAC clone NH0491B03 from 7p21-p15, complete sequence.//1.8e-17:210:73//AC006041
F-HEMBB1001356//Homo sapiens clone RG252P22, WORKING DRAFT SEQUENCE, 3 unordered pieces.//1.0:386:59//AC005079
F-HEMBB1001364//Homo sapiens chromosome 17, clone hRPC.842_A_23, complete sequence.//0.97:349:61//AC004662
F-HEMBB1001366//Homo sapiens chromosome 10 clone CIT987SK-1188I5 map 10p11.2-10p12.1, complete sequence.//5.5e-161:766:98//AC005876
F-HEMBB1001367//Homo sapiens chromosome 17, clone hRPC.906_A_24, complete sequence.//3.0e-55:510:76//AC004408
F-HEMBB1001369//Homo sapiens BAC clone RG163K11 from 7q31, complete sequence.//0.048:244:64//AC005192
F-HEMBB1001380//Homo sapiens PAC clone DJ1102B04 from 7q11.23-7q21, complete sequence.//2.5e-26:257:78//AC006204
F-HEMBB1001384//Mus musculus COP9 complex subunit 4 (COPS4) mRNA, complete cds.//5.1e-99:571:89//AF071314
F-HEMBB1001387//Leishmania tarentolae mitochondrial 12S ribosomal RNA gene.//7.1e-05:546:58//X02354
F-HEMBB1001394//Homo sapiens BAC clone GS421I03 from Xq25-q26, complete sequence.//4.0e-129:788:88//AC005023
F-HEMBB1001410//Homo sapiens wbscr1 (WBSCR1) and replication factor C subunit 2 (RFC2) genes, complete cds.//4.8e-11:632:59//AF045555
F-HEMBB1001424//Mus musculus Chromosome 4 BAC clone BacB6, complete sequence.//0.0012:435:59//AC003019
F-HEMBB1001426//Homo sapiens clone DJ0736H05, WORKING DRAFT SEQUENCE, 5 unordered pieces.//3.8e-17:360:64//AC005482
F-HEMBB1001429//leucine aminopeptidase [cattle, kidney, mRNA, 2056 nt].//4.1e-114:668:88//S65367
F-HEMBB1001436//Homo sapiens FUT2 gene, intron 1, complete sequence.//2.3e-37:438:74//AB000931
F-HEMBB1001443//Bos taurus pyruvate dehydrogenase phosphatase mRNA, complete cds.//9.1e-92:550:88//L18966
F-HEMBB1001449//Homo sapiens chromosome 5, PAC clone 228g9 (LBNL H142), complete sequence.//0.00024:385:62//AC004768
F-HEMBB1001454//Homo sapiens chromosome 19, cosmid R34169, complete sequence.//0.84:577:57//AC005790
F-HEMBB1001458//Human Chromosome 11 pac pDJ197h17, WORKING DRAFT SEQUENCE, 11 unordered pieces.//8.0e-40:377:78//AC000382
F-HEMBB1001463//Human Chromosome 16 BAC clone CIT987SK-A-270G1, complete sequence.//0.011:482:59//AF001549
F-HEMBB1001464//Human chromosome 16p13 BAC clone CIT987SK-3H8 complete sequence.//0.019:263:61//U91320
F-HEMBB1001482//Rattus norvegicus Olf-1/EBF associated Zn finger protein Roaz mRNA, alternatively spliced form, complete cds.//1.0e-30:521:66//U92564
F-HEMBB1001500//Homo sapiens clone DJ0742P04, WORKING DRAFT SEQUENCE, 6 unordered pieces.//1.3e-31:479:71//AC004873
F-HEMBB1001521//Homo sapiens clone RG269P13, WORKING DRAFT SEQUENCE, 6 unordered pieces.//3.7e-51:680:70//AC005080
F-HEMBB1001527
F-HEMBB1001531//Homo sapiens Chromosome 22q11.2 Cosmid Clone 89h In DGCR Region, complete sequence.//1.3e-79:696:79//AC000089
F-HEMBB1001535//0.aries DNA for polymorphic marker 'OVINRA01' (339 bp).//0.00034:217:62//X89268
F-HEMBB1001536//Homo sapiens PAC clone DJ1182N03 from 7q11.23-q21.1, complete sequence.//0.54:266:60//AC004548
F-HEMBB1001537//Homo sapiens chromosome 19, cosmid R29368, complete sequence.//4.6e-25:784:61//AC004262
F-HEMBB1001555//Homo sapiens Chromosome 16 BAC clone CIT987SK-A-248F7, complete sequence.//6.9e-50:213:80//AC004605
F-HEMBB1001562//Homo sapiens clone NH0523H20, complete sequence.//0.46:269:60//AC005041
F-HEMBB1001564//Human DNA sequence from clone 192P9 on chromosome Xp11.23-11.4. Contains a pseudogene similar to rat Plasmolipin, ESTs and GSSs, complete sequence.//1.7e-107:620:83//AL020989
F-HEMBB1001565//Homo sapiens BAC clone RG437L15 from 8q21, complete sequence.//2.4e-50:734:67//AC004003
F-HEMBB1001585//Human DNA sequence from clone 790B6 on chromosome 20p11.22-12.2. Contains STSs and GSSs, complete sequence.//1.4e-166:816:97//AL031677
F-HEMBB1001586
F-HEMBB1001588//Homo sapiens chromosome 19, CIT-HSP-444n24, complete sequence.//1.6e-21:419:65//AC005261
F-HEMBB1001603
F-HEMBB1001618//Homo sapiens DNA sequence from PAC 142L7 on chromosome 6q21. Contains a Laminin Alpha 4 (LAMA4) LIKE gene coding for two alternatively spliced transcripts, a Tubulin Beta LIKE pseudogene, a Connective tissue growth factor (NOV, GIG) LIKE gene, A predicted CpG island, ESTs, STSs and genomic marker D6S416, complete sequence.//4.5e-29:422:72//Z99289
F-HEMBB1001619//HS_3079_B1_A04_MR CIT Approved Human Genomic Sperm Library D Homo sapiens genomic clone Plate=3079 Col=7 Row=B, genomic survey sequence.//0.0010:77:79//AQ123388
F-HEMBB1001630//Homo sapiens clone RG315H11, WORKING DRAFT SEQUENCE, 5 unordered pieces.//3.2e-12:667:59//AC005089
F-HEMBB1001635//Plasmodium falciparum MAL3P7, complete sequence.//3.8e-05:475:57//AL034559
F-HEMBB1001637//Homo sapiens DNA sequence from PAC 934G17 on chromosome 1p36.21. Contains the alternatively spliced CLCN6 gene for chloride chanel proteins CLC-6A (KIAA0046) -B, -C and -D, the alternatively spliced NPPA gene coding for Atrial Natriuretic Factor ANF precursor (Atrial Natriuretic peptide ANP, Prepronatriodilatin), the NPPB gene for Brain Natriuretic Protein BNP, and a pseudogene similar to SBF1 (and other Myotubularin-related protein genes). Contains ESTs, STSs and the genomic marker D1S2740, complete sequence.//9.2e-13:168:76//AL021155
F-HEMBB1001641//Arabidopsis thaliana genomic DNA, chromosome 5, P1 clone: MPO12, complete sequence.//0.00097:721:58//AB006702
F-HEMBB1001653//Homo sapiens chromosome 2 clone 101B6 map 2p11, complete sequence.//0.15:276:63//AC002038
F-HEMBB1001665//Bovine herpesvirus type 1 early-intermediate transcription control protein (BICP4) gene, complete cds.//0.43:393:61//L14320
F-HEMBB1001668//F16C15-T7 IGF Arabidopsis thaliana genomic clone F16C15, genomic survey sequence.//0.040:275:60//B12308
F-HEMBB1001673//Homo sapiens mRNA for KIAA0646 protein, complete cds.//7.2e-171:803:98//AB014546
F-HEMBB1001684//Sequence 1 from patent US 5700927.//7.5e-124:883:81//I86429
F-HEMBB1001685//CIT-HSP-2287O9.TF CIT-HSP Homo sapiens genomic clone 2287O9, genomic survey sequence.//2.3e-34:191:97//B99261
F-HEMBB1001695//Human DNA sequence from clone 431P23 on chromosome 6q27. Contains the first coding exon of the MLLT4 gene for myeloid/lymphoid or mixed-lineage leukemia (trithorax (Drosophila) homolog); translocated to, 4 (AF-6, Afadin, MLLT-4, ALL-1 fusion partner), and a Serine Palmitoyltransferase 2 (EC 2.3.1.50, Long Chain Base Biosynthesis protein 2, LCB-2, SPT-2) pseudogene. Contains ESTs, STss, GSSs, and a putative CpG island, complete sequence.//0.0091:334:63//AL009178
F-HEMBB1001704//Human DNA sequence from clone 931E15 on chromosome Xq25. Contains STSs, GSSs and genomic marker DXS8098, complete sequence.//1.2e-17:144:87//AL023575
F-HEMBB1001706
F-HEMBB1001707//Guinea pig CD19 mRNA, complete cds.//0.57:232:62//M62543
F-HEMBB1001717//Saccharomyces cerevisiae mitochondrial tRNA-Tyr, tRNA-Asn, & amp; tRNA-Met genes.//1.1e-13:723:58//AJ223323
F-HEMBB1001735//Human PAC clone DJ0596O09 from 7p15, complete sequence.//1.3e-36:427:73//AC003074
F-HEMBB1001736//S.pombe chromosome II cosmid c4B4.//0.0085:479:57//AL023706
F-HEMBB1001747//Homo sapiens PAC clone DJ1002N02 from 7p21-p22, complete sequence.//4.0e-112:532:84//AC005376
F-HEMBB1001749//Homo sapiens chromosome 17, clone hRPK.259_G_18, complete sequence.//1.3e-98:395:82//AC005829
F-HEMBB1001753//S.maximus repeat region, 342bp.//4.2e-11:69:85//Z78099
F-HEMBB1001756//Homo sapiens full-length insert cDNA clone ZD86A11.//0.0015:302:62//AF088064
F-HEMBB1001760//P.falciparum complete gene map of plastid-like DNA (IR-A).//0.011:615:56//X95275
F-HEMBB1001762//CIT-HSP-2290J16.TF CIT-HSP Homo sapiens genomic clone 2290J16, genomic survey sequence.//0.84:208:64//AQ005184
F-HEMBB1001785//Plasmodium falciparum DNA *** SEQUENCING IN PROGRESS *** from MAL1P3, WORKING DRAFT SEQUENCE.//0.0019:469:60//AL031746
F-HEMBB1001797//Human heterogenous nuclear RNA W16W.//0.00012:83:86//X17272
F-HEMBB1001802//Plasmodium falciparum MAL3P7, complete sequence.//1.8e-11:538:60//AL034559
F-HEMBB1001812//Human DNA sequence *** SEQUENCING IN PROGRESS *** from clone 356B8, WORKING DRAFT SEQUENCE.//1.0e-56:304:84//Z98882
F-HEMBB1001816//Homo sapiens chromosome 19, cosmid F24083, complete sequence.//3.6e-75:300:87//AC005204
F-HEMBB1001831//Homo sapiens PAM COOH-terminal interactor protein 1 (PCIP1) mRNA, complete cds.//2.3e-162:763:98//AF056209
F-HEMBB1001834//CIT-HSP-2291012.TF CIT-HSP Homo sapiens genomic clone 2291O12, genomic survey sequence.//7.6e-08:73:94//AQ004168
F-HEMBB1001836//Homo sapiens 12q13.1 PAC RPCI1-228P16 (Roswell Park Cancer Institute Human PAC Library) complete sequence.//5.7e-30:297:79//AC004801
F-HEMBB1001839//Human Chromosome X, complete sequence.//0.016:293:63//AC004073
F-HEMBB1001850//Plasmodium falciparum 3D7 chromosome 12 PFYAC1383 genomic sequence, WORKING DRAFT SEQUENCE, 3 unordered pieces.//0.0027:812:58//AC005504
F-HEMBB1001863//Human Chromosome 15q26.1 PAC clone pDJ460g16, WORKING DRAFT SEQUENCE, 3 unordered pieces.//8.3e-43:520:72//AC004581
F-HEMBB1001867//Human proto-oncogene tyrosine-protein kinase (ABL) gene, exon 1a and exons 2-10, complete cds.//1.7e-56:399:86//U07563
F-HEMBB1001868//Rattus norvegicus clone 923 polymeric immunoglobulin receptor mRNA 3' untranslated region, GA rich region, and microsatellites with GGA-triplet and GAA-triplet repeats.//6.1e-08:234:67//U01145
F-HEMBB1001869//Homo sapiens full-length insert cDNA clone YT86F01.//7.4e-87:432:97//AF085974
F-HEMBB1001872
F-HEMBB1001874//Homo sapiens clone DJ241P17, WORKING DRAFT SEQUENCE, 7 unordered pieces.//3.4e-14:631:61//AC005000
F-HEMBB1001875//Human DNA sequence from clone J428A131, WORKING DRAFT SEQUENCE.//0.93:415:57//Z82209
F-HEMBB1001880//Human genomic DNA sequence from clone 308O1 on chromosome Xp11.3-11.4. Contains EST, CA repeat, STS, GSS, CpG island.//1.0e-18:729:60//Z93403
F-HEMBB1001899//Plasmodium falciparum DNA *** SEQUENCING IN PROGRESS *** from contig 4-10, complete sequence.//0.0038:425:58//AL010216
F-HEMBB1001905//S.pombe chromosome III cosmid c330.//1.1e-23:520:62//AL031603
F-HEMBB1001906
F-HEMBB1001908//Human monocytic leukaemia zinc finger protein (MOZ) mRNA, complete cds.//3.7e-82:672:81//U47742
F-HEMBB1001910//Plasmodium falciparum 3D7 chromosome 12 PFYAC181 genomic sequence, WORKING DRAFT SEQUENCE, 8 unordered pieces.//0.0033:566:55//AC005505
F-HEMBB1001911//Arabidopsis thaliana chromosome II BAC F26C24 genomic sequence, complete sequence.//1.0:581:58//AC004705
F-HEMBB1001915//Caenorhabditis elegans cosmid T05H10, complete sequence.//1.2e-16:283:67//Z47812
F-HEMBB1001921//Homo sapiens chromosome 17, clone hCIT.123_J_14, complete sequence.//3.4e-07:803:58//AC003950
F-HEMBB1001922//Plasmodium falciparum chromosome 2, section 28 of 73 of the complete sequence.//5.0e-06:756:56///AE001391
F-HEMBB1001925//Human DNA sequence from PAC 212P9 on chromosome 1p34.1-1p35. Contains delta opiate receptor, CpG island, CA repeat,.//3.1e-45:609:73//AL009181
F-HEMBB1001930//Homo sapiens genomic DNA of 8p21.3-p22 anti-oncogene of hepatocellular colorectal and non-small cell lung cancer , segment 10/11.//3.2e-158:745:99//AB020867
F-HEMBB1001944//, complete sequence.//4.1e-60:638:73//AC005815
F-HEMBB1001945//HS_3185_B1_G05_MR CIT Approved Human Genomic Sperm Library D Homo sapiens genomic clone Plate=3185 Col=9 Row=N, genomic survey sequence.//1.0:280:58//AQ188882
F-HEMBB1001947//Human mRNA for KIAA0392 gene, partial cds.//5.6e-20:333:66//AB002390
F-HEMBB1001950//Human lipocortin (LIP) 2 gene, upstream region.//0.0094:180:63//M62899
F-HEMBB1001952//Human DNA sequence *** SEQUENCING IN PROGRESS *** from clone 101A4, WORKING DRAFT SEQUENCE.//5.4e-19:329:70//Z93341
F-HEMBB1001953//Homo sapiens chromosome 17, clone hRPK.795_F_17, complete sequence.//0.11:589:58//AC005284
F-HEMBB1001957//Human DNA sequence from PAC 204E5 on chromosome 12. Contains exon similar to Wilms' Tumour-related protein QM-like P2X-like receptor, ATP ligand gated ion channel, ESTs, CpG island.//9.8e-25:446:67//Z98941
F-HEMBB1001962//Homo sapiens chromosome 16, BAC clone 462G18 (LANL), complete sequence.//2.8e-147:727:97//AC005736
F-HEMBB1001967//Homo sapiens clone DJ1102A12, WORKING DRAFT SEQUENCE, 15 unordered pieces.//3.2e-56:650:71//AC004963
F-HEMBB1001973//Homo sapiens chromosome 12p13.3-clone RPCI11-350L7, WORKING DRAFT SEQUENCE, 72 unordered pieces.//1.2e-42:327:84//AC005844
F-HEMBB1001983//CIT-HSP-2315M4.TF CIT-HSP Homo sapiens genomic clone 2315M4, genomic survey sequence.//8.8e-35:198:96//AQ028071
F-HEMBB1001988//D.polychroa microsatellite sequence (clone Dp 1C e12).//4.5e-07:337:62//X92189
F-HEMBB1001990//HS_3234_A1_G08_T7 CIT Approved Human Genomic Sperm Library D Homo sapiens genomic clone Plate=3234 Col=15 Row=M, genomic survey sequence.//0.039:279:59//AQ204689
F-HEMBB1001996//Human DNA sequence *** SEQUENCING IN PROGRESS *** from clone 191J18, WORKING DRAFT SEQUENCE.//0.18:392:58//AL024507
F-HEMBB1001997//Homo sapiens clone RG140B11, WORKING DRAFT SEQUENCE, 1 unordered pieces.//1.3e-43:446:71//AC005069
F-HEMBB1002002//Plasmodium falciparum 3D7 chromosome 12 PFYAC812 genomic sequence, WORKING DRAFT SEQUENCE, 8 unordered pieces.//0.077:444:58//AC004153
F-HEMBB1002005//Human DNA sequence *** SEQUENCING IN PROGRESS *** from clone 963K23, WORKING DRAFT SEQUENCE.//3.4e-16:173:78//AL031685
F-HEMBB1002009//Plasmodium falciparum 3D7 chromosome 12 PFYAC357 genomic sequence, WORKING DRAFT SEQUENCE, 7 unordered pieces.//0.00033:790:56//AC005506
F-HEMBB1002015//Homo sapiens genomic DNA, chromosome 21q11.1, segment 27/28, WORKING DRAFT SEQUENCE.//6.7e-05 :126:76//AP000056
F-HEMBB1002042//Oncorhynchus mykiss cytochrome P450 (CYP4V1) mRNA, partial cds.//6.4e-33:402:69//AF046012
F-HEMBB1002043
F-HEMBB1002044//Homo sapiens chromosome 5p, BAC clone 50g21 (LBNL H154), complete sequence.//3.0e-167:809:97//AC005740
F-HEMBB1002045
F-HEMBB1002049//Homo sapiens chromosome 17, clone hRPC.161_P_9, complete sequence.//0.87:177:65//AC006237
F-HEMBB1002050//Streptomyces coelicolor cosmid D78.//8.5e-08:644:58//AL034355
F-HEMBB1002068//Homo sapiens mRNA for KIAA0612 protein, partial cds.//2.5e-05:402:61//AB014512
F-HEMBB1002069
F-HEMBB1002092//*** SEQUENCING IN PROGRESS *** Homo sapiens chromosome 4, BAC clone B331O8; HTGS phase 1, WORKING DRAFT SEQUENCE, 10 unordered pieces.//7.8e-104:550:83//AC004064
F-HEMBB1002094//Homo sapiens genomic DNA, 21q region, clone: 125H6N2, genomic survey sequence.//2.9e-49:302:83//AG001476
F-HEMBB1002115//Homo sapiens chromosome 16, cosmid clone 378E2 (LANL), complete sequence.//0.00023:542:61//AC004035
F-HEMBB1002134//Human h-neuro-d4 protein mRNA, complete cds.//7.3e-43:533:70//U43843
F-HEMBB1002139//HS-1048-A2-B02-MR.abi CIT Human Genomic Sperm Library C Homo sapiens genomic clone Plate=CT 831 Col=4 Row=C, genomic survey sequence.//0.055:228:66//B38714
F-HEMBB1002142//Plasmodium falciparum DNA *** SEQUENCING IN PROGRESS *** from MAL1P5, WORKING DRAFT SEQUENCE.//0.0095:276:64//AL031748
F-HEMBB1002152//Human Chromosome X, WORKING DRAFT SEQUENCE, 4 unordered pieces.//0.055:520:57//AC002421
F-HEMBB1002189//Homo sapiens cosmid ICRFc104I0935Q8 from Xq28, complete sequence.//2.6e-05:311:63//AF002998
F-HEMBB1002190//Plasmodium falciparum 3D7 chromosome 12 PFYACB8-420 genomic sequence, WORKING DRAFT SEQUENCE, 14 unordered pieces.//5.4e-05:647:59//AC005140
F-HEMBB1002193//Sequence 5 from patent US 5709858.//1.8e-34:179:100//I80846
F-HEMBB1002217//Homo sapiens mRNA for zinc finger protein 10.//1.2e-23:405:67//X52332
F-HEMBB1002218//HS_2056_B1_C09_T7 CIT Approved Human Genomic Sperm Library D Homo sapiens genomic clone Plate=2056 Col=17 Row=F, genomic survey sequence.//3.3e-45:245:97//AQ244711
F-HEMBB1002232//Human chromosome 11 72g7 cosmid, complete sequence.//1.9e-21:314:70//U73648
F-HEMBB1002247
F-HEMBB1002249//Homo sapiens DNA sequence from BAC 34I8 on chromosome 6p21.3-22.1. Contains ZNF184 gene coding for Kruppel related Zinc Finger protein 184, a hnRNP core protein A1 (mouse Fli-2, rat helix destabilizing protein, mouse Topoisomerase-inhibitor suppressed gene TIS) LIKE pseudogene, a HB15 (CD83 antigen precursor) LIKE pseudogene, Ser-tRNA, Glu-tRNA and Met-tRNA (Met-tRNA-i gene 1) genes. Contains ESTs, STSs and GSSs, complete sequence.//4.1e-45:327:83//AL021918
F-HEMBB1002254//Human chromosome 16 BAC clone LANL cosmid-440E5, WORKING DRAFT SEQUENCE, 2 unordered pieces.//9.8e-40:315:82//AC002506
F-HEMBB1002255//Plasmodium falciparum MAL3P3, complete sequence.//0.0035:312:62//Z98547
F-HEMBB1002266//Plasmodium falciparum 3D7 chromosome 12 PFYAC1383 genomic sequence, WORKING DRAFT SEQUENCE, 3 unordered pieces.//0.013:469:59//AC005504
F-HEMBB1002280//Homo sapiens Chromosome 16 BAC clone CIT987SK-A-259H10, complete sequence.//5.3e-18:527:61//AC004682
F-HEMBB1002300//Human Chromosome 11 Cosmid cSRL30h11, complete sequence.//8.6e-139:818:88//U73642
F-HEMBB1002306//HS_3109_A2_H01_MR CIT Approved Human Genomic Sperm Library D Homo sapiens genomic clone Plate=3109 Col=2 Row=O, genomic survey sequence.//1.3e-75:371:98//AQ148164
F-HEMBB1002327//HS_3235_B2_G10_T7 CIT Approved Human Genomic Sperm Library D Homo sapiens genomic clone Plate=3235 Col=20 Row=N, genomic survey sequence.//3.3e-83:418:97//AQ209752
F-HEMBB1002329//CITBI-E1-2503J7.TR CITBI-E1 Homo sapiens genomic clone 2503J7, genomic survey sequence.//3.3e-31:220:88//AQ263402
F-HEMBB1002340
F-HEMBB1002342//Homo sapiens mRNA for putative thioredoxin-like protein.//4.1e-154:724:98//AJ010841
F-HEMBB1002358//Human thymidylate kinase (CDC8) mRNA, complete cds.//3.3e-36:192:98//L16991
F-HEMBB1002359//Human Rev interacting protein Rip-1 mRNA, complete cds.//1.8e-13:96:96//U55766
F-HEMBB1002364//Human DNA sequence *** SEQUENCING IN PROGRESS *** from clone 376D21, WORKING DRAFT SEQUENCE.//7.5e-24:202:71//Z98946
F-HEMBB1002371//Plasmodium falciparum 3D7 chromosome 12 PFYAC812 genomic sequence, WORKING DRAFT SEQUENCE, 8 unordered pieces.//1.9e-06:674:56//AC004153
F-HEMBB1002381//Homo sapiens chromosome 16, cosmid clone RT163 (LANL), complete sequence.//0.34:238:61//AC005222
F-HEMBB1002383
F-HEMBB1002387//CIT-HSP-2173E20.TR CIT-HSP Homo sapiens genomic clone 2173E20, genomic survey sequence.//5.2e-17:434:66//B91052
F-HEMBB1002409//Human DNA sequence from PAC 84F12 on chromosome Xq25-Xq26.3. Contains glypican-3 precursor (intestinal protein OCI-5) (GTR2-2), ESTs and CA repeat.//1.2e-56:324:88//AL008712
F-HEMBB1002415//Human DNA sequence *** SEQUENCING IN PROGRESS *** from clone 364I1, WORKING DRAFT SEQUENCE.//8.9e-35:334:75//AL031319
F-HEMBB1002425//Chromosome 22q13 BAC Clone CIT987SK-384D8 complete sequence.//1.0e-36:317:76//U62317
F-HEMBB1002442//Rattus norvegicus lin-10 protein homolog (lin-10) mRNA, complete cds.//4.3e-88:296:92//U92010
F-HEMBB1002453//Human DNA sequence *** SEQUENCING IN PROGRESS *** from clone 86D1, WORKING DRAFT SEQUENCE.//2.7e-43:419:78//AL034349
F-HEMBB1002457//Homo sapiens clone DJ0982E09, WORKING DRAFT SEQUENCE, 3 unordered pieces.//1.3e-27:542:68//AC005534
F-HEMBB1002458//HS_3246_A2_G05_MR CIT Approved Human Genomic Sperm Library D Homo sapiens genomic clone Plate=3246 Col=10 Row=M, genomic survey sequence.//3.2e-51:257:99//AQ217993
F-HEMBB1002477//Human Grb2-associated binder-1 mRNA, complete cds.//1.9e-87:493:92//U43885
F-HEMBB1002489
F-HEMBB1002492//Arabidopsis thaliana BAC T15B16.//0.028:516:57//AF104919
F-HEMBB1002495//Homo sapiens chromosome 17, clone hRPK.421_E_14, complete sequence.//1.1e-16:297:68//AC006141
F-HEMBB1002502//Homo sapiens clone DJ1163L11, complete sequence.//1.1e-91:675:82//AC005230
F-HEMBB1002509//Homo sapiens Chromosome 16 BAC clone CIT987SK-A-248F7, complete sequence.//2.7e-11:648:60//AC004605
F-HEMBB1002510//HS_3236_B1_H11_MR CIT Approved Human Genomic Sperm Library D Homo sapiens genomic clone Plate=3236 Col=21 Row=P, genomic survey sequence.//1.2e-06:67:94//AQ205992
F-HEMBB1002520//Homo sapiens BAC clone NH0004N07 from Y, complete sequence.//1.2e-70:580:72//AC006152
F-HEMBB1002522//Homo sapiens Xp22 bin 150 clone GSHB-223P11 (Genome Systems Human BAC library) complete sequence.//5.6e-22:516:64//AC004553 F-HEMBB1002531
F-HEMBB1002534//Human DNA sequence *** SEQUENCING IN PROGRESS *** from clone 668J24, WORKING DRAFT SEQUENCE.//6.9e-62:265:87//AL034346
F-HEMBB1002545//Human BAC clone RG128M16 from 7q21-7q22, complete sequence.//2.7e-44:200:82//AC000059
F-HEMBB1002550//Homo sapiens PAC clone DJ0910I17 from 7q11.21-q11.23, complete sequence.//0.22:161:68//AC004927
F-HEMBB1002556//Homo sapiens PAC clone DJ0696N01 from 7p21-p22, complete sequence.//7.5e-43:306:77//AC004861
F-HEMBB1002579
F-HEMBB1002582//Human DNA sequence *** SEQUENCING IN PROGRESS *** from clone 349A12, WORKING DRAFT SEQUENCE.//0.00018:431:61//AL033520
F-HEMBB1002590//Yeast (S.cerevisiae) mitochondrial apocytochrome b gene, 3' flank.//0.78:147:64//J01471
F-HEMBB1002596//Human DNA sequence *** SEQUENCING IN PROGRESS *** from clone 9E21, WORKING DRAFT SEQUENCE.//3.6e-50:692:69//AL008639
F-HEMBB1002600//Homo sapiens tetraspan NET-5 mRNA, complete cds.//9.1e-151:710:98//AF089749
F-HEMBB1002601//Human BAC clone RG020D02 from 7q22, complete sequence.//1.5e-07:416:60//AC002381
F-HEMBB1002603//Human BAC clone GS552A01 from 7q21-q22, complete sequence.//0.40:341:60//AC002454
F-HEMBB1002607//Mus musculus homeobox containing nuclear transcriptional factor Hmx1 (Hmx1) gene, complete cds.//0.0042:460:60//AF009614
F-HEMBB1002610//Homo sapiens Chromosome 12q24 PAC RPCI3-462E2 (Roswell Park Cancer Institute Human PAC library) complete sequence.//6.3e-23:559:63//AC003029
F-HEMBB1002613//Homo sapiens Chromosome 22q12 BAC Clone 566c1, complete sequence.//4.2e-17:441:63//AC000025
F-HEMBB1002614//Plasmodium falciparum chromosome 2, section 54 of 73 of the complete sequence.//0.013:324:56//AE001417
F-HEMBB1002617//Homo sapiens chromosome 16 BAC clone CIT987SK-334D11 complete sequence.//2.1e-07:441:60//AF001550
F-HEMBB1002623//C.hyalina microsatellite marker DNA (id ATCC4).//0.57:106:66//Z95304
F-HEMBB1002635//Human JNK3 alpha2 protein kinase (JNK3A2) mRNA, complete cds.//4.8e-22:127:100//U34819
F-HEMBB1002664//HS_2265_A1_H06_MF CIT Approved Human Genomic Sperm Library D Homo sapiens genomic clone Plate=2265 Col=11 Row=O, genomic survey sequence.//0.54:115:67//AQ101557
F-HEMBB1002677//Homo sapiens (subclone 3_d1 from P1 H25) DNA sequence, complete sequence.//2.2e-49:784:68//L81774
F-HEMBB1002683//Homo sapiens type IV collagen 5a chain (COL4A5) gene, exon 23.//1.0:112:63//U04492
F-HEMBB1002684//HS-1050-A2-G06-MF.abi CIT Human Genomic Sperm Library C Homo sapiens genomic clone Plate=CT 772 Col=12 Row=M, genomic survey sequence.//4.4e-07:86:84//B39748
F-HEMBB1002686//HS-1023-B2-F10-MR.abi CIT Human Genomic Sperm Library C Homo sapiens genomic clone Plate=CT 802 Col=20 Row=L, genomic survey sequence.//0.98:183:61//B34077
F-HEMBB1002692//Human DNA sequence *** SEQUENCING IN PROGRESS *** from clone 1108H3, WORKING DRAFT SEQUENCE.//0.00039:408:60//AL033525
F-HEMBB1002697//Homo sapiens clone DJ1087M19, WORKING DRAFT SEQUENCE, 7 unordered pieces.//7.3e-35:323:74//AC004955
F-HEMBB1002699//Mus musculus D6MM5e protein (D6Mm5e) and DOK protein (Dok) genes, complete cds; and LOR2 protein (Lor2) gene, partial cds.//0.031:325:62//AF084363
F-HEMBB1002702//HS-1025-A2-D01-MR.abi CIT Human Genomic Sperm Library C Homo sapiens genomic clone Plate=CT 804 Col=2 Row=G, genomic survey sequence.//1.8e-25:158:95//B34720
F-HEMBB1002705//Homo sapiens DNA, chromosome 21q22.2, PAC clone 25P16 complete sequence, encoding carbonyl reductase and carbonyl reductase 3 (complete cds).//1.7e-137:534:96//AB003151
F-HEMBB1002712//Human DNA sequence from cosmid cU115G11, between markers DXS6791 and DXS8038 on chromosome X contains ESTs and STS.//0.0019:612:58//Z71187
F-MAMMA1000009//Human chromosome 1 BAC 308G1 genomic sequence, WORKING DRAFT SEQUENCE, 3 unordered pieces.//6.1e-43:354:81//AC003117
F-MAMMA1000019
F-MAMMA1000020//H.sapiens mRNA for flavin-containing monooxygenase 5 (FMO5).//2.0e-40:185:97//Z47553
F-MAMMA1000025//Homo sapiens PAC clone DJ0806A17 from 7p13-p14, complete sequence.//1.0:211:65//AC005483
F-MAMMA1000043//Human angiotensin I-converting enzyme (ACE) gene, intron 12.//0.075:204:65//M73275
F-MAMMA1000045//Human DNA sequence from clone 142F18 on chromosome Xq26.3-27.2 Contains part of a gene similar to melanoma-associated antigen, EST, GSS and an inverted repeat, complete sequence.//4.1e-122:495:79//AL031073
F-MAMMA1000055//M.musculus mRNA for testin.//2.1e-35:559:66//X78989
F-MAMMA1000057//Homo sapiens chromosome 17, clone hRPK.259_G_18, complete sequence.//5.5e-121:703:89//AC005829
F-MAMMA1000069//Homo sapiens minisatellite ceb1 repeat region.//0.00013:329:60//AF048727
F-MAMMA1000084//Homo sapiens DNA sequence from PAC 958B3 on chromosome Xp22.11-Xp22.22. Contains ESTs STS and CpG island.//2.1e-53:445:79//Z93023
F-MAMMA1000085//Caenorhabditis elegans cosmid Y23H5A.//0.0017:164:64//AF077541
F-MAMMA1000092//Homo sapiens BAC clone GS465N13 from 7p15-p21, complete sequence.//1.2e-70:598:78//AC004744
F-MAMMA1000103//Homo sapiens chromosome 17, clone hCIT.91_J_4, complete sequence.//1.1e-156:857:92//AC003976
F-MAMMA1000117//HS_3223_B2_D08_T7 CIT Approved Human Genomic Sperm Library D Homo sapiens genomic clone Plate=3223 Col=16 Row=H, genomic survey sequence.//5.4e-100:527:94//AQ221160
F-MAMMA1000129//ryanodine receptor.//0.055 :492:59//A20359
F-MAMMA1000133
F-MAMMA1000134//HS_3078_B1_C02_MF CIT Approved Human Genomic Sperm Library D Homo sapiens genomic clone Plate=3078 Col=3 Row=F, genomic survey sequence.//2.1e-93:462:97//AQ144362
F-MAMMA1000139//Homo sapiens Xp22 PAC RPCI1-5G11 (from Roswell Park Cancer Center) complete sequence.//3.3e-14:322:65//AC002369
F-MAMMA1000143//Homo sapiens mRNA for KIAA0685 protein, complete cds.//6.9e-25:148:97//AB014585
F-MAMMA1000155//Homo sapiens homeobox transcription factor barx2 (BARX2) mRNA, complete cds.//1.0e-29:219:87//AF031924
F-MAMMA1000163
F-MAMMA1000171//Homo sapiens chromosome 19, CIT-HSP BAC 470n8, complete sequence.//6.3e-14:92:88//AC005393
F-MAMMA1000173//Mus musculus SH3-containing protein SH3P7 mRNA, complete cds. similar to Human Drebrin.//2.2e-114:698:87//U58884
F-MAMMA1000175//HS_3050_B1_B03_MF CIT Approved Human Genomic Sperm Library D Homo sapiens genomic clone Plate=3050 Col=5 Row=D, genomic survey sequence.//6.2e-73:357:99//AQ102678
F-MAMMA1000183//Human DNA sequence *** SEQUENCING IN PROGRESS *** from clone Y313F4, WORKING DRAFT SEQUENCE.//4.6e-94:904:73//AL023808
F-MAMMA1000198//Z.diploperennis repetitive DNA (clone ZEAR 266).//0.18:152:70//X53610
F-MAMMA1000221//Human Chromosome 15q11-q13 PAC clone pDJ778a2, complete sequence.//0.017:99:75//AC004583
F-MAMMA1000227//Human DNA sequence *** SEQUENCING IN PROGRESS *** from clone 467K16, WORKING DRAFT SEQUENCE.//0.36:312:62//AL031283
F-MAMMA1000241//HS_3217_B1_B02_MR CIT Approved Human Genomic Sperm Library D Homo sapiens genomic clone Plate=3217 Col=3 Row=D, genomic survey sequence.//1.9e-94:456:98//AQ193401
F-MAMMA1000251//Homo sapiens NF2 gene.//0.00092:270:64//Y18000
F-MAMMA1000254//Plasmodium falciparum 3D7 chromosome 12 PFYACB8-420 genomic sequence, WORKING DRAFT SEQUENCE, 14 unordered pieces.//0.0034:777:57//AC005140
F-MAMMA1000257//Homo sapiens DNA sequence from PAC 201D7 on chromosome 6p22.1-22.3. Contains EST and STS.//0.00036:230:65//AL022717
F-MAMMA1000264//Homo sapiens (subclone 9_f5 from P1 H17) DNA sequence, complete sequence.//1.5e-30:499:68//L81612
F-MAMMA1000266//Bacillus lynceorum strain pMEL12 Bag320 satellite DNA.//0.28:218:64//AF034430
F-MAMMA1000270//Human Chromosome 16 BAC clone CIT987SK-A-270G1, complete sequence.//1 .4e-157:788:96//AF001549
F-MAMMA1000277//Mycobacterium tuberculosis H37Rv complete genome; segment 48/162.//0.70:320:61//AL021897
F-MAMMA1000278//Sequence 23 from patent US 5708157.//9.3e-103:540:95//I80055
F-MAMMA1000279//Human DNA sequence from clone 769D20 on chromosome Xp21.1-21.3 Contains EST, STS, GSS, complete sequence.//2.4e-49:262:77//AL031643
F-MAMMA1000284//cSRL-165E12-u cSRL flow sorted Chromosome 11 specific cosmid Homo sapiens genomic clone cSRL-165E12, genomic survey sequence.//1.1e-30:324:75//B03004
F-MAMMA1000287//Homo sapiens, clone hRPK.15_A_1, complete sequence.//2.7e-54:401:83//AC006213
F-MAMMA1000302//Drosophila melanogaster complete mitochondrial genome.//0.0051:307:61//U37541
F-MAMMA1000307//Homo sapiens chromosome 12p13.3 clone RPCI5-1154L15, WORKING DRAFT SEQUENCE, 67 unordered pieces.//0.15:449:59//AC006205
F-MAMMA1000309//cDNA coding human apolipoprotein E3.//0.00010:691:58//E00359
F-MAMMA1000312//Human DNA sequence *** SEQUENCING IN PROGRESS *** from clone 798A17, WORKING DRAFT SEQUENCE.//0.27:301:60//AL031274
F-MAMMA1000313
F-MAMMA1000331//Human Chromosome 16 BAC clone CIT987SK-A-735G6, complete sequence.//9.8e-06:151:71//AC002400
F-MAMMA1000339
F-MAMMA1000340//HS_2181_B2_F07_MR CIT Approved Human Genomic Sperm Library D Homo sapiens genomic clone Plate=2181 Col=14 Row=L, genomic survey sequence.//4.3e-05:181:68//AQ024288
F-MAMMA1000348//Homo sapiens chromosome 17, clone HRPC843B9, complete sequence.//5.3e-30:575:66//AC004139
F-MAMMA1000356//Homo sapiens clone RG038K21, WORKING DRAFT SEQUENCE, 3 unordered pieces.//1.8e-52:264:76//AC005052
F-MAMMA1000360//Homo sapiens PAC clone DJ0755G17 from 7p21-p22, complete sequence.//6.5e-91:569:88//AC004879
F-MAMMA1000361//Human DNA sequence from PAC 507I15 on chromosome Xq26.3-27.3. Contains 60S ribosomal protein L44 (L41, L36) like gene, ESTs, STSs and a polymorphic CA repeat.//1.4e-42:315:83//Z98950
F-MAMMA1000372//Human DNA sequence *** SEQUENCING IN PROGRESS *** from clone Y738F9, WORKING DRAFT SEQUENCE.//2.9e-114:516:89//AL022345
F-MAMMA1000385//CITBI-E1-2517E13.TF CITBI-E1 Homo sapiens genomic clone 2517E13, genomic survey sequence.//6.9e-26:377:71//AQ279944
F-MAMMA1000388//Homo sapiens UKLF mRNA for ubiquitous Kruppel like factor, complete cds.//3.7e-148:710:98//AB015132
F-MAMMA1000395
F-MAMMA1000402//Homo sapiens clone DJ0718N17, complete sequence.//4.0e-115:845:85//AC005999
F-MAMMA1000410//HS_3245_A1_C02_MR CIT Approved Human Genomic Sperm Library D Homo sapiens genomic clone Plate=3245 Col=3 Row=E, genomic survey sequence.//9.6e-42:350:80//AQ205768
F-MAMMA1000413//HS_3223_B2_F01_MR CIT Approved Human Genomic Sperm Library D Homo sapiens genomic clone Plate=3223 Col=2 Row=L, genomic survey sequence.//1.6e-48:318:89//AQ188456
F-MAMMA1000414//HS_2027_B2_C04_MR CIT Approved Human Genomic Sperm Library D Homo sapiens genomic clone Plate=2027 Col=8 Row=F, genomic survey sequence.//1.4e-46:286:92//AQ231369
F-MAMMA1000416//Drosophila melanogaster DNA sequence (P1s DS07528 (D169) and DS06665 (D220)), complete sequence.//9.4e-33:310:72//AC004640
F-MAMMA1000421//Homo sapiens clone DJ1129D05, complete sequence.//3.3e-29:223:84//AC005630
F-MAMMA1000422
F-MAMMA1000423//Drosophila yakuba mitochondrial DNA molecule.//2.2e-10:639:57//X03240
F-MAMMA1000424//Homo sapiens DNA from chromosome 19, BAC 33152, complete sequence.//4.6e-47:556:68//AC003973
F-MAMMA1000429//Mus musculus SDP8 mRNA, complete cds.//8.0e-99:545:92//AF062484
F-MAMMA1000431//Homo sapiens clone DJ1039L24, WORKING DRAFT SEQUENCE, 3 unordered pieces.//4.8e-41:289:79//AC005283
F-MAMMA1000444//Human DNA sequence from clone 714B7 on chromosome 22q12.2-13.2 Contains CYTOCHROME C OXIDASE VIIB precursor like pseudogene and ESTs, complete sequence.//2.3e-34:291:80//Z99755
F-MAMMA1000446
F-MAMMA1000458//Mus musculus clone OST9003, genomic survey sequence.//5.0e-53:231:84//AF046620
F-MAMMA1000468//Human DNA sequence *** SEQUENCING IN PROGRESS *** from clone 291J10, WORKING DRAFT SEQUENCE.//0.75:303:60//Z93017
F-MAMMA1000472//Human DNA sequence *** SEQUENCING IN PROGRESS *** from clone 414D7, WORKING DRAFT SEQUENCE.//4.0e-41:403:77//AL033543
F-MAMMA1000478//Homo sapiens clone RG270D13, WORKING DRAFT SEQUENCE, 18 unordered pieces.//9.5e-54:369:77//AC005081
F-MAMMA1000483//Homo sapiens Chromosome 16 BAC clone CIT987SK-44M2, complete sequence.//3.6e-34:332:77//AC004381
F-MAMMA1000490//Homo sapiens 12q13.1 PAC RPCI1-90J4 (Roswell Park Cancer Institute Human PAC library) complete sequence.//8.9e-128:822:87//AC003686
F-MAMMA1000500//CIT-HSP-231905.TF CIT-HSP Homo sapiens genomic clone 2319O5, genomic survey sequence.//4.8e-29:175:94//AQ044812
F-MAMMA1000501//Homo sapiens DNA sequence from clone 78F24 on chromosome 22q12.1-12.3. Contains one exon of an Oxysterol-binding protein (OSBP) LIKE gene. Contains GSSs and an STS, complete sequence.//5.7e-45:334:82//AL022336
F-MAMMA1000516//Human DNA sequence from PAC 50A13 on chromosome Xp11. Contains ATP SYNTHASE LIPID BINDING PROTEIN P1 (P2, P3) precursor (ATPSG1, ATP5G2, ATP5G3) like pseudogene, ESTs and STSs. Contains polymorphic CA repeat.//2.9e-43:529:69//Z92545
F-MAMMA1000522//Human DNA sequence from clone 20J23 on chromosome Xq26.2-27.2 Contains ras-related C3 botulinum toxin substrate 1 (P21-RAC1) (ras-like protein TC25) EST, CA repeat, STS, CpG island, complete sequence.//2.0e-14:380:63//AL022576
F-MAMMA1000524//Homo sapiens chromosome 10 clone CIT-HSP-1338F24 map 10p11.2-10p12.1, complete sequence.//1.4e-22:420:66//AC006101
F-MAMMA1000559//Human HepG2 3' region cDNA, clone hmd3f08.//5.4e-29:168:97//D16922
F-MAMMA1000565//RPCI11-61K6.TJ RPCI11 Homo sapiens genomic clone R-61K6, genomic survey sequence.//1.7e-120:561:100//AQ194238
F-MAMMA1000567//Human DNA sequence from PAC 179D3, between markers DXS6791 and DXS8038 on chromosome X contains S10 GTP-binding protein, ESTs and CpG island.//3.1e-43:387:80//Z81370
F-MAMMA1000576//Homo sapiens BAC clone RG442F18 from 2, complete sequence.//1.2e-30:237:75//AC005104
F-MAMMA1000583//RPCI11-60M22.TJ RPCI11 Homo sapiens genomic clone R-60M22, genomic survey sequence.//9.6e-102:487:99//AQ198091
F-MAMMA1000585//Homo sapiens clone UWGC:djs14 from 7p14-15, complete sequence.//5.2e-39:370:78//AC006195
F-MAMMA1000594//Homo sapiens chromosome 19, cosmid R31646, complete sequence.//3.9e-43:328:83//AC005338
F-MAMMA1000597//Homo sapiens chromosome 17, clone hRPK.481_C_4, complete sequence.//1.5e-32:259:82//AC005839
F-MAMMA1000605//Human DNA sequence *** SEQUENCING IN PROGRESS *** from clone 97P20, WORKING DRAFT SEQUENCE.//2.4e-59:318:83//AL031297
F-MAMMA1000612//HS_2188_A2_D02_MF CIT Approved Human Genomic Sperm Library D Homo sapiens genomic clone Plate=2188 Col=4 Row=G, genomic survey sequence.//4.8e-30:171:96//AQ116793
F-MAMMA1000616//HS_3176_A1_E06_T7 CIT Approved Human Genomic Sperm Library D Homo sapiens genomic clone Plate=3176 Col=11 Row=I, genomic survey sequence.//4.7e-28:287:79//AQ300310
F-MAMMA1000621//Human DNA sequence *** SEQUENCING IN PROGRESS *** from clone 273F20, WORKING DRAFT SEQUENCE.//0.015:478:58//AL034371
F-MAMMA1000623
F-MAMMA1000625//DNA encoding Hepatitis C virus antigen.//0.93:196:61//E06898
F-MAMMA1000643//Homo sapiens nephrocystin (NPHP1) mRNA, partial cds.//0.95:365:59//AF023674
F-MAMMA1000664//HS_3096_B1_C02_MR CIT Approved Human Genomic Sperm Library D Homo sapiens genomic clone Plate=3096 Col=3 Row=F, genomic survey sequence.//2.7e-51:257:99//AQ145137
F-MAMMA1000669//Homo sapiens chromosome 19, cosmid R26908, complete sequence.//2.0e-66:586:67//AC004785
F-MAMMA1000670//HS_2243_B2_A08_MR CIT Approved Human Genomic Sperm Library D Homo sapiens genomic clone Plate=2243 Col=16 Row=B, genomic survey sequence.//8.7e-05:94:80//AQ153650
F-MAMMA1000672//Mus musculus clone OST8270, genomic survey sequence.//3.9e-64:471:81//AF046705
F-MAMMA1000684//Suid herpesvirus 1 Rsp40 mRNA, partial cds.//1.2e-07:186:67//U27489
F-MAMMA1000696//Human oligodendrocyte myelin glycoprotein (OMG) exons 1-2; neurofibromatosis 1 (NF1) exons 28-49; ecotropic viral integration site 2B (EVI2B) exons 1-2; ecotropic viral integration site 2A (EVI2A) exons 1-2; adenylate kinase (AK3) exons 1-2.//3.0e-53:653:70//L05367
F-MAMMA1000707//CIT-HSP-2302019.TR CIT-HSP Homo sapiens genomic clone 2302O19, genomic survey sequence.//1.8e-08:131:77//AQ017947
F-MAMMA1000713//Rattus norvegicus clonel polymeric immunoglobulin receptor mRNA 3' untranslated region, GA rich region, and microsatellites with GGA-triplet and GAA-triplet repeats.//0.062:134:67//U00762
F-MAMMA1000714//Chicken hsp90 gene for 90 kDa-heat shock protein 5'-end.//1.0:266:61//X15028
F-MAMMA1000718//CIT-HSP-2171B10.TF CIT-HSP Homo sapiens genomic clone 2171B10, genomic survey sequence.//3.6e-05:289:60//B95401
F-MAMMA1000720//Homo sapiens chromosome 19, cosmid R33632, complete sequence.//4.4e-184:842:98//AC005781
F-MAMMA1000723//Homo sapiens clone DJ0892G19, complete sequence.//8.8e-05:430:60//AC004917
F-MAMMA1000731//Drosophila melanogaster DNA sequence (P1 DS07049 (D133)), complete sequence.//3.8e-55:796:66//AC004274
F-MAMMA1000732//Homo sapiens chromosome 21q22.3 PAC 141B3, complete sequence, containing ribosomal protein homologue pseudogene L23a.//6.6e-77:555:74//AF064859
F-MAMMA1000733//Plasmodium falciparum DNA *** SEQUENCING IN PROGRESS *** from MAL1P6, WORKING DRAFT SEQUENCE.//0.98:479:58//AL031749
F-MAMMA1000734//Homo sapiens SEC63 (SEC63) mRNA, complete cds.//7.3e-168:802:98//AF100141
F-MAMMA1000738//S.cerevisiae chromosome XIV reading frame ORF YNL132w.//8.6e-31:626:63//Z71408
F-MAMMA1000744//Gorilla Alu-repetitive sequence in beta-globin gene cluster.//2.7e-54:410:82//X06123
F-MAMMA1000746//Homo sapiens Chromosome 16 BAC clone CIT987SK-A-10F4, complete sequence.//3.7e-109:779:83//AC004158
F-MAMMA1000752//Homo sapiens clone RG219E16, WORKING DRAFT SEQUENCE, 3 unordered pieces.//1.2e-20:444:63//AC005075
F-MAMMA1000760//Homo sapiens clone RG015P03, complete sequence.//1.5e-44:403:79//AC005048
F-MAMMA1000761//Homo sapiens Chromosome 7 BAC Clone 239c10, WORKING DRAFT SEQUENCE, 9 unordered pieces.//2.3e-22:159:81//AC004166
F-MAMMA1000775//Homo sapiens chromosome 17, clone hRPK.849_N_15, complete sequence.//1.3e-51:789:68//AC005703
F-MAMMA1000776//Human DNA sequence from BAC 57G9 on chromosome 22q12.1 Contains ESTs, CA repeat, GSS.//5.7e-40:238:78//Z95116
F-MAMMA1000778//Human DNA sequence *** SEQUENCING IN PROGRESS *** from clone 153G14, WORKING DRAFT SEQUENCE.//7.6e-29:222:84//AL031118
F-MAMMA1000782//Human 2,4-dienoyl-CoA reductase gene, exon 9.//0.90:137:62//U94987
F-MAMMA1000798//*** SEQUENCING IN PROGRESS *** EPM1/APECED region of chromosome 21, clones A68E8, B127P21, B173L3, B23N8, C1242C9, C579E2, A70B6, B159G9, B175D10, B52C10, C124G1 Note: Sequencing in this region has been discontinued by the Stanford Human Genome Center, WORKING DRAFT SEQUENCE, 50 unordered pieces.//0.00058:163:71//AC003656
F-MAMMA1000802//Homo sapiens chromosome 19, cosmid R33729, complete sequence.//6.3e-151:714:99//AC005339
F-MAMMA1000824//Homo sapiens 12p13.3 BAC RPCI11-543P15 (Roswell Park Cancer Institute Human BAC Library) complete sequence.//4.2e-104:503:99//AC005912
F-MAMMA1000831//Homo sapiens clone UWGC:g1211a139, complete sequence.//0.76:302:58//AC005502
F-MAMMA1000839//Human BAC clone RG013L03 from 7q21, complete sequence.//1.9e-54:322:68//AC002456
F-MAMMA1000841//Human DNA sequence *** SEQUENCING IN PROGRESS *** from clone 968D22, WORKING DRAFT SEQUENCE.//6.7e-140:647:92//AL023755
F-MAMMA1000842//, complete sequence.//0.0068:499:59//AC005817
F-MAMMA1000843//Plasmodium falciparum 3D7 chromosome 12 PFYAC588 genomic sequence, WORKING DRAFT SEQUENCE, 2 unordered pieces.//0.13:439:59//AC004710
F-MAMMA1000845//Plasmodium falciparum DNA *** SEQUENCING IN PROGRESS *** from MAL4P1, WORKING DRAFT SEQUENCE.//2.2e-05:208:64//AL034557
F-MAMMA1000851//Gallus domesticus filamin gene 5' region, partial cds.//0.86:193:63//U00146
F-MAMMA1000855//Human minisatellite region detected by myoglobin 33-repeat probe, clone lambda 33.10.//0.081:229:62//M30549
F-MAMMA1000856//B.taurus microsatellite marker ETH8 (D6S3) DNA.//0.0024:253:60//Z22747
F-MAMMA1000859//Sequence 6 from Patent WO9722695.//2.3e-79:533:82//A63553
F-MAMMA1000862
F-MAMMA1000863//Homo sapiens genomic DNA, chromosome 21q11.1, segment 21/28, WORKING DRAFT SEQUENCE.//1.0e-28:439:64//AP000050
F-MAMMA1000865
F-MAMMA1000867//CIT-HSP-2385J8.TR.1 CIT-HSP Homo sapiens genomic clone 2385J8, genomic survey sequence.//0.00017:158:70//AQ240906
F-MAMMA1000875//Homo sapiens DNA sequence from PAC 232G24 on chromosome Xq27.1-q27.3. Contains two exons similar to MAGE gene family, EST, CA repeat, STS, complete sequence.//1.0:121:68//AL022152
F-MAMMA1000876//Homo sapiens clone HS19.6 Alu-Ya5 sequence.//8.4e-41:185:90//AF015152
F-MAMMA1000877//Homo sapiens DNA sequence from clone 78F24 on chromosome 22q12.1-12.3. Contains one exon of an Oxysterol-binding protein (OSBP) LIKE gene. Contains GSSs and an STS, complete sequence.//8.3e-57:522:75//AL022336
F-MAMMA1000880//Homo sapiens full-length insert cDNA clone ZD54A10.//5.2e-26:143:100//AF086327
F-MAMMA1000883//Human DNA sequence from clone 786D3 on chromosome 22q13.31-33 Contains GSS, complete sequence.//0.99:225:63//AL023801
F-MAMMA1000897//R.norvegicus mRNA for plasma protein.//4.8e-07:479:58//Y11283
F-MAMMA1000905//F26L5TRB IGF Arabidopsis thaliana genomic clone F26L5, genomic survey sequence.//0.94:115:66//B61433
F-MAMMA1000906//HS_3110_B2_A11_MR CIT Approved Human Genomic Sperm Library D Homo sapiens genomic clone Plate=3110 Col=22 Row=B, genomic survey sequence.//2.5e-63:548:78//AQ182819
F-MAMMA1000908//Human DNA sequence *** SEQUENCING IN PROGRESS *** from clone 27K12, WORKING DRAFT SEQUENCE.//5.2e-80:480:90//AL033397
F-MAMMA1000914//Plasmodium falciparum MAL3P8, complete sequence.//7.6e-09:596:58//AL034560
F-MAMMA1000921//CIT-HSP-2171D8.TR CIT-HSP Homo sapiens genomic clone 2171D8, genomic survey sequence.//6.6e-07:249:66//889575
F-MAMMA1000931//Homo sapiens clone DJ0892G19, complete sequence.//2.9e-43:415:66//AC004917
F-MAMMA1000940//HS-1056-A2-E02-MF.abi CIT Human Genomic Sperm Library C Homo sapiens genomic clone Plate=CT 778 Col=4 Row=I, genomic survey sequence.//6.1e-44:235:78//B47296
F-MAMMA1000941//Homo sapiens Chromosome 16 BAC clone CIT987-SKA-113A6 complete genomic sequence, complete sequence.//9.4e-48:443:75//AC002299
F-MAMMA1000942//Human DNA sequence from clone 914P14 on chromosome Xq23 Contains calpain-like protease gene, DCX (doublecortin) ESTs, CA repeat, GSS, complete sequence.//1.8e-14:175:76//AL031117
F-MAMMA1000943//Plasmodium falciparum 3D7 chromosome 12 PFYAC492 genomic sequence, WORKING DRAFT SEQUENCE, 5 unordered pieces.//0.0082:684:56//AC005308
F-MAMMA1000956//Homo sapiens chromosome 16, cosmid clone 363E3 (LANL), complete sequence.//3.3e-30:530:67//AC004643
F-MAMMA1000957//HS_3039_A2_C08_MF CIT Approved Human Genomic Sperm Library D Homo sapiens genomic clone Plate=3039 Col=16 Row=E, genomic survey sequence.//1.3e-72:390:94//AQ155121
F-MAMMA1000962//Homo sapiens clone DJ0756H11, WORKING DRAFT SEQUENCE, 5 unordered pieces.//1.8e-58:318:86//AC006001
F-MAMMA1000968//Homo sapiens DNA sequence from clone 511B24 on chromosome 20q11.2-12. Contains the TOP1 gene for Topoisomerase I, the PLCG1 gene for 1-Phosphatidylinositol-4,5-Bisphosphate Phosphodiesterase Gamma 1 (EC 3.1.4.11, PLC-Gamma-1, Phospholipase C-Gamma-1 PLC-II, PLC-148), the KIAA0395 gene for a probable Zinc Finger Homeobox protein and a 60S Ribosomal Protein L23 LIKE pseudogene. Contains a predicted CpG island, ESTs, STSs and GSSs, complete sequence.//1.4e-18:396:65//AL022394
F-MAMMA1000975//Human DNA sequence from clone 344I7 on chromosome Xp11.21-11.3. Contains a Keratin, Type II Cytoskeletal 8 (Cytokeratin 8, CYK8, KRT8) pseudogene, ESTs and a GSS, complete sequence.//1.4e-79:690:77//AL024458
F-MAMMA1000979//Homo sapiens PAC clone DJ1186C01 from 7q21.2-q31.1, complete sequence.//0.089:214:66//AC004991
F-MAMMA1000987//Human PAC clone DJ527C21 from Xq23, complete sequence.//1.1e-58:458:82//AC000114
F-MAMMA1000998//Human DNA sequence from PAC 997K18 on chromosome 20p12. Contains ESTs and CA repeat.//1.1e-05:439:62//AL021406
F-MAMMA1001003//Homo sapiens DNA sequence from PAC 93L7 on chromosome Xq21. Contains part of the CHM (TCD, REP1) gene coding for RAB Escort protein 1 (REP-1, RAB proteins geranylgeranyltransferase component A 1, Choroideraemia protein, Tapetochoroidal Dystrophy (TCD) protein). Contains ESTs and an STS, complete sequence.//0.24:166:68//AL022401
F-MAMMA1001008//Homo sapiens *** SEQUENCING IN PROGRESS ***, WORKING DRAFT SEQUENCE.//1.6e-103:139:99//AJ011929
F-MAMMA1001021//Homo sapiens clone 24544 beta-dystrobrevin mRNA, partial cds.//6.5e-48:465:76//AF070567
F-MAMMA1001024//CITBI-E1-2501L21.TF.1 CITBI-E1 Homo sapiens genomic clone 2501L21, genomic survey sequence.//1.0:175:62//AQ241701
F-MAMMA1001030//Homo sapiens G protein-coupled receptor LGR5 (LGR5) mRNA, complete cds.//1.1e-30:753:6//1AF061444
F-MAMMA1001035//Human Chromosome 16 BAC clone CIT987SK-A-1000D7, complete sequence.//7.9e-24:256:76//AC002990
F-MAMMA1001038//CIT-HSP-2284N21.TF CIT-HSP Homo sapiens genomic clone 2284N21, genomic survey sequence.//0.96:78:75//AQ000903
F-MAMMA1001041//chicken mRNA for alpha-actinin, complete cds.//2.8e-09:355:63//D26597
F-MAMMA1001050//Homo sapiens BAC clone RG060P12 from 7q21, complete sequence.//2.6e-40:378:76//AC002457
F-MAMMA1001059//Mouse RNA helicase and RNA-dependent ATPase from the DEAD box family mRNA, complete cds.//4.8e-97:661:83//L25125
F-MAMMA1001067//Homo sapiens genomic intron breakpoint sequence of MLL rearrangement, 285 bp.//2.8e-18:110:100//AJ000169
F-MAMMA1001073//HS_3046_A2_G08_MF CIT Approved Human Genomic Sperm Library D Homo sapiens genomic clone Plate=3046 Col=16 Row=M, genomic survey sequence.//1.0:142:68//AQ098420
F-MAMMA1001074//Human DNA sequence *** SEQUENCING IN PROGRESS *** from clone 455J7, WORKING DRAFT SEQUENCE.//1.2e-23:386:70//AL031733
F-MAMMA1001075//Homo sapiens (clone F4) transmembrane protein mRNA sequence.//1.1e-27:559:65//L09749
F-MAMMA1001078//Homo sapiens chromosome 17, clone hRPK.346_K_10, complete sequence.//2.0e-22:334:69//AC006120
F-MAMMA1001080//Human immunoglobulin heavy chain variable region (VH III family) from IgM rheumatoid factor.//6.4e-58:327:92//L29155
F-MAMMA1001082//Homo sapiens Xp22 GSHB-314C4 (Genome Systems Human BAC library) complete sequence.//3.8e-87:695:77//AC004087
F-MAMMA1001091//Homo sapiens chromosome 19, cosmid F21967, complete sequence.//7.0e-05 :594:60//AC005256
F-MAMMA1001092//Human DNA sequence from PAC 49C23 on chromosome X contains malate dehydrogenase pseudogene and STS.//1.6e-91:174:87//Z93019
F-MAMMA1001105//Homo sapiens OVO-like 1 binding protein (OVOL1) mRNA, complete cds.//6.4e-23:507:66//AF016045
F-MAMMA1001110//Homo sapiens chromosome 19, cosmid F16815, complete sequence.//0.77:316:60//AC004637
F-MAMMA1001126//Homo sapiens PAC 50H2 in the CUTL1 locus, complete sequence.//3.3e-21:237:73//AF047825
F-MAMMA1001133//Human DNA sequence from BAC 57G9 on chromosome 22q12.1 Contains ESTs, CA repeat, GSS.//0.97:202:63//Z95116
F-MAMMA1001139//tricarboxylate carrier [rats, liver, mRNA Partial, 2986 nt].//1.6e-84:406:82//S70011
F-MAMMA1001143//Homo sapiens DNA sequence from cosmid N75B3 on chromosome 22 Contains EST, exon trap, complete sequence.//1.3e-14:182:76//AL022339
F-MAMMA1001145//Human DNA sequence from cosmid cU115G11, between markers DXS6791 and DXS8038 on chromosome X contains ESTs and STS.//5.2e-87:714:78//Z71187
F-MAMMA1001154//CIT-HSP-2341D13.TF CIT-HSP Homo sapiens genomic clone 2341D13 genomic survey sequence.//0.00051:249:61//AQ055735
F-MAMMA1001161//Homo sapiens chromosome 14, BAC CITB-135H17 containing the RAD51L1 gene, complete sequence.//2.2e-30:410:70//AC004518
F-MAMMA1001162//Homo sapiens full-length insert cDNA clone ZA79C01.//2.4e-13:87:100//AF086123
F-MAMMA1001181//Mus musculus C2C12 unknown mRNA, partial cds.//9.3e-15:432:60//U31629
F-MAMMA1001186//Homo sapiens chromosome 17, clone hRPK.74_E_22, complete sequence.//6.8e-57:670:72//AC005696
F-MAMMA1001191
F-MAMMA1001198//Mus musculus eps15R mRNA, complete cds.//1.5e-117:759:84//U29156
F-MAMMA1001202
F-MAMMA1001203//Homo sapiens chromosome 17, clone hRPK.22_N_12, WORKING DRAFT SEQUENCE, 2 ordered pieces.//1.5e-161:764:98//AC005412
F-MAMMA1001206//Homo sapiens chromosome 17, clone HCIT421K24, complete sequence.//5:1e-30:535:65//AC004099
F-MAMMA1001215//Homo sapiens chromosome 19, CIT-HSP BAC 470n8, complete sequence.//8.4e-182:860:98//AC005393
F-MAMMA1001220//Homo sapiens PAC clone DJ0745K06 from 7q31, complete sequence.//7.7e-58:690:70//AC004875
F-MAMMA1001222//Mouse loricrin mRNA, complete cds.//2.7e-07:624:58//M34398
F-MAMMA1001243//Homo sapiens chromosome 17, clone hRPK.192_H_23, complete sequence.//0.91:177:66//AC005726
F-MAMMA1001244
F-MAMMA1001249//Human 28S ribosomal RNA psuedogenes and alu repeat region sequence.//6.7e-09:502:58//U67616
F-MAMMA1001256//Human DNA sequence from clone 441J1 on chromosome 6p24 Contains STS, GSS, complete sequence.//5.0e-37:342:80//Z99495
F-MAMMA1001259
F-MAMMA1001260//Homo sapiens mRNA for KIAA0661 protein, complete cds.//8.7e-40:659:64//AB014561
F-MAMMA1001268//Homo sapiens PAC clone DJ0844F09 from 7p12-p13, complete sequence.//4.9e-43:265:81//AC004453
F-MAMMA1001271//Salmo salar DNA for a cryptic repeat.//2.6e-06:311:63//AJ012206
F-MAMMA1001274//Homo sapiens clone DJ0607J02, WORKING DRAFT SEQUENCE, 12 unordered pieces.//6.6e-70:327:83//AC004840
F-MAMMA1001280//Homo sapiens Xp22 bins 87-93 PAC RPCI1-122K4 (Roswell Park Cancer Institute Human PAC Library) complete sequence.//1.0e-05 :276:66//AC003035
F-MAMMA1001292//Human DNA sequence from clone 1170K4 on chromosome 22q12.2-13.1. Contains three novel genes, one of which codes for a Trypsin family protein with class A LDL receptor domains, and the IL2RB gene for Interleukin 2 Receptor, Beta (IL-2 Receptor, CD122 antigen). Contains a putative CpG island, ESTs, and GSSs, complete sequence.//3.6e-98:199:98//AL022314
F-MAMMA1001296//RPCI11-38B4.TV RPCI-11 Homo sapiens genomic clone RPCI-11-38B4, genomic survey sequence.//4.7e-33:292:71//AQ030084
F-MAMMA1001298//Homo sapiens chromosome 17, clone hRPK.849_N_15, complete sequence.//1.6e-182:860:98//AC005703
F-MAMMA1001305//Human DNA sequence from clone 116F5 on chromosome 22q13. Contains part of an unknown gene and part of a RhoGAP (CDC42 GTPAse Activating Protein) LIKE gene. Contains ESTs, STSs, GSSs, genomic marker D22S1168 and a CA repeat polymorphism, complete sequence.//1.9e-70:163:97//Z93244
F-MAMMA1001322//Human DNA sequence from clone 774I24 on chromosome 1q24.1-24.3 Contains protein similar to pregnancy-associated plasma protein A precursor neuronal migration protein astrotactin, ESTs, STS and GSS, complete sequence.//2.6e-19:379:68//AL031290
F-MAMMA1001324//Human DNA sequence *** SEQUENCING IN PROGRESS *** from clone 197L1, WORKING DRAFT SEQUENCE.//4.5e-131:751:90//AL031390
F-MAMMA1001330
F-MAMMA1001341//Sus scrofa.//1.6e-36:420:73//Z46906
F-MAMMA1001343//Plasmodium falciparum DNA *** SEQUENCING IN PROGRESS *** from MAL1P1, WORKING DRAFT SEQUENCE.//1.1e-05:818:58//AL031744
F-MAMMA1001346
F-MAMMA1001383//Homo sapiens, WORKING DRAFT SEQUENCE, 52 unordered pieces.//2.0e-44:505:74//AC004086
F-MAMMA1001388//Human IGF binding protein complex acid-labile subunit a mRNA, complete cds.//1.5e-07:415:58//M86826
F-MAMMA1001397//Human DNA sequence from clone 462D8 on chromosome 22q11.21-12.1 Contains EST, STS and GSS, complete sequence.//1.6e-23 :209:75//AL022332
F-MAMMA1001408//HS_3242_A1_H11_T7 CIT Approved Human Genomic Sperm Library D Homo sapiens genomic clone Plate=3242 Col=21 Row=O, genomic survey sequence.//2.7e-07:181:69//AQ207300
F-MAMMA1001411//Homo sapiens autosomal dominant polycystic kidney disease type II protein (PKD2) gene, exon 14.//0.98:120:68//AF004872
F-MAMMA1001419//HS_2053_B1_F12_T7 CIT Approved Human Genomic Sperm Library D Homo sapiens genomic clone Plate=2053 Col=23 Row=L, genomic survey sequence.//1.9e-75 :424:93//AQ244585
F-MAMMA1001420//Homo sapiens chromosome 4 clone B203C23 map 4q25, complete sequence.//2.4e-09:199:70//AC004049
F-MAMMA1001435//Homo sapiens chromosome 16p11.2 BAC clone CIT987SK-2011O4, WORKING DRAFT SEQUENCE, 4 unordered pieces.//5.1e-42:558:69//AC004529 F-MAMMA1001442//Plasmodium falciparum chromosome 2, section 37 of 73 of the complete sequence.//0.0019:516:56//AE001400
F-MAMMA1001446//Homo sapiens Xp22 BAC GSHB-519E5 (Genome Systems Human BAC library) complete sequence.//3.6e-42:486:70//AC003684
F-MAMMA1001452//RPCI11-48022.TJ RPCI11 Homo sapiens genomic clone R-48O22, genomic survey sequence.//5.3e-87:423:98//AQ199294
F-MAMMA1001465//Human DNA sequence *** SEQUENCING IN PROGRESS *** from clone 414D7, WORKING DRAFT SEQUENCE.//0.00038:114:75//AL033543
F-MAMMA1001476//Mus musculus uridine kinase mRNA, partial cds.//4.1e-99:604:87//L31783
F-MAMMA1001487//Homo sapiens clone DJ1070G24, WORKING DRAFT SEQUENCE, 12 unordered pieces.//1.0e-13:158:77//AC005486
F-MAMMA1001501//Human mRNA for calcium activated neutral protease large subunit (muCANP, calpain, EC 3.4.22.17).//9.6e-52:438:81//X04366
F-MAMMA1001502//Human DNA sequence *** SEQUENCING IN PROGRESS *** from clone 356B7, WORKING DRAFT SEQUENCE.//3.7e-152:720:99//AL031714
F-MAMMA1001510//Human PAC clone DJ438O4 from 22q12.1-qter, complete sequence.//1.1e-05:371:61//AC002378
F-MAMMA1001522
F-MAMMA1001547
F-MAMMA1001551//Homo sapiens mRNA for KIAA0462 protein, partial cds.//2.3e-128:614:98//AB007931
F-MAMMA1001575//Human Chromosome 16 BAC clone CIT987SK-A-815A9, complete sequence.//0.97:154:68//AF001548
F-MAMMA1001576//Human gamma-tubulin mRNA, complete cds.//1.8e-95:529:91//M61764
F-MAMMA1001590//Human DNA sequence from clone 125H2 on chromosome 22q11-12 Contains part of myosin heavy chain gene, EST, CA repeat, STS, GSS, complete sequence.//1.8e-07:104:84//Z98949
F-MAMMA1001600//HS_3022_A2_H01_T7 CIT Approved Human Genomic Sperm Library D Homo sapiens genomic clone Plate=3022 Col=2 Row=O, genomic survey sequence.//1.6e-66:405:90//AQ163791
F-MAMMA1001604//Human DNA sequence from clone 1114G22 on chromosome 1q24-25 Contains EST, CA repeat, Ninenin like sequence, complete sequence.//0.00043:715:58//AL008626
F-MAMMA1001606//jd114 Trypanosome Shotgun M13 genomic Trypanosoma brucei brucei genomic clone 2G6, genomic survey sequence.//0.19:266:62//B13685
F-MAMMA1001620//Homo sapiens monocyte/neutrophil elastase inhibitor gene, complete cds.//9.7e-54:442:69//AF053630
F-MAMMA1001627//X.borealis ribosomal spacer DNA, with a DNaseI-hypersensitive site.//0.14:221:62//M29833
F-MAMMA1001630//Homo sapiens chromosome 17, clone hRPK.22_N_12, WORKING DRAFT SEQUENCE, 2 ordered pieces.//2.0e-47:611:71//AC005412
F-MAMMA1001633//Human zinc finger protein (LD5-1) mRNA, complete cds.//1.1e-42:611:67//U57796
F-MAMMA1001635//Human BAC clone RG072E11 from 7q21-7q22, complete sequence.//4.0e-35:407:70//AC000118
F-MAMMA1001649//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//0.44:245:63//AL022577
F-MAMMA1001654//Mouse transcriptional control element.//0.0025:189:63//M17284
F-MAMMA1001663//CIT-HSP-2165E16.TR CIT-HSP Homo sapiens genomic clone 2165E16, genomic survey sequence.//9.7e-05:146:66//B95491
F-MAMMA1001670//HS_3136_A1_G06_MR CIT Approved Human Genomic Sperm Library D Homo sapiens genomic clone Plate=3136 Col=11 Row=M, genomic survey sequence.//3.1e-28:237:85//AQ148779
F-MAMMA1001671//Homo sapiens chromosome 19, cosmid F23269, complete sequence.//3.3e-181:863:98//AC005614
F-MAMMA1001679//HS_3054_A1_H11_MR CIT Approved Human Genomic Sperm Library D Homo sapiens genomic clone Plate=3054 Col=21 Row=O, genomic survey sequence.//1.0:89:70//AQ106118
F-MAMMA1001683//Spermatozopsis similis mRNA for 90 kD basal apparatus-protein.//8.3e-07:480:62//AJ224970
F-MAMMA1001686//HS_3219_B1_A03_MR CIT Approved Human Genomic Sperm Library D Homo sapiens genomic clone Plate=3219 Col=5 Row=B, genomic survey sequence.//0.00072:180:65//AQ180345
F-MAMMA1001692//HS_3047_B1_B10_MF CIT Approved Human Genomic Sperm Library D Homo sapiens genomic clone Plate=3047 Col=19 Row=D, genomic survey sequence.//2.5e-94:459:98//AQ134228
F-MAMMA1001711//Homo sapiens clone DJ0635O05, WORKING DRAFT SEQUENCE, 7 unordered pieces.//1.2e-42:316:82//AC004845
F-MAMMA1001715//CIT-HSP-2347A14.TF CIT-HSP Homo sapiens genomic clone 2347A14, genomic survey sequence.//1.1e-60:413:87//AQ059125
F-MAMMA1001730//Homo sapiens brain and nasopharyngeal carcinoma susceptibility protein NSG-x mRNA, partial cds.//1.8e-133:646:97//AF095687
F-MAMMA1001735//chicken brain tubulin beta chain mrna.//3.5e-110:740:84//J00913
F-MAMMA1001740//Human DNA sequence from PAC 136017 on chromosome X contains ESTs and STS.//0.98:416:57//Z72001
F-MAMMA1001743//Homo sapiens clone DJ0981O07, complete sequence.//3.2e-16:194:75//AC006017
F-MAMMA1001744//Homo sapiens DNA sequence from clone 46618 on chromosome Xq11.1-13.2. Contains an unknown gene similar to Coagulation Factor V (Activated Protein C Cofactor), Coagulation Factor VIII (Procoagulant Component) and Ceruloplasmin (EC 1.16.3.1, Ferroxidase). Contains ESTs and an STS, complete sequence.//0.0036:181:66//AL030998
F-MAMMA1001745//Homo sapiens BAC clone 529F11 from 8q21, complete sequence.//1.2e-60:822:68//AF070718
F-MAMMA1001751//Human potassium channel KCNO1 mRNA, complete cds.//1.2e-35:583:65//U90065
F-MAMMA1001754//Bos taurus vacuolar proton pump subunit SFD alpha isoform (SFD) mRNA, complete cds.//8.4e-102:627:87//AF041338
F-MAMMA1001757//HS_2058_B2_C04_MR CIT Approved Human Genomic Sperm Library D Homo sapiens genomic clone Plate=2058 Col=8 Row=F, genomic survey sequence.//1.7e-24:173:88//AQ243865
F-MAMMA1001760//Human DNA sequence from clone 354N19 on chromosome 6q22. Contains the 3' part of the gene for Mannosyl-Oligosaccharide Alpha-1,2-Mannosidase (Man(9)-alpha-mannosidase, EC 3.2.1.113), a Cytochrome C Oxidase Polypeptide I (EC 1.9.3.1) pseudogene and a pseudogene similar to 60S Ribosomal Protein L13A. Contains genomic markers D6S287 and D6S1696, ESTs, STSs, GSSs and two CA repeat polymorphisms, complete sequence.//6.6e-76:349:87//AL022722
F-MAMMA1001764//Saccharomyces douglasii mitochondrial cytochrome c oxidase subunit I (COXI) gene, complete cds.//0.23:633:57//M97514
F-MAMMA1001768//Bovine herpesvirus 1 complete genome.//2.3e-11:547:60//AJ004801
F-MAMMA1001769//Homo sapiens 12q13.1 PAC RPCI1-228P16 (Roswell Park Cancer Institute Human PAC Library) complete sequence.//1.1e-76:509:78//AC004801
F-MAMMA1001771//M.musculus mRNA for semaphorin B.//2.7e-106:744:82//X85991
F-MAMMA1001783//Human PAC clone 127H14 from 12q, complete sequence.//6.0e-20:228:75//AC002563
F-MAMMA1001785
F-MAMMA1001788//Human DNA sequence from clone 425C14 on chromosome 6q22 Contains the HSF2 gene for Heat Shock Factor 2 (Heat Shock Transcription Factor 2, HSTF 2) and an unknown gene similar to the placental protein DIFF33 gene. Contains ESTs, STSs and GSSs, complete sequence.//5.0e-05:152:74//Z99129
F-MAMMA1001790//Homo sapiens chromosome 12p13.3 clone RPCI3-454B23, WORKING DRAFT SEQUENCE, 48 unordered pieces.//4.5e-53:318:80//AC005845
F-MAMMA1001806//Homo sapiens chromosome 19, cosmid R29368, complete sequence.//1.0:131:67//AC004262
F-MAMMA1001812//Human Chromosome X clone bWXD187, complete sequence.//3.0e-34:257:83//AC004383
F-MAMMA1001815//Homo sapiens PAC clone DJ0850G01 from 7q21.2-q22, complete sequence.//5.2e-61:516:79//AC004128
F-MAMMA1001817//Homo sapiens 12q24 PAC RPCI1-261P5 (Roswell Park Cancer Institute Human PAC library) complete sequence.//3.1e-32:295:78//AC004031
F-MAMMA1001818//Homo sapiens chromosome 21q22.3, PAC clones 314N7, 225L15, BAC clone 7B7, complete sequence bases 1.333303.//0.71:179:67//AJ011930
F-MAMMA1001820//Rattus norvegicus mRNA for PAG608 gene.//3.0e-91:726:79//Y13148
F-MAMMA1001824//HS_3108_A1_G12_MR CIT Approved Human Genomic Sperm Library D Homo sapiens genomic clone Plate=3108 Col=23 Row=M, genomic survey sequence.//3.4e-05:119:74//AQ107508
F-MAMMA1001836//Homo sapiens chromosome 18, clone hRPK.537_E_1, complete sequence.//3.4e-45:312:85//AC006211
F-MAMMA1001837//Rattus norvegicus zinc finger protein Y1 (RLZF-Y) mRNA, complete cds.//4.5e-51:480:75//AF052042
F-MAMMA1001848//CITBI-E1-2516P17.TF CITBI-E1 Homo sapiens genomic clone 2516P17, genomic survey sequence.//1.0e-100:486:98//AQ279620
F-MAMMA1001851//Human DNA from overlapping chromosome 19-specific cosmids R30072 and R28588, genomic sequence, complete sequence.//5.1e-07:197:67//AC002390
F-MAMMA1001854
F-MAMMA1001858//RPCI11-11L22.TP RPCI-11 Homo sapiens genomic clone RPCI-11-11L22, genomic survey sequence.//0.091:161:65//B75631
F-MAMMA1001864//Human PAC clone DJ0205E24 from Xq23, complete sequence.//2.6e-09:397:61//AC003013
F-MAMMA1001868//HS_2196_B2_A12_MR CIT Approved Human Genomic Sperm Library D Homo sapiens genomic clone Plate=2196 Col=24 Row=B, genomic survey sequence.//5.8e-13:86:100//AQ032455
F-MAMMA1001874//H.sapiens CpG island DNA genomic Mse1 fragment, clone 63h5, reverse read cpg63h5.rtla.//1.0:127:63//Z62129
F-MAMMA1001878//Human DNA sequence from BAC 999D10 on chromosome 22q13.3. Contains two BAC end-sequences (GSSs).//1.7e-19:372:67//Z94802
F-MAMMA1001880//RPCI11-90K3.TJ RPCI11 Homo sapiens genomic clone R-90K3, genomic survey sequence.//6.6e-11:362:62//AQ283465
F-MAMMA1001890//Human DNA sequence *** SEQUENCING IN PROGRESS *** from clone 508I15, WORKING DRAFT SEQUENCE.//1.8e-45:317:86//AL021707
F-MAMMA1001907//Human DNA sequence *** SEQUENCING IN PROGRESS *** from clone 424J12, WORKING DRAFT SEQUENCE.//2.7e-23:255:77//Z82207
F-MAMMA1001908//HS_2225_A1_A03_MR CIT Approved Human Genomic Sperm Library D Homo sapiens genomic clone Plate=2225 Col=5 Row=A, genomic survey sequence.//5.4e-08:264:62//AQ301597
F-MAMMA1001931//HS_3049_B2_D09_MR CIT Approved Human Genomic Sperm Library D Homo sapiens genomic clone Plate=3049 Col=18 Row=H, genomic survey sequence.//1.7e-47:295:90//AQ100157
F-MAMMA1001956//H.sapiens DNA sequence.//0.056:233:66//Z22493
F-MAMMA1001963//Homo sapiens adenylosuccinate lyase gene, complete cds.//0.99:173:68//AF106656
F-MAMMA1001969//Human DNA sequence from cosmid 232L22, between markers DXS366 and DXS87 on chromosome X contains ESTs glycerol kinase pseudogene.//5.3e-63:479:78//Z73986
F-MAMMA1001970//Homo Sapiens BAC clone BK085E05 from 22q12.1-qter, complete sequence.//1.4e-126:699:93//AC003071
F-MAMMA1001992//HS_3078_A1_A09_MF CIT Approved Human Genomic Sperm Library D Homo sapiens genomic clone Plate=3078 Col=17 Row=A, genomic survey sequence.//3.3e-08:257:65//AQ143646
F-MAMMA1002009//Homo sapiens chromosome 17, clone hRPK.214_O_I, complete sequence.//1.5e-07:244:62//AC005224
F-MAMMA1002011//HS_3252_B1_B05_MR CIT Approved Human Genomic Sperm Library D Homo sapiens genomic clone Plate=3252 Col=9 Row=D, genomic survey sequence.//1.3e-07:170:69//AQ304711
F-MAMMA1002032//Homo sapiens chromosome 12p13.3, WORKING DRAFT SEQUENCE, 37 unordered pieces.//2.1e-34:315:79//AC004803
F-MAMMA1002033//HS_3023_A2_G04_T7 CIT Approved Human Genomic Sperm Library D Homo sapiens genomic clone Plate=3023 Col=8 Row=M, genomic survey sequence.//4.3e-69:366:94//AQ105493
F-MAMMA1002041//Genomic sequence from Human 9q34, complete sequence.//5.3e-85:439:82//AC001227
F-MAMMA1002042//Homo sapiens chromosome 3, clone hRPK.165_I_16, complete sequence.//1.4e-20:314:70//AC005669
F-MAMMA1002047//Homo sapiens 12p13.3 BAC RPCII1-429A20 (Roswell Park Cancer Institute Human BAC Library) complete sequence.//6.8e-14:526:62//AC005906
F-MAMMA1002056//Human DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs, an STS and GSSs, complete sequence.//1.1e-47:648:71//AL030996
F-MAMMA1002058//Homo sapiens PAC clone DJ0732C22 from 7p11.2-p13, complete sequence.//2.4e-19:256:74//AC004869
F-MAMMA1002068//Homo sapiens, clone hRPK.2_A_1, complete sequence.//5.4e-41:407:78//AC006197
F-MAMMA1002078//Human DNA sequence from PAC 106I20 on chromosome 22q12 Contains ESTs and STS, complete sequence.//0.021:333:64//Z81313
F-MAMMA1002082
F-MAMMA1002084//Caenorhabditis elegans cosmid F28C12, complete sequence.//0.032:469:58//Z93380
F-MAMMA1002093//HS_3050_B1_F06_MR CIT Approved Human Genomic Sperm Library D Homo sapiens genomic clone Plate=3050 Col=11 Row=L, genomic survey sequence.//1.0:77:71//AQ105997
F-MAMMA1002108//Homo sapiens anion exchanger 3 gene, exons 1 and 2 and complete 5'UTR.//8.3e-10:464:60//AF017308
F-MAMMA1002118
F-MAMMA1002125//Homo sapiens chromosome 17, clone HCIT217L10, complete sequence.//1.0e-35:619:68//AC003962
F-MAMMA1002132//RPCI11-78F11.TJ RPCI11 Homo sapiens genomic clone R-78F11, genomic survey sequence.//1.0e-90:357:97//AQ286460
F-MAMMA1002140//Homo sapiens 12q24 PAC RPCI1-66E7 (Roswell Park Cancer Institute Human PAC library) complete sequence.//1.6e-45:583:64//AC004216
F-MAMMA1002143//Human serum constituent protein (MSE55) mRNA, complete cds.//6.0e-11:192:70//M88338
F-MAMMA1002145//Human DNA sequence *** SEQUENCING IN PROGRESS *** from clone 102D24, WORKING DRAFT SEQUENCE.//0.0028:570:59//AL021391
F-MAMMA1002153//HS_3005_A1_D04_MF CIT Approved Human Genomic Sperm Library D Homo sapiens genomic clone Plate=3005 Col=7 Row=G, genomic survey sequence.//4.9e-41:213:99//AQ132213
F-MAMMA1002155//Human DNA sequence *** SEQUENCING IN PROGRESS *** from clone 462O23, WORKING DRAFT SEQUENCE.//1.2e-45:303:78//AL031431
F-MAMMA1002156
F-MAMMA1002158//CITBI-E1-2508P18.TR CITBI-E1 Homo sapiens genomic clone 2508P18, genomic survey sequence.//7.1e-42:255:92//AQ266165
F-MAMMA1002170//Homo sapiens chromosome 17, clone HCIT187M2, complete sequence.//2.0e-81:604:81//AC004448
F-MAMMA1002174//Homo sapiens clone UWGC:y67c126 from 6p21, complete sequence.//3.2e-43:333:83//AC004212
F-MAMMA1002198//H.sapiens thiol-specific antioxidant protein mRNA.//1.0e-34:121:98//Z22548
F-MAMMA1002209//HS_2197_B1_E07_MR CIT Approved Human Genomic Sperm Library D Homo sapiens genomic clone Plate=2197 Col=13 Row=J, genomic survey sequence.//9.6e-18:163:84//AQ210058
F-MAMMA1002215//Homo sapiens anion exchanger 3 gene, exons 1 and 2 and complete 5'UTR.//6.3e-08:435:60//AF017308
F-MAMMA1002219//Rattus norvegicus rexo70 mRNA, complete cds.//1.8e-124:752:87//AF032667
F-MAMMA1002230//Plasmodium falciparum 3D7 chromosome 12 PFYAC588 genomic sequence, WORKING DRAFT SEQUENCE, 2 unordered pieces.//0.67:356:59//AC004710
F-MAMMA1002236//Rattus norvegicus initiation factor eIF-2B gamma subunit (eIF-2B gamma) mRNA, complete cds.//9.3e-140:836:87//U38253
F-MAMMA1002243//Homo sapiens chromosome 17, clone hRPK.112_H_10, complete sequence.//1.4e-145:691:98//AC005666
F-MAMMA1002250//Homo sapiens chromosome 16, P1 clone 109-9G (LANL), complete sequence.//6.0e-138:660:98//AC005600
F-MAMMA1002267//Homo sapiens chromosome 2, P1 clone 777H5 (LBNL H27), complete sequence.//0.066:333:60//AC003676
F-MAMMA1002268//Mus musculus sphingosine kinase (SPHK1a) mRNA, partial cds.//1.1e-39:404:74//AF068748
F-MAMMA1002269//HS_3163_B1_D03_MR CIT Approved Human Genomic Sperm Library D Homo sapiens genomic clone Plate=3163 Col=5 Row=H, genomic survey sequence.//1.0:150:63//AQ171576
F-MAMMA1002282//Human Chromosome 16 BAC clone CIT987SK-327O24, complete sequence.//1.5e-22:315:67//AC003108
F-MAMMA1002292//B.garinii (strain TIs1) p83/100 gene (partial).//0.73:200:64//X81533
F-MAMMA1002293//Homo sapiens clone DJ1147A01, WORKING DRAFT SEQUENCE, 25 unordered pieces.//1.6e-56:408:75//AC006023
F-MAMMA1002294//Sequence 2 from Patent WO9516779.//1.8e-06:401:62//A45258
F-MAMMA1002297
F-MAMMA1002298//Homo sapiens DNA from chromosome 19, cosmid R29144, complete sequence.//0.0056:525:61//AC004221
F-MAMMA1002299//CIT-HSP-2345B2.TR CIT-HSP Homo sapiens genomic clone 2345B2, genomic survey sequence.//1.2e-90:446:98//AQ053994
F-MAMMA1002308//Human DNA sequence *** SEQUENCING IN PROGRESS *** from clone 850H21, WORKING DRAFT SEQUENCE.//1.3e-35:329:78//AL031680
F-MAMMA1002310//Human gastric (H^{+ +} K⁺)-ATPase gene, complete cds.//0.0060:301:60//J05451
F-MAMMA1002311//Human Chromosome 15q11-q13 clone pDJ276c12 from the Prader-Willi/Angelman syndrome region, WORKING DRAFT SEQUENCE, 3 unordered pieces.//8.6e-50:327:69//AC004737
F-MAMMA1002312//Homo sapiens DNA sequence from PAC 435D1 on chromosome Xq25. Contains ESTs and STS.//1.3e-09:741:58//Z86064
F-MAMMA1002317
F-MAMMA1002319//Homo sapiens chromosome 19, fosmid 39347, complete sequence.//1.9e-158:746:99//AC005756
F-MAMMA1002322//Homo sapiens Chromosome 11p14.3 PAC clone pDJ1034g4, complete sequence.//5.3e-52:617:70//AC004796
F-MAMMA1002329//Homo sapiens RaP2 interacting protein 8 (RPIP8) mRNA, complete cds.//0.22:143:67//U93871
F-MAMMA1002332//Human DNA sequence *** SEQUENCING IN PROGRESS *** from clone 30G7, WORKING DRAFT SEQUENCE.//1.6e-31:287:74//AL034402
F-MAMMA1002333//Mycobacterium tuberculosis H37Rv complete genome; segment 148/162.//2.5e-09:674:59//AL022022
F-MAMMA1002339//Homo sapiens chromosome 21q22.3, cosmid clone Q4H9 complete sequence bases 1.41604.//2.1e-57:522:77//AJ011932
F-MAMMA1002347//Homo sapiens BAC clone RG136N17 from 7p15-p21, complete sequence.//2.0e-14:258:69//AC004129
F-MAMMA1002351//Human DNA sequence *** SEQUENCING IN PROGRESS *** from clone 1059H15, WORKING DRAFT SEQUENCE.//7.8e-132:723:91//AL022100
F-MAMMA1002352//Human DNA sequence *** SEQUENCING IN PROGRESS *** from clone 128O3, WORKING DRAFT SEQUENCE.//5.8e-17:326:70//Z98742
F-MAMMA1002353//Homo sapiens clone DJ0292L20, WORKING DRAFT SEQUENCE, 2 unordered pieces.//1.1e-14:399:63//AC004825
F-MAMMA1002355//Human DNA sequence *** SEQUENCING IN PROGRESS *** from clone 109G6, WORKING DRAFT SEQUENCE.//3.7e-43:420:75//AL023879
F-MAMMA1002356//Plasmodium falciparum 3D7 chromosome 12 PFYAC812 genomic sequence, WORKING DRAFT SEQUENCE, 8 unordered pieces.//0.0022:534:59//AC004153
F-MAMMA1002359//Homo sapiens 12p13.3 PAC RPCI5-1180D12 (Roswell Park Cancer Institute Human PAC Library) complete sequence.//5.3e-18:156:75//AC005831
F-MAMMA1002360//Human DNA sequence from cosmid L21F12B, Huntington's Disease Region, chromosome 4p16.3, contains EST.//4.9e-43:353:69//Z68885
F-MAMMA1002361//Human DNA sequence from clone 342B11 on chromosome 22q12.1-12.3. Contains ESTs and a GSS, complete sequence.//1.8e-22:282:74//AL008719
F-MAMMA1002362//Platemys spixii CR1-like LINE, partial sequence.//0.00058:83:79//D82938
F-MAMMA1002380//CIT-HSP-2383K24.TF CIT-HSP Homo sapiens genomic clone 2383K24, genomic survey sequence.//4.4e-10:85:92//AQ196889
F-MAMMA1002384//RPCI11-80J20.TV RPCI11 Homo sapiens genomic clone R-80J20, genomic survey sequence.//2.7e-56:286:98//AQ284134
F-MAMMA1002385//CIT-HSP-2328G13.TF CIT-HSP Homo sapiens genomic clone 2328G13, genomic survey sequence.//5.5e-46:335:84//AQ043985
F-MAMMA1002392//Homo sapiens PAC clone DJ0797C05 from 7q31, complete sequence.//8.5e-29:273:78//AC004888
F-MAMMA1002411//Human DNA sequence from clone 1044017 on chromosome Xp11.3-11.4 Contains GSS and STS, complete sequence.//8.2e-09:287:63//AL023 875
F-MAMMA1002413//Plasmodium falciparum (strain Dd2) variant-specific surface protein (var1) gene, complete cds.//9.6e-08:730:57//L40608
F-MAMMA1002417//Human DNA sequence *** SEQUENCING IN PROGRESS *** from clone 30G7, WORKING DRAFT SEQUENCE.//4.1e-06:181:72//AL034402
F-MAMMA1002427//*** SEQUENCING IN PROGRESS *** Homo sapiens chromosome 4, BAC clone C0366H07; HTGS phase 1, WORKING DRAFT SEQUENCE, 28 unordered pieces.//1.3e-51:593:72//AC004604
F-MAMMA1002428
F-MAMMA1002434//Homo sapiens DNA sequence from PAC 958B3 on chromosome Xp22.11-Xp22.22. Contains ESTs STS and CpG island.//7.3e-56:388:81//Z93023
F-MAMMA1002446//CIT-HSP-2324O22.TR CIT-HSP Homo sapiens genomic clone 2324O22, genomic survey sequence.//2.3e-56:302:95//AQ027479
F-MAMMA1002454//Homo sapiens PAC clone DJ1136G13 from 7q35-q36, complete sequence.//1.1e-54:190:94//AC005229
F-MAMMA1002461//Rattus norvegicus calcium channel alpha-1 subunit gene, partial cds.//0.00045:457:60//U14005
F-MAMMA1002470//Saccharomyces cerevisiae chromosome VIII cosmid 9205.//9.7e-33:709:60//U10556
F-MAMMA1002475//Homo sapiens 12p13.3 PAC RPCI3-340I3 (Roswell Park Cancer Institute Human PAC Library) complete sequence.//0.092:506:58//AC004671
F-MAMMA1002480//Homo sapiens clone RG228D17, WORKING DRAFT SEQUENCE, 2-unordered pieces.//0.025:100:76//AC005077
F-MAMMA1002485//Homo sapiens stanniocalcin-2 (STC-2) mRNA, complete cds.//2.9e-118:560:98//AF055460
F-MAMMA1002494//Homo sapiens Xp22-175-176 BAC GSHB-484O17 (Genome Systems Human BAC Library) complete sequence.//1.5e-22:297:73//AC005913
F-MAMMA1002498//Human PAC clone DJ327A19 from Xq25-q26, complete sequence.//7.2e-10:330:64//AC002477
F-MAMMA1002524//Homo sapiens huntingtin gene, partial exon.//0.0080:124:72//L49359
F-MAMMA1002530//Homo sapiens cytosolic phospholipase A2 gamma (cPLA2 gamma) mRNA, complete cds.//1.4e-160:775:97//AF065214
F-MAMMA1002545//Homo sapiens chromosome 17, clone hRPK.74_E_22 complete sequence.//1.9e-41:345:80//AC005696
F-MAMMA1002554
F-MAMMA1002556
F-MAMMA1002566
F-MAMMA1002571//CIT-HSP-2296N17.TR CIT-HSP Homo sapiens genomic clone 2296N17, genomic survey sequence.//1.7e-07:76:90//AQ006579
F-MAMMA1002573//Homo sapiens DNA, trinucleotide repeats region, clone GAA C27.//2.7e-08:195:70//AB018507
F-MAMMA1002585
F-MAMMA1002590//Homo sapiens BAC clone GS250A16 from 7p21-p22, complete sequence.//2.1e-26:361:69//AC005019
F-MAMMA1002597//Human DNA sequence *** SEQUENCING IN PROGRESS *** from clone 1103G7, WORKING DRAFT SEQUENCE.//1.3e-34:550:69//AL034548
F-MAMMA1002598//H.sapiens mRNA for ribosomal protein L7.//1.1e-21:123:100//X57958
F-MAMMA1002603//Homo sapiens chromosome 20, BAC clone 99 (LBNL H80), complete sequence.//0.0018:358:61//AC005220
F-MAMMA1002612//Homo sapiens PAC clone DJ0696N01 from 7p21-p22, complete sequence.//2.1e-13:336:63//AC004861
F-MAMMA1002617//Homo sapiens clone DJ1070G24, WORKING DRAFT SEQUENCE, 12 unordered pieces.//0.14:229:64//AC005486
F-MAMMA1002618
F-MAMMA1002619//Homo sapiens chromosome 21 PAC RPCIP704E14135Q2.//9.5e-71:319:85//AJ010598
F-MAMMA1002622//Homo sapiens advillin mRNA, complete cds.//1.5e-20:157:90//AF041449
F-MAMMA1002623//Homo sapiens T-cell receptor alpha delta locus from bases 501613 to 752736 (section 3 of 5) of the Complete Nucleotide Sequence.//8.3e-06:137:72//AE000660
F-MAMMA1002625//Human DNA sequence *** SEQUENCING IN PROGRESS *** from clone 1056L3, WORKING DRAFT SEQUENCE.//1.9e-171:819:98//AL031727
F-MAMMA1002629//Human BAC clone RG385F02 from 7p15, complete sequence.//4.8e-85:478:78//AC003093
F-MAMMA1002636//Human POU daomain factor (Brn-3a) gene, exon 2, complete cds.//5.6e-09:499:62//U10063
F-MAMMA1002637//Mus musculus kinesin light chain 2 (Klc2) mRNA, complete cds.//3.6e-115:785:82//AF055666
F-MAMMA1002646//Homo sapiens chromosome 2 clone 101B6 map 2p11, complete sequence.//1.5e-45:291:90//AC002038
F-MAMMA1002650//Homo sapiens candidate tumor suppressor HIC-1 (HIC-1) gene, complete cds.//6.6e-06:661:59//L41919
F-MAMMA1002655//HS_2003_A2_A11_MR CIT Approved Human Genomic Sperm Library D Homo sapiens genomic clone Plate=2003 Col=22 Row=A, genomic survey sequence.//9.0e-15:198:74//AQ224233
F-MAMMA1002662
F-MAMMA1002665//Homo sapiens BAC clone GS588G18 from 7p12-p14, complete sequence.//1.4e-37:235:84//AC005029
F-MAMMA1002671//Human Cdk-inhibitor p57KIP2 (KIP2) mRNA, complete cds.//0.00027:272:64//U22398
F-MAMMA1002673
F-MAMMA1002684//Homo sapiens mRNA for KIAA0214 protein, complete cds.//3.7e-161:752:99//D86987
F-MAMMA1002685//Human DNA sequence *** SEQUENCING IN PROGRESS *** from clone 394I7, WORKING DRAFT SEQUENCE.//6.2e-45:510:70//AL023585
F-MAMMA1002698//HS_3024_B1_C06_T7 CIT Approved Human Genomic Sperm Library D Homo sapiens genomic clone Plate=3024 Col=11 Row=F, genomic survey sequence.//1.7e-10:155:75//AQ072214
F-MAMMA1002699//Rattus norvegicus EH domain binding protein Epsin mRNA, complete cds.//5.9e-75:509:83//AF018261
F-MAMMA1002701//Homo sapiens gene for AF-6, complete cds.//1.2e-159:749:99//AB011399
F-MAMMA1002708//Human DNA sequence from clone 267M20 on chromosome Xq22.2-22.3. Contains part of the DIAPH2 gene and a pseudogene, ESTs, STSs and GSSs, complete sequence.//3.0e-57:347:79//AL031053
F-MAMMA1002711//Homo sapiens BAC clone GS589P19 from 7p13-p14, complete sequence.//3.4e-31:484:69//AC005030
F-MAMMA1002721//CIT-HSP-2350M5.TR CIT-HSP Homo sapiens genomic clone 2350M5, genomic survey sequence.//1.4e-06:265:63//AQ061245
F-MAMMA1002727//Human DNA sequence from clone 67K17 on chromosome 6q24.1-24.3. Contains the HIVEP2 (Schnurri-2) gene for HIV type 1 Enhancer-binding Protein 2, and a possible pseudogene in an intron of this gene. Contains STSs and GSSs and an AAAT repeat polymorphism, complete sequence.//0.18:386:58//AL023584
F-MAMMA1002728//Human DNA sequence from PAC 296K21 on chromosome X contains cytokeratin exon, delta-aminolevulinate synthase (erythroid); 5-aminolevulinic acid synthase.(EC 2.3.1.37). 6-phosphofructo-2-kinase/fructose-2,6-bisphosphatase (EC 2.7.1.105, EC 3.1.3.46), ESTs and STS.//3.2e-05:362:63//Z83821
F-MAMMA1002744//Plasmodium falciparum chromosome 2, section 5 of 73 of the complete sequence.//0.00010:535:58//AE001368
F-MAMMA1002746//Homo sapiens chromosome 17, clone hRPK.136_H_19, complete sequence.//1.2e-182:880:97//AC005856
F-MAMMA1002748//Homo sapiens 3p22 Contig 7 PAC RPCI4-672N11 (Roswell Park Cancer Institute Human PAC Library) complete sequence.//2.7e-175:829:98//AC006055
F-MAMMA1002754//Homo Sapiens Chromosome X clone bWXD171, WORKING DRAFT SEQUENCE, 1 ordered pieces.//3.1e-31:372:75//AC004676
F-MAMMA1002758//Homo sapiens KIAA0442 mRNA, partial cds.//3.3e-26:151:98//AB007902
F-MAMMA1002764//Human Chromosome 11 Cosmid cSRL166a1, complete sequence.//5.2e-49:355:81//U73636
F-MAMMA1002765//RPCI11-20A22.TPB RPCI-11 Homo sapiens genomic clone RPCI-11-20A22, genomic survey sequence.//6.7e-13:155:76//B92153
F-MAMMA1002769//CIT-HSP-2323G1.TF CIT-HSP Homo sapiens genomic clone 2323G1, genomic survey sequence.//9.7e-21:151:90//AQ028244
F-MAMMA1002775//Human ABL gene, exon 1b and intron 1b, and putative M8604 Met protein (M8604 Met) gene, complete cds.//5.6e-105:179:99//U07561
F-MAMMA1002780//Plasmodium falciparum DNA *** SEQUENCING IN PROGRESS *** from contig 3-08, complete sequence.//0.071:277:58//Z98546
F-MAMMA1002782//HS_3213_B2_B08_MR CIT Approved Human Genomic Sperm Library D Homo sapiens genomic clone Plate=3213 Col=16 Row=D; genomic survey sequence.//0.00018:219:63//AQ175845
F-MAMMA1002796
F-MAMMA1002807//Human Chromosome X PAC RPCI1-290C9 from the Pieter de Jong Human PAC library; complete sequence.//6.9e-22:332:69//AC002404
F-MAMMA1002820//Homo sapiens Xp22 bins 87-93 PAC RPCI1-122K4 (Roswell Park Cancer Institute Human PAC Library) complete sequence.//5.9e-11:483:62//AC003035
F-MAMMA1002830//Homo sapiens chromosome 17, clone hCIT529I10, complete sequence.//1.0e-64:320:83//AC002553
F-MAMMA1002833//Homo sapiens PAC clone DJ0745K06 from 7q31, complete sequence.//2.8e-47:413:80//AC004875
F-MAMMA1002835
F-MAMMA1002838//A-916H10.TP CIT978SK Homo sapiens genomic clone A-916H10, genomic survey sequence.//1.1e-39:164:83//B14462
F-MAMMA1002842//Mus musculus c-Cbl associated protein CAP mRNA, complete cds.//1.9e-62:373:81//U58883
F-MAMMA1002843//Homo sapiens mRNA for KIAA0810 protein, partial cds.//1.7e-135:635:99//AB018353
F-MAMMA1002844//F1707-T7 IGF Arabidopsis thaliana genomic clone F17O7, genomic survey sequence.//6.7e-17:383:66//B11616
F-MAMMA1002858
F-MAMMA1002868//RPCI11-54F9.TJ RPCI11 Homo sapiens genomic clone R-54F9, genomic survey sequence.//8.3e-81:392:99//AQ081566
F-MAMMA1002869//Sequence 1 from patent US 5552529.//2.2e-86:696:78//I25863
F-MAMMA1002871//Lupinus angustifolius nodulin-45 gene, complete cds.//0.029:370:59//L12388
F-MAMMA1002880//RPCI11-23M23.TV RPCI-11 Homo sapiens genomic clone RPCI-11-23M23, genomic survey sequence.//1.8e-20:271:74//B86518
F-MAMMA1002881//Homo sapiens mRNA for 25 kDa trypsin inhibitor, complete cds.//1.2e-28:680:61//D45027
F-MAMMA1002886//Human DNA sequence *** SEQUENCING IN PROGRESS *** from clone 380A1, WORKING DRAFT SEQUENCE.//0.00040:505:57//Z97653
F-MAMMA1002887//HS_3238_B2_G08_MR CIT Approved Human Genomic Sperm Library D Homo sapiens genomic clone Plate=3238 Col=16 Row=N, genomic survey sequence.//5.5e-79:401:97//AQ219814
F-MAMMA1002890//Mus musculus MHC class III region RD gene, partial cds; Bf, C2, G9A, NG22, G9, HSP70, HSP70, HSC70t, and smRNP genes, complete cds; G7A gene, partial cds; and unknown genes.//4.6e-35:136:73//AF109906
F-MAMMA1002892//Mouse Cosmid ma66a100 from 14D1-D2, complete sequence.//5.7e-14:450:60//AC004096
F-MAMMA1002895//H.sapiens CpG island DNA genomic Mse1 fragment, clone 46b6, forward read cpg46b6.ft1a.//3.7e-36:190:100//Z58616
F-MAMMA1002908//Penaeus monodon microsatellite locus Pmo27.//1.1e-05:195:62//AF068828
F-MAMMA1002909//Human Chromosome 11 pac pDJ205d23, complete sequence.//1.0e-13:457:61//AC002402
F-MAMMA1002930//Homo sapiens Xp22 BAC GSHB-512P14 (Genome Systems Human BAC library) complete sequence.//0.25:260:62//AC004467
F-MAMMA1002937//H.sapiens ZNF74-1 mRNA.//6.3e-13:577:59//X71623
F-MAMMA1002938//Homo sapiens mRNA for KIAA0698 protein, complete cds.//5.1e-193:910:98//AB014598
F-MAMMA1002941//Homo sapiens Chromosome 22q11.2 BAC Clone b437g10 In BCRL2-GGT Region, complete sequence.//2.7e-23:174:77//AC004032
F-MAMMA1002947//Rhodobacter capsulatus strain SB1003, partial genome.//1.3e-09:475:61//AF010496
F-MAMMA1002964//Human thiopurine methyltransferase (TPMT) gene, exon 5.//0.0029:314:60//AF019366
F-MAMMA1002970//Human DNA sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.//4.0e-10:194:71//Z94056
F-MAMMA1002972//H.sapiens CpG island DNA genomic Mse1 fragment, clone 2g10, forward read cpg2g10.ft1aa.//0.38:156:66//Z55272
F-MAMMA1002973//Homo sapiens chromosome 17, clone hRPK.142_H_19, complete sequence.//2.9e-41:234:79//AC005919
F-MAMMA1002982//Homo sapiens DNA sequence from PAC 510L9 on chromosome 6p24.1-p25.3.//1.7e-05:322:63//AL022098
F-MAMMA1002987//CITBI-E1-2514J12.TR CITBI-E1 Homo sapiens genomic clone 2514J12, genomic survey sequence.//0.0064:135:66//AQ275871
F-MAMMA1003003//cSRL-145D12-u cSRL flow sorted Chromosome 11 specific cosmid Homo sapiens genomic clone cSRL-145D12, genomic survey sequence.//2.8e-31:201:89//B01998
F-MAMMA1003004//Human DNA sequence *** SEQUENCING IN PROGRESS *** from clone Y237C10, WORKING DRAFT SEQUENCE.//1.6e-10:180:73//AL031601
F-MAMMA1003007//Homo sapiens (clone cosmid c11q-8D1) tetranucleotide repeat polymorphism at the D11S488 locus.//3.5e-12:435:61//L04732
F-MAMMA1003011//Rattus norvegicus histone macroH2A1.2 mRNA, complete cds.//2.3e-50:734:67//U79139
F-MAMMA1003013//Mus musculus chromosome 19, clone CIT282B21, complete sequence.//1.2e-86:341:79//AC003694
F-MAMMA1003015//Homo sapiens Chromosome 16 BAC clone CIT987SK-591M7, complete sequence.//2.6e-13:443:61//AC003661
F-MAMMA1003019//HS_3221_A1_A01_MR CIT Approved Human Genomic Sperm Library D Homo sapiens genomic clone Plate=3221 Col=1 Row=A, genomic survey sequence.//2.8e-51:299:92//AQ184271
F-MAMMA1003026
F-MAMMA1003031//Homo sapiens chromosome 5, BAC clone 319C17 (LBNL H159), complete sequence.//0.0037:134:73//AC005214
F-MAMMA1003035//RPCI11-11P4.TP RPCI-11 Homo sapiens genomic clone RPCI-11-11P4, genomic survey sequence.//1.1e-07:66:100//B74936
F-MAMMA1003039//Homo sapiens 12p13.3 PAC RPCI3-340I3 (Roswell Park Cancer Institute Human PAC Library) complete sequence.//2.1e-19:220:76//AC004671
F-MAMMA1003040//Human DNA sequence from PAC 340N1 on chromosome 1p35-36.2. Contains ESTs, polymorphic CA repeat, trna and endogenous retrovirus.//9.5e-91:469:78//Z98257
F-MAMMA1003044//Human DNA sequence from clone 496N17 on chromosome 6p11.2-12.3 Contains EST, GSS, complete sequence.//0.21:289:61//AL031321
F-MAMMA1003047//Homo sapiens protein inhibitor of activated STAT protein PIASy mRNA, complete cds.//1.7e-139:663:98//AF077952
F-MAMMA1003049
F-MAMMA1003055//HS_3014_B2_F10_MR CIT Approved Human Genomic Sperm Library D Homo sapiens genomic clone Plate=3014 Col=20 Row=L, genomic survey sequence.//4.2e-05:215:64//AQ164940
F-MAMMA1003056//HS_3221_B2_D12_MR CIT Approved Human Genomic Sperm Library D Homo sapiens genomic clone Plate=3221 Col=24 Row=H, genomic survey sequence.//1.4e-16:206:74//AQ302772
F-MAMMA1003057//M.domesticus MD6 mRNA.//8.5e-128:654:94//X54352
F-MAMMA1003066//Human DNA sequence *** SEQUENCING IN PROGRESS *** from clone 273F20, WORKING DRAFT SEQUENCE.//1.0:142:71//AL034371
F-MAMMA1003089//Homo sapiens Chromosome 11p14.3 PAC clone pDJ1034g4, complete sequence.//1.7e-42:373:78//AC004796
F-MAMMA1003099//Homo sapiens beta-filamin mRNA, complete cds.//2.6e-42:288:88//AF042166
F-MAMMA1003104//Mus musculus rostral cerebellar malformation protein (rcm) mRNA, complete cds.//1.6e-12:477:64//U72634
F-MAMMA1003113//Mus musculus COP9 complex subunit 7a (COPS7a) mRNA, complete cds.//3.4e-121:789:85//AF071316
F-MAMMA1003127//R.norvegicus MYR1 mRNA for myosin I heavy chain.//9.4e-58:423:83//X68199
F-MAMMA1003135//Mus musculus dentin sialophosphoprotein precursor (DSPP) mRNA, complete cds.//0.62:676:58//U67916
F-MAMMA1003140
F-MAMMA1003146//Homo sapiens mRNA for GalT3 protein.//2.2e-80:397:97//Y15062
F-MAMMA1003150//Human DNA sequence *** SEQUENCING IN PROGRESS *** from clone 598F2, WORKING DRAFT SEQUENCE.//7.3e-123:266:88//AL021579
F-MAMMA1003166//Human DNA sequence *** SEQUENCING IN PROGRESS *** from clone 250D10, WORKING DRAFT SEQUENCE.//1.6e-33:143:82//Z99716
F-NT2RM1000001//Human DNA sequence from clone 393P23 on chromosome Xq21.1-21.33. Contains GSSs, complete sequence.//0.50:216:61//Z95400
F-NT2RM1000018//Human mRNA for KIAA0066 gene, partial cds.//4.8e-65:385:92//D31886
F-NT2RM1000032
F-NT2RM1000035//Cricetulus griseus SREBP cleavage activating protein (SCAP) mRNA, complete cds.//6.3e-135:565:84//U67060
F-NT2RM1000037//Homo sapiens mRNA for KIAA0690 protein, partial cds.//1.1 e-106:542:95//AB014590
F-NT2RM1000039//Mouse genetic suppressor element mRNA.//0.080:239:60//L27155
F-NT2RM1000055//Rattus norvegicus mRNA for TIP120, complete cds.//8.4e-96:535:91//D87671
F-NT2RM1000059//Human DNA sequence *** SEQUENCING IN PROGRESS *** from clone 390E6, WORKING DRAFT SEQUENCE.//1.0:257:59//AL031600
F-NT2RM1000062//Nephila clavipes dragline silk protein spidroin 1 gene, partial cds.//0.54:306:63//U37520
F-NT2RM1000080//Sequence 2 from patent US 5763589.//1.5e-115:566:97//AR012692
F-NT2RM1000086//Homo sapiens mRNA for KIAA0661 protein, complete cds.//1.8e-114:550:97//AB014561
F-NT2RM1000092//Homo sapiens chromosome 19, cosmid R26894, complete sequence.//0.63:180:65//AC005594
F-NT2RM1000118//Homo sapiens clone 23763 unknown mRNA, partial cds.//0.027:126:70//AF007155
F-NT2RM1000119//Human DNA sequence *** SEQUENCING IN PROGRESS *** from clone 466N1, WORKING DRAFT SEQUENCE.//0.022:644:58//Z97630
F-NT2RM1000127//RPCI11-44E5.TJ RPCI11 Homo sapiens genomic clone R-44E5, genomic survey sequence.//1.6e-45:254:94//AQ195884
F-NT2RM1000131//Homo sapiens mRNA for KIAA0792 protein, complete cds.//5.5e-153:778:95//AB018335
F-NT2RM1000132//Homo sapiens NADH:ubiquinone oxidoreductase NDUFS6 subunit mRNA, nuclear gene encoding mitochondrial protein, complete cds.//1.1e-90:448:97//AF044959
F-NT2RM1000153//Human NotI linking clone 924A081D, genomic survey sequence.//5.9e-07:66:96//U49890
F-NT2RM1000186//Homo sapiens clone 23763 unknown mRNA, partial cds.//0.025:126:70//AF007155
F-NT2RM1000187//CITBI-E1-2510J4.TR CITBI-E1 Homo sapiens genomic clone 2510J4, genomic survey sequence.//1.1e-05:56:98//AQ261184
F-NT2RM1000199//Mouse mRNA for seizure-related gene product 6 type 2 precursor, complete cds.//1.6e-38:711:65//D64009
F-NT2RM1000242
F-NT2RM1000244//HS_2229_A1_C04_MF CIT Approved Human Genomic Sperm Library D Homo sapiens genomic clone Plate=2229 Col=7 Row=E, genomic survey sequence.//2.0e-13:95:95//AQ298474
F-NT2RM1000252//Homo sapiens chromosome 17, clone hRPK.206_C_20, complete sequence.//0.023:225:61//AC006070
F-NT2RM1000256//Caenorhabditis elegans cosmid F22B3, complete sequence.//8.5e-24:473:64//Z68336
F-NT2RM1000257//Homo sapiens MAGOH mRNA, complete cds.//6.4e-69:455:85//AF035940
F-NT2RM1000260//Human mRNA for KIAA0130 gene, complete cds.//6.5e-57:460:80//D50920
F-NT2RM1000271
F-NT2RM1000272
F-NT2RM1000280//Bos gaurus vacuolar H-ATPase subunit D (VATD) mRNA, complete cds.//6.7e-97:430:92//U11927
F-NT2RM1000300//Human DNA sequence *** SEQUENCING IN PROGRESS *** from clone 92N15, WORKING DRAFT SEQUENCE.//2.1e-96:170:100//Z93097
F-NT2RM1000314//Human mRNA for KIAA0159 gene, complete cds.//8.1e-127:708:92//D63880
F-NT2RM1000318//Homo sapiens mRNA for ribosomal protein L39, complete cds.//5.7e-34:182:99//D79205
F-NT2RM1000341//Homo sapiens full-length insert cDNA clone YP11F06.//1.3e-100:504:97//AF085879
F-NT2RM1000354//HS_2001_B1_E06_T7 CIT Approved Human Genomic Sperm Library D Homo sapiens genomic clone Plate=2001 Col=11 Row=J, genomic survey sequence.//1.6e-11:201:73//AQ218494
F-NT2RM1000355//Mus musculus E25B protein mRNA, complete cds.//1.8e-77:578:82//U76253
F-NT2RM1000365//Homo sapiens clone DJ0098022, WORKING DRAFT SEQUENCE, 5 unordered pieces.//9.4e-113:367:97//AC004821
F-NT2RM1000377//H.sapiens mRNA for MAP kinase phosphatase 4.//6.1e-14:362:62//Y08302
F-NT2RM1000388//Azospirillum brasilense lateral flagellin (laf1) gene, complete cds.//1.0:482:58//U26679
F-NT2RM1000394//M.musculus mRNA for histone H3.3A.//1.7e-94:549:89//Z85979
F-NT2RM1000399
F-NT2RM1000421//HS_2213_B1_E01_MF CIT Approved Human Genomic Sperm Library D Homo sapiens genomic clone Plate=2213 Col=1 Row=J, genomic survey sequence.//3.6e-08:195:72//AQ032737
F-NT2RM1000430//Homo sapiens erythroblast macrophage protein EMP mRNA, complete cds.//3.7e-84:418:97//AF084928
F-NT2RM1000499//Human mRNA for KIAA0167 gene, complete cds.//1.3e-35:525:69//D79989
F-NT2RM1000539//Homo sapiens PAC clone DJ1194E14 from 7p21, complete sequence.//4.6e-73:533:83//AC004993
F-NT2RM1000553
F-NT2RM1000555//Homo sapiens clone 24514 unknown mRNA.//2.3e-110:555:97//AF070542
F-NT2RM1000563//Homo sapiens clone DJ0742P04, WORKING DRAFT SEQUENCE, 6 unordered pieces.//1.3e-123:477:100//AC004873
F-NT2RM1000623//HS_2213_B1_E01_MF CIT Approved Human Genomic Sperm Library D Homo sapiens genomic clone Plate=2213 Col=1 Row=J, genomic survey sequence.//8.2e-06:75:89//AQ032737
F-NT2RM1000648//Halobium cutirubrum L11, L1, L10 and L12 equivalent ribosomal protein gene cluster.//1.3e-06:414:61//X15078
F-NT2RM1000661//Homo sapiens cap-binding protein 4EHP mRNA, complete cds.//9.3e-54:275:97//AF047695
F-NT2RM1000666//HS_2016_B2_H08_MR CIT Approved Human Genomic Sperm Library D Homo sapiens genomic clone Plate=2016 Col=16 Row=P, genomic survey sequence.//5.7e-13:199:73//AQ227865
F-NT2RM1000669//Human DNA sequence from clone 281H8 on chromosome 6q25.1-25.3. Contains up to four novel genes, one with similarity to KIAA0323 and worm C30F12.1 and another with Ubiquitin-Like protein gene SMT3 (the latter in an intron of a novel gene). Contains ESTs, STSs, GSSs, a putative CpG island and genomic marker D6S1553, complete sequence.//2.7e-94:499:94//AL031133
F-NT2RM1000672
F-NT2RM1000691//Homo sapiens HRIHFB2060 mRNA, partial cds.//2.2e-119:582:98//AB015348
F-NT2RM1000699//Caenorhabditis elegans cosmid Y41C4A, complete sequence.//0.95:284:61//AL032627
F-NT2RM1000702//HS_3005_A1_A02_MR CIT Approved Human Genomic Sperm Library D Homo sapiens genomic clone Plate=3005 Col=3 Row=A, genomic survey sequence.//0.073:290:58//AQ089514
F-NT2RM1000725//Homo sapiens mRNA for neuropathy target esterase.//4.8e-65:435:85//AJ004832
F-NT2RM1000741//Homo sapiens mRNA for KIAA0567 protein, partial cds.//8.0e-126:690:92//AB011139
F-NT2RM1000742//Homo sapiens AC133 antigen mRNA, complete cds.//2.5e-66:524:83//AF027208
F-NT2RM1000746//Homo sapiens chromosome 21q22.3, PAC clones 314N7, 225L15, BAC clone 7B7, complete sequence bases 1.333303.//0.92:395:58//AJ011930
F-NT2RM1000770//Homo sapiens inosine monophosphate dehydrogenase type II gene, complete cds.//2.1e-70:407:92//L39210
F-NT2RM1000772//Human Chromosome 3 pac pDJ70i11, WORKING DRAFT SEQUENCE, 2 unordered pieces.//6.6e-36:98:93//AC000380
F-NT2RM1000780//Human DNA for 5' terminal region of LINE-1 transposable element clone CGL1-4.//9.3e-22:126:99//X52233
F-NT2RM1000781//Plasmodium falciparum 3D7 chromosome 12 PFYAC812 genomic sequence, WORKING DRAFT SEQUENCE, 8 unordered pieces.//7.1e-09:540:59//AC004153
F-NT2RM1000800//Mus musculus mRNA for B-IND1 protein.//4.0e-81:497:88//Z97207
F-NT2RM1000802
F-NT2RM1000811//Homo sapiens AC133 antigen mRNA, complete cds.//3.7e-63:490:84//AF027208
F-NT2RM1000826//Homo sapiens clone 24514 unknown mRNA.//7.2e-153:749:96//AF070542
F-NT2RM1000829//HS_3047_A1_A05_MF CIT Approved Human Genomic Sperm Library D Homo sapiens genomic clone Plate=3047 Col=9 Row=A, genomic survey sequence.//0.74:215:67//AQ099134
F-NT2RM1000833//Canis familiaris sec61 homologue mRNA, complete cds.//5.1e-114:683:88//M96629
F-NT2RM1000850//F.rubripes GSS sequence, clone 163A22aF11, genomic survey sequence.//1.1e-26:279:74//AL018762
F-NT2RM1000852//Homo sapiens mRNA for ATP-dependent RNA helicase, partial.//9.3e-148:726:97//AJ010840
F-NT2RM1000857//Rattus norvegicus gene for cytochrome P450/6 beta B, exon 2.//0.97:124:65//AB008378
F-NT2RM1000867//H.sapiens DNA sequence surrounding NotI site, clone NRLA143D.//1.2e-31:172:98//K95834
F-NT2RM1000874//Homo sapiens KE05 protein mRNA, complete cds.//2.8e-131:632:97//AF064605
F-NT2RM1000882//Homo sapiens Chromosome 11q12.2 PAC clone pDJ519o13 containing human gene for ferritin heavy chain (FTH), complete sequence.//1.2e-98:214:99//AC004228
F-NT2RM1000883//Homo sapiens I-1 receptor candidate protein mRNA, complete cds.//2.7e-156:762:97//AF082516
F-NT2RM1000885//Homo sapiens mRNA for KIAA0661 protein, complete cds.//2.0e-17:310:67//AB014561
F-NT2RM1000894//Mus musculus second largest subunit of RNA polymerase I (RPA2) mRNA, complete cds.//3.2e-95:469:83//U58280
F-NT2RM1000898
F-NT2RM1000905//Human DNA sequence *** SEQUENCING IN PROGRESS *** from clone 466N1, WORKING DRAFT SEQUENCE.//1.8e-74:188:98//Z97630
F-NT2RM1000924//Homo sapiens clone DJ0742P04, WORKING DRAFT SEQUENCE, 6 unordered pieces.//5.7e-148:601:98//AC004873
F-NT2RM1000927//Homo sapiens clone DJ0647C14, WORKING DRAFT SEQUENCE, 21 unordered pieces.//0.071:392:60//AC004846
F-NT2RM1000962//H.sapiens CpG island DNA genomic Mse1 fragment, clone 140d1, forward read cpg140d1.ft1a.//4.1e-35:187:99//Z56803
F-NT2RM1000978//Homo sapiens Chromosome 15q22.3-23 PAC 88m3, WORKING DRAFT SEQUENCE, 2 ordered pieces.//1.1e-23:266:77//AC005959
F-NT2RM1001003//Homo sapiens alpha-catenin-like protein mRNA, complete cds.//4.0e-160:760:98//U97067
F-NT2RM1001008//Kaposi's sarcoma-associated herpes-like virus ORF73 homolog gene, complete cds.//1.7e-11:602:61//U52064
F-NT2RM1001043//Human DNA sequence from PAC 27K14 on chromosome Xp11.3-Xp11.4. Contains monoamine oxidase B (MAOB), ESTs and polymorphic CA repeats.//3.9e-93:645:86//Z95125
F-NT2RM1001044//S.pombe chromosome III cosmid c320.//0.90:128:66//AL022245
F-NT2RM1001059//Homo sapiens chromosome 5, Bac clone 58g14 (LBNL H76), complete sequence.//3.8e-53:261:80//AC005915
F-NT2RM1001066//CIT-HSP-2172N17.TF CIT-HSP Homo sapiens genomic clone 2172N17, genomic survey sequence.//0.64:285:59//B94391
F-NT2RM1001072//HS_3115_B1_D07_T7 CIT Approved Human Genomic Sperm Library D Homo sapiens genomic clone Plate=3115 Col=13 Row=H, genomic survey sequence.//7.3e-23:140:95//AQ147905
F-NT2RM1001074//Homo sapiens chromosome 19, cosmid F20489, complete sequence.//5.0e-50:186:98//AC005263
F-NT2RM1001082//Sequence 1 from Patent WO9718303.//2.1e-144:736:95//A62731
F-NT2RM1001085//CIT-HSP-2310F21.TR CIT-HSP Homo sapiens genomic clone 2310F21, genomic survey sequence.//8.8e-45:235:97//AQ020757
F-NT2RM1001092//HS_3055_B1_G05_MF CIT Approved Human Genomic Sperm Library D Homo sapiens genomic clone Plate=3055 Col=9 Row=N, genomic survey sequence.//1.1e-89:471:95//AQ155489
F-NT2RM1001102//Human HEM45 mRNA, complete cds.//1.2e-28:482:63//U88964
F-NT2RM1001105//Homo sapiens hRED1 gene, exon 1 (5'UTR).//0.0014:349:61//Z95973
F-NT2RM1001112//Homo sapiens chromosome 19, cosmid R34094, complete sequence.//0.060:429:58//AC004678
F-NT2RM1001115//Plasmodium falciparum merozoite surface protein 3 (MSP-3) gene, partial cds.//0.93:156:62//AF024624
F-NT2RM1001139//Homo sapiens chromosome 19, fosmid 37502, complete sequence.//1.2e-10:466:59//AC004755
F-NT2RM2000006//Human DNA sequence *** SEQUENCING IN PROGRESS *** from clone 796F18, WORKING DRAFT SEQUENCE.//5.3e-150:724:98//AL031291
F-NT2RM2000013//D.melanogaster DmRP128 gene for RNA polymerase III second-largest subunit.//1.5e-58:749:69//X58826
F-NT2RM2000030//Homo sapiens clone DJ0708P22, WORKING DRAFT SEQUENCE, 11 unordered pieces.//2.1e-97:270:77//AC004863
F-NT2RM2000032//Human DNA sequence *** SEQUENCING IN PROGRESS *** from clone 423B22, WORKING DRAFT SEQUENCE.//1.9e-25:172:76//AL034379
F-NT2RM2000042//Human DNA sequence from cosmid U55E4, between markers DXS6791 and DXS8038 on chromosome X contains ESTs.//5.0e-05:325:65//Z73418
F-NT2RM2000092//Homo sapiens (D8S321 locus) DNA sequence, tetranucleotide repeat polymorphism.//0.63:117:68//L12269
F-NT2RM2000093//Mus musculus major histocompatibility locus class III regions Hsc70t gene, partial cds; smRNP, G7A, NG23, MutS homolog, CLCP, NG24, NG25, and NG26 genes, complete cds; and unknown genes.//0.38:312:62//AF109905
F-NT2RM2000101
F-NT2RM2000124//Mouse cAMP-dependent protein kinase catalytic subunit mRNA, complete cds.//3.8e-58:297:97//M12303
F-NT2RM2000191//Homo sapiens cGMP phosphodiesterase A2 (PDE9A) mRNA, complete cds.//3.8e-138:653:98//AF067224
F-NT2RM2000192//CIT-HSP-2172B3.TF CIT-HSP Homo sapiens genomic clone 2172B3, genomic survey sequence.//2.2e-33:191:95//B93289
F-NT2RM2000239//F rubripes GSS sequence, clone 156P04aG12, genomic survey sequence.//8.9e-44:445:69//AL018549
F-nnnnnnnnnnnn//Homo sapiens fibroblast growth factor 18 (FGF18) mRNA, complete cds.//0.00020:380:61//AF075292
F-NT2RM2000250//Homo sapiens mRNA for KIAA0590 protein, complete cds.//3.1e-128:615:98//AB011162
F-NT2RM2000259//Human DNA sequence *** SEQUENCING IN PROGRESS *** from clone 310O13, WORKING DRAFT SEQUENCE.//0.0013:305:63//AL031658
F-NT2RM2000260//Mus musculus WW domain binding protein 15 mRNA, partial sequence.//3.0e-14:645:61//AF073934
F-NT2RM2000287//*** SEQUENCING IN PROGRESS *** EPM1/APECED region of chromosome 21, clones A68E8, B127P21, B173L3, B23N8, C1242C9, C579E2, A70B6, B159G9, B175D10, B52C10, C124G1 Note: Sequencing in this region has been discontinued by the Stanford Human Genome Center, WORKING DRAFT SEQUENCE, 50 unordered pieces.//1.3e-11:96:86//AC003656
F-NT2RM2000322//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//8.5e-115:233:97//AL031864
F-NT2RM2000359//Homo sapiens mRNA for KIAA0560 protein, complete cds.//8.8e-175:805:99//AB011132
F-NT2RM2000363//RPCI11-90B10.TJ RPCI11 Homo sapiens genomic clone R-90B10, genomic survey sequence.//6.7e-15:96:98//AQ285300
F-NT2RM2000368//Homo sapiens protein kinase C-binding protein RACK7 mRNA, partial cds.//1.2e-94:599:86//U48251
F-NT2RM2000371//RPCI11-57I4.TJ RPCI11 Homo sapiens genomic clone R-57I4, genomic survey sequence.//1.1e-52:312:91//AQ083343
F-NT2RM2000374//M. musculus nodal gene, a TGF-beta-like gene.//6.7e-31:196:91//X70514
F-NT2RM2000395//Leishmania major chromosome 1, complete sequence.//0.99:345:58//AE001274
F-NT2RM2000402//Arabidopsis thaliana BAC T19D16 genomic sequence.//2.1e-23:414:63//U95973
F-NT2RM2000407//Mus musculus semaphorin VIa mRNA, complete cds.//1.4e-131:439:88//AF030430
F-NT2RM2000420//HS_3063_B2_F11_MF CIT Approved Human Genomic Sperm Library D Homo sapiens genomic clone Plate=3063 Col=22 Row=L, genomic survey sequence.//3.2e-25:154:95//AQ103204
F-NT2RM2000422//Rat orphan transporter v7-3 (NTT73) mRNA, complete cds.//1.7e-128:782:86//L22022
F-NT2RM2000452//HS_3009_B2_D05_MR CIT Approved Human Genomic Sperm Library D Homo sapiens genomic clone Plate=3009 Col=10 Row=H, genomic survey sequence.//1.2e-16:122:90//AQ130794
F-NT2RM2000469//HS_2019_A1_G02_MR CIT Approved Human Genomic Sperm Library D Homo sapiens genomic clone Plate=2019 Col=3 Row=M, genomic survey sequence.//9.6e-22:176:85//AQ229041
F-NT2RM2000490//Homo sapiens mRNA for KIAA0747 protein, partial cds.//7.5e-15:386:63//AB018290
F-NT2RM2000502
F-NT2RM2000504//Homo sapiens metalloprotease 1 (MP1) mRNA, complete cds.//5.1e-171:824:97//AF061243
F-NT2RM2000522
F-NT2RM2000540
F-NT2RM2000556//Homo sapiens 12q13.1 PAC RPCI5-1057I20 (Roswell Park Cancer Institute Human PAC library) complete sequence.//2.9e-42:344:82//AC004466
F-NT2RM2000566//Homo sapiens integrin alpha-7 mRNA, complete cds.//2.8e-154:751:97//AF072132
F-NT2RM2000567//Pseudomonas aeruginosa enoyl-CoA hydratase gene, partial cds; pilin biosynthetic protein (fimL) gene, complete cds; and unknown gene.//3.0e-06:664:58//AF083252
F-NT2RM2000569//Human DNA sequence *** SEQUENCING IN PROGRESS *** from clone 862K6, WORKING DRAFT SEQUENCE.//1.3e-15:348:67//AL031681
F-NT2RM2000577//RPCI11-43G22.TJ RPCI11 Homo sapiens genomic clone R-43G22, genomic survey sequence.//1.6e-14:155:80//AQ199391
F-NT2RM2000581//Homo sapiens mRNA for KIAA0214 protein, complete cds.//5.4e-174:820:98//D86987
F-NT2RM2000588//Homo sapiens 12q13.1 PAC RPCI5-1057I20 (Roswell Park Cancer Institute Human PAC library) complete sequence.//1.1e-60:344:82//AC004466
F-NT2RM2000594//Mus musculus DNA cytosine-5 methyltransferase 3B1 (Dnmt3b) mRNA, alternatively spliced, complete cds.//4.9e-118:761:85//AF068626
F-NT2RM2000599//O.sativa osr40g3 gene.//0.30:585:56//Y08988
F-NT2RM2000609
F-NT2RM2000612//Rattus norvegicus ADP-ribosylation factor-directed GTPase activating protein mRNA, complete cds.//7.8e-102:709:83//U35776
F-NT2RM2000623//Homo sapiens chromosome 19, cosmid F19847, complete sequence.//3.4e-17:450:65//AC005952
F-NT2RM2000624
   2.9e-06:231:64//Z82061
F-NT2RM2000635//Homo sapiens mRNA for KIAA0729 protein, partial cds.//6.3e-142:664:98//AB018272
F-NT2RM2000636//Homo sapiens mRNA for KIAA0658 protein, partial cds.//7.4e-138:664:98//AB014558
F-NT2RM2000639//RPCI11-69E5.TJ RPCI11 Homo sapiens genomic clone R-69E5, genomic survey sequence.//3.7e-14:97:97//AQ267491
F-NT2RM2000649//Homo sapiens mRNA for KIAA0676 protein, partial cds.//1.1e-167:518:99//AB014576
F-NT2RM2000669
F-NT2RM2000691//Homo sapiens chromosome 2 clone 101B6 map 2p11, complete sequence.//1.1e-106:748:82//AC002038
F-NT2RM2000714//Human mRNA for KIAA0231 gene, partial cds.//6.8e-49:748:64//D86984
F-NT2RM2000718//Homo sapiens HRIHFB2436 mRNA, partial cds.//2.4e-124:594:98//AB015342
F-NT2RM2000735//Human ZNF43 mRNA.//8.4e-111:756:82//X59244
F-NT2RM2000740//Mus musculus lymphocyte specific helicase mRNA, complete cds.//1.3e-141:815:89//U25691
F-NT2RM2000795//Human DNA sequence *** SEQUENCING IN PROGRESS *** from clone 439F8, WORKING DRAFT SEQUENCE.//1.0e-78:723:76//AL021392
F-NT2RM2000821//Rat mRNA for beta COP.//2.0e-150:879:88//X57228
F-NT2RM2000837//Homo sapiens BAC clone GS214N13 from 7p14-p15, complete sequence.//1.1e-05:361:62//AC005017
F-NT2RM2000951//Homo sapiens XYLB mRNA for xylulokinase, complete cds.//8.7e-184:847:99//AB015046
F-NT2RM2000952
F-NT2RM2000984//Mus musculus major histocompatibility locus class III regions Hsc70t gene, partial cds; smRNP, G7A, NG23, MutS homolog, CLCP, NG24, NG25, and NG26 genes, complete cds; and unknown genes.//7.6e-41:239:76//AF109905
F-NT2RM2001004//CIT-HSP-2333N18.TR CIT-HSP Homo sapiens genomic clone 2333N18, genomic survey sequence.//1.1e-11:298:66//AQ035862
F-NT2RM2001035//Mus musculus mCAF1 protein mRNA, complete cds.//1.4e-120:627:91//U21855
F-NT2RM2001065//Mus musculus COP9 complex subunit 4 (COPS4) mRNA, complete cds.//6.8e-118:690:88//AF071314
F-NT2RM2001100//Homo sapiens clone DJ0742P04, WORKING DRAFT SEQUENCE, 6 unordered pieces.//2.3e-145:614:99//AC004873
F-NT2RM2001105//Human DNA sequence *** SEQUENCING IN PROGRESS *** from clone 50O24, WORKING DRAFT SEQUENCE.//2.7e-95:461:99//AL034380
F-NT2RM2001131//Kaposi's sarcoma-associated herpes-like virus ORF73 homolog gene, complete cds.//7.2e-24:726:62//U52064
F-NT2RM2001141
F-NT2RM2001152//Homo sapiens DNA sequence from PAC 93L7 on chromosome Xq21. Contains part of the CHM (TCD, REP1) gene coding for RAB Escort protein 1 (REP-1, RAB proteins geranylgeranyltransferase component A 1, Choroideraemia protein, Tapetochoroidal Dystrophy (TCD) protein). Contains ESTs and an STS, complete sequence.//0.98:300:62//AL022401
F-NT2RM2001177//Homo sapiens clone NH0313P13, WORKING DRAFT SEQUENCE, 15 unordered pieces.//1.2e-147:741:96//AC005488
F-NT2RM2001194//Suid herpesvirus 1 UL5 gene, partial cds, UL6 and UL7 genes, complete cds, UL8 gene, partial cds.//0.026:408:59//U66829
F-NT2RM2001196//Homo sapiens clone DJ1173I20, WORKING DRAFT SEQUENCE, 5 unordered pieces.//2.2e-135:627:98//AC004987
F-NT2RM2001201//Mus musculus clone OST431, genomic survey sequence.//6.1e-80:503:86//AF046700
F-NT2RM2001221//Chimpanzee (P.paniscus) involucrin, complete cds.//0.53:670:55//M26514
F-NT2RM2001238//Rat glutaminase mRNA, complete cds.//3.4e-128:719:90//M65150
F-NT2RM2001243
F-NT2RM2001247//CITBI-E1-2521M18.TR CITBI-E1 Homo sapiens genomic clone 2521M18, genomic survey sequence.//0.0011:274:59//AQ276184
F-NT2RM2001256//M.musculus mRNA for 200 kD protein.//2.3e-129:742:90//X80169
F-NT2RM2001291//CIT-HSP-2010I15.TR CIT-HSP Homo sapiens genomic clone 2010I15, genomic survey sequence.//4.6e-09:156:72//B57734
F-NT2RM2001306//RPCI11-28I5.TP RPCI-11 Homo sapiens genomic clone RPCI-11-28I5, genomic survey sequence.//0.069:234:64//B84850
F-NT2RM2001312//Homo sapiens chromosome 17, clone hRPK.142_H_19, complete sequence.//1.1e-22:111:81//AC005919
F-NT2RM2001319//Borrelia burgdorferi (section 4 of 70) of the complete genome.//0.99:340:58//AE001118
F-NT2RM2001324//Human DNA sequence *** SEQUENCING IN PROGRESS *** from clone 209H1, WORKING DRAFT SEQUENCE.//3.7e-44:340:85//Z84465
F-NT2RM2001345//HS_3005_A1_A02_MR CIT Approved Human Genomic Sperm Library D Homo sapiens genomic clone Plate=3005 Col=3 Row=A, genomic survey sequence.//0.042:290:58//AQ089514
F-NT2RM2001360//Human HeLa mRNA isolated as a false positive in a two-hybrid-screen.//5.0e-60:365:87//U56429
F-NT2RM2001370//Homo sapiens PAC clone DJ0815D20 from 7p11-p13, complete sequence.//0.98:415:58//AC004899
F-NT2RM2001393//Homo sapiens Chromosome 22q11.2 PAC Clone p_m11 In BCRL2-GGT Region, complete sequence.//4.0e-54:394:75//AC004033
F-NT2RM2001420//Human DNA sequence *** SEQUENCING IN PROGRESS *** from clone 349A12, WORKING DRAFT SEQUENCE.//2.8e-169:789:99//AL033520
F-NT2RM2001424//Homo sapiens mRNA for E1B-55kDa-associated protein.//7.1e-96:453:99//AJ007509
F-NT2RM2001499//Rattus norvegicus mRNA for cationic amino acid transporter 3, complete cds.//7.1e-91:601:83//AB000113
F-NT2RM2001504//Homo sapiens chromosome 19, cosmid R30017, complete sequence.//0.81:200:69//AC005624
F-NT2RM2001524//Arabidopsis thaliana DNA chromosome 4, ESSA I AP2 contig fragment No. 2.//3.8e-16:316:65//Z99708
F-NT2RM2001544
F-NT2RM2001547//Caenorhabditis elegans cosmid Y47H9C, complete sequence.//3.3e-24:318:67//AL032657
F-NT2RM2001575//Human 52-kD ribonucleoprotein Ro/SSA mRNA, complete cds.//2.1e-26:582:64//M34551
F-NT2RM2001582//M.musculus red-1 gene.//1.4e-102:581:90//X92750
F-NT2RM2001588//Homo sapiens KIAA0442 mRNA, partial cds.//7.0e-10:282:65//AB007902
F-NT2RM2001592//Rattus norvegicus rexo70 mRNA, complete cds.//9.6e-131:736:90//AF032667
F-NT2RM2001605//RBP2=retinoblastoma binding protein 2 [human, Nalm-6 pre-B cell leukemia, mRNA, 6455 nt].//2.3e-85:749:75//S66431
F-NT2RM2001613//Rattus rattus sec61 homologue mRNA, complete cds.//8.6e-118:779:85//M96630
F-NT2RM2001632//Homo sapiens PAC clone DJ0740D02 from 7p14-p15, complete sequence.//1.5e-50:561:71//AC004691
F-NT2RM2001635//Homo sapiens mRNA for KIAA0618 protein, complete cds.//9.2e-153:740:98//AB014518
F-NT2RM2001637//F.rubripes GSS sequence, clone 155D22bD8, genomic survey sequence.//2.5e-13:224:64//Z91020
F-NT2RM2001641//CIT-HSP-2347F23.TF CIT-HSP Homo sapiens genomic clone 2347F23, genomic survey sequence.//1.3e-67:340:98//AQ060913
F-NT2RM2001648//Canis familiaris sec61 homologue mRNA, complete cds.//1.4e-110:459:89//M96629
F-NT2RM2001652//Bos taurus guanine nucleotide-exchange protein (ARF-GEP1) mRNA, complete cds.//1.2e-153:807:93//AF023451
F-NT2RM2001659//nbxb0002cE07f CUGI Rice BAC Library Oryza sativa genomic clone nbxb0002J13f, genomic survey sequence.//1.0:485:56//AQ051653
F-NT2RM2001664//Homo sapiens IkappaB kinase complex associated protein (IKAP) mRNA, complete cds.//3.7e-172:802:99//AF044195
F-NT2RM2001668
F-NT2RM2001670//Homo sapiens complete genomic sequence between D16S3070 and D16S3275, containing Familial Mediterranean Fever gene disease.//3.2e-18:279:70//AJ003147
F-NT2RM2001671//Oryctolagus cuniculus sarcolemmal associated protein-3 mRNA; complete cds.//1.6e-137:683:94//U21157
F-NT2RM2001675//RPCI11-51J16.TJ RPCI11 Homo sapiens genomic clone R-51J16, genomic survey sequence.//1.0:394:58//AQ053677
F-NT2RM2001681//Arabidopsis thaliana DNA chromosome 4, BAC clone T8O5 (ESSAII project).//0.87:220:61//AL021890
F-NT2RM2001688//B.parapertussis bvg locus (transcription regulators of virulence factors) with bvgA and bvgS genes.//1.0:286:62//X52948
F-NT2RM2001695//CIT-HSP-345H13.TVB CIT-HSP Homo sapiens genomic clone 345H13, genomic survey sequence.//3.2e-53:241:82//B59854
F-NT2RM2001696//Mouse DNA with homology to EBV IR3 repeat, segment 2, clone Mu2.//1.2e-05:306:58//M10668
F-NT2RM2001698//Homo sapiens DNA sequence from PAC 163M9 on chromosome 1p35.1-p36.21. Contains protein synthesis factor (eIF-4C), D1F15S1A pseudogene, ESTs, STS, GSS, complete sequence.//6.0e-06:548:59//AL021920
F-NT2RM2001699//HS_3195_8B2_DO1_T7 CIT Approved Human Genomic Sperm Library D Homo sapiens genomic clone Plate=3195 Col=2 Row=H, genomic survey sequence.//2.7e-07:322:61//AQ189056
F-NT2RM2001700//Mycobacterium tuberculosis H37Rv complete genome; segment 109/162.//7.8e-05:354:58//Z95556
F-NT2RM2001706//Homo sapiens chromosome Xp22-67-68, WORKING DRAFT SEQUENCE, 99 unordered pieces.//7.5e-42:335:81//AC004469
F-NT2RM2001716
F-NT2RM2001718//Drosophila melanogaster DNA sequence (P1 DS04106 (D172)), complete sequence.//4.2e-08:536:58//AC004290
F-NT2RM2001723//Homo sapiens clone 23770 mRNA sequence.//1.4e-26:163:95//AF052123
F-NT2RM2001727//Homo sapiens mRNA for KIAA0462 protein, partial cds.//6.2e-111:530:98//AB007931
F-NT2RM2001730//Homo sapiens chromosome 21 PAC RPCIP704E14135Q2.//3.1e-102:248:95//AJ010598
F-NT2RM2001743
F-NT2RM2001753//Caenorhabditis elegans cosmid F45E6, complete sequence.//0.11:138:66//Z68117
F-NT2RM2001760//Canis familiaris sec61 homologue mRNA, complete cds.//9.4e100:418:88//M96629
F-NT2RM2001768//HS_3064_B2_A04_MF CIT Approved Human Genomic Sperm Library D Homo sapiens genomic clone Plate=3064 Col=8 Row=B, genomic survey sequence.//3.1e-28:153:100//AQ136993
F-NT2RM2001771//Homo sapiens chromosome 19, BAC CIT-B-393i15 (BC301323), complete sequence.//1.3e-66:680:72//AC006116
F-NT2RM2001782
F-NT2RM2001784//Bovine herpesvirus type 1 (Cooper) DNA (30 kb).//0.027:384:60//Z48053
F-NT2RM2001785//Homo sapiens chromosome 11, BAC CIT-HSP-311e8 (BC269730) containing the hFEN1 gene, complete sequence.//1.6e-18:229:65//AC004770
F-NT2RM2001797//HS_3045_AT_D01_MF CIT Approved Human Genomic Sperm Library D Homo sapiens genomic clone Plate=3045 Col=1 Row=G, genomic survey sequence.//1.4e-74:381:97//AQ129456
F-NT2RM2001800
F-NT2RM2001803//Homo sapiens IkappaB kinase complex associated protein (IKAP) mRNA, complete cds.//8.3e-178:827:99//AF044195
F-NT2RM2001805//Malus domestica leucine-rich receptor-like protein kinase (LRPKm1) gene, 5' flanking region and 5' UTR.//1.0:290:58//AF053126
F-NT2RM2001813//CIT-HSP-2169F21.TR CIT-HSP Homo sapiens genomic clone 2169F21, genomic survey sequence.//3.3e-16:109:95//B89870
F-NT2RM2001823//Drosophila melanogaster DNA sequence (P1 DS07049 (D133)), complete sequence.//5.8e-62:819:68//AC004274
F-NT2RM2001839//Homo sapiens calumein (Calu) mRNA, complete cds.//3.6e-131:738:90//AF013759
F-NT2RM2001840//Homo sapiens chromosome 17, clone 297N7, complete sequence.//1.1e-57:422:79//AC002347
F-NT2RM2001855//HS_3224_A1_H07_MR CIT Approved Human Genomic Sperm Library D Homo sapiens genomic clone Plate=3224 Col=13 Row=O, genomic survey sequence.//0.00012:68:91//AQ205285
F-NT2RM2001867//Human DNA sequence from clone 889N15 on chromosome Xq22.1-22.3. Contains part of the gene for a novel protein similar to X. laevis Cortical Thymocyte Marker CTX, the possibly alternatively spliced gene for 26S Proteasome subunit p28 (Ankyrin repeat protein), a novel gene and exons 36 through 45 of the COL4A6 for Collagen Alpha 6(IV). Contains ESTs, STSs, GSSs and a putative CpG island, complete sequence.//0.068:102:70//AL031177
F-NT2RM2001879//Human DNA sequence from cosmid cU72E5, between markers DXS366 and DXS87 on chromosome X.//0.0029:500:59//Z68328
F-NT2RM2001886//Homo sapiens mRNA for KIAA0710 protein, complete cds.//1.9e-187:866:97//AB014610
F-NT2RM2001896//S.cerevisiae chromosome III complete DNA sequence.//8.6e-30:613:63//X59720
F-NT2RM2001903//Homo sapiens mRNA for KIAA0462 protein, partial cds.//2.9e-176:859:97//AB007931
F-NT2RM2001930//M.musculus mRNA for semaphorin G.//4.7e-117:730:85//X97818
F-NT2RM2001935//Sequence 11 from Patent WO9610637.//1.0:356:60//A50028
F-NT2RM2001936//Homo sapiens clone 614 unknown mRNA, complete sequence.//6.9e-138:653:98//AF091080
F-NT2RM2001950//RPCI11-24L12.TP RPCI-11 Homo sapiens genomic clone RPCI-11-24L12, genomic survey sequence.//2.7e-19:188:81//B86700
F-NT2RM2001982//Arabidopsis thaliana chromosome II BAC T24I21 genomic sequence, complete sequence.//0.42:179:65//AC005825
F-NT2RM2001983//Homo sapiens RGS-GAIP interacting protein GIPC mRNA, complete cds.//3.8e-20:123:98//AF089816
F-NT2RM2001989//Sequence 3 from patent US 5747317.//1.9e-167:786:98//AR004981
F-NT2RM2001997//Human HepG2 partial cDNA, clone hmd1b08m5.//9.6e-25:160:95//D16955
F-NT2RM2001998//Homo sapiens DNA, chromosome 21q22.2, PAC clone 25P16 complete sequence, encoding carbonyl reductase and carbonyl reductase 3 (complete cds).//0.88:380:60//AB003151
F-NT2RM2002004//Human Chromosome X, complete sequence.//5.0e-88:831:77//AC002407
F-NT2RM2002014
F-NT2RM2002030//Mus musculus glutamine:fructose-6-phosphate amidotransferase mRNA, complete cds.//1.5e-89:822:74//U00932
F-NT2RM2002049//Bovine elastin mRNA, partial cds.//8.8e-11:125:81//M26132
F-NT2RM2002055
F-NT2RM2002088//Mus musculus WW domain binding protein 17 mRNA, partial sequence.//1.4e-15:421:63//AF073936
F-NT2RM2002091//Human DNA sequence *** SEQUENCING IN PROGRESS *** from clone 50O24, WORKING DRAFT SEQUENCE.//4.6e-160:771:98//AL034380
F-NT2RM2002100//Homo sapiens mRNA for ATP-dependent RNA helicase, partial.//7.7e-164:776:98//AJ010840
F-NT2RM2002109//Homo sapiens glioma amplified on chromosome 1 protein (GAC1) mRNA, complete cds.//2.4e-143:684:98//AF030435
F-NT2RM2002128//Mesocricetus auratus guanine nucleotide-binding protein beta 5 (Gnb5) mRNA, complete cds.//7.0e-27:330:73//U13152
F-NT2RM2002142//Danio rerio gastrulation specific (G12) mRNA, complete cds.//6.3e-10:135:80//U27121
F-NT2RM2002145//Homo sapiens erythroblast macrophage protein EMP mRNA, complete cds.//4.2e-143:800:92//AF084928
F-NT2RM2002178//Homo sapiens mRNA for KIAA0467 protein, partial cds.//5.2e-164:787:97//AB007936
F-NT2RM2002580//Drosophila melanogaster DNA sequence (P1 DS02110 (D147)), complete sequence.//7.4e-13:337:62//AC004423
F-NT2RM4000024//D.melanogaster DmRP128 gene for RNA polymerase III second-largest subunit.//1.2e-62:801:70//X58826
F-NT2RM4000027//Caenorhabditis elegans cosmid F09E5.//0.36:336:60//U37429
F-NT2RM4000030//H.sapiens CpG island DNA genomic Mse1 fragment, clone 56h10, forward read cpg56h10.ft1a.//9.3e-22:127:100//Z55685
F-NT2RM4000046//Curcurbita maxima 25S - 18S rDNA intergenic spacer.//4.1e-05:386:60//X13059
F-NT2RM4000061
F-NT2RM4000085//B.taurus mRNA for nuclear DNA helicase II.//1.9e-10:485:59//X82829
F-NT2RM4000086
F-NT2RM4000104//Homo sapiens chromosome 16 zinc finger protein ZNF210 (ZNF210) mRNA, complete cds.//4.2e-23:345:69//AF060865
F-NT2RM4000139//R.norvegicus trg mRNA.//1.4e-56:708:69//X68101
F-NT2RM4000155//CIT-HSP-2282N15.TR CIT-HSP Homo sapiens genomic clone 2282N15, genomic survey sequence.//3.0e-09:88:90//AQ000070
F-NT2RM4000156//H.sapiens HPBRII-7 gene.//2.0e-21:586:60//X67336
F-NT2RM4000167//Mouse kif4 mRNA for microtubule-based motor protein KIF4, complete cds.//2.7e-143:810:90//D12646
F-NT2RM4000169//Plasmodium falciparum 3D7 chromosome 12 PFYAC293 genomic sequence, WORKING DRAFT SEQUENCE, 9 unordered pieces.//0.0054:746:57//AC004157
F-NT2RM4000191//Mus musculus cathepsin S (CatS) gene, promoter region and exons 1 and 2.//0.00018:468:60//AF051726
F-NT2RM4000197
F-NT2RM4000199//Human DNA sequence *** SEQUENCING IN PROGRESS *** from clone 620E11, WORKING DRAFT SEQUENCE.//0.67:461:60//AL031667
F-NT2RM4000200
F-NT2RM4000202//H.sapiens CpG island DNA genomic Mse1 fragment, clone 34c2, forward read cpg34c2.ft1a.//1.7e-27:190:90//Z65361
F-NT2RM4000210//Homo sapiens mRNA for KIAA0712 protein, complete cds.//1.4e-182:856:98//AB018255
F-NT2RM4000215//S.cerevisiae MAK16 protein gene, complete cds, and LTE1 protein gene, 3' end.//3.1e-31:731:62//J03852
F-NT2RM4000229//Homo sapiens chromosome 10 clone CIT987SK-1144G6 map 10q25.1, complete sequence.//4.6e-102:233:94//AC005383
F-NT2RM4000233//Mus musculus semaphorin VIa mRNA, complete cds.//1.6e-135:835:86//AF030430
F-NT2RM4000244//RPCI11-24P15.TV RPCI-11 Homo sapiens genomic clone RPCI-11-24P15, genomic survey sequence.//5.5e-08:422:62//B86757
F-NT2RM4000251//Mus musculus clone UWGC:mbac92 from 14D1-D2 (T-Cell Receptor Alpha Locus), complete sequence.//0.98:207:60//AC005855
F-NT2RM4000265//Homo sapiens Chromosome 11q12.2 PAC clone pDJ1081b4 containing human mRNA for T-cell glycoprotein CD6, complete sequence.//5.2e-41:707:65//AC003689
F-NT2RM4000290//Human transducin-like enhancer protein (TLE3) mRNA, complete cds.//7.9e-153:609:93//M99438
F-NT2RM4000324
F-NT2RM4000327//Rattus norvegicus guanine nucleotide binding protein beta 4 subunit mRNA, partial cds.//3.9e-44:727:68//AF022085
F-NT2RM4000344//Mus musculus ATP-dependent metalloprotease FtsH1 mRNA, complete cds.//1.0e-143:801:90//AF090430
F-NT2RM4000349//Mus musculus clone OST431, genomic survey sequence.//6.1e-80:503:86//AF046700
F-NT2RM4000354//HS_2221_A2_C07_MF CIT Approved Human Genomic Sperm Library D Homo sapiens genomic clone Plate=2221 Col=14 Row=E, genomic survey sequence.//1.0e-20:180:83//AQ253449
F-NT2RM4000356
F-NT2RM4000366//Homo sapiens mRNA for KIAA0642 protein, partial cds.//1.6e-133:628:99//AB014542
F-NT2RM4000368//RPCI11-91B5.TJ RPCI11 Homo sapiens genomic clone R-91B5, genomic survey sequence.//5.0e-12:431:61//AQ283217
F-NT2RM4000386//Mus musculus DOC4 (Doc4) mRNA, complete cds.//7.4e-86:845:72//AF059485
F-NT2RM4000395//Saccharomyces cerevisiae chromosome VI cosmid 9965.//2.5e-34:767:61//D44597
F-NT2RM4000414//Homo sapiens XYLB mRNA for xylulokinase, complete cds.//1.5e-15:114:94//AB015046
F-NT2RM4000421
F-NT2RM4000425//Homo sapiens chromosome 17, clone hRPK.294_J_22, complete sequence.//1.5e-37:295:82//AC005921
F-NT2RM4000433//Mus musculus retinoic acid-responsive protein (Stra6) mRNA, complete cds.//3.9e-94:740:78//AF062476
F-NT2RM4000457//CIT-HSP-2346B17.TR CIT-HSP Homo sapiens genomic clone 2346B17, genomic survey sequence.//1.5e-22:149:92//AQ062111
F-NT2RM4000471//Homo sapiens mRNA for putative tRNA splicing protein, partial.//1.3e-76:386:97//AJ010952
F-NT2RM4000486//Homo sapiens mRNA, complete cds, clone:RES4-22A, //1.1e-22:356:67//AB000459
F-NT2RM4000496//Homo sapiens 12p13.3 BAC RPCI11-476M19 (Roswell Park Cancer Institute Human BAC Library) complete sequence.//0.53:198:70//AC005908
F-NT2RM4000511
F-NT2RM4000514
F-NT2RM4000515//CIT-HSP-2285L3.TR CIT-HSP Homo sapiens genomic clone 2285L3, genomic survey sequence.//0.0012:200:66//AQ000113
F-NT2RM4000520
F-NT2RM4000531//Human zinc finger protein 42 (MZF-1) mRNA, complete cds.//2.9e-31:732:64//M58297
F-NT2RM4000532//HS_3231_B1_C05_T7 CIT Approved Human Genomic Sperm Library D Homo sapiens genomic clone Plate=3231 Col=9 Row=F, genomic survey sequence.//1.3e-59:362:90//AQ192093
F-NT2RM4000534
F-NT2RM4000585//CITBI-E1-2508I18.TR CITBI-E1 Homo sapiens genomic clone 2508I18, genomic survey sequence.//1.1e-34:208:93//AQ260706
F-NT2RM4000590//CIT-HSP-2291M14.TF CIT-HSP Homo sapiens genomic clone 2291M14, genomic survey sequence.//8.3e-34:180:99//AQ004125
F-NT2RM4000595//Homo sapiens chromosome 17, clone hCIT.131_K_11, complete sequence.//1.2e-09:203:66//AC005288
F-NT2RM4000603//Human mRNA for KIAA0392 gene, partial cds.//5.3e-14:305:68//AB002390
F-NT2RM4000611//CIT-HSP-2169F21.TR CIT-HSP Homo sapiens genomic clone 2169F21, genomic survey sequence.//8.4e-16:109:94//B89870
F-NT2RM4000616//D.melanogaster mRNA for acetyl-CoA synthetase.//2.3e-59:721:68//Z46786
F-NT2RM4000674
F-NT2RM4000689//CIT-HSP-2381O13.TF CIT-HSP Homo sapiens genomic clone 2381O13, genomic survey sequence.//2.6e-31:174:97//AQ110303
F-NT2RM4000698
F-NT2RM4000700
F-NT2RM4000712//Homo sapiens ubiquitin hydrolyzing enzyme I (UBH1) mRNA, partial cds.//1.1e-89:744:77//AF022789
F-NT2RM4000717
F-NT2RM4000733//Human DNA sequence *** SEQUENCING IN PROGRESS *** from clone 423B22, WORKING DRAFT SEQUENCE.//2.1e-140:299:99//AL034379
F-NT2RM4000734//Homo sapiens mRNA for KIAA0760 protein, partial cds.//3.8e-158:743:98//AB018303
F-NT2RM4000741
F-NT2RM4000751//Human zinc finger protein 20 (ZNF20) pentanucleotide repeat polymorphism.//7.1e-95:754:77//M99593
F-NT2RM4000764
F-NT2RM4000778//Caenorhabditis elegans cosmid F36H12.//0.30:523:60//AF078790
F-NT2RM4000779//Homo sapiens mRNA for KIAA0451 protein, complete cds.//5.5e-172:810:98//AB007920
F-NT2RM4000787//Human DNA sequence from PAC 370M22 on chromosome 22q12-qter. contains GRB2 ADAPTOR LIKE PROTEIN, UBIQUINOL-CYTOCHROME C REDUCTASE IRON-SULFUR SUBUNIT PRECURSOR (UQCRFS1) exon, ESTs, STS, CA repeat and CpG island.//0.0057:163:69//Z82206
F-NT2RM4000790//Homo sapiens chromosome 19, cosmid R27216, complete sequence.//6.9e-39:237:94//AC005306
F-NT2RM4000795//Rattus norvegicus neuroligin 3 mRNA, complete cds.//5.9e-97:857:74//U41663
F-NT2RM4000796//HS_3214_B1_F11_T7 CIT Approved Human Genomic Sperm Library D Homo sapiens genomic clone Plate=3214 Col=21 Row=L, genomic survey sequence.//1.1e-14:254:68//AQ175988
F-NT2RM4000798//Bos taurus guanine nucleotide-exchange protein (ARF-GEP1) mRNA, complete cds.//6.2e-78:816:72//AF023451
F-NT2RM4000813//Leishmania major glycoprotein 96-92 (GP 96-92) gene, partial cds.//0.33:276:63//M63109
F-NT2RM4000820//, complete sequence.//2.6e-142:450:97//AC005406
F-NT2RM4000833//Drosophila melanogaster DNA sequence (P1 DS05273 (D80)), complete sequence.//1.9e-52:501:71//AC004373
F-NT2RM4000848//Homo sapiens chromosome 17, clone hRPK.167_N_20, complete sequence.//1.0:477:56//AC005940
F-NT2RM4000852
F-NT2RM4000855//Homo sapiens chromosome 17, clone hCIT.457_L_16, complete sequence.//3.4e-29:229:83//AC003957
F-NT2RM4000887
F-NT2RM4000895//Homo sapiens HuUAP1 mRNA for UDP-N-acetylglucosamine pyrophosphorylase, complete cds.//2.1e-20:407:64//AB011004
F-NT2RM4000950//Homo sapiens clone DJ0917G04, WORKING DRAFT SEQUENCE, 35 unordered pieces.//0.41:311:64//AC004929
F-NT2RM4000971//RPCI11-53H3.TJ RPCI11 Homo sapiens genomic clone R-53H3, genomic survey sequence.//1.0:208:64//AQ053735
F-NT2RM4000979//Homo sapiens chromosome 17, clone hRPK.642_C_21, complete sequence.//1.3e-19:207:78//AC005245
F-NT2RM4000996//CITBI-E1-2506B10.TF CITBI-E1 Homo sapiens genomic clone 2506B10, genomic survey sequence.//1.4e-73:361:98//AQ263651
F-NT2RM4001002//Homo sapiens mRNA for KIAA0729 protein, partial cds.//5.1e-170:803:98//AB018272
F-NT2RM4001016//Homo sapiens mRNA for KIAA0639 protein, partial cds.//3.3e-125:584:99//AB014539
F-NT2RM4001032//Gallus gallus chicken brain factor-2 (CBF-2) mRNA, complete cds.//0.00034:777:58//U47276
F-NT2RM4001047//MO25 gene [mice, embryos, mRNA, 2322 nt].//2.5e-92:776:74//S51858
F-NT2RM4001054//Canis familiaris sec61 homologue mRNA, complete cds.//3.1e-102:859:76//M96629
F-NT2RM4001084//CIT-HSP-2330F9.TR CIT-HSP Homo sapiens genomic clone 2330F9, genomic survey sequence.//4.6e-78:379:99//AQ044479
F-NT2RM4001092//cSRL-71b1-u cSRL flow sorted Chromosome 11 specific cosmid Homosapiens genomic clone cSRL-71b1, genomic survey sequence.//1.1e-12:152:75//B05776
F-NT2RM4001116
F-NT2RM4001140//Homo sapiens PAC clone DJ0964C11 from 7p14-p15, complete sequence.//1.9e-136:717:93//AC004593
F-NT2RM4001151//Streptomyces antibioticus ATP-binding protein and membrane protein (oleC-ORF1, oleC-ORF2, oleC-ORF3, oleC-ORF4, and oleC-PRF5) genes, complete cds; 3427 base-pairs.//0.0083:368:60//L06249
F-NT2RM4001155//Bos taurus 50 kDa protein (adp50) mRNA, complete cds.//3.9e-120:764:85//U04706
F-NT2RM4001160
F-NT2RM4001187
F-NT2RM4001191//CIT-HSP-2010E7.TF CIT-HSP Homo sapiens genomic clone 2010E7, genomic survey sequence.//6.2e-12:181:72//B53378
F-NT2RM4001200//H.sapiens HZF10 mRNA for zinc finger protein.//1.3e-66:799:69//X78933
F-NT2RM4001203//Homo sapiens rab3-GAP regulatory domain mRNA, complete cds.//4.2e-152:707:99//AF004828
F-NT2RM4001204
F-NT2RM4001217//Homo sapiens ectoderm-neural cortex-1 protein (ENC-1) mRNA, complete cds.//1.6e-62:715:70//AF005381
F-NT2RM4001256//Human NotI linking clone 924A058R, genomic survey sequence.//7.6e-14:109:90//U49884
F-NT2RM4001258//HS_3171_B2_G09_T7 CIT Approved Human Genomic Sperm Library D Homo sapiens genomic clone Plate=3171 Col=18 Row=N, genomic survey sequence.//2.5e-18:215:77//AQ149676
F-NT2RM4001309//Human DNA sequence from clone 551E13 on chromosome Xp11.2-11.3 Contains farnesyl pyrophosphate synthetase pseudogene, VT4 protein pseudogene, EST, GSS, complete sequence.//4.9e-28:526:66//AL022163
F-NT2RM4001313//H.sapiens mRNA for phosphatidylinositol 3-kinase.//2.5e-77:474:89//Z46973
F-NT2RM4001316//Caenorhabditis elegans cosmid K09H11.//1.2e-16:230:73//U97002
F-NT2RM4001320//Homo sapiens mRNA for Neuroblastoma, complete cds.//1.1e-41:642:66//D89016
F-NT2RM4001340//EP(3)0614 Drosophila melanogaster EP line Drosophila melanogaster genomic Sequence recovered from 5' end of P element, genomic survey sequence.//0.0040:141:68//AQ025127
F-NT2RM4001344//Caenorhabditis elegans DNA *** SEQUENCING IN PROGRESS *** from clone Y1E3, WORKING DRAFT SEQUENCE.//5.5e-06:469:60//AL021388
F-NT2RM4001347
F-NT2RM4001371//Arabidopsis thaliana chromosome II BAC T20K9 genomic sequence, complete sequence.//0.10:400:61//AC004786
F-NT2RM4001382//Homo sapiens RanBP7/importin 7 mRNA, complete cds.//2.2e-167:790:98//AF098799
F-NT2RM4001384//Homo sapiens DNA sequence from BAC 747E2 on chromosome 22q12.1. Contains ESTs, STSs and GSSs and genomic marker D22S56, complete sequence.//0.99:255:59//AL021393
F-NT2RM4001410//Homo sapiens genomic DNA, chromosome 21q11.1, segment 1/5, WORKING DRAFT SEQUENCE.//0.027:336:58//AP000023
F-NT2RM4001411//Mus musculus Pro-rich, PH, SH2 domain-containing signaling mediator (PSM) mRNA, complete cds.//5.9e-124:783:85//AF020526
F-NT2RM4001412//Rattus norvegicus GTPase activating protein SynGAP-c mRNA, complete cds.//2.2e-34:418:71//AF050183
F-NT2RM4001414//Homo sapiens full-length insert cDNA clone ZE16C11.//9.1e-76:363:100//AF086563
F-NT2RM4001437//Homo sapiens chromosome 5, BAC clone 313n8 (LBNL H146), complete sequence.//2.0e-47:623:69//AC004226
F-NT2RM4001444//Streptococcus pneumoniae penicillin-binding protein 2b (pbp2b), RecM (recM), D-Ala-D-Ala ligase (ddl), D-Ala-D-Ala adding enzyme (murF), MutT (mutT), cell division protein FtsA (ftsA), cell division protein FtsZ (ftsZ), YlmE (ylmE), YlmF (ylmF), YlmG (ylmG), YlmH (ylmH), cell division protein DivIVA (divIVA), and isoleucine-tRNA synthetase (ileS) genes, complete cds; and unknown gene.//3.6e-09:566:58//AF068901
F-NT2RM4001454
F-NT2RM4001455
F-NT2RM4001483//Human zinc finger protein ZNF136.//3.2e-36:329:78//U09367
F-NT2RM4001489//Homo sapiens mRNA for KIAA0685 protein, complete cds.//1.2e-155:724:99//AB014585
F-NT2RM4001519//Plasmodium falciparum 3D7 chromosome 12 PFYAC69 genomic sequence, WORKING DRAFT SEQUENCE, 4 unordered pieces.//0.00019:418:59//AC004688
F-NT2RM4001522//Human HepG2 3' region MboI cDNA, clone hmd6a08m3.//1.4e-16:130:88//D17274
F-NT2RM4001557
F-NT2RM4001565
F-NT2RM4001566
F-NT2RM4001569//HS_2050_B1_C08_MR CIT Approved Human Genomic Sperm Library D Homo sapiens genomic clone Plate=2050 Col=15 Row=F, genomic survey sequence.//2.7e-09:109:84//AQ234720
F-NT2RM4001582//Mus musculus COP9 complex subunit 7b (COPS7b) mRNA, complete cds.//1.2e-127:740:89//AF071317
F-NT2RM4001592//M.musculus mRNA of enhancer-trap-locus 1.//7.3e-117:710:88//X69942
F-NT2RM4001594//Homo sapiens chromosome 9q34, clone 107G20, WORKING DRAFT SEQUENCE, 2 ordered pieces.//0.34:388:59//AC002355
F-NT2RM4001597//M.musculus red-1 gene.//6.2e-139:788:90//X92750
F-NT2RM4001605//Homo sapiens mRNA for KIAA0791 protein, complete cds.//3.3e-162:750:99//AB018334
F-NT2RM4001611//Synechocystis sp. PCC6803 complete genome, 12/27, 1430419-1576592.//2.5e-05:490:58//D90910
F-NT2RM4001629//Mus musculus palmytoylated protein p55 mRNA, complete cds.//0.65:186:64//U38196
F-NT2RM4001650//*** SEQUENCING IN PROGRESS *** Homo sapiens chromosome 4, BAC clone C0435P12; HTGS phase 1, WORKING DRAFT SEQUENCE, 10 unordered pieces.//0.99:422:59//AC004689
F-NT2RM4001662//Human mRNA for KIAA0322 gene, partial cds.//2.6e-81:449:93//AB002320
F-NT2RM4001666
F-NT2RM4001682//Mus musculus clone OST9187, genomic survey sequence.//3.2e-35:240:87//AF046699
F-NT2RM4001710//Human DNA sequence *** SEQUENCING IN PROGRESS *** from clone 126A5, WORKING DRAFT SEQUENCE.//1.9e-151:564:97//AL031447
F-NT2RM4001714//Human mRNA for KIAA0202 gene, partial cds.//7.0e-85:748:74//D86957
F-NT2RM4001715//Human DNA sequence from clone 931K24 on chromosome 20p12 Contains ESTs and GSSs, complete sequence.//1.2e-91:488:94//AL034430
F-NT2RM4001731//Orang-utan in volucrin gene, complete cds.//0.40:530:59//M25312
F-NT2RM4001741//Mouse mRNA for talin.//1.1e-129:737:90//X56123
F-NT2RM4001746//Human DNA sequence *** SEQUENCING IN PROGRESS *** from clone 316G12, WORKING DRAFT SEQUENCE.//2.3e-49:320:89//AL031709
F-NT2RM4001754//Homo sapiens 12p13.3 PAC RPCI5-1180D12 (Roswell Park Cancer Institute Human PAC Library) complete sequence.//6.3e-64:379:76//AC005831
F-NT2RM4001758//R.norvegicus mRNA for serine/threonine kinase MARK1.//3.7e-146:871:87//Z83868
F-NT2RM4001776//Homo sapiens mRNA for KIAA0727 protein, partial cds.//2.3e-173:803:99//AB018270
F-NT2RM4001783//Homo sapiens clone DJ0981007, complete sequence.//2.0e-165:593:99//AC006017
F-NT2RM4001810
F-NT2RM4001813//Homo sapiens BAC clone NH0364H22 from 2, complete sequence.//7.1e-31:176:84//AC005036
F-NT2RM4001819//Human p58/GTA (galactosyltransferase associated protein kinase) mRNA, complete cds.//4.4e-34:195:95//M37712
F-NT2RM4001823//Mus musculus zinc finger protein (Zfp64) mRNA, complete cds.//3.3e-51:490:75//U49046
F-NT2RM4001828//Human zinc finger containing protein ZNF157 (ZNF157) mRNA, complete cds.//5.6e-74:688:72//U28687
F-NT2RM4001836//Homo sapiens Chromosome 22q11.2 Cosmid Clone 2h In DGCR Region, complete sequence.//1.0:406:60//AC000076
F-NT2RM4001841//Mus musculus A kinase anchor protein (AKAP-KL) mRNA, alternatively spliced isoform 2, complete cds.//1.6e-131:831:86//AF033275
F-NT2RM4001842//HS_3163_A2_G10_MR CIT Approved Human Genomic Sperm Library D Homo sapiens genomic clone Plate=3163 Col=20 Row=M, genomic survey sequence.//1.5e-05:355:60//AQ168513
F-NT2RM4001856//Caenorhabditis elegans cosmid K08F11.//4.0e-23:823:60//U70855
F-NT2RM4001858//Notophthalmus viridescens NvTbox1 mRNA, partial cds.//6.4e-11:266:66//U64433
F-NT2RM4001865//Homo sapiens mRNA for atopy related autoantigen CALC.//6.9e-149:704:98//Y17711
F-NT2RM4001876//F.rubripes GSS sequence, clone 060E22bA4, genomic survey sequence.//5.7e-48:600:68//Z88651
F-NT2RM4001880//CIT-HSP-2348J1.TF CIT-HSP Homo sapiens genomic clone 2348J1, genomic survey sequence.//0.0025:61:88//AQ060809
F-NT2RM4001905//R.norvegicus CYP3A1 gene, 5' flanking region.//2.5e-29:535:67//X98335
F-NT2RM4001922//HS_2237_A1_C10_MF CIT Approved Human Genomic Sperm Library D Homo sapiens genomic clone Plate=2237 Col=19 Row=E, genomic survey sequence.//2.2e-73:364:98//AQ033732
F-NT2RM4001930//Arabidopsis thaliana genomic DNA, chromosome 5, P1 clone: MXI10, complete sequence.//4.9e-10:269:63//AB005248
F-NT2RM4001938//Homo sapiens chromosome 17, clone hRPC.1081_P_3, complete sequence.//7.6e-152:311:100//AC005207
F-NT2RM4001940//Homo sapiens timeless homolog mRNA, complete cds.//1.1e-170:808:98//AF098162
F-NT2RM4001953//*** SEQUENCING IN PROGRESS *** Homo sapiens chromosome 4, BAC clone B13E4; HTGS phase 1, WORKING DRAFT SEQUENCE, 10 unordered pieces.//2.7e-45:310:86//AC004046
F-NT2RM4001965//Homo sapiens genomic DNA of 8p21.3-p22 anti-oncogene of hepatocellular colorectal and non-small cell lung cancer, segment 11/11.//1.6e-107:622:90//AB020868
F-NT2RM4001969//R.norvegicus mRNA for IP63 protein.//3.9e-24:221:76//X99330
F-NT2RM4001979//Homo sapiens mRNA for KIAA0798 protein, complete cds.//1.0e-61:527:76//AB018341
F-NT2RM4001984//Human DNA sequence from cosmid U151E3, between markers on chromosome X.//5.8e-07:502:60//Z82253
F-NT2RM4001987//RPCI11-49L11.TJ RPCI11 Homo sapiens genomic clone R-49L11, genomic survey sequence.//2.6e-33:177:99//AQ051701
F-NT2RM4002013//Homo sapiens chromosome 17, clone hRPK.294_J_22, complete sequence.//0.019:65:90//AC005921
F-NT2RM4002018//Human high molecular weight B cell growth factor mRNA sequence.///1.0:527:57//L15344
F-NT2RM4002034//Human DNA sequence from PAC 84F12 on chromosome Xq25-Xq26.3. Contains glypican-3 precursor (intestinal protein OCI-5) (GTR2-2), ESTs and CA repeat.//0.11:322:60//AL008712
F-NT2RM4002044//Homo sapiens SS-A/Ro autoantigen 52 kda component gene, complete cds.//0.015:513:61//U01882
F-NT2RM4002054//Homo sapiens clone DJ1039L24, WORKING DRAFT SEQUENCE, 3 unordered pieces.//2.0e-44:473;76//AC005283
F-NT2RM4002055//Homo sapiens mRNA for KIAA0640 protein, partial cds.//1.0e-171:803:98//AB014540
F-NT2RM4002062//Drosophila melanogaster; Chromosome 2L; Region 36B1-36B3; P1 clone DS02528, WORKING DRAFT SEQUENCE, 8 unordered pieces.//0.0031:298:59//AC005122
F-NT2RM4002063//Oryctolagus cuniculus sarcosine oxidase (SOX) mRNA, complete cds.//1.1e-147:705:98//U82267
F-NT2RM4002066//Human mRNA for KIAA0192 gene, partial cds.//3.4e-73:889:69//D83783
F-NT2RM4002067//Homo sapiens chromosome 5, BAC clone 282B7 (LBNL H192), complete sequence.//1.1e-53:295:76//AC005216
F-NT2RM4002073//Mus musculus fatty acid transport protein 3 mRNA, partial cds.//7.8e-25:277:75//AF072758
F-NT2RM4002075//Homo sapiens actin binding protein MAYVEN mRNA, complete cds.//9.0e-23:588:61//AF059569
F-NT2RM4002093//Rat PYBP1 mRNA for pyrimidine binding protein 1.//3.1e-68:544:69//X60789
F-NT2RM4002109//Mouse kif4 mRNA for microtubule-based motor protein KIF4, complete cds.//2.0e-121:762:86//D12646
F-NT2RM4002128//HS_3084_A1_D04_MF CIT Approved Human Genomic Sperm Library D Homo sapiens genomic clone Plate=3084 Col=7 Row=G, genomic survey sequence.//7.7e-18:117:95//AQ186312
F-NT2RM4002140
F-NT2RM4002145//Homo sapiens chromosome 19, fosmid 37308, complete sequence.//1.8e-49:736:65//AC004152
F-NT2RM4002146//Homo sapiens MAGOH mRNA, complete cds.//6.5e-70:454:85//AF035940
F-NT2RM4002161//Homo sapiens mRNA for LAFPTPase, isoform 1, partial.//4.2e-151:763:96//AJ130763
F-NT2RM4002174//Helicobacter pylori 26695 section 18 of 134 of the complete genome.//2.1e-16:580:60//AE000540
F-NT2RM4002189//Homo sapiens DNA sequence from BAC 722E9 on chromosome 22q13.2-13.33. Contains ESTs.//1.0e-07:792:61//AL008636
F-NT2RM4002194//Mus musculus semaphorin VIa mRNA, complete cds.//3.2e-132:782:87//AF030430
F-NT2RM4002205//Rattus norvegicus nuclear-encoded mitochondrial elongation factor G mRNA, complete cds.//1.5e-40:292: 84//L14684
F-NT2RM4002213
F-NT2RM4002226//Mus musculus p190-B gene, complete cds.//0.099:350:59//U67160
F-NT2RM4002251//Homo sapiens chromosome 17, clone HCIT187M2, complete sequence.//1.0:428:58//AC004448
F-NT2RM4002256//Mouse genomic DNA, chromosome 17, clone cosmid 49.1, genomic survey sequence.//9.4e-60:294:81//AB005959
F-NT2RM4002266//Fugu rubripes GSS sequence, clone 006I18aG12, genomic survey sequence.//3.3e-12:217:67//AL024779
F-NT2RM4002278//HS_3089_A1_E05_MF CIT Approved Human Genomic Sperm Library D Homo sapiens genomic clone Plate=3089 Col=9 Row=I, genomic survey sequence.//1.9e-64:381:92//AQ121653
F-NT2RM4002281
F-NT2RM4002287//CIT-HSP-2327E14.TF CIT-HSP Homo sapiens genomic clone 2327E14, genomic survey sequence.//9.0e-49:336:86//AQ042515
F-NT2RM4002294//Human mRNA for KIAA0281 gene, complete cds.//2.1e-48:511:72//D87457
F-NT2RM4002301//Human NotI linking clone 924A053D, genomic survey sequence.//8.9e-05:62:91//U49881
F-NT2RM4002323//Human DNA sequence from clone 59B16 on chromosome 6p22.1-22.3. Contains a pseudogene similar to GPISG20 and other exonucleases). Contains ESTs, STSs, GSSs, genomic markers D6S1691 and D6S299 and a ca repeat polymorphism, complete sequence.//4.9e-115:729:87//AL032822
F-NT2RM4002339//Homo sapiens PAC clone DJ0728D04, complete sequence.//1.1e-97:457:93//AC004865
F-NT2RM4002344//Caenorhabditis elegans cosmid K04A8.//2.2e-06:190:69//U64849
F-NT2RM4002373//Homo sapiens mRNA for KIAA0649 protein, complete cds.//2.8e-149:708:98//AB014549
F-NT2RM4002374//Homo sapiens 12q24 PAC P336P3 (Research Park Cancer Institute Human Genome PAC library) complete sequence.//0.00040:312:63//AC002978
F-NT2RM4002383//Human DNA sequence *** SEQUENCING IN PROGRESS *** from clone 469D22, WORKING DRAFT SEQUENCE.//6.8e-29:378:66//AL031284
F-NT2RM4002390
F-NT2RM4002398//CIT-HSP-2288N22.TR CIT-HSP Homo sapiens genomic clone 2288N22, genomic survey sequence.//3.4e-35:184:100//AQ001110
F-NT2RM4002409//Archaeoglobus fulgidus section 15 of 172 of the complete genome.//2.0e-16:468:59//AE001092
F-NT2RM4002438//Human HLA class III region containing NOTCH4 gene, partial sequence, homeobox PBX2 (HPBX) gene, receptor for advanced glycosylation end products (RAGE) gene, complete cds, and 6 unidentified cds, complete sequence.//1.6e-16:123:91//U89336
F-NT2RM4002446//Human DNA sequence from cosmid 443D9 from a contig from the tip of the short arm of chromosome 16, spanning 2Mb of 16p13.3 Contains ESTs, STS and CpG islands,.//9.6e-64:467:84//Z92845
F-NT2RM4002452
F-NT2RM4002457//Human DNA sequence from PAC 151B14 on chromosome 22, complete sequence.//2.2e-24:201:86//Z85988
F-NT2RM4002460//Homo sapiens PAC clone DJ0630C24 from 7q31-q32, complete sequence.//1.3e-45:487:70//AC004690
F-NT2RM4002479//Homo sapiens RNA helicase-related protein mRNA, complete cds.//2.7e-163:777:98//AF083255
F-NT2RM4002482//Homo sapiens mRNA for KIAA0691 protein, complete cds.//2.3e-93:464:97//AB014591
F-NT2RM4002493
F-NT2RM4002499//Homo sapiens clone DJ0847008, WORKING DRAFT SEQUENCE, 3 unordered pieces.//3.5e-41:442:75//AC005484
F-NT2RM4002504//Human DNA sequence from clone 391O22 on chromosome 6p21.2-21.31 Contains pseudogenes similar to ribosomal protein, ESTs, GSSs, complete sequence.//3.8e-31:233:87//AL031577
F-NT2RM4002527//Fugu rubripes GSS sequence, clone 096G17aC8, genomic survey sequence.//7.7e-08:274:62//AL027162
F-NT2RM4002532
F-NT2RM4002534
F-NT2RM4002558//Mus musculus fatty acid transport protein 4 mRNA, partial cds.//3.8e-53:394:81//AF072759
F-NT2RM4002565//Mus musculus Sec8 mRNA, complete cds.//6.4e-160:902:89//AF022962
F-NT2RM4002567//CITBI-E1-2503J7.TR CITBI-E1 Homo sapiens genomic clone 2503J7, genomic survey sequence.//8.5e-31:220:88//AQ263402
F-NT2RM4002571//Rattus norvegicus UDP-GalNAc:polypeptide N-acetylgalactosaminyltransferase T5 mRNA, complete cds.//5.2e-05:199:65//AF049344
F-NT2RM4002593//Homo sapiens PAC clone DJ0745K06 from 7q31, complete sequence.//0.89:275:61//AC004875
F-NT2RM4002594//Drosophila melanogaster, chromosome 2R, region 31C1-31D6, P1 clone DS08879, complete sequence.//3.7e-44:768:64//AC005454
F-NT2RM4002623//Drosophila melanogaster; Chromosome 2L; Region 36B1-36B3; P1 clone DS02528, WORKING DRAFT SEQUENCE, 8 unordered pieces.//7.8e-34:574:65//AC005122
F-NT2RP1000018//Homo sapiens mRNA for NIK, partial cds.//3.9e-111:582:95//AB013385
F-NT2RP1000035//Homo sapiens mRNA for NS1-binding protein (NS1-BP).//1.1e-153:747:96//AJ012449
F-NT2RP1000040//Homo sapiens genomic DNA, chromosome 21q11.1, segment 18/28, WORKING DRAFT SEQUENCE.//1.6e-125:243:88//AP000047
F-NT2RP1000063//Caenorhabditis elegans cosmid F31C3, complete sequence.//9.6e-09:414:59//Z92784
F-NT2RP1000086//H.sapiens mRNA for zinc finger protein, Hsal2.//2.8e-183:548:91//X98834
F-NT2RP1000101//H.sapiens CpG island DNA genomic Mse1 fragment, clone 28b4, forward read cpg28b4.ft1a.//6.0e-27:163:95//Z60555
F-NT2RP1000111//CIT-HSP-2307O14.TR CIT-HSP Homo sapiens genomic clone 2307O14, genomic survey sequence.//1.2e-11:128:81//AQ016069
F-NT2RP1000112//Human kinase (TTK) mRNA, complete cds.//1.0e-38:324:81//M86699
F-NT2RP1000124//Plasmodium falciparum DNA *** SEQUENCING IN PROGRESS *** from MAL4P1, WORKING DRAFT SEQUENCE.//0.59:476:59//AL034557
F-NT2RP1000130//DNA encoding human Hepatoma-derived Growth Factor.//2.7e-35:535:681/E08546
F-NT2RP1000163//Homo sapiens cell cycle progression 2 protein (CPR2) mRNA, complete cds.//6.7e-05:77:90//AF011792
F-NT2RP1000170//Homo sapiens clone NH0001P09, WORKING DRAFT SEQUENCE, 1 unordered pieces.//1.9e-20:431:64//AC006030
F-NT2RP1000174//Homo sapiens clone 24432 mRNA sequence.//2.5e-138:679:97//AF070535
F-NT2RP1000191
F-NT2RP1000202//Porcine mRNA for M130 of smooth muscle myosin phosphatase, partial cds.//5.3e-05:220:61//D89496
F-NT2RP1000243//Drosophila melanogaster DNA sequence (P1 DS05273 (D80)), complete sequence.//4.7e-51:508:69//AC004373
F-NT2RP1000259
F-NT2RP1000272//Mus musculus TLS-associated protein with SR repeats mRNA, complete cds.//7.8e-142:866:88//AF042383
F-NT2RP1000324//RPCI11-81O21.TJ RPCI11 Homo sapiens genomic clone R-81O21, genomic survey sequence.//2.8e-29:182:92//AQ285136
F-NT2RP1000326//Homo sapiens metaxin 2 (MTX2) mRNA, nuclear gene encoding mitochondrial protein, complete cds.//4.2e-147:693:98//AF053551
F-NT2RP1000333//Caenorhabditis elegans cosmid C03D6, complete sequence.//1.4e-08:281:61//Z75525
F-NT2RP1000348//H.sapiens CpG island DNA genomic Mse1 fragment, clone 12f1, reverse read cpg12f1.rt1c.//1.7e-09:71:100//Z56610
F-NT2RP1000357
F-NT2RP1000358 5.7e-16:403:61//AC005456
F-NT2RP1000363//Homo sapiens mRNA for KIAA0638 protein, partial cds.//9.8e-125:497:86//AB014538
F-NT2RP1000376//Homo sapiens calcium-independent phospholipase A2 mRNA, complete cds.//1.8e-176:877:96//AF064594
F-NT2RP1000409//Homo sapiens repetitive sequences, alphoid DNA, 2482bp.//4.6e-106:700:84//AJ001558
F-NT2RP1000413//Homo sapiens mRNA for KIAA0587 protein, complete cds.//9.4e-178:710:98//AB011159
F-NT2RP1000416
F-NT2RP1000418//Oryctolagus cuniculus troponin T cardiac isoform mRNA, 3' end of cds.//1.0:198:60//L40178
F-NT2RP1000439//HS_2182_A1_D06_MF CIT Approved Human Genomic Sperm Library D Homo sapiens genomic clone Plate=2182 Col=11 Row=G, genomic survey sequence.//2.1e-68:441:87//AQ024305
F-NT2RP1000443//Homo sapiens genomic DNA, chromosome 21q11.1, segment 18/28, WORKING DRAFT SEQUENCE.//3.8e-57:185:88//AP000047
F-NT2RP1000460//Homo sapiens PAC clone DJ0844F09 from 7p12-p13, complete sequence.//2.7e-132:204:99//AC004453
F-NT2RP1000470//Human DNA from chromosome 19-specific cosmid R27090, genomic sequence, complete sequence.//4.9e-80:196:95//AC002985
F-NT2RP1000478//Human beta-tubulin class III isotype (beta-3) mRNA, complete cds.//1.9e-55:440:80//U47634
F-NT2RP1000481//Homo sapiens DNA sequence from PAC 262D12 on chromosome 1q23.3-24.3. Contains a Tenascin (Hexabrachion, Cytotactin, Neuronectin, Myotendinous antigen)-LIKE gene and a mitochondrial/chloroplast 30S ribosomal protein S14-LIKE gene preceeded by a CpG island. Contains ESTs, genomic marker D1S2691 and STSs.//2.6e-92:562:88//Z99297
F-NT2RP1000493//Homo sapiens mRNA for KIAA0017 protein, complete cds.//2.0e-130:622:98//D87686
F-NT2RP1000513//Xanthomonas campestris campestris xpsD, xpsM, and xpsN genes, complete cds's.//0.11:360:58//M81648
F-NT2RP1000522//Homo sapiens clone DJ0810E06, WORKING DRAFT SEQUENCE, 8 unordered pieces.//4.9e-34:209:93//AC004895
F-NT2RP1000547//Cricetulus griseus COP-coated vesicle membrane protein CHOp24 mRNA, partial cds.//1.2e-08:331:63//U26264
F-NT2RP1000574//Homo sapiens homeobox protein MEIS2 (MEIS2) mRNA, partial cds.//4.4e-81:295:92//AF017418
F-NT2RP1000577//HS_2228_B2_C05_MR CIT Approved Human Genomic Sperm Library D Homo sapiens genomic clone Plate=2228 Col=10 Row=F, genomic survey sequence.//1.9e-31:179:75//AQ185128
F-NT2RP1000581//Pan troglodytes von Willebrand factor (vWF) gene, partial cds.//4.7e-34:223:90//U31620
F-NT2RP1000609//Homo sapiens chromosome 11, BAC CIT-HSP-311e8 (BC269730) containing the hFEN1 gene, complete sequence.//1.6e-18:229:65//AC004770
F-NT2RP1000629//Mouse clathrin-associated protein (AP47) mRNA, complete cds.//9.3e-89:584:84//M62419
F-NT2RP1000630//Human DNA sequence from PAC 151B14 on chromosome 22 Contains EST, complete sequence.//1.0:203:63//Z85989
F-NT2RP1000677//Homo sapiens chromosome 19, cosmid R30538, complete sequence.//0.0034:350:61//AC005943
F-NT2RP1000688//H.sapiens gene for mitochondrial ATP synthase c subunit (P1 form).//5.2e-10:120:80//X69907
F-NT2RP1000695
F-NT2RP1000701//Sequence 1 from patent US 5580968.//2.4e-99:624:86//I30536
F-NT2RP1000721//Homo sapiens clone DJ0943F02, WORKING DRAFT SEQUENCE, 3 unordered pieces.//1.1e-19:188:81//AC004932
F-NT2RP1000730
F-NT2RP1000733//Human chromosome 16p13-1 BAC clone CIT987SK-551G9 complete sequence.//1.3e-30:315:75//U95742
F-NT2RP1000738//Homo sapiens Wolf-Hirschhorn syndrome candidate 2 protein (WHSC2) mRNA, complete cds.//8.0e-122:604:96//AF101434
F-NT2RP1000746//HS_3084_A1_H03_MF CIT Approved Human Genomic Sperm Library D Homo sapiens genomic clone Plate=3084 Col=5 Row=O, genomic survey sequence.//1.5e-83:466:92//AQ186344
F-NT2RP1000767//Homo sapiens full-length insert cDNA clone ZD81B04.//2.8e-21:144:91//AF086442
F-NT2RP1000782//Homo sapiens tetraspan TM4SF (TSPAN-3) mRNA, complete cds.//2.1e-121:591:97//AF054840
F-NT2RP1000796//T.thermophilus phosphofructokinase 1 (PFK1) gene, complete cds.//0.76:263:64//M71213
F-NT2RP1000825//Human DNA sequence from clone 116F5 on chromosome 22q13. Contains part of an unknown gene and part of a RhoGAP (CDC42 GTPAse Activating Protein) LIKE gene. Contains ESTs, STSs, GSSs, genomic marker D22S1168 and a CA repeat polymorphism, complete sequence.//1.5e-77:163:96//Z93244
F-NT2RP1000833//Homo sapiens cGMP-specific phosphodiesterase (PDE9A2) mRNA, complete cds.//1.3e-147:424:96//AF048837
F-NT2RP1000834//Homo sapiens alpha-methylacyl-CoA racemase mRNA, complete cds.//1.9e-89:702:79//AF047020
F-NT2RP1000836//Homo sapiens DNA sequence from PAC 434O14 on chromosome 1q32.3.41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two novel genes. Contains ESTs and GSSs, complete sequence.//8.7e-169:842:96//AL022398
F-NT2RP1000846//Human chromosome 8 BAC clone CIT987SK-2A8 complete sequence.//3.3e-15:196:76//U96629
F-NT2RP1000851//Homo sapiens PAC clone 267D11 from 12, complete sequence.//1.6e-144:724:96//AC004812
F-NT2RP1000856//Homo sapiens tetraspan TM4SF (TSPAN-3) mRNA, complete cds.//2.1e-121:591:97//AF054840
F-NT2RP1000860//Homo sapiens KL04P mRNA, complete cds.//6.7e-106:551:95//AF064094
F-NT2RP1000902//Human DNA sequence *** SEQUENCING IN PROGRESS *** from clone 316D5, WORKING DRAFT SEQUENCE.//0.0097:55:100//Z82199
F-NT2RP1000915//H.sapiens genomic DNA fragment (clone J32A032R).//1.3e-30:174:97//Z94761
F-NT2RP1000916
F-NT2RP1000943//Hylobates lar huntingtin gene, partial exon.//0.19:103:72//L49362
F-NT2RP1000944//HS_2179_B2_C12_MR CIT Approved Human Genomic Sperm Library D Homo sapiens genomic clone Plate=2179 Col=24 Row=F, genomic survey sequence.//0.032:140:63//AQ065269
F-NT2RP1000947//Mus musculus ubiquitin conjugating enzyme (ubc4) mRNA, complete cds.//3.7e-53:461:78//U62483
F-NT2RP1000954//cSRL-143G4-u cSRL flow sorted Chromosome 11 specific cosmid Homo sapiens genomic clone cSRL-143G4, genomic survey sequence.//0.030:89:78//B01950
F-NT2RP1000958//Caenorhabditis elegans cosmid K01C8, complete sequence.//3.9e-11:445:61//Z49068
F-NT2RP1000959//Homo sapiens PAC clone 278C19 from 12q, complete sequence.//3.3e-57:326:92//AC004263
F-NT2RP1000966//Human nucleolin gene, complete cds.//3.4e-64:197:981/M60858
F-NT2RP1000980//CIT-HSP-2314B10.TF CIT-HSP Homo sapiens genomic clone 2314B10, genomic survey sequence.//0.32:137:68//AQ017126
F-NT2RP1000988//Human chromosome 3p21.1 gene sequence.//8.0e-72:665:80//L13435
F-NT2RP1001011//Drosophila melanogaster DNA repair protein (mei-41) gene, complete cds, and TH1 gene, partial cds.//1.3e-31:497:65//U34925
F-NT2RP1001013//HS_3068_B1_809_MR CIT Approved Human Genomic Sperm Library D Homo sapiens genomic clone Plate=3068 Col=17 Row=D, genomic survey sequence.//1.0e-24:414:66//AQ127667
F-NT2RP1001014//HS_3252_B1_B05_MR CIT Approved Human Genomic Sperm Library D Homo sapiens genomic clone Plate=3252 Col=9 Row=D, genomic survey sequence.//0.00052:83:81//AQ304711
F-NT2RP1001033//Homo sapiens chromosome 17, clone hRPC-1073_F_15, complete sequence.//1.3e-134:241:99//AC004686
F-NT2RP1001073//Homo sapiens PAC clone DJ1194E14 from 7p21, complete sequence.//2.5e-59:451:83//AC004993
F-NT2RP1001079//Oryctolagus cuniculus sarcosine oxidase (SOX) mRNA, complete cds.//4.5e-93:476:96//U82267
F-NT2RP1001080//Homo sapiens clone DJ0971C03, WORKING DRAFT SEQUENCE, 18 unordered pieces.//6.6e-54:217:89//AC004938
F-NT2RP1001113
F-NT2RP1001173
F-NT2RP1001177//Rattus norvegicus histone macroH2A1.2 mRNA, complete cds.//8.1e-26:373:681/U79139
F-NT2RP1001185//Homo sapiens clone NH0319F03, WORKING DRAFT SEQUENCE, 3 unordered pieces.//3.5e-32:388:73//AC006039
F-NT2RP1001199
F-NT2RP1001247//Homo sapiens signaling molecule LEFTY-A gene, exon 1.//2.0e-29:166:96//AF081508
F-NT2RP1001248//Homo sapiens Chromosome 11q23 PAC clone pDJ356d6, complete sequence.//7.3e-50:128 :99//AC002036
F-NT2RP1001253//Homo sapiens oscillin (hLn) mRNA, complete cds.//4-3e-91:344:93//AF029914
F-NT2RP1001286//Homo sapiens chromosome X region from filamin (FLN) gene to glucose-6-phosphate dehydrogenase (G6PD) gene, complete cds's-//0.54:292:63//L44140
F-NT2RP1001294
F-NT2RP1001302
F-NT2RP1001310//Rabbit skeletal muscle mRNA for ryanodine receptor.//1.5e-07:335:64//X15750
F-NT2RP1001311//RPCI11-67O14.TK RPCI11 Homo sapiens genomic clone R-67O14, genomic survey sequence.//0.26:80:75//AQ239291
F-NT2RP1001313//Homo sapiens Chromosome 11q12.2 PAC clone pDJ519o13 containing human gene for ferritin heavy chain (FTH), complete sequence.//8.8e-75:304:98//AC004228
F-NT2RP1001361//B.taurus CI-B14.5b mRNA for NADH dehydrogenase (ubiquinone).//2.7e-57:412:84//X68647
F-NT2RP1001385
F-NT2RP1001395//Mus musculus COP9 complex subunit 7a (COPS7a) mRNA, complete cds.//1.4e-72:535:83//AF071316
F-NT2RP1001410//Homo sapiens DNA sequence from PAC 257I20 on chromosome 22q13.1-13.2. Contains cytochrome P450 pseudogenes CYP2D7P, CYP2D8P, CYP2D6(D),TCF20, NADH ubiquinone oxidoreductase B14 subunit, ESTs, CA repeat, STS, GSS.//5.8e-105:570:94//AL021878

F-NT2RP1001424
F-NT2RP1001432
F-NT2RP1001449//Homo sapiens clone 24733 mRNA sequence.//1.7e-84:422:97//AF052149
F-NT2RP1001457//Xenopus laevis notchless (nle) mRNA, complete cds.//1.3e-47:471:73//AF069737
F-NT2RP1001466//HS_3006_A2_D08_T7 CIT Approved Human Genomic Sperm Library D Homo sapiens genomic clone Plate=3006 Col=16 Row=G, genomic survey sequence.//0.56:289:60//AQ154336
F-NT2RP1001475//H.sapiens genomic DNA fragment (clone NLMA194R).//0.00011:91:79//Z95375
F-NT2RP1001482//Mouse oncogene (ect2) mRNA, complete cds.1/4-0e-87:563:85//L11316
F-NT2RP1001494
F-NT2RP10015431/Drosophila melanogaster DNA sequence (P1 DS01142 (D148)), complete sequence.//1.9e-27:387:67//AC004280
F-NT2RP1001546//Homo sapiens tetraspan TM4SF (TSPAN-3) mRNA, complete cds.//8.0e-63:314:98//AF054840
F-NT2RP1001569//Mus musculus signal recognition particle receptor beta subunit mRNA, complete cds.//1.2e-68:514:81//U17343
F-NT2RP100T616//Human clone 23665 mRNA sequence.//7.6e-40:496:74//U90913
F-NT2RP1001665//CIT-HSP-2059N5.TF CIT-HSP Homo sapiens genomic clone 2059N5, genomic survey sequence.//2.4e-45:305:88//B69912
F-NT2RP2000001//Homo sapiens clone 617 unknown mRNA, complete sequence.//1.5e-135:685:96//AF091081
F-NT2RP2000006//HS_3061_B2_C03_MR CIT Approved Human Genomic Sperm Library D Homo sapiens genomic clone Plate=3061 Col=6 Row=F, genomic survey sequence.//1.9e-17:394:67//AQ178856
F-NT2RP2000007//Human mRNA for KIAA0392 gene, partial cds.//3.5e-14:241:68//AB002390
F-NT2RP2000008//Human DNA sequence *** SEQUENCING IN PROGRESS *** from clone 257E24, WORKING DRAFT SEQUENCE.//1.7e-34:147:99//AL034424
F-NT2RP2000027//Homo sapiens BAC clone RG118P15 from 8q21, complete sequence.//1.4e-32:345:75//AC005066
F-NT2RP2000032//F.rubripes GSS sequence, clone 060E22aG10, genomic survey sequence.//5.0e-41:445:72//Z88655
F-NT2RP2000040//Homo sapiens mRNA for KIAA0747 protein, partial cds.//1.9e-76:383:97//AB018290
F-NT2RP2000045//Homo sapiens tumorous imaginal discs protein Tid56 homolog (TID1) mRNA, complete cds.//2.4e-95:467:97//AF061749
F-NT2RP2000054//CIT-HSP-2328J24.TF CIT-HSP Homo sapiens genomic clone 2328J24, genomic survey sequence.//3.3e-39:236:91//AQ043092
F-NT2RP2000056//Rat mRNA for protein tyrosine phosphatase epsilon C, partial cds.//3.2e-50:311:90//D78610
F-NT2RP2000067//Mus musculus DOC4 (Doc4) mRNA, complete cds.//3.0e-55:766:66//AF059485
F-NT2RP2000070//Homo sapiens chromosome 5, BAC clone 34j15 (LBNL H169), complete sequence.//2.0e-118:597:95//AC005754
F-NT2RP2000076//Homo sapiens clone NH0263G22, complete sequence.//0.0017:423:60//AC006037
F-NT2RP2000077//Homo sapiens growth arrest specific 11 (GAS11) mRNA, complete cds.//2.1e-77:278:97//AF050079
F-NT2RP2000079//H.sapiens CpG island DNA genomic Mse1 fragment, clone 40c2, forward read cpg40c2.ft1k.//3.2e-33:197:95//Z55440
F-NT2RP2000088//Homo sapiens mRNA for KIAA0795 protein, partial cds.//2.2e-158:752:98//AB018338
F-NT2RP2000091//HS_2228_A2_B02_MR CIT Approved Human Genomic Sperm Library D Homo sapiens genomic clone Plate=2228 Col=4 Row=C, genomic survey sequence.//0.26:55:90//AQ146363
F-NT2RP2000097
F-NT2RP2000098//Homo sapiens clone DJ1098J04, WORKING DRAFT SEQUENCE, 2 unordered pieces.//2.5e-05:482:60//AC004961
F-NT2RP2000108//Homo sapiens DNA from chromosome 19, BAC 33152, complete sequence.//1.0e-22:274:69//AC003973
F-NT2RP2000114//Homo sapiens mRNA for GM3 synthase, complete cds.//4.9e-114:551:97//AB018356
F-NT2RP2000120//HS_3000_B1_E03_MR CIT Approved Human Genomic Sperm Library D Homo sapiens genomic clone Plate=3000 Col=5 Row=J, genomic survey sequence.//1.8e-21:129:97//AQ090365
F-NT2RP2000126//Homo sapiens chromodomain-helicase-DNA-binding protein mRNA, complete cds.//4.2e-119:607:96//AF054177
F-NT2RP2000133//Homo sapiens PAC clone DJ044L15 from Xq23, complete sequence.//1.3e-07:339:63//AC004827
F-NT2RP2000147//Mouse clathrin-associated protein (AP47) mRNA, complete cds.//9.0e-101:638:85//M62419
F-NT2RP2000153//Human DNA sequence from clone 218J18 on chromosome Xp11.3-11.4. Contains the NDP (Norrie Disease (Pseudoglioma)) gene and a CC1.3 Splicing Factor pseudogene. Contains ESTs, STSs and GSSs, complete sequence.//0.45:377:58//AL034370
F-NT2RP2000157//Homo sapiens Chr.14 PAC RPCI4-794B2 (Roswell Park Cancer Institute Human PAC Library) complete sequence.//4.0e-73:317:87//AC005924
F-NT2RP2000161//CIT-HSP-2353L5.TF.1 CIT-HSP Homo sapiens genomic clone 2353L5, genomic survey sequence.//3.0e-14:123:90//AQ263431
F-NT2RP2000173
F-NT2RP2000175
F-NT2RP2000183//F.rubripes GSS sequence, clone 168M02aC2, genomic survey sequence.//3.7e-06:152:66//AL007295
F-NT2RP2000195//Human DNA sequence from clone 45I4 on chromosome 6q24.1-24.3. Contains two putative unknown genes, ESTs, STSs and GSSs, complete sequence.//7.6e-62:170:99//AL023581
F-NT2RP2000205
F-NT2RP2000208//Homo sapiens chromosome 19, overlapping cosmids R29828 and F25496, complete sequence.//7.2e-80:170:90//AC003030
F-NT2RP2000224//Homo sapiens Chromosome 16 BAC clone CIT987SK-A-152E5, complete sequence.//5.5e-64:400:85//AC004382
F-NT2RP2000232//Human DNA sequence from PAC 196E23 on chromosome Xq26.1-27.2. Contains the TAT-SF1 (HIV-1 transcriptional elongation factor TAT cofactor TAT-SF1) gene, the BRS3 (Bombesin Receptor subtype-3 (Uterine Bombesin Receptor, BRS-3) gene, an unknown gene coding for two isoforms, a predicted CpG island, ESTs and STSs.//2.2e-07:280:66//Z97632
F-NT2RP2000233//Mus musculus tumor metastasis associated gene product (MAG) mRNA, complete cds.//8.8e-30:508:67//U88401
F-NT2RP2000239//Homo sapiens chromosome 4 clone B353C18 map 4q25, complete sequence.//4.0e-79:504:87//AC004066
F-NT2RP2000248
F-NT2RP2000257//Caenorhabditis elegans DNA *** SEQUENCING IN PROGRESS *** from clone Y1E3, WORKING DRAFT SEQUENCE.//0.0078:286:60//AL021388
F-NT2RP2000258//CIT-HSP-2349P21.TF CIT-HSP Homo sapiens genomic clone 2349P21, genomic survey sequence.//5.7e-82:416:97//AQ059184
F-NT2RP2000270//Homo sapiens chromosome 19, BAC CIT-B-393i15 (BC301323), complete sequence.//4.5e-29:310:73//AC006116
F-NT2RP2000274
F-NT2RP2000283//G.gallus mRNA for LRP/alpha-2-macroglobulin receptor.//6.3e-20:260:73//X74904
F-NT2RP2000288
F-NT2RP2000289
F-NT2RP2000297//Figure 2. Nucleotide and translated protein sequences of HPF1, -2, and - 9.//4.6e-69:744:70//M27877
F-NT2RP2000298//Streptomyces coelicolor cosmid 2E9.//4.4e-05:502:59//AL021530
F-NT2RP2000310//WORKING DRAFT SEQUENCE, 6 unordered pieces.//2.1e-13:173:76//AC006082
F-NT2RP2000327//Homo sapiens DNA sequence from PAC 434O14 on chromosome 1q32.3.-41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two novel genes. Contains ESTs and GSSs, complete sequence.//8.3e-144:731:95//AL022398
F-NT2RP2000328//Human DNA sequence from clone 931K24 on chromosome 20p12 Contains ESTs and GSSs, complete sequence.//1.9e-102:555:90//AL034430
F-NT2RP2000329//Bovine mitochondrial GTP:AMP phosphotransferase mRNA, complete cds.//6.4e-105:639:87//M25757
F-NT2RP2000337//HS_2060_B1_E01_MR CIT Approved Human Genomic Sperm Library D Homo sapiens genomic clone Plate=2060 Col=1 Row=J, genomic survey sequence.//0.78:218:60//AQ243333
F-NT2RP2000346//Homo sapiens apoptosis associated protein (GADD34) mRNA, complete cds.//3.6e-129:627:97//U83981
F-NT2RP2000369//HS_2182_B1_B11_MR CIT Approved Human Genomic Sperm Library D Homo sapiens genomic clone Plate=2182 Col=21 Row=D, genomic survey sequence.//2.5e-87:421:99//AQ024835
F-NT2RP2000412//Human DNA sequence from PAC 124O9 on chromosome 6q21. Contains DNAJ2 (HDJ1) like pseudogene, ESTs, STSs and GSSs.//0.72:170:65//AL021327
F-NT2RP2000414//Homo sapiens HnRNP F protein mRNA, complete cds.//5.0e-66:375:93//L28010
F-NT2RP2000420//Homo sapiens full-length insert cDNA YQ86E07.//9.2e-77:423:93//AF075093
F-NT2RP2000422//Homo sapiens N-acetylglucosamine-phosphate mutase mRNA, complete cds.//2.1e-126:609:96//AF102265
F-NT2RP2000438//CITBI-E1-2519O19.TR CITBI-E1 Homo sapiens genomic clone 2519O19, genomic survey sequence.//0.96:61:78//AQ276878
F-NT2RP2000448//Homo sapiens PAC clone DJ0740D02 from 7p14-p15, complete sequence.//7.1e-17:341:67//AC004691
F-NT2RP2000459//H.sapiens mRNA for imogen 38.//5.7e-21:158:87//Z68747
F-NT2RP2000498//Human DNA sequence from PAC 435C23 on chromosome X. Contains ESTs.//3.2e-11:160:73//Z92844
F-NT2RP2000503//Homo sapiens PAC clone DJ1136G13 from 7q35-q36, complete sequence.//0.0031:187:66//AC005229
F-NT2RP2000510//Fugu rubripes GSS sequence, clone 066G04aC1, genomic survey sequence.//8.8e-07:179:64//AL026277
F-NT2RP2000516//Mus musculus t complex testis-specific protein (Tctex2) gene, wild type, promoter sequence.//0.19:72:81//U21671
F-NT2RP2000523//Human DNA sequence *** SEQUENCING IN PROGRESS *** from clone 150C2, WORKING DRAFT SEQUENCE.//5.0e-115:570:96//AL022318
F-NT2RP2000603//Homo sapiens mRNA for MCM3 import factor, complete cds.//8.4e-37:196:98//AB005543
F-NT2RP2000617//Homo sapiens chromosome 19, cosmid R27377, complete sequence.//0.81:354:60//AC005321
F-NT2RP2000634//Homo sapiens mRNA for KIAA0614 protein, partial cds.//1.3e-149:732:97//AB014514
F-NT2RP2000644//HS_3211_A1_F06_T7 CIT Approved Human Genomic Sperm Library D Homo sapiens genomic clone Plate=3211 Col=11 Row=K, genomic survey sequence.//3.6e-42:282:86//AQ175486
F-NT2RP2000656
F-NT2RP2000658//CITBI-E1-2518N15.TF CITBI-E1 Homo sapiens genomic clone 2518N15, genomic survey sequence.//0.57:141:66//AQ278386
F-NT2RP2000668
F-NT2RP2000678//Homo sapiens clone DJ0891L14, WORKING DRAFT SEQUENCE, 12 unordered pieces.//4.3e-22:433:62//AC004916
F-NT2RP2000704//Homo sapiens Xp22-175-176 BAC GSHB-484O17 (Genome Systems Human BAC Library) complete sequence.//2.7e-22:270:75//AC005913
F-NT2RP2000710//Drosophila melanogaster; Chromosome 2L; Region 36B1-36B3; P1 clone DS02528, WORKING DRAFT SEQUENCE, 8 unordered pieces.//1.4e-32:574:64//AC005122
F-NT2RP2000715//Homo sapiens PAC clone DJ1066K24 from 7p15, complete sequence.//4.8e-113:546:98//AC004540
F-NT2RP2000731//Homo sapiens clone DJ1106H14, WORKING DRAFT SEQUENCE, 42 unordered pieces.//0.97:115:70//AC004965
F-NT2RP2000758//Human LIM-kinase1 and alternatively spliced LIM-kinase1 (LIMK1) gene, complete cds.//9.7e-16:162:77//U62293
F-NT2RP2000764//HS_2254_B2_D07_MF CIT Approved Human Genomic Sperm Library D Homo sapiens genomic clone Plate=2254 Col=14 Row=H, genomic survey sequence.//0.071:45:95//AQ068887
F-NT2RP2000809
F-NT2RP2000812//Egernia stokesii clone EST3 microsatellite.//0.040:158:64//AF069698
F-NT2RP2000814
F-NT2RP2000816
F-NT2RP2000819
F-NT2RP2000841//Human mRNA for KIAA0294 gene, complete cds.//1.1e-26:390:70//AB002292
F-NT2RP2000842//H.sapiens mRNA for G protein-coupled receptor Edg-2.//1.2e-44:255:93//Y09479
F-NT2RP2000845
F-NT2RP2000863//Human partial cDNA sequence, clone x874;.//5.9e-29:173:94//Z47045
F-NT2RP2000880//Homo sapiens mRNA for KIAA0741 protein, complete cds.//2.4e-140:732:94//AB018284
F-NT2RP2000892
F-NT2RP2000931//Homo sapiens mRNA for KIAA0723 protein, complete cds.//3.4e-129:610:98//AB018266
F-NT2RP2000932//Homo sapiens BAC clone GS166A23 from 7p21, complete sequence.//1.8e-37:212:84//AC005014
F-NT2RP2000938//Human DNA sequence from cosmid RJ14 from a contig from the tip of the short arm of chromosome 16, spanning 2Mb of 16p13.3. Contains ESTs and CpG island.//1.6e-126:682:93//Z69890
F-NT2RP2000943//Homo sapiens mRNA for KIAA0755 protein, complete cds.//5.8e-112:533:98//AB018298
F-NT2RP2000965
F-NT2RP2000970//Homo sapiens DNA sequence from BAC 747E2 on chromosome 22q12.1. Contains ESTs, STSs and GSSs and genomic marker D22S56, complete sequence.//9.2e-101:505:96//AL021393
F-NT2RP2000985//Homo sapiens chromosome 17, clone hRPK.597_M_12, complete sequence.//1.6e-72:498:82//AC005277
F-NT2RP2000987//Human Chromosome 16 BAC clone CIT987SK-A-211C6, complete sequence.//7.4e-12:171:77//AC002394
F-NT2RP2001036//Homo sapiens chromosome 17, clone HRPC1096F1, complete sequence.//1.2e-37:390:76//AC004167
F-NT2RP2001044//HS_2253_B1_G01_MF CIT Approved Human Genomic Sperm Library D Homo sapiens genomic clone Plate=2253 Col=1 Row=N, genomic survey sequence.//0.21:276:61//AQ069224
F-NT2RP2001056//Homo sapiens mRNA, chromosome 1 specific transcript KIAA0488.//3.2e-144:696:97//AB007957
F-NT2RP2001065
F-NT2RP2001070//Rattus norvegicus pyridoxine 5'-phosphate oxidase mRNA, complete cds.//4.3e-104:775:81//U91561
F-NT2RP2001081//Rattus norvegicus synaptotagmin XI mRNA, complete cds.//3.7e-69:488:82//AF000423
F-NT2RP2001094//Human DNA sequence from PAC 410B11 on chromosome X contains STS.//7.4e-11:490:61//Z86063
F-NT2RP2001119//Human DNA sequence *** SEQUENCING IN PROGRESS *** from clone 745C22, WORKING DRAFT SEQUENCE.//5.1e-30:316:76//AL031596
F-NT2RP2001127//Human mRNA for KIAA0234 gene, complete cds.//1.1e-31:519:63//D87072
F-NT2RP2001137//HS_2193_B2_D12_MR CIT Approved Human Genomic Sperm Library D Homo sapiens genomic clone Plate=2193 Col=24 Row=H, genomic survey sequence.//1.8e-11:136:78//AQ032187
F-NT2RP2001149//Homo sapiens Chromosome 22q11.2 Cosmid Clone 2h In DGCR Region, complete sequence.//6.2e-29:247:78//AC000076
F-NT2RP2001168//Human DNA sequence from clone 431P23 on chromosome 6q27. Contains the first coding exon of the MLLT4 gene for myeloid/lymphoid or mixed-lineage leukemia (trithorax (Drosophila) homolog); translocated to, 4 (AF-6, Afadin, MLLT-4, ALL-1 fusion partner), and a Serine Palmitoyltransferase 2 (EC 2.3.1.50, Long Chain Base Biosynthesis protein 2, LCB-2, SPT-2) pseudogene. Contains ESTs, STss, GSSs, and a putative CpG island, complete sequence.//0.23:207:66//AL009178
F-NT2RP2001173//Homo sapiens mRNA for KIAA0480 protein, complete cds.//2.3e-112:567:96//AB007949
F-NT2RP2001174//RPCI11-58L2.TK RPCI11 Homo sapiens genomic clone R-58L2, genomic survey sequence.//7.6e-07:196:64//AQ237306
F-NT2RP2001196
F-NT2RP2001218
F-NT2RP2001226//Homo sapiens LERK-6 (EPLG6) gene, exon 1.//1.1e-09:320:65//U92893
F-NT2RP2001233//Human ZFP-36 mRNA for a zinc finger protein.//6.1e-71:681:72//X51760
F-NT2RP2001245//HS_3062_B1_F07_MR CIT Approved Human Genomic Sperm Library D Homo sapiens genomic clone Plate=3062 Col=13 Row=L, genomic survey sequence.//1.5e-05:268:63//AQ143177
F-NT2RP2001268//Homo sapiens mRNA for KIAA0810 protein, partial cds.//2.5e-106:514:97//AB018353
F-NT2RP2001277//Plasmodium falciparum chromosome 2, section 67 of 73 of the complete sequence.//0.32:183:64//AE001430
F-NT2RP2001290//M.musculus mRNA for I47 clone.//8.6e-102:641:86//X61455
F-NT2RP2001295//Caenorhabditis elegans DNA *** SEQUENCING IN PROGRESS *** from clone Y105E8, WORKING DRAFT SEQUENCE.//0.20:171:63//AL022594
F-NT2RP2001312//Bovine synaptophysin mRNA, complete cds.//0.98:253:58//M22967
F-NT2RP2001327//Human B12 protein mRNA, complete cds.//5.8e-29:359:71//M80783
F-NT2RP2001328//CIT-HSP-2335A5.TF CIT-HSP Homo sapiens genomic clone 2335A5, genomic survey sequence.//1.3e-65:366:94//AQ038539
F-NT2RP2001347//Homo sapiens complete genomic sequence between D16S3070 and D16S3275, containing Familial Mediterranean Fever gene disease.//3.8e-31:325:77//AJ003147
F-NT2RP2001366//H.sapiens CpG island DNA genomic Mse1 fragment, clone 4e11, forward read cpg4e11.f1a.//1.7e-12:98:92//Z61305
F-NT2RP2001378//HS_3054_B2_A03_MR CIT Approved Human Genomic Sperm Library D Homo sapiens genomic clone Plate=3054 Col=6 Row=B, genomic survey sequence.//9.8e-17:131:89//AQ100721
F-NT2RP2001381//Arabidopsis thaliana BAC T2L5.//0.080:434:59//AF096371
F-NT2RP2001392//S.pristinaespiralis snbC gene & amp; snbDE gene.//0.019:267:59//Y11548
F-NT2RP2001394//Human DNA sequence from PAC 389A20 on chromosome X contains ESTs STS, CpG islands and polymorphic CA repeat.//1.9e-16:133:78//Z93242
F-NT2RP2001397//Bos taurus cyclin B2 (CYCB2) mRNA, complete cds.//1.3e-63:419:84//AF080219
F-NT2RP2001420//Mus musculus nuclear protein NIP45 mRNA, complete cds.//3.1e-98:747:79//U76759
F-NT2RP2001423//Xenopus laevis ER1 mRNA, complete cds.//3.7e-34:269:85//AF015454
F-NT2RP2001427//Homo sapiens Chromosome 2p13 BAC Clone h173, complete sequence.//3.2e-13:164:78//AC003065
F-NT2RP2001436//Mus musculus clone OST1784, genomic survey sequence.//3.0e-06:136:71//AF046702
F-NT2RP2001440//cDNA sequence coding for gamma protein.//7.9e-83:553:86//E02350
F-NT2RP2001445//P.falciparum complete gene map of plastid-like DNA (IR-A).//1.5e-09:829:57//X95275
F-NT2RP2001449//B.taurus mRNA for cleavage and polyadenylation specificity factor.//1.3e-136:766:90//X75931
F-NT2RP2001450
F-NT2RP2001467
F-NT2RP2001506//CIT-HSP-2374H21.TF CIT-HSP Homo sapiens genomic clone 2374H21, genomic survey sequence.//7.9e-14:151:80//AQ109561
F-NT2RP2001511//Oryctolagus cuniculus translation initiation factor eIF2C mRNA, complete cds.//2.6e-22:462:64//AF005355
F-NT2RP2001520//Homo sapiens mRNA for mitochondrial carrier protein ARALAR1.//2.0e-136:657:97//Y14494
F-NT2RP2001526//Homo sapiens chromosome 17, clone hCIT.175_E_5, complete sequence.//1.2e-37:357:64//AC004596
F-NT2RP2001536//Homo sapiens X-ray repair cross-complementing protein 3 (XRCC3) mRNA, complete cds.//1.6e-103:384:94//AF035586
F-NT2RP2001560
F-NT2RP2001569//Homo sapiens mRNA, chromosome 1 specific transcript KIAA0488.//4.4e-123:590:98//AB007957
F-NT2RP2001576//Schistocerca americana Antennapedia homeotic protein (Antp) mRNA, complete cds.//0.038:580:58//U32943
F-NT2RP2001581//Mus musculus semaphorin VIa mRNA, complete cds.//6.5e-09:222:66//AF030430
F-NT2RP2001597//Homo sapiens alpha2-C4-adrenergic receptor gene, complete cds.//0.0057:361:60//U72648
F-NT2RP2001601//Homo sapiens mRNA for KIAA0797 protein, partial cds.//7.2e-137:647:98//AB018340
F-NT2RP2001613
F-NT2RP2001628//H.sapiens (xs128) mRNA, 380bp.//1.7e-15:279:68//Z36784
F-NT2RP2001634//Homo sapiens alpha-catenin-like protein (CTNNAL1) mRNA, complete cds.//5.4e-123:606:96//AF030233
F-NT2RP2001660//Homo sapiens putative 13 S Golgi transport complex 90kD subunit brain-specific isoform mRNA, complete cds.//4.2e-144:687:97//AF058718
F-NT2RP2001663//H.sapiens mRNA for 2-phosphopyruvate-hydratase-alpha-enolase.//1.0e-36:372:74//X84907
F-NT2RP2001675//S.pombe chromosome I cosmid c2G11.//0.070:507:59//Z54354
F-NT2RP2001677//Mouse BAC CitbCJ7 219m7, genomic sequence, complete sequence.//2.0e-60:232:96//AC005259
F-NT2RP2001678//HS_2007_A2_A04_T7 CIT Approved Human Genomic Sperm Library D Homo sapiens genomic clone Plate=2007 Col=8 Row=A, genomic survey sequence.//7.3e-62:370:91//AQ269699
F-NT2RP2001699//RPCI11-57B17.TK RPCI11 Homo sapiens genomic clone R-57B17, genomic survey sequence.//0.99:141:63//AQ115592
F-NT2RP2001720//Homo sapiens PAC clone DJ0167F23 from 7p15, complete sequence.//9.4e-117:604:95//AC004079
F-NT2RP2001721//Homo sapiens DNA sequence from clone 466I8 on chromosome Xq11.1-13.2. Contains an unknown gene similar to Coagulation Factor V (Activated Protein C Cofactor), Coagulation Factor VIII (Procoagulant Component) and Ceruloplasmin (EC 1.16.3.1, Ferroxidase). Contains ESTs and an STS, complete sequence.//1.0:273:61//AL030998
F-NT2RP2001740//Homo sapiens Chromosome 22q11.2 Cosmid Clone 8c In DGCR Region, complete sequence.//1.0:356:62//AC000090
F-NT2RP2001748//Human mRNA for KIAA0003 gene, complete cds.//3.7e-18:151:86//D14697
F-NT2RP2001762//Homo sapiens chromosome 1, BAC CIT-HSP-292g8 (BC262482), complete sequence.//6.0e-145:715:97//AC004783
F-NT2RP2001813//Plasmodium falciparum chromosome 2, section 15 of 73 of the complete sequence.//0.38:340:60//AE001378
F-NT2RP2001839//HS_3000_B1_C07_MR CIT Approved Human Genomic Sperm Library D_ Homo sapiens genomic clone Plate=3000 Col=13 Row=F, genomic survey sequence.//0.026:253:60//AQ090347
F-NT2RP2001861//Homo sapiens mRNA for paraplegin.//0.89:146:71//Y16610
F-NT2RP2001869//Homo sapiens ZNF202 beta (ZNF202) mRNA, complete cds.//0.040:174:62//AF027219
F-NT2RP2001876//Cyprinus carpio mRNA for allograft inflammatory factor-1, complete cds.//2.8e-44:483:71//AB012309
F-NT2RP2001883//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//1.8e-87:496:92//AL031864
F-NT2RP2001898//Human inositol polyphosphate 5-phosphatase (5ptase) mRNA, 3' end.//9.2e-112:633:90//M74161
F-NT2RP2001900//Caenorhabditis elegans DNA *** SEQUENCING IN PROGRESS *** from clone R08A5, WORKING DRAFT SEQUENCE.//0.0026:360:62//Z82281
F-NT2RP2001907//H.sapiens CpG island DNA genomic Mse1 fragment, clone 97f11, forward read cpg97f11.ft1a.//4.2e-26:206:84//Z64125
F-NT2RP2001926//Plasmodium falciparum 3D7 chromosome 12 PFYAC69 genomic sequence, WORKING DRAFT SEQUENCE, 4 unordered pieces.//5.5e-06:621:59//AC004688
F-NT2RP2001936//cSRL-47D9-u cSRL flow sorted Chromosome 11 specific cosmid Homo sapiens genomic clone cSRL-47D9, genomic survey sequence.//3.1e-50:282:93//B04856
F-NT2RP2001943//Drosophila melanogaster cosmid 25E8.//0.00036:248:60//AL009196
F-NT2RP2001946//Homo sapiens clone NH0140K04, complete sequence.//3.8e-78:232:99//AC005033
F-NT2RP2001947//Homo sapiens full-length insert cDNA clone ZD81B04.//2.0e-28:172:94//AF086442
F-NT2RP2001969//H.sapiens CpG island DNA genomic Mse1 fragment, clone 152a8, reverse read cpg152a8.rt1a.//1.0e-20:123:99//Z59378
F-NT2RP2001976
F-NT2RP2001985//Homo sapiens mRNA for KIAA0545 protein, partial cds.//0.0023:235:62//AB011117
F-NT2RP2001991//Rat orphan transporter v7-3 (NTT73) mRNA, complete cds.//3.1e-35:180:80//L22022
F-NT2RP2002025//Homo sapiens mRNA for KIAA0756 protein, partial cds.//9.8e-61:314:97//AB018299
F-NT2RP2002032//Homo sapiens chromosome 5, Bac clone 5m9 (LBNL H220), complete sequence.//0.76:189:65//AC005895
F-NT2RP2002033//Homo sapiens clone DJ0292L20, WORKING DRAFT SEQUENCE, 2 unordered pieces.//2.9e-12:160:79//AC004825
F-NT2RP2002041//Human BAC clone RG035E18 from 7q31, complete sequence.//0.0014:123:73//AC004029
F-NT2RP2002046//Homo sapiens Xp22 BAC GSHB-184P14 (Genome Systems Human BAC library) complete sequence.//2.2e-86:722:77//AC004552
F-NT2RP2002047//Human DNA sequence from clone 21F7 on chromosome 6q16.1-21. Contains part of an exon of a putative new gene and STSs and GSSs, complete sequence.//0.13:350:61//AL033375
F-NT2RP2002058//S.cerevisiae chromosome XII reading frame ORF YLR129w.//9.7e-11:480:60//Z73301
F-NT2RP2002066//Rattus norvegicus transmembrane receptor Unc5H2 mRNA, complete cds.//6.5e-97:610:86//U87306
F-NT2RP2002070//beta -ADD=adducin beta subunit 63 kda isoform/membrane skeleton protein, beta -ADD=adducin beta subunit 63 kda isoform/membrane skeleton protein {alternatively spliced, exon 10 to 13 region} [human, Genomic, 1851 nt, segment 3 of 3].//0.0059:107:73//S81083
F-NT2RP2002076//Homo sapiens clone 24804 mRNA sequence.//1.0e-127:643:96//AF052183
F-NT2RP2002078//F12O16-T7.1 IGF Arabidopsis thaliana genomic clone F12016, genomic survey sequence.//0.14:191:64//AQ249805
F-NT2RP2002079//Homo sapiens clone DJ0892G19, complete sequence.//0.0094:325:60//AC004917
F-NT2RP2002099//Homo sapiens mRNA for E1B-55kDa-associated protein.//9.8e-111:533:97//AJ007509
F-NT2RP2002105//H.sapiens CpG island DNA genomic Mse1 fragment, clone 10h8, forward read cpg10h8.ft1a.//2.4e-29:178:94//Z58857
F-NT2RP2002124//CIT-HSP-2023E9.TF CIT-HSP Homo sapiens genomic clone 2023E9, genomic survey sequence.//2.5e-32:202:92//B64468
F-NT2RP2002137//Human plasma membrane calcium ATPase (hPMCA4) mRNA, complete cds.//0.095:319:59//M25874
F-NT2RP2002154//Mus musculus mRNA for myosin, complete cds.//1.0:258:63//D85923
F-NT2RP2002172//HS_3020_B1_H02_T7 CIT Approved Human Genomic Sperm Library D Homo sapiens genomic clone Plate=3020 Col=3 Row=P, genomic survey sequence.//1.2e-11:124:82//AQ093169
F-NT2RP2002185//RPCI11-67B15.TJ RPCI11 Homo sapiens genomic clone R-67B15, genomic survey sequence.//2.8e-18:109:100//AQ201833
F-NT2RP2002192//Human PM-Scl-75 autoantigen (PM-sc1) mRNA, complete cds.//2.7e-36:363:78//U09215
F-NT2RP2002193//Rattus norvegicus potassium channel regulatory protein KChAP mRNA, complete cds.//9.5e-82:477:89//AF032872
F-NT2RP2002208
F-NT2RP2002219//Plasmodium falciparum DNA *** SEQUENCING IN PROGRESS *** from MAL4P1, WORKING DRAFT SEQUENCE.//1.0:378:58//AL034557
F-NT2RP2002231//Plasmodium falciparum 3D7 chromosome 12 PFYAC492 genomic sequence, WORKING DRAFT SEQUENCE, 5 unordered pieces.//0.60:560:56//AC005308
F-NT2RP2002235//P.falciparum glutamic acid-rich protein gnen, complete cds.//0.59:341:60//J03998
F-NT2RP2002252//Mus musculus mSin3A (sin3A) mRNA, complete cds.//3.5e-81:398:87//U22394
F-NT2RP2002256//Homo sapiens retinoic acid hydroxylase mRNA, complete cds.//6.6e-50:315:89//AF005418
F-NT2RP2002259//Human DNA sequence *** SEQUENCING IN PROGRESS *** from clone 118J21, WORKING DRAFT SEQUENCE.//9.7e-67:340:89//AL033527
F-NT2RP2002270//RPCI11-77C23.TV RPCI11 Homo sapiens genomic clone R-77C23, genomic survey sequence.//2.9e-18:79:93//AQ268098
F-NT2RP2002292//Human DNA sequence *** SEQUENCING IN PROGRESS *** from clone 321D2, WORKING DRAFT SEQUENCE.//1.0:290:60//AL031033
F-NT2RP2002312//Homo sapiens CDP-diacylglycerol synthase 2 (CDS2) mRNA, partial cds.//1.5e-93:467:96//AF069532
F-NT2RP2002316//HS_2171_B2_D11_MR CIT Approved Human Genomic Sperm Library D Homo sapiens genomic clone Plate=2171 Col=22 Row=H, genomic survey sequence.//7.3e-94:463:97//AQ119673
F-NT2RP2002325//Homo sapiens mRNA for Pex11p, complete cds.//3.9e-123:640:95//AB015594
F-NT2RP2002333
F-NT2RP2002373//F.rubripes GSS sequence, clone 026F10aB8, genomic survey sequence.//0.46:234:61//Z87330
F-NT2RP2002385//Homo sapiens synaptic glycoprotein SC2 spliced variant mRNA, complete cds.//9.4e-138:673:97//AF038958
F-NT2RP2002394//P.falciparum complete gene map of plastid-like DNA (IR-A).//0.79:421:56//X95275
F-NT2RP2002408//F.rubripes GSS sequence, clone 080G11aA8, genomic survey sequence.//5.7e-15:220:71//AL015615
F-NT2RP2002426//Sus scrofa SCAMP1 gene, exon 9.//7.1e-71:582:80//AJ223742
F-NT2RP2002439//Caenorhabditis elegans cosmid T07D3.//0.0018:210:67//AF016682
F-NT2RP2002442//Caenorhabditis elegans cosmid T03F1.//2.8e-18:295:67//U88169
F-NT2RP2002457//Homo sapiens Chromosome 16 BAC clone CIT987SK-44M2, complete sequence.//1.9e-06:281:66//AC004381
F-NT2RP2002464//Human mRNA for KIAA0086 gene, complete cds.//0.039:207:63//D42045
F-NT2RP2002475
F-NT2RP2002479//Homo sapiens mRNA for ABC transporter 7 protein, complete cds.//2.4e-123:607:96//AB005289
F-NT2RP2002498//Arabidopsis thaliana BAC F3D13.//0.73:395:57//AF069300
F-NT2RP2002503//Homo sapiens, clone hRPK.15_A_1, complete sequence.//7.2e-18:134:90//AC006213
F-NT2RP2002504//Homo sapiens mRNA for KIAA0791 protein, complete cds.//1.2e-157:761:97//AB018334
F-NT2RP2002520
F-NT2RP2002537
F-NT2RP2002546//Homo sapiens Chromosome 11q12 pac pDJ741n15, WORKING DRAFT SEQUENCE, 7 unordered pieces.//0.83:252:60//AC004127
F-NT2RP2002549//Human Chromosome 15q26.1 PAC clone pDJ457j11 containing DNA polymerase gamma (polg) gene, complete sequence.//5.9e-93:186:99//AC005317
F-NT2RP2002591//Human DNA sequence *** SEQUENCING IN PROGRESS *** from clone 54B20, WORKING DRAFT SEQUENCE.//4.0e-38:175:78//Z98304
F-NT2RP2002595//Sequence 2 from patent US 5763220.//1.5e-84:430:95//AR012155
F-NT2RP2002606//Rattus norvegicus Rabin3 mRNA, complete cds.//1.9e-43:282:87//U19181
F-NT2RP2002609//Mus musculus defender against death 1 (DAD1) gene, partial cds.//1.5e-11:99:90//AF051310
F-NT2RP2002618//H.sapiens mRNA for arginine methyltransferase, splice variant, 1316 bp.//5.6e-27:460:63//Y10806
F-NT2RP2002621
F-NT2RP2002643//Rat calmodulin III gene for calmodulin, promoter region and exon 1.//0.023:322:60//D90397
F-NT2RP2002672//Homo sapiens chromosome 10 clone CIT-HSP-1326H7 map 10q24.3-10q25.1, complete sequence.//3.9e-149:794:94//AC005384
F-NT2RP2002701//Human DNA sequence *** SEQUENCING IN PROGRESS *** from clone 50O24, WORKING DRAFT SEQUENCE.//9.2e-10:129:75//AL034380
F-NT2RP2002706//S.griseus secA gene.//1.3e-05:311:63//Y10980
F-NT2RP2002710//Homo sapiens mRNA for KIAA0672 protein, complete cds.//2.5e-40:631:65//AB014572
F-NT2RP2002727//Rattus norvegicus tulip 2 mRNA, complete cds.//4.8e-65:600:73//AF041107
F-NT2RP2002736//S.pombe chromosome II cosmid c887.//0.17:352:58//AL033388
F-NT2RP2002740//Absidia glauca ORF, 3' end; (+) mating type surface protein (PSSP15) gene, complete cds; ORF, 5' end.//0.0073:274:66//M94861
F-NT2RP2002741//Homo sapiens mRNA for Neuroblastoma, complete cds.//7.5e-29:628:62//D89016
F-NT2RP2002750//Homo sapiens Xp22 Bins 35-37 BAC GSHB-214D18 (Genome Systems Human BAC Library) complete sequence.//3.6e-31:568:67//AC005296
F-NT2RP2002752//Human BAC clone RG317M02 from 7p15-p21, complete sequence.//1.7e-08:206:63//AC002433
F-NT2RP2002753//Human DNA sequence from cosmid B11B7 on chromosome 22 contains ESTs.//2.8e-71:195:89//Z82171
F-NT2RP2002769//Streptomyces fradiae tylactone synthase, starter module and modules 1-7, (tylG) gene, complete cds.//0.0016:412:60//U78289
F-NT2RP2002778//CIT-HSP-2059C5.TF CIT-HSP Homo sapiens genomic clone 2059C5, genomic survey sequence.//6.8e-18:186:79//B69837
F-NT2RP2002800
F-NT2RP2002839//Homo sapiens Chromosome 11q12.2 PAC clone pDJ688p12 containing uteroglobin gene, WORKING DRAFT SEQUENCE, 11 unordered pieces.//1.2e-41:134:94//AC006078
F-NT2RP2002857//Rat T-cell receptor active beta-chain V-region (V-beta6-J-beta2.5) mRNA, partial cds, clone TRB-4.//0.85:93:68//M18845
F-NT2RP2002862//HS_3084_A1_H03_MF CIT Approved Human Genomic Sperm Library D Homo sapiens genomic clone Plate=3084 Col=5 Row=O, genomic survey sequence.//5.0e-67:390:91//AQ186344
F-NT2RP2002880
F-NT2RP2002891//CIT-HSP-2310O14.TF CIT-HSP Homo sapiens genomic clone 2310O14, genomic survey sequence.//0.11:53:90//AQ019792
F-NT2RP2002925//Pig mRNA for carbonyl reductase, complete cds.//0.66:194:65//D16511
F-NT2RP2002928//Homo sapiens pre-mRNA splicing factor (PRP17) mRNA, complete cds.//2.3e-135:628:99//AF038392
F-NT2RP2002929//F.rubripes GSS sequence, clone 123I23aA1, genomic survey sequence.//3.9e-06:66:83//AL017246
F-NT2RP2002939
F-NT2RP2002954
F-NT2RP2002959//Mus musculus ubiquitin conjugating enzyme (ubc4) mRNA, complete cds.//1.3e-47:411:79//U62483
F-NT2RP2002979//CIT-HSP-2340D12.TF CIT-HSP Homo sapiens genomic clone 2340D12, genomic survey sequence.//4.6e-96:476:97//AQ057233
F-NT2RP2002980//Sequence 20 from Patent EP0705842.//4.0e-13:100:94//A52230
F-NT2RP2002986//Homo sapiens actin binding protein MAYVEN mRNA, complete cds.//2.4e-09:272:61//AF059569
F-NT2RP2002987//Homo sapiens (subclone 6_d9 from P1 H21) DNA sequence, complete sequence.//1.0e-22:293:67//AC000958
F-NT2RP2002993//Rattus norvegicus RNA polymerase I 127 kDa subunit mRNA, complete cds.//4.0e-74:502:84//AF025424
F-NT2RP2003000//Homo sapiens chromosome 12p13.3, WORKING DRAFT SEQUENCE, 21 unordered pieces.//2.3e-46:474:76//AC004765
F-NT2RP2003034//Homo sapiens chromosome 17, clone hRPK.849_N_15, complete sequence.//4.2e-23:202:82//AC005703
F-NT2RP2003073//Human DNA sequence from PAC 306D1 on chromosome X contains ESTs.//3.4e-59:330:82//Z83822
F-NT2RP2003099//HS_3008_B2_C09_T7 CIT Approved Human Genomic Sperm Library D Homo sapiens genomic clone Plate=3008 Col=18 Row=F, genomic survey sequence.//1.4e-71:362:96//AQ089786
F-NT2RP2003108//Sequence 59 from patent US 5773577.//0.95:123:69//AR014362
F-NT2RP2003117//HS_2034_B2_D12_T7 CIT Approved Human Genomic Sperm Library D Homo sapiens genomic clone Plate=2034 Col=24 Row=H, genomic survey sequence.//1.5e-88:461:96//AQ230797
F-NT2RP2003121//Mus musculus enhancer of polycomb (Epc1) mRNA, complete cds.//4.3e-46:470:72//AF079765
F-NT2RP2003125//Homo sapiens chromosome 19, cosmid R34382, complete sequence.//5.7e-10:436:61//AC005329
F-NT2RP2003129//P.thunbergii cab gene.//0.00044:541:60//X61915
F-NT2RP2003137//CIT-HSP-2300J6.TR CIT-HSP Homo sapiens genomic clone 2300J6, genomic survey sequence.//5.0e-78:393:97//AQ012976
F-NT2RP2003157//Human DNA sequence from cDNA 16pHQG;16 from chromosome 16p13.3.//5.4e-07:137:71//Z84716
F-NT2RP2003158//Homo sapiens mRNA for proteasome subunit p58, complete cds.//1.8e-111:581:93//D67025
F-NT2RP2003161//CITBI-E1-2506E20.TR CITBI-E1 Homo sapiens genomic clone 2506E20, genomic survey sequence.//0.0025:156:67//AQ262657
F-NT2RP2003164
F-NT2RP2003165//Human hereditary haemochromatosis region, histone 2A-like protein gene, hereditary haemochromatosis (HLA-H) gene, RoRet gene, and sodium phosphate transporter (NPT3) gene, complete cds.//1.4e-43 :334:79//U91328
F-NT2RP2003177//Human signaling inositol polyphosphate 5 phosphatase SIP-110 mRNA, complete cds.//0.91:346:62//U50040
F-NT2RP2003194//Human DNA sequence *** SEQUENCING IN PROGRESS *** from clone 996D20, WORKING DRAFT SEQUENCE.//1.7e-108:511:90//AL031597
F-NT2RP2003206
F-NT2RP2003228//H.sapiens P1-Cdc21 mRNA.//2.9e-136:726:93//X74794
F-NT2RP2003230//Rattus norvegicus endo-alpha-D-mannosidase (Enman) mRNA, complete cds.//2.6e-51:348:86//AF023657
F-NT2RP2003237//Human DNA sequence *** SEQUENCING IN PROGRESS *** from clone 126A5, WORKING DRAFT SEQUENCE.//2.6e-56:415:83//AL031447
F-NT2RP2003243//RPCI11-36J1.TP RPCI-11 Homo sapiens genomic clone RPCI-11-36J1, genomic survey sequence.//2.1e-16:112:93//AQ047107
F-NT2RP2003265//Muridae sp. (mouse-rat, neuroblastoma-glioma hybrid cell line NGD5) mRNA, complete cds.//6.0e-114:696:87//L38481
F-NT2RP2003272//RPCI11-67B15.TJ RPCI11 Homo sapiens genomic clone R-67B15, genomic survey sequence.//3.8e-16:110:94//AQ201833
F-NT2RP2003277//Homo sapiens mRNA for KIAA0625 protein, partial cds.//1.5e-145:714:96//AB014525
F-NT2RP2003280//RPCI11-14I2.TVB RPCI-11 Homo sapiens genomic clone RPCI-11-14I2, genomic survey sequence.//6.4e-77:400:95//B85286
F-NT2RP2003286//CIT-HSP-2336D3.TF CIT-HSP Homo sapiens genomic clone 2336D3, genomic survey sequence.//5.3e-29:287:73//AQ041024
F-NT2RP2003293//Homo sapiens DNA from chromosome 19, BAC 33152, complete sequence.//1.5e-54:508:74//AC003973
F-NT2RP2003295//Homo sapiens RMP mRNA for RPB5 meidating protein, complete cds.//6.1e-85:416:97//AB006572
F-NT2RP2003297//S.pombe pho2 gene for specific p-nitrophenylphosphatase.//0.60:309:64//X62722
F-NT2RP2003307//Mus musculus kinesin light chain 2 (Klc2) mRNA, complete cds.//1.0e-45:442:75//AF055666
F-NT2RP2003308//D.melanogaster crn mRNA.//1.1e-63:697:70//X58374
F-NT2RP2003329//Homo sapiens chromosome 17, clone hCIT.131_K_11, complete sequence.//0.040:145:64//AC005288
F-NT2RP2003339
F-NT2RP2003347//Plasmodium falciparum MAL3P7, complete sequence.//0.12:275:60//AL034559
F-NT2RP2003367//Homo sapiens chromosome 4 clone B368A9 map 4q25, complete sequence.//0.83:225:63//AC005510
F-NT2RP2003391
F-NT2RP2003393//HS_3218_A2_B09_T7 CIT Approved Human Genomic Sperm Library D Homo sapiens genomic clone Plate=3218 Col=18 Row=C, genomic survey sequence.//0.021:93:79//AQ204356
F-NT2RP2003394
F-NT2RP2003401
F-NT2RP2003433//Rattus rattus sec61 homologue mRNA, complete cds.//4.2e-61:533:75//M96630
F-NT2RP2003445//Homo sapiens genomic DNA, chromosome 21q11.1, segment 1/5, WORKING DRAFT SEQUENCE.//2.1e-49:301:72//AP000023
F-NT2RP2003446
F-NT2RP2003456//Rickettsia prowazekii strain Madrid E, complete genome; segment 3/4.//0.0018:366:60//AJ235272
F-NT2RP2003466//Homo sapiens chromosome 11, BAC CIT-HSP-311e8 (BC269730) containing the hFEN1 gene, complete sequence.//7.5e-16:189:68//AC004770
F-NT2RP2003480//Mouse interleukin 2 receptor (p55 IL-2R) mRNA, 5' end.//1.9e-25:197:85//M21977
F-NT2RP2003499 2.1e-08:408:61//AB000826
F-NT2RP2003506//Homo sapiens clone NH0479C13, WORKING DRAFT SEQUENCE, 12 unordered pieces.//1.9e-33:192:96//AC005236
F-NT2RP2003511//Ceratopteris richardii mRNA for CRHB11, partial cds.//1.0:328:60//AB013801
F-NT2RP2003513//Human mRNA for KIAA0270 gene, partial cds.//7.3e-76:403:93//D87460
F-NT2RP2003517//Human osteosarcoma cell line U-2 OS mRNA fragment for PDGF-B chain (PDGF= platelet-derived growth factor).//1.5e-24:151:95//X03702
F-NT2RP2003522//Mouse interleukin 2 receptor (p55 IL-2R) mRNA, 5' end.//1.3e-101:564:91//M21977
F-NT2RP2003533//Human DNA sequence from cosmid F1121 on chromosome 6.//2.0e-40:315:75//Z80899
F-NT2RP2003543
F-NT2RP2003559//H.sapiens CpG island DNA genomic Mse1 fragment, clone 90a5, reverse read cpg90a5.rt1a.//1.1e-20:122:99//Z56144
F-NT2RP2003564//Human 52-kD ribonucleoprotein Ro/SSA mRNA, complete cds.//8.8e-27:664:63//M34551
F-NT2RP2003567//Homo sapiens mRNA for KIAA0462 protein, partial cds.//4.1e-113:541:98//AB007931
F-NT2RP2003581
F-NT2RP2003596//F.rubripes GSS sequence, clone 036L10aF12, genomic survey sequence.//J1.9e-11:210:65//AL012756
F-NT2RP2003604//Homo sapiens alpha-catenin-like protein (CTNNAL1) mRNA, complete cds.//1.9e-123:587:98//AF030233
F-NT2RP2003629
F-NT2RP2003643//Mus musculus mRNA for CMP-N-acetylneuraminic acid synthetase.//7.8e-88:582:84//AJ006215
F-NT2RP2003668//Homo sapiens clone RG270D13, WORKING DRAFT SEQUENCE, 18 unordered pieces.//5.6e-47:335:83//AC005081
F-NT2RP2003687//Homo sapiens Xp22 BAC GSHB-519E5 (Genome Systems Human BAC library) complete sequence.//1.2e-06:133:74//AC003684
F-NT2RP2003691//Human DNA sequence *** SEQUENCING IN PROGRESS *** from clone 525L6, WORKING DRAFT SEQUENCE.//1.7e-47:337:81//AL023807
F-NT2RP2003702//Rattus norvegicus ovarian-specific protein mRNA, complete cds.//1.3e-65:458:82//U44803
F-NT2RP2003704//H.sapiens CpG island DNA genomic Mse1 fragment, clone 2a9, reverse read cpg2a9.rt1e.//3.8e-17:170:84//Z60615
F-NT2RP2003706//Homo sapiens mRNA for KIAA0525 protein, partial cds.//2.6e-108:518:98//AB011097
F-NT2RP2003713//HS_2016_B1_B05_MR CIT Approved Human Genomic Sperm Library D Homo sapiens genomic clone Plate=2016 Col=9 Row=D, genomic survey sequence.//1.3e-11:102:90//AQ226895
F-NT2RP2003714//Homo sapiens DNA from chromosome 19, BAC 33152, complete sequence.//1.4e-27:249:78//AC003973
F-NT2RP2003727//RPCI11-77I19.TV RPCI11 Homo sapiens genomic clone R-77I19, genomic survey sequence.//3.4e-26:294:74//AQ268303
F-NT2RP2003737//Homo sapiens clone DJ1022I14, WORKING DRAFT SEQUENCE, 14 unordered pieces.//2.6e-74:194:91//AC004951
F-NT2RP2003751//Homo sapiens Chromosome 16 BAC clone CIT987SK-A-911E12, complete sequence.//1.7e-92:165:96//AC003964
F-NT2RP2003760//B.primigenius mRNA for coat protein gamma-cop.//4.5e-76:696:73//X92987
F-NT2RP2003764//Homo sapiens gene for MTG16, exon 1b, partial sequence.//1.0:109:69//AB013275
F-NT2RP2003769
F-NT2RP2003770//Homo sapiens chromosome 17, clone hRPC.1050_D_4, complete sequence.//3.0e-96:467:98//AC004771
F-NT2RP2003777
F-NT2RP2003781//tricarboxylate carrier [rats, liver, mRNA Partial, 2986 nt].//7.2e-107:731:82//S70011
F-NT2RP2003793//CIT-HSP-2326L12.TF CIT-HSP Homo sapiens genomic clone 2326L12, genomic survey sequence.//7.0e-20:124:95//AQ038761
F-NT2RP2003825//Homo sapiens BAC clone RG139P11 from 7q11-q21, complete sequence.//8.9e-06:151:74//AC004491
F-NT2RP2003840//Arabidopsis thaliana chromosome II BAC F12A24 genomic sequence, complete sequence.//0.018:145:69//AC005167
F-NT2RP2003857//HS_3227_A2_G04_T7 CIT Approved Human Genomic Sperm Library D Homo sapiens genomic clone Plate=3227 Col=8 Row=M, genomic survey sequence.//0.96:257:61//AQ303467
F-NT2RP2003859
F-NT2RP2003871//Homo sapiens 12q24 PAC RPCI1-74B13 (Roswell Park Cancer Institute Human PAC library) complete sequence.//2.0e-12:369:65//AC002375
F-NT2RP2003885//CITBI-E1-2514D6.TF CITBI-E1 Homo sapiens genomic clone 2514D6, genomic survey sequence.//0.13:167:64//AQ265722
F-NT2RP2003912//nek1=serine/threonine- and tyrosine-specific protein kinase [mice, erythroleukemia cells, mRNA, 4263 nt].//1.3e-136:838:86//S45828
F-NT2RP2003952
F-NT2RP2003968//Homo sapiens hUBP mRNA for ubiquitin specific protease, complete cds.//2.1e-28:165:96//AB014458
F-NT2RP2003976//Human DNA sequence from clone 283E3 on chromosome 1p36.21-36.33. Contains the alternatively spliced gene for Matrix Metalloproteinase in the Female Reproductive tract MIFR1, -2, MMP21/22A, -B and -C, a novel gene, the alternatively spliced CDC2L2 gene for Cell Division Cycle 2-Like 2 (PITSLRE, p58/GTA, Galactosyltransferase Associated Protein Kinase) beta 1, beta 2-1, beta 2-2 and alpha 2-4, a 40S Ribosomal Protein S7 pseudogene, part of the KIAA0447 gene, a novel alternatively spliced gene similar to many (archae)bacterial, worm and yeast hypothetical genes, and the GNB1 gene for Guanine Nucleotide Binding Protein (G protein), Beta polypeptide 1 (Transducin Beta chain 1). Contains putative CpG islands, ESTs, STSs and GSSs, complete sequence.//2.6e-24:298:74//AL031282
F-NT2RP2003981//Homo sapiens mRNA for KIAA0804 protein, partial cds.//9.9e-160:783:96//AB018347
F-NT2RP2003984
F-NT2RP2003986//Human Chromosome 11 pac pDJ197h17, WORKING DRAFT SEQUENCE, 11 unordered pieces.//1.7e-26:260:77//AC000382
F-NT2RP2003988//Human DNA sequence *** SEQUENCING IN PROGRESS *** from clone 862K6, WORKING DRAFT SEQUENCE.//9.1e-61:701:70//AL031681
F-NT2RP2004013//Human DNA sequence from clone 372K1 on chromosome 6q24 Contains EST, STS, GSS and CpG Island, complete sequence.//3.0e-123:693:91//AL023580
F-NT2RP2004014
F-NT2RP2004041//Homo sapiens chromosome 19, cosmid F17127, complete sequence.//5.8e-83:427:87//AC004780
F-NT2RP2004042
F-NT2RP2004066//Human DNA sequence *** SEQUENCING IN PROGRESS *** from clone 134O19, WORKING DRAFT SEQUENCE.//5.6e-110:528:98//AL034555
F-NT2RP2004081
F-NT2RP2004098//HS_2216_A1_B12_MF CIT Approved Human Genomic Sperm Library D Homo sapiens genomic clone Plate=2216 Col=23 Row=C, genomic survey sequence.//1.0e-07:86:84//AQ145694
F-NT2RP2004124//HS_3064_B2_A04_MF CIT Approved Human Genomic Sperm Library D Homo sapiens genomic clone Plate=3064 Col=8 Row=B, genomic survey sequence.//3.0e-25:155:94//AQ136993
F-NT2RP2004142//Arabidopsis thaliana genomic DNA, chromosome 5, TAC clone: K8K14, complete sequence.//1.0:220:62//AB007645
F-NT2RP2004152//Drosophila melanogaster DNA sequence (P1 DS02252 (D97)), complete sequence.//0.93:480:56//AC002493
F-NT2RP2004165//Plasmodium falciparum 3D7 chromosome 12 PFYACB8-420 genomic sequence, WORKING DRAFT SEQUENCE, 14 unordered pieces.//0.051:265:61//AC005140
F-NT2RP2004170//Homo sapiens distal-less homeobox protein (DLX7) gene, complete cds.//1.0:162:66//AF028235
F-NT2RP2004172//S.pombe chromosome II cosmid c24E9.//1.7e-06:466:59//AL021816
F-NT2RP2004187//Homo sapiens full-length insert cDNA YQ86E07.//3.5e-17:354:64//AF075093
F-NT2RP2004194//Rattus norvegicus Golgi SNARE GS15 mRNA, complete cds.//9.4e-53:397:82//AF003998
F-NT2RP2004196
F-NT2RP2004207//Human von Willebrand factor pseudogene corresponding to exons 23 through 34.//0.0023:386:61//M60676
F-NT2RP2004226//HS_2186_A1_D03_MF CIT Approved Human Genomic Sperm Library D Homo sapiens genomic clone Plate=2186 Col=5 Row=G, genomic survey sequence.//7.8e-58:370:87//AQ063813
F-NT2RP2004232//H.sapiens mRNA for protein kinase C mu.//1.2e-34:448:67//X75756
F-NT2RP2004239//Homo sapiens lok mRNA for protein kinase, complete cds.//5.2e-108:510:99//AB015718
F-NT2RP2004240//Pyrococcus horikoshii OT3 genomic DNA, 1166001-1485000 nt. position (6/7).//1.1e-12:489:61//AP000006
F-NT2RP2004242
F-NT2RP2004245
F-NT2RP2004270//Streptomyces coelicolor cosmid 1A9.//7.5e-07:462:62//AL034446
F-NT2RP2004300//Homo sapiens chromosome 19, cosmid R33632, complete sequence.//3.5e-11:299:64//AC005781
F-NT2RP2004316//Homo sapiens EXT-like protein 2 (EXTL2) mRNA, complete cds.//4.5e-150:735:97//AF000416
F-NT2RP2004321//Drosophila melanogaster DNA sequence (P1 DS02110 (D147)), complete sequence.//0.98:267:59//AC004423
F-NT2RP2004339//Human Chromosome 16 BAC clone CIT987SK-A-355G7, complete sequence.//1.6e-40:419:75//AC002519
F-NT2RP2004347//Human DNA sequence *** SEQUENCING IN PROGRESS *** from clone 1018D12, WORKING DRAFT SEQUENCE.//1.2e-72:439:82//AL031650
F-NT2RP2004364
F-NT2RP2004365
F-NT2RP2004366//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//0.92:427:57//AL031864
F-NT2RP2004373//Homo sapiens cosmids Qc15C1 and 94B6 from Xq28, complete sequence.//2.6e-26:493:65//AF035397
F-NT2RP2004389//HS_2183_B2_H04_MR CIT Approved Human Genomic Sperm Library D Homo sapiens genomic clone Plate=2183 Col=8 Row=P, genomic survey sequence.//2.9e-11:83:96//AQ063969
F-NT2RP2004392
F-NT2RP2004396//Homo sapiens BAC clone RG135C18 from 7q21, complete sequence.//1.1e-171:875:95//AC005164
F-NT2RP2004399//Homo sapiens SYBL1 gene.//1.4e-24:467:64//AJ004799
F-NT2RP2004400//Arabidopsis thaliana BAC T19B17 from chromsome IV, near 19.3 cM, complete sequence.//0.00074:455:59//AF069441
F-NT2RP2004412//H.sapiens CpG island DNA genomic Mse1 fragment, clone 34g4, reverse read cpg34g4.rt1a.//5.0e-27:154:98//Z65369
F-NT2RP2004425
F-NT2RP2004463//Streptomyces coelicolor cosmid 2E9.//0.0053:196:65//AL021530
F-NT2RP2004476//Drosophila melanogaster cosmid 67A9.//5.2e-15:377:63//AL034388
F-NT2RP2004490//Homo sapiens chromosome 16, P1 clone 94-10H (LANL), complete sequence.//4.3e-100:497:97//AC005591
F-NT2RP2004512//Plasmodium falciparum MAL3P5, complete sequence.//2.3e-07:815:57//AL034556
F-NT2RP2004523//Homo sapiens clone DJ0800G07, complete sequence.//8.5e-138:718:95//AC004890
F-NT2RP2004538//Homo sapiens mRNA for KIAA0591 protein, partial cds.//1.4e-137:687:96//AB011163
F-NT2RP2004551//CIT-HSP-2387G7.TF.1 CIT-HSP Homo sapiens genomic clone 2387G7, genomic survey sequence.//2.1e-85 :484:91//AQ239555
F-NT2RP2004568//H.vulgare GAA-satellite DNA.//2.0e-07:292:62//Z50100
F-NT2RP2004580//Human DNA sequence *** SEQUENCING IN PROGRESS *** from clone 968D22, WORKING DRAFT SEQUENCE.//4.5e-44:512:72//AL023755
F-NT2RP2004587//Candida albicans cytoskeleton assembly control protein (SLA2) gene, partial cds.//1.0:344:56//AF092908
F-NT2RP2004594//nbxb0019H13r CUGI Rice BAC Library Oryza sativa genomic clone nbxb0019H13r, genomic survey sequence.//0.053:324:60//AQ258020
F-NT2RP2004600
F-NT2RP2004602//Homo sapiens chromosome 19, cosmid F21431, complete sequence.//0.12:109:73//AC005176
F-NT2RP2004614
F-NT2RP2004655//Homo sapiens mRNA for leucine rich protein.//2.6e-102:496:98//AJ006291
F-NT2RP2004664//Homo sapiens mRNA for KIAA0460 protein, partial cds.//1.6e-153:728:98//AB007929
F-NT2RP2004675//Homo sapiens chromosome 12q24.1, WORKING DRAFT SEQUENCE, 33 unordered pieces.//0.092:239:61//AC005805
F-NT2RP2004681//Human DNA sequence from clone 51J23 on chromosome Xq26.3-27.3. Contains an EST and GSSs, complete sequence.//1.0:236:61//AL031312
F-NT2RP2004689//Homo sapiens mRNA for KIAA0625 protein, partial cds.//1.3e-59:327:94//AB014525
F-NT2RP2004709//HS_2033_B2_E04_MR CIT Approved Human Genomic Sperm Library D Homo sapiens genomic clone Plate=2033 Col=8 Row=J, genomic survey sequence.//1.9e-15:187:74//AQ230714
F-NT2RP2004710//HS_3185_82_D07_T7 CIT Approved Human Genomic Sperm Library D Homo sapiens genomic clone Plate=3185 Col=14 Row=H, genomic survey sequence.//9.9e-10:110:84//AQ172885
F-NT2RP2004736//Homo sapiens mRNA for KIAA0478 protein, complete cds.//6.4e-117:582:96//AB007947
F-NT2RP2004743//Human DNA sequence from PAC 37M17 chromosome X.//0.14:138:71//Z78022
F-NT2RP2004767//H.sapiens CpG island DNA genomic Mse1 fragment, clone 65c11, reverse read cpg65c11.rt1a.//1.3e-24:217:81//Z62210
F-NT2RP2004768//Homo sapiens STE20-like kinase 3 (mst-3) mRNA, complete cds.//1.6e-45:541:71//AF024636
F-NT2RP2004775//Plasmodium falciparum chromosome 2, section 35 of 73 of the complete sequence.//5.8e-13:697:59//AE001398
F-NT2RP2004791//Human HeLa mRNA isolated as a false positive in a two-hybridscreen.//5.0e-53:353:84//U56252
F-NT2RP2004799//Homo sapiens ATP-specific succinyl-CoA synthetase beta subunit (SCS) mRNA, partial cds.//1.5e-116:594:95//AF058953 F-NT2RP2004802
F-NT2RP2004816//Homo sapiens H beta 58 homolog mRNA, complete cds.//2.1e-101:495:97//AF054179
F-NT2RP2004841//Human DNA sequence from cosmid J138O17, between markers DXS6791 and DXS8038 on chromosome X contains EST CA repeat and an endogenous retroviral like element.//7.6e-82:531:84//Z72519
F-NT2RP2004861//Fugu rubripes GSS sequence, clone 040O17bA3, genomic survey sequence.//0.96:183:64//AL025645
F-NT2RP2004897//Human Chromosome X clone bWXD187, complete sequence.//4.8e-142:710:96//AC004383
F-NT2RP2004933//Homo sapiens mRNA for ZIP-kinase, complete cds.//2.0e-82:418:95//AB007144
F-NT2RP2004936
F-NT2RP2004959//HS_3197_A2_G11_MR CIT Approved Human Genomic Sperm Library D Homo sapiens genomic clone Plate=3197 Col=22 Row=M, genomic survey sequence.//3.5e-25:218:83//AQ150183
F-NT2RP2004961//Rattus norvegicus KRAB/zinc finger suppressor protein 1 (KS1) mRNA, complete cds.//2.5e-59:339:79//U56732
F-NT2RP2004962//Human hereditary haemochromatosis region, histone 2A-like protein gene, hereditary haemochromatosis (HLA-H) gene, RoRet gene, and sodium phosphate transporter (NPT3) gene, complete cds.//3.6e-19:187:72//U91328
F-NT2RP2004967//Plasmodium falciparum MAL3P6, complete sequence.//0.0020:297:61//Z98551
F-NT2RP2004978//Chlamydomonas reinhardtii VSP-3 mRNA, complete cds.//0.22:162:69//L29029
F-NT2RP2004982//F26D4-Sp6 IGF Arabidopsis thaliana genomic clone F26D4, genomic survey sequence.//0.13:273:61//B12642
F-NT2RP2004985//Human mRNA for KIAA0144 gene, complete cds.//1.5e-20:431:65//D63478
F-NT2RP2004999
F-NT2RP2005000//R.rattus gene for beta-1 subunit of Na,K-ATPase.//0.019:240:63//X63375
F-NT2RP2005001//Homo sapiens mRNA for KIAA0615 protein, complete cds.//6.0e-159:782:97//AB014515
F-NT2RP2005003//H.sapiens Staf50 mRNA.//3.1e-42:430:75//X82200
F-NT2RP2005012//Homo sapiens SEC63 (SEC63) mRNA, complete cds.//1.4e-98:501:96//AF100141
F-NT2RP2005018//Homo sapiens PAC clone DJ0659J06 from 7q33-q35, complete sequence.//1.0:209:63//AC004849
F-NT2RP2005020
F-NT2RP2005022//Human Chromosome 3 pac pDJ70i11, WORKING DRAFT SEQUENCE, 2 unordered pieces.//3.0e-43:98:93//AC000380
F-NT2RP2005031//HS_2052_B2_G10_MR CIT Approved Human Genomic Sperm Library D Homo sapiens genomic clone Plate=2052 Col=20 Row=N, genomic survey sequence.//0.019:363:61//AQ231464
F-NT2RP2005037//Human 3' of immunoglobulin heavy chain locus (IGHA2) gene.//0.70:174:65//U64454
F-NT2RP2005038//Homo sapiens chromosome 17, clone hRPK.74_E_22, complete sequence.//0.20:519:57//AC005696
F-NT2RP2005108
F-NT2RP2005116//Homo sapiens mRNA for KIAA0664 protein, partial cds.//2.0e-103:495:98//AB014564
F-NT2RP2005126//H.sapiens mRNA for RNA helicase (Myc-regulated dead box protein).//2.9e-27:157:98//X98743
F-NT2RP2005139//Amycolatopsis mediterranei genes encoding rifamycin polyketide synthases, ORFs 1 to 5.//0.00024:547:59//AJ223012
F-NT2RP2005140//Homo sapiens chromosome 21, Neurofibromatosis 1 (NF1) related locus, complete sequence.//0.95:191:62//AC004527
F-NT2RP2005144//Homo sapiens tubby like protein 3 (TULP3) mRNA, complete cds.//2.6e-89:447:96//AF045583
F-NT2RP2005147//HS_3184_A1_E01_MR CIT Approved Human Genomic Sperm Library D Homo sapiens genomic clone Plate=3184 Col=1 Row=I, genomic survey sequence.//0.10:294:60//AQ252226
F-NT2RP2005159//H.sapiens CpG island DNA genomic Mse1 fragment, clone 132g6, forward read cpg132g6.ft1a.//1.1e-13:93:97//Z59162
F-NT2RP2005162//Caenorhabditis elegans cosmid F01F1.//2.6e-20:394:64//U13070
F-NT2RP2005168//Homo sapiens mRNA for E1B-55kDa-associated protein.//1.4e-125 :633:96//AJ007509
F-NT2RP2005204//Arabidopsis thaliana ubiquitin activating enzyme (UBA1) gene, complete cds.//0.00016:316:60//U80808
F-NT2RP2005227//Homo sapiens PAC clone DJ0905J08 from 7p12-p14, complete sequence.//0.51:52:92//AC005189
F-NT2RP2005239//S.pombe chromosome II cosmid c21D10.//1.3e-22:356:67//AL031536
F-NT2RP2005254
F-NT2RP2005270//H.sapiens genomic DNA (chromosome 3; clone NL197R).//0.58:132:65//X87513
F-NT2RP2005276//Rat mRNA for brain acyl-CoA synthetase II, complete cds.//9.0e-103:656:85//D30666
F-NT2RP2005287//Cavia porcellus zinc finger protein (zfoC1) mRNA, complete cds.//3.4e-37:302:84//L26335
F-NT2RP2005288//Homo sapiens RCC1-like G exchanging factor RLG mRNA, complete cds.//7.1e-122:604:96//AF060219
F-NT2RP2005289//Homo sapiens mRNA for XRP2 protein.//4.0e-140:670:98//AJ007590
F-NT2RP2005293//HS_3245_B1_E10_T7 CIT Approved Human Genomic Sperm Library D Homo sapiens genomic clone Plate=3245 Col=19 Row=J, genomic survey sequence.//8.2e-37:223:92//AQ217454
F-NT2RP2005315//Homo sapiens mRNA for KIAA0676 protein, partial cds.//1.1e-95:483:96//AB014576
F-NT2RP2005325//Human LIM-homeobox domain protein (hLH-2) mRNA, complete cds.//8.2e-22:166:90//U11701
F-NT2RP2005336//Homo sapiens snRNA activating protein complex 190kD subunit (SNAP190) mRNA, complete cds.//0.39:353:62//AF032387
F-NT2RP2005344//Homo sapiens mRNA for KIAA0566 protein, partial cds.//8.8e-29:456:66//AB011138
F-NT2RP2005354//Human DNA sequence from PAC 435C23 on chromosome X. Contains ESTs.//0.72:431:61//Z92844
F-NT2RP2005358//Homo sapiens methyl-CpG binding protein MBD3 (MBD3) mRNA, complete cds.//4.7e-99:489:96//AF072247
F-NT2RP2005360//Pan troglodytes huntingtin gene, partial exon.//0.93:105:67//L49358
F-NT2RP2005393//Rat parathyroid hormone receptor mRNA, complete cds.//2.4e-08:97:83//M77184
F-NT2RP2005407
F-NT2RP2005436//Homo sapiens chromosome 16, cosmid clone 2H2 (LANL), complete sequence.//0.014:235:62//AC005346
F-NT2RP2005441//CIT-HSP-2338P5.TR CIT-HSP Homo sapiens genomic clone 2338P5, genomic survey sequence.//4.0e-107:532:97//AQ055548
F-NT2RP2005453//F21C16TFC IGF Arabidopsis thaliana genomic clone F21C16, genomic survey sequence.//1.0:239:61//B97865
F-NT2RP2005457//B.taurus CI-B14.5b mRNA for NADH dehydrogenase (ubiquinone).//4.7e-25:245:79//X68647
F-NT2RP2005464//Human DNA sequence from clone 836E8 on chromosome 20p12 Contains EST, CA repeat, STS, GSS, retroviral sequence, complete sequence.//4.6e-111:724:86//AL031679
F-NT2RP2005465//Homo sapiens chromosome 19, BAC CIT-B-393i15 (BC301323), complete sequence.//6.5e-18:152:75//AC006116
F-NT2RP2005472//Human DNA sequence from clone 1118D24 on chromosome 1p36.11-36.33. Contains part of a novel gene similar to worm genes T08G11.1 and C25H3.9, part of a 60S Ribosomal Protein L10 LIKE (pseudo)gene and two 3' exons of the TNFR2 gene for Tumor Necrosis Factor Receptor 2 (75 kD) (TNF Binding Protein 2, TBPII, TNF-R2, CD120B, TNFBR). Contains ESTs, STSs, GSSs, genomic marker D1S434 and a ca repeat polymorphism, complete sequence.//4.4e-12:89:97//AL031276
F-NT2RP2005476//Homo sapiens BAC clone RG293F17 from 7p15-p21, complete sequence.//4.3e-40:463:73//AC004130
F-NT2RP2005490//Homo sapiens clone NH0001P09, WORKING DRAFT SEQUENCE, 1 unordered pieces.//3.2e-115:228:99//AC006030
F-NT2RP2005491//HS_2253_A2_G10_MR CIT Approved Human Genomic Sperm Library D Homo sapiens genomic clone Plate=2253 Col=20 Row=M, genomic survey sequence.//4.6e-23:234:80//AQ116847
F-NT2RP2005495
F-NT2RP2005496//HS_3064_A1_F08_MR CIT Approved Human Genomic Sperm Library D Homo sapiens genomic clone Plate=3064 Col=15 Row=K, genomic survey sequence.//5.3e-90:436:98//AQ143097
F-NT2RP2005498//Rabbit protein phosphatase 2A beta subunit mRNA, complete cds.//1.4e-63:503:78//M64931
F-NT2RP2005501//Homo sapiens chromosome 10 clone CIT987SK-1143A11 map 10q25, complete sequence.//0.86:183:63//AC005880
F-NT2RP2005509//Homo sapiens cosmid LM1937 from Xq28.//1.0:160:65//U82695
F-NT2RP2005520//Homo sapiens chromosome-associated protein-E (hCAP-E) mRNA, complete cds.//3.9e-81:444:92//AF092563
F-NT2RP2005525//Homo sapiens mRNA for KIAA0764 protein, complete cds.//6.9e-18:112:99//AB018307
F-NT2RP2005531//Human structural protein 4.1 mRNA, complete cds.//1.1e-06:282:60//M14993
F-NT2RP2005539//Homo sapiens mRNA for NS1-binding protein (NS1-BP).//2.9e-153:747:97//AJ012449
F-NT2RP2005540//Homo sapiens mRNA for KIAA0494 protein, complete cds.//5.9e-130:618:98//AB007963
F-NT2RP2005549//Mus musculus clone OST142, genomic survey sequence.//3.1e-43:277:89//AF046734
F-NT2RP2005555//HS_2188_A2_D04_MR CIT Approved Human Genomic Sperm Library D Homo sapiens genomic clone Plate=2188 Col=8 Row=G, genomic survey sequence.//8.0e-05:195:65//AQ086723
F-NT2RP2005557//Homo sapiens clone 486790 diphosphoinositol polyphosphate phosphohydrolase mRNA, complete cds.//2.5e-44:473:71//AF062529
F-NT2RP2005581//Homo sapiens BAC clone GS180J15 from 7q31, complete sequence./0.99:213:65//AC005016
F-NT2RP2005600//H.sapiens CpG island DNA genomic Mse1 fragment, clone 172d12, reverse read cpg172d12.rt1a.//0.32:134:63//Z57359
F-NT2RP2005605
F-NT2RP2005620//Homo sapiens epsin 2a mRNA, complete cds.//9.8e-91:447:97//AF062085
F-NT2RP2005622
F-NT2RP2005635//Saccharomyces cerevisiae chromosome VIII cosmid 9205.//8.6e-17:411:61//U10556
F-NT2RP2005637//NATI (NATI*10)=acetyltransferase 1 {3' region, polyadenylation polymorphism} [human, unrelated Caucasians, mRNA Partial Mutant, 300 nt].//0.22:156:65//S78829
F-NT2RP2005640//Mouse U6 RNA gene.//5.5e-19:249:76//X06980
F-NT2RP2005645//HS_2201_B2_D07_MF CIT Approved Human Genomic Sperm Library D Homo sapiens genomic clone Plate=2201 Col=14 Row=H, genomic survey sequence.//0.30:159:65//AQ066763
F-NT2RP2005651//H.sapiens DNA sequence.//0.00037:150:66//Z22493
F-NT2RP2005654//Homo sapiens mRNA for KIAA0288 gene, complete cds.//4.7e-07:351:62//AB006626
F-NT2RP2005669//Homo sapiens KE05 protein mRNA, complete cds.//8.2e-98:472:98//AF064605
F-NT2RP2005675//Homo sapiens growth suppressor related (DOC-1R) mRNA, complete cds.//2.4e-94:462:98//AF089814
F-NT2RP2005683//HS-1024-B1-H05-MF.abi CIT Human Genomic Sperm Library C Homo sapiens genomic clone Plate=CT 803 Col=9 Row=P, genomic survey sequence.//0.99:156:64//B34405
F-NT2RP2005690//Human pyrroline 5-carboxylate reductase mRNA, complete cds.//7.7e-10:328:61//M77836
F-NT2RP2005694
F-NT2RP2005701//Homo sapiens 12p13.3 BAC RPCI11-288K12 (Roswell Park Cancer Institute Human BAC Library) complete sequence.//0.72:160:65//AC005183
F-NT2RP2005712//Homo sapiens mRNA for KIAA0799 protein, partial cds.//1.6e-124:599:97//AB018342
F-NT2RP2005719//R.norvegicus mRNA for metallothionein-III.//0.86:117:64//X89603
F-NT2RP2005722//Human zinc finger protein ZNF136.//2.6e-44:415:77//U09367
F-NT2RP2005723//Human BAC clone GS542D18 from 7q31-q32, complete sequence.//6.9e-15:153:81//AC002528
F-NT2RP2005726//Homo sapiens clone DJ0577P23, WORKING DRAFT SEQUENCE, 28 unordered pieces.//5.1e-41:138:95//AC005627
F-NT2RP2005732//Human DNA sequence *** SEQUENCING IN PROGRESS *** from clone 291J10, WORKING DRAFT SEQUENCE.//0.61:303:60//Z93017
F-NT2RP2005741//Homo sapiens PALM gene, exon 1 and joined CDS.//0.52:116:67//Y16270
F-NT2RP2005748//Human Kox11 mRNA for zinc finger protein, partial.//0.11:136:66//X52342
F-NT2RP2005752//Homo sapiens TNFR-related death receptor-6 (DR6) mRNA, complete cds.//7.8e-22:134:96//AF068868
F-NT2RP2005753//Homo sapiens I-1 receptor candidate protein mRNA, complete cds.//1.2e-100:486:98//AF082516
F-NT2RP2005763//Human mRNA for KIAA0111 gene, complete cds.//0.00073:425:56//D21853
F-NT2RP2005767//G.gallus PB1 gene.//2.1e-73:544:80//X90849
F-NT2RP2005773//Human pyrroline 5-carboxylate reductase mRNA, complete cds.//6.2e-15:153:82//M77836
F-NT2RP2005775//Sus scrofa mRNA for soluble angiotesin-binding protein, complete cds.//1.2e-121:649:88//D11336
F-NT2RP2005781//Pseudomonas aeruginosa gene for MexX and MexY, complete cds//0.96:184:60//AB015853
F-NT2RP2005784//Human DNA sequence *** SEQUENCING IN PROGRESS *** from clone 1185N5, WORKING DRAFT SEQUENCE.//1.9e-63:222:96//AL034423
F-NT2RP2005804//Oryza sativa glycine-rich protein (OSGRP1) mRNA, complete cds.//2.6e-07:232:64//AF010579
F-NT2RP2005812
F-NT2RP2005815//Streptomyces sp. gene for alkaline serine protease I.//0.031:358:59//X74103
F-NT2RP2005835//Rattus norvegicus mRNA for p47, complete cds.//2.5e-107:449:91//AB002086
F-NT2RP2005841//Human DNA sequence from cosmid U209G1 on chromosome X.//5.1e-05:144:73//Z68873
F-NT2RP2005853//RPCI11-24D4.TKBF RPCI-11 Homo sapiens genomic clone RPCI-11-24D4, genomic survey sequence.//6.4e-13:130:85//AQ013490
F-NT2RP2005857//Homo sapiens chromosome-associated protein-C (hCAP-C) mRNA, partial cds.//1.7e-174:829:98//AF092564
F-NT2RP2005859//Human DNA sequence *** SEQUENCING IN PROGRESS *** from clone 914P20, WORKING DRAFT SEQUENCE.//0.25:174:62//AL034553
F-NT2RP2005868//Fugu rubripes GSS sequence, clone 103I24aF4, genomic survey sequence.//7.8e-06:92:79//AL027276
F-NT2RP2005886//HS_3187_A2_D08_T7 CIT Approved Human Genomic Sperm Library D Homo sapiens genomic clone Plate=3187 Col=16 Row=G, genomic survey sequence.//7.1e-95:494:95//AQ155885
F-NT2RP2005890//Mouse oncogene (ect2) mRNA, complete cds.//2.7e-32:660:66//L11316
F-NT2RP2005901//H.sapiens CpG island DNA genomic Mse1 fragment, clone 15b5, reverse read cpg15b5.rt1a.//0.0026:66:84//Z54729
F-NT2RP2005908//Homo sapiens 12q13.1 PAC RPCI3-197B17 (Roswell Park Cancer Institute Human PAC library) complete sequence.//6.4e-49:481:75//AC004241
F-NT2RP2005933//Rattus norvegicus nucleoporin p54 mRNA, complete cds.//6.6e-61:657:73//U63840
F-NT2RP2005942//H.sapiens PAP mRNA.//1.6e-46:618:67//X76770
F-NT2RP2005980//Homo sapiens chromosome 17, clone hRPC.1081_P_3, complete sequence.//1.0e-48:533:71//AC005207
F-NT2RP2006023//HS_3048_A1_A11_MF CIT Approved Human Genomic Sperm Library D Homo sapiens genomic clone Plate=3048 Col=21 Row=A, genomic survey sequence.//2.1e-25:167:91//AQ126553
F-NT2RP2006038//CIT-HSP-384K4.TR CIT-HSP Homo sapiens genomic clone 384K4, genomic survey sequence.//3.9e-06:102:74//B51912
F-NT2RP2006043//Human intercrine-alpha (hIRH) mRNA, complete cds.//1.9e-05:418:59//U19495
F-NT2RP2006052//Peromyscus polionotus ammobates dinucleotide microsatellite Ppa55.//0.0035:226:65//AF016861
F-NT2RP2006069//Human HepG2 partial cDNA, clone hmd3g02m5.//3.9e-11:121:85//D17047
F-NT2RP2006071
F-NT2RP2006098//Homo sapiens chromosome 21q22.2, cosmid D13C2, complete sequence.//0.46:264:59//AF027207
F-NT2RP2006100//Human Chromosome X, complete sequence.//3.2e-94:488:95//AC004073
F-NT2RP2006103//HS_2254_A2_D02_MR CIT Approved Human Genomic Sperm Library D Homo sapiens genomic clone Plate=2254 Col=4 Row=G, genomic survey sequence.//5.7e-27:156:96//AQ129602
F-NT2RP2006106//Human Chromosome 11 pac pDJ1173a5, complete sequence./11.2e-62:655:71//AC000378
F-NT2RP2006141//Human DNA sequence *** SEQUENCING IN PROGRESS *** from clone 537K23, WORKING DRAFT SEQUENCE.//1.2e-69:316:98//AL034405
F-NT2RP2006166//Homo sapiens chromosome 4 clone B32I8, complete sequence.//3.1e-45:387:81//AC004063
F-NT2RP2006184//Cricetulus griseus beta-1,6-N-acetylglucosaminyltransferase Lec4A cell line point mutant mRNA, complete cds.//0.99:111:73//U62587
F-NT2RP2006186//Homo Sapiens mRNA for KIAA0654 protein, partial cds.//7.8e-113:567:96//AB014554
F-NT2RP2006196//Homo sapiens clone DJ1189D06, complete sequence.//2.8e-28:718:62//AC005232
F-NT2RP2006200//Homo sapiens chromosome 12p13.3 clone RPCI1-96H9, WORKING DRAFT SEQUENCE, 66 unordered pieces.//6.5e-83:239:94//AC006057
F-NT2RP2006219//H.sapiens mRNA for DGCR6 protein.//1.4e-116:618:93//X96484
F-NT2RP2006237//CIT-HSP-2300P9.TR CIT-HSP Homo sapiens genomic clone 2300P9, genomic survey sequence.//2.0e-18:118:97//AQ012480
F-NT2RP2006238//Rattus norvegicus CTD-binding SR-like protein rA8 mRNA, complete cds.//7.6e-102:635:86//U49055
F-NT2RP2006258//RPCI11-9N9.TP RPCI-11 Homo sapiens genomic clone RPCI-11-9N9, genomic survey sequence.//8.6e-05:181:63//B71615
F-NT2RP2006261//H.sapiens mRNA for serine/threonine protein kinase EMK.//0.44:111:71//X97630
F-NT2RP2006275//Pseudorabies virus UL[5,6,7,8,8.5,9,10,11,12,13] genes.//2.0e-05:501:59//X97257
F-NT2RP2006312//Homo sapiens BAF57 (BAF57) gene, complete cds.//2.7e-138:679:97//AF035262
F-NT2RP2006320//P.falciparum pfmdr1 gene.//0.00013:425:60//X56851
F-NT2RP2006321//Homo sapiens DNA from chromosome 19, BAC 33152, complete sequence.//4.1e-19:545:62//AC003973
F-NT2RP2006323//Human DNA sequence *** SEQUENCING IN PROGRESS *** from clone 745I14, WORKING DRAFT SEQUENCE.//8.9e-18:131:90//AL033532
F-NT2RP2006333//Homo sapiens PAC clone DJ0808A01 from 7q21.1-q31.1, complete sequence.//6.2e-125:602:98//AC004893
F-NT2RP2006334//Homo sapiens chromosome 19, cosmid R27139, complete sequence.//2.1e-06:241:65//AC005514
F-NT2RP2006365//Fugu rubripes GSS sequence, clone 171K15aC5, genomic survey sequence.//7.8e-06:148:70//AL029590
F-NT2RP2006393//Human DNA sequence from clone 80I19 on chromosome 6p21.31-22.2 Contains genes and pseudogenes for olfactory receptor-like proteins, STS, GSS, complete sequence.//6.8e-06:167:70//AL022727
F-NT2RP2006436//Human DNA sequence *** SEQUENCING IN PROGRESS *** from clone Y313F4, WORKING DRAFT SEQUENCE.//4.2e-92:363:84//AL023808
F-NT2RP2006441
F-NT2RP2006454//Sequence 8 from Patent WO9517522.//2.9e-06:180:66//A45338
F-NT2RP2006456
F-NT2RP2006464//Homo sapiens mRNA for AND-1 protein.//3.4e-148:545:98//AJ006266
F-NT2RP2006467//Sus scrofa IgM heavy chain gene, switch region and exons encoding ch1-ch4 and secretion domains, partial cds.//0.061:201:66//U50149
F-NT2RP2006472
F-NT2RP2006534//Human DNA sequence from clone 272E8 on chromosome Xp22.13-22.31. Contains a pseudogene similar to MDM2-Like P53-binding protein gene. Contains STSs, GSSs and a CA repeat polymorphism, complete sequence.//8.8e-10:273:66//Z93929
F-NT2RP2006554//Human DNA mismatch repair protein homolog (hMLH1) gene, exon 6.//0.71:174:59//U40965
F-NT2RP2006565//Homo sapiens secretory carrier-associated membrane protein (SCAMP) mRNA, complete cds.//6.6e-114:669:90//AF038966
F-NT2RP2006571//Rabbit cytochrome P-450 isozyme 2 (type B2) mRNA, complete cds, clone B2-1.//6.0e-26:503:63//M20855
F-NT2RP2006573//Molluscum contagiosum virus subtype 1, complete genome.//0.44:134:71//U60315
F-NT2RP2006598//Human BRCA2 region, mRNA sequence CG033.//5.0e-16:140:85//U50537
F-NT2RP3000002//***ALU WARNING: Human Alu-Sc subfamily consensus sequence.//3.8e-32:214:89//U14571
F-NT2RP3000031//Homo sapiens mRNA for histone deacetylase-like protein (JM21).//5.8e-136:637:98//AJ011972
F-NT2RP3000046//Bovine herpesvirus type 1 early-intermediate transcription control protein (BICP4) gene, complete cds.//5.4e-05:571:60//L14320
F-NT2RP3000047
F-NT2RP3000050//Figure 2. Nucleotide and translated protein sequences of HPF1, -2, and-9.//1.0e-67:626:74//M27877
F-NT2RP3000055//Genomic sequence from Human 9q34, complete sequence.//3.5e-10:394:64//AC001227
F-NT2RP3000068
F-NT2RP3000072//Homo sapiens BAC clone RG290G13 from 7q21, complete sequence.//1.0:301:61//AC004746
F-NT2RP3000080//Human DNA sequence *** SEQUENCING IN PROGRESS *** from clone 102D24, WORKING DRAFT SEQUENCE.//1.9e-44:297:79//AL021391
F-NT2RP3000085//Arabidopsis thaliana 3-methylcrotonyl-CoA carboxylase precursor mRNA, complete cds.//4.5e-33:528:65//U12536
F-NT2RP3000092//RPCI11-22M5.TV RPCI-11 Homo sapiens genomic clone RPCI-11-22M5, genomic survey sequence.//3.3e-27:157:97//B84237
F-NT2RP3000109//Arabidopsis thaliana 1-amino-1-cyclopropanecarboxylate synthase (ACS5) gene, complete cds.//0.92:185:64//L29260
F-NT2RP3000134//Homo sapiens PAC clone DJ0905J08 from 7p12-p14, complete sequence.//1.2e-112:286:89//AC005189
F-NT2RP3000142//Homo sapiens mRNA for KIAA0592 protein, partial cds.//9.0e-181:849:98//AB011164
F-NT2RP3000149//Homo sapiens chromosome 17, clone hRPK.264_B_14, complete sequence.//4.2e-24:155:94//AC005884
F-NT2RP3000186//Human DNA sequence *** SEQUENCING IN PROGRESS *** from clone 500L14, WORKING DRAFT SEQUENCE.//7.2e-43:269:81//AL023583
F-NT2RP3000197//Homo sapiens interleukin 9 receptor (IL9R) pseudogene, exons 1-9.//0.098:405:57//L39063
F-NT2RP3000207//Drosophila melanogaster DNA sequence (P1 DS00164 (D269)), complete sequence.//0.96:608:55//AC004716
F-NT2RP3000220
F-NT2RP3000233//Homo sapiens actin binding protein MAYVEN mRNA, complete cds.//2.0e-18:509:58//AF059569
F-NT2RP3000235//Mouse Cosmid ma53a016 from 14D1-D2, complete sequence.//3.5e-05:224:65//AC004101
F-NT2RP3000247//Human mRNA for KIAA0218 gene, complete cds.//2.1e-109:691:86//D86972
F-NT2RP3000251//Caenorhabditis elegans cosmid ZK930, complete sequence.//0.20:119:68//Z70213
F-NT2RP3000252//Homo sapiens cosmid 1F1, complete sequence.//9.8e-78:174:88//AF065393
F-NT2RP3000255
F-NT2RP3000267
F-NT2RP3000299//Mus musculus Crk-associated substrate (Cas-b) mRNA, complete cds.//5.9e-48:374:82//U48853
F-NT2RP3000312//Fruit fly (D.melanogaster) Glued mRNA, complete cds.//4.9e-22:583:63//J02932
F-NT2RP3000320//RPCI11-36J1.TP RPCI-11 Homo sapiens genomic clone RPCI-11-36J1, genomic survey sequence.//4.4e-06:87:88//AQ047107
F-NT2RP3000324//Rattus norvegicus potassium channel regulator 1 mRNA, complete cds.//5.5e-26:283:79//U78090
F-NT2RP3000333//Human DNA sequence *** SEQUENCING IN PROGRESS *** from clone 973M2, WORKING DRAFT SEQUENCE.//1.0:309:60//AL033533
F-NT2RP3000341//Homo sapiens DNA sequence from PAC 95C20 on chromosome Xp11.3-11.4. Contains STSs and the DXS7 locus with GT and GTG repeat polymorphisms, complete sequence.//6.7e-42:465:74//Z97181
F-NT2RP3000348
F-NT2RP3000350//Homo sapiens cosmid 1F1, complete sequence.//3.4e-79:174:88//AF065393
F-NT2RP3000359//Bovine mitochondrial GTP:AMP phosphotransferase mRNA, complete cds.//2.2e-127:816:85//M25757
F-NT2RP3000361//Schizosaccharomyces pombe DNA for pre-mRNA splicing factor, complete cds.//0.0075:288:58//D83743
F-NT2RP3000366//Mus musculus ras-related protein (rab18) mRNA, complete cds.//7.1e-134:693:94//L04966
F-NT2RP3000393//Rattus norvegicus mRNA for GABA-B R2 receptor.//0.049:308:60//AJ011318
F-NT2RP3000397//S.cerevisiae chromosome VII reading frame ORF YGL120c.//0.00012:441:58//Z72642
F-NT2RP3000403//Homo sapiens formin binding protein 21 mRNA, complete cds.//5.0e-174:841:97//AF071185
F-NT2RP3000418//Homo sapiens chromosome 17, clone hRPK.1053_B_8, complete sequence.//7.9e-53:817:68//AC006083
F-NT2RP3000433//Human DNA sequence *** SEQUENCING IN PROGRESS *** from clone 862K6, WORKING DRAFT SEQUENCE.//6.1e-31:590:63//AL031681
F-NT2RP3000439//Fugu rubripes GSS sequence, clone 075E22aB10, genomic survey sequence.//4.0e-19:169:81//AL026471
F-NT2RP3000441//Human DNA sequence from PAC 93H18 on chromosome 6 contains ESTs heterochromatin protein HP1Hs-gamma pseudogene, STS and CpG island.//2.4e-41:459:65//Z84488
F-NT2RP3000449//Human DNA sequence *** SEQUENCING IN PROGRESS *** from clone 1018D12, WORKING DRAFT SEQUENCE.//1.1e-100:365:87//AL031650
F-NT2RP3000451//HS_2024_A1_E10_T7 CIT Approved Human Genomic Sperm Library D Homo sapiens genomic clone Plate=2024 Col=19 Row=I, genomic survey sequence.//0.011:367:57//AQ229420
F-NT2RP3000456//CIT-HSP-2338P5.TR CIT-HSP Homo sapiens genomic clone 2338P5, genomic survey sequence.//1.5e-89:458:96//AQ055548
F-NT2RP3000484//Human DNA sequence *** SEQUENCING IN PROGRESS *** from clone 90L6, WORKING DRAFT SEQUENCE.//0.043:147:70//Z97353
F-NT2RP3000487//H.sapiens CpG island DNA genomic Mse1 fragment, clone 11b11, forward read cpg11b11.ft1a.//1.7e-11:96:92//Z64440
F-NT2RP3000512//Human HOX2G mRNA from the Hox2 locus.//9.7e-17:109:97//X16667
F-NT2RP3000526//Homo sapiens full-length insert cDNA clone YZ38E04.//4.1e-30:283:76//AF086071
F-NT2RP3000527//Human mRNA for KIAA0211 gene, complete cds.//2.5e-34:706:63//D86966
F-NT2RP3000531//Mus musculus immunosuperfamily protein B12 mRNA, complete cds.//1.9e-14:220:70//AF061260
F-NT2RP3000542//Human Chromosome 11p11.2 PAC clone pDJ404m15, complete sequence.//0.00019:361:60//AC002554
F-NT2RP3000561//Homo sapiens PAC clone DJ0942I16 from 7q11, complete sequence.//9.0e-171:827:98//AC006012
F-NT2RP3000562
F-NT2RP3000578//F.rubripes GSS sequence, clone 013G07cE7, genomic survey sequence.//1.7e-25:284:74//AL011271
F-NT2RP3000582//CIT978SK-A-56H4.TP CIT978SK Homo sapiens genomic clone A-56H4, genomic survey sequence.//5.8e-07:239:66//B73597
F-NT2RP3000584
F-NT2RP3000590//H.sapiens CpG island DNA genomic Mse1 fragment, clone 170d7, forward read cpg170d7.ft1a.//3.0e-22:128:100//Z59723
F-NT2RP3000592//CIT-HSP-2288J7.TR CIT-HSP Homo sapiens genomic clone 2288J7, genomic survey sequence.//2.2e-78:382:98//B98868
F-NT2RP3000596//CIT-HSP-2375J10.TR CIT-HSP Homo sapiens genomic clone 2375J10, genomic survey sequence.//0.00076:143:67//AQ109305
F-NT2RP3000599//Caenorhabditis elegans cosmid T19B10, complete sequence.//1.2e-13:295:66//Z74043
F-NT2RP3000603//Bovine herpesvirus type 1 early-intermediate transcription control protein (BICP4) gene, complete cds.//0.37:520:57//L14320
F-NT2RP3000605//Homo sapiens chromosome 19, cosmid F20900, complete sequence.//8.8e-155:526:97//AC006128
F-NT2RP3000622//HS_3213_A2_D02_T7 CIT Approved Human Genomic Sperm Library D Homo sapiens genomic clone Plate=3213 Col=4 Row=G, genomic survey sequence.//4.1e-29:238:85//AQ175104
F-NT2RP3000624//Homo sapiens clone DJ0800G07, complete sequence.//0.47:75:80//AC004890
F-NT2RP3000628//Human DNA sequence from clone 581F12 on chromosome Xq21. Contains Eukaryotic Translation Initiation Factor EIF3 P35 Subunit and 60S Ribosomal protein L22 pseudogenes. Contains ESTs, complete sequence.//0.078:393:58//AL031313
F-NT2RP3000632//Human zinc finger protein zfp6 (ZF6) mRNA, partial cds.//1.4e-96:541:79//U71363
F-NT2RP3000644//Homo sapiens clone RG315H11, WORKING DRAFT SEQUENCE, 5 unordered pieces.//5.2e-46:421:77//AC005089
F-NT2RP3000661
F-NT2RP3000665//Human DNA sequence from clone 1191B2 on chromosome 22q13.2-13.3. Contains part of the BIK (NBK, BP4, BIP1) gene for BCL2-interacting killer (apoptosis-inducing), a 40S Ribososmal Protein S25 pseudogene and part of an alternatively spliced novel Acyl Transferase gene similar to C. elegans C50D2.7. Contains ESTs, STSs, GSSs, two putative CpG islands and genomic marker D22S1151, complete sequence.//1.7e-11:292:65//AL022237
F-NT2RP3000685//H.sapiens mRNA for novel protein.//2.4e-80:460:92//X99961
F-NT2RP3000690//H.sapiens flow-sorted chromosome 6 TaqI fragment, SC6pA10F6.//1.0:141:65//Z77872
F-NT2RP3000736//Human mRNA for KIAA0140 gene, complete cds.//6.1e-20:127:96//D50930
F-NT2RP3000739//Rattus norvegicus golgi peripheral membrane protein p65 (GRASP65) mRNA, complete cds.//1.1e-46:622:67//AF015264
F-NT2RP3000742//Rattus norvegicus phospholipase C delta-4 mRNA, complete cds.//4.7e-37:429:70//U16655
F-NT2RP3000753
F-NT2RP3000759//Caenorhabditis elegans cosmid Y57G11C, complete sequence.//2.8e-38:519:69//Z99281
F-NT2RP3000815//HS_2237_A2_D12_MF CIT Approved Human Genomic Sperm Library D Homo sapiens genomic clone Plate=2237 Col=24 Row=G, genomic survey sequence.//0.79:151:61//AQ067252
F-NT2RP3000825//Campanula ramosa chloroplast NADH dehydrogenase (ndhF) gene, complete cds.//0.36:378:58//L39387
F-NT2RP3000826//Suid herpesvirus 1 Kaplan glycoprotein L (UL1) and uracil-DNA glycosylase (UL2) genes, complete cds, and (UL3) gene, partial cds.//0.0025:291:62//U02513 F-NT2RP3000836//Mouse complement factor H-related protein mRNA, complete cds, clone 9C4.//0.69:563:57//M29009
F-NT2RP3000841//Human DNA sequence from PAC 121G13 on chromosome 6 contains flow sorted chromosome 6 HindIII fragment ESTs. polymorphic CA repeat, CpG island, CpG island genomic fragments.//2.1e-46:666:68//Z86062
F-NT2RP3000845//Homo sapiens chromosome 19, cosmid R31237, complete sequence.//3.4e-92:193:93//AC005581
F-NT2RP3000847//Human HepG2 3' region cDNA, clone hmd5d02.//3.4e-32:261:81//D16938
F-NT2RP3000850//Homo sapiens clone RG271G13, WORKING DRAFT SEQUENCE, 7 unordered pieces.//5.1e-44:358:81//AC005082
F-NT2RP3000852//Homo sapiens DNA sequence from PAC 117P20 on chromosome 1q24. Contains the LNHR (SELL) gene coding for Lymph Node Homing Receptor (L-Selectin precursor, LAM-1 Leukocyte Adhesion Molecule, Leukocyte surface antigen Leu-8, TQ1, GP90-MEL, LECAM1 Leukocyte-Endothelial Cell Adhesion Molecule 1, CD62L). Contains the SELE gene coding for E-Selectin precursor (CD62E, ELAM-1 Endothelial Leukocyte Adhesion Molecule 1, LECAM-2 Leukocyte-Endothelial Cell Adhesion Molecule 2). Contains an unknown gene with homology to predicted yeast. plant and worm proteins. Contains ESTs and STSs, complete sequence.//4.4e-123:150:98//AL021940
F-NT2RP3000859//T19M2TF TAMU Arabidopsis thaliana genomic clone T19M2, genomic survey sequence.//0.016:185:65//B60831
F-NT2RP3000865
F-NT2RP3000868//Human ovarian cancer downregulated myosin heavy chain homolog (Doc1) mRNA, complete cds.//2.0e-29:766:60//U53445
F-NT2RP3000869//H.sapiens gene for plectin.//1.1e-12:700:60//Z54367
F-NT2RP3000875//HS_2236_B1_G10_MF CIT Approved Human Genomic Sperm Library D Homo sapiens genomic clone Plate=2236 Col=19 Row=N, genomic survey sequence.//0.98:153:68//AQ154007
F-NT2RP3000901//Human herpesvirus 2 glycoprotein B precursor (UL27) gene, complete cds.//0.44:213:65//AF021340
F-NT2RP3000904//Rat Na+ channel mRNA, 3' end.//3.6e-106:505:99//M27223
F-NT2RP3000917//Mouse mRNA for Dhm1 protein, complete cds.//3.1e-132:691:93//D38517
F-NT2RP3000919//Rattus norvegicus golgi peripheral membrane protein p65 (GRASP65) mRNA, complete cds.//3.2e-97:585:88//AF015264
F-NT2RP3000968//Human Chromosome 16 BAC clone CIT987SK-A-234F9, complete sequence.//5.8e-70:181:89//U91326
F-NT2RP3000980//R.norvegicus CYP3A1 gene, 5' flanking region.//6.1e-26:507:66//X98335
F-NT2RP3000994//HS-1049-B2-F03-MF.abi CIT Human Genomic Sperm Library C Homo sapiens genomic clone Plate=CT 771 Col=6 Row=L, genomic survey sequence.//1.5e-22:128:100//B39529
F-NT2RP3001004//H.sapiens CpG island DNA genomic Mse1 fragment, clone 39c1, reverse read cpg39c1.rt1a./15.9e-27:150:99//Z60925
F-NT2RP3001007//Homo sapiens clone NH0319F03, WORKING DRAFT SEQUENCE, 3 unordered pieces.//0.11:610:57//AC006039
F-NT2RP3001055//Drosophila melanogaster; Chromosome 2R; Region 47F1-47F7; P1 clone DS02304, WORKING DRAFT SEQUENCE, 5 unordered pieces.//1.8e-23:352:67//AC005653
F-NT2RP3001057//H.sapiens HZF4 mRNA for zinc finger protein.//1.4e-49:437:77//X78927
F-NT2RP3001081//Homo sapiens RCC1-like G exchanging factor RLG mRNA, complete cds.//8.4e-50:534:74//AF060219
F-NT2RP3001084//Homo sapiens mRNA for KIAA0782 protein, partial cds.//1.2e-14:474:60//AB018325
F-NT2RP3001096//CIT-HSP-2305P8.TF CIT-HSP Homo sapiens genomic clone 2305P8, genomic survey sequence.//3.4e-37:222:93//AQ021278
F-NT2RP3001107//Human mRNA for KIAA0215 gene, complete cds.//8.5e-33:712:64//D86969
F-NT2RP3001109//Human Chromosome 15q26.1 PAC clone pDJ457j11 containing DNA polymerase gamma (polg) gene, complete sequence.//2.7e-116:186:99//AC005317
F-NT2RP3001111
F-NT2RP3001113//Human DNA sequence from cosmid U157D4, between markers DXS366 and DXS87 on chromosome X.//2.4e-05:702:58//Z68871
F-NT2RP3001115//Homo sapiens PAC clone DJ0905J08 from 7p12-p14, complete sequence.//1.9e-170:821:98//AC005189
F-NT2RP3001116//HS_3075_A1_F01_MF CIT Approved Human Genomic Sperm Library D Homo sapiens genomic clone Plate=3075 Col=1 Row=K, genomic survey sequence.//7.3e-49:290:92//AQ120581
F-NT2RP3001119//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//1.4e-121:598:97//AL031864
F-NT2RP3001120//Human zinc finger protein ZNF136.//7.4e-76:687:75//U09367
F-NT2RP3001126//Bovine herpesvirus type 1 DNA for UL36, UL37, UL38, UL39, UL40 and UL41.//6.8e-05:344:64//Z49078
F-NT2RP3001133//Nephila clavipes minor ampullate silk protein MiSp1 mRNA, partial cds.//0.00021:529:60//AF027735
F-NT2RP3001140//Homo sapiens mRNA for KIAA0762 protein, partial cds.//3.6e-179:851:98//AB018305
F-NT2RP3001147//RPCI11-3M16.TP RPCI-11 Homo sapiens genomic clone RPCI-11-3M16, genomic survey sequence.//2.1e-15:106:96//B48859
F-NT2RP3001150//Human DNA sequence *** SEQUENCING IN PROGRESS *** from clone 423B22, WORKING DRAFT SEQUENCE.//2.0e-159:418:95//AL034379
F-NT2RP3001155//Homo sapiens mRNA for AND-1 protein.//5.1e-190:891:98//AJ006266
F-NT2RP3001176//Human DNA sequence from clone 879K22 on chromosome 1q32.1-41 Contains GSS, complete sequence.//1.1e-69:207:97//AL034351
F-NT2RP3001214//Plasmodium falciparum 3D7 chromosome 12 PFYACB8-628 genomic sequence, WORKING DRAFT SEQUENCE, 9 unordered pieces.//0.16:475:58//AC005507
F-NT2RP3001216//Homo sapiens clone DJ0635O05, WORKING DRAFT SEQUENCE, 7 unordered pieces.//3.3e-05:561:56//AC004845
F-NT2RP3001221
F-NT2RP3001232//Mouse mRNA for serine protease PC6, comlete cds.//1.0e-11:120:87//D12619
F-NT2RP3001236
F-NT2RP3001239//Mouse MAP1B mRNA for MAP1B microtubule-associated protein.//3.9e-19:501:61//X51396
F-NT2RP3001245//CITBI-E1-2505C1.TF.1 CITBI-E1 Homo sapiens genomic clone 2505C1, genomic survey sequence.//8.5e-70:337:100//AQ242007
F-NT2RP3001253//CITBI-E1-2505N14.TR CITBI-E1 Homo sapiens genomic clone 2505N14, genomic survey sequence.//0.83:235:60//AQ260430
F-NT2RP3001260//Homo sapiens mRNA for KIAA0726 protein, complete cds.//3.8e-47:761:64//AB018269
F-NT2RP3001268//Homo sapiens zinc finger protein (HZF6) mRNA, 5' UTR and partial cds.//2.3e-64:618:72//AF027513
F-NT2RP3001272//Mus musculus mRNA for macrophage actin-associated-tyrosine-phosphorylated protein.//2.6e-99:669:83//Y18101
F-NT2RP3001274//Human ABL gene, exon 1b and intron 1b, and putative M8604 Met protein (M8604 Met) gene, complete cds.//0.99:400:58//U07561
F-NT2RP3001281//Homo sapiens chromosome 17, clone hRPK.318_A_15, complete sequence.//5.9e-39:304:70//AC005837
F-NT2RP3001297//Human mRNA for KIAA0281 gene, complete cds.//7.6e-47:544:69//D87457
F-NT2RP3001307//Ambystoma tigrinum RPE65 protein mRNA, complete cds.//2.4e-27:547:63//AF047465
F-NT2RP3001318//Plasmodium falciparum 3D7 chromosome 12 PFYAC1122 genomic sequence, WORKING DRAFT SEQUENCE, 3 unordered pieces.//0.00022:624:60//AC004709
F-NT2RP3001325//Caenorhabditis elegans cosmid F36H12.//0.25:523:59//AF078790
F-NT2RP3001338//Human mRNA for KIAA0211 gene, complete cds.//5.1e-29:345:73//D86966
F-NT2RP3001339//Rattus norvegicus mytonic dystrophy kinase-related Cdc42-binding kinase (MRCK) mRNA, complete cds.//1.2e-151:821:91//AF021935
F-NT2RP3001340//Homo sapiens HMG box factor SOX-13 mRNA, complete cds.//5.3e-27:247:81//AF083105
F-NT2RP3001355//Homo sapiens Chromosome 22q11.2 BAC Clone 77h2 In CES Region, WORKING DRAFT SEQUENCE, 7 unordered pieces.//2.1e-16:130:76//AC000052
F-NT2RP3001356
F-NT2RP3001374
F-NT2RP3001383//Homo sapiens DNA sequence from PAC 140C12 on chromosome 6q26-q27.//0.00082:365:61//AL008628
F-NT2RP3001384//Homo sapiens HRIHFB2018 mRNA, partial cds.//6.4e-157:743:98//AB015332
F-NT2RP3001392//Human DNA sequence from PAC 302D9 on chromosome 22q11.2-qter. Contains STS, complete sequence.//0.045:359:61//Z82198
F-NT2RP3001396//Drosophila melanogaster DNA sequence (P1 DS08860 (D181)), complete sequence.//1.3e-16:336:65//AC004296
F-NT2RP3001398//Mus musculus zinc finger protein (Zfp64) mRNA, complete cds.//3.1e-100:711:82//U49046
F-NT2RP3001399//Homo sapiens PAC clone DJ1106E03 from 7q31.3-7q3, complete sequence.//5.4e-20:245:73//AC005521
F-NT2RP3001407//RPCI11-41A20.TP RPCI-11 Homo sapiens genomic clone RPCI-11-41A20, genomic survey sequence.//0.051:306:59//AQ029031
F-NT2RP3001420//Human DNA sequence from PAC 12409 on chromosome 6q21. Contains DNAJ2 (HDJ1) like pseudogene, ESTs, STSs and GSSs.//0.90:170:65//AL021327
F-NT2RP3001426//Human DNA sequence *** SEQUENCING IN PROGRESS *** from clone 126A5, WORKING DRAFT SEQUENCE.//2.9e-89:138:98//AL031447
F-NT2RP3001427//CIT-HSP-2302H24.TF CIT-HSP Homo sapiens genomic clone 2302H24, genomic survey sequence.//8.1e-36:212:94//AQ020997
F-NT2RP3001428//Human nuclear pore complex-associated protein TPR (tpr) mRNA, complete cds.//8.5e-73:431:91//U69668
F-NT2RP3001432//HS_3032_B1_A03_MF CIT Approved Human Genomic Sperm Library D Homo sapiens genomic clone Plate=3032 Col=5 Row=B, genomic survey sequence.//0.00024:111:76//AQ096619
F-NT2RP3001447
F-NT2RP3001449//Human DNA sequence from clone 283E3 on chromosome 1p36.21-36.33. Contains the alternatively spliced gene for Matrix Metalloproteinase in the Female Reproductive tract MIFR1, -2, MMP21/22A, -B and -C, a novel gene, the alternatively spliced CDC2L2 gene for Cell Division Cycle 2-Like 2 (PITSLRE, p58/GTA, Galactosyltransferase Associated Protein Kinase) beta 1, beta 2-1, beta 2-2 and alpha 2-4, a 40S Ribosomal Protein S7 pseudogene, part of the KIAA0447 gene, a novel alternatively spliced gene similar to many (archae)bacterial, worm and yeast hypothetical genes, and the GNB1 gene for Guanine Nucleotide Binding Protein (G protein), Beta polypeptide 1 (Transducin Beta chain 1). Contains putative CpG islands, ESTs, STSs and GSSs, complete sequence.//2.1e-105:223:99//AL031282
F-NT2RP3001453//Ralstonia sp. E2 positive phenol-degradative gene regulator (poxR), phenol hydroxylase components (poxA, poxB, poxC, poxD, poxE, poxF), and ferredoxin-like protein (poxG) genes, complete cds.//0.75:349:59//AF026065
F-NT2RP3001457
F-NT2RP3001459
F-NT2RP3001472//Homo sapiens Sox-like transcriptional factor mRNA, complete cds.//1.3e-08:168:70//AF072836
F-NT2RP3001490
F-NT2RP3001495//Human oxidoreductase (HHCMA56) mRNA, complete cds.//1.0e-26:191:90//U13395
F-NT2RP3001497//Homo sapiens multiple membrane spanning receptor TRC8 (TRC8) mRNA, complete cds.//8.5e-171:804:98//AF064801
F-NT2RP3001527//Human lymphoid-specific SP100 homolog (LYSP100-A) mRNA, complete, cds.//8.9e-140:743:91//U36499
F-NT2RP3001529//Streptomyces griseus DNA for ribosoma protein L21, ribosomal protein L27, Obg, complete cds.//2.1e-14:517:59//D87916
F-NT2RP3001538//Capra hircus hircus clone 12 RAPD PCR sequence, genomic survey sequence.//4.7e-05:217:63//AF078176
F-NT2RP3001554//Rattus norvegicus microtubule-associated protein 1A MAP1A (Mtap-1) mRNA, complete cds.//4.3e-17:332:67//M83196
F-NT2RP3001580//RPCI11-91E19.TV RPCI11 Homo sapiens genomic clone R-91E19, genomic survey sequence.//4.2e-15:110:91//AQ281332
F-NT2RP3001587//S.pombe chromosome II cosmid c16H5.//6.6e-28:491:64//AL022104
F-NT2RP3001589//RPCI11-68M15.TK RPCI11 Homo sapiens genomic clone R-68M15, genomic survey sequence.//8.7e-108:517:98//AQ237629
F-NT2RP3001607//Homo sapiens Xp22 BAC GSHB-600G8 (Genome Systems Human BAC library) complete sequence.//1.0e-09:257:65//AC004674
F-NT2RP3001608//Methylococcus capsulatus methane monooxygenase component A alpha chain, methane monooxygenase A beta chain and methane monooxygenase component C genes, complete cds.//0.59:450:57//M90050
F-NT2RP3001621//Human DNA sequence from clone 24o18 on chromosome 6p21.31-22.2 Contains zinc finger protein pseudogene, VNO-type olfactory receptor pseudogene, nuclear envelope pore membrane protein, EST, STS, GSS, complete sequence.//1.8e-42:278:79//AL021808
F-NT2RP3001629
F-NT2RP3001634//Homo sapiens mRNA for Ariadne-2 protein.//1.5e-63:276:97//AJ130978
F-NT2RP3001642//Caenorhabditis elegans cosmid F45E6, complete sequence.//0.018:127:66//Z68117
F-NT2RP3001646
F-NT2RP3001671//Homo sapiens mRNA for NS1-binding protein (NS1-BP).//3.4e-171:816:98//AJ012449
F-NT2RP3001672//Drosophila melanogaster transcriptional repressor protein (Scm) mRNA, complete cds.//1.6e-38:542:66//U49793
F-NT2RP3001676//HS_3090_B1_B04_T7 CIT Approved Human Genomic Sperm Library D Homo sapiens genomic clone Plate=3090 Col=7 Row=D, genomic survey sequence.//3.1e-07:333:64//AQ123250
F-NT2RP3001678//Drosophila melanogaster; Chromosome 3L; Region 63C5-63D3; P1 clone DS01859, WORKING DRAFT SEQUENCE, 6 unordered pieces.//1.0:539:57//AC004358
F-NT2RP3001679//Homo sapiens genomic DNA of 8p21.3-p22 anti-oncogene of hepatocellular colorectal and non-small cell lung cancer , segment 3/11.//2.8e-130:355:96//AB020860
F-NT2RP3001688//Rattus norvegicus glucocorticoid modulatory element binding protein 2 mRNA, complete cds.//2.1e-37:512:70//AF059273
F-NT2RP3001690//CIT-HSP-2300P9.TR CIT-HSP Homo sapiens genomic clone 2300P9, genomic survey sequence.//2.8e-19:123:95//AQ012480
F-NT2RP3001698//Rat mRNA for RhoGAP, complete cds.//9-4e-11:167:74//D31962
F-NT2RP3001708//H.sapiens CpG island DNA genomic Mse1 fragment, clone 4g7, reverse read cpg4g7.rt1d.//1.3e-17:113:97//Z61312
F-NT2RP3001712//M.musculus mRNA for HP1-BP74 protein.//2.2e-95:601:88//X99642
F-NT2RP3001716
F-NT2RP3001724//Homo sapiens chromodomain-helicase-DNA-binding protein mRNA, complete cds.//1.4e-159:565:97//AF054177
F-NT2RP3001727//Rattus norvegicus implantation-associated protein (IAG2)-mRNA, partial cds.//1.7e-132:786:88//AF008554
F-NT2RP3001730//Human mRNA for KIAA0128 gene, partial cds.//3.9e-104:811:78//D50918
F-NT2RP3001739//Homo sapiens Chromosome 22q11.2 PAC Clone p201m18 In DGCR Region, complete sequence.//6.5e-07:178:69//AC000097
F-NT2RP3001752//Human DNA sequence from clone 105D16 on chromosome Xp11.3-11.4 Contains pseudogene similar to laminin-binding protein, CA repeat, STS, complete sequence.//5.2e-31:311:77//AL031311
F-NT2RP3001753//Sequence 29 from patent US 5658882.//0.11:513:58//I62381
F-NT2RP3001764//Sequence 6 from Patent WO9706245.//6.4e-47:673:66//A59888
F-NT2RP3001777//Caenorhabditis elegans cosmid T10E10.//0.078:290:63//U39644
F-NT2RP3001782//Homo sapiens mRNA for KIAA0459 protein, partial cds.//2.8e-151:710:98//AB007928
F-NT2RP3001792//Mus musculus myelin gene expression factor (MEF-2) mRNA, partial cds.//1.2e-26:213:85//U13262
F-NT2RP3001799//Human DNA sequence *** SEQUENCING IN PROGRESS *** from clone 469D22, WORKING DRAFT SEQUENCE.//8.4e-51:168:95//AL031284
F-NT2RP3001819//S.glaucescens genes strU, strX, strV and strW for 5'-hydroxystreptomycin pruduction and transport polypeptides.//0.084:526:58//X89010
F-NT2RP3001844//HS_3110_B1_E10_T7 CIT Approved Human Genomic Sperm Library D Homo sapiens genomic clone Plate=3110 Col=19 Row=J, genomic survey sequence.//1.5e-40:232:82//AQ140433
F-NT2RP3001854//Plasmodium falciparum 3D7 chromosome 12 PFYAC181 genomic sequence, WORKING DRAFT SEQUENCE, 8 unordered pieces.//0.14:452:58//AC005505
F-NT2RP3001855//Mus musculus homeobox protein PKNOX1 (Pknox1) mRNA, complete cds.//2.7e-39:575:67//AF061270
F-NT2RP3001857//M.musculus tex292 mRNA (5'region).//8.7e-07:106:81//X80434
F-NT2RP3001896
F-NT2RP3001898//Human DNA sequence *** SEQUENCING IN PROGRESS *** from clone 163G9, WORKING DRAFT SEQUENCE.//0.094:456:60//AL008733
F-NT2RP3001915//Caenorhabditis elegans cosmid C12D8, complete sequence.//0.58:482:56//Z73969
F-NT2RP3001926//Plasmodium falciparum DNA *** SEQUENCING IN PROGRESS *** from MAL4P1, WORKING DRAFT SEQUENCE.//0.42:401:58//AL034557
F-NT2RP3001929//Homo sapiens chromosome 16, cosmid clone RT102 (LANL), complete sequence.//3.1e-28:263:77//AC004651
F-NT2RP3001931
F-NT2RP3001938//CIT-HSP-2165E8.TR CIT-HSP Homo sapiens genomic clone 2165E8, genomic survey sequence.//3.6e-24:182:91//B95475
F-NT2RP3001943//Homo sapiens mRNA for KIAA0675 protein, complete cds.//1.8e-165:815:96//AB014575
F-NT2RP3001944
F-NT2RP3001969//Homo sapiens chromosome 12p13.3 clone RPCI11-350L7, WORKING DRAFT SEQUENCE, 72 unordered pieces.//4.8e-62:304:89//AC005844
F-NT2RP3001989//Plasmodium falciparum strain Dd2 heat shock protein 86 (HSP86), O1 (o1), O3 (o3), O2 (o2), CG8 (cg8), CG4 (cg4), CG3 (cg3), CG9 (cg9), CG1 (cg1), CG6 (cg6), chloroquine resistance candidate protein (cg2), and CG7 (cg7) genes, complete cds.//8.2e-10:564:60//AF030694
F-NT2RP3002002//Human DNA sequence from PAC 306D1 on chromosome X contains ESTs.//2.5e-57:361:80//Z83822
F-NT2RP3002004//Sequence 3 from patent US 5798245.//1.6e-26:104:100//AR025386
F-NT2RP3002007//Human Chromosome 15q11-q13 PAC clone pDJ223c9 from the Prader-Willi/Angelman Syndrome region, complete sequence.//0.0053:633:58//AC004137
F-NT2RP3002014//Drosophila melanogaster DNA sequence (P1s DS07528 (D169) and DS06665 (D220)), complete sequence.//1.3e-32:334:68//AC004640
F-NT2RP3002033//H.sapiens DNA sequence.//0.012:214:63//Z22493
F-NT2RP3002045//Rat mRNA for alpha-c large chain of the protein complex AP-2 associated with clathrin.//8.7e-116:713:86//X53773
F-NT2RP3002054//Mycobacterium tuberculosis H37Rv complete genome; segment 143/162.//1.6e-12:613:60//AL021841
F-NT2RP3002056//Human DNA sequence from PAC 358H7 on chromosome X.//0.17:566:59//Z77249
F-NT2RP3002057//Homo sapiens clone NH0084K19, WORKING DRAFT SEQUENCE, 30 unordered pieces.//3.3e-24:167:82//AC005682
F-NT2RP3002062
F-NT2RP3002063//Rickettsia prowazekii strain Madrid E, complete genome; segment 3/4.//0.24:508:58//AJ235272
F-NT2RP3002081//HS_2001_B1_E06_T7 CIT Approved Human Genomic Sperm Library D Homo sapiens genomic clone Plate=2001 Col=11 Row=J, genomic survey sequence.//9.7e-22:155:90//AQ218494
F-NT2RP3002097//Homo sapiens Xp22-150 BAC GSHB-309P15 (Genome Systems Human BAC Library) complete sequence.//9.6e-66:562:77//AC006210
F-NT2RP3002102//CIT-HSP-2307B10.TR CIT-HSP Homo sapiens genomic clone 2307B10, genomic survey sequence.//5.9e-16:214:74//AQ018040
F-NT2RP3002108
F-NT2RP3002142//Homo sapiens Chromosome 16 BAC clone CIT987SK-A-319E8, complete sequence.//7.6e-29:414:68//AC004020
F-NT2RP3002146//Pseudomonas fluorescens polyketide synthase type I (pltB) and polyketide synthase type I (pltC) genes, complete cds.//0.96:434:60//AF003370
F-NT2RP3002147//Human DNA sequence *** SEQUENCING IN PROGRESS *** from clone 329F2, WORKING DRAFT SEQUENCE.//1.3e-63:380:91//AL031710
F-NT2RP3002151//Human chromosome 16p13.1 BAC clone CIT987SK-551G9 complete sequence.//9.9e-60:315:80//U95742
F-NT2RP3002163
F-NT2RP3002165//M.musculus HCNGP mRNA.//1.4e-142:867:87//X68061
F-NT2RP3002166//Homo sapiens chromosome X, clone hCIT.200_L_4, complete sequence.//0.090:394:59//AC006121
F-NT2RP3002173//HS_3062_B1_G05_MF CIT Approved Human Genomic Sperm Library D Homo sapiens genomic clone Plate=3062 Col=9 Row=N, genomic survey sequence.//3.3e-101:509:96//AQ193219
F-NT2RP3002181//Human DNA sequence from clone 24o18 on chromosome 6p21.31-22.2 Contains zinc finger protein pseudogene, VNO-type olfactory receptor pseudogene, nuclear envelope pore membrane protein, EST, STS, GSS, complete sequence.//4.5e-106:432:84//AL021808
F-NT2RP3002244//Homo sapiens chromosome 19, cosmid R27377, complete sequence.//0.63:353:60//AC005321
F-NT2RP3002248//HS_3029_A1_D10_MR CIT Approved Human Genomic Sperm Library D Homo sapiens genomic clone Plate=3029 Col=19 Row=G, genomic survey sequence.//3.5e-10:125:79//AQ094880
F-NT2RP3002255//Bovine herpesvirus type 1 immedidate-early transcriptional control protein (BICP4) gene, 5' end.//5.6e-09:629:59//L14321
F-NT2RP3002273//cSRL-165E12-u cSRL flow sorted Chromosome 11 specific cosmid Homo sapiens genomic clone cSRL-165E12, genomic survey sequence.//4.9e-35:366:74//B03004
F-NT2RP3002276//B.taurus mRNA for B15 subunit of NADH: ubiquinone oxidoreductase complex.//0.023:326:60//X64898
F-NT2RP3002303//Methanobacterium thermoautotrophicum from bases 172512 to 182957 (section 16 of 148) of the complete genome.//3.8e-12:643:57//AE000810
F-NT2RP3002304//Plasmodium falciparum 3D7 chromosome 12 PFYAC1383 genomic sequence, WORKING DRAFT SEQUENCE, 3 unordered pieces.//1.6e-09:490:60//AC005504
F-NT2RP3002330//Human DNA sequence from cosmid L58b6, Huntington's Disease Region, chromosome 4p16.3, containing STS matches.//1.9e-93:572:88//Z49862
F-NT2RP3002343//HS_3010_A2_B08_T7 CIT Approved Human Genomic Sperm Library D Homo sapiens genomic clone Plate=3010 Col=16 Row=C, genomic survey sequence.//9.0e-75:373:97//AQ119068
F-NT2RP3002351//Human mRNA for NAD-dependent methylene tetrahydrofolate dehydrogenase cyclohydrolase (EC 1.5.1.15).//4.9e-64:588:75//X16396
F-NT2RP3002352//Homo sapiens mRNA for protein encoded by cxorf5 (71-7A) gene, alternatively spliced form.//1.3e-164:770:98//Y16355
F-NT2RP3002377//Homo sapiens mRNA for KIAA0788 protein, partial cds.//1.4e-190:911:98//AB018331
F-NT2RP3002399
F-NT2RP3002402//Rattus norvegicus mRNA for dipeptidyl peptidase III, complete cds.//7.2e-25:249:79//D89340
F-NT2RP3002455//Homo sapiens mRNA for KIAA0678 protein, partial cds.//1.2e-138:649:99//AB014578
F-NT2RP3002484//CIT-HSP-367N3.TP.1 CIT-HSP Homo sapiens genomic clone 367N3, genomic survey sequence.//5.0e-18:115:96//B78927
F-NT2RP3002501//Caenorhabditis elegans cosmid K01C8, complete sequence.//0.00020:170:65//Z49068
F-NT2RP3002512//Homo sapiens clone 664 unknown mRNA, partial sequence.//1.6e-59:308:97//AF091088
F-NT2RP3002529//Human vacuolar protein sorting homolog h-vps45 mRNA, complete cds.//1.4e-144:763:93//U35246
F-NT2RP3002545//Homo sapiens mRNA for KIAA0729 protein, partial cds.//1.8e-178:833:98//AB018272
F-NT2RP3002549//Homo sapiens clone DJ0098O22, WORKING DRAFT SEQUENCE, 5 unordered pieces.//4.7e-26:123:72//AC004821
F-NT2RP3002566//Streptomyces viridifaciens sigma factor (hrdD) gene, complete cds.//0.76:459:59//U60418
F-NT2RP3002587//Homo sapiens chromosome Y, clone 264,M,20, complete sequence.//4.6e-13:199:76//AC004617
F-NT2RP3002590//Porphyra purpurea chloroplast, complete genome.//0.88:284:60//U38804
F-NT2RP3002602//CIT978SK-A-441H11-2.TPB CIT978SK Homo sapiens genomic clone A-441H11, genomic survey sequence.//2.0e-22:140:95//B68331
F-NT2RP3002603
F-NT2RP3002628//C.acetobutylicum dnaJ and orfB genes.//2.0e-05:333:60//X69050
F-NT2RP3002631
F-NT2RP3002650//Mus musculus mRNA for cartilage-associated protein (CASP).//1.5e-20:641:62//AJ006469
F-NT2RP3002659//Bovine herpesvirus type 1 UL22-35 genes.//5.2e-05:621:59//Z78205
F-NT2RP3002660//Homo sapiens PAC clone DJ1006K12 from 7q31.2-q31, complete sequence.//0.98:453:57//AC004946
F-NT2RP3002663//Homo sapiens chromosome 19, cosmid F6697, complete sequence.//3.3e-22:407:67//AC006129
F-NT2RP3002671//S.pombe chromosome III cosmid c553.//1.0e-12:336:66//AL023704
F-NT2RP3002682//Caenorhabditis elegans cosmid F17C11, complete sequence.//1.3e-21:448:64//Z72507
F-NT2RP3002687//CIT978SK-A-789B1.TP CIT978SK Homo sapiens genomic clone A-789B1, genomic survey sequence.//2.5e-25:173:91//B51656
F-NT2RP3002688//Mouse mRNA for kinesin-like protein (Kif1b), complete cds.//1.2e-73:728:74//D17577
F-NT2RP3002701//CITBI-E1-2507L14.TF CITBI-E1 Homo sapiens genomic clone 2507L14, genomic survey sequence.//0.0012:55:92//AQ263530
F-NT2RP3002713
F-NT2RP3002763//Caenorhabditis elegans cosmid T20F10, complete sequence.//0.98:209:63//Z81594
F-NT2RP3002770
F-NT2RP3002785//Homo sapiens laminin beta-4 chain precursor (LAMB4) mRNA, alternatively spliced short variant, partial cds.//0.78:515:57//AF029325
F-NT2RP3002799//Human DNA sequence from clone 1052M9 on chromosome Xq25. Contains the SH2D1A gene for SH2 domain protein 1A, Duncan's disease (lymphoproliferative syndrome) (DSHP), part of a 60S Acidic Ribosomal protein 1 (RPLP1) LIKE gene and part of a mouse DOC4 LIKE gene. Contains ESTs and GSSs, complete sequence.//1.9e-21:167:79//AL022718
F-NT2RP3002810//Homo sapiens chromosome 17, clone hRPK.215_E_13, complete sequence.//0.32:187:66//AC005549
F-NT2RP3002818//Homo sapiens jerky gene product homolog mRNA, complete cds.//6.9e-54:615:70//AF004715
F-NT2RP3002861//Caenorhabditis elegans cosmid M03F4.//4.2e-05:226:65//U64601
F-NT2RP3002869//Mus musculus semaphorin VIa mRNA, complete cds.//2.0e-93:638:83//AF030430
F-NT2RP3002876//Homo sapiens mRNA for B120, complete cds.//8.5e-89:557:88//AB001895
F-NT2RP3002877//Homo sapiens chromosome 12p13.3 clone RPCI11-433J6, WORKING DRAFT SEQUENCE, 100 unordered pieces.//7.9e-12:160:78//AC006087
F-NT2RP3002909//Homo sapiens mRNA for KIAA0771 protein, partial cds.//5.7e-180:853:98//AB018314
F-NT2RP3002911//RPCI11-24N15.TPC RPCI-11 Homo sapiens genomic clone RPCI-11-24N15, genomic survey sequence.//2.3e-13:442:61//B88815
F-NT2RP3002948//, complete sequence.//2.2e-110:637:91//AC005500
F-NT2RP3002953//Homo sapiens chromosome 5, BAC clone 34j15 (LBNL H169), complete sequence.//1.7e-166:793:98//AC005754
F-NT2RP3002955//Human HepG2 partial cDNA, clone hmd3c02m5.//0.00011:61:95//D17024
F-NT2RP3002969//Rat mRNA for brain acyl-CoA synthetase II, complete cds.//1.2e-128:808:85//D30666
F-NT2RP3002972//H.sapiens (xs168) mRNA, 381bp.//1.5e-43:312:85//Z36820
F-NT2RP3002978//Plasmodium falciparum 3D7 chromosome 12 PFYAC181 genomic sequence, WORKING DRAFT SEQUENCE, 8 unordered pieces.//0.00044:527:57//AC005505
F-NT2RP3002985//Genomic sequence from Human 9q34, complete sequence.//0.92:341:60//AC001644
F-NT2RP3002988//HS_3015_A1_B07_T7 CIT Approved Human Genomic Sperm Library D Homo sapiens genomic clone Plate=3015 Col=13 Row=C, genomic survey sequence.//4.4e-05:379:58//AQ091708
F-NT2RP3003008//Mus musculus major histocompatibility locus class III regions Hsc70t gene, partial cds; smRNP, G7A, NG23, MutS homolog, CLCP, NG24, NG25, and NG26 genes, complete cds; and unknown genes.//1.4e-72:197:79//AF109905
F-NT2RP3003032//Plasmodium falciparum DNA *** SEQUENCING IN PROGRESS *** from contig 3-80, complete sequence.//1.6e-08:809:58//AL010153
F-NT2RP3003059//Rattus norvegicus potassium channel regulator 1 mRNA, complete cds.//4.1e-111:804:81//U78090
F-NT2RP3003061//Human mRNA for ankyrin (variant 2.1).//1.4e-12:633:59//X16609
F-NT2RP3003068//Human BAC clone RG264L19 from 7p15-p21, complete sequence.//0.034:282:60//AC002410
F-NT2RP3003071//H.sapiens CpG island DNA genomic Mse1 fragment, clone 13d12, reverse read cpg13d12.rt1c.//6.8e-15:95:100//Z64565
F-NT2RP3003078
F-NT2RP3003101//Mouse mRNA for tetracycline transporter-like protein, complete cds.//8.1e-72:732:71//D88315
F-NT2RP3003121
F-NT2RP3003133//Homo sapiens chromosome 19, cosmid R30385, complete sequence.//3.5e-12:168:76//AC004510
F-NT2RP3003138//Mouse kif4 mRNA for microtubule-based motor protein KIF4, complete cds.//4.0e-148:908:87//D12646
F-NT2RP3003139//Rattus norvegicus kappa opioid receptor gene, exon 4 and complete cds.//2.0e-31:658:63//U17995
F-NT2RP3003145//Mus musculus carboxypeptidase X2 mRNA, complete cds.//3.5e-22:430:63//AF017639
F-NT2RP3003150
F-NT2RP3003157//HS_3055_B1_G05_MF CIT Approved Human Genomic Sperm Library D Homo sapiens genomic clone Plate=3055 Col=9 Row=N, genomic survey sequence.//1.9e-92:493:94//AQ155489
F-NT2RP3003185//Rattus norvegicus brain-enriched guanylate kinase-associated protein 1 mRNA, complete cds.//8.6e-06:228:65//AF064868
F-NT2RP3003193//H.sapiens HZF10 mRNA for zinc finger protein.//7.4e-73:737:71//X78933
F-NT2RP3003197
F-NT2RP3003203//Rattus norvegicus golgi peripheral membrane protein p65 (GRASP65) mRNA, complete cds.//4.1e-48:640:67//AF015264
F-NT2RP3003204//Human Mermaid LINE-1 element mRNA sequence.//0.0033:69:81//U31059
F-NT2RP3003210//Homo sapiens SYBL1 gene.//1.1e-34:430:70//AJ004799
F-NT2RP3003212//Rattus norvegicus lamina associated polypeptide 1C (LAP1C) mRNA, complete cds.//6.3e-75:776:74//U20286
F-NT2RP3003230//Rattus norvegicus mRNA for coronin-like protein.//1.8e-62:575:74//AJ006064
F-NT2RP3003242//Homo sapiens stanniocalcin-2 (STC-2) mRNA, complete cds.//3.7e-128:617:98//AF055460
F-NT2RP3003251//H.sapiens Staf50 mRNA.//3.5e-67:651:76//X82200
F-NT2RP3003264//Plasmodium falciparum 3D7 chromosome 12 PFYAC812 genomic sequence, WORKING DRAFT SEQUENCE, 8 unordered pieces.//0.015:473:58//AC004153
F-NT2RP3003278//H.sapiens CpG island DNA genomic Mse1 fragment, clone 28b4, forward read cpg28b4.ft1a.//4.0e-27:174:93//Z60555
F-NT2RP3003282//Homo sapiens dynamin (DNM) mRNA, complete cds.//1.3e-131:694:93//L36983
F-NT2RP3003290//Homo sapiens nickel-specific induction protein (Cap43) mRNA, complete cds.//1.7e-64:662:71//AF004162
F-NT2RP3003301//Spinacia oleracea mRNA for ATP-dependent protease Lon, complete cds.//4.9e-37:682:64//D85610
F-NT2RP3003302//Homo sapiens, clone hRPK.15_A_1, complete sequence.//4.6e-95:680:82//AC006213
F-NT2RP3003311//Homo sapiens chromosome 21, Neurofibromatosis 1 (NF1) related locus, complete sequence.//1.0:191:62//AC004527
F-NT2RP3003313//Streptomyces coelicolor cosmid 5A7.//0.0084:403:61//AL031107
F-NT2RP3003327//H.sapiens Staf50 mRNA.//2.5e-29:253:67//X82200
F-NT2RP3003330
F-NT2RP3003344
F-NT2RP3003346//Homo sapiens chromosome 17, clone hRPK.795_F_17, complete sequence.//9.0e-41:296:84//AC005284
F-NT2RP3003353//Human DNA sequence from PAC 970D1 on chromosome 1q24. Contains ESTs, STSs and a BAC end-sequence (GSS).//0.047:404:60//AL021069
F-NT2RP3003377//Homo sapiens clone DJ0919J22, WORKING DRAFT SEQUENCE, 34 unordered pieces.//8.3e-122:632:96//AC005519
F-NT2RP3003384//Homo sapiens Chromosome 2 BAC Clone 376a1, WORKING DRAFT SEQUENCE, 17 unordered pieces.//0.0036:127:74//AC000360
F-NT2RP3003385//Mus musculus SKD3 mRNA, complete cds.//2.0e-110:843:79//U09874
F-NT2RP3003403//Human Chromosome X, complete sequence.//7.5e-21:647:61//AC002407
F-NT2RP3003409//Human DHHC-domain-containing cysteine-rich protein mRNA, complete cds.//1.0e-20:430:63//U90653
F-NT2RP3003411//Mus musculus COP9 complex subunit 7b (COPS7b) mRNA, complete cds.//4.2e-139:524:90//AF071317
F-NT2RP3003427//HS-1051-A1-D03-MF.abi CIT Human Genomic Sperm Library C Homo sapiens genomic clone Plate=CT 773 Col=5 Row=G, genomic survey sequence.//8.8e-18:111:97//B40173
F-NT2RP3003433//HS_2219_B2_A11_MF CIT Approved Human Genomic Sperm Library D Homo sapiens genomic clone Plate=2219 Col=22 Row=B, genomic survey sequence.//1.2e-57:410:83//AQ145866
F-NT2RP3003464//Homo sapiens rab3-GAP regulatory domain mRNA, complete cds.//5.2e-181:853:98//AF004828
F-NT2RP3003490//Homo sapiens mRNA for KIAA0725 protein, partial cds.//1.6e-173:826:98//AB018268
F-NT2RP3003491//CIT-HSP-2344O1.TR CIT-HSP Homo sapiens genomic clone 2344O1, genomic survey sequence.//1.2e-39:213:97//AQ057124
F-NT2RP3003500//HS_3000_B1_C07_MR CIT Approved Human Genomic Sperm Library D Homo sapiens genomic clone Plate=3000 Col=13 Row=F, genomic survey sequence.//0.025:253:60//AQ090347
F-NT2RP3003543//Homo sapiens chromosome 16, cosmid clone 399H11 (LANL), complete sequence.//0.95:279:60//AC004234
F-NT2RP3003552//Homo sapiens clone UWGC:y54c222 from 6p21, complete sequence.//1.8e-88:166:84//AC006049
F-NT2RP3003555//Human DNA sequence *** SEQUENCING IN PROGRESS *** from clone 228H13, WORKING DRAFT SEQUENCE.//8.9e-17:245:72//AL031985
F-NT2RP3003564//HS_3141_B1_G10_T7 CIT Approved Human Genomic Sperm Library D Homo sapiens genomic clone Plate=3141 Col=19 Row=N, genomic survey sequence.//2.7e-79:442:93//AQ187798
F-NT2RP3003572
F-NT2RP3003576//Homo sapiens clone RG031N19, WORKING DRAFT SEQUENCE, 1 unordered pieces.//5.8e-55:275:84//AC005632
F-NT2RP3003589//Canine rab10 mRNA for ras-related GTP-binding protein.//1.1e-94:488:95//X56387
F-NT2RP3003621//Homo sapiens chromosome 16, cosmid clone 432A1 (LANL), complete sequence.//6.0e-88:463:84//AC004235
F-NT2RP3003625//Human DNA sequence *** SEQUENCING IN PROGRESS *** from clone 390E6, WORKING DRAFT SEQUENCE.//0.98:307:60//AL031600
F-NT2RP3003656
F-NT2RP3003659//F.rubripes GSS sequence, clone 013G07cE7, genomic survey sequence.//1.7e-25:284:74//AL011271
F-NT2RP3003665//Homo sapiens chromosome 9q34, clone 63G10, complete sequence.//0.011:279:65//AC002096
F-NT2RP3003672
F-NT2RP3003680//Drosophila melanogaster; Chromosome 2R; Region 39B1-39B3; P1 clone DS05527, WORKING DRAFT SEQUENCE, 9 unordered pieces.//3.4e-16:425:64//AC005811
F-NT2RP3003686//HS_3064_B2_A04_MF CIT Approved Human Genomic Sperm Library D Homo sapiens genomic clone Plate=3064 Col=8 Row=B, genomic survey sequence.//3.1e-27:153:98//AQ136993
F-NT2RP3003701
F-NT2RP3003716//Rattus norvegicus Shal-related potassium channel Kv4.3 mRNA, complete cds.//4.6e-107:788:82//U42975
F-NT2RP3003726//Homo sapiens mRNA for KIAA0757 protein, complete cds.//2.3e-148:700:98//AB018300
F-NT2RP3003746//CIT-HSP-2306A10.TF CIT-HSP Homo sapiens genomic clone 2306A10, genomic survey sequence.//0.39:212:61//AQ015785
F-NT2RP3003795//Human DNA sequence from clone 333H23 on chromosome 22q12.1-12.3. Contains the (possibly alternatively spliced) RPL3 gene for 60S Ribosomal Protein L3 and the threefold alternatively spliced gene for Synaptogyrin 1A, 1B and 1C (SYNGR1A, SYBGRIB, SYNGR1C), both genes downstream of a putative CpG island. Contains ESTs, an STS, GSSs, genomic marker D22S1155 and a ca repeat polymorphism, complete sequence.//4.2e-21:445:66//AL022326
F-NT2RP3003799//Homo sapiens DNA from chromosome 19-cosmids R31158, R31874, and R28125, genomic sequence, complete sequence.//1.0:257:63//AF038458
F-NT2RP3003800//Mouse neuronal proto-oncogene c-src mRNA encoding tyrosine-specific protein kinase, complete cds.//1.2e-63:484:81//M17031
F-NT2RP3003805//Homo sapiens chromosome 19, cosmid R27377, complete sequence.//0.96:353:60//AC005321
F-NT2RP3003809//Bovine herpesvirus 1 complete genome.//7.2e-12:615:60//AJ004801
F-NT2RP3003819
F-NT2RP3003825
F-NT2RP3003828//Human rRNA primary transcript internal transcribed spacer 2 (ITS2).//6.2e-16:543:62//X17626
F-NT2RP3003831//RPCI11-50N15.TJ RPCI11 Homo sapiens genomic clone R-50N15, genomic survey sequence.//1.1e-21:174:85//AQ082633
F-NT2RP3003833//Homo sapiens clones 24718 and 24825 mRNA sequence.//8.0e-47:242:98//AF070611
F-NT2RP3003842//RPCI11-44E5.TJ RPCI11 Homo sapiens genomic clone R-44E5, genomic survey sequence.//9.7e-25:143:97//AQ195884
F-NT2RP3003846//Homo sapiens mRNA for KIAA0725 protein, partial cds.//4.2e-36:335:68//AB018268
F-NT2RP3003870//Homo sapiens mRNA for KIAA0800 protein, complete cds.//4.1e-174:805:99//AB018343
F-NT2RP3003876//Rattus norvegicus Rabin3 mRNA, complete cds.//2.7e-109:709:84//U19181
F-NT2RP3003914//Drosophila melanogaster UDP-glucose:glycoprotein glucosyltransferase mRNA, complete cds.//8.9e-11:193:70//U20554
F-NT2RP3003918//Homo sapiens VAMP-associated protein of 33 kDa (VAP-33) mRNA, complete cds.//2.6e-47:404:77//AF057358
F-NT2RP3003932//Plasmodium falciparum 3D7 chromosome 12 PFYAC1383 genomic sequence, WORKING DRAFT SEQUENCE, 3 unordered pieces.//0.68:597:55//AC005504
F-NT2RP3003989//Human DNA sequence *** SEQUENCING IN PROGRESS *** from clone 404H4, WORKING DRAFT SEQUENCE.//0.37:548:56//AL031661
F-NT2RP3003992//Human cGMP-gated cation channel beta subunit (CNCG2) mRNA, complete cds.//0.021:433:58//U58837
F-NT2RP3004013//M.musculus Spnr mRNA for RNA binding protein.//1.4e-164:838:94//X84692
F-NT2RP3004016//Human DNA sequence *** SEQUENCING IN PROGRESS *** from clone 1018K9, WORKING DRAFT SEQUENCE.//0.00042:356:62//AL031726
F-NT2RP3004041//Human DNA sequence *** SEQUENCING IN PROGRESS *** from clone 809F4, WORKING DRAFT SEQUENCE.//6.8e-112:627:82//AL022400
F-NT2RP3004051//Human mRNA for KIAA0319 gene, complete cds.//2.2e-61:774:67//AB002317
F-NT2RP3004070//Homo sapiens DNA sequence from PAC 352A20 on chromosome 6q24.1-25.1. Contains a pseudogene similar to yeast, bacterial, worm and slime mold hypothetical genes, and a gene coding for an aldehyde dehydrogenase family protein. Contains ESTs, STSs and GSSs, complete sequence.//7.9e-17:484:62//AL021939
F-NT2RP3004078//M.musculus (BALB/c) MRFX2 mRNA.//1.9e-102:684:83//X76089
F-NT2RP3004093//F24P17-Sp6 IGF Arabidopsis thaliana genomic clone F24P17, genomic survey sequence.//0.021:207:63//B09433
F-NT2RP3004095//Homo sapiens clone NH0486I22, WORKING DRAFT SEQUENCE, 5 unordered pieces.//3.5e-25:272:77//AC005038
F-NT2RP3004110//Homo sapiens DNA from chromosome 19, BAC 33152, complete sequence.//8.6e-28:223:73//AC003973
F-NT2RP3004125//Homo sapiens TTF-I interacting peptide 20 mRNA, partial cds.//2.2e-28:637:63//AF000560
F-NT2RP3004145
F-NT2RP3004148
F-NT2RP3004155//Homo sapiens timing protein CLK-1 mRNA, complete cds.//6.5e-120:578:98//AF032900
F-NT2RP3004189//M.musculus tex292 mRNA (5'region).//1.1e-06:102:82//X80434
F-NT2RP3004206//D.melanogaster crn mRNA.//7.3e-69:715:71//X58374
F-NT2RP3004207//Mouse mRNA for seizure-related gene product 6 type 2 precursor, complete cds.//4.8e-42:650:66//D64009
F-NT2RP3004209//Human cosmid Q7A10 (D21S246) insert DNA, complete sequence.//8.4e-55:184:84//D42052
F-NT2RP3004215//Homo sapiens chromosome 5, Pac clone 9c13 (LBNL H127), complete sequence.//0.22:458:60//AC006084
F-NT2RP3004242//Caenorhabditis elegans cosmid ZK632, complete sequence.//1.6e-29:409:69//Z22181
F-NT2RP3004246//Homo sapiens chromosome 10 clone CIT987SK-1010K1 map 10q25, complete sequence.//3.6e-117:242:100//AC005385
F-NT2RP3004253//H.sapiens 28S rRNA V8 region (LAN5-6).//2.6e-12:589:59//X69353
F-NT2RP3004258//Rattus norvegicus Zis mRNA, complete cds.//1.2e-88:489:91//AF013967
F-NT2RP3004262//Homo sapiens heat shock protein hsp40-3 mRNA, complete cds.//3.1e-153:733:98//AF088982
F-NT2RP3004282//Homo sapiens torsinA (DYT1) mRNA, complete cds.//1.3e-24:597:61//AF007871
F-NT2RP3004332
F-NT2RP3004334//L.esculentum gene for fruit ripening polygalacturonase.//0.23:501:57//X80908
F-NT2RP3004341//Human DNA sequence from clone 503G16 on chromosome 6p23 Contains EST, CpG island, complete sequence.//0.0014:198:66//Z93020
F-NT2RP3004348//R.norvegicus mRNA for cytosolic resiniferatoxin-binding protein.//1.4e-103:600:82//X67877
F-NT2RP3004349//Homo sapiens Xp22 BAC GS-321G17 (Genome Systems Human BAC library) complete sequence.//5.1e-49:480:75//AC004025
F-NT2RP3004378//Drosophila melanogaster; Chromosome 2R; Region 47F1-47F7; P1 clone DS02304, WORKING DRAFT SEQUENCE, 5 unordered pieces.//1.8e-23 :352:67//AC005653
F-NT2RP3004399//H.sapiens mRNA for leucine-rich primary response protein 1.//7.2e-140:804:90//X97249
F-NT2RP3004424//Mus musculus mRNA for nuclear protein SA3.//6.8e-53:413:81//AJ005678
F-NT2RP3004428//Salmo salar DNA for a cryptic repeat.//3.2e-07:270:63//AJ012206
F-NT2RP3004451//RPCI11-51J15.TK RPCI11 Homo sapiens genomic clone R-51J15, genomic survey sequence.//8.8e-19:180:82//AQ052326
F-NT2RP3004454//Homo sapiens mRNA for KIAA0448 protein, complete cds.//6.2e-123:583:99//AB007917
F-NT2RP3004466//HS_3038_B2_F08_MF CIT Approved Human Genomic Sperm Library D Homo sapiens genomic clone Plate=3038 Col=16 Row=L, genomic survey sequence.//0.41:172:59//AQ102458
F-NT2RP3004470//H.sapiens CpG island DNA genomic Mse1 fragment, clone 81a11, reverse read cpg81a11.rt1a.//7.0e-25:148:96//Z56029
F-NT2RP3004472//RPCI11-42M5.TJ RPCI11 Homo sapiens genomic clone R-42M5, genomic survey sequence.//1.6e-20:143:92//AQ052792
F-NT2RP3004475//Homo sapiens mRNA for KIAA0456 protein, partial cds.//3.0e-150:715:98//AB007925
F-NT2RP3004480//Mus musculus maternal-embryonic 3 (Mem3) mRNA, complete cds.//1.0e-119:679:90//U47024
F-NT2RP3004490//Homo sapiens mRNA for Musashi, complete cds.//7.1e-155:752:97//AB012851
F-NT2RP3004498//Homo sapiens clone DJ1147A01, WORKING DRAFT SEQUENCE, 25 unordered pieces.//4.0e-67:265:84//AC006023
F-NT2RP3004503//Homo sapiens Xp22-132-134 BAC GSHB-590J15 (Genome Systems Human BAC library) complete sequence.//1.2e-55:415:78//AC004673
F-NT2RP3004504//M.musculus mRNA for CPEB protein.//2.0e-110:618:91//Y08260
F-NT2RP3004507//Homo sapiens chromosome 19, cosmid R26660, complete sequence.//9.3e-46:433:76//AC005328
F-NT2RP3004527//Homo sapiens mRNA; transcriptional unit N144, 5' end.//1.1e-100:508:97//AJ002574
F-NT2RP3004534//Mouse oncogene (ect2) mRNA, complete cds.//2.0e-93:442:84//L11316
F-NT2RP3004539//Homo sapiens mRNA for KIAA0632 protein, partial cds.//8.5e-145:679:98//AB014532
F-NT2RP3004544//Homo sapiens mRNA for KIAA0554 protein, partial cds.//2.8e-169:793:98//AB011126
F-NT2RP3004566//Mus musculus krupple-related zinc finger protein (Emzf1) mRNA, complete cds.//6.9e-18:433:64//AF031955
F-NT2RP3004569//CITBI-E1-2522H6.TF CITBI-E1 Homo sapiens genomic clone 2522H6, genomic survey sequence.//5.3e-15:138:84//AQ280780
F-NT2RP3004572//Homo sapiens cofactor of initiator function (CIF150) mRNA, complete cds.//1.0e-179:860:97//AF026445
F-NT2RP3004578//Homo sapiens mRNA for KIAA0477 protein, complete cds.//4.2e-150:711:98//AB007946
F-NT2RP3004594//Homo sapiens mRNA for AND-1 protein.//1.1e-158:796:95//AJ006266
F-NT2RP3004617//Homo sapiens clone DJ1152C17, WORKING DRAFT SEQUENCE, 1 unordered pieces.//9.3e-14:360:65//AC004977
F-NT2RP3004618//Oryctolagus cuniculus translation initiation factor eIF2C mRNA, complete cds.//2.9e-52:539:73//AF005355
F-NT2RP3004669//Brn-3a=class V POU transcription factor [mice, CD/CD, embryo fibroblast cells, Genomic, 2160 nt].//0.046:437:57//S69350
F-NT2RP3004670//Human DNA sequence *** SEQUENCING IN PROGRESS *** from clone 356B8, WORKING DRAFT SEQUENCE.//1.9e-05:625:59//Z98882
F-NT2RP4000008//Homo sapiens chromosome X, clone hCIT.200_L_4, complete sequence.//1.5e-155:844:92//AC006121
F-NT2RP4000023//Arabidopsis thaliana genomic DNA, chromosome 5, TAC clone: K24G6, complete sequence.//0.012:417:59//AB012242
F-NT2RP4000035//Homo sapiens BAC clone NH0353P23 from 2, complete sequence.//8.0e-18:242:74//AC005035
F-NT2RP4000049//Homo sapiens decoy receptor 2 mRNA, complete cds.//2.1e-81:556:85//AF029761
F-NT2RP4000051//Mus musculus mRNA for cartilage-associated protein (CASP).//1.6e-19:654:63//AJ006469
F-NT2RP4000078//Homo sapiens mRNA for NS1-binding protein (NS1-BP).//2.5e-149:720:97//AJ012449
F-NT2RP4000102//Plasmodium falciparum MAL3P2, complete sequence.//0.28:336:57//AL034558
F-NT2RP4000109//Homo sapiens mRNA for MEGF5, partial cds.//4.4e-166:774:99//AB011538
F-NT2RP4000111//B.taurus mRNA for cleavage and polyadenylation specificity factor.//2.6e-137:678:91//X75931
F-NT2RP4000129//Homo sapiens mRNA for KIAA0483 protein, partial cds.//3.3e-114:548:98//AB007952
F-NT2RP4000147//Rattus norvegicus ADP-ribosylation factor-directed GTPase activating protein mRNA, complete cds.//1.2e-104:677:85//U35776
F-NT2RP4000150//Rat proto-oncogene (Ets-1) mRNA, complete cds.//7.2e-54:327:74//L20681
F-NT2RP4000151//Homo sapiens clone 664 unknown mRNA, partial sequence.//2.2e-62:360:92//AF091088
F-NT2RP4000159//RPCI11-75N16.TJ RPCI11 Homo sapiens genomic clone R-75N16, genomic survey sequence.//2.6e-19:119:98//AQ267551
F-NT2RP4000167//Homo sapiens Xp22-150 BAC GSHB-309P15 (Genome Systems Human BAC Library) complete sequence.//3.3e-49:683:67//AC006210
F-NT2RP4000185//Homo sapiens clone DT1P1E11 mRNA, CAG repeat region.//1.1e-99:543:93//U92989
F-NT2RP4000210//Homo sapiens mRNA for KIAA0700 protein, partial cds.//4.9e-174:825:98//AB014600
F-NT2RP4000212//, complete sequence.//4.0e-131:233:94//AC005300
F-NT2RP4000214//Homo sapiens chromosome 19, CIT-HSP-444n24, complete sequence.//1.8e-161:751:99//AC005261
F-NT2RP4000218//RPCI11-69B7.TJ RPCI11 Homo sapiens genomic clone R-69B7, genomic survey sequence.//1.7e-84:413:98//AQ268504
F-NT2RP4000243//Homo sapiens mRNA for cartilage-associated protein (CASP).//2.6e-156:771:97//AJ006470
F-NT2RP4000246//Mus musculus neural variant mena+++ protein (Mena) mRNA, complete cds.//2.1e-120:707:87//U72523
F-NT2RP4000259//Homo sapiens clone 683 unknown mRNA, complete sequence.//2.8e-128:604:99//AF091092
F-NT2RP4000263//CIT-HSP-2336N24.TF CIT-HSP Homo sapiens genomic clone 2336N24, genomic survey sequence.//0.27:124:69//AQ043515
F-NT2RP4000290//S.cerevisiae chromosome XIV reading frame ORF YNL132w.//8.6e-32:619:63//Z71408
F-NT2RP4000312//Human mRNA for KIAA0147 gene, partial cds.//4.7e-41:685:63//D63481
F-NT2RP4000321//Mus musculus transcription factor HOXA13 (Hoxa13) gene, complete cds.//6.9e-05:756:59//U59322
F-NT2RP4000323
F-NT2RP4000355
F-NT2RP4000360//Homo sapiens mRNA for KIAA0738 protein, complete cds.//2.0e-140:654:99//AB018281
F-NT2RP4000367//Homo sapiens IkappaB kinase complex associated protein (IKAP) mRNA, complete cds.//2.6e-135:649:97//AF044195
F-NT2RP4000370//Rickettsia prowazekii strain Madrid E, complete genome; segment 3/4.//2.0e-23:524:62//AJ235272
F-NT2RP4000376//Sequence 1 from patent US 5580968.//1.6e-115:716:87//I30536
F-NT2RP4000381//Mus musculus mRNA for hepatoma-derived growth factor, complete cds, strain:BALB/c.//4.3e-05:450:58//D63850
F-NT2RP4000398//Homo sapiens chromosome 19, BAC CIT-B-393i15 (BC301323), complete sequence.//9.2e-37:336:69//AC006116
F-NT2RP4000415//Caenorhabditis elegans cosmid C42D8.//0.30:222:60//U56966
F-NT2RP4000417//Drosophila melanogaster cosmid clone 86E4.//1.8e-48:580:69//AL021086
F-NT2RP4000424//Homo sapiens chromosome 17, clone HRPC41C23, complete sequence.//1.6e-42:265:81//AC003101
F-NT2RP4000448//CIT-HSP-2370F8.TF CIT-HSP Homo sapiens genomic clone 2370F8, genomic survey sequence.//2.0e-56:287:98//AQ110194
F-NT2RP4000449//CIT-HSP-2366N18.TR CIT-HSP Homo sapiens genomic clone 2366N18, genomic survey sequence.//2.4e-42:236:95//AQ076183
F-NT2RP4000455//Homo sapiens PAC clone 166H1 from 12q, complete sequence.//0.17:158:67//AC003982
F-NT2RP4000457//H.sapiens mRNA for herpesvirus associated ubiquitin-specific protease (HAUSP).//0.00034:532:57//Z72499
F-NT2RP4000480//Rhodothermus marinus R-21 DNA ligase gene, complete cds.//0.0094:616:58//U10483
F-NT2RP4000481
F-NT2RP4000498//S.cerevisiae chromosome IX cosmid 9150.//5.7e-24:633:60//Z38125
F-NT2RP4000500//G.gallus mRNA for LRP/alpha-2-macroglobulin receptor.//2.4e-62:667:73//X74904
F-NT2RP4000515
F-NT2RP4000517//Homo sapiens chromosome 18, clone hRPK.474_N_24, complete sequence.//1.6e-179:851:98//AC006238
F-NT2RP4000518//Homo sapiens mRNA for ATP-dependent RNA helicase, partial.//6.7e-33:203:93//AJ010840
F-NT2RP4000519//Mus musculus tyrosine kinase growth factor receptor (Etk2/tyro3) gene, alternative 5' coding exon 2C.//0.26:162:61//U23720
F-NT2RP4000524//Rattus norvegicus rsec8 mRNA, partial cds.//1.2e-139:809:89//U32498
F-NT2RP4000528//Caenorhabditis elegans cosmid F59E12.//1.0e-06:404:59//AF003386
F-NT2RP4000541//Drosophila melanogaster DNA sequence (P1 DS02109 (D53)), complete sequence.//1.3e-05:498:58//AC002443
F-NT2RP4000556//Sequence 1 from Patent EP 0285405.//1.2e-18:586:61//I05465
F-NT2RP4000560//Murine genomic DNA; partially digested Sau3A fragment, cloned into cosmid vector pEMBLcos2, complete sequence.//2.5e-53:183:82//AF059580
F-NT2RP4000588//Human DNA sequence *** SEQUENCING IN PROGRESS *** from clone 414D7, WORKING DRAFT SEQUENCE.//0.00062:253:65//AL033543
F-NT2RP4000614//Homo sapiens TLS-associated protein TASR-2 mRNA, complete cds.//3.2e-138:666:98//AF067730
F-NT2RP4000638//HS_3042_B2_D05_MF CIT Approved Human Genomic Sperm Library D Homo sapiens genomic clone Plate=3042 Col=10 Row=H, genomic survey sequence.//3.0e-06:78:89//AQ099333
F-NT2RP4000648//Homo sapiens KNSL4 and MAZ genes for kinesin-like DNA binding protein and Myc-associated zinc finger protein, complete cds.//1.9e-11:104:85//AB017335
F-NT2RP4000657//Mus musculus bone morphogenetic factor 11 (Bmp11) gene, exon 1.//0.34:350:62//AF100904
F-NT2RP4000704//Homo sapiens mRNA expressed in 19week fetal lung, clone IMAGE:300856.//3.3e-167:785:99//AB004852
F-NT2RP4000713//Gallus gallus atonal homolog 1 (Cath1) gene, complete cds.//3.7e-07:261:65//U61149
F-NT2RP4000724//Human endogenous retrovirus env mRNA.//9.2e-136:474:89//X82272
F-NT2RP4000728//Homo sapiens mRNA for KIAA0606 protein, partial cds.//3.1e-41:350:71//AB011178
F-NT2RP4000737//Myxococcus xanthus ATP-dependent protease (bsgA) gene, complete cds.//1.0:504:58//L19301
F-NT2RP4000739//CIT-HSP-2010O22.TR CIT-HSP Homo sapiens genomic clone 2010O22, genomic survey sequence.//1.1e-24:161:93//B57903
F-NT2RP4000781//Homo sapiens clone DJ0892G19, complete sequence.//0.052:493:58//AC004917
F-NT2RP4000787//Cricetulus griseus SRD-2 mutant sterol regulatory element binding protein-2 (SREBP-2) mRNA, complete cds.//9.6e-18:259:68//U22818
F-NT2RP4000817//Homo sapiens mRNA for KIAA0470 protein, complete cds.//1.5e-174:816:98//AB007939
F-NT2RP4000833//Homo sapiens PAC clone DJ0905J08 from 7p12-p14, complete sequence.//0.97:52:92//AC005189
F-NT2RP4000837//Human DNA sequence *** SEQUENCING IN PROGRESS *** from clone 1112F19, WORKING DRAFT SEQUENCE.//2.1e-128:644:97//AL034420
F-NT2RP4000839//RPCI11-6D8.TP RPCI-11 Homo sapiens genomic clone RPCI-11-6D8, genomic survey sequence.//1.5e-44:281:91//B48216
F-NT2RP4000855//Rattus norvegicus mRNA for aminopeptidase-B, complete cds.//9.5e-43:722:64//D87515
F-NT2RP4000865//Human zinc finger protein ZNF136.//6.8e-95:415:78//U09367
F-NT2RP4000878//Mus musculus mRNA for myeloid associated differentiation protein.//7.0e-87:646:80//AJ001616
F-NT2RP4000879//N.tabaccum mRNA for ubiquitin activating enzyme E1.//9.0e-17:806:58//Y10804
F-NT2RP4000907//Mouse NLRR-1 mRNA for leucine-rich-repeat protein, complete cds.//6.8e-153:934:86//D45913
F-NT2RP4000915//Homo sapiens mRNA for ZNF198 protein.//9.4e-79:584:78//AJ224901
F-NT2RP4000918//Drosophila melanogaster DNA sequence (P1 DS04106 (D172)), complete sequence.//2.0e-08:609:58//AC004290
F-NT2RP4000925//Rattus norvegicus Shal-related potassium channel Kv4.3 mRNA, complete cds.//3.5e-64:415:87//U42975
F-NT2RP4000927//H.sapiens genomic DNA (chromosome 3; clone NRL062R).//0.75:175:62//X87547
F-NT2RP4000928//Homo sapiens CDP-diacylglycerol synthase 2 (CDS2) mRNA, partial cds.//3.5e-163:781:97//AF069532
F-NT2RP4000929//Plasmodium falciparum 3D7 chromosome 12 PFYAC69 genomic sequence WORKING DRAFT SEQUENCE, 4 unordered pieces.//0.94:763:56//AC004688
F-NT2RP4000955//Homo sapiens clone DJ0919J22, WORKING DRAFT SEQUENCE, 34 unordered pieces.//1.0e-128:673:96//AC005519
F-NT2RP4000973//Caenorhabditis elegans cosmid Y47H9C, complete sequence.//1.6e-15:255:69//AL032657
F-NT2RP4000975//CIT-HSP-2307I6.TF CIT-HSP Homo sapiens genomic clone 2307I6, genomic survey sequence.//6.5e-31:317:79//AQ015742
F-NT2RP4000979//Human bullous pemphigoid antigen mRNA, 3' end.//0.88:54:90//M22942
F-NT2RP4000984//Rhodobacter sphaeroides mRNA.//0.76:214:64//M83823
F-NT2RP4000989//F.rubripes GSS sequence, clone 011A11aE12, genomic survey sequence.//1.0:149:65//AL010911
F-NT2RP4000996//Penaeus setiferus microsatellite Pse017 repeat region.//3.3e-08:139:74//AF047358
F-NT2RP4000997//Rattus norvegicus RNA polymerase I 127 kDa subunit mRNA, complete cds.//3.6e-126:824:84//AF025424
F-NT2RP4001004
F-NT2RP4001006//Mus musculus ROSA 26 transcription AS ROSA26AS mRNA, complete cds.//1.4e-110:861:78//U83176
F-NT2RP4001010//Rattus norvegicus PSD-95/SAP90-associated protein-4 mRNA, complete cds.//2.0e-135:789:89//U67140
F-NT2RP4001029//Mus domesticus nuclear binding factor NF2d9 mRNA, complete cds.//3.7e-120:718:88//U20086
F-NT2RP4001041//Schizosaccharomyces pombe mRNA, partial cds, clone: SY 0717.//4.1e-22:452:64//D89170
F-NT2RP4001057
F-NT2RP4001064//Mus musculus mRNA for cartilage-associated protein (CASP).//1.2e-20:639:62//AJ006469
F-NT2RP4001078//Streptomyces coelicolor cosmid 1C2.//0.0025:474:59//AL031124
F-NT2RP4001079//Rat alternatively spliced mRNA.//1.4e-141:832:88//M93018
F-NT2RP4001080//H.sapiens PTB-4 gene for polypirimidine tract binding protein.//9.0e-64:628:70//X65372
F-NT2RP4001086//Homo sapiens mRNA for KIAA0592 protein, partial cds.//4.7e-84:604:86//AB011164
F-NT2RP4001095
F-NT2RP4001100//CITBI-E1-2503J7.TR CITBI-E1 Homo sapiens genomic clone 2503J7, genomic survey sequence.//9.4e-17:185:79//AQ263402
F-NT2RP4001117//Canis familiaris sec61 homologue mRNA, complete cds.//1.0e-143:760:87//M96629
F-NT2RP4001122
F-NT2RP4001126//Homo sapiens shox gene, alternatively spliced products, complete cds.//4.2e-17:636:61//U82668
F-NT2RP4001138//Homo sapiens PAC clone DJ1121E10 from 7q21.1-q2, complete sequence.//2.5e-23:408:60//AC004969
F-NT2RP4001143//Sequence 5 from patent US 5753432.//1.8e-39:276:86//AR008079
F-NT2RP4001148//Homo sapiens clone RG332P12, WORKING DRAFT SEQUENCE, 1 unordered pieces.//2.7e-116:684:89//AC005095
F-NT2RP4001149//Mouse mRNA for thymic epithelial cell surface antigen, complete cds.//3.0e-48:581:66//D67067
F-NT2RP4001150//Homo sapiens alone DJ1032D07, WORKING DRAFT SEQUENCE, 3 unordered pieces.//9.4e-25:193:67//AC004952
F-NT2RP4001159//Human FMR1 gene, 5' end.//0.28:130:66//L19476
F-NT2RP4001174//FMR1 {CGG repeats} [human, Fragile X syndrome patient, Genomic, 429 nt].//0.0014:187:67//S74494
F-NT2RP4001206//Dictyostelium discoideum random slug cDNA19 protein (rscl9) mRNA, partial cds.//0.032:453:58//U82511
F-NT2RP4001207//HS_2248_A1_C03_MF CIT Approved Human Genomic Sperm Library D Homo sapiens genomic clone Plate=2248 Col=5 Row=E, genomic survey sequence.//0.00018:58:94//AQ192358
F-NT2RP4001210//Homo sapiens chromosome 10 clone CIT987SK-1019O18 map 10p11.2-10p12.1, complete sequence.//0.93:515:58//AC005877
F-NT2RP4001213//Human KRAB zinc finger protein (ZNF177) mRNA, splicing variant, complete cds.//3.6e-44:187:74//U37251
F-NT2RP4001219//Caenorhabditis elegans cosmid Y47H9C, complete sequence.//1.3e-15:288:67//AL032657
F-NT2RP4001228//Homo sapiens actin binding protein MAYVEN mRNA, complete cds.//2.2e-26:855:60//AF059569
F-NT2RP4001235//RPCI11-18E11.TVB RPCI-11 Homo sapiens genomic clone RPCI-11-18E11, genomic survey sequence.//2.7e-15:101:98//B88081
F-NT2RP4001256//Amycolatopsis mediterranei 3-amino-5-hydroxy benzoic acid synthase (rifD) gene, complete cds.//1.0:459:59//U33061
F-NT2RP4001260//Sequence 2 from Patent WO9601901.//0.0018:246:63//A48324
F-NT2RP4001274//Homo sapiens, complete sequence.//2.5e-05:201:67//AC005854
F-NT2RP4001276//CIT-HSP-2324B15.TF CIT-HSP Homo sapiens genomic clone 2324B15, genomic survey sequence.//3.5e-18:138:92//AQ040728
F-NT2RP4001313//Homo sapiens mitochondrial outer membrane protein (TOM40) mRNA, nuclear gene encoding mitochondrial protein, complete cds.//7.4e-30:535:65//AF043250
F-NT2RP4001315//Bos taurus mRNA for Rab5 GDP/GTP exchange factor, Rabex5.//3.5e-145:795:91//AJ001119
F-NT2RP4001336//CIT-HSP-2169F21.TR CIT-HSP Homo sapiens genomic clone 2169F21, genomic survey sequence.//8.4e-16:109:94//B89870
F-NT2RP4001339//HS_3205_B1_E08_T7 CIT Approved Human Genomic Sperm Library D Homo sapiens genomic clone Plate=3205 Col=15 Row=J, genomic survey sequence.//7.1e-24:305:73//AQ183725
F-NT2RP4001343//Homo sapiens PAC clone DJ0894A10 from 7q32-q32, complete sequence.//1.9e-17:106:91//AC004918
F-NT2RP4001345//G.gallus mRNA for lecithin-cholesterol acyltransferase.//7.6e-40:631:66//X91011
F-NT2RP4001351//Human DNA sequence *** SEQUENCING IN PROGRESS *** from clone 184J9, WORKING DRAFT SEQUENCE.//2.7e-30:608:64//AL031428
F-NT2RP4001353//Streptomyces coelicolor cosmid 5A7.//0.23:540:57//AL031107
F-NT2RP4001372//RPCI11-49L11.TJ RPCI11 Homo sapiens genomic clone R-49L11, genomic survey sequence.//8.5e-23:129:100//AQ051701
F-NT2RP4001373//G.gallus genomic DNA repeat region, clone 16E1.//0.15:213:61//X78609
F-NT2RP4001375
F-NT2RP4001379//Homo sapiens chromosome 17, clone hRPK.311_F_12, complete sequence.//7.3e-28:153:88//AC005722
F-NT2RP4001389//Homo sapiens PAC clone DJ0740D02 from 7p14-p15, complete sequence.//7.2e-47:518:73//AC004691
F-NT2RP4001407//P.falciparum glutamic acid-rich protein gnen, complete cds.//0.00079:686:57//J03998
F-NT2RP4001414//Human mRNA for KIAA0202 gene, partial cds.//2.0e-76:818:71//D86957
F-NT2RP4001433//H.sapiens HZF10 mRNA for zinc finger protein.//3.5e-87:839:73//X78933
F-NT2RP4001442
F-NT2RP4001447//Homo sapiens mRNA for KIAA0783 protein, complete cds.//0.21:218:63//AB018326
F-NT2RP4001474//Human NotI linking clone 924A058R, genomic survey sequence.//7.6e-14:109:90//U49884
F-NT2RP4001483//Human mRNA for 2-oxoglutarate dehydrogenase, complete cds.//2.5e-59:480:75//D10523
F-NT2RP4001498//Homo sapiens huntingtin interacting protein HYPH mRNA, partial cds.//9.7e-39:392:72//AF049612
F-NT2RP4001502//H.sapiens (D8S135) DNA segment containing GT repeat.//2.7e-24:147:96//X61693
F-NT2RP4001507//Plasmid pSB24.2 (from S.cyanogenus) neomycin resistance protein gene, complete cds.//0.87:583:58//M32513
F-NT2RP4001524//Plasmodium falciparum 3D7 chromosome 12 PFYAC492 genomic sequence, WORKING DRAFT SEQUENCE, 5 unordered pieces.//0.93:394:58//AC005308
F-NT2RP4001529//Mus domesticus nuclear binding factor NF2d9 mRNA, complete cds.//3.1e-143:820:89//U20086
F-NT2RP4001547//S.cerevisiae chromosome XIV reading frame ORF YNR048w.//2.2e-05:319:61//Z71663
F-NT2RP4001551//S.pombe chromosome II p1 p8B7.//0.64:335:60//AL032684
F-NT2RP4001555//Homo sapiens 12q24.2 BAC RPCI11-360E11 (Roswell Park Cancer Institute Human BAC Library) complete sequence.//1.0:309:58//AC004806
F-NT2RP4001567//HS_2166_B1_C07_MF CIT Approved Human Genomic Sperm Library D Homo sapiens genomic clone Plate=2166 Col=13 Row=F, genomic survey sequence.//0.99:188:59//AQ086290
F-NT2RP4001568//Human mRNA for KIAA0167 gene, complete cds.//7.0e-53:566:72//D79989
F-NT2RP4001571//RPCI11-21F20.TP RPCI-11 Homo sapiens genomic clone RPCI-11-21F20, genomic survey sequence.//2.8e-19:119:97//B85885
F-NT2RP4001574//B.primigenius mRNA for coat protein gamma-cop.//5.8e-129:813:85//X92987
F-NT2RP4001575//Rattus norvegicus mRNA for ARE1 protein.//3.4e-131:795:86//AJ223830
F-NT2RP4001592//S.aureus gene for isoleucyl-tRNA synthetase.//1.3e-14:663:59//X74219
F-NT2RP4001610//Homo sapiens Xp22 Cosmids U15E4, U115H5, U132E12, U115B9 (Lawrence Livermore human cosmid library) complete sequence.//6.4e-10:135:73//AC002364
F-NT2RP4001614//HS_3042_B2_D05_MF CIT Approved Human Genomic Sperm Library D Homo sapiens genomic clone Plate=3042 Col=10 Row=H, genomic survey sequence.//3.4e-06:78:89//AQ099333
F-NT2RP4001634
F-NT2RP4001638//cSRL-161F1-u cSRL flow sorted Chromosome 11 specific cosmid Homo sapiens genomic clone cSRL-161Fl, genomic survey sequence.//4.9e-12:144:76//B02870
F-NT2RP4001644//M.musculus mRNA for map kinase interacting kinase, Mnk2.//3.8e-69:437:86//Y11092
F-NT2RP4001656//HS_2013_A1_D01_MR CIT Approved Human Genomic Sperm Library D Homo sapiens genomic clone Plate=2013 Col=1 Row=G, genomic survey sequence.//2.0e-30:207:89//AQ224793
F-NT2RP4001677//Hylobates lar huntingtin gene, partial exon.//0.23:105:71//L49362
F-NT2RP4001679//Human DNA sequence *** SEQUENCING IN PROGRESS *** from clone 462O23, WORKING DRAFT SEQUENCE.//2.7e-45:351:84//AL031431
F-NT2RP4001696//Human chromosome 8 BAC clone CIT987SK-2A8 complete sequence.//1.8e-30:163:88//U96629
F-NT2RP4001725//Drosophila melanogaster DNA sequence (P1 DS08860 (D181)), complete sequence.//1.1e-13:402:63//AC004296
F-NT2RP4001730//RPCI11-37M21.TK RPCI-11 Homo sapiens genomic clone RPCI-11-37M21, genomic survey sequence.//0.88:177:67//AQ029840
F-NT2RP4001739
F-NT2RP4001753//H.sapiens telomeric DNA sequence, clone 12QTEL023, read 12QTELOO023.seq.//4.9e-36:192:98//Z96232
F-NT2RP4001760//Mouse oncogene (ect2) mRNA, complete cds.//2.3e-140:866:86//L11316
F-NT2RP4001790//Homo sapiens clone NH0569I24, complete sequence.//1.4e-29:327:74//AC005678
F-NT2RP4001803
F-NT2RP4001822//Homo sapiens tetraspan TM4SF (TSPAN-4) mRNA, complete cds.//1.0e-16:576:60//AF054841
F-NT2RP4001823//Human DNA sequence from clone 181C9 on chromosome 22q13.2-13.33. Contains a PHAPI2 Leucine Rich Acidic Nuclear Protein pseudogene, part of a putative novel gene, ESTs, STSs and GSSs, complete sequence.//2.1e-08:601:59//Z98743
F-NT2RP4001828
F-NT2RP4001838//Human mRNA for KIAA0071 gene, partial cds.//2.2e-53:555:73//D31888
F-NT2RP4001841
F-NT2RP4001849//Homo sapiens mRNA for KIAA0672 protein, complete cds.//1.7e-55:813:65//AB014572
F-NT2RP4001861//Human simple repeat polymorphism.//0.0014:145:66//M87691
F-NT2RP4001889//HS_2052_B1_H06_T7 CIT Approved Human Genomic Sperm Library D Homo sapiens genomic clone Plate=2052 Col=11 Row=P, genomic survey sequence.//1.0e-23:187:86//AQ270425
F-NT2RP4001893//Homo sapiens BAC clone GS166A23 from 7p21, complete sequence.//7.3e-76:178:95//AC005014
F-NT2RP4001896//T3B4TFC TAMU Arabidopsis thaliana genomic clone T3B4, genomic survey sequence.//0.99:354:61//B26193
F-NT2RP4001901//Streptomyces griseus genes for Orf2, Orf3, Orf4, Orf5, AfsA, Orf8, partial and complete cds.//0.031 :409:60//AB011413
F-NT2RP4001927//HS_2216_B1_D03_MF CIT Approved Human Genomic Sperm Library D Homo sapiens genomic clone Plate=2216 Col=5 Row=H, genomic survey sequence.//4.9e-32:216:89//AQ184677
F-NT2RP4001938//Mus musculus zinc finger protein (Zfp64) mRNA, complete cds.//1.2e-83:709:79//U49046
F-NT2RP4001946//HS_3021_B2_H10_MR CIT Approved Human Genomic Sperm Library D Homo sapiens genomic clone Plate=3021 Col=20 Row=P, genomic survey sequence.//7.6e-09:120:76//AQ133185
F-NT2RP4001950//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//2.1e-18:421:65//AL022577
F-NT2RP4001953//CIT-HSP-2294D14.TR CIT-HSP Homo sapiens genomic clone 2294D14, genomic survey sequence.//0.030:358:61//AQ005028
F-NT2RP4001966//Mus musculus DOC4 (Doc4) mRNA, complete cds.//2.5e-68:812:68//AF059485
F-NT2RP4001975//Homo sapiens chromosome 17, clone hCIT.91_J_4, complete sequence.//1.9e-57:555:75//AC003976
F-NT2RP4002018//cSRL-143G4-u cSRL flow sorted Chromosome 11 specific cosmid Homo sapiens genomic clone cSRL-143G4, genomic survey sequence.//8.9e-21:123:98//B01950 F-NT2RP4002047//Saccharomyces cerevisiae chromosome XII cosmid 8003.//1.6e-29:520:64//U17243
F-NT2RP4002052//CIT-HSP-2045A15.TF CIT-HSP Homo sapiens genomic clone 2045A15, genomic survey sequence.//2.8e-22:137:96//B80243
F-NT2RP4002058//T20L11-T7 TAMU Arabidopsis thaliana genomic clone T20L11, genomic survey sequence.//0.019:141:65//AQ248640
F-NT2RP4002071//CIT-HSP-2314J9.TF CIT-HSP Homo sapiens genomic clone 2314J9, genomic survey sequence.//0.99:163:63//AQ027223
F-NT2RP4002075//Caenorhabditis elegans DNA *** SEQUENCING IN PROGRESS *** from clone Y57G11, WORKING DRAFT SEQUENCE.//0.15:506:59//Z92841
F-NT2RP4002078//RPCI11-73M20.TJ RPCI11 Homo sapiens genomic clone R-73M20, genomic survey sequence.//4.8e-21:130:96//AQ269030
F-NT2RP4002081//F.rubripes GSS sequence, clone 190O22bB9, genomic survey sequence.//0.0024:350:60//Z92062
F-NT2RP4002083//M.musculus tex27 mRNA.//8.2e-77:456:89//X80437
F-NT2RP4002408//Caenorhabditis elegans serine/threonine kinase LET-502 (let-502) mRNA, complete cds.//3.7e-18:541:62//U85515
F-NT2RP4002791
F-NT2RP4002888//Homo sapiens BAC clone RG067E13 from 7q21, complete sequence.//4.7e-39:385:75//AC002383
F-NT2RP4002905//Homo sapiens chromosome 17, clone hRPC.842_A_23, complete sequence.//6.5e-91:672:83//AC004662
F-NT2RP5003459//Human glyceraldehyde-3-phosphate dehydrogenase (GAPDH) mRNA, complete cds.//2.9e-37:193:99//M33197
F-NT2RP5003461//Human DNA sequence from PAC 506G2 contains ESTs.//7.9e-51:300:80//Z82901
F-NT2RP5003477//Human Chromosome 3 pac pDJ70i11, WORKING DRAFT SEQUENCE, 2 unordered pieces.//6.7e-77:150:100//AC000380
F-NT2RP5003492
F-NT2RP5003500//Human DNA sequence from cosmid 97K10, between markers DXS6791 and DXS8038 on chromosome X contains STSs and CpG island.//1.7e-111:623:93//Z81365
F-NT2RP5003506//H.sapiens CpG island DNA genomic Mse1 fragment, clone 71h2, reverse read cpg71h2.rt1a.//1.4e-49:283:93//Z62703
F-NT2RP5003512//HS_3084_A1_D04_MF CIT Approved Human Genomic Sperm Library D Homo sapiens genomic clone Plate=3084 Col=7 Row=G, genomic survey sequence.//7.7e-18:117:95//AQ186312
F-NT2RP5003522//Homo sapiens clone NH0479C13, WORKING DRAFT SEQUENCE, 12 unordered pieces.//3.8e-101:211:96//AC005236
F-NT2RP5003524//Homo sapiens beta-spectrin (HSpTB1) gene, exon 14 and partial cds.//0.00056:650:57//AF013178
F-NT2RP5003534//H.sapiens CpG island DNA genomic Mse1 fragment, clone 14c10, forward read cpg14c10.ft1b.//0.00013:70:91//Z54631
F-OVARC1000001//Homo sapiens mRNA for KIAA0465 protein, partial cds.//1.2e-67:373:94//AB007934
F-OVARC1000004//Homo sapiens chromosome 4 clone B368A9 map 4q25, complete sequence.//5.8e-93:518:81//AC005510
F-OVARC1000006//Gallus gallus histone H2A (H2A-VIII) gene, complete cds.//9.1e-56:392:84//U38933
F-OVARC1000013
F-OVARC1000014//Homo sapiens GLE1 (GLE1) mRNA, complete cds.//5.6e-170:815:98//AF058922
F-OVARC1000017//Streptomyces glaucescens tcm operon.//0.37:347:60//M80674
F-OVARC1000035//Homo sapiens GA17 protein mRNA, complete cds.//6.8e-36:238:89//AF064603
F-OVARC1000058
F-OVARC1000060//Homo sapiens ribonuclease 6 precursor, mRNA, complete cds.//2.5e-36:192:98//U85625
F-OVARC1000068//Human DNA sequence *** SEQUENCING IN PROGRESS *** from clone 404K8, WORKING DRAFT SEQUENCE.//0.14:554:57//AL023883
F-OVARC1000071//Human DNA sequence *** SEQUENCING IN PROGRESS *** from clone 596C15, WORKING DRAFT SEQUENCE.//5.3e-104:197:100//AL031387
F-OVARC1000085//Human DNA sequence from clone 191N21 on chromosome 6q27 Contains genes for PDCD2 (PROGRAMMED CELL DEATH-2/RP8 HOMOLOG), TATA factor (TFIID), proteasome subunit HC5, EST, STS, GSS, complete sequence.//1.6e-116:588:96//AL031259
F-OVARC1000087//HS_2004_B2_E11_MR CIT Approved Human Genomic Sperm Library D Homo sapiens genomic clone Plate=2004 Col=22 Row=J, genomic survey sequence.//7.1e-11:94:94//AQ221037
F-OVARC1000091//nbxb0020P17r CUGI Rice BAC Library Oryza sativa genomic clone nbxb0020P17r, genomic survey sequence.//5.2e-05:238:64//AQ258489
F-OVARC1000092//Homo sapiens chromosome Y, clone 264,M,20, complete sequence.//1.1e-10:720:58//AC004617
F-OVARC1000106//HS_3212_B2_G12_MR CIT Approved Human Genomic Sperm Library D Homo sapiens genomic clone Plate=3212 Col=24 Row=N, genomic survey sequence.//9.9e-05:141:73//AQ175369
F-OVARC1000109
F-OVARC1000113//Homo sapiens okadaic acid-inducible phosphoprotein (OA48-18) mRNA, complete cds.//1.6e-133:663:96//AF069250
F-OVARC1000114//Human DNA sequence *** SEQUENCING IN PROGRESS *** from clone 1111N9, WORKING DRAFT SEQUENCE.//2.3e-51:547:70//AL022574
F-OVARC1000133//Homo sapiens clone GS512I21, WORKING DRAFT SEQUENCE, 9 unordered pieces.//0.62:349:61//AC005027
F-OVARC1000139//Caenorhabditis elegans cosmid F09D1.//2.5e-18:314:64//AF040640
F-OVARC1000145//HS_2257_B2_D11_MR CIT Approved Human Genomic Sperm Library D Homo sapiens genomic clone Plate=2257 Col=22 Row=H, genomic survey sequence.//5.8e-30:203:90//AQ304854
F-OVARC1000148//CIT-HSP-2345A22.TR CIT-HSP Homo sapiens genomic clone 2345A22, genomic survey sequence.//1.1e-26:146:100//AQ056703
F-OVARC1000151//Sequence 1 from patent US 5665588.//2.6e-61:677:70//I64695
F-OVARC1000168//Homo sapiens chromosome 19, cosmid R31343, complete sequence.//4.9e-19:381:63//AC005764
F-OVARC1000191//Plasmodium falciparum DNA *** SEQUENCING IN PROGRESS *** from MAL4P1, WORKING DRAFT SEQUENCE.//1.3e-06:745:57//AL034557
F-OVARC1000198//*** SEQUENCING IN PROGRESS *** Homo sapiens chromosome 4, BAC clone C0366H07; HTGS phase 1, WORKING DRAFT SEQUENCE, 28 unordered pieces.//6.4e-161:781:97//AC004604
F-OVARC1000209//Oryza sativa submergence induced protein 2A mRNA, complete cds.//9.2e-33:511:65//AF068332
F-OVARC1000212//F.rubripes GSS sequence, clone 185L11aC1, genomic survey sequence.//1.1e-13:139:79//AL019910
F-OVARC1000240//Sequence 1 from patent US 5710024.//1.4e-129:623:98//I81226
F-OVARC1000241//Mus musculus hypoxia inducible factor three alpha mRNA, complete cds.//1.1e-112:697:87//AF060194
F-OVARC1000288 2.2e-22:181:83//J00345
F-OVARC1000302//A-192A9.TP CIT978SK Homo sapiens genomic clone A-192A9, genomic survey sequence.//4.8e-18:110:99//B18003
F-OVARC1000304//Mouse mRNA from Mov10 locus.//5.5e-100:631:85//X52574
F-OVARC1000309
F-OVARC1000321//Homo sapiens clone NH0479C13, WORKING DRAFT SEQUENCE, 12 unordered pieces.//3.1e-122:325:95//AC005236
F-OVARC1000326//Rattus norvegicus lamina-associated polypeptide 1C (LAP1C) mRNA, complete cds.//4.0e-46:339:84//U19614
F-OVARC1000335//Caenorhabditis elegans cosmid F15B10.//0.020:545:57//AF036696
F-OVARC1000347//Homo sapiens clone GS051M12, complete sequence.//0.71:252:59//AC005007
F-OVARC1000384//Homo sapiens expanded SCA7 CAG repeat.//2.2e-09:276:64//AF020275
F-OVARC1000408//Human Chromosome 11p15.5 PAC clone pDJ915f1 containing KvLQT1 gene, complete sequence.//0.61:343:59//AC003693
F-OVARC1000411//S.cerevisiae chromosome XI reading frame ORF YKL202w.//0.075:242:60//Z28201
F-OVARC1000414//Homo sapiens PAC clone DJ0905M06 from 7q31, complete sequence.//0.00088:285:62//AC005166
F-OVARC1000420//Human DNA sequence *** SEQUENCING IN PROGRESS *** from clone 371H6, WORKING DRAFT SEQUENCE.//0.14:487:60//AL031718
F-OVARC1000427//Homo sapiens clone UWGC:rg041a03 from 7p14-15, complete sequence.//4.9e-30:195:84//AC005826
F-OVARC1000431//Plasmodium falciparum MAL3P2, complete sequence.//1.3e-05:651:59//AL034558
F-OVARC1000437//Chicken tensin mRNA, complete cds.//9.6e-54:296:78//M74165
F-OVARC1000440//Human PINCH protein mRNA, complete cds.//2.7e-19:116:99//U09284
F-OVARC1000442//Human DNA sequence from clone 816K17 on chromosome 20p12.2-13 Contains TGM3 (PROTEIN-GLUTAMINE GLUTAMYLTRANSFERASE E3 PRECURSOR (EC 2.3.2.13) (TGASE E3) (TRANSGLUTAMINASE 3), and another member of the Transglutaminase family, complete sequence.//1.0e-21:202:79//AL031678 F-OVARC1000443//Homo sapiens mRNA for KIAA0683 protein, complete cds.//1.0e-138:566:99//AB014583
F-OVARC1000461
F-OVARC1000465//Bos taurus guanine nucleotide-exchange protein (ARF-GEP1) mRNA, complete cds.//4.7e-124:650:93//AF023451
F-OVARC1000466//Homo sapiens DNA from chromosome 19, cosmid R29144, complete sequence.//1.0e-15:510:59//AC004221
F-OVARC1000473//Ciona intestinalis genomic fragment, clone 3F4, genomic survey sequence.//2.5e-06:272:62//AJ227191
F-OVARC1000479//cDNA encoding novel rat protein TIP120 which is formed of complex with TBP (TATA binding protein).//1.1e-117:652:90//E12829
F-OVARC1000486//Homo sapiens DNA sequence from PAC 262D12 on chromosome 1q23.3-24.3. Contains a Tenascin (Hexabrachion, Cytotactin, Neuronectin, Myotendinous antigen)-LIKE gene and a mitochondrial/chloroplast 30S ribosomal protein S14-LIKE gene preceeded by a CpG island. Contains ESTs, genomic marker D1S2691 and STSs.//1.7e-13:709:60//Z99297
F-OVARC1000496//Human DNA sequence *** SEQUENCING IN PROGRESS *** from clone 455J7, WORKING DRAFT SEQUENCE.//6.0e-23:316:72//AL031733
F-OVARC1000520//Homo sapiens supervillin mRNA, complete cds.//2.1e-113:539:99//AF051850
F-OVARC1000526//Homo sapiens clone GS438P06, WORKING DRAFT SEQUENCE, 17 unordered pieces.//8.0e-149:716:98//AC005024
F-OVARC1000533//Homo sapiens chromosome 19, cosmid R30385, complete sequence.//5.8e-137:545:97//AC004510
F-OVARC1000543//HS_3055_A2_F10_MF CIT Approved Human Genomic_Sperm Library D Homo sapiens genomic clone Plate=3055 Col=20 Row=K, genomic survey sequence.//0.19:104:71//AQ102820
F-OVARC1000556//Homo sapiens DNA sequence from PAC 168L15 on chromosome 6q26-27. Contains RSK3 gene, ribosomal protein S6 kinase, EST, GSS, STS. CpG island, complete sequence.//4.4e-136:670:97//AL022069
F-OVARC1000557//Human DNA from chromosome 19-specific cosmid R27090, genomic sequence, complete sequence.//1.3e-15:262:69//AC002985
F-OVARC1000564//Mus musculus clone OST7314, genomic survey sequence.//1.9e-41:476:70//AF046733
F-OVARC1000573//HS_3241_B1_H03_T7 CIT Approved Human Genomic Sperm Library D Homo sapiens genomic clone Plate=3241 Col=5 Row=P, genomic survey sequence.//2.2e-101:530:95//AQ211942
F-OVARC1000576//Human Chromosome X, WORKING DRAFT SEQUENCE, 2 unordered pieces.//9.7e-97:445:90//AC002414
F-OVARC1000578//Homo sapiens DNA from chromosome 19, BAC 33152, complete sequence.//9.1 e-27:354:72//AC003973
F-OVARC1000588//Human DNA sequence from clone 497J21 on chromosome 6q26-27. Contains a KOC (KH-domain containg transcript overexpressed in cancer) pseudogene, genomic marker D6S193, ESTs, STSs and GSSs, and a ca repeat polymorphism, complete sequence.//0.97:276:62//AL023775
F-OVARC1000605
F-OVARC1000622//Homo sapiens (subclone 2_d8 from P1 H42) DNA sequence, complete sequence.//7.2e-60:457:82//L81648
F-OVARC1000640//Human BAC clone RG326K09 from 7q21, complete sequence.//6.2e-58:499:80//AC002069
F-OVARC1000649//Human squamous cell carcinama of esophagus mRNA for GRB-7 SH2 domain protein, complete cds.//5.1e-77:424:93//D43772
F-OVARC1000661//Homo sapiens mRNA for KIAA0590 protein, complete cds.//4.8e-99:536:94//AB011162
F-OVARC1000678//cSRL-29c7-u cSRL flow sorted Chromosome 11 specific cosmid Homo sapiens genomic clone cSRL-29c7, genomic survey sequence.//2.5e-57:336:91//B04244
F-OVARC1000679//Rattus norvegicus mRNA for myosin-RhoGAP protein Myr 7.//1.6e-81:291:84//AJ001713
F-OVARC1000681//Human DNA sequence *** SEQUENCING IN PROGRESS *** from clone 257E24, WORKING DRAFT SEQUENCE.//8.2e-158:782:96//AL034424
F-OVARC1000682//Homo sapiens alpha 1,2-mannosidase IB mRNA, complete cds.//1.5e-151:549:99//AF027156
F-OVARC1000689//nbxb0003aG01f CUGI Rice BAC Library Oryza sativa genomic clone nbxb0003M01f, genomic survey sequence.//0.17:499:60//AQ050003
F-OVARC1000700
F-OVARC1000703//Drosophila melanogaster DNA repair protein (mei-41) gene, complete cds, and TH1 gene, partial cds.//3.5e-26:425:65//U34925
F-OVARC1000722//Homo sapiens chromosome 1q21-1q23 beta-1,4-galactosyltransferase mRNA, complete cds.//3.7e-109:451:91//AF038661
F-OVARC1000730
F-OVARC1000746
F-OVARC1000769//HS_2056_B2_G06_T7 CIT Approved Human Genomic Sperm-Library D Homo sapiens genomic clone Plate=2056 Col=12 Row=N, genomic survey sequence.//8.8e-19:147:86//AQ245905
F-OVARC1000771//M.musculus mRNA for GTP-binding protein.//2.2e-62:305:78//X95403
F-OVARC1000781//Sequence 5 from Patent WO9722695.//1.9e-89:705:78//A63552
F-OVARC1000787//Homo sapiens PAC clone DJ430N08 from 22q12.1-qter, complete sequence.//3.0e-131:631:98//AC004542
F-OVARC1000800//Human Chromosome 11q23 PAC clone pDJ254e13, complete sequence.//1.7e-32:295:80//AC003691
F-OVARC1000802//Homo sapiens chromosome Xp22-67-68, WORKING DRAFT SEQUENCE, 99 unordered pieces.//3.2e-55:356:88//AC004469
F-OVARC1000834//Homo sapiens mRNA for atopy related autoantigen CALC.//9.5e-27:163:94//Y17711
F-OVARC1000846//Homo sapiens mRNA for KIAA0643 protein, partial cds.//6.0e-150:432:100//AB014543
F-OVARC1000850//Homo sapiens PB39 mRNA, complete cds.//1.0e-135:632:99//AF045584
F-OVARC1000862//M.musculus mRNA for FT1.//2.6e-109:769:83//Z67963
F-OVARC1000876//S.cerevisiae chromosome IX cosmid 9150.//7.4e-21:541:61//Z38125
F-OVARC1000883//Mus domesticus nuclear binding factor NF2d9 mRNA, complete cds.//2.2e-08:98:88//U20086
F-OVARC1000885//B.subtilis 25 kb genomic DNA segment (from sspE to katA).//0.25:231:61//Z82044
F-OVARC1000886//CIT-HSP-2171H6.TR CIT-HSP Homo sapiens genomic clone 2171H6, genomic survey sequence.//0.00035:139:69//B89721
F-OVARC1000890
F-OVARC1000891
F-OVARC1000897//Human DNA sequence from clone 215F16 on chromosome 22q12.1-12.3. Contains part of a Homeobox domain containing gene and GSSs, complete sequence.//1.4e-18:473:64//AL024494
F-OVARC1000912//Bovine herpesvirus type 1 early-intermediate transcription control protein (BICP4) gene, complete cds.//8.9e-08:378:63//L14320
F-OVARC1000915//Homo sapiens mRNA for KIAA0600 protein, partial cds.//7.7e-85:440:95//AB011172
F-OVARC1000924//HS_2022_A1_C01_T7 CIT Approved Human Genomic Sperm Library D Homo sapiens genomic clone Plate=2022 Col=1 Row=E, genomic survey sequence.//5.7e-21:122:99//AQ269493
F-OVARC1000936//Human PAC clone DJ0093I03 from Xq23, complete sequence.//1.2e-113:476:91//AC003983
F-OVARC1000937//Human DNA sequence *** SEQUENCING IN PROGRESS *** from clone 20208, WORKING DRAFT SEQUENCE.//0.00066:436:61//AL031848
F-OVARC1000945//Rattus norvegicus mRNA for atypical PKC specific binding protein, complete cds.//5.0e-89:556:86//AB005549
F-OVARC1000948//P.falciparum complete gene map of plastid-like DNA (IR-B).//0.98:160:64//X95276
F-OVARC1000959//CIT-HSP-2348O16.TR CIT-HSP Homo sapiens genomic clone 2348O16, genomic survey sequence.//0.99:270:59//AQ062850
F-OVARC1000960//Human DNA sequence from PAC 212P9 on chromosome 1p34.1-1p35. Contains delta opiate receptor, CpG island, CA repeat,.//3.9e-41:577:72//AL009181
F-OVARC1000964//P.falciparum malaria antigen (M26-32-2) gene, partial cds.//0.19:83:73//M63270
F-OVARC1000971//Caenorhabditis elegans DNA *** SEQUENCING IN PROGRESS *** from clone Y57G11, WORKING DRAFT SEQUENCE.//0.013:670:57//Z92841
F-OVARC1000984//Leishmania major chromosome 1, complete sequence.//0.80:345:58//AE001274
F-OVARC1000996//MO25 gene [mice, embryos, mRNA, 2322 nt].//2.6e-55:403:82//S51858
F-OVARC1000999//Synthetic construct galanin receptor type 3 (GALR3) gene, complete cds.//0.33:105:69//AF042785
F-OVARC1001000//HS_2247_A1_H05_MR CIT Approved Human Genomic Sperm Library D Homo sapiens genomic clone Plate=2247 Col=9 Row=O, genomic survey sequence.//3.1e-60:315:96//AQ153910
F-OVARC1001004//Homo sapiens from UWGC:y18c282 from 6p21, complete sequence.//3:1e-124:595:98//AC004190
F-OVARC1001010//CIT-HSP-2034M3.TF CIT-HSP Homo sapiens genomic clone 2034M3, genomic survey sequence.//1.0:151:60//B74290
F-OVARC1001011//Human DNA sequence from cosmid U85A3, between markers DXS366 and DXS87 on chromosome X contains rad21 and T-cell cyclophorin pseudogenes, STS.//3.0e-08:149:79//Z78021
F-OVARC1001032//Yeast (S.cerevisiae) mitochondrial Tyr-tRNA gene.//3.2e-13:667:60//M12451
F-OVARC1001034//Mus musculus Fn54 mRNA, partial cds.//2.5e-119:737:86//AF001533
F-OVARC1001038//Homo sapiens TRIAD1 type I mRNA, complete cds.//2.7e-150:733 :97//AF099149
F-OVARC1001040//Homo sapiens clone RG270D13, WORKING DRAFT SEQUENCE, 18 unordered pieces.//9.8e-29:277:76//AC005081
F-OVARC1001044//Human DNA sequence *** SEQUENCING IN PROGRESS *** from clone 364I1, WORKING DRAFT SEQUENCE.//0.0017:387:6.1//AL031319
F-OVARC1001051//Rattus norvegicus brain specific cortactin-binding protein CBP90 mRNA, partial cds.//0.012:112:74//AF053768
F-OVARC1001055//Sequence 1 from patent US 5580754.//3.3e-45:381:81//I30292
F-OVARC1001062//nbxb0026H08r CUGI Rice BAC Library Oryza sativa genomic clone nbxb0026H08r, genomic survey sequence.//0.018:344:59//AQ271878
F-OVARC1001065//S.pombe chromosome I cosmid c29E6.//0.86:338:59//Z66525
F-OVARC1001068//Homo sapiens Era GTPase A protein (HERA-A) mRNA, partial cds.//2.0e-130:620:98//AF082657
F-OVARC1001072//Homo sapiens glypican 3 (GPC3) gene, partial cds and flanking repeat regions.//9.3e-24:285:65//AF003529
F-OVARC1001074//Human DNA sequence from clone 23K20 on chromosome Xq25-26.2 Contains EST, STS, GSS, complete sequence.//2.0e-07:652:59//AL022153
F-OVARC1001085//Homo sapiens c-syn protooncogene mRNA, complete cds.//5.0e-35:187:99//M14333
F-OVARC1001092//Homo sapiens mRNA for JM5 protein, complete CDS (clone IMAGE 53337, LLNLc110F1857Q7 (RZPD Berlin) and LLNLc110G0913Q7 (RZPD Berlin)).//4.0e-74:289:95//AJ005897
F-OVARC1001107//Homo sapiens SKB1Hs mRNA, complete cds.//3.6e-72:351:86//AF015913
F-OVARC1001113//Homo sapiens diaphanous 1 (HDIA1) mRNA, complete cds.//6.4e-150:710:98//AF051782
F-OVARC1001117//Homo sapiens chromosome 5, P1 clone 328E3 (LBNL H53), complete sequence.//0.99:148:67//AC005178
F-OVARC1001118//Human Chromosome 11 pac pDJ197h17, WORKING DRAFT SEQUENCE, 11 unordered pieces.//2.6e-35:302:74//AC000382
F-OVARC1001129//CIT-HSP-647P20.TP CIT-HSP Homo sapiens genomic clone 647P20, genomic survey sequence.//0.94:106:66//B79052
F-OVARC1001154//R.norvegicus mRNA for epithelin 1 and 2.//1.8e-95:462:79//X62322
F-OVARC1001161//Homo sapiens chromosome 4 clone B71M12 map 4q25, complete sequence.//2.9e-90:496:84//AC004069
F-OVARC1001162
F-OVARC1001167//Homo sapiens clone DJ1098J04, WORKING DRAFT SEQUENCE, 2 unordered pieces.//0.00090:219:64//AC004961
F-OVARC1001169//Borrelia burgdorferi (section 27 of 70) of the complete genome.//1.0:265:59//AE001141
F-OVARC1001170//H.sapiens (xs170) mRNA, 350bp.//4.6e-58:355:90//Z36823
F-OVARC1001171//CIT-HSP-2285E22.TF CIT-HSP Homo sapiens genomic clone 2285E22, genomic survey sequence.//1.5e-25:152:83//AQ002315
F-OVARC1001173//Human DNA sequence from clone 243E7 on chromosome 22q12.1. Contains ESTs, STSs and GSSs, complete sequence.//0.0024:94:80//AL022323
F-OVARC1001176//Streptomyces plicatus B-N-acetylhexosaminidase (hex) gene, complete cds.//1.0:356:60//AF063001
F-OVARC1001180//G.gallus DNA for polyubiquitin gene Ub II.//0.0062:275:60//X58195
F-OVARC1001188//Homo sapiens full-length insert cDNA clone ZD93F03.//1.8e-32:180:97//AF086486
F-OVARC1001200
F-OVARC1001232//Caenorhabditis elegans cosmid F10B5, complete sequence.//0.013:128:67//Z48334
F-OVARC1001240//Human Chromosome 11 pac pDJ360p17, WORKING DRAFT SEQUENCE, 44 unordered pieces.//3.7e-131:811:87//AC001235
F-OVARC1001243//Human BAC clone GS117O10 from 7q21-q22, complete sequence.//0.044:457:59//AC003078
F-OVARC1001244//Human homolog of Drosophila female sterile homeotic mRNA, complete cds.//8.4e-18:118:95//M80613
F-OVARC1001261//Mus musculus putative membrane-associated guanylate kinase 1 (Magi-1) mRNA, alternatively spliced c form, partial cds.//1.4e-95 :649:84//AF027505
F-OVARC1001268//Rattus norvegicus ADP-ribosylation factor-directed GTPase activating protein mRNA, complete cds.//0.00051:72:83//U35776
F-OVARC1001270
F-OVARC1001271//Homo sapiens mRNA for KIAA0643 protein, partial cds.//2.1e-142:644:96//AB014543
F-OVARC1001282//RPCI11-60K8.TK RPCI11 Homo sapiens genomic clone R-60K8, genomic survey sequence.//0.0089:285:58//AQ195857
F-OVARC1001296//Homo sapiens echinoderm microtubule-associated protein homolog HuEMAP mRNA, complete cds.//3.0e-20:263:73//U97018
F-OVARC1001306//nbxb0002M13r CUGI Rice BAC Library Oryza sativa genomic clone nbxb0002M13r, genomic survey sequence.//0.98:170:66//AQ156061
F-OVARC1001329//Homo sapiens BAC clone RG370M10 from 7p15, complete sequence.//1.3e-05:432:61//AC003986
F-OVARC1001330//Plasmodium falciparum 3D7 chromosome 12 PFYAC1383 genomic sequence, WORKING DRAFT SEQUENCE, 3 unordered pieces.//0.027:444:59//AC005504
F-OVARC1001339//Homo sapiens chromosome 17, clone hCIT.124_H_2, complete sequence.//0.76:89:74//AC006071
F-OVARC1001341//CITBI-E1-2503J7.TR CITBI-E1 Homo sapiens genomic clone 2503J7, genomic survey sequence.//0.99:45:86//AQ263402
F-OVARC1001342
F-OVARC1001344//HS-1059-A2-H02-MF.abi CIT Human Genomic Sperm Library C Homo sapiens genomic clone Plate=CT 781 Col=4 Row=O, genomic survey sequence.//1.5e-07:254:67//B44456
F-OVARC1001357//Homo sapiens Xp22-149 BAC RPCI11-466O4 (Roswell Park Cancer Institute Human BAC Library) complete sequence.//0.83:376:61//AC005297
F-OVARC1001360
F-OVARC1001369//Homo sapiens clone 162B15, complete sequence.//0.0066:99:76//AC004811
F-OVARC1001372//Homo sapiens liprin-alpha4 mRNA, partial cds.//2.7e-142:683:98//AF034801
F-OVARC1001376//Human DNA sequence *** SEQUENCING IN PROGRESS *** from clone 850H21, WORKING DRAFT SEQUENCE.//1.9e-52:382:73//AL031680
F-OVARC1001381//Homo sapiens mRNA for candidate tumor suppressor involved in B-CLL.//1.2e-147:683:99//AJ224819
F-OVARC1001391//S.coelicolor whiB gene.//0.018:454:59//X62287
F-OVARC1001399//CIT-HSP-2291I8.TR CIT-HSP Homo sapiens genomic clone 2291I8, genomic survey sequence.//1.7e-11 :104:87//AQ007611
F-OVARC1001417//Homo sapiens EXLM1 mRNA, complete cds.//3.9e-149:707:98//AB006651
F-OVARC1001419//Homo sapiens GOK (STIM1) mRNA, complete cds.//4.9e-48:586:69//U52426
F-OVARC1001425//Human DNA sequence from clone 1048E9 on chromosome 22q11.2-12.2 Contains pseudogene similar to ribosomal protein S3A and part of a gene similar to C.elegans protein CE02118, ESTs, STS, GSS, complete sequence.//0.0019:96:78//Z99714
F-OVARC1001436//Caenorhabditis elegans mitotic chromosome and X-chromosome associated MIX-1 protein (mix-1) mRNA, complete cds.//0.77:519:59//U96387
F-OVARC1001442//Human DNA sequence *** SEQUENCING IN PROGRESS *** from clone 998H6, WORKING DRAFT SEQUENCE.//1.0:167:64//AL031687
F-OVARC1001453//Human DNA sequence from PAC 453D15 on chromosome 6 contains STS.//4.4e-64:376:79//Z84482
F-OVARC1001476//Caenorhabditis elegans DNA *** SEQUENCING IN PROGRESS *** from clone Y24F12, WORKING DRAFT SEQUENCE.//0.20:107:71//AL022277
F-OVARC1001480
F-OVARC1001489//Plasmodium falciparum 3D7 chromosome 12 PFYACB8-420 genomic sequence, WORKING DRAFT SEQUENCE, 14 unordered pieces.//0.20:281:63//AC005140
F-OVARC1001496//Homo sapiens C-terminal binding protein 2 mRNA, complete cds.//8.1e-85:479:92//AF016507
F-OVARC1001506//Homo sapiens Chromosome 16 BAC clone CIT987-SKA-13F4 ^{~}complete genomic sequence, complete sequence.//1.2e-98:503:83//AC002039
F-OVARC1001525//Human beta-hexosaminidase alpha chain (HEXA) gene, exon 1.//1.7e-13:87:100//M16411
F-OVARC1001542//H.sapiens polymorphic repeat associated with glutamate dehydrogenase pseudogene 5.//0.43:190:68//X69219
F-OVARC1001547//Plasmodium falciparum 3D7 chromosome 12 PFYACB8-420 genomic sequence, WORKING DRAFT SEQUENCE, 14 unordered pieces.//0.017:533:56//AC005140
F-OVARC1001555//Homo sapiens clone NH0469M07, WORKING DRAFT SEQUENCE, 7 unordered pieces.//7.4e-159:416:99//AC005037
F-OVARC1001577//Homo sapiens SRp46 splicing factor transcribed retropseudogene.//2.4e-115:540:99//AF031165
F-OVARC1001600//Homo sapiens chromosome 21q22.3 PAC 39C17, complete sequence.//5.5e-13:529:62//AF043945
F-OVARC1001610//, complete sequence.//1.4e-12:152:77//AC005409
F-OVARC1001611
F-OVARC1001615//Human DNA sequence from clone 873P14 on chromosome 20p12 Contains STS, GSS, complete sequence.//0.022:146:70//AL031682
F-OVARC1001668//Homo sapiens mRNA for MCM3 import factor, complete cds.//6.5e-109:358:96//AB005543
F-OVARC1001702//Homo sapiens mRNA for hSOX20 protein, complete cds.//1.8e-47:393:81//AB006867
F-OVARC1001703//CIT-HSP-2164L6.TF CIT-HSP Homo sapiens genomic clone 2164L6, genomic survey sequence.//0.94:85:69//B92840
F-OVARC1001711//Human DNA sequence *** SEQUENCING IN PROGRESS *** from clone 317C6, WORKING DRAFT SEQUENCE.//1.9e-06:489:61//Z97651
F-OVARC1001713//Rattus norvegicus neuroligin 2 mRNA, complete cds.//1.0:262:59//U41662
F-OVARC1001726//Human telomere associated repeat sequence, complete sequence.//7.5e-08:283:65//M57752
F-OVARC1001731//Mus musculus gene for beta-tropomyosin.//2.6e-83:606:81//X12650
F-OVARC1001745//HS_3007_B2_G09_T7 CIT Approved Human Genomic Sperm Library D Homo sapiens genomic clone Plate=3007 Col=18 Row=N, genomic survey sequence.//0.00020:269:60//AQ164522
F-OVARC1001762//S.pombe chromosome III cosmid c338.//3.0e-17:624:61//AL023781
F-OVARC1001766//Homo sapiens eukaryotic translation initiation factor eIF3, p35 subunit mRNA, complete cds.//4.2e-149:706:98//U97670
F-OVARC1001767//Homo sapiens mRNA for KIAA0675 protein, complete cds.//3.0e-115:580:96//AB014575
F-OVARC1001768
F-OVARC1001791//Homo sapiens BAC clone RG118P15 from 8q21, complete sequence.//5.7e-64:477:78//AC005066
F-OVARC1001795//Homo sapiens chromosome 4 clone B341C20 map 4q25, complete sequence.//6.5e-11:171:76//AC004704
F-OVARC1001802//CITBI-E1-2502A17.TR CITBI-E1 Homo sapiens genomic clone 2502A17, genomic survey sequence.//0.98:214:61//AQ264481
F-OVARC1001805//Human DNA sequence from clone 511E16 on chromosome 6p24.3-25.1. Contains the last coding exon of the gene for P18 component of aminoacyl-tRNA synthetase complex, part of an unknown gene downstream of a putative CpG island, and an STS with a CA repeat polymorphism, complete sequence.//9.5e-151:712:99//AL023694
F-OVARC1001809//Mus musculus sphingosine kinase (SPHK1a) mRNA, partial cds.//2.7e-56:522:75//AF068748
F-OVARC1001812//Homo sapiens chromosome 17, clone HCIT104N19, complete sequence.//1.7e-63:526:81//AC003662
F-OVARC1001813//Human DNA sequence from cosmid U144A10, between markers DXS366 and DXS87 on chromosome X contains STS.//0.17:214:65//Z70224
F-OVARC1001820//Human DNA sequence *** SEQUENCING IN PROGRESS *** from clone 445N2, WORKING DRAFT SEQUENCE.//3.2e-55:379:82//AL031779
F-OVARC1001828//Homo sapiens chromosome 5, BAC clone 203o13 (LBNL H155), complete sequence.//2.8e-17:509:62//AC005609
F-OVARC1001846//Human DNA sequence from cosmid U73E8, between markers DXS366 and DXS87 on chromosome X.//0.35:403:58//Z73361
F-OVARC1001861//CIT-HSP-2165M3.TR CIT-HSP Homo sapiens genomic clone 2165M3, genomic survey sequence.//2.4e-25:148:96//B94622
F-OVARC1001873//Homo sapiens clones 24718 and 24825 mRNA sequence.//1.2e-18:122:95//AF070611
F-OVARC1001879//HS_3026_B1_F09_T7 CIT Approved Human Genomic Sperm Library D Homo sapiens genomic clone Plate=3026 Col=17 Row=L, genomic survey sequence.//4.9e-29:204:87//AQ207748
F-OVARC1001880//Human interferon regulatory factor 5 (Humirf5) mRNA, complete cds.//3.5e-05:489:60//U51127
F-OVARC1001883//Homo sapiens clone GS259H13, WORKING DRAFT SEQUENCE, 4 unordered pieces.//1.9e-29:350:74//AC005020
F-OVARC1001900//Homo sapiens tumorous imaginal discs protein Tid56 homolog (TID1) mRNA, complete cds.//8.6e-56:300:96//AF061749
F-OVARC1001901//Human DNA sequence from clone 103M22 on chromosome 6p24. Contains STSs and GSSs, complete sequence.//2.3e-10:253:66//AL031904
F-OVARC1001911//HS_2196_B2_H11_MF CIT Approved Human Genomic Sperm Library D Homo sapiens genomic clone Plate=2196 Col=22 Row=P, genomic survey sequence.//3.4e-09:123:78//AQ294069
F-OVARC1001916//HS_3054_B1_C11_MF CIT Approved Human Genomic Sperm Library D Homo sapiens genomic clone Plate=3054 Col=21 Row=F, genomic survey sequence.//1.2e-31:126:97//AQ099979
F-OVARC1001928
F-OVARC1001942//H.sapiens CpG island DNA genomic Mse1 fragment, clone 21d7, forward read cpg21d7.ft1a.//7.2e-12:83:98//Z60390
F-OVARC1001943//Aplysia californica potassium channel modulatory factor mRNA, complete cds.//3.5e-50:535:69//AF059179
F-OVARC1001949//Human KRAB zinc finger protein (ZNF177) mRNA, complete cds.//1.7e-16:294:67//U37263
F-OVARC1001950//Homo sapiens *** SEQUENCING IN PROGRESS ***, WORKING DRAFT SEQUENCE.//1.5e-20:261:68//AJ011929
F-OVARC1001987//D.melanogaster G6PD gene, exons 2-4.//0.99:447:57//Z19021
F-OVARC1001989//Homo sapiens clone DJ0042M02, WORKING DRAFT SEQUENCE, 20 unordered pieces.//2.9e-19:178:83//AC005995
F-OVARC1002044//Plasmodium falciparum MAL3P7, complete sequence.//0.17:232:62//AL034559
F-OVARC1002050//Homo sapiens mRNA for KIAA0465 protein, partial cds.//2.1e-158:739:98//AB007934
F-OVARC1002066//Homo sapiens genomic DNA, chromosome 21q22.2 (Down Syndrome region), segment 4/15, WORKING DRAFT SEQUENCE.//3.0e-17:781:59//AP000011
F-OVARC1002082//Homo sapiens clone DJ0965K10, WORKING DRAFT SEQUENCE, 6 unordered pieces.//5.4e-136:683:96//AC006015
F-OVARC1002107//Homo sapiens BAC clone RG276003 from 7q22-q31.1, complete sequence.//1.0:220:61//AC004668
F-OVARC1002112//Homo sapiens histone macroH2A1.2 mRNA, complete cds.//6.1e-115:557:98//AF041483
F-OVARC1002127//Homo sapiens chromosome 9, clone hRPK.202_H_3, complete sequence.//0.013:461:57//AC006241
F-OVARC1002l38//Caenorhabditis elegans cosmid F32D1.//1.0e-29:545:64//AF016427
F-OVARC1002143//CIT-HSP-2343H20.TR CIT-HSP Homo sapiens genomic clone 2343H20, genomic survey sequence.//2.3e-11:258:67//AQ055576
F-OVARC1002156
F-OVARC1002158//F17O7-T7 IGF Arabidopsis thaliana genomic clone F17O7, genomic survey sequence.//1.8e-16:383:66//B11616
F-OVARC1002165//H.sapiens BDP1 mRNA for protein-tyrosinephosphatase.//0.0041:300:64//X79568
F-OVARC1002182//F.rubripes GSS sequence, clone 123I23aA7, genomic survey sequence.//1.4e-10:240:66//AL017241
F-PLACE1000004//CIT-HSP-2294H13.TF CIT-HSP Homo sapiens genomic clone 2294H13, genomic survey sequence.//8.2e-10:158:75//AQ003859
F-PLACE1000005//Mouse alpha-1 antitrypsin gene, segment 1.//4.8e-15:89:93//M12585
F-PLACE1000007//Homo sapiens ubiquitin hydrolyzing enzyme I (UBH1) mRNA, partial cds.//3.8e-51:550:72//AF022789
F-PLACE1000014
F-PLACE1000031//Homo sapiens alone DJ0098O22, WORKING DRAFT SEQUENCE, 5 unordered pieces.//0.91:333:61//AC004821
F-PLACE1000040//Homo sapiens DNA sequence from PAC 958B3 on chromosome Xp22.11-Xp22.22. Contains ESTs STS and CpG island.//2.6e-20:279:67//Z93023
F-PLACE1000048//Homo sapiens chromosome 17, clone HCIT462L7, complete sequence.//3.6e-63:488:82//AC005177
F-PLACE1000050//Mus musculus chromosome 14 marker um-m24 GA dinucleotide DNA sequence.//2.3e-10:141:75//U31508
F-PLACE1000061//Human ribosomal protein L37a mRNA sequence.//1.9e-30:190:94//L22154
F-PLACE1000066//Homo sapiens PAC clone DJ1106E03 from 7q31.3-7q3, complete sequence.//6.0e-63:597:74//AC005521
F-PLACE1000078//Homo sapiens chromosome 11 clone CIT987SK-1012F4, WORKING DRAFT SEQUENCE, 6 unordered pieces.//5.2e-09:143:73//AC005848
F-PLACE1000081//Human DNA from chromosome 19 specific cosmid R28461, genomic sequence, complete sequence.//0.52:390:60//AC002389
F-PLACE1000094
F-PLACE1000133//Human DNA sequence from clone 372K1 on chromosome 6q24 Contains EST, STS, GSS and CpG Island, complete sequence.//4.4e-129:731:92//AL023580
F-PLACE1000142//H.sapiens AUH mRNA.//6.4e-09:328:62//X79888
F-PLACE1000184//Homo sapiens estrogen-related receptor gamma mRNA, complete cds.//7.7e-150:737:97//AF058291
F-PLACE1000185//Sequence 15 from patent US 5691147.//5.7e-106:558:94//I76211 F-PLACE1000213
F-PLACE1000214//Plasmodium falciparum 3D7 chromosome 12 PFYAC1383 genomic sequence, WORKING DRAFT SEQUENCE, 3 unordered pieces.//1.8e-06:644:57//AC005504
F-PLACE1000236//Human DNA sequence *** SEQUENCING IN PROGRESS *** from clone 695O20, WORKING DRAFT SEQUENCE.//2.6e-39:191:83//AL032818
F-PLACE1000246//HS_2008_A2_D04_T7 CIT Approved Human Genomic Sperm Library D Homo sapiens genomic clone Plate=2008 Col=8 Row=G, genomic survey sequence.//0.96:153:61//AQ269813
F-PLACE1000292//Drosophila melanogaster Oregon-R mitochondrial A+T region.//5.1e-12:571:60//U11584
F-PLACE1000308//D.teissieri mitochondrial DNA for tRNA-fmet, tRNA-Ile, tRNA-Gln & amp; tRNA-Val.//0.00013:369:59//X54011
F-PLACE1000332//HS_2016_B2_D08_T7 CIT Approved Human Genomic Sperm Library D Homo sapiens genomic clone Plate=2016 Col=16 Row=H, genomic survey sequence.//7.5e-83:424:96//AQ232106
F-PLACE1000347//CIT-HSP-2326A16.TV CIT-HSP Homo sapiens genomic clone 2326A16, genomic survey sequence.//0.13:46:100//AQ047350
F-PLACE1000374//Mus musculus putative CCAAT binding factor 1 (mCBF) mRNA, alternatively spliced transcript mCBF1, complete cds.//0.00048:84:83//U19891
F-PLACE1000380//F.rubripes GSS sequence, clone 047P21aA10, genomic survey sequence.//0.43:198:62//Z88163
F-PLACE1000383//Homo sapiens myotubularin related protein 1 (MTMR1) mRNA, partial cds.//8.7e-149:740:96//U58032
F-PLACE1000401//Pinctada fucata mRNA for insoluble protein, complete cds.//0.22:484:56//D86074
F-PLACE1000406//Human nuclear matrix protein 55 (nmt55) mRNA, complete cds.//3.3e-19:372:65//U89867
F-PLACE1000420//Homo sapiens chromosome 17, clone hRPK.227_G_15, complete sequence.//1.6e-85:421:87//AC005899
F-PLACE1000421//Human GT334 protein (GT334) gene, exons 16 and 17.//0.88:145:68//U61515
F-PLACE1000424//Homo sapiens PAC clone DJ0905J08 from 7p12-p14, complete sequence.//0.076:196:66//AC005189
F-PLACE1000435//HS_3217_A2_A12_MR CIT Approved Human Genomic-Sperm Library D Homo sapiens genomic clone Plate=3217 Col=24 Row=A, genomic survey sequence.//2.2e-47:438:76//AQ181698
F-PLACE1000444//Homo sapiens Chromosome 16 BAC clone CIT987SK-A-152E5, complete sequence.//6.9e-61:616:71//AC004382
F-PLACE1000453//Murine genomic DNA; partially digested Sau3A fragment, cloned into cosmid vector pEMBLcos2, complete sequence.//5.8e-18:314:69//AF059580
F-PLACE1000481//Homo sapiens Chromosome 22q11.2 Cosmid Clone 94a In DGCR Region, complete sequence.//1.1e-33:349:76//AC002491
F-PLACE1000492//Rat vacuolar protein sorting homolog r-vps33b mRNA, complete cds.//1.1e-34:256:83//U35245
F-PLACE1000540//P.falciparum complete gene map of plastid-like DNA (IR-B).//0.099:336:58//X95276
F-PLACE1000547//Arabidopsis thaliana GDP-mannose pyrophosphorylase (GMP1) mRNA, complete cds.//5.4e-11:279:63//AF076484
F-PLACE1000562//, complete sequence.//1.7e-97:559:88//AC005409
F-PLACE1000564
F-PLACE1000583//Figure 2. Nucleotide and translated protein sequences of HPF1, -2, and-9.//3.3e-46:631:68//M27877
F-PLACE1000588//Human guanylate binding protein isoform I (GBP-2) mRNA, complete cds.//7.3e-84:503:88//M55542
F-PLACE1000596//Homo sapiens mRNA for NS1-binding protein (NS1-BP).//3.8e-164:798:97//AJ012449
F-PLACE1000599//P.falciparum complete gene map of plastid-like DNA (IR-B).//0.018:295:61//X95276
F-PLACE1000610//HS_2056_A1_D10_T7 CIT Approved Human Genomic Sperm Library D Homo sapiens genomic clone Plate=2056 Col=19 Row=G, genomic survey sequence.//5.3e-24:188:87//AQ235967
F-PLACE1000611//Rattus norvegicus neural membrane protein 35 mRNA, complete cds.//2.4e-47:687:66//AF044201
F-PLACE1000636
F-PLACE1000653//Homo sapiens N-acetylglucosamine-phosphate mutase mRNA, complete cds.//1.5e-152:747:96//AF102265
F-PLACE1000656//Homo sapiens mRNA for JM4 protein, complete CDS (clone IMAGE 546750 and LLNLc110F1857Q7 (RZPD Berlin)).//2.3e-156:775:97//AJ005896
F-PLACE1000706//nuclear protein TIF1 [mice, mRNA, 3951 nt].//8.0e-60:675:70//S78219
F-PLACE1000712
F-PLACE1800716//HS-1057-A1-A03-MR.abi CIT Human Genomic Sperm Library C Homo sapiens genomic clone Plate=CT 779 Col=5 Row=A, genomic survey sequence.//2.7e-42:266:82//B43026
F-PLACE1000748//CIT-HSP-2372J8.TR CIT-HSP Homo sapiens genomic clone 2372J8, genomic survey sequence.//0.023:157:68//AQ113109
F-PLACE1000749//Plasmodium falciparum MAL3P7, complete sequence.//0.099:664:57//AL034559
F-PLACE1000755//H.sapiens DNA 3' flanking simple sequence region clone wg2c3.//0.00068:206:62//X76589
F-PLACE1000769//RPCI11-3J18.TPB RPCI-11 Homo sapiens genomic clone RPCI-11-3J18, genomic survey sequence.//6.5e-08:93:89//B63806
F-PLACE1000785//Homo sapiens mRNA for KIAA0648 protein, partial cds.//3.5e-138:663:98//AB014548
F-PLACE1000786//Drosophila melanogaster cosmid 80H7.//1.4e-43:589:68//AL031027
F-PLACE1000793//H.sapiens CpG island DNA genomic Mse1 fragment, clone 13d12, reverse read cpg13d12.rt1c.//4.6e-09:71:100//Z64565
F-PLACE1000798//Human Chromosome 16 BAC clone CIT987SK-A-635H12, complete sequence.//5.0e-14:235:72//AC002310
F-PLACE1000841//Homo sapiens clone NH0441G08, WORKING DRAFT SEQUENCE, 12 unordered pieces.//0.013:404:60//AC006158
F-PLACE1000849//H.sapiens CpG island DNA genomic Mse1 fragment, clone 72a10, reverse read cpg72a10.rt1a.//3.3e-09:82:92//Z62712
F-PLACE1000856//Hydra vulgaris HT4 mRNA for collagen-like protein, partial cds.//1.0:317:59//AB008935
F-PLACE1000863//H.sapiens CpG island DNA genomic Mse1 fragment, clone 53d2, forward read cpg53d2.ft1b.//7.3e-37:199:98//Z55621
F-PLACE1000909//H.sapiens CpG island DNA genomic Mse1 fragment, clone 173f8, reverse read cpg173f8.rt1a.//1.5e-17:128:92//Z57391
F-PLACE1000931//Human DNA sequence from PAC 212P9 on chromosome 1p34.1-1p35. Contains delta opiate receptor, CpG island, CA repeat,.//8.1e-55:647:72//AL009181
F-PLACE1000948
F-PLACE1000972//RPCI11-61B1.TJ RPCI11 Homo sapiens genomic clone R-61B1, genomic survey sequence.//1.0e-26:148:99//AQ194348
F-PLACE1000977//Homo sapiens mRNA for KIAA0672 protein, complete cds.//6.1e-08:413:61//AB014572
F-PLACE1000979//H.sapiens CpG island DNA genomic Mse1 fragment, clone 76e8, reverse read cpg76e8.rt1a.//2.7e-10:84:94//Z55963
F-PLACE1000987//Homo sapiens mRNA for KIAA0724 protein, complete cds.//8.0e-140:694:96//AB018267
F-PLACE1001000//Herpetomonas muscarum muscarum kinetoplast 12S rRNA gene.//0.0056:443:58//U01011
F-PLACE1001007//CIT-HSP-2013L15.TF CIT-HSP Homo sapiens genomic clone 2013L15, genomic survey sequence.//0.99:277:58//B58681
F-PLACE1001010//Human cosmid g1572c101, complete sequence.//3.6e-55:294:88//AC000357
F-PLACE1001015//Homo sapiens PAC clone DJ0754J18 from 7p21, complete sequence.//7.2e-33:333:73//AC004741
F-PLACE1001024
F-PLACE1001036//CIT-HSP-2373I10.TF CIT-HSP Homo sapiens genomic clone 2373I10, genomic survey sequence.//1.1e-80:393:98//AQ108662
F-PLACE1001054//Arabidopsis thaliana genomic DNA, chromosome 5, TAC clone: K9I9, complete sequence.//8.8e-40:483:66//AB013390
F-PLACE1001062//Mus musculus mRNA encoding lysine-ketoglutarate reductase/saccharopine dehydrogenase.//1.2e-23:224:80//AJ224761
F-PLACE1001076//HS_2195_B1_D05_MF CIT Approved Human Genomic Sperm Library D Homo sapiens genomic clone Plate=2195 Col=9 Row=H, genomic survey sequence.//0.0014:168:66//AQ066659
F-PLACE1001088
F-PLACE1001092//Homo sapiens sorting nexin 4 mRNA, complete cds.//3.1e-95:489:96//AF065485
F-PLACE1001104//Caprine arthritis-encephalitis virus envelope glycoprotein (env) gene, partial cds.//0.0073:253:62//U81400
F-PLACE1001118//Homo sapiens KRAB domain zinc finger protein (ZFP37) mRNA, complete cds.//2.5e-64:676:71//AF022158
F-PLACE1001136//Human amphiregulin (AR) gene, exon 5, clones lambda-ARH(6,12).//3.8e-26:174:93//M30702
F-PLACE1001168
F-PLACE1001171//Homo sapiens subtelomeric cosmid 11b-1, complete sequence.//7.6e-23:245:68//AC005603
F-PLACE1001185//Human DNA sequence from clone 889N15 on chromosome Xq22.1-22.3. Contains part of the gene for a novel protein similar to X. laevis Cortical Thymocyte Marker-CTX, the possibly alternatively spliced gene for 26S Proteasome subunit p28 (Ankyrin repeat protein), a novel gene and exons 36 through 45 of the COL4A6 for Collagen Alpha 6(IV). Contains ESTs, STSs, GSSs and a putative CpG island, complete sequence.//0.010:102:70//AL031177
F-PLACE1001238//Mouse mRNA for RNA polymerase I associated factor (PAF53), complete cds.//9.3e-82:684:77//D14336
F-PLACE1001241
F-PLACE1001257//Caenorhabditis elegans cosmid R12E2.//1.1e-16:480:60//AF067219
F-PLACE1001272//H.sapiens subunit of coatomer complex.//0.31:50:96//X70476
F-PLACE1001279//Plasmodium falciparum 3D7 chromosome 12 PFYACB8-628 genomic sequence, WORKING DRAFT SEQUENCE, 9 unordered pieces.//0.054:352:60//AC005507
F-PLACE1001280//Bovine herpesvirus type 1 early-intermediate transcription control protein (BICP4) gene, complete cds.//1.0e-10:620:61//L14320
F-PLACE1001294//M.musculus GEG-154 mRNA.//5.0e-107:826:80//X71642
F-PLACE1001304//Mouse Zfp-35 mRNA for zinc finger protein ZFP-35.//1.2e-67:510:77//X17617
F-PLACE1001311//Homo sapiens clone DJ0826E18, WORKING DRAFT SEQUENCE, 4 unordered pieces.//1.6e-47:491:73//AC005282
F-PLACE1001323//HS-1007-A2-B10-MF.abi CIT Human Genomic Sperm Library C Homo sapiens genomic clone Plate=CT 328 Col=20 Row=C, genomic survey sequence.//9.6e-26:142:100//B31181
F-PLACE1001351
F-PLACE1001366//Homo sapiens mRNA for KIAA0799 protein, partial cds.//8.6e-25:155:95//AB018342
F-PLACE1001377//H.sapiens MADM gene (exon 1).//1.6e-43:393:79//Z48614
F-PLACE1001383//Human DNA sequence from clone 246H3 on chromosome 22q11.21-12.2 Contains LRP5 (Lipoprotein Receptor Related Protein) pseudogene, EST, CA repeats (D22S414, D22S925, D22S926), STS, GSS and CpG island, complete sequence.//1.5e-119:705:91//AL022324
F-PLACE1001384//Homo sapiens mRNA for multi PDZ domain protein.//5.7e-08:117:84//AJ001319
F-PLACE1001387//Sequence 3 from patent US 5610018.//1.7e-06:395:58//I57340
F-PLACE1001395//Plasmodium falciparum circular DNA rpoB and rpoC genes for beta and beta-prime subunits of RNA polymerase (EC 2.7.7.6).//7.2e-11:620:60//X52177
F-PLACE1001399//Homo sapiens chromosome 17, clone hRPK.22_N_12, WORKING DRAFT SEQUENCE, 2 ordered pieces.//3.0e-145:700:98//AC005412
F-PLACE1001412//Homo sapiens clone 643 unknown mRNA, complete sequence.//2.0e-69:365:96//AF091087
F-PLACE1001414//Homo sapiens chromosome 9, clone hRPK.202_H_3, complete sequence.//8.2e-121:608:97//AC006241
F-PLACE1001440//Human Chromosome 11 pac pDJ393o15, WORKING DRAFT SEQUENCE, 8 unordered pieces.//1.3e-06:437:61//AC000384
F-PLACE1001456//Homo sapiens Xp22 GS-524I1 (Genome Systems Human BAC library), complete sequence.//0.98:348:60//AC003106
F-PLACE1001468//Homo sapiens DNA sequence from PAC 435A7 on chromosome Xq22.1-q22.3. Contains STS.//4.4e-05:358:62//AL022148
F-PLACE1001484//Human DNA sequence *** SEQUENCING IN PROGRESS *** from clone 387E22, WORKING DRAFT SEQUENCE.//5.7e-31:195:93//AL031660
F-PLACE1001502//Human fibroblast growth factor receptor 3 (FGFR3) gene, exon L//0.00015:333:59//L78720
F-PLACE1001503//Drosophila melanogaster DNA sequence (P1 DS05273 (D80)), complete sequence.//0.00016:161:66//AC004373
F-PLACE1001517//Human DNA sequence from PAC 696H22 on chromosome Xq21.1-21.2. Contains a mouse E25 like gene, a Kinesin like pseudogene and ESTs.//3.7e-22:260:76//AL021786
F-PLACE1001534//Human DNA sequence *** SEQUENCING IN PROGRESS *** from clone 620E11, WORKING DRAFT SEQUENCE.//1.1e-143:713:97//AL031667
F-PLACE1001545//Homo sapiens chromosome 3, clone hRPK.165_I_16, complete sequence.//2.7e-139:482:96//AC005669
F-PLACE1001551//Homo sapiens chromosome 19, CIT-HSP-444n24, complete sequence.//6.9e-116:681:89//AC005261
F-PLACE1001570//HS_3105_A1_F06_MR CIT Approved Human Genomic Sperm Library D Homo sapiens genomic clone Plate=3105 Col=11 Row=K, genomic survey sequence.//1.2e-10:137:79//AQ139817
F-PLACE1001602//Homo sapiens genomic DNA of 8p21.3-p22 anti-oncogene of hepatocellular colorectal and non-small cell lung cancer , segment 3/11.//1.8e-102:217:99//AB020860
F-PLACE1001603//Homo sapiens nitrilase homolog 1 (NIT1) gene, alternatively spliced product, complete cds.//3.7e-104:501:98//AF069984
F-PLACE1001608//HS_2189_A1_G07_MR CIT Approved Human Genomic Sperm Library D Homo sapiens genomic clone Plate=2189 Col=13 Row=M, genomic survey sequence.//2.9e-60:429:84//AQ221959
F-PLACE1001610//Homo sapiens clone NH0469M07, WORKING DRAFT SEQUENCE, 7 unordered pieces.//4.4e-114:552:98//AC005037
F-PLACE1001611//Human DNA sequence from clone 1039K5 on chromosome 22q12.3-13.2 Contains gene similar to PICK1 perinuclear binding protein, gene similar to monocarboxylate transporter (MCT3), ESTs, STS, GSS and a CpG island, complete sequence.//0.93:131:71//AL031587
F-PLACE1001632//Homo sapiens mRNA for KIAA0798 protein, complete cds.//1.1e-74:702:75//AB018341
F-PLACE1001634//Human p190-B (p190-B) mRNA, complete cds.//1.2e-18:114:100//U17032
F-PLACE1001640//Homo sapiens chromosome 17, clone hRPK.651_L_9, complete sequence.//7.7e-159:788:97//AC005971
F-PLACE1001672//Human DNA sequence from clone 71L16 on chromosome Xp11. Contains a probable Zinc Finger protein (pseudo)gene, an unknown putative gene, a pseudogene with high similarity to part of antigen KI-67, a putative Chondroitin 6-Sulfotransferase LIKE gene and a KIAA0267 LIKE putative Na(+)/H(+) exchanger protein gene. Contains a predicted CpG island, ESTs, STSs and GSSs and genomic markers DXS1003 and DXS1055, complete sequence.//7.8e-36:365:73//AL022165
F-PLACE1001691//Homo sapiens chromosome 17, clone hRPK.294_J_22, complete sequence.//9.1e-149:760:96//AC005921
F-PLACE1001692//Rat medium-chain S-acyl fatty acid synthetase thio ester hydrolase (MCH), complete cds.//2.9e-57:643:71//M16200
F-PLACE1001705//Homo sapiens chromosome 17, clone hRPK.958_E_11, WORKING DRAFT SEQUENCE, 2 ordered pieces.//3.9e-18:284:71//AC005883
F-PLACE1001716//Human mRNA for KIAA0191 gene, partial cds.//6.6e-68:369:73//D83776
F-PLACE1001720//Homo sapiens Chromosome 22q11.2 Cosmid Clone 31f3 In IGLC Region, complete sequence.//1.0:274:59//AC000051
F-PLACE1001729//Streptomyces coelicolor cosmid 1C2.//0.22:433:57//AL031124
F-PLACE1001739//Caenorhabditis elegans cosmid C18H7.//0.049:341:61//AF067607
F-PLACE1001740//Homo sapiens chromosome 5, P1 clone 1108H7 (LBNL H81), complete sequence.//4.8e-26:372:68//AC005221
F-PLACE1001745
F-PLACE1001746//Plasmodium falciparum DNA *** SEQUENCING IN PROGRESS *** from MAL1P1, WORKING DRAFT SEQUENCE.//0.018:472:57//AL031744
F-PLACE1001748//Homo sapiens metalloprotease 1 (MP1) mRNA, complete cds.//8.8e-159:773:97//AF061243
F-PLACE1001756//Homo sapiens chromosome 12p13.3 clone RPCI11-303E5, WORKING DRAFT SEQUENCE, 65 unordered pieces.//1.9e-54:274:81//AC005842
F-PLACE1001761//HS_3027_A1_D02_MR CIT Approved Human Genomic Sperm Library D Homo sapiens genomic clone Plate=3027 Col=3 Row=G, genomic survey sequence.//0.095:49:93//AQ130972
F-PLACE1001771//Homo sapiens transient receptor potential protein 6 mRNA, complete cds.//1.0e-146:709:97//AF080394
F-PLACE1001781 1.3e-08:238:65//AC005637
F-PLACE1001799//HS_3075_B1_H03_MR CIT Approved Human Genomic Sperm Library D Homo sapiens genomic clone Plate=3075 Col=5 Row=P, genomic survey sequence.//1.7e-09:166:69//AQ138474
F-PLACE1001810//Arabidopsis thaliana genomic DNA, chromosome 3, P1 clone: MRC8, complete sequence.//0.00035:196:66//AB020749
F-PLACE1001817//Homo sapiens ATP-specific succinyl-CoA synthetase beta subunit (SCS) mRNA, partial cds.//1.1e-108:546:96//AF058953
F-PLACE1001821//RPCI11-35D17.TK RPCI-11 Homo sapiens genomic clone RPCI-11-35D17, genomic survey sequence.//2.1e-55:300:97//AQ045286
F-PLACE1001844//Homo sapiens chromosome 17, clone HCIT462L7, complete sequence.//2.8e-67:443:86//AC005177
F-PLACE1001845//Arabidopsis thaliana chromosome I BAC T25B24 genomic sequence, complete sequence.//0.34:219:64//AC005850
F-PLACE1001869//Klebsiella pneumoniae ribitol kinase (rbtK) and ribitol transporter (rbtT) genes, complete cds.//7.1e-11:505:57//AF045244
F-PLACE1001897//RPCI11-46D15.TJ RPCI11 Homo sapiens genomic clone R-46D15, genomic survey sequence.//9.3e-08:383:63//AQ194408
F-PLACE1001912
F-PLACE1001920//Homo sapiens MDC-3.13 isoform 2 mRNA, complete cds.//7.3e-156:753:98//AF099935
F-PLACE1001928//HS_2220_B2_G04_MR CIT Approved Human Genomic Sperm Library D Homo sapiens genomic clone Plate=2220 Col=8 Row=N, genomic survey sequence.//2.8e-43:233:97//AQ152361
F-PLACE1001983//Human DNA sequence *** SEQUENCING IN PROGRESS *** from clone 745C22, WORKING DRAFT SEQUENCE.//1.6e-07:396:62//AL031596
F-PLACE1001989//Human DNA sequence *** SEQUENCING IN PROGRESS *** from clone 968D22, WORKING DRAFT SEQUENCE.//1.0e-109:602:93//AL023755
F-PLACE1002004//Human DNA sequence *** SEQUENCING IN PROGRESS *** from clone 317E23, WORKING DRAFT SEQUENCE.//1.0e-69:475:87//AL020996
F-PLACE1002046//Mus musculus ligatin (Lgtn) mRNA, partial cds.//7.2e-97:623:85//U58337
F-PLACE1002052//HS_2178_B2_D05_MR CIT Approved Human Genomic Sperm Library D Homo sapiens genomic clone Plate=2178 Col=10 Row=H, genomic survey sequence.//4.8e-22:140:95//AQ307908
F-PLACE1002066//Apis mellifera NADH dehydrogenase subunit 2 (ND2) gene, mitochondrial gene encoding mitochondrial protein, partial cds.//0.0063:371:60//U72284
F-PLACE1002072//Homo sapiens tight junction protein ZO (ZO-2) gene, alternative splice products, promoter and exon A.//0.97:248:60//AF043195
F-PLACE1002073//Homo sapiens mRNA for KIAA0606 protein, partial cds.//1.3e-37:635:64//AB011178
F-PLACE1002090//Homo sapiens full-length insert cDNA clone ZA85C09.//7.0e-122:583:98//AF086131
F-PLACE1002115//nbxb0038A20r CUGI Rice BAC Library Oryza sativa genomic clone nbxb0038A20r, genomic survey sequence.//0.039:210:69//AQ291086
F-PLACE1002119//Mus musculus IER5 (Ier5) mRNA, complete cds.//7.1e-61:540:77//AF079527
F-PLACE1002140//Homo sapiens DNA sequence from PAC 454M7 on chromosome Xq25-26.3. Contains the OCRL1 gene for Lowe Oculocerebrorenal Syndrome protein OCRL-1. Contains ESTs, STSs and GSSs, complete sequence.//2.1e-125:491:98//AL022162
F-PLACE1002150//Plasmodium falciparum MAL3P5, complete sequence.//0.12:408:61//AL034556
F-PLACE1002157//Homo sapiens BAC clone NH0335J18 from 2, complete sequence.//1.1e-44:515:71//AC005539
F-PLACE1002163//Homo sapiens T-cell receptor alpha delta locus from bases 1000498 to 1071650 (section 5 of 5) of the Complete Nucleotide Sequence.//0.98:210:65//AE000662
F-PLACE1002170//Homo sapiens Xp22 bins 16-17 BAC GSHB-531I17 (Genome Systems Human BAC Library) complete sequence.//1.2e-06:283:60//AC004805
F-PLACE1002171//Mus musculus interferon alpha/beta receptor (IFNAR) gene, exon 11 and partial cds.//1.0e-24:313:71//U06244
F-PLACE1002205//Drosophila melanogaster; Chromosome 3L; Region 79F1-80A2; BAC clone BACR48E05, WORKING DRAFT SEQUENCE, 4 unordered pieces.//1.6e-05:428:60//AC005720
F-PLACE1002213//HS_3238_B1_G03_MR CIT Approved Human Genomic Sperm Library D Homo sapiens genomic clone Plate=3238 Col=5 Row=N, genomic survey sequence.//2.2e-74:371:98//AQ206965
F-PLACE1002227//HS-1056-B1-C01-MF.abi CIT Human Genomic Sperm Library C Homo sapiens genomic clone Plate=CT 778 Col=1 Row=F, genomic survey sequence.//2.1e-07:174:71//B42800
F-PLACE1002256//Plasmodium falciparum DNA *** SEQUENCING IN PROGRESS *** from contig 3-72, complete sequence.//0.022:458:59//AL010142
F-PLACE1002259//Human DNA sequence from clone 246H3 on chromosome 22q11.21-12.2 Contains LRP5 (Lipoprotein Receptor Related Protein) pseudogene, EST, CA repeats (D22S414, D22S925, D22S926), STS, GSS and CpG island, complete sequence.//3.5e-91:637:84//AL022324
F-PLACE1002319
F-PLACE1002342//Caenorhabditis elegans cosmid M03A1.//0.47:403:58//U49956
F-PLACE1002395//Homo sapiens chromosome 19, cosmid R28991, complete sequence.//1.9e-127:487:93//AC004623
F-PLACE1002399//Homo sapiens chromosome 17, clone hRPK.235_I_10, complete sequence.//5.6e-05:474:59//AC005922
F-PLACE1002433//Drosophila melanogaster fidipidine gene, exons 1-7.//1.7e-11:613:58//AJ011928
F-PLACE1002437//M.musculus abc1 mRNA.//5.5e-62:452:85//X75926
F-PLACE1002438//Dictyostelium discoideum developmental protein DG1098 (DG1098) gene, partial cds.//0.013:372:59//AF081801
F-PLACE1002450//HS_3233_A1_G01_MR CIT Approved Human Genomic Sperm Library D Homo sapiens genomic clone Plate=3233 Col=1 Row=M, genomic survey sequence.//3.1e-07:449:59//AQ204769
F-PLACE1002465
F-PLACE1002474//Mus musculus matrilin-2 precursor mRNA, complete cds.//1.5e-110:720:85//U69262
F-PLACE1002477//Homo sapiens Xp22-171-173 BAC GSHB-312I4 (Genome Systems Human BAC Library) complete sequence.//3.9e-05:195:71//AC005926
F-PLACE1002493//Homo sapiens 3p22-8 PAC RPCI4-736H12 (Roswell Park Cancer Institute Human PAC Library) complete sequence.//0.020:301:60//AC006060
F-PLACE1002499
F-PLACE1002500//Rattus norvegicus zinc transporter (ZnT-2) mRNA, complete cds.//2.1e-58:465:80//U50927
F-PLACE1002514//Human DNA sequence *** SEQUENCING IN PROGRESS *** from clone 292E10, WORKING DRAFT SEQUENCE.//3.7e-08:139:76//Z93930
F-PLACE1002529//Homo sapiens mRNA for KIAA0713 protein, partial cds.//9.0e-143:583:95//AB018256
F-PLACE1002532//Homo sapiens BAC clone RG300E22 from 7q21-q31.1, complete sequence.//0.00019:193:65//AC004774
F-PLACE1002537//Human DNA sequence from clone 127F18 on chromosome Xp11.4-21.3. Contains part of a novel gene with some similarity to parts of chicken Myosin Light Chain and various species' Interleukin-1 Receptor Type 1 (IL1-R-1). Contains GSSs, complete sequence.//4.7e-25:198:84//AL031575
F-PLACE1002571//Drosophila melanogaster actin-related protein mRNA, complete cds.//2.0e-13:400:60//L25314
F-PLACE1002578//Homo sapiens Xq28 BACs 360 F12, GSHB-555C13, complete sequence.//3.5e-11:167:72//AC002523
F-PLACE1002583//Mus musculus glutamate receptor subunit (GluR6) gene, partial cds.//4.2e-09:370:61//U31443
F-PLACE1002591//H.sapiens mRNA for coronin.//7.2e-26:279:74//X89109
F-PLACE1002598//Homo sapiens clone GS308H05, WORKING DRAFT SEQUENCE, 6 unordered pieces.//0.0013:375:64//AC005537
F-PLACE1002604//Hansenula wingei mitochondrial DNA, complete sequence.//4.7e-05:556:59//D31785
F-PLACE1002625
F-PLACE1002655//Homo sapiens PAC clone DJ0722F20 from 7q31.1-q31.3, complete sequence.//1.6e-128:229:92//AC005281
F-PLACE1002665//Mus musculus enhancer of polycomb (Epc1) mRNA, complete cds.//3.6e-107:706:84//AF079765
F-PLACE1002685//Homo sapiens B cell linker protein BLNK mRNA, alternatively spliced, complete cds.//3.4e-186:804:97//AF068180
F-PLACE1002714//Mus musculus cathepsin S (CatS) gene, promoter region and exons 1 and 2.//2.3e-16:474:64//AF051726
F-PLACE1002722//Sequence 1 from patent US 5686597.//1.7e-107:552:95//I73723
F-PLACE1002768//Human DNA sequence from clone 726F20 on chromosome 1p36.11-36.23. Contains ESTs and a GSS, complete sequence.//0.0076:161:70//AL031273
F-PLACE1002772//HS_3058_A1_D02_MR CIT Approved Human Genomic Sperm Library D Homo sapiens genomic clone Plate=3058 Col=3 Row=G, genomic survey sequence.//0.0046:192:64//AQ134567
F-PLACE1002775//Mus musculus bromodomain-containing protein BP75 mRNA, complete cds.//7.6e-14:459:62//AF084259
F-PLACE1002782//Rattus norvegicus zinc transporter (ZnT-2) mRNA, complete cds.//3.6e-43:385:77//U50927
F-PLACE1002794//CIT-HSP-2368A17.TR CIT-HSP Homo sapiens genomic clone 2368A17, genomic survey sequence.//1.3e-71:368:96//AQ075879
F-PLACE1002811//Human mRNA for KIAA0172 gene, partial cds.//1.8e-44:567:70//D79994
F-PLACE1002815//Sequence 25 from patent US 5747660.//2.6e-07:150:73//AR005295
F-PLACE1002816//Homo sapiens antigen NY-CO-9 (NY-CO-9) mRNA, partial cds.//1.3e-68:687:73//AF039691
F-PLACE1002834//Figure 2. Nucleotide and translated protein sequences of HPF1, -2, and 9.//9.3e-41:240:93//M27877
F-PLACE1002839//Human BAC clone RG205G13 from 7q31, complete sequence.//0.00087:213:63//AC003045
F-PLACE1002851//Plasmodium falciparum 3D7 chromosome 12 PFYACB8-420 genomic sequence, WORKING DRAFT SEQUENCE, 14 unordered pieces.//0.0032:269:66//AC005140
F-PLACE1002853//Leishmania tarentolae kinetoplast pre-edited mitochondrial maxicircle DNA complete transcribed region and flanks.//0.032:275:62//M10126
F-PLACE1002881//Human DNA sequence *** SEQUENCING IN PROGRESS *** from clone 111B22, WORKING DRAFT SEQUENCE.//4.7e-38:355:76//Z98200
F-PLACE1002908//Gallus gallus beta-1,4-galactosyltransferase (CKII) mRNA, complete cds.//0.00012:200:64//U19889
F-PLACE1002941//Human BAC clone RG161K23 from 7q21, complete sequence.//1.1e-14:241:70//AC000120
F-PLACE1002962
F-PLACE1002968//Plasmodium falciparum MAL3P2, complete sequence.//0.21:410:59//AL034558
F-PLACE1002991//Human DNA sequence *** SEQUENCING IN PROGRESS *** from clone 968D22, WORKING DRAFT SEQUENCE.//6.8e-121:605 :93//AL023755
F-PLACE1002993//CIT-HSP-2338I16.TF CIT-HSP Homo sapiens genomic clone 2338I16, genomic survey sequence.//1.9e-13:100:95//AQ054760
F-PLACE1002996//Mouse U6 RNA gene.//2.0e-13:113:90//X06980
F-PLACE1003025//Plasmodium falciparum MAL3P6, complete sequence.//0.84:374:58//Z98551
F-PLACE1003027//Homo sapiens mRNA for KIAA0516 protein, partial cds.//6.1e-130:632:97//AB011088
F-PLACE1003044//cDNA encoding novel rat protein TIP120 which is formed of complex with TBP (TATA binding protein).//1.6e-123:687:91//E12829
F-PLACE1003045//H.sapiens CpG island DNA genomic Mse1 fragment, clone 47g6, forward read cpg47g6.ft1a.//0.0064:52:96//Z61200
F-PLACE1003092//CIT-HSP-387P22.TRB CIT-HSP Homo sapiens genomic clone 387P22, genomic survey sequence.//0.0031:249:63//B60158
F-PLACE1003100//Human Hep27 protein mRNA, complete cds.//8.9e-65:650:73//U31875
F-PLACE1003108
F-PLACE1003136//Homo sapiens chromosome 5, P1 clone 1130f1 (LBNL H40), complete sequence.//6.3e-46:606:68//AC004219
F-PLACE1003145
F-PLACE1003153//RPCI11-13P16.TP RPCI-11 Homo sapiens genomic clone RPCI-11-13P16, genomic survey sequence.//2.7e-63:478:82//B76206
F-PLACE1003174//Human DNA sequence from clone 441J1 on chromosome 6p24 Contains STS, GSS, complete sequence.//0.61:147:65//Z99495
F-PLACE1003176//HS_2255_A2_B01_MF CIT Approved Human Genomic Sperm Library D Homo sapiens genomic clone Plate=2255 Col=2 Row=C, genomic survey sequence.//6.3e-09:137:76//AQ131934
F-PLACE1003190//Homo sapiens clone RG332P12, WORKING DRAFT SEQUENCE, 1 unordered pieces.//2.4e-138:791:901/AC005095
F-PLACE1003200//P.falciparum complete gene map of plastid-like DNA (IR-B).//8.7e-06:728:57//X95276
F-PLACE1003205//Human BAC clone RG354L07 from 7q31, complete sequence.//7.5e-05:249:63//AC002466
F-PLACE1003238//HS_3239_A2_G02_MR CIT Approved Human Genomic Sperm Library D Homo sapiens genomic clone Plate=3239 Col=4 Row=M, genomic survey sequence.//0.36:64:87//AQ209954
F-PLACE1003249
F-PLACE1003256
F-PLACE1003258//HS_3223_A1_G10_T7 CIT Approved Human Genomic Sperm Library D Homo sapiens genomic clone Plate=3223 Col=19 Row=M, genomic survey sequence.//1.4e-07:227:65//AQ190317
F-PLACE1003296//CIT-HSP-2337F11.TF CIT-HSP Homo sapiens genomic clone 2337F11, genomic survey sequence.//1.1e-13:97:95//AQ057429
F-PLACE1003302//Figure 2. Nucleotide and translated protein sequences of HPF1, -2, and 9.//2.3e-92:485:95//M27877
F-PLACE1003334
F-PLACE1003342
F-PLACE1003343//Homo sapiens clone DJ1022I14, WORKING DRAFT SEQUENCE, 14 unordered pieces.//1.0e-20:179:84//AC004951
F-PLACE1003353//Homo sapiens breast cancer antiestrogen resistance 3 protein (BCAR3) mRNA, complete cds.//8.0e-143:773:92//U92715
F-PLACE1003361//Human Cosmid g1248a143 from 7q31.3, complete sequence.//1.9e-30:402:70//AC004095
F-PLACE1003366
F-PLACE1003369//Plasmodium falciparum MAL3P2, complete sequence.//7.6e-07:378:60//AL034558
F-PLACE1003373//Homo sapiens PAC clone DJ0740L10 from 7p13-p14, complete sequence.//6.0e-18:471:61//AC005247
F-PLACE1003375
F-PLACE1003383//Homo sapiens genomic DNA of 9q32 anti-oncogene of flat epitherium cancer , segment 10/10.//2.3e-157:779:96//AB020878
F-PLACE1003394//Sprague-Dawley (clone LRB13) RAB14 mRNA, complete cds.//1.2e-104:596:91//M83680
F-PLACE1003401//RPCI11-71J5.TJ RPCI11 Homo sapiens genomic clone R-71J5, genomic survey sequence.//0.85:140:65//AQ268588
F-PLACE1003420//Caenorhabditis elegans DNA *** SEQUENCING IN PROGRESS *** from clone Y1E3, WORKING DRAFT SEQUENCE.//0.0015:286:60//AL021388
F-PLACE1003454//Plasmodium falciparum microsatellite pe63 sequence.//0.0084:219:61//AF015470
F-PLACE1003478//Homo sapiens calcium-dependent chloride channel-1 (hCLCA1) gene, complete cds.//1.3e-11:746:60//AF039401
F-PLACE1003493
F-PLACE1003516//Homo sapiens chromosome 17, clone HRPC987K16, complete sequence.//8.2e-41:379:78//AC002994
F-PLACE1003519//Homo sapiens chromosome 21q22.3 PAC 141B3, complete sequence, containing ribosomal protein homologue pseudogene L23a.//6.2e-21:247:76//AF064859
F-PLACE1003521//Human DNA sequence from PAC 257A7 on chromosome 6p24. Contains two unknown genes and ESTs, STSs and a GSS.//4.4e-68:502:79//AL008729
F-PLACE1003528//Homo sapiens DNA sequence from clone 78F24 on chromosome 22q12.1-12.3. Contains one exon of an Oxysterol-binding protein (OSBP) LIKE gene. Contains GSSs and an STS, complete sequence.//1.0:323:58//AL022336
F-PLACE1003537//Homo sapiens multispanning membrane protein mRNA, complete cds.//0.0054:322:59//U94831
F-PLACE1003553//Human DNA sequence *** SEQUENCING IN PROGRESS *** from clone 97P20, WORKING DRAFT SEQUENCE.//2.9e-78:267:88//AL031297
F-PLACE1003566//Plasmodium falciparum MAL3P3, complete sequence.//0.00026:514:58//Z98547
F-PLACE1003575//Plasmodium falciparum 3D7 chromosome 12 PFYAC69 genomic sequence, WORKING DRAFT SEQUENCE, 4 unordered pieces.//0.079:755:54//AC004688
F-PLACE1003583//Human DNA sequence from clone 246H3 on chromosome 22q11.21-12.2 Contains LRP5 (Lipoprotein Receptor Related Protein) pseudogene, EST, CA repeats (D22S414, D22S925, D22S926), STS, GSS and CpG island, complete sequence.//1.1e-41:212:74//AL022324
F-PLACE1003584//Plasmodium falciparum DNA *** SEQUENCING IN PROGRESS *** from contig 4-56, complete sequence.//0.0038:465:57//AL010230
F-PLACE1003592//Homo sapiens chromosome 17, clone 296K1, WORKING DRAFT SEQUENCE, 10 unordered pieces.//0.72:111:71//AC002557
F-PLACE1003593//Human PAC clone DJ318C15 from Xq23, complete sequence.//0.096:162:66//AC002476
F-PLACE1003596//Mus musculus integral membrane protein 1 (Itm1) mRNA, complete cds.//1.4e-54:685:68//L34260
F-PLACE1003602//Homo sapiens mRNA expressed in placenta.//1.1e-138:679:97//D83200
F-PLACE1003605//Homo sapiens chromosome 16, cosmid clone RT81 (LANL), complete sequence.//0.0074:265:63//AC005356
F-PLACE1003611//HS_2198_B1_D02_MF CIT Approved Human Genomic Sperm Library D Homo sapiens genomic clone Plate=2198 Col=3 Row=H, genomic survey sequence.//2.1e-23:137:97//AQ184475
F-PLACE1003618//Homo sapiens chromosome 4 clone C0011C13 map 4p16, complete sequence.//3.0e-122:725:89//AC006226
F-PLACE1003625//HS_2238_B2_D11_MF CIT Approved Human Genomic Sperm Library D Homo sapiens genomic clone Plate=2238 Col=22 Row=H, genomic survey sequence.//4.8e-12:92:94//AQ065662
F-PLACE1003638//Arabidopsis thaliana genomic DNA, chromosome 5, P1 clone: MKD10, complete sequence.//0.043:264:63//AB011478
F-PLACE1003669
F-PLACE1003704//RPCI11-23H21.TKBF RPCI-11 Homo sapiens genomic clone RPCI-11-23H21, genomic survey sequence.//7.1e-31:199:91//AQ013830
F-PLACE1003709//Homo sapiens mitotic checkpoint kinase Bub1 (BUB1) mRNA, complete cds.//4.3e-132:669:95//AF053305
F-PLACE1003711//Homo sapiens DNA sequence from PAC 163M9 on chromosome 1p35.1-p36.21. Contains protein synthesis factor (eIF-4C), D1F15S1A pseudogene, ESTs, STS, GSS, complete sequence.//1.5e-31:166:99//AL021920
F-PLACE1003723//HS_2231_A2_C07_MR CIT Approved Human Genomic Sperm Library D Homo sapiens genomic clone Plate=2231 Col=14 Row=E, genomic survey sequence.//1.2e-12:114:90//AQ235672
F-PLACE1003738//Human zinc finger protein 42 (MZF-1) mRNA, complete cds.//5.9e-33:592:67//M58297
F-PLACE1003760//Homo sapiens tetraspan TM4SF (TSPAN-3) mRNA, complete cds.//3.6e-11:92:93//AF054840
F-PLACE1003762
F-PLACE1003768//Homo sapiens genomic DNA, chromosome 21q22.2 (Down Syndrome region), segment 7/15, WORKING DRAFT SEQUENCE.//4.8e-77:737:76//AP000014
F-PLACE1003771//Homo sapiens BAC clone GS164B05 from 7p21-p22, complete sequence.//2.1e-164:793:98//AC004160
F-PLACE1003783//HS_2190_A2_C02_T7 CIT Approved Human Genomic Sperm Library D Homo sapiens genomic clone Plate=2190 Col=4 Row=E, genomic survey sequence.//1.1e-26:147:100//AQ218757
F-PLACE1003784//Homo sapiens Xp22-150 BAC GSHB-309P15 (Genome Systems Human BAC Library) complete sequence.//4.5e-57:706:68//AC006210
F-PLACE1003795//Homo sapiens Xq28 genomic DNA in the region of the L1CAM locus containing the genes for neural cell adhesion molecule L1 (L1CAM), arginine-vasopressin receptor (AVPR2), C1 p115 (C1), ARD1 N-acetyltransferase related protein (TE2), renin-binding protein (RbP), host cell factor 1 (HCF1), and interleukin-1 receptor-associated kinase (IRAK) genes, complete cds, and Xq281u2 gene.//0.015:296:60//U52112
F-PLACE1003833//Homo sapiens DNA sequence from cosmid N75B3 on chromosome 22 Contains EST, exon trap, complete sequence.//0.52:212:64//AL022339
F-PLACE1003850//P.falciparum histidine-rich protein genes.//0.39:330:60//M17028
F-PLACE1003858//Human DNA sequence from PAC 332O11 on chromosome 1q24-1q25. Contains ESTs and STSs.//4.8e-07:461:59//Z98043
F-PLACE1003864//Plasmodium falciparum 3D7 chromosome 12 PFYAC336 genomic sequence, WORKING DRAFT SEQUENCE, 5 unordered pieces.//0.026:538:56//AC005139
F-PLACE1003870//Human DNA sequence *** SEQUENCING IN PROGRESS *** from clone 54B20, WORKING DRAFT SEQUENCE.//6.5e-06:175:69//Z98304
F-PLACE1003885//Mus musculus poly(A) polymerase VI mRNA, complete cds.//9.4e-75:754:72//U58134
F-PLACE1003886//Homo sapiens clone NH0001P09, WORKING DRAFT SEQUENCE, 1 unordered pieces.//6.7e-20:432:64//AC006030
F-PLACE1003888//Human mRNA for phospholipase C, complete cds.//2.6e-53:702:67//D42108
F-PLACE1003892//RPCI11-24P17.TV RPCI-11 Homo sapiens genomic clone RPCI-11-24P17, genomic survey sequence.//3.3e-20:245:65//B86759
F-PLACE1003900//Human DNA sequence *** SEQUENCING IN PROGRESS *** from clone 328E19, WORKING DRAFT SEQUENCE.//2.5e-17:260:71//AL022240
F-PLACE1003903//Mus musculus CTP synthetase homolog (CTPsH) mRNA, complete cds.//2.7e-86:533:87//U49385
F-PLACE1003915//Mus musculus clone OST1963, genomic survey sequence.//6.4e-29:251:80//AF046591
F-PLACE1003923//Homo sapiens full-length insert cDNA clone ZD40A05.//2.8e-25:316:70//AF086251
F-PLACE1003932//Plasmodium falciparum 3D7 chromosome 12 PFYAC181 genomic sequence, WORKING DRAFT SEQUENCE, 8 unordered pieces.//1.6e-05:652:58//AC005505
F-PLACE1003936//CIT-HSP-2387C11.TR.1 CIT-HSP Homo sapiens genomic clone 2387C11, genomic survey sequence-//1.0:223:62//AQ239494
F-PLACE1003968//Rattus norvegicus 5'-AMP-activated protein kinase, gamma-1 subunit mRNA, complete cds.//5.2e-47:505:72//U42413
F-PLACE1004103//Homo sapiens chromosome 19, cosmid R28784, complete sequence.//6.7e-29:241:84//AC005954
F-PLACE1004104//Rattus norvegicus rsec5 mRNA, complete cds.//3.0e-115:719:86//AF032666
F-PLACE1004114//Homo sapiens Chromosome 22q11.2 BAC Clone 77h2 In CES Region, WORKING DRAFT SEQUENCE, 7 unordered pieces.//1.5e-22:213:80//AC000052
F-PLACE1004118//Pseudorabies virus with upstream and downsteam sequences.//0.87:209:64//M34651
F-PLACE1004128//M.musculus G protein beta-subunit mRNA, complete cds.//2.5e-62:437:84//M63658
F-PLACE1004149//Oryctolagus cuniculus translation initiation factor eIF2C mRNA, complete cds.//1.4e-16:342:65//AF005355
F-PLACE1004156//Homo sapiens DNA sequence from PAC 57E3 on chromosome 6p12.1-21.1. Contains GSSs and an STS with a TATC repeat polymorphism, complete sequence.//1.2e-26:299:74//AL022099
F-PLACE1004161
F-PLACE1004183//Homo sapiens for TOM1-like protein.//1.2e-146:731:96//AJ010071
F-PLACE1004197
F-PLACE1004203//Homo sapiens GPI-anchored membrane protein CDw108 precursor, mRNA, complete cds.//4.0e-144:695:98//AF069493
F-PLACE1004242//Homo sapiens DNA sequence from PAC 124C6 on chromosome 6q21. Contains genomic marker D6S1603, ESTs, GSSs and a STS with a CA repeat polymorphism, complete sequence.//2.3e-151:772:95//AL021326
F-PLACE1004256//HS_2010_B2_G04_T7 CIT Approved Human Genomic Sperm Library D Homo sapiens genomic clone Plate=2010 Col=8 Row=N, genomic survey sequence.//1.5e-44:372:79//AQ252434
F-PLACE1004257//Homo sapiens BAC clone NH0342K06 from 2, complete sequence.//0.00011:349:63//AC005034
F-PLACE1004258//Homo sapiens DNA sequence from PAC 779B17 on chromosome 22q13.1. Contains exon trap, complete sequence.//0.77:475:59//AL021806
F-PLACE1004270//Human IgA C alpha 1 switch region (Sa1).//1.7e-08:622:61//L19121
F-PLACE1004274//H.sapiens CpG island DNA genomic Mse1 fragment, clone 18g6, forward read cpg18g6.ft1b.//8.6e-37:196:98//Z57691
F-PLACE1004277//Homo sapiens two pore domain K+ channel (TASK-2) mRNA, complete cds.//6.0e-156:756:97//AF084830
F-PLACE1004284//Arabidopsis thaliana genomic DNA, chromosome 5, P1 clone: MPI7, complete sequence.//0.0060:635:57//AB011480
F-PLACE1004289//HS_3023_B1_E04_T7 CIT Approved Human Genomic Sperm Library D Homo sapiens genomic clone Plate=3023 Col=7 Row=J, genomic survey sequence.//2.4e-12:86:98//AQ094451
F-PLACE1004302//Streptomyces coelicolor cosmid 7H1.//0.26:297:64//AL021411
F-PLACE1004316//H.sapiens mRNA for apoptosis specific protein.//2.9e-150:797:94//Y11588
F-PLACE1004336//Drosophila melanogaster DNA sequence (P1 DS07968 (D117)), complete sequence.//0.87:206:59//AC004267
F-PLACE1004358//Homo sapiens connector enhancer of KSR-like protein CNK1 mRNA, complete cds.//5.9e-139:688:97//AF100153
F-PLACE1004376//Mus musculus clone OST20307, genomic survey sequence.//4.1e-81:498:89//AF046631
F-PLACE1004384//Human DNA sequence *** SEQUENCING IN PROGRESS *** from clone 1121J18, WORKING DRAFT SEQUENCE.//3.6e-41:482:73//AL031653
F-PLACE1004388//Caenorhabditis elegans cosmid K08F11.//8.6e-26:615:62//U70855
F-PLACE1004405//Homo sapiens clone GS512I21, WORKING DRAFT SEQUENCE, 9 unordered pieces.//9.2e-150:749:96//AC005027
F-PLACE1004425//Homo sapiens PAC clone DJ0733B09 from 7p14-p13, complete sequence.//2.4e-08:129:76//AC005532
F-PLACE1004428//R.norvegicus mRNA for Pristanoyl-CoA Oxidase.//7.0e-17:549:61//X95188
F-PLACE1004437//Human NAD+-specific isocitrate dehydrogenase beta subunit precursor, mRNA, nuclear gene encoding mitochondrial protein, complete cds.//3.1e-129:536:99//U49283
F-PLACE1004451//Human DNA sequence from PAC 214K23, BRCA2 gene region chromosome 13q12-13 contains BRCA2 exons 1-24, Interferon Induced 56Kd pseudogene and ESTs.//4.8e-23:231:71//Z74739
F-PLACE1004460//Homo sapiens PAC clone DJ1064B22 from 7q21, complete sequence.//0.96:454:56//AC004954
F-PLACE1004467//HS_2058_B1_C09_T7 CIT Approved Human Genomic Sperm Library D Homo sapiens genomic clone Plate=2058 Col=17 Row=F, genomic survey sequence.//2.4e-87:433:98//AQ242700
F-PLACE1004471//Figure 2. Nucleotide and translated protein sequences of HPF1, -2, and-9.//1.4e-74:665:70//M27877
F-PLACE1004473//CIT-HSP-2045A15.TF CIT-HSP Homo sapiens genomic clone 2045A15; genomic survey sequence.//3.3e-20:140:92//B80243
F-PLACE1004491//Plasmodium falciparum 3D7 chromosome 12 PFYAC1122 genomic sequence, WORKING DRAFT SEQUENCE, 3 unordered pieces.//9.9e-05:794:57//AC004709
F-PLACE1004506//Human Gx-alpha gene.//1.0e-05:231:63//D90150
F-PLACE1004510//Homo sapiens TATA binding protein associated factor (TAFII150) mRNA, complete cds.//3.2e-146:699:98//AF040701
F-PLACE1004516//Human DNA sequence from cosmid SRL9A13, chromosome region 11p13. Contains EST.//1.4e-33:367:71//Z86001
F-PLACE1004518
F-PLACE1004548//Dictyostelium discoideum MigA (migA) gene, complete cds.//2.6e-05:318:62//U86962
F-PLACE1004550//Human FMR1 gene, 5' end.//0.0018:142:66//L19476
F-PLACE1004564//B.taurus mRNA for cleavage and polyadenylation specificity factor.//1.7e-114:513:85//X75931
F-PLACE1004629//Anolis carolinensis Brain-1 gene, complete cds.//0.00013:188:67//AB001868
F-PLACE1004645//Mycobacterium tuberculosis H37Rv complete genome; segment 138/162.//0.66:337:60//Z95120
F-PLACE1004646//Rattus norvegicus retinal pigment epithelium-specific protein (Rpe65) mRNA, complete cds.//1.1e-19:326:63//AF035673
F-PLACE1004658//H.sapiens CpG island DNA genomic Mse1 fragment, clone 55h1, forward read cpg55h1.ft1a./12.4e-34:188:98//Z61632
F-PLACE1004664//Caenorhabditis elegans cosmid W10G6, complete sequence.//1.0:148:65//Z81140
F-PLACE1004672//Human ABL gene, exon 1b and intron 1b, and putative M8604 Met protein (M8604 Met) gene, complete cds.//1.9e-101:182:95//U07561
F-PLACE1004674//Homo sapiens calcium binding protein (ALG-2) mRNA, complete cds.//4.3e-109:625:91//AF035606
F-PLACE1004681//Homo sapiens genomic DNA of 8p21.3-p22 anti-oncogene of hepatocellular colorectal and non-small cell lung cancer , segment 3/11.//1.9e-152:759:96//AB020860
F-PLACE1004686//Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence.//1.2e-34:320:71//Z95152
F-PLACE1004691//HS_3044_A1_G01_MF CIT Approved Human Genomic Sperm Library D Homo sapiens genomic clone Plate=3044 Col=1 Row=M, genomic survey sequence.//0.018:191:63//AQ098323
F-PLACE1004693//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//0.28:573:57//AL022577
F-PLACE1004716//Plasmodium falciparum MAL3P6, complete sequence.//0.00081:428:59//Z98551
F-PLACE1004722//HS_3052_B1_C10_MR CIT Approved Human Genomic Sperm Library D Homo sapiens genomic clone Plate=3052 Col=19 Row=F, genomic survey sequence.//2.3e-05:104:75//AQ134959
F-PLACE1004736//CIT-HSP-2365J21.TF CIT-HSP Homo sapiens genomic clone 2365J21, genomic survey sequence.//1.3e-24:180:88//AQ080498
F-PLACE1004740//RPCI11-58A7.TJ RPCI11 Homo sapiens genomic clone R-58A7, genomic survey sequence.//8.6e-26:522:65//AQ195766
F-PLACE1004743//Mus musculus ubiquitin-protein ligase E3-alpha (Ubr1) mRNA, complete cds.//1.1e-112:711:86//AF061555
F-PLACE1004751
F-PLACE1004773//Homo sapiens inversin protein mRNA, complete cds.//5.4e-171:828:97//AF084367
F-PLACE1004777//Rattus norvegicus mRNA for myosin-RhoGAP protein Myr 7.//4.2e-134:763:90//AJ001713
F-PLACE1004793//Human DNA sequence from clone 323P24 on chromosome Xp11.21-11.23 Contains SPIN (spindlin homolog (PROTEIN DXF34), hypothetical protein EST, STS, GSS, complete sequence.//9.3e-132:759:90//AL022157
F-PLACE1004804
F-PLACE1004813//Plasmodium falciparum 3D7 chromosome 12 PFYAC588 genomic sequence, WORKING DRAFT SEQUENCE, 2 unordered pieces.//6.5e-06:403:58//AC004710
F-PLACE1004814//Homo sapiens chromosome 17, clone hRPK.294_J_22, complete sequence.//9.8e-39:207:99//AC005921
F-PLACE1004815//Homo sapiens PAC clone DJ0651K02 from 7p21-p22, complete sequence.//8.1e-15:203:73//AC004613
F-PLACE1004824//G.gallus PB1 gene.//1.1e-103:759:80//X90849
F-PLACE1004827//HS_2230_A2_A05_MR CIT Approved Human Genomic Sperm Library D Homo sapiens genomic clone Plate=2230 Col=10 Row=A, genomic survey sequence.//4.1e-38:330:81//AQ299313
F-PLACE1004836//H.sapiens nidogen gene (exon 8).//0.97:116:68//X84825
F-PLACE1004838//HS_3241_A2_A04_MR CIT Approved Human Genomic Sperm Library D Homo sapiens genomic clone Plate=3241 Col=8 Row=A, genomic survey sequence.//1.8e-87:425:98//AQ206740
F-PLACE1004840//Sequence 2 from patent US 5728819.//6.7e-47:285:91//I92819
F-PLACE1004868
F-PLACE1004885//Arabidopsis thaliana DNA chromosome 4, ESSA I contig fragment No. 9.//0.14:465:59//Z97344
F-PLACE1004900
F-PLACE1004902//CITBI-E1-2510J4.TR CITBI-E1 Homo sapiens genomic clone 2510J4, genomic survey sequence.//3.6e-06:56:100//AQ261184
F-PLACE1004913//Homo sapiens BAC clone RG054D04 from 7q31, complete sequence.//2.6e-151:770:91//AC005058
F-PLACE1004918//Mus musculus signaling molecule (ATTP) mRNA, complete cds.//2.6e-68:459:84//U97571
F-PLACE1004930//Homo sapiens TNF-induced protein GG2-1 mRNA, complete cds.//4.4e-106:545:95//AF070671
F-PLACE1004934//Human DNA sequence from clone 192P9 on chromosome Xp11.23-11.4. Contains a pseudogene similar to rat Plasmolipin, ESTs and GSSs, complete sequence.//3.5e-45:226:84//AL020989
F-PLACE1004937
F-PLACE1004969
F-PLACE1004972//Homo sapiens PAC clone DJ0612F12 from 7p12-p14, complete sequence.//0.012:316:61//AC004843
F-PLACE1004979//Human DNA sequence from clone 142F18 on chromosome Xq26.3-27.2 Contains part of a gene similar to melanoma-associated antigen, EST, GSS and an inverted repeat, complete sequence.//4.7e-39:394:77//AL031073
F-PLACE1004982//Caenorhabditis elegans cosmid B0507.//0.16:167:65//U64833
F-PLACE1004985//Plasmodium falciparum chromosome 2, section 10 of 73 of the complete sequence.//8.8e-14:590:61//AE001373
F-PLACE1005026
F-PLACE1005027
F-PLACE1005046
F-PLACE1005052//Homo sapiens chromosome Xp22-135-136 clone GSHB-567I1, WORKING DRAFT SEQUENCE, 35 unordered pieces.//2.1e-135:675:97//AC005867
F-PLACE1005055//Homo sapiens mRNA for KIAA0576 protein, partial cds.//1.9e-159:761:98//AB011148
F-PLACE1005066//Homo sapiens actin binding protein MAYVEN mRNA, complete cds.//9.2e-10:757:56//AF059569
F-PLACE1005077
F-PLACE1005085//Homo sapiens Xp22-132-134 BAC GSHB-590J15 (Genome Systems Human BAC library) complete sequence.//6.9e-29:253:77//AC004673
F-PLACE1005086//Homo sapiens chromosome 17, clone HCIT11023, complete sequence.//6.5e-52:446:78//AC002316
F-PLACE1005101//Homo sapiens clone DJ0414A15, WORKING DRAFT SEQUENCE, 9 unordered pieces.//2.0e-146:734:96//AC005225
F-PLACE1005102//Homo sapiens chromosome 19, cosmid R29388, complete sequence.//9.8e-83:254:95//AC004476
F-PLACE1005108//Human BAC clone RG009H02 from 7q31, complete sequence.//0.46:179:64//AC003081
F-PLACE1005111
F-PLACE1005128//Bovine herpesvirus type 1 early-intermediate transcription control protein (BICP4) gene, complete cds.//0.00051:287:63//L14320
F-PLACE1005146//HS_3071_A1_E03_MF CIT Approved Human Genomic Sperm Library D Homo sapiens genomic clone Plate=3071 Col=5 Row=I, genomic survey sequence.//7.4e-38:299:82//AQ103361
F-PLACE1005162//Human BAC clone GS306C12 from 7q21-q22, complete sequence.//2.6e-44:346:82//AC002451
F-PLACE1005176
F-PLACE1005181//CIT-HSP-2340O5.TR CIT-HSP Homo sapiens genomic clone 2340O5, genomic survey sequence.//0.99:211:63//AQ054651
F-PLACE1005187//CIT-HSP-2358N6.TR CIT-HSP Homo sapiens genomic clone 2358N6, genomic survey sequence.//2.7e-07:80:90//AQ074445
F-PLACE1005206//Human BAC clone 133K23 from 7q31.2, complete sequence.//0.98:216:61//AC000061
F-PLACE1005232//Homo sapiens clone DJ1106H14, WORKING DRAFT SEQUENCE, 42 unordered pieces.//0.70:245:63//AC004965
F-PLACE1005243
F-PLACE1005261//Caenorhabditis elegans cosmid T05H10, complete sequence.//0.00041:254:61//Z47812
F-PLACE1005266//H.sapiens mRNA (fetal brain cDNA a4_2g).//9.6e-33:177:98//Z70695
F-PLACE1005277//Homo sapiens mRNA for KIAA0610 protein, partial cds.//1.6e-148:706:98//AB011182
F-PLACE1005287//Plasmodium falciparum (MESA) mRNA exons 1-2, complete cds.//2.8e-15:737:60//M69183
F-PLACE1005305//Bovine mitochondrial GTP:AMP phosphotransferase mRNA, complete cds.//3.8e-111:728:84//M25757
F-PLACE1005308//Clethrionomys glareolus endogenous retroviral sequence ERV-L pol gene, clone ERV-L Vole Cg14.//1.0:128:67//AJ233621
F-PLACE1005313//Caenorhabditis elegans cosmid D2092.//8.8e-11:342:62//U88167
F-PLACE1005327//HS_3080_B2_A12_MR CIT Approved Human Genomic Sperm Library D Homo sapiens genomic clone Plate=3080 Col=24 Row=B, genomic survey sequence.//4.1e-25:147:96//AQ139116
F-PLACE1005331//Homo sapiens chromosome 19, cosmid F20569, complete sequence.//1.4e-132:399:94//AC004794
F-PLACE1005335//Human Chromosome 3 pac pDJ70i11, WORKING DRAFT SEQUENCE, 2 unordered pieces.//5.5e-114:237:92//AC000380
F-PLACE1005373
F-PLACE1005374//Homo sapiens chromosome 7 common fragile site, complete sequence.//0.20:305:58//AF017104
F-PLACE1005409//Human BAC clone RG167B05 from 7q21, complete sequence.//2.5e-148:760:95//AC003991
F-PLACE1005453//Caenorhabditis elegans DNA *** SEQUENCING IN PROGRESS *** from clone Y48A6, WORKING DRAFT SEQUENCE.//0.00069:582:59//Z92854
F-PLACE1005467//Rat mRNA.//0.0014:131:70//M59859
F-PLACE1005471//Human DNA sequence from clone 45I4 on chromosome 6q24.1-24.3. Contains two putative unknown genes, ESTs, STSs and GSSs, complete sequence.//3.0e-23:530:67//AL023581
F-PLACE1005477//Human DNA sequence from clone J181N11, WORKING DRAFT SEQUENCE.//3.3e-131:814:88//Z82191
F-PLACE1005480//Homo sapiens DNA sequence from PAC 257I20 on chromosome 22q13.1-13.2. Contains cytochrome P450 pseudogenes CYP2D7P, CYP2D8P, CYP2D6(D),TCF20, NADH ubiquinone oxidoreductase B14 subunit, ESTs, CA repeat, STS, GSS.//7.0e-34:246:73//AL021878
F-PLACE1005481//RPCI11-74L17.TJ RPCI11 Homo sapiens genomic clone R-74L17, genomic survey sequence.//0.37:403:57//AQ266885
F-PLACE1005494//Homo sapiens transient receptor potential protein 6 mRNA, complete cds.//2.1e-67:325:99//AF080394
F-PLACE1005502//Homo sapiens BAC clone NH0161H12 from 7p14-p15, complete sequence.//0.015:403:61//AC005589
F-PLACE1005526//H.sapiens CpG island DNA genomic Mse1 fragment, clone 9f1, reverse read cpg9f1.rt1a.//3.6e-27:159:96//Z66485
F-PLACE1005528//Homo sapiens genomic DNA, chromosome 21q11.1, segment 9/28, WORKING DRAFT SEQUENCE.//2.6e-28:449:67//AP000038
F-PLACE1005530//Homo sapiens clone DJ0691L07, complete sequence.//6.5e-18:234:72//AC004860
F-PLACE1005550//Fugu rubripes GSS sequence, clone 048A08bH3, genomic survey sequence.//1.2e-14:123:75//AL025925
F-PLACE1005554//Leishmania tarentolae mitochondrial 12S ribosomal RNA gene.//0.43:209:66//X02354
F-PLACE1005557//Homo sapiens chromosome 17, clone hRPC.117_B_12, complete sequence.//9.3e-113:536:97//AC004707
F-PLACE1005574//Plasmodium falciparum 3D7 chromosome 12 PFYAC1383 genomic sequence, WORKING DRAFT SEQUENCE, 3 unordered pieces.//1.1e-10:514:59//AC005504
F-PLACE1005584//Homo sapiens mRNA for KIAA0617 protein, complete cds.//0.00056:289:63//AB014517
F-PLACE1005595//Human Chromosome 11q12.2 PAC clone pDJ606g6, complete sequence.//1.2e-111:262:89//AC004126
F-PLACE1005603
F-PLACE1005611//F16O5TFC IGF Arabidopsis thaliana genomic clone F16O5, genomic survey sequence.//2.0e-10:209:66//B98589
F-PLACE1005623
F-PLACE1005630//High throughput sequencing of human chromosome 12, WORKING DRAFT SEQUENCE, 1 ordered pieces.//1.2e-93:230:98//AC005840
F-PLACE1005639//HS_3095_B1_A03_MF CIT Approved Human Genomic Sperm Library D Homo sapiens genomic clone Plate=3095 Col=5 Row=B, genomic survey sequence.//1.2e-05:220:63//AQ123022
F-PLACE1005646//Homo sapiens RNA helicase-related protein mRNA, complete cds.//6.4e-150:721:98//AF083255
F-PLACE1005656//H.sapiens RR2 mRNA for small subunit ribonucleotide reductase.//1.3e-51:480:74//X59618
F-PLACE1005666//RPCI11-78O15.TV RPCI11 Homo sapiens genomic clone R-78O15, genomic survey sequence.//8.7e-05:243:62//AQ284667
F-PLACE1005698//Human membrane-associated lectin type-C mRNA.//1.9e-63:374:85//M98457
F-PLACE1005727//Plasmodium falciparum chromosome 2, section 59 of 73 of the complete sequence.//0.69:633:57//AE001422
F-PLACE1005730//HS_2026_B1_H11_T7 CIT Approved Human Genomic Sperm Library D Homo sapiens genomic clone Plate=2026 Col=21 Row=P, genomic survey sequence.//2.0e-24:286:74//AQ231147
F-PLACE1005739//Mus musculus IFN-gamma induced (Mg11) mRNA, complete cds.//2.8e-55:621:71//U15635
F-PLACE1005755//HS_2213_A2_H11_MR CIT Approved Human Genomic Sperm Library D Homo sapiens genomic clone Plate=2213 Col=22 Row=O, genomic survey sequence.//1.4e-25:290:75//AQ136844
F-PLACE1005763//Rat medium-chain S-acyl fatty acid synthetase thio ester hydrolase (MCH), complete cds.//4.5e-40:297:70//M16200
F-PLACE1005799//R.norvegicus mRNA for mitochondrial isoform of cytochrome b5.//0.91:287:63//Y12517
F-PLACE10058021/Homo sapiens PAC clone DJ044L15 from Xq23, complete sequence.//5.0e-109:530:98//AC004827
F-PLACE1005803//HS_3092_B1_A10_MF CIT Approved Human Genomic Sperm Library D Homo sapiens genomic clone Plate=3092 Col=19 Row=B, genomic survey sequence.//2.4e-08:76:96//AQ103695
F-PLACE1005804//Homo sapiens alpha 1,2-mannosidase IB mRNA, complete cds.//1.4e-126:636:96//AF027156
F-PLACE1005813//Homo sapiens sorting nexin 2 (SNX2) mRNA, complete cds.//2.6e-154:739:98//AF065482
F-PLACE1005828//Homo sapiens chromosome 17, clone hRPC.971_F_3, WORKING DRAFT SEQUENCE, 1 ordered pieces.//2.2e-37:355:77//AC004150
F-PLACE1005834//Plasmodium falciparum DNA *** SEQUENCING IN PROGRESS *** from contig 4-105, complete sequence.//0.00080:663:58//AL010283
F-PLACE1005845//Plasmodium falciparum 3D7 chromosome 12 PFYAC812 genomic sequence, WORKING DRAFT SEQUENCE, 8 unordered pieces.//0.00015:340:58//AC004153
F-PLACE1005850//Human DNA sequence from clone 465N24 on chromosome 1p35.1-36.13. Contains two novel genes, ESTs, GSSs and CpG islands, complete sequence.//1.8e-46:278:85//AL031432
F-PLACE1005851
F-PLACE1005876//B.taurus mRNA for cleavage and polyadenylation specificity factor.//5.0e-120:701:89//X75931
F-PLACE1005884//CIT-HSP-2333O12.TR CIT-HSP Homo sapiens genomic clone 2333O12, genomic survey sequence.//4.6e-78:385:98//AQ039226
F-PLACE1005890//Schizosaccharomyces pombe bem1/bud5 suppressor (Bem46+) mRNA, partial cds.//9.3e-16:638:57//U29892
F-PLACE1005898//Rattus norvegicus A-kinase anchoring protein AKAP150 mRNA, complete cds.//1.0:178:65//U67136
F-PLACE1005921//M.musculus mRNA for immunity associated protein 38.//6.6e-17:614:59//Y08026
F-PLACE1005923//RPCI11-33G19.TJ RPCI-11 Homo sapiens genomic clone RPCI-11-33G19, genomic survey sequence.//4.0e-10:535:57//AQ046151
F-PLACE1005925//Human DNA sequence *** SEQUENCING IN PROGRESS *** from clone 537K23, WORKING DRAFT SEQUENCE.//0.17:159:65//AL034405
F-PLACE1005932
F-PLACE1005934//H.sapiens CpG island DNA genomic Mse1 fragment, clone 165g2, forward read cpg165g2.ft1a.//8.3e-43:247:93//Z57153
F-PLACE1005936//F.rubripes GSS sequence, clone 069K22aG2, genomic survey sequence.//0.91:116:68//AL014719
F-PLACE1005951//Rhodobacter sphaeroides DMSO/TMAO-sensor kinase (dorS), DMSO/TMAO-response regulator (dorR), DMSO/TMAO-cytochrome c-containing subunit (dorC), DMSO-membrane protein (dorB), and DMSO/TMAO-reductase (dorA) genes, complete cds.//0.0022:495:59//AF016236
F-PLACE1005953//Homo sapiens PAC clone DJ0320J15 from Xq23, complete sequence.//2.9e-05:442:61//AC004081
F-PLACE1005955//Caenorhabditis elegans cosmid F01F1.//4.3e-20:409:64//U13070
F-PLACE1005966//P.falciparum aarp3 gene, exon.//0.0083:270:64//Y08925
F-PLACE1005968
F-PLACE1005990//Homo sapiens chromosome 12p13.3 clone RPCI11-407G6, WORKING DRAFT SEQUENCE, 51 ordered pieces.//1.0e-100:513:96//AC005866
F-PLACE1006002//Human DNA sequence *** SEQUENCING IN PROGRESS *** from clone 229A8, WORKING DRAFT SEQUENCE.//2.5e-54:444:77//Z86090
F-PLACE1006003//HS-1059-A2-G01-MF.abi CIT Human Genomic Sperm Library C Homo sapiens genomic clone Plate=CT 781 Col=2 Row=M, genomic survey sequence.//3.4e-05:214:64//B44442
F-PLACE1006011//Mus musculus poly-(ADPribosyl)-transferase homolog PARP mRNA, complete cds.//4.3e-71:580:79//AF072521
F-PLACE1006017//Homo sapiens Chromosome 16 BAC clone CIT987-SKA-113A6 -complete genomic sequence, complete sequence.//8.6e-32:177:83//AC002299
F-PLACE1006037//Mus musculus B6D2F1 clone 2C11B mRNA.//1.8e-34:269:83//U01139
F-PLACE1006040//Homo sapiens mRNA for alpha endosulfine.//3.4e-147:719:97//X99906
F-PLACE1006076//Homo sapiens DNA sequence from PAC 79C4 on chromosome 1q24. Contains the PMX1 gene, coding for two alternative forms of the Paired Mesoderm Homeobox protein 1 (PMX-1, PHOX-1). Contains ESTs, STSs and BAC end sequences (GSSs), complete sequence.//0.37:332:62//Z97200
F-PLACE1006119//Homo sapiens Ran-GTP binding protein mRNA, partial cds.//1.3e-145:679:99//AF039023
F-PLACE1006129
F-PLACE1006139//Saccharomyces cerevisiae chromosome VI cosmid 9965.//4.8e-27:693:60//D44597
F-PLACE1006143//Human DNA sequence *** SEQUENCING IN PROGRESS *** from clone 169I5, WORKING DRAFT SEQUENCE.//4.7e-46:435:77//Z93015
F-PLACE1006157//Saguinus oedipus mRNA for membrane cofactor protein CD46, complete cds, clone:B2.//0.048:290:60//D85750
F-PLACE1006159//Homo sapiens chromosome 10 clone CIT987SK-1054O2 map 10q25, complete sequence.//3.2e-129:466:96//AC005661
F-PLACE1006164//HS_3003_A1_F08_MR CIT Approved Human Genomic Sperm Library D Homo sapiens genomic clone Plate=3003 Col=15 Row=K, genomic survey sequence.//1.4e-70:388:93//AQ118200
F-PLACE1006167//Homo sapiens chromosome 19, cosmid F23149, complete sequence.//4.3e-78:385:86//AC005239
F-PLACE1006170//Mouse mRNA for alpha-adaptin (C).//3.5e-91:630:84//X14972
F-PLACE1006187//Homo sapiens cyclin E2 mRNA, complete cds.//3.9e-149:694:99//AF091433
F-PLACE1006195//Homo sapiens Xp22 BAC GS-607H18 (Genome Systems Human BAC library) complete sequence.//2.5e-16:283:70//AC003658
F-PLACE1006196//Mouse RNA helicase and RNA-dependent ATPase from the DEAD box family mRNA, complete cds.//2.2e-94:648:84//L25125
F-PLACE1006205//Human Xp22 cosmid U250A9, complete sequence.//0.15:533:58//U75931
F-PLACE1006223//F24L20-T7 IGF Arabidopsis thaliana genomic clone F24L20, genomic survey sequence.//0.0068:175:64//B19803
F-PLACE1006225//CIT-HSP-2335I23.TF CIT-HSP Homo sapiens genomic clone 2335I23, genomic survey sequence.//2.1e-19:149:90//AQ039880
F-PLACE1006236//Human chromosome 12p15 BAC clone CIT987SK-99D8 complete sequence.//0.51:290:58//U91327
F-PLACE1006239//Homo sapiens BAC clone RG118D07 from 7q31, complete sequence.//7.4e-158:452:96//AC004142
F-PLACE1006246//RPCI11-36I23.TK RPCI-11 Homo sapiens genomic clone RPCI-11-36I23, genomic survey sequence.//2.6e-31:176:97//AQ045400
F-PLACE1006248//Homo sapiens mRNA for KIAA0648 protein, partial cds.//2.3e-166:791:98//AB014548
F-PLACE1006262//342E3.TVD CIT978SKA1 Homo sapiens genomic clone A-342E03, genomic survey sequence.//1.0:228:63//B16447
F-PLACE1006288//Human DNA sequence *** SEQUENCING IN PROGRESS *** from clone 20N2, WORKING DRAFT SEQUENCE.//6.6e-172:809:99//AL031320
F-PLACE1006318
F-PLACE1006325//Homo sapiens PAC clone DJ0988L12 from 7q11.23-q21.1, complete sequence.//0.079:396:59//AC004454
F-PLACE1006335//Mouse Ig third hypervariable region (HCDR3), nonproductively rearranged alpha-chain gene VHSB32-D-JH2 region.//1.0:90:67//M55721
F-PLACE1006357//Plasmodium falciparum 3D7 chromosome 12 PFYAC1383 genomic sequence, WORKING DRAFT SEQUENCE, 3 unordered pieces.//0.16:445:56//AC005504
F-PLACE1006360//Plasmodium falciparum MAL3P7, complete sequence.//6.1e-05:625:57//AL034559
F-PLACE1006368//X.laevis mRNA for KLP2 protein.//3.0e-25:376:68//X94082
F-PLACE1006371//Homo sapiens chromosome 16, cosmid clone 360H6 (LANL), complete sequence.//2.0e-146:711:97//AC004232
F-PLACE1006382
F-PLACE1006385//Homo sapiens epsin 2a mRNA, complete cds.//5.1e-110:539:97//AF062085
F-PLACE1006412//Homo sapiens BAC clone GS588G18 from 7p12-p14, complete sequence.//1.3e-23:463:68//AC005029
F-PLACE1006414//Homo sapiens PCAF associated factor 65 alpha mRNA, complete cds.//1.3e-109:525:98//AF069735
F-PLACE1006438//Homo sapiens mRNA for KIAA0557 protein, partial cds.//6.9e-23:531:65//AB011129
F-PLACE1006445//HS_3071_A1_C11_MF CIT Approved Human Genomic Sperm Library D Homo sapiens genomic clone Plate=3071 Col=21 Row=E, genomic survey sequence.//4.7e-74:392:95//AQ 103347
F-PLACE1006469//Rhodobacter capsulatus strain SB1003, partial genome.//1.1e-40:686:65//AF010496
F-PLACE1006470//T.brucei kinetoplast maxicircle variable region DNA.//0.99:250:59//Z15118
F-PLACE1006482//Human DNA sequence *** SEQUENCING IN PROGRESS *** from clone 447C4, WORKING DRAFT SEQUENCE.//4.3e-120:328:98//AL021977
F-PLACE1006488//Canine mRNA for 68kDA subunit of signal recognition particle (SRP68).//6.5e-86:478:91//X53744
F-PLACE1006492
F-PLACE1006506
F-PLACE1006521//Homo sapiens BAC clone RG281G05 from 7p15-p21, complete sequence.//0.0010:547:58//AC005083
F-PLACE1006531//Oryctolagus cuniculus translation initiation factor eIF2C mRNA, complete cds.//2.6e-84:625:80//AF005355
F-PLACE1006534//Caenorhabditis elegans cosmid Y40H7A, complete sequence.//0.00031:671:58//AL033510
F-PLACE1006540
F-PLACE1006552//P.falciparum glutamic acid-rich protein gnen, complete cds.//6.0e-10:636:59//J03998
F-PLACE1006598//Homo sapiens BAC clone NH0539B24 from 7p15.1-p14, complete sequence.//9.8e-25:170:77//AC006044
F-PLACE1006615//Homo sapiens eukaryotic translation initiation factor eIF3, p35 subunit mRNA, complete cds.//6.7e-167:781:99//U97670
F-PLACE1006617//Homo sapiens Xp22 BAC GSHB-433024 (Genome Systems Human BAC library) complete sequence.//0.98:514:59//AC004470
F-PLACE1006626//H.sapiens DNA 3' flanking simple sequence region clone wg2c3.//0.00079:206:62//X76589
F-PLACE1006629//Human BAC clone RG333F24 from 7q11.2-q21, complete sequence.//0.0012:576:57//AC004015
F-PLACE1006640//P.falciparum complete gene map of plastid-like DNA (IR-B).//0.0018:588:59//X95276
F-PLACE1006673//Plasmodium falciparum 3D7 chromosome 12 PFYAC69 genomic sequence, WORKING DRAFT SEQUENCE, 4 unordered pieces.//0.0028:469:58//AC004688
F-PLACE1006678//Mus musculus UDP-Gal:betaGlcNAc beta 1,3-galactosyltranferase-I (b3GT1) gene, complete cds.//0.00011:184:64//AF029790
F-PLACE1006704//Mus musculus dentin sialophosphoprotein precursor (DSPP) mRNA, complete cds.//0.0013:380:62//U67916
F-PLACE1006731//Human DNA sequence from PAC 408N23 on chromosome 22q13. Contains HIP, HSC70-INTERACTING PROTEIN (PROGESTERONE RECEPTOR-ASSOCIATED P48 PROTEIN), ESTs and STS.//1.5e-78:520:86//Z98048
F-PLACE1006754//Homo sapiens chromosome 19, cosmid R29124, complete sequencer/1.9e-135:378:99//AC005626
F-PLACE1006760//CIT-HSP-2336O13.TR CIT-HSP Homo sapiens genomic clone 2336O13, genomic survey sequence.//0.018:147:66//AQ039246
F-PLACE1006779//Plasmodium falciparum chromosome 2, section 63 of 73 of the complete sequence.//2.6e-08:823:58//AE001426
F-PLACE1006782//Homo sapiens clone NH0005N18, WORKING DRAFT SEQUENCE, 2 unordered pieces.//0.043:252:65//AC005487
F-PLACE1006792//HS_3165_B1_H01_MR CIT Approved Human Genomic Sperm Library D Homo sapiens genomic clone Plate=3165 Col=1 Row=P, genomic survey sequence.//1.4e-11:249:67//AQ149559
F-PLACE1006795//Mouse eph-related receptor tyrosine kinase (Mek4) mRNA, complete cds.//1.3e-12:155:80//M68513
F-PLACE1006800//Plasmodium falciparum DNA *** SEQUENCING IN PROGRESS *** from contig 4-92, complete sequence.//6.7e-05:391:62//AL010272
F-PLACE1006805//paramecium species 1,168 mt dna dimer: replication init. region.//9.1e-09:369:62//K00915
F-PLACE1006815//Human DNA sequence *** SEQUENCING IN PROGRESS *** from clone 321D2, WORKING DRAFT SEQUENCE.//0.89:465:58//AL031033
F-PLACE1006819//Homo sapiens clone DJ1163L11, complete sequence.//1.5e-121:618:91//AC005230
F-PLACE1006829//Brn-3a=class V POU transcription factor [mice, CD/CD, embryo fibroblast cells, Genomic, 2160 nt].//0.011:145:68//S69350
F-PLACE1006860//Plasmodium falciparum MAL3P7, complete sequence.//2.2e-07:691:58//AL034559
F-PLACE1006867//Human DNA sequence *** SEQUENCING IN PROGRESS *** from clone 323M4, WORKING DRAFT SEQUENCE.//1.5e-132:643:98//AL033378
F-PLACE1006878
F-PLACE1006883//Mycobacterium tuberculosis H37Rv complete genome; segment 138/162.//1.0:236:62//Z95120
F-PLACE1006901//Mus musculus t complex testis-specific protein (Tctex2) gene, t haplotype, promoter sequence.//2.7e-19:171:81//U21672
F-PLACE1006904
F-PLACE1006917//H.sapiens CpG island DNA genomic Mse1 fragment, clone 79g10, forward read cpg79g10.ft1a.//1.3e-21:131:98//Z63175
F-PLACE1006932//Mus musculus FKBP65 binding protein mRNA, complete cds.//0.99:248:61//L07063
F-PLACE1006935//Homo sapiens chromosome 9 duplication of the T cell receptor beta locus and trypsinogen gene families.//0.85:161:63//AF029308
F-PLACE1006956//Hylobates lar involucrin gene, complete cds.//0.077:355:61//M35447
F-PLACE1006958//Mus musculus osmotic stress protein 94 (Osp94) mRNA, complete cds.//2.9e-89:483:86//U23921
F-PLACE1006961//Saccharomyces cerevisiae mitochondrial tRNA-Tyr, tRNA-Asn, & amp; tRNA-Met genes.//1.6e-06:651:58//AJ223323
F-PLACE1006962//H.sapiens ir1B mRNA.//7.1e-15:202:71//X63417
F-PLACE1006966//Caenorhabditis elegans DNA *** SEQUENCING IN PROGRESS *** from clone Y105E8, WORKING DRAFT SEQUENCE.//1.7e-26:451:61//AL022594
F-PLACE1006989//cSRL-172A4-u cSRL flow sorted Chromosome 11 specific cosmid Homo sapiens genomic clone cSRL-172A4, genomic survey sequence.//1.0:97:67//B03188
F-PLACE1007014//Rattus norvegicus equilbrative nitrobenzylthioinosine-insensitive nucleoside transporter mRNA, complete cds.//4.2e-07:592:58//AF015305
F-PLACE1007021//Homo sapiens chromosome 19, cosmid F16403; complete sequence.//5.1e-17:285:70//AC005777
F-PLACE1007045//Human DNA sequence from PAC 181N1 on chromosome X contains ESTs, STS polymorphic CA repeat*.//6.2e-131:775 :89//Z82899
F-PLACE1007053//Homo sapiens clone DJ0810E06, WORKING DRAFT SEQUENCE, 8 unordered pieces.//1.7e-143:675:99//AC004895
F-PLACE1007068//Homo sapiens chromosome 17, clone hRPK.214_O_1, complete sequence.//1.3e-131:652:97//AC005224
F-PLACE1007097//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs (BAC end sequences) and a CA repeat polymorphism, complete sequence.//8.3e-158:768:97//AL021368
F-PLACE1007105//Mus musculus muskelin mRNA, complete cds.//4.1e-124:687:91//U72194
F-PLACE1007111//Plasmodium falciparum 3D7 chromosome 12 PFYAC336 genomic sequence, WORKING DRAFT SEQUENCE, 5 unordered pieces.//4.7e-05:586:56//AC005139
F-PLACE1007112//HS_2234_B2_G10_MF CIT Approved Human Genomic Sperm Library D Homo sapiens genomic clone Plate=2234 Col=20 Row=N, genomic survey sequence.//0.26:200:62//AQ087801
F-PLACE1007132//CIT978SK-A-211C6.TVB CIT978SK Homo sapiens genomic clone A-211C6, genomic survey sequence.//1.3e-40:255:92//B72112
F-PLACE1007140//QN1 orf [Coturnix coturnix, japonica, K2 neuroretinal cells, mRNA Partial, 3884 nt].//4.9e-15:386:62//S68151
F-PLACE1007178//Plasmodium falciparum 3D7 chromosome 12 PFYACB8-420 genomic sequence, WORKING DRAFT SEQUENCE, 14 unordered pieces.//0.011:329:61//AC005140
F-PLACE1007226//Human lipocortin (LIP) 2 gene, upstream region.//0.0036:180:63//M62899
F-PLACE1007238//FMR1 {CGG repeats} [human, Fragile X syndrome patient, Genomic, 429 nt].//2.8e-08:269:63//S74494
F-PLACE1007239//Homo sapiens mRNA for transcription elongation factor S-II, hS-II-T1, complete cds.//6.3e-57:405:87//D50495
F-PLACE1007242//HS_3006_A1_B11_MR CIT Approved Human Genomic Sperm Library D Homo sapiens genomic clone Plate=3006 Col=21 Row=C, genomic survey sequence.//0.088:191:59//AQ089443
F-PLACE1007243//Human transporter protein (g17) mRNA, complete cds.//7.9e-12:245:66//U49082
F-PLACE1007257//Homo sapiens mRNA for dia-12c protein.//5.2e-144:677:98//Y15908
F-PLACE1007274//HS_3003_A1_D08_MF CIT Approved Human Genomic Sperm Library D Homo sapiens genomic clone Plate=3003 Col=15 Row=G, genomic survey sequence.//7.4e-49:345:85//AQ294154
F-PLACE1007276//Fugu rubripes GSS sequence, clone 014O10aG11, genomic survey sequence.//0.0052:228:62//AL024982
F-PLACE1007282//F.rubripes GSS sequence, clone 019O07aB3, genomic survey sequence.//0.024:289:58//AL011743
F-PLACE1007286//Human Chromosome 16 BAC clone CIT987SK-A-256A9, complete sequence.//0.0048:185:69//AC002492
F-PLACE1007301//Dictyostelium discoideum gene for TRFA, complete cds.//0.069:761:57//AB009080
F-PLACE1007317
F-PLACE1007342
F-PLACE1007346//Homo sapiens estrogen-responsive B box protein (EBBP) mRNA, complete cds.//5.4e-120:567:98//AF096870
F-PLACE1007367//Homo sapiens clone RG228D17, WORKING DRAFT SEQUENCE, 2 unordered pieces.//1.2e-59:613:75//AC005077
F-PLACE1007375//Caenorhabditis elegans cosmid D2092.//1.8e-12:193:70//U88167
F-PLACE1007386
F-PLACE1007402//HS_2170_A2_D12_MF CIT Approved Human Genomic Sperm Library D Homo sapiens genomic clone Plate=2170 Col=24 Row=G, genomic survey sequence.//5.6e-06:162:67//AQ125590
F-PLACE1007409//Homo sapiens mitoxantrone resistance protein 2 mRNA, complete sequence.//1.6e-25:165:93//AF093772
F-PLACE1007416
F-PLACE1007450//Homo sapiens DNA from chromosome 19, BAC 33152, complete sequence.//4.9e-34:764:62//AC003973
F-PLACE1007452//Mus musculus bet3 (Bet3) mRNA, complete cds.//4.1e-17:374:64//AF041433
F-PLACE1007454//Homo sapiens (clone s153) mRNA fragment.//8.1e-52:317:93//L40391
F-PLACE1007460//Human DNA sequence from clone 914P14 on chromosome Xq23 Contains calpain-like protease gene, DCX (doublecortin) ESTs, CA repeat, GSS, complete sequence.//0.0019:280:64//AL031117
F-PLACE1007478//Homo sapiens Chromosome 16 BAC clone CIT987-SKA-345G4 -complete genomic sequence, complete sequence.//2.5e-24:362:71//AC002302
F-PLACE1007484
F-PLACE1007488//Danio rerio faciogenital dysplasia protein (fgd) mRNA, complete cds.//3.8e-14:293:63//AF017370
F-PLACE1007507//Human DNA sequence from clone 105D16 on chromosome Xp11.3-11.4 Contains pseudogene similar to laminin-binding protein, CA repeat, STS, complete sequence.//4.6e-10:152:75//AL031311
F-PLACE1007511//Homo sapiens chromosome 17, clone hRPC.1110_E_20, complete sequence.//3.6e-139:477:98//AC004231
F-PLACE1007524//Plasmodium falciparum microsatellite 14C sequence.//0.0055:395:59//AF015461
F-PLACE1007525//Trypanoplasma borelli mitochondrion cytochrome oxidase subunit 1 (cox1), cytochrome oxidase subunit 2 (cox2), and apocytochrome b (cytb) genes, complete cds, and complete 9S rRNA gene and partial 12S rRNA gene.//0.0013:550:58//U11682 F-PLACE1007537//H.sapiens CpG island DNA genomic Mse1 fragment, clone 198g6, reverse read cpg198g6.rt1a.//0.98:121:67//Z60280
F-PLACE1007544//Mus musculus chromosome 14 marker um-m24 GA dinucleotide DNA sequence.//2.3e-10:141:75//U31508
F-PLACE1007547//Homo sapiens mRNA for KIAA0661 protein, complete cds.//3.1e-69:733:71//AB014561
F-PLACE1007557//Drosophila yakuba mitochondrial DNA molecule.//0.022:393:61//X03240
F-PLACE1007583//Human DNA sequence *** SEQUENCING IN PROGRESS *** from clone 545L17, WORKING DRAFT SEQUENCE.//3.6e-114:565:97//AL031665
F-PLACE1007598//CIT-HSP-2371G14.TF CIT-HSP Homo sapiens genomic clone 2371G14, genomic survey sequence.//2.0e-22:304:70//AQ111183
F-PLACE1007618//Homo sapiens chromosome 17, clone hRPK.642_C_21, complete sequence.//1.0:386:59//AC005245
F-PLACE1007621
F-PLACE1007632//Homo sapiens 12p13.3 PAC RPCI5-940J5 (Roswell Park Cancer Institute Human PAC Library) complete sequence.//1.0e-88:276:96//AC006064
F-PLACE1007645//Bovine elastin mRNA, partial cds.//2.1e-07:110:79//M26132
F-PLACE1007649
F-PLACE1007677//Human DNA sequence *** SEQUENCING IN PROGRESS *** from clone 968D22, WORKING DRAFT SEQUENCE.//1.2e-21:567:64//AL023755
F-PLACE1007688//Pseudorabies virus immediate-early gene.//2.2e-05:287:66//X15120
F-PLACE1007690//Caenorhabditis elegans cosmid R07G3.//0.40:122:70//U23452
F-PLACE1007697//Mus musculus LIM/homeobox (Lhx3) gene fragment.//0.85:117:71//L40483
F-PLACE1007705//Human DNA sequence *** SEQUENCING IN PROGRESS *** from clone 460J8, WORKING DRAFT SEQUENCE.//0.0035:75:88//AL031662
F-PLACE1007706//Homo sapiens metalloprotease 1 (MP1) mRNA, complete cds.//1.3e-147:709:97//AF061243
F-PLACE1007725//Arabidopsis thaliana genomic DNA, chromosome 5, P1 clone: MBB18, complete sequence.//1.0:510:58//AB005231
F-PLACE1007729//Human endogenous retrovirus HML6 proviral clone HML6p, putative leader region, gag, pro and pol pseudogenes.//4.8e-136:516:89//U86698
F-PLACE1007730//Homo sapiens mRNA for KIAA0685 protein, complete cds.//7.9e-155:728:98//AB014585
F-PLACE1007737//Homo sapiens clone DJ0847O08, WORKING DRAFT SEQUENCE, 3 unordered pieces.//5.8e-22:806:60//AC005484
F-PLACE1007743//Plasmodium falciparum 3D7 chromosome 12 PFYAC1383 genomic sequence, WORKING DRAFT SEQUENCE, 3 unordered pieces.//1.1e-06:510:56//AC005504
F-PLACE1007746//HS_2268_B1_G10_MF CIT Approved Human Genomic Sperm Library D Homo sapiens genomic clone Plate=2268 Col=19 Row=N, genomic survey sequence.//0.10:171:63//AQ124780
F-PLACE1007791//Plasmodium falciparum DNA *** SEQUENCING IN PROGRESS *** from MAL1P6, WORKING DRAFT SEQUENCE.//0.63:241:58//AL031749
F-PLACE1007807//Homo sapiens chromosome 17, clone hRPK.879_D_6, complete sequence.//1.0e-120:743:87//AC005273
F-PLACE1007810//Homo sapiens Xp22 BAC GS-607H18 (Genome Systems Human BAC library) complete sequence.//1.0e-113:739:86//AC003658
F-PLACE1007829//CIT-HSP-2383J22.TR CIT-HSP Homo sapiens genomic clone 2383J22, genomic survey sequence.//1.0e-47:254:97//AQ196438
F-PLACE1007843//F.rubripes GSS sequence, clone 162K02bC12, genomic survey sequence.//1.6e-10:148:72//AL006903
F-PLACE1007846//Homo sapiens genomic DNA, chromosome 21q22.2 (Down Syndrome region), segment 3/15, WORKING DRAFT SEQUENCE.//3.4e-177:844:98//AP000010
F-PLACE1007852//Mouse perlecan mRNA, complete cds.//8.5e-39:243:90//M77174
F-PLACE1007858//Homo sapiens mRNA for KIAA0766 protein, complete cds.//3.9e-189:894:98//AB018309
F-PLACE1007866//CIT-HSP-2353D11.TF.1 CIT-HSP Homo sapiens genomic clone 2353D11, genomic survey sequence.//0.015:279:61//AQ263271
F-PLACE1007877
F-PLACE1007897
F-PLACE1007908//Homo sapiens mRNA, chromosome 1 specific transcript KIAA0487.//2.3e-154:755:97//AB007956
F-PLACE1007946//Human chromosome Y cosmid 56B5 genomic sequence, WORKING DRAFT SEQUENCE.//1.1e-59:310:81//AC003097
F-PLACE1007954//Homo sapiens BAC clone NH0414C23 from Y, complete sequence.//2.1e-61:522:79//AC006157
F-PLACE1007955//Homo sapiens cyclin-D binding Myb-like protein mRNA, complete cds.//2.7e-171:813:98//AF084530
F-PLACE1007958//Homo sapiens cAMP-specific phosphodiesterase 8B (PDE8B) mRNA, partial cds.//2.5e-153:730:98//AF079529
F-PLACE1007969//Mus musculus myelin gene expression factor (MEF-2) mRNA, partial cds.//3.4e-32:383:74//U13262
F-PLACE1007990//H.sapiens genomic DNA fragment (clone J31A212R).//6.6e-35:198:96//Z94758
F-PLACE1008000//Mus musculus veli 3 mRNA, complete cds.//1.5e-118:706:88//AF087695
F-PLACE1008002//Homo sapiens clone DJ0613C23, WORKING DRAFT SEQUENCE, 4 unordered pieces.//6.4e-163:786:98//AC005628
F-PLACE1008044//Rattus norvegicus nuclear pore complex protein NUP107 mRNA, complete cds.//1.2e-95:625:84//L31840
F-PLACE1008045//Caenorhabditis elegans cosmid F17C8, complete sequence.//0.016:165:65//Z35719
F-PLACE1008080//Human DNA sequence from cosmid L118G10, Huntington's Disease Region, chromosome 4p16.3.//4.0e-07:251:64//Z68883
F-PLACE1008095//RPCI11-21F19.TP RPCI-11 Homo sapiens genomic clone RPCI-11-21F19, genomic survey sequence.//1.5e-30:166:99//B85883
F-PLACE1008111//Aphidius picipes NADH dehydrogenase 1 gene, mitochondrial gene encoding mitochondrial protein, partial cds.//7.5e-06:414:60//AF069163
F-PLACE1008122//S.cerevisiae chromosome XV reading frame ORF YOL125w.//0.046:477:59//Z74867
F-PLACE1008129//Human Chromosome 15q26.1 PAC clone pDJ290i21 containing fur, fes, and alpha mannosidase IIx genes, WORKING DRAFT SEQUENCE, 9 unordered pieces.//0.0068:446:57//AC004586
F-PLACE1008132//Human DNA sequence *** SEQUENCING IN PROGRESS *** from clone 316D5, WORKING DRAFT SEQUENCE.//3.6e-20:111:93//Z82199
F-PLACE1008177//Mouse mRNA for meiosis-specific nuclear structural protein 1 (MNS1), complete cds.//2.5e-88:866:73//D14849
F-PLACE1008181//Human DNA sequence *** SEQUENCING IN PROGRESS *** from clone 159A1, WORKING DRAFT SEQUENCE.//0.0033:727:56//AL034397
F-PLACE1008198//HS_3073_A1_C06_MR CIT Approved Human Genomic Sperm Library D Homo sapiens genomic clone Plate=3073 Col=11 Row=E, genomic survey sequence.//2.3e-12:94:92//AQ171450
F-PLACE1008201//Homo sapiens clone RG140B11, WORKING DRAFT SEQUENCE, 1 unordered pieces.//2.5e-162:791:97//AC005069
F-PLACE1008209
F-PLACE1008231//Mouse testis-specific protein mRNA, complete cds.//0.65:174:66//M26332
F-PLACE1008244//CIT-HSP-2337B4.TR CIT-HSP Homo sapiens genomic clone 2337B4, genomic survey sequence.//6.7e-28:165:95//AQ039317
F-PLACE1008273//B.primigenius mRNA for coat protein gamma-cop.//2.8e-71:709:71//X92987
F-PLACE1008275//D.discoideum actin A-13 gene, 5' flank.//0.12:131:64//M29123
F-PLACE1008280//Homo sapiens Xp22-175-176 BAC GSHB-484O17 (Genome Systems Human BAC Library) complete sequence.//0.011:96:73//AC005913
F-PLACE1008309//Rattus norvegicus putative four repeat ion channel mRNA, complete cds.//8.2e-86:672:77//AF078779
F-PLACE1008329//HS_2027_A1_C06_T7 CIT Approved Human Genomic Sperm Library D Homo sapiens genomic clone Plate=2027 Col=11 Row=E, genomic survey sequence.//8.7e-09:116:81//AQ244432
F-PLACE1008330//Homo sapiens chromosome 19, cosmid F21431, complete sequence.//2.2e-141:670:98//AC005176
F-PLACE1008331//Homo sapiens clone DJ241P17, WORKING DRAFT SEQUENCE, 7 unordered pieces.//2.1e-27:157:78//AC005000
F-PLACE1008356//Homo sapiens mRNA for KIAA0679 protein, partial cds.//1.1e-137:659:98//AB014579
F-PLACE1008368//CIT-HSP-2311C9.TR CIT-HSP Homo sapiens genomic clone 2311C9, genomic survey sequence.//7.1e-08:398:60//AQ016352
F-PLACE1008369//HS_2251_B1_A02_MF CIT Approved Human Genomic Sperm Library D Homo sapiens genomic clone Plate=2251 Col=3 Row=B, genomic survey sequence.//2.1e-35:217:93//AQ066512
F-PLACE1008392//Homo sapiens chromosome 17, clone hRPK.136_H_19, complete sequence.//1.4e-11:403:64//AC005856
F-PLACE1008398//Human DNA sequence *** SEQUENCING IN PROGRESS *** from clone 215D11, WORKING DRAFT SEQUENCE.//3.7e-144:681:99//AL034417
F-PLACE1008401//*** SEQUENCING IN PROGRESS *** Homo sapiens chromosome 4, BAC clone C0366H07; HTGS phase 1, WORKING DRAFT SEQUENCE, 28 unordered pieces.//2.8e-45:257:96//AC004604
F-PLACE1008402//Homo sapiens mRNA for p115, complete cds.//4.3e-148:711:98//D86326
F-PLACE1008405//Plasmodium falciparum 3D7 chromosome 12 PFYAC69 genomic sequence, WORKING DRAFT SEQUENCE, 4 unordered pieces.//0.089:672:56//AC004688
F-PLACE1008424
F-PLACE1008426//Homo sapiens genomic DNA of 8p21.3-p22 anti-oncogene of hepatocellular colorectal and non-small cell lung cancer , segment 7/11.//1.0e-88:331:84//AB020864
F-PLACE1008429//Chromosome 22q13 BAC Clone CIT987SK-384D8 complete sequence.//0.55:530:58//U62317
F-PLACE1008437//CIT-HSP-2376H4.TR CIT-HSP Homo sapiens genomic clone 2376H4, genomic survey sequence.//3.3e-78:349:94//AQ112479
F-PLACE1008455//HS_2064_B1_E09_MR CIT Approved Human Genomic Sperm Library D Homo sapiens genomic clone Plate=2064 Col=17 Row=J, genomic survey sequence.//4.7e-59:471:81//AQ246589
F-PLACE1008457//Homo sapiens chromosome 17, Neurofibromatosis 1 locus, complete sequence.//8.9e-43:307:73//AC004526
F-PLACE1008465//CIT-HSP-2163F24.TR CIT-HSP Homo sapiens genomic clone 2163F24, genomic survey sequence.//8.9e-41:210:99//B90014
F-PLACE1008488//Mus musculus mRNA for testis-specific protein kinase 1, complete cds.//0.00013:516:58//AB003494
F-PLACE1008524//Human DNA sequence *** SEQUENCING IN PROGRESS *** from clone 34B21, WORKING DRAFT SEQUENCE.//1.3e-161:778:98//AL031778
F-PLACE1008531//Homo sapiens wbscr1 (WBSCR1) and replication factor C subunit 2 (RFC2) genes, complete cds.//1.1e-78:191:100//AF045555
F-PLACE1008532//Human DNA sequence *** SEQUENCING IN PROGRESS *** from clone 92N15, WORKING DRAFT SEQUENCE.//3.8e-24:257:70//Z93097
F-PLACE1008533//Homo sapiens PAC clone DJ130H16 from 22q12.1-qter, complete sequence.//1.0e-13:215:71//AC004997
F-PLACE1008568//Human DNA sequence from PAC 388N15 on chromosome Xq21.1.//0.66:263:64//Z99571
F-PLACE1008584//Homo sapiens cosmid clone U39B3 from Xp22.1-22.2, complete sequence.//1.1e-19:315:68//U73023
F-PLACE1008603//Homo sapiens mRNA for KIAA0791 protein, complete cds.//1.2e-173:812:98//AB018334
F-PLACE1008621//Homo sapiens clone RG228D17, WORKING DRAFT SEQUENCE, 2 unordered pieces.//3.9e-09:198:71//AC005077
F-PLACE1008625//Homo sapiens chromosome 5, PAC clone 45L14 (LBNL H91), complete sequence.//0.68:568:59//AC005373
F-PLACE1008626//HS_3221_A2_F03_T7 CIT Approved Human Genomic Sperm Library D Homo sapiens genomic clone Plate=3221 Col=6 Row=K, genomic survey sequence.//1.7e-13:147:82//AQ180967
F-PLACE1008627//Cricetulus griseus mRNA for Zn finger factor.//9.7e-98:586:88//Y12836
F-PLACE1008629//CIT-HSP-2012I4.TR CIT-HSP Homo sapiens genomic clone 2012I4, genomic survey sequence.//0.00085:203:66//B53732
F-PLACE1008630//Sequence 26 from Patent WO9517522.//9.7e-05:97:80//A45356
F-PLACE1008643//Human mRNA for inter-alpha-trypsin inhibitor family heavy chain-related protein (IHRP), complete cds.//1.4e-23 :299:64//D38595
F-PLACE1008650//Homo sapiens pleiotropic regulator 1 (PLRG1) mRNA, complete cds.//1.1e-133:622:99//AF044333
F-PLACE1008693//CIT-HSP-2346F2.TF CIT-HSP Homo sapiens genomic clone 2346F2, genomic survey sequence.//0.24:89:76//AQ060732
F-PLACE1008696//Homo sapiens NADH dehydrogenase-ubiquinone Fe-S protein 8 23 kDa subunit (NDUFS8) gene, nuclear gene encoding mitochondrial protein, complete cds.//1.4e-94:420:97//AF038406
F-PLACE1008715//CIT-HSP-2294K20.TR CIT-HSP Homo sapiens genomic clone 2294K20, genomic survey sequence.//2.1e-70:349:98//AQ007199
F-PLACE1008748//Arabidopsis thaliana chromosome I BAC T14N5 genomic sequence, complete sequence.//0.14:347:59//AC004260
F-PLACE1008757//Homo sapiens Xp22 BAC GSHB 526D21 (Genome Systems Human BAC library) complete sequence.//7.9e-25 :244:71//AC003037
F-PLACE1008790//Homo sapiens importin alpha 7 subunit mRNA, complete cds.//4.5e-120:503:97//AF060543
F-PLACE1008798//Human Chromosome 16 BAC clone CIT987SK-A-270G1, complete sequence.//0.00026:370:61//AF001549
F-PLACE1008807//CIT-HSP-2334B19.TF CIT-HSP Homo sapiens genomic clone 2334B19, genomic survey sequence.//3.3e-08:220:65//AQ036643
F-PLACE1008808//Homo sapiens exonuclease homolog RAD1 (RAD1) mRNA, complete cds.//1.7e-120:470:97//AF030933
F-PLACE1008813//Rattus norvegicus rsec15 mRNA, complete cds.//2.8e-87:504:89//AF032668
F-PLACE1008851//Homo sapiens DNA sequence from PAC 163M9 on chromosome 1p35.1-p36.21. Contains protein synthesis factor (eIF-4C), D1F15S1A pseudogene, ESTs, STS, GSS, complete sequence.//4.0e-21:212:74//AL021920
F-PLACE1008854
F-PLACE1008867//Human DNA sequence from clone J428A131, WORKING DRAFT SEQUENCE.//4.7e-77:477:84//Z82209
F-PLACE1008887//Homo sapiens BAC clone NH0335J18 from 2, complete sequence.//3.4e-53:699:70//AC005539
F-PLACE1008902//Mouse G-alpha-13 protein mRNA, complete cds.//2.1e-06:164:68//M63660
F-PLACE1008920//Homo sapiens mRNA for KIAA0765 protein, partial cds.//6.4e-158:753:98//AB018308
F-PLACE1008925//Homo sapiens chromosome 16p11.2 BAC clone CIT987SK-A-180G2, WORKING DRAFT SEQUENCE, 5 unordered pieces.//0.00013:400:63//AC002042
F-PLACE1008934//Human DNA sequence *** SEQUENCING IN PROGRESS *** from clone 1104E15, WORKING DRAFT SEQUENCE.//7.4e-05:145:71//AL022312
F-PLACE1008941//Human zinc finger protein (ZNF141) mRNA, complete cds.//4.3e-41:282:87//L15309
F-PLACE1008947//Pseudorabies virus with upstream and downsteam sequences.//5.9e-15:710:60//M34651
F-PLACE1009020//HS_3051_B1_H01_MR CIT Approved Human Genomic Sperm Library D Homo sapiens genomic clone Plate=3051 Col=1 Row=P, genomic survey sequence.//1.9e-21:167:86//AQ253727
F-PLACE1009027//Human DNA sequence from clone 914P14 on chromosome Xq23 Contains calpain-like protease gene, DCX (doublecortin) ESTs, CA repeat, GSS, complete sequence.//4.1e-152:763:97//AL031117
F-PLACE1009039//HS_2034_A2_F08_T7 CIT Approved Human Genomic Sperm Library D Homo sapiens genomic clone Plate=2034 Col=16 Row=K, genomic survey sequence.//0.17:252:59//AQ230137
F-PLACE1009045//HS_3185_B2_B03_T7 CIT Approved Human Genomic Sperm Library D Homo sapiens genomic clone Plate=3185 Col=6 Row=D, genomic survey sequence.//1.9e-34:260:86//AQ172861
F-PLACE1009048//Pig pituitary glycoprotein hormone alpha subunit gene, 5'flank and exon 1.//4.7e-70:463:80//D00766
F-PLACE1009050//Homo sapiens 12q13.1 PAC RPCI3-197B17 (Roswell Park Cancer Institute Human PAC library) complete sequence.//0.63:280:61//AC004241
F-PLACE1009060//Mus musculus mRNA for Alix (ALG-2-interacting protein X), complete CDS.//5.9e-113:725:85//AJ005073
F-PLACE1009090//Human DNA sequence *** SEQUENCING IN PROGRESS *** from clone 1045J21, WORKING DRAFT SEQUENCE.//9.1e-27:222:84//AL021919
F-PLACE1009091//Homo sapiens clone DJ0968I16, complete sequence.//0.027:630:58//AC006016
F-PLACE1009094
F-PLACE1009099//Mouse zinc finger protein (mkr4) mRNA, partial cds.//2.1e-85:726:76//M36515
F-PLACE1009110
F-PLACE1009111//Human DNA sequence *** SEQUENCING IN PROGRESS *** from clone 138B7, WORKING DRAFT SEQUENCE.//6.0e-12:362:64//Z98752
F-PLACE1009113//Homo sapiens X-ray repair cross-complementing protein 3 (XRCC3) mRNA, complete cds.//3.4e-138:671:97//AF035586
F-PLACE1009130//Human mRNA for KIAA0032 gene, complete cds.//3.6e-23:718:59//D25215
F-PLACE1009150//Homo sapiens *** SEQUENCING IN PROGRESS ***, WORKING DRAFT SEQUENCE.//6.1e-142:684:98//AJ011929
F-PLACE1009155//Homo sapiens genomic DNA, chromosome 21q11.1, segment 2/28, WORKING DRAFT SEQUENCE.//4.3e-36:227:77//AP000031
F-PLACE1009158//H.sapiens genomic sequence for ERCC2 gene 3'region involved in DNA excision repair.//1.0:173:60//X52222
F-PLACE1009166
F-PLACE1009172//Human BAC clone 7E17 from 12q, complete sequence.//4.0e-35:257:85//AC002070
F-PLACE1009174//Homo sapiens Xp22 bins 16-17 BAC GSHB-531I17 (Genome Systems Human BAC Library) complete sequence.//2.9e-19:288:72//AC004805
F-PLACE1009183//Arabidopsis thaliana genomic DNA, chromosome 5, P1 clone: MHJ24, complete sequence.//0.053:388:60//AB008266
F-PLACE1009186//Rattus norvegicus fracture callus 1 (FxC1) mRNA, complete cds.//1.8e-50:317:89//AF061242
F-PLACE1009190//RPCI11-81N5.TJ RPCI11 Homo sapiens genomic clone R-81N5, genomic survey sequence.//0.91:114:67//AQ281881
F-PLACE1009200//CITBI-E1-2509J16.TF CITBI-E1 Homo sapiens genomic clone 2509J16, genomic survey sequence.//2.8e-44:175:83//AQ262198
F-PLACE1009230//H.sapiens gene for pregnancy specific beta-1 glycoprotein.//1.1e-106:495:88//X63203
F-PLACE1009246//HS_3058_B1_A06_MF CIT Approved Human Genomic Sperm Library D Homo sapiens genomic clone Plate=3058 Col=11 Row=B, genomic survey sequence.//0.10:175:68//AQ185945
F-PLACE1009298//Mus musculus maternal-embryonic 3 (Mem3) mRNA, complete cds.//1.8e-94:575:89//U47024
F-PLACE1009308//Human clone mcag32 chromosome 7 CTG repeat region.//0.0017:350:62//U23862
F-PLACE1009319//Homo sapiens post-synaptic density protein 95 (PSD95) mRNA, complete cds.//3.0e-06:411:59//U83192
F-PLACE1009328//Human DNA sequence *** SEQUENCING IN PROGRESS *** from clone 191P20, WORKING DRAFT SEQUENCE.//5.7e-138:830:86//AL034399
F-PLACE1009335//Human (lambda) DNA for immunoglobin light chain.//0.071:253:62//D87015
F-PLACE1009338//RPCI11-74N24 TV RPCI11 Homo sapiens genomic clone R-74N24, genomic survey sequence.//2.4e-34:180:100//AQ268811
F-PLACE1009368
F-PLACE1009375
F-PLACE1009388//Human DNA sequence *** SEQUENCING IN PROGRESS *** from clone 1014D13, WORKING DRAFT SEQUENCE.//2.0e-37:288:84//AL022311
F-PLACE1009398//Human DNA binding protein (HPF2) mRNA, complete cds.//4.3e-78:730:74//M27878
F-PLACE1009404//SmD homolog [mice, liver, mRNA Partial, 199 nt].//0.16:95:71//S71494
F-PLACE1009410//Homo sapiens chromosome 17, clone hRPK.142_H_19, complete sequence.//1.6e-150:701:99//AC005919
F-PLACE1009434//Mus musculus clone OST431, genomic survey sequence.//2.9e-73:442:88//AF046700
F-PLACE1009443//Mycobacterium tuberculosis H37Rv complete genome; segment 148/162.//0.012:582:56//AL022022
F-PLACE1009444//Homo sapiens phosphatidylinositol 4-kinase 230 (pi4K230) mRNA, complete cds.//4.6e-21:146:93//AF012872
F-PLACE1009459//Mus musculus clone OST9217, genomic survey sequence.//2.9e-31:264:81//AF046660
F-PLACE1009468//Sequence 1 from patent US 5580968.//1.9e-83:567:84//I30536
F-PLACE1009476//Homo sapiens Chromosome 16 BAC clone CIT987SK-A-67A1, complete sequence.//1.9e-142:704:97//AC004531
F-PLACE1009477//Human 11p14.3 PAC clone pDJ939m16, complete sequence.//2.2e-09:235:68//AC004601
F-PLACE1009493//Human Chromosome 16 BAC clone CIT987SK-A-363E6, complete sequence.//2.9e-83:171:92//U91321
F-PLACE1009524//Homo sapiens DNA sequence from PAC 63G5 on chromosome 22q12.3-13.1. Contains part of a gene for a human SEC7 homolog B2-1 (cytohesin-2, Arno, ARF exchange factor) LIKE protein, an unknown gene and a gene coding for a Leucine rich protein. Contains ESTs, STSs and GSSs, complete sequence.//3.8e-69:175:92//Z94160
F-PLACE1009539//Mus musculus synaptojanin 2 isoform alpha mRNA, complete cds.//7.0e-26:237:78//AF041862
F-PLACE1009542//Human DNA sequence from clone 1039K5 on chromosome 22q12.3-13.2 Contains gene similar to PICK1 perinuclear binding protein, gene similar to monocarboxylate transporter (MCT3), ESTs, STS, GSS and a CpG island, complete sequence.//3.1e-10:126:79//AL031587
F-PLACE1009571//RPCI11-60K12.TK RPCI11 Homo sapiens genomic clone R-60K12, genomic survey sequence.//1.4e-05:68:91//AQ195869
F-PLACE1009581
F-PLACE1009595//Homo sapiens chromosome 5, P1 clone 1029A7 (LBNL H15), complete sequence.//6.6e-19:309:70//AC003959
F-PLACE1009596//Rattus norvegicus platelet-activating factor acetylhydrolase beta subunit (PAF-AH beta) gene, complete cds.//9.0e-09:485:59//AF016049
F-PLACE1009607//Human DNA sequence *** SEQUENCING IN PROGRESS *** from clone 409J21, WORKING DRAFT SEQUENCE.//4.9e-43:714:66//Z83824
F-PLACE1009613//Plasmodium falciparum 3D7 chromosome 12 PFYAC293 genomic sequence, WORKING DRAFT SEQUENCE, 9 unordered pieces.//0.017:655:57//AC004157
F-PLACE1009621
F-PLACE1009622//HS-1016-B2-E08-MF.abi CIT Human Genomic Sperm Library C Homo sapiens genomic clone Plate=CT 791 Col=16 Row=J, genomic survey sequence.//2.7e-15:100:98//B33248
F-PLACE1009637//Plasmodium falciparum 3D7 chromosome 12 PFYAC492 genomic sequence, WORKING DRAFT SEQUENCE, 5 unordered pieces.//0.63:130:67//AC005308
F-PLACE1009639//S.pombe chromosome II cosmid c24E9.//0.86:509:58//AL021816
F-PLACE1009659//Homo sapiens mRNA for KIAA0587 protein, complete cds.//1.4e-171:816:98//AB011159
F-PLACE1009665//Homo sapiens chromosome 17, clone HCIT462L7, complete sequence.//3.4e-67:437:87//AC005177
F-PLACE1009670//Homo sapiens genethonin 1 mRNA, complete cds.//2.5e-147:701:98//AF062534
F-PLACE1009708//Homo sapiens clone DJ0935K16, complete sequence.//1.5e-98:228:100//AC006011
F-PLACE1009721//Human Cosmid g0771a222 from 7q31.3, complete sequence.//2.2e-130:736:91//AC000109
F-PLACE1009731//M.musculus mRNA for immunity associated protein 38.//1.1e-13:311:64//Y08026
F-PLACE1009763//Homo sapiens UBA3 (UBA3) mRNA, complete cds.//4.2e-125:602:98//AF046024
F-PLACE1009794
F-PLACE1009798//Hnman DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs, an STS and GSSs, complete sequence.//1.3e-73:271:84//AL030996
F-PLACE1009845
F-PLACE1009861//B.tauris cathepsin B mRNA, 3' end.//0.00023:147:65//M64620
F-PLACE1009879//Human DNA sequence *** SEQUENCING IN PROGRESS *** from clone 159A1, WORKING DRAFT SEQUENCE.//4.9e-27:725:63//AL034397
F-PLACE1009886//Human DNA sequence *** SEQUENCING IN PROGRESS *** from clone 167A19, WORKING DRAFT SEQUENCE.//8.2e-12:135:82//AL031427
F-PLACE1009888//F14G3-T7 IGF Arabidopsis thaliana genomic clone F14G3, genomic survey sequence.//0.0044:232:60//AQ251431
F-PLACE1009908//S.pombe chromosome I cosmid c3F10.//1.5e-19:559:59//Z69369
F-PLACE1009921//Homo sapiens cosmid clone HDAB (1S149) insert DNA, complete cosmid.//5.9e-48:304:87//M63005
F-PLACE1009924//Homo sapiens chromosome 16p11.2 BAC clone CIT987SK-2011O4, WORKING DRAFT SEQUENCE, 4 unordered pieces.//2.4e-51:481:78//AC004529
F-PLACE1009925//nbxb0027C22r CUGI Rice BAC Library Oryza sativa genomic clone nbxb0027C22r, genomic survey sequence.//0.98:220:67//AQ272066
F-PLACE1009935//Sequence 16 from patent US 5552281.//0.030:152:67//I25655
F-PLACE1009947//Homo sapiens clone GS096J14, WORKING DRAFT SEQUENCE, 3 unordered pieces.//2.6e-12:322:67//AC006026
F-PLACE1009971
F-PLACE1009992//HS_3178_B1_F04_T7 CIT Approved Human Genomic Sperm Library D Homo sapiens genomic clone Plate=3178 Col=7 Row=L, genomic survey sequence.//4.9e-23:142:95//AQ150311
F-PLACE1009995//Caenorhabditis elegans cosmid C01A2, complete sequence.//0.00019:231:64//Z81029
F-PLACE1009997//Rattus norvegicus A-kinase anchoring protein AKAP 220 mRNA, complete cds.//7.9e-87:552:80//U48288
F-PLACE1010023
F-PLACE1010031//Human DNA sequence from clone 30M3 on chromosome 6p22.1-22.3. Contains three novel genes, one similar to C. elegans Y63D3A.4 and one similar to (predicted) plant, worm, yeast and archaea bacterial genes, and the first exon of the KIAA0319 gene. Contains ESTs, GSSs and putative CpG islands, complete sequence.//6.9e-101:181:98//AL031775
F-PLACE1010053//M.musculus Spnr mRNA for RNA binding protein.//2.3e-136:689:95//X84692
F-PLACE1010069//Human DNA sequence *** SEQUENCING IN PROGRESS *** from clone 212A2, WORKING DRAFT SEQUENCE.//0.0090:383:60//Z95114
F-PLACE1010074//Homo sapiens sorting nexin 2 (SNX2) mRNA, complete cds.//1.8e-166:792:98//AF065482
F-PLACE1010076//Mouse mRNA for TGF-beta type I receptor, complete cds.//7.5e-13:203:77//D25540
F-PLACE1010083//Homo sapiens mRNA for KIAA0456 protein, partial cds.//3.0e-152:727:98//AB007925
F-PLACE1010089//HS_3111_A1_E08_T7 CIT Approved Human Genomic Sperm Library D Homo sapiens genomic clone Plate=3111 Col=15 Row=I, genomic survey sequence.//4.8e-07:124:78//AQ101268
F-PLACE1010096//R.norvegicus mRNA for 100 kDa protein.//1.2e-108:700:85//X64411
F-PLACE1010102//Plasmodium falciparum 3D7 chromosome 12 PFYAC357 genomic sequence, WORKING DRAFT SEQUENCE, 7 unordered pieces.//2.1e-07:476:60//AC005506
F-PLACE1010105//Homo sapiens actin binding protein MAYVEN mRNA, complete cds.//3.8e-25:728:60//AF059569
F-PLACE1010106//Human DNA sequence from PAC 127B14 on chromosome Xq22.//6.5e-25:488:63//Z93928
F-PLACE1010134//S.pombe chromosome I cosmid c29B12.//1.9e-13:238:67//Z99164
F-PLACE1010148//Homo sapiens partial human cDNA (660 bp).//4.8e-83 :409:98//AJ222636
F-PLACE1010152//CIT-HSP-2381F24.TF CIT-HSP Homo sapiens genomic clone 2381F24, genomic survey sequence.//1.5e-28:163:98//AQ196757
F-PLACE1010181//Homo sapiens PAC clone DJ1139I01 from Xq23, complete sequence.//2.4e-15:197:72//AC004973
F-PLACE1010194//Ictalurus punctatus tumor supressor p53 mRNA, complete cds.//3.0e-14:181:74//AF074967
F-PLACE1010202//Homo sapiens mRNA for MBNL protein.//1.2e-27:509:66//Y13829
F-PLACE1010231//Human DNA sequence *** SEQUENCING IN PROGRESS *** from clone 287G14, WORKING DRAFT SEQUENCE.//2.3e-101:194:95//AL033377
F-PLACE1010261//Homo sapiens mRNA for KIAA0448 protein, complete cds.//5.8e-145:693:97//AB007917
F-PLACE1010270//Plasmodium falciparum 3D7 chromosome 12 PFYAC588 genomic sequence; WORKING DRAFT SEQUENCE, 2 unordered pieces.//2.1e-05:347:60//AC004710
F-PLACE1010274//Caenorhabditis elegans cosmid C01A2, complete sequence.//0.00040:231:64//Z81029
F-PLACE1010293//Homo sapiens chromosome 2 PAC RPCI3-417E16 (Roswell Park Cancer Institute Human PAC library) complete sequence.//6.5e-25:344:70//AC004464
F-PLACE1010310//Homo sapiens DNA sequence from PAC 329E20 on chromosome 1p34.4-36.13. Contains endothelin-converting-enzyme 1 (ECE-1), EST, STS, CA repeat, complete sequence.//3.5e-l 0:185:67//AL031005
F-PLACE1010321//Human DNA sequence from clone 299D3 on chromosome 22q13.3, complete sequence.//0.010:524:58//Z84468
F-PLACE1010324//CIT-HSP-2335J21.TR CIT-HSP Homo sapiens genomic clone 2335J21, genomic survey sequence.//9.1e-90:448:97//AQ041837
F-PLACE1010329//Apis mellifera ligustica complete mitochondrial genome.//2.8e-08:384:64//L06178
F-PLACE1010341//HS-1047-A2-C04-MR.abi CIT Human Genomic Sperm Library C Homo sapiens genomic clone Plate=CT 830 Col=8 Row=E, genomic survey sequence.//4.1e-21:141:92//B38252
F-PLACE1010362//Mycobacterium tuberculosis H37Rv complete genome; segment 155/162.//0.94:398:57//AL022121
F-PLACE1010364//Caenorhabditis elegans DNA *** SEQUENCING IN PROGRESS *** from clone Y102G3, WORKING DRAFT SEQUENCE.//0.11:404:56//AL020985
F-PLACE1010383//Homo sapiens chromosome 17, clone hCIT.186_H_2, complete sequence.//0.066:88:76//AC004675
F-PLACE1010401//CIT-HSP-2367K17.TR CIT-HSP Homo sapiens genomic clone 2367K17, genomic survey sequence.//2.4e-71:454:88//AQ076825
F-PLACE1010481//Bos taurus C5-glucuronyl epimerase mRNA, partial cds.//7.5e-134:722:93//AF003927
F-PLACE1010491//Homo sapiens Cre binding protein-like 2 mRNA, complete cds.//2.2e-150:702:99//AF039081
F-PLACE1010492
F-PLACE1010522//Homo sapiens cosmid LM1937 from Xq28.//0.022:405:60//U82695
F-PLACE1010529//Sequence 1 from patent US 5776717.//2.9e-145 :684:98//AR016417
F-PLACE1010547//Human DNA sequence from clone 790B6 on chromosome 20p11.22-12.2. Contains STSs and GSSs, complete sequence.//1.0:283:61//AL031677
F-PLACE1010562//RPCI11-65I16.TK RPCI11 Homo sapiens genomic clone R-65I16, genomic survey sequence.//0.017:216:67//AQ200831
F-PLACE1010579//Homo sapiens full-length insert cDNA YI23D12.//3.9e-19:147:89//AF075014
F-PLACE1010580//Mouse RNA helicase and RNA-dependent ATPase from the DEAD box family mRNA, complete cds.//6.4e-96:559:89//L25125
F-PLACE1010599//Homo sapiens peroxisomal membrane anchor protein HsPex14p (PEX14) mRNA, complete cds.//3.1e-146:707:97//AF045186
F-PLACE1010616//Plasmodium falciparum 3D7 chromosome 12 PFYAC492 genomic sequence, WORKING DRAFT SEQUENCE, 5 unordered pieces.//0.045:454:59//AC005308
F-PLACE1010622//Plasmodium falciparum MAL3P2, complete sequence.//9.1e-07:378:60//AL034558
F-PLACE1010624//Streptomyces coelicolor cosmid 5A7.//1.4e-05:518:61//AL031107
F-PLACE1010628//Homo sapiens clone DJ0647C14, WORKING DRAFT SEQUENCE, 21 unordered pieces.//5.0e-137:675:97//AC004846
F-PLACE1010629//Homo sapiens Chromosome 16 BAC clone CIT987SK-A-259H10, complete sequence.//2.5e-17:187:80//AC004682
F-PLACE1010630//Arabidopsis thaliana genomic DNA, chromosome 5, TAC clone: K21P3, complete sequence.//0.21:159:64//AB016872
F-PLACE1010631//Homo sapiens clone RG140B11, WORKING DRAFT SEQUENCE, 1 unordered pieces.//1.2e-144:720:97//AC005069
F-PLACE1010661
F-PLACE1010662//Arabidopsis thaliana DNA chromosome 4, BAC clone F7J7 (ESSA project).//0.90:257:61//AL021960
F-PLACE1010702//Human repressor transcriptional factor (ZNF85) mRNA, complete cds.//3.3e-73:697:74//U35376
F-PLACE1010714//Human Chromosome 15q11-q13 PAC clone pDJ778a2, complete sequence.//0.010:447:59//AC004583
F-PLACE1010720//Mouse TPA-induced TIS11 mRNA.//2.0e-86:535:88//X14678
F-PLACE1010739//HS_2013_B2_B10_MR CIT Approved Human Genomic Sperm Library D Homo sapiens genomic clone Plate=2013 Col=20 Row=D, genomic survey sequence.//5.7e-87:435:97//AQ235864
F-PLACE1010743//R.norvegicus mRNA for myr5.//1.7e-87:582:85//X77609
F-PLACE1010761//Homo sapiens chromosome 17, clone hRPK.294_J_22, complete sequence.//4.7e-45:235:99//AC005921
F-PLACE1010771//M.musculus HCNGP mRNA.//1.6e-135:801:88//X68061
F-PLACE1010786//Plasmodium falciparum DNA *** SEQUENCING IN PROGRESS *** from contig 4-15, complete sequence.//0.35:334:60//AL010221
F-PLACE1010800//RPCI11-79H17.TV RPCI11 Homo sapiens genomic clone R-79H17, genomic survey sequence.//5.8e-18:168:82//AQ284252
F-PLACE1010802//Human Chromosome X clone bWXD531, complete sequence.//1.6e-30:693:63//AC004384
F-PLACE1010811//RPCI11-51N5.TK RPCI11 Homo sapiens genomic clone R-51N5, genomic survey sequence.//8.3e-11:142:78//AQ052380
F-PLACE1010833//Human DNA sequence *** SEQUENCING IN PROGRESS *** from clone 467K16, WORKING DRAFT SEQUENCE.//7.3e-40:147:88//AL031283
F-PLACE1010856//M.musculus mRNA for utrophin.//7.3e-17:150:86//Y12229
F-PLACE1010857//Homo sapiens genomic DNA of 8p21.3-p22 anti-oncogene of hepatocellular colorectal and non-small cell lung cancer, segment 11/11.//1.4e-94:422:95//AB020868
F-PLACE1010870//M.musculus mRNA for ZT3 zinc finger factor.//1.3e-93:530:90//Z67747
F-PLACE1010877//Homo sapiens mRNA for KIAA0610 protein, partial cds.//1.1e-147:694:98//AB011182
F-PLACE1010891
F-PLACE1010896//Mouse BAC mbac20 from 14D1-D2 (T-Cell Receptor Alpha Locus), complete sequence.//3.9e-26:394:68//AC003997
F-PLACE1010900
F-PLACE1010916//HS_2242_A1_C04_MF CIT Approved Human Genomic Sperm Library D Homo sapiens genomic clone Plate=2242 Col=7 Row=E, genomic survey sequence.//1.0e-78:391:97//AQ146687
F-PLACE1010917
F-PLACE1010925//Plasmodium falciparum 3D7 chromosome 12 PFYAC69 genomic sequence, WORKING DRAFT SEQUENCE, 4 unordered pieces.//0.11:629:56//AC004688
F-PLACE1010926//Homo sapiens mRNA for KIAA0554 protein, partial cds.//9.5e-138:653:98//AB011126
F-PLACE1010942//Homo sapiens intersectin short form mRNA, complete cds.//5.6e-90:437:98//AF064243
F-PLACE1010944//Homo sapiens full-length insert cDNA clone ZD38E12.//1.4e-09:208:68//AF086247
F-PLACE1010947
F-PLACE1010954//CIT-HSP-2283D9.TR CIT-HSP Homo sapiens genomic clone 2283D9, genomic survey sequence.//2.1e-29:190:91//B98965
F-PLACE1010960//Plasmodium falciparum DNA *** SEQUENCING IN PROGRESS *** from contig 4-52, complete sequence.//0.00074:421:60//AL010226
F-PLACE1010965//CIT-HSP-2386K24:TF.1 CIT-HSP Homo sapiens genomic clone 2386K24, genomic survey sequence.//1.8e-84:412:99//AQ240696
F-PLACE1011026//Plasmodium falciparum DNA *** SEQUENCING IN PROGRESS *** from contig 3-20, complete sequence.//0.00037:257:64//AL008972
F-PLACE1011032//Homo sapiens chromosome 5, BAC clone 118L13 (LBNL H176), complete sequence.//3.8e-06:315:65//AC005348
F-PLACE1011041//Human Fas-ligand associated factor 3 mRNA, partial cds.//1.5e-56:286:98//U70669
F-PLACE1011046//Rat phospholipase C-1 mRNA, complete cds.//1.3e-24:278:76//M20636
F-PLACE1011054//Human DNA sequence *** SEQUENCING IN PROGRESS *** from clone 981L23, WORKING DRAFT SEQUENCE.//3.8e-27:196:84//AL031686
F-PLACE1011056//Ovis aries bactinecin 11 (Bac11) gene, exon 4, and complete cds.//5.4e-06:182:67//U77049
F-PLACE1011057//protein kinase PRK2 [human, DX3 B-cell myeloma cell line, mRNA, 3255 nt].//3.2e-31:169:100//S75548
F-PLACE1011090//Human DNA sequence *** SEQUENCING IN PROGRESS *** from clone 998H6, WORKING DRAFT SEQUENCE.//5.1e-80:479:89//AL031687
F-PLACE1011109//Rattus norvegicus nuclear-encoded mitochondrial elongation factor G mRNA, complete cds.//2.3e-24:192:84//L14684
F-PLACE1011114//S.cerevisiae chromosome XI reading frame ORF YKR024c.//1.4e-14:346:60//Z28249
F-PLACE1011133//T7E9-T7.1 TAMU Arabidopsis thaliana genomic clone T7E9, genomic survey sequence.//0.010:345:60/B19698
F-PLACE1011143//CIT-HSP-2375J10.TR CIT-HSP Homo sapiens genomic clone 2375J10, genomic survey sequence.//0.00013:95:76//AQ109305
F-PLACE1011160//Homo sapiens PAC clone DJ0808A01 from 7q21.1-q31.1, complete sequence.//3.7e-111:692:87//AC004893
F-PLACE1011165//H.sapiens galactokinase (GK2) mRNA, complete cds.//8.4e-31:194:92//M84443
F-PLACE1011185//Homo sapiens Chromosome 16 BAC clone CIT987SK-A-249B10, complete sequence.//3.1e-43:447:72//AC002288
F-PLACE1011203//Homo sapiens chromosome 18q11 beta-1,4-galactosyltransferase mRNA, complete cds.//3.3e-124:584:99//AF038664
F-PLACE1011214//HS_2046_A2_B01_MR CIT Approved Human Genomic Sperm Library D Homo sapiens genomic clone Plate=2046 Col=2 Row=C, genomic survey sequence.//2.0e-39:346:81//AQ305965
F-PLACE1011219
F-PLACE1011221//CITBI-E1-2513F18.TR CITBI-E1 Homo sapiens genomic clone 2513F18, genomic survey sequence.//2.4e-20:119:100//AQ279801
F-PLACE1011229//Homo sapiens mRNA for KIAA0529 protein, partial cds.//4.4e-146:675:99//AB011101
F-PLACE1011263//Homo sapiens BAC clone GS166A23 from 7p21, complete sequence.//1.7e-42:212:84//AC005014
F-PLACE1011273//Caenorhabditis elegans DNA *** SEQUENCING IN PROGRESS *** from clone Y37D8, WORKING DRAFT SEQUENCE.//1.0:214:60//Z92819
F-PLACE1011291//RPCI11-16P9.TP RPCI-11 Homo sapiens genomic clone RPCI-11-16P9, genomic survey sequence.//8.0e-08:66:98//B81770
F-PLACE1011296//Homo sapiens chromosome 16, cosmid clone 443G8 (LANL), complete sequence.//0.027:135:67//AC004647
F-PLACE1011310//H.sapiens CpG island DNA genomic Mse1 fragment, clone 53c10, reverse read cpg53c10.rt1b.//1.4e-05:57:100//Z61496
F-PLACE1011325//Human immunodeficiency virus type 1 (D9) proviral structural capsid protein (gag) gene, partial cds.//0.077:193:60//L02290
F-PLACE1011332//Homo sapiens N-acetylglucosamine-phosphate mutase mRNA, complete cds.//3.1e-150:699:99//AF102265
F-PLACE1011340//Homo sapiens chromosome 17, clone hRPK.388_F_14, complete sequence.//2.4e-38:186:83//AC005375
F-PLACE1011371//Mus musculus PK-120 precursor (itih-4) mRNA, complete cds.//6.0e-35:689:63//AF023919
F-PLACE1011375//Mus musculus Kv3.4 gene, exon 4.//6.0e-88:584:86//AJ010310
F-PLACE1011399//paramecium species 7,325 mt dna dimer: replication init. region.//0.00011:255:63//K00919
F-PLACE1011419//Homo sapiens chromosome 21 PAC LLNLP704G1150Q13.//0.067:337:62//AJ006996
F-PLACE1011433//Homo sapiens mRNA for KIAA0530 protein, partial cds.//4.6e-157:743:98//AB011102
F-PLACE1011452//Homo sapiens *** SEQUENCING IN PROGRESS ***, WORKING DRAFT SEQUENCE.//1.1e-53:557:73//AJ011929
F-PLACE1011465//Homo sapiens Chromosome 16 BAC clone CIT987SK-A-248F7, complete sequence.//3.5e-71:498:80//AC004605
F-PLACE1011472//Homo sapiens mRNA for KIAA0712 protein, complete cds.//4.8e-151:703:99//AB018255
F-PLACE1011477//Homo sapiens sorting nexin 2 (SNX2) mRNA, complete cds.//5.2e-145:675:99//AF065482
F-PLACE1011492//Ray (T.californica) acetylcholine receptor beta-subunit mRNA.//1.0:448:59//J00964
F-PLACE1011503
F-PLACE1011520//Homo sapiens clone DJ1119N05, complete sequence.//3.8e-147:692:99//AC004968
F-PLACE1011563//R.norvegicus mRNA for leucocyte common antigen-related protein (3941 bp).//0.00036:296:61//X83546
F-PLACE1011567//Homo sapiens PAC clone DJ1164K10 from 7p21-p22, complete sequence.//1.1e-38:315:82//AC004984
F-PLACE1011576//Homo sapiens hematopoietic cell derived zinc finger protein mRNA, complete cds.//1.3e-65:268:86//AF054180
F-PLACE1011586//Homo sapiens chromosome 17, clone HRPC890E16, complete sequence.//2.0e-82:188:96//AC004477
F-PLACE1011635//Homo sapiens chromosome 17, clone hRPK.214_O_1, complete sequence.//1.8e-153:752:97//AC005224
F-PLACE1011641//Homo sapiens T-cell receptor alpha delta locus from bases 501613 to 752736 (section 3 of 5) of the Complete Nucleotide Sequence.//4.8e-05:190:67//AE000660
F-PLACE1011643//Alcaligenes eutrophus phaP gene.//0.16:466:59//X85729
F-PLACE1011646//Human DNA sequence *** SEQUENCING IN PROGRESS *** from clone 1013A10, WORKING DRAFT SEQUENCE.//9.1e-19:156:76//AL033383
F-PLACE1011649
F-PLACE1011650//Homo sapiens retinol dehydrogenase gene, complete cds.//6.4e-09:172:74//AF037062
F-PLACE1011664//D.melanogaster crn mRNA.//1.1e-52:650:68//X58374
F-PLACE1011675//Plasmodium falciparum 3D7 chromosome 12 PFYACB8-628 genomic sequence, WORKING DRAFT SEQUENCE, 9 unordered pieces.//0.11:443:58//AC005507
F-PLACE1011682//Human DNA sequence from clone 342B11 on chromosome 22q12.1-12.3. Contains ESTs and a GSS, complete sequence.//0.31:127:71//AL008719
F-PLACE1011719//Human BAC clone RG369K23 from 7q31, complete sequence.//4.6e-52:461:77//AC002487
F-PLACE1011725
F-PLACE1011729//Human Chromosome 15q11-q13 clone pDJ276c12 from the Prader-Willi/Angelman syndrome region, WORKING DRAFT SEQUENCE, 3 unordered pieces.//0.011:320:62//AC004737
F-PLACE1011749//Plasmodium falciparum 3D7 chromosome 12 PFYAC293 genomic sequence, WORKING DRAFT SEQUENCE, 9 unordered pieces.//0.00031:544:59//AC004157
F-PLACE1011762//Homo sapiens BAC clone RG437L15 from 8q21, complete sequence.//2.4e-115:682:90//AC004003
F-PLACE1011778//RPCI11-22D17.TVB RPCI-11 Homo sapiens genomic clone RPCI-11-22D17, genomic survey sequence.//2.7e-114:611:93//AQ008944
F-PLACE1011783//CIT-HSP-2317N1.TF CIT-HSP Homo sapiens genomic clone 2317N1, genomic survey sequence.//2.3e-17:120:94//AQ042330
F-PLACE1011858//Gallus domesticus filamin mRNA, complete cds.//4.1e-24:565:64//U00147
F-PLACE1011874//Homo Sapiens Chromosome X clone bWXD312, complete sequence.//2.5e-141:678:98//AC004478
F-PLACE1011875//Homo sapiens mRNA for KIAA0580 protein, partial cds.//1.6e-108:526:98//AB011152
F-PLACE1011891//Human DNA sequence *** SEQUENCING IN PROGRESS *** from clone 439F8, WORKING DRAFT SEQUENCE.//0.0014:330:62//AL021392
F-PLACE1011896//Mus musculus Wnt10a mRNA, complete cds.//1.4e-89:678:82//U61969
F-PLACE1011922//Caprine arthritis-encephalitis virus envelope glycoprotein (env) gene, partial cds.//0.069:246:61//U81400
F-PLACE1011923//Homo sapiens serum-inducible kinase mRNA, complete cds.//1.2e-138:664:98//AF059617
F-PLACE1011962//HS_3212_B2_G12_MR CIT Approved Human Genomic Sperm Library D Homo sapiens genomic clone Plate=3212 Col=24 Row=N, genomic survey sequence.//2.4e-07:154:74//AQ175369
F-PLACE1011964//Human DNA sequence *** SEQUENCING IN PROGRESS *** from clone 322P7, WORKING DRAFT SEQUENCE.//3.7e-22:369:69//AL023799
F-PLACE1011982//HS-1041-A1-B01-MR.abi CIT Human Genomic Sperm Library C Homo sapiens genomic clone Plate=CT 823 Col=1 Row=C, genomic survey sequence.//0.44:309:58//B36529
F-PLACE1011995//Homo sapiens Xq28 BAC RPCI11-382P7 (Roswell Park Cancer Institute Human BAC Library) complete sequence.//8.8e-53:687:71//AC006054
F-PLACE1012031//Homo sapiens mRNA for KIAA0713 protein, partial cds.//1.2e-146:690:98//AB018256
F-PLACE2000003//Homo sapiens chromosome 17, clone hRPK.318_A_15, complete sequence.//1.7e-62:293:88//AC005837
F-PLACE2000006//Homo sapiens chromosome 12p13.3 clone RPCI1-96H9, WORKING DRAFT SEQUENCE, 66 unordered pieces.//1.4e-116:261:91//AC006057
F-PLACE2000007
F-PLACE2000011//Homo sapiens chromosome 19, cosmid F20887, complete sequence.//5.2e-102:489:99//AC005578
F-PLACE2000014//Human DNA sequence *** SEQUENCING IN PROGRESS *** from clone 1111N9, WORKING DRAFT SEQUENCE.//0.0095:307:62//AL022574
F-PLACE2000015//Homo sapiens clone RG140B11, WORKING DRAFT SEQUENCE, 1 unordered pieces.//2.0e-36:316:81//AC005069
F-PLACE2000017//HS_3042_A1_F08_MR CIT Approved Human Genomic Sperm Library D Homo sapiens genomic clone Plate=3042 Col=15 Row=K, genomic survey sequence.//1.0:184:61//AQ098074
F-PLACE2000021//Homo sapiens TRF1-interacting ankyrin-related ADP-ribose polymerase mRNA, complete cds.//4.6e-84:844:72//AF082556
F-PLACE2000030//Human Chromosome 11 Cosmid cSRL16b6, complete sequence.//2.3e-22:233:77//U73638
F-PLACE2000033//C.capitata mRNA for chorion protein s18.//0.0019:342:62//Y08913
F-PLACE2000034//Rattus norvegicus transmembrane receptor Robo1 mRNA, complete cds.//2.8e-13:335:63//AF041082
F-PLACE2000039//Rattus norvegicus cytoplasmic dynein heavy chain (MAP 1C), mRNA, complete cds.//7.7e-84:489:90//L08505
F-PLACE2000047//Homo sapiens ccr2b (ccr2), ccr2a (ccr2), ccr5 (ccr5) and ccr6 (ccr6) genes, complete cds, and lactoferrin (lactoferrin) gene, partial cds, complete sequence.//5.0e-28:327:76//U95626
F-PLACE2000050//Homo sapiens chromosome 17, clone HRPC41C23, complete sequence.//1.1e-32:527:68//AC003101
F-PLACE2000061//CIT-HSP-2346L20.TF CIT-HSP Homo sapiens genomic clone 2346L20, genomic survey sequence.//1.1e-05:89:83//AQ059010
F-PLACE2000062//Human membrane-associated lectin type-C mRNA.//9.0e-113:662:86//M98457
F-PLACE2000072//Homo sapiens ZNF202 beta (ZNF202) mRNA, complete cds.//2.2e-133:631:98//AF027219
F-PLACE2000097//Homo sapiens chromosome 12p13.3 clone RPCI11-189M20, WORKING DRAFT SEQUENCE, 39 unordered pieces.//1.6e-16:119:93//AC005910
F-PLACE2000100//HS_3184_A1_D06_T7 CIT Approved Human Genomic Sperm Library D Homo sapiens genomic clone Plate=3184 Col=11 Row=G, genomic survey sequence.//1.5e-80:409:97//AQ150004
F-PLACE2000103//Human DNA sequence *** SEQUENCING IN PROGRESS *** from clone 20208, WORKING DRAFT SEQUENCE.//1.0e-172:830:98//AL031848
F-PLACE2000111//Homo sapiens DNA, trinucleotide repeats region.//1.0:200:64//AB018491
F-PLACE2000115
F-PLACE2000124//Homo sapiens Chromosome 16 BAC clone CIT987SK-A-67A1, complete sequence.//6.2e-43:362:80//AC004531
F-PLACE2000132//RPCI11-79F15.TV RPCI11 Homo sapiens genomic clone R-79F15, genomic survey sequence.//5.4e-35:206:94//AQ284166
F-PLACE2000136//Human BAC clone 7E17 from 12q, complete sequence.//2.7e-12:814:59//AC002070
F-PLACE2000140//Human DNA sequence *** SEQUENCING IN PROGRESS *** from clone 11703, WORKING DRAFT SEQUENCE.//3.6e-165:799:97//AL020995
F-PLACE2000164//Canine histamine H2 receptor gene, complete cds.//0.10:392:56//M32701
F-PLACE2000170
F-PLACE2000172//Homo sapiens PAC clone DJ0811017 from 7q21-22, complete sequence.//3.9e-91:552:88//AC006005
F-PLACE2000176//Homo sapiens Chromosome 22q11.2 BAC Clone b437g10 In BCRL2-GGT Region, complete sequence.//0.98:201:64//AC004032
F-PLACE2000187
F-PLACE2000216
F-PLACE2000223//RPCI11-12L17.TP RPCI-11 Homo sapiens genomic clone RPCI-11-12L17, genomic survey sequence.//0.00039:325:58/B75888
F-PLACE2000235//Human Chromosome 16 BAC clone CIT987SK-254P9, complete sequence.//7.5e-55:237:78//AC003003
F-PLACE2000246//Homo sapiens chromosome 3p clone RPCI4-544D10, WORKING DRAFT SEQUENCE, 58 unordered pieces.//2.4e-92:236:94//AC005902
F-PLACE2000264//Human DNA sequence from clone 391022 on chromosome 6p21.2-21.31 Contains pseudogenes similar to ribosomal protein, ESTs, GSSs, complete sequence.//1.4e-32:331:78//AL031577
F-PLACE2000274//Anthocidaris crassispina mRNA for B2HC, partial cds.//8.5e-48:765:66//AB012308
F-PLACE2000302//Kaposi's sarcoma-associated herpes-like virus ORF73 homolog gene, complete cds.//8.3e-08:662:58//US2064
F-PLACE2000305//Homo sapiens clone DJ1129L24, WORKING DRAFT SEQUENCE, 5 unordered pieces.//2.4e-08:95:81//AC006021
F-PLACE2000317//HS_3183_B2_F05_MR CIT Approved Human Genomic Sperm-Library D Homo sapiens genomic clone Plate=3183 Col=10 Row=L, genomic survey sequence.//2.5e-71:346:99//AQ172747
F-PLACE2000335//Homo sapiens clone DJ1032D07, WORKING DRAFT SEQUENCE, 3 unordered pieces.//3.7e-14:402:65//AC004952
F-PLACE2000341//Rattus norvegicus sodium-dependent multi-vitamin transporter (SMVT) mRNA, complete cds.//4.5e-77:555:82//AF026554
F-PLACE2000342//Suid herpesvirus 1 UL5 gene, partial cds, UL6 and UL7 genes, complete cds, UL8 gene, partial cds.//1.8e-14:259:71//U66829
F-PLACE2000347//Human DNA from overlapping chromosome 19-specific cosmids R32543,, and F15613 containing ZNF gene family member, genomic sequence, complete sequence.//6.0e-34:376:74//AC003006
F-PLACE2000359//RPCI11-23J20.TKBR RPCI-11 Homo sapiens genomic clone RPCI-11-23J20, genomic survey sequence.//8.4e-21:288:69//AQ013849
F-PLACE2000366//Human Tigger1 transposable element, complete consensus sequence.//5.0e-114:692:80//U49973
F-PLACE2000371//Homo sapiens 12p13.3 PAC RPCI1-29K11 (Roswell Park Cancer Institute Human PAC Library) complete sequence.//0.38:356:58//AC005182
F-PLACE2000373//RPCI11-49C18.TJ RPCI11 Homo sapiens genomic clone R-49C18, genomic survey sequence.//0.064:132:68//AQ051776
F-PLACE2000379//Homo sapiens Xp22 BAC GS-607H18 (Genome Systems Human BAC library) complete sequence.//1.6e-130:776:88//AC003658
F-PLACE2000394//Homo sapiens chromosome 18 BAC RPCI11-128D14 (Roswell Park Cancer Institute Human BAC Library) complete sequence.//5.4e-113:808:83//AC005909
F-PLACE2000398//Mouse hexamer repeat sequence (117) homologous to Drosophila 'period' gene.//0.87:286:63//X06967
F-PLACE2000399
F-PLACE2000404//Caenorhabditis elegans cosmid R74, complete sequence.//2.9e-59:532:68//Z36238
F-PLACE2000411//Acanthamoeba castellanii transformation-sensitive protein homolog mRNA, complete cds.//0.44:553:56//U89984
F-PLACE2000419//Human adenosine deaminase (ADA) gene, complete cds.//1.4e-56:303:86//M13792
F-PLACE2000425//HS_3047_A1_H05_MR CIT Approved Human Genomic Sperm Library D Homo sapiens genomic clone Plate=3047 Col=9 Row=O, genomic survey sequence.//2.8e-42:224:97//AQ126949
F-PLACE2000427
F-PLACE2000433//Homo sapiens chromosome 17, clone hRPK.156_L_14, complete sequence.//1.1e-19:363:67//AC005821
F-PLACE2000435//HS_3036_B1_F11_MR CIT Approved Human Genomic Sperm Library D Homo sapiens genomic clone Plate=3036 Col=21 Row=L, genomic survey sequence.//3.1e-06:184:66//AQ096999
F-PLACE2000438//Caenorhabditis elegans cosmid Y45F10D, complete sequence.//4.6e-23:550:62//AL021492
F-PLACE2000450//Homo sapiens PAC clone DJ1188N21 from 7q11.23-q21.1, complete sequence.//1.0e-78:604:80//AC006025
F-PLACE2000455//Homo sapiens Chromosome 16 BAC clone CIT987SK-A-279B10, complete sequence.//8.2e-05:330:63//AC002300
F-PLACE2000458//Homo sapiens chromosome 5p, BAC clone 50g21 (LBNL H154), complete sequence.//5.7e-168:816:97//AC005740
F-PLACE2000465//Human Chromosome 11 Overlapping Cosmids cSRL72g7 and cSRL140b8, complete sequence.//4.3e-33:296:79//AC002037
F-PLACE2000477//Homo sapiens clone RG052H06, WORKING DRAFT SEQUENCE, 11 unordered pieces.//3.4e-59:598:74//AC005057
F-PLACE3000004//Human EYA3 homolog (EYA3) mRNA, complete cds.//7.6e-49:361:84//U81602
F-PLACE3000009//Human placenta (Diff48) mRNA, complete cds.//3.0e-58:713:69//U49187
F-PLACE3000020//R.norvegicus type III adenylyl cyclase mRNA, complete cds.//6.1e-103:600:89//M55075
F-PLACE3000029
F-PLACE3000059//Mus musculus mRNA for ubiquitin conjugating enzyme.//4.4e-115:718:86//Y17267
F-PLACE3000070//Homo sapiens chromosome 5, BAC clone 194j18 (LBNL H158), complete sequence.//1.8e-17:250:74//AC005368
F-PLACE3000103//Caenorhabditis elegans cosmid C13F10.//4.6e-07:408:61//U97006
F-PLACE3000119//*** SEQUENCING IN PROGRESS *** Homo sapiens chromosome 4, BAC clone C0190L06; HTGS phase 1, WORKING DRAFT SEQUENCE, 21 unordered pieces.//1.5e-58:291:86//AC004670
F-PLACE3000121//Rattus norvegicus rsec15 mRNA, complete cds.//8.1e-81:837:71//AF032668
F-PLACE3000124//Homo sapiens chromosome 17, clone hRPK.85_B_7, complete sequence.//1.8e-48:330:79//AC005695
F-PLACE3000136
F-PLACE3000142//Human DNA sequence *** SEQUENCING IN PROGRESS *** from clone 349A12, WORKING DRAFT SEQUENCE.//0.011:294:62//AL033520
F-PLACE3000145//Gallus gallus tensin mRNA, 3' end.//6.9e-52:659:68//L06662
F-PLACE3000147//Human DNA sequence from clone 267M20 on chromosome Xq22.2-22.3. Contains part of the DIAPH2 gene and a pseudogene, ESTs, STSs and GSSs, complete sequence.//5.1e-37:305:81//AL031053
F-PLACE3000148//Homo sapiens chromosome Y, clone 47511, complete sequence.//4.7e-32:766:63//AC004474
F-PLACE3000155//Homo sapiens chromosome 17, clone hRPK.597_M_12, complete sequence.//7.4e-173:822:98//AC005277
F-PLACE3000156//Homo sapiens chromosome 19, overlapping cosmids F18547, F11133, R27945, R28830 and R32804, complete sequence.//2.2e-81:783:74//AC003682
F-PLACE3000157
F-PLACE3000158//, complete sequence.//1.0e-180:845:97//AC005500
F-PLACE3000160//CIT978SK-152K7.TV CIT978SK Homo sapiens genomic clone 152K7, genomic survey sequence.//0.080:259:59//B50878
F-PLACE3000169//Homo sapiens chromosome 19, BAC CIT-B-191n6, complete sequence.//9.8e-158:749:98//AC006130
F-PLACE3000194
F-PLACE3000197//F.rubripes GSS sequence, clone 075N04bB7, genomic survey sequence.//1.4e-08:164:68//AL003352
F-PLACE3000199//Human DNA sequence *** SEQUENCING IN PROGRESS *** from clone 424J12, WORKING DRAFT SEQUENCE.//0.0019:277:58//Z82207
F-PLACE3000207//Homo sapiens BAC clone GS165L15 from 7p15, complete sequence.//6.6e-21:312:67//AC005013
F-PLACE3000208//Homo sapiens (clones: CW52-2, CW27-6, CW15-2, CW26-5, 11-67) collagen type VII intergenic region and (COL7A1) gene, complete cds.//1.0:279:61//L23982
F-PLACE3000218//Homo sapiens, WORKING DRAFT SEQUENCE, 52 unordered pieces.//9.3e-43:383:79//AC004086
F-PLACE3000220//RPCI11-54B4.TV RPCI11 Homo sapiens genomic clone R-54B4, genomic survey sequence.//2.4e-36:381:76//AQ082056
F-PLACE3000221//Homo sapiens clone DJ1186P10, WORKING DRAFT SEQUENCE, 6 unordered pieces//7.2e-135:721:91//AC005231
F-PLACE3000226
F-PLACE3000230//Homo sapiens c1cr2b (ccr2), ccr2a (ccr2), ccr5 (ccr5) and ccr6 (ccr6) genes, complete cds, and lactoferrin (lactoferrin) gene, partial cds, complete sequence.//3.3e-80:498:78//U95626
F-PLACE3000242//Human DNA sequence from clone 1409 on chromosome Xp11.1-11.4. Contains a Inter-Alpha-Trypsin Inhibitor Heavy Chain LIKE gene, a alternatively spliced Melanoma-Associated Antigen MAGE LIKE gene and a 6-Phosphofructo-2-kinase (Fructose-2,6-bisphosphatase) LIKE pseudogene. Contains ESTs, STSs and genomic marker DXS8032, complete sequence.//2.6e-54:254:92//Z98046
F-PLACE3000244//M.musculus mRNA for 200 kD protein.//1.4e-139:850:86//X80169
F-PLACE3000254//Ateline herpesvirus 3 complete genome.//1.3e-10:399:61//AF083424
F-PLACE3000271//Human Chromosome 16 BAC clone CIT987SK-A-815A9, complete sequence.//1.8e-21:350:68//AF001548
F-PLACE3000276//HS_2026_B1_H11_T7 CIT Approved Human Genomic Sperm Library D Homo sapiens genomic clone Plate=2026 Col=21 Row=P, genomic survey sequence.//5.7e-45:376:81//AQ231147
F-PLACE3000304//Homo sapiens chromosome 19, cosmid R26660, complete sequence.//1.6e-138:650:99//AC005328
F-PLACE3000310
F-PLACE3000320//Human DNA sequence *** SEQUENCING IN PROGRESS *** from clone 423B22, WORKING DRAFT SEQUENCE.//1.9e-41:379:77//AL034379
F-PLACE3000322//Homo sapiens chromosome 17, clone hRPK.209_J_20, complete sequence.//3.3e-35:419:68//AC005822
F-PLACE3000331//CIT-HSP-2347D24.TR CIT-HSP Homo sapiens genomic clone 2347D24, genomic survey sequence.//2.7e-20:119:99//AQ061543
F-PLACE3000339//Rhodobacter sphaeroides magnesium chelatase subunits BchI (bchI) and BchD (bchD) genes, complete cds; and BchO (bchO) gene, partial cds.//0.99:310:58//AF017642
F-PLACE3000341//Homo sapiens 3p22 Contig 7 PAC RPCI4-672N11 (Roswell Park Cancer Institute Human PAC Library) complete sequence.//7.5e-159:752:98//AC006055
F-PLACE3000350//Rattus norvegicus serine/threonine protein kinase TAO1 mRNA, complete cds.//2.3e-107:592:92//AF084205
F-PLACE3000352//Human DNA sequence from PAC 293L6 on chromosome 22, complete sequence.//2.1e-37:480:70//Z83732
F-PLACE3000353
F-PLACE3000362//Homo sapiens chromosome 17, clone hRPK.215_P_18, complete sequence.//0.00011:373:60//AC005969
F-PLACE3000363
F-PLACE3000365//Human DNA sequence from PAC 227P17, between markers DXS6791 and DXS8038 on chromosome X contains CpG island, EST.//0.074:279:61//Z81007
F-PLACE3000373//Human DNA sequence from PAC 50A13 on chromosome Xp11. Contains ATP SYNTHASE LIPID BINDING PROTEIN P1 (P2, P3) precursor (ATP5G1, ATP5G2, ATP5G3) like pseudogene, ESTs and STSs. Contains polymorphic CA repeat.//2.8e-118:653:92//Z92545
F-PLACE3000388//Homo sapiens PAC clone DJ0777023 from 7p14-p15, complete sequence.//2.2e-25:288:71//AC005154
F-PLACE3000399//Human DNA sequence *** SEQUENCING IN PROGRESS *** from clone 466N1, WORKING DRAFT SEQUENCE.//2.3e-69:303:86//Z97630
F-PLACE3000400//Caenorhabditis elegans cosmid H03A11, complete sequence.//0.0063:435:58//Z93239
F-PLACE3000401//Homo sapiens clone DJ1147A01, WORKING DRAFT SEQUENCE, 25 unordered pieces.//5.8e-25 :292:73//AC006023
F-PLACE3000402//RPCI11-20D6.TVB RPCI-11 Homo sapiens genomic clone RPCI-11-20D6, genomic survey sequence.//1.1e-10:154:74//AQ008761
F-PLACE3000405//Homo sapiens chromosome 17, clone hRPK.628_E_12, complete sequence.//2.9e-41:515:72//AC005701
F-PLACE3000406//cSRL-179E11-u cSRL flow sorted Chromosome 11 specific cosmid Homosapiens genomic clone cSRL-179E11, genomic survey sequence.//2.8e-91:540:89//B03443
F-PLACE3000413
F-PLACE3000416//F19L8-Sp6 IGF Arabidopsis thaliana genomic clone F19L8, genomic survey sequence.//0.0018:664:55//B11305
F-PLACE3000425//Human DNA sequence from clone 231L4 on chromosome Xq27.1-27.3 Contains GSS, STS, complete sequence.//1.1e-16:284:70//AL022719
F-PLACE3000455//Human DNA sequence *** SEQUENCING IN PROGRESS *** from clone 469D22, WORKING DRAFT SEQUENCE.//3.6e-146:732:96//AL031284
F-PLACE3000475//HS_2164_A2_H10_MF CIT Approved Human Genomic Sperm Library D Homo sapiens genomic clone Plate=2164 Col=20 Row=O, genomic survey sequence.//1.5e-07:159:71//AQ132983
F-PLACE3000477//Human DNA sequence from PAC 368A4 on chromosome X. Contains ESTs, CELLULAR NUCLEIC ACID BINDING PROTEIN (CNBP) like gene and STSs.//2.9e-11:213:70//Z83843
F-PLACE4000009//Sequence 93 from patent US 5616500.//9.9e-08 :692:60//I39845
F-PLACE4000014//Homo sapiens mRNA for KIAA0809 protein, partial cds.//1.1e-116:331:100//AB018352
F-PLACE4000034//Homo sapiens Chromosome 16 BAC clone CIT987SK-A-69G12, complete sequence.//5.0e-05:244:63//AC004131
F-PLACE4000049//Homo sapiens Xp22-171-173 BAC GSHB-312I4 (Genome Systems Human BAC Library) complete sequence.//1.2e-37:385:74//AC005926
F-PLACE4000052//M.musculus abcl mRNA.//1.5e-110:671:88//X75926
F-PLACE4000063
F-PLACE4000089//M.musculus BOX DNA for regulatory element and promoter region related to EC cell differentiation.//3.7e-12:114:85//X74311
F-PLACE4000093//CIT-HSP-2380K5.TF CIT-HSP Homo sapiens genomic clone 2380K5, genomic survey sequence.//0.11:245:60//AQ108342
F-PLACE4000100//Human DNA sequence *** SEQUENCING IN PROGRESS *** from clone 20208, WORKING DRAFT SEQUENCE.//2.9e-19:384:65//AL031848
F-PLACE4000106//Homo sapiens mRNA for KIAA0462 protein, partial cds.//1.2e-145:684:99//AB007931
F-PLACE4000128//Mus musculus putative transcription factor mRNA, complete cds.//3.7e-62:541:78//AF091234
F-PLACE4000129
F-PLACE4000131//HS_3139_B2_F12_T7 CIT Approved Human Genomic Sperm Library D Homo sapiens genomic clone Plate=3139 Col=24 Row=L, genomic survey sequence.//2.3e-14:221:70//AQ183207
F-PLACE4000147//Human DNA sequence from clone 740A11 on chromosome Xq22.2-23. Contains part of the COL4A5 gene for Collagen Alpha 5(IV) Chain Precursor. Contains GSSs, complete sequence.//0.28:412:58//AL031622
F-PLACE4000156//Human zinc finger protein ZNF136.//7.2e-88:764:76//U09367
F-PLACE4000192
F-PLACE4000211
F-PLACE4000222//344J1.TVB CIT978SKA1 Homo sapiens genomic clone A-344J01, genomic survey sequence.//1.2e-14:177:76//B17158
F-PLACE4000230//Mus musculus semaphorin VIa mRNA, complete cds.//9.8e-116:662:89//AF030430
F-PLACE4000233//Homo sapiens DNA from chromosome 19, BAC 33152, complete sequence.//5.2e-54:363:70//AC003973
F-PLACE4000247
F-PLACE4000250//Homo sapiens Xp22-132-134 BAC GSHB-590J15 (Genome Systems Human BAC library) complete sequence.//0.0053:229:65//AC004673
F-PLACE4000252
F-PLACE4000259//H.sapiens gene for U5 snRNP-specific 200kD protein.//2.0e-25:191:87//Z70200
F-PLACE4000261//Mus musculus bromodomain-containing protein BP75 mRNA, complete cds.//2.6e-23:314:71//AF084259
F-PLACE4000269//Rattus norvegicus rexo70 mRNA, complete cds.//5.5e-122:734:88//AF032667
F-PLACE4000270
F-PLACE4000300
F-PLACE4000320//Human FKBP-rapamycin associated protein (FRAP) mRNA, complete cds.//1.4e-21:135:96//L34075
F-PLACE4000323//HS_2165_B1_B02_MF CIT Approved Human Genomic Sperm Library D Homo sapiens genomic clone Plate=2165 Col=3 Row=D, genomic survey sequence.//4.3e-08:170:71//AQ125036
F-PLACE4000326//Mouse DNA with homology to EBV IR3 repeat, segment 1, clone Mu2.//2.8e-06:311:63//M10296
F-PLACE4000344//Plasmodium falciparum chromosome 2, section 38 of 73 of the complete sequence.//0.014:252:60//AE001401
F-PLACE4000367
F-PLACE4000369
F-PLACE4000379//CIT-HSP-2350B9.TF CIT-HSP Homo sapiens genomic clone 2350B9, genomic survey sequence.//9.2e-46:282:86//AQ062661
F-PLACE4000387//CIT-HSP-2382F11.TR CIT-HSP Homo sapiens genomic clone 2382F11, genomic survey sequence.//0.96:102:70//AQ080649
F-PLACE4000392//Rattus norvegicus polymorphic marker D20UIA1 sequence.//1.2e-05:222:68//AF054088
F-PLACE4000401//Homo sapiens mRNA for KIAA0640 protein, partial cds.//9.6e-46:605:71//AB014540
F-PLACE4000411//Human DNA sequence *** SEQUENCING IN PROGRESS *** from clone 173D1, WORKING DRAFT SEQUENCE.//3.2e-29:179:79//AL031984
F-PLACE4000431//H.sapiens gene for U5 snRNP-specific 200kD protein.//4.0e-44:263:92//Z70200
F-PLACE4000445//HS-1053-B1-D02-MF.abi CIT Human Genomic Sperm Library C Homo sapiens genomic clone Plate=CT 775 Col=3 Row=H, genomic survey sequence.//0.070:47:100//B41346
F-PLACE4000450
F-PLACE4000465//Homo sapiens BAC clone RG114B19 from 7q31.1, complete sequence.//2.3e-07:273:65//AC005065
F-PLACE4000487//Homo sapiens chromosome 17, clone hRPK.156_L_14, complete sequence.//4.1e-34:351:70//AC005821
F-PLACE4000489//HS_3012_B1_G05_MR CIT Approved Human Genomic Sperm Library D Homo sapiens genomic clone Plate=3012 Col=9 Row=N, genomic survey sequence.//2.0e-36:220:92//AQ095537
F-PLACE4000494//Homo sapiens 12p13.3 PAC RPCI5-1063M23 (Roswell Park Cancer Institute Human PAC Library) complete sequence.//2.3e-57:395:79//AC005865
F-PLACE4000521//Homo sapiens *** SEQUENCING IN PROGRESS ***, WORKING DRAFT SEQUENCE.//1.6e-163:770:98//AJ011929
F-PLACE4000522//Feline leukemia virus Notch2 gene, clone FeLV/Notch2-C, partial cds.//4.0e-124:686:90//U47645
F-PLACE4000548
F-PLACE4000558//Bothrops atrox batroxobin gene (EC 3.4.21.29).//0.049:435:59//X12747
F-PLACE4000581
F-PLACE4000590//Homo sapiens chromosome Y, clone 475I1, complete sequence.//3.6e-20:747:59//AC004474
F-PLACE4000593//Caenorhabditis elegans cosmid F25D7, complete sequence.//5.6e-16:326:65//Z78418
F-PLACE4000612//Homo sapiens PAC clone DJ0722F20 from 7q31.1-q31.3, complete sequence.//1.7e-163:785:97//AC005281
F-PLACE4000638//Homo sapiens clone NH0319F03, WORKING DRAFT SEQUENCE, 3 unordered pieces.//8.7e-74:707:74//AC006039
F-PLACE4000650
F-PLACE4000654//Mus musculus mRNA for ubiquitin conjugating enzyme.//1.1e-145:840:89//Y17267
F-PLACE4000670//Sequence 13 from patent US 5712381.//1.0:311:59//I82816
F-SKNMC1000011//Gallus gallus bone sialoprotein II mRNA, complete cds.//0.014:92:73//U10577
F-SKNMC1000013//Orang-utan involucrin gene, complete cds.//0.021:417:59//M25312
F-SKNMC1000046//Homo sapiens mRNA for KIAA0654 protein, partial cds.//7.6e-147:706:98//AB014554
F-SKNMC1000050//Sequence 5 from patent US 5789181.//1.6e-52:330:90//AR020616
F-SKNMC1000091//Human NK homeobox protein (Nkx6.1) gene, exon 1.//0.0018:375:60//U66797
F-THYRO1000017//Rattus norvegicus pyridoxine 5'-phosphate oxidase mRNA, complete cds.//6.6e-97:542:84//U91561
F-THYRO1000026//Human DNA sequence from clone 833B7 on chromosome 22q12.3-13.2 Contains genes for NCF4 (P40PHOX) protein,cytokine receptor common beta chain precursor CSF2RB (partial), ESTs, CA repeat, STS, GSS, complete sequence.//3.5e-46:353:82//AL008637
F-THYRO1000034//Human DNA sequence *** SEQUENCING IN PROGRESS *** from clone 90L6, WORKING DRAFT SEQUENCE.//0.83:227:61//Z97353
F-THYRO1000035//Human Chromosome X clone bWXD187, complete sequence.//1.2e-39:303:83//AC004383
F-THYRO1000040
F-THYRO1000070//Homo sapiens chromosome 10 clone CIT987SK-1144G6 map 10q25.1, complete sequence.//1.3e-05:613:58//AC005383
F-THYRO1000072//Homo sapiens mRNA for KIAA0657 protein, partial cds.//2.7e-84:722:77//AB014557
F-THYRO1000085
F-THYRO1000092//CIT-HSP-2013L16.TFB CIT-HSP Homo sapiens genomic clone 2013L16, genomic survey sequence.//0.31:186:61//B60606
F-THYRO1000107
F-THYRO1000111//Human genomic DNA sequence from clone 308O1 on chromosome Xp11.3-11.4. Contains EST, CA repeat, STS, GSS, CpG island.//6.4e-110:690:87//Z93403
F-THYRO1000121//Rattus norvegicus CTD-binding SR-like protein rA8 mRNA, complete cds.//1.4e-127:816:85//U49055
F-THYRO1000124//H.sapiens CpG island DNA genomic Mse1 fragment, clone 72a7, forward read cpg72a7.ft1a.//9.5e-26:169:94//Z62724
F-THYRO1000129//Homo sapiens TED protein (TED) mRNA, complete cds.//8.5e-154:732:98//AF087142
F-THYRO1000132//Homo sapiens chromosome 9q34, clone 63G10, complete sequence.//3.7e-39:315:82//AC002096
F-THYRO1000156//Human DNA sequence from clone 113J7 on chromosome Xp11.22-11.4. Contains part of a putative Homeobox (pseudo?) gene, ESTs and an STS, complete sequence.//1.2e-21:335:71//AL023574
F-THYRO1000163//Homo sapiens Chromosome 16 BAC clone CIT987SK-A-A-218C7, complete sequence.//8.4e-52:301:88//AC002331
F-THYRO1000173//Mouse clathrin-associated protein (AP47) mRNA, complete cds.//4.0e-89:821:74//M62419
F-THYRO1000186//Human DNA sequence *** SEQUENCING IN PROGRESS *** from clone 424J12, WORKING DRAFT SEQUENCE.//7.2e-39:293:85//Z82207
F-THYRO1000187//Clostridium tetani gene for tetanus toxin.//0.041:473:57//X06214
F-THYRO1000190//Homo sapiens chromosome 17, clone hRPK.332_H_18, complete sequence.//0.38:184:64//AC005746
F-THYRO1000197//Homo sapiens mRNA for poly(A)-specific ribonuclease.//7.5e-174:805:99//AJ005698
F-THYRO1000199//Homo sapiens mRNA for KIAA0652 protein, complete cds.//1.2e-86:616:84//AB014552
F-THYRO1000206//HS_3047_A1_A05_MF CIT Approved Human Genomic Sperm Library D Homo sapiens genomic clone Plate=3047 Col=9 Row=A, genomic survey sequence.//0.51:331:63//AQ099134
F-THYRO1000221//Plasmodium falciparum 3D7 chromosome 12 PFYAC293 genomic sequence, WORKING DRAFT SEQUENCE, 9 unordered pieces.//0.092:738:56//AC004157
F-THYRO1000241//Gallus gallus genome fragment with pentamer tandem repeats.//0.43:191:62//X00186
F-THYRO1000242//Human zinc finger gene HZF7.//2.8e-43:534:64//X60156
F-THYRO1000253//Homo sapiens 3p22 Contig 7 PAC RPCI4-672N11 (Roswell Park Cancer Institute Human PAC Library) complete sequence.//0.95:139:68//AC006055
F-THYRO1000270
F-THYRO1000279//Human DNA sequence *** SEQUENCING IN PROGRESS *** from clone 531H16, WORKING DRAFT SEQUENCE.//1.4e-174:826:98//AL031664
F-THYRO1000288//Homo sapiens mRNA for Hs Ste24p, complete cds.//3.9e-179:848:98//AB016068
F-THYRO1000320//Mus musculus sphingosine-1-phosphate lyase mRNA, complete cds.//1.0e-44:331:83//AF036894
F-THYRO1000327//Homo sapiens autocrine motility factor receptor (AMFR) mRNA, complete cds.//5.7e-112:641:91//L35233
F-THYRO1000343//Homo sapiens mRNA for KIAA0790 protein, partial cds.//2.2e-162:763:98//AB018333
F-THYRO1000358//Human selenium-binding protein (hSBP) mRNA, complete cds.//2.2e-32:177:84//U29091
F-THYRO1000368//Caenorhabditis elegans cosmid W09G3, complete sequence.//0.97:206:60//Z82080
F-THYRO1000381//Arthrobacter sp. glcI gene for beta-1,3-glucanase, complete cds.//0.27:427:62//D23668
F-THYRO1000387//Homo sapiens PAC clone DJ1048B16 from 7q34-q36, complete sequence.//9.7e-147:698:98//AC006019
F-THYRO1000394//HS_2061_A2_C04_MR CIT Approved Human Genomic Sperm Library D Homo sapiens genomic clone Plate=2061 Col=8 Row=E, genomic survey sequence.//1.6e-29:202:91//AQ247672
F-THYRO1000395//Drosophila melanogaster ring canel protein and ORF2 mRNA, complete cds.//4.3e-15:512:59//L08483
F-THYRO1000401 3.2e-116:504:80//AF051908
F-THYRO1000438//Plasmodium falciparum 3D7 chromosome 12 PFYAC492 genomic sequence, WORKING DRAFT SEQUENCE, 5 unordered pieces.//3.4e-09:539:59//AC005308
F-THYRO1000452//RPCI11-1C19.TVB RPCI-11 Homo sapiens genomic clone RPCI-11-1C19, genomic survey sequence.//0.27:132:64//B49573
F-THYRO1000471//Homo sapiens PAC clone DJ1136G13 from 7q35-q36, complete sequence.//1.3e-38:332:81//AC005229
F-THYRO1000484//Homo sapiens BAC378, complete sequence.//2.2e-37:254:76//U85196
F-THYRO1000488//Homo sapiens chromosome 5p, BAC clone 50g21 (LBNL H154), complete sequence.//6.3e-130:327:97//AC005740
F-THYRO1000501//H.sapiens Staf50 mRNA.//9.8e-74:615:77//X82200
F-THYRO1000502//Human DNA sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.//0.076:380:59//Z94056
F-THYRO 1000505
F-THYRO1000558//Human PAC clone 127H14 from 12q, complete sequence.//2.4e-27:412:69//AC002563
F-THYRO1000569//HS_2178_B2_E03_T7 CIT Approved Human Genomic Sperm Library D Homo sapiens genomic clone Plate=2178 Col=6 Row=J, genomic survey sequence.//1.9e-27:326:74//AQ307499
F-THYRO1000570
F-THYRO1000585//Homo sapiens protein associated with Myc mRNA, complete cds.//7.4e-167:808:97//AF075587
F-THYRO1000596//Human Chromosome 16 BAC clone CIT987SK-A-972D3, complete sequence.//0.99:280:61//U91323
F-THYRO1000602//HS_3037_B2_E04_MR CIT Approved Human Genomic Sperm Library D Homo sapiens genomic clone Plate=3037 Col=8 Row=J, genomic survey sequence.//1.2e-05:109:75//AQ097057
F-THYRO1000605//Homo sapiens map 2p11.2; 83cM from GATA85A06 repeat region, complete sequence.//1.0:84:70//AF067777
F-THYRO1000625//Homo sapiens chromosome 19, cosmid R29425, complete sequence.//3.4e-174:820:98//AC005546
F-THYRO1000637//Human DNA sequence from clone 91J24 on chromosome 6q24 Contains part of utrophin Gene, part of cytochrome C oxidase gene, EST, CpG island, complete sequence.//3.6e-38:289:84//AL024474
F-THYRO1000641//Plasmodium falciparum MAL3P7, complete sequence.//6.8e-07:540:56//AL034559
F-THYRO1000658//Homo sapiens chromosome 17, clone hRPK.74_E_22, complete sequence.//1.1e-68:468:84//AC005696
F-THYRO1000662//Arabidopsis thaliana genomic DNA, chromosome 5, TAC clone: K23L20, complete sequence.//0.0072:141:70//AB016874
F-THYRO1000666//Mus musculus mRNA for motor domain of KIF9, partial cds.//4.7e-58:367:87//AB001437
F-THYRO1000676//Homo sapiens chromosome 19, cosmid F22676, complete sequence.//1.2e-36:396:71//AC005778
F-THYRO1000684//Fugu rubripes cosmid 165K09 DNA for GRM7, TRIP, Sand, PRGFR3 genes.//6.6e-13:236:69//AJ010317
F-THYRO1000699//RPCI11-50D4.TK RPCI11 Homo sapiens genomic clone R-50D4, genomic survey sequence.//2.7e-09:135:78//AQ052641
F-THYRO1000712//Homo sapiens BAC clone RG041D11 from 7q21, complete sequence.//5.2e-17:290:67//AC005053
F-THYRO1000715//Bovine herpesvirus type 1 early-intermediate transcription control protein (BICP4) gene, complete cds.//8.6e-08:517:60//L14320
F-THYRO1000734//HS_3233_B1_B04_T7 CIT Approved Human Genomic Sperm Library D-Homo sapiens genomic clone Plate=3233 Col=7 Row=D, genomic survey sequence.//6.0e-72:463:89//AQ182143
F-THYRO1000748//Homo sapiens KIAA0411 mRNA, complete cds.//9.7e-34:339:74//AB007871
F-THYRO1000756//M.musculus mRNA for Gal beta1, 3GalNAc alpha2,3-sialyltransferase.//0.00034:349:60//X73523
F-THYRO1000777//S.griseus strO gene and sts gene cluster.//8.2e-05:625:59//Y08763
F-THYRO1000783//Xenopus laevis tail-specific thyroid hormone up-regulated (gene 5) mRNA, complete cds.//4.0e-70:860:69//U37373
F-THYRO1000787//Human DNA sequence *** SEQUENCING IN PROGRESS *** from clone 366D1, WORKING DRAFT SEQUENCE.//5.3e-09:221:66//Z97986
F-THYRO1000793
F-THYRO1000796//Cristatella mucedo clone 5.9 microsatellite sequence.//0.34:173:63//AF085422
F-THYRO1000805//Homo sapiens Xp21 PAC RPCI1-37A12 containing exons 10 to 16 of the Duchenne Muscular Dystrophy gene, complete sequence.//7.8e-43:677:66//AC004468
F-THYRO1000815//Homo sapiens chromosome 5, Bac clone 189 (LBNL H135), complete sequence.//5.5e-43:405:77//AC005914
F-THYRO1000829//CIT-HSP-2387C10.TF.1 CIT-HSP Homo sapiens genomic clone 2387C10, genomic survey sequence.//2.0e-20:159:88//AQ240053
F-THYRO1000843
F-THYRO1000852//Homo sapiens chromosome 19, cosmid R31855, complete sequence.//1.8e-33:445:72//AC005782
F-THYRO1000855//Mus musculus potassium channel alpha subunit (Kv9.1) mRNA, complete cds.//0.038:208:64//AF008573
F-THYRO1000865//Homo sapiens PAC clone DJ0283M22 from 14, complete sequence.//1.9e-30:286:74//AC005477
F-THYRO1000895//Human DNA sequence *** SEQUENCING IN PROGRESS *** from clone 385E7, WORKING DRAFT SEQUENCE.//2.8e-18:186:80//AL031720
F-THYRO1000916//Homo sapiens clone DJ0965K10, WORKING DRAFT SEQUENCE, 6 unordered pieces.//3.6e-78:432:93//AC006015
F-THYRO1000926//Homo sapiens cAMP-specific phosphodiesterase 8B (PDE8B) mRNA, partial cds.//9.2e-178:839:98//AF079529
F-THYRO1000934//Human pyrroline 5-carboxylate reductase mRNA, complete cds.//3.5e-32:759:63//M77836
F-THYRO1000951//Homo sapiens Chromosome 11q12 pac pDJ57114, WORKING DRAFT SEQUENCE, 29 unordered pieces.//4.9e-76:224:93//AC004229
F-THYRO1000952
F-THYRO1000974//HS_3238_B2_F01_MR CIT Approved Human Genomic Sperm Library D Homo sapiens genomic clone Plate=3238 Col=2 Row=L, genomic survey sequence./12.4e-26:154:96//AQ219846
F-THYRO1000975//Plasmodium falciparum TopoII gene.//0.32:491:58//X79345
F-THYRO1000983//Mvwf9A3 exon amplification products from BACs in Mvwf region Mus musculus genomic, genomic survey sequence.//7.0e-16:112:94//AQ010457
F-THYRO1000984//CIT-HSP-2167O17.TR CIT-HSP Homo sapiens genomic clone 2167O17, genomic survey sequence.//0.00015:186:66//B91313
F-THYRO1000988//Human Chromosome 11q12.2 PAC clone pDJ756b9 containing human ferritin heavy chain mRNA (FTH), WORKING DRAFT SEQUENCE, 19 unordered pieces.//0.024:267:63//AC004588
F-THYRO1001003
F-THYRO1001031//Homo sapiens chromosome 17, clone hRPC.859_O_20, complete sequence.//1.1e-55:543:72//AC003695
F-THYRO1001033//Methanobacterium thermoautotrophicum from bases 48264 to 58328 (section 5 of 148) of the complete genome.//0.94:445:58//AE000799
F-THYRO1001062//Human DNA sequence *** SEQUENCING IN PROGRESS *** from clone 199H16, WORKING DRAFT SEQUENCE.//4.4e-45:441:75//AL022320
F-THYRO1001093//Homo sapiens chromosome 9, clone hRPK.202_H_3, complete sequence.//4.9e-34:353:76//AC006241
F-THYRO1001100//Human DNA-binding protein mRNA, 3'end.//1.1e-72:742:74//L14787
F-THYRO1001120//Homo sapiens clone DJ1129E22, WORKING DRAFT SEQUENCE, 7 unordered pieces.//1.2e-76:521:86//AC005522
F-THYRO1001121//Human DNA sequence *** SEQUENCING IN PROGRESS *** from clone 671O14, WORKING DRAFT SEQUENCE.//0.00078:594:58//AL031595
F-THYRO1001133//Homo sapiens PAC clone DJ1200I23 from 7p15, complete sequence.//4.0e-35:349:76//AC004996
F-THYRO1001134//Homo sapiens clone DJ1070G24, WORKING DRAFT SEQUENCE, 12 unordered pieces.//1.0:154:66//AC005486
F-THYRO1001142//Human DNA sequence from clone B79B4 on chromosome 22 Contains CA repeat and GSS, complete sequence.//1.4e-44:374:80//Z82178
F-THYRO1001173
F-THYRO1001177//Human pigment epithelium-derived factor gene, complete cds.//1.9e-42:250:86//U29953
F-THYRO1001189//HS_3171_B2_F10_MR CIT Approved Human Genomic Sperm Library D Homo sapiens genomic clone Plate=3171 Col=20 Row=L, genomic survey sequence.//1.8e-28:246:83//AQ302330
F-THYRO1001204//Drosophila melanogaster DNA repair protein (mei-41) gene, complete cds, and TH1 gene, partial cds.//4.9e-39:657:64//U34925
F-THYRO1001213//, complete sequence.//1.7e-45:257:84//AC005300
F-THYRO1001262//Homo sapiens genomic DNA, chromosome 21q11.1, segment 7/28, WORKING DRAFT SEQUENCE.//1.5e-40:274:87//AP000036
F-THYRO1001271//Streptomyces coelicolor cosmid 1A6.//0.033:364:61//AL023496
F-THYRO1001287//Drosophila melanogaster cosmid clone 86E4.119.6e-49:586:69//AL021086
F-THYRO1001290//HS_2045_B1_H09_MR CIT Approved Human Genomic Sperm Library D Homo sapiens genomic clone Plate=2045 Col=17 Row=P, genomic survey sequence.//4.4e-13:156:78//AQ248237
F-THYRO1001313//S. lavendulae bla gene for beta-lactamase, complete cds.//1.0:229:64//D12693
F-THYRO1001320//Homo sapiens Chromosome 22q11.2 PAC Clone p_n5 In BCRL2-GGT Region, complete sequence.//1.1e-88:672:82//AC002472
F-THYRO1001321//Human PAC clone DJ527C21 from Xq23, complete sequence.//1.2e-115:740:87//AC000114
F-THYRO1001322//HS_3205_B2_C12_MR CIT Approved Human Genomic Sperm Library D Homo sapiens genomic clone Plate=3205 Col=24 Row=F, genomic survey sequence.//0.00031:285:61//AQ304025
F-THYRO1001347//Homo sapiens mRNA for KIAA0745 protein, partial cds.//2.2e-43:638:64//AB018288
F-THYRO1001363//Homo sapiens PAC clone DJ0845I21 from 7q11.21-q11.23, complete sequence.//1.0e-09:189:74//AC004905
F-THYRO1001365//Homo sapiens chromosome 10 clone CIT987SK-1163G10 map-10q25, complete sequence.//7.6e-168:821:97//AC005660
F-THYRO1001374//Homo sapiens mRNA for KIAA0707 protein, partial cds.//2.3e-155:740:97//AB014607
F-THYRO1001401//Homo sapiens chromosome 19, cosmid F23149, complete sequence.//3.2e-07:138:73//AC005239
F-THYRO1001403//Homo sapiens chromosome 12p13.3 clone RPCI3-454B23, WORKING DRAFT SEQUENCE, 48 unordered pieces.//3.6e-70:360:86//AC005845
F-THYRO1001405//Bos taurus mRNA for NDP52, complete cds.//2.6e-14:559:63//AB008852
F-THYRO1001406//Mus musculus putative steroid dehydrogenase (KIK-I) mRNA, complete cds.//1.0e-91:631:82//AF064635
F-THYRO1001411//Homo sapiens chromosome 19, cosmid F18718, complete sequence.//5.5e-42:509:71//AC006126
F-THYRO1001426//*** SEQUENCING IN PROGRESS *** Homo sapiens genomic DNA (PAC 1118i22) from chromosome 11; HTGS phase 1, WORKING DRAFT SEQUENCE.//2.7e-31:172:81//AJ002553
F-THYRO1001434//Human Chromosome 11 pac pDJ393o15, WORKING DRAFT SEQUENCE, 8 unordered pieces.//1.0:98:70//AC000384
F-THYRO1001458//Bos taurus non-muscle myosin heavy chain mRNA, partial cds.//1.9e-58:653:71//U87265
F-THYRO1001480//Homo sapiens clone DJ0756H11, WORKING DRAFT SEQUENCE, 5 unordered pieces.//7.5e-42:357:80//AC006001
F-THYRO1001487//H.sapiens DNA sequence.//0.92:160:64//Z22449
F-THYRO1001534//Homo sapiens chromosome 17, clone hCIT.468_F_23, WORKING DRAFT SEQUENCE, 3 unordered pieces.//4.8e-47:266:80//AC004666
F-THYRO1001537//Human DNA sequence *** SEQUENCING IN PROGRESS *** from clone 998H6, WORKING DRAFT SEQUENCE.//1.3e-79:479:89//AL031687
F-THYRO1001541//Human DNA sequence from clone 399M14 on chromosome Xq26.1-26.3. Contains ESTs, an STS and GSSs, complete sequence.//0.0034:106:77//Z96074
F-THYRO1001559//Rattus norvegicus simple sequence repeat D18Mco6.//1.6e-09:351:63//AF006056
F-THYRO1001570//RPCI11-49B23.TJ RPCI11 Homo sapiens genomic clone R-49B23, genomic survey sequence.//1.4e-65:384:91//AQ052105
F-THYRO1001573//Homo sapiens clone 24778 unknown mRNA.//8.2e-104:546:95//AF070572
F-THYRO1001584//CIT-HSP-2365J21.TF CIT-HSP Homo sapiens genomic clone 2365J21, genomic survey sequence.//1.3e-24:180:88//AQ080498
F-THYRO1001595//Human DNA sequence *** SEQUENCING IN PROGRESS *** from clone Y313F4, WORKING DRAFT SEQUENCE.//8.7e-145:779:93//AL023808
F-THYRO1001602//Homo sapiens chromosome 17, clone hRPK.786_O_4, complete sequence.//2.9e-26:393:68//AC005863
F-THYRO1001605//Dictyostelium discoideum filopodin (talA) gene, complete cds.//0.0012:436:58//U14576
F-THYRO1001617//Homo sapiens full-length insert cDNA clone ZD69D05.//8.6e-43:342:82//AF086381
F-THYRO1001637//Homo sapiens clone DJ1019E05, WORKING DRAFT SEQUENCE, 10 unordered pieces.//6.2e-15:318:66//AC004950
F-THYRO1001656//Homo sapiens PAC clone DJ044L15 from Xq23, complete sequence.//1.5e-05:147:68//AC004827
F-THYRO1001661
F-THYRO1001671//Homo sapiens mRNA for 2'-5' oligoadenylate synthetase 59 kDa isoform.//2.5e-164:780:98//AJ225089
F-THYRO1001673//Homo sapiens clone RG161A02, complete sequence.//4.4e-40:770:64//AC005071
F-THYRO1001703//S.coelicolor plasmid SCP2 transfer region DNA.//0.14:414:59//X72857
F-THYRO1001706//Homo sapiens BAC clone RG281B09 from 7q21.1-q31.1, complete sequence.//2.6e-43:308:75//AC004745
F-THYRO1001721//, complete sequence.//9.9e-134:770:91//AC005500
F-THYRO1001738//Human DNA sequence *** SEQUENCING IN PROGRESS *** from clone 355C18, WORKING DRAFT SEQUENCE.//0.99:163:61//AL022327
F-THYRO1001745
F-THYRO1001746
F-THYRO1001772//HS_3069_B1_C05_MF CIT Approved Human Genomic Sperm Library D Homo sapiens genomic clone Plate=3069 Col=9 Row=F, genomic survey sequence.//1.5e-61:360:91//AQ171021
F-THYRO1001793//B.taurus mRNA for beta-subunit of rod photoreceptor CNG-channel.//0.028:446:58//X89626
F-THYRO 1001809
F-THYRO1001828//Human DNA sequence *** SEQUENCING IN PROGRESS *** from clone 110F11, WORKING DRAFT SEQUENCE.//1.3e-175:841:98//AL033526
F-THYRO1001854//Homo sapiens chromosome 17, clone hCIT54K19, complete sequence.//7.9e-07:445:59//AC003664
F-THYRO1001895
   4.4e-13:248:68//AB012576
F-THYRO1001907//Homo sapiens BAC clone RG054D04 from 7q31, complete sequence.//2.9e-15:144:77//AC005058
F-VESEN1000122//HS_3075_B1_C09_MF CIT Approved Human Genomic Sperm Library D Homo sapiens genomic clone Plate=3075 Col=17 Row=F, genomic survey sequence.//1.1e-16:130:90//AQ143749
F-Y79AA1000013
F-Y79AA1000033//Homo sapiens BAC clone GS114I09 from 7p14-p15, complete sequence.//2.9e-95:300:94//AC006027
F-Y79AA1000037//Human prot-oncogene (BMI-1) mRNA, complete cds.//2.4e-19:230:66//L13689
F-Y79AA1000059//Homo sapiens immunophilin homolog ARA9 mRNA, complete cds.//2.2e-38:629:64//U78521
F-Y79AA1000065//Human DNA sequence from cosmid J256K24, between markers DXS6791 and DXS8038 on chromosome X contains EST.//5.3e-10:117:83//Z72005
F-Y79AA1000131//Homo sapiens LERK-6 (EPLG6) gene, exon 1.//7.6e-10:381:64//U92893
F-Y79AA1000181//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//1.4e-165:732:99//AL031864
F-Y79AA1000202//Drosophila melanogaster DNA sequence (P1 DS06882 (D310)), complete sequence.//9.1e-20:339:65//AC005115
F-Y79AA1000214//Homo sapiens clone DJ0673M15, WORKING DRAFT SEQUENCE, 33 unordered pieces.//3.7e-72:397:93//AC004854
F-Y79AA1000230
F-Y79AA1000231//Mus musculus SIK similar protein mRNA, complete cds.//8.5e-151:833:90//AF053232
F-Y79AA1000258//Leishmania donovani histidine secretory acid phosphatase (SAcP-1) gene, complete cds.//0.0099:547:58//U78522
F-Y79AA1000268//Mus musculus Nip21 mRNA, complete cds.//4.0e-11:424:62//AF035207
F-Y79AA1000313
F-Y79AA1000328//CIT-HSP-386A20.TF CIT-HSP Homo sapiens genomic clone 386A20, genomic survey sequence.//5.9e-07:173:69//B55085
F-Y79AA1000342//RPCI11-57J6.TK.1 RPCI11 Homo sapiens genomic clone R-57J6, genomic survey sequence.//5.2e-27:151:99//AQ115511
F-Y79AA1000346//B.primigenius mRNA for coat protein gamma-cop.//5.7e-69:694:71//X92987
F-Y79AA1000349//M.musculus Spnr mRNA for RNA binding protein.//1.8e-98:535:92//X84692
F-Y79AA1000355//Homo sapiens clone DJ0847008, WORKING DRAFT SEQUENCE, 3 unordered pieces.//1.6e-21:129:85//AC005484
F-Y79AA1000368//H.sapiens CpG island DNA genomic Mse1 fragment, clone 12f1, reverse read cpg12f1.rt1c.//0.00016:53:98//Z56610
F-Y79AA1000405//Plasmodium falciparum DNA *** SEQUENCING IN PROGRESS *** from MAL1P4, WORKING DRAFT SEQUENCE.//0.069:366:59//AL031747
F-Y79AA1000410//Human DNA sequence from PAC 117P19 on chromosome X.//1.0e-25:235:80//Z86061
F-Y79AA1000420//H.sapiens CpG island DNA genomic Mse1 fragment, clone 82c3, forward read cpg82c3.ft1a.//2.0e-36:194:98//Z63378
F-Y79AA1000469//Mus musculus ancient ubiquitous 46 kDa protein AUP1 precursor (Aup1) mRNA, complete cds.//8.5e-121:696:89//U41736
F-Y79AA1000480//HS_2175_A2_H11_T7 CIT Approved Human Genomic Sperm Library D Homo sapiens genomic clone Plate=2175 Col=22 Row=O, genomic survey sequence.//2.5e-26:178:89//AQ307693
F-Y79AA1000538//Homo sapiens clone DJ1158B01, WORKING DRAFT SEQUENCE, 23 unordered pieces.//0.67:111:72//AC004980
F-Y79AA1000539//HS_2237_B2_F10_MF CIT Approved Human Genomic Sperm Library D Homo sapiens genomic clone Plate=2237 Col=20 Row=L, genomic survey sequence.//1.2e-14:168:77//AQ153503
F-Y79AA1000540//Homo sapiens clone DJ0655N24, WORKING DRAFT SEQUENCE, 8 unordered pieces.//0.94:127:67//AC005193
F-Y79AA1000560//Mouse mRNA for alpha-adaptin (C).//1.7e-114:776:84//X14972
F-Y79AA1000574//M.musculus tex23 mRNA (5'region).//1.8e-23:291:75//X80424
F-Y79AA1000589//Homo sapiens clone 614 unknown mRNA, complete sequence.//8.6e-153:755:97//AF091080
F-Y79AA1000627//Homo sapiens zinc finger protein (ZF5128) mRNA, complete cds.//5.2e-135:644:98//AF060503
F-Y79AA1000705//M.musculus mRNA of enhancer-trap-locus 1.//6.9e-148:902:86//X69942
F-Y79AA1000734//Homo sapiens PEX11 beta mRNA for peroxisome assembly factor, complete cds.//4.8e-180:850:98//AB018080
F-Y79AA1000748//Caenorhabditis elegans cosmid F25B5.//0.00019:308:60//U23172
F-Y79AA1000752//Oryctolagus cuniculus mRNA for hnRNP-E1 protein.//1.7e-40:513:68//AJ003023
F-Y79AA1000774
F-Y79AA1000782
F-Y79AA1000784//Homo sapiens RanBP7/importin 7 mRNA, complete cds.//3.5e-177:847:97//AF098799
F-Y79AA1000794//H.sapiens CpG island DNA genomic Mse1 fragment, clone 45a4, forward read cpg45a4.ft1a.//2.5e-13:104:92//Z61120
F-Y79AA1000800//Homo sapiens GABA-B receptor mRNA, complete cds.//0.98:244:60//AF056085
F-Y79AA1000802
F-Y79AA1000805//Human Chromosome 11 Cosmid cSRL30h11, complete sequence.//9.3e-76:528:85//U73642
F-Y79AA1000824//RPCI11-26B4.TP RPCI-11 Homo sapiens genomic clone RPCI-11-26B4, genomic survey sequence.//4.4e-14:99:95//B84538
F-Y79AA1000827//Human DNA sequence *** SEQUENCING IN PROGRESS *** from clone 1177I5, WORKING DRAFT SEQUENCE.//1.5e-08:249:69//AL022315
F-Y79AA1000833//Macaca fascicularis mRNA for alpha-tubulin.//1.8e-103:603:89//X04757
F-Y79AA1000850
F-Y79AA1000962//Human DNA sequence from PAC 360E18 on chromosome X contains EST, CpG island and polymorphic CA repeat.//0.038:468:59//Z82203
F-Y79AA1000966//Mus musculus COP9 complex subunit 4 (COPS4) mRNA, complete cds.//9.7e-150:865:89//AF071314
F-Y79AA1000968//Rattus norvegicus initiation factor eIF-2B gamma subunit (eIF-2B gamma) mRNA, complete cds.//6.4e-122:717:88//U38253
F-Y79AA1000969//Mouse chromosome 6 BAC-284H12 (Research Genetics mouse BAC library) complete sequence.//1.0:155:63//AC002397
F-Y79AA1000976//Caenorhabditis elegans cosmid F54C1.//4.3e-06:130:73//U88165
F-Y79AA1000985//Mus musculus pericentrin mRNA, complete cds.//2.4e-44:428:77//U05823
F-Y79AA1001023
F-Y79AA1001041//Human mutY homolog (hMYH) gene, complete cds.//2.3e-13:90:100//U63329
F-Y79AA1001048//Human mRNA for very-long-chain acyl-CoA dehydrogenase (VLCAD), complete cds.//2.6e-28:772:60//D43682
F-Y79AA1001061//Homo sapiens chromosome 4 clone B331M8 map 4q25, complete sequence.//9.4e-36:292:82//AC004701
F-Y79AA1001068//tipAL-AS complex: tipA=TipAL-AS [Streptomyces lividans, Genomic, 1146 nt].//0.17:537:59//S64314
F-Y79AA1001077//Zea mays mRNA for aldehyde oxidase-2, complete cds.//0.17:231:64//D88452
F-Y79AA1001078
F-Y79AA1001105//Zebrafish otx2 mRNA for otx homeoprotein, complete cds.//3.1e-63:529:77//D26173
F-Y79AA1001145//Homo sapiens clone GS166C05, WORKING DRAFT SEQUENCE, 7 unordered pieces.//1.3e-23:228:76//AC005015
F-Y79AA1001167
F-Y79AA1001177//M.musculus mRNA for NfiX1-protein.//4.0e-10:398:64//Y07688
F-Y79AA1001185//Human DNA sequence *** SEQUENCING IN PROGRESS *** from clone 169I5, WORKING DRAFT SEQUENCE.//1.1e-113:666:90//Z93015
F-Y79AA1001211//HS_3124_B2_H08_MR CIT Approved Human Genomic Sperm Library D Homo sapiens genomic clone Plate=3124 Col=16 Row=P, genomic survey sequence.//5.5e-12:87:96//AQ187492
F-Y79AA1001216
F-Y79AA1001228//Mycobacterium tuberculosis H37Rv complete genome; segment 143/162.//0.028:188:67//AL021841
F-Y79AA1001233//Human placental 17-beta-hydroxysteroid dehydrogenase mRNA, complete cds.//3.5e-24:731:60//M36263
F-Y79AA1001236//Homo sapiens mRNA for JM23 protein, complete coding sequence (clone IMAGE 34581 and IMAGE 45355 and LLNLc110I133Q7 (RZPD Berlin)).//1.2e-133:441:97//AJ005892
F-Y79AA1001281//HS_2241_B2_F09_MF CIT Approved Human Genomic Sperm Library D Homo sapiens genomic clone Plate=2241 Col=18 Row=L, genomic survey sequence.//5.0e-27:169:94//AQ217497
F-Y79AA1001299//Human Ini1 mRNA, complete cds.//6.7e-115:323:93//U04847
F-Y79AA1001312
F-Y79AA1001323
F-Y79AA1001384
F-Y79AA1001391//Mus musculus transcription factor HOXA13 (Hoxa13) gene, complete cds.//5.8e-42:245:74//U59322
F-Y79AA1001394//Caenorhabditis elegans cosmid F54B3, complete sequence.//7.8e-18:636:58//Z48583
F-Y79AA1001402//Homo sapiens Chr.14 PAC RPCI4-794B2 (Roswell Park Cancer Institute Human PAC Library) complete sequence.//1.2e-110:738:85//AC005924
F-Y79AA1001493//H.sapiens DNA sequence.//2.0e-27:254:82//Z22497
F-Y79AA1001511//Human DNA sequence from clone 931K24 on chromosome 20p12 Contains ESTs and GSSs, complete sequence.//1.1e-158:804:95//AL034430
F-Y79AA1001533//Mouse mRNA for RNA polymerase I associated factor (PAF53), complete cds.//1.7e-100:820:78//D14336
F-Y79AA1001541//HS_3197_A2_G11_MR CIT Approved Human Genomic Sperm Library D Homo sapiens genomic clone Plate=3197 Col=22 Row=M, genomic survey sequence.//5.1e-28:218:86//AQ150183
F-Y79AA1001548//Homo sapiens chromosome 19, cosmid R28738, complete sequence.//5.4e-21:167:86//AC004151
F-Y79AA1001555//R.norvegicus mRNA for drebrin A.//0.88:463:59//X59267
F-Y79AA1001581//FMR1 {CGG repeats} [human, Fragile X syndrome patient, Genomic, 429 nt].//0.00051:252:65//S74494
F-Y79AA1001585//Human hypoxanthine phosphoribosyltransferase (HPRT) gene, complete cds.//7.2e-33:375:76//M26434
F-Y79AA1001594
F-Y79AA1001603//Homo sapiens PAC 128M19 derived from chromosome 21q22.3, containing the HMG-14 and CHD5 genes, complete cds, complete sequence.//4.2e-06:338:66//AF064861
F-Y79AA1001613//Homo sapiens mRNA for KIAA0683 protein, complete cds.//0.024:520:57//AB014583
F-Y79AA1001647//Caenorhabditis elegans DNA *** SEQUENCING IN PROGRESS *** from clone Y53F4, WORKING DRAFT SEQUENCE.//0.014:331:61//Z92860
F-Y79AA1001665//Human DNA sequence from clone 299D3 on chromosome 22q13.3, complete sequence.//0.99:273:63//Z84468
F-Y79AA1001679//O.cuniculus lambda-crystallin mRNA, complete cds.//1.2e-97:682:81//M22743
F-Y79AA1001692//insulin-like growth factor binding protein-2 [human, placenta, Genomic, 1292 nt, segment 1 of 4].//5.6e-05:426:59//S37712
F-Y79AA1001696//Rice endogenous double-stranded RNA encoding polyprotein (containing putative helicase and putative RNA-dependent RNA polymerase domains), complete cds.//1.0:437:60//D32136
F-Y79AA1001705//M.musculus fkh-5 gene.//0.18:153:64//X71943
F-Y79AA1001711//Human DNA sequence *** SEQUENCING IN PROGRESS *** from clone 328E19, WORKING DRAFT SEQUENCE.//5.4e-76:191:98//AL022240
F-Y79AA1001781//Homo sapiens genomic DNA, chromosome 21q22.2 (Down Syndrome region), segment 10/15, WORKING DRAFT SEQUENCE.//0.99:227:63//AP000017
F-Y79AA1001805//H.sapiens CpG island DNA genomic Mse1 fragment, clone 13d12, reverse read cpg13d12.rt1c.//2.6e-13:88:100//Z64565
F-Y79AA1001827//Oryctolagus cuniculus PiUS mRNA, complete cds.//3.7e-130:775:88//U74297
F-Y79AA1001846//CIT-HSP-2300M6.TR CIT-HSP Homo sapiens genomic clone 2300M6, genomic survey sequence.//8.3e-17:218:76//AQ012369
F-Y79AA1001848//Human mRNA for KIAA0390 gene, complete cds.//4.2e-10:378:62//AB002388
F-Y79AA1001866//Rattus norvegicus Cys2/His2 zinc finger protein (rKr1) mRNA, complete cds.//6.9e-41:441:71//U41164
F-Y79AA1001874//Homo sapiens hJAG2.del-E6 (JAG2) mRNA, alternatively spliced isoform of Jagged2, complete cds.//0.00017:412:62//AF029779
F-Y79AA1001875//CTT-HSP-2317G18.TR CIT-HSP Homo sapiens genomic clone 2317G18, genomic survey sequence.//1.9e-09:271:67//AQ042654
F-Y79AA1001923//H.sapiens CpG island DNA genomic Mse1 fragment, clone 193c12, forward read cpg193c12.ft1a.//0.0031:108:75//Z60186
F-Y79AA1001963//CITBI-E1-2510J4.TR CITBI-E1 Homo sapiens genomic clone 2510J4, genomic survey sequence.//1.8e-05:56:100//AQ261184
F-Y79AA1002027//Arabidopsis thaliana ubiquitin-conjugating enzyme 17 (UBC17) mRNA, complete cds.//3.3e-13:451:62//AF028340
F-Y79AA1002083//Human DNA sequence *** SEQUENCING IN PROGRESS *** from clone 526I14, WORKING DRAFT SEQUENCE.//0.91:134:65//Z82214
F-Y79AA1002089
F-Y79AA1002093//Mus musculus transcription factor like protein 4 TCFL4 mRNA, partial cds.//1.2e-112:678:88//U43548
F-Y79AA1002103//HS_3052_B1_H08_MR CIT Approved Human Genomic Sperm Library D Homo sapiens genomic clone Plate=3052 Col=15 Row=P, genomic survey sequence.//6.5e-18:238:72//AQ135014
F-Y79AA1002115
F-Y79AA1002125//H.sapiens (D8S135) DNA segment containing GT repeat.//1.5e-14:99:96//X61693
F-Y79AA1002139//Saccharomyces cerevisiae dnaJ homolog Hlj1p (HLJ1) gene, complete cds.//2.5e-07:208:64//U19358
F-Y79AA1002204//HS_2235_B2_D12_MF CIT Approved Human Genomic Sperm Library D Homo sapiens genomic clone Plate=2235 Col=24 Row=H, genomic survey sequence.//2.9e-13:89:98//AQ154260
F-Y79AA1002208//CIT-HSP-2006M21.TV CIT-HSP Homo sapiens genomic clone 2006M21, genomic survey sequence.//3.7e-27:154:98//B56397
F-Y79AA1002209//E.coli tyrS gene coding for tyrosyl-tRNA synthetase.//2.8e-05:143:70//J01719
F-Y79AA1002210//Homo sapines chromosome 19, cosmid R28058, complete sequence.//8.3e-22:229:78//AC005615
F-Y79AA1002211//Homo sapiens chromosome 17, clone HRPC1067M6, complete sequence.//1.0e-06:241:67//AC003043
F-Y79AA1002220//CIT-HSP-2374P23.TR CIT-HSP Homo sapiens genomic clone 2374P23, genomic survey sequence.//1.3e-68:375:95//AQ109738
F-Y79AA1002229//Human mRNA for KIAA0086 gene, complete cds.//0.12:203:63//D42045
F-Y79AA1002234//Homo sapiens mRNA for KIAA0692 protein, partial cds.//1.3e-174:821:98//AB014592
F-Y79AA1002246//Homo sapiens clone GS166C05, WORKING DRAFT SEQUENCE, 7 unordered pieces.//0.50:470:60//AC005015
F-Y79AA1002258//Homo sapiens mRNA for KIAA0655 protein, partial cds.//6.8e-159:748:98//AB014555
F-Y79AA1002298//Human density enhanced phosphatase-1 mRNA, complete cds.//0.036:278:62//U10886
F-Y79AA1002307//Homo sapiens mRNA for KIAA0634 protein, partial cds.//6.4e-129:622:97//AB014534
F-Y79AA1002311//R.norvegicus mRNA for cytosolic resiniferatoxin-binding protein.//2.0e-116:693:82//X67877
F-Y79AA1002351//S.clavuligerus pah and cas genes.//1.0:369:58//X84101
F-Y79AA1002361//Rattus norvegicus mRNA for protein phosphatase 1 (GL-subunit).//5.4e-105:762:80//Y18208
F-Y79AA1002399//Homo sapiens chromosome 17, clone hRPK.700_H_6, complete sequence.//1.0e-159:411:100//AC005920
F-Y79AA1002407//Homo sapiens chromosome 17, clone hRPC.842_A_23, complete sequence.//1.1e-118:609:84//AC004662
F-Y79AA1002416//Mus musculus CTP synthetase homolog (CTPsH) mRNA, complete cds.//4.4e-90:529:88//U49385
F-Y79AA1002431//Chlamydomonas reinhardtii novel protein kinase mRNA, complete cds.//1.0:166:66//U36196
F-Y79AA1002433//CIT-HSP-384K8.TF CIT-HSP Homo sapiens genomic clone 384K8, genomic survey sequence.//0.24:85:72//B51917
F-Y79AA1002472//Homo sapiens chromosome 19, BAC CIT-B-393i15 (BC301323), complete sequence.//1.9e-13:242:69//AC006116
F-Y79AA1002482//Homo sapiens full-length insert cDNA clone ZC18H06.//1.2e-35:462:71//AF088022
F-Y79AA1002487//Bovine herpesvirus type 1 genes for UL[27,28,29,30,31].//0.93:215:60//X94677

### Homology Search Result Data 3.

The result of the homology search of the GenBank using the clone sequence of 3'-end except EST and STS.

Data include
the name of clone,
definition of the top hit data,
the P-value: the length of the compared sequence: identity (%), and
the Accession No. of the top hit data, as in the order separated by //.

Blank indicates that the 3'-end sequence corresponding to the 5'-end was not determined in the clone.

Data are not shown for the clones in which the P-value was higher than 1.
R-HEMBA1000005//Mouse tumor cell dnaJ-like protein 1 mRNA, complete cds.//3.6e-60:504:78//L16953
R-HEMBA1000030//F.rubripes GSS sequence, clone 063K10bD3, genomic survey sequence.//0.28:117:68//Z88864
R-HEMBA1000042//RPCI11-77G23.TV RPCI11 Homo sapiens genomic clone R-77G23, genomic survey sequence.//1.3e-56:292:97//AQ268240
R-HEMBA1000046//Homo sapiens chromosome X map Xq28, complete sequence.//9.8e-56:401:82//U82696
R-HEMBA1000050//Human cosmid insert containing polymorphic marker DXS455.//0.0010:175:68//L31948
R-HEMBA1000076//Homo sapiens clone DJ1021I20, WORKING DRAFT SEQUENCE, 6 unordered pieces.//4.9e-41:364:79//AC005520
R-HEMBA1000111//Homo sapiens Xp22 BAC GSHB-519E5 (Genome Systems Human BAC library) complete sequence.//4.7e-30:229:84//AC003684
R-HEMBA1000129//Homo sapiens chromosome 17, clone HCIT48C15, complete sequence.//2.4e-93:503:93//AC003104
R-HEMBA1000141//Homo sapiens mRNA for KIAA0797 protein, partial cds.//6.5e-99:514:94//AB018340
R-HEMBA1000150//Homo sapiens clone RG086D03, WORKING DRAFT SEQUENCE, 3 unordered pieces.//2.7e-37:289:83//AC005060
R-nnnnnnnnnnnn//Homo sapiens scaffold attachment factor B (SAF-B) mRNA, partial cds.//3.1e-21:417:64//L43631
R-HEMBA1000158
R-nnnnnnnnnnnn
R-HEMBA1000180//Plasmodium falciparum encoding Pfg27/25.//0.073:292:56//X84904
R-HEMBA1000185//Homo sapiens clone DJ0693M11, WORKING DRAFT SEQUENCE, 7 unordered pieces.//5.3e-40:286:85//AC006146
R-HEMBA1000193
R-HEMBA1000201//Homo sapiens SNF5/INI1 gene, exon 9.//2.0e-24:137:99//Y17126
R-HEMBA1000213//Caenorhabditis elegans cosmid C44C8.//0.025:192:68//AF100655
R-HEMBA1000216//Human Chromosome 16 BAC clone CIT987SK-A-815A9, complete sequence.//2.5e-31:269:79//AF001548
R-nnnnnnnnnnnn
R-HEMBA1000231//Human DNA sequence from PAC 212P9 on chromosome 1p34.1-1p35. Contains delta opiate receptor, CpG island, CA repeat,.//4.3e-24:400:68//AL009181
R-HEMBA1000243//Homo sapiens chromosome 17, Neurofibromatosis 1 locus, complete sequence.//1.3e-19:319:69//AC004526
R-HEMBA1000244
R-HEMBA1000251//Meloidogyne hapla mitochondrial COII gene, 3' end of cds; transfer RNA-His gene; 16S ribosomal RNA gene; ND3 gene, complete cds; cytochrome b (cytb) gene, 5' end of cds.//0.16:338:60//L76262
R-HEMBA1000264//Homo sapiens genomic DNA, chromosome 21q22.2 (Down Syndrome region), segment 5/15, WORKING DRAFT SEQUENCE.//0.00093:300:66//AP000012
R-nnnnnnnnnnnn//Homo sapiens Xp22 BAC GSHB 526D21 (Genome Systems Human BAC library) complete sequence.//3.5e-10:238:70//AC003037
R-HEMBA1000282//Arabidopsis thaliana BAC IG002P16.//0.71:344:60//AF007270
R-HEMBA1000288//Homo sapiens Xp22 PACs RPC11-263P4 and RPC11-164K3 complete sequence.//4.8e-33:267:82//AC003046
R-HEMBA1000290//Homo sapiens chromosome 17, clone HRPC837J1, complete sequence.//2.2e-15:249:69//AC004223
R-HEMBA1000302//CIT-HSP-2173N10.TF CIT-HSP Homo sapiens genomic clone 2173N10, genomic survey sequence.//1.0:215:61//B95105
R-nnnnnnnnnnnn//Mus musculus Plenty of SH3s (POSH) mRNA, complete cds.//1.0e-77:551:82//AF030131
R-nnnnnnnnnnnn//Rattus norvegicus Ca2+-dependent activator protein (CAPS) mRNA, complete cds.//2.0e-96:546:90//U16802
R-HEMBA1000307//Mus musculus mRNA for CDV-1 protein.//3.8e-36:315:68//Y10496
R-nnnnnnnnnnnn//Plasmodium falciparum 3D7 chromosome 12 PFYAC181 genomic sequence, WORKING DRAFT SEQUENCE, 8 unordered pieces.//0.078:379:59//AC005505
R-HEMBA1000338//Human DNA sequence *** SEQUENCING IN PROGRESS *** from clone 620E11, WORKING DRAFT SEQUENCE.//2.0e-33:399:72//AL031667
R-HEMBA1000351//Homo sapiens complete genomic sequence between D16S3070 and D16S3275, containing Familial Mediterranean Fever gene disease.//1.7e-39:272:87//AJ003147
R-HEMBA1000355//Human primary Alu transcript.//0.0045:67:85//U67829
R-HEMBA1000357//Homo sapiens (subclone 9_h8 from Pl H16) DNA sequence.//8.7e-93:426:88//L42086
R-HEMBA1000366//Homo sapiens PAC clone DJ0942I16 from 7q11, complete sequence.//1.7e-12:130:83//AC006012
R-HEMBA1000369//Human DNA sequence from clone 1039K5 on chromosome 22q12.3-13.2 Contains gene similar to PICK1 perinuclear binding protein, gene similar to monocarboxylate transporter (MCT3), ESTs, STS, GSS and a CpG island, complete sequence.//1.9e-69:355:97//AL031587
R-HEMBA1000376//Homo sapiens chromosome 19, BAC CIT-B-393i15 (BC301323), complete sequence.//3.7e-66:410:89//AC006116
R-HEMBA1000387//Homo sapiens chromosome 17, clone HCIT169H9, WORKING DRAFT SEQUENCE, 6 unordered pieces.//2.0e-43:363:81//AC002993
R-HEMBA1000390//Homo sapiens BAC clone RG041D11 from 7q21, complete sequence.//4.6e-23:417:69//AC005053
R-HEMBA1000392//Human Chromosome 11p14.3 PAC clone pDJ59m18, complete sequence.//6.2e-05:174:68//AC004582
R-HEMBA1000396//Homo sapiens DNA sequence from PAC 159A15 on chromosome Xp11.21-p11.23. Contains inter-alpha-trypsin inhibitor heavy chain H3 precursor-like protein.//1.4e-62:564:77//AL022575
R-HEMBA1000411
R-HEMBA1000418//Liverwort Marchantia polymorpha chloroplast genome DNA.//0.94:210:60//X04465
R-HEMBA1000422//CIT-HSP-2382A6.TR CIT-HSP Homo sapiens genomic clone 2382A6, genomic survey sequence.//4.4e-12:98:92//AQ078233
R-HEMBA1000428//Human DNA sequence from clone 393P23 on chromosome Xq21.1-21.33. Contains GSSs, complete sequence.//2.0e-93 :526:90//Z95400
R-HEMBA1000434//Homo sapiens clone DJ0309D19, WORKING DRAFT SEQUENCE, 12 unordered pieces.//2.7e-07:452:60//AC004826
R-HEMBA1000442//E.caballus microsatellite DNA, clone HMB4.//0.39:135:62//Y07733
R-HEMBA1000456//Plasmodium falciparum DNA *** SEQUENCING IN PROGRESS *** from contig 4-52, complete sequence.//2.6e-05:174:70//AL010226
R-HEMBA1000459//Arabidopsis thaliana putative transmembrane protein G1p (AtG1), putative nuclear DNA-binding protein G2p (AtG2), Em1 protein (ATEM1), putative chlorophyll synthetase (AtG4), putative transmembrane protein G5p (AtG5), putative acyl-coA dehydrogenase (AtG6), and calcium dependent protein kinase genes, complete cds; and unknown genes.//0.013:212:63//AF049236
R-HEMBA1000460//Homo sapiens PAC clone DJ0593H12 from 7p31, complete sequence.//8.6e-114:556:98//AC004839
R-HEMBA1000464//Caenorhabditis elegans cosmid C34B7, complete sequence.//0.086:334:61//Z83220
R-HEMBA1000469//Homo sapiens BAC clone RG442F18 from 2, complete sequence.//1.8e-52:472:79//AC005104
R-HEMBA1000488//, complete sequence.//3.3e-68:200:99//AC005500
R-HEMBA1000490//Caenorhabditis elegans cosmid Y53C12B, complete sequence.//0.97:233:6l//Z99278
R-HEMBA1000491
R-HEMBA1000504//Plasmodium falciparum DNA *** SEQUENCING IN PROGRESS *** from contig 3-64, complete sequence.//1.7e-08:440:60//AL009014
R-HEMBA1000505//Homo sapiens genomic DNA of 8p21.3-p22 anti-oncogene of hepatocellular colorectal and non-small cell lung cancer, segment 1/11.//0.37:189:62//AB020858
R-HEMBA1000508//Human DNA sequence from cosmid V210E9, between markers DXS366 and DXS87 on chromosome X.//1.1e-25:248:80//Z70280
R-HEMBA1000518//RPCI11-6022.TV RPCI-11 Homo sapiens genomic clone RPCI-11-6022, genomic survey sequence.//0.0035:293:61//B49544
R-HEMBA1000519
R-HEMBA1000520//Arabidopsis thaliana chromosome II BAC F10A12 genomic sequence, complete sequence.//0.30:255:63//AC006232
R-HEMBA1000523//Human cleavage stimulation factor 77kDa subunit mRNA, complete cds.//1.2e-53:203:92//U15782
R-HEMBA1000531//CIT-HSP-388J17.TR CIT-HSP Homo sapiens genomic clone 388J17, genomic survey sequence.//2.7e-24:137:99//B55638
R-HEMBA1000540//Human DNA sequence *** SEQUENCING IN PROGRESS *** from clone 510D11, WORKING DRAFT SEQUENCE.//0.00014:329:60//Z98044
R-HEMBA1000545//Homo sapiens Xp22 BAC GS-619J3 (Genome Systems Human BAC library) complete sequence.//6.9e-87:552:87//AC004103
R-nnnnnnnnnnnn//Human DNA sequence *** SEQUENCING IN PROGRESS ∗∗∗ from clone 134019, WORKING DRAFT SEQUENCE.//8.9e-121:584:98//AL034555
R-HEMBA1000557//Homo sapiens Chromosome 16 BAC clone CIT987SK-44M2, complete sequence.//5.7e-45:307:87//AC004381
R-HEMBA1000561//Mus musculus clone OST20235, genomic survey sequence.//1.3e-43:279:90//AF046762
R-HEMBA1000563//Plasmodium falciparum chromosome 2, section 5 of 73 of the complete sequence.//3.8e-05:506:56//AE001368
R-HEMBA1000568//RPCI11-49P8.TK.1 RPCI11 Homo sapiens genomic clone R-49P8, genomic survey sequence.//1.7e-101:498:97//AQ116293
R-nnnnnnnnnnnn
R-HEMBA1000575//Human DNA sequence *** SEQUENCING IN PROGRESS *** from clone 754E20, WORKING DRAFT SEQUENCE.//1.3e-47:458:75//AL022335
R-HEMBA1000588//Mus musculus FLI-LRR associated protein-1 mRNA, complete cds.//2.9e-62:447:81//AF045573
R-HEMBA1000591//Homo sapiens mRNA for E1B-55kDa-associated protein.//1.2e-111:591:9411AJ007509
R-HEMBA1000592//Plasmodium falciparum DNA *** SEQUENCING IN PROGRESS *** from contig 4-10, complete sequence.//3.5e-09:421:60//AL010216
R-HEMBA1000594//Homo sapiens clone RG004N09, WORKING DRAFT SEQUENCE, 5 unordered pieces.//1.1e-15:421:66//AC005044
R-HEMBA1000604//HS_2220_A1_G10_MF CIT Approved Human Genomic Sperm Library D Homo sapiens genomic clone Plate=2220 Col=19 Row=M, genomic survey sequence.//1.0e-51:306:92//AQ151991
R-HEMBA1000608
R-HEMBA1000622//H.sapiens CpG island DNA genomic Mse1 fragment, clone 155e4, reverse read cpg155e4.rt1a.//4.5e-16:105:98//Z56962
R-HEMBA1000636//Homo sapiens genomic DNA, chromosome 21q22.2 (Down Syndrome region), segment 1/15, WORKING DRAFT SEQUENCE.//4.8e-62:421:86//AP000008
R-HEMBA1000637//Homo sapiens mRNA for KIAA0690 protein, partial cds.//1.2e-97:443:97//AB014590
R-HEMBA1000655//Homo sapiens chromosome 19, cosmid R26349, complete sequence.//9.8e-61:311:90//AC005953
R-HEMBA1000657
R-HEMBA1000662
R-HEMBA1000673//Human DNA sequence from PAC 448E20 on chromosome Xq26.1 contains ESTs and STS.//1.0e-13:351:63//Z97196
R-HEMBA1000682//Homo sapiens clone DJ1136G02, WORKING DRAFT SEQUENCE, 4 unordered pieces.//1.2e-50:298:79//AC005377
R-HEMBA1000686//HS_3018_B1_H10_T7 CIT Approved Human Genomic Sperm Library D Homo sapiens genomic clone Plate=3018 Col=19 Row=P, genomic survey sequence.//0.00048:210:62//AQ093513
R-HEMBA1000702//Homo sapiens clone DJ241P17, WORKING DRAFT SEQUENCE, 7 unordered pieces.//9.7e-54:317:88//AC005000
R-HEMBA1000705//Glossonotus uhivittatus 12S mitochondrial ribosomal RNA, small subunit, mitochondrial gene, partial sequence.//0.080:138:65//U77850
R-HEMBA1000719//Rattus norvegicus mRNA for TESK1, complete cds.//0.96:291:58//D50864
R-HEMBA1000722
R-HEMBA1000726//Homo sapiens PAC clone DJ0701016 from 7q33-q36, complete sequence.//4.4e-26:284:77//AC005531
R-HEMBA1000727//Plasmodium falciparum DNA *** SEQUENCING IN PROGRESS *** from contig 4-89, complete sequence.//9.1e-05:351:60//AL010266
R-HEMBA1000747//Homo sapiens DNA sequence from PAC 124C6 on chromosome 6q21. Contains genomic marker D6S1603, ESTs, GSSs and a STS with a CA repeat polymorphism, complete sequence.//2.5e-16:123:93//AL021326
R-HEMBA1000749//Human Chromosome 16 BAC clone CIT987SK-327O24, complete sequence.//2.8e-32:298:79//AC003108
R-HEMBA1000752//Human DNA sequence from PAC 50A13 on chromosome Xp11. Contains ATP SYNTHASE LIPID BINDING PROTEIN P1 (P2, P3) precursor (ATP5G1, ATP5G2, ATP5G3) like pseudogene, ESTs and STSs. Contains polymorphic CA repeat.//2.8e-90:542:90//Z92545
R-HEMBA1000769//Homo sapiens P1 clone GSP13996 from 5q12, complete sequence.//2.7e-36:405:75//AC005031
R-HEMBA1000773//HS_3050_A2_B08_MF CIT Approved Human Genomic Sperm Library D Homo sapiens genomic clone Plate=3050 Col=16 Row=C, genomic survey sequence.//0.00053:268:60//AQ105619
R-HEMBA1000774//Homo sapiens PAC clone DJ0630C24 from 7q31-q32, complete sequence.//4.7e-46:338:85//AC004690
R-HEMBA1000791//***ALU WARNING: Human Alu-Sc subfamily consensus sequence.//5.3e-47:279:91//U14571
R-HEMBA10008177/Sequence 1 from Patent WO 8904839.//0.86:148:67//I09339
R-HEMBA1000822//T.brucei kinetoplast maxicircle variable region DNA.//0.00061:246:61//Z15118
R-HEMBA1000827//Homo sapiens Ser/Arg-related nuclear matrix protein (SRM160) mRNA, complete cds.//6.9e-43:228:98//AF048977
R-HEMBA1000843//Homo sapiens DNA sequence from clone 511B24 on chromosome 20q11.2-12. Contains the TOP1 gene for Topoisomerase I, the PLCG1 gene for 1-Phosphatidylinositol-4,5-Bisphosphate Phosphodiesterase Gamma 1 (EC 3.1.4.11, PLC-Gamma-1, Phospholipase C-Gamma-1 PLC-II, PLC-148), the KIAA0395 gene for a probable Zinc Finger Homeobox protein and a 60S Ribosomal Protein L23 LIKE pseudogene. Contains a predicted CpG island, ESTs, STSs and GSSs, complete sequence.//1.7e-41:319:84//AL022394
R-HEMBA1000851//Arabidopsis thaliana chromosome I BAC T14N5 genomic sequence, complete sequence.//0.40:168:67//AC004260
R-HEMBA1000852//Homo sapiens Xp22 bins 3-5 PAC RPCI4-617A9 (Roswell Park Cancer Institute Human PAC Library) containing Arylsulfatase D and E genes, complete sequence.//1.5e-112:572:96//AC005295
R-HEMBA1000867//Homo sapiens clone DJ0971C03, WORKING DRAFT SEQUENCE, 18 unordered pieces.//0.11:121:71//AC004938
R-HEMBA1000869//Homo sapiens chromosome 16p11.2 BAC clone CIT987SK-A-180G2, WORKING DRAFT SEQUENCE, 5 unordered pieces.//3.2e-22:186:76//AC002042
R-HEMBA1000870//Human BAC clone GS542D18 from 7q31-q32, complete sequence.//0.0060:283:63//AC002528
R-HEMBA1000872//Rattus norvegicus polymorphic satellite repetitive elements.//3.8e-05:269:61//M98801
R-HEMBA1000876//Homo sapiens chromosome 12p13.3 clone RPCI1-96H9, WORKING DRAFT SEQUENCE, 66 unordered pieces.//6.5e-38:327:77//AC006057
R-HEMBA1000908//CIT-HSP-2373I4.TR CIT-HSP Homo sapiens genomic clone 2373I4, genomic survey sequence.//5.0e-34:221:90//AQ108658
R-HEMBA1000910//T.pigmentosa UM1060 macronuclear rDNA telomeric region 3' term.//0.19:280:61//X04205
R-HEMBA1000918//RPCI11-68E14.TK RPCI11 Homo sapiens genomic clone R-68E14, genomic survey sequence.//1.3e-32:172:100//AQ267293
R-HEMBA1000919
R-HEMBA1000934//Homo sapiens DNA sequence from PAC 874C20 on chromosome 6p22.1-22.3. Contains a Zinc Finger Protein ZFP47 LIKE gene, a Zinc Finger Protein pseudogene and a Zinc Finger Protein SRE-ZBP pseudogene. Contains ESTs, STSs and GSSs, complete sequence.//2.6e-18:284:71//AL021997
R-HEMBA1000942//Homo sapiens clone RG350L10, WORKING DRAFT SEQUENCE, 15 unordered pieces.//1.4e-17:217:76//AC005098
R-HEMBA1000943//Homo sapiens chromosome 17, clone hRPK.640_I_15, complete sequence.//9.0e-113:586:95//AC005324
R-HEMBA1000946//T5N8TFB TAMU Arabidopsis thaliana genomic clone T5N8, genomic survey sequence.//0.030:369:59//B26224
R-HEMBA1000960//Homo sapiens clone RG339C12, WORKING DRAFT SEQUENCE, 10 unordered pieces.//2.5e-52:494:77//AC005096
R-HEMBA1000968//Homo sapiens P1 clone 797a11 containing MHC class II DQ-beta (HLA-DQB) and MHC class II DC-alpha (HLA-DCA) genes, complete cds.//3.5e-77:568:83//U92032
R-HEMBA1000971//RPCI11-54D1.TJ RPCI11 Homo sapiens genomic clone R-54D1, genomic survey sequence.//2.3e-27:153:98//AQ081552
R-HEMBA1000972//Human DNA sequence from clone 111F4 on chromosome Xq23 Contains GSSs, complete sequence.//7.3e-43:375:79//AL023876
R-HEMBA1000974//Homo sapiens clone DA0091H08, complete sequence.//2.8e-104:521:97//AC004817
R-HEMBA1000975//Human DNA sequence from clone 105D16 on chromosome Xp11.3-11.4 Contains pseudogene similar to laminin-binding protein, CA repeat, STS, complete sequence.//8.0e-22:352:68//AL031311
R-HEMBA1000985//Homo sapiens PAC clone DJ0797C05 from 7q31, complete sequence.//8.5e-05:306:63//AC004888
R-HEMBA1000986//Homo sapiens clone RG031N19, WORKING DRAFT SEQUENCE, 1 unordered pieces.//5.7e-37:296:83//AC005632
R-HEMBA1000991//RPCI11-22017.TVB RPCI-11 Homo sapiens genomic clone RPCI-11-22017, genomic survey sequence.//6.5e-44:162:90//AQ008952
R-HEMBA1001007
R-HEMBA1001008//Homo sapiens chromosome 16, P1 clone 79-2A (LANL), complete sequence.//0.082:313:60//AC005365
R-HEMBA1001009//O.sativa osr40g2 gene.//0.99:203:62//Y08987
R-HEMBA1001017//Homo sapiens mRNA for KIAA0468 protein, complete cds.//1.0e-113:587:95//AB007937
R-HEMBA1001019//Bos taurus cyclin-dependent kinase 1 (cdk1/cdc2) mRNA, complete cds.//7.4e-24:215:82//L26547
R-HEMBA1001020//Human DNA sequence *** SEQUENCING IN PROGRESS *** from clone 732E4, WORKING DRAFT SEQUENCE.//2.8e-18:449:64//AL008722
R-HEMBA1001022
R-HEMBA1001024//Homo sapiens BAC clone 393I22 from 8q21, complete sequence.//6.6e-48:536:74//AF070717
R-HEMBA1001026//T33H14TF TAMU Arabidopsis thaliana genomic clone T33H14, genomic survey sequence.//0.013:180:66//B97363
R-nnnnnnnnnnnn//Caenorhabditis elegans cosmid R10H10, complete sequence.//1.2e-25:438:65//Z70686
R-HEMBA1001051//Homo sapiens 12q24.1 PAC RPCI3-521E19 (Roswell Park Cancer Institute Human PAC library) complete sequence.//7.3e-38:188:89//AC004217
R-HEMBA1001052//Rabbit alpha-1-globin gene to theta-1-globin pseudogene region.//2.4e-24:279:74//X04751
R-HEMBA1001060//HS_2056_B1_C01_MR CIT Approved Human Genomic Sperm Library D Homo sapiens genomic clone Plate=2056 Col=1 Row=F, genomic survey sequence.//4.1e-14:137:83//AQ245004
R-HEMBA1001071//M.musculus COL3A1 gene for collagen alpha-I.//6.9e-38:513:70//X52046
R-HEMBA1001077//Human DNA sequence *** SEQUENCING IN PROGRESS *** from clone 150C2, WORKING DRAFT SEQUENCE.//1.9e-22:507:61//AL022318
R-HEMBA1001080
R-HEMBA1001085//Human Chromosome 15q26.1 PAC clone pDJ290i21 containing fur, fes, and alpha mannosidase IIx genes, WORKING DRAFT SEQUENCE, 9 unordered pieces.//2.2e-43:317:83//AC004586
R-HEMBA1001088//Caenorhabditis elegans cosmid C18H7.//0.46:301:60//AF067607
R-HEMBA1001094//Homo sapiens clone RG491N20, complete sequence.//5.3e-98:501:96//AC005105
R-HEMBA1001099
R-HEMBA1001109//Human DNA sequence *** SEQUENCING IN PROGRESS *** from clone 118J21, WORKING DRAFT SEQUENCE.//3.1e-39:335:80//AL033527
R-HEMBA1001121//Human cosmid LL12NC01-132B11A, ETV6 gene, intron 2.//9.8e-11:122:81//U81833
R-HEMBA1001122//Plasmodium falciparum MAL3P6, complete sequence.//0.0024:284:63//Z98551
R-HEMBA1001123//Human NFE genomic fragment.//3.6e-26:318:72//M98511
R-HEMBA1001133
R-HEMBA1001137//Homo sapiens full-length insert cDNA clone ZD29F04.//4.2e-88:426:98//AF086241
R-HEMBA1001140//Homo sapiens clone RG228D17, WORKING DRAFT SEQUENCE, 2 unordered pieces.//4.0e-41:304:84//AC005077
R-HEMBA1001172//Human DNA sequence *** SEQUENCING IN PROGRESS *** from clone 54B20, WORKING DRAFT SEQUENCE.//3.7e-36:261:85//Z98304
R-HEMBA1001174//Plasmodium falciparum chromosome 2, section 35 of 73 of the complete sequence.//1.0:219:58//AE001398
R-HEMBA1001197
R-HEMBA1001208//HS_2233_A1_G10_MF CIT Approved Human Genomic Sperm Library D Homo sapiens genomic clone Plate=2233 Col=19 Row=M, genomic survey sequence.//0.083:174:68//AQ170789
R-HEMBA100l226//Homo sapiens clone DJ1136G02, WORKING DRAFT SEQUENCE, 4 unordered pieces.//5.1e-59:553:75//AC005377
R-HEMBA1001235//RPCI11-50E6.TJ RPCI11 Homo sapiens genomic clone R-50E6, genomic survey sequence.//2.6e-08:97:76//AQ052666
R-HEMBA1001247//Caenorhabditis elegans cosmid C01F1.//2.4e-05:319:63//U58761
R-HEMBA1001257//Rattus norvegicus alpha-methylacyl-CoA racemase mRNA, complete cds.//1.5e-24:439:66//U89905
R-HEMBA1001265//Homo sapiens BAC clone RG139P11 from 7q11-q21, complete sequence.//9.9e-21:537:63//AC004491
R-nnnnnnnnnnnn//Homo sapiens chromosome 17, clone HCIT75G16, complete sequence.//0.022:169:65//AC003042
R-HEMBA1001286
R-HEMBA1001289
R-HEMBA1001294//HS_3219_A2_G01_T7 CIT Approved Human Genomic Sperm Library D Homo sapiens genomic clone Plate=3219 Col=2 Row=M, genomic survey sequence.//0.24:251:63//AQ189882
R-HEMBA1001299//Homo sapiens, clone hRPK.12_A_1, complete sequence.//1.3e-38:381:76//AC006222
R-HEMBA1001302//cDNA encoding a human homologue of a mouse novel polypeptide derived from stromal cell.//4.1e-28:114:92//E12258
R-HEMBA1001303//Plasmodium falciparum DNA *** SEQUENCING IN PROGRESS *** from MAL1P1, WORKING DRAFT SEQUENCE.//0.00011:382:58//AL031744
R-HEMBA1001310
R-HEMBA1001319//Plasmodium falciparum 3D7 chromosome 12 PFYAC1383 genomic sequence, WORKING DRAFT SEQUENCE, 3 unordered pieces.//4.2e-09:491:58//AC005504
R-HEMBA1001323//Drosophila yakuba mitochondrial DNA molecule.//8.3e-06:485:60//X03240
R-HEMBA1001326//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs (BAC end sequences) and a CA repeat polymorphism, complete sequence.//2.2e-14:277:69//AL021368
R-HEMBA1001327//Human DNA sequence from clone 522P13 on chromosome 6p21.31-22.3. Contains a 60S Ribosomal Protein L21 pseudogene and an HNRNP A3 (Heterogenous Nuclear Riboprotein A3, FBRNP) pseudogene. Contains ESTs, STSs and GSSs, complete sequence.//0.15:360:6l//AL024509
R-HEMBA1001330//Homo sapiens 12q24 PAC RPCI1-66E7 (Roswell Park Cancer Institute Human PAC library) complete sequence.//1.3e-27:481:67//AC004216
R-HEMBA1001351//Homo sapiens chromosome 18, clone hRPK.474_N_24, complete sequence.//7.1e-45:252:94//AC006238
R-HEMBA1001361//Homo sapiens chromosome 9, clone hRPK.202_H_3, complete sequence.//1.4e-113:569:97//AC006241
R-HEMBA1001375//Homo sapiens full-length insert cDNA clone ZE09H03.//2.8e-89:428:99//AF086542
R-HEMBA1001377//Homo sapiens PAC clone DJ0728D04, complete sequence.//2.3e-32:324:77//AC004865
R-HEMBA1001383
R-HEMBA1001387
R-HEMBA1001388//Homo sapiens clone RG189J21, WORKING DRAFT SEQUENCE, 15 unordered pieces.//8.9e-06:108:83//AC005073
R-HEMBA1001391//Yeast mitochondrial aapl gene for ATPase subunit 8.//7.3e-08:500:59//X00960
R-HEMBA1001398//Homo sapiens genomic DNA, chromosome 21q11.1, segment 21/28, WORKING DRAFT SEQUENCE.//2.3e-48:315:88//AP000050
R-HEMBA1001405//Human DNA sequence *** SEQUENCING IN PROGRESS *** from clone 50024, WORKING DRAFT SEQUENCE.//5.5e-35 :464:68//AL034380
R-HEMBA1001407
R-HEMBA1001411//Yeast (S.cerevisiae) mitochondria Ser-tRNA-UCN gene and flanks.//0.00029:301:62//K01981
R-HEMBA1001413
R-HEMBA1001415//Human DNA sequence *** SEQUENCING IN PROGRESS *** from clone 410I8, WORKING DRAFT SEQUENCE.//5.6e-101:512:96//AL031732
R-HEMBA1001432//Homo sapiens clone DJ0693M11, WORKING DRAFT SEQUENCE, 7 unordered pieces.//6.3e-37:302:81//AC006146
R-HEMBA1001433//Human DNA sequence from PAC 339A18 on chromosome Xp11.2. Contains KIAA0178 gene, similar to mitosis-specific chromosome segregation protein SMC1 of S.cerevisiae, DNA binding protein similar to URE-B1, ESTs and STS.//1.9e-32:242:79//Z97054
R-HEMBA1001435//Homo sapiens chromosome 21, Neurofibromatosis 1 (NF1) related locus, complete sequence.//5.7e-59:457:82//AC004527
R-HEMBA1001442//Human DNA sequence from PAC 507I15 on chromosome Xq26.3-27.3. Contains 60S ribosomal protein L44 (L41, L36) like gene, ESTs, STSs and a polymorphic CA repeat.//0.051:276:63//Z98950
R-HEMBA1001446//HS_3207_A1_A08_MR CIT Approved Human Genomic Sperm Library D Homo sapiens genomic clone Plate=3207 Col=15 Row=A, genomic survey sequence.//8.9e-06:119:73//AQ175385
R-HEMBA1001450//Homo sapiens BAC clone RG114B19 from 7q31.1, complete sequence.//0.0043:266:63//AC005065
R-HEMBA1001454//Homo sapiens PAC clone DJ0673011 from 7q31, complete sequence.//7.1e-25:210:82//AC004855
R-HEMBA1001455//Homo sapiens chromosome 17, clone hRPK.640_I_15, complete sequence.//2.7e-08:316:62//AC005324
R-HEMBA1001463//Homo sapiens chromosome 17, clone hRPK.1064_E_11, complete sequence.//0.57:219:60//AC005208
R-HEMBA1001476//Homo sapiens clone DJ0607J02, WORKING DRAFT SEQUENCE, 12 unordered pieces.//9.3e-50:252:80//AC004840
R-HEMBA1001478
R-HEMBA1001497
R-HEMBA1001510/Human HLA class III region containing cAMP response element binding protein-related protein (CREB-RP) and tenascin X (tenascin-X) genes, complete cds, complete sequence.//3.5e-41:282:86//U89337
R-HEMBA1001515//Human DNA sequence from PAC 238J17 on chromosome 6q22. Contains EST and STS.//1.9e-79:529:86//Z98753
R-HEMBA1001517//Homo sapiens BAC clone RG459N13 from 7p15, complete sequence.//4.3e-18:335:71//AC004549
R-HEMBA1001522
R-HEMBA1001526//Human DNA sequence from cosmid 444G9 from a contig from the tip of the short arm of chromosome 16, spanning 2Mb of 16p13.3 Contains ESTs and CpG islands,.//5.6e-08:265:67//Z98258
R-HEMBA1001533//Human DNA sequence from PAC 179M20 on chromosome 20q12-13.1. Contains adenosine deaminase (ADA), placental protein Diff33, CA repeat, ESTs, STS.//7.8e-16:235:72//Z97053
R-HEMBA1001557
R-HEMBA1001566//Human Chromosome X clone bWXD187, complete sequence.//2.2e-44:416:78//AC004383
R-HEMBA1001569//Sequence 15 from patent US 5693476.//1.8e-59:389:88//I77040
R-HEMBA1001570//Homo sapiens PAC clone DJ0844F09 from 7p12-p13, complete sequence.//1.1e-44:316:87//AC004453
R-HEMBA1001579//Plasmodium falciparum 3D7 chromosome 12 PFYAC357 genomic sequence, WORKING DRAFT SEQUENCE, 7 unordered pieces.//0.0047:437:60//AC005506
R-HEMBA1001581//P.falciparum complete gene map of plastid-like DNA (IR-B).//2.3e-07:491:58//X95276
R-HEMBA1001585//Caenorhabditis elegans cosmid C06A6.//0.68:224:62//U41012
R-HEMBA1001589
R-HEMBA1001595//CIT-HSP-2349G19.TF CIT-HSP Homo sapiens genomic clone 2349G19, genomic survey sequence.//8.0e-69:337:99//AQ060483
R-HEMBA1001608//Homo sapiens chromosome 17, clone HCIT462L7, complete sequence.//9.5e-59:514:78//AC005177
R-HEMBA1001620//S.polyrrhiza mRNA for D-myo-inositol-3-phosphate synthase.//4.5e-12:289:65//Z11693
R-nnnnnnnnnnnn//HS_2195_A1_E09_MF CIT Approved Human Genomic Sperm Library D Homo sapiens genomic clone Plate=2195 Col=17 Row=I, genomic survey sequence.//5.8e-09:358:58//AQ292688
R-HEMBA1001636//Human putative potassium channel subunit (h-erg) mRNA, complete cds.//0.77:225:59//U04270
R-HEMBA1001640//Human DNA sequence from PAC 50J22 on chromosome 6p21. Contains ETS related protein TEL like and GS2 like genes, ESTs and an STS.//6.0e-49:404:79//Z84484
R-nnnnnnnnnnnn
R-HEMBA1001655//Homo sapiens chromosome 5, BAC clone 194j18 (LBNL H158), complete sequence.//1.1e-103:532:95//AC005368
R-HEMBA1001658//Human DNA sequence *** SEQUENCING IN PROGRESS *** from clone Y313F4, WORKING DRAFT SEQUENCE.//1.0:197:64//AL023808
R-HEMBA1001661//Homo sapiens chromosome 5p, BAC clone 50g21 (LBNL H154), complete sequence.//1.5e-100:457:93//AC005740
R-HEMBA1001672//Homo sapiens methyl-CpG binding protein MBD3 (MBD3) mRNA, complete cds.//1.2e-90:496:91//AF072247
R-HEMBA1001675
R-HEMBA1001678//Homo sapiens voltage dependent anion channel protein mRNA, complete cds.//1.3e-101:534:94//AF038962
R-HEMBA1001681//CIT-HSP-2345M7.TF CIT-HSP Homo sapiens genomic clone 2345M7, genomic survey sequence.//0.21:124:68//AQ056593
R-HEMBA1001702//Homo sapiens 12q13.1 PAC RPCI1-228P16 (Roswell Park Cancer Institute Human PAC Library) complete sequence.//8.3e-06:279:63//AC004801
R-HEMBA1001709//Homo sapiens mRNA for KIAA0698 protein, complete cds.//1.9e-96:483:96//AB014598
R-HEMBA1001711//Human HepG2 3' region cDNA, clone hmd2b02.//2.3e-31:169:100//D16886
R-HEMBA1001712//HS-1015-B1-E01-MR.abi CIT Human Genomic Sperm Library C Homo sapiens genomic clone Plate=CT 790 Col=1 Row=J, genomic survey sequence.//0.0025:200:65/B32577
R-HEMBA1001714//Rattus norvegicus mitochondrial ATPase inhibitor gene, complete cds.//6.6e-27:316:75//U12250
R-HEMBA1001718//CIT-HSP-2171J2.TR CIT-HSP Homo sapiens genomic clone 2171J2, genomic survey sequence.//3.1e-41:167:87//B89781
R-HEMBA1001723//Rattus norvegicus EH domain binding protein Epsin mRNA, complete cds.//0.53:275:61//AF018261
R-HEMBA1001731//Human DNA sequence *** SEQUENCING IN PROGRESS *** from clone 322P7, WORKING DRAFT SEQUENCE.//2.9e-48:292:84//AL023799
R-HEMBA1001734//Homo sapiens Chromosome 15q22.3-23 PAC 88m3, WORKING DRAFT SEQUENCE, 2 ordered pieces.//3.2e-33:290:81//AC005959
R-HEMBA1001744//Human DNA sequence from clone 134E15 on chromosome 6q21 Contains Blimp-1, apoptosis specific protein similar to yeast APG5 ESTs, GSSs and retroviral sequence, complete sequence.//0.98:203:62//AL022067
R-HEMBA1001745//Homo sapiens BAC clone RG298G08 from 7p15-p21, complete sequence.//0.00019:312:59//AC005084
R-HEMBA1001746//Plasmodium falciparum 3D7 chromosome 12 PFYAC812 genomic sequence, WORKING DRAFT SEQUENCE, 8 unordered pieces.//0.045:457:61//AC004153
R-HEMBA1001761//Homo sapiens chromosome X, clone hCIT.200_L_4, complete sequence.//3.8e-39:331:80//AC006121
R-HEMBA1001781//Homo sapiens Xp22 BAC GSHB-590J6 (Genome Systems Human BAC library) complete sequence.//0.0062:245:60//AC004554
R-HEMBA1001784//Homo sapiens chromosome 5p, BAC clone 50g21 (LBNL H154), complete sequence.//2.1e-22:370:63//AC005740
R-HEMBA1001791//Human DNA sequence from clone 931E15 on chromosome Xq25. Contains STSs, GSSs and genomic marker DXS8098, complete sequence.//3.0e-50:408:80//AL023575
R-HEMBA1001800//CIT-HFP-2049N5.TF CIT-HSP Homo sapiens genomic clone 2049N5, genomic survey sequence.//9.0e-37:335:77//AQ009222
R-HEMBA1001803//Plasmodium falciparum 3D7 chromosome 12 PFYAC357 genomic sequence, WORKING DRAFT SEQUENCE, 7 unordered pieces.//0.86:536:56//AC005506
R-nnnnnnnnnnnn//Mouse interleukin 2 receptor (p55 IL-2R) mRNA, 5' end.//2.9e-93:553:89//M21977
R-HEMBA1001808//Homo sapiens mRNA, chromosome 1 specific transcript KIAA0500.//2.8e-112:548:98//AB007969
R-HEMBA1001809
R-HEMBA1001815//Homo sapiens Xp22 BAC GS-321G17 (Genome Systems Human BAC library) complete sequence.//2.6e-48:363:84//AC004025
R-HEMBA1001819//Homo sapiens *** SEQUENCING IN PROGRESS *** from PAC 1577, WORKING DRAFT SEQUENCE.//1.1e-15:275:68//AJ009612
R-HEMBA1001820//HS_3022_B1_A09_T7 CIT Approved Human Genomic Sperm Library D Homo sapiens genomic clone Plate=3022 Col=17 Row=B, genomic survey sequence.//0.00054:335:59//AQ165107
R-nnnnnnnnnnnn//Xenopus laevis intersectin mRNA, complete cds.//1.4e-19:533:63//AF032118
R-HEMBA1001824//S.clavuligerus linear plasmid pSCL (complete sequence).//0.62:189:65//X54107
R-HEMBA1001835//Human DNA sequence *** SEQUENCING IN PROGRESS *** from clone 191J18, WORKING DRAFT SEQUENCE.//1.0:450:60//AL024507
R-HEMBA1001844//Human familial Alzheimer's disease (STM2) gene, complete cds.//1.6e-07:170:68//U50871
R-HEMBA1001847
R-HEMBA1001861//Homo sapiens mRNA for KIAA0617 protein, complete cds.//3.3e-108:553:96//AB014517
R-HEMBA1001864//Homo sapiens genomic DNA, 21q22.1 region, clone: Q82F5A16, genomic survey sequence.//1.7e-14:245:67//AG002463
R-HEMBA1001866//HS_2258_B2_D01_MR CIT Approved Human Genomic Sperm Library D Homo sapiens genomic clone Plate=2258 Col=2 Row=H, genomic survey sequence.//2.8e-39:397:75//AQ221138
R-nnnnnnnnnnnn//Homo sapiens BAC clone RG114B19 from 7q31.1, complete sequence.//5.9e-56:303:94//AC005065
R-HEMBA1001888//Homo sapiens Xp22-150 BAC GSHB-309P15 (Genome Systems Human BAC Library) complete sequence.//1.7e-43:281:88//AC006210
R-HEMBA1001896
R-HEMBA1001910
R-HEMBA1001912//Homo sapiens chromosome 5, P1 clone 1308e5 (LBNL H13), complete sequence.//0.10:307:61//AC004775
R-HEMBA1001913
R-HEMBA1001915//HS_2037_A1_E12_MR CIT Approved Human Genomic Sperm Library D Homo sapiens genomic clone Plate=2037 Col=23 Row=I, genomic survey sequence.//0.071:206:64//AQ233106
R-HEMBA1001918//Homo sapiens chromosome 5, P1 clone 1308e5 (LBNL H13), complete sequence.//0.97:449:59//AC004775
R-HEMBA1001921//Homo sapiens germinal center kinase related protein kinase mRNA, complete cds.//2.0e-105:534:96//AF000145
R-HEMBA1001939//Human DNA sequence *** SEQUENCING IN PROGRESS *** from clone 508I15, WORKING DRAFT SEQUENCE.//4.6e-13:120:82//AL021707
R-HEMBA1001940//Homo sapiens clone DJ1093I16, WORKING DRAFT SEQUENCE, 5 unordered pieces.//2.2e-36:301:81//AC005629
R-HEMBA1001942//Human PAC clone DJ0205E24 from Xq23, complete sequence.//1.9e-10:208:68//AC003013
R-HEMBA1001945//Plasmodium falciparum chromosome 2, section 70 of 73 of the complete sequence.//1.2e-06:393:60//AE001433
R-HEMBA1001950//R.prowazekii genomic DNA fragment (clone A437R).//0.33:122:66//Z82646
R-HEMBA1001960//Borrelia afzelii VS461 outer surface protein D (ospD) gene, complete cds.//0.0086:427:59//U05329
R-HEMBA1001962//Homo sapiens chromosome 4 clone B71M12 map 4q25, complete sequence.//4.5e-07:176:70//AC004069
R-HEMBA1001964//HS_2215_B1_H01_MR CIT Approved Human Genomic Sperm Library D Homo sapiens genomic clone Plate=2215 Col=1 Row=P, genomic survey sequence.//7.3e-25:215:74//AQ151931
R-HEMBA1001967//Human DNA sequence from clone 341E18 on chromosome 6p11.2-12.3. Contains a Serine/Threonine Protein Kinase gene (presumptive isolog of a Rat gene) and a novel alternatively spliced gene. Contains a putative CpG island, ESTs and GSSs, complete sequence.//1.7e-51:209:95//AL031178
R-HEMBA1001979//CIT-HSP-2387I12.TF.1 CIT-HSP Homo sapiens genomic clone 2387I12, genomic survey sequence.//4.9e-06:153:71//AQ240461
R-HEMBA1001987//Human DNA sequence from clone 444C7 on chromosome 6p22.3-23. Contains an EST, an STS and GSSs, complete sequence.//3.1e-46:437:77//AL033521
R-HEMBA1001991//Human DNA sequence from PAC 426I6 on chromosome 1p34.1-1p35. Contains NIPP-1-like gene a nuclear inhibitor of protein phosphatase-1, ESTs, and a CA repeat.//1.1e-48:446:78//AL020997
R-HEMBA1002003//Homo sapiens mRNA for protein phosphatase 2C (beta).//5.1e-90:448:97//AJ005801
R-HEMBA1002008//Homo sapiens DNA sequence from PAC 95C20 on chromosome Xp11.3-11.4. Contains STSs and the DXS7 locus with GT and GTG repeat polymorphisms, complete sequence.//3.2e-42:317:84//Z97181
R-HEMBA1002018//HS_3006_B1_D10_T7 CIT Approved Human Genomic Sperm Library D Homo sapiens genomic clone Plate=3006 Col=19 Row=H, genomic survey sequence.//1.0:63:74//AQ089717
R-HEMBA1002022//Homo sapiens chromosome 18, clone hRPK.453_M_1, complete sequence.//0.93:339:59//AC006203
R-HEMBA1002035//Mus musculus chromosome 19, clone CIT282B21, complete sequence.//1.4e-11:285:67//AC003694
R-HEMBA1002039
R-HEMBA1002049//Human DNA sequence *** SEQUENCING IN PROGRESS *** from clone 1177I5, WORKING DRAFT SEQUENCE.//5.3e-52:266:84//AL022315
R-HEMBA1002084//CIT-HSP-2357Ll1.TR CIT-HSP Homo sapiens genomic clone 2357L11, genomic survey sequence.//0.0013:185:66//AQ063078
R-HEMBA1002092//Mus musculus Olf-1/EBF-like-3 transcription factor (O/E-3) mRNA, complete cds.//2.7e-70:479:86//U92703
R-HEMBA1002100//Homo sapiens thyroid receptor interactor (TRIP7) mRNA, 3' end of cds.//8.5e-32:206:91//L40357
R-HEMBA1002102//Homo sapiens Chromosome 15q26.1 PAC clone pDJ427d15, complete sequence.//4.3e-42:302:85//AC005800
R-HEMBA1002113//Human chromosome 12p13 sequence, complete sequence.//1.6e-64:550:80//U47924
R-HEMBA1002119//Human Chromosome 11 pac pDJ1173a5, complete sequence.//1.2e-92:435:92//AC000378
R-HEMBA1002125
R-HEMBA1002139//Human nebulin mRNA, partial cds.//0.056:68:88//U35637
R-HEMBA1002144//Homo sapiens Chromosome 11p14.3 PAC clone 6-130a9 containing tryptophan hydroxylase gene, complete sequence.//2.0e-26:323:70//AC005728
R-HEMBA1002150//Human DNA sequence from clone 742C19 on chromosome 22q12.3-13.1. Contains a pseudogene similar to Cytochrome C Oxidase Polypeptide VB and (parts of) up to four novel genes, two with homology to Phorbolin genes and one a novel Chromobox protein gene. Contains ESTs, an STS, GSSs and putative CpG islands, complete sequence.//1.0:371:61//AL031846
R-HEMBA1002151
R-HEMBA1002153//Human BAC 367D17 from chromosome 18, complete sequence.//2.4e-21:322:70//AC003971
R-HEMBA1002l60//Human DNA sequence from PAC 339A18 on chromosome Xp11.2. Contains KIAA0178 gene, similar to mitosis-specific chromosome segregation protein SMC1 of S.cerevisiae, DNA binding protein similar to URE-B1, ESTs and STS.//2.5e-38:216:84//Z97054
R-HEMBA1002161//CIT-HSP-2163F10.TF CIT-HSP Homo sapiens genomic clone 2163F10, genomic survey sequence.//3.1e-58:284:80//B89969
R-HEMBA1002162//Caenorhabditis elegans cosmid F48C11, complete sequence.//0.0079:286:57//Z80789
R-HEMBA1002166//Homo sapiens Xp22 BAC 620F15 (Genome Systems BAC library) complete sequence.//5.9e-53:326:80//AC002980
R-HEMBA1002177
R-HEMBA1002185//Human DNA sequence *** SEQUENCING IN PROGRESS *** from clone 745I14, WORKING DRAFT SEQUENCE.//9.5e-37:356:76//AL033532
R-HEMBA1002189//Homo sapiens Xp22 BAC GSHB-519E5 (Genome Systems Human BAC library) complete sequence.//3.4e-43:244:77//AC003684
R-HEMBA1002191//Homo sapiens clone RG228D17, WORKING DRAFT SEQUENCE, 2 unordered pieces.//4.3e-37:323:78//AC005077
R-HEMBA1002199//Human Cosmid g5129g124 from 7q31.3, complete sequence.//1.4e-89:564:87//AC002498
R-HEMBA1002204//Homo sapiens Chromosome 22q11.2 Cosmid Clone 817g In IGLC Region, complete sequence.//1.5e-31:313:71//AC000053
R-HEMBA1002212//K.lactis mitochondrial COX1 and A8 genes for cytochrome oxidase subunit I and ATPase subunit 8.//0.0023:346:60//X57546
R-HEMBA1002215//M.musculus mRNA for testin.//4.7e-61:414:84//X78989
R-HEMBA1002226//Human DNA sequence *** SEQUENCING IN PROGRESS *** from clone 2705, WORKING DRAFT SEQUENCE.//4.6e-46:375:77//AL033529
R-HEMBA1002229//Homo sapiens growth suppressor related (DOC-1R) mRNA, complete cds.//4.6e-46:238:98//AF089814
R-HEMBA1002237//Homo sapiens 12q13 PAC RPCI1-316M24 (Roswell Park Cancer Institute Human PAC library) complete sequence.//4.3e-26:469:67//AC004242
R-HEMBA1002253//Homo sapiens BAC clone GS180J15 from 7q31, complete sequence.//5.1e-23:162:82//AC005016
R-HEMBA1002257
R-HEMBA1002267//Equus caballus dermatan sulfate proteoglycan II mRNA, complete cds.//4.6e-44:300:88//AF03 8127
R-HEMBA1002270//Human BAC clone RG067M09 from 7q21-7q22, complete sequence.//1.9e-19:176:85//AC000057
R-HEMBA1002321
R-HEMBA1002328//HS_3061_A1_D06_T7 CIT Approved Human Genomic Sperm Library D Homo sapiens genomic clone Plate=3061 Col=11 Row=G, genomic survey sequence.//1.0:151:65//AQ127617
R-HEMBA1002337//Saccharomyces cerevisiae RNA polymerase II holoenzyme component (SRB7) gene, complete cds.//3.7e-07:328:63//U23811
R-HEMBA1002341//Homo sapiens mRNA for KIAA0771 protein, partial cds.//2.4e-128:642:96//AB018314
R-HEMBA1002348//Human DNA sequence from clone 409O10 on chromosome 20q12 Contains CA repeat, GSS, STS, complete sequence.//3.7e-07:587:58//AL031256
R-HEMBA1002349//Leishmania tarentolae maxicircle DNA fragment.//0.018:341:58//X02438
R-nnnnnnnnnnnn//Homo sapiens chromosome-associated protein-E (hCAP-E) mRNA, complete cds.//1.2e-121:661:93//AF092563
R-HEMBA1002381//Homo sapiens genomic DNA of 8p21.3-p22 anti-oncogene of hepatocellular colorectal and non-small cell lung cancer , segment 11/11.//1.1e-70:559:79//AB020868
R-HEMBA1002389//HS_3218_B2_E08_MR CIT Approved Human Genomic Sperm Library D Homo sapiens genomic clone Plate=3218 Col=16 Row=J, genomic survey sequence.//0.0011:122:72//AQ213602
R-HEMBA1002417//Homo sapiens chromosome 19, cosmid R28784, complete sequence.//4.2e-81:232:97//AC005954
R-HEMBA1002419//Homo sapiens PAC clone DJ0649P17 from 7q11.23-q21, complete sequence.//0.50:231:64//AC004848
R-HEMBA1002430//P.falciparum complete gene map of plastid-like DNA (IR-B).//0.0023:604:56//X95276
R-HEMBA1002439//Homo sapiens clone GS096J14, WORKING DRAFT SEQUENCE, 3 unordered pieces.//3.4e-23:183:80//AC006026
R-HEMBA1002458//Human DNA sequence from clone 146H21 on chromosome Xq22 Contains cleavage stimulation factor, 64 KD subunit, gene similar to CYTOCHROME B-245 HEAVY CHAIN. pseudogene similar to hnRNP A1 protein and ESTs, complete sequence.//7.7e-32:161:83//Z83819
R-HEMBA1002460//Homo sapiens clone DJ1137M13, complete sequence.//2.6e-100:305:100//AC005378
R-HEMBA1002462//Sequence 43 from patent US 5708157.//2.0e-10:131:77//I80068
R-nnnnnnnnnnnn
R-HEMBA1002477//Homo sapiens PAC clone DJ0607J23 from 7q21.2-q31.1, complete sequence.//6.6e-33:279:80//AC004841
R-HEMBA1002486//***ALU WARNING: Human Alu-Sq subfamily consensus sequence.//2.1e-50:290:92//U14573
R-HEMBA1002495//CITBI-E1-2515J10.TR CITBI-E1 Homo sapiens genomic clone 2515J10, genomic survey sequence.//1.0:122:68//AQ261762
R-HEMBA1002498//Homo sapiens clone DJ1102A12, WORKING DRAFT SEQUENCE, 15 unordered pieces.//2.8e-22:210:78//AC004963
R-HEMBA1002503//Homo sapiens chromosome 17, clone HRPC1067M6, complete sequence.//2.7e-17:435:58//AC003043
R-HEMBA1002508//Homo sapiens, clone hRPK.15_A_1, complete sequence.//3.7e-09:408:61//AC006213
R-nnnnnnnnnnnn//Homo sapiens mRNA for histone deacetylase-like protein (JM21).//7.1e-112:456:92//AJ011972
R-HEMBA1002515
R-HEMBA1002538//Homo sapiens mRNA for KIAA0454 protein, partial cds.//1.6e-104:564:93//AB007923
R-HEMBA1002542//HS_3197_B2_B10_T7 CIT Approved Human Genomic Sperm Library D Homo sapiens genomic clone Plate=3197 Col=20 Row=D, genomic survey sequence.//2.8e-25:186:86//AQ188792
R-HEMBA1002547//Mus musculus agrin gene, exon 36.//0.0095:93:75//M92658
R-HEMBA1002552//Homo sapiens clone DJ1137M13, complete sequence.//4.0e-49:308:90//AC005378
R-HEMBA1002555//Homo sapiens full-length insert cDNA clone YR87G10.//8.3e-65:318:99//AF085957
R-HEMBA1002558//, complete sequence.//2.3e-38:264:89//AC005409
R-HEMBA1002561//Human DNA sequence from clone 396D17 on chromosome 1p33-35.3 Contains EST, STS, GSS, complete sequence.//7.1e-44:192:80//AL008634
R-nnnnnnnnnnnn//Homo sapiens protein associated with Myc mRNA, complete cds.//4.5e-119:587:97//AF075587
R-HEMBA1002583
R-HEMBA1002590//Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence.//9.4e-42:248:88//Z95152
R-HEMBA1002592//Homo sapiens chromosome 19, cosmid R30385, complete sequence.//2.6e-56:302:84//AC004510
R-HEMBA1002621
R-HEMBA1002624//Homo sapiens mRNA for KIAA0808 protein, complete cds.//6.7e-76:380:97//AB018351
R-HEMBA1002628//P.falciparum complete gene map of plastid-like DNA (IR-A).//8.8e-05:327:60//X95275
R-HEMBA1002629//Mus musculus clone OST16705, genomic survey sequence.//4.3e-06:205:66//AF046247
R-HEMBA1002645//***ALU WARNING: Human Alu-J subfamily consensus sequence.//7.1e-39:281:84//U14567
R-HEMBA1002651//Homo sapiens PAC clone DJ0593H12 from 7p31, complete sequence.//1.1e-104:500:95//AC004839
R-HEMBA1002659//Human DNA sequence from clone 243E7 on chromosome 22q12.1. Contains ESTs, STSs and GSSs, complete sequence.//1.2e-61:280:92//AL022323
R-HEMBA1002661//Human DNA sequence *** SEQUENCING IN PROGRESS *** from clone 225E12, WORKING DRAFT SEQUENCE.//3.2e-41:325:81//AL031772
R-HEMBA1002666//Homo sapiens full-length insert cDNA clone YY74A07.//0.00037:79:84//AF088008
R-HEMBA1002678//Human DNA sequence *** SEQUENCING IN PROGRESS *** from clone 1137F22, WORKING DRAFT SEQUENCE.//2.3e-107:561:94//AL034421
R-nnnnnnnnnnnn//CIT-HSP-2287E8.TF CIT-HSP Homo sapiens genomic clone 2287E8, genomic survey sequence.//5.4e-17:137:88//B99281
R-HEMBA1002688//Homo sapiens chromosome 5, P1 clone 1354A7 (LBNL H47), complete sequence.//0.033:146:70//AC004503
R-HEMBA1002696
R-HEMBA1002712//Homo sapiens PAC clone 166H1 from 12q, complete sequence.//6.2e-44:302:87//AC003982
R-HEMBA1002716//Mus musculus mRNA for ELM1, complete cds.//1.1e-31:332:76//AB004873
R-HEMBA1002728//Homo sapiens mRNA for KIAA0621 protein, partial cds.//1.2e-35:287:81//AB014521
R-HEMBA1002730//D.discoideum actin M6 gene, 5' flank.//0.018:233:66//M29109
R-HEMBA1002742//Human DNA sequence *** SEQUENCING IN PROGRESS *** from clone 1108H3, WORKING DRAFT SEQUENCE.//2.6e-13:419:62//AL033525
R-HEMBA1002746//Mus musculus chromosome 19, clone CIT282B21, complete sequence.//0.019:202:65//AC003694
R-HEMBA1002748//Human DNA sequence *** SEQUENCING IN PROGRESS *** from clone 404K8, WORKING DRAFT SEQUENCE.//0.046:263:60//AL023883
R-HEMBA1002750//Human DNA sequence from PAC 452H17 on chromosome X contains sodium-and chloride-dependent glycine transporter 1 (GLYT-1) like, ESTs.//0.052:421:58//Z96810
R-HEMBA1002768//Homo sapiens mRNA for KIAA0554 protein, partial cds.//1.2e-104:545:95//AB011126
R-HEMBA1002770//Plasmodium falciparum 3D7 chromosome 12 PFYACB8-420 genomic sequence, WORKING DRAFT SEQUENCE, 14 unordered pieces.//3.0e-07:523:59//AC005140
R-HEMBA1002777
R-HEMBA1002779//Human HepG2 3' region MboI cDNA, clone hmd1e03m3.//9.4e-25:158:93//D17139
R-HEMBA1002780//Human DNA sequence *** SEQUENCING IN PROGRESS *** from clone Y214H10, WORKING DRAFT SEQUENCE.//1.6e-42:463:75//AL022344
R-HEMBA1002794//Plasmodium falciparum MAL3P8, complete sequence.//2.2e-05:417:59//AL034560
R-HEMBA1002801//Meloidogyne javanica mitochondrial transfer RNA His, 16S ribosomal RNA (16S rRNA) genes, ND3 gene, complete cds, and cytochrome b gene, 5' end of CDS.//0.00055:444:59//L76261
R-HEMBA1002810//Homo sapiens formin binding protein 21 mRNA, complete cds.//4.4e-115:559:97//AF071185
R-HEMBA1002816//Homo sapiens clone NH0576N21, WORKING DRAFT SEQUENCE, 5 unordered pieces.//4.3e-88:329:94//AC005043
R-HEMBA1002826//Homo sapiens genomic DNA, chromosome 21q11.1, segment 12/28, WORKING DRAFT SEQUENCE.//1.9e-22:262:67//AP000041
R-HEMBA1002833//Homo sapiens chromosome 17, clone hRPC.117_B_12, complete sequence.//1.3e-79:396:97//AC004707
R-HEMBA1002850//Plasmodium falciparum 3D7 chromosome 12 PFYAC357 genomic sequence, WORKING DRAFT SEQUENCE, 7 unordered pieces.//0.013:393:61//AC005506
R-HEMBA1002863//Homo sapiens chromosome 17, clone hRPK.271_K_11, complete sequence.//4.1e-73:489:85//AC005562
R-HEMBA1002876//Plasmodium falciparum DNA *** SEQUENCING IN PROGRESS *** from MAL4P1, WORKING DRAFT SEQUENCE.//0.21:549:55//AL034557
R-HEMBA1002886//CIT-HSP-2013C4.TR CIT-HSP Homo sapiens genomic clone 2013C4, genomic survey sequence.//0.30:431:56//B53836
R-HEMBA1002896//Homo sapiens SH3-containing adaptor molecule-1 mRNA, complete cds.//3.9e-106:541:95//AF037261
R-HEMBA1002921
R-HEMBA1002924//Homo sapiens genomic DNA of 9q32 anti-oncogene of flat epitherium cancer, segment 7/10.//4.6e-19:139:78//AB020875
R-HEMBA1002934//Human DNA sequence *** SEQUENCING IN PROGRESS *** from clone 862K6, WORKING DRAFT SEQUENCE.//7.5e-45:282:89//AL031681
R-HEMBA1002935//CIT-HSP-2282P14.TFB CIT-HSP Homo sapiens genomic clone 2282P14, genomic survey sequence.//1.5e-102:514:97//AQ008584
R-HEMBA1002937//Human DNA sequence *** SEQUENCING IN PROGRESS *** from clone 745114, WORKING DRAFT SEQUENCE.//3.3e-87:444:97//AL033532
R-HEMBA1002939
R-HEMBA1002944//HS_3107_A1_C05_T7 CIT Approved Human Genomic Sperm Library D Homo sapiens genomic clone Plate=3107 Col=9 Row=E, genomic survey sequence.//6.3e-21:250:73//AQ103952
R-HEMBA1002951//Xerolycosa miniata mitochondrial 12S rRNA gene.//0.013:228:63//AJ008020
R-HEMBA1002954//HS_3246_A2_G09_MR CIT Approved Human Genomic Sperm Library D Homo sapiens genomic clone Plate=3246 Col=18 Row=M, genomic survey sequence.//5.8e-42:258:91//AQ218005
R-HEMBA1002968//Homo sapiens chromosome 17, clone hRPK.112_J_9, complete sequence.//4.2e-38:300:83//AC005553
R-HEMBA1002970//Slime mold (D.discoideum) prestalk D11 gene, complete cds.//5.0e-05:541:57//M11012
R-HEMBA1002971//Homo sapiens mRNA for KIAA0679 protein, partial cds.//7.2e-29:162:99//AB014579
R-HEMBA1002973//Homo sapiens chromosome 19, cosmid F20900, complete sequence.//9.1e-36:520:69//AC006128
R-nnnnnnnnnnnn//Homo Sapiens Chromosome X clone bWXD691, complete sequence.//0.00040:504:59//AC004386
R-HEMBA1002999//Rattus norvegicus lamina-associated polypeptide 1C (LAP1C) mRNA, complete cds.//3.7e-66:556:79//U19614
R-HEMBA1003021//Human Chromosome 11 overlapping pacs pDJ235k10 and pDJ239b22, WORKING DRAFT SEQUENCE, 17 unordered pieces.//1.6e-44:530:70//AC000406
R-HEMBA1003033//Homo sapiens full-length insert cDNA clone ZC34B10.//4.6e-78:414:94//AF086194
R-HEMBA1003034//Homo sapiens chromosome 19, cosmid R29351, complete sequence.//9.0e-52:322:75//AC004026
R-HEMBA1003035//HS_2008_A2_G08_T7 CIT Approved Human Genomic Sperm Library D Homo sapiens genomic clone Plate=2008 Col=16 Row=M, genomic survey sequence.//4.0e-68:343:97//AQ269839
R-HEMBA1003037//347G15.TVB CIT978SKA1 Homo sapiens genomic clone A-347G15, genomic survey sequence.//0.57:188:58//B17694
R-HEMBA1003041//Homo sapiens PAC clone DJ1163J12 from 7q21.2-q31.1, complete sequence.//6.3e-30:350:72//AC004983
R-HEMBA1003046//Homo sapiens mitochondrial processing peptidase beta-subunit mRNA, complete cds.//4.1e-118:578:97//AF054182
R-HEMBA1003064//Human cosmid LL12NC01-N-136B11, located centromeric to the ETV6 gene, chromosome 12p12-13.//0.0018:271:60//U59962
R-HEMBA1003067//Human DNA sequence *** SEQUENCING IN PROGRESS *** from clone 633019, WORKING DRAFT SEQUENCE.//5.3e-48:464:76//AL022302
R-HEMBA1003071//CIT-HSP-2370D6.TR CIT-HSP Homo sapiens genomic clone 2370D6, genomic survey sequence.//0.19:48:87//AQ110136
R-HEMBA1003077//Rattus norvegicus Shal-related potassium channel Kv4.3 mRNA, complete cds.//4.9e-69:494:84//U42975
R-HEMBA1003078//Human DNA sequence from PAC 339A18 on chromosome Xp11.2. Contains KIAA0178 gene, similar to mitosis-specific chromosome segregation protein SMC1 of S.cerevisiae, DNA binding protein similar to URE-B1, ESTs and STS.//1.1e-11:331:64//Z97054
R-HEMBA1003079//Homo sapiens Xp22-132-134 BAC GSHB-590J15 (Genome Systems Human BAC library) complete sequence.//4.6e-116:576:98//AC004673
R-HEMBA1003083//*** SEQUENCING IN PROGRESS *** Homo sapiens chromosome 4, BAC clone C0442P12; HTGS phase 1, WORKING DRAFT SEQUENCE, 5 unordered pieces.//3.1e-43:280:83//AC005798
R-HEMBA1003086//Homo sapiens clone NH0319F03, WORKING DRAFT SEQUENCE, 3-unordered pieces.//1.2e-43:281:88//AC006039
R-HEMBA1003096//Human DNA sequence from clone J506G21, WORKING DRAFT SEQUENCE.//0.00037:421:59//Z82213
R-HEMBA1003098//*** SEQUENCING IN PROGRESS *** Homo sapiens chromosome 4, BAC clone C0024K08; HTGS phase 1, WORKING DRAFT SEQUENCE, 5 unordered pieces.//1.4e-30:303:78//AC005598
R-HEMBA1003117
R-HEMBA1003129//Human DNA sequence *** SEQUENCING IN PROGRESS *** from clone 407F11, WORKING DRAFT SEQUENCE.//7.9e-11:109:85//AL022329
R-HEMBA1003133//Homo sapiens chromosome 9, P1 clone 11659, complete sequence.//3.9e-99:484:98//AC004472
R-HEMBA1003136//CIT-HSP-2281L22.TF CIT-HSP Homo sapiens genomic clone 2281L22, genomic survey sequence.//2.0e-10:93:92//B99861
R-HEMBA1003142//Homo sapiens 12q24.2 PAC RPCI1-128M12 (Roswell Park Cancer Institute Human PAC library) complete sequence.//9.8e-40:270:87//AC004024
R-HEMBA1003148//Homo sapiens mRNA for dachshund protein.//1.1e-116:586:96//AJ005670
R-HEMBA1003166//Human DNA sequence from PAC 306D1 on chromosome X contains ESTs.//6.4e-35:364:70//Z83822
R-HEMBA1003175//Human IFNAR gene for interferon alpha/beta receptor.//1.9e-30:282:77//X60459
R-HEMBA1003197
R-HEMBA1003199//HS_2166_A1_E12_MR CIT Approved Human Genomic Sperm Library D Homo sapiens genomic clone Plate=2166 Col=23 Row=I, genomic survey sequence.//0.00026:271:61//AQ164162
R-HEMBA1003202//Homo sapiens clone DJ0592G07, WORKING DRAFT SEQUENCE, 3 unordered pieces.//5.4e-44:291:83//AC005480
R-HEMBA1003204//Human BAC clone RG072E11 from 7q21-7q22, complete sequence.//3.1e-10:293:62//AC000118
R-HEMBA1003212//Homo sapiens clone DJ0902E20, WORKING DRAFT SEQUENCE, 1 unordered pieces.//1.0:118:69//AC006148
R-HEMBA1003220//HS_3092_B1_F09_MR CIT Approved Human Genomic Sperm Library D Homo sapiens genomic clone Plate=3092 Col=17 Row=L, genomic survey sequence.//0:00014:59:91//AQ128202
R-HEMBA1003222//Caenorhabditis elegans DNA *** SEQUENCING IN PROGRESS *** from clone Y43F8, WORKING DRAFT SEQUENCE.//0.84:214:62//Z95393
R-HEMBA1003229//RPCI11-16F15.TPB RPCI-11 Homo sapiens genomic clone RPCI-11-16F15, genomic survey sequence.//0.42:167:64//B83610
R-HEMBA1003235//CIT-HSP-2320G19.TF CIT-HSP Homo sapiens genomic clone 2320G19, genomic survey sequence.//3.6e-36:195:81//AQ037231
R-HEMBA1003250//HS_2168_A2_C09_MF CIT Approved Human Genomic Sperm Library D Homo sapiens genomic clone Plate=2168 Col=18 Row=E, genomic survey sequence.//1.4e-22:158:89//AQ125356
R-HEMBA1003257//Human PCP4 gene, exon 3 and complete cds.//0.96:268:61//U53709
R-HEMBA1003273//Homo sapiens Xp22 BAC GS-377014 (Genome Systems Human BAC library) complete sequence.//1.0e-32:255:84//AC002549
R-HEMBA1003276//Plasmodium falciparum 3D7 chromosome 12 PFYAC492 genomic sequence, WORKING DRAFT SEQUENCE, 5 unordered pieces.//0.0044:212:60//AC005308
R-HEMBA1003278//Homo sapiens 12q24.1 PAC RPCI1-315L5 (Roswell Park Cancer Institute Human PAC library) complete sequence.//1.1e-34:286:74//AC002395
R-HEMBA1003281//High throughput sequencing of human chromosome 12, WORKING DRAFT SEQUENCE, 1 ordered pieces.//1.8e-53:428:83//AC005840
R-HEMBA1003291//Homo sapiens mRNA for KIAA0537 protein, complete cds.//3.0e-115:551:99//AB011109
R-HEMBA1003296//CIT-HSP-2196L16.TR CIT-HSP Homo sapiens genomic clone 2196L16, genomic survey sequence.//2.9e-20:337:65//AQ003073
R-HEMBA1003304//Sequence 23 from patent US 5552281.//1.8e-31:179:97//I25662
R-HEMBA1003309//Arabidopsis thaliana genomic DNA, chromosome 5, TAC clone: K19E20, complete sequence.//0.00019:334:60//AB017061
R-HEMBA1003314//Homo sapiens mRNA for leucine zipper bearing kinase, complete cds.//2.8e-111:545:97//AB001872
R-HEMBA1003322//Human DNA sequence from clone 23K20 on chromosome Xq25-26.2 Contains EST, STS, GSS, complete sequence.//0.60:274:61//AL022153
R-HEMBA1003327//Homo sapiens BAC clone RG351J01 from 7q22-q31, complete sequence.//0.00028:172:65//AC005099
R-HEMBA1003328//Homo sapiens clone RG270D13, WORKING DRAFT SEQUENCE, 18 unordered pieces.//2.2e-44:268:90//AC005081
R-HEMBA1003330//Homo sapiens poly(A) binding protein II (PABP2) gene, complete cds.//2.7e-61:312:97//AF026029
R-HEMBA1003348//***ALU WARNING: Human Alu-J subfamily consensus sequence.//7.2e-38:186:83//U14567
R-HEMBA1003369//Caenorhabditis elegans cosmid F59C6, complete sequence.//0.00012:465:59//Z79600
R-HEMBA1003370//Homo sapiens chromosome 17, clone hRPC867C24, complete sequence.//3.2e-42:301:87//AC002558
R-HEMBA1003373//Human DNA sequence from clone 109F14 on chromosome 6p21.2-21.3. Contains the alternatively spliced gene for Transcriptional Enhancer Factor TEF-5, the 60S Ribosomal Protein RPL10A gene, a PUTATIVE ZNF127 LIKE gene, and the PPARD for Peroxisome Proliferator Activated Receptor Delta (PPAR-Delta, PPAR-Beta, Nuclear Hormone Receptor 1, NUC1, NUCI, PPARB). Contains three putative CpG islands, ESTs, STSs, GSSs and a ca repeat polymorphism, complete sequence.//7.4e-34:375:74//AL022721
R-HEMBA1003376//Homo sapiens chromosome 16, cosmid clone RT102 (LANL), complete sequence.//1.6e-46:309:88//AC004651
R-HEMBA1003380//HS_3184_B2_E06_MR CIT Approved Human Genomic Sperm Library D Homo sapiens genomic clone Plate=3184 Col=12 Row=J, genomic survey sequence.//1.0e-35:237:88//AQ189144
R-HEMBA1003384//HS_2193_B2_H08_MR CIT Approved Human Genomic Sperm Library D Homo sapiens genomic clone Plate=2193 Col=16 Row=P, genomic survey sequence.//0.00029:96:76//AQ032212
R-HEMBA1003395//Homo sapiens chromosome 17, clone HCIT169H9, WORKING DRAFT SEQUENCE, 6 unordered pieces.//2.6e-21:139:86//AC002993
R-HEMBA1003402//CIT-HSP-2166E19.TR CIT-HSP Homo sapiens genomic clone 2166E19, genomic survey sequence.//0.99:144:61//B91549
R-nnnnnnnnnnnn
R-HEMBA1003417//Human DNA sequence from clone 496N17 on chromosome 6p11.2-12.3 Contains EST, GSS, complete sequence.//2.5e-112:547:98//AL031321
R-HEMBA1003418//Homo sapiens PAC clone DJ0755G17 from 7p21-p22, complete sequence.//0.082:352:59//AC004879
R-HEMBA1003433//Homo sapiens cell cycle regulatory protein p95 (NBS1) mRNA, complete cds.//9.9e-114:544:98//AF058696
R-HEMBA1003461
R-HEMBA1003463
R-HEMBA1003480//Homo sapiens clone NH0523H20, complete sequence.//9.1e-106:533:96//AC005041
R-HEMBA1003528
R-HEMBA1003531//Human BAC clone GS552A01 from 7q21-q22, complete sequence.//3.4e-08:333:64//AC002454
R-HEMBA1003538//Human mRNA for complement component C1r.//1.4e-23:333:68//X04701
R-HEMBA1003545//Zebrafish mRNA for zflsl-2 (insulin gene enhancer binding protein homolog), complete cds.//0.030:144:68//D38453
R-HEMBA1003548//Plasmodium falciparum 3D7 chromosome 12 PFYAC812 genomic sequence, WORKING DRAFT SEQUENCE, 8 unordered pieces.//0.0017:487:57//AC004153
R-HEMBA1003555//Human DNA sequence *** SEQUENCING IN PROGRESS *** from clone 371H6, WORKING DRAFT SEQUENCE.//2.8e-99:503:96//AL031718
R-HEMBA1003556//Homo sapiens Xp22-175-176 BAC GSHB-484O17 (Genome Systems Human BAC Library) complete sequence.//1.6e-114:574:97//AC005913
R-HEMBA1003560//Diplolepis rosae microsatellite clone DR04096.//0.24:116:67//AF034416
R-HEMBA1003568//Homo sapiens clone NH0215P16, WORKING DRAFT SEQUENCE, 3 unordered pieces.//3.9e-05:422:63//AC006036
R-HEMBA1003569//Homo sapiens full-length insert cDNA clone ZD82D06.//8.7e-108:545:95//AF086450
R-HEMBA1003571//Homo sapiens PAC clone DJ0886O08 from 7q32-q35, complete sequence.//4.6e-51:570:71//AC004914
R-HEMBA1003579//HS_3237_B2_E05_T7 CIT Approved Human Genomic Sperm Library D Homo sapiens genomic clone Plate=3237 Col=10 Row=J, genomic survey sequence.//8.5e-97:495:95//AQ209302
R-HEMBA1003581//Mouse mRNA for talin.//8.3e-12:128:82//X56123
R-HEMBA1003591//Homo sapiens chromosome 16, BAC clone 2603 (LANL), complete sequence.//2.9e-87:251:95//AC005774
R-HEMBA1003595//Homo sapiens DNA sequence from BAC 1216H12 on chromosome 22q12. Contains a pseudogene with similarity to part of mouse Ninein and the KIAA0609 gene for a protein similar to C. elegans K09C8.4. Contains ESTs, GSSs and a ggtt repeat polymorphism, complete sequence.//4.5e-52:384:83//AL008715
R-HEMBA1003597//Homo sapiens DNA sequence from PAC 418A9 on chromosome 6q21. Contains the first (5') two exons of a CDK8 (Cell Division Protein Kinase 8) LIKE gene, a Neutral Calponin LIKE pseudogene, ESTs and STSs, complete sequence.//4.6e-41:442:74//Z84480
R-HEMBA1003598//Homo sapiens PAC clone DJ0537P09 from 7p11.2-p12, complete sequence.//1.8e-23:177:88//AC005153
R-HEMBA1003615
R-HEMBA1003617//Plasmodium falciparum 3D7 chromosome 12 PFYAC336 genomic sequence, WORKING DRAFT SEQUENCE, 5 unordered pieces.//0.039:494:57//AC005139
R-HEMBA100362111*** SEQUENCING IN PROGRESS *** Homo sapiens chromosome 4, BAC clone C0052I22; HTGS phase 1, WORKING DRAFT SEQUENCE, 4 unordered pieces.//2.3e-26:309:75//AC004599
R-HEMBA1003622//Homo sapiens Xp22 BAC 620F15 (Genome Systems BAC library) complete sequence.//7.1e-56:545:75//AC002980
R-HEMBA1003630//Homo sapiens CC chemokine gene cluster, complete sequence.//2.8e-32:546:68//AF088219
R-HEMBA1003637//Human BAC clone GS552A01 from 7q21-q22, complete sequence.//8.0e-25:457:68//AC002454
R-HEMBA1003640//Homo sapiens chromosome X, PAC 671D9, complete sequence.//2.8e-40:280:86//AF031078
R-HEMBA1003645//Human DNA sequence *** SEQUENCING IN PROGRESS *** from clone 32B1, WORKING DRAFT SEQUENCE.//1.7e-33:297:82//AL023693
R-HEMBA1003646//Plasmodium falciparum MAL3P7, complete sequence.//0.44:319:59//AL034559
R-HEMBA1003656//Homo sapiens Chromosome 16 BAC clone CIT987SK-A-152E5, complete sequence.//6.9e-36:242:80//AC004382
R-HEMBA1003662//Homo sapiens chromosome 17, clone hRPK.332_H_18, complete sequence.//8.6e-117:588:96//AC005746
R-HEMBA1003667//Sequence 8 from patent US 5420245.//1.8e-21:170:88//I12222
R-HEMBA1003679//Homo sapiens BAC clone RG114B19 from 7q31.1, complete sequence.//1.6e-22:180:87//AC005065
R-HEMBA1003680//C. elegans cosmid ZK353.//1.1e-06:270:61//L15313
R-HEMBA1003684//Colias alexandra alexandra cytochrome oxidase subunit I (cox1) gene, mitochondrial gene encoding mitochondrial protein, partial cds.//0.77:171:66//AF044872
R-HEMBA1003690//Homo sapiens 12q13.1 PAC RPCI5-1057I20 (Roswell Park Cancer Institute Human PAC library) complete sequence.//1.6e-104:523:97//AC004466
R-HEMBA1003692//Human DNA sequence *** SEQUENCING IN PROGRESS *** from clone 508I15, WORKING DRAFT SEQUENCE.//1.7e-41:414:77//AL021707
R-HEMBA1003711//Human Chromosome 11 overlapping pacs pDJ235k10 and pDJ239b22, WORKING DRAFT SEQUENCE, 17 unordered pieces.//1.6e-29:304:77//AC000406
R-HEMBA1003714
R-HEMBA1003715//Homo sapiens chromosome 16p11.2 BAC clone CIT987SK-A-685D8, WORKING DRAFT SEQUENCE, 16 unordered pieces.//1.4e-63:578:77//AC005136
R-HEMBA1003720//Homo sapiens, WORKING DRAFT SEQUENCE, 135 unordered pieces.//2.4e-36:350:78//AC002353
R-HEMBA1003725//Homo sapiens chromosome 19, cosmid R31973, complete sequence.//6.3e-42:250:75//AC004699
R-HEMBA1003729//RPCI11-22D14.TV RPCI-11 Homo sapiens genomic clone RPCI-11-22D14, genomic survey sequence.//1.0:234:62//B86158
R-HEMBA1003733//Human DNA sequence from clone 396D17 on chromosome 1p33-35.3 Contains EST, STS, GSS, complete sequence.//7.7e-80:558:83//AL008634
R-HEMBA1003742//HS_3080_B2_H06_MR CIT Approved Human Genomic Sperm Library D Homo sapiens genomic clone Plate=3080 Col=12 Row=P, genomic survey sequence.//3.4e-55:331:91//AQ139179
R-HEMBA1003758//Human DNA sequence from PAC 295C6 on chromosome 1q24. Contains ESTs, CA repeat, STS and CpG istand.//4.5e-59:521:75//Z97876
R-HEMBA1003760
R-HEMBA1003773//Mus musculus signal recognition particle receptor beta subunit mRNA, complete cds.//2.6e-72:467:86//U17343
R-HEMBA1003783//Mus musculus bromodomain-containing protein BP75 mRNA, complete cds.//1.0e-77:557:81//AF084259
R-HEMBA1003784
R-HEMBA1003799//Homo sapiens PAC clone DJ1032B10 from 7p15.3-p21, complete sequence.//2.1 e-49:390:72//AC004455
R-HEMBA1003803
R-HEMBAl003804//Homo sapiens chromosome 17, clone hCIT.175_E_5, complete sequence.//9.4e-99:359:99//AC004596
R-HEMBA1003805//Human DNA sequence from clone 51J12 on chromosome 6q26-27. Contains the 3' part of the alternatively spliced gene for the human orthologs of mouse QKI-7 and QKI-7B (KH Domain RNA Binding proteins) and zebrafish ZKQ-1 (Quaking protein homolog). Contains ESTs, STSs and GSSs, complete sequence.//8.0e-113:567:96//AL031781
R-HEMBA1003807//Bovine dinucleotide microsatellite HUJII77.//5.4e-18:194:78//M96348
R-HEMBA1003836//Human DNA from overlapping chromosome 19 cosmids R31396, F2545L and R31076 containing COX6B and UPKA, genomic sequence, complete sequence.//3.4e-40:256:85//AC002115
R-HEMBA1003838//CIT-HSP-2380F18.TF CIT-HSP Homo sapiens genomic clone 2380F18, genomic survey sequence.//9.7e-25:150:96//AQ196624
R-HEMBA1003856//Human DNA sequence from clone 272E8 on chromosome Xp22.13-22.31. Contains a pseudogene similar to MDM2-Like P53-binding protein gene. Contains STSs, GSSs and a CA repeat polymorphism, complete sequence.//4.8e-33:486:68//Z93929
R-HEMBA1003864//, complete sequence.//4.4e-100:531:94//AC005300
R-HEMBA1003866//HS_3203_B2_C01_MR CIT Approved Human Genomic Sperm Library D Homo sapiens genomic clone Plate=3203 Col=2 Row=F, genomic survey sequence.//2.6e-05:206:64//AQ180298
R-HEMBA1003879//Homo sapiens chromosome 10 clone CIT987SK-1119P3 map 10q25.1, WORKING DRAFT SEQUENCE, 1 ordered pieces.//4.7e-17:170:79//U82207
R-HEMBA1003880//Homo sapiens genomic DNA, chromosome 21q11.1, segment 7/28, WORKING DRAFT SEQUENCE.//7.8e-103:526:96//AP000036
R-HEMBA1003885//Human apolipoprotein apoC-IV (APOC4) gene, complete cds.//3.5e-45:299:87//U32576
R-HEMBA1003893//Human DNA sequence *** SEQUENCING IN PROGRESS *** from clone 1137F22, WORKING DRAFT SEQUENCE.//1.1e-41:386:77//AL034421
R-HEMBA1003902//HS_3031_B2_E07_MR CIT Approved Human Genomic Sperm Library D Homo sapiens genomic clone Plate=3031 Col=14 Row=J, genomic survey sequence.//5.3e-50:293:93//AQ165549
R-HEMBA1003908//CIT-HSP-2367K7.TR CIT-HSP Homo sapiens genomic clone 2367K7, genomic survey sequence.//1.2e-32:220:92//AQ076795
R-HEMBA1003926//Homo sapiens chromosome 5, BAC clone 194j18 (LBNL H158), complete sequence.//3.1e-58:294:85//AC005368
R-HEMBA1003937//Homo sapiens chromosome 3 subtelomeric region.//8.0e-111:590:93//AF109718
R-HEMBA1003939
R-HEMBA1003942//Homo sapiens clone DJ0828F13, complete sequence.//2.2e-08:474:58//AC004904
R-HEMBA1003950//Plasmodium vivax from Brazil cytochrome b (cytb) gene, mitochondrial gene encoding mitochondrial protein, partial cds.//0.034:258:62//AF069619
R-HEMBA1003953//Plasmodium falciparum MAL3P8, complete sequence.//0.096:492:57//AL034560
R-HEMBA1003958//Human DNA sequence *** SEQUENCING IN PROGRESS *** from clone 64K7, WORKING DRAFT SEQUENCE.//7.3e-40:382:78//AL031668
R-HEMBA1003959//Amaranthus hypochondriacus betaine aldehyde dehydrogenase (ahybadh4) gene, complete cds.//0.11:428:60//AF000132
R-HEMBA1003976//Homo sapiens PAC clone DJ0724E13 from 7p11.2-p12, complete sequence.//1.0:222:62//AC004414
R-HEMBA1003978//Sequence 31 from patent US 5708157.//1.9e-14:159:77//I80060
R-HEMBA1003985//Homo sapiens 12p13.3 PAC RPCI5-927J10 (Roswell Park Cancer Institute Human PAC library) complete sequence.//5.6e-14:136:83//AC004804
R-HEMBA1003987//Human chromosome 12p13 sequence, complete sequence.//3.2e-26:268:79//U47924
R-HEMBA1003989//RPCI11-52K22.TJ RPCI11 Homo sapiens genomic clone R-52K22, genomic survey sequence.//2.2e-86:443:95//AQ052484
R-HEMBA1004000
R-HEMBA1004011
R-HEMBA1004012//Homo sapiens chromosome 17, clone hRPK.63_A_1, complete sequence.//4.7e-38:284:85//AC005670
R-HEMBA1004015//Human DNA sequence from clone 931E15 on chromosome Xq25. Contains STSs, GSSs and genomic marker DXS8098, complete sequence.//0.48:460:58//AL023575
R-HEMBA1004024//Homo sapiens clone RG270D13, WORKING DRAFT SEQUENCE, 18 unordered pieces.//2.5e-21:159:80//AC005081
R-HEMBA1004038//Homo sapiens Xq28 BAC RPCI11-382P7 (Roswell Park Cancer Institute Human BAC Library) complete sequence.//7.9e-10:231:66//AC006054
R-HEMBA1004042//Homo sapiens clone DJ0968I16, complete sequence.//0.00071:263:68//AC006016
R-HEMBA1004045//Homo sapiens PAC clone DJ0074M20 from X, complete sequence.//8.8e-23:196:69//AC006143
R-HEMBA1004048//CIT-HSP-2288N20.TF CIT-HSP Homo sapiens genomic clone 2288N20, genomic survey sequence.//0.013:162:67//AQ007283
R-HEMBA1004049//Human hsp 70 gene 3' region for 70 kDa heat shock protein.//7.7e-30:176:96//X04677
R-HEMBA1004055//Plasmodium falciparum 3D7 chromosome 12 PFYAC1383 genomic sequence, WORKING DRAFT SEQUENCE, 3 unordered pieces.//8.4e-05:395:63//AC005504
R-HEMBA1004056//Homo sapiens clone DJ0847008, WORKING DRAFT SEQUENCE, 3 unordered pieces.//3.5e-61:551:77//AC005484
R-HEMBA1004074//Homo sapiens clone DJ1032D07, WORKING DRAFT SEQUENCE, 3 unordered pieces.//0.98:275:63//AC004952
R-HEMBA1004086//Sequence 65 from patent US 5691147.//2.8e-54:313:92//I76237
R-HEMBA1004097//Mus musculus putative transcription factor mRNA, complete cds.//1.8e-11:323:63//AF091234
R-HEMBA1004131//Human mRNA for KIAA0128 gene, partial cds.//9.3e-42:534:69//D50918
R-HEMBA1004132//Homo sapiens chromosome 17, clone hCIT.211_P_7, complete sequence.//6.0e-49:491:76//AC003665
R-HEMBA1004133//HS_3229_B2_E09_T7 CIT Approved Human Genomic Sperm Library D Homo sapiens genomic clone Plate=3229 Col=18 Row=J, genomic survey sequence.//1.1e-72:374:97//AQ192003
R-HEMBA1004138//Human DNA sequence *** SEQUENCING IN PROGRESS *** from clone 417M14, WORKING DRAFT SEQUENCE.//3.1e-09:277:66//AL024498
R-HEMBA1004143//Plasmodium falciparum MAL3P4, complete sequence.//0.53:239:61//AL008970
R-HEMBA1004146//Homo sapiens clone DJ0038I10, WORKING DRAFT SEQUENCE, 5 unordered pieces.//3.0e-35:165:88//AC004820
R-HEMBA1004150//CITBI-E1-2517I2.TR CITBI-E1 Homo sapiens genomic clone 2517I2, genomic survey sequence.//0.56:379:59//AQ277616
R-HEMBA1004164//Human BAC clone GS200K05 from 7q21-q22, complete sequence.//4.6e-49:448:77//AC002429
R-HEMBA1004168//Homo sapiens geminin mRNA, complete cds.//2.4e-110:563:96//AF067855
R-HEMBA1004199//S.pombe chromosome I cosmid c8A4.//0.73:187:64//Z66569
R-HEMBA1004200//Homo sapiens Xp22 BAC GSHB-184P14 (Genome Systems Human BAC library) complete sequence.//6.3e-30:293:77//AC004552
R-HEMBA1004202//rah=ras-related homolog [mice, HT4 neural cell line, mRNA, 993 nt].//3.0e-64:517:80//S72304
R-HEMBA1004203//Homo sapiens clone NH0313P13, WORKING DRAFT SEQUENCE, 15 unordered pieces.//1.0e-97:303:98//AC005488
R-HEMBA1004207//Homo sapiens leptin receptor short form (db) mRNA, complete cds.//3.6e-116:573:97//U50748
R-HEMBA1004225//Drosophila melanogaster mitochondrial DNA with 12 tRNAs and 7 genes.//5.4e-11:493:60//M37275
R-HEMBA1004227//Rattus norvegicus protein phosphatase 2C mRNA, complete cds.//6.1e-76:443:86//AF095927
R-HEMBA1004238//Homo sapiens chromosome 19, cosmid R28341, complete sequence.//1.1e-42:330:83//AC005763
R-HEMBA1004241
R-HEMBA1004246//Homo sapiens genomic DNA, chromosome 21q22.2 (Down Syndrome region), segment 4/15, WORKING DRAFT SEQUENCE.//1.1e-45:288:85//AP000011
R-HEMBA1004248//Homo sapiens PAC clone DJ0828B12 from 7q11.23-q21.1, complete sequence.//5.2e-09:516:61//AC004903
R-HEMBA1004264
R-HEMBA1004267//HS_2255_A2_H12_MR CIT Approved Human Genomic Sperm Library D Homo sapiens genomic clone Plate=2255 Col=24 Row=O, genomic survey sequence.//8.6e-59:318:95//AQ068854
R-HEMBA1004272//Homo sapiens 12p13.3 PAC RPCIS-1180D12 (Roswell Park Cancer Institute Human PAC Library) complete sequence.//1.1e-113:576:96//AC005831
R-nnnnnnnnnnnn//Homo sapiens clone 617 unknown mRNA, complete sequence.//4.4e-110:553:96//AF091081
R-HEMBA1004276
R-HEMBA1004286//Homo sapiens TGF beta receptor associated protein-1 mRNA, complete cds.//1.9e-106:538:97//AF022795
R-HEMBA1004289//RPCI11-74010.TJ RPCI11 Homo sapiens genomic clone R-74O10, genomic survey sequence.//2.3e-37:248:76//AQ266668
R-HEMBA1004295//Baboon apolipoprotein A-VI mRNA, 3' end.//0.0016:273:64//L13174
R-HEMBA1004306//HS_3175_B2_F01_T7 CIT Approved Human Genomic Sperm Library D Homo sapiens genomic clone Plate=3175 Col=2 Row=L, genomic survey sequence.//1.6e-28:190:77//AQ169206
R-HEMBA1004312//Human BAC clone RG119P24 from 7q31, complete sequence.//6.3e-36:267:82//AC003088
R-HEMBA1004321//Homo sapiens *** SEQUENCING IN PROGRESS *** from PAC 10155, WORKING DRAFT SEQUENCE.//4.1e-111:576:95//AJ009611
R-HEMBA1004323//CIT-HSP-2374C8.TR CIT-HSP Homo sapiens genomic clone 2374C8, genomic survey sequence.//2.7e-42:136:91//AQ114933
R-HEMBA1004327//CIT-HSP-2303L24.TF CIT-HSP Homo sapiens genomic clone 2303L24, genomic survey sequence.//1.0:78:67//AQ017600
R-HEMBA1004330//Homo sapiens clone DJ1173120, WORKING DRAFT SEQUENCE, 5 unordered pieces.//2.3e-119:580:98//AC004987
R-HEMBA1004334//Pimpinella brachycarpa Phybl mRNA, complete cds.//3.3e-14:238:69//AF082024
R-HEMBA1004335//Homo sapiens Chromosome 16 BAC clone CIT987SK-A-116A10, complete sequence.//1.8e-21:291:71//AC004638
R-HEMBA1004341
R-HEMBA1004353//Homo sapiens mRNA for c-myc binding protein, complete cds.//4.1e-74:444:90//D89667
R-HEMBA1004354//Human DNA from overlapping chromosome 19-specific cosmids R29515 and R28253, genomic sequence, complete sequence.//7.0e-38:287:82//AC003002
R-HEMBA1004356//Sequence 2 from patent US 5652144.//3.7e-108:588:92//I58611
R-HEMBA1004366//WORKING DRAFT SEQUENCE, 3 unordered pieces.//1.8e-14:446:63//AC005949
R-HEMBA1004372//CIT-HSP-2005C13.TF CIT-HSP Homo sapiens genomic clone 2005C13, genomic survey sequence.//0.010:334:61//B55811
R-HEMBA1004389//Homo sapiens full-length insert cDNA clone ZE09A11.//1.5e-19:170:83//AF086540
R-HEMBA1004394//Human (D21S198) DNA segment containing (TG)23 repeat.//1.0:50:84//X58124
R-HEMBA1004396//Homo sapiens chromosome 4 clone B240N9 map 4q25, complete sequence.//8.2e-34:459:69//AC004057
R-HEMBA1004405//Homo sapiens BAC clone GS589P19 from 7p13-p14, complete sequence.//2.8e-42:314:84//AC005030
R-HEMBA1004408
R-HEMBA1004429//M.musculus of DNA encoding DNA-binding protein.//1.6e-66:449:82//Z54200
R-HEMBA1004433//Homo sapiens chromosome 21q22.3, PAC clones 314N7, 225L15, BAC clone 7B7, complete sequence bases 1..333303.//7.2e-32:460:68//AJ011930
R-HEMBA1004460//Homo sapiens clone DJ0647C14, WORKING DRAFT SEQUENCE, 21 unordered pieces.//3.9e-113:581:96//AC004846
R-HEMBA1004461//HS_3244_A2_F12_MR CIT Approved Human Genomic Sperm Library D Homo sapiens genomic clone Plate=3244 Col=24 Row=K, genomic survey sequence.//8.0e-83:397:99//AQ220876
R-HEMBA1004479//Homo sapiens PAC clone DJ0942I16 from 7q11, complete sequence.//1.7e-40:485:70//AC006012
R-HEMBA1004482//Plasmodium falciparum chromosome 2, section 7 of 73 of the complete sequence.//2.2e-11:513:59//AE001370
R-HEMBA1004502//Homo sapiens chromosome 17, clone hRPK.372_K_20, complete sequence.//2.0e-08:245 :66//AC005951
R-HEMBA1004506//Human DNA sequence *** SEQUENCING IN PROGRESS *** from clone 34606, WORKING DRAFT SEQUENCE.//4.2e-81:582:83//Z84487
R-HEMBA1004507//Caenorhabditis elegans cosmid C40C9, complete sequence.//0.56:235:64//Z70266
R-HEMBA1004509
R-HEMBA1004534//Sequence 58 from patent US 5691147.//1.9e-61:430:83//I76230
R-HEMBA1004538//HS_3189_B2_C03_T7 CIT Approved Human Genomic Sperm Library D Homo sapiens genomic clone Plate=3189 Col=6 Row=F, genomic survey sequence.//6.1e-21:140:92//AQ170330
R-HEMBA1004554//CIT-HSP-712K9.TP CIT-HSP Homo sapiens genomic clone 712K9, genomic survey sequence.//1.7e-16:116:93//B73329
R-HEMBA1004560//Human mRNA for KIAA0281 gene, complete cds.//2.2e-14:213:71//D87457
R-HEMBA1004573
R-HEMBA1004577//Human DNA sequence from cosmid L247F6, Huntington's Disease Region, chromosome 4p16.3 contains protein similar to Mouse SH3 binding protein 3BP2, multiple ESTs and a CpG island.//1.0:352:60//Z68279
R-HEMBA1004586
R-nnnnnnnnnnnn//Plasmodium falciparum MAL3P6, complete sequence.//0.0012:359:60//Z98551
R-HEMBA1004610//S.pombe chromosome II cosmid c354.//0.0011:362:62//AL022071
R-HEMBA1004617//Homo sapiens mRNA, chromosome 1 specific transcript KIAA0501./1.4e-50:327:85//AB007970
R-HEMBA1004629//Homo sapiens Xp22 bins 16-17 BAC GSHB-531I17 (Genome Systems Human BAC Library) complete sequence.//4.4e-13:527:63//AC004805
R-HEMBA1004631//Rattus norvegicus Nclone10 mRNA.//2.9e-24:364:71//U31866
R-HEMBA1004632
R-HEMBA1004637//Homo sapiens clone DJ0982E09, WORKING DRAFT SEQUENCE, 3 unordered pieces.//7.7e-117:573:98//AC005534
R-HEMBA1004638//H.sapiens mRNA for DGCR2.//3.8e-19:118:99//X84076
R-HEMBA1004666//Arabidopsis thaliana chromosome II BAC T4E14 genomic sequence, complete sequence.//0.00013:501:58//AC005171
R-HEMBA1004669//Human DNA sequence from clone 465N24 on chromosome 1p35.1-36.13. Contains two novel genes, ESTs, GSSs and CpG islands, complete sequence.//1.5e-120:571:98//AL031432
R-HEMBA1004670//Human DNA sequence *** SEQUENCING IN PROGRESS *** from clone 222E13, WORKING DRAFT SEQUENCE.//4.4e-12:110:88//Z93241
R-HEMBA1004672//Human DNA sequence from PAC 308I13 on chromosome 1p35-1p36.3.//3.4e-38:324:81//Z99291
R-HEMBA1004693//Arabidopsis thaliana genomic DNA, chromosome 5, P1 clone: MPO12, complete sequence.//0.86:309:57//AB006702
R-HEMBA1004697//T33B22TF TAMU Arabidopsis thaliana genomic clone T33B22, genomic survey sequence.//0.29:331:61//B97342
R-HEMBA1004705//Plasmodium falciparum MAL3P7, complete sequence.//0.051:424:58//AL034559
R-HEMBA1004709//Homo sapiens Chromosome 16 BAC clone CIT987SK-A-116A10, complete sequence.//1.7e-49:497:76//AC004638
R-HEMBA1004711//Homo sapiens chromosome 17, clone hRPK.271_K_11, complete sequence.//1.6e-38:362:79//AC005562
R-HEMBA1004725
R-HEMBA1004730//Homo sapiens Chromosome 17p13 Cosmid Clone cos26, complete sequence.//1.1e-58:489:79//AC002085
R-HEMBA1004733
R-HEMBA1004734//Human DNA sequence from clone 273N12 on chromosome 6q16.1-16.3. Contains the gene for the N-Oct5a (N-Oct3, N-Oct5b) POU domain proteins and an unknown gene. Contains a putative CpG island, ESTs, STS; and GSSs, complete sequence.//0.0030:362:61//AL022395
R-HEMBA1004736//Homo sapiens clone DJ0981O07, complete sequence.//1.9e-58:282:87//AC006017
R-HEMBA1004748//Homo sapiens PAC clone DJ1059M17 from 7q21-q31.1, complete sequence.//3.6e-34:287:81//AC004953
R-HEMBA1004751//Human DNA sequence from PAC 507I15 on chromosome Xq26.3-27.3. Contains 60S ribosomal protein L44 (L41, L36) like gene, ESTs, STSs and a polymorphic CA repeat.//5.3e-40:266:89//Z98950
R-HEMBA1004752//Human DNA sequence *** SEQUENCING IN PROGRESS *** from clone 495010, WORKING DRAFT SEQUENCE.//3.3e-39:281:85//AL031121
R-HEMBA1004753//Homo sapiens ribosomal protein S20 (RPS20) mRNA, complete cds.//2.6e-65:475:84//L06498
R-HEMBA1004756//Homo sapiens DNA sequence from PAC 86C11 on chromosome 6p21.31-22.1. Contains histone genes H2A/1,H2B.1A,H4,H2A.1b,H3 pseudogene, pheromone receptor pseudogene, ESTs, STS and CpG island.//1.8e-08:516:59//AL021807
R-HEMBA1004758//Homo sapiens chromosome 4 clone B240N9 map 4q25, complete sequence.//5.1e-45:577:72//AC004057
R-HEMBA1004763
R-HEMBA1004768//Human DNA sequence from clone 395P12 on chromosome 1q24-25. Contains the TXGP1 gene for tax-transcriptionally activated glycoprotein 1 (34kD) (OX40 ligand, OX40L) and a GOT2 (Aspartate Aminotransferase, mitochondrial precursor, EC 2.6.1.1, Transaminase A, Glutamate Oxaloacetate Transaminase-2) pseudogene. Contains ESTs, STSs and GSSs, complete sequence.//4.1e-60:435:78//AL022310
R-HEMBA1004770//Plasmodium falciparum chromosome 2, section 8 of 73 of the complete sequence.//8.7e-05:476:61//AE001371
R-HEMBA1004771//Homo sapiens Xp22 Cosmid U152D7 (Lawrence Livermore human cosmid library) complete sequence.//5.0e-08:113:80//AC003047
R-HEMBA1004776
R-HEMBA1004778//***ALU WARNING: Human Alu-J subfamily consensus sequence.//1.1e-35:288:84//U14567
R-nnnnnnnnnnnn/HS_3192_B1_F09_T7 CIT Approved Human Genomic Sperm Library D Homo sapiens genomic clone Plate=3192 Col=17 Row=L, genomic survey sequence.//1.9e-44:233:98//AQ155855
R-HEMBA1004803//Homo sapiens minisatellite ms31 repeat region.//3.0e-67:318:87//AF048728
R-HEMBA1004806
R-HEMBA1004807//Homo sapiens clone GS166C05, WORKING DRAFT SEQUENCE, 7 unordered pieces.//3.6e-20:333:69//AC005015
R-HEMBA1004816//Human DNA sequence from PAC 50A13 on chromosome Xp11. Contains ATP SYNTHASE LIPID BINDING PROTEIN P1 (P2, P3) precursor (ATP5G1, ATP5G2, ATP5G3) like pseudogene, ESTs and STSs. Contains polymorphic CA repeat.//6.3e-13:148:77//Z92545
R-HEMBA1004820//Human arginine-rich nuclear protein mRNA, complete cds.//1.5e-12:141:85//M74002
R-HEMBA1004847//Canine mRNA for 68kDA subunit of signal recognition particle (SRP68).//7.6e-80:297:85//X53744
R-HEMBA1004850
R-HEMBA1004863//Human DNA sequence from PAC 345P10 on chromosome 22q12-qter contains ESTs and STS and polymorphic CA repeat D22S927.//2.0e-14:159:79//Z82201
R-HEMBA1004864
R-HEMBA1004865//Homo sapiens Xp22-149 BAC RPCI11-466O4 (Roswell Park Cancer Institute Human BAC Library) complete sequence.//0.90:76:76//AC005297
R-HEMBA1004880//Homo sapiens clone DJ0309D19, WORKING DRAFT SEQUENCE, 12 unordered pieces.//1.9e-49:551:73//AC004826
R-HEMBA1004889//Human DNA sequence *** SEQUENCING IN PROGRESS *** from clone 223B1, WORKING DRAFT SEQUENCE.//0.0021:189:65//AL031943
R-HEMBA1004900//Homo sapiens chromosome 17, clone hRPK.180_P_8, complete sequence.//6.6e-11:144:7711AC005972
R-HEMBA1004909//Human DNA sequence from clone 505B13 on chromosome 1p36.2-36.3 Contains CA repeat and GSSs, complete sequence.//7.6e-46:341:83//Z98052
R-HEMBA1004918//Human DNA sequence *** SEQUENCING IN PROGRESS *** from clone 994L9, WORKING DRAFT SEQUENCE.//1.6e-54:301:89//AL034554
R-HEMBA1004923//Homo sapiens 47kB DNA fragment from Xq28, proximal to MTM1 gene.//2.0e-07:182:69//Y15994
R-HEMBA1004929
R-HEMBA1004930//Homo sapient chromosome 11 clone CIT987SK-1012F4, WORKING DRAFT SEQUENCE, 6 unordered pieces.//7.7e-66:547:79//AC005848
R-HEMBA1004933//H.sapiens Humig mRNA.//0.13:233:62//X72755
R-HEMBA1004934//CIT-HSP-2021I16.TF CIT-HSP Homo sapiens genomic-clone 2021I16, genomic survey sequence.//0.66:268:62//B65345
R-HEMBA1004944//CIT-HSP-2281L12.TR CIT-HSP Homo sapiens genomic clone 2281L12, genomic survey sequence.//3.8e-20:104:82//B99849
R-HEMBA1004954//Homo sapiens chromosome 17, clone hRPK.146_P_2, WORKING DRAFT SEQUENCE, 4 unordered pieces.//0.00082:385:60//AC005341
R-HEMBA1004956//CIT-HSP-2305H22.TF CIT-HSP Homo sapiens genomic clone 2305H22, genomic survey sequence.//1.6e-84:411:99//AQ020408
R-HEMBA1004960//Human DNA sequence from PAC 358H7 on chromosome X.//3.3e-22:249:74//Z77249
R-HEMBA1004972//nbxb0003aF01f CUGI Rice BAC Library Oryza sativa genomic clone nbxb0003K01f, genomic survey sequence.//0.52:171:64//AQ049982
R-HEMBA1004973//*** SEQUENCING IN PROGRESS *** EPM1/APECED region of chromosome 21, clones A68E8, B127P21, B173L3, B23N8, C1242C9, C579E2, A70B6, B159G9, B175D10, B52C10, C124G1 Note: Sequencing in this region has been discontinued by the Stanford Human Genome Center, WORKING DRAFT SEQUENCE, 50 unordered pieces.//0.69:179:64//AC003656
R-HEMBA1004977//Caenorhabditis elegans cosmid F08G2, complete sequence.//7.6e-07:492:58//Z81495
R-HEMBA1004978//Human DNA sequence from clone 522P13 on chromosome 6p21.31-22.3. Contains a 60S Ribosomal Protein L21 pseudogene and an HNRNP A3 (Heterogenous Nuclear Riboprotein A3, FBRNP) pseudogene. Contains ESTs, STSs and GSSs, complete sequence.//0.20:427:60//AL024509
R-HEMBA1004980//CIT-HSP-2379K5.TF CIT-HSP Homo sapiens genomic clone 2379K5, genomic survey sequence.//1.6e-53:331:88//AQ108614
R-HEMBA1004983//Genomic sequence from Human 17, complete sequence.//0.00061:473:58//AC000389
R-HEMBA1004995//Homo sapiens chromosome 16, cosmid clone 306E5 (LANL), complete sequence.//1.6e-90:527:89//AC004224
R-HEMBA10050087/Human DNA sequence from clone 461P17 on chromosome 20q12-13.2. Contains four novel (pseudo)genes for proteins with Kunitz/Bovine pancreatic trypsin inhibitor and/or WAP-type (Whey Acidic Protein) 'four-disulfide core' domains, COX6C (Cytochrome C Oxidase Polypeptide VIC, EC 1.9.3.1) and RPL5 (60S Ribosomal Protein L5) pseudogenes, a pseudogene similar to part of the HSPD1 (HSP60, Mitochondrial Matrix Protein P1 precursor, Heat Shock Protein 60, GROEL protein, HUCHA60) gene, and the Major Epididymis-specific protein E4 precursor (HE4, Epididymis Secretory protein E4, WAP-type (Whey Acidic Protein) 'four-disulfide core' domain) gene. Contains ESTs, an STS, GSSs and a putative CpG island, complete sequence.//5.4e-65:357:83//AL031663
R-HEMBA1005009//Homo sapiens BAF53a (BAF53a) mRNA, complete cds.//5.6e-107:550:96//AF041474
R-HEMBA1005019//Homo sapiens mRNA for KIAA0648 protein, partial cds.//6.3e-104:542:94//AB014548
R-HEMBA1005029//Homo sapiens DNA sequence from PAC 97D16 on chromosome 6p21.3-22.2. Contains an unknown pseudogene, a 60S Ribosomal protein L24 (L30) LIKE pseudogene and histone genes H2BFC (H2B/c), H4FFP (H4/f pseudogene), H2AFC (H2A/c), H3F1K (H3.1/k) and a tRNA-Val pseudogene and tRNA-Thr gene. Contains ESTs, STSs, GSSs and genomic marker D6S464, complete sequence.//3.1e-67:493:83//AL009179
R-HEMBA1005035//Homo sapiens chromosome 17, clone hCIT.175_E_5, complete sequence.//7.4e-101:537:94//AC004596
R-HEMBA1005039//Human DNA sequence *** SEQUENCING IN PROGRESS *** from clone 1018D12, WORKING DRAFT SEQUENCE.//9.5e-30:446:68//AL031650
R-HEMBA1005047//Mus musculus mRNA for Rab24 protein.//1.4e-34:229:88//Z22819
R-HEMBA1005050//Human Chromosome X PAC RPCI1-290C9 from the Pieter de Jong Human PAC library; complete sequence.//4.0e-43:371:80//AC002404
R-HEMBA1005062//Homo sapiens chromosome 17, clone hCIT.186_H_2, complete sequence.//2.3e-15:269:66//AC004675
R-HEMBA1005066//Homo sapiens clone NH0001P09, WORKING DRAFT SEQUENCE, 1 unordered pieces.//4.0e-30:305:74//AC006030
R-HEMBA1005075
R-HEMBA1005079//Homo sapiens clone HS 19.11 Alu-Ya5 sequence.//6.5e-48:245:91//AF015156
R-HEMBA1005083//Human DNA sequence *** SEQUENCING IN PROGRESS *** from clone 1185N5, WORKING DRAFT SEQUENCE.//1.3e-15:142:83//AL034423
R-HEMBA1005101//Homo sapiens SYT interacting protein SIP mRNA, complete cds.//5.3e-110:545:96//AF080561
R-HEMBA1005113//Caenorhabditis elegans DNA *** SEQUENCING IN PROGRESS ∗∗∗ from clone Y53C10, WORKING DRAFT SEQUENCE.//0.026:252:64//Z93340
R-HEMBA1005123//Homo sapiens DNA sequence from clone 78F24 on chromosome 22q12.1-12.3. Contains one exon of an Oxysterol-binding protein (OSBP) LIKE gene. Contains GSSs and an STS, complete sequence.//7.1e-55:306:82//AL022336
R-HEMBA1005133//Human DNA sequence *** SEQUENCING IN PROGRESS *** from clone Y738F9, WORKING DRAFT SEQUENCE.//6.4e-45:309:87//AL022345
R-HEMBA1005149//Human cosmid LL12NC01-95H4, ETV6 gene, exon 2 and partial cds.//3.2e-31:310:76//U81834
R-HEMBA1005152//Homo sapiens DNA sequence from PAC 13D10 on chromosome 6p22.3-23. Contains CpG island.//1.4e-33:361:79//AL021407
R-HEMBA1005159//Human DNA sequence from clone 163016 on chromosome 1p35.1-36.13 Contains CA repeat, STS, complete sequence.//2.7e-22:440:66//AL031279
R-HEMBA1005185//Caenorhabditis elegans DNA *** SEQUENCING IN PROGRESS *** from clone Y105E8, WORKING DRAFT SEQUENCE.//0.0017:381:58//AL022594
R-HEMBA1005201//P.falciparum complete gene map of plastid-like DNA (IR-B).//8.5e-05:457:57//X95276
R-HEMBA1005202//Human 18S ribosomal RNA.//4.7e-38:236:91//X03205
R-HEMBA1005219
R-HEMBA1005223//Homo sapiens clone DJ0673M15, WORKING DRAFT SEQUENCE, 33 unordered pieces.//1.0:209:65//AC004854
R-HEMBA1005232//Homo-sapiens chromosome Y, clone 264,M,20, complete sequence.//0.0040:439:58//AC004617
R-HEMBA1005241//Homo sapiens PAC clone DJ0777023 from 7p14-p15, complete sequence.//4.2e-111:568:96//AC005154
R-HEMBA1005244//HS_3092_B2_C11_MF CIT Approved Human Genomic Sperm Library D Homo sapiens genomic clone Plate=3092 Col=22 Row=F, genomic survey sequence.//4.9e-12:116:84//AQ127947
R-HEMBA1005251//Homo sapiens PAC clone DJ1182N03 from 7q11.23-q21.1, complete sequence.//3.2e-27:210:84//AC004548
R-HEMBA1005252//Homo sapiens chromosome 17, clone hRPK.318_A_15, complete sequence.//4.6e-105:437:97//AC005837
R-HEMBA1005274//Slime mold mitochondrial DNA, binding region to the membrane system.//0.011:339:59//D86630
R-HEMBA1005275//Homo sapiens PAC clone DJ0886O08 from 7q32-q35, complete sequence.//3.4e-17:269:71//AC004914
R-HEMBA1005293//Human DNA sequence from PAC 130N4, BRCA2 gene region chromosome 13q12-13 contains xs7 mRNA, ESTs.//6.9e-20:193:73//Z75887
R-HEMBA1005296//HS_3037_B1_D01_MR CIT Approved Human Genomic Sperm Library D Homo sapiens genomic clone Plate=3037 Col=1 Row=H, genomic survey sequence.//0.26:184:64//AQ117120
R-HEMBA1005304//Homo sapiens clone DJ0693M11, WORKING DRAFT SEQUENCE, 7 unordered pieces.//1.5e-58:445:78//AC006146
R-HEMBA1005311//Human DNA sequence *** SEQUENCING IN PROGRESS *** from clone 796E4, WORKING DRAFT SEQUENCE.//9.3e-42:383:78//AL022337
R-HEMBA1005314//Caenorhabditis elegans cosmid F23H11.//0.80:179:65//AF003389
R-HEMBA1005315//Homo sapiens clone NH0001P09, WORKING DRAFT SEQUENCE, 1 unordered pieces.//2.4e-40:409:71//AC006030
R-HEMBA1005318//S.pombe chromosome I cosmid c2E11.//0.97:370:61//AL031181
R-HEMBA1005331//Homo sapiens chromosome 17, clone hRPK.214_C_8, complete sequence.//1.9e-112:577:95//AC005803
R-HEMBA1005353//Human DNA sequence *** SEQUENCING IN PROGRESS *** from clone 429E7, WORKING DRAFT SEQUENCE.//8.9e-80:406:97//AL031722
R-HEMBA1005359//Homo sapiens chromosome 17, clone hRPK.22_N_12, WORKING DRAFT SEQUENCE, 2 ordered pieces.//3.2e-50:320:84//AC005412
R-HEMBA1005367//RPCI11-85E23.TV RPCI11 Homo sapiens genomic clone R-85E23, genomic survey sequence.//0.39:148:67//AQ281915
R-HEMBA1005372//Homo sapiens full-length insert cDNA YH93B03.//2.6e-108:557:95//AF074997
R-HEMBA1005374//Homo sapiens full-length insert cDNA clone ZA95D11.//1.9e-110:531:98//AF086142
R-HEMBA1005389//Human DNA sequence from clone 245G19 on chromosome Xp22.11-22.2 Contains serine-threonine kinase (Txp3) gene, a pseudogene similar to ALPHA-1 PROTEIN ((CONNEXIN 43, CX43, GAP JUNCTION 43 KD HEART PROTEIN)), and the 3' end of the RS (X-linked juvenile retinoschisis precursor protein) gene. Contains ESTs, STSs and GSSs, complete sequence.//6.0e-41:432:75//Z92542
R-HEMBA1005394//Human DNA sequence *** SEQUENCING IN PROGRESS *** from clone 681N20, WORKING DRAFT SEQUENCE.//4.9e-107:585:93//AL031670
R-HEMBA1005403//Human DNA sequence *** SEQUENCING IN PROGRESS *** from clone 423B22, WORKING DRAFT SEQUENCE.//5.1e-118:586:97//AL034379
R-HEMBA1005408//Bos taurus retina membrane guanylate cyclase ROS-GC2 mRNA, complete cds.//1.6e-06:204:68//U95958
R-HEMBA1005410//Human DNA sequence *** SEQUENCING IN PROGRESS *** from clone 732E4, WORKING DRAFT SEQUENCE.//1.2e-23:452:66//AL008722
R-HEMBA1005411//RPCI11-66N19.TK RPCI11 Homo sapiens genomic clone R-66N19, genomic survey sequence.//2.2e-38:222:79//AQ237442
R-HEMBA1005423//Homo sapiens cyclin-dependent kinase inhibitor (CDKN2C) mRNA, complete cds.//5.6e-117:453:99//AF041248
R-HEMBA1005426//Human DNA sequence from PAC 448E20 on chromosome Xq26.1 contains ESTs and STS.//0.86:278:60//Z97196
R-HEMBA1005443//Homo sapiens (clone s153) mRNA fragment.//5.4e-46:305:87//L40391
R-HEMBA1005447//Human DNA sequence from clone 48G12 on chromosome Xq27.1-27.3. Contains STSs and GSSs, complete sequence.//3.3e-79:531:86//AL031054
R-HEMBA1005468//Homo sapiens PAC clone DJ0808G16 from 7q11.23-q21, complete sequence.//4.0e-27:469:66//AC004894
R-HEMBA1005469//Homo sapiens chromosome 16, P1 clone 96-4B (LANL), complete sequence.//7.2e-40:410:76//AC005212
R-HEMBA1005472//Human DNA Sequence *** SEQUENCING IN PROGRESS *** from clone 1090E8, WORKING DRAFT SEQUENCE.//3.1e-40:296:85//AL033524
R-HEMBA1005475//HS_2266_B2_C04_MF CIT Approved Human Genomic Sperm Library D Homo sapiens genomic clone Plate=2266 Col=8 Row=F, genomic survey sequence.//0.49:209:61//AQ069377
R-HEMBA1005497
R-HEMBA1005500//Homo sapiens PAC clone DJ1093O17 from 7q11.23-q21, complete sequence.//4.5e-116:580:97//AC004957
R-HEMBA1005506//Arabidopsis thaliana BAC T26D22.//0.0050:442:59//AF058826
R-HEMBA1005508//Sigalphus sp. 16S ribosomal RNA gene, partial sequence.//0.020:391:59//AF003509
R-HEMBA1005511//Human DNA sequence from PAC 52D1 on chromosome Xq21. Contains CA repeats, STS.//0.44:195:63//Z96811
R-HEMBA1005517//Bovine herpesvirus type 1 early-intermediate transcription control protein (BICP4) gene, complete cds.//0.44:470:57//L14320
R-HEMBA1005518//M.musculus mRNA for paladin gene.//6.2e-29:183:81//X99384
R-HEMBA1005520//Homo sapiens clone DJ0876A24, WORKING DRAFT SEQUENCE, 6 unordered pieces.//7.2e-40:281:86//AC004913
R-HEMBA1005526//Human DNA sequence *** SEQUENCING IN PROGRESS *** from clone 341D10, WORKING DRAFT SEQUENCE.//3.9e-40:482:73//Z97985
R-HEMBA1005528//Homo sapiens genomic DNA of 8p21.3-p22 anti-oncogene of hepatocellular colorectal and non-small cell lung cancer , segment 3/11.//3.8e-84:309:99//AB020860
R-HEMBA1005530//Homo sapiens PAC clone 946B23 SCA2 region, SP6 end, genomic sequence, genomic survey sequence.//8.1e-25:154:94//U84091
R-HEMBA1005548//Human DNA sequence *** SEQUENCING IN PROGRESS *** from clone 970A17, WORKING DRAFT SEQUENCE.//5.3e-105:534:96//AL034431
R-HEMBA1005552//Homo sapiens PAC clone DJ0807C15 from 7q34-q36, complete sequence.//2.8e-69:432:88//AC004743
R-HEMBA1005558
R-HEMBA1005568//Homo sapiens Xp22 GSHB-314C4 (Genome Systems Human BAC library) complete sequence.//5.9e-33:367:74//AC004087
R-HEMBA1005570//Human DNA sequence from clone 192P9 on chromosome Xp11.23-11.4. Contains a pseudogene similar to rat Plasmolipin, ESTs and GSSs, complete sequence.//2.2e-67:399:91//AL020989
R-HEMBA1005576//Homo sapiens chromosome 16, BAC clone 97H22 (LANL), complete sequence.//1.0:156:631/AC005737
R-HEMBA1005577
R-HEMBA1005581//Homo sapiens mRNA for MEGFS, partial cds.//9.7e-27:561:64//AB011538
R-HEMBA1005582//Torulopsis glabrata mitochondrial intergenic region ATPase 9 -cytochrome oxidase 2 genes.//2.3e-10:404:62//X02171
R-HEMBA1005583//HS_3014_B1_D05_T7 CIT Approved Human Genomic Sperm Library D Homo sapiens genomic clone Plate=3014 Col=9 Row=H, genomic survey sequence.//3.0e-81:442:94//AQ154499
R-HEMBA1005588//Human DNA sequence from clone 1409 on chromosome Xp11.1-11.4. Contains a Inter-Alpha-Trypsin Inhibitor Heavy Chain LIKE gene, a alternatively spliced Melanoma-Associated Antigen MAGE LIKE gene and a 6-Phosphofructo-2-kinase (Fructose-2,6-bisphosphatase) LIKE pseudogene. Contains ESTs, STSs and genomic marker DXS8032, complete sequence.//1.8e-54:490:77//Z98046
R-HEMBA1005593//Homo sapiens chromosome 17, clone hRPK.332_H_18, complete sequence.//2.2e-28:262:79//AC005746
R-HEMBA1005595//HS_2224_A2_G03_MF CIT Approved Human Genomic Sperm Library D Homo sapiens genomic clone Plate=2224 Col=6 Row=M, genomic survey sequence.//3.6e-48:263:95//AQ033446
R-HEMBA1005606//Human PAC clone DJ0093I03 from Xq23, complete sequence.//2.5e-08:355:63//AC003983
R-HEMBA1005609//HS_2182_B1_H06_MF CIT Approved Human Genomic Sperm Library D Homo sapiens genomic clone Plate=2182 Col=11 Row=P, genomic survey sequence.//2.2e-82:400:99//AQ023130
R-HEMBA1005616//Human DNA sequence *** SEQUENCING IN PROGRESS *** from clone 124K22, WORKING DRAFT SEQUENCE.//0.80:308:60//AL031176
R-HEMBA1005621//Human DNA sequence *** SEQUENCING IN PROGRESS *** from clone 330012, WORKING DRAFT SEQUENCE.//7.4e-76:338:98//AL031731
R-HEMBA1005627//Homo sapiens full-length insert cDNA clone ZD53D02.//4.5e-72:398:93//AF086321
R-HEMBA1005631//Homo sapiens PAC clone DJ1086D14, complete sequence.//3.8e-17:548:60//AC004460
R-HEMBA1005632//Homo sapiens DNA sequence from PAC 168L15 on chromosome 6q26-27. Contains RSK3 gene, ribosomal protein S6 kinase, EST, GSS, STS. CpG island, complete sequence.//1.4e-13:172:75//AL022069
R-HEMBA1005634//RPCI11-13O15.TVB RPCI-11 Homo sapiens genomic clone RPCI-11-13015, genomic survey sequence.//1.0e-28:153:82//B73293
R-HEMBA1005666//Human DNA sequence from PAC 696H22 on chromosome Xq21.1-21.2. Contains a mouse E25 like gene, a Kinesin like pseudogene and ESTs.//4.5e-51:343:87//AL021786
R-HEMBA1005670//Human DNA sequence *** SEQUENCING IN PROGRESS *** from clone 11703, WORKING DRAFT SEQUENCE.//2.5e-33:288:78//AL020995
R-HEMBA1005679//Human esterase D mRNA, 3'end.//4.2e-49:322:88//M13450
R-HEMBA1005680//Homo sapiens Chr.14 PAC RPCI4-794B2 (Roswell Park Cancer Institute Human PAC Library) complete sequence.//3.0e-36:285:83//AC005924
R-HEMBA1005685//H.sapiens (MAR8) chromosome 19 DNA, 343bp.//0.022:65:86//Z35281
R-HEMBA1005699//Human putative EPH-related PTK receptor ligand LERK-8 (Eplg8) mRNA, complete cds.//5.4e-46:376:84//U66406
R-HEMBA1005705//RPCI11-13014.TP RPCI-11 Homo sapiens genomic clone RPCI-11-13O14, genomic survey sequence.//0.071:182:59//B76186
R-HEMBA1005717//Human DNA sequence from PAC 50A13 on chromosome Xp11. Contains ATP SYNTHASE LIPID BINDING PROTEIN P1 (P2, P3) precursor (ATP5G1, ATP5G2, ATPSG3) like pseudogene, ESTs and STSs. Contains polymorphic CA repeat.//1.0:189:66//Z92545
R-HEMBA1005732//Human Chromosome 11q12 pac pDJ363p2, WORKING DRAFT SEQUENCE, 22 unordered pieces.//2.1e-47:449:75//AC003023
R-HEMBA1005737
R-nnnnnnnnnnnn//H.sapiens DNA for repeat unit locus D18S51 (285 bp).//0.11:174:63//X91255
R-HEMBA1005755//Human DNA-sequence from clone 396D17 on chromosome 1p33-35.3 Contains EST, STS, GSS, complete sequence.//0.15:160:65//AL008634
R-HEMBA1005765//Human Xq28 cosmids U225B5 and U236A12, complete sequence.//5.2e-39:422:74//U71148
R-HEMBA1005780//Homo sapiens genomic DNA, chromosome 21q22.2 (Down Syndrome region), segment 3/15, WORKING DRAFT SEQUENCE.//0.037:261:61//AP000010
R-HEMBA1005813//Human DNA sequence *** SEQUENCING IN PROGRESS *** from clone Y313F4, WORKING DRAFT SEQUENCE.//1.7e-26:242:80//AL023808
R-HEMBA1005815//Bufo boreas MVZ 145227 c-mos gene, partial cds.//0.17:199:62//U52805
R-HEMBA1005822//Plasmodium falciparum MAL3P7, complete sequence.//0.26:437:56//AL034559
R-HEMBA1005829//Human Cosmid g1572c035, complete sequence.//3.8e-05:366:61//AC000124
R-HEMBA1005834//Human DNA sequence from clone 51J12 on chromosome 6q26-27. Contains the 3' part of the alternatively spliced gene for the human orthologs of mouse QKI-7 and QKI-7B (KH Domain RNA Binding proteins) and zebrafish ZKQ-1 (Quaking protein homolog). Contains ESTs, STSs and GSSs, complete sequence.//8.2e-107:551:96//AL031781
R-HEMBA1005852//F.rubripes GSS sequence, clone 163A22aA4, genomic survey sequence.//2.6e-17:225:72//AL018730
R-HEMBA1005853//Human Chromosome 15 pac pDJ24m8, complete sequence.//1.1e-27:314:75//AC000379
R-HEMBA1005884//Homo sapiens 12p13.3 BAC RPCI3-488H23 (Roswell Park Cancer Institute Human BAC Library) complete sequence.//2.6e-20:328:67//AC006207
R-HEMBA1005891//Homo sapiens PAC clone DJ0997N05 from 7q11.23-q21.1, complete sequence.//2.0e-102:543:95//AC004945
R-HEMBA1005894
R-HEMBA1005909
R-HEMBA1005911//CIT-HSP-2342E5.TR CIT-HSP Homo sapiens genomic clone 2342E5, genomic survey sequence.//0.0012:315:60//AQ058081
R-HEMBA1005921//P.chrysogenum mitochondrion genes for tRNA-Arg, tRNA-Asn, tRNA-Tyr, small subunit rRNA, and ATPase subunit 6.//0.0090:445:58//Z23072
R-HEMBA1005931//Human DNA sequence *** SEQUENCING IN PROGRESS *** from clone 54B20, WORKING DRAFT SEQUENCE.//1.7e-46:351:83//Z98304
R-HEMBA1005934//Homo sapiens chromosome 17, clone hRPK.261_A_13, complete sequence.//0.0052:179:71//AC005138
R-HEMBA1005962//Homo sapiens clone RG012D21, complete sequence.//1.1e-11:149:74//AC005045
R-HEMBA1005963//HS_3055_A1_E08_MR CIT Approved Human Genomic Sperm Library D Homo sapiens genomic clone Plate=3055 Col=15 Row=I, genomic survey sequence.//5.4e-79:403:97//AQ147357
R-HEMBA1005990//Homo sapiens I-1 receptor candidate protein mRNA, complete cds.//6.9e-112:580:95//AF082516
R-HEMBA1005991//Human DNA sequence from clone 58A9 on chromosome 1q24.1-24.3. Contains STSs, GSSs, genomic marker D1S210 and a ca repeat polymorphism, complete sequence.//2.6e-39:299:82//AL031285
R-HEMBA1005999//Homo sapiens clone DJ0691F11, WORKING DRAFT SEQUENCE, 11 unordered pieces.//1.1e-29:260:70//AC004859
R-HEMBA1006002//Rattus norvegicus s-nexilin mRNA, complete cds.//6.3e-15:174:78//AF056035
R-HEMBA1006005//Homo sapiens MLL (MLL) gene, exons 1-3, and partial cds.//2.6e-112:574:95//AF036405
R-nnnnnnnnnnnn//Homo sapiens mRNA for KIAA0725 protein, partial cds.//7.6e-27:444:67//AB018268
R-HEMBA1006035//Plasmodium falciparum 3D7 chromosome 12 PFYAC336 genomic sequence, WORKING DRAFT SEQUENCE, 5 unordered pieces.//0.025:373:60//AC005139
R-HEMBA1006036//Homo sapiens Chromosome 16 BAC clone CIT987SK-625P11, complete sequence.//0.0056:535:59//AC004125
R-HEMBA1006042//HS_2169_A1_B11_MF CIT Approved Human Genomic Sperm Library D Homo sapiens genomic clone Plate=2169 Col=21 Row=C, genomic survey sequence.//1.7e-73:390:95//AQ132995
R-nnnnnnnnnnnn
R-HEMBA1006081
R-HEMBA1006090//HS_2262_A2_A01_T7 CIT Approved Human Genomic Sperm Library D Homo sapiens genomic clone Plate=2262 Col=2 Row=A, genomic survey sequence.//2.1e-70:360:97//AQ216324
R-HEMBA1006091
R-HEMBA1006100//Homo sapiens DNA sequence from PAC 212G6 on chromosome Xp11.3-p11.4. Contains synapsin 1, brain protein 4.1, properdin, tyrosine kinase (ELK1) oncogene, ESTs, STS, GSS, complete sequence.//1.6e-36:354:77//AL009172
R-HEMBA1006108
R-HEMBA1006121
R-HEMBA1006124//Human DNA sequence from BAC 175E3 on chromosome 22q11.2-qter. Contains ESTs, STSs and polymorphic CA repeat.//1.3e-12:327:64//Z95113
R-HEMBA1006130//WORKING DRAFT SEQUENCE, 3 unordered pieces.//0.60:326:62//AC005948
R-nnnnnnnnnnnn//Homo sapiens chromosome 19, cosmid F16403, complete sequence.//4.3e-52:321:80//AC005777
R-HEMBA100614211, complete sequence.//1.0e-13:160:78//AC005500
R-HEMBA1006155//Plasmodium falciparum 3D7 chromosome 12 PFYAC69 genomic sequence, WORKING DRAFT SEQUENCE, 4 unordered pieces.//0.0013:389:60//AC004688
R-HEMBA1006158//Homo sapiens transcription factor forkhead-like 7 (FKHL7) gene, complete cds.//1.4e-119:574:98//AF048693
R-HEMBA1006173//Mus musculus protein tyrosine phosphatase STEP61 mRNA, complete cds.//4.1e-43:307:86//U28217
R-HEMBA1006182//Homo sapiens BAC clone RG139P11 from 7q11-q21, complete sequence.//1.7e-30:300:71//AC004491
R-HEMBA1006198//***ALU WARNING: Human Alu-J subfamily consensus sequence.//1.3e-36:284:85//U14567
R-HEMBA1006235//Homo sapiens clone 24422 mRNA sequence.//2.1e-110:545:97//AF070557
R-HEMBA1006248//Homo sapiens mRNA for KIAA0667 protein, partial cds.//0.46:365:58//AB014567
R-HEMBA1006252//Human Chromosome 16 BAC clone CIT987SK-A-972D3, complete sequence.//2.8e-41:438:71//U91323
R-HEMBA1006253//Homo sapiens 45kDa splicing factor mRNA, complete cds.//1.8e-28:179:91//AF083384
R-HEMBA1006259//RPCI11-44N14.TJ RPCI11 Homo sapiens genomic clone R-44N14, genomic survey sequence.//1.5e-48:348:85//AQ203161
R-HEMBA1006268
R-HEMBA1006272//Human DNA sequence from clone 1198H6 on chromosome 1p36.11-36.31. Contains two Melanoma Preferentially Expressed Antigen PRAME LIKE genes. Contains GSSs and ESTs, complete sequence.//2.8e-73:273:87//AL023753
R-nnnnnnnnnnn//H.sapiens PAP mRNA.//1.6e-54:585:71//X76770
R-HEMBA1006283//Sequence 7 from patent US 5776683.//9.7e-18:113:98//AR016240
R-HEMBA1006284//Homo sapiens chromosome 17, clone hRPC.1028_K_7, complete sequence.//0.97:447:59//AC004585
R-HEMBA1006291//Homo sapiens full-length insert cDNA clone ZB76B10.//2.9e-94:454:98//AF086161
R-HEMBA1006293//Sequence 8 from patent US 5721351.//8.1e-10:111:72//I89415
R-HEMBA1006309//Homo sapiens chromosome 17, clone hRPK.22_N_12, WORKING DRAFT SEQUENCE, 2 ordered pieces.//8.6e-37:288:84//AC005412
R-HEMBA1006310//Rattus norvegicus cytosolic sorting protein PACS-1a (PACS-1) mRNA, complete cds.//6.5e-29:132:81//AF076183
R-HEMBA1006328//Human DNA sequence *** SEQUENCING IN PROGRESS *** from clone 894K16, WORKING DRAFT SEQUENCE.//3.3e-50:340:75//AL034429
R-HEMBA1006334
R-HEMBA1006344//Rattus norvegicus nitzin mRNA, partial cds.//8.7e-22:259:72//AF087945
R-HEMBA1006347//Human prostasin gene, complete cds.//1.8e-78:170:100//U33446
R-HEMBA1006349//Rat brain calcium channel alpha-1 subunit mRNA, complete cds.//0.00051:120:73//M57682
R-HEMBA1006359//CITBI-E1-2516C16.TR CITBI-E1 Homo sapiens genomic clone 2516C16, genomic survey sequence.//4.7e-74:576:82//AQ277951
R-HEMBA1006364//G.gallus gene for transforming growth factor-beta2, exons 5-7.//2.5e-21:118:85//X59080
R-HEMBA1006377//Homo sapiens chromosome 19, cosmid F23149, complete sequence.//5.7e-68:367:85//AC005239
R-HEMBA1006380//Human BAC clone RG007J15 from 7q31, complete sequence.//6.1e-47:300:83//AC003989
R-HEMBA1006381//Homo sapiens chromosome 5, Bac clone 189 (LBNL H135), complete sequence.//1.5e-47:336:86//AC005914
R-HEMBA1006398//Homo sapiens chromosome 5, BAC clone 203o13 (LBNL H155), complete sequence.//1.5e-67:501:83//AC005609
R-HEMBA1006416//Homo sapiens chromosome 17, clone 347_H_5, complete sequence.//4.4e-37:319:76//AC002119
R-HEMBA1006419//Homo sapiens chromosome 17, clone HCIT542B22, complete sequence.//2.9e-50:502:75//AC004253
R-HEMBA1006421//Homo sapiens chromosome 14q24.3 clone BAC270M14 transforming growth factor-beta 3 (TGF-beta 3) gene, complete cds; and unknown genes.//4.1e-116:572:97//AF107885
R-HEMBA1006424//Human DNA sequence from clone 51J12 on chromosome 6q26-27. Contains the 3' part of the alternatively spliced gene for the human orthologs of mouse QKI-7 and QKI-7B (KH Domain RNA Binding proteins) and zebrafish ZKQ-1 (Quaking protein homolog). Contains ESTs, STSs and GSSs, complete sequence.//9.4e-117:578:97//AL031781
R-HEMBA1006426//Human DNA sequence *** SEQUENCING IN PROGRESS *** from clone 291J10, WORKING DRAFT SEQUENCE.//2.2e-08:353:63//Z93017
R-HEMBA1006438//HS_2008_A1_D04_T7 CIT Approved Human Genomic Sperm Library D Homo sapiens genomic clone Plate=2008 Col=7 Row=G, genomic survey sequence.//1.2e-29:194:91//AQ245162
R-HEMBA1006445//Homo sapiens clone RG219E16, WORKING DRAFT SEQUENCE, 3 unordered pieces.//0.011:330:60//AC005075
R-HEMBA1006446//Plasmodium falciparum chromosome 2, section 35 of 73 of the complete sequence.//0.032:256:61//AE001398
R-HEMBA1006461//Homo sapiens Xp22 Cosmids U15E4, U115H5, U132E12, U115B9 (Lawrence Livermore human cosmid library) complete sequence.//5.6e-35:229:77//AC002364
R-HEMBA1006467//Homo sapiens Chromosome 9p22 Cosmid clone 34a5, complete sequence./11.1e-14:354:63//AC002052
R-HEMBA1006471
R-HEMBA1006474//p40, p24 [Borna disease virus BDV, WT-1, Halle B1/91, horse brain, field isolate, Genomic RNA, 1138 nt, segment 1 of 3].//1.1e-14:442:60//S67502
R-HEMBA1006483//Human chromosome 16p13.1 BAC clone CIT987SK-551G9 complete sequence.//3.7e-37:290:82//U95742
R-HEMBA1006485//H.sapiens mRNA for aminopeptidase.//7.6e-91:517:91//Y07701
R-HEMBA1006486//Homo sapiens clone RG315H11, WORKING DRAFT SEQUENCE, 5 unordered pieces.//1.1e-33:289:81//AC005089
R-HEMBA1006489//Human DNA sequence from clone 192P9 on chromosome Xp11.23-11.4. Contains a pseudogene similar to rat Plasmolipin, ESTs and GSSs, complete sequence.//6.0e-07:485:60//AL020989
R-HEMBA1006492//Homo sapiens chromosome 17, clone hRPK.269_G_24, complete sequence.//4.3e-112:572:95//AC005828
R-HEMBA1006494//Homo sapiens chromosome 17, clone HRPC987K16, complete sequence.//2.3e-10:186:67//AC002994
R-HEMBA1006497//RPCI11-16L10.TPB RPCI-11 Homo sapiens genomic clone RPCI-11-16L10, genomic survey sequence.//1.5e-10:75:100//B88015
R-HEMBA1006502//Human DNA sequence from clone 272E8 on chromosome Xp22.13-22.31. Contains a pseudogene similar to MDM2-Like P53-binding protein gene. Contains STSs, GSSs and a CA repeat polymorphism, complete sequence.//3.3e-36:516:70//Z93929
R-HEMBA1006507//Homo sapiens mRNA for KIAA0666 protein, partial cds.//1.2e-115:570:96//AB014566
R-HEMBA1006521//Human DNA sequence *** SEQUENCING IN PROGRESS *** from clone 54B20, WORKING DRAFT SEQUENCE.//2.2e-20:266:71//Z98304
R-HEMBA1006530//RPCI11-52M1.TJ RPCI11 Homo sapiens genomic clone R-52M1, genomic survey sequence.//0.00015:227:64//AQ052526
R-HEMBA1006535//HS_2234_B1_B07_MF CIT Approved Human Genomic Sperm Library D Homo sapiens genomic clone Plate=2234 Col=13 Row=D, genomic survey sequence.//7.5e-33:191:95//AQ129525
R-HEMBA1006540//Homo sapiens clone GS051M12, complete sequence.//0.026:497:58//AC005007
R-HEMBA1006546//Homo sapiens chromosome 19, cosmid R33496, complete sequence.//5.2e-41:289:86//AC004603
R-HEMBA1006559//Mus musculus PRAJA1 (Praja1) mRNA, complete cds.//3.4e-64:551:78//U06944
R-HEMBA1006562//Human Chromosome 11p11.2 PAC clone pDJ404m15, complete sequence.//5.7e-09:266:66//AC002554
R-HEMBA1006566//HS_2171_B1_B04_MF CIT Approved Human Genomic Sperm Library D Homo sapiens genomic clone Plate=2171 Col=7 Row=D, genomic survey sequence.//0.012:306:61//AQ125421
R-HEMBA1006569//Ovis aries beta actin mRNA, complete cds.//3.8e-70:529:82//U39357
R-HEMBA1006579//Homo sapiens BAC clone NH0115E20 from Y, complete sequence.//1.0:141:65//AC006032
R-HEMBA1006583//CIT-HSP-2377M16.TR CIT-HSP Homo sapiens genomic clone 2377M16, genomic survey sequence.//1.7e-31:271:76//AQ111875
R-HEMBA1006595//Plasmodium falciparum 3D7 chromosome 12 PFYAC1122 genomic sequence, WORKING DRAFT SEQUENCE, 3 unordered pieces.//0.093:270:61//AC004709
R-HEMBA1006597//Homo sapiens P1 clone GSP13996 from 5q12, complete sequence.//2.7e-45:371:80//AC005031
R-HEMBA1006612
R-nnnnnnnnnnnn//Human DNA sequence *** SEQUENCING IN PROGRESS *** from clone 8B22, WORKING DRAFT SEQUENCE.//2.1e-20:229:77//AL031737
R-HEMBA1006624//Human DNA sequence from clone 406A7 on chromosome 6q23-24. Contains three pseudogenes similar to Elongation Factor 1-Alpha (EF-1-ALPHA, Statin S1), 60S Acidic Ribosomal Protein P1 and NADH-Ubiquinone Oxidoreductase 15 kDa subunit, and part of the Microtuble Associated Protein E-MAP-115 gene. Contains ESTs, STSs and GSSs, complete sequence.//4.8e-40:321:83//AL023284
R-HEMBA1006631//Human DNA sequence *** SEQUENCING IN PROGRESS *** from clone 202O8, WORKING DRAFT SEQUENCE.//1.5e-45:477:77//AL031848
R-HEMBA1006635//***ALU WARNING: Human Alu-Sp subfamily consensus sequence.//8.0e-40:245:91//U14572
R-HEMBA1006639
R-HEMBA1006643
R-HEMBA1006648//Homo sapiens integrin-linked kinase (ILK) mRNA, complete cds.//2.5e-106:567:94//U40282
R-HEMBA1006652//Human BAC clone RG308B22 from 7q22-q31, complete sequence.//8.7e-54:334:76//AC002089
R-HEMBA1006653//Homo sapiens 7q telomere, complete sequence.//5.0e-36:207:89//AF027390
R-HEMBA1006665//HS_3213_B2_D04_T7 CIT Approved Human Genomic Sperm Library D Homo sapiens genomic clone Plate=3213 Col=8 Row=H, genomic survey sequence.//1.2e-21:235:67//AQ175625
R-HEMBA1006674//H.sapiens telomeric DNA sequence, clone 9QTEL023, read 9QTELOO023.seq.//2.6e-32:212:83//Z96776
R-HEMBA1006676//Plasmodium falciparum MAL3P6, complete sequence.//1.9e-10:436:60//Z98551
R-HEMBA1006682//Plasmodium falciparum (strain Dd2) variant-specific surface protein (var-1) gene, complete cds.//6.1e-06:477:59//L40608
R-HEMBA1006695//Homo sapiens clone RG339C12, WORKING DRAFT SEQUENCE, 10 unordered pieces.//1.8e-30:266:80//AC005096
R-HEMBA1006696
R-HEMBA1006708
R-HEMBA1006709//Human DNA sequence *** SEQUENCING IN PROGRESS *** from clone 715N11, WORKING DRAFT SEQUENCE.//6.8e-14:139:82//AL031674
R-HEMBA1006717
R-HEMBA1006737//Homo sapiens chromosome 17, clone hRPK.269_G_24, complete sequence.//9.9e-18:365:66//AC005828
R-HEMBA1006744//Human Chromosome 16 BAC clone CIT987SK-327O24, complete sequence.//1.3e-37:380:75//AC003108
R-HEMBA1006754//Homo sapiens chromosome 5, P1 clone 962c5 (LBNL H87), complete sequence.//2.1e-75:338:85//AC003951
R-HEMBA1006758//Homo sapiens chromosome 5, BAC clone 182a8 (LBNL H161), complete sequence.//1.2e-112:579:95//AC005752
R-HEMBA1006767//Plasmodium falciparum MAL3P6, complete sequence.//0.00022:528:58//Z98551
R-HEMBA1006779//Homo sapiens chromosome 17, clone hRPK.628_E_12, complete sequence.//2.3e-46:305:87//AC005701
R-HEMBA10067801/Human DNA sequence from clone 243E7 on chromosome 22q12.1. Contains ESTs, STSs and GSSs, complete sequence.//7.2e-39:305:82//AL022323
R-HEMBA1006789//Streptomyces coelicolor cosmid 6G4.//0.0085:449:61//AL031317
R-HEMBA1006795//Homo sapiens chromosome 17, clone hRPK.346_K_10, complete sequence.//4.1e-43:355:801/AC006120
R-HEMBA1006796//HS_3038_B2_H11_MF CIT Approved Human Genomic Sperm Library D Homo sapiens genomic clone Plate=3038 Col=22 Row=P, genomic survey sequence.//0.99:158:63//AQ102483
R-HEMBA1006807//Homo sapiens clone DJ0673M15, WORKING DRAFT SEQUENCE, 33 unordered pieces.//8.4e-47:481:75//AC004854
R-HEMBA1006821//Homo sapiens chromosome 17, clone hRPC.62_O_9, complete sequence.//3.0e-08:84:90//AC004797
R-HEMBA1006824//Homo sapiens DNA sequence from PAC 958B3 on chromosome Xp22.11-Xp22.22. Contains ESTs STS and CpG island.//3.7e-54:496:76//Z93023
R-HEMBA1006832//Homo sapiens chromosome 17, clone hRPK.243_K_12, complete sequence.//0.70:206:65//AC005668
R-HEMBA1006849//Homo sapiens 12q24.1 PAC RPCI3-521E19 (Roswell Park Cancer Institute Human PAC library) complete sequence.//1.2e-46:281:91//AC004217
R-HEMBA1006865//Mus musculus clone 101 B1 repeat region sequence.//0.61:115:70//AF056074
R-nnnnnnnnnnnn//Mus musculus mRNA for oxysterol-binding protein, complete cds.//3.3e-102:618:87//AB017026
R-HEMBA1006885 4.2e-14:379:63//AG006839
R-HEMBA1006900//CIT-HSP-2006M20.TR CIT-HSP Homo sapiens genomic clone 2006M20, genomic survey sequence.//2.6e-07:230:66//B56395
R-HEMBA1006921//Homo sapiens PAC clone DJ0777O23 from 7p14-p15, complete sequence.//2.1e-68:267:86//AC005154
R-HEMBA1006926
R-HEMBA1006929//HS_3244_A2_C01_T7 CIT Approved Human Genomic Sperm Library D Homo sapiens genomic clone Plate=3244 Col=2 Row=E, genomic survey sequence.//6.9e-21:191:83//AQ207500
R-HEMBA1006936
R-HEMBA1006938//Colias philodice eriphyle large subunit ribosomal RNA gene, partial sequence; tRNA-Val gene, complete sequence; and small subunit ribosomal RNA gene, partial sequence, mitochondrial genes for mitochondrial RNAs.//0.11:309:59//AF044853
R-HEMBA1006941//Homo sapiens mRNA for putative thioredoxin-like protein.//2.0e-75:371:98//AJ010841
R-HEMBA1006949//Homo sapiens PAC clone DJ0777G09 from 7q34-q36, complete sequence.//0.47:240:63//AC005518
R-HEMBA1006973//HS_2009_A2_A12_MR CIT Approved Human Genomic Sperm Library D Homo sapiens genomic clone Plate=2009 Col=24 Row=A, genomic survey sequence.//9.6e-05:407:60//AQ232302
R-HEMBA1006976//RPCI11-49L11.TJ RPCI11 Homo sapiens genomic clone R-49L11, genomic survey sequence.//0.0018:184:63//AQ051701
R-HEMBA1006993//Human thymopoietin (TMPO) gene, partial exon 6, complete exon 7, partial exon 8, and partial cds for thymopoietin beta.//1.9e-47:394:79//U18271
R-HEMBA1006996//CIT-HSP-2172D17.TF CIT-HSP Homo sapiens genomic clone 2172D17, genomic survey sequence.//1.8e-07:365:62//B93406
R-HEMBA1007002//Plasmodium falciparum MAL3P2, complete sequence.//0.0012:505:56//AL034558
R-HEMBA1007017//Homo sapiens chromosome 17, clone hRPK.597_M_12, complete sequence.//5.6e-41:437:71//AC005277
R-HEMBA1007018//G.gallus mRNA for dynein light chain-A.//8.2e-73:556:80//X79088
R-HEMBA1007045
R-HEMBA1007051//Human DNA sequence from cosmid N69F4 on chromosome 22q11.2-qter contains EST.//9.9e-27:342:71//Z72006
R-HEMBA1007052//Homo sapiens FSHD-associated repeat DNA, proximal region.//5.4e-85:558:87//U85056
R-HEMBA1007062
R-HEMBA1007066
R-HEMBA1007073//Homo sapiens chromosome 17, clone hRPK.421_E_14, complete sequence.//2.0e-66:476:85//AC006141
R-HEMBA1007078//Homo sapiens chromosome 17, clone hRPK.60_A_24, complete sequence.//1.0e-38:179:82//AC005325
R-HEMBA1007085//Homo sapiens clone DJ0965K10, WORKING DRAFT SEQUENCE, 6 unordered pieces.//3.2e-49:551:73//AC006015
R-HEMBA1007087//Human Chromosome 11 pac pDJ392a17, complete sequence.//1.0:261:61//AC000385
R-HEMBA1007112//Homo sapiens chromosome 12p13.3, WORKING DRAFT SEQUENCE, 37 unordered pieces.//0.043:295:62//AC004803
R-HEMBA1007113//Homo sapiens (subclone 6_a8 from P1 H16) DNA sequence.//1.4e-52:307:87//L43392
R-HEMBA1007129//Human DNA sequence from PAC 863K19 on chromosome X. Contains STS.//1.2e-08:131:75//Z92547
R-HEMBA1007147//H.sapiens CpG island DNA genomic Mse1 fragment, clone 65f1, reverse read cpg65f1.rt1a.//0.16:187:64//Z62246
R-HEMBA1007149//Homo sapiens chromosome 19, cosmid F23149, complete sequence.//7.6e-108:543:96//AC005239
R-HEMBA1007151//Homo sapiens PAC clone DJ0745K06 from 7q31, complete sequence.//0.14:323:58//AC004875
R-nnnnnnnnnnnn//Homo sapiens epsin 2a mRNA, complete cds.//5.1e-103:529:94//AF062085
R-HEMBA1007178//Homo sapiens chromosome 12p13.3 clone RPCI11-372B4, WORKING DRAFT SEQUENCE, 129 ordered pieces.//5.4e-106:537:96//AC005911
R-HEMBA1007194//Homo sapiens Xp22 bins 87-93 PAC RPCI1-122K4 (Roswell Park Cancer Institute Human PAC Library) complete sequence.//4.1e-39:262:80//AC003035
R-HEMBA1007203//Homo sapiens mRNA for KIAA0214 protein, complete cds.//5.3e-61:332:95//D86987
R-HEMBA1007206//Homo sapiens DNA sequence from PAC 958B3 on chromosome Xp22.11-Xp22.22. Contains ESTs STS and CpG island.//1.9e-50:436:81//Z93023
R-HEMBA1007224//Homo sapiens mRNA for KIAA0797 protein, partial cds.//2.3e-96:471:97//AB018340
R-HEMBA1007251//Homo sapiens chromosome 5, PAC clone 247f3 (LBNL H85), complete sequence.//0.011:349:62//AC004777
R-HEMBA1007256//Homo sapiens PAC clone DJ0676L20 from 7q35-q36, complete sequence.//2.8e-10:224:70//AC004856
R-HEMBA1007267//Homo sapiens Chr.14 PAC RPCI4-794B2 (Roswell Park Cancer Institute Human PAC Library) complete sequence.//3.4e-53:362:86//AC005924
R-HEMBA1007273
R-HEMBA1007279//Rickettsia prowazekii strain Madrid E, complete genome; segment 4/4.//0.042:454:57//AJ235273
R-HEMBA1007281//Rickettsia prowazekii strain Madrid E, complete genome; segment 3/4.//0.99:288:60//AJ235272
R-HEMBA1007288//Human DNA sequence from clone 422G23 on chromosome 6q24 Contains EST, STS, GSS, CpG island, complete sequence.//7.4e-107:554:95//AL031003
R-HEMBA1007300//Caenorhabditis elegans cosmid C48C5.//0.22:474:59//U39994
R-HEMBA1007301
R-HEMBA1007319//Campylobacter jejuni repetitive DNA, clone pINT.//4.9e-08:524:58//Y14425
R-HEMBA1007320//Homo sapiens genomic DNA, chromosome 21q11.1, segment 14/28, WORKING DRAFT SEQUENCE.//3.4e-16:244:71//AP000043
R-HEMBA1007322//Homo sapiens BAC clone RG324D18 from 7p15-p21, complete sequence.//3.9e-83:383:85//AC005251
R-HEMBA1007327//Human DNA sequence *** SEQUENCING IN PROGRESS *** from clone 7706, WORKING DRAFT SEQUENCE.//1.6e-38:533:71//Z96804
R-HEMBA1007341//Human DNA sequence *** SEQUENCING IN PROGRESS *** from clone 268D13, WORKING DRAFT SEQUENCE.//3.6e-21:394:66//AL023513
R-HEMBA1007342//Human BAC clone GS368F15 from 7q31, complete sequence.//1.7e-15:190:73//AC003080
R-HEMBA1007347//Human DNA sequence *** SEQUENCING IN PROGRESS *** from clone N38G6, WORKING DRAFT SEQUENCE.//2.2e-47:455:77//Z96802
R-HEMBB1000005//Homo sapiens 3p21.1-9 PAC RPCI4-793P23 (Roswell Park Cancer Institute Human PAC Library) complete sequence.//1.1e-62:539:79//AC006208
R-HEMBB1000008//Homo sapiens chromosome 17, clone hCIT.211_P_7, complete sequence.//1.2e-36:285:83//AC003665
R-HEMBB1000018//Homo sapiens clone DJ0038I10, WORKING DRAFT SEQUENCE, 5 unordered pieces.//1.2e-51:416:79//AC004820
R-HEMBB1000024//Human DNA sequence from BAC 175E3 on chromosome 22q11.2-qter. Contains ESTs, STSs and polymorphic CA repeat.//3.9e-18:211:79//Z95113
R-HEMBB1000025//HS_3064_B2_B07_MF CIT Approved Human Genomic Sperm Library D Homo sapiens genomic clone Plate=3064 Col=14 Row=D, genomic survey sequence.//5.9e-40:254:90//AQ132765
R-HEMBB1000030//Human DNA sequence from clone 108K11 on chromosome 6p21 Contains SRP20 (SR protein family member), Ndr protein kinase gene similar to yeast suppressor protein SRP40, EST and GSS, complete sequence.//1.5e-32:452:70//Z85986
R-HEMBB1000036//CIT-HSP-2024L15.TF CIT-HSP Homo sapiens genomic clone 2024L15, genomic survey sequence.//9.3e-63:541:77//B66264
R-HEMBB1000037//Homo sapiens erythroblast macrophage protein EMP mRNA, complete cds.//7.6e-91:467:97//AF084928
R-HEMBB1000039//Homo sapiens chromosome 17, clone hRPK.401_O_9, complete sequence.//2.4e-44:456:68//AC005291
R-HEMBB1000044//Human BAC clone RG016J04 from 7q21, complete sequence.//1.4e-54:307:80//AC002064
R-HEMBB1000048//Homo sapiens Chromosome 16 BAC clone CIT987SK-A-279B10, complete sequence.//3.8e-09:330:63//AC002300
R-HEMBB1000050//Human DNA sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.//6.7e-12:225:65//Z94056
R-HEMBB1000054//Human DNA sequence from clone 444C7 on chromosome 6p22.3-23. Contains an EST, an STS and GSSs, complete sequence.//8.9e-76:557:82//AL033521
R-HEMBB1000055//Human housekeeping (Q1Z 7F5) gene, exons 2 through 7, complete cds.//1.6e-88:350:86//M81806
R-HEMBB1000059//Homo sapiens clone DJ0850I01, WORKING DRAFT SEQUENCE, 1 unordered pieces.//4.9e-12:356:65//AC006009
R-HEMBB1000083//Homo sapiens clone DJ0607J02, WORKING DRAFT SEQUENCE, 12 unordered pieces.//3.7e-41:311:82//AC004840
R-HEMBB1000089//Homo sapiens clone DJ1021I20, WORKING DRAFT SEQUENCE, 6 unordered pieces.//3.6e-34:314:78//AC005520
R-HEMBB1000099//Homo sapiens DNA sequence from BAC 1216H12 on chromosome 22q12. Contains a pseudogene with similarity to part of mouse Ninein and the KIAA0609 gene for a protein similar to C. elegans K09C8.4. Contains ESTs, GSSs and a ggtt repeat polymorphism, complete sequence.//8.8e-32:434:71//AL008715
R-HEMBB1000103//Human DNA sequence from BAC 445C9 on chromosome 22q12.1. Contains CRYBB1, beta B1 crystallin, CRYBA4, beta A4 crystallin, high mobility group-1 protein (HMG-1), ESTs.//2.5e-16:207:74//Z95115
R-HEMBB1000113//HS_3013_A1_B08_T7 CIT Approved Human Genomic Sperm Library D Homo sapiens genomic clone Plate=3013 Col=15 Row=C, genomic survey sequence.//0.94:211:63//AQ118730
R-HEMBB1000119//Homo sapiens ASMTL gene.//1.9e-106:531:96//Y15521
R-HEMBB1000136//Human Chromosome X, complete sequence.//0.00073:359:59//AC002407
R-HEMBB1000141//Homo sapiens chromosome 21q22.3 PAC 39C17, complete sequence.//6.8e-41:280:74//AF043945
R-HEMBB1000144//Homo sapiens chromosome 17, clone hCIT.507_E_2, complete sequence.//0.00083:206:66//AC004134
R-HEMBB1000173//Homo sapiens, WORKING DRAFT SEQUENCE, 97 unordered pieces.//2.5e-82:401:90//AC004085
R-HEMBB1000175
R-HEMBB1000198//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs (BAC end sequences) and a CA repeat polymorphism, complete sequence.//0.91:428:56//AL021368
R-HEMBB1000215//Homo sapiens DNA sequence from PAC 69E11 on chromosome 1q23-24. Contains a NADH-Ubiquinone Oxidoreductase MLRQ subunit (EC 1.6.5.3, EC 1.6.99.3, CI-MLRQ) LIKE pseudogene, a 60S Ribosomal protein L34 LIKE pseudogene, an unknown gene similar to yeast YPR037W and worm C02C2.6 predicted genes, a predicted CpG island, ESTs and an STS, complete sequence.//4.4e-54:298:91//AL021397
R-HEMBB1000217
R-HEMBB1000218//Homo sapiens 12q24 PAC RPCI1-66E7 (Roswell Park Cancer Institute Human PAC library) complete sequence.//5.8e-32:517:70//AC004216
R-HEMBB1000226//Human DNA sequence from cosmid COS12 from a contig from the tip of the short arm of chromosome 16, spanning 2Mb of 16p13.3. Contains ESTs, Flanking sequences of 3' alpha globin HVR and CpG island.//2.5e-77:450:92//Z69706
R-HEMBB1000240//Homo sapiens chromosome 9 duplication of the T cell receptor beta locus and trypsinogen gene families.//4.1e-05:310:62//AF029308
R-HEMBB1000244//Human DNA sequence *** SEQUENCING IN PROGRESS *** from clone 1112F19, WORKING DRAFT SEQUENCE.//1.3e-43:278:85//AL034420
R-HEMBB1000250//Human DNA sequence from clone 34B20 on chromosome 6p21.31-22.2. Contains seventeen Histone (pseudo)genes and a 40S Ribosomal protein S10 pseudogene. Contains ESTs, STSs and GSSs, complete sequence.//3.8e-16:484:64//AL031777 R-HEMBB1000258//Human hereditary haemochromatosis region, histone 2A-like protein gene, hereditary haemochromatosis (HLA-H) gene, RoRet gene, and sodium phosphate transporter (NPT3) gene, complete cds.//4.3e-11:286:67//U91328
R-HEMBB1000264//Mus musculus enhancer of polycomb (Epc1) mRNA, complete cds.//1.2e-42:406:79//AF079765
R-HEMBB1000266//RPCI11-76C20.TV RPCI11 Homo sapiens genomic clone R-76C20, genomic survey sequence.//1.0:232:59//AQ265533
R-HEMBB1000272//HS_3032_B1_H06_MF CIT Approved Human Genomic Sperm Library D Homo sapiens genomic clone Plate=3032 Col=11 Row=P, genomic survey sequence.//0.0082:209:62//AQ096702
R-HEMBB1000274//Homo sapiens Chromosome 22q11.2 Cosmid Clone 817g In IGLC Region, complete sequence.//1.6e-45:277:72//AC000053
R-HEMBB1000284//Homo sapiens full-length insert cDNA clone YY88A05.//6.9e-112:572:96//AF088018
R-HEMBB1000307//Homo sapiens chromosome 17, clone hRPK.471_L_13, complete sequence.//5.7e-96:523:93//AC005244
R-HEMBB1000312//Human DNA sequence *** SEQUENCING IN PROGRESS *** from clone 32B1, WORKING DRAFT SEQUENCE.//7.5e-21:218:67//AL023693
R-HEMBB1000317//Toxoplasma gondii chloroplast, complete genome.//0.062:354:58//U87145
R-HEMBB1000318//Human DNA sequence from PAC 292H14 on chromosome Xp21. Contains STS and CA repeat polymorphism.//4.5e-52:302:81//AL008710
R-HEMBB1000335//Homo sapiens chromosome 5, P1 clone 1041F10 (LBNL H88), complete sequence.//1.9e-16:139:84//AC005179
R-HEMBB1000336//Homo sapiens complete genomic sequence between D16S3070 and D16S3275, containing Familial Mediterranean Fever gene disease.//0.0062:231:64//AJ003147
R-HEMBB1000337//CIT-HSP-2329010.TF CIT-HSP Homo sapiens genomic clone 2329O10, genomic survey sequence.//1.2e-31:192:92//AQ035976
R-HEMBB1000338//Homo sapiens Chromosome 16 BAC clone CIT987SK-A-248F7, complete sequence.//1.9e-39:477:71//AC004605
R-HEMBB1000339//Human DNA sequence *** SEQUENCING IN PROGRESS *** from clone 862K6, WORKING DRAFT SEQUENCE.//4.1e-54:357:76//AL031681
R-HEMBB1000341//Homo sapiens 12q24 PAC RPCI3-424M6 (Roswell Park Cancer Institute Human PAC library) complete sequence.//1.8e-19:501:63//AC002350
R-HEMBB1000343//Homo sapiens chromosome 16, cosmid clone 367E12 (LANL), complete sequence.//3.6e-41:457:72//AC004644
R-HEMBB1000354//Human DNA sequence from PAC 560B9 on chromosome 1q24-1q25. Contains profilin-like pseudogene, 60S ribosomal protein L4 pseudogene RNA binding protein, ESTs, GSS.//7.2e-36:325:74//Z98751
R-HEMBB1000369//Homo sapiens chromosome 4 clone B366O24 map 4q25, complete sequence.//9.0e-25:179:79//AC004067
R-HEMBB10003741/Human DNA sequence *** SEQUENCING IN PROGRESS *** from clone 75N14, WORKING DRAFT SEQUENCE.//8.4e-58:332:79//Z97199
R-HEMBB1000376//Homo sapiens DNA for amyloid precursor protein, complete cds.//2.1e-47:309:88//D87675
R-HEMBB1000391//Homo sapiens clone RG269P13, WORKING DRAFT SEQUENCE, 6 unordered pieces.//5.7e-46:302:85//AC005080
R-HEMBB1000399//Homo sapiens Radl7-like protein (RAD17) mRNA, complete cds.//1.0e-107:531:97//AF076838
R-HEMBB1000402//Human DNA sequence from clone 505B13 on chromosome 1p36.2-36.3 Contains CA repeat and GSSs, complete sequence.//1.1e-25:441:67//Z98052
R-HEMBB1000404//HS_2246_A2_D01_MF CIT Approved Human Genomic Sperm Library D Homo sapiens genomic clone Plate=2246 Col=2 Row=G, genomic survey sequence.//0.0025:196:63//AQ084251
R-HEMBB1000420//Homo sapiens Chromosome 22q11.2 Cosmid Clone 817g In IGLC Region, complete sequence.//1.2e-29:358:72//AC000053
R-HEMBB1000434//Homo sapiens chromosome 4 clone B71M12 map 4q25, complete sequence.//2.8e-51:299:89//AC004069
R-HEMBB1000438//HS_2239_B2_E08_MF CIT Approved Human Genomic Sperm Library D Homo sapiens genomic clone Plate=2239 Col=16 Row=J, genomic survey sequence.//1.3e-10:76:100//AQ067700
R-HEMBB1000441//Human DNA sequence *** SEQUENCING IN PROGRESS *** from clone 424J12, WORKING DRAFT SEQUENCE.//4.4e-60:281:90//Z82207
R-HEMBB1000449//Homo sapiens clone DJ0898O18, WORKING DRAFT SEQUENCE, 8 unordered pieces.//4.8e-11:228:68//AC004920
R-HEMBB1000455//Homo sapiens clone GS051M12, complete sequence.//3.1e-14:388:65//AC005007
R-HEMBB1000472//Homo sapiens chromosome 17, clone HCIT48C15, complete sequence.//4.9e-34:320:79//AC003104
R-HEMBB1000480//Human DNA sequence from Fosmid 65B7 on chromosome 22q11.2-qter. Contains exons 6-12 of the SLC5A1 (SGLT1) gene for solute carrier family 5 (sodium/glucose cotransporter) member 1 (High Affinity Sodium-Glucose Cotransporter), complete sequence.//3.4e-36:285:82//Z83849
R-HEMBB1000487
R-HEMBB1000490//Human DNA sequence *** SEQUENCING IN PROGRESS *** from clone 1185N5, WORKING DRAFT SEQUENCE.//1.5e-34:281:81//AL034423
R-HEMBB1000491//Homo sapiens DNA sequence from PAC 958B3 on chromosome Xp22.11-Xp22.22. Contains ESTs STS and CpG island.//8.5e-37:483:72//Z93023
R-HEMBB1000493//Human DNA sequence from clone 109F14 on chromosome 6p21.2-21.3. Contains the alternatively spliced gene for Transcriptional Enhancer Factor TEF-5, the 60S Ribosomal Protein RPL10A gene, a PUTATIVE ZNF127 LIKE gene, and the PPARD for Peroxisome Proliferator Activated Receptor Delta (PPAR-Delta, PPAR-Beta, Nuclear Hormone Receptor 1, NUC1, NUCI, PPARB). Contains three putative CpG islands, ESTs, STSs, GSSs and a ca repeat polymorphism, complete sequence.//7.6e-14:217:71//AL022721
R-HEMBB1000510//Human DNA sequence *** SEQUENCING IN PROGRESS *** from clone 27K12, WORKING DRAFT SEQUENCE.//7.1e-44:221:80//AL033397
R-HEMBB1000518//Human PAC clone DJ327A19 from Xq25-q26, complete sequence.//3.5e-51:280:90//AC002477
R-HEMBB1000523//Homo sapiens PAC clone DJ0167F23 from 7p15, complete sequence.//1.7e-53:304:82//AC004079
R-HEMBB1000530//Homo sapiens chromosome 17, clone hCIT.162_E_12, complete sequence.//4.2e-74:428:92//AC006236
R-HEMBB1000550//Human Chromosome 16 BAC clone CIT987SK-A-363E6, complete sequence.//5.6e-13:112:80//U91321
R-HEMBB1000554//Human DNA sequence *** SEQUENCING IN PROGRESS *** from clone 409J21, WORKING DRAFT SEQUENCE.//5.1e-14:239:63//Z83824
R-HEMBB1000556//Homo sapiens envoplakin (EVPL) mRNA, complete cds.//0.031:275:60//U53786
R-HEMBB1000564//Homo sapiens chromosome 5, Bac clone 189 (LBNL H135), complete sequence.//3.1e-17:227:76//AC005914
R-HEMBB1000573//Borrelia afzelii (strain NT28) DNA, internal transcribed spacer.//0.078:161:63//D84405
R-HEMBB1000575//Homo sapiens chromosome 17, clone hRPC.859_O_20, complete sequence.//7.2e-52:260:80//AC003695
R-HEMBB1000586//Human DNA sequence from cosmid V210E9, between markers DXS366 and DXS87 on chromosome X.//2.0e-33:305:79//Z70280
R-HEMBB1000589//Homo sapiens chromosome 17, clone hRPK.1064_E_11, complete sequence.//1.3e-14:409:65//AC005208
R-HEMBB1000591//Homo sapiens Xp22 bins 45-47 BAC GSHB-665N22 (Genome Systems Human BAC Library) complete sequence.//6.2e-39:493:71//AC005184
R-HEMBB1000592//Homo sapiens 12p13.3 PAC RPCI5-1180D12 (Roswell Park Cancer Institute Human PAC Library) complete sequence.//1.6e-08:254:64//AC005831
R-HEMBB1000598//Homo sapiens chromosome 11 pac pDJ159ol, complete sequence.//3.3e-38:407:76//AC000381
R-HEMBB1000623//CIT-HSP-2374P17.TR CIT-HSP Homo sapiens genomic clone 2374P17, genomic survey sequence.//1.3e-41:212:100//AQ109717
R-HEMBB1000630//Human DNA sequence from clone 413H6 on chromosome 6p22.3-24.3. Contains a hamster Androgen-dependent Expressed Protein like protein gene, ESTs and GSSs, complete sequence.//5.2e-31:319:78//AL022724
R-HEMBB1000631//Sequence 28 from patent US 5708157.//6.8e-20:208:80//I80058
R-HEMBB1000632//Homo sapiens Cosmid C4, WORKING DRAFT SEQUENCE, 1 ordered pieces.//7.4e-47:457:75//AC004176
R-HEMBB1000637//Human BAC clone RG094H21 from 7q21-q22, complete sequence.//2.9e-45:263:87//AC003085
R-HEMBB1000638//Genomic sequence from Human 6, complete sequence.//9.1e-34:375:73//AC002112
R-HEMBB1000643//HS_2242_A2_B07_MF CIT Approved Human Genomic Sperm Library D Homo sapiens genomic clone Plate=2242 Col=14 Row=C, genomic survey sequence.//0.010:239:60//AQ065993
R-HEMBB1000649//Homo sapiens RBP56/hTAFII68 gene, exon 7.//8.3e-63:306:100//AB010061
R-HEMBB1000652//Human DNA sequence from PAC 467D16 on chromosome 6p22.3-24.1. Contains the 3' part of the SCA1 (ataxin-1) gene with a poly-glutamine (CAG repeat) polymorphism, the 3' part of the GMPR (GMP reductase, Guanosine 5'-monophosphate oxidoreductase) gene, ESTs and an STS with a polymorphic CA repeat.//3.3e-14:450:64//AL009031
R-HEMBB1000665//Arabidopsis thaliana genomic DNA, chromosome 5, P1 clone: MXA21, complete sequence.//0.98:251:63//AB005247
R-HEMBB1000671//Human DNA sequence from PAC 106C24, between markers DXS294 and DXS730 on chromosome X.//6.8e-58:296:85//Z83313
R-HEMBB1000673//CITBI-E1-2506F20.TR CITBI-E1 Homo sapiens genomic clone 2506F20, genomic survey sequence.//0.98:71:76//AQ264731
R-HEMBB1000684//Human DNA sequence from clone 1158E12 on chromosome Xp11.23-11.4 Contains EST, STS, GSS, CpG island, complete sequence.//2.6e-11:153:77//AL031584
R-nnnnnnnnnnnn//Homo sapiens neuroan1 mRNA, complete cds.//2.0e-50:287:93//AF040723
R-HEMBB1000705//Homo sapiens chromosome 19, cosmid R30538, complete sequence.//3.4e-18:340:65//AC005943
R-HEMBB1000706//Human DNA sequence *** SEQUENCING IN PROGRESS *** from clone 462C17, WORKING DRAFT SEQUENCE.//4.7e-10:358:64//AL033380
R-HEMBB1000709//RPCI11-79A8.TV RPCI11 Homo sapiens genomic clone R-79A8, genomic survey sequence.//1.4e-40:262:89//AQ282374
R-HEMBB1000725//Arabidopsis thaliana genomic DNA, chromosome 5, P1 clone: MGN6, complete sequence.//0.00018:386:60//AB017066
R-HEMBB1000726//Homo sapiens PAC clone DJ1185I07 from 7q11.23-q21, complete sequence.//1.5e-48:316:88//AC004990
R-HEMBB1000738//Homo sapiens PAC clone DJ0745K06 from 7q31, complete sequence.//7.1e-53:382:85//AC004875
R-HEMBB1000749//Homo sapiens clone RG140B11, WORKING DRAFT SEQUENCE, 1 unordered pieces.//6.5e-51:438:80//AC005069
R-HEMBB1000763//Plasmid Col Ib-P9 (from E.coli K12) colicin Ib promoter region and 5' coding region.//1.0:115:63//K02071
R-HEMBB1000770//Human Rhesus blood group antigen (RHCE) gene, intron 6, partial sequence.//5.6e-24:183:86//U83205
R-HEMBB1000781//Homo sapiens Xp22 PACs RPC11-263P4 and RPC11-164K3 complete sequence.//0.00054:154:67//AC003046
R-HEMBB1000789//RPCI11-2I14.TVB RPCI-11 Homo sapiens genomic clone RPCI-11-2I14, genomic survey sequence.//3.0e-09:299:64//B63628
R-HEMBB1000790//Human Chromosome 16 BAC clone CIT987SK-A-362G6, complete sequence.//4.5e-46:185:85//U95740
R-HEMBB1000794//HS_3253_A1_G06_T7 CIT Approved Human Genomic Sperm Library D Homo sapiens genomic clone Plate=3253 Col=11 Row=M, genomic survey sequence.//5.7e-13:172:65//AQ216291
R-HEMBB1000807
R-HEMBB1000810//Human BAC clone RG114A06 from 7q31, complete sequence.//1.3e-24:385:71//AC002542
R-HEMBB1000821
R-HEMBB1000822//CITBI-E1-2517E13.TF CITBI-E1 Homo sapiens genomic clone 2517E13, genomic survey sequence.//4.5e-08:278:64//AQ279944
R-HEMBB1000826//Homo sapiens genomic DNA, chromosome 21q11.1, segment 14/28, WORKING DRAFT SEQUENCE.//1.2e-44:521:72//AP000043
R-HEMBB1000827//Homo sapiens clone DJ0981O07, complete sequence.//6.8e-43:319:84//AC006017
R-HEMBB1000831//HS_3247_B2_A09_T7 CIT Approved Human Genomic Sperm Library D Homo sapiens genomic clone Plate=3247 Col=18 Row=B, genomic survey sequence.//5.5e-74:381:96//AQ223850
R-HEMBB1000835//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs (BAC end sequences) and a CA repeat polymorphism, complete sequence.//4.2e-17:167:80//AL021368
R-HEMBB1000840//Homo sapiens clone DJ1039L24, WORKING DRAFT SEQUENCE, 3 unordered pieces.//7.9e-26:220:73//AC005283
R-HEMBB1000848//Homo sapiens, WORKING DRAFT SEQUENCE, 52 unordered pieces.//7.8e-39:356:79//AC004086
R-HEMBB1000852//HS_3075_A2_B07_MF CIT Approved Human Genomic Sperm Library D Homo sapiens genomic clone Plate=3075 Col=14 Row=C, genomic survey sequence.//3.4e-11:151:75//AQ138816
R-HEMBB1000870//Human DNA sequence *** SEQUENCING IN PROGRESS *** from clone 72E17, WORKING DRAFT SEQUENCE.//1.8e-44:454:75//AL033523
R-HEMBB1000876//Human DNA sequence from clone 91J24 on chromosome 6q24 Contains part of utrophin Gene, part of cytochrome C oxidase gene, EST, CpG island, complete sequence.//0.0016:227:65//AL024474
R-HEMBB1000883//Homo sapiens chromosome 19, cosmid F19678, complete sequence.//0.62:238:62//AC005621
R-HEMBB1000887//Synthetic human/adenovirus type 5 recombination junction.//9.9e-24:275:76//M34061
R-HEMBB1000888//CIT-HSP-2282A13.TR CIT-HSP Homo sapiens genomic clone 2282A13, genomic survey sequence.//2.4e-05:310:60//AQ000826
R-HEMBB1000890//Homo sapiens clone DJ0042M02, WORKING DRAFT SEQUENCE, 20 unordered pieces.//6.5e-44:305:84//AC005995
R-HEMBB1000893//Homo sapiens BAC clone RG363E19 from 7q31.1, complete sequence.//3.7e-30:265:80//AC004492
R-HEMBB1000908//RPCI11-13P12.TV RPCI-11 Homo sapiens genomic clone RPCI-11-13P12, genomic survey sequence.//0.98:183:61//B76199
R-HEMBB1000910//Homo sapiens Chromosome 22q11.2 Cosmid Clone 50d10 In IGLC Region, complete sequence.//1.7e-28:302:76//AC000024
R-HEMBB1000913//Homo sapiens Xp22 BAC GSHB 526D21 (Genome Systems Human BAC library) complete sequence.//4.1e-34:314:76//AC003037
R-HEMBB1000915//Human chromosome 16p11.2-p12 BAC clone CIT987SK-224D6 complete sequence.//6.3e-09:536:59//U95739
R-HEMBB1000917//Human DNA sequence *** SEQUENCING IN PROGRESS *** from clone 169I5, WORKING DRAFT SEQUENCE.//1.6e-47:234:86//Z93015
R-HEMBB1000927
R-HEMBB1000947//CIT-HSP-2287M13.TF CIT-HSP Homo sapiens genomic clone 2287M13, genomic survey sequence.//0.090:115:69//B99228
R-HEMBB1000959//Homo sapiens chromosome 17, clone HRPC905N1, complete sequence.//5.7e-89:544:90//AC003098
R-HEMBB1000973//Arabidopsis thaliana chromosome II BAC F2I9 genomic sequence, complete sequence.//0.038:377:58//AC005560
R-HEMBB1000975//Arabidopsis thaliana chromosome II BAC F5H14 genomic sequence, complete sequence.//1.0e-05:342:62//AC006234
R-HEMBB1000981//CIT-HSP-2386J13.TF.1 CIT-HSP Homo sapiens genomic clone 2386J13, genomic survey sequence.//1.1e-18:231:74//AQ239443
R-HEMBB1000985//HS_3184_A1_D12_T7 CIT Approved Human Genomic Sperm Library D. Homo sapiens genomic clone Plate=3184 Col=23 Row=G, genomic survey sequence.//6.3e-52:286:95//AQ150008
R-HEMBB1000991
R-HEMBB1000996//Homo sapiens Xq28 BAC PAC and cosmid clones containing FMR2 gene exons 1,2, and 3, complete sequence.//1.4e-42:343:81//AC002368
R-HEMBB1001004
R-HEMBB1001008//CITBI-E1-2504L23.TF CITBI-E1 Homo sapiens genomic clone 2504L23, genomic survey sequence.//3.1e-57:317:94//AQ262056
R-HEMBB1001011//HS_3017_B1_G03_T7 CIT Approved Human Genomic Sperm Library D Homo sapiens genomic clone Plate=3017 Col=5 Row=N, genomic survey sequence.//7.3e-34:237:86//AQ101944
R-HEMBB1001014//Human DNA sequence *** SEQUENCING IN PROGRESS *** from clone 460J8, WORKING DRAFT SEQUENCE.//2.4e-49:417:80//AL031662
R-HEMBB1001020//Homo sapiens Xp22 BAC GS-377014 (Genome Systems Human BAC library) complete sequence.//7.6e-41:303:76//AC002549
R-HEMBB1001024//Homo sapiens (subclone 2_g5 from P1 H16) DNA sequence.//7.4e-48:341:85//L48475
R-HEMBB1001037//Homo sapiens 22q11 BAC Clone 489d1 In MDR Region, complete sequence.//2.0e-50:416:82//AC005527
R-HEMBB1001047//Homo sapiens chromosome 19, cosmid R31973, complete sequence.//8.4e-22:288:71//AC004699
R-HEMBB1001051//H.sapiens mRNA for FAN protein.//7.1e-18:114:98//X96586
R-HEMBB1001056//Homo sapiens clone DJ0953A04, WORKING DRAFT SEQUENCE, 5 unordered pieces.//6.1e-94:520:93//AC006014
R-HEMBB1001058//Homo sapiens clone UWGC:y17c131 from 6p21, complete sequence.//1.1e-56:242:82//AC004187
R-HEMBB1001060//Human Tigger1 transposable element, complete consensus sequence.//4.2e-66:323:81//U49973
R-HEMBB1001063//Human DNA sequence *** SEQUENCING IN PROGRESS *** from clone 523G1, WORKING DRAFT SEQUENCE.//4.0e-114:556:98//AL034375
R-HEMBB1001068//Homo sapiens liprin-beta2 mRNA, partial cds.//2.8e-105:512:97//AF034803
R-HEMBB1001096//Human DNA sequence from PAC 246O8, between markers DXS6791 and DXS8038 on chromosome X contains ESTs.//2.4e-13:225:69//Z76735
R-HEMBB1001102//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//2.4e-35:295:80//AL022577
R-HEMBB1001105//Human DNA sequence *** SEQUENCING IN PROGRESS *** from clone 462O23, WORKING DRAFT SEQUENCE.//7.9e-46:380:80//AL031431
R-HEMBB1001114//Homo sapiens DNA sequence from PAC 119E23 on chromosome Xq25-q27.1. Contains glypican-3 precursor (intestinal protein OCI-5) (GTR2-2),5'UTR. ESTs, STS.//1.1e-38:306:84//Z99570
R-HEMBB1001117//RPCI11-35I8.TK RPCI-11 Homo sapiens genomic clone RPCI-11-35I8, genomic survey sequence.//1.5e-08:67:100//AQ047113
R-HEMBB1001119//Homo sapiens BAC clone BK085E05 from 22q12.1-qter, complete sequence.//9.0e-26:481:67//AC003071
R-HEMBB1001126//Human DNA sequence from clone 441J1 on chromosome 6p24 Contains STS, GSS, complete sequence.//0.045:127:69//Z99495
R-HEMBB1001133//Human SS-A/Ro ribonucleoprotein autoantigen 60 kd subunit mRNA, complete cds.//5.0e-23:285:73//M25077
R-HEMBB1001137//Plasmodium falciparum DNA *** SEQUENCING IN PROGRESS *** from contig 4-09, complete sequence.//2.5e-07:334:62//AL010222
R-HEMBB1001142//Human BAC clone RG164L14 from 7q21-q22, complete sequence.//2.5e-46:412:79//AC002564
R-HEMBB1001151//Mus musculus IFN alpha-treated embryonic fibroblast mRNA.//1.8e-11:148:77//U51904
R-HEMBB1001153//RPCI11-10L7.TP RPCI-11 Homo sapiens genomic clone RPCI-11-10L7, genomic survey sequence.//2.3e-34:213:82//B71766
R-HEMBB1001169//Homo sapiens chromosome 17, clone HCIT39G8, complete sequence.//0.040:465:56//AC003070
R-nnnnnnnnnnnn//Sequence 1 from patent US 5618695.//2.8e-15:176:80//I40055
R-HEMBB1001177
R-HEMBB1001182//Plasmodium falciparum DNA *** SEQUENCING IN PROGRESS *** from contig 4-52, complete sequence.//1.9e-05:174:70//AL010226
R-HEMBB1001199
R-HEMBB1001208
R-HEMBB1001209//RPCI11-41E13.TP RPCI-11 Homo sapiens genomic clone RPCI-11-41E13, genomic survey sequence.//1.1e-95:473:97//AQ029098
R-HEMBB1001210//Homo sapiens chromosome 16, cosmid clone 330D11 (LANL), complete sequence.//6.2e-08:412:61//AC005199
R-HEMBB1001218//RPCI11-13L8.TV RPCI-11 Homo sapiens genomic clone RPCI-11-13L8, genomic survey sequence.//1.0e-46:498:74//B75158
R-HEMBB1001221//RPCI11-62024.TJ RPCI11 Homo sapiens genomic clone R-62024, genomic survey sequence.//3.2e-09:215:68//AQ200950
R-HEMBB1001234
R-HEMBB1001242
R-HEMBB1001249//Homo sapiens clone DJ1136G02, WORKING DRAFT SEQUENCE, 4 unordered pieces.//1.4e-33:361:72//AC005377
R-HEMBB1001253//Homo sapiens chromosome 3, olfactory receptor pseudogene cluster 1, complete sequence, and myosin light chain kinase (MLCK) pseudogene, partial sequence.//3.8e-105:517:98//AF042089
R-HEMBB1001254//Methanococcus jannaschii section 3 of 150 of the complete genome.//0.96:203:61//U67461
R-HEMBB1001267//Human DNA sequence from clone 14O9 on chromosome Xp11.1-11.4. Contains a Inter-Alpha-Trypsin Inhibitor Heavy Chain LIKE gene, a alternatively spliced Melanoma-Associated Antigen MAGE LIKE gene and a 6-Phosphofructo-2-kinase (Fructose-2,6-bisphosphatase) LIKE pseudogene. Contains ESTs, STSs and genomic marker DXS8032, complete sequence.//2.8e-39:320:80//Z98046
R-HEMBB1001271//Homo sapiens chromosome 17, clone hRPK.349_A_8, complete sequence.//3.9e-47:494:75//AC005544
R-HEMBB1001282//Human DNA sequence *** SEQUENCING IN PROGRESS *** from clone 184J9, WORKING DRAFT SEQUENCE.//0.0011:97:79//AL031428
R-HEMBB1001288
R-HEMBB1001289//Homo sapiens chromosome 5, BAC clone 343g16 (LBNL H180), complete sequence.//2.0e-31:301:78//AC005601
R-HEMBB1001294//Homo sapiens BAC clone RG060N22 from 7q21, complete sequence.//0.053:283:60//AC003083
R-HEMBB1001302
R-HEMBB1001304//Human DNA sequence *** SEQUENCING IN PROGRESS *** from clone 27K12, WORKING DRAFT SEQUENCE.//6.3e-15:396:64//AL033397
R-HEMBB1001314//Homo sapiens genomic DNA, 21q region, clone: f30F8SpN6, genomic survey sequence.//3.4e-42:293:86//AG013777
R-HEMBB1001315//Human NFE genomic fragment.//7.5e-30:243:78//M98511
R-HEMBB1001317//Homo sapiens chromosome 17, clone hRPC.1028_K_7, complete sequence.//2.3e-39:301:82//AC004585
R-HEMBB1001326//HS_3054_A1_F12_MR CIT Approved Human Genomic Sperm Library D Homo sapiens genomic clone Plate=3054 Col=23 Row=K, genomic survey sequence.//0.90:117:63//AQ106096
R-HEMBB1001331//Mus musculus mRNA for hepatoma-derived growth factor, complete cds, strain:BALB/c.//0.037:103:77//D63850
R-HEMBB1001335//Homo sapiens Xp22 BAC GSHB 526D21 (Genome Systems Human BAC library) complete sequence.//9.1e-19:229:77//AC003037
R-HEMBB1001337
R-HEMBB1001339//Homo sapiens FSHD-associated repeat DNA, proximal region.//2.9e-45:551:72//U85056
R-HEMBB1001346//Homo sapiens phenylalanine-tRNA synthetase (FARS1) mRNA, nuclear gene encoding mitochondrial protein, complete cds.//2.7e-59:292:99//AF097441
R-HEMBB1001348//Homo sapiens clone DJ0691F11, WORKING DRAFT SEQUENCE, 11 unordered pieces.//9.1e-41:326:82//AC004859
R-HEMBB1001356//Human DNA sequence *** SEQUENCING IN PROGRESS *** from clone 424J12, WORKING DRAFT SEQUENCE.//1.8e-11:213:67//Z82207
R-HEMBB1001364//HS_3050_A2_F05_MR CIT Approved Human Genomic Sperm Library D Homo sapiens genomic clone Plate=3050 Col=10 Row=K, genomic survey sequence.//1.8e-21:158:91//AQ133940
R-HEMBB1001366//Homo sapiens chromosome 10 clone CIT987SK-1188I5 map 10p11.2-10p12.1, complete sequence.//4.1e-37:419:73//AC005876
R-HEMBB1001367//Human Chromosome 16 BAC clone CIT987SK-A-234F9, complete sequence.//9.5e-15:201:75//U91326
R-HEMBB1001369//Human DNA sequence *** SEQUENCING IN PROGRESS *** from clone 477J10, WORKING DRAFT SEQUENCE.//1.8e-28:224:83//AL021686
R-HEMBB1001380//HS_2267_B1_F11_MR CIT Approved Human Genomic Sperm Library D Homo sapiens genomic clone Plate=2267 Col=21 Row=L, genomic survey sequence.//4.0e-14:100:95//AQ084896
R-HEMBB1001384//Mus musculus COP9 complex subunit 4 (COPS4) mRNA, complete cds.//9.6e-55:312:81//AF071314
R-HEMBB1001387//Homo sapiens chromosome 9, P1 clone 8660 (LBNL H105), complete sequence.//1.0:166:63//AC003953
R-HEMBB1001394//Homo sapiens chromosome 17, clone hRPK.215_E_13, complete sequence.//1.4e-55:494:76//AC005549
R-HEMBB1001410//Homo sapiens PAC clone DJ1102B04 from 7q11.23-7q21, complete sequence.//0.011:208:63//AC006204
R-HEMBB1001424//Homo sapiens, WORKING DRAFT SEQUENCE, 76 unordered pieces.//1.5e-22:325:69//AC002370
R-HEMBB1001426//Homo sapiens 12q24 PAC RPCI3-424M6 (Roswell Park Cancer Institute Human PAC library) complete sequence.//1.3e-46:328:84//AC002350
R-HEMBB1001429//*** SEQUENCING IN PROGRESS *** Homo sapiens chromosome 4, BAC clone C0481P14; HTGS phase 1, WORKING DRAFT SEQUENCE, 7 unordered pieces.//6.6e-105:550:95//AC006160
R-HEMBB1001436
R-HEMBB1001443//HS_2228_A1_B05_MF CIT Approved Human Genomic Sperm Library D Homo sapiens genomic clone Plate=2228 Col=9 Row=C, genomic survey sequence.//0.37:173:62//AQ066934
R-HEMBB1001449//Homo sapiens clone DJ1129E22, WORKING DRAFT SEQUENCE, 7 unordered pieces.//2.7e-23:339:69//AC005522
R-HEMBB1001454//Homo sapiens chromosome 5, P1 clone 1307e8 (LBNL H60), complete sequence.//1.1e-39:299:84//AC005355
R-HEMBB1001458//Plasmodium falciparum chromosome 2, section 67 of 73 of the complete sequence.//6.0e-05:486:59//AE001430
R-HEMBB1001463//Homo sapiens PAC clone DJ0777O23 from 7p14-p15, complete sequence.//1.2e-50:317:89//AC005154
R-HEMBB1001464//CIT-HSP-2370C10.TF CIT-HSP Homo sapiens genomic clone 2370C10, genomic survey sequence.//0.20:95:71//AQ107941
R-HEMBB1001482//Mus musculus clone OST20235, genomic survey sequence.//4.3e-09:192:70//AF046762
R-HEMBB1001500//Human DNA sequence from PAC 465G10 on chromosome X contains Menkes Disease (ATP7A) putative Cu⁺⁺-transporting P-type ATPase exons 2 to 21, PGAM-B, ESTs.//1.9e-21:253:70//Z94801
R-HEMBB1001521//Mus musculus clone OST1209, genomic survey sequence.//7.5e-30:332:75//AF046642
R-HEMBB1001527//Homo sapiens clone DJ241P17, WORKING DRAFT SEQUENCE, 7 unordered pieces.//9.5e-55:483:76//AC005000
R-HEMBB1001531//Human BAC clone 7E17 from 12q, complete sequence.//1.3e-08:159:71//AC002070
R-HEMBB1001535//Human DNA sequence from cosmid E127C11 on chromosome 22q11.2-qter contains STS.//4.0e-30:286:79//Z74581
R-HEMBB1001536//Homo sapiens cosmid clone LUCA16 from 3p21.3, complete sequence.//1.6e-39:342:80//U73169
R-HEMBB1001537//Genomic sequence from Human 9q34, complete sequence.//3.7e-41:361:77//AC000394
R-HEMBB1001555//Homo sapiens Chromosome 16 BAC clone CIT987SK-A-485G10, complete sequence.//0.34:212:61//AC003049
R-HEMBB1001562//Homo sapiens Chromosome 16 BAC clone CIT987SK-A-328A3, complete sequence.//8.0e-40:267:88//AC002301
R-HEMBB1001564//Homo sapiens clone DJ0414A15, WORKING DRAFT SEQUENCE, 9 unordered pieces.//5.1e-30:286:76//AC005225
R-HEMBB1001565//Homo sapiens clone DJ0607J02, WORKING DRAFT SEQUENCE, 12 unordered pieces.//2.5e-15:194:75//AC004840
R-HEMBB1001585//Human DNA sequence from clone 790B6 on chromosome 20p11.22-12.2. Contains STSs and GSSs, complete sequence.//2.6e-33:234:79//AL031677
R-HEMBB1001586//Homo sapiens clone NH0479C13, WORKING DRAFT SEQUENCE, 12 unordered pieces.//2.7e-30:371:74//AC005236
R-HEMBB1001588//Homo sapiens Xp22 GS-524I1 (Genome Systems Human BAC library), complete sequence.//8.0e-32:323:73//AC003106
R-HEMBB1001603//Plasmodium falciparum DNA *** SEQUENCING IN PROGRESS *** from contig 4-59, complete sequence.//0.034:302:59//AL010235
R-HEMBB1001618//Homo sapiens DNA sequence from PAC 958B3 on chromosome Xp22.11-Xp22.22. Contains ESTs STS and OpG island.//7.1e-31:503:68//Z93023
R-HEMBB1001619//Homo sapiens Xq28 BAC PAC and cosmid clones containing FMR2 gene exons 1,2, and 3, complete sequence.//3.7e-50:539:72//AC002368
R-HEMBB1001630//Human DNA sequence from PAC 121G13 on chromosome 6 contains flow sorted chromosome 6 HindIII fragment ESTs. polymorphic CA repeat, CpG island, CpG island genomic fragments.//1.3e-27:228:82//Z86062
R-HEMBB1001635//Homo Sapiens Chromosome X clone bWXD90, complete sequence.//1.5e-23:407:69//AC004075
R-HEMBB1001637//Homo sapiens Xq28 BAC PAC and cosmid clones containing FMR2 gene exons 1,2, and 3, complete sequence.//3.9e-54:519:74//AC002368
R-HEMBB1001641//Human DNA sequence from clone 133H11 on chromosome 6p24. Contains STSs, GSSs and genomic marker D6S410, complete sequence.//1.9e-08:464:60//AL024506
R-HEMBB1001653//Homo sapiens chromosome 17, clone HCIT3L16, WORKING DRAFT SEQUENCE, 7 unordered pieces.//2.8e-39:318:82//AC002344
R-HEMBB1001665//***ALU WARNING: Human Alu-Sp subfamily consensus sequence.//3.8e-47:283:90//U14572
R-HEMBB1001668
R-HEMBB1001673//Homo sapiens mRNA for KIAA0646 protein, complete cds.//1.8e-115:573:97//AB014546
R-HEMBB1001684//Sequence 1 from patent US 5700927.//1.9e-40:343:77//I86429
R-HEMBB1001685//Homo sapiens chromosome 17, clone hRPK.721_K_1, complete sequence.//2.6e-43:31:83//AC005411
R-HEMBB1001695
R-HEMBB1001704//CIT-HSP-2324C15.TR CIT-HSP Homo sapiens genomic clone 2324C15, genomic survey sequence.//0.0074:259:58//AQ028704
R-HEMBB1001706//Homo sapiens clone DJ0665P05, WORKING DRAFT SEQUENCE, 5 unordered pieces.//9.1e-34:296:80//AC004851
R-HEMBB1001707//Homo sapiens Chromosome 16 BAC clone CIT987SK-A-319E8, complete sequence.//7.7e-32:241:76//AC004020
R-HEMBB1001717//CIT-HSP-2378C19.TF CIT-HSP Homo sapiens genomic clone 2378C19, genomic survey sequence.//4.8e-35:228:89//AQ108992
R-HEMBB1001735//Homo sapiens chromosome 5, BAC clone 114k9 (LBNL H94), complete sequence.//1.8e-10:80:90//AC005613
R-HEMBB1001736//CIT-HSP-2369K6.TF CIT-HSP Homo sapiens genomic clone 2369K6, genomic survey sequence.//9.9e-38:242:90//AQ075221
R-HEMBB1001747//Homo sapiens cosmids Qc14E2, Qc12H12, Qc11F9, Qc10G9, LA1733 and Qc17B8 from Xq28, complete sequence.//3.3e-60:366:80//U82671
R-HEMBB1001749//Homo sapiens chromosome 17, clone hRPK.259_G_18, complete sequence.//1.4e-60:242:92//AC005829
R-HEMBB1001753//RPCI11-59J22.TK RPCI11 Homo sapiens genomic clone R-59J22, genomic survey sequence.//6.2e-08:281:64//AQ200046
R-HEMBB1001756//Homo sapiens BAC clone RG293F17 from 7p15-p21, complete sequence.//3.1e-18:395:67//AC004130
R-HEMBB1001760//Homo sapiens genomic DNA, chromosome 21q11.1, segment 21/28, WORKING DRAFT SEQUENCE.//9.9e-18:416:64//AP000050
R-HEMBB1001762//Mus musculus major histocompatibility locus class II region: major histocompatibility protein class II alpha chain (IAalpha) and major histocompatibility protein class II beta chain (IEbeta) genes, complete cds; butyrophilin-like (NG9), butyrophilin-like (NG10), hypothetical protein (NG8), and butyrophilin-like (NG11) genes, partial cds; NG12 pseudogene, partial sequence; and hypothetical butyrophilin-like protein (NG13) gene, partial cds.//0.21:521:57//AF050157
R-HEMBB1001785//Torulopsis glabrata mitochondrial intergenic region ATPase 6 -ATPase 9 genes.//0.00073:189:65//X02170
R-HEMBB1001797//Plasmodium falciparum 3D7 chromosome 12 PFYACB8-420 genomic sequence, WORKING DRAFT SEQUENCE, 14 unordered pieces.//0.0049:322:62//AC005140
R-HEMBB1001802//Human desmin gene, complete cds.//8.1e-95:510:93//M63391
R-HEMBB1001812//Human DNA sequence *** SEQUENCING IN PROGRESS *** from clone 356B8, WORKING DRAFT SEQUENCE.//1.3e-71:368:96//Z98882
R-HEMBB1001816//Homo sapiens chromosome 21 PAC LLNLP704G1150Q13.//8.4e-21:164:76//AJ006996
R-HEMBB1001831//Homo sapiens PAM COOH-terminal interactor protein 1 (PCIP1) mRNA, complete cds.//1.7e-104:498:98//AF056209
R-HEMBB1001836//Homo sapiens chromosome 19, cosmid R26660, complete sequence.//9.2e-44:388:71//AC005328
R-HEMBB1001839
R-HEMBB1001850//Arabidopsis thaliana genomic DNA, chromosome 5, P1 clone: MOP10, complete sequence.//0.00093:488:60//AB005241
R-HEMBB1001863//Human poly(ADP-ribose) polymerase gene, 5' end.//1.2e-16:458:65//M60436
R-HEMBB1001867//Human DNA sequence from cosmid U25D11, between markers DXS366 and DXS87 on chromosome X.//5.0e-31:399:74//Z68327
R-HEMBB1001868//Arabidopsis thaliana genomic DNA, chromosome 5, P1 clone: MYN8, complete sequence.//0.26:303:59//AB020754
R-HEMBB1001869//Homo sapiens chromosome 17, clone hCIT529I10, complete sequence.//7.0e-37:285:85//AC002553
R-HEMBB1001872//Caenorhabditis elegans DNA *** SEQUENCING IN PROGRESS *** from clone Y44F5, WORKING DRAFT SEQUENCE.//0.093:367:58//AL009027
R-HEMBB1001874
R-HEMBB1001875//Lactococcus lactis DPC3147 plasmid pMRC01, complete plasmid sequence.//0.037:406:60//AE001272
R-HEMBB1001880//Homo sapiens chromosome 17, clone hRPK.235_I_10, complete sequence.//1.3e-49:461:77//AC005922
R-HEMBB1001899//Caenorhabditis elegans DNA *** SEQUENCING IN PROGRESS *** from clone Y116A8, WORKING DRAFT SEQUENCE.//0.56:295:60//Z98858
R-HEMBB1001905//Human DNA sequence *** SEQUENCING IN PROGRESS *** from clone Y738F9, WORKING DRAFT SEQUENCE.//1.9e-28:181:75//AL022345
R-HEMBB1001906
R-HEMBB1001908//Genomic sequence from Human 17, complete sequence.//2.9e-36:274:76//AC001231
R-HEMBB1001910//Homo sapiens chromosome 17, clone HCIT39G8, complete sequence.//3.5e-41:408:76//AC003070
R-HEMBB1001911//Homo sapiens *** SEQUENCING IN PROGRESS ***, WORKING DRAFT SEQUENCE.//6.1e-64:310:89//AJ011929
R-HEMBB1001915//Mouse mRNA for arylhydrocarbon receptor, complete cds.//2.0e-20:220:78//D38417
R-HEMBB1001921//Human DNA sequence *** SEQUENCING IN PROGRESS *** from clone 1141E15, WORKING DRAFT SEQUENCE.//1.9e-47:410:80//AL034422
R-HEMBB1001922//Homo sapiens chromosome 17, clone HCIT421K24, complete sequence.//6.2e-32:378:74//AC004099
R-HEMBB1001925//Human Chromosome 11 overlapping pacs pDJ235k10 and pDJ239b22, WORKING DRAFT SEQUENCE, 17 unordered pieces.//8.2e-41:304:84//AC000406
R-HEMBB1001930//Homo sapiens genomic DNA of 8p21.3-p22 anti-oncogene of hepatocellular colorectal and non-small cell lung cancer, segment 10/11.//8.3e-12:202:69//AB020867
R-HEMBB1001944//P.falciparum gene for beta subunit RNA polymerase.//0.00090:264:62//X75544
R-HEMBB1001945//Swietenia humilis DNA for simple tandem repeat (242bp).//0.056:224:62//AJ000408
R-HEMBB1001947//RPCI11-60L13.TJ RPCI11 Homo sapiens genomic clone R-60L13, genomic survey sequence.//7.4e-23:146:94//AQ202335
R-HEMBB1001950//Human DNA sequence from clone 415G2 on chromosome 22 Contains synapsin IIIa exon 1, EST and GSS, complete sequence.//0.57:115:68//Z83846
R-HEMBB1001952//Homo Sapiens Chromosome X clone bWXD171, WORKING DRAFT SEQUENCE, 1 ordered pieces.//5.6e-36:283:84//AC004676
R-HEMBB1001953//Homo sapiens clone NH0469M07, WORKING DRAFT SEQUENCE, 7 unordered pieces.//8.9e-60:334:82//AC005037
R-HEMBB1001957//Homo sapiens clone RG228D17, WORKING DRAFT SEQUENCE, 2 unordered pieces.//1.9e-56:518:77//AC005077
R-HEMBB1001962//Homo sapiens chromosome 16, BAC clone 462G18 (LANL), complete sequence.//3.2e-19:157:86//AC005736
R-HEMBB1001967//Homo sapiens DNA for amyloid precursor protein, complete cds.//5.7e-68:314:89//D87675
R-HEMBB1001973//Homo sapiens *** SEQUENCING IN PROGRESS *** from PAC E7.1 / cosmid 40M1, WORKING DRAFT SEQUENCE.//1.4e-37:484:70//AJ009617
R-HEMBBl001983//Human DNA sequence *** SEQUENCING IN PROGRESS *** from clone 215D11, WORKING DRAFT SEQUENCE.//2.1e-28:286:75//AL034417
R-HEMBB1001988//Human DNA sequence *** SEQUENCING IN PROGRESS *** from clone 1112F19, WORKING DRAFT SEQUENCE.//6.9e-29:203:88//AL034420
R-HEMBB1001990//Homo sapiens full-length insert cDNA clone ZC33G03.//7.8e-95:456:99//AF086192
R-HEMBB1001996
R-HEMBB1001997//Homo sapiens clone RG050N15, WORKING DRAFT SEQUENCE, 26 unordered pieces.//6.4e-26:162:83//AC005055
R-HEMBB1002002//Human DNA sequence from PAC 2A2 on chromosome X contains ESTs.//8.2e-83:362:93//Z84816
R-HEMBB1002005//Homo sapiens chromosome 3p clone RPCI5-1034C16, WORKING DRAFT SEQUENCE, 45 unordered pieces.//8.5e-36:291:83//AC005903
R-HEMBB1002009//Homo sapiens clone DJ0828F13, complete sequence.//5.6e-08:307:65//AC004904
R-HEMBB1002015//HS-1039-A1-C10-MF.abi CIT Human Genomic Sperm Library C Homo sapiens genomic clone Plate=CT 821 Col=19 Row=E, genomic survey sequence.//1.9e-05:375:62//B36336
R-HEMBB1002042//CIT-HSP-2313E13.TF CIT-HSP Homo sapiens genomic clone 2313E13, genomic survey sequence.//0.34:241:62//AQ028389
R-HEMBB1002043//Homo sapiens chromosome 21, P1 clone LBL#8 (LBNL H8), complete sequence.//7.4e-35:297:82//AC005612
R-HEMBB1002044//Homo sapiens chromosome 5p, BAC clone 50g21 (LBNL H154), complete sequence.//5.8e-96:582:90//AC005740
R-HEMBB1002045//Homo sapiens chromosome 19, cosmid F22676, complete sequence.//4.7e-63:575:77//AC005778
R-HEMBB1002049//Human Chromosome X clone bWXD187, complete sequence.1/1.9e-21:384:64//AC004383
R-HEMBB1002050//Homo sapiens chromosome 17, clone hRPK.112_J_9, complete sequence.//2.5e-37:368:76//AC005553
R-HEMBB1002068//Homo sapiens chromosome 5, BAC clone 205e20 (LBNL H170), complete sequence.//0.30:167:65//AC004782
R-HEMBB1002069//Homo sapiens chromosome 19, cosmid R33516, complete sequence.//2.3e-73:449:84//AC004799
R-HEMBB1002092//Homo sapiens chromosome 17, clone hRPK.269_G_24, complete sequence.//3.8e-45:307:87//AC005828
R-HEMBB1002094//Homo sapiens chromosome 19, cosmid R30538, complete sequence.//3.1e-47:457:76//AC005943
R-HEMBB1002115//HS_2223_B1_G10_MF CIT Approved Human Genomic Sperm Library D Homo sapiens genomic clone Plate=2223 Col=19 Row=N, genomic survey sequence.//3.0e-58:295:98//AQ152279
R-HEMBB1002139//***ALU WARNING: Human Alu-Sq subfamily consensus sequence.//6.6e-49:283:93//U14573
R-HEMBB1002142//Homo sapiens clone DJ0813F11, WORKING DRAFT SEQUENCE, 5 unordered pieces.//1.1e-45:451:76//AC006006
R-HEMBB1002152//Homo sapiens chromosome 10 clone CIT987SK-1079E16 map 10q25, complete sequence.//1.3e-57:359:81//AC005881
R-HEMBB1002189//Human Chromosome 11 pac pDJ392a17, complete sequence.//4.5e-43:420:77//AC000385
R-HEMBB1002190//Homo sapiens clone DJ0876A24, WORKING DRAFT SEQUENCE, 6 unordered pieces.//8.2e-33:340:64//AC004913
R-HEMBB1002193//Sequence 5 from patent US 5709858.//3.2e-23:154:92//I80846
R-HEMBB1002217//Homo sapiens clone HS19.2 Alu-Ya5 sequence.//2.6e-52:415:81//AF015148
R-HEMBB1002218//, complete sequence.//3.4e-17:178:82//AC005300
R-HEMBB1002232//*** SEQUENCING IN PROGRESS *** Homo sapiens chromosome 4, BAC clone C0052I22; HTGS phase 1, WORKING DRAFT SEQUENCE, 4 unordered pieces.//1.6e-55:292:88//AC004599
R-HEMBB1002247//Homo sapiens chromosome 17, clone hRPK.259_G_18, complete sequence.//2.9e-13:227:70//AC005829
R-HEMBB1002249//Human DNA sequence *** SEQUENCING IN PROGRESS *** from clone 455J7, WORKING DRAFT SEQUENCE.//1.1e-06:284:64//AL031733
R-HEMBB1002254//Human Chromosome X, WORKING DRAFT SEQUENCE, 6 unordered pieces.//6.3e-104:593:91//AC002415
R-HEMBB1002255//Human DNA sequence *** SEQUENCING IN PROGRESS *** from clone 292E10, WORKING DRAFT SEQUENCE.//2.1e-40:284:85//Z93930
R-HEMBB1002266//Plasmodium falciparum DNA *** SEQUENCING IN PROGRESS *** from contig 4-10, complete sequence.//1.3e-09:371:63//AL010216
R-HEMBB1002280//Homo sapiens PAC clone DJ0545C24 from 7q21-q22, complete sequence.//1.3e-39:247:86//AC004534
R-HEMBB1002300//Human Chromosome 11 Cosmid cSRL30h11, complete sequence.//4.1e-84:549:86//U73642
R-HEMBB1002306//Homo sapiens BAC clone RG136N17 from 7p15-p21, complete sequence.//2.5e-10:164:71//AC004129
R-HEMBB1002327//Homo sapiens BAC clone GS539F22 from 7p12-p14, complete sequence.//0.39:365:59//AC005028
R-HEMBB1002329//HS-1049-B1-D05-MR.abi CIT Human Genomic Sperm Library C Homo sapiens genomic clone Plate=CT 771 Col=9 Row=H, genomic survey sequence.//0.96:180:58//B39313
R-HEMBB1002340//Homo sapiens PAC clone DJ0659J06 from 7q33-q35, complete sequence.//7.9e-17:258:73//AC004849
R-HEMBB1002342//Homo sapiens mRNA for putative thioredoxin-like protein.//6.9e-96:479:97//AJ010841
R-HEMBB1002358//Human Xp22 BAC CT-285I15 (from CalTech/Research Genetics) , PAC RPCI1-27C22 (from Roswell Park Cancer Center), and Cosmid U35B5 (from Lawrence Livermore), complete sequence.//2.3e-53:309:83//AC002366
R-HEMBB1002359//Homo sapiens clone NH0486I22, WORKING DRAFT SEQUENCE, 5 unordered pieces.//4.9e-27:350:74//AC005038
R-HEMBB1002364//Homo sapiens Xp22 PAC RPCI1-108M6 (Roswell Park Cancer Center PAC library) complete sequence.//8.6e-53:302:79//AC003036
R-HEMBB1002371//Human gene for catalase (EC 1.11.1.6) exon 11 mapping to chromosome 11, band p13.//3.2e-38:199:100//X04094
R-HEMBB1002381//Homo sapiens (JH8) mRNA, partial cds.//3.2e-07:120:78//AF072467
R-HEMBB1002383//Human DNA sequence from cosmid U19H10 on chromosome X. Contains ESTs and CA repeat.//0.98:351:58//AL021182
R-HEMBB1002387//HS-1052-B2-G10-MR.abi CIT Human Genomic Sperm Library C Homo sapiens genomic clone Plate=CT 774 Col=20 Row=N, genomic survey sequence.//2.0e-07:276:67//B41091
R-HEMBB1002415//Homo sapiens chromosome 17, clone hRPK.209_D_14, complete sequence.//1.4e-25:202:79//AC005730
R-HEMBB1002425//Homo sapiens chromosome 19, cosmid R33516, complete sequence.//3.6e-60:401:87//AC004799
R-HEMBB1002442//Homo sapiens clone UWGC:r9a from 6p21, complete sequence.//3.1e-51:358:81//AC006046
R-HEMBB1002453//Human DNA sequence *** SEQUENCING IN PROGRESS *** from clone 86D1, WORKING DRAFT SEQUENCE.//1.4e-115:557:98//AL034349
R-HEMBB1002457//Human DNA sequence from clone 364I22 on chromosome Xq21.31-22.3. Contains an STS and GSSs, complete sequence.//6.3e-37:338:80//AL031012
R-HEMBB1002458//Homo sapiens T-cell receptor alpha delta locus from bases 250472 to 501670 (section 2 of 5) of the Complete Nucleotide Sequence.//9.7e-09:314:64//AE000659
R-HEMBB1002477//Arabidopsis thaliana DNA chromosome 4, BAC clone T12H17 (ESSAII project).//0.42:110:74//AL021635
R-HEMBB1002489//Salvelinus fontinalis microsatellite sequence SFO-12.//6.6e-06:167:71//U50302
R-HEMBB1002492//RPCI11-74F21.TK RPCI11 Homo sapiens genomic clone R-74F21, genomic survey sequence.//3.1e-14:410:63//AQ238960
R-HEMBB1002495//HS_3220_A2_F07_T7 CIT Approved Human Genomic Sperm Library D Homo sapiens genomic clone Plate=3220 Col=14 Row=K, genomic survey sequence.//1.3e-24:137:100//AQ180762
R-HEMBB1002502//Homo sapiens chromosome 17, clone hRPK.346_K_10, complete sequence.//9.6e-81:538:86//AC006120
R-HEMBB1002509//Human DNA sequence from clone 581F12 on chromosome Xq21. Contains Eukaryotic Translation Initiation Factor EIF3 P35 Subunit and 60S Ribosomal protein L22 pseudogenes. Contains ESTs, complete sequence.//0.0061:482:57//AL031313
R-HEMBB1002510//HS_2179_A1_F03_MR CIT Approved Human Genomic Sperm Library D Homo sapiens genomic clone Plate=2179 Col=5 Row=K, genomic survey sequence.//6.9e-35:423:72//AQ298309
R-HEMBB1002520//Human DNA sequence *** SEQUENCING IN PROGRESS *** from clone 27K12, WORKING DRAFT SEQUENCE.//2.0e-62:201:85//AL033397
R-HEMBB1002522//Homo sapiens chromosome 5, Pac clone 61c2 (LBNL H139), complete sequence.//0.99:323:58//AC004225
R-HEMBB1002531
R-HEMBB1002534//Homo sapiens genomic DNA, chromosome 21q22.2 (Down Syndrome region), segment 2/15, WORKING DRAFT SEQUENCE.//1.0e-61:380:79//AP000009
R-HEMBB1002545//RPCI11-2F3.TVB RPCI-11 Homo sapiens genomic clone RPCI-11-2F3, genomic survey sequence.//3.5e-12:414:63//B63283
R-HEMBB1002550
R-HEMBB1002556//*** SEQUENCING IN PROGRESS *** Homo sapiens chromosome 4, BAC clone C0481P14; HTGS phase 1, WORKING DRAFT SEQUENCE, 7 unordered pieces.//2.6e-62:299:85//AC006160
R-HEMBB1002579//Human DNA sequence *** SEQUENCING IN PROGRESS *** from clone 1141E15, WORKING DRAFT SEQUENCE.//1.7e-42:286:88//AL034422
R-HEMBB1002582//Homo sapiens clone DJ1119N05, complete sequence.//3.0e-14:426:60//AC004968
R-HEMBB1002590//Homo sapiens clone RG132J19, complete sequence.//1.1e-30:392:74//AC005163
R-HEMBB1002596//Human DNA sequence *** SEQUENCING IN PROGRESS *** from clone 508I15, WORKING DRAFT SEQUENCE.//8.5e-44:335:83//AL021707
R-HEMBB1002600//Homo sapiens 12p13.3 PAC RPCI5-1063M23 (Roswell Park Cancer Institute Human PAC Library) complete sequence.//2.0e-105:470:96//AC005865
R-HEMBB1002601//Homo sapiens chromosome 17, clone HRPC837J1, complete sequence.//1.3e-44:445:77//AC004223
R-HEMBB1002603//Homo sapiens clone UWGC:y23c049 from 6p21, complete sequence.//7.0e-40:321:82//AC006162
R-HEMBB1002607//CIT-HSP-2347D7.TF CIT-HSP Homo sapiens genomic clone 2347D7, genomic survey sequence.//1.1e-44:234:98//AQ060197
R-HEMBB1002610//Human Chromosome 16 BAC clone CIT987SK-A-363E6, complete sequence.//7.0e-22:455:65//U91321
R-HEMBB1002613//Homo sapiens 12p13.3 BAC RPCI11-476M19 (Roswell Park Cancer Institute Human BAC Library) complete sequence.//3.0e-72:302:85//AC005908
R-HEMBB1002614//Homo sapiens 12q13.1 PAC RPCI1-228P16 (Roswell Park Cancer Institute Human PAC Library) complete sequence.//3.8e-10:512:60//AC004801
R-HEMBB1002617//Homo sapiens clone DJ1021I20, WORKING DRAFT SEQUENCE, 6 unordered pieces.//6.8e-24:486:63//AC005520
R-HEMBB1002623//Homo sapiens PAC clone DJ1059M17 from 7q21-q31.1, complete sequence.//2.4e-41:326:83//AC004953
R-HEMBB1002635//Homo sapiens chromosome 12p13.3 clone RPCI11-189M20, WORKING DRAFT SEQUENCE, 39 unordered pieces.//2.6e-42:360:80//AC005910
R-HEMBB1002664//Homo sapiens chromosome 21q22.3 PAC 171F15, complete sequence.//9.1e-51:335:87//AF042090
R-HEMBB1002677//Plasmodium falciparum strain Dd2 heat shock protein 86 (HSP86), O1 (o1), O3 (o3), O2 (o2), CG8 (cg8), CG4 (cg4), CG3 (cg3), CG9 (cg9), CG1 (cg1), CG6 (cg6), chloroquine resistance candidate protein (cg2), and CG7 (cg7) genes, complete cds.//0.0011:399:59//AF030694
R-HEMBB1002683//Homo sapiens chromosome 21q22.3 PAC 171F15, complete sequence.//4.1e-55:515:76//AF042090
R-HEMBB1002684//Human BAC clone RG066D11 from 7q22, complete sequence.//1.7e-18:504:62//AC002430
R-HEMBB1002686//Homo sapiens full-length insert cDNA clone ZC65D06.//7.0e-85:413:99//AF086217
R-HEMBB1002692//Homo sapiens 12p13.3 BAC RPCI11-319E16 (Roswell Park Cancer Institute Human BAC Library) complete sequence.//9.8e-69:505:82//AC006206
R-HEMBB1002697//Plasmodium falciparum 3D7 chromosome 12 PFYAC812 genomic sequence, WORKING DRAFT SEQUENCE, 8 unordered pieces.//0.26:390:58//AC004153
R-HEMBB1002699//Human NFE genomic fragment.//8.0e-32:226:79//M98511
R-HEMBB1002702//CIT-HSP-344K23.TVC CIT-HSP Homo sapiens genomic clone 344K23, genomic survey sequence.//8.6e-43:351:8011859764
R-HEMBB1002705//Plasmodium yoelii rhoptry protein, complete cds.//0.0064:454:59//L27838
R-HEMBB1002712//Human DNA sequence from clone 505B13 on chromosome 1p36.2-36.3 Contains CA repeat and GSSs, complete sequence.//9.6e-09:187:67//Z98052
R-MAMMA1000009//Homo sapiens clone NH0469M07, WORKING DRAFT SEQUENCE, 7 unordered pieces.//4.1e-21:201:80//AC005037
R-MAMMA1000019//Homo sapiens chromosome 21q22.2 PAC clone P169K17, complete sequence.//4.2e-48:306:82//AF015720
R-MAMMA1000020//Human DNA sequence from clone 551E13 on chromosome Xp11.2-11.3 Contains farnesyl pyrophosphate synthetase pseudogene, VT4 protein pseudogene, EST, GSS, complete sequence.//1.4e-41:306:86//AL022163
R-MAMMA1000025//Human DNA sequence from clone 512B11 on chromosome 6p24-25. Contains the Desmoplakin I (DPI) gene, ESTs, STSs and GSSs, complete sequence.//6.1e-36:281:83//AL031058
R-MAMMA1000043//Homo sapiens Chromosome 22q11.2 Cosmid Clone 8c In DGCR Region, complete sequence.//1.3e-67:321:88//AC000090
R-MAMMA1000045//Homo sapiens chromosome 4 clone B220G8 map 4q21, complete sequence.//6.7e-86:559:86//AC004054
R-MAMMA1000055//Branta canadensis CA dinucleotide repeat locus Bcamicrol.//0.79:63:77//AF025889
R-MAMMA1000057//Homo sapiens DNA sequence from cosmid ICK0721Q on chromosome 6. Contains a 60S Ribosomal Protein L35A LIKE pseudogene, a gene coding for a 60S Ribosomal Protein L12 LIKE protein in an intron of the HSET gene coding for a Kinesin related protein, the PHF1 (PHF2) gene coding for alternative splice products PHD finger proteins 1 and 2, the gene coding for five different alternatively spliced mRNAs coding for a protein similar to CYTA (CYCY) and identical to a polypeptide coded for by a known patented cDNA, and the first two exons of the gene coding for the human homolog of the rat synaptic ras GTPase-activating protein p135 SynGAP. Contains three predicted CpG islands, ESTs and an STS, complete sequence.//1.6e-53:397:83//AL021366
R-MAMMA1000069//Homo sapiens clone RG052H06, WORKING DRAFT SEQUENCE, 11 unordered pieces.//2.0e-37:295:83//AC005057
R-MAMMA1000084//Homo sapiens chromosome Xp22-135-136 clone GSHB-567I1, WORKING DRAFT SEQUENCE, 35 unordered pieces.//7.1e-45:296:88//AC005867
R-MAMMA1000085
R-MAMMA1000092//Human DNA sequence *** SEQUENCING IN PROGRESS *** from clone 774G10, WORKING DRAFT SEQUENCE.//8.2e-34:539:69//AL034410
R-MAMMA1000103//Homo sapiens chromosome 17, clone hCIT.91_J_4, complete sequence.//3.4e-39:297:85//AC003976
R-MAMMA1000117//Homo sapiens p47-phox (NCF1) pseudogene, clone P38, exon 5.//2.6e-07:162:67//U69641
R-MAMMA1000129//Homo sapiens clone DJ076B20, WORKING DRAFT SEQUENCE, 6 unordered pieces.//6.1e-13:141:80//AC004882
R-MAMMA1000133
R-MAMMA1000134//Homo sapiens chromosome 19, cosmid R26660, complete sequence.//9.7e-18:171:80//AC005328
R-MAMMA1000139//Homo sapiens clone DJ241P17, WORKING DRAFT SEQUENCE, 7 unordered pieces.//1.2e-49:366:75//AC005000
R-MAMMA1000143//Homo sapiens *** SEQUENCING IN PROGRESS *** from PAC D9.2, WORKING DRAFT SEQUENCE.//3.9e-56:318:89//AJ009615
R-MAMMA1000155//Human DNA sequence from clone 323M22 on chromosome 22q13.1-13.2. Contains the 5' part of the human ortholog of chicken P52 and mouse H74, and a novel gene coding for a protein similar to KIAA0173 and worm Tubulin Tyrosine Ligase. Contains ESTs, STSs, GSSs, genomic marker D22S418 and putative CpG islands, complete sequence.//2.1e-68:562:78//AL022476
R-MAMMA1000163//Homo sapiens clone RG315H11, WORKING DRAFT SEQUENCE, 5 unordered pieces.//5.3e-06:408:58//AC005089
R-MAMMA1000171//CIT-HSP-2335L20.TR CIT-HSP Homo sapiens genomic clone 2335L20, genomic survey sequence.//1.5e-42:173:89//AQ037381
R-MAMMA1000173
R-MAMMA1000175//H.sapiens CpG island DNA genomic Mse1 fragment, clone 186c5, reverse read cpg186c5.rt1b.//0.072:90:72//Z57594
R-MAMMA1000183//Homo sapiens Xp22 BAC GSHB-184P14 (Genome Systems Human BAC library) complete sequence.//1.5e-44:445:75//AC004552
R-MAMMA1000198//Homo sapiens clone c102D0968, complete sequence.//1.9e-23:135:85//AF038667
R-MAMMA1000221//HS_3242_B2_H02_T7 CIT Approved Human Genomic Sperm Library D Homo sapiens genomic clone Plate=3242 Col=4 Row=P, genomic survey sequence.//0.031:167:67//AQ220385
R-MAMMA1000227//Human DNA sequence *** SEQUENCING IN PROGRESS *** from clone 1071N3, WORKING DRAFT SEQUENCE.//4.5e-36:487:71//AL031728
R-MAMMA1000241//Homo sapiens DNA sequence from PAC 93L7 on chromosome Xq21. Contains part of the CHM (TCD, REP1) gene coding for RAB Escort protein 1 (REP-1, RAB proteins geranylgeranyltransferase component A 1, Choroideraemia protein, Tapetochoroidal Dystrophy (TCD) protein). Contains ESTs and an STS, complete sequence.//6.2e-07:445:59//AL022401
R-MAMMA1000251//Homo sapiens chromosome 19, cosmid F23465, complete sequence.//1.6e-25:390:69//AC005266
R-MAMMA1000254//Homo sapiens DNA sequence from BAC 1216H12 on chromosome 22q12. Contains a pseudogene with similarity to part of mouse Ninein and the KIAA0609 gene for a protein similar to C. elegans K09C8.4. Contains ESTs, GSSs and a ggtt repeat polymorphism, complete sequence.//1.1e-37:327:80//AL008715
R-MAMMA1000257//Human DNA sequence *** SEQUENCING IN PROGRESS *** from clone 1125A11, WORKING DRAFT SEQUENCE.//1.3e-22:281:74//AL034549
R-MAMMA1000264//*** SEQUENCING IN PROGRESS *** EPM1/APECED region of chromosome 21, clones A68E8, B127P21, B173L3, B23N8, C1242C9, C579E2, A70B6, B159G9, B175D10, B52C10, C124G1 Note: Sequencing in this region has been discontinued by the Stanford Human Genome Center, WORKING DRAFT SEQUENCE, 50 unordered pieces.//1.7e-29:337:67//AC003656
R-MAMMA1000266//Human DNA sequence *** SEQUENCING IN PROGRESS *** from clone 681N20, WORKING DRAFT SEQUENCE.//7.7e-37:339:80//AL031670
R-MAMMA1000270//Human Chromosome 16 BAC clone CIT987SK-A-270G1, complete sequence.//1.2e-40:283:86//AF001549
R-MAMMA1000277//CIT-HSP-516K6.TP CIT-HSP Homo sapiens genomic clone 516K6, genomic survey sequence.//3.0e-29:265:80//B49900
R-MAMMA1000278//Sequence 25 from patent US 5708157.//2.6e-39:282:82//I80056
R-MAMMA1000279//Homo sapiens chromosome 16, cosmid clone 390H2 (LANL), complete sequence.//1.6e-52:295:84//AC004494
R-MAMMA1000284//CITBI-E1-2522B20.TF CITBI-E1 Homo sapiens genomic clone 2522B20, genomic survey sequence.//1.8e-11:288:61//AQ280722
R-MAMMA1000287
R-MAMMA1000302//Homo sapiens chromosome 17, clone hRPK.112_J_9, complete sequence.//4.1e-16:169:77//AC005553
R-MAMMA1000307//RPCI11-89L1.TV RPCI11 Homo sapiens genomic clone R-89L1, genomic survey sequence.//1.3e-86:429:97//AQ284795
R-MAMMA1000309//Homo sapiens hJAG2.del-E6 (JAG2) mRNA, alternatively spliced isoform of Jagged2, complete cds.//0.00020:384:60//AF029779
R-MAMMA1000312//Ichneutes sp. 16S ribosomal RNA gene, partial sequence.//0.0026:310:60//AF003518
R-MAMMA1000313//Human cosmid Xq28_IA649, complete sequence.//1.5e-26:317:67//U82694
R-MAMMA1000331//Homo sapiens clone DJ1007F24, WORKING DRAFT SEQUENCE, 5 unordered pieces.//3.1e-39:277:86//AC004947
R-MAMMA1000339//Homo sapiens clone HS19.1 Alu-Ya5 sequence.//3.2e-44:180:89//AF015147
R-MAMMA1000340//Plasmodium falciparum chromosome 2, section 25 of 73 of the complete sequence.//0.97:293:64//AE001388
R-MAMMA1000348//Homo sapiens BAC129, complete sequence.//4.4e-27:365:72//U85195
R-MAMMA1000356//Drosophila melanogaster DNA sequence (P1 DS02252 (D97)), complete sequence.//0.73:332:61//AC002493
R-MAMMA1000360//Homo sapiens PAC clone DJ0905J08 from 7p12-p14, complete sequence.//4.6e-80:279:89//AC005189
R-MAMMA1000361//Human DNA sequence *** SEQUENCING IN PROGRESS *** from clone 753D4, WORKING DRAFT SEQUENCE.//7.8e-18:346:63//AL031676
R-MAMMA1000372//Human DNA sequence *** SEQUENCING IN PROGRESS *** from clone Y214H10, WORKING DRAFT SEQUENCE.//5.3e-40:299:83//AL022344
R-MAMMA1000385//Human DNA sequence *** SEQUENCING IN PROGRESS *** from clone 310013, WORKING DRAFT SEQUENCE.//1.0e-28:225:84//AL031658
R-MAMMA1000388//CIT-HSP-2321D3.TR CIT-HSP Homo sapiens genomic clone 2321D3, genomic survey sequence.//4.7e-60:298:99//AQ038102
R-MAMMA1000395
R-MAMMA1000402//Homo sapiens PAC clone DJ1107K12 from 7p12-p14, complete sequence.//1.4e-84:276:88//AC004692
R-MAMMA1000410//Human Chromosome 16 BAC clone CIT987SK-A-211C6, complete sequence.//6.7e-35:360:76//AC002394
R-MAMMA1000413//Homo sapiens chromosome 17, clone hRPC.842_A_23, complete sequence.//3.1e-69:327:79//AC004662
R-MAMMA1000414//Homo sapiens DNA sequence from PAC 164L12 on chromosome Xq13.1-Xq21.2. Contains GSS (BAC end sequence),STS.//3.6e-41:180:87//AL009028
R-MAMMA1000416//Homo sapiens clone DJ1136G02, WORKING DRAFT SEQUENCE, 4 unordered pieces.//3.1e-59:478:77//AC005377
R-MAMMA1000421//Human coxVIb gene, last exon and flanking sequence.//5.3e-53:294:82//X58139
R-MAMMA1000422//Human DNA sequence *** SEQUENCING IN PROGRESS *** from clone 8B22, WORKING DRAFT SEQUENCE.//1.0:252:59//AL031737
R-MAMMA1000423//Homo sapiens clone DA0065G23, complete sequence.//2.0e-50:491:76//AC004816
R-MAMMA1000424//Human DNA sequence from PAC 507I15 on chromosome Xq26.3-27.3. Contains 60S ribosomal protein L44 (L41, L36) like gene, ESTs, STSs and a polymorphic CA repeat.//3.5e-40:340:80//Z98950
R-MAMMA1000429//Mus musculus SDP8 mRNA, complete cds.//0.0019:87:79//AF062484
R-MAMMA1000431//Homo sapiens clone DJ0098O22, WORKING DRAFT SEQUENCE, 5 unordered pieces.//2.0e-58:564:77//AC004821
R-MAMMA1000444//Human BAC clone RG126M09 from 7q21-q22, complete sequence.//3.0e-43:328:83//AC002067
R-MAMMA1000446//Human chromosome X clone Qc15B1, complete sequence.//0.95:209:65//U82672
R-MAMMA1000458//Arabidopsis thaliana genomic DNA, chromosome 5, P1 clone: MXK3, complete sequence.//0.99:182:61//AB019236
R-MAMMA1000468
R-MAMMA1000472//Homo sapiens genomic DNA, 21q region, clone: 655M9N34, genomic survey sequence.//1.0e-38:142:88//AG010148
R-MAMMA1000478//Human DNA sequence *** SEQUENCING IN PROGRESS *** from clone 169I5, WORKING DRAFT SEQUENCE.//1.3e-37:286:83//Z93015
R-MAMMA1000483//CIT-HSP-384B14.TR CIT-HSP Homo sapiens genomic clone 384B14, genomic survey sequence.//4.3e-34:158:86//B54637
R-MAMMA1000490//Homo sapiens chromosome 19, BAC CIT-B-191n6, complete sequence.//4.2e-98:569:90//AC006130
R-MAMMA1000500//Human BRCA1, Rho7 and vatI genes, complete cds, and ipf35 gene, partial cds.//1.2e-41:334:79//L78833
R-MAMMA1000501//Human DNA sequence *** SEQUENCING IN PROGRESS *** from clone 153G14, WORKING DRAFT SEQUENCE.//1.4e-38:250:84//AL031118
R-MAMMA1000516//Human DNA sequence *** SEQUENCING IN PROGRESS *** from clone 424J12, WORKING DRAFT SEQUENCE.//1.3e-43:318:83//Z82207
R-MAMMA1000522//Human DNA sequence from clone 739H11 on chromosome 1p33-34.2 Contains KIAA0237 gene, EST, STS, GSS, complete sequence.//4.4e-13:202:73//AL031289
R-MAMMA1000559//Human DNA sequence *** SEQUENCING IN PROGRESS *** from clone 169I5, WORKING DRAFT SEQUENCE.//2.2e-30:245:83//Z93015
R-MAMMA1000565//Homo sapiens chromosome 10 clone LA10NC01_183_B_7 map 10q24, WORKING DRAFT SEQUENCE, 1 ordered pieces.//3.6e-39:281:80//U82205
R-MAMMA1000567//Rattus norvegicus nonmuscle caldesmon mRNA, complete cds.//9.2e-19:216:76//U18419
R-MAMMA1000576
R-MAMMA1000583//Homo sapiens chromosome 17, clone hRPK.112_H_10, complete sequence.//5.4e-53:297:85//AC005666
R-MAMMA1000585//Homo sapiens clone DJ1015P16, WORKING DRAFT SEQUENCE, 4 unordered pieces.//1.2e-35:450:71//AC006018
R-MAMMA1000594//Homo sapiens *** SEQUENCING IN PROGRESS *** from cosmid 5L5, WORKING DRAFT SEQUENCE.//4.3e-26:293:75//AJ009613
R-MAMMA1000597//CIT-HSP-2341F4.TF CIT-HSP Homo sapiens genomic clone 2341F4, genomic survey sequence.//0.83:110:70//AQ057131
R-MAMMA1000605//Homo sapiens clone DJ1090E20, WORKING DRAFT SEQUENCE, 4 unordered pieces.//2.6e-50:290:86//AC004956
R-MAMMA1000612//CIT-HSP-2334J18.TF CIT-HSP Homo sapiens genomic clone 2334J18, genomic survey sequence.//0.76:132:65//AQ038364
R-MAMMA1000616//Ibalia leucospoides mitochondrion 16S rRNA gene, partial sequence.//6.8e-06:431:59//U06970
R-MAMMA1000621//Human NBR2 mRNA, complete cds.//5.3e-27:258:80//U88573
R-MAMMA1000623
R-MAMMA1000625//Homo sapiens chromosome 19, cosmid R31665, complete sequence.//3.3e-07:325:63//AC005498
R-MAMMA1000643//Human DNA sequence *** SEQUENCING IN PROGRESS *** from clone 39B17, WORKING DRAFT SEQUENCE.//1.4e-06:236:68//AL023656
R-MAMMA1000664//*** SEQUENCING IN PROGRESS *** Homo sapiens chromosome 4, BAC clone C0326F06; HTGS phase 1, WORKING DRAFT SEQUENCE, 16 unordered pieces.//1.4e-40:338:81//AC004555
R-MAMMA1000669//Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.//1.2e-46:327:86//AL021578
R-MAMMA1000670
R-MAMMA1000672//Human DNA sequence from clone 478D8 on chromosome 6p24. Contains STSs and GSSs, complete sequence.//2.2e-29:328:76//AL031785
R-MAMMA1000684//Mus musculus frizzled-1 mRNA, complete cds.//0.21:247:63//AF054623
R-MAMMA1000696//Human Chromosome X clone bWXD173, WORKING DRAFT SEQUENCE, 2 ordered pieces.//2.7e-46:464:71//AC004387
R-MAMMA1000707//Homo sapiens clone RG219E16, WORKING DRAFT SEQUENCE, 3 unordered pieces.//3.4e-09:244:66//AC005075
R-MAMMA1000713//Homo sapiens clone DJ0425I02, WORKING DRAFT SEQUENCE, 5 unordered pieces.//3.7e-51:439:74//AC005478
R-MAMMA1000714//Homo sapiens BAC clone RG152H24 from 7p15-p21, complete sequence.//2.8e-29:288:75//AC004694
R-MAMMA1000718//Human Xp22 BAC CT-285I15 (from CalTech/Research Genetics) , PAC RPCI1-27C22 (from Roswell Park Cancer Center), and Cosmid U35B5 (from Lawrence Livermore), complete sequence.//3.0e-37:231:91//AC002366
R-MAMMA1000720//Homo sapiens chromosome 19, cosmid R33632, complete sequence.//1.4e-35:299:81//AC005781
R-MAMMA1000723//Human DNA sequence from clone 551E13 on chromosome Xp11.2-11.3 Contains farnesyl pyrophosphate synthetase pseudogene, VT4 protein pseudogene, EST, GSS, complete sequence.//3.9e-59:409:79//AL022163
R-MAMMA1000731//Homo sapiens clone RG228D17, WORKING DRAFT SEQUENCE, 2 unordered pieces.//9.4e-29:560:66//AC005077
R-MAMMA1000732//Homo sapiens clone DJ0539M06, WORKING DRAFT SEQUENCE, 10 unordered pieces.//2.4e-14:309:68//AC004832
R-MAMMA1000733//Human DNA sequence *** SEQUENCING IN PROGRESS *** from clone 732E4, WORKING DRAFT SEQUENCE.//4.1e-29:377:71//AL008722
R-MAMMA1000734//Human DNA sequence *** SEQUENCING IN PROGRESS *** from clone 191J18, WORKING DRAFT SEQUENCE.//2.0e-108:420:99//AL024507
R-MAMMA1000738//Human V beta T-cell receptor (TCRBV) gene locus.//6.6e-41:347:82//U03115
R-MAMMA1000744//T27O8-T7 TAMU Arabidopsis thaliana genomic clone T27O8, genomic survey sequence.//0.095:367:60//B20150
R-MAMMA1000746//*** SEQUENCING IN PROGRESS *** Homo sapiens chromosome 4, BAC clone C0135005; HTGS phase 1, WORKING DRAFT SEQUENCE, 23 unordered pieces.//7.4e-95:569:87//AC004661
R-MAMMA1000752//Homo sapiens BAC clone BK085E05 from 22q12.1-qter, complete sequense.//1.3e-48:295:84//AC003071
R-MAMMA1000760//Human DNA sequence from clone B79B4 on chromosome 22 Contains CA repeat and GSS, complete sequence.//5.7e-45:347:82//Z82178
R-MAMMA1000761//Homo sapiens cosmid clone LUCA16 from 3p21.3, complete sequence.//1.1e-32:292:80//U73169
R-MAMMA1000775//Homo sapiens chromosome 17, clone hRPK.22_N_12, WORKING DRAFT SEQUENCE, 2 ordered pieces.//2.5e-50:467:79//AC005412
R-MAMMA1000776//Human BAC clone GS552A01 from 7q21-q22, complete sequence.//1.0e-63:429:79//AC002454
R-MAMMA1000778//Human DNA sequence from 4PTEL, Huntington's Disease Region, chromosome 4p16.3.//3.5e-25:234:81//Z95704
R-MAMMA1000782//Human DNA sequence from clone 459L4 on chromosome 6p22.3-24.1 Contains EST, STS, GSS, complete sequence.//0.0021:119:74//AL031120
R-MAMMA1000798//Homo sapiens 959 kb contig between AML1 and CBR1 on chromosome 21q22, segment 2/3.//6.3e-08:269:64//AJ229042
R-MAMMA1000802//Homo sapiens chromosome 19, cosmid R33729, complete sequence.//1.1e-36:261:80//AC005339
R-MAMMA1000831//CIT-HSP-2387J3.TF.1 CIT-HSP Homo sapiens genomic clone 2387J3, genomic survey sequence.//0.68:156:65//AQ240807
R-MAMMA1000839//Homo sapiens chromosome 17, clone hRPK.726_O_12, WORKING DRAFT SEQUENCE, 6 unordered pieces.//4.6e-50:335:86//AC005517
R-MAMMA1000841//Human Chromosome 16 BAC clone CIT987SK-A-972D3, complete sequence.//1.3e-40:322:77//U91323
R-MAMMA1000842//Human DNA sequence *** SEQUENCING IN PROGRESS *** from clone 341D10, WORKING DRAFT SEQUENCE.//4.1e-44:471:74//Z97985
R-MAMMA1000843//Homo sapiens clone 82F9, WORKING DRAFT SEQUENCE, 4 unordered pieces.//0.85:394:60//AC004815
R-MAMMA1000845//Plasmodium falciparum DNA *** SEQUENCING IN PROGRESS *** from MAL1P1, WORKING DRAFT SEQUENCE.//0.54:303:63//AL031744
R-MAMMA1000851//Homo sapiens chromosome X, MeCP2 locus, complete sequence.//1.7e-10:115:83//AF030876
R-MAMMA1000855//Homo sapiens PAC clone 278C19 from 12q, complete sequence.//5.0e-44:352:83//AC004263
R-MAMMA1000856//Homo sapiens chromosome 19, cosmid F24200, complete sequence.//1.8e-10:149:74//AC00461
R-MAMMA1000862//Hepatitis C virus genomic RNA, 3' nonstranslated region, partial sequence. clone #16.//8.1e-05:205:66//AF009075
R-MAMMA1000863//Homo sapiens Xp22 Cosmids U15E4, U115H5, U132E12, U115B9 (Lawrence Livermore human cosmid library) complete sequence.//2.9e-49:421:80//AC002364
R-MAMMA1000865//Homo sapiens Chromosome 16 BAC clone CIT987SK-A-328A3, complete sequence.//9.1e-41:302:83//AC002301
R-MAMMA1000867//Human BRCA1, Rho7 and vatI genes, complete cds, and ipf35 gene, partial cds.//1.9e-17:500:61//L78833
R-MAMMA1000875//Homo sapiens chromosome 16, cosmid clone RT99 (LANL), complete sequenced.//1.2e-17:211:74//AC004653
R-MAMMA1000876//Homo sapiens Xp22 BAC GS-607H18 (Genome Systems Human BAC library) complete sequence.//4.7e-09:160:65//AC003658
R-MAMMA1000877//Homo sapiens DNA sequence from PAC 958B3 on chromosome Xp22.11-Xp22.22. Contains ESTs STS and CpG island.//3.2e-34:354:75//Z93023
R-MAMMA1000880//Homo sapiens Chromosome 16 BAC clone CIT987SK-A-575C2, complete sequence.//1.4e-41:411:74//AC002425
R-MAMMA1000883
R-MAMMA1000897
R-MAMMA1000905//Homo sapiens chromosome 5, P1 clone 274A11 (LBNL H66), complete sequence.//1.3e-73:304:91//AC004506
R-MAMMA1000906//Human DNA from chromosome 19-specific cosmid F14150, genomic sequence, complete sequence.//8.4e-23:194:83//AC003110
R-MAMMA1000908//Human Chromosome 15q26.1 PAC clone pDJ416i6, complete sequence.//1.5e-09:170:71//AC003024
R-MAMMA1000914//Homo sapiens PAC clone DJ0740L10 from 7p13-p14, complete sequence.//8.3e-13:323:67//AC005247
R-MAMMA1000921//Human DNA sequence *** SEQUENCING IN PROGRESS *** from clone 423B22, WORKING DRAFT SEQUENCE.//6.8e-28:333:72//AL034379
R-MAMMA1000931//HS_3227_B1_B03_T7 CIT Approved Human Genomic Sperm Library D Homo sapiens genomic clone Plate=3227 Col=5 Row=D, genomic survey sequence.//1.4e-55:443:79//AQ191777
R-MAMMA1000940//Homo sapiens clone RG013F03, WORKING DRAFT SEQUENCE, 6 unordered pieces.//2.0e-43:340:84//AC005046
R-MAMMA1000941//Homo sapiens chromosome 17, clone 297N7, complete sequence.//1.8e-53:330:84//AC002347
R-MAMMA1000942//Human Chromosome X clone bWXD187, complete sequence.//1.2e-39:391:74//AC004383
R-MAMMA1000943//Human PAC clone DJ327A19 from Xq25-q26, complete sequence.//4.6e-75:566:81//AC002477
R-MAMMA1000956//Plasmodium falciparum MAL3P7, complete sequence.//0.013:285:59//AL034559
R-MAMMA1000957//Homo sapiens clone RG339C12, WORKING DRAFT SEQUENCE, 10 unordered pieces.//5.2e-45:288:90//AC005096
R-MAMMA1000962//Homo sapiens clone DJ0756H11, WORKING DRAFT SEQUENCE, 5 unordered pieces.//2.9e-108:561:96//AC006001
R-MAMMA1000968//Homo sapiens PAC clone 278C19 from 12q, complete sequence.//3.9e-41:287:87//AC004263
R-MAMMA1000975//Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence.//9.4e-65:542:79//Z95152
R-MAMMA1000979//Homo sapiens chromosome 21q22.3, PAC clones 314N7, 225L15, BAC clone 7B7, complete sequence bases 1..333303.//3.2e-34:296:80//AJ011930
R-MAMMA1000987//Homo sapiens CC chemokine gene cluster, complete sequence.//1.7e-40:255:87//AF088219
R-MAMMA1000998//Homo sapiens PAC clone DJ1152D16 from Xq23, complete sequence.//2.5e-39:315:73//AC005190
R-MAMMA1001003//Homo sapiens chromosome 10 clone CIT-HSP-1338F24 map 10p11.2-10p12.1, complete sequence.//2.4e-52:296:84//AC006101
R-MAMMA1001008//Homo sapiens *** SEQUENCING IN PROGRESS ***, WORKING DRAFT SEQUENCE.//7.9e-88:432:98//AJ011929
R-MAMMA1001021//Homo sapiens PAC clone DJ0859M06 from 7q11, complete sequence.//3.8e-39:286:87//AC004910
R-MAMMA1001024//Homo sapiens clone DJ0876A24, WORKING DRAFT SEQUENCE, 6 unordered pieces.//2.0e-31:274:80//AC004913
R-MAMMA1001030//Homo sapiens full-length insert cDNA clone ZD96C01.//3.2e-99:469:99//AF088074
R-MAMMA1001035//RPCI-1-46G8Sp6 RPCI-1 Homo sapiens genomic clone RPCI-1-46G8Sp6, genomic survey sequence.//3.5e-49:270:90//AQ275285
R-MAMMA1001038//Homo sapiens chromosome 3, olfactory receptor pseudogene cluster 1, complete sequence, and myosin light chain kinase (MLCK) pseudogene, partial sequence.//1.1e-41:285:87//AF042089
R-nnnnnnnnnnnn
R-MAMMA1001050//Homo sapiens genomic DNA, 237 kb segment from 6p21.3 region including HLA genes, WORKING DRAFT SEQUENCE.//1.3e-55:334:91//D84394
R-MAMMA1001059//Mouse RNA helicase and RNA-dependent ATPase from the DEAD box family mRNA, complete cds.//1.7e-51:481:77//L25125
R-MAMMA1001067//CIT-HSP-2371K20.TF CIT-HSP Homo sapiens genomic clone 2371K20, genomic survey sequence.//7.2e-65:946:95//AQ111326
R-MAMMA1001073
R-MAMMA1001074//Homo sapiens BAC clone NH0400O10 from Y, complete sequence.//8.6e-33:457:69//AC006040
R-MAMMA1001075//Homo sapiens Chromosome 16 BAC clone CIT987SK-A-248F7, complete sequence.//0.15:325:62//AC004605
R-MAMMA1001078//Homo sapiens chromosome 5, BAC clone 203o13 (LBNL H155), complete sequence.//1.6e-45:344:84//AC005609
R-MAMMA1001082//Human genomic DNA sequence from clone 308O1 on chromosome Xp11.3-11.4. Contains EST, CA repeat, STS, GSS, CpG island.//8.5e-15:413:64//Z93403
R-MAMMA1001091//Sequence 7 from patent US 5468610.//0.0027:159:64//I15499
R-MAMMA1001092//Homo sapiens chromosome 17, clone hRPK.372_K_20, complete sequence.//2.0e-51:267:82//AC005951
R-MAMMA1001105//Homo sapiens DNA sequence from PAC 119E23 on chromosome Xq25-q27.1. Contains glypican-3 precursor (intestinal protein OCI-5) (GTR2-2),5'UTR. ESTs, STS.//6.9e-22:178:85//Z99570
R-MAMMA1001110//Homo sapiens chromosome 17, clone HRPC1169K15, complete sequence.//3.0e-19:141:81//AC003963
R-MAMMA1001126//Human DNA from overlapping chromosome 7 PAC and P1 clones containing the XRCC2 gene, genomic sequence, complete sequence.//2.2e-46:462:75//AC003109
R-MAMMA1001133//Human DNA sequence *** SEQUENCING IN PROGRESS *** from clone 120G22, WORKING DRAFT SEQUENCE.//1.8e-68:455:86//AL031847
R-MAMMA1001139//Human DNA sequence *** SEQUENCING IN PROGRESS *** from clone Y738F9, WORKING DRAFT SEQUENCE.//7.1e-09:100:84//AL022345
R-MAMMA1001143//Papio hamadryas lipoprotein lipase (LPL) gene, intron 7.//1.9e-49:362:85//U73684
R-MAMMA1001145//Homo sapiens chromosome 17, clone hRPK.235_I_10, complete sequence.//9.5e-49:512:74//AC005922
R-MAMMA1001154//Homo sapiens Chromosome 16 BAC clone CIT987-SKA-88D1 ∼complete genomic sequence, complete sequence.//1.5e-29:305:76//AC002289
R-MAMMA1001161//Human DNA sequence from clone 681J21 on chromosome 1q23.2-24.3 Contains CpG island, complete sequence.//1.1e-64:339:90//AL031286
R-MAMMA1001162//Human DNA from cosmid DNA MMDB (f10080) and MMDC (f13544) from chromosome 19q13.3 (obtained by automated sequence analysis).//3.4e-09:243:64//M89651
R-MAMMA1001181//Human Chromosome X clone bWXD173, WORKING DRAFT SEQUENCE, 2 ordered pieces.//3.7e-29:351:74//AC004387
R-MAMMA1001186//Homo sapiens chromosome 19, cosmid R28778, complete sequence.//2.2e-25:415:68//AC006125
R-MAMMA1001191//Homo sapiens T-cell receptor alpha delta locus from bases 1000498 to 1071650 (section 5 of 5) of the Complete Nucleotide Sequence.//0.99:243:61//AE000662
R-MAMMA1001198//Mus musculus eps15R mRNA, complete cds.//8.0e-57:223:86//U29156
R-MAMMA1001202//Mus musculus clone OST13722, genomic survey sequence.//1.0e-30:220:85//AF046748
R-MAMMA1001203//Homo sapiens chromosome 17, clone hRPK.22_N_12, WORKING DRAFT SEQUENCE, 2 ordered pieces.//8.9e-61:567:78//AC005412
R-MAMMA1001206//Homo sapiens chromosome 5, P1 clone 854b11 (LBNL H44), complete sequence.//4.6e-08:442:61//AC004763
R-MAMMA1001215//Homo sapiens chromosome 19, CIT-HSP BAC 470n8, complete sequence.//1.3e-117:564:97//AC005393
R-MAMMA1001220//HS-1023-A1-G10-MR.abi CIT Human Genomic Sperm Library C Homo sapiens genomic clone Plate=CT 802 Col=19 Row=M, genomic survey sequence.//6.0e-16:276:68//B33708
R-MAMMA1001222//F17E12TFB IGF Arabidopsis thaliana genomic clone F17E12, genomic survey sequence.//0.041:277:61//B97762
R-MAMMA1001243
R-MAMMA1001244//HS-1058-A2-G01-MF.abi CIT Human Genomic Sperm Library C Homo-sapiens genomic clone Plate=CT 780 Col=2 Row=M, genomic survey sequence.//3.5e-05:104:74//B43862
R-MAMMA1001249//H.sapiens DNA for matrix attachment region.//0.0013:95:75//Z54221
R-MAMMA1001256//Human BAC clone GS188P18, complete sequence.//3.4e-32:356:74//AC000115
R-MAMMA1001259
R-MAMMA1001260//Homo sapiens mRNA for KIAA0661 protein, complete cds.//6.3e-20:226:75//AB014561
R-MAMMA1001268//Human DNA sequence from PAC 225D2 on chromosome Xq21. Contains ESTs, CA repeat.//1.1e-47:352:85//Z95124
R-MAMMA1001271
R-MAMMA1001274//H.sapiens DNA for trapped exon (ID HMC07C06), genomic survey sequence.//3.1e-40:232:93//X88457
R-MAMMA1001280//Homo sapiens full-length insert cDNA clone YW26C09.//1.9e-112:574:95//AF087976
R-MAMMA1001292//Human DNA sequence from clone 1170K4 on chromosome 22q12.2-13.1. Contains three novel genes, one of which codes for a Trypsin family protein with class A LDL receptor domains, and the IL2RB gene for Interleukin 2 Receptor, Beta (IL-2 Receptor, CD122 antigen). Contains a putative CpG island, ESTs, and GSSs, complete sequence.//2.9e-114:582:96//AL022314
R-MAMMA1001296//Human DNA sequence from PAC 487J7 on chromosome 6q21-22.1. Contains an unknown gene coding for three alternative mRNAs. Contains ESTs, STSs, a BAC end-sequence (GSS) and a CA repeat polymorphism.//1.9e-64:268:88//AL008730
R-MAMMA1001298//Homo sapiens chromosome 17, clone hRPK.849_N_15, complete sequence.//1.5e-38:306:83//AC005703
R-MAMMA1001305//Human DNA sequence from PAC 127B20 on chromosome 22q11.2-qter, contains gene for GTPase-activating protein similar to rhoGAP protein. ribosomal protein L6 pseudogene, ESTs and CA repeat.//1.5e-37:306:82//Z83838
R-MAMMA1001322//Homo sapiens DNA sequence from PAC 434O14 on chromosome 1q32.3.-41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two novel genes. Contains ESTs and GSSs, complete sequence.//2.4e-15:260:71//AL022398
R-MAMMA1001324//Homo sapiens chromosome 19, cosmid F23269, complete sequence.//4.0e-06:90:83//AC005614
R-MAMMA1001330//Human BAC clone RG066D11 from 7q22, complete sequence.//1.4e-45:439:74//AC002430
R-MAMMA1001341//Human DNA sequence from PAC 211D12 on chromosome 20q12-13.2. Contains Krs-2, K+ channel protein, stress responsive.//1.3e-24:137:81//Z93016
R-MAMMA1001343//Human Chromosome 16 BAC clone CIT987SK-A-17E1, complete sequence.//5.4e-51:197:89//AC002041
R-MAMMA1001346//Homo sapiens Chromosome 16 BAC clone CIT987SK-A-233A8, complete sequence.//0.99:182:64//AC004685
R-MAMMA1001383//Homo sapiens clone 82F9, WORKING DRAFT SEQUENCE, 4 unordered pieces.//1.9e-42:303:86//AC004815
R-MAMMA1001388//Human DNA sequence *** SEQUENCING IN PROGRESS *** from clone 508I15, WORKING DRAFT SEQUENCE.//1.5e-44:324:83//AL021707
R-MAMMA1001397//Homo sapiens genomic DNA, chromosome 21q11.1, segment 15/28, WORKING DRAFT SEQUENCE.//2.0e-39:254:89//AP000044
R-MAMMA1001408//Homo sapiens chromosome 12q24.1, WORKING DRAFT SEQUENCE, 33 unordered pieces.//9.4e-36:251:88//AC005805
R-MAMMA1001411//T15F1-T7.1 TAMU Arabidopsis thaliana genomic clone T15F1, genomic survey sequence.//1.0:98:71//AQ248928
R-MAMMA1001419//Homo sapiens translation initiation factor 4e mRNA, complete cds.//4.8e-18:117:96//AF038957
R-MAMMA1001420//Homo sapiens chromosome 5, P1 clone 1041F10 (LBNL H88), complete sequence.//2.8e-09:377:63//AC005179
R-MAMMA1001435//S.pombe chromosome I cosmid c26H5.//1.0:356:59//Z99126
R-MAMMA1001442//Homo sapiens chromosome 4 clone B150J4 map 4q25, complete sequence.//3.4e-17:259:72//AC004047
R-MAMMA1001446//Homo sapiens BAC clone RG139P11 from 7q11-q21, complete sequence.//2.9e-17:231:71//AC004491
R-MAMMA1001452//Human DNA sequence from clone 452M16 on chromosome Xq21.1-21.33 Contains capping protein alpha subunit isoform 1 pseudogene, STS, GSS, and CA repeat, complete sequence.//6.1e-50:558:73//AL024493
R-MAMMA1001465//cSRL-2F3-u cSRL flow sorted Chromosome 11 specific cosmid Homo sapiens genomic clone cSRL-2F3, genomic survey sequence.//3.0e-23:141:96//B04295
R-MAMMA1001476//Mus musculus uridine kinase mRNA, partial cds.//3.4e-09:309:64//L31783
R-MAMMA1001487//Homo sapiens chromosome 17, clone hRPC.1108_L_11, complete sequence.//5.1e-30:286:79//AC005206
R-MAMMA1001501
R-MAMMA1001502//Human DNA sequence *** SEQUENCING IN PROGRESS *** from clone 356B7, WORKING DRAFT SEQUENCE.//4.3e-19:349:64//AL031714
R-MAMMA1001510
R-MAMMA1001522//Homo sapiens chromosome 5, BAC clone 24h24 (LBNL H194), complete sequence.//1.5e-09:136:75//AC005352
R-MAMMA1001547//Human Chromosome X, complete sequence.//3.5e-40:300:84//AC002418
R-MAMMA1001551//Human DNA sequence from PAC 426I6 on chromosome 1p34.1-1p35. Contains NIPP-1-like gene a nuclear inhibitor of protein phosphatase-1, ESTs, and a CA repeat.//1.1e-57:282:89//AL020997
R-MAMMA1001575
R-MAMMA1001576//Human gamma-tubulin mRNA, complete cds.//7.6e-60:530:78//M61764
R-MAMMA1001590//Homo sapiens Bruton's tyrosine kinase (BTK), alpha-D-galactosidase A (GLA), L44-like ribosomal protein (L44L) and FTP3 (FTP3) genes, complete cds.//1.3e-29:161:86//U78027
R-MAMMA1001600//Homo sapiens 12q24 PAC RPCI1-66E7 (Roswell Park Cancer Institute Human PAC library) complete sequence.//2.1e-18:390:66//AC004216
R-MAMMA1001604//Human DNA sequence from clone 1042K10 on chromosome 22q13.1-13.2. Contains the ADSL gene for Adenylosuccinate lyase (EC 4.3.2.2, Adenylosuccinase, ASL) and 4 novel genes (one with probable rabGAP domains and Src homology domain 3). Contains ESTs, STSs, GSSs and a putative CpG island, complete sequence.//1.0:227:62//AL022238
R-MAMMA1001606//Human DNA sequence *** SEQUENCING IN PROGRESS *** from clone 228H13, WORKING DRAFT SEQUENCE.//1.3e-17:219:69//AL031985
R-MAMMA1001620//Human DNA sequence *** SEQUENCING IN PROGRESS *** from clone 1018D12, WORKING DRAFT SEQUENCE.//2.1e-51:298:84//AL031650
R-MAMMA1001627//Human DNA sequence *** SEQUENCING IN PROGRESS *** from clone 229A8, WORKING DRAFT SEQUENCE.//7.8e-45:328:85//Z86090
R-MAMMA1001630//, complete sequence.//2.5e-08:170:72//AC005399
R-MAMMA1001633//Homo sapiens chromosome 10 clone CIT987SK-1057L21 map 10q25, complete sequence.//2.2e-21:241:70//AC005386
R-MAMMA1001635//Homo sapiens DNA sequence from PAC 230G1 on chromosome Xp11.3. Contains EST, STS and GSS, complete sequence.//1.1e-32:346:74//Z84466
R-MAMMA1001649
R-MAMMA1001663//Homo sapiens clone 162B15, complete sequence.//9.4e-68:267:89//AC004811
R-MAMMA1001670//Human DNA sequence from PAC 75N13 on chromosome Xq21.1. Contains ZNF6 like gene, ESTs, STSs and CpG islands.//1.7e-49:322:88//Z82216
R-MAMMA1001671//Homo sapiens chromosome 19, cosmid F23269, complete sequence.//2.4e-114:575:96//AC005614
R-MAMMA1001679//CIT-HSP-2335N4.TF CIT-HSP Homo sapiens genomic clone 2335N4, genomic survey sequence.//2.4e-82:400:99//AQ037393
R-MAMMA1001683//Homo sapiens Chromosome 7 BAC Clone 239c10, WORKING DRAFT SEQUENCE, 9 unordered pieces.//5.7e-47:533:72//AC004166
R-MAMMA1001686//Homo sapiens chromosome 19, CIT-HSP-444n24, complete sequence.//6.6e-12:194:72//AC005261
R-MAMMA1001692//Human DNA sequence *** SEQUENCING IN PROGRESS *** from clone Y738F9, WORKING DRAFT SEQUENCE.//9.6e-44:414:77//AL022345
R-MAMMA1001711//Homo sapiens clone BAC 9H13 chromosome 8 map 8q21, complete sequence.//3.1e-31:436:70//AF110324
R-MAMMA1001715//Human DNA sequence *** SEQUENCING IN PROGRESS *** from clone 73E16, WORKING DRAFT SEQUENCE.//8.8e-76:524:84//Z95330
R-MAMMA1001730
R-MAMMA1001735//Cricetulus griseus (chinese hamster) mRNA for beta tubulin (clone B9T), partial.//2.7e-13:382:63//X60786
R-MAMMA1001740//Homo sapiens genomic DNA, chromosome 21q11.1, segment 21/28, WORKING DRAFT SEQUENCE.//3.9e-47:318:87//AP000050
R-MAMMA1001743//Homo sapiens clone DJ0981O07, complete sequence.//4.0e-108:566:95//AC006017
R-MAMMA1001744
R-MAMMA1001745//Homo sapiens BAC clone 529F11 from 8q21, complete sequence.//3.5e-113:564:97//AF070718
R-MAMMA1001751//Homo sapiens chromosome 19, cosmid R27328, complete sequence.//3.6e-30:312:75//AC005625
R-MAMMA1001754//Bos taurus vacuolar proton pump subunit SFD alpha isoform (SFD) mRNA, complete cds.//4.7e-34:320:77//AF041338
R-MAMMA1001757//Homo sapiens chromosome 17, clone hRPC.4_G_17, complete sequence.//4.7e-10:244:67//AC003688
R-MAMMA1001760//RPCI11-38L16.TV RPCI-11 Homo sapiens genomic clone RPCI-11-38L16, genomic survey sequence.//1.3e-10:236:64//AQ029432
R-MAMMA1001764//Plasmodium falciparum 3D7 chromosome 12 PFYACB8-420 genomic sequence, WORKING DRAFT SEQUENCE, 14 unordered pieces.//0.74:361:60//AC005140
R-MAMMA1001768//Homo sapiens chromosome 17, clone hRPK.147_L_13, complete sequence.//1.6e-42:416:76//AC005332
R-MAMMA1001769//Homo sapiens chromosome 17, clone hRPC.1073_F_15, complete sequence.//1.4e-13:129:83//AC004686
R-MAMMA1001771//M.musculus mRNA for semaphorin B.//1.1e-34:530:69//X85991
R-MAMMA1001783//Homo sapiens Chromosome 2 BAC Clone 376a1, WORKING DRAFT SEQUENCE, 17 unordered pieces.//1.1e-42:282:85//AC000360
R-MAMMA1001785//Human chromosome 16p13.11 BAC clone CIT987SK-98H8 complete sequence.//3.0e-49:282:86//U91319
R-MAMMA1001788
R-MAMMA1001790//Homo sapiens clone DJ0876A24, WORKING DRAFT SEQUENCE, 6 unordered pieces.//9.8e-43:530:71//AC004913
R-MAMMA1001806//Homo sapiens Chromosome 16 BAC clone CIT987SK-A-319E8, complete sequence.//1.8e-43:324:79//AC004020
R-MAMMA1001812//Plasmodium falciparum chromosome 2, section 69 of 73 of the complete sequence.//0.65:183:63//AE001432
R-MAMMA1001815//Homo sapiens clone GS223D04, WORKING DRAFT SEQUENCE, 3 unordered pieces.//1.1e-10:417:62//AC005018
R-MAMMA1001817//Homo sapiens Xp22-83 BAC GSHB-324M7 (Genome Systems Human BAC Library) complete sequence.//2.6e-40:313:84//AC005859
R-MAMMA1001818
R-MAMMA1001820//Homo sapiens, WORKING DRAFT SEQUENCE, 52 unordered pieces.//2.2e-45:340:82//AC004086
R-MAMMA1001824//Homo sapiens clone DJ1107K15, WORKING DRAFT SEQUENCE, 8 unordered pieces.//1.9e-53:291:85//AC004966
R-MAMMA1001836//HS_3164_B1_A02_MR CIT Approved Human Genomic Sperm Library D Homo sapiens genomic clone Plate=3164 Col=3 Row=B, genomic survey sequence.//6.5e-08:79:89//AQ185484
R-MAMMA1001837//Homo sapiens chromosome 19, overlapping cosmids F18547, F11133, R27945, R28830 and R32804, complete sequence.//8.4e-55:309:85//AC003682
R-MAMMA1001848//Homo sapiens PAC clone DJ0296G17 from Xq23, complete sequence.//1.6e-16:125:90//AC006144
R-MAMMA1001851//Genomic sequence from Human 9q34, WORKING DRAFT SEQUENCE, 2 unordered pieces.//2.4e-50:516:74//AC002099
R-MAMMA1001854//Homo sapiens Chromosome 16 BAC clone CIT987SK-A-575C2, complete sequence.//1.7e-38:308:82//AC002425
R-MAMMA1001858//Human Xq13 3' end of PAC 92E23 containing the X inactivation transcipt (XIST) gene, complete sequence.//6.5e-50:283:86//U80460
R-MAMMA1001864//Human Chromosome 15q26.1 PAC clone pDJ398g19, WORKING DRAFT SEQUENCE, 21 unordered pieces.//3.4e-36:224:86//AC005143
R-nnnnnnnnnnnn//Plasmodium falciparum chromosome 2, section 54 of 73 of the complete sequence.//1.4e-11:495:63//AE001417
R-MAMMA1001874//Human chromosome 1 BAC 308G1 genomic sequence, WORKING DRAFT SEQUENCE, 3 unordered pieces.//3.2e-42:446:76//AC003117
R-MAMMA1001878//Human DNA sequence from PAC 431A14 on chromosome 6p21. Contains CYCLOPHILIN (PEPTIDYLPROLYL ISOMERASE) like and CIP1 (WAF1, CDKNA1, CDKN1, MDA-6, SDI1, PIC1, CAP20) genes. Contains probable GTPase and receptor genes and ESTs, STSs and CpG islands.//6.9e-44:391:78//Z85996
R-MAMMA1001880//Human DNA sequence from fosmid F77D12 on chromosome 22q12-qter contains ESTs, tRNA.//1.3e-15:181:76//Z82097
R-MAMMA1001890//Homo sapiens Chromosome 16 BAC clone CIT987-SKA-670B5 ∼complete genomic sequence, complete sequence.//1.7e-43:283:86//AC002303
R-MAMMA1001907//Human DNA sequence *** SEQUENCING IN PROGRESS *** from clone 385E7, WORKING DRAFT SEQUENCE.//1.4e-48:420:79//AL031720
R-nnnnnnnnnnnn//Saccharomyces cerevisiae chromosome IV cosmid 9481.//2.9e-14:505:60//U28373
R-MAMMA1001931//Homo sapiens NACP/alpha-synuclein gene, allele A0, intron 4, partial sequence.//0.51:162:63//AF041008
R-MAMMA1001956//Human DNA sequence *** SEQUENCING IN PROGRESS *** from clone 50O24, WORKING DRAFT SEQUENCE.//1.4e-51:422:79//AL034380
R-MAMMA1001963//Homo sapiens clone HS19.3 Alu-Ya5 sequence.//1.9e-31:163:91//AF015149
R-MAMMA1001969//Human DNA from chromosome 19 cosmid F19410, genomic sequence, complete sequence.//8.7e-10:186:76//AC002128
R-MAMMA1001970//Homo sapiens BAC clone BK085E05 from 22q12.1-qter, complete sequence.//1.0e-62:298:86//AC003071
R-MAMMA1001992//Human Chromosome 15q26.1 PAC clone pDJ460g16, WORKING DRAFT SEQUENCE, 3 unordered pieces.//1.8e-44:525:72//AC004581
R-MAMMA1002009//Human DNA sequence *** SEQUENCING IN PROGRESS *** from clone 109G6, WORKING DRAFT SEQUENCE.//1.4e-43:282:79//AL023879
R-MAMMA1002011
R-MAMMA1002032//Human DNA sequence *** SEQUENCING IN PROGRESS *** from clone 469D22, WORKING DRAFT SEQUENCE.//1.1e-39:310:84//AL031284
R-MAMMA1002033//Homo sapiens chromosome 5, Pac clone 162o17 (LBNL H147), complete sequence.//2.5e-17:170:81//AC003954
R-MAMMA1002041//Homo sapiens PAC clone DJ0728D04, complete sequence.//8.7e-79:296:85//AC004865
R-MAMMA1002042//Human chromosome 16 BAC clone CIT987SK-A-962B4, complete sequence.//8.8e-46:386:80//U91318
R-MAMMA1002047//Human chromosome 16 BAC clone CIT987SK-A-962B4, complete sequence.//1.9e-32:326:75//U91318
R-MAMMA1002056//Homo sapiens chromosome 17, clone hRPK.506_H_21, complete sequence.//6.6e-48:367:82//AC005962
R-MAMMA1002058//Homo sapiens clone RG038K21, WORKING DRAFT SEQUENCE, 3 unordered pieces.//0.25:139:69//AC005052
R-MAMMA1002068//Homo Sapiens Chromosome X clone bWXD171, WORKING DRAFT SEQUENCE, 1 ordered pieces.//2.2e-45:406:78//AC004676
R-MAMMA1002078//Homo sapiens chromosome 17, clone hRPK.401_O_9, complete sequence.//2.3e-22:357:64//AC005291
R-MAMMA1002082//Homo sapiens PAC clone 278C19 from 12q, complete sequence.//2.5e-38:304:82//AC004263
R-MAMMA1002084//Human DNA sequence *** SEQUENCING IN PROGRESS *** from clone 1174N9, WORKING DRAFT SEQUENCE.//8.9e-41:319:83//AL031602
R-MAMMA1002093//CIT-HSP-2060J9.TF CIT-HSP Homo sapiens genomic clone 2060J9, genomic survey sequence.//9.7e-17:129:88//B69983
R-MAMMA1002108
R-MAMMA1002118//Human DNA sequence from cosmid E116C6, on chromosome 22 Contains ESTs, complete sequence.//0.94:168:64//Z73495
R-MAMMA1002125//Homo sapiens chromosome 17, clone hRPK.63_A_1, complete sequence.//4.8e-40:313:83//AC005670
R-MAMMA1002132//Homo sapiens PAC clone DJ1059M17 from 7q21-q31.1, complete sequence.//2.0e-70:461:83//AC004953
R-MAMMA1002140//Human DNA sequence from PAC 465G10 on chromosome X contains Menkes Disease (ATP7A) putative Cu⁺⁺-transporting P-type ATPase exons 2 to 21, PGAM-B, ESTs.//1.1e-32:477:73//Z94801
R-MAMMA1002143//Homo sapiens platelet-activating factor acetylhydrolase gene, promoter region and exon 1.//6.6e-06:130:73//AF027357
R-MAMMA1002145//Human DNA sequence *** SEQUENCING IN PROGRESS *** from clone 126A5, WORKING DRAFT SEQUENCE.//6.0e-19:242:73//AL031447
R-MAMMA1002153//*** SEQUENCING IN PROGRESS *** Homo sapiens chromosome 4, BAC clone C0281M17; HTGS phase 1, WORKING DRAFT SEQUENCE, 3 unordered pieces.//2.1e-51:291:75//AC006052
R-MAMMA1002155//Human DNA sequence *** SEQUENCING IN PROGRESS *** from clone 608E8, WORKING DRAFT SEQUENCE.//1.2e-53:461:79//AL022343
R-MAMMA1002156//Homo sapiens PAC clone DJ130H16 from 22q12.1-qter, complete sequence.//5.1e-37:305:82//AC004997
R-MAMMA1002158//Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.//8.1e-34:296:81//AL034418
R-MAMMA1002170//Human DNA sequence from clone 1163J1 on chromosome 22q13.2-13.33. Contains the 3' part of a gene for the ortholog of mouse transmembrane receptor Celsr1, a novel gene for a protein similar to C. elegans B0035.16 and bacterial tRNA (5-Methylaminomethyl-2-thiouridylate)-Methyltransferases, and the 3' part of a novel gene for a protein similar to mouse B99. Contains ESTs, GSSs and putative CpG islands, complete sequence.//7.9e-39:332:82//AL031588
R-MAMMA1002174//Homo sapiens chromosome 10 clone CIT987SK-1109P11, complete sequence.//4.4e-12:189:72//AC005871
R-MAMMA1002198//Homo sapiens clone DJ0800G07, complete sequence.//1.1e-48:338:81//AC004890
R-MAMMA1002209//Homo sapiens chromosome 17, clone hRPK.156_L_14, complete sequence.//1.2e-23:269:74//AC005821
R-MAMMA1002215//Homo sapiens clone GS250N06, WORKING DRAFT SEQUENCE, 5 unordered pieces.//3.2e-12:243:68//AC005158
R-MAMMA1002219//Homo sapiens 12p13.3 RPCI4-773N5 (Roswell Park Cancer Institute Human PAC library) complete sequence.//3.3e-45:295:88//AC004802
R-MAMMA1002230//Human DNA sequence *** SEQUENCING IN PROGRESS *** from clone 423B22, WORKING DRAFT SEQUENCE.//7.3e-41:385:78//AL034379
R-MAMMA1002236//Rattus norvegicus initiation factor eIF-2B gamma subunit (eIF-2B gamma) mRNA, complete cds.//7.3e-45:363:79//U38253
R-MAMMA1002243//Homo sapiens chromosome 17, clone hRPK.112_H_10, complete sequence.//2.8e-119:582:98//AC005666
R-MAMMA1002250//Homo sapiens chromosome 16, P1 clone 109-9G (LANL), complete sequence.//4.7e-42:319:84//AC005600
R-MAMMA1002267//Homo sapiens chromosome 17, clone hRPK.346_K_10, complete sequence.//1.5e-33:571:67//AC006120
R-MAMMA1002268//Mus musculus sphingosine kinase (SPHK1b) mRNA, complete cds.//2.3e-35:462:70//AF068749
R-MAMMA1002269//345I17.TV CIT978SKA1 Homo sapiens genomic clone A-345I17, genomic survey sequence.//4.7e-05:153:69//B15590
R-MAMMA1002282//Human DNA sequence *** SEQUENCING IN PROGRESS *** from clone 112K5, WORKING DRAFT SEQUENCE.//8.5e-37:467:71//Z85987
R-MAMMA1002292//Hordeum vulgare lipoxygenase 2 (LoxC) mRNA, complete cds.//0.074:178:61//L37358
R-MAMMA1002293//Homo sapiens chromosome 16, cosmid clone RT167 (LANL), complete sequence.//5.8e-26:355:71//AC005568
R-MAMMA1002294//Homo sapiens chromosome 17, clone hRPC.1110_E_20, complete sequence.//1.2e-35:281:82//AC004231
R-MAMMA1002297//Human DNA sequence from cosmid L174G8, Huntington's Disease Region, chromosome 4p16.3.//6.7e-48:381:80//Z69375
R-MAMMA1002298//Homo sapiens BAC clone RG208H19 from 7q11.23, complete sequence.//.8e-17:296:70//AC005074
R-MAMMA1002299//HS_3116_A2_F07_T7 CIT Approved Human Genomic Sperm Library D Homo sapiens genomic clone Plate=3116 Col=14 Row=K, genomic survey sequence.//4.1e-60:354:91//AQ140526
R-MAMMA1002308
R-MAMMA1002310//Human DNA sequence from cosmid B10B1 on chromosome 22 Contains ESTs, CA repeat and STS, complete sequence.//9.9e-35:283:83//Z73979
R-MAMMA1002311//Homo sapiens Xp22-150 BAC GSHB-309P15 (Genome Systems Human BAC Library) complete sequence.//1.3e-86:503:90//AC006210
R-MAMMA1002312//H.sapiens gene encoding La autoantigen.//1.3e-23:382:67//X97869
R-MAMMA1002317//Human DNA sequence from clone 48G12 on chromosome Xq27.1-27.3. Contains STSs and GSSs, complete sequence.//1.3e-59:323:87//AL031054
R-MAMMA1002319//Homo sapiens chromosome 19, fosmid 39347, complete sequence.//2.2e-106:522:98//AC005756
R-MAMMA1002322//Homo sapiens genomic DNA, chromosome 21q11.1, segment 13/28, WORKING DRAFT SEQUENCE.//2.3e-48:452:76//AP000042
R-MAMMA1002329//M.musculus mRNA for semaphorin B.//2.0e-12:210:73//X85991
R-MAMMA1002332//Homo sapiens PAC clone DJ1139I01 from Xq23, complete sequence.//3.4e-46:393:71//AC004973
R-MAMMA1002333//HS_3245_A1_B04_MR CIT Approved Human Genomic Sperm Library D Homo sapiens genomic clone Plate=3245 Col=7 Row=C, genomic survey sequence.//3.1e-21:146:92//AQ205759
R-MAMMA1002339//Human Chromosome 16 BAC clone CIT987SK-A-270G1, complete sequence.//9.7e-39:310:79//AF001549
R-MAMMA1002347//Homo sapiens 12q24.1 PAC RPCI3-305I20 (Roswell Park Cancer Institute Human PAC Library) complete sequence.//1.2e-46:443:76//AC006088
R-MAMMA1002351//Human DNA sequence *** SEQUENCING IN PROGRESS *** from clone 1059H15, WORKING DRAFT SEQUENCE.//1.1e-90:553:89//AL022100
R-MAMMA1002352//Homo sapiens mRNA for leukemia associated gene 2.//8.8e-81:388:92//Y15228
R-MAMMA1002353//Homo sapiens 12q24 BAC RPCI11-162P23 (Roswell Park Cancer Institute Human BAC library) complete sequence.//5.5e-35:302:80//AC002996
R-MAMMA1002355//Human DNA sequence *** SEQUENCING IN PROGRESS *** from clone 222E13, WORKING DRAFT SEQUENCE.//5.4e-52:361:76//Z93241
R-MAMMA1002356//Homo sapiens chromosome 17, clone hRPC.842_A_23, complete sequence.//8.3e-28:187:91//AC004662
R-MAMMA1002359//Human DNA sequence from cosmid L118D5, Huntington's Disease Region, chromosome 4p16.3 contains CpG islands.//6.3e-47:297:85//268869
R-MAMMA1002360//HS_2163_B2_C08_MR CIT Approved Human Genomic Sperm Library D Homo sapiens genomic clone Plate=2163 Col=16 Row=F, genomic survey sequence.//1.5e-20:374:66//AQ125213
R-MAMMA1002361//Human DNA sequence *** SEQUENCING IN PROGRESS *** from clone 349A12, WORKING DRAFT SEQUENCE.//2.2e-35:264:85//AL033520
R-MAMMA1002362//H.sapiens PEX gene.//1.8e-40:243:86//Y10196
R-MAMMA1002380//RPCI11-73J4.TJ RPCI11 Homo sapiens genomic clone R-73J4, genomic survey sequence.//1.7e-38:295:77//AQ268168
R-MAMMA1002384//Homo sapiens 12q13.1 PAC RPCI1-228P16 (Roswell Park Cancer Institute Human PAC Library) complete sequence.//2.5e-37:311:81//AC004801
R-MAMMA1002385
R-MAMMA1002392//Human BAC clone RG066D11 from 7q22, complete sequence.//2.0e-37:365:77//AC002430
R-MAMMA1002411//Human DNA sequence *** SEQUENCING IN PROGRESS *** from clone 64K7, WORKING DRAFT SEQUENCE.//9.4e-22:496:65//AL031668
R-MAMMA1002413//Homo sapient 12q24.2 PAC RPCI1-157K6 (Roswell Park Cancer Institute Human PAC library) complete sequence.//2.3e-15:153:77//AC005146
R-MAMMA1002417//Human DNA sequence from PAC 426I6 on chromosome 1p34.1-1p35. Contains NIPP-1-like gene a nuclear inhibitor of protein phosphatase-1, ESTs, and a CA repeat.//1.8e-23:508:62//AL020997
R-MAMMA1002427//Human Chromosome 16 BAC clone CIT987SK-A-363E6, complete sequence.//2.5e-37:288:84//U91321
R-MAMMA1002428//Human DNA sequence *** SEQUENCING IN PROGRESS *** from clone 1185N5, WORKING DRAFT SEQUENCE.//6.0e-05:130:75//AL034423
R-MAMMA1002434//Homo sapiens DNA sequence from PAC 380E11 on chromosome 6p22.3-p24. Contains HB15 gene, ESTs, CA repeat, STS and GSS.//4.8e-18:205:78//AL022396
R-MAMMA1002446//CIT-HSP-2021L14.TR CIT-HSP Homo sapiens genomic clone 2021L14, genomic survey sequence.//4.6e-41:387:72//B65379
R-MAMMA1002454//Homo sapiens chromosome 19, cosmid F23259, complete sequence.//1.2e-67:491:82//AC005512
R-MAMMA1002461//Homo sapiens PAC clone 166H1 from 12q, complete sequence.//1.4e-28:188:85//AC003982
R-MAMMA1002470//Saccharomyces cerevisiae chromosome VIII cosmid 9205.//6.3e-09:280:61//U10556
R-MAMMA1002475//Human DNA sequence from PAC 306D1 on chromosome X contains ESTs.//1.5e-25:310:74//Z83822
R-MAMMA1002480//Homo sapiens clone RG228D17, WORKING DRAFT SEQUENCE, 2 unordered pieces.//1.2e-98:533:93//AC005077
R-MAMMA1002485//Homo sapiens stanniocalcin-2 (STC-2) mRNA, complete cds.//2.7e-114:560:97//AF055460
R-MAMMA1002494//Human DNA sequence from cosmid L174G8, Huntington's Disease Region, chromosome 4p16.3.//2.1e-46:329:84//Z69375
R-MAMMA1002498//Rat mRNA.//0.0068:223:64//M59859
R-MAMMA1002524//Plasmodium falciparum 3D7 chromosome 12 PFYAC336 genomic sequence, WORKING DRAFT SEQUENCE, 5 unordered pieces.//0.012:460:60//AC005139
R-MAMMA1002530//Homo sapiens cytosolic phospholipase A2 gamma (cPLA2 gamma) mRNA, complete cds.//1.2e-101:529:95//AF065214
R-MAMMA1002545//Homo sapiens ribosomal protein s4 Y isoform gene, complete cds.//6.6e-50:471:77//AF041427
R-MAMMA1002554//Homo sapiens chromosome 4 clone B227H22 map 4q25, complete sequence.//5.7e-38:279:84//AC004056
R-MAMMA1002556//Homo sapiens chromosome 10 clone CIT-HSP-1255F20 map 10p11.2-10p12.1, complete sequence.//9.6e-13:237:67//AC005878
R-MAMMA1002566//CITBI-E1-2509P21.TR CITBI-E1 Homo sapiens genomic clone 2509P21, genomic survey sequence.//9.7e-14:216:73//AQ261427
R-MAMMA1002571//CITBI-E1-2516L21.TF CITBI-E1 Homo sapiens genomic clone 2516L21, genomic survey sequence.//4.6e-25:142:99//AQ279542
R-MAMMA1002573//Human DNA sequence *** SEQUENCING IN PROGRESS *** from clone 811H13, WORKING DRAFT SEQUENCE.//1.1e-30:250:82//AL023805
R-MAMMA1002585//Rabbit angiotensin-converting enzyme (ACE) gene, 5' end.//1.0:196:61//M58580
R-MAMMA1002590//H.sapiens CpG island DNA genomic Mse1 fragment, clone 8d5, forward read cpg8d5.f1g.//1.0:114:64//Z63758
R-MAMMA1002597//Human DNA sequence *** SEQUENCING IN PROGRESS *** from clone 1103G7, WORKING DRAFT SEQUENCE.//9.0e-96:459:98//AL034548
R-MAMMA1002598//Human DNA sequence *** SEQUENCING IN PROGRESS *** from clone 120G22, WORKING DRAFTSEQUENCE.//0.79:362:58//AL031847
R-MAMMA1002603//Homo sapiens chromosome 17, clone hRPK.214_C_8, complete sequence.//1.3e-46:333:80//AC005803
R-MAMMA1002612//Human DNA sequence *** SEQUENCING IN PROGRESS *** from clone 269M15, WORKING DRAFT SEQUENCE.//7.4e-41:283:86//AL021395
R-MAMMA1002617//Human DNA sequence *** SEQUENCING IN PROGRESS *** from clone 591N18, WORKING DRAFT SEQUENCE.//1.7e-20:308:71//AL031594
R-MAMMA1002618//Homo sapiens clone RG122E10, complete sequence.//1.2e-31:230:76//AC005067
R-MAMMA1002619//Homo sapiens chromosome 21 PAC RPCIP704E14135Q2.//9.0e-113:551:98//AJ010598
R-MAMMA1002622//Homo sapiens chromosome 4 clone B207D4 map 4q25, complete sequence.//2.8e-43:324:83//AC004050
R-MAMMA1002623//Homo sapiens chromosome 17, clone hRPC.1171_I_10, complete sequence.//2.7e-80:344:84//AC004687
R-MAMMA1002625//Human DNA sequence *** SEQUENCING IN PROGRESS *** from clone 1056L3, WORKING DRAFT SEQUENCE.//2.6e-34:391:72//AL031727
R-MAMMA1002629//Human DNA from overlapping chromosome 19-specific cosmids R32543,, and F15613 containing ZNF gene family member, genomic sequence, complete sequence.//5.5e-58:346:81//AC003006
R-MAMMA1002636//Homo sapiens clone DJ0810E06, WORKING DRAFT SEQUENCE, 8 unordered pieces.//1.1e-52:285:92//AC004895
R-MAMMA1002637//Mus musculus kinesin light chain 2 (Klc2) mRNA, complete cds.//2.1e-13:359:64//AF055666
R-MAMMA1002646//Human DNA sequence *** SEQUENCING IN PROGRESS *** from clone 394I7, WORKING DRAFT SEQUENCE.//2.5e-24:285:68//AL023585
R-MAMMA1002650//Human IGF-II gene exon 2 for insulin-like growth factor II located on chromosome 11.//0.64:237:61//X03424
R-MAMMA1002655//Homo sapiens mini satellite cebl repeat region.//0.18:152:65//AF048727
R-MAMMA1002662//Homo sapiens clone DJ0739M23, complete sequence.//2.5e-46:370:82//AC004870
R-MAMMA1002665//Human DNA sequence from PAC 435C23 on chromosome X. Contains ESTs.//7.4e-55:298:92//Z92844
R-MAMMA1002671//RPCI11-45M10.TK RPCI11 Homo sapiens genomic clone R-45M10, genomic survey sequence.//0.99:151:66//AQ194411
R-MAMMA1002673//Homo sapiens DNA sequence from PAC 454M7 on chromosome Xq25-26.3. Contains the OCRL1 gene for Lowe Oculocerebrorenal Syndrome protein OCRL-1. Contains ESTs, STSs and GSSs, complete sequence.//3.1e-38:410:76//AL022162
R-MAMMA1002684//Homo sapiens mRNA for KIAA0214 protein, complete cds.//1.4e-107:544:96//D86987
R-MAMMA1002685//HS_2052_A1_H02_T7 CIT Approved Human Genomic Sperm Library D Homo sapiens genomic clone Plate=2052 Col=3 Row=O, genomic survey sequence.//1.2e-23:255:75//AQ231087
R-MAMMA1002698//Homo sapiens Xp22-132-134 BAC GSHB-590J15 (Genome Systems Human BAC library) complete sequence.//1.1e-38:299:83//AC004673
R-MAMMA1002699//Mus musculus intersectin-EH binding protein Ibp1 mRNA, partial cds.//3.3e-05:61:93//AF057285
R-MAMMA1002701//Homo sapiens gene for AF-6, complete cds.//3.5e-39:317:81//AB011399
R-MAMMA1002708//Homo sapiens 12p13.3 PAC RPCI5-977L1 (Roswell Park Cancer Institute Human PAC library) complete sequence.//0.26:365:62//AC005293
R-MAMMA1002711//Homo sapiens chromosome 21 PAC LLNLP704F18108Q13.//2.5e-31:304:77//AJ006995
R-MAMMA1002721//Human DNA sequence *** SEQUENCING IN PROGRESS *** from clone 473B4, WORKING DRAFT SEQUENCE.//2.3e-40:279:87//Z83826
R-MAMMA1002727//Plasmodium falciparum 3D7 chromosome 12 PFYAC588 genomic sequence, WORKING DRAFT SEQUENCE, 2 unordered pieces.//0.45:183:64//AC004710
R-MAMMA1002728//Human Chromosome 11 Overlapping Cosmids cSRL72g7 and cSRL140b8, complete sequence.//1.1e-42:410:74//AC002037
R-MAMMA1002744//Human chromosome 8 BAC clone CIT987SK-2A8 complete sequence.//1.6e-19:473:63//U96629
R-MAMMA1002746//Homo sapiens chromosome 17, clone hRPK.136_H_19, complete sequence.//2.2e-108:544:97//AC005856
R-MAMMA1002748//Homo sapiens 3p22 Contig 7 PAC RPCI4-672N11 (Roswell Park Cancer Institute Human PAC Library) complete sequence.//5.9e-106:551:95//AC006055
R-MAMMA1002754//Homo sapiens clone GS259H13, WORKING DRAFT SEQUENCE, 4 unordered pieces.//1.7e-34:305:79//AC005020
R-MAMMA1002758//Homo sapiens ccr2b (ccr2), ccr2a (ccr2), ccr5 (ccr5) and ccr6 (ccr6) genes, complete cds, and lactoferrin (lactoferrin) gene, partial cds, complete sequence.//0.00014:130:74//U95626
R-MAMMA1002764//Homo sapiens chromosome 19, cosmid R33632, complete sequence.//8.7e-10:118:81//AC005781
R-MAMMA1002765//Homo sapiens chromosome 19, cosmid F20900, complete sequence.//1.2e-31:290:78//AC006128
R-MAMMA1002769//Human DNA sequence from PAC 36J3, between markers DXS1192 and DXS102 on chromosome X.//0.94:260:62//Z82975
R-MAMMA1002780//Human DNA sequence *** SEQUENCING IN PROGRESS *** from clone 620E11, WORKING DRAFT SEQUENCE.//2.6e-21:529:62//AL031667
R-MAMMA1002782//Human DNA sequence *** SEQUENCING IN PROGRESS *** from clone 199H16, WORKING DRAFT SEQUENCE.//2.8e-30:234:72//AL022320
R-MAMMA1002796//Human DNA sequence *** SEQUENCING IN PROGRESS *** from clone 237J2, WORKING DRAFT SEQUENCE.//1.0:155:66//AL021394
R-MAMMA1002807//Human DNA sequence from BAC 941F9 on chromosome 22q11.2-qter. Contains ESTs, STSs and 3' part of FIBULIN-1 D PRECURSOR like gene, part of a Brain Protein E46 like gene and a CpG island, complete sequence.//5.0e-42:443:75//Z95331
R-MAMMA1002820//345M16.TVB CIT978SKA1 Homo sapiens genomic clone A-345M16, genomic survey sequence.//1.3e-14:95:87//B17487
R-MAMMA1002830//Human PAC clone DJ515N1 from 22q11.2-q22, complete sequence.//4.1 e-20:223:74//AC002073
R-MAMMA1002833//Homo sapiens Xp22 bins 3-5 PAC RPCI4-617A9 (Roswell Park Cancer Institute Human PAC Library) containing Arylsulfatase D and E genes, complete sequence.//1.8e-37:295:84//AC005295
R-MAMMA1002835
R-MAMMA1002838//Human gene hY3 encoding a cytoplasmic Ro RNA.//4.4e-14:108:92//V00585
R-MAMMA1002842//CIT-HSP-2017022.TRB CIT-HSP Homo sapiens genomic clone 2017022, genomic survey sequence.//5.2e-43:168:85//B67141
R-MAMMA1002843//Homo sapiens clone GS051M12, complete sequence.//8.7e-44:525:71//AC005007
R-MAMMA1002844
R-MAMMA1002858//H.sapiens ERF-1 mRNA 3' end.//2.8e-99:361:91//X79067
R-MAMMA1002868//Homo sapiens clone DJ0852O24, WORKING DRAFT SEQUENCE, 2 unordered pieces.//9.6e-39:288:81//AC004906
R-MAMMA1002871//Homo sapiens BAC clone NH0539B24 from 7p15.1-p14, complete sequence.//0.0022:490:57//AC006044
R-MAMMA1002880//Homo sapiens Xp22 Bins 35-37 BAC GSHB-214D18 (Genome Systems Human BAC Library) complete sequence.//1.3e-09:143:76//AC005296
R-MAMMA1002881//Human thymopoietin (TMPO) gene, partial exon 6, complete exon 7, partial exon 8, and partial cds for thymopoietin beta.//5.1e-41:264:87//U18271
R-MAMMA1002886//Homo sapiens DNA sequence from PAC 168L15 on chromosome 6q26-27. Contains RSK3 gene, ribosomal protein S6 kinase, EST, GSS, STS. CpG island, complete sequence.//4.7e-32:216:90//AL022069
R-MAMMA1002887
R-MAMMA1002890
   3.4e-49:376:81//AG006257
R-MAMMA1002892//Homo sapiens PAC clone DJ0765G07 from 7q11, complete sequence.//6.0e-60:344:79//AC004881
R-MAMMA1002895//RPCI11-90K13.TV RPCI11 Homo sapiens genomic clone R-90K13, genomic survey sequence.//2.1e-34:300:77//AQ283502
R-MAMMA1002908//Human Chromosome X, complete sequence.//4.2e-39:297:85//AC004070
R-MAMMA1002909//*** SEQUENCING IN PROGRESS *** Homo sapiens chromosome 4, BAC clone C0442P12; HTGS phase 1, WORKING DRAFT SEQUENCE, 5 unordered pieces.//3.4e-23:344:74//AC005798
R-MAMMA1002930//Homo sapiens PAC clone DJ1048B16 from 7q34-q36, complete sequence.//5.2e-39:261:88//AC006019
R-MAMMA1002938//C.pasteurianum gap gene.//1.0:343:59//X72219
R-MAMMA1002941//Homo sapiens chromosome 17, clone hRPK.346_K_10, complete sequence.//6.3e-88:556:87//AC006120
R-MAMMA1002947
   0.48:156:69//AC005469
R-MAMMA1002964//Human DNA sequence from PAC 426I6 on chromosome 1p34.1-1p35. Contains NIPP-1-like gene a nuclear inhibitor of protein phosphatase-1, ESTs, and a CA repeat.//1.2e-39:473:73//AL020997
R-MAMMA1002970//Homo sapiens chromosome 5, P1 clone 793c5 (LBNL H57), complete sequence.//4.7e-47:420:77//AC005200
R-MAMMA1002972//alpha 1 syntrophin [human, mRNA Partial, 1771 nt] .//0.97:305:62//S81737
R-MAMMA1002973//Human DNA sequence from cosmid V210E9, between markers DXS366 and DXS87 on chromosome X.//2.6e-35:256:85//Z70280
R-MAMMA1002982 1.0e-27:110:85//AG005524
R-MAMMA1002987//Homo sapiens PAC clone DJ1086D14, complete sequence.//1.4e-28:527:66//AC004460
R-MAMMA1003003//Homo sapiens chromosome 10 clone CRI-JC2059 map 10q24.1-10q24.2, WORKING DRAFT SEQUENCE, 1 ordered pieces.//7.9e-48:418:78//AC006109
R-MAMMA1003004//, complete sequence.//2.0e-12:442:61//AC005406
R-MAMMA1003007//Homo sapiens chromosome 10 clone CRI-JC2059 map 10q24.1-10q24.2, WORKING DRAFT SEQUENCE, 1 ordered pieces.//1.7e-48:293:91//AC006109
R-MAMMA1003011//A-306G8.TP CIT978SK Homo sapiens genomic clone A-306G8, genomic survey sequence.//0.45:168:64//B18092
R-MAMMA1003015//Homo sapiens chromosome 5p, BAC clone 50g21 (LBNL H154), complete sequence.//2.9e-44:399:77//AC005740
R-MAMMA1003019//RPCI11-9J9.TV RPCI-11 Homo sapiens genomic clone RPCI-11-9J9, genomic survey sequence.//2.7e-14:294:68//B71583
R-MAMMA1003026//HS_2166_B2_C12_MF CIT Approved Human Genomic Sperm Library D Homo sapiens genomic clone Plate=2166 Col=24 Row=F, genomic survey sequence.//0.021:189:64//AQ125639
R-MAMMA1003031//Homo sapiens chromosome 5, BAC clone 319C17 (LBNL H159), complete sequence.//1.8e-98:525:95//AC005214
R-MAMMA1003035//Homo sapiens 12q13.1 Cosmid C174F5 (Lawrence Livermore LL12NC01 or LL12NC02 human cosmid libraries) complete sequence.//6.7e-06:297:63//AC004550
R-MAMMA1003039//RPCI11-56J17.TJ RPCI11 Homo sapiens genomic clone R-56J17, genomic survey sequence.//0.21:375:59//AQ081889
R-MAMMA1003040//Human DNA sequence from cosmid L108f12, Huntington's Disease Region, chromosome 4p16.3.//2.7e-29:298:67//Z49235
R-MAMMA1003044//Homo sapiens chromosome 19, cosmid R30676, complete sequence.//2.9e-14:113:91//AC004560
R-MAMMA1003047
R-MAMMA1003049
R-MAMMA1003055//Human DNA sequence *** SEQUENCING IN PROGRESS *** from clone 377F16, WORKING DRAFT SEQUENCE.//2.3e-45:317:86//Z93783
R-MAMMA1003056//Homo sapiens chromosome 19, cosmid R34275, complete sequence.//1.0:229:63//AC005305
R-MAMMA1003057//M.domesticus MD6 mRNA.//6.2e-42:326:82//X54352
R-MAMMA1003066//Human DNA sequence *** SEQUENCING IN PROGRESS *** from clone 473B4, WORKING DRAFT SEQUENCE.//3.1e-49:299:87//Z83826
R-MAMMA1003089//Homo sapiens BAC clone RG298G08 from 7p15-p21, complete sequence.//2.7e-30:520:67//AC005084
R-MAMMA1003099//RPCI11-8N9.TP RPCI-11 Homo sapiens genomic clone RPCI-11-8N9, genomic survey sequence.//4.2e-44:338:82//B71494
R-MAMMA1003104//Mus musculus rostral cerebellar malformation protein (rcm) mRNA, complete cds.//3.4e-48:423:79//U72634
R-MAMMA1003113//Homo sapiens chromosome 12p13.3 clone RPCI11-433J6, WORKING DRAFT SEQUENCE, 100 unordered pieces.//4.8e-114:567:97//AC006087
R-MAMMA1003127//Human DNA sequence *** SEQUENCING IN PROGRESS *** from clone 250D10, WORKING DRAFT SEQUENCE.//1.4e-34:283:83//Z99716
R-MAMMA1003135//P.knowlesi Mbn-cutting sites in lambda KBS50.//0.010:243:62//M38776
R-MAMMA1003140//Homo sapiens chromosome 17, clone HCIT87G17, complete sequence.//6.7e-34:288:81//AC003663
R-MAMMA1003146//Saccharomyces douglasii mitochondrial cytochrome c oxidase subunit I (COXI) gene, complete cds.//4.8e-08:438:59//M97514
R-nnnnnnnnnnnn//Human DNA sequence *** SEQUENCING IN PROGRESS *** from clone 598F2, WORKING DRAFT SEQUENCE.//1.7e-63:149:94//AL021579
R-MAMMA1003166//HS_3128_A1_B01_MR CIT Approved Human Genomic Sperm Library D Homo sapiens genomic clone Plate=3128 Col=1 Row=C, genomic survey sequence.//3.0e-17:261:70//AQ140766
R-NT2RM2002580//Homo sapiens clone 24781 mRNA sequence.//2.6e-111:593:94//AF070640
R-NT2RM4000024
R-NT2RM4000027//Homo sapiens PAC clone DJ1194E14 from 7p21, complete sequence.//0.026:476:56//AC004993
R-NT2RM4000030//Mus musculus musculus sex determining protein (Sry) gene, complete cds.//0.00044:378:59//U70653
R-NT2RM4000046//M.mulatta MHC DR beta 6 gene encoding major histocompatibility complex.//0.27:130:64//Z26239
R-NT2RM4000061
R-NT2RM4000085//Homo sapiens clone 24700 unknown mRNA, partial cds.//7.2e-112:550:97//AF070639
R-NT2RM4000086//RPCI11-6J23.TV RPCI-11 Homo sapiens genomic clone RPCI-11-6J23, genomic survey sequence.//7.2e-18:277:71//B49463
R-NT2RM4000104//F.rubripes GSS sequence, clone 063K10aG5, genomic survey sequence.//3.6e-08:287:61//Z88817
R-NT2RM4000139//Homo sapiens chromosome 16, cosmid clone 330D11 (LANL), complete sequence.//9.4e-08:336:65//AC005199
R-NT2RM4000155
R-NT2RM4000156//Homo sapiens chromosome 17, clone hRPK.136_H_19, complete sequence.//3.4e-23:335:72//AC005856
R-nnnnnnnnnnnn//Mouse kif4 mRNA for microtubule-based motor protein KIF4, complete cds.//1.6e-87:551:87//D12646
R-NT2RM4000169//Human ribosomal protein L37a mRNA sequence.//5.9e-14:122:88//L22154
R-NT2RM4000191
R-NT2RM4000197//HS_3241_A2_H05_MR CIT Approved Human Genomic Sperm Library D Homo sapiens genomic clone Plate=3241 Col=10 Row=O, genomic survey sequence.//2.8e-86:430:97//AQ206812
R-NT2RM4000199//Mus musculus Yp BAC GSMB-368G7 (Genome Systems Mouse BAC Library) complete sequence.//0.0047:193:63//AC006056
R-NT2RM4000200
R-NT2RM4000202//Homo sapiens chromosome 16, cosmid clone 378E2 (LANL), complete sequence.//2.1e-40:334:76//AC004035
R-NT2RM4000210//Homo sapiens mRNA for KIAA0712 protein, complete cds.//5.2e-102:546:94//AB018255
R-NT2RM4000215
R-nnnnnnnnnnnn//Homo sapiens chromosome 10 clone CIT987SK-1144G6 map 10q25.1, complete sequence.//2.1e-55:303:86//AC005383
R-NT2RM4000233//Struthio camelus microsatellite sequence OSM 7.//1.2e-07:198:67//AF003735
R-NT2RM4000244//Homo sapiens chromosome 19, BAC CIT-B-393i15 (BC301323), complete sequence.//1.7e-49:322:88//AC006116
R-NT2RM4000251//Homo sapiens Chromosome 22q11.2 BAC Clone 72f8 In DGCR Region, complete sequence.//0.97:184:66//AC000085
R-NT2RM4000265//Human PAC clone DJ073F11 from Xq23, complete sequence.//6.2e-66:552:78//AC000055
R-NT2RM4000290//Human DNA sequence *** SEQUENCING IN PROGRESS *** from clone 394I7, WORKING DRAFT SEQUENCE.//1.4e-05:229:65//AL023585
R-NT2RM4000324
R-NT2RM4000327//Human DNA sequence *** SEQUENCING IN PROGRESS *** from clone 75N14, WORKING DRAFT SEQUENCE.//3.3e-42:443:75//Z97199
R-NT2RM4000344//Homo sapiens clone DJ0309D19, WORKING DRAFT SEQUENCE, 12 unordered pieces.//6.4e-64:433:84//AC004826
R-NT2RM4000349//Human mRNA for KIAA0005 gene, complete cds.//7.7e-11:210:69//D13630
R-NT2RM4000354//Caenorhabditis elegans cosmid T14A8.//0.084:257:60//U50066
R-NT2RM4000356
R-NT2RM4000366//Homo sapiens mRNA for KIAA0642 protein, partial cds.//8.7e-112:577:95//AB014542
R-NT2RM4000368
   1.6e-48:348:85//AG006257
R-NT2RM4000386//Rat mRNA for growth potentiating factor, complete cds.//4.4e-35:141:87//D42148
R-NT2RM4000395//RPCI11-8N9.TP RPCI-11 Homo sapiens genomic clone RPCI-11-8N9, genomic survey sequence.//1.4e-25:207:75//871494
R-NT2RM4000414//Human DNA sequence *** SEQUENCING IN PROGRESS *** from clone 228H13, WORKING DRAFT SEQUENCE.//7.1e-17:492:64//AL031985
R-NT2RM4000421//RPCI11-66B1.TK RPCI11 Homo sapiens genomic clone R-66B1, genomic survey sequence.//1.8e-40:311:82//AQ241167
R-NT2RM4000425//Homo sapiens chromosome Xp22-135-136 clone GSHB-567I1, WORKING DRAFT SEQUENCE, 35 unordered pieces.//2.5e-47:316:87//AC005867
R-NT2RM4000433//Mus musculus retinoic acid-responsive protein (Stra6) mRNA, complete cds.//1.6e-17:133:78//AF062476
R-NT2RM4000457
R-NT2RM4000471//Homo sapiens mRNA for putative tRNA splicing protein, partial.//4.6e-113:559:96//AJ010952
R-NT2RM4000486//Homo sapiens mRNA, complete cds, clone:RES4-22C.//0.00015:170:67//AB000461
R-NT2RM4000496
R-NT2RM4000511//Rat troponin T cardiac isoform gene, complete cds.//0.21:290:58//M80829
R-NT2RM4000514//CIT-HSP-2169K4.TR CIT-HSP Homo sapiens genomic clone 2169K4, genomic survey sequence.//1.5e-20:150:89//B95717
R-nnnnnnnnnnnn//HS-1024-B2-G01-MR.abi CIT Human Genomic Sperm Library C Homo sapiens genomic clone Plate=CT 803 Col=2 Row=N, genomic survey sequence.//6.3e-10:74:98//B34556
R-NT2RM4000520//Caenorhabditis elegans cosmid F36H12.//0.15:406:61//AF078790
R-NT2RM4000531
R-NT2RM4000532//Plasmodium falciparum chromosome 2, section 28 of 73 of the complete sequence.//1.0:119:66//AE001391
R-NT2RM4000534//paramecium species 4.51er mt dna dimer: replication init. region, clone 2.//9.8e-05:326:60//K00909
R-NT2RM4000585//HS_3252_A2_G08_MR CIT Approved Human Genomic Sperm Library D Homo sapiens genomic clone Plate=3252 Col=16 Row=M, genomic survey sequence.//1.9e-69:376:93//AQ219890
R-NT2RM4000590//CIT-HSP-539O24.TV CIT-HSP Homo sapiens genomic clone 539O24, genomic survey sequence.//1.7e-38:226:93//B50657
R-NT2RM4000595//Human Chromosome X clone bWXD342, complete sequence.//1.0:239:61//AC004072
R-NT2RM4000603//RPCI11-49P13.TK RPCI11 Homo sapiens genomic clone R-49P13, genomic survey sequence.//0.77:139:64//AQ051950
R-nnnnnnnnnnnn
R-NT2RM4000616//HS_3107_A2_B03_MR CIT Approved Human Genomic Sperm Library D Homo sapiens genomic clone Plate=3107 Col=6 Row=C, genomic survey sequence.//1.3e-54:272:99//AQ210034
R-NT2RM4000674
R-NT2RM4000689//Mus musculus pericentrin mRNA, complete cds.//3.5e-70:551:80//U05823
R-NT2RM4000698
R-nnnnnnnnnnnn
R-NT2RM4000712//Homo sapiens clone NH0512E16, complete sequence.//0.54:294:58//AC005039
R-NT2RM4000717//Plasmodium falciparum MAL3P8, complete sequence.//0.050:387:58//AL034560
R-NT2RM4000733//Human DNA sequence *** SEQUENCING IN PROGRESS *** from clone 423B22, WORKING DRAFT SEQUENCE.//1.0e-107:566:95//AL034379
R-NT2RM4000734//Homo sapiens mRNA for KIAA0760 protein, partial cds.//1.1e-103:536:95//AB018303
R-NT2RM4000741//CIT-HSP-2294N4.TR CIT-HSP Homo sapiens genomic clone 2294N4, genomic survey sequence.//5.2e-41:244:93//AQ006361
R-NT2RM4000751//Human DNA sequence *** SEQUENCING IN PROGRESS *** from clone 537K23, WORKING DRAFT SEQUENCE.//2.7e-28:416:67//AL034405
R-NT2RM4000764//Human HepG2 3' region MboI cDNA, clone hmd3g01m3.//2.1e-33:199:96//D17217
R-NT2RM4000778//Homo sapiens Xp22 BAC 620F15 (Genome Systems BAC library) complete sequence.//0.00060:241:62//AC002980
R-NT2RM4000779//Homo sapiens mRNA for KIAA0451 protein, complete cds.//2.9e-104:546:94//AB007920
R-NT2RM4000787//Homo sapiens, clone hRPK.3_A_1, complete sequence.//5.3e-32:321:77//AC006198
R-NT2RM4000790//Homo sapiens chromosome 19, cosmid R27216, complete sequence.//1.9e-111:552:97//AC005306
R-NT2RM4000795//Homo sapiens Chromosome 17p13 Cosmid Clone cos39, complete sequence.//0.74:364:57//U58675
R-NT2RM4000796//Homo sapiens full-length insert cDNA clone ZD62D10.//2.7e-105:510:98//AF086348
R-NT2RM4000798//Human polymorphic epithelial mucin core protein mRNA, 3' end.//7.7e-27:158:96//M21868
R-NT2RM4000813
R-NT2RM4000820//, complete sequence.//2.0e-104:432:97//AC005406
R-NT2RM4000833//Arabidopsis thaliana genomic DNA, chromosome 5, P1 clone: MXI22, complete sequence.//2.0e-07:166:68//AB012248
R-NT2RM4000848//Rabies virus matrix (M) protein mRNA, complete cds.//0.073:70:84//M22013
R-NT2RM4000852//Plasmodium falciparum 3D7 chromosome 12 PFYAC1122 genomic sequence, WORKING DRAFT SEQUENCE, 3 unordered pieces.//1.0:237:62//AC004709
R-NT2RM4000855
R-nnnnnnnnnnnn//HS_3189_B2_B08_T7 CIT Approved Human Genomic Sperm Library D Homo sapiens genomic clone Plate=3189 Col=16 Row=D, genomic survey sequence.//2.1e-06:114:73//AQ300597
R-NT2RM4000895//Pan troglodytes HS19.8-similar locus and Y Alu element, genomic survey sequence.//3.8e-46:207:91//AF077058
R-NT2RM4000950//Human BAC clone RG341D10 from 7p15-p21, complete sequence.//1.0:336:60//AC002530
R-NT2RM4000971//Human Xq28 cosmids U126G1, U142F2, U69B6, U145C10, U169A5, U84H1, U24D12, U80A7, U153E6, L35485, and R7-163A8 containing iduronate 2-sulfatase gene and pseudogene, complete sequence.//7.1e-09:259:64//AF011889
R-NT2RM4000979
R-NT2RM4000996//HS_3164_A1_E02_T7 CIT Approved Human Genomic Sperm Library D Homo sapiens genomic clone Plate=3164 Col=3 Row=I, genomic survey sequence.//2.0e-82:443:94//AQ141622
R-NT2RM4001002//Homo sapiens mRNA for KIAA0729 protein, partial cds.//1.2e-112:545:97//AB018272
R-NT2RM4001016//Homo sapiens mRNA for KIAA0639 protein, partial cds.//7.9e-113:556:97//AB014539
R-NT2RM4001032//Homo sapiens Surf-5 and Surf-6 genes.//1.2e-10:120:82//AJ224639
R-NT2RM4001047//Human DNA sequence *** SEQUENCING IN PROGRESS *** from clone 163G9, WORKING DRAFT SEQUENCE.//1.0:158:67//AL008733
R-NT2RM4001054//CIT-HSP-2292N8.TR CIT-HSP Homo sapiens genomic clone 2292N8, genomic survey sequence.//5.8e-19:118:97//AQ004096
R-nnnnnnnnnnnn//Mouse DNA with homology to EBV IR3 repeat, segment 1, clone Mu2.//1.0e-05:271:64//M10296
R-NT2RM4001092//CITBI-E1-2524J20.TR CITBI-E1 Homo sapiens genomic clone 2524J20, genomic survey sequence.//1.0:186:63//AQ277294
R-NT2RM4001116
R-NT2RM4001140//Homo sapiens PAC clone DJ0964C11 from 7p14-p15, complete sequence.//3.6e-79:468:90//AC004593
R-NT2RM4001151//HS_2270_B1_E05_MF CIT Approved Human Genomic Sperm Library D Homo sapiens genomic clone Plate=2270 Col=9 Row=J, genomic survey sequence.//5.5e-62:312:98//AQ163739
R-NT2RM4001155//Homo sapiens chromosome 12p13.3 clone RPCI4-816N1, WORKING DRAFT SEQUENCE, 31 unordered pieces.//1.4e-107:536:97//AC005841
R-NT2RM4001160//HS_3015_B1_H10_T7 CIT Approved Human Genomic Sperm Library D Homo sapiens genomic clone Plate=3015 Col=19 Row=P, genomic survey sequence.//7.1e-35:201:95//AQ118712
R-NT2RM4001187//X.laevis xUBFbeta2 mRNA for upstream binding factor 1.//0.019:177:63//X57201
R-NT2RM4001191//HS_3002_A1_F05_MF CIT Approved Human Genomic Sperm Library D Homo sapiens genomic clone Plate=3002 Col=9 Row=K, genomic survey sequence.//3.9e-33:230:75//AQ088791
R-NT2RM4001200//Homo sapiens full-length insert cDNA clone YL35H03.//7.5e-69:335:99//AF085857
R-NT2RM4001203
R-NT2RM4001204
R-NT2RM4001217
R-NT2RM4001256
R-NT2RM4001258
R-NT2RM4001309
R-NT2RM4001313//Homo sapiens 12q24.1 PAC RPCI1-71H24 (Roswell Park Cancer Institute Human PAC library) complete sequence.//0.00055:183:63//AC004551
R-NT2RM4001316//Homo sapiens chromosome 17, clone hCIT.117_K_16, complete sequence.//4.5e-21:212:79//AC004757
R-NT2RM4001320//CIT-HSP-2303E22.TR CIT-HSP Homo sapiens genomic clone 2303E22, genomic survey sequence.//3.8e-30:86:89//AQ021084
R-NT2RM4001340
   0.0027:493:60//AC005133
R-NT2RM4001344
R-NT2RM4001347//CITBI-E1-2506I20.TR CITBI-E1 Homo sapiens genomic clone 2506I20, genomic survey sequence.//6.5e-16:1.01:99//AQ262797
R-NT2RM4001371//CITBI-E1-2503G21.TR CITBI-E1 Homo sapiens genomic clone 2503G21, genomic survey sequence.//0.063:140:65//AQ265776
R-NT2RM4001382//HS_3044_A1_F02_MR CIT Approved Human Genomic Sperm Library D Homo sapiens genomic clone Plate=3044 Col=3 Row=K, genomic survey sequence.//0.96:103:66//AQ098668
R-NT2RM4001384//R.norvegicus mRNA for dendrin.//8.5e-07:120:75//Y09000
R-NT2RM4001410//Bovine cytochrome P450-scc mRNA fragment.//2.3e-15:199:75//M25920
R-NT2RM4001411//Rattus norvegicus FceRI gamma-chain interacting protein SH2-B (SH2-B) mRNA, complete cds.//1.7e-55:235:83//U57391
R-NT2RM4001412
R-NT2RM4001414//Homo sapiens Xp22 Cosmids U98B4 and U24F2 (Lawrence Livermore human cosmid library) complete sequence.//1.7e-80:489:89//U69730
R-NT2RM4001437//RPCI11-56D2.TJ RPCI11 Homo sapiens genomic clone R-56D2, genomic survey sequence.//3.8e-43:250:93//AQ081969
R-NT2RM4001444//Homo sapiens Xp22-171-173 BAC GSHB-312I4 (Genome Systems Human BAC Library) complete sequence.//0.0034:224:63//AC005926
R-NT2RM4001454//Homo Sapiens Chromosome X clone bWXD90, complete sequence.//2.4e-33:360:68//AC004075
R-NT2RM4001455//HS_3229_B1_E04_MR CIT Approved-Human Genomic Sperm Library D Homo sapiens genomic clone Plate=3229 Col=7 Row=J, genomic survey sequence//1.0:183:61//AQ191289
R-NT2RM4001483//Homo sapiens clone DJ0826E18, WORKING DRAFT SEQUENCE, 4 unordered pieces.//2.2e-51:451:79//AC005282
R-NT2RM4001489//Homo sapiens mRNA for KIAA0685 protein, complete cds.//2.2e-102:547:93//AB014585
R-NT2RM4001519//HS_2208_A1_F07_MR CIT Approved Human Genomic Sperm Library D Homo sapiens genomic clone Plate=2208 Col=13 Row=K, genomic survey sequence.//0.25:214:63//AQ091836
R-NT2RM4001522//H.sapiens gene for Cu/Zn-superoxide dismutase.//3.6e-13:246:70//Z29336
R-NT2RM4001557//Plasmodium falciparum MAL3P4, complete sequence.//0.055:320:58//AL008970
R-NT2RM4001565//Homo sapiens chromosome 12p13.3 clone RPCI11-189M20, WORKING DRAFT SEQUENCE, 39 unordered pieces.//3.9e-26:329:72//AC005910
R-NT2RM4001566//Human trophinin mRNA, complete cds.//6.3e-38:296:86//U04811
R-NT2RM4001569//Human DNA sequence from clone 461P17 on chromosome 20q12-13.2. Contains four novel (pseudo)genes for proteins with Kunitz/Bovine pancreatic trypsin inhibitor and/or WAP-type (Whey Acidic Protein) 'four-disulfide core' domains, COX6C (Cytochrome C Oxidase Polypeptide VIC, EC 1.9.3.1) and RPL5 (60S Ribosomal Protein L5) pseudogenes, a pseudogene similar to part of the HSPD1 (HSP60, Mitochondrial Matrix Protein P1 precursor, Heat Shock Protein 60, GROEL protein, HUCHA60) gene, and the Major Epididymis-specific protein E4 precursor (HE4, Epididymis Secretory protein E4, WAP-type (Whey Acidic Protein) 'four-disulfide core' domain) gene. Contains ESTs, an STS, GSSs and a putative CpG island, complete sequence.//2.0e-35:213:89//AL031663
R-NT2RM4001582//Mus musculus COP9 complex subunit 7b (COPS7b) mRNA, complete cds.//5.4e-60:558:77//AF071317
R-nnnnnnnnnnnn//M.musculus mRNA of enhancer-trap-locus 1.//4.8e-86:565:85//X69942
R-NT2RM4001594//Human interleukin-13 (IL-13) precursor gene, complete cds.//0.083:283:61//U31120
R-NT2RM4001597//HS_2059_A1_G11_MR CIT Approved Human Genomic Sperm Library D Homo sapiens genomic clone Plate=2059 Col=21 Row=M, genomic survey sequence.//4.4e-09:105:83//AQ245136
R-NT2RM4001605//Homo sapiens mRNA for KIAA0791 protein, complete cds.//6.7e-111:565:95//AB018334
R-NT2RM4001611//Homarus americanus ryanodine receptor (RyR) mRNA, partial cds.//1.0:364:61//AF051936
R-NT2RM4001629//RPCI11-54G14.TJ RPCI11 Homo sapiens genomic clone R-54G14, genomic survey sequence.//0.0018:347:61//AQ083173
R-NT2RM4001650
R-NT2RM4001662//Homo sapiens DNA sequence from PAC 159A15 on chromosome Xp11.21-p11.23. Contains inter-alpha-trypsin inhibitor heavy chain H3 precursor-like protein.//0.75:212:62//AL022575
R-NT2RM4001666//Homo sapiens Chromosome 16 BAC clone CIT987SK-A-233A8, complete sequence.//2.6e-26:461:65//AC004685
R-NT2RM4001682//Human DNA sequence from clone 30M3 on chromosome 6p22.1-22.3. Contains three novel genes, one similar to C. elegans Y63D3A.4 and one similar to (predicted) plant, worm, yeast and archaea bacterial genes, and the first exon of the KIAA0319 gene. Contains ESTs, GSSs and putative CpG islands, complete sequence.//1.5e-107:544:96//AL031775
R-NT2RM4001710//Human DNA sequence *** SEQUENCING IN PROGRESS *** from clone 126A5, WORKING DRAFT SEQUENCE.//1.8e-110:580:95//AL031447
R-NT2RM4001714//Plasmodium falciparum 3D7 chromosome 12 PFYAC812 genomic sequence, WORKING DRAFT SEQUENCE, 8 unordered pieces.//3.1e-10:543:59//AC004153
R-nnnnnnnnnnnn//Human DNA sequence from clone 931K24 on chromosome 20p12 Contains ESTs and GSSs, complete sequence.//8.7e-111:577:94//AL034430
R-NT2RM4001731//Ovis aries dinucleotide repeat polymorphism at MAF92 locus.//0.017:93:73//M80527
R-NT2RM4001741//Mouse mRNA for talin.//2.4e-34:273:83//X56123
R-NT2RM4001746//Human DNA sequence *** SEQUENCING IN PROGRESS *** from clone 316G12, WORKING DRAFT SEQUENCE.//1.7e-112:567:96//AL031709
R-NT2RM4001754//Homo sapiens PAC clone 248O15 from 13q12-q13, complete sequence.//1.4e-64:475:83//AC002483
R-NT2RM4001758//R.norvegicus mRNA for serine/threonine kinase MARK1.//1.9e-18:202:78//Z83868
R-NT2RM4001776//Homo sapiens mRNA for KIAA0727 protein, partial cds.//2.0e-22:236:80//AB018270
R-NT2RM4001783//Homo sapiens clone DJ0981O07, complete sequence.//4.4e-106:551:95//AC006017
R-NT2RM4001810//T28D3TF TAMU Arabidopsis thaliana genomic clone T28D3, genomic survey sequence.//0.76:279:60//B27099
R-NT2RM4001813
R-NT2RM4001823
R-NT2RM4001828//HS_3073_A2_E01_MF CIT Approved Human Genomic Sperm Library D Homo sapiens genomic clone Plate=3073 Col=2 Row=I, genomic survey sequence.//1.6e-46:255:96//AQ121030
R-NT2RM4001836//Sus scrofa microsatellite S0398 sequence.//9.4e-06:141:69//U78024
R-NT2RM4001841//Salmo salar microsatellite Ssa65 DNA.//1.5e-06:175:65//AF019184
R-NT2RM4001842//Homo sapiens clone RG228D17, WORKING DRAFT SEQUENCE, 2 unordered pieces.//5.0e-07:332:61//AC005077
R-NT2RM4001856//Mus musculus clone OST16642, genomic survey sequence.//4.8e-30:235:85//AF046633
R-nnnnnnnnnnnn//Hs_3244_B1_F10_MR CIT Approved Human Genomic Sperm Library D Homo sapiens genomic clone Plate=3244 Col=19 Row=L, genomic survey sequence.//3.0e-40:263:89//AQ252798
R-NT2RM4001865//Homo sapiens mRNA for atopy related autoantigen CALC.//5.0e-119:592:97//Y17711
R-NT2RM4001876//Megastigmus wachtli dinucleotide microsatellite, clone
MWA47CT.//0.13:134:64//AJ001069
R-NT2RM4001880
R-NT2RM4001905//HS_2016_B1_H11 _T7 CIT Approved Human Genomic Sperm Library D Homo sapiens genomic clone Plate=2016 Col=21 Row=P, genomic survey sequence.//0.0066:264:59//AQ226877
R-NT2RM4001922//HS_2228_B2_B07_MF CIT Approved Human Genomic Sperm Library D Homo sapiens genomic clone Plate=2228 Col=14 Row=D, genomic survey sequence.//2.5e-35:205:96//AQ065498
R-NT2RM4001930//Homo sapiens chromosome 17, clone hRPC.34_M_24, complete sequence.//0.26:325:63//AC004562
R-NT2RM4001938//Homo sapiens chromosome 17, clone hRPC.1081_P_3, complete sequence.//2.9e-85:421:98//AC005207
R-NT2RM4001940//Homo sapiens timeless homolog mRNA, complete cds.//6.2e-109:556:95//AF098162
R-NT2RM4001953//Human DNA sequence *** SEQUENCING IN PROGRESS *** from clone 473B4, WORKING DRAFT SEQUENCE.//1.3e-08:175:70//Z83826
R-NT2RM4001965//CIT-HSP-385N14.TR CIT-HSP Homo sapiens genomic clone 385N14, genomic survey sequence.//5.7e-69:532:81//B55044
R-nnnnnnnnnnnn//R.norvegicus mRNA for IP63 protein.//1.9e-61:352:83//X99330
R-NT2RM4001979//Homo sapiens full-length insert cDNA clone ZD29F04.//1.1e-98:465:100//AF086241
R-NT2RM4001984//Borrelia burgdorferi (section 47 of 70) of the complete genome.//0.14:461:60//AE001161
R-NT2RM4001987
R-NT2RM4002013
R-NT2RM4002018
R-NT2RM4002034//Homo sapiens chromosome 5, BAC clone 24p24 (LBNL H195), complete sequence.//3.6e-42:277:89//AC005353
R-NT2RM4002044//Homo sapiens PAC clone DJ1102B04 from 7q11.23-7q21, complete sequence.//0.83:476:57//AC006204
R-NT2RM4002054
R-NT2RM4002062//Human microsomal epoxide hydrolase gene, exons 5 and 6.//0.11:136:67//U06659
R-NT2RM4002063//Oryctolagus cuniculus sarcosine oxidase (SOX) mRNA, complete cds.//2.9e-99:503 :96//U82267
R-nnnnnnnnnnnn//Homo sapiens CAGH45 mRNA, complete cds.//9.6e-41:554:68//U80742
R-NT2RM4002067//Human DNA sequence *** SEQUENCING IN PROGRESS *** from clone 329A5, WORKING DRAFT SEQUENCE.//7.7e-64:476:81//Z97832
R-NT2RM4002073//Mus musculus fatty acid transport protein 3 mRNA, partial cds.//1.1e-33:238:85//AF072758
R-NT2RM4002075//Plasmodium falciparum 3D7 chromosome 12 PFYAC1383 genomic sequence, WORKING DRAFT SEQUENCE, 3 unordered pieces.//0.0031:403:57//AC005504
R-NT2RM4002093//Human Chromosome 11 pac pDJ227b23, WORKING DRAFT SEQUENCE, 19 unordered pieces.//9.4e-07:322:62//AC000383
R-nnnnnnnnnnnn//Mouse kif4 mRNA for microtubule-based motor protein KIF4, complete cds.//5.6e-44:432:74//D12646
R-NT2RM4002128//Human HepG2 partial cDNA, clone hmd2e12m5.//2.0e-26:186:90//D17000
R-NT2RM4002140
R-NT2RM4002145//Homo sapiens full-length insert cDNA clone ZD38E12.//1.4e-15:193:76//AF086247
R-NT2RM4002146//Human ABL gene, intron 1b, partial sequence.//0.66:170:63//U07562
R-NT2RM4002161//Homo sapiens laforin (EPM2A) mRNA, partial cds.//4.5e-110:560:96//AF084535
R-NT2RM4002174//Homo sapiens chromosome 17, clone hRPK.74_E_22, complete sequence.//8.0e-43:302:85//AC005696
R-NT2RM4002189
R-NT2RM4002194//Human Cosmid g5129g129 from 7q31.3, complete sequence.//0.29:382:60//AC003960
R-NT2RM4002205//Spiroplasma virus (SpV1-R8A2 B) complete genome.//3.5e-05:432:56//X51344
R-NT2RM4002213
R-NT2RM4002226//Homo sapiens chromosome 17, clone HCIT187M2, complete sequence.//0.94:198:61//AC004448
R-NT2RM4002251
R-NT2RM4002256//Homo sapiens PAC clone DJ0570D02 from 7p13-p14, complete sequence.//2.3e-58:299:85//AC004837
R-NT2RM4002266//H.sapiens CpG island DNA genomic Mse1 fragment, clone 179f11, forward read cpg179f11.ft1a.//0.72:97:69//Z57487
R-NT2RM4002278//Homo sapiens clone RG140B11, WORKING DRAFT SEQUENCE, 1 unordered pieces.//7.5e-49:405:84//AC005069
R-NT2RM4002281//Human DNA sequence *** SEQUENCING IN PROGRESS *** from clone 702J19, WORKING DRAFT SEQUENCE.//1.7e-13:168:77//AL033531
R-NT2RM4002287
R-NT2RM4002294//Homo Sapiens Chromosome X clone bWXD171, WORKING DRAFT SEQUENCE, 1 ordered pieces.//0.98:208:65//AC004676
R-NT2RM4002301//HS_2028_A1_E10_T7 CIT Approved Human Genomic Sperm Library D Homo sapiens genomic clone Plate=2028 Col=19 Row=I, genomic survey sequence.//0.94:321:57//AQ233262
R-NT2RM4002323//Human DNA sequence from clone 59B16 on chromosome 6p22.1-22.3. Contains a pseudogene similar to GPISG20 and other exonucleases). Contains ESTs, STSs, GSSs, genomic markers D6S1691 and D6S299 and a ca repeat polymorphism, complete sequence.//1.9e-35:265:84//AL032822
R-nnnnnnnnnnnn//Human mRNA for KIAA0319 gene, complete cds.//2.4e-42:569:68//AB002317
R-NT2RM4002344//Plasmodium falciparum 3D7 chromosome 12 PFYAC1122 genomic sequence, WORKING DRAFT SEQUENCE, 3 unordered pieces.//0.013:391:59//AC004709
R-NT2RM4002373//Homo sapiens mRNA for KIAA0649 protein, complete cds.//8.6e-121:593:97//AB014549
R-NT2RM4002374//Human DNA sequence from cosmid U131B10, between markers DXS366 and DXS87 on chromosome X contains XK membrane transport protein, ESTs and STS.//3.8e-44:258:86//Z73417
R-NT2RM4002383//Human Chromosome 15q26.1 PAC clone pDJ10k5 containing human DNA polymerase gamma (polg) gene, complete sequence.//0.00084:345:60//AC005316
R-NT2RM4002390
R-NT2RM4002409//RPCI11-45M10.TK RPCI11 Homo sapiens genomic clone R-45M10, genomic survey sequence.//0.99:151:66//AQ194411
R-NT2RM4002438
R-NT2RM4002446//Human DNA sequence from clone 360A4 on chromosome 16. Contains ESTs, complete sequence.//2.8e-103:533:95//AL031008
R-NT2RM4002452
R-NT2RM4002457//Homo sapiens chromosome 16, cosmid clone 321D4 (LANL), complete sequence.//0.99:171:64//AC004034
R-NT2RM4002460//Human DNA sequence from PAC 50A13 on chromosome Xp11. Contains ATP SYNTHASE LIPID BINDING PROTEIN P1 (P2, P3) precursor (ATP5G1, ATP5G2, ATP5G3) like pseudogene, ESTs and STSs. Contains polymorphic CA repeat.//0.96:94:71//Z92545
R-NT2RM4002479//Homo sapiens RNA helicase-related protein mRNA, complete cds.//2.9e-102:508:97//AF083255
R-NT2RM4002482//Homo sapiens mRNA for KIAA0691 protein, complete cds.//7.0e-31:172:98//AB014591
R-NT2RM4002493//CIT-HSP-2296C24.TF CIT-HSP Homo sapiens genomic clone 2296C24, genomic survey sequence.//0.46:182:62//AQ006882
R-NT2RM4002499//Human v-fos transformation effector protein (Fte-1), mRNA complete cds.//7.3e-24:134:99//M84711
R-NT2RM4002504//Homo sapiens Xq28 BAC PAC and cosmid clones containing FMR2 geneexons 1,2, and 3, complete sequence.//3.9e-11:334:63//AC002368
R-nnnnnnnnnnnn
R-NT2RM4002532//Human DNA sequence *** SEQUENCING IN PROGRESS *** from clone 341D10, WORKING DRAFT SEQUENCE.//3.4e-17:171:79//Z97985
R-NT2RM4002534
R-NT2RM4002567//Homo sapiens chromosome 7 clone UWGC:g1564a040 from 7p14-15, complete sequence.//2.2e-26:181:76//AC005271
R-NT2RM4002571
R-NT2RM4002593//CIT-HSP-2303L15.TF CIT-HSP Homo sapiens genomic clone 2303L15, genomic survey sequence.//0.034:73:82//AQ015579
R-NT2RM4002623//Homo sapiens clone UWGC:g1564a209 from 7p14-15, complete sequence.//0.0014:670:55//AC005862
R-NT2RP2000001//Plasmodium falciparum chromosome 2, section 59 of 73 of the complete sequence.//0.00087:251:59//AE001422
R-NT2RP2000006//Human DNA sequence from PAC 155D22 on chromosome 6q27. Contains EST, STSs and a GSS.//2.7e-37:259:86//Z97205
R-NT2RP2000008//RPCI11-41G16.TP RPCI-11 Homo sapiens genomic clone RPCI-11-41G16, genomic survey sequence.//4.1e-25:365:70//AQ029090
R-NT2RP2000027//Homo sapiens chromosome 17, clone HCIT305D20, complete sequence.//6.0e-05:307:62//AC004098
R-NT2RP2000040//Homo sapiens mRNA for KIAA0747 protein, partial cds.//8.4e-41:223:96//AB018290
R-NT2RP2000045//Homo sapiens tumorous imaginal discs protein Tid56 homolog (TID1) mRNA, complete cds.//5.8e-63:325:96//AF061749
R-NT2RP2000054//Human tyrosinase gene, 5'-flanking region (containing enhancer element resposible for pigment cell-specific transcription).//0.88:210:60//D26163
R-NT2RP2000056//Mus musculus epsilon tyrosine phosphatase cytoplasmic isoform (Ptpre) mRNA, complete cds.//4.7e-38:377:78//U36758
R-NT2RP2000067//Rat mRNA for growth potentiating factor, complete cds.//6.0e-10:137:79//D42148
R-NT2RP2000070//Homo sapiens chromosome 5, BAC clone 34j15 (LBNL H169), complete sequence.//3.1e-76:381:98//AC005754
R-NT2RP2000076//Plasmodium falciparum chromosome 2, section 9 of 73 of the complete sequence.//2.3e-06:380:60//AE001372
R-NT2RP2000077//Homo sapiens growth arrest specific 11 (GAS11) mRNA, complete cds.//3.5e-77:379:97+++F050079
R-NT2RP2000079//Human DNA sequence *** SEQUENCING IN PROGRESS *** from clone 1125A11, WORKING DRAFT SEQUENCE.//6.5e-32:314:78//AL034549
R-NT2RP2000088//Homo sapiens mRNA for KIAA0795 protein, partial cds.//5.6e-74:378:96//AB018338
R-NT2RP2000091//Homo sapiens clone RG015P03, complete sequence.//9.3e-21:226:76//AC005048
R-NT2RP2000097//Human DNA sequence from cosmid U209G1 on chromosome X.//9.2e-40:278:81//Z68873
R-NT2RP2000098//Human BAC clone RG333F24 from 7q11.2-q21, complete sequence.//0.34:132:65//AC004015
R-NT2RP2000108//Homo sapiens DNA from chromosome 19, BAC 33152, complete sequence.//3.1e-09:259:67//AC003973
R-NT2RP2000114//Homo sapiens mRNA for GM3 synthase, complete cds.//1.8e-74:386:95//AB018356
R-NT2RP2000120//CITBI-E1-2503M8.TR CITBI-E1 Homo sapiens genomic clone 2503M8, genomic survey sequence.//5.1e-05:87:77//AQ263909
R-nnnnnnnnnnnn
R-nnnnnnnnnnnn//Homo sapiens PAC clone DJ044L15 from Xq23, complete sequence.//4.9e-11:153:69//AC004827
R-NT2RP2000147
R-NT2RP2000153//Homo sapiens ccr2b (ccr2), ccr2a (ccr2), ccr5 (ccr5) and ccr6 (ccr6) genes, complete cds, and lactoferrin (lactoferrin) gene, partial cds, complete sequence.//0.0058:261:57//U95626
R-NT2RP2000157//Homo sapiens Chr.14 PAC RPCI4-794B2 (Roswell Park Cancer Institute Human PAC Library) complete sequence.//2.5e-119:603:96//AC005924
R-NT2RP2000161//CIT-HSP-2045P7.TR CIT-HSP Homo sapiens genomic clone 2045P7, genomic survey sequence.//0.89:173:63//B79728
R-NT2RP2000175
R-NT2RP2000183
R-NT2RP2000195//Homo sapiens chromosome 17, clone hRPK.60_A_24, complete sequence.//4.3e-39:306:83//AC005325
R-NT2RP2000205//Human DNA sequence from clone 302L24 on chromosome Xq21-22, complete sequence.//7.5e-05:101:78//AL022155
R-NT2RP2000224//Homo sapiens Chromosome 16 BAC clone CIT987SK-A-152E5, complete sequence.//7.3e-55:306:94//AC004382
R-NT2RP2000232
R-NT2RP2000233//Mus musculus tumor metastasis associated gene product (MAG) mRNA, complete cds.//7.6e-13:144:75//U88401
R-NT2RP2000239//Homo sapiens chromosome 4 clone B353C18 map 4q25, complete sequence.//9.6e-63:410:86//AC004066
R-NT2RP2000248//Caenorhabditis elegans cosmid T01C8.//1.0:282:58//U58726
R-NT2RP2000257//Homo sapiens PAC clone DJ0808G16 from 7q11.23-q21, complete sequence.//2.5e-11:163:72//AC004894
R-NT2RP2000258//Arabidopsis thaliana chromosome II BAC T31E10 genomic sequence, complete sequence.//0.58:442:58//AC004077
R-NT2RP2000270//Homo sapiens DNA sequence from PAC 97D16 on chromosome 6p21.3-22.2. Contains an unknown pseudogene, a 60S Ribosomal protein L24 (L30) LIKE pseudogene and histone genes H2BFC (H2B/c), H4FFP (H4/f pseudogene), H2AFC (H2A/c), H3F1K (H3.1/k) and a tRNA-Val pseudogene and tRNA-Thr gene. Contains ESTs, STSs, GSSs and genomic marker D6S464, complete sequence.//1.1e-39:292:84//AL009179
R-NT2RP2000274//CIT-HSP-237901.TR CIT-HSP Homo sapiens genomic clone 237901, genomic survey sequence.//6.9e-10:121:81//AQ109409
R-NT2RP2000288
R-NT2RP2000289
R-NT2RP2000297//Homo sapiens full-length insert cDNA clone ZB81C03.//7.7e-109:519:99//AF086165
R-NT2RP2000298
R-NT2RP2000310//Homo sapiens p53 induced protein mRNA, partial cds.//1.5e-38:224:93//AF010310
R-NT2RP2000327//Homo sapiens DNA sequence from PAC 434O14 on chromosome 1q32.3.-41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two novel genes. Contains ESTs and GSSs, complete sequence.//4.3e-113:580:96//AL022398
R-NT2RP2000329//Homo sapiens clone NH0319F03, WORKING DRAFT SEQUENCE, 3 unordered pieces.//7.4e-47:367:77//AC006039
R-NT2RP2000337//Anopheles quadrimaculatus NADH dehydrogenase subunits (1-4, 4L, 5-6); cytochrome oxidase subunits (1-3); adenosine triphosphatase subunits (6,8); cytochrome b; transfer RNA; ribosomal RNA (large and small subunits).//4.9e-08:494:58//L04272

R-NT2RP2000346//Homo sapiens apoptosis associated protein (GADD34) mRNA, complete cds.//3.4e-46:262:94//U83981
R-NT2RP2000369//Homo sapiens chromosome 17, clone HCIT169H9, WORKING DRAFT SEQUENCE, 6 unordered pieces.//3.0e-07:334:61//AC002993
R-NT2RP2000414//Mouse DNA sequence *** SEQUENCING IN PROGRESS *** from clone BAC394, WORKING DRAFT SEQUENCE.//7.0e-08:98:83//AJ004828
R-NT2RP2000420//Homo sapiens chromosome 17, clone hRPK.640_I_15, complete sequence.//0.99:150:62//AC005324
R-NT2RP2000422//Homo sapiens N-acetylglucosamine-phosphate mutase mRNA, complete cds.//4.6e-19:142:90//AF102265
R-NT2RP2000438//RPCI11-62I13.TK RPCI11 Homo sapiens genomic clone R-62I13, genomic survey sequence.//3.1e-06:103:79//AQ199572
R-NT2RP2000448//Homo sapiens PAC clone DJ0740D02 from 7p14-p15, complete sequence.//2.0e-22:276:73//AC004691
R-NT2RP2000459//CIT-HSP-2013N9.TR CIT-HSP Homo sapiens genomic clone 2013N9, genomic survey sequence.//5.5e-27:205:87//853940
R-NT2RP2000498//Homo sapiens Chromosome 11q23 PAC clone pDJ149k2 containing PLZF gene encoding kruppel-like zinc finger protein, complete sequence.//6.0e-12:119:84//AC001234
R-NT2RP2000503//Human CYP11B2 gene for steroid 18-hydroxylase (P-450 C18), 5'-flanking region and exon 1.//0.48:201:64//D10170
R-NT2RP2000510//Bactrocera dorsalis strain Tahiti mitochondrial D-loop region, complete sequence.//3.6e-07:472:59//AF033929
R-nnnnnnnnnnnn
R-NT2RP2000523//Human DNA sequence *** SEQUENCING IN PROGRESS *** from clone 150C2, WORKING DRAFT SEQUENCE.//2.3e-61:317:97//AL022318
R-NT2RP2000603//Homo sapiens mRNA for MCM3 import factor, complete cds.//6.6e-29:167:97//AB005543
R-NT2RP2000617
R-NT2RP2000634//Homo sapiens mRNA for KIAA0614 protein, partial cds.//2.5e-64:335:96//AB014514
R-NT2RP2000644//Human DNA sequence from PAC 50A13 on chromosome Xp11. Contains ATP SYNTHASE LIPID BINDING PROTEIN P1 (P2, P3) precursor (ATP5G1, ATP5G2, ATP5G3) like pseudogene, ESTs and STSs. Contains polymorphic CA repeat.//1.8e-28:383:70//Z92545
R-NT2RP2000656//Homo sapiens DNA sequence from PAC 874C20 on chromosome 6p22.1-22.3. Contains a Zinc Finger Protein ZFP47 LIKE gene, a Zinc Finger Protein pseudogene and a Zinc Finger Protein SRE-ZBP pseudogene. Contains ESTs, STSs and GSSs, complete sequence.//0.0093:110:70//AL021997
R-NT2RP2000658//Bacillus thuringiensis chitinase (chi) gene, complete cds.//0.73:301:60//U89796
R-NT2RP2000668
R-NT2RP2000678//Homo sapiens genomic DNA, chromosome 21q22.2 (Down Syndrome region), segment 8/15, WORKING DRAFT SEQUENCE.//2.8e-11:256:66//AP000015
R-NT2RP2000710//Genomic sequence from Human 17, WORKING DRAFT SEQUENCE, 9 unordered pieces.//0.036:176:69//AC002346
R-NT2RP2000715//Homo sapiens PAC clone DJ1066K24 from 7p15, complete sequence.//2.7e-110:555:96//AC004540
R-NT2RP2000731//Human DNA sequence from clone 497J21 on chromosome 6q26-27. Contains a KOC (KH-domain containg transcript overexpressed in cancer) pseudogene, genomic marker D6S193, ESTs, STSs and GSSs, and a ca repeat polymorphism, complete sequence.//2.6e-18:319:68//AL023775
R-NT2RP2000758//CIT-HSP-507A14.TP CIT-HSP Homo sapiens genomic clone 507A14, genomic survey sequence.//1.0:189:60//B50590
R-NT2RP2000764
R-NT2RP2000809//Human BAC clone RG356F09 from 7p21, complete sequence.//1.7e-24:215:81//AC004002
R-NT2RP2000812//CIT-HSP-2281C3.TR CIT-HSP Homo sapiens genomic clone 2281C3, genomic survey sequence.//9.5e-32:176:97//B99575
R-nnnnnnnnnnnn//paramecium species 5,87 mt dna dimer: replication init. region.//0.0077:418:57//K00916
R-NT2RP2000816//F.rubripes GSS sequence, clone 011H02aA6, genomic survey sequence.//0.61:52:73//AL011013
R-NT2RP2000819
R-NT2RP2000841//Human DNA sequence *** SEQUENCING IN PROGRESS *** from clone 43408, WORKING DRAFT SEQUENCE.//0.00012:181:70//AL033504
R-NT2RP2000842//Mus musculus (C57BL/10 X C3H)F2 clone 4.9 novel mRNA from reninexpressing kidney tumor cell line, partial sequence.//3.7e-27:388:72//U13370
R-NT2RP2000845//Homo sapiens chromosome 17, clone hRPK.849_N_15, complete sequence.//0.0022:200:68//AC005703
R-NT2RP2000863
R-NT2RP2000880//Homo sapiens mRNA for putative GTP-binding protein, partial.//2.3e-43:279:89//AJ006412
R-NT2RP2000892//Homo sapiens genomic DNA of 9q32 anti-oncogene of flat epitherium cancer , segment 7/10.//0.0028:221:62//AB020875
R-NT2RP2000931//Homo sapiens mRNA for KIAA0723 protein, complete cds.//2.2e-55:290:96//AB018266
R-NT2RP2000938//Homo sapiens full-length insert cDNA clone ZD55G12.//2.1e-37:215:93//AF086336
R-NT2RP2000943//Homo sapiens mRNA for KIAA0755 protein, complete cds.//3.0e-96:494:96//AB018298
R-NT2RP2000965
R-NT2RP2000970//Homo sapiens DNA sequence from BAC 747E2 on chromosome 22q12.1. Contains ESTs, STSs and GSSs and genomic marker D22S56, complete sequence.//4.5e-87:440:97//AL021393
R-NT2RP2000985//Homo sapiens chromosome 17, clone hRPK.597_M_12, complete sequence.//5.4e-93:484:95//AC005277
R-NT2RP2000987//Plasmodium falciparum chromosome 2, section 9 of 73 of the complete sequence.//2.1e-06:318:62//AE001372
R-NT2RP2001036//Human DNA sequence *** SEQUENCING IN PROGRESS *** from clone 410I8, WORKING DRAFT SEQUENCE.//2.0e-24:273:73//AL031732
R-NT2RP2001044//Plasmodium falciparum 3D7 chromosome 12 PFYACB8-420 genomic sequence, WORKING DRAFT SEQUENCE, 14 unordered pieces.//3.3e-07:365:65//AC005140
R-NT2RP2001065//Caenorhabditis elegans cosmid F10G7.//9.2e-06:273:59//U40029
R-NT2RP2001070//CITBI-E1-2503F4.TF CITBI-E1 Homo sapiens genomic clone 2503F4, genomic survey sequence.//0.13:97:72//AQ265973
R-NT2RP2001094//Mycoplasma mycoides mycoides SC immunodominant protein P72 (p72) gene, complete cds, mannitol-1-phosphate dehydrogenase (mt1D) gene, partial cds and insertion sequence IS1296, complete sequence.//0.018:373:57//U61140
R-NT2RP2001119
R-NT2RP2001127//Homo sapiens HRIHFB2060 mRNA, partial cds.//4.5e-55:304:94//AB015348
R-NT2RP2001137//Homo sapiens DNA sequence from clone 511B24 on chromosome 20q11.2-12. Contains the TOP1 gene for Topoisomerase I, the PLCG1 gene for 1-Phosphatidylinositol-4,5-Bisphosphate Phosphodiesterase Gamma 1 (EC 3.1.4.11, PLC-Gamma-1, Phospholipase C-Gamma-1 PLC-II, PLC-148), the KIAA0395 gene for a probable Zinc Finger Homeobox protein and a 60S Ribosomal Protein L23 LIKE pseudogene. Contains a predicted CpG island, ESTs, STSs and GSSs, complete sequence.//0.69:129:65//AL022394
R-NT2RP2001149//Sequence 5 from Patent US 4798885.//8.5e-28:322:77//I01838
R-NT2RP2001168
R-NT2RP2001173//Homo sapiens mRNA for KIAA0480 protein, complete cds.//4.8e-95:490:96//AB 007949
R-NT2RP2001174//CIT-HSP-2170B18.TR CIT-HSP Homo sapiens genomic clone 2170B18, genomic survey sequence.//1.3e-33:204:93//B89680
R-NT2RP2001196//Plasmodium falciparum DNA *** SEQUENCING IN PROGRESS *** from contig 3-65, complete sequence.//1.7e-06:413:61//AL010134
R-NT2RP2001218//Human DNA sequence from clone 23K20 on chromosome Xq25-26.2 Contains EST, STS, GSS, complete sequence.//8.5e-15:278:68//AL022153
R-NT2RP2001226//Human DNA sequence from clone 1170D6 on chromosome Xq22.3-23. Contains a pseudogene similar to U-SNRNP associated Cyclophilin (USA-CYP, EC 5.2.1.8), ESTs, an STS and a GSS, complete sequence.//0.0020:462:57//AL030995
R-NT2RP2001233//CIT-HSP-2356P23.TR CIT-HSP Homo sapiens genomic clone 2356P23, genomic survey sequence.//8.0e-108:547:96//AQ081110
R-NT2RP2001245//Spodoptera frugiperda 16S rRNA gene, Val-tRNA, and Leu-tRNA genes, and ND-1 protein gene, 5' end.//0.0052:350:58//M76713
R-NT2RP2001268//Homo sapiens mRNA for KIAA0810 protein, partial cds.//4.6e-111:544:97//AB018353
R-NT2RP2001277//Caenorhabditis elegans DNA *** SEQUENCING IN PROGRESS *** from clone Y59A8, WORKING DRAFT SEQUENCE.//0.0058:327:59//Z98870
R-NT2RP2001290//Plasmodium falciparum 3D7 chromosome 12 PFYAC1122 genomic sequence, WORKING DRAFT SEQUENCE, 3 unordered pieces.//0.96:187:65//AC004709
R-NT2RP2001295//Homo sapiens BAC clone NH0491B03 from 7p21-p15, complete sequence.//0.59:218:62//AC006041
R-NT2RP2001312//Human DNA sequence *** SEQUENCING IN PROGRESS *** from clone 349A12, WORKING DRAFT SEQUENCE.//0.12:117:64//AL033520
R-NT2RP2001327//Caenorhabditis elegans cosmid R04D3, complete sequence.//0.31:119:66//Z70212
R-NT2RP2001328//HS_2213_A1_D07_MF CIT Approved Human Genomic Sperm Library D Homo sapiens genomic clone Plate=2213 Col=13 Row=G, genomic survey sequence.//1.7e-22:200:83//AQ136874
R-NT2RP2001347//Plasmodium falciparum MAL3P8, complete sequence.//0.81:509:56//AL034560
R-NT2RP2001378//H.sapiens DNA sequence.//0.94:147:63//Z22404
R-NT2RP2001381//Homo sapiens cyclin E2 mRNA, complete cds.//3.2e-09:75:97//AF091433
R-NT2RP2001392//Myxococcus xanthus ATP-dependent protease (bsgA) gene, complete cds.//0.079:178:62//L19301
R-NT2RP2001394//Human DNA sequence from PAC 389A20 on chromosome X contains ESTs STS, CpG islands and polymorphic CA repeat.//3.4e-60:351:90//Z93242
R-NT2RP2001397//Hamster mRNA for cyclinB2, complete cds.//5.4e-55:320:83//D17294
R-NT2RP2001420//Human DNA sequence *** SEQUENCING IN PROGRESS *** from clone 1108D11, WORKING DRAFT SEQUENCE.//1.0e-44:246:85//AL034419
R-NT2RP2001423//Human DNA sequence from clone 726F20 on chromosome 1p36.11-36.23. Contains ESTs and a GSS, complete sequence.//3.7e-05:417:61//AL031273 R-NT2RP2001427//Human Chromosome 11 Cosmid cSRL34e5, complete sequence.//0.94:287:59//U73643
R-NT2RP2001436//Mus musculus clone OST1784, genomic survey sequence.//5.2e-31:299:77//AF046702
R-NT2RP2001440//Rattus norvegicus mRNA for 14-3-3 protein gamma-subtype, complete cds.//7.8e-75:548:83//D17447
R-NT2RP2001445//Homo sapiens 12q13.1 PAC RPCI1-228P16 (Roswell Park Cancer Institute Human PAC Library) complete sequence.//1.0e-06:452:59//AC004801
R-NT2RP2001449//Homo sapiens clone DJ0647C14, WORKING DRAFT SEQUENCE, 21 unordered pieces.//5.1e-08:218:67//AC004846
R-NT2RP2001450
R-NT2RP2001467//Human BAC clone RG343P13 from 7q31, complete sequence.//3.8e-31:254:83//AC002465
R-NT2RP2001506//C.barati p-47, ntnh, bonT genes.//1.2e-06:415:60//Y12091
R-NT2RP2001511//Plasmodium falciparum MAL3P7, complete sequence.//0.11:155:63//AL034559
R-NT2RP2001520//Homo sapiens mRNA for mitochondrial carrier protein ARALAR1.//2.1e-104:545:95//Y14494
R-NT2RP2001526//Homo sapiens chromosome 17, clone hCIT.175_E_5, complete sequence.//7.0e-16:283:68//AC004596
R-NT2RP2001536//Human DNA from chromosome 14-specific cosmid containing XRCC3 DNA repair gene, genomic sequence, complete sequence.//7.7e-16:108:96//AF037222
R-NT2RP2001560//CIT978SK-A-56H4.TP CIT978SK Homo sapiens genomic clone A-56H4, genomic survey sequence.//0.052:112:66//B73597
R-NT2RP2001569//CIT-HSP-2335F8.TF CIT-HSP Homo sapiens genomic clone 2335F8, genomic survey sequence.//6.0e-78:383:98//AQ042029
R-NT2RP2001576//Homo sapiens sulfonylurea receptor (SUR2) gene, exon 37.//0.33:135:66//AF061322
R-NT2RP2001581//Homo sapiens (clone MFD220) PCR primer.//2.7e-07:240:63//L15407
R-NT2RP2001597//HS_3016_B2_F06_T7 CIT Approved Human Genomic Sperm Library D Homo sapiens genomic clone Plate=3016 Col=12 Row=L, genomic survey sequence.//5.3e-45:310:87//AQ118854
R-NT2RP2001601//Homo sapiens chromosome 17, clone hRPK.855_D_21, complete sequence.//0.015:445:58//AC006079
R-NT2RP2001613//Mus musculus orphan nuclear hormone receptor (CAR) gene, complete sequence.//3.5e-16:413:63//AF009326
R-NT2RP2001628//Phytomonas serpens kinetoplast maxicircle ribosomal protein S12 (G6) edited mRNA, complete cds.//0.11:190:63//AF034626
R-NT2RP2001663//Homo sapiens Chromosome 16 BAC clone CIT987SK-625P11, complete sequence.//3.0e-26:157:81//AC004125
R-NT2RP2001677//Homo sapiens chromosome 9, P1 clone 11659, complete sequence.//3.0e-58:305:96//AC004472
R-NT2RP2001678//Human BAC clone RG222A16 from 7q31, complete sequence.//0.95:107:66//AC002385
R-NT2RP2001699//Mus musculus erythroid ankyrin and two alternatively spliced erythroid ankyrins (Ank1) gene, putative exon 41 and partial cds.//8.8e-05:211:63//U76758
R-NT2RP2001720//Homo sapiens PAC clone DJ0167F23 from 7p15, complete sequence.//4.7e-68:352:97//AC004079
R-NT2RP2001721//HS-1052-B1-G06-MF.abi CIT Human Genomic Sperm Library C Homo sapiens genomic clone Plate=CT 774 Col=11 Row=N, genomic survey sequence.//7.7e-05:346:59//B40914
R-NT2RP2001740//HS_3213_A2_D02_T7 CIT Approved Human Genomic Sperm Library D Homo sapiens genomic clone Plate=3213 Col=4 Row=G, genomic survey sequence.//1.1e-16:162:82//AQ175104
R-NT2RP2001748//Human gene for L-histidine decarboxylase, complete cds.//2.0e-33:312:77//D16583
R-NT2RP2001762//Homo sapiens chromosome 1, BAC CIT-HSP-292g8 (BC262482), complete sequence.//2.3e-100:435:97//AC004783
R-NT2RP2001813//Human leukocyte common antigen T200 (CD45, LCA) gene, exon 9.//0.031:261:60//M23468
R-NT2RP2001861
R-NT2RP2001869//Sequence 5 from patent US 5595900.//4.2e-21:194:77//I34189
R-NT2RP2001876
R-NT2RP2001883//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//5.0e-111:485:97//AL031864
R-NT2RP2001900
R-NT2RP2001907//Human proto-oncogene tyrosine-protein kinase (ABL) gene, exon 1a and exons 2-10, complete cds.//5.4e-42:382:77//U07563
R-NT2RP2001926//HS_3180_B2_F02_MR CIT Approved Human Genomic Sperm Library D Homo sapiens genomic clone Plate=3180 Col=4 Row=L, genomic survey sequence.//2.8e-25:138:80//AQ185415
R-NT2RP2001936//Plasmodium falciparum 3D7 chromosome 12 PFYAC1383 genomic sequence, WORKING DRAFT SEQUENCE, 3 unordered pieces.//1.0:320:60//AC005504
R-NT2RP2001943//Dictyostelium discoideum PkgA (pkgA) gene, partial cds.//1.4e-08:378:59//AF020280
R-NT2RP2001946//Homo sapiens clone NH0140K04, complete sequence.//3.6e-85:409:100//AC005033
R-NT2RP2001947//Human mRNA for KIAA0390 gene, complete cds.//0.85:140:64//AB002388
R-NT2RP2001969
R-NT2RP2001976//CIT-HSP-2281C3.TR CIT-HSP Homo sapiens genomic clone 2281C3, genomic survey sequence.//2.0e-60:307:98//B99575
R-NT2RP2001985//Arabidopsis thaliana DNA chromosome 4, BAC clone F1N20 (ESSAII project).//0.031:282:61//AL022140
R-NT2RP2002025
R-NT2RP2002032//CITBI-E1-2502C19.TF CITBI-E1 Homo sapiens genomic clone 2502C19, genomic survey sequence.//1.2e-52:285:95//AQ264715
R-NT2RP2002033//Human (lambda) DNA for immunoglobin light chain.//1.1e-08:389:61//D88270
R-NT2RP2002041//Homo sapiens 12p13.3 BAC RPCI11-319E16 (Roswell Park Cancer Institute Human BAC Library) complete sequence.//1.1e-49:264:97//AC006206
R-NT2RP2002046//Human BAC clone GS119P05 from 7q21, complete sequence.//0.0023:429:61//AC004011
R-NT2RP2002047//P.falciparum PK1 gene.//0.00015:239:62//X83707
R-NT2RP2002058//HS_2183_A1_G01_MR CIT Approved Human Genomic Sperm Library D Homo sapiens genomic clone Plate=2183 Col=1 Row=M, genomic survey sequence.//1.2e-21:185:84//AQ022560
R-NT2RP2002066//G.gallus microsatellite DNA (LEI0222 (=T15ivD04)).//0.18:102:70//Z83792
R-NT2RP2002070//P.falciparum major merozoite surface antigen (PMMSA) mRNA, complete cds, isolate FC27.//0.95:192:61//M19143
R-NT2RP2002076//Homo sapiens clone 24804 mRNA sequence.//3.8e-25:182:86//AF052183
R-NT2RP2002079//Human DNA sequence from clone 431P23 on chromosome 6q27. Contains the first coding exon of the MLLT4 gene for myeloid/lymphoid or mixed-lineage leukemia (trithorax (Drosophila) homolog); translocated to, 4 (AF-6, Afadin, MLLT-4, ALL-1 fusion partner), and a Serine Palmitoyltransferase 2 (EC 2.3.1.50, Long Chain Base Biosynthesis protein 2, LCB-2, SPT-2) pseudogene. Contains ESTs, STss, GSSs, and a putative CpG island, complete sequence.//1.7e-10:97:90//AL009178
R-NT2RP2002099//Homo sapiens mRNA for E1B-55kDa-associated protein.//4.6e-59:376:89//AJ007509
R-NT2RP2002105
R-NT2RP2002124//RPCI11-75J16.TJ RPCI11 Homo sapiens genomic clone R-75J16, genomic survey sequence.//0.58:191:64//AQ266779
R-NT2RP2002137//Homo sapiens Xp22-175-176 BAC GSHB-484O17 (Genome Systems Human BAC Library) complete sequence.//0.0065:294:61//AC005913
R-NT2RP2002154
R-NT2RP2002172//RPCI11-90C20.TJ RPCI11 Homo sapiens genomic clone R-90C20, genomic survey sequence.//0.049:160:65//AQ282591
R-NT2RP2002185//CIT-HSP-2341I15.TF CIT-HSP Homo sapiens genomic clone 2341I15, genomic survey sequence.//6.0e-36:230:90//AQ053355
R-NT2RP2002192//HS_2222_B1_F08_MF CIT Approved Human Genomic Sperm Library D Homo sapiens genomic clone Plate=2222 Col=15 Row=L, genomic survey sequence.//1.9e-15:249:71//AQ178491
R-NT2RP2002193//Rattus norvegicus potassium channel regulatory protein KChAP mRNA, complete cds.//4.7e-35:438:73//AF032872
R-NT2RP2002208//Hansenula wingei mitochondrial DNA, complete sequence.//0.00057:468:57//D31785
R-NT2RP2002219//HS_2058_A1_C09_MR CIT Approved Human Genomic Sperm Library D Homo sapiens genomic clone Plate=2058 Col=17 Row=E, genomic survey sequence.//3.4e-55:512:77//AQ234380
R-NT2RP2002231//Plasmodium falciparum DNA *** SEQUENCING IN PROGRESS *** from contig 3-31, complete sequence.//1.5e-06:398:61//Z98557
R-nnnnnnnnnnnn//Sequence 11 from patent US 5624818.//3.3e-91:553:87//I41141
R-NT2RP2002256//Homo sapiens retinoic acid hydroxylase mRNA, complete cds.//3.0e14:132:84//AF005418
R-NT2RP2002259//Human DNA sequence *** SEQUENCING IN PROGRESS *** from clone 118J21, WORKING DRAFT SEQUENCE.//1.6e-96:548:91//AL033527
R-NT2RP2002270//Homo sapiens Chromosome 16 BAC clone CIT987SK-A-248F7, complete sequence.//5.1e-06:391:60//AC004605
R-NT2RP2002292//Genomic sequence from Human 13, complete sequence.//0.91:159:64//AC001226
R-NT2RP2002312//Homo sapiens CDP-diacylglycerol synthase 2 (CDS2) mRNA, partial cds.//1.3e-101:527:94//AF069532
R-NT2RP2002316//Plasmodium falciparum chromosome 2, section 45 of 73 of the complete sequence.//0.00052:389:59//AE001408
R-NT2RP2002325//Homo sapiens peroxisomal biogenesis factor (PEX11a) mRNA, complete cds.//2.3e-112:567:95//AF093668
R-NT2RP2002333//Rat POU domain factor (Brn-5) mRNA.//1.5e-22:323:73//L23204
R-NT2RP2002385//Homo sapiens synaptic glycoprotein SC2 spliced variant mRNA, complete cds.//3.7e-102:600:89//AF038958
R-NT2RP2002394//Plasmodium falciparum 3D7 chromosome 12 PFYAC492 genomic sequence, WORKING DRAFT SEQUENCE, 5 unordered pieces.//0.039:399:59//AC005308
R-NT2RP2002408//HS_2212_A1_E09_MR CIT Approved Human Genomic Sperm Library D Homo sapiens genomic clone Plate=2212 Col=17 Row=I, genomic survey sequence.//9.6e-35:231:88//AQ184632
R-NT2RP2002426//Human DNA sequence from clone 101G11 on chromosome 22q12. Contains an ACO2 (Mitochondrial Aconitate Hydratase (Aconitase, Citrate Hydro-Lyase, EC 4.2.1.3)) pseudogene, ESTs, STSs, GSSs and a putative CpG island, complete sequence.//2.8e-39:308:82//AL021877
R-NT2RP2002439//Leishmania tarentolae mitochondrial electron transport chain component mRNA.//0.022:102:71//M74225
R-NT2RP2002457//Homo sapiens DNA sequence from PAC 142L7 on chromosome 6q21. Contains a Laminin Alpha 4 (LAMA4) LIKE gene coding for two alternatively spliced transcripts, a Tubulin Beta LIKE pseudogene, a Connective tissue growth factor (NOV, GIG) LIKE gene, A predicted CpG island, ESTs, STSs and genomic marker D6S416, complete sequence.//0.00099:354:59//Z99289
R-NT2RP2002464//Homo sapiens genomic DNA, chromosome 21q22.2 (Down Syndrome region), segment 6/15, WORKING DRAFT SEQUENCE.//0.0015:219:67//AP000013
R-NT2RP2002475
R-nnnnnnnnnnnn//Homo sapiens mRNA for ABC transporter 7 protein, complete cds.//3.1e-113:605:92//AB005289
R-NT2RP2002498//Human DNA sequence from PAC 162H14 on chromosome 22. Contains 3' part of a FIBULIN 1 like gene and ESTs, complete sequence.//0.32:210:64//Z98047
R-NT2RP2002503//Homo sapiens, clone hRPK.15_A_1, complete sequence.//4.0e-86:429:98//AC006213
R-NT2RP2002504//Homo sapiens mRNA for KIAA0791 protein, complete cds.//2.7e-105:583:91//AB018334
R-NT2RP2002520//Saccharomyces cerevisiae mitochondrial tRNA-Tyr, tRNA-Asn, & amp; tRNA-Met genes.//0.14:406:58//AJ223323
R-NT2RP2002537//Human DNA sequence *** SEQUENCING IN PROGRESS *** from clone 500L14, WORKING DRAFT SEQUENCE.//2.8e-16:188:78//AL023583
R-NT2RP2002546//Homo sapiens clone TUA8 Cri-du-chat region mRNA.//4.7e-108:571:93//AF009314
R-NT2RP2002549//Human Chromosome 15q26.1 PAC clone pDJ10k5 containing human DNA polymerase gamma (polg) gene, complete sequence.//1.1e-103:422:95//AC005316
R-NT2RP2002591//Human DNA binding protein (HPF2) mRNA, complete cds.//1.8e-36:526:67//M27878
R-NT2RP2002595
R-NT2RP2002606//Human DNA sequence *** SEQUENCING IN PROGRESS *** from clone 2705, WORKING DRAFT SEQUENCE.//7.2e-10:211:71//AL033529
R-NT2RP2002609
R-NT2RP2002618//Plasmodium falciparum MAL3P6, complete sequence.//2.9e-05:566:60//Z98551
R-NT2RP2002621//Human DNA sequence from PAC 341I10 on chromosome 6q22.2-22.33. Contains 60S ribosomal protein L5 like (pseudo)gene, ESTs and STSs.//1.1e-38:348:78//Z97352
R-NT2RP2002643//Homo sapiens chromosome 11 clone pTWB15.28 map 11p15.4-p15.5, genomic survey sequence.//1.2e-35:414:66//AF074030
R-NT2RP2002672//Homo sapiens chromosome 10 clone CIT-HSP-1326H7 map 10q24.3-10q25.1, complete sequence.//1.3e-77:403:95//AC005384
R-NT2RP2002701
R-NT2RP2002706//Homo sapiens chromosome 19, cosmid F22676, complete sequence.//4.0e-42:147:90//AC005778
R-NT2RP2002710//P.falciparum serine rich protein (SERP I) gene.//0.84:135:67//J03983
R-NT2RP2002727//, complete sequence.//1.0:363:59//AC005815
R-NT2RP2002736//Arabidopsis thaliana chromosome II BAC T17M13 genomic sequence, complete sequence.//0.44:267:60//AC004138
R-NT2RP2002740//Homo sapiens Xp22 BAC GSHB-600G8 (Genome Systems Human BAC library) complete sequence.//0.0016:474:60//AC004674
R-NT2RP2002741//HS_3051_B1_H11_MF CIT Approved Human Genomic Sperm Library D Homo sapiens genomic clone Plate=3051 Col=21 Row=P, genomic survey sequence.//1.1e-38:217:86//AQ106283
R-NT2RP2002750//Homo sapiens 12q24.1 PAC RPCI1-315L5 (Roswell Park Cancer Institute Human PAC library) complete sequence.//5.0e-36:430:75//AC002395
R-NT2RP2002752//Human DNA sequence *** SEQUENCING IN PROGRESS *** from clone 366L4, WORKING DRAFT SEQUENCE.//8.2e-41:437:76//AL023494
R-NT2RP2002753//Homo sapiens clone DJ076B20, WORKING DRAFT SEQUENCE, 6 unordered pieces.//6.8e-100:496:97//AC004882
R-NT2RP2002769//paramecium species 5,311 mt dna dimer: replication init. region.//7.4e-10:404:60//K00917
R-NT2RP2002778//Homo sapiens clone 24606 mRNA sequence.//1.2e-63:341:94//AF070537
R-NT2RP2002800//RPCI11-37G8.TV RPCI-11 Homo sapiens genomic clone RPCI-11-37G8, genomic survey sequence.//4.9e-60:321:95//AQ029850
R-NT2RP2002839//Homo sapiens Chromosome 11q12.2 PAC clone pDJ688p12 containing uteroglobin gene, WORKING DRAFT SEQUENCE, 11 unordered pieces.//2.9e-100:492:98//AC006078
R-NT2RP2002857//HS_3026_B2_H07_MR CIT Approved Human Genomic Sperm Library D Homo sapiens genomic clone Plate=3026 Col=14 Row=P, genomic survey sequence.//8.9e-06:242:62//AQ 128697
R-NT2RP2002862//RPCI11-42I15.TJ RPCI11 Homo sapiens genomic clone R-42I15, genomic survey sequence.//1.5e-44:270:85//AQ052700
R-NT2RP2002880//Human DNA sequence *** SEQUENCING IN PROGRESS *** from clone 150C2, WORKING DRAFT SEQUENCE.//1.0:295:58//AL022318
R-NT2RP2002891
R-NT2RP2002925//Human DNA sequence *** SEQUENCING IN PROGRESS *** from clone 243L18, WORKING DRAFT SEQUENCE.//2.0e-24:395:67//AL034395
R-NT2RP2002928//Plasmodium falciparum MAL3P5, complete sequence.//0.044:461:55//AL034556
R-NT2RP2002929//Plasmodium falciparum 3D7 chromosome 12 PFYACB8-420 genomic sequence, WORKING DRAFT SEQUENCE, 14 unordered pieces.//0.35:491:56//AC005140
R-NT2RP2002954//Homo sapiens chromosome 17, clone hRPK.628_E_12, complete sequence.//1.0:275:61//AC005701
R-NT2RP2002959//Mus musculus ubiquitin conjugating enzyme (ubc4) mRNA, complete cds.//2.7e-61:508:79//U62483
R-NT2RP2002979//RPCI11-20F13.TPK RPCI-11 Homo sapiens genomic clone RPCI-11-20F13, genomic survey sequence.//0.88:110:72//AQ008132
R-NT2RP2002980//Homo sapiens PAC clone DJ0841B21 from 7q21.1-q31.1, complete sequence.//1.1e-102:433:95//AC004140
R-NT2RP2002986//Human DNA sequence from clone 1147O16 on chromosome Xp21.1-21.3. Contains 13 exons of the DMD muscular dystrophy gene. Contains an STS and GSSs, complete sequence.//0.31:219:62//AL031542
R-NT2RP2002987//Homo sapiens chromosome 18, clone hRPK.24_A_23, complete sequence.//1.3e-51:283:88//AC005968
R-NT2RP2002993//Human DNA sequence from PAC 106B9 on chromosome Xq21://4.3e-11:430:63//AL021307
R-NT2RP2003000//Saccharomyces cerevisiae mitochondrion transfer RNA- Leu, Gln, Lys, Arg, Gly, Asp, Ser2, Arg2, Ala, Ile, Tyr, Asn genes.//0.00088:347:62//L36887
R-NT2RP2003034//Homo sapiens genomic DNA of 9q32 anti-oncogene of flat epitherium cancer, segment 2/10.//3.5e-33:271:82//AB020870
R-NT2RP2003073
R-NT2RP2003099//Homo sapiens PAC clone DJ0886O08 from 7q32-q35, complete sequence.//1.5e-45:548:69//AC004914
R-NT2RP2003108
R-NT2RP2003117//Homo sapiens clone DJ1137M13, complete sequence.//2.0e-51:323:88//AC005378
R-NT2RP2003121//HS_2238_A1_E08_MF CIT Approved Human Genomic Sperm Library D Homo sapiens genomic clone Plate=2238 Col=15 Row=I, genomic survey sequence.//0.00055:324:61//AQ293058
R-NT2RP2003125
R-NT2RP2003129
R-NT2RP2003137//Human BAC clone RG084D04 from 7q31, complete sequence.//1.1e-46:521:74//AC003084
R-NT2RP2003161//Homo sapiens chromosome 10 clone CIT-HSP-1287C20, complete sequence.//1.0:368:59//AC005879
R-NT2RP2003164//Dictyostelium discoideum actin 4 gene, 3' UTR.//1.0:120:64//M25581
R-NT2RP2003165//Homo sapiens chromosome 17, clone hRPK.1018_N_14, complete sequence.//2.2e-71:467:86//AC005823
R-NT2RP2003177
R-NT2RP2003194//Human DNA sequence *** SEQUENCING IN PROGRESS *** from clone 996D20, WORKING DRAFT SEQUENCE.//1.1e-95:585:88//AL031597
R-NT2RP2003206//P.falciparum interspersed repeat antigen (FIRA) gene.//0.039:338:60//M17877
R-NT2RP2003230//Plasmodium falciparum MAL3P6, complete sequence.//1.9e-11:542:60//Z98551
R-NT2RP2003237//Arabidopsis thaliana genomic DNA, chromosome 5, P1 clone: MDH9, complete sequence.//1.0:311:60//AB016888
R-NT2RP2003243//CIT-HSP-2368D12.TR CIT-HSP Homo sapiens genomic clone 2368D12, genomic survey sequence.//0.39:112:66//AQ077738
R-NT2RP2003265//Muridae sp. (mouse-rat, neuroblastoma-glioma hybrid cell line NGD5) mRNA, complete cds.//1.3e-38:273:83//L38481
R-NT2RP2003272//Homo sapiens clone UWGC:y17c131 from 6p21, complete sequence.//4.4e-15:181:66//AC004187
R-NT2RP2003277//Homo sapiens mRNA for KIAA0625 protein, partial cds.//4.2e-110:565:95//AB014525
R-NT2RP2003280//Homo sapiens 12p13.3 PAC RPCI5-1180D12 (Roswell Park Cancer Institute Human PAC Library) complete sequence.//3.2e-12:221:70//AC005831
R-NT2RP2003286//Homo sapiens chromosome 19, CIT-HSP-444n24, complete sequence.//0.86:379:60//AC005261
R-NT2RP2003293//Homo sapiens clone RG252P22, WORKING DRAFT SEQUENCE, 3 unordered pieces.//1.0e-39:418:74//AC005079
R-NT2RP2003295//HS_2053_B1_A10_MR CIT Approved Human Genomic Sperm Library D Homo sapiens genomic clone Plate=2053 Col=19 Row=B, genomic survey sequence.//0.0016:346:61//AQ235251
R-NT2RP2003297//Arabidopsis thaliana chromosome II BAC F4P9 genomic sequence, complete sequence.//0.74:397:56//AC002332
R-NT2RP2003308//Homo sapiens PAC clone DJ1098B01 from 7q11.23-q21, complete sequence.//0.99:447:60//AC004960
R-NT2RP2003329//C.reinhartii psbB 5' flanking region.//0.79:161:59//X59731
R-NT2RP2003339//RPCI11-57H15.TK RPCI11 Homo sapiens genomic clone R-57H15, genomic survey sequence.//0.13:184:64//AQ116039
R-NT2RP2003347//RPCI11-15B19.TV RPCI-11 Homo sapiens genomic clone RPCI-11-15B19, genomic survey sequence.//6.4e-31:218:89//B76357
R-NT2RP2003367//Human Chromosome 16 BAC clone CIT987SK-A-363E6, complete sequence.//9.0e-11:101:84/U91321
R-NT2RP2003391//HS_2255_B2_B04_MF CIT Approved Human Genomic Sperm Library D Homo sapiens genomic clone Plate=2255 Col=8 Row=D, genomic survey sequence.//1.6e-38:247:90//AQ068937
R-NT2RP2003393//RPCI11-44K6.TJ RPCI11 Homo sapiens genomic clone R-44K6, genomic survey sequence.//3.9e-31:290:79//AQ202481
R-NT2RP2003394//Yeast mitochondrial oxi3 gene exon 1 for cytochrome c oxidase subunit I.//5.1e-14:579:61//X14910
R-NT2RP2003401//Caprine arthritis-encephalitis virus tat protein (tat) and envelope glycoprotein (env) gene, partial cds.//0.32:174:66//U81429
R-NT2RP2003433//Ascidian mRNA for HRSec61, complete cds.//1.5e-10:193:69//D25536
R-NT2RP2003445//Human DNA sequence *** SEQUENCING IN PROGRESS *** from clone Y313F4, WORKING DRAFT SEQUENCE.//4.4e-99:585:89//AL023808
R-NT2RP2003446
R-NT2RP2003456//Plasmodium falciparum MAL3P7, complete sequence.//0.98:399:57//AL034559
R-NT2RP2003480//Homo sapiens full-length insert cDNA clone ZE09A11.//4.7e-111:540:98//AF086540
R-NT2RP2003499
R-NT2RP2003506
R-NT2RP2003511
R-NT2RP2003513//Human mRNA for KIAA0270 gene, partial cds.//4.1e-107:566:93//D87460
R-NT2RP2003517//Human c-sis/platelet-derived growth factor 2 (SIS/PDGF2) mRNA, complete cds.//1.5e-60:518:79//M12783
R-NT2RP2003522//HS_2182_A1_D05_MF CIT Approved Human Genomic Sperm Library D Homo sapiens genomic clone Plate=2182 Col=9 Row=G, genomic survey sequence.//0.053:251:60//AQ024304
R-NT2RP2003533//Homo sapiens chromosome 12p13.3 clone RPCI4-816N1, WORKING DRAFT SEQUENCE, 31 unordered pieces.//1.5e-37:328:80//AC005841
R-NT2RP2003543//HS_3028_A2_C12_MF CIT Approved Human Genomic Sperm Library D Homo sapiens genomic clone Plate=3028 Col=24 Row=E, genomic survey sequence.//2.0e-39:203:100//AQ094957
R-NT2RP2003559//Homo sapiens full-length insert cDNA clone ZD65E09.//2.3e-59:325:95//AF088055
R-NT2RP2003564
R-NT2RP2003581
R-NT2RP2003596//HS_2163_B1_D11_MF CIT Approved Human Genomic Sperm Library D Homo sapiens genomic clone Plate=2163 Col=21 Row=H, genomic survey sequence.//0.0011:212:67//AQ125143
R-NT2RP2003604//Homo sapiens alpha-catenin-like protein mRNA, complete cds.//5.4e-102:501:97//U97067
R-NT2RP2003629//Plasmodium falciparum 3D7 chromosome 12 PFYACB8-628 genomic sequence, WORKING DRAFT SEQUENCE, 9 unordered pieces.//0.0012:363:61//AC005507
R-NT2RP2003643//Mus musculus mRNA for CMP-N-acetylneuraminic acid synthetase.//5.1e-37:561:68//AJ006215
R-NT2RP2003668//Human DNA sequence from PAC 24608, between markers DXS6791 and DXS8038 on chromosome X contains ESTs.//0.0053:395:58//Z76735
R-NT2RP2003687//Human BAC clone RG222A16 from 7q31, complete sequence.//8.0e-10:205:67//AC002385
R-NT2RP2003691//HS_3252_A2_A11_MR CIT Approved Human Genomic Sperm Library D Homo sapiens genomic clone Plate=3252 Col=22 Row=A, genomic survey sequence.//5.3e-05:332:60//AQ219783
R-NT2RP2003702//CIT-HSP-2333P5.TF CIT-HSP Homo sapiens genomic clone 2333P5, genomic survey sequence.//3.9e-43:431:75//AQ035000
R-NT2RP2003704
R-NT2RP2003706//Homo sapiens mRNA for KIAA0525 protein, partial cds.//2.6e-45:265:93//AB011097
R-NT2RP2003713//Human DNA sequence from PAC 411B6 on chromosome X *.//0.64:169:67//Z84470
R-NT2RP2003714//Human DNA sequence from 4PTEL, Huntington's Disease Region, chromosome 4p16.3.//4.6e-11:152:73//295704
R-nnnnnnnnnnnn//H.sapiens mRNA for PIBF1 protein, complete.//0.94:443:59//Y09631
R-NT2RP2003737//Homo sapiens clone DJ1022I14, WORKING DRAFT SEQUENCE, 14 unordered pieces.//2.2e-109:547:96//AC004951
R-NT2RP2003751//Homo sapiens Chromosome 16 BAC clone CIT987SK-A-427H10, complete sequence.//4.1e-109:545:97//AC004626
R-NT2RP2003760//B. taurus mRNA for gamma-COP.//6.3e-28:400:69//X70019
R-NT2RP2003764//Mouse preprosomatostatin gene.//0.90:285:62//X51468
R-NT2RP2003769//Schizosaccharomyces pombe gene for protein involved in sexual development, complete cds.//0.96:446:58//D87956
R-NT2RP2003770//Homo sapiens sperm acrosomal protein mRNA, complete cds.//1.8e-104:531:96//AF047437
R-NT2RP2003777
R-NT2RP2003781//HS_3109_B1_B04_T7 CIT Approved Human Genomic Sperm Library D Homo sapiens genomic clone Plate=3109 Col=7 Row=D, genomic survey sequence.//1.3e-60:346:92//AQ186749
R-NT2RP2003793
R-NT2RP2003840
R-NT2RP2003857//HS_2205_A2_H12_MF CIT Approved Human Genomic Sperm Library D Homo sapiens genomic clone Plate=2205 Col=24 Row=O, genomic survey sequence.//8.1e-22:127:99//AQ151299
R-NT2RP2003859//RPCI11-37G8.TV RPCI-11 Homo sapiens genomic clone RPCI-11-37G8, genomic survey sequence.//8.3e-60:320:95//AQ029850
R-NT2RP2003871//HS_3210_A1_C08_T7 CIT Approved Human Genomic Sperm Library D Homo sapiens genomic clone Plate=3210 Col=15 Row=E, genomic survey sequence.//8.6e-09:322:61//AQ175028
R-NT2RP2003885//RPCI11-7M10.TP RPCI-11 Homo sapiens genomic clone RPCI-11-7M10, genomic survey sequence.//4.7e-67:380:92//B72214
R-NT2RP2003912//Human DNA sequence *** SEQUENCING IN PROGRESS *** from clone 32B1, WORKING DRAFT SEQUENCE.//1.2e-33:379:75//AL023693
R-NT2RP2003952
R-NT2RP2003968//Homo sapiens hUBP mRNA for ubiquitin specific protease, complete cds.//2.3e-114:568:97//AB014458
R-NT2RP2003976//Homo sapiens mRNA for KIAA0447 protein, complete cds.//1.1e-107:540:97//AB007916
R-NT2RP2003981//Homo sapiens mRNA for KIAA0804 protein, partial cds.//7.7e-114:568:96//AB018347
R-NT2RP2003984
R-NT2RP2003986//Human Chromosome 11 pac pDJ197h17, WORKING DRAFT SEQUENCE, 11 unordered pieces.//6.6e-99:551:92//AC0003 82
R-NT2RP2003988
R-NT2RP2004014
R-NT2RP2004041//Homo sapiens chromosome 19, cosmid F17127, complete sequence.//4.9e-114:568:97//AC004780
R-NT2RP2004042//nbxb0020F03r CUGI Rice BAC Library Oryza sativa genomic clone nbxb0020F03r, genomic survey sequence.//0.11:195:64//AQ258389
R-nnnnnnnnnnnn//Human DNA sequence *** SEQUENCING IN PROGRESS *** from clone 134019, WORKING DRAFT SEQUENCE.//7.6e-110:564:95//AL034555
R-NT2RP2004081//Plasmodium falciparum 3D7 chromosome 12 PFYAC492 genomic sequence, WORKING DRAFT SEQUENCE, 5 unordered pieces.//0.012:503:57//AC005308
R-NT2RP2004098//H.sapiens CpG island DNA genomic Mse1 fragment, clone 133h3, reverse read cpg133h3.rt1a.//7.9e-25:140:100//Z64530
R-NT2RP2004124
R-NT2RP2004142//CIT-HSP-2316F21.TR CIT-HSP Homo sapiens genomic clone 2316F21, genomic survey sequence.//2.8e-83:409:98//AQ034964
R-NT2RP2004152//HS_3065_A2_D04_MF CIT Approved Human Genomic Sperm Library D Homo sapiens genomic clone Plate=3065 Col=8 Row=G, genomic survey sequence.//2.5e-62:304:100//AQ137776
R-NT2RP2004165//Anthocidaris crassispina mRNA for dynein beta-heavy chain, complete cds.//3.4e-20:343:65//D01021
R-NT2RP2004170//*** SEQUENCING IN PROGRESS *** Homo sapiens chromosome 4, BAC clone B33108; HTGS phase 1, WORKING DRAFT SEQUENCE, 10 unordered pieces.//2.5e-89:587:86//AC004064
R-NT2RP2004172//Dictyostelium discoideum LTR-retrotransposon Skipper, partial genomic sequence, 3' end.//0.24:440:60//AF017047
R-NT2RP2004187//RPCI11-59E12.TK RPCI11 Homo sapiens genomic clone R-59E12, genomic survey sequence.//3.1e-05:175:66//AQ198120
R-NT2RP2004194
R-NT2RP2004196//Fugu rubripes GSS sequence, clone 076D01bE2, genomic survey sequence.//1.6e-22:178:71//AL026601
R-NT2RP2004207//Homo sapiens BAC clone GS421I03 from Xq25-q26, complete sequence.//0.19:175:64//AC005023
R-NT2RP2004226//Human DNA sequence *** SEQUENCING IN PROGRESS *** from clone Y313F4, WORKING DRAFT SEQUENCE.//6.1e-17:445:64//AL023808
R-NT2RP2004232//M.musculus (Balb/c) mRNA for serine/threonine protein kinase.//3.2e-25:326:71//Z34524
R-NT2RP2004239//Homo sapiens lok mRNA for protein kinase, complete cds.//8.7e-108:563:94//AB015718
R-NT2RP2004240//Homo sapiens antigen NY-CO-1 (NY-CO-1) mRNA, complete cds.//1.1e-101:530:93//AF039687
R-NT2RP2004242
R-NT2RP2004245//Homo sapiens DNA sequence from PAC 455H14 on chromosome Xq21.3-22.3. Contains genomic marker DXS1203 with a CA repeat polymorphism, STSs and GSSs, complete sequence.//5.1e-08:236:65//AL023280
R-NT2RP2004270//Lycopersicon esculentum ldh2 gene.//0.98:259:61//Y10603
R-NT2RP2004300//Human DNA sequence *** SEQUENCING IN PROGRESS *** from clone 1068F16, WORKING DRAFT SEQUENCE.//5.0e-14:396:65//AL023913
R-NT2RP2004316//Homo sapiens EXT-like protein 2 (EXTL2) mRNA, complete cds.//1.5e-108:544:96//AF000416
R-NT2RP2004321//Caenorhabditis elegans cosmid F47B8, complete sequence.//0.0078:333:61//Z77662
R-NT2RP2004339//Homo sapiens PAC clone DJ1136G13 from 7q35-q36, complete sequence.//1.4e-75:306:86//AC005229
R-NT2RP2004347//RPCI11-90N11.TJ RPCI11 Homo sapiens genomic clone R-90N11, genomic survey sequence.//2.9e-87:494:92//AQ284548
R-NT2RP2004364//Human DNA sequence from clone 422F24 on chromosome 6q24.1-25.2. Contains a novel gene similar to C. elegans C02C2.5. Contains ESTs, STSs and GSSs, complete sequence.//4.2e-10:161:76//AL031010
R-NT2RP2004365//Plasmodium falciparum chromosome 2, section 70 of 73 of the complete sequence.//3.6e-08:483:57//AE001433
R-NT2RP2004366//F.rubripes GSS sequence, clone 013B16aF3, genomic survey sequence.//2.1e-05:128:67//AL000528
R-NT2RP2004373//Homo sapiens 12q24.2 BAC RPCI11-407A16 (Roswell Park Cancer Institute Human BAC Library) complete sequence.//0.81:205:62//AC006065
R-NT2RP2004389//HS_2183_B2_H04_MR CIT Approved Human Genomic Sperm Library D Homo sapiens genomic clone Plate=2183 Col=8 Row=P, genomic survey sequence.//3.9e-06:82:84//AQ063969
R-NT2RP2004392//Ceratovacuna sp. mitochondrial cytochrome oxidase I (3' end), cytochrome oxidase II (complete cds) and transfer RNA-Leu gene.//2.7e-06:495:58//L39993
R-NT2RP2004396//Homo sapiens BAC clone RG135C18 from 7q21, complete sequence.//6.4e-111:572:96//AC005164
R-NT2RP2004399//Arabidopsis thaliana chromosome I BAC F11M15 genomic sequence, complete sequence.//0.13:253:64//AC006085
R-NT2RP2004400//HS_3238_A2_H11_T7 CIT Approved Human Genomic Sperm Library D Homo sapiens genomic clone Plate=3238 Col=22 Row=O, genomic survey sequence.//5.1e-23:162:89//AQ211412
R-NT2RP2004412//Saccharomyces douglasii mitochondrial cytochrome c oxidase subunit I (COXI) gene, complete cds.//2.6e-09:458:60//M97514
R-NT2RP2004425//Human DNA sequence from clone 1052M9 on chromosome Xq25. Contains the SH2D1A gene for SH2 domain protein 1A, Duncan's disease (lymphoproliferative syndrome) (DSHP), part of a 60S Acidic Ribosomal protein 1 (RPLP1) LIKE gene and part of a mouse DOC4 LIKE gene. Contains ESTs and GSSs, complete sequence.//0.99:481:56//AL022718
R-NT2RP2004476//Rattus norvegicus activity and neurotransmitter-induced early gene 6 (ania-6) mRNA, 3'UTR.//5.3e-99:600:90//AF030091
R-NT2RP2004490//Homo sapiens chromosome 16, P1 clone 94-10H (LANL), complete sequence.//3.9e-115:575:97//AC005591
R-NT2RP2004512//Plasmodium falciparum MAL3P3, complete sequence.//0.00034:517:58//Z98547
R-NT2RP2004523//Homo sapiens clone DJ0800G07, complete sequence.//1.8e-115:571:97//AC004890
R-NT2RP2004538//Homo sapiens BAC clone RG318C11 from 7p14-p15, complete sequence.//1.7e-47:322:87//AC005091
R-NT2RP2004551//Homo sapiens Xp22 bins 45-47 BAC GSHB-665N22 (Genome Systems Human BAC Library) complete sequence.//0.035:511:58//AC005184
R-NT2RP2004568//T7C20-Sp6 TAMU Arabidopsis thaliana genomic clone T7C20, genomic survey sequence.//0.70:446:54//B08766
R-NT2RP2004580//Human DNA sequence *** SEQUENCING IN PROGRESS *** from clone 136B1, WORKING DRAFT SEQUENCE.//2.2e-53:397:74//AL031768
R-NT2RP2004587//CIT-HSP-2376P22.TF CIT-HSP Homo sapiens genomic clone 2376P22, genomic survey sequence.//0.0079:223:63//AQ108976
R-NT2RP2004594//Homo sapiens Chromosome 16 BAC clone CIT987SK-A-248F7, complete sequence.//5.3e-10:493:62//AC004605
R-NT2RP2004600//Homo sapiens full-length insert cDNA clone ZE04E06.//2.1e-70:343:99//AF086522
R-NT2RP2004602//Homo sapiens full-length insert cDNA clone YW26E09.//2.0e-96:528:93//AF086033
R-NT2RP2004614
R-NT2RP2004655//Homo sapiens mRNA for leucine rich protein.//7.3e-117:587:96//AJ006291
R-NT2RP2004664//Homo sapiens mRNA for KIAA0460 protein, partial cds.//1.8e-105:520:96//AB007929
R-NT2RP2004675//Human elastin (ELN) gene, partial cds, and LIM-kinase (LIMK1) gene, complete cds.//3.4e-22:197:79//U63721
R-NT2RP2004681//Rat notch 2 mRNA.//8.0e-30:276:78//M93661
R-NT2RP2004689//Homo sapiens mRNA for KIAA0625 protein, partial cds.//1.6e-118:600:96//AB014525
R-NT2RP2004709//Homo sapiens full-length insert cDNA clone ZD42A08.//3.5e-14:139:86//AF086259
R-NT2RP2004710//Human DNA sequence *** SEQUENCING IN PROGRESS *** from clone 126A5, WORKING DRAFT SEQUENCE.//6.9e-117:592:96//AL031447
R-NT2RP2004736//Homo sapiens mRNA for KIAA0478 protein, complete cds.//4.2e-117:594:96//AB007947
R-NT2RP2004743//Plasmodium falciparum 3D7 chromosome 12 PFYAC181 genomic sequence, WORKING DRAFT SEQUENCE, 8 unordered pieces.//0.53:403:59//AC005505
R-NT2RP2004767//Human DNA sequence from PAC 491M17 on chromosome 1p36.2-1p36.3.//2.0e-81:568:84//Z97988
R-NT2RP2004775//Anopheles quadrimaculatus NADH dehydrogenase subunits (1-4, 4L, 5-6); cytochrome oxidase subunits (1-3); adenosine triphosphatase subunits (6,8); cytochrome b; transfer RNA; ribosomal RNA (large and small subunits).//4.0e-08:365:62//L04272

R-NT2RP2004791//Homo sapiens chromosome 5, BAC clone 282B7 (LBNL H192), complete sequence.//7.8e-111:541:98//AC005216
R-NT2RP2004799//Homo sapiens ATP-specific succinyl-CoA synthetase beta subunit (SCS) mRNA, partial cds.//2.5e-114:564:96//AF058953
R-NT2RP2004802
R-NT2RP2004816//Homo sapiens H beta 58 homolog mRNA, complete cds.//2.7e-118:584:97//AF054179
R-NT2RP2004841//Human BAC clone RG308B22 from 7q22-q31, complete sequence.//4.0e-46:447:72//AC002089
R-NT2RP2004861//Plasmodium"falciparum MAL3P5, complete sequence.//0.19:189:66//AL034556
R-NT2RP2004897//Human Chromosome X clone bWXD187, complete sequence.//1.1e-08:330:61//AC004383
R-NT2RP2004936//CIT-HSP-2374L4.TF CIT-HSP Homo sapiens genomic clone 2374L4, genomic survey sequence.//0.99:129:65//AQ110571
R-nnnnnnnnnnnn//Plasmodium falciparum MAL3P6, complete sequence.//0.014:402:61//Z98551
R-NT2RP2004961//RPCI11-45P2.TK RPCI11 Homo sapiens genomic clone R-45P2, genomic survey sequence.//9.3e-90:453:97//AQ202282
R-NT2RP2004962//Caenorhabditis elegans DNA *** SEQUENCING IN PROGRESS *** from clone Y40H4, WORKING DRAFT SEQUENCE.//0.017:291:61//AL022573
R-NT2RP2004967//Homo sapiens clone RG228D17, WORKING DRAFT SEQUENCE, 2 unordered pieces.//4.6e-52:496:77//AC005077
R-NT2RP2004978//Homo sapiens chromosome 19, cosmid F23269, complete sequence.//0.088:322:63//AC005614
R-NT2RP2004982//Homo sapiens BAC clone BK085E05 from 22q12.1-qter, complete sequence.//0.025:339:61//AC003071
R-NT2RP2004985//T31H24TF TAMU Arabidopsis thaliana genomic clone T31H24, genomic survey sequence.//0.40:111:70//B78148
R-NT2RP2004999//Homo sapiens clone NH0084K19, WORKING DRAFT SEQUENCE, 30 unordered pieces.//0.23:157:68//AC005682
R-NT2RP2005000
R-NT2RP2005001//Homo sapiens mRNA for KIAA0615 protein, complete cds.//3.0e-111:577:95//AB014515
R-NT2RP2005003//Homo sapiens Xp22-132-134 BAC GSHB-590J15 (Genome Systems Human BAC library) complete sequence.//2.4e-21:246:77//AC004673
R-nnnnnnnnnnnn//Homo sapiens SEC63 (SEC63) mRNA, complete cds.//9.5e-115:568:97//AF100141
R-NT2RP2005018//HS_3108_B1_E09_T7 CIT Approved Human Genomic Sperm Library D Homo sapiens genomic clone Plate=3108 Col=17 Row=J, genomic survey sequence.//1.9e-31:222:89//AQ104050
R-NT2RP2005020//Rattus norvegicus cationic amino acid transporter-1 (CAT-1) mRNA, complete cds.//6.6e-41:566:73//U70476
R-NT2RP2005031//CIT-HSP-516A2.TV CIT-HSP Homo sapiens genomic clone 516A2, genomic survey sequence.//4.1e-31:357:75//B49897
R-NT2RP2005037
R-NT2RP2005038//Sequence 5 from patent US 5552281.//2.2e-32:178:98//I25644
R-NT2RP2005108//Mus musculus orphan nuclear hormone receptor (CAR) gene, complete sequence.//3.7e-23:475:67//AF009326
R-NT2RP2005116//Homo sapiens mRNA for KIAA0664 protein, partial cds.//8.4e-104:518:97//AB014564
R-NT2RP2005126//H.sapiens mRNA for RNA helicase (Myc-regulated dead box protein).//1.4e-67:464:85//X98743
R-NT2RP2005139
R-NT2RP2005140//Leishmania mexicana amazonensis kinetoplast (clone 29) maxicircle A+T-rich repetitive DNA sequence.//7.9e-08:460:60//U00101
R-NT2RP2005144//Homo sapiens chromosome 12p13.3 clone RPCI11-372B4, WORKING DRAFT SEQUENCE, 129 ordered pieces.//2.5e-103:519:96//AC005911
R-NT2RP2005147//Homo sapiens clone DJ1125K23, WORKING DRAFT SEQUENCE, 21 unordered pieces.//0.068:100:75//AC004971
R-NT2RP2005159//CITBI-E1-2506A8.TF CITBI-E1 Homo sapiens genomic clone 2506A8, genomic survey sequence.//0.90:113:71//AQ262104
R-NT2RP2005162//Homo sapiens chromosome 17, clone HCIT307A16, complete sequence.//5.0e-14:183:75//AC003041
R-NT2RP2005168//Homo sapiens mRNA for E1B-55kDa-associated protein.//7.5e-100:513:95//AJ007509
R-NT2RP2005204
R-NT2RP2005227//Homo sapiens PAC clone DJ0905J08 from 7p12-p14, complete sequence.//7.2e-119:583:97//AC005189
R-NT2RP2005239//Homo sapiens mRNA for putative tRNA splicing protein, partial.//8.4e-62:312:98//AJ010952
R-NT2RP2005254//Homo sapiens DNA sequence from PAC 262D12 on chromosome 1q23.3-24.3. Contains a Tenascin (Hexabrachion, Cytotactin, Neuronectin, Myotendinous antigen)-LIKE gene and a mitochondrial/chloroplast 30S ribosomal protein S14-LIKE gene preceeded by a CpG island. Contains ESTs, genomic marker D1S2691 and STSs.//5.7e-09:328:62//Z99297
R-NT2RP2005270//Plasmodium falciparum MAL3P8, complete sequence.//2.3e-05:355:61//AL034560
R-NT2RP2005276//Genomic sequence for Arabidopsis thaliana BAC F17F8, complete sequence.//0.0014:541:58//AC000107
R-NT2RP2005287//Cavia porcellus zinc finger protein (zfoC1) mRNA, complete cds.//4.4e-69:459:86//L26335
R-NT2RP2005288//Homo sapiens RCC1-like G exchanging factor RLG mRNA, complete cds.//7.4e-124:594:98//AF060219
R-NT2RP2005289//Homo sapiens mRNA for XRP2 protein.//1.5e-110:545:96//AJ007590
R-NT2RP2005293//Leishmania mexicana amazonensis kinetoplast (clone 29) maxicircle A+T-rich repetitive DNA sequence.//1.1e-12:554:61//U00101
R-NT2RP2005315//Homo sapiens DNA sequence from PAC 168L15 on chromosome 6q26-27. Contains RSK3 gene, ribosomal protein S6 kinase, EST, GSS, STS. CpG island, complete sequence.//9.5e-15:218:77//AL022069
R-NT2RP2005325//Rattus norvegicus LIM homeodomain protein (LH-2) mRNA sequence.//2.0e-72:478:88//L06804
R-NT2RP2005336//***ALU WARNING: Human Alu-J subfamily consensus sequence.//7.3e-33:139:82//U14567
R-NT2RP2005344//Human DNA sequence from PAC 128N22 on chromosome Xq25-Xq26.3. contains STS.//0.094:451:60//297629
R-NT2RP2005354//Homo sapiens mRNA for putative thioredoxin-like protein.//1.3e-11:89:96//AJ010841
R-NT2RP2005360//Homo sapiens clone RG023I15, WORKING DRAFT SEQUENCE, 1 unordered pieces.//0.046:266:60//AC005049
R-NT2RP2005393//Homo sapiens chromosome 17, clone hRPK.85_B_7, complete sequence.//6.0e-41:226:86//AC005695
R-NT2RP2005407
R-NT2RP2005436//Polistes annularis (clone pan117AAT) tandem repeat region.//0.039:169:63//L10835
R-NT2RP2005441//CIT-HSP-2338P5.TR CIT-HSP Homo sapiens genomic clone 2338P5, genomic survey sequence.//3.0e-38:263:88//AQ055548
R-NT2RP2005453//CIT-HSP-2367N1.TR CIT-HSP Homo sapiens genomic clone 2367N1, genomic survey sequence.//0.67:409:59//AQ079845
R-NT2RP2005457//Homo sapiens partial XPGC gene, exon 2.//2.0e-42:315:82//X71342
R-NT2RP2005464//CIT-HSP-2359C16.TF CIT-HSP Homo sapiens genomic clone 2359C16, genomic survey sequence.//1.0:251:60//AQ075816
R-NT2RP2005465//Drosophila melanogaster, chromosome 2R, region 44D1-44D2, P1 clone DS08616, complete sequence.//01251288:62//AC005457
R-NT2RP2005472//Chlorarachnion CCMP621 small subunit ribosomal RNA, 5.8S ribosomal RNA, large subunit ribosomal RNA, U6 small nuclear RNA, small subunit ribosomal protein S13 (RPS13), pre-mRNA splicing factor PRP 6 homolog, small subunit ribosomal protein 4 (RPS4), small nucleolar ribonucleoprotein E homolog (snRNPE), ATP-dependent clp protease proteolytic subunit homolog (CLPP), putative RNA polymerase II subunit (RNA POLII), and RNA helicase homolog (RNAHEL) genes, complete cds.//1.0:356:59//U58510
R-NT2RP2005476//Plasmodium falciparum DNA *** SEQUENCING IN PROGRESS *** from MAL1P3, WORKING DRAFT SEQUENCE.//0.00092:421:60//AL031746
R-NT2RP2005490//Homo sapiens clone NH0001P09, WORKING DRAFT SEQUENCE, 1 unordered pieces.//6.2e-71:187:100//AC006030
R-NT2RP2005491//paramecium species 5,311 mt dna dimer: replication init. region.//1.6e-10:403:62//K00917
R-NT2RP2005495//Homo sapiens clone RG037F03, WORKING DRAFT SEQUENCE, 12 unordered pieces.//1.3e-25:208:82//AC005051
R-NT2RP2005496//Human DNA sequence from clone 354N19 on chromosome 6q22. Contains the 3' part of the gene for Mannosyl-Oligosaccharide Alpha-1,2-Mannosidase (Man(9)-alpha-mannosidase, EC 3.2.1.113), a Cytochrome C Oxidase Polypeptide I (EC 1.9.3.1) pseudogene and a pseudogene similar to 60S Ribosomal Protein L13A. Contains genomic markers D6S287 and D6S1696, ESTs, STSs, GSSs and two CA repeat polymorphisms, complete sequence.//1.5e-22:196:84//AL022722
R-NT2RP2005498
R-NT2RP2005501//Homo sapiens chromosome 17, clone hRPK.269_G_24, complete sequence.//.7e-29:252:76//AC005828
R-NT2RP2005509//CIT-HSP-2060J6.TR CIT-HSP Homo sapiens genomic clone 2060J6, genomic survey sequence.//3.1e-53:402:84//B69979
R-NT2RP2005520//Homo sapiens chromosome-associated protein-E (hCAP-E) mRNA, complete cds.//9.9e-109:570:94//AF092563
R-NT2RP2005525//Human clone JkA2 mRNA induced upon T-cell activation, 3' end.//5.1e-32:175:98//U38432
R-NT2RP2005531//Homo sapiens PAC clone DJ0870F17 from 7q33-q36, complete sequence.//0.94:288:61//AC004911
R-NT2RP2005539//Homo sapiens mRNA for NSl-binding protein (NS1-BP).//2.7e-106:560:94//AJ012449
R-NT2RP2005540//Homo sapiens mRNA for KIAA0494 protein, complete cds.//5.3e-114:583:96//AB007963
R-NT2RP2005549//Homo sapiens *** SEQUENCING IN PROGRESS ***, WORKING DRAFT SEQUENCE.//0.91:287:58//AJ011929
R-NT2RP2005555//Homo sapiens 12p13.3 PAC RPCIS-927J10 (Roswell Park Cancer Institute Human PAC library) complete sequence.//3.6e-05:222:66//AC004804
R-NT2RP2005557//Homo sapiens PAC clone DJ1200I23 from 7p15, complete sequence.//8.2e-22:236:76//AC004996
R-NT2RP2005581//Homo sapiens clone DJ0693M11, WORKING DRAFT SEQUENCE, 7 unordered pieces.//7.2e-45:286:85//AC006146
R-NT2RP2005600//Human polymorphic microsatellite DNA.//0.043:304:58//M99148
R-NT2RP2005605//Human Cosmid g1572c190, complete sequence.//2.4e-17:163:77//AC000126
R-NT2RP2005620
R-NT2RP2005622//jd432 Trypanosome Shotgun M13 genomic Trypanosoma brucei brucei genomic clone 11B7, genomic survey sequence.//0.010:308:58//B13538
R-NT2RP2005637//Homo sapiens PAC clone DJ0555L14 from 7q34-q36, complete sequence.//2.5e-26:322:72//AC005996
R-NT2RP2005640//Mus musculus squamous cell carcinoma antigen 2 (Scca2) gene, complete cds.//0.030:370:60//AF063937
R-NT2RP2005645//Plasmodium falciparum chromosome 2, section 35 of 73 of the complete sequence.//3.2e-08:355:62//AE001398
R-NT2RP2005651
R-NT2RP2005654//Leishmania major Friedlin cosmid L5769, complete sequence.//0.96:216:66//AL031908
R-NT2RP2005669//Homo sapiens nitrilase homolog 1 (NIT1) gene, alternatively spliced product, complete cds.//6.7e-117:594:95//AF069984
R-NT2RP2005675//Homo sapiens growth suppressor related (DOC-1R) mRNA, complete cds.//1.8e-89:434:98//AF089814
R-NT2RP2005683//jd432 Trypanosome Shotgun M13 genomic Trypanosoma brucei brucei genomic clone 11B7, genomic survey sequence.//0.037:283:58//B13538
R-NT2RP2005690//Homo sapiens clone DJ0425I02, WORKING DRAFT SEQUENCE, 5 unordered pieces.//1.5e-38:295:83//AC005478
R-NT2RP2005694//Plasmodium falciparum DNA *** SEQUENCING IN PROGRESS *** from contig 3-106, complete sequence.//0.0026:414:57//AL010210
R-NT2RP2005701
R-NT2RP2005712//Homo sapiens mRNA for KIAA0799 protein, partial cds.//4.1e-104:503:98//AB018342
R-NT2RP2005719//Caenorhabditis elegans cosmid LLC1, complete sequence.//0.83:275:61//Z82277
R-NT2RP2005722//Human DNA sequence *** SEQUENCING IN PROGRESS *** from clone 228H13, WORKING DRAFT SEQUENCE.//1.2e-21:199:75//AL031985
R-NT2RP2005723
R-NT2RP2005726//Homo sapiens clone DJ0609N19, WORKING DRAFT SEQUENCE, 3 unordered pieces.//2.6e-64:503:82//AC004842
R-NT2RP2005741//Human Chromosome 11 pac pDJ393o15, WORKING DRAFT SEQUENCE, 8 unordered pieces.//2.5e-09:261:64//AC000384
R-NT2RP2005748//RPCI11-64K11.TK RPCI11 Homo sapiens genomic clone R-64K11, genomic survey sequence.//0.00039:215:66//AQ239313
R-NT2RP2005752//Homo sapiens TNFR-related death receptor-6 (DR6) mRNA, complete cds.//1.3e-40:223:96//AF068868
R-NT2RP2005753//Homo sapiens I-1 receptor candidate protein mRNA, complete cds.//3.7e-103:494:98//AF082516
R-NT2RP2005763//Homo sapiens DNA sequence from PAC 510L9 on chromosome 6p24.1-p25.3.//9.7e-34:172:86//AL022098
R-NT2RP2005767//Human clone H3 mRNA.//2.5e-21:179:87//U03672
R-NT2RP2005773//HS_2168_B1_G12_MF CIT Approved Human Genomic Sperm Library D Homo sapiens genomic clone Plate=2168 Col=23 Row=N, genomic survey sequence.//0.99:212:63//AQ086414
R-NT2RP2005775//Rabbit mRNA for endopeptidase, complete cds.//4.8e-98:591:88//D13310
R-NT2RP2005781//Streptomyces sp. genomic DNA for sarcosine oxidase.//0.019:384:59//D10623
R-NT2RP2005784//Human DNA sequence *** SEQUENCING IN PROGRESS *** from clone 1185N5, WORKING DRAFT SEQUENCE.//1.8e-102:490:99//AL034423
R-NT2RP2005804//Homo sapiens chromosome 17, clone hRPK.147_L_13, complete sequence.//6.3e-16:481:63//AC005332
R-NT2RP2005812//Caenorhabditis elegans cosmid F15810.//0.81:147:63//AF036696
R-NT2RP2005815
R-NT2RP2005835
R-NT2RP2005841//Human DNA sequence from cosmid U209G1 on chromosome X.//1.5e-26:512:64//Z68873
R-NT2RP2005853//Human DNA sequence from clone 1156N12 on chromosome X. Contains an STS and GSSs, complete sequence.//3.7e-16:340:64//AL009047
R-NT2RP2005857//Human DNA sequence from cosmid U246D9 on chromosome X. Contains a histone H2B like pseudogene.//1.3e-09:331:65//AL021308
R-NT2RP2005859//Plasmodium falciparum DNA *** SEQUENCING IN PROGRESS *** from contig 3-83, complete sequence.//0.0097:363:59//AL010152
R-NT2RP2005868//Plasmodium falciparum DNA *** SEQUENCING IN PROGRESS *** from contig 3-18, complete sequence.//1.1e-07:508:60//AL008971
R-NT2RP2005890//Mouse oncogene (ect2) mRNA, complete cds.//2.7e-31:500:67//AL11316
R-NT2RP2005901//Homo sapiens T-cell receptor alpha delta locus from bases 752679 to 1000555 (section 4 of 5) of the Complete Nucleotide Sequence.//0.89:276:60//AE000661
R-NT2RP2005908
R-NT2RP2005933//Rattus norvegicus nucleoporin p54 mRNA, complete cds.//1.2e-40:285:80//U63840
R-NT2RP2005942//Homo sapiens DNA sequence from PAC 142L7 on chromosome 6q21. Contains a Laminin Alpha 4 (LAMA4) LIKE gene coding for two alternatively spliced transcripts, a Tubulin Beta LIKE pseudogene, a Connective tissue growth factor (NOV, GIG) LIKE gene, A predicted CpG island, ESTs, STSs and genomic marker D6S416, complete sequence.//0.0011:480:58//Z99289
R-NT2RP2005980//Homo sapiens Xp22 BAC GSHB-536K7 (Genome Systems Human BAC library) complete sequence.//8.9e-21:136:78//AC004616
R-NT2RP2006023//HS_2176_B1_C10_MR CIT Approved Human Genomic Sperm Library D Homo sapiens genomic clone Plate=2176 Col=19 Row=F, genomic survey sequence.//2.5e-66:369:95//AQ023148
R-NT2RP2006038//Plasmodium falciparum chromosome 2, section 6 of 73 of the complete sequence.//0.00029:408:58//AE001369
R-NT2RP2006043//Polistes annularis (clone pan117AAT) tandem repeat region.//0.032:195:62//L10835
R-NT2RP2006052//Plasmodium falciparum 3D7 chromosome 12 PFYACB8-420 genomic sequence, WORKING DRAFT SEQUENCE, 14 unordered pieces.//0.11:263:61//AC005140
R-NT2RP2006069
R-NT2RP2006071//Plasmodium falciparum 3D7 chromosome 12 PFYAC1122 genomic sequence, WORKING DRAFT SEQUENCE, 3 unordered pieces.//0.00044:333:61//AC004709
R-NT2RP2006098//Plasmodium falciparum DNA *** SEQUENCING IN PROGRESS *** from contig 3-77, complete sequence.//4.1e-09:393:62//AL010151
R-NT2RP2006100//HS_2020_A2_H02_T7 CIT Approved Human Genomic Sperm Library D Homo sapiens genomic clone Plate=2020 Col=4 Row=O, genomic survey sequence.//8.3e-53:304:92//AQ228761
R-NT2RP2006103//Rat sodium-hydrogen exchange protein-isoform 3 (NHE-3) mRNA, complete cds.//1.5e-16:199:79//M85300
R-NT2RP2006141
R-NT2RP2006166//Human Chromosome 16 BAC clone CIT987SK-A-589H1, complete sequence.//8.2e-48:329:76//AC002045
R-NT2RP2006184//RPCI11-6O16.TP RPCI-11 Homo sapiens genomic clone RPCI-11-6O16, genomic survey sequence.//0.52:273:61//B49539
R-NT2RP2006186//Homo sapiens mRNA for KIAA0654 protein, partial cds.//1.9e-108:553:95//AB014554
R-NT2RP2006196//Plasmodium falciparum DNA *** SEQUENCING IN PROGRESS *** from contig 3-57, complete sequence.//4.2e-05:420:59//AL008981
R-NT2RP2006200//Homo sapiens chromosome 12p13.3 clone RPCI1-96H9, WORKING DRAFT SEQUENCE, 66 unordered pieces.//2.1e-100:409:96//AC006057
R-NT2RP2006219//H.sapiens mRNA for DGCR6 protein.//3.8e-93:532:90//X96484
R-NT2RP2006237//P.falciparum PK1 gene.//2.9e-08:481:59//X83707
R-NT2RP2006238//Human chromosome 16 BAC clone CIT987SK-A-962B4, complete sequence.//3.5e-79:405:89//U91318
R-NT2RP2006258//Human PAC clone DJ0899B21 from 7p15-p21, complete sequence.//2.2e-08:283:63//AC004008
R-NT2RP2006261//H.sapiens mRNA for serine/threonine protein kinase EMK.//6.2e-13:234:68//X97630
R-NT2RP2006312//Homo sapiens BAF57 (BAF57) gene, complete cds.//2.0e-108:542:97//AF035262
R-NT2RP2006320//347J16.TVB CIT978SKA1 Homo sapiens genomic clone A-347J16, genomic survey sequence.//1.2e-27:215:65//B17768
R-NT2RP2006321//Human karyopherin beta 3 mRNA, complete cds.//1.7e-48:298:90//U72761
R-NT2RP2006323//Human DNA sequence *** SEQUENCING IN PROGRESS *** from clone 702J19, WORKING DRAFT SEQUENCE.//2.8e-104:524:96//AL033531
R-NT2RP2006333//Homo sapiens PAC clone DJ0808A01 from 7q21.1-q31.1, complete sequence.//3.9e-33:298:78//AC004893
R-NT2RP2006334
R-NT2RP2006365//RPCI11-72I15.TK RPCI11 Homo sapiens genomic clone R-72I15, genomic survey sequence.//2.6e-35:217:92//AQ267043
R-NT2RP2006393//*** SEQUENCING IN PROGRESS *** Homo sapiens chromosome 4, BAC clone B13E4; HTGS phase 1, WORKING DRAFT SEQUENCE, 10 unordered pieces.//8.0e-40:317:81//AC004046
R-NT2RP2006436//Human DNA sequence *** SEQUENCING IN PROGRESS *** from clone Y738F9, WORKING DRAFT SEQUENCE.//3.2e-42:184:86//AL022345
R-NT2RP2006441//Plasmodium falciparum microsatellite TA80 sequence.//0.00021:188:68//AF010568
R-NT2RP2006454//Plasmodium falciparum chromosome 2, section 60 of 73 of the complete sequence.//0.30:265:60//AE001423
R-NT2RP2006456//Homo sapiens clone 23566 mRNA sequence.//2.5e-104:532:96//AF052098
R-NT2RP2006464//Homo sapiens mRNA for AND-1 protein.//6.6e-108:524:97//AJ006266
R-NT2RP2006467//Sequence 50 from patent US 5691147.//8.3e-22:235:74//I76222
R-NT2RP2006472//Human DNA sequence *** SEQUENCING IN PROGRESS *** from clone 1172A22, WORKING DRAFT SEQUENCE.//5.4e-12:407:62//AL034386
R-NT2RP2006534//Dictyostelium discoideum actin 8 gene, 3' UTR.//0.44:111:65//M25216
R-NT2RP2006554//Plasmodium falciparum chromosome 2, section 7 of 73 of the complete sequence.//0.19:392:58//AE001370
R-NT2RP2006565//Sus scrofa SCAMP 1 gene, exon 9.//1.5e-13:292:68//AJ223742
R-NT2RP2006571//Homo sapiens chromosome 19, cosmid F17972, complete sequence.//0.0024:409:58//AC004660
R-nnnnnnnnnnnn//Human BRCA2 region, mRNA sequence CG005.//3.3e-16:334:64//U50532
R-NT2RP2006598//Mus musculus retinoid X receptor interacting protein (RIP110) mRNA, partial cds.//1.6e-19:448:64//U22015
R-NT2RP3000002//Human DNA sequence from cosmid N104C7 on chromosome 22, complete sequence.//4.4e-14:501:63//Z82246
R-NT2RP3000031//Homo sapiens mRNA for histone deacetylase-like protein (JM21).//5.9e-115:560:97//AJ011972
R-NT2RP3000046//Homo sapiens clone DJ0042M02, WORKING DRAFT SEQUENCE, 20 unordered pieces.//3.9e-57:402:83//AC005995
R-NT2RP3000047//Homo sapiens chromosome 17, clone hRPK.138_P_22, complete sequence.//1.0:158:66//AC005697
R-NT2RP3000050//Human DNA sequence *** SEQUENCING IN PROGRESS *** from clone 451B21, WORKING DRAFT SEQUENCE.//2.7e-32:411:69//AL033522
R-NT2RP3000055//Human DNA sequence *** SEQUENCING IN PROGRESS *** from clone 1000N6, WORKING DRAFT SEQUENCE.//7.9e-17:309:69//AL034378
R-NT2RP3000072//Brassica rapa DNA for S-locus glycoprotein, complete cds.//2.9e-07:516:60//D88192
R-NT2RP3000080//Homo sapiens clone DJ1129D05, complete sequence.//1.7e-27:186:90//AC005630
R-NT2RP3000085//Arabidopsis thaliana acetyl-CoA carboxylase biotin-containing subunit mRNA, nuclear gene encoding chloroplast protein, complete cds.//0.0051:289:59//U-23155
R-NT2RP3000109//HS_3065_A2_D04_MF CIT Approved Human Genomic Sperm Library D Homo sapiens genomic clone Plate-3065 Col=8 Row=G, genomic survey sequence.//2.5e-62:304:100//AQ137776
R-NT2RP3000134//Plasmodium falciparum DNA *** SEQUENCING IN PROGRESS *** from MAL1P3, WORKING DRAFT SEQUENCE.//0.027:414:57//AL031746
R-NT2RP3000142//Homo sapiens mRNA for KIAA0592 protein, partial cds.//3.8e-115:578:96//AB011164
R-NT2RP3000149//Homo sapiens chromosome 17, clone hRPK.332_H_18, complete sequence.//1.3e-67:354:95//AC005746
R-NT2RP3000186
R-NT2RP3000197//Human DNA sequence from PAC 181N1 on chromosome X contains ESTs, STS polymorphic CA repeat*.//2.5e-31:295:78//Z82899
R-NT2RP3000207//Homo sapiens Chromosome 16 BAC clone CIT987SK-A-954B10, complete sequence.//0.016:305:61//AC004514
R-NT2RP3000220//RPCI11-63O7.TJ RPCI11 Homo sapiens genomic clone R-63O7, genomic survey sequence.//0.25:118:66//AQ201832
R-NT2RP3000233//Plasmodium falciparum mRNA for major merozoite surface antigen gp195.//3.2e-11:440:59//X15063
R-NT2RP3000235//Mus musculus chromosome 6 clone TB6 subclone TB6pD1//0.81:114:64//U19530
R-NT2RP3000247//Homo sapiens DNA sequence from clone 326L12 on chromosome Xq27.1 27.3. Contains the cancer/testis antigen CT7 (melanoma-associated antigen MAGE-C1) gene, two MAGE family pseudogenes, STSs and a CA repeat polymorphism, complete sequence.//4.8e-73:362:86//AL023279
R-NT2RP3000251//Homo sapiens chromosome 17, clone hRPK.192_H_23, complete sequence.//0.025:131:66//AC005726
R-NT2RP3000252
R-NT2RP3000255//HS-1025-B2-F08-MF.abi CIT Human Genomic Sperm Library C Homo sapiens genomic clone Plate=CT 804 Col=16 Row=L, genomic survey sequence.//0.67:119:66//B34879
R-NT2RP3000267
R-NT2RP3000299//Rattus norvegicus mRNA for Crk-associated substrate, p130, complete cds.//1.2e-23:424:69//D29766
R-NT2RP3000312//Plasmodium falciparum MAL3P4, complete sequence.//0.55:414:59//AL008970
R-NT2RP3000320//HS_3056_A1_C03_MR CIT Approved Human Genomic Sperm Library D Homo sapiens genomic clone Plate=3056 Col=5 Row=E, genomic survey sequence.//4.1e-32:214:89//AQ134064
R-NT2RP3000324//Rattus norvegicus potassium channel regulator 1 mRNA, complete cds.//1.5e-22:265:75//U78090
R-NT2RP3000333//Plasmodium falciparum MAL3P6, complete sequence.//0.68:460:57//Z98551
R-NT2RP3000341//H.sapiens mRNA for TIM17 preprotein translocase.//1.4e-19:137:90//X97544
R-NT2RP3000348//CITBI-E1-2513C11.TF CITBI-E1 Homo sapiens genomic clone 2513C11, genomic survey sequence.//0.0014:118:72//AQ278177
R-NT2RP3000350
R-NT2RP3000359//Homo sapiens clone NH0319F03, WORKING DRAFT SEQUENCE, 3 unordered pieces.//2.8e-55:320:75//AC006039
R-NT2RP3000361//Homo sapiens mRNA for KIAA0552 protein, complete cds.//0.18:275:61//AB011124
R-NT2RP3000366//CIT-HSP-2317H13.TF CIT-HSP Homo sapiens genomic clone 2317H13, genomic survey sequence.//6.7e-42:214:100//AQ041634
R-NT2RP3000397//HS-1012-B1-F01-MR.abi CIT Human Genomic Sperm Library C Homo sapiens genomic clone Plate=CT 787 Col=1 Row=L, genomic survey sequence//0.015:184:63//B31814
R-NT2RP3000403//Homo sapiens formin binding protein 21 mRNA, complete cds.//1.3e-109:529:98//AF071185
R-NT2RP3000418//Human DNA sequence *** SEQUENCING IN PROGRESS *** from clone 510B21, WORKING DRAFT SEQUENCE.//6.2e-15:445:65//AL031885
R-NT2RP3000433
R-NT2RP3000439
R-NT2RP3000441
R-NT2RP3000449//Human DNA sequence *** SEQUENCING IN PROGRESS *** from clone 1018D12, WORKING DRAFT SEQUENCE.//1.6e-43:300:76//AL031650 R-NT2RP3000451//3'untranslated region of human mRNA for a K⁺ channel protein.//0.71:101:66//E13519
R-NT2RP3000456//Human Xq28 cosmids U126G1, U142F2, U69B6, U145C10, U169A5, U84H1, U24D12, U80A7, U153E6, L35485, and R7-163A8 containing iduronate 2-sulfatase gene and pseudogene, complete sequence.//5.2e-16:376:65//AF011889
R-NT2RP3000484//Human DNA sequence *** SEQUENCING IN PROGRESS *** from clone 120G22, WORKING DRAFT SEQUENCE.//0.61:326:58//AL031847
R-NT2RP3000487//Sequence 32 from patent US 5476781.//8.6e-08:409:61//I16692
R-NT2RP3000512//RPCI11-60F15.TK RPCI11 Homo sapiens genomic clone R-60F15, genomic survey sequence.//2.2e-68:379:93//AQ201516
R-NT2RP3000526//Human DNA sequence *** SEQUENCING IN PROGRESS *** from clone 377F16, WORKING DRAFT SEQUENCE.//4.1e-07:224:65//Z93783
R-NT2RP3000527//HS_3228_A1_H07_T7 CIT Approved Human Genomic Sperm Library D Homo sapiens genomic clone Plate=3228 Col=13 Row=O, genomic survey sequence.//4.5e-30:184:93//AQ209131
R-NT2RP3000531//T6M24-Sp6 TAMU Arabidopsis thaliana genomic clone T6M24, genomic survey sequence.//0.67:88:68//AQ248538
R-NT2RP3000542//Human DNA sequence *** SEQUENCING IN PROGRESS *** from clone 126B4, WORKING DRAFT SEQUENCE.//2.0e-24:145:82//AL022316
R-NT2RP3000561//Homo sapiens PAC clone DJ0942I16 from 7q11, complete sequence.//6.1e-107:548:95//AC006012
R-NT2RP3000562//HS_2041_B1_E08_T7 CIT Approved Human Genomic Sperm Library D Homo sapiens genomic clone Plate=2041 Col=15 Row=J, genomic survey sequence.//9.6e-55:279:98//AQ230207
R-NT2RP3000578//Plasmodium falciparum DNA *** SEQUENCING IN PROGRESS *** from contig 3-105, complete sequence.//0.00060:356:58//AL010212
R-NT2RP3000582//Homo sapiens chromosome 17, clone hCIT.468_F_23, WORKING DRAFT SEQUENCE, 3 unordered pieces.//4.2e-29:282:67//AC004666
R-NT2RP3000584//Human PAC clone DJ222H05 from Xq25-q26, complete sequence.//7.4e-44:245:78//AC002377
R-NT2RP3000590//Arabidopsis thaliana chromosome II BAC T31E10 genomic sequence, complete sequence.//0.66:341:59//AC004077
R-NT2RP3000592//Plasmodium falciparum 3D7 chromosome 12 PFYAC181 genomic sequence, WORKING DRAFT SEQUENCE, 8 unordered pieces.//0.022:491:56//AC005505
R-nnnnnnnnnnnn//HS_3025_A1_D11_MR CIT Approved Human Genomic Sperm Library D Homo sapiens genomic clone Plate=3025 Col=21 Row=G, genomic survey sequence.//2.6e-21:161:88//AQ101452
R-NT2RP3000599//Plasmodium falciparum MAL3P8, complete sequence.//1.3e-09:543:58//AL034560
R-NT2RP3000605//Homo sapiens chromosome 19, cosmid F20900, complete sequence.//5.6e-115:554:98//AC006128
R-NT2RP3000622//Homo sapiens chromosome 12p13.3, WORKING DRAFT SEQUENCE, 27 unordered pieces.//0.15:233:63//AC005414
R-NT2RP3000624//CIT-HSP-2022D4.TR CIT-HSP Homo sapiens genomic clone 2022D4, genomic survey sequence.//1.0:166:66//B64262
R-NT2RP3000628//Human BAC clone GS188P18, complete sequence.//5.3e-56:384:83//AC000115
R-NT2RP3000632//Human cyclin-selective ubiquitin carrier protein mRNA, complete cds.//4.0e-61:438:85//U73379
R-NT2RP3000644//Homo sapiens DNA from chromosome 19p13.2 cosmids R31240, R30272 and R28549 containing the EKLF, GCDH, CRTC, and RAD23A genes, genomic sequence.//1.0e-43:408:77//AD000092
R-NT2RP3000661//F.rubripes GSS sequence, clone 148D22bB9, genomic survey sequence.//2.7e-17:234:69//AL005927
R-NT2RP3000665//Human chromosome 11 46b2 cosmid, complete sequence.//2.1e-42:526:72//U73645
R-NT2RP3000685//HS_3007_A2_F02_T7 CIT Approved Human Genomic Sperm Library D Homo sapiens genomic clone Plate=3007 Col=4 Row=K, genomic survey sequence.//1.6e-101:506:97//AQ118425
R-NT2RP3000690//Plasmodium falciparum MAL3P6, complete sequence.//1.3e-13:411:61//Z98551
R-NT2RP3000736
R-NT2RP3000742//Rattus norvegicus phospholipase C delta-4 mRNA, complete cds.//0.0071:231:65//U16655
R-NT2RP3000753//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs (BAC end sequences) and a CA repeat polymorphism, complete sequence.//0.88:366:56//AL021368
R-NT2RP3000759//HS_2055_A2_D09_MR CIT Approved Human Genomic Sperm Library D Homo sapiens genomic clone Plate=2055 Col=18 Row=G, genomic survey sequence.//0.45:251:60//AQ234828
R-NT2RP3000815//Homo sapiens chromosome 17, clone hRPK.209_J_20, complete sequence.//2.0e-20:293:72//AC005822
R-NT2RP3000825//Plasmodium falciparum MAL3P6, complete sequence.//0.0044:325:62//Z98551
R-NT2RP3000826//Human DNA sequence *** SEQUENCING IN PROGRESS *** from clone 1177I5, WORKING DRAFT SEQUENCE.//5.3e-25:375:72//AL022315
R-NT2RP3000836//Human DNA sequence *** SEQUENCING IN PROGRESS *** from clone Y214H10, WORKING DRAFT SEQUENCE.//1.3e-19:181:81//AL022344
R-NT2RP3000841//Homo sapiens, clone hRPK.1_A_1, complete sequence.//0.20:226:61//AC006196
R-NT2RP3000845//Homo sapiens chromosome 19, cosmid R33632, complete sequence.//6.8e-91:512:92//AC005781
R-NT2RP3000847//***ALU WARNING: Human Alu-Sp subfamily consensus sequence.//7.9e-38:179:86//U14572
R-NT2RP3000850//Homo sapiens BAC clone GS166A23 from 7p21, complete sequence.//4.4e-48:505:76//AC005014
R-NT2RP3000852//Human DNA sequence *** SEQUENCING IN PROGRESS *** from clone 97P20, WORKING DRAFT SEQUENCE.//2.9e-82:311:98//AL031297
R-NT2RP3000859
R-NT2RP3000865//Human DNA sequence from clone 23K20 on chromosome Xq25-26.2 Contains EST, STS, GSS, complete sequence.//1.2e-15:482:63//AL022153 R-NT2RP3000868//Fruitfly strain g20 mitochondrial DNA, A+T-rich region, partial sequence.//0.00045:260:59//AB003097
R-NT2RP3000869//Human DNA sequence *** SEQUENCING IN PROGRESS *** from clone 330012, WORKING DRAFT SEQUENCE.//0.0058:172:64//AL031731
R-NT2RP3000875//H.sapiens /Hepatitis B virus fusion mRNA for mevalonate kinase.//1.4e-99:531:93//X75311
R-NT2RP3000901
R-NT2RP3000904//Genomic sequence for Arabidopsis thaliana BAC T7N9, complete sequence.//0.32:261:57//AC000348
R-NT2RP3000917//Plasmodium falciparum MAL3P7, complete sequence.//0.00092:456:58//AL034559
R-NT2RP3000919
R-NT2RP3000968//H.sapiens mRNA for ribosomal protein S15a.//4.5e-24:375:71//X84407
R-NT2RP3000980//Homo sapiens chromosome 17, clone hRPK.855_D_21, complete sequence.//0.36:186:62//AC006079
R-NT2RP3000994//Plasmodium falciparum 3D7 chromosome 12 PFYACB8-420 genomic sequence, WORKING DRAFT SEQUENCE, 14 unordered pieces.//0.00052:413:60//AC005140
R-NT2RP3001004//Saccharomyces cerevisiae VAR1 gene, mitochondrial gene encoding mitochondrial protein, 3' processing site, partial sequence.//1.1e-07:330:64//U32857
R-NT2RP3001007//Plasmodium falciparum DNA *** SEQUENCING IN PROGRESS *** from contig 4-82, complete sequence.//0.045:286:61//AL010255
R-NT2RP3001055//Human DNA sequence from PAC 27K14 on chromosome Xp11.3-Xp11.4. Contains monoamine oxidase B (MAOB), ESTs and polymorphic CA repeats.//2.3e-56:348:91//Z95125
R-NT2RP3001057//H.sapiens HZF4 mRNA for zinc finger protein.//8.2e-84:531:86//X78927
R-NT2RP3001081//Plasmodium falciparum DNA *** SEQUENCING IN PROGRESS *** from MAL1P3, WORKING DRAFT SEQUENCE.//1.1e-08:537:60//AL031746
R-NT2RP3001084
R-NT2RP3001096
R-NT2RP3001107
R-nnnnnnnnnnnn//Human Chromosome 15q26.1 PAC clone pDJ10k5 containing human DNA polymerase gamma (polg) gene, complete sequence.//7.4e-62:272:73//AC005316
R-NT2RP3001111
R-NT2RP3001113
R-NT2RP3001115//Homo sapiens PAC clone DJ0905J08 from 7p12-p14, complete sequence.//7.2e-112:550:97//AC005189
R-NT2RP3001116//CIT-HSP-2282K23.TR CIT-HSP Homo sapiens genomic clone 2282K23, genomic survey sequence.//0.000.13.160:69//AQ002011
R-NT2RP3001119//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//5.9e-99:497:96//AL031864
R-NT2RP3001120
R-NT2RP3001126//Plasmodium falciparum MAL3P7, complete sequence.//0.035:266:56//AL034559
R-NT2RP3001133
R-NT2RP3001140//Homo sapiens mRNA for KIAA0762 protein, partial cds.//8.1e-114:549:97//AB018305
R-NT2RP3001147//Homo sapiens chromosome 17, clone HCIT187M2, complete sequence.//0.69:198:63//AC004448
R-NT2RP3001150//Human DNA sequence *** SEQUENCING IN PROGRESS *** from clone 423B22, WORKING DRAFT SEQUENCE.//2.4e-108:542:97//AL034379
R-NT2RP3001155//Homo sapiens mRNA for AND-1 protein.//2.9e-116:563:98//AJ006266
R-NT2RP3001176//Plasmodium falciparum 3D7 chromosome 12 PFYAC69 genomic sequence, WORKING DRAFT SEQUENCE, 4 unordered pieces.//0.44:227:62//AC004688
R-NT2RP3001214//Borrelia burgdorferi plasmid lp25, complete plasmid sequence.//0.0023:381:61//AE000785
R-NT2RP3001216//RPCI11-18C15.TPC RPCI-11 Homo sapiens genomic clone RPCI-11-18C15, genomic survey sequence.//7.0e-29:167:97//B88077
R-NT2RP3001221//Homo sapiens clone 14503, WORKING DRAFT SEQUENCE, 1 ordered pieces.//0.020:211:63//AC005827
R-NT2RP3001232//Homo sapiens DNA sequence from PAC 124C6 on chromosome 6q21. Contains genomic marker D6S1603, ESTs, GSSs and a STS with a CA repeat polymorphism, complete sequence.//2.7e-08:390:62//AL021326
R-NT2RP3001236//RPCI11-25C17.TKBR RPCI-11 Homo sapiens genomic clone RPCI-11-25C17, genomic survey sequence.//9.5e-41:217:88//AQ014003
R-NT2RP3001239//Human microtubule-associated protein 1B (MAP1B) gene, complete cds.//2.9e-21:438:63//L06237
R-NT2RP3001245//Homo sapiens DNA sequence from PAC 964D12 on chromosome 1q24-q25. Contains EST, GSS.//0.00026:439:59//AL021398
R-NT2RP3001253//HS_3002_A2_H12_MR CIT Approved Human Genomic Sperm Library D Homo sapiens genomic clone Plate=3002 Col=24 Row=O, genomic survey sequence.//0.98:190:63//AQ251982
R-NT2RP3001260
R-NT2RP3001268//Homo sapiens clone DJ0959C21, WORKING DRAFT SEQUENCE, 2 unordered pieces.//0.012:509:57//AC004936
R-NT2RP3001272//Homo sapiens BAC clone NH0161H12 from 7p14-p15, complete sequence.//2.2e-22:134:87//AC005589
R-NT2RP3001274//Sequence 11 from Patent WO9517522.//0.0058:133:66//A45341
R-NT2RP3001281//Human DNA sequence from PAC 52D1 on chromosome Xq21. Contains CA repeats, STS.//4.4e-55:558:76//Z96811
R-NT2RP3001307//HS_2058_A1_C06_MR CIT Approved Human Genomic Sperm Library D Homo sapiens genomic clone Plate=2058 Col=11 Row=E, genomic survey sequence.//7.2e-33:260:86//AQ305868
R-NT2RP3001318//Homo sapiens PAC clone DJ0649P17 from 7q11.23-q21, complete sequence.//0.27:210:65//AC004848
R-NT2RP3001325
R-NT2RP3001338//Rat tropoelastin gene, intron 17 (partial).//1.0:184:64//M86367
R-NT2RP3001339//Homo sapiens mRNA for KIAA0451 protein, complete cds.//1.2e-112:566:96//AB007920
R-NT2RP30%1340//Homo sapiens HMG box factor SOX-13 mRNA, complete cds.//3.2e-86:450:95//AF083105
R-NT2RP3001355
R-NT2RP3001374//HS_2184_A2_G04_MR CIT Approved Human Genomic Sperm Library D Homo sapiens genomic clone Plate=2184 Col=8 Row=M, genomic survey sequence.//3.7e-10:101:84//AQ024647
R-NT2RP3001383//Plasmodium falciparum chromosome 2, section 34 of 73 of the complete sequence.//7.4e-07:279:63//AE001397
R-NT2RP3001384//Homo sapiens chromosome 19, cosmid R33907, complete sequence.//4.4e-75:382:97//AC005785
R-NT2RP3001392//HS_3078_B2_D05_MR CIT Approved Human Genomic Sperm Library D Homo sapiens genomic clone Plate=3078 Col=10 Row=H, genomic survey sequence.//1.0:164:64//AQ140587
R-NT2RP3001396//RPCI11-63N18.TJ RPCI11 Homo sapiens genomic clone R-63N18, genomic survey sequence.//0.14:242:61//AQ238544
R-NT2RP3001398//Mus musculus zinc finger protein (Zfp64) mRNA, complete cds.//1.8e-10:193:72//U49046
R-NT2RP3001399
R-NT2RP3001407//Caenorhabditis elegans cosmid D1046, complete sequence.//0.0011:392:60//Z68160
R-NT2RP3001420//Human BAC clone GS165I04 from 7q21, complete sequence.//3.7e-29:412:74//AC002379
R-NT2RP3001426//Homo sapiens clone 24616 mRNA sequence.//1.1e-104:550:94//AF052158
R-NT2RP3001427//Caenorhabditis elegans cosmid K11D5.//0.39:174:64//U53152
R-nnnnnnnnnnnn//Human nuclear pore complex-associated protein TPR (tpr) mRNA, complete cds.//1.4e-94:533:91//U69668
R-NT2RP3001432//Homo sapiens DNA sequence from PAC 164C20 on chromosome 6q16.1-22.1. Contains ESTs and GSSs (BAC end sequences), complete sequence.//2.5e-12:415:61//AL009029
R-NT2RP3001447//Homo sapiens PAC clone DJ0828B12 from 7q11.23-q21.1, complete sequence.//5.6e-36:358:77//AC004903
R-NT2RP3001449//Homo sapiens clone 24497 mRNA sequence.//1.5e-100:499:97//AF070630
R-NT2RP3001453//Homo sapiens clone DJ0852024, WORKING DRAFT SEQUENCE, 2 unordered pieces.//4.0e-47:295:86//AC004906
R-NT2RP3001457
R-NT2RP3001459
R-NT2RP3001472//Crithidia fasciculata kinetoplast apocytochrome b gRNA-mRNA chimera, clone:24.//0.33:150:66//D13030
R-NT2RP3001490//Plasmodium falciparum DNA *** SEQUENCING IN PROGRESS *** from contig 3-103, complete sequence.//2.3e-08:483:60//AL010208
R-NT2RP3001495//Human oxidoreductase (HHCMA56) mRNA, complete cds.//4.4e-60:338:93//U13395
R-NT2RP3001497//Homo sapiens multiple membrane spanning receptor TRC8 (TRC8) mRNA, complete cds.//2.1e-110:549:97//AF064801
R-NT2RP3001527//Human DNA sequence *** SEQUENCING IN PROGRESS *** from clone 1125A11, WORKING DRAFT SEQUENCE.//5.3e-32:310:78//AL034549
R-NT2RP3001529//Human Chromosome X, complete sequence.//5.5e-67:280:93//AC002420
R-NT2RP3001538
R-NT2RP3001554//Human microtubule-associated protein la (MAP1A) mRNA, complete cds.//7.8e-16:391:62//U38292
R-NT2RP3001580//Plasmodium falciparum 3D7 chromosome 12 PFYAC69 genomic sequence, WORKING DRAFT SEQUENCE, 4 unordered pieces.//0.00026:456:58//AC004688
R-NT2RP3001587//Homo sapiens HRIHFB2115 mRNA, partial cds.//5.6e-08:86:88//AB015337
R-NT2RP3001589//Homo sapiens chromosome 17, clone hRPK.1096_G_20, complete sequence.//0.066:360:60//AC005410
R-NT2RP3001607//CIT-HSP-2010M8.TR CIT-HSP Homo sapiens genomic clone 2010M8, genomic survey sequence.//0.041:194:67//B53490
R-NT2RP3001608//Human DNA sequence from PAC 296K21 on chromosome X contains cytokeratin exon, delta-aminolevulinate synthase (erythroid); 5-aminolevulinic acid synthase.(EC 2.3.1.37). 6-phosphofructo-2-kinase/fructose-2,6-bisphosphatase (EC 2.7.1.105, EC 3.1.3.46), ESTs and STS.//0.69:151:64//Z83821
R-NT2RP3001621//Human DNA sequence from clone 24o18 on chromosome 6p21:31-22.2 Contains zinc finger protein pseudogene, VNO-type olfactory receptor pseudogene, nuclear envelope pore membrane protein, EST, STS, GSS, complete sequence.//1.4e-46:354:83//AL021808
R-NT2RP3001629//H.sapiens simple DNA sequence region clone wg1a10.//0.99:137:63//X76572
R-NT2RP3001634//Homo sapiens TRIAD1 type I mRNA, complete cds.//8.5e-108:541:96//AF099149
R-NT2RP3001642
R-NT2RP3001646//HS_3218_A2_A01_T7 CIT Approved Human Genomic Sperm Library D Homo sapiens genomic clone Plate=3218 Col=2 Row=A, genomic survey sequence.//2.6e-32:215:91//AQ303003
R-NT2RP3001671//Plasmodium falciparum DNA *** SEQUENCING IN PROGRESS *** from contig 3-88, complete sequence.//0.018:262:61//AL010157
R-NT2RP3001672
R-NT2RP3001676//Homo sapiens cosmid Q95D4, chromosome 21 5' of IFNAR2.//2.1e-48:413:77//AF039905
R-NT2RP3001678//RPCI11-50C17.TK RPCI11 Homo sapiens genomic clone R-50C17, genomic survey sequence.//0.15:232:62//AQ116359
R-NT2RP3001679//Homo sapiens genomic DNA of 8p21.3-p22 anti-oncogene of hepatocellular colorectal and non-small cell lung cancer , segment 3/11.//7.8e-104:549:95//AB020860
R-NT2RP3001688//Homo sapiens PAC clone DJ1048B16 from 7q34-q36, complete sequence.//6.6e-41:291:86//AC006019
R-NT2RP3001690//Plasmodium falciparum chromosome 2, section 52 of 73 of the complete sequence.//3.1e-07:433:59//AE001415
R-NT2RP3001708//Homo sapiens allele 14 fragile site locus (FRA10B) minisatellite sequence.//6.0e-06:237:64//AF053523
R-NT2RP3001712//CITBI-E1-2516N9.TF CITBI-E1 Homo sapiens genomic clone 2516N9, genomic survey sequence.//1.5e-95:456:99//AQ279562
R-NT2RP3001716//Homo sapiens chromosome Y, clone 264,M,20, complete sequence.//0.0012:346:58//AC004617
R-NT2RP3001724//Human HepG2 3' region MboI cDNA, clone hmd6a06m3.//1.3e-27:163:95//D17273
R-NT2RP3001730//Human DNA sequence *** SEQUENCING IN PROGRESS *** from clone 111B22, WORKING DRAFT SEQUENCE.//7.6e-43:409:76//Z98200
R-NT2RP3001739
R-NT2RP3001752//Human clone 23774 mRNA sequence.//1.9e-08:104:84//U79279
R-NT2RP3001753//CIT-HSP-2379P21.TF CIT-HSP Homo sapiens genomic clone 2379P21, genomic survey sequence.//8.8e-06:102:78//AQ113378
R-NT2RP3001764
R-NT2RP3001777//Human mRNA for heparan sulfate proteaglycan (glypican).//0.99:166:66//X54232
R-NT2RP3001782//Homo sapiens mRNA for KIAA0459 protein, partial cds.//1.3e-111:549:97//AB007928
R-NT2RP3001792//Mus musculus myelin gene expression factor (MEF-2) mRNA, partial cds.//1.6e-32:266:83//U13262
R-NT2RP3001799//H.sapiens mRNA for OX40 homologue.//8.5e-44:374:79//X75962
R-NT2RP3001819
R-NT2RP3001844//Caenorhabditis elegans cosmid C54G7.//0.0042:231:63//U40410
R-NT2RP3001854//Plasmodium falciparum strain Dd2 heat shock protein 86 (HSP86), O1 (o1), O3 (o3), O2 (o2), CG8 (cg8), CG4 (cg4), CG3 (cg3), CG9 (cg9), CG1 (cg1), CG6 (cg6), chloroquine resistance candidate protein (cg2), and CG7 (cg7) genes, complete cds.//1.0:404:59//AF030694
R-NT2RP3001855
R-NT2RP3001896//CIT978SK-A-686F10.TV CIT978SK Homo sapiens genomic clone A-636F10, genomic survey sequence.//0.0012:68:82//AQ116409
R-NT2RP3001898//Homo sapiens Chromsome 11p15.5 PAC clone pDJ754h15 containing cdk-inhibitor p57/KIP2 (CDKN1C) gene, complete sequence.//0.37:266:65//AC005950
R-NT2RP3001915//Human BAC clone RG367O17 from 7p15-p21, complete sequence.//0.018:144:66//AC002486
R-NT2RP3001926//Human polyadenylate binding protein (TIA-1) mRNA, complete cds.//2.4e-10:77:100//M77142
R-NT2RP3001929
R-NT2RP3001931//Homo sapiens full-length insert cDNA clone YU73B11.//1.0e-110:562:96//AF087969
R-NT2RP3001938//Human DNA sequence from PAC 447B16 on chromosome Xq13.1-Xq13.3.//0.38:386:56//Z95328
R-NT2RP3001943//Homo sapiens chromosome 5, P1 clone 1076B9 (LBNL H14), complete sequence.//0.87:298:61//AC004500
R-NT2RP3001944//Bos taurus clone CSSM056 satellite DNA sequence.//0.0095:76:78//U03836
R-NT2RP3001969//Homo sapiens chromosome 12p13.3 clone RPCI11-350L7, WORKING DRAFT SEQUENCE, 72 unordered pieces.//7.0e-109:552:96//AC005844
R-NT2RP3001989//Caenorhabditis elegans cosmid C01A2, complete sequence.//0.15:111:68//Z81029
R-NT2RP3002002//Plasmodium falciparum 14-3-3 protein gene, partial cds.//0.016:286:60//AF065987
R-NT2RP3002004//H.sapiens mRNA for FAST kinase.//5.1e-41:335:82//X86779
R-NT2RP3002007
R-NT2RP3002014//Human DNA sequence from clone 228A9 on chromosome 22q12.3-13.32 Contains 85 KDA CALCIUM-INDEPENDENT PHOSPHOLIPASE A2, EST, GSS, CpG island, complete sequence.//6.6e-41:297:86//AL022322
R-NT2RP3002033
R-NT2RP3002045//Drosophila melanogaster fat protein (fat) gene, complete cds.//0.77:320:60//M80537
R-NT2RP3002054//Caenorhabditis elegans cosmid Y69H2, complete sequence.//0.82:362:57//Z98877
R-NT2RP3002056//F.rubripes GSS sequence, clone 020E22bF7, genomic survey sequence.//0.010:185:63//Z87006
R-NT2RP3002057
R-NT2RP3002062//Human BAC clone RG356F09 from 7p21, complete sequence.//1.7e-17:164:81//AC004002
R-nnnnnnnnnnnn
R-NT2RP3002081//HS_3082_A1_G09_MR CIT Approved Human Genomic Sperm Library D Homo sapiens genomic clone Plate=3082 Col=17 Row=M, genomic survey sequence.//4.2e-25 :344:73//AQ122260
R-NT2RP3002097//Homo sapiens Xp22-150 BAC GSHB-309P15 (Genome Systems Human BAC Library) complete sequence.//2.6e-23:212:80//AC006210
R-NT2RP3002102//Homo sapiens BAC clone RG290G13 from 7q21, complete sequence.//0.43:168:64//AC004746
R-NT2RP3002108//CIT-HSP-2346P16.TF CIT-HSP Homo sapiens genomic clone 2346P16, genomic survey sequence.//3.5e-08:110:78//AQ059071
R-NT2RP3002146//Streptococcus gordonii competence factor (comC) and histidine protein kinase (comD) genes, complete cds, and response regulator (comE) gene, partial cds.//0.11:534:55//U80077
R-NT2RP3002147//Human DNA sequence *** SEQUENCING IN PROGRESS *** from clone 329F2, WORKING DRAFT SEQUENCE.//4.1e-108:551:96//AL031710
R-NT2RP3002151//Mus musculus mRNA for Guanine Nucleotide Regulatory Protein, complete cds.//6.8e-62:347:80//AB003503
R-NT2RP3002163//Anolis pulchellus vitellogenin mRNA, partial cds.//0.77:281:63//U46857
R-NT2RP3002165
R-NT2RP3002166//D.sargus satellite DNA (clone PSE3).//0.81:124:62//Z48711
R-NT2RP3002173
R-NT2RP3002181//HS-1042-A2-F01-MR.abi CIT Human Genomic Sperm Library C Homo sapiens genomic clone Plate=CT 824 Col=2 Row=K, genomic survey sequence.//1.3e-35:305:81//B36980
R-NT2RP3002244//Caenorhabditis elegans cosmid R11E3.//0.0024:393:61//AF100669
R-NT2RP3002248//Human DNA sequence from PAC 170A21 on chromosome 22q12-qter contains ESTs.//0.30:217:63//Z82189
R-NT2RP3002255
R-NT2RP3002273//Homo sapiens BAC clone 393I22 from 8q21, complete sequence.//0.84:463:57//AF070717
R-NT2RP3002276//HS_2260_A1_MF_E07 CIT Approved Human Genomic Sperm Library D Homo sapiens genomic clone Plate=2260 Col=13 Row=I, genomic survey sequence.//0.0017:198:63//AQ292491
R-NT2RP3002303//Human HMG-17 gene for non-histone chromosomal protein HMG-17.//7.4e-93:510:93//X13546
R-NT2RP3002304//Human BAC clone GS188P18, complete sequence.//6.3e-09:477:59//AC000115
R-NT2RP3002330//Plasmodium falciparum 3D7 chromosome 12 PFYAC69 genomic sequence, WORKING DRAFT SEQUENCE, 4 unordered pieces.//0.087:388:58//AC004688
R-NT2RP3002343
R-NT2RP3002351//Homo sapiens chromosome Y, clone 264,M,20, complete sequence.//0.20:489:56//AC004617
R-NT2RP3002352//Homo sapiens mRNA for protein encoded by cxorf5 (71-7A) gene.//2.4e-104:516:94//Y15164
R-NT2RP3002455//Homo sapiens mRNA for KIAA0678 protein, partial cds.//4.7e-102:524:95//AB014578
R-NT2RP3002484
R-NT2RP3002501//Human DNA sequence from PAC 92M18, BRCA2 gene region chromosome 13q12-13 contains BRCA2 exons 25, 26 and 27 ESTs and STS.//5.2e-17:232:75//Z73359
R-NT2RP3002512
R-NT2RP3002529//CIT-HSP-2340H2.TR CIT-HSP Homo sapiens genomic clone 2340H2, genomic survey sequence.//0.81:266:58//AQ057387
R-NT2RP3002545//Homo sapiens mRNA for KIAA0729 protein, partial cds.//3.3e-82:438:94//AB018272
R-NT2RP3002549//Medicago truncatula ENBP1 gene, exons 1 to 12.//0.95:381:56//AJ002479
R-NT2RP3002566//HS_2036_A1_D08_MR CIT Approved Human Genomic Sperm Library D Homo sapiens genomic clone Plate=2036 Col=15 Row=G, genomic survey sequence.//0.18:162:64//AQ230627
R-NT2RP3002587//Homo sapiens clone DJ1090E20, WORKING DRAFT SEQUENCE, 4 unordered pieces.//5.1e-15:213:73//AC004956
R-NT2RP3002590//Arabidopsis thaliana genomic DNA; chromosome 5, P1 clone: MXK3, complete sequence.//0.00010:431:59//AB019236
R-NT2RP3002602//Mus musculus stannin gene, complete cds.//1.6e-20:339:70//AF030522
R-NT2RP3002603
R-NT2RP3002631//Homo sapiens chromosome 21 PAC
   RPCIP704A9190Q2.//1.0:241:59//AJ006997
R-NT2RP3002659//Rat sodium-hydrogen exchange protein-isoform 3 (NHE-3) mRNA, complete cds.//6.8e-24:331:76//M85300
R-NT2RP3002660//H.sapiens partial gene for progesterone receptor and Alu element DNA.//9.8e-43:273:82//Z49816
R-NT2RP3002663//Lymnaea stagnalis 16S ribosomal RNA gene, mitochondrial gene encoding ribosomal RNA, partial sequence.//0.60:300:59//U82072
R-NT2RP3002671//S.pombe chromosome III cosmid c553.//1.2e-20:399:66//AL023704
R-NT2RP3002682//RPCI11-44K6.TJ RPCI11 Homo sapiens genomic clone R-44K6, genomic survey sequence.//4.7e-09:122:77//AQ202481
R-NT2RP3002687//P.falciparum complete gene map of plastid-like DNA (IR-B).//1.1e-07:494:59//X95276
R-NT2RP3002688//Human 7SL RNA sequence.//2.7e-32:290:79//X01037
R-NT2RP3002701
R-NT2RP3002713//Human DNA sequence *** SEQUENCING IN PROGRESS *** from clone 167A19, WORKING DRAFT SEQUENCE.//0.95:334:59//AL031427
R-NT2RP3002763//***ALU WARNING: Human Alu-J subfamily consensus sequence.//3.9e-40:288:85//U14567
R-NT2RP3002770//R.prowazekii genomic DNA fragment (clone A615F).//0.21:174:63//Z82710
R-NT2RP3002785//Homo sapiens PAC clone DJ0170D19 from Xq23, complete sequence.//0.78:354:59//AC004822
R-NT2RP3002799//Homo sapiens X-linked anhidroitic ectodermal dysplasia protein gene (EDA), exon 2 and flanking repeat regions.//1.1e-20:161:77//AF003528
R-NT2RP3002810//Caenorhabditis elegans cosmid F10D2.//0.28:441:56//AF022972
R-NT2RP3002818//HS_3053_A2_A08_MR CIT Approved Human Genomic Sperm Library D Homo sapiens genomic clone Plate=3053 Col=16 Row=A, genomic survey sequence.//0.19:220:60//AQ135025
R-NT2RP3002861//P.falciparum complete gene map of plastid-like DNA (IR-B).//9.3e-05:414:60//X95276
R-NT2RP3002869//Homo sapiens chromosome 19, cosmid F21967, complete sequence.//0.14:165 :64//AC005256
R-NT2RP3002876//Human DNA sequence *** SEQUENCING IN PROGRESS *** from clone 50024, WORKING DRAFT SEQUENCE.//2.6e-59:311:96//AL034380
R-NT2RP3002877//Homo sapiens Xp22 bins 87-93 PAC RPCI1-122K4 (Roswell Park Cancer Institute Human PAC Library) complete sequence.//4.6e-24:422:63//AC003035
R-NT2RP3002909//Homo sapiens mRNA for KIAA0771 protein, partial cds.//4.7e-109:570:95//AB018314
R-NT2RP3002911//Homo sapiens BAC clone GS166A23 from 7p21, complete sequence.//3.1e-16:471:64//AC005014
R-NT2RP3002948//, complete sequence.//4.5e-94:516:93//AC005500
R-NT2RP3002953//Homo sapiens chromosome 5, BAC clone 34j15 (LBNL H169), complete sequence.//3.4e-111:566:96//AC005754
R-NT2RP3002955//Plasmodium falciparum chromosome 2, section 28 of 73 of the complete sequence.//0.19:424:58//AE001391
R-NT2RP3002969//Rat mRNA for brain acyl-CoA synthetase II, complete cds.//1.1e-89:562:88//D30666
R-NT2RP3002972//Stealth virus 5 clone C1311 T7 genomic sequence.//1.0:122:67//AF067482
R-NT2RP3002978//Human DNA sequence *** SEQUENCING IN PROGRESS *** from clone 455J7, WORKING DRAFT SEQUENCE.//4.8e-05:249:63//AL031733
R-NT2RP3002988//Human DNA sequence from PAC 106H8 on chromosome 1q24. Contains PHOSPHATIDYLINISITOL-GLYCAN class C (PIG-C) and DYNAMIN-3 genes. Contains ESTs and STSs and a CpG island.//0.0097:246:67//Z97195
R-NT2RP3003008//Mus musculus major histocompatibility locus class III regions Hsc70t gene, partial cds; smRNP, G7A, NG23, MutS homolog, CLCP, NG24, NG25, and NG26 genes, complete cds; and unknown genes.//1.9e-24:188:78//AF109905
R-NT2RP3003032//Arabidopsis thaliana (clone DW1) DNA retrotransposon Ta11-1 integration site.//5.3e-07:376:63//L47211
R-NT2RP3003059//Homo sapiens chromosome 3, clone hRPK.165_I_16, complete sequence//1.4e-13:323:66//AC005669
R-NT2RP3003061//Homo sapiens mRNA from HIV associated non-Hodgkin's lymphoma (clone hll-10).//3.8e-42:265:91//Y16708
R-NT2RP3003068//HS_3214_B2_G09_T7 CIT Approved Human Genomic Sperm Library D Homo sapiens genomic clone Plate=3214 Col=18 Row=N, genomic survey sequence.//0.025:207:64//AQ181894
R-NT2RP3003071//Human DNA sequence *** SEQUENCING IN PROGRESS *** from clone 510D11, WORKING DRAFT SEQUENCE.//0.00014:329:60//Z98044
R-NT2RP3003078//T26A1TF TAMU Arabidopsis thaliana genomic clone T26A1, genomic survey sequence.//0.95:219:63//B27013
R-NT2RP3003101//Plasmodium falciparum 3D7 chromosome 12 PFYAC812 genomic sequence, WORKING DRAFT SEQUENCE, 8 unordered pieces.//1.4e-05:285:62//AC004153
R-NT2RP3003121//Homo sapiens full-length insert cDNA clone ZD62D10.//2.1e-47:242:98//AF086348
R-NT2RP3003133//Human DNA sequence *** SEQUENCING IN PROGRESS *** from clone 228H13, WORKING DRAFT SEQUENCE.//1.4e-21:199:75//AL031985
R-NT2RP3003138//Mouse kif4 mRNA for microtubule-based motor protein KIF4, complete cds.//5.1e-14:287:68//D12646
R-NT2RP3003139//Rattus norvegicus kappa opioid receptor gene, exon 4 and complete cds.//1.5e-13:122:80//U17995
R-NT2RP3003150
R-NT2RP3003157//Homo sapiens 12q15 BAC GSHB-410F4 (Genome Systems Human Bac Library) complete sequence.//5.5e-42:289:74//AC005294
R-NT2RP3003185//HS_2058_A1_H03_T7 CIT Approved Human Genomic Sperm Library D Homo sapiens genomic clone Plate=2058 Col=5 Row=O, genomic survey sequence.//0.025:52:94//AQ231298
R-NT2RP3003193//Homo sapiens chromosome 17, clone hRPK.628_E_12, complete sequence.//4.8e-40:349:79//AC005701
R-NT2RP3003197//Human DNA sequence *** SEQUENCING IN PROGRESS *** from clone 364I1, WORKING DRAFT SEQUENCE.//5.2e-10:180:71//AL031319
R-NT2RP3003203//Mus musculus IFN alpha-treated embryonic fibroblast mRNA.//1.8e-11:148:77//U51904
R-NT2RP3003204//Human DNA sequence *** SEQUENCING IN PROGRESS *** from clone 892F13, WORKING DRAFT SEQUENCE.//6.6e-41:282:86//AL009183
R-NT2RP3003212//Homo sapiens full-length insert cDNA clone ZB91B11.//1.7e-68:363:95//AF086173
R-NT2RP3003230//Caenorhabditis elegans cosmid T12B5.//0.0018:279:64//AF100307
R-NT2RP3003242//Homo sapiens chromosome 7 clone UWGC:g3586a160 from 7p14-15, complete sequence.//1.0:346:57//AC005272
R-NT2RP3003251//Homo sapiens BAC clone RG060N22 from 7q21, complete sequence.//2.5e-10:436:62//AC003083
R-NT2RP3003264//CIT-HSP-2296M7.TR CIT-HSP Homo sapiens genomic clone 2296M7, genomic survey sequence.//5.8e-05:308:61//AQ005862
R-NT2RP3003278//Human HepG2 partial cDNA, clone hmd3b11m5.//9.4e-47:302:89//D17022
R-NT2RP3003282//Homo sapiens dynamin (DNM) mRNA, complete cds.//7.4e-101:550:93//L36983
R-NT2RP3003290//Human DNA sequence *** SEQUENCING IN PROGRESS *** from clone 460J8, WORKING DRAFT SEQUENCE.//3.0e-22:228:78//AL031662
R-NT2RP3003301
R-NT2RP3003302//CIT-HSP-2319H19.TF CIT-HSP Homo sapiens genomic clone 2319H19, genomic survey sequence.//1.5e-69:367:95//AQ034950
R-NT2RP3003311//Plasmodium falciparum 3D7 chromosome 12 PFYAC181 genomic sequence, WORKING DRAFT SEQUENCE, 8 unordered pieces.//5.1e-08:398:64//AC005505
R-NT2RP3003313//Caenorhabditis elegans cosmid F39B1, complete sequence.//0.00022:436:58//Z69660
R-NT2RP3003327//Homo sapiens Chromosome 16 BAC clone CIT987-SKA-237H1 ∼complete genomic sequence, complete sequence.//1.5e-16:334:70//AC002287
R-NT2RP3003330//Homo sapiens full-length insert cDNA YI24C02.//4.4e-96:458:99//AF075015
R-NT2RP3003344//HS_3235_B2_H09_T7 CIT Approved Human Genomic Sperm Library D Homo sapiens genomic clone Plate=3235 Col=18 Row=P, genomic survey sequence.//4.1e-18:197:80//AQ303203
R-NT2RP3003346
R-NT2RP3003353//CITBI-E1-2523B18.TR CITBI-E1 Homo sapiens genomic clone 2523B18, genomic survey sequence.//8.3e-06:130:73//AQ278834
R-NT2RP3003377//Homo sapiens clone DJ0919J22, WORKING DRAFT SEQUENCE, 34 unordered pieces.//1.9e-97:481:94//AC005519
R-NT2RP3003384//Homo sapiens clone DJ0038I10, WORKING DRAFT SEQUENCE, 5 unordered pieces.//2.3e-10:226:71//AC004820
R-NT2RP3003385
R-NT2RP3003403//Human DNA sequence from clone 227L5 on chromosome Xp11.22-11.3. Contains a Keratin, Type 1 Cytoskeletal 18 (KRT18, CYK18, K18, CK18) pseudogene and an STS, complete sequence.//2.8e-40:496:72//AL031585
R-NT2RP3003409//Rat POU domain factor (Brn-5) mRNA.//1.5e-20:375:68//L23204
R-NT2RP3003411//Human DNA sequence *** SEQUENCING IN PROGRESS *** from clone 438L4, WORKING DRAFT SEQUENCE.//1.0:180:61//Z97635
R-NT2RP3003427//RPCI11-45J23.TJ RPCI11 Homo sapiens genomic clone R-45J23, genomic survey sequence.//0.82:162:69//AQ195566
R-NT2RP3003433//Homo sapiens BAC clone NH0044G14 from 7q11.23-21.1, complete sequence.//1.1e-10:379:61//AC006031
R-NT2RP3003464//Homo sapiens rab3-GAP regulatory domain mRNA, complete cds.//1.1e-95:479:96//AF004828
R-NT2RP3003490//Homo sapiens mRNA for KIAA0725 protein, partial cds.//1.3e-100:527:93//AB018268
R-NT2RP3003491//Plasmodium falciparum chromosome 2, section 35 of 73 of the complete sequence.//4.0e-08:495:59//AE001398
R-NT2RP3003500//W.suaveolens mitochondrial ATP9 gene.//0.0074:514:59//X77238
R-NT2RP3003543//Human clone A9A2BRB7 (CAC)n/(GTG)n repeat-containing mRNA.//1.3e-31:217:88//U00952
R-NT2RP3003552
R-NT2RP3003555//Dictyostelium discoideum interaptin (abpD) gene, complete cds.//0.98:321:61//AF057019
R-NT2RP3003564
R-NT2RP3003572//Human DNA sequence from BAC 992D9 on chromosome 22q12.1 contains STS.//0.0015:507:59//AL008638
R-NT2RP3003576//Human Chromosome 16 BAC clone CIT987SK-A-61E3, complete sequence.//1.2e-39:359:79//AC003007
R-NT2RP3003589//Plasmodium falciparum MAL3P8, complete sequence.//0.014:539:58//AL034560
R-NT2RP3003625//Human DNA sequence from clone 1042K10 on chromosome 22q13.1-13.2. Contains the ADSL gene for Adenylosuccinate lyase (EC 4.3.2.2, Adenylosuccinase, ASL) and 4 novel genes (one with probable rabGAP domains and Src homology domain 3). Contains ESTs, STSs, GSSs and a putative CpG island, complete sequence.//1.8e-44:448:77//AL022238
R-NT2RP3003656//Homo sapiens chromosome 17, clone hRPK.401_O_9, complete sequence.//0.34:257:62//AC005291
R-NT2RP3003659//O.fuscipennis 16S rRNA gene, partial.//0.021:145:65//Z93701
R-NT2RP3003665//HS_3078_B2_C09_MR CIT Approved Human Genomic Sperm Library D Homo sapiens genomic clone Plate=3078 Col=18 Row=F, genomic survey sequence.//1.3e-75:397:95//AQ140580
R-NT2RP3003672
R-NT2RP3003686
R-NT2RP3003701//Human BAC clone GS310A05 from 7q21-q22, complete sequence.//6.4e-17:464:62//AC002452
R-NT2RP3003716//Human DNA sequence *** SEQUENCING IN PROGRESS *** from clone 774G10, WORKING DRAFT SEQUENCE.//0.00072:425:62//AL034410
R-NT2RP3003726//Homo sapiens mRNA for KIAA0757 protein, complete cds.//1.7e-101:492:97//AB018300
R-NT2RP3003746//Homo sapiens Chromosome 16 BAC clone CIT987-SK502C10, complete sequence.//3.7e-07:217:66//AC003009
R-NT2RP3003795//Human DNA sequence from clone 505B13 on chromosome 1p36.2-36.3 Contains CA repeat and GSSs, complete sequence.//8.1e-26:456:68//Z98052
R-NT2RP3003799//cSRL-138g10-u cSRL flow sorted Chromosome 11 specific cosmid Homo sapiens genomic clone cSRL-138g10, genomic survey sequence.//4.9e-09:117:77//B01736
R-NT2RP3003800//Homo sapiens tyrosine kinase pp60c-src (SRC) gene, exon 12 and partial cds.//2.8e-106:551:95//AF077754
R-NT2RP3003805
R-NT2RP3003809//Homo sapiens full-length insert cDNA clone YZ95A01.//3.6e-106:533:97//AF086107
R-NT2RP3003819//Human DNA sequence *** SEQUENCING IN PROGRESS *** from clone 34606, WORKING DRAFT SEQUENCE.//6.0e-44:288:81//Z84487
R-NT2RP3003825//Mus domesticus interleukin 1 receptor antagonist (IL-1RA) mRNA.//0.0014:410:58//M64404
R-NT2RP3003828
R-NT2RP3003831//****ALU WARNING: Human Alu-J subfamily consensus sequence.//2.3e-41:289:85//U14567
R-NT2RP3003833//Homo sapiens clones 24718 and 24825 mRNA sequence.//1.6e-108:541:97//AF070611
R-NT2RP3003842//Homo sapiens Xp22 BAC 620F15 (Genome Systems BAC library) complete sequence.//1.5e-46:457:74//AC002980
R-NT2RP3003846//Plasmodium falciparum MAL3P3, complete sequence.//3.5e-06:356:62//Z98547
R-NT2RP3003870//Homo sapiens full-length insert cDNA clone ZD75H11.//8.2e-09:68:98//AF086402
R-NT2RP3003876//Human DNA sequence ∗∗∗ SEQUENCING IN PROGRESS *** from clone 1018D12, WORKING DRAFT SEQUENCE.//0.0027:180:66//AL031650
R-NT2RP3003914//Dictyostelium discoideum DNA for transposable element Tdd-3 tandem array.//0.029:234:62//X53439
R-NT2RP3003918
R-NT2RP3003932//Mus musculus MRC OX-2 antigen homolog gene, exons 2-5, and complete cds.//0.00087:164:67//AF029215
R-NT2RP3003989
R-NT2RP3003992//Sequence 1 from patent US 5591825.//0.56:235:59//I33465
R-NT2RP3004013//HS_3018_A1_G09_MR CIT Approved Human Genomic Sperm Library D Homo sapiens genomic clone Plate=3018 Col=17 Row=M, genomic survey sequence.//0.00026:421:60//AQ119904
R-NT2RP3004016//Drosophila melanogaster DNA sequence (P1s DS03465 (D149) and DS08544 (D187)), complete sequence.//4.8e-12:308:62//AC004532
R-NT2RP3004041//Human DNA sequence ∗∗∗ SEQUENCING IN PROGRESS *** from clone 598F2, WORKING DRAFT SEQUENCE.//0.42:190:64//AL021579
R-NT2RP3004051//Homo sapiens chromosome 19, BAC CIT-B-191n6, complete sequence.//3.6e-21:332:69//AC006130
R-NT2RP3004070//Plasmodium falciparum 3D7 chromosome 12 PFYAC492 genomic sequence, WORKING DRAFT SEQUENCE, 5 unordered pieces.//2.0e-05:476:57//AC005308
R-NT2RP3004078//Homo sapiens chromosome 19, cosmid R30335, complete sequence.//2.0e-86:486:93//AC005784
R-NT2RP3004093//Human PAC clone 257C22A from 13q12-q13, complete sequence.//5.3e-11:230:69//AC002525
R-NT2RP3004095//Homo sapiens clone NH0486I22, WORKING DRAFT SEQUENCE, 5 unordered pieces.//7.5e-93:551:92//AC005038
R-NT2RP3004110//Homo sapiens 12p13.3 PAC RPCI5-940J5 (Roswell Park Cancer Institute Human PAC Library) complete sequence.//1.6e-104:317:100//AC006064
R-NT2RP3004125//Pongo pygmaeus CT microsatellite, clone #3, from the tandemly repeated genes encoding U2 small nuclear RNA (RNU2 locus).//0.73:168:60//U36532
R-NT2RP3004145//Homo sapiens full-length insert cDNA clone ZE09H03.//2.3e-89:427:99//AF086542
R-NT2RP3004148//Arabidopsis thaliana chromosome II BAC T1B8 genomic sequence, complete sequence.//0.013:134:70//U78721
R-NT2RP3004155//Homo sapiens PAC clone DJ0320J15 from Xq23, complete sequence.//3.8e-10:101:87//AC004081
R-NT2RP3004206//Homo sapiens clone DJ0794K21, complete sequence.//1.5e-06:442:57//AC005533
R-NT2RP3004207//Mouse mRNA for seizure-related gene product 6.//1.7e-07:220:69//D29763
R-NT2RP3004209//Human cosmid Q7A10 (D21S246) insert DNA, complete sequence.//7.3e-89:504:92//D42052
R-NT2RP3004215//Caenorhabditis elegans cosmid F11A6, complete sequence.//0.018:353:59//Z81498
R-NT2RP3004242//Plasmodium falciparum chromosome 2, section 52 of 73 of the complete sequence.//4.5e-06:407:60//AE001415
R-NT2RP3004246//Homo sapiens chromosome 10 clone CIT987SK-1010K1 map 10q25, complete sequence.//2.8e-105:534:97//AC005385
R-NT2RP3004253//RPCI11-78J12.TJ RPCI11 Homo sapiens genomic clone R-78J12, genomic survey sequence.//4.0e-64:382:90//AQ281324
R-NT2RP3004258//Rattus norvegicus Zis mRNA, complete cds.//7.0e-60:417:84//AF013967
R-NT2RP3004262//Mus musculus heat shock protein hsp40-3 gene, complete cds.//2.7e-43:528:73//AF092536
R-NT2RP3004334//Homo sapiens chromosome 17, clone hRPC.1110_E_20, complete sequence.//1.4e-06:435:62//AC004231
R-NT2RP3004341//CITBI-E1-2503F11.TR CITBI-E1 Homo sapiens genomic clone 2503F11, genomic survey sequence.//0.0018:210:65//AQ263365
R-NT2RP3004348//Homo sapiens chromosome 17, clone hRPK.85_B_7, complete sequence.//7.1 e-46:340:83//AC005695
R-NT2RP3004349//Human DNA sequence *** SEQUENCING IN PROGRESS *** from clone 117O3, WORKING DRAFT SEQUENCE.//9.4e-29:263:79//AL020995
R-NT2RP3004378//Human DNA sequence from PAC 27K14 on chromosome Xp11.3-Xp11.4. Contains monoamine oxidase B (MAOB), ESTs and polymorphic CA repeats.//2.0e-67:422:90//Z95125
R-NT2RP3004399//HS_3046_A1_E02_MR CIT Approved Human Genomic Sperm Library D Homo sapiens genomic clone Plate=3046 Col=3 Row=I, genomic survey sequence.//0.00014:186:67//AQ137619
R-NT2RP3004424//RPCI11-59I14.TJ RPCI11 Homo sapiens genomic clone R-59I14, genomic survey sequence.//7.4e-71:370:95//AQ201461
R-NT2RP3004428//Caenorhabditis elegans DNA *** SEQUENCING IN PROGRESS *** from clone Y66A7, WORKING DRAFT SEQUENCE.//0.096:205:64//AL022282
R-NT2RP3004451//Arabidopsis thaliana chromosome II BAC F15K20 genomic sequence, complete sequence.//0.0029:396:60//AC005824
R-NT2RP3004454//Homo sapiens mRNA for KIAA0448 protein, complete cds.//2.9e-106:526:98//AB007917
R-NT2RP3004466
R-NT2RP3004470//Homo sapiens chromosome 5, Bac clone 5m9 (LBNL H220), complete sequence.//8.3e-06:229:64//AC005895
R-NT2RP3004472//Plasmodium falciparum 3D7 chromosome 12 PFYAC1383 genomic sequence, WORKING DRAFT SEQUENCE, 3 unordered pieces.//0.87:442:59//AC005504
R-NT2RP3004475//Homo sapiens mRNA for KIAA0456 protein, partial cds.//1.6e-105:521:97//AB007925
R-NT2RP3004480//Mus musculus maternal-embryonic 3 (Mem3) mRNA, complete cds.//3.9e-38:322:81//U47024
R-NT2RP3004490//Homo sapiens PAC clone 166H1 from 12q, complete sequence.//4.2e-96:527:92//AC003982
R-NT2RP3004498//Homo sapiens clone DJ1147A01, WORKING DRAFT SEQUENCE, 25 unordered pieces.//2.3e-43:342:82//AC006023
R-NT2RP3004503//Human cosmid g1572c101, complete sequence.//2.3e-25:392:68//AC000357
R-NT2RP3004504//M.musculus mRNA for CPEB protein.//1.8e-28:387:70//Y08260
R-NT2RP3004507
R-NT2RP3004527//Homo sapiens chromosome 14, BAC CITB-135H17 containing the RAD51L1 gene, complete sequence.//0.68:244:62//AC004518
R-nnnnnnnnnnnn//Mouse oncogene (ect2) mRNA, complete cds.//2.6e-79:525:84//L11316
R-NT2RP3004544
R-NT2RP3004566
R-NT2RP3004569//Plasmodium falciparum 3D7 chromosome 12 PFYAC1122 genomic sequence, WORKING DRAFT SEQUENCE, 3 unordered pieces.//0.96:296:58//AC004709
R-NT2RP3004572//Homo sapiens BAC clone RG281G05 from 7p15-p21, complete sequence.//8.2e-12:457:63//AC005083
R-NT2RP3004578//Homo sapiens mRNA for KIAA0477 protein, complete cds.//2.4e-97:488:96//AB007946
R-NT2RP3004594//Homo sapiens BAC clone NH0436H22 from 2, complete sequence.//1.7e-10:368:61//AC005234
R-NT2RP3004617
R-NT2RP3004618//F2H16TF IGF Arabidopsis thaliana genomic clone F2H16, genomic survey sequence.//0.96:212:64//B26414
R-NT2RP3004670//Homo sapiens GN6ST mRNA for N-acetylglucosamine-6-O-sulfotransferas e (GlcNAc6ST), complete cds.//2.2e-55:291:95//AB014679
R-NT2RP4000008//H.sapiens polyA site DNA sequence.//2.5e-25:202:85//Z24749
R-NT2RP4000023//CIT-HSP-2372A9.TF CIT-HSP Homo sapiens genomic clone 2372A9, genomic survey sequence.//3.6e-51:313:89//AQ112388
R-NT2RP4000035//Homo sapiens clone GS166C05, WORKING DRAFT SEQUENCE, 7 unordered pieces.//4.3e-69:536:81//AC005015
R-NT2RP4000049//Homo sapiens TRAIL receptor 2 mRNA, complete cds.//2.1e-58:289:82//AF016266
R-NT2RP4000051//Homo sapiens Chromosome 22q11.2 Cosmid Clone 20b In DGCR Region, complete sequence.//0.56:462:58//AC000074
R-NT2RP4000078//Plasmodium falciparum 3D7 chromosome 12 PFYAC357 genomic sequence, WORKING DRAFT SEQUENCE, 7 unordered pieces.//0.00021:460:60//AC005506
R-NT2RP4000102//Homo sapiens chromosome 5, PAC clone 17e19 (LBNL H148), complete sequence.//1.6e-08:518:58//AC004648
R-NT2RP4000109//Homo sapiens mRNA for MEGF5, partial cds.//3.5e-106:536:96//AB011538
R-NT2RP4000129//Homo sapiens mRNA for KIAA0483 protein, partial cds.//1.1e-110:554:97//AB007952
R-NT2RP4000147
R-NT2RP4000150//Rat proto-oncogene (Ets-1) mRNA, complete cds.//3.5e-46:395:83//L20681
R-NT2RP4000151
R-NT2RP4000159//Caenorhabditis elegans cosmid R02F11.//0.00011:261:63//AF016439
R-NT2RP4000167//RPCI11-59L8.TK RPCI11 Homo sapiens genomic clone R-59L8, genomic survey sequence.//6.2e-26:163:93//AQ200049
R-NT2RP4000185
R-NT2RP4000210//Homo sapiens mRNA for KIAA0700 protein, partial cds.//4.6e-99:505 :96//AB014600
R-NT2RP4000212//, complete sequence.//1.0e-106:538:96//AC005300
R-NT2RP4000214//Homo sapiens chromosome 19, CIT-HSP-444n24, complete sequence.//1.2e-39:272:88//AC005261
R-NT2RP4000218//Homo sapiens PAC clone DJ0320J15 from Xq23, complete sequence.//1.6e-09:457:60//AC004081
R-NT2RP4000243//Homo sapiens mRNA for cartilage-associated protein (CASP).//9.0e-69:354:96//AJ006470
R-NT2RP4000246//Mus musculus mRNA for NDPP-1 protein, complete cds.//2.0e-27:344:73//D10727
R-NT2RP4000259//Homo sapiens clone 683 unknown mRNA, complete sequence.//9.7e-78:381:99//AF091092
R-NT2RP4000263//CIT-HSP-2336N24.TF CIT-HSP Homo sapiens genomic clone 2336N24, genomic survey sequence.//0.26:124:69//AQ043515
R-nnnnnnnnnnnn//ORF 5' of ECLF2...ECRF3=G protein-coupled receptor homolog [herpesvirus saimiri HVS, host-squirrel monkey, Genomic, 4 genes, 3720 nt].//0.12:326:61//S76368
R-NT2RP4000312//Human DNA sequence from clone 523E19 on chromosome 6p11.2-12.3 Contains ESTs STS and GSSs, complete sequence.//2.2e-111:538:98//AL033384
R-NT2RP4000321//Homo sapiens clone 24453 mRNA sequence.//1.4e-108:515:99//AF070524
R-NT2RP4000323//S.cerevisiae telomeric sequence DNA, clone YLP108CA-2-i.//0.048:107:69//M34311
R-NT2RP4000355//Homo sapiens clone DJ1136A10, WORKING DRAFT SEQUENCE, 4 unordered pieces.//4.3e-39:350:79//AC004972
R-NT2RP4000360//Homo sapiens mRNA for KIAA0738 protein, complete cds.//2.4e-109:520:99//AB018281
R-NT2RP4000367//Homo sapiens IkappaB kinase complex associated protein (IKAP) mRNA, complete cds.//8.7e-109:527:98//AF044195
R-NT2RP4000370//Homo sapiens PAC clone DJ0777O23 from 7p14-p15, complete sequence.//9.9e-25 :348:72//AC005154
R-NT2RP4000376//Rattus norvegicus phospholipase A-2-activating protein (plap) mRNA, complete cds.//2.2e-69:391:89//U17901
R-NT2RP4000381//Homo sapiens chromosome 17, clone hRPK.394_K_10, complete sequence.//0.066:197:63//AC006080
R-NT2RP4000415//345F19.TV CIT978SKA1 Homo sapiens genomic clone A-345F19, genomic survey sequence.//0.10:79:75//B15527
R-NT2RP4000417//Homo sapiens full-length insert cDNA clone ZD52B10.//9.6e-96:468:97//AF086313
R-NT2RP4000424//Homo sapiens DNA sequence from PAC 127D3 on chromosome 1q23-25. Contains FMO2 and FMO3 genes for Flavin-containing Monooxygenase 2 and Flavin-containing Monooxygenase 3 (Dimethylaniline Monooxygenase (N-Oxide 3, EC1.14.13.8, Dimethylaniline Oxidase 3, FMO II, FMO 3), and a gene for another, unknown, Flavin-containing Monooxygenase family protein. Contains ESTs and GSSs, complete sequence.//1.8e-08:489:59//AL021026
R-NT2RP4000448//Plasmodium falciparum 3D7 chromosome 12 PFYAC181 genomic sequence, WORKING DRAFT SEQUENCE, 8 unordered pieces.//3.3e-07:510:60//AC005505
R-NT2RP4000449//HS_2037_B2_A09_T7 CIT Approved Human Genomic Sperm Library D Homo sapiens genomic clone Plate=2037 Col=18 Row=B, genomic survey sequence.//1.3e-58:375:88//AQ243047
R-NT2RP4000455//Phocine herpesvirus type 1 glycoprotein D (gD) gene, partial cds.//0.62:133:63//U92271
R-nnnnnnnnnnnn
R-NT2RP4000480//cSRL-54b11-u cSRL flow sorted Chromosome 11 specific cosmid Homo sapiens genomic clone CSRL-54b11, genomic survey sequence.//2.1e-19:145:88//B05082
R-nnnnnnnnnnnn
R-NT2RP4000500
R-NT2RP4000515//Plasmodium falciparum 3D7 chromosome 12 PFYACB8-420 genomic sequence, WORKING DRAFT SEQUENCE, 14 unordered pieces.//1.4e-05:411:59//AC005140
R-NT2RP4000517//Human Chromosome 16 BAC clone CIT987SK-A-61E3, complete sequence.//2.7e-21:230:77//AC003007
R-NT2RP4000518//Homo sapiens DNA sequence from PAC 206D15 on chromosome 1q24. Contains a Reduced Folate Carrier protein (RFC) LIKE gene, a mitochondrial ATP Synthetase protein 8 (ATP8, MTATP8) LIKE pseudogene, an unknown gene and the last exon of the JEM1 gene coding for the Basic-Leucine Zipper nuclear factor JEM-1. Contains ESTs, an STS and a BAC end sequence (GSS), complete sequence.//0.0080:461:59//AL021068
R-NT2RP4000519
R-NT2RP4000524
R-NT2RP4000528//Homo sapiens chromosome 17, clone hRPK.138_P_22, complete sequence.//0.99:158:66//AC005697
R-NT2RP4000541//Homo sapiens Chromosome 22q11.2 Cosmid Clone 33e In DGCR Region, complete sequence.//1.0:309:59//AC000078
R-NT2RP4000556//Rattus norvegicus cell cycle protein p55CDC gene, complete cds.//0.0031:126:72//AF052695
R-NT2RP4000588//Homo sapiens BAC clone RG208K23 from 7q31, complete sequence.//1.0:186:64//AC004161
R-NT2RP4000614//Plasmodium falciparum DNA *** SEQUENCING IN PROGRESS *** from contig 3-62, complete sequence.//1.4e-06:526:58//AL009013
R-NT2RP4000638//Homo sapiens chromosome 17, clone hCIT.468_F_23, WORKING DRAFT SEQUENCE, 3 unordered pieces.//6.9e-48:497:75//AC004666
R-NT2RP4000648//CIT-HSP-2300I7.TR CIT-HSP Homo sapiens genomic clone 2300I7, genomic survey sequence.//0.22:110:68//AQ012747
R-NT2RP4000657//Lycodichthys dearborni type III antifreeze peptide gene, clone 5'LD-1/NotI-EcoRI subclone Sphl-Xbal, partial cds.//0.0065:189:63//U20443
R-NT2RP4000704//Human DNA sequence *** SEQUENCING IN PROGRESS *** from clone 409J21, WORKING DRAFT SEQUENCE.//0.22:334:60//Z83824
R-NT2RP4000724//Homo sapiens Chromosome 22q11.2 Cosmid Clone 56c In DGCR Region, complete sequence.//2.2e-70:448:88//AC000080
R-NT2RP4000728//CIT-HSP-2310K14.TF CIT-HSP Homo sapiens genomic clone 2310K14, genomic survey sequence.//0.00013:289:61//AQ019669
R-NT2RP4000739//Homo sapiens chromosome 12p13.3, WORKING DRAFT SEQUENCE, 21 unordered pieces.//0.53:254:61//AC004765
R-NT2RP4000781//P.cepacia fusaric acid-resistance genes encoding 5 proteins, complete cds.//1.0:392:59//D12503
R-NT2RP4000817//Homo sapiens Xp22 BAC GSHB 526D21 (Genome Systems Human BAC library) complete sequence.//0.59:378:58//AC003037
R-NT2RP4000833//Human DNA sequence *** SEQUENCING IN PROGRESS *** from clone Y313F4, WORKING DRAFT SEQUENCE.//3.4e-53:307:85//AL023808
R-NT2RP4000837//Homo sapiens T-cell receptor alpha delta locus from bases 501613 to 752736 (section 3 of 5) of the Complete Nucleotide Sequence.//7.0e-50:367:77//AE000660
R-NT2RP4000855
R-NT2RP4000865//Homo sapiens chromosome 17, clone HRPC905N1, complete sequence.//1.5e-78:479:88//AC003098
R-NT2RP4000878//Mus musculus mRNA for myeloid associated differentiation protein.//4.5e-09:186:69//AJ001616
R-NT2RP4000879//Plasmodium falciparum 3D7 chromosome 12 PFYAC812 genomic sequence, WORKING DRAFT SEQUENCE, 8 unordered pieces.//7.8e-08:364:60//AC004153
R-nnnnnnnnnnnn//Human S-adenosylmethionine decarboxylase (AMD1) gene, exons 5-9.//3.5e-90:459:96//M88006
R-nnnnnnnnnnnn//H.sapiens ung gene for uracil DNA-glycosylase.//7.6e-09:392:61//X89398
R-NT2RP4000925//Rattus norvegicus Shal-related potassium channel Kv4.3 mRNA, complete cds.//5.8e-45:264:92//U42975
R-nnnnnnnnnnnn//epstein-barr virus simple repeat array (ir3).//0.00012:367:61//J02079
R-NT2RP4000928//Arabidopsis thaliana genomic DNA, chromosome 5, P1 clone: MCL19, complete sequence.//1.0:138:68//AB006698
R-NT2RP4000929//Human DNA sequence from PAC 293L6 on chromosome 22, complete sequence.//0.45:288:62//Z82197
R-NT2RP4000955//Human DNA sequence *** SEQUENCING IN PROGRESS *** from clone 633O19, WORKING DRAFT SEQUENCE.//1.1e-09:322:62//AL022302
R-NT2RP4000973//Homo sapiens X-linked anhidroitic ectodermal dysplasia protein gene (EDA), exon 2 and flanking repeat regions.//2.3e-06:326:62//AF003528
R-NT2RP4000975
R-NT2RP4000979//HS_3009_B1_F08_MR CIT Approved Human Genomic Sperm Library D Homo sapiens genomic clone Plate=3009 Col=15 Row=L, genomic survey sequence.//2.3e-14:117:89//AQ090957
R-NT2RP4000984//Human immunodeficiency virus type 1 envelope glycoprotein (env) gene, C2-V3 region, isolate HIV194UG011TIN.01_di1PD, partial cds.//0.11:219:62//U44882
R-NT2RP4000989//Sequence 30 from patent US 5552281.//3.5e-25:154:97//I25669
R-NT2RP4000996//Plasmodium falciparum strain Dd2 heat shock protein 86 (HSP86), O1 (o1), O3 (o3), O2 (o2), CG8 (cg8), CG4 (cg4), CG3 (cg3), CG9 (cg9), CG1 (cg1), CG6 (cg6), chloroquine resistance candidate protein (cg2), and CG7 (cg7) genes, complete cds.//3.8e-07:421:59//AF030694
R-NT2RP4000997//Homo sapiens chromosome 17, clone 104H12, complete sequence.//4.2e-37:499:72//AC000003
R-NT2RP4001004//HS_3163_A2_H02_MR CIT Approved Human Genomic Sperm Library D Homo sapiens genomic clone Plate=3163 Col=4 Row=O, genomic survey sequence.//2.8e-38:241:90//AQ168515
R-NT2RP4001006//Homo sapiens clone DJ1147A01, WORKING DRAFT SEQUENCE, 25 unordered pieces.//7.1e-55:372:73//AC006023
R-NT2RP4001010//Homo sapiens full-length insert cDNA clone ZD38E12.//3.3e-09:153:74//AF086247
R-NT2RP4001029//Mus domesticus nuclear binding factor NF2d9 mRNA, complete cds.//2.1e-34:361:78//U20086
R-NT2RP4001041//Homo sapiens chromosome 5, BAC clone 282B7 (LBNL H192), complete sequence.//9.9e-84:435:96//AC005216
R-NT2RP4001057//Homo sapiens KIAA0399 mRNA, partial cds.//6.2e-50:282:94//AB007859
R-NT2RP4001064//H.sapiens NOS2 gene, exon 15.//0.71:183:61//X85771
R-NT2RP4001078//Human D-site binding protein gene, exon 4 and complete cds.//1.9e-114:569:97//U48213
R-NT2RP4001079//Homo sapiens mRNA for putative Ca2⁺-transporting ATPase, partial.//2.4e-118:574:98//AJ010953
R-NT2RP4001080//Plasmodium falciparum chromosome 2, section 66 of 73 of the complete sequence.//0.013:430:58//AE001429
R-nnnnnnnnnnnn//Homo sapiens mRNA for KIAA0592 protein, partial cds.//1.8e-119:548:95//AB011164
R-NT2RP4001095//Homo sapiens cosmids IM0525, LC1233, Qc3C1, LB1439, Qc12C11 and 220B3 from Xq28, complete sequence.//2.8e-39:312:81//AF003626
R-NT2RP4001100//Human DNA sequence from cosmid U85A3, between markers DXS366 and DXS87 on chromosome X contains rad21 and T-cell cyclophorin pseudogenes, STS.//8.7e-41:389:78//Z78021
R-NT2RP4001117//Canis familiaris sec61 homologue mRNA, complete cds.//2.8e-12:292:68//M96629
R-NT2RP4001122//Caenorhabditis elegans cosmid F44D12, complete sequence.//0.97:129:66//Z68298
R-NT2RP4001126//HS_3146_A1_805_T7 CIT Approved Human Genomic Sperm Library D Homo sapiens genomic clone Plate=3146 Col=9 Row=C, genomic survey sequence.//0.013:268:63//AQ141093
R-NT2RP4001138
R-NT2RP4001143//Human DNA sequence *** SEQUENCING IN PROGRESS *** from clone 64K7, WORKING DRAFT SEQUENCE.//1.8e-31:380:68//AL031668
R-NT2RP4001148//Homo sapiens clone RG332P12, WORKING DRAFT SEQUENCE, 1 unordered pieces.//1.2e-83:325:92//AC005095
R-NT2RP4001149//Mouse mRNA for thymic epithelial cell surface antigen, complete cds.//8.1e-32:553:67//D67067
R-NT2RP4001150//AK011 Genomic DNA Hordeum vulgare genomic clone tel44a similar to barley TAS, genomic survey sequence.//0.91:132:63//AQ248412
R-NT2RP4001159//Cloning vector pAP3neo DNA, complete sequence.//4.0e-118:437:97//AB003468
R-NT2RP4001174//Homo sapiens 12q24 BAC RPCI11-162P23 (Roswell Park Cancer Institute Human BAC library) complete sequence.//1.7e-33:289:82//AC002996
R-nnnnnnnnnnnn//P.falciparum mRNA for AARP2 protein.//0.93:187:64//Y08924
R-NT2RP4001207
R-NT2RP4001210//CIT-HSP-2042D13.TF CIT-HSP Homo sapiens genomic clone 2042D13, genomic survey sequence.//3.8e-06:268:63//B74772
R-NT2RP4001213//Human zinc finger protein 20 (ZNF20) pentanucleotide repeat polymorphism.//4.7e-16:371:66//M99593
R-NT2RP4001219//HS_2190_A1_A06_T7 CIT Approved Human Genomic Sperm Library D Homo sapiens genomic clone Plate=2190 Col=11 Row=A, genomic survey sequence.//2.4e-06:288:61//AQ216635
R-NT2RP4001228//Plasmodium falciparum DNA *** SEQUENCING IN PROGRESS *** from MAL1P2, WORKING DRAFT SEQUENCE.//0.024:357:58//AL031745
R-NT2RP4001235//HS_3047_A1_E07_MR CIT Approved Human Genomic Sperm Library D Homo sapiens genomic clone Plate=3047 Col=13 Row=L, genomic survey sequence.//0.0033:301:63//AQ126918
R-NT2RP4001256//HS_3007_A2_B06_T7 CIT Approved Human Genomic Sperm Library D Homo sapiens genomic clone Plate=3007 Col=12 Row=C, genomic survey sequence.//1.5e-11:140:80//AQ118389
R-NT2RP4001260//Plasmodium falciparum chromosome 2, section 63 of 73 of the complete sequence.//0.0013:486:59//AE001426
R-NT2RP4001274//RPCI11-24O21.TKBF RPCI-11 Homo sapiens genomic clone RPCI-11-24O21, genomic survey sequence.//3.9e-25:142:99//AQ013887
R-nnnnnnnnnnnn//Homo sapiens full-length insert cDNA clone ZD55D10.//1.2e-10:90:92//AF086334
R-NT2RP4001313//Mus musculus orphan nuclear hormone receptor (CAR) gene, complete sequence.//7.7e-23:466:66//AF009326
R-NT2RP4001315//CIT-HSP-2312C6.TR CIT-HSP Homo sapiens genomic clone 2312C6, genomic survey sequence.//0.98:305:62//AQ018036
R-NT2RP4001339
R-NT2RP4001345
R-NT2RP4001351//Fruitfly strain g20 mitochondrial DNA, A+T-rich region, partial sequence.//0.00082:260:59//AB003097
R-NT2RP400l353//RPCI11-55N17.TJ RPCI11 Homo sapiens genomic clone R-55N17, genomic survey sequence.//0.74:106:66//AQ081821
R-NT2RP4001372
R-NT2RP4001373//Homo sapiens chromosome 17, clone hRPK.394_K_10, complete sequence.//1.5e-09:473:60//AC006080
R-NT2RP4001375
R-NT2RP4001379//CIT-HSP-2335A10.TF CIT-HSP Homo sapiens genomic clone 2335A10, genomic survey sequence.//9.4e-41:441:75//AQ040083
R-NT2RP4001389//Homo sapiens PAC clone DJ0740D02 from 7p14-p15, complete sequence.//2.4e-22:276:73//AC004691
R-NT2RP4001407//Plasmodium falciparum 3D7 chromosome 12 PFYACB8-420 genomic sequence, WORKING DRAFT SEQUENCE, 14 unordered pieces.//0.49:254:61//AC005140
R-NT2RP4001414
R-NT2RP4001433//Human prohibitin (PHB) gene, exons 1-7.//6.6e-66:357:90//L14272
R-NT2RP4001442//Plasmodium falciparum 3D7 chromosome 12 PFYAC492 genomic sequence, WORKING DRAFT SEQUENCE, 5 unordered pieces.//0.11:307:59//AC005308
R-NT2RP4001447//cSRL-58d2-u cSRL flow sorted Chromosome 11 specific cosmid Homo sapiens genomic clone cSRL-58d2, genomic survey sequence.//0.0039:112:71//B05220
R-NT2RP4001474
R-NT2RP4001483
R-NT2RP4001498//Plasmodium falciparum (clone Dd2) heat shock protein 86 gene, complete cds.//1.2e-07:339:61//L34027
R-NT2RP4001502//HS_2187_B1_C10_MR CIT Approved Human Genomic Sperm Library D Homo sapiens genomic clone Plate=2187 Col=19 Row=F, genomic survey sequence.//1.3e-20:183:81//AQ214108
R-NT2RP4001507//Arabidopsis thaliana chromosome 1 BAC T17H3 sequence, WORKING DRAFT SEQUENCE, 4 unordered pieces.//0.15:333:62//AC005916
R-NT2RP4001524//Genomic sequence from Human 13, complete sequence.//0.96:159:65//AC001226
R-NT2RP4001529//Mus domesticus nuclear binding factor NF2d9 mRNA, complete cds.//9.5e-34:337:80//U20086
R-NT2RP4001547//Plasmodium falciparum 3D7 chromosome 12 PFYAC588 genomic sequence, WORKING DRAFT SEQUENCE, 2 unordered pieces.//0.00027:336:63//AC004710
R-nnnnnnnnnnnn//Arabidopsis thaliana BAC T12H20.//1.5e-11:517:60//AF080119
R-NT2RP4001555//Human DNA sequence from PAC 481A17 on chromosome X contains ESTs.//0.0069:305:62//Z82212
R-NT2RP4001567//RPCI11-61A2.TJ RPCI11 Homo sapiens genomic clone R-61A2, genomic survey sequence.//0.0072:180:60//AQ200771
R-NT2RP4001568
R-NT2RP4001571//Trypanoplasma borreli kinetoplast ribosomal protein S12 (RPS12), putative cryptogene (GRII), 12S ribosomal RNA, and apocytochrome b (CYb) genes, primary transcripts, and cytochrome c oxidase subunit III (COIII) gene, complete cds.//1.6e-09:555:58//U14181
R-NT2RP4001574//HS_2247_B1_B05_MR CIT Approved Human Genomic Sperm Library D Homo sapiens genomic clone Plate=2247 Col=9 Row=D, genomic survey sequence.//1.1e-41:254:90//AQ182345
R-NT2RP4001575//Human DNA sequence from clone 1033B10 on chromosome 6p21.2-21.31. Contains the BING5 gene, exons 11 to 15 of the BING4 gene, the gene for GalT3 (beta3-Galactosyltransferase), the RPS18 (40S ribosomal protein S18) gene, the SACM2L (suppressor of actin mutation 2, yeast, homolog) gene, a pseudogene similar to TAT-SF1, a Pseudogene similar to zinc finger genes, the RING1 gene, the gene for HKE6 (RING2), the gene for HKE4 (RING5), the RXRB (Retinoid X receptor beta) gene, the COL11A2 (collagen, type XI, alpha 2) gene, the HLA-DPB2 pseudogene and part of the HLA-DPA3 pseudogene. Contains predicted CpG islands, ESTs, STSs, and GSSs, complete sequence.//1.1e-118:567:98//AL031228
R-NT2RP4001592//Human DNA sequence *** SEQUENCING IN PROGRESS *** from clone 1018D12, WORKING DRAFT SEQUENCE.//2.5e-09:370:61//AL031650
R-NT2RP4001610//Homo sapiens Xp22 Cosmids U15E4, U115H5, U132E12, U115B9 (Lawrence Livermore human cosmid library) complete sequence.//0.99:73:75//AC002364
R-NT2RP4001614
R-NT2RP4001634//Homo sapiens full-length insert cDNA clone YU73B11.//5.8e-101:526:94//AF087969
R-NT2RP4001638//Homo sapiens clone 23967 unknown mRNA, partial cds.//5.4e-115:559:97//AF007151
R-NT2RP4001644//M.musculus mRNA for map kinase interacting kinase, Mnk2.//6.8e-33:286:79//Y11092
R-NT2RP4001656//Human Chromosome 11 pac pDJ393o15, WORKING DRAFT SEQUENCE, 8 unordered pieces.//2.2e-109:515:99//AC000384
R-NT2RP4001677//Genomic sequence from Human 9q34, complete sequence.//0.19:504:58//AC000397
R-NT2RP4001696//Human chromosome 8 BAC clone CIT987SK-2A8 complete sequence.//4.5e-115:583:96//U96629
R-NT2RP4001725//Human Chromosome 3 pac pDJ70i11, WORKING DRAFT SEQUENCE, 2 unordered pieces.//0.98:301:60//AC000380
R-nnnnnnnnnnnn//Caenorhabditis elegans cosmid F48E3.//2.2e-17:328:64//U28735
R-NT2RP4001739//RPCI11-74E7.TJ RPCI11 Homo sapiens genomic clone R-74E7, genomic survey sequence.//1.1e-08:141:65//AQ268408
R-NT2RP4001753//H.sapiens HZF3 mRNA for zinc finger protein.//1.7e-111:552:96//X78926
R-NT2RP4001760//Mouse oncogene (ect2) mRNA, complete cds.//9.3e-27:358:72//L11316
R-NT2RP4001790//Homo sapiens clone GS259H13, WORKING DRAFT SEQUENCE, 4 unordered pieces.//1.7e-99:484:98//AC005020
R-NT2RP4001803//HS_3087_B2_B05_MF CIT Approved Human Genomic Sperm Library D Homo sapiens genomic clone Plate=3087 Col=10 Row=D, genomic survey sequence.//2.7e-96:471:97//AQ121405
R-NT2RP4001822
R-NT2RP4001823
R-NT2RP4001828//Human DNA sequence from PAC 179115, BRCA2 gene region chromosome 13q12-q13 contains Klotho ESTs and CpG island.//4.1e-14:136:83//Z92540
R-NT2RP4001838//Plasmodium falciparum chromosome 2, section 9 of 73 of the complete sequence.//2.5e-06:418:60//AE001372
R-NT2RP4001849//P.falciparum serine rich protein (SERP I) gene.//0.64:135:67//J03983
R-NT2RP4001889//Homo sapiens PAC clone DJ1182N03 from 7q11.23-q21.1, complete sequence.//4.3e-26:212:82//AC004548
R-NT2RP4001893//Homo sapiens BAC clone GS166A23 from 7p21, complete sequence.//1.8e-111:570:96//AC005014
R-NT2RP4001896
R-NT2RP4001901
R-NT2RP4001927//Borrelia burgdorferi (section 32 of 70) of the complete genome.//1.0:242:60//AE001146
R-NT2RP4001938//Human aminopeptidase N gene, exon 1.//3.3e-42:195:85//M55523
R-NT2RP4001946//Plasmodium falciparum 3D7 chromosome 12 PFYAC293 genomic sequence, WORKING DRAFT SEQUENCE, 9 unordered pieces.//0.97:371:57//AC004157
R-NT2RP4001950//RPCI11-69C18.TJ RPCI11 Homo sapiens genomic clone R-69C18, genomic survey sequence.//4.7e-91:552:89//AQ236641
R-NT2RP4001953//Homo sapiens DNA sequence from PAC 958B3 on chromosome Xp22.11-Xp22.22. Contains ESTs STS and CpG island.//6.6e-70:325:84//Z93023
R-NT2RP4001966//Rat mRNA for growth potentiating factor, complete cds.//5.5e-37:141:86//D42148
R-NT2RP4001975//Human Newcastle disease virus inducible protein mRNA, partial 3'UTR region.//1.0e-46:242:98//U25276
R-NT2RP4002018//RPCI11-76I23.TV RPCI11 Homo sapiens genomic clone R-76I23, genomic survey sequence.//7.9e-89:438:97//AQ268536
R-NT2RP4002047//Human DNA sequence *** SEQUENCING IN PROGRESS *** from clone 97P20, WORKING DRAFT SEQUENCE.//4.1e-07:325:62//AL031297
R-NT2RP4002052//Human DNA sequence from clone 352E11 on chromosome 22q13.1-13.31. Contains GSSs, complete sequence.//0.31:452:57//AL022353
R-NT2RP4002058//RPCI11-69O1.TJ RPCI11 Homo sapiens genomic clone R-69O1, genomic survey sequence.//0.23:163:64//AQ268418
R-NT2RP4002071//Human DNA sequence ∗∗∗ SEQUENCING IN PROGRESS *** from clone 1172A22, WORKING DRAFT SEQUENCE.//1.1e-11:407:62//AL034386
R-NT2RP4002075//Human DNA sequence from clone 21F7 on chromosome 6q16.1-21. Contains part of an exon of a putative new gene and STSs and GSSs, complete sequence.//0.085:350:61//AL033375
R-NT2RP4002078//RPCI11-79I16.TV RPCI11 Homo sapiens genomic clone R-79I16, genomic survey sequence.//3.3e-87:452:95//AQ283131
R-nnnnnnnnnnnn
R-NT2RP4002083//Homo sapiens mineralocorticoid receptor (MLR), exon 5.//0.50:256:61//AF068619
R-NT2RP4002408//CIT-HSP-2376023.TF CIT-HSP Homo sapiens genomic clone 2376O23, genomic survey sequence.//6.8e-62:320:96//AQ111163
R-NT2RP4002791//Human PAC clone DJ318C15 from Xq23, complete sequence.//0.022:435:61//AC002476
R-NT2RP4002888//Homo sapiens BAC clone RG067E13 from 7q21, complete sequence.//6.0e-56:660:71//AC002383
R-NT2RP4002905//Plasmodium falciparum DNA *** SEQUENCING IN PROGRESS *** from contig 3-20, complete sequence.//0.0017:533:57//AL008972
R-OVARC1000001//Homo sapiens mRNA for KIAA0465 protein, partial cds.//8.7e-114:605:94//AB007934
R-OVARC1000004//Homo sapiens chromosome 4 clone B368A9 map 4q25, complete sequence.//2.1e-43:326:74//AC005510
R-OVARC1000006//HS_2253_B1_F01_MR CIT Approved Human Genomic Sperm Library D Homo sapiens genomic clone Plate=2253 Col=1 Row=L, genomic survey sequence.//3.7e-35:191:98//AQ069124
R-OVARC1000013//HS_2212_A2_G06_MF CIT Approved Human Genomic Sperm Library D Homo sapiens genomic clone Plate=2212 Col=12 Row=M, genomic survey sequence.//0.14:212:63//AQ210584
R-OVARC1000014//Human DNA sequence from PAC 463A9, on chromosome Xq25 contains STS.//0.0053:356:62//Z80232
R-OVARC1000017
R-OVARC1000035//RPCI11-65E1.TJ RPCI11 Homo sapiens genomic clone R-65E1, genomic survey sequence.//3.3e-05:236:63//AQ237194
R-OVARC1000058//Homo sapiens DNA sequence from BAC 390C10 on chromosome 22q11.21-12.1. Contains an Immunoglobulin LIKE gene and a pseudogene similar to Beta Crystallin. Contains ESTs, STSs, GSSs and taga and tat repeat polymorphisms, complete sequence.//2.7e-48:325:82//AL008721
R-OVARC1000060//Human DNA sequence *** SEQUENCING IN PROGRESS *** from clone 27K12, WORKING DRAFT SEQUENCE.//5.0e-21:297:70//AL033397
R-OVARC1000068//P.falciparum complete gene map of plastid-like DNA (IR-B).//0.00038:553:58//X95276
R-OVARC1000071//Human DNA sequence *** SEQUENCING IN PROGRESS *** from clone 596C15, WORKING DRAFT SEQUENCE.//5.1e-110:599:93//AL031387
R-OVARC1000085//DNA encoding component HC5 of human proteasome.//2.7e-65:366:92//E03413
R-nnnnnnnnnnnn//CIT-HSP-2172N17.TF CIT-HSP Homo sapiens genomic clone 2172N17, genomic survey sequence.//0.80:285:59//B94391
R-OVARC1000091
R-OVARC1000092//CIT-HSP-2373J20.TR CIT-HSP Homo sapiens genomic clone 2373J20, genomic survey sequence.//1.4e-17:141:85//AQ111520
R-OVARC 1000106
R-OVARC1000113//Homo sapiens okadaic acid-inducible phosphoprotein (OA48-18) mRNA, complete cds.//2.6e-100:495:97//AF069250
R-OVARC1000114//Homo sapiens partial XPGC gene, exon 2.//9.5e-49:392:80//X71342
R-OVARC1000133//Human Chromosome 16 BAC clone CIT987SK-A-362G6, complete sequence.//0.00020:243:65//U95740
R-OVARC1000145//Homo sapiens chromosome 10 clone CIT987SK-1010K1 map 10q25, complete sequence.//1.8e-16:370:67//AC005385
R-OVARC1000148//CIT-HSP-2386P14.TF.1 CIT-HSP Homo sapiens genomic clone 2386P14, genomic survey sequence.//1.1e-05:55:98//AQ240492
R-OVARC1000151//M.musculus GEG-154 mRNA.//9.8e-21:192:81//X71642
R-OVARC1000168//CIT-HSP-2336F6.TR CIT-HSP Homo sapiens genomic clone 2336F6, genomic survey sequence.//0.050:176:62//AQ042932
R-OVARC1000191//Plasmodium falciparum 3D7 chromosome 12 PFYAC357 genomic sequence, WORKING DRAFT SEQUENCE, 7 unordered pieces.//3.7e-08:534:58//AC005506
R-OVARC1000198//*** SEQUENCING IN PROGRESS *** Homo sapiens chromosome 4, BAC clone C0366H07; HTGS phase 1, WORKING DRAFT SEQUENCE, 28 unordered pieces.//5.2e-111:556:96//AC004604
R-OVARC1000209//Blacus sp. 16S ribosomal RNA gene, partial sequence.//0.55:165:67//AF003501
R-OVARC1000212//Mouse DNA for beta-casein.//0.56:225:63//X13484
R-OVARC1000240//Homo sapiens chromosome 17, clone hRPK.63_A_1, complete sequence.//6.2e-38:193:82//AC005670
R-OVARC1000241//Mus musculus hypoxia inducible factor three alpha mRNA, complete cds.//1.1e-25:312:73//AF060194
R-OVARC1000288//Human HepG2 3' region MboI cDNA, clone hmd1d01m3.//5.4e-07:128:70//D17131
R-OVARC1000302//Homo sapiens chromosome 17, clone hRPK.651_L_9, complete sequence.//1.7e-10:100:88//AC005971
R-OVARC1000304//Mouse mRNA from Mov10 locus.//7.9e-66:379:81//X52574
R-OVARC 1000309
R-OVARC1000321//Homo sapiens clone NH0479C13, WORKING DRAFT SEQUENCE, 12 unordered pieces.//6.5e-83:453:94//AC005236
R-OVARC1000326//Rattus norvegicus lamina-associated polypeptide 1C (LAP1C) mRNA, complete cds.//5.0e-58:455:81//U19614
R-OVARC1000335//*** SEQUENCING IN PROGRESS *** Homo sapiens chromosome 4, BAC clone C0483I23; HTGS phase 1, WORKING DRAFT SEQUENCE, 7 unordered pieces.//0.034:429:60//AC005690
R-OVARC1000347//Mus musculus HRS gene, complete cds.//4.6e-06:339:61//AF020308
R-OVARC1000384//D.discoideum glycoprotein 24 A and B (GP24A and GP24B) genes, complete cds.//0.48:296:62//M27588
R-OVARC1000408//Homo sapiens DNA from chromosome 19-cosmid R27740 containing MEF2B and RSRFR2 genes, genomic sequence.//9.4e-39:286:87//AD000812
R-OVARC1000411//CIT-HSP-2303H10.TF CIT-HSP Homo sapiens genomic clone 2303H10, genomic survey sequence.//1.5e-07:94:84//AQ016720
R-OVARC1000414//Homo sapiens genomic DNA, 21q region, clone: 149C3X10, genomic survey sequence.//1.8e-32:296:75//AG002388
R-OVARC1000420//Homo sapiens clone DJ1137M13, complete sequence.//2.0e-48:354:77//AC005378
R-OVARC1000427//D.discoideum vegetative specific gene V18 gene for ribosomal protein.//2.5e-09:370:59//X15382
R-OVARC1000431//HS_2199_A2_E02_T7 CIT Approved Human Genomic Sperm Library D Homo sapiens genomic clone Plate=2199 Col=4 Row=I, genomic survey sequence.//1.3e-34:186:98//AQ093722
R-OVARC1000437//Gallus gallus tensin mRNA, 3' end.//1.3e-15:160:80//L06662
R-OVARC1000440//Homo sapiens BAC clone NH0538D15 from 7q11.23-q21.1, complete sequence.//0.0054:337:61//AC006043
R-OVARC1000442//CIT-HSP-2335L20.TR CIT-HSP Homo sapiens genomic clone 2335L20, genomic survey sequence.//1.0e-45:322:86//AQ037381
R-OVARC1000443//Homo sapiens mRNA for KIAA0683 protein, complete cds.//1.1e-77:418:94//AB014583
R-OVARC1000461//Human DNA sequence *** SEQUENCING IN PROGRESS *** from clone 215D11, WORKING DRAFT SEQUENCE.//0.62:333:59//AL034417
R-OVARC1000465//Bos taurus guanine nucleotide-exchange protein (ARF-GEP1) mRNA, complete cds.//1.1e-81:489:91//AF023451
R-OVARC1000466//Homo sapiens chromosome 17, Neurofibromatosis 1 locus, complete sequence.//0.0088:98:72//AC004526
R-OVARC1000473//Homo sapiens full-length insert cDNA clone YI53C10.//3.2e-92:317:100//AF085851
R-OVARC1000479//Rattus norvegicus mRNA for TIP120, complete cds.//2.7e-70:502:84//D87671
R-OVARC1000486//Dictyostelium discoideum FusC (fusC) gene, partial cds.//0.52:411:58//AF019984
R-OVARC1000496
R-OVARC1000520//Homo sapiens PAC clone DJ412A9 from 22, complete sequence.//3.8e-17:294:71//AC005005
R-OVARC1000526//Homo sapiens clone GS438P06, WORKING DRAFT SEQUENCE, 17 unordered pieces.//4.5e-109:547:96//AC005024
R-OVARC1000533//Homo sapiens chromosome 19, cosmid R30385, complete sequence.//3.0e-46:264:93//AC004510
R-OVARC1000543//Caenorhabditis elegans cosmid F10C1.//0.00063:417:59//U49831
R-OVARC1000556//Homo sapiens DNA sequence from PAC 168L15 on chromosome 6q26-27. Contains RSK3 gene, ribosomal protein S6 kinase, EST, GSS, STS. CpG island, complete sequence.//1.5e-39:144:92//AL022069
R-OVARC1000557//Homo sapiens chromosome 19, cosmid R32469, complete sequence.//1.5e-81:429:96//AC005197
R-OVARC1000564//Homo sapiens chromosome 17, clone HRPC837J1, complete sequence.//0.83:301:58//AC004223
R-OVARC1000573//Homo sapiens Xq28 genomic DNA in the region of the ALD locus containing the genes for creatine transporter (SLC6A8), CDM, adrenoleukodystrophy (ALD), Na⁺-isocitrate dehydrogenase gamma subunit (IDH), and translocon-associated protein delta (TRAP) genes, complete cds, plexin related protein (PLEXR) and serine kinase (SK) genes, partial cds, Xq28lu1 gene and cytochrome C (CCp) pseudogene.//2.4e-44:300:88//U52111
R-OVARC1000578//Human Chromosome 16 BAC clone CIT987SK-A-270G1, complete sequence.//6.4e-48:436:78//AF001549
R-OVARC1000588//Homo sapiens chromosome 19, cosmid F19847, complete sequence.//2.7e-32:313:78//AC005952
R-OVARC 1000605
R-OVARC1000622//Homo sapiens PAC clone DJ0942I16 from 7q11, complete sequence.//6.2e-43:328:83//AC006012
R-OVARC1000640//High throughput sequencing of human chromosome 12, WORKING DRAFT SEQUENCE, 1 ordered pieces.//1.9e-47:514:73//AC005840
R-OVARC1000661//Homo sapiens mRNA for KIAA0590 protein, complete cds.//1.6e-29:162:100//AB011162.
R-OVARC1000678//Plasmodium falciparum 3D7 chromosome 12 PFYACB8-420 genomic sequence, WORKING DRAFT SEQUENCE, 14 unordered pieces.//0.50:270:60//AC005140
R-nnnnnnnnnnnn//Rattus norvegicus mRNA for myosin-RhoGAP protein Myr 7.//1.4e-83:549:86//AJ001713
R-OVARC1000681//Human DNA sequence *** SEQUENCING IN PROGRESS *** from clone 257E24, WORKING DRAFT SEQUENCE.//3.2e-13:160:76//AL034424
R-OVARC1000689//Schistocerca americana Antennapedia homeotic protein (Antp) mRNA, complete cds.//0.90:230:61//U32943
R-OVARC1000700//Homo sapiens chromosome 5, BAC clone 34j15 (LBNL H169), complete sequence.//5.1e-15:133:85//AC005754
R-OVARC1000703//Homo sapiens chromosome 22, clone hRPC.130_H_16, complete sequence.//6.9e-48:525:73//AC005585
R-OVARC1000730//HS_3018_B1_H10_T7 CIT Approved Human Genomic Sperm Library D Homo sapiens genomic clone Plate=3018 Col=19 Row=P, genomic survey sequence.//0.00019:198:63//AQ093513
R-OVARC1000746//P.falciparum complete gene map of plastid-like DNA (IR-B).//0.98:154:65//X95276
R-OVARC1000769//Human coagulation factor XI gene, intron 2, partial, clone pTZ18R.//2.0e-30:187:78//M21185
R-OVARC1000771
R-OVARC1000781//Sequence 5 from Patent WO9722695.//8.4e-47:401:77//A63552
R-OVARC1000787//Homo sapiens PAC clone DJ430N08 from 22q12.1-qter, complete sequence.//7.8e-111:567:96//AC004542
R-OVARC1000800//Homo sapiens mitochondrial HSP75 mRNA, complete cds.//1.3e-17:119:95//L15189
R-OVARC1000802//Homo sapiens chromosome 5, BAC clone 120c13 (LBNL H171), complete sequence.//2.3e-51:482:78//AC005574
R-OVARC1000834//Homo sapiens mRNA for atopy related autoantigen CALC.//3.6e-105:536:95//Y1771
R-OVARC1000846//Homo sapiens chromosome 16, cosmid clone 390H2 (LANL), complete sequence.//2.7e-107:538:96//AC004494
R-OVARC1000850//Homo sapiens PB39 mRNA, complete cds.//3.6e-114:579:96//AF045584
R-OVARC1000862//M.musculus Fif mRNA.//2.3e-20:346:73//X71978
R-OVARC1000876//Plasmodium falciparum chromosome 2, section 53 of 73 of the complete sequence.//9.1e-08:427:58//AE001416
R-OVARC1000883//Mus domesticus nuclear binding factor NF2d9 mRNA, complete cds.//5.6e-34:357:78//U20086
R-OVARC1000885//Lycopersicon esculentum alcohol dehydrogenase homolog (GAD3) mRNA, partial cds.//0.47:305:60//U21801
R-OVARC 1000886
R-OVARC1000891//HS_3082_A2_F04_MR CIT Approved Human Genomic Sperm Library D Homo sapiens genomic clone Plate=3082 Col=8 Row=K, genomic survey sequence.//1.1e-16:187:79//AQ122500
R-OVARC1000897//Human DNA sequence from clone 192P9 on chromosome Xp11.23-11.4. Contains a pseudogene similar to rat Plasmolipin, ESTs and GSSs, complete sequence.//7.2e-07:476:60//AL020989
R-OVARC1000912
R-OVARC1000915//Homo sapiens chromosome 17, clone hRPC.971_F_3, WORKING DRAFT SEQUENCE, 1 ordered pieces.//5.4e-70:509:86//AC004150
R-OVARC1000924//Homo sapiens Chromosome 22q11.2 Cosmid Clone cosk In NF1 Region, complete sequence.//1.6e-77:465:90//AC002471
R-OVARC1000936//HS_2195_A2_C12_MR CIT Approved Human Genomic Sperm Library D Homo sapiens genomic clone Plate=2195 Col=24 Row=E, genomic survey sequence.//2.4e-76:463:90//AQ191108
R-OVARC1000937//Human DNA sequence *** SEQUENCING IN PROGRESS *** from clone 250D10, WORKING DRAFT SEQUENCE.//0.0028:161:65//Z99716
R-OVARC1000945//Rattus norvegicus mRNA for atypical PKC specific binding protein, complete cds.//3.5e-62:526:78//AB005549
R-OVARC1000948//Hypera postica NADH dehydrogenase subunit 1 (ND1) gene, partial cds, tRNA-Leu gene, complete sequence, and 16S ribosomal gene, partial sequence, mitochondrial genes encoding mitochondrial products.//0.018:212:61//U61169
R-OVARC1000959//CIT-HSP-2371K16.TR CIT-HSP Homo sapiens genomic clone 2371K16, genomic survey sequence.//1.1e-45:303:87//AQ111323
R-OVARC1000960//Homo sapiens BAC clone GS293C05 from 7q21-q22, complete sequence.//7.5e-44:353:81//AC005021
R-OVARC1000971//H.sapiens DNA for repeat unit locus D18S51(285 bp).//2.2e-07:223:70//X91255
R-OVARC1000984
R-OVARC1000996//Human DNA sequence from clone 272L16 on chromosome 1q32.1-32.3. Contains the 3' end of the LAMB3 gene for Laminin, Beta 3 (Nicein, Kalinin, BM600) and a novel Rat Ca2⁺/Calmodulin dependent Protein Kinase LIKE gene. Contains ESTs, STSs, GSSs, genomic marker D1S491 and a ca repeat polymorphism, complete sequence.//1.3e-06:179:70//AL023754
R-OVARC1000999//Homo sapiens chromosome 17, clone hCIT.457_L_16, complete sequence.//5.8e-71:332:87//AC003957
R-OVARC1001000//HS_3032_B1_G11_MF CIT Approved Human Genomic Sperm Library D Homo sapiens genomic clone Plate=3032 Col=21 Row=N, genomic survey sequence.//5.1e-51:257:99//AQ096695
R-OVARC1001004//Homo sapiens from UWGC:y18c282 from 6p21, complete sequence.//5.6e-92:473:96//AC004190
R-OVARC1001010//RPCI11-10P1.TV RPCI-11 Homo sapiens genomic clone RPCI-11-10P1, genomic survey sequence.//4.1e-05:201:65//B71813
R-OVARC1001011//Homo sapiens clone DJ1021I20, WORKING DRAFT SEQUENCE, 6 unordered pieces.//7.9e-18:219:69//AC005520
R-OVARC1001032//Human DNA sequence *** SEQUENCING IN PROGRESS *** from clone Y738F9, WORKING DRAFT SEQUENCE.//2.7e-89:464:86//AL022345
R-OVARC1001034//Homo sapiens chromosome 20, BAC clone 99 (LBNL H80), complete sequence.//1.4e-18:451:64//AC005220
R-OVARC1001038//Homo sapiens TRIAD1 type I mRNA, complete cds.//1.3e-99:501:96//AF099149
R-OVARC1001040//Homo sapiens chromosome 17, clone hRPK.1096_G_20, complete sequence.//9.7e-17:180:78//AC005410
R-OVARC1001044
R-OVARC1001051//H.sapiens mRNA for homologue to yeast ribosomal protein L41.//3.7e-15:124:88//Z12962
R-OVARC1001055//Homo sapiens, clone hRPK.15_A_1, complete sequence.//2.0e-30:292:76//AC006213
R-OVARC1001062//Sequence 65 from patent US 5691147.//2.6e-54:312:92//I76237
R-OVARC1001068//Homo sapiens Era GTPase A protein (HERA-A) mRNA, partial cds.//2.3e-95:463:98//AF082657
R-OVARC1001072//Gallus gallus chicken brain factor-2 (CBF-2) mRNA, complete cds.//0.92:272:59//U47276
R-OVARC1001074//HS_2205_A1_D07_MF CIT Approved Human Genomic Sperm Library D Homo sapiens genomic clone Plate=2205 Col=13 Row=G, genomic survey sequence.//1.3e-35:205:94//AQ184530
R-OVARC1001085
R-OVARC1001092//Homo sapiens mRNA for JM5 protein, complete CDS (clone IMAGE 53337, LLNLc110F1857Q7 (RZPD Berlin) and LLNLc110G0913Q7 (RZPD Berlin)).//4.5e-95:325:98//AJ005897
R-OVARC1001113//Homo sapiens diaphanous 1 (HDIA1) mRNA, complete cds.//1.0e-73:386:95//AF051782
R-OVARC1001117//Homo sapiens chromosome 7 clone UWGC:g3586a160 from 7p14-15, complete sequence.//6.1e-37:314:81//AC005272
R-OVARC1001118//Homo sapiens chromosome 5, P1 clone 1195e2 (LBNL H73), complete sequence.//1.5e-44:390:77//AC005372
R-OVARC1001129//Rickettsia prowazekii strain Madrid E, complete genome; segment 1/4.//0.81:461:57//AJ235270
R-OVARC1001161//Human DNA sequence *** SEQUENCING IN PROGRESS *** from clone 850H21, WORKING DRAFT SEQUENCE.//4.6e-08:342:64//AL031680
R-OVARC1001162//CIT-HSP-2171J2.TR CIT-HSP Homo sapiens genomic clone 2171J2, genomic survey sequence.//5.9e-48:347:85//B89781
R-OVARC1001167//Homo sapiens clone DJ1102A12, WORKING DRAFT SEQUENCE, 15 unordered pieces.//1.3e-28:427:70//AC004963
R-OVARC1001169//RPCI11-36P6.TV RPCI-11 Homo sapiens genomic clone RPCI-11-36P6, genomic survey sequence.//0.56:113:72//AQ045859
R-OVARC1001170//Homo sapiens Xp22 BAC GS-377014 (Genome Systems Human BAC library) complete sequence.//8.8e-39:301:85//AC002549
R-OVARC1001173//Human clone HS2.30 Alu-Ya5 sequence.//2.4e-35:183:83//U67213
R-OVARC1001180//Homo sapiens 12q24.1 NOVECTOR P443K8 () complete sequence.//9.1e-41:516:72//AC005907
R-OVARC1001188//Homo sapiens Chromosome 11p14.3 PAC clone pDJ1034g4, complete sequence.//1.2e-14:134:85//AC004796
R-OVARC1001200//ALS=85 kda insulin-like growth factor binding protein-3 complex acid-labile subunit [baboons, liver, mRNA Partial, 1818 nt].//0.12:345:60//S83462
R-OVARC1001232//Bovine tyrosine hydroxylase mRNA, complete cds.//0.66:257:59//M36794
R-OVARC1001240//Homo sapiens chromosome 17, clone hCIT.124_H_2, complete sequence.//1.4e-41:284:87//AC006071
R-OVARC1001243//HS_2055_B2_C01_MR CIT Approved Human Genomic Sperm Library D Homo sapiens genomic clone Plate=2055 Col=2 Row=F, genomic survey sequence.//0.59:83:75//AQ243142
R-OVARC1001261//Crocodylus porosus mRNA for transthyretin.//0.93:121:66//AJ223148
R-OVARC1001268
R-OVARC1001270//Plasmodium falciparum MAL3P6, complete sequence.//0.0031:295:62//Z98551
R-OVARC1001271//Homo sapiens chromosome 16, cosmid clone 390H2 (LANL), complete sequence.//1.6e-107:544:97//AC004494
R-OVARC1001282//Homo sapiens Xp22-39-47 PAC RPCI1-199J3 (Roswell Park Cancer Institute Human PAC Library) complete sequence.//0.025:402:59//AC006062
R-OVARC1001296//Homo sapiens echinoderm microtubule-associated protein homolog HuEMAP mRNA, complete cds.//1.1e-05:319:62//U97018
R-nnnnnnnnnnnn//Sequence 13 from patent US 5624818.//5.4e-85:577:84//I41142
R-OVARC1001329//Human DNA sequence *** SEQUENCING IN PROGRESS *** from clone 30G7, WORKING DRAFT SEQUENCE.//4.2e-71:282:88//AL034402
R-OVARC1001330//Homo sapiens PAC clone DJ0697H17 from 7q11.23-q21.1, complete sequence.//0.19:256:59//AC004862
R-OVARC1001339//Homo sapiens 12q13 PAC RPCI1-316M24 (Roswell Park Cancer Institute Human PAC library) complete sequence.//2.5e-49:366:83//AC004242
R-OVARC1001341//Human DNA sequence *** SEQUENCING IN PROGRESS *** from clone 695O20, WORKING DRAFT SEQUENCE.//4.8e-26:447:69//AL032818
R-OVARC1001342//Homo sapiens chromosome 10 clone CIT987SK-1175G20 map 10q25.2-10q25.3, complete sequence.//5.5e-86:569:86//AC005874
R-OVARC1001344//Homo sapiens chromosome 5, BAC clone 261j17 (LBNL H190), complete sequence.//2.8e-46:424:78//AC005350
R-OVARC1001357//Sequence 1 from patent US 5597707.//3.0e-42:250:93//I34297
R-OVARC1001360//Homo sapiens chromosome 17, clone hRPK.786_O_4, complete sequence.//0.20:335:60//AC005863
R-OVARC 1001369
R-OVARC1001372//S.scrofa DNA for myogenin 3'flanking region (285 bp).//6.9e-29:249:83//X89210
R-OVARC1001376//Homo sapiens BAC clone RG139P11 from 7q11-q21, complete sequence.//2.1e-50:491:73//AC004491
R-OVARC1001381//Homo sapiens chromosome 17, clone hRPK.156_L_14, complete sequence.//9.3e-20:422:60//AC005821
R-OVARC1001391
R-nnnnnnnnnnnn
R-OVARC1001417//Homo sapiens EXLM1 mRNA, complete cds.//9.9e-110:561:95//AB00665
R-OVARC1001419//CIT-HSP-2362F16.TR CIT-HSP Homo sapiens genomic clone 2362F16, genomic survey sequence.//7.6e-47:242:98//AQ074668
R-OVARC1001425//Homo sapiens PAC clone DJ1108A12 from 14q24.3, complete sequence.//2.3e-20:211:66//AC005157
R-OVARC1001436//Human DNA flanking 3' end of transposon L1.1.//0.18:148:66//M80341
R-OVARC1001442
R-OVARC1001453//Human PAC clone DJ525N14 from Xq23, complete sequence.//2.3e-19:181:81//AC002086
R-OVARC1001476//CITBI-E1-2517B6.TR CITBI-E1 Homo sapiens genomic clone 2517B6, genomic survey sequence.//0.24:308:59//AQ278655
R-OVARC1001480//Human DNA sequence *** SEQUENCING IN PROGRESS *** from clone 753D4, WORKING DRAFT SEQUENCE.//0.99:294:62//AL031676
R-OVARC1001489//E.caballus microsatellite DNA marker (clone ASB32).//0.87:81:71//X93546
R-OVARC1001496//Homo sapiens C-terminal binding protein 2 mRNA, complete cds.//9.3e-116:585:96//AF016507
R-OVARC1001506//Homo sapiens Chromosome 16 BAC clone CIT987-SKA-13F4 ∼complete genomic sequence, complete sequence.//2.6e-40:285:86//AC002039
R-OVARC1001525//Homo sapiens clone NH0215P16, WORKING DRAFT SEQUENCE, 3 unordered pieces.//1.0:320:59//AC006036
R-OVARC1001542//Homo sapiens hJTB mRNA, complete cds.//5.0e-110:566:95//AB016488
R-OVARC1001547
R-OVARC1001577//Homo sapiens SRp46 splicing factor transcribed retropseudogene.//5.9e-33:216:92//AF031165
R-OVARC1001600//Human Chromosome X, complete sequence.//3.0e-22:157:89//AC002418
R-OVARC1001610//HS_3070_A2_A06_MR CIT Approved Human Genomic Sperm Library D Homo sapiens genomic clone Plate=3070 Col=12 Row=A, genomic survey sequence.//0.47:107:66//AQ103523
R-OVARC1001611//Human DNA sequence *** SEQUENCING IN PROGRESS *** from clone 1185N5, WORKING DRAFT SEQENCE.//0.17:236:63//AL034423
R-OVARC1001615//Human DNA sequence *** SEQUENCING IN PROGRESS *** from clone 310O13, WORKING DRAFT SEQUENCE.//1.3e-19:248:70//AL031658
R-OVARC1001668//HS_3228_A2_E12_MR CIT Approved Human Genomic Sperm Library D Homo sapiens genomic clone Plate=3228 Col=24 Row=I, genomic survey sequence.//4.6e-13:156:76//AQ188379
R-OVARC1001702//CITBI-E1-2501P16.TR.1 CITBI-E1 Homo sapiens genomic clone 2501P16, genomic survey sequence.//1.6e-41:217:99//AQ241965
R-OVARC1001703
R-OVARC1001711//CITBI-E1-2502N10.TF CITBI-E1 Homo sapiens genomic clone 2502N10, genomic survey sequence.//2.0e-14:220:72//AQ266194
R-OVARC1001726//CIT-HSP-2320O1.TF CIT-HSP Homo sapiens genomic clone 2320O1, genomic survey sequence.//0.021:170:62//AQ038145
R-OVARC1001731//Human mRNA for fibroblast tropomyosin TM30 (pl).//2.5e-72:422:90//X05276
R-OVARC1001745//Human DNA sequence from clone 796I11 on chromosome 20q12. Contains ESTs, an STS and GSSs, complete sequence.//7.6e-44:314:84//AL031257
R-nnnnnnnnnnnn//S.cerevisiae N-acetyltransferase (AAA1) mRNA, complete cds.//1.6e-08:396:60//M23166
R-OVARC1001766//Homo sapiens eukaryotic translation initiation factor eIF3, p35 subunit mRNA, complete cds.//3.5e-108:567:94//U97670
R-nnnnnnnnnnnn//Homo sapiens mRNA for KIAA0675 protein, complete cds.//6.3e-108:529:97//AB014575
R-OVARC1001768//Caenorhabditis elegans cosmid Y57G11A, complete sequence.//0.24:205:64//Z99279
R-OVARC1001791//Homo sapiens BAC clone RG118P15 from 8q21, complete sequence.//4.6e-58:558:76//AC005066
R-OVARC1001795
R-OVARC1001802//Human HLA class III region containing cAMP response element binding protein-related protein (CREB-RP) and tenascin X (tenascin-X) genes, complete cds, complete sequence.//1.1e-37:346:78//U89337
R-OVARC1001805//Human DNA sequence from clone 511E16 on chromosome 6p24.3-25.1. Contains the last coding exon of the gene for P18 component of aminoacyl-tRNA synthetase complex, part of an unknown gene downstream of a putative CpG island, and an STS with a CA repeat polymorphism, complete sequence.//3.0e-112:581:95//AL023694
R-OVARC1001812//Human DNA sequence from clone 227L5 on chromosome Xp11.22-11.3. Contains a Keratin, Type 1 Cytoskeletal 18 (KRT18, CYK18, K18, CK18) pseudogene and an STS, complete sequence.//6.6e-41:345:81//AL031585
R-OVARC1001813//CITBI-E1-2508J18.TR CITBI-E1 Homo sapiens genomic clone 2508J18, genomic survey sequence.//1.6e-72:386:95//AQ263046
R-OVARC1001820//Human PAC clone DJ525N14 from Xq23, complete sequence.//4.8e-41:320:83//AC002086
R-OVARC1001828//Plasmodium falciparum 3D7 chromosome 12 PFYAC69 genomic sequence, WORKING DRAFT SEQUENCE, 4 unordered pieces.//3.4e-08:527:58//AC004688
R-OVARC1001846//CIT-HSP-2014F15.TR CIT-HSP Homo sapiens genomic clone 2014F15, genomic survey sequence.//0.0045:165:67//B58905
R-OVARC1001861//M.musculus mRNA for pMEM2 protein.//9.5e-28:405:68//X95350
R-OVARC1001873//Homo sapiens clones 24718 and 24825 mRNA sequence.//5.9e-104:571:91//AF070611
R-OVARC1001879//Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.//9.1e-20:206:80//AL031864
R-OVARC1001880//RPCI11-42I15.TJ RPCI11 Homo sapiens genomic clone R-42I15, genomic survey sequence.//3.9e-50:287:88//AQ052700
R-OVARC1001883//Homo sapiens chromosome 17, clone hCIT.123_J_14, complete sequence.//6.1e-13:457:63//AC003950
R-OVARC1001900//Homo sapiens tumorous imaginal discs protein Tid56 homolog (TID1) mRNA, complete cds.//2.5e-86:346:90//AF061749
R-OVARC1001901//Homo sapiens testis specific methyl-CpG binding protein MBD2 (MBD2) mRNA, partial cds.//7.2e-89:421:100//AF072246
R-OVARC1001911//Homo sapiens full-length insert cDNA clone ZD52F10.//8.2e-106:510:98//AF086315
R-OVARC1001916
R-OVARC1001928
R-OVARC1001942//S.cerevisiae N-acetyltransferase (AAA1) mRNA, complete cds.//0.0013:231:63//M23166
R-OVARC1001943//Human immunodeficiency virus type 1, strain FRMP329, envelope glycoprotein V3 region (env) gene, partial cds.//0.14:173:64//U58826
R-OVARC1001949//Human zinc finger protein 20 (ZNF20) pentanucleotide repeat polymorphism.//1.3e-09:306:63//M99593
R-OVARC1001950//Homo sapiens chromosome 17, clone hRPK.112_H_10, complete sequence.//8.2e-38:385:75//AC005666
R-OVARC1001987
R-OVARC1001989//Caenorhabditis elegans DNA *** SEQUENCING IN PROGRESS *** from clone Y57G11, WORKING DRAFT SEQUENCE.//6.3e-08:355:60//Z92841
R-OVARC1002044//Human DNA sequence from clone 681J21 on chromosome 1q23.2-24.3 Contains CpG island, complete sequence.//5.0e-42:298:86//AL031286
R-OVARC1002050//Homo sapiens mRNA for KIAA0465 protein, partial cds.//1.4e-107:542:96//AB007934
R-OVARC1002066//Arabidopsis thaliana chromosome II BAC F14M4 genomic sequence, complete sequence.//0.23:210:61//AC004411
R-OVARC1002082//Homo sapiens clone DJ0965K10, WORKING DRAFT SEQUENCE, 6 unordered pieces.//5.4e-99:546:92//AC006015
R-OVARC1002107//Human DNA sequence from PAC 417G15 on chromosome Xq25-Xq26. Contains glypican-3 precursor (intestinal protein OCI-5) (GTR2-2), pseudogene, ESTs.//4.4e-34:375:74//AL009174
R-OVARC1002127
R-OVARC1002138//CIT-HSP-2290O18.TF CIT-HSP Homo sapiens genomic clone 2290O18, genomic survey sequence.//2.4e-07:316:62//AQ003988
R-OVARC1002143//RPCI11-54M8.TJ RPCI11 Homo sapiens genomic clone R-54M8, genomic survey sequence.//2.3e-35:220:90//AQ083241
R-OVARC1002156
R-OVARC1002158//CITBI-E1-2514D4.TF CITBI-E1 Homo sapiens genomic clone 2514D4, genomic survey sequence.//1.6e-12:140:79//AQ265720
R-OVARC1002165//CIT-HSP-2307C9.TF CIT-HSP Homo sapiens genomic clone 2307C9, genomic survey sequence.//5.0e-59:291:99//AQ020420
R-OVARC1002182//P. falciparum SD17 gene for knob-associated histidine-rich protein.//0.74:161:65//Y00060
R-PLACE1000004//D.discoideum gene for protein kinase.//0.00081:263:59//Z37981
R-PLACE1000005//Plasmodium falciparum 3D7 chromosome 12 PFYACB8-628 genomic sequence, WORKING DRAFT SEQUENCE, 9 unordered pieces.//0.0082:477:58//AC005507
R-PLACE1000007//Homo sapiens clone 24422 mRNA sequence.//1.2e-14:100:97//AF070557
R-PLACE1000014//Homo sapiens genomic DNA, chromosome 21q22.2, p1 clone: T1212 and T1601, WORKING DRAFT SEQUENCE.//2.8e-44:405:77//D83253
R-PLACE1000031//Homo sapiens clone UWGC:y23c049 from 6p21, complete sequence.//1.8e-24:291:73//AC006162
R-PLACE1000040//Caenorhabditis elegans DNA *** SEQUENCING IN PROGRESS *** from clone Y105C5, WORKING DRAFT SEQUENCE.//0.00039:289:61//Z98855
R-PLACE1000048//Human BAC clone RG210I04, complete sequence.//4.7e-83:518:89//AC002462
R-PLACE1000050//Plasmodium falciparum 3D7 chromosome 12 PFYAC181 genomic sequence, WORKING DRAFT SEQUENCE, 8 unordered pieces.//0.98:73:76//AC005505
R-PLACE1000061//Human ribosomal protein L37a mRNA sequence.//5.9e-21:125:98//L22154
R-PLACE1000066
R-PLACE1000078//Homo sapiens chromosome 11 clone CIT987SK-1012F4, WORKING DRAFT SEQUENCE, 6 unordered pieces.//1.2e-87:456:95//AC005848
R-PLACE1000081
R-PLACE1000094//RPCI11-91K6.TV RPCI11 Homo sapiens genomic clone R-91K6, genomic survey sequence.//2.3e-83:409:98//AQ282619
R-PLACE1000133//Homo sapiens chromosome 17, clone hRPK.746_E_8, complete sequence.//1.8e-06:420:57//AC005358
R-PLACE1000142
R-PLACE1000184//Homo sapiens estrogen-related receptor gamma mRNA, complete cds.//1.3e-112:594:94//AF058291
R-PLACE1000185
R-PLACE1000213//CIT-HSP-2308A18.TR CIT-HSP Homo sapiens genomic clone 2308A18, genomic survey sequence.//8.2e-80:410:97//AQ022149
R-PLACE1000214//Plasmodium falciparum DNA *** SEQUENCING IN PROGRESS *** from contig 3-09, complete sequence.//1.6e-05:548:59//AL008989
R-PLACE1000236//Human DNA sequence *** SEQUENCING IN PROGRESS *** from clone 695O20, WORKING DRAFT SEQUENCE.//2.2e-16:118:91//AL032818
R-PLACE1000246//X.laevis mRNA for XLCL2 protein.//6.5e-13:66:95//Z14122
R-PLACE1000292//Human DNA sequence *** SEQUENCING IN PROGRESS *** from clone 111B22, WORKING DRAFT SEQUENCE.//6.6e-41:322:84//Z98200
R-PLACE1000332//Homo sapiens chromosome 17, clone hCIT.281_F_24, complete sequence.//1.8e-16:598:62//AC004706
R-PLACE1000347//Homo sapiens PAC clone DJ1090P18 from 7q21-q22, complete sequence.//2.3e-11:237:69//AC005326
R-PLACE1000374//Arabidopsis thaliana chromosome 1 BAC F15K9 sequence, complete sequence.//8.7e-09:492:58//AC005278
R-PLACE1000380//Plasmodium falciparum chromosome 2, section 1 of 73 of the complete sequence.//0.59:354:59//AE001364
R-PLACE1000383//Mus musculus myotubularin related protein 1 (Mtmr1) mRNA, complete cds.//0.55:65:84//AF073997
R-PLACE1000401//Homo sapiens clone GS166C05, WORKING DRAFT SEQUENCE, 7 unordered pieces.//3.6e-17:152:83//AC005015
R-PLACE1000406//Arabidopsis thaliana genomic DNA, chromosome 5, TAC clone: K21H1, complete sequence.//0.51:346:58//AB020742
R-PLACE1000420//Homo sapiens genomic DNA, chromosome 21q22.2 (Down Syndrome region), segment 2/15, WORKING DRAFT SEQUENCE.//1.5e-25:243:79//AP000009
R-PLACE1000421//HS_2251_B2_G12_MR CIT Approved Human Genomic Sperm Library D Homo sapiens genomic clone Plate=2251 Col=24 Row=N, genomic survey sequence.//1.4e-82:430:95//AQ192807
R-PLACE1000424//Human PAC clone DJ515N1 from 22q11.2-q22, complete sequence.//1.8e-36:483:71//AC002073
R-PLACE1000435//Homo sapiens chromosome 21q22.2 cosmid clone Q71A3, complete sequence.//2.6e-37:371:76//AF015724
R-PLACE1000444//Homo sapiens chromosome 17, clone hRPK.227_G_15, complete sequence.//1.0e-54:429:81//AC005899
R-PLACE1000453//Murine genomic DNA; partially digested Sau3A fragment, cloned into cosmid vector pEMBLcos2, complete sequence.//0.66:103:72//AF059580
R-PLACE1000481//Human DNA sequence from clone 960O17 on chromosome Xp11.21-11.22 Contains EST, CA repeat(DXS991), STS, GSS, complete sequence.//0.019:171:66//AL022166
R-PLACE1000492//Rat vacuolar protein sorting homolog r-vps33b mRNA, complete cds.//3.2e-17:221:72//U35245
R-PLACE1000540//Plasmodium falciparum 3D7 chromosome 12 PFYAC492 genomic sequence, WORKING DRAFT SEQUENCE, 5 unordered pieces.//0.00045:480:60//AC005308
R-PLACE1000547//Homo sapiens chromosome 19, cosmid F17987, complete sequence.//9.6e-32:231:85//AC004790
R-PLACE1000562//, complete sequence.//1.8e-45:280:92//AC005409
R-PLACE1000564//Human chromosome 16 creatine transporter (SLC6A8) and (CDM) paralogous genes, complete cds.//0.0079:180:65//U41302
R-PLACE1000583//Homo sapiens chromosome 17, clone hRPK.799_N_11, complete sequence.//1.5e-37:414:74//AC005323
R-nnnnnnnnnnnn//Human guanylate binding protein isoform I (GBP-2) mRNA, complete cds.//1.9e-77:542:82//M55542
R-PLACE1000596//Plasmodium falciparum 3D7 chromosome 12 PFYAC357 genomic sequence, WORKING DRAFT SEQUENCE, 7 unordered pieces.//0.00019:482:59//AC005506
R-PLACE1000599//Human germline T-cell receptor beta chain Dopamine-beta-hydroxylase-like, TRY1, TRY2, TRY3, TCRBV27S1P, TCRBV22S1A2N1T, TCRBV9S1A1T, TCRBV7S1A1N2T, TCRBV5S1A1T, TCRBV13S3, TCRBV6S7P, TCRBV7S3A2T, TCRBV13S2A1T, TCRBV9S2A2PT, TCRBV7S2A1N4T, TCRBV13S9/13S2A1T, TCRBV6S5A1N1, TCRBV30S1P, TCRBV31S1, TCRBV13S5, TCRBV6S1A1N1, TCRBV32S1P, TCRBV5S5P, TCRBV1S1A1N1, TCRBV12S2A1T, TCRBV21S1, TCRBV8S4P, TCRBV12S3, TCRBV21S3A2N2T, TCRBV8S5P, TCRBV13S1 genes from bases 1 to 267156 (section 1 of 3).//5.6e-51:369:85//U66059
R-PLACE1000610//HS_3071_A1_C05_MF CIT Approved Human Genomic Sperm Library D Homo sapiens genomic clone Plate=3071 Col=9 Row=E, genomic survey sequence.//0.051:147:65//AQ103341
R-PLACE1000636//HS_3220_B2_E09_T7 CIT Approved Human Genomic Sperm Library D Homo sapiens genomic clone Plate=3220 Col=18 Row=J, genomic survey sequence.//0.010:253 :64//AQ181157
R-PLACE1000653//Homo sapiens N-acetylglucosamine-phosphate mutase mRNA, complete cds.//1.6e-99:506:96//AF102265
R-PLACE1000656//Homo sapiens mRNA for JM4 protein, complete CDS (clone IMAGE 546750 and LLNLc110F1857Q7 (RZPD Berlin)).//4.5e-101:559:92//AJ005896
R-PLACE1000706//nuclear protein TIF1 [mice, mRNA, 3951 nt].//9.1e-10:331:63//S78219
R-PLACE1000712//Homo sapiens full-length insert cDNA clone ZD76G10.//1.0e-69:345:98//AF086408
R-PLACE1000716//Homo sapiens Chromosome 16 BAC clone CIT987SK-A-279B10, complete sequence.//1.0:174:62//AC002300
R-PLACE1000748//Plasmodium falciparum MAL3P3, complete sequence.//1.0e-06:337:60//Z98547
R-PLACE1000749//cSRL-15g9-u cSRL flow sorted Chromosome 11 specific cosmid Homo sapiens genomic clone cSRL-15g9, genomic survey sequence.//8.8e-26:236:80//B02791
R-PLACE1000755//HS_2183_B1_H11_MF CIT Approved Human Genomic Sperm Library D Homo sapiens genomic clone Plate=2183 Col=21 Row=P, genomic survey sequence.//0.47:151:65//AQ064202
R-PLACE1000769//Homo sapiens clone DJ0647J21, WORKING DRAFT SEQUENCE, 10 unordered pieces.//7.0e-38:492:74//AC004847
R-PLACE1000785//Homo sapiens mRNA for KIAA0648 protein, partial cds.//2.6e-101:513:96//AB014548
R-PLACE1000786//Human putative outer mitochondrial membrane 34 kDa translocase hTOM34 mRNA, complete cds.//0.078:180:68//U58970
R-nnnnnnnnnnnn
R-PLACE1000798//Homo sapiens cosmid D66B10, chromosome 21 5' of IFNAR1.//5.1e-26:348:72//AF039904
R-PLACE1000841//Human guanine nucleotide regulatory protein (NET1) mRNA, complete cds.//1.4e-26:110:95//U02081
R-nnnnnnnnnnnn//Homo sapiens full-length insert cDNA clone ZD55D10.//1.4e-13:93:96//AF086334
R-PLACE1000856//Anopheles quadrimaculatus NADH dehydrogenase subunits (1-4, 4L, 5-6); cytochrome oxidase subunits (1-3); adenosine triphosphatase subunits (6,8); cytochrome b; transfer RNA; ribosomal RNA (large and small subunits).//2.7e-09:484:59//L04272

R-PLACE1000863
R-PLACE1000909//Plasmodium falciparum 3D7 chromosome 12 PFYAC181 genomic sequence, WORKING DRAFT SEQUENCE, 8 unordered pieces.//3.0e-05:274:60//AC005505
R-PLACE1000931//RPCI11-66P7.TK RPCI11 Homo sapiens genomic clone R-66P7, genomic survey sequence.//3.4e-73:369:97//AQ237489
R-PLACE1000948//RPCI11-64K15.TK RPCI11 Homo sapiens genomic clone R-64K15, genomic survey sequence.//6.6e-06:258:62//AQ239337
R-PLACE1000972//Homo sapiens chromosome 17, clone hRPK.112_J_9, complete sequence.//8.3e-20:223:76//AC005553
R-PLACE1000977//Plasmodium falciparum 3D7 chromosome 12 PFYAC357 genomic sequence, WORKING DRAFT SEQUENCE, 7 unordered pieces.//0.00030:448:59//AC005506
R-PLACE1000979
R-PLACE1001000//CIT-HSP-2297I8.TF CIT-HSP Homo sapiens genomic clone 2297I8, genomic survey sequence.//7.0e-07:64:95//AQ004997
R-PLACE1001007//Human endothelial nitric oxide synthase gene, complete cds.//0.0078:215:64//D26607
R-PLACE1001010
R-PLACE1001015//Human DNA sequence *** SEQUENCING IN PROGRESS *** from clone 150C2, WORKING DRAFT SEQUENCE.//1.5e-16:452:63//AL022318
R-PLACE1001024//Human DNA sequence *** SEQUENCING IN PROGRESS *** from clone 417M14, WORKING DRAFT SEQUENCE.//0.99:186:63//AL024498
R-PLACE1001036//Homo sapiens clone DJ1136G02, WORKING DRAFT SEQUENCE, 4 unordered pieces.//2.5e-15:313:68//AC005377
R-PLACE1001062//Homo sapiens chromosome 17, clone hCIT54K19, complete sequence.//7.3e-16:119:84//AC003664
R-PLACE1001076
R-PLACE1001088//Human DNA sequence from cosmid 203C2, between markers DXS6791 and DXS8038 on chromosome X contains ESTs.//0.97:332:59//Z74696
R-PLACE1001092//Plasmodium falciparum 3D7 chromosome 12 PFYAC336 genomic sequence, WORKING DRAFT SEQUENCE, 5 unordered pieces.//6.2e-07 :302:62//AC005139
R-PLACE1001104//Plasmodium falciparum chromosome 2, section 9 of 73 of the complete sequence.//0.057:280:60//AE001372
R-PLACE1001118//Homo sapiens DNA sequence from PAC 418A9 on chromosome 6q21. Contains the first (5') two exons of a CDK8 (Cell Division Protein Kinase 8) LIKE gene, a Neutral Calponin LIKE pseudogene, ESTs and STSs, complete sequence.//4.9e-06:334:60//Z84480
R-PLACE1001136//Homo sapiens chromosome 17, clone hRPK.22_N_12, WORKING DRAFT SEQUENCE, 2 ordered pieces.//1.1e-31:331:75//AC005412
R-PLACE1001168//HS_2036_A1_H04_MR CIT Approved Human Genomic Sperm Library D Homo sapiens genomic clone Plate=2036 Col=7 Row=O, genomic survey sequence.//0.40:144:63//AQ230662
R-PLACE1001171
R-PLACE1001185
R-PLACE1001238//Human coxVIb gene, last exon and flanking sequence.//3.4e-36:349:76//X58139
R-PLACE1001241//Plasmodium falciparum DNA *** SEQUENCING IN PROGRESS *** from contig 3-20, complete sequence.//0.11:258:61//AL008972
R-PLACE1001257//*** SEQUENCING IN PROGRESS *** Homo sapiens chromosome 4, BAC clone B4P3; HTGS phase 1, WORKING DRAFT SEQUENCE, 9 unordered pieces.//1.9e-46:484:73//AC000016
R-PLACE1001272//Homo sapiens chromosome 21q22.3 PAC 191P10, complete sequence.//0.89:119:65//AF045448
R-PLACE1001279//Caenorhabditis elegans cosmid Y39A1C, complete sequence.//0.99:95:69//AL023839
R-PLACE1001280//CIT-HSP-2328B24.TF CIT-HSP Homo sapiens genomic clone 2328B24, genomic survey sequence.//5.4e-24:147:76//AQ042129
R-PLACE1001294//M.musculus GEG-154 mRNA.//1.3e-22:472:65//X71642
R-PLACE1001304//Homo sapiens chromosome 19, overlapping cosmids F18547, F11133, R27945, R28830 and R32804, complete sequence.//2.2e-22:139:77//AC003682
R-PLACE1001311//Loligo pealei repeat region.//0.84:232:64//Z18286
R-PLACE1001323//Homo sapiens DNA sequence from PAC 418A9 on chromosome 6q21. Contains the first (5') two exons of a CDK8 (Cell Division Protein Kinase 8) LIKE gene, a Neutral Calponin LIKE pseudogene, ESTs and STSs, complete sequence.//7.2e-39:308:83//Z84480
R-PLACE1001351//Caenorhabditis elegans DNA *** SEQUENCING IN PROGRESS *** from clone Y39B6, WORKING DRAFT SEQUENCE.//0.0018:408:59//Z95399
R-PLACE1001366//Human Na+/phosphate co-transporter gene, exon 1, partial sequence.//2.2e-46:369:82//D89927
R-PLACE1001377//Homo sapiens ADAM10 (ADAM10) mRNA, complete cds.//7.1e-80:431:93//AF009615
R-PLACE1001383//Homo sapiens clone 24538 mRNA sequence.//3.6e-35:192:97//AF055030
R-PLACE1001384//Homo sapiens mRNA for multi PDZ domain protein.//2.6e-86:456:94//AJ001319
R-PLACE1001387
R-PLACE1001395//Nyctalus leisleri mitochondrial D-loop, partial sequence.//0.054:148:68//U95355
R-PLACE1001399//Homo sapiens chromosome 17, clone hRPK.22_N_12, WORKING DRAFT SEQUENCE, 2 ordered pieces.//6.7e-70:352:98//AC005412
R-PLACE1001412//Homo sapiens clone 643 unknown mRNA, complete sequence.//8.0e-44:242:95//AF091087
R-PLACE1001414//Homo sapiens chromosome 9, clone hRPK.202_H_3, complete sequence.//0.12:53:84//AC006241
R-PLACE1001440//Homo sapiens Xq28 genomic DNA in the region of the ALD locus containing the genes for creatine transporter (SLC6A8), CDM, adrenoleukodystrophy (ALD), Na⁺-isocitrate dehydrogenase gamma subunit (IDH), and translocon-associated protein delta (TRAP) genes, complete cds, plexin related protein (PLEXR) and serine kinase (SK) genes, partial cds, Xq281u1 gene and cytochrome C (CCp) pseudogene.//1.0:250:61//U52111
R-PLACE1001456//Borrelia burgdorferi (section 16 of 70) of the complete genome.//0.0077:173:62//AE001130
R-PLACE1001468//HS_3050_A2_D07_MR CIT Approved Human Genomic Sperm Library D Homo sapiens genomic clone Plate=3050 Col=14 Row=G, genomic survey sequence.//0.00023:202:65//AQ133920
R-PLACE1001484//Homo sapiens Xq28 BAC PAC and cosmid clones containing FMR2 gene exons 1,2, and 3, complete sequence.//7.2e-17:180:80//AC002368
R-PLACE1001502//RPCI11-24F2.TP RPCI-11 Homo sapiens genomic clone RPCI-11-24F2, genomic survey sequence.//0.15:203:66//B84401
R-PLACE1001503//HS_2183_A1_B10_MR CIT Approved Human Genomic Sperm Library D Homo sapiens genomic clone Plate=2183 Col=19 Row=C, genomic survey sequence.//1.3e-38:181:82//AQ022613
R-PLACE1001517//Homo sapiens hGAA1 mRNA, complete cds.//6.4e-56:339:90//AB006969
R-PLACE1001534//Human DNA sequence *** SEQUENCING IN PROGRESS *** from clone 620E11, WORKING DRAFT SEQUENCE.//8.6e-59:304:97//AL031667
R-PLACE1001545//Homo sapiens chromosome 3, clone hRPK.165_I_16, complete sequence.//2.6e-18:171:82//AC 005669
R-PLACE1001551
R-PLACE1001570//M.capricolum DNA for CONTIG MC188.//0.0043:305:57//Z33135
R-PLACE1001602//Homo sapiens genomic DNA of 8p21.3-p22 anti-oncogene of hepatocellular colorectal and non-small cell lung cancer, segment 3/11.//2.5e-82:408:98//AB020860
R-PLACE1001603//Homo sapiens KE05 protein mRNA, complete cds.//1.5e-40:295:84//AF064605
R-PLACE1001610//Homo sapiens clone NH0469M07, WORKING DRAFT SEQUENCE, 7 unordered pieces.//2.5e-39:307:82//AC005037
R-PLACE1001611//Homo sapiens histone macroH2A1.2 mRNA, complete cds.//4.9e-41:217:97//AF054174
R-PLACE1001632//Human DNA binding protein (HPF2) mRNA, complete cds.//1.4e-08:178:65//M27878
R-PLACE1001634//Caenorhabditis elegans DNA *** SEQUENCING IN PROGRESS *** from clone H06C16, WORKING DRAFT SEQUENCE.//0.00026:221:62//Z92791
R-PLACE1001640//Homo sapiens chromosome 17, clone hRPK.651_L_9, complete sequence.//2.6e-83:441:95//AC005971
R-PLACE1001672//H.sapiens flow-sorted chromosome 6 TaqI fragment, SC6pA26H8.//0.91:115:69//Z79253
R-PLACE1001691//Homo sapiens okadaic acid-inducible phosphoprotein (OA48-18) mRNA, complete cds.//1.5e-111:545:97//AF069250
R-PLACE1001692//Homo sapiens clone RG228D17, WORKING DRAFT SEQUENCE, 2 unordered pieces.//1.0e-46:478:75//AC005077
R-PLACE1001705//Human DNA sequence *** SEQUENCING IN PROGRESS *** from clone 250D10, WORKING DRAFT SEQUENCE.//0.79:91:73//Z99716
R-PLACE1001716//Homo sapiens Xp22 PAC RPCI1-167A22 (from Roswell Park Cancer Center) complete sequence.//0.96:172:66//AC002349
R-PLACE1001720
R-PLACE1001729//Human interleukin-13 (IL-13) precursor gene, complete cds.//0.79:280:60//U31120
R-PLACE1001739//Homo sapiens chromosome 19, CIT-HSP-444n24, complete sequence.//1.0:109:65//AC005261
R-PLACE1001740//Homo sapiens BAC clone GS114I09 from 7p14-p15, complete sequence.//5.3e-11:249:67//AC006027
R-PLACE1001745
R-PLACE1001746//Homo sapiens chromosome 4 clone B200N5 map 4q25, complete sequence.//6.0e-05:337:61//AC005509
R-PLACE1001748//Homo sapiens metalloprotease 1 (MP1) mRNA, complete cds.//1.3e-91:540:89//AF061243
R-PLACE1001756//Human BAC clone RG302F04 from 7q31, complete sequence.//0.074:344:62//AC002463
R-PLACE1001761
R-PLACE1001771//Homo sapiens full-length insert cDNA clone ZD79C11.//4.4e-57:298:96//AF086426
R-PLACE1001781//T.thermophila micronuclear DNA containing to chromosomal breakage sequence Cbs-1, clone Tt819.//4.6e-05:282:61//M15711
R-PLACE1001799//Plasmodium falciparum 3D7 chromosome 12 PFYAC588 genomic sequence, WORKING DRAFT SEQUENCE, 2 unordered pieces.//0.015:331:58//AC004710
R-PLACE1001817//Homo sapiens ATP-specific succinyl-CoA synthetase beta subunit (SCS) mRNA, partial cds.//4.1e-92:463:95//AF058953
R-PLACE1001821//***ALU WARNING: Human Alu-J subfamily consensus sequence.//3.6e-36:281:82//U14567
R-PLACE1001845//Mus musculus Paneth cell enhanced expression PCEE mRNA, complete cds.//9.1e-26:313:73//U37351
R-PLACE1001869
R-PLACE1001897//Mus musculus homeobox protein (D1x5) mRNA, complete cds.//0.0043:207:64//AF033011
R-PLACE1001912//RPCI11-25F23.TKBR RPCI-11 Homo sapiens genomic clone RPCI-11-25F23, genomic survey sequence.//6.3e-33:248:67//AQ013567
R-PLACE1001920//Homo sapiens TNF-induced protein GG2-1 mRNA, complete cds.//5.0e-73:363:98//AF070671
R-PLACE1001928//Homo sapiens chromosome 17, clone hRPK.642_C_21, complete sequence.//0.98:248:60//AC005245
R-PLACE1001983//Caenorhabditis elegans DNA *** SEQUENCING IN PROGRESS *** from clone Y40H7, WORKING DRAFT SEQUENCE.//0.12:157:61//AL021389
R-PLACE1001989//Human DNA sequence *** SEQUENCING IN PROGRESS *** from clone 968D22, WORKING DRAFT SEQUENCE.//1.4e-44:376:80//AL023755
R-PLACE1002046//CITBI-E1-2520J24.TF CITBI-E1 Homo sapiens genomic clone 2520J24, genomic survey sequence.//4.5e-20:144:89//AQ280117
R-PLACE1002052//Human DNA sequence from cosmid U160A4, between markers DXS366 and DXS87 on chromosome X contains STS.//0.025:362:57//Z80900
R-PLACE1002066//Leishmania tarentolae maxicircle DNA fragment.//0.0034:197:62//X02438
R-PLACE1002072//Homo sapiens chromosome 5, P1 clone 854b11 (LBNL H44), complete sequence.//9.7e-06:414:60//AC004763
R-PLACE1002073
R-PLACE1002090//Homo sapiens Chromosome 16 BAC clone CIT987-SKA-345G4 ∼complete genomic sequence, complete sequence.//1.8e-06:278:63//AC002302
R-PLACE1002115//Human DNA sequence *** SEQUENCING IN PROGRESS *** from clone Y214H10, WORKING DRAFT SEQUENCE.//6.0e-12:327:64//AL022344
R-PLACE1002119//Mus musculus IERS (Ier5) mRNA, complete cds.//5.1e-67:442:86//AF079527
R-PLACE1002140//Homo sapiens DNA sequence from PAC 454M7 on chromosome Xq25-26.3. Contains the OCRL1 gene for Lowe Oculocerebrorenal Syndrome protein OCRL-1.
Contains ESTs, STSs and GSSs, complete sequence.//2.2e-80:403:97//AL022162 R-PLACE1002150//Human DNA sequence from PAC 145B12 on chromosome Xq27-Xq28. Contains EST, CA repeat and STS.//0.043:455:59//AL008706
R-PLACE1002157//Human DNA sequence from Fosmid 65B7 on chromosome 22q11.2-qter. Contains exons 6-12 of the SLC5A1 (SGLT1) gene for solute carrier family 5 (sodium/glucose cotransporter) member 1 (High Affinity Sodium-Glucose Cotransporter), complete sequence.//9.8e-58:384:79//Z83849
R-PLACE1002163//Canis familiaris MHC class IIA DLA-DQA (DQA 1 allele) gene, exon 2, partial cds.//0.82:96:70//U44785
R-PLACE1002171//Homo sapiens PAC clone DJ1100F23 from 7q31, complete sequence.//0.83:196:65//AC004456
R-PLACE1002205//Human DNA sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.//0.0017:193:61//Z94056
R-PLACE1002213//Homo sapiens chromosome 19, fosmid 37308, complete sequence.//8.0e-42:330:81//AC004152
R-PLACE1002227//Homo sapiens BAC clone BK085E05 from 22q12.1-qter, complete sequence.//2.1e-10:126:80//AC003071
R-PLACE1002256//Homo sapiens clone DJ0853H20, WORKING DRAFT SEQUENCE, 5 unordered pieces.//2.7e-06:478:57//AC004907
R-PLACE1002259//Human DNA sequence from cosmid U75A4 on chromosome X.//6.5e-81:501:88//Z82255
R-PLACE1002319//Plasmodium falciparum 3D7 chromosome 12 PFYAC181 genomic sequence, WORKING DRAFT SEQUENCE, 8 unordered pieces.//0.00023:549:58//AC005505
R-PLACE1002342//Homo sapiens mRNA for KIAA0728 protein, partial cds.//4.9e-94:501:93//AB018271
R-PLACE1002395//Homo sapiens chromosome 19, cosmid R34382, complete sequence.//1.4e-69:385:93//AC005329
R-PLACE1002399//Human HepG2 3' region cDNA, clone hmd5d06.//2.4e-71:411:92//D16939
R-PLACE1002433//Human DNA sequence *** SEQUENCING IN PROGRESS *** from clone 173D1, WORKING DRAFT SEQUENCE.//0.85:176:63//AL031984
R-PLACE1002437//Human BAC clone RG114A06 from 7q31, complete sequence.//0.0040:213:63//AC002542
R-PLACE1002438//CITBI-E1-2501M20.TF.1 CITBI-E1 Homo sapiens genomic clone 2501M20, genomic survey sequence.//0.70:247:61//AQ242104
R-PLACE1002450//Homo sapiens 959 kb contig between AML1 and CBR1 on chromosome 21q22; segment 1/3.//0.00060:471:59//AJ229041
R-PLACE1002465//Homo sapiens clone DJ0673M15, WORKING DRAFT SEQUENCE, 33 unordered pieces.//2.5e-10:98:81//AC004854
R-PLACE1002474//Mus musculus matrilin-2 precursor mRNA, complete cds.//1.7e-25:199:71//U69262
R-PLACE1002477//Human DNA sequence from PAC 50A13 on chromosome Xp11. Contains ATP SYNTHASE LIPID BINDING PROTEIN P1 (P2, P3) precursor (ATP5G1, ATP5G2, ATP5G3) like pseudogene, ESTs and STSs. Contains polymorphic CA repeat.//1.2e-11:382:63//Z92545
R-PLACE1002493//Homo sapiens signal transducing adaptor molecule 2A (STAM2) mRNA, complete cds.//1.1e-53:307:91//AF042273
R-PLACE1002499//Plasmodium falciparum MAL3P6, complete sequence.//0.56:270:60//Z98551
R-PLACE1002500//CIT-HSP-2337C20.TR CIT-HSP Homo sapiens genomic clone 2337C20, genomic survey sequence.//3.2e-42:297:85//AQ037614
R-PLACE1002514//Human DNA Sequence *** SEQUENCING IN PROGRESS *** from clone 212A2, WORKING DRAFT SEQUENCE.//7.8e-16:221:73//Z95114
R-PLACE1002529//Homo sapiens mRNA for KIAA0713 protein, partial cds.//1.6e-86:582:85//AB018256
R-PLACE1002532//Homo sapiens BAC clone RG300E22 from 7q21-q31.1, complete sequence.//9.0e-91:453:97//AC004774
R-PLACE1002537//Hansenula wingei mitochondrial gene for NADH dehydrogenase subunit 5, complete cds.//0.0042:489:60//D16253
R-PLACE1002571//Apis mellifera ligustica complete mitochondrial genome.//0.034:493:55//L06178
R-PLACE1002578//Homo sapiens chromosome 5, Pac clone 9c13 (LBNL H127), complete sequence.//2.5e-44:292:84//AC006084
R-PLACE1002583//Homo sapiens wbscr1 (WBSCR1) and replication factor C subunit 2 (RFC2) genes, complete cds.//3.1e-17:517:61//AF045555
R-PLACE1002591
R-PLACE1002598//Caenorhabditis elegans cosmid Y37D8A, complete sequence.//0.080:308:60//AL032626
R-PLACE1002604//Human cosmid LL12NC01-88A9, ETV6 gene, exons 6, 7 and 8 and partial cds.//0.0013:176:65//U63313
R-PLACE1002625//HS_2233_B2_H04_MF CIT Approved Human Genomic Sperm Library D Homo sapiens genomic clone Plate=2233 Col=8 Row=P, genomic survey sequence.//5.2e-13:137:79//AQ146663
R-PLACE1002665//Mus musculus enhancer of polycomb (Epc1) mRNA, complete cds.//5.8e-46:272:94//AF079765
R-PLACE1002685//Homo sapiens B cell linker protein BLNK mRNA, alternatively spliced, complete cds.//1.2e-77:390:97//AF068180
R-PLACE1002714//Mus musculus clone OST2473, genomic survey sequence.//1.3e-35:328:78//AF046656
R-PLACE1002722//Sequence 1 from patent US 5686597.//1.7e-42:276:89//I73723
R-PLACE1002768//Homo sapiens Xp22 bins 169-171 BAC GSHB-383H3 (Genome Systems Human BAC Library) complete sequence.//0.0098:197:64//AC005185
R-PLACE1002772//Homo sapiens PAC clone DJ0560O14 from 7q21.1-q21.2, complete sequence.//6.7e-49:378:82//AC006145
R-PLACE1002782
R-PLACE1002794
R-PLACE1002811//CIT-HSP-2316H11.TF CIT-HSP Homo sapiens genomic clone 2316H11, genomic survey sequence.//6.0e-50:250:100//AQ034981
R-PLACE1002815//Sequence 2 from patent US 5747660.//2.7e-59:312:84//AR005279
R-PLACE1002816//Homo sapiens 12q13.1 PAC RPCI5-1057I20 (Roswell Park Cancer Institute Human PAC library) complete sequence.//6.3e-59:339:93//AC004466
R-PLACE1002834//Figure 2. Nucleotide and translated protein sequences of HPF1, -2, and-9.//1.4e-78:413:95//M27877
R-PLACE1002839//Homo sapiens PAC clone DJ0015I23 from 22, complete sequence.//6.5e-25:301:74//AC004819
R-PLACE1002851//CIT-HSP-2317M9.TR CIT-HSP Homo sapiens genomic clone 2317M9, genomic survey sequence.//0.0011:210:61//AQ040519
R-PLACE1002853//Human interleukin 6 (IL6) gene, 3' flank.//5.8e-06:327:61//J03049
R-PLACE1002881
R-PLACE1002908//HS_3064_A1_D04_MF CIT Approved Human Genomic Sperm Library D Homo sapiens genomic clone Plate=3064 Col=7 Row=G, genomic survey sequence.//1.9e-09:156:72//AQ142985
R-PLACE1002941
R-PLACE1002962
R-PLACE1002968//Human DNA sequence from clone 109F14 on chromosome 6p21.2-21.3. Contains the alternatively spliced gene for Transcriptional Enhancer Factor TEF-5, the 60S Ribosomal Protein RPL10A gene, a PUTATIVE ZNF127 LIKE gene, and the PPARD for Peroxisome Proliferator Activated Receptor Delta (PPAR-Delta, PPAR-Beta, Nuclear Hormone Receptor 1, NUC1, NUCI, PPARB). Contains three putative CpG islands, ESTs, STSs, GSSs and a ca repeat polymorphism, complete sequence.//1.9e-32:314:77//AL022721
R-PLACE1002991//Human DNA sequence *** SEQUENCING IN PROGRESS *** from clone 968D22, WORKING DRAFT SEQUENCE.//1.6e-42:343:81//AL023755
R-PLACE1002993//Homo sapiens PAC clone DJ0899E09 from 7q11.23-q21.1, complete sequence.//0.56:88:72//AC004921
R-PLACE1002996//HS_2064_A1_A05_MR CIT Approved Human Genomic Sperm Library D Homo sapiens genomic clone Plate=2064 Col=9 Row=A, genomic survey sequence.//4.9e-18:117:95//AQ243211
R-PLACE1003025//Homo sapiens PAC clone DJ0560O14 from 7q21.1-q21.2, complete sequence.//0.26:428:58//AC006145
R-PLACE1003027//Homo sapiens chromosome 17, clone hRPK.700_H_6, complete sequence.//1.3e-95:465:98//AC005920
R-PLACE1003044
R-PLACE1003092//Plasmodium falciparum DNA *** SEQUENCING IN PROGRESS *** from contig 4-89, complete sequence.//3.6e-05:358:60//AL010266
R-PLACE1003100//HS_2244_A2_H12_MR CIT Approved Human Genomic Sperm Library D Homo sapiens genomic clone Plate=2244 Col=24 Row=O, genomic survey sequence.//2.3e-42:288:86//AQ084224
R-PLACE1003108//Homo sapiens clone DJ0781A18, WORKING DRAFT SEQUENCE, 3 unordered pieces.//0.00066:233:61//AC004885
R-PLACE1003136//Plasmodium falciparum MAL3P2, complete sequence.//0.019:429:57//AL034558
R-PLACE1003145
R-PLACE1003153//Homo sapiens Xp22 BAC GSHB-536K7 (Genome Systems Human BAC library) complete sequence.//3.2e-05:390:58//AC004616
R-PLACE1003174//Arabidopsis thaliana genomic DNA, chromosome 5, P1 clone: MTE17, complete sequence.//2.4e-06:390:60//AB015479
R-PLACE1003176
R-PLACE1003190//Homo sapiens clone RG332P12, WORKING DRAFT SEQUENCE, 1 unordered pieces.//4.0e-78:406:81//AC005095
R-PLACE1003200//Plasmodium falciparum MAL3P6, complete sequence.//0.016:411:57//Z98551
R-PLACE1003205//Plasmodium falciparum 3D7 chromosome 12 PFYAC336 genomic sequence, WORKING DRAFT SEQUENCE, 5 unordered pieces.//0.00084:288:61//AC005139
R-PLACE1003238//Homo sapiens full-length insert cDNA clone ZD79H11.//7.6e-114:567:96//AF086432
R-PLACE1003249//Human Chromosome X, complete sequence.//1.3e-45:317:85//AC002416
R-PLACE1003256//Homo sapiens chromosome 17, clone HCIT421K24, complete sequence.//1.0e-45:328:85//AC004099
R-PLACE1003258
R-PLACE1003296//Diphoropria sp. 16S ribosomal RNA gene, mitochondrial gene encoding mitochondrial rRNA, partial sequence.//0.050:228:59//U39952
R-PLACE1003302//Figure 2. Nucleotide and translated protein sequences of HPF1, 2, and-9.//1.7e-91:458:96//M27877
R-PLACE1003334//Homo sapiens DNA sequence from BAC 217C2 on chromosome 22q13-ql3.33. Contains a gene for the presumtive isolog of Rat RTP60 (nuclear pore complex protein Npap60). Contains ESTs, complete sequence.//4.3e-34:370:71//Z82243
R-PLACE1003342//CIT-HSP-2311D21.TF CIT-HSP Homo sapiens genomic clone 2311D21, genomic survey sequence.//1.0:159:68//AQ020460
R-PLACE1003343//Plasmodium falciparum 3D7 chromosome 12 PFYAC812 genomic sequence, WORKING DRAFT SEQUENCE, 8 unordered pieces.//1.1e-05:330:61//AC004153
R-PLACE1003353//Homo sapiens breast cancer antiestrogen resistance 3 protein (BCAR3) mRNA, complete cds.//3.4e-98:469:98//U92715
R-PLACE1003361
R-PLACE1003366//Homo sapiens CAG repeated sequence.//0.018:319:61//AJ006805
R-PLACE1003369//T18H17-T7 TAMU Arabidopsis thaliana genomic clone T18H17, genomic survey sequence.//0.050:155:63//B20174
R-PLACE1003373//Homo sapiens chromosome 17, clone hRPC.1050_D_4, complete sequence.//1.2e-62:434:83//AC004771
R-PLACE1003375//Dictyostelium discoideum golvesin (gol) gene, complete cds.//0.042:263:57//U89350
R-PLACE1003383//Homo sapiens genomic DNA of 9q32 anti-oncogene of flat epitherium cancer , segment 10/10.//1.7e-83:429:96//AB020878
R-PLACE1003401//Homo sapiens chromosome 17, clone hRPK.85_B_7, complete sequence.//2.4e-13:175:76//AC005695
R-PLACE1003420//Homo sapiens PAC clone DJ0988G15 from 7q33-q35, complete sequence.//2.1e-05:340:61//AC005587
R-PLACE1003454//Plasmodium falciparum DNA *** SEQUENCING IN PROGRESS *** from contig 3-64, complete sequence.//0.47:411:58//AL009014
R-PLACE1003478//M.capricolum DNA for CONTIG MC175.//0.51:253:59//Z33125
R-PLACE1003493//Homo sapiens chromosome 17, clone hRPK.394_K_10, complete sequence.//4.6e-37:319:81//AC006080
R-PLACE1003516//CIT-HSP-2295M19.TF CIT-HSP Homo sapiens genomic clone 2295M19, genomic survey sequence.//1.0e-40:251:90//AQ007480
R-PLACE1003519//Homo sapiens chromosome 21q22.3 PAC 141B3, complete sequence, containing ribosomal protein homologue pseudogene L23a.//2.7e-29:163:89//AF064859
R-PLACE1003521//HS_3252_A2_G05_MR CIT Approved Human Genomic Sperm Library D Homo sapiens genomic clone Plate=3252 Col=10 Row=M, genomic survey sequence.//0.00017:274:60//AQ221562
R-PLACE1003528//HS_2041_B1_B07_MR CIT Approved Human Genomic Sperm Library D Homo sapiens genomic clone Plate=2041 Col=13 Row=D, genomic survey sequence.//6.6e-40:219:83//AQ230483
R-PLACE1003537//Drosophila melanogaster mitochondrial cytochrome c oxidase subunits, ATPase6, 7 tRNAs (Trp, Cys, Tyr, Leu(UUR), Lys, Asp, Gly) genes, and unidentified reading frames A61, 2 and 3.//8.3e-05:300:61//J01404
R-PLACE1003553//Human DNA sequence *** SEQUENCING IN PROGRESS *** from clone 97P20, WORKING DRAFT SEQUENCE.//2.7e-87:450:96//AL031297
R-PLACE1003566
R-PLACE1003575//Homo sapiens chromosome 16, cosmid clone 325D7, complete sequence.//4.7e-20:148:78//AC003965
R-PLACE1003583//Human DNA sequence from PAC 388N15 on chromosome Xq21.1.//3.5e-18:287:68//Z99571
R-PLACE1003584
R-PLACE1003592//Homo sapiens cosmid 223D9 from Xq28, complete sequence.//2.5e-10:153:73//AF061032
R-PLACE1003593//Human BAC clone RG030H15 from 7q31, complete sequence.//6.9e-07:240:65//AC002066
R-PLACE1003596//Caenorhabditis elegans DNA *** SEQUENCING IN PROGRESS *** from clone Y87G2, WORKING DRAFT SEQUENCE.//0.13:393:60//AL022597
R-PLACE1003602//Homo sapiens mRNA expressed in placenta.//2.4e-95:576:88//D83200
R-PLACE1003605//Homo sapiens BAC clone RG331C24 from 7q21, complete sequence.//2.9e-19:302:71//AC002081
R-nnnnnnnnnnnn
R-PLACE1003618//Human DNA sequence *** SEQUENCING IN PROGRESS *** from clone 191E19, WORKING DRAFT SEQUENCE.//8.3e-57:469:80//AL034451
R-PLACE1003625//Plasmodium falciparum 3D7 chromosome 12 PFYAC69 genomic sequence, WORKING DRAFT SEQUENCE, 4 unordered pieces.//2.1e-05:339:62//AC004688
R-PLACE1003638//Human DNA sequence *** SEQUENCING IN PROGRESS *** from clone 1104E15, WORKING DRAFT SEQUENCE.//2.5e-38:279:84//AL022312
R-PLACE1003669//HS_3054_A2_E07_MR CIT Approved Human Genomic Sperm Library D Homo sapiens genomic clone Plate=3054 Col=14 Row=I, genomic survey sequence.//0.014:265:61//AQ132713
R-PLACE1003704//HS_3213_A1_D12_T7 CIT Approved Human Genomic Sperm Library D Homo sapiens genomic clone Plate=3213 Col=23 Row=G, genomic survey sequence.//0.80:195:61//AQ176784
R-PLACE1003709//Human BAC clone RG126M09 from 7q21-q22, complete sequence.//0.018:152:61//AC002067
R-PLACE1003711//Human endothelial nitric oxide synthase gene, complete cds.//1.7e-61:366:89//D26607
R-PLACE1003723//Homo sapiens DNA sequence from clone 78F24 on chromosome 22q12.1-12.3. Contains one exon of an Oxysterol-binding protein (OSBP) LIKE gene. Contains GSSs and an STS, complete sequence.//2.7e-44:505:73//AL022336
R-PLACE1003738//H.sapiens DNA sequence.//0.93:185:60//Z22357
R-PLACE1003760//Human globin gene.//5.9e-97:538:91//M69023
R-PLACE1003762//Homo sapiens chromosome 17, clone HCIT39G8, complete sequence.//4.6e-13:134:79//AC003070
R-PLACE1003768//Homo sapiens chromosome 17, clone hRPK.142_H_19, complete sequence.//5.4e-12:189:71//AC005919
R-PLACE1003771//Homo sapiens BAC clone GS164B05 from 7p21-p22, complete sequence.//1.7e-119:619:95//AC004160
R-PLACE1003783
R-PLACE1003784//Homo sapiens chromosome 19, CIT-HSP-87m17 BAC clone, complete sequence.//5.6e-15:204:74//AC004659
R-PLACE1003795//CIT-HSP-2374C8.TR CIT-HSP Homo sapiens genomic clone 2374C8, genomic survey sequence.//7.0e-37:234:89//AQ114933
R-PLACE1003833//Homo sapiens full-length insert cDNA clone ZE15C06.//4.4e-59:313:95//AF086558
R-PLACE1003850
R-PLACE1003858
R-nnnnnnnnnnnn
R-PLACE1003870//Homo sapiens Chromosome 22q11.2 Cosmid Clone 15a10 In DGCR Region, complete sequence.//8.7e-33:285:81//AC000072
R-nnnnnnnnnnnn
R-PLACE1003886
R-PLACE1003888//Homo sapiens chromosome 4 clone B71M12 map 4q25, complete sequence.//0.73:127:65//AC004069
R-PLACE1003900//Homo sapiens ADP/ATP carrier protein (ANT-2) gene, complete cds.//1.9e-05:239:59//L78810
R-PLACE1003903//Homo sapiens full=length insert cDNA clone ZD78D11.//8.1e-74:369:97//AF086422
R-PLACE1003915//Mus musculus bone morphogenetic protein-6 (BMP-6) gene, exons 6 and 7 and complete cds.//0.56:247:61//U73520
R-PLACE1003923//Caenorhabditis elegans cosmid Y57G11C, complete sequence.//0.67:213:63//Z99281
R-PLACE1003932//Human DNA sequence from cosmid U90B3, on chromosome Xp11, contains ESTs.//8.7e-49:342:85//Z74022
R-PLACE1003936//H.sapiens gene for ventricular myosin light chain 2.//2.6e-09:394:61//Z15030
R-PLACE1003968//Plasmodium falciparum DNA *** SEQUENCING IN PROGRESS *** from contig 4-62, complete sequence.//1.3e-07:245:65//AL010247
R-PLACE1004104
R-PLACE1004114//Human PAC clone RG212D03, complete sequence.//5.0e-07:336:61//AC002485
R-PLACE1004118//HS_3092_B1_B01_MR CIT Approved Human Genomic Sperm Library D Homo sapiens genomic clone Plate=3092 Col=1 Row=D, genomic survey sequence.//0.80:207:60//AQ128151
R-PLACE1004128//Rattus norvegicus guanine nucleotide binding protein beta 4 subunit mRNA, partial cds.//1.8e-06:193:66//AF022085
R-PLACE1004149//HS_2253_A2_F11_MR CIT Approved Human Genomic Sperm Library D Homo sapiens genomic clone Plate=2253 Col=22 Row=K, genomic survey sequence.//2.4e-59:315:95//AQ129711
R-PLACE1004156//Homo sapiens Xp22 bins 3-5 PAC RPCI4-617A9 (Roswell Park Cancer Institute Human PAC Library) containing Arylsulfatase D and E genes, complete sequence.//8.3e-53:299:76//AC005295
R-PLACE1004161
R-PLACE1004183//Homo sapiens for TOM1-like protein.//1.3e-80:434:93//AJ010071
R-PLACE1004197//RPCI11-69N15.TK RPCI11 Homo sapiens genomic clone R-69N15, genomic survey sequence.//0.0078:170:65//AQ265515
R-PLACE1004203//Homo sapiens semaphorin L (SEMAL) mRNA, complete cds.//3.4e-105:501:98//AF030698
R-PLACE1004242//Homo sapiens DNA sequence from PAC 124C6 on chromosome 6q21. Contains genomic marker D6S1603, ESTs, GSSs and a STS with a CA repeat polymorphism, complete sequence.//6.1e-65:373:86//AL021326
R-PLACE1004256//Homo sapiens BAC clone NH0044G14 from 7q11.23-21.1, complete sequence.//0.011:383:61//AC006031
R-PLACE1004257//Homo sapiens Xp22 BAC GSHB-433024 (Genome Systems Human BAC library) complete sequence.//3.4e-09:576:59//AC004470
R-PLACE1004258//HS_3034_A1_B12_MR CIT Approved Human Genomic Sperm Library D Homo sapiens genomic clone Plate=3034 Col=23 Row=C, genomic survey sequence.//1.4e-35:359:77//AQ128936
R-PLACE1004270//CITBI-E1-2504K14.TR CITBI-E1 Homo sapiens genomic clone 2504K14, genomic survey sequence.//2.7e-06:150:74//AQ261108
R-PLACE1004274//Homo sapiens BAC clone NH0436H22 from 2, complete sequence.//0.025:116:72//AC005234
R-PLACE1004277//Homo sapiens two pore domain K⁺ channel (TASK-2) mRNA, complete cds.//4.4e-106:581:91//AF084830
R-PLACE1004284//Plasmodium falciparum 3D7 chromosome 12 PFYAC492 genomic sequence, WORKING DRAFT SEQUENCE, 5 unordered pieces.//0.59:231:60//AC005308
R-FLACE1004289//Homo sapiens chromosome 17, clone hRPK.700_H_6, complete sequence.//5.8e-31:340:75//AC005920
R-PLACE1004302//Homo sapiens clone RG332P12, WORKING DRAFT SEQUENCE, 1 unordered pieces.//6.4e-90:572:86//AC005095
R-PLACE1004316//H.sapiens mRNA for apoptosis specific protein.//1.9e-113:590:94//Y11588
R-PLACE1004336//Human DNA sequence *** SEQUENCING IN PROGRESS *** from clone 1013A10, WORKING DRAFT SEQUENCE.//2.3e-65:292:82//AL033383
R-PLACE1004358//Homo sapiens connector enhancer of KSR-like protein CNK1 mRNA, complete cds.//2.4e-70:379:93//AF100153
R-PLACE1004376//CIT-HSP-2287M8.TF CIT-HSP Homo sapiens genomic clone 2287M8, genomic survey sequence.//0.47:173:61//AQ000837
R-PLACE1004384//CIT-HSP-2316J11.TF CIT-HSP Homo sapiens genomic clone 2316J11, genomic survey sequence.//0.035:109:69//AQ037817
R-PLACE1004388//Plasmodium falciparum DNA *** SEQUENCING IN PROGRESS *** from contig 3-82, complete sequence.//4.2e-06:381:60//AL010149
R-PLACE1004405//Homo sapiens clone GS512I21, WORKING DRAFT SEQUENCE, 9 unordered pieces.//0.20:270:60//AC005027
R-PLACE1004425//Homo sapiens PAC clone DJ0733B09 from 7p14-p13, complete sequence.//1.3e-96:516:94//AC005532
R-PLACE1004428//Human DNA sequence from clone 888M10 on chromosome 1p36.11-36.31 Contains part of gene KIAA0453, EST, STS, GSS, complete sequence.//5.8e-10:279:65//AL031296
R-PLACE1004437//Human NAD⁺-specific isocitrate dehydrogenase beta subunit precursor, mRNA, nuclear gene encoding mitochondrial protein, complete cds.//2.9e-88:516:88//U49283
R-PLACE1004451//HS_2258_B2_F01_MR CIT Approved Human Genomic Sperm Library D Homo sapiens genomic clone Plate=2258 Col=2 Row=L, genomic survey sequence.//0.82:172:61//AQ221189
R-PLACE1004460
R-PLACE1004467//Syrian hamster carbamoylphosphate synthetase-aspartate transcarbamylasedihydroorotase (CAD) gene, exons 1 and 2.//1.2e-24:311:62//M31621
R-PLACE1004471//Homo Sapiens Chromosome X clone bWXD75, complete sequence.//2.1e-34:333 :70//AC004389
R-PLACE1004473
R-PLACE1004491//Drosophila melanogaster Oregon-R mitochondrial A+T region.//1.0e-08:485:60//U11584
R-PLACE1004506
R-PLACE1004510//Plasmodium falciparum chromosome 2, section 64 of 73 of the complete sequence.//0.0094:543:56//AE001427
R-PLACE1004516//Homo sapiens BAC clone BK085E05 from 22q12.1-qter, complete sequence.//0.00011:343:59//AC003071
R-PLACE1004518
R-PLACE1004548//Homo sapiens Xp22 BAC GS-551O19 (Genome Systems Human BAC library) and cosmids U199A7 and U209F2 (Lawrence Livermore X chromosome cosmid library) containing part of human chloride channel 4 gene, complete sequence.//4.9e-40:245:80//AC003666
R-PLACE1004550
R-PLACE1004564//B.taurus mRNA for cleavage and polyadenylation specificity factor.//2.7e-82:532:86//X75931
R-PLACE1004629//Homo sapiens chromosome 7 clone UWGC:g3586a230 from 7p14-15, complete sequence.//0.015:437:59//AC004800
R-PLACE1004645//CIT-HSP-2370D6.TR CIT-HSP Homo sapiens genomic clone 2370D6, genomic survey sequence.//0.033:76:75//AQ110136
R-PLACE1004646//Homo sapiens cosmid 120C12 from Xq28, complete sequence.//2.0e-23:237:79//AF036876
R-PLACE1004658//Homo sapiens Chromosome 12p13.3 BAC RPCI11-21K20 (Roswell Park Cancer Institute Human BAC Library) complete sequence.//7.1e-09:94:87//AC005343
R-nnnnnnnnnnnn//RPCI11-79G23.TV RPCI11 Homo sapiens genomic clone R-79G23, genomic survey sequence.//2.2e-81:433:94//AQ283692
R-PLACE1004672//Human ABL gene, exon 1b and intron 1b, and putative M8604 Met protein (M8604 Met) gene, complete cds.//2.7e-24:263:74//U07561
R-PLACE1004674//Homo sapiens calcium binding protein (ALG-2) mRNA, complete cds.//1.1e-89:513:91//AF035606
R-PLACE1004681//Homo sapiens genomic DNA of 8p21.3-p22 anti-oncogene of hepatocellular colorectal and non-small cell lung cancer , segment 3/11.//1.3e-96:498:95//AB020860
R-PLACE1004686
R-PLACE1004691//Homo sapiens genomic DNA of 8p21.3-p22 anti-oncogene of hepatocellular colorectal and non-small cell lung cancer, segment 2/11.//2.1e-33:290:80//AB020859
R-PLACE1004693//Caenorhabditis elegans cosmid Y2H9A, complete sequence.//1.0:195:60//AL021448
R-PLACE1004716//CITBI-E1-2519C14.TR CITBI-E1 Homo sapiens genomic clone 2519C14, genomic survey sequence.//5.0e-43:245:93//AQ276965
R-PLACE1004722//Plasmodium falciparum 3D7 chromosome 12 PFYACB8-628 genomic sequence, WORKING DRAFT SEQUENCE, 9 unordered pieces.//0.0022:360:60//AC005507
R-PLACE1004736
R-PLACE1004740
R-nnnnnnnnnnnn//Homo sapiens ubiquitin-protein ligase E3-alpha (UBR1) mRNA, partial cds.//5.4e-105:575:92//AF061556
R-PLACE1004751//Homo sapiens Xq28 BACs 360 F12, GSHB-555C13, complete sequence.//9.0e-26:317:76//AC002523
R-PLACE1004773//Homo sapiens inversin protein mRNA, complete cds.//8.5e-88:437:96//AF084367
R-PLACE1004777//Homo sapiens genomic DNA, chromosome 21q22.2 (Down Syndrome region), segment 3/15, WORKING DRAFT SEQUENCE.//0.050:138:65//AP000010
R-PLACE1004793//Human endogenous retrovirus HERV-K(HML6) proviral clone HML6.17 putative polymerase and envelope genes, partial cds, and 3'LTR.//5.1e-58:313:80//U60269
R-nnnnnnnnnnnn//Homo sapiens mRNA for KIAA0606 protein, partial cds.//5.8e-98:580:88//AB011178
R-PLACE1004813//Plasmodium falciparum 3D7 chromosome 12 PFYACB8-420 genomic sequence, WORKING DRAFT SEQUENCE, 14 unordered pieces.//5.3e-09:256:64//AC005140
R-PLACE1004814//Homo sapiens okadaic acid-inducible phosphoprotein (OA48-18) mRNA, complete cds.//3.5e-107:358:99//AF069250
R-PLACE1004815//Human Chromosome 11q12.2 PAC clone pDJ606g6, complete sequence.//3.8e-61:353:89//AC004126
R-PLACE1004824//Homo sapiens chromosome 17, clone hCIT.468_F_23, WORKING DRAFT SEQUENCE, 3 unordered pieces.//5.7e-42:364:79//AC004666
R-PLACE1004827//Homo sapiens Xp22 BAC GS-594A7 (Genome Systems Human BAC library) contains Bmx gene, complete sequence.//2.7e-14:156:79//AC003669
R-PLACE1004836//HS_2270_A2_H10_MR CIT Approved Human Genomic Sperm Library D Homo sapiens genomic clone Plate=2270 Col=20 Row=O, genomic survey sequence.//8.6e-51:267:96//AQ164110
R-PLACE1004838//CIT-HSP-2343E10.TR CIT-HSP Homo sapiens genomic clone 2343E10, genomic survey sequence.//0.071:168:63//AQ058544
R-PLACE1004840//Sequence 4 from patent US 5728819.//1.6e-26:150:98//I92820
R-PLACE1004868//Human Chromosome X clone bWXD342, complete sequence.//0.57:344:59//AC004072
R-PLACE1004885//HS_3235_B2_E07_MR CIT Approved Human Genomic Sperm Library D Homo sapiens genomic clone Plate=3235 Col=14 Row=J, genomic survey sequence.//1.1e-38:175:78//AQ210193
R-PLACE1004900//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//2.0e-44:334:84//AL022577
R-PLACE1004902
R-nnnnnnnnnnnn//Human DNA sequence from clone J428A131, WORKING DRAFT SEQUENCE.//7.7e-58:377:87//Z82209
R-PLACE1004918//Homo sapiens Chromosome 16 BAC clone CIT987SK-A-248F7, complete sequence.//0.00084:373:60//AC004605
R-PLACE1004930//Homo sapiens MDC-3.13 isoform 1 mRNA, complete cds.//2.0e-100:532:93//AF099936
R-PLACE1004934//Homo sapiens clone RG062N11, WORKING DRAFT SEQUENCE, 2 unordered pieces.//0.00030:198:66//AC005683
R-PLACE1004937//Caenorhabditis elegans SEL-10 (sel-10) mRNA, complete cds.//1.3e-13:367:61//AF020788
R-PLACE1004969//Human DNA sequence from clone LUCA7 on chromosome 3, complete sequence.//0.97:116:71//Z84494
R-PLACE1004972
R-PLACE1004979//Plasmodium falciparum MAL3P4, complete sequence.//0.74:304:60//AL008970
R-PLACE1004982//Plasmodium falciparum 3D7 chromosome 12 PFYAC492 genomic sequence, WORKING DRAFT SEQUENCE, 5 unordered pieces.//4.7e-05:495:57//AC005308
R-PLACE1004985//Human DNA sequence *** SEQUENCING IN PROGRESS *** from clone 451B21, WORKING DRAFT SEQUENCE.//2.5e-10:410:60//AL033522
R-PLACE1005026//Homo sapiens PAC clone DJ0907C10 from 7q31-3q32, complete sequence.//2.7e-56:158:99//AC004925
R-PLACE1005027
R-PLACE1005046//Homo sapiens chromosome 19, cosmid F20237, complete sequence.//3.1e-63:438:86//AC005775
R-PLACE1005052//Homo sapiens chromosome Xp22-135-136 clone GSHB-567I1, WORKING DRAFT SEQUENCE, 35 unordered pieces.//6.1e-87:301:98//AC005867
R-PLACE1005066//Human DNA sequence from clone 67K17 on chromosome 6q24.1-24.3. Contains the HIVEP2 (Schnurri-2) gene for HIV type 1 Enhancer-binding Protein 2, and a possible pseudogene in an intron of this gene. Contains STSs and GSSs and an AAAT repeat polymorphism, complete sequence.//1.1e-09:453:61//AL023584
R-PLACE1005077//H.sapiens genes for semenogelin I and semenogelin II.//2.6e-05:199:66//Z47556
R-PLACE1005085//Homo sapiens chromosome 17, clone hRPK.293_K_20, complete sequence.//2.1e-42:384:69//AC005495
R-PLACE1005086//RPCI11-30H10.TV RPCI-11 Homo sapiens genomic clone RPCI-11-30H10, genomic survey sequence.//0.13:112:67//B87788
R-PLACE1005101//Homo sapiens (clone zap128) mRNA, 3' end of cds.//2.5e-97:531:92//L40401
R-PLACE1005102//Homo sapiens chromosome 19, cosmid R29388, complete sequence.//1.3e-91:504:92//AC004476
R-PLACE1005108//Homo sapiens BAC129, complete sequence.//4.0e-28:232:84//U85195
R-PLACE1005111//Human DNA sequence *** SEQUENCING IN PROGRESS *** from clone 566H6, WORKING DRAFT SEQUENCE.//3.0e-18:174:74//AL031845
R-PLACE1005128
R-PLACE1005146
R-PLACE1005162//Plasmodium falciparum 3D7 chromosome 12 PFYACB8-420 genomic sequence, WORKING DRAFT SEQUENCE, 14 unordered pieces.//2.4e-07:273:61//AC005140
R-nnnnnnnnnnnn//Rat alternatively spliced mRNA.//8.1e-20:185:82//M93018
R-PLACE1005181//HS_2182_B2_B05_MF CIT Approved Human Genomic Sperm Library D Homo sapiens genomic clone Plate=2182 Col=10 Row=D, genomic survey sequence.//4.9e-05:193:65//AQ030787
R-PLACE1005187//Arabidopsis thaliana chromosome II BAC T14A4 genomic sequence, complete sequence.//0.00073:264:60//AC006161
R-PLACE1005206//Homo sapiens full-length insert cDNA YN66A06.//6.3e-64:343:93//AF075043
R-PLACE1005232//Human DNA sequence *** SEQUENCING IN PROGRESS *** from clone 25J6, WORKING DRAFT SEQUENCE.//1.3e-34:286:81//Z84476
R-PLACE1005243
R-PLACE1005261//Caenorhabditis elegans cosmid ZK666, complete sequence.//0.66:180:60//Z49132
R-PLACE1005266//Homo sapiens clone RG122E10, complete sequence.//1.3e-15:166:78//AC005067
R-PLACE1005277//CITBI-E1-2514D4.TF CITBI-E1 Homo sapiens genomic clone 2514D4, genomic survey sequence.//2.5e-34:358:74//AQ265720
R-PLACE1005287//Plasmodium falciparum DNA *** SEQUENCING IN PROGRESS *** from MAL1P1, WORKING DRAFT SEQUENCE.//4.1e-07:495:60//AL031744
R-PLACE1005305//HS_3180_B2_D02_MR CIT Approved Human Genomic Sperm Library D Homo sapiens genomic clone Plate=3180 Col=4 Row=H, genomic survey sequence.//1.1e-42:308:85//AQ169443
R-PLACE1005308
R-PLACE1005313//Human Chromosome 11 pac pDJ227b23, WORKING DRAFT SEQUENCE, 19 unordered pieces.//0.00048:320:60//AC000383
R-PLACE1005327//chromosome 1 specific transcript KIAA0491.//5.4e-103:537:94//AB007960
R-PLACE1005331//Homo sapiens chromosome 19, cosmid F20569, complete sequence.//2.2e-94:536:91//AC004794
R-PLACE1005335//Human Chromosome 3 pac pDJ70i11, WORKING DRAFT SEQUENCE, 2 unordered pieces.//5.3e-32:313:79//AC000380
R-PLACE1005373//Homo sapiens BAC129, complete sequence.//8.8e-10:229:68//U85195
R-PLACE1005374//Homo sapiens chromosome 17, clone hRPK.401_O_9, complete sequence.//3.0e-44:434:77//AC005291
R-PLACE1005409//Human BAC clone RG167B05 from 7q21, complete sequence.//8.8e-105:529:96//AC003991
R-PLACE1005453//Human PAC clone DJ327A19 from Xq25-q26, complete sequence.//4.7e-39:302:82//AC002477
R-PLACE1005467//Human DNA sequence *** SEQUENCING IN PROGRESS *** from clone 167P19, WORKING DRAFT SEQUENCE.//1.1e-40:328:81//Z93014
R-PLACE1005471//Human DNA sequence from clone 395P12 on chromosome 1q24-25. Contains the TXGP1 gene for tax-transcriptionally activated glycoprotein 1 (34kD) (OX40 ligand, OX40L) and a GOT2 (Aspartate Aminotransferase, mitochondrial precursor, EC 2.6.1.1, Transaminase A, Glutamate Oxaloacetate Transaminase-2) pseudogene. Contains ESTs, STSs and GSSs, complete sequence.//6.4e-68:409:90//AL022310
R-PLACE1005477//Human DNA sequence *** SEQUENCING IN PROGRESS *** from clone 32B1, WORKING DRAFT SEQUENCE.//0.020:216:66//AL023693
R-PLACE1005480//Homo sapiens chromosome 19, CIT-HSP BAC 490g23 (BC338531), complete sequence.//2.8e-44:327:70//AC005392
R-PLACE1005481//Homo sapiens-chromosome 17, clone hRPC.1164_O_3, complete sequence.//4.2e-23:284:74//AC004703
R-PLACE1005494//Danio rerio homeobox protein LIM-3 (lim3) gene, exon 4.//0.19:468:60//AF031631
R-PLACE1005502//Homo sapiens formin binding protein 21 mRNA, complete cds.//1.6e-55:277:98//AF071185
R-PLACE1005526//Human mRNA for alpha-1 type II collagen.//0.10:227:63//X16468
R-PLACE1005528//Homo sapiens genomic DNA, chromosome 21q11.1, segment 9/28, WORKING DRAFT SEQUENCE.//2.3e-76:395:96//AP000038
R-PLACE1005530//C.familiaris CA repeat sequence (isolate ).//0.023:90:75//X86184
R-PLACE1005550//Fugu rubripes GSS sequence, clone 048A08bH1, genomic survey sequence.//2.0e-09:235:64//AL025928
R-PLACE1005554//Homo sapiens chromosome 17, clone hRPK.215_P_18, complete sequence.//0.069:305:60//AC005969
R-PLACE1005557//Homo sapiens chromosome 17, clone hRPC.117_B_12, complete sequence.//4.3e-105:587:91//AC004707
R-PLACE1005574//Human BAC 367D17 from chromosome 18, complete sequence.//1.5e-17:274:67//AC003971
R-PLACE1005584//Homo sapiens PAC clone DJ1186C01 from 7q21.2-q31.1, complete sequence.//2.7e-15:191:77//AC004991
R-PLACE1005595//Human Chromosome 11q12.2 PAC clone pDJ606g6, complete sequence.//6.4e-90:453:96//AC004126
R-PLACE1005603//Homo sapiens cosmid clone U169D2 from Xp22.1-22.2, complete sequence.//0.69:322:61//U72788
R-PLACE1005611//Borrelia burgdorferi plasmid cp18, OspE (ospE) gene, partial cds.//0.059:473:56//U42599
R-PLACE1005623//Homo sapiens full-length insert cDNA clone ZD76B03.//1.6e-113:575:95//AF086405
R-PLACE1005630//High throughput sequencing of human chromosome 12, WORKING DRAFT SEQUENCE, 1 ordered pieces.//5.6e-79:270:94//AC005840
R-PLACE1005639//Human BAC clone RG022J17 from 7q21, complete sequence.//8.2e-56:441:83//AC002382
R-PLACE1005646//Homo sapiens RNA helicase-related protein mRNA, complete cds.//3.2e-110:585:93//AF083255
R-PLACE1005656//Homo sapiens chromosome 17, clone hRPK.628_E_12, complete sequence.//8.6e-08:505:58//AC005701
R-PLACE1005666//Human DNA sequence from PAC 360E18 on chromosome X contains EST, CpG island and polymorphic CA repeat.//3.2e-27:307:72//Z82203
R-PLACE1005698//344B22.TV CIT978SKA1 Homo sapiens genomic clone A-344B22, genomic survey sequence.//0.030:91:70//B15144
R-PLACE1005727//Human variable number tandem repeat (VNTR) region, allele 17R1 3' to collagen type II (COL2A1) gene.//5.2e-10:587:59//L10171
R-PLACE1005730//Homo sapiens ADP/ATP carrier protein (ANT-2) gene, complete cds.//0.0039:239:58//L78810
R-PLACE1005739//Mus musculus IFN-gamma induced (Mg11) mRNA, complete cds.//2.2e-21:270:72//U15635
R-PLACE1005755//Caenorhabditis elegans cosmid M03F4.//6.9e-08:219:64//U64601
R-PLACE1005763//Human mRNA for KIAA0118 gene, partial cds.//1.0e-45:268:87//D42087
R-PLACE1005799//Human X chromsome mRNA for CCG1 protein inv. in cell proliferation.//0.030:91:78//X07024
R-PLACE1005802//Homo sapiens PAC clone DJ044L15 from Xq23, complete sequence.//1.4e-69:391:92//AC004827
R-PLACE1005803
R-PLACE1005804//Human BAC clone RG341D10 from 7p15-p21, complete sequence.//1.8e-21:175:75//AC002530
R-PLACE1005828//Homo sapiens chromosome 17, clone hRPC.971_F_3, WORKING DRAFT SEQUENCE, 1 ordered pieces.//2.9e-56:333:91//AC004150
R-PLACE1005834//Plasmodium falciparum DNA *** SEQUENCING IN PROGRESS *** from MAL1P2, WORKING DRAFT SEQUENCE.//0.020:513:55//AL031745
R-PLACE1005845//Rabbit mRNA for protein phosphatase 2A-beta.//1.8e-10:182:69//Y00763 R-PLACE1005850
R-PLACE1005851//Homo sapiens clone DJ0789I05, WORKING DRAFT SEQUENCE, 2 unordered pieces.//5.5e-06:318:63//AC004887
R-PLACE1005876//B.taurus mRNA for cleavage and polyadenylation specificity factor.//6.7e-28:366:72//X75931
R-PLACE1005884//Human DNA sequence from cosmid V526F1, between markers DXS366 and DXS87 on chromosome X contains STS.//1.0e-06:306:64//Z70281
R-PLACE1005898//Plasmodium falciparum 3D7 chromosome 12 PFYAC336 genomic sequence, WORKING DRAFT SEQUENCE, 5 unordered pieces.//0.0094:449:59//AC005139
R-PLACE1005921//CITBI-E1-2509N21.TF CITBI-E1 Homo sapiens genomic clone 2509N21,-genomic survey sequence.//4.8e-84:494:89//AQ261347
R-PLACE1005923//RPCI11-65N9.TJ RPCI11 Homo sapiens genomic clone R-65N9, genomic survey sequence.//8.3e-97:520:93//AQ237243
R-PLACE1005925//Human DNA sequence from clone 231L4 on chromosome Xq27.1-27.3 Contains GSS, STS, complete sequence.//5.2e-67:578:78//AL022719
R-PLACE1005932//Caenorhabditis elegans cosmid Y52B11A, complete sequence.//0.0035:176:62//AL032654
R-PLACE1005934
R-PLACE1005936//Arabidopsis Thaliana BAC F6A4, Chromosome IV, near 60.5 cM, complete sequence.//0.00021:272:62//AF069716
R-PLACE1005951
R-PLACE1005953//Caenorhabditis elegans cosmid F09E5.//1.3e-07:349:60//U37429
R-PLACE1005955//Human HepG2 3' region MboI cDNA, clone hmd1d01m3.//8.3e-08:128:70//D17131
R-PLACE1005966//Pontia protodice large subunit ribosomal RNA gene, partial sequence; tRNA-Val gene, complete sequence; and small subunit ribosomal RNA gene, partial sequence, mitochondrial genes for mitochondrial RNAs.//7.0e-09:549:59//AF044863
R-PLACE1005968//Rattus norvegicus mRNA for p47, complete cds.//1.1e-51:394:81//AB002086
R-PLACE1005990//Homo sapiens chromosome 12p13.3 clone RPCI11-407G6, WORKING DRAFT SEQUENCE, 51 ordered pieces.//4.4e-63:369:91//AC005866
R-PLACE1006002//Human cosmid CRI-JC2015 at D10S289 in 10sp13.//5.9e-27:299:74//U15177
R-PLACE1006003//Mus musculus clone OST18050, genomic survey sequence.//3.5e-07:164:67//AF046375
R-PLACE1006011//Mus musculus poly-(ADPribosyl)-transferase homolog PARP mRNA, complete cds.//1.1e-32:266:83//AF072521
R-PLACE1006017//Homo sapiens Chromosome 22q11.2 Cosmid Clone 31e In DGCR Region, complete sequence.//1.8e-17:164:82//AC000077
R-PLACE1006037//Mus musculus B6D2F1 clone 2C11B mRNA.//2.0e-49:557:72//U01139
R-PLACE1006040//Homo sapiens mRNA for alpha endosulfine.//4.3e-13:128:81//X99906
R-PLACE1006076//Homo sapiens clone DJ0781A18, WORKING DRAFT SEQUENCE, 3 unordered pieces.//3.3e-18:220:74//AC004885
R-PLACE1006119//Plasmodium berghei (STRAIN ANKA) gamma-GCS gene, complete CDS.//0.0050:271:63//AJ005122
R-PLACE1006129//Drosophila melanogaster, chromosome 2R, region 31C1-31D6, P1 clone DS08879, complete sequence.//0.43:178:65//AC005454
R-PLACE1006139//Homo sapiens PAC clone DJ0659J06 from 7q33-q35, complete sequence.//7.5e-13:222:68//AC004849
R-PLACE1006143//Plasmodium falciparum MAL3P6, complete sequence.//0.00019:455:59//Z98551
R-PLACE1006157//Plasmodium falciparum DNA *** SEQUENCING IN PROGRESS *** from MAL4P1, WORKING DRAFT SEQUENCE.//0.00018:351:60//AL034557
R-PLACE1006159//Homo sapiens chromosome 10 clone LA10NC01_124_D_3 map 10q25.1, WORKING DRAFT SEQUENCE, 1 ordered pieces.//1.0e-113:586:96//AC006103
R-PLACE1006164//Human hereditary haemochromatosis region, histone 2A-like protein gene, hereditary haemochromatosis (HLA-H) gene, RoRet gene, and sodium phosphate transporter (NPT3) gene, complete cds.//1.0e-28:342:75//U91328
R-PLACE1006167//Homo sapiens full-length insert cDNA clone ZE14E04.//4.6e-77:426:93//AF086555
R-nnnnnnnnnnnn//Mouse mRNA for alpha-adaptin (C).//3.0e-46:188:82//X14972
R-PLACE1006187//Homo sapiens cyclin E2 mRNA, complete cds.//1.6e-116:597:95//AF091433
R-PLACE1006195//Homo sapiens chromosome 19, fosmid 39554, complete sequence.//8.8e-11:148:74//AC004410
R-PLACE1006196
R-PLACE1006205//Genomic sequence from Mouse 11, complete sequence.//8.4e-44:332:85//AC000398
R-PLACE1006223//Human DNA sequence from cosmid U74C11, between markers DXS6791 and DXS8038 on chromosome X contains ESTs.//0.041:215:61//Z73362
R-PLACE1006225//Caenorhabditis elegans cosmid Y69H2, complete sequence.//9.7e-13:358:63//Z98877
R-PLACE1006236//Plasmodium falciparum MAL3P4, complete sequence.//0.00019:538:58//AL008970
R-nnnnnnnnnnnn//Homo sapiens BAC clone RG118D07 from 7q31, complete sequence.//3.1e-96:497:95//AC004142
R-PLACE1006246//Homo sapiens clone NH0144M13, WORKING DRAFT SEQUENCE, 1 unordered pieces.//0.029:499:56//AC006034
R-PLACE1006248//Homo sapiens mRNA for KIAA0648 protein, partial cds.//9.2e-96:499:95//AB014548
R-PLACE1006262//Homo sapiens Xp22 GSHB-314C4 (Genome Systems Human BAC library) complete sequence.//0.00043:160:66//AC004087
R-PLACE1006288//Human DNA sequence *** SEQUENCING IN PROGRESS *** from clone 20N2, WORKING DRAFT SEQUENCE.//3.5e-120:611:96//AL031320
R-PLACE1006318
R-PLACE1006325//Plasmodium falciparum MAL3P8, complete sequence.//1.0:426:57//AL034560
R-PLACE1006335//Human DNA sequence from PAC 849L7 on chromosome Xq21.//0.96:173:66//AL008987
R-PLACE1006357//P.falciparum complete gene map of plastid-like DNA (IR-B).//1.9e-07:491:58//X95276
R-PLACE1006360//Plasmodium falciparum chromosome 2, section 35 of 73 of the complete sequence.//0.25:484:56//AE001398
R-PLACE1006368//Caenorhabditis elegans cosmid Y38H6C, complete sequence.//1.0:240:59//AL031630
R-PLACE1006371//Homo sapiens chromosome 16, cosmid clone 360H6 (LANL), complete sequence.//3.7e-101:574:91//AC004232
R-PLACE1006382
R-PLACE1006385//Mus musculus intersectin-EH binding protein Ibp2 mRNA, partial cds.//1.4e-50:350:86//AF057286
R-PLACE1006412//Homo sapiens clone DJ0673M15, WORKING DRAFT SEQUENCE, 33 unordered pieces.//5.1e-51:339:82//AC004854
R-PLACE1006414//Homo sapiens 12p13.3 PAC RPCI5-927J10 (Roswell Park Cancer Institute Human PAC library) complete sequence.//1.6e-38:297:84//AC004804
R-PLACE1006438//Homo sapiens full-length insert cDNA YH73H06.//7.6e-73:422:90//AF074985
R-PLACE1006445//Human DNA sequence *** SEQUENCING IN PROGRESS *** from clone 1018K9, WORKING DRAFT SEQUENCE.//3.0e-07:376:61//AL031726
R-PLACE1006469
R-PLACE1006470//Mouse B1 repetitive sequence DNA.//1.0:96:66//M24152
R-PLACE1006482//Human DNA sequence *** SEQUENCING IN PROGRESS *** from clone 447C4, WORKING DRAFT SEQUENCE.//3.0e-101:535:94//AL021977
R-PLACE1006492//Homo sapiens chromosome 17, clone hRPK.180_P_8, complete sequence.//0.78:44:95//AC005972
R-PLACE1006506//R.norvegicus BSP gene.//1.0:206:60//X86100
R-PLACE1006521//RPCI11-13L8.TV RPCI-11 Homo sapiens genomic clone RPCI-11-13L8, genomic survey sequence.//9.0e-17:414:61//B75158
R-PLACE1006531//Plasmodium falciparum coronin gene, isolate 3D7.//0.98:186:63//AJ002197
R-PLACE1006534//Anopheles gambiae complete mitochondrial genome.//0.051:412:61//L20934
R-PLACE1006540//Homo sapiens clone UWGC:y55c025 from 6p21, complete sequence.//7.5e-41:470:70//AC004209
R-PLACE1006552//Caenorhabditis elegans DNA *** SEQUENCING IN PROGRESS *** from clone Y47D3, WORKING DRAFT SEQUENCE.//0.57:355:57//Z98865
R-PLACE1006598//Plasmodium falciparum 3D7 chromosome 12 PFYAC588 genomic sequence, WORKING DRAFT SEQUENCE, 2 unordered pieces.//0.016:291:58//AC004710
R-PLACE1006615//Homo sapiens eukaryotic translation initiation factor eIF3, p35 subunit mRNA, complete cds.//2.9e-116:590:95//U97670
R-PLACE1006617//Homo sapiens chromosome 4 clone B207D4 map 4q25, complete sequence.//2.2e-45:209:88//AC004050
R-PLACE1006626//C. elegans cosmid K12H4.//1.2e-16:344:64//L14331
R-PLACE1006629//Homo sapiens chromosome 19, cosmid F20900, complete sequence.//2.8e-25:343:70//AC006128
R-PLACE1006640//CIT-HSP-2169L1.TF CIT-HSP Homo sapiens genomic clone 2169L1, genomic survey sequence.//0.00020:201:62//B90038
R-PLACE1006673//Homo sapiens clone DJ076B20, WORKING DRAFT SEQUENCE, 6 unordered pieces.//1.4e-42:309:84//AC004882
R-PLACE1006678//Homo sapiens PAC clone DJ1166G19 from 7p12-p11.2, complete sequence.//6.4e-09:454:59//AC006024
R-PLACE1006704//Human DNA sequence from clone 249C1 on chromosome Xq21.1-22.2 Contains GSS, complete sequence.//0.56:226:63//AL022154
R-PLACE1006731//Homo sapiens clone 23923 mRNA sequence.//6.0e-101:486:98//AF038172
R-PLACE1006754//Homo sapiens chromosome 19, cosmid R29124, complete sequence.//1.4e-68:381:93//AC005626
R-PLACE1006760//Homo sapiens clone 24800 mRNA sequence.//6.2e-72:397:92//AF070622
R-PLACE1006779//Rattus norvegicus intestinal trefoil factor gene, promoter and partial cds.//1.6e-11:420:61//U20984
R-PLACE1006782//Caenorhabditis elegans DNA *** SEQUENCING IN PROGRESS *** from clone Y47D3, WORKING DRAFT SEQUENCE.//0.60:321:58//Z98865
R-PLACE1006792//Homo sapiens chromosome 4 clone C0026P05 map 4P16, complete sequence.//2.9e-40:379:77//AC005599
R-PLACE1006795//Homo sapiens BAC clone RG281G05 from 7p15-p21, complete sequence.//6.2e-07:291:63//AC005083
R-PLACE1006800//HS_2270_B1_D02_MR CIT Approved Human Genomic Sperm Library D Homo sapiens genomic clone Plate=2270 Col=3 Row=H, genomic survey sequence.//4.1e-76:367:99//AQ085793
R-PLACE1006805//Plasmodium falciparum 3D7 chromosome 12 PFYACB8-628 genomic sequence, WORKING DRAFT SEQUENCE, 9 unordered pieces.//0.00058:354:59//AC005507
R-PLACE1006815//HS_3028_B1_B04_MF CIT Approved Human Genomic Sperm Library D Homo sapiens genomic clone Plate=3028 Col=7 Row=D, genomic survey sequence.//1.5e-33:251:77//AQ120174
R-PLACE1006819//Human DNA sequence from PAC 121G13 on chromosome 6 contains flow sorted chromosome 6 HindIII fragment ESTs. polymorphic CA repeat, CpG island, CpG island genomic fragments.//1.4e-76:544:84//Z86062
R-PLACE1006829
R-PLACE1006860
R-PLACE1006867//Human DNA sequence *** SEQUENCING IN PROGRESS *** from clone 323M4, WORKING DRAFT SEQUENCE.//3.2e-107:549:95//AL033378
R-PLACE1006878//Homo sapiens full-length insert cDNA clone ZB55G05.//1.4e-46:241:97//AF086155
R-PLACE1006883//Homo sapiens chromosome 16, cosmid clone 360H6 (LANL), complete sequence.//1.3e-38:283:85//AC004232
R-nnnnnnnnnnnn
R-PLACE1006904//Human DNA sequence from PAC 360E18 on chromosome X contains EST, CpG island and polymorphic CA repeat.//4.1e-15:477:62//Z82203
R-PLACE1006917//Homo sapiens Xp22 bins 45-47 BAC GSHB-665N22 (Genome Systems Human BAC Library) complete sequence.//1.3e-42:305:87//AC005184
R-PLACE1006932
R-PLACE1006935//Human DNA sequence from PAC 117P19 on chromosome X.//0.0014:114:74//Z86061
R-nnnnnnnnnnnn//Mouse mRNA for germ cell specific protein APG-1, complete cds.//9.5e-85:590:83//D49482
R-PLACE1006961//Homo sapiens chromosome 17, clone hRPK.349_A_8, complete sequence.//6.7e-42:295:86//AC005544
R-PLACE1006962//Homo sapiens Xp22 PAC RPCI1-167A22 (from Roswell Park Cancer Center) complete sequence.//1.1e-19:302:71//AC002349
R-PLACE1006966//HS_2219_B2_C02_MF CIT Approved Human Genomic Sperm Library D Homo sapiens genomic clone Plate=2219 Col=4 Row=F, genomic survey sequencer.//0.019:180:63//AQ145873
R-PLACE1006989
R-PLACE1007014
R-PLACE1007021//Homo sapiens chromosome 12p13.3 clone RPCI3-454B23, WORKING DRAFT SEQUENCE, 48 unordered pieces.//1.6e-23:362:70//AC005845
R-PLACE1007045//Human DNA sequence *** SEQUENCING IN PROGRESS *** from clone 32B1, WORKING DRAFT SEQUENCE.//2.3e-90:584:86//AL023693
R-PLACE1007053//Homo sapiens clone DJ0810E06, WORKING DRAFT SEQUENCE, 8 unordered pieces.//2.4e-108:550:96//AC004895
R-PLACE1007097//Homo sapiens DNA sequence from BAC 55C20 on chromosome 6. Contains a Spinal Muscular Atrophy (SMA3) LIKE gene overlapping with a beta-glucoronidase LIKE pseudogene. Contains a membrane protein LIKE pseudogene, a Glyceraldehyde 3-phosphate dehydrogenase (GAPDH) LIKE pseudogene, five predicted tRNA genes. Contains ESTs, GSSs (BAC end sequences) and a CA repeat polymorphism, complete sequence.//1.8e-103:552:93//AL021368
R-PLACE1007105//Mus musculus muskelin mRNA, complete cds.//2.7e-32:379:73//U72194
R-PLACE1007111//Plasmodium falciparum 3D7 chromosome 12 PFYAC69 genomic sequence, WORKING DRAFT SEQUENCE, 4 unordered pieces.//0.14:422:57//AC004688
R-PLACE1007112//Cynips cornifex cytb gene.//0.020:427:58//AJ228479
R-PLACE1007132//Homo sapiens full-length insert cDNA YH77E09.//5.7e-107:535:96//AF074987
R-PLACE1007140//Homo sapiens clone RG030L05, WORKING DRAFT SEQUENCE, 3 unordered pieces.//0.36:408:58//AC005050
R-PLACE1007178//Homo sapiens clone HEA4 Cri-du-chat region mRNA.//0.99:63:73//AF009283
R-PLACE1007226
R-PLACE1007238
R-PLACE1007239//Homo sapiens mRNA for transcription elongation factor S-II, hS-II-T1, complete cds.//2.0e-91:534:89//D50495
R-PLACE1007242//CITBI-E1-2512M9.TF CITBI-E1 Homo sapiens genomic clone 2512M9, genomic survey sequence.//1.3e-05:117:76//AQ279454
R-PLACE1007243//Prototheca wickerhamii 263-11 complete mitochondrial DNA.//0.21:284:58//U02970
R-PLACE1007257//Homo sapiens mRNA for dia-12c protein.//6.9e-113:607:93//Y15908
R-PLACE1007274//Homo sapiens chromosome 17, clone hRPK.394_K_10, complete sequence.//4.4e-10:135:74//AC006080
R-PLACE1007276//Homo sapiens BAC clone 255A7 from 8q21 containing NBS1 gene, complete sequence.//1.7e-36:435:72//AF069291
R-PLACE1007282//B.garinii (strain TIs1) p83/100 gene (partial).//0.95:183:60//X81533
R-PLACE1007286//RPCI11-13L8.TV RPCI-11 Homo sapiens genomic clone RPCI-11-13L8, genomic survey sequence.//6.1e-55:519:76//B75158
R-PLACE1007301//Human DNA sequence from PAC 106H8 on chromosome 1q24. Contains PHOSPHATIDYLINISITOL-GLYCAN class C (PIG-C) and DYNAMIN-3 genes. Contains ESTs and STSs and a CpG island.//0.75:180:62//Z97195
R-PLACE1007317//Drosophila dasycnemia 16S ribosomal RNA gene, mitochondrial gene for mitochondrial RNA, partial sequence.//0.59:236:59//U94253
R-PLACE1007342
R-PLACE1007346//Homo sapiens estrogen-responsive B box protein (EBBP) mRNA, complete cds.//3.7e-65:367:91//AF096870
R-PLACE1007367//Plasmodium falciparum 3D7 chromosome 12 PFYACB8-628 genomic sequence, WORKING DRAFT SEQUENCE, 9 unordered pieces.//1.0e-06:385:62//AC005507
R-PLACE1007375//Plasmodium falciparum 3D7 chromosome 12 PFYAC1122 genomic sequence, WORKING DRAFT SEQUENCE, 3 unordered pieces.//0.10:309:59//AC004709
R-PLACE1007386//Reclinomonas americana mitochondrial DNA, complete genome.//0.0012:403:58//AF007261
R-PLACE1007402//HS_2055_A2_D03_MR CIT Approved Human Genomic Sperm Library D Homo sapiens genomic clone Plate=2055 Col=6 Row=G, genomic survey sequence.//0.0046:88:79//AQ234824
R-PLACE1007409//Homo sapiens mitoxantrone resistance protein 1 mRNA, partial sequence.//7.6e-112:590:94//AF093771
R-PLACE1007416//Homo sapiens chromosome 19, cosmid R26894, complete sequence.//0.96:98:70//AC005594
R-PLACE1007450//Human DNA sequence *** SEQUENCING IN PROGRESS *** from clone 54B20, WORKING DRAFT SEQUENCE.//1.7e-39:308:82//Z98304
R-PLACE1007452//Homo sapiens PAC clone DJ0320J15 from Xq23, complete sequence.//2.6e-59:389:82//AC004081
R-PLACE1007460
R-PLACE1007478//Homo sapiens 12q13.1 PAC RPCI3-197B17 (Roswell Park Cancer Institute Human PAC library) complete sequence.//7.0e-08:335:60//AC004241
R-PLACE1007484
R-PLACE1007488//Glossina morsitans morsitans 16S ribosomal RNA gene, mitochondrial gene for mitochondrial RNA, partial sequence.//2.5e-05:421:61//AF072373
R-PLACE1007507//Plasmodium falciparum MAL3P7, complete sequence.//2.3e-09:577:57//AL034559
R-PLACE1007511//Homo sapiens chromosome 17, clone hRPC.1110_E_20, complete sequence.//1.2e-79:387:96//AC004231
R-PLACE1007524//Homo sapiens chromosome 19, overlapping cosmids F18547, F11133, R27945, R28830 and R32804, complete sequence.//3.4e-09:148:73//AC003682
R-PLACE1007525//Homo sapiens Chromosome 16 BAC clone CIT987SK-44M2, complete sequence.//4.7e-38:297:82//AC004381
R-PLACE1007544
R-PLACE1007547//Human laminin alpha 4 chain (LAMA4*-1) mRNA, complete cds.//4.0e-17:108:97//U77706
R-PLACE1007557//Human BAC clone RG343P13 from 7q31, complete sequence.//2.2e-45:390:77//AC002465
R-PLACE1007583//Human DNA sequence *** SEQUENCING IN PROGRESS *** from clone 545L17, WORKING DRAFT SEQUENCE.//1.0e-56:302:95//AL031665
R-PLACE1007598//Homo sapiens clone 23939 mRNA sequence.//1.5e-102:554:93//AF038179
R-PLACE1007618
R-PLACE1007621//Homo sapiens clone 23859 mRNA sequence.//1.4e-103:537:94//AF038176
R-PLACE1007632//High throughput sequencing of human chromosome 12, WORKING DRAFT SEQUENCE, 1 ordered pieces.//3.3e-76:289:94//AC005840
R-PLACE1007645//Homo sapiens full-length insert cDNA clone ZD76G10.//0.0080:96:77//AF086408
R-PLACE1007649//CIT-HSP-2308A18.TR CIT-HSP Homo sapiens genomic clone 2308A18, genomic survey sequence.//1.1e-82:412:97//AQ022149
R-PLACE1007677//Plasmodium falciparum chromosome 2, section 4 of 73 of the complete sequence.//0.0041:470:57//AE001367
R-PLACE1007688
R-PLACE1007690//Human Chromosome 16 BAC clone CIT987SK-A-418G10, complete sequence.//1.3e-22:162:91//AC002044
R-PLACE1007697
R-PLACE1007705//Human DNA sequence *** SEQUENCING IN PROGRESS *** from clone 460J8, WORKING DRAFT SEQUENCE.//4.4e-121:624:95//AL031662
R-PLACE1007706//Homo sapiens metalloprotease 1 (MP1) mRNA, complete cds.//1.8e-73:374:96//AF061243
R-PLACE1007725//Caenorhabditis elegans cosmid F38A5.//0.070:186:60//U70854
R-PLACE1007729//Human endogenous retrovirus HERV-K(HML6) proviral clone HML6.17 putative polymerase and envelope genes, partial cds, and 3'LTR.//3.8e-53:415:81//U60269
R-PLACE1007730//Homo sapiens mRNA for KIAA0685 protein, complete cds.//2.1e-92:556:89//AB014585
R-PLACE1007737//Homo sapiens clone Sb19.12 Alu-Yb8 sequence.//4.0e-43:302:77//AF015169
R-PLACE1007743//Plasmodium falciparum MAL3P8, complete sequence.//1.0e-06:533:59//AL034560
R-PLACE1007746//T.brucei mitochondrial maxicircle DNA encoding cytochrome c oxidase subunit I (COI), and NADH dehydrogenase subunits 4 and 5, complete cds.//0.28:386:58//M14820
R-PLACE1007791//D.discoideum gene for protein kinase.//0.17:263:60//Z37981
R-PLACE1007807//Human DNA sequence from clone 878O8 on chromosome Xq21.1-21.33. Contains an EST, STSs, a GSS and genomic marker DXS472, complete sequence.//1.1e-72:324:88//AL031116
R-PLACE1007810//Homo sapiens chromosome 7 common fragile site, complete sequence.//2.2e-14:325:67//AF017104
R-PLACE1007829//Human BAC clone GS165I04 from 7q21, complete sequence.//0.00052:455:61//AC002379
R-PLACE1007843//P.falciparum complete gene map of plastid-like DNA (IR-A).//0.0050:447:57//X95275
R-PLACE1007846//Homo sapiens genomic DNA, chromosome 21q22.2 (Down Syndrome region), segment 3/15, WORKING DRAFT SEQUENCE.//2.2e-111:570:95//AP000010
R-PLACE1007852//HS_3028_B2_F04_MR CIT Approved Human Genomic Sperm Library D Homo sapiens genomic clone Plate=3028 Col=8 Row=L, genomic survey sequence.//1.3e-12:209:71//AQ131021
R-PLACE1007858//Homo sapiens mRNA for KIAA0766 protein, complete cds.//6.6e-110:574:94//AB018309
R-PLACE1007866//Homo sapiens DNA sequence from PAC 454M7 on chromosome Xq25-26.3. Contains the OCRL1 gene for Lowe Oculocerebrorenal Syndrome protein OCRL-1. Contains ESTs, STSs and GSSs, complete sequence.//1.6e-43:551:70//AL022162
R-PLACE1007877//Homo sapiens chromosome 5, BAC clone 34j15 (LBNL H169), complete sequence.//1.6e-22:222:78//AC005754
R-PLACE1007897//HS_3113_B2_E04_T7 CIT Approved Human Genomic Sperm Library D Homo sapiens genomic clone Plate=3113 Col=8 Row=J, genomic survey sequencer.//2.9e-72:381:95//AQ186905
R-PLACE1007908//Homo sapiens mRNA, chromosome 1 specific transcript KIAA0487.//8.8e-88:460:95//AB007956
R-PLACE1007946//Human chromosome Y cosmid 54E8 genomic sequence, WORKING DRAFT SEQUENCE.//4.9e-23:172:78//AC003095
R-PLACE1007954//Homo sapiens BAC clone NH0414C23 from Y, complete sequence.//1.7e-27:303:75//AC006157
R-PLACE1007955//Homo sapiens cyclin-D binding Myb-like protein mRNA, complete cds.//3.9e-102:513:95//AF084530
R-PLACE1007958//Homo sapiens cAMP-specific phosphodiesterase 8B (PDE8B) mRNA, partial cds.//2.2e-87:465:93//AF079529
R-PLACE1007969//Mus musculus myelin gene expression factor (MEF-2) mRNA, partial cds.//4.8e-72:556:81//U13262
R-PLACE1007990//E.tenella antigen LPMC61 mRNA, partial cds.//0.043:273:63//M30933
R-PLACE1008000//Human DNA sequence *** SEQUENCING IN PROGRESS *** from clone 668J24, WORKING DRAFT SEQUENCE.//8.8e-10:453:62//AL034346
R-PLACE1008002//Homo sapiens clone DJ0613C23, WORKING DRAFT SEQUENCE, 4 unordered pieces.//9.0e-114:563:96//AC005628
R-PLACE1008044//Rattus norvegicus nuclear pore complex protein NUP107 mRNA, complete cds.//2.6e-44:509:72//L31840
R-PLACE1008045//Homo sapiens chromosome 5, BAC clone 79a6 (LBNL H172), complete sequence.//0.32:137:66//AC005592
R-PLACE1008080//Arabidopsis thaliana chromosome II BAC F10A12 genomic sequence, complete sequence.//0.082:292:59//AC006232
R-PLACE1008095//Homo sapiens BAC clone NH0364H22 from 2, complete sequence.//5.4e-27:260:76//AC005036
R-PLACE1008111//Human variable number tandem repeat (VNTR) region, allele 12R1 3' to collagen type II (COL2A1) gene.//2.2e-07:444:59//L10157
R-PLACE1008122//Homo sapiens chromosome 17, clone hRPK.142_H_19, complete sequence.//1.9e-11:384:63//AC005919
R-PLACE1008129//Homo sapiens clone DJ1087M19, WORKING DRAFT SEQUENCE, 7 unordered pieces.//3.0e-10:189:66//AC004955
R-PLACE1008132//Human HepG2 3' region cDNA, clone hmd5d06.//7.4e-47:320:86//D16939
R-PLACE1008177//Mouse mRNA for meiosis-specific nuclear structural protein 1 (MNS1), complete cds.//2.6e-32:410:70//D14849
R-PLACE1008181//Caenorhabditis elegans cosmid C31H2.//0.055:358:60//U41748
R-PLACE1008198
R-nnnnnnnnnnnn//Homo sapiens mRNA for KIAA0530 protein, partial cds.//4.8e-103:551:93//AB011102
R-PLACE1008209//Human DNA sequence *** SEQUENCING IN PROGRESS *** from clone 1125A11, WORKING DRAFT SEQUENCE.//4.6e-16:250:71//AL034549
R-PLACE1008231//Plasmodium falciparum 3D7 chromosome 12 PFYAC69 genomic sequence, WORKING DRAFT SEQUENCE, 4 unordered pieces.//0.13:341:60//AC004688
R-PLACE1008244//P.falciparum P.195 gene.//0.11:212:66//A04562
R-PLACE1008273//Human MEST mRNA, complete cds.//0.00013:52:100//D78611
R-nnnnnnnnnnnn
R-PLACE1008280//Homo sapiens chromosome 7 clone UWGC:g3586a160 from 7p14-15, complete sequence.//1.5e-05:104:76//AC005272
R-PLACE1008309//Human 'at'-rich region adjacent to alpha satellite DNA.//0.70:138:63//M80308
R-PLACE1008329//Homo sapiens chromosome 10 clone CIT-HSP-1240G16 map 10q25.1, complete sequence.//0.00061:150:68//AC005886
R-PLACE1008330//Homo sapiens chromosome 19, cosmid F21431, complete sequence.//4.8e-74:252:98//AC005176
R-PLACE100833//Genomic sequence from Human 13, complete sequence.//1.0:176:65//AC001226
R-PLACE1008356//Homo sapiens meningioma-expressed antigen 5 (MEA5) mRNA, 3' UTR.//2.5e-98:556:90//AF036145
R-PLACE1008368//HS-1039-A1-C10-MF.abi CIT Human Genomic Sperm Library C Homo sapiens genomic clone Plate=CT 821 Col=19 Row=E, genomic survey sequence.//1.2e-05:375:62//B36336
R-PLACE1008369//Homo sapiens genomic DNA, chromosome 21q22.2 (Down Syndrome region), segment 4/15, WORKING DRAFT SEQUENCE.//2.8e-10:466:61//AP000011
R-PLACE1008392//Homo sapiens chromosome 17, clone hRPK.471_L_13, complete sequence.//1.0e-46:282:82//AC005244
R-PLACE1008398//Human DNA sequence *** SEQUENCING IN PROGRESS *** from clone 215D11, WORKING DRAFT SEQUENCE.//4.1e-101:529:94//AL034417
R-PLACE1008401//*** SEQUENCING IN PROGRESS *** Homo sapiens chromosome 4, BAC clone C0366H07; HTGS phase 1, WORKING DRAFT SEQUENCE, 28 unordered pieces.//0.18:379:58//AC004604
R-nnnmnnnnnnn//Homo sapiens mRNA for p115, complete cds.//1.6e-101:521:95//D86326
R-PLACE1008405//Human cosmidCRI-JC2015 at D10S289 in 10sp13.//6.8e-22:328:71//U15177
R-PLACE1008424
R-PLACE1008426//Homo sapiens genomic DNA of 8p21.3-p22 anti-oncogene of hepatocellular colorectal and non-small cell lung cancer, segment 7/11.//7.5e-101:505:96//AB020864
R-PLACE1008429//Human DNA sequence from clone 20J23 on chromosome Xq26.2-27.2 Contains ras-related C3 botulinum toxin substrate 1 (P21-RAC1) (ras-like protein TC25) EST, CA repeat, STS, CpG island, complete sequence.//1.2e-11:118:78//AL022576
R-PLACE1008437//H.sapiens genomic DNA (PAC 838L14) from chromosome 11, WORKING DRAFT SEQUENCE.//2.2e-06:159:69//Y12335
R-PLACE1008455
R-PLACE1008457//Homo sapiens chromosome 17, Neurofibromatosis 1 locus, complete sequence.//1.2e-109:588:93//AC004526
R-PLACE1008465//CIT978SK-A-28A11.TVE CIT978SK Homo sapiens genomic clone A-28A11, genomic survey sequence.//1.1e-10:133:77//B78696
R-PLACE1008488
R-PLACE1008524//Human DNA sequence *** SEQUENCING IN PROGRESS *** from clone 34B21, WORKING DRAFT SEQUENCE.//7.3e-120:612:95//AL031778
R-PLACE1008531//Homo sapiens wbscr1 (WBSCR1) and replication factor C subunit 2 (RFC2) genes, complete cds.//8.5e-96:510:93//AF045555
R-PLACE1008532
R-PLACE1008533
R-PLACE1008568//HS_3218_B2_D08_T7 CIT Approved Human Genomic Sperm Library D Homo sapiens genomic clone Plate=3218 Col=16 Row=H, genomic survey sequence.//0.0042:295:62//AQ214623
R-PLACE1008584//Human PAC clone DJ0596009 from 7p15, complete sequence.//5.0e-26:254:66//AC003074
R-PLACE1008621//Homo sapiens chromosome 17, clone hRPK.346_K_10, complete sequence.//4.0e-78:498:86//AC006120
R-nnnnnnnnnnnn
R-PLACE1008626//Human DNA sequence *** SEQUENCING IN PROGRESS *** from clone 97P20, WORKING DRAFT SEQUENCE.//5.5e-06:228:67//AL031297
R-PLACE1008627//Cricetulus griseus mRNA for Zn finger factor.//3.4e-20:335:71//Y12836
R-PLACE1008629//Homo sapiens clone DJ0309D19, WORKING DRAFT SEQUENCE, 12 unordered pieces.//0.55:326:58//AC004826
R-PLACE1008630//Homo sapiens genomic DNA, 21q region, clone: B175P11X96, genomic survey sequence.//0.13:440:55//AG011096
R-PLACE1008643//Human BAC clone RG083J23 from 7q31, complete sequence.//1.3e-58:356:82//AC004001
R-PLACE1008650//Homo sapiens pleiotropic regulator 1 (PLRG1) mRNA, complete cds.//2.4e-88:434:97//AF044333
R-PLACE1008693//CIT-HSP-2025M9.TR CIT-HSP Homo sapiens genomic clone 2025M9, genomic survey sequence.//1.2e-41:300:82//B64742
R-PLACE1008696//Homo sapiens NADH dehydrogenase-ubiquinone Fe-S protein 8 23 kDa subunit (NDUFS8) gene, nuclear gene encoding mitochondrial protein, complete cds.//4.8e-31:320:75//AF038406
R-PLACE1008715//Human DNA sequence *** SEQUENCING IN PROGRESS *** from clone 799N4, WORKING DRAFT SEQUENCE.//0.074:478:58//AL022147
R-PLACE1008748//CIT-HSP-2170P12.TR CIT-HSP.Homo sapiens genomic clone 2170P12, genomic survey sequence.//8.5e-42:160:86//B90841
R-PLACE1008757//Homo sapiens 12q24.2 PAC RPCI4-765H13 (Roswell Park Cancer Institute Human PAC Library) complete sequence.//0.99:211:61//AC005864
R-PLACE1008790//Rattus norvegicus clone1 polymeric immunoglobulin receptor mRNA 3' untranslated region, GA rich region, and microsatellites with GGA-triplet and GAA-triplet repeats.//0.052:108:68//U00762
R-PLACE1008798//Homo sapiens full-length insert cDNA clone YZ86C05.//7.7e-58:285:100//AF086088
R-PLACE1008807//CIT-HSP-2366014.TR CIT-HSP Homo sapiens genomic clone 2366O14, genomic survey sequence.//3.5e-35:223:89//AQ079210
R-PLACE1008808//Homo sapiens exonuclease homolog RAD1 (RAD1) mRNA, complete cds.//2.3e-97:499:95//AF030933
R-PLACE1008813//Rattus norvegicus rsec15 mRNA, complete cds.//9.7e-45:394:78//AF032668
R-PLACE1008851//Human Chromosome 15q26.1 PAC clone pDJ460g16, WORKING DRAFT SEQUENCE, 3 unordered pieces.//2.9e-28:207:87//AC004581
R-nnnnnnnnnnnn//CIT-HSP-2172B3.TF CIT-HSP Homo sapiens genomic clone 2172B3, genomic survey sequence.//8.9e-30:166:97//B93289
R-PLACE1008867//Homo sapiens BAC clone RG054D04 from 7q31, complete sequence.//3.5e-76:404:95//AC005058
R-PLACE1008887//Homo sapiens clone DJ0943F02, WORKING DRAFT SEQUENCE, 3 unordered pieces.//7.7e-37:585:67//AC004932
R-PLACE1008902//Homo sapiens chromosome Y, clone hCIT.494_G_17, complete sequence.//0.0022:409:60//AC005820
R-PLACE1008920//Homo sapiens mRNA for KIAA0765 protein, partial cds.//8.2e-55:344:89//AB018308
R-PLACE1008925//Caenorhabditis elegans DNA *** SEQUENCING IN PROGRESS *** from clone Y53F4, WORKING DRAFT SEQUENCE.//0.0014:398:58//Z92860
R-PLACE1008934
R-PLACE1008941//Homo sapiens chromosome 17, clone hRPK.293_K_20, complete sequence.//9.8e-84:429:92//AC005495
R-PLACE1008947
R-PLACE1009020
R-PLACE1009027//Human DNA sequence from clone 914P14 on chromosome Xq23 Contains calpain-like protease gene, DCX (doublecortin) ESTs, CA repeat, GSS, complete sequence.//1.3e-82:434:95//AL031117
R-PLACE1009039//Human DNA sequence from clone 276K20 on chromosome 6p22.1-22.3. Contains STSs, GSSs and a putative CpG island, complete sequence.//0.00010:297:58//AL031391
R-PLACE1009045//Homo sapiens chromosome 17, clone hRPC.117_B_12, complete sequence.//2.9e-06:160:70//AC004707
R-PLACE1009048//Human DNA sequence from clone 511E16 on chromosome 6p24.3-25.1. Contains the last coding exon of the gene for P18 component of aminoacyl-tRNA synthetase complex, part of an unknown gene downstream of a putative CpG island, and an STS with a CA repeat polymorphism, complete sequence.//1.3e-16:339:66//AL023694
R-PLACE1009050//Aedes aegypti gene sequence, primary transcript.//0.40:393:59//L17023
R-PLACE1009060//Mus musculus mRNA for Alix-SF (ALG-2-interacting protein X, short form, complete CDS.//0.00075:79:83//AJ005074
R-PLACE1009090//Homo sapiens chromosome 1, BAC CIT-HSP-292g8 (BC262482), complete sequence.//6.7e-13:212:73//AC004783
R-PLACE1009094//Caenorhabditis elegans cosmid C49F8, complete sequence.//0.49:221:61//Z70206
R-PLACE1009099
R-PLACE1009110//Homo sapiens Xp22 BAC GS-321G17 (Genome Systems Human BAC library) complete sequence.//5.1e-17:301:66//AC004025
R-PLACE1009111//Plasmodium falciparum 3D7 chromosome 12 PFYACB8-420 genomic sequence, WORKING DRAFT SEQUENCE, 14 unordered pieces.//1.2e-06:234:61//AC005140
R-PLACE1009130//Plasmodium falciparum MAL3P6, complete sequence.//7.5e-06:426:58//Z98551
R-PLACE1009150//Homo sapiens *** SEQUENCING IN PROGRESS ***, WORKING DRAFT SEQUENCE.//2.3e-118:614:95//AJ011929
R-PLACE1009155//Homo sapiens genomic DNA, chromosome 21q11.1, segment 2/28, WORKING DRAFT SEQUENCE.//1.4e-107:584:93//AP000031
R-PLACE1009158//Homo sapiens full-length insert cDNA clone YP10D03.//1.9e-105:539:95//AF085876
R-PLACE1009166//Homo sapiens chromosome 17, clone hRPK.180_P_8, complete sequence.//2.8e-44:360:71//AC005972
R-PLACE1009172//Human cosmid QLL2C9 from Xq28.//4.1e-37:401:74//Z47046
R-PLACE1009174//Homo sapiens PAC clone DJ0907C10 from 7q31-3q32, complete sequence.//2.1e-17:140:81//AC004925
R-PLACE1009183//Homo sapiens DNA sequence from PAC 418A9 on chromosome 6q21. Contains the first (5') two exons of a CDK8 (Cell Division Protein Kinase 8) LIKE gene, a Neutral Calponin LIKE pseudogene, ESTs and STSs, complete sequence.//1.9e-46:572:69//Z84480
R-PLACE1009186//Human Chromosome X, complete sequence.//0.016:322:61//AC004070
R-PLACE1009190//Plasmodium falciparum MAL3P8, complete sequence.//0.050:487:58//AL034560
R-PLACE1009200//H.sapiens mRNA for sortilin.//1.0e-31:195:92//X98248
R-PLACE1009230//Homo sapiens chromosome 19, CIT-HSP BAC 490g23 (BC338531), complete sequence.//1.8e-75:364:85//AC005392
R-PLACE1009246//Cricetulus griseus SRD-2 mutant sterol regulatory element binding protein-2 (SREBP-2) mRNA, complete cds.//6.6e-44:525:71//U22818
R-PLACE1009308
R-PLACE1009319//Homo sapiens 12q13.1 PAC RPCI1-228P16 (Roswell Park Cancer Institute Human PAC Library) complete sequence.//0.00010:132:75//AC004801
R-PLACE1009328//Homo sapiens chromosome 17, clone hRPK.346_K_10, complete sequence.//3.3e-87:576:85//AC006120
R-PLACE1009335//Borrelia burgdorferi (section 62 of 70) of the complete genome.//0.32:315:60//AE001176
R-PLACE1009338//Plasmodium falciparum 3D7 chromosome 12 PFYACB8-420 genomic sequence, WORKING DRAFT SEQUENCE, 14 unordered pieces.//6.8e-05:411:59//AC005140
R-PLACE1009368//Homo sapiens PAC clone DJ1179J19 from 7q11.23-q21, complete sequence.//0.00040:280:61//AC004989
R-PLACE1009375//D. yakuba mitochondrial DNA for origin of replication, small ribosomal RNA , transfer RNAs tRNA-fMet, tRNA-GIn, tRNA-Ile and tRNA-Val.//1.1e-08:444:60//X05915
R-PLACE1009388
R-PLACE1009398//Homo sapiens BAC clone GS011E15 from 5q31, complete sequencer.//0.065:279:61//AC002427
R-nnnnnnnnnnnn//Homo sapiens clone NH0486I22, WORKING DRAFT SEQUENCE, 5 unordered pieces.//1.0e-06:253:64//AC005038
R-PLACE1009410//Homo sapiens chromosome 17, clone hRPK.142_H_19, complete sequence.//9.8e-112:561:96//AC005919
R-PLACE1009434//Human DNA sequence from clone 459L4 on chromosome 6p22.3-24.1 Contains EST, STS, GSS, complete sequence.//2.2e-21:126:79//AL031120 R-PLACE1009443//Homo sapiens nucleolar protein Nop30 and cytoplasmic protein Myp (NOP) gene, alternatively spliced products, complete cds.//4.5e-14:117:91//AF064598
R-PLACE1009444//Homo sapiens phosphatidylinositol 4-kinase mRNA, complete cds.//9.6e-85:479:90//L36151
R-PLACE1009459
R-PLACE1009476//Homo sapiens Chromosome 16 BAC clone CIT987SK-A-67A1, complete sequence.//5.6e-101:540:94//AC004531
R-PLACE1009477//Homo sapiens, clone hRPK.15_A_1, complete sequence.//3.4e-46:284:91//AC006213
R-PLACE1009493//Human Chromosome 16 BAC clone CIT987SK-A-363E6, complete sequence.//5.5e-107:581:92//U91321
R-PLACE1009524//Homo sapiens DNA sequence from PAC 63G5 on chromosome-22q12.3-13.1. Contains part of a gene for a human SEC7 homolog B2-1 (cytohesin-2, Arno, ARF exchange factor) LIKE protein, an unknown gene and a gene coding for a Leucine rich protein. Contains ESTs, STSs and GSSs, complete sequence.//0.74:301:61//Z94160
R-PLACE1009539//Human DNA sequence *** SEQUENCING IN PROGRESS *** from clone 167A19, WORKING DRAFT SEQUENCE.//5.7e-29:357:74//AL031427
R-PLACE1009542//CIT-HSP-2166P10.TRB CIT-HSP Homo sapiens genomic clone 2166P10, genomic survey sequence.//2.6e-10:145:75//B89614
R-PLACE1009571//RPCI11-61J16.TK RPCI11 Homo sapiens genomic clone R-61J16, genomic survey sequence.//0.016:68:80//AQ202146
R-PLACE1009581
R-PLACE1009595//Homo sapiens clone DJ56J10, complete sequence.//1.8e-38:365:79//AC005006
R-PLACE1009596//Human DNA sequence *** SEQUENCING IN PROGRESS *** from clone 501A4, WORKING DRAFT SEQUENCE.//1.2e-29:314:76//Z98051
R-PLACE1009607//cSRL-77g9-u cSRL flow sorted Chromosome 11 specific cosmid Homo sapiens genomic clone cSRL-77g9, genomic survey sequence.//2.1e-05:142:69//B06230
R-PLACE1009613//Plasmodium falciparum DNA *** SEQUENCING IN PROGRESS *** from contig 4-89, complete sequence.//3.6e-08:434:59//AL010266
R-PLACE1009621//Sequence 50 from patent US 5691147.//1.5e-20:235:73//I76222
R-PLACE1009622//CIT-HSP-2023D13.TFB CIT-HSP Homo sapiens genomic clone 2023D13, genomic survey sequence.//0.72:176:62//B81271
R-PLACE1009637//P.falciparum complete gene map of plastid-like DNA (IR-B).//0.0068:396:59//X95276
R-PLACE1009639//Arabidopsis thaliana DNA chromosome 4, BAC clone F10M6 (ESSAII project).//0.013:521:58//AL021811
R-PLACE1009659//Homo sapiens mRNA for KIAA0587 protein, complete cds.//1.0e-107:589:92//AB011159
R-PLACE1009665//Human PAC clone DJ0658N05 from 7p21, complete sequence.//8.4e-72:487:85//AC003075
R-PLACE1009670//Homo sapiens genethonin 1 mRNA, complete cds.//2.0e-61:310:97//AF062534
R-PLACE1009708//Homo sapiens clone DJ0935K16, complete sequence.//2.8e-103:542:94//AC006011
R-PLACE1009721//Human Cosmid g0771a222 from 7q31.3, complete sequence.//4.6e-85:518:88//AC000109
R-PLACE1009731//Homo sapiens DNA sequence from PAC 434O14 on chromosome 1q32.3.-41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two novel genes. Contains ESTs and GSSs, complete sequence.//0.0033:215:65//AL022398
R-PLACE1009763//Homo sapiens UBA3 (UBA3) mRNA, complete cds.//6.2e-116:598:95//AF046024
R-PLACE1009794
R-nnnnnnnnnnnn//Human DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs, an STS and GSSs, complete sequence.//7.5e-88:191:96//AL030996
R-PLACE1009845//Homo sapiens DNA sequence from PAC 234H5 on chromosome 6q21. Contains an unknown gene, ESTs and STSs, complete sequence.//8.7e-19:226:69//Z98172
R-PLACE1009879//Homo sapiens genomic DNA, 21q region, clone: 149C3A68, genomic survey sequence.//2.1e-29:230:76//AG002672
R-PLACE1009886//Homo sapiens PAC clone DJ0997N05 from 7q11.23-q21.1, complete sequence.//0.99:203:61//AC004945
R-PLACE1009888//Homo sapiens chromosome 19, BAC CIT-B-393i15 (BC301323), complete sequence.//5.3e-91:577:88//AC006116
R-nnnnnnnnnnnn
R-PLACE1009921//Homo sapiens cosmid clone HDAB (1S149) insert DNA, complete cosmid.//4.7e-81:385:84//M63005
R-PLACE1009924//HS_3151_B1_B10_MR CIT Approved Human Genomic Sperm Library D Homo sapiens genomic clone Plate=3151 Col=19 Row=D, genomic survey sequence.//5.5e-47:240:99//AQ167412
R-PLACE1009925//CIT978SK-A-931F6.TV CIT978SK Homo sapiens genomic clone A-931F6, genomic survey sequence.//0.00010:159:68//B51673
R-PLACE1009935//Plasmodium falciparum 3D7 chromosome 12 PFYAC492 genomic sequence, WORKING DRAFT SEQUENCE, 5 unordered pieces.//0.081:238:65//AC005308
R-PLACE1009947//Homo sapiens PAC clone 248015 from 13q12-q13, complete sequence.//1.0:353:58//AC002483
R-PLACE1009971//Homo sapiens full-length insert cDNA clone ZD38E12.//3.7e-11:152:75//AF086247
R-PLACE1009992
R-PLACE1009995//Plasmodium falciparum chromosome 2, section 4 of 73 of the complete sequence.//0.0019:305:61//AE001367
R-PLACE1009997//Homo sapiens chromosome 10 clone CIT987SK-1175G20 map 10q25.2-10q25.3, complete sequence.//1.8e-43:462:76//AC005874
R-PLACE1010023//HS_30l8_B1_H10_T7 CIT Approved Human Genomic Sperm Library D Homo sapiens genomic clone Plate=3018 Col=19 Row=P, genomic survey sequence.//0.00013:198:63//AQ093513
R-PLACE1010031//Human DNA sequence from clone 30M3 on chromosome 6p22.1-22.3. Contains three novel genes, one similar to C. elegans Y63D3A.4 and one similar to (predicted) plant, worm, yeast and archaea bacterial genes, and the first exon of the KIAA0319 gene. Contains ESTs, GSSs and putative CpG islands, complete sequence.//7.4e-115:581:96//AL031775
R-PLACE1010053//M.musculus Spnr mRNA for RNA binding protein.//1.9e-05:136:74//X84692
R-PLACE1010069//CIT-HSP-2328B12.TF CIT-HSP Homo sapiens genomic clone 2328B12, genomic survey sequence.//2.6e-60:324:94//AQ042094
R-PLACE1010074//Homo sapiens sorting nexin 2 (SNX2) mRNA, complete cds.//4.6e-87:543:88//AF065482
R-PLACE1010076//*** SEQUENCING IN PROGRESS *** Homo sapiens chromosome 4, BAC clone C0473M13; HTGS phase 1, WORKING DRAFT SEQUENCE, 4 unordered pieces.//6.3e-08:489:58//AC005699
R-PLACE1010083
R-PLACE1010089//F19F22-Sp6 IGF Arabidopsis thaliana genomic clone F19F22, genomic survey sequence.//0.14:400:59/B10583
R-PLACE1010096//R.norvegicus mRNA for 100 kDa protein.//4.3e-91:562:87//X64411
R-PLACE1010102//Apis mellifera tRNA-Leu cytochrome oxidase II intergenic spacer region, mitochondrial sequence.//1.5e-08:357:60//AF039556
R-PLACE1010105//Plasmodium falciparum chromosome 2, section 11 of 73 of the complete sequence.//4.0e-09:510:59//AE001374
R-PLACE1010106//Human DNA sequence *** SEQUENCING IN PROGRESS *** from clone 54B20, WORKING DRAFT SEQUENCE.//1.4e-12:194:73//Z98304
R-PLACE1010134
R-PLACE1010148//HS_3128_A1_D09_MR CIT Approved Human Genomic Sperm Library D Homo sapiens genomic clone Plate=3128 Col=17 Row=G, genomic survey sequence.//0.17:281:61//AQ140790
R-PLACE1010152//Mouse mRNA for arylhydrocarbon receptor, complete cds.//3.1e-45:351:81//D38417
R-PLACE1010181//Homo sapiens clone DJ0914M06, WORKING DRAFT SEQUENCE, 1 unordered pieces.//3.6e-06:207:66//AC004928
R-PLACE101019411HS_2232_B1_H10_MR CIT Approved Human Genomic Sperm Library D. Homo sapiens genomic clone Plate=2232 Col=19 Row=P, genomic survey sequence.//2.4e-08:134:74//AQ185425
R-PLACE1010202//Human DNA sequence from clone 227L5 on chromosome Xp11.22-11.3. Contains a Keratin, Type 1 Cytoskeletal 18 (KRT18, CYK18, K18, CK18) pseudogene and an STS, complete sequence.//0.00035:383:61//AL031585
R-PLACE1010231//Human DNA sequence *** SEQUENCING IN PROGRESS *** from clone 287G14, WORKING DRAFT SEQUENCE.//1.2e-95:519:94//AL033377
R-PLACE1010261
R-PLACE1010270//H.sapiens CpG island DNA genomic Mse1 fragment, clone 85a6, reverse read cpg85a61rt1a.//0.068:171:63//Z63482
R-PLACE1010274//S.douglasii gene for cytochrome b.//4.5e-07:276:63//X59280
R-PLACE1010293//Homo sapiens chromosome 2 PAC RPCI3-417E16 (Roswell Park Cancer Institute Human PAC library) complete sequence.//4.7e-91:522:90//AC004464
R-PLACE1010321
R-PLACE1010324//Caenorhabditis elegans DNA *** SEQUENCING IN PROGRESS *** from clone Y38E10, WORKING DRAFT SEQUENCE.//5.7e-08:484:57//AL021149
R-PLACE1010329//Homo sapiens Chromosome 22q11.2 Cosmid Clone 50d10 In IGLC Region, complete sequence.//7.9e-35:328:79//AC000024
R-PLACE1010341//Homo sapiens clone DJ1125K23, WORKING DRAFT SEQUENCE, 21 unordered pieces.//1.3e-31:418:66//AC004971
R-PLACE1010362
R-PLACE1010364//Mus cookii mitochondrion DNA fragment.//0.23:162:64//M77098
R-PLACE1010383//Homo sapiens chromosome 17, clone hCIT.186_H_2, complete sequence.//1.4e-105:543:95//AC004675
R-PLACE1010401//Human Chromosome 15q11-q13 PAC clone pDJ223c9 from the Prader-Willi/Angelman Syndrome region, complete sequence.//0.00017:268:62//AC004137
R-PLACE1010481//Bos taurus C5-glucuronyl epimerase mRNA, partial cds.//8.6e-79:556:83//AF003927
R-PLACE1010491//Homo sapiens Cre binding protein-like 2 mRNA, complete cds.//7.3e-88:438:96//AF039081
R-PLACE1010492//HS_3169_B2_B04_T7 CIT Approved Human Genomic Sperm Library D Homo sapiens genomic clone Plate=3169 Col=8 Row=D, genomic survey sequence.//0.98:171:63//AQ169892
R-PLACE1010522//WORKING DRAFT SEQUENCE, 6 unordered pieces.//0.34:407:62//AC006082
R-nnnnnnnnnnnn
R-PLACE1010562//CITBI-E1-2503B16.TF CITBI-E1 Homo sapiens genomic clone 2503B16, genomic survey sequence.//6.4e-17:152:84//AQ265929
R-PLACE1010579//Torulopsis glabrata mitochondrial gene for ribosomal protein varl.//1.7e-05:271:65//X02893
R-PLACE1010580
R-PLACE1010599
R-PLACE1010616//Human BAC clone RG343P13 from 7q31, complete sequence.//3.0e-13:151:75//AC002465
R-PLACE1010622//Arabidopsis thaliana BAC F1104.//0.00031:366:60//AF096370
R-PLACE1010624//Homo sapiens chromosome 7q22 sequence, complete sequence.//8.2e-34:322:79//AF053356
R-PLACE1010628//Homo sapiens clone DJ0647C14, WORKING DRAFT SEQUENCE, 21 unordered pieces.//2.3e-97:515:94//AC004846
R-PLACE1010629//HS_3003_A2_C08_MR CIT Approved Human Genomic Sperm Library D Homo sapiens genomic clone Plate=3003 Col=16 Row=E, genomic survey sequence.//5.6e-60:321:95//AQ130493
R-PLACE1010630//Plasmodium falciparum chromosome 2, section 19 of 73 of the complete sequence.//0.051:372:59//AE001382
R-PLACE1010631//Homo sapiens mRNA for KIAA0530 protein, partial cds.//2.6e-92:497:93//AB011102
R-PLACE1010661//CIT-HSP-2008K15.TR CIT-HSP Homo sapiens genomic clone 2008K15, genomic survey sequence.//5.7e-27:160:95//B57089
R-PLACE1010662//Caenorhabditis elegans cosmid C12C8, complete sequence.//9.4e-09:151:73//Z81467
R-PLACE1010702//CIT-HSP-2314C3.TR CIT-HSP Homo sapiens genomic clone 2314C3, genomic survey sequence.//1.3e-90:459:96//AQ028536
R-PLACE1010714//Saccharomyces douglasii mitochondrial tRNA-Ser and tRNA-Phe genes, partial sequence, and Var1p (var1) gene, mitochondrial gene encoding mitochondrial protein, complete cds.//5.3e-08:478:58//U49822
R-PLACE1010720//Homo sapiens chromosome-associated protein-C (hCAP-C) mRNA, partial cds.//3.8e-55:300:95//AF092564
R-PLACE1010739//Human DNA sequence from clone 393P23 on chromosome Xq21.1-21.33. Contains GSSs, complete sequence.//3.4e-89:507:90//Z95400
R-PLACE1010743
R-PLACE1010761//Homo sapiens chromosome 17, clone hRPK.294_J_22, complete sequence.//3.0e-103:511:97//AC005921
R-PLACE1010771
R-PLACE1010786
R-PLACE1010800//Homo sapiens clone NH0084K19, WORKING DRAFT SEQUENCE, 30 unordered pieces.//1.8e-43:545:71//AC005682
R-PLACE1010802//Phoebis agarithe large subunit ribosomal RNA gene, partial sequence; tRNA-Val gene, complete sequence; and small subunit ribosomal RNA gene, partial sequence, mitochondrial genes for mitochondrial RNAs.//1.9e-09:492:59//AF044862
R-PLACE1010811//Homo sapiens Xp22 BAC GSHB-257G1 (Genome Systems BAC Library) complete sequence.//0.041:415:59//AC002524
R-PLACE1010833
R-PLACE1010856//Plasmodium falciparum 3D7 chromosome 12 PFYAC812 genomic sequence, WORKING DRAFT SEQUENCE, 8 unordered pieces.//0.0022:512:55//AC004153
R-PLACE1010857//Homo sapiens genomic DNA of 8p21.3-p22 anti-oncogene of hepatocellular colorectal and non-small cell lung cancer , segment 11/11//4.9e-85:507:90//AB020868
R-PLACE1010870//RPCI11-59K21:TK RPCI11 Homo sapiens genomic clone R-59K21, genomic survey sequence.//8.2e-85:422:97//AQ195697
R-PLACE1010877//Homo sapiens mRNA for KIAA0610 protein, partial cds.//7.0e-100:501:96//AB011182
R-PLACE1010891//Homo sapiens chromosome X, clone 592, WORKING DRAFT SEQUENCE, 8 unordered pieces.//0.10:162:61//AC002489
R-PLACE1010896//Plasmodium falciparum 3D7 chromosome 12 PFYAC181 genomic sequence, WORKING DRAFT SEQUENCE, 8 unordered pieces.//0.00032:409:59//AC005505
R-PLACE1010900//Homo sapiens DNA, trinucleotide repeats region.//3.2e-07:180:71//AB018488
R-PLACE1010916//Plasmodium falciparum DNA *** SEQUENCING IN PROGRESS *** from MAL4P1, WORKING DRAFT SEQUENCE.//0.041:205:60//AL034557
R-PLACE1010917
R-PLACE1010925//HS_2027_B2_B09_T7 CIT Approved Human Genomic Sperm Library D Homo sapiens genomic clone Plate=2027 Col=18 Row=D, genomic survey sequence.//1.6e-46:404:77//AQ247031
R-PLACE1010926//Homo sapiens mRNA for KIAA0554 protein, partial cds.//4.2e-65:402:89//AB011126
R-nnnnnnnnnnnn//Homo sapiens intersectin short form mRNA, complete cds.//1.9e-80:441:93//AF064243
R-PLACE1010944
R-PLACE1010947//D.discoideum rasG gene.//0.00044:181:65//Z11533
R-PLACE1010954//Homo sapiens clone RG228D17, WORKING DRAFT SEQUENCE, 2 unordered pieces.//3.0e-51:518:74//AC005077
R-PLACE1010960//Human DNA sequence *** SEQUENCING IN PROGRESS *** from clone 451B21, WORKING DRAFT SEQUENCE.//0.022:292:63//AL033522
R-PLACE1010965//Human mariner1 transposase gene, complete consensus sequence.//1.0e-64:444:84//U52077
R-PLACE1011026//Rickettsia prowazekii strain Madrid E, complete genome; segment 3/4.//0.59:345:61//AJ235272
R-PLACE1011032//Human DNA sequence from PAC 389A20 on chromosome X contains ESTs STS, CpG islands and polymorphic CA repeat.//0.62:82:75//Z93242
R-PLACE1011041//H.sapiens DNA sequence.//0.051:162:66//Z22248
R-nnnnnnnnnnnn//Homo sapiens mRNA for KIAA0581 protein, partial cds.//2.9e-100:563:91//AB011153
R-PLACE1011054//Human DNA sequence from PAC 46H23, BRCA2 gene region chromosome 13q12-13 contains Klotho, ESTs.//4.7e-29:280:73//Z84483
R-PLACE1011056//Human DNA sequence *** SEQUENCING IN PROGRESS *** from clone 341D10, WORKING DRAFT SEQUENCE.//1.7e-39:288:84//Z97985
R-PLACE1011057//CIT-HSP-2014F10.TF CIT-HSP Homo sapiens genomic clone 2014F10, genomic survey sequence.//2.4e-60:370:90//B58896
R-PLACE1011090//Homo sapiens chromosome 4 clone B200N5 map 4q25, complete sequence.//0.12:489:59//AC005509
R-PLACE1011109//Homo sapiens chromosome Y, clone 486, O, 2, complete sequence.//8.4e-43:427:76//AC002531
R-PLACE1011114//Homo sapiens mRNA from HIV associated non-Hodgkin's lymphoma (clone hl1-14).//1.7e-29:179:94//Y16709
R-PLACE1011133//HS-1058-B1-H02-MF.abi CIT Human Genomic Sperm Library C Homo sapiens genomic clone Plate=CT 780 Col=3 Row=P, genomic survey sequence.//1.0:133:63//44006
R-PLACE1011143//H.sapiens CpG island DNA genomic Mse1 fragment, clone 127a4, forward read cpg127a4.ft1a.//1.0:127:67//Z56550
R-PLACE1011160//Homo sapiens HRIHFB2038 mRNA, partial cds.//2.4e-95:534:91//AB015333
R-PLACE1011165//Human Cosmid g5129s232 from 7q31.3, complete sequence.//0.47:355:58//AC003968
R-PLACE1011185//Homo sapiens clone DJ0038I10, WORKING DRAFT SEQUENCE, 5 unordered pieces.//1.3e-26:403:70//AC004820
R-PLACE1011203//paramecium species 4.51er mt dna dimer: replication init. region, clone 1.//1.0e-10:379:60//K00908
R-PLACE1011219//HS_3036_B1_F08_MR CIT Approved Human Genomic Sperm Library D Homo sapiens genomic clone Plate=3036 Col=15 Row=L, genomic survey sequence.//2.6e-39:253:88//AQ104587
R-PLACE1011221//Homo sapiens T-cell receptor alpha delta locus from bases 250472 to 501670 (section 2 of 5) of the Complete Nucleotide Sequence.//0.32:279:60//AE000659
R-PLACE1011229//HS_3002_B1_E10_MR CIT Approved Human Genomic Sperm Library D Homo sapiens genomic clone Plate=3002 Col=19 Row=J, genomic survey sequence.//9.3e-3l:317:74//AQ303626
R-PLACE1011263//Homo sapiens BAC clone GS166A23 from 7p21, complete sequence.//1.2e-109:571:94//AC005014
R-PLACE1011273//Saccharomyces douglasii mitochondrial cytochrome c oxidase subunit I (COXI) gene, complete cds.//0.00027:337:61//M97514
R-PLACE1011291
R-PLACE1011296//H.sapiens steroid reductase pseudogene.//4.2e-37:326:80//M68887
R-PLACE1011310//H.sapiens 5' flanking sequence of gene for corticotropin.//0.0017:416:60//X67661
R-PLACE1011325//Plasmodium falciparum chromosome 2, section 35 of 73 of the complete sequence.3.0e-10:511:59//AE001398
R-PLACE1011332//Homo sapiens chromosome 17, clone HCIT3L16, WORKING DRAFT SEQUENCE, 7 unordered pieces.//8.3e-06:250:64//AC002344
R-PLACE1011340//Human BAC clone RG341D10 from 7p15-p21, complete sequence.//0.67:290:58//AC002530
R-PLACE1011375//Mus musculus Kv3.4 gene, exon 4.//6.8e-23:190:86//AJ010310
R-PLACE1011399//Plasmodium falciparum 3D7 chromosome 12 PFYACB8-420 genomic sequence, WORKING DRAFT SEQUENCE, 14 unordered pieces.//0.22:359:60//AC005140
R-PLACE1011419//Human DNA sequence from cosmid U90B3, on chromosome Xp11, contains ESTs.//5.1e-32:282:81//Z74022
R-nnnnnnnnnnnn//Homo sapiens mRNA for KIAA0530 protein, partial cds.//1.5e-112:600:94//AB011102
R-PLACE1011452//Homo sapiens clone DJ0945F02, WORKING DRAFT SEQUENCE, 7 unordered pieces.//3.9e-77:303:85//AC006013
R-PLACE1011465
R-PLACE1011472//Homo sapiens mRNA for KIAA0712 protein, complete cds.//7.9e-103:515:96//AB018255
R-PLACE1011492//A-837A4.TP CIT978SK Homo sapiens genomic clone A-837A4, genomic survey sequence.//6.5e-37:234:82//B14085
R-PLACE1011503//Homo sapiens chromosome 17, clone hRPC.1171_I_10, complete sequence./0.99:267:60//AC004687
R-PLACE1011520//Homo sapiens clone DJ1119N05, complete sequence.//2.0e-116:591:96//AC004968
R-PLACE1011563//Plasmodium falciparum 3D7 chromosome 12 PFYAC69 genomic sequence, WORKING DRAFT SEQUENCE, 4 unordered pieces.//1.2e-13:566:59//AC004688
R-PLACE1011567//Plasmodium-falciparum MAL3P6, complete sequence.//0.62:358:61//Z98551
R-PLACE1011576//Homo sapiens DNA from chromosome 19, BAC 33152, complete sequence.//8.7e-45:400:78//AC003973
R-PLACE1011586//Homo sapiens chromosome 17, clone HRPC890E16, complete sequence.//2.2e-59:338:93//AC004477
R-PLACE1011635//C.pasteurianum pfl gene and act gene.//0.71:288:60//X93463
R-PLACE1011641//Mycoplasma genitalium random genomic clone sg11, partial cds.//0.023:232:60//U02205
R-PLACE1011643//Homo sapiens chromosome 19, cosmid R33590, complete sequence.//1.4e-21:432:67//AC005620
R-PLACE1011649//Homo sapiens clone 24432 mRNA sequence.//7.8e-72:414:91//AF070535
R-PLACE1011650//Human PAC clone DJ327A19 from Xq25-q26, complete sequence.//5.1e-27:174:79//AC002477
R-PLACE1011664//Human DNA sequence *** SEQUENCING IN PROGRESS *** from clone. 460D19, WORKING DRAFT SEQUENCE.//7.4e-05:238:65//AL031905
R-PLACE1011675//CIT-HSP-2370M16.TR CIT-HSP Homo sapiens genomic clone 2370M16, genomic survey sequence.//1.3e-27:233:81//AQ108283
R-PLACE1011682//H.sapiens HLA-DMB gene.//2.3e-22:390:67//X76776
R-PLACE1011719//Homo sapiens 12q24.2 BAC RPCI11-360E11 (Roswell Park Cancer Institute Human BAC Library) complete sequence.//3.1e-24:409:66//AC004806
R-PLACE1011725//Homo sapiens unknown mRNA downregulated by induced differentiation with 13-cis retinoic acid.//0.13:143:65//AF026526
R-PLACE1011729//Human DNA sequence *** SEQUENCING IN PROGRESS *** from clone Y738F9, WORKING DRAFT SEQUENCE.//1.1e-15:157:82//AL022345
R-PLACE1011749//Homo sapiens clone RG315H11, WORKING DRAFT SEQUENCE, 5 unordered pieces.//1.5e-38:314:81//AC005089
R-PLACE1011762//Homo sapiens BAC clone RG067E13 from 7q21, complete sequence.//1.9e-35:538:68//AC002383
R-PLACE1011778//CIT-HSP-2326C17.TV CIT-HSP Homo sapiens genomic clone 2326C17, genomic survey sequence.//2.8e-58:346:91//AQ028782
R-PLACE1011783//Human DNA sequence *** SEQUENCING IN PROGRESS *** from clone 229A8, WORKING DRAFT SEQUENCE.//4.6e-38:288:84//Z86090
R-PLACE1011858//Human DNA sequence from clone 496N17 on chromosome 6p11.2-12.3 Contains EST, GSS, complete sequence./4.1e-104:524:97//AL031321
R-PLACE1011874//Homo Sapiens Chromosome X clone bWXD312, complete sequence.//2.1e-100:511:95//AC004478
R-PLACE1011875
R-PLACE1011891//Human lens membrane protein (mp19) gene, exon 11.//0.0011:195:64//L04193
R-PLACE1011896//Homo sapiens DNA sequence from PAC 434014 on chromosome 1q32.3.-41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two novel genes. Contains ESTs and GSSs, complete sequence.//0.010:110:74//AL022398
R-PLACE1011922//Homo sapiens chromosome 21q22.3 PAC 171F15, complete sequence.//3.5e-10:152:74//AF042090
R-PLACE1011923//Homo sapiens serum-inducible kinase mRNA, complete cds.//7.0e-98:546:92//AF059617
R-PLACE1011962//CIT-HSP-2294L24.TF CIT-HSP Homo sapiens genomic clone 2294L24, genomic survey sequence.//0.31:131:63//AQ006352
R-PLACE1011964//Homo sapiens chromosome 17, clone HRPC987K16, complete sequence.//2.5e-08:393:63//AC002994
R-PLACE1011982//Arabidopsis thaliana genomic DNA, chromosome 3, P1 clone: MDJ14, complete sequence.//9.6e-09:463:62//AB016889
R-PLACE1011995//Human Down Syndrome region of chromosome 21, clone A12H1-2H4.//2.7e-39:294:82//U44738
R-PLACE1012031//Homo sapiens mRNA for KIAA0713 protein, partial cds.//2.5e-104:540:95//AB018256
R-PLACE2000003//Human PAC clone DJ404F18 from Xq23, complete sequence.//4.9e-10:439:63//AC004000
R-PLACE2000007//Human fibroblast growth factor receptor 3 (FGFR3) gene, intron 3.//1.0:151:66//L78722
R-PLACE2000011//Homo sapiens clone 614 unknown mRNA, complete sequence.//1.5e-103:524:95//AF091080
R-PLACE2000015//Homo sapiens PAC clone DJ269005 from Xq23, complete sequence.//0.94:372:57//AC005191
R-PLACE2000017//Homo sapiens chromosome 17, clone hCIT.162_E_12, complete sequence.//3.0e-55:299:86//AC006236
R-PLACE2000021//CIT-HSP-2343C18.TR CIT-HSP Homo sapiens genomic clone 2343C18, genomic survey sequence.//4.5e-54:295:94//AQ058140
R-PLACE2000033//H.sapiens gene for mitochondrial ATP synthase c subunit (P1 form).//6.5e-38:298:82//X69907
R-PLACE2000034//Homo sapiens clone DJ0613C23, WORKING DRAFT SEQUENCE, 4 unordered pieces.//5.3e-34:200:79//AC005628
R-PLACE2000039//Homo sapiens BAC clone RG060N22 from 7q21, complete sequence.//1.8e-49:274:89//AC003083
R-PLACE2000047//CIT-HSP-2373C2.TR CIT-HSP Homo sapiens genomic clone 2373C2, genomic survey sequence.//1.8e-48:389:79//AQ112243
R-PLACE2000050//Human DNA sequence *** SEQUENCING IN PROGRESS *** from clone 1177I5, WORKING DRAFT SEQUENCE.//0.0027:95:76//AL022315
R-PLACE2000061//Homo sapiens mRNA for KIAA0575 protein, complete cds.//2.9e-39:429:72//AB011147
R-PLACE2000062//Homo sapiens clone DJ0539M06, WORKING DRAFT SEQUENCE, 10 unordered pieces.//5.9e-40:310:84//AC004832
R-PLACE2000072//Homo sapiens ZNF202 beta (ZNF202) mRNA, complete cds.//1.9e-109:550:95//AF027219
R-PLACE2000097//Homo sapiens chromosome 12p13.3 clone RPCI11-189M20, WORKING DRAFT SEQUENCE, 39 unordered pieces.//1.6e-106:553:95//AC005910
R-PLACE2000100//Human DNA sequence from clone 301K23 on chromosome 1p35.1-36.21. Contains the 5' part of a novel gene similar to predicted yeast and worm genes. Contains ESTs and GSSs, complete sequence.//1.8e-38:285:84//AL031730
R-PLACE2000103//Human DNA sequence *** SEQUENCING IN PROGRESS *** from clone 20208, WORKING DRAFT SEQUENCE.//4.3e-113:559:97//AL031848
R-PLACE2000111//Rat MLC1V gene encoding alkali myosin ventricel light chain, exon 1.//0.00041:347:61//X16325
R-PLACE2000115//Cervus elaphus MHC class II DRB pseudogene, intron 2 microsatellite.//0.50:165:63//U63067
R-PLACE2000132
R-PLACE2000136//Plasmodium falciparum DNA *** SEQUENCING IN PROGRESS *** from contig 3-30, complete sequence.//0.0032:310:61//AL008974
R-PLACE2000140//Human DNA sequence *** SEQUENCING IN PROGRESS *** from clone 11703, WORKING DRAFT SEQUENCE.//1.1e-111:566:96//AL020995
R-PLACE2000164
R-PLACE2000170//*** SEQUENCING IN PROGRESS *** Homo sapiens chromosome 4, BAC clone C0024K08; HTGS phase 1, WORKING DRAFT SEQUENCE, 5 unordered pieces.//3.9e-40:390:76//AC005598
R-PLACE2000172
R-PLACE2000176
R-PLACE2000187//Human DNA sequence *** SEQUENCING IN PROGRESS *** from clone 268H5, WORKING DRAFT SEQUENCE.//8.7e-45:298:87//AL008718
R-PLACE2000216//Dog nonerythroid beta-spectrin mRNA, 3' end.//5.6e-88:495:92//L02897
R-PLACE2000223
R-PLACE2000235//HS_3159_B1_B06_T7 CIT Approved Human Genomic Sperm Library D Homo sapiens genomic clone Plate=3159 Col=11 Row=D, genomic survey sequence.//1.8e-88:454:96//AQ179271
R-PLACE2000246//Homo sapiens chromosome 3p clone RPCI4-544D10, WORKING DRAFT SEQUENCE, 58 unordered pieces.//9.1e-41:282:86//AC005902
R-PLACE2000264//Homo sapiens DNA sequence from PAC 95C20 on chromosome Xp11.3-11.4. Contains STSs and the DXS7 locus with GT and GTG repeat polymorphisms, complete sequence.//8.3e-35:305:80//Z97181
R-PLACE2000274//Human Chromosome 16 BAC clone CIT987SK-A-211C6, complete sequence.//3.5e-18:325:67//AC002394
R-PLACE2000302//Homo sapiens chromosome 17, clone HRPC1067M6, complete sequence.//1.5e-39:287:85//AC003043
R-PLACE2000305//Human DNA sequence *** SEQUENCING IN PROGRESS *** from clone 16915, WORKING DRAFT SEQUENCE.//1.2e-43:295:85//Z93015
R-PLACE2000317//Human DNA sequence from clone 245G19 on chromosome Xp22.11-22.2 Contains serine-threonine kinase (Txp3) gene, a pseudogene similar to ALPHA-1 PROTEIN ((CONNEXIN 43, CX43, GAP JUNCTION 43 KD HEART PROTEIN)), and the 3' end of the RS1 (X-linked juvenile retinoschisis precursor protein) gene. Contains ESTs, STSs and GSSs, complete sequence.//4.0e-05:284:65//Z92542
R-PLACE2000335//Homo sapiens clone DJ0755D09, WORKING DRAFT SEQUENCE, 3 unordered pieces.//1.5e-26:334:70//AC006147
R-PLACE2000342//Fugu rubripes cosmid 258N02 containing IGFII, TH, NAP2 genes.//4.0e-05:254:64//AL021880
R-PLACE2000347//Human DNA sequence *** SEQUENCING IN PROGRESS *** from clone 799N4, WORKING DRAFT SEQUENCE.//1.6e-82:504:88//AL022147
R-PLACE2000359//Human DNA sequence *** SEQUENCING IN PROGRESS *** from clone 40E16, WORKING DRAFT SEQUENCE.//2.0e-36:314:80//AL031963
R-PLACE2000366//Human DNA sequence *** SEQUENCING IN PROGRESS *** from clone 796F18, WORKING DRAFT SEQUENCE.//2.0e-48:389:80//AL031291
R-PLACE2000371
R-PLACE2000373//Human DNA sequence *** SEQUENCING IN PROGRESS *** from clone 324M8, WORKING DRAFT SEQUENCE.//0.61:231:61//AL008734
R-PLACE2000379//Homo sapiens clone DJ0892G19, complete sequence.//3.5e-11:287:67//AC004917
R-PLACE2000394//Human DNA sequence from clone 465N24 on chromosome 1p35.1-36.13. Contains two novel genes, ESTs, GSSs and CpG islands, complete sequence.//6.8e-108:553:96//AL031432
R-PLACE2000398//Homo sapiens clone RG074A24, WORKING DRAFT SEQUENCE, 25 unordered pieces.//2.9e-26:326:73//AC005059
R-PLACE2000399
R-PLACE2000404//Homo sapiens chromosome 5, BAC clone 282B7 (LBNL H192), complete sequence.//6.5e-84:434:96//AC005216
R-PLACE2000411//P.clarkii mRNA; repeat region (ID 2R).//0.47:104:70//Z54273
R-PLACE2000419
R-PLACE2000425//Homo sapiens X-linked anhidroitic ectodermal dysplasia protein gene (EDA), exon 2 and flanking repeat regions.//1.9e-40:447:74//AF003528
R-PLACE2000427
R-PLACE2000433//Human Chromosome 15 pac pDJ24m8, complete sequence.//3.5e-40:286:85//AC000379
R-PLACE2000435
R-PLACE2000438//Homo sapiens full-length insert cDNA clone ZE04D01.//2.2e-107:523:98//AF086521
R-PLACE2000450 4.1e-42:328:79//AG006257
R-PLACE2000455
R-PLACE2000458//Homo sapiens chromosome 5p, BAC clone 50g21 (LBNL H154), complete-sequence.//5.1e-116:570:97//AC005740
R-PLACE2000465//Human BAC clone RG191D16, complete sequence.//6.3e-37:408:75//AC002460
R-PLACE2000477//M.musculus tex264 mRNA (3'region).//7.5e-06:117:76//X80427
R-PLACE3000004
R-PLACE3000029//Human DNA sequence from PAC 506G2 contains STSs and a CpG island.//5.8e-34:308:78//Z82976
R-PLACE3000059//Mus musculus mRNA for ubiquitin conjugating enzyme.//1.1e-36:273:87//Y17267
R-PLACE3000070//Homo sapiens chromosome 5, PAC clone 17e19 (LBNL H148), complete sequence.//2.3e-10:181:71//AC004648
R-PLACE3000103//Human DNA sequence *** SEQUENCING IN PROGRESS *** from clone 30A23, WORKING DRAFT SEQUENCE.//1.2e-48:495:74//AL022156
R-PLACE3000119//Homo sapiens Chromosome 22q12 BAC Clone 58b8 In Meningioma Deletion Region, complete sequence.//3.4e-39:283:85//AC000026
R-PLACE3000124//Homo sapiens chromosome 5, P1 clone 793c5 (LBNL H57), complete sequence.//9.2e-23:171:76//AC005200
R-PLACE3000136//U.arctos microsatellite DNA, clone UarMU23.//0.00052:171:65//Y09645
R-PLACE3000142//HS_3037_82_B02_MR CIT Approved Human Genomic Sperm Library D Homo sapiens genomic clone Plate=3037 Col=4 Row=D, genomic survey sequence.//0.88:121:66//AQ097023
R-PLACE3000147//Mus musculus DNA for ADAMTS-1, complete cds.//3.3e-23:472:66//AB001735
R-PLACE3000148//Human DNA from cosmid L27h9, Huntington's Disease Region, chromosome 4p16.3 contains CpG island.//3.5e-11:176:73//Z49237
R-PLACE3000155//Homo sapiens chromosome 17, clone hRPK.597_M_12, complete sequence.//6.9e-106:549:94//AC005277
R-PLACE3000156//Homo sapiens BAC clone RG067E13 from 7q21, complete sequence.//7.0e-38:545:70//AC002383
R-PLACE3000157
R-PLACE3000158//, complete sequence.//1.4e-33:283:81//AC005500
R-PLACE3000160
R-PLACE3000169//Homo sapiens chromosome 19, BAC CIT-B-191n6, complete sequence.//5.2e-43:229:85//AC006130
R-PLACE3000194
R-PLACE3000197//Homo sapiens chromosome 17, clone hRPK.401_O_9, complete sequence.//7.2e-61:394:89//AC005291
R-PLACE3000199//Homo sapiens Xq28 genomic DNA in the region of the L1CAM locus containing the genes for neural cell adhesion molecule L1 (L1CAM), arginine-vasopressin receptor (AVPR2), C1 p115 (C1), ARD)1 N-acetyltransferase related protein (TE2), renin-binding protein (RbP), host cell factor 1 (HCF1), and interleukin-1 receptor-associated kinase (IRAK) genes, complete cds, and Xq281u2 gene.//0.23:309:57//U52112 R-PLACE3000207//CIT-HSP-384B14.TR CIT-HSP Homo sapiens genomic clone 384B14, genomic survey sequence.//1.1e-15:156:81//B54637
R-PLACE3000208//Human DNA sequence *** SEQUENCING IN PROGRESS *** from clone 591N18, WORKING DRAFT SEQUENCE.//1.3e-16:139:87//AL031594
R-PLACE3000218//HS_3185_B1_B01_T7 CIT Approved Human Genomic Sperm Library D Homo sapiens genomic clone Plate=3185 Col=1 Row=D, genomic survey sequence.//3.5e-07:120:75//AQ155720
R-PLACE3000220//Homo sapiens chromosome 17, clone HRPC1096F1, complete sequence.//2.4e-44:363:80//AC004167
R-PLACE3000226//Caenorhabditis elegans cosmid M01G5.//0.88:95:77//AF078786
R-PLACE3000230//Homo sapiens ccr2b (ccr2), ccr2a (ccr2), ccr5 (ccr5) and ccr6 (ccr6) genes, complete cds, and lactoferrin (lactoferrin) gene, partial cds, complete sequence.//5.3e-69:536:81//U95626
R-PLACE3000242//Sequence 1 from patent US 5599918.//3.2e-62:546:78//I35489
R-PLACE3000244//M.musculus mRNA for 200 kD protein.//1.7e-45:404:75//X80169
R-PLACE3000254//Human mRNA for KIAA0309 gene, partial cds.//7.5e-28:174:94//AB002307
R-PLACE3000271//Human DNA sequence *** SEQUENCING IN PROGRESS *** from clone 423B22, WORKING DRAFT SEQUENCE.//3.9e-54:492:77//AL034379
R-PLACE3000276//Homo sapiens PAC clone DJ0320J15 from Xq23, complete sequence.//5.4e-12:176:69//AC004081
R-PLACE3000304//Homo sapiens chromosome 19, cosmid R26660, complete sequence.//5.7e-114:555:97//AC005328
R-PLACE3000310//Human DNA sequence *** SEQUENCING IN PROGRESS *** from clone 467L1, WORKING DRAFT SEQUENCE.//6.2e-51:314:84//Z98884
R-PLACE3000320//Homo sapiens elastin gene, exons 5-27 and alternatively spliced products, partial cds.//2.5e-44:289:90//U93037
R-PLACE3000322//Human argininosuccinate lyase (ASL) gene, exon 3.//5.9e-20:153:88//M21006
R-PLACE3000331//Homo sapiens clone DJ0592G07, WORKING DRAFT SEQUENCE, 3 unordered pieces.//1.1e-43:230:84//AC005480
R-PLACE3000339
R-PLACE3000341//Homo sapiens 3p22 Contig 7 PAC RPCI4-672N11 (Roswell Park Cancer Institute Human PAC Library) complete sequence.//2.5e-111:550:97//AC006055
R-PLACE3000350//Human DNA sequence from clone 243E7 on chromosome 22q12.1. Contains ESTs, STSs and GSSs, complete sequence.//1.5e-44:314:78//AL022323
R-PLACE3000352//HS_3095_B1_E09_MR CIT Approved Human Genomic Sperm Library D Homo sapiens genomic clone Plate=3095 Col=17 Row=J, genomic survey sequence.//8.5e-73:356:99//AQ123142
R-PLACE3000353//Caenorhabditis elegans DNA *** SEQUENCING IN PROGRESS *** from clone Y22F5, WORKING DRAFT SEQUENCE.//0.21:194:63//Z99712
R-PLACE3000362//Plasmodium falciparum coronin gene, isolate 3D7.//0.26:360:60//AJ002197
R-PLACE3000363
R-PLACE3000365//Human BAC clone RG343P13 from 7q31, complete sequence.//4.6e-52:487:76//AC002465
R-PLACE3000373//HS_3202_B1_G05_T7 CIT Approved Human Genomic Sperm Library D Homo sapiens genomic clone Plate=3202 Col=9 Row=N, genomic survey sequence.//2.4e-75:437:90//AQ252699
R-PLACE3000388//Human DNA sequence *** SEQUENCING IN PROGRESS *** from clone 732E4, WORKING DRAFT SEQUENCE.//6.4e-61:515:81//AL008722
R-PLACE3000399//Homo sapiens clone DJ1186P10, WORKING DRAFT SEQUENCE, 6 unordered pieces.//0.00098:444:60//AC005231
R-PLACE3000400//Plasmodium falciparum 3D7 chromosome 12 PFYAC357 genomic sequence, WORKING DRAFT SEQUENCE, 7 unordered pieces.//0.78:155:66//AC005506
R-PLACE3000401//Homo sapiens clone DJ1147A01, WORKING DRAFT SEQUENCE, 25 unordered pieces.//8.0e-47:223:81//AC006023
R-PLACE3000402//Homo sapiens chromosome 17, clone 104H12, complete sequence.//1.0:179:63//AC000003
R-PLACE3000405//Homo sapiens chromosome 7qtelo BAC F6, complete sequence.//2.4e-44:466:74//AF104455
R-PLACE3000406//Human DNA sequence *** SEQUENCING IN PROGRESS *** from clone 268H5, WORKING DRAFT SEQUENCE.//7.7e-49:471:75//AL008718
R-PLACE3000413
R-PLACE3000416//Homo sapiens *** SEQUENCING IN PROGRESS *** from PAC 1577, WORKING DRAFT SEQUENCE.//5.4e-42:416:77//AJ009612
R-PLACE3000425//Human DNA sequence from PAC 130G2 on chromosome 6p22.2-22.3. Contains ribosomal protein L29 pseudogene, ESTs and STSs.//1.1e-41:366:78//AL008627
R-PLACE3000455//Human DNA sequence *** SEQUENCING IN PROGRESS *** from clone 469D22, WORKING DRAFT SEQUENCE.//3.8e-98:549:92//AL031284
R-PLACE3000475//Human signal transducing adaptor molecule STAM mRNA, complete cds.//1.9e-82:440:92//U43899
R-PLACE3000477
R-PLACE4000009//R.norvegicus mRNA encoding 45kDa protein which binds to heymann nephritis antigen gp330.//6.6e-17:344:68//Z11995
R-PLACE4000014//Homo sapiens mRNA for KIAA0809 protein, partial cds.//2.7e-83:433:95//AB018352
R-PLACE4000034//cSRL-51C5-u cSRL flow sorted Chromosome 11 specific cosmid Homo sapiens genomic clone cSRL-51C5, genomic survey sequence.//0.54:116:66//B04984
R-PLACE4000049//Human BAC clone GS165I04 from 7q21, complete sequence.//0.29:313:59//AC002379
R-PLACE4000052//Plasmodium falciparum DNA *** SEQUENCING IN PROGRESS *** from MAL4P1, WORKING DRAFT SEQUENCE.//0.0058:466:57//AL034557
R-PLACE4000063//Homo sapiens chromosome 7q22 sequence, complete sequence.//0.98:246:61//AF053356
R-PLACE4000089//RPCI11-15I1.TUB RPCI-11 Homo sapiens genomic clone RPCI-11-15I1, genomic survey sequence.//3.2e-07:284:60//B82414
R-PLACE4000093//Plasmodium falciparum 3D7 chromosome 12 PFYAC357 genomic sequence, WORKING DRAFT SEQUENCE, 7 unordered pieces.//2.4e-07:429:60//AC005506
R-PLACE4000100
R-PLACE4000106//Homo sapiens clone 24561 unknown mRNA, partial cds.//9.3e-100:419:91//AF055010
R-PLACE4000128//Human Chromosome 16 BAC clone CIT987SK-A-61E3, complete sequence.//9.6e-45:284:90//AC003007
R-PLACE4000129//Homo sapiens mRNA, chromosome 1 specific transcript KIAA0500.//1.6e-19:118:100//AB007969
R-PLACE4000147//Homo sapiens BAC clone NH0342K06 from 2, complete sequence.//8.9e-17:208:73//AC005034
R-PLACE4000156//Homo sapiens DNA sequence from PAC 352A20 on chromosome 6q24.1-25.1. Contains a pseudogene similar to yeast, bacterial, worm and slime mold hypothetical genes, and a gene coding for an aldehyde dehydrogenase family protein. Contains ESTs, STSs and GSSs, complete sequence.//3.7e-43:281:90//AL021939
R-PLACE4000192
R-PLACE4000222//Homo sapiens clone DJ1129J21, WORKING DRAFT SEQUENCE, 25 unordered pieces.//5.4e-44:280:82//AC005631
R-PLACE4000233//Homo sapiens full-length insert cDNA YH59G06.//1.8e-79:414:97//AF074981
R-PLACE4000247//Homo sapiens chromosome 17, clone hRPK.156_L_14, complete sequence.//5.7e-59:558:76//AC005821
R-PLACE4000250//CIT-HSP-2335L20.TR CIT-HSP Homo sapiens genomic clone 2335L20, genomic survey sequence.//1.7e-44:313:84//AQ037381
R-PLACE4000252//Homo sapiens chromosome 17, clone hRPK.700_H_6, complete sequence.//1.5e-39:311:83//AC005920
R-PLACE4000261//H.sapiens BF1P-g1H03np gene for immunoglobulin heavy chain variable region.//0.33:197:61//Z80410
R-PLACE4000269//Homo sapiens chromosome 4 clone B368A9 map 4q25, complete sequence.//1.4e-31:327:68//AC005510
R-PLACE4000270//Homo sapiens DNA for amyloid precursor protein, complete cds.//2.3e-32:345:74//D87675
R-PLACE4000300//Sequence 61 from patent US 5744300.//0.0017:51:98//AR003339
R-PLACE4000320//Human DNA sequence from clone 441J1 on chromosome 6p24 Contains STS, GSS, complete sequence.//8.2e-41:295:85//Z99495
R-PLACE4000323//Human chromosome 11 187a8 cosmid, complete sequence.//1.3e-32:404:75//U73640
R-PLACE4000326
R-PLACE4000344//Homo sapiens PAC clone DJ0988G15 from 7q33-q35, complete sequence.//0.32:135:68//AC005587
R-PLACE4000367//H.sapiens gene encoding RING finger protein.//0.61:146:67//Y07829
R-PLACE4000369//HS_3181_A1_B02_MR CIT Approved Human Genomic Sperm Library D Homo sapiens genomic clone Plate=3181 Col=3 Row=C, genomic survey sequence.//7.1e-80:424:94//AQ173222
R-PLACE4000379//Human DNA sequence *** SEQUENCING IN PROGRESS *** from clone 1104E15, WORKING DRAFT SEQUENCE.//1.7e-05:160:65//AL022312
R-PLACE4000387//Homo sapiens clone DJ0876A24, WORKING DRAFT SEQUENCE, 6 unordered pieces.//2.4e-47:351:81//AC004913
R-PLACE4000392//Human DNA sequence from clone 751H9 on chromosome 6q13. Contains part of an unknown gene, ESTs, STSs and GSSs, complete sequence.//8.5e-88:541:88//AL034377
R-PLACE4000401//Human Chromosome 11 overlapping pacs pDJ235k10 and pDJ239b22, WORKING DRAFT SEQUENCE, 17 unordered pieces.//2.7e-17:143:83//AC000406
R-PLACE4000411
R-PLACE4000445//Homo sapiens clone DJ0613C23, WORKING DRAFT SEQUENCE, 4 unordered pieces.//0.028:91:78//AC005628
R-PLACE4000465//Human DNA sequence *** SEQUENCING IN PROGRESS *** from clone 30A23, WORKING DRAFT SEQUENCE.//1.6e-43:532:71//AL022156
R-PLACE4000489//Plasmodium falciparum chromosome 2, section 64 of 73 of the complete sequence.//4.1e-06:357:61//AE001427
R-PLACE4000494//Homo sapiens 12p13.3 PAC RPCI5-1063M23 (Roswell Park Cancer Institute Human PAC Library) complete sequence.//2.7e-37:416:74//AC005865
R-PLACE4000522
R-PLACE4000548//Homo sapiens 12p13.3 PAC RPCI5-1096D14 (Roswell Park Cancer Institute Human PAC Library) complete sequence.//0.0020:383:60//AC005342
R-PLACE4000558//Homo sapiens 12q24 BAC RPCI11-162P23 (Roswell Park Cancer Institute Human BAC library) complete sequence.//2.9e-44:465:75//AC002996
R-THYRO1000026//Human DNA sequence *** SEQUENCING IN PROGRESS *** from clone 37E16, WORKING DRAFT SEQUENCE.//2.2e-43:354:82//Z83844
R-THYRO1000034//Plasmodium falciparum chromosome 2, section 59 of 73 of the complete sequence.//0.022:327:60//AE001422
R-THYRO1000035//HS_3018_B2_F10_T7 CIT Approved Human Genomic Sperm Library D Homo sapiens genomic clone Plate=3018 Col=20 Row=L, genomic survey sequence.//2.3e-36:228:91//AQ092318
R-THYRO1000040//Plasmodium falciparum 3D7 chromosome 12 PFYAC293 genomic sequence, WORKING DRAFT SEQUENCE, 9 unordered pieces.//1.0:367:56//AC004157
R-THYRO1000070//***ALU WARNING: Human Alu-Sq subfamily consensus sequence.1/1e-44:284:89//U14573
R-THYRO1000072//***ALU WARNING: Human Alu-J subfamily consensus sequence.//6.6e-33:150:83//U14567
R-THYRO1000085
R-THYRO1000092//Homo sapiens chromosome 7qtelo BAC F6, complete sequence.//3.3e-36:301:78//AF104455
R-THYRO1000107//Human DNA sequence *** SEQUENCING IN PROGRESS *** from clone 125I3, WORKING DRAFT SEQUENCE.//1.4e-35:282:82//AL033528
R-THYRO1000111//Homo sapiens Chromosome 16 BAC clone CIT987SK-A-279B10, complete sequence.//4.0e-32:351:65//AC002300
R-THYRO1000121//Human chromosome 16 BAC clone CIT987SK-A-962B4, complete sequence.//6.6e-77:507:85//U91318
R-THYRO1000124//High throughput sequencing of human chromosome 12, WORKING DRAFT SEQUENCE, 1 ordered pieces.//0.66:334:59//AC005840
R-THYRO1000129//Homo sapiens TED protein (TED) mRNA, complete cds.//2.3e-88:449:96//AF087142
R-THYRO1000132//Human DNA sequence *** SEQUENCING IN PROGRESS *** from clone 212A2, WORKING DRAFT SEQUENCE.//1.1e-40:298:84//Z95114
R-THYRO1000156//Homo sapiens chromosome 17, clone hRPK.849_N_15, complete sequence.//3.4e-37:425:73//AC005703
R-THYRO1000163//RPCI11-1B20.TVB RPCI-11 Homo sapiens genomic clone RPCI-11-1B20, genomic survey sequence.//8.4e-38:276:84//B63536
R-THYRO1000173//Human DNA sequence from PAC 323B6 on chromosome X contains ESTs CpG island.//1.1e-70:553:81//Z83841
R-THYRO1000186//Human DNA sequence *** SEQUENCING IN PROGRESS *** from clone 410I8, WORKING DRAFT SEQUENCE.//6.7e-41:345:81//AL031732
R-THYRO1000187//Human thymopoietin (TMPO) gene, partial exon 6, complete exon 7, partial exon 8, and partial cds for thymopoietin beta.//1.3e-43:356:80//U18271
R-THYRO1000190//Homo sapiens chromosome 17, clone HRPC843B9, complete sequence.//2.6e-40:386:77//AC004139
R-THYRO1000197//Homo sapiens mRNA for poly(A)-specific ribonuclease.//1.1e-108:535:97//AJ005698
R-THYRO1000199//Homo sapiens mRNA for KIAA0652 protein, complete cds.//1.4e-113:559:97//AB014552
R-THYRO1000206//Rat PMSG-induced ovarian mRNA, 3'sequence, N4.//4.0e-43:318:86//D84482
R-THYRO1000221//Human DNA from overlapping chromosome 19 cosmids R31396, F25451, and R31076 containing COX6B and UPKA, genomic sequence, complete sequence.//2.7e-44:452:76//AC002115
R-THYRO1000241//Homo sapiens Cosmid Clone p129d11 unknown chromosomal location, complete sequence.//4.8e-58:447:81//AC000039
R-THYRO 1000242
R-THYRO1000253//Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence.//3.4e-56:300:84//Z95152
R-THYRO1000270
R-THYRO1000279//Human DNA sequence *** SEQUENCING IN PROGRESS *** from clone 531H16, WORKING DRAFT SEQUENCE.//4.8e-113:584:96//AL031664
R-THYRO1000288//Homo sapiens mRNA for Hs Ste24p, complete cds.//1.1e-98:566:91//AB016068
R-THYRO1000320//HS_2033_B1_A07_T7 CIT Approved Human Genomic Sperm Library D Homo sapiens genomic clone Plate=2033 Col=13 Row=B, genomic survey sequence.//0.97:211:63//AQ233366
R-THYRO1000327//Sequence 1 from patent US 5541298.//2.8e-52:289:93//I24058
R-THYRO1000343//Homo sapiens mRNA for KIAA0790 protein, partial cds.//1.1e-111:559:96//AB018333
R-THYRO1000358//Human selenium-binding protein (hSBP) mRNA, complete cds.//4.6e-47:317:87//U29091
R-THYRO1000368//HS_3049_A1_E12_MF CIT Approved Human Genomic Sperm Library D Homo sapiens genomic clone Plate=3049 Col=23 Row=I, genomic survey sequence.//7.0e-11:111:83//AQ126777
R-nnnnnnnnnnnn
R-THYRO1000387//Homo sapiens PAC clone DJ1048B16 from 7q34-q36, complete sequence.//2.4e-101:545:93//AC006019
R-THYRO1000394//Homo sapiens Chromosome 11q12.2 PAC clone pDJ688p12 containing uteroglobin gene, WORKING DRAFT SEQUENCE, 11 unordered pieces.//1.6e-46:233:88//AC006078
R-THYRO1000395//Mouse MIPP mRNA for a placenta-expressed gene.//2.3e-57:395:85//X58523
R-THYRO 1000401
   3.3e-111:546:97//AF051907
R-THYRO1000438//Homo sapiens clone DJ1186P10, WORKING DRAFT SEQUENCE, 6 unordered pieces.//2.7e-44:289:89//AC005231
R-THYRO1000452//Homo sapiens chromosome 17, clone hRPK.243_K_12, complete sequence.//6.7e-27:222:82//AC005668
R-THYRO1000471//Human DNA sequence *** SEQUENCING IN PROGRESS *** from clone 102D24, WORKING DRAFT SEQUENCE.//2.4e-36:369:76//AL021391
R-THYRO1000484//Homo sapiens clone DJ1099N07, complete sequence.//1.6e-43:288:81//AC004962
R-THYRO1000488//Homo sapiens chromosome 5p, BAC clone 50g21 (LBNL H154), complete sequence.//1.6e-95:512:94//AC005740
R-THYRO1000501//HS _2208_A1_G11_T7 CIT Approved Human Genomic Sperm Library D Homo sapiens genomic clone Plate=2208 Col=21 Row=M, genomic survey sequence.//0.0063:189:63//AQ091586
R-THYRO1000502//Homo sapiens eIF-1A, Y isoform (EIF1AY) mRNA, complete cds.//0.19:468:60//AF000987
R-THYRO1000505//Homo sapiens chromosome 19, cosmid R31546, complete sequence.//0.20:214:58//AC004798
R-THYRO1000558
R-THYRO 1000569
R-THYRO1000570//Homo sapiens full-length insert cDNA clone ZD76G10.//4.3e-41:209:100//AF086408
R-nmmmnnnnn//Homo sapiens protein associated with Myc mRNA, complete cds.//8.2e-107:533:97//AF075587
R-THYRO 000596//Mus musculus mitochondrial DNA polymerase accessory subunit (MtPolB) mRNA, nuclear gene encoding mitochondrial protein, partial cds.//0.36:170:67//AF006072
R-THYRO1000602//Homo sapiens DNA for amyloid precursor protein, complete cds.//2.2e-53:289:92//D87675
R-THYRO 1000605
R-THYRO1000625//Homo sapiens chromosome 19, cosmid R29425, complete sequence.//1.3e-31:261:82//AC005546
R-THYRO1000637//Human DNA sequence from clone 243E7 on chromosome 22q12.1. Contains ESTs, STSs and GSSs, complete sequence.//4.0e-06:249:63//AL022323
R-THYRO1000641//P.falciparum glutamic acid-rich protein gnen, complete cds.//3.1e-08:244:68//J03998
R-THYRO1000658//***ALU WARNING: Human Alu-Sp subfamily consensus sequence.//3.9e-49:282:93//U14572
R-nnnnnnnnnnnn
R-THYRO1000666//Homo sapiens DNA sequence from PAC 329E20 on chromosome 1p34.4-36.13. Contains endothelin-converting-enzyme 1 (ECE-1), EST, STS, CA repeat, complete sequence.//1.9e-20:215:77//AL031005
R-THYRO1000676//Homo sapiens chromosome 4 clone B71M12 map 4q25, complete sequence.//1.2e-06:227:64//AC004069
R-THYRO1000684
R-THYRO1000699
R-THYRO1000712
R-THYRO1000734//Human BAC clone RG191D16, complete sequence.//3.7e-14:468:64//AC002460
R-THYRO1000748//Homo sapiens cosmid 123E15, complete sequence.//2.6e-11:182:73//AF024533
R-THYRO1000756//Sequence 21 from patent US 5552281.//1.4e-15:106:98//I25660
R-THYRO1000777//Plasmodium falciparum MAL3P2, complete sequence.//1.0:175:66//AL034558
R-THYRO1000783//CIT-HSP-2335P6.TF CIT-HSP Homo sapiens genomic clone 2335P6, genomic survey sequence.//1.2e-81:391:99//AQ038226
R-THYRO1000787//Homo sapiens chromosome Y, clone 264,M,20, complete sequence.//9.4e-07:494:58//AC004617
R-THYRO1000793
R-THYRO1000796//Human DNA sequence *** SEQUENCING IN PROGRESS *** from clone 167P19, WORKING DRAFT SEQUENCE.//1.7e-42:379:79//Z93014
R-THYRO1000805//Human Chromosome 11 pac pDJ610i20, WORKING DRAFT SEQUENCE, 18 unordered pieces.//4.7e-40:362:76//AC002555
R-THYRO1000815//Human DNA sequence *** SEQUENCING IN PROGRESS *** from clone 316D5, WORKING DRAFT SEQUENCE.//4.0e-58:295:92//Z82199
R-THYRO1000829//Sequence 7 from patent US 5716622.//0.97:362:61//I87788
R-THYRO1000843//Homo sapiens Chromosome 15q11-q13 PAC clone pDJ351h23 from the Prader-Willi/Angelman Syndrome region, complete sequence.//3.3e-57:522:76//AC004738
R-THYRO1000852//Homo sapiens chromosome 11 clone CIT-HSP-1337H24, WORKING DRAFT SEQUENCE, 9 unordered pieces.//4.2e-17:291:69//AC005849
R-THYRO1000855//Human DNA sequence from clone 366B10 on chromosome 22q12.2-12.3. Contains GSSs, complete sequence.//1.1e-41:419:75//AL031592
R-THYRO1000865//Human DNA sequence *** SEQUENCING IN PROGRESS *** from clone 1125A11, WORKING DRAFT SEQUENCE.//9.0e-47:294:84//AL034549
R-THYRO1000895//Human DNA sequence *** SEQUENCING IN PROGRESS *** from clone 380F5, WORKING DRAFT SEQUENCE.//3.7e-111:569:96//AL031719
R-THYRO1000916//Homo sapiens clone DJ0965K10, WORKING DRAFT SEQUENCE, 6 unordered pieces.//1.0e-97:554:92//AC006015
R-THYRO1000926//Homo sapiens CAMP-specific phosphodiesterase 8B (PDE8B) mRNA, partial cds.//9.6e-109:566:94//AF079529
R-THYRO1000934//Homo sapiens full-length insert cDNA clone ZD69A10.//1.6e-104:539:95//AF086378
R-THYRO1000951//Homo sapiens Chromosome 11q12 pac pDJ57l14, WORKING DRAFT SEQUENCE, 29 unordered pieces.//8.9e-61:479:81//AC004229
R-THYRO1000952//Human autoimmune thyroid disease-related antigen mRNA.//5.3e-16:116:93//M28639
R-THYRO1000974//Homo sapiens ribosomal protein L33-like protein mRNA, complete cds.//3.2e-59:321:95//AF047440
R-THYRO1000975//Homo sapiens chromosome 19, cosmid F18718, complete sequence.//1.9e-44:396:79//AC006126
R-THYRO1000983//Homo sapiens chromosome 17, clone hRPK.271_K_11, complete sequence.//0.99:71:78//AC005562
R-THYRO1000984//Homo sapiens Chromosome 11q12.2 PAC clone pDJ688p12 containing uteroglobin gene, WORKING DRAFT SEQUENCE, 11 unordered pieces.//6.7e-42:320:84//AC006078
R-THYRO1000988//Homo sapiens DNA sequence from PAC 230G1 on chromosome Xp11.3. Contains EST, STS and GSS, complete sequence.//6.7e-39:292:78//Z84466
R-THYRO1001003//HS_3051_B1_H01_MR CIT Approved Human Genomic Sperm Library D Homo sapiens genomic clone Plate=3051 Col=1 Row=P, genomic survey sequence.//2.5e-39:310:83//AQ253727
R-THYRO1001031//Homo sapiens DNA sequence from PAC 230G1 on chromosome Xp11.3. Contains EST, STS and GSS, complete sequence.//2.5e-50:300:88//Z84466
R-THYRO1001033//CIT-HSP-2007J14.TF CIT-HSP Homo sapiens genomic clone 2007J14, genomic survey sequence.//5.1e-26:143:100//B56677
R-THYRO1001062//CIT-HSP-2386P3.TF.1 CIT-HSP Homo sapiens genomic clone 2386P3, genomic survey sequence.//1.4e-48:316:87//AQ239882
R-THYRO1001093
R-THYRO1001100//Homo sapiens BAC clone RG152G17 from 7q22-q31.1, complete sequence.//0.47:102:73//AC005070
R-THYRO1001120
R-THYRO1001121//Homo sapiens mRNA for beta-tubulin folding cofactor D.//8.9e-81:429:94//AJ006417
R-THYRO1001133//CIT-HSP-2381I10.TR CIT-HSP Homo sapiens genomic clone 2381I10, genomic survey sequence.//4.7e-12:237:67//AQ111077
R-THYRO1001134
R-THYRO1001142//H.sapiens CpG island DNA genomic Mse1 fragment, clone 81d1, reverse read cpg81d1.rt1a.//0.95:214:60//Z56037
R-THYRO1001173//cSRL-27c11-u cSRL flow sorted Chromosome 11 specific cosmid Homo sapiens genomic clone cSRL-27c11, genomic survey sequence.//4.6e-26:262:77//B04145
R-THYRO1001177
R-THYRO1001189//Homo sapiens DNA from chromosome 19, BAC 33152, complete sequence.//1.0e-41:281:87//AC003973
R-THYRO 1001204
R-THYRO1001213//Human Alu repeat sequence A6.//3.8e-38:236:88//U12581
R-THYRO1001262//Homo sapiens, clone hRPK.16_A_1, complete sequence.//8.7e-53:442:79//AC006227
R-THYRO1001271//*** SEQUENCING IN PROGRESS *** Homo sapiens chromosome 4, BAC clone C0224P12; HTGS phase 1, WORKING DRAFT SEQUENCE, 13 unordered pieces.//0.53:330:61//AC004630
R-THYRO 1001290
R-THYRO1001313//H.sapiens CpG island DNA genomic Mse1 fragment, clone 195h3, forward read cpg195h3.ft1b.//0.046:126:66//Z57783
R-THYRO1001320//Human DNA sequence *** SEQUENCING IN PROGRESS *** from clone 424J12, WORKING DRAFT SEQUENCE.//3.0e-58:476:80//Z82207
R-THYRO100132//Plasmodium falciparum MAL3P2, complete sequence.//1.0e-08:408:62//AL034558
R-nnnnnnnnnnnn
R-THYRO1001347//Homo sapiens mRNA for KIAA0745 protein, partial cds.//3.2e-08:266:64//AB018288
R-THYRO1001363//cSRL-72f5-u cSRL flow sorted Chromosome 11 specific cosmid Homo sapiens genomic clone cSRL-72f5, genomic survey sequence.//1.7e-85:471:92/B05884
R-THYRO1001365//Homo sapiens chromosome 10 clone CIT987SK-1163G10 map 10q25, complete sequence.//1.8e-109:584:94//AC005660
R-THYRO1001374
R-THYRO1001401//Human pigment epithelium-derived factor gene, complete cds.//4.2e-51:333:88//U29953
R-THYRO1001403//Human PAC clone DJ222H05 from Xq25-q26, complete sequence.//8.7e-38:307:82//AC002377
R-THYRO1001405
R-THYRO1001406//RPCI11-69F22.TK RPCI1 Homo sapiens genomic clone R-69F22, genomic survey sequence.//1.9e-67:400:90//AQ238297
R-THYRO1001411//Human DNA sequence *** SEQUENCING IN PROGRESS *** from clone 80N2, WORKING DRAFT SEQUENCE.//2.2e-06:349:63//AL031123
R-THYRO1001426//*** SEQUENCING IN PROGRESS *** Homo sapiens genomic DNA (PAC 1118i22) from chromosome 11; HTGS phase 1, WORKING DRAFT SEQUENCE.//2.2e-89:506:86//AJ002553
R-THYRO1001434//Microcentus caryae 12S mitochondrial ribosomal RNA, small subunit, mitochondrial gene, partial sequence.//1.0:176:61//U77877
R-THYRO1001458//Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.//3.3e-07:196:67//AL021578
R-THYRO1001480//Homo sapiens clone DJ0756H11, WORKING DRAFT SEQUENCE, 5 unordered piece.//1.2e-99:517:95//AC006001
R-THYRO1001487//Homo sapiens, WORKING DRAFT SEQUENCE, 97 unordered pieces.//8.5e-14:221:70//AC004085
R-THYRO10001534//HS_2242_B2_H04_MR CIT Approved Human Genomic Sperm Library D Homo sapiens genomic clone Plate=2242 Col=8 Row=P, genomic survey sequence.//0.00012:141:68//AQ182326
R-THYRO1001537//Human DNA sequence from clone 111F4 on chromosome Xq23 Contains GSSs, complete sequence.//0.42:323:60//AL023876
R-THYRO1001541//Homo sapiens clone RG228D17, WORKING DRAFT SEQUENCE, 2 unordered pieces.//1.7e-42:370:78//AC005077
R-THYRO1001559//Homo sapiens 12q24.2 PAC RPCI5-944M2 (Roswell Park Cancer Institute Human PAC Library) complete sequence.//1.0:144:67//AC005868
R-THYRO100l570//Plasmodium falciparum 3D7 chromosome 12 PFYAC492 genomic sequence, WORKING DRAFT SEQUENCE, 5 unordered pieces.//0.43:268:61//AC005308
R-THYRO1001573//M.avium rpsL gene.//0.98:131:66//X80120
R-THYRO1001584//A.longa plastid genes for ribosomal proteins and tRNAs.//0.29:502:58//X75653
R-THYRO1001595//Human DNA sequence *** SEQUENCING IN PROGRESS *** from clone Y313F4, WORKING DRAFT SEQUENCE.//1.5e-33:319:78//AL023808
R-THYRO1001602//Homo sapiens chromosome 17, clone hRPK.142_H_19, complete sequence.//4.4e-13:320:67//AC005919
R-THYRO1001605//Human DNA sequence from PAC 358H7 on chromosome X.//1.9e-32:391:76//Z77249
R-THYRO1001617//Homo sapiens cDNA for dihydroxyacetone phosphate acyltransferase (DAP-AT).//1.9e-81:448:92//AJ002190
R-THYRO1001637//Human DNA sequence *** SEQUENCING IN PROGRESS *** from clone 688G8, WORKING DRAFT SEQUENCE.//5.4e-41:381:78//AL031671
R-THYRO1001656//HS_2201_B2_A08_MF CIT Approved Human Genomic Sperm Library D Homo sapiens genomic clone Plate=2201 Col=16 Row=B, genomic survey sequence.//0.096:162:63//AQ293168
R-THYRO1001661//Human immunoglobulin-associated (B29) gene, promoter and exon 1, partial cds.//1.0:229:62//U22954
R-THYRO1001671//Homo sapiens mRNA for 2'-5' oligoadenylate synthetase 59 kDa isoform.//4.8e-110:562:95//AJ225089
R-THYRO1001673//CIT-HSP-2327D12.TR CIT-HSP Homo sapiens genomic clone 2327D12, genomic survey sequence.//1.5e-17:224:68//AQ042426
R-THYRO1001703//Homo sapiens clone 198 unknown mRNA, partial sequence.//1.6e-44:251:93//AF091072
R-THYRO1001706//Homo sapiens clone DJ0935K16, complete sequence.//1.8e-26:378:68//AC006011
R-THYRO1001721//, complete sequence.//1.3e-101:571:92//AC005500
R-nnnnnnnnnnnn
R-THYRO1001745//Homo sapiens chromosome 5, PAC clone 247f3 (LBNL H85), complete sequence.//1.1e-15:193:70//AC004777
R-THYRO1001746//Human inter-alpha-trypsin inhibitor light chain (ITI) gene, exon 3.//0.54:260:61//M88244
R-THYRO1001772//Human DNA sequence *** SEQUENCING IN PROGRESS *** from clone 30A23, WORKING DRAFT SEQUENCE.//1.6e-12:285:64//AL022156
R-THYRO1001793
R-THYRO1001809//Human DNA sequence *** SEQUENCING IN PROGRESS *** from clone 1071N3, WORKING DRAFT SEQUENCE.//2.5e-43:486:74//AL031728
R-THYRO1001854//Homo sapiens chromosome 17, clone hRPK.74_E_22, complete sequence.//5.0e-41:245:87//AC005696
R-THYRO1001895//Human Chromosome 11p14.3 PAC clone 6-106f23, complete sequence.//4.4e-12:419:61//AC005137
R-THYRO1001907//Homo sapiens Chromosome 22q11.2 Cosmid Clone 24b In DGCR Region, complete sequence.//8.1e-35:340:78//AC000075
R-VESEN1000122//Homo sapiens Luman mRNA, complete cds.//1.3e-23:138:98//AF009368
R-Y79AA1000013
R-Y79AA1000033//Homo sapiens BAC clone GS114I09 from 7p14-p15, complete sequence.//9.9e-112:551:97//AC006027
R-Y79AA1000037//CIT-HSP-2334F3.TR CIT-HSP Homo sapiens genomic clone 2334F3, genomic survey sequence.//0.16:308:60//AQ036673
R-Y79AA1000059//Homo sapiens Chromosome 16 BAC clone CIT987SK-A-279B10, complete sequence.//6.1e-56:314:88//AC002300
R-Y79AA1000065//Human carboxylesterase gene, exon 5.//0.64:203:63//D21079
R-Y79AA1000131//*** SEQUENCING IN PROGRESS *** Homo sapiens chromosome 4, BAC clone C0548N01; HTGS phase 1, WORKING DRAFT SEQUENCE, 31 unordered pieces.//7.0e-18:169:79//AC004795
R-Y79AA1000181//Human DNA sequence from clone 612B18 on chromosome lq24-25.3 Contains exon from gene similar to 40S ribosomal protein, first coding exon of dynamin 2 (DYNII). ESTs, STS, GSS, CpG Island, complete sequence.//1.1e-106:474:98//AL031864
R-Y79AA1000202//CIT978SK-A-518G2.TP CIT978SK Homo sapiens genomic clone A-518G2, genomic survey sequence.//1.0e-10:78:97//B68074
R-Y79AA1000214//Homo sapiens clone DJ0673M15, WORKING DRAFT SEQUENCE, 33 unordered pieces.//6.5e-59:386:90//AC004854
R-Y79AA1000230//Cytauxzoon felis 18S ribosomal RNA.//1.0:167:62//L19080
R-Y79AA1000231//HS_3009_A1_H03_T7 CIT Approved Human Genomic Sperm Library D Homo sapiens genomic clone Plate=3009 Col=5 Row=O, genomic survey sequence.//6.4e-52:348:88//AQ090225
R-Y79AA1000258//Hepatitis C virus HCV-B9 gene for NS5, partial cds.//0.65:127:65//D10558
R-Y79AA1000268//Human DNA sequence from PAC 162H14 on chromosome 22. Contains 3' part of a FIBULIN 1 like gene and ESTs, complete sequence.//4.7e-40:300:84//Z98047
R-Y79AA10003131//Human DNA sequence from PAC 179I15, BRCA2 gene region chromosome 13q12-q13 contains Klotho ESTs and CpG island.//5.0e-14:136:83//Z92540
R-Y79AA1000328
R-Y79AA1000342//S.clavuligerus linear plasmid pSCL (complete sequence).//0.55:189:65//X54107
R-Y79AA1000346//Human MEST mRNA, complete cds.//0.00013:52:100//D78611
R-Y79AA1000349//M.musculus Spnr mRNA for RNA binding protein.//8.8e-36:300:81//X84692
R-Y79AA1000355//Human DNA sequence from clone 551E13 on chromosome Xp11.2-11.3 Contains farnesyl pyrophosphate synthetase pseudogene, VT4 protein pseudogene, EST, GSS, complete sequence.//5.7e-45:403:80//AL022163
R-Y79AA1000368
R-Y79AA1000405//RPCI11-16B12.TPB RPCI-11 Homo sapiens genomic clone RPCI-11-16B12, genomic survey sequence.//0.10:171:65//B88000
R-Y79AA1000410//Human DNA sequence *** SEQUENCING IN PROGRESS *** from clone 92N15, WORKING DRAFT SEQUENCE.//4.1e-50:361:83//Z93097
R-Y79AA1000420//Plasmodium falciparum merozoite surface protein 4, merozoite surface protein 5, merozoite surface protein 2, and adenylosuccinate lyase genes, complete cds.//0.071:474:57//AF033037
R-Y79AA1000469//Homo sapiens clone NH0140K04, complete sequence.//1.8e-86:221:90//AC005033
R-Y79AA1000480//Homo sapiens chromosome 4 clone B240N9 map 4q25, complete sequence.//2.1e-14:179:72//AC004057
R-Y79AA1000538//Homo sapiens clone DJ0826E18, WORKING DRAFT SEQUENCE, 4 unordered pieces.//4.5e-43:321:83//AC005282
R-Y79AA1000539//Homo sapiens PAC clone DJ0074M20 from X, complete sequence.//0.0012:275:59//AC006143
R-Y79AA1000540//Z.diploperemnis repetitive DNA (clone ZEAR 260).//0.0017:258:62//X53609
R-Y79AA1000560//Mouse mRNA for alpha-adaptin (C).//6.1e-32:390:70//X14972
R-Y79AA1000574//Homo sapiens chromosome 9q34, clone 23B4, complete sequence.//0.96:224:61//AC002325
R-Y79AA1000627//Homo sapiens full-length insert cDNA ZA77G02.//6.3e-100:533:94//AF075117
R-Y79AA1000705//RPCI11-76G7.TV RPCI11 Homo sapiens genomic clone R-76G7, genomic survey sequence.//4.6e-88:429:98//AQ268433
R-Y79AA1000734//Homo sapiens peroxisomal biogenesis factor (PEX11b) mRNA, complete cds.//2.7e-112:586:95//AF093670
R-Y79AA1000748
R-Y79AA1000752
R-Y79AA1000774//CIT-HSP-2288K24.TF CIT-HSP Homo sapiens genomic clone 2288K24, genomic survey sequence.//5.3e-45:316:86//AQ005014
R-Y79AA1000782//Human mRNA for KIAA0246 gene, partial cds.//5.0e-17:107:100//D87433
R-Y79AA1000784//Plasmodium falciparum 3D7 chromosome 12 PFYAC181 genomic sequence, WORKING DRAFT SEQUENCE, 8 unordered pieces.//0.00034:520:55//AC005505
R-Y79AA1000794//Human DNA sequence *** SEQUENCING IN PROGRESS *** from clone 989H11, WORKING DRAFT SEQUENCE.//0.015:322:60//Z83851
R-Y79AA1000800//M.musculus tex264 mRNA (3'region).//1.1e-06:104:78//X80427
R-nnnnnnnnnnnn//CIT-HSP-2295G6.TF CIT-HSP Homo sapiens genomic clone 2295G6, genomic survey sequence.//0.67:152:62//AQ007605
R-Y79AA1000805//Human Chromosome 11 Cosmid cSRL30h11, complete sequence.//3.1e-26:423:68//U73642
R-Y79AA1000824//Human DNA sequence *** SEQUENCING IN PROGRESS *** from clone 329A5, WORKING DRAFT SEQUENCE.//1.1e-08:449:61//Z97832
R-Y79AA1000827//Triticum aestivum heat shock protein 101 kDa (HSP101) mRNA, complete cds.//1.0:101:69//AF083344
R-Y79AA1000850//Homo sapiens small optic lobes homolog (SOLH) mRNA, complete cds.//0.40:386:59//U85647
R-Y79AA1000962//CIT-HSP-2298N11.TR CIT-HSP Homo sapiens genomic clone 2298N11, genomic survey sequence.//0.00019:253:65//AQ013111
R-Y79AA1000968//Rattus norvegicus initiation factor eIF-2B gamma subunit (eIF-2B gamma) mRNA, complete cds.//1.7e-58:446:80//U38253
R-Y79AA1000969
R-Y79AA1000976//CIT-HSP-2350C4.TF CIT-HSP Homo sapiens genomic clone 2350C4, genomic survey sequence.//3.3e-60:295:100//AQ061422
R-Y79AA1000985//Mus musculus pericentrin mRNA, complete cds.//5.9e-38:348:76//U05823
R-Y79AA1001023
R-Y79AA1001041
R-Y79AA1001048
R-Y79AA1001061//Homo sapiens Chromosome 16 BAC clone CIT987SK-A-427H10, complete sequence.//1.2e-60:537:78//AC004626
R-Y79AA1001068//Homo sapiens P1 clone GSP13996 from 5q12, complete sequence.//2.3e-41:405:77//AC005031
R-Y79AA1001077
R-Y79AA1001078//Homo sapiens 12q13.1 PAC RPCI1-228P16 (Roswell Park Cancer Institute Human PAC Library) complete sequence.//2.0e-09:534:59//AC004801
R-Y79AA1001105//Staphyloccous epidermidis trimethoprim resistance plasmid pSK639//0.0072:309:63//U40259
R-Y79AA1001145//RPCI11-59N12.TK RPCI11 Homo sapiens genomic clone R-59N12, genomic survey sequence.//3.7e-07:256:64//AQ200068
R-Y79AA1001167//Homo sapiens genomic DNA, chromosome 21q22.2 (Down Syndrome region), segment 5/15, WORKING DRAFT SEQUENCE.//0.55:223:61//AP000012
R-Y79AA1001177//Human gene for Gi3 alpha protein, intron 7 through exon 9, variant U6 gene, and snRNP E protein pseudogene LH87.//7.0e-09:203:69//X54048
R-Y79AA1001185
R-Y79AA1001211//Homo sapiens 12p13.3 BAC RPCI11-543P15 (Roswell Park Cancer Institute Human BAC Library) complete sequence.//2.1e-32:277:81//AC005912
R-Y79AA1001216//Human chromosome 12p13 sequence, complete sequence.//0.98:325:59//U47924
R-Y79AA1001228//Arabidopsis thaliana genomic DNA, chromosome 5, P1 clone: MAB16, complete sequence.//0.0034:378:59//AB018112
R-Y79AA1001233//Homo sapiens clone DJ1178G13, WORKING DRAFT SEQUENCE, 5 unordered pieces.//0.19:106:72//AC004988
R-Y79AA1001236//Homo sapiens mRNA for JM23 protein, complete coding sequence (clone IMAGE 34581 and IMAGE 45355 and LLNLc110I133Q7 (RZPD Berlin)).//3.4e-109:549:95//AJ005892
R-Y79AA1001281
R-Y79AA1001299//Homo sapiens SNF5/INI1 gene, exon 9.//6.3e-24:133:100//Y17126
R-Y79AA1001312//Human immunodeficiency virus type 1 variant 43 polymerase pseudogene, partial cds.//0.0070:284:58//U45372
R-Y79AA1001323//Fugu rubripes GSS sequence, clone 027L23aG3, genomic survey sequence.//0.11:125:70//AL025355
R-Y79AA1001384//W.makrii mitochondrial CYTB and tRNA genes.//0.070:209:65//X66594
R-Y79AA1001391//Plasmodium falciparum DNA *** SEQUENCING IN PROGRESS *** from MAL1P2, WORKING DRAFT SEQUENCE.//0.80:163:62//AL031745
R-Y79AA1001394//Homo sapiens DNA from chromosome 19, cosmid R29144, complete sequence.//0.99:241:63//AC004221
R-Y79AA1001402//Homo sapiens Chr.14 PAC RPCI4-794B2 (Roswell Park Cancer Institute Human PAC Library) complete sequence.//0.25:81:80//AC005924
R-Y79AA1001493
R-Y79AA1001511//Human DNA sequence from clone 931K24 on chromosome 20p12 Contains ESTs and GSSs, complete sequence.//1.3e-35:207:95//AL034430
R-Y79AA1001533//Mouse mRNA for RNA polymerase I associated factor (PAF53), complete cds.//2.7e-44:285:81//D14336
R-nnnnnnnnnnnn//Human DNA sequence from clone 113J7 on chromosome Xp11.22-11.4. Contains part of a putative Homeobox (pseudo?) gene, ESTs and an STS, complete sequence.//0.70:365:60//AL023574
R-Y79AA1001548//Homo sapiens phosphatidylinositol 4-kinase mRNA, complete cds.//5.9e-95:517:91//L36151
R-Y79AA1001555
R-Y79AA1001585
R-Y79AA1001594//Human DNA sequence from PAC 60G11 on chromosome X; contains STS.//6.6e-19:241:76//Z94722
R-Y79AA1001603//H.sapiens CpG island DNA genomic Mse1 fragment, clone 72f8, forward read cpg72f8.ft1a.//3.3e-21:131:96//Z62766
R-Y79AA1001613
R-Y79AA1001647//Human DNA sequence from PAC 36J3, between markers DXS1192 and DXS102 on chromosome X.//6.3e-08:338:63//Z82975
R-Y79AA1001665//Homo sapiens genomic DNA, chromosome 21q22.2 (Down Syndrome region), segment 1/15, WORKING DRAFT SEQUENCE.//3.2e-11:114:84//AP000008
R-Y79AA1001679//O.cuniculus lambda-crystallin mRNA, complete cds.//3.9e-15:270:68//M22743
R-nnnnnnnnnnnn//RPCI11-42M5.TJ RPCI11 Homo sapiens genomic clone R-42M5, genomic survey sequence.//0.013:64:89//AQ052792
R-Y79AA1001696//Apis mellifera ligustica complete mitochondrial genome.//9.3e-09:428:58//L06178
R-Y79AA1001705
R-Y79AA1001711//Mus musculus 60 kDa ribonucleoprotein Ro gene, partial cds.//2.2e-45:554:75//AF042139
R-Y79AA1001781//Plasmodium falciparum chromosome 2, section 39 of 73 of the complete sequence.//1.0:414:57//AE001402
R-nnnnnnnnnnnn//Human DNA sequence *** SEQUENCING IN PROGRESS *** from clone 510D11, WORKING DRAFT SEQUENCE.//2.8e-05:329:61//Z98044
R-Y79AA1001827//Oryctolagus cuniculus PiUS mRNA, complete cds.//2.3e-90:557:89//U74297
R-Y79AA1001846//Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence.//2.1e-34:306:78//Z95152
R-Y79AA1001848//Sequence 11 from patent US 5449616.//1.0:221:59//I14369
R-Y79AA1001866//Arabidopsis thaliana genomic DNA, chromosome 5, TAC clone: K23L20, complete sequence.//0.0089:527:58//AB016874
R-Y79AA1001874
R-Y79AA1001875//M.musculus mRNA for Rab7 protein.//5.8e-45:170:92//X89650
R-Y79AA1001923//Human DNA sequence from clone 353H6 on chromosome Xq25-26.2. Contains the alternatively spliced SMARCA1 gene for SW1/SNF related, matrix associated, actin dependent regulator of chromatin, subfamily a, member 1 (SNF2L1) and a 40S Ribosomal Protein S26 pseudogene. Contains ESTs, STSs and GSSs, complete sequence.//1.0:138:68//AL022577
R-Y79AA1002027//Liverwort Marchantia polymorpha chloroplast genome DNA.//0.71:153:67//X04465
R-Y79AA1002083//Human DNA sequence *** SEQUENCING IN PROGRESS *** from clone 172B20, WORKING DRAFT SEQUENCE.//1.0:178:64//AL022319
R-Y79AA1002089//Homo sapiens clone GS111G14, WORKING DRAFT SEQUENCE, 5 unordered pieces.//6.3e-49:377:81//AC005011
R-Y79AA1002093//Homo sapiens (clone SEL366) 17q YAC (368C7) RNA.//4.0e-32:174:99//L77612
R-Y79AA1002103//CIT-HSP-2328I21.TR CIT-HSP Homo sapiens genomic clone 2328I21, genomic survey sequence.//1.9e-44:245:96//AQ044502
R-Y79AA1002115//CITBI-E1-2514F10.TF CITBI-E1 Homo sapiens genomic clone 2514F10, genomic survey sequence.//1.8e-24:249:78//AQ265752
R-Y79AA1002125//RPCI11-15J6.TV RPCI-11 Homo sapiens genomic clone RPCI-11-15J6, genomic survey sequence.//8.5e-21:147:91//B75354
R-Y79AA1002139
R-Y79AA1002204
R-nnnnnnnnnnnn//Human ankyrin G (ANK-3) mRNA, complete cds.//0.040:319:59//U13616
R-Y79AA1002209//Psilotum nudum RT gene for reverse transcriptase (PT4).//0.99:106:65//X65415
R-Y79AA1002210
R-Y79AA1002211//H.sapiens NGAL gene.//1.0:311:59//X99133
R-Y79AA1002220//Plasmodium falciparum DNA *** SEQUENCING IN PROGRESS *** from MAL4P1, WORKING DRAFT SEQUENCE.//5.9e-07:535:57//AL034557
R-Y79AA1002229
R-Y79AA1002234//Homo sapiens mRNA for KIAA0692 protein, partial cds.//6.1e-117:564:98//AB014592
R-Y79AA1002246
R-Y79AAl002258//Homo sapiens mRNA for HIP3, complete cds.//1.3e-92:453:97//AB013384
R-Y79AA1002298//HS_3071_B2_E08_MR CIT Approved Human Genomic Sperm Library D Homo sapiens genomic clone Plate=3071 Col=16 Row=J, genomic survey sequence.//1.9e-56:384:87//AQ171331
R-Y79AA1002307//Homo sapiens mRNA for KIAA0634 protein, partial cds.//2.5e-108:403:99//AB014534
R-Y79AA1002311//Homo sapiens chromosome 10 clone CIT987SK-1173I12 map 10q25, complete sequence.//1.1e-07:368:61//AC005887
R-Y79AA1002351
R-Y79AA1002361//H.sapiens CpG island DNA genomic Mse1 fragment, clone 65b9, reverse read cpg65b9.rt1a.//0.57:59:79//Z62206
R-Y79AA1002399//Homo sapiens chromosome 17, clone hRPK.700_H_6, complete sequence.//2.0e-98:385:99//AC005920
R-Y79AA1002407//Homo sapiens chromosome 17, clone hRPC.842_A_23, complete sequence.//5.4e-59:490:76//AC004662
R-Y79AA1002416//Homo sapiens Xp22 GSHB-314C4 (Genome Systems Human BAC library) complete sequence.//6.3e-08:103:80//AC004087
R-Y79AA1002431
R-nnnnnnnnnnnn//Mouse transcriptional control element.//0.064:84:71//M17284
R-Y79AA1002472//Homo sapiens chromosome 19, BAC CTY-B-393i15 (BC301323), complete sequence.//1.6e-103:525:96//AC006116
R-Y79AA1002482//Homo sapiens chromosome 18, clone hRPK.474_N_24, complete sequence.//9.7e-38:302:83//AC006238
R-Y79AA1002487//P.falciparum complete gene map of plastid-like DNA (IR-B).//0.23:266:61//X95276

### Homology Search Result Data 4.

The result of the homology search of the Human Unigene using the clone sequence of 5'-end.

Data include
the name of clone,
title of the top hit data,
the P-value: the length of the compared sequence: identity (%), and
the Accession No. of the top hit data, as in the order separated by //.

Data are not shown for the clones in which the P-value was higher than 1.
F-HEMBA1000005//EST//4.3e-87:422:97//Hs.147830:AI222069
F-HEMBA1000012//Human endosome-associated protein (EEA1) mRNA, complete cds//0.82:170:64//Hs.2864:L40157
F-HEMBA1000020//Homo sapiens beta 2 gene//4.0e-74:529:83//Hs.150244:U83668
F-HEMBA1000030//ESTs//1.1e-91:494:93//Hs.7958:W22078
F-HEMBA1000042//ESTs//3.5e-22:228:77//Hs.145406:AI253247
F-HEMBA1000046//ESTs, Highly similar to PRE-MRNA SPLICING FACTOR RNA HELICASE PRP22 [Saccharomyces cerevisiae]//0.00019:192:65//Hs.7900:W22411
F-HEMBA1000050//EST//0.81:74:72//Hs.156298:AI336759
F-HEMBA1000076//ESTs//0.11:252:62//Hs.131939:AI417910
F-HEMBA1000111//ESTs//8.5e-89:449:96//Hs.41105:N66734
F-HEMBA1000129//Human phosphatidylinositol 3-kinase catalytic subunit p110delta mRNA, complete cds//0.27:342:61//Hs.14207:U86453
F-HEMBA1000141//Homo sapiens mRNA for KIAA0797 protein, partial cds//6.8e-169:791:98//Hs.27197:AB018340
F-HEMBA1000150//Homo sapiens mRNA for KIAA0788 protein, partial cds//1.4e-37:243:88//Hs.2397:Z70200
F-HEMBA1000156//ESTs, Weakly similar to The KIAA0138 gene product is novel. [H.sapiens]//5.3e-80:383:98//Hs.135552:AI215187
F-HEMBA1000158//Homo sapiens OPA-containing protein mRNA, complete cds//2.1e-07:265:63//Hs.85313:AF071309
F-HEMBA1000168//ESTs//6.1e-35:257:85//Hs.13533:H23079
F-HEMBA1000180//ESTs, Moderately similar to RETROVIRUS-RELATED POL POLYPROTEIN [H.sapiens]//1.3e-18:111:96//Hs.163863:W28729
F-HEMBA1000185//H.sapiens ERF-2 mRNA//1.0:125:68//Hs.78909:U07802
F-HEMBA1000193//EST//1.5e-48:266:95//Hs.160642:AI240133
F-HEMBA1000201//Human Ini1 mRNA, complete cds//6.5e-75:440:92//Hs.155626:U04847
F-HEMBA1000213//ESTs//0.21:239:62//Hs.26838:AA527529
F-HEMBA1000216//Homo sapiens clone 23698 mRNA sequence//1.1e-57:529:68//Hs.8136:U81984
F-HEMBA1000227//Human RNA-binding protein CUG-BP/hNab50 (NAB50) mRNA, complete cds//1.3e-05:311:64//Hs.81248:U63289
F-HEMBA1000231
F-HEMBA1000243//EST//5.9e-52:359:85//Hs.141433:N23377
F-HEMBA1000244//H.sapiens mRNA for cytokine inducible nuclear protein//0.0022:350:60//Hs.74019:X83703
F-HEMBA1000251//ESTs//3.2e-84:443:95//Hs.21068:N47460
F-HEMBA1000264//ESTs//0.76:227:61//Hs.5159:AA588562
F-HEMBA1000280//EST//1.7e-12:149:75//Hs.103418:AA035568
F-HEMBA1000282//ESTs//1.7e-16:164:79//Hs.123111:AA813186
F-HEMBA1000288//ESTs//5.4e-06:154:68//Hs.54174:N64406
F-HEMBA1000290//Human novel homeobox mRNA for a DNA binding protein//3.8e-07:412:61//Hs.37035:U07664
F-HEMBA1000302//EST/1.2e-41:238:94//Hs.147245:AI206095
F-HEMBA1000303
F-HEMBA1000304//ESTs//3.5e-11:96:87//Hs.163057:AA728946
F-HEMBA1000307//EST//7.7e-05:280:62//Hs.146462:AI124898
F-HEMBA1000327//ESTs//5.3e-92:435:99//Hs.100605:AA305965
F-HEMBA1000333//Human mRNA for KIAA0206 gene, partial cds//0.84:395:56//Hs.79299:D86961
F-HEMBA1000338//ESTs, Moderately similar to novel stromal cell protein [M.musculus]//2.4e-38:317:80//Hs.99189:X84712
F-HEMBA1000351//Human Line-1 repeat mRNA with 2 open reading frames//0.020:334:59//Hs.23094:M19503
F-HEMBA1000355//Myosin, heavy polypeptide 11, smooth muscle//0.11:336:61//Hs.78344:AF001548
F-HEMBA1000356//H.sapiens ERF-2 mRNA//0.031:317:59//Hs.78909:U07802
F-HEMBA1000357//Human mRNA for KIAA0118 gene, partial cds//1.2e-50:441:78//Hs.154326:D42087
F-HEMBA1000366//ESTs//0.025 :56:87//Hs.141629:H74010
F-HEMBA1000369//Homo sapiens PAC clone DJ0669B10 from 7q33-q35//0.99:433:58//Hs.159899:AC004853
F-HEMBA1000376//Oxytocin receptor//3.4e-43:569:70//Hs.2820:X64878
F-HEMBA1000387//ESTs//8.2e-104:535:94//Hs.78110:AA741320
F-HEMBA1000390//Homo sapiens BAC clone RG119C02 from 7p15//2.3e-141:712:95//Hs.22900:AC004520
F-HEMBA1000392//Homo sapiens clone 24619 mRNA sequence//1.7e-47:461:74//Hs.139088:AF070533
F-HEMBA1000396//ESTs, Weakly similar to hypothetical protein [H.sapiens]//1.2e-26:351:70//Hs.138992:C14008
F-HEMBA1000411//EST//2.8e-27:401:71//Hs.138719:N52915
F-HEMBA1000418//ESTs//0.0094:375:61//Hs.40140:AI079253
F-HEMBA1000422//EST//6.2e-23:225:78//Hs.132635:A1032875
F-HEMBA1000428//Homo sapiens mRNA, chromosome 1 specific transcript KIAA0501//7.6e-31:616:66//Hs.159897:AB007970
F-HEMBA1000434//EST//0.0031:157:64//Hs.162328:AA559034
F-HEMBA1000442//EST//1.0:201:61//Hs.162434:AA577398
F-HEMBA1000456//Fanconi anemia complementation group C//0.58:362:59//Hs.37953:X66893
F-HEMBA1000459//EST//9.2e-21:157:86//Hs.132635:AI032875
F-HEMBA1000460//ESTs//2.9e-77:409:95//Hs.27135:W49590
F-HEMBA1000464//ESTs//6.6e-17:365:65//Hs.150675:AA127853
F-HEMBA1000469
F-HEMBA1000488//Homo sapiens HIV-1 inducer of short transcripts binding protein (FBI1) mRNA, complete cds//0.15:253:58//Hs.104640:AF000561
F-HEMBA1000490//Homo sapiens kinectin mRNA, complete cds//0.71:539:56//Hs.82709:Z22551
F-HEMBA1000491//ESTs//2.0e-21:361:65//Hs.152453:AA864970
F-HEMBA1000501//Homo sapiens tapasin (NGS-17) mRNA, complete cds//2.5e-39:312:77//Hs.5247:AF029750
F-HEMBA1000504//Homo sapiens mRNA for osteoblast specific factor 2 (OSF-2os)//1.3e-08:57:100//Hs.155095:D13666
F-HEMBA1000505//Homo sapiens KE04p mRNA, complete cds//1.0:197:62//Hs.131962:AF064093
F-HEMBA1000508//EST//0.67:156:60//Hs.162898:AA659646
F-HEMBA1000518
F-HEMBA1000519//EST//6.8e-52:300:91//Hs.149580:AI281881
F-HEMBA1000520//ESTs, Weakly similar to coded for by C. elegans cDNA CEESB82F [C.elegans]//2.9e-16:132:84//Hs.155871:AA533783
F-HEMBA1000523//ESTs, Highly similar to TESTIS-SPECIFIC PROTEIN PBS13 [Mus musculus]//2.1e-25:192:87//Hs.22383:R51067
F-HEMBA1000531//ESTs, Weakly similar to heat shock protein [H.sapiens]//2.4e-57:288:97//Hs.116022:AA455706
F-HEMBA1000534//Homo sapiens PYRIN (MEFV) mRNA, complete cds//2.8e-47:153:88//Hs.113283:AF018080
F-HEMBA1000540//ESTs//8.6e-07:60:100//Hs.109755:AA180809
F-HEMBA1000542//Human lysyl oxidase-like protein mRNA, complete cds//0.088:581:57//Hs.65436:U24389
F-HEMBA1000545//Human kpni repeat mrna (cdna clone pcd-kpni-4), 3' end//7.8e-106:731:83//Hs.139107:K00629
F-HEMBA1000555//Human mRNA for KIAA0242 gene, partial cds//0.75:283:58//Hs.77495:D87684
F-HEMBA1000557//ESTs//3.9e-27:389:71//Hs.125087:AA495729
F-HEMBA1000561//Homo sapiens mRNA for KIAA0760 protein, partial cds//3.8e-64:665:72//Hs.137168:AB018303
F-HEMBA1000563//ESTs//3.8e-51:257:98//Hs.47122:AI338977
F-HEMBA1000568//EST//0.12:270:61//Hs.134833 :AI091046
F-HEMBA1000569//H.sapiens mRNA encoding GPI-anchored protein p137//3.8e-19:409:62//Hs.119283:Z48042
F-HEMBA1000575//EST//0.060:156:64//Hs.126277:AA826681
F-HEMBA1000588//ESTs, Weakly similar to weakly similar to myosin heavy chain [C.elegans]//7.7e-41:217:96//Hs.55084:AA479162
F-HEMBA1000591//Homo sapiens mRNA for E1B-55kDa-associated protein//2.3e-44:228:97//Hs.155218:AJ007509
F-HEMBA1000592//ESTs, Weakly similar to sorting nexin 1 [H.sapiens]//1.7e-27:463:65//Hs.13794:AA203241
F-HEMBA1000594//Human clone 230971 defective mariner transposon Hsmar2 mRNA sequence//4.0e-68:574:79//Hs.159176:U92019
F-HEMBA1000604//ESTs//3.3e-21:158:74//Hs.142924:AI092535
F-HEMBA1000608//Homo sapiens mRNA for KIAA0456 protein, partial cds//3.7e-120:561:99//Hs.5003:AB007925
F-HEMBA1000622//Homo sapiens DEC-205 mRNA, complete cds//5.2e-34:592:68//Hs.153563:AF011333
F-HEMBA1000636//ESTs, Weakly similar to 50S RIBOSOMAL PROTEIN L20 [E.coli]//7.4e-22:166:84//Hs.26252:AA643235
F-HEMBA1000637//Homo sapiens mRNA for KIAA0690 protein, partial cds//2.1e-138:639:99//Hs.60103:AB014590
F-HEMBA1000655//ESTs//1.2e-54:503:77//Hs.140864:AA176174
F-HEMBA1000657//Mucin 1, transmembrane//0.99:219:61//Hs.89603:J05582
F-HEMBA1000662//ESTs//2.2e-52:257:99//Hs.63243:AI123912
F-HEMBA1000673//H.sapiens mRNA for translin associated protein X//1.7e-47:366:79//Hs.96247:X95073
F-HEMBA1000682//Oxytocin receptor//4.7e-59:673:72//Hs.2820:X64878
F-HEMBA1000686
F-HEMBA1000702
F-HEMBA1000705//EST//0.047:363:60//Hs.136379:AA521309
F-HEMBA1000719//ESTs//2.7e-68:333:98//Hs.146195:AI039850
F-HEMBA1000722//ESTs//0.49:283:60//Hs.21108:N92630
F-HEMBA1000726//EST//1.1e-45:183:87//Hs.149580:AI281881
F-HEMBA1000727//ESTs//4.8e-95:442:100//Hs.22119:AA885491
F-HEMBA1000747
F-HEMBA1000749//ESTs//8.0e-14:108:77//Hs.154892:AI091568
F-HEMBA1000752//EST//1.3e-25:344:69//Hs.160992:H52716
F-HEMBA1000769//ESTs//0.0018:206:63//Hs.153268:AA887239
F-HEMBA1000773//ESTs//0.56:336:58//Hs.105964:N35803
F-HEMBA1000774//EST//4.0e-38:312:79//Hs.162197:AA535216
F-HEMBA1000791//ESTs//2.8e-87:413:99//Hs.112050:AA431300
F-HEMBA1000817//ESTs//5.6e-124:617:96//Hs.101366:AA167536
F-HEMBA1000822//ESTs//0.94:347:58//Hs.23905:AA928542
F-HEMBA1000827//EST//0.064:133:60//Hs.138738:N58367
F-HEMBA1000843
F-HEMBA1000851//Fragile X mental retardation 1//0.014:219:62//Hs.89764:X69962
F-HEMBA1000852//Arylsulfatase D//6.7e-38:244:75//Hs.43887:X83572
F-HEMBA1000867
F-HEMBA1000869//ESTs//5.1 e-33:166:77//Hs.141186:R99609
F-HEMBA1000870//EST//0.032:130:66//Hs.157351:AI367237
F-HEMBA1000872//ESTs//2.4e-20:134:92//Hs.155982:AA406047
F-HEMBA1000876//EST//5.3e-20:233:72//Hs.124339:AA829660
F-HEMBA1000908//ESTs//5.4e-28:219:84//Hs.12247:AI203154
F-HEMBA1000910//Human DNA sequence from clone 14O9 on chromosome Xp11.1-11.4. Contains a Inter-Alpha-Trypsin Inhibitor Heavy Chain LIKE gene, a alternatively spliced Melanoma-Associated Antigen MAGE LIKE gene and a 6-Phosphofructo-2-kinase (Fructose-2,6-bisphosphatase) LIKE pseudogene. Contains ESTs, STSs and genomic marker DXS8032//2.8e-11:309:65//Hs.4943:Z98046
F-HEMBA1000918//ESTs//0.11:234:59//Hs.96499:AA252537
F-HEMBA1000919//Human mRNA for histone H1x, complete cds//0.18:221:64//Hs.109804:D64142
F-HEMBA1000934//Homo sapiens mRNA for KIAA0547 protein, complete cds//3.8e-09:360:62//Hs.36850:AB011119
F-HEMBA1000942//ESTs, Highly similar to PMS4 homolog mismatch repair protein [H.sapiens]//9.4e-10:77:93//Hs.111445:H00596
F-HEMBA1000943//ESTs, Highly similar to ZINC FINGER PROTEIN 10 [Homo sapiens]//0.0039:54:92//Hs.58338:AA609476
F-HEMBA1000946//Phosphoribosylglycinamide formyltransferase, phosphoribosylglycinamide-synthetase, phosphoribosylaminoimidazole synthetase//0.93:132:66//Hs.82285:X54199
F-HEMBA1000960//ESTs, Moderately similar to !!!! ALU SUBFAMILY SX WARNING ENTRY !!!! [H.sapiens]//0.080:128:71//Hs.118972:AA761369
F-HEMBA1000968//Human transposon-like element mRNA//2.8e-95:352:87//Hs.84775:M23161
F-HEMBA1000971//ESTs//8.4e-88:417:98//Hs.128631:AI127903
F-HEMBA1000972//EST//0.75:134:64//Hs.117228:AA682775
F-HEMBA1000974//ESTs//1.3e-103:497:98//Hs.126786:U74314
F-HEMBA1000975//Homo sapiens diacylglycerol kinase iota (DGKi) mRNA, complete cds//1.3e-05:424:59//Hs.159564:AF061936
F-HEMBA1000985//Homo sapiens mRNA, chromosome 1 specific transcript KIAA0492//0.0036:389:60//Hs.127338:AB007961
F-HEMBA1000986//ESTs//0.00025:272:64//Hs.12364:H09132
F-HEMBA1000991//Homo sapiens mRNA for Hrs, complete cds//3.9e-24:193:84//Hs.24756:U43895
F-HEMBA1001007//EST//0.96:70:71//Hs.163258:AA828835
F-HEMBA1001008//Human G protein-coupled receptor (STRL22) mRNA, complete cds//4.9e-43:472:74//Hs.46468:U45984
F-HEMBA1001009//Immunoglobulin mu//0.18:367:59//Hs.75758:X58529
F-HEMBA1001017//Homo sapiens mRNA for KIAA0468 protein, complete cds//1.4e-140:661:98//Hs.158287:AB007937
F-HEMBA1001019//EST//4.1e-14:251:68//Hs.148769:AI239572
F-HEMBA1001020//Von Hippel-Lindau syndrome//2.2e-28:253:69//Hs.78160:AF010238
F-HEMBA1001022
F-HEMBA1001024//Homo sapiens mRNA, chromosome 1 specific transcript KIAA0501//6.8e-28:376:72//Hs.159897:AB007970
F-HEMBA1001026//Homo sapiens klotho mRNA, complete cds//1.3e-05:745:57//Hs.94592:AB005142
F-HEMBA1001043//ESTs//2.1e-28:448:67//Hs.112469:AA598515
F-HEMBA1001051//EST//3.1e-48:310:87//Hs.149580:AI281881
F-HEMBA1001052//EST//0.94:149:67//Hs.312l6:AI017971
F-HEMBA1001059//N-ACETYLGALACTOSAMINE-6-SULFATASE PRECURSOR//4.6e-165:777:98//Hs.159479:U06088
F-HEMBA1001060//ESTs//6.8e-14:150:78//Hs.24821:AA044813
F-HEMBA1001071//Alpha-1 type 3 collagen//3.5e-32:181:96//Hs.119571:X14420
F-HEMBA1001077//ESTs, Moderately similar to transcription intermediary factor 1 [H.sapiens]//1.1e-98:487:97//Hs.147802:R71297
F-HEMBA1001080//Human N-type calcium channel alpha-1 subunit mRNA, complete cds//0.013:385:58//Hs.69949:M94172
F-HEMBA1001085//Human hSIAH2 mRNA, complete cds//0.55:338:59//Hs.20191:U76248
F-HEMBA1001088//Human PINCH protein mRNA, complete cds//7.3e-73:303:78//Hs.83987:U09284
F-HEMBA1001094//Interleukin 8//0.092:530:58//Hs.624:M17017
F-HEMBA1001099
F-HEMBA1001109//Homo sapiens tapasin (NGS-17) mRNA, complete cds//2.4-61:341:85//Hs.5247:AF029750
F-HEMBA1001121//EST//7.3e-13:265:64//Hs.142423:AA412497
F-HEMBA1001122//Homo sapiens mRNA for KIAA0471 protein, complete cds//0.066:649:56//Hs.5347:AB007940
F-HEMBA1001123//Homo sapiens mRNA for KIAA0448 protein, complete cds//1.5e-10:231:68//Hs.27349:AB007917
F-HEMBA1001133//EST//0.50:222:63//Hs.131018:AI015747
F-HEMBA1001137//Homo sapiens mRNA for KIAA0798 protein, complete cds//2.2e-73:527:77//Hs.159277:AB018341
F-HEMBA1001140//Homo sapiens mRNA for KIAA0682 protein, complete cds//0.020:141:65//Hs.7482:AB014582
F-HEMBA1001172//EST//0.77:158:60//Hs.158894:AI378457
F-HEMBA1041174//ESTs//1.4e-63:363:92//Hs.132798:AA922226
F-HEMBA1001197//ESTs, Weakly similar to Rap2 interacting protein 8 [M.musculus]//5.0e-54:555:71//Hs.55165:AA573499
F-HEMBA1001208//EST//6.2e-26:213:77//Hs.146964:AI183463
P-HEMBA1001213//Human mRNA for KIAA0013 gene, complete cds//0.026:569:57//Hs.48824:D87717
F-HEMBA1001226//ESTs/1.9e-11:407:65//Hs.157977:AI369694
F-HEMBA1001235//ESTs//0.0042:161:63//Hs.155170:AA167748
F-HEMBA1001247//ESTs//1.2e-91:429:99//Hs.143304:AI084058
F-HEMBA1001257//Human zinc finger protein (MAZ) mRNA//0.017:330:62//Hs.7647:M94046
F-HEMBA1001265
F-HEMBA1001281
F-HEMBA1001286//Natriuretic peptide precursor B//0.76:163:63//Hs.937:AL021155
F-HEMBA1001289//Homo sapiens Chromosome 16 BAC clone CIT987SK-A-69G12//5.1e-30:530:64//Hs.154050:AC004131
F-HEMBA1001294//Homo sapiens mRNA for matrilin-3//0.00023:657:56//Hs.119534:AJ224741
F-HEMBA1001299//Small inducible cytokine A5 (RANTES)//2.2e-27:271:77//Hs.155464:AF088219
F-HEMBA1001302//ESTs, Moderately similar to Cab45a [M.musculus]//3.3e-53:272:97//Hs.154563:AI129590
F-HEMBA1001303//ESTs, Weakly similar to RNA splicing-related protein [R.norvegicus]//2.6e-66:241:99//Hs.120847:AA731201
F-HEMBA1001310//ESTs//2.0e-21:133:93//Hs.159116:W55873
F-HEMBA1001319//Homo sapiens mRNA for KIAA0758 protein, partial cds//0.23:562:58//Hs.22039:AB018301
F-HEMBA1001323//Wingless-type MMTV integration site 5A, human homolog//2.5e-31:165:99//Hs.152213:L20861
F-HEMBA1001326//ESTs, Highly similar to HYPOTHETICAL 55.1 KD PROTEIN IN FAB1-PES4 INTERGENIC REGION [Saccharomyces cerevisiae]//8.9e-08:185:68//Hs.108734:AI073427
F-HEMBA1001327//ESTs//0.085:337:60//Hs.114157:AA703013
F-HEMBA1001330//EST//0.0018:225:63//Hs.127987:AA970569
F-HEMBA1001351//Homo sapiens VAMP-associated protein of 33 kDa (VAP-33) mRNA, complete cds//3.6e-105:516:97//Hs.9006:AF057358
F-HEMBA1001361//ESTs//1.2e-62:317:97//Hs.6639:R39794
F-HEMBA1001375//ESTs//0.93:180:60//Hs.148425 :AI198074
F-HEMBA1001377//ESTs//9.2e-87:414:99//Hs.48469:N62156
F-HEMBA1001383//ESTs//0.0023:336:60//Hs.140622:AA844353
F-HEMBA1001387//ESTs, Highly similar to RAS-LIKE PROTEIN TC10 [Homo sapiens]//1.0e-132:643:97//Hs.124217:AA020848
F-HEMBA1001388
F-HEMBA1001391//ESTs//5.6e-32:191:93//Hs.71628:N41660
F-HEMBA1001398
F-HEMBA1001405//EST//1.0:135:63//Hs.146833:AI151117
F-HEMBA1001407//ESTs/10.53:390:57//Hs.150447:AI017798
F-HEMBA1001411//EST//8.8e-06:270:62//Hs.145386:AI253108
F-HEMBA1001413
F-HEMBA1001415//EST//1.3e-12:176:75//Hs.133172:AI051605
F-HEMBA1001432//RING3 PROTEIN//0.57:345:59//Hs.75243:D42040
F-HEMBA1001433//ESTs//1.3e-21:333:69//Hs.131648:AI025726
F-HEMBA1001435//Cytochrome P450, subfamily I (aromatic compound-inducible), polypeptide 2//1.2e-74:469:80//Hs.1361:M55053
F-HEMBA1001442//EST//0.29:181:64//Hs.116883:AA663031
F-HEMBA1001446//ESTs, Weakly similar to Rap2 interacting protein 8 [M.musculus]//6.8e-47:550:71//Hs.55165:AA573499
F-HEMBA1001450//Homo sapiens GTPase-activating protein (SIPA1) mRNA, complete cds//0.82:312:58//Hs.7019:AB005666
F-HEMBA1001454//ESTs//1.2e-46:297:80//Hs.152395:AA533107
F-HEMBA1001455//ESTs//7.3e-103:502:97//Hs.112860:AA442412
F-HEMBA1001463//Human mRNA for KIAA0392 gene, partial cds//8.7e-51:323:88//Hs.40100:AB002390
F-HEMBA1001476//Homo sapiens mRNA for KIAA0572 protein, partial cds//6.2e-104:489:99//Hs.14409:AB011144
F-HEMBA1001478//EST//0.013:205:61//Hs.157309:AI365451
F-HEMBA1001497//Small inducible cytokine A5 (RANTES)//5.9e-45:307:84//Hs.155464:AF088219
F-HEMBA1001510//H.sapiens mRNA for G13 protein//2.1e-71:405:92//Hs.42853:X98054
F-HEMBA1001515//Human Line-1 repeat mRNA with 2 open reading frames//4.5e-105:773:82//Hs.23094:M19503
F-HEMBA1001517//EST//3.6e-09:271:65//Hs.162347:AA564902
F-HEMBA1001522//ESTs//4.3e-13:85:95//Hs.126707:AI376869
F-HEMBA1001526
F-HEMBA1001533//EST//1.0:75:73//Hs.145360:AI252476
F-HEMBA1001557//EST//3.5e-13:261:64//Hs.161496:N66580
F-HEMBA1001566//EST//3.7e-07:354:64//Hs.43830:N26652
F-HEMBA1001569//Homo sapiens mRNA for vesicle associated membrane protein 2 (VAMP2)//8.0e-68:338:97//Hs.91589:M36205
F-HEMBA1001570//ESTs//1.5e-47:369:82//Hs.107657:AA126814
F-HEMBA1001579//Homo sapiens mRNA for NS1-binding protein (NS1-BP)//7.0e-175:678:99//Hs.159597:AJ012449
F-HEMBA1001581//ESTs//4.4e-07:237:67//Hs.152304:AA605184
F-HEMBA1001585//ESTs//1.1e-11:81:100//Hs.16364:AI357228
F-HEMBA1001589//Human mRNA for KIAA0166 gene, complete cds//0.82:210:64//Hs.115778:D79988
F-HEMBA1001595//Human mRNA for KIAA0128 gene, partial cds//2.6e-110:855:78//Hs.90998:D50918
F-HEMBA1001608//EST//1.0:201:60//Hs.136747:AA749210
F-HEMBA1001620//ESTs//1.5e-39:211:98//Hs.131063:AI016400
F-HEMBA1001635//ESTs//4.0e-33:168:100//Hs.122655:AI361870
F-HEMBA1001636//ESTs, Moderately similar to !!!! ALU SUBFAMILY SP WARNING ENTRY !!!! [H.sapiens]//0.038:198:64//Hs.34579:AI338536
F-HEMBA1001640//ESTs//1.1e-24:315:71//Hs.34114:AA776899
F-HEMBA1001647//Human plectin (PLEC1) mRNA, complete cds//0.00049:629:61//Hs.79706:U53204
F-HEMBA1001651//EST//3.6e-07:285:63//Hs.132558:AA948560
F-HEMBA1001655//ESTs//1.4e-95:497:96//Hs.59563:AA203283
F-HEMBA1001658//EST//0.18:251:59//Hs.117724:H47121
F-HEMBA1001661
F-HEMBA1001672//Homo sapiens methyl-CpG binding protein MBD3 (MBD3) mRNA, complete cds//7.9e-146:669:99//Hs.107254:AC005943
F-HEMBA1001675//Homo sapiens mRNA, chromosome 1 specific transcript KIAA0484//2.0e-57:447:79//Hs.158095:AB007953
F-HEMBA1001678//ESTs//4.0e-50:360:83//Hs.146811:AA410788
F-HEMBA1001681//EST//1.0:165:58//Hs.136790:AA776060
F-HEMBA1001702//EST//0.015:312:61//Hs.162839:AA648760
F-HEMBA1001709//EST//0.85:131:65//Hs.131451:AI023995
F-HEMBA1001711//ESTs//0.084:425:56//Hs.125346:AI302836
F-HEMBA1001712//EST//0.26:214:59//Hs.159088:AI383114
F-HEMBA1001714//ESTs, Highly similar to ATPASE INHIBITOR, MITOCHONDRIAL PRECURSOR [Rattus norvegicus]//3.0e-30:195:92//Hs.132948:AA194452
F-HEMBA1001718//EST//0.0044:275:60//Hs.125969:AA889554
F-HEMBA1001723//INTERLEUKIN ENHANCER-BINDING FACTOR//0.24:501:57//Hs.101524:U58197
F-HEMBA1001731//EST//1.2e-06:261:63//Hs.132331:AI028363
F-HEMBA1001734//ESTs//0.018:177:63//Hs.129631:AI000415
F-HEMBA1001744//EST//8.7e-77 :420:92//Hs.133226:AI052250
F-HEMBA1001745//Homo sapiens mRNA for TSC403 protein, complete cds//0.37:300:62//Hs.10887:AB013924
F-HEMBA100l746//ESTs//0.31:168:66//Hs.27237:N68328
F-HEMBA1001761//ESTs, Weakly similar to ZINC FINGER PROTEIN 91 [H.sapiens]//0.76:218:60//Hs.135553:N41598
F-HEMBA1001781//Homo sapiens chromosome 19, cosmid R30953//0.98:219:60//Hs.98776:AC005622
F-HEMBA1001784//Homo sapiens mRNA for KJAA0474 protein, complete cds//6.4e-09:265:67//Hs.158232:AB007943
F-HEMBA1001791
F-HEMBA1001800//EST//3.1e-41:331:81//Hs.127142:AA937570
F-HEMBA1001803//EST//0.0062:269:59//Hs.49075:N64817
F-HEMBA1001804//Human POU domain protein (Brn-3b) mRNA, complete cds//1.8e-07:439:59//Hs.266:U06233
F-HEMBA1001808//Homo sapiens mRNA, chromosome 1 specific transcript KIAA0500//2.-5e-175:809:98//Hs.118164:AB007969
F-HEMBA1001809//ESTs//6.0e-101:497:97//Hs.155127:AA625305
F-HEMBA1001815
F-HEMBA1001819//Human kruppel-related zinc finger protein (ZNF184) mRNA, partial cds//4.9e-80:842:70//Hs.158174:U66561
F-HEMBA1001820//EST//0.057:214:62//Hs.148715:A1223845
F-HEMBA1001822//Homo sapiens intersectin short form mRNA, complete cds//6.7e-42:510:65//Hs.66392:AF064244
F-HEMBA1001824//Homo sapiens OPA-containing protein mRNA, complete cds//5.2e-13:253:68//Hs.85313:AF071309
F-HEMBA1001835//Human mRNA for KIAA0235 gene, partial cds//0.96:288:60//Hs.6151:D87078
F-HEMBA1001844//ESTs//1.1e-29:197:80//Hs.l55243:N70293
F-HEMBA1001847//Human mRNA for KIAA0326 gene, partial cds//2.0e-23:379:68//Hs.6833:AB002324
F-HEMBA1001861//Homo sapiens mRNA for KIAA0617 protein, complete cds//2.8e-185:865:98//Hs.78946:AB014517
F-HEMBA1001864//EST//0.27:145:63//Hs.162585:AA593121
F-HEMBA1001866//ESTs. Weakly similar to UDP-GLUCOSE:GLYCOPROTEIN GLUCOSYLTRANSFERASE PRECURSOR [D.melanogaster]//3.2e-39:293:84//Hs.152332:AI141922
F-HEMBA1001869//ESTs, Weakly similar to ASH1 [D.melanogaster]//8.1e-70:367:95//Hs.15423:T84036
F-HEMBA1001888//Homo sapiens mRNA, chromosome 1 specific transcript KIAA0484//5.4e-86:835:76//Hs.158095:AB007953
F-HEMBA1001896
F-HEMBA1001910//Human calpain-like protease (htra-3) mRNA, complete cds//0.43:114:71//Hs.6133:U94346
F-HEMBA1001912//ESTs//4.1e-79:398:97//Hs.26660:AI312633
F-HEMBA1001913//Homo sapiens TNF-alpha stimulated ABC protein (ABC50) mRNA, complete cds//0.00031:200:62//Hs.9573:AF027302
F-HEMBA1001915//EST//0.082:128:64//Hs.126542:AA916511
F-HEMBA1001918//Homo sapiens SEC63 (SEC63) mRNA, complete cds//0.46:374:59//Hs.31575:AF100141
F-HEMBA1001921//Homo sapiens germinal center kinase related protein kinase mRNA, complete cds//6.7e-186:855:99//Hs.154934:AF000145
F-HEMBA1001939//ESTs//4.9e-34:342:77//Hs.132711:AI377295
F-HEMBA1001940//ESTs//8.6e-15:149:81//Hs.141129:R86221
F-HEMBA1001942//ESTs//0.0014:271:62//Hs.124514:AI219882
F-HEMBA1001945//EST//0.98:142:64//Hs.161540:N85943
F-HEMBA1001950//ESTs//0.99:188:64//Hs.28639:R78360
F-HEMBA1001960//Homo sapiens methyl-CpG binding protein MBD2 (MBD2) mRNA, complete cds//0.30:85:69//Hs.25674:AF072242
F-HEMBA1001962//ESTs//0.0012:289:59//Hs.125492:AA938930
F-HEMBA1001964//EST//0.73:153:64//Hs.112161:AA477708
F-HEMBA1001967//Human DNA sequence from clone 341E18 on chromosome 6p11.2-12.3. Contains a Serine/Threonine Protein Kinase gene (presumptive isolog of a Rat gene) and a novel alternatively spliced gene. Contains a putative CpG island, ESTs and GSSs//4.6e-156:720:99//Hs.11050:AL031178
F-HEMBA1001979//ESTs//0.86:184:67//Hs.77208:AA044732
F-HEMBA1001987//ESTs, Moderately similar to hTAFII68 [H.sapiens]//2.8e-29:151:100//Hs.124106:AA948100
F-HEMBA1001991//Homo sapiens clone 24540 mRNA sequence//0.049:121:70//Hs.153529:AF070581
F-HEMBA1002003//Keratin 10 (epidermolytic hyperkeratosis; keratosis palmaris et plantaris)//9.8e-09:294:63//Hs.99936:X14487
F-HEMBA1002008//ESTs//0.12:299:59//Hs.132803 :W63582
F-HEMBA1002018//PROTEIN-TYROSINE PHOSPHATASE ZETA PRECURSOR//0.98:212:64//Hs.78867:M93426
F-HEMBA1002022//Human p37NB mRNA, complete cds//0.00044:58:96//Hs.155545:U32907
F-HEMBA1002035//EST//6.4e-07:145:68//Hs.135336:AI049827
F-HEMBA1002039//EST//0.99:79:67//Hs.98451:AA426057
F-HEMBA1002049//ESTs, Weakly similar to !!!! ALU SUBFAMILY J WARNING ENTRY !!!! [H.sapiens]//4.5e-26:223:81//Hs.105292:AA504776
F-HEMBA1002084
F-HEMBA1002092
F-HEMBA1002100//Homo sapiens zinc finger homeodomain protein (ATBF1-A) mRNA, complete cds//5.6e-21:124:96//Hs.101842:L32832
F-HEMBA1002102//ESTs, Highly similar to ANKYRIN [Mus musculus]//5.9e-09:434:62//Hs.135102:AI190276
F-HEMBA1002113//ESTs//0.049:255:63//Hs.106137:AI129973
F-HEMBA1002119
F-HEMBA1002125//H.sapiens ERF-2 mRNA//0.026:341:59//Hs.78909:U07802
F-HEMBA1002139//ESTs//0.082:309:60//Hs.36383:W52393
F-HEMBA1002144//Human mRNA for KIAA0227 gene, partial cds//5.6e-06:601:60//Hs.79170:D86980
F-HEMBA1002150//Homo sapiens mRNA for KIAA0720 protein, partial cds//5.6e-06:353:62//Hs.23741:AB018263
F-HEMBA1002151
F-HEMBA1002153//EST/10.014:328:60//Hs.149115:AI244695
F-HEMBA1002160//Homo sapiens mRNA, chromosome 1 specific transcript KIAA0507//5.6e-49:303:79//Hs.158241:AB007976
F-HEMBA1002161//Myosin, heavy polypeptide 7, cardiac muscle, beta//1.2e-40:616:67//Hs.929:M57965
F-HEMBA1002162//Homo sapiens mRNA for XPR2 protein//3.4e-48:749:67//Hs.44766:AJ007590
F-HEMBA1002166//Small inducible cytokine A5 (RANTES)//2.1e-60:485:79//Hs.155464:AF088219
F-HEMBA1002177//Homo sapiens yotiao mRNA, complete cds//2.4e-19:151:86//Hs.114808:AF026245
F-HEMBA1002185//EST//0.00011:233:65//Hs.125552:AA884141
F-HEMBA1002189//EST//5.1 e-24:193:81//Hs.163161:AA778363
F-HEMBA1002191//Homo sapiens mRNA for KIAA0689 protein, partial cds//0.27:382:59//Hs.21992:AB014589
F-HEMBA1002199//Homo sapiens mRNA, chromosome 1 specific transcript KIAA0501//1.2e-14:199:72//Hs.159897:AB007970
F-HEMBA1002204//ESTs//0.46:312:59//Hs.61210:AA024696
F-HEMBA1002212//ESTs//1.0:191:63//Hs.149752:AI285767
F-HEMBA1002215//ESTs, Highly similar to TESTIN 2 PRECURSOR [Mus musculus]//1.6e-47:251:96//Hs.59906:AA001281
F-HEMBA1002226//Homo sapiens mRNA, chromosome 1 specific transcript KIAA0488//2.4e-57:375:71//Hs.67619:AB007957
F-HEMBA1002229//Homo sapiens KIAA0395 mRNA, partial cds//7.9e-47:377:80//Hs.43681:AL022394
F-HEMBA1002237//EST//0.044:1 37:66//Hs.144448:AA812455
F-HEMBA1002241
F-HEMBA1002253//EST//2.2e-41:219:96//Hs.137065:AA888887
F-HEMBA1002257//Homo sapiens diacylglycerol kinase iota (DGKi) mRNA, complete cds//1.1e-152:731:97//Hs.159564:AF061936
F-HEMBA1002265//ESTs//5.4e-11:337:65//Hs.112639:AI125420
F-HEMBA1002267//Homo sapiens GDP-L-fucose pyrophosphorylase (GFPP) mRNA, complete cds//1.0:395:60//Hs.150926:AF017445
F-HEMBA1002270//ESTs//2.5e-87:504:89//Hs.124440:H95404
F-HEMBA1002321//Homo sapiens oxidized low-density lipoprotein receptor mRNA, complete cds//0.17:338:60//Hs.77729:AB010710
F-HEMBA1002328//ESTs//7.9e-103:480:99//Hs.123318:AI201982
F-HEMBA1002337//Human mRNA for KIAA0118 gene, partial cds//0.93:220:61//Hs.154326:D42087
F-HEMBA1002341//Homo sapiens mRNA for KIAA0771 protein, partial cds//7.8e-187:872:98//Hs.6162:AB018314
F-HEMBA10023481/EST//1.0e-19:285:70/Ms.121860:AA776692
F-HEMBA1002349//EST//0.011:385:59//Hs.148533:AI200996
F-HEMBA1002363//Homo sapiens chromosome-associated protein-E (hCAP-E) mRNA, complete cds//2.4e-189:872:99//Hs.119023:AF092563
F-HEMBA1002381//EST//7.9e-34:236:77//Hs.162197:AA535216
F-HEMBA1002389//ESTs//4.3e-59:342:92//Hs.133391:AA535144
F-HEMBA1002417//Homo sapiens chromosome 19, cosmid R28784//2.2e-159:775:97//Hs.25527:AC005954
F-HEMBA1002419//EST, Moderately similar to ROD CGMP-SPECIFIC 3',5'-CYCLIC PHOSPHODIESTERASE BETA-SUBUNIT [H.sapiens]//1.0:144:65//Hs.136096:W27141
F-HEMBA1002430//Human clone 23695 mRNA sequence//2.7e-06:563:59//Hs.90798:U79289
F-HEMBA1002439//EST, Weakly similar to LINE-1 REVERSE TRANSCRIPTASE HOMOLOG [H.sapiens]//0.11:111:67//Hs.162154:AA528561
F-HEMBA1002458//ESTs, Weakly similar to hypothetical protein B, 6.8K [H.sapiens]//1.3e-71:346:98//Hs.136121:W26490
F-HEMBA1002460//ESTs//2.1e-94:484:96//Hs.106441:R53160
F-HEMBA1002462//Homo sapiens N-methyl-D-aspartate receptor 2D subunit precursor (NMDAR2D) mRNA, complete cds//0.00024:240:64//Hs.113286:U77783
F-HEMBA1002469//Human mRNA for KIAA0122 gene, partial cds//1.3e-109:603:92//Hs.154583:D50912
F-HEMBA1002475//RYANODINE RECEPTOR, SKELETAL MUSCLE//0.025:261:63//Hs.89631:U48508
F-HEMBA1002477//Homo sapiens mRNA for KIAA0561 protein, partial cds//2.8e-45:331:83//Hs.6189:AB011133
F-HEMBA1002486//EST//0.00039:174:67//Hs.96680:AA303235
F-HEMBA1002495
F-HEMBA1002498//ESTs//1.2e-91:460:97//Hs.118327:W79161
F-HEMBA1002503//H.sapiens mRNA for MACH-alpha-2 protein//4.8e-13:164:74//Hs.19949:X98173
F-HEMBA1002508//Homo sapiens PYRIN (MEFV) mRNA, complete cds//6.1e-79:460:83//Hs.113283:AF018080
F-HEMBA1002513//Homo sapiens mRNA for histone deacetylase-like protein (JM21)//9.Oe-159:738:98//Hs.6764:AJ011972
F-HEMBA1002515//ESTs//3.6e-08:185:69//Hs.118701:AA420795
F-HEMBA1002538//ESTs//0.97:68:73//Hs.134672:AI087951
F-HEMBA1002542//Homo sapiens mRNA for chemokine LEC precursor, complete cds//6.1e-46:238:87//Hs.10458:AF088219
F-HEMBA1002547//Homo sapiens agrin precursor mRNA, partial cds//1.1e-138:655:98//Hs.68900:AF016903
F-HEMBA1002552//Human Hep27 protein mRNA, complete cds//2.8e-08:173:68//Hs.102137:U31875
F-HEMBA1002555//Homo sapiens mRNA for APC 2 protein, complete cds//0.00020:603:57//Hs.20912:AB012162
F-HEMBA1002558//ESTs//6.0e-25:262:77//Hs.136304:AA431205
F-HEMBA1002561//Humanclone 23574 mRNA sequence//4.7e-17:268:72//Hs.79385:U90905
F-HEMBA1002569//Homo sapiens protein associated with Myc mRNA, complete cds//4.3e-142:457:99//Hs.151411:AF075587
F-HEMBA1002583//Homo sapiens UKLF mRNA for ubiquitous Kruppel like factor, complete cds//2.8e-30:156:100//Hs.32170:AB015132
F-HEMBA1002590//ESTs//1.0e-30:277:77//Hs.139158:AA226159
F-HEMBA1002592//ESTs//2.4e-20:233:75//Hs.159329:AI378363
F-HEMBA1002609//Homo sapiens mRNA for KIAA0597 protein, partial cds//1.4e-176:820:99//Hs.20141:AB011169
F-HEMBA1002621//EST//0.99:208:60//Hs.159127:AI384013
F-HEMBA1002624//Homo sapiens mRNA for KIAA0808 protein, complete cds//9.2e-189:632:97//Hs.91338:AB018351
F-HEMBA1002628//Human mRNA for KIAA0336 gene, complete cds//0.079:231:65//Hs.125129:AB002334
F-HEMBA1002629//Human density enhanced phosphatase 1 mRNA, complete cds//1.3e-07:473:61//Hs.1177:U10886
F-HEMBA1002645//ESTs//2.6e-32:209:88//Hs.141323:N80390
F-HEMBA1002651
F-HEMBA1002659//Human vascular endothelial growth factor related protein VRP mRNA, complete cds//0.74:223:60//Hs.79141:U43142
F-HEMBA1002661//Human Line-1 repeat mRNA with 2 open reading frames//1.4e-122:781:85//Hs.23094:M19503
F-HEMBA1002666//ESTs//0.39:117:65//Hs.3794:T08497
F-HEMBA1002678//EST//0.0081:148:64//Hs.156768:AI351368
F-HEMBA1002679//Cyclic nucleotide gated channel (photoreceptor), cGMP gated 1 (alpha)//0.00096:418:61//Hs.1323:S42457
F-HEMBA1002688//Homo sapiens hyperpolarization-activated channel 1 (IH1) mRNA, partial cds//1.8e-11:541:601/Hs.124161:AF065164
F-HEMBA10026961/Homo sapiens DNA from chromosome 19, cosmid R29144//1.9e-06:345:61//Hs.155647:AC004221
F-HEMBA1002703//Homo sapiens mRNA for KIAA0455 protein, complete cds//6.0e-12:327:62//Hs.13245:AB007924
F-HEMBA1002712
F-HEMBA1002716//EST//1.2e-56:284:97//Hs.131329:AA922800
F-HEMBA1002728//Homo sapiens mRNA for KIAA0621 protein, partial cds//3.7e-127:614:97//Hs.132942:AB014521
F-HEMBA1002730//Homo sapiens microsomal glutathione S-transferase 3 (MGST3) mRNA, complete cds//0.21:157:66//Hs.111811 :AB007867
F-HEMBA1002742//EST//0.97:138:60//Hs.160545:71596
F-HEMBA1002746//Human HOX4C mRNA for a homeobox protein//0.72:347:58//Hs.74061:X59372
F-HEMBA1002748//ESTs, Weakly similar to C27H6.5 [C.elegans]//0.24:83:74//Hs.40806:AA018786
F-HEMBA1002750//ESTs//5.8e-37:185:76//Hs.140577:AA827817
F-HEMBA1002768//Homo sapiens mRNA for KIAA0554 protein, partial cds//2.9e-178:834:98//Hs.74750:AB011126
F-HEMBA1002770//ESTs, Highly similar to TIP120 [R.norvegicus]//8.0e-98:492:96//Hs.11833:AI299947
F-HEMBA1002777//Homo sapiens prostate apoptosis response protein par-4 mRNA, complete cds//3.9e-05:528:59//Hs.128208:U63809
F-HEMBA1002779//ESTs//8.1e-134:662:96//Hs.107295:W80392
F-HEMBA1002780//ESTs//3.8e-41:421:74//Hs.141576:N90326
F-HEMBA1002794//Protein kinase C, mu//4.8e-06:244:67//Hs.2891:X75756
F-HEMBA1002801//ESTs//2.1e-24:182:87//Hs.124633:AA856938
F-HEMBA1002810//Homo sapiens formin binding protein 21 mRNA, complete cds//3.4e-169:820:97//Hs.28307:AF071185
F-HEMBA1002816//ESTs//2.5e-91:387:94//Hs.8008:R52744
F-HEMBA1002818//Homo sapiens UPH1 (UPH1) mRNA, complete cds//7.0e-122:733:89//Hs.6059:AF093119
F-HEMBA1002826//ESTs//0.00015:235:62//Hs.119383:M279904
F-HEMBA1002833
F-HEMBA1002850//EST//0.0014:201:65//Hs.156235:AA770550
F-HEMBA1002863//ESTs//1.2e-50:295:91//Hs.57980:W68823
F-HEMBA1002876//ESTs, Weakly similar to HYPOTHETICAL 26.4 KD PROTEIN EEED8.8 IN CHROMOSOME II [C.elegans]//4.9e-18:110:94//Hs.13322:AA151730
F-HEMBA1002886//EST//0.99:184:65//Hs.160684:AE79429
F-HEMBA1002896//ESTs//2.1e-11:72:100//Hs.149215:AI051679
F-HEMBA1002921
F-HEMBA1002924//EST//3.7e-05:291:64//Hs.134677:AI088001
F-HEMBA1002934//ESTs//2.3e-42:324:80//Hs.141658:N77915
F-HEMBA1002935//Homo sapiens mRNA for KIAA0576 protein, partial cds//1.6e-174:803:99//Hs.14687:AB011148
F-HEMBA1002937//ESTs, Weakly similar to homologous to mouse gene PC326:GenBank Accession Number M95564 [H.sapiens]//8.1e-36:256:85//Hs.36899:AA130053
F-HEMBA1002939//H.sapiens mRNA for cytokine inducible nuclear protein//1.1e-05:479:59//Hs.74019:X83703
F-HEMBA1002944//Human putative endothelin receptor type B-like protein mRNA, complete cds//0.83:326:58//Hs.27747:U87460
F-HEMBA1002951//ESTs//6.1e-08:137:70//Hs.26762:AA913925
F-HEMBA1002954//ESTs//9.3e-39:249:89//Hs.146185:R19099
F-HEMBA1002968//ESTs//0.73:142:64//Hs.136371:AA506092
F-HEMBA1002970//EST//2.9e-10:103:82//Hs.162580:AA593828
F-HEMBA1002971//ESTs//3.5e-21:190:81//Hs.61170:AA454219
F-HEMBA1002973//Phosphodiesterase 4B, cAMP-specific (dunce (Drosophila)-homolog phosphodiesterase E4)//1.5e-37:247:89//Hs.188:L20971
F-HEMBA1002997//Homo sapiens chromosome-associated protein-C (hCAP-C) mRNA, partial cds//1.7e-05:797:58//Hs.50758:AF092564
F-HEMBA1002999//EST//9.9e-38:453:70//Hs.161635:W22525
F-HEMBA1003021//Small inducible cytokine A5 (RANTES)//4.6e-49:373:81//Hs.155464:AF088219
F-HEMBA1003033//ESTs//5.0e-64:340:95//Hs.154270:N26486
F-HEMBA1003034//Homo sapiens PYRIN (MEFV) mRNA, complete cds//7.4e-70:330:78//Hs.113283:AF018080
F-HEMBA1003035//Homo sapiens mRNA for testican-3//0.041:623:57//Hs.159425:AJ001454
F-HEMBA1003037//EST//0.53:59:74//Hs.148011:M268003
F-HEMBA1003041//ESTs, Weakly similar to F58G11.6 [C.elegans]//1.7e-64:337:95//Hs.l05907:AA186514
F-HEMBA1003046//Homo sapiens mitochondrial processing peptidase beta-subunit mRNA, complete cds//3.2e-166:777:98//Hs.44097:AF054182
F-HEMBA1003064//ESTs//3.2e-07:320:65//Hs.23466:AI223438
F-HEMBA1003067
F-HEMBA1003071//Homo sapiens hyperpolarization-activated channel 1 (IH1) mRNA, partial cds//1.5e-15:611:59//Hs.124161:AF065164
F-HEMBA1003077//Homo sapiens KIAA0405 mRNA, complete cds//2.2e-29:542:62//Hs.48998:AB007865
F-HEMBA1003078//CYTOCHROME P450 IVF3//2.0e-29:452:67//Hs.106242:AB002454
F-HEMBA1003079//EST//2.0e-20:273:73//Hs.138001:AI034461
F-HEMBA1003083//EST//2.0e-48:314:86//Hs.149580:AI281881
F-HEMBA1003086//ESTs//2.6e-20:237:73//Hs.129331:AI090721
F-HEMBA1003096//ESTs, Weakly similar to HMG-box transcription factor [M.musculus]//0.98:216:61//Hs.97865:AA405872
F-HEMBA1003098//EST//2.9e-19:239:73//Hs.152366:AA486721
F-HEMBA1003117//H.sapiens ERF-2 mRNA//0.0048:447:59//Hs.78909:U07802
F-HEMBA1003129//Homo sapiens clone 24407 mRNA sequence//1.9e-06:507:58//Hs.12432:AF070575
F-HEMBA1003133//Homo sapiens mRNA for KIAA0771 protein, partial cds//0.038:288:63//Hs.6162:AB018314
F-HEMBA1003136
F-HEMBA1003142//ESTs//3.6e-112:526:99//Hs.55982:AA284279
F-HEMBA1003148//Homo sapiens mRNA for dachshund protein//2.2e-184:850:99//Hs.63931:AJ005670
F-HEMBA1003166//Homo sapiens mRNA for KIAA0688 protein, complete cds//1.1e-24:171:83//Hs.l41874:AB014588
F-HEMBA1003175//EST//0.91:168:60//Hs.123335:AA810740
F-HEMBA1003179//EST, Weakly similar to hypothetical protein in purB 5' region [E.coli]//4.7e-20:118:97//Hs.ll8831:AA211895
F-HEMBA1003197//ESTs//0.049:265:58//Hs.153718:AI215523
F-HEMBA1003199//SOX-3 PROTEIN//0.00034:383:60//Hs.157429:X71135
F-HEMBA1003202//ESTs//7.1e-84:408:98//Hs.130134:AA905412
F-HEMBA1003204//Homo sapiens PYRIN (MEFV) mRNA, complete cds//4.6e-33:154:85//Hs.113283:AF018080
F-HEMBA1003212//ESTs//1.0e-31:159:84//Hs.134067:AI076765
F-HEMBA1003220//EST//8.6e-29:317:73//Hs.150552:AI053784
F-HEMBA1003222//ESTs//0.77:208:62//Hs.85451:AA181310
F-HEMBA1003229//EST//0.084:233:60//Hs.98176:AA417012
F-HEMBA1003235//Homo sapiens antigen NY-CO-16 mRNA, complete cds//0.00054:432:58//Hs.l32206:AF039694
F-HEMBA1003250
F-HEMBA1003257//Homo sapiens fibroblast growth factor 18 (FGF18) mRNA, complete cds//4.3e-08:426:64//Hs.49585:AF075292
F-HEMBA1003273//EST//0.00078:195:65//Hs.158019:AA867991
F-HEMBA1003276//EST//6.6e-09:159:74//Hs.162664:AA605020
F-HEMBA1003278//ESTs//0.89:257:63//Hs.23207:R42864
F-HEMBA1003281//ESTs//2.6e-33:175:98//Hs.122278:AA781867
F-HEMBA1003286//Homo sapiens chromosome 3q13 beta-1,4-galactosyltransferase mRNA, complete cds//2.9e-146:539:97//Hs.13225:AF038662
F-HEMBA1003291//Homo sapiens mRNA for KIAA0537 protein, complete cds//1.6e-167:799:98//Hs.12836:AB011109
F-HEMBA1003296//EST//0.0013:49:97//Hs.137157:R44912
F-HEMBA1003304//ESTs//0.047:164:64//Hs.94448:AA770160
F-HEMBA1003309//ESTs//7.8e-123:589:98//Hs.l05486:AA521012
F-HEMBA1003314//Homo sapiens mRNA for leucine zipper bearing kinase, complete cds//1.5e-189:865:99//Hs.124224:AB001872
F-HEMBA1003322//H.sapiens mRNA for sigma 3B protein//4.5e-49:399:80//Hs.154782:X99459
F-HEMBA1003327//EST//7.7e-10:165:72//Hs.114826:AA056254
F-HEMBA1003328//EST//0.00023:128:67//Hs.126467:AA913328
F-HEMBA1003330
F-HEMBA1003348//Human mRNA for KIAA0331 gene, complete cds//4.8e-26:256:78//Hs.146395:AB002329
F-HEMBA1003369//Homo sapiens DNA from chromosome 19p13.2 cosmids R31240, R30272 and R28549 containing the EKLF, GCDH, CRTC, and RAD23A genes, genomic sequence//0.37:187:65//Hs.80265:AD000092
F-HEMBA1003370//ESTs//8.2e-36:196:79//Hs.139158:AA226159
F-HEMBA1003373//ESTs//1.0:195:61//Hs.127307:AI263819
F-HEMBA1003376//Clathrin, light polypeptide (Lcb)//2.3e-29:606:64//Hs.73919:X81637
F-HEMBA1003380//ESTs//2.5e-21:303:70//Hs.37528:H58017
F-HEMBA1003384//ESTs//0.14:281:61//Hs.159650:N95552
F-HEMBA1003395//ESTs//0.53:121:70//Hs.144873:AI202488
F-HEMBA1003402//EST//0.029:148:66//Hs.116798:AA633813
F-HEMBA1003403//Adducin 2 (beta) {alternative products }//5.0e-05:445:61//Hs.90951:U43959
F-HEMBA1003408//ESTs//9.0e-12:87:98//Hs.70266:Z78309
F-HEMBA1003417//Glutamate-cysteine ligase (gamma-glutamylcysteine synthetase), regulatory (30.8kD)//9.5e-05:541:58//Hs.89709:L35546
F-HEMBA1003418//ESTs//3.5e-85:399:100//Hs.154489:AA564962
F-HEMBA1003433//Homo sapiens nibrin (NBS) mRNA, complete cds//2.0e-149:686:99//Hs.25812:AF058696
F-HEMBA1003447//Human mRNA for KIAA0380 gene, complete cds//0.43:271:60//Hs.47822:AB002378
F-HEMBA1003461//Glycoprotein Ib (platelet), beta polypeptide//4.8e-08:775:58//Hs.3847:U59632
F-HEMBA1003463//ESTs//3.3e-22:121:99l/Hs.130847:AA058578
F-HEMBA1003480//Homo sapiens mRNA for KIAA0700 protein, partial cds//0.16:321:60//Hs.13999:AB014600
F-HEMBA1003528//ESTs//3.8e-53:315:91//Hs.129688:AA057443
F-HEMBA1003531//Human mRNA for KIAA0033 gene, partial cds//4.9e-51:451:78//Hs.22271:D26067
F-HEMBA1003538//ESTs//1.2e-82:415:96//Hs.162075:AI392811
F-HEMBA1003545//ISL1 transcription factor, LIM/homeodomain, (islet-1)//5.0e-75:736:73//Hs.505:U07559
F-HEMBA1003548//ESTs//8.7e-77:411:95//Hs.163443:R23311
F-HEMBA1003555//Human nucleotide-binding protein mRNA, complete cds//3.6e-33:562:64//Hs.81469:U01833
F-HEMBA1003556
F-HEMBA1003560//EST//3.7e-29:202:86//Hs.136858:AA767122
F-HEMBA1003568//ESTs//2.4e-06:214:65//Hs.143371:AI342327
F-HEMBA1003569//Human metastasis-associated mtal mRNA, complete cds//2.0e-58:455:66//Hs.101448:U35113
F-HEMBA1003571//ESTs//0.0025:198:63//Hs.116448:AA648972
F-HEMBA1003579//ESTs//6.0e-110:513:99//Hs.97372:AA398546
F-HEMBA1003581//ESTs, Highly similar to TALIN [Mus musculus]//3.6e-19:108:99//Hs.18420:AA599232
F-HEMBA1003591//ESTs, Weakly similar to R74.5 [C.elegans]//5.2e-85:487:92//Hs.57937:W68285
F-HEMBA1003595//Membrane cofactor protein (CD46, trophoblast-lymphocyte cross-reactive antigen)//2.8e-06:439:62//Hs.83532:X59405
F-HEMBA1003597//ESTs//0.0025:200:64//Hs.8473:T40827
F-HEMBA1003598//ESTs//0.18:187:63//Hs.98641:AA429916
F-HEMBA1003615//ESTs, Highly similar to phosphorylation regulatory protein HP-10 [H.sapiens]//2.4e-133:644:97//Hs.3566:AA314782
F-HEMBA1003617//Homa sapiens mRNA for HRIHFB2157, partial cds//7.9e-171:501:97//Hs.124956:AB015344
F-HEMBA1003621//Homo sapiens protein inhibitor of activated STAT protein PIASx-alpha mRNA, complete cds//4.4e-16:161:78/IHs.111323:AF077954
F-HEMBA1003622//EST//0.0085:251:62//Hs.97343:AA401750
F-HEMBA1003630//ESTs//7.5e-05:304:61//Hs.87131:AA233159
F-HEMBA1003637//Homo sapiens homolog of the Aspergillus nidulans sudD gene product mRNA, complete cds//7.9e-26:546:63//Hs.109901:AF013591
F-HEMBA1003640//ESTs//1.1e-11:267:661/Hs.34359:AI122791
F-HEMBA1003645
F-HEMBA1003646
F-HEMBA1003656
F-HEMBA1003662
F-HEMBA1003667//ESTs//1.5e-27:235:81//Hs.55855:AA621381
F-HEMBA1003679//ESTs//4.3e-49:251:97//Hs.152811:AA630906
F-HEMBA1003680//Human plectin (PLEC1) mRNA, complete cds//3.4e-06:464:61//Hs.79706:U53204
F-HEMBA1003684//ESTs, Weakly similar to zinc finger protein C2H2-171 [H.sapiens]//1.6e-100:478:98//Hs.118866:AI017072
F-HEMBA1003690//Homo sapiens mRNA for KIAA0600 protein, partial cds//9.5e-74:606:77//Hs.9028:AF039691
F-HEMBA1003692//ESTs//4.2e-43:252:92//Hs.39748:AA487187
F-HEMBA1003711//Homo sapiens mRNA for KIAA0544 protein, partial cds//0.81:254:62//Hs.32316:AB011116
F-HEMBA1003714//ESTs//6.4e-98:495:95//Hs.43846:N49995
F-HEMBA1003715//ESTs//1.3e-11:228:69//Hs.101237:AA708760
F-HEMBA1003720//Homo sapiens clone 23892 mRNA sequence//5.5e-45:692:68//Hs.91916:AF035317
F-HEMBA1003725//EST//2.5e-46:228:100//Hs.160069:AA926921
F-HEMBA1003729//ESTs//4.1e-48:253:96//Hs.26270:AA258839
F-HEMBA1003733//Human Line-1 repeat mRNA with 2 open reading frames//8.6e-102:753:81//Hs.23094:M19503
F-HEMBA1003742//Homo sapiens chromosome 19, cosmid
R31180//0.16:242:62//Hs.153325:AC005390
F-HEMBA1003758//ESTs//9.3e-12:408:61//Hs.148459:AI198946
F-HEMBA1003760//Homo sapiens clone 23698 mRNA sequence//9.7e-35:430:69//Hs.8136:U81984
F-HEMBA1003773//EST//0.76:191:61//Hs.127020:AA934920
F-HEMBA1003783//ESTs, Weakly similar to C01H6.7 [C.elegans]//1.7e-24:224:81//Hs.18171:AA524327
F-HEMBA1003784//ESTs//0.13:120:67//Hs.161993:AA503172
F-HEMBA1003799//Interleukin 9 receptor//2.0e-17:263:70//Hs.1702:L39064
F-HEMBA1003803//Homo sapiens calcium-activated potassium channel (KCNN3) mRNA, complete cds//0.13:222:61//Hs.89230:AF031815
F-HEMBA1003804//ESTs//1.4e-112:275:98//Hs.72132:AF039239
F-HEMBA1003805//Human p62 mRNA, complete cds//1.1e-11:523:60//Hs.119537:M88108
F-HEMBA1003807//ESTs//4.1e-08:279:68//Hs.115679:AI379721
F-HEMBA1003827//Homo sapiens mRNA for KIAA0616 protein, partial cds//3.3e-85:586:87//Hs.6051:AB014516
F-HEMBA1003836//EST//6.8e-06:98:74//Hs.l45447:AI204220
F-HEMBA1003838//ESTs, Moderately similar to !!!! ALU SUBFAMILY SC WARNING ENTRY !!!! [H.sapiens]//3.8e-40:151:88//Hs.139007:H74314
F-HEMBA1003856//ESTs//8.6e-53286:95//Hs.116645:AI005167
F-HEMBA1003864//Human mRNA for KIAA0369 gene, complete cds//0.11:144:66//Hs.21355:AB002367
F-HEMBA1003866//Homo sapiens semaphorin F homolog mRNA, complete cds//4.3e-30:580:63//Hs.27621 :U52840
F-HEMBA1003879//Nuclear cap binding protein, 80kD//6.7e-10:87:95//Hs.89563:D32002
F-HEMBA1003880
F-HEMBA1003885//Homo sapiens mRNA for KIAA0752 protein, partial cds//4.2e-18:302:67//Hs.23711:AB018295
F-HEMBA1003893//ESTs, Weakly similar to HYPOTHETICAL 27.8 KD PROTEIN IN VMA7-RPS31A INTERGENIC REGION [S.cerevisiae]//1.2e-49:295:92//Hs.114673:W72675
F-HEMBA1003902//ESTs//1.1e-11:165:74//Hs.54632:AA976236
F-HEMBA1003908//Homo sapiens mRNA for KIAA0525 protein, partial cds//0.081:345:58//Hs.78494:AB011097
F-HEMBA1003926/IEST//2.5e-32:253 :83//Hs.132635:AI032875
F-HEMBA1003937//Human mRNA for KIAA0391 gene, complete cds//2.9e-38:313:69//Hs.154668:AB002389
F-HEMBA1003939//ESTs//3.4e-07:150:71//Hs.148926:R59562
F-HEMBA1003942//EST, Weakly similar to 24 KD PROTEIN [Xenopus laevis]//0.0029:222:61//Hs.l44236:W52380
F-HEMBA1003950//ESTs//0.98:200:62//Hs.163912:W20055
F-HEMBA1003953//Zinc finger protein 7 (KOX 4, clone HF.16)//0.00014:271:66//Hs.2076:M29580
F-HEMBA1003958//ESTs, Moderately similar to !!!! ALU SUBFAMILY J WARNING ENTRY !!!! [H.sapiens]//2.1e-44:243:76//Hs.91146:N73230
F-HEMBA1003959//ESTs//0.067:251:59//Hs.39915:H78567
F-HEMBA1003976//EST//6.7e-09:109:81//Hs.154635:AI138965
F-HEMBA1003978
F-HEMBA1003985//EST//0.32:115:69//Hs.102617:N47009
F-HEMBA1003987//ESTs//7.8e-07:60:100//Hs.66058:AA424456
F-HEMBA1003989//Homo sapiens HIV-1 inducer of short transcripts binding protein (FBI1) mRNA, complete cds//0.022:349:58//Hs.104640:AF000561
F-HEMBA1004000//EST//7.2e-07:200:66//Hs.119082:AA358468
F-HEMBA1004011//EST//0.019:241:62//Hs.116989:AA676493
F-HEMBA1004012//ESTs//3.6e-09:177:68//Hs.106132:AA812573
F-HEMBA1004015//ESTs//3.0e-86:407:99//Hs.115679:AI379721
F-HEMBA1004024//Homo sapiens mRNA for KIAA0772 protein, complete cds//5.2e-51:359:84//Hs.l5519:AB018315
F-HEMBA1004038//ESTs//1.2e-58:324:94//Hs.61658:AI239930
F-HEMBA1004042//EST//0.00088:272:6l//Hs.155763:AI312281
F-HEMBA1004045//EST//2.7e-20:408:66//Hs.l62529:AA584160
F-HEMBA1004048//Transforming growth factor beta//0.026:462:57//Hs.6101:M60315
F-HEMBA1004049//ESTs//8.1e-68:430:86/JHs.146307:AA584638
F-HEMBA1004055//Human chromosome 3p21.1 gene sequence//1.5e-10:457:58//Hs.82837:L13435
F-HEMBA1004056//Homo sapiens mRNA for alpha(1,2)fucosyltransferase, complete cds//1.5e-46:199:80//Hs.46328:D87942
F-HEMBA1004074//ESTs//3.0e-23:219:74//Hs.70279:AA757426
F-HEMBA1004086//EST//0.36:189:62//Hs.156218:AA770107
F-HEMBA1004097//NADH-CYTOCHROME B5 REDUCTASE//1.0:302:57//Hs.75666:M28713
F-HEMBA1004111//Human G protein-coupled receptor (STRL22) mRNA, complete cds//4.3e-39:335:79//Hs.46468:U45984
F-HEMBA1004131//Human mRNA for KIAA0202 gene, partial cds//1.9e-24:610:61//Hs.80712:D86957
F-HEMBA1004132//EST//3.5e-06:143:70//Hs.136799:AA780064
F-HEMBA1004133//ESTs//1.0:157:68//Hs.161226:AI419759
F-HEMBA1004138//H.sapiens mRNA for RanGTPase activating protein 1//0.00055:343:62//Hs.5923:X82260
F-HEMBA1004143
F-HEMBA1004146
F-HEMBA1004150//EST//0.0046:402:57//Hs.147027:AI186056
F-HEMBA1004164//Homo sapiens mRNA for KIAA0798 protein, complete cds//1.8e-15:591:60//Hs.159277:AB018341
F-HEMBA1004168//Homo sapiens geminin mRNA, complete cds//1.5e-134:649:97//Hs.59988:AF067855
F-HEMBA1004199
F-HEMBA1004200//ESTs//0.0083:150:66//Hs.116424:AI375427
F-HEMBA1004202//ESTs, Weakly similar to GTP-BINDING PROTEIN YPTM1 [Zea mays]//1.2e-35:205:94//Hs.10092:AI189282
F-HEMBA1004203//ESTs//3.9e-14:237:70//Hs.118273:AA626040
F-HEMBA1004207//Leptin receptor//1.1e-167:791:98//Hs.54515:U50748
F-HEMBA1004225//ESTs//0.00087:231:64//Hs.13109:AA192514
F-HEMBA1004227//ESTs, Weakly similar to F55A11.4 [C.elegans]//0.012:156:67//Hs.l63588:AI073878
F-HEMBA1004238
F-HEMBA1004241//ESTs//8.7e-05:51:96//Hs.162826:AA679571
F-HEMBA1004246//EST//1.2e-36:198:96//Hs.121343:AA758522
F-HEMBA1004248//Homo sapiens insulin induced protein 1 (INSIG1) gene, complete cds//1.1e-28:295:72//Hs.56205:U96876
F-HEMBA1004264//Human HCF1 gene related mRNA sequence//3.1e-07:553:60//Hs.83634:U52112
F-HEMBA1004267//Homo sapiens mRNA for KIAA0688 protein, complete cds//4.9e-73:490:77//Hs.141874:AB014588
F-HEMBA1004272
F-HEMBA1004274//EST//0.43:154:61//Hs.125347:AA876444
F-HEMBA1004275//Human mRNA for KIAA0333 gene, partial cds//0.71:118:65//Hs.155313:AB002331
F-HEMBA1004276//Homo sapiens mRNA for KIAA0800 protein, complete cds//1.0:364:56//Hs.118738:AB018343
F-HEMBA1004286//Homo sapiens TGF beta receptor associated protein-1 mRNA, complete cds//6.9e-187:868:99//Hs.101766:AF022795
F-HEMBA1004289
F-HEMBA1004295//EST//0.20:149:62//Hs.162415:AA573484
F-HEMBA1004306//ESTs//0.041:177:64//Hs.158234:AI270047
F-HEMBA1004312//ESTs//0.83:253:59//Hs.121898:AI336314
F-HEMBA1004321//Zinc finger protein 136 (clone pHZ-20)//2.3e-40:452:65//Hs.69740:U09367
F-HEMBA1004323//EST//0.44:134:64//Hs.145464:AI204532
F-HEMBA1004327//Homo sapiens SOX22 protein (SOX22) mRNA, complete cds//0.017:209:64//Hs.43627:U35612
F-HEMBA1004330//ESTs//4.5e-27:171:91//Hs.112838:AA614062
F-HEMBA1004334//EST//2.4e-53:556:75//Hs.139093:AA166888
F-HEMBA1004335//Homo sapiens mRNA for KIAA0706 protein, complete cds//0.49:80:73//Hs.139648:AB014606
F-HEMBA1004341
F-HEMBA1004353//Homo sapiens mRNA for c-myc binding protein, complete cds//2.7e-39:270:86//Hs.80686:D89667
F-HEMBA1004354//Human CHL1 potential helicase (CHLR1), complete cds//1.3e-46:190:92//Hs.27424:U75968
F-HEMBA1004356//Thyrotropin-releasing hormone receptor//0.15:296:62//Hs.3022:D85376
F-HEMBA1004366//ESTs, Weakly similar to transposon LRE2 reverse transcriptase homolog [H.sapiens]//7.8e-10:396:61//Hs.33688:AA020928
F-HEMBA1004372//ESTs//0.90:172:62//Hs.145611:R68800
F-HEMBA1004389//Zinc finger protein 148 (pHZ-52)//8.0e-28:359:67//Hs.112180:AF039019
F-HEMBA1004394//ESTs//0.023:357:58//Hs.47212:N51250
F-HEMBA1004396//EST//3.4e-22:244:74//Hs.162554:AA584818
F-HEMBA1004405//EST//4.0e-43:214:100//Hs.33100:H42199
F-HEMBA1004408//ESTs, Weakly similar to The ha1539 protein is related to cyclophilin. [H.sapiens]/1.4e-20:144:88//HS.121076:AI246426
F-HEMBA1004429//Fucosyltransferase 1 (galactoside 2-alpha-L-fucosyltransferase, Bombay phenotype included)//4.8e-18:248:72//Hs.69747:M35531
F-HEMBA1004433//Small inducible cytokine A5 (RANTES)//8.2e-39:248:81//Hs.155464:AF088219
F-HEMBA1004460//Homo sapiens PYRIN (MEFV) mRNA, complete cds//5.6e-87:650:81//Hs.113283:AF018080
F-HEMBA1004461//ESTs//0.057:217:61//Hs.26989:Z41606
F-HEMBA1004479//Homo sapiens clone 23698 mRNA sequence//4.9e-17:223:71//Hs.8136:U81984
F-HEMBA1004482//EST//0.0056:261:59//Hs.45012:N39450
F-HEMBA1004499//ESTs//4.1e-68:340:97//Hs.134266:AA992600
F-HEMBA1004502//ESTs//7.7e-32:195:91//Hs.134906:H93431
F-HEMBA1004506//Human Line-1 repeat mRNA with 2 open reading frames//9.0e-89:758:76//Hs.23094:M19503
F-HEMBA1004507//ESTs, Weakly similar to T19B10.6 [C.elegans]//1.4e-61:296:99//Hs.114622:AA693492
F-HEMBA1004509//Homo sapiens suppressor of white apricot homolog 2 (SWAP2) mRNA, complete cds//0.014:265:61//Hs.43543:AF042800
F-HEMBA1004534//Filamin 1 (actin-binding protein-280)//5.0e-74:678:74//Hs.76279:X53416
F-HEMBA1004538//EST//0.00047:268:58//Hs.136870:AA805381
F-HEMBA1004542//Human butyrophilin protein (BT3.3) mRNA, partial cds//0.74:74:75//Hs.87497:U90552
F-HEMBA1004554
F-HEMBA1004560//ESTs//3.1e-19:240:73//Hs.112637:AA805331
F-HEMBA1004573//EST//2.4e-59:290:99//Hs.112908:AA620802
F-HEMBA1004577//ESTs, Weakly similar to UTR1 PROTEIN [S.cerevisiae]//1.2e-17:334:67//Hs.24536:AA479825
F-HEMBA1004586//Von Hippel-Lindau syndrome//5.1 e-35:337:78//Hs.78160:AF010238
F-HEMBA1004596//ESTs//3.3e-32:189:94//Hs.42530:N41661
F-HEMBA1004604//Human hindlimb expressed homeobox protein backfoot (Bft) mRNA, complete cds//0.42:186:66//Hs.84136:1170370
F-HEMBA1004610//ESTs, Moderately similar to !!!! ALU SUBFAMILY J WARNING ENTRY !!!! [H.sapiens]//2.3e-16:297:68//Hs.106008:AA147606
F-HEMBA1004617//EST//0.027:188:61//Hs.l59094:AI383198
F-HEMBA1004629//ESTs//7.8e-09:348:63//Hs.138358:T66178
F-HEMBA1004631//EST//0.0012:268:60//Hs.150685:AA923416
F-HEMBA1004632//ESTs//0.82:125:67//Hs.143619:AI360891
F-HEMBA1004637//ESTs//0.0034:229:64//Hs.157178:AI346780
F-HEMBA1004638//ESTs//2.0e-11:166:71//Hs.128657:AI017522
F-HEMBA1004666//EST//0.44:294:58//Hs.44780:N36083
F-HEMBA1004669//ESTs//1.7e-28:200:86//Hs.8084:W22796
F-HEMBA1004670//Mucin 1, transmembrane//0.060:416:57//Hs.89603:J05582
F-HEMBA1004672//ESTs//0.27:44:95//Hs.86237:AA206141
F-HEMBA1004693//ESTs//5.3e-55:301:95//Hs.159066:AI093252
F-HEMBA1004697//H.sapiens mRNA for ribosomal protein L18a homologue//0.64:313:61//Hs.118578:X80821
F-HEMBA1004705//Homo sapiens KIAA0432 mRNA, complete cds//4.5e-19:230:73//Hs.155174:AB007892
F-HEMBA1004709//ESTs//3.1e-31:176:88//Hs.152413:AA780515
F-HEMBA1004711//Cholinergic receptor, nicotinic, delta polypeptide//1.0:244:57//Hs.99975:X55019
F-HEMBA1004725//Homo sapiens agrin precursor mRNA, partial cds//0.24:328:60//Hs.68900:AF016903
F-HEMBA1004730//ESTs, Weakly similar to ORF2-like protein [H.sapiens]//5.9e-32:476:70//Hs.116874:AA524909
F-HEMBA1004733//ESTs//3.8e-16:96:79//Hs.152413:AA780515
F-HEMBA1004734//Human epidermoid carcinoma mRNA for ubiquitin-conjugating enzyme E2 similar to Drosophila bendless gene product, complete cds//0.l6:329:58//Hs.75355:D83004
F-HEMBA1004736//Human Line-1 repeat mRNA with 2 open reading frames//2.0e-61:663:71//Hs.23094:M19503
F-HEMBA1004748//ESTs//1.5e-05:343:63//Hs.42241:H96813
F-HEMBA1004751//ESTs//3.7e-32:147:80//Hs.138788:N54504
F-HEMBA1004752//Homo sapiens mRNA for KIAA0288 gene, complete cds//0.00020:521:59//Hs.91400:AB006626
F-HEMBA1004753//Homo sapiens DEC-205 mRNA, complete cds//5.1e-46:337:84//Hs.l53563:AF011333
F-HEMBA1004756//Human transporter protein (g17) mRNA, complete cds//3.1e-24:416:65//Hs.76460:U49082
F-HEMBA1004758//Homosapiens transcription factor SL1 mRNA, complete cds//1.2e-136:769:91//Hs.153088:L39060
F-HEMBA1004763//Loricrin//0.0018:227:62//Hs.l55657:M61120
F-HEMBA1004768//Human Line-1 repeat mRNA with.2 open reading frames//4.5e-115:909:78//Hs.23094:M19503
F-HEMBA1004770//Human Rad50 (Rad50) mRNA, complete cds//0.020:728:57//Hs.41587:U63139
F-HEMBA1004771
F-HEMBA1004776//ESTs, Weakly similar to progesterone receptor-related protein p23 [H.sapiens]//1.0:158:63//Hs.62004:AF039235
F-HEMBA1004778//ESTs//1.2e-70:336:99//Hs.113052:AI222106
F-HEMBA1004795
F-HEMBA1004803//ESTs//5.0e-75:454:88//Hs.138632:H97952
F-HEMBA1004806//EST//0.080:142:65//Hs.160268:AI148971
F-HEMBA1004807//Human HIV1 tata element modulatory factor mRNA sequence from chromosome 3//4.5e-48:171:92//Hs.134510:L01042
F-HEMBA1004816//EST//1.0e-17:175:71//Hs.140680:AA873646
F-HEMBA1004820//ESTs//1.3e-136:629:99//Hs.160726:AI300481
F-HEMBA1004847//ESTs//2.1 e-09:66:98//Hs.158161:AA312511
F-HEMBA1004850//EST//0.033:253:64//Hs.158782:A376601
F-HEMBA1004863//Homo sapiens mRNA for KIAA0578 protein, partial cds//0.83:179:62//Hs.22998:AB011150
F-HEMBA1004864//ESTs, Weakly similar to ANON-66Db [D.melanogaster]//1.7e-13:81:100//Hs.75884:AA446987
F-HEMBA1004865//ESTs//0.92:148:65//Hs.126980:AA934077
F-HEMBA1004880//H.sapiens mRNA for retrotransposon//1.2e-30:264:79//Hs.6940:Z48633
F-HEMBA1004889//Growth arrest-specific l//0.20:146:68/Hs.65029:L13698
F-HEMBA1004900//ESTs//1.6e-32:196:93//Hs.132032:R85304
F-HEMBA1004909//ESTs//3.4e-13:154:75//Hs.151467:N51106
F-HEMBA1004918//EST//0.78:122:61//Hs.l45491:AI254348
F-HEMBA1004923//ELK1, member of ETS oncogene family//1.6e-40:340:79//Hs.116549:AL009172
F-HEMBA1004929//Cardiac gap junction protein//0.0048:588:57//Hs.74471:X52947
F-HEMBA1004930//ESTs//1.5e-17:227:74//Hs.148739:AI224959
F-HEMBA1004933//Human pseudoautosomal homeodomain-containing protein (PHOG) mRNA, complete cds//0.11:182:65//Hs.105932:U89331
F-HEMBA1004934
F-HEMBA1004944//EST//1.2e-67:349:96//Hs.162281:AA553981
F-HEMBA1004954//ESTs//0.0i4:404:60//Hs.11177:AA417813
F-HEMBA1004956//EST//2.3e-05:208:64//Hs.146958:AI174478
F-HEMBA1004960//ESTs//0.79:169:62//Hs.11637:W03274
F-HEMBA1004972
F-HEMBA1004973//Homo sapiens mRNA for KIAA0445 protein, complete cds//0.073:574:58//Hs.154139:AB007914
F-HEMBA1004977//EST//4.4e-12:86:94//Hs.157819:AI361946
F-HEMBA1004978//ESTs//0.097:337:60//Hs.114157:AA703013
F-HEMBA1004980//EST//3.2e-10:169:65//Hs.149I23:AI244750
F-HEMBA1004983//EST//0.93:85:71//Hs.162267:AA553589
F-HEMBA1004995//ESTs//0.46:296:61//Hs.135168:AI394026
F-HEMBA1005008//ESTs//1.5e-20:156:85//Hs.114140:U35429
F-HEMBA1005009//Homo sapiens chromosome 7q22 sequence//1.5e-52:379:72//Hs.151887:AF053356
F-HEMBA1005019//Homo sapiens mRNA for KIAA0648 protein, partial cds//4.5e-148:693:98//Hs.31921:AB014548
F-HEMBA1005029//Homo sapiens mRNA for KIAA0660 protein, complete cds//1.0:215:65//Hs.6727:AB014560
F-HEMBA1005035//ESTs, Weakly similar to HYPOTHETICAL 82.8 KD PROTEIN B0303.4 IN CHROMOSOME III [C.elegans]//9.4e-106:503:98//Hs.21362:AF039237
F-HEMBA1005039//Human kpni repeat mrna (cdna clone pcd-kpni-8), 3' end//5.8e-60:272:89//Hs.103948:K00627
F-HEMBA1005047//Homo sapiens MAD-related gene SMAD7 (SMAD7) mRNA, complete cds//0.078:442:59//Hs.100602:AF010193
F-HEMBA1005050//H.sapiens ERF-2 mRNA//0.0025:251:63//Hs.78909:U07802
F-HEMBA1005062//ESTs//0.020:268:59//Hs.146181:AI264462
F-HEMBA1005066//Homo sapiens X-ray repair cross-complementing protein 2 (XRCC2) mRNA, complete cds//1.5e-59:411:85//Hs.129727:AF035587
F-HEMBA1005075//Human mRNA for KIAA0383 gene, partial cds//0.00010:395:57//Hs.27590:AB002381
F-HEMBA1005079//Dihydrolipoamide branched chain transacylase (E2 component of branched chain keto acid dehydrogenase complex)//3.5e-26:344:72//Hs.89479:X66785
F-HEMBA1005083//Homo sapiens centrosomal Nek2-associated protein 1 (C-NAP1) mRNA, complete cds//0.59:631:59//Hs.27910:AF049105
F-HEMBA1005101//Homo sapiens SYT interacting protein SIP mRNA, complete cds//4.1e-163:762:98//Hs.11170:AF080561
F-HEMBA1005113//ESTs//0.52:109:68//Hs.106330:AI031916
F-HEMBA1005123//Homo sapiens mRNA for KIAA0761 protein, partial cds//1.3e-52:468:78//Hs.93121:AB018304
F-HEMBA1005133//ESTs//1.6e-27:366:73//Hs.151467:N51106
F-HEMBA1005149//EST//3.3e-37:304:80//Hs.132635:AI032875
F-HEMBA1005152//ESTs//3.9e-09:285:62//Hs.155876:AA593021
F-HEMBA1005159//EST//8.4e-05:289:64//Hs.125563:AA884216
F-HEMBA1005185//ESTs//1.4e-22:129:96//Hs.14920:AA910914
F-HEMBA1005201//EST//4.0e-16:96:98//Hs.89002:AA282197
F-HEMBA1005202
F-HEMBA1005206//Homo sapiens sox1 gene//0.0079:431:58//Hs.144029:Y13436
F-HEMBA1005219//ESTs//4.3e-47:299:88//Hs.5019:W26547
F-HEMBA1005223//ESTs//0.00030:168:66//Hs.76487:N37081
F-HEMBA1005232//EST//0.0078:209:61//Hs.46852:N48302
F-HEMBA1005241//Homo sapiens neuronal thread protein AD7c-NTP mRNA, complete cds//6.0e-54:399:79//Hs.129735:AF010144
F-HEMBA1005244//ESTs//2.5e-14:85:10011Hs,128744:AI191922
F-HEMBA1005251//ESTs//0.012:49:95//Hs.161554:AA393896
F-HEMBA1005252//Homo sapiens mRNA for KIAA0585 protein, partial cds//4.7e-151:705:98//Hs.72660:AB011157
F-HEMBA1005274//ESTs//7.1e-09:298:64//Hs.145522:AI261380
F-HEMBA1005275//ESTs//7.9e-13:375:63//Hs.148974:AA001777
F-HEMBA1005293//Homo sapiens clone 23662 mRNA sequence//7.7e-22:338:65//Hs.12451:U97018
F-HEMBA1005296//ESTs//0.055:299:60//Hs.86320:AI149232
F-HEMBA1005304//Small inducible cytokine A5 (RANTES)//1.7e-45:322:85//Hs.155464:AF088219
F-HEMBA1005311
F-HEMBA1005314//ESTs//8.1e-39:199:98//Hs.119974:AI279516
F-HEMBA1005315//ESTs//1.9e-07:266:64//Hs.141440:N21615
F-HEMBA1005318//ESTs//5.3e-06:161:72//Hs.119411:AA937117
F-HEMBA1005331//Human checkpoint suppressor 1 mRNA, complete cds//0.00075:310:63//Hs.111597:U68723
F-HEMBA1005338//Homo sapiens mRNA for matrilin-4, partial//4.4e-153:740:97//Hs.29361:AJ007581
F-HEMBA1005353//EST//5.4e-09:2-22:68//Hs.119508:AA485732
F-HEMBA1005359//Zinc finger protein 137 (clone pHZ-30)//5.7e-100:500:88//Hs.151689:U09414
F-HEMBA1005367//Homo sapiens melastatin 1 (MLSN1) mRNA, complete cds//2.5e-70:572:73//Hs.43265:AF071787
F-HEMBA1005372//ESTs//0.00045:163:66//Hs.164058:AI417905
F-HEMBA1005374//Human melanoma antigen recognized by T-cells (MART-1) mRNA//6.1e-43:341:81//Hs.154069:U06452
F-HEMBA1005382//EST//2.4e-32:167:99//Hs.147186:AI93053
F-HEMBA1005389//ESTs//0.0021:245:59//Hs.104463:AA804448
F-HEMBA1005394//ESTs, Weakly similar to No definition line found [C.elegans]//1.0e-130:620:98//Hs.108990:N25951
F-HEMBA1005403//ESTs, Weakly similar to No definition line found [C.elegans]//7.7e-151:727:97//Hs.17118:AI033807
F-HEMBA1005408//ESTs//3.2e-70:426:89//Hs.158078:H24513
F-HEMBA1005410//EST//2.5e-25:460:67//Hs.138765:N70347
F-HEMBA1005411
F-HEMBA1005423//Homo sapiens cyclin-dependent kinase inhibitor (CDKN2C) mRNA, complete cds//3.3e-171:537:99//Hs.4854:AF041248
F-HEMBA1005426//EST//1.0:148:64//Hs.44469:N33323
F-HEMBA1005443//Zinc finger protein 157 (HZF22)//9.0e-34:259:72//Hs.89897:U28687
F-HEMBA1005447//EST//3.9e-10:211:70//Hs.145960:AI276783
F-HEMBA1005468//ESTs//8.4e-53:390:81//Hs.152395:AA533107
F-HEMBA1005469//Human (clone E5.1) RNA-binding protein mRNA, complete cds//3.1e-29:155:99//Hs.75104:L37368
F-HEMBA1005472//Human Line-1 repeat mRNA with 2 open reading frames//1.4e-88:481:92//Hs.23094:M19503
F-HEMBA1005474//Small inducible cytokine A5 (RANTES)//4.2e-29:257:78//Hs.155464:AF088219
F-HEMBA1005475//Homo sapiens antigen NY-CO-16 mRNA, complete cds//5.3e-09:414:60//Hs.132206:AF039694
F-HEMBA1005497//Glucocorticoid receptor alpha { alternative products}//8.7e-41:588:69//Hs.102761:U25029
F-HEMBA1005500//Homo sapiens PAC clone DJ1093017 from 7q11.23-q21//1.1e-28:318:73//Hs.159530:AC004957
F-HEMBA1005506//Human mRNA for KIAA0010 gene, complete cds//0.67:351:58//Hs.155287:D13635
F-HEMBA1005508//ESTs//0.45:326:59//Hs.102756:AA526911
F-HEMBA1005511//Human mRNA for KIAA0355 gene, complete cds//4.2e-49:400:79//Hs.153014:AB002353
F-HEMBA1005513//ESTs, Weakly similar to males-absent on the first [D.melanogaster]//5.3e-76:378:97//Hs.22767:N99220
F-HEMBA1005517//Homo sapiens transcription factor forkhead-like 7 (FKHL7) gene, complete cds//0.54:623:56//Hs.143551:AF048693
F-HEMBA1005518//ESTs//0.10:207:60//Hs.72447:AA160575
F-HEMBA1005520//Human mRNA for tryptophan hydroxylase (EC 1.14.16.4)//3.1e-55:288:85//Hs.144563:AF057280
F-HEMBA1005526//Small inducible cytokine A5 (RANTES)//5.4e-48:176:76//Hs.155464:AF088219
F-HEMBA1005528//ESTs, Highly similar to POP2 PROTEIN [Saccharomyces cerevisiae]//1.2e-30:166:96//Hs.17035:AI080471
F-HEMBA1005530
F-HEMBA1005548//Homo sapiens short form transcription factor C-MAF (c-maf) mRNA, complete cds//4.6e-18:391:64//Hs.30250:AF055376
F-HEMBA1005552//ESTs//1.8e-46:238:88//Hs.138856:H47461
F-HEMBA1005558//Human involucrin mRNA//3.0e-07:501:60//Hs.157091:M13903
F-HEMBA1005568//ESTs//0.013:259:63//Hs.13669:H47257
F-HEMBA1005570//ESTs//0.0084:442:59//Hs.125384:AI346507
F-HEMBA1005576//Homo sapiens mRNA for KIAA0463 protein, partial cds//1.9e-128:610:98//Hs.77738:AB007932
F-HEMBA1005577//ESTs//0.98:199:61//Hs.146226:AI312873
F-HEMBA1005581//Homo sapiens mRNA for MEGF5, partial cds//9.1e-53:830:64//Hs.57929:AB011538
F-HEMBA1005582
F-HEMBA1005583
F-HEMBA1005588//ESTs//1.3e-35:386:70//Hs.55855:AA621381
F-HEMBA1005593//S-ADENOSYLMETHIONINE SYNTHETASE ALPHA AND BETA
FORMS//0.54:439:591/Hs.2137:D49357
F-HEMBA1005595//Human mRNA for KIAA0325 gene, partial cds//5.5e-06:378:57//Hs.7720:AB002323
F-HEMBA1005606//EST//2.0e-60:324:94//Hs.5062:D19609
F-HEMBA1005609//ESTs//6.0e-39:378:76//Hs.142242:H06982
F-HEMBA1005616//Human mRNA for tryptophan hydroxylase (EC 1.14.16.4)//8.2e-22:721:61//Hs.144563:AF057280
F-HEMBA1005621//ESTs, Weakly similar to MITOTIC MAD2 PROTEIN [S.cerevisiae]//1.8e-89:454:96//Hs.19400:AA662845
F-HEMBA1005627//EST//1.0:161:60//Hs.162765:AA622535
F-HEMBA1005631//EST//0.74:124:62//Hs.156185:AA723734
F-HEMBA1005632//ESTs//1.0:96:70//Hs.141321:N70199
F-HEMBA1005634//EST//6.6e-10:105:73//Hs.159692:AI416956
F-HEMBA1005666
F-HEMBA1005670//Homo sapiens mRNA for KIAA0570 protein, complete cds//2.7e-45:255:79//Hs.114293:AB011142
F-HEMBA1005679//Human kpni repeat mrna (cdna clone pcd-kpni-4), 3' end//1.2e-37:356:77//Hs.139107:K00629
F-HEMBA1005680
F-HEMBA1005685
F-HEMBA1005699//Human putative EPH-related PTK receptor ligand LERK-8 (Eplg8) mRNA, complete cds//3.3e-71:497:85//Hs.26988:U66406
F-HEMBA1005705//ESTs//0.00093:149:65//Hs.163564:R43678
F-HEMBA1005717//EST//0.018:115:66//Hs.160541:AI270143
F-HEMBA1005732//Farnesyl diphosphate synthase (farnesyl pyrophosphate synthetase, dimethylallyltranstransferase, geranyltranstransferase)//2.6e-20:151:88//Hs.77393:D14697
F-HEMBA1005737//ESTs//9.5e-34:235:88//Hs.160197:AA393754
F-HEMBA1005746//ESTs//0.20:260:59//Hs.112451:AI264024
F-HEMBA1005755//Human kpni repeat mrna (cdna clone pcd-kpni-8), 3' end//1.8e-48:425:78//Hs.103948:K00627
F-HEMBA1005765//Small inducible cytokine A5 (RANTES)//1.3e-36:280:81//Hs.155464:AF088219
F-HEMBA1005780//ESTs//1.0:139:67//Hs.88684:AA885141
F-HEMBAI0058131/ESTs//0.012:209:63//Hs.113365:R77747
F-HEMBA1005815//Human calpain-like protease (htra-3) mRNA, complete cds//2.0e-07:439:62//Hs.6133:U94346
F-HEMBA1005822//ESTs//9.3e-06:444:59//Hs.124344:T10577
F-HEMBA1005829//ESTs//1.1e-47:394:80//Hs.146811:AA410788
F-HEMBA1005834//Human Line-1 repeat mRNA with 2 open reading frames//7.9e-42:690:66//Hs.23094:M19503
F-HEMBA1005852//Human plectin (PLEC1) mRNA, complete cds//0.17:470:56//Hs.79706:U53204
F-HEMBA1005853//EST//0.013:211:60//Hs.162604:AA595150
F-HEMBA1005884//Homosapiens mRNA, chromosome 1 specific transcript KIAA0484//1.4e-53:332:83//Hs.158095:AB007953
F-HEMBA1005891//ESTs//1.1e-77:393:97//Hs.28545:AI268097
F-HEMBA1005894//Human G protein-coupled receptor (STRL22) mRNA, complete cds//7.2e-45:411:77//Hs.46468:U45984
F-HEMBA1005909//Human neuropeptide y2 receptor mRNA, complete cds//0.00054:477:59//Hs.37125:U42766
F-HEMBA1005911//Thromboxane A2 receptor//4.1e-45:419:75//Hs.89887:D38081
F-HEMBA1005921//Homo sapiens haemopoietic progenitor homeobox HPX42B (HPX42B) mRNA, complete cds//2.0e-46:434:78//Hs.125231:AF068006
F-HEMBA1005931//ESTs, Weakly similar to kruppel-related zinc finger protein [H.sapiens]//1.2e-46:228:100//Hs.152178:AI224880
F-HEMBA1005934//EST//3.1e-14:121:85//Hs.150003:AI291588
F-HEMBA1005962//EST//0.0010:212:62//Hs.163197:AA767883
F-HEMBA1005963
F-HEMBA1005990//Homo sapiens I-1 receptor candidate protein mRNA, complete cds//4.2e-151:697:99//Hs.26285:AF082516
F-HEMBA1005991//EST//3.0e-07:361:59//Hs.146442:AI127530
F-HEMBA1005999//EST//1.2e-14:350:66//Hs.122326:AA782526
F-HEMBA1006002
F-HEMBA1006005//ESTs, Weakly similar to TH1 protein [D.melanogaster]//0.98:197:61//Hs.5184:AA709151
F-HEMBA1006031
F-HEMBA1006035
F-HEMBA1006036//Homo sapiens PYRIN (MEFV) mRNA, complete cds//5.4e-92:617:84//Hs.113283:AF018080
F-HEMBA1006042//ESTs//6.3e-41:161:81//Hs.41186:R99609
F-HEMBA1006067//ESTs//2.0e-74:354:99//Hs.43321:AI139422
F-HEMBA1006081
F-HEMBA1006090//EST//1.2e-12:340:62//Hs.61195:AI418788
F-HEMBA1006091//ESTs//4.7e-98:473:98//Hs.9658:AA506313
F-HEMBA1006100//ESTs//7.1 e-22:273:73//Hs.144407:AA737799
F-HEMBA1006108//ESTs, Weakly similar to ZK792.1 [C.elegans]//2.1e-26:273:66//Hs.8763:W30741
F-HEMBA1006121//EST//0.00012:232:59//Hs.117096:AA677968
F-HEMBA1006124//EST//0.047:251:62//Hs.132257:AI027222
F-HEMBA1006130//Human HOX4C mRNA for a homeobox protein//1.0:150:62//Hs.74061:X59372
F-HEMBA1006138//ESTs//1.8e-27:132:84//Hs.141575:AA211734
F-HEMBA1006142//EST//2.5e-47:310:87//Hs.149580:AI281881
F-HEMBA1006155
F-HEMBA1006158//ESTs//5.1e-105:506:98//Hs.93468:N40575
F-HEMBA1006173//ESTs//2.5e-24:195:84//Hs.79092:H29627
F-HEMBA1006182//ESTs//2.5e-19:237:72//Hs.141840:AA028117
F-HEMBA1006198//ESTs//0.017:133 :67//Hs.142168:AA292540
F-HEMBA1006235//Homo sapiens clone 24422 mRNA sequence//8.6e-177:836:98//Hs.109268:AF070557
F-HEMBA1006248//Human zinc finger protein (MAZ) mRNA//0.0014:221:67//Hs.7647:M94046
F-HEMBA1006252
F-HEMBA1006253//EST//1.3e-100:467:100//Hs.146619:AI140706
F-HEMBA1006259//Homo sapiens mRNA for KIAA0798 protein, complete cds//0.00037:158:69//Hs.159277:AB018341
F-HEMBA1006268//ESTs//1.1e-20:376:67//Hs.72814:AA706631
F-HEMBA1006272//EST//4.8e-20:252:69//Hs.162992:AA688140
F-HEMBA1006278//H.sapiens PAP mRNA//6.5e-57:610:71//Hs.49007:X76770
F-HEMBA1006283
F-HEMBA1006284//ESTs//0.00017:248:63//Hs.143840:AI189964
F-HEMBA1006291
F-HEMBA1006293
F-HEMBA1006309//Homo sapiens T cell immune response cDNA7 (TIRC7) mRNA, complete cds//0.76:416:58//Hs.46465:U45285
F-HEMBA1006310//Homo sapiens mRNA for KIAA0602 protein, partial cds//9.3e-49:637:68//Hs.37656:AB011174
F-HEMBA1006328//ESTs//1.8e-71:429:88//Hs.139922:AA281350
F-HEMBA1006334//EST//0.082:267:57//Hs.136449:AA572789
F-HEMBA1006344//ESTs//6.2e-08:67:94//Hs.42302:AI032142
F-HEMBA1006347//ESTs, Weakly similar to males-absent on the first [D.melanogaster]//5.3e-76:378:97//Hs.22767:N99220
F-HEMBA1006349//ESTs//0.87:276:60//Hs.23628:H03287
F-HEMBA1006359//Zinc finger protein 43 (HTF6)//4.4e-117:823:81//Hs.74107:X59244
F-HEMBA1006364//EST//0.0012:168:66//Hs.156756:AI351026
F-HEMBA1006377//Homo sapiens RaIBP1-interacting protein (POB1) mRNA, complete cds//0.0028:422:59//Hs.80667:AF010233
F-HEMBA1006380//Homo sapiens syntaxin 4 binding protein UNC-18c (UNC-18c) mRNA, complete cds//0.41:265:61//Hs.8813:AF032922
F-HEMBA1006381//ESTs//3.8e-78:382:98//Hs.132171:AI042531
F-HEMBA1006398//Human Line-1 repeat mRNA with 2 open reading frames//2.1e-49:395:80//Hs.23094:M19503
F-HEMBA1006416//EST//7.3e-12:154:77//Hs.134086:AI077477
F-HEMBA1006419//EST//4.6e-51:179:86//Hs.149580:AI281881
F-HEMBA1006421//ISLET AMYLOID POLYPEPTIDE PRECURSOR//4.9e-46:517:72//Hs.51048:X68830
F-HEMBA1006424//ESTs//2.7e-08:380:60//Hs.44369:AI206835
F-HEMBA1006426//ESTs//3.0e-98:465:99//Hs.129251:AA993264
F-HEMBA1006438//EST//1.3e-29:183:93//Hs.147412:AI209194
F-HEMBA1006445
F-HEMBA1006446//EST//0.14:200:59//Hs.160695:AI282889
F-HEMBA1006461//Thiopurine S-methyltransferase//1.4e-29:210:72//Hs.51124:AF019369
F-HEMBA1006467
F-HEMBA1006471//ESTs//1.4e-05:391:60//Hs.121282:AI091453
F-HEMBA1006474//ESTs, Highly similar to 40 KD PROTEIN [Borna disease virus]//1.1e-13:346:63//Hs.31257:AA875998
F-HEMBA1006483//Thromboxane A2 receptor//2.2e-51:386:82//Hs.89887:D38081
F-HEMBA1006485//EST//5.4e-111:516:99//Hs.61925:AA039532
F-HEMBA1006486//EST//4.7e-23:286:72//Hs.137800:AA886897
F-HEMBA1006489//ESTs//2.5e-06:137:71//Hs.28621:AA910431
F-HEMBA1006492
F-HEMBA1006494//ESTs//8.5e -24:299:72//Hs.153413:AI248625
F-HEMBA1006497//EST//0.00034:431:61//Hs.130057:AA903389
F-HEMBA1006502//ESTs//2.6e-11:131:80//Hs.141267:H22072
F-HEMBA1006507//Homo sapiens mRNA for KIAA0666 protein, partial cds//7.3e-141:470:98//Hs.153858:AB014566
F-HEMBA1006521//ESTs, Weakly similar to 3-oxoacyl-[acyl-carrier protein] reductase [E.coli]//3.9e-98:483:97//Hs.94811:AA011185
F-HEMBA1006530//EST//1.7e-42:530:71//Hs.163207:AA808002
F-HEMBA1006535//ESTs//2.9e-84:404:98//Hs.128679:AI160081
F-HEMBA1006540//Homo sapiens multi PDZ domain protein MUPP1 (MUPP1) mRNA, complete cds//4.4e-173:654:98//Hs21301:AF093419
F-HEMBA1006546//ESTs//2.8e-45:391:78//Hs.146307:AA584638
F-HEMBA1006559//Homo sapiens KIAA0438 mRNA, complete cds//2.1e-47:363:79//Hs.21490:AB007898
F-HEMBA1006562//ESTs//4.5e-09:116:75//Hs.142368:AI198425
F-HEMBA1006566//EST//0.85:100:68//Hs.13052:T67136
F-HEMBA1006569//ESTs//2.7e-06:213:64//Hs.44372:AI346522
F-HEMBA1006579//EST//0.064:160:62//Hs.126244:AA873479
F-HEMBA1006583//Homo sapiens Jagged 2 mRNA, complete cds//1.7e-07:533:60//Hs.106387:AF029778
F-HEMBA1006595//Small inducible cytokine A5 (RANTES)//6.8e-69:328:81//Hs.155464:AF088219
F-HEMBA1006597//Homo sapiens mRNA for KIAA0752 protein, partial cds//2.6e-38:441:69//Hs.23711:AB018295
F-HEMBA1006612//ESTs//8.8e-135:668:97//Hs.7942:AA205862
F-HEMBA1006617//EST//4.6e-31:254:81//Hs.132635:AI032875
F-HEMBA1006624//ESTs, Weakly similar to HYPOTHETICAL 41.9 KD PROTEIN IN SDS3-THS1 INTERGENIC REGION [S.cerevisiae]//2.5e-75:379:97//Hs.40911:AI391502
F-HEMBA1006631//ESTs//1.4e-126:612:98//Hs.131737:AI343331
F-HEMBA1006635//EST//0.65:145:63//Hs.104560:AA340589
F-HEMBA1006639//ESTs, Highly similar to POLYADENYLATE-BINDING PROTEIN [Homo sapiens]//9.1e-27:170:92//Hs.109818:AA411185
F-HEMBA1006643//ESTs, Moderately similar to putative p150 [H.sapiens]//9.7e-05:259:65//Hs.105747:AA505003
F-HEMBA1006648//Homo sapiens integrin-linked kinase (ILK) mRNA, complete cds//3.9e-28:108:93//Hs.6196:U40282
F-HEMBA1006652//ESTs, Highly similar to 60S RIBOSOMAL PROTEIN L7 [Drosophila melanogaster]//3.0e-87:452:96//Hs.159574:AA190615
F-HEMBA1006653
F-HEMBA1006659//Homo sapiens PAC clone DJ0905J08 from 7p12-p14//2.9e-92:438:98//Hs.8173:AC005189
F-HEMBA1006665//Homo sapiens clone 23892 mRNA sequence//2.8e-18:180:80//Hs.91916:AF035317
F-HEMBA1006674//Homo sapiens mRNA for nucleolar protein hNop56//1.6e-16:122:90//Hs.5092:Y12065
F-HEMBA1006676
F-HEMBA1006682//EST//0.12:193:61//Hs.128367:AA974575
F-HEMBA1006695//ESTs//5.6e-27:110:80//Hs.159510:AA297145
F-HEMBA1006696//EST//3.2e-12:160:75//Hs.146472:AI128198
F-HEMBA1006708
F-HEMBA1006709//ESTs//0.69:60:80//Hs.152752:AA643545
F-HEMBA1006717//ESTs/12.6e-31:286:78//Hs.55573:W37226
F-HEMBA1006737//ESTs//1.6e-37:189:99//Hs.97490:AA394105
F-HEMBA1006744//Human mRNA for KIAA0118 gene, partial cds//1.9e-52:360:84//Hs.154326:D42087
F-HEMBA1006754//Homo sapiens X-ray repair cross-complementing protein 2 (XRCC2) mRNA, complete cds//2.0e-92:817:78//Hs.129727:AF035587
F-HEMBA1006758//Human mRNA for KIAA0327 protein, complete cds//4.0e-10:576:56//Hs.149323:AB002325
F-HEMBA1006767//ESTs//1.7e-18:252:72//Hs141073:W72720
F-HEMBA1006779//EST//9.1e-26:395:69//Hs.145366:AI252657
F-HEMBA1006780//EST//1.0:93:69//Hs.116946:AA680250
F-HEMBA1006789//ESTs//0.0060:276:59//Hs.144121:AI369798
F-HEMBA1006795//Human Line-1 repeat mRNA with 2 open reading frames//4.1e-37:781:64//Hs.23094:M19503
F-HEMBA1006796//Human clone 23803 mRNA, partial cds//1.4e-07:202:68//Hs.34054:U79298
F-HEMBA1006807//ESTs, Moderately similar to HYPOTHETICAL 46.4 KD PROTEIN T16H12.5 IN CHROMOSOME III [C.elegans]//4.8e-110:523:98/IHs.125790:AA287723
F-HEMBA1006821//EST//5.1e-ll:246:66//Hs.150542:AI051551
F-HEMBA1006824//ESTs//1.4e-29:158:98//Hs.127712:AA961624
F-HEMBA1006832//EST//3.1e-24:277:74//Hs.139357:AA420970
F-HEMBA1006849//ESTs//0.99:332:57//Hs.128993:AA985327
F-HEMBA1006865
F-HEMBA1006877//ESTs, Highly similar to HYPOTHETICAL 113.8 KD PROTEIN IN ERG7-NMD2 INTERGENIC REGION [Saccharomyces cerevisiae]//2.4e-61:311:97//Hs.127793:W25938
F-HEMBA1006885//ESTs, Highly similar to HYPOTHETICAL 29.1 KD PROTEIN IN URA7-POL12 INTERGENIC REGION [Saccharomyces cerevisiae]//9.1e-128:805:87//Hs.32376:AA758214
F-HEMBA1006900//EST//6.8e-05:255:63//Hs.163173:AA781592
F-HEMBA1006914//EST//0.065 :366:6211Hs.162914:AA666199
F-HEMBA1006921//ESTs//2.9e-42:347:82//Hs.159266:AI376989
F-HEMBA1006926//Human I kappa BR mRNA, complete cds//0.90:545:59//Hs.154764:U16258
F-HEMBA1006929//EST//0.00013:403:61//Hs.162642:AA602539
F-HEMBA1006936//ESTs//0.00014:60:93//Hs.8737:W22712
F-HEMBA1006938//ESTs//4.7e-51:256:98//Hs.143651:AI150382
F-HEMBA1006941//Homo sapiens mRNA for putative thioredoxin-like protein//4.4e-92:437:98//Hs.42644:AJ010841
F-HEMBA1006949//H.sapiens mRNA for retrotransposon//6.9e-43:385:76//Hs.6940:Z48633
F-HEMBA1006973//Homo sapiens rab3-GAP regulatory domain mRNA, complete cds//1.8e-144:740:94//Hs.14934:AF004828
F-HEMBA1006976//H.sapiens mRNA for Gal-beta(1-3/1-4)GlcNAc alpha-2.3-sialyltransferase//1.9e-79:447:89//Hs.75268:X74570
F-HEMBA1006993//ESTs//5.4e-19:380:66//Hs.152635:AA600968
F-HEMBA1006996//ESTs//0.17:242:59//Hs.106879:AA054723
F-HEMBA1007002
F-HEMBA1007017//EST//1.0:59:72//Hs.113400:R39282
F-HEMBA1007018//Homo sapiens dynein light intermediate chain 2 (LIC2) mRNA, complete cds//2.5e-78:827:70//Hs.43003:AF035812
F-HEMBA1007045
F-HEMBA1007051//EST//0.85:65:73//Hs.158641:AI370659
F-HEMBA1007052
F-HEMBA1007062
F-HEMBA1007066//ESTs//0.94:160:63//Hs.56071:W52212
F-HEMBA1007073//ESTs//3.6e-50:246:80//Hs.142678:H37845
F-HEMBA1007078//Human arginine-rich nuclear protein mRNA, complete cds//6.7e-75:417:91//Hs.80510:M74002
F-HEMBA1007080
F-HEMBA1007085//Guanylate cyclase 2D, membrane (retina-specific)//1.3e-06:568:61//Hs.1974:M92432
F-HEMBA1007087//Human mevalonate pyrophosphate decarboxylase (MPD) mRNA, complete cds//0.95:541:57//Hs.3828:U49260
F-HEMBA1007112//ESTs//3.4e-104:494:98//Hs.19207:AA039595
F-HEMBA1007113//ESTs//0.71:246:62//Hs.96235:AA196354
F-HEMBA1007121//ESTs/l3.Se-69:335:98//Hs.140519:AA643182
F-HEMBA1007129
F-HEMBA1007147//ESTs//3.2e-07:235:641/Hs.124813:W46172
F-HEMBA1007149//ESTs//7.2e-08:161:68//Hs.121179:AA757136
F-HEMBA1007151
F-HEMBA1007174//Homo sapiens epsin 2b mRNA, complete cds//6.6e-64:318:97//Hs.22396:AF062085
F-HEMBA1007178//ESTs, Moderately similar to !!!! ALU SUBFAMILY J WARNING ENTRY !!!! [H.sapiens]//4.2e-39:248:90//Hs.157148:AA311921
F-HEMBA1007194//ESTs//2.3e-107:503:99//Hs.100605:AA305965
F-HEMBA1007203//Homo sapiens mRNA for KIAA0214 protein, complete cds//5.6e-158:478:98//Hs.3363:D86987
F-HEMBA1007206//EST//0.23:119:66//Hs.144402:AA609252
F-HEMBA1007224//Homo sapiens mRNA for KIAA0797 protein, partial cds//1.6e-177:839:98//Hs.27197:AB018340
F-HEMBA1007243//Hypoxanthine phosphoribosyltransferase 1 (Lesch-Nyhan syndrome)//2.7e-56:647:69//Hs.82314:M31642
F-HEMBA1007251//Human plectin (PLEC1) mRNA, complete cds//0.19:210:67//Hs.79706:U53204
F-HEMBA1007256//Homo sapiens clone 24407 mRNA sequence//1.0:144:6411Hs.12432:AF070575
F-HEMBA1007267//Human homolog of yeast mutL (hPMS1) gene, complete cds//0.99:239:60//Hs.111749:U13695
F-HEMBA1007273//ESTs//5.6e-24:271:73//Hs.144951:N34836
F-HEMBA1007279//ESTs//6. 1e-36:185:78//Hs.141022:H06475
F-HEMBA1007281//ESTs//0.74:94:65//Hs.162533:AA584529
F-HEMBA1007288//EST//0.83:99:67//Hs.127878:AA968637
F-HEMBA1007300//EST//3.6e-62:355:91//Hs.150139:AI300062
F-HEMBA1007301//Collagen, type I, alpha 1//1.5e-09:406:61//Hs.111913:Z74615
F-HEMBA1007319//EST//0.0068:50:96//Hs.163362:AA890506
F-HEMBA1007320//ESTs//1.0:133:66//Hs.38032:N63634
F-HEMBA1007322//ESTs//0.0077:187:66//Hs.4852:R84241
F-HEMBA1007327//ESTs, Weakly similar to HOST CELL FACTOR C1 [H.sapiens]//3.5e-09:144:76//Hs.20597:W58370
F-HEMBA1007341//ESTs//7.5e-61:302:98//Hs.154944:AA494130
F-HEMBA1007342//ESTs//2.9e-12:289:64//Hs.135555:AA911006
F-HEMBA1007347//EST//0.44:89:70//Hs.65949:Z40561
F-HEMBB1000005//ESTs//1.6e-07:337:60//Hs.126718:AA916568
F-HEMBB1000008//H.sapiens mRNA for translin associated protein X//1.1e-43:370:78//Hs.96247:X95073
F-HEMBB1000018//Nuclear factor of kappa light polypeptide gene enhancer in B-cells 1 (p105)//1.0:108:70//Hs.83428:M58603
F-HEMBB1000024//EST//5.4e-07:137:70//Hs.125389:AA878307
F-HEMBB1000025//EST//0.99:362:58//Hs.121221:AA757392
F-HEMBB1000030//H.sapiens mRNA for cylicin II//1.3e-10:525:62//Hs.3232:Z46788
F-HEMBB1000036
F-HEMBB1000037//Homo sapiens erythroblast macrophage protein EMP mRNA, complete cds//6.2e-102:450:98//Hs.20815:AF084928
F-HEMBB1000039//EST//0.0034:97:73//Hs.141684:W35358
F-HEMBB1000044//ESTs//0.0048:218:63//Hs.123161:AA807319
F-HEMBB1000048//EST//0.00025:222:62//Hs.122474:AA765131
F-HEMBB1000050//ESTs//5.6e-28:293:75//Hs.136839:H93717
F-HEMBB1000054//Human Line-1 repeat mRNA with 2 open reading frames//3.3e-54:259:88//Hs.23094:M19503
F-HEMBB1000055//ESTs//0.0017:289:62//Hs.125755:AA286923
F-HEMBB1000059//Homo sapiens mRNA for KIAA0761 protein, partial cds//5.9e-59:286:84//Hs.93121:AB018304
F-HEMBB1000083
F-HEMBB1000089//EST//0.0016:192:661/Hs.137093:AA917621
F-HEMBB1000099//ESTs//5.7e-20:213:76//Hs.57883:AA218645
F-HEMBB1000103//Human kpni repeat mrna (cdna clone pcd-kpni-8), 3' end//4.9e-43:418:74//Hs.103948:K00627
F-HEMBB1000113//EST//4.6e-23:221:76//Hs.142065:AA173763
F-HEMBB1000119//Homo sapiens ASMTL gene//2.5e-132:621:98//Hs.6315:Y15521
F-HEMBB1000136//ESTs112.3e-101:507:96//Hs.12659:AA195207
F-HEMBB1000141//ESTs//2.1e-15:283:69//Hs.126257:AI279044
F-HEMBB1000144//EST//4.5e-52:298:91//Hs.149580:AI281881
F-HEMBB1000173//Zinc finger protein 74 (Cos52)//2.4e-63:285:82//Hs.3057:X92715
F-HEMBB1000175//EST//1.0:101:65//Hs.162898:AA659646
F-HEMBB1000198//EST//0.99:179:56//Hs.116880:AA662457
F-HEMBB1000215//Homo sapiens mRNA for KIAA0557 protein, partial cds//1.4e-15:139:82//Hs.101414:AB011129
F-HEMBB1000217//ESTs//3.4e-06:81:88//Hs,121151:T66277
F-HEMBB1000218//EST//0.11:136:63//Hs.134683:AI092013
F-HEMBB1000226//Fragile X mental retardation 1//0.99:126:65//Hs.89764:X69962
F-HEMBB1000240//H.sapiens mRNA for Nup88 protein//1.0:334:57//Hs.90734:Y08612
F-HEMBB1000244//ESTs//3.2e-15:139:81//HS.134549:AI078483
F-HEMBB1000250//Homo sapiens protein associated with Myc mRNA, complete cds//2.1e-156:735:981/Hs.151411:AF075587
F-HEMBB1000258//EST//0.0091:325:60//Hs.97533:AA435884
F-HEMBB1000264//Human CHL1 potential helicase (CHLR1), complete cds//1.4e-33:100:100//Hs.27424:U75968
F-HEMBB1000266//Homo sapiens mRNA for myosin phosphatase target subunit 1 (MYPT1)//0.0019:373:60//Hs.16533:D87930
F-HEMBB1000272//ESTs//1.3e-93:440:99//Hs.l09224:N46684
F-HEMBB1000274//ESTs//0.41:221:65//Hs.71990:AA151796
F-HEMBB1000284//EST//0.00024:108:73//Hs.100725:F13689
F-HEMBB1000307//EST//3.6e-10:149:73//Hs.140415:AA778574
F-HEMBB1000312//Homo sapiens mRNA for KIAA0783 protein, complete cds//0.00092:252:65//Hs.41153:AB018326
F-HEMBB1000317//Thrombospondin 1//7.1e-05:342:59//Hs.87409:X14787
F-HEMBB1000318//EST//0.014:184:61//Hs.155758:AI311870
F-HEMBB1000335//EST//0.99:187:63//Hs.137424:AA243729
F-HEMBB1000336//EST//1.0:209:63//Hs.150410:AI003611
F-HEMBB1000337//EST//0.086:133:66//Hs.128207:AA972330
F-HEMBB1000338//EST//7.1e-07:129:72//Hs.140488:AA767127
F-HEMBB1000339//Small inducible cytokine A5 (RANTES)//1.2e-36:336:7611Hs.155464:AF088219
F-HEMBB1000341
F-HEMBB1000343//EST//0.66:163:63//Hs.150822:AI302729
F-HEMBB1000354//ESTs//7.e-61:292:100//Hs.152266:AA926874
F-HEMBB1000369//ESTs, Highly similar to t-BOP [M.musculus]/10.013:157:64//Hs.129982:AI420970
F-HEMBB1000374//ESTs//8.7e-53:454:79//Hs.133518:R69934
F-HEMBB1000376//ESTs//5.9e-14:87:97//Hs.163973:AA744348
F-HEMBB1000391//ESTs//0.033:237:64//Hs.135289:AI092963
F-HEMBB1000399//Homo sapiens mRNA for cell cycle checkpoint protein//9.4e-165:762:98//Hs.16184:AJ001642
F-HEMBB1000402//EST//0.013:291:59//Hs.149191:AI246155
F-HEMBB1000404//ESTs//3.0e-69:353:96//Hs.135857:AA947194
F-HEMBB1000420//EST//6.3e-52:258:98//Hs.136434:AA557925
F-HEMBB1000434//Homo sapiens neuronal thread protein AD7c-NTP mRNA, complete cds//9.4e-73:364:83//Hs.129735AF010144
F-HEMBB1000438//ESTs//0.073:446:58//Hs.134632:AI223429
F-HEMBB1000441//Interleukin 10//1.7e-38:336:77//Hs.2180:M57627
F-HEMBB1000449//EST//5.5e-21:356:671/Hs.157848:AI362501
F-HEMBB1000455//ESTs//0.092:147:65//Hs.106446:N93227
F-HEMBB1000472
F-HEMBB1000480//EST//0.98:83:71//Hs.146462:AI124898
F-HEMBB1000487//ESTs//1.4e-59:341:92//Hs.48561:N79206
F-HEMBB1000490//ESTs//2.5e-27:200:79//Hs.56825:AI057560
F-HEMBB1000491
F-HEMBB1000493//ESTs//0.019:103:69//Hs.138358:T66178
F-HEMBB1000510//Glucocorticoid receptor alpha {alternative products}//1.6e-46:409:77//Hs.102761:U25029
F-HEMBB1000518//ESTs//3.7e-06:187:64//Hs.140989:R68413
F-HEMBB1000523//ESTs//0.69:332:59//Hg.106845:W19543
F-HEMBB1000530//H.sapiens mRNA for extracellular matrix protein collagen type XIV, C-terminus//2.1e-38:138:96//Hs.36131:Y11710
F-HEMBB1000550//ESTs, Weakly similar to !!!! ALU SUBFAMILY SC WARNING ENTRY !!!! [H.sapiens]//7.7e-31:554:67//Hs.157142:U85996
F-HEMBB1000554//Homo sapiens mRNA, chromosome 1 specific transcript KIAA0484//4.0e-27:282:75//Hs.158095:AB007953
F-HEMBB1000556//Homo sapiens mRNA for KIAA0750 protein, complete cds//2.0e-33:537:65//Hs.5444:AB018293
F-HEMBB1000564
F-HEMBB1000573//H.sapiens HCG II mRNA//7.5e-27:197:76//Hs.146333:X81001
F-HEMBB1000575//Von Hippel-Lindau syndrome//2.7e-72:255:79//Hs.78160:AF010238
F-HEMBB1000586//Dystrophin (muscular dystrophy, Duchenne and Becker types), includes DXS142, DXS164, DXS206, DXS230, DXS239, DXS268, DXS269, DXS270, DXS272//0.011:338:59//Hs.79012:M18533
F-HEMBB10005891/PLATELET GLYCOPROTEIN V PRECURSOR//2.4e-22:228:79//Hs.73734:Z23091
F-HEMBB1000591//ESTs//1.0e-17:370:64//Hs.58156:W71990
F-HEMBB1000592//EST//0.0038:51:88//Hs.148022:AI269323
F-HEMBB1000593//Homo sapiens chromosome 7q22 sequence//4.7e-109:503:99//Hs.3386:AF053356
F-HEMBB1000598//Ribosomal protein L5//3.5e-29:537:66//Hs.118781:U66589
F-HEMBB1000623//H.sapiens mRNA for GAIP protein//0.89:376:59//Hs.22698:X91809
F-HEMBB1000630//Homo sapiens KIAA0404 mRNA, partial cds//0.074:168:61//Hs.105850:AB007864
F-HEMBB1000631//ESTs//1.7e-06:247:64//Hs.156864:AI346481
F-HEMBB1000632//Human mRNA for KIAA0351 gene, complete cds//5.1e-50:811:65//Hs.29963:AB002349
F-HEMBB1000637//Sialophorin (gpL115, leukosialin, CD43)//2.4e-79:304:85//Hs.80738:X52075
F-HEMBB1000638//EST//0.0076:92:75//Hs.125496:AA883735
F-HEMBB1000643//ISLET AMYLOID POLYPEPTIDE PRECURSOR//3.5e-45:477:74//Hs.51048:X68830
F-HEMBB1000649//Homo sapiens histone H2A.1b mRNA, complete cds//7.4e-52:533:75//Hs.51011:L19778
F-HEMBB1000652//ESTs//1.6e-49:345:84//Hs.132722:AA618531
F-HEMBB1000665//EST//0.44:152:63//Hs.149534:AI280924
F-HEMBB1000671//Human Line-1 repeat mRNA with 2 open reading frames//2.2e-79:280:85//Hs.23094:M19503
F-HEMBB1000673//ESTs//0.99:177:59//Hs.149864:N80474
F-HEMBB1000684//Protein kinase, interferon-inducible double stranded RNA dependent//2.6e-31:220:87//Hs.73821:M35663
F-HEMBB1000693//Homo sapiens neuroanl mRNA, complete cds//5.3e-120:575:97//Hs.158300:AF040723
F-HEMBB1000705//ESTs//4.7e-65:350:94//Hs.24610:R33125
F-HEMBB1000706//EST//8.6e-14:373:61//Hs.138281:RS5703
F-HEMBB1000709//EST//0.99:110:651/Hs.162437:AA577510
F-HEMBB1000725//RAS-RELATED PROTEIN RAB-8//1.7e-77:635:77//Hs.123109:X56741
F-HEMBB1000726//EST//1.3e-43:257:84//Hs.162197:AA535216
F-HEMBB1000738//EST//5.9e-13:259:64//Hs.159699:AI417328
F-HEMBB1000749//EST//3.1e-42:271:871/Hs.162197:AA535216
F-HEMBB1000763
F-HEMBB1000770//ESTs, Weakly similar to MOESIN/EZRIN/RADIXIN HOMOLOG [D.melanogaster]//0.021:111:72//Hs.38178:AA921830
F-HEMBB1000774//ESTs, Weakly similar to mTERF [H.sapiens]//2.5 e-116:580:97//Hs.5009:AA081390
F-HEMBB1000781//Human MEK kinase 3 mRNA, complete cds//5.3e-47:426:74//Hs.86201:U78876
F-HEMBB1000789//Homosapiens mRNA for KIAA0677 protein, complete cds//3.0e-65:672:71//Hs.155983:AB014577
F-HEMBB1000790//ESTs//1.2e-52:344:86//Hs.35254:AI133727
F-HEMBB1000794//ESTs//0.00098:289:59//Hs.138782:N73572
F-HEMBB1000807//ESTs//2.1e-91:434:99//Hs.61334:AI298375
F-HEMBB1000810//ESTs//0.038:92:71//Hs.148763:AA66887
F-HEMBB1000821//EST//0.94:129:62//Hs.162299:AA555154
F-HEMBB1000822//ESTs//7.5e-05:199:63//Hs.117018:AA832421
F-HEMBB1000826//ESTs//4.8e-13:343:65//Hs.153429:AI283069
F-HEMBB1000827
F-HEMBB1000831
F-HEMBB1000835//EST//4.3e-27:201:851/Hs.141451:N29915
F-HEMBB1000840//EST//6.3e-75:380:96//Hs.142557:AA464948
F-HEMBB1000848//Human Line-1 repeat mRNA with 2 open reading frames//1.4e-135:875:85//Hs.23094:M19503
F-HEMBB1000852//Phosphoribosyl pyrophosphate amidotransferase//0.12:292:61//Hs.311:U00238
F-HEMBB1000870//EST//0.00091:246:62//Hs.126502:AA913831
F-HEMBB1000876//Homo sapiens ELISC-1 mRNA, partial cds//4.9e-34:200:94//Hs.128434:AF085351
F-HEMBB1000883//ESTs//0.42:107:67//Hs.154173:AI379823
F-HEMBB1000887
F-HEMBB1000888//ESTs//1.0:137:67//Hs.8121:AA521290
F-HEMBB1000890//ESTs//1.0:116:65//Hs.7105:T23433
F-HEMBB1000893//EST//0.0079:408:58//Hs.146504:AI129834
F-HEMBB1000908//EST//9.2e-21:205:79//Hs.132635:AI032875
F-HEMBB1000910//Human mRNA for KIAA0231 gene, partial cds//0.16:327:60//Hs.7938:D86984
F-HEMBB1000913//ESTs//1.0e-12:233:68//Hs.137545:AA487049
F-HEMBB1000915//ESTs//2.5e-90:423:99//Hs.135254:AI095468
F-HEMBB1000917//EST//2.8e-49:241:100//Hs.162216:AA548089
F-HEMBB1000927//Hippocalcin//1.2e-31:528:65//Hs.89692:D16593
F-HEMBB1000947
F-HEMBB1000959//Cytochrome P450, 51 (lanosterol 14-alpha-demethylase)//9.3e-48:572:72//Hs.2379:U23942
F-HEMBB1000973//ESTs//4.5e-26:286:76//Hs.137393:AA142938
F-HEMBB1000975//ESTs//0.78:180:66//Hs.104789:AA417124
F-HEMBB1000981
F-HEMBB1000985//Homo sapiens actin binding protein MAYVEN mRNA, complete cds//6.7e-07:308:62//Hs.122967:AF059569
F-HEMBB1000991//EST//0.12:125:66//Hs.22945:R43713
F-HEMBB1000996//ESTs//6.9e-05:273:63//Hs.133116:AI054055
F-HEMBB1001004//Homo sapiens mRNA for KIAA0665 protein, complete cds//0.62:193:62//Hs.119004:AB014565
F-HEMBB1001008//EST//4.7e-09:203:65//Hs.105221:AA489025
F-HEMBB1001011//Human Chromosome 16 BAC clone CIT987SK-A-635H12//2.4e-17:384:67//Hs.108604:AC002310
F-HEMBB1001014//EST, Weakly similar to putative p150 [H.sapiens]//0.21:284:60//Hs.161547:W04991
F-HEMBB1001020//ESTs//9.7e-37:186:76//Hs.138852:AA284247
F-HEMBB1001024//ESTs, Highly similar to t-BOP [M.musculus]//0.11:242:61//Hs.129982:AI420970
F-HEMBB1001037//EST//0.0057:192:66//Hs.149987:AI291177
F-HEMBB1001047//ESTs//1.6e-22:360:70//Hs.120734:W58721
F-HEMBB1001051//H.sapiens mRNA for FAN protein//3.8e-29:160:98//Hs.78687:X96586
F-HEMBB1001056//Homo sapiens mRNA for KIAA0618 protein, complete cds//1.0e-42:149:96//Hs.15832:AB014518
F-HEMBB1001058//Small inducible cytokine A5 (RANTES)//1.1e-45:349:82//Hs.155464:AF088219
F-HEMBB1001060//ESTs//1.6e-62:464:81//Hs.138663:N24942
F-HEMBB1001063
F-HEMBB1001068//Homo sapiens liprin-beta2 mRNA, partial cds//9.9e-148:736:95//Hs.12953:AF034803
F-HEMBB1001096//EST//0.017:154:66//Hs.130403:AA909272
F-HEMBB1001102//ESTs//2.1e-18:120:95//Hs.163767:R06293
F-HEMBB1001105//Human BRCA2 region, mRNA sequence
CG016//0.30:84:75//Hs.112434:U50529
F-HEMBB1001112//ESTs, Highly similar to PROTEIN TRANSPORT PROTEIN SEC61 ALPHA SUBUNIT [Canis familiaris]//9.3e-38:341:77//Hs.14038:R06800
F-HEMBB1001114//EST//6.4e-07:296:62//Hs.128420:AA975062
F-HEMBB1001117//EST//1.6e-99:464:99//Hs.130493:AA928139
F-HEMBB1001119
F-HEMBB1001126
F-HEMBB1001133//H.sapiens mRNA for translin associated protein X//1.2e-28:739:61//Hs.96247:X95073
F-HEMBB1001137
F-HEMBB1001142//Human mRNA for KIAA0331 gene, complete cds//2.1e-23:340:69//Hs.146395:AB002329
F-HEMBB1001151//ESTs//2.6e-30:252:79//Hs.6880:W26854
F-HEMBB1001153//ESTs//7.6e-16:97:96//Hs.113307:H16716
F-HEMBB1001169//ESTs//1.4e-32:374:71//Hs.161682:AA206863
F-HEMBB1001175//Human mRNA for ankyrin motif, complete cds//7.1e-36:509:66//Hs.73073:D78334
F-HEMBB1001177//ESTs, Weakly similar to HYPOTHETICAL TRP-ASP REPEATS CONTAINING PROTEIN IN HXT14-PHA2 INTERGENIC REGION [S.cerevisiae]//1.5e-65:312:100//Hs.86878:AA599183
F-HEMBB1001182//Electron-transfer-flavoprotein, beta polypeptide//0.94:199:64//Hs.74047:X71129
F-HEMBB1001199
F-HEMBB1001208//ESTs//0.12:120:69//Hs.130093:AA928802
F-HEMBB1001209//EST//0.00028:215:65//Hs.118276:W15258
F-HEMBB1001210//EST//2.9e-05:297:60//Hs.88840:AA281452
F-HEMBB1001218//Homo sapiens mRNA for KIAA0585 protein, partial cds//8.5e-37:260:76//Hs.72660:AB011157
F-HEMBB1001221//Homo sapiens mRNA, chromosome 1 specific transcript KIAA0507//0.00046:650:58//Hs.158241:AB007976
F-HEMBB1001234//ESTs, Highly similar to 65 KD YES-ASSOCIATED PROTEIN [Mus musculus]//6.7e-103:477:100//Hs.127835:AI378790
F-HEMBB1001242//Homo sapiens mRNA for LAK-1, complete cds//1.2e-31:458:67//Hs.129918:AB005754
F-HEMBB1001249//EST//0.26:203:63//Hs.140791:AA935909
F-HEMBB1001253//ESTs//4.0e-91:433:98//Hs.120636:AA325219
F-HEMBB1001254//ESTs//2.0e-24:180:85//Hs.136391:H04977
F-HEMBB1001267//Ataxia telangiectasia mutated (includes complementation groups A, C and D)//6.1e-24:146:78//Hs.51187:U82828
F-HEMBB1001271//ESTs//2.5e-05:686:58//Hs.115423:AI359248
F-HEMBB1001282//GA-binding protein transcription factor, beta subunit 2 (47kD)//0.39:531:57//Hs.78915:U13045
F-HEMBB1001288//ESTs, Highly similar to HYPOTHETICAL 27.3 KD PROTEIN ZK353.7 IN CHROMOSOME III [Caenorhabditis elegans]//4.9e-10:91:89//Hs.16606:W81021
F-HEMBB1001289//ESTs//6.4e-100:467:99//Hs.151720:AI287890
F-HEMBB1001294//ESTs, Highly similar to RAS-LIKE PROTEIN TC10 [Homo sapiens]//1.3e-135:654:98//Hs.124217:AA020848
F-HEMBB1001302
F-HEMBB1001304//ESTs//0.98:109:68//Hs.138972:AA047725
F-HEMBB1001314//ESTs//7.4e-39:285:77//Hs.144749:AI217339
F-HEMBB1001315//Small inducible cytokine A5 (RANTES)//1.9e-40:355:78//Hs.155464:AF088219
F-HEMBB1001317//Human Line-1 repeat mRNA with 2 open reading frames//4.7e-98:625:85//Hs.23094:M19503
F-HEMBB1001326//ESTs//0.00030:257:63//Hs.62208:H12380
F-HEMBB1001331//ESTs, Weakly similar to DFS70 [H.sapiens]//1.0e-48:332:87//Hs.43071:AA206222
F-HEMBB1001335
F-HEMBB1001337//Homo sapiens mRNA for KIAA0563 protein, complete cds//8.5e-56:282:87//Hs.15731:AB011135
F-HEMBB1001339//Homo sapiens antigen NY-CO-16 mRNA, complete cds//0.039:161:65//Hs.132206:AF039694
F-HEMBB1001346//Oxytocin receptor//4.2e-42:456:73//Hs.2820:X64878
F-HEMBB1001348//Homo sapiens mRNA for KIAA0570 protein, complete cds//1.2e-45:176:77//Hs.114293:AB011142
F-HEMBB1001356//EST//0.32:292:59//Hs.135771:AI005648
F-HEMBB1001364
F-HEMBB1001366//EST//7.8e-24:367:69//Hs.138765:N70347
F-HEMBB1001367//Small inducible cytokine A5 (RANTES)//8.7e-50:326:86//Hs.155464:AF088219
F-HEMBB1001369//EST//0.17:211:63//Hs.120066:AA707973
F-HEMBB1001380//Homo sapiens mRNA for KIAA0527 protein, partial cds//8.2e-36:225:79//Hs.129748:AB011099
F-HEMBB1001384
F-HEMBB1001387//ESTs//0.61:215:60//Hs.145915:AI342230
F-HEMBB1001394//Human Line-1 repeat mRNA with 2 open reading frames//3.8e-94:568:83//Hs.23094:M19503
F-HEMBB1001410//Homo sapiens keratan sulfate proteoglycan mRNA, complete cds//0.021:373:58//Hs.125750:AF065988
F-HEMBB1001424//EST//0.20:307:58//Hs.135336:AI049827
F-HEMBB1001426//Homo sapiens clone 23579 mRNA sequence//8.3e-17:205:72//Hs.83466:AF038174
F-HEMBB1001429//ESTs, Highly similar to CYTOSOL AMINOPEPTIDASE [Bos taurus]//5.5e-153:729:96//Hs.21679:AF034175
F-HEMBB1001436//Human mRNA for KIAA0347 gene, complete cds//1.2e-44:316:85//Hs.101996:AB002345
F-HEMBB1001443
F-HEMBB1001449//Homo sapiens sodium bicarbonate cotransporter (HNBC1) mRNA, complete cds//0.033:478:58//Hs.5462:AF007216
F-HEMBB1001454//ESTs//1.4e-46:279:93//HS.104866:AA426038
F-HEMBB1001458//EST//1.7e-09:106:83//Hs.141422:N20920
F-HEMBB1001463//Homo sapiens mRNA for semaphorin E, complete cds//0.18:387:59//Hs.62705:AB000220
F-HEMBB1001464//Homo sapiens Coch-5B2 mRNA, complete cds//0.26:189:67//Hs.21016:AF006740
F-HEMBB1001482//Homo sapiens mRNA for KIAA0760 protein, partial cds//1.2e-27:292:74//Hs.137168:AB018303
F-HEMBB1001500//ESTs//8.1e-28:312:74//Hs.18498:N52088
F-HEMBB1001521//Homo sapiens mRNA for alpha(1,2)fucosyltransferase, complete cds//8.8e-54:359:74//Hs.46328:D87942
F-HEMBB1001527//Protein tyrosine phosphatase, receptor type, f polypeptide//1.0:198:63//Hs.75216:Y00815
F-HEMBB1001531//ESTs//4.3e-33:403:75//Hs.44862:N38735
F-HEMBB1001535//ESTs//0.0029:47:93//Hs.124864:AA663093
F-HEMBB1001536//ESTs//0.0047:120:68//Hs.144858:R67748
F-HEMBB1001537//ESTs, Weakly similar to eukaryotic initiation factor eIF-2 alpha kinase [D.melanogaster]//3.7e-20:297:73//Hs.42457:AA523306
F-HEMBB1001555//Human ring zinc-finger protein (ZNF127-Xp) gene and 5' flanking sequence//1.1e-35:188:77//Hs.102877:U41315
F-HEMBB1001562//ESTs//0.95:161:61//Hs.145075:AI208240
F-HEMBB1001564//Homo sapiens mRNA, chromosome 1 specific transcript KIAA0484//3.4e-49:526:73//Hs.158095:AB007953
F-HEMBB1001565//Homo sapiens PYRIN (MEFV) mRNA, complete cds//1.9e-44:324:84//Hs.113283:AF018080
F-HEMBB1001585
F-HEMBB1001586//EST//0.84:132:64//Hs.145264:AI218708
F-HEMBB1001588//Human clone 23695 mRNA sequence//6.6e-20:327:67//Hs.90798:U79289
F-HEMBB1001603//ESTs//1.3e-12:84:96//Hs.13380:R60414
F-HEMBB1001618//ESTs//4.4e-11:349:63//Hs.132046:AA693680
F-HEMBB1001619//ESTs//2.1e-06:246:63//Hs.63428:AA058314
F-HEMBB1001630//EST//1.4e-07:334:62//Hs.145698:AI266713
F-HEMBB1001635//ESTs//0.92:282:60//Hs.126980:AA934077
F-HEMBB1001637//ELK1, member of ETS oncogene family//1.1e-27:395:64//Hs.116549:AL009172
F-HEMBB1001641//EST//0.11:53:81//Hs.112445:AA594279
F-HEMBB1001653//EST//0.91:124:64//Hs.144213:T40480
F-HEMBB1001665//Human mRNA for apolipoprotein E receptor 2, complete cds//7.0e-13:473:63//Hs.54481:D86407
F-HEMBB1001668//ESTs//0.94:83:69//Hs.146202:AI252519
F-HEMBB1001673//Homo sapiens mRNA for KIAA0646 protein, complete cds//2.3e-172:803:98//Hs.24439:AB014546
F-HEMBB1001684//ESTs, Highly similar to Tbc1 [M.musculus]//5.4e-20:110:100//Hs.106104:AA599496
F-HEMBB1001685//EST//2.2e-05:112:73//Hs.130984:AI015430
F-HEMBB1001695//Human novel homeobox mRNA for a DNA binding protein//1.6e-08:425:62//Hs.37035:U07664
F-HEMBB1001704//EST//5.8e-20:295:69//Hs.140231:AI054398
F-HEMBB1001706
F-HEMBB1001707//EST//0.091:241:60//Hs.136830:AA769219
F-HEMBB1001717//ESTs//2.9e-06:325:60//Hs.150063:AI298064
F-HEMBB1001735//Small inducible cytokine A5 (RANTES)//3.2e-46:326:83//Hs.155464:AF088219
F-HEMBB1001736//ESTs, Weakly similar to E04D5.1 [C.elegans]//5.4e-99:485:97//Hs.120581:W25578
F-HEMBB1001747//ESTs//8.3e-87:421:98//Hs.137051:AA884244
F-HEMBB1001749//Homo sapiens neuronal thread protein AD7c-NTP mRNA, complete cds//3.5e-75:315:83//Hs.129735:AF010144
F-HEMBB1001753//ESTs//0.00013:35:100//Hs.139643:H06263
F-HEMBB1001756//ESTs//2.3e-89:433:98//Hs.128868:AA931077
F-HEMBB1001760//ESTs//6.5e-06:503:58//Hs.21766:AI357639
F-HEMBB1001762//Homo sapiens mRNA, chromosome 1 specific transcript KIAA0507//2.9e-13:498:60//Hs.158241:AB007976
F-HEMBB1001785//EST//0.16:262:60//Hs.162526:AA584102
F-HEMBB1001797//ESTs//0.37:201:63//Hs.91559:AA806370
F-HEMBB1001802//ESTs//1.6e-06:447:58//Hs.134672:AI087951
F-HEMBB1001812//Homo sapiens mRNA, chromosome 1 specific transcript KIAA0487//1.3e-54:311:81//Hs.92381:AB007956
F-HEMBB1001816//ESTs//2.2e-39:302:84//Hs.35985:AA783017
F-HEMBB1001831//Homo sapiens PAM COOH-terminal interactor protein 1 (PCIP1) mRNA, complete cds//7.6e-164:763:98//Hs.159396:AF056209
F-HEMBB1001834//TRICHOHYALIN//7.1e-05:548:60//Hs.82276:L09190
F-HEMBB1001836//Human mRNA for KIAA0033 gene, partial cds//4.0e-34:272:86//Hs.22271:D26067
F-HEMBB1001839//Pyruvate carboxylase//0.050:686:59//Hs.89890:S72370
F-HEMBB1001850//EST//0.0035:204:61//Hs.7311:T23858
F-HEMBB1001863//Small inducible cytokine A5 (RANTES)//3.0e-48:357:82//Hs.155464:AF088219
F-HEMBB1001867//ESTs//2.2e-40:265:88//Hs.146323:AI251752
F-HEMBB1001868//ESTs//5.2e-06:131:73//Hs.123362:AA811371
F-HEMBB1001869//ESTs//1.0e-86:429:96//Hs.141208:AA825503
F-HEMBB1001872
F-HEMBB1001874//H.sapiens mRNA for CHD5 protein//0.0033:388:60//Hs.19923:Y12478
F-HEMBB1001875//H.sapiens mRNA for RNA helicase (Myc-regulated dead box protein)//0.32:346:60//Hs.100555:X98743
F-HEMBB1001880//EST//4.0e-28:171:92//Hs.151194:AI125868
F-HEMBB1001899//ESTs//0.17:242:62//Hs.136969:AA830918
F-HEMBB1001905
F-HEMBB1001906//ESTs//5.6e-49:290:92//Hs.127298:H09155
F-HEMBB1001908//Human monocytic leukaemia zinc finger protein (MOZ) mRNA, complete cds//1.2e-83:672:81//Hs.82210:U47742
F-HEMBB1001910//EST, Weakly similar to albumin [H.sapiens]//0.047:206:62//Hs.159777:Z19955
F-HEMBB1001911
F-HEMBB1001915//ESTs//0.92:136:71//Hs.144465:R68882
F-HEMBB1001921//EST//2.0e-19:398:67//Hs.44789:N36113
F-HEMBB1001922//ESTs//4.3e-05:370:59//Hs.123669:AA805245
F-HEMBB1001925//ESTs//5.7e-27:329:71//Hs.141071:H16398
F-HEMBB1001930//EST//0.043:157:63//Hs.161927:AA483904
F-HEMBB1001944//Human mRNA for KIAA0118 gene, partial cds//5.7e-55:444:80//Hs.154326:D42087
F-HEMBB1001945//ESTs//1.1e-19:142:88//Hs.7341:N57875
F-HEMBB1001947//Human mRNA for KIAA0392 gene, partial cds//1.8e-21:333:66//Hs.40100:AB002390
F-HEMBB1001950//Homo sapiens Notch3 (NOTCH3) mRNA, complete cds//0.020:384:60//Hs.8546:U97669
F-HEMBB1001952//EST//7.0e-13:302:63//Hs.120089:AA708101
F-HEMBB1001953//ATL-derived PMA-responsive (APR) peptide//0.97:252:60//Hs.96:D90070
F-HEMBB1001957//ESTs//6.1e-32:446:67//Hs.51305:T47418
F-HEMBB1001962//Cytochrome P450, subfamily I (aromatic compound-inducible), polypeptide 2//2.3e-31:390:70//Hs.1361:M55053
F-HEMBB1001967//H.sapiens mRNA for urea transporter//9.7e-52:322:88//Hs.66710:X96969
F-HEMBB1001973//Myelin oligodendrocyte glycoprotein {alternative products}//2.1e-48:426:78//Hs.53217:Z48051
F-HEMBB1001983
F-HEMBB1001988//ESTs//6.5e-05:237:63//Hs.49760:AA741051
F-HEMBB1001990//ESTs//0.25:171:64//Hs.7961:AA401205
F-HEMBB1001996//ESTs//1.8e-19:436:65//Hs.125539:AI339103
F-HEMBB1001997//EST//5.3e-33:294:76//Hs.161041:H82636
F-HEMBB1002002//ESTs//1.9e-06:224:67//Hs.110915:AA132964
F-HEMBB1002005//ESTs//5.8e-17:170:78//Hs.141825:AA017093
F-HEMBB1002009//ESTs//0.066:441:58//Hs.125313:AI201685
F-HEMBB1002015//EST//2.3e-18:310:68//Hs.145899:AI274951
F-HEMBB1002042//CYTOCHROME P450 IVB1//2.9e-11:446:62//Hs.687:X16699
F-HEMBB1002043//ESTs, Weakly similar to T06E6.d [C.elegans]//1.0:217:60//Hs.3487:AA425553
F-HEMBB1002044
F-HEMBB1002045
F-HEMBB1002049//Homo sapiens mRNA for KIAA0713 protein, partial cds//0.082:201:61//Hs.88756:AB018256
F-HEMBB1002050//Breakpoint cluster region protein BCR//0.84:267:59//Hs.2557:Y00661
F-HEMBB1002068//Homo sapiens mRNA for KIAA0612 protein, partial cds//8.1e-07:402:61//Hs.112499:AB014512
F-HEMBB1002069
F-HEMBB1002092//EST//5.1e-15:180:75//Hs.127928:AA969239
F-HEMBB1002094//EST//2.0e-52:264:98//Hs.71763:AA146625
F-HEMBB1002115//EST//0.0083:244:64//Hs.125353:AA877080
F-HEMBB1002134//ESTs//1.7e-69:398:91//Hs.157492:AI361027
F-HEMBB1002139//ESTs//0.64:145:71//Hs.157821:AI362013
F-HEMBB1002142//ESTs//0.013:311:59//Hs.150037:AI292214
F-HEMBB1002152//ESTs//8.4e-12:121:82//Hs.119540:T95254
F-HEMBB1002189//EST//0.26:81:70//Hs.147726:AI220208
F-HEMBB1002190//Alcohol dehydrogenase 2 (class I), beta polypeptide//0.16:608:58//Hs.4:X03350
F-HEMBB1002193//Human sky mRNA for Sky, complete cds//6.6e-35:179:100//Hs.301:U18934
F-HEMBB1002217//Homo sapiens mRNA for zinc finger protein 10//3.7e-25:405:67//Hs.104115:X52332
F-HEMBB1002218//EST//0.015:241:61//Hs.105298:AA489813
F-HEMBB1002232//Small inducible cytokine A5 (RANTES)//9.0e-31:365:71//Hs.155464:AF088219
F-HEMBB1002247
F-HEMBB1002249//Homo sapiens haemopoietic progenitor homeobox HPX42B (HPX42B) mRNA, complete cds//6.8e-47:418:77//Hs.125231:AF068006
F-HEMBB1002254//Homo sapiens mRNA for KIAA0594 protein, partial cds//5.0e-47:437:77//Hs.154872:AB011166
F-HEMBB1002255//ESTs//0.017:255:61//Hs.126786:U74314
F-HEMBB1002266//Homo sapiens retinoblastoma-associated protein HEC mRNA, complete cds//0.17:511:57//Hs.58169:AF017790
F-HEMBB1002280//EST//4.0e-35:182:98//Hs.127701:AA864998
F-HEMBB1002300
F-HEMBB1002306//Human G protein-coupled receptor (STRL22) mRNA, complete cds//6.3e-14:228:72//Hs.46468:U45984
F-HEMBB1002327//EST//4.3e-21:242:75//Hs.72377:AA161083
F-HEMBB1002329//ESTs, Weakly similar to C17G10.1 [C.elegans]//1.7e-77:399:96//Hs.105837:AA536054
F-HEMBB1002340//INSULIN-DEGRADING ENZYME//1.0:319:60//Hs.1508:M21188
F-HEMBB1002342//Homo sapiens mRNA for putative thioredoxin-like protein//1.4e-155:724:98//Hs.42644:AJ010841
F-HEMBB1002358//Deoxythymidylate kinase//1.1e-37:192:98//Hs.79006:L16991
F-HEMBB1002359//Human Rev interacting protein Rip-1 mRNA, complete cds//1.7e-06:66:96//Hs.154762:U00943
F-HEMBB1002364//EST//4.7e-16:201:73//Hs.149925:AI288838
F-HEMBB1002371//EST//2.4e-07:319:61//Hs.136459:AA577796
F-HEMBB1002381
F-HEMBB1002383//vasoactive intestinal peptide receptor 2//0.98:190:63//Hs.2126:L36566
F-HEMBB1002387//EST//2.1e-07:253:61//Hs.145993:AI277784
F-HEMBB1002409//ESTs//1.4e-11:94:91//Hs.125958:AI206456
F-HEMBB1002415//Homo sapiens mRNA, chromosome 1 specific transcript KIAA0501//2.0e-32:371:73//Hs.159897:AB007970
F-HEMBB1002425//Fc fragment of IgA, receptor for//2.7e-32:156:82//Hs.54486:X54150
F-HEMBB1002442
F-HEMBB1002453//Human mRNA for KIAA0118 gene, partial cds//5.6e-53:461:77//Hs.154326:D42087
F-HEMBB1002457//ESTs//3.4e-25:184:70//Hs.140225:AA704101
F-HEMBB1002458//ESTs//7.0e-10:343:62//Hs.163816:N76274
F-HEMBB1002477//Human Grb2-associated binder-1 mRNA, complete cds//6.0e-89:493:92//Hs.159605:U43885
F-HEMBB1002489//Homo sapiens 195 kDa cornified envelope precursor mRNA, complete cds//0.019:228:63//Hs.74304:AF001691
F-HEMBB1002492//EST//0.24:149:62//Hs.146790:AI149051
F-HEMBB1002495//Fc fragment of IgE, high affinity I, receptor for; beta polypeptide//1.3e-22:331:71//Hs.30:M89796
F-HEMBB1002502//ESTs//1.3e-41:380:78//Hs.61199:AA024494
F-HEMBB1002509//ESTs//0.017:220:63//Hs.155263:AI273725
F-HEMBB1002510//ESTs//6.4e-102:476:99//Hs.152289:AI247354
F-HEMBB1002520//Human Line-1 repeat mRNA with 2 open reading frames//2.4e-50:580:72//Hs.23094:M19503
F-HEMBB1002522//EST//0.010:172:62//Hs.147224:AI205719
F-HEMBB1002531
F-HEMBB1002534//Small inducible cytokine A5 (RANTES)//3.7e-59:258:88//Hs.155464:AF088219
F-HEMBB1002545//ESTs//3.9e-24:181:86//Hs.13753:AI088102
F-HEMBB1002550//Syntaxin 5A//0.27:354:59//Hs.154546:U26648
F-HEMBB1002556//ESTs//1.7e-33:286:79//Hs.146173:AA906191
F-HEMBB1002579//EST//1.0:77:68//Hs.147935:AI250286
F-HEMBB1002582//ESTs//0.00032:178:68//Hs.139163:AA226095
F-HEMBB1002590//ESTs//0.64:132:63//Hs.155688:AI003657
F-HEMBB1002596//ESTs//3.4e-19:462:64//Hs.124399:AA832336
F-HEMBB1002600//Homo sapiens tetraspan NET-5 mRNA, complete cds//3.0e-152:710:98//Hs.129826:AF089749
F-HEMBB1002601//EST//9.6e-13:368:62//Hs.137080:AA894817
F-HEMBB1002603//EST//0.10:144:63//Hs.158180:AI367945
F-HEMBB1002607//ESTs//0.024:345:62//Hs.143304:AI084058
F-HEMBB1002610//EST//2.1e-14:291:64//Hs.140573:AA826323
F-HEMBB1002613//ESTs//1.9e-17:192:75//Hs.141161:AA210711
F-HEMBB1002614//ESTs//0.0048:136:71//Hs.106280:R13901
F-HEMBB1002617//EST//0.034:320:59//Hs.41223:H89127
F-HEMBB1002623//ESTs//0.88:222:60//Hs.129920:AA167217
F-HEMBB1002635//Human MAP kinase mRNA, complete cds//3.1e-23:127:100//Hs.151051:U07620
F-HEMBB1002664//EST//0.00013:203:61//Hs.117141:AA678811
F-HEMBB1002677//ESTs//2.4e-22:439:66//Hs.132046:AA693680
F-HEMBB1002683//ESTs//0.23:224:61//Hs.128883:AI026679
F-HEMBB1002684//ESTs//7.2e-09:82:87//Hs.140457:H05124
F-HEMBB1002686//EST//0.25:189:62//Hs.132431:AA909674
F-HEMBB1002692//ESTs//0.00020:162:66//Hs.118180:N68504
F-HEMBB1002697//EST//7.2e-17:219:74//Hs.100459:T61992
F-HEMBB1002699//Homo sapiens transmembrane activator and CAML interactor (TACI) mRNA, complete cds//0.059:297:62//Hs.158341:AF023614
F-HEMBB1002702//ESTs//0.26:284:61//Hs.41250:H89588
F-HEMBB1002705//ESTs, Weakly similar to HYPOTHETICAL 38.5 KD PROTEIN IN SUI2-TDH2 INTERGENIC REGION [Saccharomyces cerevisiae]//0.0048:84:83//Hs.20814:AI242922
F-HEMBB1002712//ESTs//0.0025:317:58//Hs.7344:AA972729
F-MAMMA1000009//Human c-yes-1mRNA//1.0e-48:447:77//Hs.75680:M15990
F-MAMMA1000019
F-MAMMA1000020//EST//2.6e-84:431:95//Hs.143333:H51750
F-MAMMA1000025//EST//1.0:169:59//Hs.130165:AA906945
F-MAMMA1000043//Human NSCL-1 mRNA sequence//0.94:262:60//Hs.30956:M96739
F-MAMMA1000045//ESTs//1.7e-48:499:75//Hs.158469:AA897461
F-MAMMA1000055//ESTs, Highly similar to TESTIN 2 PRECURSOR [Mus musculus]//2.7e-18:330:63//Hs.59906:AA001281
F-MAMMA1000057//Homo sapiens DNA fragmentation factor 40 kDa subunit (DFF40) mRNA, complete cds//1.2e-50:367:75//Hs.133089:AF064019
F-MAMMA1000069//ESTs//0.58:286:60//Hs.134417:AI336840
F-MAMMA1000084//Human mRNA for KIAA0033 gene, partial cds//1.1e-48:641:70//Hs.22271:D26067
F-MAMMA1000085//Homo sapiens mRNA for KIAA0602 protein, partial cds//0.00013:199:69//Hs.37656:AB011174
F-MAMMA1000092//Homo sapiens telomeric repeat binding factor (TRF1) mRNA, complete cds//1.2e-52:346:77//Hs.90357:U40705
F-MAMMA1000103//Homo sapiens mRNA for extracellular matrix protein, complete cds//1.0:151:64//Hs.35094:AB011792
F-MAMMA1000117
F-MAMMA1000129//RYANODINE RECEPTOR, SKELETAL MUSCLE//0.0015:492:60//Hs.89631:U48508
F-MAMMA1000133//ESTs//1.0:125:67//Hs.118309:AA653402
F-MAMMA1000134//EST//1.2e-08:75:92//Hs.160674:AI248319
F-MAMMA1000139//EST//5.5e-10:139:76//Hs.159121:AI383843
F-MAMMA1000143//Homo sapiens mRNA for KIAA0685 protein, complete cds//2.2e-26:148:97//Hs.153121:AB014585
F-MAMMA1000155//Homo sapiens homeobox transcription factor barx2 (BARX2) mRNA, complete cds//3.3e-31:219:87//Hs.129724:AF031924
F-MAMMA1000163//ESTs//1.2e-59:317:94//Hs.49559:AA401050
F-MAMMA1000171//ESTs//1.7e-09:161:69//Hs.119070:AA629695
F-MAMMA1000173//Human drebrin E2 mRNA (DBN1), complete cds//9.2e-40:686:65//Hs.89434:D17530
F-MAMMA1000175//ESTs//0.65:141:68//Hs.133152:H91657
F-MAMMA1000183//Human mRNA for KIAA0065 gene, partial cds//1.0e-92:904:72//Hs.70617:D31763
F-MAMMA1000198//ESTs//0.0092:235:62//Hs.98783:AI091739
F-MAMMA1000221//EST//3.3e-16:95:98//Hs.128271:AA973035
F-MAMMA1000227//ESTs//0.010:268:60//Hs.16412:AA506926
F-MAMMA1000241//ESTs//0.13:140:67//Hs.12328:AI377913
F-MAMMA1000251//EST//3.7e-07:118:73//Hs.153116:AA856873
F-MAMMA1000254//ESTs//0.00023:245:59//Hs.150513:AI247587
F-MAMMA1000257//EST//4.2e-10:155:74//Hs.150409:AI003543
F-MAMMA1000264//ESTs//2.0e-18:217:75//Hs.152748:N53015
F-MAMMA1000266//EST//0.14:270:60//Hs.132593:AI031874
F-MAMMA1000270//Human mRNA for KIAA0118 gene, partial cds//2.5e-54:354:87//Hs.154326:D42087
F-MAMMA1000277//Hydroxysteroid (11-beta) dehydrogenase 2//1.0e-07:306:65//Hs.1376:U26726
F-MAMMA1000278//ESTs//4.0e-09:197:67//Hs.157034:AI347361
F-MAMMA1000279//Complement component 5 receptor 1 (C5a ligand)//8.4e-34:341:68//Hs.2161:M62505
F-MAMMA1000284
F-MAMMA1000287//Human mRNA for KIAA0118 gene, partial cds//5.4e-50:245:84//Hs.154326:D42087
F-MAMMA1000302//EST//5.3e-40:213:98//Hs.122363:AA788641
F-MAMMA1000307//Polycystic kidney disease 1 (autosomal dominant)//0.55:510:57//Hs.75813:L33243
F-MAMMA1000309//Apolipoprotein E//9.7e-06:691:58//Hs.76260:M12529
F-MAMMA1000312//EST//0.042:183:63//Hs.158928:AI379519
F-MAMMA1000313
F-MAMMA1000331
F-MAMMA1000339
F-MAMMA1000340//ESTs, Highly similar to HYPOTHETICAL 29.4 KD PROTEIN IN STE6-LOS1 INTERGENIC REGION [Saccharomyces cerevisiae]//2.9e-11:87:93//Hs.13096:AA180963
F-MAMMA1000348//Homo sapiens KIAA0432 mRNA, complete cds//3.6e-23:270:72//Hs.155174:AB007892
F-MAMMA1000356//Homo sapiens mRNA, chromosome 1 specific transcript KIAA0484//3.7e-24:233:72//Hs.158095:AB007953
F-MAMMA1000360//Human Line-1 repeat mRNA with 2 open reading frames//9.0e-75:498:85//Hs.23094:M19503
F-MAMMA1000361//Human mRNA for KIAA0118 gene, partial cds//9.1e-50:304:85//Hs.154326:D42087
F-MAMMA1000372//EST//1.2e-53:376:86//Hs.144295:AA136569
F-MAMMA1000385//ESTs//1.4e-22:220:76//Hs.142552:AA235344
F-MAMMA1000388//Homo sapiens UKLF mRNA for ubiquitous Kruppel like factor, complete cds//1.2e-149:710:98//Hs.32170:AB015132
F-MAMMA1000395//Acyl-Coenzyme A dehydrogenase, very long chain//0.74:330:60//Hs.82208:L46590
F-MAMMA1000402//Human Line-1 repeat mRNA with 2 open reading frames//2.4e-58:834:68//Hs.23094:M19503
F-MAMMA1000410//Human NADH:ubiquinone oxidoreductase subunit B13 (B13) mRNA, complete cds//1.2e-08:117:84//Hs.83916:U53468
F-MAMMA1000413//ESTs//3.3e-31:209:88//Hs.146154:AI200725
F-MAMMA1000414//ESTs//0.82:132:62//Hs.124857:AA687092
F-MAMMA1000416//ESTs, Weakly similar to HYPOTHETICAL 32.0 KD PROTEIN C09F5.2 IN CHROMOSOME III [C.elegans]//9.8e-33:267:81//Hs.32370:AA521111
F-MAMMA1000421//ESTs//7.3e-33:320:75//Hs.121659:H02532
F-MAMMA1000422//Homo sapiens protocadherin (PCDH8) mRNA, complete cds//0.98:553:56//Hs.19492:AF061573
F-MAMMA1000423//EST//0.0075:179:63//Hs.162974:AA678459
F-MAMMA1000424//ESTs//1.3e-17:313:67//Hs.139858:AI377641
F-MAMMA1000429//Homo sapiens sorting nexin 3 (SNX3) mRNA, complete cds//5.1e-48:491:72//Hs.12102:AF034546
F-MAMMA1000431//ISLET AMYLOID POLYPEPTIDE PRECURSOR//5.1e-39:320:81//Hs.51048:X68830
F-MAMMA1000444//Homo sapiens mRNA for KIAA0594 protein, partial cds//9.1e-39:342:78//Hs.154872:AB011166
F-MAMMA1000446
F-MAMMA1000458//ESTs, Weakly similar to similar to CCAAT/enhancer-binding protein [C.elegans]//5.1e-08:58:93//Hs.9043:W21827
F-MAMMA1000468//Homo sapiens mRNA for 6-phosphofructo-2-kinase/fructose-2,6-bisphosphatase, complete cds//0.58:311:63//Hs.66721:D49818
F-MAMMA1000472//ISLET AMYLOID POLYPEPTIDE PRECURSOR//2.1e-44:346:80//Hs.51048:X68830
F-MAMMA1000478//Homo sapiens PYRIN (MEFV) mRNA, complete cds//0.0017:157:73//Hs.113283:AF018080
F-MAMMA1000483//ISLET AMYLOID POLYPEPTIDE PRECURSOR//4.5e-39:400:75//Hs.51048:X68830
F-MAMMA1000490//ESTs//3.6e-52:331:88//Hs.163686:AA291948
F-MAMMA1000500//EST//9.7e-73:346:99//Hs.98812:AA434482
F-MAMMA1000501//Small inducible cytokine A5 (RANTES)//2.3e-50:325:86//Hs.155464:AF088219
F-MAMMA1000516//Oxytocin receptor//1.6e-29:660:64//Hs.2820:X64878
F-MAMMA1000522//ESTs//2.9e-23:328:70//Hs.125142:AA421352
F-MAMMA1000524//ESTs//1.1e-08:211:65//Hs.33467:R85497
F-MAMMA1000559//EST//4.7e-17:207:71//Hs.162733:AA614352
F-MAMMA1000565
F-MAMMA1000567//Homo sapiens haemopoietic progenitor homeobox HPX42B (HPX42B) mRNA, complete cds//5.8e-51:404:80//Hs.125231:AF068006
F-MAMMA1000576//ESTs//3.8e-32:236:74//Hs.140039:AA047045
F-MAMMA1000583//ESTs//0.00099:123:70//Hs.135173:AI276780
F-MAMMA1000585//Homo sapiens class-I MHC-restricted T cell associated molecule (CRTAM) mRNA, complete cds//8.8e-45:390:78//Hs.159523:AF001622
F-MAMMA1000594//ESTs//8.3e-42:322:81//Hs.161660:AA167744
F-MAMMA1000597//Homo sapiens KIAA0426 mRNA, complete cds//2.6e-37:592:68//Hs.97476:AB007886
F-MAMMA1000605//Homo sapiens 4F5S mRNA, complete cds//5.1e-26:228:73//Hs.32567:AF073519
F-MAMMA1000612//Homo sapiens Gx protein (GX) mRNA, complete cds//0.00091:300:60//Hs.29207:AF071494
F-MAMMA1000616//ESTs//0.41:373:59//Hs.130699:AA621478
F-MAMMA1000621//EST//0.027:146:62//Hs.148305:AA909605
F-MAMMA1000623
F-MAMMA1000625//Homo sapiens ES/130 mRNA, complete cds//0.89:428:56//Hs.98614:AF006751
F-MAMMA1000643//Homo sapiens nephrocystin (NPHP1) mRNA, partial cds//0.092:365:59//Hs.75474:AF023674
F-MAMMA1000664//ESTs//7.6e-07:259:64//Hs.140622:AA844353
F-MAMMA1000669//Human kpni repeat mrna (cdna clone pcd-kpni-4),3' end//9.0e-30:531:64//Hs.139107:K00629
F-MAMMA1000670//ESTs//6.6e-83:389:100//Hs.148595:AI244490
F-MAMMA1000672//Homo sapiens CAGH32 mRNA, partial cds//0.17:109:73//Hs.4316:U80743
F-MAMMA1000684//Homo sapiens forkhead protein FREAC-2 mRNA, complete cds//3.3e-07:249:62//Hs.44481:U13220
F-MAMMA1000696//Interleukin 10//5.6e-47:355:82//Hs.2180:M57627
F-MAMMA1000707//ESTs//1.4e-09:225:65//Hs.138722:N51081
F-MAMMA1000713//Acetylcholinesterase {I4-E5 doman} [human, tumor cell lines, Genomic, 847 nt]//0.16:84:72//Hs.157124:S71129
F-MAMMA1000714//Human clone 23947 mRNA, partial cds//0.97:263:6//Hs.27414:U79275
F-MAMMA1000718//ESTs, Weakly similar to putative p150 [H.sapiens]//5.0e-07:210:66//Hs.71148:AA854648
F-MAMMA1000720//ESTs//1.4e-50:301:83//Hs.138852:AA284247
F-MAMMA1000723//ESTs, Weakly similar to ORF2-like protein [H.sapiens]//8.1e-22:288:72//Hs.114685:AA700024
F-MAMMA1000731//Homo sapiens CHD1 mRNA, complete cds//1.5e-23:292:66//Hs.22670:AF006513
F-MAMMA1000732//Homo sapiens mRNA, chromosome 1 specific transcript KIAA0484//4.8e-40:288:78//Hs.158095:AB007953
F-MAMMA1000733//RAS GTPASE-ACTIVATING-LIKE PROTEIN IQGAP1//0.25:467:58//Hs.1742:L33075
F-MAMMA1000734//Homo sapiens SEC63 (SEC63) mRNA, complete cds//2.3e-169:802:98//Hs.31575:AF100141
F-MAMMA1000738//EST//1.0:149:63//Hs.136928:AA812580
F-MAMMA1000744//Homo sapiens mRNA for KIAA0575 protein, complete cds//3.3e-51:323:88//Hs.153468:AB011147
F-MAMMA1000746//ESTs//2.3e-42:409:76//Hs.61199:AA024494
F-MAMMA1000752//EST, Weakly similar to putative p150 [H.sapiens]//1.1e-14:285:68//Hs.162011:AA513663
F-MAMMA1000760//Myelin oligodendrocyte glycoprotein {alternative products}//6.2e-47:341:82//Hs.53217:Z48051
F-MAMMA1000761//ESTs, Moderately similar to !!!! ALU SUBFAMILY SX WARNING ENTRY !!!! [H.sapiens]//9.8e-19:131:76//Hs.118972:AA761369
F-MAMMA1000775//EST//6.9e-32:424:69//Hs.44554:N34288
F-MAMMA1000776//ESTs//5.5e-43:154:84//Hs.141581:AA315361
F-MAMMA1000778//EST//4.4e-28:226:80//Hs.128952:AA984114
F-MAMMA1000782//ESTs//0.35:270:60//Hs.29153:AA551137
F-MAMMA1000798//Homo sapiens clone 24407 mRNA sequence//1.6e-23:531:65//Hs.12432:AF070575
F-MAMMA1000802//ESTs//3.1e-67:340:97//Hs.126081:AA459849
F-MAMMA1000824//ESTs//0.98:44:90//Hs.42802:N20130
F-MAMMA1000831//ESTs//0.0081:194:60//Hs.150400:AI298089
F-MAMMA1000839//Small inducible cytokine A5 (RANTES)//4.7e48:241:74//Hs.155464:AF088219
F-MAMMA1000841
F-MAMMA1000842//Human monocytic leukaemia zinc finger protein (MOZ) mRNA, complete cds//0.18:483:59//Hs.82210:U47742
F-MAMMA1000843//EST//0.34:113:68//Hs.58415:W74696
F-MAMMA1000845//EST//2.9e-06:56:80//Hs.123243:AA804877
F-MAMMA1000851//EST//0.78:103:65//Hs.135656:AA907022
F-MAMMA1000855
F-MAMMA1000856//Homo sapiens preprocathepsin P mRNA, partial cds//0.14:320:59//Hs.71388:AF032906
F-MAMMA1000859//SOX-3 PROTEIN//0.014:474:57//Hs.157429:X71135
F-MAMMA1000862//EST//1.0:92:66//Hs.157599:AI357342
F-MAMMA1000863//ELK1, member of ETS oncogene family//1.2e-30:214:75//Hs.116549:AL009172
F-MAMMA1000865//ESTs//0.99:127:66//Hs.125230:AA873812
F-MAMMA1000867//EST//0.027:236:60//Hs.147156:AI191777
F-MAMMA1000875//Human mRNA for KIAA0269 gene, complete cds//0.96:245:59//Hs.75850:D87459
F-MAMMA1000876//ESTs//1.5e-39:192:90//Hs.132020:AA704147
F-MAMMA1000877//ESTs, Weakly similar to !!!! ALU SUBFAMILY J WARNING ENTRY !!!! [H.sapiens]//1.4e-91:484:94//Hs.138938:AA012894
F-MAMMA1000880//EST//0.014:142:66//Hs.137044:AA878812
F-MAMMA1000883//EST//1.0:166:62//Hs.126352:AA894465
F-MAMMA1000897//H.sapiens mRNA for inter-alpha-trypsin inhibitor heavy chain H3//2.6e-06:211:63//Hs.76716:X67055
F-MAMMA1000905//Cartilage matrix protein//0.97:190:64//Hs.150366:M55683
F-MAMMA1000906//ESTs//3.0e-07:145:72//Hs.133556:AA702506
F-MAMMA1000908//ESTs//1.1e-70:484:84//Hs.142497:AA189081
F-MAMMA1000914//Angiopoietin 1//0.14:450:59//Hs.2463:D13628
F-MAMMA1000921//ESTs//6.8e-96:448:99//Hs.135721:AI125239
F-MAMMA1000931//CD4 receptor {exons 1 and 2} [human, T-lymphocyte, mRNA, 3429 nt]//1.0e-25:312:66//Hs.116007:S79267
F-MAMMA1000940//EST//2.9e-42:209:76//Hs.140567:AA825968
F-MAMMA1000941//Dihydrolipoamide branched chain transacylase (E2 component of branched chain keto acid dehydrogenase complex)//1.8e-38:395:71//Hs.89479:X66785
F-MAMMA1000942//ESTs//1.9e-19:252:71//Hs.141575:AA211734
F-MAMMA1000943//Human mRNA for KIAA0305 gene, complete cds//0.077:236:63//Hs.83790:AB002303
F-MAMMA1000956//Homo sapiens hRVP1 mRNA for RVP1, complete cds//8.8e-33:566:64//Hs.25640:AB000714
F-MAMMA1000957//ESTs//1.0:177:59//Hs.149864:N80474
F-MAMMA1000962//Homo sapiens neuronal thread protein AD7c-NTP mRNA, complete cds//1.1e-56:310:85//Hs.129735:AF010144
F-MAMMA1000968//ESTs//9.2e-18:128:89//Hs.163980:AA715814
F-MAMMA1000975//ESTs//3.8e-08:219:66//Hs.110937:AA137096
F-MAMMA1000979//EST//0.00022:155:65//Hs.101379:Z39802
F-MAMMA1000987//EST//1.1e-48:373:81//Hs.139034:W27062
F-MAMMA1000998//EST//2.0e-07:356:62//Hs.132467:AA922007
F-MAMMA1001003//ESTs//0.47:129:67//Hs.164016:AI003724
F-MAMMA1001008//ESTs//1.9e-17:153:82//Hs.141161:AA210711
F-MAMMA1001021//Homo sapiens beta-dystrobrevin (BDTN) mRNA, complete cds//4.7e-17:100:100//Hs.13451:Y15718
F-MAMMA1001024//ESTs//0.97:251:62//Hs.59389:R93968
F-MAMMA1001030//Homo sapiens orphan G protein-coupled receptor HG38 mRNA, complete cds//3.6e-32:753:61//Hs.98384:AF062006
F-MAMMA1001035//ESTs//6.9e-28:268:77//Hs.139536:AA180857
F-MAMMA1001038
F-MAMMA1001041//ALPHA-ACTININ 1, CYTOSKELETAL ISOFORM//2.7e-10:357:65//Hs.119000:M95178
F-MAMMA1001050//EST//1.8e-29:321:74//Hs.161240:AI419882
F-MAMMA1001059//ESTs, Weakly similar to protein synthesis initiation factor 4A-II homolog//7.9e-87:415:99//Hs.135623:AA134719
F-MAMMA1001067//EST//0.30:166:60//Hs.148441:AI198503
F-MAMMA1001073//ESTs//1.0e-98:476:98//Hs.98321:AA455585
F-MAMMA1001074//ESTs//1.6e-82:396:98//Hs.118923:AA252116
F-MAMMA1001075//Homo sapiens (clone F4) transmembrane protein mRNA sequence//3.7e-29:559:65//Hs.135251:L09749
F-MAMMA1001078//Human Line-1 repeat mRNA with 2 open reading frames//2.7e-99:689:83//Hs.23094:M19503
F-MAMMA1001080//IG ALPHA-2 CHAIN C REGION//5.8e-43:319:81//Hs.32225:AF067420
F-MAMMA1001082//ESTs//6.2e-28:275:77//Hs.152685:AA613896
F-MAMMA1001091//Homo sapiens mRNA for KIAA0711 protein, complete cds//0.0081:586:57//Hs.5333:AB018254
F-MAMMA1001092//Human kpni repeat mrna (cdna clone pcd-kpni-8), 3' end//5.1e-24:328:72//Hs.103948:K00627
F-MAMMA1001105//Homo sapiens OVO-like 1 binding protein (OVOL1) mRNA, complete cds//2.1e-24:507:66//Hs.97905:AF016045
F-MAMMA1001110//Human mRNA for KIAA0125 gene, complete cds//0.94:448:57//Hs.38365:D50915
F-MAMMA1001126//Small inducible cytokine A5 (RANTES)//4.6e-18:123:85//Hs.155464:AF088219
F-MAMMA1001133
F-MAMMA1001139
F-MAMMA1001143//ESTs//2.6e-18:121:82//Hs.135117:AI091534
F-MAMMA1001145//ESTs//1.5e-36:442:69//Hs.124712:H90217
F-MAMMA1001154//EST//0.054:208:61//Hs.162088:AA505741
F-MAMMA1001161//Homo sapiens mRNA for KIAA0575 protein, complete cds//6.6e-38:337:77//Hs.153468:AB011147
F-MAMMA1001162//EST//4.7e-16:117:90//Hs.130894:AI014299
F-MAMMA1001181
F-MAMMA1001186//Human macrophage-derived chemokine precursor (MDC) mRNA, complete cds//6.5e-47:313:81//Hs.97203:U83171
F-MAMMA1001191//ESTs//5.8e-34:197:94//Hs.121575:AA758083
F-MAMMA1001198
F-MAMMA1001202//ESTs//1.5e-37:210:83//Hs.79788:AA527348
F-MAMMA1001203//ESTs//1.2e-29:199:76//Hs.141605:H92974
F-MAMMA1001206//ESTs, Weakly similar to !!!! ALU SUBFAMILY J WARNING ENTRY !!!! [H.sapiens]//5.5e-25:275:75//Hs.105292:AA504776
F-MAMMA1001215//ESTs//1.9e-06:300:63//Hs.113566:T03200
F-MAMMA1001220//Human mRNA for KIAA0118 gene, partial cds//2.7e-53:367:84//Hs.154326:D42087
F-MAMMA1001222//Homo sapiens mRNA for KIAA0634 protein, partial cds//1.8e-05:435:59//Hs.30898:AB014534
F-MAMMA1001243//ESTs//5.2e-19:118:94//Hs.122830:AA765587
F-MAMMA1001244
F-MAMMA1001249//ESTs//1.3e-89:420:99//Hs.147744:AI220476
F-MAMMA1001256//ESTs//2.1e-34:282:80//Hs.46158:AI160121
F-MAMMA1001259//ESTs//2.9e-07:68:95//Hs.6193:AA045149
F-MAMMA1001260//Homo sapiens mRNA for KIAA0661 protein, complete cds//2.8e-41:659:64//Hs.65238:AB014561
F-MAMMA1001268//Human Line-1 repeat mRNA with 2 open reading frames//1.7e-33:336:74//Hs.23094:M19503
F-MAMMA1001271//Homo sapiens CAGH3 mRNA, complete cds//3.4e-06:487:59//Hs.21858:U80747
F-MAMMA1001274//Human mRNA for KIAA0080 gene, partial cds//5.1e-62:396:76//Hs.74554:D38522
F-MAMMA1001280//ESTs//7.3e-14:273:67//Hs.126503:AA913832
F-MAMMA1001292//Human mRNA for KIAA0176 gene, partial cds//5.6e-54:616:71//Hs.4935:D79998
F-MAMMA1001296//ESTs//4.8e-34:136:85//Hs.70279:AA757426
F-MAMMA1001298//ESTs//0.021:73:80//Hs.114233:N91305
F-MAMMA1001305//Human DNA sequence from PAC 127B20 on chromosome 22q11.2-qter, contains gene for GTPase-activating protein similar to rhoGAP protein. ribosomal protein L6 pseudogene, ESTs and CA repeat//1.9e-58:295:97//Hs.102336:Z83838
F-MAMMA1001322//ESTs//9.4e-18:221:74//Hs.139132:AA211087
F-MAMMA1001324//Human endogenous retrovirus pHE.1 (ERV9)//6.7e-75:745:73//Hs.93174:X57147
F-MAMMA1001330//ESTs//2.6e-26:169:91//Hs.4209:AA205806
F-MAMMA1001341//ESTs//0.10:267:62//Hs.155922:AI147197
F-MAMMA1001343//ESTs//0.0024:323:62//Hs.119238:AA476267
F-MAMMA1001346//Homo sapiens mRNA for KIAA0715 protein, partial cds//0.94:89:75//Hs.109358:AB018258
F-MAMMA1001383//Putative mismatch repair/binding protein hMSH3//7.3e-49:273:80//Hs.42674:U61981
F-MAMMA1001388//INSULIN-LIKE GROWTH FACTOR BINDING PROTEIN COMPLEX ACID LABILE CHAIN PRECURSOR//4.6e-09:415:58//Hs.839:M86826
F-MAMMA1001397//Prostaglandin I2 (prostacyclin) synthase //1.3e-26:358:67//Hs.61333:D83402
F-MAMMA1001408//ESTs//7.2e-06:123:72//Hs.26753:R60763
F-MAMMA1001411//Autosomal dominant polycystic kidney disease type II//1.0:176:64//Hs.82001:U50928
F-MAMMA1001419//Homo sapiens KIAA0395 mRNA, partial cds//4.1e-45:409:80//Hs.43681:AL022394
F-MAMMA1001420//Homo sapiens mRNA for alpha(1,2)fucosyltransferase, complete cds//0.00042:125:75//Hs.46328:D87942
F-MAMMA1001435//Human HsLIM15 mRNA for HsLim15, complete cds//8.2e-43:543:71//Hs.37181:D64108
F-MAMMA1001442//ESTs//7.9e-15:103:92//Hs.25780:R51321
F-MAMMA1001446//ESTs//3.5e-44:292:73//Hs.111583:AA463590
F-MAMMA1001452//ESTs//0.73:152:65//Hs.163766:AI424040
F-MAMMA1001465//ESTs//1.0e-15:201:75//Hs.8836:AA181053
F-MAMMA1001476//Human mRNA for 5'-terminal region of UMK, complete cds//2.0e-24:273:72//Hs.75939:D78335
F-MAMMA1001487//ESTs, Weakly similar to ORF2-like protein [H.sapiens]//3.2e-25:397:68//Hs.116874:AA524909
F-MAMMA1001501//CALPAIN 1, LARGE//3.1e-53:438:81//Hs.2575:X04366
F-MAMMA1001502//Human p120E4F transcription factor mRNA, complete cds//0.99:258:61//Hs.154196:U87269
F-MAMMA1001510//ESTs//8.7e-09:380:61//Hs.118701:AA420795
F-MAMMA1001522//ESTs//7.1e-44:321:80//Hs.120170:AI018506
F-MAMMA1001547
F-MAMMA1001551//Homo sapiens mRNA for KIAA0462 protein, partial cds//7.5e-130:614:98//Hs.129937:AB007931
F-MAMMA1001575//ESTs, Weakly similar to zinc finger protein C2H2-171 [H.sapiens]//0.71:181:62//Hs.118866:AI017072
F-MAMMA1001576//Tubulin, gamma polypeptide//5.7e-97:529:91//Hs.150785:M61764
F-MAMMA1001590//EST//1.7e-13:94:92//Hs.95900:AA160339
F-MAMMA1001600//EST/1.0e-08:81:87//Hs.149220:AI247132
F-MAMMA1001604//EST//0.0070:157:62//Hs.162516:AA583375
F-MAMMA1001606//Human clone 23627 mRNA, complete cds//0.64:336:58//Hs.23642:U79266
F-MAMMA1001620//ESTs//6.8e-16:99:79//Hs.164052:AA836152
F-MAMMA1001627//Pregnancy-associated plasma protein A//0.27:379:58//Hs.158229:U28727
F-MAMMA1001630//Human DNA sequence from clone 71L16 on chromosome Xp11. Contains a probable Zinc Finger protein (pseudo)gene, an unknown putative gene, a pseudogene with high similarity to part of antigen KI-67, a putative Chondroitin 6-Sulfotransferase LIKE gene and a KIAA0267 LIKE putative Na(+)/H(+) exchanger protein gene. Contains a predicted CpG island, ESTs, STSs and GSSs and genomic markers DXS1003 and DXS1055//1.4e-40:447:73//Hs.154353:AL022165
F-MAMMA1001633//Human zinc finger protein (LD5-1) mRNA, complete cds//3.6e-44:611:67//Hs.57679:U57796
F-MAMMA1001635
F-MAMMA1001649//ESTs//1.4e-47:238:99//Hs.124063:T75524
F-MAMMA1001654//Homo sapiens retinal rod Na-Ca+K exchanger (NCKX1) mRNA, complete cds//0.00069:140:68//Hs.59829:AB014602
F-MAMMA1001663//Homo sapiens mRNA for KIAA0448 protein, complete cds//0.015:135:71//Hs.27349:AB007917
F-MAMMA1001670//ESTs, Highly similar to 52 KD RO PROTEIN [Homo sapiens]//0.064:472:60//Hs.110819:AI027548
F-MAMMA1001671
F-MAMMA1001679//ESTs//0.94:55:83//Hs.152506:AA573317
F-MAMMA1001683//ESTs//1.6e-92:480:96//Hs.118496:AA036889
F-MAMMA1001686//ESTs//0.00019:171:66//Hs.140402:AI138765
F-MAMMA1001692//ESTs//0.97:104:70//Hs.27596:AI188549
F-MAMMA1001711//Human G protein-coupled receptor (STRL22) mRNA, complete cds//8.0e-45:323:83//Hs.46468:U45984
F-MAMMA1001715//ESTs//1.3e-14:188:72//Hs.130815:AA936548
F-MAMMA1001730//ESTs//0.048:198:65//Hs.116412:AA506926
F-MAMMA1001735//Human beta-tubulin class III isotype (beta-3) mRNA, complete cds//1.5e-111:725:84//Hs.159154:U47634
F-MAMMA1001740//EST//0.77:119:65//Hs.148140:AA887098
F-MAMMA1001743//ESTs//6.5e-27:195:72//Hs.163688:H48768
F-MAMMA1001744//EST//0.00019:134:70//Hs.146863:AI161245
F-MAMMA1001745//Human Line-1 repeat mRNA with 2 open reading frames//4.7e-67:822:69//Hs.23094:M19503
F-MAMMA1001751//Homo sapiens two P domain potassium channel subunit (HOHO1) mRNA, complete cds//1.0e-36:583:65//Hs.79351:U33632
F-MAMMA1001754//ESTs//5.1e-97:456:99//Hs.157928:AA775822
F-MAMMA1001757//EST//0.042:177:63//Hs.144436:R07109
F-MAMMA1001760//Homo sapiens RET finger protein-like 1 antisense transcript, partial//6.6e-41:309:84//Hs.102576:AJ010230
F-MAMMA1001764//ESTs//0.057:290:60//Hs.68647:AA524072
F-MAMMA1001768//Human transcription factor, forkhead related activator 4 (FREAC-4) mRNA, complete cds//2.2e-05:504:60//Hs.96028:AF042832
F-MAMMA1001769//Homo sapiens PYRIN (MEFV) mRNA, complete cds//1.1e-85:686:79//Hs.113283:AF018080
F-MAMMA1001771//Human semaphorin III family homolog mRNA, complete cds//0.00071:392:60//Hs.32981:U38276
F-MAMMA1001783//ESTs//8.8e-23:206:79//Hs.142524:H02940
F-MAMMA1001785//ESTs//1.3e-52:270:97//Hs.61809:AA503549
F-MAMMA1001788//Human kpni repeat mrna (cdna clone pcd-kpni-8), 3' end//6.7e-21:212:77//Hs.103948:K00627
F-MAMMA1001790//Homo sapiens KIAA0409 mRNA, partial cds//2.2e-06:139:72//Hs.5158:AB007869
F-MAMMA1001806//ESTs//6.4e-44:373:79//Hs.105665:H78987
F-MAMMA1001812//ESTs//4.8e-83:407:97//Hs.98613:D83884
F-MAMMA1001815//EST//2.1e-56:374:85//Hs.141488:N47096
F-MAMMA1001817//EST//8.6e-39:336:78//Hs.162236:AA551582
F-MAMMA1001818//EST//0.32:375:58//Hs.72729:AA167589
F-MAMMA1001820//Homo sapiens cytokine-like factor-1 precursor (CLF-1) mRNA, complete cds//0.082:153:66//Hs.114948:AF059293
F-MAMMA1001824//EST//0.0013:195:63//Hs.129275:AA992742
F-MAMMA1001836//ESTs//7.4e-52:283:95//Hs.92290:R78691
F-MAMMA1001837/Homo sapiens mRNA for zinc finger protein FPM315, complete cds//2.0e-29:641:62//Hs.56808:D88827
F-MAMMA1001848//ESTs//3.5e-53:264:99//Hs.116430:AA644665
F-MAMMA1001851//ESTs//0.00050:251:64//Hs.163776:AI393028
F-MAMMA1001854
F-MAMMA1001858//EST//1.0:113:68//Hs.132482:AA922218
F-MAMMA1001864//EST//1.3e-06:399:60//Hs.161500:N68060
F-MAMMA1001868//Homo sapiens nuclear receptor co-repressor N-CoR mRNA, complete cds//0.084:672:58//Hs.152455:AF044209
F-MAMMA1001874//ESTs//0.97:292:58//Hs.24553:AI150687
F-MAMMA1001878
F-MAMMA1001880//ESTs//9.2e-09:277:62//Hs.15776:T91944
F-MAMMA1001890//EST//1.7e-85:440:97//Hs.128842:AA977576
F-MAMMA1001907//EST//2.7e-26:294:74//Hs.98794:AA434078
F-MAMMA1001908//ESTs//3.2e-109:505:100//Hs.146145:AI391521
F-MAMMA1001931//ESTs//1.0:108:67//Hs.126624:AA768874
F-MAMMA1001956//Apolipoprotein E//1.0:322:59//Hs.76260:M12529
F-MAMMA1001963//ESTs//0.84:320:60//Hs.6523:AA218859
F-MAMMA1001969//Homo sapiens clone 23892 mRNA sequence//3.6e-79:423:81//Hs.91916:AF035317
F-MAMMA1001970//Oxytocin receptor//9.7e-31:626:64//Hs.2820:X64878
F-MAMMA1001992//EST, Weakly similar to reverse transcriptase [H.sapiens]//7.9e-09:150:72//Hs.118222:N91115
F-MAMMA1002009//ESTs//2.2e-18:234:69//Hs.21978:AA009633
F-MAMMA1002011//ESTs//0.91:276:59//Hs.141196:AA704826
F-MAMMA1002032//ESTs//7.8e-40:344:77//Hs.141658:N77915
F-MAMMA1002033//ESTs//2.5e-30:293:76//Hs.139158:AA226159
F-MAMMA1002041//Homo sapiens mRNA, chromosome 1 specific transcript KIAA0484//1.2e-54:455:70//Hs.158095:AB007953
F-MAMMA1002042//ESTs//1.4e-20:199:79//Hs.140913:R44580
F-MAMMA1002047//EST//4.2e-14:170:75//Hs.124348:AA830225
F-MAMMA1002056//EST//2.1e-49:414:80//Hs.162335:AA564256
F-MAMMA1002058//EST//4.7e-26:268:78//Hs.140520:AA809305
F-MAMMA1002068//Human Line-1 repeat mRNA with 2 open reading frames//8.5e-36:382:75//Hs.23094:M19503
F-MAMMA1002078
F-MAMMA1002082
F-MAMMA1002084//EST//0.37:351:59//Hs.46576:N46012
F-MAMMA1002093//Homo sapiens mRNA for ATP-dependent RNA helicase, partial//0.54:388:57//Hs.99423:AJ010840
F-MAMMA1002108//Loricrin//0.00066:410:56//Hs.155657:M61120
F-MAMMA1002118//EST//0.50:202:64//Hs.126872:AA932932
F-MAMMA1002125//Small inducible cytokine A5 (RANTES)//2.4e-39:272:86//Hs.155464:AF088219
F-MAMMA1002132//EST//6.4e-05:245:60//Hs.149361:AI272963
F-MAMMA1002140//ESTs//5.8e-33:212:77//Hs.141203:H52638
F-MAMMA1002143//SERUM PROTEIN MSE55//1.9e-12:192:70//Hs.148101:M88338
F-MAMMA1002145//EST//0.12:204:60//Hs.160983:AI392837
F-MAMMA1002153
F-MAMMA1002155//ESTs, Weakly similar to p40 [H.sapiens]//3.6e-67:335:97//Hs.88424:AA281385
F-MAMMA1002156//Integrin, beta 3 (platelet glycoprotein IIIa, antigen CD61)//0.99:310:58//Hs.87149:M35999
F-MAMMA1002158//EST//0.015:278:58//Hs.162666:AA605196
F-MAMMA1002170//40S RIBOSOMAL PROTEIN S2//6.9e-82:573:82//Hs.119389:X17206
F-MAMMA1002174//Human NOF1 mRNA, complete cds//2.2e-42:375:78//Hs.75859:U39400
F-MAMMA1002198//H.sapiens mRNA for thiol-specific antioxidant//3.3e-36:121:98//Hs.146354:Z22548
F-MAMMA1002209//ESTs//1.1e-84:409:98//Hs.139235:AA278362
F-MAMMA1002215//Loricrin//0.0024:369:57//Hs.155657:M61120
F-MAMMA1002219//ESTs, Weakly similar to coded for by C. elegans cDNA yk52b10.3 [C.elegans]//9.5e-41:202:100//Hs.118849:AA215645
F-MAMMA1002230//ESTs//0.92:253:60//Hs.4222:AI024063
F-MAMMA1002236//ESTs, Moderately similar to initiation factor eIF-2B gamma subunit [R.norvegicus]//4.6e-69:344:90//Hs.76822:AI359536
F-MAMMA1002243//Homo sapiens serine threonine kinase 11 (STK11) mRNA, complete cds//0.99:454:56//Hs.122755:AF032986
F-MAMMA1002250//Human involucrin mRNA//0.0037:396:62//Hs.157091:M13903
F-MAMMA1002267//ESTs//2.0e-12:296:62//Hs.155686:AI308841
F-MAMMA1002268//Human N-type calcium channel alpha-1 subunit mRNA, complete cds//1.2e-06:427:61//Hs.69949:M94172
F-MAMMA1002269
F-MAMMA1002282//ESTs//5.9e-65:342:95//Hs.13962:T72715
F-MAMMA1002292//EST//0.0050:346:58//Hs.97639:AA398440
F-MAMMA1002293//Homo sapiens DNA fragmentation factor 40 kDa subunit (DFF40) mRNA, complete cds//2.8e-60:387:75//Hs.133089:AF064019
F-MAMMA1002294//Human growth/differentiation factor 1 (GDF-1) mRNA, complete cds//4.3e-07:349:64//Hs.92614:M62302
F-MAMMA1002297//EST//0.98:98:68//Hs.148207:AA897460
F-MAMMA1002298//Paired basic amino acid cleaving system 4//0.0061:471:57//Hs.77234:AB001914
F-MAMMA1002299//ESTs//1.0:162:68//Hs.134132:AA205935
F-MAMMA1002308//ESTs, Weakly similar to !!!! ALU SUBFAMILY J WARNING ENTRY !!!! [H.sapiens]//6.9e-41:293:83//Hs.105292:AA504776
F-MAMMA1002310//Homo sapiens serine protease-like protease (nes1) mRNA, complete cds//0.0037:173:67//Hs.69423:AF055481
F-MAMMA1002311//Homo sapiens mRNA, chromosome 1 specific transcript KIAA0487//1.8e-41:473:65//Hs.92381:AB007956
F-MAMMA1002312//ESTs//0.0017:279:60//Hs.163773:AA806291
F-MAMMA1002317//ESTs//1.0:131:64//Hs.66075:F08908
F-MAMMA1002319//Homo sapiens clone 24566 mRNA sequence//1.2e-28:410:68//Hs.133342:AF070536
F-MAMMA1002322//ESTs//1.2e-47:356:82//Hs.152413:AA780515
F-MAMMA1002329//Homo sapiens clone 24444 RaP2 interacting protein 8 (RPIP8) mRNA, complete cds//0.0079:143:67//Hs.6755:AF055026
F-MAMMA1002332//Human kpni repeat mrna (cdna clone pcd-kpni-8), 3' end//1.2e-26:342:72//Hs.103948:K00627
F-MAMMA1002333//Homo sapiens mRNA for KIAA0711 protein, complete cds//6.8e-07:669:58//Hs.5333:AB018254
F-MAMMA1002339//H.sapiens mRNA for retrotransposon//3.2e-40:348:73//Hs.6940:Z48633
F-MAMMA1002347//EST, Moderately similar to !!!! ALU SUBFAMILY J WARNING ENTRY !!!! [H.sapiens]//3.9e-14:146:81//Hs.163073:R02591
F-MAMMA1002351//ESTs//1.2e-74:371:96//Hs.111429:W28907
F-MAMMA1002352//EST//1.7e-09:198:68//Hs.149218:AI247086
F-MAMMA1002353//ESTs//7.4e-15:163:77//Hs.157253:AI357539
F-MAMMA1002355//Homo sapiens KIAA0441 mRNA, complete cds//7.7e-47:307:78//Hs.32511:AB007901
F-MAMMA1002356//ESTs//0.012:380:58//Hs.105349:AA779733
F-MAMMA1002359//EST//1.1e-44:264:77//Hs.141095:H23818
F-MAMMA1002360//ESTs//7.6e-15:200:70//Hs.19770:AA447830
F-MAMMA1002361//ESTs//2.5e-29:277:79//Hs.155115:AA669923
F-MAMMA1002362//EST//0.25:304:58//Hs.1.62427:AA576345
F-MAMMA1002380//FACTOR VIII INTRON 22 PROTEIN//0.29:485:59//Hs.83363:M34677
F-MAMMA1002384//ESTs//1.1 e-05:220:65//Hs.141388:R52022
F-MAMMA1002385//ESTs, Moderately similar to T11G6.8 [C.elegans]//8.4e-118:578:97//Hs.25516:AI086362
F-MAMMA1002392//EST//0.85:319:57//Hs.126484:AA913624
F-MAMMA1002411//ESTs//0.00044:89:76//Hs.141685:AI142632
F-MAMMA1002413//ESTs//0.0020:303:61//Hs.94903:W85737
F-MAMMA1002417//ESTs//1.4e-06:223:65//Hs.143695:AA662745
F-MAMMMA1002427//ESTs//5.4e-48:356:82//Hs.146811:AA410788
F-MAMMA1002428//EST//1.0:96:71//Hs.105130:AA482030
F-MAMMA1002434//Human mRNA for KIAA0118 gene, partial cds//2.2e-52:370:83//Hs.154326:D42087
F-MAMMA1002446
F-MAMMA1002454//ESTs//9.1e-50:163:100//Hs.80162:AA534809
F-MAMMA1002461//Human diacylglycerol kinase (DAGK) mRNA, complete cds//6.3e-06:595:59//Hs.99932:L38707
F-MAMMA1002470
F-MAMMA1002475//Human MAP kinase activated protein kinase 2 mRNA, complete cds//0.018:417:58//Hs.75074:U12779
F-MAMMA1002480//ESTs//0.0015:258:62//Hs.132082:N67059
F-MAMMA1002485//Homo sapiens stanniocalcin-2 (STC-2) mRNA, complete cds//9.4e-120:560:98//Hs.155223:AF055460
F-MAMMA1002494//ESTs//2.4e-68:359:95//Hs.124652:AA857628
F-MAMMA1002498//ESTs, Weakly similar to hypothetical protein [H.sapiens]//4.0e-07:257:63//Hs.133013:AA604920
F-MAMMA1002524//Huntingtin (Huntington disease)//0.0085:215:65//Hs.79391:L12392
F-MAMMA1002530//Homo sapiens cytosolic phospholipase A2 gamma (cPLA2 gamma) mRNA, complete cds//4.5e-162:775:97//Hs.18858:AF065214
F-MAMMA1002545//ESTs//6.4e-46:351:81//Hs.146811:AA410788
F-MAMMA1002554
F-MAMMA1002556//Human beige-like protein (BGL) mRNA, partial cds//0.96:187:62//Hs.62354:M83822
F-MAMMA1002566//ESTs//0.0033:130:68//Hs.117018:AA832421
F-MAMMA1002571//EST//0.28:115:66//Hs.156768:AI351368
F-MAMMA1002573//ESTs//2.1e-4.8:265:94//Hs.155128:AI224516
F-MAMMA1002585
F-MAMMA1002590//ESTs//3.2e-11:280:63//Hs.36049:AA436831
F-MAMMA1002597//ESTs//4.8e-10:118:77//Hs.156166:AI334107
F-MAMMA1002598//Ribosomal protein L7//3.6e-23:123:100//Hs.153:X57958
F-MAMMA1002603//EST//0.070:99:71//Hs.122387:AA789220
F-MAMMA1002612//ESTs, Moderately similar to hCDC10 protein [H.sapiens]//8.3e-18:353:65//Hs.60895:AA428463
F-MAMMA1002617//B94 PROTEIN//0.0097:229:62//Hs.75522:M92357
F-MAMMA1002618
F-MAMMA1002619
F-MAMMA1002622//Homo sapiens advillin mRNA, complete cds//4.7e-22:157:90//Hs.47344:AF041449
F-MAMMA1002623//EST//1.5e-33:168:81//Hs.141526:N52300
F-MAMMA1002625
F-MAMMA1002629//Homo sapiens mRNA, chromosome 1 specific transcript KIAA0507//1.1e-35:355:76//Hs.158241:AB007976
F-MAMMA1002636//Homo sapiens mRNA for KIAA0288 gene, complete cds//1.9e-05:439:61//Hs.91400:AB006626
F-MAMMA1002637//KINESIN LIGHT CHAIN//2.0e-47:367:72//Hs.117977:L04733
F-MAMMA1002646//EST//1.2e-32:302:78//Hs.112540:AA601385
F-MAMMA1002650//TRICHOHYALIN//1.2e-08:570:63//Hs.82276:L09190
F-MAMMA1002655//EST//8.8e-40:198:100//Hs.159724:AI393335
F-MAMMA1002662//EST//0.99:95:63//Hs.144074:AI005489
F-MAMMA1002665//Lysosomal-associated membrane protein 2//1.8e-35:722:64//Hs.8262:U36336
F-MAMMA1002671//Cyclin-dependent kinase inhibitor 1C (p57, Kip2)//8.6e-06:272:64//Hs.106070:U22398
F-MAMMA1002673
F-MAMMA1002684//Homo sapiens mRNA for KIAA0214 protein, complete cds//1.2e-162:752:99//Hs.3363:D86987
F-MAMMA1002685//ESTs//7.5e-40:373:78//Hs.163937:N69915
F-MAMMA1002698//ESTs//2.5e-09:190:68//Hs.138292:AI220397
F-MAMMA1002699//Homo sapiens epsin 2b mRNA, complete cds//4.7e-56:398:81//Hs.22396:AF062085
F-MAMMA1002701//ESTs//4.3e-10:110:80//Hs.156041:AI274697
F-MAMMA1002708//Homo sapiens mRNA for alpha(1,2)fucosyltransferase, complete cds//1.1e-51:307:79//Hs.46328:D87942
F-MAMMA1002711//EST//3.6e-38:186:77//Hs.139715:N25041
F-MAMMA1002721//EST//3.9e-06:110:71//Hs.136758:AA714692
F-MAMMA1002727//EST//0.97:137:63//Hs.145153:AI150165
F-MAMMA1002728//ESTs, Highly similar to PAB-DEPENDENT POLY(A)-SPECIFIC RIBONUCLEASE [Saccharomyces cerevisiae]//2.6e-12:129:81//Hs.154181:AA193502
F-MAMMA1002744//ESTs//0.0026:420:58//Hs.95793:AA617853
F-MAMMA1002746//ESTs//0.28:117:69//Hs.12925:T66312
F-MAMMA1002748
F-MAMMA1002754//ESTs//1.1e-34:340:77//Hs.163641:R61848
F-MAMMA1002758//Homo sapiens KIAA0442 mRNA, partial cds//1.1e-27:151:98//Hs.32168:AB007902
F-MAMMA1002764//ESTs//1.7e-45:323:84//Hs.155243:N70293
F-MAMMA1002765//EST//3.2e-11:145:73//Hs.162551:AA584782
F-MAMMA1002769
F-MAMMA1002775//Human ABL gene, exon 1b and intron 1b, and putative M8604 Met protein (M8604 Met) gene//7.6e-84:417:97//Hs.77705:U07563
F-MAMMA1002780//EST//0.78:210:63//Hs.149413:AI273988
F-MAMMA1002782
F-MAMMA1002796//ESTs//0.021:122:65//Hs.132221:AI380710
F-MAMMA1002807//EST//1.0e-31:184:71//Hs.161497:N66919
F-MAMMA1002820//ESTs//0.21:292:59//Hs.132513:AI778514
F-MAMMA1002830//Homo sapiens mRNA for KIAA0563 protein, complete cds//2.4e-57:286:88//Hs.15731:AB011135
F-MAMMA1002833//Human mRNA for KIAA0033 gene, partial cds//9.1e-52:583:72//Hs.22271:D26067
F-MAMMA1002835
F-MAMMA1002838//ESTs, Weakly similar to NADH-UBIQUINONE OXIDOREDUCTASE CHAIN 1 [Locusta migratoria]//7.7e-38:179:78//Hs.141344:H29951
F-MAMMA1002842//ESTs//1.7e-19:134:89//Hs.111583:AA463590
F-MAMMA1002843//Homo sapiens mRNA for KIAA0810 protein, partial cds//5.4e-137:635:99//Hs.7531:AB018353
F-MAMMA1002844//ESTs, Weakly similar to Y53C12A.3 [C.elegans]//1.6e-07:329:58//Hs.107747:AI357868
F-MAMMA1002858
F-MAMMA1002868//EST//4.1e-23:180:77//Hs.163196:AA767643
F-MAMMA1002869//Human PINCH protein mRNA, complete cds//7.0e-88:696:78//Hs.83987:U09284
F-MAMMA1002871//ESTs//3.4e-93:466:96//Hs.11873:T68423
F-MAMMA1002880//EST//2.0e-09:364:59//Hs.145181:AI183632
F-MAMMA1002881//Homo sapiens mRNA for 25 kDa trypsin inhibitor, complete cds//3.8e-30:680:61//Hs.129732:D45027
F-MAMMA1002886//Long (electrocardiographic) QT syndrome 2//0.00075:504:60//Hs.19944:U04270
F-MAMMA1002887//ESTs//0.044:144:68//Hs.133152:H91657
F-MAMMA1002890//EST//1.7e-05:74:86//Hs.116013:AA612666
F-MAMMA1002892//EST//2.1e-67:383:93//Hs.22815:R44265
F-MAMMA1002895//Human transcription factor ERF-1 mRNA, complete cds//0.00053:382:57//Hs.61796:U85658
F-MAMMA1002908//EST//0.0022:132:68//Hs.161697:AA224952
F-MAMMA1002909//ESTs//9.1e-21:343:70//Hs.142068:AA176125
F-MAMMA1002930//ESTs//0.55:72:72//Hs.132440:AA923730
F-MAMMA1002937//ESTs, Weakly similar to ZINC FINGER PROTEIN 84 [H.sapiens]//7.9e-103:485:99//Hs.102928:AI346344
F-MAMMA1002938//Homo sapiens mRNA for KIAA0698 protein, complete cds//1.6e-194:910:98//Hs.31720:AB014598
F-MAMMA1002941//ESTs//9.5e-19:196:67//Hs.137945:AI423389
F-MAMMA1002947//ESTs//1.2e-96:460:99//Hs.156001:AI313418
F-MAMMA1002964//Homo sapiens KIAA0424 mRNA, partial cds//0.48:250:60//Hs.54697:AB007884
F-MAMMA1002970//EST//2.0e-16:132:84//Hs.136518:AA601400
F-MAMMA1002972
F-MAMMA1002973//ESTs//3.2e-43:225:74//Hs.155179:AA223932
F-MAMMA1002982//ESTs//0.0017:162:66//Hs.152669:AA604944
F-MAMMA1002987//EST//0.044:254:59//Hs.135014:AI095645
F-MAMMA1003003//Coagulation factor III (thromboplastin, tissue factor)//3.9e-22:185:83//Hs.62192:J02931
F-MAMMA1003004//Homo sapiens mRNA, chromosome 1 specific transcript KIAA0501//1.0e-16:343:61//Hs.159897:AB007970
F-MAMMA1003007//EST//6.6e-10:265:66//Hs.144389:AA530979
F-MAMMA1003011//Homo sapiens histone macroH2A1.2 mRNA, complete cds//6.2e-51:620:69//Hs.75258:AF054174
F-MAMMA1003013//Human HOX4C mRNA for a homeobox protein//0.73:347:58//Hs.74061:X59372
F-MAMMA1003015//EST//2.5e-11:137:77//Hs.141312:H73062
F-MAMMA1003019//ESTs//0.0099:182:65//Hs.60787:AI374951
F-MAMMA1003026//EST//1.0:136:67//Hs.9123:T50137
F-MAMMA1003031//EST//1.3e-11:244:67//Hs.136611:AA669549
F-MAMMA1003035
F-MAMMA1003039//ESTs//1.4e-23:265:74//Hs.33393:R83391
F-MAMMA1003040//Homo sapiens tapasin (NGS-17) mRNA, complete cds//1.5e-93:339:85//Hs.5247:AF029750
F-MAMMA1003044//Cyclin D2//1.0:234:61//Hs.75586:D13639
F-MAMMA1003047//H.sapiens mRNA for F25B3.3 kinase like protein from C.elegans//1.0:209:60//Hs.99491:Y12336
F-MAMMA1003049//EST//0.99:126:67//Hs.162634:AA601742
F-MAMMA1003055//ESTs//0.00011:130:70//Hs.130539:R68518
F-MAMMA1003056
F-MAMMA1003057//ESTs, Moderately similar to hypothetical protein MD6 [M.musculus]//1.3e-88:334:97//Hs.96500:AI206781
F-MAMMA1003066//ESTs//0.77:88:71//Hs.143618:AI022618
F-MAMMA1003089//Homo sapiens mRNA for KIAA0631 protein, partial cds//4.5e-51:329:71//Hs.75154:AB014531
F-MAMMA1003099//Homo sapiens actin-binding protein homolog ABP-278 mRNA, complete cds//8.5e-44:288:88//Hs.81008:AF043045
F-MAMMA1003104//H.sapiens mRNA for ASM-like phosphodiesterase 3a//1.0:213:60//Hs.42945:Y08136
F-MAMMA1003113//Homo sapiens mRNA for hair keratin acidic 3-II//0.99:200:64//Hs.32950:X82634
F-MAMMA1003127//Homo sapiens brush border myosin I (BBMI) mRNA, complete cds//5.4e-27:421:66//Hs.5394:AF105424
F-MAMMA1003135//Envoplakin//0.56:250:62//Hs.25482:U53786
F-MAMMA1003140
F-MAMMA1003146//Homo sapiens mRNA for GalT3 protein//7.2e-82:397:97//Hs.151344:Y15062
F-MAMMA1003150//Homo sapiens mRNA for KIAA0515 protein, partial cds//0.00019:297:61//Hs.108945:AB011087
F-MAMMA1003166//Glycoprotein Ib (platelet), beta polypeptide//1.2e-31:487:65//Hs.3847:U59632
F-NT2RM1000001//Human plectin (PLEC1) mRNA, complete cds//0.16:244:63//Hs.79706:U53204
F-NT2RM1000018//Human mRNA for KIAA0066 gene, partial cds//1.5e-66:385:92//Hs.82510:D31886
F-NT2RM1000032
F-NT2RM1000035//Human mRNA for KIAA0199 gene, partial cds//4.1e-110:849:81//Hs.78442:D83782
F-NT2RM1000037//Homo sapiens mRNA for KIAA0690 protein, partial cds//3.5e-108:542:95//Hs.60103:AB014590
F-NT2RM1000039//Human plectin (PLEC1) mRNA, complete cds//0.11:545:57//Hs.79706:U53204
F-NT2RM1000055//ESTs, Highly similar to TIP120 [R.norvegicus]//3.2e-69:353:96//Hs.154980:AA948067
F-NT2RM1000059//Homo sapiens T cell immune response cDNA7 (TIRC7) mRNA, complete cds//0.029:281:59//Hs.46465:U45285
F-NT2RM1000062//ESTs//0.30:368:59//Hs.131675:AA843210
F-NT2RM1000080//Homo sapiens chromosome 9, P1 clone 11659//2.8e-102:493:97//Hs.3439:AC004472
F-NT2RM1000086//Homo sapiens mRNA for KIAA0661 protein, complete cds//5.8e-116:550:97//Hs.65238:AB014561
F-NT2RM1000092//Murine leukemia viral (bmi-1) oncogene homolog//0.42:190:63//Hs.431:L13689
F-NT2RM1000118//Homo sapiens clone 23763 unknown mRNA, partial cds//0.00086:126:70//Hs.92693:AF007155
F-NT2RM1000119//Peroxisome receptor 1//0.00055:458:58//Hs.158084:Z48054
F-NT2RM1000127
F-NT2RM1000131
F-NT2RM1000132//Homo sapiens NADH:ubiquinone oxidoreductase NDUFS6 subunit mRNA, nuclear gene encoding mitochondrial protein, complete cds//3.7e-92:448:97//Hs.49767:AF044959
F-NT2RM1000153//Homo sapiens mRNA for MTG8-related protein MTG16a, complete cds//1.0:546:58//Hs.110099:AB010419
F-NT2RM1000186//Homo sapiens clone 23763 unknown mRNA, partial cds//0.00081:126:70//Hs.92693:AF007155
F-NT2RM1000187//ESTs//3.4e-79:400:96//Hs.54971:AI424382
F-NT2RM1000199//Homo sapiens mRNA for KIAA0722 protein, complete cds//0.87:454:59//Hs.47061:AF045458
F-NT2RM1000242
F-NT2RM1000244//Homo sapiens centrosomal Nek2-associated protein 1 (C-NAP1) mRNA, complete cds//0.97:135:66//Hs.27910:AF049105
F-NT2RM1000252//TRICHOHYALIN//0.030:273:58//Hs.82276:L09190
F-NT2RM1000256//Glutamine-fructose-6-phosphate transaminase//1.5e-13:248:69//Hs.1674:M90516
F-NT2RM1000257//ESTs, Highly similar to similar to mago nashi [H.sapiens]//2.9e-98:530:93//Hs.104650:AI037879
F-NT2RM1000260//Human mRNA for KIAA0130 gene, complete cds//2.1e-58:460:80//Hs.23106:D50920
F-NT2RM1000271//ESTs//0.93:224:60//Hs.91226:AA649047
F-NT2RM1000272
F-NT2RM1000280//ESTs, Highly similar to VACUOLAR ATP SYNTHASE SUBUNIT D [Bos taurus]//1.3e-21:308:73//Hs.15071:AA781144
F-NT2RM1000300
F-NT2RM1000314//Human mRNA for KIAA0159 gene, complete cds//2.6e-128:708:92//Hs.5719:D63880
F-NT2RM1000318//Human mRNA for ribosomal protein L39, complete cds//1.8e-35:182:99//Hs.9837:D79205
F-NT2RM1000341//ESTs//2.3e-72:381:95//Hs.23070:AA631976
F-NT2RM1000354//EST//5.2e-27:202:84//Hs.151186:AI125798
F-NT2RM1000355//ESTs, Weakly similar to putative [M.musculus]//7.7e-75:387:95//Hs.108619:W28608
F-NT2RM1000365//ESTs//1.7e-99:495:97//Hs.103926:AA165691
F-NT2RM1000377//ESTs, Weakly similar to protein-tyrosine-phosphatase [H.sapiens]//7.4e-91:481:95//Hs.163707:AA137181
F-NT2RM1000388//65 KD YES-ASSOCIATED PROTEIN//0.36:340:57//Hs.8939:X80507
F-NT2RM1000394//HISTONE H3.3//8.5e-91:474:93//Hs.118838:M11353
F-NT2RM1000399
F-NT2RM1000421
F-NT2RM1000430//Homo sapiens erythroblast macrophage protein EMP mRNA, complete cds//1.2e-85:418:97//Hs.20815:AF084928
F-NT2RM1000499//ESTs, Weakly similar to KIAA0167 protein [H.sapiens]//1.6e-38:201:97//Hs.106262:AI052382
F-NT2RM1000539//EST//0.070:145:62//Hs.149711:AI284660
F-NT2RM1000553//EST//2.2e-48:265:95//Hs.99230:AA449847
F-NT2RM1000555//ESTs//0.82:193:61//Hs.96944:AI359957
F-NT2RM1000563//Human plectin (PLEC1) mRNA, complete cds//1.0:336:58//Hs.79706:U53204
F-NT2RM1000623//Homo sapiens mRNA for KIAA0287 gene, partial cds//0.98:226:61//Hs.17931:AB006625
F-NT2RM1000648//ESTs, Weakly similar to similar to M. musculus MER5 and other AHPC/TSA proteins [C.elegans]//6.2e-51:254:98//Hs.132096:AA314601
F-NT2RM1000661//Homo sapiens translation initiation factor 4e mRNA, complete cds//8.5e-55:276:97//Hs.19122:AF038957
F-NT2RM1000666//Homo sapiens BAI 1 mRNA, complete cds//0.87:274:60//Hs.113936:AB005297
F-NT2RM1000669//ESTs//5.5e-63:481:85//Hs.90527:AI188279
F-NT2RM1000672
F-NT2RM1000691//Homa sapiens mRNA for HRIHFB2060, partial cds//7.0e-121:582:98//Hs.146282:AB015348
F-NT2RM1000699//ESTs//1.1e-89:435:97//Hs.28964:AA715101
F-NT2RM1000702//ESTs//5.4e-90:429:99//Hs.151001:AA564706
F-NT2RM1000725//Homo sapiens mRNA for neuropathy target esterase//1.5e-66:435:85//Hs.5038:AJ004832
F-NT2RM1000741//Homo sapiens mRNA for KIAA0567 protein, partial cds//2.6e-127:690:92//Hs.147946:AB011139
F-NT2RM1000742//Homo sapiens AC133 antigen mRNA, complete cds//8.2e-68:524:83//Hs.112360:AF027208
F-NT2RM1000746//ESTs//2.6e-37:231:89//Hs.94446:AA845465
F-NT2RM1000770//Homo sapiens KIAA0425 mRNA, complete cds//3.3e-09:321:63//Hs.150390:AB007885
F-NT2RM1000772//Eukaryotic translation initiation factor 3 (eIF-3) p36 subunit//0.053:271:60//Hs.139745 :U39067
F-NT2RM1000780//Human Line-1 repeat mRNA with 2 open reading frames//6.9e-20:128:94//Hs.23094:M19503
F-NT2RM1000781//ESTs//4.4e-60:346:92//Hs.35089:N50845
F-NT2RM1000800
F-NT2RM1000802
F-NT2RM1000811//Homo sapiens AC133 antigen mRNA, complete cds//1.2e-64:490:84//Hs.112360:AF027208
F-NT2RM1000826//ESTs//0.82:193:61//Hs.96944:AI359957
F-NT2RM1000829//Mannose-binding lectin, soluble (opsonic defect)//0.92:283:58//Hs.2314:X15422
F-NT2RM1000833//Hydroxysteroid (11-beta) dehydrogenase 2//0.022:178:67//Hs.1376:U26726
F-NT2RM1000850//Human protein tyrosine kinase related mRNA sequence//3.8e-06:384:59//Hs.90314:L05148
F-NT2RM1000852//Homo sapiens mRNA for ATP-dependent RNA helicase, partial//3.0e-149:726:97//Hs.99423:AJ010840
F-NT2RM1000857//ESTs//0.52:274:60//Hs.112095:AA447643
F-NT2RM1000867//ESTs, Highly similar to signal peptidase:SUBUNIT//5.3e-54:277:96//Hs.11125:AI015619
F-NT2RM1000874//ESTs//0.032:185:64//Hs.97713:AA442239
F-NT2RM1000882//Homo sapiens chromosome 11, BAC CIT-HSP-311e8 (BC269730) containing the hFEN1 gene//4.0e-155:750:97//Hs.132898:AC004770
F-NT2RM1000883//Homo sapiens I-1 receptor candidate protein mRNA, complete cds//8.8e-158:762:97//Hs.26285:AF082516
F-NT2RM1000885//Homo sapiens mRNA for KIAA0661 protein, complete cds//6.3e-19:310:67//Hs.65238:AB014561
F-NT2RM1000894
F-NT2RM1000898
F-NT2RM1000905//EST//4.8e-07:77:84//Hs.148017:AI268701
F-NT2RM1000924//HOMEOBOX PROTEIN HOX-A5//0.00051:458:59//Hs.37034:M26679
F-NT2RM1000927//Homo sapiens mRNA for KIAA0807 protein, partial cds//0.084:386:58//Hs.101474:AB018350
F-NT2RM1000962//Human mRNA for KIAA0252 gene, partial cds//0.98:299:59//Hs.83419:D87440
F-NT2RM1000978
F-NT2RM1001003//Homo sapiens alpha-catenin related protein (ACRP) mRNA, complete cds//1.3e-161:760:98//Hs.58488:U97067
F-NT2RM1001008//ESTs//1.3e-12:144:75//Hs.133122:AI025200
F-NT2RM1001043//EST//0.24:117:64//Hs.161536:N80395
F-NT2RM1001044//ESTs, Weakly similar to C43E11.9[C.elegans]//3.0e-98:491:96//Hs.102173:AA045270
F-NT2RM1001059//Human plectin (PLEC1) mRNA, complete cds//0.52:533:57//Hs.79706:U53204
F-NT2RM1001066//ESTs//1.2e-114:538:99//Hs.129020:AI380703
F-NT2RM1001072//Human beige-like protein (BGL) mRNA, partial cds//0.69:586:56//Hs.62354:M83822
F-NT2RM1001074//Macrophage stimulating 1 (hepatocyte growth factor-like)//0.0019:294:64//Hs.30223:X90846
F-NT2RM1001082//Archain//3.9e-37:290:81//Hs.33642:X81198
F-NT2RM1001085
F-NT2RM1001092//Zinc finger protein 43 (HTF6)//1.9e-57:770:68//Hs.74107:X59244
F-NT2RM1001102//ESTs//1.2e-35:638:63//Hs.131737:AI343331
F-NT2RM1001105//WEE1-LIKE PROTEIN KINASE//0.0024:246:63//Hs.75188:U10564
F-NT2RM1001112//ESTs//8.9e-82:437:93//Hs.6330:H38495
F-NT2RM1001115
F-NT2RM1001139//Keratin 9//1.5e-05:518:59//Hs.2783:Z29074
F-NT2RM2000006//ESTs//3.9e-16:96:98//Hs.101117:AA576113
F-NT2RM2000013//RNA polymerase II polypeptide B (140 kD)//6.3e-13:640:59//Hs.148027:X63563
F-NT2RM2000030
F-NT2RM2000032//ESTs//7.1 e-18:138:68//Hs.114031:AA700958
F-NT2RM2000042//ESTs//0.0091:241:61//Hs.147895:AI286243
F-NT2RM2000092
F-NT2RM2000093//ESTs//2.6e-40:226:94//Hs.163521:H42085
F-NT2RM2000101//ESTs//1.0:235:61//Hs.48860:N27428
F-NT2RM2000124//Protein kinase, cAMP-dependent, catalytic, alpha//5.8e-46:287:88//Hs.77271:X07767
F-NT2RM2000191//Homo sapiens cGMP phosphodiesterase A1 (PDE9A) mRNA, complete cds//3.0e-139:566:97//Hs.18953:AF067223
F-NT2RM2000192//EST//3.5e-07:168:65//Hs.163122:AA756999
F-NT2RM2000239//ESTs, Weakly similar to K04G2.6 [C.elegans]//3.6e-93:489:95//Hs.143499:R72672
F-nnnnnnnnnnnn//ESTs//1.0e-70:269:97//Hs.156175:AI334328
F-NT2RM2000250//Homo sapiens mRNA for KIAA0590 protein, complete cds//1.0e-129:615:98//Hs.111862:AB011162
F-NT2RM2000259//ESTs//6.1e-30:172:85//Hs.116406:AA209520
F-NT2RM2000260//ESTs//2.5e-25:133:93//Hs.14169:AA203500
F-NT2RM2000287//ESTs//6.2e-13:97:83//Hs.118523:H98981
F-NT2RM2000322//Interferon regulatory factor 5//0.84:208:61//Hs.54434:U51127
F-NT2RM2000359//Homo sapiens mRNA for KIAA0560 protein, complete cds//2.8e-176:805:99//Hs.129952:AB011132
F-NT2RM2000363//ESTs//1.2e-24:139:96//Hs.48818:N63543
F-NT2RM2000368//Homo sapiens protein kinase C-binding protein RACK7 mRNA, partial cds//3.7e-96:599:86//Hs.75871:U48251
F-NT2RM2000371
F-NT2RM2000374//ESTs//3.2e-13:98:91//Hs.65853:AI050866
F-NT2RM2000395//Growth arrest-specific 1//0.80:129:67//Hs.65029:L13698
F-NT2RM2000402//Human p76 mRNA, complete cds//7.2e-23:714:59//Hs.28757:U81006
F-NT2RM2000407//ESTs//9.4e-92:458:96//Hs.148873:T33582
F-NT2RM2000420//EST//1.8e-61:296:99//Hs.147186:AI193053
F-NT2RM2000422//Solute carrier family 6 (neurotransmitter transporter, serotonin), member 4//1.5e-06:260:61//Hs.553:L05568
F-NT2RM2000452//ESTs//1.0:132:62//Hs.110004:AI097379
F-NT2RM2000469//ESTs//0.34:249:60//Hs.149575:AI281807
F-NT2RM2000490//Homo sapiens mRNA for KIAA0747 protein, partial cds//2.4e-16:386:63//Hs.8309:AB018290
F-NT2RM2000502//Human nicotinamide N-methyltransferase (NNMT) mRNA, complete cds//0.99:272:61//Hs.76669:U08021
F-NT2RM2000504//Homo sapiens metalloprotease 1 (MP1) mRNA, complete cds//1.6e-172:824:97//Hs.4812:AF061243
F-NT2RM2000522//Homo sapiens Nck-2 (NCK2) mRNA, complete cds//0.18:313:60//Hs.129725:AF047487
F-NT2RM2000540//ESTs, Weakly similar to C27F2.7 gene product [C.elegans]//2.7e-41:231:94//Hs.7049:AI141736
F-NT2RM2000556//ESTs//3.1e-33:183:96//Hs.136990:AA769220
F-NT2RM2000566//Integrin, alpha 7B//2.0e-155:751:97//Hs.74369:AF032108
F-NT2RM2000567//RYANODINE RECEPTOR, SKELETAL MUSCLE//6.3e-09:689:59//Hs.89631:U48508
F-NT2RM2000569//ESTs//5.4e-17:170:77//Hs.158277:H09128
F-NT2RM2000577//ESTs, Highly similar to ISOLEUCYL-TRNA SYNTHETASE, MITOCHONDRIAL [Saccharomyces cerevisiae]//1.4e-33:214:92//Hs.55609:W37993
F-NT2RM2000581//Homo sapiens mRNA for KIAA0214 protein, complete cds//1.8e-175:820:98//Hs.3363:D86987
F-NT2RM2000588//ESTs//1.5e-33:183:97//Hs.136990:AA769220
F-NT2RM2000594
F-NT2RM2000599//Homo sapiens Mad4 homolog (Mad4) mRNA, complete cds//0.017:253:65//Hs.102402:AF040963
F-NT2RM2000609//ESTs//1.0:220:59//Hs.110155:AA007313
F-NT2RM2000612//ESTs//0.97:208:59//Hs.73217:AA846548
F-NT2RM2000623//Homo sapiens mRNA for KIAA0521 protein, partial cds//0.024:326:59//Hs.6150:AB011093
F-NT2RM2000624//ESTs//2.3e-118:557:99//Hs.145904:AA203258
F-NT2RM2000635//Homo sapiens mRNA for KIAA0729 protein, partial cds//2.0e-143:664:98//Hs.19542:AB018272
F-NT2RM2000636//Homo sapiens mRNA for KIAA0658 protein, partial cds//2.4e-139:664:98//Hs.7278:AB014558
F-NT2RM2000639//ESTs//0.98:144:65//Hs.154364:AI189702
F-NT2RM2000649//Homo sapiens mRNA for KIAA0676 protein, partial cds//3.4e-169:518:99//Hs.115763:AB014576
F-NT2RM2000669//ESTs//1.3e-56:283:98//Hs.156342:AI337371
F-NT2RM2000691//Homo sapiens actin-related protein Arp3 (ARP3) mRNA, complete cds//6.7e-86:746:74//Hs.5321:AF006083
F-NT2RM2000714//Human mRNA for KIAA0231 gene, partial cds//2.2e-50:748:64//Hs.7938:D86984
F-NT2RM2000718//Homa sapiens mRNA for HRIHFB2436, partial cds//7.6e-126:594:98//Hs.136058:AB015342
F-NT2RM2000735//Zinc finger protein 43 (HTF6)//2.7e-112:756:82//Hs.74107:X59244
F-NT2RM2000740//ESTs, Highly similar to HYPOTHETICAL 132.7 KD HELICASE IN ALG7-ENP1 INTERGENIC REGION [Saccharomyces cerevisiae]//4.2e-85:464:91//Hs.161551:W24286
F-NT2RM2000795//Homo sapiens tapasin (NGS-17) mRNA, complete cds//1.0e-82:640:81//Hs.5247:AF029750
F-NT2RM2000821//Human mRNA for KIAA0340 gene, partial cds//0.32:679:59//Hs.105919:AB002338
F-NT2RM2000837//ESTs//2.3e-105:501:98//Hs.101514:AI346701
F-NT2RM2000951//Homo sapiens XYLB mRNA for xylulokinase, complete cds//2.8e-185:847:99//Hs.137580:AB015046
F-NT2RM2000952//ESTs, Weakly similar to lethal(2)denticleless [D.melanogaster]//6.2e-94:441:99//Hs.59075:AI023761
F-NT2RM2000984//Human mRNA for KIAA0246 gene, partial cds//0.94:351:62//Hs.84753:D87433
F-NT2RM2001004//ESTs//5.0e-10:247:64//Hs.36049:AA436831
F-NT2RM2001035//ESTs, Highly similar to POP2 PROTEIN [Saccharomyces cerevisiae]//2.9e-48:282:93//Hs.17035:AI080471
F-NT2RM2001065
F-NT2RM2001100//Homo sapiens mRNA for serin protease with IGF-binding motif, complete cds//1.7e-08:449:62//Hs.75111:D87258
F-NT2RM2001105//Homo sapiens proline and glutamic acid rich nuclear protein isoform mRNA, partial cds//0.00079:274:59//Hs.102732:U88153
F-NT2RM2001131//TRICHOHYALIN//2.5e-20:684:62//Hs.82276:L09190
F-NT2RM2001141
F-NT2RM2001152//ESTs//0.53:333:58//Hs.153087:AA649042
F-NT2RM2001177
F-NT2RM2001194//ESTs, Weakly similar to T28H10.2 [C.elegans]//2.4e-23:149:93//Hs.10618:AI288739
F-NT2RM2001196//ESTs//4.0e-98:486:97//Hs.59628:W91959
F-NT2RM2001201//Human mRNA for KIAA0005 gene, complete cds//2.8e-44:554:69//Hs.155291:D13630
F-NT2RM2001221//Homo sapiens mRNA for KIAA0806 protein, complete cds//0.97:165:64//Hs.24279:AB018349
F-NT2RM2001238//EST//6.8e-67:420:89//Hs.130586:AI004766
F-NT2RM2001243//V-jun avian sarcoma virus 17 oncogene homolog//0.87:125:64//Hs.75889:U65928
F-NT2RM2001247//Homo sapiens antigen NY-CO-16 mRNA, complete cds//0.0066:321:61//Hs.132206:AF039694
F-NT2RM2001256
F-NT2RM2001291//ESTs//1.1e-86:459:93//Hs.10267:W27845
F-NT2RM2001306//Homo sapiens paraoxonase (PON2) mRNA, complete cds//1.0:182:65//Hs.75221:AF001601
F-NT2RM2001312//ESTs//2.0e-35:338:70//Hs.141440:N21615
F-NT2RM2001319//ESTs, Weakly similar to No definition line found [C.elegans]//5.2e-30:277:77//Hs.25347:AI138605
F-NT2RM2001324//Homo sapiens mRNA for beta-spectrin III, complete cds//0.031:245:62//Hs.26915:AB008567
F-NT2RM2001345//ESTs//9.2e-91:428:99//Hs.151001:AA564706
F-NT2RM2001360//ESTs//0.98:45:80//Hs.133520:AA878905
F-NT2RM2001370//Human transportin (TRN) mRNA, complete cds//0.72:224:61//Hs.82925:U70322
F-NT2RM2001393//Mannosidase, alpha B, lysosomal//0.42:383:57//Hs.108969:U68382
F-NT2RM2001420//EST//1.0:287:62//Hs.125285:AA830378
F-NT2RM2001424//Homo sapiens mRNA for E1B-55kDa-associated protein//2.3e-97:453:99//Hs.155218:AJ007509
F-NT2RM2001499//Ecotropic retroviral receptor//5.4e-47:589:68//Hs.2928:X57303
F-NT2RM2001504//Homo sapiens agrin precursor mRNA, partial cds//0.25:328:60//Hs.68900:AF016903
F-NT2RM2001524//ESTs//1.0e-11:93:90//Hs.33687:R85969
F-NT2RM2001544//ESTs//1.0e-25:157:92//Hs.137451:AA351459
F-NT2RM2001547//ESTs//2.0e-29:168:96//Hs.116392:AA936262
F-NT2RM2001575//Sjogren syndrome antigen A1 (52kD, ribonucleoprotein autoantigen SS-A/Ro)//6.9e-28:582:64//Hs.1042:M62800
F-NT2RM2001582//ESTs, Moderately similar to red-1 [M.musculus]//0.0032:57:89//Hs.114722:AA448077
F-NT2RM2001588//Homo sapiens KIAA0442 mRNA, partial cds//2.3e-11:282:65//Hs.32168:AB007902
F-NT2RM2001592//ESTs//4.8e-73:372:95//Hs.163801:AI391729
F-NT2RM2001605//Homo sapiens clone 23592 mRNA sequence//7.3e-87:749:75//Hs.76272:S66431
F-NT2RM2001613//ESTs, Highly similar to PROTEIN TRANSPORT PROTEIN SEC61 ALPHA SUBUNIT [Canis familiaris]//1.3e-17:181:75//Hs.131840:AI016073
F-NT2RM2001632//EST//8.7e-18:222:76//Hs.160402:AI393918
F-NT2RM2001635//Homo sapiens mRNA for KIAA0618 protein, complete cds//3.0e-154:740:98//Hs.15832:AB014518
F-NT2RM2001637//ESTs//2.2e-06:386:61//Hs.145198:AI276952
F-NT2RM2001641//ESTs, Highly similar to NADH-CYTOCHROME B5 REDUCTASE [Bos taurus]//3.5e-13:94:92//Hs.22142:AA814725
F-NT2RM2001648//ESTs, Highly similar to PROTEIN TRANSPORT PROTEIN SEC61 ALPHA SUBUNIT [Canis familiaris]//1.3e-17:181:75//Hs.131840:AI016073
F-NT2RM2001652//ESTs//2.5e-06:82:80//Hs.128203:AA972301
F-NT2RM2001659//ESTs//2.8e-15:92:98//Hs.123321:AA810287
F-NT2RM2001664//Homo sapiens IkappaB kinase complex associated protein (IKAP) mRNA, complete cds//1.2e-173:802:99//Hs.31323:AF044195
F-NT2RM2001668//ESTs, Weakly similar to DNA MISMATSCH REPAIR PROTEIN MSH6 [H.sapiens]//1.1e-136:671:97//Hs.27721:U17907
F-NT2RM2001670//Homo sapiens mRNA for KIAA0557 protein, partial cds//1.1e-25:352:70//Hs.101414:AB011129
F-NT2RM2001671//ESTs//1.8e-08:63:98//Hs.158069:AI365356
F-NT2RM2001675
F-NT2RM20016811/ESTs//0.16:197:63//Hs.20585:R10305
F-NT2RM2001688//ESTs//1.8e-24:130:100//Hs.162504:AA668211
F-NT2RM2001695//EST//5.6e-51:189:89//Hs.162197:AA535216
F-NT2RM2001696//ESTs, Highly similar to gene ERCC5 protein [H.sapiens]//5.8e-16:144:84//Hs.14671:T79937
F-NT2RM2001698//ESTs//0.14:184:63//Hs.148080:AI277415
F-NT2RM2001699//ESTs//6.5e-14:136:79//Hs.127790:AI003817
F-NT2RM2001700//Homo sapiens putative seven pass transmembrane protein (TM7SF1) mRNA, complete cds//0.95:270:61//Hs.15791:AF027826
F-NT2RM2001706//ESTs//2.8e-47:304:86//Hs.146811:AA410788
F-NT2RM2001716//Semenogelin I//0.98:153:64//Hs.1968:M81650
F-NT2RM2001718
F-NT2RM2001723//Homo sapiens clone 23770 mRNA sequence//4.4e-28:163:95//Hs.12457:AF052123
F-NT2RM2001727//Homo sapiens mRNA for KIAA0462 protein, partial cds//2.0e-112:530:98//Hs.129937:AB007931
F-NT2RM2001730//Homo sapiens mRNA for KIAA0560 protein, complete cds//0.95:269:58//Hs.129952:AB011132
F-NT2RM2001743
F-NT2RM2001753//Human AF-6 mRNA, complete cds//0.095:350:59//Hs.100469:AB011399
F-NT2RM2001760//ESTs, Highly similar to PROTEIN TRANSPORT PROTEIN SEC61 ALPHA SUBUNIT [Canis familiaris]//1.3e-17:181:75//Hs.131840:AI016073
F-NT2RM2001768//ESTs//0.61:189:62//Hs.144847:AI222742
F-NT2RM2001771//Zinc finger protein 10 (KOX 1)//1.1e-66:669:71//Hs.2479:X78933
F-NT2RM2001782//YY1 transcription factor//0.094:149:65//Hs.97496:M77698
F-NT2RM2001784//ESTs//8.2e-31:190:92//Hs.144587:AI193595
F-NT2RM2001785//Homo sapiens chromosome 11, BAC CIT-HSP-311e8 (BC269730) containing the hFEN1 gene//1.6e-48:476:74//Hs.132898:AC004770
F-NT2RM2001797//Human mRNA for KIAA0065 gene, partial cds//6.1e-66:481:72//Hs.70617:D31763
F-NT2RM2001800//Human mRNA for transcriptional activator hSNF2b, complete cds//0.49:142:66//Hs.78202:U29175
F-NT2RM2001803//Homo sapiens IkappaB kinase complex associated protein (IKAP) mRNA, complete cds//2.7e-179:827:99//Hs.31323:AF044195
F-NT2RM2001805//EST//1.0:45:80//Hs.159007:AI381341
F-NT2RM2001813//EST//0.41:268:58//Hs.150031:AI292068
F-NT2RM2001823//H.sapiens mRNA for 218kD Mi-2 protein//9.7e-21:554:60//Hs.74441:X86691
F-NT2RM2001839//Homo sapiens calumein (Calu) mRNA, complete cds//1.2e-132:738:90//Hs.7753:AF013759
F-NT2RM2001840//Homo sapiens PYRIN (MEFV) mRNA, complete cds//5.8e-58:329:86//Hs.113283:AF018080
F-NT2RM2001855//ADP-ribosylation factor 5//1.0:301:60//Hs.77541:M57567
F-NT2RM2001867//ESTs, Weakly similar to ZK792.1 [C.elegans]//3.0e-28:421:66//Hs.8763:W30741
F-NT2RM2001879//ESTs//6.3e-43:234:94//Hs.122546:AA186723
F-NT2RM2001886//Homo sapiens mRNA for KIAA0710 protein, complete cds//6.1e-189:866:97//Hs.4198:AB014610
F-NT2RM2001896//Homo sapiens mRNA for JM23 protein, complete coding sequence (clone IMAGE 34581 and IMAGE 45355 and LLNLc110I133Q7 (RZPD Berlin))//3.0e-13:606:57//Hs.23170:AJ005892
F-NT2RM2001903//Homo sapiens mRNA for KIAA0462 protein, partial cds//9.4e-178:859:97//Hs.129937:AB007931
F-NT2RM2001930//Homo sapiens semaphorin F homolog mRNA, complete cds//4.2e-08:481:59//Hs.27621:U52840
F-NT2RM2001935//ESTs, Highly similar to MULTIDRUG RESISTANCE PROTEIN HOMOLOG 50 [Drosophila melanogaster]//0.37:424:60//Hs.118634:U66688
F-NT2RM2001936//Homo sapiens clone 614 unknown mRNA, complete sequence//2.2e-139:653:98//Hs.21811:AF091080
F-NT2RM2001950//ESTs//0.12:91:76//Hs.107295:W80392
F-NT2RM2001982
F-NT2RM2001983//Homo sapiens Tax interaction protein 2 mRNA, partial cds//1.2e-21:123:98//Hs.6454:AF089816
F-NT2RM2001989//Homo sapiens mRNA for DRIM protein//0.71:319:59//Hs.104135:AJ006778
F-NT2RM2001997//ESTs//1.7e-25:135:100//Hs.126894:AA932538
F-NT2RM2001998//ESTs, Weakly similar to Mi-2 protein [H.sapiens]//0.99:271:60//Hs.63888:AA203398
F-NT2RM2002004//Homo sapiens mRNA for KIAA0731 protein, partial cds//3.5e-37:509:65//Hs.6214:AB018274
F-NT2RM2002014//Homo sapiens mRNA for CRM1 protein, complete cds//0.79:429:58//Hs.79090:D89729
F-NT2RM2002030//Glutamine-fructose-6-phosphate transaminase//9.0e-89:822:73//Hs.1674:M90516
F-NT2RM2002049//ESTs//0.99:109:71//Hs.19303:AA928427
F-NT2RM2002055//ESTs//1.1e-91:453:98//Hs.158370:AI382154
F-NT2RM2002088//ESTs//6.1e-75:302:96//Hs.153471:AI198377
F-NT2RM2002091//RYANODINE RECEPTOR, SKELETAL MUSCLE//0.69:293:58//Hs.89631:U48508
F-NT2RM2002100//Homo sapiens mRNA for ATP-dependent RNA helicase, partial//2.5e-165:776:98//Hs.99423:AJ010840
F-NT2RM2002109//Homo sapiens glioma amplified on chromosome 1 protein (GAC1) mRNA, complete cds//7.6e-145:684:98//Hs.26312:AF030435
F-NT2RM2002128
F-NT2RM2002142//ESTs//0.0031:183:66//Hs.144505:AA757274
F-NT2RM2002145//Homo sapiens erythroblast macrophage protein EMP mRNA, complete cds//1.4e-144:800:92//Hs.20815:AF084928
F-NT2RM2002178//Homo sapiens mRNA for KIAA0467 protein, partial cds//1.7e-165:787:97//Hs.11147:AB007936
F-NT2RM2002580//Keratin 10 (epidermolytic hyperkeratosis; keratosis palmaris et plantaris)//0.064:291:61//Hs.99936:X14487
F-NT2RM4000024//RNA polymerase II polypeptide B (140 kD)//8.0e-10:610:59//Hs.148027:X63563
F-NT2RM4000027//ESTs//1.6e-64:352:94//Hs.21331:H93074
F-NT2RM4000030//ESTs//1.0:115:63//Hs.131055:AI391464
F-NT2RM4000046//ESTs//2.6e-09:207:65//Hs.143533:AI094674
F-NT2RM4000061//ESTs//0.89:207:60//Hs.98445:AI038511
F-NT2RM4000085//ESTs, Weakly similar to The KIAA0134 gene product is related to human RNA helicase A. [H.sapiens]//1.6e-30:369:70//Hs.114623:AI204280
F-NT2RM4000086
F-NT2RM4000104//Homo sapiens chromosome 16 zinc finger protein ZNF210 (ZNF210) mRNA, complete cds//1.3e-24:345:69//Hs.13128:AF060865
F-NT2RM4000139
F-NT2RM4000155
F-NT2RM4000156//ESTs//5.9e-73:345:100//Hs.155958:AA573632
F-NT2RM4000167//Homo sapiens kinesin family member protein KIF3A mRNA, complete cds//9.8e-30:676:61//Hs.159228:AF041853
F-NT2RM4000169//ESTs//2.0e-103:483:99//Hs.43729:AA497044
F-NT2RM4000191//TRICHOHYALIN//0.011:324:60//Hs.82276:L09190
F-NT2RM4000197//ESTs//1.5e-48:311:88//Hs.136144:W27744
F-NT2RM4000199//ESTs, Weakly similar to !!!! ALU SUBFAMILY J WARNING ENTRY !!!! [H.sapiens]//0.13:322:61//Hs.145088:AI221147
F-NT2RM4000200
F-NT2RM4000202//Homo sapiens mRNA for KIAA0288 gene, complete cds//0.0027:424:60//HS.91400:AB006626
F-NT2RM4000210//Homo sapiens mRNA for KIAA0712 protein, complete cds//4.4e-184:856:98//Hs.111138:AB018255
F-NT2RM4000215//SET translocation (myeloid leukemia-associated)//0.0013:358:60//Hs.75055:M93651
F-NT2RM4000229//Homo sapiens mRNA for KIAA0722 protein, complete cds//0.65:572:60//Hs.47061:AF045458
F-NT2RM4000233//ESTs//2.0e-37:269:85//Hs.148873:T33582
F-NT2RM4000244//EST//0.83:319:57//Hs.162412:AA573439
F-NT2RM4000251//ESTs, Weakly similar to CUT1 PROTEIN [Schizosaccharomyces pombe]//1.1e-16:112:92//Hs.93841:AA442297
F-NT2RM4000265//Homo sapiens mRNA for alpha(1,2)fucosyltransferase, complete cds//1.8e-48:229:83//Hs.46328:D87942
F-NT2RM4000290//Human transducin-like enhancer protein (TLE3) mRNA, complete cds//2.5e-154:609:93//Hs.31305:M99438
F-NT2RM4000324//Homo sapiens hCPE-R mRNA for CPE-receptor, complete cds//0.070:460:59//Hs.5372:AB000712
F-NT2RM4000327//ESTs//0.019:269:60//Hs.153697:AI240707
F-NT2RM4000344//ESTs, Highly similar to YME1 PROTEIN [Saccharomyces cerevisiae]//2.7e-83:432:95//Hs.12796:W27884
F-NT2RM4000349//Human mRNA for KIAA0005 gene, complete cds//5.2e-53:666:68//Hs.155291:D13630
F-NT2RM4000354//ESTs, Weakly similar to lethal(2)denticleless [D.melanogaster]//0.0078:55:92//Hs.59075:M023761
F-NT2RM4000356//ESTs//1.0:225:60//Hs.161175:AI418425
F-NT2RM4000366//Homo sapiens mRNA for KIAA0642 protein, partial cds//5.3e-135:628:99//Hs.8152:AB014542
F-NT2RM4000368//ESTs//4.9e-13:323:63//Hs.143695:AA662745
F-NT2RM4000386//Human DNA sequence from clone 1052M9 on chromosome Xq25. Contains the SH2D1A gene for SH2 domain protein 1A, Duncan's disease (lymphoproliferative syndrome) (DSHP), part of a 60S Acidic Ribosomal protein 1 (RPLP1) LIKE gene and part of a mouse DOC4 LIKE gene. Contains ESTs and GSSs//2.0e-72:843:68//Hs.23796:AL022718
F-NT2RM4000395//Nitric oxide synthase 2A (inducible, hepatocytes)//0.63:166:65//Hs.946:X73029
F-NT2RM4000414//Homo sapiens XYLB mRNA for xylulokinase, complete cds//4.9e-17:114:94//Hs.137580:AB015046
F-NT2RM4000421
F-NT2RM4000425//Homo sapiens mRNA for KIAA0594 protein, partial cds//1.1e-42:432:74//Hs.154872:AB011166
F-NT2RM4000433//Colony stimulating factor 3 receptor (granulocyte)//0.023:543:58//Hs.2175:M59820
F-NT2RM4000457
F-NT2RM4000471//Human transcriptional corepressor hKAP1/TIF1B mRNA, complete cds//0.060:178:631/Hs.66369:U95040
F-NT2RM4000486//ESTs//9.2e-48:237:99//Hs.160685:AI280004
F-NT2RM4000496//ESTs//0.069:252:61//Hs.155958:AA573632
F-NT2RM4000511//EST//0.92:191:58//Hs.61517:AA028915
F-NT2RM4000514
F-NT2RM4000515//ESTs//7.3e-93:450:98//Hs.120975:AA034409
F-NT2RM4000520//ESTs//0.13:183:65//Hs.144828:AI221305
F-NT2RM4000531//ESTs, Highly similar to ZINC FINGER PROTEIN MLZ-4 [Mus musculus]//1.8e-153:756:96//Hs.125870:AI364967
F-NT2RM4000532//ESTs//7.7e-43:388:78//Hs.105665:H78987
F-NT2RM4000534
F-NT2RM4000585
F-NT2RM4000590//Homo sapiens mRNA for KIAA0469 protein, complete cds//1.2e-19:593:62//Hs.7764:AB007938
F-NT2RM4000595//ESTs, Highly similar to HYPOTHETICAL 54.9 KD PROTEIN C02F5.7 IN CHROMOSOME III [Caenorhabditis elegans]//3.1e-104:532:96//Hs.6092:T75227
F-NT2RM4000603//Human mRNA for KIAA0392 gene, partial cds//1.7e-15:305:68//Hs.40100:AB002390
F-NT2RM4000611//EST//0.76:268:58//Hs.150031:AI292068
F-NT2RM4000616
F-NT2RM4000674
F-NT2RM4000689
F-NT2RM4000698//Apolipoprotein E//1.0:290:59//Hs.76260:M12529
F-NT2RM4000700
F-NT2RM4000712//Homo sapiens ubiquitin hydrolyzing enzyme I (UBH1) mRNA, partial cds//3.5e-91:744:77//Hs.42400:AF022789
F-NT2RM4000717//ESTs, Highly similar to BONE MORPHOGENETIC PROTEIN 1 PRECURSOR [Mus musculus]//2.6e-163:771:97//Hs.6823:W18181
F-NT2RM4000733//PUTATIVE TACHYKININ RECEPTOR//0.70:257:60//Hs.957:M84605
F-NT2RM4000734//Homo sapiens mRNA for KIAA0760 protein, partial cds//1.2e-159:743:98//Hs.137168:AB018303
F-NT2RM4000741
F-NT2RM4000751//ESTs, Highly similar to ZINC FINGER PROTEIN MLZ-4 [Mus musculus]//1.1e-75:388:96//Hs.112361:R99396
F-NT2RM4000764//ESTs//3.8e-104:539:95//Hs.24739:H67815
F-NT2RM4000778//ESTs//1.5e-85:419:97//Hs.99838:AA204731
F-NT2RM4000779//Homo sapiens mRNA for KIAA0451 protein, complete cds//1.8e-173:810:98//Hs.18586:AB007920
F-NT2RM4000787//EST//0.011:182:65//Hs.159928:AA969186
F-NT2RM4000790//Homo sapiens chromosome 19, cosmid R27216//4.5e-156:736:98//Hs.25817:AC005306
F-NT2RM4000795//ESTs, Highly Similar to LIVER CARBOXYLESTERASE PRECURSOR [Homo sapiens]//6.7e-19:160:80//Hs.124902:AI337820
F-NT2RM4000796//Human K+ channel subunit gene, complete cds//0.96:292:62//Hs.124212:M64676
F-NT2RM4000798//ESTs//1.9e-34:271:82//Hs.128203:AA972301
F-NT2RM4000813//Homo sapiens snRNA activating protein complex 190kD subunit (SNAP190) mRNA, complete cds//0.052:238:64//Hs:113265:AF032387
F-NT2RM4000820//ESTs//0.053:274:61//Hs.23748:H16568
F-NT2RM4000833
F-NT2RM4000848//Human mRNA for KIAA0324 gene, partial cds//0.97:374:61//Hs.7841:AB002322
F-NT2RM4000852//EST//1.0:222:60//Hs.120354:AA718934
F-NT2RM4000855//ESTs, Highly similar to RAS-RELATED C3 BOTULINUM TOXIN SUBSTRATE 2 [Homo sapiens]//4.4e-29:164:95//Hs.115095:AI392943
F-NT2RM4000887
F-NT2RM4000895//Homo sapiens HuUAP1 mRNA for UDP-N-acetylglucosamine pyrophosphorylase, complete cds//6.8e-22:407:64//Hs.21293:AB011004
F-NT2RM4000950
F-NT2RM4000971//ESTs//3.6e-27:142:100//Hs.130912:AI014546
F-NT2RM4000979//Homo sapiens KIAA0415 mRNA, complete cds//3.7e-63:571:77//Hs.7289:AB007875
F-NT2RM4000996//Zinc finger protein 3 (A8-51)//8.7e-34:381:67//Hs.2481:X78926
F-NT2RM4001002//Homo sapiens mRNA for KIAA0729 protein, partial cds//1.6e-171:803:98//Hs.19542:AB018272
F-NT2RM4001016//Homo sapiens mRNA for KIAA0639 protein, partial cds//1.1e-126:584:99//Hs.15711:AB014539
F-NT2RM4001032//Homo sapiens mRNA for KIAA0711 protein, complete cds//4.8e-05:469:58//Hs.5333:AB018254
F-NT2RM4001047//ESTs, Moderately similar to MO25 PROTEIN [M.musculus]//7.0e-56:340:92//Hs.87310:AI247543
F-NT2RM4001054//HIGH AFFINITY IMMUNOGLOBULIN GAMMA FC RECEPTOR I "A FORM" PRECURSOR//0.79:142:69//Hs.77424:M63835
F-NT2RM4001084
F-NT2RM4001092//Human mRNA for KIAA0050 gene, complete cds//0.045:235:62//Hs.108947:D30758
F-NT2RM4001116
F-NT2RM4001140//Human engrailed protein (EN2) gene, 5' end//0.00029:225:61//Hs.134989:L12701
F-NT2RM4001151//ESTs//1.1e-07:190:65//Hs.151691:AA443730
F-NT2RM4001155//ESTs//2.2e-12:181:74//Hs.128826:AI004145
F-NT2RM4001160//EST//0.83:166:61//Hs.117051:AA677351
F-NT2RM4001187
F-NT2RM4001191//ESTs//1.3e-42:248:93//Hs.13475:R18220
F-NT2RM4001200//Zinc finger protein 10 (KOX 1)//4.0e-68:799:69//Hs.2479:X78933
F-NT2RM4001203//Homo sapiens rab3-GAP regulatory domain mRNA, complete cds//1.4e-153:707:99//Hs.14934:AF004828
F-NT2RM4001204//ESTs, Moderately similar to HYPOTHETICAL 59.1 KD PROTEIN ZK637.1 IN CHROMOSOME III [Caenorhabditis elegans]//0.19:291:62//Hs.31582:AA877205
F-NT2RM4001217//Homo sapiens nuclear matrix protein NRP/B (NRPB) mRNA, complete cds//7.0e-63:715:70//Hs.104925:AF059611
F-NT2RM4001256//ESTs, Weakly similar to probable CBP3 protein homolog [C.elegans]//1.1e-67:208:96//Hs.26676:AA033997
F-NT2RM4001258//Homo sapiens mRNA for KIAA0481 protein, complete cds//0.0019:435:59//Hs.6360:AB007950
F-NT2RM4001309//Human Chromosome 16 BAC clone CIT987SK-254P9//0.019:356:59//Hs.26971:AC003003
F-NT2RM4001313//H.sapiens mRNA for phosphatidylinositol 3-kinase//8.0e-79:474:89//Hs.32971:Z46973
F-NT2RM4001316//ESTs//1.2e-14:126:84//Hs.154344:AA258335
F-NT2RM4001320//Human mRNA for Neuroblastoma, complete cds//3.6e-43:642:66//Hs.87435:D89016
F-NT2RM4001340//EST//0.40:135:70//Hs.161198:AI418988
F-NT2RM4001344//ESTs, Highly similar to HYPOTHETICAL GTP-BINDING PROTEIN IN PMI40-PAC2 INTERGENIC REGION [Saccharomyces cerevisiae]//0.0096:284:58//Hs.120997:R56714
F-NT2RM4001347//ESTs, Weakly similar to weakly similar to ANK repeat region of Fowlpox virus BamHI-orf7 protein [C.elegans]//3.7e-52:252:100//Hs.15301:AA167818
F-NT2RM4001371//EST//0.52:262:59//Hs.145991:AI277656
F-NT2RM4001382//Homo sapiens RanBP7/importin 7 mRNA, complete cds//7.2e-169:790:98//Hs.5151:AF098799
F-NT2RM4001384
F-NT2RM4001410//ESTs//1.1e-47:290:91//Hs.72447:AA160575
F-NT2RM4001411//Homo sapiens mRNA for APS, complete cds//2.5e-23:475:64//Hs.105052:AB000520
F-NT2RM4001412
F-NT2RM4001414//ESTs, Moderately similar to 18547_1 [H.sapiens]//5.2e-18:133:87//Hs.28209:AI073817
F-NT2RM4001437//Human mRNA for KIAA0118 gene, partial cds//2.5e-42:611:70//Hs.154326:D42087
F-NT2RM4001444
F-NT2RM4001454//ESTs//3.9e-31:169:96//Hs.117982:AA644658
F-NT2RM4001455//ESTs//0.0054:48:100//Hs.14920:AA910914
F-NT2RM4001483//ESTs, Weakly similar to ZINC FINGER PROTEIN ZFP-36 [H.sapiens]//1.1e-71:313:99//Hs.163754:AA587784
F-NT2RM4001489//Homo sapiens mRNA for KIAA0685 protein, complete cds//3.9e-157:724:99//Hs.153121:AB014585
F-NT2RM4001519//ESTs//0.66:264:59//Hs.139891:AA553619
F-NT2RM4001522//ESTs, Weakly similar to D9481.12 gene product [S.cerevisiae]//1.3e-114:536:99//Hs.88820:AA456247
F-NT2RM4001557
F-NT2RM4001565//ESTs//1.7e-107:509:99//Hs.146139:AA731487
F-NT2RM4001566//Human phosphatidylinositol 3-kinase catalytic subunit p110delta mRNA, complete cds//1.0:255:60//Hs.14207:U86453
F-NT2RM4001569//ESTs//1.4e-86:417:98//Hs.153044:AI198859
F-NT2RM4001582
F-NT2RM4001592//EST//0.61:142:64//Hs.162900:AA664566
F-NT2RM4001594//Homo sapiens mRNA for KIAA0522 protein, partial cds//0.0072:484:60//Hs.129892:AB011094
F-NT2RM4001597//ESTs, Moderately similar to red-1 [M.musculus]//2.3e-72:387:95//Hs.114722:AA448077
F-NT2RM4001605//Homo sapiens mRNA for KIAA0791 protein, complete cds//1.1e-163:750:99//Hs.23255:AB018334
F-NT2RM4001611//ESTs, Weakly similar to F25H9.6 [C.elegans]//8.6e-05:91:79//Hs.24647:W19739
F-NT2RM4001629//ESTs, Moderately similar to 55 KD ERYTHROCYTE MEMBRANE PROTEIN [Homo sapiens]//0.0042:153:68//Hs.114832:AI147946
F-NT2RM4001650//Human mRNA for KIAA0341 gene, partial cds//0.95:328:60//Hs.101761:AB002339
F-NT2RM4001662//Human mRNA for KIAA0322 gene, partial cds//8.3e-83:449:93//Hs.153685:AB002320
F-NT2RM4001666//ESTs//2.1e-11:78:96//Hs.152446:AA555323
F-NT2RM4001682//EST//0.027:145:70//Hs.133253:AI052638
F-NT2RM4001710//ESTs//0.098:140:62//Hs.5796:AA767384
F-NT2RM4001714//Human mRNA for KIAA0202 gene, partial cds//2.2e-86:748:74//Hs.80712:D86957
F-NT2RM4001715//ESTs//1.3e-104:490:99//Hs.127336:AI332905
F-NT2RM4001731//Human involucrin mRNA//0.23:432:59//Hs.157091:M13903
F-NT2RM4001741//Human mRNA for KIAA0320 gene, partial cds//6.9e-80:737:73//Hs.150443:AB002318
F-NT2RM4001746//H.sapiens NF-H gene, exon 1 (and joined CDS)//2.1e-07:418:61//Hs.75735:X15306
F-NT2RM4001754//ESTs, Weakly similar to RETROVIRUS-RELATED POL POLYPROTEIN [Mus musculus]//2.0e-27:205:83//Hs.110601:AA206719
F-NT2RM4001758//H.sapiens mRNA for serine/threonine protein kinase EMK//2.1e-86:729:75//Hs.157199:X97630
F-NT2RM4001776//Homo sapiens mRNA for KIAA0727 protein, partial cds//7.4e-175:803:99//Hs.39871:AB018270
F-NT2RM4001783//ESTs, Weakly similar to T12D8.i [C.elegans]//3.1e-71:376:95//Hs.108396:AA160677
F-NT2RM4001810//Homo sapiens centrosomal Nek2-associated protein 1 (C-NAP1) mRNA, complete cds//0.99:446:58//Hs.27910:AF049105
F-NT2RM4001813//Homo sapiens clone 24820 mRNA sequence//6.6e-14:249:70//Hs.146312:AF070547
F-NT2RM4001819//Cell division cycle 2-like 1 (PITSLRE proteins)//1.4e-35:195:95//Hs.963:M37712
F-NT2RM4001823//ESTs, Weakly similar to ZINC FINGER PROTEIN 91 [H.sapiens]//2.3e-40:252:90//Hs.119294:AI379442
F-NT2RM4001828//Zinc fmger protein 157 (HZF22)//1.8e-75:688:72//Hs.89897:U28687
F-NT2RM4001836//NUCLEOBINDIN PRECURSOR//0.0022:588:59//Hs.953 :M96824
F-NT2RM4001841//ESTs//0.86:156:67//Hs.146276:AI214204
F-NT2RM4001842//ESTs//0.20:191:62//Hs.107657:AA126814
F-NT2RM4001856
F-NT2RM4001858//Human putative cerebral cortex transcriptional regulator T-Brain-1 (Tbr-1) mRNA, complete cds//8.0e-10:244:66//Hs.22138:U49250
F-NT2RM4001865//Homo sapiens mRNA for atopy related autoantigen CALC//2.3e-150:704:98//Hs.61628:Y17711
F-NT2RM4001876//Human mRNA for KIAA0231 gene, partial cds//9.1e-44:621:66//Hs.7938:D86984
F-NT2RM4001880
F-NT2RM4001905//ESTs//7.5e-11:137:75//Hs.86950:AI204212
F-NT2RM4001922//ESTs//2.5e-51:291:93//Hs.26660:AI312633
F-NT2RM4001930//Homo sapiens mRNA for putative glucosyltransferase, partial cds//0.98:359:57//Hs.155356:AJ224875
F-NT2RM4001938
F-NT2RM4001940//Homo sapiens timeless homolog mRNA, complete cds//3.6e-172:808:98//Hs.118631:AF098162
F-NT2RM4001953//Human mRNA for KIAA0118 gene, partial cds//5.0e-54:362:83//Hs.154326:D42087
F-NT2RM4001965//ESTs, Weakly similar to KIAA0157 gene product is novel. [H.sapiens]//1.8e-65:337:96//Hs.130135:AA905493
F-NT2RM4001969//ESTs//0.00024:261:63//Hs.157579:AI312862
F-NT2RM4001979//Homo sapiens mRNA for KIAA0798 protein, complete cds//3.2e-63:527:76//Hs.159277:AB018341
F-NT2RM4001984//EST//7.1e-05:235:61//Hs.105444:AA508082
F-NT2RM4001987//Homo sapiens mRNA for KIAA0467 protein, partial cds//0.73:181:65//Hs.11147:AB007936
F-NT2RM4002013//ESTs//0.97:185:63//Hs.103345:AI302271
F-NT2RM4002018//ESTs//2.5e-76:398:94//Hs.119544:T95601
F-NT2RM4002034
F-NT2RM4002044//ESTs//9.6e-83:410:97//Hs.128162:AA815048
F-NT2RM4002054//EST//8.5e-12:176:71//Hs.137181:R56912
F-NT2RM4002055//Homo sapiens mRNA for KIAA0640 protein, partial cds//3.3e-173:803:98//Hs.153026:AB014540
F-NT2RM4002062//ESTs, Weakly similar to ASPARTYL-TRNA SYNTHETASE [Thermus aquaticus thermophilus]//7.0e-94:396:94//Hs.59346:AI126802
F-NT2RM4002063
F-NT2RM4002066//Homo sapiens OPA-containing protein mRNA, complete cds//1.1e-74:889:69//Hs.85313:AF071309
F-NT2RM4002067//ESTs//2.3e-34:455:69//Hs.118273:AA626040
F-NT2RM4002073//Insulin-like growth factor binding protein 2//3.2e-10:470:61//Hs.162:X16302
F-NT2RM4002075//Homo sapiens actin binding protein MAYVEN mRNA, complete cds//2.9e-24:588:61//Hs.122967:AF059569
F-NT2RM4002093//Polypyrimidine tract binding protein (hnRNP I) {alternative products}//9.2e-34:532:65//Hs.146459:X66975
F-NT2RM4002109//Homo sapiens mitotic centromere-associated kinesin mRNA, complete cds//0.99:408:62//Hs.69360:U63743
F-NT2RM4002128//Homo sapiens mRNA for KIAA0642 protein, partial cds//0.93:202:63//Hs.8152:AB014542
F-NT2RM4002140//Human p300 protein mRNA, complete cds//0.99:320:59//Hs.25272:U01877
F-NT2RM4002145//CARBOXYPEPTIDASE N 83 KD CHAIN//2.7e-06:388:59//Hs.73858:J05158
F-NT2RM4002146//ESTs, Highly similar to similar to mago nashi [H.sapiens]//1.6e-135:646:97//Hs.104650:AI037879
F-NT2RM4002161//Homo sapiens laforin (EPM2A) mRNA, partial cds//1.4e-150:763:95//Hs.22464:AF084535
F-NT2RM4002174
F-NT2RM4002189//Mucin 2, intestinal/tracheal//0.087:298:61//Hs.315:L21998
F-NT2RM4002194//Human semaphorin III family homolog mRNA, complete cds//7.3e-11:454:60//Hs.32981:U38276
F-NT2RM4002205//EST//2.6e-21:270:71//Hs.120013:AA707454
F-NT2RM4002213//Homo sapiens mRNA for KIAA0610 protein, partial cds//0.52:313:61//Hs.118087:AB011182
F-NT2RM4002226//ESTs, Highly similar to GTPASE ACTIVATING PROTEIN ROTUND [Drosophila melanogaster]//8.4e-125:588:98//Hs.23900:U82984
F-NT2RM4002251//ESTs//1.0:77:74//Hs.155135:AA910966
F-NT2RM4002256//ESTs//7.5e-28:358:74//Hs.13356:AI205764
F-NT2RM4002266//Human kinase Myt1 (Myt1) mRNA, complete cds//0.73:502:57//Hs.77783:AF014118
F-NT2RM4002278//EST//0.33:138:63//Hs.144096:AI032180
F-NT2RM4002281
F-NT2RM4002287//ESTs//0.00037:55:98//Hs.11134:T62979
F-NT2RM4002294//Human mRNA for KIAA0281 gene, complete cds//6.7e-50:511:72//Hs.31463:D87457
F-NT2RM4002301
F-NT2RM4002323//ESTs//3.6e-09:105:87//Hs.131737:AI343331
F-NT2RM4002339
F-NT2RM4002344//EST//0.16:166:64//Hs.128600:AA906454
F-NT2RM4002373//Homo sapiens mRNA for KIAA0649 protein, complete cds//9.1e-151:708:98//Hs.26163:AB014549
F-NT2RM4002374//Homo sapiens mRNA for KIAA0720 protein, partial cds//0.0040:303:63//Hs.23741:AB018263
F-NT2RM4002383//ESTs//8.0e-16:153:78//Hs.155243:N70293
F-NT2RM4002390
F-NT2RM4002398
F-NT2RM4002409
F-NT2RM4002438//ESTs, Weakly similar to probable CBP3 protein homolog [C.elegans]//1.1e-55:282:96//Hs.26676:AA033997
F-NT2RM4002446//Homo sapiens clone 24574 mRNA sequence//0.59:339:60//Hs.18686:AF052151
F-NT2RM4002452
F-NT2RM4002457//Homo sapiens mRNA for epiregulin, complete cds//3.2e-25:228:81//Hs.115263:D30783
F-NT2RM4002460//EST//1.0:142:65//Hs.145370:AI252780
F-NT2RM4002479//Homo sapiens RNA helicase-related protein mRNA, complete cds//8.9e-165:777:98//Hs.8765:AF083255
F-NT2RM4002482//Homo sapiens mRNA for KIAA0691 protein, complete cds//7.3e-95:464:97//Hs.94781:AB014591
F-NT2RM4002493
F-NT2RM4002499//ESTs//1.3e-44:653:67//Hs.23790:N99347
F-NT2RM4002504//Small inducible cytokine A5 (RANTES)//4.3e-30:225:83//Hs.155464:AF088219
F-NT2RM4002527//Human pre-B cell enhancing factor (PBEF) mRNA, complete cds//0.99:290:60//Hs.154968:U02020
F-NT2RM4002532//Human mRNA for KIAA0238 gene, partial cds//1.0:232:61//Hs.82042:D87075
F-NT2RM4002534//Homo sapiens angiotensin/vasopressin receptor AII/AVP mRNA, complete cds//1.0:100:70//Hs.159483:AF054176
F-NT2RM4002558//Homo sapiens amphiphysin II mRNA, complete cds//0.17:393:61//Hs.6619:U84004
F-NT2RM4002565//Homo sapiens mRNA for Asparaginyl tRNA Synthetase, complete cds//1.0:226:60//Hs.84043:D84273
F-NT2RM4002567//ESTs, Weakly similar to C17G10.1 [C.elegans]//3.3e-88:484:93//Hs.105837:AA536054
F-NT2RM4002571//ESTs, Weakly similar to UDP-GalNAc:polypeptide N-acetylgalactosaminyltransferase [H.sapiens]//0.059:121:70//Hs.155413:AA429394
F-NT2RM4002593//ESTs//1.0e-15:103:95//Hs.108920:W28151
F-NT2RM4002594//Homo sapiens 26S proteasome regulatory subunit (SUG2) mRNA, complete cds//1.0e-06:499:59//Hs.79357:D78275
F-NT2RM4002623//ESTs//1.2e-11:92:92//Hs.164046:T97402
F-NT2RP1000018//Homo sapiens mRNA for KIAA0687 protein, partial cds//2.0e-102:746:81//Hs.3628:AB014587
F-NT2RP1000035//Homo sapiens mRNA for NS1-binding protein (NS1-BP)//3.7e-155:747:96//Hs.159597:AJ012449
F-NT2RP1000040//ESTs//1.3e-58:338:92//Hs.17534:H16907
F-NT2RP1000063//ESTs//0.0013:72:83//Hs.108196:W81647
F-NT2RP1000086//Human mRNA for KIAA0360 gene, partial cds//5.4e-185:548:91//Hs.79971:X98834
F-NT2RP1000101//Homo sapiens hook2 protein (HOOK2) mRNA, complete cds//0.33:247:61//Hs.30792:AF044924
F-NT2RP1000111
F-NT2RP1000112//TTK protein kinase//3.2e-40:324:81//Hs.2052:M86699
F-NT2RP1000124//ESTs//2.4e-42:268:89//Hs.146078:AI084025
F-NT2RP1000130//ESTs, Moderately similar to HEPATOMA-DERIVED GROWTH FACTOR [H.sapiens]//1.4e-71:382:94//Hs.127842:W38901
F-NT2RP1000163//Homo sapiens cell cycle progression 2 protein (CPR2) mRNA, complete cds//2.1e-06:77:90//Hs.3760:AF011792
F-NT2RP1000170//EST//0.68:130:63//Hs.146994:AI184430
F-NT2RP1000174//Homo sapiens clone 24432 mRNA sequence//8.3e-140:679:97//Hs.78019:AF070535
F-NT2RP1000191//ESTs//1.3e-71:405:93//Hs.24054:N46499
F-NT2RP1000202//H.sapiens mRNA for cytokine inducible nuclear protein//2.0e-05:591:58//Hs.74019:X83703
F-NT2RP1000243
F-NT2RP1000259
F-NT2RP1000272//Homo sapiens TLS-associated protein TASR-2 mRNA, complete cds//5.4e-109:528:97//Hs.4214:AF067730
F-NT2RP1000324//ESTs//3.4e-98:499:96//Hs.42530:N41661
F-NT2RP1000326//Homo sapiens metaxin 2 (MTX2) mRNA, nuclear gene encoding mitochondrial protein, complete cds//1.3e-148:693:98//Hs.31584:AF053551
F-NT2RP1000333//Homo sapiens monocyte/macrophage Ig-related receptor MIR-10 (MIR cl-10) mRNA, complete cds//0.28:328:60//Hs.22405:AF004231
F-NT2RP1000348//Human plectin (PLEC1) mRNA, complete cds//0.018:337:62//Hs.79706:U53204
F-NT2RP1000357
F-NT2RP1000358//DYNAMIN-1//0.96:273:59//Hs.126:L07807
F-NT2RP1000363//Homo sapiens mRNA for KIAA0638 protein, partial cds//3.2e-126:497:86//Hs.77864:AB014538
F-NT2RP1000376//Homo sapiens calcium-independent phospholipase A2 mRNA, complete cds//5.9e-178:877:96//Hs.120360:AF064594
F-NT2RP1000409//ESTs//5.4e-59:415:83//Hs.140578:AA828031
F-NT2RP1000413//Homo sapiens mRNA for KIAA0587 protein, complete cds//3.0e-179:710:98//Hs.21862:AB011159
F-NT2RP1000416//ESTs, Highly similar to BONE MORPHOGENETIC PROTEIN 1 PRECURSOR [Mus musculus]//7.3e-177:857:97//Hs.6823:W18181
F-NT2RP1000418//Homo sapiens calcium-activated potassium channel (KCNN3) mRNA, complete cds//0.46:222:60//Hs.89230:AF031815
F-NT2RP1000439//EST//0.98:339:56//Hs.137377:AA101603
F-NT2RP1000443//Human SLP-76 associated protein mRNA, complete cds//1.0:356:59//Hs.58435:AF001862
F-NT2RP1000460
F-NT2RP1000470//Human DNA from chromosome 19-specific cosmid R27090, genomic sequence//3.7e-134:665:96//Hs.143187:AC002985
F-NT2RP1000478//Human beta-tubulin class III isotype (beta-3) mRNA, complete cds//6.2e-57:440:80//Hs.159154:U47634
F-NT2RP1000481//ESTs//4.8e-21:154:87//Hs.17392:AA535102
F-NT2RP1000493
F-NT2RP1000513//ESTs//2.2e-71:409:91//Hs.121029:AA480977
F-NT2RP1000522//Homo sapiens clone DT1P1A11 mRNA, CAG repeat region//0.21:255:62//Hs.98834:U92992
F-NT2RP1000547//H.sapiens mRNA for transmembrane protein rnp24//1.9e-06:337:63//Hs.75914:X92098
F-NT2RP1000574//Homo sapiens homeobox protein MEIS2 (MEIS2) mRNA, partial cds//1.4e-82:295:92//Hs.104105:AF017418
F-NT2RP1000577//Human sialoprotein mRNA, complete cds//0.014:235:65//Hs.121552:J05213
F-NT2RP1000581//VON WILLEBRAND FACTOR PRECURSOR//1.6e-33:223:89//Hs.110802:X04385
F-NT2RP1000609//Homo sapiens chromosome 11, BAC CIT-HSP-311e8 (BC269730) containing the hFEN1 gene//2.2e-49:506:73//Hs.132898:AC004770
F-NT2RP1000629//Human clathrin assembly protein 50 (AP50) mRNA, complete cds//3.6e-19:556:62//Hs.152936:D63475
F-NT2RP1000630
F-NT2RP1000677//Human breast tumor autoantigen mRNA, complete sequence//2.4e-05:389:59//Hs.3844:U24576
F-NT2RP1000688//ESTs, Weakly similar to T06E6.d [C.elegans]//2.5e-43:232:95//Hs.3487:AA425553
F-NT2RP1000695//ESTs, Weakly similar to C27F2.7 gene product [C.elegans]//9.2e-53:312:90//Hs.7049:AI141736
F-NT2RP1000701//Myogenic factor 3//0.81:186:63//Hs.2834:AF027148
F-NT2RP1000721//Homo sapiens mRNA for repressor protein, partial cds//4.0e-33:278:78//Hs.58167:D30612
F-NT2RP1000730//ESTs, Weakly similar to putative p150 [H.sapiens]//6.2e-40:297:84//Hs.18122:AI338045
F-NT2RP1000733//G1 to S phase transition 1//1.4e-31:286:78//Hs.2707:X17644
F-NT2RP1000738//Homo sapiens Wolf-Hirschhorn syndrome candidate 2 protein (WHSC2) mRNA, complete cds//2.6e-123:604:96//Hs.21771:AF101434
F-NT2RP1000746
F-NT2RP1000767
F-NT2RP1000782//Human globin gene//3.6e-21:140:91//Hs.100090:M69023
F-NT2RP1000796//H.sapiens mRNA for ROX protein//0.17:404:57//Hs.25497:X96401
F-NT2RP1000825//Human DNA sequence from PAC 127B20 on chromosome 22q11.2-qter, contains gene for GTPase-activating protein similar to rhoGAP protein. ribosomal protein L6 pseudogene, ESTs and CA repeat//2.7e-23:147:91//Hs.102336:Z83838
F-NT2RP1000833//Homo sapiens cGMP phosphodiesterase A1 (PDE9A) mRNA, complete cds//5.4e-143:424:96//Hs.18953:AF067223
F-NT2RP1000834//ESTs//0.18:280:60//Hs.157215:AI332903
F-NT2RP1000836//EST//0.60:103:66//Hs.145708:AI267990
F-NT2RP1000846//EST//1.2e-15:322:65//Hs.149925:AI288838
F-NT2RP1000851//ESTs//6.1e-96:459:98//Hs.121586:AA423875
F-NT2RP1000856//Human globin gene//6.7e-22:140:91//Hs.100090:M69023
F-NT2RP1000860//Homo sapiens KL04P mRNA, complete cds//2.2e-107:551:95//Hs.125156:AF064094
F-NT2RP1000902//EST//1.8e-28:218:85//Hs.145258:AI218683
F-NT2RP1000915//ESTs//8.8e-11:102:81//Hs.163740:AI248847
F-NT2RP1000916//ESTs, Weakly similar to coded for by C. elegans cDNA cm04e9 [C.elegans]//2.2e-27:159:94//Hs.122153:AA780270
F-NT2RP1000943//Human hSIAH2 mRNA, complete cds//0.45:130:68//Hs.20191:U76248
F-NT2RP1000944//EST//0.99:116:63//Hs.116633:AA668400
F-NT2RP1000947//Human E2 ubiquitin conjugating enzyme UbcH5B (UBCH5B) mRNA, complete cds//2.7e-26:185:87//Hs.108332:U39317
F-NT2RP1000954//Homo sapiens BACH1 mRNA, complete cds//0.81:329:56//Hs.154276:AB002803
F-NT2RP1000958//ESTs//1.3e-20:129:92//Hs.163740:AI248847
F-NT2RP1000959//Ribosomal protein, large, P0//0.36:76:73//Hs.73742:M17885
F-NT2RP1000966//NUCLEOLIN//1.2e-72:353:98//Hs.79110:M60858
F-NT2RP1000980//ESTs//1.6e-109:555:96//Hs.84429:N28866
F-NT2RP1000988//Human chromosome 3p21.1 gene sequence//2.6e-73:665:80//Hs.82837:L13435
F-NT2RP1001011
F-NT2RP1001013//ESTs//3.4e-40:393:74//Hs.120206:AI089163
F-NT2RP1001014
F-NT2RP1001033//Tubulin, gamma polypeptide//0.00041:313:59//Hs.150785:M61764
F-NT2RP1001073//Glucocorticoid receptor//1.0:204:61//Hs.75772:M10901
F-NT2RP1001079//ESTs//1.0:174:62//Hs.158209:AI360531
F-NT2RP1001080//Homo sapiens forkhead protein (FKHRL1) mRNA, complete cds//0.57:215:64//Hs.14845:AF032886
F-NT2RP1001113//ESTs, Weakly similar to coded for by C. elegans cDNA CEESB82F [C.elegans]//1.4e-65:293:95//Hs.32751:H38087
F-NT2RP1001173
F-NT2RP1001177//Homo sapiens histone macroH2A1.2 mRNA, complete cds//6.1e-26:259:74//Hs.75258:AF054174
F-NT2RP1001185//EST//1.4e-27:266:77//Hs.122245:AA781524
F-NT2RP1001199//ESTs//0.97:75:73//Hs.131498:AI022150
F-NT2RP1001247//Human endometrial bleeding associated factor mRNA, complete cds//1.6e-19:120:95//Hs.25195:U81523
F-NT2RP1001248//ESTs//3.0e-21:143:93//Hs.157243:AI337094
F-NT2RP1001253//PUTATIVE GLUCOSAMINE-6-PHOSPHATE ISOMERASE//1.2e-89:344:93//Hs.3090:AJ002231
F-NT2RP1001286//H.sapiens mRNA for adenosine triphosphatase, calcium//0.026:392:57//Hs.5541:Y15724
F-NT2RP1001294
F-NT2RP1001302
F-NT2RP1001310//Homo sapiens creatine transporter mRNA, complete cds//3.6e-07:379:61//Hs.154503:U36341
F-NT2RP1001311//ESTs//9.5e-73:403:93//Hs.24739:H67815
F-NT2RP1001313//Homo sapiens chromosome 11, BAC CIT-HSP-311e8 (BC269730) containing the hFEN1 gene//3.1e-87:437:97//Hs.132898:AC004770
F-NT2RP1001361//ESTs, Highly similar to NADH-UBIQUINONE OXIDOREDUCTASE SUBUNIT B14.5B [Bos taurus]//6.8e-101:480:94//Hs.75017:AA166853
F-NT2RP1001385//EST//0.86:127:65//Hs.156304:AI336859
F-NT2RP1001395//Homo sapiens stannin mRNA, complete cds//0.75:355:58//Hs.76691:AF070673
F-NT2RP1001410//Thromboxane A2 receptor//1.0:157:63//Hs.89887:D38081
F-NT2RP1001424//ESTs//5.3e-20:118:95//Hs.159792:R60700
F-NT2RP1001432//ESTs//5.3e-20:118:95//Hs.159792:R60700
F-NT2RP1001449//Homo sapiens clone 24733 mRNA sequence//5.7e-86:422:97//Hs.21970:AF052149
F-NT2RP1001457//H.sapiens DAP-kinase mRNA//0.40:231:61//Hs.153924:X76104
F-NT2RP1001466
F-NT2RP1001475//ESTs//1.2e-98:495:97//Hs.14347:AA287742
F-NT2RP1001482
F-NT2RP1001494
F-NT2RP1001543//ESTs//1.2e-38:207:98//Hs.131063:AI016400
F-NT2RP1001546//Homo sapiens mRNA for DAP-1 beta, complete cds//0.00077:254:64//Hs.75814:AB000277
F-NT2RP1001569
F-NT2RP1001616//Homo sapiens Tax interaction protein 1 mRNA, partial cds//2.5e-41:496:74//Hs.12956:U90913
F-NT2RP1001665//ESTs//9.4e-58:311:96//Hs.127391:AA954420
F-NT2RP2000001//Homo sapiens clone 617 unknown mRNA, complete sequence//4.7e-137:685:96//Hs.93677:AF091081
F-NT2RP2000006//ESTs, Weakly similar to B0035.14 [C .elegans]//8.2e-47:300:89//Hs.6473:AA853955
F-NT2RP2000007//Human mRNA for KIAA0392 gene, partial cds//1.1e-15:241:68//Hs.40100:AB002390
F-NT2RP2000008//Human mRNA for KIAA0065 gene, partial cds//1.5e-29:526:66//Hs.70617:D31763
F-NT2RP2000027//ESTs, Highly similar to LINE-1 REVERSE TRANSCRIPTASE HOMOLOG [Homo sapiens]//2.0e-26:214:82//Hs.140385:AA773359
F-NT2RP2000032//ESTs//0.91:368:57//Hs.131209:AI038867
F-NT2RP2000040//Homo sapiens mRNA for KIAA0747 protein, partial cds//6.1e-78:383:97//Hs.8309:AB018290
F-NT2RP2000045//Homo sapiens tumorous imaginal discs protein Tid56 homolog (TID1) mRNA, complete cds//7.8e-97:467:97//Hs.6216:AF061749
F-NT2RP2000054//HOMEOBOX/POU DOMAIN PROTEIN RDC-1//1.0:110:70//Hs.74095:L20433
F-NT2RP2000056//Human HPTP epsilon mRNA for protein tyrosine phosphatase epsilon//1.2e-27:146:100//Hs.155991:X54134
F-NT2RP2000067//Human DNA sequence from clone 1052M9 on chromosome Xq25. Contains the SH2D1A gene for SH2 domain protein 1A, Duncan's disease (lymphoproliferative syndrome) (DSHP), part of a 60S Acidic Ribosomal protein 1 (RPLP1) LIKE gene and part of a mouse DOC4 LIKE gene. Contains ESTs and GSSs//8.1e-41:767:61//Hs.23796:AL022718 F-NT2RP2000070//Homo sapiens chromosome 5, BAC clone 203o13 (LBNL H155), complete sequence//6.5e-08:344:58//Hs.159402:AC005609
F-NT2RP2000076//H.sapiens mRNA for TFIIAI/0.00023:356:62//Hs.121686:D14887
F-NT2RP2000077//Homo sapiens growth arrest specific 11 (GAS11) mRNA, complete cds//6.8e-79:278:97//Hs.54877:AF050078
F-NT2RP2000079//ESTs//1.2e-36:202:94//Hs.17606:AI279879
F-NT2RP2000088//Homo sapiens mRNA for KIAA0795 protein, partial cds//7.1e-160:752:98//Hs.22926:AB018338
F-NT2RP2000091
F-NT2RP2000097
F-NT2RP2000098//ESTs//0.086:92:69//Hs.159389:AI371963
F-NT2RP2000108//Human mRNA for KIAA0392 gene, partial cds//1.4e-18:200:77//Hs.40100:AB002390
F-NT2RP2000114//Homo sapiens mRNA for GM3 synthase, complete cds//1.6e-115:551:97//Hs.17706:AB018356
F-NT2RP2000120//ESTs, Weakly similar to HYPOTHETICAL 68.7 KD PROTEIN ZK757.1 IN CHROMOSOME IlI [C.elegans]//0.019:72:81//Hs.5268:W22670
F-NT2RP2000126//Homo sapiens chromodomain-helicase-DNA-binding protein mRNA, complete cds//1.4e-120:607:96//Hs.159273:AF054177
F-NT2RP2000133//Neuronal pentraxin II//0.00014:401:61//Hs.3281:U29195
F-NT2RP2000147//Human clathrin assembly protein 50 (AP50) mRNA, complete cds//2.2e-18:559:60//Hs.152936:D63475
F-NT2RP2000153//Homo sapiens splicing factor (CC1.3) mRNA, complete cds//0.33:85:70//Hs.256:L10910
F-NT2RP2000157//ESTs//0.53:75:81//Hs.24885:R49291
F-NT2RP2000161//ESTs//2.6e-06:89:84//Hs.21738:AI188190
F-NT2RP2000173
F-NT2RP2000175
F-NT2RP2000183//Homo sapiens mRNA for dihydropyrimidinase related protein 4, complete cds//0.0018:324:58//Hs.100058:AB006713
F-NT2RP2000195//ESTs, Weakly similar to C37E2.2 [C.elegans]//3.6e-37:233:90//Hs.56750:AI148761
F-NT2RP2000205//ESTs//5.6e-58:317:93//Hs.49559:AA401050
F-NT2RP2000208
F-NT2RP2000224//Homo sapiens hLRp105 mRNA for LDL receptor related protein 105, complete cds//0.0071:243:61//Hs.143641:AB009462
F-NT2RP2000232//EST//0.0087:187:62//Hs.151024:Z39990
F-NT2RP2000233//Homo sapiens Notch3 (NOTCH3) mRNA, complete cds//0.17:342:59//Hs.8546:U97669
F-NT2RP2000239//Human mRNA for KIAA0380 gene, complete cds//1.0:227:60//Hs.47822:AB002378
F-NT2RP2000248//EST//0.49:117:70//Hs.61016:AA019719
F-NT2RP2000257//Macrophage stimulating 1 (hepatocyte growth factor-like)//0.51:227:60//Hs.30223:X90846
F-NT2RP2000258//ESTs//3.1e-48:261:94//Hs.128230:AA972691
F-NT2RP2000270//ESTs//2.9e-38:357:75//Hs.140329:AA714011
F-NT2RP2000274//ESTs//1.1e-106:508:98//Hs.47646:AA307599
F-NT2RP2000283//EST//1.0:139:63//Hs.128256:AA972910
F-NT2RP2000288
F-NT2RP2000289
F-NT2RP2000297//Human repressor transcriptional factor (ZNF85) mRNA, complete cds//4.2e-60:744:70//Hs.37138:U35376
F-NT2RP2000298//ESTs//6.1e-46:322:85//Hs.159490:AI123467
F-NT2RP2000310//Human proline dehydrogenase/proline oxidase (PRODH) mRNA, complete cds//4.3e-13:140:80//Hs.58218:U82381
F-NT2RP2000327//ESTs//4.3e-18:108:98//Hs.126212:AI417006
F-NT2RP2000328//ESTs//6.3e-88:437:96//Hs.127336:AI332905
F-NT2RP2000329//GTP:AMP PHOSPHOTRANSFERASE MITOCHONDRIAL//6.6e-41:607:66//Hs.101642:X60673
F-NT2RP2000337//Homo sapiens neurocan (CSPG3) mRNA, complete cds//0.96:126:69//Hs.153706:AF026547
F-NT2RP2000346//Homo sapiens apoptosis associated protein (GADD34) mRNA, complete cds//1.2e-130:627:97//Hs.76556:U83981
F-NT2RP2000369//Homo sapiens mRNA for KIAA0630 protein, partial cds//0.56:464:57//Hs.12259:AB014530
F-NT2RP2000412//ESTs//1.0:214:60//Hs.91226:AA649047
F-NT2RP2000414//Homo sapiens HnRNP F protein mRNA, complete cds//1.6e-67:375:93//Hs.808:L28010
F-NT2RP2000420//ESTs, Moderately similar to zinc finger protein [H.sapiens]//3.9e-75:413:92//Hs.36779:AA626790
F-NT2RP2000422//Homo sapiens N-acetylglucosamine-phosphate mutase mRNA, complete cds//6.7e-128:609:96//Hs.5819:AF102265
F-NT2RP2000438//ESTs//1.3e-05:50:98//Hs.156532:AA913381
F-NT2RP2000448//EST//1.1e-24:136:98//Hs.160402:AI393918
F-NT2RP2000459//H.sapiens mRNA for imogen 38//1.9e-22:158:87//Hs.154655:Z68747
F-NT2RP2000498//ESTs//1.0e-17:181:79//Hs.155243:N70293
F-NT2RP2000503//ESTs//4.5e-41:205:100//Hs.62751:AA765702
F-NT2RP2000510
F-NT2RP2000516
F-NT2RP2000523//ESTs, Highly similar to APOLIPOPROTEIN B MRNA EDITING PROTEIN [Rattus norvegicus]//3.2e-15:167:75//Hs.10984:AA806768
F-NT2RP2000603//Homo sapiens mRNA for KIAA0572 protein, partial cds//5.6e-38:196:98//Hs.14409:AB011144
F-NT2RP2000617//Myosin, heavy polypeptide 6, cardiac muscle, alpha (cardiomyopathy, hypertrophic 1)//1.0:242:57//Hs.114001:Z20656
F-NT2RP2000634//Homo sapiens mRNA for KIAA0614 protein, partial cds//4.2e-151:732:97//Hs.7314:AB014514
F-NT2RP2000644//ESTs//0.035:276:60//Hs.43660:N33174
F-NT2RP2000656
F-NT2RP2000658//ESTs//0.032:281:59//Hs.124853:AA420602
F-NT2RP2000668
F-NT2RP2000678//ESTs//2.9e-16:310:65//Hs.126867:AI093453
F-NT2RP2000704//ESTs, Highly similar to PUTATIVE SERINE/THREONINE-PROTEIN KINASE C41C4.4 IN CHROMOSOME II PRECURSOR [Caenorhabditis elegans]//2.4e-31:233:78//Hs.114905:AA088442
F-NT2RP2000710
F-NT2RP2000715
F-NT2RP2000731
F-NT2RP2000758//EST//1.0e-14:199:71//Hs.162409:AA573242
F-NT2RP2000764//ESTs, Weakly similar to NIFS-LIKE 54.5 KD PROTEIN [Saccharomyces cerevisiae]//1.6e-74:445:89//Hs.21421:AA911739
F-NT2RP2000809//ESTs//1.2e-36:235:89//Hs.154580:N34101
F-NT2RP2000812//Homo sapiens pendrin (PDS) mRNA, complete cds//0.22:351:58//Hs.159275:AF030880
F-NT2RP2000814
F-NT2RP2000816//Homo sapiens mRNA for KIAA0610 protein, partial cds//1.0:311:61//Hs.118087:AB011182
F-NT2RP2000819
F-NT2RP2000841//Human mRNA for KIAA0294 gene, complete cds//3.4e-28:390:70//Hs.20695:AB002292
F-NT2RP2000842//Human lysophosphatidic acid receptor homolog mRNA, complete cds//9.5e-29:167:94//Hs.75794:U80811
F-NT2RP2000845//ESTs//1.0e-83:403:98//Hs.156828:AI336850
F-NT2RP2000863//ESTs, Highly similar to HYPOTHETICAL 36.7 KD PROTEIN C2F7.02C IN CHROMOSOME I [Schizosaccharomyces pombe]//6.4e-34:207:92//Hs.135235:AI081880
F-NT2RP2000880//Homo sapiens mRNA for KIAA0741 protein, complete cds//7.7e-142:732:94//Hs.3615:AB018284
F-NT2RP2000892//ESTs, Weakly similar to mitogen-activated kinase kinase kinase 5 [H.sapiens]//0.50:189:65//Hs.46146:AA418097
F-NT2RP2000931//MATRIN3//1.1e-130:610:98//Hs.78825:AB018266
F-NT2RP2000932//Homo sapiens BAC clone GS166A23 from 7p21//5.5e-66:326:97//Hs.15144:AC005014
F-NT2RP2000938//ESTs//1.8e-28:296:75//Hs.22822:H06408
F-NT2RP2000943//Homo sapiens mRNA for KIAA0755 protein, complete cds//1.9e-113:533:98//Hs.19822:AB018298
F-NT2RP2000965//ESTs//5.3e-59:328:94//Hs.35575:R96494
F-NT2RP2000970
F-NT2RP2000985//ESTs, Weakly similar to HYPOTHETICAL 96.8 KD PROTEIN IN SIS2-MTD1 INTERGENIC REGION [Saccharomyces cerevisiae]//7.3e-76:385:96//Hs.21875:AA243700
F-NT2RP2000987//ESTs//5.6e-11:177:72//Hs.15776:T91944
F-NT2RP2001036//ESTs//2.0e-55:352:88//Hs.122131:AA789292
F-NT2RP2001044//EST//0.069:267:60//Hs.102808:N67117
F-NT2RP2001056//Homo sapiens mRNA, chromosome 1 specific transcript KIAA0488//1.0e-145:696:97//Hs.67619:AB007957
F-NT2RP2001065
F-NT2RP2001070//Human mRNA for KIAA0315 gene, partial cds//1.0:310:60//Hs.3989:AB002313
F-NT2RP2001081
F-NT2RP2001094//ESTs//0.0071:262:64//Hs.128115:AI356560
F-NT2RP2001119//Small inducible cytokine A5 (RANTES)//2.2e-34:311:78//Hs.155464:AF088219
F-NT2RP2001127//Human mRNA for KIAA0234 gene, complete cds//3.5e-33:519:63//Hs.80358:U52191
F-NT2RP2001137//ESTs, Highly similar to RAB GDP DISSOCIATION INHIBITOR ALPHA [Bos taurus]//6.4e-34:201:91//Hs.118470:AI336362
F-NT2RP2001149//EST//3.9e-27:244:78//Hs.162236:AA551582
F-NT2RP2001168//ESTs//0.0023:216:62//Hs.134938:AI091361
F-NT2RP2001173//Homo sapiens mRNA for KIAA0480 protein, complete cds//7.4e-114:567:96//Hs.26247:AB007949
F-NT2RP2001174//H.sapiens ZNF81 gene//0.21:256:59//Hs.104020:X68011
F-NT2RP2001196
F-NT2RP2001218//ESTs//1.1e-65:337:96//Hs.115710:AA524598
F-NT2RP2001226//Guanylate cyclase 1, soluble, alpha 2//0.030:395:59//Hs.2685:Z50053
F-NT2RP2001233//Zinc finger protein 136 (clone pHZ-20)//4.4e-58:656:70//Hs.69740:U09367
F-NT2RP2001245//EST//0.018:228:62//Hs.116798:AA633813
F-NT2RP2001268//Homo sapiens mRNA for KIAA0810 protein, partial cds//8.1e-108:514:97//Hs.7531:AB018353
F-NT2RP2001277//EST//0.42:127:66//Hs.42834:N20277
F-NT2RP2001290//Homo sapiens alpha SNAP mRNA, complete cds//1.8e-62:527:76//Hs.75848:U39412
F-NT2RP2001295//ESTs//3.4e-29:90:100//Hs.123321:AA810287
F-NT2RP2001312//ESTs//1.0:121:61//Hs.160261:AI146387
F-NT2RP2001327//Human B12 protein mRNA, complete cds//1.9e-30:359:71//Hs.76090:M80783
F-NT2RP2001328//ESTs//5.2e-103:532:94//Hs.69476:AA628522
F-NT2RP2001347//ESTs//4.3e-28:217:82//Hs.31775:H41883
F-NT2RP2001366//ESTs, Weakly similar to ZK1058.5 [C.elegans]//1.8e-72:418:91//Hs.107039:W27244
F-NT2RP2001378
F-NT2RP2001381//ESTs//0.59:235:62//Hs.118569:AI377558
F-NT2RP2001392//Homo sapiens chromosome 5, BAC clone 203o13 (LBNL H155), complete sequence//0.28:225:62//Hs.159402:AC005609
F-NT2RP2001394//ESTs//8.3e-22:133:78//Hs.109655:AI189767
F-NT2RP2001397//ESTs//0.090:265:60//Hs.152775:AA633088
F-NT2RP2001420
F-NT2RP2001423//ESTs, Weakly similar to hypothetical protein [H.sapiens]//0.030:443:59//Hs.140506:AA308018
F-NT2RP2001427//EST//1.9e-19:174:79//Hs.132635:AI032875
F-NT2RP2001436//EST//0.16:132:66//Hs.128265:AA972966
F-NT2RP2001440//Tyrosine 3-monooxygenase/tryptophan 5-monooxygenase activation protein, eta polypeptide//9.8e-56:603:72//Hs.75544:Z82248
F-NT2RP2001445//ESTs//2.2e-26:193:86//Hs.128610:AA504218
F-NT2RP2001449
F-NT2RP2001450
F-NT2RP2001467
F-NT2RP2001506
F-NT2RP2001511//ESTs, Weakly similar to F48F7.1 [C.elegans]//3.2e-83:409:98//Hs.156161:AI333779
F-NT2RP2001520//Homo sapiens mRNA for mitochondrial carrier protein ARALAR1//6.4e-138:657:97//Hs.4277:Y14494
F-NT2RP2001526//EST//1.0:180:61//Hs.136311:AA437134
F-NT2RP2001536//Homo sapiens X-ray repair cross-complementing protein 3 (XRCC3) mRNA, complete cds//5.2e-105:384:94//Hs.99742:AF035586
F-NT2RP2001560
F-NT2RP2001569//Homo sapiens mRNA, chromosome 1 specific transcript KIAA0488//1.4e-124:590:98//Hs.67619:AB007957
F-NT2RP2001576//Erythrocyte membrane protein band 4.9 (dematin)//0.046:521:60//Hs.75936:U28389
F-NT2RP2001581//EST//1.0:28:96//Hs.148002:AI264876
F-NT2RP2001597//Casein kinase 2, alpha prime polypeptide//0.069:165:65//Hs.82201:M55268
F-NT2RP2001601//Homo sapiens mRNA for KIAA0797 protein, partial cds//2.3e-138:647:98//Hs.27197:AB018340
F-NT2RP2001613
F-NT2RP2001628//ESTs//4.9e-45:238:96//Hs.135222:AI082229
F-NT2RP2001634//Homo sapiens alpha-catenin related protein (ACRP) mRNA, complete cds//4.9e-124:604:96//Hs.58488:U97067
F-NT2RP2001660//Homo sapiens putative 13 S Golgi transport complex 90kD subunit brain-specific isoform mRNA, complete cds//1.3e-145:687:97//Hs.159558:AF058718
F-NT2RP2001663//Enolase 1, (alpha)//4.2e-38:372:74//Hs.675:M14328
F-NT2RP2001675//X-LINKED HELICASE II//0.040:454:58//Hs.96264:U72936
F-NT2RP2001677//Homo sapiens mRNA for KIAA0771 protein, partial cds//0.028:285:63//Hs.6162:AB018314
F-NT2RP2001678//Homo sapiens semaphorin F homolog mRNA, complete cds//1.7e-34:328:76//Hs.27621 :U52840
F-NT2RP2001699//EST//0.029:94:68//Hs.125936:AA889091
F-NT2RP2001720//ESTs, Highly similar to Rap2 interacting protein 8 [M.musculus]//1.0:173:62//Hs.107361:AI197870
F-NT2RP2001721
F-NT2RP2001740//Homo sapiens Rigui (RIGUI) mRNA, complete cds//0.58:403:57//Hs.8114:AF022991
F-NT2RP2001748//Farnesyl diphosphate synthase (farnesyl pyrophosphate synthetase, dimethylallyltranstransferase, geranyltranstransferase)//1.2e-19:151:86//Hs.77393:D14697
F-NT2RP2001762//Homo sapiens exonuclease 1a (EXO1a) mRNA, complete_cds//5.2e-34:191:96//Hs.47504:AF091754
F-NT2RP2001813//EST//0.46:183:57//Hs.144096:AI032180
F-NT2RP2001839//EST//2.5e-12:86:94//Hs.133226:AI052250
F-NT2RP2001861//Homo sapiens mRNA for paraplegin//0.068:146:71//Hs.78497:Y16610
F-NT2RP2001869//Homo sapiens ZNF202 alpha (ZNF202) mRNA, complete cds//0.0013:174:62//Hs.9443:AF027219
F-NT2RP2001876//Allograft inflammatory factor 1//2.2e-08:162:67//Hs.76364:Y14768
F-NT2RP2001883
F-NT2RP2001898//75 KD INOSITOL-1,4,5-TRISPHOSPHATE 5-PHOSPHATASE PRECURSOR//3.0e-113:633:90//Hs.142189:M74161
F-NT2RP2001900//EST//1.9e-14:132:84//Hs.130049:AA902650
F-NT2RP2001907//ESTs, Weakly similar to ankyrin 3, long form [H.sapiens]//0.37:263:62//Hs.106377:H29757
F-NT2RP2001926//ESTs//1.1e-87:430:97//Hs.133487:AI393754
F-NT2RP2001936
F-NT2RP2001943
F-NT2RP2001946//ESTs//1.0:110:69//Hs.7941:AA894797
F-NT2RP2001947
F-NT2RP2001969//ESTs//3.3e-93:433:93//Hs.9622:W44489
F-NT2RP2001976//Homo sapiens KIAA0432 mRNA, complete cds//0.20:238:63//Hs.155174:AB007892
F-NT2RP2001985//Homo sapiens mRNA for KIAA0545 protein, partial cds//7.4e-05:235:62//Hs.129943:AB011117
F-NT2RP2001991//EST//0.0027:163:68//Hs.162458:AA579196
F-NT2RP2002025//Homo sapiens mRNA for KIAA0756 protein, partial cds//3.2e-62:314:97//Hs.116604:AB018299
F-NT2RP2002032
F-NT2RP2002033//EST//1.2e-16:224:74//Hs.150409:AI003543
F-NT2RP2002041//EST//0.022:139:69//Hs.127219:AA939336
F-NT2RP2002046//ESTs//1.1e-35:218:92//Hs.130678:R51509
F-NT2RP2002047//ESTs//0.43:131:64//Hs.153939:AI284198
F-NT2RP2002058//Homo sapiens mRNA for KIAA0741 protein, complete cds//0.96:137:71//Hs.3615:AB018284
F-NT2RP2002066//Homo sapiens transmembrane receptor UNC5C (UNC5C) mRNA, complete cds//3.1e-36:509:66//Hs.44553:AF055634
F-NT2RP2002070//ESTs//0.00027:107:72//Hs.4852:R84241
F-NT2RP2002076//Homo sapiens clone 24804 mRNA sequence//3.4e-129:643:96//Hs.11039:AF052183
F-NT2RP2002078//EST//1.0:83:65//Hs.115996:AA609014
F-NT2RP2002079//ESTs//6.2e-06:326:60//Hs.134202:AI313156
F-NT2RP2002099//Homo sapiens mRNA for E1B-55kDa-associated protein//3.2e-112:533:97//Hs.155218:AJ007509
F-NT2RP2002105//Homo sapiens serine threonine kinase 11 (STK11) mRNA, complete cds//6.1e-07:408:60//Hs.122755:AF032986
F-NT2RP2002124//ESTs//1.3e-90:459:96//Hs.142053:AA224286
F-NT2RP2002137//ATPase, Ca++ transporting, plasma membrane 4//0.0032:319:59//Hs.995:M83363
F-NT2RP2002154//Homo sapiens mRNA for C17orf1 protein//1.0:149:65//Hs.100217:AJ008112
F-NT2RP2002172//EST//4.4e-14:276:67//Hs.148392:AI085314
F-NT2RP2002185//ESTs, Weakly similar to ubiquitin S6(1) [D.melanogaster]//6.8e-61:354:91//Hs.109966:C06057
F-NT2RP2002192//Human 75-kD autoantigen (PM-Sc1) mRNA, complete cds//3.7e-37:194:97//Hs.91728:M58460
F-NT2RP2002193//Homo sapiens protein inhibitor of activated STAT protein PIASx-alpha mRNA, complete cds//6.8e-15:228:67//Hs.111323:AF077954
F-NT2RP2002208
F-NT2RP2002219//ESTs//0.0059:247:61//Hs.36495:AA151628
F-NT2RP2002231//ESTs//0.29:167:63//Hs.112013:AI394318
F-NT2RP2002235//H.sapiens mRNA for PHAPI2b protein//0.86:67:82//Hs.84264:U70439
F-NT2RP2002252//Homo sapiens mRNA for KIAA0527 protein, partial cds//0.79:264:59//Hs.129748:AB011099
F-NT2RP2002256//Homo sapiens retinoic acid hydroxylase mRNA, complete cds//2.1e-51:315:89//Hs.150595:AF005418
F-NT2RP2002259//Human L-myc protein gene, complete cds//1.2e-26:343:71//Hs.92137:M19720
F-NT2RP2002270//ESTs, Weakly similar to AF-9 PROTEIN [H.sapiens]//1.3e-31:206:88//Hs.4029:Z78373
F-NT2RP2002292//ESTs//1.3e-07:153:67//Hs.13533:H23079
F-NT2RP2002312//Homo sapiens CDP-diacylglycerol synthase 2 (CDS2) mRNA, partial cds//5.0e-95:467:96//Hs.24812:AF069532
F-NT2RP2002316//ESTs//0.95:194:63//Hs.157214:AA805445
F-NT2RP2002325//Homo sapiens peroxisomal biogenesis factor (PEX11a) mRNA, complete cds//1.3e-124:640:95//Hs.31034:AB015594
F-NT2RP2002333//Protein-tyrosine kinase tyk2 (non-receptor)//1.0:257:60//Hs.75516:X54637
F-NT2RP2002373
F-NT2RP2002385//Homo sapiens synaptic glycoprotein SC2 spliced variant mRNA, complete cds//3.1e-139:673:97//Hs.109051:AF038958
F-NT2RP2002394//Human clone 23695 mRNA sequence//0.16:456:59//Hs.90798:U79289
F-NT2RP2002408//HOMEOBOX/POU DOMAIN PROTEIN RDC-1//0.00069:265:65//Hs.74095:L20433
F-NT2RP2002426//EST//4.3e-33:271:79//Hs.145743:AI269098
F-NT2RP2002439//ESTs//0.0041:129:68//Hs.146064:AA714326
F-NT2RP2002442//ESTs, Weakly similar to similar to molybdoterin biosynthesis MOEB proteins [C.elegans]//5.6e-26:169:89//Hs.25198:AA904265
F-NT2RP2002457//ESTs//0.00031:121:71//Hs.134860:AI091436
F-NT2RP2002464//Human mRNA for KIAA0086 gene, complete cds//0.0013:207:63//Hs.1560:D42045
F-NT2RP2002475//ESTs//1.0:85:75//Hs.155371:AI139929
F-NT2RP2002479//Homo sapiens mRNA for ABC transporter 7 protein, complete cds//7.6e-125:607:96//Hs.125856:AB005289
F-NT2RP2002498
F-NT2RP2002503//Human zinc finger protein (FDZF2) mRNA, complete cds//2.2e-89:314:87//Hs.102681:U95044
F-NT2RP2002504//Homo sapiens mRNA for KIAA0791 protein, complete cds//3.8e-159:761:97//Hs.23255:AB018334
F-NT2RP2002520//RAB6, member RAS oncogene family//0.99:216:59//Hs.107563:M28212
F-NT2RP2002537
F-NT2RP2002546//EST//0.81:161:65//Hs.120562:AA741096
F-NT2RP2002549//ESTs//0.76:228:61//Hs.146313:AA594979
F-NT2RP2002591//Homo sapiens mRNA for KIAA0798 protein, complete cds//2.9e-33:285:78//Hs.159277:AB018341
F-NT2RP2002595//Adenylate cyclase 8 (brain)//0.39:377:59//Hs.2522:Z35309
F-NT2RP2002606//Human Line-1 repeat mRNA with 2 open reading frames//6.4e-24:144:95//Hs.23094:M19503
F-NT2RP2002609//Human guanine nucleotide regulatory protein (tim1) mRNA, complete cds//1.0:120:68//Hs.334:U02082
F-NT2RP2002618//H.sapiens mRNA for arginine methyltransferase, splice variant, 1262 bp//4.3e-28:460:63//Hs.20521:Y10805
F-NT2RP2002621
F-NT2RP2002643//Human p300/CBP-associated factor (P/CAF) mRNA, complete cds//0.0022:210:64//Hs.155302:U57317
F-NT2RP2002672//ESTs//7.4e-30:226:84//Hs.94694:W52493
F-NT2RP2002701//ESTs, Highly similar to HYPOTHETICAL 68.7 KD PROTEIN ZK757.1 IN CHROMOSOME III [Caenorhabditis elegans]//8.3e-56:278:97//Hs.109857:AA088385
F-NT2RP2002706//CEREBELLIN 1 PRECURSOR//0.00042:367:61//Hs.662:M58583
F-NT2RP2002710//Homo sapiens mRNA for KIAA0672 protein, complete cds//8.0e-42:631:65//Hs.6336:AB014572
F-NT2RP2002727
F-NT2RP2002736//ESTs//3.2e-67:336:97//Hs.86583:AA761217
F-NT2RP2002740//EST//1.0e-70:352:97//Hs.145168:AI150297
F-NT2RP2002741//Human mRNA for Neuroblastoma, complete cds//2.4e-30:628:62//Hs.87435:D89016
F-NT2RP2002750//Human mRNA for KIAA0331 gene, complete cds//2.1e-29:285:75//Hs.146395:AB002329
F-NT2RP2002752//EST//2.2e-06:126:74//Hs.159913:AA862709
F-NT2RP2002753//ESTs//4.3e-14:137:81//Hs.133478:T79705
F-NT2RP2002769//Human plectin (PLEC1) mRNA, complete cds//0.017:507:57//Hs.79706:U53204
F-NT2RP2002778//EST//1.6e-57:319:93//Hs.147519:AI216407
F-NT2RP2002800
F-NT2RP2002839//ESTs//0.075:177:62//Hs.132445:AA921763
F-NT2RP2002857//ESTs//0.99:88:69//Hs.132104:AI382142
F-NT2RP2002862
F-NT2RP2002880
F-NT2RP2002891//Homo sapiens mRNA for KIAA0673 protein, partial cds//1.0:237:62//Hs.106487:AB014573
F-NT2RP2002925//ESTs//1.6e-33:318:77//Hs.16808:W22606
F-NT2RP2002928//Homo sapiens pre-mRNA splicing factor (PRP17) mRNA, complete cds//3.9e-136:623:99//Hs.116674:AF038392
F-NT2RP2002929//Homo sapiens ataxin-7 (SCA7) mRNA, complete cds//0.24:158:65//Hs.108447:AJ000517
F-NT2RP2002939
F-NT2RP2002954
F-NT2RP2002959//Human E2 ubiquitin conjugating enzyme UbcH5B (UBCH5B) mRNA, complete cds//6.4e-21:135:91//Hs.108332:U39317
F-NT2RP2002979
F-NT2RP2002980
F-NT2RP2002986//Homo sapiens actin binding protein MAYVEN mRNA, complete cds//7.8e-11:272:61//Hs.122967:AF059569
F-NT2RP2002987//ESTs//8.2e-20:99:82//Hs.138965:AI004740
F-NT2RP2002993
F-NT2RP2003000//Small inducible cytokine A5 (RANTES)//2.1e-46:353:81//Hs.155464:AF088219
F-NT2RP2003034//ESTs//1.6e-08:263:66//Hs.164048:AA811741
F-NT2RP2003073//Human clone 230971 defective mariner transposon Hsmar2 mRNA sequence//4.6e-43:381:78//Hs.159176:U92019
F-NT2RP2003099//TRICHOHYALIN//0.98:183:62//Hs.82276:L09190
F-NT2RP2003108//H.sapiens nek2 mRNA for protein kinase//0.025:185:67//Hs.153704:U11050
F-NT2RP2003117//ESTs//7.6e-30:219:88//Hs.153408:AA416633
F-NT2RP2003121//ESTs//1.9e-13:158:73//Hs.129998:AI291379
F-NT2RP2003125//Serum response factor (c-fos serum response element-binding transcription factor)//4.5e-06:556:57//Hs.155321:J03161
F-NT2RP2003129//ESTs//0.095:218:63//Hs.70836:AA121544
F-NT2RP2003137
F-NT2RP2003157//Homo sapiens mRNA for KIAA0620 protein, partial cds//0.40:227:61//Hs.105958:AB014520
F-NT2RP2003158//Homo sapiens mRNA for proteasome subunit p58, complete cds//5.7e-113:581:93//Hs.9736:D67025
F-NT2RP2003161//ESTs//0.0095:120:65//Hs.163532:AI424170
F-NT2RP2003164//EST//0.11:179:63//Hs.163299:AA853944
F-NT2RP2003165//Human mRNA for KIAA0355 gene, complete cds//1.0e-39:342:79//Hs.153014:AB002353
F-NT2RP2003177//ESTs//3.6e-80:414:96//Hs.4767:N91123
F-NT2RP2003194//ESTs//5.4e-20:119:95//Hs.149531:AI393223
F-NT2RP2003206//EST//0.095:182:60//Hs.88461:AA278594
F-NT2RP2003228//CDC21 HOMOLOG//9.3e-138:726:93//Hs.154443:X74794
F-NT2RP2003230//ESTs//3.0e-10:239:62//Hs.163720:AA526947
F-NT2RP2003237//Human 53K isoform of Type II phosphatidylinositol-4-phosphate 5-kinase (PIPK) mRNA, complete cds//1.3e-62:543:77//Hs.108966:U48696
F-NT2RP2003243//Homo sapiens proline and glutamic acid rich nuclear protein isoform mRNA, partial cds//0.52:200:62//Hs.102732:U88153
F-NT2RP2003265
F-NT2RP2003272//ESTs, Weakly similar to ubiquitin S6(1) [D.melanogaster]//5.8e-57:313:93//Hs.109966:C06057
F-NT2RP2003277//Homo sapiens mRNA for KIAA0625 protein, partial cds//4.9e-147:714:96//Hs.154919:AB014525
F-NT2RP2003280
F-NT2RP2003286//Homo sapiens mRNA for KIAA0587 protein, complete cds//0.0097:243:65//Hs.21862:AB011159
F-NT2RP2003293//ESTs//5.5e-28:418:70//Hs.146227:AI269334
F-NT2RP2003295//Homo sapiens RMP mRNA for RPB5 meidating protein, complete cds//2.0e-86:416:97//Hs.7943:AB006572
F-NT2RP2003297//EST//0.99:240:60//Hs.133228:AI052312
F-NT2RP2003307//ESTs//5.6e-15:137:81//Hs.90020:AA442752
F-NT2RP2003308
F-NT2RP2003329//ESTs, Highly similar to HYPOTHETICAL 54.9 KD PROTEIN C02F5.7 IN CHROMOSOME III [Caenorhabditis elegans]//1.8e-102:532:95//Hs.6092:T75227
F-NT2RP2003339//ESTs//0.13:166:63//Hs.149649:AI346765
F-NT2RP2003347//ESTs//0.96:185:59//Hs.125003:H85963
F-NT2RP2003367//Human HsLIM15 mRNA for HsLim15, complete cds//0.99:243:60//Hs.37181:D64108
F-NT2RP2003391
F-NT2RP2003393
F-NT2RP2003394//Homo sapiens Ran-GTP binding protein mRNA, partial cds//0.86:416:57//Hs.4976:AF039023
F-NT2RP2003401
F-NT2RP2003433//ESTs, Highly similar to PROTEIN TRANSPORT PROTEIN SEC61 ALPHA SUBUNIT [Canis familiaris]//3.7e-33:303:77//Hs.14038:R06800
F-NT2RP2003445//EST//1.7e-06:154:65//Hs.142843:R36893
F-NT2RP2003446//Prostaglandin receptor, ep1 subtype//0.81:273:61//Hs.159360:L22647
F-NT2RP2003456//EST//0.17:95:65//Hs.147190:AI193320
F-NT2RP2003466//Homo sapiens chromosome 11, BAC CIT-HSP-311e8 (BC269730) containing the hFEN1 gene//4.3e-53:339:78//Hs.132874:AC004770
F-NT2RP2003480//Calpain, small polypeptide//1.1e-06:154:66//Hs.74451:X04106
F-NT2RP2003499//Homo sapiens delta-catenin mRNA, complete cds//3.1e-10:481:60//Hs.80220:U96136
F-NT2RP2003506
F-NT2RP2003511//Spectrin, beta, non-erythrocytic 1//0.76:189:62//Hs.107164:M96803
F-NT2RP2003513//Human mRNA for KIAA0270 gene, partial cds//8.3e-78:403:94//Hs.78482:Y16270
F-NT2RP2003517//Platelet-derived growth factor beta polypeptide (simian sarcoma viral (v-sis) oncogene homolog)//1.3e-24:151:95//Hs.1976:M12783
F-NT2RP2003522//Zinc finger protein 148 (pHZ-52)//1.1e-17:512:60//Hs.112180:AF039019
F-NT2RP2003533//ESTs//1.8e-76:373:98//Hs.140402:AI138765
F-NT2RP2003543//ESTs//9.3e-65:363:92//Hs.70643:AA030010
F-NT2RP2003559//ESTs//0.00037:93:77//Hs.157564:AI356513
F-NT2RP2003564//Sjogren syndrome antigen A1 (52kD, ribonucleoprotein autoantigen SS-A/Ro)//2.9e-28:664:63//Hs.1042:M62800
F-NT2RP2003567//Homo sapiens mRNA for KIAA0462 protein, partial cds//1.3e-114:541:98//Hs.129937:AB007931
F-NT2RP2003581//EST//1.0:59:76//Hs.158575:AI368947
F-NT2RP2003596//ESTs, Weakly similar to No definition line found [C.elegans]//1.3e-63:224:95//Hs.34627:AA126463
F-NT2RP2003604//Homo sapiens alpha-catenin related protein (ACRP) mRNA, complete cds//1.7e-124:585:98//Hs.58488:U97067
F-NT2RP2003629//ESTs//2.0e-103:535:95//Hs.105633:AA479166
F-NT2RP2003643//Kallmann syndrome 1 sequence//0.85:216:61//Hs.89591:M97252
F-NT2RP2003668//Homo sapiens haemopoietic progenitor homeobox HPX42B (HPX42B) mRNA, complete cds//9.4e-47:371:80//Hs.125231:AF068006
F-NT2RP2003687//EST//2.9e-14:134:80//Hs.132635:AI032875
F-NT2RP2003691//ESTs//8.2e-47:296:83//Hs.138852:AA284247
F-NT2RP2003702//DNA POLYMERASE EPSILON, CATALYTIC SUBUNIT A//0.85:190:61//Hs.18366:L09561
F-NT2RP2003704//ESTs, Weakly similar to putative p150 [H.sapiens]//5.1e-44:269:91//Hs.139757:N95271
F-NT2RP2003706//Homo sapiens mRNA for KIAA0525 protein, partial cds//8.3e-110:518:98//Hs.78494:AB011097
F-NT2RP2003713
F-NT2RP2003714//Homo sapiens hematopoietic cell derived zinc finger protein mRNA, complete cds//2.7e-56:252:83//Hs.86371:AF054180
F-NT2RP2003727//EST//0.52:277:59//Hs.69507:AA111879
F-NT2RP2003737//Human E2 ubiquitin conjugating enzyme UbcH5C (UBCH5C) mRNA, complete cds//4.0e-55:584:71//Hs.118797:U39318
F-NT2RP2003751
F-NT2RP2003760
F-NT2RP2003764
F-NT2RP2003769
F-NT2RP2003770//RETINOBLASTOMA BINDING PROTEIN 3//0.58:247:59//Hs.96055:U47677
F-NT2RP2003777
F-NT2RP2003781//ESTs, Weakly similar to C47D12.3 [C.elegans]//3.7e-63:356:92//Hs.16131:AA568689
F-NT2RP2003793//ESTs//4.8e-68:392:92//Hs.93949:AA782955
F-NT2RP2003825//ESTs//7.6e-79:232:98//Hs.14347:AA287742
F-NT2RP2003840//DNAJ PROTEIN HOMOLOG HSJ1//0.95:300:59//Hs.77768:X63368
F-NT2RP2003857//EST//1.0:112:62//Hs.139216:AA244425
F-NT2RP2003859
F-NT2RP2003871//ESTs//2.5e-44:222:99//Hs.146295:AA935780
F-NT2RP2003885
F-NT2RP2003912//ESTs, Weakly similar to G2-SPECIFIC PROTEIN KINASE NIMA [Emericella nidulans]//2.2e-113:632:92//Hs.50072:AI378221
F-NT2RP2003952//ESTs, Moderately similar to 60S RIBOSOMAL PROTEIN L32 [H.sapiens]//1.0:146:67//Hs.156920:AA489296
F-NT2RP2003968//Homo sapiens hUBP mRNA for ubiquitin specific protease, complete cds//6.8e-30:165:96//Hs.35086:AB014458
F-NT2RP2003976//Homo sapiens mRNA for KIAA0447 protein, complete cds//7.9e-116:610:94//Hs.7302:AB007916
F-NT2RP2003981//Homo sapiens mRNA for KIAA0804 protein, partial cds//3.2e-161:783:96//Hs.7316:AB018347
F-NT2RP2003984
F-NT2RP2003986//ESTs//1.3e-39:296:83//Hs.152482:AI050036
F-NT2RP2003988//Thiopurine S-methyltransferase//7.1e-44:532:70//Hs.51124:AF019369
F-NT2RP2004013//ESTs, Highly similar to TRANSCRIPTION FACTOR BTF3 [Homo sapiens]//7.0e-104:556:93//Hs.111081:AI380378
F-NT2RP2004014
F-NT2RP2004041//Homo sapiens chromosome 19, cosmid F17127//6.0e-11:120:80//Hs.10116:AC004780
F-NT2RP2004042
F-NT2RP2004066//Homo sapiens zinc finger protein (ZnF20) mRNA, complete cds//0.80:292:61//Hs.1147:AF011573
F-NT2RP2004081//ESTs//5.7e-87:427:96//Hs.102296:AI217942
F-NT2RP2004098//Homo sapiens leucine-rich repeat protein SHOC-2 (SHOC-2) mRNA, complete cds//0.15:199:60//Hs.104315:AF054828
F-NT2RP2004124//Homo sapiens mRNA for ephrin-A2//0.98:233:59//Hs.158306:AJ007292
F-NT2RP2004142
F-NT2RP2004152//ESTs//5.7e-35:187:96//Hs.98977:AA625872
F-NT2RP2004165//Homo sapiens serine kinase SRPK2 mRNA, complete cds//0.69:176:63//Hs.78353:U88666
F-NT2RP2004170//ESTs//3.9e-05:380:61//Hs.143748:AI419966
F-NT2RP2004172//ESTs//5.8e-18:104:99//Hs.157031:AI343501
F-NT2RP2004187//ESTs, Moderately similar to zinc finger protein [H.sapiens]//1.7e-16:276:67//Hs.36779:AA626790
F-NT2RP2004194//Human p300/CBP-associated factor (P/CAF) mRNA, complete cds//1.0:124:69//Hs.155302:U57317
F-NT2RP2004196
F-NT2RP2004207//ESTs//3.8e-11:92:88//Hs.22678:AA604756
F-NT2RP2004226//ESTs, Weakly Similar to teg292 protein [M.musculus]//1.8e-80:386:98//Hs.68791:AA527270
F-NT2RP2004232//Protein kinase C, mu//3.9e-36:448:67//Hs.2891:X75756
F-NT2RP2004239//ESTs//0.12:196:61//Hs.127209:AA976680
F-NT2RP2004240//EST//1.0:134:63//Hs.104466:AA282536
F-NT2RP2004242//Homo sapiens Nck-2 (NCK2) mRNA, complete cds//0.27:313:59//Hs.129725:AF047487
F-NT2RP2004245//ESTs, Weakly similar to No definition line found [C.elegans]//8.2e-51:474:74//Hs.108990:N25951
F-NT2RP2004270//MUELLERIAN INHIBITING FACTOR PRECURSOR//1.6e-06:490:60//Hs.12432:AC005263
F-NT2RP2004300//1-PHOSPHATIDYLINOSITOL-4,5-BISPHOSPHATE PHOSPHODIESTERASE BETA 3//0.35:157:67//Hs.37121:Z37544
F-NT2RP2004316//Homo sapiens EXT-like protein 2 (EXTL2) mRNA, complete cds//1.5e-151:735:97//Hs.61152:AF000416
F-NT2RP2004321//ESTs//2.6e-64:385:88//Hs.133128:W27735
F-NT2RP2004339//ESTs//3.3e-46:338:83//Hs.145091:AA814510
F-NT2RP2004347//ESTs//1.0:184:61//Hs.134469:AA731632
F-NT2RP2004364//ESTs//2.9e-70:366:95//Hs.14928:AA256202
F-NT2RP2004365
F-NT2RP2004366//Homo sapiens mRNA for DFFRY protein, abundant transcript//0.60:295:57//Hs.39163:AF000986
F-NT2RP2004373
F-NT2RP2004389//ESTs, Highly similar to HYPOTHETICAL 70.7 KD PROTEIN F09G8.3 IN CHROMOSOME III [Caenorhabditis elegans]//3.3e-97:477:98//Hs.30490:AA146916
F-NT2RP2004392//ESTs//2.6e-61:305:98//Hs.43100:AA186588
F-NT2RP2004396//Homo sapiens BAC clone RG135C18 from 7q21//1.4e-174:875:95//Hs.152759:AC005164
F-NT2RP2004399//ESTs, Weakly similar to K01H12.1 [C.elegans]//1.2e-92:519:91//Hs.13275:AI341468
F-NT2RP2004400//EST//0.018:150:65//Hs.158739:AI375367
F-NT2RP2004412
F-NT2RP2004425//EST//0.049:145:64/Hs.160759:R36944
F-NT2RP2004463//ESTs//1.5e-40:207:98//Hs.98057:C15687
F-NT2RP2004476//Homo sapiens TWIK-related acid-sensitive K+ channel (TASK) mRNA, complete cds//0.45:208:61//Hs.24040:AF006823
F-NT2RP2004490
F-NT2RP2004512//ESTs//0.0012:330:61//Hs.70258:AI091203
F-NT2RP2004523//Human high-affinity copper uptake protein (hCTR1) mRNA, complete cds//1.3e-29:270:79//Hs.73614:U83460
F-NT2RP2004538//Homo sapiens mRNA for KIAA0591 protein, partial cds//4.6e-139:687:96//Hs.129908:AB011163
F-NT2RP2004551//ESTs//0.0075:285:62//Hs.149442:Al346891
F-NT2RP2004568//Homo sapiens antigen NY-CO-16 mRNA, complete cds//8.8e-06:291:61//Hs.132206:AF039694
F-NT2RP2004580//Small inducible cytokine A5 (RANTES)//1.2e-45:334:82//Hs.155464:AF088219
F-NT2RP2004587//Homo sapiens mRNA for KIAA0766 protein, complete cds//0.98:136:64//Hs.28020:AB018309
F-NT2RP2004594//ESTs, Highly similar to MKR2 PROTEIN [Mus musculus]//1.0:104:68//Hs.125729:N99898
F-NT2RP2004600//Homo sapiens mRNA for Hrs, complete cds//0.20:260:60//Hs.24756:U43895
F-NT2RP2004602//ESTs, Weakly similar to !!!! ALU SUBFAMILY J WARNING ENTRY !!!! [H.sapiens]//3.0e-59:273:93//Hs.12845:N28835
F-NT2RP2004614//EST//0.99:103:68//Hs.148738:AI224908
F-NT2RP2004655//Homo sapiens mRNA for leucine rich protein//8.4e-104:496:98//Hs.5198:AJ006291
F-NT2RP2004664//Homo sapiens mRNA for KIAA0460 protein, partial cds//5.2e-155:728:98//Hs.29956:AB007929
F-NT2RP2004675//EST//0.65:151:62//Hs.130504:AI003839
F-NT2RP2004681
F-NT2RP2004689//Homo sapiens mRNA for KIAA0625 protein, partial cds//4.1e-61:327:94//Hs.154919:AB014525
F-NT2RP2004709//ESTs//2.2e-05:98:77//Hs.161898:AA286942
F-NT2RP2004710//ESTs//0.0035:76:82//Hs.108470:R93780
F-NT2RP2004736//Homo sapiens mRNA for KIAA0478 protein, complete cds//2.1e-118:582:96//Hs.4236:AB007947
F-NT2RP2004743//EST//0.11:170:64//Hs.112670:AA609242
F-NT2RP2004767//EST//1.5e-09:303:65//Hs.148374:AA948183
F-NT2RP2004768//ESTs, Highly similar to SERINE/THREONINE-PROTEIN KINASE PAK [Rattus norvegicus]//3.7e-110:548:96//Hs.85768:W16504
F-NT2RP2004775//Homo sapiens transcriptional regulatory protein p54 mRNA, complete cds//0.025:547:57//Hs.107474:AF045451
F-NT2RP2004791//Human endosome-associated protein (EEA1) mRNA, complete cds//0.99:121:64//Hs.2864:L40157
F-NT2RP2004799//Homo sapiens ATP-specific succinyl-CoA synthetase beta subunit (SCS) mRNA, partial cds//4.9e-118:594:95//Hs.40820:AF058953
F-NT2RP2004802//ESTs//5.6e-16:116:91//Hs.153841:N36043
F-NT2RP2004816//Homo sapiens H beta 58 homolog mRNA, complete cds//6.8e-103:495:97//Hs.67052:AF054179
F-NT2RP2004841//Human transposon-like element mRNA//3.0e-70:519:83//Hs.84775:M23161
F-NT2RP2004861//ESTs//6.7e-89:427:98//Hs.132980:AI290258
F-NT2RP2004897//ESTs//6.4e-81:431:94//Hs.130961:N79111
F-NT2RP2004933//Homo sapiens mRNA for ZIP-kinase, complete cds//6.5e-84:418:95//Hs.25619:AB007144
F-NT2RP2004936
F-NT2RP2004959
F-NT2RP2004961//Human mRNA for KIAA0065 gene, partial cds//7.2e-26:456:66//Hs.70617:D31763
F-NT2RP2004962//EST//2.8e-15:242:69//Hs.146794:AI149478
F-NT2RP2004967//ESTs//0.0022:218:63//Hs.131987:AI239735
F-NT2RP2004978//Homo sapiens mRNA for KIAA0458 protein, complete cds//1.0:218:61//Hs.7414:AB007927
F-NT2RP2004982//Human kinesin-like spindle protein HKSP (HKSP) mRNA, complete cds//0.13:260:60//Hs.41723:U37426
F-NT2RP2004985//Human mRNA for KIAA0144 gene, complete cds//4.8e-22:431:65//Hs.8127:D63478
F-NT2RP2004999
F-NT2RP2005000//Homo sapiens hyperpolarization-activated channel 1 (IH1) mRNA, partial cds//0.99:269:58//Hs.124161:AF065164
F-NT2RP2005001//Homo sapiens mRNA for KIAA0615 protein, complete cds//1.9e-160:782:97//Hs.155972:AB014515
F-NT2RP2005003//H.sapiens Staf50 mRNA//9.9e-44:430:75//Hs.68054:X82200
F-NT2RP2005012//Homo sapiens SEC63 (SEC63) mRNA, complete cds//4.5e-100:501:96//Hs.31575:AF100141
F-NT2RP2005018//Arachidonate 5-lipoxygenase//1.0:232:58//Hs.89499:J03600
F-NT2RP2005020//ESTs//1.2e-06:61:100//Hs.106160:AA527433
F-NT2RP2005022//Eukaryotic translation initiation factor 3 (eIF-3) p36 subunit//0.095:271:60//Hs.139745:U39067
F-NT2RP2005031//Homo sapiens mRNA for SCP-1, complete cds//0.99:338:61//Hs.112743:D67035
F-NT2RP2005037//Homo sapiens mRNA for repressor protein, partial cds//0.098:217:60//Hs.58167:D30612
F-NT2RP2005038//Homo sapiens protease-activated receptor 4 mRNA, complete cds//0.22:498:59//Hs.137574:AF055917
F-NT2RP2005108//ESTs//0.74:145:63//Hs.116557:AA657838
F-NT2RP2005116//Homo sapiens mRNA for KIAA0664 protein, partial cds//6.4e-105:495:98//Hs.22616:AB014564
F-NT2RP2005126//H.sapiens mRNA for RNA helicase (Myc-regulated dead box protein)//9.2e-29:157:98//Hs.100555:X98743
F-NT2RP2005139//ESTs//2.6e-91:479:95//Hs.125037:W42803
F-NT2RP2005140//ESTs//0.81:308:59//Hs.27308:AA534947
F-NT2RP2005144//Homo sapiens tubby like protein 3 (TULP3) mRNA, complete cds//8.3e-91:447:96//Hs.132226:AF045583
F-NT2RP2005147
F-NT2RP2005159//ESTs//1.5e-44:242:94//Hs.109819:AI357582
F-NT2RP2005162//ESTs, Weakly similar to Y53C12A.3 [C.elegans]//0.97:80:73//Hs.107747:AI357868
F-NT2RP2005168//Homo sapiens mRNA for E1B-55kDa-associated protein//4.4e-127:633:96//Hs.155218:AJ007509
F-NT2RP2005204//H.sapiens 5T4 gene for 5T4 Oncofetal antigen//0.0034:187:66//Hs.82128:AJ012159
F-NT2RP2005227//Homo sapiens PAC clone DJ0905J08 from 7p12-p14//1.3e-66:340:95//Hs.8173:AC005189
F-NT2RP2005239//EST//1.3e-05:215:66//Hs.129528:AA994783
F-NT2RP2005254//H.sapiens mRNA for PHAPI2b protein//1.0:101:71//Hs.84264:U70439
F-NT2RP2005270//Homo sapiens creatine transporter mRNA, complete cds//0.56:114:68//Hs.154503:U36341
F-NT2RP2005276//Homo sapiens acyl-CoA synthetase 4 (ACS4) mRNA, complete cds//1.2e-40:594:65//Hs.81452:AF030555
F-NT2RP2005287//ESTs//8.2e-07:175:70//Hs.117134:AI383932
F-NT2RP2005288//Homo sapiens RCC1-like G exchanging factor RLG mRNA, complete cds//2.3e-123:604:96//Hs.27007:AF060219
F-NT2RP2005289//Homo sapiens mRNA for XPR2 protein//1.3e-141:670:98//Hs.44766:AJ007590
F-NT2RP2005293//EST//1.9e-50:254:98//Hs.162017:AA505833
F-NT2RP2005315//Homo sapiens mRNA for KIAA0676 protein, partial cds//3.6e-97:483:96//Hs.115763:AB014576
F-NT2RP2005325//Human LIM-homeobox domain protein (hLH-2) mRNA, complete cds//2.6e-23:166:90//Hs.1569:U11701
F-NT2RP2005336//Homo sapiens snRNA activating protein complex 190kD subunit (SNAP190) mRNA, complete cds//0.016:353:62//Hs.113265:AF032387
F-NT2RP2005344//Homo sapiens mRNA for KIAA0566 protein, partial cds//2.8e-30:456:66//Hs.44697:AB011138
F-NT2RP2005354//ESTs//0.71:192:60//Hs.39063:AA708958
F-NT2RP2005358//Homo sapiens methyl-CpG binding protein MBD3 (MBD3) mRNA, complete cds//1.4e-100:489:96//Hs.107254:AC005943
F-NT2RP2005360//ESTs//8.2e-35:190:95//Hs.163038:AA700122
F-NT2RP2005393//Homo sapiens CTG26 alternate open reading frame mRNA, complete cds//0.87:244:59//Hs.113252:U80761
F-NT2RP2005407
F-NT2RP2005436//Homo sapiens mRNA for KIAA0561 protein, partial cds//0.28:338:57//Hs.6189:AB011133
F-NT2RP2005441//ESTs//3.3e-45:238:96//Hs.5209:AA780068
F-NT2RP2005453//ESTs//2.1e-20:115:99//Hs.133087:AI091164
F-NT2RP2005457//ESTs, Highly similar to NADH-UBIQUINONE OXIDOREDUCTASE SUBUNIT B14.5B [Bos taurus]//8.5e-48:295:90//Hs.75017:AA166853
F-NT2RP2005464//ESTs//2.0e-99:495:96//Hs.3530:AA808243
F-NT2RP2005465//V-crk avian sarcoma virus CT10 oncogene homolog//0.032:176:64//Hs.16:D10656
F-NT2RP2005472//ESTs//1.4e-34:180:98//Hs.158892:AD78412
F-NT2RP2005476//Homo sapiens mRNA for KIAA0772 protein, complete cds//9.9e-48:432:77//Hs.15519:AB018315
F-NT2RP2005490//ESTs//4.5e-19:165:84//Hs.134382:AA083573
F-NT2RP2005491
F-NT2RP2005495//ESTs//5.6e-96:452:99//Hs.145417:AI084164
F-NT2RP2005496//Human mRNA for KIAA0326 gene, partial cds//4.4e-48:621:68//Hs.6833:AB002324
F-NT2RP2005498//Human protein phosphatase 2A beta subunit mRNA, complete cds//1.6e-63:503:78//Hs.7688:M64930
F-NT2RP2005501//Homo sapiens Notch3 (NOTCH3) mRNA, complete cds//0.56:139:66//Hs.8546:U97669
F-NT2RP2005509//Glutamate-cysteine ligase (gamma-glutamylcysteine synthetase), regulatory (30.8kD)//1.0:291:59//Hs.89709:L35546
F-NT2RP2005520//Homo sapiens chromosome-associated protein-E (hCAP-E) mRNA, complete cds//1.2e-82:444:92//Hs.119023:AF092563
F-NT2RP2005525//Homo sapiens mRNA for KIAA0764 protein, complete cds//2.2e-19:112:99//Hs.6232:AB018307
F-NT2RP2005531//ESTs, Weakly similar to erythrocyte membrane protein 4.1 [H.sapiens]//3.5e-50:366:83//Hs.61833:AA036735
F-NT2RP2005539//Homo sapiens mRNA for NS1-binding protein (NS1-BP)//9.4e-155:747:97//Hs.159597:AJ012449
F-NT2RP2005540//Homo sapiens mRNA for KIAA0494 protein, complete cds//1.9e-131:618:98//Hs.62515:AB007963
F-NT2RP2005549//ESTs, Weakly similar to HYPOTHETICAL 32.0 KD PROTEIN C16C10.10 IN CHROMOSOME III [C.elegans]//2.5e-51:292:93//Hs.105684:H24407
F-NT2RP2005555//EST//0.046:308:57//Hs.145962:AI276822
F-NT2RP2005557//ESTs//4.6e-48:382:79//Hs.125014:AI422839
F-NT2RP2005581//ESTs//6.3e-28:166:93//Hs.87803:AA034436
F-NT2RP2005600//ESTs//1.6e-40:228:93//Hs.160085:AI218627
F-NT2RP2005605//ESTs//5.7e-13:115:86//Hs.37718:H60071
F-NT2RP2005620//Homo sapiens epsin 2b mRNA, complete cds//3.1e-92:447:97//Hs.22396:AF062085
F-NT2RP2005622//ESTs//0.16:242:63//Hs.136395:AA523702
F-NT2RP2005635
F-NT2RP2005637//ESTs//0.055:96:69//Hs.105998:R90905
F-NT2RP2005640//ESTs//4.5e-16:107:92//Hs.150823:AI292145
F-NT2RP2005645//ESTs//2.7e-29:181:90//Hs.121653:AI375440
F-NT2RP2005651//Oxysterol binding protein//0.00011:122:69//Hs.1433065:M86917
F-NT2RP2005654//Homo sapiens mRNA for KIAA0288 gene, complete cds//1.5e-08:351:62//Hs.91400:AB006626
F-NT2RP2005669//ESTs//0.016:185:64//Hs.97713:AA442239
F-NT2RP2005675//Homo sapiens growth suppressor related (DOC-1R) mRNA, complete cds//7.7e-96:462:98//Hs.25664:AF089814
F-NT2RP2005683//ESTs//0.83:242:62//Hs.136395:AA523702
F-NT2RP2005690//PYRROLINE-5-CARBOXYLATE REDUCTASE//2.5e-11:328:61//Hs.79217:M77836
F-NT2RP2005694
F-NT2RP2005701//Homo sapiens protein phosphatase 2A B56-epsilon (PP2A) mRNA, complete cds//0.15:496:55//Hs.79326:L76703
F-NT2RP2005712//Homo sapiens mRNA for KIAA0799 protein, partial cds//5.1e-126:599:97//Hs.61638:AB018342
F-NT2RP2005719//ESTs//0.58:326:60//Hs.157209:N57527
F-NT2RP2005722//Zinc finger protein 136 (clone pHZ-20)//8.2e-46:415:77//Hs.69740:U09367
F-NT2RP2005723//ESTs//1.0e-15:141:81//Hs.163747:AA174017
F-NT2RP2005726//EST//3.4e-15:96:95//Hs.156170:AI334191
F-NT2RP2005732//ESTs//0.99:162:62//Hs.154914:AA721086
F-NT2RP2005741//Homo sapiens chondroadherin gene, 5'flanking region and//0.80:362:58//Hs.97220:U96769
F-NT2RP2005748//H.sapiens ZNF33B gene//0.47:99:65//Hs.72991:X68688
F-NT2RP2005752//Homo sapiens TNFR-related death receptor-6 (DR6) mRNA, complete cds//2.5e-23:134:96//Hs.159651:AF068868
F-NT2RP2005753//Homo sapiens I-1 receptor candidate protein mRNA, complete cds//4.0e-102:486:98//Hs.26285:AF082516
F-NT2RP2005763//EUKARYOTIC INITIATION FACTOR 4A-LIKE NUK-34//2.3e-05:425:56//Hs.79768:D21853
F-NT2RP2005767//Homolog 2 of Drosophila large discs//0.085:262:61//Hs.23205:X82895
F-NT2RP2005773//PYRROLINE-5-CARBOXYLATE REDUCTASE//2.0e-16:153:82//Hs.79217:M77836
F-NT2RP2005775//Human thimet oligopeptidase (THOP1) mRNA, complete cds//1.7e-42:645:64//Hs.78769:Z50115
F-NT2RP2005781//ESTs//1.1e-19:132:90//Hs.13550:AI378556
F-NT2RP2005784//Inhibitor of DNA binding 4, dominant negative helix-loop-helix protein//2.9e-06:201:67//Hs.34853:U28368
F-NT2RP2005804//ESTs//1.2e-07:62:93//Hs.125509:AA883820
F-NT2RP2005812
F-NT2RP2005815//ESTs//1.9e-32:173:97//Hs.144587:AI193595
F-NT2RP2005835
F-NT2RP2005841//Homo sapiens retinal rod Na-Ca+K exchanger (NCKX1) mRNA, complete cds//0.94:148:65//Hs.59829:AB014602
F-NT2RP2005853
F-NT2RP2005857//Homo sapiens chromosome-associated protein-C (hCAP-C) mRNA, partial cds//5.4e-176:829:98//Hs.50758:AF092564
F-NT2RP2005859//ESTs//2.1e-97:537:92//Hs.131915:W22567
F-NT2RP2005868
F-NT2RP2005886//Human putative M phase phosphoprotein 1 (MPP1) mRNA, partial cds//0.26:728:57//Hs.240:L16782
F-NT2RP2005890//ESTs//2.0e-97:453:100//Hs.88671:AA279943
F-NT2RP2005901//ESTs//0.99:188:64//Hs.28639:R78360
F-NT2RP2005908//ESTs//2.5e-43:325:82//Hs.152340:AA521399
F-NT2RP2005933//ESTs, Highly similar to nucleoporin p54 [R.norvegicus]//7.9e-90:326:98//Hs.156882:AA292186
F-NT2RP2005942//H.sapiens PAP mRNA//5.1e-48:618:67//Hs.49007:X76770
F-NT2RP2005980//ESTs//2.8e-22:358:68//Hs.125446:AA883339
F-NT2RP2006023
F-NT2RP2006038//ESTs//8.0e-37:351:74//Hs.128787:AA418382
F-NT2RP2006043//Human novel homeobox mRNA for a DNA binding protein//0.51:271:59//Hs.37035:U07664
F-NT2RP2006052//ESTs//4.0e-05:233:63//Hs.124864:AA663093
F-NT2RP2006069//Human mRNA for KIAA0279 gene, partial cds//0.0082:770:58//Hs.57652:D87469
F-NT2RP2006071//ESTs//2.1e-24:396:65//Hs.104404:AI337416
F-NT2RP2006098//ESTs//0.97:125:67//Hs.97996:AA405970
F-NT2RP2006100
F-NT2RP2006103//ESTs//5.2e-11:102:83//Hs.125656:AA883135
F-NT2RP2006106//ESTs//1.6e-78:456:90//Hs.133496:AA315349
F-NT2RP2006141//ESTs//1.7e-20:262:72//Hs.128677:AA649240
F-NT2RP2006166
F-NT2RP2006184//H.sapiens p63 mRNA for transmembrane protein//1.0:94:73//Hs.74368:X69910
F-NT2RP2006186//Homo sapiens mRNA for KIAA0654 protein, partial cds//2.5e-114:567:96//Hs.109299:AB014554
F-NT2RP2006196//Homo sapiens mRNA for KIAA0772 protein, complete cds//2.0e-23:187:85//Hs.15519:AB018315
F-NT2RP2006200//ESTs//1.0:224:62//Hs.144100:AI205503
F-NT2RP2006219//H.sapiens mRNA for DGCR6 protein//4.4e-118:618:93//Hs.153910:X96484
F-NT2RP2006237
F-NT2RP2006238
F-NT2RP2006258//ESTs//0.0034:143:69//Hs.145798:AI269970
F-NT2RP2006261//H.sapiens mRNA for serine/threonine protein kinase EMK//0.019:111:71//Hs.157199:X97630
F-NT2RP2006275//Homo sapiens mRNA for serin protease with IGF-binding motif, complete cds//2.4e-05:388:60//Hs.75111:D87258
F-NT2RP2006312//Homo sapiens BAF57 (BAF57) gene, complete cds//2.1e-121:598:97//Hs.3404:AF035262
F-NT2RP2006320//ESTs, Moderately similar to maternal transcript Maid [M.musculus]//1.9e-29:151:100//Hs.36794:AI038407
F-NT2RP2006321//ESTs//7.0e-15:141:82//Hs.71241:H09371
F-NT2RP2006323//Homo sapiens mRNA for NBPhox, complete cds//4.7e-06:170:70//Hs.87202:D82344
F-NT2RP2006333//Homo sapiens TRRAP protein (TRRAP) mRNA, complete cds//0.11:43:100//Hs.6892:AF076974
F-NT2RP2006334//Homo sapiens mRNA for KIAA0602 protein, partial cds//3.1e-05:233:65//Hs.37656:AB011174
F-NT2RP2006365//ESTs//8.9e-46:268:93//Hs.58403:AA058501
F-NT2RP2006393//ESTs//1.2e-20:159:86//Hs.146018:AA280341
F-NT2RP2006436//Human homeodomain-containing protein (HANF) mRNA, complete cds//0.59:133:64//Hs.95838:AF059734
F-NT2RP2006441//ESTs//1.6e-82:400:98//Hs.143514:AI221934
F-NT2RP2006454//EST//5.2e-07:172:68//Hs.157742:AI360509
F-NT2RP2006456
F-NT2RP2006464//Homo sapiens mRNA for AND-1 protein/1.1e-149:545:98//Hs.72160:AJ006266
F-NT2RP2006467
F-NT2RP2006472
F-NT2RP2006534//ESTs//5.6e-05:192:66//Hs.135750:AA160048
F-NT2RP2006554//EST//0.60:116:65//Hs.160110:AA922134
F-NT2RP2006565//Homo sapiens secretory carrier-associated membrane protein (SCAMP) mRNA, complete cds//2.1e-115:669:90//Hs.31218:AF038966
F-NT2RP2006571//Cytochrome P450, subfamily IIA (phenobarbital-inducible), polypeptide 6//2.1e-24:476:64//Hs.73864:U22029
F-NT2RP2006573
F-NT2RP2006598//ESTs//1.3e-16:137:85//Hs.131350:AA805223
F-NT2RP3000002//ESTs//3.6e-32:215:86//Hs.155446:AA188180
F-NT2RP3000031//Homo sapiens mRNA for histone deacetylase-like protein (JM21)//1.9e-137:637:98//Hs.6764:AJ011972
F-NT2RP3000046//Homo sapiens TTF-I interacting peptide 20 mRNA, partial cds//9.1e-07:568:61//Hs.79531:AF000560
F-NT2RP3000047
F-NT2RP3000050//Human repressor transcriptional factor (ZNF85) mRNA, complete cds//1.2e-58:633:69//Hs.37138:U35376
F-NT2RP3000055//ESTs//1.2e-07:200:66//Hs.127362:AA954961
F-NT2RP3000068
F-NT2RP3000072//EST//0.99:199:63//Hs.8469:T40769
F-NT2RP3000080//Landsteiner-Wiener blood group glycoprotein//4.8e-41:353:78//Hs.108287:L27670
F-NT2RP3000085//Propionyl-coA carboxylase alpha chain//7.9e-30:665:60//Hs.80741:X14608
F-NT2RP3000092//EST//2.0e-15:94:97//Hs.145389:AI253140
F-NT2RP3000109//ESTs//6.8e-11:77:96//Hs.153931:AI243595
F-NT2RP3000134//Homo sapiens PAC clone DJ0905J08 from 7p12-p14//5.0e-94:438:100//Hs.8173:AC005189
F-NT2RP3000142//Homo sapiens mRNA for KIAA0592 protein, partial cds//2.9e-182:849:98//Hs.13273:AB011164
F-NT2RP3000149//Human Line-1 repeat mRNA with 2 open reading frames//4.1e-20:133:94//Hs.23094:M19503
F-NT2RP3000186//Homo sapiens mRNA, chromosome 1 specific transcript KIAA0492//6.6e-08:152:71//Hs.127338:AB007961
F-NT2RP3000197//ESTs//1.1e-58:301:96//Hs.87461:AA292779
F-NT2RP3000207
F-NT2RP3000220
F-NT2RP3000233//Homo sapiens actin binding protein MAYVEN mRNA, complete cds//6.6e-20:509:58//Hs.122967:AF059569
F-NT2RP3000235//ESTs//1.7e-06:220:62//Hs.42771:N26740
F-NT2RP3000247//Human mRNA for KIAA0218 gene, complete cds//6.7e-111:691:86//Hs.75863:D86972
F-NT2RP3000251//ESTs//6.7e-48:245:97//Hs.28249:AA203733
F-NT2RP3000252
F-NT2RP3000255
F-NT2RP3000267//ESTs//0.14:53:92//Hs.151586:W45568
F-NT2RP3000299//Homo sapiens enhancer of filamentation (HEF1) mRNA, complete cds//1.7e-13:214:67//Hs.80261:L43821
F-NT2RP3000312//ESTs//2.6e-50:255:97//Hs.146263:AA255863
F-NT2RP3000320//Homo sapiens proline and glutamic acid rich nuclear protein isoform mRNA, partial cds//0.0088:236:63//Hs.102732:U88153
F-NT2RP3000324//ESTs//3.8e-10:102:83//Hs.55495:AI091242
F-NT2RP3000333//ESTs, Weakly similar to mitogen-activated kinase kinase kinase 5 [H.sapiens]//0.57:189:65//Hs.46146:AA418097
F-NT2RP3000341//Human mRNA for KIAA0392 gene, partial cds//1.1e-49:442:78//Hs.40100:AB002390
F-NT2RP3000348
F-NT2RP3000350//H.sapiens mRNA for GTP-binding protein//0.93:164:59//Hs.78582:X80754
F-NT2RP3000359//GTP:AMP PHOSPHOTRANSFERASE MITOCHONDRIAL//1.8e-43:649:66//Hs.101642:X60673
F-NT2RP3000361//ESTs//2.6e-112:531:98//Hs.17672:AA305921
F-NT2RP3000366//ESTs, Highly similar to RAS-RELATED PROTEIN RAB-18A [Lymnaea stagnalis]//4.0e-116:596:95//Hs.21094:AI337016
F-NT2RP3000393//ESTs//2.6e-18:137:89//Hs.115600:AA351639
F-NT2RP3000397//ESTs//8.7e-44:355:73//Hs.121961:AA777873
F-NT2RP3000403//Homo sapiens formin binding protein 21 mRNA, complete cds//1.6e-175:841:97//Hs.28307:AF071185
F-NT2RP3000418//Human Line-1 repeat mRNA with 2 open reading frames//2.7e-33:610:65//Hs.23094:M19503
F-NT2RP3000433//ESTs//1.5e-32:246:69//Hs.120892:AA724948
F-NT2RP3000439//Adenosine A2b receptor//0.44:210:62//Hs.45743:X68487
F-NT2RP3000441
F-NT2RP3000449//ESTs//0.60:177:64//Hs.132605:AI051562
F-NT2RP3000451//Receptor protein-tyrosine kinase EDDR1//0.95:315:58//Hs.75562:U48705
F-NT2RP3000456//ESTs//7.5e-23:140:92//Hs.5209:AA780068
F-NT2RP3000484//EST//2.5e-06:166:67//Hs.149950:AI289822
F-NT2RP3000487//ESTs//1.2e-63:311:98//Hs.143304:AI084058
F-NT2RP3000512//Homeo box B3//3.1e-18:109:97//Hs.49931:X16667
F-NT2RP3000526//ESTs//3.7e-74:424:93//Hs.42991:N21379
F-NT2RP3000527//Human mRNA for KIAA0211 gene, complete cds//8.0e-36:706:63//Hs.79347:D86966
F-NT2RP3000531//ESTs//9.6e-75:392:95//Hs.144148:H08308
F-NT2RP3000542//ESTs//3.2e-88:448:96//Hs.30622:AA486412
F-NT2RP3000561//EST//0.88:92:64//Hs.148290:AA908404
F-NT2RP3000562//ESTs//1.1e-112:522:99//Hs.125153:AA453723
F-NT2RP3000578
F-NT2RP3000582//ESTs//2.1e-82:413:97//Hs.118544:R17277
F-NT2RP3000584
F-NT2RP3000590//ESTs//1.0:134:64//Hs.12969:N56904
F-NT2RP3000592//Paired basic amino acid cleaving system 4//3.4e-05:502:57//Hs.77234:AB001914
F-NT2RP3000596//ESTs//6.8e-71:361:95//Hs.118741:AA179811
F-NT2RP3000599//ESTs, Weakly similar to T19B10.6 [C.elegans]//9.3e-61:355:92//Hs.114622:AA693492
F-NT2RP3000603//Human mRNA for KIAA0227 gene, partial cds//6.3e-10:553:59//Hs.79170:D86980
F-NT2RP3000605//ESTs//5.8e-51:283:94//Hs.127152:AI421203
F-NT2RP3000622//ESTs//1.7e-10:72:98//Hs.155360:AA984683
F-NT2RP3000624//64 KD AUTOANTIGEN D1//0.99:194:61//Hs.79386:X54162
F-NT2RP3000628//ESTs//0.96:221:61//Hs.131161:AI017333
F-NT2RP3000632//ESTs//4.4e-53:244:77//Hs.143010:AA767904
F-NT2RP3000644//Small inducible cytokine A5 (RANTES)//3.0e-49:343:84//Hs.155464:AF088219
F-NT2RP3000661
F-NT2RP3000665//Homo sapiens putative transcription factor CA150 mRNA, complete cds//0.62:305:59//Hs.13063:AF017789
F-NT2RP3000685
F-NT2RP3000690//EST//1.0:149:64//Hs.140263:AA709001
F-NT2RP3000736//ESTs//5.3e-26:146:97//Hs.98613:D83884
F-NT2RP3000739//ESTs//0.0046:66:87//Hs.6880:W26854
F-NT2RP3000742//ESTs//5.5e-08:311:61//Hs.152224:AI369426
F-NT2RP3000753//ESTs//2.6e-63:318:97//Hs.153000:AA777765
F-NT2RP3000759//Homo sapiens mRNA for follistain-related protein (FRP), complete cds//1.6e-38:245:91//Hs.2427:D89937
F-NT2RP3000815
F-NT2RP3000825//EST//1.0:220:61//Hs.135944:N45132
F-NT2RP3000826//Homo sapiens deltex (Dx) mRNA, complete cds//0.00040:263:65//Hs.124024:AF053700
F-NT2RP3000836//ESTs, Highly similar to CLATHRIN COAT ASSEMBLY PROTEIN AP47 HOMOLOG 2 [H.sapiens]//1.1e-71:363:96//Hs.23803:AA126476
F-NT2RP3000841//EST//0.36:224:60//Hs.162094:AA524012
F-NT2RP3000845//H.sapiens mRNA for serine/threonine protein kinase EMK//6.5e-48:593:68//Hs.157199:X97630
F-NT2RP3000847//ESTs//0.0028:56:92//Hs.116406:AA209520
F-NT2RP3000850//Small inducible cytokine A5 (RANTES)//2.0e-49:323:86//Hs.155464:AF088219
F-NT2RP3000852
F-NT2RP3000859//ESTs//0.39:169:62//Hs.148948:AA699918
F-NT2RP3000865//EST//0.15:236:62//Hs.123366:AA811476
F-NT2RP3000868//Human ovarian cancer downregulated myosin heavy chain homolog (Doc1) mRNA, complete cds//6.4e-31:766:60//HS.15432:U53445
F-NT2RP3000869//Human plectin (PLEC1) mRNA, complete cds//1.1e-13:701:60//Hs.79706:U53204
F-NT2RP3000875
F-NT2RP3000901//ESTs//8.2e-26:191:87//Hs.18793:R99101
F-NT2RP3000904//EST//2.4e-49:240:100//Hs.160842:AI348374
F-NT2RP3000917
F-NT2RP3000919//MAP KINASE PHOSPHATASE-1//0.19:340:60//Hs.109895:X68277
F-NT2RP3000968//40S RIBOSOMAL PROTEIN S15A//7.7e-44:351:83//Hs.2953:X84407
F-NT2RP3000980//ESTs//6.5e-10:102:81//Hs.86950:AI204212
F-NT2RP3000994//ESTs//4.1e-120:571:98//Hs.127295:AA918411
F-NT2RP3001004//ESTs//1.1e-76:438:88//Hs.144554:N92198
F-NT2RP3001007
F-NT2RP3001055//ESTs, Weakly similar to weak similarity to procollagen alpha chain 1(V) chain [C.elegans]//2.9e-121:588:98//Hs.128781:AA160707
F-NT2RP3001057//ESTs, Highly similar to ZINC FINGER PROTEIN 45 [Homo sapiens]//9.8e-54:282:97//Hs.30303:AI244662
F-NT2RP3001081//Homo sapiens RCC1-like G exchanging factor RLG mRNA, complete cds//2.7e-51:534:74//Hs.27007:AF060219
F-NT2RP3001084//Homo sapiens mRNA for KIAA0782 protein, partial cds//3.7e-16:474:60//Hs.21264:AB018325
F-NT2RP3001096//Homo sapiens mRNA for cartilage-associated protein (CASP)//4.4e-16:428:60//Hs.155481:AJ006470
F-NT2RP3001107//Human mRNA for KIAA0215 gene, complete cds//2.8e-34:712:64//Hs.82292:D86969
F-NT2RP3001109//ESTs//1.2e-67:323:99//Hs.134734:AI337050
F-NT2RP3001111
F-NT2RP3001113//EST//1.1e-33:173:99//Hs.112640:AA609088
F-NT2RP3001115//EST//1.3e-22:122:100//Hs.162990:AA688023
F-NT2RP3001116//ESTs//1.1e-15:93:98//Hs.58412:W74779
F-NT2RP3001119//Homo sapiens BC-2 protein mRNA, complete cds//0.96:258:61//Hs.12107:AF042384
F-NT2RP3001120//Zinc finger protein 136 (clone pHZ-20)//2.4e-77:687:75//Hs.69740:U09367
F-NT2RP3001126//Homo sapiens mRNA for KIAA0775 protein, complete cds//0.00018:341:60//Hs.94790:AB018318
F-NT2RP3001133//Homeo box A4//0.00011:484:59//Hs.77637:M74297
F-NT2RP3001140//Homo sapiens mRNA for KIAA0762 protein, partial cds//1.1e-180:851:98//Hs.5378:AB018305
F-NT2RP3001147
F-NT2RP3001150//PUTATIVE TACHYKININ RECEPTOR//0.97:257:59//Hs.957:M84605
F-NT2RP3001155//Homo sapiens mRNA for AND-1 protein//1.7e-191:891:98//Hs.72160:AJ006266
F-NT2RP3001176
F-NT2RP3001214//EST//0.88:218:60//Hs.161147:AI417859
F-NT2RP3001216//ESTs//1.5e-66:340:96//Hs.105994:W19981
F-NT2RP3001221//ESTs, Weakly similar to M05D6.7 [C.elegans]//1.7e-97:512:95//Hs.103816:AA130866
F-NT2RP3001232//EST//0.0016:116:71//Hs.136498:AA594010
F-NT2RP3001236//ESTs//3.7e-97:455:99//Hs.157488:AI362756
F-NT2RP3001239//MICROTUBULE-ASSOCIATED PROTEIN 1B//1.7e-20:501:62//Hs.103042:L06237
F-NT2RP3001245//ESTs//7.1e-80:434:93//Hs.22587:AA743132
F-NT2RP3001253//Human prepromultimerin mRNA, complete cds//0.99:293:60//Hs.32934:U27109
F-NT2RP3001260//Homo sapiens mRNA for KIAA0726 protein, complete cds//1.2e-48:761:64//Hs.107809:AB018269
F-NT2RP3001268//Zinc finger protein 45 (a Kruppel-associated box (KRAB) domain polypeptide)//1.2e-42:454:72//Hs.41728:L75847
F-NT2RP3001272//ESTs//5.0e-21:162:87//Hs.69149:AA102566
F-NT2RP3001274
F-NT2RP3001281//ESTs//2.1e-39:186:73//Hs.161662:AA836811
F-NT2RP3001297//Human mRNA for KIAA0281 gene, complete cds//2.4e-48:544:69//Hs.31463:D87457
F-NT2RP3001307//Human homeodomain protein (Prox 1) mRNA, complete cds//0.72:151:68//Hs.159437:U44060
F-NT2RP3001318//Amylo-1,6-glucosidase, 4-alpha-glucanotransferase (glycogen debranching enzyme, glycogen storage disease type III)//0.012:522:56//Hs.904:U84010
F-NT2RP3001325//ESTs//2.9e-80:396:97//Hs.99838:AA204731
F-NT2RP3001338//Human mRNA for KIAA0211 gene, complete cds//1.6e-30:345:73//Hs.79347:D86966
F-NT2RP3001339//Homo sapiens mRNA for KIAA0451 protein, complete cds//6.3e-67:559:80//Hs.18586:AB007920
F-NT2RP3001340//Homo sapiens hyperpolarization-activated channel 1 (IH1) mRNA, partial cds//0.00019:473:61//Hs.124161:AF065164
F-NT2RP3001355//ESTs, Weakly similar to ADP,ATP CARRIER PROTEIN, LIVER ISOFORM T2 [H.sapiens]//1.1e-81:421:96//Hs.32508:H29831
F-NT2RP3001356//Homo sapiens Nck-2 (NCK2) mRNA, complete cds//0.15:313:60//Hs.129725:AF047487
F-NT2RP3001374//ESTs//0.98:269:59//Hs.125303:AA873022
F-NT2RP3001383//Homo sapiens mRNA for Sck, partial cds//0.73:173:65//Hs.30965:AB001451
F-NT2RP3001384//Homa sapiens mRNA for HRIHFB2018, partial cds//2.1e-158:743:98//Hs.146214:AB015332
F-NT2RP3001392//ESTs//0.013:246:63//Hs.95111:AA514595
F-NT2RP3001396//ESTs//5.6e-16:141:85//Hs.97664:H10783
F-NT2RP3001398//Zinc finger protein 45 (a Kruppel-associated box (KRAB) domain polypeptide)//1.0e-05:189:66//Hs.41728:L75847
F-NT2RP3001399//Homo sapiens mitochondrial citrate transport protein (CTP) mRNA, 3' end//0.77:132:66//Hs.111024:L77567
F-NT2RP3001407//EST//0.015:167:65//Hs.42217:H96658
F-NT2RP3001420//ESTs//1.0:214:60//Hs.91226:AA649047
F-NT2RP3001426
F-NT2RP3001427
F-NT2RP3001428//Neurotrophic tyrosine kinase, receptor, type 1//1.8e-73:431:91//Hs.85844:X66397
F-NT2RP3001432//ESTs, Moderately similar to !!!! ALU SUBFAMILY SX WARNING ENTRY !!!! [H.sapiens]//6.9e-05:195:65//Hs.115868:AA568393
F-NT2RP3001447
F-NT2RP3001449//RYANODINE RECEPTOR, SKELETAL MUSCLE//0.00033:187:68//Hs.89631:U48508
F-NT2RP3001453//ESTs//0.020:260:60//Hs.97882:AA203212
F-NT2RP3001457//ESTs//9.4e-29:165:94//Hs.71749:AA988323
F-NT2RP3001459
F-NT2RP3001472//Homo sapiens Sox-like transcriptional factor mRNA, complete cds//4.2e-10:168:70//Hs.32317:AF072836
F-NT2RP3001490//ESTs//3.1e-35:198:94//Hs.163665:AA250877
F-NT2RP3001495//ESTs//2.5e-47:239:98//Hs.128045:AA970231
F-NT2RP3001497//Homo sapiens multiple membrane spanning receptor TRC8 (TRC8) mRNA, complete cds//2.8e-172:804:98//Hs.28285:AF064801
F-NT2RP3001527//Human lymphoid-specific SP100 homolog (LYSP100-B) mRNA, complete cds//9.4e-139:743:91//Hs.85283:U36500
F-NT2RP3001529//ESTs, Moderately similar to topoisomerase IC-terminal fragment [H.sapiens]//0.28:224:65//Hs.105912:AI431328
F-NT2RP3001538//ESTs//4.1e-05:139:71//Hs.148425:AI198074
F-NT2RP3001554//Microtubule-associated protein 1A//9.8e-16:327:64//Hs.147918:U38291
F-NT2RP3001580//Insulin-like growth factor binding protein 2//1.9e-06:426:59//Hs.162:X16302
F-NT2RP3001587//Guanine nucleotide binding protein (G protein), alpha 11 (Gq class)//0.049:185:65//Hs.1686:M69013
F-NT2RP3001589//Human mRNA for tryptophan hydroxylase (EC 1.14.16.4)//9.6e-51:345:82//Hs.144563:AF057280
F-NT2RP3001607//ESTs//1.3e-07:299:63//Hs.43231:N22688
F-NT2RP3001608//ESTs//5.7e-14:85:98//Hs.161133:AI091349
F-NT2RP3001621//ESTs//1.6e-106:310:96//Hs.128505:AA306435
F-NT2RP3001629
F-NT2RP3001634//Homo sapiens TRIAD1 type I mRNA, complete cds//1.4e-62:276:97//Hs.9899:AF099149
F-NT2RP3001642//ESTs//1.0:148:63//Hs.159495:T70173
F-NT2RP3001646
F-NT2RP3001671//Homo sapiens mRNA for NS1-binding protein (NS1-BP)//1.1e-172:816:98//Hs.159597:AJ012449
F-NT2RP3001672//ESTs//5.0e-16:138:82//Hs.151864:T69027
F-NT2RP3001676//ESTs, Highly similar to GTP-BINDING PROTEIN LEPA [Pseudomonas fluorescens]//9.0e-53:375:85//Hs.41127:AA555184
F-NT2RP3001678//Human mRNA for KIAA0233 gene, complete cds//0.21:321:65//Hs.79077:D87071
F-NT2RP3001679//ESTs, Highly similar to HYPOTHETICAL 68.7 KD PROTEIN ZK757.1 IN CHROMOSOME III [Caenorhabditis elegans]//4.0e-111:518:99//Hs.20364:AI420022
F-NT2RP3001688//Homo sapiens mRNA expressed in thyroid gland//1.0:230:63//Hs.7486:D83198
F-NT2RP3001690//EST//0.15:291:59//Hs.162336:AA564329
F-NT2RP3001698//ESTs//0.24:134:69//Hs.129551:AA885219
F-NT2RP3001708//ESTs, Weakly similar to TWISTED GASTRULATION PROTEIN PRECURSOR [D.melanogaster]//1.4e-31:191:94//Hs.131279:AA486291
F-NT2RP3001712//Human SLP-76 associated protein mRNA, complete cds//0.41:259:59//Hs.58435:AF001862
F-NT2RP3001716//ESTs, Highly similar to BONE MORPHOGENETIC PROTEIN 1 PRECURSOR [Mus musculus]//7.6e-159:747:98//Hs.6823:W18181
F-NT2RP3001724//Homo sapiens chromodomain-helicase-DNA-binding protein mRNA, complete cds//4.4e-161:565:97//Hs.159273:AF054177
F-NT2RP3001727//ESTs, Highly similar to HYPOTHETICAL 37.7 KD PROTEIN ZK686.3 IN CHROMOSOME III [Caenorhabditis elegans]//3.5e-116:554:98//Hs.144332:AA046836
F-NT2RP3001730//Human mRNA for KIAA0128 gene, partial cds//1.3e-105:811:78//Hs.90998:D50918
F-NT2RP3001739
F-NT2RP3001752//ELK1, member of ETS oncogene family//7.2e-35:299:80//Hs.116549:AL009172
F-NT2RP3001753//Human putative cerebral cortex transcriptional regulator T-Brain-1 (Tbr-1) mRNA, complete cds//0.10:528:56//Hs.22138:U49250
F-NT2RP3001764//Human protein-tyrosine phosphatase mRNA, complete cds//2.4e-47:725:64//Hs.41688:U27193
F-NT2RP3001777//Human eukaryotic translation initiation factor (eIF3) mRNA, complete cds//0.42:198:61//Hs.57783:U78525
F-NT2RP3001782//Homo sapiens mRNA for KIAA0459 protein, partial cds//9.1e-153:710:98//Hs.28169:AB007928
F-NT2RP3001792//Human M4 protein mRNA, complete cds//5.6e-27:358:69//Hs.79024:L03532
F-NT2RP3001799//ESTs//0.0088:178:64//Hs.134938:AI091361
F-NT2RP3001819//Collagen, type IX, alpha 3//0.026:530:58//Hs.53563:L41162
F-NT2RP3001844//Homo sapiens mRNA for hair keratin acidic 3-II//0.90:379:58//Hs.32950:X82634
F-NT2RP3001854//ESTs//1.5e-100:501:96//Hs.72217:AA166729
F-NT2RP3001855//Human homeobox-containing protein mRNA, complete cds//7.8e-35:481:67//Hs.158225:U68727
F-NT2RP3001857//ESTs//2.7e-85:414:98//Hs.151001:AA564706
F-NT2RP3001896//ESTs, Weakly similar to F20D12.3 gene product [C.elegans]//2.9e-94:452:98//Hs.54952:AA872675
F-NT2RP3001898//Homo sapiens mRNA for synaptogyrin 1a//0.65:245:61//Hs.6139:AL022326
F-NT2RP3001915//ESTs//1.1e-83:397:99//Hs.157125:AA723896
F-NT2RP3001926//EST//0.53:362:57//Hs.127917:AA969185
F-NT2RP3001929//ESTs//7.4e-16:141:82//Hs.138852:AA284247
F-NT2RP3001931
F-NT2RP3001938//Cyclin-dependent kinase inhibitor 1C (p57, Kip2)//0.0022:268:61//Hs.106070:U22398
F-NT2RP3001943//Homo sapiens mRNA for KIAA0675 protein, complete cds//5.8e-167:815:96//Hs.15869:AB014575
F-NT2RP3001944//ESTs//0.00052:60:91//Hs.131731:AI339335
F-NT2RP3001969
F-NT2RP3001989//EST//0.00016:263:63//Hs.144096:AI032180
F-NT2RP3002002//Small inducible cytokine A5 (RANTES)//4.0e-61:293:83//Hs.155464:AF088219
F-NT2RP3002004//H.sapiens mRNA for FAST kinase//5.2e-28:104:100//Hs.75087:X86779
F-NT2RP3002007//ESTs//0.025:88:69//Hs.163310:AA856946
F-NT2RP3002014//ESTs//4.8e-70:291:98//Hs.123693:AA283821
F-NT2RP3002033//Homo sapiens mRNA for HYA22, complete cds//0.021:175:67//Hs.147189:D88153
F-NT2RP3002045//ESTs, Highly similar to ALPHA-ADAPTIN [M.musculus]//3.8e-48:353:81//Hs.127507:AA993745
F-NT2RP3002054//ESTs, Weakly similar to KIAA0319 [H.sapiens]//3.0e-25:212:83//Hs.71622:AA195155
F-NT2RP3002056//ESTs, Highly similar to RETINOBLASTOMA BINDING PROTEIN 1 [Homo sapiens]//4.2e-82:407:97//Hs.131888:AI091806
F-NT2RP3002057//Human Line-1 repeat mRNA with 2 open reading frames//3.7e-21:168:85//Hs.23094:M19503
F-NT2RP3002062//EST//0.46:198:62//Hs.157711:AI359710
F-NT2RP3002063//Membrane metallo-endopeptidase (neutral endopeptidase, enkephalinase, CALLA, CD10)//0.91:194:65//Hs.1298:J03779
F-NT2RP3002081
F-NT2RP3002097//Homo sapiens proline and glutamic acid rich nuclear protein isoform mRNA partial cds//0.073:297:61//Hs.102732:U88153
F-NT2RP3002102//EST//2.8e-16:237:67//Hs.136255:T70256
F-NT2RP3002108
F-NT2RP3002142//ESTs//4.3e-138:654:98//Hs.5729:AA306018
F-NT2RP3002146//H.sapiens mRNA for RanGTPase activating protein 1//0.27:276:62//Hs.5923:X82260
F-NT2RP3002147//Human DNA sequence from clone 431H6 on chromosome 16. Contains a novel gene with some homology to mouse HN1 (Hematological and Neurological expressed sequence 1) downstream of a putative CpG island. Contains ESTs and GSSs//6.0e-51:204:99//Hs.107256:AL031009
F-NT2RP3002151//G1 to S phase transition 1//2.6e-37:292:81//Hs.2707:X17644
F-NT2RP3002163//Human DNA fragmentation factor-45 mRNA, complete cds//0.46:224:60//Hs.155344:U91985
F-NT2RP3002165//ESTs, Highly similar to TRANSCRIPTIONAL REGULATOR PROTEIN HCNGP [Mus musculus]//3.0e-61:340:93//Hs.11379:AA594140
F-NT2RP3002166//EST//0.039:114:69//Hs.140335:AA737046
F-NT2RP3002173//ESTs, Weakly similar to HYPOTHETICAL 92.1 KD PROTEIN ZK1098.3 IN CHROMOSOME III [Caenorhabditis elegans]//4.0e-39:255:72//Hs.141429:AA631915
F-NT2RP3002181//ESTs//3.6e-111:518:99//Hs.128505:AA30643
F-NT2RP3002244//Myosin, heavy polypeptide 6, cardiac muscle, alpha (cardiomyopathy, hypertrophic1)//0.98:242:57//Hs.114001:Z20656
F-NT2RP3002248
F-NT2RP3002255//ESTs//8.4e-19:227:75//Hs.122817:AA772261
F-NT2RP3002273//Homo sapiens homeobox protein A10 (HOXA10) gene, complete cds//0.42:189:62//Hs.110637:AC004080
F-NT2RP3002276//ESTs//8.2e-97:463:98//Hs.45120:AA225139
F-NT2RP3002303//ESTs//7.1e-10:96:87//Hs.135700:AA989386
F-NT2RP3002304//Protein phosphatase 1, catalytic subunit, beta isoform//1.3e-05:496:60//Hs.21537:X80910
F-NT2RP3002330//ESTs//1.3e-81:482:90//Hs.121460:AA744871
F-NT2RP3002343//Homo sapiens potassium channel mRNA, complete cds//0.30:462:56//Hs.143624:AF033383
F-NT2RP3002351//NAD-DEPENDENT METHYLENETETRAHYDROFOLATE DEHYDROGENASE//1.6e-65:588:75//Hs.154672:X16396
F-NT2RP3002352//Homo sapiens mRNA for protein encoded by cxorf5 (71-7A) gene//4.2e-166:770:98//Hs.6483:Y16355
F-NT2RP3002377//Homo sapiens mRNA for KIAA0788 protein, partial cds//7.5e-161:911:89//Hs.2397:Z70200
F-NT2RP3002399
F-NT2RP3002402//ESTs, Weakly similar to F02E9.6 [C.elegans]//4.3e-41:233:94//Hs.22880:AA056274
F-NT2RP3002455//Homo sapiens mRNA for KIAA0678 protein, partial cds//3.9e-140:649:99//Hs.12707:AB014578
F-NT2RP3002484//ESTs//0.95:166:63//Hs.149993:AI291310
F-NT2RP3002501//ESTs//0.92:43:90//Hs.119314:AA432108
F-NT2RP3002512//Homo sapiens mRNA for KIAA0466 protein, partial cds//1.0:173:61//Hs.81234:AB007935
F-NT2RP3002529//Human vacuolar protein sorting homolog h-vps45 mRNA, complete cds//4.4e-146:763:93//Hs.57738:U35246
F-NT2RP3002545//Homo sapiens mRNA for KIAA0729 protein, partial cds//5.9e-180:833:98//Hs.19542:AB018272
F-NT2RP3002549//ESTs, Weakly similar to POLYPOSIS LOCUS PROTEIN 1 [H.sapiens]//1.3e-42:510:70//Hs.96759:AA469984
F-NT2RP3002566//Carnitine acetyltransferase//0.032:226:62//Hs.12068:X78706
F-NT2RP3002587//EST//4.8e-31:330:74//Hs.139415:AA426054
F-NT2RP3002590//EST//1.3e-40:202:100//Hs.144716:AI187919
F-NT2RP3002602//RYANODINE RECEPTOR, SKELETAL MUSCLE//1.3e-06:280:63//Hs.89631:U48508
F-NT2RP3002603
F-NT2RP3002628//Homo sapiens mRNA for MSJ-1, complete cds//1.5e-05:264:61//Hs.3845:AB014888
F-NT2RP3002631//Homo sapiens ADAM 21 mRNA, partial cds//0.97:320:58//Hs.121287:AF029900
F-NT2RP3002650//Homo sapiens mRNA for cartilage-associated protein (CASP)//2.6e-13:441:63//Hs.155481:AJ006470
F-NT2RP3002659//Human TAR RNA loop binding protein (TRP-185) mRNA, complete cds//1.7e-05:615:58//Hs.151518:U38847
F-NT2RP3002660//ESTs//2.9e-32:164:100//Hs.152982:AA584308
F-NT2RP3002663//ESTs, Highly similar to OXYSTEROL-BINDING PROTEIN [Homo sapiens]//4.1e-38:493:70//Hs.41086:AI337400
F-NT2RP3002671//ESTs//3.7e-05:288:59//Hs.161359:AI421991
F-NT2RP3002682//ESTs, Weakly similar to F17C11.8 [C.elegans]//1.6e-61:294:100//Hs.128750:AI367584
F-NT2RP3002687
F-NT2RP3002688//EST//1.0:312:58//Hs.156800:AI352200
F-NT2RP3002701//EST//0.00083:55:87//Hs.159750:AI393657
F-NT2RP3002713//ESTs//0.93:229:61//Hs.150459:AI279514
F-NT2RP3002763//ESTs//1.7e-97:419:96//Hs.121593:W86291
F-NT2RP3002770//Homo sapiens G protein-coupled receptor kinase 6 (GRK6) gene, partial cds//0.91:161:62//Hs.129736:AF040753
F-NT2RP3002785
F-NT2RP3002799//EST//1.7e-17:199:73//Hs.118694:AA148713
F-NT2RP3002810//ESTs, Weakly similar to KIAA0062 [H.sapiens]//1.4e-76:423:93//Hs.41068:AA844350
F-NT2RP3002818//Homo sapiens jerky gene product homolog mRNA, complete cds//2.2e-55:615:70//Hs.105940:AF004715
F-NT2RP3002861//ESTs//1.1e-88:468:94//Hs.159821:AA524070
F-NT2RP3002869//ESTs//3.4e-23:132:97//Hs.148873:T33582
F-NT2RP3002876//Homo sapiens mRNA for B120, complete cds//2.7e-90:557:88//Hs.123090:AB001895
F-NT2RP3002877//ESTs//1.1e-19:160:84//Hs.118273:AA626040
F-NT2RP3002909//Homo sapiens mRNA for KIAA0771 protein, partial cds//1.8e-181:853:98//Hs.6162:AB018314
F-NT2RP3002911//ESTs//2.8e-07:160:70//Hs.140402:AI138765
F-NT2RP3002948//ESTs, Highly similar to RING CANAL PROTEIN [Drosophila melanogaster]//1.4e-133:645:97//Hs.3826:U69560
F-NT2RP3002953//Homo sapiens mRNA for KIAA0588 protein, complete cds//5.2e-13:594:57//Hs.74599:AB011160
F-NT2RP3002955//Homo sapiens mRNA for KIAA0719 protein, complete cds//0.76:412:57//Hs.21198:AB018262
F-NT2RP3002969//EST//3.7e-50:272:94//Hs.162331:AA563870
F-NT2RP3002972//Homo sapiens PAC clone DJ130H16 from 22q12.1-qter//5.1e-35:361:75//Hs.8003:AC004997
F-NT2RP3002978//ESTs//2.8e-46:253:95//Hs.151924:AI287703
F-NT2RP3002985//Human TFIIB related factor hBRF (HBRF) mRNA, complete cds//0.071:550:58//Hs.32935:U28838
F-NT2RP3002988//EST//0.0016:180:63//Hs.147632:AI218308
F-NT2RP3003008//Human DNA-binding protein (HRC1) mRNA, complete cds//0.59:201:63//Hs.72925:M91083
F-NT2RP3003032//ESTs//9.1e-40:241:92//Hs.113363:C06446
F-NT2RP3003059//ESTs//0.0015:399:58//Hs.136895:AA897749
F-NT2RP3003061//Ankyrin 1, erythrocytic//4.5e-14:633:59//Hs.1242:X16609
F-NT2RP3003068//EST//0.00014:80:83//Hs.121993:AA777928
F-NT2RP3003071//ESTs//1.1e-62:315:98//Hs.16141:W56079
F-NT2RP3003078
F-NT2RP3003101
F-NT2RP3003121//EST, Moderately similar to !!!! ALU SUBFAMILY SC WARNING ENTRY !!!! [H.sapiens]//0.98:88:68//Hs.99715:AA292700
F-NT2RP3003133//EST//8.0e-17:218:68//Hs.134815:AI090740
F-NT2RP3003138//Homo sapiens vasopressin-activated calcium mobilizing putative receptor protein (VACM-1) mRNA, complete cds//0.013:438:57//Hs.101299:AF017061
F-NT2RP3003139//ESTs//0.020:260:61//Hs.59142:W88975
F-NT2RP3003145//Homo sapiens aortic carboxypeptidase-like protein ACLP mRNA, complete cds//2.2e-20:430:63//Hs.118397:AF053944
F-NT2RP3003150
F-NT2RP3003157//Human repressor transcriptional factor (ZNF85) mRNA, complete cds//2.0e-72:894:68//Hs.37138:U35376
F-NT2RP3003185//Homo sapiens mRNA for KIAA0521 protein, partial cds//0.045:410:59//Hs.6150:AB011093
F-NT2RP3003193//Zinc finger protein 10 (KOX 1)//2.4e-74:737:71//Hs.2479:X78933
F-NT2RP3003197//ESTs//1.8e-24:130:100//Hs.162504:AA668211
F-NT2RP3003203//ESTs//3.5e-30:232:82//Hs.6880:W26854
F-NT2RP3003204//ESTs//3.1e-109:524:98//Hs.152982:AA584308
F-NT2RP3003210//ESTs//3.6e-16:113:91//Hs.121030:AA625325
F-NT2RP3003212//EST//1.0e-52:500:74//Hs.161635:W22525
F-NT2RP3003230//Human mRNA for actin binding protein p57, complete cds//6.0e-55:587:70//Hs.109606:D44497
F-NT2RP3003242//Homo sapiens stanniocalcin-2 (STC-2) mRNA, complete cds//1.2e-129:617:98//Hs.155223:AF055460
F-NT2RP3003251//H.sapiens Staf50 mRNA//1.1e-68:651:76//Hs.68054:X82200
F-NT2Rp3003264//Human bullous 230 kDa pemphigoid antigen (BPAG1) mRNA, complete cds//0.069:382:59//Hs.620:M69225
F-NT2RP3003278//Homo sapiens hook2 protein (HOOK2) mRNA, complete cds//0.98:261:59//Hs.30792:AF044924
F-NT2RP3003282//Homo sapiens dynamin (DNM) mRNA, complete cds//4.2e-133:694:93//Hs.11702:L36983
F-NT2RP3003290//Human mRNA for RTP, complete cds//6.3e-66:662:71//Hs.75789:D87953
F-NT2RP3003301//EST//1.0:58:74//Hs.158575:AI368947
F-NT2RP3003302//Human Line-1 repeat mRNA with 2 open reading frames//3.1e-91:681:80//Hs.23094:M19503
F-NT2RP3003311//ESTs//0.95:308:59//Hs.27308:AA534947
F-NT2RP3003313//ESTs//0.0016:345:61//Hs.143304:AI084058
F-NT2RP3003327//H.sapiens Staf50 mRNA//8.0e-31:253:67//Hs.68054:X82200
F-NT2RP3003330
F-NT2RP3003344
F-NT2RP3003346//H.sapiens mRNA for delta 4-3-oxosteroid 5 beta-reductase//1.2e-42:644:66//Hs.2638:Z28339
F-NT2RP3003353//Breast cancer 1, early onset//0.30:145:67//Hs.66746:L78833
F-NT2RP3003377//Human mRNA for cadherin-15, complete cds//0.019:416:60//Hs.148090:D83542
F-NT2RP3003384//ESTs//1.1e-65:346:96//Hs.35012:R92791
F-NT2RP3003385//ESTs, Highly similar to SKD3 [M.musculus]//7.0e-74:384:96//Hs.21263:H16363
F-NT2RP3003403//ESTs//4.9e-12:335:63//Hs.87258:AA463850
F-NT2RP3003409//Human DHHC-domain-containing cysteine-rich protein mRNA, complete cds//3.2e-22:430:63//Hs.113272:U90653
F-NT2RP3003411//Human metallothionein-Ie gene (hMT-Ie)//0.99:116:62//Hs.74170:M10942
F-NT2RP3003427//ESTs//0.24:447:61//Hs.160907:AI422830
F-NT2RP3003433//Protein tyrosine phosphatase, non-receptor type 12//1.0:243:61//Hs.62:M93425
F-NT2RP3003464//Homo sapiens rab3-GAP regulatory domain mRNA, complete cds//1.7e-182:853:98//Hs.14934:AF004828
F-NT2RP3003490//Homo sapiens mRNA for KIAA0725 protein, partial cds//5.2e-175:826:98//Hs.26450:AB018268
F-NT2RP3003491//Ryanodine receptor 2 (cardiac)//1.0:148:66//Hs.90821:X98330
F-NT2RP3003500//ESTs//0.86:211:62//Hs.136037:AA013302
F-NT2RP3003543//Homo sapiens clone 23790 unknown protein mRNA, complete cds//0.64:626:58//Hs.150828:AF038169
F-NT2RP3003552
F-NT2RP3003555//ESTs//1.4e-12:81:98//Hs.144487:AI418322
F-NT2RP3003564//EST//4.5e-08:186:69//Hs.116769:AA630365
F-NT2RP3003572//EST//0.27:105:69//Hs.162134:AA526311
F-NT2RP3003576//ESTs//1.2e-57:277:84//Hs.138852:AA284247
F-NT2RP3003589//RAS-RELATED PROTEIN RAB-8//6.3e-38:373:73//Hs.123109:X56741
F-NT2RP3003621//HEPATOCYTE GROWTH FACTOR ACTIVATOR PRECURSOR//8.0e-09:564:61//Hs.104:D14012
F-NT2RP3003625
F-NT2RP3003656
F-NT2RP3003659
F-NT2RP3003665//ESTs//0.015:221:62//Hs.153705:AA527586
F-NT2RP3003672//ESTs//0.70:351:57//Hs.27633:N76184
F-NT2RP3003680//Human Bcl2, p53 binding protein Bbp/53BP2 (BBP/53BP2) mRNA, complete cds//0.013:190:63//Hs.44585:U58334
F-NT2RP3003686//Homo sapiens clone 24519 unknown mRNA, partial cds//0.69:246:62//Hs.118463:AF055000
F-NT2RP3003701//EST//0.93:79:69//Hs.145285:AI249848
F-NT2RP3003716//Homo sapiens KIAA0405 mRNA, complete cds//8.3e-24:478:61//Hs.48998:AB007865
F-NT2RP3003726//Homo sapiens mRNA for KIAA0757 protein, complete cds//7.4e-150:700:98//Hs.48513:AB018300
F-NT2RP3003746
F-NT2RP3003795//ESTs//7.1e-20:228:74//Hs.159571:AA454230
F-NT2RP3003799
F-NT2RP3003800//Gardner-Rasheed feline sarcoma viral (v-fgr) oncogene homolog//4.7e-41:432:73//Hs.1422:M19722
F-NT2RP3003805//Myosin, heavy polypeptide 6, cardiac muscle, alpha (cardiomyopathy, hypertrophic 1)//0.98:242:57//Hs.114001:Z20656
F-NT2RP3003809//Human transcription factor, forkhead related activator 4 (FREAC-4) mRNA, complete cds//5.1e-07:624:59//Hs.96028:AF042832
F-NT2RP3003819//Human ring zinc-finger protein (ZNF127-Xp) gene and 5' flanking sequence//0.84:171:63//Hs.102877:U41315
F-NT2RP3003825
F-NT2RP3003828//ESTs//2.1e-12:434:61//Hs.156864:AI346481
F-NT2RP3003831
F-NT2RP3003833//Homo sapiens clones 24718 and 24825 mRNA sequence//2.6e-48:242:98//Hs.25300:AF070611
F-NT2RP3003842//Integrin, beta 8//1.0:345:60//Hs.832:M73780
F-NT2RP3003846//Homo sapiens mRNA for KIAA0725 protein, partial cds//1.3e-37:335:68//Hs.26450:AB018268
F-NT2RP3003870//Homo sapiens mRNA for KIAA0800 protein, complete cds//1.3e-175:805:99//Hs.118738:AB018343
F-NT2RP3003876//ESTs, Highly similar to Rabin3 [R.norvegicus]//6.8e-39:243:90//Hs.124832:AA846576
F-NT2RP3003914//ESTs, Weakly similar to UDP-GLUCOSE:GLYCOPROTEIN GLUCOSYLTRANSFERASE PRECURSOR [D.melanogaster]//1.1e-107:499:99//Hs.105794:AA701659
F-NT2RP3003918//Homo sapiens VAMP-associated protein of 33 kDa (VAP-33) mRNA, complete cds//8.3e-49:404:77//Hs.9006:AF057358
F-NT2RP3003932//ESTs//0.94:278:58//Hs.15661:W02396
F-NT2RP3003989//ESTs//1.0:174:64//Hs.8095:AI359006
F-NT2RP3003992//Cyclic nucleotide gated channel (photoreceptor), cGMP gated 2 (beta)//0.00070:433:58//Hs.93909:AF042498
F-NT2RP3004013//ESTs, Moderately similar to M-phase phosphoprotein 4 [H.sapiens]//2.8e-127:617:97//Hs.142151:AA984061
F-NT2RP3004016//Human p300/CBP-associated factor (P/CAF) mRNA, complete cds//0.0086:283:62//Hs.155302:U57317
F-NT2RP3004041//EST//0.98:264:58//Hs.127552:AA953234
F-NT2RP3004051//Human mRNA for KIAA0319 gene, complete cds//7.0e-63:774:67//Hs.26441:AB002317
F-NT2RP3004070//EST//6.8e-22:163:85//Hs.132635:AI032875
F-NT2RP3004078//Regulatory factor (trans-acting) 2 (influences HLA class II expression)//5.3e-90:520:90//Hs.100007:X76091
F-NT2RP3004093
F-NT2RP3004095//Human clone 23732 mRNA, partial cds//3.3e-27:372:69//Hs.81281:U79258
F-NT2RP3004110//Human mRNA for KIAA0392 gene, partial cds//1.2e-20:211:77//Hs.40100:AB002390
F-NT2RP3004125//ESTs, Highly similar to OOCYTE ZINC FINGER PROTEIN XLCOF7.1 [Xenopus laevis]//1.0e-126:590:99//Hs.129888:AI096509
F-NT2RP3004145
F-NT2RP3004148
F-NT2RP3004155//Homo sapiens timing protein CLK-1 mRNA, complete cds//2.1e-121:578:98//Hs.157113:AF032900
F-NT2RP3004189//ESTs//1.3e-80:409:97//Hs.151001:AA564706
F-NT2RP3004206//Human mRNA for stac, complete cds//1.0:245:60//Hs.56045:D86640
F-NT2RP3004207//Transcription factor 3 (E2A immunoglobulin enhancer binding factors E12/E47)//0.095:281:62//Hs.101047:M31523
F-NT2RP3004209//ESTs//5.8e-87:458:94//Hs.155303:AI221835
F-NT2RP3004215//ESTs//0.074:56:80//Hs.163590:H43361
F-NT2RP3004242
F-NT2RP3004246//EST//0.20:219:63//Hs.161920:AA483240
F-NT2RP3004253//ESTs//1.2e-36:204:96//Hs.143588:AI149140
F-NT2RP3004258//Human gene for neurofilament subunit M (NF-M)//7.2e-07:369:59//Hs.71346:Y00067
F-NT2RP3004262//Homo sapiens heat shock protein hsp40-3 mRNA, complete cds//1.0e-154:733:98//Hs.158471:AF088982
F-NT2RP3004282//Homo sapiens torsinA (DYT1) mRNA, complete cds//4.2e-26:597:61//Hs.19261:AF007871
F-NT2RP3004332
F-NT2RP3004334//ESTs//8.8e-27:142:99//Hs.28068:H06285
F-NT2RP3004341//EST//0.0068:213:64//Hs.153208:X98426
F-NT2RP3004348//ESTs//1.2e-18:126:93//Hs.58595:AA830999
F-NT2RP3004349//ESTs, Weakly similar to HYPOTHETICAL 92.1 KD PROTEIN ZK1098.3 IN CHROMOSOME III [Caenorhabditis elegans]//3.9e-45:337:83//Hs.141429:AA631915
F-NT2RP3004378//ESTs, Weakly similar to weak similarity to procollagen alpha chain 1(V) chain [C.elegans]//4.3e-125:608:98//Hs.128781:AA160707
F-NT2RP3004399//H.sapiens mRNA for leucine-rich primary response protein 1//2.3e-141:804:90//Hs.123122:X97249
F-NT2RP3004424//ESTs, Weakly similar to JTV-1 [H.sapiens]//3.2e-122:609:96//Hs.20132:AA203113
F-NT2RP3004428//Homo sapiens ALR mRNA, complete cds//0.00044:458:60//Hs.153638:AF010403
F-NT2RP3004451//Bone morphogenetic protein 8 (osteogenic protein 2)//0.00023:357:59//Hs.99948:M97016
F-NT2RP3004454//Homo sapiens mRNA for KIAA0448 protein, complete cds//2.0e-124:583:99//Hs.27349:AB007917
F-NT2RP3004466//Homo sapiens mRNA for KIAA0664 protein, partial cds//0.48:399:58//Hs.22616:AB014564
F-NT2RP3004470//EST//1.3e-56:331:91//Hs.136830:AA769219
F-NT2RP3004472
F-NT2RP3004475//Homo sapiens mRNA for KIAA0456 protein, partial cds//9.8e-152:715:98//Hs.5003:AB007925
F-NT2RP3004480//ESTs, Highly similar to VACUOLAR SORTING PROTEIN 35 [Saccharomyces cerevisiae]//4.6e-118:547:99//Hs.124768:AA307735
F-NT2RP3004490//Homo sapiens mRNA for Musashi, complete cds//2.3e-156:752:97//Hs.158311:AB012851
F-NT2RP3004498//ESTs, Moderately similar to ROSA26AS [M.musculus]//3.5e-89:425:99//Hs.126082:AI077718
F-NT2RP3004503//EST//5.3e-49:399:81//Hs.162335:AA564256
F-NT2RP3004504//Homo sapiens mRNA for KIAA0479 protein, partial cds//1.0:370:59//Hs.158244:AB007948
F-NT2RP3004507//Human zinc finger protein (MAZ) mRNA//0.86:129:66//Hs.7647:M94046
F-NT2RP3004527//EST//0.053:260:62//Hs.123314:AA810110
F-NT2RP3004534//ESTs//3.5e-78:370:99//Hs.132808:AI031571
F-NT2RP3004539//Homo sapiens mRNA for KIAA0632 protein, partial cds//2.7e-146:679:98//Hs.75970:AB014532
F-NT2RP3004544//Homo sapiens mRNA for KIAA0554 protein, partial cds//9.1e-171:793:98//Hs.74750:AB011126
F-NT2RP3004566//ESTs, Highly similar to ZINC FINGER PROTEIN MLZ-4 [Mus musculus]//2.2e-66:362:94//Hs.125870:AI364967
F-NT2RP3004569
F-NT2RP3004572//Homo sapiens cofactor of initiator function (CIF50) mRNA, complete cds//3.3e-181:860:97//Hs.122752:AF026445
F-NT2RP3004578//Homo sapiens mRNA for KIAA0454 protein, partial cds//4.0e-85:422:97//Hs.129928:AB007923
F-NT2RP3004594//Homo sapiens mRNA for AND-1 protein//3.7e-160:796:95//Hs.72160:AJ006266
F-NT2RP3004617//ESTs, Weakly similar to estrogen-responsive finger protein, efp [H.sapiens]//6.4e-13:356:64//Hs.124138:AI266336
F-NT2RP3004618//ESTs//1.5e-42:481:70//Hs.130768:AA909232
F-NT2RP3004669//Human plectin (PLEC1) mRNA, complete cds//0.0099:538:56//Hs.79706:U53204
F-NT2RP3004670//Homo sapiens sox1 gene//0.11:311:58//Hs.144029:Y13436
F-NT2RP4000008//ESTs, Highly similar to CHLORINE CHANNEL PROTEIN P64 [Bos taurus]//8.0e-177:827:98//Hs.118991:AA675919
F-NT2RP4000023//ESTs//1-4e-33:182:96//Hs.122722:AA455668
F-NT2RP4000035//ESTs//1.1e-23:283:72//Hs.142147:AA706495
F-NT2RP4000049//Homo sapiens decoy receptor 2 mRNA, complete cds//6.8e-83:556:85//Hs.129844:AF029761
F-NT2RP4000051//Homo sapiens mRNA for cartilage-associated protein (CASP)//4.9e-13:441:62//Hs.155481:AJ006470
F-NT2RP4000078//Homo sapiens mRNA for NS1-binding protein (NS1-BP)//8.0e-151:720:97//Hs.159597:AJ012449
F-NT2RP4000102//ESTs//8.8e-33:184:82//Hs.93054:H47743
F-NT2RP4000109//Homo sapiens mRNA for MEGF5, partial cds//1.4e-167:774:99//Hs.57929:AB011538
F-NT2RP4000111
F-NT2RP4000129//Homo sapiens mRNA for KIAA0483 protein, partial cds//1.1e-115:548:98//Hs.64691:AB007952
F-NT2RP4000147//Human mRNA for KIAA0041 gene, partial cds//0.00045:212:63//Hs.75520:D26069
F-NT2RP4000150
F-NT2RP4000151//Homo sapiens chromosome 7q22 sequence//0.98:431:59//Hs.3386:AF053356
F-NT2RP4000159
F-NT2RP4000167
F-NT2RP4000185//ESTs//1.1e-51:240:68//Hs.33020:N31946
F-NT2RP4000210//Homo sapiens mRNA for KIAA0700 protein, partial cds//1.6e-175:825:98//Hs.13999:AB014600
F-NT2RP4000212//ESTs//1.6e-10:74:95//Hs.111885:AA422006
F-NT2RP4000214//ESTs//3.9e-11:225:68//Hs.59793:AA451731
F-NT2RP4000218//Human G protein-coupled receptor (STRL22) mRNA, complete cds//6.2e-34:425:71//Hs.46468:U45984
F-NT2RP4000243//Homo sapiens mRNA for cartilage-associated protein (CASP)//8.6e-158:771:97//Hs.155481:AJ006470
F-NT2RP4000246//ESTs, Highly similar to NPC DERIVED PROLINE RICH PROTEIN 1 [M.musculus]//1.9e-62:384:89//Hs.115498:AA436298
F-NT2RP4000259//Homo sapiens clone 683 unknown mRNA, complete sequence//9.4e-130:604:99//Hs.43728:AF091092
F-NT2RP4000263
F-NT2RP4000290//EST//1.0:149:63//Hs.136928:AA812580
F-NT2RP4000312//Human mRNA for KIAA0147 gene, partial cds//1.5e-42:685:63//Hs.158132:D63481
F-NT2RP4000321//Homo sapiens gene for insulin receptor substrate-2, complete cds//8.6e-05:547:57//Hs.143648:AB000732
F-NT2RP4000323//Human HCF1 gene related mRNA sequence//0.48:589:58//Hs.83634:U52112
F-NT2RP4000355
F-NT2RP4000360//Homo sapiens mRNA for KIAA0738 protein, complete cds//6.4e-142:654:99//Hs.107479:AB018281
F-NT2RP4000367//Homo sapiens IkappaB kinase complex associated protein (IKAP) mRNA, complete cds//8.5e-137:649:97//Hs.31323:AF044195
F-NT2RP4000370//ESTs, Weakly similar to MITOCHONDRIAL PEPTIDE CHAIN RELEASE FACTOR 1 PRECURSOR [S.cerevisiae]//1.2e-09:157:76//Hs.97950:AI382073
F-NT2RP4000376//1-PHOSPHATIDYLINOSITOL-4,5-BISPHOSPHATE PHOSPHODIESTERASE BETA 2//0.098:291:59//Hs.994:M95678
F-NT2RP4000381//Myosin, heavy polypeptide 7, cardiac muscle, beta//0.00025:509:59//Hs.929:M57965
F-NT2RP4000398//Zinc finger protein 140 (clone pHZ-39)//4.9e-60:469:68//Hs.154205:U09368
F-NT2RP4000415//ESTs//0.85:89:67//Hs.152312:AA485688
F-NT2RP4000417//Homo sapiens alpha 1,2-mannosidase IB mRNA, complete cds//0.014:178:66//Hs.125315:AF027156
F-NT2RP4000424//Human G protein-coupled receptor (STRL22) mRNA, complete cds//2.0e-34:431:73//Hs.46468:U45984
F-NT2RP4000448//Human mRNA for KIAA0118 gene, partial cds//1.9e-37:360:75//Hs.154326:D42087
F-NT2RP4000449//EST//0.84:113:65//Hs.145274:AI249468
F-NT2RP4000455//ALPHA-2C-1 ADRENERGIC RECEPTOR//0.063:221:61//Hs.123022:J03853
F-NT2RP4000457//H.sapiens mRNA for herpesvirus associated ubiquitin-specific protease (HAUSP)//1.1e-05:532:57//Hs.78683:Z72499
F-NT2RP4000480//Homo sapiens mRNA, complete cds//0.056:655:60//Hs.133151:AB001535
F-NT2RP4000481//Human mRNA for KIAA0268 gene, partial cds//0.46:272:58//Hs.78862:D87742
F-NT2RP4000498//Human DNA binding protein FKHL15 (FKHL15) mRNA, complete cds//0.94:133:69//Hs.159234:U89995
F-NT2RP4000500//V-myb avian myeloblastosis viral oncogene homolog-like 2//0.60:335:61//Hs.74605:X13293
F-NT2RP4000515//ESTs//2.9e-45:253:95//Hs.104898:AA429594
F-NT2RP4000517//EST//0.043:131:64//Hs.99030:AA443904
F-NT2RP4440518//Homo sapiens mRNA for ATP-dependent RNA helicase, partial//2.0e-34:203:93//Hs.99423:AJ010840
F-NT2RP4000519//Human mRNA for KIAA0374 gene, complete cds//0.33:154:66//Hs.100837:AB002372
F-NT2RP4000524
F-NT2RP4000528
F-NT2RP4000541//ESTs//2.1e-51:251:99//Hs.157240:AI348154
F-NT2RP4000556//ESTs, Highly similar to 60S RIBOSOMAL PROTEIN L11 [R.norvegicus]//1.1e-27:162:93//Hs.25597:H93026
F-NT2RP4000560//ESTs//2.5e-09:181:66//Hs.122609:AA778351
F-NT2RP4000588//ESTs//1.4e-46:533:70//Hs.8836:AA181053
F-NT2RP4000614//Homo sapiens TLS-associated protein TASR-2 mRNA, complete cds//1.0e-139:666:98//Hs.4214:AF067730
F-NT2RP4000638//Fibroblast growth factor 2 (basic)//1.0:226:61//Hs.56066:J04513
F-NT2RP4000648//ESTs//2.5e-11:116:80//Hs.115449:AA418396
F-NT2RP4000657//Homo sapiens bone morphogenetic protein 11 (BMP11) mRNA, complete cds//0.00056:367:60//Hs.144626:AF100907
F-NT2RP4000704//Homo sapiens mRNA expressed in 19week fetal lung, clone IMAGE:300856//8.0e-167:676:98//Hs.50748:AB004848
F-NT2RP4000713//Homo sapiens N-methyl-D-aspartate receptor 2D subunit precursor (NMDAR2D) mRNA, complete cds//6.9e-07:494:61//Hs.113286:U77783
F-NT2RP4000724//ESTs, Weakly similar to pol/env ORF [H.sapiens]//2.8e-46:411:78//Hs.111817:T80622
F-NT2RP4000728//Homo sapiens mRNA for KIAA0606 protein, partial cds//9.9e-43:350:71//Hs.38176:AB011178
F-NT2RP4000737//Human mRNA for KIAA0252 gene, partial cds//0.97:409:60//Hs.83419:D87440
F-NT2RP4000739//DESMOPLAKIN I AND II//0.99:192:63//Hs.74316:AL031058
F-NT2RP4000781//Homo sapiens mRNA for APC 2 protein, complete cds//0.023:351:60//Hs.20912:AB012162
F-NT2RP4000787//Human mRNA for ESP1/CRP2, complete cds//0.0051:276:58//Hs.70327:D42123
F-NT2RP4000817//Homo sapiens mRNA for KIAA0470 protein, complete cds//4.8e-176:816:98//Hs.25132:AB007939
F-NT2RP4000833//Homo sapiens PAC clone DJ0905J08 from 7p12-p14//1.3e-93:438:99//Hs.8173:AC005189
F-NT2RP4000837//Homo sapiens SALL1 gene, partial//5.9e-05:470:59//Hs.123094:X98833
F-NT2RP4000839//ESTs//5.7e-11:133:82//Hs.103852:W27603
F-NT2RP4000855//Homo sapiens DNA-binding protein (CROC-1B) mRNA, complete cds//1.4e-37:680:63//Hs.75875:U49278
F-NT2RP4000865//Zinc finger protein 136 (clone pHZ-20)//2.0e-96:415:78//Hs.69740:U09367
F-NT2RP4000878//ESTs//2.7e-16:390:63//Hs.163451:AI206803
F-NT2RP4000879//ESTs//0.89:184:64//Hs.122333:AA782843
F-NT2RP4000907//Homo sapiens BAC clone RG118D07 from 7q31//4.5e-52:933:61//Hs.3781:AC004142
F-NT2RP4000915//Homo sapiens mRNA for ZNF198 protein//3.0e-80:584:78//Hs.109526:AJ224901
F-NT2RP4000918
F-NT2RP4000925//Homo sapiens KIAA0405 mRNA, complete cds//1.9e-47:861:61//Hs.48998:AB007865
F-NT2RP4000927//ESTs//0.37:159:63//Hs.147949:AI341503
F-NT2RP4000928//Homo sapiens CDP-diacylglycerol synthase 2 (CDS2) mRNA, partial cds//1.1e-164:781:97//Hs.24812:AF069532
F-NT2RP4000929//ESTs//0.88:284:60//Hs.141317:AI281371
F-NT2RP4000955//Human mRNA for cadherin-15, complete cds//0.0019:495:58//Hs.148090:D83542
F-NT2RP4000973//Homo sapiens mRNA for MSJ-1, complete cds//1.2e-05:318:60//Hs.3845:AB014888
F-NT2RP4000975//ESTs//0.0051:345:61//Hs.143304:AI084058
F-NT2RP4000979
F-NT2RP4000984
F-NT2RP4000989//Homo sapiens Tax interaction protein 1 mRNA, partial cds//0.85:257:63//Hs.12956:U90913
F-NT2RP4000996//ESTs//4.3e-10:329:62//Hs.33085:AA258068
F-NT2RP4000997//Human plectin (PLEC1) mRNA, complete cds//1.0:218:58//Hs.79706:U53204
F-NT2RP4001004
F-NT2RP4001006//ESTs, Moderately similar to ROSA26AS [M.musculus]//7.4e-90:425:99//Hs.126082:AI077718
F-NT2RP4001010//Homo sapiens PSD-95/SAP90-associated protein-2 mRNA, partial cds//2.8e-19:689:61//Hs.113287:AF009204
F-NT2RP4001029//Human transcription factor LSF mRNA, complete cds//9.6e-84:778:74//Hs.154970:U03494
F-NT2RP4001041//Human endosome-associated protein (EEA1) mRNA, complete cds//0.95:170:64//Hs.2864:L40157
F-NT2RP4001057//EST//9.6e-05:122:72//Hs.132518:AA928157
F-NT2RP4001064//Homo sapiens mRNA for cartilage-associated protein (CASP)//7.2e-13:441:63//Hs.155481:AJ006470
F-NT2RP4001078//ESTs//1.3e-29:165:95//Hs.113817:AA702497
F-NT2RP4001079//Homo sapiens mRNA for putative Ca2⁺-transporting ATPase, partial//1.4e-131:634:98//Hs.106778:AJ010953
F-NT2RP4001080//Polypyrimidine tract binding protein (hnRNP I) {alternative products}//0.025:166:66//Hs.146459:X66975
F-NT2RP4001086//Homo sapiens mRNA for KIAA0592 protein, partial cds//1.5e-85:604:86//Hs.13273:AB011164
F-NT2RP4001095
F-NT2RP4001100//ESTs, Weakly similar to C17G10.1 [C.elegans]//1.4e-93:448:98//Hs.105837:AA536054
F-NT2RP4001117//ESTs, Highly similar to PROTEIN TRANSPORT PROTEIN SEC61 ALPHA SUBUNIT [Canis familiaris]//2.2e-26:171:92//Hs.14038:R06800
F-NT2RP4001122//Human mRNA for histone H1x, complete cds//0.99:185:66//Hs.109804:D64142
F-NT2RP4001126//ESTs, Moderately similar to The KIAA0138 gene product is novel. [H.sapiens]//5.8e-37:185:100//Hs.126925:AA931237
F-NT2RP4001138//ESTs//3.4e-09:125:77//Hs.1433 82:AA476266
F-NT2RP4001143//ESTs//1.0:282:57//Hs.157423:AI358261
F-NT2RP4001148//ESTs//0.82:206:62//Hs.129259:AA992207
F-NT2RP4001149//EST//1.3e-17:140:88//Hs.101727:H16171
F-NT2RP4001150//AXONIN-1 PRECURSOR//7.7e-07:562:59//Hs.2998:X67734
F-NT2RP4001159//EST//0.26:125:66//Hs.152092:AA377324
F-NT2RP4001174//ESTs//2.9e-103:502:98//Hs.125886:AA884264
F-NT2RP4001206//EST//0.33:125:66//Hs.152092:AA377324
F-NT2RP4001207
F-NT2RP4001210//ESTs//3.1e-95:460:97//Hs.46913:AI017636
F-NT2RP4001213//KRAB zinc finger protein {alternative products}//1.1e-45:187:74//Hs.22556:U37251
F-NT2RP4001219//ESTs//1.4e-69:352:96//Hs.116392:AA936262
F-NT2RP4001228//Homo sapiens actin binding protein MAYVEN mRNA, complete cds//7.2e-28:855:60//Hs.122967:AF059569
F-NT2RP4001235//Homo sapiens Jagged 2 mRNA, complete cds//1.0:257:59//Hs.106387:AF029778
F-NT2RP4001256//Human mRNA for KIAA0273 gene, complete cds//0.96:247:62//Hs.75899:D87463
F-NT2RP4001260//Syntrophin, alpha (dystrophin-associated protein A1, 59kD, acidic component)//0.015:246:62//Hs.31121:U40571
F-NT2RP4001274//Homo sapiens clone 24674 mRNA sequence//1.2e-06:259:64//Hs.71168:AF070578
F-NT2RP4001276//Homo sapiens CAGF9 mRNA, partial cds//7.6e-06:266:62//Hs.110826:U80736
F-NT2RP4001313//Homo sapiens mitochondrial outer membrane protein (TOM40) mRNA, nuclear gene encoding mitochondrial protein, complete cds//2.3e-31:535:65//Hs.30928:AF043250
F-NT2RP4001315//EST//9.5e-20:146:88//Hs.158755:AI375917
F-NT2RP4001336//ESTs//1.0:128:67//Hs.99598:AA603110
F-NT2RP4001339
F-NT2RP4001343
F-NT2RP4001345//Lecithin-cholesterol acyltransferase//8.0e-39;686:64//Hs.112125:M12625
F-NT2RP4001351//Human ovarian cancer downregulated myosin heavy chain homolog (Doc1) mRNA, complete cds//2.0e-31:784:62//Hs.15432:U53445
F-NT2RP4001353//Homo sapiens chromosome 7q22 sequence//0.0034:497:57//Hs.125742:AF053356
F-NT2RP4001372
F-NT2RP4001373//Homo sapiens clone Dt1P1b11 mRNA, CAG repeat region//0.43:290:58//Hs.82101:Z50194
F-NT2RP4001375
F-NT2RP4001379//TRICHOHYALIN//8.2e-05:591:58//Hs.82276:L09190
F-NT2RP4001389//EST//5.3e-27:212:84//Hs.160402:AI393918
F-NT2RP4001407//Homo sapiens mRNA for RGS5, complete cds//0.93:218:58//Hs.24950:AB008109
F-NT2RP4001414//Human mRNA for KIAA0202 gene, partial cds//6.3e-78:818:71//Hs.80712:D86957
F-NT2RP4001433//Zinc finger protein 10 (KOX 1)//1.1e-88:839:73//Hs.2479:X78933
F-NT2RP4001442
F-NT2RP4001447//Homo sapiens mRNA for KIAA0783 protein, complete cds//0.0075:218:63//Hs.41153:AB018326
F-NT2RP4001474//ESTs, Weakly similar to probable CBP3 protein homolog [C.elegans]//2.1e-90:460:96//Hs.26676:AA033997
F-NT2RP4001483//Oxoglutarate dehydrogenase (lipoamide)//8.1e-61:480:75//Hs.75533:D10523
F-NT2RP4001498//ESTs, Weakly similar to GA BINDING PROTEIN BETA-2 CHAIN [H.sapiens]//0.25:216:60//Hs.63220:AA522707
F-NT2RP4001502//ESTs//2.6e-41:206:99//Hs.159257:N40395
F-NT2RP4001507//H.sapiens mRNA for RanGTPase activating protein 1//0.51:281:61//Hs.5923:X82260
F-NT2RP4001524//ESTs, Weakly similar to F13B12.1 [C.elegans]//9.4e-30:173:94//Hs.5570:AI377863
F-NT2RP4001529//Human transcription factor LSF mRNA, complete cds//1.3e-35:329:76//Hs.154970:U03494
F-NT2RP4001547//Homo sapiens forkhead protein FREAC-2 mRNA, complete cds//0.0015:221:65//Hs.44481:U13220
F-NT2RP4001551//Human BRCA2 region, mRNA sequence CG003//0.56:428:59//Hs.30649:U50534
F-NT2RP4001555//EST//0.99:225:64//Hs.96863:AA347174
F-NT2RP4001567
F-NT2RP4001568//ESTs, Weakly similar to HYPOTHETICAL 32.6 KD PROTEIN IN MET30-CBR5 INTERGENIC REGION [Saccharomyces cerevisiae]//1.1e-54:252:83//Hs.158208:AA167836
F-NT2RP4001571//ESTs//3.0e-94:475:96//Hs.65322:AA019410
F-NT2RP4001574
F-NT2RP4001575//Homo sapiens mRNA for ARE1-like protein//1.8e-169:796:98//Hs.108826:AL031228
F-NT2RP4001592
F-NT2RP4001610//Human involucrin mRNA//0.94:462:59//Hs.157091:M13903
F-NT2RP4001614//ESTs//0.71:331:58//Hs.116533:AI343952
F-NT2RP4001634
F-NT2RP4001638//ESTs, Weakly similar to HYPOTHETICAL 117.9 KD PROTEIN IN FKH1-STH1 INTERGENIC REGION [S.cerevisiae]//8.6e-57:287:97//Hs.117439:C18436
F-NT2RP4001644//Human mRNA for MNK1, complete cds//1.7e-53:415:80//Hs.5591:AB000409
F-NT2RP4001656//ESTs, Highly similar to PHENYLALANYL-TRNA SYNTHETASE MITOCHONDRIAL PRECURSOR [Saccharomyces cerevisiae]//1.0:311:59//Hs.57969:AA203629
F-NT2RP4001677//Homo sapiens short form transcription factor C-MAF (c-maf) mRNA, complete cds//0.19:162:67//Hs.30250:AF055376
F-NT2RP4001679//Homo sapiens PYRIN (MEFV) mRNA, complete cds//2.2e-50:332:86//Hs.113283:AF018080
F-NT2RP4001696
F-NT2RP4001725//Galactokinase 1//1.0:202:63//Hs.92357:L76927
F-NT2RP4001730//Human growth/differentiation factor 1 (GDF-1) mRNA, complete cds//0.0035:247:62//Hs.92614:M62302
F-NT2RP4001739//Complement component 8, gamma polypeptide//0.74:654:56//Hs.1285:U08198
F-NT2RP4001753//Zinc finger protein 84 (HPF2)//4.5e-29:476:67//Hs.9450:M27878
F-NT2RP4001760//ESTs//1.0:411:60//Hs.108548:AA081656
F-NT2RP4001790//Homo sapiens PAC clone DJ0604G05 from 7q22-q31.1//9.1e-34:400:68//Hs.154212:AC004522
F-NT2RP4001803//Human high conductance inward rectifier potassium channel alpha subunit mRNA, complete cds//0.028:580:58//Hs.2363:L36069
F-NT2RP4001822//ESTs//3.4e-50:307:90//Hs.113509:AA132131
F-NT2RP4001823//Human faciogenital dysplasia (FGD1) mRNA, complete cds//3.1e-07:509:59//Hs.1572:U11690
F-NT2RP4001828
F-NT2RP4001838//Human mRNA for KIAA0071 gene, partial cds//6.9e-55:555:73//Hs.78398:D31888
F-NT2RP4001841//ESTs//0.99:215:60//Hs.136895:AA897749
F-NT2RP4001849//Homo sapiens mRNA for KIAA0672 protein, complete cds//5.6e-57:813:65//Hs.6336:AB014572
F-NT2RP4001861//ESTs, Weakly similar to !!!! ALU SUBFAMILY J WARNING ENTRY !!!! [H.sapiens]//4.8e-12:84:94//Hs.140232:AA705170
F-NT2RP4001889
F-NT2RP4001893//Homo sapiens BAC clone GS166A23 from 7p21//4.4e-108:535:97//Hs.15144:AC005014
F-NT2RP4001896
F-NT2RP4001901//ESTs//1.4e-50:291:93//Hs.67991:AA147848
F-NT2RP4001927
F-NT2RP4001938//ESTs, Weakly similar to ZINC FINGER PROTEIN 91 [H.sapiens]//2.8e-54:375:84//Hs.119294:AI379442
F-NT2RP4001946//EST//0.050:268:60//Hs.148341:AA921894
F-NT2RP4001950//EST//7.9e-14:336:63//Hs.112810:AA610063
F-NT2RP4001953//ESTs//0.018:206:65//Hs.130105:AA904868
F-NT2RP4001966//Human DNA sequence from clone 1052M9 on chromosome Xq25. Contains the SH2D1A gene for SH2 domain protein 1A, Duncan's disease (lymphoproliferative syndrome) (DSHP), part of a 60S Acidic Ribosomal protein 1 (RPLP1) LIKE gene and part of a mouse DOC4 LIKE gene. Contains ESTs and GSSs//1.7e-54:788:65//Hs.23796:AL022718 F-NT2RP4001975//Homo sapiens homeobox protein Six3 (SIX3) gene, complete cds//0.0019:279:65//Hs.159439:AF092047
F-NT2RP4002018//ESTs, Highly similar to RING CANAL PROTEIN [Drosophila melanogaster]//0.58:463:55//Hs.3826:U69560
F-NT2RP4002047//EST//2.5e-13:102:90//Hs.148997:AI243139
F-NT2RP4002052
F-NT2RP4002058//ESTs//5.2e-41:347:72//Hs.121961:AA777873
F-NT2RP4002071//Homo sapiens TTAGGG repeat binding factor 2 (hTRF2) mRNA, complete cds//0.97:227:60//Hs.100030:AF002999
F-NT2RP4002075
F-NT2RP4002078//ESTs, Moderately similar to zinc finger protein [H.sapiens]//1.0e-38:243:90//Hs.139115:AA325104
F-NT2RP4002081//TATA box binding protein//0.0059:310:60//Hs.1100:M55654
F-NT2RP4002083//H.sapiens Pur (pur-alpha) mRNA, complete cds//0.0015:152:70//Hs.25180:M96684
F-NT2RP4002408//Human protein kinase C-L (PRKCL) mRNA, complete cds//8.0e-10:401:59//Hs.89616:M55284
F-NT2RP4002791//Ataxin 1//1.0:215:61//Hs.74520:X79204
F-NT2RP4002888
F-NT2RP4002905//ESTs//3.4e-50:280:94//Hs.131697:H14960
F-NT2RP5003459//Glyceraldehyde-3-phosphate dehydrogenase//1.3e-35:193:96//Hs.74456:U34995
F-NT2RP5003461//ESTs//3.6e-104:513:98//Hs.88088:AA521071
F-NT2RP5003477//Eukaryotic translation initiation factor 3 (eIF-3) p36 subunit//0.18:271:60//Hs.139745:U39067
F-NT2RP5003492
F-NT2RP5003500//Homo sapiens mRNA for heparan-sulfate 6-sulfotransferase, complete cds//6.1e-56:750:69//Hs.132884:AB006179
F-NT2RP5003506//Homo sapiens Chromosome 16 BAC clone CIT987SK-A-69G12//5.1e-14:348:62//Hs.154050:AC004131
F-NT2RP5003512//Homo sapiens mRNA for KIAA0642 protein, partial cds//0.94:202:63//Hs.8152:AB014542
F-NT2RP5003522
F-NT2RP5003524//ESTs//8.7e-08:340:62//Hs.152730:AI308943
F-NT2RP5003534
F-OVARC1000001//Homo sapiens mRNA for KIAA0465 protein, partial cds//4.0e-69:373:94//Hs.108258:AB007934
F-OVARC1000004//ESTs//6.0e-38:216:93//Hs.163801:AI391729
F-OVARC1000006//ESTs, Highly similar to HISTONE H2A [Cairina moschata]//4.4e-75:355:99//Hs.36727:AI051983
F-OVARC1000013//ESTs//0.65:331:58//Hs.146326:AA534304
F-OVARC1000014//Homo sapiens GLE1 (GLE1) mRNA, complete cds//1.8e-171:815:98//Hs.81449:AF058922
F-OVARC1000017//Homo sapiens mRNA for NTAK, complete cds//0.50:482:58//Hs.113264:AB005060
F-OVARC1000035//Homo sapiens GA17 protein mRNA, complete cds//2.2e-37:238:89//Hs.69469:AF064603
F-OVARC1000058//ESTs//1.1e-23:132:97//Hs.61809:AA503549
F-OVARC1000060//ESTs, Highly similar to ribonuclease 6 precursor [H.sapiens]//6.7e-60:305:97//Hs.31696:H50008
F-OVARC1000068//ESTs//3.8e-10:69:100//Hs.89048:AA282798
F-OVARC1000071//ESTs//1.9e-36:202:95//Hs.125013:AA400543
F-OVARC1000085
F-OVARC1000087//EST//1.0:199:58//Hs.122919:AA768442
F-OVARC1000091//Homo sapiens Jagged 2 mRNA, complete . cds//0.00017:414:59//Hs.106387:AF029778
F-OVARC1000092//ESTs//4.6e-06:410:60//Hs.152250:AA203600
F-OVARC1000106//ESTs, Weakly similar to C25A1.1 [C.elegans]//2.9e-73:406:92//Hs.109463:AI205174
F-OVARC1000109
F-OVARC1000113//Homo sapiens okadaic acid-inducible phosphoprotein (OA48-18) mRNA, complete cds//5.3e-135:663:96//Hs.3688:AF069250
F-OVARC1000114//Homo sapiens mRNA for KIAA0562 protein, complete cds//3.4e-43:532:72//Hs.118401:AB011134
F-OVARC1000133//ESTs//9.4e-50:249:98//Hs.159146:AI384010
F-OVARC1000139
F-OVARC1000145//ESTs//1.6e-09:87:90//Hs.25219:AA291293
F-OVARC1000148//ESTs//4.4e-28:146:100//Hs.133223:AA677414
F-OVARC1000151
F-OVARC1000168//ESTs//2.3e-48:264:95//Hs.14539:H67305
F-OVARC1000191//Thrombopoietin (myeloproliferative leukemia virus oncogene ligand, megakaryocyte growth and development factor)//0.10:504:59//Hs.154083:U70136
F-OVARC1000198//ESTs//1.3e-103:505:97//Hs.149341:AI249131
F-OVARC1000209//EST//1.0:73:72//Hs.162600:AA594840
F-OVARC1000212//ESTs//1.7e-17:121:91//Hs.50473:W68834
F-OVARC1000240//ESTs, Highly similar to THREONYL-TRNA SYNTHETASE, CYTOPLASMIC [Homo sapiens]//2.7e-31:264:79//Hs.151895:AA196379
F-OVARC1000241//Homo sapiens clone 23698 mRNA sequence//3.4e-35:466:68//Hs.8136:U81984
F-OVARC1000288//TESTs, Weakly similar to Y53C12A.3 [C.elegans]//0.00084:170:65//Hs.107747:AI357868
F-OVARC1000302//EST//4.1e-05:249:60//Hs.136432:AA555306
F-OVARC1000304//ESTs//1.0:252:64//Hs.12126:AA203287
F-OVARC1000309//ESTs, Highly similar to BRAIN ENRICHED HYALURONAN BINDING PROTEIN PRECURSOR [Felis catus]//0.51:193:66//Hs.6194:AI378579
F-OVARC1000321
F-OVARC1000326//Homo sapiens T-type calcium channel alpha-1 subunit mRNA, complete cds//0.0018:507:60//Hs.122359:AF051946
F-OVARC1000335//ESTs//9.3e-39:202:98//Hs.132849:AA779444
F-OVARC1000347
F-OVARC1000384//Homo sapiens (clone PEBP2aA1) core-binding factor, runt domain, alpha subunit 1 (CBFA1) mRNA, 3' end of cds//3.4e-06:353:62//Hs.121895:AF001450
F-OVARC1000408//Human mRNA for KIAA0140 gene, complete cds//0.94:231:64//Hs.156016:D50930
F-OVARC1000411//EST//0.43:234:59//Hs.124673:AA858162
F-OVARC1000414//EST//5.2e-05:105:72//Hs.98827:AA435682
F-OVARC1000420//Human mRNA for KIAA0140 gene, complete cds//0.86:231:58//Hs.156016:D50930
F-OVARC1000427//ESTs, Moderately similar to ORF1 [H.sapiens]//1.7e-25:190:84//Hs.139513:AA259082
F-OVARC1000431//ESTs//0.041:356:57//Hs.139907:AA621615
F-OVARC1000437//Filamin 1 (actin-binding protein-280)//0.93 :281:60//Hs.76279:X53416
F-OVARC1000440//Human PINCH protein mRNA, complete cds//8.8e-21:116:99//Hs.83987:U09284
F-OVARC1000442//ESTs//2.0e-19:207:78//Hs.134071:AI377423
F-OVARC1000443//Homo sapiens mRNA for KIAA0683 protein, complete cds//3.2e-140:566:99//Hs.12334:AB014583
F-OVARC1000461//ESTs//1.0e-39:215:95//Hs.131532:AI024524
F-OVARC1000465//Homo sapiens clone 24781 mRNA sequence//1.0:252:58//Hs.108112:AF070640
F-OVARC1000466//ESTs//3.6e-14:189:71//Hs.164041:R51854
F-OVARC1000473//ESTs//0.00012:77:85//Hs.29173:AA134926
F-OVARC1000479
F-OVARC1000486//ESTs//4.2e-07:409:60//Hs.99280:AA453036
F-OVARC1000496//ESTs//6.0e-14:240:69//Hs.131900:AI023327
F-OVARC1000520//Homo sapiens supervillin mRNA, complete cds//6.9e-115:539:99//Hs.111285:AF051850
F-OVARC1000526//ESTs//2.9e-08:368:611/Hs.42771:N26740
F-OVARC1000533//EST//3.4e-14:137:82//Hs.123405:AA813492
F-OVARC1000543//ESTs//0.13:278:61//Hs.54894:N98475
F-OVARC1000556//ESTs//1.4e-31:217:90//Hs.106385:W26667
F-OVARC1000557//ESTs//3.8e-20:208:76//Hs.138919:AA827410
F-OVARC1000564//Human dsRNA adenosine deaminase DRADA2b (DRADA2b) mRNA, complete cds//0.87:135:66//Hs.85302:U76421
F-OVARC1000573//ESTs//2.1e-22:268:76//Hs.121852:AA776358
F-OVARC1000576//ESTs//9.4e-22:124:98//Hs.24220:W22200
F-OVARC1000578//EST//4.7e-31:335:74//Hs.162881:AA652729
F-OVARC1000588//Human BMK1 alpha kinase mRNA, complete cds//0.67:263:63//Hs.3080:U29725
F-OVARC1000605//EST//1.0:148:62//Hs.163346:AA883722
F-OVARC1000622//EST//4.3e-50:313:88//Hs.149580:AI281881
F-OVARC1000640//ESTs//2.6e-55:441:80//Hs.105319:AA470097
F-OVARC1000649//Human squamous cell carcinama of esophagus mRNA for GRB-7 SH2 domain protein, complete cds//1.6e-78:424:93//Hs.86859:D43772
F-OVARC1000661//Homo sapiens mRNA for KIAA0590 protein, complete cds//1.6e-100:536:94//Hs.111862:AB011162
F-OVARC1000678//EST//1.3e-08:131:77//Hs.145970:AI277106
F-OVARC1000679//ESTs//0.66:223:61//Hs.134782:H74279
F-OVARC1000681//EST//0.017:315:61//Hs.147799:AI221639
F-OVARC1000682//Homo sapiens alpha 1,2-mannosidase IB mRNA, complete cds//4.8e-153:549:99//Hs.125315:AF027156
F-OVARC1000689//Homo sapiens clone 24640 mRNA sequence//0.030:479:57//Hs.4764:AB018306
F-OVARC 1000700
F-OVARC1000703//ESTs//0.41:100:68//Hs.160699:AI284320
F-OVARC1000722//Homo sapiens chromosome 1q21-1q23 beta-1,4-galactosyltransferase mRNA, complete cds//1.2e-110:451:91//Hs.13476:AF038661
F-OVARC1000730//ESTs, Weakly similar to C27F2.7 gene product [C.elegans]//2.9e-53:318:91//Hs.7049:AI141736
F-OVARC1000746//ESTs//3.2e-123:570:99//Hs.127295:AA918411
F-OVARC1000769//ESTs//0.072:177:67//Hs.142573:AA601196
F-OVARC1000771//ESTs, Moderately similar to RAS-RELATED PROTEIN RAB-2 [H.sapiens]//1.2e-38:194:99//Hs.157059:W28130
F-OVARC1000781//ESTs//4.0e-14:113:89//Hs.41972:AA626793
F-OVARC1000787//EST//0.92:91:64//Hs.163258:AA828835
F-OVARC1000800//ESTs//1.6e-44:193:81//Hs.163971:N27584
F-OVARC1000802//ESTs//4.6e-43:395:80//Hs.115401:AA400032
F-OVARC1000834//ESTs//1.9e-91:431:99//Hs.154450:AA069390
F-OVARC1000846//Homo sapiens mRNA for KIAA0643 protein, partial cds//1.9e-151:432:100//Hs.155995:AB014543
F-OVARC1000850//Homo sapiens PB39 mRNA, complete cds//3.3e-137:632:99//Hs.18910:AF045584
F-OVARC1000862//ESTs, Highly similar to gene Fif protein [M.musculus]//6.1e-31:183:93//Hs.108620:AA418155
F-OVARC1000876//Human DNA binding protein FKHL15 (FKHL15) mRNA, complete cds//0.54:133:69//Hs.159234:U89995
F-OVARC1000883//ESTs//0.44:154:63//Hs.98183:AA417143
F-OVARC1000885//EST//0.91:152:63//Hs.160765 :AI313323
F-OVARC1000886//ESTs//4.6e-08:375:61//Hs.131653:AI025777
F-OVARC 1000890
F-OVARC1000891
F-OVARC1000897//ESTs//1.1e-07:145:69//Hs.119878:AA706818
F-OVARC1000912//EST//3.6e-08:376:61//Hs.158782:AI376601
F-OVARC1000915//Homo sapiens mRNA for KIAA0600 protein, partial cds//2.3e-85:419:97//Hs.9028:AF039691
F-OVARC1000924//ESTs//3.6e-113:540:98//Hs.66058:AA424456
F-OVARC1000936//Human endogenous retrovirus envelope region mRNA (PL1)//4.3e-64:623:72//Hs.114440:M11119
F-OVARC1000937//EST//2.4e-39:170:96//Hs.129138:AA988078
F-OVARC1000945//ESTs, Weakly similar to protein tyrosine phosphatase [H.sapiens]//2.4e-29:157:97//Hs.136243:AA307843
F-OVARC 1000948
F-OVARC1000959//EST//0.65:293:55//Hs.134725:AI088986
F-OVARC1000960//Ley I-L//1.4e-41:425:72//Hs.37062:AC005952
F-OVARC1000964//ESTs//1.4e-95:486:96//Hs.57079:D45288
F-OVARC1000971//ESTs//0.19:198:62//Hs.153429:AI283069
F-OVARC1000984//Breakpoint cluster region protein BCR//0.26:365:56//Hs.2557:Y00661
F-OVARC1000996//Human p300/CBP-associated factor (P/CAF) mRNA, complete cds//6.8e-10:312:65//Hs.155302:U57317
F-OVARC1000999//Homo sapiens mRNA for chemokine LEC precursor, complete cds//0.0056:209:62//Hs.10458:AF088219
F-OVARC1001000//EST//4.2e-24:242:77//Hs.128952:AA984114
F-OVARC1001004
F-OVARC1001010
F-OVARC1001011//ESTs, Moderately similar to Tera [M.musculus]//3.8e-47:234:99//Hs.110327:AA205866
F-OVARC1001032//HUMAN IMMUNODEFICIENCY VIRUS TYPE I ENHANCER-BINDING PROTEIN 2//0.0076:624:57//Hs.75063:AL023584
F-OVARC1001034//ESTs, Highly similar to mitogen-induced [M.musculus]//3.9e-97:578:89//Hs.111974:AI050735
F-OVARC1001038//Homo sapiens TRIAD1 type I mRNA, complete cds//8.6e-152:733:97//Hs.9899:AF099149
F-OVARC 1001040//ESTs//2.2e-38:204:96//Hs.128927:AI168074
F-OVARC1001044//EST//0.036:304:61//Hs.137342:AA017385
F-OVARC1001051
F-OVARC1001055//Human pre-B cell enhancing factor (PBEF) mRNA, complete cds//1.1e-46:381:81//Hs.154968:U02020
F-OVARC1001062//ESTs//0.020:265:60//Hs.146226:AI312873
F-OVARC1001065//ESTs, Weakly similar to C50F4.12 [C.elegans]//1.4e-21:183:84//Hs.46680:AA809451
F-OVARC1001068//Homo sapiens Era GTPase A protein (HERA-A) mRNA, partial cds//6.6e-132:620:98//Hs.3426:AF082657
F-OVARC1001072//ESTs//1.1e-24:289:74//Hs.139614:AA709013
F-OVARC1001074//ESTs//0.059:198:63//Hs.59974:AA001937
F-OVARC1001085//H.sapiens mRNA for sortilin//0.99:142:67//Hs.104247:X98248
F-OVARC1001092//Homo sapiens mRNA for JM5 protein, complete CDS (clone IMAGE 53337, LLNLc110F1857Q7 (RZPD Berlin) and LLNLc110G0913Q7 (RZPD Berlin))//1.3e-75:289:95//Hs.21753:AJ005897
F-OVARC1001107//Homo sapiens SKB1Hs mRNA, complete cds//1.2e-73:351:86//Hs.12912:AF015913
F-OVARC1001113//Homo sapiens diaphanous 1 (HDIA1) mRNA, complete cds//2.1e-151:710:98//Hs.26584:AF051782
F-OVARC1001117//ESTs//3.8e-73:347:99//Hs.116029:AA813102
F-OVARC1001118
F-OVARC1001129
F-OVARC1001154//Granulin//2.4e-94:686:83//Hs.75451:AF055008
F-OVARC1001161//ESTs//2.2e-40:208:97//Hs.113006:AA621725
F-OVARC1001162
F-OVARC1001167
F-OVARC1001169//ESTs//0.81:158:63//Hs.48527:AI078279
F-OVARC1001170//ESTs//9.0e-87:412:99//Hs.116550:AA813287
F-OVARC1001171//ESTs//4.9e-26:167:79//Hs.139158:AA226159
F-OVARC1001173//ESTs, Moderately similar to GLUTAMATE DEHYDROGENASE 1 PRECURSOR [Homo sapiens]//1.8e-11:192:69//Hs.130020:AA887581
F-OVARC1001176//Homo sapiens chromosome 19, cosmid R26529//0.61:387:58//Hs.91103:AC005551
F-OVARC1001180//ESTs, Weakly similar to ubiquitin S6(1) [D.melanogaster]//1.5e-13:199:71//Hs.109966:C06057
F-OVARC1001188//ESTs, Weakly similar to HYPOTHETICAL 27.8 KD PROTEIN IN VMA7-RPS31A INTERGENIC REGION [S.cerevisiae]//1.4e-52:324:90//Hs.114673:W72675
F-OVARC1001200//ESTs//3.9e-16:104:94//Hs.125520:AA883889
F-OVARC1001232//Cyclin A//0.95:124:67//Hs.85137:X51688
F-OVARC1001240//EST//0.017:351:60//Hs.120655:AA745676
F-OVARC1001243//ESTs//0.78:291:59//Hs.132458:AI424825
F-OVARC1001244//RING3 PROTEIN//2.8e-19:118:95//Hs.75243:D42040
F-OVARC1001261//EST//1.9e-42:225:96//Hs.158854:AI377837
F-OVARC1001268//ESTs//0.66:239:61//Hs.132525:AA576821
F-OVARC1001270//ESTs//0.99:204:60//Hs.144647:AA625224
F-OVARC1001271//Homo sapiens mRNA for KIAA0643 protein, partial cds//6.8e-144:644:96//Hs.155995:AB014543
F-OVARC1001282//ESTs, Weakly similar to Ydr438wp [S.cerevisiae]//0.11:355:60//Hs.108812:AA044835
F-OVARC1001296//ESTs//1.1e-46:237:98//Hs.33746:N78172
F-OVARC1001306//Homo sapiens nuclear receptor co-repressor N-CoR mRNA, complete cds//0.20:188:64//Hs.152455:AF044209
F-OVARC1001329//ESTs//1.4e-97:486:97//Hs.125886:AA884264
F-OVARC1001330
F-OVARC1001339//Solute carrier family 4, anion exchanger, member 2 (erythrocyte membrane protein band 3-like 1)//0.021:232:62//Hs.79410:U62531
F-OVARC1001341//ESTs, Weakly similar to C17G10.1 [C.elegans]//2.5e-76:363:99//Hs.105837:AA536054
F-OVARC1001342//EST//0.98:97:65//Hs.148210:AA897493
F-OVARC1001344//EST//5.3e-10:241:64//Hs.138777:N67251
F-OVARC1001357//Homo sapiens jerky gene product homolog mRNA, complete cds//0.64:198:61//Hs.105940:AF004715
F-OVARC1001360//ESTs//4.9e-87:429:97//Hs.130145:AI264633
F-OVARC1001369//ESTs//6.3e-07:371:62//Hs.131653:AI025777
F-OVARC1001372//Homo sapiens mRNA for KIAA0654 protein, partial cds//1.4e-69:533:74//Hs.109299:AB014554
F-OVARC1001376//Homo sapiens neuronal thread protein AD7c-NTP mRNA, complete cds//2.5e-49:365:73//Hs.129735:AF010144
F-OVARC1001381//Homo sapiens mRNA for candidate tumor suppressor involved in B-CLL//4.1e-149:683:99//Hs.151428:AJ224819
F-OVARC1001391//Homo sapiens methyl-CpG binding protein MBD2 (MBD2) mRNA, complete cds//0.097:235:65//Hs.25674:AF072242
F-OVARC1001399//ESTs//1.1e-35:264:83//Hs.59379:W28225
F-OVARC1001417//Homo sapiens EXLM1 mRNA, complete cds//1.3e-150:707:98//Hs.21586:AB006651
F-OVARC1001419//Homo sapiens GOK (STIM1) mRNA, complete cds//1.6e-49:586:69//Hs.74597:U52426
F-OVARC1001425//ESTs//2.4e-11:258:67//Hs.119197:T83651
F-OVARC1001436
F-OVARC1001442
F-OVARC1001453
F-OVARC1001476//ESTs, Weakly similar to HYPOTHETICAL 38.6 KD PROTEIN IN TIF4631-KRE11 INTERGENIC REGION [S.cerevisiae]//1.9e-125:581:99//Hs.110950:AI041823
F-OVARC1001480//ESTs//0.95:125:72//Hs.152584:AA584568
F-OVARC1001489//EST//4.9e-72:341:100//Hs.148191:AA897343
F-OVARC1001496//Homo sapiens C-terminal binding protein 2 mRNA, complete cds//2.6e-86:479:92//Hs.6534:AF016507
F-OVARC1001506//Polycystic kidney disease 1 (autosomal dominant)//1.1e-97:538:92//Hs.75813:L33243
F-OVARC1001525
F-OVARC1001542//Envoplakin//0.34:258:60//Hs.25482:U53786
F-OVARC1001547//EST//0.0046:237:62//Hs.54638:N90595
F-OVARC1001555
F-OVARC1001577//Homo sapiens SRp46 splicing factor retropseudogene mRNA//6.8e-57:275:98//Hs.155160:AF031166
F-OVARC1001600//ESTs, Weakly similar to !!!! ALU SUBFAMILY J WARNING ENTRY !!!! [H.sapiens]//0.0035:271:60//Hs.108465:AI144299
F-OVARC1001610//ESTs, Weakly similar to F22E10.5 [C.elegans]//1.4e-43:216:99//Hs.120002:AI038398
F-OVARC1001611
F-OVARC1001615//EST//0.99:135:68//Hs.129410:AA993500
F-OVARC1001668//Homo sapiens mRNA for KIAA0572 protein, partial cds//3.3e-37:217:94//Hs.14409:AB011144
F-OVARC1001702//Homo sapiens mRNA for hSOX20 protein, complete cds//5.9e-49:393:81//Hs.95582:AB006867
F-OVARC1001703//EST//1.7e-24:172:88//Hs.121198:AA757229
F-OVARC1001711//Fms-related tyrosine kinase 3 ligand//0.049:353:61//Hs.428:U03858
F-OVARC1001713//ESTs//8.9e-37:263:86//Hs.110298:AA621807
F-OVARC1001726//ESTs//2.0e-12:121:82//Hs.153332:AA236863
F-OVARC1001731//Tropomyosin beta chain (skeletal muscle)//1.7e-83:617:80//Hs.155652:X06825
F-OVARC1001745//EST//0.75:174:64//Hs.146778:AI148588
F-OVARC1001762
F-OVARC1001766//Homo sapiens eukaryotic translation initiation factor eIF3, p35 subunit mRNA, complete cds//1.4e-150:706:98//Hs.155377:U97670
F-OVARC1001767//Homo sapiens mRNA for KIAA0675 protein, complete cds//9.8e-117:580:96//Hs.15869:AB014575
F-OVARC 1001768//ESTs//0.035:179:64//Hs.87279:AI218697
F-OVARC1001791
F-OVARC1001795//ESTs//0.19:68:76//Hs.37699:AA062830
F-OVARC1001802//EST//3.7e-45:254:92//Hs.130620:AI005102
F-OVARC1001805//Homo sapiens mRNA for KIAA0744 protein, complete cds//0.77:362:58//Hs.116753:AB018287
F-OVARC1001809//Human N-type calcium channel alpha-1 subunit mRNA, complete cds//2.2e-07:435:62//Hs.69949:M94172
F-OVARC1001812//ESTs//3.0e-47:360:83//Hs.141756:AA700825
F-OVARC1001813//EST//1.8e-57:277:100//Hs.162414:AA573453
F-OVARC1001820//ESTs//1.4e-64:310:99//Hs.137398:AA164567
F-OVARC1001828//EST//1.0e-09:184:66//Hs.130435:AA923537
F-OVARC1001846//ESTs//1.8e-80:410:97//Hs.114539:N54973
F-OVARC1001861
F-OVARC1001873//Homo sapiens clones 24718 and 24825 mRNA sequence//3.9e-20:122:95//Hs.25300:AF070611
F-OVARC1001879//Homo sapiens putative tumor suppressor gene 26 protein alpha 2 delta calcium channel subunit mRNA, complete cds//0.042:199:67//Hs.127436:AF040709
F-OVARC1001880//Interferon regulatory factor 5//1.1e-06:489:60//Hs.54434:U51127
F-OVARC1001883//Homo sapiens mRNA, chromosome 1 specific transcript KIAA0484//9.5e-33:509:68//Hs.158095:AB007953
F-OVARC1001900//Homo sapiens tumorous imaginal discs protein Tid56 homolog (TID1) mRNA, complete cds//2.6e-57:300:96//Hs.6216:AF061749
F-OVARC1001901//ESTs//2.3e-07:185:69//Hs.145630:AI263834
F-OVARC1001911//EST//0.88:101:66//Hs.162622:AA601261
F-OVARC1001916//H.sapiens mRNA for prepronociceptin//1.0:540:58//Hs.89040:U48263
F-OVARC1001928
F-OVARC1001942//Human plectin (PLEC1) mRNA, complete cds//0.038:290:62//Hs.79706:U53204
F-OVARC1001943//ESTs, Weakly similar to HYPOTHETICAL 62.2 KD PROTEIN ZK652.6 IN CHROMOSOME III [C.elegans]//2.3e-119:565:98//Hs.5392:AA313794
F-OVARC1001949//KRAB zinc finger protein {alternative products}//1.8e-17:294:67//Hs.22556:U37251
F-OVARC1001950//ESTs//1.5e-15:300:65//Hs.138501:AI051228
F-OVARC1001987//ESTs//6.7e-34:202:92//Hs.115600:AA351639
F-OVARC1001989//ESTs, Weakly similar to !!!! ALU SUBFAMILY J WARNING ENTRY !!!! [H.sapiens]//1.2e-23:213:78//Hs.105292:AA504776
F-OVARC1002044//EST//0.26:164:66//Hs.161094:N30417
F-OVARC1002050//Homo sapiens mRNA for KIAA0465 protein, partial cds//6.6e-160:739:98//Hs.108258:AB007934
F-OVARC1002066//ESTs//1.8e-103:482:99//Hs.124923:AI375865
F-OVARC1002082//EST//2.5e-09:213:67//Hs.112810:AA610063
F-OVARC1002107
F-OVARC1002112//Homo sapiens histone macroH2A1.2 mRNA, complete cds//2.7e-101:498:96//Hs.75258:AF054174
F-OVARC1002127//ESTs//1.6e-76:397:96//Hs.33432:R83913
F-OVARC1002138//Homo sapiens p60 katanin mRNA, complete cds//3.5e-20:399:62//Hs.112725:AF056022
F-OVARC1002143//EST//4.2e-09:240:65//Hs.140547:AA812795
F-OVARC1002156//EST//0.35:112:66//Hs.136761:AA738097
F-OVARC1002158//ESTs, Weakly similar to Y53C12A.3 [C.elegans]//7.4e-07:329:58//Hs.107747:AI357868
F-OVARC1002165//H.sapiens BDP1 mRNA for protein-tyrosine-phosphatase//0.00010:300:64//Hs.118929:X79568
F-OVARC1002182//Homo sapiens ataxin-7 (SCA7) mRNA, complete cds//0.19:178:64//Hs.108447:AJ000517
F-PLACE1000004//ESTs//0.79:332:59//Hs.120221:AA731230
F-PLACE1000005//ESTs//1.8e-10:89:87//Hs.158913:AI378928
F-PLACE1000007//Homo sapiens ubiquitin hydrolyzing enzyme I (UBH1) mRNA, partial cds//1.2e-52:550:72//Hs.42400:AF022789
F-PLACE1000014
F-PLACE1000031
F-PLACE1000040//ESTs//3.1e-18:123:91//Hs.138387:AA873088
F-PLACE1000048//ESTs//1.2e-43:387:78//Hs.61199:AA024494
F-PLACE1000050//ESTs//1.8e-84:421:96//Hs.128632:AI076755
F-PLACE1000061//Ribosomal protein L37a//5.5e-29:177:93//Hs.1946:L06499
F-PLACE1000066//ESTs, Weakly similar to coded for by C. elegans cDNA yk10c10.3 [C.elegans]//1.4e-47:266:93//Hs.30026:AI356771
F-PLACE1000078//ESTs, Weakly similar to !!!! ALU SUBFAMILY SB1 WARNING ENTRY !!!! [H.sapiens]//6.4e-15:203:70//Hs.157422:R85366
F-PLACE1000081//Human transporter protein (g17) mRNA, complete cds//0.30:324:60//Hs.76460:U49082
F-PLACE1000094
F-PLACE1000133//ESTs, Highly similar to TRANSCRIPTION FACTOR BTF3 [Homo sapiens]//6.2e-82:476:92//Hs.111081:AI380378
F-PLACE1000142//ESTs, Weakly similar to enoyl-CoA hydratase [H.sapiens]//7.7e-27:205:85//Hs.9670:AA632135
F-PLACE1000184//Homo sapiens estrogen-related receptor gamma mRNA, complete cds//2.5e-151:737:97//Hs.151017:AF058291
F-PLACE1000185
F-PLACE1000213
F-PLACE1000214//ESTs//0.00059:335:59//Hs.143849:AI167255
F-PLACE1000236//Fanconi anemia, complementation group A//0.44:306:61//Hs.86297:X99226
F-PLACE1000246//ESTs//7.3e-80:457:89//Hs.57209:W22022
F-PLACE1000292//ESTs//1.8e-05:323:60//Hs.59962:AI278202
F-PLACE1000308//EST//0.0024:253:62//Hs.144238:W52294
F-PLACE1000332//EST//5.6e-18:223:74//Hs.99532:AA461047
F-PLACE1000347//ESTs//6.4e-33:169:99//Hs.122975:AA428675
F-PLACE1000374//Human CCAAT-box-binding factor (CBF) mRNA, complete cds//0.26:45:95//Hs.147991:M37197
F-PLACE1000380//Homo sapiens proline and glutamic acid rich nuclear protein isoform mRNA, partial cds//1.0:262:58//Hs.102732:U88153
F-PLACE1000383//Myotubular myopathy 1//1.1e-50:669:67//Hs.75302:U46024
F-PLACE1000401//Homo sapiens mRNA for KIAA0616 protein, partial cds//0.036:471:58//Hs.6051:AB014516
F-PLACE1000406//ESTs, Highly similar to PTB-ASSOCIATED SPLICING FACTOR [Homo sapiens]//8.7e-63:346:93//Hs.19501:AA742260
F-PLACE1000420//Homo sapiens mRNA for KIAA0602 protein, partial cds//0.0023:216:65//Hs.37656:AB011174
F-PLACE1000421//Human lipid-activated protein kinase PRK1 mRNA, complete cds//0.55:212:63//Hs.2499:U33053
F-PLACE1000424
F-PLACE1000435//Homo sapiens mRNA for XPR2 protein//0.58:674:55//Hs.44766:AJ007590
F-PLACE1000444//Fucosyltransferase 1 (galactoside 2-alpha-L-fucosyltransferase, Bombay phenotype included)//2.7e-52:421:80//Hs.69747:M35531
F-PLACE1000453//Human mRNA for MTG8a protein, complete cds//0.026:240:60//Hs.31551:D43638
F-PLACE1000481//Oxytocin receptor//1.6e-25:347:71//Hs.2820:X64878
F-PLACE1000492//Human mRNA for KIAA0355 gene, complete cds//0.58:302:60//Hs.153014:AB002353
F-PLACE1000540//EST//0.32:229:59//Hs.163011:AA700573
F-PLACE1000547//Human heparan sulfate proteoglycan (HSPG2) mRNA, complete cds//0.0046:223:65//Hs.75578:M85289
F-PLACE1000562
F-PLACE1000564//ESTs//8.0e-35:247:89//Hs.12999:AA278538
F-PLACE1000583//Homo sapiens clone 23939 mRNA sequence//6.6e-47:525:72//Hs.21838:AF038179
F-PLACE1000588//Guanylate binding protein 1, interferon-inducible, 67kD//2.3e-85:503:88//Hs.62661:M55542
F-PLACE1000596//Homo sapiens mRNA for NS1-binding protein (NS1-BP)//1.2e-165:798:97//Hs.159597:AJ012449
F-PLACE1000599//ESTs//0.65:201:58//Hs.98216:AA758751
F-PLACE1000610//Homo sapiens mRNA for KIAA0642 protein, partial cds//0.98:215:60//Hs.8152:AB014542
F-PLACE1000611//ESTs//7.2e-20:406:64//Hs.128966:AA620986
F-PLACE1000636
F-PLACE1000653//Homo sapiens N-acetylglucosamine-phosphate mutase mRNA, complete cds//5.0e-154:747:96//Hs.5819:AF102265
F-PLACE1000656//Homo sapiens mRNA for JM4 protein, complete CDS (clone IMAGE 546750 and LLNLc110F1857Q7 (RZPD Berlin))//7.5e-158:775:97//Hs.29595:AJ005896
F-PLACE1000706//Homo sapiens transcription intermediary factor 1 (TIF1) mRNA, complete cds//1.0e-57:675:69//Hs.128763:AF009353
F-PLACE1000712//EST//0.56:171:61//Hs.112790:AA609949
F-PLACE1000716//Human mRNA for KIAA0258 gene, complete cds//6.1e-38:426:70//Hs.47313:D87447
F-PLACE1000748//ESTs//2.6e-43:233:95//Hs.110754:AA112288
F-PLACE1000749//Human MAGE-9 antigen (MAGE9) gene, complete cds//0.72:331:57//Hs.37110:U10694
F-PLACE1000755//NUCLEOLIN//0.0038:186:66//Hs.79110:M60858
F-PLACE1000769
F-PLACE1000785//Homo sapiens mRNA for KIAA0648 protein, partial cds//1.1e-139:663:98//Hs.31921:AB014548
F-PLACE1000786//Myosin, heavy polypeptide 9, non-muscle//8.5e-06:362:59//Hs.44782:Z82215
F-PLACE1000793//ESTs//2.7e-62:315:97//Hs.16141:W56079
F-PLACE1000798//ESTs//1.4e-55:316:93//Hs.139119:N32189
F-PLACE1000841//EST//0.47:143:61//Hs.144096:AI032180
F-PLACE1000849//Homo sapiens CAGF9 mRNA, partial cds//1.6e-06:266:63//Hs.110826:U80736
F-PLACE1000856//ESTs//2.6e-60:319:96//Hs.25994:AA470000
F-PLACE1000863//EST//9.4e-29:249:78//Hs.121919:AA777428
F-PLACE1000909//ESTs//0.97:214:60//Hs.128601:AA906455
F-PLACE1000931//ESTs//2.1e-46:592:70//Hs.154244:AA195201
F-PLACE1000948
F-PLACE1000972//Homo sapiens enhancer of filamentation (HEF1) mRNA, complete cds//7.9e-10:294:66//Hs.80261:L43821
F-PLACE1000977//ESTs, Weakly similar to coded for by C. elegans cDNA yk28h2.5 [C.elegans]//9.3e-45:309:88//Hs.13531:R61789
F-PLACE1000979//Zinc finger protein 91 (HPF7, HTF10)//0.0034:229:62//Hs.8597:L11672
F-PLACE1000987//Homo sapiens mRNA for KIAA0724 protein, complete cds//2.6e-141:694:96//Hs.158497:AB018267
F-PLACE1001000//ESTs//0.0035:116:73//Hs.144532:H39913
F-PLACE1001007//Guanylate cyclase 2D, membrane (retina-specific)//0.050:338:61//Hs.1974:M92432
F-PLACE1001010//H.sapiens mRNA for retrotransposon//1.6e-45:371:80//Hs.6940:Z48633
F-PLACE1001015//ESTs//8.6e-27:211:71//Hs.88040:AA256876
F-PLACE1001024
F-PLACE1001036//EST//1.0:133:65//Hs.161424:AI424741
F-PLACE1001054//Human plectin (PLEC1) mRNA, complete cds//0.98:284:59//Hs.79706:U53204
F-PLACE1001062
F-PLACE1001076//EST//0.84:223:59//Hs.161147:AI417859
F-PLACE1001088
F-PLACE1001092//Homo sapiens sorting nexin 4 mRNA, complete cds//1.0e-96:489:96//Hs.95448:AF065485
F-PLACE1001104//ESTs//0.19:249:64//Hs.152627:AA595817
F-PLACE1001118//Homo sapiens KRAB domain zinc finger protein (ZFP37) mRNA, complete cds//8.2e-66:676:71//Hs.150406:AF022158
F-PLACE1001136//Amphiregulin (schwannoma-derived growth factor)//1.5e-16:122:91//Hs.1257:M30704
F-PLACE1001168
F-PLACE1001171//ESTs//4.3e-12:214:72//Hs.141392:R95135
F-PLACE1001185//ESTs, Weakly similar to ZK792.1 [C.elegans]//1.6e-28:421:66//Hs.8763:W30741
F-PLACE1001238
F-PLACE1001241//ESTs//1.1e-22:225:79//Hs.159786:R49494
F-PLACE1001257//ESTs//1.9e-23:165:89//Hs.126518:AA913929
F-PLACE1001272//COATOMER BETA'SUBUNIT//0.012:50:96//Hs.75724:X70476
F-PLACE1001279//ESTs//0.97:377:59//Hs.152628:N51283
F-PLACE1001280//Homo sapiens hyperpolarization-activated channel 1 (IH1) mRNA, partial cds//1.2e-08:586:58//Hs.124161:AF065164
F-PLACE1001294//Homo sapiens mRNA for myosin phosphatase target subunit 1 (MYPT1)//0.91:221:61//Hs.16533:D87930
F-PLACE1001304//Human zinc finger protein mRNA, complete cds//8.6e-08:370:60//Hs.42672:AF016052
F-PLACE1001311//ESTs//1.7e-44:480:73//Hs.155384:Z78385
F-PLACE1001323//ESTs//1.1e-25:151:95//Hs.134120:AA699591
F-PLACE1001351
F-PLACE1001366//Homo sapiens mRNA for KIAA0799 protein, partial cds//2.8e-26:155:95//Hs.61638:AB018342
F-PLACE1001377//Homo sapiens ADAM10 (ADAM10) mRNA, complete cds//3.4e-44:393:79//Hs.152005:AF009615
F-PLACE1001383//ESTs//1.0:159:65//Hs.128501:AA973748
F-PLACE1001384//Homo sapiens multi PDZ domain protein MUPP1 (MUPP1) mRNA, complete cds//2.6e-09:117:84//Hs.21301:AF093419
F-PLACE1001387//ESTs, Weakly similar to EPIDERMAL GROWTH FACTOR RECEPTOR KINASE SUBSTRATE EPS8 [H.sapiens]//0.00083:187:64//Hs.5399:N30646
F-PLACE1001395//Homo sapiens mRNA for putative DNA methyltransferase, complete
CDS//0.0038:496:57//Hs.97681:AJ223333
F-PLACE1001399//Human melanoma antigen recognized by T-cells (MART-1) mRNA//7.0e-45:456:75//Hs.154069:U06452
F-PLACE1001412//Homo sapiens clone 643 unknown mRNA, complete sequence//6.5e-71:365:96//Hs.110404:AF091087
F-PLACE1001414//EST//1.2e-75:364:98//Hs.136622:AA633232
F-PLACE1001440//ESTs//2.8e-05:163:66//Hs.141082:H18987
F-PLACE1001456//EST//0.95:132:61//Hs.20373:R09510
F-PLACE1001468//ESTs//0.00019:184:66//Hs.126536:AI379455
F-PLACE1001484//EST//8.6e-18:190:76//Hs.160992:H52716
F-PLACE1001502//Apolipoprotein E//2.5e-05:306:60//Hs.76260:M12529
F-PLACE1001503
F-PLACE1001517//ESTs//1.9e-12:138:78//Hs.120352:AA718914
F-PLACE1001534//EST//0.015:121:65//Hs.144156:R85753
F-PLACE1001545
F-PLACE1001551
F-PLACE1001570//EST//0.58:286:59//Hs.120202:AA728835
F-PLACE1001602//Human POU domain protein (Brn-3b) mRNA, complete cds//0.013:159:66//Hs.266:U06233
F-PLACE1001603//Homo sapiens nitrilase 1 (NIT1) mRNA, complete cds//1.1e-10:133:77//Hs.146406:AF069987
F-PLACE1001608//ESTs//0.022:187:60//Hs.145915:AI342230
F-PLACE1001610//ESTs//1.4e-77:377:97//Hs.115700:AA808005
F-PLACE1001611//Human faciogenital dysplasia (FGD1) mRNA, complete cds//0.96:141:66//Hs.1572:U11690
F-PLACE1001632//Homo sapiens mRNA for KIAA0798 protein, complete cds//3.4e-76:702:75//Hs.159277:AB018341
F-PLACE1001634//ESTs//1.2e-43:260:92//Hs.134064:AI276198
F-PLACE1001640
F-PLACE1001672//EST//2.8e-21:201:82//Hs.123341:AA810927
F-PLACE1001691//Homo sapiens okadaic acid-inducible phosphoprotein (OA48-18) mRNA, complete cds//2.8e-148:726:96//Hs.3688:AF069250
F-PLACE1001692//ESTs, Highly similar to S-ACYL FATTY ACID SYNTHASE THIOESTERASE, MEDIUM CHAIN [Rattus norvegicus]//1.1e-95:481:92//Hs.24309:AI125696
F-PLACE1001705//Human RNA polymerase III subunit (RPC39) mRNA, complete cds//6.0e-30:347:76//Hs.101555:U93869
F-PLACE1001716//Human mRNA for KIAA0191 gene, partial cds//2.1e-69:369:73//Hs.12413:D83776
F-PLACE1001720//ESTs//1.2e-27:146:99//Hs.106432:AI391686
F-PLACE1001729//Homo sapiens mRNA for KIAA0522 protein, partial cds//0.0084:484:60//Hs.129892:AB011094
F-PLACE1001739//Histidine-rich calcium binding protein//0.14:240:64//Hs.1480:M60052
F-PLACE1001740//ESTs//4.9e-32:343:74//Hs.139158:AA226159
F-PLACE1001745
F-PLACE1001746//ESTs//7.0e-15:168:80//Hs.46601:N78361
F-PLACE1001748//Homo sapiens metalloprotease 1 (MP1) mRNA, complete cds//2.8e-160:773:97//Hs.4812:AF061243
F-PLACE1001756//Homo sapiens tapasin (NGS-17) mRNA, complete cds//2.7e-35:269:83//Hs.5247:AF029750
F-PLACE1001761//ESTs//6.9e-27:159:93//Hs.78277:AA131283
F-PLACE1001771//Human putative calcium influx channel (htrp3) mRNA, complete cds//3.4e-52:548:72//Hs.150981:U47050
F-PLACE1001781
F-PLACE1001799//EST//5.4e-07:145:70//Hs.121840:AA776115
F-PLACE1001810//ESTs//0.024:134:67//Hs.43134:AA766138
F-PLACE1001817//Homo sapiens ATP-specific succinyl-CoA synthetase beta subunit (SCS) mRNA, partial cds//3.6e-110:546:96//Hs.40820:AF058953
F-PLACE1001821
F-PLACE1001844//ESTs//5.4e-45:387:79//Hs.61199:AA024494
F-PLACE1001845//ESTs//2.5e-47:232:100//Hs.120809:AA150214
F-PLACE1001869//EST//1.0:139:59//Hs.122285:AA781906
F-PLACE1001897//ESTs//0.29:348:57//Hs.139993:AI343257
F-PLACE1001912//ESTs//4.0e-10:95:89//Hs.13475:R18220
F-PLACE1001920//Homo sapiens TNF-induced protein GG2-1 mRNA, complete cds//4.0e-153:685:95//Hs.17839:AF099936
F-PLACE1001928//H.sapiens HUMM9 mRNA//0.063:196:66//Hs.2750:X74837
F-PLACE1001983//Homo sapiens Jagged 2 mRNA, complete cds//9.8e-06:431:58//Hs.106387:AF029778
F-PLACE1001989
F-PLACE1002004
F-PLACE1002046
F-PLACE1002052//Human mRNA for phospholipase C, complete cds//0.0092:465:58//Hs.153322:D42108
F-PLACE1002066//EST//0.49:307:61//Hs.150652:AA908555
F-PLACE1002072//EST//1.0:103:65//Hs.116488:F13707
F-PLACE1002073//Homo sapiens mRNA for KIAA0606 protein, partial cds//4.2e-39:635:64//Hs.38176:AB011178
F-PLACE1002090//Homo sapiens signal recognition particle 72 (SRP72) mRNA, complete cds//4.3e-83:388:99//Hs.5171:AF069765
F-PLACE1002115//EST//0.18:215:62//Hs.135747:AI002637
F-PLACE1002119//Human transcription factor ETR101 mRNA, complete cds//6.2e-13:384:61//Hs.737:M62831
F-PLACE1002140//EST, Moderately similar to ALPHA-1-ANTITRYPSIN PRECURSOR [Homo sapiens]//0.89:60:75//Hs.144290:T61747
F-PLACE1002150//ESTs//0.56:245:64//Hs.24119:AA115631
F-PLACE1002157//Human mRNA for KIAA0392 gene, partial cds//2.8e-51:440:79//Hs.40100:AB002390
F-PLACE1002163//ESTs//0.76:212:61//Hs.112494:AI366891
F-PLACE1002170//ESTs//6.5e-09:108:76//Hs.41418:H90627
F-PLACE1002171//ESTs//3.5e-81:493:89//Hs.122553:H66674
F-PLACE1002205//Human clone 23695 mRNA sequence//0.00080:472:60//Hs.90798:U79289
F-PLACE1002213//ESTs//0.041:146:67//Hs.119162:AA399989
F-PLACE1002227//ESTs//9.4e-06:173:66//Hs.127882:AI024442
F-PLACE1002256//ESTs//1.8e-93:440:99//Hs.128700:AA970935
F-PLACE1002259//Human Line-1 repeat mRNA with 2 open reading frames//2.3e-75:434:83//Hs.23094:M19503
F-PLACE1002319//ESTs//0.82:188:62//Hs.50918:AA036675
F-PLACE1002342//EST//0.61:148:66//Hs.144319:AA280279
F-PLACE1002395//ESTs//1.2e-18:168:83//Hs.3853:AA034291
F-PLACE1002399//EST//0.0011:166:65//Hs.137500:AA436710
F-PLACE1002433//ESTs//1.2e-14:151:80//Hs.161837:AA421067
F-PLACE1002437//Human ATP binding cassette transporter (ABCR) mRNA, complete cds//2.6e-23:458:66//Hs.40993:AF000148
F-PLACE1002438//EST//0.81:48:77//Hs.158575:AI368947
F-PLACE1002450//Homo sapiens KRAB domain zinc finger protein (ZFP37) mRNA, complete cds//7.1e-07:270:66//Hs.150406:AF022158
F-PLACE1002465
F-PLACE1002474//Homo sapiens mRNA for matrilin-4, partial//1.3e-14:369:63//Hs.129361:AJ007581
F-PLACE1002477//ESTs//3.5e-13:125:71//Hs.145032:AA343523
F-PLACE1002493
F-PLACE1002499
F-PLACE1002500//Human putative zinc transporter ZnT-3 (ZnT-3) mRNA, complete cds//4.3e-19:708:59//Hs.111967:U76010
F-PLACE1002514//ESTs//3.1e-07:178:66//Hs.70932:AA126482
F-PLACE1002529//Homo sapiens mRNA for KIAA0713 protein, partial cds//2.9e-144:583:95//Hs.88756:AB018256
F-PLACE1002532//Homo sapiens BAC clone RG300E22 from 7q21-q31.1//3.1e-115:566:96//Hs.99348:AC004774
F-PLACE1002537//Thiopurine S-methyltransferase//1.9e-28:198:86//Hs.51124:AF019369
F-PLACE1002571//Homo sapiens mRNA for TP55, complete cds//0.99:274:59//Hs.138202:AF027866
F-PLACE1002578//ESTs//7.3e-10:185:73//Hs.41418:H90627
F-PLACE1002583//EST//0.0028:348:61//Hs.160396:AI393725
F-PLACE1002591//Human mRNA for actin binding protein p57, complete cds//2.8e-27:279:74//Hs.109606:D44497
F-PLACE1002598//EST//0.011:209:62//Hs.131470:AI024187
F-PLACE1002604//EST//0.47:220:61//Hs.145434:AI198915
F-PLACE1002625
F-PLACE1002655//GELSOLIN PRECURSOR, PLASMA//1.7e-36:693:62//Hs.80562:X04412
F-PLACE1002665//EST//0.15:156:65//Hs.161793:AA380706
F-PLACE1002685//Homo sapiens B cell linker protein BLNK mRNA, alternatively spliced, complete cds//1.1e-187:804:97//Hs.124903:AF068180
F-PLACE1002714//Human involucrin mRNA//3.6e-08:509:60//Hs.157091:M13903
F-PLACE1002722//Human protease-activated receptor 3 (PAR3) mRNA, complete cds//0.34:230:58//Hs.159196:U92971
F-PLACE1002768//EST//0.37:126:69//Hs.125353:AA877080
F-PLACE1002772//ESTs//0.0017:147:69//Hs.132439:AA923728
F-PLACE1002775//EST//5.5e-09:129:75//Hs.135336:AI049827
F-PLACE1002782//Homo sapiens I-1 receptor candidate protein mRNA, complete cds//0.0031:298:62//Hs.26285:AF082516
F-PLACE1002794//ESTs//0.71:125:66//Hs.97441:AI368926
F-PLACE1002811//Human mRNA for KIAA0172 gene, partial cds//5.8e-46:567:70//Hs.77546:D79994
F-PLACE1002815
F-PLACE1002816//Homo sapiens mRNA for KIAA0600 protein, partial cds//4.3e-70:687:73//Hs.9028:AF039691
F-PLACE1002834//ESTs//2.6e-41:393:74//Hs.120206:AI089163
F-PLACE1002839//ESTs//0.26:177:63//Hs.149013:AI334167
F-PLACE1002851//EST//0.0034:102:72//Hs.129630:AI000405
F-PLACE1002853//ESTs//1.1e-20:136:90//Hs.125895:AA889024
F-PLACE1002881//Interleukin 10//1.1e-41:454:72//Hs.2180:M57627
F-PLACE1002908//ESTs//3.8e-48:325:88//Hs.54702:AI040029
F-PLACE1002941//ESTs//5.0e-18:128:88//Hs.17376:AA855056
F-PLACE1002962
F-PLACE1002968//ESTs, Highly similar to trg gene product [R.norvegicus]//0.031:372:59//Hs.8021:AI041815
F-PLACE1002991
F-PLACE1002993
F-PLACE1002996//ESTs, Weakly similar to T20D3.3 [C.elegans]//1.3e-12:104:86//Hs.124808:T86959
F-PLACE1003025//Homo sapiens mRNA, chromosome 1 specific transcript KIAA0510//0.99:192:64//Hs.92660:AB007979
F-PLACE1003027//Homo sapiens mRNA for KIAA0516 protein, partial cds//2.0e-131:632:97//Hs.129872:AB011088
F-PLACE1003044//Homo sapiens mRNA for KIAA0667 protein, partial cds//2.7e-14:555:58//Hs.154740:AB014567
F-PLACE1003045
F-PLACE1003092//ESTs//1.1e-108:506:99//Hs.22119:AA885491
F-PLACE1003100//Human Hep27 protein mRNA, complete cds//2.9e-66:650:73//Hs.102137:U31875
F-PLACE1003108//EST//0.016:181:65//Hs.119762:AA703419
F-PLACE1003136
F-PLACE1003145
F-PLACE1003153//ESTs//3.1e-09:209:65//Hs.111583:AA463590
F-PLACE1003174//ESTs//0.073:97:69//Hs.12992:W01997
F-PLACE1003176//ESTs//3.3e-60:296:90//Hs.58239:AA215797
F-PLACE1003190//Homo sapiens C19steroid specific UDP-glucuronosyltransferase mRNA, complete cds//0.98:221:60//Hs.139756:U59209
F-PLACE1003200//EST//0.0021:309:60//Hs.140561:AA765532
F-PLACE1003205//EST//1.2e-07:204:65//Hs.147372:AI208770
F-PLACE1003238//ESTs//7.4e-62:343:94//Hs.121302:AA758208
F-PLACE1003249//Insulin-like growth factor 1 (somatomedia C)//0.99:175:62//Hs.85112:X57025
F-PLACE1003256
F-PLACE1003258//H.sapiens mRNA for ZYG homologue//0.00020:217:64//Hs.29285:X99802
F-PLACE1003296//ESTs//2.6e-14:80:86//Hs.155441:AA533106
F-PLACE1003302//Human repressor transcriptional factor (ZNF85) mRNA, complete cds//4.3e-51:700:67//Hs.37138:U35376
F-PLACE1003334
F-PLACE1003342//ESTs//0.94:310:57//Hs.131502:AI023308
F-PLACE1003343//EST//1.2e-09:114:77//Hs.103418:AA035568
F-PLACE1003353//Homo sapiens breast cancer antiestrogen resistance 3 protein (BCAR3) mRNA, complete cds//2.6e-144:773:92//Hs.6564:U92715
F-PLACE1003361//ESTs, Weakly similar to ATP SYNTHASE A CHAIN [Trypanosoma brucei brucei]//8.9e-35:332:78//Hs.163820:H71277
F.-PLACE1003366//Homo sapiens dysferlin mRNA, complete cds//7.9e-06:502:57//Hs.143897:AF075575
F-PLACE1003369//NUCLEOLIN//0.00037:282:60//Hs.79110:M60858
F-PLACE1003373//EST//1.1e-11:420:63//Hs.156592:AI343009
F-PLACE1003375//EST//0.75:119:68//Hs.160270:AI149069
F-PLACE1003383
F-PLACE1003394//ESTs, Highly similar to RAS-RELATED PROTEIN RAB-14 [Rattus norvegicus]//8.9e-113:590:94//Hs.125175:AI142546
F-PLACE1003401//ESTs//0.55:176:66//Hs.154292:AA886178
F-PLACE1003420//Macrophage stimulating 1 (hepatocyte growth factor-like)//0.40:206:62//Hs.30223:X90846
F-PLACE1003454//ESTs//0.98:74:72//Hs.127131:AA150912
F-PLACE1003478//EST//5.0e-06:183:69//Hs.127524:AA952874
F-PLACE1003493//Protein-tyrosine kinase 7//0.98:232:63//Hs.90572:U33635
F-PLACE1003516//Human kpni repeat mrna (cdna clone pcd-kpni-8), 3' end//3.4e-85:357:86//Hs.103948:K00627
F-PLACE1003519//ESTs//1.6e-33:288:72//Hs.159510:AA297145
F-PLACE1003521//H.sapiens mRNA for retrotransposon//1.4e-45:269:76//Hs.6940:Z48633
F-PLACE1003528//ESTs//0.65:120:68//Hs.162376:AA570248
F-PLACE1003537//ESTs, Weakly similar to ZK858.6 [C.elegans]//3.6e-110:543:97//Hs.120416:AA057428
F-PLACE1003553
F-PLACE1003566//ESTs//0.0015:508:59//Hs.5724:AA156780
F-PLACE1003575//Homo sapiens cdc14 homolog mRNA, complete cds//4.4e-05:499:58//Hs.65993:AF000367
F-PLACE1003583//ESTs//5.5e-19:448:63//Hs.161701:AA225932
F-PLACE1003584//EST//1.6e-46:263:94//Hs.147412:AI209194
F-PLACE1003592//ESTs, Moderately similar to !!!! ALU CLASS B WARNING ENTRY !!!! [H.sapiens]//1.4e-50:287:93//Hs.154799:AA130620
F-PLACE1003593//ESTs//0.0025:318:61//Hs.106771:AA806965
F-PLACE1003596//Integral transmembrane protein 1//1.9e-54:685:68//Hs.89650:L38961
F-PLACE1003602//Homo sapiens mRNA expressed in placenta//3.4e-140:679:97//Hs.56851:D83200
F-PLACE1003605//Homo sapiens Cdc14B2 phosphatase mRNA, partial cds//0.00065:236:64//Hs.22116:AF064104
F-PLACE1003611//EST//0.00015:318:59//Hs.28788:R66896
F-PLACE1003618//Human Line-1 repeat mRNA with 2 open reading frames//1.3e-122:737:87//Hs.23094:M19503
F-PLACE1003625//ESTs//1.6e-16:103:96//Hs.111223:N51105
F-PLACE1003638//ESTs//0.60:305:57//Hs.19104:W07762
F-PLACE1003669//ESTs, Weakly similar to 3-7 gene product [H.sapiens]//0.021:445:58//Hs.158275:AI365413
F-PLACE1003704//Human mRNA for KIAA0301 gene, partial cds//0.014:622:56//Hs.76730:AB002299
F-PLACE1003709//Homo sapiens protein kinase (BUB1) mRNA, complete cds//1.4e-133:669:95//Hs.98658:AF053305
F-PLACE1003711//ESTs//2.2e-14:178:77//Hs.114831:T57101
F-PLACE1003723//Homo sapiens mRNA for T lymophocyte specific adaptor protein//8.5e-09:393:60//Hs.103527:AJ000553
F-PLACE1003738//ESTs, Weakly similar to ZINC FINGER PROTEIN 84 [H.sapiens]//1.8e-53:260:99//Hs.102928:AI346344
F-PLACE1003760//ESTs//5.1e-08:334:63//Hs.43675:AA805648
F-PLACE1003762//ESTs//1.0:59:83//Hs.29863:W28983
F-PLACE1003768//Human kpni repeat mrna (cdna clone pcd-kpni-4), 3' end//2.7e-40:608:68//Hs.139107:K00629
F-PLACE1003771//ESTs//6.6e-10:226:65//Hs.15776:T91944
F-PLACE1003783
F-PLACE1003784//Homo sapiens mRNA for KIAA0765 protein, partial cds//1.0:457:57//Hs.62318:AB018308
F-PLACE1003795//Human homologue of yeast sec7 mRNA, complete cds//0.85:314:60//Hs.1050:M85169
F-PLACE1003833//ESTs, Weakly similar to C27H6.5 [C.elegans]//0.00059:201:68//Hs.40806:AA018786
F-PLACE1003850//ESTs//0.0088:220:61//Hs.145504:AI254165
F-PLACE1003858//EST//0.77:137:61//Hs.146935:AI168124
F-PLACE1003864//ESTs//0.11:225:59//Hs.160910:AI370359
F-PLACE1003870//EST//7.2e-18:283:69//Hs.135497:AI091257
F-PLACE1003885//H.sapiens PAP mRNA//2.4e-75:759:72//Hs.49007:X76770
F-PLACE1003886
F-PLACE1003888//Human mRNA for phospholipase C, complete cds//8.4e-55:702:67//Hs.153322:D42108
F-PLACE1003892//ESTs//2.4e-13:258:67//Hs.28039:H24050
F-PLACE1003900//ESTs//3.5e-14:271:66//Hs.28589:AI004944
F-PLACE1003903//CTP synthetase//1.6e-49:528:71//Hs.84112:X52142
F-PLACE1003915//ESTs, Highly similar to ARGINYL-TRNA SYNTHETASE, MITOCHONDRIAL PRECURSOR [Saccharomyces cerevisiae]//1.2e-49:251:98//Hs.65831:F03069
F-PLACE1003923//Interferon, alpha 16//0.48:278:60//Hs.56303:M28585
F-PLACE1003932//EST//0.00060:221:63//Hs.163044:AA707537
F-PLACE1003936//ESTs//0.86:211:62//Hs.150751:AI123536
F-PLACE1003968//Human 5'-AMP-activated protein kinase, gamma-1 subunit mRNA, complete cds//2.0e-47:522:71//Hs.3136:U42412
F-PLACE1004103//ESTs//8.6e-35:226:89//Hs.78973:AI026812
F-PLACE1004104//ESTs//1.0:179:61//Hs.163935:AA506940
F-PLACE1004114//ESTs//1.3e-52:323:89//Hs.35156:AA148516
F-PLACE1004118//Spleen focus forming virus (SFFV) proviral integration oncogene spi1//0.85:164:64//Hs.153045:X52056
F-PLACE1004128//Guanine nucleotide binding protein (G protein), beta polypeptide 1//3.1e-41:422:74//Hs.3620:X04526
F-PLACE1004149//ESTs, Weakly similar to F48F7.1 [C.elegans]//8.2e-82:418:96//Hs.156161:AI333779
F-PLACE1004156//ESTs//0.10:166:63//Hs.133279:AI053552
F-PLACE1004161//Human mRNA for KIAA0200 gene, complete cds//0.85:269:64//Hs.76986:D83785
F-PLACE1004183//EST//1.3e-40:224:94//Hs.156603:AI343666
F-PLACE1004197//ESTs//2.8e-91:441:98//Hs.97269:AA292201
F-PLACE1004203//Homo sapiens GPI-anchored membrane protein CDw108 precursor, mRNA, complete cds//1.3e-145:695:98//Hs.24640:AF069493
F-PLACE1004242//ESTs//0.99:213:60//Hs.117311:AA699722
F-PLACE1004256//EST//0.019:364:58//Hs.122395:AA789273
F-PLACE1004257//ESTs//0.77:154:64//Hs.112582:AA608689
F-PLACE1004258//ESTs, Weakly similar to vanilloid receptor subtype 1 [R.norvegicus]//1.1e-98:479:97//Hs.31718:N29128
F-PLACE1004270//Homo sapiens CAGF9 mRNA, partial cds//0.00010:369:63//Hs.110826:U80736
F-PLACE1004274//Homo sapiens mRNA for KIAA0445 protein, complete cds//0.085:573:56//Hs.154139:AB007914
F-PLACE1004277//Homo sapiens two pore domain K+ channel (TASK-2) mRNA, complete cds//2.0e-157:756:97//Hs.127007:AF084830
F-PLACE1004284//ESTs//3.6e-71:344:99//Hs.145870:AI271884
F-PLACE1004289//ESTs//2.6e-57:370:85//Hs.16740:AA586576
F-PLACE1004302//FACTOR VIII INTRON 22 PROTEIN//0.032:513:59//Hs.83363:M34677
F-PLACE1004316//H.sapiens mRNA for apoptosis specific protein//9.3e-152:797:94//Hs.11171:Y11588
F-PLACE1004336
F-PLACE1004358//Homo sapiens connector enhancer of KSR-like protein CNK1 mRNA, complete cds//1.9e-140:688:97//Hs.16232:AF100153
F-PLACE1004376//ESTs, Weakly similar to F27D4.4 [C.elegans]//3.9e-109:521:98//Hs.14079:AA306552
F-PLACE1004384//Human HsLIM15 mRNA for HsLiml5, complete cds//2.0e-49:466:76//Hs.37181:D64108
F-PLACE1004388
F-PLACE1004405//EST//0.010:191:64//Hs.147600:AI217871
F-PLACE1004425//ESTs//2.1e-20:124:80//Hs.94195:W03579
F-PLACE1004428//H.sapiens mRNA for Branched chain Acyl-CoA Oxidase//1.0:552:58//Hs.9795:X95190
F-PLACE1004437//Human NAD⁺-specific isocitrate dehydrogenase beta subunit precursor, mRNA, nuclear gene encoding mitochondrial protein, complete cds//9.9e-131:536:99//Hs.155410:U49283
F-PLACE1004451//ESTs//5.9e-18:203:73//Hs.156097:AI348867
F-PLACE1004460
F-PLACE1004467//ESTs//8.0e-17:345:66//Hs.112993:AA824363
F-PLACE1004471//EST//9.3e-69:463:84//Hs.116391:AA644085
F-PLACE1004473//ESTs//0.93:358:58//Hs.33263:AA724416
F-PLACE1004491//EST//2.5e-58:285:99//Hs.97603:AA398163
F-PLACE1004506//CD81 ANTIGEN//7.2e-06:228:63//Hs.54457:M33680
F-PLACE1004510//Homo sapiens cofactor of initiator function (CIF150) mRNA, complete cds//2.5e-147:699:97//Hs.122752:AF026445
F-PLACE1004516//EST//1.0e-26:343:71//Hs.142595:N24150
F-PLACE1004518
F-PLACE1004548//EST//0.84:193:62//Hs.99583:AA461314
F-PLACE1004550//ESTs, Weakly similar to No definition line found [C.elegans]//4.0e-120:627:94//Hs.107387:AA058854
F-PLACE1004564//EST//1.0:240:62//Hs.16824:T91371
F-PLACE1004629//Centromere protein B (80kD)//0.0015:242:64//Hs.85004:X05299
F-PLACE1004645
F-PLACE1004646//Retinal pigment epithelium-specific protein (65kD)//1.4e-12:386:63//Hs.2133:U18991
F-PLACE1004658//ESTs//0.52:273:61//Hs.97252:AA291590
F-PLACE1004664
F-PLACE1004672//Human ABL gene, exon 1b and intron 1b, and putative M8604 Met protein (M8604 Met) gene//1.5e-66:357:95//Hs.77705:U07563
F-PLACE1004674//Homo sapiens calcium binding protein (ALG-2) mRNA, complete cds//1.4e-110:625:91//Hs.80019:AF035606
F-PLACE1004681//EST//0.00092:303:61//Hs.149560:AI281589
F-PLACE1004686//ESTs//3.0e-31:186:76//Hs.139130:AA704561
F-PLACE1004691//Homo sapiens clone 23963 mRNA sequence//0.54:242:61//Hs.48483:AF007131
F-PLACE1004693//ESTs, Weakly similar to pot. ORF III [H.sapiens]//0.56:96:71//Hs.125740:AA884845
F-PLACE1004716//ESTs//2.0e-79:388:98//Hs.150999:AI306542
F-PLACE1004722//ESTs//7.5e-06:105:72//Hs.128796:AA485891
F-PLACE1004736//ESTs//1.7e-27:203:86//Hs.119593:AA700148
F-PLACE1004740//ESTs//1.0e-25:174:89//Hs.29696:AA910680
F-PLACE1004743
F-PLACE1004751//ESTs, Highly similar to CMP-N-ACETYLNEURAMINATE-BETA-1,4-GALACTOSIDE ALPHA-2,3-SIALYLTRANSFERASE [Rattus norvegicus]//2.0e-41:260:90//Hs.6863:W52470
F-PLACE1004773//Homo sapiens inversin protein mRNA, complete cds//1.7e-172:828:97//Hs.104715:AF084367
F-PLACE1004777//Human myosin IXb mRNA, complete cds//1.0e-29:556:63//Hs.159629:U42391
F-PLACE1004793
F-PLACE1004804
F-PLACE1004813//EST//2.8e-42:296:83//Hs.155725:AI310340
F-PLACE1004814//ESTs, Weakly similar to U1 SMALL NUCLEAR RIBONUCLEOPROTEIN 70 KD [Xenopus laevis]//2.4e-78:415:95//Hs.80965:AA493284
F-PLACE1004815//Human mRNA for KIAA0364 gene, complete cds//4.3e-14:294:69//Hs.22111:AB002362
F-PLACE1004824//ESTs//0.0072:128:69//Hs.164062:AA934047
F-PLACE1004827//ESTs//0.78:38:100//Hs.18925:W30943
F-PLACE1004836//Homo sapiens Notch3 (NOTCH3) mRNA, complete cds//0.78:338:57//Hs.8546:U97669
F-PLACE1004838
F-PLACE1004840//Protein phosphatase 1, catalytic subunit, beta isoform//0.89:200:66//Hs.21537:X80910
F-PLACE1004868
F-PLACE1004885//ESTs//0.41:181:61//Hs.116796:AA633772
F-PLACE1004900
F-PLACE1004902//ESTs//4.7e-72:367:96//Hs.54971:AI424382
F-PLACE1004913//ESTs//0.031:166:63//Hs.130110:AA904929
F-PLACE1004918//Human tumor susceptiblity protein (TSG101) mRNA, complete cds//4.1e-24:402:64//Hs.118910:U82130
F-PLACE1004930//Homo sapiens TNF-induced protein GG2-1 mRNA, complete cds//9.7e-86:519:88//Hs.17839:AF099936
F-PLACE1004934//ESTs//7.2e-43:231:78//Hs.133503:AA628592
F-PLACE1004937//ESTs//0.97:80:68//Hs.144264:C00851
F-PLACE1004969
F-PLACE1004972//Human retinoic acid- and interferon-inducible 58K protein RI58 mRNA, complete cds//0.031:235:60//Hs.27610:U34605
F-PLACE1004979//Homo sapiens mRNA for KIAA0575 protein, complete cds//4.9e-43:331:83//Hs.153468:AB011147
F-PLACE1004982//ESTs//0.020:148:63//Hs.129377:AI218520
F-PLACE1004985//ESTs//7.9e-05:372:61//Hs.87606:AA242831
F-PLACE1005026//ESTs//4.6e-29:212:89//Hs.137451:AA351459
F-PLACE1005027//ESTs//6.5e-91:455:97//Hs.30890:H15159
F-PLACE1005046//ESTs//3.7e-56:250:96//Hs.152730:AI308943
F-PLACE1005052//EST//1.8e-36:370:73//Hs.123424:AA813594
F-PLACE1005055//Homo sapiens mRNA for KIAA0576 protein, partial cds//6.2e-161:761:98//Hs.14687:AB011148
F-PLACE1005066//Homo sapiens actin binding protein MAYVEN mRNA, complete cds//3.0e-11:757:56//Hs.122967:AF059569
F-PLACE1005077//EST//0.79:283:591/Hs.89276:AA283899
F-PLACE1005085//ESTs//3.5e-18:231:72//Hs.142654:AA324740
F-PLACE1005086//Homo sapiens mRNA for KIAA0575 protein, complete cds//1.9e-49:401:80//Hs.153468:AB011147
F-PLACE1005101//Homo sapiens (clone zapl28) mRNA, 3' end of cds//8.2e-20:194:80//Hs.75437:L40401
F-PLACE1005102//Homo sapiens HIV-1 inducer of short transcripts binding protein (FBI1) mRNA, complete cds//8.9e-18:538:62//Hs.104640:AF000561
F-PLACE1005108//Treacher Collins syndrome susceptibility protein//0.73:405:57//Hs.73166:U76366
F-PLACE1005111//ESTs//0.66:191.63//Hs.106446:N93227
F-PLACE1005128//Breakpoint cluster region protein BCR//5.6e-08:291:63//Hs.2557:Y00661
F-PLACE1005146//ESTs, Weakly similar to hypothetical protein II [H.sapiens]//4.8e-12:360:63//Hs.142177:H11741
F-PLACE1005162//Human mRNA for KIAA0118 gene, partial cds//3.9e-49:563:72//Hs.154326:D42087
F-PLACE1005176//Homo sapiens mRNA for KIAA0641 protein, complete cds//0.82:259:60//Hs.128316:AB014541
F-PLACE1005181//ESTs, Weakly similar to No definition line found [C.elegans]//4.4e-126:583:99//Hs.25347:AI138605
F-PLACE1005187//ESTs//6.2e-34:222:90//Hs.124265:N70417
F-PLACE1005206//EST//0.089:167:62//Hs.140487:AA767009
F-PLACE1005232//ESTs, Weakly similar to synapse-associated protein sap47-1 [D.melanogaster]//0.56:192:60//Hs.47334:W72370
F-PLACE1005243
F-PLACE1005261//ESTs//0.52:245:58//Hs.6682:T76941
F-PLACE1005266//Kallmann syndrome 1 sequence//7.8e-06:484:60//Hs.89591:M97252
F-PLACE1005277//Homo sapiens mRNA for KIAA0610 protein, partial cds//5.1e-150:706:98//Hs.118087:AB011182
F-PLACE1005287//ESTs//8.1e-107:501:99//Hs.145703:AA447947
F-PLACE1005305//GTP:AMP PHOSPHOTRANSFERASE MITOCHONDRIAL//4.4e-37:597:66//Hs.101642:X60673
F-PLACE1005308//High-mobility group (nonhistone chromosomal) protein 2//0.83:239:62//Hs.80684:X62534
F-PLACE1005313
F-PLACE1005327//ESTs, Weakly similar to No definition line found [C.elegans]//6.0e-81:459:91//Hs.146177:R51650
F-PLACE1005331//Homo sapiens chromosome 19, cosmid F20569//3.7e-66:412:88//Hs.134031:AC004794
F-PLACE1005335//Homo sapiens mRNA for KIAA0754 protein, partial cds//0.96:510:56//Hs.159183:AB018297
F-PLACE1005373
F-PLACE1005374//ESTs//7.5e-77:437:91//Hs.143266:AI141348
F-PLACE1005409//ESTs//2.4e-05:267:63//Hs.163307:AA856751
F-PLACE1005453//ESTs//0.12:333:58//Hs.134672:AI087951
F-PLACE1005467//HOMEOBOX/POU DOMAIN PROTEIN RDC-1//0.0043:148:67//Hs.74095:L20433
F-PLACE1005471//ESTs//3.4e-24:135:97//Hs.49275:N66925
F-PLACE1005477//Human Line-1 repeat mRNA with 2 open reading frames//3.5e-126:744:87//Hs.23094:M19503
F-PLACE1005480//ESTs//3.7e-26:184:70//Hs.113198:N39323
F-PLACE1005481//EST//0.27:153-:64//Hs.120066:AA707973
F-PLACE1005494//ESTs//2.4e-50:257:98//Hs.159003:AA633029
F-PLACE1005502//ESTs//0.15:408:57//Hs.45106:AA504105
F-PLACE1005526//ESTs//3.2e-61:305:98//Hs.122574:AA776747
F-PLACE1005528//ESTs//9.9e-32:249:78//Hs.142531:N91572
F-PLACE1005530//ESTs//1.0e-94:491:95//Hs.131731:AI339335
F-PLACE1005550//ESTs//0.084:290:58//Hs.157775:AI359385
F-PLACE1005554//EST//0.38:213:58//Hs.102749:N64144
F-PLACE1005557//ESTs, Highly similar to MITOCHONDRIAL 60S RIBOSOMAL PROTEIN L2 PRECURSOR [Saccharomyces cerevisiae]//4.5e-51:258:97//Hs.7736:W81261
F-PLACE1005574//ESTs//3.2e-09:236:66//Hs.146884:AI160278
F-PLACE1005584//Fragile X mental retardation 2//1.2e-05:151:69//Hs.54472:U48436
F-PLACE1005595//ESTs//2.1e-98:512:95//Hs.118552:W74594
F-PLACE1005603//EST//1.0:90:66//Hs.111204:AA211851
F-PLACE1005611//ESTs, Weakly similar to B0035.14 [C.elegans]//3.5e-32:197:92//Hs.8241:AA283057
F-PLACE1005623//ESTs//3.0e-30:191:92//Hs.77570:N48234
F-PLACE1005630//ESTs//2.3e-32:175:97//Hs.122278:AA781867
F-PLACE1005639//ESTs//0.88:218:58//Hs.117389:AA701991
F-PLACE1005646//Homo sapiens RNA helicase-related protein mRNA, complete cds//2.1e-151:721:98//Hs.8765:AF083255
F-PLACE1005656//Ribonucleotide reductase M2 polypeptide//3.9e-53:480:74//Hs.75319:X59618
F-PLACE1005666//Homo sapiens mRNA for KIAA0448 protein, complete cds//0.086:223:59//Hs.27349:AB007917
F-PLACE1005698//Human membrane-associated lectin type-C mRNA//6.1e-65:374:85//Hs.23759:M98457
F-PLACE1005727//ESTs//8.7e-65:330:96//Hs.127027:AA935437
F-PLACE1005730//ESTs//2.9e-14:270:67//Hs.28589:AI004944
F-PLACE1005739//Homo sapiens mRNA for serin protease with IGF-binding motif, complete cds//0.75:289:59//Hs.75111:D87258
F-PLACE1005755//Insulin-like growth factor binding protein 2//3.6e-05:377:62//Hs.162:X16302
F-PLACE1005763//ESTs, Highly similar to S-ACYL FATTY ACID SYNTHASE THIOESTERASE, MEDIUM CHAIN [Rattus norvegicus]//5.7e-49:252:88//Hs.24309:AI125696
F-PLACE1005799//ESTs//5.2e-13:392:58//Hs.110530:AA191493
F-PLACE1005802
F-PLACE1005803
F-PLACE1005804//Homo sapiens alpha 1,2-mannosidase IB mRNA, complete cds//4.5e-128:636:96//Hs.125315:AF027156
F-PLACE1005813//Homo sapiens sorting nexin 2 (SNX2) mRNA, complete cds//8.4e-156:739:98//Hs.11183:AF065482
F-PLACE1005828//ESTs, Weakly similar to !!!! ALU SUBFAMILY J WARNING ENTRY !!!! [H.sapiens]//4.1e-42:327:81//Hs.138404:R70986
F-PLACE1005834//Retinoblastoma 1 (including osteosarcoma)//0.038:436:58//Hs.75770:L41870
F-PLACE1005845//ESTs//4.8e-50:309:89//Hs.107149:AI379497
F-PLACE1005850//ESTs//7.1e-40:253:79//Hs.158096:AA186905
F-PLACE1005851//ESTs//7.6e-93:483:95//Hs.135608:AA732242
F-PLACE1005876//ESTs//0.97:282:60//Hs.98664:AI381487
F-PLACE1005884//ESTs//0.070:276:60//Hs.106057:AI031552
F-PLACE1005890//ESTs//1.5e-91:500:93//Hs.136993:AA843300
F-PLACE1005898
F-PLACE1005921
F-PLACE1005923//ESTs//0.50:308:58//Hs.52489:R61504
F-PLACE1005925//ESTs//0.024:93:68//Hs.149868:AI288274
F-PLACE1005932//TYROSINE-PROTEIN KINASE RECEPTOR EPH PRECURSOR//0.97:342:57//Hs.89839:M18391
F-PLACE1005934//ESTs//8.6e-10:74:93//Hs.25092:AA922142
F-PLACE1005936//DNA excision repair protein ERCC5//1.0:144:63//Hs.48576:X69978
F-PLACE1005951//B94 PROTEIN//0.00025:371:61//Hs.75522:M92357
F-PLACE1005953//ESTs//2.8e-06:290:61//Hs.140996:R73468
F-PLACE1005955//ESTs, Weakly similar to Y53C12A.3 [C.elegans]//0.15:136:66//Hs.107747:AI357868
F-PLACE1005966//Human zinc fmger/leucine zipper protein (AF10) mRNA, complete cds//1.0:215:63//Hs.7885:U13948
F-PLACE1005968
F-PLACE1005990
F-PLACE1006002//Putative mismatch repair/binding protein hMSH3//1.9e-48:312:77//Hs.42674:U61981
F-PLACE1006003//EST//0.00018:171:67//Hs.138882:W73256
F-PLACE1006011
F-PLACE1006017//ESTs//3.1e-21:159:88//Hs.142173:AA757743
F-PLACE1006037//Homo sapiens mRNA for KIAA0789 protein, complete cds//0.021:202:64//Hs.158319:AB018332
F-PLACE1006040//Homo sapiens mRNA for alpha endosulfine//1.1e-148:719:97//Hs.98782:X99906
F-PLACE1006076//EST//0.29:92:64//Hs.161536:N80395
F-PLACE1006119//Homo sapiens Ran-GTP binding protein mRNA, partial cds//4.1e-147:679:99//Hs.4976:AF039023
F-PLACE1006129
F-PLACE1006139
F-PLACE1006143//Human mRNA for KIAA0355 gene, complete cds//9.3e-43:357:79//Hs.153014:AB002353
F-PLACE1006157//ESTs, Weakly similar to ETX1 {alternatively spliced} [H.sapiens]//2.9e-12:119:84//Hs.23153:R92857
F-PLACE1006159//ESTs//2.3e-87:443:96//Hs.23740:H17868
F-PLACE10061641/ESTs//0.099:223:60//Hs.8108:AA902721
F-PLACE1006167//Homo sapiens chromosome 19, cosmid F23149//1.1e-68:333:92//Hs.152894:AC005239
F-PLACE1006170//ESTs//0.081:171:67//Hs.135187:AI074005
F-PLACE1006187//Homo sapiens cyclin E2 mRNA, complete cds//1.2e-150:694:99//Hs.30464:AF091433
F-PLACE1006195//ESTs//8.9e-14:229:70//Hs.141470:N49608
F-PLACE1006196//ESTs, Weakly similar to protein synthesis initiation factor 4A-II homolog//3.5e-59:369:88//Hs.135623:AA134719
F-PLACE1006205
F-PLACE1006223//ESTs, Weakly similar to TERATOCARCINOMA-DERIVED GROWTH FACTOR 1 [H.sapiens]//0.0089:166:63//Hs.127179:AI279486
F-PLACE1006225
F-PLACE1006236//EST//0.060:89:69//Hs.136977:AA830668
F-PLACE1006239//ESTs//0.028:105:66//Hs.142336:AA358185
F-PLACE1006246//ESTs//0.060:330:60//Hs.105695:AI085802
F-PLACE1006248//Homo sapiens mRNA for KIAA0648 protein, partial cds//7.3e-168:791:98//Hs.31921:AB014548
F-PLACE1006262
F-PLACE1006288//Homo sapiens mRNA for Pex3 protein//4.8e-37:186:100//Hs.7277:AJ001625
F-PLACE1006318
F-PLACE1006325//ESTs//3.7e-25:206:83//Hs.102319:AI246503
F-PLACE1006335//ESTs//2.0e-27:161:95//Hs.163529:AI361492
F-PLACE1006357//ESTs//0.013:268:61//Hs.105775:AA526249
F-PLACE1006360//ESTs//4.8e-27:146:98//Hs.100739:Z98481
F-PLACE1006368//Homo sapiens clone 24540 mRNA sequence//0.65:272:59//Hs.153529:AF070581
F-PLACE1006371//Homo sapiens jerky gene product homolog mRNA, complete cds//2.6e-07:403:61//Hs.105940:AF004715
F-PLACE1006382//EST//0.98:77:68//Hs.136933:AA814693
F-PLACE1006385//Homo sapiens epsin 2b mRNA, complete cds//1.6e-111:539:97//Hs.22396:AF062085
F-PLACE1006412//Human mRNA for KIAA0298 gene, complete cds//1.0e-36:424:74//Hs.21560:AB002296
F-PLACE1006414//Homo sapiens PCAF associated factor 65 alpha mRNA, complete cds//4.3e-111:525:98//Hs.131846:AF069735
F-PLACE1006438//Homo sapiens mRNA for KIAA0557 protein, partial cds//2.2e-24:531:65//Hs.101414:AB011129
F-PLACE1006445//Homo sapiens chromosome 16 zinc finger protein ZNF200 (ZNF200) mRNA, complete cds//1.0:248:60//Hs.88219:AF060866
F-PLACE1006469//Human SA mRNA for SA gene product, complete cds//0.24:210:62//Hs.89659:AC004381
F-PLACE1006470
F-PLACE1006482//Homo sapiens basic-leucine zipper transcription factor MafK (MAFK) mRNA, complete cds//5.0e-46:520:71//Hs.131953:AF059194
F-PLACE1006488//ESTs//6.2e-47:239:97//Hs.158161:AA312511
F-PLACE1006492//ESTs//0.82:37:100//Hs.160417:AA488493
F-PLACE1006506//HUMAN IMMUNODEFICIENCY VIRUS TYPE I ENHANCER-BINDING PROTEIN 2//0.98:505:56//Hs.75063:AL023584
F-PLACE1006521//ESTs//0.032:222:63//Hs.23171:AA706542
F-PLACE1006531//EST//2.1e-53:258:100//Hs.117316:AA699358
F-PLACE1006534//EST//1.8e-07:78:89//Hs.157551:AI356219
F-PLACE1006540//Homo sapiens mRNA for cadherin-6, complete cds//0.96:383:58//Hs.32963:D31784
F-PLACE1006552//Human (clone N5-4) protein p84 mRNA, complete cds//0.058:464:57//Hs.1540:L36529
F-PLACE1006598//Homo sapiens mRNA for KIAA0737 protein, complete cds//4.1e-17:372:65//Hs.17630:AB018280
F-PLACE1006615//Homo sapiens eukaryotic translation initiation factor eIF3, p35 subunit mRNA, complete cds//2.2e-168:781:99//Hs.155377:U97670
F-PLACE1006617//ESTs//6.0e-08:354:60//Hs.42624:H99088
F-PLACE1006626//NUCLEOLIN//0.0044:186:66//Hs.79110:M60858
F-PLACE1006629//Homo sapiens (clone s22i71) mRNA fragment//0.097:229:63//Hs.26956:L40396
F-PLACE1006640//ESTs//0.00019:380:59//Hs.13672:AI131473
F-PLACE1006673//ESTs, Weakly similar to T14B4.2 gene product [C.elegans]//1.6e-12:113:83//Hs.3385:N25917
F-PLACE1006678
F-PLACE1006704//Homo sapiens ALR mRNA, complete cds//0.16:284:60//Hs.153638:AF010403
F-PLACE1006731//Homo sapiens SOX22 protein (SOX22) mRNA, complete cds//1.6e-05:382:63//Hs.43627:U35612
F-PLACE1006754//Biliary glycoprotein//8.9e-27:305:72//Hs.50964:X16354
F-PLACE1006760//ESTs//0.10:207:62//Hs.152589:AA954152
F-PLACE1006779//Kallmann syndrome 1 sequence//0.00025:251:64//Hs.89591:M97252
F-PLACE1006782//ESTs//1.2e-90:423:100//Hs.132826:AI075783
F-PLACE1006792//ESTs//1.5e-10:439:58//Hs.138501:AI051228
F-PLACE1006795//TYROSINE-PROTEIN KINASE RECEPTOR ETK1 PRECURSOR//4.5e-10:84:95//Hs.123642:M83941
F-PLACE1006800//ESTs//0.00068:360:61//Hs.157876:AI422017
F-PLACE1006805//ESTs//4.6e-103:491:98//Hs.140465:AA769892
F-PLACE1006815//Homo sapiens mRNA for KIAA0618 protein, complete cds//0.47:403:56//Hs.15832:AB014518
F-PLACE1006819//Human Line-1 repeat mRNA with 2 open reading frames//3.7e-103:619:87//Hs.23094:M19503
F-PLACE1006829//ESTs//1.5e-22:141:94//Hs.142988:AA142876
F-PLACE1006860//EST//0.0062:206:65//Hs.158793:AI376773
F-PLACE1006867//ESTs//0.068:218:62//Hs.91166:AA551273
F-PLACE1006878//Homo sapiens mRNA for KIAA0711 protein, complete cds//1.0:268:58//Hs.5333:AB018254
F-PLACE1006883//ESTs//1.6e-75:398:94//Hs.119544:T95601
F-PLACE1006901//ESTs//1.9e-13:87:96//Hs.134737:AI089187
F-PLACE1006904//EST//1.0:91:70//Hs.148270:AA906443
F-PLACE1006917
F-PLACE1006932//ESTs//0.98:110:70//Hs.100855:AI423913
F-PLACE1006935//EST//1.0:92:65//Hs.124554:AA847211
F-PLACE1006956//PERIPHERIN//0.13:443:57//Hs.37044:L14565
F-PLACE1006958//Heat shock 70kD protein 4//6.4e-40:456:70//Hs.127:L12723
F-PLACE1006961//ESTs, Highly similar to RSP5 PROTEIN [Saccharomyces cerevisiae]//3.2e-07:67:98//Hs.21806:AA630312
F-PLACE1006962//H.sapiens ir1B mRNA//2.3e-16:202:71//Hs.135202:X63417
F-PLACE1006966//Homo sapiens syntaxin 4 binding protein UNC-18c (UNC-18c) mRNA, complete cds//0.14:191:67//Hs.8813:AF032922
F-PLACE1006989//Cyclin B1//0.99:224:59//Hs.23960:M25753
F-PLACE1007014//Homo sapiens NBMPR-insensitive nucleoside transporter ei (ENT2) mRNA, complete cds//3.1e-05:594:58//Hs.32951:AF034102
F-PLACE1007021//ESTs//7.2e-89:446:96//Hs.7111:U55971
F-PLACE1007045//Human Line-1 repeat mRNA with 2 open reading frames//1.0e-117:775:84//Hs.23094:M19503
F-PLACE1007053//Homo sapiens mRNA for ARNO3 protein//0.35:63:82//Hs.129811:AJ223957
F-PLACE1007068//Polycystic kidney disease 1 (autosomal dominant)//0.22:361:60//Hs.75813:L33243
F-PLACE1007097//ESTs//2.9e-25:197:83//Hs.105665:H78987
F-PLACE1007105//Amylo-1,6-glucosidase, 4-alpha-glucanotransferase (glycogen debranching enzyme, glycogen storage disease type III)//0.18:268:63//Hs.904:U84010
F-PLACE1007111//EST//0.0066:260:60//Hs.147903:AI223385
F-PLACE1007112
F-PLACE1007132//ESTs//3.1e-30:195:76//Hs.46158:AI160121
F-PLACE1007140//TRANSCRIPTION ELONGATION FACTOR S-II//0.13:302:60//Hs.78869:M81601
F-PLACE1007178//ESTs//9.6e-54:289:95//Hs.12251:H12965
F-PLACE1007226//Homo sapiens Notch3 (NOTCH3) mRNA, complete cds//0.00090:412:59//Hs.8546:U97669
F-PLACE1007238//Human plectin (PLEC1) mRNA, complete cds//1.4e-07:492:64//Hs.79706:U53204
F-PLACE1007239//Human mRNA for transcription elongation factor S-II, hS-II-T1, complete cds//2.0e-58:405:87//Hs.80598:D50495
F-PLACE1007242//EST//0.014:55:89//Hs.88432:AA262141
F-PLACE1007243//ESTs//2.0e-43:227:97//Hs.124775:AA648467
F-PLACE1007257//Homo sapiens mRNA for dia-156 protein//3.7e-144:677:98//Hs.121556:Y15909
F-PLACE1007274
F-PLACE1007276//ATPase, Cu++ transporting, alpha polypeptide (Menkes syndrome)//0.94:167:64//Hs.606:L06133
F-PLACE1007282
F-PLACE1007286//ESTs//1.0e-25:333:71//Hs.134860:AI091436
F-PLACE1007301//EST//0.78:171:61//Hs.160990:H52412
F-PLACE1007317//Homo sapiens oxysterol 7alpha-hydroxylase (CYP7b1) mRNA, complete cds//0.88:298:58//Hs.144877:AF029403
F-PLACE1007342
F-PLACE1007346//Homo sapiens estrogen-responsive B box protein (EBBP) mRNA, complete cds//1.7e-121:567:98//Hs.76596:AF096870
F-PLACE1007367//H.sapiens mRNA for MACH-alpha-2 protein//2.2e-55:532:77//Hs.19949:X98173
F-PLACE1007375
F-PLACE1007386//ESTs//0.00066:61:91//Hs.149318:AI248642
F-PLACE1007402//EST//1.7e-06:193:65//Hs.132124:AI041287
F-PLACE1007409//Homo sapiens mitoxantrone resistance protein 1 mRNA, partial sequence//3.8e-18:128:92//Hs.14387:AF093771
F-PLACE1007416
F-PLACE1007450//ESTs//2.6e-36:194:97//Hs.22359:AI024436
F-PLACE1007452//EST//1.8e-34:197:94//Hs.134795:AI090359
F-PLACE1007454//Homo sapiens (clone s153) mRNA fragment//2.6e-53:317:93//Hs.6445:L40391
F-PLACE1007460//ESTs//0.0012:168:64//Hs.151708:AA554714
F-PLACE1007478//ESTs//1.0e-42:440:74//Hs.141722:AA769103
F-PLACE1007484//ESTs//7.1e-18:127:91//Hs.100251:AA535975
F-PLACE1007488
F-PLACE1007507//ESTs//1.2e-99:274:98//Hs.123462:AA903385
F-PLACE1007511//Keratin 19//4.2e-31:586:64//Hs.23761:Y00503
F-PLACE1007524//ESTs//6.8e-71:356:97//Hs.163067:AA897296
F-PLACE1007525//ESTs//0.073:242:59//Hs.128711:AA856979
F-PLACE1007537//Homo sapiens PYRIN (MEFV) mRNA, complete cds//0.93:468:57//Hs.113283:AF018080
F-PLACE1007544//ESTs//1.7e-74:360:98//Hs.128632:AI076755
F-PLACE1007547//Homo sapiens mRNA for KIAA0661 protein, complete cds//1.0e-70:733:71//Hs.65238:AB014561
F-PLACE1007557//EST//0.58:80:72//Hs.130267:AI001863
F-PLACE1007583//ESTs//1.8e-46:234:98//Hs.155071:AA584257
F-PLACE1007598//ESTs//1.7e-83:400:99//Hs.120206:AI089163
F-PLACE1007618//Homo sapiens mRNA for KIAA0633 protein, partial cds//7.2e-12:778:56//Hs.33010:AB014533
F-PLACE1007621
F-PLACE1007632//ESTs//1.7e-32:175:97//Hs.122278:AA781867
F-PLACE1007645
F-PLACE1007649
F-PLACE1007677//ESTs//3.0e-13:125:82//Hs.143382:AA476266
F-PLACE1007688//ESTs//6.8e-06:311:61//Hs.132926:AI027055
F-PLACE1007690//ESTs//1.9e-13:83:98//Hs.150088:AI348503
F-PLACE1007697//TRANSFORMING GROWTH FACTOR BETA 1 PRECURSOR//0.99:216:63//Hs.1103:X02812
F-PLACE1007705//Human mRNA for RTP, complete cds//4.8e-58:637:70//Hs.75789:D87953
F-PLACE1007706//Homo sapiens metalloprotease 1 (MP1) mRNA, complete cds//4.1e-149:709:97//Hs.4812:AF061243
F-PLACE1007725//ESTs, Weakly similar to No definition line found [C.elegans]//4.5e-36:233:89//Hs.108797:AA476815
F-PLACE1007729//ESTs, Moderately similar to RETRO VIRUS-RELATED PROTEASE [H.sapiens]//0.00033:270:64//Hs.104129:AA923278
F-PLACE1007730//Homo sapiens mRNA for KIAA0685 protein, complete cds//2.6e-156:728:98//Hs.153121:AB014585
F-PLACE1007737//Coagulation factor II (thrombin) receptor//1.1e-18:364:68//Hs.159347:M62424
F-PLACE1007743//ESTs//0.029:421:58//Hs.106090:AA457030
F-PLACE1007746//ESTs//6.7e-55:330:89//Hs.153392:AI089469
F-PLACE1007791//EST//0.39:261:62//Hs.145991:AI277656
F-PLACE1007807//ESTs//2.0e-54:385:83//Hs.163930:AA640504
F-PLACE1007810//ESTs//6.1e-53:416:81//Hs.152395:AA533107
F-PLACE1007829//EST//0.28:271:61//Hs.125514:AA883841
F-PLACE1007843//EST//0.020:307:59//Hs.145535:AI261635
F-PLACE1007846//Human Line-1 repeat mRNA with 2 open reading frames//6.3e-38:396:77//Hs.23094:M19503
F-PLACE1007852
F-PLACE1007858//Homo sapiens mRNA for KIAA0766 protein, complete cds//1.3e-190:894:98//Hs.28020:AB018309
F-PLACE1007866//ESTs//3.0e-50:333:86//Hs.15792:AI038387
F-PLACE1007877
F-PLACE1007897//EST//1.0:59:72//Hs.138770:N70943
F-PLACE1007908//Homo sapiens mRNA, chromosome 1 specific transcript KIAA0487//7.3e-156:755:97//Hs.92381:AB007956
F-PLACE1007946//ESTs//8.9e-16:250:68//Hs.88527:N24002
F-PLACE1007954//ESTs//1.6e-05:76:90//Hs.63314:AA056538
F-PLACE1007955//Homo sapiens cyclin-D binding Myb-like protein mRNA, complete cds//8.9e-173:813:98//Hs.5671:AF084530
F-PLACE1007958//Homo sapiens cAMP-specific phosphodiesterase 8B (PDE8B) mRNA, partial cds//8.2e-155:730:98//Hs.78106:AF079529
F-PLACE1007969//ESTs, Weakly similar to hnRNA-binding protein M4 [H.sapiens]//5.1e-45:264:92//Hs.42222:W28567
F-PLACE1007990//ESTs//1.2e-104:493:99//Hs.118445:AI097043
F-PLACE1008000//Homo sapiens veli 1 mRNA, complete cds//5.7e-63:578:74//Hs.150380:AF087693
F-PLACE1008002//ESTs//0.52:236:59//Hs.134292:AA603031
F-PLACE1008044
F-PLACE1008045//COL10A1//0.29:221:58//Hs.37075:X60382
F-PLACE1008080//Human homeodomain protein (Prox 1) mRNA, complete cds//0.00037:151:71//Hs.159437:U44060
F-PLACE1008095//Human hybrid receptor gp250 precursor mRNA, complete cds//1.0:461:58//Hs.155494:U60975
F-PLACE1008111//Homo sapiens B lymphocyte chemoattractant BLC mRNA, complete cds//0.034:497:58//Hs.100431:AF044197
F-PLACE1008122//ESTs//0.95:198:60//Hs.126776:N28769
F-PLACE1008129//ESTs//1.1e-99:499:96//Hs.131807:AA778874
F-PLACE1008132//EST//3.3e-27:218:83//Hs.145258:AI218683
F-PLACE1008177//ESTs, Moderately similar to meiosis-specific nuclear structural protein 1 [M.musculus]//5.1e-20:124:95//Hs.146238:AI263135
F-PLACE1008181//ESTs//0.018:285:61//Hs.88843:AA281427
F-PLACE1008198//ESTs//5.9e-07:410:60//Hs.63348:AA643524
F-PLACE1008201
F-PLACE1008209
F-PLACE1008231//ESTs//0.40:188:61//Hs.130266:AI001856
F-PLACE1008244//Miller-Dieker syndrome chromosome region//0.22:247:61//Hs.77318:L13385
F-PLACE1008273
F-PLACE1008275//EST//0.77:74:71//Hs.145907:AI275113
F-PLACE1008280//Homo sapiens mRNA, chromosome 1 specific transcript KIAA0501//2.6e-25:389:70//Hs.159897:AB007970
F-PLACE1008309//Homo sapiens serine phosphatase FCP1a (FCP1) mRNA, complete cds//0.16:263:63//Hs.4076:AF081287
F-PLACE1008329//EST//1.3e-09:94:85//Hs.144135:R82071
F-PLACE1008330//Homo sapiens mRNA for KIAA0557 protein, partial cds//1.5e-45:291:83//Hs.101414:AB011129
F-PLACE1008331//ESTs, Weakly similar to ORF2-like protein [H.sapiens]//5.4e-74:356:98//Hs.105382:AA496362
F-PLACE1008356//Homo sapiens mRNA for KIAA0679 protein, partial cds//3.4e-139:659:98//Hs.5734:AB014579
F-PLACE1008368//Homo sapiens actin binding protein MAYVEN mRNA, complete cds//0.011:355:60//Hs.122967:AF059569
F-PLACE1008369//ESTs//0.00074:443:61//Hs.102756:AA526911
F-PLACE1008392//EST//7.4e-08:324:60//Hs.149930:AI289171
F-PLACE1008398
F-PLACE1008401//Homo sapiens methyl-CpG binding protein MBD2 (MBD2) mRNA, complete cds//2.5e-09:461:62//Hs.25674:AF072242
F-PLACE1008402//Homo sapiens mRNA for p115, complete cds//1.4e-149:711:98//Hs.7763:D86326
F-PLACE1008405//ESTs//2.8e-102:529:95//Hs.116278:AA628943
F-PLACE1008424//Human DNA sequence from clone 753P9 on chromosome Xq25-26.1. Contains the gene coding for Aminopeptidase P (EC 3.4.11.9, XAA-Pro/X-Pro/Proline/Aminoacylproline Aminopeptidase) and a novel gene. Contains ESTs, STSs, GSSs and a gaaa repeat polymorphism//0.98:113:67//Hs.57922:AL023653
F-PLACE1008426//ESTs//3.2e-77:393:95//Hs.37585:W28499
F-PLACE1008429//Orf1 5' to PD-ECGF/TP...orf2 5' to PD-ECGF/TP [human, epidermoid carcinoma cell line A431, mRNA, 3 genes, 1718 nt]//0.019:530:58//Hs.72248:S72487
F-PLACE1008437
F-PLACE1008455//ESTs//0.51:279:61//Hs.122319:AA782335
F-PLACE1008457//ESTs//3.0e-30:229:75//Hs.60740:AA053901
F-PLACE1008465//Human mRNA for KIAA0383 gene, partial cds//0.0084:210:63//Hs.27590:AB002381
F-PLACE1008488//Human density enhanced phosphatase-1 mRNA, complete cds//6.8e-07:469:60//Hs.1177:U10886
F-PLACE1008524//Homo sapiens TWIK-related acid-sensitive K+ channel (TASK) mRNA, complete cds//1.0:304:60//Hs.24040:AF006823
F-PLACE1008531//ESTs//1.1e-17:190:76//Hs.156041:AI274697
F-PLACE1008532//Thromboxane A2 receptor//5.6e-17:231:71//Hs.89887:D38081
F-PLACE1008533//Homo sapiens PAC clone DJ130H16 from 22q12.1-qter//1.1e-45:507:71//Hs.8003:AC004997
F-PLACE1008568//Homo sapiens mRNA for neuronatin alpha, complete cds//1.0:95:71//Hs.117546:U31767
F-PLACE1008584//ESTs//1.4e-13:252:68//Hs.153429:AI283069
F-PLACE1008603//Homo sapiens mRNA for KIAA0791 protein, complete cds//3.9e-175:812:98//Hs.23255:AB018334
F-PLACE1008621//ESTs, Weakly similar to reverse transcriptase [H.sapiens]//1.2e-15:350:66//Hs.151087:AA649326
F-PLACE1008625//ESTs//0.86:269:57//Hs.94998:N26794
F-PLACE1008626//ESTs//0.55:69:71//Hs.92096:F10560
F-PLACE1008627//ESTs//3.0e-62:302:99//Hs.120766:H82458
F-PLACE1008629//EST//0.0012:174:67//Hs.121195:AA757211
F-PLACE1008630//ESTs//4.5e-77:371:99//Hs.132960:AA252394
F-PLACE1008643//Human mRNA for PK-120//4.7e-25:299:64//Hs.76415:D38535
F-PLACE1008650//Homo sapiens pleiotropic regulator 1 (PLRG1) mRNA, complete cds//3.5e-135:622:99//Hs.147967:AF044333
F-PLACE1008693//EST//0.19:36:94//Hs.138817:N93728
F-PLACE1008696//Human mitochondrial NADH dehydrogenase-ubiquinone Fe-S protein 8, 23 kDa subunit precursor (NDUFS8) nuclear mRNA encoding mitochondrial protein, complete cds//8.3e-25:137:97//Hs.90443:AF038406
F-PLACE1008715//Homo sapiens mRNA for matrilin-3//0.99:183:63//Hs.119534:AJ224741
F-PLACE1008748//ESTs//0.88:204:63//Hs.15139:AA527080
F-PLACE1008757//ESTs, Weakly similar to unknown protein [R.norvegicus]//4.3e-17:285:69//Hs.35460:H65503
F-PLACE1008790//Homo sapiens importin alpha 7 subunit mRNA, complete cds//1.4e-121:503:97//Hs.6458:AF060543
F-PLACE1008798//ESTs, Weakly similar to putative p150 [H.sapiens]//0.30:127:68//Hs.111380:AA258772
F-PLACE1008807//ESTs//0.81:346:58//Hs.116901:AA663542
F-PLACE1008808//Homo sapiens putative checkpoint control protein HRAD1 mRNA, complete cds//6.7e-104:376:98//Hs.7179:AF011905
F-PLACE1008813//Glutamate decarboxylase 1 (brain, 67kD)//0.17:318:61//Hs.75668:M81883
F-PLACE1008851//ESTs, Highly similar to CELL DIVISION CONTROL PROTEIN 2 HOMOLOG [Plasmodium falciparum (isolate k1 / thailand)]//0.73:354:59//Hs.26322:AA156858
F-PLACE1008854//ESTs//3.0e-26:391:66//Hs.133260:AI052728
F-PLACE1008867//ESTs//5.9e-08:64:93//Hs.91115:AI221563
F-PLACE1008887//Human Line-1 repeat mRNA with 2 open reading frames//5.5e-51:701:68//Hs.23094:M19503
F-PLACE1008902//EST//0.85:425:60//Hs.140573:AA826323
F-PLACE1008920//Homo sapiens mRNA for KIAA0765 protein, partial cds//2.1e-159:753:98//Hs.62318:AB018308
F-PLACE1008925//ESTs//0.025:133:67//Hs.103218:W84771
F-PLACE1008934//ESTs//0.27:307:59//Hs.135168:AI394026
F-PLACE1008941//ESTs//3.3e-53:266:98//Hs.108677:AA488937
F-PLACE1008947//Human TBP-associated factor (hTAFII130) mRNA, partial cds//2.4e-13:625:58//Hs.24644:U75308
F-PLACE1009020//ESTs//3.3e-11:122:81//Hs.131777:AI024950
F-PLACE1009027//Homo sapiens mRNA for doublecortin//1.2e-151:763:96//Hs.34780:AJ003112
F-PLACE1009039//EST//0.76:111:63//Hs.160997:H55762
F-PLACE1009045//ESTs//2.2e-76:399:95//Hs.114919:AA457689
F-PLACE1009048//GLYCOPROTEIN HORMONES ALPHA CHAIN PRECURSOR//2.6e-16:93:100//Hs.119689:S70585
F-PLACE1009050//ESTs//1.4e-92:451:98//Hs.66373:AI239698
F-PLACE1009060//ESTs//1.4e-14:86:100//Hs.131725:AI090525
F-PLACE1009090//ESTs//2.7e-20:198:78//Hs.110044:AA181800
F-PLACE1009091//ESTs//0.99:342:57//Hs.46903:AI093091
F-PLACE1009094//ESTs//1.0:225:63//Hs.120374:AI337031
F-PLACE1009099//H.sapiens ZNF81 gene//2.2e-79:733:74//Hs.104020:X68011
F-PLACE1009110//ESTs//2.6e-91:453:96//Hs.143756:AI040890
F-PLACE1009111//ESTs//2.7e-15:159:77//Hs.146811:AA410788
F-PLACE1009113//Homo sapiens X-ray repair cross-complementing protein 3 (XRCC3) mRNA, complete cds//1.1e-139:671:97//Hs.99742:AF035586
F-PLACE1009130//Human mRNA for KIAA0032 gene, complete cds//1.1e-24:718:59//Hs.35804:D25215
F-PLACE1009150//Human HsLIM15 mRNA for HsLiml5, complete cds//1.7e-50:440:78//Hs.37181:D64108
F-PLACE1009155//Homo sapiens mRNA, chromosome 1 specific transcript KIAA0484//4.0e-46:440:69//Hs.158095:AB007953
F-PLACE1009158//Human growth/differentiation factor 1 (GDF-1) mRNA, complete cds//0.28:245:61//Hs.92614:M62302
F-PLACE1009166//EST//0.98:114:67//Hs.137706:AA977250
F-PLACE1009172//EST//6.2e-34:257:84//Hs.161081:N22770
F-PLACE1009174//ESTs//6.0e-24:234:77//Hs.155196:AI282821
F-PLACE1009183//EST//0.021:261:62//Hs.144222:N90100
F-PLACE1009186//ESTs, Weakly similar to No definition line found [C.elegans]//3.6e-117:588:95//Hs.54943:Z78396
F-PLACE1009190//EST//0.046:95:70//Hs.131646:AI025689
F-PLACE1009200//EST//2.5e-41:195:78//Hs.162404:AA573131
F-PLACE1009230//CARCINOEMBRYONIC ANTIGEN PRECURSOR//5.3e-29:157:77//Hs.146403:M29540
F-PLACE1009246//EST//0.13:178:62//Hs.23298:R22575
F-PLACE1009298//ESTs, Highly similar to VACUOLAR SORTING PROTEIN 35 [Saccharomyces cerevisiae]//1.9e-21:121:98//Hs.124768:AA307735
F-PLACE1009308//SERUM PROTEIN MSE55//0.44:195:62//Hs.148101:M88338
F-PLACE1009319//Homo sapiens post-synaptic density protein 95 (PSD95) mRNA, complete cds//9.7e-08:411:59//Hs.23731:U83192
F-PLACE1009328//Human Line-1 repeat mRNA with 2 open reading frames//2.3e-91:594:86//Hs.23094:M19503
F-PLACE1009335//EST//0.037:169:63//Hs.148875:AI240767
F-PLACE1009338//ESTs//5.7e-22:123:98//Hs.66783:AA059473
F-PLACE1009368
F-PLACE1009375
F-PLACE1009388//Homo sapiens KIAA0395 mRNA, partial cds//1.7e-41:317:81//Hs.43681:AL022394
F-PLACE1009398//Zinc finger protein 84 (HPF2)//1.4e-79:730:74//Hs.9450:M27878
F-PLACE1009404//MICROTUBULE-ASSOCIATED PROTEIN TAU//0.099:207:61//Hs.101174:AF047863
F-PLACE1009410//Homo sapiens BAF57 (BAF57) gene, complete cds//1.4e-27:210:86//Hs.3404:AF035262
F-PLACE1009434//Human mRNA for KIAA0005 gene, complete cds//2.8e-45:599:68//Hs.155291:D13630
F-PLACE1009443//H.sapiens 5T4 gene for 5T4 Oncofetal antigen//0.11:350:58//Hs.82128:AJ012159
F-PLACE1009444//PHOSPHATIDYLINOSITOL 4-KINASE ALPHA//1.5e-22:146:93//Hs.76987:AF012872
F-PLACE1009459//H.sapiens garp gene mRNA, complete CDS//1.0:241:60//Hs.151641:Z24680
F-PLACE1009468//1-PHOSPHATIDYLINOSITOL-4,5-BISPHOSPHATE PHOSPHODIESTERASE BETA 2//0.00039:347:60//Hs.994:M95678
F-PLACE1009476//Homo sapiens Chromosome 16 BAC clone CIT987SK-A-67Al//4.1e-91:464:96//Hs.155049:AC004531
F-PLACE1009477//ESTs//0.30:221:61//Hs.107287:AI308839
F-PLACE1009493//Homo sapiens mRNA for LAK-4p, complete cds//1.6e-30:608:63//Hs.16165:AB002405
F-PLACE1009524//Human Sec7p-like protein mRNA, partial cds//2.3e-68:526:78//Hs.8517:U70728
F-PLACE1009539//ESTs//3.3e-18:186:83//Hs.71922:AA148417
F-PLACE1009542//EST//7.8e-11:265:65//Hs.159692:AI416956
F-PLACE1009571//ESTs//6.1e-15:94:97//Hs.151458:AA600866
F-PLACE1009581//Microtubule-associated protein 1A//1.0:196:59//Hs.147918:U38291
F-PLACE1009595//EST//1.8e-28:179:92//Hs.60090:AA004806
F-PLACE1009596//ESTs, Weakly similar to LIS-1 protein [H.sapiens]//4.1e-16:281:66//Hs.13889:AI341394
F-PLACE1009607//Homo sapiens PYRIN (MEFV) mRNA, complete cds//4.9e-52:313:79//Hs.113283:AF018080
F-PLACE1009613//ESTs//0.50:297:60//Hs.25114:AI074011
F-PLACE1009621//ESTs//1.4e-98:470:98//Hs.124695:AI094085
F-PLACE1009622//ESTs//9.8e-14:94:93//Hs.117227:AA682773
F-PLACE1009637//ESTs//4.9e-92:440:98//Hs.126587:AA917087
F-PLACE1009639
F-PLACE1009659//Homo sapiens mRNA for KIAA0587 protein, complete cds//4.4e-173:816:98//Hs.21862:AB011159
F-PLACE1009665//ESTs//9.1e-45:383:79//Hs.61199:AA024494
F-PLACE1009670//Homo sapiens genethonin 1 mRNA, complete cds//8.1e-149:701:98//Hs.109590:AF062534
F-PLACE1009708//ESTs, Weakly similar to HYPOTHETICAL TRP-ASP REPEATS CONTAINING PROTEIN IN HXT14-PHA2 INTERGENIC REGION [S.cerevisiae]//7.5e-51:295:92//Hs.48541:AA827926
F-PLACE1009721//EST//0.18:467:58//Hs.124358:AA830650
F-PLACE1009731//ESTs//1.0:207:63//Hs.60440:AA195789
F-PLACE1009763//Homo sapiens UBA3 (UBA3) mRNA, complete cds//1.3e-126:602:98//Hs.154320:AF046024
F-PLACE1009794//ESTs//4.0e-41:252:91//Hs.42927:N20989
F-PLACE1009798//Human DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs and GSSs//5.5e-130:600:95//Hs.16411:AL030996
F-PLACE1009845
F-PLACE1009861
F-PLACE1009879//ESTs//6.3e-12:293:66//Hs.147071:AI200021
F-PLACE1009886
F-PLACE1009888//EST//0.044:255:58//Hs.160695:AI282889
F-PLACE1009908
F-PLACE1009921//Apoptosis (APO-1) antigen 1//0.62:407:57//Hs.82359:X63717
F-PLACE1009924//EST//2.9e-29:155:99//Hs.162937:AA634379
F-PLACE1009925
F-PLACE1009935//CATHEPSIN K PRECURSOR//0.43:153:66//Hs.83942:X82153
F-PLACE1009947//ESTs//1.8e-07:56:100//Hs.149940:AI306446
F-PLACE1009971//Acyl-Coenzyme A dehydrogenase, C-2 to C-3 short chain//0.89:243:61//Hs.127610:Z80345
F-PLACE1009992//ESTs//0.99:123:68//Hs.91202:AI139114
F-PLACE1009995//ESTs, Weakly similar to C01A2.4 [C.elegans]//3.3e-24:174:88//Hs.11449:AI201540
F-PLACE1009997//Homo sapiens mRNA for KIAA0629 protein, partial cds//3.7e-36:196:96//Hs.153545:AB014529
F-PLACE1010023
F-PLACE1010031//ESTs//1.3e-16:132:87//Hs.46847:W02878
F-PLACE1010053//ESTs, Moderately similar to M-phase phosphoprotein 4 [H.sapiens]//5.2e-63:312:98//Hs.142151:AA984061
F-PLACE1010069//ESTs//6.6e-33:171:98//Hs.128844:AA977596
F-PLACE1010074//Homo sapiens sorting nexin 2 (SNX2) mRNA, complete cds//5.9e-168:792:98//Hs.11183:AF065482
F-PLACE1010076//ESTs//0.88:379:55//Hs.5884:N21424
F-PLACE1010083//Homo sapiens mRNA for KIAA0456 protein, partial cds//9.6e-154:727:98//Hs.5003:AB007925
F-PLACE1010089//ESTs, Highly similar to PROBABLE UBIQUITIN CARBOXYL-TERMINAL HYDROLASE [Mus musculus]//1.8e-38:212:95//Hs.98067:AA236822
F-PLACE1010096//ESTs, Highly similar to hypothetical protein, 100K [R.norvegicus]//1.8e-08:100:89//Hs.11469:U69567
F-PLACE1010102//Homo sapiens stimulator of Fe transport mRNA, complete cds//0.0035:339:60//Hs.129683:AF020761
F-PLACE1010105//Homo sapiens actin binding protein MAYVEN mRNA, complete cds//1.2e-26:728:60//Hs.122967:AF059569
F-PLACE1010106//EST//8.5e-28:394:70//Hs.142044:AA166682
F-PLACE1010134//H.sapiens hbrm mRNA//1.2e-14:380:64//Hs.77590:X72889
F-PLACE1010148//Human trans-Golgi p230 mRNA, complete cds//0.26:708:57//Hs.158245:U41740
F-PLACE1010152
F-PLACE1010181//EST//1.3e-21:312:71//Hs.141501:N50792
F-PLACE1010194//ESTs//2.6e-55:284:97//Hs.155940:AA459582
F-PLACE1010202//ESTs, Weakly similar to No definition line found [C.elegans]//2.3e-72:391:94//Hs.35225:H69637
F-PLACE1010231
F-PLACE1010261//Homo sapiens mRNA for KIAA0448 protein, complete cds//1.9e-146:693:97//Hs.27349:AB007917
F-PLACE1010270//ESTs//2.0e-104:514:98//Hs.124062:H04590
F-PLACE1010274//ESTs, Weakly similar to C01A2.4 [C.elegans]//6.8e-25:149:93//Hs.11449:AI201540
F-PLACE1010293//EST//4.5e-36:358:74//Hs.162398:AA572813
F-PLACE1010310//HOMEOBOX/POU DOMAIN PROTEIN RDC-1//2.1e-10:352:62//Hs.74095:L20433
F-PLACE1010321//Human hSIAH2 mRNA, complete cds//0.071:604:58//Hs.20191:U76248
F-PLACE1010324//ESTs//0.22:286:58//Hs.130853:AI367875
F-PLACE1010329//EST//5.7e-05:351:60//Hs.120644:AA742659
F-PLACE1010341//EST//4.5e-16:255:72//Hs.141206:H53117
F-PLACE1010362//ESTs//1.9e-41:246:92//Hs.128771:AA236855
F-PLACE1010364//EST//0.11:292:58//Hs.135771:AI005648
F-PLACE10l0383//EST//6.1e-08:107:76//Hs.136441:AA564986
F-PLACE1010401
F-PLACE1010481//Human BLu protein (BLu) mRNA, complete cds//0.94:254:61//Hs.125257:U70824
F-PLACE1010491//Homo sapiens Cre binding protein-like 2 mRNA, complete cds//7.2e-152:702:99//Hs.13313:AF039081
F-PLACE1010492//ESTs//1.0:201:60//Hs.146036:AI038500
F-PLACE1010522//ESTs//3.9e-52:263:97//Hs.125149:AI302100
F-PLACE1010529//Homo sapiens chromodomain-helicase-DNA-binding protein mRNA, complete cds//1.0:175:64//Hs.159273:AF054177
F-PLACE1010547//ESTs//0.96:288:57//Hs.87156:AA233472
F-PLACE1010562//EST//1.0:164:66//Hs.147868:AI222979
F-PLACE1010579//EST//0.39:279:58//Hs.158960:AI380148
F-PLACE1010580//ESTs, Moderately similar to PUTATIVE ATP-DEPENDENT RNA HELICASE C12C2.06 [Schizosaccharomyces pombe]//3.8e-31:193:91//Hs.145229:N44661
F-PLACE1010599//Homo sapiens peroxisomal membrane anchor protein HsPex14p (PEX14) mRNA, complete cds//9.9e-148:707:97//Hs.19851:AF045186
F-PLACE1010616//EST//3.1e-43:213:100//Hs.128215:AA972394
F-PLACE1010622//NUCLEOLIN//0.00040:282:60//Hs.79110:M60858
F-PLACE1010624//Homo sapiens Jagged 2 mRNA, complete cds//1.2e-05:516:61//Hs.106387:AF029778
F-PLACE1010628//EST, Weakly similar to line-1 protein ORF2 [H.sapiens]//0.012:258:62//Hs.144375:AA484200
F-PLACE1010629//EST//8.3e-23:218:79//Hs.161975:AA501461
F-PLACE1010630//EST//0.29:319:58//Hs.137277:N62225
F-PLACE1010631//Homo sapiens mRNA for KIAA0530 protein, partial cds//9.5e-66:363:95//Hs.10801:AB011102
F-PLACE1010661//ESTs//3.9e-89:504:92//Hs.122666:W27076
F-PLACE1010662
F-PLACE1010702//Human repressor transcriptional factor (ZNF85) mRNA, complete cds//1.1e-74:697:74//Hs.37138:U35376
F-PLACE1010714//EST//0.018:253:59//Hs.148028:AI270027
F-PLACE1010720//Homo sapiens chromosome-associated protein-C (hCAP-C) mRNA, partial cds//6.1e-77:393:96//Hs.50758:AF092564
F-PLACE1010739//Homo sapiens mRNA for Sec24 protein (Sec24A isoform), partial//0.97:314:59//Hs.14574:AJ131244
F-PLACE1010743//Human myosin-IXb mRNA, complete cds//2.4e-56:409:86//Hs.159629:U42391
F-PLACE1010761//ESTs, Weakly similar to U1 SMALL NUCLEAR RIBONUCLEOPROTEIN 70 KD [Xenopus laevis]//5.1e-80:407:96//Hs.80965:AA493284
F-PLACE1010771//ESTs, Highly similar to TRANSCRIPTIONAL REGULATOR PROTEIN HCNGP [Mus musculus]//6.0e-45:251:94//Hs.11379:AA594140
F-PLACE1010786
F-PLACE1010800
F-PLACE1010802//EST//0.94:128:64//Hs.120366:AA719157
F-PLACE1010811//ESTs//0.89:339:59//Hs.127314:N48085
F-PLACE1010833//ESTs, Weakly similar to allograft inflammatory factor-1 [H.sapiens]//2.9e-28:245:79//Hs.132736:AA583494
F-PLACE1010856//ESTs//1.5e-06:95:87//Hs.17401:W81048
F-PLACE1010857//ESTs, Weakly similar to KIAA0157 gene product is novel. [H.sapiens]//5.8e-67:336:97//Hs.130135:AA905493
F-PLACE1010870//Zinc finger protein 43 (HTF6)//9.7e-40:498:69//Hs.74107:X59244
F-PLACE1010877//Homo sapiens mRNA for KIAA0610 protein, partial cds//3.7e-149:694:98//Hs.118087:AB011182
F-PLACE1010891//ESTs//6.9e-54:377:87//Hs.24453:R31671
F-PLACE1010896//Human homologue of yeast sec7 mRNA, complete cds//0.64:167:65//Hs.1050:M85169
F-PLACE1010900
F-PLACE1010916//EST//0.55:151:66//Hs.145800:AI269981
F-PLACE1010917
F-PLACE1010925//ESTs//2.6e-81:437:94//Hs.5876:H26537
F-PLACE1010926//Homo sapiens mRNA for KIAA0554 protein, partial cds//3.1e-139:653:98//Hs.74750:AB011126
F-PLACE1010942//Homo sapiens intersectin short form mRNA, complete cds//2.9e-91:437:98//Hs.66392:AF064244
F-PLACE1010944//ESTs//1.3e-17:117:91//Hs.29444:W30985
F-PLACE1010947//EST//0.97:93:72//Hs.162299:AA555154
F-PLACE1010954//Apolipoprotein B (including Ag(x) antigen)//0.28:444:59//Hs.585:X04506
F-PLACE1010960//ESTs//0.98:238:60//Hs.163674:AA506632
F-PLACE1010965//ESTs//3.1e-74:376:96//Hs.115679:AI379721
F-PLACE1011026//EST//0.022:222:60//Hs.47154:N50931
F-PLACE1011032//EST//1.1e-05:88:79//Hs.118024:N34032
F-PLACE1011041//Human density enhanced phosphatase-1 mRNA, complete cds//0.28:179:67//Hs.1177:U10886
F-PLACE1011046//1-PHOSPHATIDYLINOSITOL-4,5-BISPHOSPHATE PHOSPHODIESTERASE BETA 2//6.2e-11:207:68//Hs.994:M95678
F-PLACE1011054//H.sapiens OBF-1 mRNA for octamer binding factor 1//6.1e-35:310:78//Hs.2407:Z49194
F-PLACE1011056//Human putative serine/threonine protein kinase PRK (prk) mRNA, complete cds//0.74:228:61//Hs.153640:U56998
F-PLACE1011057//EST//2.5e-80:388:98//Hs.126466:AA913320
F-PLACE1011090//ESTs//1.4e-94:469:97//Hs.106448:R76663
F-PLACE1011109//ESTs//0.13:303:62//Hs.49294:AA418037
F-PLACE1011114//ESTs//5.8e-12:75:100//Hs.147422:AI214317
F-PLACE1011133//ESTs//0.17:225:62//Hs.132853:AI370857
F-PLACE1011143//ESTs//0.013:264:63//Hs.115368:AA629949
F-PLACE1011160
F-PLACE1011165//Galactokinase 2//2.7e-32:194:92//Hs.129228:M84443
F-PLACEL011185//EST//1.4e-34:261:83//Hs.140250:AA708114
F-PLACE1011203//Homo sapiens chromosome 18q11 beta-1,4-galactosyltransferase mRNA, complete cds//6.9e-124:576:99//Hs.159140:AF038664
F-PLACE1011214//ESTs, Weakly similar to B0035.14 [C.elegans]//9.7e-101:469:99//Hs.8241:AA283057
F-PLACE1011219//ESTs, Weakly similar to coded for by C. elegans cDNA CEESL70F [C.elegans]//2.6e-62:221:88//Hs.101821:W27452
F-PLACE1011221//ESTs//0.46:238:62//Hs.32853:AA015751
F-PLACE1011229//Homo sapiens mRNA for KIAA0529 protein, partial cds//1.4e-147:675:99//Hs.23168:AB011101
F-PLACE1011263//Homo sapiens BAC clone GS166A23 from 7p21//5.9e-71:350:98//Hs.15144:AC005014
F-PLACE1011273//ESTs//1.0:222:59//Hs.35274:AA495803
F-PLACE1011291//Homo sapiens clone 24712 unknown mRNA, partial cds//3.4e-09:191:65//Hs.140950:AF070637
F-PLACE1011296//ESTs//0.019:137:63//Hs.140654:AA865915
F-PLACE1011310//EST//0.066:336:58//Hs.162529:AA584160
F-PLACE1011325//ESTs//7.4e-43:229:96//Hs.21081:H08310
F-PLACE1011332//Homo sapiens N-acetylglucosamine-phosphate mutase mRNA, complete cds//4.8e-151:696:99//Hs.5819:AF102265
F-PLACE1011340//Homo sapiens mRNA, chromosome 1 specific transcript KIAA0501//1.5e-20:120:81//Hs.159897:AB007970
F-PLACE1011371//Human mRNA for PK-120//9.5e-35:684:63//Hs.76415:D38535
F-PLACE1011375//ESTs, Moderately similar to potassium channel protein Raw3 [R.norvegicus]//6.7e-68:325:99//Hs.107245:AA627053
F-PLACE1011399//ESTs//8.6e-05:285:61//Hs.130105:AA904868
F-PLACE1011419//ESTs//0.70:240:62//Hs.159650:N95552
F-PLACE1011433//Homo sapiens mRNA for KIAA0530 protein, partial cds//1.5e-158:743:98//Hs.10801:AB011102
F-PLACE1011452//Human Line-1 repeat mRNA with 2 open reading frames//1.9e-53:557:72//Hs.23094:M19503
F-PLACE1011465//EST//3.1e-58:380:85//Hs.131605:AI025204
F-PLACE1011472//Homo sapiens mRNA for KIAA0712 protein, complete cds//1.5e-152:703:99//Hs.111138:AB018255
F-PLACE1011477//Homo sapiens sorting nexin 2 (SNX2) mRNA, complete cds//1.7e-146:675:99//Hs.11183:AF065482
F-PLACE1011492//ESTs//2.0e-35:186:98//Hs.125886:AA884264
F-PLACE1011503//EST//0.67:149:65//Hs.149774:AI285997
F-PLACE1011520//ESTs//0.00014:213:64//Hs.119889:AA705319
F-PLACE1011563//ESTs//2.2e-61:394:86//Hs.117718:AA883476
F-PLACE1011567//Homo sapiens DEC-205 mRNA, complete cds//3.1e-46:325:84//Hs.153563:AF011333
F-PLACE1011576//Homo sapiens hematopoietic cell derived zinc finger protein mRNA, complete cds//4.3e-67:268:86//Hs.86371:AF054180
F-PLACE1011586//Homo sapiens hLRpl05 mRNA for LDL receptor related protein 105, complete cds//0.98:153:65//Hs.143641:AB009462
F-PLACE1011635//Homo sapiens Jagged 2 mRNA, complete cds//0.00029:585:57//Hs.106387:AF029778
F-PLACE1011641
F-PLACE1011643//Homo sapiens mRNA for KIAA0293 gene, partial cds//0.00058:499:58//Hs.12784:AB006631
F-PLACE1011646//EST//3.2e-26:201:68//Hs.140349:AA757661
F-PLACE1011649//ESTs//0.25:145:64//Hs.23033:R46086
F-PLACE1011650//ESTs//0.041:96:77//Hs.119351:AA447745
F-PLACE1011664//Human mRNA for stac, complete cds//1.0:245:60//Hs.56045:D86640
F-PLACE1011675//Cell division cycle 27//0.098:448:57//Hs.73151:S78234
F-PLACE1011682//EST//9.6e-06:119:72//Hs.93664:N23366
F-PLACE1011719//Human mRNA for KIAA0352 gene, complete cds//0.92:365:60//Hs.17262:AB002350
F-PLACE1011725
F-PLACE1011729//EST//0.56:304:58//Hs.86378:AA210853
F-PLACE1011749//ESTs//4.3e-88:443:96//Hs.132850:AA779891
F-PLACE1011762//ESTs//0.012:149:68//Hs.145075:AI208240
F-PLACE1011778//ESTs//0.00016:199:64//Hs.160395:AI393693
F-PLACE1011783//EST//1.0:119:66//Hs.162191:AA534660
F-PLACE1011858//Human novel homeobox mRNA for a DNA binding protein//8.9e-05:477:59//Hs.37035:U07664
F-PLACE1011874//EST//0.20:118:66//Hs.127351:AA954775
F-PLACE1011875//Homo sapiens mRNA for KIAA0580 protein, partial cds//5.3e-110:526:98//Hs.22572:AB011152
F-PLACE1011891//ESTs//1.8e-58:397:88//Hs.84698:AA725913
F-PLACE1011896//ESTs, Weakly similar to Y53C12A.3 [C.elegans]//9.4e-09:478:56//Hs.107747:AI357868
F-PLACE1011922//ESTs//0.49:249:62//Hs.152627:AA595817
F-PLACE1011923//Homo sapiens serum-inducible kinase mRNA, complete cds//3.7e-140:664:98//Hs.3838:AF059617
F-PLACE1011962//EST//1.7e-07:81:85//Hs.104333:AA250763
F-PLACE1011964//EST//6.6e-38:412:74//Hs.140562:AA826514
F-PLACE1011982//ESTs//0.40:405:60//Hs.127743:AI261591
F-PLACE1011995//ESTs//1.7e-22:486:64//Hs.105157:AA527514
F-PLACE1012031//Homo sapiens mRNA for KIAA0713 protein, partial cds//4.0e-148:690:98//Hs.88756:AB018256
F-PLACE2000003//Homo sapiens mRNA, chromosome 1 specific transcript KIAA0487//6.5e-54:290:81//Hs.92381:AB007956
F-PLACE2000006//ESTs//0.067:224:62//Hs.144100:AI205503
F-PLACE2000007//ESTs//8.1e-23:147:91//Hs.128530:AA325330
F-PLACE2000011//Interleukin 10//4.2e-42:362:78//Hs.2180:M57627
F-PLACE2000014//EST//0.10:214:61//Hs.160247:AI138831
F-PLACE2000015//Interleukin 10//1.4e-44:393:78//Hs.2180:M57627
F-PLACE2000017
F-PLACE2000021//Homo sapiens TRF1-interacting ankyrin-related ADP-ribose polymerase mRNA, partial cds//5.7e-85:844:72//Hs.7928:AF082557
F-PLACE2000030
F-PLACE2000033//Human adhesion molecule ninjurin mRNA, complete cds//0.85:234:66//Hs.11342:U91512
F-PLACE2000034//Homo sapiens mRNA for KIAA0607 protein, partial cds//0.058:348:62//Hs.94653:AB011179
F-PLACE2000039//Human plectin (PLEC1) mRNA, complete cds//0.0058:473:59//Hs.79706:U53204
F-PLACE2000047//ESTs//4.9e-32:328:75//Hs.141024:H07128
F-PLACE2000050//ESTs//3.0e-36:270:83//Hs.155512:AA663966
F-PLACE2000061
F-PLACE2000062//Human membrane-associated lectin type-C mRNA//2.9e-114:662:86//Hs.23759:M98457
F-PLACE2000072//Homo sapiens ZNF202 alpha (ZNF202) mRNA, complete cds//7.1e-135:631:98//Hs.9443:AF027219
F-PLACE2000097//ESTs//0.021:117:70//Hs.132811:AI034333
F-PLACE2000100
F-PLACE2000103//ESTs//1.1e-56:284:98//Hs.144786:AI219219
F-PLACE2000111//H.sapiens mRNA for l-acylglycerol-3-phosphate O-acyltransferase//0.76:215:65//Hs.6587:U56417
F-PLACE2000115
F-PLACE2000124//Human mRNA for KIAA0355 gene, complete cds//2.8e-49:400:79//Hs.153014:AB002353
F-PLACE2000132
F-PLACE2000136//ESTS, Moderately similar to hypothetical protein [H.sapiens]//1.2e-08:245:64//Hs.140343:AA718911
F-PLACE2000140//Adenylate kinase 2 (adk2)//3.7e-24:162:90//Hs.83833:U54645
F-PLACE2000164
F-PLACE2000170
F-PLACE2000172//ESTs//0.64:239:62//Hs.31175:AI219179
F-PLACE2000176
F-PLACE2000187
F-PLACE2000216
F-PLACE2000223//EST//0.0092:171:60//Hs.162830:AA643933
F-PLACE2000235//Human mRNA for KIAA0298 gene, complete cds//1.6e-38:792:63//Hs.21560:AB002296
F-PLACE2000246//Homo sapiens mRNA for KIAA0795 protein, partial cds//1.5e-74:367:98//Hs.22926:AB018338
F-PLACE2000264//Homo sapiens mRNA for KIAA0792 protein, complete cds//2.0e-29:366:73//Hs.119387:AB007958
F-PLACE2000274//Homo sapiens mRNA for dynein heavy chain//1.0e-23:650:62//Hs.144672:AJ000522
F-PLACE2000302//ESTs//1.7e-05:66:89//Hs.55572:W37560
F-PLACE2000305//ESTs//1.6e-78:382:98//Hs.136731:AA745869
F-PLACE2000317
F-PLACE2000335//Fc fragment of IgE, high affinity I, receptor for; beta polypeptide//6.1e-24:295:76//Hs.30:M89796
F-PLACE2000341//Human sodium iodide symporter mRNA, complete cds//6.8e-21:593:61//Hs.103983:U66088
F-PLACE2000342//Centromere protein B (80kD)//1.4e-06:326:61//Hs.85004:X05299
F-PLACE2000347//ESTs, Moderately similar to F18547_1 [H.sapiens]//3.7e-16:139:82//Hs.28209:AI073817
F-PLACE2000359//ESTs//5.0e-19:251:71//Hs.58272:W76645
F-PLACE2000366//ESTs//1.7e-37:399:75//Hs.136646:AA748045
F-PLACE2000371//EST//0.65:107:65//Hs.157677:AI358861
F-PLACE2000373//ESTs//0.30:207:59//Hs.143902:AI131032
F-PLACE2000379//ESTs//1.3e-64:402:87//Hs.146307:AA584638
F-PLACE2000394//Homo sapiens mRNA, chromosome 1 specific transcript KIAA0484//1.0e-87:694:80//Hs.158095:AB007953
F-PLACE2000398
F-PLACE2000399
F-PLACE2000404
F-PLACE2000411
F-PLACE2000419//Homo sapiens PYRIN (MEFV) mRNA, complete cds//8.0e-52:463:74//Hs.113283:AF018080
F-PLACE2000425//EST//0.44:168:62//Hs.44677:N34966
F-PLACE2000427
F-PLACE2000433//ESTs//4.7e-18:213:74//Hs.110187:AA699719
F-PLACE2000435//EST//4.7e-05:159:64//Hs.123604:AA815257
F-PLACE2000438//H.sapiens mRNA for UDP-GalNAc:polypeptide N-acetylgalactosaminyltransferase (T2)//1.9e-20:418:64//Hs.130181:X85019
F-PLACE2000450//Homo sapiens PYRIN (MEFV) mRNA, complete cds//4.0e-83:324:81//Hs.113283:AF018080
F-PLACE2000455//ESTs, Moderately similar to !!!! ALU SUBFAMILY SC WARNING ENTRY !!!! [H.sapiens]//4.0e-05:100:73//Hs.104239:AA488082
F-PLACE2000458//H.sapiens mRNA for hFat protein//0.0010:545:57//Hs.91107:X87241
F-PLACE2000465//ESTs//4.4e-38:377:75//Hs.55855:AA621381
F-PLACE2000477//Homo sapiens PYRIN (MEFV) mRNA, complete cds//1.8e-68:520:81//Hs.113283:AF018080
F-PLACE3000004//Human EYA3 homolog (EYA3) mRNA, complete cds//3.9e-14:204:73//Hs.46925:Y10262 ,
F-PLACE3000009//Human mRNA for KIAA0386 gene, complete cds//4.8e-59:696:69//Hs.101359:AB002384
F-PLACE3000020//Prostaglandin 12 (prostacyclin) receptor (IP)//0.00081:500:61//Hs.393:D38128
F-PLACE3000029
F-PLACE3000059//ESTs//0.0026:49:100//Hs.42913:AI082248
F-PLACE3000070//ESTs//5.6e-15:202:74//Hs.154993:AA142842
F-PLACE3000103//Homo sapiens cofactor of initiator function (CIF150) mRNA, complete cds//1.0:186:62//Hs.122752:AF026445
F-PLACE3000119//Homo sapiens mRNA for KIAA0752 protein, partial cds//2.8e-48:283:83//Hs.23711:AB018295
F-PLACE3000121
F-PLACE3000124//Thromboxane A2 receptor//1.1e-55:195:83//Hs.89887:D38081
F-PLACE3000136//Homo sapiens mRNA for KIAA0703 protein, complete cds//1.0:194:59//Hs.6168:AB014603
F-PLACE3000142//EST//0.41:179:59//Hs.137438:AA282243
F-PLACE3000145//ESTs//3.5e-25:145:96//Hs.163950:AA683016
F-PLACE3000147//EST//5.0e-43:285:86//Hs.160895:AI365871
F-PLACE3000148
F-PLACE3000155//Homo sapiens mRNA for KIAA0672 protein, complete cds//5.6e-80:382:99//Hs.6336:AB014572
F-PLACE3000156//ESTs//0.00015:277:62//Hs.156834:AI336023
F-PLACE3000157//Calcium channel, voltage-dependent, P/Q type, alpha 1A subunit//0.54:320:60//Hs.96253:U79666
F-PLACE3000158//Homo sapiens mRNA for KIAA0575 protein, complete cds//4.9e-66:319:88//Hs.153468:AB011147
F-PLACE3000160
F-PLACE3000169//Small inducible cytokine A5 (RANTES)//1.3e-64:501:80//Hs.155464:AF088219
F-PLACE3000194
F-PLACE3000197
F-PLACE3000199//EST//1.0:108:68//Hs.98488:AA426546
F-PLACE3000207//EST//1.0e-32:184:75//Hs.160146:AI049975
F-PLACE3000208//CLASS II HISTOCOMPATIBILITY ANTIGEN, M ALPHA CHAIN PRECURSOR//1.0:271:61//Hs.77522:X62744
F-PLACE3000218//EST//1.3e-46:317:84//Hs.162197:AA535216
F-PLACE3000220//EST//9.3e-95:443:99//Hs.112702:AA609377
F-PLACE3000221//Homo sapiens DNA fragmentation factor 40 kDa subunit (DFF40) mRNA, complete cds//9.2e-56:200:85//Hs.133089:AF064019
F-PLACE3000226
F-PLACE3000230//EST//6.1e-16:173:72//Hs.148578:AI201568
F-PLACE3000242//Human DNA sequence from clone 1409 on chromosome Xp11.1-11.4. Contains a Inter-Alpha-Trypsin Inhibitor Heavy Chain LIKE gene, a alternatively spliced Melanoma-Associated Antigen MAGE LIKE gene and a 6-Phosphofructo-2-kinase (Fructose-2,6-bisphosphatase) LIKE pseudogene. Contains ESTs, STSs and genomic marker DXS8032//1.2e-54:434:80//Hs.4943:Z98046
F-PLACE3000244
F-PLACE3000254//NUCLEOLIN//2.6e-05:445:60//Hs.79110:M60858
F-PLACE3000271//ESTs//1.6e-25:195:72//Hs.108452:H78650
F-PLACE3000276//ESTs//1.0e-13:274:66//Hs.28589:AI004944
F-PLACE3000304//EST//0.043:210:61//Hs.132378:AI026770
F-PLACE3000310
F-PLACE3000320//EST//1.2e-12:188:70//Hs.145771:AI269586
F-PLACE3000322//Small inducible cytokine A5 (RANTES)//4.7e-29:252:80//Hs.155464:AF088219
F-PLACE3000331
F-PLACE3000339//Homo sapiens mRNA for KIAA0645 protein, complete cds//0.91:222:61//Hs.155987:AB014545
F-PLACE3000341//EST//1.8e-05:394:58//Hs.112894:AA620741
F-PLACE3000350//ESTs, Highly similar to SERINE/THREONINE-PROTEIN KINASE SULU [Caenorhabditis elegans]//2.9e-59:474:77//Hs.125850:AA885355
F-PLACE3000352//H.sapiens OBF-1 mRNA for octamer binding factor 1//2.5e-48:442:78//Hs.2407:Z49194
F-PLACE3000353//H.sapiens mRNA for UDP-GalNAc:polypeptide N-acetylgalactosaminyltransferase (T1)//0.78:234:63//Hs.7498:U41514
F-PLACE3000362//EST//6.5e-25:302:73//Hs.140504:AA810441
F-PLACE3000363
F-PLACE3000365//ESTs//0.81:200:60//Hs.141556:N49928
F-PLACE3000373//ESTs//0.0071:82:73//Hs.136310:AA442641
F-PLACE3000388//ESTs//7.9e-16:235:71//Hs.44701:AA830432
F-PLACE3000399//Clathrin, light polypeptide (Lcb)//5.2e-70:391:81//Hs.73919:X81637
F-PLACE3000400//ESTs//0.53:162:66//Hs.49303:AA810785
F-PLACE3000401//EST//2.3e-35:178:100//Hs.162851:AA632270
F-PLACE3000402//ESTs//2.4e-84:425:96//Hs.148962:AI219715
F-PLACE3000405//EST//2.1e-39:452:73//Hs.140414:AA778541
F-PLACE3000406//Homo sapiens apoptotic protease activating factor 1 (Apaf-1) mRNA, complete cds//1.9e-07:116:78//Hs.77579:AF013263
F-PLACE3000413//ESTs, Weakly similar to methyl sterol oxidase [H.sapiens]//1.6e-51:260:98//Hs.122512:H61502
F-PLACE3000416//Homo sapiens mRNA for KIAA0801 protein, complete cds//0.00020:630:57//Hs.17585:AB018344
F-PLACE3000425//EST//3.8e-34:286:79//Hs.135301:AI039161
F-PLACE3000455//Homo sapiens mRNA for cytochrome b small subunit of complex II, complete cds//3.6e-32:183:93//Hs.108326:AB006202
F-PLACE3000475//ESTs//1.9e-09:422:61//Hs.145783:AA081874
F-PLACE3000477//H.sapiens mRNA for chemokine receptor D6//1.0:426:54//Hs.117572:U94888
F-PLACE4000009//TRICHOHYALIN//3.1e-09:692:60//Hs.82276:L09190
F-PLACE4000014//Homo sapiens mRNA for KIAA0809 protein, partial cds//3.6e-118:331:100//Hs.105399:AB018352
F-PLACE4000034//Homo sapiens Chromosome 16 BAC clone CIT987SK-A-69G12//1.1e-06:244:63//Hs.154050:AC004131
F-PLACE4000049//Homo sapiens clone 24619 mRNA sequence//4.3e-45:371:79//Hs.139088:AF070533
F-PLACE4000052//Human ATP binding cassette transporter (ABCR) mRNA, complete cds//1.4e-53:669:67//Hs.40993:AF000148
F-PLACE4000063
F-PLACE4000089//ESTs//2.2e-10:121:85//Hs.49391:W00713
F-PLACE4000093//ESTs//0.0053:273:60//Hs.136952:AA825819
F-PLACE4000100//ESTs//8.0e-21:246:73//Hs.140207:N32058
F-PLACE4000106//Homo sapiens mRNA for KIAA0462 protein, partial cds//3.8e-147:684:99//Hs.129937:AB007931
F-PLACE4000128//Homo sapiens ES/130 mRNA, complete cds//0.23:398:60//Hs.98614:AF006751
F-PLACE4000129
F-PLACE4000131//ESTs//2.4e-13:194:72//Hs.41418:H90627
F-PLACE4000147//ESTs//0.0060:324:60//Hs.85640:AA535856
F-PLACE4000156//Zinc finger protein 136 (clone pHZ-20)//2.3e-89:764:76//Hs.69740:U09367
F-PLACE4000192
F-PLACE4000211
F-PLACE4000222//EST//1.9e-15:317:66//Hs.149206:AI246594
F-PLACE4000230//Human mRNA for KIAA0331 gene, complete cds//0.0048:258:60//Hs.146395:AB002329
F-PLACE4000233//ESTs//4.4e-38:240:80//Hs.114605:AI304317
F-PLACE4000247//Homo sapiens mitochondrial outer membrane protein (TOM40) mRNA, nuclear gene encoding mitochondrial protein, complete cds//0.0095:156:69//Hs.30928:AF043250
F-PLACE4000250//ESTs//3.8e-72:377:94//Hs.124234:T89609
F-PLACE4000252//ESTs//1.0:196:64//Hs.144869:AA493886
F-PLACE4000259//Homo sapiens mRNA for KIAA0788 protein, partial cds//6.2e-27:191:87//Hs.2397:Z70200
F-PLACE4000261
F-PLACE4000269//ESTs, Weakly similar to coded for by C. elegans cDNA yk52b10.3 [C.elegans]//9.5e-41:202:100//Hs.118849:AA215645
F-PLACE4000270
F-PLACE4000300
F-PLACE4000320//FKBP-RAPAMYCIN ASSOCIATED PROTEIN//4.5e-23:135:96//Hs.155952:U88966
F-PLACE4000323//EST//6.7e-09:180:68//Hs.116769:AA630365
F-PLACE4000326//ESTs//2.1e-94:453:98//Hs.103177:W72798
F-PLACE4000344//EST//6.4e-05:135:67//Hs.146729:AI147292
F-PLACE4000367
F-PLACE4000369
F-PLACE4000379//EST//3.9e-42:381:79//Hs.162335:AA564256
F-PLACE4000387//ESTs//0.19:93:69//Hs.154173:AI379823
F-PLACE4000392//ESTs//0.0015:381:59//Hs.120172:AA709046
F-PLACE4000401//Homo sapiens mRNA for KIAA0640 protein, partial cds//3.1e-47:605:71//Hs.153026:AB014540
F-PLACE4000411//ESTs, Moderately similar to plakophilin 2b [H.sapiens]//4.7e-33:159:81//Hs.154257:AI275982
F-PLACE4000431//Homo sapiens mRNA for KIAA0788 protein, partial cds//1.3e-45:263:92//Hs.2397:Z70200
F-PLACE4000445
F-PLACE4000450
F-PLACE4000465//ESTs//1.5e-11:273:65//Hs.145783:AA081874
F-PLACE4000487//Sialophorin (gpL115, leukosialin, CD43)//3.0e-14:189:71//Hs.80738:X52075
F-PLACE4000489//ESTs//0.94:104:68//Hs.125119:R38951
F-PLACE4000494//ESTs//1.0:185:60//Hs.143053:AI126289
F-PLACE4000521//ESTs//0.0027:161:70//Hs.135740:AA651731
F-PLACE4000522//ESTs, Highly similar to NEUROGENIC LOCUS NOTCH PROTEIN HOMOLOG 1 PRECURSOR [Homo sapiens]//0.047:119:65//Hs.129053:AA767022
F-PLACE4000548
F-PLACE4000558//Homo sapiens mRNA for DFFRY protein, abundant transcript//0.0035:510:59//Hs.39163:AF000986
F-PLACE4000581
F-PLACE4000590//ESTs, Highly similar to POL POLYPROTEIN [Friend murine leukemia virus (isolate 57)]//3.4e-13:275:68//Hs.113980:AI034080
F-PLACE4000593//ESTS, Weakly similar to F25D7.1 [C.elegans]//5.2e-28:239:79//Hs.109084:AI004675
F-PLACE4000612//Keratin 9//0.27:207:64//Hs.2783:Z29074
F-PLACE4000638//Homo sapiens mRNA from chromosome 5q21-22, clone:sF2//3.5e-47:562:69//Hs.129685:AB002446
F-PLACE4000650
F-PLACE4000654
F-PLACE4000670//ESTs//6.1e-88:411:100//Hs.130688:AI028132
F-SKNMC1000011//Centromere protein B (80kD)//0.0013:243:62//Hs.85004:X05299
F-SKNMC1000013//ESTs, Highly similar to MULTIDRUG RESISTANCE PROTEIN HOMOLOG 50 [Drosophila melanogaster]//2.5e-36:197:96//Hs.118634:U66688
F-SKNMC1000046//Homo sapiens mRNA for KIAA0654 protein, partial cds//2.5e-148:706:98//Hs.109299:AB014554
F-SKNMC1000050//Calpain, large polypeptide L2//4.1e-53:330:90//Hs.76288:M23254
F-SKNMC1000091//ESTs//3.3e-64:420:88//Hs.90997:AA946877
F-THYRO1000017//Human mRNA for KIAA0315 gene, partial cds//1.0:310:60//Hs.3989:AB002313
F-THYRO1000026//H.sapiens OBF-1 mRNA for octamer binding factor 1//2.9e-35:299:81//Hs.2407:Z49194
F-THYRO1000034
F-THYRO1000035//ESTs//4.1e-37:317:79//Hs.141254:AI334099
F-THYRO1000040//ESTs//0.30:331:59//Hs.87176:AI148326
F-THYRO1000070//Human mRNA for KIAA0347 gene, complete cds//0.069:278:63//Hs.101996:AB002345
F-THYRO1000072//Homo sapiens clone 23584 mRNA sequence//8.7e-86:722:77//Hs.6654:AB014557
F-THYRO1000085
F-THYRO1000092//ESTs//3.1e-100:469:99//Hs.132207:AI148065
F-THYRO1000107
F-THYRO1000111//Human Line-1 repeat mRNA with 2 open reading frames//6.8e-106:690:86//Hs.23094:M19503
F-THYRO1000121
F-THYRO1000124//Human mRNA for alanine aminotransferase//0.0026:420:58//Hs.103502:U70732
F-THYRO1000129//Homo sapiens TED protein (TED).mRNA, complete cds//2.8e-155:732:98//Hs.87619:AF087142
F-THYRO1000132//ESTs//1.9e-35:164:79//Hs.139179:AA650203
F-THYRO1000156//EST//0.32:102:68//Hs.139634:AA478416
F-THYRO1000163//Small inducible cytokine A5 (RANTES)//5.2e-50:331:85//Hs.155464:AF088219
F-THYRO1000173//Human clathrin assembly protein 50 (AP50) mRNA, complete cds//1.1e-05:261:61//Hs.152936:D63475
F-THYRO1000186//H.sapiens mRNA for phosphoinositide 3-kinase//3.7e-41:270:87//Hs.101238:Y11312
F-THYRO1000187//EST//0.11:227:62//Hs.101773:H23270
F-THYRO1000190//ESTs//0.82:194:63//Hs.128818:AA976883
F-THYRO1000197//Homo sapiens mRNA for poly(A)-specific ribonuclease//2.4e-175:805:99//Hs.43445:AJ005698
F-THYRO1000199//Homo sapiens mRNA for KIAA0652 protein, complete cds//4.0e-88:616:84//Hs.79672:AB014552
F-THYRO1000206//EST//0.96:291:61//Hs.104962:AA443848
F-THYRO1000221//Human clone 23589 mRNA sequence//0.035:242:62//Hs.11506:U79297
F-THYRO1000241//EST//0.48:102:69//Hs.160764:AI313322
F-THYRO1000242//Zinc finger protein 84 (HPF2)//1.2e-42:534:64//Hs.9450:M27878
F-THYRO1000253//Homo sapiens mRNA for KIAA0690 protein, partial cds//0.61:211:64//Hs.60103:AB014590
F-THYRO1000270
F-THYRO1000279//ESTs//0.0020:104:72//Hs.121476:AI215500
F-THYRO1000288//Homo sapiens mRNA for Hs Ste24p, complete cds//1.3e-180:848:98//Hs.25846:AB016068
F-THYRO1000320//ESTs, Weakly similar to Similar to glutamate decarboxylase [C.elegans]//7.6e-92:431:99//Hs.122719:AA777803
F-THYRO1000327//Autocrine motility factor receptor//2.8e-52:290:93//Hs.80731:M63175
F-THYRO1000343//Homo sapiens mRNA for KIAA0790 protein, partial cds//7.2e-164:763:98//Hs.12002:AB018333
F-THYRO1000358//Human selenium-binding protein (hSBP) mRNA, complete cds//6.9e-34:177:84//Hs.7833:U29091
F-THYRO1000368//ESTs//0.0011:55:96//Hs.34994:AA252919
F-THYRO1000381//Homo sapiens mRNA for KIAA0562 protein, complete cds//0.081:240:62//Hs.118401:AB011134
F-THYRO1000387//EST//3.6e-14:197:71//Hs.139399:AA416855
F-THYRO1000394//ESTs, Weakly similar to No definition line found [C.elegans]//5.8e-39:245:91//Hs.119095:T79413
F-THYRO1000395//EST//5.8e-69:333:99//Hs.156524:AA724572
F-THYRO1000401//ESTs//1.8e-24:132:98//Hs.54852:W26238
F-THYRO1000438//EST//1.9e-05:217:63//Hs.115930:AA579773
F-THYRO1000452//B cell lymphoma protein 6 (zinc finger protein 51)//0.096:306:60//Hs.155024:U00115
F-THYRO1000471//Tyrosine aminotransferase//5.6e-44:403:77//Hs.2999:X52520
F-THYRO1000484//EST, Weakly similar to putative p150 [H.sapiens]//8.9e-22:248:76//Hs.162011:AA513663
F-THYRO1000488
F-THYRO1000501//H.sapiens Staf50 mRNA//3.2e-75:615:77//Hs.68054:X82200
F-THYRO1000502//ESTs//1.0:350:57//Hs.119749:AA689298
F-THYRO1000505//Interleukin 13//0.95:245:60//Hs.845:U31120
F-THYRO1000558//EST//1.3e-24:351:64//Hs.142326:AA351877
F-THYRO1000569//Homo sapiens mRNA for dihydropyrimidinase related protein 4, complete cds//0.28:229:61//Hs.100058:AB006713
F-THYRO1000570//EST//0.80:171:61//Hs.112790:AA609949
F-THYRO1000585//Homo sapiens protein associated with Myc mRNA, complete cds//2.4e-168:808:97//Hs.151411:AF075587
F-THYRO1000596//EST//9.5e-94:461:96//Hs.135397:AI056322
F-THYRO1000602//EST//4.9e-06:80:80//Hs.162135:AA526331
F-THYRO1000605//Guanylate cyclase 1, soluble, alpha 2//0.44:182:62//Hs.2685:Z50053
F-THYRO1000625//Thromboxane A2 receptor//4.5e-45:323:82//Hs.89887:D38081
F-THYRO1000637//ESTs//4.4e-24:255:75//Hs.101014:AA194941
F-THYRO1000641//ESTs//0.00017:375:58//Hs.32703:AA054125
F-THYRO1000658//CD4 receptor {exons 1 and 2} [human, T-lymphocyte, mRNA, 3429 nt]//1.8e-09:127:77//Hs.116007:S79267
F-THYRO1000662
F-THYRO1000666//ESTs//1.9e-28:149:99//Hs.105187:AI394157
F-THYRO1000676//CD4 receptor {exons 1 and 2} [human, T-lymphocyte, mRNA, 3429 nt]//5.7e-49:281:77//Hs.116007:S79267
F-THYRO1000684//ESTs, Weakly similar to band-6-protein [H.sapiens]//0.46:368:57//Hs.26557:AA480380
F-THYRO1000699//ESTs//1.6e-10:314:65//Hs.139212:AA243452
F-THYRO1000712//ESTs//3.3e-42:211:99//Hs.69330:AI056324
F-THYRO1000715//Human plectin (PLEC1) mRNA, complete cds//2.9e-06:631:59//Hs.79706:U53204
F-THYRO1000734//ESTs//8.4e-08:226:64//Hs.125754:AA806085
F-THYRO1000748//Homo sapiens KIAA0411 mRNA, complete cds//3.1e-35:339:74//Hs.7977:AB007871
F-THYRO1000756//Homo sapiens protocadherin (PCDH8) mRNA, complete cds//1.0:209:62//Hs.19492:AF061573
F-THYRO1000777//Human mRNA for KIAA0147 gene, partial cds//0.00069:636:57//Hs.158132:D63481
F-THYRO1000783//Homo sapiens Arp2/3 protein complex subunit p41-Arc (ARC41) mRNA, complete cds//0.70:452:58//Hs.11538:AF006084
F-THYRO1000787
F-THYRO1000793
F-THYRO1000796
F-THYRO1000805//Homo sapiens mRNA from chromosome 5q21-22, clone:sF2//9.4e-36:561:68//Hs.129685:AB002446
F-THYRO1000815//Human mRNA for KIAA0118 gene, partial cds//1.2e-45:465:75//Hs.154326:D42087
F-THYRO1000829//ESTs//1.7e-66:361:95//Hs.7906:H16339
F-THYRO1000843
F-THYRO1000852//ESTs//6.2e-23:204:81//Hs.144452:AA838788
F-THYRO1000855//ESTs//0.049:159:64//Hs.163532:AI424170
F-THYRO1000865//ESTs, Weakly similar to !!!! ALU SUBFAMILY J WARNING ENTRY !!!! [H.sapiens]//3.0e-33:190:75//Hs.133526:N21103
F-THYRO1000895//ESTs//3.8e-24:191:84//Hs.132722:AA618531
F-THYRO1000916//Homo sapiens mRNA, chromosome 1 specific transcript KIAA0487//1.8e-43:318:79//Hs.92381:AB007956
F-THYRO1000926//Homo sapiens cAMP-specific phosphodiesterase 8B (PDE8B) mRNA, partial cds//3.0e-179:839:98//Hs.78106:AF079529
F-THYRO1000934//PYRROLINE-5-CARBOXYLATE REDUCTASE//1.1e-33:759:63//Hs.79217:M77836
F-THYRO1000951//MUELLERIAN INHIBITING FACTOR PRECURSOR//0.055:662:56//Hs.112432:AC005263
F-THYRO1000952//Human mRNA for KIAA0208 gene, complete cds//0.98:177:65//Hs.83558:D86963
F-THYRO1000974//Homo sapiens putative ATP-dependent mitochondrial RNA helicase (SUV3) mRNA, nuclear gene encoding mitochondrial protein, complete cds//2.7e-15:123:90//Hs.106469:AF042169
F-THYRO1000975//EST//0.45:172:62//Hs.105449:AA513907
F-THYRO1000983
F-THYRO1000984//EST//0.0075:119:65//Hs.150347:AA984646
F-THYRO1000988//ESTs//0.056:99:71//Hs.153409:AI224307
F-THYRO1001003
F-THYRO1001031//Thiopurine S-methyltransferase//3.8e-44:568:71//Hs.51124:AF019369
F-THYRO1001033//H.sapiens mRNA for cylicin II//0.0061:287:60//Hs.3232:Z46788
F-THYRO1001062//ISLET AMYLOID POLYPEPTIDE PRECURSOR//3.2e-45:394:79//Hs.51048:X68830
F-THYRO1001093//Human mRNA for KIAA0355 gene, complete cds//3.4e-33:421:72//Hs.153014:AB002353
F-THYRO1001100//Human DNA-binding protein mRNA, 3'end//2.1e-74:741:74//Hs.159249:Z99130
F-THYRO1001120//Homo sapiens deltex (Dx) mRNA, complete cds//4.5e-18:447:62//Hs.124024:AF053700
F-THYRO1001121//ESTs//0.92:257:61//Hs.118246:N95416
F-THYRO1001133//EST//1.1e-38:367:75//Hs.144175:H70425
F-THYRO1001134//ESTs//1.4e-28:186:91//Hs.109468:W52074
F-THYRO1001142//ESTs//1.8e-44:332:82//Hs.146811:AA410788
F-THYRO1001173
F-THYRO1001177//ESTs//7.7e-40:240:84//Hs.155384:Z78385
F-THYRO1001189//ESTs//2.1e-36:323:76//Hs.120206:AI089163
F-THYRO1001204
F-THYRO1001213//Small inducible cytokine A5 (RANTES)//3.1e-43:256:81//Hs.155464:AF088219
F-THYRO1001262//ESTs//7.9e-44:279:87//Hs.138856:H47461
F-THYRO1001271//Homo sapiens mRNA for synaptogyrin 3//0.0045:273:60//Hs.6467:AJ002309
F-THYRO1001287//Homo sapiens alpha 1,2-mannosidase IB mRNA, complete cds//0.014:178:66//Hs.125315:AF027156
F-THYRO1001290//ESTs//3.9e-43:145:99//Hs.147797:AA069836
F-THYRO1001313//ESTs//1.0:244:61//Hs.127488:AA528182
F-THYRO1001320//ESTs//0.062:126:67//Hs.133296:AI311872
F-THYRO1001321//Homo sapiens DEC-205 mRNA, complete cds//2.5e-35:560:68//Hs.153563:AF011333
F-THYRO1001322//ESTs//0.12:238:61//Hs.29169:N66545
F-THYRO1001347//ESTs//7.5e-61:293:99//Hs.129962:AA927207
F-THYRO1001363//ESTs//1.0e-16:178:78//Hs.163954:N57939
F-THYRO1001365//Homo sapiens KIAA0417 mRNA, complete cds//3.6e-18:187:79//Hs.12385:AB007877
F-THYRO1001374//Homo sapiens mRNA for KIAA0707 protein, partial cds//7.4e-157:740:97//Hs.138488:AB014607
F-THYRO1001401//EST//4.6e-14:171:76//Hs.157587:AI356993
F-THYRO1001403//ESTs//2.2e-50:464:79//Hs.118046:N49946
F-THYRO1001405//ESTs//1.7e-44:226:98//Hs.156667:AI347694
F-THYRO1001406//Hydroxysteroid (17-beta) dehydrogenase 3//2.8e-20:459:62//Hs.477:U05659
F-THYRO1001411//ESTs//1.9e-41:342:78//Hs.146811:AA410788
F-THYRO1001426//Human ring zinc-finger protein (ZNF127-Xp) gene and 5' flanking sequence//4.6e-33:153:81//Hs.102877:U41315
F-THYRO1001434//ESTs//1.1e-07:274:60//Hs.151093:AI224099
F-THYRO1001458//Myosin, heavy polypeptide 9, non-muscle//6.2e-60:653:71//Hs.44782:Z82215
F-THYRO1001480//ISLET AMYLOID POLYPEPTIDE PRECURSOR//1.3e-42:370:78//Hs.51048:X68830
F-THYRO1001487//EST//1.0:88:71//Hs.160760:AI311943
F-THYRO1001534//ESTs//1.2e-94:457:98//Hs.125523:AA883904
F-THYRO1001537//ESTs//3.5e-94:469:97//Hs.106448:R76663
F-THYRO1001541//EST//1.4e-10:158:65//Hs.145159:AI150211
F-THYRO1001559//ESTs//1.4e-07:91:81//Hs.43507:N24046
F-THYRO1001570//ESTs//2.3e-41:280:80//Hs.119752:AA703335
F-THYRO1001573//Homo sapiens clone 24778 unknown mRNA//2.7e-105:546:95//Hs.25306:AF070572
F-THYRO1001584//Human RGP3 mRNA, complete cds//0.14:335:58//Hs.82294:U27655
F-THYRO1001595//Human RSU-1/RSP-1 mRNA, complete cds//3.6e-35:165:84//Hs.75551:L12535
F-THYRO1001602//ESTs//3.1e-42:350:80//Hs.138384:R72849
F-THYRO1001605//EST//0.11:426:57//Hs.151206:AI126071
F-THYRO1001617//ESTs//5.2e-43:345:81//Hs.8710:W07046
F-THYRO1001637//ESTs, Weakly similar to anion exchanger [H.sapiens]//5.2e-13:108:86//Hs.141045:AA191659
F-THYRO1001656//Solute carrier family 2 (facilitated glucose transporter), member 4//0.099:540:55//Hs.95958:M91463
F-THYRO1001661//ESTs//0.12:53:92//Hs.151586:W45568
F-THYRO1001671//Homo sapiens mRNA for 2'-5' oligoadenylate synthetase 59 kDa isoform//8.0e-166:780:98//Hs.118633:AJ225089
F-THYRO1001673//Von Hippel-Lindau syndrome//4.6e-25:212:73//Hs.78160:AF010238
F-THYRO1001703//Homo sapiens clone 24767 mRNA sequence//0.27:421:57//Hs.122908:AF070552
F-THYRO1001706//ESTs//1.8e-24:142:95//Hs.112536:AI147691
F-THYRO1001721//ESTs, Highly similar to RING CANAL PROTEIN [Drosophila melanogaster]//2.5e-51:296:92//Hs.3826:U69560
F-THYRO100173 8//EST//6.9e-30:180:94//Hs.58641:W81229
F-THYRO1001745//ESTs//6.1e-49:244:98//Hs.97534:AA398813
F-THYRO1001746//EST//0.96:119:63//Hs.144107:AI053590
F-THYRO1001772//ESTS, Weakly similar to !!!! ALU SUBFAMILY J WARNING ENTRY !!!! [H.sapiens]//2.2e-21:182:81//Hs.118053:N75725
F-THYRO1001793//ESTs//1.9e-93:439:99//Hs.150116:AI299324
F-THYRO1001809//Human mRNA for KIAA0297 gene, partial cds//0.47:168:67//Hs.11711:AB002295
F-THYRO1001828
F-THYRO1001854//EST//0.038:128:67//Hs.160649:AI241823
F-THYRO1001895//Intercellular adhesion molecule 1 (CD54), human rhinovirus receptor//9.6e-13:288:65//Hs.51061:M24283
F-THYRO1001907//EST//1.9e-12:126:80//Hs.139296:AA350198
F-VESEN1000122
F-Y79AA1000013//ESTs//1.7e-72:369:96//Hs.97176:AA447885
F-Y79AA1000033
F-Y79AA1000037//Murine leukemia viral (bmi-1) oncogene homolog//7.8e-21:230:66//Hs.431:L13689
F-Y79AA1000059//Homo sapiens immunophilin homolog ARA9 mRNA, complete cds//7.3e-40:629:64//Hs.75305:U78521
F-Y79AA1000065//CD81 ANTIGEN//0.0050:241:60//Hs.54457:M33680
F-Y79AA1000131//Guanylate cyclase 1, soluble, alpha 2//0.078:477:58//Hs.2685:Z50053
F-Y79AA1000181//Fatty acid synthase {3' region} [human, breast and HepG2 cells, mRNA Partial, 2237 nt]//0.0022:684:58//Hs.83190:U29344
F-Y79AA1000202//ESTs//2.5e-17:143:86//Hs.76925:AA211860
F-Y79AA1000214//Homo sapiens histone H2A.F/Z variant (H2AV) mRNA, complete cds//3.9e-73:345:100//Hs.9242:AF081192
F-Y79AA1000230//Polymeric immunoglobulin receptor//0.98:335:59//Hs.842:X73079
F-Y79AA1000231//ESTs//0.11:209:66//Hs.132184:AI278623
F-Y79AA1000258//Homo sapiens metase (MET-1) mRNA, complete cds//0.30:444:61//Hs.99941:L23134
F-Y79AA1000268//Human mRNA for KIAA0367 gene, partial cds//9.1e-11:300:64//Hs.23311:AB002365
F-Y79AA1000313//Human mRNA for KIAA0129 gene, complete cds//0.89:744:56//Hs.44361:D50919
F-Y79AA1000328
F-Y79AA1000342//Homo sapiens OPA-containing protein mRNA, complete cds//8.4e-15:223:75//Hs.85313:AF071309
F-Y79AA1000346
F-Y79AA1000349//ALPHA-2C-1 ADRENERGIC RECEPTOR//8.3e-06:180:73//Hs.123022:J03853
F-Y79AA1000355
F-Y79AA1000368//ESTs//0.0062:235:64//Hs.114777:AA782908
F-Y79AA1000405//ESTs//0.76:244:62//Hs.153027:AA648897
F-Y79AA1000410//Small inducible cytokine A5 (RANTES)//8.1e-31:229:83//Hs.155464:AF088219
F-Y79AA1000420//ESTs//1.1e-53:271:87//Hs.13056:AA181018
F-Y79AA1000469//Homo sapiens I-1 receptor candidate protein mRNA, complete cds//0.0047:315:66//Hs.26285:AF082516
F-Y79AA1000480
F-Y79AA1000538//ESTs//5.7e-09:110:77//Hs.98790:AA284871
F-Y79AA1000539//ESTs//2.6e-52:412:77//Hs.81648:W26521
F-Y79AA1000540//Homo sapiens chromosome 7q22 sequence//0.70:133:69//Hs.151555:AF053356
F-Y79AA1000560//Homo sapiens gamma2-adaptin (G2AD) mRNA, complete cds//1.2e-07:371:63//Hs.8991:AF068706
F-Y79AA1000574//Human mRNA for GC box bindig protein, complete cds//0.95:258:62//Hs.150557:D31716
F-Y79AA1000589//Homo sapiens clone 614 unknown mRNA, complete sequence//2.8e-154:755:97//Hs.21811:AF091080
F-Y79AA10006277/Homo sapiens zinc finger protein (ZF5128) mRNA, complete cds//1.7e-136:644:98//Hs.60580:AF060503
F-Y79AA1000705//Homo sapiens CHD1 mRNA, complete cds//0.0023:523:59//Hs.22670:AF006513
F-Y79AA1000734//Homo sapiens peroxisomal biogenesis factor (PEX11b) mRNA, complete cds//1.6e-181:850:98//Hs.83023:AF093670
F-Y79AA1000748//ESTs//4.2e-12:95:90//Hs.33687:R85969
F-Y79AA1000752//ESTs//8.1e-114:551:97//Hs.153471:AI198377
F-Y79AA1000774//ESTs//2.9e-59:296:98//Hs.150536:W20067
F-Y79AA1000782//EST//0.97:78:69//Hs.147351:AI208468
F-Y79AA1000784//Homo sapiens RanBP7/importin 7 mRNA, complete cds//1.1e-178:847:97//Hs.5151:AF098799
F-Y79AA1000794//G-rich RNA sequence binding factor 1//0.83:228:61//Hs.79295:U07231
F-Y79AA1000800//Homo sapiens GABA-B receptor mRNA, complete cds//0.12:244:60//Hs.12307:AF056085
F-Y79AA1000802//Homo sapiens actin binding protein MAYVEN mRNA, complete cds//0.87:466:59//Hs.122967:AF059569
F-Y79AA1000805
F-Y79AA1000824//Titin//1.0:437:58//Hs.83049:X90568
F-Y79AA1000827//Fatty acid synthase {3' region} [human, breast and HepG2 cells, mRNA Partial, 2237 nt]//0.0048:630:57//Hs.83190:U29344
F-Y79AA1000833//TUBULIN ALPHA-4 CHAIN//6.9e-107:603:90//Hs.75318:X06956
F-Y79AA1000850//ESTs, Weakly similar to T22C1.7 [C.elegans]//6.0e-77:368:99//Hs.86660:AA398644
F-Y79AA1000962//Homo sapiens orphan nuclear hormone receptor BD73 mRNA, 3' end//0.14:499:58//Hs.37288:D16815
F-Y79AA1000966//ESTs//0.80:52:86//Hs.6671:AI341699
F-Y79AA1000968//ESTs, Moderately similar to initiation factor eIF-2B gamma subunit [R.norvegicus]//6.9e-69:310:94//Hs.76822:AI359536
F-Y79AA1000969//LYMPHOTOXIN-BETA RECEPTOR PRECURSOR//1.0:150:64//Hs.1116:L04270
F-Y79AA1000976//Arachidonate 15-lipoxygenase//0.87:174:66//Hs.73809:M23892
F-Y79AA1000985//Human plectin (PLEC1) mRNA, complete cds//0.091:385:58//Hs.79706:U53204
F-Y79AA1001023
F-Y79AA1001041//Human mutY homolog (hMYH) gene, complete cds//0.99:37:100//Hs.78489:U63329
F-Y79AA1001048//Acyl-Coenzyme A dehydrogenase, very long chain//8.7e-30:772:60//Hs.82208:L46590
F-Y79AA1001061//ESTs//6.3e-41:303:84//Hs.55855:AA621381
F-Y79AA1001068//EST//3.0e-23:165:90//Hs.157607:AI357511
F-Y79AA1001077//ESTs//4.9e-40:237:94//Hs.11197:AA309047
F-Y79AA1001078
F-Y79AA1001105//Homo sapiens homeodomain protein (OG12) mRNA, complete cds//6.5e-11:247:66//Hs.55967:AF022654
F-Y79AA1001145//ESTs//1.3e-20:234:75//Hs.55855:AA621381
F-Y79AA1001167//Homo sapiens mRNA for KIAA0750 protein, complete cds//1.0:155:63//Hs.5444:AB018293
F-Y79AA1001177//Human hSIAH2 mRNA, complete cds//6.5e-09:299:65//Hs.20191:U76248
F-Y79AA1001185//ESTs//1.7e-56:318:93//Hs.102991:AA639646
F-Y79AA1001211//ESTs//9.1e-108:503:99//Hs.100605:AA305965
F-Y79AA1001216//Peroxisome receptor 1//0.00028:458:57//Hs.158084:Z48054
F-Y79AA1001228//Fragile X mental retardation 2//0.040:207:64//Hs.54472:U48436
F-Y79AA1001233//ESTRADIOL 17 BETA-DEHYDROGENASE 1//6.5e-25:731:60//Hs.85279:U34879
F-Y79AA1001236//Homo sapiens mRNA for JM23 protein, complete coding sequence (clone IMAGE 34581 and IMAGE 45355 and LLNLc110I133Q7 (RZPD Berlin))//4.0e-135:441:97//Hs.23170:AJ005892
F-Y79AA1001281//ESTs//2.7e-21:157:88//Hs.163825:AI393240
F-Y79AA1001299//Human Ini1 mRNA, complete cds//2.2e-116:323:93//Hs.155626:U04847
F-Y79AA1001312//ESTs//3.7e-95:448:99//Hs.104469:W38395
F-Y79AA1001323//ESTs//8.9e-50:340:86//Hs.144198:AI017555
F-Y79AA1001384
F-Y79AA1001391//Human Hoxb-13 mRNA, complete cds//8.6e-42:505:70//Hs.66731:U81599
F-Y79AA1001394//ESTs, Weakly similar to F54B3.3 [C.elegans]//1.5e-90:424:96//Hs.154221:H23167
F-Y79AA1001402//ESTs//1.0:245:62//Hs.134695:AI088489
F-Y79AA1001493//SRY (sex determining region Y)-box 4//0.38:311:61//Hs.83484:X70683
F-Y79AA1001511//ESTs//9.9e-105:487:99//Hs.153581:AA630465
F-Y79AA1001533//ESTs, Highly similar to RETROVIRUS-RELATED POL POLYPROTEIN [Homo sapiens]//0.95:256:63//Hs.29974:AI360447
F-Y79AA1001541//EST//0.96:202:61//Hs.99141:AA447744
F-Y79AA1001548//ESTs//2.6e-25:166:90//Hs.164036:AA845659
F-Y79AA1001555//ESTs//1.6e-35:191:97//Hs.52885:H29851
F-Y79AA1001581//Cyclin-dependept kinase inhibitor 1C (p57, Kip2)//2.5e-05:272:64//Hs.106070:U22398
F-Y79AA1001585//ESTs//1.1e-84:473:93//Hs.42547:AA210783
F-Y79AA1001594//ESTs//1.7e-08:169:71//Hs.97366:AA393109
F-Y79AA1001603//ESTs//4.6e-07:429:59//Hs.160422:AI363426
F-Y79AA1001613//Homo sapiens mRNA for KIAA0683 protein, complete cds//0.00078:520:57//Hs.12334:AB014583
F-Y79AA1001647//ESTs, Weakly similar to ZK1058.5 [C.elegans]//9.4e-79:421:94//Hs.107039:W27244
F-Y79AA1001665//VON WILLEBRAND FACTOR PRECURSOR//1.0:386:60//Hs.110802:X04385
F-Y79AA1001679//Guanine nucleotide binding protein (G protein), beta polypeptide 1//0.88:243:61//Hs.3620:X04526
F-Y79AA1001692//Insulin-like growth factor binding protein 2//1.9e-06:426:59//Hs.162:X16302
F-Y79AA1001696//ESTs//2.3e-44:249:94//Hs.163665:AA250877
F-Y79AA1001705//Homo sapiens interleukin-1 receptor-associated kinase (IRAK) mRNA, complete cds//0.19:609:58//Hs.77297:L76191
F-Y79AA1001711//ESTs//5.2e-29:224:83//Hs.100461:AI018620
F-Y79AA1001781//Homo sapiens KIAA0443 mRNA, complete cds//0.49:183:66//Hs.113082:AB007903
F-Y79AA1001805//ESTs//1.1e-62:315:98//Hs.16141:W56079
F-Y79AA1001827//ESTs, Weakly similar to Similar to S.cerevisiae YD9335.03c protein [H.sapiens]//2.9e-62:313:98//Hs.15709:W81213
F-Y79AA1001846//ESTs//9.4e-16:146:82//Hs.140588:H60533
F-Y79AA1001848//ESTs, Weakly similar to KIAA0390 [H.sapiens]//1.6e-19:142:90//Hs.103349:AI141124
F-Y79AA1001866//Homo sapiens mRNA for zinc finger protein 10//5.1e-09:215:67//Hs.104115:X52332
F-Y79AA1001874//Homo sapiens Jagged 2 mRNA, complete cds//5.4e-06:412:62//Hs.106387:AF029778
F-Y79AA1001875//ESTs//6.8e-09:198:67//Hs.138036:AI343173
F-Y79AA1001923//Homo sapiens growth-arrest-specific protein (gas) mRNA, complete cds//0.98:430:58//Hs.78501:L13720
F-Y79AA1001963//ESTs//8.1e-131:642:97//Hs.54971:AI424382
F-Y79AA1002027//ESTs//0.00042:58:91//Hs.5375:AA620611
F-Y79AA1002083//ESTs//2.5e-51:285:95//Hs.117205:W88943
F-Y79AAl002089//ESTs, Weakly similar to putative p150 [H.sapiens]//8.3e-53:348:88//Hs.18122:AI338045
F-Y79AA1002093
F-Y79AA1002103//ESTs//1.5e-15:223:71//Hs.97427:AA411865
F-Y79AA1002115
F-Y79AA1002125//ESTs//6.5e-41:206:99//Hs.159257:N40395
F-Y79AA1002139//ESTs, Weakly similar to B0035.14 [C.elegans]//1.2e-24:165:90//Hs.6473:AA853955
F-Y79AA1002204//Homo sapiens mRNA for KIAA0638 protein, partial cds//9.5e-05:393:62//Hs.77864:AB014538
F-Y79AA1002208//ESTs//2.7e-13:211:69//Hs.112469:AA598515
F-Y79AA1002209//ESTs, Weakly similar to TYROSYL-TRNA SYNTHETASE [Bacillus caldotenax]//2.3e-113:568:96//Hs.111637:AA305890
F-Y79AA1002210//ESTs, Weakly similar to D2045.8 [C.elegans]//8.6e-33:338:73//Hs.26662:U55984
F-Y79AA1002211//ESTs//2.6e-15:121:75//Hs.159584:AA524477
F-Y79AA1002220//EST//0.010:360:60//Hs.136341:AA482508
F-Y79AA1002229//Human mRNA for KIAA0086 gene, complete cds//0.0041:203:63//Hs.1560:D42045
F-Y79AA1002234//Homo sapiens mRNA for KIAA0692 protein, partial cds//4.1e-176:821:98//Hs.100729:AB014592
F-Y79AA1002246//Human involucrin mRNA//5.6e-05:525:59//Hs.157091:M13903
F-Y79AA1002258//Homo sapiens mRNA for KIAA0655 protein, partial cds//2.2e-160:748:98//Hs.96731:AB014555
F-Y79AA1002298//ESTs//2.5e-05:115:77//Hs.87164:T84489
F-Y79AA1002307//Homo sapiens mRNA for KIAA0634 protein, partial cds//2.1e-130:622:97//Hs.30898:AB014534
F-Y79AA1002311//ESTs//4.9e-19:126:94//Hs.58595:AA830999
F-Y79AA1002351//Human high conductance inward rectifier potassium channel alpha subunit mRNA, complete cds//0.028:587:58//Hs.2363:L36069
F-Y79AA1002361//ESTs//8.7e-29:149:100//Hs.156074:AA824377
F-Y79AA1002399
F-Y79AA1002407//ESTs//1.5e-25:183:89//Hs.110031:T52569
F-Y79AA1002416//CTP synthetase//9.1e-51:489:72//Hs.84112:X52142
F-Y79AA1002431
F-Y79AA1002433//EST//0.0037:94:71//Hs.136780:AA772318
F-Y79AA1002472//Homo sapiens DNA from chromosome 19, BAC 33152//1.1e-37:263:69//Hs.55452:AC003973
F-Y79AA1002482//ESTs//1.4e-49:313:80//Hs.132590:AI160765
F-Y79AA1002487//Insulin-like growth factor binding protein 2//0.43:249:61//Hs.162:X16302

### Homology Search Result Data 5.

The result of the homology search of the Human Unigene using the clone sequence of 3'-end.

Data include
the name of clone,
title of the top hit data,
the P-value: the length of the compared sequence: identity (%), and
the Accession No. of the top hit data, as in the order separated by //.

Blank indicates that the 3'-end sequence corresponding to the 5'-end was not determined in the clone.

Data are not shown for the clones in which the P-value was higher than 1.
R-HEMBA1000005//ESTs, Highly similar to HYPOTHETICAL 31.6 KD PROTEIN F54F2.9 IN CHROMOSOME III [Caenorhabditis elegans]//5.6e-93:501:93//Hs.13015:AA628434
R-HEMBA1000030//Human POU domain protein (Brn-3b) mRNA, complete cds//0.83:314:61//Hs.266:U06233
R-HEMBA1000042//Archain//1:4e-45:282:89//Hs.33642:X81198
R-HEMBA1000046//Human mRNA for KIAA0118 gene, partial cds//8.3e-52:528:72//Hs.154326:D42087
R-HEMBA1000050//EST//0.043:155:63//Hs.149031:AI243340
R-HEMBA1000076//ESTs//3.1e-77:394:97//Hs.111742:R39329
R-HEMBA1000111//ESTs//1.7e-33:228:85//Hs.146811:AA410788
R-HEMBA1000129//ESTs, Weakly similar to contains similarity to helicases [C.elegans]//4.4e-90:502:90//Hs.55918:AA151667
R-HEMBA1000141//Homo sapiens mRNA for KIAA0797 protein, partial cds//2.1e-100:514:94//Hs.27197:AB018340
R-HEMBA1000150//Homo sapiens mRNA for KIAA0640 protein, partial cds//3.1e-45:435:77//Hs.153026:AB014540
R-nnnnnnnnnnnn//ESTs, Moderately similar to The KIAA0138 gene product is novel. [H.sapiens]//7.7e-92:428:100//Hs.126925:AA931237
R-HEMBA1000158
R-nnnnnnnnnnnn//ESTs, Weakly similar to F13B12.1 [C.elegans]//1.3e-05:58:91//Hs.5570:AI377863
R-HEMBA1000180//ESTs//7.7e-90:461:95//Hs.159200:N50545
R-HEMBA1000185//ESTs//1.3e-72:371:96//Hs.134506:AA308366
R-HEMBA1000193//ESTs//4.2e-103:481:99//Hs.143251:AA769927
R-HEMBA1000201//Human Ini1 mRNA, complete cds//3.0e-25:137:99//Hs.155626:U04847
R-HEMBA1000213//ESTs//5.4e-85:465:94//Hs.23412:AA133311
R-HEMBA1000216//ESTs//3.0e-37:311:79//Hs.137875:AA993532
R-nnnnnnnnnnnn//EST//2.2e-100:498:96//Hs.161570:W80404
R-HEMBA1000231//Homo sapiens KIAA0414 mRNA, partial cds//2.7e-34:287:70//Hs.127649:AB007874
R-HEMBA1000243//Homo sapiens mRNA for KIAA0475 protein, complete cds//1.3e-23:276:75//Hs.5737:AB007944
R-HEMBA1000244//ESTs//2.3e-88:455:96//Hs.8929:AA719019
R-HEMBA1000251//ESTs//0.96:411:56//Hs.120277:AI243808
R-HEMBA1000264//ESTs//3.7e-97:487:96//Hs.29258:W37424
R-nnnnnnnnnnnn//ESTs, Moderately similar to ovarian-specific protein [R.norvegicus]//4.9e-14:208:73//Hs.93332:AA811920
R-HEMBA1000282//ESTs//2.5e-38:216:94//Hs.120757:R92485
R-HEMBA1000288//ESTs//2.6e-43:289:86//Hs.151365:AA643962
R-HEMBA1000290//ESTs//5.1e-110:543:96//Hs.139068:AA516409
R-HEMBA1000302//Homo sapiens mRNA for KIAA0527 protein, partial cds//1.0:122:67//Hs.129748:AB011099
R-nnnnnnnnnnnn//ESTs//7.4e-76:386:97//Hs.22276:AA191323
R-nnnnnnnnnnnn//Human Ca2⁺-dependent activator protein for secretion mRNA, complete cds//8.8e-30:160:98//Hs.151301:U36448
R-HEMBA1000307//ESTs, Highly similar to 8A-2V protein [M.musculus]//1.1e-103:489:99//Hs.108881:AI018024
R-nnnnnnnnnnnn//ESTs//9.3e-99:472:98//Hs.163512:AA903238
R-HEMBA1000338//EST//5.1e-49:278:92//Hs.150815:AI302560
R-HEMBA1000351//Human high-affinity copper uptake protein (hCTR1) mRNA, complete cds//1.1e-42:270:88//Hs.73614:U83460
R-HEMBA1000355//ESTs//1.0e-105:531:96//Hs.61762:AI422243
R-HEMBA1000357//Human kpni repeat mrna (cdna clone pcd-kpni-4), 3' end//9.4e-89:432:87//Hs.139107:K00629
R-HEMBA1000366//ESTs//1.1e-99:524:95//Hs.11785:T65857
R-HEMBA1000369//ESTs//6.5e-70:355:96//Hs.124847:AA843938
R-HEMBA1000376//Human mRNA for KIAA0205 gene, complete cds//3.6e-44:388:77//Hs.3610:D86960
R-HEMBA1000387//Human high-affinity copper uptake protein (hCTR1) mRNA, complete cds//5.5e-47:337:83//Hs.73614:U83460
R-HEMBA1000390//Oxytocin receptor//2.4e-16:428:62//Hs.2820:X64878
R-HEMBA1000392//ESTs//3.9e-105:531:96//Hs.130661:AI340248
R-HEMBA1000396//ESTs, Weakly similar to line-1 protein ORF2 [H.sapiens]//1.1e-44:447:75//Hs.42849:N31920
R-HEMBA1000411//ESTs, Weakly similar to ankyrin 3, long form [H.sapiens]//6.1e-92:373:99//Hs.48675:AI005282
R-HEMBA1000418//ESTs//3.1e-66:315:100//Hs.94133:AI270700
R-HEMBA1000422//ESTs//1.6e-99:464:99//Hs.33024:AA002140
R-HEMBA1000428//Homo sapiens mRNA for oligophrenin 1//4.9e-85:535:87//Hs.158122:AJ001189
R-HEMBA1000434//ESTs//3.7e-53:266:99//Hs.22782:Z38143
R-HEMBA1000442//ESTs//0.93:322:57//Hs.144763:AI218014
R-HEMBA1000456//ESTs//4.1e-48:277:93//Hs.6937:AA524349
R-HEMBA1000459//ESTs//0.010:184:63//Hs.128797:AI246316
R-HEMBA1000460
R-HEMBA1000464//EST//0.082:87:70//Hs.147977:AI262370
R-HEMBA1000469//Small inducible cytokine A5 (RANTES)//1.4e-65:494:81//Hs.155464:AF088219
R-HEMBA1000488//ESTs, Weakly similar to The KIAA0132 gene product is related to Drosophila melanogaster ring canel protein. [H.sapiens]//1.1e-31:181:94//Hs.61454:AA312449
R-HEMBA1000490//ESTs//6.4e-17:132:86//Hs.32855:N25528
R-HEMBA1000491//ESTs//2.2e-22:171:85//Hs.8035:AA195087
R-HEMBA1000504//ESTs//0.016:282:58//Hs.130778:AI077571
R-HEMBA1000505//EST//6.1e-15:116:87//Hs.162783:AA627318
R-HEMBA1000508//ESTs//1.1e-28:244:81//Hs.132722:AA618531
R-HEMBA1000518//EST//0.60:141:60//Hs.97831:AA400885
R-HEMBA1000519//ESTs//2.8e-64:334:96//Hs.97885:AA402414
R-HEMBA1000520//ESTs//6.9e-104:503:97//Hs.18370:AA947280
R-HEMBA1000523//Cleavage stimulation factor, 3' pre-RNA, subunit 3, 77kD//4.0e-55:203:92//Hs.155510:U15782
R-HEMBA1000531//ESTs, Weakly similar to HEAT SHOCK 70 KD PROTEIN 1 [H.sapiens]//1.3e-117:550:99//Hs.99722:AI422277
R-HEMBA1000540//ESTs//4.7e-72:350:98//Hs.109755:AA180809
R-HEMBA1000545//Homo sapiens clone 23892 mRNA sequence//3.7e-68:549:80//Hs.91916:AF035317
R-nnnnnnnnnnnn//ESTs//2.3e-66:342:97//Hs.71916:AA219699
R-HEMBA1000557//EST//1.5e-49:297:90//Hs.149580:AI281881
R-HEMBA1000561//ESTs, Moderately similar to zinc finger protein [R.norvegicus]//1.8e-108:550:96//Hs.26799:W74481
R-HEMBA1000563//Adenosine kinase//0.16:367:58//Hs.94382:U50196
R-HEMBA1000568//ESTs//5.1e-42:321:82//Hs.141024:H07128
R-nnnnnnnnnnnn
R-HEMBA1000575//ESTs//3.8e-45:352:80//Hs.146811:AA410788
R-HEMBA1000588//ESTs//0.18:122:67//Hs.140507:AA761944
R-HEMBA1000591//Homo sapiens mRNA for ElB-55kDa-associated protein//3.9e-113:591:94//Hs.155218:AJ007509
R-HEMBA1000592//TYROSINE-PROTEIN KINASE
ITK/TSK//0.024:309:61//Hs.89519:L10717
R-HEMBA1000594//ESTs//8.6e-07:172:68//Hs.160289:AI168041
R-HEMBA1000604//Human telomerase-associated protein TP-1 mRNA, complete cds//1.5e-19:129:93//Hs.158334:U86136
R-HEMBA1000608//ESTs//2.2e-95:506:94//Hs.6103:AA496424
R-HEMBA1000622//ESTs//3.8e-10:440:61//Hs.137538:AA769438
R-HEMBA1000636//ESTs, Weakly similar to 50S RIBOSOMAL PROTEIN L20 [E.coli]//1.4e-86:422:97//Hs.26252:AA643235
R-HEMBA1000637//Homo sapiens mRNA for KIAA0690 protein, partial cds//3.7e-99:443:97//Hs.60103:AB014590
R-HEMBA1000655//Human mRNA for KIAA0392 gene, partial cds//1.3e-50:426:79//Hs.40100:AB002390
R-HEMBA1000657//ESTs//3.0e-74:419:93//Hs.109477:AA477929
R-HEMBA1000662//EST//1.1e-90:425:99//Hs.122144:AA780136
R-HEMBA1000673//ESTs//1.2e-101:473:99//Hs.138215:AI123922
R-HEMBA1000682//ESTs, Weakly similar to putative pi 50 [H.sapiens]//3.5e-114:553:97//Hs.111730:AA604403
R-HEMBA1000686//ESTs, Weakly similar to C27F2.7 gene product [C.elegans]//6.8e-18:137:86//Hs.7049:AI141736
R-HEMBA1000702//Human mRNA for tryptophan hydroxylase (EC 1.14.16.4)//7.4e-52:345:84//Hs.144563:AF057280
R-HEMBA1000705//EST//0.21:139:63//Hs.132687:AI033672
R-HEMBA1000719//ESTs//8.4e-90:484:94//Hs.29005:AA477213
R-HEMBA1000722//ESTs, Weakly similar to similar to enoyl-COA hydratases/isomerases [C.elegans]//7.2e-113:572:95//Hs.28644:AI018612
R-HEMBA1000726//ERYTHROCYTE BAND 7 INTEGRAL MEMBRANE PROTEIN//2.8e-40:449:75//Hs.74478:U33931
R-HEMBA1000727//ESTs//0.0047:267:60//Hs.133095:AA927777
R-HEMBA1000747//EST//3.9e-20:160:85//Hs.99048:AA446110
R-HEMBA1000749//Small inducible cytokine A5 (RANTES)//4.7e-37:286:82//Hs.155464:AF088219
R-HEMBA1000752//EST//0.041:39:94//Hs.127772:AA961131
R-HEMBA1000769//Homo sapiens mRNA for chemokine LEC precursor, complete cds//1.6e-32:309:75//Hs.10458:AF088219
R-HEMBA1000773//EST//7.5e-05:201:63//Hs.122887:AA767612
R-HEMBA1000774//Kangai 1 (suppression of tumorigenicity 6, prostate; CD82 antigen (R2 leukocyte antigen, antigen detected by monoclonal and antibody IA4))//1.3e-48:284:90//Hs.103458:X53795
R-HEMBA1000791//Human mRNA for KIAA0118 gene, partial cds//1.2e-45:291:87//Hs.154326:D42087
R-HEMBA1000817//ESTs//8.3e-95:445:99//Hs.107357:AA983939
R-HEMBA1000822//ESTs//1.1e-107:522:97//Hs.92832:AA631027
R-HEMBA1000827//Homo sapiens Ser/Arg-related nuclear matrix protein (SRM160) mRNA, complete cds//2.2e-44:228:98//Hs.18192:AF048977
R-HEMBA1000843//Homo sapiens LIM protein mRNA, complete cds//6.6e-46:410:77//Hs.154103:AF061258
R-HEMBA1000851
R-HEMBA1000852//Aldehyde dehydrogenase 10 (fatty aldehyde dehydrogenase)//3.7e-33:284:80//Hs.159608:U46689
R-HEMBA1000867//EST//2.0e-17:211:74//Hs.145670:AI265794
R-HEMBA1000869//ESTs//3.1e-16:237:71//Hs.116518:AA653202
R-HEMBA1000870//ESTs//1.6e-43:222:98//Hs.69564:AA203608
R-HEMBA1000872//ESTs//1.9e-93:453:98//Hs.152622:AA594951
R-HEMBA1000876//Small inducible cytokine A5 (RANTES)//3.0e-41:329:79//Hs.155464:AF088219
R-HEMBA1000908//ESTs//1.6e-51:291:92//Hs.12247:AI203154
R-HEMBA1000910//EST//0.98:139:64//Hs.132687:AI033672
R-HEMBA1000918//EST//9.6e-30:152:84//Hs.162136:AA526508
R-HEMBA1000919
R-HEMBA1000934//ESTs//4.1e-38:254:89//Hs.87784:AA460597
R-HEMBA1000942//ESTs//3.5e-20:172:69//Hs.160065:AI018619
R-HEMBA1000943//Homo sapiens mRNA for KIAA0748 protein, complete cds//1.3e-44:281:78//Hs.33187:AB018291
R-HEMBA1000946//ESTs//1.6e-68:352:96//Hs.21331:H93074
R-HEMBA1000960//Homo sapiens tapasin (NGS-17) mRNA, complete cds//4.0e-61:347:81//Hs.5247:AF029750
R-HEMBA1000968//Homo sapiens mRNA, chromosome 1 specific transcript KIAA0508//6.8e-51:362:84//Hs.159187:AB007977
R-HEMBA1000971//ESTs//2.8e-41:246:91//Hs.104287:AI363498
R-HEMBA1000972//Homo sapiens mRNA for XPR2 protein//7.3e-44:341:81//Hs.44766:AJ007590
R-HEMBA1000974//ESTs//1.4e-32:166:100//Hs.149274:AI018170
R-HEMBA1000975//Oxytocin receptor//2.7e-46:563:73//Hs.2820:X64878
R-HEMBA1000985//ESTs//4.4e-05:125:69//Hs.147434:AI214464
R-HEMBA1000986//ESTs//7.8e-44:266:84//Hs.163784:N54902
R-HEMBA1000991//EST//1.4e-42:162:86//Hs.149580:AI281881
R-HEMBA1001007
R-HEMBA1001008//ESTs//2.3e-82:463:92//Hs.10339:AA058764
R-HEMBA1001009//ESTs, Weakly similar to non-lens beta gamma-crystallin like protein [H.sapiens]//2.6e-58:280:100//Hs.128738:AA970836
R-HEMBA1001017//Homo sapiens mRNA for KIAA0468 protein, complete cds//3.3e-115:587:95//Hs.158287:AB007937
R-HEMBA1001019//Cell division cycle 2, G1 to S and G2 to M//1.1e-24:140:95//Hs.58393:X05360
R-HEMBA1001020//ESTs//0.52:86:72//Hs.69683:AA115292
R-HEMBA1001022//ESTs//3.4e-18:102:100//Hs.63243:AI123912
R-HEMBA1001024//ESTs//1.9e-07:262:61//Hs.124399:AA832336
R-HEMBA1001026//ESTs//0.0017:142:67//Hs.144109:AI345543
R-nnnnnnnnnnnn//Ankyrin G//0.23:244:60//Hs.75893:U13616
R-HEMBA1001051//Homo sapiens mRNA for KIAA0621 protein, partial cds//6.4e-21:186:79//Hs.132942:AB014521
R-HEMBA1001052//ESTs//5.4e-107:497:99//Hs.121773:AI357886
R-HEMBA1001060//ESTs//1.1e-31:298:80//Hs.24821:AA044813
R-HEMBA1001071//Alpha-1 type 3 collagen//9.1e-34:179:98//Hs.119571:X14420
R-HEMBA1001077//Homo sapiens mRNA, chromosome 1 specific transcript KIAA0492//2.7e-21:417:64//Hs.127338:AB007961
R-HEMBA1001080
R-HEMBA1001085//ESTs//1.9e-47:385:79//Hs.146811:AA410788
R-HEMBA1001088//ESTs//2.8e-102:548:93//Hs.127273:AA522674
R-HEMBA1001094
R-HEMBA1001099//ESTs//0.24:41:97//Hs.18612:T99245
R-HEMBA1001109//Small inducible cytokine A5 (RANTES)//2.4e-46:396:80//Hs.155464:AF088219
R-HEMBA1001121//ESTs//1.7e-15:216:71//Hs.141605:H92974
R-HEMBA1001122//ESTs//2.0e-90:474:94//Hs.107884:AA131320
R-HEMBA1001123//B-CELL GROWTH FACTOR PRECURSOR//2.7e-45:319:84//Hs.99879:M15530
R-HEMBA1001133//ESTs//1.2e-92:443:99//Hs.99626:AA632341
R-HEMBA1001137//ESTs//2.0e-86:426:97//Hs.157103:W60265
R-HEMBA1001140//Small inducible cytokine A5 (RANTES)//2.9e-45:323:83//Hs.155464:AF088219
R-HEMBA1001172//ESTs, Moderately similar to !!!! ALU SUBFAMILY J WARNING ENTRY !!!! [H.sapiens]//1.1e-39:309:82//Hs.96337:AA225358
R-HEMBA1001174//Homo sapiens mRNA, chromosome 1 specific transcript KIAA0492//0.21:238:60//Hs.127338:AB007961
R-HEMBA1001197//ESTs//0.010:388:61//Hs.14881:R91896
R-HEMBA1001208//ESTs, Highly similar to Similar to S.cerevisiae hypothetical protein 5 [H.sapiens]//0.27:305:62//Hs.100238:U69194
R-HEMBA1001226//Homo sapiens PYRIN (MEFV) mRNA, complete cds//5.0e-54:333:81//Hs.113283:AF018080
R-HEMBA1001235//EST//2.3e-07:42:92//Hs.141620:N63316
R-HEMBA1001247//ESTs, Weakly similar to WWP2 [H.sapiens]//2.9e-20:160:87//Hs.103102:W55932
R-HEMBA1001257//ESTs//3.3e-112:544:97//Hs.128749:AA779728
R-HEMBA1001265//ESTs//8.7e-116:564:98//Hs.155150:AI061435
R-nnnnnnnnnnnn//ESTs, Weakly similar to Lpa8p [S.cerevisiae]//2.4e-35:239:87//Hs.103919:AA159181
R-HEMBA1001286//ESTs//1.4e-97:507:95//Hs.26244:AI352674
R-HEMBA1001289//ESTs//8.2e-44:122:96//Hs.76267:AA877534
R-HEMBA1001294//ESTs//1.0:140:65//Hs.149638:AI298324
R-HEMBA1001299//Small inducible cytokine A5 (RANTES)//1.1e-45:307:84//Hs.155464:AF088219
R-HEMBA1001302//Homo sapiens mRNA for APC 2 protein, complete cds//0.53:89:68//Hs.20912:AB012162
R-HEMBA1001303//EST//0.00053:271:60//Hs.156148:AI333214
R-HEMBA1001310//ESTs//1.4e-91:486:93//Hs.86228:AA206019
R-HEMBA1001319//ESTs//0.051:228:61//Hs.99404:AA953977
R-HEMBA1001323//ESTs//6.2e-83:401:98//Hs.47343:AI282950
R-HEMBA1001326//ESTs, Weakly similar to HYPOTHETICAL 55.1 KD PROTEIN IN FAB1-PES4 INTERGENIC REGION [S.cerevisiae]//1.3e-77:458:92//Hs.9398:N41838
R-HEMBA1001327//ESTs//0.60:251:58//Hs.117162:AA701259
R-HEMBA1001330//Homo sapiens PYRIN (MEFV) mRNA, complete cds//1.1e-46:249:78//Hs.113283:AF018080
R-HEMBA1001351//ESTs//0.13:230:57//Hs.138510:R94816
R-HEMBA1001361//ESTs//3.5e-107:570:94//Hs.7727:AA142837
R-HEMBA1001375//ESTs//1.1e-96:454:99//Hs.59584:AA587334
R-HEMBA1001377//ESTs//8.5e-91:459:95//Hs.61859:AA628550
R-HEMBA1001383//ESTs//0.077:381:58//Hs.163093:AA745458
R-HEMBA1001387//ESTs//2.0e-85:405:99//Hs.152127:AI246482
R-HEMBA1001388//ESTs//1.5e-83:395:99//Hs.105191:AA133439
R-HEMBA1001391//ESTs//7.7e-90:455:96//Hs.120905:R22204
R-HEMBA1001398//Thromboxane A2 receptor//4.0e-46:279:89//Hs.89887:D38081
R-HEMBA1001405//ESTs//1.2e-98:485:97//Hs.73287:W16714
R-HEMBA1001407//ESTs//2.2e-76:365:99//Hs.110128:AA584364
R-HEMBA1001411//ESTs//1.2e-102:476:100//Hs.143162:AI380343
R-HEMBA1001413//ESTs//3.7e-66:321:98//Hs.152472:AA041199
R-HEMBA1001415
R-HEMBA1001432//Putative mismatch repair/binding protein hMSH3//7.9e-42:183:82//Hs.42674:U61981
R-HEMBA1001433//ESTs//1.4e-34:240:77//Hs.95611:U51704
R-HEMBA1001435//ESTs//5.6e-23:292:70//Hs.116315:AA629263
R-HEMBA1001442//ESTs//0.76:414:58//Hs.156189:AI419982
R-HEMBA1001446//ESTs//2.2e-95:447:99//Hs.154091:AA767546
R-HEMBA1001450//ESTs//1.0e-93:491:94//Hs.16130:AA195077
R-HEMBA1001454//Human Line-1 repeat mRNA with 2 open reading frames//1.7e-47:304:88//Hs.23094:M19503
R-HEMBA1001455//ESTs//7.1e-103:482:99//Hs.97407:AI417220
R-HEMBA1001463
R-HEMBA1001476//Human mRNA for KIAA0186 gene, complete cds//2.0e-25:409:66//Hs.36232:D80008
R-HEMBA1001478
R-HEMBA1001497
R-HEMBA1001510//ESTs//3.3e-44:381:78//Hs.139882:AA864426
R-HEMBA1001515//Human Line-1 repeat mRNA with 2 open reading frames//5.9e-79:528:84//Hs.23094:M19503
R-HEMBA1001517//ESTs//5.8e-32:272:81//Hs.119512:AA487269
R-HEMBA1001522//ESTs//1.7e-84:364:95//Hs.117858:AA-702493
R-HEMBA1001526//ESTs//1.8e-93:527:93//Hs.10624:N64723
R-HEMBA1001533//ESTs//1.9e-42:211:100//Hs.55830:AA580270
R-HEMBA1001557//ESTs//4.2e-83:413:97//Hs.47546:AA181348
R-HEMBA1001566//Small inducible cytokine A5 (RANTES)//3.4e-50:304:88//Hs.155464:AF088219
R-HEMBA1001569//POU domain, class 3, transcription factor 4//2.3e-06:259:62//Hs.2229:X82324
R-HEMBA1001570//Homo sapiens pendrin (PDS) mRNA, complete cds//3.5e-47:456:77//Hs.159275:AF030880
R-HEMBA1001579//ESTs//0.11:299:60//Hs.106090:AA457030
R-HEMBA1001581//ESTs//0.016:350:61//Hs.124664:AI015652
R-HEMBA1001585//Human mRNA for KIAA0331 gene, complete cds//0.30:251:63//Hs.146395:AB002329
R-HEMBA1001589
R-HEMBA1001595//ESTs, Weakly similar to SEPTIN 2 [D.melanogaster]//6.9e-71:431:88//Hs.26625:W25874
R-HEMBA1001608//Human kpni repeat mrna (cdna clone pcd-kpni-8), 3' end//1.3e-73:533:82//Hs.103948:K00627
R-HEMBA1001620//ESTs, Highly similar to MYO-INOSITOL-1-PHOSPHATE SYNTHASE [Arabidopsis thaliana]//4.5e-93:537:90//Hs.20218:AA628530
R-nnnnnnnnnnnn//Homo sapiens antigen NY-CO-16 mRNA, complete cds//0.054:362:60//Hs.132206:AF039694
R-HEMBA1001636//ESTs//4.9e-53:267:97//Hs.47459:AA700158
R-HEMBA1001640//ESTs//2.9e-27:299:72//Hs.65236:AA927623
R-nnnnnnnnnnnn//ESTs, Weakly similar to Mi-2 protein [H.sapiens]//1.2e-86:442:95//Hs.63888:AA203398
R-HEMBA1001655//ESTs//1.5e-101:516:95//Hs.86541:AA214554
R-HEMBA1001658
R-HEMBA1001661//Homo sapiens protocadherin 68 (PCH68) mRNA, complete cds//1.3e-16:427:61//Hs.106511:AF029343
R-HEMBA1001672//Homo sapiens methyl-CpG binding protein MBD3 (MBD3) mRNA, complete cds//1.4e-93:493:92//Hs.107254:AC005943
R-HEMBA1001675
R-HEMBA1001678//Homo sapiens voltage dependent anion channel protein mRNA, complete cds//4.2e-103:534:94//Hs.7381:AF038962
R-HEMBA1001681//ESTs//6.0e-49:292:92//Hs.65588:AA523424
R-HEMBA1001702//ESTs//9.0e-98:478:97//Hs.28661:AA805916
R-HEMBA1001709//Homo sapiens mRNA for KIAA0698 protein, complete cds//6.3e-98:483:96//Hs.31720:AB014598
R-HEMBA1001711//ESTs//5.8e-83:398:98//Hs.34804:AA514960
R-HEMBA1001712//ESTs//0.028:202:63//Hs.105790:AA528095
R-HEMBA1001714//ESTs, Highly similar to ATPASE INHIBITOR, MITOCHONDRIAL PRECURSOR [Rattus norvegicus]//1.8e-46:236:98//Hs.132948:AA194452
R-HEMBA1001718//Small inducible cytokine A5 (RANTES)//8.6e-43:166:88//Hs.155464:AF088219
R-HEMBA1001723//ESTs, Highly similar to HYPOTHETICAL TRP-ASP REPEATS CONTAINING PROTEIN IN SIS1-MRPL2 INTERGENIC REGION [Saccharomyces cerevisiae]//7.1e-88:431:96//Hs.29203:AI344105
R-HEMBA1001731//EST//0.25:100:68//Hs.149171:AI245712
R-HEMBA1001734//Human mRNA for KIAA0355 gene, complete cds//2.6e-39:366:77//Hs.153014:AB002353
R-HEMBA1001744
R-HEMBA1001745//ESTs//6.6e-05:244:62//Hs.157663:AI358623
R-HEMBA1001746//EST//4.9e-65:409:88//Hs.124673:AA858162
R-HEMBA1001761//ESTs//1.9e-44:315:84//Hs.159510:AA297145
R-HEMBA1001781//ESTs//3.0e-98:462:99//Hs.60059:AI057306
R-HEMBA1001784//EST//1.0e-12:250:68//Hs.152366:AA486721
R-HEMBA1001791//EST//1.4e-47:292:89//Hs.163333:AA879053
R-HEMBA1001800//ESTs//8.4e-37:314:79//Hs.105151:AA970243
R-HEMBA1001803//ESTs//4.5e-99:465:99//Hs.135159:AI095823
R-nnnnnnnnnnnn//Zinc finger protein 148 (pHZ-52)//0.78:232:57//Hs.112180:AF039019
R-HEMBA1001808//Homo sapiens mRNA, chromosome 1 specific transcript KIAA0500//9.0e-114:548:98//Hs.118164:AB007969
R-HEMBA1001809//EST//3.8e-63:292:89//Hs.158591:AI369334
R-HEMBA1001815//Calcium modulating ligand//1.1e-47:299:87//Hs.13572:AF068179
R-HEMBA1001819//ZINC FINGER PROTEIN HF.12//1.2e-16:259:69//Hs.155470:X07290
R-HEMBA1001820//ESTs//2.6e-86:404:100//Hs.112881:AA620707
R-nnnnnnnnnnnn//ESTs//2.2e-101:480:99//Hs.159940:AA971578
R-HEMBA1001824//ESTs, Weakly similar to MATRIN 3 [H.sapiens]//6.2e-27:147:97//Hs.23476:AA401210
R-HEMBA1001835//EST//0.79:216:64//Hs.47437:N52250
R-HEMBA1001844//ESTs//4.7e-62:319:95//Hs.55200:N98513
R-HEMBA1001847//ESTs//2.3e-102:522:95//Hs.20879:AA845446
R-HEMBA1001861//Homo sapiens mRNA for KIAA0617 protein, complete cds//1.1e-109:553:96//Hs.78946:AB014517
R-HEMBA1001864//ESTs//7.4e-94:449:99//Hs.132776:AI142853
R-HEMBA1001866//Myelin oligodendrocyte glycoprotein {alternative products}//1.9e-37:357:76//Hs.53217:Z48051
R-nnnnnnnnnnnn//ESTs, Weakly similar to trithorax homolog HTX, version 2 [H.sapiens]//2.3e-32:193:94//Hs.9489:R84329
R-HEMBA1001888//H.sapiens mRNA for urea transporter//2.0e-47:425:78//Hs.66710:X96969
R-HEMBA1001896//ESTs//3.5e-56:274:99//Hs.129018:H03128
R-HEMBA1001910
R-HEMBA1001912//ESTs, Weakly similar to !!!! ALU SUBFAMILY J WARNING ENTRY !!!! [H.sapiens]//1.5e-73:347:100//Hs.30991:AA994438
R-HEMBA1001913//ESTs, Highly similar to GCN20 PROTEIN [Saccharomyces cerevisiae]//5.1e-57:320:91//Hs.91251:U66685
R-HEMBA1001915//ESTs//4.9e-88:459:95//Hs.122810:AI273706
R-HEMBA1001918//ESTs//1.2e-106:505:99//Hs.98518:AI027125
R-HEMBA1001921//Homo sapiens germinal center kinase related protein kinase mRNA, complete cds//5.5e-107:534:96//Hs.154934:AF000145
R-HEMBA1001939//ESTs, Moderately similar to !!!! ALU SUBFAMILY J WARNING ENTRY !!!! [H.sapiens]//2.9e-99:482:98//Hs.96849:AA879470
R-HEMBA1001940//Human mRNA for KIAA0392 gene, partial cds//5.6e-45:336:82//Hs.40100:AB002390
R-HEMBA1001942//EST//2.6e-84:397:99//Hs.145444:AI203668
R-HEMBA1001945//ESTs//1.4e-92:437:99//Hs.144565:AI192452
R-HEMBA1001950//ESTs//3.9e-43:280:88//Hs.84429:N28866
R-HEMBA1001960//ESTs//0.040:243:62//Hs.29567:AA640421
R-HEMBA1001962//ESTs//0.0071:113:69//Hs.49792:N70048
R-HEMBA1001964//ESTs//3.0e-38:239:87//Hs.158126:W26825
R-HEMBA1001967//Human DNA sequence from clone 341E18 on chromosome 6p11.2-12.3. Contains a Serine/Threonine Protein Kinase gene (presumptive isolog of a Rat gene) and a novel alternatively spliced gene. Contains a putative CpG island, ESTs and GSSs//1.8e-106:517:97//Hs.11050:AL031178
R-HEMBA1001979//EST//0.039:167:63//Hs.129451:AA993932
R-HEMBA1001987//ESTs//3.1e-44:320:83//Hs.136839:H93717
R-HEMBA1001991//Human mRNA for KIAA0355 gene, complete cds//9.5e-47:303:88//Hs.153014:AB002353
R-HEMBA1002003//Homo sapiens mRNA for protein phosphatase 2C (beta)//1.6e-91:448:97//Hs.5687:AJ005801
R-HEMBA1002008//ESTs//9.2e-47:297:87//Hs.142314:AA347930
R-HEMBA1002018//ESTs//9.4e-21:118:97//Hs.7871:AI041837
R-HEMBA1002022//Human mRNA for KIAA0075 gene, partial cds//0.25:196:63//Hs.1189:D38550
R-HEMBA1002035//ESTs//7.7e-101:475:99//Hs.8858:AI131538
R-HEMBA1002039//H.sapiens mRNA for phosphoinositide 3-kinase//0.68:256:64//Hs.101238:Y11312
R-HEMBA1002049//Homo sapiens mRNA for KIAA0563 protein, complete cds//2.4e-51:254:85//Hs.15731:AB011135
R-HEMBA1002084//EST//0.31:219:60//Hs.162396:AA572764
R-HEMBA1002092//EST//6.4e-72:342:99//Hs.148533:AI200996
R-HEMBA1002100//EST//5.6e-38:258:85//Hs.103094:W52354
R-HEMBA1002102//Thiopurine S-methyltransferase//1.4e-46:403:79//Hs.51124:AF019369
R-HEMBA1002113//Prostaglandin 12 (prostacyclin) synthase //1.4e-76:280:90//Hs.61333:D83402
R-HEMBA1002119//Homo sapiens OR7E12P pseudogene, complete sequence//1.4e-87:362:94//Hs.103443:AF065854
R-HEMBA1002125//ESTs, Weakly similar to Y53C12A.3 [C.elegans]//1.7e-16:94:100//Hs.107747:AI357868
R-HEMBA1002139//H.sapiens mRNA for nebulin//0.0019:68:88//Hs.83870:X83957
R-HEMBA1002144//ESTs//3.1e-30:259:72//Hs.141575:AA211734
R-HEMBA1002150//ESTs//7.1e-105:543:95//Hs.32275:AA595199
R-HEMBA1002151//ESTs//2.2e-35:178:100//Hs.77703:W19642
R-HEMBA1002153//EST//4.5e-49:458:77//Hs.141708:W44337
R-HEMBA1002160//Homo sapiens nephrocystin (NPHP1) mRNA, partial cds//1.4e-36:400:75//Hs.75474:AF023674
R-HEMBA1002161//Homo sapiens EVI5 homolog mRNA, complete cds//1.9e-33:294:77//Hs.26929:AF008915
R-HEMBA1002162//ESTs//1.0e-47:317:85//Hs.48919:N64043
R-HEMBA1002166//Thromboxane A2 receptor//6.8e-46:296:81//Hs.89887:D38081
R-HEMBA1002177//EST//2.6e-42:215:99//Hs.116880:AA662457
R-HEMBA1002185//Homo sapiens class-I MHC-restricted T cell associated molecule (CRTAM) mRNA, complete cds//6.0e-42:419:73//Hs.159523:AF001622
R-HEMBA1002189//Homo sapiens mRNA for KIAA0792 protein, complete cds//1.4e-29:244:72//Hs.119387:AB007958
R-HEMBA1002191//ESTs//2.6e-31:275:66//Hs.133852:AI076357
R-HEMBA1002199//Human Line-1 repeat mRNA with 2 open reading frames//4.3e-84:557:84//Hs.23094:M19503
R-HEMBA1002204//EST//0.00057:113:71//Hs.144868:AI202342
R-HEMBA1002212//ESTs//1.5e-48:277:93//Hs.104741:AI393315
R-HEMBA1002215//ESTs//1.1e-23:158:90//Hs.152529:AA897151
R-HEMBA1002226//Homo sapiens mRNA for KIAA0706 protein, complete cds//5.1e-21:230:75//Hs.139648:AB014606
R-HEMBA1002229//Homo sapiens growth suppressor related (DOC-1R) mRNA, complete cds//1.5e-47:238:98//Hs.25664:AF089814
R-HEMBA1002237//ESTs//6.9e-35:357:76//Hs.116518:AA653202
R-HEMBA1002253//EST//6.0e-19:125:81//Hs.140596:AA829426
R-HEMBA1002257
R-HEMBA1002267//ESTs, Weakly similar to HYPOTHETICAL 27.8 KD PROTEIN IN VMA7-RPS31A INTERGENIC REGION [S.cerevisiae]//1.3e-31:201:91//Hs.114673:W72675
R-HEMBA1002270//ESTs//4.6e-100:483:97//Hs.34940:AI264314
R-HEMBA1002321//ESTs//2.3e-85:403:99//Hs.120388:AA723595
R-HEMBA1002328//ESTs//1.3e-90:423:100//Hs.117936:AI280818
R-HEMBA1002337//ESTs//8.7e-24:147:93//Hs.9893:AA007679
R-HEMBA1002341//Homo sapiens mRNA for KIAA0771 protein, partial cds//7.8e-130:642:96//Hs.6162:AB018314
R-HEMBA1002348//ESTs//5.0e-71:387:93//Hs.30494:H04822
R-HEMBA1002349//ESTs//9.7e-88:420:98//Hs.132972:AA543094
R-nnnnnnnnnnnn//Homo sapiens chromosome-associated protein-E (hCAP-E) mRNA, complete cds//3.9e-123:661:93//Hs.119023:AF092563
R-HEMBA1002381//ESTs//1.3e-73:352:99//Hs.56121:AA781435
R-HEMBA1002389//EST//2.3e-05:132:69//Hs.37558:H58237
R-HEMBA1002417//Homo sapiens chromosome 19, cosmid R28784//3.9e-63:358:91//Hs.25527:AC005954
R-HEMBA1002419//ESTs, Weakly similar to APK1 antigen [H.sapiens)//5.6e-87:429:96//Hs.13209:AI417849
R-HEMBA1002430//ESTs//0.10:388:57//Hs.119238AA476267
R-HEMBA1002439//Human mRNA for KIAA0080 gene, partial cds//2.0e-22:181:80//Hs.74554:D38522
R-HEMBA1002458//ESTs//1.8e-88:448:95//Hs.97914:AA769069
R-HEMBA1002460//Catalase//0.67:314:60//Hs.76359:X04085
R-HEMBA1002462//EST//0.032:44:88//Hs.161536:N80395
R-nnnnnnnnnnnn//ESTs, Weakly similar to F08G12.1 [C.elegans]//5.4e-95:488:95//Hs.108115:AA582193
R-HEMBA1002477//Homo sapiens KIAA0395 mRNA, partial cds//2.5e-37:281:80//Hs.43681:AL022394
R-HEMBA-1002486//Small inducible cytokine A5 (RANTES)//1.1e-49:311:88//Hs.155464:AF088219
R-HEMBA1002495//ESTs//1.2e-94:457:98//Hs.42140:AI188995
R-HEMBA1002498//ESTs//1.7e-35:240:78//Hs.119871:AA705133
R-HEMBA1002503//ESTs//2.3e-14:64:85//Hs.140190:AA701449
R-HEMBA1002508//ESTs//0.00057:160:62//Hs.149661:AA872990
R-nnnnnnnnnnnn//Homo sapiens mRNA for histone deacetylase-like protein (JM21)//2.3e-113:456:92//Hs.6764:AJ011972
R-HEMBA1002515//EST//1.0:153:63//Hs.118045:N51715
R-HEMBA1002538//Homo sapiens mRNA for KIAA0454 protein, partial cds//5.1e-106:564:93//Hs.129928:AB007923
R-HEMBA1002542//ESTs//1.0e-101:539:93//Hs.93872:AA524700
R-HEMBA1002547//EST//8.7e-27:151:96//Hs.132145:AI041804
R-HEMBA1002552//EST//5.9e-49:335:85//Hs.149580:AI281881
R-HEMBA1002555//ESTs//1.1e-77:461:91//Hs.38750:N30012
R-HEMBA1002558//Homo sapiens 4F5S mRNA, complete cds//1.3e-42:264:89//Hs.32567:AF073519
R-HEMBA1002561//Small inducible cytokine A5 (RANTES)//6.4e-40:196:78//Hs.155464:AF088219
R-nnnnnnnnnnnn//Homo sapiens protein associated with Myc mRNA, complete cds//1.4e-120:587:97//Hs.151411:AF075587
R-HEMBA1002583//ESTs//7.1e-79:410:95//Hs.21599:AA478904
R-HEMBA1002590//EST//3.3e-54:278:97//Hs.138637:N20838
R-HEMBA1002592//ESTs//2.6e-44:500:74//Hs.110934:N26055
R-HEMBA1002621
R-HEMBA1002624//Homo sapiens mRNA for KIAA0808 protein, complete cds//2.2e-77:380:97//Hs.91338:AB018351
R-HEMBA1002628//ESTs//0.0020:167:66//Hs.140605:AA830881
R-HEMBA1002629//ESTs//0.00014:50:100//Hs.119132:AA398715
R-HEMBA1002645//EST//2.1e-37:285:82//Hs.141728:W73041
R-HEMBA1002651//EST//2.2e-23:374:69//Hs.139357:AA420970
R-HEMBA1002659//Human 53K isoform of Type II phosphatidylinositol-4-phosphate 5-kinase (PIPK) mRNA, complete cds//1.5e-53:406:81//Hs.108966:U48696
R-HEMBA1002661//Homo sapiens mRNA for KIAA0764 protein, complete cds//1.1e-41:296:84//Hs.6232:AB018307
R-HEMBA1002666//EST//4.4e-09:79:88//Hs.72015:AA151945
R-HEMBA1002678//EST, Moderately similar to !!!! ALU SUBFAMILY J WARNING ENTRY !!!! [H.sapiens]//7.6e-104:560:92//Hs.161748:T64896
R-nnnnnnnnnnnn//EST//0.15:136:69//Hs.129570:AA995396
R-HEMBA1002688//T-CELL SURFACE PROTEIN TACTILE PRECURSOR//0.16:247:62//Hs.142023:M88282
R-HEMBA1002696//ESTs//3.5e-94:529:92//Hs.16725:AA196477
R-HEMBA1002712//Homo sapiens mRNA for KIAA0772 protein, complete cds//6.0e-46:302:86//Hs.15519:AB018315
R-HEMBA1002716//ESTs//1.3e-109:555:96//Hs.9812:AA147884
R-HEMBA1002728//Homo sapiens mRNA for KIAA0621 protein, partial cds//3.8e-37:287:81//Hs.132942:AB014521
R-HEMBA1002730//ESTs//1.2e-95:488:95//Hs.22030:AA521168
R-HEMBA1002742//ESTs//1.0e-91:437:99//Hs.139987:AA652163
R-HEMBA1002746//ESTs//4.4e-97:468:98//Hs.129903:AA576526
R-HEMBA1002748//ESTs//5.0e-98:475:98//Hs.125461:AI375792
R-HEMBA1002750//ESTs//1.6e-42:223:97//Hs.40460:N36090
R-HEMBA1002768//Homo sapiens mRNA for KIAA0554 protein, partial cds//4.0e-106:545:95//Hs.74750:AB011126
R-HEMBA1002770//EST//0.34:294:59//Hs.43091:N22127
R-HEMBA1002777//ESTs//3.0e-85:316:98//Hs.17537:C06491
R-HEMBA1002779//Human mRNA for KIAA0013 gene, complete cds//0.25:342:58//Hs.48824:D87717
R-HEMBA1002780//Homo sapiens DEC-205 mRNA, complete cds//4.2e-46:449:75//Hs.153563:AF011333
R-HEMBA1002794//ESTs//1.2e-115:559:97//Hs.79741:AI279709
R-HEMBA1002801//EST//0.00049:287:60//Hs.126466:AA913320
R-HEMBA1002810//Homo sapiens formin binding protein 21 mRNA, complete cds//1.4e-116:559:97//Hs.28307:AF071185
R-HEMBA1002816//Human plectin (PLEC1) mRNA, complete cds//0.28:281:62//Hs.79706:U53204
R-HEMBA1002826//EST//6.7e-25:134:99//Hs.134683:AI092013
R-HEMBA1002833//ESTs, Highly similar to ribosome-binding protein p34 [R.norvegicus]//4.3e-25:137:98//Hs.5337:AA243757
R-HEMBA1002850//ESTs//0.010:323:57//Hs.18282:W67514
R-HEMBA1002863//ESTs//1.1e-67:359:94//Hs.124699:W27830
R-HEMBA1002876//ESTs//0.72:202:62//Hs.144816:AI220827
R-HEMBA1002886//EST//3.2e-85:401:99//Hs.96580:AA405670
R-HEMBA1002896//Homo sapiens SH3-containing adaptor molecule-1 mRNA, complete cds//1.2e-107:541:95//Hs.33787:AF037261
R-HEMBA1002921//Human mRNA for KIAA0189 gene, complete cds//0.84:103:71//Hs.95140:D80011
R-HEMBA1002924//ESTs//3.5e-86:423:98//Hs.27513:N34820
R-HEMBA1002934//Human mRNA for KIAA0118 gene, partial cds//2.1e-50:308:88//Hs.154326:D42087
R-HEMBA1002935//ESTs//1.0e-73:384:95//Hs.118193:N74481
R-HEMBA1002937//ESTs//0.052:167:65//Hs.145504:AI254165
R-HEMBA1002939//ESTs//1.6e-94:467:97//Hs.9893:AA007679
R-HEMBA1002944//ESTs//2.7e-17:176:80//Hs.143768:AA229732
R-HEMBA1002951//ESTs//3.7e-119:565:98//Hs.16218:AI190892
R-HEMBA1002954//EST//0.076:285:58//Hs.98706:AA431085
R-HEMBA1002968//Thiopurine S-methyltransferase//1.9e-46:314:85//Hs.51124:AF019369
R-HEMBA1002970//EST//0.00050:164:64//Hs.129630:AI000405
R-HEMBA1002971//Homo sapiens mRNA for KIAA0679 protein, partial cds//2.3e-30:162:99//Hs.5734:AB014579
R-HEMBA1002973//Small inducible cytokine A5 (RANTES)//5.7e-42:318:81//Hs.155464:AF088219
R-nnnnnnnnnnnn//ESTs//3.2e-18:102:100//Hs.146255:AA197064
R-HEMBA1002999//ESTs, Moderately similar to lamina associated polypeptide 1C [R.norvegicus]//7.9e-113:560:96//Hs.125749:AI377682
R-HEMBA1003021//Homo sapiens PYRIN (MEFV) mRNA, complete cds//3.3e-42:290:85//Hs. 113283:AF018080
R-HEMBA1003033//ESTs//2.8e-77:417:94//Hs.138860:W47480
R-HEMBA1003034//ESTs//3.7e-42:429:74//Hs.132818:AI038577
R-HEMBA1003035//ESTs//0.025:156:64//Hs.8473:T40827
R-HEMBA1003037//ESTs//0.69:381:57//Hs.47312:AI240366
R-HEMBA1003041//ESTs, Highly similar to PUTATIVE SERINE/THREONINE-PROTEIN KINASE C41C4.4 IN CHROMOSOME II PRECURSOR [Caenorhabditis elegans]//5.6e-34:280:79//Hs.114905:AA088442
R-HEMBA1003046//Homo sapiens mitochondrial processing peptidase beta-subunit mRNA, complete cds//1.3e-119:578:97//Hs.44097:AF054182
R-HEMBA1003064//ESTs//7.8e-85:419:96//Hs.87020:AA706627
R-HEMBA1003067//Von Hippel-Lindau syndrome//2.0e-30:299:75//Hs.78160:AF010238
R-HEMBA1003071//ESTs//2.3e-74:360:98//Hs.17270:AA701903
R-HEMBA1003077//ESTs, Weakly similar to KIAA0405 [H.sapiens]//1.1e-90:434:99//Hs.14146:W92235
R-HEMBA1003078//ESTs//5.9e-16:156:77//Hs.142684:AA902402
R-HEMBA1003079//ESTs//0.16:341:58//Hs.95923:AI075249
R-HEMBA1003083//Small inducible cytokine A5 (RANTES)//1.9e-39:284:83//Hs.155464:AF088219
R-HEMBA1003086//EST//1.0e-48:372:82//Hs.161917:AA483223
R-HEMBA1003096//ESTs, Weakly similar to Mouse 19.5 mRNA, complete cds [M.musculus]//4.2e-100:531:94//Hs.104800:AA709155
R-HEMBA1003098//ESTs//4.2e-107:537:96//Hs.107213:AA121624
R-HEMBA1003117//ESTs//2.4e-67:331:97//Hs.157158:AI150058
R-HEMBA1003129//Human nucleolar fibrillar center protein (ASE-1) mRNA, complete cds//2.1e-13:109:88//Hs.118717:U86751
R-HEMBA1003133//ESTs//1.1e-34:180:98//Hs.159387:AI370845
R-HEMBA1003136//ESTs, Weakly similar to MANNOSE-1-PHOSPHATE GUANYLTRANSFERASE [Saccharomyces cerevisiae]//9.2e-114:577:95//Hs.27059:AI088615
R-HEMBA1003142//Small inducible cytokine A5 (RANTES)//1.1e-45:285:88//Hs.155464:AF088219
R-HEMBA1003148//Homo sapiens mRNA for dachshund protein//3.6e-118:586:96//Hs.63931:AJ005670
R-HEMBA1003166//ESTs//1.6e-96:479:96//Hs.119940:AA705933
R-HEMBA1003175//ESTs//2.7e-74:407:92//Hs.139167:AA715389
R-HEMBA1003197//ESTs//1.6e-68:384:94//Hs.120969:W92000
R-HEMBA1003199//Sjogren syndrome antigen B (autoantigen La)//0.19:328:57//Hs.83715:X69804
R-HEMBA1003202//Homo sapiens mRNA for KIAA0640 protein, partial cds//1.3e-40:290:83//Hs.153026:AB014540
R-HEMBA1003204//ESTs//1.1e-34:215:91//Hs.108090:AA424943
R-HEMBA1003212//ESTs//1.9e-81:441:93//Hs.28471:W20265
R-HEMBA1003220//ESTs, Weakly similar to MITOCHONDRIAL 40S RIBOSOMAL PROTEIN S28 PRECURSOR [S.cerevisiae]//1.6e-40:232:93//Hs.107707:N32817
R-HEMBA10032227/ESTs, Weakly similar to weak similarity to HSP90 [C.elegans]//1.1e-42:310:85//Hs.23294:W27666
R-HEMBA1003229//ESTs//4.8e-18:133:90//Hs.61763:AA035305
R-HEMBA1003235//ESTs//7.7e-35:201:78//Hs.163979:AA828834
R-HEMBA1003250//Homo sapiens p21-activated kinase 3 (PAK3) mRNA, complete cds//7.4e-05:534:58//Hs.152663:AF068864
R-HEMBA1003257//EST//1.4e-95:473:97//Hs.32443:H28929
R-HEMBA1003273//Small inducible cytokine A5 (RANTES)//2.6e-38:253:86//Hs.155464:AF088219
R-HEMBA1003276//ESTs//7.6e-55:269:99//Hs.23817:AA526392
R-HEMBA1003278//ESTs//2.6e-45:301:71//Hs.51652:AI084785
R-HEMBA1003281
R-HEMBA1003291//Homo sapiens mRNA for KIAA0537 protein, complete cds//9.7e-117:551:99//Hs.12836:AB011109
R-HEMBA1003296//ESTs//4.8e-17:210:72//Hs.44451:AA203266
R-HEMBA1003304//ESTs//2.8e-98:468:98//Hs.120849:AI148353
R-HEMBA1003309//ESTs//1.8e-97:455:99//Hs.11571:AA713504
R-HEMBA1003314//Homo sapiens mRNA for leucine zipper bearing kinase, complete cds//8.9e-113:545:97//Hs.124224:AB001872
R-HEMBA1003322//ESTs//4.9e-79:419:95//Hs.138760:N66869
R-HEMBA1003327//Homo sapiens clone 23622 mRNA sequence//1.4e-16:177:78//Hs.151608:AF052119
R-HEMBA1003328//H.sapiens mRNA for MACH-alpha-2 protein//2.1e-43:269:88//Hs.19949:X98173
R-HEMBA1003330//Homo sapiens poly(A) binding protein II (PABP2) gene, complete cds//0.66:64:76//Hs.117176:AF026029
R-HEMBA1003348//ESTs//1.4e-35:185:78//Hs.117879:H77357
R-HEMBA1003369//ESTs, Weakly similar to F59C6.9 [C.elegans]//3.2e-113:553:97//Hs.65539:AI148540
R-HEMBA1003370//ESTs//2.0e-46:319:86//Hs.37573:H59651
R-HEMBA1003373//ESTs//1.6e-31:136:81//Hs.114849:AI139588
R-HEMBA1003376//ESTs//3.0e-47:383:80//Hs.138852:AA284247
R-HEMBA1003380//ESTs, Moderately similar to !!!! ALU SUBFAMILY J WARNING ENTRY !!!! [H.sapiens]//1.8e-11:261:65//Hs.87578:AI125363
R-HEMBA1003384//EST//0.00013:82:75//Hs.141237:H57847
R-HEMBA1003395//ESTs//5.2e-78:379:98//Hs.162208:AA536127
R-HEMBA1003402//ESTs//8.6e-14:108:89//Hs.55424:AA774204
R-nnnnnnnnnnnn//ESTs//1.7e-24:188:85//Hs.70266:Z78309
R-HEMBA1003417//ESTs//4.2e-74:396:94//Hs.55220:D11563
R-HEMBA1003418//ESTs//3.1e-107:545:95//Hs.3494:AI421013
R-HEMBA1003433//Homo sapiens nibrin (NBS) mRNA, complete cds//3.2e-115:544:98//Hs.25812:AF058696
R-HEMBA1003461//ESTs//2.8e-62:304:99//Hs.148747:AI225121
R-HEMBA1003463//ESTs//2.3e-112:549:97//Hs.104627:AA885516
R-HEMBA1003480//Homo sapiens PYRIN (MEFV) mRNA, complete cds//7.7e-76:529:84//Hs.113283:AF018080
R-HEMBA1003528//ESTs//2.1e-59:312:96//Hs.22505:R41688
R-HEMBA1003531//ESTs//2.2e-17:116:93//Hs.140217:AA702760
R-HEMBA1003538//Complement component C1r//4.7e-25:333:68//Hs.1279:M14058
R-HEMBA1003545//ESTs//8.7e-89:432:98//Hs.99497:AA776817
R-HEMBA1003548//EST//0.0091:274:60//Hs.148336:AA911673
R-HEMBA1003555//ESTs, Weakly similar to NUCLEOTIDE-BINDING PROTEIN [H.sapiens]//2.8e-93:495:93//Hs.91619:AA552351
R-HEMBA1003556//ESTs//7.1e-44:406:77//Hs.141575:AA211734
R-HEMBA1003560//ESTs//4.0e-34:182:97//Hs.14811:AA434522
R-HEMBA1003568//ESTs//2.0e-101:486:98//Hs.118570:AI342058
R-HEMBA1003569//ESTs, Moderately similar to metastasis-associated gene [H.sapiens]//4.0e-63:343:93//Hs.58598:AA625440
R-HEMBA1003571//Homo sapiens clone 23632 mRNA sequence//3.7e-47:338:84//Hs.46918:AF052099
R-HEMBA1003579//EST//0.00057:239:60//Hs.162828:AA643892
R-HEMBA1003581//ESTs//2.6e-10:118:79//Hs.44856:N37065
R-HEMBA1003591//ESTs//2.4e-96:460:98//Hs.128741:AI244212
R-HEMBA1003595//Human mRNA for KIAA0118 gene, partial cds//1.7e-48:421:78//Hs.154326:D42087
R-HEMBA1003597//EST//1.6e-38:313:80//Hs.160911:AI371042
R-HEMBA1003598//ESTs//0.0085:273:61//Hs.145333:AI251374
R-HEMBA1003615
R-HEMBA1003617//ESTs//1.0e-111:574:95//Hs.4552:W68167
R-HEMBA1003621//EST//1.7e-31:288:78//Hs.140909:R49387
R-HEMBA1003622//EST//1.1e-46:468:75//Hs.139093:AA166888
R-HEMBA1003630//ESTs//1.4e-21:411:69//Hs.128729:AA973021
R-HEMBA1003637//ESTs, Weakly similar to !!!! ALU SUBFAMILY SB WARNING ENTRY !!!! [H.sapiens]//9.3e-24:189:84//Hs.142208:AA209438
R-HEMBA1003640//ISLET AMYLOID POLYPEPTIDE PRECURSOR//2.5e-42:332:81//Hs.51048:X68830
R-HEMBA1003645//ESTs//2.4e-77:423:94//Hs.99539:R59010
R-HEMBA1003646//ESTs//2.6e-98:549:91//Hs.96427:AA151783
R-HEMBA1003656//Homo sapiens mRNA, chromosome 1 specific transcript KIAA0488//5.6e-44:245:77//Hs.67619:AB007957
R-HEMBA1003662//Human TBX2 (TXB2) mRNA, complete cds//2.6e-17:144:84//Hs.32931:U28049
R-HEMBA1003667//Farnesyltransferase, CAAX box, beta//1.3e-22:170:88//Hs.117596:L00635
R-HEMBA1003679//ESTs, Weakly similar to trithorax homolog HTX, version 2 [H.sapiens]//4.1e-87:434:97//Hs.9489:R84329
R-HEMBA1003680//Human DNA-binding protein (HRC1) mRNA, complete cds//0.86:315:61//Hs.72925:M91083
R-HEMBA1003684//ESTs, Highly similar to ZINC FINGER PROTEIN 7 [Homo sapiens]//1.1e-101:528:95//Hs.22934:AA581379
R-HEMBA1003690//ESTs//0.0021:119:69//Hs.98641:AA429916
R-HEMBA1003692//Human cytochrome P450-IIB (hIIB3) mRNA, complete cds//2.0e-43:360:80//Hs.110194:M29873
R-HEMBA1003711//ESTs//1.0e-70:375:94//Hs.150407:AI279064
R-HEMBA1003714//VASOACTIVE INTESTINAL POLYPEPTIDE RECEPTOR 1 PRECURSOR//0.94:367:62//Hs.1139:X77777
R-HEMBA1003715//Homo sapiens PYRIN (MEFV) mRNA, complete cds//5.1e-77:299:85//Hs.113283:AF018080
R-HEMBA1003720//Homo sapiens TWIK-related acid-sensitive K+ channel (TASK) mRNA, complete cds//1.2e-33:377:74//Hs.24040:AF006823
R-HEMBA1003725//ESTs//3.8e-103:481:99//Hs.122518:AA778847
R-HEMBA1003729//ESTs//2.5e-51:277:95//Hs.26270:AA258839
R-HEMBA1003733//ESTs//1.9e-69:350:96//Hs.139278:AA702592
R-HEMBA1003742//ESTs, Moderately similar to T13H5.2 [C.elegans]//4.6e-70:348:96//Hs.11282:AI147040
R-HEMBA1003758//ESTs//1.7e-52:306:85//Hs.138852:AA284247
R-HEMBA1003760//ESTs//7.4e-76:420:93//Hs.26501:H05089
R-HEMBA1003773//ESTs, Highly similar to SIGNAL RECOGNITION PARTICLE RECEPTOR BETA SUBUNIT [Mus musculus]//1.9e-77:364:100//Hs.12152:AA156214
R-HEMBA1003783//ESTs, Weakly similar to C01H6.7 [C.elegans]//2.1e-101:558:93//Hs.18171:AA524327
R-HEMBA1003784//EST//0.83:127:62//Hs.144002:F01600
R-HEMBA1003799//EST//9.7e-30:362:71//Hs.156577:AA860236
R-HEMBA1003803//ESTs, Weakly similar to Y53C12A.3 [C.elegans]//2.8e-16:93:100//Hs.107747:AI357868
R-HEMBA1003804//Interleukin 15//0.13:227:62//Hs.111867:AB007295
R-HEMBA1003805//ESTs//0.029:199:65//Hs.91582:T25344
R-HEMBA1003807//EST//2.4e-13:137:81//Hs.145645:AI264163
R-HEMBA1003836//Small inducible cytokine A5 (RANTES)//3.2e-39:284:83//Hs.155464:AF088219
R-HEMBA1003838//ESTs, Weakly similar to NADH-UBIQUINONE OXIDOREDUCTASE CHAIN 2 [Paramecium tetraurelia)//6.5e-71:357:96//Hs.107573:AA524333
R-HEMBA1003856//ESTs//8.2e-20:266:71//Hs.48312:N68161
R-HEMBA1003864//ESTs//1.6e-99:528:93//Hs.26890:AA449033
R-HEMBA1003866//POLYPOSIS LOCUS PROTEIN 1//0.30:146:64//Hs.74648:M73547
R-HEMBA1003879//EST, Weakly similar to DNA-REPAIR PROTEIN COMPLEMENTING XP-A CELLS [Homo sapiens]//2.1e-59:295:98//Hs.161661:AA166911
R-HEMBA1003880//Homo sapiens clone 24760 mRNA sequence//3.8e-34:286:79//Hs.61408:AF070621
R-HEMBA1003885//ESTs//4.6e-50:293:90//Hs.142314:AA347930
R-HEMBA1003893//Calcium modulating ligand//2.1e-43:294:86//Hs.13572:AF068179
R-HEMBA1003902//ESTs//1.8e-43:300:85//Hs.146811:AA410788
R-HEMBA1003908//ESTs//3.5e-91:477:94//Hs.6638:AA536187
R-HEMBA1003926//ESTs//7.9e-44:294:87//Hs.164036:AA845659
R-HEMBA1003937//Homo sapiens mRNA for KIAA0585 protein, partial cds//3.5e-48:276:81//Hs.72660:AB011157
R-HEMBA1003939
R-HEMBA1003942//ESTs//1.6e-81:428:94//Hs.50418:AA524669
R-HEMBA1003950//ESTs//8.1e-54:283:95//Hs.145528:AI261545
R-HEMBA1003953//ESTs//3.8e-30:194:89//Hs.99681:AA504591
R-HEMBA1003958//ESTs//4.0e-45:394:77//Hs.141602:N63562
R-HEMBA1003959//ESTs//5.2e-28:197:86//Hs.9951:W56253
R-HEMBA1003976//ESTs//2.0e-29:232:84//Hs.133947:AI074525
R-HEMBA1003978//ESTs//3.2e-115:549:98//Hs.76798:AI050882
R-HEMBA1003985//ESTs, Weakly similar to !!!! ALU SUBFAMILY J WARNING ENTRY !!!! [H.sapiens]//2.2e-91:448:97//Hs.117834:AA766771
R-HEMBA1003987//ESTs//8.1e-36:193:88//Hs.151844:N92756
R-HEMBA1003989//Human mRNA for KIAA0241 gene, partial cds//3.6e-43:360:81//Hs.150275:D87682
R-HEMBA1004000//EST//5.5e-62:308:97//Hs.50438:N74105
R-HEMBA1004011//ESTs//8.6e-85:431:96//Hs.36185:R99899
R-HEMBA1004012//ESTs//1.3e-40:309:83//Hs.140329:AA714011
R-HEMBA1004015//ESTs//5.1e-97:453:99//Hs.111446:AI333774
R-HEMBA1004024//ESTs//5.2e-19:159:79//Hs.138856:H47461
R-HEMBA1004038//ESTs//1.3e-41:346:79//Hs.146173:AA906191
R-HEMBA1004042//ESTs//0.0012:201:69//Hs.24248:AA528253
R-HEMBA1004045//ESTs, Weakly similar to putative p150 [H.sapiens]//1.5e-22:365:70//Hs.99692:AA811804
R-HEMBA1004048//ESTs//9.5e-104:497:98//Hs.77735:AI125469
R-HEMBA1004049//HEAT SHOCK 70 KD PROTEIN 1//6.3e-31:176:96//Hs.8997:M11717
R-HEMBA1004055//ESTs//1.7e-115:577:96//Hs.59503:W63754
R-HEMBA1004056//Homo sapiens PYRIN (MEFV) mRNA, complete cds//5.2e-78:577:82//Hs.113283:AF018080
R-HEMBA1004074//EST//1.0:152:61//Hs.149093:AI243988
R-HEMBA1004086//ESTs//4.0e-53:266:98//Hs.34658:N98652
R-HEMBA1004097//ESTs//4.4e-46:279:91//Hs.110533:H16251
R-HEMBA1004131//Human mRNA for KIAA0128 gene, partial cds//3.0e-43:534:69//Hs.90998:D50918
R-HEMBA1004132//ESTs//4.6e-47:316:86//Hs.141602:N63562
R-HEMBA1004133
R-HEMBA1004138//EST//1.7e-08:211:64//Hs.129189:AA988736
R-HEMBA1004143//ESTs//4.0e-25:137:97//Hs.21307:AA203320
R-HEMBA1004146//Small inducible cytokine A5 (RANTES)//4.1e-27:191:86//Hs.155464:AF088219
R-HEMBA1004150//GRANCALCIN//0.99:357:59//Hs.79381:M81637
R-HEMBA1004164//Human mRNA for KIAA0118 gene, partial cds//9.5e-47:313:84//Hs.154326:D42087
R-HEMBA1004168//Homo sapiens geminin mRNA, complete cds//7.7e-112:563:96//Hs.59988:AF067855
R-HEMBA1004199
R-HEMBA1004200//EST//3.1e-89:441:97//Hs.141173:R97701
R-HEMBA1004202//ESTs, Weakly similar to GTP-BINDING PROTEIN YPTM1 [Zea mays]//1.7e-107:552:94//Hs.10092:AI189282
R-HEMBA1004203//Homo sapiens mRNA for KIAA0618 protein, complete cds//1.5e-96:275:98//Hs.15832:AB014518
R-HEMBA1004207//Leptin receptor//1.1e-117:573:97//Hs.54515:U50748
R-HEMBA1004225//EST//9.7e-34:186:95//Hs.137567:R20617
R-HEMBA1004227//ESTs, Moderately similar to !!!! ALU SUBFAMILY SQ WARNING ENTRY !!!! [H.sapiens]//4.0e-16:117:91//Hs.92033:AA255832
R-HEMBA1004238//Human mRNA for KIAA0355 gene, complete cds//3.0e-46:338:83//Hs.153014:AB002353
R-HEMBA1004241//ESTs//1.3e-10:93:87//Hs.137511:AA456389
R-HEMBA1004246//Homo sapiens LIM protein mRNA, complete cds//2.7e-43:511:72//Hs.154103:AF061258
R-HEMBA1004248//ESTs, Highly similar to INSULIN-INDUCED GROWTH RESPONSE PROTEIN CL-6 [Rattus norvegicus]//2.1e-61:221:86//Hs.7089:W37284
R-HEMBA1004264//ESTs//1.5e-80:425:95//Hs.107206:AA234962
R-HEMBA1004267//ESTs, Moderately similar to !!!! ALU SUBFAMILY SP WARNING ENTRY !!!! [H.sapiens]//1.4e-89:465:95//Hs.113660:D20018
R-HEMBA1004272//ESTs//4.5e-111:577:94//Hs.115696:N57931
R-nnnnnnnnnnnn//Homo sapiens clone 617 unknown mRNA, complete sequence//1.4e-111:553:96//Hs.93677:AF091081
R-HEMBA1004276//ESTs, Highly similar to BETA-ADAPTIN [Homo sapiens; Rattus norvegicus; Bos taurus]//4.4e-92:559:89//Hs.28298:AA203228
R-HEMBA1004286//Homo sapiens TGF beta receptor associated protein-1 mRNA, complete cds//6.2e-108:538:97//Hs.101766:AF022795
R-HEMBA1004289//Sulfotransferase, dehydroepiandrosterone (DHEA) -preferring//1.7e-34:223:75//Hs.81884:U13061
R-HEMBA1004295//ESTs, Weakly similar to weakly similar to ANK repeat region of Fowlpox virus BamHI-orf7 protein [C.elegans]//3.6e-93:496:94//Hs.14337:AA534961
R-HEMBA1004306//ESTs//3.4e-26:363:68//Hs.70279:AA757426
R-HEMBA1004312//ESTs//4.8e-64:351:94//Hs.138611:H82679
R-HEMBA1004321//Zinc finger protein 44 (KOX 7)//2.6e-37:415:64//Hs.51199:X16281
R-HEMBA1004323//ESTs//2.1e-40:280:70//Hs.153300:AA928904
R-HEMBA1004327//ESTs//3.8e-72:343:99//Hs.151708:AA554714
R-HEMBA1004330//ESTs//4.0e-52:270:97//Hs.24654:AA456561
R-HEMBA1004334//ESTs//1.6e-46:234:98//Hs.47159:AI310231
R-HEMBA1004335//ESTs//1.9e-25:250:76//Hs.155880:AA703336
R-HEMBA1004341//ESTs//3.7e-101:480:98//Hs.69321:AA633240
R-HEMBA1004353//Homo sapiens mRNA for c-myc binding protein, complete cds//1.3e-75:444:90//Hs.80686:D89667
R-HEMBA1004354//Human mRNA for KIAA0355 gene, complete cds//5.9e-39:286:83//Hs.153014:AB002353
R-HEMBA1004356//SINGLE-STRANDED DNA-BINDING PROTEIN MSSP-1//1.3e-107:576:93//Hs.55458:X77494
R-HEMBA1004366//ESTs//2.3e-94:524:91//Hs.111496:AA652869
R-HEMBA1004372//EST//0.27:198:60//Hs.162665:AA605057
R-HEMBA1004389//ESTs//4.1e-102:490:98//Hs.153708:AA687264
R-HEMBA1004394//ESTs//1.5e-94:471:96//Hs.151647:AA002084
R-HEMBA1004396//Small inducible cytokine A5 (RANTES)//6.2e-41:285:83//Hs.155464:AF088219
R-HEMBA1004405//ESTs//2.0e-44:329:83//Hs.136839:H93717
R-HEMBA1004408//ESTs, Weakly similar to homologous to mouse Rsu-1 [H.sapiens]//6.1e-89:420:99//Hs.88365:AA648933
R-HEMBA1004429//ESTs, Weakly similar to homeotic protein protein zhx-1 [M.musculus]//3.0e-112:552:96//Hs.12940:AI123518
R-HEMBA1004433//Human Line-1 repeat mRNA with 2 open reading frames//2.9e-32:463:68//Hs.23094:M19503
R-HEMBA1004460//ESTs//2.0e-104:574:93//Hs.46848:AA195829
R-HEMBA1004461//ESTs//2.9e-102:503:98//Hs.16370:AA017033
R-HEMBA1004479//ELK1, member of ETS oncogene family//1.1e-45:310:75//Hs.116549:AL009172
R-HEMBA1004482//ESTs//9.1e-05:322:62//Hs.34489:AA759306
R-HEMBA1004502//ESTs//6.9e-112:566:96//Hs.93985:N50034
R-HEMBA1004506//EST//5.3e-59:456:80//Hs.72412:AA160941
R-HEMBA1004507
R-HEMBA1004509//ESTs, Moderately similar to HYPOTHETICAL 52.2 KD PROTEIN IN MPR1-GCN20 INTERGENIC REGION [Saccharomyces cerevisiae]//2.9e-82:262:99//Hs.12820:AA004271
R-HEMBA1004534//ESTs, Highly similar to ENDOTHELIAL ACTIN-BINDING PROTEIN [Homo sapiens]//1.1e-43:281:89//Hs.58414:AA196947
R-HEMBA1004538//EST//3.3e-15:270:71//Hs.136667:AA707972
R-HEMBA1004554
R-HEMBA1004560//ESTs//8.2e-25:179:88//Hs.96560:W22924
R-HEMBA1004573//ESTs, Moderately similar to ALR [H.sapiens]//1.0:305:60//Hs.30272:AA134913
R-HEMBA1004577//ESTs//7.9e-50:319:89//Hs.22660:AA582243
R-HEMBA1004586//ESTs//2.6e-73:384:96//Hs.9582:R39769
R-nnnnnnnnnnnn//ESTs//6.0e-22:190:82//Hs.42530:N41661
R-HEMBA1004610//ESTs//1.2e-91:438:98//Hs.47823:AA780767
R-HEMBA1004617//Homo sapiens mRNA, chromosome 1 specific transcript KIAA0501//4.6e-52:327:85//Hs.159897:AB007970
R-HEMBA1004629//ESTs//2.3e-19:215:76//Hs.111995:AI375915
R-HEMBA1004631//ESTs//3.6e-99:470:98//Hs.49303:AA810785
R-HEMBA1004632//ESTs//1.0:128:66//Hs.159182:AA831152
R-HEMBA1004637//ESTs, Highly similar to HYPOTHETICAL 83.6 KD PROTEIN R05D3.2 IN CHROMOSOME III [Caenorhabditis elegans]//4.8e-111:532:98//Hs.12263:AA282393
R-HEMBA1004638//ESTs//1.2e-66:341:95//Hs.122687:AI278454
R-HEMBA1004666//ESTs//2.1e-65:333:96//Hs.98873:AA625442
R-HEMBA1004669//ESTs//0.00039:116:74//Hs.138725:N76348
R-HEMBA1004670//ESTs//1.7e-16:116:89//Hs.56825:AI057560
R-HEMBA1004672//EST//6.7-e-76:315:97//Hs.20821:R19368
R-HEMBA1004693//ESTs//6.4e-68:327:99//Hs.159066:AI093252
R-HEMBA1004697//ESTs//9.3e-98:467:98//Hs.62637:AA043562
R-HEMBA1004705//EST//0.0034:271:58//Hs.112503:AA599042
R-HEMBA1004709//EST//1.3e-55:392:85//Hs.149580:AI281881
R-HEMBA1004711//Small inducible cytokine A5 (RANTES)//1.9e-47:449:76//Hs.155464:AF088219
R-HEMBA1004725//EST//1.8e-71:424:88//Hs.155712:AI309235
R-HEMBA1004730//Homo sapiens clone 23892 mRNA sequencer//2.1e-44:467:73//Hs.91916:AF035317
R-HEMBA1004733//EST//0.99:84:65//Hs.161372:AI423151
R-HEMBA1004734//ESTs//1.8e-82:421:96//Hs.21275:N73275
R-HEMBA1004736//Ataxia telangiectasia mutated (includes complementation groups A, C and D)//9.5e-39:296:82//Hs.51187:U82828
R-HEMBA1004748//ESTs//1.7e-43:166:86//Hs.37573:H59651
R-HEMBA1004751//ESTs//8.0e-23:155:88//Hs.149464:AI279428
R-HEMBA1004752//Thromboxane A2 receptor//2.7e-45:281:89//Hs.89887:D38081
R-HEMBA1004753//40S RIBOSOMAL PROTEIN S20//8.3e-67:475:84//Hs.8102:L06498
R-HEMBA1004756//ESTs//2.0e-81:384:99//Hs.129545:N68679
R-HEMBA1004758//EST//2.0e-43:367:80//Hs.133006:AI049504
R-HEMBA1004763//ESTs//2.0e-108:567:94//Hs.3757:W87380
R-HEMBA1004768//ESTs, Weakly similar to RETROVIRUS-RELATED POL POLYPROTEIN [Mus musculus]//1.4e-47:379:81//Hs.141273:H66705
R-HEMBA1004770//ESTs//0.0014:246:61//Hs.124857:AA687092
R-HEMBA1004771//ESTs//1.1e-12:323:63//Hs.124146:AA699633
R-HEMBA1004776//ESTs//2.5e-112:567:95//Hs.12680:W74476
R-HEMBA1004778//ESTs//1.4e-33:272:75//Hs.141123:AA848167
R-nnnnnnnnnnnn
R-HEMBA1004803//ESTs//1.0e-48:319:86//Hs.139231:W87732
R-HEMBA1004806
R-HEMBA1004807//ESTs//6.2e-77:362:100//Hs.140945:N47676
R-HEMBA1004816//EST//4.3e-18:246:72//Hs.150552:AI053784
R-HEMBA1004820//Human arginine-rich nuclear protein mRNA, complete cds//5.0e-14:141:85//Hs.80510:M74002
R-HEMBA1004847
R-HEMBA1004850//ESTs//1.2e-83:395:99//Hs.30925:AA577120
R-HEMBA1004863//ESTs//7.5e-21:204:79//Hs.35036:H95267
R-HEMBA1004864
R-HEMBA1004865//EST//6.7e-18:191:75//Hs.129944:AA429362
R-HEMBA1004880//EST//4.4e-70:346:98//Hs.145094:AA452409
R-HEMBA1004889//ESTs//4.8e-117:496:97//Hs.15641:W63676
R-HEMBA1004900//ESTs//1.2e-15:283:68//Hs.157606:AI357470
R-HEMBA1004909//ESTs//7.3e-44:366:79//Hs.140329:AA714011
R-HEMBA1004918//Human mRNA for KIAA0392 gene, partial cds//4.6e-50:313:89//Hs.40100:AB002390
R-HEMBA1004923//ESTs//0.013:162:64//Hs.143655:AI128388
R-HEMBA1004929//EST//2.3e-48:250:97//Hs.131589:AI025053
R-HEMBA1004930//Cytochrome P450, subfamily I (aromatic compound-inducible), polypeptide 2//1.2e-70:547:80//Hs.1361:M55053
R-HEMBA1004933//ESTs, Weakly similar to R06C7.6 [C.elegans]//5.3e-110:530:98//Hs.18029:AI422883
R-HEMBA1004934//ESTs//1.3e-103:522:96//Hs.40415:AA037215
R-HEMBA1004944//ESTs//6.0e-21:97:84//Hs.141973:N21434
R-HEMBA1004954//ESTs//7.9e-112:596:93//Hs.6226:W61007
R-HEMBA1004956//ESTs//3.1e-58:280:100//Hs.120750:AA741074
R-HEMBA1004960//ESTs//6.9e-89:476:93//Hs.163738:AA601040
R-HEMBA1004972//ESTs//3.0e-72:381:95//Hs.55014:AA934035
R-HEMBA1004973//ESTs//2.7e-91:441:98//Hs.28144:AI292065
R-HEMBA1004977//ESTs//2.0e-95:446:99//Hs.29690:AI168404
R-HEMBA1004978//Homo sapiens natual killer cell group 2-F (NKG2-F) mRNA, complete cds//0.43:187:67//Hs.129734:AJ001683
R-HEMBA1004980//Human mRNA for KIAA0331 gene, complete cds//6.4e-53:305:91//Hs.146395:AB002329
R-HEMBA1004983//ESTs//0.16:482:57//Hs.131929:AI021894
R-HEMBA1004995
R-HEMBA1005008//EST, Weakly similar to mariner transposase [H.sapiens]//6.9e-51:482:78//Hs.141601:N63520
R-HEMBA1005009//ESTs, Highly similar to ACTIN I [Naegleria fowleri]//3.8e-109:551:96//Hs.103180:AI365212
R-HEMBA1005019//Homo sapiens mRNA for KIAA0648 protein, partial cds//2.0e-105:542:94//Hs.31921:AB014548
R-HEMBA1005029//ESTs, Weakly similar to LINE-1 REVERSE TRANSCRIPTASE HOMOLOG [Homo sapiens]//8.4e-95:491:94//Hs.16085:AI261382
R-HEMBA1005035//Human mRNA for KIAA0033 gene, partial cds//2.3e-64:312:85//Hs.22271:D26067
R-HEMBA1005039//ESTs, Weakly similar to zinc finger protein [H.sapiens]//2.6e-48:443:78//Hs.139019:N99348
R-HEMBA1005047//ESTs, Highly similar to RAS-RELATED PROTEIN RAB-5A [Canis familiaris]//1.2e-87:542:87//Hs.16258:AI376436
R-HEMBA1005050//ESTs//6.3e-46:311:86//Hs.159510:AA297145
R-HEMBA1005062//ESTs//1.1e-14:216:68//Hs.129935:AA994451
R-HEMBA1005066//Human clone 23574 mRNA sequence//2.2e-24:303:73//Hs.79385:U90905
R-HEMBA1005075//EST//0.65:214:62//Hs.133991:AI075789
R-HEMBA1005079//Human BENE mRNA, partial cds//1.9e-44:304:83//Hs.85889:U17077
R-HEMBA1005083//ESTs//2.8e-74:356:98//Hs.132272:AI393958
R-HEMBA1005101//Homo sapiens SYT interacting protein SIP mRNA, complete cds//1.7e-111:545:96//Hs.11170:AF080561
R-HEMBA1005113//ESTs//1.1e-101:512:95//Hs.7972:AI052739
R-HEMBA1005123//Ley I-L//3.6e-58:519:77//Hs.37062:AC005952
R-HEMBA1005133//H.sapiens mRNA for MACH-alpha-2 protein//8.3e-46:309:85//Hs.19949:X98173
R-HEMBA1005149//Homo sapiens mRNA, chromosome 1 specific transcript KIAA0488//4.7e-36:394:75//Hs.67619:AB007957
R-HEMBA1005152//Homo sapiens antigen NY-CO-16 mRNA, complete cds//3.6e-32:362:77//Hs.132206:AF039694
R-HEMBA1005159//EST//7.4e-47:252:94//Hs.134930:AI093397
R-HEMBA1005185//ESTs//5.2e-48:305:89//Hs.14920:AA910914
R-HEMBA1005201//ESTs//4.7e-58:293:97//Hs.23752:C05766
R-HEMBA1005202//ESTs//1.0:169:59//Hs.153423:AI198239
R-HEMBA1005219//Homo sapiens putative tumor suppressor protein (123F2) mRNA, complete cds//0.84:191:61//Hs.26931:AF061836
R-HEMBA1005223//ESTs//0.75:90:70//Hs.127446:AA167284
R-HEMBA1005232//EST//0.056:162:67//Hs.65649:F13687
R-HEMBA1005241//ESTs//3.6e-113:564:96//Hs.12770:W84331
R-HEMBA1005244//ESTs//6.4e-22:118:100//Hs.21396:AA114834
R-HEMBA1005251//ESTs//8.5e-36:213:92//Hs.161554:AA393896
R-HEMBA1005252//Homo sapiens mRNA for KIAA0585 protein, partial cds//6.1e-49:277:93//Hs.72660:AB011157
R-HEMBA1005274//ESTs//3.7e-65:322:98//Hs.105166:AA668862
R-HEMBA1005275//ESTs//2.1e-29:298:73//Hs.33393:R83391
R-HEMBA1005293//ESTs//3.5e-93:448:98//Hs.12066:AI208611
R-HEMBA1005296//ESTs//4.3e-33:168:100//Hs.13916:AI025750
R-HEMBA1005304//Small inducible cytokine A5 (RANTES)//2.8e-50:315:82//Hs.155464:AF088219
R-HEMBA1005311//Homo sapiens 4F5S mRNA, complete cds//1.3e-44:318:83//Hs.32567:AF073519
R-HEMBA1005314//ESTs//3.0e-103:491:98//Hs.41606:AI095046
R-HEMBA1005315//EST//1.9e-29:370:72//Hs.161483:N59169
R-HEMBA1005318//ESTs//3.9e-110:535:97//Hs.26771:AA126472
R-HEMBA1005331//Intercellular adhesion molecule 2//7.6e-39:256:87//Hs.83733:X15606
R-HEMBA1005353//ESTs//1.7e-81:406:96//Hs.155374:AI341467
R-HEMBA1005359//Homo sapiens neuronal thread protein AD7c-NTP mRNA, complete cds//4.7e-46:294:81//Hs.129735:AF010144
R-HEMBA1005367//Alcohol dehydrogenase 2 (class I), beta polypeptide//1.0:210:62//Hs.4:X03350
R-HEMBA1005372//ESTs//6.2e-95:451:99//Hs.135219:AI091653
R-HEMBA1005374//ESTs//1.5e-107:502:99//Hs.118208:AA947305
R-HEMBA1005389//Fc fragment of IgA, receptor for//1.0e-39:311:80//Hs.54486:X54150
R-HEMBA1005394//ESTs, Weakly similar to coded for by C. elegans cDNA yk30b3.5 [C.elegans]//4.0e-88:489:92//Hs.43864:AA131568
R-HEMBA1005403//EST//0.0011:78:75//Hs.127061:AA863278
R-HEMBA1005408//ESTs//3.2e-29:395:71//Hs.117532:AA676725
R-HEMBA1005410//ESTs//1.5e-18:271:70//Hs.144604:AI052059
R-HEMBA1005411//ESTs//1.1e-35:335:77//Hs.141181:R98757
R-HEMBA1005423//Homo sapiens cyclin-dependent kinase inhibitor (CDKN2C) mRNA, complete cds//1.8e-118:453:99//Hs.4854:AF041248
R-HEMBA1005426//Chromosome 1 specific transcript KIAA0491//0.25:264:61//Hs.136309:AB007960
R-HEMBA1005443//Homo sapiens (clone s153) mRNA fragment//1.7e-47:305:87//Hs.6445:L40391
R-HEMBA1005447//ESTs//5.7e-83:529:86//Hs.114253:AA745961
R-HEMBA1005468//ESTs//7.3e-23:249:73//Hs.61199:AA024494
R-HEMBA1005469//Human mRNA for KIAA0355 gene, complete cds//4.5e-45:320:85//Hs.153014:AB002353
R-HEMBA1005472//Human kpni repeat mrna (cdna clone pcd-kpni-8), 3' end//8.4e-73:464:87//Hs.103948:K00627
R-HEMBA1005475//ESTs//0.32:192:59//Hs.62694:AA100445
R-HEMBA1005497
R-HEMBA1005500//ESTs//2.2e-43:307:85//Hs.146811:AA410788
R-HEMBA1005506//75 kda infertility-related sperm protein [human, testis, mRNA Partial, 2427 nt]//0.11:295:60//Hs.62608:S58544
R-HEMBA1005508//ESTs//2.8e-55:319:93//Hs.50150:N90870
R-HEMBA1005511//ESTs, Weakly similar to similar to mouse MMR1 [C.elegans]//2.6e-82:387:99//Hs.67466:AI219740
R-HEMBA1005517//ESTs//4.6e-77:469:90//Hs.126787:AA203322
R-HEMBA1005518//ESTs//1.5e-108:561:94//Hs.123167:AA601045
R-HEMBA1005520//Putative mismatch repair/binding protein hMSH3//7.5e-44:179:84//Hs.42674:U61981
R-HEMBA1005526//ESTs//8.7e-46:308:86//Hs.146811:AA410788
R-HEMBA1005528//ESTs, Highly similar to POP2 PROTEIN [Saccharomyces cerevisiae]//8.6e-115:578:95//Hs.17035:AI080471
R-HEMBA1005530//ESTs//1.5e-110:551:96//Hs.107294:W72350
R-HEMBA1005548//ESTs//1.7e-100:510:96//Hs.9115:N90926
R-HEMBA1005552//Interleukin 10//2.4e-38:306:80//Hs.2180:M57627
R-HEMBA1005558//ESTs, Weakly similar to unknown [S.cerevisiae]//5.3e-77:439:91//Hs.22897:R43193
R-HEMBA1005568//ESTs, Weakly similar to !!!! ALU SUBFAMILY J WARNING ENTRY !!!! [H.sapiens]//3.4e-31:182:76//Hs.133526:N21103
R-HEMBA1005570//ESTs//3.3e-67:411:88//Hs.142245:AA489709
R-HEMBA1005576//EST//0.91:52:73//Hs.149518:AI280497
R-HEMBA1005577
R-HEMBA1005581//Homo sapiens mRNA for MEGF5, partial cds//3.1e-28:561:64//Hs.57929:AB011538
R-HEMBA1005582//ESTs//6.0e-73:371:97//Hs.103758:C06392
R-HEMBA1005583//ESTs//8.3e-79:413:95//Hs.62348:AA419539
R-HEMBA1005588//Human c-yes-1 mRNA//2.6e-52:403:83//Hs.75680:M15990
R-HEMBA1005593//ESTs//3.3e-30:139:80//Hs.142273:W37905
R-HEMBA1005595//ESTs//1.1e-97:454:100//Hs.27497:AI274820
R-HEMBA1005606//EST//1.0e-12:313:64//Hs.162402:AA573125
R-HEMBA1005609//ESTs//0.49:278:58//Hs.76235:W56390
R-HEMBA1005616//EST//1.3e-98:470:99//Hs.122230:AA781422
R-HEMBA1005621//ESTs, Weakly similar to MITOTIC MAD2 PROTEIN [S.cerevisiae]//2.8e-95:539:92//Hs.19400:AA662845
R-HEMBA1005627//Human mRNa for adipogenesis inhibitory factor//5.5e-38:317:78//Hs.1721:X58377
R-HEMBA1005631//Human mRNA for KIAA0393 gene, complete cds//2.3e-11:279:65//Hs.15245:AF041081
R-HEMBA1005632//EST//1.5e-10:181:70//Hs.120259:AA731522
R-HEMBA1005634//Homo sapiens mRNA for chemokine LEC precursor, complete cds//1.4e-25:234:80//Hs.10458:AF088219
R-HEMBA1005666//ESTs//2.3e-103:534:95//Hs.14512:AA205973
R-HEMBA1005670//ESTs//2.6e-39:166:81//Hs.139414:AI279477
R-HEMBA1005679//Esterase D/formylglutathione hydrolase//1.3e-50:322:88//Hs.82193:M13450
R-HEMBA1005680//Homo sapiens LIM protein mRNA, complete cds//3.3e-43:343:81//Hs.154103:AF061258
R-HEMBA1005685//Human homeodomain protein (Prox 1) mRNA, complete cds//0.0050:235:64//Hs.159437:U44060
R-HEMBA1005699//Human putative EPH-related PTK receptor ligand LERK-8 (Eplg8) mRNA, complete cds//1.7e-47:376:84//Hs.26988:U66406
R-HEMBA1005705//ESTs//3.0e-53:259:99//Hs.55314:AA772055
R-HEMBA1005717//EST//2.5e-59:287:99//Hs.146870:AI159943
R-HEMBA1005732//Homo sapiens mRNA for cartilage-associated protein (CASP)//1.2e-45:398:79//Hs.155481:AJ006470
R-HEMBA1005737//ESTs//2.5e-57:416:83//Hs.23245:AA053815
R-nnnnnnnnnnnn//EST//0.098:125:68//Hs.136945:AA765672
R-HEMBA1005755//EST//2.2e-22:180:84//Hs.141488:N47096
R-HEMBA1005765//Human peptide transporter (HPEPT1) mRNA, complete cds//3.9e-47:404:80//Hs.2217:U21936
R-HEMBA1005780//ESTs//1.3e-106:512:97//Hs.11901:AA173974
R-HEMBA1005813//Homo sapiens mRNA for chemokine LEC precursor, complete cds//2.0e-33:195:84//Hs.10458:AF088219
R-HEMBA1005815//ESTs//7.6e-19:290:71//Hs.112218:AI038601
R-HEMBA1005822//ESTs//5.4e-49:246:98//Hs.34804:AA514960
R-HEMBA1005829//ESTs//2.7e-72:344:99//Hs.54548:AI039201
R-HEMBA1005834//ESTs//1.6e-44:317:82//Hs.157029:AI080618
R-HEMBA1005852//ESTs//1.6e-102:544:93//Hs.9911:AA098911
R-HEMBA1005853//ESTs//1.8e-78:398:95//Hs.140248:AA757917
R-HEMBA1005884//EST//2.6e-18:275:67//Hs.139357:AA420970
R-HEMBA1005891//ESTs//2.1e-89:427:98//Hs.67317:AI022252
R-HEMBA1005894
R-HEMBA1005909//ESTs//2.6e-91:436:99//Hs.147492:AI215686
R-HEMBA1005911//ESTs//1.1e-85:446:95//Hs.134494:AI076363
R-HEMBA1005921//ESTs//1.4e-84:428:95//Hs.127993:AA970632
R-HEMBA1005931//Homo sapiens mRNA for KIAA0526 protein, complete cds//9.5e-45:446:75//Hs.59403:AB011098
R-HEMBA1005934//ESTs//0.20:142:65//Hs.97079:AA370867
R-HEMBA1005962//ESTs//1.8e-87:409:100//Hs.161292:AI199418
R-HEMBA1005963
R-HEMBA1005990//Homo sapiens I-1 receptor candidate protein mRNA, complete cds//2.2e-113:580:95//Hs.26285:AF082516
R-HEMBA1005991//Human antisecretory factor-1 mRNA, complete cds//2.0e-45:551:70//Hs.148495:AF050199
R-HEMBA1005999//ESTs//7.5e-24:201:69//Hs.157029:AI080618
R-HEMBA1006002//ESTs//3.1e-112:573:95//Hs.61233:AI379875
R-HEMBA1006005//EST//1.0:105:63//Hs.145273:AI249436
R-nnnnnnnnnnnn//Homo sapiens mRNA for KIAA0725 protein, partial cds//2.4e-28:444:67//Hs.26450:AB018268
R-HEMBA1006035//ESTs//4.5e-94:465:97//Hs.44625:N49951
R-HEMBA1006036//ESTs//6.1e-90:420:100//Hs.126771:AA916508
R-HEMBA1006042//EST//1.5e-88:424:98//Hs.132551:AA948490
R-nnnnnnnnnnnn
R-HEMBA1006081//ESTs//7.8e-68:356:95//Hs.27410:N25612
R-HEMBA1006090//EST//5.1e-66:320:99//Hs.99551:AA461517
R-HEMBA1006091//ESTs//2.0e-84:441:94//Hs.9658:AA506313
R-HEMBA1006100//Human high-affinity copper uptake protein (hCTR1) mRNA, complete cds//3.4e-43:328:82//Hs.73614:U83460
R-HEMBA1006108//ESTs//1.5e-44:228:98//Hs.26368:AA789297
R-HEMBA1006121//ESTs//1.6e-116:547:99//Hs.34151:AI279293
R-HEMBA1006124//EST//1.6e-20:286:64//Hs.148457:AI198931
R-HEMBA1006130//ESTs//8.8e-47:231:99//Hs.16470:AA121635
R-nnnnnnnnnnnn//Homo sapiens mRNA for KIAA0792 protein, complete cds//8.7e-27:296:73//Hs.119387:AB007958
R-HEMBA1006142//ESTs//1.5e-27:255:70//Hs.139507:T77542
R-HEMBA1006155//ESTs//4.9e-64:353:94//Hs.84560:R41212
R-HEMBA1006158//Deoxyuridine triphosphatase//0.99:162:62//Hs.82113:U31930
R-HEMBA1006173//ESTs//7.5e-85:462:92//Hs.79092:H29627
R-HEMBA1006182//ESTs//5.5e-29:218:72//Hs.141466:H96906
R-HEMBA1006198//ESTs//2.1e-34:282:82//Hs.142068:AA176125
R-HEMBA1006235//Homo sapiens clone 24422 mRNA sequence//6.9e-112:545:97//Hs.109268:AF070557
R-HEMBA1006248//ESTs, Highly similar to ZINC FINGER PROTEIN MFG1 [Mus musculus]//3.3e-114:581:95//Hs.23617:AA928683
R-HEMBA1006252//Human mRNA for KIAA0080 gene, partial cds//7.0e-48:284:76//Hs.74554:D38522
R-HEMBA1006253//Homo sapiens 45kDa splicing factor mRNA, complete cds//5.7e-30:179:91//Hs.15836:AF083384
R-HEMBA1006259//Homo sapiens KIAA0421 mRNA, partial cds//1.5e-45:326:84//Hs.41742:AB007881
R-HEMBA1006268//ESTs, Highly similar to c-Jun leucine zipper interactive [M.musculus]//1.2e-97:529:93//Hs.10552:AA524401
R-HEMBA1006272//ESTs, Moderately similar to RETROVIRUS-RELATED PROTEASE [H.sapiens]//2.7e-88:484:92//Hs.104129:AA923278
R-nnnnnnnnnnnn//H.sapiens PAP mRNA//5.2e-56:585:71//Hs.49007:X76770
R-HEMBA1006283//ESTs, Weakly similar to NUCLEAR POLYADENYLATED RNA-BINDING PROTEIN NAB2 [S.cerevisiae]//1.6e-66:377:91//Hs.108674:W25821
R-HEMBA1006284//ESTs//3.7e-110:544:96//Hs.55296:AI084735
R-HEMBA1006291//ESTs//2.2e-91:457:96//Hs.114611:N37019
R-HEMBA1006293//ESTs//5.4e-78:370:99//Hs.155111:AI202037
R-HEMBA1006309//ERYTHROCYTE BAND 7 INTEGRAL MEMBRANE PROTEIN//3.7e-40:167:86//Hs.74478:U33931
R-HEMBA1006310//ESTs, Weakly similar to reverse transcriptase [M.musculus]//5.6e-76:417:94//Hs.111754:AI204587
R-HEMBA1006328//Small inducible cytokine A5 (RANTES)//2.8e-60:397:78//Hs.155464:AF088219
R-HEMBA1006334//Human occludin mRNA, complete cds//0.72:369:59//Hs.93518:U49184
R-HEMBA1006344//Human plectin (PLEC1) mRNA, complete cds//0.016:217:64//Hs.79706:U53204
R-HEMBA1006347//ESTs, Highly similar to HYPOTHETICAL 97.6 KD PROTEIN IN SHP1-SEC17 INTERGENIC REGION [Saccharomyces cerevisiae]//3.6e-119:582:97//Hs.42343:AI417075
R-HEMBA1006349//ESTs//5.2e-57:305:94//Hs.6338:AA411382
R-HEMBA1006359//ESTs//8.2e-90:426:99//Hs.100873:AA678008
R-HEMBA1006364//ESTs//2.2e-98:582:91//Hs.23837:AA541787
R-HEMBA1006377//EST//0.0097:145:621/Hs.133027:AI049830
R-HEMBA1006380//Homo sapiens mRNA for KIAA0594 protein, partial cds//1.0e-41:349:79//Hs.154872:AB011166
R-HEMBA1006381//ESTs//5.1e-46:320:85//Hs.37573:H59651
R-HEMBA1006398//Human Line-1 repeat mRNA with 2 open reading frames//9.0e-87:5 82:84//Hs.23094:M19503
R-HEMBA1006416//ESTs//1.5e-17:251:73//Hs.33950:AI218923
R-HEMBA1006419//EST//8.5e-65:353:94//Hs.141309:H72778
R-HEMBA1006421//Oxytocin receptor//1.2e-12:249:68//Hs.2820:X64878
R-HEMBA1006424//ESTs, Weakly similar to pot. ORF II [H.sapiens]//6.3e-13:263:66//Hs.43127:AA258004
R-HEMBA1006426//ESTs//6.5e-84:401:99//Hs.37303:C16964
R-HEMBA1006438//EST//0.87:266:57//Hs.99456:AA457380
R-HEMBA1006445//ESTs//2.0e-81:414:96//Hs.58153:W72033
R-HEMBA1006446//Homo sapiens mRNA for cadherin-6, complete cds//1.6e-05:487:58//Hs.32963:D31784
R-HEMBA1006461//ESTs//5.1e-78:393:97//Hs.142677:R95895
R-HEMBA1006467//ESTs, Weakly similar to putative p150 [H.sapiens]//3.0e-17:342:63//Hs.111730:AA604403
R-HEMBA1006471//ESTs//3.8e-66:370:92//Hs.14063:T77441
R-HEMBA1006474
R-HEMBA1006483//Human G protein-coupled receptor (STRL22) mRNA, complete cds//4.2e-40:365:78//Hs.46468:U45984
R-HEMBA1006485//H.sapiens mRNA for aminopeptidase//2.5e-92:517:91//Hs.132243:Y07701
R-HEMBA1006486//EST//7.0e-47:240:76//Hs.161917:AA483223
R-HEMBA1006489//ESTs//2.1e-93:440:99//Hs.125264:AA873350
R-HEMBA1006492//ESTs//0.00034:52:90//Hs.163219:AA810720
R-HEMBA1006494//EST//1.8e-06:192:67//Hs.141401:H93387
R-HEMBA1006497//ESTs//6.2e-45:232:97//Hs.118015:N33117
R-HEMBA1006502//Complement component 5 receptor 1 (C5a ligand)//8.7e-16:135:72//Hs.2161:M62505
R-HEMBA1006507//Homo sapiens mRNA for KIAA0666 protein, partial cds//3.9e-117:570:96//Hs.153858:AB014566
R-HEMBA1006521//ESTs//9.9e-99:496:96//Hs.64906:AA677300
R-HEMBA1006530//ESTs//0.18:260:60//Hs.24970:AI057628
R-HEMBA1006535//GS1 PROTEIN//0.52:267:62//Hs.78991:M86934
R-HEMBA1006540//EST//0.016:143:66//Hs.148189:AA897331
R-HEMBA1006546//Homo sapiens mRNA for KIAA0582 protein, partial cds//2.2e-48:287:91//Hs.79507:AB011154
R-HEMBA10065597/ESTs, Moderately similar to neurodegeneration-associated protein 1 [R.norvegicus]//1.8e-109:547:96//Hs.21122:AA191594
R-HEMBA1006562//EST//1.1e-13:327:63//Hs.149641:AI283064
R-HEMBA1006566//ESTs//2.6e-59:311:97//Hs.146014:R51876
R-HEMBA1006569//ESTs//4.7e-89:458:96//Hs.42861:W74725
R-HEMBA1006579//ESTs//2.9e-19:110:99//Hs.126191:AA873876
R-HEMBA1006583//Human mRNA for tryptophan hydroxylase (EC 1.14.16.4)//9.5e-29:276:76//Hs.144563:AF057280
R-HEMBA1006595//ESTs//1.3e-96:487:96//Hs.43228:N67390
R-HEMBA1006597//Small inducible cytokine A5 (RANTES)//9.8e-44:291:85//Hs.155464:AF088219
R-HEMBA1006612
R-nnnnnnnnnnnn//ESTs//1.2e-25:225:80//Hs.138852:AA284247
R-HEMBA1006624//ESTs//1.9e-93:454:98//Hs.72531:AA773630
R-HEMBA1006631//Human mRNA for KIAA0033 gene, partial cds//7.5e-60:286:90//Hs.22271:D26067
R-HEMBA1006635//ESTs, Moderately similar to !!!! ALU SUBFAMILY SP WARNING ENTRY !!!! [H.sapiens]//2.7e-91:426:100//Hs.139469:AI299889
R-HEMBA1006639//ESTs, Highly similar to POLYADENYLATE-BINDING PROTEIN [Homo sapiens]//3.4e-37:186:100//Hs.109818:AA411185
R-HEMBA1006643//ESTs//1.8e-35:189:97//Hs.139640:AA846777
R-HEMBA1006648//Homo sapiens integrin-linked kinase (ILK) mRNA, complete cds//8.1e-108:567:94//Hs.6196:U40282
R-HEMBA1006652//ESTs//7.6e-100:536:93//Hs.142613:AA129427
R-HEMBA1006653//ESTs//2.0e-33:181:87//Hs.153599:AI282511
R-HEMBA1006665//EST//1.2e-13:141:72//Hs.145596:AI263102
R-HEMBA1006674//ESTs//3.1e-32:212:83//Hs.95115:AA206594
R-HEMBA1006676//ESTs//2.6e-95:510:93//Hs.39140:AI041842
R-HEMBA1006682//EST//1.4e-05:277:62//Hs.145762:AI269435
R-HEMBA1006695//Homo sapiens apoptotic protease activating factor 1 (Apaf-1) mRNA, complete cds//1.9e-32:261:79//Hs.77579:AF013263
R-HEMBA1006696//ESTs//4.5e-95:448:99//Hs.155694:AI032695
R-HEMBA1006708//ESTs, Weakly similar to Miller-Dieker lissencephaly gene [H.sapiens]//1.1e-92:483:94//Hs.6525:AI205313
R-HEMBA1006709//ESTs//3.4e-25:207:80//Hs.88617:AA872062
R-HEMBA1006717
R-HEMBA1006737//EST//5.9e-30:317:75//Hs.140568:AA826002
R-HEMBA1006744//Interleukin 10//3.7e-41:419:74//Hs.2180:M57627
R-HEMBA1006754//ESTs//1.2e-46:276:83//Hs.141254:AI334099
R-HEMBA1006758//ESTs//0.00043:48:100//Hs.157265:AA489646
R-HEMBA1006767//EST//0.094:120:65//Hs.159873:R92763
R-HEMBA1006779//EST//9.3e-45:298:85//Hs.149580:AI281881
R-HEMBA1006780//ESTs//1.6e-46:423:77//Hs.141602:N63562
R-HEMBA1006789//ESTs//7.6e-55:245:95//Hs.6459:AI092936
R-HEMBA1006795//ESTs//8.6e-47:315:78//Hs.140491:W52705
R-HEMBA1006796//ESTs//0.26:175:65//Hs.103280:AI334978
R-HEMBA1006807//Homo sapiens DEC-205 mRNA, complete cds//5.7e-47:461:75//Hs.153563:AF011333
R-HEMBA1006821//ESTs//3.5e-12:222:68//Hs.150439:AI016305
R-HEMBA1006824//Homo sapiens mRNA, clone:RES4-16//6.7e-51:298:90//Hs.121493:D25272
R-HEMBA1006832//ESTs//0.0050:108:70//Hs.12853:T65556
R-HEMBA1006849//Human mRNA for KIAA0118 gene, partial cds//2.1e-49:367:83//Hs.154326:D42087
R-HEMBA1006865//ESTs//0.85:112:63//Hs.116430:AA644665
R-nnnnnnnnnnnn//Homo sapiens mRNA for KIAA0772 protein, complete cds//1.8e-67:611:74//Hs.15519:AB018315
R-HEMBA1006885//ESTs//2.4e-66:347:96//Hs.100624:N95453
R-HEMBA1006900//ESTs//2.7e-91:466:96//Hs.32984:R89739
R-HEMBA1006921//ESTs//2.2e-33:170:100//Hs.152277:AA593117
R-HEMBA1006926//ESTs, Weakly similar to ZK1053.6 [C.elegans]//2.9e-28:213:84//Hs.9096:AA029400
R-HEMBA1006929//ESTs//4.0e-13:210:66//Hs.100895:AA479308
R-HEMBA1006936//ESTs//3.9e-05:60:93//Hs.8737:W22712
R-HEMBA1006938//EST//0.0021:244:62//Hs.144237:W52382
R-HEMBA1006941//Homo sapiens mRNA for putative thioredoxin-like protein//6.5e-77:371:98//Hs.42644:AJ010841
R-HEMBA1006949//ESTs//1.2e-67:335:98//Hs.25780:R51321
R-HEMBA1006973//ESTs//0.029:242:61//Hs.146074:N34457
R-HEMBA1006976//EST//0.70:206:61//Hs.147092:AI189827
R-HEMBA1006993//Human mRNA for KIAA0327 protein, complete cds//2.6e-47:368:80//Hs.149323:AB002325
R-HEMBA1006996//ESTs//0.027:326:58//Hs.105008:AA451679
R-HEMBA1007002//ESTs//0.13:116:66//Hs.26928:Z41440
R-HEMBA1007017//ESTs//4.3e-47:208:87//Hs.155243:N70293
R-HEMBA1007018//ESTs, Moderately similar to LIC-2 [R.norvegicus]//2.8e-112:558:96//Hs.107905:AI248363
R-HEMBA1007045
R-HEMBA1007051//ESTs//2.5e-39:321:80//Hs.146811:AA410788
R-HEMBA1007052//EST//3.4e-41:377:74//Hs.44634:N34839
R-HEMBA1007062//ESTs//1.2e-92:439:99//Hs.162882:AA807140
R-HEMBA1007066//ESTs//0.85:204:61//Hs.22795:AI208272
R-HEMBA1007073//ESTs//6.6e-52:362:85//Hs.30821:AI096866
R-HEMBA1007078//EST, Moderately similar to !!!! ALU SUBFAMILY SQ WARNING ENTRY !!!! [H.sapiens]//7.2e-40:163:83//Hs.152369:AA504818
R-HEMBA1007085//ESTs//8.1e-103:519:96//Hs.90638:AI348087
R-HEMBA1007087//ESTs//3.1e-51:354:86//Hs.6449:W95025
R-HEMBA1007112//EST//0.090:328:59//Hs.136623:AA633597
R-HEMBA1007113//Homo sapiens mRNA, clone:RES4-16//1.1e-47:427:76//Hs.121493:D25272
R-HEMBA1007129//ESTs//6.1e-13:314:65//Hs.137538:AA769438
R-HEMBA1007147
R-HEMBA1007149//ESTs//9.7e-103:540:94//Hs.127240:AA149818
R-HEMBA1007151//ESTs//8.2e-102:505:96//Hs.24948:AA977674
R-nnnnnnnnnnnn//Homo sapiens epsin 2b mRNA, complete cds//1.6e-104:529:94//Hs.22396:AF062085
R-HEMBA1007178//ESTs//2.2e-57:366:90//Hs.21648:AI302954
R-HEMBA1007194//ESTs//9.0e-68:336:98//Hs.49760:AA741051
R-HEMBA1007203//Homo sapiens mRNA for KIAA0214 protein, complete cds//1.7e-62:332:95//Hs.3363:D86987
R-HEMBA1007206//Human c-yes-1 mRNA//4.5e-49:390:80//Hs.75680:M15990
R-HEMBA1007224//Homo sapiens mRNA for KIAA0797 protein, partial cds//7.4e-98:471:97//Hs.27197:AB018340
R-HEMBA1007251//ESTs//1.6e-78:377:99//Hs.98912:AA436864
R-HEMBA1007256//ESTs//3.5e-20:127:79//Hs.137352:AA024934
R-HEMBA1007267//Homo sapiens KIAA0395 mRNA, partial cds//8.8e-48:343:83//Hs.43681:AL022394
R-HEMBA1007273//ESTs//1.0e-98:472:98//Hs.122610:AA807062
R-HEMBA1007279//ESTs//3.3e-107:558:94//Hs.126480:AI221207
R-HEMBA1007281//EST//0.074:244:63//Hs.29304:R73543
R-HEMBA1007288//EST//9.4e-43:344:81//Hs.162112:AA524804
R-HEMBA1007300//ESTs//0.096:371:57//Hs.102680:N52990
R-HEMBA1007301
R-HEMBA1007319//ESTs//7.7e-113:570:96//Hs.29263:AI337917
R-HEMBA1007320//ESTs, Moderately similar to hypothetical protein 2 [H.sapiens]//5.5e-15:311:64//Hs.142764:AA205569
R-HEMBA1007322//Human kpni repeat mrna (cdna clone pcd-kpni-4), 3' end//5.7e-49:383:83//Hs.139107:K00629
R-HEMBA1007327//Human melanoma antigen recognized by T-cells (MART-1) mRNA//1.9e-42:371:79//Hs.154069:U06452
R-HEMBA1007341//EST//3.0e-17:291:68//Hs.150788:AI301848
R-HEMBA1007342//EST//2.7e-11:263:67//Hs.145259:AI218684
R-HEMBA1007347//Homo sapiens DEC-205 mRNA, complete cds//9.7e-47:368:82//Hs.153563:AF011333
R-HEMBB1000005//ESTs, Weakly similar to putative p150 [H.sapiens]//3.3e-44:341:71//Hs.111730:AA604403
R-HEMBB1000008//Homo sapiens tumor necrosis factor superfamily member LIGHT mRNA, complete cds//3.2e-40:292:83//Hs.129708:AF064090
R-HEMBB1000018//H.sapiens mRNA for urea transporter//5.0e-49:311:87//Hs.66710:X96969
R-HEMBB1000024//ESTs//7.5e-21:234:76//Hs.157049:AI345418
R-HEMBB1000025//ESTs//2.2e-36:371:78//Hs.56562:AA056332
R-HEMBB1000030//ESTs//3.2e-76:373:97//Hs.140190:AA701449
R-HEMBB1000036//ESTs, Highly similar to HYPOTHETICAL 43.2 KD PROTEIN C34E10.1 IN CHROMOSOME III [Caenorhabditis elegans]//6.0e-92:477:95//Hs.4877:AA418465
R-HEMBB1000037//Homo sapiens erythroblast macrophage protein EMP mRNA, complete cds//2.5e-92:467:97//Hs.20815:AF084928
R-HEMBB1000039//ESTs//1.8e-43:361:71//Hs.108206:N64702
R-HEMBB1000044//EST//7.6e-70:367:95//Hs.140860:R42954
R-HEMBB1000048//EST//1.5e-45:262:91//Hs.157627:AI357802
R-HEMBB1000050//ESTs//0.039:91:74//Hs.163189:AA236903
R-HEMBB1000054//ESTs//3.0e-104:550:94//Hs.152395:AA533107
R-HEMBB1000055//ESTs, Moderately similar to UBIQUINOL-CYTOCHROME C REDUCTASE COMPLEX SUBUNIT VI REQUIRING PROTEIN [H.sapiens]//1.1e-72:350:99//Hs.116490:AA659584
R-HEMBB1000059//ESTs//1.7e-10:200:70//Hs.163954:N57939
R-HEMBB1000083//Homo sapiens mRNA for GCP170, complete cds//6.0e-41:337:80//Hs.4953:D63997
R-HEMBB1000089//Human mRNA for KIAA0355 gene, complete cds//3.5e-39:487:70//Hs.153014:AB002353
R-HEMBB1000099//ESTs//5.7e-37:353:75//Hs.22910:W18193
R-HEMBB1000103//Homo sapiens mRNA for KIAA0640 protein, partial cds//6.5e-18:298:69//Hs.153026:AB014540
R-HEMBB1000113//EST//8.2e-94:437:100//Hs.136893:AA805239
R-HEMBB1000119//Homo sapiens ASMTL gene//1.2e-84:428:95//Hs.6315:Y15521
R-HEMBB1000136//ESTs//0.043:262:59//Hs.61304:AA025692
R-HEMBB1000141//ESTs//5.0e-38:254:79//Hs.141658:N77915
R-HEMBB1000144//ESTs//9.6e-05:235:60//Hs.61700:AA033951
R-HEMBB1000173//EST//9.6e-44:258:76//Hs.161917:AA483223
R-HEMBB1000175//ESTs//4.8e-98:475:97//Hs.149740:AI199558
R-HEMBB1000198//ESTs//1.0:123:62//Hs.116602:AA665965
R-HEMBB1000215//Human mRNA for KIAA0355 gene, complete cds//2.2e-46:302:86//Hs.153014:AB002353
R-HEMBB1000217//ESTs//2.2e-105:496:99//Hs.65973:AI339364
R-HEMBB1000218//Homo sapiens DNA fragmentation factor 40 kDa subunit (DFF40) mRNA, complete cds//1.1e-48:292:79//Hs.133089:AF064019
R-HEMBB10002267/ESTs, Weakly similar to HYPOTHETICAL 37.0 KD PROTEIN B0495.8 IN CHROMOSOME II [C.elegans]//5.1e-73:449:89//Hs.16803:AA843214
R-HEMBB1000240//ESTs//1.1e-109:536:97//Hs.13528:AA523106
R-HEMBB1000244//Small inducible cytokine A5 (RANTES)//9.5e-42:323:83//Hs.155464:AF088219
R-HEMBB1000250//EST//8.8e-12:284:64//Hs.145960:AI276783
R-HEMBB1000258//EST//4.5e-14:315:66//Hs.162551:AA584782
R-HEMBB1000264
R-HEMBB1000266//ESTs, Weakly similar to similar to the beta transducin family [C.elegans]//2.7e-102:556:93//Hs.16079:AA083522
R-HEMBB1000272//ESTs//4.3e-91:480:94//Hs.107467:H11385
R-HEMBB1000274//Homo sapiens mRNA for KIAA0557 protein, partial cds//7.9e-24:198:72//Hs.101414:AB011129
R-HEMBB1000284//ESTs//4.8e-64:389:91//Hs.118043:N50458
R-HEMBB1000307//Human mRNA for KIAA0355 gene, complete cds//3.6e-43:288:87//Hs.153014:AB002353
R-HEMBB1000312//ESTs//6.0e-23:272:73//Hs.121354:AA758601
R-HEMBB1000317//ESTs//7.5e-90:424:99//Hs.150042:AI298034
R-HEMBB1000318//Small inducible cytokine A5 (RANTES)//3.3e-41:318:80//Hs.155464:AF088219
R-HEMBB1000335//ESTs//3.7e-15:324:65//Hs.85077:AA968576
R-HEMBB1000336//ESTs//6.4e-76:402:95//Hs.17207:H92480
R-HEMBB-1000337//ESTs//2.1e-80:391:97//Hs.118990:AI378084
R-HEMBB1000338//Small inducible cytokine A5 (RANTES)//4.0e-39:274:85//Hs.155464:AF088219
R-HEMBB1000339//EST//5.8e-41:336:79//Hs.151873:AA205736
R-HEMBB1000341//ESTs//3.8e-19:310:68//Hs.37573:H59651
R-HEMBB1000343//EST//1.1e-77:396:95//Hs.162664:AA605020
R-HEMBB1000354//Human mRNA for KIAA0186 gene, complete cds//1.7e-15:293:65//Hs.36232:D80008
R-HEMBB1000369//ESTs//1.6e-21:234:73//Hs.111583:AA463590
R-HEMBB1000374//Homo sapiens mRNA, chromosome 1 specific transcript KIAA0487//2.3e-56:335:77//Hs.92381:AB007956
R-HEMBB1000376//H.sapiens mRNA for urea transporter//2.7e-50:525:74//Hs.66710:X96969
R-HEMBB1000391//ESTs//6.6e-50:316:88//Hs.142259:AA828840
R-HEMBB1000399//Homo sapiens mRNA for cell cycle checkpoint protein//3.8e-109:531:97//Hs.16184:AJ001642
R-HEMBB1000402//H.sapiens mRNA for MACH-alpha-2 protein//2.7e-35:369:72//Hs.19949:X98173
R-HEMBB1000404//ESTs//0.088:298:59//Hs.61607:AA032026
R-HEMBB1000420//EST//2.2e-78:376:98//Hs.160787:AI336591
R-HEMBB1000434//Human mRNA for KIAA0118 gene, partial cds//3.9e-50:302:89//Hs.154326:D42087
R-HEMBB1000438//ESTs, Weakly similar to !!!! ALU CLASS B WARNING ENTRY !!!! [H.sapiens]//0.30:214:63//Hs.142209:AA873303
R-HEMBB1000441//Human c-yes-1 mRNA//2.2e-46:280:90//Hs.75680:M15990
R-HEMBB1000449//ESTs//7.8e-59:332:92//Hs.87013:AA130221
R-HEMBB1000455//EST//4.8e-14:421:65//Hs.68832:AA088438
R-HEMBB1000472//ESTs//1.1e-104:505:98//Hs.132824:AI033396
R-HEMBB1000480//Human mRNA for KIAA0392 gene, partial cds//2.5e-49:295:90//Hs.40100:AB002390
R-HEMBB1000487//EST//0.78:87:68//Hs.134601:AI081506
R-HEMBB1000490//Small inducible cytokine A5 (RANTES)//4.0e-39:320:80//Hs.155464:AF088219
R-HEMBB1000491//Homo sapiens PYRIN (MEFV) mRNA, complete cds//3.7e-50:312:76//Hs.113283:AF018080
R-HEMBB1000493//ESTs//7.1e-18:150:82//Hs.142068:AA176125
R-HEMBB1000510//EST//1.4e-45:139:97//Hs.152260:AA489703
R-HEMBB1000518//Human mRNA for KIAA0118 gene, partial cds//4.8e-50:415:78//Hs.154326:D42087
R-HEMBB1000523//Homo sapiens PYRIN (MEFV) mRNA, complete cds//2.7e-57:497:78//Hs.113283:AF018080
R-HEMBB1000530//ESTs//2.7e-73:425:90//Hs.141254:AI334099
R-HEMBB1000550//EST//2.9e-11:113:79//Hs.161503:N68662
R-HEMBB1000554//Human huntingtin interacting protein (HIP1) mRNA, complete cds//8.2e-13:92:81//Hs.97206:AF052288
R-HEMBB1000556//ESTs//1.1e-94:529:92//Hs.33476:N36986
R-HEMBB1000564//ESTs//1.3e-19:128:91//Hs.142058:N34258
R-HEMBB1000573//ESTs//1.6e-86:494:90//Hs.120979:AI160709
R-HEMBB1000575//ESTs//1.6e-45:232:74//Hs.141019:AA287618
R-HEMBB1000586//ESTs//5.1e-42:281:83//Hs.138852:AA284247
R-HEMBB1000589//ESTs//1.0e-10:184:71//Hs.142677:R95895
R-HEMBB1000591//ESTs//3.2e-40:406:75//Hs.138787:H73704
R-HEMBB1000592//ESTs//1.8e-97:455:99//Hs.94229:W65391
R-HEMBB1000598//Human anti secretory factor-1 mRNA, complete cds//1.8e-46:305:85//Hs.148495:AF050199
R-HEMBB1000623//ESTs//8.3e-47:277:92//Hs.6045:W67125
R-HEMBB1000630//ESTs//5.1e-106:538:96//Hs.13422:AI082249
R-HEMBB1000631//ESTs//5.1e-100:508:96//Hs.110379:N58152
R-HEMBB1000632//ESTs//6.2e-44:371:80//Hs.132722:AA618531
R-HEMBB1000637//Human mRNA for KIAA0080 gene, partial cds//6.4e-49:254:86//Hs.74554:D38522
R-HEMBB1000638//EST//2.2e-38:371:76//Hs.162236:AA551582
R-HEMBB1000643//ESTs//0.0049:191:62//Hs.55445:W31963
R-HEMBB1000649//ESTs, Moderately similar to hTAFII68 [H.sapiens]//4.0e-76:399:95//Hs.124106:AA948100
R-HEMBB1000652//ESTs//1.5e-14:271:64//Hs.163954:N57939
R-HEMBB1000665//ESTs//4.2e-12:109:87//Hs.41407:W94988
R-HEMBB1000671//ESTs//2.8e-68:439:87//Hs.140491:W52705
R-HEMBB1000673//EST//0.58:46:82//Hs.142286:AA338293
R-HEMBB1000684//ESTs//8.5e-20:307:72//Hs.122825:AA765454
R-nnnnnnnnnnnn//Homo sapiens neuroan1 mRNA, complete cds//6.5e-52:287:93//Hs.158300:AF040723
R-HEMBB1000705//Small inducible cytokine A5 (RANTES)//4.6e-24:165:78//Hs.155464:AF088219
R-HEMBB1000706//EST//1.2e-10:211:65//Hs.105524:AA521412
R-HEMBB1000709//ESTs, Weakly similar to putative p150 [H.sapiens]//3.9e-50:245:99//Hs.111730:AA604403
R-HEMBB1000725//Human mRNA for KIAA0308 gene, partial cds//0.11:350:59//Hs.10351:AB002306
R-HEMBB1000726//EST//5.3e-49:303:88//Hs.149580:AI281881
R-HEMBB100073 8//Homo sapiens mRNA, clone:RES4-16//2.5e-49:302:89//Hs.121493:D25272
R-HEMBB1000749//ESTs//1.6e-49:331:86//Hs.152788:AA630925
R-HEMBB1000763//ESTs//9.7e-104:474:95//Hs.77480:AA100522
R-HEMBB1000770//EST//1.0e-75:359:99//Hs.136564:AA642445
R-HEMBB1000781//ESTs//5.3e-66:317:99//Hs.28827:AI125541
R-HEMBB1000789//ESTs//5.9e-83:394:99//Hs.120842:AA435771
R-HEMBB1000790//PLATELET GLYCOPROTEIN V PRECURSORY//1.3e-37:193:75//Hs.73734:Z23091
R-HEMBB1000794//ESTs//7.1e-98:490:96//Hs.105743:AA532718
R-HEMBB1000807//ESTs//2.6e-22:145:92//Hs.53913:AA908961
R-HEMBB1000810//Small inducible cytokine A5 (RANTES)//1.8e-34:206:79//Hs.155464:AF088219
R-HEMBB1000821//ESTs//2.4e-90:425:99//Hs.118659:AI052447
R-HEMBB1000822//ESTs//1.7e-45:288:89//Hs.24130:R27124
R-HEMBB1000826//Small inducible cytokine A5 (RANTES)//2.9e-51:245:82//Hs.155464:AF088219
R-HEMBB1000827//EST//2.8e-40:295:84//Hs.149580:AI281881
R-HEMBB1000831//ESTs//4.0e-59:291:98//Hs.62675:AA044176
R-HEMBB1000835//ESTs//7.3e-21:124:82//Hs.102671:N52545
R-HEMBB1000840//ATPase, Na⁺/K⁺ transporting, beta 2 polypeptide//1.3e-43:163:84//Hs.78854:AF007876
R-HEMBB1000848//Homo sapiens mRNA for KIAA0565 protein, complete cds//9.5e-41:367:78//Hs.129740:AB011137
R-HEMBB1000852//EST//1.2e-09:188:70//Hs.127869:AA968599
R-HEMBB1000870//Cytochrome P450, 51 (lanosterol 14-alpha-demethylase)//1.0e-41:483:73//Hs.2379:U23942
R-HEMBB1000876//EST//0.0022:211:63//Hs.125552:AA884141
R-HEMBB1000883//ESTs//1.4e-65:343:95//Hs.98269:H27247
R-HEMBB1000887//ESTs//4.0e-22:212:79//Hs.138965:AI004740
R-HEMBB1000888//EST//8.2e-07:196:64//Hs.118276:W15258
R-HEMBB1000890//ISLET AMYLOID POLYPEPTIDE PRECURSORY//1.1e-46:327:83//Hs.51048:X68830
R-HEMBB1000893//EST//4.7e-34:242:85//Hs.149580:AI281881
R-HEMBB1000908//EST//0.95:27:100//Hs.142568:AA285066
R-HEMBB1000910//ESTs//1.9e-36:318:78//Hs.141140:AA715983
R-HEMBB1000913//Human mRNA for KIAA0327 protein, complete cds//2.5e-33:367:73//Hs.149323:AB002325
R-HEMBB1000915//ESTs//0.00018:188:61//Hs.44847:AI222742
R-HEMBB1000917//Homo sapiens KIAA0414 mRNA, partial cds//3.7e-41:228:84//Hs.127649:AB007874
R-HEMBB1000927//ESTs//2.2e-62:307:98//Hs.97044:AA365784
R-HEMBB1000947//ESTs, Weakly similar to F26E4.13 [C.elegans]//3.3e-60:350:91//Hs.49163:AA532881
R-HEMBB1000959//Human Line-1 repeat mRNA with 2 open reading frames//8.1e-84:546:86//Hs.23094:Ml9503
R-HEMBB1000973//ESTs//6.8e-95:445:99//Hs.105859:AI419354
R-HEMBB1000975//ESTs//1.2e-39:197:100//Hs.26176:AI032007
R-HEMBB1000981//EST//7.7e-58:284:98//Hs.60179:AA007242
R-HEMBB1000985//ESTs//1.2e-103:524:95//Hs.43102:AA131369
R-HEMBB1000991//EST//0.99:58:72//Hs.100246:T23625
R-HEMBB1000996//Homo sapiens LIM protein mRNA, complete cds//1.3e-41:482:70//Hs.154103:AF061258
R-HEMBB1001004//ESTs//5.7e-70:362:95//Hs.6434:W27112
R-HEMBB1001008//ESTs, Weakly similar to hypothetical L1 protein [H.sapiens]//2.3e-25:339:71//Hs.129992:H58762
R-HEMBB1001011//ESTs//4.0e-53:325:92//Hs.33268:AI191214
R-HEMBB1001014//ESTs//1.3e-46:323:83//Hs.163980:AA715814
R-HEMBB1001020//Homo sapiens PYRIN (MEFV) mRNA, complete cds//3.0e-46:305:76//Hs.113283:AF018080
R-HEMBB1001024//ESTs//8.5e-47:374:80//Hs.141602:N63562
R-HEMBB1001037//ESTs//2.6e-47:282:91//Hs.155384:Z78385
R-HEMBB1001047//EST//6.2e-33:232:74//Hs.160146:AI049975
R-HEMBB1001051//ESTs//3.7e-79:385:98//Hs.95290:AA046107
R-HEMBB1001056//Homo sapiens mRNA for KIAA0618 protein, complete cds//1.1e-87:497:91//Hs.15832:AB014518
R-HEMBB1001058//Homo sapiens mRNA for KIAA0475 protein, complete cds//2.2e-26:125:81//Hs.5737:AB007944
R-HEMBB1001060//ESTs//1.9e-37:541:69//Hs.141534:N64785
R-HEMBB1001063//ESTs//4.7e-42:269:88//Hs.55855:AA621381
R-HEMBB1001068//Homo sapiens liprin-beta2 mRNA, partial cds//9.1e-107:512:97//Hs.12953:AF034803
R-HEMBB1001096//Human HsLIM15 mRNA for HsLiml5, complete cds//1.2e-20:233:70//Hs.37181:D64108
R-HEMBB1001102//Human mRNA for KIAA0355 gene, complete cds//9.1e-40:299:82//Hs.153014:AB002353
R-HEMBB1001105//Homo sapiens PYRIN (MEFV) mRNA, complete cds//4.8e-46:296:87//Hs.113283:AF018080
R-HEMBB1001114//ESTs//6.2e-44:293:86//Hs.70279:AA757426
R-HEMBB1001117//ESTs//1.1e-80:471:90//Hs.61935:T75092
R-HEMBB1001119//ESTs//4.0e-38:213:84//Hs.109140:AI289942
R-HEMBB1001126
R-HEMBB1001133//Human SS-A/Ro ribonucleoprotein autoantigen 60 kd subunit mRNA, complete cds//1.6e-24:285:73//Hs.554:M25077
R-HEMBB1001137//ESTs//4.6e-10:66:100//Hs.74924:AI332962
R-HEMBB1001142//EST//6.4e-48:315:85//Hs.149580:AI281881
R-HEMBB1001151
R-HEMBB1001153//ESTs, Moderately similar to !!!! ALU SUBFAMILY J WARNING ENTRY !!!! [H.sapiens]//1.3e-65:331:96//Hs.154179:AA579197
R-HEMBB1001169//Oxytocin receptor//1.5e-25:165:73//Hs.2820:X64878
R-nnnnnnnnnnnn//ESTs//3.5e-41:233:93//Hs.129218:AA991162
R-HEMBB1001177
R-HEMBB1001182//ESTs//1.9e-86:455:95//Hs.6937:AA524349
R-HEMBB1001199
R-HEMBB1001208//ESTs//3.3e-43:216:99//Hs.121806:N71183
R-HEMBB1001209//ESTs//6.7e-80:409:96//Hs.141185:R99549
R-HEMBB1001210//ESTs//2.2e-46:290:88//Hs.103329:D11573
R-HEMBB1001218//Kangai 1 (suppression of tumorigenicity 6, prostate; CD82 antigen (R2 leukocyte antigen, antigen detected by monoclonal and antibody IA4))//3.1e-44:298:87//Hs.103458:X53795
R-HEMBB1001221//ESTs//9.4e-75:353:100//Hs.151504:AA550817
R-HEMBB1001234//ESTs, Highly similar to 65 KD YES-ASSOCIATED PROTEIN [Gallus gallus]//3.8e-80:400:96//Hs.71873:AA148213
R-HEMBB1001242//ESTs//1.6e-63:404:87//Hs.25534:AA149560
R-HEMBB1001249//ESTs//3.8e-34:360:70//Hs.150727:AI292236
R-HEMBB1001253//EST//0.0011:84:77//Hs.124579:AA853987
R-HEMBB1001254//ESTs//4.5e-95:444:99//Hs.161059:AI431268
R-HEMBB1001267//Homo sapiens mRNA, chromosome 1 specific transcript KIAA0501//1.3e-50:524:73//Hs.159897:AB007970
R-HEMBB1001271//Human mRNA for KIAA0118 gene, partial cds//4.0e-45:323:84//Hs.154326:D42087
R-HEMBB1001282//EST//2.9e-78:401:96//Hs.72871:AA169412
R-HEMBB1001288//ESTs, Highly similar to HYPOTHETICAL 27.3 KD PROTEIN ZK353.7 IN CHROMOSOME III [Caenorhabditis elegans]//2.6e-104:515:97//Hs.16606:W81021
R-HEMBB1001289//ESTs//7.8e-45:440:75//Hs.44702:AI148840
R-HEMBB1001294//ESTs//1.9e-100:476:99//Hs.109017:AI057112
R-HEMBB1001302
R-HEMBB1001304//ESTs//4.0e-92:431:99//Hs.113750:AI091154
R-HEMBB1001314//Interleukin 10//6.3e-41:334:79//Hs.2180:M57627
R-HEMBB1001315//Interleukin 10//1.9e-43:285:87//Hs.2180:M57627
R-HEMBB1001317//Human cytochrome P450-IIB (hIIB3) mRNA, complete cds//8.4e-45:357:81//Hs.110194:M29873
R-HEMBB1001326//ESTs//0.85:174:62//Hs.133487:AI393754
R-HEMBB1001331//ESTs, Weakly similar to DFS70 [H.sapiens]//6.5e-61:313:96//Hs.43071:AA206222
R-HEMBB1001335//EST//5.2e-80:381:99//Hs.116769:AA630365
R-HEMBB1001337//ESTs//2.7e-84:404:99//Hs.148966:AI242639
R-HEMBB1001339//ESTs//2.1e-97:485:96//Hs.88357:AA262470
R-HEMBB1001346
R-HEMBB1001348//ESTs//1.1e-43:295:85//Hs.163604:R94354
R-HEMBB1001356//EST//6.0e-11:89:88//Hs.152366:AA486721
R-HEMBB1001364//ESTs, Moderately similar to !!!! ALU SUBFAMILY J WARNING ENTRY !!!! [H.sapiens]//3.0e-12:129:79//Hs.9792:AA027055
R-HEMBB1001366//Human mRNA for KIAA0118 gene, partial cds//1.2e-50:550:72//Hs.154326:D42087
R-HEMBB1001367//ESTs//1.2e-19:165:82//Hs.146314:R99617
R-HEMBB1001369//Small inducible cytokine A5 (RANTES)//1.9e-25:217:80//Hs.155464:AF088219
R-HEMBB1001380//ESTs//4.0e-08:216:63//Hs.143763:AI174205
R-HEMBB1001384//ESTs//6.6e-110:547:96//Hs.6671:AI341699
R-HEMBB1001387//ESTs//1.1e-104:497:98//Hs.87654:AA853970
R-HEMBB1001394//ESTs//6.4e-73:428:89//Hs.139922:AA281350
R-HEMBB1001410//Alcohol dehydrogenase 7 sigma subunit (class IV)//0.88:365:58//Hs.389:X76342
R-HEMBB1001424//ESTs//1.3e-88:466:94//Hs.42174:AA194644
R-HEMBB1001426//ESTs//2.2e-45:337:82//Hs.37573:H59651
R-HEMBB1001429//EST//3.8e-59:543:76//Hs.158803:AI376846
R-HEMBB1001436//ESTs//3.7e-69:332:99//Hs.156518:AA724317
R-HEMBB1001443//ESTs//4.8e-54:270:98//Hs.21898:AI088201
R-HEMBB1001449//ESTs//3.2e-43:170:84//Hs.150727:AI292236
R-HEMBB1001454//ESTs//9.1e-46:304:86//Hs.139190:N55515
R-HEMBB1001458//ESTs//3.2e-98:478:97//Hs.50144:N67293
R-HEMBB1001463//Homo sapiens KIAA0421 mRNA, partial cds//4.3e-50:440:78//Hs.41742:AB007881
R-HEMBB1001464//ESTs, Weakly similar to K01H12.1 [C.elegans]//0.25:222:61//Hs.13275:AI341468
R-HEMBB1001482//ESTs, Moderately similar to zinc finger protein [R.norvegicus]//0.80:53:83//Hs.26799:W74481
R-HEMBB1001500//EST//1.4e-13:310:67//Hs.162663:AA604515
R-HEMBB1001521//Homo sapiens mRNA for KIAA0737 protein, complete cds//2.5e-29:186:92//Hs.17630:AB018280
R-HEMBB1001527//ESTs, Weakly similar to HYPOTHETICAL 92.1 KD PROTEIN ZK1098.3 IN CHROMOSOME III [Caenorhabditis elegans]//4.7e-51:404:81//Hs.141429:AA631915
R-HEMBB1001531//ESTs//3.3e-13:250:67//Hs.139158:AA226159
R-HEMBB1001535//H.sapiens mRNA for sigma 3B protein//1.9e-39:291:82//Hs.154782:X99459
R-HEMBB1001536//Human mRNA for KIAA0355 gene, complete cds//5.0e-44:318:83//Hs.153014:AB002353
R-HEMBB1001537//Homo sapiens KIAA0409 mRNA, pardal cds//3.2e-47:318:80//Hs.5158:AB007869
R-HEMBB1001555//ESTs//2.6e-13:182:71//Hs.112671:AI377274
R-HEMBB1001562//ESTs//1.7e-43:316:83//Hs.151365:AA643962
R-HEMBB1001564//EST//1.3e-35:141:81//Hs.162197:AA53521
R-HEMBB1001565//Human mRNA for KIAA0331 gene, complete cds//5.1e-18:152:85//Hs.146395:AB002329
R-HEMBB1001585//ESTs//1.1e-32:190:84//Hs.33354:AA179944
R-HEMBB1001586//ESTs//4.9e-94:447:99//Hs.124084:AA843219
R-HEMBB1001588//EST//8.3e-27:363:69//Hs.141603:N66015
R-HEMBB1001603//ESTs//1.2e-101:482:99//Hs.12403:AI090184
R-HEMBB1001618//ESTs//5.8e-35:437:70//Hs.136868:AA805044
R-HEMBB1001619//EST//1.7e-38:476:70//Hs.139093:AA166888
R-HEMBB1001630//Homo sapiens mRNA, clone:RES4-16//5.7e-41:193:90//Hs.121493:D25272
R-HEMBB1001635//ESTs//9.5e-34:304:82//Hs.140444:AI002082
R-HEMBB1001637//ESTs//1.0e-42:443:74//Hs.21978:AA009633
R-HEMBB1001641//EST//2.4e-06:67:86//Hs.162398:AA572813
R-HEMBB1001653//ESTs//4.8e-80:381:99//Hs.140502:AA806438
R-HEMBB1001665//ESTs//2.3e-44:372:79//Hs.132818:AI038577
R-HEMBB1001668//ESTs//0.73:212:62//Hs.8928:N32572
R-HEMBB1001673//Homo sapiens mRNA for KIAA0646 protein, complete cds//5.9e-117:573:97//Hs.24439:AB014546
R-HEMBB1001684//ESTs, Moderately similar to Tbcl [M.musculus]//5.4e-106:523:97//Hs.26939:AA804534
R-HEMBB1001685//ESTs, Moderately similar to !!!! ALU SUBFAMILY J WARNING ENTRY !!!! [H.sapiens]//1.9e-43:292:86//Hs.96337:AA225358
R-HEMBB1001695//ESTs//3.7e-101:539:94//Hs.78289:R60867
R-HEMBB1001704//EST//0.96:248:57//Hs.163025:AA703038
R-HEMBB1001706//ESTs//1.3e-39:308:81//Hs.141318:N71080
R-HEMBB1001707//ESTs, Moderately similar to hypothetical protein 2 [H.sapiens]//4.9e-32:277:73//Hs.142764:AA205569
R-HEMBB1001717//ESTs//1.6e-34:225:87//Hs.57883:AA218645
R-HEMBB1001735//ESTs, Highly similar to LINE-1 REVERSE TRANSCRIPTASE HOMOLOG [Homo sapiens]//8.6e-11:158:71//Hs.141263:H64113
R-HEMBB1001736//ESTs//0.0035:223:60//Hs.21354:AA203403
R-HEMBB1001747//EST//9.9e-55:293:81//Hs.112866:AA620488
R-HEMBB1001749//ESTs//2.5e-13:95:91//Hs.139888:N25287
R-HEMBB1001753//ESTs//2.6e-07:141:70//Hs.144604:AI052059
R-HEMBB1001756//EST//2.6e-06:165:64//Hs.121195:AA757211
R-HEMBB1001760//LOW-DENSITY LIPOPROTEIN RECEPTOR PRECURSOR//1.3e-24:264:74//Hs.70008:L00352
R-HEMBB1001762//ESTs//2.1e-81:447:93//Hs.152766:AA211369
R-HEMBB1001785//ESTs//0.040:390:58//Hs.116651:AA993406
R-HEMBB1001797//ESTs//2.1e-90:428:99//Hs.8958:AA169253
R-HEMBB1001802//Desmin//9.9e-95:497:93//Hs.119104:M63391
R-HEMBB1001812//ESTs//1.2e-12:91:78//Hs.138852:AA284247
R-HEMBB1001816//Human Line-1 repeat mRNA with 2 open reading frames//5.9e-13:143:76//Hs.23094:M19503
R-HEMBB1001831//Homo sapiens PAM COOH-terminal interactor protein 1 (PCIP1) mRNA, complete cds//5.5e-106:498:98//Hs.159396:AF056209
R-HEMBB1001836//Homo sapiens mRNA, chromosome 1 specific transcript KIAA0488//9.6e-39:288:73//Hs.67619:AB007957
R-HEMBB1001839
R-HEMBB1001850//EST//0.020:119:68//Hs.32767:H38125
R-HEMBB1001863//ESTs//4.5e-17:226:72//Hs.157253:AI357539
R-HEMBB1001867//ESTs//2.3e-16:254:68//Hs.123664:AA806106
R-HEMBB1001868//EST//9.8e-30:155:100//Hs.160572:AA888397
R-HEMBB1001869//ESTs//2.8e-42:376:78//Hs.141973:N21434
R-HEMBB1001872//EST//0.85:156:64//Hs.119501:AA487980
R-HEMBB1001874//EST//0.64:107:70//Hs.147482:AI215572
R-HEMBB1001875//EST//0.079:199:59//Hs.121810:AA775240
R-HEMBB1001880//Thromboxane A2 receptor//9.0e-47:297:88//Hs.89887:D38081
R-HEMBB1001899//ESTs//6.3e-68:323:100//Hs.121538:AA609310
R-HEMBB1001905//ESTs//4.4e-19:227:73//Hs.146173:AA906191
R-HEMBB1001906//ESTs//1.6e-90:463:95//Hs.28266:H46725
R-HEMBB1001908//Homo sapiens EVI5 homolog mRNA, complete cds//3.7e-27:557:64//Hs.26929:AF008915
R-HEMBB1001910//EST//6.0e-37:308:78//Hs.162197:AA535216
R-HEMBB1001911//Homo sapiens tapasin (NGS-17) mRNA, complete cds//8.0e-58:367:79//Hs.5247:AF029750
R-HEMBB1001915//ESTs//3.1e-73:395:93//Hs.17054:AI139897
R-HEMBB1001921//Human mRNA for KIAA0392 gene, partial cds//2.7e-50:323:88//Hs.40100:AB002390
R-HEMBB1001922//H.sapiens mRNA for novel member of serine-arginine domain protein, SRrp129//7.4e-38:531:70//Hs.153086:Y11251
R-HEMBB1001925//Human mRNA for KIAA0327 protein, complete cds//9.5e-19:199:77//Hs.149323:AB002325
R-HEMBB1001930//EST//1.9e-18:136:78//Hs.132635:AI032875
R-HEMBB1001944//EST//0.034:228:57//Hs.93664:N23366
R-HEMBB1001945//ESTs//1.8e-83:439:95//Hs.7341:N57875
R-HEMBB1001947//ESTs//5.6e-109:533:97//Hs.48855:AA134589
R-HEMBB1001950//ESTs//1.5e-107:583:93//Hs.8033:N94998
R-HEMBB1001952//ESTs//3.1e-40:283:85//Hs.146811:AA410788
R-HEMBB1001953//Human mRNA for KIAA0080 gene, partial cds//6.2e-50:284:83//Hs.74554:D38522
R-HEMBB1001957//EST//4.8e-50:382:81//Hs.149580:AI281881
R-HEMBB1001962//ESTs//1.5e-20:143:88//Hs.11924:W26972
R-HEMBB1001967//Homo sapiens mRNA for KIAA0575 protein, complete cds//2.3e-61:296:88//Hs.153468:AB011147
R-HEMBB1001973//ESTs//1.4e-48:303:88//Hs.132722:AA618531
R-HEMBB1001983//ESTs//2.6e-72:374:95//Hs.141022:H06475
R-HEMBB1001988//ESTs//2.0e-31:204:88//Hs.142531:N91572
R-HEMBB1001990//ESTs//9.4e-115:574:96//Hs.44426:AA173223
R-HEMBB1001996
R-HEMBB1001997//ESTs//7.6e-78:380:98//Hs.32682:H37798
R-HEMBB1002002//Human kpni repeat mrna (cdna clone pcd-kpni-8), 3' end//3.0e-18:222:71//Hs.103948:K00627
R-HEMBB1002005//EST//2.2e-41:339:80//Hs.160833:AI345334
R-HEMBB1002009//EST//2.9e-44:245:94//Hs.28788:R66896
R-HEMBB1002015//EST//0.0027:198:63//Hs.160868:AI359052
R-HEMBB1002042//ESTs//1.1e-75:529:84//Hs.106919:AA523900
R-HEMBB1002043//ESTs//7.9e-40:292:83//Hs.70279:AA757426
R-HEMBB1002044//ESTs//2.1e-92:460:94//Hs.115897:AA156638
R-HEMBB1002045//Homo sapiens PYRIN (MEFV) mRNA, complete cds//5.6e-75:301:85//Hs.113283:AF018080
R-HEMBB1002049//ESTs//3.8e-77:409:94//Hs.122624:R82638
R-HEMBB1002050//ESTs//8.7e-45:330:82//Hs.44702:AI148840
R-HEMBB1002068//ESTs//8.3e-70:333:99//Hs.134807:AI090671
R-HEMBB1002069//Homo sapiens neuronal thread protein AD7c-NTP mRNA, complete cds//1.5e-75:486:81//Hs.129735:AF010144
R-HEMBB1002092//ESTs//6.5e-46:331:83//Hs.22910:W18193
R-HEMBB1002094//EST//3.6e-45:280:88//Hs.149580:AI281881
R-HEMBB1002115
R-HEMBB1002139//ESTs//4.2e-45:318:85//Hs.107657:AA126814
R-HEMBB1002142//Homo sapiens haemopoietic progenitor homeobox HPX42B (HPX42B) mRNA, complete cds//1.4e-45:281:88//Hs.125231:AF068006
R-HEMBB1002152//EST//4.3e-39:250:89//Hs.156552:AA833553
R-HEMBB1002189//H.sapiens mRNA for translin associated protein X//1.4e-47:328:85//Hs.96247:X95073
R-HEMBB1002190//ESTs//8.3e-05:122:70//Hs.41974:AF039185
R-HEMBB1002193//Human sky mRNA for Sky, complete cds//8.9e-24:398:69//Hs.301:U18934
R-HEMBB1002217//EST//6.6e-50:303:89//Hs.149580:AI281881
R-HEMBB1002218//ESTs//2.3e-19:150:86//Hs.136031:W95841
R-HEMBB1002232//ESTs//8.9e-47:445:77//Hs.163971:N27584
R-HEMBB1002247//EST//6.6e-09:236:65//Hs.130578:AI004631
R-HEMBB1002249//ESTs//5.2e-16:325:64//Hs.156253:AI334807
R-HEMBB1002254//Human Line-1 repeat mRNA with 2 open reading frames//3.8e-99:590:88//Hs.23094:M19503
R-HEMBB1002255//Human mRNA for KIAA0365 gene, partial cds//5.6e-45:342:83//Hs.84123:AB002363
R-HEMBB1002266//ESTs//4.4e-98:472:98//Hs.65366:AI189112
R-HEMBB1002280//EST//2.9e-41:247:90//Hs.161917:AA483223
R-HEMBB1002300//ESTs//8.4e-19:229:75//Hs.138463:N72305
R-HEMBB1002306//Homo sapiens KIAA0432 mRNA, complete cds//0.0021:138:67//Hs.155174:AB007892
R-HEMBB1002327//EST//0.042:249:61//Hs.121097:AA714637
R-HEMBB1002329//ESTs//1.7e-94:453:99//Hs.7114:R24312
R-HEMBB1002340//ESTs//5.8e-15:163:77//Hs.26378:H10228
R-HEMBB1002342//Homo sapiens mRNA for putative thioredoxin-like protein//0.85:46:84//Hs.42644:AJ010841
R-HEMBB1002358//ESTs//2.0e-52:319:81//Hs.140255:AA708322
R-HEMBB1002359//ESTs//2.7e-106:517:97//Hs.13634:AI051613
R-HEMBB1002364//Human mRNA for KIAA0080 gene, partial cds//5.3e-37:360:65//Hs.74554:D38522
R-HEMBB1002371//Catalase//3.3e-22:235:77//Hs.76359:X04085
R-HEMBB1002381//Homo sapiens (JH8) mRNA, partial cds//1.0e-08:120:78//Hs.142296:AF072467
R-HEMBB1002383//ESTs//3.5e-108:520:98//Hs.45140:D80055
R-HEMBB1002387
R-HEMBB1002415//ESTs, Weakly similar to !!!! ALU SUBFAMILY J WARNING ENTRY !!!! [H.sapiens]//2.3e-23:168:77//Hs.133526:N21103
R-HEMBB1002425//Human mRNA for tryptophan hydroxylase (EC 1.14.16.4)//3.2e-57:304:90//Hs.144563:AF057280
R-HEMBB1002442//ESTs//2.7e-48:289:87//Hs.155243:N70293
R-HEMBB1002453//Human mRNA for KIAA0355 gene, complete cds//6.2e-45:292:87//Hs.153014:AB002353
R-HEMBB1002457//Human mRNA for KIAA0118 gene, partial cds//2.7e-46:546:71//Hs.154326:D42087
R-HEMBB1002458//EST//1.8e-72:343:100//Hs.162006:AA508089
R-HEMBB1002477//ESTs//1.6e-38:215:93//Hs.18240:AA460083
R-HEMBB1002489//ESTs//1.2e-101:534:94//Hs.7981:H15176
R-HEMBB1002492//ESTs//5.0e-14:350:62//Hs.99205:AA204969
R-HEMBB1002495//ESTs//2.1e-19:147:86//Hs.163747:AA174017
R-HEMBB1002502//ESTs, Weakly similar to p40 [H.sapiens]//1.2e-68:336:98//Hs.141515:T41142
R-HEMBB1002509//ESTs//2.7e-97:459:99//Hs.127638:AI014615
R-HEMBB1002510//ESTs, Weakly similar to located at OATL1 [H.sapiens]//2.2e-48:265:95//Hs.48827:AA873278
R-HEMBB1002520//EST//7.2e-40:198:84//Hs.140493:AA804538
R-HEMBB1002522//Human putative transmembrane receptor IL-1Rrp mRNA, complete cds//0.50:142:69//Hs.159301:U43672
R-HEMBB1002531//EST//0.024:147:61//Hs.148305:AA909605
R-HEMBB1002534//EST//3.1e-22:168:84//Hs.146794:AI149478
R-HEMBB1002545//ESTs//9.2e-90:421:99//Hs.118317:AI033259
R-HEMBB1002550//ESTs, Weakly similar to similar to S. cerevisiae LAG1 [C.elegans]//5.1e-22:210:81//Hs.11896:T68813
R-HEMBB1002556//ISLET AMYLOID POLYPEPTIDE PRECURSORY//1.9e-45:344:82//Hs.51048:X68830
R-HEMBB1002579//ESTs//4.6e-47:326:85//Hs.155184:AA573189
R-HEMBB1002582//ESTs//0.00036:91:76//Hs.140039:AA047045
R-HEMBB1002590//ESTs//1.0e-37:210:84//Hs.36658:N91138
R-HEMBB1002596//Human mRNA for KIAA0118 gene, partial cds//2.2e-46:297:87//Hs.154326:D42087
R-HEMBB1002600//EST//2.5e-17:147:84//Hs.121918:AA777424
R-HEMBB1002601//ESTs//7.8e-68:358:95//Hs.101489:R66923
R-HEMBB1002603//EST//1.1e-47:281:90//Hs.149580:AI281881
R-HEMBB1002607//ESTs//5.4e-75:379:97//Hs.29438:H42896
R-HEMBB1002610//ESTs, Weakly similar to !!!! ALU SUBFAMILY J WARNING ENTRY !!!! [H.sapiens]//6.2e-07:140:70//Hs.155456:AA707265
R-HEMBB1002613//Homo sapiens mRNA, chromosome 1 specific transcript KIAA0508//8.5e-47:278:83//Hs.159187:AB007977
R-HEMBB1002614//ESTs//3.4e-81:383:99//Hs.13012:AI094150
R-HEMBB1002617//Homo sapiens protease-activated receptor 4 mRNA, complete cds//7.4e-19:151:80//Hs.137574:AF055917
R-HEMBB1002623//ESTs//1.6e-45:288:87//Hs.138852:AA284247
R-HEMBB1002635//Small inducible cytokine A5 (RANTES)//5.5e-39:278:81//Hs.155464:AF088219
R-HEMBB1002664//EST//8.9e-49:315:87//Hs.149580:AI281881
R-HEMBB1002677//ESTs//0.65:159:62//Hs.163517:AI419775
R-HEMBB1002683//H.sapiens mRNA for delta 4-3-oxosteroid 5 beta-reductase//8.6e-54:543:75//Hs.2638:Z28339
R-HEMBB1002684//ESTs//3.0e-18:148:87//Hs.158270:AA776646
R-HEMBB1002686//ESTs//6.1e-80:419:96//Hs.103002:W02753
R-HEMBB1002692//ESTs//3.3e-58:451:82//Hs.141254:AI334099
R-HEMBB1002697//ESTs//6.2e-86:423:98//Hs.129812:AA769487
R-HEMBB1002699//EST//5.6e-46:322:84//Hs.140231:AI054398
R-HEMBB1002702//ESTs//5.6e-36:412:72//Hs.154993:AA142842
R-HEMBB1002705//POLYPOSIS LOCUS PROTEIN 1//0.024:412:58//Hs.74648:M73547
R-HEMBB1002712//ESTs//9.0e-96:451:99//Hs.136806:AA805682
R-MAMMA1000009//ESTs//3.0e-78:392:96//Hs.163947:AA678701
R-MAMMA1000019//Small inducible cytokine A5 (RANTES)//1.5e-47:247:87//Hs.155464:AF088219
R-MAMMA1000020//Zinc finger protein 2 (A1-5)//4.9e-49:384:80//Hs.155533:X60152
R-MAMMA1000025//Homo sapiens KIAA0441 mRNA, complete cds//4.7e-11:154:71//Hs.32511:AB007901
R-MAMMA1000043//Homo sapiens mRNA for KIAA0761 protein, partial cds//2.0e-58:277:84//Hs.93121:AB018304
R-MAMMA1000045//ESTs//1.0e-38:225:92//Hs.142567:AA287165
R-MAMMA1000055//EST//0.14:91:67//Hs.144061:AA996350
R-MAMMA1000057//Fucosyltransferase 1 (galactoside 2-alpha-L-fucosyltransferase, Bombay phenotype included)//3.8e-77:545:83//Hs.69747:M35531
R-MAMMA1000069//ESTs//8.0e-108:546:96//Hs.44856:N37065
R-MAMMA1000084//Homo sapiens clone 23632 mRNA sequence//7.3e-43:313:83//Hs.46918:AF052099
R-MAMMA1000085//ESTs, Highly similar to PUTATIVE CYSTEINYL-TRNA SYNTHETASE C29E6.06C [Schizosaccharomyces pombe]//7.7e-104:546:94//Hs.7779:AA045241
R-MAMMA1000092//EST, Moderately similar to !!!! ALU SUBFAMILY J WARNING ENTRY !!!! [H.sapiens]//4.2e-22:287:71//Hs.136063:U51713
R-MAMMA1000103//LOW-DENSITY LIPOPROTEIN RECEPTOR PRECURSOR//8.4e-49:334:86//Hs.70008:L00352
R-MAMMA1000117//ESTs, Weakly similar to !!!! ALU SUBFAMILY J WARNING ENTRY !!!! [H.sapiens]//3.1e-08:96:80//Hs.115088:AA230172
R-MAMMA1000129//EST//2.8e-64:310:99//Hs.136394:AA523577
R-MAMMA1000133
R-MAMMA1000134//ESTs//1.1e-21:152:87//Hs.163747:AA174017
R-MAMMA1000139//Homo sapiens mRNA, chromosome 1 specific transcript KIAA0501//6.3e-40:288:78//Hs.159897:AB007970
R-MAMMA1000143//EST//5.0e-52:314:89//Hs.149580:AI281881
R-MAMMA1000155//Homo sapiens apoptotic protease activating factor 1 (Apaf-1) mRNA, complete cds//1.5e-59:562:75//Hs.77579:AF013263
R-MAMMA1000163//ESTs//2.8e-92:457:96//Hs.114413:AA884787
R-MAMMA1000171//Homo sapiens mRNA for putative lipoic acid synthetase, partial//2.5e-39:173:83//Hs.53531:AJ224162
R-MAMMA1000173//ESTs, Highly similar to SRC SUBSTRATE P80/85 PROTEINS [Gallus gallus]//2.4e-07:63:90//Hs.90367:AI357069
R-MAMMA1000175//EST//0.66:217:58//Hs.146444:AI127611
R-MAMMA1000183//ESTs//6.7e-30:341:73//Hs.125254:AA872054
R-MAMMA1000198//EST//2.8e-45:185:88//Hs.149580:AI281881
R-MAMMA1000221//ESTs, Weakly similar to circadian clock protein [M.musculus]//1.4e-41:272:90//Hs.68398:AA421103
R-MAMMA1000227//EST//2.4e-39:388:76//Hs.144175:H70425
R-MAMMA1000241//EST//0.0027:263:61//Hs.37532:H57946
R-MAMMA1000251//Homo sapiens mRNA for KIAA0772 protein, complete cds//5.3e-47:322:86//Hs.15519:AB018315
R-MAMMA1000254//Homo sapiens tumor necrosis factor superfamily member LIGHT mRNA, complete cds//2.2e-43:315:83//Hs.129708:AF064090
R-MAMMA1000257//EST//1.6e-62:330:93//Hs.141728:W73041
R-MAMMA1000264//Von Hippel-Lindau syndrome//2.3e-31:141:81//Hs.78160:AF010238
R-MAMMA1000266//ESTs//3.4e-34:150:81//Hs.163980:AA715814
R-MAMMA1000270//Homo sapiens mRNA, chromosome 1 specific transcript KIAA0508//2.7e-57:304:78//Hs.159187:AB007977
R-MAMMA1000277//Thiopurine S-methyltransferase//3.7e-27:380:71//Hs.51124:AF019369
R-MAMMA1000278//ESTs//5.2e-99:504:95//Hs.8494:W72694
R-MAMMA1000279//Homo sapiens mRNA, chromosome 1 specific transcript KIAA0487//3.1e-58:295:83//Hs.92381:AB007956
R-MAMMA1000284//EST//4.1e-10:151:73//Hs.60742:AA017066
R-MAMMA1000287
R-MAMMA1000302//Homo sapiens KIAA0432 mRNA, complete cds//1.0:50:84//Hs.155174:AB007892
R-MAMMA1000307//Human mRNA for KIAA0033 gene, partial cds//1.8e-48:468:76//Hs.22271:D26067
R-MAMMA1000309//ESTs//1.7e-94:491:94//Hs.135106:AI335251
R-MAMMA1000312//ESTs//8.9e-74:377:96//Hs.133163:AI051434
R-MAMMA1000313//EST//8.3e-19:294:62//Hs.127400:AA954491
R-MAMMA1000331//ESTs, Moderately similar to envelope protein [H.sapiens]//8.6e-54:278:97//Hs.139170:AA662998
R-MAMMA1000339//EST//6.8e-44:169:89//Hs.149580:AI281881
R-MAMMA1000340//Homo sapiens mRNA for KIAA0625 protein, partial cds//0.82:204:61//Hs.154919:AB014525
R-MAMMA1000348//ESTs//3.3e-34:320:75//Hs.139158:AA226159
R-MAMMA1000356//ESTs, Highly similar to URIDYLATE KINASE [Saccharomyces cerevisiae]//0.42:172:61//Hs.11463:AA535912
R-MAMMA1000360//Human mRNA for KIAA0118 gene, partial cds//3.8e-43:212:82//Hs.154326:D42087
R-MAMMA1000361//ESTs//3.1e-17:188:68//Hs.164036:AA845659
R-MAMMA1000372//ESTs//1.0e-46:307:85//Hs.145032:AA343523
R-MAMMA1000385//ESTs//8.2e-97:467:98//Hs.152282:AA412065
R-MAMMA1000388//Homo sapiens UKLF mRNA for ubiquitous Kruppel like factor, complete cds//8.6e-14:106:92//Hs.32170:AB015132
R-MAMMA1000395//ESTs//1.9e-57:292:96//Hs.11365:AB01060
R-MAMMA1000402//ESTs, Moderately similar to RETROVIRUS-RELATED POL POLYPROTEIN [Mus musculus]//9.1e-47:316:81//Hs.138698:N38973
R-MAMMA1000410//Archain//1.8e-40:443:74//Hs.33642:X81198
R-MAMMA1000413//Homo sapiens mRNA for KIAA0792 protein, complete cds//1.3e-27:304:72//Hs.119387:AB007958
R-MAMMA1000414//ESTs//2.9e-27:181:87//Hs.141254:AI334099
R-MAMMA1000416//Human macrophage-derived chemokine precursor (MDC) mRNA, complete cds//1.5e-58:282:82//Hs.97203:U83171
R-MAMMA1000421//Thromboxane A2 receptor//4.9e-48:372:80//Hs.89887:D38081
R-MAMMA1000422//ESTs//0.077:240:62//Hs.123136:AA631067
R-MAMMA1000423//Human mRNA for KIAA0392 gene, partial cds//1.3e-48:375:81//Hs.40100:AB002390
R-MAMMA1000424//Human melanoma antigen recognized by T-cells (MART-1) mRNA//1.4e-44:418:75//Hs.154069:U06452
R-MAMMA1000429//ESTs//3.9e-113:565:96//Hs.5076:N53461
R-MAMMA1000431//Human macrophage-derived chemokine precursor (MDC) mRNA, complete cds//8.6e-68:302:85//Hs.97203:U83171
R-MAMMA1000444//Calcium modulating ligand//5.5e-44:344:81//Hs.13572:AF068179
R-MAMMA1000446//ESTs//1.0:236:60//Hs.126958:AI147447
R-MAMMA1000458
R-MAMMA1000468//ESTs//4.4e-51:271:96//Hs.6839:AA055176
R-MAMMA1000472//ESTs//5.4e-39:146:86//Hs.141581:AA315361
R-MAMMA1000478//ESTs//2.3e-74:365:98//Hs.140591:AA828959
R-MAMMA1000483//ESTs//9.9e-23:235:75//Hs.163592:AA280886
R-MAMMA1000490//EST//2.1e-80:500:87//Hs.142137:AA213759
R-MAMMA1000500//Small inducible cytokine A5 (RANTES)//4.7e-43:283:86//Hs.155464:AF088219
R-MAMMA1000501//ESTs//4.2e-37:250:86//Hs.141323:N80390
R-MAMMA1000516//Human mRNA for KIAA0392 gene, partial cds//5.1e-46:459:75//Hs.40100:AB002390
R-MAMMA1000522//ESTs//9.5e-16:226:70//Hs.116673:AA669267
R-MAMMA1000559//ESTs//5.2e-34:244:84//Hs.150727:AI292236
R-MAMMA1000565//EST//2.7e-38:386:76//Hs.162404:AA573131
R-MAMMA1000567//EST//0.33:49:79//Hs.147754:AI220561
R-MAMMA1000576//ESTs//4.9e-57:348:89//Hs.108921:N31211
R-MAMMA1000583//Homo sapiens KIAA0412 mRNA, partial cds//1.3e-52:373:77//Hs.6200:AB007872
R-MAMMA1000585//ESTs//5.1e-40:337:78//Hs.130815:AA936548
R-MAMMA1000594//Small inducible cytokine A5 (RANTES)//3.0e-45:225:80//Hs.155464:AF088219
R-MAMMA1000597//ESTs//2.0e-98:461:99//Hs.43212:AA993042
R-MAMMA1000605//CD4 receptor {exons 1 and 2} [human, T-lymphocyte, mRNA, 3429 nt]//1.5e-50:500:73//Hs.116007:S79267
R-MAMMA1000612//ESTs, Highly similar to HYPOTHETICAL TRP-ASP REPEATS CONTAINING PROTEIN IN SIS1-MRPL2 INTERGENIC REGION [Saccharomyces cerevisiae]//8.6e-108:559:94//Hs.29203:AI344105
R-MAMMA1000616//EST//0.071:169:60//Hs.144096:AI032180
R-MAMMA1000621//ESTs//1.0e-90:477:94//Hs.26073:R96361
R-MAMMA1000623
R-MAMMA1000625//ESTs//3.4e-98:556:91//Hs.119482:AI361002
R-MAMMA1000643//EST//4.9e-74:379:96//Hs.137447:AA342203
R-MAMMA1000664//Homo sapiens mRNA for putative lipoic acid synthetase, partial//3.2e-43:400:76//Hs.53531:AJ224162
R-MAMMA1000669//EST//6.9e-53:368:84//Hs.149580:AI281881
R-MAMMA1000670//ESTs, Highly similar to HYPOTHETICAL PROTEIN IN TONB 3'REGION [Klebsiella pneumoniae]//8.4e-98:464:98//Hs.31431:AI022065
R-MAMMA1000672//ESTs//2.0e-80:382:99//Hs.106747:AI080476
R-MAMMA1000684//ESTs//6.2e-72:357:98//Hs.67896:AA865212
R-MAMMA1000696//Human mRNA for KIAA0345 gene, complete cds//3.3e-52:216:75//Hs.98938:AB002343
R-MAMMA1000707//EST//7.0e-11:195:68//Hs.147002:AI184644
R-MAMMA1000713//Homo sapiens DEC-205 mRNA, complete cds//1.5e-45:485:74//Hs.153563:AF011333
R-MAMMA1000714//ESTs, Moderately similar to hypothetical protein 2 [H.sapiens]//1.2e-29:158:79//Hs.142764:AA205569
R-MAMMA1000718//ESTs//3.1e-45:264:88//Hs.152413:AA780515
R-MAMMA1000720//ESTs//7.4e-44:244:87//Hs.111742:R39329
R-MAMMA1000723//Homo sapiens mRNA for alpha(l,2)fucosyltransferase, complete cds//5.6e-52:350:82//Hs.46328:D87942
R-MAMMA1000731//ESTs//1.1e-19:420:66//Hs.35036:H95267
R-MAMMA1000732//EST//2.9e-20:229:74//Hs.135400:AI056893
R-MAMMA1000733//ESTs, Weakly similar to HYPOTHETICAL 92.1 KD PROTEIN ZK1098.3 IN CHROMOSOME III [Caenorhabditis elegans]//1.2e-35:371:74//Hs.141429:AA631915
R-MAMMA1000734//Homo sapiens SEC63 (SEC63) mRNA, complete cds//2.1e-58:253:98//Hs.31575:AF100141
R-MAMMA1000738//ESTs, Weakly similar to similar to Achlya ambisexualis antheridiol steroid receptor [C.elegans]//2.3e-116:557:98//Hs.71472:AA632288
R-MAMMA1000744//ESTs//0.015:143:67//Hs.135382:AI224205
R-MAMMA1000746//Human Line-1 repeat mRNA with 2 open reading frames//2.3e-90:568:86//Hs.23094:M19503
R-MAMMA1000752//Interleukin 10//2.8e-43:339:80//Hs.2180:M57627
R-MAMMA1000760//EST//5.0e-44:306:86//Hs.162404:AA573131
R-MAMMA1000761//EST//5.0e-41:187:85//Hs.162335:AA564256
R-MAMMA1000775//Human mRNA for KIAA0355 gene, complete cds//3.0e-46:465:76//Hs.153014:AB002353
R-MAMMA1000776//ESTs//1.9e-43:429:73//Hs.141742:W22204
R-MAMMA1000778//ESTs//1.8e-31:445:70//Hs.111723:H57439
R-MAMMA1000782//EST//0.0019:102:68//Hs.120686:AA747150
R-MAMMA1000798//ESTs//1.4e-13:267:69//Hs.140156:AA704163
R-MAMMA1000802//Clathrin, light polypeptide (Lcb)//1.5e-45:358:76//Hs.73919:X81637
R-MAMMA1000831//ESTs//1.3e-1,04:510:97//Hs.17494:AA572675
R-MAMMA1000839//EST//2.9e-51:307:89//Hs.149580:AI281881
R-MAMMA1000841//ESTs//1.3e-34:412:72//Hs.121256:AA757902
R-MAMMA1000842//ESTs, Moderately similar to !!!! ALU SUBFAMILY J WARNING ENTRY !!!! [H.sapiens]//9.4e-44:363:79//Hs.96337:AA225358
R-MAMMA1000843//ESTs//2.2e-106:525:97//Hs.152016:AA603097
R-MAMMA1000845//ESTs//1.6e-66:327:98//Hs.156900:AA468955
R-MAMMA1000851//ESTs//3.7e-14:115:86//Hs.140590:R76251
R-MAMMA1000855//Human mRNA for KIAA0392 gene, partial cds//5.7e-47:281:91//Hs.40100:AB002390
R-MAMMA1000856//EST//1.8e-16:150:79//Hs.136811:AA789212
R-MAMMA1000862//EST//3.2e-05:93:73//Hs.161205:AI419311
R-MAMMA1000863//ESTs//1.0e-46:446:73//Hs.153432:AA098922
R-MAMMA1000865//Homo sapiens clone 23632 mRNA sequence//3.0e-39:324:80//Hs.46918:AF052099
R-MAMMA1000867//ESTs//9.8e-16:193:76//Hs.152340:AA521399
R-MAMMA1000875//EST//3.1e-24:301:72//Hs.132635:AI032875
R-MAMMA1000876//ESTs//9.9e-48:246:97//Hs.112165:AA621243
R-MAMMA1000877//ESTs//1.4e-38:324:79//Hs.141024:H07128
R-MAMMA1000880//Homo sapiens mRNA for KIAA0594 protein, partial cds//3.2e-40:542:68//Hs.154872:AB011166
R-MAMMA1000883//ESTs//1.0:207:60//Hs.47199:N51107
R-MAMMA1000897//ESTs//2.6e-78:383:97//Hs.41067:AI310215
R-MAMMA1000905//Human mRNA for KIAA0331 gene, complete cds//9.7e-53:307:91//Hs.146395:AB002329
R-MAMMA1000906//ESTs//8.0e-25:206:83//Hs.141825:AA017093
R-MAMMA1000908//ESTs//4.4e-32:176:96//Hs.38559:AA701634
R-MAMMA1000914//ESTs//0.032:150:63//Hs.119162:AA399989
R-MAMMA1000921//Human 53K isoform of Type II phosphatidylinositol-4-phosphate 5-kinase (PIPK) mRNA, complete cds//7.7e-38:269:74//Hs.108966:U48696
R-MAMMA1000931//ESTs//1.2e-80:457:91//Hs.122319:AA782335
R-MAMMA1000940//ESTs//3.3e-43:329:82//Hs.35254:AI133727
R-MAMMA1000941//ESTs//7.5e-55:306:84//Hs.163936:AA632281
R-MAMMA1000942//ESTs//2.5e-83:405:98//Hs.116491:AA650428
R-MAMMA1000943//Cytochrome P450, subfamily I (aromatic compound-inducible), polypeptide 2//9.3e-79:567:80//Hs.1361:M55053
R-MAMMA1000956//EST//5.7e-53:256:100//Hs.162209:AA536178
R-MAMMA1000957//Kangai 1 (suppression of tumorigenicity 6, prostate; CD82 antigen (R2 leukocyte antigen, antigen detected by monoclonal and antibody IA4))//7.5e-49:340:85//Hs.103458:X53795
R-MAMMA1000962//Homo sapiens mRNA for KIAA0575 protein, complete cds//2.0e-48:216:85//Hs.153468:AB011147
R-MAMMA1000968//EST//6.2e-46:302:86//Hs.149580:AI281881
R-MAMMA1000975//ESTs//1.4e-85:428:96//Hs.141742:W22204
R-MAMMA1000979//Homo sapiens mRNA for KIAA0761 protein, partial cds//8.0e-39:338:79//Hs.93121:AB018304
R-MAMMA1000987//EST//2.8e-41:249:90//Hs.149580:AI281881
R-MAMMA1000998//Homo sapiens apoptotic protease activating factor 1 (Apaf-1) mRNA, complete cds//3.9e-50:445:77//Hs.77579:AF013263
R-MAMMA1001003//Sialophorin (gpL115, leukosialin, CD43)//4.1e-51:282:82//Hs.80738:X52075
R-MAMMA1001008//ESTs, Weakly similar to renin [H.sapiens]//1.9e-82:405:97//Hs.25863:AA630313
R-MAMMA1001021//Homo sapiens DEC-205 mRNA, complete cds//3.0e-44:309:86//Hs.153563:AF011333
R-MAMMA1001024//ESTs//6.8e-35:333:78//Hs.107657:AA126814
R-MAMMA1001030//ESTs//1.6e-110:552:96//Hs.59483:AA524536
R-MAMMA1001035//ESTs//1.0e-45:273:85//Hs.138856:H47461
R-MAMMA1001038//Human mRNA for KIAA0392 gene, partial cds//3.0e-50:298:91//Hs.40100:AB002390
R-nnnnnnnnnnnn//ESTs//3.6e-86:445:95//Hs.122625:R68650
R-MAMMA1001050//EST//2.2e-54:387:85//Hs.149580:AI281881
R-MAMMA1001059//ESTs, Moderately similar to RNA helicase [M.musculus]//1.7e-13:273:65//Hs.98738:AI015487
R-MAMMA1001067//ESTs//1.3e-38:324:78//Hs.20190:AA525532
R-MAMMA1001073//ESTs//5.2e-106:554:94//Hs.12336:W63748
R-MAMMA1001074//Human mRNA for KIAA0355 gene, complete cds//1.2e-38:544:68//Hs.153014:AB002353
R-MAMMA1001075//ESTs//2.0e-98:463:99//Hs.18341:N38944
R-MAMMA1001078//Human Line-1 repeat mRNA with 2 open reading frames//1.7e-84:556:85//Hs.23094:M19503
R-MAMMA1001082//ESTs//2.4e-71:356:97//Hs.152302:T90222
R-MAMMA1001091//ESTs//4.7e-83:429:95//Hs.154412:AA310926
R-MAMMA1001092//Homo sapiens X-ray repair cross-complementing protein 2 (XRCC2) mRNA, complete cds//6.4e-34:262:82//Hs.129727:AF035587
R-MAMMA1001105//Human putative RNA binding protein RNPL mRNA, complete cds//4.2e-27:232:76//Hs.61840:U28686
R-MAMMA1001110//ESTs//1.6e-17:128:87//Hs.161314:AI421576
R-MAMMA1001126//CD4 receptor {exons 1 and 2} [human, T-lymphocyte, mRNA, 3429 nt]//8.8e-53:462:78//Hs.116007:S79267
R-MAMMA1001133//Homo sapiens tapasin (NGS-17) mRNA, complete cds//1.8e-59:460:81//Hs.5247:AF029750
R-MAMMA1001139//ESTs//1.3e-62:341:94//Hs.18819:R01029
R-MAMMA1001143//ESTs//3.0e-48:383:80//Hs.152340:AA521399
R-MAMMA1001145//Calcium modulating ligand//5.1e-48:403:79//Hs.13572:AF068179
R-MAMMA1001154//EST//6.8e-35:313:75//Hs.162404:AA573131
R-MAMMA1001161//Homo sapiens tapasin (NGS-17) mRNA, complete cds//1.1e-58:409:84//Hs.5247:AF029750
R-MAMMA1001162//ESTs, Highly similar to t-BOP [M.musculus]//2.1e-91:430:99//Hs.129982:AI420970
R-MAMMA1001181//ESTs//5.0e-112:557:96//Hs.118181:W02251
R-MAMMA1001186//ESTs//3.8e-85:410:99//Hs.163811:W44959
R-MAMMA1001191//ESTs//0.018:57:87//Hs.141253:AA226519
R-MAMMA1001198//ESTs, Weakly similar to involved in signaling by the epidermal growth factor receptor [M.musculus]//2.6e-80:358:96//Hs.163827:AA074202
R-MAMMA1001202//ESTs//7.0e-43:230:95//Hs.79788:AA527348
R-MAMMA1001203//Clathrin, light polypeptide (Lcb)//2.8e-65:348:79//Hs.73919:X81637
R-MAMMA1001206//EST//0.098:84:72//Hs.162941:AA635148
R-MAMMA1001215//ESTs//1.3e-43:156:86//Hs.155243:N70293
R-MAMMA1001220//ESTs//8.9e-17:276:68//Hs.116518:AA653202
R-MAMMA1001222//ESTs//0.49:112:66//Hs.24668:AA897315
R-MAMMA1001243//EST//0.99:143:62//Hs.68522:C20701
R-MAMMA1001244//ESTs//2.2e-06:79:83//Hs.123163:AA809619
R-MAMMA1001249//ESTs//4.2e-68:343:97//Hs.147139:AI191307
R-MAMMA1001256//ESTs, Moderately similar to hypothetical protein 2 [H.sapiens]//4.7e-31:221:77//Hs.142764:AA205569
R-MAMMA1001259//ESTs//1.3e-43:266:90//Hs.6193:AA045149
R-MAMMA1001260//Homo sapiens mRNA for KIAA0661 protein, complete cds//2.0e-21:226:75//Hs.65238:AB014561
R-MAMMA1001268//H.sapiens HCG II mRNA//2.4e-53:181:85//Hs.146333:X81001
R-MAMMA1001271//ESTs, Highly similar to PUTATIVE SERINE/THREONINE-PROTEIN KINASE EMK [Mus musculus]//1.1e-108:546:95//Hs.18999:N30643
R-MAMMA1001274//Homo sapiens mRNA for KIAA0572 protein, partial cds//4.4e-32:188:94//Hs.14409:AB011144
R-MAMMA1001280//EST//0.0015:170:62//Hs.116770:AA630371
R-MAMMA1001292//ESTs//5.6e-102:481:99//Hs.94810:AA811876
R-MAMMA1001296//Homo sapiens mRNA for KIAA0563 protein, complete cds//2.2e-27:348:70//Hs.15731:AB011135
R-MAMMA1001298//ESTs//1.4e-44:375:79//Hs.70279:AA757426
R-MAMMA1001305//Human G protein-coupled receptor (STRL22) mRNA, complete cds//4.0e-43:300:85//Hs.46468:U45984
R-MAMMA1001322//Homo sapiens stress-activated protein kinase 4 mRNA, complete cds//8.8e-12:188:70//Hs.55771:AF004709
R-MAMMA1001324//ESTs//5.3e-68:297:88//Hs.121228:AA709471
R-MAMMA1001330//ESTs//1.6e-57:429:83//Hs.70279:AA757426
R-MAMMA1001341//Homo sapiens 4F5S mRNA, complete cds//4.8e-27:285:75//Hs.32567:AF073519
R-MAMMA1001343//ESTs//8.1e-51:273:93//Hs.162208:AA536127
R-MAMMA1001346//ESTs//1.0:122:65//Hs.33028:AA482478
R-MAMMA1001383//ESTs//1.4e-45:377:80//Hs.114671:N39322
R-MAMMA1001388//EST//7.7e-47:361:80//Hs.162197:AA535216
R-MAMMA1001397//EST//8.7e-48:337:83//Hs.149580:AI281881
R-MAMMA1001408//EST//1.2e-38:251:87//Hs.162677:AA604831
R-MAMMA1001411//ESTs//4.3e-93:435:99//Hs.105460:AA780275
R-MAMMA1001419//Homo sapiens translation initiation factor 4e mRNA, complete cds//1.6e-19:117:96//Hs.19122:AF038957
R-MAMMA1001420//ESTs//7.3e-96:507:95//Hs.55299:AI335267
R-MAMMA1001435//ESTs//5.0e-97:459:99//Hs.144843:AI222168
R-MAMMA1001442//ESTs//7.1e-28:167:83//Hs.141019:AA287618
R-MAMMA1001446//Homo sapiens KIAA0432 mRNA, complete cds//6.2e-19:328:67//Hs.155174:AB007892
R-MAMMA1001452//EST//5.6e-44:487:75//Hs.161476:N57542
R-MAMMA1001465
R-MAMMA1001476//Homo sapiens yolk sac permease-like molecule 3 (YSPL3) mRNA, complete cds//0.79:182:66//Hs.136529:AF058317
R-MAMMA1001487//Homo sapiens KIAA0395 mRNA, partial cds//1.1e-35:328:78//Hs.43681:AL022394
R-MAMMA1001501//ESTs//4.6e-100:472:98//Hs.123660:AA813065
R-MAMMA1001502//Human mRNA for KIAA0080 gene, partial cds//5.6e-15:220:69//Hs.74554:D38522
R-MAMMA1001510
R-MAMMA1001522//ESTs//3.2e-16:214:75//Hs.152816:AA634242
R-MAMMA1001547//H.sapiens mRNA for urea transporter//2.3e-45:282:89//Hs.66710:X96969
R-MAMMA1001551//Human 53K isoform of Type II phosphatidylinositol-4-phosphate 5-kinase (PIPK) mRNA, complete cds//1.9e-56:489:76//Hs.108966:U48696
R-MAMMA1001575//ESTs//4.3e-92:440:98//Hs.162882:AA807140
R-MAMMA1001576//ESTs, Highly similar to TUBULIN GAMMA CHAIN [Homo sapiens]//1.9e-111:549:96//Hs.21635:AI417305
R-MAMMA1001590//ESTs//1.1e-63:324:96//Hs.142217:AA278441
R-MAMMA1001600//ESTs//5.6e-15:159:78//Hs.138633:H98792
R-MAMMA1001604
R-MAMMA1001606//ESTs, Weakly similar to finger protein kox1 [H.sapiens]//1.9e-97:488:96//Hs.143263:AI057616
R-MAMMA1001620//Homo sapiens mRNA, clone:RES4-16//5.4e-43:408:76//Hs.121493:D25272
R-MAMMA1001627//Homo sapiens mRNA for KIAA0772 protein, complete cds//2.0e-49:472:76//Hs.15519:AB018315
R-MAMMA1001630//ESTs, Weakly similar to putative p150 [H.sapiens]//6.8e-15:168:73//Hs.115216:AA291074
R-MAMMA1001633//EST//5.1e-14:228:68//Hs.141456:N36377
R-MAMMA1001635//ESTs//3.4e-37:368:75//Hs.164033:AA769606
R-MAMMA1001649
R-MAMMA1001663//Homo sapiens neuronal thread protein AD7c-NTP mRNA, complete cds//1.7e-54:272:81//Hs.129735:AF010144
R-MAMMA1001670//Small inducible cytokine A5 (RANTES)//5.7e-50:304:89//Hs.155464:AF088219
R-MAMMA1001671//EST//1.9e-14:312:65//Hs.137153:R46248
R-MAMMA1001679//H.sapiens mRNA for rho GDP-dissociation Inhibitor 1//0.066:196:62//Hs.159161:X69550
R-MAMMA1001683//ESTs//4.9e-94:447:98//Hs.134464:AI151081
R-MAMMA1001686//Homo sapiens mRNA, chromosome 1 specific transcript KIAA0488//1.0e-17:246:73//Hs.67619:AB007957
R-MAMMA1001692//Human mRNA for KIAA0063 gene, complete cds//2.1e-47:294:89//Hs.3094:D31884
R-MAMMA1001711//ESTs//2.4e-86:439:96//Hs.18498:N52088
R-MAMMA1001715//ESTs//1.2e-73:399:9311Hs.124620:AI082338
R-MAMMA1001730//ESTs//1.1e-85:403:99//Hs.125464:AI084596
R-MAMMA1001735//ESTs, Highly similar to TUBULIN BETA-5 CHAIN [Gallus gallus]//3.7e-110:552:96//Hs.6923:AI161158
R-MAMMA1001740//ESTs//4.6e-45:342:82//Hs.37573:H59651
R-MAMMA1001743//EST//2.7e-58:412:85//Hs.149742:AI285666
R-MAMMA1001744
R-MAMMA1001745//EST//5.6e-54:374:84//Hs.137041:AA877817
R-MAMMA1001751//EST//3.5e-36:375:73//Hs.139715:N25041
R-MAMMA1001754//EST//0.18:144:66//Hs.71957:AA151413
R-MAMMA1001757//ESTs//1.0e-9.8:488:96//Hs.45184:C14904
R-MAMMA1001760//ESTs//8.7e-29:206:86//Hs.143310:AI142276
R-MAMMA1001764//ESTs//0.00012:434:58//Hs.120051:AA707847
R-MAMMA1001768//Human mRNA for KIAA0327 protein, complete cds//2.3e-41:299:85//Hs.149323:AB002325
R-MAMMA1001769//EST//1.7e-15:139:81//Hs.162399:AA572825
R-MAMMA1001771//ESTS, Moderately similar to semaphorin B [M.musculus]//7.6e-43:257:91//Hs.7634:AA481246
R-MAMMA1001783//Human high-affinity copper uptake protein (hCTR1) mRNA, complete cds//5.6e-42:272:86//Hs.73614:U83460
R-MAMMA1001785//ESTs//1.5e-87:431:98//Hs.131065:AA972238
R-MAMMA1001788//EST//0.95:108:62//Hs.145881:AI274644
R-MAMMA1001790//ESTs//4.0e-41:340:80//Hs.158045:AA425744
R-MAMMA1001806//EST//1.4e-40:297:84//Hs.141240:H60313
R-MAMMA1001812//ESTs//2.4e-93:446:98//Hs.129034:AA776892
R-MAMMA1001815//EST//0.00053:371:59//Hs.133255:AI052659
R-MAMMA1001817//Human mRNA for KIAA0226 gene, complete cds//2.1e-46:325:87//Hs.44106:D86979
R-MAMMA1001818
R-MAMMA1001820//EST//1.9e-49:303:89//Hs.149580:AI281881
R-MAMMA1001824//Homo sapiens 4F5S mRNA, complete cds//4.3e-48:438:75//Hs.32567:AF073519
R-MAMMA1001836//ESTs//3.8e-06:128:71//Hs.143611:M78140
R-MAMMA1001837//Homo sapiens KIAA0395 mRNA, partial cds//3.8e-47:339:83//Hs.43681:AL022394
R-MAMMA1001848//ESTs//2.1e-16:125:85//Hs.161662:AA836811
R-MAMMA1001851//ESTs//4.5e-48:344:84//Hs.138856:H47461
R-MAMMA1001854//Small inducible cytokine A5 (RANTES)//2.6e-38:280:83//Hs.155464:AF088219
R-MAMMA1001858//ESTs//1.1e-44:331:83//Hs.44702:AI148840
R-MAMMA1001864//Homo sapiens mRNA for KIAA0475 protein, complete cds//7.8e-31:262:77//Hs.5737:AB007944
R-nnnnnnnnnnnn//Homo sapiens antigen NY-CO-16 mRNA, complete cds//9.2e-06:450:58//Hs.132206:AF039694
R-MAMMA1001874//Human high-affinity copper uptake protein (hCTR1) mRNA, complete cds//4.9e-46:332:83//Hs.73614:U83460
R-MAMMA1001878//Cytochrome P450, 51 (lanosterol 14-alpha-demethylase)//1.2e-46:429:78//Hs.2379:U23942
R-MAMMA1001880//ESTs, Moderately similar to !!!! ALU SUBFAMILY J WARNING ENTRY !!!! [H.sapiens]//7.6e-26:230:79//Hs.106008:AA147606
R-MAMMA1001890//ESTs//1.1e-39:338:79//Hs.146811:AA410788
R-MAMMA1001907//Kangai 1 (suppression of tumorigenicity 6, prostate; CD82 antigen (R2 leukocyte antigen, antigen detected by monoclonal and antibody IA4))//6.7e-47:283:89//Hs.103458:X53795
R-nnnnnnnnnnnn//ESTs//0.043:134:65//Hs.145333:AI251374
R-MAMMA1001931//ESTs//1.8e-75:361:99//Hs.148125:AA693801
R-MAMMA1001956//Homo sapiens mRNA for KIAA0706 protein, complete cds//1.4e-18:174:77//Hs.139648:AB014606
R-MAMMA1001963//ESTs//6.7e-28:206:84//Hs.163254:AA828790
R-MAMMA1001969//ESTs, Weakly similar to hypothetical protein [H.sapiens]//6.7e-24:331:71//Hs.140506:AA308018
R-MAMMA1001970//ESTs//8.9e-61:286:84//Hs.141575:AA211734
R-MAMMA1001992//ESTs//4.4e-43:339:82//Hs.155498:W27084
R-MAMMA1002009//Small inducible cytokine A5 (RANTES)//4.6e-24:330:70//Hs.155464:AF088219
R-MAMMA1002011//ESTs//9.5e-72:360:97//Hs.13525:R39054
R-MAMMA1002032//Human melanoma antigen recognized by T-cells (MART-1) mRNA//3.7e-45:370:80//Hs.154069:U06452
R-MAMMA1002033//EST//4.6e-23:264:74//Hs.161917:AA483223
R-MAMMA1002041//ESTs//3.8e-100:465:100//Hs.141361:AI206412
R-MAMMA1002042//Homo sapiens 4F5S mRNA, complete cds//1.1e-43:407:76//Hs.32567:AF073519
R-MAMMA1002047//Homo sapiens mRNA for chemokine LEC precursor, complete cds//1.9e-37:316:74//Hs.10458:AF088219
R-MAMMA1002056//EST//1.3e-51:310:90//Hs.149580:AI281881
R-MAMMA1002058//ESTs//5.9e-16:135:84//Hs.95807:AA146979
R-MAMMA1002068//ESTs, Weakly similar to HYPOTHETICAL 43.3 KD PROTEIN IN QOXD-VPR INTERGENIC REGION [Bacillus subtilis]//4.0e-45:404:7811Hs/138596:N38806
R-MAMMA1002078//EST//2.2e-15:207:71//Hs.132635:AI032875
R-MAMMA1002082//Homo sapiens mRNA for TSC403 protein, complete cds//1.7e-42:314:83//Hs.10887:AB013924
R-MAMMA1002084//Human mRNA for KIAA0392 gene, partial cds//3.7e-46:308:87//Hs.40100:AB002390
R-MAMMA1002093//EST//0.89:213:60//Hs.151201:AI125907
R-MAMMA1002108//ESTs//1.0e-95:515:93//Hs.29002:H11347
R-MAMMA1002118
R-MAMMA1002125//Thromboxane A2 receptor//7.2e-43:335:83//Hs.89887:D38081
R-MAMMA1002132//Homo sapiens neuronal thread protein AD7c-NTP mRNA, complete cds//1.4e-58:396:78//Hs.129735:AF010144
R-MAMMA1002140//Homo sapiens nephrin (NPHS1) mRNA, complete cds//1.4e-37:422:75//Hs.128834:AF035835
R-MAMMA1002143//ESTs//0.050:123:69//Hs.8231:AA152276
R-MAMMA1002145//Homo sapiens KIAA0426 mRNA, complete cds//5.0e-21:371:69//Hs.97476:AB007886
R-MAMMA1002153//ESTs//2.0e-31:159:77//Hs.130815:AA936548
R-MAMMA1002155//Human Line-1 repeat mRNA with 2 open reading frames//8.7e-39:506:69//Hs.23094:M19503
R-MAMMA1002156//Homo sapiens mRNA for putative lipoic acid synthetase, partial//2.9e-44:336:82//Hs.53531:AJ224162
R-MAMMA1002158//ESTs//3-0e-40:313:83//Hs.118273:AA626040
R-MAMMA1002170//Homo sapiens mRNA for TRAF5, complete cds//7.7e-37:370:77//Hs.29736:AB000509
R-MAMMA1002174//ESTs//2.5e-16:186:75//Hs.141203:H52638
R-MAMMA1002198//Homo sapiens mRNA, chromosome 1 specific transcript KIAA0487//6.2e-51:318:82//Hs.92381:AB007956
R-MAMMA1002209//ESTs//9.2e-34:111:88//Hs.141575:AA211734
R-MAMMA1002215//ESTs//3.6e-101:530:94//Hs.26780:N50038
R-MAMMA1002219//Homo sapiens mRNA for KIAA0640 protein, partial cds//5.2e-45:283:88//Hs.153026:AB014540
R-MAMMA1002230//Human 53K isoform of Type II phosphatidylinositol-4-phosphate 5-kinase (PIPK) mRNA, complete cds//9.1e-50:330:77//Hs.108966:U48696
R-MAMMA1002236
R-MAMMA1002243
R-MAMMA1002250//Homo sapiens PYRIN (MEFV) mRNA, complete cds//1.2e-44:299:87//Hs.113283:AF018080
R-MAMMA1002267//Homo sapiens mRNA, chromosome 1 specific transcript
KIAA0487//1.6e-54:207:81//Hs.92381:AB007956
R-MAMMA1002268//ESTs//2.9e-94:439:100//Hs.68061:AI042283
R-MAMMA1002269//ESTs//7.4e-05:170:65//Hs.140466:AA766772
R-MAMMA1002282//ESTs//7.8e-09:69:78//Hs.159502:AA225141
R-MAMMA1002292//ESTs//5.3e-64:334:94//Hs.113606:AI138751
R-MAMMA1002293//ESTs, Moderately similar to plakophilin 2b [H.sapiens]//1.7e-39:203:81//Hs.154257:AI275982
R-MAMMA1002294//EST//8.1e-43:326:82//Hs.149580:AI281881
R-MAMMA1002297//ESTs//6.5e-45:323:83//Hs.155475:AA761454
R-MAMMA1002298//ESTs//1.7e-68:355:96//Hs.52683:H87153
R-MAMMA1002299//ESTs, Highly similar to LINE-1 REVERSE TRANSCRIPTASE HOMOLOG [Homo sapiens]//2.3e-58:346:91//Hs.140385:AA773359
R-MAMMA1002308
R-MAMMA1002310//Human melanoma antigen recognized by T-cells (MART-1) mRNA//2.2e-44:280:87//Hs.154069:U06452
R-MAMMA1002311//Human Line-1 repeat mRNA with 2 open reading frames//2.3e-70:503:81//Hs.23094:M19503
R-MAMMA1002312//EST//1.7e-31:144:80//Hs.135936:N36094
R-MAMMA1002317//Human mRNA for tryptophan hydroxylase (EC 1.14.16.4)//4.3e-49:457:76//Hs.144563:AF057280
R-MAMMA1002319//ESTs//3.9e-38:297:70//Hs.140326:AA827183
R-MAMMA1002322//ESTs//1.1e-46:301:86//Hs.155498:W27084
R-MAMMA1002329//EST//2.6e-09:146:72//Hs.132366:AI026658
R-MAMMA1002332//Homo sapiens clone 23892 mRNA sequence//2.6e-45:387:70//Hs.91916:AF035317
R-MAMMA1002333//EST//1.8e-09:139:74//Hs.137800:AA886897
R-MAMMA1002339//ESTs//4.2e-47:310:76//Hs.138865:W57618
R-MAMMA1002347//ESTS//1.5e-44:326:83//Hs.111723:H57439
R-MAMMA1002351//ESTs//3.0e-112:545:97//Hs.26209:AI143127
R-MAMMA1002352//Homo sapiens mRNA for leukemia associated gene 2//1.5e-58:259:92//Hs.43628:Y15228
R-MAMMA1002353//Human mRNA for KIAA0392 gene, partial cds//4.5e-40:360:77//Hs.40100:AB002390
R-MAMMA1002355//ESTs//1.4e-29:307:75//Hs.3769:AI085367
R-MAMMA1002356//Clathrin, light polypeptide (Lcb)//4.9e-31:217:88//Hs.73919:X81637
R-MAMMA1002359//Homo sapiens PYRIN (MEFV) mRNA, complete cds//1.1e-70:483:84//Hs.113283:AF018080
R-MAMMA1002360//ESTs//3.5e-19:301:69//Hs.124701:AA701475
R-MAMMA1002361//Homo sapiens X-ray repair cross-complementing protein 2 (XRCC2) mRNA, complete cds//2.6e-30:244:81//Hs.129727:AF035587
R-MAMMA1002362//ESTs//2.3e-43:241:88//Hs.150727:AI292236
R-MAMMA1002380//ESTs//5.1e-36:322:79//Hs.136994:AA843542
R-MAMMA1002384//Small inducible cytokine A5 (RANTES)//1.8e-42:298:84//Hs.155464:AF088219
R-MAMMA1002385//ESTs//0.57:203:63//Hs.146303:AA579061
R-MAMMA1002392//Human mRNA for platelet-activating factor acetylhydrolase 2, complete cds//5.8e-41:305:83//Hs.86188:D87845
R-MAMMA1002411//ESTs//4.4e-68:385:92//Hs.53478:N92294
R-MAMMA1002413//Homo sapiens mRNA for small GTP-binding protein, complete cds//3.3e-14:138:75//Hs.115325:D84488
R-MAMMA1002417//ESTs//1.6e-98:475:98//Hs.96345:N22588
R-MAMMA1002427//ESTs//3.1e-39:274:79//Hs.141130:H28477
R-MAMMA1002428//ESTs//8.4e-11:215:66//Hs.141022:H06475
R-MAMMA1002434//ESTS, Moderately similar to !!!! ALU SUBFAMILY SP WARNING ENTRY !!!! [H.sapiens]//2.5e-106:521:98//Hs.112152:AA487348
R-MAMMA1002446//ESTs, Weakly similar to !!!! ALU SUBFAMILY SC WARNING ENTRY !!!! [H.sapiens]//4.7e-37:374:68//Hs.157142:U85996
R-MAMMA1002454//Homo sapiens mRNA, chromosome 1 specific transcript KIAA0485//2.0e-60:323:81//Hs.89121:AB007954
R-MAMMA1002461//ESTs//4.7e-111:548:97//Hs.104281:AA147076
R-MAMMA1002470//ESTs, Highly similar to HYPOTHETICAL 80.7 KD PROTEIN IN ERG7-NMD2 INTERGENIC REGION [Saccharomyces cerevisiae]//8.5e-104:544:93//Hs.94570:AI192106
R-MAMMA1002475//ESTs, Weakly similar to !!!! ALU SUBFAMILY J WARNING ENTRY !!!! [H.sapiens]//3.4e-31:263:79//Hs.38687:AA744496
R-MAMMA10024807/ESTs, Weakly similar to !!!! ALU SUBFAMILY J WARNING ENTRY !!!! [H.sapiens]//1.0e-34:159:79//Hs.133526:N21103
R-MAMMA1002485//Homo sapiens stanniocalcin-2 (STC-2) mRNA, complete cds//8.9e-116:560:97//Hs.155223:AF055460
R-MAMMA1002494//ESTs//3.2e-47:303:88//Hs.155243:N70293
R-MAMMA1002498//Human novel homeobox mRNA for a DNA binding protein//0.0043:331:58//Hs.37035:U07664
R-MAMMA1002524//ESTs//0.0039:354:61//Hs.125797:AA806277
R-MAMMA1002530//Homo sapiens cytosolic phospholipase A2 gamma (cPLA2 gamma) mRNA, complete cds//3.9e-103:529:95//Hs.18858:AF065214
R-MAMMA1002545//Homo sapiens mRNA for KIAA0575 protein, complete cds//9.5e-50:317:88//Hs.153468:AB011147
R-MAMMA1002554//ESTs//2.3e-85:445:95//Hs.139140:AA218851
R-MAMMA1002556//ESTs, Weakly similar to !!!! ALU SUBFAMILY J WARNING ENTRY !!!! [H.sapiens]//1.0e-12:280:65//Hs.12725:T65058
R-MAMMA1002566//ESTs//2.3e-88:421:99//Hs.17602:AA705681
R-MAMMA1002571//ESTs//5.1e-97:456:99//Hs.152834:AA595693
R-MAMMA1002573//ESTs//3.1e-38:258:87//Hs.163989:R74433
R-MAMMA1002585//ESTs//7.8e-96:533:91//Hs.26009:H49371
R-MAMMA1002590//ESTs//0.61:202:62//Hs.161190:AI419258
R-MAMMA1002597//Cytochrome P450, subfamily IIB (phenobarbital-inducible), polypeptide 6//2.9e-21:177:75//Hs.1360:M29874
R-MAMMA1002598//ESTs//3.4e-113:544:97//Hs.20263:AA573737
R-MAMMA1002603//Thiopurine S-methyltransferase//7.6e-35:225:80//Hs.51124:AF019369
R-MAMMA1002612//Cytochrome P450, subfamily I (aromatic compound-inducible), polypeptide 2//4.2e-46:424:75//Hs.1361:M55053
R-MAMMA1002617//ESTs//1.1e-38:229:92//Hs.96987:W27389
R-MAMMA1002618//Landsteiner-Wiener blood group glycoprotein//1.3e-27:185:73//Hs.108287:L27670
R-MAMMA1002619//ESTs//1.7e-95:480:96//Hs.54873:AA526306
R-MAMMA1002622//Thromboxane A2 receptor//3.2e-46:298:87//Hs.89887:D38081
R-MAMMA1002623//EST//4.3e-49:336:85//Hs.149580:AI281881
R-MAMMA1002625//ESTs, Moderately similar to ovarian-specific protein [R.norvegicus]//2.3e-35:308:79//Hs.93332:AA811920
R-MAMMA1002629//Homo sapiens mRNA for small GTP-binding protein, complete cds//9.7e-57:283:86//Hs.115325:D84488
R-MAMMA1002636//Human mRNA for KIAA0392 gene, partial cds//1.2e-49:303:89//Hs.40100:AB002390
R-MAMMA1002637//ESTs//1.3e-55:391:85//Hs.95074:AI144421
R-MAMMA1002646//ESTs//7.4e-36:182:80//Hs.163937:N69915
R-MAMMA1002650//ESTs//1.6e-102:547:94//Hs.57841:W63776
R-MAMMA1002655
R-MAMMA1002662//Homo sapiens KIAA0426 mRNA, complete cds//2.2e-46:462:75//Hs.97476:AB007886
R-MAMMA1002665//Human mRNA for KIAA0118 gene, partial cds//9.1e-51:376:82//Hs.154326:D42087
R-MAMMA1002671//ESTs, Weakly similar to coded for by C. elegans cDNA yk52e10.5 [C.elegans]//5.3e-108:544:96//Hs.16464:W19606
R-MAMMA1002673//EST//3.3e-35:169:79//Hs.140046:AA668213
R-MAMMA1002684//Homo sapiens mRNA for KIAA0214 protein, complete cds//4.6e-109:544:96//Hs.3363:D86987
R-MAMMA1002685//EST//1.9e-31:223:86//Hs.112540:AA601385
R-MAMMA1002698//ESTs//5.9e-43:292:85//Hs.144660:AA652675
R-MAMMA1002699//ESTs//3.2e-25:134:100//Hs.126049:F22510
R-MAMMA1002701//ESTs, Weakly similar to !!!! ALU SUBFAMILY J WARNING ENTRY !!!! [H.sapiens]//6.9e-70:353:96//Hs.138404:R70986
R-MAMMA1002708//ESTs//2.1e-76:413:94//Hs.57932:W69234
R-MAMMA1002711//ESTs//1.9e-44:236:96//Hs.138575:H67858
R-MAMMA1002721//Homo sapiens DEC-205 mRNA, complete cds//2.7e-43:273:89//Hs.153563:AF011333
R-MAMMA1002727//ESTs//2.9e-84:395:10011Hs.162826:AA679571
R-MAMMA1002728//Small inducible cytokine A5 (RANTES)//3.4e-42:266:88//Hs.155464:AF088219
R-MAMMA1002744//ESTs//4.2e-18:473:63//Hs.42826:AA846757
R-MAMMA1002746//ESTs//1.8e-100:473:99//Hs.117558:AA779907
R-MAMMA1002748//Human melanoma antigen recognized by T-cells (MART-1) mRNA//5.8e-40:330:80//Hs.154069:U06452
R-MAMMA1002754//ESTs, Weakly similar to !!!! ALU SUBFAMILY J WARNING ENTRY !!!! [H.sapiens]//4.5e-40:369:77//Hs.105292:AA504776
R-MAMMA1002758
R-MAMMA1002764//ESTs//4.2e-103:486:99//Hs.159909:AI393281
R-MAMMA1002765//ESTs//1.6e-37:338:76//Hs.37573:H59651
R-MAMMA1002769//ESTs//0.72:409:57//Hs.141376:AI301272
R-MAMMA1002780//ESTs//1.6e-52:292:92//Hs.135985:AA342750
R-MAMMA1002782//ESTs//1.0e-31:157:80//Hs.159510:AA297145
R-MAMMA1002796//ESTs//3.8e-49:284:92//Hs.156479:AA513812
R-MAMMA1002807//Archain//1.4e-39:315:80//Hs.33642:X81198
R-MAMMA1002820//ESTs//5.0e-14:192:74//Hs.134635:AA226260
R-MAMMA1002830//EST//4.0e-50:255:97//Hs.160674:AI248319
R-MAMMA1002833//EST//1.2e-48:306:88//Hs.149580:AI281881
R-MAMMA1002835
R-MAMMA1002838//EST//2.7e-12:161:76//Hs.163252:AA828723
R-MAMMA1002842//ESTs//1.7e-41:366:78//Hs.141899:N22395
R-MAMMA1002843//Von Hippel-Lindau syndrome//8.8e-38:258:79//Hs.78160:AF010238
R-MAMMA1002844//ESTs//3.5e-51:250:99//Hs.151445:AA351081
R-MAMMA1002858//H.sapiens ERF-1 mRNA 3' end//9.0e-101:361:91//Hs.85155:X79067
R-MAMMA1002868//ESTs//2.1e-38:301:80//Hs.132717:AA171941
R-MAMMA1002871//EST//6.0e-88:413:99//Hs.149057:AI243592
R-MAMMA1002880//ESTs//6.5e-100:506:96//Hs.163533:N52194
R-MAMMA1002881//EST//1.1e-40:335:80//Hs.160895:AI365871
R-MAMMA1002886//Small inducible cytokine A5 (RANTES)//3.4e-36:228:88//Hs.155464:AF088219
R-MAMMA1002887//ESTs//4.7e-87:409:99//Hs.152l55:AA424811
R-MAMMA1002890//ESTs, Weakly similar to coded for by C. elegans cDNA CEESB82F [C.elegans]//4.2e-92:438:99//Hs.155871:AA533783
R-MAMMA1002892//Homo sapiens EVI5 homolog mRNA, complete cds//4.9e-62:322:80//Hs.26929:AF008915
R-MAMMA1002895//ESTs//2.7e-32:330:76//Hs.139132:AA211087
R-MAMMA1002908//Calcium modulating ligand//4.6e-48:313:86//Hs.13572:AF068179
R-MAMMA1002909//Human mRNA for KIAA0180 gene, partial cds//3.4e-09:132:76//Hs.90981:D80002
R-MAMMA1002930//EST//4.9e-44:260:91//Hs.149580:AI281881
R-MAMMA1002938
R-MAMMA1002941//Human Line-1 repeat mRNA with 2 open reading frames//1.1e-83:556:85//Hs.23094:M19503
R-MAMMA1002947//ESTs//7.0e-22:222:80//Hs.103395:T79243
R-MAMMA1002964//Human mRNA for KIAA0355 gene, complete cds//1.6e-44:427:77//Hs.153014:AB002353
R-MAMMA1002970//Thromboxane A2 receptor//7.9e-48:300:84//Hs.89887:D38081
R-MAMMA1002972//ESTs, Weakly similar to KIAA0371 [H.sapiens]//9.6e-104:525:95//Hs.94396:AA399630
R-MAMMA1002973//ESTs//4.4e-40:257:87//Hs.163580:H15835
R-MAMMA1002982//ESTs//2.5e-28:115:87//Hs.141694:W15279
R-MAMMA1002987//Homo sapiens DNA fragmentation factor 40 kDa subunit (DFF40) mRNA, complete cds//2.1e-41:402:67//Hs.133089:AF064019
R-MAMMA1003003//Calcium modulating ligand//1.9e-45:380:79//Hs.13572:AF068179
R-MAMMA1003004//ESTs//3.0e-07:378:60//Hs.61885:AI127857
R-MAMMA1003007//ESTs//2.0e-47:404:80//Hs.146314:R99617
R-MAMMA1003011//ESTs, Highly similar to HISTONE MACRO-H2A.1 [Rattus norvegicus]//1.4e-53:320:90//Hs.92023:AI022248
R-MAMMA1003015//ESTs//1.5e-42:363:79//Hs.155184:AA573189
R-MAMMA1003019//ESTs//4.8e-10:232:66//Hs.111341:AA251268
R-MAMMA1003026//ESTs//2.3e-83:394:99//Hs.24668:AA897315
R-MAMMA1003031//ESTs, Moderately similar to !!!! ALU SUBFAMILY J WARNING ENTRY !!!! [H.sapiens]//3.5e-27:257:77//Hs.96337:AA225358
R-MAMMA1003035//ESTs//1.3e-94:481:94//Hs.92411:AA603321
R-MAMMA1003039//EST//0.56:210:61//Hs.162248:AA552160
R-MAMMA1003040//ESTs//2.1e-17:261:70//Hs.46980:W55940
R-MAMMA1003044//EST//2.4e-18:124:91//Hs.130321:AI002941
R-MAMMA1003047//ESTs//1.0e-20:209:78//Hs.15916:H12862
R-MAMMA1003049//14-3-3 PROTEIN SIGMA//0.94:184:60//Hs.2510:X57348
R-MAMMA1003055//EST//1.0e-49:281:92//Hs.149580:AI281881
R-MAMMA1003056//ESTs//0.99:107:66//Hs.30348:AI038559
R-MAMMA1003057//ESTs, Highly similar to hypothetical protein MD6 [M.musculus]//1.1e-102:545:93//Hs.13755:AA878911
R-MAMMA1003066//H.sapiens mRNA for urea transporter//8.1e-45:322:83//Hs.66710:X96969
R-MAMMA1003089//ESTS, Weakly similar to !!!! ALU SUBFAMILY SQ WARNING ENTRY !!!! [H.sapiens]//1.4e-34:421:70//Hs.161959:AA493652
R-MAMMA1003099//ESTs//1.1e-43:379:79//Hs.37573:H59651
R-MAMMA1003104//ESTs//2.1e-97:498:96//Hs.9299:T51283
R-MAMMA1003113//EST//3.7e-29:457:70//Hs.123616:AA815366
R-MAMMA1003127//ESTs//2.6e-41:283:86//Hs.146811:AA410788
R-MAMMA1003135//ESTs//7.2e-101:504:97//Hs.87729:AA863125
R-MAMMA1003140//ESTs//4.3e-44:200:89//Hs.152093:AI149537
R-MAMMA1003146//Wingless-type MMTV integration site 5A, human homolog//0.020:413:61//Hs.152213:L20861
R-nnnnnnnnnnnn
R-MAMMA1003166//ESTs, Moderately similar to PEANUT PROTEIN [Drosophila melanogaster]//2.0e-87:524:89//Hs.6884:W30736
R-NT2RM2002580//Homo sapiens clone 24781 mRNA sequence//1.6e-111:587:94//Hs.108112:AF070640
R-NT2RM4000024//ESTs//2.9e-98:523:94//Hs.26641:R59312
R-NT2RM4000027
R-NT2RM4000030//ESTs//1.6e-96:482:96//Hs.90625:T03663
R-NT2RM4000046//ESTs//1.6e-91:461:97//Hs.151237:AI86169
R-NT2RM4000061//ESTs//4.3e-31:167:97//Hs.110821:Z78379
R-NT2RM4000085//Homo sapiens clone 24700 unknown mRNA, partial cds/4.0e-113:549:97//Hs.95665:AF070639
R-NT2RM4000086//EST//2.7e-17:212:76//Hs.137041:AA877817
R-NT2RM4000104//ESTs//3.0e-85:452:94//Hs.101750:H19708
R-NT2RM4000139//EST//3.3e-05:156:66//Hs.133228:AI052312
R-NT2RM4000155//ESTs, Moderately similar to THREONYL-TRNA SYNTHETASE, CYTOPLASMIC [H.sapiens]//1.9e-99:536:92//Hs.127810:AI246301
R-NT2RM4000156//EST//0.89:169:62//Hs.162967:AA676397
R-nnnnnnnnnnnn//ESTs//1.0:214:61//Hs.119370:W52962
R-NT2RM4000169//ESTs//5.4e-82:440:93//Hs.159379:AI382160
R-NT2RM4000191//ESTs, Weakly similar to P68 PROTEIN [H.sapiens]//4.1e-99:542:93//Hs.6366:AA614113
R-NT2RM4000197//ESTs//5.4e-113:567:96//Hs.22975:AA156723
R-NT2RM400019911ESTsl10.020:95:6511Hs.146203:AI254528
R-NT2RM4000200//ESTs//1.4e-100:488:97//Hs.126538:AA931876
R-NT2RM4000202//Small inducible cytokine A5 (RANTES)//4.3e-37:330:77//Hs.155464:AF088219
R-NT2RM4000210//Homo sapiens mRNA for KIAA0712 protein, complete cds//1.7e-103:546:94//Hs.111138:AB018255
R-NT2RM4000215
R-nnnnnnnnnnn//ESTs//7.1e-92:457:97//Hs.162074:AA477760
R-NT2RM4000233//Fms-related tyrosine kinase 1 (vascular endothelial growth factor/vascular permeability factor receptor)//0.00020:174:66//Hs.235:X51602
R-NT2RM4000244//ESTs//6.6e-61:320:95//Hs.108646:AA613031
R-NT2RM4000251//Homo sapiens mRNA for TRIP6 (thyroid receptor interacting protein)//0.63:219:62//Hs.119498:AF000974
R-NT2RM4000265//ESTs//8.8e-105:489:99//Hs.131001:AI378742
R-NT2RM4000290//ESTs//4.0e-87:435:96//Hs.162592:AA594128
R-NT2RM4000324//ESTs//2.2e-80:413:96//Hs.12313:R43673
R-NT2RM4000327//Small inducible cytokine A5 (RANTES)//3.2e-45:286:87//Hs.155464:AF088219
R-NT2RM4000344//Clathrin, light polypeptide (Lcb)//8.6e-60:452:84//Hs.73919:X81637
R-NT2RM4000349//ESTs, Weakly similar to KIAA0005 [H.sapiens]//2.5e-. 117:579:96//Hs.5216:AA534881
R-NT2RM4000354//ESTs//2.1e-85:406:99//Hs.126774:AI224479
R-NT2RM4000356//ESTs//7.9e-109:548:96//Hs.44278:AA418063
R-NT2RM4000366//Homo sapiens mRNA for KIAA0642 protein, partial cds//2.8e-113:577:95//Hs.8152:AB014542
R-NT2RM4000368//ESTs//2.2e-61:310:97//Hs.143611:M78140
R-NT2RM4000386//ESTs, Weakly similar to tenascin-like protein [D.melanogaster]//1.0e-93:521:92//Hs.41793:AA775879
R-NT2RM4000395//ESTs, Highly similar to HYPOTHETICAL 52.9 KD PROTEIN IN SAP155-YMR31 INTERGENIC REGION [Saccharomyces cerevisiae]//1.9e-99:524:94//Hs.5249:U55977
R-NT2RM4000414//EST//2.7e-06:196:64//Hs.136648:AA688285
R-NT2RM4000421//ESTs, Weakly similar to No definition line found [C.elegans]//5.4e-75:470:90//Hs.69235:AA192359
R-NT2RM4000425//H.sapiens mRNA for MACH-alpha-2 protein//0.17:112:69//Hs.19949:X98173
R-NT2RM4000433//ESTs//2.7e-100:479:98//Hs.24553:AI150687
R-NT2RM4000457//ESTs//5.1e-107:535:95//Hs.7579:AA775865
R-NT2RM4000471//ESTs, Highly similar to NIFS-LIKE 54.5 KD PROTEIN [Saccharomyces cerevisiae]//6.0e-99:492:96//Hs.21090:AA418587
R-NT2RM4000486//ESTs, Moderately similar to unnamed protein product [H.sapiens]//2.2e-102:493:97//Hs.111279:W84558
R-NT2RM4000496
R-NT2RM4000511//EST//5.1e-43:326:81//Hs.157658:AI358465
R-NT2RM4000514//ESTS//1.7e-112:552:96//Hs.6686:AA205496
R-nnnnnnnnnnnn//ESTs, Weakly similar to HYPOTHETICAL 85.0 KD PROTEIN IN CPA2-ATP2 INTERGENIC REGION [Saccharomyces cerevisiae]//1.4e-60:343:93//Hs.16014:AA074879
R-NT2RM4000520//ESTs//2.7e-55:266:100//Hs.99838:AA204731
R-NT2RM4000531//ESTs//2.0e-88:502:91//Hs.13110:T67461
R-NT2RM4000532//ESTs//0.47:290:58//Hs.148753:T91777
R-NT2RM4000534//EST//0.00025:303:60//Hs.162809:AA632198
R-NT2RM4000585//EST//0.28:63:77//Hs.150024:AI291981
R-NT2RM4000590//ESTs//5.8e-65:320:98//Hs.116017:AA613437
R-NT2RM4000595//Homo sapiens KIAA0431 mRNA, partial cds//0.99:189:64//Hs.16349:AB007891
R-NT2RM4000603//ESTs//4.6e-68:356:96//Hs.48855:AA134589
R-nnnnnnnnnnnn//ESTs//1.5e-89:431:97//Hs.26117:W16697
R-NT2RM4000616//ESTs, Highly similar to ACETYL-COENZYME A SYNTHETASE [Escherichia coli]//1.4e-102:519:96//Hs.14779:N64822
R-NT2RM4000674//ESTs//5.1e-78:398:97//Hs.8268:N70144
R-NT2RM4000689//ESTs, Weakly similar to T01G9.4 [C.elegans]//2.9e-115:550:98//Hs.11820:AA205531
R-NT2RM4000698//ESTs//2.0e-17:130:87//Hs.86420:AA927510
R-nnnnnnnnnnnn
R-NT2RM4000712//EST//0.99:103:65//Hs.114039:AA701128
R-NT2RM4000717//ESTs, Highly similar to BONE MORPHOGENETIC PROTEIN 1 PRECURSOR [Mus musculus]//2.2e-103:519:95//Hs.6823:W18181
R-NT2RM4000733//ESTs//8.7e-88:429:98//Hs.72185:AA465311
R-NT2RM4000734//Homo sapiens mRNA for KIAA0760 protein, partial cds//3.6e-105:536:95//Hs.137168:AB018303
R-NT2RM40007.41//ESTs//0.99:266:58//Hs.142718:AA034046
R-NT2RM4000751//ESTs//1.6e-20:351:66//Hs.43145:AA776988
R-NT2RM4000764
R-NT2RM4000778//EST//0.066:254:61//Hs.148232:AA904174
R-NT2RM4000779//Homo sapiens mRNA for KIAA0451 protein, complete cds//9.3e-106:546:94//Hs.18586:AB007920
R-NT2RM4000787//Human melanoma antigen recognized by T-cells (MART-1) mRNA//6.5e-40:424:73//Hs.154069:U06452
R-NT2RM4000790//EST//9.0e-48:259:94//Hs.159694:AI417008
R-NT2RM4000795//Human mRNA for KIAA0067 gene, complete cds//1.0:203:63//Hs.20991:D31891
R-NT2RM4000796//ESTs//7.0e-106:506:98//Hs.43559:AI003520
R-NT2RM4000798//Human polymorphic epithelial mucin core protein mRNA, 3' end//2.5e-28:158:96//Hs.118249:M21868
R-NT2RM4000813
R-NT2RM4000820//ESTs, Weakly similar to hypothetical protein [H.sapiens]//1.3e-109:539:97//Hs.99636:AI219667
R-NT2RM4000833//ESTs, Moderately similar to ZK863.3 [C.elegans]//4.0e-112:448:99//Hs.20223:AA482031
R-NT2RM4000848//ESTs//8.1e-97:476:97//Hs.16036:AA883864
R-NT2RM4000852//ESTs//6.4e-94:467:97//Hs.11556:AI309597
R-NT2RM4000855//ESTs//2.9e-95:544:90//Hs.106525:AI283343
R-nnnnnnnnnnnn
R-NT2RM4000895//ESTs, Moderately similar to !!!! ALU SUBFAMILY SQ WARNING ENTRY !!!! [H.sapiens]//9.3e-96:450:99//Hs.142076:AA604514
R-NT2RM4000950//ESTs//2.6e-91:438:98//Hs.43827:AA455262
R-NT2RM4000971//EST//2.9e-96:461:99//Hs.139709:AA227887
R-NT2RM4000979//EST//1.6e-67:329:98//Hs.96927:AA349647
R-NT2RM4000996//ESTs, Weakly similar to ZINC FINGER PROTEIN 91 [H.sapiens]//1.7e-82:414:96//Hs.115342:AA650126
R-NT2RM4001002//Homo sapiens mRNA for KIAA0729 protein, partial cds//3.8e-114:545:97//Hs.19542:AB018272
R-NT2RM4001016//Homo sapiens mRNA for KIAA0639 protein, partial cds//2.5e-114:556:97//Hs.15711:AB014539
R-NT2RM4001032//ESTs//7.8e-17:132:84//Hs.138720:N53352
R-NT2RM4001047//Homo sapiens UKLF mRNA for ubiquitous Kruppel like factor, complete cds//0.42:133:67//Hs.32170:AB015132
R-NT2RM4001054//ESTs//1.7e-84:404:99//Hs.116407:AA815300
R-nnnnnnnnnn//ESTs//3.4e-91:439:99//Hs.103177:W72798
R-NT2RM4001092//ESTs//1.4e-86:517:8911Hs.132969:Z78324
R-NT2RM4001116//EST//5.2e-57:275:100//Hs.131115:AI016962
R-NT2RM4001140//ESTs//5.5e-96:461:98//Hs.86965:AA252276
R-NT2RM4001151//ESTs//0.40:263:58//Hs.113189:R08311
R-NT2RM4001155//ESTs//8.3e-105:544:94//Hs.29647:W60848
R-NT2RM4001160//EST//7.6e-25:380:68//Hs.147405:AI209085
R-NT2RM4001187//ESTs, Moderately similar to !!!! ALU SUBFAMILY SC WARNING ENTRY !!!! [H.sapiens]//9.2e-43:273:91//Hs.109005:N31174
R-NT2RM4001191//Cytochrome P450, 51 (lanosterol 14-alpha-demethylase)//3.1e-32:274:70//Hs.2379:U23942
R-NT2RM4001200//ESTs//4.5e-102:494:97//Hs.31844:N32849
R-NT2RM4001203
R-NT2RM4001204//ESTs//9.8e-88:468:93//Hs.4990:T65307
R-NT2RM4001217//ESTs//1.2e-75:396:94//Hs.25042:R72410
R-NT2RM4001256//ESTs//1.0:157:62//Hs.65377:AA994677
R-NT2RM4001258//ESTs//9.6e-41:260:88//Hs.27633:N76184
R-NT2RM4001309
R-NT2RM4001313//EST//0.0022:150:66//Hs.161573:W84857
R-NT2RM4001316//ESTs//3.5e-26:139:99//Hs.23100:AI128899
R-NT2RM4001320//ESTs//1.6e-97:308:99//Hs.112024:AI042352
R-NT2RM4001340//ESTs, Highly similar to UTR4 PROTEIN [Saccharomyces cerevisiae]//1.9e-105:522:97//Hs.18442:AI129307
R-NT2RM4001344//EST//1.1e-90:436:99//Hs.95900:AA160339
R-NT2RM4001347//EST//0.17:186:61//Hs.16751:T90476
R-NT2RM4001371//EST//0.0069:270:62//Hs.99239:AA450211
R-NT2RM4001382
R-NT2RM4001384//ESTs//9.6e-91:445:98//Hs.55000:AA805507
R-NT2RM4001410//EST//0.13:50:82//Hs.157675:AI358790
R-NT2RM4001411//ESTs, Weakly similar to lymphocyte specific adaptor protein Lnk [M.musculus]//4.0e-102:539:94//Hs.15744:AI055859
R-NT2RM4001412
R-NT2RM4001414//ESTs//6.5e-35:226:88//Hs.121727:AA775895
R-NT2RM4001437//EST//0.017:169:67//Hs.13207:F10054
R-NT2RM4001444//ESTs, Weakly similar to ISOLEUCYL-TRNA SYNTHETASE, MITOCHONDRIAL [S.cerevisiae]//7.4e-108:544:94//Hs.7558:AA526812
R-NT2RM4001454//ESTs//4.7e-108:517:98//Hs.32295:N32277
R-NT2RM4001455//EST//9.6e-81:395:97//Hs.127978:AA969739
R-NT2RM4001483//Human mRNA for KIAA0033 gene, partial cds//1.8e-58:324:85//Hs.22271:D26067
R-NT2RM4001489//Homo sapiens mRNA for KIAA0685 protein, complete cds//7.0e-104:547:93//Hs.153121:AB014585
R-NT2RM4001519//Histatin 1//0.53:340:59//Hs.119101:M26664
R-NT2RM40015227/Small inducible cytokine A5 (RANTES)//8.4e-55:306:80//Hs.155464:AF088219
R-NT2RM40015577/ESTs, Weakly similar to F11A10.4 [C.elegans]//6.1e-21:165:83//Hs.29134:H43072
R-NT2RM4001565//ESTs//2.0e-103:483:99//Hs.121273:AA758027
R-NT2RM4001566//Human DNA sequence from clone 1409 on chromosome Xp11.1-11.4. Contains a Inter-Alpha-Trypsin Inhibitor Heavy Chain LIKE gene, a alternatively spliced Melanoma-Associated Antigen MAGE LIKE gene and a 6-Phosphofructo-2-kinase (Fructose-2,6-bisphosphatase) LIKE pseudogene. Contains ESTs, STSs and genomic marker DXS8032//2.7e-43:446:72//Hs.4943:Z98046
R-NT2RM4001569//ESTs//3.6e-37:186:100//Hs.86959:AA888009
R-NT2RM4001582//ESTs//1.2e-96:459:98//Hs.114432:N52946
R-nnnnnnnnnnnn
R-NT2RM4001594//ESTs//1.6e-83:404:98//Hs.134740:AA282171
R-NT2RM4001597//ESTs//6.9e-111:558:96//Hs.11408:AI358871
R-NT2RM4001605//Homo sapiens mRNA for KIAA0791 protein, complete cds//2.1e-112:565:95//Hs.23255:AB018334
R-NT2RM4001611//EST//5.9e-74:353:99//Hs.125318:AA837079
R-NT2RM4001629//ESTs//6.1e-95:453:99//Hs.115765:AA485957
R-NT2RM4001650
R-NT2RM4001662
R-NT2RM4001666//Homo sapiens mRNA for KIAA0469 protein, complete cds//3.6e-36:230:70//Hs.7764:AB007938
R-NT2RM4001682//EST//4.3e-68:393:90//Hs.157362:AI367496
R-NT2RM4001710//ESTs//4.3e-48:235:99//Hs.7299:AA203440
R-NT2RM4001714//ESTs//0.0014:568:58//Hs.50458:AA868686
R-nnnnnnnnnnn//ESTs//6.5e-104:487:99//Hs.153581:AA630465
R-NT2RM4001731//ESTs, Weakly similar to No definition line found [C.elegans]//3.1e-108:563:94//Hs.18510:AA522887
R-NT2RM4001741//T3 receptor-associating cofactor-1 [human, fetal liver, mRNA, 2930 nt]//0.083:124:68//Hs.120980:S83390
R-NT2RM4001746//ESTs//6.1e-90:420:100//Hs.139003:AA948200
R-NT2RM4001754//Human kpni repeat mrna (cdna clone pcd-kpni-4), 3' end//5.4e-59:504:78//Hs.139107:K00629
R-NT2RM4001758//ESTs//8.9e-27:140:100//Hs.149973:AI290740
R-NT2RM4001776//Homo sapiens mRNA for KIAA0727 protein, partial cds//6.4e-24:236:80//Hs.39871:AB018270
R-NT2RM4001783//ESTs//9.9e-30:156:99//Hs.115260:AA314956
R-NT2RM4001810//ESTs//1.3e-65;346:95//Hs.131915:W22567
R-NT2RM4001813//ESTs//5.7e-102:473:100//Hs.87574:AI089920
R-NT2RM4001823//ESTs//3.8e-62:324:95//Hs.124109:AA888839
R-NT2RM4001828//ESTs//1.3e-119:563:98//Hs.102397:AA706551
R-NT2RM4001836//ESTs//5.5e-16:92:100//Hs.26996:AA551070
R-NT2RM4001841//ESTs//1.3e-99:540:94//Hs.42322:AA082619
R-NT2RM4001842//ESTs, Weakly similar to !!!! ALU SUBFAMILY SQ WARNING ENTRY !!!! [H.sapiens]//4.1e-10:274:62//Hs.161959:AA493652
R-NT2RM4001856//ESTs, Weakly similar to contains similarity to ATP/GTP-binding site motif [C.elegans]//3.0e-43:292:86//Hs.14202:N46000
R-nnnnnnnnnnnn//ESTs//6.2e-104:495:98//Hs.118686:AA682280
R-NT2RM40018657/Homo sapiens mRNA for atopy related autoantigen CALC//1.6e-120:592:97//Hs.61628:Y17711
R-NT2RM4001876//ESTs//2.9e-98:532:92//Hs.100734:AA158252
R-NT2RM4001880//ESTs//2.5e-29:224:86//Hs.6193:AA045149
R-NT2RM4001905//ESTs//5.6e-109:565:95//Hs.9536:AA114178
R-NT2RM4001922//ESTs, Weakly similar to !!!! ALU SUBFAMILY J WARNING ENTRY !!!! [H.sapiens]//1.2e-105:535:95//Hs.30991:AA994438
R-NT2RM4001930//ESTs//4.1-84:425:96//Hs.80042:N63143
R-NT2RM4001938//EST//0.00040:241:60//Hs.147235:AI205893
R-NT2RM4001940//Homo sapiens timeless homolog mRNA, complete cds//2.0e-110:556:95//Hs.118631:AF098162
R-NT2RM4001953//ESTs//5.3e-65:338:96//Hs.33718:AA453268
R-NT2RM4001965//ESTs, Weakly similar to T14B4.2 gene product [C.elegans]//5.7e-62:326:95//Hs.3385:N25917
R-nnnnnnnnnnnn//ESTs, Weakly similar to IP63 protein [R.norvegicus]//1.9e-21:121:98//Hs.8772:AA521097
R-NT2RM4001979//ESTs//1.4e-96:465:98//Hs.157103:W60265
R-NT2RM4001984
R-NT2RM4001987
R-NT2RM4002013//EST//2.2e-14:110:90//Hs.160835:AI345528
R-NT2RM4002018
R-NT2RM4002034//Human mRNA for KIAA0118 gene, partial cds//9.4e-46:293:87//Hs.154326:D42087
R-NT2RM4002044//ESTs//2.8e-107:537:96//Hs.24078:W44435
R-NT2RM4002054//ESTs//3.7e-88:482:94//Hs.4243:T78226
R-NT2RM4002062//ESTs//1.4e-55:377:85//Hs.152592:AA587887
R-NT2RM4002063//Calcium modulating ligand//1.8e-43:385:78//Hs.13572:AF068179
R-nnnnnnnnnnnn//Homo sapiens OPA-containing protein mRNA, complete cds//5.5e-42:554:68//Hs.85313:AF071309
R-NT2RM4002067//Human kpni repeat mrna (cdna clone pcd-kpni-4), 3' end//2.3e-43:468:73//Hs.139107:K00629
R-NT2RM4002073//ESTs, Weakly similar to very-long-chain acyl-CoA synthetase [H.sapiens]//6.8e-57:290:96//Hs.109274:AA193416
R-NT2RM4002075//ESTs//0.078:267:61//Hs.163563:AA641655
R-NT2RM4002093//ESTs//1.2e-64:316:99//Hs.34956:AI052528
R-nnnnnnnnnnnn//ESTs//1.0:95:69//Hs.25897:W65409
R-NT2RM4002128//Homo sapiens mRNA for BCL9 gene//0.51:258:60//Hs.122607:Y13620
R-NT2RM4002140//ESTs//5.5e-46:187:94//Hs.8737:W22712
R-NT2RM4002145//ESTs//4.6e-70:374:94//Hs.141082:H18987
R-NT2RM4002146//ESTs//1.9e-93:43 9:99//Hs.119295:AA442090
R-NT2RM4002161//Homo sapiens laforin (EPM2A) mRNA, partial cds//1.5e-111:560:96//Hs.22464:AF084535
R-NT2RM4002174//Homo sapiens LIM protein mRNA, complete cds//3.2e-46:552:72//Hs.154103:AF061258
R-NT2RM4002189//ESTs//9.6e-75:352:100//Hs.98350:H15400
R-NT2RM4002194//EST//0.22:68:72//Hs.149104:AI244343
R-NT2RM4002205//EST//0.00028:103:72//Hs.130032:AA897678
R-NT2RM4002213//ESTs//3.3e-15:160:78//Hs.63304:W22079
R-NT2RM4002226//ESTs, Highly similar to GTPASE ACTIVATING PROTEIN ROTUND [Drosophila melanogaster]//5.1e-112:569:95//Hs.23900:U82984
R-NT2RM4002251//ESTs, Weakly similar to similar to alpha-1,3-mannosyl-glycoprotein beta-1, 2-N-acetylglucosaminyltransferase [C.elegans]//1.1e-100:544:93//Hs.27567:W72190
R-NT2RM4002256//Small inducible cytokine A5 (RANTES)//1.0e-44:341:81//Hs.155464:AF088219
R-NT2RM4002266//ESTs//2.6e-100:539:93//Hs.57976:AA535864
R-NT2RM4002278//ESTs//1.8e-112:569:95//Hs.87281:AA128263
R-NT2RM4002281//ESTs//4.9e-20:187:80//Hs.141203:H52638
R-NT2RM4002287//ESTs//7.9e-84:388:94//Hs.33977:N52461
R-NT2RM4002294
R-NT2RM4002301//ESTs//4.5e-111:556:96//Hs.85916:AA194164
R-NT2RM4002323//ESTs//4.5e-102:498:97//Hs.85782:AA191498
R-nnnnnnnnnnnn//ESTs//5.0e-59:283:100//Hs.125048:AA682913
R-NT2RM4002344//V-akt murine thymoma viral oncogene homolog 2//0.29:153:66//Hs.155129:M77198
R-NT2RM4002373//Homo sapiens mRNA for KIAA0649 protein, complete cds//2.8e-122:593:97//Hs.26163:AB014549
R-NT2RM4002374//ESTs//3.3e-40:505:70//Hs.95115:AA206594
R-NT2RM4002383//ESTs//2.7e-93:455:97//Hs.134278:AA648884
R-NT2RM4002390//ESTs//3.3e-93:481:95//Hs.48764:AA613328
R-NT2RM4002409//ESTs, Weakly similar to coded for by C. elegans cDNA yk52e10.5 [C.elegans]//1.3e-97:473:98//Hs.16464:W19606
R-NT2RM4002438//ESTs//0.74:162:61//Hs.65377:AA994677
R-NT2RM4002446
R-NT2RM4002452//EST//1.0:164:60//Hs.1166l9:AA668142
R-NT2RM4002457
R-NT2RM4002460//ESTs//3.0e-74:385:96//Hs.6933:R07890
R-NT2RM4002479//Homo sapiens RNA helicase-related protein mRNA, complete cds//1.6e-103:507:97//Hs.8765:AF083255
R-NT2RM4002482//Homo sapiens mRNA for KIAA0691 protein, complete cds//2.3e-32:172:98//Hs.94781:AB014591
R-NT2RM4002493//ESTs//6.4e-73:366:97//Hs.157114:T58884
R-NT2RM4002499//ESTs//3.5e-61:307:97//Hs.117737:AI088029
R-NT2RM4002504//ESTs//2.1e-55:306:94//Hs.10949:AA464464
R-nnnnnnnnnnnn//ESTs, Weakly similar to peroxisome targeting signal 2 receptor [H.sapiens]//1.4e-73:360:91//Hs.31030:H50467
R-NT2RM4002532//ESTs//1.3e-21:191:78//Hs.146811:AA410788
R-NT2RM4002534//ESTs//1.8e-99:512:95//Hs.13526:AI417057
R-NT2RM4002567//ESTs//7.6e-41:272:87//Hs.7114:R24312
R-NT2RM4002571//ESTs, Highly similar to POLYPEPTIDE N-ACETYLGALACTOSAMINYLTRANSFERASE [Bos taurus]//2.3e-89:435:97//Hs.15830:AA165698
R-NT2RM4002593//ESTs//2.3e-109:552:96//Hs.17424:AA190569
R-NT2RM4002623//ESTs, Weakly similar to ASPARTYL-TRNA SYNTHETASE [Thermus aquaticus thermophilus]//9.6e-28:194:87//Hs.59346:AI126802
R-NT2RP2000001//ESTs//2.6e-80:386:99//Hs.105061:N45096
R-NT2RP2000006//Thromboxane A2 receptor//7.2e-37:253:84//Hs.89887:D38081
R-NT2RP2000008//Zinc finger protein 37a (KOX 21)//5.2e-25:366:67//Hs.54488:X69115
R-NT2RP2000027//ESTs//9.5e-74:377:96//Hs.96557:AA286713
R-NT2RP2000040//Homo sapiens mRNA for KIAA0747 protein, partial cds//2.7e-42:223:96//Hs.8309:AB018290
R-NT2RP2000045//Homo sapiens tumorous imaginal discs protein Tid56 homolog (TID1) mRNA, complete cds//4.3e-64:309:98//Hs.6216:AF061749
R-NT2RP2000054//EST//1.2e-71:375:96//Hs.98835:AA435798
R-NT2RP2000056//EST//2.8e-28:342:69//Hs.135526:AI094910
R-NT2RP2000067//ESTs, Weakly similar to tenascin-like protein [D.melanogaster]//2.3e-35:199:94//Hs.41793:AA775879
R-NT2RP2000070//ESTs, Weakly similar to proto-cadherin 3 [R.norvegicus]//1.4e-78:383:98//Hs.58254:W72881
R-NT2RP2000076//EST//0.0014:227:63//Hs.136761:AA738097
R-NT2RP2000077//Homo sapiens growth arrest specific 11 (GAS11) mRNA, complete cds//1.1e-78:379:97//Hs.54877:AF050078
R-NT2RP2000079//Homo sapiens RET finger protein-like 1 antisense transcript, partial//2.9e-21:232:75//Hs.102576:AJ010230
R-NT2RP2000088//Homo sapiens mRNA for KIAA0795 protein, partial cds//1.8e-75:378:96//Hs.22926:AB018338
R-NT2RP2000091//Carcinoembryonic antigen gene family member 6//0.030:236:63//Hs.41:D90064
R-NT2RP2000097//ESTs//4.2e-15:92:97//Hs.7432:AA281757
R-NT2RP2000098//ESTs//9.0e-53:279:94//Hs.87807:AA813827
R-NT2RP2000108//EST//1.5e-75:378:96//Hs.162105:AA524419
R-NT2RP2000114//Homo sapiens mRNA for GM3 synthase, complete cds//5.8e-76:386:95//Hs.17706:AB018356
R-NT2RP2000120//ESTs, Weakly similar to HYPOTHETICAL 68.7 KD PROTEIN ZK757.1 IN CHROMOSOME III [C.elegans]//1.9e-19:153:86//Hs.5268:W22670
R-nnnnnnnnnn//ESTs//1.0e-55:293:95//Hs.14570:AI422099
R-nnnnnnnnnnnn//ESTs//0.24:354:59//Hs.157564:AI356513
R-NT2RP2000147//ESTs, Highly similar to CLATHRIN COAT ASSEMBLY PROTEIN AP47 [Mus musculus]//3.0e-89:457:95//Hs.3832:AI208601
R-NT2RP2000153//EST//0.0039:93:68//Hs.140386:AA773548
R-NT2RP2000157//ESTs//1.1e-53:322:91//Hs.6877:AA040820
R-NT2RP2000161//EST5//1.6e-99:492:97//Hs.21738:AI188190
R-NT2RP2000175//ESTs//1.4e-98:489:96//Hs.4849:AI143741
R-NT2RP2000183//ESTs//9.0e-72:358:96//Hs.4856:N51373
R-NT2RP2000195//ESTs//3.9e-92:439:98//Hs.145091:AA814510
R-NT2RP2000205//ESTs, Moderately similar to !!!! ALU SUBFAMILY J WARNING ENTRY !!!! [H.sapiens]//1.4e-80:415:95//Hs.11807:T86897
R-NT2RP2000224//RNA polymerase II, polypeptide C (33kD)//1.1e-57:306:94//Hs.79402:AC004382
R-NT2RP2000232
R-NT2RP2000233//ESTs//1.1e-08:63:96//Hs.124861:AI090683
R-NT2RP2000239//ESTs//5.3e-87:427:96//Hs.86211:AA604379
R-NT2RP2000248//ESTs, Weakly similar to O-linked GlcNAc transferase [H.sapiens]//1.3e-95:454:99//Hs.102057:AA649005
R-NT2RP2000257//ESTs//5.1e-58:282:99//Hs.122565:AI126840
R-NT2RP2000258//EST//1.0:67:68//Hs.61812:AA035649
R-NT2RP2000270//ESTs, Weakly similar to LINE-1 REVERSE TRANSCRIPTASE HOMOLOG [Homo sapiens]//8.4e-59:298:96//Hs.16085:AI261382
R-NT2RP2000274//ESTs//7.5e-61:296:98//Hs.86081:AA196635
R-NT2RP2000288//ESTs//1.8e-56:305:93//Hs.7579:AA775865
R-NT2RP2000289
R-NT2RP2000297//ESTs, Highly similar to MKR2 PROTEIN [Mus musculus]//9.8e-106:494:99//Hs.102951:AA574249
R-NT2RP2000298//ESTs//2.1e-62:256:90//Hs.8737:W22712
R-NT2RP2000310//Human proline dehydrogenase/proline oxidase (PRODH) mRNA, complete cds//2.8e-39:222:93//Hs.58218:U82381
R-NT2RP2000327//Homo sapiens DNA sequence from PAC 434014 on chromosome 1q32.3.-41. Contains the HSD11B1 gene for Hydroxysteroid (11-beta) Dehydrogenase 1, the ADORA2BP adenosine A2b receptor LIKE pseudogene, the IRF6 gene for Interferon Regulatory Factor 6 and two unknown genes. Contains ESTs and GSSs//2.9e-71:342:98//Hs.87684:AL022398
R-NT2RP2000329//ESTs, Highly similar to GTP:AMP PHOSPHOTRANSFERASE MITOCHONDRIAL [Bos taurus]//3.4e-69:371:94//Hs.43436:N32441
R-NT2RP2000337//ESTs//5.2e-79:411:95//Hs.101799:AI276062
R-NT2RP2000346//Homo sapiens apoptosis associated protein (GADD34) mRNA, complete cds//1.1e-47:262:94//Hs.76556:U83981
R-NT2RP2000369//ESTs//4.3e-102:531:94//Hs.15855:H98103
R-NT2RP2000414//Homo sapiens HnRNP F protein mRNA, complete cds//8.4e-09:93:83//Hs.808:L28010
R-NT2RP2000420//ESTs//8.2e-24:142:94//Hs.144893:AI222324
R-NT2RP2000422//Homo sapiens N-acetylglucosamine-phosphate mutase mRNA, complete cds//4.2e-20:140:90//Hs.5819:AF102265
R-NT2RP2000438//ESTs, Weakly similar to misato [D.melanogaster]//1.3e-65:362:93//Hs.22197:AI151425
R-NT2RP2000448//ESTs, Highly similar to HYPOTHETICAL 51.6 KD PROTEIN IN PAP1-MRPL13 INTERGENIC REGION [Saccharomyces cerevisiae]//3.6e-75:435:92//Hs.21938:W81045
R-NT2RP2000459//ESTs//2.8e-95:527:93//Hs.103422:AI352013
R-NT2RP2000498//ESTs//2.3e-17:119:79//Hs.161714:AA229078
R-NT2RP2000503//ESTs//5.2e-91:438:98//Hs.152335:AI290215
R-NT2RP2000510//Homo sapiens KIAA0436 mRNA, partial cds//0.13:455:58//Hs.110:AB007896
R-nnnnnnnnnn//ESTs//9.9e-63:376:89//Hs.47546:AA181348
R-NT2RP2000523
R-NT2RP2000603//Homo sapiens mRNA for KIAA0572 protein, partial cds//3.5e-30:167:97//Hs.14409:AB011144
R-NT2RP2000617//ESTs//9.5e-103:493:98//Hs.9412:W72446
R-NT2RP2000634//Homo sapiens mRNA for KIAA0614 protein, partial cds//8.1e-66:335:96//Hs.7314:AB014514
R-NT2RP2000644//ESTs//1.1e-18:372:63//Hs.82419:AA789222
R-NT2RP2000656//ESTs//1.0e-10:128:80//Hs.23977:AA115275
R-NT2RP2000658//ESTs//0.31:278:59//Hs.15661:W02396
R-NT2RP2000668//ESTs//8.2e-40:255:88//Hs.113310:R16767
R-NT2RP2000678//ESTs//2.6e-53:271:9611Hs.23790:N99347
R-NT2RP2000710//ESTs//0.49:190:63//Hs.145521:AI261368
R-NT2RP2000715//EST//1.2e-87:418:9911Hs.139425:AA429279
R-NT2RP2000731//EST//5.3e-65:322:97//Hs.136754:AA713965
R-NT2RP2000758//ESTS//1.0:187:61//Hs.10545:N62642
R-NT2RP2000764//ESTs//5.8e-84:485:91//Hs.121816:AA775419
R-NT2RP2000809
R-NT2RP2000812//ESTs//1.2e-45:231:97//Hs.121028:AA902745
R-nnnnnnnnnnnn//ESTs//6.3e-87:433:97//Hs.145479:AA969404
R-NT2RP2000816//ESTS//0.45:100:69//Hs.147529:AA458918
R-NT2RP2000819
R-NT2RP2000841//ESTs//1.9e-73:351:99//Hs.116385:AI224511
R-NT2RP2000842//TUMOR NECROSIS FACTOR-INDUCIBLE PROTEIN TSG-6
PRECURSOR//4.6e-10:247:66//Hs.29352:M31165
R-NT2RP2000845//ESTs//2.8e-91:443:97//Hs.66810:AI206552
R-NT2RP2000863//ESTs//4.3e-49:310:88//Hs.104336:W07345
R-NT2RP2000880//Homo sapiens mRNA for KIAA0741 protein, complete cds//2.8e-43:277:89//Hs.3615:AB018284
R-NT2RP2000892//ESTs//2.8e-50:25 8:96//Hs.119238:AA476267
R-NT2RP2000931//MATRIN 3//7.2e-57:290:96//Hs.78825:AB018266
R-NT2RP2000938//ESTs, Highly similar to HYPOTHETICAL 6.3 KD PROTEIN ZK652.2 IN CHROMOSOME III [Caenorhabditis elegans]//3.9e-37:199:95//Hs.112318:AA186477
R-NT2RP2000943//Homo sapiens mRNA for KIAA0755 protein, complete cds//9.8e-98:494:96//Hs.19822:AB018298
R-NT2RP2000965//EST//0.22:223:60//Hs.105703:AA487021
R-NT2RP2000970//EST//8-7e-06:255:62//Hs.149202:AI246481
R-NT2RP2000985//ESTs, Weakly similar to HYPOTHETICAL 96.8 KD PROTEIN IN SIS2-MTD1 INTERGENIC REGION [S.cerevisiae]//7.8e-92:468:95//Hs.12124:AA522537
R-NT2RP2000987//ESTs//4.5e-78:419:93//Hs.21968:H97521
R-NT2RP2001036//EST//2.0e-33:148:82//Hs.163196:AA767643
R-NT2RP2001044//ESTs//5.6e-95:493:95//Hs.21958:AA453660
R-NT2RP2001065//ESTs//3.6e-28:153:96//Hs.119314:AA432108
R-NT2RP2001070//EST//0.30:94:67//Hs.94289:N73665
R-NT2RP2001094//EST//0.75:101:69//Hs.161040:H82068
R-NT2RP2001119
R-NT2RP2001127//Homa sapiens mRNA for HRIHFB2060, partial cds//1.5e-56:304:94//Hs.146282:AB015348
R-NT2RP2001137
R-NT2RP2001149//ESTs//5.1e-66:324:9711Hs.27475:AA704512
R-NT2RP2001168//ESTs//2.0e-98:539:92//Hs.77870:AI188145
R-NT2RP2001173//Homo sapiens mRNA for KIAA0480 protein, complete cds//1.5e-96:490:96//Hs.26247:AB007949
R-NT2RP2001174//ESTs//2.2e-63:354:93//Hs.24266:R28287
R-NT2RP2001196//ESTs//1.4e-83:463:93//Hs.124304:AA825510
R-NT2RP2001218//ESTs//1.4e-100:506:96//Hs.93391:AI188402
R-NT2RP2001226//EST//0.0074:154:63//Hs.128612:AA909358
R-NT2RP2001233/TESTs, Highly similar to ZINC FINGER PROTEIN ZFP-36 [Homo sapiens]//3.7e-65:538:80//Hs.44014:AA632298
R-NT2RP2001245//ESTs//5.2e-90:447:97//Hs.14559:H92996
R-NT2RP2001268//Homo sapiens mRNA for KIAA0810 protein, partial cds//1.5e-112:544:97//Hs.7531:AB018353
R-NT2RP2001277//ESTs//2.0e-81:387:99//Hs.13751:AA908229
R-NT2RP2001290//ESTs//2.4e-91:501:92//Hs.12600:AA044775
R-NT2RP2001295//ESTs//1.4e-70:337:99//Hs.123854:AA412665
R-NT2RP2001312//ESTs//4.6e-53:276:95//Hs.7961:AA401205
R-NT2RP2001327//ESTs, Moderately similar to tumor necrosis factor-alpha-induced protein B12 [H.sapiens]//2.3e-43:238:93//Hs.106632:N25679
R-NT2RP2001328//ESTs//5.1e-99:499:96//Hs.34868:AI341138
R-NT2RP2001347//ESTs//6.7e-05:100:77//Hs.9536:AA114178
R-NT2RP2001378//ESTs//4.2e-83:456:93//Hs.10554:N50028
R-NT2RP2001381//ESTs//1.1e-26:148:96//Hs.161859:AA444038
R-NT2RP2001392//ESTs, Weakly similar to MITOCHONDRIAL LON PROTEASE HOMOLOG PRECURSOR [H.sapiens]//3.9e-74:411:93//Hs.47305:AA195153
R-NT2RP2001394//ESTs//9.5e-54:305:93//Hs.70256:R07875
R-NT2RP2001397//ESTs, Highly similar to G2/MITOTIC-SPECIFIC CYCLIN B2 [Mesocricetus auratus]//5.2e-97:469:97//Hs.20483:AA522505
R-NT2RP2001420//ESTs//1.6e-49:228:88//Hs.163602:N32030
R-NT2RP2001423//ESTs//2.0e-37:190:99//Hs.101565:R35431
R-NT2RP2001427//EST//1.7e-1 1:107:84//Hs.148584:AI201728
R-NT2RP2001436//ESTs, Weakly similar to F02D8.3 [C.elegans]//2.9e-114:558:97//Hs.7627:AI341556
R-NT2RP2001440//EST//0.17:192:58//Hs.133442:AI061394
R-NT2RP2001445//ESTs//1.1e-43:215:100//Hs.145497:AA501453
R-NT2RP2001449//ESTs//4.1e-08:234:61//Hs.134067:AI076765
R-NT2RP2001450//ESTs//9.5e-65:356:94//Hs.61829:AI079539
R-NT2RP2001467//Small inducible cytokine A5 (RANTES)//1.2e-34:255:83//Hs.155464:AF088219
R-NT2RP2001506//ESTs//2.9e-23:170:88//Hs.7147:T23513
R-NT2RP2001511//ESTs//2.0e-08:59:100//Hs.57660:AA251146
R-NT2RP2001520//Homo sapiens mRNA for mitochondrial carrier protein ARALAR1//6.7e-106:545:95//Hs.4277:Y14494
R-NT2RP2001526//ESTs//3.7e-23:295:72//Hs.8514:AF039240
R-NT2RP2001536//Homo sapiens X-ray repair cross-complementing protein 3 (XRCC3) mRNA, complete cds//1.9e-15:99:95//Hs.99742:AF035586
R-NT2RP2001560//ESTs//2.2e-58:310:94//Hs.87454:AA732816
R-NT2RP2001569//Homo sapiens mRNA, chromosome 1 specific transcript KIAA0488//2.0e-76:387:96//Hs.67619:AB007957
R-NT2RP2001576//Human mRNA for KIAA0105 gene, complete cds//0.17:193:60//Hs.119:D14661
R-NT2RP2001581//ESTs//5.1e-08:107:78//Hs.157114:T58884
R-NT2RP2001597//EST//5.2e-22:151:88//Hs.158613:AI369995
R-NT2RP2001601//ESTs//1.5e-78:373:99//Hs.137558:AI393767
R-NT2RP2001613
R-NT2RP2001628//EST//0.99:195:60//Hs.144238:W52294
R-NT2RP2001663//ESTs//4.0e-37:282:84//Hs.12319:W56090
R-NT2RP2001677//ESTs//1.4e-44:232:96//Hs.159387:AI370845
R-NT2RP2001678//ESTs//0.91:124:60//Hs.10593:AI201336
R-NT2RP2001699//EST//0.0033:230:61//Hs.146544:AI125323
R-NT2RP2001720//ESTs//1.8e-52:255:99//Hs.101064:AA290579
R-NT2RP2001721//ESTs//7.0e-101:479:99//Hs.129750:AA987538
R-NT2RP2001740//ESTs//3.3e-76:379:96//Hs.144704:AI147100
R-NT2RP2001748//ESTs//1.4e-44:352:81//Hs.142259:AA828840
R-NT2RP2001762//Homo sapiens exonuclease 1a (EXO1a) mRNA, complete cds//2.1e-105:519:96//Hs.47504:AF091754
R-NT2RP2001813//ESTs//6.3e-78:406:95//Hs.21902:R44037
R-NT2RP2001861
R-NT2RP2001869//EST//2.8e-21:173:82//Hs.130321:AI002941
R-NT2RP2001876//ESTs//6.1e-102:526:95//Hs.4944:AA533088
R-NT2RP2001883//ESTs, Weakly similar to No definition line found [C.elegans]//6.9e-110:556:95//Hs.23159:AA113849
R-NT2RP2001900//ESTs//6.9e-85:442:95//Hs.154220:AA171724
R-NT2RP2001907//ESTs//2.1e-82:432:94//Hs.142257:AA188423
R-NT2RP2001926//EST//2.3e-24:299:71//Hs.135085:AI097268
R-NT2RP2001936//ESTs//1.1e-45:265:92//Hs.112482:T66087
R-NT2RP2001943//EST//1.4e-05:246:61//Hs.144096:AI032180
R-NT2RP2001946//ESTs//3.6e-87:410:99//Hs.20242:W72594
R-NT2RP2001947//ESTs//1.9e-55:338:88//Hs.58582:T72588
R-NT2RP2001969
R-NT2RP2001976//ESTs//1.2e-98:499:95//Hs.121028:AA902745
R-NT2RP2001985//ESTs, Weakly similar to GTPASE-ACTIVATING PROTEIN SPA-1 [M.musculus]//8.3e-15:118:89//Hs.18760:AA166678
R-NT2RP2002025//ESTs//2.1e-82:393:98//Hs.159488:AI378233
R-NT2RP2002032//ESTs//4.4e-98:531:91//Hs.93836:AA813332
R-NT2RP2002033//ESTs//3.5e-43:229:96//Hs.30563:AA102627
R-NT2RP2002041
R-NT2RP2002046//ESTs//1.6e-101:476:99//Hs.101107:AA825938
R-NT2RP2002047//ESTs//9.1e-85:431:95//Hs.116750:AA629895
R-NT2RP2002058//ESTs//1.3e-31:163:99//Hs.33085:AA258068
R-NT2RP2002066//ESTS//1.9e-87:459:93//Hs.118871:AA846091
R-NT2RP2002070//ESTs//4.1e-63:332:96//Hs.156446:T92265
R-NT2RP2002076//Homo sapiens clone 24804 mRNA sequence//1.7e-26:178:87//Hs.11039:AF052183
R-NT2RP2002079//ESTs//1.2e-79:389:97//Hs.135214:AI350524
R-NT2RP2002099//Homo sapiens mRNA for E1B-55kDa-associated protein//1.5e-60:376:89//Hs.155218:AJ007509
R-NT2RP2002105//ESTs//8.4e-54:313:90//Hs.98702:AI123000
R-NT2RP2002124//ESTs//6.6e-81:431:93//Hs.127326:AA525134
R-NT2RP2002137//Deoxycytidine kinase//0.29:183:62//Hs.709:M60527
R-NT2RP2002154//ESTs//9.6e-97:539:91//Hs.18624:AA523268
R-NT2RP2002172//EST//0.69:53:75//Hs.156238:AI334495
R-NT2RP2002185//ESTs, Weakly similar to F15C11.2 [C.elegans]//1.4e-54:269:98//Hs.107201:W52859
R-NT2RP2002192//ESTs, Moderately similar to !!!! ALU SUBFAMILY J WARNING ENTRY !!!! [H.sapiens]//3.9e-15:245:71//Hs.87578:AI125363
R-NT2RP2002193//ESTs//3.5e-79:45 3:90//Hs.76578:AI290672
R-NT2RP2002208//ESTs//2.0e-72:347:99//Hs.164028:AI003946
R-NT2RP2002219//EST//0.039:229:63//Hs.149830:AI287499
R-NT2RP2002231//ESTs//3.3e-64:337:94//Hs.79828:AA642341
R-nnnnnnnnnnnn//ESTs, Highly similar to co-repressor protein [M.musculus]//5.4e-48:238:99//Hs.22583:AA188168
R-NT2RP2002256//Homo sapiens retinoic acid hydroxylase mRNA, complete cds//1.6e-15:131:83//Hs.150595:AF005418
R-NT2RP2002259//Human L-myc protein gene, complete cds//5.3e-99:548:91//Hs.92137:M19720
R-NT2RP2002270//ESTs, Weakly similar to AF-9 PROTEIN [H.sapiens]//4.8e-100:550:91//Hs.4029:Z78373
R-NT2RP2002292//ESTs, Weakly similar to F13B12.1 [C.elegans]//3.2e-92:482:93//Hs.5570:AI377863
R-NT2RP2002312//Homo sapiens CDP-diacylglycerol synthase 2 (CDS2) mRNA, partial cds//4.1e-103:527:94//Hs.24812:AF069532
R-NT2RP2002316//ESTs//4.2e-91:425:100//Hs.3350:AI368015
R-NT2RP2002325//Homo sapiens peroxisomal biogenesis factor (PEX11a) mRNA, complete cds//1.2e-112:567:95//Hs.31034:AB015594
R-NT2RP2002333//ESTs//1.9e-86:483:91//Hs.155198:AA767372
R-NT2RP2002385//Homo sapiens synaptic glycoprotein SC2 spliced variant mRNA, complete cds//1.2e-103:600:89//Hs.109051:AF038958
R-NT2RP2002394//ESTS//0.11:158:65//Hs.28792:AI343467
R-NT2RP2002408//ESTs//1.5e-51:278:93//Hs.6044:W22815
R-NT2RP2002426//Homo sapiens mRNA for KIAA0563 protein, complete cds//1.7e-33:285:80//Hs.15731:AB011135
R-NT2RP2002439//ESTS//3.2e-12:134:76//Hs.32246:AA464020
R-NT2RP2002457//ESTs//4.7e-52:282:94//Hs.21968:H97521
R-NT2RP2002464//ESTs//5.3e-27:148:98//Hs.115660:AI362230
R-NT2RP2002475//ESTs//3.9e-85:439:94//Hs.9873:W27233
R-nnnnnnnnnnnn//Homo sapiens mRNA for ABC transporter 7 protein, complete cds//9.9e-115:605:92//Hs.125856:AB005289
R-NT2RP2002498//ESTs//6.3e-37:227:93//Hs.108779:N73180
R-NT2RP2002503//ESTs//1.9e-54:358:86//Hs.57800:W60838
R-NT2RP2002504//Homo sapiens mRNA for KIAA0791 protein, complete cds//8.5e-107:583:91//Hs.23255:AB018334
R-NT2RP2002520//ESTs//4.2e-99:509:94//Hs.32368:AA205305
R-NT2RP2002537//ESTs//4.2e-105:552:93//Hs.154363:AA533090
R-NT2RP2002546//Homo sapiens clone TUA8 Cri-du-chat region mRNA//2.6e-109:570:93//Hs.49476:AF009314
R-NT2RP2002549//DNA polymerase gamma//1.1e-35:189:86//Hs.80961:U60325
R-NT2RP2002591//ESTs, Weakly similar to ZINC FINGER PROTEIN 84 [H.sapiens]//7.5e-118:564:97//Hs.94549:AA149547
R-NT2RP2002595//EST//1.4e-15:101:95//Hs.129528:AA994783
R-NT2RP2002606//ESTs//4.5e-99:475:98//Hs.45046:N40170
R-NT2RP2002609//ESTs//1.9e-104:568:92//Hs.9175:AI184220
R-NT2RP2002618//ESTs//0.014:493:57//Hs.96322:AA541615
R-NT2RP2002621//EST//4.4e-36:252:84//Hs.149580:AI281881
R-NT2RP2002643//ESTs//6.9e-32:247:74//Hs.33354:AA179944
R-NT2RP2002672
R-NT2RP2002701//N-acetylglucosaminidase, alpha- (Sanfilippo disease IIIB//0.99:184:63//Hs.50727:U43572
R-NT2RP2002706//EST//2.8e-41:148:86//Hs.161917:AA483223
R-NT2RP2002710//EST//0.34:105:71//Hs.136747:AA749210
R-NT2RP2002727//ESTs//8.7e-68:368:94//Hs.14366:T78626
R-NT2RP2002736//ESTs//9.7e-98:457:99//Hs.74899:AA993300
R-NT2RP2002740//Homo sapiens mRNA for KIAA0536 protein, partial cds//0.66:360:59//Hs.119139:AB011108
R-NT2RP2002741//ESTs//3.1e-102:489:98//Hs.112024:AI042352
R-NT2RP2002750//EST//3.6e-43:166:86//Hs.162404:AA573131
R-NT2RP2002752//ESTs//5.0e-56:355:89//Hs.95867:M62042
R-NT2RP2002753//ESTs//1.7e-49:262:96//Hs.49005:W89124
R-NT2RP2002769//ESTs//1.3e-59:376:88//Hs.4046:H03587
R-NT2RP2002778//Homo sapiens clone 24606 mRNA sequence//4.0e-65:341:94//Hs.17481:AF070537
R-NT2RP2002800//ESTs//6.5e-08:79:84//Hs.153262:AA551124
R-NT2RP2002839//ESTs, Moderately similar to !!!! ALU SUBFAMILY J WARNING ENTRY !!!! [H.sapiens]//1.6e-100:501:97//Hs.136202:AA206578
R-NT2RP2002857//ESTs//4.3e-94:463:97//Hs.134292:AA603031
R-NT2RP2002862//ESTs//2.3e-42:302:82//Hs.117969:H94870
R-NT2RP2002880
R-NT2RP2002891
R-NT2RP2002925//ESTs//1.3e-103:564:92//Hs.142079:AA182894
R-NT2RP2002928//ESTs//3.9e-108:502:99//Hs.29105:AA574143
R-NT2RP2002929//ESTs//4.1e-106:499:99//Hs.44743:AA837096
R-NT2RP2002954//ESTs//2.6e-88:417:99//Hs.100824:AI308771
R-NT2RP2002959//ESTs//7.5e-101:489:97//Hs.32690:N57480
R-NT2RP2002979//ESTs//5.4e-06:197:65//Hs.146726:AI147060
R-NT2RP2002980//ESTs//1.0e-110:562:96//Hs.28444:AA083213
R-NT2RP2002986//ESTs, Highly similar to RING CANAL PROTEIN [Drosophila melanogaster]//3.1e-119:578:97//Hs.106290:AI125291
R-NT2RP2002987//Human mRNA for KIAA0331 gene, complete cds//1.0:78:74//Hs.146395:AB002329
R-NT2RP2002993//ESTS, Weakly similar to DNA-DIRECTED RNA POLYMERASE II 140 KD POLYPEPTIDE [H.sapiens]//2.4e-98:467:98//Hs.86337:AA149311
R-NT2RP2003000//ESTs//0.0070:400:61//Hs.138506:U85642
R-NT2RP2003034//ESTs//9.3e-87:408:96//Hs.164042:H12594
R-NT2RP2003073//Human transporter protein (g17) mRNA, complete cds//0.95:259:61//Hs.76460:U49082
R-NT2RP2003099//Thromboxane A2 receptor//2.6e-42:328:81//Hs.89887:D38081
R-NT2RP2003108//ESTs//2.3e-82:398:98//Hs.5105:AA115512
R-NT2RP2003117//Human mRNA for KIAA0347 gene, complete cds//2.4e-49:336:86//Hs.101996:AB002345
R-NT2RP2003121//ESTs//2.0e-75:380:96//Hs.133127:AA133355
R-NT2RP2003125
R-NT2RP2003129//EST//0.68:115:69//Hs.122196:AA780986
R-NT2RP2003137//ESTs//2.1e-37:259:85//Hs.63169:N78506
R-NT2RP2003161//ESTs//2.5e-88:451:96//Hs.29041:W37379
R-NT2RP2003164//ESTs//4.3e-113:543:97//Hs.8980:AA629067
R-NT2RP2003165//ESTs//6.9e-83:486:89//Hs.138632:H97952
R-NT2RP2003177//ESTs//0.47:38:100//Hs.61790:AA421156
R-NT2RP2003194//ESTs//4.7e-118:582:96//Hs.27266:AA053816
R-NT2RP2003206//ESTs//0.032:388:58//Hs.122148:AA442074
R-NT2RP2003230//ESTs//8.8e-103:478:99//Hs.40140:AI079253
R-NT2RP2003237//ESTs//2.7e-76:392:96//Hs.106278:R37661
R-NT2RP2003243//ESTs//3.6e-53:300:92//Rs.18793:AA192438
R-NT2RP2003265//ESTs, Highly similar to protein NGD5 [M.musculus]//3.3e-110:557:96//Hs.24994:AA236937
R-NT2RP2003272//ESTs, Weakly similar to F15C11.2 [C.elegans]//1.2e-34:228:89//Hs.107201:W52859
R-NT2RP2003277//Homo sapiens mRNA for KIAA0625 protein, partial cds//1.4e-111:565:95//Hs.1549l9:AB014525
R-NT2RP2003280//ESTs//2.6e-101:541:94//Hs.6982:AA622427
R-NT2RP2003286//ESTs//1.2e-104:497:98//Hs.113052:AI222106
R-NT2RP2003293//Human mRNA for KIAA0118 gene, partial cds//9.1e-44:458:74//Hs.154326:D42087
R-NT2RP2003295//Protein serine/threonine kinase stk2//0.31:321:57//Hs.1087:L20321
R-NT2RP2003297//ESTs//3.0e-15:118:87//Hs.16621:AA098874
R-NT2RP2003308//ESTs, Moderately similar to CROOKED NECK PROTEIN [Drosophila melanogaster]//4.8e-109:553:96//Hs.26089:AA195126
R-NT2RP2003329//ESTs//0.99:208:62//Hs.143607:AI424948
R-NT2RP2003339//ESTs//1.3e-85:441:96//Rs.24115:N32618
R-NT2RP2003347//ESTs//1.5e-70:365:96//Hs.155773:AI312825
R-NT2RP2003367//EST//5.8e-80:376:100//Hs.112500:AA599014
R-NT2RP2003391//ESTs//2.8e-98:484:97//Hs.5842:AA534476
R-NT2RP2003393//ESTs//2.0e-96:510:93//Hs.75844:AA115502
R-NT2RP2003394//EST//5.2e-06:264:63//Hs.144234:W52249
R-NT2RP2003401//ESTs//6.1e-25:161:90//Hs.155360:AA984683
R-NT2RP2003433//ESTs, Highly similar to PROTEIN TRANSPORT PROTEIN SEC61 ALPHA SUBUNIT [Canis familiaris]//1.2e-106:508:98//Hs.131840:AI016073
R-NT2RP2003445//ESTs, Moderately similar to !!!! ALU SUBFAMILY J WARNING ENTRY !!!! [H.sapiens]//5.6e-21:161:70//Hs.43153:N22360
R-NT2RP2003446//ESTs, Weakly similar to C27H6.4 [C.elegans]//6.0e-105:529:96//Hs.8055:W60903
R-NT2RP2003456//ESTs//7.5e-96:449:99//Hs.25362:AI277332
R-NT2RP2003480//ESTs//1.6e-116:583:96//Hs.59757:AA176121
R-NT2RP2003499//ESTs, Weakly similar to elastin like protein [D.melanogaster]//7.0e-71:365:95//Hs.101056:R52777
R-NT2RP2003506//ESTs, Weakly similar to ORF YPL207w [S.cerevisiae]//2.3e-115:577:96//Hs.16277:N36831
R-NT2RP2003511//ESTs//1.6e-22:182:85//Hs.28249:AA203733
R-NT2RP2003513//Human mRNA for KIAA0270 gene, partial cds//1.3e-108:566:94//Hs.78482:Y16270
R-NT2RP2003517//Platelet-derived growth factor beta polypeptide (simian sarcoma viral (v-sis) oncogene homolog)//4.9e-62:518:79//Hs.1976:M12783
R-NT2RP2003522//ESTs//2.0e-97:462:99//Hs.24512:D60170
R-NT2RP2003533//ESTs//4.4e-45:273:78//Hs.140225:AA704101
R-NT2RP2003543//EST//1.0:80:68//Hs.65646:F13684
R-NT2RP2003559//ESTs, Moderately similar to !!!! ALU SUBFAMILY J WARNING ENTRY !!!! [H.sapiens]//1.8e-58:316:94//Hs.28891:W72439
R-NT2RP2003564//ESTs//3.2e-112:528:99//Hs.53940:N46696
R-NT2RP2003581//ESTs//1.3e-88:506:93//Hs.16157:AA203719
R-NT2RP2003596//ESTs, Weakly similar to No definition line found [C.elegans]//4.7e-101:495:98//Hs.34627:AA126463
R-NT2RP2003604//Homo sapiens alpha-catenin related protein (ACRP) mRNA, complete cds//1.7e-103:501:97//Hs.58488:U97067
R-NT2RP2003629//EST//0.032:440:59//Hs.135297:AI038981
R-NT2RP2003643//ESTs, Weakly similar to HYPOTHETICAL 14.1 KD PROTEIN IN MURZ-RPON INTERGENIC REGION [E.coli]//9.1e-62:359:92//Hs.12492:AA203188
R-NT2RP2003668//EST//9.4e-110:535:97//Hs.116279:AA628951
R-NT2RP2003687//EST//5.9e-05:196:65//Hs.139064:AA135523
R-NT2RP2003691//ESTs, Weakly similar to F59C6.9 [C.elegans]//1.0:202:62//Hs.65539:AI148540
R-NT2RP2003702//ESTs, Moderately similar to ovarian-specific protein [R.norvegicus]//4.3e-99:492:96//Hs.93332:AA811920
R-NT2RP2003704//ESTs//1.0:155:63//Hs.104166:AA740246
R-NT2RP2003706//Homo sapiens mRNA for KIAA0525 protein, partial cds//8.4e-47:265:93//Hs.78494:AB011097
R-NT2RP2003713//EST//0.81:210:59//Hs.14551:T79401
R-NT2RP2003714//ESTs//1.7e-99:495:96//Hs.158101:AI365003
R-nnnnnnnnnnnnn//Human 19.8 kDa protein mRNA, complete cds//0.84:221:60//Hs.2384:U18914
R-NT2RP2003737//ESTs, Highly similar to UBIQUITIN-CONJUGATING ENZYME E2-17 KD [Caenorhabditis elegans]//2.4e-50:302:90//Hs.19196:W74577
R-NT2RP2003751
R-NT2RP2003760//ESTs//2.6e-101:548:93//Hs.115987:AA483808
R-NT2RP2003764//ESTs//8.2e-25:134:98//Hs.64036:AA127709
R-NT2RP2003769//ESTs//1.7e-108:545:95//Hs.56847:AA541606
R-NT2RP2003770//Homo sapiens sperm acrosomal protein mRNA, complete cds//6.0e-106:531:96//Hs.90436:AF047437
R-NT2RP2003777//ESTs//2.6e-59:323:94//Hs.10101:AI381811
R-NT2RP2003781//ESTs//2.0e-25:269:75//Hs.144951:N34836
R-NT2RP2003793//ESTs//8.7e-94:466:97//Hs.93949:AA782955
R-NT2RP2003840//ESTs//3.4e-97:533:93//Hs.16130:AA195077
R-NT2RP2003857//H.sapiens mRNA for G9a//2.8e-23:351:65//Hs.75196:X69838
R-NT2RP2003859//ESTs//3.0e-07:96:81//Hs.153262:AA551124
R-NT2RP2003871//ESTs//1.9e-102:509:97//Hs.25726:AA430167
R-NT2RP2003885//ESTs//1.0e-102:502:97//Hs.36353:AA702341
R-NT2RP2003912//EST//1.2e-38:336:76//Hs.134975:AI094611
R-NT2RP2003952//Homo sapiens DNA-binding protein (CROC-1B) mRNA, complete cds//0.90:190:60//Hs.75875:U49278
R-NT2RP2003968//Homo sapiens hUBP mRNA for ubiquitin specific protease, complete cds//7.6e-116:568:97//Hs.35086:AB014458
R-NT2RP2003976//Homo sapiens mRNA for KIAA0447 protein, complete cds//3.6e-109:540:97//Hs.7302:AB007916
R-NT2RP2003981//Homo sapiens mRNA for KIAA0804 protein, partial cds//2.5e-115:568:96//Hs.7316:AB018347
R-NT2RP2003984
R-NT2RP2003986//ESTs//4.9e-36:272:82//Hs.158268:AA738087
R-NT2RP2003988//ESTs, Weakly similar to reverse transcriptase [H.sapiens]//3.2e-110:519:99//Hs.36093:AI149968
R-NT2RP2004014//ESTs//8.4e-102:483:99//Hs.22867:AI417478
R-NT2RP2004041
R-NT2RP2004042//ESTs//1.5e-105:466:97//Hs.7296:N29706
R-nnnnnnnnnnnn//ESTs//1.4e-110:559:96//Hs.71916:AA219699
R-NT2RP2004081//ESTs//3.7e-105:503:98//Hs.27542:AA977204
R-NT2RP2004098//EST//7.3e-26:203:87//Hs.21897:R41461
R-NT2RP2004124//ESTs//1.1e-83:435:95//Hs.43299:N23036
R-NT2RP2004142//EST//1.3e-06:165:65//Hs.146742:AI147500
R-NT2RP2004152//ESTs//7.0e-98:455:100//Hs.17731:AI342241
R-NT2RP2004165//ESTs, Highly similar to DYNEIN BETA CHAIN, CILIARY [Anthocidaris crassispina]//1.0e-118:583:97//Hs.16520:AI224533
R-NT2RP2004170//ESTs//6.7e-66:407:88//Hs.157138:AI348544
R-NT2RP2004172//ESTs//1.5e-109:567:95//Hs.159091:AA033974
R-NT2RP2004187//ESTs//3.6e-92:488:93//Hs.22954:W26589
R-NT2RP2004194//ESTs//6.2e-114:585:95//Hs.18778:AA203167
R-NT2RP2004196
R-NT2RP2004207//ESTs//6.3e-102:488:98//Hs.22678:AA604756
R-NT2RP2004226//ESTs//8.8e-18:252:71//Hs.11924:W26972
R-NT2RP2004232//ESTs, Highly similar to protein kinase C mu [H.sapiens]//5.2e-105:499:98//Hs.143460:AA483305
R-NT2RP2004239//ESTs//1.2e-16:171:80//Hs.16134:AA203116
R-NT2RP2004240//Homo sapiens antigen NY-CO-1 (NY-CO-1) mRNA, complete cds//3.4e-103:530:93//Hs.54900:AF039687
R-NT2RP2004242//ESTs//1.3e-85:460:93//Hs.104535:AA211483
R-NT2RP2004245//ESTs//6.4e-117:575:97//Hs.23744:AA035744
R-NT2RP2004270//ESTs//1.0:95:69//Hs.141371:H92187
R-NT2RP2004300//ESTs//4.4e-80:379:99//Hs.130874:AA905056
R-NT2RP2004316//Homo sapiens EXT-like protein 2 (EXTL2) mRNA, complete cds//4.7e-110:544:96//Hs.61152:AF000416
R-NT2RP2004321//ESTs//2.1e-18:104:99//Hs.107207:AA044788
R-NT2RP2004339//EST//1.4e-47:309:86//Hs.161917:AA483223
R-NT2RP2004347
R-NT2RP2004364//ESTs//1.1e-113:566:96//Hs.25880:AI268173
R-NT2RP2004365//ESTs//0.022:271:62//Hs.38897:AI129310
R-NT2RP2004366//ESTs//9.5e-71:335:100//Hs.91867:AI218624
R-NT2RP2004373//ESTs//4.2e-25:172:87//Hs.83243:N32192
R-NT2RP2004389//ESTs, Highly similar to HYPOTHETICAL 70.7 KD PROTEIN F09G8.3 IN CHROMOSOME III [Caenorhabditis elegans]//1.4e-11:108:82//Hs.30490:AA146916
R-NT2RP2004392//ESTs//3.4e-81:427:94//Hs.5827:AA581646
R-NT2RP2004396//EST//5.6e-06:100:77//Hs.138623:H92473
R-NT2RP2004399//EST//0.98:337:59//Hs.118446:N67900
R-NT2RP2004400//ESTs//2.1e-90:422:100//Hs.152460:AA602921
R-NT2RP2004412//ESTS//1.4e-105:503:98//Hs.15929:AA403121
R-NT2RP2004425//EST//0.00017:225:60//Hs.146935:AI168124
R-NT2RP2004476//ESTs//1.4e-88:477:94//Hs.4859:N29695
R-NT2RP2004490//Homo sapiens 3-phosphoinositide dependent protein kinase-1 (PDK1) mRNA, complete cds//8.6e-34:143:98//Hs.154729:AF017995
R-NT2RP2004512//ESTs//2.6e-91:426:100//Hs.94133:AI270700
R-NT2RP2004523//ESTs//1.6e-74:377:97//Hs.14217:R61320
R-NT2RP2004538//Thromboxane A2 receptor//1.4e-45:279:89//Hs.89887:D38081
R-NT2RP2004551//ESTs//0.47:147:66//Hs.131519:AI024347
R-NT2RP2004568//ESTs//1.3e-107:567:94//Hs.65234:AA195470
R-NT2RP2004580//ESTs//5.9e-29:156:98//Hs.147801:AI221661
R-NT2RP2004587//ESTs//1.0e-102:495:97//Hs.91662:AA781126
R-NT2RP2004594//ESTs//4.1e-56:298:95//Hs.24641:AA954666
R-NT2RP2004600//ESTs//4.8e-67:374:93//Hs.49762:N69862
R-NT2RP2004602//ESTs, Weakly similar to !!!! ALU SUBFAMILY J WARNING ENTRY !!!! [H.sapiens]//4.5e-07:149:76//Hs.12845:N28835
R-NT2RP2004614//ESTs//1.0e-111:557:96//Hs.37892:N53497
R-NT2RP2004655//Homo sapiens mRNA for leucine rich protein//2.4e-118:587:96//Hs.5198:AJ006291
R-NT2RP2004664//Homo sapiens mRNA for KIAA0460 protein, partial cds//5.9e-107:520:96//Hs.29956:AB007929
R-NT2RP2004675//ESTs//2.7e-82:407:97//Hs.116113:F18930
R-NT2RP2004681//NUCLEOLIN//0.34:387:58//Hs.79110:M60858
R-NT2RP2004689//Homo sapiens mRNA for KIAA0625 protein, partial cds//5.0e-120:600:96//Hs.154919:AB014525
R-NT2RP2004709//ESTs//1.1e-106:511:98//Hs.38034:AI149793
R-NT2RP2004710//ESTs//9.9e-87:477:93//Hs.6834:AA203433
R-NT2RP2004736//Homo sapiens mRNA for KIAA0478 protein, complete cds//1.3e-118:594:96//Hs.4236:AB007947
R-NT2RP2004743//ESTs//2.1e-48:327:88//Hs.43635:AA447015
R-NT2RP2004767//EST//4.0e-57:328:81//Hs.142796:N51423
R-NT2RP2004775//ESTs//9.4e-60:326:94//Hs.115339:AA136774
R-NT2RP2004791//ESTs//3.2e-82:367:96//Hs.141911:N64013
R-NT2RP2004799//Homo sapiens ATP-specific succinyl-CoA synthetase beta subunit (SCS) mRNA, partial cds//8.0e-116:564:96//Hs.40820:AF058953
R-NT2RP2004802//ESTs//6.5e-111:586:94//Hs.90375:W74579
R-NT2RP2004816//Homo sapiens H beta 58 homolog mRNA, complete cds//8.7e-120:584:97//Hs.67052:AF054179
R-NT2RP2004841//EST//3.8e-31:323:74//Hs.147714:AI219906
R-NT2RP2004861//EST//0.92:147:63//Hs.23064:R20803
R-NT2RP2004897//ESTs//1.7e-46:390:80//Hs.139225:H96567
R-NT2RP2004936//EST//0.97:176:63//Hs.137436:AA280529
R-nnnnnnnnnnn//ESTs//0.059:137:64//Hs.144109:AI345543
R-NT2RP2004961//ESTs//1.8e-87:409:100//Hs.138297:AA781941
R-NT2RP2004962//ESTs//0.0021:292:59//Hs.145917:AI275458
R-NT2RP2004967//Human mRNA for KIAA0118 gene, partial cds//7.4e-51:506:75//Hs.154326:D42087
R-NT2RP2004978//ESTs//0.95:138:63//Hs.13619:W93496
R-NT2RP2004982//ESTs//7.8e-95:468:97//Hs.22545:R43910
R-NT2RP2004985
R-NT2RP2004999//ESTs//2.9e-94:450:98//Hs.128766:AI419902
R-NT2RP2005000
R-NT2RP2005001//Homo sapiens mRNA for KIAA0615 protein, complete cds//9.6e-113:577:95//Hs.155972:AB014515
R-NT2RP2005003//EST//1.3e-75:387:96//Hs.140843:R42235
R-nnnnnnnnnnnn//Homo sapiens SEC63 (SEC63) mRNA, complete cds//3.1e-116:568:97//Hs.31575:AF100141
R-NT2RP2005018//ESTs//7.5e-46:280:90//Hs.126857:AA932161
R-NT2RP2005020//ESTs//1.6e-105:554:94//Hs.14846:AA148507
R-NT2RP2005031//EST//3.1e-79:379:99//Hs.139709:AA227887
R-NT2RP2005037//ESTs//5.3e-102:551:93//Hs.26516:AA195220
R-NT2RP2005038//ESTs//5.8e-101:566:92//Hs.46964:N49757
R-NT2RP2005108
R-NT2RP2005116//Homo sapiens mRNA for KIAA0664 protein, partial cds//2.7e-105:518:97//Hs.22616:AB014564
R-NT2RP2005126//H.sapiens mRNA for RNA helicase (Myc-regulated dead box protein)//4.6e-69:464:85//Hs.100555:X98743
R-NT2RP2005139//ESTs//1.0e-108:545:95//Hs.21006:AA523383
R-NT2RP2005140//ESTs//4.3e-90:422:99//Hs.62180AI341261
R-NT2RP2005144//ESTs//0.91:162:62//Hs.52399:AI075744
R-NT2RP2005147//ESTs//4.6e-100:502:96//Hs.27931:AA633438
R-NT2RP2005159//ESTs//7.5e-105:533:95//Hs.109819:AI357582
R-NT2RP2005162//ESTs//6.6e-83:419:96//Hs.113998:H50648
R-NT2RP2005168//Homo sapiens mRNA for ElB-55kDa-associated protein//2.4e-101:513:95//Hs.155218:AJ007509
R-NT2RP2005204//ESTs, Weakly similar to UBIQUITIN-ACTIVATING ENZYME E1 HOMOLOG [H.sapiens]//1.9e-115:577:96//Hs.7600:H98166
R-NT2RP2005227//Homo sapiens UM protein mRNA, complete cds//1.0e-45:359:82//Hs.154103:AF061258
R-NT2RP2005239//ESTs, Highly similar to NIFS-LIKE 54.5 KD PROTEIN [Saccharomyces cerevisiae]//1.0e-47:245:97//Hs.21090:AA418587
R-NT2RP2005254//ESTs//3.3e-111:581:94//Hs.22549:AA524503
R-NT2RP2005270//ESTs, Highly similar to HYPOTHETICAL 67.6 KD PROTEIN ZK637.3 IN CHROMOSOME III [Caenorhabditis elegans]//1.1e-79:412:95//Hs.23047:N66596
R-NT2RP2005276//ESTs//4.6e-85:426:96//Hs.24550:AA316272
R-NT2RP2005287//ESTs//1.7e-109:565:94//Hs.61976:AI279001
R-NT2RP2005288//Homo sapiens RCC1-like G exchanging factor RLG mRNA, complete cds//2.4e-125:594:98//Hs.27007:AF060219
R-NT2RP2005289//Homo sapiens mRNA for XPR2 protein//4.9e-112:545:96//Hs.44766:AJ007590
R-NT2RP2005293//ESTs//5.1e-116:538:99//Hs.62180:AI341261
R-NT2RP2005315//ESTs//1.4e-82:415:97//Hs.155829:AA018338
R-NT2RP2005325//Human LIM-homeobox domain protein (hLH-2) mRNA, complete cds//2.5e-45:272:91//Hs.1569:U11701
R-NT2RP2005336//ESTs//1.9e-93:444:99//Hs.110966:AA151699
R-NT2RP2005 344//Homo sapiens GDP-L-fucose pyrophosphorylase (GFPP) mRNA, complete cds//0.011:463:58//Hs.150926:AF017445
R-NT2RP2005354//ESTs//7.2e-22:148:91//Hs.153783:H14544
R-NT2RP2005360//ESTs//0.048:225:60//Hs.7602:AA099247
R-NT2RP2005393//Homo sapiens mRNA for KIAA0761 protein, partial cds//2.9e-41:248:82//Hs.93121:AB018304
R-NT2RP2005407//ESTs, Weakly similar to OSH1 PROTEIN [Saccharomyces cerevisiae]//2.5e-75:461:88//Hs.70849:AA121697
R-NT2RP2005436//ESTs, Weakly similar to HYPOTHETICAL 37.0 KD PROTEIN B0495.8 IN CHROMOSOME II [C.elegans]//8.1e-96:491:95//Hs.7194:AI185631
R-NT2RP2005441//ESTs//1.1e-110:548:96//Hs.5209:AA780068
R-NT2RP2005453//ESTs//0.94:352:58//Hs.25870:H14423
R-NT2RP2005457//ESTs//2.1e-46:236:97//Hs.19522:AA975096
R-NT2RP2005464//ESTS//1.8e-72:349:99//Hs.44045:N51307
R-NT2RP2005465//ESTs//0.0058:322:58//Hs.127009:AI378936
R-NT2RP2005472//ESTs//0.47:309:60//Hs.144838:AI222019
R-NT2RP2005476//ESTS//5.1 e-40:205:9811Hs.101577:AI168526
R-NT2RP2005490//ESTs//L3e-70:364:96//Hs.134382:AA083573
R-NT2RP2005491//EST//0.012:220:60//Hs.144448:AA812455
R-NT2RP2005495//ESTs//1.2e-86:501:91//Hs.99445:R93540
R-NT2RP2005496//ESTs//3.2e-34:263:81//Hs.70279:AA757426
R-NT2RP2005498//ESTS, Highly similar to PROTEIN PHOSPHATASE PP2A, 55 KD REGULATORY SUBUNIT, NEURONAL ISOFORM [Oryctolagus cuniculus]//2.3e-45:284:88//Hs.85752:AI138993
R-NT2RP2005501//ESTs//2.5e-84:404:98//Hs.143812:AI141755
R-NT2RP2005509//ESTs, Highly similar to HYPOTHETICAL 37.2 KD PROTEIN C12C2.09C IN CHROMOSOME I [Schizosaccharomyces pombe]//8.2e-36:215:92//Hs.5298:AA725071
R-NT2RP2005520//Homo sapiens chromosome-associated protein-E (hCAP-E) mRNA, complete cds//3.2e-110:570:9411Hs.119023:AF092563
R-NT2RP2005525//ESTs, Weakly similar to !!!! ALU SUBFAMILY SQ WARNING ENTRY !!!! [H.sapiens]//1.3e-84:433:95//Hs.36942:AA524535
R-NT2RP2005531//EST//0.98:64:70//Hs.146573:AI139856
R-NT2RP2005539//Homo sapiens mRNA for NS1-binding protein (NS1-BP)//8.8e-108:560:94//Hs.159597:AJ012449
R-NT2RP2005540//Homo sapiens mRNA for KIAA0494 protein, complete cds//1.7e-115:583:96//Hs.62515:AB007963
R-NT2RP2005549//EST//0.61:111:62//Hs.147482:AI215572
R-NT2RP2005555//ESTs//6.6e-108:507:99//Hs.68613:AI357567
R-NT2RP2005557//ESTs//3.1e-105:495:99//Hs.105985:AA885169
R-NT2RP2005581//ESTs//1.7e-79:445:92//Hs.138152:H03240
R-NT2RP2005600//ESTs//1.3e-38:192:100//Hs.48329:W92733
R-NT2RP2005605//ESTs//7.6e-87:409:99//Hs.45005:AA975060
R-NT2RP2005620//ESTs//2.9e-96:463:97//Hs.7407:AI376788
R-NT2RP2005622//ESTs//1.8e-104:497:98//Hs.22595:AA394229
R-NT2RP2005637//EST//2.5e-20:163:71//Hs.161164:AI418211
R-NT2RP2005640//ESTs//5.0e-99:473:98//Hs.23467:AA708740
R-NT2RP2005645//ESTs//9.5e-23:231:77//Hs.5534:AA195173
R-NT2RP2005651//ESTS, Highly similar to XFIN PROTEIN [Xenopus laevis]//2.9e-103:525:96//Hs.70589:AA868470
R-NT2RP2005654//Insulin-like growth factor binding protein 2//0.94:223:60//Hs.162:X16302
R-NT2RP2005669//Homo sapiens nitrilase 1 (VIII) mRNA, complete cds//2.7e-14:87:100//Hs.146406:AF069987
R-NT2RP2005675//Homo sapiens growth suppressor related (DOC-1R) mRNA, complete cds//5.8e-91:434:98//Hs.25664:AF089814
R-NT2RP2005683//ESTs//1.5e-98:494:96//Hs.22595:AA394229
R-NT2RP2005690//ESTs//4.8e-43:286:86//Hs.150727:AI292236
R-NT2RP2005694//EST//3.1e-82:386:100//Hs.149391:AI273643
R-NT2RP2005701//ESTs, Highly similar to BUTYROPHILIN PRECURSOR [Bos tauros]//2.8e-68:376:93//Hs.9095:AA532630
R-NT2RP2005712//Homo sapiens mRNA for KIAA0799 protein, partial cds//1.3e-105:503:98//Hs.61638:AB018342
R-NT2RP2005719//ESTs, Weakly similar to GPI-anchored protein p137 precursor [H.sapiens]//5.4e-105:500:98//Hs.14298:AI417523
R-NT2RP2005722//EST//6.5e-76:395:94//Hs.142150:AA223982
R-NT2RP2005723//ESTs//1.5e-84:452:93//Hs.91753:R44455
R-NT2RP2005726//ESTs//3.5e-64:500:82//Hs.100526:AI223153
R-NT2RP2005741//ESTs//4.7e-60:333:93//Hs.107242:R40258
R-NT2RP2005748//ESTs//3.4e-102:498:97//Hs.82660:N78064
R-NT2RP2005752//Homo sapiens TNFR-related death receptor-6 (DR6) mRNA, complete cds//4.3e-42:223:96//Hs.159651:AF068868
R-NT2RP2005753//Homo sapiens I-1 receptor candidate protein mRNA, complete cds//1.2e-104:494:98//Hs.26285:AF082516
R-NT2RP2005763//ESTs//1.1e-97:456:99//Hs.65412:AI362163
R-NT2RP2005767//ESTs//8.0e-38:204:96//Hs.18460:AA193463
R-NT2RP2005773//ESTs, Highly similar to PYRROLINE-5-CARBOXYLATE REDUCTASE [Homo sapiens]//5.4e-112:559:96//Hs.14214:AI189379
R-NT2RP2005775//ESTs, Highly similar to NEUROLYSIN PRECURSOR [Sus scrofa]//3.0e-108:544:96//Hs.22151:AI214321
R-NT2RP2005781//ESTs//1.7e-43:217:99//Hs.144391:AA365664
R-NT2RP2005784//EST//0.0071:217:60//Hs.117332:AA699724
R-NT2RP2005804//ESTs//8.8e-107:512:98//Hs.15496:W44398
R-NT2RP2005812//ESTs//9.0e-76:359:99//Hs.113937:AI298746
R-NT2RP2005815//ESTs//5.5e-76:363:99//Hs.136230:AA594981
R-NT2RP2005835//ESTs//1.5e-100:541:94//Hs.86813:N25122
R-NT2RP2005841//ESTs//2.8e-105:556:92//Hs.69993:AA628403
R-NT2RP2005853//EST//2.0e-13:219:70//Hs.134016:AI076062
R-NT2RP2005857//ESTS//1.0e-115:576:96//Hs.30663:AI338462
R-NT2RP2005859//ESTs//7.3e-116:571:97//Hs.85986:AA195105
R-NT2RP2005868//EST//0.00023:320:61//Hs.149689:AI284133
R-NT2RP2005890//ESTs//1.0e-96:466:98//Hs.122579:AA766315
R-NT2RP2005901//ESTs//8.3e-116:548:98//Hs.66296:AI125268
R-NT2RP2005908//ESTs, Weakly similar to weakly similar to gastrula zinc finger protein [C.elegans]//2.4e-73:397:94//Hs.16667:T92427
R-NT2RP2005933//ESTs, Highly similar to nucleoporin p54 [R.norvegicus]//2.8e-114:560:97//Hs.9082:AA873170
R-NT2RP2005942//ESTs//5.6e-117:582:96//Hs.146123:AI338419
R-NT2RP2005980//ESTs//6.9e-101:478:98//Hs.43145:AA776988
R-NT2RP2006023//Homo sapiens PYRIN (MEFV) mRNA, complete cds//8.5e-51:398:80//Hs.113283:AF018080
R-NT2RP2006038//ESTs//0.025:284:59//Hs.97852:AA404347
R-NT2RP2006043//ESTs, Weakly similar to HYPOTHETICAL 37.0 KD PROTEIN B0495.8 IN CHROMOSOME II [C.elegans]//1.2e-50:278:94//Hs.7194:AI185631
R-NT2RP2006052//ESTs//5.0e-52:272:95//Hs.99545:AA461492
R-NT2RP2006069//ESTs//1.8e-90:495:93//Hs.43654:AA522714
R-NT2RP2006071//ESTs//1.5e-38:218:94//Hs.107882:W72093
R-NT2RP2006098//ESTs//2.9e-105:540:95//Hs.26860:N56918
R-NT2RP2006100//Human organic anion transporting polypeptide (OATP) mRNA, complete cds//0.031:254:62//Hs.46440:U21943
R-NT2RP2006103//ESTs//1.5e-86:416:98//Hs.152114:AA401365
R-NT2RP2006141//ESTs//5.3e-88:432:98//Hs.77480:AA100522
R-NT2RP2006166//Homo sapiens LIM protein mRNA, complete cds//2.8e-17:255:72//Hs.154103:AF061258
R-NT2RP2006184//ESTs//8.4e-101:487:98//Hs.58009:W69435
R-NT2RP2006186//Homo sapiens mRNA for KIAA0654 protein, partial cds//6.1e-110:553:95//Hs.109299:AB014554
R-NT2RP2006196//Human clone 23960 mRNA sequence//0.0037:48:100//Hs.151293:U79276
R-NT2RP2006200//ESTs//6.5e-77:398:96//Hs.163953:R01398
R-NT2RP2006219//H.sapiens mRNA for DGCR6 protein//1.2e-94:532:90//Hs.153910:X96484
R-NT2RP2006237//ESTs//1.2e-57:305:95//Hs.86149:AI341312
R-NT2RP2006238//ESTs, Highly similar to rA8 [R.norvegicus]//1.5e-29:183:91//Hs.4048:AA404253
R-NT2RP2006258//ESTs//3.2e-87:462:94//Hs.141556:N49928
R-NT2RP2006261//ESTs//3.4e-57:3 26:92//Hs.22523:W02999
R-NT2RP2006312//Homo sapiens BAF57 (BAF57) gene, complete cds//4.7e-96:481:97//Hs.3404:AF035262
R-NT2RP2006320//EST//3.4e-21:335:65//Hs.141603:N66015
R-NT2RP2006321//ESTs, Moderately similar to karyopherin beta 3 [H.sapiens]//1.9e-89:460:96//Hs.21889:N78664
R-NT2RP2006323//ESTs//3.5e-91:439:98//Hs.61697:AI081771
R-NT2RP2006333//ESTs//4.9e-38:301:82//Hs.155999:AA196412
R-NT2RP2006334//EST//3.1e-45:264:91//Hs.149599:AI282321
R-NT2RP2006365//ESTs//2.9e-81:417:95//Hs.11814:W44411
R-NT2RP2006393//Cytochrome P450, subfamily I (aromatic compound-inducible), polypeptide 2//3.9e-48:403:77//Hs.1361:M55053
R-NT2RP2006436//Homo sapiens mRNA for small GTP-binding protein, complete cds//1.4e-27:155:76//Hs.115325:D84488
R-NT2RP2006441//ESTs//6.0e-108:529:97//Hs.101282:N45092
R-NT2RP2006454//ESTs//9.2e-20:110:99//Hs.144687:AI341146
R-NT2RP2006456//ESTs//7.1e-91:508:92//Hs.12488:W63595
R-NT2RP2006464//Homo sapiens mRNA for AND-1 protein//2.1e-109:524:97//Hs.72160:AJ006266
R-NT2RP2006467//EST//0.99:140:61//Hs.146958:AI174478
R-NT2RP2006472//ESTs//3.3e-92:473:95//Hs.29216:AA916679
R-NT2RP2006534//ESTs//1.2e-83:394:99//Hs.162116:AA524947
R-NT2RP2006554//ESTs//1.0e-87:460:95//Hs.47095:AA181474
R-NT2RP2006565//ESTs//3.2e-24:129:100//Hs.13499:AI299886
R-NT2RP2006571//ESTs//2.6e-56:306:94//Hs.98370:AA316622
R-nnnnnnnnnnn//ESTs//2.0e-112:533:98//Hs.18685:AI393829
R-NT2RP2006598//ESTs, Weakly similar to retinoid X receptor interacting protein [M.musculus]//4.1e-109:542:97//Hs.7889:AI337112
R-NT2RP3000002//ESTs//1.3e-08:399:59//Hs.126044:AI301598
R-NT2RP3000031//Homo sapiens mRNA for histone deacetylase-like protein (JM21)//1.9e-116:560:97//Hs.6764:AJ011972
R-NT2RP3000046//Small inducible cytokine A5 (RANTES)//1.9e-57:312:85//Hs.155464:AF088219
R-NT2RP3000047//EST//0.91:130:66//Hs.140208:AA702213
R-NT2RP3000050//ESTs, Weakly similar to putative p150 [H.sapiens]//3.1e-41:249:90//Hs.156155:AI222202
R-NT2RP3000055//EST//2.4e-19:146:86//Hs.160497:AI255095
R-NT2RP3000072//ESTs//2.2e-82:424:96//Hs.21542:N49574
R-NT2RP3000080//ESTs//2.1e-29:186:89//Hs.153372:AA424029
R-NT2RP3000085//ESTs//4.5e-101:482:98//Hs.47649:AA838715
R-NT2RP3000109//ESTs//9.5e-97:455:99//Hs.17731:AI342241
R-NT2RP3000134//EST//4.7e-106:497:99//Hs.125531:AA884000
R-NT2RP3000142//Homo sapiens mRNA for KIAA0592 protein, partial cds//1.2e-116:578:96//Hs.13273:AB011164
R-NT2RP3000149//ESTs//7.7e-62:361:90//Hs.6649:N93418
R-NT2RP3000186
R-NT2RP3000197//ESTs//1.5e-75:436:91//Hs.140931:R51882
R-NT2RP3000207//ESTs//1.3e-98:468:98//Hs.126908:AA933091
R-NT2RP3000220//ESTs//2.2e-27:144:99//Hs.106861:R61306
R-NT2RP3000233//EST//7.8e-77:368:99//Hs.49075:N64817
R-NT2RP3000235//ESTs//0.43:82:74//Hs.132828:AI032819
R-NT2RP3000247//EST//2.2e-97:459:99//Hs.127928:AA969239
R-NT2RP3000251
R-NT2RP3000252//ESTs, Weakly similar to Lpg15p [S.cerevisiae]//2.0e-108:532:97//Hs.111086:AI379177
R-NT2RP3000255//EST//0.67:93:67//Hs.120579:AA743073
R-NT2RP3000267//ESTs//8.5e-108:542:95//Hs.24984:AA534446
R-NT2RP3000299//ESTs, Weakly similar to enhancer of filmentation 1 [H.sapiens]//3.6e-103:516:96//Hs.4894:AI191323
R-NT2RP3000312//ESTs//1.3e-100:493:97//Hs.29379:AI094117
R-NT2RP3000320//ESTs//3.2e-95:538:91//Hs.118793:AA192438
R-NT2RP3000324
R-NT2RP3000333//ESTs//6.0e-39:194:100//Hs.119238:AA476267
R-NT2RP3000341//ESTS//0.51:251:61//Hs.94090:AA777689
R-NT2RP3000348//EST//1.8e-80:389:98//Hs.145944:AI276225
R-NT2RP3000350//ESTs, Weakly similar to Lpg15p [S.cerevisiae]//3.1e-110:556:96//Hs.111086:AI379177
R-NT2RP3000359//EST//4.9e-61:340:92//Hs.126495:AA913741
R-NT2RP3000361//ESTs, Weakly similar to PRE-MRNA SPLICING FACTOR PRP6 [S.cerevisiae]//4.8e-91:439:97//Hs.31334:AI144423
R-NT2RP3000366//EST//0.20:392:57//Hs.149652:AI283303
R-NT2RP3000397//EST//8.7e-26:150:94//Hs.124617:AA855106
R-NT2RP3000403//Homo sapiens formin binding protein 21 mRNA, complete cds//4.2e-111:529:98//Hs.28307:AF071185
R-NT2RP3000418//EST//3.3e-09:202:67//Hs.117189:AA682947
R-NT2RP3000433
R-NT2RP3000439//ESTs//3.1e-79:426:92//Hs.26548:W26340
R-NT2RP3000441//ESTs//6.3e-84:420:97//Hs.137482:AA421254
R-NT2RP3000449//ESTs//4.9e-93:435:99//Hs.54617:AI379102
R-NT2RP3000451//ESTs//2.3e-89:439:97//Hs.9196:AA748492
R-NT2RP3000456//Homo Sapiens (clone B3B3E13) chromosome 4pl6.3 DNA fragment//1.8e-23:347:70//Hs.114963:L34408
R-NT2RP3000484//Heparin cofactor II//0.98:166:62//Hs.1478:M58600
R-NT2RP3000487//ESTs//0.012:384:60//Hs.88684:AA885141
R-NT2RP3000512//Homeo box B3//2.0e-69:377:93//Hs.49931:X16667
R-NT2RP3000526//ESTS//1.6e-91:432:99//Hs.38042:AA187151
R-NT2RP3000527//ESTs//1.2e-100:518:94//Hs.104557:AI078161
R-NT2RP3000531//ESTs, Weakly similar to TH1 protein [D.melanogaster]//0.95:85:71//Hs.5184:AA709151
R-NT2RP3000542//ESTs//2.6e-53:375:84//Hs.44158:N30180
R-NT2RP3000561//EST//1.1e-13:170:75//Hs.148421:AI198036
R-NT2RP3000562//Human mRNA for KIAA0233 gene, complete cds//0.97:141:68//Hs.79077:D87071
R-NT2RP3000578//ESTs//2.6e-68:324:100//Hs.5445:AA779447
R-NT2RP3000582//ESTS//2.1 e-25:131:80//Hs.152465:AA563785
R-NT2RP3000584//ESTs//1.8e-97:460:99//Hs.120698:AI241511
R-NT2RP3000590//ESTs//2.0e-97:453:100//Hs.105355:AA953817
R-NT2RP3000592//ESTs//2.8e-91:432:99//Hs.144304:AI190916
R-nnnnnnnnnnnn//Human mRNA for KIAA0314 gene, partial cds//1.5e-09:447:58//Hs.155045:AB002312
R-NT2RP3000599//ESTs//3.8e-93:437:99//Hs.23971:AA829880
R-NT2RP3000605//ESTs//4.2e-111:554:96//Hs.40780:AA422049
R-NT2RP3000622//ESTs//2.0e-100:473:99//Hs.11387:AI127394
R-NT2RP3000624//ESTs, Weakly similar to KIAA0256 [H.sapiens]//5.4e-115:545:98//Hs.4857:AI090739
R-NT2RP3000628//Homo sapiens mRNA for KIAA0772 protein, complete cds//4.3e-49:397:80//Hs.15519:AB018315
R-NT2RP3000632//ESTs, Moderately similar to cyclin-selective ubiquitin carrier protein [H.sapiens]//6.3e-92:434:99//Hs.152517:AA719022
R-NT2RP3000644//ESTs//1.0e-44:306:84//Hs.155498:W27084
R-NT2RP3000661//ESTs//3.1e-95:470:97//Hs.126069:W76185
R-NT2RP3000665//ESTs//3.3e-95:503:94//Hs.34313:W81185
R-NT2RP3000685//ESTs//2.7e-99:515:94//Hs.9711:R60873
R-NT2RP3000690//ESTs//3.3e-88:414:99//Hs.1465 89:AI085578
R-NT2RP3000736
R-NT2RP3000742//ESTs, Highly similar to 1-PHOSPHATIDYLINOSITOL-4,5-BISPHOSPHATE PHOSPHODIESTERASE DELTA 1 [Rattus norvegicus]//1.8e-07:114:75//Hs.136065:W21960
R-NT2RP3000753//ESTs//3.1e-99:461:100//Hs.150901:AI310447
R-NT2RP3000759//ESTs//2.0e-74:384:95//Hs.104222:AA207243
R-NT2RP3000815//ESTs//8.5e-97:455:99//Hs.158897:AI378583
R-NT2RP3000825//EST//0.0089:343:59//Hs.42897:N20810
R-NT2RP3000826//EST//3.4e-33:342:74//Hs.162236:AA551582
R-NT2RP3000836//ESTs//6.8e-24:181:84//Hs.134464:AI151081
R-NT2RP3000841//ESTs//4.5e-93:491:93//Hs.23618:H98082
R-NT2RP3000845//ESTs//2.4e-88:473:93//Hs.8312:AA813022
R-NT2RP3000847//ESTs//9.3e-89:460:95//Hs.154106:AI051657
R-NT2RP3000850
R-NT2RP3000852//Fibrillin 2//0.55:237:63//Hs.79432:U03272
R-NT2RP3000859//ESTs//1.4e-96:509:94//Hs.7187:AA576895
R-NT2RP3000865//EST//4.8e-23:461:66//Hs.162088:AA505741
R-NT2RP3000868//ESTs//5.4e-78:430:93//Hs.102796:N70837
R-NT2RP3000869//ESTs//8.5e-77:397:94//Hs.84484:AI014673
R-NT2RP3000875//Mevalonate kinase//3.8e-78:531:84//Hs.75138:M88468
R-NT2RP3000901//ESTs//2.1e-95:466:97//Hs.10647:AA428217
R-NT2RP3000904//ESTs//1.6e-79:380:99//Hs.100850:AA479385
R-NT2RP3000917//ESTs, Highly similar to mouse Dhml protein [M.musculus]//9.5e-113:566:96//Hs.5900:AA035728
R-NT2RP3000919
R-NT2RP3000968//40S RIBOSOMAL PROTEIN S15A/1.5e-25:375:71//Hs.2953:X84407
R-NT2RP3000980//ESTs//3.3e-72:364:96//Hs.9536:AA114178
R-NT2RP3000994//ESTs//3.5e 111:537:97//Hs.21146:AA683542
R-NT2RP3001004//ESTs//9.6e-91:456:96//Hs.58974:W87405
R-NT2RP3001007//ESTs//6.7e-99:482:97//Hs.117737:AI088029
R-NT2RP3001055//ESTs//0.0012:294:60//Hs.66479:AA863044
R-NT2RP3001057//ESTs, Highly similar to ZINC FINGER PROTEIN HF.12 [Homo sapiens]//5.6e-102:486:99//Hs.145956:AA007349
R-NT2RP3001081//Retinal pigment epithelium-specific protein (65kD)//0.0012:447:58//Hs.2133:U18991
R-NT2RP3001084//ESTs//4.3e-102:528:96//Hs.25277:W87874
R-NT2RP3001096//ESTS//1.1e-110:540:96//Hs.42824:AA873182
R-NT2RP3001107//ESTs//7.6e-100:478:98//Hs.99669:AA287832
R-nnnnnnnnnnnn//DNA polymerase gamma//0.0014:50:100//Hs.80961:U60325
R-NT2RP3001111//ESTs, Weakly similar to Trf-proximal protein [D.melanogaster]//3.2e-104:543:95//Hs.93796:C06063
R-NT2RP3001113//ESTs//3.3e-100:467:99//Hs.97757:AA401575
R-NT2RP3001115//0xytocin receptor//7.9e-30:505:67//Hs.2820:X64878
R-NT2RP3001116//ESTs//4.6e-41:229:96//Hs.58412:W74779
R-NT2RP3001119//ESTs//6.9e-88:478:92//Hs.19469:AA203180
R-NT2RP3001120//ESTs//3.1e-82:430:93//Hs.110956:AI190166
R-NT2RP3001126//ESTs//4.4e-52:264:96//Hs.25264:R78188
R-NT2RP3001133//ESTs//4.7e-105:541:94//Hs.73239:AA573761
R-NT2RP3001140//Homo sapiens mRNA for KIAA0762 protein, partial cds//2.6e-115:549:97//Hs.5378:AB018305
R-NT2RP3001147//ESTs, Highly similar to GTPASE ACTIVATING PROTEIN ROTUND [Drosophila melanogaster]//9.6e-113:552:97//Hs.23900:U82984
R-NT2RP3001150//ESTs//2.9e-90:444:97//Hs.99601:AA760717
R-NT2RP3001155//Homo sapiens mRNA for AND-1 protein//9.4e-118:563:98//Hs.72160:AJ006266
R-NT2RP3001176//ESTs//1.8e-110:534:98//Hs.58650:AI074460
R-NT2RP3001214//ESTs//1.7e-109:545:96//Hs.24481:AA573139
R-NT2RP3001216//EST//0.00098:128:66//Hs.160493:AI254963
R-NT2RP3001221//EST//0.010:106:66//Hs.147774:AI221196
R-NT2RP3001232//ESTs//1.5e-101:5l8:94//Hs.21630:AA778399
R-NT2RP3001236//ESTs, Highly similar to KIAA0377 [H.sapiens]//2.8e-89:462:95//Hs.116793:AA779588
R-NT2RP3001239//ESTs, Moderately similar to NEURAXIN [Rattus norvegicus]//5.2e-82:466:91//Hs.66048:AA524416
R-NT2RP3001245//EST//0.53:237:62//Hs.161131:AI417631
R-NT2RP3001253//ESTs//1.7e-105:535:96//Hs.42315:AI222997
R-NT2RP3001260//EST//0.16:144:62//Hs.126856:AA932135
R-NT2RP3001268//Human Aac11(aac11) mRNA, complete cds//0.12:494:59//Hs.151031:U83857
R-NT2RP3001272//ESTs//1.4e-92:436:99//Hs.149831:AI383965
R-NT2RP3001274//ESTs//3.9e-81:424:95//Hs.1113184:N25651
R-NT2RP3001281//EST//3.1e-60:298:98//Hs.149230:AI247332
R-NT2RP3001307//EST//0.42:215:62//Hs.126165:AA868691
R-NT2RP3001318//ESTs//4.1e-74:363:97//Hs.130832:H92571
R-NT2RP3001325//ESTs//1.7e-106:534:96//Hs.21214:H98989
R-NT2RP3001338//Human protein tyrosine phosphatase sigma mRNA, complete cds//0.22:199:63//Hs.159534:U35234
R-NT2RP3001339//Homo sapiens mRNA for KIAA0451 protein, complete cds//3.9e-114:566:96//Hs.18586:AB007920
R-NT2RP3001340//ESTs//1.1e-72:411:92//Hs.21135:W81653
R-NT2RP3001355//ESTs//9.0e-103:521:95//Hs.99486:AA776798
R-NT2RP3001374//ESTs//2.7e-82:395:98//Hs.117102:AA993090
R-NT2RP3001383//ESTs//3.6e-10:118:78//Hs.111055:AA169778
R-NT2RP3001384//ESTs, Weakly similar to A-kinase anchor protein 95, AKAP95 [R.norvegicus]//5.7e-92:522:90//Hs.96200:AA218942
R-NT2RP3001392//ESTs//5.9e-62:296:100//Hs.125034:AA907375
R-NT2RP3001396//ESTS//3.7e-111:528:98//Hs.22612:AA152232
R-NT2RP3001398//ESTs//2.6e-94:449:99//Hs.146332:AI276628
R-NT2RP3001399//ESTs//2.6e-82:401:97//Hs.7932:AI041186
R-NT2RP3001407//ESTs//2.2e-101:488:97//Hs.71573:AA496898
R-NT2RP3001420//EST//7.4e-44:394:79//Hs.137041:AA877817
R-NT2RP3001426//Homo sapiens clone 24616 mRNA sequence//3.6e-106:550:94//Hs.6957:AF052158
R-NT2RP3001427//ESTs//1.3e-87:374:97//Hs.5457:H05692
R-nnnnnnnnnnnn//Neurotrophic tyrosine kinase, receptor, type 1//4.7e-96:533:91//Hs.85844:X66397
R-NT2RP3001432//ESTs//1.9e-102:523:95//Hs.132978:AI041374
R-NT2RP3001447//ESTs, Moderately similar to !!!! ALU SUBFAMILY J WARNING ENTRY !!!! [H.sapiens]//5.1e-101:482:98//Hs.124135:AA910560
R-NT2RP3001449//ESTs//2.2e-99:502:96//Hs.7834:N45994
R-NT2RP3001453//Small inducible cytokine A5 (RANTES)//8.1e-45:295:85//Hs.155464:AF088219
R-NT2RP3001457//ESTS//1.5e-52:256:99//Hs.117982:AA644658
R-NT2RP3001459//ESTs//3.4e-62:299:99//Hs.146098:AA167280
R-NT2RP3001472//ESTs//4.8e-108:540:96//Hs.69594:N37009
R-NT2RP3001490//ESTs//3.5e-91:549:88//Hs.6606:AA211783
R-NT2RP3001495//Human oxidoreductase (HHCMA56) mRNA, complete cds//1.4e-61:338:93//Hs.519:U13395
R-NT2RP3001497//Homo sapiens multiple membrane spanning receptor TRC8 (TRC8) mRNA, complete cds//6.8e-112:549:9711Hs.28285:AF064801
R-NT2RP3001527//ESTs//4.4e-105:543:95//Hs.158761:AA631047
R-NT2RP3001529//Homo sapiens tapasin (NGS-17) mRNA, complete cds//7.9e-59:427:83//Hs.5247:AF029750
R-NT2RP3001538//ESTs//1.6e-94:521:92//Hs.6846:AA209463
R-NT2RP3001554//ESTs, Moderately similar to NEURAXIN [Rattus norvegicus]//2.8e-76:392:95//Hs.66048:AA524416
R-NT2RP3001580//ESTs//3.7e-82:398:98//Hs.23490:N49477
R-NT2RP3001587//Homa sapiens mRNA for HRIHFB2115, partial cds//1.8e-09:86:88//Hs.4311:AB015337
R-NT2RP3001589//ESTs//0.0029:243:62//Hs.158924:AA605194
R-NT2RP3001607//EST//0.00096:76:78//Hs.140319:AA748328
R-NT2RP3001608//ESTs//3.8e-105:525:96//Hs.144655:AI279798
R-NT2RP3001621//ESTs//3.3e-108:535:97//Hs.47378:AI193598
R-NT2RP3001629
R-NT2RP3001634//Homo sapiens TRIAD1 type I mRNA, complete cds//2.7e-109:541:96//Hs.9899:AF099149
R-NT2RP3001642//ESTs//6.0e-105:525:96//Hs.3376:AA915989
R-NT2RP3001646//ESTs//4.8e-95:523:92//Hs.64036:AA127709
R-NT2RP3001671//ESTs//0.0013:367:60//Hs.106090:AA457030
R-NT2RP3001672//ESTs//3.4e-37:191:98//Hs.57475:AI382189
R-NT2RP3001676//ESTs//1.5e-81:408:97//Hs.142547:N67648
R-NT2RP3001678//ESTs//4.3e-85:405:99//Hs.121915:AI268225
R-NT2RP3001679//ESTs//3.4e-100:545:93//Hs.5943:AI222558
R-NT2RP3001688//Human mRNA for KIAA0392 gene, partial cds//8.6e-46:301:87//Hs.40100:AB002390
R-NT2RP3001690//ESTs//3.3e-111:542:97//Hs.86149:AI341312
R-NT2RP3001708//ESTs//1.4e-96:349:95//Hs.17975:AA868618
R-NT2RP3001712//ESTs//9.3e-14:102:92//Hs.78041:N29669
R-NT2RP3001716//ESTs, Highly similar to BONE MORPHOGENETIC PROTEIN 1 PRECURSOR [Mus musculus]//4.1e-80:444:91//Hs.6823:W18181
R-NT2RP3001724//ESTs//1.8e-109:547:96//Hs.14570:AI422099
R-NT2RP3001730//ESTs//4.1e-98:528:92//Hs.155115:AA669923
R-NT2RP3001739//ESTs//4.4e-87:444:94//Hs.27239:W27810
R-NT2RP3001752//ESTS//6.1e-93:490:94//Hs.4210:AA740440
R-NT2RP3001753//ESTs//2.5e-82:395:99//Hs.126435:AA912968
R-NT2RP3001764//ESTs, Weakly similar to protein-tyrosine phosphatase [H.sapiens]//1.2e-87:450:96//Hs.20281:N92517
R-NT2RP3001777//ESTs//1.1e-86:360:97//Hs.100530:H06725
R-NT2RP3001782//Homo sapiens mRNA for KIAA0459 protein, partial cds//4.2e-113:549:97//Hs.28169:AB007928
R-NT2RP3001792//ESTs, Weakly similar to F35C12.2 [C.elegans]//1.1e-21:119:99//Hs.44268:AA455900
R-NT2RP3001799//OX40L RECEPTOR PRECURSOR//2.8e-45:374:79//Hs.129780:X75962
R-NT2RP3001819//ESTs//2.6e-87:432:96//Hs.10414:AI291292
R-NT2RP3001844//ESTs//0.024:128:67//Hs.25131:N50117
R-NT2RP3001854//ESTs//1.4e-92:490:92//Hs.15165:N52900
R-NT2RP3001855//ESTs//1.9e-66:361:93//Hs.10043:D81792
R-NT2RP3001896//ESTs//1.4e-96:343:97//Hs.24809:N73642
R-NT2RP3001898//ESTs//4.1e-90:515:91//Hs.4867:AA521180
R-NT2RP3001915//ESTs//4.4e-32:175:95//Hs.24641:AA954666
R-NT2RP3001926//ESTs, Highly similar to NUCLEOLYSIN TIA-1 [Homo sapiens]//1.0e-40:202:100//Hs.24709:AI123300
R-NT2RP3001929//ESTs//6.6e-84:449:94//Hs.26962:AA682781
R-NT2RP3001931//ESTs//1.0e-41:214:99//Hs.32360:AA534737
R-NT2RP3001938//ESTs, Highly similar to SPORULATION-SPECIFIC PROTEIN 1 [Saccharomyces cerevisiae]//1.3e-95:483:96//Hs.5771:W74591
R-NT2RP3001943//ESTs//1.2e-23:169:88//Hs.103930:AA160990
R-NT2RP3001944//ESTs//2.0e-90:439:97//Hs.103380:AI291325
R-NT2RP3001969//ESTs//0.95:133:65//Hs.131669:AI025889
R-NT2RP3001989//ESTS, Weakly similar to C01A2.4 [C.elegans]//8.9e-64:310:99//Hs.11449:AI201540
R-NT2RP3002002//ESTs//2.1e-95:562:89//Hs.5997:AA897088
R-NT2RP3002004//H.sapiens mRNA for FAST kinase//1.6e-42:335:82//Hs.75087:X86779
R-NT2RP3002007//ESTs//0.12:184:66//Hs.94030:AA846729
R-NT2RP3002014//Small inducible cytokine A5 (RANTES)//6.8e-47:291:89//Hs.155464:AF088219
R-NT2RP3002033
R-NT2RP3002045//ESTs//1.0e-92:555:88//Hs.106411:W29081
R-NT2RP3002054//EST//0.45:155:63//Hs.5656:D20426
R-NT2RP3002056//ESTs//1.4e.95:504:93//Hs.17428:AI365221
R-NT2RP3002057//Human mRNA for KIAA0152 gene, complete cds//0.69:127:66//Hs.90438:D63486
R-NT2RP3002062
R-nnnnnnnnnnnn//ESTs//2.1e-113:552:97//Hs.9591:AA069657
R-NT2RP3002081//ESTs//5.5e-43:212:100//Hs.124852:AA969139
R-NT2RP3002097//EST//2.3e-10:80:91//Hs.102717:N59148
R-NT2RP3002102
R-NT2RP3002108
R-NT2RP3002146//ESTs//5.5e-58:296:97//Hs.65328:AA625385
R-NT2RP3002147//EST//2.5e-53:387:81//Hs.147928:M249703
R-NT2RP3002151//ESTs, Highly similar to G1 TO S PHASE TRANSITION PROTEIN 1 HOMOLOG [Homo sapiens]//6.2e-107:534:96//Hs.59523:AA602837
R-NT2RP3002163//ESTs//2.7e-106:520:97//Hs.21258:AA412293
R-NT2RP3002165//ESTs//7.4e-93:479:95//Hs.27299:AI074024
R-NT2RP3002166//ESTs//1.0:261:59//Hs.132817:AA593713
R-NT2RP3002173//ESTs//2.7e-93:512:92//Hs.23648:H07120
R-NT2RP3002181//ESTs//1.0e-84:435:96//Hs.47378:AI193598
R-NT2RP3002244//ESTs//2.7e-11:97:89//Hs.9412:W72446
R-NT2RP3002248//ESTs//4.3e-90:459:95//Hs.9848:AA130588
R-NT2RP3002255//ESTs//1.3e-45:289:88//Hs.9100:AA431672
R-NT2RP3002273//ESTs//2.3e-100:489:97//Hs.8258:AA744743
R-NT2RP3002276//ESTs//1.2e-50:306:91//Hs.16160:AA778171
R-NT2RP3002303//ESTs//1.1e-67:323:99//Hs.129761:AA836898
R-NT2RP3002304//ESTs//2.8e-86:405:99//Hs.29643:AA418500
R-NT2RP3002330//ESTs, Weakly similar to G1 TO S PHASE TRANSITION PROTEIN 1 HOMOLOG [H.sapiens]//1.8e-19:136:87//Hs.106928:AI041737
R-NT2RP3002343//ESTs//1.0e-42:260:93//Hs.7797:W25667
R-NT2RP3002351//Homo sapiens 9G8 splicing factor mRNA, complete cds//0.0048:221:64//Hs.556:L41887
R-NT2RP3002352//Homo sapiens mRNA for protein encoded by cxorf5 (71-7A) gene//5.8e-105:516:94//Hs.6483:Y16355
R-NT2RP3002455//Homo sapiens mRNA for KIAA0678 protein, partial cds//1.5e-103:524:95//Hs.12707:AB014578
R-NT2RP3002484//Human APRT gene for adenine phosphoribosyltransferase//0.54:108:71//Hs.28914:Y00486
R-NT2RP3002501//ESTs//2.7e-96:489:95//Hs.27335:N74185
R-NT2RP3002512//ESTs, Weakly similar to HYPOTHETICAL 31.0 KD PROTEIN R107.2 IN CHROMOSOME III [C.elegans]//3.2e-90:526:90//Hs.8083:AA521436
R-NT2RP3002529//ESTs, Highly similar to PUTATIVE VACUOLAR PROTEIN SORTING-ASSOCIATED PROTEIN C2G11.03C [Schizosaccharomyces pombe]//3.8e-101:497:96//Hs.6650:AA843246
R-NT2RP3002545//Homo sapiens mRNA for KIAA0729 protein, partial cds//1.1e-83:438:94//Hs.19542:AB018272
R-NT2RP3002549//ESTs//3.8e-98:493:96//Hs.7358:AA191673
R-NT2RP3002566//Homo sapiens calcium-activated potassium channel (KCNN3) mRNA, complete cds//0.14:184:63//Hs.89230:AF031815
R-NT2RP3002587//Homo sapiens KIAA0420 mRNA, complete cds//2.0e-18:138:78//Hs.129883:AB007880
R-NT2RP3002590//ESTs//2.9e-51:290:93//Hs.162942:AI243850
R-NT2RP3002602//Homo sapiens stannin mRNA, complete cds//5.5e-06:58:100//Hs.76691:AF070673
R-NT2RP3002603
R-NT2RP3002631//ESTs//4.8e-54:367:85//Hs.13109:AA192514
R-NT2RP3002659//ESTs//5.3e-30:229:85//Hs.152114:AA401365
R-NT2RP3002660//ESTs//1.9e-88:452:95//Hs.120146:AA708573
R-NT2RP3002663//EST//3.2e-89:469:95//Hs.105767:AA525172
R-NT2RP3002671//ESTs, Highly similar to ELONGATION FACTOR 2 [Drosophila melanogaster]//5.9e-109:537:97//Hs.19348:AA151678
R-NT2RP3002682//ESTs//2.3e-98:541:91//Hs.75844:AA115502
R-NT2RP3002687//ESTs//5.5e-103:498:97//Hs.72782:AA910871
R-NT2RP3002688//ESTs, Weakly similar to !!!! ALU SUBFAMILY J WARNING ENTRY !!!! [H.sapiens]//5.0e-101:524:95//Hs.32580:AI123601
R-NT2RP3002701//EST//0.87:131:63//Hs.161916:AA483169
R-NT2RP3002713//ESTs//4.7e-106:542:95//Hs.14479:AA160945
R-NT2RP3002763//ESTs//1.3e-54:290:94//Hs.142031:AA809159
R-NT2RP3002770//ESTs//0.047:275:61//Hs.122984:AA526973
R-NT2RP3002785//ESTs//2.4e-52:255:99//Hs.132959:AI376958
R-NT2RP3002799//EST//8.2e-61:321:94//Hs.140992:R71377
R-NT2RP3002810//EST//0.19:116:68//Hs.121810:AA775240
R-NT2RP3002818//ESTs//1.3e-109:531:98//Hs.58924:AI348080
R-NT2RP3002861//ESTs//2.5e-84:429:95//Hs.23920:AA909678
R-NT2RP3002869//EST//0.00011:116:71//Hs.161606:AA019641
R-NT2RP3002876//ESTs//0.0024:182:63//Hs.117306:AA687262
R-NT2RP3002877//Homo sapiens X-ray repair cross-complementing protein 2 (XRCC2) mRNA, complete cds//8.1e-14:146:72//Hs.129727:AF035587
R-NT2RP3002909//Homo sapiens mRNA for KIAA0771 protein, partial cds//1.5e-110:570:95//Hs.6162:AB018314
R-NT2RP3002911//ESTs//3.6e-92:436:99//Hs.143917:AI206286
R-NT2RP3002948//EST//1.0:102:65//Hs.144730:AI191975
R-NT2RP3002953//ESTs//1.8e-107:513:98//Hs.119693:AI201698
R-NT2RP3002955//Homo sapiens mRNA, chromosome 1 specific transcript KIAA0492//0.23:563:56//Hs.127338:AB007961
R-NT2RP3002969//ESTS, Weakly similar to LONG-CHAIN-FATTY-ACID--COA LIGASE 1 [Saccharomyces cerevisiae]112.0e-56:387:86//Hs.144597:W20143
R-NT2RP3002972//ESTs//1.7e-97:502:96//Hs.7274:AA476850
R-NT2RP3002978//ESTs//8.6e-104:498:98//Hs.118923:AA252116
R-NT2RP3002988//EST//1.2e-59:315:94//Hs.157743:AI360553
R-NT2RP3003008//ESTs//1.4e-97:515:94//Hs.6544:AA524423
R-NT2RP3003032//ESTs, Weakly similar to RETROVIRUS-RELATED POL POLYPROTEIN [Mus musculus]//3.0e-100:528:94//Hs.90353:N98551
R-NT2RP3003059//ESTs//1.7e-76:398:95//Hs.102971:W05355
R-NT2RP3003061//ESTs//4.9e-82:414:96//Hs.99603:AI141912
R-NT2RP3003068//ESTs, Weakly similar to M18.3 [C.elegans]//5.9e-83:392:99//Hs.101364:AA534439
R-NT2RP3003071//ESTs//6.3e-85:399:99//Hs.109755:AA180809
R-NT2RP3003078//ESTs//1.0e-98:471:99//Hs.7995:AI359466
R-NT2RP3003101//EST//0.032:235:60//Hs.147920:AI202441
R-NT2RP3003121//ESTs//3.0e-47:238:97//Hs.43559:AI003520
R-NT2RP3003133//EST//1.5e-77:395:96//Hs.142150:AA223982
R-NT2RP3003138//ESTs, Highly similar to KINESIN-LIKE PROTEIN KIF4 [Mus musculus]//3.3e-107:535:96//Hs.27437:AA004208
R-NT2RP3003139//ESTs//2.5e-106:504:98//Hs.106795:AI271632
R-NT2RP3003150//ESTs//1.6e.99:539:91//Hs.46500:AA129774
R-NT2RP3003157//ESTs//1.5e-114:563:97//Hs.58608:AA081007
R-NT2RP3003185//ESTs//3.9e-93:443:98//Hs.9741:AI131226
R-NT2RP3003193//ESTs//2.0e-37:428:71//Hs.33354:AA179944
R-NT2RP3003197//ESTs//5.8e-56:312:94//Hs.7016:AA215796
R-NT2RP3003203//EST//0.0073:212:63//Hs.161355:AI422634
R-NT2RP3003204//ESTs//7.4e-52:253:99//Hs.120146:AA708573
R-NT2RP3003212//ESTs//1.8e-76:401:95//Hs.29067:N26107
R-NT2RP3003230//ESTs, Highly similar to CORONIN [Dictyostelium discoideum]//2.0e-40:229:93//Hs.17377:AI078151
R-NT2RP3003242//ESTs//8.3e-97:458:99//Hs.23057:AI290343
R-NT2RP3003251//ESTs//1.5e-60:320:95//Hs.36495:AA151628
R-NT2RP3003264//ESTs//2.1e-103:521:95//Hs.4094:AA173960
R-NT2RP3003278//ESTs//8.2e-109:536:96//Hs.23788:AA524061
R-NT2RP3003282//Homo sapiens dynamin (DNM) mRNA, complete cds//2.4e-102:550:93//Hs.11702:L36983
R-NT2RP3003290//EST//4.3e-27:372:70//Hs.159131:AI384035
R-NT2RP3003301//ESTs//4.4e-56:285:97//Hs.95370:AA601055
R-NT2RP3003302//EST//7.2e-10:395:63//Hs.162554:AA584818
R-NT2RP3003311//ESTs//4.2e-110:538:97//Hs.62180:AI341261
R-NT2RP3003313//ESTs//2.1e-106:531:96//Hs.22630:C05931
R-NT2RP3003327//ESTs//4.3e-102:518:95//Hs.120355:AA625445
R-NT2RP3003330//ESTs//8.6e-104:497:97//Hs.72071:AI125289
R-NT2RP3003344//ESTs//2.5e-105:494:99//Hs112188:AA872993
R-NT2RP3003346//ESTs//1.0:123:69//Hs.116029:AA813102
R-NT2RP3003353//EST//0.0014:162:68//Hs.149191:AI246155
R-NT2RP3003377//EST//4.5e-15:119:85//Hs.148129:AA885567
R-NT2RP3003384//EST//0.0057:86:74//Hs.127735:AA962272
R-NT2RP3003385//ESTs//0.64:347:59//Hs.5646:W72721
R-NT2RP3003403//ESTs, Weakly similar to LINE-1 REVERSE TRANSCRIPTASE HOMOLOG [H.sapiens]//2.2e-24:418:67//Hs.139488:AI124095
R-NT2RP3003409//ESTs//5.3e-98:479:97//Hs.155198:AA767372
R-NT2RP3003411//ESTs//4.8e-86:416:97//Hs.129059:AA126041
R-NT2RP3003427//ESTs//7.4e-103:510:96//Hs.25303:AA641023
R-NT2RP3003433//ESTs//3.5e-85:405:99//Hs.63131:AA664156
R-NT2RP3003464//Homo sapiens rab3-GAP regulatory domain mRNA, complete cds//3.6e-97:479:96//Hs.14934:AF004828
R-NT2RP3003490//Homo sapiens mRNA for KIAA0725 protein, partial cds//4.1e-102:527:93//Hs.26450:AB018268
R-NT2RP3003491//ESTs, Weakly similar to No definition line found [C.elegans]//4.0e-106:549:94//Hs.7886:AI057529
R-NT2RP3003500//Human RP3 mRNA, complete cds//0.66:401:60//Hs.75307:U02556
R-NT2RP3003543//Human clone A9A2BRB7 (CAC)n/(GTG)n repeat-containing mRNA//4.1e-33:217:88//Hs.8068:U00952
R-NT2RP3003552//ESTs//3.1e-106:546:94//Hs.101754:AI123430
R-NT2RP3003555//ESTs//3.4e-106:537:95//Hs.85550:AA187681
R-NT2RP3003564
R-NT2RP3003572//ESTs//1.2e-20:122:88//Hs.8253:N48721
R-NT2RP3003576//ESTs//2.7e-71:394:94//Hs.151136:R99944
R-NT2RP3003589//EST//0.58:242:59//Hs.130804:AA894759
R-NT2RP3003625//ESTs//7.6e-41:349:80//Hs.140608:N53448
R-NT2RP3003656//Human LIM protein (LPP) rnRNA, partial cds//0.26:222:60//Hs.17217:U49957
R-NT2RP3003659//ESTs//2.0e-113:547:97//Hs.23389:AA769310
R-NT2RP3003665//ESTs//1.6e-80:415:95//Hs.141084:H11714
R-NT2RP3003672
R-NT2RP3003686//ESTs//6.8e-114:552:97//Hs.43299:N23036
R-NT2RP3003701//ESTs//2.1e-16:282:66//Hs.115512:AI208768
R-NT2RP3003716//ESTs//2.1e-45:195:91//Hs.41296:N71923
R-NT2RP3003726//Homo sapiens mRNA for KIAA0757 protein, complete cds//5.6e-103:492:97//Hs.48513:AB018300
R-NT2RP3003746//ESTs//1.9e-85:411:98//Hs.54835:AI050863
R-NT2RP3003795//EST//6.2e-97:459:99//Hs.134769:AI089747
R-NT2RP3003799//ESTs//2.8e-62:337:94//Hs.124023:H18913
R-NT2RP3003800//PROTO-ONCOGENE TYRO SINE-PROTEIN KINASE SRC//8.9e-108:551:95//Hs.115742:AF077754
R-NT2RP3003805//ESTs//2.2e-103:490:99//Hs.9412:W72446
R-NT2RP3003809//ESTs, Highly similar to SAV PROTEIN [Sulfolobus acidocaldarius]//3.4e-89:456:95//Hs.5555:AI285198
R-NT2RP3003819//Interieukin 10//3.3e-43:173:89//Hs.2180:M57627
R-NT2RP3003825//ESTs//1.6e-66:485:80//Hs.7405:W27761
R-NT2RP3003828//ESTs, Weakly similar to unknown.[H.sapiens]//9.6e-98:511:95//Hs.26955:AI333224
R-NT2RP3003831//ESTs//2.2e-38:317:79//Hs.142173:AA757743
R-NT2RP3003833//Homo sapiens clones 24718 and 24825 mRNA sequence//5.2e-110:541:97//Hs.25300:AF070611
R-NT2RP3003842//EST//9.9e-44:506:70//Hs.139093:AA166888
R-NT2RP3003846//ESTs//4.6e-10:66:100//Hs.74924:AI332962
R-NT2RP3003870//ESTs//3.4e-82:449:92//Hs.122691:AA152298
R-NT2RP3003876//ESTs//1.9e-89:449:96//Hs.45046:N40170
R-NT2RP3003914//ESTs//1.3e-99:470:98//Hs.118966:AA926726
R-NT2RP3003918//ESTs//1.3e-79:417:94//Hs.5005:W25933
R-NT2RP3003932//ESTs//6.0e-83:427:94//Hs.93581:H50221
R-NT2RP3003989//ESTs//4.8e-76:403:93//Hs.127243:W80409
R-NT2RP3003992//ESTs//2.4e-88:508:90//Hs.134200:D19593
R-NT2RP3 004013//ESTs//3.7e-111:551:97//Hs.105108:AA781142
R-NT2RP3004016//ESTs//1.7e-81:394:98//Hs.63368:AA613714
R-NT2RP3004041
R-NT2RP3004051//ESTs//3.5e-69:386:93//Hs.51347:T72820
R-NT2RP3004070//ESTs//5.5e-108:552:9511Hs.23392:AI310139
R-NT2RP3004078//ESTs//3.3e-82:443:93//Hs.26407:W4537
R-NT2RP3004093//ESTs//4.4e-83:426:94//Hs,140932:AI262104
R-NT2RP3004095//ESTs//0.00013:93:78//Hs.36567:AA262045
R-NT2RP3004110//ESTs, Weakly similar to similar to oxysterol-binding proteins: partial CDS [C.elegans]//3.5e-76:402:95//Hs.55847:W31092
R-NT2RP3004125//ESTs//9.3e-74:363:97//Hs.32988:C01696
R-NT2RP3004145//ESTs//2.6e-96:451:99//Hs.59584:AA587334
R-NT2RP3004148//ESTs//1.3e-10:77:92//Hs.135890:AI183425
R-NT2RP3004155//ESTS//1.7e-110:558:96//Hs.27003:AI279093
R-NT2RP3004206//ESTs, Moderately similar to CROOKED NECK PROTEIN [Drosophila melanogaster]//1.8e-40:200:100//Hs.26089:AA195126
R-NT2RP3004207//ESTs, Weakly similar to gene SEZ-6 [M.musculus]//1.1e-41:266:89//Hs.6314:AA522619
R-NT2RP3004209//ESTs, Highly similar to PUTATIVE UBIQUITIN CARBOXYL-TERMINAL HYDROLASE C13A11.04C [Schizosaccharomyces pombe]//3.7e-112:547:97//Hs.99819:AI346680
R-NT2RP3004215//ESTs//1.1e-103:541:95//Hs.124918:N64794
R-NT2RP3004242//ESTs//4.5e-105:524:96//Hs.29724:N46252
R-NT2RP3004246//EST//1.9e-07:67:91//Hs.125687:AA884827
R-NT2RP3004253//EST//2.9e-88:454:94//Hs.127713:AA961628
R-NT2RP3004258//ESTs, Weakly similar to PRE-MRNA SPLICING FACTOR SRP75 [Homo sapiens]//1.6e-89:468:95//Hs.5117:AA831530
R-NT2RP3004262//ESTs//4.1e-86:443:96//Hs.101393:T87623
R-NT2RP3004334//EST//0.00057:206:63//Hs.149388:AI273630
R-NT2RP3004341//EST//0.00042:151:68//Hs.148498:AI200264
R-NT2RP3004348//Homo sapiens LIM protein mRNA, complete cds//5.9e-61:299:85//Hs.154103:AF061258
R-NT2RP3004349//EST//3.6e-42:175:88//Hs.161917:AA483223
R-NT2RP3004378//ESTs//0.27:294:60//Hs.66479:AA863044
R-NT2RP3004399//ESTs//5.8e-99:479:98//Hs.120234:AA732224
R-NT2RP3004424//EST, Highly similar to F21G4.6 [C.elegans]//0.30:253:58//Hs.97184:AA385934
R-NT2RP3004428//ESTs//2.8e-48:279:91//Hs.106826:W25985
R-NT2RP3004451//ESTs//4.8e-101:509:96//Hs.29725:W74621
R-NT2RP3004454//Homo sapiens mRNA for KIAA0448 protein, complete cds//9.3e-108:526:98//Hs.27349:AB007917
R-NT2RP3004466//ESTs//0.25:51:90//Hs.7778:AA195616
R-NT2RP3004470//EST//0.032:70:71//Hs.147925:AI249332
R-NT2RP3004472//ESTs//0.0069:430:59//Hs.116651:AA993406
R-NT2RP3004475//Homo sapiens mRNA for KIAA0456 protein, partial cds//5.0e-107:521:97//Hs.5003:AB007925
R-NT2RP3004480
R-NT2RP3004490//ESTs//4.7e-68:354:95//Hs.163721:H42504
R-NT2RP3004498//ESTs, Moderately similar to ORF2: function unknown [H.sapiens]//3.4e-100:508:95//Hs.47393:AA218858
R-NT2RP3004503//ESTs//4.6e-90:478:93//Hs.133998:AA994735
R-NT2RP3004504//ESTs, Highly similar to cytoplasmic polyadenylation element-binding protein [M.musculus]//1.8e-83:465:92//Hs.137064:AA318257
R-NT2RP3004507//ESTs//1.5e-98:495:96//Hs.128905:AI051971
R-NT2RP3004527//EST//1.6e-109:535:97//Hs.149481:AI279865
R-nnnnnnnnnnnn
R-NT2RP3004544//EST//0.035:226:60//Hs.99195:AA449232
R-NT2RP3004566//ESTs//4.1e-86:455:95//Hs.13110:T67461
R-NT2RP3004569//ESTs//2.9e-94:493:94//Hs.24948:AA977674
R-NT2RP3004572//ESTs//1.1e-92:437:99//Hs.24846:AI420493
R-NT2RP3004578//ESTs//0.98:166:64//Hs.124593:AA854456
R-NT2RP3004594//EST//5.8e-89:426:98//Hs.134213:AI080213
R-NT2RP3004617//ESTs//1.4e-40:226:85//Hs.15921:R71157
R-NT2RP3004618//ESTs//1.8e-38:229:90//Hs.125153:AA453723
R-NT2RP3004670//Homo sapiens GN6ST mRNA for long form of N-acetylglucosamine-6-O-sulfotransferase (GlcNAc6ST), complete cds//7.2e-57:291:95//Hs.8786:AB014680
R-NT2RP4000008//ESTs//8.9e-119:561:98//Hs.25035:AI123335
R-NT2RP4000023//EST//1.2e-34:271:80//Hs.98300:AA418560
R-NT2RP4000035//Small inducible cytokine A5 (RANTES)//2.1e-68:320:82//Hs.155464:AF088219
R-NT2RP4000049//Homo sapiens TRAIL receptor 2 mRNA, complete cds//6.7e-60:289:82//Hs.51233:AF016266
R-NT2RP4000051//ESTs, Weakly similar to protein B [H.sapiens]//8.3e-98:462:99//Hs.10114:AI345945
R-NT2RP4000078//ESTs//0.00068:367:60//Hs.106090:AA457030
R-NT2RP4000102//ESTs//9.7e-50:256:97//Hs.24266:R28287
R-NT2RP4000109//Homo sapiens mRNA for MEGF5, partial cds//1.1e-107:536:96//Hs.57929:AB011538
R-NT2RP4000129//Homo sapiens mRNA for KIAA0483 protein, partial cds//3.5e-112:554:97//Hs.64691:AB007952
R-NT2RP4000147//ESTs//3.9e-11:122:80//Hs.25584:AA632014
R-NT2RP4000150//EST//4.4e-84:510:88//Hs.144238:W52294
R-NT2RP4000151//ESTs, Weakly similar to HYPOTHETICAL 31.0 KD PROTEIN R107.2 IN CHROMOSOME III [C.elegans]//5.7e-93:515:92//Hs.8083:AA521436
R-NT2RP4000159//ESTs//0.0019:209:65//Hs.161816:AA400295
R-NT2RP4000167//ESTs//2.1e-113:549:97//Hs.109441:N66569
R-NT2RP4000185//ESTs//0.65:232:59//Hs.144445:AA807257
R-NT2RP4000210//Homo sapiens mRNA for KIAA0700 protein, partial cds//1.5e-100:505:96//Hs.13999:AB014600
R-NT2RP4000212//ESTs//8.5e-14:169:75//Hs.8520:AA081788
R-NT2RP4000214//Human mRNA for KIAA0392 gene, partial cds//6.2e-43:272:90//Hs.40100:AB002390
R-NT2RP4000218//ESTs//6.1e-10:335:64//Hs.105658:AA978185
R-NT2RP4000243//Homo sapiens mRNA for cartilage-associated protein (CASP)//2.9e-70:354:96//Hs.155481:AJ006470
R-NT2RP4000246//ESTs//7.1e-26:154:94//Hs.14838:AA502757
R-NT2RP4000259//Homo sapiens clone 683 unknown mRNA, complete sequence//9.3e-79:379:99//Hs.43728:AF091092
R-NT2RP4000263
R-nnnnnnnnnnnn/ESTs, Weakly similar to similar to Achlya ambisexualis antheridiol steroid receptor [C.elegans]//4.7e-104:525:96//Hs.152069:AA548972
R-NT2RP4000312//ESTs//8.2e-66:319:99//Hs.35091:AI271631
R-NT2RP4000321//Homo sapiens clone 24453 mRNA sequence//1.3e-109:513:99//Hs.13410:AF070524
R-NT2RP4000323//ESTs//7.7e-109:534:97//Hs.34790:AA192760
R-NT2RP4000355//ESTs//3.1e-44:320:83//Hs.141323:N80390
R-NT2RP4000360//Homo sapiens mRNA for KIAA0738 protein, complete cds//7.6e-111:520:99//Hs.107479:AB018281
R-NT2RP4000367//Homo sapiens IkappaB kinase complex associated protein (IKAP) mRNA, complete cds//2.8e-110:527:98//Hs.31323:AF044195
R-NT2RP4000370//ESTs//8.9e-32:166:98//Hs.70488:AI301130
R-NT2RP4000376//ESTs//6.8e-99:465:99//Hs.27182:AA604498
R-NT2RP4000381//ESTs//3.0e-50:280:93//Hs.8395:W27376
R-NT2RP4000415//ESTs, Weakly similar to coded for by C. elegans cDNA yk30b3.5 [C.elegans]//3.9e-87:499:91//Hs.26156:AA630975
R-NT2RP4000417//ESTs, Moderately similar to HYPOTHETICAL 91.2 KD PROTEIN IN RPS7A-SCH9 INTERGENIC REGION [Saccharomyces cerevisiae]//8.9e-95:468:96//Hs.93871:AI191318
R-NT2RP4000424//ESTs//3.7e-98:473:98//Hs.24945:AI189011
R-NT2RP4000448//ESTs//2.6e-79:446:91//Hs.25159:R60955
R-NT2RP4000449//ESTs//3.6e-98:468:98//Hs.31176:AI037953
R-NT2RP4000455//Homo sapiens N-methyl-D-aspartate receptor 2D subunit precursor (NMDAR2D) mRNA, complete cds//0.35:153:63//Hs.113286:U77783
R-nnnnnnnnnnnn//ESTs//4.5e-89:455:96//Hs.62638:AA127740
R-NT2RP4000480//ESTs//4.9e-92:431:99//Hs.121072:AI204167
R-nnnnnnnnnnnn
R-NT2RP4000500//ESTs, Weakly similar to HYPOTHETICAL 83.6 KD PROTEIN R05D3.2 IN CHROMOSOME III [C.elegans]//1.2e-40:125:97//Hs.56124:AI424792
R-NT2RP4000515//EST//6.7e-30:183:90//Hs.150710:AI122713
R-NT2RP4000517//Aldehyde dehydrogenase 7//7.5e-28:183:76//Hs.83155:U10868
R-NT2RP4000518//EST//0.091:178:58//Hs.133031:AI049874
R-NT2RP4000519
R-NT2RP4000524//ESTS, Highly similar to rsec8 [R.norvegicus]//3.4e-93:496:93//Hs.107394:H07126
R-NT2RP4000528//EST//0.84:130:66//Hs.140208:AA702213
R-NT2RP4000541//EST//5.2e-63:337:94//Hs.156337:AI337328
R-NT2RP4000556//ESTs, Highly similar to 60S RIBOSOMAL PROTEIN L11 [R.norvegicus]//8.2e-92:448:98//Hs.25597:H93026
R-NT2RP4000588//ESTs//3.8e-94:445:98//Hs.44077:N28840
R-NT2RP4000614//ESTs//6.5e-18:159:83//Hs.24549:N57263
R-NT2RP4000638//ESTs//2.5e-46:296:87//Hs.132722:AA618531
R-NT2RP4000648//ESTs//2.6e-103:559:93//Hs.23794:W80393
R-NT2RP4000657//ESTs//1.0:189:60//Hs.87073:AA972704
R-NT2RP4000704//ESTs//2.8e-101:509:96//Hs.84824:AA935651
R-NT2RP4000724//ESTS//1.5e-83:442:94//Hs.142114:AA205615
R-NT2RP4000728//ESTs//0.84:61:75//Hs.145334:AI251399
R-NT2RP4000739//ESTs//8.8e-80:418:94//Hs.42959:N21211
R-NT2RP4000781//ESTs//1.4e-79:376:99//Hs.135458:AI081312
R-NT2RP4000817//Homo sapiens mRNA for KIAA0470 protein, complete cds//3.1e-106:550:94//Hs.25132:AB007939
R-NT2RP4000833//ESTs//5.8e-46:309:85//Hs.163979:AA828834
R-NT2RP4000837//ESTs//1.7e-112:539:97//Hs.97718:AI334028
R-NT2RP4000855//ESTs//1.1e-95:486:95//Hs.5345:AA988104
R-NT2RP4000865//EST//6.2e-68:412:89//Hs.142196:AA258356
R-NT2RP4000878//ESTs//1.9e-80:417:95//Hs.104716:AI023185
R-NT2RP4000879//ESTs//1.8e-42:211:99//Hs.89991:AI374617
R-nnnnnnnnnnn//ESTs//1.2e-89:453:97//Hs.100182:N92594
R-nnnnnnnnnnnn//EST//9.4e-06:197:63//Hs.145970:AI277106
R-NT2RP4000925//ESTs, Weakly similar to KIAA0405 [H.sapiens]//5.9e-17:134:85//Hs.14146:W92235
R-nnnnnnnnnnnn//ESTs//4.3e-14:84:100//Hs.155360:AA984683
R-NT2RP4000928//Homo sapiens CDP-diacylglycerol synthase 2 (CDS2) mRNA, partial cds//8.2e-108:548:95//Hs.24812:AF069532
R-NT2RP4000929//ESTs//1.3e-119:567:98//Hs.62717:AA044905
R-NT2RP4000955//ESTs//3.5e-10:l 19:78//Hs.42946:N21111
R-NT2RP4000973//ESTs//2.8e-05:93:69//Hs.155126:AA563986
R-NT2RP4000975//ESTs//4.4e-58:324:95//Hs.126070:AA045179
R-NT2RP4000979//ESTs//3.5e-42:468:73//Hs.106210:AI193017
R-NT2RP4000984//Homo sapiens clone 23770 mRNA sequence//8.7e-120:570:98//Hs.12457:AF052123
R-NT2RP4000989//ESTs//1.3e-122:581:98//Hs.10499:AA528018
R-NT2RP4000996//ESTs//9.2e-113:579:94//Hs.23762:N26620
R-NT2RP4000997//Homo sapiens neuronal thread protein AD7c-NTP mRNA, complete cds//1.1e-28:439:68//Hs.129735:AF010144
R-NT2RP4001004//ESTs//3.6e-78:389:98//Hs.156290:AI016769
R-NT2RP4001006//ESTS, Moderately similar to ORF2: function unknown [H.sapiens]//6.6e-124:574:99//Hs.47393:AA218858
R-NT2RP4001010//EST//2.8e-31:194:90//Hs.161186:AI418635
R-NT2RP4001029//ESTs//4.4e-111:523:99//Hs.28423:AI336292
R-NT2RP4001041//ESTs, Highly similar to LEUCYL-TRNA SYNTHETASE, CYTOPLASMIC [Saccharomyces cerevisiae]//3.6e-114:569:96//Hs.6762:AA088424
R-NT2RP4001057//Homo sapiens KIAA0399 mRNA, partial cds//2.0e-51:282:94//Hs.100955:AB007859
R-NT2RP4001064//ESTs, Weakly similar to protein B [H.sapiens]//2.1e-103:485:99//Hs.10114:AD45945
R-NT2RP4001078
R-NT2RP4001079//Homo sapiens mRNA for putative Ca2⁺-transporting ATPase, partial/1.7e-119:569:98//Hs.106778:AJ010953
R-NT2RP4001080//ESTs//7.6e-10:65:100//Hs.131694:AA927668
R-nnnnnnnnnnnn//Homo sapiens mRNA for KIAA0592 protein, partial cds//5.9e-121:548:95//Hs.13273:AB011164
R-NT2RP4001095//ESTs//1.5e-113:563:96//Hs.118732:AI344055
R-NT2RP4001100//ESTs//2.0e-46:413:79//Hs.146314:R99617
R-NT2RP4001117//EST//7.4e-51:294:92//Hs.7260:T23737
R-NT2RP4001122//ESTs//5.4e-109:509:99//Hs.16390:AI052357
R-NT2RP4001126//EST//0.97:169:61//Hs.148107:AA693476
R-NT2RP4001138//ESTs//3.0e-110:543:97//Hs.57655:AI056890
R-NT2RP4001143//ESTs, Highly similar to HYPOTHETICAL 52.9 KD PROTEIN IN SAP155-YMR31 INTERGENIC REGION [Saccharomyces cerevisiae]//5.4e-113:573:96//Hs.5249:U55977
R-NT2RP4001148//ESTs//3.1e-103:490:98//Hs.121282:AI091453
R-NT2RP4001149//EST//1.7e-50:281:93//Hs.101727:H16171
R-NT2RP4001150//ESTS//1.9e-90:422:100//Hs.125490:AI138884
R-NT2RP4001159
R-NT2RP4001174//ESTs//2.5e-110:526:98//Hs.116555:AA639278
R-nnnnnnnnnnnn//ESTs//1.1 e-25:140:97//Hs.83756:AI002822
R-NT2RP4001207//ESTs//4.4e-70:432:89//Hs.13109:AA192514
R-NT2RP4001210//ESTs//1.4e-108:509:99//Hs.27021:AI359495
R-NT2RP4001213//ESTs, Highly similar to ZINC FINGER PROTEIN 8 [Homo sapiens]//4.4e-123:624:95//Hs.22744:AI379892
R-NT2RP4001219//ESTs//0.0043:142:65//Hs.6733:AI160750
R-NT2RP4001228//ESTs//4.9e-101:482:98//Hs.62684:AA806103
R-NT2RP4001235//ESTs//3.7e-105:571:93//Hs.37706:AA005120
R-NT2RP4001256//ESTs//1.1e-12:189:74//Hs.20621:W28255
R-NT2RP4001260//EST//6.9e-05:313:61//Hs.116438:AA648430
R-NT2RP4001274//EST//0.0020:246:63//Hs.149955:AI289933
R-nnnnnnnnnnnn//ESTs//2.9e-34:213:91//Hs.43100:AA186588
R-NT2RP4001313
R-NT2RP4001315//EST//6.1e-38:217:93//Hs.97832:AA400892
R-NT2RP4001339//ESTs//3.8e-91:430:99//Hs.34840:AI279612
R-NT2RP4001345//ESTs//5.3e-89:443:96//Hs.6770:AA972732
R-NT2RP4001351//ESTs//6.0e-78:394:97//Hs.102796:N70837
R-NT2RP4001353//ESTs//4.8e-06:90:82//Hs.7778:AA195616
R-NT2RP4001372
R-NT2RP4001373//ESTs, Weakly similar to HYPOTHETICAL 48.8 KD PROTEIN IN TRK2-MRS4 INTERGENIC REGION [Saccharomyces cerevisiae]//1.7e-108:546:96//Hs.32271:AA203680
R-NT2RP4001375//ESTs//2.4e-19:155:87//Hs.62119:AA043299
R-NT2RP4001379//EST//4.4e-29:288:72//Hs.157848:AI362501
R-NT2RP4001389//ESTs, Highly similar to HYPOTHETICAL 51.6 KD PROTEIN IN PAP1-MRPL13 INTERGENIC REGION [Saccharomyces cerevisiae]//3.8e-79:438:93//HS.21938:W81045
R-NT2RP4001407//ESTs//8.3e-112:541:97//Hs.22587:AA743132
R-NT2RP4001414//ESTs//8.6e-18:117:90//Hs.90789:W27649
R-NT2RP4001433//ESTs, Moderately similar to PROHIBITIN [H.sapiens]//1.6e-102:498:97//Hs.62386:AA512948
R-NT2RP4001442//ESTs//8.8e-104:489:99//Hs.101619:AI339433
R-NT2RP4001447
R-NT2RP4001474
R-NT2RP4001483//ESTs//2.1e-100:528:92//Hs.17860:AA706655
R-NT2RP4001498//ESTs//1.1e-97:470:98//Hs.95744:AI392846
R-NT2RP4001502//ESTs//6.7e-73:382:96//Hs.11874:N93511
R-NT2RP4001507//ESTs//2.6e-57:302:96//Hs.65328:AA625385
R-NT2RP4001524//ESTs, Weakly similar to F13B12.1 [C.elegans]//2.9e-107:546:96//Hs.5570:AI377863
R-NT2RP4001529//ESTs//3.3e-112:524:99//Hs.28423:AI336292
R-NT2RP4001547//ESTs, Weakly similar to NADH-UBIQUINONE OXIDOREDUCTASE CHAIN 5 [Paramecium tetraurelia]//2.8e-120:566:98//Hs.108530:AA523928
R-nnnnnnnnnnnn//ESTs, Weakly similar to CELL DIVISION CONTROL PROTEIN 68 [S.cerevisiae]//1.4e-26:184:88//Hs.136189:AA133224
R-NT2RP4001555//ESTs//1.1e-95:445:100//Hs.134403:AA677552
R-NT2RP4001567//ESTs//2.8e-106:506:98//Hs.102708:AA292285
R-NT2RP4001568//ESTs//6.4e-55:300:94//Hs.57442:N63437
R-NT2RP4001571//ESTs//1.3e-114:556:97//Hs.30340:AA521251
R-NT2RP4001574//ESTs//0.0035:120:67//Hs.96339:AA225906
R-NT2RP4001575
R-NT2RP4001592//ESTs, Weakly similar to ISOLEUCYL-TRNA SYNTHETASE, MITOCHONDRIAL[S.cerevisiae]//8.7e-112:557:97//Hs.7558:AA526812
R-NT2RP4001610//ESTs//6.2e-77:382:96//Hs.21543:AA166776
R-NT2RP4001614//ESTs//2.8e-117:565:98//Hs.9591:AA069657
R-NT2RP4001634//ESTs//2.0e-39:213:96//Hs.32360:AA534737
R-NT2RP4001638//Homo sapiens clone 23967 unknown mRNA, partial cds//1.7e-116:559:97//Hs.5332:AF007151
R-NT2RP4001644//ESTs, Moderately similar to MNK1 [H.sapiens]//5.3e-36:192:97//Hs.5662:AA868361
R-NT2RP4001656//ESTs, Highly similar to HYPOTHETICAL 108.5 KD PROTEIN R06F6.2 IN CHROMOSOME II [Caenorhabditis elegans]//1.1e-104:525:96//Hs.20472:W28734
R-NT2RP4001677//ESTs//1.8e-106:522:97//Hs.106390:AA156805
R-NT2RP4001696//Human chromosome 8 BAC clone CIT987SK-2A8 complete sequence//5.7e-118:583:96//Hs.15562:U96629
R-NT2RP4001725//ESTs//2.0e-11:141:74//Hs.117589:N25941
R-nnnnnnnnnnnn//ESTs, Weakly similar to UDP-GLUCOSE:GLYCOPROTEIN GLUCOSYLTRANSFERASE PRECURSOR [D.melanogaster]//3.4e-73:362:97//Hs.152332:AI141922
R-NT2RP4001739//ESTs//6.6e-59:340:91//Hs.122293:AA843692
R-NT2RP4001753//Zinc finger protein 3 (A8-51)//5.6e-113:552:96//Hs.2481:X78926
R-NT2RP4001760//ESTs//2.5e-94:453:98//Hs.122579:AA766315
R-NT2RP4001790//ESTs, Weakly similar to ZINC FINGER PROTEIN 84 [H.sapiens]//2.0e-62:326:94//Hs.110839:W28098
R-NT2RP4001803
R-NT2RP4001822//ESTs//4.4e-98:526:92//Hs.96908:AI161133
R-NT2RP4001823//ESTs//1.7e-72:357:97//Hs.144900:AI218434
R-NT2RP4001828//ESTs//3.3e-101:536:92//Hs.18851:AA857826
R-NT2RP4001838//ESTs//4.2e-58:344:90//Hs.48723:N66663
R-NT2RP4001849//EST//0.24:105:71//Hs.136747:AA749210
R-NT2RP4001889//Human mRNA for KIAA0118 gene, partial cds//3.4e-34:212:88//Hs.154326:D42087
R-NT2RP4001893//ESTs//3.0e-58:321:95//Hs.158787:W79602
R-NT2RP4001896//EST//3.8e-15:108:92//Hs.160835:AI345528
R-NT2RP4001901//ESTs//1.2e-110:536:97//Hs.31443:AI018606
R-NT2RP4001927//ESTs//2.1e-105:546:93//Hs.73291:AI417099
R-NT2RP4001938//ESTs//2.8e-40:235:78//Hs.163641:R61848
R-NT2RP4001946//ESTs//1.3e-29:175:93//Hs.43703:AA088436
R-NT2RP4001950//ESTs//4.6e-95:458:98//Hs.150890:AI341793
R-NT2RP4001953//Clathrin, light polypeptide (Lcb)//2.3e-62:310:82//Hs.73919:X81637
R-NT2RP4001966//ESTs, Weakly similar to tenascin-like protein [D.melanogaster]//8.3e-87:457:94//Hs.41793:AA775879
R-NT2RP4001975//ESTs//1.9e-52:281:94//Hs.7704:W58252
R-NT2RP4002018
R-NT2RP4002047//ESTs, Highly similar to GTP-BINDING PROTEIN LEPA [Pseudomonas fluorescens]//4.7e-09:90:86//Hs.41127:AA555184
R-NT2RP4002052//ESTs//0.054:353:60//Hs.117510:AA903738
R-NT2RP4002058//EST//7.8e-26:151:94//Hs.124617:AA855106
R-NT2RP4002071//ESTs//6.9e-99:475:98//Hs.29216:AA916679
R-NT2RP4002075//ESTs//0.67:121:65//Hs.153939:AI284198
R-NT2RP4002078//ESTs, Highly similar to ZINC FINGER PROTEIN 35 [Homo sapiens]//1.6e-61:464:82//Hs.144228:N99507
R-nnnnnnnnnnnn//ESTs, Weakly similar to HYPOTHETICAL 139.1 KD PROTEIN C08B11.3 IN CHROMOSOME II [C.elegans]//2.3e-56:271:100//Hs.6185:AA428565
R-NT2RP4002083//ESTs//2.0e-108:548:96//Hs.6120:W80407
R-NT2RP4002408//ESTs//2.6e-77:391:96//Hs.14014:AA745592
R-NT2RP4002791//ESTs//7.9e-101:527:93//Hs.22394:N32555
R-NT2RP4002888//ESTs, Highly similar to ENV POLYPROTEIN [Avian spleen necrosis virus]//1.9e-65:373:92//Hs.31532:H18272
R-NT2RP4002905//ESTs//1.5e-107:517:98//Hs.40460:N36090
R-OVARC1000001//Homo sapiens mRNA for KIAA0465 protein, partial cds//2.8e-115:605:94//Hs.108258:AB007934
R-OVARC1000004
R-OVARC1000006//ESTs//1.5e-19:139:89//Hs.143034:AI126929
R-OVARC1000013//ESTs//5.9e-98:531:93//Hs.16470:AA121635
R-OVARC1000014//ESTs//0.24:243:60//Hs.19569:AA464273
R-OVARC1000017
R-OVARC1000035//ESTs//0.035:252:63//Hs.134123:AI078286
R-OVARC1000058//H.sapiens mRNA for translin associated protein X//3.8e-46:331:83//Hs.96247:X95073
R-OVARC1000060//EST//2.8e-28:348:71//Hs.141728:W73041
R-OVARC1000068//ESTs//3.0e-83:491:90//Hs.29397:N51367
R-OVARC1000071//ESTs//2.5e-60:321:96//Us.25010:R6787
R-OVARC1000085//Proteasome component C5//8.6e-67:366:92//Hs.75748:AL031259
R-nnnnnnnnnnnn//ESTs//1.0e-111:526:98//Hs.129020:AI380703
R-OVARC1000091//ESTS, Weakly similar to HOST CELL FACTOR Cl [H.sapiens]//3.9e-112:596:94//Hs.20597:W58370
R-OVARC1000092//ESTs//5.1e-18:144:82//Hs.109140:AI289942
R-OVARC1000106
R-OVARC1000113//Homo sapiens okadaic acid-inducible phosphoprotein (OA48-18) mRNA, complete cds//8.3e-102:495:97//Hs.3688:AF069250
R-OVARC1000114//H.sapiens mRNA for phosphoinositide 3-kinase//1.7e-45:489:74//Hs.101238:Y11312
R-OVARC1000133//EST//0.00028:284:61//Hs.30547:H05482
R-OVARC1000145//EST//3.9e-40:201:99//Hs.156148:AI333214
R-OVARC1000148//EST//0.79:150:62//Hs.100078:T05090
R-OVARC1000151
R-OVARC1000168//EST//1.7e-19:142:90//Hs.38441:H66023
R-OVARC1000191//EST//0.0072:292:63//Hs.132492:AA922629
R-OVARC1000198//Homo sapiens LIM protein mRNA, complete cds//6.1e-44:339:81//Hs.154103:AF061258
R-OVARC1000209//ESTs, Moderately similar to ZINC FINGER PROTEIN 93 [H.sapiens]//1.1e-32:196:92//Hs.64322:AA142864
R-OVARC1000212//EST//0.20:178:61//Hs.133031:AI049874
R-OVARC1000240//ESTs//9.0e-64:314:98//Hs.42300:AA204958
R-OVARC1000241//EST//0.00018:115:68//Hs.150728:AI123130
R-OVARC1000288//ESTs, Highly similar to HYPOTHETICAL 54.2 KD PROTEIN IN CDC12-ORC6 INTERGENIC REGION [Saccharomyces cerevisiae]//3.3e-74:403:93//Hs.108117:AI097079
R-OVARC1000302//EST//4.0e-14:102:90//Hs.136617:AA630476
R-OVARC1000304//ESTs, Highly similar to PUTATIVE GTP-BINDING PROTEIN MOV10 [Mus musculus]//2.9e-37:191:98//Hs.20725:AI027777
R-OVARC1000309//ESTs//3.6e-66:348:94//Hs.9547:AA532449
R-OVARC1000321//ESTs//3.6e-87:454:95//Hs.110445:AA044743
R-OVARC1000326//ESTs, Moderately similar to lamina associated polypeptide 1C [R.norvegicus]//1.3e-98:488:96//Hs.125749:AI377682
R-OVARC1000335//ESTs//3.0e-115:565:97//Hs.54835:AI050863
R-OVARC1000347//EST//0.0018:145:65//Hs.136945:AA765672
R-OVARC1000384//ESTs//2.8e-38:253:89//Hs.15093:AA203423
R-OVARC1000408//ESTs//2.6e-98:515:94//Hs.119808:C05928
R-OVARC1000411//ESTs//3.2e-82:395:98//Hs.104747:AA406219
R-OVARC1000414//Landsteiner-Wiener blood group glycoprotein//1.5e-27:211:79//Hs.108287:L27670
R-OVARC1000420//EST//2.8e-38:255:74//Hs.138525:R99237
R-OVARC1000427//EST//2.6e-58:302:96//Hs.122914:AA767034
R-OVARC1000431//ESTs//4.9e-108:551:96//Hs.11668:AI123426
R-OVARC1000437
R-OVARC1000440//ESTs//2.9e-91:456:96//Hs.93701:AI018671
R-OVARC1000442//Human high-affinity copper uptake protein (hCTR1) mRNA, complete cds//4.3e-45:320:84//Hs.73614:U83460
R-OVARC1000443//Homo sapiens mRNA for KIAA0683 protein, complete cds//3.6e-79:418:94//Hs.12334:AB014583
R-OVARC1000461//ESTs//3.1e-62:342:93//Hs.23241:R46582
R-OVARC1000465//ESTs//1.7e-67:349:95//Hs.127238:AA477576
R-OVARC1000466//ESTs//1.9e-66:337:95//Hs.5212:AI421211
R-OVARC1000473//ESTs//5.4e-89:320:99//Hs.29173:AA134926
R-OVARC1000479//ESTs, Highly similar to TIP120 [R.norvegicus]//1.1e-102:514:96//Hs.11833:AI299947
R-OVARC1000486//ESTs//3.9e-78:405:95//Hs.98312:AA424983
R-OVARC1000496
R-OVARC1000520//ESTs//1.2e-20:145:88//Hs.87456:AA434484
R-OVARC1000526//Small inducible cytokine A5 (RANTES)//8.9e-47:217:87//Hs.155464:AF088219
R-OVARC1000533//ESTs, Moderately similar to integrase [H.sapiens]//8.5e-48:264:92//Hs.49860:AA702248
R-OVARC1000543//ESTs//5.7e-74:410:94//Hs.62817:AA047021
R-OVARC1000556//H.sapiens mRNA for ribosomal S6 kinase//9.5e-27:202:85//Hs.90859:X85106
R-OVARC1000557//EST//2.8e-18:169:79//Hs.149101:AI244285
R-OVARC1000564//EST//2.3e-34:199:92//Hs.146637:AI141587
R-OVARC1000573//Interleukin 10//4.7e-42:300:83//Hs.2180:M57627
R-OVARC1000578//Small inducible cytokine A5 (RANTES)//5.2e-58:392:84//Hs.155464:AF088219
R-OVARC1000588//EST//1.8e-41:174:85//Hs.163333:AA879053
R-OVARC1000605
R-OVARC1000622//Homo sapiens mRNA, chromosome 1 specific transcript KIAA0501//6.4e-47:417:77//Hs.159897:AB007970
R-OVARC1000640//H.sapiens mRNA for translin associated protein X//1.9e-28:366:72//Hs.96247:X95073
R-OVARC1000661//Homo sapiens mRNA for KIAA0590 protein, complete cds//5.1e-31:162:100//Hs.111862:AB011162
R-OVARC1000678//EST//0.92:199:60//Hs.122025:AA778480
R-nnnnnnnnnnnn//ESTs//0.94:416:59//Hs.130754:AA279522
R-OVARC1000681//EST//9.2e-21:179:80//Hs.132635:AI032875
R-OVARC1000689//Homo sapiens ataxin-7 (SCA7) mRNA, complete cds//0.053:160:64//Hs.108447:AJ000517
R-OVARC1000700//Homo sapiens KIAA0441 mRNA, complete cds//7.1e-09:141:73//Hs.32511:AB007901
R-OVARC1000703//ESTs//1.7e-46:298:87//Hs.138856:H47461
R-OVARC1000730//ESTs, Weakly similar to C27F2.7 gene product [C.elegans]//1.7e-17:137:86//Hs.7049:AI141736
R-OVARC1000746//ESTs//0.16:366:60//Hs.136969:AA830918
R-OVARC1000769//ESTs, Weakly similar to eukaryotic initiation factor eIF-2 alpha kinase [D.melanogaster]//4.6e-28:430:69//Hs.42457:AA523306
R-OVARC1000771//ESTs//1.3e-87:461:94//Hs.22399:AA531016
R-OVARC1000781//ESTs//8.3e-119:572:97//Hs.41972:AA626793
R-OVARC1000787//ESTs//7.4e-18:115:93//Hs.164036:AA845659
R-OVARC1000800//MITOCHONDRIAL STRESS-70 PROTEIN PRECURSOR//4.9e-19:119:95//Hs.3069:L11066
R-OVARC1000802//ESTs//2.2e-41:383:78//Hs.161228:AI419764
R-OVARC1000834//Homo sapiens mRNA for atopy related autoantigen CALC//1.2e-106:536:95//Hs.61628:Y17711
R-OVARC1000846//Clathrin, light polypeptide (Lcb)//1.6e-66:282:87//Hs.73919:X81637
R-OVARC1000850//Homo sapiens PB39 mRNA, complete cds//1.2e-115:579:96//Hs.18910:AF045584
R-OVARC1000862//EST//4.3e-14:129:81//Hs.150663:AA923096
R-OVARC1000876//ESTs//1.0e-115:573:96//Hs.87287:AI150674
R-OVARC1000883//ESTs//3.5e-109:523:98//Hs.28423:AI336292
R-OVARC1000885//ESTs, Highly similar to HYPOTHETICAL OXIDOREDUCTASE IN ROCC-PTA INTERGENIC REGION [Bacillus subtilis]//7.9e-98:525:93//Hs.10366:W21953
R-OVARC1000886//ESTs//8.2e-79:417:94//Hs.7729:AA830777
R-OVARC1000891//ESTs//6.8e-75:401:94//Hs.5833:H15401
R-OVARC1000897//ESTs//3.5e-91:440:98//Hs.125264:AA873350
R-OVARC1000912
R-OVARC1000915//ESTs//1.0e-45:328:82//Hs.163980:AA715814
R-OVARC1000924//ESTs//1.0e-100:501:96//Hs.30204:AA497127
R-OVARC1000936//EST//3.0e-74:367:98//Hs.145098:AA421696
R-OVARC1000937//EST//1.1e-53:290:95//Hs.162846:AA631215
R-OVARC1000945//ESTs//4.9e-51:301:89//Hs.20100:W25794
R-OVARC1000948//ESTs//3.7e-67:332:98//Hs.112570:AA621971
R-OVARC1000959//Small inducible cytokine A5 (RANTES)//7.2e-44:283:86//Hs.155464:AF088219
R-OVARC1000960//Homo sapiens KIAA0395 mRNA, partial cds//1.1e-41:348:80//Hs.43681:AL022394
R-OVARC1000971//EST//6.2e-05:126:70//Hs.160491:AI254909
R-OVARC1000984//ESTS, Weakly similar to No definition line found [C.elegans]//3.5e-68:346:96//Hs.25544:AA532784
R-OVARC1000996//EST//0.12:92:71//Hs.117141:AA678811
R-OVARC1000999//Homo sapiens KIAA0414 mRNA, partial cds//1.5e-44:513:73//Hs.127649:AB007874
R-OVARC1001000//ESTs//1.8e-22:198:80//Hs.140608:N53448
R-OVARC1001004//Human kpni repeat mrna (cdna clone pcd-kpni-4), 3' end//1.7e-28:181:77//Hs.139107:K00629
R-OVARC1001010//EST//2.1e-09:92:85//Hs.147893:AI223270
R-OVARC1001011//EST//2.4e-14:200:75//Hs.149290:AI248117
R-OVARC1001032//EST//2.7e-29:304:73//Hs.141733:W80630
R-OVARC1001034//Homo sapiens apoptotic protease activating factor 1 (Apaf-1) mRNA, complete cds//2.1e-09:137:74//Hs.77579:AF013263
R-OVARC1001038//Homo sapiens TRIAD1 type I mRNA, complete cds//4.1e-101:501:96//Hs.9899:AF099149
R-OVARC1001040//ESTs//2.9e-87:415:99//Hs.132812:AI032046
R-OVARC1001044//ESTs//1.1e-83:432:96//Hs.55043:N94384
R-OVARC1001051//60S RIBOSOMAL PROTEIN L41//1.2e-16:124:88//Hs.108124:Z12962
R-OVARC1001055//ESTs//2.4e-23:238:76//Hs.141421:H99231
R-OVARC1001062//ESTs//3.4e-92:469:96//Hs.34658:N98652
R-OVARC1001068//Homo sapiens Era GTPase A protein (HERA-A) mRNA, partial cds//7.3e-97:463:98//Hs.3426:AF082657
R-OVARC1001072//ESTs//1.3e-34:227:89//Hs.126704:W95844
R-OVARC1001074
R-OVARC1001085//Human T-cell leukemia virus enhancer factor//1.0:94:69//Hs.103126:U57029
R-OVARC1001092//Homo sapiens mRNA for JM5 protein, complete CDS (clone IMAGE 53337, LLNLc110F1857Q7 (RZPD Berlin) and LLNLc110G0913Q7 (RZPD Berlin))//1.4e-96:325:98//Hs.21753:AJ005897
R-OVARC1001113//Homo sapiens diaphanous 1 (HDIA1) mRNA, complete cds//3.3e-75:386:95//Hs.26584:AF051782
R-OVARC1001117//Human G protein-coupled receptor (STRL22) mRNA, complete cds//3.9e-37:283:84//Hs.46468:U45984
R-OVARC1001118//ESTs//5.3e-99:485:97//Hs.130815:AA936548
R-OVARC1001129//ESTs//9.8e-66:351:95//Hs.18616:T99312
R-OVARC1001l61//ESTs, Moderately similar to !!!! ALU SUBFAMILY SX WARNING ENTRY !!!! [H.sapiens]//2.2e-66:346:95//Hs.53263:AA173226
R-OVARC1001162//EST//1.5e-44:376:80//Hs.161917:AA483223
R-OVARC1001167//ESTs//4.7e-110:548:96//Hs.35254:AI133727
R-OVARC1001169//ESTs//0.22:152:68//Hs.149424:AI274200
R-OVARC1001170//Small inducible cytokine A5 (RANTES)//1.8e-42:305:84//Hs.155464:AF088219
R-OVARC1001173//EST//2.5e-35:182:84//Hs.161917:AA483223
R-OVARC1001180//Human macrophage-derived chemokine precursor (MDC) mRNA, complete cds//6.6e-64:247:80//Hs.97203:U83171
R-OVARC1001188//ESTs//4.1e-18:296:69//Hs.139197:AA228343
R-OVARC1001200//ESTs//2.0e-28:207:85//Hs.35121:AA877826
R-OVARC1001232//ESTs//3.2e-61:358:91//Hs.6449:W95025
R-OVARC1001240//ESTs//6.7e-45:316:85//Hs.121675:AA629668
R-OVARC1001243//ESTs//2.3e-86:409:99//Hs.163091:AA742361
R-OVARC1001261//ESTs//0.63:125:64//Hs.155743:AI344166
R-OVARC1001268//ESTs//8.1e-20:113:98//Hs.109477:AA477929
R-OVARC1001270//ESTs//1.5e-107:530:97//Hs.62905:AA460708
R-OVARC1001271//ESTs//4.5e-36:401:72//Hs.20190:AA525532
R-OVARC1001282//EST//4.0e-91:428:99//Hs.145599:AI263113
R-OVARC1001296//ESTs//2.6e-63:301:100//Hs.125753:AA740885
R-nnnnnnnnnnnnn//Homo sapiens mRNA for KIAA0518 protein, partial cds//3.8e-70:334:100//Hs.23763:AB011090
R-OVARC1001329//Clathrin, light polypeptide (Lcb)//1.3e-68:304:83//Hs.73919:X81637
R-OVARC1001330//Proline arginine-rich end leucine-rich repeat protein//1.0:147:63//Hs.76494:U41344
R-OVARC1001339//Small inducible cytokine A5 (RANTES)//5.0e-48:452:76//Hs.155464:AF088219
R-OVARC1001341//ESTs, Moderately similar to !!!! ALU SUBFAMILY SQ WARNING ENTRY !!!! [H.sapiens]//6.9e-85:464:93//Hs.23651:AA650356
R-OVARC1001342//40S RIBOSOMAL PROTEIN S8//4.9e-110:568:95//Hs.118690:X67247
R-OVARC1001344//EST//3.6e-44:341:81//Hs.162197:AA535216
R-OVARC1001357//TUMOR-ASSOCIATED ANTIGEN L6//9.8e-44:250:93//Hs.3337:M90657
R-OVARC1001360//ESTs//5.2e-110:534:98//Hs.24743:AA843844
R-OVARC1001369//ESTs//1.7e-98:478:97//Hs.7729:AA830777
R-OVARC1001372//ESTs//2.6e-97:456:99//Hs.153648:AI341415
R-OVARC1001376//Homo sapiens mRNA for KIAA0575 protein, complete cds//1.1e-53:344:72//Hs.153468:AB011147
R-OVARC1001381//ESTs//5.1e-19:200:66//Hs.114031:AA700958
R-OVARC1001391
R-nnnnnnnnnnnn//ESTs//0.003 9:48:95//Hs.117964:N20913
R-OVARC1001417//Homo sapiens EXLM1 mRNA, complete cds//3.2e-111:561:95//Hs.21586:AB006651
R-OVARC1001419
R-OVARC1001425//EST//5.7e-20:395:66//Hs.159707:AI393136
R-OVARC1001436//ESTs//9.6e-90:427:99//Hs.6982:AA622427
R-OVARC1001442//ESTs//1.1e-66:317:100//Hs.18437:AI206345
R-OVARC1001453//ESTs//2.0e-20:163:84//Hs.133503:AA628592
R-OVARC1001476//EST//0.23:125:66//Hs.71444:AA131700
R-OVARC1001480//ESTs//3.1e-56:181:97//Hs.40109:AA928694
R-OVARC1001489//ESTs//1.0:297:58//Hs.86723:AA393089
R-OVARC1001496//Homo sapiens C-terminal binding protein 2 mRNA, complete cds//3.0e-117:585:96//Hs.6534:AF016507
R-OVARC1001506//Small inducible cytokine A5 (RANTES)//1.8e-48:283:90//Hs.155464:AF088219
R-OVARC1001525//EST//0.80:170:60//Hs.157398:AI364539
R-OVARC1001542//Homo sapiens hJTB mRNA, complete cds//1.6e-111:566:95//Hs.6396:AB016492
R-OVARC1001547//ESTs//5.7e-105:564:93//Hs.68835:AA088388
R-OVARC1001577//Homo sapiens SRp46 splicing factor retropseudogene mRNA7/4.4e-20:150:89//Hs.155160:AF031166
R-OVARC1001600//Human mRNA for KIAA0118 gene, partial cds//8.6e-21:282:72//Hs.154326:D42087
R-OVARC1001610//ESTs//4.6e-108:555:95//Hs.44295:N32019
R-OVARC1001611//ESTs//0.0021:117:71//Hs.135568:AA972965
R-OVARC1001615//Homo sapiens KIAA0409 mRNA, partial cds//9.2e-19:114:78//Hs.5158:AB007869
R-OVARC1001668//ESTs//1.0:127:69//Hs.153290:AI022659
R-OVARC1001702//ESTs//4.8e-44:225:97//Hs.96855:AA346854
R-OVARC1001703//ESTs//2.3e-89:426:99//Hs.27099:W60080
R-OVARC1001711//ESTs//1.9e-57:251:99//Hs.9732:AA527784
R-OVARC1001726//ESTs, Highly similar to APICAL PROTEIN [Xenopus laevis]//1.2e-27:236:81//Hs.15485:AA046954
R-OVARC1001731//Tropomyosin4(fibroblast)//7.9e-74:422:90//Hs.102824:X05276
R-OVARC1001745//Human mRNA for tryptophan hydroxylase (EC 1.14.16.4)//1.7e-62:300:83//Hs.144563:AF057280
R-nnnnnnnnnnnnn//ESTs, Weakly similar to N-TERMINAL ACETYLTRANSFERASE 1 [S.cerevisiae]//6.8e-100:540:92//Hs.117741:AA903456
R-OVARC1001766//Homo sapiens eukaryotic translation initiation factor eIF3, p35 subunit mRNA, complete cds//1.1e-109:567:94//Hs.155377:U97670
R-nnnnnnnnnnnnn//Homo sapiens mRNA for KIAA0675 protein, complete cds//2.0e-109:529:97//Hs.15869:AB014575
R-OVARC1001768//ESTs//3.5e-59:327:94//Hs.107923:H66127
R-OVARC1001791//ESTs//1.3e-111:565:96//Hs.6107:AA160604
R-OVARC1001795//ESTs//2.8e-97:526:93//Hs.72158:AA156978
R-OVARC1001802//Homo sapiens DEC-205 mRNA, complete cds//4.8e-36:276:81//Hs.153563:AF011333
R-OVARC1001805//ESTs//4.1e-78:375:98//Hs.126902:AI374688
R-OVARC1001812//EST//4.8e-45:349:80//Hs.162677:AA604831
R-OVARC1001813//Homo sapiens mRNA for KIAA0538 protein, partial cds//2.1e-15:519:63//Hs.25639:AB011110
R-OVARC1001820//ESTs//9.5e-50:314:80//Hs.140491:W52705
R-OVARC1001828//ESTs//0.11:186:63//Hs.29055:AI374621
R-OVARC1001846//ESTs//0.34:134:66//Hs.152992:AI242160
R-OVARC1001861//ESTs//2.3e-19:120:92//Hs.42225:N31809
R-OVARC1001873//Homo sapiens clones 24718 and 24825 mRNA sequence/1.9e-105:571:91//Hs.25300:AF070611
R-OVARC1001879//EST//1.3e-24:185:85//Hs.136617:AA630476
R-OVARC1001880//Homo sapiens mRNA for KIAA0575 protein, complete cds//2.2e-49:302:90//Hs.153468:AB011147
R-OVARC1001883//ESTs//1.0e-51:295:93//Hs.164059:AA447310
R-OVARC1001900//Homo sapiens tumorous imaginal discs protein Tid56 homolog (TID1) mRNA, complete cds//1.6e-87:346:90//Hs.6216:AF061749
R-OVARC1001901//ESTs//6.8e-24:132:98//Hs.130797:AA904435
R-OVARC1001911//ESTs//1.1e-88:491:92//Hs.32343:W73855
R-OVARC1001916//ESTs//7.9e-97:491:95//Hs.24989:H97842
R-OVARC1001928
R-OVARC1001942//ESTs, Weakly similar to N-TERMINAL ACETYLTRANSFERASE 1 [S.cerevisiae]//2.5e-39:253:88//Hs.117741:AA903456
R-OVARC1001943//ESTs//9.3e-13:78:100//Hs.143680:W38637
R-OVARC1001949//ESTs, Highly similar to ZINC FINGER PROTEIN 8 [Homo sapiens]//8.3e-96:498:94//Hs.22744:AI379892
R-OVARC1001950//EST//1.3e-35:236:81//Hs.132635:AI032875
R-OVARC1001987//ESTs//5.6e-94:514:92//Hs.21148:AI183729
R-OVARC1001989//ESTs//9.7e-46:228:99//Hs.127046:AA935887
R-OVARC1002044//ESTS//3.4e-45:303:85//Hs.132722:AA618531
R-OVARC1002050//Homo sapiens mRNA for KIAA0465 protein, partial cds//4.4e-109:542:96//Hs.108258:AB007934
R-OVARC1002066//ESTs//8.5e-97:455:99//Hs.135477:AI088556
R-OVARC1002082//Homo sapiens mRNA for KIAA0772 protein, complete cds//8.1e-47:340:82//Hs.15519:AB018315
R-OVARC1002107//ESTs//5.9e-103:498:98//Hs.157207:AA629860
R-OVARC1002127//ESTs//3.0e-87:419:98//Hs.127833:AI347130
R-OVARC1002138//ESTs, Weakly similar to HYPOTHETICAL 54.7 KD PROTEIN C07A9.1 IN CHROMOSOME III [Caenorhabditis elegans]//1.7e-102:485:98//Hs.137516:AA805691
R-OVARC1002143//ESTs//1.3e-79:428:92//Hs.158126:W26825
R-OVARC1002156//ESTs//1.6e-38:198:98//Hs.22957:AA478923
R-OVARC1002158//ESTs//7.3e-81:412:96//Hs.12211:AA908631
R-OVARC1002165//ESTs//1.8e-09:154:72//Hs.49354:AA424160
R-OVARC1002182//ESTs//4.3e-80:465:91//Hs.77067:AA040478
R-PLACE1000004//ESTs, Weakly similar to TEICHOIC ACID BIOSYNTHESIS PROTEIN A [Bacillus subtilis]//7.5e-32:164:99//Hs.144194:AA706337
R-PLACE1000005//EST//0.37:212:60//Hs.127020:AA934920
R-PLACE1000007//Homo sapiens clone 24422 mRNA sequence//3.8e-16:100:97//Hs.109268:AF070557
R-PLACE1000014//EST//9.6e-44:344:77//Hs.161917:AA483223
R-PLACE1000031//ESTs//2.2e-32:374:70//Hs.117969:H94870
R-PLACE1000040//ESTs//0.00017:316:59//Hs.23342:AI310440
R-PLACE1000048//Human Line-1 repeat mRNA with 2 open reading frames//4.8e-79:519:86//Hs.23094:M19503
R-PLACE100005011ESTs//9.7e-90:453:96//Hs.27410:N25612
R-PLACE1000061//Ribosomal protein L37a//5.5e-22:126:97//Hs.1946:L06499
R-PLACE1000066//ESTs, Weakly similar to coded for by C. elegans cDNA yk10c10.3 [C.elegans]//1.4e-61:331:94//Hs.30026:AI356771
R-PLACE1000078//ESTs//2.6e-30:212:85//Hs.89312:AA167659
R-PLACE1000081
R-PLACE1000094
R-PLACE1000133//ESTs//4.4e-87:448:94//Hs.93748:AA884505
R-PLACE1000142//ESTs, Weakly similar to enoyl-CoA hydratase [H.sapiens]//5.5e-103:538:94//Hs.9670:AA632135
R-PLACE1000184//Homo sapiens estrogen-related receptor gamma mRNA, complete cds//4.1e-114:594:94//Hs.151017:AF058291
R-PLACE1000185//ESTs, Weakly similar to No definition line found [C.elegans]//2.0e-19:114:95//Hs.7036:W22072
R-PLACE1000213//ESTs//9.4e-99:494:96//Hs.24398:AI262946
R-PLACE1000214//ESTs//5.3e-98:466:98//Hs.28661:AA805916
R-PLACE1000236//Human BENE mRNA, partial cds//1.7e-19:162:84//Hs.85889:U17077
R-PLACE1000246//EST//0.026:134:66//Hs.135611:Z21545
R-PLACE1000292//ESTs//2.5e-80:418:96//Hs.138233:N57912
R-PLACE1000332//EST//1.7e-82:422:96//Hs.118637:T61940
R-PLACE1000347//ESTs//8.5e-36:180:100//Hs.6377:AA632424
R-PLACE1000374//ESTs//2.8e-90:434:98//Hs.161785:AI423126
R-PLACE1000380//ESTs//1.0e-81:399:97//Hs.47105:AI334994
R-PLACE1000383//ESTs//3.7e-75:405:94//Hs.23200:AA203708
R-PLACE1000401//ESTs//1.4e-16:212:72//Hs.151665:AA020959
R-PLACE1000406//ESTs//2.1e-51:259:97//Hs.129651:N53089
R-PLACE1000420//ESTs//7.7e-92:471:95//Hs.144407:AA737799
R-PLACE1000421//ESTs//2.9e-14:282:67//Hs.142068:AA176125
R-PLACE1000424//EST//2.9e-35:453:70//Hs.162404:AA573131
R-PLACE1000435//Homo sapiens protein phosphatase with EF-hands-2 long form (PPEF-2) mRNA, complete cds//1.6e-47:472:77//Hs.113259:AF023456
R-PLACE1000444//ESTs, Moderately similar to platelet glycoprotein IIb precursor [H.sapiens]//2.0e-58:410:81//Hs.97579:AA398118
R-PLACE1000453//ESTs//2.3e-85:442:95//Hs.9725:AA039793
R-PLACE1000481//ESTS, Weakly similar to Ndr protein kinase [H.sapiens]//3.2e-109:549:95//Hs.19074:U69566
R-PLACE1000492//ESTs, Highly similar to vacuolar protein sorting homolog r-vps33b [R.norvegicus]//3.5e-83:435:94//Hs.26510:AA700425
R-PLACE1000540//ESTs//3.2e-58:281:99//Hs.118270:AA844729
R-PLACE1000547//Homo sapiens mRNA for KIAA0640 protein, partial cds//2.2e-32:208:88//Hs.153026:AB014540
R-PLACE1000562//ESTs, Weakly similar to HYPOTHETICAL 23.0 KD PROTEIN IN IXR1-TFA1 INTERGENIC REGION [Saccharomyces cerevisiae]//1.9e-26:220:81//Hs.163791:W25348
R-PLACE1000564//ESTs//1.1e-54:302:92//Hs.158520:AI380485
R-PLACE1000583//Human mRNA for KIAA0355 gene, complete cds//5.5e-43:404:75//Hs.153014:AB002353
R-nnnnnnnnnnnn//Guanylate binding protein 1, interferon-inducible, 67kD//6.1e-79:542:82//Hs.62661:M55542
R-PLACE1000596//ESTs//0.0028:364:59//Hs.106090:AA457030
R-PLACE1000599//Human mRNA for KIAA0118 gene, partial cds//4.3e-49:295:90//Hs.154326:D42087
R-PLACE1000610//ESTs//0.0010:104:74//Hs.17413:N45301
R-PLACE1000636//ESTs//1.8e-64:340:95//Hs.100895:AA479308
R-PLACE1000653//Homo sapiens N-acetylglucosamine-phosphate mutase mRNA, complete cds//5.3e-101:506:96//Hs.5819:AF102265
R-PLACE1000656//Homo sapiens mRNA for JM4 protein, complete CDS (clone IMAGE 546750 and LLNLc110F1857Q7 (RZPD Berlin))//1.4e-102:559:92//Hs.29595:AJ005896
R-PLACE1000706//Homo sapiens transcription intermediary factor 1 (TIF1) mRNA, complete cds//2.8e-10:281:64//Hs.128763:AF009353
R-PLACE1000712//ESTs//7.8e-60:317:95//Hs.8245:AA115485
R-PLACE1000716
R-PLACE1000748//ESTs//8.9e-87:466:93//Hs.25245:AA176701
R-PLACE1000749//EST//0.019:186:61//Hs.135443:AI077396
R-PLACE1000755//ESTs, Weakly similar to HYPOTHETICAL HELICASE K12H4.8 IN CHROMOSOME III [C.elegans]//3.9e-40:224:94//Hs.87889:AA262008
R-PLACE1000769//Homo sapiens clone 24566 mRNA sequence//6.5e-27:531:66//Hs.133342:AF070536
R-PLACE1000785//Homo sapiens mRNA for KIAA0648 protein, partial cds//8.5e-103:513:96//Hs.31921:AB014548
R-PLACE1000786//ESTs//5.2e-93:449:97//Hs.58389:W74482
R-nnnnnnnnnnnn//H.sapiens mRNA for chemokine HCC-1//0.88:201:60//Hs.20144:AF088219
R-PLACE1000798//ESTs//1.1e-97:508:94//Hs.139119:N32189
R-PLACE1000841//ESTs, Highly similar to guanine nucleotide regulatory protein [H.sapiens]//7.7e-31:220:86//Hs.117576:R33135
R-nnnnnnnnnnnn//ESTs//1.8e-87:459:94//Hs.43100:AA186588
R-PLACE1000856//ESTs//0.0084:224:59//Hs.145906:AI275039
R-PLACE1000863//ESTs, Highly similar to PUTATIVE 40S RIBOSOMAL PROTEIN YHR148W [Saccharomyces cerevisiae]//2.2e-92:467:95//Hs.6118.-AI141558
R-PLACE1000909//ESTs//4.7e-89:435:97//Hs.95744:AI392846
R-PLACE1000931//EST//1.9e-28:261:73//Hs.135545:AI097091
R-PLACE1000948//ESTs//0.034:329:58//Hs.114851:AA608697
R-PLACE1000972//EST//3.3e-24:264:74//Hs.130321:AI002941
R-PLACE1000977//EST//0.085:153:65//Hs.131646:AI025689
R-PLACE1000979
R-PLACE1001000//ESTs//4.7e-56:284:96//Hs.117978:AA810725
R-PLACE1001007//ESTs, Moderately similar to MNK1 [H.sapiens]//5.2e-63:343:93//Hs.5662:AA868361
R-PLACE1001010//EST//0.96:53:71//Hs.96973:AA351146
R-PLACE1001015//Oxytocin receptor//2.8e-25:308:71//Hs.2820:X64878
R-PLACE1001024//ESTs//5.0e-12:79:96//Hs.97910:AA404736
R-PLACE1001036//ESTs//4.0e-15:301:65//Hs.137947:AI025762
R-PLACE1001062//ESTs//5.2e-15:199:73//Hs.138982:AA056120
R-PLACE1001076//ESTs//3.9e-84:406:98//Hs.115455:AA678124
R-PLACE1001088//ESTs//3.0e-106:518:97//Hs.158964:AA639580
R-PLACE1001092//Homo sapiens SEC63 (SEC63) mRNA, complete cds//0.035:259:59//Hs.31575:AF100141
R-PLACE1001104//ESTs//6.1e-115:582:95//Hs.10972:AA164268
R-PLACE1001118//ESTs//6.9e-81:440:93//Hs.5383:AA913610
R-PLACE1001136//ESTs//7.4e-41:168:83//Hs.95115:AA206594
R-PLACE1001168//ESTs//3.9e-21:116:99//Hs.5897:AA148834
R-PLACE1001171//ESTs, Highly similar to CYTOCHROME B-245 LIGHT CHAIN [H.sapiens]//0.91:77:71//Hs.115211:AA287527
R-PLACE1001185//ESTs//1.5e-65:330:96//Hs.26368:AA789297
R-PLACE1001238//ESTs, Moderately similar to RNA polymerase I associated factor [M.musculus]//1.9e-99:512:94//Hs.24884:AA176812
R-PLACE1001241//ESTs//1.1e-81:446:93//Hs.42278:AI073464
R-PLACE1001257//EST//6.4e-46:298:87//Hs.162404:AA573131
R-PLACE1001272//ESTs//0.31:158:61//Hs.42960:N95371
R-PLACE1001279//ESTs//1.8e-77:376:97//Hs.29276:AA427780
R-PLACE1001280//ESTs//1.1e-30:134:89//Hs.163492:AI334460
R-PLACE1001294//ESTs, Moderately similar to GAMETOGENESIS EXPRESSED PROTEIN GEG-154 [M.musculus]//2.7e-22:181:84//Hs.48320:AA149548
R-PLACE1001304//ESTs, Weakly similar to ZINC FINGER PROTEIN 135 [H.sapiens]//4.2e-34:195:92//Hs.86276:W27601
R-PLACE1001311//ESTs//9.1e-91:438:97//Hs.41055:AI339056
R-PLACE1001323//Human transmembrane 4 superfamily protein (SAS) mRNA, complete cds//5.5e-44:215:86//Hs.50984:U01160
R-PLACE1001351//ESTs//2.4e-101:494:97//Hs.23944:AI097077
R-PLACE1001366//Small inducible cytokine A5 (RANTES)//8.7e-43:284:85//Hs.155464:AF088219
R-PLACE1001377//Homo sapiens ADAM10 (ADAM10) mRNA, complete cds//2.3e-81:431:93//Hs.152005:AF009615
R-PLACE1001383//Homo sapiens clone 24538 mRNA sequence/1.0e-36:192:97//Hs.12342:AF055030
R-PLACE1001384//Homo sapiens multi PDZ domain protein MUPP1 (MUPP1) mRNA, complete cds//1.0e-86:456:94//Hs.21301:AF093419
R-PLACE1001387//ESTs//6.0e-74:383:94//Hs.55016:AI298280
R-PLACE1001395//ESTs//2.3e-94:473:95//Hs.22394:N32555
R-PLACE1001399//ESTs//2.6e-41:204:100//Hs.24462:N36348
R-PLACE1001412//Homo sapiens clone 643 unknown mRNA, complete sequence//2.6e-45:242:95//Hs.110404:AF091087
R-PLACE1001414//ESTs//0.0013:77:75//Hs.144614:AA291800
R-PLACE1001440
R-PLACE1001456//EST//0.76:120:62//Hs.34011:H48115.
R-PLACE1001468//ESTs//4.0e-80:403:96//Hs.131832:AI017547
R-PLACE1001484//ESTs//3.0e-16:201:72//Hs.153413:AI248625
R-PLACE1001502//ESTs//8.1e-31:161:99//Hs.126264:AA455617
R-PLACE1001503//ESTs//2.4e-37:176:81//Hs.141581:AA315361
R-PLACE1001517//Homo sapiens hGAAl mRNA, complete cds//2.1e-57:339:90//Hs.4742:AB006969
R-PLACE1001534//ESTs//3.6e-61:304:97//Hs.45207:AI042153
R-PLACE1001545/TESTs, Weakly similar to !!!! ALU SUBFAMILY J WARNING ENTRY !!!! [H.sapiens]//1.6e-22:170:85//Hs.155456:AA707265
R-PLACE1001551//ESTs//1.5e-39:202:98//Hs.139269:AA894431
R-PLACE1001570//EST//1.1e-70:495:82//Hs.144234:W52249
R-PLACE1001602//EST//0.33:297:57//Hs.149839:AI287601
R-PLACE1001603//ESTs//2.0e-17:181:76//Hs.155334:AA827904
R-PLACE1001610//EST//1.1e-86:442:95//Hs.112580:AA608683
R-PLACE1001611//Homo sapiens histone macroH2A1.2 mRNA, complete cds//1.1e-42:217:97//Hs.75258:AF054174
R-PLACE1001632//ESTs, Highly similar to ZINC FINGER PROTEIN 91 [Homo sapiens]//1.5e-78:458:91//Hs.114547:AA167095
R-PLACE1001634//ESTs//0.0035:40:97//Hs.101577:AI168526
R-PLACE1001640//ESTs//0.0028:377:57//Hs.131044:D61640
R-PLACE10016727/ESTs, Moderately similar to !!!! ALU SUBFAMILY J WARNING ENTRY !!!! [H.sapiens]//0.98:141:62//Hs.153060:AA195804
R-PLACE1001691//Homo sapiens okadaic acid-inducible phosphoprotein (OA48-18) mRNA, complete cds//4.7e-113:545:97//Hs.3688:AF069250
R-PLACE1001692//EST//3.0e-43:430:75//Hs.162975:AA679124
R-PLACE1001705//ESTs//3.0e-81:418:94//Hs.22646:AI374903
R-PLACE1001716//EST//0.76:150:62//Hs.128906:AA983667
R-PLACE1001720//ESTs//2.4e-64:385:90//Hs.60455:AA010993
R-PLACE1001729//ESTs//2.9e-84:418:96//Hs.134740:AA282171
R-PLACE1001739//ESTs, Weakly similar to P68 PROTEIN [H.sapiens]//9.1e-32:206:89//Hs.6366:AA614113
R-PLACE1001740//EST//6.5e-05:113:68//Hs.139949:AA644266
R-PLACE1001745//ESTs//3.3e-92:473:95//Hs.104270:AA236479
R-PLACE1001746//ESTs//8.8e-93:443:98//Hs.112198:AI423937
R-PLACE1001748//Homo sapiens metalloprotease 1 (MP1) mRNA, complete cds//4.1e-93:540:89//Hs.4812:AF061243
R-PLACE1001756//ESTs//0.17:157:66//Hs.141565:N64662
R-PLACE1001761
R-PLACE1001771//ESTs//0.92:165:62//Hs.473 87:N51980
R-PLACE1001781//ESTs//5.7e-84:437:95//Hs.23363:AA081236
R-PLACE1001799//EST//0.00039:126:65//Hs.123267:AA807352
R-PLACE1001817//Homo sapiens ATP-specific succinyl-CoA synthetase beta subunit (SCS) mRNA; partial cds//1.3e-93:463:95//Hs.40820:AF058953
R-PLACE1001821//Small inducible cytokine A5 (RANTES)//2.7e-35:328:75//Hs.155464:AF088219
R-PLACE1001845
R-PLACE1001869//EST//1.0:207:62//Hs.137298:W32868
R-PLACE1001897//ESTs//2.4e-23:219:80//Hs.7503:H50009
R-PLACE1001912//ESTs//1.5e-32:162:78//Hs.136810:AA789098
R-PLACE1001920//Homo sapiens TNF-induced protein GG2-1 mRNA, complete cds//3.9e-74:363:97//Hs.17839:AF099936
R-PLACE1001928//Homo sapiens mRNA for KIAA0623 protein, complete cds//0.85:130:66//Hs.151406:AB014523
R-PLACE1001983//ESTs//2.8e-66:334:96//Hs.110155:AA007313
R-PLACE1001989//ESTs//1.3e-88:453:95//Hs.132717:AA171941
R-PLACE1002046
R-PLACE1002052//ESTs//1.7e-79:428:94//Hs.6737:N32595
R-PLACE1002066//ESTs//2.8e-82:427:94//Hs.132972:AA543094
R-PLACE1002072//ESTs//0.27:108:66//Hs.123163:AA809619
R-PLACE1002073//EST//5.5e-70:369:95//Hs.132339:AI028552
R-PLACE1002090//ESTs//6.3e-73:361:96//Hs.134469:AA731632
R-PLACE1002115//ESTs//4.6e-34:233:88//Hs.163443:R23311
R-PLACE1002119//ESTs//1.2e-88:444:96//Hs.15725:AA521293
R-PLACE1002140//ESTs//6.6e-22:118:100//Hs.22793:W91937
R-PLACE1002150//ESTs//4.0e-96:465:98//Hs.7312:AI167614
R-PLACE1002157//EST, Weakly similar to LINE-1 REVERSE TRANSCRIPTASE HOMOLOG[H.sapiens]//3.6e-39:400:76//Hs.162172:AA534189
R-PLACE1002163//ESTs//3.2e-83:428:95//Hs.137011:AI185965
R-PLACE1002171//ESTs//5.3e-68:392:90//Hs.62273:AA143745
R-PLACE1002205//ESTS//1.5e-39:211:95//Hs.28338:N48793
R-PLACE1002213//ESTs//5.1e-38:290:83//Hs.146811:AA410788
R-PLACE1002227//EST//1.3e-14:214:72//Hs.46979:N49892
R-PLACE1002256//ESTs//2.4e-100:484:98//Hs.9343:AI004257
R-PLACE1002259//Human Line-1 repeat mRNA with 2 open reading frames//5.8e-67:501:81//Hs.23094:M19503
R-PLACE1002319//ESTs//1.4e-28:17 8:92//Hs.7353:AA209308
R-PLACE1002342//Homo sapiens mRNA for KIAA0728 protein, partial cds//1.6e-95:501:93//Hs.18277:AB018271
R-PLACE1002395//ESTs//3.6e-25:248:77//Hs.3853:AA034291
R-PLACE1002399//ESTs//1.5e-27:238:78//Hs.13014:W26381
R-PLACE1002433//ESTs//4.3e-108:511:98//Hs.98324:AA621959
R-PLACE1002437//EST//1.2e-06:158:61//Hs.159833:T24110
R-PLACE1002438//Sjogren syndrome antigen B (autoantigen La)//0.93:176:60//Hs.83715:X69804
R-PLACE1002450//ESTs//1.5e-89:432:98//Hs.47371:AA136333
R-PLACE1002465//ESTS//1.6e-92:488:93//Hs.78110:AA741320
R-PLACE1002474//Human matrilin-2 precursor mRNA, partial cds//4.9e-23:166:85//Hs.19368:U69263
R-PLACE1002477//ESTs//2.5e-62:305:98//Hs.88605:AA421132
R-PLACE1002493//Homo sapiens signal transducing adaptor molecule 2A (STAM2) mRNA, complete cds//3.6e-55:307:91//Hs.17200:AF042273
R-PLACE1002499//ESTs//7.4e-72:373:96//Hs.128221:AA972429
R-PLACE1002500//Homo sapiens KIAA0409 mRNA, partial cds//1.2e-40:296:83//Hs.5158:AB007869
R-PLACE1002514//ESTs, Weakly similar to !!!! ALU SUBFAMILY SB1 WARNING ENTRY !!!! [H.sapiens]//6.4e-14:217:69//Hs.152230:AI140609
R-PLACE1002529//Homo sapiens mRNA for KIAA0713 protein, partial cds//5.1e-88:582:85//Hs.88756:AB018256
R-PLACE1002532//Homo sapiens BAC clone RG300E22 from 7q21-q31.1//2.7e-19:116:93//Hs.99348:AC004774
R-PLACE1002537//ESTs//4.8e-93:440:99//Hs.164005:AA766491
R-PLACE1002571//ESTs, Highly similar to ACTIN-LIKE PROTEIN 13E [Drosophila melanogaster]//1.3e-108:555:95//Hs.23259:AA532437
R-PLACE1002578//EST//1.9e-40:337:81//Hs.162404:AA573131
R-PLACE1002583//EST//1.2e-07:264:65//Hs.156414:AI339738
R-PLACE1002591//ESTs//2.3e-67:372:94//Hs.143046:N73778
R-PLACE1002598//ESTs, Highly similar to PROTEIN HI1715 [Haemophilus influenzae]//1.2e-44:228:97//Hs.7527:AA843208
R-PLACE1002604//ESTs//3.3e-106:532:96//Hs.86828:AA632147
R-PLACE1002625//EST//3.8e-13:173:74//Hs.138597:H77749
R-PLACE1002665//Small inducible cytokine A4 (homologous to mouse Mip-1b)//1.0:189:58//Hs.75703:J04130
R-PLACE1002685//Homo sapiens B cell linker protein BLNK mRNA, alternatively spliced, complete cds//3.8e-79:390:97//Hs.124903:AF068180
R-PLACE1002714//ESTs//8.2e-63:340:93//Hs.7973:H19830
R-PLACE1002722//ESTs, Weakly similar to putative G-protein-coupled receptor [H.sapiens]//6.8e-75:445:90//Hs.29202:R71586
R-PLACE1002768//ESTs//1.2e-70:359:95//Hs.132600:H12865
R-PLACE1002772//ESTs//8.1e-49:362:82//Hs.141254:AI334099
R-PLACE1002782//ESTs//2.4e-58:284:98//Hs.143545:AI149014
R-PLACE1002794//ESTs//5.4e-21:114:100//Hs.77365:W93593
R-PLACE1002811//ESTs//6.7e-68:329:98//Hs.78026:AA456955
R-PLACE1002815//ESTs//6.8e-103:537:93//Hs.5459:AI304392
R-PLACE1002816//ESTs//3.9e-05:118:68//Hs.98641:AA429916
R-PLACE1002834//ESTs, Highly similar to ZINC FINGER PROTEIN 91 [Homo sapiens]//2.1e-42:233:94//Hs.61518:AA167094
R-PLACE1002839//ESTs//1.7e-10:292:64//Hs.93012:R96142
R-PLACE1002851//ESTs//1.7e-73:381:95//Hs.135021:AI096756
R-PLACE1002853//ESTs//1.2e-89:453:96//Hs.23630:N57539
R-PLACE1002881//ESTs//1.1e-71:360:96//Hs.34392:AI066762
R-PLACE1002908//EST//2.7e-31:177:94//Hs.147925:AI249332
R-PLACE1002941//ESTs//4.0e-96:519:92//Hs.125139:AA523995
R-PLACE1002962
R-PLACE1002968//ESTs//4.7e-31:420:69//Hs.116518:AA653202
R-PLACE1002991//ESTs//9.0e-81:418:95//Hs.132717:AA171941
R-PLACE10029937/ESTs, Weakly similar to !!!! ALU SUBFAMILY SB WARNING ENTRY !!!! [H.sapiens]//1.3e-86:502:89//Hs.32232:AA604268
R-PLACE1002996//ESTs//1.9e-44:218:100//Hs.63657:AI144268
R-PLACE1003025//ESTs//8.4e-104:517:96//Hs.10711:AI151499
R-PLACE1003027//Human mRNA for KIAA0238 gene, partial cds//0.97:156r60//Hs.82042:D87075
R-PLACE1003044//Human onconeural ventral antigen-1 (Nova-1) mRNA, complete cds//1.0:200:63//Hs.214:U04840
R-PLACE1003092//ESTs//0.0046:267:60//Hs.133095:AA927777
R-PLACE1003100//ESTs, Highly similar to NODULATION PROTEIN G [Rhizobium meliloti]//9.5e-94:491:93//Hs.6318:AI131178
R-PLACE1003108//ESTs//0.00065:184:66//Hs.154366:AA527359
R-PLACE1003136//Signal recognition particle 54 kD protein//0.057:317:59//Hs.49346:U51920
R-PLACE1003145//ESTs//1.9e-98:534:92//Hs.61929:AA044757
R-PLACE1003153//ESTs//5.8e-76:367:98//Hs.105196:AA483467
R-PLACE1003174//ESTs//1.7e-44:226:98//Hs.59688:AA453924
R-PLACE1003176
R-PLACE1003190//ESTs//1.6e-74:356:99//Hs.121282:AI091453
R-PLACE1003200//ESTs//4.6e-93:461:96//Hs.24321:AA971017
R-PLACE1003205//ESTs//0.037:171:61//Hs.157077:H44802
R-PLACE100323 8//ESTs, Weakly similar to KIAA0001 [H.sapiens]//2.5e-82:436:94//Hs.58561:W79123
R-PLACE1003249//Human high-affinity copper uptake protein (hCTR1) mRNA, complete cds//7.9e-44:313:84//Hs.73614:U83460
R-PLACE1003256//EST//9.6e-46:284:88//Hs.162404:AA573131
R-PLACE1003258//ESTs, Weakly similar to !!!! ALU SUBFAMILY J WARNING ENTRY !!!! [H.sapiens]//8.3e-102:551:92//Hs.52431:AA625326
R-PLACE1003296//ESTs//1.9e-88:451:96//Hs.57749:W92986
R-PLACE1003302//ESTs, Highly similar to ZINC FINGER PROTEIN 43 [Homo sapiens]//8.2e-93:458:96//Hs.29147:AA883993
R-PLACE1003334//ESTs, Weakly similar to !!!! ALU CLASS B WARNING ENTRY !!!! [H.sapiens]//3.3e-94:463:97//Hs.155050:AA908765
R-PLACE1003342//ESTs//6.0e-88:447:96//Hs.107527:R66438
R-PLACE1003343//EST//0.0087:412:58/Hs.159963:AA977701
R-PLACE1003353//Homo sapiens breast cancer antiestrogen resistance 3 protein (BCAR3) mRNA, complete cds//1.1e-99:469:98//Hs.6564:U92715
R-PLACE1003361//ESTs//3.5e-64:332:95//Hs.163861:AI199636
R-PLACE1003366//ESTs//1.0e-87:492:92//Hs.72222:AA158234
R-PLACE1003369//ESTs, Weakly similar to ZK1058.4 [C.elegans]//3.5e-18:109:95//Hs.27670:AI051591
R-PLACE1003373//Homo sapiens mRNA for KIAA0472 protein, partial cds//2.6e-54:279:80//Hs.6874:AB007941
R-PLACE1003375//ESTs//1.7e-88:431:97//Hs.41327:AI039909
R-PLACE1003383//ESTs//0.00084:177:64//Hs.120695:AI377755
R-PLACE1003401//ESTs//1.1e-16:147:80//Hs.132187:AI039020
R-PLACE1003420//ESTs//1.4e-93:481:94//Hs.122565:AI126840
R-PLACE1003454//ESTs//4.0e-57:310:93//Hs.121688:AA743697
R-PLACE1003478//EST//1.0:162:63//Hs.147003:AI184671
R-PLACE1003493//ESTs//1.2e-73:383:95//Hs.28852:R64270
R-PLACE1003516//ESTs//3.2e-23:206:80//Hs.138632:H97952
R-PLACE1003519//H.sapiens hnRNP-E1 mRNA//1.7e-22:236:79//Hs.2853:Z29505
R-PLACE1003521//ESTs//5.8e-74:371:96//Hs.30818:AA194980
R-PLACE1003528//ESTs//1.1e-40:219:82//Hs.138856:H47461
R-PLACE1003537//ESTs, Weakly similar to multispanning membrane protein [H.sapiens]//7.4e-69:338:98//Hs.110439:N93209
R-PLACE1003553//ESTs//2.2e-87:438:97//Hs.132022:AI040321
R-PLACE1003566//ESTs//1.2e-62:298:92//Hs.30799:AI052591
R-PLACE1003575//Homo sapiens mRNA, chromosome 1 specific transcript KIAA0487//2.4e-22:145:80//Hs.92381:AB007956
R-PLACE1003583//ESTs, Weakly similar to hypothetical L1 protein [H.sapiens]//1.5e-14:264:65//Hs.158253:R86178
R-PLACE1003584
R-PLACE1003592//ESTs//1.3e-15:213:69//Hs.139507:T77542
R-PLACE1003593//ESTs, Highly similar to FRG1 gene product [H.sapiens]//5.8e-75:459:89//Hs.23884:AD77106
R-PLACE1003596//ESTs//0.011:273:61//Hs.71719:AA142875
R-PLACE1003602//Homo sapiens mRNA expressed in placenta//7.8e-97:576:88//Hs.56851:D83200
R-PLACE1003605//ESTs//3.7e-86:407:99//Hs.136057:AA988299
R-nnnnnnnnnnnn//ESTs//1.0:78:71//Hs.101248:T26446
R-PLACE1003618//ESTs//6.8e-30:281:79//Hs.114455:AA411943
R-PLACE1003625//ESTs//7.2e-78:377:98//Hs.102708:AA292285
R-PLACE1003638//ESTs//6.7e-38:274:82//Hs.138852:AA284247
R-PLACE1003669//ESTs//9.7e-83:418:95//Hs.4842:AI342607
R-PLACE1003704//ESTs//3.0e-13:99:89//Hs.81648:W26521
R-PLACE1003709//ESTs//0.019:178:60//Hs.32100:N59866
R-PLACE1003711//ESTs//0.99:126:63//Hs.47005:N98639
R-PLACE1003723//ESTS//1.7e-89:448:96//Hs.157222:AA766987
R-PLACE1003738//ESTs//2.5e-36:182:100//Hs.122162:AI057087
R-PLACE1003760//Human globin gene//L9e-98:538:91//Hs.100090:M69023
R-PLACE1003762//EST//2.9e-15:125:85//Hs.162083:AA487512
R-PLACE1003768//Human P042 gene, complete cds//3.1e-18:300:69//Hs.158302:U88965
R-PLACE1003771//ESTs//1.2e-09:64:100//Hs.23799:AI003798
R-PLACE1003783//ESTs, Weakly similar to D2085.5 [C.elegans]//3.8e-38:199:97//Hs.115197:AA215757
R-PLACE1003784//ESTs//3.7e-87:428:97//Hs.157985:AI366909
R-PLACE1003795//Homo sapiens mRNA for KIAA0575 protein, complete cds//3.2e-36:236:88//Hs.153468:AB011147
R-PLACE1003833//ESTs, Moderately similar to !!!! ALU SUBFAMILY SC WARNING ENTRY !!!! [H.sapiens]//8.5e-62:313:96//Hs.121020:AA526092
R-PLACE1003850//ESTs//4.0e-67:351:96//Hs.159303:T91059
R-PLACE1003858//ESTs//0.96:87:66//Hs.107112:AA679058
R-nnnnnnnnnnnn
R-PLACE1003870//EST//2.9e-34:281:79//Hs.160895:AI365871
R-nnnnnnnnnnnn
R-PLACE1003886//ESTs//6.7e-85:410:97//Hs.25129:W93595
R-PLACE1003888//ESTs//0.0085:165:64//Hs.96739:AA441915
R-PLACE1003900//EST//2.4e-05:129:69//Hs.127931:AA969259
R-PLACE1003903//ESTs, Highly similar to CTP SYNTHASE [Homo sapiens]//1.5e-54:282:96//Hs.58553:AA100804
R-PLACE1003915//EST//0.87:55:76//Hs.145930:AI275760
R-PLACE1003923//ESTs//1.7e-89:456:95//Hs.14125:AA156236
R-PLACE1003932//ESTs//3.0e-50:340:84//Hs.151208:AI126110
R-PLACE1003936//EST//1.8e-08:208:65//Hs.162656:AA603567
R-PLACE1003968//ESTs//7.4e-49:301:90//Hs.93850:AA115330
R-PLACE1004104//ESTs//1.9e-46:254:94//Hs.96802:AA443231
R-PLACE1004114//ESTs//1.2e-64:322:97//Hs.28928:AI052052
R-PLACE1004118//ESTs//1.0e-83:404:98//Hs.112764:AA609770
R-PLACE1004128//ESTs//5.3e-80:415:95//Hs.11835:AA040244
R-PLACE1004149//ESTs//7.2e-25:331:72//Hs.141084:H11714
R-PLACE1004156//Homo sapiens PYRIN (MEFV) mRNA, complete cds//2.0e-56:491:76//Hs.113283:AF018080
R-PLACE1004161//ESTs//2.0e-59:355:88//Hs.13830:AA918601
R-PLACE1004183//Homo sapiens cytochrome c oxidase assembly protein COX11(COX11) mRNA, complete cds//4.7e-78:434:91//Hs.153504:AF044321
R-PLACE1004197
R-PLACE1004203//Homo sapiens GPI-anchored membrane protein CDw108 precursor, mRNA, complete cds//1.5e-105:501:98//Hs.24640:AF069493
R-PLACE1004242//ESTs//1.0e-71:364:87//Hs.138632:H97952
R-PLACE1004256//EST//0.0011:347:61//Hs.131385:AI022630
R-PLACE1004257//EST//0.027:99:71//Hs.97587:AA398209
R-PLACE1004258//KERATIN. TYPE I CYTOSKELETAL 14//0.72:180:63//Hs.117729:100124
R-PLACE1004270//ESTS//0.011:264:59//Hs.110044:AA181800
R-PLACE1004274//Human retinoic acid receptor-beta associated open reading frame, complete sequence//0.28:121:66//Hs.1938:S82362
R-PLACE1004277//Homo sapiens two pore domain K⁺ channel (TASK-2) mRNA, complete cds//1.4e-107:581:91//Hs.127007:AF084830
R-PLACE1004284//ESTs//5.0e-22:187:82//Hs.23141:W92114
R-PLACE1004289//ESTs, Weakly similar to !!!! ALU SUBFAMILY J WARNING ENTRY !!!! [H.sapiens]//2.9e-28:279:77//Hs.38687:AA744496
R-PLACE1004302//ESTs, Weakly similar to SOF1 PROTEIN [Saccharomyces cerevisiae]//8.2e-61:313:95//Hs.71435:AI253099
R-PLACE1004316//H.sapiens mRNA for apoptosis specific protein//6.0e-115:590:94//Hs.11171:Y11588
R-PLACE1004336//Cytochrome P450, subfamily I (aromatic compound-inducible), polypeptide 2//6.7e-69:572:77//Hs.1361:M55053
R-PLACE1004358//Homo sapiens connector enhancer of KSR-like protein CNK1 mRNA, complete cds//7.7e-72:379:93//Hs.16232:AF100153
R-PLACE1004376//ESTs//0.49:362:59//Hs.138086:AI056309
R-PLACE1004384//EST//1.0:47:76//Hs.128546:AA905556
R-PLACE1004388//ESTs, Weakly similar to contains similarity to ATP/GTP-binding site motif [C.elegans]//1.3e-98:572:90//Hs.14202:N46000
R-PLACE1004405//ESTs//3.4e-99:507:95//Hs.28792:AI343467
R-PLACE1004425//ESTs//2.7e-85:442:95//Hs.12544:N53665
R-PLACE1004428//ESTs//1.0e-07:114:78//Hs.140225:AA704101
R-PLACE1004437//Human NAD⁺-specific isocitrate dehydrogenase beta subunit precursor, mRNA, nuclear gene encoding mitochondrial protein, complete cds//9.4e-90:516:88//Hs.155410:U49283
R-PLACE1004451
R-PLACE1004460//ESTs//5.4e-14:338:64//Hs.97464:AA662980
R-PLACE1004467//ESTs//3.3e-85:467:92//Hs.9527:W52721
R-PLACE1004471//ESTs//3.0e-73:389:94//Hs.23240:R46578
R-PLACE1004473//ESTs, Weakly similar to F20D1.2 [C.elegans]//3.8e-101:510:95//Hs.16986:W89194
R-PLACE1004491//Human mitochondrial 1,25-dihydroxyvitamin D3 24-hydroxylase mRNA, complete cds//0.23:278:61//Hs.89663:L13286
R-PLACE1004506//ESTs//2.5e-98:559:90//Hs.19447:AI057117
R-PLACE1004510//ESTs//1.5e-91:436:98//Hs.24846:AI420493
R-PLACE1004516//EST//1.7e-66:344:96//Hs.99303:AA453164
R-PLACE1004518//ESTs//5.2e-79:410:94//Hs.27091:AA436553
R-PLACE1004548//Homo sapiens mRNA for small GTP-binding protein, complete cds//1.8e-40:332:72//Hs.115325:084488
R-PLACE1004550
R-PLACE1004564//ESTs//5.5e-76:367:98//Hs.49683:AA564742
R-PLACE1004629//ESTs, Weakly similar to OS-9 precurosor [H.sapiens]//8.1e-40:272:87//Hs.7100:W07181
R-PLACE1004645//ESTs//6.3e-14:83:100//Hs.17270:AA701903
R-PLACE1004646//ESTs//3.7e-22:231:76//Hs.141250:N29734
R-PLACE1004658//ESTs//2.0e-12:109:84//Hs.23508:AA101113
R-nnnnnnnnnnnn//Homo sapiens mRNA for KIAA0714 protein, partial cds//7.8e-23:129:99//Hs.123129:AB018257
R-PLACE1004672//ESTs//2.0e-50:256:98//Hs.136367:AI144254
R-PLACE1004674//Homo sapiens calcium binding protein (ALG-2) mRNA, complete cds//L8e-90:510:91//Hs.80019:AF035606
R-PLACE1004681//EST//2.1e-08:283:62//Hs.99543:AA461482
R-PLACE1004686
R-PLACE1004691//EST//7.3e-42:305:82//Hs.141833:AA021552
R-PLACE1004693//ESTs//0.014:135:64//Hs.145333:AI251374
R-PLACE1004716//ESTs, Weakly similar to No definition line found [C.elegans]//3.4e-80:413:94//Hs.23528:AI279571
R-PLACE1004722//EST//0.14:165:63//Hs.18213:T97997
R-PLACE1004736//ESTs//1.0e-72:385:94//Hs.10657:N6391
R-PLACE1004740//ESTs//1.0:267:58//Hs.101661:AA416619
R-nnnnnnnnnnnn//EST//0.45:94:69//Hs.147174:AI192195
R-PLACE1004751//EST//9.8e-32:174:83//Hs.147901:AI223374
R-PLACE1004773//Homo sapiens inversin protein mRNA, complete cds//2.7e-89:437:96//Hs.104715:AF084367
R-PLACE1004777//ESTs//7.4e-68:351:94//Hs.23395:AA398548
R-PLACE1004793//ESTs//1.3e-53:290:78//Hs.142375:AA398619
R-nnnnnnnnnnnn//Homo sapiens mRNA for KIAA0606 protein, partial cds//1.9e-99:580:88//Hs.38176:AB011178
R-PLACE1004813//ESTs//7.6e-86:433:96//Hs.85640:AA535856
R-PLACE1004814//Homo sapiens okadaic acid-inducible phosphoprotein (OA48-18) mRNA, complete cds//1.1e-108:358:99//Hs.3688:AF069250
R-PLACE1004815//EST//4.7e-50:333:84//Hs.142196:AA258356
R-PLACE1004824//Protein kinase, interferon-inducible double stranded RNA dependent//4.8e-46:450:76//Hs.73821:M35663
R-PLACE1004827//ESTs//2.3e-48:250:96//Hs.138766:AA342185
R-PLACE1004836//ESTs//2.7e-39:222:94//Hs.78661:AA195299
R-PLACE1004838//EST//0.056:198:60//Hs.129589:AA995901
R-PLACE1004840//ESTs, Highly similar to TRANSCRIPTIONAL ACTIVATOR GCN5 [Saccharomyces cerevisiae]//6.5e-71:381:93//Hs.8383:AA013272
R-PLACE1004868//ESTs//4.9e-70:367:94//Hs.100895:AA479308
R-PLACE1004885//Homo sapiens protein phosphatase with EF-hands-2 long form (PPEF-2) mRNA, complete cds//1.8e-37:330:78//Hs.113259:AF023456
R-PLACE1004900//EST//1.2e-46:306:86//Hs.149580:AI211881
R-PLACE1004902//Sucrase-isomaltase//0.87:254:61//Hs.2996:X63597
R-nnnnnnnnnnnn//ESTs//4.5e-75:375:96//Hs.91115:AI221563
R-PLACE1004918//ESTs//2.6e-103:519:95//Hs.143607:AI424948
R-PLACE1004930//Homo sapiens TNF-induced protein GG2-1 mRNA, complete cds//6.6e-102:532:93//Hs.17839:AF099936
R-PLACE1004934//EST//0.035:156:67//Hs.162071:AA478980
R-PLACE1004937//ESTs, Weakly similar to F55B12.3 [C.elegans]//6.4e-80:409:95//Hs.31945:AA702l66
R-PLACE1004969//ESTs//9.8e-18:101:99//Hs.112837:N78013
R-PLACE1004972//ESTs//1.3e-65:337:95//Hs.75798:H29106
R-PLACE1004979//EST//1.2e-96:475:96//Hs.120158:AA708789
R-PLACE1004982//ESTs//1.0e-98:471:98//Hs.106496:AI291776
R-PLACE1004985//ESTs//2.1e-88:456:93//Hs.135050:AI420335
R-PLACE1005026
R-PLACE1005027//ESTs, Weakly similar to N-methyl-D-aspartate receptor glutamate-binding chain [R.norvegicus]//0.72:145:66//Hs.11215:N56719
R-PLACE1005046//Homo sapiens mRNA for KIAA0575 protein, complete cds//5.3e-66:297:88//Hs.153468:AB011147
R-PLACE1005052//ESTs, Weakly similar to weak similarity to rat cytosolic acyl coenzyme A thioester hydrolase [C.elegans]//1.2e-106:543:95//Hs.18625:AI074605
R-PLACE1005066//ESTs//3.9e-92:459:96//Hs.62684:AA806103
R-PLACE1005077//Human triadin mRNA, complete cds//1.8e-05:121:69//Hs.68731:U18985
R-PLACE1005085//Homo sapiens PYRIN (MEFV) mRNA, complete cds//6.6e-49:314:74//Hs.113283:AF018080
R-PLACE1005086//ESTs//1.2e-73:379:94//Hs.110128:AA584364
R-PLACE1005101//Homo sapiens (clone zap128) mRNA, 3' end ofcds//8.0e-99:531:92//Hs.75437:L40401
R-PLACE1005102//ESTs//7.2e-68:493:84//Hs.10593:AI201336
R-PLACE1005108//Human DNA fragmentation factor-45 mRNA, complete cds//9.2e-40:232:82//Hs.155344:U91985
R-PLACE1005111//EST//8.1e-10:189:68//Hs.136356:AA493225
R-PLACE1005128//ESTs//1.4e-78:501:87//Hs.15093:AA203423
R-PLACE1005146//ESTs//4.8e-93:460:97//Hs.37896:AA777349
R-PLACE1005162//ESTs//7.5e-51:277:95//Hs.28838:AI089013
R-nnnnnnnnnnnn//ESTs//5.4e-75:366:97//Hs.48119:AA454227
R-PLACE1005181//EST//0.012:172:66//Hs.147107:AI190589
R-PLACE1005187//ESTs//5.6e-72:363:95//Hs.16577:AI022830
R-PLACE1005206//ESTs//5.3e-48:203:88//Hs.31792:H45211
R-PLACE1005232//ESTs//5.1e-41:287:84//Hs.138552:R99532
R-PLACE1005243//ESTs//1.1e-48:348:83//Hs.113310:R16767
R-PLACE1005261//ESTs//0.19:175:62//Hs.124337:AA829524
R-PLACE1005266//ESTs//1.9e-22:388:66//Hs.124146:AA699633
R-PLACE1005277//ESTs//1.5e-29:314:72//Hs.163710:AA024516
R-PLACE1005287//ESTs//3.6e-95:456:98//Hs.49282:AA970322
R-PLACE1005305//ESTs//9.9e-71:428:88//Hs.144855:AI197937
R-PLACE1005308//ESTs//3.8e-32:173:96//Hs.58239:AA215797
R-PLACE1005313//ESTs//5.2e-74:409:93//Hs.33368:AA206614
R-PLACE1005327//Chromosome 1 specific transcript KIAA0491//1.7e-104:537:94//Hs.136309:AB007960
R-PLACE1005331//ESTs//2.1e-91:487:93//Hs.9291:AI189343
R-PLACE1005335//ESTs, Weakly similar to F23B2.4 [C.elegans]//3.8e-90:442:97//Hs.70202:AA732975
R-PLACE1005373//ESTs//8.0e-93:526:91//Hs.98541:N38901
R-PLACE1005374//Homo sapiens KIAA0395 mRNA, partial cds//3.3e-44:344:80//Hs.43681:AL022394
R-PLACE1005409//EST//0.43:174:59//Hs.162077:AA479978
R-PLACE1005453//EST//7.9e-57:330:90//Hs.162306:AA555304
R-PLACE1005467//ESTs//2.2e-42:294:84//Hs.142257:AA188423
R-PLACE1005471//Human Line-1 repeat mRNA with 2 open reading frames//2.3e-88:561:86//Hs.23094:M19503
R-PLACE1005477//Human methionine aminopeptidase mRNA, complete cds//6.9e-80:549:83//Hs.78935:U29607
R-PLACE1005480//EST//0.99:39:82//Hs.157275:AI364046
R-PLACE1005481//EST//1.5e-31:281:79//Hs.132635:AI032875
R-PLACE1005494//Homo sapiens mRNA for semaphorin E, complete cds//0.036:319:59//Hs.62705:AB000220
R-PLACE1005502//Homo sapiens formin binding protein 21 mRNA, complete cds//5.4e-57:277:98//Hs.28307:AF071185
R-PLACE1005526//ESTs//2.5e-30:233:83//Hs.119304:AA443325
R-PLACE1005528//Homo sapiens mRNA for cartilage-associated protein (CASP)//8.9e-20:321:69//Hs.155481:AJ006470
R-PLACE1005530//ESTs//3.7e-81:438:92//Hs.103380:AI291325
R-PLACE1005550//ESTs, Highly similar to HYPOTHETICAL 40.2 KD PROTEIN K12H4.3 IN CHROMOSOME III [Caenorhabditis elegans]//5.2e-95:458:98//Hs.38114:N62927
R-PLACE1005554//ESTs//8.8e-36:267:86//Hs.98288:AA203555
R-PLACE1005557//ESTs, Highly similar to MITOCHONDRIAL 60S RIBOSOMAL PROTEIN L2 PRECURSOR [Saccharomyces cerevisiae]//2.2e-64:345:94//Hs.7736:W81261
R-PLACE1005574//ESTs//2.3e-27:231:83//Hs.117771:R99835
R-PLACE1005584//ESTs//1.6e-36:188:98//Hs.152050:AA724612
R-PLACE1005595//ESTs//1.6e-91:453:96//Hs.85079:AI276023
R-PLACE1005603//ESTs//8.2e-99:533:93//Hs.96357:AI026927
R-PLACE1005611//ESTs//5.2e-28:183:89//Hs.24941:AA261857
R-PLACE1005623//ESTs//1.4e-102:505:96//Hs.58382:AA808964
R-PLACE1005630
R-PLACE1005639//ESTs//1.4e-51:256:98//Hs.1975:W72452
R-PLACE1005646//Homo sapiens RNA helicase-related protein mRNA, complete cds//1.0e-111:585:93//Hs.8765:AF083255
R-PLACE1005656//ESTs//2.7e-88:469:92//Hs.164054:AA528169
R-PLACE1005666//Homo sapiens X-ray repair cross-complementing protein 2 (XRCC2) mRNA, complete cds//3.3e-24:401:66//Hs.129727:AF035587
R-PLACE1005698//ESTs//0.00013:82:79//Hs.116331:AA629355
R-PLACE1005727//EST//0.15:206:63//Hs.105002:AA449332
R-PLACE1005730//EST//0.0014:129:70//Hs.127931:AA969259
R-PLACE1005739//ESTs, Moderately similar to unknown intracellular protein [M.musculus]//1.3e-42:236:94//Hs.23889:AI341137
R-PLACE1005755//ESTs//2.8e-32:308:80//Hs.159821:AA524070
R-PLACE1005763//Human mRNA for KIAA0118 gene, partial cds//3.3e-47:268:87//Hs.154326:D42087
R-PLACE1005799//ESTs, Highly similar to HYPOTHETICAL 68.7 KD PROTEIN ZK757.1 IN CHROMOSOME III [Caenorhabditis elegans]//7.7e-15:88:98//Hs.109857:AA088385
R-PLACE1005802//ESTs//2.8e-19:208:76//Hs.9271:W30941
R-PLACE1005803//ESTs//2.6e-75:417:92//Hs.71414:AA131327
R-PLACE1005804//EST//6.5e-20:182:70//Hs.149844:AI287693
R-PLACE1005828//ESTs//3.0e-15:194:77//Hs.106236:N50058
R-PLACE1005834//Retinoblastoma 1 (including osteosarcoma)//0.040:435:58//Hs.75770:L41870
R-PLACE1005845//EST//5.0e-61:294:99//Hs.133202:AI050965
R-PLACE1005850//ESTs//3.4e-82:425:96//Hs.7966:AI203471
R-PLACE1005851//ESTs//2.9e-21:165:84//Hs.23607:N98305
R-PLACE1005876//ESTs//0.48:296:57//Hs.39140:AI041842
R-PLACE1005884//ESTs//0.0027:177:66//Hs.150295:AA570558
R-PLACE1005898//ESTs//1.7e-98:467:98//Hs.159475:AI339981
R-PLACE1005921//ESTs//5.8e-96:480:95//Hs.30822:AA885501
R-PLACE1005923//ESTs//1.8e-66:333:96//Hs.150890:AI341793
R-PLACE1005925//Human Line-1 repeat mRNA with 2 open reading frames//2.8e-27:382:70//Hs.23094:M19503
R-PLACE1005932//ESTs, Moderately similar to MNK1 [H.sapiens]//1.1e-70:377:93//Hs.5662:AA868361
R-PLACE1005934//ESTs//1.0e-42:251:91//Hs.25092:AA922142
R-PLACE1005936//ESTs//1.2e-88:461:94//Hs.94125:N62913
R-PLACE1005951//ESTs//1.4e-83:533:86//Hs.21148:AI183729
R-PLACE1005953
R-PLACE1005955//ESTs, Highly similar to HYPOTHETICAL 54.2 KD PROTEIN-IN CDC12-ORC6 INTERGENIC REGION [Saccharomyces cerevisiae]//2.2e-83:494:88//Hs.108117:AI097079
R-PLACE1005966//ESTs//1.1e-95:465:97//Hs.98510:AI016239
R-PLACE1005968//EST//0.26:103:66//Hs.161300:AI420897
R-PLACE1005990
R-PLACE1006002//Human mRNA for KIAA0355 gene, complete cds//2.0e-45:481:74//Hs.153014:AB002353
R-PLACE1006003//ESTs, Highly similar to HYPOTHETICAL 30.3 KD PROTEIN IN APE1/LAP4-CWP1 INTERGENIC REGION [Saccharomyces cerevisiae]//3.1e-112:593:93//Hs.111449:AI192946
R-PLACE1006011//ESTs, Moderately similar to NAD(⁺) ADP-RIBOSYLTRANSFERASE [D.melanogaster]//5.7e-100:596:88//Hs.24284:AA595596
R-PLACE1006017//ESTs//4.2e-18:296:68//Hs.133350:AI056276
R-PLACE1006037//ESTs, Weakly similar to T23D8.3 [C.elegans]//4.1e-102:491:98//Hs.61164:AI096332
R-PLACE1006040//ESTs//1.2e-92:443:98//Hs.111680:N93765
R-PLACE1006076//ESTs, Moderately similar to !!!! ALU SUBFAMILY SC WARNING ENTRY !!!! [H.sapiens]//2.0e-26:213:77//Hs.139007:H74314
R-PLACE1006119//ESTs//0.14:257:61//Hs.113149:AA908904
R-PLACE1006129//ESTs//3.8e-54:285:97//Hs.18827:W68002
R-PLACE1006139//ESTs, Highly similar to HYPOTHETICAL 52.9 KD PROTEIN IN SAP155-YMR31 INTERGENIC REGION [Saccharomyces cerevisiae]//2.6e-99:560:91//Hs.5249:U55977
R-PLACE1006143//Amylo-1,6-glucosidase, 4-alpha-glucanotransferase (glycogen debranching enzyme, glycogen storage disease type III)//0.038:463:59//Hs.904:U84010
R-PLACE1006157//ESTs//0.014:341:58//Hs.121773:AI357886
R-PLACE1006159//EST//0.00036:247:61//Hs.140054:AA668925
R-PLACE1006164//ESTs//2.6e-31:362:73//Hs.141024:H07128
R-PLACE1006167//Homo sapiens chromosome 19, cosmid F23149//5.8e-54:286:94//Hs.152894:AC005239
R-nnnnnnnnnnnn//ESTs, Highly similar to ALPHA-ADAPTIN [Rattus norvegicus]//2.7e-79:393:96//Hs.19121:AI125280
R-PLACE1006187//Homo sapiens cyclin E2 mRNA, complete cds//5.1e-118:597:95//Hs.30464:AF091433
R-PLACE1006195//ESTS, Weakly similar to !!!! ALU SUBFAMILY J WARNING ENTRY !!!! [H.sapiens]//6.8e-94:532:91//Hs.105216:AI361807
R-PLACE1006196//ESTs//3.2e-66:382:90//Hs.18665:T99507
R-PLACE1005205//EST//1.7e-89:448:96//Hs.116665:AA669114
R-PLACE1006223//Human RNaseP protein p38 (RPP38) mRNA, complete cds//0.90:304:58//Hs.94986:U77664
R-PLACE1006225//ESTs//7.2e-96:474:97//Hs.91165:AI079555
R-PLACE1006236//ESTs//8.8e-105:535:95//Hs.7919:AI341472
R-nnnnnnnnnnnn//Homo sapiens BAC clone RG118D07 from 7q31//3.2e-99:497:95//Hs.3781:AC004142
R-PLACE1006246//ESTs, Weakly similar to CMP-sialic acid transporter [M.musculus]//1.3e-104:532:95//Hs.41151:AI301961
R-PLACE1006248//Homo sapiens mRNA for KIAA0648 protein, partial cds//3.0e-97:499:95//Hs.31921:AB014548
R-PLACE1006262//ESTs, Moderately similar to !!!! ALU SUBFAMILY SC WARNING ENTRY !!!! [H.sapiens]//1.6e-07:321:62//Hs.53057:W67839
R-PLACE1006288//Voltage-dependent anion channel 1//3.8e-100:605:88//Hs.2060:L06132
R-PLACE1006318//ESTs//2.4e-102:536:94//Hs.8109:AA005265
R-PLACE1006325//ESTs//5.2e-105:518:96//Hs.102319:AI246503
R-PLACE1006335//ESTs//5.1e-45:254:93//Hs.153585:R70900
R-PLACE1006357//EST//6.5e-09:309:62//Hs.132493:AA923168
R-PLACE1006360//Human mRNA for KIAA0090 gene, partial cds//0.0097:381:58//Hs.154797:D42044
R-PLACE1006368//ESTs//7.9e-85:412:97//Hs.150587:AI079284
R-PLACE1006371//ESTs//7.7e-74:442:88//Hs.143671:W61053
R-PLACE1006382
R-PLACE1006385//ESTs//5.3e-06:346:61//Hs.163706:AA515748
R-PLACE1006412//EST//7.7e-46:306:86//Hs.149580:AI281881
R-PLACE1006414//Homo sapiens UM protein mRNA, complete cds//4.1e-43:551:69//Hs.154103:AF061258
R-PLACE1006438//ESTs//1.1e-77:284:86//Hs.24545:AI278629
R-PLACE1006445//ESTs//4.4e-53:259:99//Hs.24481:AA573139
R-PLACE1006469//ESTs//9.4e-102:482:98//Hs.7218:AA936961
R-PLACE1006470//ESTs//1.0:271:57//Hs.144517:AA938297
R-PLACE1006482//ESTs//4.0e-61:354:92//Hs.51305:T47418
R-PLACE1006492//EST//1.8e-09:48:91//Hs.144451:AA827722
R-PLACE1006506//ESTs//0.012:161:61//Hs.145333:AI251374
R-PLACE1006521//Human mRNA for KIAA0013 gene, complete cds//2.1e-15:415:63//Hs.48824:D87717
R-PLACE1006531//ESTs//5.6e-31:213:87//Hs.125153:AA453723
R-PLACE1006534//ESTs//6.5e-101:512:95//Hs.27763:W46368
R-PLACE1006540//ESTs//7.3e-40:320:79//Hs.121659:H02532
R-PLACE1006552//EST//0.38:418:56//Hs.140470:AA765214
R-PLACE1006598//ESTs//4.0e-80:409:95//Hs.142868:AI128443
R-PLACE1006615//Homo sapiens eukaryotic translation initiation factor eIF3, p35 subunit mRNA, complete cds//9.3e-118:590:95//Hs.155377:U97670
R-PLACE1006617//ESTs//8.1e-31:246:83//Hs.139128:AA205322
R-PLACE1006626//ESTs//0.90:98:68//Hs.96322:AA541615
R-PLACE1006629//Human mRNA for KIAA0386 gene, complete cds//5.3e-33:315:78//Hs.101359:AB002384
R-PLACE1006640//ESTs//3.7e-26:137:100//Hs.32672:W16522
R-PLACE1006673//Interleukin 10//8.4e-47:330:83//Hs.2180:M57627
R-PLACE1006678//ESTs//1.1e-13:87:98//Hs.34035:D87736
R-PLACE1006704//ESTs//2.6e-65:394:89//Hs.30582:D12214
R-PLACE1006731//Homo sapiens clone 23923 mRNA sequence/1.9e-102:486:98//Hs.12472:AF038172
R-PLACE1006754//EST//1.0e-61:381:89//Hs.14727:T83861
R-PLACE1006760//Homo sapiens clone 24800 mRNA sequence//3.8e-73:394:93//Hs.7252:AF070622
R-PLACE1006779//ESTs//1.4e-69:405:90//Hs.136235:AA262658
R-PLACE1006782//EST//1.8e-25:197:86//Hs.137257:N33234
R-PLACE1006792//ESTs//1.8e-43:317:84//Hs.139190:N55515
R-PLACE1006795//ESTs//6.4e-68:350:95//Hs.11092:AA916335
R-PLACE1006800//ESTs//1.9e-55:268:100//Hs.126695:AA917989
R-PLACE1006805//ESTs//6.6e-91:484:93//Hs.94262:AA768847
R-PLACE1006815//ESTs//2.1e-49:364:83//Hs.142031:AA809159
R-PLACE1006819//ESTs, Highly similar to LINE-1 REVERSE TRANSCRIPTASE HOMOLOG [Homo sapiens]//1.0e-87:481:92//Hs.141263:H64113
R-PLACE1006829//ESTs//5.7e-43:332:83//Hs.19906:AA456933
R-PLACE1006860//ESTs//0.96:138:63//Hs.136649:AA-828359
R-PLACE1006867//ESTs//1.4e-98:478:97//Hs.10299:N35008
R-PLACE1006878//EST//8.4e-48:243:97//Hs.54970:N93536
R-PLACE1006883//EST//3.1e-46:300:88//Hs.162404:AA573131
R-nnnnnnnnnnnn//ESTs//3.0e-95:496:94//Hs.47546:AA181348
R-PLACE1006904//ESTs//5.8e-18:304:68//Hs.125816:AA806089
R-PLACE1006917//Endothelin receptor type B//0.00012:451:60//Hs.82002:D13168
R-PLACE1006932//ESTs//4.6e-56:285:96//Hs.114727:AI379514
R-PLACE1006935//ESTs//3.6e-12:157:73//Hs.161714:AA229078
R-nnnnnnnnnnnn//Human mRNA for KIAA0201 gene, complete cds//3.2e-25:494:63//Hs.36927:D86956
R-PLACE1006961//Tyrosine aminotransferase//2.5e-46:471:74//Hs.2999:X52520
R-PLACE1006962//ESTs, Moderately similar to plakophilin 2b [H.sapiens]//9.0e-29:324:68//Hs.154257:AI275982
R-PLACE1006966//ESTs//4.5e-99:470:99//Hs.46913:AI017636
R-PLACE1006989//ESTs//2.2e-68:353:97//Hs.14394:R61257
R-PLACE1007014//ESTs//3.4e-86:457:94//Hs.129819:AA838366
R-PLACE1007021//ESTs//1.6e-93:539:90//Hs.7111:U55971
R-PLACE1007045//Human Line-1 repeat mRNA with 2 open reading frames//6.6e-83:584:82//Hs.23094:M19503
R-PLACE1007053//ESTs//4.2e-85:550:88//Hs.7984:AI202575
R-PLACE1007097//ESTs//6.4e-78:493:86//Hs.56406:N91027
R-PLACE1007105//ESTs//5.3e-70:381:91//Hs.22605:N74202
R-PLACE1007111//ESTs//8.6e-75:358:99//Hs.145629:AA398646
R-PLACE1007112//ESTs//6.9e-69:371:94//Hs.71922:AA148417
R-PLACE1007132//ESTs//1.2e-36:373:69//Hs.10762:W28948
R-PLACE1007140//ESTs//1.7e-70:360:96//Hs.56179:W56794
R-PLACE1007178//EST//0.68:85:65//Hs.147010:AI184765
R-PLACE1007226//ESTs//3.1e-78:452:90//Hs.8033:N94998
R-PLACE1007238//ESTs//5.2e-70:362:95//Hs.85636:AA740619
R-PLACE1007239//Human mRNA for transcription elongation factor S-II, hS-II-T1, complete cds//6.3e-93:534:89//Hs.80598:D50495
R-PLACE1007242//ESTS//1.2e-80:390:98//Hs.117325:AA699450
R-PLACE1007243//ESTs, Weakly similar to transporter protein [H. sapiens]//3.7e-73:357:98//Hs.18272:N78499
R-PLACE1007257//Homo sapiens mRNA for dia-156 protein//4.3e-85:487:91//Hs.121556:Y15909
R-PLACE1007274//ESTs//4.3e-79:430:93//Hs.146023 AI275071
R-PLACE1007276//ESTs//1.5e-33:338:74//Hs.142850:R38419
R-PLACE1007282//ESTs//4.8e-98:532:93//Hs.10071:AA100812
R-PLACE1007286//Human mRNA for KIAA0118 gene, partial cds//2.9e-50:518:74//Hs.154326:D42087
R-PLACE1007301
R-PLACE1007317
R-PLACE1007342
R-PLACE1007346//Homo sapiens estrogen-responsive B box protein (EBBP) mRNA, complete cds//1.2e-66:367:91//Hs.76596:AF096870
R-PLACE1007367//ESTs, Weakly similar to !!!! ALU SUBFAMILY J WARNING ENTRY !!!! [H.sapiens]//2.2e-98:488:96//Hs.24359:AA699594
R-PLACE1007375//ESTs//2.3e-67:375:92//Hs.33368:AA206614
R-PLACE1007386//ESTs//0.020:242:62//Hs.42768:AI129945
R-PLACE1007402//ESTs//1.6e-91:441:97//Hs.26243:AA455877
R-PLACE1007409//Homo sapiens mitoxantrone resistance protein 1 mRNA, partial sequence//2.4e-113:590:94//Hs.14387:AF093771
R-PLACE1007416//ESTs, Weakly similar to DIPEPTIDYL PEPTIDASE IV [H.sapiens]//3.8e-115:579:95//Hs.72165:AI243857
R-PLACE1007450//Human macrophage-derived chemokine precursor (MDC) mRNA, complete cds//2.7e-38:311:80//Hs.97203:U83171
R-PLACE1007452//EST//2.5e-42:386:77//Hs.140562:AA826514
R-PLACE1007460//ESTs//4.9e-87:434:95//Hs.28472:AI028230
R-PLACE1007478
R-PLACE1007484//ESTs//6.8e-08:64:92//Hs.100251:AA535975
R-PLACE1007488//Dystrophin (muscular dystrophy, Duchenne and Becker types), includes DXS142, DXS164, DXS206, DXS230, DXS239, DXS268, DXS269, DXS270, DXS272//0.26:411:60//Hs.79012:M18533
R-PLACE1007507//ESTs//2.2e-11:136:76//Hs.128815:AA678072
R-PLACE1007511//ESTs, Highly similar to KERATIN, TYPE I CYTOSKELETAL 14 [Homo sapiens]//1.5e-41:261:89//Hs.9029:W57657
R-PLACE1007524//ESTs//5.8e-45:297:87//Hs.154923:AA491377
R-PLACE1007525//Human mRNA for KIAA0118 gene, partial cds//1.9e-44:422:75//Hs.154326:D42087
R-PLACE1007544//ESTs//8.4e-59:327:93//Hs.27410:N25612
R-PLACE1007547//EST//0.00010:107:71//Hs.146867:AI161404
R-PLACE1007557//ESTs//1.6e-43:356:79//Hs.44702:AI148840
R-PLACE1007583//ESTs//1.7e-41:214:97//Hs.155071:AA584257
R-PLACE1007598//Homo sapiens clone 23939 mRNA sequence//4.8e-104:554:93//Hs.21838:AF038179
R-PLACE1007618//Lymphocyte cytosolic protein 1 (L-plastin)//0.54:l61:65//Hs.76506:J02923
R-PLACE1007621//Homo sapiens clone 23859 mRNA sequence//4.8e-105:537:94//Hs.151046:AF038176
R-PLACE1007632
R-PLACE1007645//ESTs//0.99:187:62//Hs.163453:AI344106
R-PLACE1007649//ESTs//2.2e-108:561:94//Hs.24398:AI262946
R-PLACE1007677//ESTs, Moderately similar to !!!! ALU SUBFAMILY SB2 WARNING ENTRY !!!! [H.sapiens]//9.0e-37:190:97//Hs.23437:AA707331
R-PLACE1007688//ESTs//7.5e-79:409:95//Hs.6166:AI376944
R-PLACE1007690//ESTs, Weakly similar to NADH-UBIQUINONE OXIDOREDUCTASE CHAIN 5 [Ascaris suum]//3.4e-61:384;89//Hs.92918:AA133274
R-PLACE1007697//ESTs, Highly similar to GCN20 PROTEIN [Saccharomyces cerevisiae]//1.8e-84:501:88//Hs.91251:U66685
R-PLACE1007705//Human mRNA for apolipoprotein E receptor 2, complete cds//0.43:307:59//Hs.54481:D86407
R-PLACE1007706//Homo sapiens metalloprotease 1 (MP1) mRNA, complete cds//5.7e-75:374:96//Hs.4812:AF061243
R-PLACE1007725//ESTs, Weakly similar to No definition line found [C.elegans]//3.1e-39:253:88//Hs.108797:AA476815
R-PLACE1007729//ESTs//2.7e-44:392:79//Hs.142375:AA398619
R-PLACE1007730//Homo sapiens mRNA for KIAA0685 protein, complete cds//6.7e-94:556:89//Hs.153121:AB014585
R-PLACE1007737//ESTs//1.1e-41:345:80//Hs.114671:N39322
R-PLACE1007743//ESTs//2.8e-17:98:100//Hs.124258:AA976778
R-PLACE1007746//ESTs//5.3e-69:413:90//Hs.5297:AA156903
R-PLACE1007791//ESTS, Weakly similar to TEICHOIC ACID BIOSYNTHESIS PROTEIN A [Bacillus subtilis]//8.6e-27:143:98//Hs.144194:AA706337
R-PLACE1007807//Human Line-1 repeat mRNA with 2 open reading frames//9.9e-45:428:76//Hs.23094:M 9503
R-PLACE1007810//ESTs//5.9e-15:143:82//Hs.126257:AI279044
R-PLACE1007829//ESTs//2.2e-22:190:84//Hs.142707:W24050
R-PLACE1007843//ESTs//5.3e-110:556:95//Hs.107287:AI308839
R-PLACE1007846//Human Line-1 repeat mRNA with 2 open reading frames//1.7e-95:525:91//Hs.23094:M19503
R-PLACE1007852//ESTs//4.5e-14:174:75//Hs.153419:N52017
R-PLACE1007858//Homo sapiens mRNA for KIAA0766 protein, complete cds//2.1e-111:574:94//Hs.28020:AB018309
R-PLACE1007866//EST//1.8e-48:262:96//Hs.141009:H01178
R-PLACE1007877//ESTs//1.2e-94:478:96//Hs.5999:AI207832
R-PLACE1007897//ESTs//2.3e-92:437:99//Hs.122843:AI189060
R-PLACE1007908//Homo sapiens mRNA, chromosome 1 specific transcript KIAA0487//2.8e-89:460:95//Hs.92381:AB007956
R-PLACE1007946//ESTs//2.8e-28:172:78//Hs.126784:AA521510
R-PLACE1007954//ESTs//6.1e-72:366:95//Hs.27842:AI217966
R-PLACE1007955//Homo sapiens cyclin-D binding Myb-like protein mRNA, complete cds//3.9e-103:509:96//Hs.5671:AF084530
R-PLACE1007958//Homo sapiens cAMP-specific phosphodiesterase 8B (PDE8B) mRNA, partial cds//7.2e-89:465:93//Hs.78106:AF079529
R-PLACE1007969//ESTs, Weakly similar to F35C12.2 [C.elegans]//1.4e-113:534:99//Hs.44268:AA455900
R-PLACE1007990//ESTs, Highly similar to DOSAGE COMPENSATION REGULATOR [Drosophila melanogaster)//3.8e-97:493:95//Hs.6141:U69564
R-PLACE1008000//ESTs//0.00013:241:65//Hs.44369:AI206835
R-PLACE1008002//ESTs//2.2e-83:397:98//Hs.28780:AI263612
R-PLACE1008044//ESTs, Moderately similar to NUCLEAR PORE COMPLEX PROTEIN NUP107 [R.norvegicus]//2.0e-115:575:95//Hs.92395:AA779854
R-PLACE1008045//EST//2.6e-89:465:94//Hs.47374:N51935
R-PLACE1008080//EST//0.27:118:65//Hs.144110:AI054269
R-PLACE1008095//ESTs//5.5e-23:268:73//Hs.152525:AA516469
R-PLACE1008111//ESTs, Weakly similar to oxidoreductase [H.sapiens]//4.4e-108:537:96//Hs.28877:AI309334
R-PLACE1008122//ESTs//6.5e-103:531:94//Hs.34737:AI028617
R-PLACE1008129//ESTs//0.76:96:66//Hs.65373:AA883511
R-PLACE1008132//ESTs//5.9e-05:113:72//Hs.13014:W26381
R-PLACE1008177//ESTs//7.2e-107:557:93//Hs.132851:AI028266
R-PLACE1008181//ESTs//5.3e-97:473:97//Hs.57483:AA776267
R-PLACE1008198//ESTs//3.9e-16:120:85//Hs.9142:AA662107
R-nnnnnnnnnnnn//Homo sapiens mRNA for KIAA0530 protein, partial cds//1.6e-104:551:93//Hs.10801:AB011102
R-PLACE1008209//ESTs//L2e-72:366:96//Hs.92308:AI052701
R-PLACE1008231//ESTs//1.2e-70:363:94//Hs.25094:R80871
R-PLACE1008244//ESTs//1.3e-98:543:92//Hs.25130:AA218990
R-PLACE1008273//ESTs//6.1e-16:153:79//Hs.115987:AA483808
R-nnnnnnnnnnnn
R-PLACE1008280//ESTs//1.3e-66:353:94//Hs.156376:AI338705
R-PLACE1008309//ESTs//2.8e-100:511:95//Hs.45080:N49852
R-PLACE1008329//V-myc avian myelocytomatosis viral oncogene homolog//0.53:206:62//Hs.79070:K02276
R-PLACE1008330//ESTs, Weakly similar to EOSINOPHIL LYSOPHOSPHOLIPASE [H.sapiens]//8.6e-79:297:91//Hs.146477:AI128445
R-PLACE1008331//ESTs//0.98:156:62//Hs.108548:AA081656
R-PLACE1008356//Homo sapiens mRNA for KIAA0679 protein, partial cds//2.1e-99:556:90//Hs.5734:AB014579
R-PLACE1008368//EST//0.0027:198:63//Hs.160868:AI359052
R-PLACE1008369//ESTs//5.4e-28:167:92//Hs.19530:AA480009
R-PLACE1008392//ESTs, Moderately similar to !!!! ALU SUBFAMILY SC WARNING ENTRY !!!! [H.sapiens]//2.0e-41:448:72//Hs.139007:H74314
R-PLACE1008398//ESTs, Highly similar to Mig-6//1.4e-103:529:94//Hs.11169:AA156242
R-PLACE1008401//ESTs. Weakly similar to !!!! ALU SUBFAMILY J WARNING ENTRY !!!! [H.sapiens]//1.2e-81:536:87//Hs.7570:W31010
R-nnnnnnnnnnnnn//Homo sapiens mRNA for p115, complete cds//5.1e-103:521:95//Hs.7763:D86326
R-PLACE1008405//ESTs//1.2e-89:485:92//Hs.138241:AA767440
R-PLACE1008424//ESTs//6.7e-97:508:93//Hs.6709:AI379778
R-PLACE1008426//ESTs//5.5e-30:174:92//Hs.7946:AA651757
R-PLACE1008429//ESTs//2.1e-12:188:71//Hs.140769:AA931562
R-PLACE1008437//ESTs//7.1e-54:266:98//Hs.13068:AA001928
R-PLACE1008455//ESTs//4.7e-69:471:85//Hs.28337:AA210761
R-PLACE1008457//EST//8.6e-14:202:71//Hs.149887:AI289387
R-PLACE1008465//ESTs//3.8e-80:426:93//Hs.153146:AI299636
R-PLACE1008488//ESTs//7.9e-73:388:94//Hs.97268:AA292180
R-PLACE1008524//ESTs//7.4e-107:545:95//Hs.10441:N62816
R-PLACE1008531//ESTs//3.8e-68:427:87//Hs.56607:H23560
R-PLACE1008532
R-PLACE1008533//ESTs//2.5e-52:318:88//Hs.7274:AA476850
R-PLACE1008568//ESTs//3.2e-99:486:97//Hs.84414:AI423223
R-PLACE1008584//EST//2.2e-18:154:68//Hs.141498:N50064
R-PLACE1008621//ESTS, Weakly similar to line-1 protein ORF1 [H.sapiens]//8.6e-67:483:82//Hs.140416:AA778649
R-nnnnnnnnnnnn
R-PLACE1008626//ESTs//4.7e-73:372:95//Hs.23491:AA642454
R-PLACE1008627//ESTS//1.6e-90:475:93//Hs.102401:AI004972
R-PLACE1008629//ESTs//8.0e-93:492:93//Hs.20843:AA699512
R-PLACE1008630//ESTs//1.0e-94:453:98//Hs.34840:AI279612
R-PLACE1008643//Human mRNA for KIAA0355 gene, complete cds//2.8e-49:422:79//Hs.153014:AB002353
R-PLACE10086507/Homo sapiens pleiotropic regulator 1 (PLRG1) mRNA, complete cds//7.9e-90:434:97//Hs.147967:AF044333
R-PLACE1008693//ISLET AMYLOID POLYPEPTIDE PRECURSOR//1.8e-41:505:71//Hs.51048:X68830
R-PLACE1008696//Cytochrome P450, subfamily I (aromatic compound-inducible), polypeptide 2//1.7e-51:316:76//Hs.1361:M55053
R-PLACE1008715//EST//0.63:114:64//Hs.121353:AA758600
R-PLACE1008748//ESTs, Weakly similar to !!!! ALU CLASS B WARNING ENTRY !!!! [H.sapiens]//2.3e-40:281:83//Hs.142209:AA873303
R-PLACE1008757//ESTs//1.4e-45:226:99//Hs.22822:H06408
R-PLACE1008790//ESTs//0.035:67:76//Hs.153554:AI286313
R-PLACE1008798//ESTs//4.9e-59:285:99//Hs.49018:N79930
R-PLACE1008807//ESTs//1.7e-82:413:96//Hs.130745:AA573217
R-PLACE1008808//Homo sapiens putative checkpoint control protein HRAD1 mRNA, complete cds//1.1e-98:499:95//Hs.7179:AF011905
R-PLACE1008813//ESTs, Weakly similar to coded for by C. elegans cDNA cm10e3 [C.elegans]//4.2e-92:490:93//Hs.110454:H11810
R-PLACE1008851//ESTs//2.4e-84:421:95//Hs.158893:AI378428
R-nnnnnnnnnnnn
R-PLACE1008867//ESTs//1.1e-77:400:95//Hs.44198:AI093502
R-PLACE1008887//0xytocin receptor//1.1e-43:601:67//Hs.2820:X64878
R-PLACE1008902//ESTs//0.023:208:61//Hs.154164:AI246893
R-PLACE1008920//Homo sapiens mRNA for KIAA0765 protein, partial cds//2.6e-56:344:89//Hs.62318:AB018308
R-PLACE1008925//ESTs//0.17:294:57//Hs.105113:AA457018
R-PLACE1008934//ESTs//2.0e-61:339:92//Hs.100448:AA622653
R-PLACE1008941//ESTs, Moderately similar to ATP-BINDING CASSETTE TRANSPORTER 2 [Mus musculus]//1.3e-19:488:63//Hs.15780:U66680
R-PLACE1008947//ESTs//1.3e-81:385:99//Hs.71574:AI376573
R-PLACE1009020//ESTs//2.9e-79:419:94//Hs.121816:AA775419
R-PLACE1009027//Homo sapiens mRNA for doublecortin//3.1e-82:434:94//Hs.34780:AJ003112
R-PLACE1009039//ESTs//2.8e-83:448:92//Hs.129179:AA988520
R-PLACE1009045//ESTs//1.6e-64:318:97//Hs.103423:AA814195
R-PLACE1009048//ESTs//2.7e-17:403:63//Hs.149343:AI249139
R-PLACE1009050//ESTs//2.0e-88:475:92//Hs.122925:AA909008
R-PLACE1009060//ESTs, Highly similar to HYPOTHETICAL 98.3 KD PROTEIN R10E12.1 IN CHROMOSOME III [Caenorhabditis elegans]//1.2e-112:555:96//Hs.9663:AA527142
R-PLACE1009090//ESTs//5.0e-13:175:75//Hs.140608:N53448
R-PLACE1009094//Human splicing factor SRp30c mRNA, complete cds//0.98:161:63//Hs.77608:AL021546
R-PLACE1009099//ESTs, Highly similar to MKR2 PROTEIN [Mus musculus]//0.037:63:84//Hs.39943:AA203136
R-PLACE1009110//EST//5.8e-17:307:65//Hs.117264:AA682549
R-PLACE1009111//ESTs//1.9e-57:349:90//Hs.11260:N98983
R-PLACE1009130//ESTs, Weakly similar to hypothetical protein 2 [H.sapiens]//6.5e-97:501:94//Hs.11123:AA703945
R-PLACE1009150//LAMIN B1//0.064:393:60//Hs.89497:L37747
R-PLACE1009155/TESTs, Moderately similar to ovarian-specific protein [R.norvegicus]//2.5e-36:163:82//Hs.93332:AA811920
R-PLACE1009158//ESTs//0.30:149:65//Hs.155796:R80005
R-PLACE1009166//ESTs//3.3e-34:292:77//Hs.140255:AA708322
R-PLACE1009172//EST//8.9e-21:364:67//Hs.142557:AA464948
R-PLACE1009174//ESTs//2.9e-18:274:70//Hs.139241:AA283707
R-PLACE1009183//ESTs//2.3e-44:297:87//Hs.136839:H93717
R-PLACE10091867/ESTs, Weakly similar to No definition line found [C.elegans]//1.5e-109:572:94//Hs.54943:Z78396
R-PLACE1009190//ESTs//2.6e-53:318:90//Hs.25245:AA176701
R-PLACE1009200//H.sapiens mRNA for sortilin//3.2e-33:195:92//Hs.104247:X98248
R-PLACE1009230//ESTs//3.0e-31:153:92//Hs.124116:AA772680
R-PLACE1009246//ESTs//2.7e-90:488:92//Hs.10706:AA909018
R-PLACE1009308//ESTs//0.022:46:97//Hs.36545:AA075423
R-PLACE1009319//ESTs//7.7e-99:533:92//Hs.109654:N91279
R-PLACE1009328//Human Line-1 repeat mRNA with 2 open reading frames//7.3e-82:578:82//Hs.23094:M19503
R-PLACE1009335//EST//1.3e-64:311:99//Hs.130558:AI004397
R-PLACE1009338//ESTs//6.0e-70:386:93//Hs.3542:AI015782
R-PLACE1009368//ESTs//1.4e-18:107:98//Hs.133303:W04760
R-PLACE1009375//ESTs//8.9e-36:313:76//Hs.24608:AA161260
R-PLACE1009388//EST//4.4e-11:101:83//Hs.147074:AI188883
R-PLACE1009398//ESTs//5.7e-63:335:93//Hs.149003:AI243186
R-nnnnnnnnnnnnn//ESTs//3.6e-94:452:98//Hs.103177:W72798
R-PLACE1009410//ESTs//2.2e-112:553:96//Hs.61779:AA195255
R-PLACE1009434//EST//3.4e-15:109:74//Hs.103742:U48632
R-PLACE1009443//EST//7.5e-61:302:98//Hs.157787:AI361269
R-PLACE1099444//PHOSPHATIDYLINOSITOL 4-KINASE ALPHA//6.6e-85:479:90//Hs.76987:AF012872
R-PLACE1009459//ESTs//9.3e-86:437:95//Hs.104871:AI161427
R-PLACE1009476//Homo sapiens Chromosome 16 BAC clone CIT987SK-A-67A1//1.3e-42:266:89//Hs.155049:AC004531
R-PLACE1009477//ESTs//2.0e-50:367:82//Hs.152788:AA630925
R-PLACE1009493//ESTs//4.5e-14:150:78//Hs.143918:AA699596
R-PLACE1009524//ESTs//2.9e-97:454:99//Hs.7189:AA767698
R-PLACE1009539//ESTs//9.1e-94:454:97//Hs.154706:AI262131
R-PLACE1009542//Homo sapiens apoptotic protease activating factor 1 (Apaf-1) mRNA, complete cds//1.4e-10:289:63//Hs.77579:AF013263
R-PLACE1009571//ESTs//2.1e-23:125:100//Hs.41767:AA732326
R-PLACE1009581//ESTS, Weakly similar to FIBRINOGEN ALPHA AND ALPHA-E CHAIN PRECURSORS [H.sapiens]//0.0012:56:91//Hs.12151:AA001818
R-PLACE1009595//Homo sapiens mRNA for KIAA0635 protein, complete cds//6.0e-42:547:70//Hs.69157:AB014535
R-PLACE1009596//ESTs//1.9e-102:588:90//Hs.142395:AI374735
R-PLACE1009607//ESTs//0.0093:107:70//Hs.70932:AA126482
R-PLACE1009613//ESTs//7.5e-101:488:97//Hs.5905:AA946680
R-PLACE1009621//EST//0.99:261:60//Hs.149030:AI243338
R-PLACE1009622//ESTs//8.0e-93:508:92//Hs.20967:AI422858
R-PLACE1009637//EST//8.7e-90:442:97//Hs.121372:AA758701
R-PLACE1009639//EST//8.5e-49:279:93//Hs.117447:R27213
R-PLACE1009659//Homo sapiens mRNA for KIAA0587 protein, complete cds//3.3e-109:589:92//Hs.21862:AB011159
R-PLACE1009665//ESTs, Weakly similar to line-1 protein ORF1 [H.sapiens]//9.9e-62:483:79//Hs.140416:AA778649
R-PLACE1009670//Homo sapiens genethonin 1 mRNA, complete cds//6.6e-63:310:97//Hs.109590:AF062534
R-PLACE1009708//ESTs//3.Oe-94:471:96//Hs.40091:N48582
R-PLACE1009721//ESTs, Weakly similar to MSF1 PROTEIN [S.cerevisiae]//4.2e-98:529:92//Hs.3945:AA004210
R-PLACE1009731/TESTs, Weakly similar to immune associated protein 38 [M.musculus]//6.8e-85:489:89//Hs.26194:AA033989
R-PLACE1009763//Homo sapiens UBA3 (UBA3) mRNA, complete cds//2.0e-117:598:95//Hs.154320:AF046024
R-PLACE1009794//ESTs//7.9e-102:529:95//Hs.42927:N20989
R-nnnnnnnnnnnn//Human DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs and GSSs//1.1e-113:549:97//Hs.16411:AL030996
R-PLACE1009845//ESTs//9.5e-106:560:93//Hs.117751:AI056868
R-PLACE1009879//ESTs//1.8e-61:399:86//Hs.141012:R68748
R-PLACE1009886//EST//0.54:153:64//Hs.144281:AA081328
R-PLACE1009888//ESTs//2.7e-105:520:97//Hs.108646:AA613031
R-nnnnnnnnnnnn//ESTs, Weakly similar to similar to mouse MMR1 [C.elegans]//1.6e-114:594:94//Hs.67466:AI219740
R-PLACE1009921//ESTs//7.6e-05:291:60//Hs.124786:AA825563
R-PLACE1009924//EST//1.2e-42:216:98//Hs.31742:H20276
R-PLACE1009925//ESTs//5.4e-30:154:100//Hs.114605:AI04317
R-PLACE1009935//ESTs//1.4e-83:417:97//Hs.131755:AA496543
R-PLACE1009947//Keratin 9//1.0:273:61//Hs.2783:Z29074
R-PLACE1009971//ESTs//1.5e-87:424:98//Hs.13781:AI160540
R-PLACE1009992//ESTs//1.3e-87:531:87//Hs.55044:AA460698
R-PLACE1009995//ESTs//1.3e-103:575:91//Hs.71218:C75347
R-PLACE1009997//Small inducible cytokine A5 (RANTES)//1.1e-42:286:86//Hs.155464:AF088219
R-PLACE1010023//ESTs, Weakly similar to C27F2.7 gene product [C.elegans]//1.7e-17:137:86//Hs.7049:AI141736
R-PLACE1010031//ESTs//0.22:191:62//Hs.127787:AA832204
R-PLACE1010053//ESTs, Moderately similar to spermatid perinuclear RNA-binding protein Spur [M.musculus]//7.6e-104:546:94//Hs.8215:AA521150
R-PLACE1010069//ESTs//0.99:173:59//Hs.21415:AI150905
R-PLACE1010074//Homo sapiens sorting nexin 2 (SNX2) mRNA, complete cds//1.5e-88:543:88//Hs.11183AF065482
R-PLACE1010076//ESTs//3.4e-106:530:95//Hs.28005:AA604375
R-PLACE1010083//ESTs//4.1e-65:395:88//Hs.6103:AA496424
R-PLACE1010089//ESTs//1.6e-70:348:97//Hs.9011:AA418615
R-PLACE1010096//ESTs, Highly similar to hypothetical protein, 100K [R.norvegicus]//2.8e-104:565:92//Hs.11469:U69567
R-PLACE1010102//ESTs//7.7e-50:311:89//Hs.5518:AI052015
R-PLACE1010105//ESTs//6.0e-94:483:94//Hs.62684:AA806103
R-PLACE1010106//ESTs, Weakly similar to putative p150 [H.sapiens]//1.6e-107:575:93//Hs.48301:AA122270
R-PLACE1010134//EST//8.5e-59:314:94//Hs.135005:AI095130
R-PLACE1010148//A-KINASE ANCHOR PROTEIN 79//0.52:351:56//Hs.48714:M90359
R-PLACE1010152//ESTS//1.9e-40:240:90//Hs.17054:AI139897
R-PLACE1010181//ESTs//3.6e-64:307:99//Hs.154163:AJ003313
R-PLACE1010194//ESTs//2.7e-70:366:96//Hs.5301:T58466
R-PLACE1010202//ESTs//0.57:120:67//Hs.58873:W95037
R-PLACE1010231
R-PLACE1010261//EST//6.9e-50:251:98//Hs.148208:AA897478
R-PLACE1010270//ESTs//1.9e-87:430:96//Hs.25252:AI079545
R-PLACE1010274//ESTs//1.9e-57:439:81//Hs.30078:H04535
R-PLACE1010293//ESTs//8.1e-41:310:81//Hs.146811:AA410788
R-PLACE1010321//ESTs//5.7e-50:246:99//Hs.151445:AA351081
R-PLACE1010324//ESTs//0.00025:377:60//Hs.97430:AA398568
R-PLACE1010329//Small inducible cytokine A5 (RANTES)//2.4e-40:300:82//Hs.155464:AF088219
R-PLACE1010341//EST, Moderately similar to !!!! ALU SUBFAMILY SQ WARNING ENTRY !!!! [H.sapiens]//9.9e-32:190:77//Hs.152369:AA504818
R-PLACE1010362//ESTs//8.2e-86:404:99//Hs.25625:AA669327
R-PLACE1010364//ESTs//1.5e-105:556:93//Hs.12229:AA149594
R-PLACE1010383//Homo sapiens mRNA for putative lipoic acid synthetase, partial//4.9e-35:166:86//Hs.53531:AJ224162
R-PLACE1010401//ESTs//2.3e-85:450:93//Hs.23193:AA418152
R-PLACE1010481//ESTs//0.012:280:59//Hs.5579:AI392816
R-PLACE1010491//Homo sapiens Cre binding protein-like 2 mRNA, complete cds//2.4e-89:438:96//Hs.13313:AF039081
R-PLACE1010492
R-PLACE1010522//EST//0.43:82:68//Hs.89303:AA284031
R-nnnnnnnnnnnn//ESTs//3.4e-36:228:89//Hs.128724:AA215455
R-PLACE1010562//ESTs//4.8e-68:408:90//Hs.17244:W86306
R-PLACE1010579//EST//0.015:193:63//Hs.67093:C14033
R-PLACE1010580//ESTs//2.4e-93:445:98//Hs.127325:AA234116
R-PLACE1010599
R-PLACE1010616//ESTs//2.9e-101:497:97//Hs.142197:AA573418
R-PLACE1010622//ESTs//7.1e-23:157:91//Hs.159877:N57895
R-PLACE1010624//ESTs//1.4e-89:428:98//Hs.116561:AA658475
R-PLACE1010628//ESTS, Weakly similar to !!!! ALU SUBFAMILY J WARNING ENTRY !!!! [H.sapiens]//6.4e-74:391:95//Hs.163495:W57637
R-PLACE1010629//ESTs//5.8e-75:359:99//Hs.123630:AI250805
R-PLACE1010630//ESTs//9.5e-101:519:94//Hs.77873:AA731719
R-PLACE1010631//Homo sapiens mRNA for KIAA0530 protein, partial cds//8.3e-94:497:93//Hs.10801:AB011102
R-PLACE1010661//ESTs, Highly similar to TESTIS-SPECIFIC PROTEIN PBS13 [Mus musculus]//4.8e-83:467:91//Hs.22383:R51067
R-PLACE1010662//ESTs, Weakly similar to UDP-GLUCOSE:GLYCOPROTEIN GLUCOSYLTRANSFERASE PRECURSOR [D.melanogaster]//8.3e-103:538:94//Hs.105794:AA701659
R-PLACE1010702//Homo sapiens DNA from chromosome 19, BAC 33152//4.8e-46:531:71//Hs.55452:AC003973
R-PLACE1010714//Human organic anion transporting polypeptide (OATP) mRNA, complete cds//0.0074:351:60//Hs.46440:U21943
R-PLACE1010720//Homo sapiens chromosome-associated protein-C (hCAP-C) mRNA, partial cds//1.2e-56:300:95//Hs.50758:AF092564
R-PLACE1010739//Homo sapiens mRNA for oligophrenin 1//2.6e-84:501:88//Hs.158122:AJ001189
R-PLACE1010743
R-PLACE1010761//Homo sapiens okadaic acid-inducible phosphoprotein (OA48-18) mRNA, complete cds//5.2e-94:442:96//Hs.3688:AF069250
R-PLACE1010771//ESTs//3.8e-54:264:99//Hs.27299:AI074024
R-PLACE1010786//ESTs, Highly similar to MYOSIN HEAVY CHAIN IB [Acanthamoeba castellanii]//7.6e-111:575:94//Hs.10260:AI126627
R-PLACE1010800//ESTs//1.9e-109:557:95//Hs.11460:AA057558
R-PLACE1010802//ESTs//0.00021:428:5 8//Hs.70258:AI091203
R-PLACE1010811//ESTs//7.4e-73:394:93//Hs.48499:AA428896
R-PLACE1010833//ESTs//9.0e-33:274:78//Hs.24391:W27472
R-PLACE1010856//ESTs//5.8e-41:351:81//Hs.17401:W81048
R-PLACE1010857//ESTs, Weakly similar to T14B4.2 gene product [C.elegans]//1.4e-71:326:92//Hs.3385:N25917
R-PLACE1010870//ESTs//5.8e-57:303:96//1Hs.30503:H05090
R-PLACE1010877//Homo sapiens mRNA for KIAA0610 protein, partial cds//2.3e-101:501:96//Hs.118087:AB011182
R-PLACE1010891
R-PLACE1010896//EST//0.0039:249:57//Hs.126090:AA867983
R-PLACE1010900//Human Xq28 mRNA, complete cds//3.3e-07:106:76//Hs.20136:U46023
R-PLACE1010916//Plasminogen activator inhibitor, type II (arginine-serpin)//0.25:190:61//Hs.75716:Y00630
R-PLACE1010917//ESTs//1.3e-82:452:92//Hs.68055:AA081093
R-PLACE1010925//ESTs//1.1e-92:471:95//Hs.17448:AI125479
R-PLACE1010926//Homo sapiens mRNA for KIAA0554 protein, partial cds//1.3e-66:402:89//Hs.74750:AB011126
R-nnnnnnnnnnnn//Homo sapiens intersectin short form mRNA, complete cds//8.9e-82:441:93//Hs.66392:AF064244
R-PLACE1010944
R-PLACE1010947//ESTs//6.7e-15:102:91//Hs.116808:AA211519
R-PLACE1010954//Small inducible cytokine A5 (RANTES)//8.8e-51:278:93//Hs.155464:AF088219
R-PLACE1010960//ESTs, Highly similar to ACTIN-LIKE PROTEIN 13E [Drosophila melanogaster]//1.0e-103:565:92//Hs.23259:AA532437
R-PLACE1010965//EST//6.3e-80:447:91//Hs.139529:AA219580
R-PLACE1011026//ESTs//4.6e-99:463:99//Hs.149732:AI199846
R-PLACE1011032//ESTs//6.3e-56:295:94//Hs.143576:AI147867
R-PLACE1011041//ESTs//5.3e-27:168:91//Hs.7936:AA923249
R-nnnnnnnnnnnn//Homo sapiens mRNA for KIAA0581 protein, partial cds//9.4e-102:563:91//Hs.41143:AB011153
R-PLACE1011054//EST//1.1e-15:245:69//Hs.112648:AA609135
R-PLACE1011056//Small inducible cytokine A5 (RANTES)//3.5e-38:285:82//Hs.155464:AF088219
R-PLACE1011057//ESTs//3.5e-81:410:96//Hs.96499:AA252537
R-PLACE1011090//ESTS. Weakly similar to !!!! ALU SUBFAMILY J WARNING ENTRY !!!! [H.sapiens]//1.6e-54:398:84//Hs.108740:W20094
R-PLACE1011109//EST//1.3e-48:321:85//Hs.146794:AI149478
R-PLACE101111 4//ESTs//5.4e-90:475:94//Hs.69331:AA099587
R-PLACE1011133//ESTs, Highly similar to 40 KD PROTEIN [Borna disease virus]//3.0e-105:552:93//Hs.31257:AA875998
R-PLACE1011143//ESTs//0.40:127:65//Hs.118701:AA420795
R-PLACE1011160//Homa sapiens mRNA for HRIHFB2038, partial cds//7.7e-97:534:91//Hs.28719:AB015333
R-PLACE1011165//ESTs//1.0:135:69//Hs.32163:AI374673
R-PLACE1011185//ESTs, Weakly similar to !!!! ALU CLASS B WARNING ENTRY !!!! [H.sapiens]//3.4e-85:442:95//Hs.136910:AA810782
R-PLACE1011203//EST//0.0047:268:60//Hs.68832:AA088438
R-PLACE1011219//ESTs//7.6e-96:504:93//Hs.124834:AI138671
R-PLACE1011221//ESTs//5.2e-23:241:78//Hs.26761:AA203299
R-PLACE1011229//ESTs//1.9e-90:461:95//Hs.132288:AI027693
R-PLACE1011263//ESTs//6.6e-56:321:93//Hs.158787:W79602
R-PLACE1011273//ESTs//0.016:131:65//Hs.140466:AA766772
R-PLACE1011291//EST//8.7e-47:267:91//Hs.158806:AI376913
R-PLACE1011296//EST//2.7e-38:225:92//Hs.160934:AI376849
R-PLACE1011310//ESTs//9.1e-37:196:96//Hs.39328:H71807
R-PLACE1011325//Human clone 23721 mRNA sequence//0.0012:486:58//Hs.83572:U79291
R-PLACE1011332//ESTs//8.4e-44:217:99//Hs.101365:R60578
R-PLACE1011340//ESTs, Weakly similar to TEICHOIC ACID BIOSYNTHESIS PROTEIN A [Bacillus subtilis]//3.4e-92:452:97//Hs.144194:AA706337
R-PLACE1011375//ESTs//2.2e-35:195:96//Hs.106486:H11376
R-PLACE1011399//ESTs//0.00096:224:67//Hs.151643:AA001194
R-PLACE1011419//ESTs//4.9e-50:267:95//Hs.7045:AA167337
R-nnnnnnnnnnnnn//Homo sapiens mRNA for KIAA0530 protein, partial cds//4.8e-114:600:94//Hs.10801:AB011102
R-PLACE1011452//Homo sapiens mRNA for KIAA0707 protein, partial cds//3.7e-32:310:76//Hs.138488:AB014607
R-PLACE1011465//ESTs//4.5e-86:471:93//Hs.144519:R70887
R-PLACE1011472//Homo sapiens mRNA for KIAA0712 protein, complete cds//2.6e-104:515:96//Hs.111138:AB018255
R-PLACE1011492//ESTs//1.7e-96:488:95//Hs.116555:AA639278
R-PLACE1011503//Homo sapiens clone 23597 mRNA sequence//1.0:193:60//Hs.28197:AF035294
R-PLACE1011520//ESTs//6.8e-99:477:97//Hs.85077:AA968576
R-PLACE1011563//ESTs//1.4e-94:514:92//Hs.16471:AA206421
R-PLACE1011567//EST//2.8e-89:417:100//Hs.149770:AI285985
R-PLACE1011576//Zinc finger protein 91 (HPF7, HTF10)//4.7e-55:267:81//Hs.8597:L11672
R-PLACE1011586//Myosin, heavy polypeptide 11, smooth muscle//0.98:168:61//Hs.78344:AF001548
R-PLACE1011635//ESTs//2.5e-67:332:98//Hs.108194:AA780067
R-PLACE1011641//ESTs//2.5e-71:J38:100//Hs.153085:AA993965
R-PLACE1011643//EST//1.9e-18:181:78//Hs.160879:AI361900
R-PLACE1011649//Homo sapiens clone 24432 mRNA sequence//2.5e-73:414:91//Hs.78019:AF070535
R-PLACE1011650//EST//5.8e-18:118:92//Hs.124486:AA846036
R-PLACE1011664//Restin (Reed-Steinberg cell-expressed intermediate filament-associated protein)//0.50:178:62//Hs.31638:X64838
R-PLACE1011675
R-PLACE1011682//ESTs//2.4e-90:465:94//Hs.57830:AI312025
R-PLACE1011719//Human Line-1 repeat mRNA with 2 open reading frames//8.5e-57:410:83//Hs.23094:M19503
R-PLACE1011725//ESTs//2.0e-70:340:98//Hs.161725:AA251392
R-PLACE1011729//ESTs//7.5e-19:180:79//Hs.119516:AA443426
R-PLACE1011749//Myelin oligodendrocyte glycoprotein {alternative products}//7.3e-40:361:77//Hs.53217:Z48051
R-PLACE1011762//Human kpni repeat mrna (cdna clone pcd-kpni-8), 3' end//3.0e-60:319:76//Hs.103948:K00627
R-PLACE1011778//ESTs//8.0e-70:372:94//Hs.46765:AA521080
R-PLACE1011783//Calcium modulating ligand//8.4e-41:279:85//Hs.13572:AF068179
R-PLACE1011858//ESTs//2.6e-69:396:91//Hs.55220:D11563
R-PLACE1011874//Human mRNA for KIAA0033 gene, partial cds//1.2e-53:439:80//Hs.22271:D26067
R-PLACE1011875//ESTs//9.0e-88:420:98//Hs.70897:AA987648
R-PLACE1011891//ESTs//3.9e-17:97:100//Hs.84698:AA725913
R-PLACE1011896//ESTs//2.8e-23:176:84//Hs.121540:AI275497
R-PLACE1011922//ESTs//6.6e-35:415:73//Hs.10972:AA164268
R-PLACE1011923//Homo sapiens serum-inducible kinase mRNA, complete cds//2.3e-99:546:92//Hs.3838:AF059617
R-PLACE1011962//ESTs//3.3e-49:294:90//Hs.106800:AI031969
R-PLACE1011964//ESTs, Weakly similar to LINE-1 REVERSE TRANSCRIPTASE HOMOLOG [H.sapiens]//2.6e-06:284:63//Hs.124102:AA701285
R-PLACE1011982//ESTs//2.9e-51:291:93//Hs.20792:R14890
R-PLACE1011995//ESTs//4.5e-39:304:81//Hs.138852:AA284247
R-PLACE1012031//Homo sapiens mRNA for KIAA0713 protein, partial cds//8.0e-106:540:95//Hs.88756:AB018256
R-PLACE2000003//ESTs//2.0e-103:488:98//Hs.8341:AA490069
R-PLACE2000007//ESTs//2.4e-110:564:95//Hs.65135:W89120
R-PLACE2000011//Homo sapiens clone 614 unknown mRNA, complete sequence//4.8e-105:524:95//Hs.21811:AF091080
R-PLACE2000015//ESTs//7.1e-111:543:96//Hs.32178:AA083211
R-PLACE2000017//EST//8.2e-46:404:79//Hs.133006:AI049504
R-PLACE2000021//EST//4.5e-19:221:71//Hs.150830:AI302868
R-PLACE2000033//Human melanoma antigen recognized by T-cells (MART-1) mRNA//1.6e-43:355:79//Hs.154069:U06452
R-PLACE2000034//ESTs//2.2e-21:314:70//Hs.107697:W29013
R-PLACE2000039//H.sapiens mRNA for translin associated protein X//2.9e-45:514:72//Hs.96247:X95073
R-PLACE2000047//Homo sapiens class-I MHC-restricted T cell associated molecule (CRTAM) mRNA, complete cds//4.1e-45:358:81//Hs.159523:AF001622
R-PLACE2000050//ESTs//4.5e-65:322:98//Hs.155820:N67652
R-PLACE2000061//Homo sapiens mRNA for KIAA0575 protein, complete cds//9.2e-41:429:72//Hs.153468:AB011147
R-PLACE2000062//Human mRNA for KIAA0392 gene, partial cds//2.0e-43:296:86//Hs.40100:AB002390
R-PLACE2000072//Homo sapiens ZNF202 alpha (ZNF202) mRNA, complete cds//6.2e-111:550:95//Hs.9443:AF027219
R-PLACE2000097//Calcium modulating ligand//6.2e-47:372:80//Hs.13572:AF068179
R-PLACE2000100//ESTs//8.8e-42:281:86//Hs.150727:AI292236
R-PLACE2000103//ESTs//4.7e-97:518:93//Hs.118727:W26941
R-PLACE2000111//Homo sapiens ubiquitin hydrolyzing enzyme I (UBH1) mRNA, partial cds//0.00043:127:71//Hs.42400:AF022789
R-PLACE2000115//ESTs//7.8e-93:458:96//Hs.104520:AA481662
R-PLACE2000132//ESTs//3.8e-69:409:91//Hs.98502:AA433988
R-PLACE2000136//ESTs//6.2e-05:274:61//Hs.114067:AA701558
R-PLACE2000140//Homo sapiens mRNA for KIAA0562 protein, complete cds//4.7e-44:302:85//Hs.118401:AB011134
R-PLACE2000164//ESTs//6.3e-106:506:98//Hs.16390:AI052357
R-PLACE2000170//Small inducible cytokine A5 (RANTES)//3.7e-42:326:79//Hs.155464:AF088219
R-PLACE2000172//ESTs//9.6e-43:232:94//Hs.6709:AI379778
R-PLACE2000176//EST//1.6e-24:154:91//Hs.157734:AI360292
R-PLACE2000187//Human mRNA for KIAA0033 gene, partial cds//2.0e-49:292:90//Hs.22271:D26067
R-PLACE2000216//ESTS//0.0041:166:64//Hs.159476:AI382378
R-PLACE2000223//ESTs//0.49:171:60//Hs.86154:AA207191
R-PLACE2000235//ESTs//2.9e-39:264:85//Hs.136839:H93717
R-PLACE2000246//NAD(P)H:menadione oxidoreductase//4.0e-44:331:82//Hs.80706:M81600
R-PLACE2000264//Human mRNA for KIAA0365 gene, partial cds/4.0e-38:311:81//Hs.84123:AB002363
R-PLACE2000274//ESTs, Weakly similar to dynein-related protein [H.sapiens]//1.9e-87:422:98//Hs.9740:AI004779
R-PLACE2000302//ESTs, Highly similar to THREONYL-TRNA SYNTHETASE, CYTOPLASMIC [Homo sapiens]//4.8e-68:380:92//Hs.107365:AA720664
R-PLACE2000305//ESTs//2.6e-43:413:75//Hs.I18732:AI344055
R-PLACE2000317//ESTs//2.8e-92:501:92//Hs.28432:R83380
R-PLACE2000335//ESTs//4.3e-32:300:77//Hs.163035:AA748058
R-PLACE2000342//Homo sapiens ubiquitin hydrolyzing enzyme I (UBH1) mRNA, partial cds//0.00071:117:73//Hs.42400:AF022789
R-PLACE2000347//ESTs//1.6e-30:214:86//Hs.135272:AI347618
R-PLACE2000359//Zinc finger protein 139 (clone pHZ-37)//5.5e-42:288:86//Hs.140090:U09848
R-PLACE2000366//Thromboxane A2 receptor//6.7e-53:392:82//Hs.89887:D38081
R-PLACE2000371//ESTs//3.6e-81:409:97//Hs.155138:AA158731
R-PLACE2000373//Homo sapiens mRNA for KIAA0734 protein, partial cds//0.89:186:62//Hs.101516:AB018277
R-PLACE2000379//ESTs//3.4e-10:228:64//Hs.57842:W63781
R-PLACE2000394//ESTs//6.7e-41:462:74//Hs.107657:AA126814
R-PLACE2000398//ESTs//4.2e-33:373:74//Hs.155184:AA573189
R-PLACE2000399
R-PLACE2000404//ESTs, Highly similar to LEUCYL-TRNA SYNTHETASE, CYTOPLASMIC [Saccharomyces cerevisiae]//4.2e-109:540:96//Hs.6762:AA088424
R-PLACE2000411//ESTs//1.6e-89:459:95//Hs.117589:N25941
R-PLACE2000419//ESTs, Weakly similar to F25H9.6 [C.elegans]//1.6e-97:436:95//Hs.24647:W19739
R-PLACE2000425//Homo sapiens PEC-205 mRNA, complete cds//2.2e-44:287:88//Hs.153563:AF011333
R-PLACE2000427//ESTs, Weakly similar to coded for by C. elegans cDNA CEESI42F [C.elegans]//3.0e-113:543:97//Hs.16933:AA976002
R-PLACE2000433//ESTs//1.8e-46:311:85//Hs.145032:AA343523
R-PLACE2000435//ESTs//2.9e-33:243:87//Hs.90964:AA393986
R-PLACE2000438//ESTs//2.8e-09:66:96//Hs.59548:AI279887
R-PLACE2000450//Human mRNA for KIAA0392 gene, partial cds//3.3e-39:394:74//Hs.40100:AB002390
R-PLACE2000455//ESTs//1.2e-62:301:99//Hs.151708:AA554714
R-PLACE2000458//ESTs//6.8e-92:473:96//Hs.115897:AA156638
R-PLACE2000465//ESTs//1.3e-45:435:76//Hs.141635:N79228
R-PLACE2000477//ESTs//2.6e-100:536:94//Hs.77822:AA532642
R-PLACE3000004//ESTs//9.1e-114:558:97//Hs.13035:AA151838
R-PLACE3000029//Homo sapiens mRNA for KIAA0575 protein, complete cds//6.3e-64:350:86//Hs.153468:AB011147
R-PLACE3000059//EST//0.028:175:61//Hs.159873:R92763
R-PLACE3000070//ESTs//3.8e-16:200:74//Hs.138771:N70979
R-PLACE3000103//ISLET AMYLOID POLYPEPTIDE PRECURSOR//3.7e-48:468:75//Hs.51048:X68830
R-PLACE3000119//ESTs//1.2e-45:330:83//Hs.35254:AI133727
R-PLACE3000124//EST//3.1e-75:391:96//Hs.161515:N71739
R-PLACE3000136//ESTs//8.3e-18:152:84//Hs.10043:D81792
R-PLACE3000142//ESTs//0.047:183:62//Hs.43102:AA131369
R-PLACE3000147//ESTs//6.6e-53:310:90//Hs.8230:W07142
R-PLACE3000148//EST//1.9e-16:184:76//Hs.146570:AI139815
R-PLACE3000155//ESTS//1.2e-19:192:79//Hs.131350:AA805223
R-PLACE3000156//ESTs, Highly similar to ENV POLYPROTEIN [Avian spleen necrosis virus]//4.8e-36:262:88//Hs.31532:H18272
R-PLACE3000157
R-PLACE3000158//Small inducible cytokine A5 (RANTES)//8.2e-39:296:81//Hs.155464:AF088219
R-PLACE3000160
R-PLACE3000169//ESTs//1.5e-64:329:97//Hs.129864:R20798
R-PLACE3000194
R-PLACE3000197//ESTs//1.4e-3 8:197:98//Hs.146341:AI269930
R-PLACE3000199//ESTs, Highly similar to APOLIPOPROTEIN E PRECURSOR [Sus scrofa]//0.018:261:61//Hs.131370:AA927516
R-PLACE3000207//EST//1.3e-15:154:78//Hs.136617:AA630476
R-PLACE3000208//ESTS//1.6e-18:151:82//Hs.155498:W27084
R-PLACE3000218//ESTs//1.8e-85:463:93//Hs.7849:AI129964
R-PLACE3000220//ESTs//6.4e-44:308:84//Hs.136839:H93717
R-PLACE3000226//ESTs//L3e-49:269:95//Hs.9059:AI359014
R-PLACE3000230//EST//2.3e-34:258:83//Hs.4382:T02878
R-PLACE3000242//Human trophinin mRNA, complete cds//1.1e-63:546:78//Hs.76313:U04811
R-PLACE3000244//ESTs, Highly similar to NEGATIVE REGULATOR OF MITOSIS [Emericella nidulans]//7.5e-110:549:95//Hs.13692:AA632002
R-PLACE3000254//Human mRNA for KIAA0309 gene, partial cds//2.4e-29:174:94//Hs.87908:AB002307
R-PLACE3000271//Human macrophage-derived chemokine precursor (MDC) mRNA, complete cds//2.3e-62:287:82//Hs.97203:U83171
R-PLACE3000276//ESTs//7.5e-07:187:64//Hs.80720:AA031782
R-PLACE3000304//Human 53K isoform of Type II phosphatidylinositol-4-phosphate 5-kinase (PIPK) mRNA, complete cds//4.0e-59:456:80//Hs.108966:U48696
R-PLACE3000310//ISLET AMYLOID POLYPEPTIDE PRECURSOR//6.0e-45:302:86//Hs.51048:X68830
R-PLACE3000320//Interleuldn 10//9.6e-42:288:85//Hs.2180:M57627
R-PLACE3000322//ESTs, Highly similar to ARGININOSUCCINATE LYASE [Homo sapiens]//5.8e-34:190:95//Hs.114531:N74103
R-PLACE3000331//Homo sapiens mRNA for KIAA0772 protein, complete cds//3.7e-32:239:84//Hs.15519:AB018315
R-PLACE3000339//ESTs//1.3e-109:548:96//Hs.7871:AI041837
R-PLACE3000341//EST//1.1e-11:231:68//Hs.131328:AA922688
R-PLACE3000350//Human mRNa for adipogenesis inhibitory factor//8.0e-40:291:76//Hs.1721:X58377
R-PLACE3000352//EST//1.8e-72:343:100//Hs.144871:AI202380
R-PLACE3000353//ESTs//2.0e-75:395:95//Hs.107260:W52683
R-PLACE3000362//EST//2.8e-80:381:99//Hs.136233:AA261888
R-PLACE3000363
R-PLACE3000365//EST//4.8e-50:307:88//Hs.149580:AI281881
R-PLACE3000373//ESTs//5.8e-60:422:83//Hs.142826:W87430
R-PLACE3000388//ESTs, Moderately similar to !!!! ALU SUBFAMILY J WARNING ENTRY !!!! [H.sapiens]//1.0e-35:427:73//Hs.138795:R98534
R-PLACE3000399//ESTs//6.5e-05:162:66//Hs.149440:AI274570
R-PLACE3000400//ESTs//8.3e-05:310:63//Hs.17697:AA287528
R-PLACE3000401//ESTs//4.6e-60:326:80//Hs.139555:N48230
R-PLACE3000402//Homo sapiens clone 24629 mRNA sequence//0.50:227:62//Hs.142570:AF052l60
R-PLACE3000405//Human HsLIM15 mRNA for HsLim15, complete cds//5.3e-43:315:82//Hs.37181:D64108
R-PLACE3000406//Human high-affinity copper uptake protein (hCTR1) mRNA, complete cds//4.4e-47:302:87//Hs.73614:U83460
R-PLACE3000413//ESTs//1.6e-116:571:97//Hs.10235:H93077
R-PLACE3000416//Small inducible cytokine A5 (RANTES)//1.8e-41:300:85//Hs.155464:AF088219
R-PLACE3000425//Homo sapiens 4F5S mRNA, complete cds//1.6e-46:307:85//Hs.32567:AF073519
R-PLACE3000455//ESTs//1.0:160:64//Hs.156045:AA884461
R-PLACE3000475//Human signal transducing adaptor molecule STAM mRNA, complete cds//6.1e-84:440:92//Hs.153487:U43899
R-PLACE3000477//ESTs//2.4e-113:568:96//Hs.24557:AA142980
R-PLACE4000009//ESTs//1.5e-72:361:96//Hs.10119:AA700227
R-PLACE4000014//Homo sapiens mRNA for KIAA0809 protein, partial cds//8.8e-85:433:95//Hs.105399:AB018352
R-PLACE4000034//ESTs//7.0e-110:550:96//Hs.76607:AA156240
R-PLACE4000049//EST//0.028:87:75//Hs.89303:AA284031
R-PLACE4000052//ESTs//5.6e-116:553:98//Hs.19067:AA521292
R-PLACE4000063//ESTs//5.0e-80:388:98//Hs.135028:AI096444
R-PLACE4000089//ESTs//2.3e-97:479:97//Hs.102425:AA807547
R-PLACE4000093//ESTs//1.5e-82:391:99//Hs.160730:AI142739
R-PLACE4000100
R-PLACE4000106//Homo sapiens mRNA for KIAA0462 protein, partial cds//2.7e-98:419:91//Hs.129937:AB007931
R-PLACE4000128//ESTs, Moderately similar to !!!! ALU SUBFAMILY J WARNING ENTRY !!!! [H.sapiens]//3.8e-11:184:71//Hs.154278:N45985
R-PLACE4000129//Homo sapiens mRNA, chromosome 1 specific transcript KIAA0500//5.2e-21:118:100//Hs.118164:AB007969
R-PLACE4000147//EST//1.6e-23:175:79//Hs.162236:AA551582
R-PLACE4000156//Homo sapiens mRNA for KIAA0575 protein, complete cds//3.0e-47:306:88//Hs.153468:AB011147
R-PLACE4000192//ESTs, Weakly similar to similar to Human zinc finger protein(ZNF142) [H.sapiens]//6.7e-31:232:82//Hs.16493:T92186
R-PLACE4000222//ESTs//2.2e-53:195:85//Hs.141575:AA211734
R-PLACE4000233//ESTs//2.9e-81:456:93//Hs.124964:R81949
R-PLACE4000247//Homo sapiens PYRIN (MEFV) mRNA, complete cds//5.5e-72:307:85//Hs.113283:AF018080
R-PLACE4000250//Small inducible cytokine A5 (RANTES)//7.1e-43:301:83//Hs.155464:AF088219
R-PLACE4000252//EST//1.6e-40:275:85//Hs.162197:AA535216
R-PLACE4000261//EST//0.0063:384:58//Hs.136284:AA400442
R-PLACE4000269//ESTs//7.3e-67:345:97//Hs.5000:R44586
R-PLACE4000270//Homo sapiens apoptotic protease activating factor 1 (Apaf-1) mRNA, complete cds//2.1e-37:352:77//Hs.77579:AF013263
R-PLACE4000300//EST//0.26:103:68//Hs.144438:AA780782
R-PLACE4000320//EST//2.7e-44:298:85//Hs.162404:AA573131
R-PLACE4000323//ESTs//8.8e-38:178:79//Hs.155475:AA761454
R-PLACE4000326//ESTs//7.4e-103:516:96//Hs.55042:AA150460
R-PLACE4000344//ESTs//9.9e-94:463:96//Hs.100057:AA001414
R-PLACE4000367//ESTs//0.81:102:73//Hs.107692:H38478
R-PLACE4000369//ESTs//1.5e-69:390:92//Hs.13733:AA418656
R-PLACE4000379//ESTs//1.3e-67:373:91//Hs.48569:AA905425
R-PLACE4000387//EST, Moderately similar to !!!! ALU SUBFAMILY SQ WARNING ENTRY !!!! [H.sapiens]//1.9e-44:379:78//Hs.152369:AA504818
R-PLACE4000392//ESTs, Weakly similar to line-1 protein ORF1 [H.sapiens]//2.3e-70:482:83//Hs.140416:AA778649
R-PLACE4000401//ESTs//1.3e-18:151:84//Hs.150355:AI273502
R-PLACE4000411//ESTs//1.1e-108:543:96//Hs.23901:AA169780
R-PLACE4000445//ESTs, Weakly similar to C05D9.6 gene product [C.elegans]//2.6e-111:530:98//Hs.12003:AA643063
R-PLACE4000465//Cytochrome P450, subfamily I (aromatic compound-inducible), polypeptide 2//8.5e-58:409:72//Hs.1361:M55053
R-PLACE4000489//ESTs//5.0e-70:342:98//Hs.72865:AI380932
R-PLACE4000494//EST&//1.4e-109:525:98//Hs.22539:AI334210
R-PLACE4000522//ESTs//6.3e-88:471:93//Hs.8121:AA521290
R-PLACE4000548//ESTs//3.3e-86:441:96//Hs.5070:AA149527
R-PLACE4000558//Human putative monocarboxylate transporter (MCT) mRNA, complete cds//5.7e-46:425:76//Hs.23590:U59185
R-THYRO1000026//ESTs//2.6e-42:331:82//Hs.137875:AA993532
R-THYRO1000034//ESTs//2.1e-43:214:100//Hs.153018:AI243524
R-THYRO1000035//ESTs//7.6e-52:325:90//Hs.49817:AA001249
R-THYRO1000040//ESTs//1.7e-94:459:98//Hs.48712:AI027889
R-THYRO1000070//ESTs//6.7e-43:283:86//Hs.37573:H59651
R-THYRO1000072//ESTs//1.3e-57:313:96//Hs.127827:H13438
R-THYRO1000085//ESTs//1.1e-90:439:98//Hs.150539:AA908435
R-THYRO1000092//Human mRNA for KIAA0355 gene, complete cds//1.3e-41:344:79//Hs.153014:AB002353
R-THYRO1000107//Interieuldn 10//2.8e-43:292:84//Hs.2180:M57627
R-THYRO1000111//ESTs, Highly similar to LINE-1 REVERSE TRANSCRIPTASE HOMOLOG [Homo sapiens]//1.0e-52:413:80//Hs.140385:AA773359
R-THYRO1000121//EST//0.24:78:74//Hs.156632:AI345108
R-THYRO1000124//ESTs//2.8e-86:428:96//Hs.141634:AI122764
R-THYRO1000129//Homo sapiens TED protein (TED) mRNA, complete cds//6.8e-90:449:96//Hs.87619:AF087142
R-THYRO1000132//ESTs, Moderately similar to !!!! ALU SUBFAMILY SC WARNING ENTRY !!!! [H.sapiens]//5.2e-49:486:77//Hs.24164:N95217
R-THYRO1000156//ESTs//6.1e-36:344:75//Hs.70279:AA757426
R-THYRO1000163//Homo sapiens LIM protein mRNA, complete cds//4.8e-38:278:84//Hs.154103:AF061258
R-THYRO1000173//ESTs, Highly similar to CLATHRIN COAT ASSEMBLY PROTEIN AP47 [Mus musculus]//1.1e-111:554:96//Hs.18894:AA910946
R-THYRO1000186//ESTs//1.0e-44:339:83//Hs.155184:AA573189
R-THYRO1000187//Small inducible cytokine A5 (RANTES)//1.1e-41:305:81//Hs.155464:AF088219
R-THYRO1000190//Small inducible cytokine A5 (RANTES)//2.3e-44:301:85//Hs.155464:AF088219
R-THYRO1000197//Homo sapiens mRNA for poly(A)-specific ribonuclease//3.6e-110:535:97//Hs.43445:AJ005698
R-THYRO1000199//Homo sapiens mRNA for KIAA0652 protein, complete cds//4.3e-115:559:97//Hs.79672:AB014552
R-THYRO1000206//ESTs//3.1e-90:507:90//Hs.32456:W29063
R-THYRO1000221//ESTs, Weakly similar to !!!! ALU SUBFAMILY J WARNING
ENTRY !!!! [H.sapiens]//1.1e-72:357:98//Hs.140002:AA635349
R-THYRO1000241//Homo sapiens mRNA for KIAA0688 protein, complete cds//7.8e-69:524:82//Hs.141874:AB014588
R-THYRO1000242//ESTs//4.2e-27:222:85//Hs.77554:W87927
R-THYRO1000253//Sialophorin (gpL115, leukosialin, CD43)//7.3e-40:318:80//Hs.80738:X52075
R-THYRO1000270//ESTs//1.9e-99:531:94//Hs.17767:N62925
R-THYRO1000279//EST//2.7e-54:266:99//Hs.149527:AI280674
R-THYRO1000288//Homo sapiens mRNA for Hs Ste24p, complete cds//3.5e-100:566:91//Hs.25846:AB016068
R-THYRO1000320//POLYPOSIS LOCUS PROTEIN 1//1.0:321:58//Hs.74648:M73547
R-THYRO1000327//Autocrine motility factor receptor//9.2e-54:289:93//Hs.80731:M63175
R-THYRO1000343//Homo sapiens mRNA for KIAA0790 protein, partial cds//3.4e-113:559:96//Hs.12002:AB018333
R-THYRO1000358//Human selenium-binding protein (hSBP) mRNA, complete cds//1.5e-48:317:87//Hs.7833:U29091
R-THYRO1000368//ESTs//4.7e-88:430:98//Hs.146085:AA021064
R-nnnnnnnnnnnn//ESTs//1.0:253:57//Hs.128783:AA436250
R-THYRO1000387//Homo sapiens ubiquitin conjugating enzyme G2 (UBE2G2) mRNA, complete cds//4.6e-69:294:84//Hs.151614:AF032456
R-THYRO1000394//Thromboxane A2 receptor//4.1e-40:232:87//Hs.89887:D38081
R-THYRO1000395//ESTs//3.3e-20:160:83//Hs.101570:AA505429
R-THYRO1000401//ESTs//1.3e-109:516:99//Hs.78524:AI140601
R-THYRO1000438//ESTs//2.1e-48:360:83//Hs.141203:H52638
R-THYRO1000452//ESTs, Weakly similar to No definition line found [C.elegans]//8.5e-40:239:90//Hs.84009:AI309761
R-THYRO1000471//ESTs//3.3e-36:302:80//Hs.70279:AA757426
R-THYRO1000484//Homo sapiens mRNA for KIAA0737 protein, complete cds//2.2e-49:479:75//Hs.17630:AB018280
R-THYRO1000488//Homa sapiens mRNA for HRIHFB2038, partial cds//4.1e-89:471:94//Hs.28719:AB015333
R-THYRO1000501//ESTs//L5e-46:287:89//Hs.125300:R62360
R-THYRO1000502//ESTs//1.7e-08:63:96//Hs.116319:AI208005
R-THYRO1000505//ESTs, Weakly similar to KIAA0281 [H. sapiens]//3.9e-57:286:96//Hs.105861:AI206965
R-THYRO1000558//ESTs//1.7e-95:454:99//Hs.125063:AA648511
R-THYRO1000569//ESTs//3.2e-89:463:94//Hs.20555:W22193
R-THYRO1000570//ESTs//2.8e-97:471:97//Hs.8245:AA115485
R-nnnnnnnnnnnn//Homo sapiens protein associated with Myc mRNA, complete cds//2.6e-108:533:97//Hs.151411:AF075587
R-THYRO1000596//ESTs//3.1e-99:527:94//Hs.6084:AA045247
R-THYRO1000602//EST//6.9e-50:381:83//Hs.161917:AA483223
R-THYRO1000605//ESTs, Weakly similar to monocytic leukaemia zinc finger protein [H.sapiens]//1.2e-96:483:96//Hs.21907:N24415
R-THYRO1000625//ESTs//5.6e-36:257:84//Hs.139657:AA191742
R-THYRO1000637
R-THYRO1000641//ESTs, Weakly similar to ERYTHROCYTE BAND 7 INTEGRAL MEMBRANE PROTEIN [H.sapiens]//4.9e-46:245:95//Hs.97398:AA398634
R-THYRO1000658//ESTs//5.8e-48:281:90//Hs.142259:AA828840
R-nnnnnnnnnnnn//ESTs//1.5e-82:389:99//Hs.155573:AA487384
R-THYRO1000666//ESTs//1.4e-26:179:88//Hs.98382:AA779866
R-THYRO1000676//EST//6.4e-05:88:77//Hs.133424:AI061063
R-THYRO1000684//ESTs//1.9e-69:374:94//Hs.144617:R77109
R-THYRO1000699//ESTs//1.7e-58:394:86//Hs.26373:AA700713
R-THYRO1000712
R-THYRO1000734//EST//2.0e-06:95:73//Hs.156201:AA724287
R-THYRO1000748//EST//4.1e-12:155:74//Hs.118694:AA148713
R-THYRO1000756//ESTs, Weakly similar to CMP-N-ACETYLNEURAMINATE-BETA-GALACTOSAMIDE-ALPHA-2,3-SIALYLTRANSFERASE [H.sapiens]//8.1e-82:497:87//Hs.109672:W22624
R-THYRO1000777
R-THYRO1000783//EST//5.6e-100:470:99//Hs.123515:AA812932
R-THYRO1000787//EST//8.0e-34:175:99//Hs.99607:AA463897
R-THYRO1000793//ESTs//2.2e-106:505:99//Hs.50929:AA443144
R-THYRO1000796//ESTs//4.3e-44:445:75//Hs.55855:AA621381
R-THYRO1000805//EST//2.6e-32:407:67//Hs.123424:AA813594
R-THYRO1000815//Human mRNA for KIAA0033 gene, partial cds//2.0e-56:307:87//Hs.22271:D26067
R-THYRO1000829
R-THYRO1000843//Interleukin 10//1.1e-44:285:87//Hs.2180:M57627
R-THYRO1000852//EST//2.3e-20:157:85//Hs.149580:AI281881
R-THYRO1000855//ESTs//2.6e-44:359:81//Hs.140329:AA714011
R-THYRO1000865//Protein kinase, interferon-inducible double stranded RNA dependent//2.8e-44:374:79//Hs.73821:M35663
R-THYRO1000895//ESTs//1.0e-32:196:85//Hs.138630:H97871
R-THYRO1000916//ESTs//4.6e-99:492:96//Hs.152442:AA528234
R-THYRO1000926//Homo sapiens cAMP-specific phosphodiesterase 8B (PDE8B) mRNA, partial cds//3.1e-110:566:94//Hs.78106:AF079529
R-THYRO1000934//ESTs//7.4e-102:535:95//Hs.58194:W72182
R-THYRO1000951//ESTs//4.2e-11:91:89//Hs.6278:T15859
R-THYRO1000952//ESTs//3.9e-93:489:94//Hs.48928:AA211761
R-THYRO1000974//Homo sapiens ribosomal protein L33-like protein mRNA, complete cds//1.1e-60:321:95//Hs.14454:AF047440
R-THYRO1000975//EST//9.8e-49:303:89//Hs.149580:AI281881
R-THYRO1000983//ESTs, Highly similar to UBIQUITIN-CONJUGATING ENZYME E2-17 KD 11 [Arabidopsis thaliana]//1.6e-90:474:93//Hs.106616:AI027524
R-THYRO1000984//ESTs//5.9e-97:481:96//Hs.142457:AI202777
R-THYRO1000988//EST//3.5e-42:241:83//Hs.162404:AA573131
R-THYRO1001003//ESTs, Weakly similar to ubiquitin-conjugating enzyme [H.sapiens]//3.0e-57:341:91//Hs.44049:AA521489
R-THYRO1001031//ESTs//5.5e-47:322:85//Hs.136839:H93717
R-THYRO1001033//ESTs//5.7e-89:427:98//Hs.71508:AA809070
R-THYRO1001062//EST//1.5e-46:291:89//Hs.161917:AA483223
R-THYRO1001093//ESTs//2.7e-80:468:90//Hs.124601:AA203497
R-THYRO1001100
R-THYRO1001120//ESTs, Moderately similar to fractionated X-irradiation-induced 29 thymoma [M.musculus]//6.6e-86:491:89//Hs.89135:AI138834
R-THYRO1001121//Homo sapiens mRNA for beta-tubulin folding cofactor D//2.6e-82:429:94//Hs.12570:AJ006417
R-THYRO1001133//ESTs//2.9e-39:242:90//Hs.152340:AA521399
R-THYRO1001134//ESTs//1.8e-102:521:95//Hs.108408:N31922
R-THYRO1001142//ESTs//0.26:84:69//Hs.153434:AI287853
R-THYRO1001173//Human mRNA for KIAA0238 gene, partial cds//0.0012:305:62//Hs.82042:D87075
R-THYRO1001177
R-THYRO1001189//H.sapiens F11 mRNA//1.5e-59:260:83//Hs.159639:X77744
R-THYRO1001204//ESTs, Weakly similar to TH1 protein [D.melanogaster]//1.0e-75:431:91//Hs.5184:AA709151
R-THYRO1001213//ESTs//1.3e-75:409:92//Hs.140213:AA828932
R-THYRO1001262//Human kpni repeat mrna (cdna clone pcd-kpni-4), 3' end//1.3e-48:349:83//Hs.139107:K00629
R-THYRO1001271//PUTATIVE PROTEIN PHOSPHATASE 2C//1.0:128:64//Hs.118728:D13640
R-THYRO1001290//ESTs//2.1e-89:424:99//Hs.118152:AA702561
R-THYRO1001313//ESTs//3.5e-17:139:87//Hs.15827:H16269
R-THYRO1001320//ESTs//1.4e-61:403:79//Hs.139555:N48230
R-THYRO1001321//Hypoxanthine phosphoribosyltransferase 1 (Lesch-Nyhan syndrome)//8.5e-05:326:60//Hs.82314:M31642
R-nnnnnnnnnnnnn//ESTs//0.16:422:5.9//Hs.23876:AA082935
R-THYRO1001347//ESTs, Weakly similar to C35A5.8 [C.elegans]//1.1e-106:562:94//Hs.15032:AA774250
R-THYRO1001363//ESTs//1.4e-99:508:95//Hs.5028:D51033
R-THYRO1001365
R-THYRO1001374
R-THYRO001401//Human HsLIM15 mRNA for HsLiml5, complete cds//2.5e-48:467:75//Hs.37181:D64108
R-THYRO1001403//Interleukin 10//2.1e-46:305:85//Hs.2180:M57627
R-THYRO1001405//ESTs//4.8e-25:197:84//Hs.6907:W72733
R-THYRO1001406//EST//0.0023:117:66//Hs.162931:AA633197
R-THYRO1001411//ESTs//6.1e-77:421:93//Hs.22973:R40979
R-THYRO1001426//Homo sapiens mRNA, chromosome 1 specific transcript KIAA0508//9.1e-49:305:86//Hs.159187:AB007977
R-THYRO1001434//ESTs//0.40:161:61//Hs.161993:AA503172
R-THYRO1001458//ESTs, Moderately similar to !!!! ALU SUBFAMILY SC WARNING ENTRY !!!! [H.sapiens]//1.7e-05:159:66//Hs.104239:AA488082
R-THYRO1001480//Small inducible cytokineA5 (RANTES)//1.3e-40:331:79//Hs.155464:AF088219
R-THYRO1001487//Homo sapiens mRNA for KIAA0563 protein, complete cds//2.1e-17:134:76//Hs.15731:AB011135
R-THYRO1001534//ESTs//4.6e-96:447:100//Hs.135204:AI093110
R-THYRO1001537//ESTS, Weakly similar to !!!! ALU SUBFAMILY J WARNING ENTRY !!!! [H.sapiens]//5.0e-33:304:80//Hs.108740:W20094
R-THYRO1001541//Human peptide transporter (HPEPT1) mRNA, complete cds//9.0e-49:427:76//Hs.2217:U21936
R-THYRO1001559//ESTs//0.99:210:62//Hs.33619:AA021594
R-THYRO1001570//ESTs//4.9e-48:287:91//Hs.27131:AA442413
R-THYRO1001573//ESTs//2.1e-87:446:95//Hs.143669:AA621958
R-THYRO1001584//ESTs//1.5e-64:354:95//Hs.146222:AA397741
R-THYRO1001595//ESTs//5.7e-39:366:78//Hs.22562:R54247
R-THYRO1001602//Insulin-like growth factor 1 (somatomedia C)//7.4e-12:288:67//Hs.85112:X57025
R-THYRO1001605//Human GS2 mRNA, complete cds//6.9e-49:359:83//Hs.264:U03886
R-THYRO1001617//Homo sapiens peroxisomal acyl-CoA:dihydroxyacetonephosphate acyltransferase (DHAPAT) mRNA, complete cds//1.3e-82:434:93//Hs.12482:AJ002190
R-THYRO1001637//Homo sapiens KIAA0414 mRNA, partial cds//7.1e-58:331:83//Hs.127649:AB007874
R-THYRO1001656//ESTs//3.8e-19:209:75//Hs.92186:AI080282
R-THYRO100166l//ESTs//1.4e-56:323:91//Hs.24984:AA534446
R-THYRO1001671//Homo sapiens mRNA for 2'-5' oligoadenylate synthetase 59 kDa isoform//-1.6e-111:562:95//Hs.118633:AJ225089
R-THYRO1001673//Homo sapiens mRNA, chromosome 1 specific transcript KIAA0488//1.0e-17:246:73//Hs.67619:AB007957
R-THYRO1001703//ESTs//1.1e-39:142:97//Hs.110748:AI341726
R-THYRO1001706//ESTs//2.2e-42:214:99//Hs.112536:AI147691
R-THYRO1001721
R-nnnnnnnnnnnnn//ESTs, Weakly similar to ZK1128.6 [C.elegans]//1.7e-10:147:77//Hs.158196:R53184
R-THYRO1001745//ELK1, member of ETS oncogene family//1.8e-12:282:65//Hs.116549:AL009172
R-THYRO1001746//EST//0.0073:226:61//Hs.146544:AI125323
R-THYRO1001772//ESTs//8.2e-100:495:97//Hs.144993:AA243474
R-THYRO1001793//ESTs//2.5e-89:430:97//Hs.58127:AA534224
R-THYRO1001809//ESTs//1.0e-41:327:80//Hs.146811:AA410788
R-THYRO1001854//Homo sapiens mRNA, chromosome 1 specific transcript KIAA0487//5.7e-38:242:83//Hs.92381:AB007956
R-THYRO1001895//ESTs//1.7e-08:213:64//Hs.156056:AI352123
R-THYRO1001907//ESTs, Moderately similar to !!!! ALU SUBFAMILY SC WARNING ENTRY !!!! [H.sapiens]//3.7e-41:362:79//Hs.139007:H74314
R-VESEN1000122
R-Y79AA1000013//ESTs//0.99:233:57//Hs.132216:AA923289
R-Y79AA1000033//EST//1.9e-62:324:95//Hs.157692:AI359321
R-Y79AA1000037//ESTs//6.1e-47:234:98//Hs.30773:AA557178
R-Y79AA1000059//Homo sapiens mRNA for KIAA0640 protein, partial cds//2.8e-51:330:89//Hs.153026:AB014540
R-Y79AA1000065//ESTs//2.0e-91:497:94//Hs.37759:H59629
R-Y79AA1000131//EST//2.3e-16:184:75//Hs.141501:N50792
R-Y79AA1000181//ESTs, Weakly similar to No definition line found [C.elegans]//2.4e-110:553:95//Hs.23159:AA113849
R-Y79AA1000202//Human mRNA for KIAA0169 gene, partial cds//0.094:185:62//Hs.79414:D79991
R-Y79AA1000214//ESTs//1.7e-93:495:94//Hs.11673:W68103
R-Y79AA1000230//ESTs//3.5e-114:553:98//Hs.47125:AI421812
R-Y79AA1000231//ESTs//1.1e-106:526:97//Hs.82856:AI246624
R-Y79AA1000258//ESTs//1.5e-99:490:97//Hs.6459:AI092936
R-Y79AA1000268//Human mRNA for KIAA0365 gene, partial cds//1.3e-44:320:84//Hs.84123:AB002363
R-Y79AA1000313//ESTs//1.7e-105:558:93//Hs.18851:AA857826
R-Y79AA1000328//ESTs//1.9e-76:448:91//Hs.16470:AA121635
R-Y79AA1000342//ESTs, Weakly similar to MATRIN 3 [H.sapiens]//2.0e-37:239:88//Hs.23476:AA401210
R-Y79AA1000346//ESTs//7.9e-12:139:76//Hs.115987:AA483808
R-Y79AA1000349//ESTs, Moderately similar to spermatid perinuclear RNA-binding protein Spnr [M.musculus]//4.4e-66:339:97//Hs.8215:AA521150
R-Y79AA1000355//ESTs, Moderately similar to !!!! ALU SUBFAMILY SC WARNING ENTRY !!!! [H.sapiens]//3.2e-44:279:88//Hs.139007:H74314
R-Y79AA1000368//ESTs//3.8e-97:513:94//Hs.68090:AA641018
R-Y79AA1000405//ESTs//4.4e-47:267:94//Hs.125304:R51613
R-Y79AA1000410//ESTs//7.4e-49:359:82//Hs.158107:AA707758
R-Y79AA1000420//EST//0.17:99:69//Hs.160859:AI352292
R-Y79AA1000469//ESTs, Highly similar to ancient ubiquitous 46 kDa protein AUP46 precursor [M.musculus]//3.1e-60:362:88//Hs.6381:AI188509
R-Y79AA1000480//ESTs//1.0e-75:433:91//Hs.78110:AA741320
R-Y79AA1000538//EST//7.9e-48:307:87//Hs.149580:AI281881
R-Y79AA1000539//Human kinesin-like spindle protein HKSP (HKSP) mRNA, complete cds//0.95:172:62//Hs.41723:U37426
R-Y79AA1000540//ESTs//1.5e-97:534:93//Hs.67991:AA147848
R-Y79AA1000560//ESTs, Highly similar to ALPHA-ADAPTIN [Rattus norvegicus]//8.2e-97:482:97//Hs.19121:AI125280
R-Y79AA1000574//ESTs, Weakly similar to M04B2.4 [C.elegans]//1.3e-107:564:93//Hs.16361:AI147455
R-Y79AA1000627//Homo sapiens zinc finger protein (ZF5128) mRNA, complete cds//3.4e-99:517:94//Hs.60580:AF060503
R-Y79AA1000705//ESTs, Weakly similar to HYPOTHETICAL 128.5 KD HELICASE IN ATS1-TPD3 INTERGENIC REGION [Saccharomyces cerevisiae]//8.1e-27:140:100//Hs.129049:H28818
R-Y79AA1000734//Homo sapiens peroxisomal biogenesis factor (PEX11b) mRNA, complete cds//8.7e-114:586:95//Hs.83023:AF093670
R-Y79AA1000748//ESTs, Weakly similar to HYPOTHETICAL 61.3 KD PROTEIN F25B5.5 IN CHROMOSOME III [C.elegans]//9.8e-111:563:95//Hs.19845:AI005330
R-Y79AA1000752//Homo sapiens (huc) mRNA, complete cds//0.97:235:59//Hs.1701:L26405
R-Y79AA1000774//ESTs//5.9e-109:559:95//Hs.17138:N91463
R-Y79AA1000782//Human mRNA for KIAA0246 gene, partial cds//1.6e-18:107:100//Hs.84753:D87433
R-Y79AA1000784//EST//0.80:87:67//Hs.158558:AI368359
R-Y79AA1000794//ESTs//2.7e-99:498:96//Hs.25441:AA580512
R-Y79AA1000800//ESTs//1.2e-97:532:93//Hs.77822:AA532642
R-nnnnnnnnnnnn//Carboxypeptidase E//0.018:354:59//Hs.75360:X51405
R-Y79AA1000805
R-Y79AA1000824//ESTs//0.99:276:61//Hs.153992:AA280227
R-Y79AA1000827//ESTs//1.2e-55:326:92//Hs.158127:AI334650
R-Y79AA1000850//Homo sapiens small optic lobes homolog (SOLH) mRNA, complete cds//0.016:386:59//Hs.55836:U85647
R-Y79AA1000962//EST//0.024:177:63//Hs.25214:R37079
R-Y79AA1000968
R-Y79AA1000969//ESTs//2.9e-70:251:98//Hs.120858:AA417181
R-Y79AA1000976//ESTs//7.8e-56:299:95//Hs.120125:M86049
R-Y79AA1000985
R-Y79AA1001023//ESTs//5.7e-66:379:90//Hs.64616:W22851
R-Y79AA1001041//ESTs//8.6e-06:54:100//Hs.8980:AA629067
R-Y79AA1001048//ESTs//4.4e-97:461:99//Hs.7010:AA837407
R-Y79AA1001061//ESTs//3.8e-105:493:99//Hs.128419:AI271325
R-Y79AA1001068//Homo sapiens mRNA for KIAA0563 protein, complete cds//4.8e-53:279:83//Hs.15731:AB011135
R-Y79AA1001077//ESTs//1.9e-51:339:87//Hs.11197:AA309047
R-Y79AA1001078//ESTs//8.3e-98:528:92//Hs.24608:AA161260
R-Y79AA1001105//ESTs//6.0e-77:393:96//Hs.30837:H08155
R-Y79AA1001145//ESTs//1.7e-13:285:64//Hs.128259:AA343015
R-Y79AA1001167
R-Y79AA1001177//EST//1.2e-05:92:76//Hs.65277:T15884
R-Y79AA1001185
R-Y79AA1001211//ESTs//1.3e-70:344:97//Hs.49760:AA741051
R-Y79AA1001216//ESTs//5.8e-63:416:88//Hs.8595:W60933
R-Y79AA1001228//ESTs//9.3e-101:483:98//Hs.13916:AI025750
R-Y79AA1001233//EST//0.00027:232:62//Hs.132431:AA909674
R-Y79AA1001236//Homo sapiens mRNA for JM23 protein, complete coding sequence (clone IMAGE 34581 and IMAGE 45355 and LLNLc110I133Q7 (RZPD Berlin))//1.1e-110:549:95//Hs.23170:AJ005892
R-Y79AA1001281//ESTs//3.6e-98:466:99//Hs.104442:AA481271
R-Y79AA1001299//Human Ini1 mRNA, complete cds//9.6e-25:133:100//Hs.155626:U04847
R-Y79AA1001312//ESTs//3.4e-92:454:97//Hs.127319:AI191149
R-Y79AA1001323//ESTs//1.6e-67:422:89//Hs.118559:AA887084
R-Y79AA1001384//ESTs//3.1e-104:496:98//Hs.153692:AA604143
R-Y79AA1001391//ESTs//2.2e-77:418:94//Hs.118608:AA101819
R-Y79AA1001394//ESTs//2.1e-78:409:95//Hs.23413:AA579859
R-Y79AA1001402//EST//9.3e-08:128:75//Hs.141607:N63891
R-Y79AA1001493//ESTs, Highly similar to UBIQUITIN-CONJUGATING ENZYME E2-17 KD 11 [Arabidopsis thaliana]//4.4e-109:553:95//Hs.106616:AI027524
R-Y79AA1001511//ESTs//4.9e-49:271:92//Hs.109045:AA523704
R-Y79AA1001533//ESTs, Moderately similar to RNA polymerase I associated factor [M.musculus]//6.2e-46:260:94//Hs.24884:AA176812
R-nnnnnnnnnnnn//EST//0.62:126:67//Hs.137020:AA868563
R-Y79AA1001548//PHOSPHATIDYLINOSITOL 4-KINASE ALPHA//3.5e-95:517:91//Hs.76987:AF012872
R-Y79AA1001555//Collagen, type XI, alpha 1//1.0:157:64//Hs.82772:J04177
R-Y79AA1001585//ESTs//1.9e-90:430:98//Hs.48333:AA704508
R-Y79AA1001594//ESTs//9.6e-23:122:100//Hs.63795:AI126237
R-Y79AA1001603//ESTs//1.0e-50:193:100//Hs.25635:AI336204
R-Y79AA1001613//ESTs, Weakly similar to zinc finger protein [H.sapiens]//7.2e-81:400:97//Hs.13323:AA897542
R-Y79AA1001647//ESTs//6.8e-92:479:95//Hs.154270:N26486
R-Y79AA1001665//ESTs, Weakly similar to 50S RIBOSOMAL PROTEIN L20 [E.coli]//2.5e-19:112:97//Hs.26252:AA643235
R-Y79AA1001679//ESTs, Highly similar to LAMBDA-CRYSTALLIN [Oryctolagus cuniculus]//9.7e-99:553:92//Hs.108896:R54040
R-nnnnnnnnnnnn
R-Y79AA1001696//ESTs//1.4e-84:478:91//Hs.6606:AA211783
R-Y79AA1001705//ESTs//6.7e-107:546:95//Hs.106805:AA418490
R-Y79AA1001711//Human DNA sequence from clone 1119D9 on chromosome 20p12. Contains part of a gene for a PAK1 LIKE Serine/Threonine-Protein Kinase and part of the PLCB4 gene for Phopholipase C, beta (1-Phosphatidylinositol -4,5-Bisphosphate Phosphodiesterase Beta 4). Contains ESTs, STSs and GSSs//0.0085:251:63//Hs.21864:AL031652
R-Y79AA1001781//ESTs, Weakly similar to partial CDS [C.elegans]//9.4e-87:427:97//Hs.18645:AI023798
R-nnnnnnnnnnnn//ESTs//1.1e-112:558:97//Hs.109755:AA180809
R-Y79AA1001827//ESTs, Weakly similar to Similar to S.cerevisiae YD9335.03c protein [H.sapiens]//8.1e-95:530:91//Hs.72444:W23217
R-Y79AA1001846//EST//2.8e-41:312:81//Hs.162236:AA551582
R-Y79AA1001848//Human adhalin (DAG2) mRNA, complete cds//0.54:221:58//Hs.99931:L34355
R-Y79AA1001866//ESTs//2.2e-102:498:97//Hs.130683:AI278630
R-Y79AA1001874//ESTs//1.9e-76:377:98//Hs.79707:AA354094
R-Y79AA1001875//ESTs//0.64:152:63//Hs.156159:AI333652
R-Y79AA1001923//EST//0.19:180:58//Hs.148290:AA908404
R-Y79AA1002027//ESTs//1.6e-104:497:98//Hs.21275:N73275
R-Y79AA1002083//Homo sapiens mRNA for KIAA0563 protein, complete cds//0.69:93:73//Hs.15731:AB011135
R-Y79AA1002089//Homo sapiens PYRJN (MEFV) mRNA, complete cds//1.1e-46:392:80//Hs.113283:AF018080
R-Y79AA1002093//Homo sapiens GT198 mRNA, complete ORF//1.2e-12:80:100//Hs.78185:L38933
R-Y79AA1002103//ESTs//1.3e-52:535:76//Hs.142167:AI417785
R-Y79AA1002115//ESTs//4.2e-101:519:96//Hs.23977:AA115275
R-Y79AA1002125//ESTs//9.8e-68:363:94//Hs.72085:AA193399
R-Y79AA1002139//ESTs//1.2e-100:498:96//Hs.72020:AA149858
R-Y79AA1002204//ESTs//2.1e-83:434:95//Hs.22979:R43725
R-nnnnnnnnnnnn//ESTs//1.7e-55:478:76//Hs.154554:AA552715
R-Y79AA1002209//ESTs, Weakly similar to similar to tyrosyl-tRNA synthetase. [C.elegans]//3.5e-108:553:95//Hs.50441:AA747428
R-Y79AA1002210//ESTs//4.2e-16:92:100//Hs.54862:AA248349
R-Y79AA1002211//ESTs, Weakly similar to PHOSPHATIDYLETHANOLAMINE-BINDING PROTEIN [H.sapiens]//6.5e-86:518:90//Hs.25682:AA857843
R-Y79AA1002220//EST//1.3e-68:326:100//Hs.131052:AI016274
R-Y79AA1002229//ESTs//1.9e-98:467:98//Hs.132002:AI039977
R-Y79AA1002234//Homo sapiens mRNA for KIAA0692 protein, partial cds//2.0e-118:564:98//Hs.100729:AB014592
R-Y79AA1002246//ESTs, Weakly similar to PROTEIN KINASE C, BRAIN ISOZYME [D.melanogaster]//9.0e-102:507:96//Hs.25895:AI341537
R-Y79AA1002258//Homo sapiens mRNA for KIAA0655 protein, partial cds//2.4e-93:453:97//Hs.96731:AB014555
R-Y79AA1002298//ESTs//0.022:241:62//Hs.118272:N90288
R-Y79AA1002307//Homo sapiens mRNA for KIAA0634 protein, partial cds//8.1e-110:403:99//Hs.30898:AB014534
R-Y79AA1002311//EST//2.6e-27:214:85//Hs.144721:AI187985
R-Y79AA1002351//ESTs//5.6e-100:489:97//Hs.30318:AA913371
R-Y79AA1002361
R-Y79AA1002399//ESTs//0.029:149:65//Hs.43872:N26908
R-Y79AA1002407//ESTs//2.8e-117:552:99//Hs.99519:AI042000
R-Y79AA1002416//ESTs//2.6e-107:531:96//Hs.6716:AA502753
R-Y79AA100243//EST//6.6e-23:128:98//Hs.128417:AA975026
R-nnnnnnnnnnnn//ESTs, Highly similar to CELL DIVISION CONTROL PROTEIN 68 [Saccharomyces cerevisiae]//4.4e-62:390:88//Hs.143930:AI207821
R-Y79AA1002472//ESTs//1.1e-39:234:78//Hs.117969:H94870
R-Y79AA1002482//ESTs//3.4e-45:312:85//Hs.146811:AA410788
R-Y79AA1002487//ESTs//1.7e-80:427:94//Hs.49210:N66499

### Homology Search Result Data 6

Data obtained by the homology search for full-length nucleotide sequences and deduced amino acid sequences. In the result of the search shown below, both units, aa and bp, are used as length units for the sequences to be compared. Each data includes Clone name, Definition in hit data, P value, Length of sequence to be compared, Homology, and Accession number (No.) of hit data. These items are shown in this order and separated by a double-slash mark, //.
C-HEMBA1000005//DNAJ PROTEIN HOMOLOG MTJ1.//1.9E-250//554aa//85%//Q61712
C-HEMBA1000030
C-HEMBA1000046
C-HEMBA1000050
C-HEMBA1000076
C-HEMBA1000156//NEUROFILAMENT TRIPLET M PROTEIN (160 KD NEUROFILAMENT PROTEIN) (NF-M).//1.9E-12//368aa//24%//P08553
C-HEMBA1000158//HEPATOCYTE NUCLEAR FACTOR 3.-GAMMA (HNF-3G).//5E-16//166aa//36%//P35584
C-HEMBA1000168//CYLICIN I (MULTIPLE-BAND POLYPEPTIDE I).//2.9E-14//303aa//25%//P35662
C-HEMBA1000185//RAS-RELATED PROTEIN RAL-A.//3.4E-12//125aa//31%//P48555
C-HEMBA1000193
C-HEMBA1000227
C-HEMBA1000288
C-HEMBA1000302
C-HEMBA1000304
C-HEMBA1000307//CARNITINE DEFICIENCY-ASSOCIATED PROTEIN EXPRESSED IN VENTRICLE 1//5.2E-49//107aa//91 %//035594
C-HEMBA1000369//Novel human mRNA similar to mouse gene PICK1 (TR:Q62083).//0//1950bp//98%//AL049654
C-HEMBA1000387
C-HEMBA1000392
C-HEMBA1000460
C-HEMBA1000488//RING CANAL PROTEIN (KELCH PROTEIN).//3.3E-45//481aa//29%//Q04652
C-HEMBA1000491//RAS-LIKE PROTEIN 2.//2E-22//188aa//31%//P22279
C-HEMBA1000501
C-HEMBA1000508
C-HEMBA1000520
C-HEMBA1000531//HEAT SHOCK 70 KD PROTEIN COGNATE 1 (HEAT SHOCK 70 KD PROTEIN 70C) (FRAGMENTS).//2.6E-12//73aa//41%//P02826
C-HEMBA1000534
C-HEMBA1000555
C-HEMBA1000568
C-HEMBA1000588
C-HEMBA1000608//HYPOTHETICAL PROTEIN KIAA0411 (FRAGMENT).//1.8E-55//179aa//61%//O43295
C-HEMBA1000636
C-HEMBA1000682
C-HEMBA1000686
C-HEMBA1000719
C-HEMBA1000727
C-HEMBA1000752
C-HEMBA1000817
C-HEMBA1000851
C-HEMBA1000867
C-HEMBA1000869
C-HEMBA1000872
C-HEMBA1000910//MELANOMA-ASSOCIATED ANTIGEN B1 (MAGE-B1 ANTIGEN) (MAGE-XP ANTIGEN)//1.6E-30//127aa//40%//P43366
C-HEMBA1000918
C-HEMBA1000919//HYPOTHETICAL 65.5 KD TRP-ASP REPEATS CONTAINING PROTEIN F02E8.5 IN CHROMOSOME X.//1E-10//288aa//23%//Q19124
C-HEMBA1000946
C-HEMBA1000968
C-HEMBA1000971
C-HEMBA1000975
C-HEMBA1001009
C-HEMBA1001022
C-HEMBA1001043//ANKYRIN, BRAIN VARIANT 2 (ANKYRIN B) (ANKYRIN, NONERYTHROID)(FRAGMENT).//1.4E-12//131aa//38%//Q01485
C-HEMBA1001052
C-HEMBA1001080
C-HEMBA1001085
C-HEMBA1001088//PINCH PROTEIN (PARTICULARY INTERESTING NEW CYS-HIS PROTEIN).//3.5E-50//176aa//57%//P48059
C-HEMBA1001109
C-HEMBA1001122
C-HEMBA1001133
C-HEMBA1001137//ZINC FINGER PROTEIN 33A (ZINC FINGER PROTEIN KOX31) (KIAA0065) (HA0946) (FRAGMENT).//1.5E-116//197aa//58%//Q06730
C-HEMBA1001140
C-HEMBA1001174//ADP-RIBOSYLATION FACTOR-LIKE PROTEIN 5.//6.8E-79//179aa//80%//P51646
C-HEMBA1001197//Homo sapiens mRNA for KIAA0871 protein, complete cds.//9.5E-257//1307bp//94%//AB020678
C-HEMBA1001235
C-HEMBA1001257//Homo sapiens mRNA 2-methylacyl-CoA racemase.//0//1672bp//99%//AJ130733
C-HEMBA1001281
C-HEMBA1001286//COMPLEMENT DECAY-ACCELERATING FACTOR PRECURSOR.//0.00000002//198aa//29%//Q60401
C-HEMBA1001303
C-HEMBA1001310
C-HEMBA1001326
C-HEMBA1001351//Homo sapiens VAMP-associated protein of 33 kDa (VAP-33) mRNA, complete cds.//1.4E-133//614bp//99%//AF057358
C-HEMBA1001387//GTP-BINDING PROTEIN TC10.//2.9E-64//104aa//82%//P17081
C-HEMBA1001388
C-HEMBA1001398
C-HEMBA1001405
C-HEMBA1001407
C-HEMBA1001413
C-HEMBA1001415
C-HEMBA1001446
C-HEMBA1001450
C-HEMBA1001455
C-HEMBA1001510//CYCLIC-AMP-DEPENDENT TRANSCRIPTION FACTOR ATF-6 (FRAGMENT).//1.7E-16//63aa//61%//P18850
C-HEMBA1001526//PERIPLASMIC [FE] HYDROGENASE 1 (EC 1.18.99.1).//4.9E-37//399aa//29%//P29166
C-HEMBA1001533
C-HEMBA1001579//Homo sapiens mRNA for KIAA0850 protein, complete cds.//0//1662bp//99%//AB020657
C-HEMBA1001581
C-HEMBA1001595//SEPTIN 2 HOMOLOG (FRAGMENT).//4.9E-156//348aa//83%//Q14141
C-HEMBA1001635//TESTIS SPECIFIC PROTEIN A (ZINC FINGER PROTEIN TSGA).//1.6E-10//155aa//28%//Q63679
C-HEMBA1001661//CADHERIN-RELATED TUMOR SUPPRESSOR PRECURSOR (FAT PROTEIN).//4.6E-36//365aa//33%//P33450
C-HEMBA1001702
C-HEMBA1001714//Homo sapiens mRNA; cDNA DKFZp564G0422 (from clone DKFZp564G0422).//0//1845bp//99%//AL050386
C-HEMBA1001731
C-HEMBA1001744//SCY1PROTEIN.//9.9E-32//481aa//25%//P53009
C-HEMBA1001809//IMMEDIATE-EARLY PROTEIN IE180.//3.8E-11//206aa//36%//P11675
C-HEMBA1001815
C-HEMBA1001819//ZINC FINGER PROTEIN 184 (FRAGMENT).//2.9E-135//459aa//52%//Q99676
C-HEMBA1001847//ZINC FINGER PROTEIN 29 (ZFP-29).//7.6E-64//221aa//55%//Q07230
C-HEMBA1001864
C-HEMBA1001869//TRITHORAX PROTEIN.//0.000096//166aa//27%//P20659
C-HEMBA1001896//DIMETHYLGLYCINE DEHYDROGENASE PRECURSOR (EC 1.5.99.2) (ME2GLYDH).//9.3E-36//395aa//26%//Q63342
C-HEMBA1001987
C-HEMBA1002018
C-HEMBA1002049
C-HEMBA1002084
C-HEMBA1002125
C-HEMBA1002161//MYOSIN HEAVY CHAIN, CARDIAC MUSCLE BETA ISOFORM.//1.4E-51//180aa//56%//P79293
C-HEMBA1002177//TRANSCRIPTION FACTOR GATA-4 (GATA BINDING FACTOR-4).//6E-13//190aa//36%//P43694
C-HEMBA1002191
C-HEMBA1002199
C-HEMBA1002212//TYROSINE-PROTEIN KINASE 2 (EC 2.7.1.112) (FRAGMENT).//3E-17//267aa//29%//P18161
C-HEMBA1002237
C-HEMBA1002265
C-HEMBA1002267//Sus scrofa decorin mRNA, complete cds.//1.1E-46//302bp//90%//AF125537
C-HEMBA1002349
C-HEMBA1002363//Homo sapiens chromosome-associated protein-E (hCAP-E) mRNA, complete cds.//0//1847bp//99%//AF092563
C-HEMBA1002419//TRICHOHYALIN.//1.9E-09//299aa//24%//P22793
C-HEMBA1002430
C-HEMBA1002439
C-HEMBA1002458//OVARIAN GRANULOSA CELL 13.0 KD PROTEIN HGR74.//4.2E-24//109aa//55%//Q00994
C-HEMBA1002460
C-HEMBA1002462
C-HEMBA1002469//DXS8237E PROTEIN (FRAGMENT).//3.5E-50//199aa//61%//P98175
C-HEMBA1002475//SKIN SECRETORY PROTEIN XP2 PRECURSOR (APEG PROTEIN).//1.1E-12//285aa//31%//P17437
C-HEMBA1002477
C-HEMBA1002495//LIGHT-MEDIATED DEVELOPMENT PROTEIN DET1.//6.8E-53//257aa//36%//P48732
C-HEMBA1002515
C-HEMBA1002542
C-HEMBA1002569//Homo sapiens protein associated with Myc mRNA, complete cds.//6.8E-305//951bp//99%//AF075587
C-HEMBA1002583
C-HEMBA1002609//Homo sapiens mRNA for KIAA0597 protein, partial cds.//1.4E-253//1149bp//99%//AB011169
C-HEMBA1002624//Homo sapiens mRNA for KIAA0808 protein, complete cds.//0//1539bp//99%//AB018351
C-HEMBA1002688
C-HEMBA1002696
C-HEMBA1002750
C-HEMBA1002768//Homo sapiens mRNA for Cdc42-interacting protein 4 (CIP4).//1E-80//882bp//61%//AJ000414
C-HEMBA1002770//Homo sapiens mRNA for KIAA0829 protein, partial cds.//0//1532bp//99%//AB020636
C-HEMBA1002777
C-HEMBA1002794
C-HEMBA1002810//Homo sapiens formin binding protein 21 mRNA, complete cds.//8.2e-314//1437bp//99%//AF071185
C-HEMBA1002818//Homo sapiens, mRNA for fibulin-4.//2E-304//1383bp//99%//AJ132819
C-HEMBA1002850
C-HEMBA1002863
C-HEMBA1002876//HYPOTHETICAL 26.4 KD PROTEIN EEED8.8 IN CHROMOSOME II.//1.5E-44//188aa//52%//Q09297
C-HEMBA1002935//Homo sapiens mRNA for KIAA0576 protein, partial cds.//0//1483bp//100%//AB011148
C-HEMBA1002937
C-HEMBA1002939//ANKYRIN R (ANKYRINS 2.1 AND 2.2) (ERYTHROCYTE ANKYRIN).//2E-34//300aa//34%//P16157
C-HEMBA1002951//Homo sapiens mRNA for KIAA0903 protein, partial cds.//0//1752bp//99%//AB020710
C-HEMBA1002954
C-HEMBA1002971
C-HEMBA1002973//CAMP-DEPENDENT 3',5'-CYCLIC PHOSPHODIESTERASE 4B (EC 3.1.4.17) (DPDE4).//1.2E-27//63aa//100%//P14646
C-HEMBA1002997//CENTROMERIC PROTEIN E (CENP-E PROTEIN).//3.8E-25//5 34aa//24%//Q02224
C-HEMBA1003033
C-HEMBA1003035
C-HEMBA1003041
C-HEMBA1003046//MITOCHONDRIAL PROCESSING PROTEASE BETA SUBUNIT PRECURSOR (EC 3.4.24.64) (BETA-MPP) (P-52).//2.5E-263//489aa//99%//O75439
C-HEMBA1003067
C-HEMBA1003096
C-HEMBA1003117
C-HEMBA1003129
C-HEMBA1003136//MANNOSE-1-PHOSPHATE GUANYLTRANSFERASE (EC 2.7.7.13) (ATP-MANNOSE-1-PHOSPHATE GUANYLYLTRANSFERASE) (NDP-HEXOSE PYROPHOSPHORYLASE).//8.5E-51//221aa//33%//P41940
C-HEMBA1003148//Homo sapiens mRNA full-length insert cDNA clone EUROIMAGE 381801.//0//1583bp//99%//AL079278
C-HEMBA1003175
C-HEMBA1003179//PROBABLE TRNA (5-METHYLAMINOMETHYL-2-THIOURIDYLATE)-METHYLTRANSFERASE (EC 2.1.1.61).//5.9E-74//134aa//53%//P44551
C-HEMBA1003199
C-HEMBA1003222
C-HEMBA1003235//TROPOMYOSIN.//0.0000023//109aa//33%//Q02088
C-HEMBA1003250//PROTEIN KINASE APK1A (EC 2.7.1.-).//7.2E-41//245aa//42%//Q06548
C-HEMBA1003257
C-HEMBA1003281//POLIOVIRUS RECEPTOR PRECURSOR.//6E-11//239aa//32%//P32506
C-HEMBA1003286//Homo sapiens mRNA for beta-1,4-galactosyltransferase IV, complete cds.//5.4E-229//1043bp//99%//AB024436
C-HEMBA1003291//Homo sapiens mRNA for KIAA0537 protein, complete cds.//0//791bp//99%//AB011109
C-HEMBA1003322
C-HEMBA1003327
C-HEMBA1003369//CENTROMERIC PROTEIN E (CENP-E PROTEIN).//0.00000002//248aa//23%//Q02224
C-HEMBA1003370
C-HEMBA1003380
C-HEMBA1003395
C-HEMBA1003402
C-HEMBA1003408//Homo sapiens mRNA for KIAA0905 protein, complete cds.//0//1732bp//98%//AB020712
C-HEMBA1003417//Homo sapiens mRNA; cDNA DKFZp586C021 (from clone DKFZp586C021).//1.6e-312//1414bp//99%//AL050287
C-HEMBA1003418//TRICHOHYALIN.//8.7E-19//281aa//31%//P37709
C-HEMBA1003433//Homo sapiens gene for NBS1, complete cds.//0//511bp//94%//AB013139
C-HEMBA1003447
C-HEMBA1003461
C-HEMBA1003463
C-HEMBA1003528
C-HEMBA1003545//INSULIN GENE ENHANCER PROTEIN ISL-2 (ISLET-2).//8.8E-189//360aa//96%//P50480
C-HEMBA1003555//NUCLEOTIDE-BINDING PROTEIN (NBP).//2.1E-68//251aa//52%//P53384
C-HEMBA1003560//GUANINE NUCLEOTIDE-BINDING PROTEIN G(I)/G(S)/G(O) GAMMA-2 SUBUNIT (G GAMMA-I).//1.2E-31//71aa//100%//P16874
C-HEMBA1003568//52 KD RO PROTEIN (SJOGREN SYNDROME TYPE A ANTIGEN (SS-A)) (RO(SS-A)).//7.9E-49//279aa//32%//P19474
C-HEMBA1003569//METASTASIS-ASSOCIATED PROTEIN MTA1.//6.9E-206//445aa//74%//Q13330
C-HEMBA1003581//TALIN.//4.4E-45//52aa//98%//P26039
C-HEMBA1003591//CHLOROPLAST 28 KD RIBONUCLEOPROTEIN PRECURSOR (28RNP).//4.4E-10//118aa//35%//P19682
C-HEMBA1003615
C-HEMBA1003617//Homo sapiens HRIHFB2157 mRNA, partial cds.//8.2E-178//501bp//97%//AB015344
C-HEMBA1003621
C-HEMBA1003662//TBX2 PROTEIN (T-BOX PROTEIN 2).//1.2E-75//151aa//99%//Q13207
C-HEMBA1003690//HISTONE DEACETYLASE HDA1.//2.1E-59//249aa//47%//P53973
C-HEMBA1003711
C-HEMBA1003807
C-HEMBA1003864
C-HEMBA1003953//ZINC FINGER PROTEIN MFG-1 (ZINC FINGER PROTEIN 58) (FRAGMENT).//3.8E-16//89aa//46%//P16372
C-HEMBA1003959
C-HEMBA1003989
C-HEMBA1004074
C-HEMBA1004097//Mus musculus putative transcription factor mRNA, complete cds.//8.5E-221//1188bp//78%//AF091234
C-HEMBA1004146
C-HEMBA1004199//Homo sapiens mRNA for KIAA0928 protein, partial cds.//0//1893bp//98%//AB023145
C-HEMBA1004207//Homo sapiens leptin receptor short form (db) mRNA, complete cds.//0//1892bp//99%//U50748
C-HEMBA1004227//Rattus norvegicus protein phosphatase 2C mRNA, complete cds.//5.7E-217//1217bp//88%//AF095927
C-HEMBA1004246
C-HEMBA1004276//Homo sapiens AP-4 adaptor complex beta4 subunit mRNA, complete cds.//4.8E-257//738bp//99%//AF092094
C-HEMBA1004289
C-HEMBA1004509//Homo sapiens CGI-21 protein mRNA, complete cds.//0//1512bp//96%//AF132955
C-HEMBA1004534//Homo sapiens gamma-filamin (ABPL) mRNA, complete cds.//1.2e-316//1445bp//99%//AF089841
C-HEMBA1004596
C-HEMBA1004693
C-HEMBA1004736
C-HEMBA1004753
C-HEMBA1004756//Human transporter protein (g17) mRNA, complete cds.//9.1E-34//515bp//66%//U49082
C-HEMBA1004758//Homo sapiens transcription factor SL1 mRNA, complete cds.//2.6E-246//1249bp//94%//L39060
C-HEMBA1004763
C-HEMBA1004768//LINE-1 REVERSE TRANSCRIPTASE HOMOLOG.//5.4E-111//314aa//58%//P08547
C-HEMBA1004771
C-HEMBA1004776
C-HEMBA1004795//CDC4-LIKE PROTEIN (FRAGMENT).//3.8E-69//198aa//66%//P50851
C-HEMBA1004806
C-HEMBA1004847//SIGNAL RECOGNITION PARTICLE 68 KD PROTEIN (SRP68).//8.2E-154//317aa//94%//Q00004
C-HEMBA1004850
C-HEMBA1004863//Homo sapiens mRNA; cDNA DKFZp586M2022 (from clone DKFZp586M2022).//0//1443bp//100%//AL080114
C-HEMBA1004923
C-HEMBA1004929
C-HEMBA1004930//26S PROTEASOME SUBUNIT S5B (KIAA0072) (HA1357).//3.3E-27//65aa//100%//Q16401
C-HEMBA1004933
C-HEMBA1004954
C-HEMBA1004972//NEUROFILAMENT TRIPLET H PROTEIN (200 KD NEUROFILAMENT PROTEIN) (NF-H).//0.00000096//286aa//23%//P12036
C-HEMBA1005475
C-HEMBA1005581//Homo sapiens SLIT2 (SUL2) mRNA, complete cds.//0//1721bp//100%//AF133270
C-HEMBA1006248//ZINC FINGER PROTEIN MFG-1 (ZINC FINGER PROTEIN 58) (FRAGMENT).//8.6E-23//151aa//37%//P16372
C-HEMBA1006310//Rattus norvegicus cytosolic sorting protein PACS-1a (PACS-1) mRNA, complete cds.//3.7E-225//1189bp//88%//AF076183
C-HEMBA1006344//RADIXIN.//1.5E-31//333aa//28%//P26043
C-HEMBA1006377
C-HEMBA1006467
C-HEMBA1006474//40 KD PROTEIN.//1.4E-39//292aa//34%//Q01552
C-HEMBA1006530
C-HEMBA1006737//ANKYRIN, BRAIN VARIANT 2 (ANKYRIN B) (ANKYRIN, NONERYTHROID) (FRAGMENT).//0.000000043//111aa//40%//Q01485
C-HEMBA1006795
C-HEMBA1006877//OXYSTEROL-BINDING PROTEIN.//2E-59//378aa//39%//P16258
C-HEMBA1006936
C-HEMBA1007018//Homo sapiens dynein light chain-A mRNA, complete cds.//1.5E-267//1215bp//99%//AP078849
C-HEMBA1007342
C-HEMBB1000008
C-HEMBB1000018
C-HEMBB1000024
C-HEMBB1000025
C-HEMBB1000036
C-HEMBB1000037//Homo sapiens erythroblast macrophage protein EMP mRNA, complete cds.//2.8E-187//1582bp//80%//AF084928
C-HEMBB1000083//MYOSIN LIGHT CHAIN KINASE, SMOOTH MUSCLE AND NON-MUSCLE ISOZYMES (EC 2.7.1.117) (MLCK) [CONTAINS: TELOKIN].//1.9E-22//426aa//25%//P11799
C-HEMBB1000103
C-HEMBB1000119//Homo sapiens ASMTL gene.//0//1891bp//99%//Y15521
C-HEMBB1000136
C-HEMBB1000215
C-HEMBB1000226//PUTATIVE PRE-MRNA SPLICING FACTOR ATP-DEPENDENT RNA HELICASE EEED8.5.//2.7E-12//112aa//47%//Q09530
C-HEMBB1000244
C-HEMBB1000266//HYPOTHETICAL 54.5 KD TRP-ASP REPEATS CONTAINING PROTEIN ZC302.2 IN CHROMOSOME V.//6.1E-09//242aa//26%//Q23256
C-HEMBB1000338
C-HEMBB1000339
C-HEMBB1000391
C-HEMBB1000438
C-HEMBB1000449
C-HEMBB1000589
C-HEMBB1000591
C-HEMBB1000623
C-HEMBB1000630
C-HEMBB1000631//LONGEVITY-ASSURANCE PROTEIN 1 (LONGEVITY ASSURANCE FACTOR 1).//4.1E-19//232aa//28%//P78970
C-HEMBB1000632//GUANINE NUCLEOTIDE RELEASING PROTEIN (GNRP).//2.2E-28//273aa//31%//P27671
C-HEMBB1000671
C-HEMBB1000673
C-HEMBB1000705
C-HEMBB1000706
C-HEMBB1000725//Rattus norvegicus GTPase Rab8b (Rab8b) mRNA, complete cds.//6.2E-130//692bp//93%//U53475
C-HEMBB1000763//Homo sapiens CGI-89 protein mRNA, complete cds.//0//1676bp//96%//AF151847
C-HEMBB1000781//Homo sapiens mitogen-activated protein kinase kinase kinase MEKK2 mRNA, complete cds.//1.2E-126//613bp//97%//AF111105
C-HEMBB1000789//PUTATIVE 90.2 KD ZINC FINGER PROTEIN IN CCA1-ADK2 INTERGENIC REGION.//5.1E-54//232aa//43%//P39956
C-HEMBB1000807
C-HEMBB1000810
C-HEMBB1000848
C-HEMBB1000852
C-HEMBB1000870
C-HEMBB1000887
C-HEMBB1000908
C-HEMBB1000927//Homo sapiens calsenilin mRNA, complete cds.//1.1E-70//595bp//76%//AF120102
C-HEMBB1000947//Homo sapiens clone HAW 100 putative ribonuclease III mRNA, complete cds.//0//2292bp//99%//AF116910
C-HEMBB1000973//Mus musculus schlafen3 (Slfn3) mRNA, complete cds.//3.4E-120//580bp//67%//AF099974
C-HEMBB1000975
C-HEMBB1000985//MEPP PROTEIN (MURINE IAP-PROMOTED PLACENTA-EXPRESSED PROTEIN).//8.6E-18//178aa//30%//P28575
C-HEMBB1000991
C-HEMBB1001011//ZINC FINGER PROTEIN 84 (ZINC FINGER PROTEIN HPF2).//1.4E-73//230aa//45%//P51523
C-HEMBB1001014
C-HEMBB1001024
C-HEMBB1001056//PROLIFERATING-CELL NUCLEOLAR ANTIGEN P120 (PROLIFERATION-ASSOCIATED NUCLEOLAR PROTEIN P120).//2.9E-19//264aa//34%//P46087
C-HEMBB1001058//Homo sapiens neuronal thread protein AD7c-NTP mRNA, complete cds//3.6E-52//331bp//80%//AF010144
C-HEMBB1001068//Homo sapiens liprin-beta2 mRNA, partial cds.//2.4E-307//1447bp//97%//AF034803
C-HEMBB1001096
C-HEMBB1001105
C-HEMBB1001117
C-HEMBB1001126
C-HEMBB1001137//Homo sapiens mRNA for putative phospholipase, complete cds.//0//3069bp//99%//AB019435
C-HEMBB1001151//Rattus norvegicus golgi stacking protein homolog GRASP55 mRNA, complete cds.//4.2E-210//1835bp//76%//AF110267
C-HEMBB1001153
C-HEMBB1001169
C-HEMBB1001175//ANKYRIN.//6.9E-11//169aa//31%//Q02357
C-HEMBB1001182
C-HEMBB1001199
C-HEMBB1001210//Homo sapiens mRNA for KIAA0970 protein, complete cds.//0//1816bp//99%//AB023187
C-HEMBB1001242//Homo sapiens topoisomerase-related function protein (TRF4-2) mRNA, partial cds.//1.8E-284//713bp//100%//AF089897
C-HEMBB1001288//Homo sapiens CGI-32 protein mRNA, complete cds.//1.8E-274//642bp//99%//AF132966
C-HEMBB1001289
C-HEMBB1001294//GTP-BINDING PROTEIN TC10.//1.2E-79//196aa//80%//P17081
C-HEMBB1001314//Mus musculus Olf-1/EBF-like-3 transcription factor (O/E-3) mRNA, complete cds.//1.3E-129//724bp//86%//U92703
C-HEMBB1001331
C-HEMBB1001339//DXS8237E PROTEIN (FRAGMENT).//0.0000046//124aa//37%//P98175
C-HEMBB1001346//Homo sapiens phenylalanine-tRNA synthetase (FARS1) mRNA, nuclear gene encoding mitochondrial protein, complete cds.//1.1E-58//292bp//99%//AF097441
C-HEMBB1001369
C-HEMBB1001384//Homo sapiens COP9 complex subunit 4 mRNA, complete cds.//0//1586bp//99%//AF100757
C-HEMBB1001387
C-MAMMA1002317
C-MAMMA1002319
C-MAMMA1002385//RIBONUCLEOPROTEIN RB97D.//0.00000015//206aa//29%//Q02926
C-NT2RM1000080//UNC-1 PROTEIN.//5.9E-25//211aa//31%//Q21190
C-NT2RM1000242
C-NT2RM1000257//MAGO NASHI PROTEIN.//7.9E-69//143aa//91%//P49028
C-NT2RM1000280//VACUOLAR ATP SYNTHASE SUBUNIT D (EC 3.6.1.34) (V-ATPASE D SUBUNIT) (V- ATPASE 28 KD ACCESSORY PROTEIN).//1.5E-106//118aa//97%//P39942
C-NT2RM1000669
C-NT2RM1000781
C-NT2RM1000867//Homo sapiens HSPC033 mRNA, complete cds.//6.3E-172//798bp//99%//AF092138
C-NT2RM1001008
C-NT2RM1001044//Homo sapiens HSPC031 mRNA, complete cds.//0.000000002//980bp//95%//AF085360
C-NT2RM1001074
C-NT2RM1001115//ENDOCHITINASE 2 PRECURSOR (EC 3.2.1.14).//0.0000056//239aa//27%//
C-NT2RM2000006//Human DNA sequence from clone 796F18 on chromosome 1p36.11-36.33 Contains a pseudogene similar to MMS2, ESTs and GSSs, complete sequence.//0//1740bp//99%//AL031291
C-NT2RM2000013//DNA-DIRECTED RNA POLYMERASE III 128 KD POLYPEPTIDE (EC 2.7.7.6) (RNA POLYMERASE III SUBUNIT 2).//2.2E-144//362aa//71%//P25167
C-NT2RM2000030//DYNEIN INTERMEDIATE CHAIN, CYTOSOLIC (DH IC) (CYTOPLASMIC DYNEIN INTERMEDIATE CHAIN).//0.00000043//136aa//31%//P54703
C-NT2RM2000032
C-NT2RM2000042
C-NT2RM2000092//UBIQUITIN CARBOXYL-TERMINAL HYDROLASE 8 (EC 3.1.2.15) (UBIQUTTIN THIOLESTERASE 8) (UBIQUITIN-SPECIFIC PROCESSING PROTEASE 8) (DEUBIQUITINATING ENZYME 8).//1.3E-36//160aa//40%//P50102
C-NT2RM2000093
C-NT2RM2000101
C-NT2RM2000191//Homo sapiens cGMP phosphodiesterase A1 (PDE9A) mRNA, complete cds.//0//1574bp//99%//AF067223
C-NT2RM2000192
C-NT2RM2000239
C-NT2RM2000250//Homo sapiens mRNA; cDNA DKFZp564L232 (from clone DKFZp564L232).//4.2e-314//1416bp//100%//AL080069
C-NT2RM2000259
C-NT2RM2000260//EXTENSIN PRECURSOR (PROLINE-RICH GLYCOPROTEIN).//3.6E-19//181-aa//34%//P14918
C-NT2RM2000287
C-NT2RM2000322//Homo sapiens mRNA for KIAA0859 protein, complete cds.//3.4E-294//863bp//99%//AB020666
C-NT2RM2000359//Homo sapiens mRNA for KIAA0560 protein, complete cds.//0//1637bp//99%//AB011132
C-NT2RM2000363//BREAKPOINT CLUSTER REGION PROTEIN.//1.8E-14//245aa//29%//P11274
C-NT2RM2000368//Homo sapiens protein kinase C-binding protein RACK7 mRNA, partial cds.//0//1506bp//99%//U48251
C-NT2RM2000371//POLYRIBONUCLEOTIDE NUCLEOTIDYLTRANSFERASE (EC 2.7.7.8) (POLYNUCLEOTIDE//1.7E-68//419aa//36%//P50849
C-NT2RM2000374
C-NT2RM2000395
C-NT2RM2000402//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//1.6E-54//344aa//33%//P32802
C-NT2RM2000407
C-NT2RM2000422//SODIUM- AND CHLORIDE-DEPENDENT TRANSPORTER NTT73.//1E-222//237aa//89%//Q08469
C-NT2RM2000452//HYPOTHETICAL 63.6 KD PROTEIN IN YPT52-GCN3 INTERGENIC REGION.//0.0000001//157aa//28%//P36113
C-NT2RM2000469//NITROGEN PERMEASE REACTIVATOR PROTEIN (EC 2.7.1.-).//0.0000089//377aa//24%//P22211
C-NT2RM2000490//SYNAPTOTAGMIN(P65).//1.8E-13//166aa//34%//P41823
C-NT2RM2000502
C-NT2RM2000504//Homo sapiens metalloprotease 1 (MP1) mRNA, complete cds.//0//1673bp//99%//AF061243
C-NT2RM2000522//SKIN SECRETORY PROTEIN XP2 PRECURSOR (APEG PROTEIN).//1.3E-12//282aa//32%//P17437
C-NT2RM2000540
C-NT2RM2000567
C-NT2RM2000569
C-NT2RM2000577//ISOLEUCYL-TRNA SYNTHETASE (EC 6.1.1.5) (ISOLEUCINE-TRNA LIGASE) (ILERS).//1.7E-187//741aa//46%//P73505
C-NT2RM2000581//Homo sapiens mRNA for KIAA0214 protein, complete cds.//0//3001bp//99%//D86987
C-NT2RM2000588//HISTONE DEACETYLASE HDA1.//2.8E-60//384aa//40%//P53973
C-NT2RM2000594//Homo sapiens DNA cytosine-5 methyltransferase 3 beta 3 (DNMT3B) mRNA, complete cds.//0//2712bp//99%//AF156487
C-NT2RM2000599//Homo sapiens F-box protein Lilina (LILINA) mRNA, complete cds.//4.9E-70//838bp//69%//AF179221
C-NT2RM2000624//SPLICING FACTOR, ARGININE/SERINE-RICH 4 (PRE-MRNA SPLICING FACTOR SRP75).//4.4E-32//319aa//35%//Q08170
C-NT2RM2000635//Homo sapiens mRNA for KIAA0729 protein, partial cds.//0//3791bp//99%//AB018272
C-NT2RM2000636//Homo sapiens mRNA for KIAA0658 protein, partial cds.//0//2530bp//99%//AB014558
C-NT2RM2000639
C-NT2RM2000649//Homo sapiens mRNA for KIAA0676 protein, partial cds.//0//1543bp//99%//AB014576
C-NT2RM2000669
C-NT2RM2000691//ACTIN-LIKE PROTEIN 3 (ACTIN-2).//3.7E-142//285aa//90%//P32391
C-NT2RM2000714//RAS SUPPRESSOR PROTEIN 1 (RSU-1) (RSP-1 PROTEIN) (RSP1).//3.8E-23//184aa//36%//Q15404
C-NT2RM2000718//Homo sapiens HRIHFB2436 mRNA, partial cds.//4.4E-231//1065bp//99%//AB015342
C-NT2RM2000740//POSSIBLE GLOBAL TRANSCRIPTION ACTIVATOR SNF2L.//5.7E-53//266aa//43%//P41877
C-NT2RM2000795
C-NT2RM2000821//COATOMER BETA SUBUNIT (BETA-COAT PROTEIN) (BETACOP).//9.5E-279//545aa//98%//P23514
C-NT2RM2000837
C-NT2RM2000951//Homo sapiens XYLB mRNA for xylulokinase, complete cds.//1.7E-200//927bp//99%//AB015046
C-NT2RM2000952
C-NT2RM2000984
C-NT2RM2001004
C-NT2RM2001035//CCR4-ASSOCIATED FACTOR 1 (CAF1).//8.2E-154//285aa//99%//Q60809
C-NT2RM2001065
C-NT2RM2001100//HYPOTHETICAL 39.7 KD PROTEIN C34E10.2 IN CHROMOSOME III.//2.4E-15//266aa//26%//P46577
C-NT2RM2001131
C-NT2RM2001141
C-NT2RM2001152
C-NT2RM2001177//Homo sapiens mRNA; cDNA DKFZp586G1822 (from clone DKFZp586G1822).//2.1E-293//1335bp//99%//AL080109
C-NT2RM2001194
C-NT2RM2001196//PROLINE-RICH PROTEIN MP-3 (FRAGMENT).//1.3E-20//267aa//35%//P05143
C-NT2RM2001201//EUKARYOTIC TRANSLATION INITIATION FACTOR 5 (EIF5).//0.00000015//95aa//35%//P48724
C-NT2RM2001221//KALIRIN (PAM COOH-TERMINAL INTERACTOR PROTEIN 10) (PCIP10).//3.6E-10//177aa//32%//P97924
C-NT2RM2001238//GLUTAMINASE, KIDNEY ISOFORM PRECURSOR (EC 3.5.1.2) (GLS) (L-GLUTAMINE AMIDOHYDROLASE).//1.3E-180//328aa//99%//P13264
C-NT2RM2001243
C-NT2RM2001247
C-NT2RM2001256//PROTEIN TSG24 (MEIOTIC CHECK POINT REGULATOR).//1.6E-166//312aa//98%//P53995
C-NT2RM2001291
C-NT2RM2001306//Homo sapiens mRNA; cDNA DKFZp564I052 (from clone DKFZp564I052).//0//1694bp//99%//AL080063
C-NT2RM2001312
C-NT2RM2001319
C-NT2RM2001324//ZYXIN.//6.8E-55//200aa//41%//Q04584
C-NT2RM2001345//VEGETATIBLE INCOMPATIBILITY PROTEIN HET-E1.//0.000000029//334aa//22%//000808
C-NT2RM2001370
C-NT2RM2001393
C-NT2RM2001420
C-NT2RM2001424//Homo sapiens mRNA; cDNA DKFZp586D0920 (from clone DKFZp586D0920).//0//1621bp//100%//AL050146
C-NT2RM2001499//LOW-AFFINITY CATIONIC AMINO ACID TRANSPORTER-2 (CAT-2) (CAT2).//7.4E-121//437aa//57%//P52569
C-NT2RM2001504
C-NT2RM2001524
C-NT2RM2001544
C-NT2RM2001547//PROBABLE PROTEIN DISULFIDE ISOMERASE P5 PRECURSOR (EC 5.3.4.1).//6.9E-27//90aa//42%//P38660
C-NT2RM2001575//52 KD RO PROTEIN (SJOGREN SYNDROME TYPE A ANTIGEN (SSA)) (RO(SS-A)).//4.3E-61//312aa//44%//P19474
C-NT2RM2001582
C-NT2RM2001886//Homo sapiens mRNA for KIAA0710 protein, complete cds.//0//1000bp//100%//AB014610
C-NT2RM2001896//CELL DIVISION PROTEIN FTSJ.//5.1E-26//204aa//34%//P28692
C-NT2RM2001903//Homo sapiens mRNA for KIAA0462 protein, partial cds.//0//2390bp//99%//AB007931
C-NT2RM2001930
C-NT2RM2001935
C-NT2RM2001936//32.3 KD PROTEIN IN CWP1-MBR1 INTERGENIC REGION.//2.7E-27//216aa//34%//P28320
C-NT2RM2001950//HYPOTHETICAL 105.9 KD PROTEIN IN AAC3-RFC5 INTERGENIC REGION.//0.0000001//212aa//23%//P38250
C-NT2RM2001982
C-NT2RM2001989//NUCLEOLAR PROTEIN NOP4 (NUCLEOLAR PROTEIN NOP77).//1.9E-39//253aa//35%//P37838
C-NT2RM2001997//PROTEIN DISULFIDE ISOMERASE PRECURSOR (PDI) (EC 5.3.4.1).//1.3E-10//232aa//28%//Q12730
C-NT2RM2001998//HYPOTHETICAL 85.7 KD PROTEIN C13G6.03 IN CHROMOSOME I.//3.1E-12//206aa//30%//Q09782
C-NT2RM2002004//LA PROTEIN HOMOLOG (LA RIBONUCLEOPROTEIN) (LA AUTOANTIGEN HOMOLOG).//0.000000029//83aa//44%//P40796
C-NT2RM2002014//HYPOTHETICAL 81.4 KD PROTEIN IN GREB-FEOA INTERGENIC REGION.//1.1E-89//425aa//41%//P46837
C-NT2RM2002030//Homo sapiens mRNA for Glutamine:fructose-6-phosphate amidotransferase, complete cds.//0//1959bp//99%//AB016789
C-NT2RM2002049
C-NT2RM2002055//VACUOLAR PROTEIN SORTING-ASSOCIATED PROTEIN VPS13.//0.00000099//338aa//24%//Q07878
C-NT2RM2002088//PUTATIVE HETEROGENEOUS NUCLEAR RIBONUCLEOPROTEIN X (HNRNP X) (CBP).//5E-62//104aa//57%//Q61990
C-NT2RM2002091
C-NT2RM2002100//Homo sapiens mRNA for ATP-dependent RNA helicase, partial.//0//1807bp//99%//AJ010840
C-NT2RM2002109//Homo sapiens glioma amplified on chromosome 1 protein (GAC1) mRNA, complete cds.//0//1868bp//99%//AF030435
C-NT2RM2002128//PUTATIVE SERINE/THREONINE-PROTEIN KINASE PKWA (EC 2.7.1.-).//4.9E-13//487aa//26%//P49695
C-NT2RM2002142//GASTRULATION SPECIFIC PROTEIN G12.//8E-31//105aa//47%//P47805
C-NT2RM2002178//Homo sapiens mRNA; cDNA DKFZp434E0335 (from clone DKFZp434E0335).//0//1683bp//99%//AL117402
C-NT2RM4000024//DNA-DIRECTED RNA POLYMERASE III 128 KD POLYPEPTIDE (EC 2.7.7.6) (RNA POLYMERASE III SUBUNIT 2).//7.1E-155//381aa//72%//P25167
C-NT2RM4000061
C-NT2RM4000104//ZINC FINGER PROTEIN 135.//1.5E-81//251aa//53%//P52742
C-NT2RM4000139//R.norvegicus trg mRNA.//2.3E-114//1161bp//72%//X68101
C-NT2RM4000169//INTRACELLULAR PROTEIN TRANSPORT PROTEIN USO1.//4.8E-13//686aa//23%//P25386
C-NT2RM4000191//PUTATIVE ATP-DEPENDENT RNA HELICASE PL10.//9.2E-75//439aa//41%//P16381
C-NT2RM4000197
C-NT2RM4000210//Homo sapiens mRNA for KIAA0712 protein, complete cds.//0//1926bp//100%//AB018255
C-NT2RM4000229//Gallus gallus actin filament-associated protein (AFAP-110) mRNA, complete cds.//1.1E-27//633bp//64%//L20303
C-NT2RM4000290//Human transducin-like enhancer protein (TLE3) mRNA, complete cds.//2.2E-276//1124bp//97%//M99438
C-NT2RM4000344//Homo sapiens mRNA for ATP-dependent metalloprotease YME1L.//0//2030bp//99%//AJ132637
C-NT2RM4000349//Homo sapiens HSPC028 mRNA, complete cds.//0//1827bp//99%//AF083246
C-NT2RM4000354//LETHAL(2)DENTICLELESS PROTEIN (DTL83 PROTEIN).//1.5E-21//208aa//35%//Q24371
C-NT2RM4000386//Mus musculus mRNA for Ten-m3, complete cds.//0//2156bp//86%//AB025412
C-NT2RM4000395
C-NT2RM4000421//Homo sapiens mRNA for nuclear transport receptor.//0//1730bp//99%//AJ133769
C-NT2RM4000457//HYPOTHETICAL 111.9 KD PROTEIN C22H10.03C IN CHROMOSOME I.//8E-20//393aa//24%//Q10297
C-NT2RM4000471//Homo sapiens cysteine desulfurase (nifS) mRNA, complete cds.//0//2092bp//99%//AF097025
C-NT2RM4000486//SALIVARY PROLINE-RICH PROTEIN PRECURSOR (CLONES CP3, CP4 AND CP5) [CONTAINS: BASIC PEPTIDE IB-6; PEPTIDE P-H].//4.8E-11//242aa//31%//P04280
C-NT2RM4000496//SAP1 PROTEIN.//8.3E-53//434aa//29%//P39955
C-NT2RM4000511
C-NT2RM4000515//NEUROFILAMENT TRIPLET H PROTEIN (200 KD NEUROFILAMENT PROTEIN) (NF-H) (FRAGMENT).//1.1E-11//394aa//24%//P16884
C-NT2RM4000520
C-NT2RM4000585
C-NT2RM4000595//Homo sapiens leucine-rich repeats containing F-box protein FBL3 mRNA, complete cds.//1.1E-285//1293bp//99%//AF186273
C-NT2RP1000018//Homo sapiens mRNA for KIAA0687 protein, partial cds.//0//1940bp//95%//AB014587
C-NT2RP1000035//Homo sapiens mRNA for KIAA0850 protein, complete cds.//0//1652bp//99%//AB020657
C-NT2RP1000040
C-NT2RP1000063
C-NT2RP1000086//H.sapiens mRNA for zinc finger protein, Hsal2.//0//1162bp//99%//X98834
C-NT2RP1000101
C-NT2RP1000111//COP1 REGULATORY PROTEIN.//4E-116//296aa//51%//P93471
C-NT2RP1000112
C-NT2RP1000124
C-NT2RP1000130//HEPATOMA-DERIVED GROWTH FACTOR (HDGF).//4.5E-50//181aa//60%//P51859
C-NT2RP1000163//Homo sapiens mRNA for KIAA0948 protein, complete cds.//0//1889bp//98%//AB023165
C-NT2RP1000170
C-NT2RP1000191
C-NT2RP1000202//ANKYRIN.//1E-25//302aa//34%//Q02357
C-NT2RP1000243
C-NT2RP1000259
C-NT2RP1000272//Homo sapiens TLS-associated protein TASR-2 mRNA, complete cds.//5.8E-114//616bp//93%//AF067730
C-NT2RP1000326//Homo sapiens metaxin 2 (MTX2) mRNA, nuclear gene encoding mitochondrial protein, complete cds.//1.3E-275//1249bp//99%//AF053551
C-NT2RP1000333//ANTI-SILENCING PROTEIN 1.//8.7E-47//155aa//58%//P32447
C-NT2RP1000348//REDUCED VIABILITY UPON STARVATION PROTEIN 161.//1.7E-15//162aa//30%//P25343
C-NT2RP1000357
C-NT2RP1000376//Homo sapiens mRNA; cDNA DKFZp434A102 (from clone DKFZp434A102).//0//2265bp//95%//AL080187
C-NT2RP1000413//Homo sapiens mRNA for KIAA0587 protein, complete cds.//0//1056bp//99%//AB011159
C-NT2RP1000416
C-NT2RP1000439//Xenopus laevis chromosome condensation protein XCAP-G mRNA, complete cds.//1.8E-94//1019bp//63%//AF111423
C-NT2RP1000443//QUINONE OXIDOREDUCTASE (EC 1.6.5.5) (NADPH:QUINONE REDUCTASE) (ZETA- CRYSTALLIN).//2.4E-10//227aa//25%//Q08257
C-NT2RP1000470//PUTATIVE ATP-DEPENDENT RNA HELICASE T26G10.1 IN CHROMOSOME III.//2.6E-94//254aa//47%//P34580
C-NT2RP1000478//TUBULIN BETA-5 CHAIN (CLASS-V).//4.5E-240//445aa//97%//P09653
C-NT2RP1000481
C-NT2RP1000493//Homo sapiens mRNA for KIAA0017 protein, complete cds.//0//2728bp//99%//D87686
C-NT2RP1000547//COP-COATED VESICLE MEMBRANE PROTEIN P24 PRECURSOR (FRAGMENT).//1.1E-27//193aa//35%//P49020
C-NT2RP1000574//HOMEOBOX PROTEIN MEIS2 (MEIS1-RELATED PROTEIN 1).//3.5E-75//151aa//94%//P97367
C-NT2RP1000581
C-NT2RP1000630//NECDIN.//2.4E-44//227aa//41%//P25233
C-NT2RP1000688
C-NT2RP1000695
C-NT2RP1000733//Human mRNA for GSPT1-TK protein,complete cds.//0//2057bp//99%//E14379
C-NT2RP1000738//Homo sapiens Wolf-Hirschhorn syndrome candidate 2 protein (WHSC2) mRNA, complete cds.//0//2186bp//99%//AF101434
C-NT2RP1000782//PLATELET-ENDOTHELIAL TETRASPAN ANTIGEN 3 (PETA-3) (GP27) (MEMBRANE GLYCOPROTEIN SFA-1) (CD151 ANTIGEN).//1.2E-30//232aa//30%//O35566
C-NT2RP1000825//GTPASE-ACTIVATING PROTEIN RHOGAP (RHO-RELATED SMALL GTPASE PROTEIN ACTIVATOR) (CDC42 GTPASE-ACTIVATING PROTEIN) (P50-RHOGAP).//8.2E-83//334aa//50%//Q07960
C-NT2RP1000833//Homo sapiens cGMP phosphodiesterase A1 (PDE9A) mRNA, complete cds.//0//1494bp//99%//AF067223
C-NT2RP1000846
C-NT2RP1000851
C-NT2RP1000856//PLATELET-ENDOTHELIAL TETRASPAN ANTIGEN 3 (PETA-3) (GP27) (MEMBRANE GLYCOPROTEIN SFA-1) (CD151 ANTIGEN).//1.2E-30//232aa//30%//035566
C-NT2RP1000915//AUTOANTIGEN NGP-1.//1.7E-19//343aa//25%//Q13823
C-NT2RP1000947//Human E2 ubiquitin conjugating enzyme UbcH5B (UBCH5B) mRNA, complete cds.//4.6E-105//504bp//99<%//U39317
C-NT2RP1000954//RING CANAL PROTEIN (KELCH PROTEIN).//1.4E-23//370aa//28%//Q04652
C-NT2RP1000958//AUTOANTIGEN NGP-1.//1.4E-19//343aa//25%//Q13823
C-NT2RP1000959//Human acidic ribosomal phosphoprotein P0 mRNA, complete cds.//2.5E-236//966bp//99%//M17885
C-NT2RP1000966//NUCLEOLIN (PROTEIN C23).//8.9E-299//554aa//99%//P19338
C-NT2RP1000980
C-NT2RP1000988
C-NT2RP1001011//Drosophila melanogaster putative 43 kDa protein (TH1) mRNA, complete cds.//2.2E-78//1529bp//61%//L01790
C-NT2RP1001014
C-NT2RP1001395
C-NT2RP1001410//PUTATIVE GTP-BINDING PROTEIN W08E3.3.//8.9E-141//396aa//67%//P91917
C-NT2RP1001424
C-NT2RP1001449
C-NT2RP1001457//Homo sapiens partial mRNA for beta-transducin family protein (putative).//1.2E-137//629bp//100%//AJ005257
C-NT2RP1001466
C-NT2RP1001475
C-NT2RP1001482
C-NT2RP1001494//MALE STERILITY PROTEIN 2.//7.2E-40//261aa//27%//Q08891
C-NT2RP1001543//MYO-INOSITOL-1-PHOSPHATE SYNTHASE (EC 5.5.1.4) (IPS).//1.6E-166//506aa//60%//P42803
C-NT2RP1001546//PLATELET-ENDOTHELIAL TETRASPAN ANTIGEN 3 (PETA-3) (GP27) (MEMBRANE GLYCOPROTEIN SFA-1) (CD151 ANTIGEN).//1.6E-30//232aa//30%//035566
C-NT2RP1001569//SIGNAL RECOGNITION PARTICLE RECEPTOR BETA SUBUNIT (SR-BETA).//5.8E-121//271aa//89%//P47758
C-NT2RP1001616
C-NT2RP1001665//CALMODULIN.//0.00000051//83aa//30%//P02594
C-NT2RP2000006//DNAJ PROTEIN (40 KD HEAT SHOCK CHAPERONE PROTEIN) (HSP40).//9.8E-17//79aa//55%//O34136
C-NT2RP2000007
C-NT2RP2000008//ZINC FINGER PROTEIN 84 (ZINC FINGER PROTEIN HPF2).//2.4E-177//726aa//47%//P51523
C-NT2RP2000032//RAS SUPPRESSOR PROTEIN 1 (RSU-1) (RSP-1 PROTEIN) (RSP1).//1.8E-22//184aa//34%//Q01730
C-NT2RP2000045//Homo sapiens tumorous imaginal discs protein Tid56 homolog (TID1) mRNA, complete cds.//0//1390bp//98%//AF061749
C-NT2RP2000054
C-NT2RP2000056//PROTEIN-TYROSINE PHOSPHATASE EPSILON PRECURSOR (EC 3.1.3.48) (R-PTP- EPSILON).//9.4E-16//45aa//100%//P49446
C-NT2RP2000067
C-NT2RP2000070//CADHERIN-RELATED TUMOR SUPPRESSOR PRECURSOR (FAT PROTEIN).//3.4E-51//383aa//32%//P33450
C-NT2RP2000079
C-NT2RP2000088//Homo sapiens mRNA for KIAA0795 protein, partial cds.//0//2286bp//100%//AB018338
C-NT2RP2000091
C-NT2RP2000097
C-NT2RP2000114//Homo sapiens mRNA for GM3 synthase, complete cds.//0//2244bp//99%//AB018356
C-NT2RP2000120
C-NT2RP2000126//POSSIBLE GLOBAL TRANSCRIPTION ACTIVATOR SNF2L.//2.5E-117//541aa//42%//P41877
C-NT2RP2000133//Homo sapiens mRNA for KIAA0989 protein, partial cds.//0//2286bp//99%//AB023206
C-NT2RP2000147//CLATHRIN COAT ASSEMBLY PROTEIN AP47 (CLATHRIN COAT ASSOCIATED PROTEIN AP47) (GOLGI ADAPTOR AP-1-47 KD PROTEIN) (HA1 47 KD SUBUNIT) (CLATHRIN ASSEMBLY PROTEIN ASSEMBLY PROTEIN COMPLEX 1 MEDIUM CHAIN).//4.4E-226//423aa//99%//P35585
C-NT2RP2000153//GAR2 PROTEIN.//9.8E-23//311aa//28%//P41891
C-NT2RP2000157//MLO2 PROTEIN.//2.6E-11//62aa//40%//Q09329
C-NT2RP2000161//Homo sapiens mRNA for KIAA1008 protein, complete cds.//3.4e-315//1430bp//99%//AB023225
C-NT2RP2000173
C-NT2RP2000175
C-NT2RP2000195
C-NT2RP2000205
C-NT2RP2000208//Homo sapiens mRNA for KIAA0892 protein, partial cds.//0//2898bp//99%//AB020699
C-NT2RP2000224//INSULIN RECEPTOR SUBSTRATE-1 (IRS1).//0.000043//103aa//28%//P35568
C-NT2RP2000232
C-NT2RP2000233
C-NT2RP2000239
C-NT2RP2000248//UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).//3.4E-21//210aa//33%//P56558
C-NT2RP2000270
C-NT2RP2000274
C-NT2RP2000283
C-NT2RP2000288//HYPOTHETICAL 111.9 KD PROTEIN C22H10.03C IN CHROMOSOME I.//1.6E-27//576aa//25%//Q10297
C-NT2RP2000297//ZINC FINGER PROTEIN 184 (FRAGMENT).//3.3E-186//256aa//60%//Q99676
C-NT2RP2000298
C-NT2RP2000310//Human proline-dehydrogenase/proline oxidase (PRODH) mRNA, complete cds.//4.3E-279//1193bp//99%//U82381
C-NT2RP2000328
C-NT2RP2000329//GTP:AMP PHOSPHOTRANSFERASE MITOCHONDRIAL (EC 2.7.4.10) (AK3).//2E-111//226aa//92%//P08760
C-NT2RP2000346//MYELOID DIFFERENTIATION PRIMARY RESPONSE PROTEIN MYD116.//6.3E-115//674aa//46%//P17564
C-NT2RP2000369
C-NT2RP2000412
C-NT2RP2000414//HETEROGENEOUS NUCLEAR RIBONUCLEOPROTEIN F (HNRNP F).//4.3E-228//415aa//100%//P52597
C-NT2RP2000422//Homo sapiens N-acetylglucosamine-phosphate mutase mRNA, complete cds.//0//1757bp//99%//AF102265
C-NT2RP2000438
C-NT2RP2000448//KES1 PROTEIN.//8.7E-54//392aa//38%//P35844
C-NT2RP2000503
C-NT2RP2000510
C-NT2RP2000516
C-NT2RP2000603
C-NT2RP2000617
C-NT2RP2000634//Homo sapiens mRNA for KIAA0614 protein, partial cds.//0//2482bp//99%//AB014514
C-NT2RP2000656
C-NT2RP2000658
C-NT2RP2000668//SERINE/THREONINE PROTEIN KINASE PKPA (EC 2.7.1.-).//1.3E-27//349aa//32%//Q01577
C-NT2RP2000704
C-NT2RP2000710//ASPARTYL-TRNA SYNTHETASE (EC 6.1.1.12) (ASPARTATE--TRNA LIGASE)//2.7E-100//488aa//44%//O32038
C-NT2RP2000764//NIFS PROTEIN.//6.6E-36//252aa//42%//P12623
C-NT2RP2000809//Homo sapiens mRNA for KIAA0873 protein, partial cds.//0//3347bp//99%//AB020680
C-NT2RP2000812//DILUTE MYOSIN HEAVY CHAIN, NON-MUSCLE (MYOSIN 5A).//0.000000056//179aa//29%//Q99104
C-NT2RP2000814//GELATION FACTOR (ACTIN BINDING PROTEIN 120) (ABP-120).//0.00000011//96aa//29%//P13466
C-NT2RP2000816//MAGNESIUM-CHELATASE 30 KD SUBUNIT.//0.000000079//172aa//28%//P26174
C-NT2RP2000819
C-NT2RP2000841
C-NT2RP2000845
C-NT2RP2000863
C-NT2RP2000880//PROBABLE TRANSLATION INITIATION FACTOR IF-2.//0//694aa//99%//060841
C-NT2RP2000892
C-NT2RP2000931//MATRIN 3.//2.4E-289//467aa//95%//P43244
C-NT2RP2000932//Homo sapiens mRNA; cDNA DKFZp5640043 (from clone DKFZp5640043).//0//2487bp//99%//AL050390
C-NT2RP2000938
C-NT2RP2000943//Homo sapiens mRNA for KIAA0755 protein, complete cds.//0//3458bp//99%//AB018298
C-NT2RP2000965//Homo sapiens mRNA for fls353, complete cds.//0//1989bp//96%//AB024704
C-NT2RP2000985
C-NT2RP2001036
C-NT2RP2001044
C-NT2RP2001056//Homo sapiens mRNA, chromosome 1 specific transcript KIAA0488.//0//2749bp//99%//AB007957
C-NT2RP2001065
C-NT2RP2001070//PUTATIVE PYRIDOXAMINE 5'-PHOSPHATE OXIDASE (EC 1.4.3.5) (PNP/PMP OXIDASE).//5.8E-46//222aa//45%//Q20939
C-NT2RP2001081//SYNAPTOTAGMIN IV.//4.2E-118//430aa//54%//P50232
C-NT2RP2001094
C-NT2RP2001119
C-NT2RP2001127//Homo sapiens mRNA for PLU-1 protein.//0//2514bp//99%//AJ132440
C-NT2RP2001218
C-NT2RP2001245//MYOSIN HEAVY CHAIN, NONMUSCLE (CELLULAR MYOSIN HEAVY CHAIN) (NMMHC).//2.2E-10//366aa//28%//P14105
C-NT2RP2001381
C-NT2RP2001397//Homo sapiens mRNA; cDNA DKFZp434B174 (from clone DKFZp434B174).//0//1495bp//100%//AL080146
C-NT2RP2001427
C-NT2RP2001601//Homo sapiens mRNA for KIAA0797 protein, partial cds.//0//1748bp//99%//AB018340
C-NT2RP2001675
C-NT2RP2001721
C-NT2RP2001907
C-NT2RP2001969
C-NT2RP2001976//Mus musculus calmodulin-binding protein SHA1 (Sha1) mRNA, complete cds.//4.7E-177//1538bp//74%//AF062378
C-NT2RP2002046
C-NT2RP2002154
C-NT2RP2002208
C-NT2RP2002270//AF-9 PROTEIN.//0.00000012//74aa//36%//P42568
C-NT2RP2002312//Homo sapiens mRNA for CDS2 protein.//0//2333bp//99%//Y16521
C-NT2RP2002325//Homo sapiens mRNA for Pex11p, complete cds.//8.4E-254//1158bp//99%//AB015594
C-NT2RP2002385//Homo sapiens synaptic glycoprotein SC2 spliced variant mRNA, complete cds.//4.3E-240//1105bp//99%//AF038958
C-NT2RP2002426
C-NT2RP2002479//Homo sapiens mRNA for ABC transporter 7 protein, complete cds.//0//2180bp//99%//AB005289
C-NT2RP2002537//HYPOTHETICAL 55.1 KD PROTEIN B0416.5 IN CHROMOSOME X.//6.2E-19//288aa//26%//Q11073
C-NT2RP2002595//PROBABLE CALCIUM-BINDING PROTEIN ALG-2 (PMP41) (ALG-257).//7.5E-35//181aa//42%//P12815
C-NT2RP2002618//PROTEIN ARGININE N-METHYLTRANSFERASE 2 (EC 2.1.1.-).//1.7E-51//326aa//38%//P55345
C-NT2RP2002621
C-NT2RP2002672
C-NT2RP2002701//HYPOTHETICAL 38.1 KD PROTEIN C2F12.15C IN CHROMOSOME II.//1.9E-14//210aa//30%//O14345
C-NT2RP2002769
C-NT2RP2002862//60S ACIDIC RIBOSOMAL PROTEIN P0 (LIGHT-INDUCED 34 KD PROTEIN).//8.8E-10//203aa//27%//P29764
C-NT2RP2002928//Homo sapiens pre-mRNA splicing factor (PRP17) mRNA, complete cds.//1.9E-136//623bp//100%//AF038392
C-NT2RP2002954
C-NT2RP2002959//UBIQUITIN-CONJUGATING ENZYME E2-17 KD 2 (EC 6.3.2.19) (UBIQUITIN- PROTEIN LIGASE) (UBIQUITIN CARRIER PROTEIN) (E2(17)KB 2).//4.6E-80//147aa//100%//P51669
C-NT2RP2002980//30S RIBOSOMAL PROTEIN S10.//0.00000001//98aa//36%//P10129
C-NT2RP2002986//Homo sapiens mRNA for Kelch motif containing protein, complete cds.//0//2209bp//99%//AB026190
C-NT2RP2003108
C-NT2RP2003117
C-NT2RP2003121//Mus musculus enhancer of polycomb (Epc1) mRNA, complete eds.//2.3E-82//642bp//68%//AF079765
C-NT2RP2003125//RING CANAL PROTEIN (KELCH PROTEIN).//2.4E-38//539aa//25%//004652
C-NT2RP2003177
C-NT2RP2003194
C-NT2RP2003265//Homo sapiens CGI-53 protein mRNA, complete cds.//0//1580bp//99%//AF151811
C-NT2RP2003295//Homo sapiens RMP mRNA for RPB5 meidating protein, complete cds.//0//1526bp//99%//AB006572
C-NT2RP2003329//PUTATIVE ADENYLATE CYCLASE REGULATORY PROTEIN.//3.6E-14//332aa//32%//P26337
C-NT2RP2003367
C-NT2RP2003433//PROTEIN TRANSPORT PROTEIN SEC61 ALPHA SUBUNIT.//5E-131//269aa//91%//P38378
C-NT2RP2003446
C-NT2RP2003533
C-NT2RP2003543//HYPOTHETICAL TRNA/RRNA METHYLTRANSFERASE SLR1673 (EC 2.1.1.-).//1.7E-17//148aa//34%//P74261
C-NT2RP2003596
C-NT2RP2003629
C-NT2RP2003687
C-NT2RP2003714//ZINC FINGER PROTEIN 91 (ZINC FINGER PROTEIN HTF10) (HPF7).//5.4E-29//85aa//72%//Q05481
C-NT2RP2003737//UBIQUITIN-CONJUGATING ENZYME E2-17 KD 2 (EC 6.3.2.19) (UBIQUITIN-PROTEIN LIGASE) (UBIQUITIN CARRIER PROTEIN) (E2(17)KB 2).//1.7E-75//147aa//93%//P51669
C-NT2RP2003793
C-NT2RP2003952//AMINOPEPTIDASE B (EC 3.4.11.6) (ARGINYL AMINOPEPTIDASE) (ARGININE AMINOPEPTIDASE) (CYTOSOL AMINOPEPTIDASE IV) (AP-B).//1.5E-23//200aa//30%//O09175
C-NT2RP2003986
C-NT2RP2004042
C-NT2RP2004316//Homo sapiens chromosome 1 clone J549L20, WORKING DRAFT SEQUENCE, in unordered pieces.//8.2E-202//926bp//100%//AL096820
C-NT2RP2004389//PROBABLE MITOCHONDRIAL 40S RIBOSOMAL PROTEIN S9 PRECURSOR.//9.3E-15//126aa//39%//P38120
C-NT2RP2004392//MNN4 PROTEIN.//1.4E-11//143aa//27%//P36044
C-NT2RP2004463
C-NT2RP2004602
C-NT2RP2004614//Homo sapiens mRNA for KIAA0922 protein, partial cds.//0//2040bp//99%//AB023139
C-NT2RP2004655//Homo sapiens mRNA for leucine rich protein.//8.5E-233//1061bp//99%//AJ006291
C-NT2RP2004689//HYPOTHETICAL 192.5 KD PROTEIN C6G9.10C IN CHROMOSOME I.//5.6E-64//616aa//33%//Q92355
C-NT2RP2004791//PUTATIVE LEUCYL-TRNA SYNTHETASE, CYTOPLASMIC (EC 6.1.1.4) (LEUCINE-- TRNA LIGASE) (LEURS).//9.5E-73//153aa//59%//Q10490
C-NT2RP2004799//PROBABLE SUCCINYL-COA LIGASE [GDP-FORMING], BETA-CHAIN PRECURSOR (EC 6.2.1.4) (SUCCINYL-COA SYNTHETASE, BETA CHAIN) (SCS-BETA).//3.7E-135//414aa//62%//P53588
C-NT2RP2004802
C-NT2RP2004841
C-NT2RP2004936
C-NT2RP2004959//P54 PROTEIN PRECURSOR.//0.00000095//297aa//20%//P13692
C-NT2RP2004999
C-NT2RP2005000
C-NT2RP2005001//Homo sapiens mRNA for KIAA0615 protein, complete cds.//0//l694bp//99%//AB014515
C-NT2RP2005012//Homo sapiens mRNA for SEC63 protein.//0//1693bp//99%//AJ011779
C-NT2RP2005037//ANTI-SILENCING PROTEIN 1.//3.3E-47//155aa//59%//P32447
C-NT2RP2005126//H.sapiens mRNA for RNA helicase (Myc-regulated dead box protein).//0//2388bp//98%//X98743
C-NT2RP2005140
C-NT2RP2005147
C-NT2RP2005159
C-NT2RP2005239//Homo sapiens cysteine desulfurase (nifS) mRNA, complete cds.//0//2087bp//99%//AF097025
C-NT2RP2005270
C-NT2RP2005276//Homo sapiens mRNA for Acyl-CoA synthetase 3, complete cds.//0//2122bp//99%//D89053
C-NT2RP2005293
C-NT2RP2005315//Homo sapiens mRNA for KIAA0676 protein, partial cds.//0//1515bp//99%//AB014576
C-NT2RP2005358//Homo sapiens methyl-CpG binding domain-containing protein MBD3 (MBD3) mRNA, complete cds.//0//2199bp//99%//AF072247
C-NT2RP2005393//AUTOANTIGEN NGP-1.//7.2E-39//224aa//35%//Q13823
C-NT2RP2005436//SPLICING FACTOR, ARGININE/SERINE-RICH 4 (PRE-MRNA SPLICING FACTOR SRP75).//1.2E-13//185aa//38%//Q08170
C-NT2RP2005441
C-NT2RP2005453
C-NT2RP2005464
C-NT2RP2005465//MITOCHONDRIAL CARRIER PROTEIN RIM2.//3E-44//252aa//41%//P38127
C-NT2RP2005472
C-NT2RP2005495
C-NT2RP2005498//PROTEIN PHOSPHATASE PP2A, 55 KD REGULATORY SUBUNIT, ALPHA ISOFORM (PROTEIN PHOSPHATASE PP2A B SUBUNIT ALPHA ISOFORM) (ALPHA-PR55).//5.2E-81//166aa//88%//P36876
C-NT2RP2005509//Homo sapiens CGI-45 protein mRNA, complete cds.//0//1825bp//99%//AF151803
C-NT2RP2005520//Homo sapiens chromosome-associated protein-E (hCAP-E) mRNA, complete cds.//0//3994bp//99%//AF092563
C-NT2RP2005525//Mus musculus kanadaptin mRNA, complete cds.//2.4E-304//1687bp//85%//AF035526
C-NT2RP2005540//Homo sapiens mRNA for KIAA0494 protein, complete cds.//0//2856bp//99%//AB007963
C-NT2RP2005549//PUTATIVE LACTOYLGLUTATHIONE LYASE (EC 4.4.1.5) (METHYLGLYOXALASE) (ALDOKETOMUTASE) (GLYOXALASE I) (GLX I) (KETONE-ALDEHYDE MUTASE) (S-D-LACTOYLGLUTATHIONE METHYLGLYOXAL LYASE).//2E-20//181aa//36%//Q39366
C-NT2RP2005555
C-NT2RP2005557//Homo sapiens clone 486790 diphosphoinositol polyphosphate phosphohydrolase mRNA, complete cds.//1E-46//576bp//70%//AF062529
C-NT2RP2005620//Homo sapiens epsin 2a mRNA, complete cds.//8.9e-313//1455bp//98%//AF062085
C-NT2RP2005622
C-NT2RP2005635//PROBABLE NH(3)-DEPENDENT NAD(⁺) SYNTHETASE (EC 6.3.5.1).//1E-11//128aa//36%//P47623
C-NT2RP2005637
C-NT2RP2005640
C-NT2RP2005654//CYSTEINE STRING PROTEIN (CCCS1).//1.2E-13//74aa//45%//P56l01
C-NT2RP2005669//Homo sapiens mRNA for DEDD protein.//3.9E-209//957bp//99%//AJ010973
C-NT2RP2005675//Homo sapiens growth suppressor related (DOC-1R) mRNA, complete cds.//4.4E-200//908bp//99%//AF089814
C-NT2RP2005683
C-NT2RP2005690
C-NT2RP2005712//Homo sapiens mRNA for KIAA0799 protein, partial cds.//0//1684bp//99%//AB018342
C-NT2RP2005723//HNRNP ARGININE N-METHYLTRANSFERASE (EC 2.1.1.-) (ODP1 PROTEIN).//0.000000003//169aa//28%//P38074
C-NT2RP2005748
C-NT2RP2005752//Homo sapiens TNFR-related death receptor-6 (DR6) mRNA, complete cds.//0//1968bp//99%//AF068868
C-NT2RP2005753//Homo sapiens I-1 receptor candidate protein mRNA, complete cds.//0//1966bp//99%//AF082516
C-NT2RP2005763//EUKARYOTIC INITIATION FACTOR 4A (EIF-4A).//1.7E-61//374aa//38%//P47943
C-NT2RP2005767//G.gallus PB1 gene.//5E-163//1158bp//81%//X90849
C-NT2RP2005773//Homo sapiens pyrroline 5-carboxylate reductase isoform (P5CR2) mRNA, complete cds.//2.7E-180//656bp//99%//AF151351
C-NT2RP2005775//NEUROLYSIN PRECURSOR (EC 3.4.24.16) (NEUROTENSIN ENDOPEPTIDASE) (MITOCHONDRIAL OLIGOPEPTIDASE M) (MICROSOMAL ENDOPEPTIDASE) (MEP) (SOLUBLE ANGIOTENSIN-BINDING PROTEIN) (SABP).//2.1E-213//249aa//85%//Q02038
C-NT2RP2005781
C-NT2RP2005804
C-NT2RP2005835//SHP1 PROTEIN.//1.8E-28//208aa//32%//P34223
C-NT2RP2005853
C-NT2RP2005868
C-NT2RP2005886
C-NT2RP2005890
C-NT2RP2005901//Homo sapiens mRNA for KIAA0971 protein, complete cds.//0//1977bp//99%//AB023188
C-NT2RP2005933//NUCLEOPORIN NUP57 (NUCLEAR PORE PROTEIN NUP57).//5E-11//155aa//34%//P48837
C-NT2RP2006038
C-NT2RP2006043//SPLICING FACTOR, ARGININE/SERINE-RICH 4 (PRE-MRNA SPLICING FACTOR SRP75).//1.5E-13//185aa//38%//Q08170
C-NT2RP2006052
C-NT2RP2006069
C-NT2RP2006071
C-NT2RP2006100//Homo sapiens mRNA; cDNA DKFZp564B102 (from clone DKFZp564B102).//0//1759bp//99%//AL049970
C-NT2RP2006106
C-NT2RP2006141
C-NT2RP2006186//Homo sapiens mRNA for KIAA0654 protein, partial cds.//3.3E-189//899bp//97%//AB014554
C-NT2RP2006196
C-NT2RP2006200
C-NT2RP2006219//H.sapiens mRNA for DGCR6 protein.//1.1E-214//1026bp//97%//X96484
C-NT2RP2006237
C-NT2RP2006238
C-NT2RP2006275//MICROTUBULE-ASSOCIATED PROTEIN 1B [CONTAINS: LIGHT CHAIN LC1].//2E-59//388aa//32%//P46821
C-NT2RP2006312//Homo sapiens BAF57 (BAF57) gene, complete cds.//2.8E-274//1236bp//99%//AF035262
C-NT2RP2006333
C-NT2RP2006365
C-NT2RP2006393
C-NT2RP2006436//ANTERIOR-RESTRICTED HOMEOBOX PROTEIN (RATHKE POUCH HOMEO BOX).//0.00000034//50aa//50%//Q61658
C-NT2RP2006456
C-NT2RP2006464//Homo sapiens mRNA for AND-1 protein.//0//2181bp//99%//AJ006266
C-NT2RP2006467
C-NT2RP2006472
C-NT2RP2006565//Sus scrofa mRNA for SCAMPI protein.//0//1276bp//84%//Y15710
C-NT2RP2006571//CYTOCHROME P450 2G1 (EC 1.14.14.1) (CYPIIG1) (P450-NMB) (OLFACTIVE).//4.2E-134//486aa//50%//P24461
C-NT2RP2006573//2',3'-CYCLIC NUCLEOTIDE 3'-PHOSPHODIESTERASE (EC 3.1.4.37) (CNP).//0.0000055//169aa//25%//P09543
C-NT2RP3000031//Homo sapiens mRNA for KIAA0901 protein, complete cds.//0//2547bp//99%//AB020708
C-NT2RP3000072
C-NT2RP3000142//Homo sapiens mRNA for KIAA0592 protein, partial cds.//0//1404bp//97%//AB011164
C-NT2RP3000220
C-NT2RP3000251
C-NT2RP3000252//Homo sapiens GTP-binding protein NGB mRNA, complete cds.//0//2388bp//99%//AF120334
C-NT2RP3000312
C-NT2RP3000320//Homo sapiens partial mRNA for putative p621 protein which interacts with transcription factor Sp1.//0//1544bp//100%//AJ242978
C-NT2RP3000333
C-NT2RP3000348
C-NT2RP3000350//PROBABLE GTP-BINDING PROTEIN
HP0303.//0.000000028//185aa//31%//O25074
C-NT2RP3000359//GTP:AMP PHOSPHOTRANSFERASE MITOCHONDRIAL (EC 2.7.4.10) (AK3).//2E-111//226aa//92%//P08760
C-NT2RP3000361//Homo sapiens mRNA, complete cds, similar to yeast pre-mRNA splicing factors, Prp1/Zer1 and Prp6.//0//2072bp//98%//AB019219
C-NT2RP3000366//RAS-RELATED PROTEIN RAB-18.//2.1E-107//206aa//99%//P35293
C-NT2RP3000397//PUTATIVE PRE-MRNA SPLICING FACTOR RNA HELICASE (DEAH BOX PROTEIN 13)//1.7E-139//679aa//41%//O43143
C-NT2RP3000403//Homo sapiens formin binding protein 21 mRNA, complete cds.//0//2364bp//99%//AF071185
C-NT2RP3000484
C-NT2RP3000527//ZINC FINGER PROTEIN 43 (ZINC PROTEIN HTF6).//4.8E-28//536aa//27%//P28160
C-NT2RP3000531//POLIOVIRUS RECEPTOR PRECURSOR (CD155 ANTIGEN).//1.9E-12//192aa//30%//P15151
C-NT2RP3000596/TRICHOHYALIN.//2.5E-17//304aa//28%//Q07283
C-NT2RP3000599
C-NT2RP3000632//ZINC FINGER PROTEIN 84 (ZINC FINGER PROTEIN HPF2).//3E-140//499aa//46%//P51523
C-NT2RP3000644
C-NT2RP3000661
C-NT2RP3000665
C-NT2RP3000690
C-NT2RP3000759//ADP-RIBOSYLATION FACTOR.//7E-28//176aa//34%//Q94650
C-NT2RP3000825//NEUROGENIC LOCUS NOTCH 3 PROTEIN.//2.5E-36//417aa//31%//Q61982
C-NT2RP3000836
C-NT2RP3000841
C-NT2RP3000850
C-NT2RP3000852
C-NT2RP3000859
C-NT2RP3000868//Human ovarian cancer downregulated myosin heavy chain homolog (Doc1) mRNA, complete cds.//6.9E-69//1611bp//61%//U53445
C-NT2RP3000869
C-NT2RP3000901
C-NT2RP3000917//Homo sapiens Dhm1-like protein mRNA, complete cds.//0//3199bp//99%//AF064257
C-NT2RP3000919//Rattus norvegicus golgi peripheral membrane protein p65 (GRASP65) mRNA, complete cds.//2.7E-185//585bp//88%//AF015264
C-NT2RP3000980
C-NT2RP3000994//MATERNAL EFFECT PROTEIN
STAUFEN.//0.00000006//78aa//48%//P25159
C-NT2RP3001004
C-NT2RP3001081
C-NT2RP3001084
C-NT2RP3001096//Rattus norvegicus leprecan (lepre1) mRNA, complete cds.//1.7E-94//787bp//66%//AF087433
C-NT2RP3001107//PEREGRIN (BR140 PROTEIN).//3E-44//260aa//40%//P55201
C-NT2RP3001109
C-NT2RP3001116
C-NT2RP3001119
C-NT2RP3001133
C-NT2RP3001140//Homo sapiens mRNA for KIAA0762 protein, partial cds.//0//2802bp//99%//AB018305
C-NT2RP3001155//Homo sapiens mRNA for AND-1 protein.//0//2732bp//99%//AJ006266
C-NT2RP3001176//HYPOTHETICAL 65.3 KD PROTEIN IN MAD1-SCY1 INTERGENIC REGION.//1.7E-10//196aa//27%//P53154
C-NT2RP3001214
C-NT2RP3001216//CYLICIN I (MULTIPLE-BAND POLYPEPTIDE I) (FRAGMENT).//0.0000023//137aa//33%//P35663
C-NT2RP3001221//GAMMA-BUTYROBETAINE,2-OXOGLUTARATE DIOXYGENASE (EC 1.14.11.1) (GAMMA-BUTYROBETAINE HYDROXYLASE).//1.9E-31//353aa//30%//P80193
C-NT2RP3001236
C-NT2RP3001239//MICROTUBULE-ASSOCIATED PROTEIN 1B (MAP1.2) (MAP1(X)) [CONTAINS: LIGHT CHAIN LC1].//1.2E-166//395aa//51%//P14873
C-NT2RP3001260//Homo sapiens mRNA for KIAA0911 protein, complete cds.//0//2497bp//99%//AB020718
C-NT2RP3001307
C-NT2RP3001325
C-NT2RP3001384//Homo sapiens NAKAP95 mRNA for neighbor of A-kinase anchoring protein 95, complete cds.//0//1213bp//99%//AB025905
C-NT2RP3001392
C-NT2RP3001396
C-NT2RP3001398//TRANSCRIPTIONAL REPRESSOR CTCF.//1.3E-61//374aa//36%//P49711
C-NT2RP3001407//SCY1 PROTEIN.//0.00000033//143aa//25%//P53009
C-NT2RP3001420
C-NT2RP3001426//DNAJ PROTEIN (FRAGMENT).//1E-16//77aa//46%//O33529
C-NT2RP3001427//WERNER SYNDROME HELICASE HOMOLOG.//2.7E-10//159aa//33%//O09053
C-NT2RP3001457
C-NT2RP3001472//NONHISTONE CHROMOSOMAL PROTEIN 6A.//9.1E-13//87aa//43%//P11632
C-NT2RP3001495//Human oxidoreductase (HHCMA56) mRNA, complete cds.//0//1475bp//99%//U13395
C-NT2RP3001497//Homo sapiens multiple membrane spanning receptor TRC8 (TRC8) mRNA, complete cds.//0//2295bp//99%//AF064801
C-NT2RP3001529//SPO0B-ASSOCIATED GTP-BINDING PROTEIN.//1E-61//345aa//42%//P20964
C-NT2RP3001621
C-NT2RP3001629
C-NT2RP3001642//HYPOTHETICAL PROTEIN KIAA0210.//6.8E-18//91aa//38%//Q92609
C-NT2RP3001646//WD-40 REPEAT PROTEIN MSI2.//8.8E-09//132aa//31%//O22468
C-NT2RP3001676
C-NT2RP3001679
C-NT2RP3001799//MYOSIN HEAVY CHAIN, STRIATED MUSCLE.//1.6E-11//348aa//27%//P24733
C-NT2RP3001819//RING CANAL PROTEIN (KELCH PROTEIN).//7.4E-18//249aa//30%//Q04652
C-NT2RP3001896
C-NT2RP3001915
C-NT2RP3001929
C-NT2RP3003193//ZINC FINGER PROTEIN 135.//7.3E-98//269aa//62%//P52742
C-NT2RP3004466
C-NT2RP3004480//VACUOLAR PROTEIN SORTING-ASSOCIATED PROTEIN VPS35.//3.3E-113//466aa//42%//P34110
C-NT2RP3004539//Homo sapiens mRNA for KIAA0632 protein, partial cds.//0//1520bp//99%//AB014532
C-NT2RP3004544//Homo sapiens mRNA for KIAA0554 protein, partial cds.//0//974bp//95%//AB011126
C-NT2RP3004569//ANKYRIN, BRAIN VARIANT 1 (ANKYRIN B) (ANKYRIN, NONERYTHROID).//0.000000038//150aa//28%//Q01484
C-NT2RP3004572//Homo sapiens cofactor of initiator function (CIF150) mRNA, complete cds.//0//1770bp//99%//AF026445
C-NT2RP3004578//Homo sapiens mRNA for KIAA0477 protein, complete cds.//0//1639bp//99%//AB007946
C-NT2RP3004594//Homo sapiens mRNA for AND-1 protein.//0//1807bp//99%//AJ006266
C-NT2RP3004617//ZINC-BINDING PROTEIN A33.//7.2E-75//464aa//35%//Q02084
C-NT2RP3004618//Homo sapiens putative RNA-binding protein Q99 mRNA, complete cds.//0//3972bp//98%//AF093097
C-NT2RP3004669//ETHANOLAMINE KINASE (EC 2.7.1.82) (EASILY SHOCKED PROTEIN).//1.7E-72//254aa//45%//P54352
C-NT2RP4000008//CHLORINE CHANNEL PROTEIN P64.//2.6E-98//239aa//64%//P35526
C-NT2RP4000051//SYNAPTONEMAL COMPLEX-PROTEIN SC65.//4.9E-51//335aa//37%//Q64375
C-NT2RP4000078//Homo sapiens mRNA for KIAA0850 protein, complete cds.//0//3013bp//99%//AB020657
C-NT2RP4000109//Homo sapiens mRNA for MEGF5, partial cds.//0//2161bp//99%//AB011538
C-NT2RP4000111//CLEAVAGE AND POLYADENYLATION SPECIFICITY FACTOR, 100 KD SUBUNIT (CPSF 100 KD SUBUNIT).//0//728aa//99%//Q10568
C-NT2RP4000129
C-NT2RP4000147//Drosophila melanogaster putative ARF1 GTPase activating protein (ARF1-GAP) mRNA, complete cds.//3.8E-28//528bp//67%//AF011427
C-NT2RP4000150
C-NT2RP4000151
C-NT2RP4000159
C-NT2RP4000185
C-NT2RP4000210//Homo sapiens mRNA for KIAA0700 protein, partial cds.//0//4149bp//99%//AB014600
C-NT2RP4000212//ATRIAL GLAND-SPECIFIC ANTIGEN PRECURSOR (AGSA).//5.9E-15//104aa//40%//P15287
C-NT2RP4000243//Homo sapiens mRNA for cartilage-associated protein (CASP).//0//1932bp//99%//AJ006470
C-NT2RP4000246//NPC DERIVED PROLINE RICH PROTEIN 1 (NDPP-1).//2.7E-84//208aa//76%//Q03173
C-NT2RP4000259//GLUTATHIONE PEROXIDASE 2 (EC 1.11.1.9).//5.5E-29//153aa//43%//O23968
C-NT2RP4000290//HYPOTHETICAL 116.5 KD PROTEIN C20G8.09C IN CHROMOSOME I.//3.5E-297//1024aa//55%//P87115
C-NT2RP4000312//ADENYLATE CYCLASE (EC 4.6.1.1) (ATP PYROPHOSPHATE-LYASE) (ADENYLYL CYCLASE).//1.5E-26//237aa//28%//Q01631
C-NT2RP4000323//KERATIN, ULTRA HIGH-SULFUR MATRIX PROTEIN (UHS KERATIN).//0.0000003//101aa//32%//P26372
C-NT2RP4000355
C-NT2RP4000360//Homo sapiens mRNA for KIAA0738 protein, complete cds.//0//4074bp//99%//AB018281
C-NT2RP4000367//Homo sapiens IkappaB kinase complex associated protein (IKAP) mRNA, complete cds.//0//4782bp//99%//AF044195
C-NT2RP4000370//MITOCHONDRIAL PEPTIDE CHAIN RELEASE FACTOR 1 PRECURSOR (MRF-1).//2.6E-77//262aa//54%//O75570
C-NT2RP4000376//Homo sapiens mRNA for phospholipase A2 activating protein.//0//2412bp//99%//AJ238243
C-NT2RP4000381
C-NT2RP4000398//ZINC FINGER PROTEIN 140.//2.9E-110//435aa//50%//P52738
C-NT2RP4000415
C-NT2RP4000417//MANNOSYL-OLIGOSACCHARIDE ALPHA-1,2-MANNOSIDASE (EC 3.2.1.113)(MAN(9)-ALPHA-MANNOSIDASE) (FRAGMENT).//2.6E-51//438aa//33%//P45701
C-NT2RP4000448//Homo sapiens mRNA; cDNA DKFZp566G0746 (from clone DKFZp566G0746).//0//3991bp//99%//AL050078
C-NT2RP4000449
C-NT2RP4000455//TRANS-ACTING TRANSCRIPTIONAL PROTEIN ICP0.//0.0000003//175aa//27%//P09309
C-NT2RP4000457//UBIQUITIN CARBOXYL-TERMINAL HYDROLASE 15 (EC 3.1.2.15) (UBIQUITIN THIOLESTERASE 15) (UBIQUITIN-SPECIFIC PROCESSING PROTEASE 15)(DEUBIQUITINATING ENZYME 15).//2.5E-37//291aa//38%//P50101
C-NT2RP4000480
C-NT2RP4000481//ATP-DEPENDENT RNA HELICASE DOB1 (MRNA TRANSPORT REGULATOR MTR4).//1.9E-67//721aa//29%//Q09475
C-NT2RP4000498//MOB1 PROTEIN (MPS1 BINDER 1).//8.8E-50//214aa//50%//P40484
C-NT2RP4000500
C-NT2RP4000518//ATP-DEPENDENT RNA HELICASE ROK1.//1.5E-106//495aa//45%//P45818
C-NT2RP4000524
C-NT2RP4000541
C-NT2RP4000556//SUR4 PROTEIN (SRE1 PROTEIN).//7.4E-14//233aa//31%//P40319
C-NT2RP4000560
C-NT2RP4000588
C-NT2RP4000614//Homo sapiens TLS-associated protein TASR-2 mRNA, complete cds.//2.9E-188//863bp//99%//AF067730
C-NT2RP4000638
C-NT2RP4000648//TRANS-ACTING TRANSCRIPTIONAL PROTEIN ICP0.//0.00000037//175aa//27%//P09309
C-NT2RP4000657//SPORE COAT POLYSACCHARIDE BIOSYNTHESIS PROTEIN SPSE.//1.1E-32//350aa//30%//P39625
C-NT2RP4000704
C-NT2RP4000713//HYPOTHETICAL 55.1 KD PROTEIN B0416.5 IN CHROMOSOME X.//1.1E-13//295aa//27%//Q11073
C-NT2RP4000724//RETROVIRUS-RELATED ENV POLYPROTEIN.//3.2E-191//199aa//78%//P10267
C-NT2RP4000728//Homo sapiens mRNA for KIAA0931 protein, partial cds.//0//3392bp//95%//AB023148
C-NT2RP4000737
C-NT2RP4000739//Homo sapiens mRNA for KIAA1012 protein, complete cds.//0//3574bp//99%//AB023229
C-NT2RP4000781//HYPOTHETICAL 27.7 KD PROTEIN IN CPT1-SPC98 INTERGENIC REGION.//0.000000032//67aa//31%//P53915
C-NT2RP4000817//Homo sapiens mRNA for KIAA0470 protein, complete cds.//0//1927bp//99%//AB007939
C-NT2RP4000833
C-NT2RP4000837//Homo sapiens mRNA for zinc finger protein SALL1.//4.3E-94//810bp//65%//Y18265
C-NT2RP4000839//VEGETATIBLE INCOMPATIBILITY PROTEIN HET-E-1.//8.5E-21//271 aa//28%//Q00808
C-NT2RP4000855//AMINOPEPTIDASE B (EC 3.4.11.6) (ARGINYL AMINOPEPTIDASE)(ARGININE AMINOPEPTIDASE) (CYTOSOL AMINOPEPTIDASE IV)(AP-B).//5.7E-82//324aa//48%//O09175
C-NT2RP4000865//ZINC FINGER PROTEIN ZFP-36 (FRAGMENT).//4.1E-85//174aa//55%//P16415
C-NT2RP4000878//MYELOID UPREGULATED PROTEIN.//6.2E-91//173aa//87%//O35682
C-NT2RP4000879//UBIQUITIN-ACTIVATING ENZYME E1 (A1S9 PROTEIN).//9.6E-96//513aa//42%//P22314
C-NT2RP4000925//FIBROMODULIN PRECURSOR (FM) (COLLAGEN-BINDING 59 KD PROTEIN).//2.6E-26//227aa//36%//Q06828
C-NT2RP4000927//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//1.5E-76//346aa//43%//Q61068
C-NT2RP4000928//Homo sapiens mRNA for CDS2 protein.//0//2487bp//99%//Y16521
C-NT2RP4000929//PUTATIVE ATP-DEPENDENT RNA HELICASE MJ1505.//0.00000014//185aa//25%//Q58900
C-NT2RP4000955
C-NT2RP4000973//PROBABLE PROTEIN DISULFIDE ISOMERASE P5 PRECURSOR (EC 5.3.4.1).//1.4E-26//90aa//42%//P38660
C-NT2RP4000975
C-NT2RP4000979
C-NT2RP4000984
C-NT2RP4000989//UNC-47 PROTEIN.//0.0000082//173aa//25%//P34579
C-NT2RP4000997//DNA-DIRECTED RNA POLYMERASE 1135 KD POLYPEPTIDE (EC 2.7.7.6) (RNA POLYMERASE I SUBUNIT 2) (RPA135).//0//838aa//87%//P70700
C-NT2RP4001004//VACUOLAR PROTEIN 8.//3.7E-16//401aa//26%//P39968
C-NT2RP4001006
C-NT2RP4001010//Homo sapiens mRNA for KIAA0964 protein, complete cds.//0//2482bp//99%//AB023181
C-NT2RP4001041//PROBABLE LEUCYL-TRNA SYNTHETASE (EC 6.1.1.4) (LEUCINE--TRNA LIGASE)//1.5E-92//443aa//44%//Q09996
C-NT2RP4001057
C-NT2RP4001064//SYNAPTONEMAL COMPLEX PROTEIN SC65.//6.7E-51//335aa//37%//Q64375
C-NT2RP4001079//CALCIUM-TRANSPORTING ATPASE 1 (EC 3.6.1.38) (GOLGI CA2⁺-ATPASE).//1.3E-123//563aa//46%//P13586
C-NT2RP4001080//Homo sapiens mRNA for Rodi, complete cds.//0//1439bp//99%//AB023967
C-NT2RP4001086
C-NT2RP4001095//DOUBLE-STRANDED RNA-SPECIFIC EDITASE 1 (EC 3.5.-.-) (DSRNA ADENOSINE DEAMINASE) (RNA EDITING ENZYME 1).//2.6E-17//121aa//36%//P51400
C-NT2RP4001100
C-NT2RP4001117//PROTEIN TRANSPORT PROTEIN SEC61 ALPHA SUBUNIT.//1.9E-115//224aa//100%//P38378
C-NT2RP4001122//TIPD PROTEIN.//1.4E-65//253aa//41%//O15736
C-NT2RP4001126//TRICHOHYALIN.//2.9E-18//380aa//26%//Q07283
C-NT2RP4001138
C-NT2RP4001143//SUCCINYL-DIAMINOPIMELATE DESUCCINYLASE (EC 3.5.1.18) (SDAP).//0.00000021//93aa//33%//P44514
C-NT2RP4001148//SOF1 PROTEIN.//1.3E-104//236aa//52%//P33750
C-NT2RP4001149
C-NT2RP4001150//NG-CAM RELATED CELL ADHESION MOLECULE PRECURSOR (NR-CAM) (BRAVO).//3.4E-29//385aa//29%//P35331
C-NT2RP4001174//NON-GREEN PLASTID TRIOSE PHOSPHATE TRANSLOCATOR PRECURSOR (CTPT).//4.7E-29//227aa//35%//P52178
C-NT2RP4001206//Drosophila melanogaster strawberry notch (sno) mRNA, complete cds.//4.4E-104//1460bp//65 %//U95760
C-NT2RP4001207
C-NT2RP4001210
C-NT2RP4001219//PROBABLE PROTEIN DISULFIDE ISOMERASE P5 PRECURSOR (EC 5.3.4.1).//6.2E-27//90aa//42%//P38660
C-NT2RP4001228//RING CANAL PROTEIN (KELCH PROTEIN).//1.8E-103//508aa//43%//Q04652
C-NT2RP4001235
C-NT2RP4001256
C-NT2RP4001260//Homo sapiens mRNA for KIAA0875 protein, partial cds.//0//2876bp//99%//AB020682
C-NT2RP4001274//Human transporter protein (g17) mRNA, complete cds.//4.4E-58//1196bp//61%//U49082
C-NT2RP4001276//TRICHOHYALIN.//7.9E-09//126aa//32-%//Q07283
C-NT2RP4001313//MITOCHONDRIAL IMPORT RECEPTOR SUBUNIT TOM40 (MOM38 PROTEIN) (TRANSLOCASE OF OUTER MEMBRANE 40 KD SUBUNIT).//5.9E-17//296aa//29%//P24391
C-NT2RP4001315//Bos taurus mRNA for Rab5 GDP/GTP exchange factor, Rabex5.//8.5E-213//1129bp//92%//AJ001119
C-NT2RP4001339//Homo sapiens mRNA for AMMERC1 protein.//9.2E-160//736bp//99%//AJ007014
C-NT2RP4001343
C-NT2RP4001345//Homo sapiens mRNA for LCAT-like lysophospholipase (LLPL), complete cds.//2.7e-310//1400bp//100%//AB017494
C-NT2RP4001351//Human ovarian cancer downregulated myosin heavy chain homolog (Doc1) mRNA, complete cds.//1.4E-58//2425bp//59%//U53445
C-NT2RP4001353
C-NT2RP4001372//IRREGULAR CHIASM C-ROUGHEST PROTEIN PRECURSOR (IRREC PROTEIN).//1.6E-19//222aa//30%//Q08180
C-NT2RP4001373
C-NT2RP4001375//NON-RECEPTOR TYROSINE KINASE SPORE LYSIS A (EC 2.7.1.112) (TYROSINE- PROTEIN KINASE 1).//9.2E-17//146aa//35%//P18160
C-NT2RP4001379//HYPOTHETICAL 49.1 KD PROTEIN C11D3.06 IN CHROMOSOME I.//2E-53//436aa//30%//Q10085
C-NT2RP4001407//Homo sapiens mRNA for KIAA0923 protein, complete cds.//0//2716bp//99%//AB023140
C-NT2RP4001414//SEPTIN 2 HOMOLOG (FRAGMENT).//7.7E-190//422aa//82%//Q14141
C-NT2RP4001433//ZINC FINGER PROTEIN 184 (FRAGMENT).//1.2E-138//419aa//54%//Q99676
C-NT2RP4001474//Xenopus laevis putative Zic3 binding protein mRNA, complete cds.//2.7E-66//738bp//71%//AF129131
C-NT2RP4001483//2-OXOGLUTARATE DEHYDROGENASE E1 COMPONENT PRECURSOR (EC 1.2.4.2) (ALPHA-KETOGLUTARATE DEHYDROGENASE).//0//962aa//78%//Q02218
C-NT2RP4001498//ANKYRIN REPEAT-CONTAINING PROTEIN AKR1.//1E-27//374aa//29%//P39010
C-NT2RP4001502
C-NT2RP4001507
C-NT2RP4001524
C-NT2RP4001547//HYPOTHETICAL 45.0 KD PROTEIN IN NOT1/CDC39-HMR INTERGENIC REGION.//5.7E-54//242aa//3 8%//P25656
C-NT2RP4001551//Homo sapiens chromatin-specific transcription elongation factor FACT 140 kDa subunit mRNA, complete cds.//0//3202bp//99%//AF152961
C-NT2RP4001555//PUTATIVE ENDONUCLEASE VIII (EC 3.2.-.-).//4.7E-09//216aa//24%//P96902
C-NT2RP4001567//ARMADILLO SEGMENT POLARITY PROTEIN.//0.00000054//213aa//26,%//Q02453
C-NT2RP4001568//ZINC FINGER PROTEIN GCS1.//1.8E-10//109aa//36%//P35197
C-NT2RP4001571
C-NT2RP4001574//Homo sapiens coat protein gamma-cop mRNA, complete cds.//0//3046bp//99%//AF100756
C-NT2RP4001575//Rattus norvegicus mRNA for ARE1 protein.//0//1087bp//87%//AJ223830
C-NT2RP4001592//ISOLEUCYL-TRNA SYNTHETASE (EC 6.1.1.5) (ISOLEUCINE--TRNA LIGASE) (ILERS).//1.7E-141//373aa//47%//P73505
C-NT2RP4001610//Homo sapiens mRNA for KIAA0869 protein, partial cds.//0//1897bp//99%//AB020676
C-NT2RP4001614
C-NT2RP4001634
C-NT2RP4001638//DNA REPAIR/TRANSCRIPTION PROTEIN MET18/MMS19.//5.1E-46//234aa//32%//P40469
C-NT2RP4001644//MYOSIN LIGHT CHAIN KINASE (EC 2.7.1.117) (MLCK).//6.4E-19//111aa//45%//P25323
C-NT2RP4001677
C-NT2RP4001679
C-NT2RP4001696//CLEAVAGE AND POLYADENYLATION SPECIFICITY FACTOR, 100 KD SUBUNIT (CPSF 100 KD SUBUNIT).//4E-10//243aa//25%//Q10568
C-NT2RP4001725//GUANINE NUCLEOTIDE-BINDING PROTEIN BETA SUBUNIT.//3E-10//128aa//32%//Q10282
C-NT2RP4001730//UDP-GLUCOSE:GLYCOPROTEIN GLUCOSYLTRANSFERASE PRECURSOR (EC 2.4.1.-) (DUGT).//6.4E-170//1168aa//33%//Q09332
C-NT2RP4001739
C-NT2RP4001753//ZINC FINGER PROTEIN 84 (ZINC FINGER PROTEIN HPF2).//3.9E-236//665aa//58%//P51523
C-NT2RP4001760//PUTATIVE RHO/RAC GUANINE NUCLEOTIDE EXCHANGE FACTOR (RHO/RAC GEF) (FACIOGENITAL DYSPLASIA PROTEIN).//4.1E-16//263aa//27%//P98174
C-NT2RP4001790//Homo sapiens mRNA for KIAA1015 protein, complete cds.//0//3144bp//99%//AB023232
C-NT2RP4001803
C-NT2RP4001822//PLATELET-ENDOTHELIAL TETRASPAN ANTIGEN 3 (PETA-3) (GP27) (MEMBRANE GLYCOPROTEIN SFA-1) (CD151 ANTIGEN).//1.2E-30//241aa//30%//O35566
C-NT2RP4001823//MICROFIBRIL-ASSOCIATED GLYCOPROTEIN 4.//1.1E-19//77aa//54%//P55083
C-NT2RP4001828
C-NT2RP4001838//Homo sapiens CoREST protein (COREST) mRNA, complete cds.//6.3E-99//555.bp//73%//AF155595
C-NT2RP4001861//TRICHOHYALEN.//1E-35//307aa//34%//P37709
C-NT2RP4001893//Homo sapiens mRNA; cDNA DKFZp5640043 (from clone DKFZp5640043).//0//1306bp//98%//AL050390
C-NT2RP4001896//VEGETATIBLE INCOMPATIBILITY PROTEIN HET-E1.//0.000000014//345aa//25%//Q00808
C-NT2RP4001901
C-NT2RP4001927//MICROTUBULE-ASSOCIATED PROTEIN YTM1.//1.3E-38//258aa//32%//Q12024
C-NT2RP400193 8//TRANSCRIPTIONAL REPRESSOR CTCF.//9.8E-60//303aa//38%//P49711
C-NT2RP4001946//PROTEIN-L-ISOASPARTATE O-METHYLTRANSFERASE (EC 2.1.1.77) (PROTEIN- BETA-ASPARTATE METHYLTRANSFERASE) (PIMT) (PROTEIN L-ISOASPARTYL METHYLTRANSFERASE) (L-ISOASPARTYL PROTEIN CARBOXYL METHYLTRANSFERASE).//1.5E-13//211aa//28%//Q43209
C-NT2RP4001950//GLUTAMIC ACID-RICH PROTEIN PRECURSOR.//1.2E-13//356aa//27%//P13816
C-NT2RP4001953
C-NT2RP4001966
C-NT2RP4001975
C-NT2RP4002018//RING CANAL PROTEIN (KELCH PROTEIN).//6.9E-24//370aa//27%//Q04652
C-NT2RP4002052
C-NT2RP4002058//PUTATIVE PRE-MRNA SPLICING FACTOR RNA HELICASE (DEAH BOX PROTEIN 13)//1E-137//679aa//40%//O43143
C-NT2RP4002071
C-NT2RP4002078//ZINC FINGER PROTEIN 91 (ZINC FINGER PROTEIN HTF10) (HPF7).//3E-150//722aa//39%//Q05481
C-NT2RP4002081//TRANSCRIPTION INITIATION FACTOR IIA ALPHA AND BETA CHAINS (TFIIA P35 AND P19 SUBUNITS) (TFIIA-42) (TFIIAL).//0.0000067//250aa//31%//P52655
C-NT2RP4002298
C-NT2RP4002408//PROTEIN KINASE CEK1 (EC 2.7.1.-).//1.5E-63//159aa//53%//P38938
C-NT2RP4002791
C-NT2RP4002888//Homo sapiens mRNA; cDNA DKFZp434Fl 72 (from clone DKFZp434F172).//0//2557bp//99%//AL080202
C-NT2RP4002905
C-NT2RP5003461//RLR1 PROTEIN.//9.7E-22//177aa//27%//P53552
C-NT2RP5003477//VEGETATIBLE INCOMPATIBILITY PROTEIN HET-E-1.//5.5E-15//280aa//27%//Q00808
C-NT2RP5003492
C-NT2RP5003500
C-NT2RP5003506
C-NT2RP5003522//NADPH-CYTOCHROME P450 REDUCTASE (EC 1.6.2.4) (CPR).//3.3E-23//219aa//40%//P37116
C-NT2RP5003524
C-NT2RP5003534
C-OVARC1000006//HISTONE H2A.1.//1.1E-55//117aa//99%//P02262
C-OVARC1000013//APOPTOTIC PROTEASE ACTIVATING FACTOR 1 (APAF-1).//0.0000042//102aa//32%//O14727
C-OVARC1000014//Homo sapiens GLE1 (GLE1) mRNA, complete cds.//2.6E-295//1393bp//97%//AF058922
C-OVARC1000035
C-OVARC1000060//EXTRACELLULAR RIBONUCLEASE LE PRECURSOR (EC 3.1.27.1) (RNASE LE).//0.00000032//60aa//45 %//P80022
C-OVARC1000087//HISTONE MACRO-H2A.1.//1.6E-12//174aa//26%//Q02874
C-OVARC1000091//HOST CELL FACTOR C1 (HCF) (VP16 ACCESSORY PROTEIN) (HFC1) (VCAF) (CFF).//8.4E-14//259aa//30%//P51610
C-OVARC1000113
C-OVARC1000139//Homo sapiens CGI-21 protein mRNA, complete cds.//0//1562bp//99%//AF132955
C-OVARC1000148
C-OVARC1000151//Homo sapiens partial mRNA for putative protein p38 interacting with transcription factor Sp1.//2.5E-95//461bp//98%//AJ242975
C-OVARC1000168
C-OVARC1000209//Oryza sativa submergence induced protein 2A mRNA, complete cds.//1.8E-32//511bp//65%//AF068332
C-OVARC1000212
C-OVARC1000241//HYPOXIA-INDUCIBLE FACTOR 1 ALPHA (HIF-1 ALPHA) (ARNT INTERACTING PROTEIN) (MEMBER OF PAS PROTEIN 1) (MOP1) (HIF1 ALPHA).//8.2E-120//351aa//54%//Q16665
C-OVARC1000288//VACUOLAR AMINOPEPTIDASE I PRECURSOR (EC 3.4.11.22) (POLYPEPTIDASE)(LEUCINE AMINOPEPTIDASE IV) (LAPIV) (AMINOPEPTIDASE III)(AMINOPEPTIDASE YSCI).//5.4E-53//384aa//30%//P14904
C-OVARC1000304//PROTEIN MOV-10.//1.1E-249//519aa//87%//P23249
C-OVARC1000309//THREONINE SYNTHASE (EC 4.2.99.2).//2.7E-40//154aa//38%//P29363
C-OVARC1000321
C-OVARC1000326
C-OVARC1000335//HYPOTHETICAL 39.3 KD PROTEIN IN GCN4-WBP1 INTERGENIC REGION.//5.9E-14//200aa//27%//P40004
C-OVARC1000347
C-OVARC1000384
C-OVARC1000411
C-OVARC1000420
C-OVARC1000437//TENSIN.//7.9E-181//340aa//84%//Q04205
C-OVARC1000443//Homo sapiens mRNA; cDNA DKFZp434A073 (from clone DKFZp434A073).//0//1216bp//99%//AL080126
C-OVARC1000461
C-OVARC1000465//PROTEIN TRANSPORT PROTEIN SEC7.//1.2E-25//227aa//25%//P11075
C-OVARC1000466
C-OVARC1000473//DUAL SPECIFICITY PROTEIN PHOSPHATASE 3 (EC 3.1.3.48) (EC 3.1.3.16) (DUAL SPECIFICITY PROTEIN PHOSPHATASE VHR).//3.1E-10//125aa//35%//P51452
C-OVARC1000479//Homo sapiens mRNA for KIAA0829 protein, partial cds.//0//1919bp//99%//AB020636
C-OVARC1000520//Homo sapiens supervillin mRNA, complete cds.//2.2E-157//892bp//91 %//AF051850
C-OVARC1000564
C-OVARC1000576
C-OVARC1000588
C-OVARC1000605
C-OVARC1000640
C-OVARC1000649//Human squamous cell carcinama of esophagus mRNA for GRB-7 SH2 domain protein, complete cds.//0//1812bp//98%//D43772
C-OVARC1000661
C-OVARC1000771//RAS-RELATED PROTEIN RAB-2.//1.1E-46//121aa//79%//P08886
C-OVARC1000959//HYPOTHETICAL PROTEIN MJ0933.//1.2E-17//127aa//33%//Q58343
C-OVARC1001034//Mus musculus Fn54 mRNA, partial cds.//1.5E-178//1113bp//86%//AF001533
C-OVARC1001038//Homo sapiens mRNA for Ariadne-2 protein.//01/1172bp//97%//AJ130978
C-OVARC1001065//Homo sapiens CGI-12 protein mRNA, complete cds.//1E-215//1027bp//98%//AF132946
C-OVARC1001162
C-OVARC1001243
C-OVARC1001296
C-OVARC1001360
C-OVARC1001381//Homo sapiens mRNA for candidate tumor suppressor involved in B-CLL.//6E-148//683bp//99%//AJ224819
C-OVARC1001425
C-PLACE1000005
C-PLACE1000066//SSU72 PROTEIN.//1.1E-39//206aa//43%//P53538
C-PLACE1000142//3-HYDROXYBUTYRYL-COA DEHYDRATASE (EC 4.2.1.55) (CROTONASE).//2.8E-29//134aa//43%//P52046
C-PLACE1000184//Homo sapiens mRNA for KIAA0832 protein, complete cds.//5.5e-312//1411bp//99%//AB020639
C-PLACE1000185
C-PLACE1000213//Homo sapiens mRNA for KIAA0977 protein, complete cds.//0//1904bp//99%//AB023194
C-PLACE1000347
C-PLACE1000374
C-PLACE1000380//Homo sapiens mRNA for KIAA0853 protein, partial cds.//0//2208bp//99%//AB020660
C-PLACE1000383//Homo sapiens mRNA for MTMR1 protein.//0//753bp//99%//AJ224979
C-PLACE1000401//POLIOVIRUS RECEPTOR PRECURSOR (CD155 ANTIGEN).//2.7E-30//352aa//31%//P15151
C-PLACE1000406//PTB-ASSOCIATED SPLICING FACTOR (PSF).//1.2E-132//334aa//72%//P23246
C-PLACE1000420//7.8-DIHYDRO-8-OXOGUANINE TRIPHOSPHATASE (EC 3.1.6.-) (8-OXO-DGTPASE).//0.0000028//134aa//29%//P53368
C-PLACE1000435
C-PLACE1000444
C-PLACE1000562
C-PLACE1000564
C-PLACE1000588//INTERFERON-INDUCED GUANYLATE-BINDING PROTEIN 1 (GUANINE NUCLEOTIDE- BINDING PROTEIN 1).//1.6E-270//437aa//86%//P32455
C-PLACE1000596//Homo sapiens mRNA for KIAA0850 protein, complete cds.//0//2393bp//99%//AB020657
C-PLACE1000611//Rattus norvegicus neural membrane protein 35 mRNA, complete cds.//2E-55//779bp//67%//AF044201
C-PLACE1000636//MALE STERILITY PROTEIN 2.//1.2E-39//261aa//27%//Q08891
C-PLACE1000716
C-PLACE1000748
C-PLACE1000755//Homo sapiens mRNA for Helicase-MOI, complete-cds.//4.6E-250//1189bp//97%//AB028449
C-PLACE1000785//Homo sapiens mRNA for KIAA0648 protein, partial cds.//0//2002bp//99%//AB014548
C-PLACE1000798
C-PLACE1000863//PUTATIVE MITOCHONDRIAL 40S RIBOSOMAL PROTEIN
YHR148W.//2.5E-49//181aa//54%//P32899
C-PLACE1000909//ANKYRIN REPEAT-CONTAINING PROTEIN AKR1.//2.6E-19//404aa//26%//P39010
C-PLACE1000948
C-PLACE1000972
C-PLACE1000977//BETA-CHIMAERIN (BETA-CHIMERIN).//4.4E-22//129aa//35%//Q03070
C-PLACE1001000
C-PLACE1001092//Homo sapiens sorting nexin 4 mRNA, complete cds.//0//1500bp//99%//AF065485
C-PLACE1001257//RING CANAL PROTEIN (KELCH PROTEIN).//4.3E-54//257aa//46%//Q04652
C-PLACE1001383//ZINC-FINGER PROTEIN UBI-D4 (APOPTOSIS RESPONSE ZINC FINGER PROTEIN REQUIEM).//3E-33//138aa//42%//Q61103
C-PLACE1001387//EPIDERMAL GROWTH FACTOR RECEPTOR KINASE SUBSTRATE EPS8.//2.3E-61//132aa//46%//Q12929
C-PLACE1001399//Homo sapiens chromosome 17, clone hRPK.22_N_12, complete sequence.//0//2118bp//99%//AC005412
C-PLACE1001412
C-PLACE1001484//Homo sapiens chromosome 20 clone 387E22, WORKING DRAFT SEQUENCE, in unordered pieces.//0//1440bp//99%//AL031660
C-PLACE1001503
C-PLACE1001570
C-PLACE1001610
C-PLACE1001692//S-ACYL FATTY ACID SYNTHASE THIOESTERASE, MEDIUM CHAIN (EC 3.1.2.14) (THIOESTERASE n).//4E-81//263aa//56%//P08635
C-PLACE1001729
C-PLACE1001739//PUTATIVE ATP-DEPENDENT RNA HEUCASE PL10.//3.5E-75//439aa//41%//P16381
C-PLACE1001781//PROBABLE PHOSPHOMANNOMUTASE (EC 5.4.2.8) (PMM).//5.4E-63//427aa//35%//Q57290
C-PLACE1001810
C-PLACE1001817//Homo sapiens ATP-specific succinyl-CoA synthetase beta subunit (SCS) mRNA, partial cds.//0//1995bp//99%//AF058953
C-PLACE1001869//L-RIBULOKINASE (EC 2.7.1.16).//2E-27//270aa//31%//P94524
C-PLACE1001912//Homo sapiens clone 24963 mRNA sequence, complete cds.//0//1196bp//99%//AF131737
C-PLACE1001920//Homo sapiens MDC-3.13 isoform 2 mRNA, complete cds.//0//1729bp//99%//AF099935
C-PLACE1001928
C-PLACE1001989//PUTATIVE AMIDASE (EC 3.5.1.4).//1.4E-78//496aa//37%//Q49091
C-PLACE1002046//LIGATIN (FRAGMENT).//1.7E-240//560aa//80%//Q61211
C-PLACE1002072
C-PLACE1002073//ADENYLATE CYCLASE (EC 4.6.1.1) (ATP PYROPHOSPHATE-LYASE) (ADENYLYL CYCLASE).//0.00000053//188aa//29%//P49606
C-PLACE1002140
C-PLACE1002163
C-PLACE1002170
C-PLACE1002433
C-PLACE1002438//ZINC FINGER PROTEIN 151 (MIZ-1 PROTEIN).//0.0000042//133aa//29%//Q13105
C-PLACE1002465
C-PLACE1002529//Homo sapiens mRNA for KIAA0713 protein, partial cds.//6.7E-214//956bp//94%//AB018256
C-PLACE1002685//Homo sapiens B cell linker protein BLNK mRNA, alternatively spliced, complete cds.//0//1750bp//99%//AF068180
C-PLACE1002722//PROBABLE G PROTEIN-COUPLED RECEPTOR KIAA0001.//9E-45//305aa//33%//Q15391
C-PLACE1002794
C-PLACE1002815
C-PLACE1002839
C-PLACE1002851
C-PLACE1002941
C-PLACE1002996
C-PLACE1003045
C-PLACE1003092
C-PLACE1003100//HEP27 PROTEIN (PROTEIN D).//2.6E-79//253aa//60%//Q13268
C-PLACE1003108
C-PLACE1003145
C-PLACE1003174//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//3.8E-37//143aa//51%//P42743
C-PLACE1003190//SOF1 PROTEIN.//1.9E-110//325aa//48%//P33750
C-PLACE1003200
C-PLACE1003296//Homo sapiens mRNA; cDNA DKFZp434G173 (from clone DKFZp434G173).//0//1706bp//99%//AL080133
C-PLACE1003302//ZINC FINGER PROTEIN 83 (ZINC FINGER PROTEIN HPF1).//6.9E-206//396aa//86%//P51522
C-PLACE1003334
C-PLACE1003342
C-PLACE1003353//Homo sapiens breast cancer antiestrogen resistance 3 protein (BCAR3) mRNA, complete cds.//0//2435bp//99%//U92715
C-PLACE1003369
C-PLACE1003602//Homo sapiens mRNA expressed in placenta.//5.9E-278//1275bp//99%//D83200
C-PLACE1003611
C-PLACE1003625//ARMADILLO SEGMENT POLARITY PROTEIN.//3.2E-10//380aa//25%//P18824
C-PLACE1003704//SPLICING FACTOR, ARGININE/SERINE-RICH 4 (PRE-MRNA SPLICING FACTOR SRP75).//8E-19//209aa//34%//Q08170
C-PLACE1003711
C-PLACE1003723
C-PLACE1003762
C-PLACE1003771
C-PLACE1003784
C-PLACE1003923
C-PLACE1003936
C-PLACE1003968//5'-AMP-ACTIVATED PROTEIN KINASE, GAMMA-1 SUBUNIT (AMPK GAMMA-1 CHAIN).//2.4E-124//326aa//73%//P80385
C-PLACE1004104
C-PLACE1004114
C-PLACE1004128//GUANINE NUCLEOTIDE-BINDING PROTEIN BETA SUBUNIT 4 (TRANSDUCIN BETA CHAIN 4).//6.1E-181//340aa//96%//P29387
C-PLACE1004149
C-PLACE1004156
C-PLACE1004161
C-PLACE1004183//Homo sapiens for TOM1-like protein.//0//1279bp//97%//AJ010071
C-PLACE1004197//BUTYROPHILIN PRECURSOR (BT).//4.5E-10//208aa//27%//Q62556
C-PLACE1004203//Homo sapiens GPI-anchored membrane protein CDw108 precursor, mRNA, complete cds.//0//1882bp//99%//AF069493
C-PLACE1004258
C-PLACE1004270//TRANSMEMBRANE PROTEASE, SERINE 2 (EC 3.4.21.-).//9.7E-36//389aa//31%//O15393
C-PLACE1004277//Homo sapiens two pore domain K⁺ channel (TASK-2) mRNA, complete cds.//0//1498bp//99%//AF084830
C-PLACE1004289
C-PLACE1004302//SOF1 PROTEIN.//1.9E-110//325aa//48%//P33750
C-PLACE1004316//H.sapiens mRNA for apoptosis specific protein.//0//1767bp//99%//Y11588
C-PLACE1004358//Homo sapiens connector enhancer of KSR-like protein CNK1 mRNA, complete cds.//0//2512bp//99%//AF100153
C-PLACE1004376
C-PLACE1004388
C-PLACE1004405
C-PLACE1004428//PRISTANOYL-COA OXIDASE (EC 1.3.3.-).//1.2E-39//385aa//33%//Q63448
C-PLACE1004437//Human NAD⁺-specific isocitrate dehydrogenase beta subunit precursor, mRNA, nuclear gene encoding mitochondrial protein, complete cds.//0//985bp//99%//U49283
C-PLACE1004451
C-PLACE1004460//MATERNAL TUDOR PROTEIN.//0.0000002//218aa//23%//P25823
C-PLACE1004473
C-PLACE1004510//Homo sapiens cofactor of initiator function (CIF150) mRNA, complete//1.3E-209//954bp//99%//AF026445
C-PLACE1004516
C-PLACE1004548
C-PLACE1004564//CLEAVAGE AND POLYADENYLATION SPECIFICITY FACTOR, 100
KD SUBUNIT (CPSF 100 KD SUBUNIT).//0//525aa//99%//Q10568
C-PLACE1004629//PROTEIN OS-9 PRECURSOR.//7.7E-18//264aa//32%//Q13438
C-PLACE1004645
C-PLACE1004646//B.taurus mRNA for retinal pigment epithelial membrane receptor p63.//4.4E-42//985bp//59%//X66277
C-PLACE1004664
C-PLACE1004672
C-PLACE1004674//PROBABLE CALCIUM-BINDING PROTEIN ALG-2 (PMP41) (ALG-257).//1.6E-95//191aa//96%//P12815
C-PLACE1004691
C-PLACE1004722
C-PLACE1004736
C-PLACE1004740
C-PLACE1004743//PROBABLE N-END-RECOGNIZING PROTEIN (UBIQUITIN-PROTEIN LIGASE E3 COMPONENT) (N- RECOGNIN).//4.4E-35//578aa//27%//O60152
C-PLACE1004751//Homo sapiens mRNA for alpha2,3-sialyltransferase ST3Gal VI, complete cds.//7.1E-224//790bp//98%//AB022918
C-PLACE1004777//N-CHIMAERIN (NC) (N-CHIMERIN) (ALPHA CHIMERIN) (A-CHIMAERIN).//1.9E-32//259aa//32%//P30337
C-PLACE1004804//ADENYLATE CYCLASE (EC 4.6.1.1) (ATP PYROPHOSPHATE-LYASE) (ADENYLYL CYCLASE).//4.7E-65//695aa//29%//Q01631
C-PLACE1004814//SPLICING FACTOR, ARGININE/SERINE-RICH 4 (PRE-MRNA SPLICING FACTOR SRP75).//5.9E-19//196aa//36%//Q08170
C-PLACE1004824
C-PLACE1004868//MALE STERILITY PROTEIN 2.//3.9E-39//261aa//27%//Q08891
C-PLACE1004885
C-PLACE1004902//PUTATIVE PRE-MRNA SPLICING FACTOR ATP-DEPENDENT RNA HELICASE SPAC10F6.02C.//9.3E-11//94aa//47%//O42643
C-PLACE1004918//L-LACTATE DEHYDROGENASE M CHAIN (EC 1.1.1.27) (LDHA).//4.9E-48//198aa//44%//P06151
C-PLACE1004930//Homo sapiens MDC-3.13 isoform 2 mRNA, complete cds//0//1853bp//98%//AF099936
C-PLACE1004934
C-PLACE1004937//SEL-10 PROTEIN.//6.3E-125//357aa//58%//Q93794
C-PLACE1004969//HYPOTHETICAL 55.1 KD PROTEIN B0416.5 IN CHROMOSOME X.//2E-14//205aa//26%//Q11073
C-PLACE1004982
C-PLACE1005026
C-PLACE1005027
C-PLACE1005046
C-PLACE1005077
C-PLACE1005101//Homo sapiens (clone zap128) mRNA, 3' end of cds.//1E-209//1031bp//96%//L40401
C-PLACE1005102//RING CANAL PROTEIN (KELCH PROTEIN).//2.6E-56//565aa//30%//Q04652
C-PLACE1005111
C-PLACE1005181
C-PLACE1005187//APAG PROTEIN.//3.8E-13//122aa//36%//P05636
C-PLACE1005206
C-PLACE1005232
C-PLACE1005243//SERINE/THREONINE PROTEIN KINASE PKPA (EC 2.7.1.-).//1.3E-27//349aa//32%//Q01577
C-PLACE1005261
C-PLACE1005266
C-PLACE1005277//Homo sapiens mRNA for KIAA0610 protein, partial cds.//3.2E-297//1341bp//100%//AB011182
C-PLACE1005287//INNER CENTROMERE PROTEIN (INCENP).//2.3E-13//269aa//28%//P53352
C-PLACE1005305//GTP:AMP PHOSPHOTRANSFERASE MITOCHONDRIAL (EC 2.7.4.10) (AK3).//2E-111//226aa//92%//P08760
C-PLACE1005308
C-PLACE1005313
C-PLACE1005327
C-PLACE1005335
C-PLACE1005373//TRNA PSEUDOURIDINE SYNTHASE B (EC 4.2.1.70) (TRNA PSEUDOURIDINE 55 SYNTHASE) (PSI55 SYNTHASE) (PSEUDOURIDYLATE SYNTHASE) (URACIL HYDROLYASE).//8.6E-09//194aa//27%//O33335
C-PLACE1005374
C-PLACE1005480
C-PLACE1005481
C-PLACE1005494//Homo sapiens mRNA for transient receptor potential protein TRP6.//0//1649bp//99%//AJ006276
C-PLACE1005530//HYPOTHETICAL 47.6 KD PROTEIN C16C10.5 IN CHROMOSOME III.//5.6E-52//173aa//57%//Q09251
C-PLACE1005550
C-PLACE1005554
C-PLACE1005623
C-PLACE1005646//Homo sapiens RNA helicase-related protein mRNA, complete cds.//0//2130bp//99%//AF083255
C-PLACE1005656//RIBONUCLEOSIDE-DIPHOSPHATE REDUCTASE M2 CHAIN (EC 1.17.4.1) (RIBONUCLEOTIDE REDUCTASE).//2.1E-148//321aa//83%//P31350
C-PLACE1005730
C-PLACE1005755
C-PLACE1005763//S-ACYL FATTY ACID SYNTHASE THIOESTERASE, MEDIUM CHAIN (EC 3.1.2.14) (THIOESTERASE II).//2.5E-79//209aa//53%//P08635
C-PLACE1005803
C-PLACE1005804//Homo sapiens alpha 1,2-mannosidase IB mRNA, complete cds.//1.1E-217//994bp//99%//AF027156
C-PLACE1005851
C-PLACE1005921//AIG1 PROTEIN.//3E-31//284aa//31%//P54120
C-PLACE1005923
C-PLACE1005925
C-PLACE1005934
C-PLACE1005936
C-PLACE1005951
C-PLACE1005953//GLYCOSYLTRANSFERASE ALG2 (EC 2.4.1.-).//6.7E-30//198aa//37%//P43636
C-PLACE1005955//VACUOLAR AMINOPEPTIDASE I PRECURSOR (EC 3.4.11.22) (POLYPEPTIDASE)//5.4E-54//455aa//32%//P14904
C-PLACE1005966//TRANSCRIPTION INITIATION FACTOR TFIID 90 KD SUBUNIT (TAFII-90).//0.00000014//254aa//25%//P38129
C-PLACE1005990
C-PLACE1006011//Homo sapiens mRNA for poly(ADP-ribose) polymerase-2.//0//1564bp//99%//AJ236876
C-PLACE1006040//Homo sapiens mRNA for alpha endosulfine.//4.7E-161//744bp//99%//X99906
C-PLACE1006119//Homo sapiens Ran-GTP binding protein mRNA, partial cds.//1.5E-148//681bp//99%//AF039023
C-PLACE1006139
C-PLACE1006159
C-PLACE1006167
C-PLACE1006170//Homo sapiens mRNA for KIAA0899 protein, partial cds.//4.5E-293//953bp//99%//AB020706
C-PLACE1006195
C-PLACE1006196//PUTATIVE ATP-DEPENDENT RNA HELICASE C12C2.06.//2.7E-116//496aa//48%//Q09747
C-PLACE1006225
C-PLACE1006236
C-PLACE1006239//BONE PROTEOGLYCAN II PRECURSOR (PG-S2) (DECORIN).//2E-16//244aa//31%//P28675
C-PLACE1006246
C-PLACE1006325//Homo sapiens mRNA; cDNA DKFZp564J142 (from clone DKFZp564J142).//3.8E-278//1271-bp//99%//AL080066
C-PLACE1006335
C-PLACE1006357
C-PLACE1006385//Homo sapiens epsin 2a mRNA, complete cds.//0//1168bp//99%//AF062085
C-PLACE1006412
C-PLACE1006414
C-PLACE1006438//ZINC FINGER PROTEIN 165.//2.5E-45//122aa//43%//P49910
C-PLACE1006445
C-PLACE1006470
C-PLACE1006482//TRANSCRIPTION FACTOR MAFF.//7.7E-55//142aa//85%//Q90595
C-PLACE1006488//SIGNAL RECOGNITION PARTICLE 68 KD PROTEIN (SRP68).//1.1E-229//367aa//96%//Q00004
C-PLACE1006492
C-PLACE1006531
C-PLACE1006552
C-PLACE1006598//Homo sapiens clone NH0310K15, WORKING DRAFT SEQUENCE, 4 unordered pieces.//0//2182bp//99%//AC007383
C-PLACE1006615
C-PLACE1006626//Homo sapiens mRNA for KIAA0928 protein, partial cds.//0//1760bp//99%//AB023145
C-PLACE1006673
C-PLACE1006678//Homo sapiens mRNA for type II membrane protein, complete cds, clone:HP10328.//5.8E-24//734bp//62%//AB015630
C-PLACE1006704
C-PLACE1006731//RIBOFLAVIN KINASE (EC 2.7.1.26) (FLAVOKINASE) / FMN ADENYLYLTRANSFERASE (EC 2.7.7.2) (FAD PYROPHOSPHORYLASE) (FAD SYNTHETASE).//6.9E-13//177aa//33%//Q59263
C-PLACE1006782
C-PLACE1006819//LINE-1 REVERSE TRANSCRIPTASE HOMOLOG.//9.8E-213//232aa//80%//P08547
C-PLACE1006829//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN).//2E-15//188aa//29%//P35123
C-PLACE1006883
C-PLACE1006901
C-PLACE1006917//HSH49 PROTEIN.//5.5E-12//97aa//35%//Q99181
C-PLACE1006932
C-PLACE1006935//HYPOTHETICAL 95.2 KD PROTEIN R144.6 IN CHROMOSOME III.//6.7E-48//278aa//41%//Q10000
C-PLACE1006956//ATP-DEPENDENT PERMEASE MDL1.//1.3E-86//522aa//36%//P97998
C-PLACE1006958//Homo sapiens mRNA for heat shock protein apg-1, complete cds.//0//1770bp//99%//AB023421
C-PLACE1006961
C-PLACE1006962
C-PLACE1006966
C-PLACE1007014//36 KD NUCLEOLAR PROTEIN HNP36 (DELAYED-EARLY RESPONSE PROTEIN 12) (DER12).//3.2E-35//180aa//33%//Q14542
C-PLACE1007021
C-PLACE1007105
C-PLACE1007178
C-PLACE1007226//PROBABLE OXYGEN-INDEPENDENT COPROPORPHYRINOGEN III OXIDASE (EC 1.-.-.-) (COPROPORPHYRINOGENASE) (COPROGEN OXIDASE).//1E-42//370aa//31%//P54304
C-PLACE1007238
C-PLACE1007239//Homo sapiens mRNA for transcription elongation factor S-II, hS-II-T1, complete cds.//6.5E-216//1068bp//96%//D50495
C-PLACE1007242
C-PLACE1007243//UNC-47 PROTEIN.//0.00000017//211aa//27%//P34579
C-PLACE1007257//Homo sapiens mRNA for dia-12c protein.//0//2052bp//99%//Y15908
C-PLACE1007274
C-PLACE1007282
C-PLACE1007301
C-PLACE1007317//Drosophila melanogaster Adrift (adrift) mRNA, complete cds.//4.1E-17//1037bp//56%//AF117649
C-PLACE1007342
C-PLACE1007346//Homo sapiens estrogen-responsive B box protein (EBBP) mRNA, complete cds.//0//2366bp//99%//AF096870
C-PLACE1007367
C-PLACE1007375//PHORBOL ESTER/DIACYLGLYCEROL-BINDING PROTEIN UNC-13.//0.00000044//127aa//30%//P27715
C-PLACE1007386
C-PLACE1007402
C-PLACE1007409//WHITE PROTEIN.//1.1E-64//428aa//32%//Q17320
C-PLACE1007416//DIPEPTIDYL PEPTIDASE IV (EC 3.4.14.5) (DPP IV) (T-CELL ACTIVATION ANTIGEN CD26) (TP103) (ADENOSINE DEAMINASE COMPLEXING PROTEIN-2) (ADABP).//8.8E-25//140aa//35%//P27487
C-PLACE1007450
C-PLACE1007452
C-PLACE1007460
C-PLACE1007484
C-PLACE1007488//PUTATIVE RHO/RAC GUANINE NUCLEOTIDE EXCHANGE FACTOR (RHO/RAC GEF) (FACIOGENITAL DYSPLASIA PROTEIN HOMOLOG).//5.4E-53//426aa//33%//P52734
C-PLACE1007507
C-PLACE1007511//KERATIN, TYPE I CYTOSKELETAL 19 (CYTOKERATIN 19) (K19) (CK 19).//1.4E-85//385aa//45%//P08728
C-PLACE1007524
C-PLACE1007537//Homo sapiens ankyrin repeat-containing protein ASB-2 mRNA, complete cds.//8.9e-316//1485bp//98%//AF159164
C-PLACE1007544
C-PLACE1007547//HYPOTHETICAL 97.1 KD PROTEIN R05D3.4 IN CHROMOSOME III.//1E-49//361aa//36%//P34537
C-PLACE1007583
C-PLACE1007598//ZINC FINGER PROTEIN 184 (FRAGMENT).//1.6E-143//666aa//44%//Q99676
C-PLACE1007618//Homo sapiens mRNA for KIAA0977 protein, complete cds.//0//713bp//99%//AB023194
C-PLACE1007621
C-PLACE1007632//POLIOVIRUS RECEPTOR PRECURSOR.//0.0000001//228aa//31%//P32506
C-PLACE1007645
C-PLACE1007649//Homo sapiens mRNA for KIAA0977 protein, complete cds.//0//1952-bp//99%//AB023194
C-PLACE1007688//LA PROTEIN HOMOLOG (LA RIBONUCLEOPROTEIN) (LA AUTOANTIGEN HOMOLOG).//8.7E-09//279aa//28%//Q26457
C-PLACE1007690
C-PLACE1007697//GCN20 PROTEIN.//7.6E-119//717aa//38%//P43535
C-PLACE1007706//Homo sapiens metalloprotease 1 (MP1) mRNA, complete cds.//0//3431bp//99%//AF061243
C-PLACE1007725
C-PLACE1007729//RETROVIRUS-RELATED PROTEASE (EC 3.4.23.-).//1.5E-44//231aa//42%//P10265
C-PLACE1007730//Homo sapiens mRNA for KIAA0685 protein, complete cds.//9.2E-294//1504bp//94%//AB014585
C-PLACE1007746
C-PLACE1007791//Homo sapiens IDN3-B mRNA, complete cds.//0//1836bp//99%//AB019602
C-PLACE1007810
C-PLACE1007843
C-PLACE1007846//Homo sapiens genomic DNA of 21q22.2 Down Syndrome region, segment 3/13.//0//1751bp//99%//AP000010
C-PLACE1007858//Homo sapiens mRNA for KIAA0766 protein, complete cds.//0//3112bp//99%//AB018309
C-PLACE1007897
C-PLACE1007946//MYOSIN HEAVY CHAIN, NON-MUSCLE (ZIPPER PROTEIN) (MYOSIN II).//2.6E-14//370aa//25%//Q99323
C-PLACE1007954
C-PLACE1007955//Homo sapiens cyclin-D binding Myb-like protein mRNA, complete cds.//0//2252bp//99%//AF084530
C-PLACE1007958//Homo sapiens cAMP-specific phosphodiesterase 8B (PDE8B) mRNA, partial cds.//0//2300bp//99%//AF079529
C-PLACE1007969//HETEROGENEOUS NUCLEAR RIBONUCLEOPROTEIN M (HNRNP M).//1.1E-36//202aa//48%//P52272
C-PLACE1007990
C-PLACE1008000//CHANNEL ASSOCIATED PROTEIN OF SYNAPSE-110 (CHAPSYN-110) (SYNAPTIC DENSITY PROTEIN PSD-93).//6.1E-14//128aa//39%//Q63622
C-PLACE1008002//Homo sapiens clone DJ0613C23, WORKING DRAFT SEQUENCE, 4 unordered pieces.//0//1833bp//99%//AC005628
C-PLACE1008044//NUCLEAR PORE COMPLEX PROTEIN NUP107 (NUCLEOPORIN NUP107) (107 KD NUCLEOPORIN) (P105).//4.6e-318//613aa//94%//P52590
C-PLACE1008095
C-PLACE1008122
C-PLACE1008129
C-PLACE1008132//HYPOTHETICAL 127.4 KD PROTEIN F07F6.4 IN CHROMOSOME III.//1.3E-24//395aa//31%//Q09531
C-PLACE1008177/TRICHOHYALIN.//2.3E-29//487aa//26%//P37709
C-PLACE1008209
C-PLACE1008273//COATOMER GAMMA SUBUNIT (GAMMA-COAT PROTEIN) (GAMMA-COP).//1.3E-283//671aa//77%//P53620
C-PLACE1008275//DNA REPAIR PROTEIN REV1 (EC 2.7.7.-).//2.3E-18//162aa//37%//P12689
C-PLACE1008280
C-PLACE1008309
C-PLACE1008329
C-PLACE1008356//Homo sapiens mRNA for KIAA0679 protein, partial cds.//0//1853bp//100%//AB014579
C-PLACE1008398//GENE 33 POLYPEPTIDE.//7.3E-114//243aa//87%//P05432
C-PLACE1008401
C-PLACE1008402//GENERAL VESICULAR TRANSPORT FACTOR P115 (TRANSCYTOSIS ASSOCIATED PROTEIN) (TAP).//0//698aa//95%//P41541
C-PLACE1008429//ANKYRIN HOMOLOG PRECURSOR.//3.1E-11//189aa//32%//Q06527
C-PLACE1008457
C-PLACE1008465
C-PLACE1008488
C-PLACE1008524//Human DNA sequence from clone 34B21 on chromosome 6p12.1-21.1. Contains part of a gene for a novel protein with ZU5 domain similar to part of Tight Junction Protein ZO1 (TJP1) and UNC5 Homologs, the gene for a novel BZRP (peripheral benzodiazapine recepto//0//1980bp//99%//AL031778
C-PLACE1008531
C-PLACE1008532
C-PLACE1008533//101 KD MALARIA ANTIGEN (P101) (ACIDIC BASIC REPEAT ANTIGEN).//1.1E-09//62aa//48%//P22620
C-PLACE1008568
C-PLACE1008603//NUCLEAR PORE COMPLEX PROTEIN NUP155 (NUCLEOPORIN NUP155) (155 KD NUCLEOPORIN) (P140).//7.8E-236//453aa//96%//P37199
C-PLACE1008621
C-PLACE1008626
C-PLACE1008627//Homo sapiens mRNA for cysteine-rich protein.//0//1850bp//99%//AJ006591
C-PLACE1008629
C-PLACE1008650//Homo sapiens pleiotropic regulator 1 (PLRG1) mRNA, complete cds.//0//1548bp//100%//AF044333
C-PLACE1008693
C-PLACE1008696//Homo sapiens NADH dehydrogenase-ubiquinone Fe-S protein 8 23 kDa subunit (NDUFS8) gene, nuclear gene encoding mitochondrial protein, complete cds.//0//3002bp//99%//AF038406
C-PLACE1008790//IMPORTIN ALPHA-6 SUBUNIT (KARYOPHERIN ALPHA-6 SUBUNIT) (IMPORTIN ALPHA S2).//3.1E-280//533aa//98%//O35345
C-PLACE1008808//Homo sapiens mRNA for cell cycle checkpoint protein rad1A.//2.3E-269//1225bp//99%//AJ004974
C-PLACE1008813
C-PLACE1008854
C-PLACE1008867
C-PLACE1008887
C-PLACE1008902
C-PLACE1008925
C-PLACE1009020//NIFS PROTEIN.//3.9E-55//279aa//41%//P12623
C-PLACE1009027//Homo sapiens mRNA for doublecortin.//0//1919bp//99%//AJ003112
C-PLACE1009045
C-PLACE1009060//BRO1 PROTEIN.//6.7E-19//567aa//24%//P48582
C-PLACE1009090
C-PLACE1009091
C-PLACE1009094//FURIN-LIKE PROTEASE 2 PRECURSOR (EC 3.4.21.75) (FURIN 2).//1.9E-44//480aa//30%//P30432
C-PLACE1009099//ZINC FINGER PROTEIN 41 (FRAGMENT).//1.1E-179//452aa//67%//P51814
C-PLACE1009110
C-PLACE1009111
C-PLACE1009130//UBIQUITIN-PROTEIN LIGASE E3A (EC 6.3.2.-) (ONCOGENIC PROTEIN-ASSOCIATED PROTEIN E6-AP).//2E-68//181aa//43%//Q05086
C-PLACE1009158
C-PLACE1009166
C-PLACE1009174
C-PLACE1009186
C-PLACE1009190
C-PLACE1009230
C-PLACE1009319//Rattus norvegicus outer membrane protein (OMP25) mRNA, complete cds; nuclear gene for mitochondrial product.//2.1E-132//1229bp//75%//AF107295
C-PLACE1009328
C-PLACE1009335
C-PLACE1009338
C-PLACE1009368//METAL HOMEOSTASIS FACTOR ATX2.//2.5E-10//151aa//29%//Q12067
C-PLACE1009375
C-PLACE1009388
C-PLACE1009404//HYPOTHETICAL 105.6 KD PROTEIN C16C9.06C IN CHROMOSOME I.//0.000000047//165aa7/33%//Q09820
C-PLACE1009434
C-PLACE1009443
C-PLACE1009444//PHOSPHATIDYLINOSITOL 4-KINASE ALPHA (EC 2.7.1.67) (PI4-KINASE) (PTDINS-4-KINASE) (PI4K-ALPHA).//7.8E-71//82aa//89%//P42356
C-PLACE1009459
C-PLACE1009468//PHOSPHOLIPASE A-2-ACTIVATING PROTEIN (PLAP).//3.1E-289//550aa//93%//P54319
C-PLACE1009476//PUTATIVE ATP-DEPENDENT RNA HELICASE T26G10.1 IN CHROMOSOME III.//3.9E-40//179aa//37%//P34580
C-PLACE1009524//ARF NUCLEOTIDE-BINDING SITE OPENER (ARNO PROTEIN) (ARF EXCHANGE FACTOR).//8.1E-99//228aa//75%//Q99418
C-PLACE1009542
C-PLACE1009571
C-PLACE1009581
C-PLACE1009596//VEGETATIBLE INCOMPATIBILITY PROTEIN HET-E-1.//5.1E-54//291aa//40%//Q00808
C-PLACE1009607
C-PLACE1009621
C-PLACE1009622//MATERNAL EFFECT PROTEIN STAUFEN.//1.3E-60//209aa//41%//P25159
C-PLACE1009659//MEMBRANE-ASSOCIATED PROTEIN HEM-2 (NAPI PROTEIN).//1.5E-285//538aa//99%//P55161
C-PLACE1009665
C-PLACE1009670//Homo sapiens genethonin 1 mRNA, complete cds.//0//1854bp//100%//AF062534
C-PLACE1009708//HYPOTHETICAL 143.3 KD TRP-ASP REPEATS CONTAINING PROTEIN C12G12.13C IN CHROMOSOME I.//7E-33//166aa//43%//Q09876
C-PLACE1009721//MSF1 PROTEIN.//1.7E-22//176aa//33%//P35200
C-PLACE1009731//AIG1 PROTEIN.//1.6E-22//274aa//28%//P54120
C-PLACE1009763//Homo sapiens mRNA for Nedd8-activating enzyme hUba3, complete cds.//4.3E-294//1329bp//100%//AB012190
C-PLACE1009794
C-PLACE1009845//Homo sapiens mRNA for KIAA0905 protein, complete cds.//0//2685bp//99%//AB020712
C-PLACE1009886
C-PLACE1009908//HYPOTHETICAL GTP-BINDING PROTEIN IN SEH1-PRP20 INTERGENIC REGION.//1.9E-108//277aa//43%//P53145
C-PLACE1009971
C-PLACE1009992//LIMULUS CLOTTING FACTOR C PRECURSOR (EC 3.4.21.84).//4.6E-59//450aa//34%//P28175
C-PLACE1009995//Homo sapiens mRNA; cDNA DKFZp5640123 (from clone DKFZp5640123).//0//1962bp//99%//AL080122
C-PLACE1009997//Rattus norvegicus A-kinase anchoring protein AKAP 220 mRNA, complete cds.//5.2E-70//736bp//73%//U48288
C-PLACE1010023
C-PLACE1010031
C-PLACE1010053//M.musculus Spnr mRNA for RNA binding protein.//6E-279//1402bp//94%//X84692
C-PLACE1010074//Homo sapiens sorting nexin 2 (SNX2) mRNA, complete cds.//0//2019bp//99%//AF065482
C-PLACE1010076
C-PLACE1010096//100 KD PROTEIN (EC 6.3.2.-).//1.4E-268//506aa//98%//Q62671
C-PLACE1010102
C-PLACE1010105//RING CANAL PROTEIN (KELCH PROTEIN).//7.3E-114//537aa//44%//O04652
C-PLACE1010106//Homo sapiens mRNA; cDNA DKFZp586M1418 (from clone DKFZp586M1418).//0//1974bp//99%//AL049385
C-PLACE1010134//TRANSCRIPTION REGULATORY PROTEIN SNF2 (SWI/SNF COMPLEX COMPONENT SNF2) (REGULATORY PROTEIN SWI2) (REGULATORY PROTEIN GAM1) (TRANSCRIPTION FACTOR TYE3).//1.7E-20//156aa//42%//P22082
C-PLACE1010148//CYLICIN I (MULTIPLE-BAND POLYPEPTIDE I).//0.00000046//431aa//23%//P35662
C-PLACE1010194//SPLICING FACTOR, ARGININE/SERINE-RICH 2 (SPLICING FACTOR SC35) (SC-35) (SPLICING COMPONENT, 35 KD) (PR264 PROTEIN).//9.8E-11//95aa//49%//Q01130
C-PLACE1010202
C-PLACE1010261//SEGREGATION DISTORTER PROTEIN.//1.6E-77//214aa//62%//P25722
C-PLACE1010274//Homo sapiens mRNA; cDNA DKFZp5640123 (from clone DKFZp5640123).//0//1964bp//99%//AL080122
C-PLACE1010293
C-PLACE1010321//NON-GREEN PLASTID TRIOSE PHOSPHATE TRANSLOCATOR PRECURSOR (CTPT).//1.1E-09//350aa//22%//P52178
C-PLACE1010324
C-PLACE1010329
C-PLACE1010362//1-PHOSPHATIDYLINOSITOL PHOSPHODIESTERASE PRECURSOR (EC 3.1.4.10) (PHOSPHATIDYLINOSITOL-SPECIFIC PHOSPHOLIPASE C) (PI-PLC).//0.000000002//126aa//29%//P34024
C-PLACE1010364
C-PLACE1010383
C-PLACE1010481//Homo sapiens mRNA for KIAA0836 protein, partial cds.//0//2121bp//99%//AB020643
C-PLACE1010491
C-PLACE1010492
C-PLACE1010522//Homo sapiens mRNA for DEPP (decidual protein induced by progesterone), complete cds.//0//1981bp//99%//AB022718
C-PLACE1010529
C-PLACE1010547//INTRACELLULAR PROTEIN TRANSPORT PROTEIN USO1.//0.00000012//616aa//24%//P253 86
C-PLACE1010599//Homo sapiens Pex14 mRNA for peroxisomal membrane anchor protein, complete cds.//0//1904bp//99%//AB017546
C-PLACE1010616
C-PLACE1010622//TROPONIN T, CARDIAC MUSCLE ISOFORMS (TNTC).//0.00000016//120aa//28%//P02642
C-PLACE1010629
C-PLACE1010630
C-PLACE1010661//TESTIS-SPECIFIC PROTEIN PBS13.//5.7E-75//423aa//39%//Q01755
C-PLACE1010714
C-PLACE1010720//Homo sapiens mRNA for chromosome-associated polypeptide-C, complete cds.//4E-299//1091bp//99%//AB019987
C-PLACE1010743//Homo sapiens myosin-IXb splice variant (Myo9b) mRNA, partial cds.//8.9E-91//668bp//82%//AF020267
C-PLACE1010771//M.musculus HCNGP mRNA.//7.4E-168//966bp//89%//X68061
C-PLACE1010786
C-PLACE1010800
C-PLACE1010811
C-PLACE1010870//ZINC FINGER PROTEIN 91 (ZINC FINGER PROTEIN HTF10) (HPF7).//1.3E-143//407aa//58%//Q05481
C-PLACE1010877//Homo sapiens mRNA for KIAA0610 protein, partial cds.//0//1885bp//99%//AB011182
C-PLACE1010900
C-PLACE2000050
C-PLACE4000522//NEUROGENIC LOCUS NOTCH PROTEIN HOMOLOG PRECURSOR (XOTCH PROTEIN)-//2.4E-191//828aa//48%//P21783
C-PLACE4000590
C-PLACE4000638
C-PLACE4000650//TUBERIN (TUBEROUS SCLEROSIS 2 HOMOLOG PROTEIN).//7.9E-17//201aa//34%//P49816
C-Y79AA1001647

### Homology Search Result Data 7.

The result of the homology search of the SwissProt using the 5'-end sequence (54 clones selected in EXAMPLE 16).

Data include
the name of clone,
definition of the top hit data,
the P-value: the length of the compared sequence: identity (%), and
the organism and the Accession No. of the top hit data, as in the order separated by //.

Data are not shown for the clones in which the P-value was higher than 1.
F-HEMBA1000497//METALLOTHIONEIN-LIKE PROTEIN 2A (MT-2A) (MT-K) (MT-1G).//0.13//52//38//P25860
F-HEMBA1001750//UBIQUITIN CARBOXYL-TERMINAL HYDROLASE 64E (EC 3.1.2.15) (UBIQUITIN THIOLESTERASE 64E) (UBIQUITIN-SPECIFIC PROCESSING PROTEASE 64E) (DEUBIQUITINATING ENZYME 64E).//2.2e-28//104//59//Q24574
F-HEMBA1003854//VERPROLIN.//0.012//138//31//P37370
F-HEMBA1004193//NADH-UBIQUINONE OXIDOREDUCTASE CHAIN 4L (EC 1.6.5.3) (FRAGMENT).//0.93//39//33//Q37131
F-HEMBA1004860//HIGH POTENTIAL IRON-SULFUR PROTEIN, ISOZYME 2 (HIPIP 2).//0.90//20//50//P38524
F-HEMBA1005572//ZINC FINGER PROTEIN 124 (HZF-16).//7.6e-46//141//58//Q15973
F-HEMBA1006038//COLLAGEN ALPHA 1(XII) CHAIN (FRAGMENT).//0.0033//32//46//P70560
F-HEMBA1006092//VERPROLIN.//1.0//62//35//P37370
F-HEMBA1006406//MATING PHEROMONE ER-10 PRECURSOR (EUPLOMONE R10).//0.30//41//36//P12350
F-HEMBA1006650//MATING-TYPE PHEROMONE BAP1(2) PRECURSOR.//0.089//21//52//Q02593
F-HEMBA1006812//HEAT SHOCK PROTEIN HTGA (HEAT SHOCK PROTEIN HTPY).//0.38//156//30//P28697
F-HEMBB1000672
F-HEMBB1001197//DNA-BINDING PROTEIN 65 (PROTEIN GP65).//1.0//30//36//P16012
F-HEMBB1001871//BONE/CARTILAGE PROTEOGLYCAN I PRECURSOR (BIGLYCAN) (PG-S1).//3.7e-54//241//47//P47853
F-MAMMA1001252//HYPOTHETICAL 9.1 KD PROTEIN IN NIRQ 3'REGION (ORF3).//0.59//48//39//Q51483
F-MAMMA1002094
F-NT2RM4000634//T-CELL RECEPTOR BETA CHAIN PRECURSOR (ANA 11).//0.26//58//27//P06333
F-NT2RM4000657//1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III) (FRAGMENT).//8.9e-20//83//48//P10895
F-NT2RM4000783//ZINC FINGER PROTEIN (FRAGMENT).//1.0//42//40//P19326
F-NT2RM4000857//INSULIN-LIKE GROWTH FACTOR BINDING PROTEIN COMPLEX ACID LABILE CHAIN PRECURSOR (ALS).//6.0e-23//207//32//O02833
F-NT2RM4001178//HOMEOBOX PROTEIN OTX3 (ZOTX3).//0.012//156//28//Q90267
F-NT2RM4002420//GLUTAMIC ACID-RICH PROTEIN PRECURSOR.//0.0012//81//37//P13816
F-NT2RP2000198//CREB-BINDING PROTEIN.//0.29//98//37//Q92793
F-NT2RP2000551//PROTEIN Q300.//0.00017//23//60//Q02722
F-NT2RP2000660//HYPOTHETICAL PROTEIN MJ0401.//1.0//41//29//Q57844
F-NT2RP2001214//MALE SPECIFIC SPERM PROTEIN MST84DC.//0.27//13//61//Q01644
F-NT2RP2001460//PROTEIN KINASE C-LIKE (EC 2.7.1.-).//0.089//99//29//Q99014
F-NT2RP2001756//ZINC FINGER PROTEIN MFG-1 (ZINC FINGER PROTEIN 58) (FRAGMENT)-//4.0e-13//177//28//P16372
F-NT2RP2002056//HYPOTHETICAL 6.0 KD PROTEIN IN THI12 5'REGION.//0.37//12//75//P53820
F-NT2RP2002677//NONSPECIFIC LIPID-TRANSFER PROTEIN 3 PRECURSOR (LTP 3).//0.99//61//32//Q42616
F-NT2RP2002755//OCTAPEPTIDE-REPEAT PROTEIN T2.//3.3e-10//90//35//Q06666
F-NT2RP2002843//CYTOCHROME B.//0.78//103//26//P48884
F-NT2RP2003101//ATPASE INHIBITOR, MITOCHONDRIAL HOMOLOG.//0.40//28//46//P37209
F-NT2RP2003799//HYPOTHETICAL PROTEIN MJ0116.1.//0.80//55//32//P81303
F-NT2RP2004095
F-NT2RP2004732
F-NT2RP2004920//HISTIDINE-RICH, METAL BINDING POLYPEPTIDE.//0.18//18//55//Q48251
F-NT2RP2005454
F-NT2RP2005776//POLY(A) POLYMERASE (EC 2.7.7.19) (PAP) (POLYNUCLEOTIDE ADENYLYLTRANSFERASE) (FRAGMENT).//7.4e-38//136//41//P51003
F-NT2RP2005806//A-AGGLUTININ ATTACHMENT SUBUNIT PRECURSOR.//4.0e-08//180//28//P32323
F-NT2RP2005882
F-NT2RP3001282//METHYL-ACCEPTING CHEMOTAXIS PROTEIN TLPB.//0.0022//69//39//P39217
F-NT2RP3001723//TRANSCRIPTIONAL REGULATORY PROTEIN ALGP (ALGINATE REGULATORY PROTEIN ALGR3).//0.00035//127//31//P15276
F-NT2RP3002099//NONHISTONE CHROMOSOMAL PROTEIN HMG-17.//0.97//71//28//P05204
F-NT2RP3003155//CCAAT DISPLACEMENT PROTEIN (HOMEOBOX PROTEIN CLOX) (CLOX-1) (FRAGMENT).//0.064//110//34//P39881
F-NT2RP3004028//NADH-UBIQUINONE OXIDOREDUCTASE CHAIN 5 (EC 1.6.5.3) (FRAGMENT).//0.020//95//29//P15583
F-OVARC1000008//SKIN SECRETORY PROTEIN XP2 PRECURSOR (APEG PROTEIN).//2.8e-05//165//29//P17437
F-OVARC1000724//SALIVARY PROLINE-RICH PROTEIN PO (ALLELE K) [CONTAINS: PEPTIDE P-D] (FRAGMENT).//0.035//152//30//P10162
F-OVARC1000751//TRANS-ACTING TRANSCRIPTIONAL PROTEIN ICPO (VMW118 PROTEIN).//0.38//124//31//P28284
F-OVARC1001029
F-PLACE1000814//EC PROTEIN HOMOLOG 2 (FRAGMENT).//0.45//61//24//Q42377
F-PLACE1003030//GALECTIN-3 (GALACTOSE-SPECIFIC LECTIN 3) (MAC-2 ANTIGEN) (IGE-BINDING PROTEIN) (35 KD LECTIN) (CARBOHYDRATE BINDING PROTEIN 35) (CBP 35) (LAMININ-BINDING PROTEIN) (LECTIN L-29).//0.70//121//32//P47845
F-PLACE1005549//RHO1 GDP-GTP EXCHANGE PROTEIN 1 (PROTEIN KINASE C SUPPRESSOR SKC1).//3.2e-08//205//24//P53046
F-PLACE1007218//IG KAPPA CHAIN V-III REGION (PC 7210).//0.99//52//38//P01668

### Homology Search Result Data 8.

The result of the homology search of the GenBank using the clone sequence of 5'-end (54 clones selected in EXAMPLE 16.) except EST and STS.

Data include
the name of clone,
definition of the top hit data,
the P-value: the length of the compared sequence: identity (%), and
the Accession No. of the top hit data, as in the order separated by //.

Data are not shown for the clones in which the P-value was higher than 1.
F-HEMBA1000497
F-HEMBA1001750//Human mitochondrial genes for several tRNAs (Phe, Val, Leu) and 12S and 16S ribosomal RNAs.//6.6e-101//473//99//V00710
F-HEMBA1003854//Homo sapiens clone RG270D13, *** SEQUENCING IN PROGRESS ***, 18 unordered pieces.//1.7e-05//412//61//AC005081
F-HEMBA1004193//Human BAC clone RG343H22 from 7q31, complete sequence.//0.77//466//59//AC002386
F-HEMBA1004860//Human pigment epithelium-derived factor gene, complete cds.//6.7e-07//492//57//U29953
F-HEMBA1005572//HZF-16=Kruppel-related zinc finger gene homolog {alternatively spliced} [human, hepatoblastoma cell line, HEP-G2, mRNA, 2080 nt].//2.9e-47//341//77//S54641
F-HEMBA1006038//Human DNA sequence from clone 989H11 on chromosome 22q13.1-13.2, complete sequence.//0.28//436//59//Z83851
F-HEMBA1006092//Human chromosome 16pl3.11 BAC clone CIT987SK-29B12 complete sequence.//0.28//309//60//U95738
F-HEMBA1006406//HS_2268_B2_C07_MR CIT Approved Human Genomic Sperm Library D Homo sapiens genomic clone Plate=2268 Col=14 Row=F, genomic survey sequence.//3.7e-69//340//99//AQ070566
F-HEMBA1006650//H.sapiens CpG island DNA genomic Mse1 fragment, clone 5h5, forward read cpg5h5.f1a.//9.4e-24//143//96//Z55730
F-HEMBA1006812//X.laevis xUBFalphal mRNA for upstream binding factor 2.//0.96//234//64//X59863
F-HEMBB1000672//CIT-HSP-2350H6.TF CIT-HSP Homo sapiens genomic clone 2350H6, genomic survey sequence.//1.1e-68//375//94//AQ059158
F-HEMBB1001197//Drosophila melanogaster strawberry notch (sno) mRNA, complete cds.//2.8e-10//229//66//U95760
F-HEMBB1001871//Equus caballus dermatan sulfate proteoglycan II mRNA, complete cds.//1.2e-27//619//62//AF038127
F-MAMMA1001252
F-MAMMA1002094//H.sapiens CpG island DNA genomic Mse1 fragment, clone 184g7, forward read cpg184g7.ft1a.//3.4e-29//167//97//Z59993
F-NT2RM4000634//Chionoecetes opilio (clone COP41) DNA microsatellite repeat regions.//1.4e-21//230//73//L49136
F-NT2RM4000657//Human mRNA for phospholipase C, complete cds.//0.029//245//61//D42108
F-NT2RM4000783//Homo sapiens chromosome 16, cosmid clone 330D11 (LANL), complete sequence.//3.7e-36//324//70//AC005199
F-NT2RM4000857//RPCI11-49P19.TJ RPCI-11 Homo sapiens genomic clone RPCI-11-49P19, genomic survey sequence.//1.5e-62//322//97//AQ051961
F-NT2RM4001178//Streptomyces coelicolor cosmid 7H1.//0.0025//296//62//AL021411
F-NT2RM4002420//Homo sapiens chromosome 17, clone hRPK.63_A_1, complete sequence.//0.00013//121//76//AC005670
F-NT2RP2000198//Human platelet glycoprotein IX mRNA, 3' end.//0.016//246//62//M25827
F-NT2RP2000551//Rattus norvegicus microsatellite sequence clone 82G9.//2.0e-08//223//69//AJ233812
F-NT2RP2000660//Homo sapiens chromosome 19, cosmid R30953, complete sequence.//0.0073//209//66//AC005622
F-NT2RP2001214
F-NT2RP2001460//Homo sapiens PAC clone DJ0905J08 from 7p12-p14, complete sequence.//1.0//80//76//AC005189
F-NT2RP2001756//CIT-HSP-2373P1.TR CIT-HSP Homo sapiens genomic clone 2373P1, genomic survey sequence.//3.0e-38//220//94//AQ110589
F-NT2RP2002056//Genomic sequence from Human 17, complete sequence.//1.2e-80//317//91//AC002094
F-NT2RP2002677//Homo sapiens chromosome 10 clone CIT987SK-1031G15 map 10q25, *** SEQUENCING IN PROGRESS ***, 1 ordered pieces.//0.032//141//70//AC006097
F-NT2RP2002755//Homo sapiens genomic DNA of 21q22.2 Down Syndrome region, segment 9/13.7/1.8e-22//377//69//AP000018
F-NT2RP2002843//Homo sapiens BAC clone RG030L05 from 7q22, complete sequence.//6.5e-16//311//63//AC005050
F-NT2RP2003101//Human FMR1 gene, 5' end.//0.32//105//67//L19476
F-NT2RP2003799//Human DNA for 5' terminal region of LINE-1 transposable element clone CGL1-4.//1.6e-33//119//96//X52233
F-NT2RP2004095//HS_3083_A1_A02_MF CIT Approved Human Genomic Sperm Library D Homo sapiens genomic clone Plate=3083 Col=3 Row=A, genomic survey sequence.//1.0e-14//154//79//AQ106698
F-NT2RP2004732//CIT-HSP-631P16.TP CIT-HSP Homo sapiens genomic clone 631P16, genomic survey sequence.//2.3e-20//120//99//B79035
F-NT2RP2004920//Plasmodium falciparum MAL3P4, complete sequence.//0.030//397//59//AL008970
F-NT2RP2005454//Plasmodium falciparum chromosome 2, section 47 of 73 of the complete sequence.//0.97//455//56//AE001410
F-NT2RP2005776//H.sapiens PAP mRNA.//1.0e-33//451//68//X76770
F-NT2RP2005806//Mus musculus musculus sex determining protein (Sry) gene, complete cds.//0.029//412//60//U70652
F-NT2RP2005882//Human DNA sequence from PAC 389A20 on chromosome X contains ESTs STS, CpG islands and polymorphic CA repeat.//9.4e-25//155//90//Z93242
F-NT2RP3001282//RPCI11-52L16.TJ RPCI-11 Homo sapiens genomic clone RPCI-11-52L16, genomic survey sequence.//3.2e-21//122//100//AQ052775
F-NT2RP3001723//H.sapiens CpG island DNA genomic Mse1 fragment, clone 13g5, reverse read cpg13g5.rt1a.//2.2e-18//163//85//Z56771
F-NT2RP3002099//Homo sapiens chromosome 17, clone hCIT.296_K_1, complete sequence.//1.3e-76//351//86//AC005180
F-NT2RP3003155
F-NT2RP3004028//Sequence 1 from patent US 5618695.//3.3e-13//217//70//I40055
F-OVARC1000008////0.0040//674//57//M82836
F-OVARC1000724//Herpes simplex virus type I immediate early (IE) gene 3 for transcriptional activator IE175 (= ICP 4).//1.1e-07//519//59//X06461
F-OVARC1000751//Homo sapiens DNA from chromosome 19, cosmid R29144, complete sequence.//7.2e-11//509//62//AC004221
F-OVARC1001029//Human DNA sequence from clone 19408 on chromosome 6q24.1-25.3 Contains STS and GSSs, complete sequence.//1.1e-05//388//61//AL031769
F-PLACE1000814//Homo sapiens BAC clone GS011E15 from 5q31, complete sequence.//1.4e-84//717//78//AC002427
F-PLACE1003030
F-PLACE1005549//Human guanine nucleotide regulatory protein (NET1) mRNA, complete cds.//4.9e-56//709//68//U02081
F-PLACE1007218//Homo sapiens chromosome 20 clone RP3-387E22, *** SEQUENCING IN PROGRESS ***, in unordered pieces.//3.1e-39//214//98//AL031660

### Homology Search Result Data 9.

The result of the homology search of the GenBank using the clone sequence of 3'-end (54 clones selected in EXAMPLE 16.) except EST and STS.

Data include
the name of clone,
definition of the top hit data,
the P-value: the length of the compared sequence: identity (%), and
the Accession No. of the top hit data, as in the order separated by //.

Blank indicates that the 3'-end sequence corresponding to the 5'-end was not determined in the clone.

Data are not shown for the clones in which the P-value was higher than 1.
R-HEMBA1000497//***ALU WARNING: Human Alu-J subfamily consensus sequence.//1.4e-38//185//84//U14567
R-HEMBA1001750//Hansenula wingei mitochondrial DNA, complete sequence.//1.7e-07//399//59//D31785
R-HEMBA1003854//Human DNA sequence from clone 224A6 on chromosome 1p35.1-36.23 Contains part of a gene similar to Mouse Wnt-4 protein, the gene for CDC42 (cell division cycle 42 (GTP-binding protein, 25kD)), ESTs, STSs, GSSs and a CpG Island, complete sequence.//1.4e-75//309//85//AL031281
R-HEMBA1004193//***ALU WARNING: Human Alu-J subfamily consensus sequence.//1.1e-34//188//81//U14567
R-HEMBA1004860//Homo sapiens 12q13.1 PAC RPCI3-197B17 (Roswell Park Cancer Institute Human PAC library) complete sequence.//1.3e-06//239//66//AC004241
R-HEMBA1005572//Homo sapiens chromosome 21 PAC RPCIP704E14135Q2, complete sequence.//3.1e-21//341//67//AJ010598
R-HEMBA1006038//Homo sapiens chromosome 19, cosmid R34094, complete sequence.//1.7e-24//307//71//AC004678
R-HEMBA1006092//H.Sapiens mRNA for alpha2-subunit of soluble guanylyl cyclase.//0.76//246//62//X63282
R-HEMBA1006406//Human DNA sequence from clone 113J7 on chromosome Xp11.22-11.4 Contains part of a putative Homeobox (pseudo?) gene, ESTs and an STS, complete sequence.//1.3e-31//297//77//AL023574
R-HEMBA1006650//Homo sapiens BAC clone BK085E05 from 22q12.1-qter, complete sequence.//1.8e-15//350//65//AC003071
R-HEMBA1006812//Homo sapiens chromosome X clone RP3-424J12, *** SEQUENCING IN PROGRESS ***, in unordered pieces.//1.8e-55//430//81//Z82207
R-HEMBB1000672//Homo sapiens clone UWGC:y54c283 from 6p21, complete sequence.//9.1e-39//437//71//AC006166
R-HEMBB1001197//Homo sapiens PAC clone DJ0964C11 from 7p14-p15, complete sequence.//1.5e-37//275//85//AC004593
R-HEMBB1001871//Plasmodium falciparum chromosome 12 clone 3D7, *** SEQUENCING IN PROGRESS ***, 5 unordered pieces.//0.00097//410//59//AC004688
R-MAMMA1001252//Homo sapiens clone 201104, *** SEQUENCING IN PROGRESS ***, 4 unordered pieces.//2.9e-13//364//64//AC004529
R-MAMMA1002094//HS_3163_A1_A09_MR CIT Approved Human Genomic Sperm Library D Homo sapiens genomic clone Plate=3163 Col=17 Row=A, genomic survey sequence.//5.9e-41//256//91//AQ141441
R-NT2RM4000634//Homo sapiens chromosome 19, cosmid R30783, complete sequence.//1-6e-21//283//73//AC005258
R-NT2RM4000657
R-NT2RM4000783
R-NT2RM4000857//RPCI11-63K2.TK RPCI-11 Homo sapiens genomic clone RPCI-11-63K2, genomic survey sequence.//4.0e-07//62//98//AQ203073
R-NT2RM4001178
R-NT2RM4002420
R-NT2RP2000198//Homo sapiens Chromosome 16 BAC clone CIT987-SK37914 -complete genomic sequence, complete sequence.//0.58//108//67//AC002307
R-NT2RP2000551//Homo sapiens DNA, pseudoautosomal boundary-like sequence PABL2.//6.2e-72//391//87//D30043
R-NT2RP2000660//Homo sapiens chromosome 17, clone hRPK.640_I_15, complete sequence.//0.0058//166//69//AC005324
R-NT2RF2001214//Saccharomyces douglasii mitochondrial tRNA-Ser and tRNA-Phe genes, partial sequence, and Var1p (var1) gene, mitochondrial gene encoding mitochondrial protein, complete cds.//0.93//117//65//U49822
R-NT2RP2001460
R-NT2RP2001756//CIT-HSP-2382021.TR CIT-HSP Homo sapiens genomic clone 2382021, genomic survey sequence.//3.4e-91//507//92//AQ114228
R-NT2RP2002056//Homo sapiens DNA sequence from PAC 95C20 on chromosome Xp11.3-11.4. Contains STSs and the DXS7 locus with GT and GTG repeat polymorphisms, complete sequence.//0.00022//225//69//Z97181
R-NT2RP2002677//CIT-HSP-2349K20.TF CIT-HSP Homo sapiens genomic clone 2349K20, genomic survey sequence.//3.1e-29//178//94//AQ062168
R-NT2RP2002755//Human DNA sequence from cosmid U65A4, between markers DXS366 and DXS87 on chromosome X *.//5.3e-39//449//72//Z81014
R-NT2RP2002843//Homo sapiens chromosome 17, clone hRPK.22_N_12, complete sequence.//0.0097//498//59//AC005412
R-NT2RP2003101//CIT-HSP-238301.TR CIT-HSP Homo sapiens genomic clone 238301, genomic survey sequence.//1.2e-32//344//75//AQ196754
R-NT2RP2003799////3.6e-05//408//60//AL010237
R-NT2RP2004095//Plasmodium falciparum chromosome 4 strain 3D7, *** SEQUENCING IN PROGRESS ***, in unordered pieces.//2.1e-10//455//61//AL034557
R-NT2RP2004732//Human DNA sequence from clone 703H14 on chromosome 1q23.2-24.3 Contains 3' end of a novel gene, ESTs, CA repeat(D1S445), STS, GSSs, complete sequence.//5.1e-51//383//74//AL031287
R-NT2RP2004920//Homo sapiens chromosome 5, P1 clone 878H11 (LBNL H45), complete sequence.//0.062//315//61//AC005219
R-NT2RP2005454//Human DNA sequence from PAC 121G13 on chromosome 6 contains flow sorted chromosome 6 HindDIII fragment ESTs. polymorphic CA repeat, CpG island, CpG island genomic fragments.//0.75//246//63//Z86062
R-NT2RP2005776//Homo sapiens PAC clone DJ1189D06 from 7p15.3-p14, complete sequence.//0.91//232//61//AC005232
R-NT2RP2005806//Human neurofibromatosis type 1 (NF1) gene, intron 19a, complete sequence.//1.3e-19//405//66//U37368
R-NT2RP2005882//Plasmodium falciparum MAL3P1, complete sequence.//1.1e-09//533//60//Z97348
R-NT2RP3001282//Plasmodium falciparum MAL3P8, complete sequence.//0.00026//499//58//AL034560
R-NT2RP3001723//Human BAC clone RG354L07 from 7q31, complete sequence.//0.00035//337//61//AC002466
R-NT2RP3002099//Homo sapiens chromosome 17, clone hCIT.296_K_1, complete sequence.//1.8e-44//307//86//AC005180
R-NT2RP3003155
R-NT2RP3004028//F14A6-Sp6 IGF Arabidopsis thaliana genomic clone F14A6, genomic survey sequence.//0.95//95//65//B21351
R-OVARC1000008
R-OVARC1000724//Homo sapiens BAC clone RG017K18 from 7q31, complete sequence.//0.91//83//71//AC005161
R-OVARC1000751//HS_2222_A2_C09_MR CIT Approved Human Genomic Sperm Library D Homo sapiens genomic clone Plate=2222 Col=18 Row=E, genomic survey sequence.//2.8e-12//176//72//AQ033143
R-OVARC1001029//Homo sapiens Xp22 Cosmid U151G1 (from Lawrence Livermore X library) and PAC RPCI1-93D11 (from Roswell Park Cancer Center) complete sequence.//1.2e-09//165//75//AC002357
R-PLACE1000814//Homo sapiens BAC clone GS465N13 from 7p15-p21, complete sequence.//6.2e-52//514//75//AC004744
R-PLACE1003030//Homo sapiens snRNA activating protein complex 190kD subunit (SNAP190) mRNA, complete cds.//9.6e-33//225//90//AF032387
R-PLACE10e5549//Arabidopsis thaliana genomic DNA, chromosome 5, TAC clone: K19P17, complete sequence.//0.097//323//61//AB007644
R-PLACE1007218//Homo sapiens chromosome 20 clone RP3-387E22, *** SEQUENCING IN PROGRESS ***, in unordered pieces.//1.1e-88//497//91//AL031660

### Homology Search Result Data 10.

The result of the homology search of the Human Unigene using the clone sequence of 5'-end (54 clones selected in EXAMPLE 16.) .

Data include
the name of clone,
title of the top hit data,
the P-value: the length of the compared sequence: identity (%), and
the Accession No. of the top hit data, as in the order separated by //.

Data are not shown for the clones in which the P-value was higher than 1.
F-HEMBA1000497//ou15a11.x1 Soares_NFL_T_GBC_S1 Homo sapiens cDNA clone IMAGE:1626332 3', mRNA sequence.//1.0//186//65//AI018130
F-HEMBA1001750//Human mRNA for TI-227H.//2.5e-101//473//99//D50525
F-HEMBA1003854//Homo sapiens mRNA for KIAA1031 protein, partial cds.//7.2e-06//103//80//AB028954
F-HEMBA1004193//Homo sapiens mRNA for TL132.//0.75//334//59//AJ012755
F-HEMBA1004860//ny07e01.s1 NCI_CGAP_GCB1 Homo sapiens cDNA clone IMAGE:1271064 3' similar to contains Alu repetitive element;, mRNA sequence.//3.7e-06//140//70//AA749151
F-HEMBA1005572//HZF-16=Kruppel-related zinc finger gene homolog {alternatively spliced} [human, hepatoblastoma cell line, HEP-G2, mRNA, 2080 nt].//1.1e-48//341//77//S54641
F-HEMBA1006038//Homo sapiens gene for insulin receptor substrate-2, complete cds.//0.036//297//60//AB000732
F-HEMBA1006092//ab80f12.s1 Stratagene fetal retina 937202 Homo sapiens cDNA clone IMAGE:853295 3' similar to contains Alu repetitive element;, mRNA sequence.//0.65//150//63//AA663266
F-HEMBA1006406//ws26e11.x1 NCI_CGAP_GC6 Homo sapiens cDNA clone IMAGE:2498348 3' similar to TR:002710 002710 GAG POLYPROTEIN ; mRNA sequence.//1.4e-32//518//67//AI989639
F-HEMBA1006650//Homo sapiens Arp2/3 protein complex subunit p20-Arc (ARC20) mRNA, complete cds.//1.3e-19//136//90//AF006087
F-HEMBA1006812//zh49f01.s1 Soares_fetal_liver_spleen_1NFLS_S1 Homo sapiens cDNA clone IMAGE:415417 3', mRNA sequence.//1.3e-120//579//98//W80404
F-HEMBB1000672//Homo sapiens mRNA for KIAA1040 protein, partial cds.//0.00047//706//57//AB028963
F-HEMBB1001197//tq45e03.x1 NCI_CGAP_Ut1 Homo sapiens cDNA clone IMAGE:2211772 3' similar to TR:001940 001940 STRAWBERRY NOTCH ;, mRNA sequence.//1.2e-16//117//92//AI580023
F-HEMBB1001871//Human chondroitin/dermatan sulfate proteoglycan (PG40) core protein mRNA, complete cds.//4.6e-26//527//62//M14219
F-MAMMA1001252
F-MAMMA1002094
F-NT2RM4000634//DKFZp434D1813_r1 434 (synonym: htes3) Homo sapiens cDNA clone DKFZp434D1813 5', mRNA sequence.//9.7e-16//226//69//AL040136
F-NT2RM4000657//Homo sapiens mRNA for KIAA1069 protein, partial cds.//7.6e-179//817//99//AB028992
F-NT2RM4000783//wd82f06.x1 NCI_CGAP_Lu24 Homo sapiens cDNA clone IMAGE:2338115 3', mRNA sequence.//1.8e-20//470//65//AI703299
F-NT2RM4000857//Homo sapiens KIAA0416 mRNA, partial cds.//1.9e-46//749//65//AB007876
F-NT2RM4001178//Homo sapiens protein tyrosine phosphatase (PAC-1) mRNA, complete cds.//0.0024//254//63//L11329
F-NT2RM4002420//wg39f11.x1 Soares_NSF_F8_9W_OT_PA_P_S1 Homo sapiens cDNA clone IMAGE:2367501 3' similar to contains element L1 L1 repetitive element ; mRNA sequence.//1.4e-13//127//84//AI742251
F-NT2RP2000198//Human mRNA for platelet glycoprotein IX.//0.0033//241//62//X52997
F-NT2RP2000551//ze37d12.s1 Soares retina N2b4HR Homo sapiens cDNA clone IMAGE:361175 3', mRNA sequence.//5.0e-07//116//71//AA017066
F-NT2RP2000660//qx01g11.x1 NGI_CGAP_Br14 Homo sapiens cDNA clone IMAGE:1999364 3', mRNA sequence.//0.027//120//65//AI225283
F-NT2RP2001214
F-NT2RP2001460//wb50h10.x1 NCI_CGAP_GC6 Homo sapiens cDNA clone IMAGE:2309155 3', mRNA sequence.//0.0013//89//78//AI651878
F-NT2RP2001756//zw54e12.s1 Soares_total_fetus_Nb2HF8_9w Homo sapiens cDNA clone IMAGE:773902 3' similar to TR:G456660 G456660 ZINC FINGER PROTEIN ZFP-1 ; mRNA sequence.//2.3e-18//120//93//AA427992
F-NT2RP2002056//tw44g09.x1 NCI_CGAP_Ut1 Homo sapiens cDNA clone IMAGE:2262592 3' similar to contains Alu repetitive element; mRNA sequence.//2.4e-07//99//79//AI811687
F-NT2RP2002677
F-NT2RP2002755//zj83d10.s1 Soares_fetal_liver_spleen_1NFLS_S1 Homo sapiens cDNA clone IMAGE:461491 3' similar to contains element TAR1 repetitive element ; mRNA sequence.//1.9e-19//229//76//AA705059
F-NT2RP2002843//wt88dl2.x1 NCI_CGAP_GC6 Homo sapiens cDNA clone IMAGE:2514551 3' similar to TR:P79522 P79522 MHC CLASS I REGION PROLINE RICH PROTEIN.; mRNA sequence.//8.2e-15//314//67//AI964055
F-NT2RP2003101//wi65a03.x1 NCI_CGAP_Kid12 Homo sapiens cDNA clone IMAGE:2398156 3', mRNA sequence.//0.38//106//68//AI763133
F-NT2RP2003799//Homo sapiens mRNA; cDNA DKFZp564C142 (from clone DKFZp564C142).//2.5e-29//124//91//AL049979
F-NT2RP2004095
F-NT2RP2004732//Homo sapiens mRNA for KIAA0884 protein, partial cds.//2.6e-109//533//96//AB020691
F-NT2RP2004920//wz68d10.x1 NCI_CGAP_Mel15 Homo sapiens cDNA clone IMAGE:2563219 3' similar to TR:000172 000172 LINE-1 REVERSE TRANSCRIPTASE ;, mRNA sequence.//0.0020//220//61//AI969546
F-NT2RP2005454//Homo sapiens mRNA for KIAA0977 protein, complete cds.//0.058//143//69//AB023194
F-NT2RP2005776//H.sapiens PAP mRNA.//4.3e-35//451//68//X76770
F-NT2RP2005806//HSZ78328 Human fetal brain S. Meier-Ewert Homo sapiens cDNA clone 2.48 (CEPH) 3', mRNA sequence.//2.0e-05//385//62//Z78328
F-NT2RP2005882//Human mRNA for KIAA0364 gene, complete cds.//7.3e-23//141//94//AB002362
F-NT2RP3001282
F-NT2RP3001723//ws73d05.x1 NCI_CGAP_Co3 Homo sapiens cDNA clone IMAGE:2503593 3' similar to contains MSR1.t1 TAR1 TAR1 repetitive element ; mRNA sequence.//2.6e-07//245//66//AW008782
F-NT2RP3002099//yg49d01.s1 Soares infant brain 1NIB Homo sapiens cDNA clone IMAGE:36239 3', mRNA sequence.//0.58//164//64//R46086
F-NT2RP3003155
F-NT2RP3004028//Homo sapiens mRNA for KIAA1074 protein, complete cds.//1.3e-29//488//66//AB028997
F-OVARC1000008//Homo sapiens mRNA for KIAA0665 protein, complete cds.//0.00032//430//59//AB014565
F-OVARC1000724//Homo sapiens mRNA for KIAA0641 protein, complete cds.//0.0054//426//58//AB014541
F-OVARC1000751//Human Tis11d gene, complete cds.//4.6e-12//527//62//U07802
F-OVARC1001029//qv29c05.x1 NCI_CGAP_Ov31 Homo sapiens cDNA clone IMAGE:1982984 3' similar to contains element L1 repetitive element ; mRNA sequence.//0.0012//145//68//AI252422
F-PLACE1000814//ak42f05.s1 Soares_testis_NHT Homo sapiens cDNA clone IMAGE:1408641 3', mRNA sequence.//7.1e-31//275//76//AA868469
F-PLACE1003030
F-PLACE1005549//Homo sapiens mRNA for Rho guanine nucleotide-exchange factor, splice variant NET1A.//1.2e-57//737//67//AJ010046
F-PLACE1007218//yo34a08.s1 Soares adult brain N2b4HB55Y Homo sapiens cDNA clone IMAGE:179798 3', mRNA sequence.//2.2e-21//216//76//H52716

### Homology Search Result Data 11.

The result of the homology search of the Human Unigene using the clone sequence of 3'-end (54 clones selected in EXAMPLE 16.).

Data include
the name of clone,
title of the top hit data,
the P-value: the length of the compared sequence: identity (%), and
the Accession No. of the top hit data, as in the order separated by //.

Blank indicates that the 3'-end sequence corresponding to the 5'-end was not determined in the clone.

Data are not shown for the clones in which the P-value was higher than 1.
R-HEMBA1000497//np09h02.s1 NCI_CGAP_Pr3 Homo sapiens cDNA clone IMAGE:1115859 similar to contains Alu repetitive element;contains element MER22 repetitive element ; mRNA sequence.//6.2e-38//185//83//AA614254
R-HEMBA1001750//yy71b10.s1 Soares_multiple_sclerosis_2NbHMSP Homo sapiens cDNA clone IMAGE:278971 3', mRNA sequence.//0.004511193//63//N63303
R-HEMBA1003854//Homo sapiens mRNA; cDNA DKFZp564F133 (from clone DKFZp564F133).//3.4e-72//310//80//AL049263
R-HEMBA1004193//tr01e08.x1 NCI_CGAP_Ov23 Homo sapiens cDNA clone IMAGE:2217062 3' similar to contains Alu repetitive element;contains element MER4 repetitive element ; mRNA sequence.//1.5e-33//186//81//AI914747
R-HEMBA1004860//qh16b06.x1 Soares_NFL_T_GBC_S1 Homo sapiens cDNA clone IMAGE:1844819 3', mRNA sequence.//0.017//118//69//AI218308
R-HEMBA1005572//wj16h05.x1 NCI_CGAP_Kid12 Homo sapiens cDNA clone IMAGE:2403033 3', mRNA sequence.//4.6e-111//522//99//AI861830
R-HEMBA1006038//DKFZp434E1117_r1 434 (synonym: htes3) Homo sapiens cDNA clone DKFZp434E1117 5', mRNA sequence.//1.2e-22//295//72//AL041450
R-HEMBA1006092//qt30d09.x1 Soares_pregnant_uterus_NbHPU Homo sapiens cDNA clone IMAGE: 1949489 3' similar to contains element PTR5 repetitive element ; mRNA sequence.//1.4e-87//422//98//AI337963
R-HEMBA1006406//Homo sapiens mRNA for KIAA0752 protein, partial cds.//4.1e-30//291-//76//AB018295
R-HEMBA1006650//H.sapiens mRNA for serine/threonine protein kinase EMK.//3.6e-09//319//62//X97630
R-HEMBA1006812//Human mRNA for KIAA0118 gene, partial cds.//3.1e-52//337//87//D42087
R-HEMBB1000672//Homo sapiens mRNA; cDNA DKFZp434M011 (from clone DKFZp434M011).//3.2e-48//276//74//AL096734
R-HEMBB1001197//zt35b11.r1 Soares ovary tumor NbHOT Homo sapiens cDNA clone IMAGE:724317 5' similar to contains Alu repetitive element; mRNA sequence.//9.9e-44//275//88//AA410788
R-HEMBB1001871//wg20c02.x1 Soares_NSF_F8_9W_OT_PA_P_S1 Homo sapiens cDNA clone IMAGE:2365634 3', mRNA sequence.//6.3e-104//501//98//AI741321
R-MAMMA1001252//aa61h04.s1 NCI_CGAP_GCB1 Homo sapiens cDNA clone IMAGE:825463 3' similar to contains Alu repetitive element;contains element XTR repetitive element ; mRNA sequence.//9.0e-19//127//91//AA504355
R-MAMMA1002094//wd28h12.x1 Soares_NFL_T_GBC_S1 Homo sapiens cDNA clone IMAGE:2329511 3', mRNA sequence.//2.5e-68//328//99//AI936520
R-NT2RM4000634//DKFZp434F20l6_s1 434 (synonym: htes3) Homo sapiens cDNA clone DKFZp434F2016 3', mRNA sequence.//8.2e-20//185//81//AL041146
R-NT2RM4000657//Homo sapiens mRNA for KIAA1069 protein, partial cds.//5.7e-62//335//94//AB028992
R-NT2RM4000783
R-NT2RM4000857//Human megakaryocyte stimulating factor mRNA, complete cds.//0.00074//360//61//U70136
R-NT2RM4001178//tk08e03.x1 NCI_CGAP_Lu24 Homo sapiens cDNA clone IMAGE:2150428 3', mRNA sequence.//0.77//96//62//AI457506
R-NT2RM4002420//wl58b04.x1 NCI_CGAP_Bm25 Homo sapiens cDNA clone IMAGE:2429071 3', mRNA sequence.//2.4e-85//438//94//AI857508
R-NT2RP2000198//nx19b11.s1 NCI_CGAP_GC3 Homo sapiens cDNA clone IMAGE:1256541 3', mRNA sequence.//1.9e-45//270//91//AA738352
R-NT2RP2000551//tg80h11.x1 Soares_NhHMPu_S1 Homo sapiens cDNA clone IMAGE:2115141 3', mRNA sequence.//3.3e-53//311//85//AI417680
R-NT2RP2000660//ns42a06.s1 NCI_CGAP_GCB1 Homo sapiens cDNA clone IMAGE:1186258 3', mRNA sequence.//4.3e-26//142//97//AA805691
R-NT2RP2001214//tw65g08.x1 NCI_CGAP_Ut3 Homo sapiens cDNA clone IMAGE:2264606 3' similar to contains element MSR1 repetitive element ; mRNA sequence.//1.5e-57//289//97//AI680174
R-NT2RP2001460
R-NT2RP2001756//zw54e12.s1 Soares_total_fetus_Nb2HF8_9w Homo sapiens cDNA clone IMAGE:773902 3' similar to TR:G456660 G456660 ZINC FINGER PROTEIN ZFP-1 ; mRNA sequence.//6.0e-13//85//96//AA427992
R-NT2RP2002056//yh26a12.s1 Soares placenta Nb2HP Homo sapiens cDNA clone IMAGE:130846 3', mRNA sequence.//0.0016//208//65//R22302
R-NT2RP2002677//Homo sapiens mRNA for KIAA0524 protein, partial cds.//3.4e-26//339//71//AB011096
R-NT2RP2002755//qd50d10.x1 Soares_fetal_heart_NbHH19W Homo sapiens cDNA clone IMAGE:1732915 3', mRNA sequence.//1.5e-26//419//66//AI190698
R-NT2RP2002843//at31f08.x1 Barstead colon HPLRB7 Homo sapiens cDNA clone IMAGE:2373639 3' similar to contains L1.t1 L1 repetitive element;, mRNA sequence.//1.8e-45//463//74//AI749673
R-NT2RP2003101//ty24h05.x1 NCI_CGAP_Ut3 Homo sapiens cDNA clone IMAGE:2280057 3', mRNA sequence.//7.5e-73//347//99//AI758824
R-NT2RP2003799//Homo sapiens mRNA for KIAA0751 protein, complete cds.//0.0026//247//65//AB018294
R-NT2RP2004095//zv08c02.s1 Soares_NhHMPu_S1 Homo sapiens cDNA clone IMAGE:753026 3' similar to contains element MER32 repetitive element ; mRNA sequence.//9.6e-07//188//66//AA436455
R-NT2RP2004732//tu60a07.x1 NCI_CGAP_Gas4 Homo sapiens cDNA clone IMAGE:2255412 3' similar to contains Alu repetitive element;contains element L1 repetitive element ; mRNA sequence.//4.3e-25//414//68//AI678956
R-NT2RP2004920//wd13h02.x1 NCI_CGAP_Co3 Homo sapiens cDNA clone IMAGE:2328051 3', mRNA sequence.//6.8e-91//483//93//AI694022
R-NT2RP2005454//yy77g09.s1 Soares_multiple_sclerosis_2NbHMSP Homo sapiens cDNA clone IMAGE:279616 3', mRNA sequence.//0.0070//325//59//N48302
R-NT2RP2005776//qq97d06.x1 Soares_total_fetus_Nb2HF8_9w Homo sapiens cDNA clone IMAGE:1939307 3', mRNA sequence.//7.5e-08//89//82//AI338419
R-NT2RP2005806//wc29h01.x1 NCI_CGAP_Kid11 Homo sapiens cDNA clone IMAGE:2316625 3' similar to contains MER2.b3 MER2 repetitive element ; mRNA sequence.//3.2e-16//235//71//AI671398
R-NT2RP2005882//wo31f09.x1 NCI_CGAP_Gas4 Homo sapiens cDNA clone IMAGE:2456969 3', mRNA sequence.//0.00095//352//59//AI925528
R-NT2RP3001282//wg35b03.x1 Soares_NSF_F8_9W_OT_PA_P_S1 Homo sapiens cDNA clone IMAGE:2367053 3', mRNA sequence.//1.7e-113//555//97//AI769199
R-NT2RP3001723//wo48e06.x1 NCI_CGAP_Gas4 Homo sapiens cDNA clone IMAGE:2458594 3', mRNA sequence.//4.2e-98//471//98//AI926617
R-NT2RP3002099//DKFZp564L227_s1 564 (synonym: hfbr2) Homo sapiens cDNA clone DKFZp564L227 3', mRNA sequence.//9.2e-50//329//87//AL037910
R-NT2RP3003155//zp07a07.s1 Stratagene ovarian cancer (#937219) Homo sapiens cDNA clone IMAGE:595668 3', mRNA sequence.//1.4e-30//159//99//AA173172
R-NT2RP3004028//Homo sapiens protein kinase C-alpha mRNA, partial 3' UTR.//0.43//66//75//AF035594
R-OVARC1000008//wa69e12.x1 Soares_NFL_T_GBC_S1 Homo sapiens cDNA clone IMAGE:2301454 3', mRNA sequence.//1.0e-77//376//98//AI699393
R-OVARC1000724//tf94b10.x1 NCI_CGAP_CLL1 Homo sapiens cDNA clone IMAGE:2106907 3', mRNA sequence.//0.71//27//100//AI380236
R-OVARC1000751//og93d04.s1 NCI_CGAP_Kid5 Homo sapiens cDNA clone IMAGE:1455847 3', mRNA sequence.//3.5e-13//274//63//AA863306
R-OVARC1001029//yz96e02.r1 Soares melanocyte 2NbHM Homo sapiens cDNA clone IMAGE:290906 5' similar to contains Alu repetitive element;contains element PTR5 repetitive element ; mRNA sequence//3.5e-13//175//74//N99464
R-PLACE1000814//tg49a08.x1 Soares_NFL_T_GBC_S1 Homo sapiens cDNA clone IMAGE:2112086 3' similar to contains Ll.t2 L1 L1 repetitive element; mRNA sequence.//2.2e-18//285//69//AI424789
R-PLACE1003030//Homo sapiens snRNA activating protein complex 190kD subunit (SNAP190) mRNA, complete cds.//4.0e-34//225//90//AF032387
R-PLACE1005549//tm26b11.x1 Soares_NFL_T_GBC_S1 Homo sapiens cDNA clone IMAGE:2157693 3', mRNA sequence.//0.91//127//66//AI480253
R-PLACE1007218//yq06e01.r1 Soares fetal liver spleen 1NFLS Homo sapiens cDNA clone IMAGE:196152 5' similar to contains Alu repetitive element;contains LTR4 repetitive element; mRNA sequence.//2.4e-36//245//87//R92256

### Homology Search Result Data 12.

Data obtained by the homology search for full-length nucleotide sequences and deduced amino acid sequences. Each data includes Clone name, Definition in hit data, P value, Length of sequence to be compared, Homology, and Accession number (No.) of hit data. These items are shown in this order and separated by a double-slash mark, //.
C-HEMBA1000012//PROBABLE LEUCYL-TRNA SYNTHETASE (EC 6.1.1.4) (LEUCINE--TRNA LIGASE) (LEURS).//6.4E-99//457aa//45%//Q09996
C-HEMBA1000020//Homo sapiens beta 2 gene.//7.5E-264//1194bp//95%//X02344
C-HEMBA1000129//HYTOTHETICAL HELICASE C8A4.08C IN CHROMOSOME I.//3.8E-25//166aa//36%//Q09884
C-HEMBA1000201//Homo sapiensimRNA for integrase interactor 1b protein (INI1B).//0//1612bp//99%//AJ011738
C-HEMBA1000216//HYPOXIA-INDUCIBLE FACTOR 1 ALPHA (HIF-1 ALPHA) (ARNT INTERACTING PROTEIN).//1E-86//146aa//56%//Q61221
C-HEMBA1000231
C-HEMBA1000264
C-HEMBA1000280
C-HEMBA1000282
C-HEMBA1000303//"Mus musculus Plenty of SH3s (POSH) mRNA, complete cds."//7.1E-254//1440bp//87%//AF030131
C-HEMBA1000333//"Homo sapiens mRNA for KIAA0874 protein, partial cds."//4.8E-253//1148bp//99%//AB020681
C-HEMBA1000351
C-HEMBA1000356//Homo sapiens mRNA; cDNA DKFZp566C243 (from clone DKFZp566C243).//3.3E-287//815bp//98%//AL050274
C-HEMBA1000396
C-HEMBA1000411//ANKYRIN.//5.7E-12//127aa//38%//Q02357
C-HEMBA1000442
C-HEMBA1000456
C-HEMBA1000504
C-HEMBA1000518//PECANEX PROTEIN.//2.1E-19//227aa//38%//P18490
C-HEMBA1000519
C-HEMBA1000523//TESTIS-SPECIFIC PROTEIN PBS13.//2.4E-44//292aa//36%//Q01755
C-HEMBA1000542//"Rattus norvegicus mRNA for dipeptidyl peptidase III, complete cds."//2.2E-194//663bp//83%//D89340
C-HEMBA1000545
C-HEMBA1000557
C-HEMBA1000592//"Homo sapiens sorting nexin 6 (SNX6) mRNA, complete cds."//0//1465bp//99%//AF121856
C-HEMBA1000594
C-HEMBA1000604
C-HEMBA1000622
C-HEMBA1000637
C-HEMBA1000655
C-HEMBA1000657//"Rattus norvegicus ADP-ribosylation factor-directed GTPase activating protein mRNA, complete cds."//7.2E-156//1366bp//76%//U35776
C-HEMBA1000749
C-HEMBA1000769
C-HEMBA1000773
C-HEMBA1000774
C-HEMBA1000822
C-HEMBA1000843
C-HEMBA1000852//ARYLSULFATASE D PRECURSOR (EC 3.1.6.-) (ASD).//1E-78//119aa//87%//P51689
C-HEMBA1000870
C-HEMBA1000908
C-HEMBA1000934
C-HEMBA1000972
C-HEMBA1000986
C-HEMBA1000991
C-HEMBA1001008
C-HEMBA1001059//"Human N-acetylgalactosamine 6-sulphatase (GALNS) gene, exon 14."//4.8E-169//786bp//99%//U06088
C-HEMBA1001094
C-HEMBA1001302//"Homo sapiens calcium binding protein precursor, mRNA, complete cds."//9.6E-258//682bp//94%//AF153686
C-HEMBA1001330
C-HEMBA1001497
C-HEMBA1001569//SYNAPTOBREVIN 2 (VESICLE ASSOCIATED MEMBRANE PROTEIN 2) (VAMP-2).//2.3E-53//110aa//100%//P19065
C-HEMBA1001570
C-HEMBA1001620//MYO-INOSITOL-1-PHOSPHATE SYNTHASE (EC 5.5.1.4) (IPS).//1.6E-166//506aa//60%//P42803
C-HEMBA1001640
C-HEMBA1001655
C-HEMBA1001672//"Homo sapiens methyl-CpG binding domain-containing protein MBD3 (MBD3) mRNA, complete cds."//0//1707bp//98%//AF072247
C-HEMBA1001711
C-HEMBA1001723//"Rattus norvegicus G beta-like protein GBL mRNA, complete cds."//4.7E-172//1240bp//81%//AF051155
C-HEMBA1001746//"Homo sapiens squamous cell carcinoma antigen recognized by T cell (SART-2) mRNA, complete cds."//7.6E-59//998bp//64%//AF098066
C-HEMBA1001781
C-HEMBA1001804//"Homo sapiens zinc finger DNA binding protein 99 (ZNF281) mRNA, complete cds."//0//1637bp//99%//AF125158
C-HEMBA1001822//"Mus musculus Ese2L protein mRNA, complete cds."//1.9E-235//1329bp//89%//AF132479
C-HEMBA1001824
C-HEMBA1001866//UDP-GLUCOSE:GLYCOPROTEIN GLUCOSYLTRANSFERASE PRECURSOR (EC 2.4.1.-) (DUGT).//5.7E-51//234aa//41%//Q09332
C-HEMBA1001910
C-HEMBA1001913//GCN20 PROTEIN.//2.3E-81//158aa//50%//P43535
C-HEMBA1001921//"Homo sapiens germinal center kinase related protein kinase mRNA, complete cds."//0//1850bp//99%//AF000145
C-HEMBA1001939
C-HEMBA1001950//"Homo sapiens mRNA for KIAA0971 protein, complete cds."//0//1974bp//99%//AB023188
C-HEMBA1001967//"Homo sapiens NY-REN-57 antigen mRNA, partial cds."//0//1721bp//99%//AF155114
C-HEMBA1002035//Homo sapiens mRNA; cDNA DKFZp586E0518 (from clone DKFZp586E0518).//0//2149bp//99%//AL050089
C-HEMBA1002092//"Mus musculus Olf-1/EBF-like-3 transcription factor (O/E-3) mRNA, complete cds."//1.3E-271//1583bp//88%//U92703
C-HEMBA1002102//ANKYRIN.//4.40E-10//106aa//35%//Q02357
C-HEMBA1002150
C-HEMBA1002151//"Rattus norvegicus p34 mRNA, complete cds."//1.1E-153//1059bp//82%//AF178669
C-HEMBA1002189
C-HEMBA1002215//TESTIN 2 (TES2) [CONTAINS: TESTIN 1 (TES1)].//2.2E-199//392aa//89%//P47226
C-HEMBA1002229
C-HEMBA1002241//PROLIFERATING-CELL NUCLEOLAR ANTIGEN P120 (PROLIFERATION-ASSOCIATED NUCLEOLAR PROTEIN P120).//3.70E-06//95aa//33%//P46087
C-HEMBA1002341//"Homo sapiens mRNA for KIAA0771 protein, partial cds."//0//1514bp//99%//AB018314
C-HEMBA1002417//"Homo sapiens chromosome 19, cosmid R28784, complete sequence."//1.4E-299//294bp//100%//AC005954
C-HEMBA1002547//"Homo sapiens agrin precursor mRNA, partial cds."//0//1605bp//97%//AF016903
C-HEMBA1002703
C-HEMBA1002779
C-HEMBA1002816
C-HEMBA1002970
C-HEMBA1002999//"Rattus norvegicus lamina associated polypeptide 1C (LAP1C) mRNA, complete cds."//1.4E-171//1552bp//75%//U20286
C-HEMBA1003021
C-HEMBA1003077//SLIT PROTEIN PRECURSOR.//2.6E-15//199aa//31%//P24014
C-HEMBA1003079
C-HEMBA1003273
C-HEMBA1003304
C-HEMBA1003309
C-HEMBA1003376
C-HEMBA1003384
C-HEMBA1003531
C-HEMBA1003548
C-HEMBA1003556
C-HEMBA1003571
C-HEMBA1003579
C-HEMBA1003684//ZINC FINGER PROTEIN 151 (MIZ-1 PROTEIN).//2E-73//526aa//32%//Q13105
C-HEMBA1003692
C-HEMBA1003720
C-HEMBA1003725
C-HEMBA1003729
C-HEMBA1003758
C-HEMBA1003773//"Mus musculus signal recognition particle receptor beta subunit mRNA, complete cds."//5.8E-81//511bp//86%//U17343
C-HEMBA1003783//"Mus musculus bromodomain-containing protein BP75 mRNA, complete cds."//1.1E-190//1204bp//84%//AF084259
C-HEMBA1003799
C-HEMBA1003804
C-HEMBA1003805//"Mus musculus KH domain RNA binding protein QKI-5A mRNA, complete cds."//0//988bp//95%//AF090402
C-HEMBA1003836//MOB1 PROTEIN (MPS1 BINDER 1).//8.10E-31//134aa//52%//P40484
C-HEMBA1003856
C-HEMBA1003866//"Mus musculus semaphorin VIa mRNA, complete cds."//1.2E-105//1192bp//70%//AF030430
C-HEMBA1003879
C-HEMBA1003880
C-HEMBA1003893
C-HEMBA1003908
C-HEMBA1003937
C-HEMBA1003942
C-HEMBA1003958
C-HEMBA1003976
C-HEMBA1003978//"Homo sapiens mRNA for KIAA0840 protein, partial cds."//0//1530bp//100%//AB020647
C-HEMBA1003985
C-HEMBA1004011
C-HEMBA1004024
C-HEMBA1004038
C-HEMBA1004045
C-HEMBA1004048
C-HEMBA1004111//"Homo sapiens mRNA for KIAA1276 protein, partial cds."//1.00E-163//751bp//99%//AB033102
C-HEMBA1004131//SEPTIN 2 HOMOLOG (FRAGMENT).//1.6E-166//416aa//72%//Q14141
C-HEMBA1004138
C-HEMBA1004143
C-HEMBA1004150
C-HEMBA1004168//"Homo sapiens geminin mRNA, complete cds."//3.9E-208//951 bp//99%//AF067855
C-HEMBA1004200
C-HEMBA1004202//RAS-RELATED PROTEIN RAB-13.//6.2E-30//208aa//37%//P51153
C-HEMBA1004203//NUCLEOLAR PROTEIN NOP2.//1.5E-12//258aa//29%//P40991
C-HEMBA1004238
C-HEMBA1004248//"Homo sapiens insulin induced protein 2 mRNA, complete cds."//8.20E-175//552bp//97%//AF125392
C-HEMBA1004272
C-HEMBA1004274
C-HEMBA1004275//"Homo sapiens mRNA for KIAA1111 protein, partial cds."//0//1341bp//99%//AB029034
C-HEMBA1004286//"Homo sapiens TGF beta receptor associated protein-1 mRNA, complete cds."//0//1982bp//99%//AF022795
C-HEMBA1004312
C-HEMBA1004321//ZINC FINGER PROTEIN 184 (FRAGMENT).//2.3E-93//357aa//42%//Q99676
C-HEMBA1004323
C-HEMBA1004327
C-HEMBA1004330
C-HEMBA1004341
C-HEMBA1004366
C-HEMBA1004372
C-HEMBA1004389//"Homo sapiens zinc finger DNA binding protein 99 (ZNF281) mRNA, complete cds."//0//1437bp//99%//AF125158
C-HEMBA1004394
C-HEMBA1004408//PEPTIDYL-PROLYL CIS-TRANS ISOMERASE 10 (EC 5.2.1.8) (PPIASE) (ROTAMASE) (CYCLOPHILIN-10).//3.2E-32//148aa//52%//P52017
C-HEMBA1004429
C-HEMBA1004460
C-HEMBA1004461
C-HEMBA1004502
C-HEMBA1004554
C-HEMBA1004560
C-HEMBA1004610
C-HEMBA1004629
C-HEMBA1004632
C-HEMBA1004637
C-HEMBA1004670
C-HEMBA1004672
C-HEMBA1004697
C-HEMBA1004711
C-HEMBA1004725
C-HEMBA1004730
C-HEMBA1004734//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//9.9E-39//143aa//52%//P42743
C-HEMBA1004751
C-HEMBA1004752
C-HEMBA1004889//"Human C3f mRNA, complete cds."//6.70E-24//341aa//26%//U72515
C-HEMBA1004934
C-HEMBA1004944
C-HEMBA1004973
C-HEMBA1004977
C-HEMBA1005009//"Homo sapiens BAF53a (BAF53a) mRNA, complete cds."//0//1813bp//99%//AF041474
C-HEMBA1005083
C-HEMBA1005113
C-HEMBA1005133
C-HEMBA1005185
C-HEMBA1005219//NUCLEAR PROTEIN SNF7.//5.3E-10//189aa//25%//P39929
C-HEMBA1005252//"Homo sapiens mRNA for KIAA0585 protein, partial cds."//1.2E-268//1215bp//99%//AB011157
C-HEMBA1005296
C-HEMBA1005314
C-HEMBA1005331
C-HEMBA1005394
C-HEMBA1005403
C-HEMBA1005423//"Homo sapiens cyclin-dependent kinase inhibitor (CDKN2C) mRNA, complete cds."//2E-213//537bp//99%//AF041248
C-HEMBA1005468
C-HEMBA1005469
C-HEMBA1005474
C-HEMBA1005517
C-HEMBA1005518
C-HEMBA1005528//CCR4-ASSOCIATED FACTOR 1 (CAF1).//3.1E-154//285aa//99%//Q60809
C-HEMBA1005558//NUCLEAR PROTEIN SNF7.//6.40E-16//170aa//31%//P39929
C-HEMBA1005576//"Homo sapiens mRNA for KIAA0463 protein, partial cds."//1.1E-181//835bp//99%//AB007932
C-HEMBA1005582//"TROPOMYOSIN 1, NON-MUSCLE ISOFORM (TROPOMYOSIN II) (CYTOSKELETAL TROPOMYOSIN)."//0.00000009//213aa//27%//P09492
C-HEMBA1005583
C-HEMBA1005595//"DYNEIN HEAVY CHAIN, CYTOSOLIC (DYHC)."//2.3E-54//562aa//29%//P34036
C-HEMBA1005609//Homo sapiens mRNA; cDNA DKFZp564K133 (from clone DKFZp564K133).//2.2e-315//1448bp//99%//AL050012
C-HEMBA1005621//"Homo sapiens Mad2B protein (MAD2B) mRNA, complete cds."//2.9E-224//1031bp//99%//AF139365
C-HEMBA1005666
C-HEMBA1005680
C-HEMBA1005685
C-HEMBA1005737//CALCINEURIN B SUBUNIT (PROTEIN PHOSPHATASE 2B REGULATORY SUBUNIT).//4.4E-17//167aa//34%//P25296
C-HEMBA1005746
C-HEMBA1005755
C-HEMBA1005813
C-HEMBA1005822
C-HEMBA1005834
C-HEMBA1005884
C-HEMBA1005891
C-HEMBA1005909
C-HEMBA1005911
C-HEMBA1005931
C-HEMBA1005963
C-HEMBA1005991
C-HEMBA1006005
C-HEMBA1006031//"Homo sapiens mRNA for putative phospholipase, complete cds."//0//1413bp//99%//AB019435
C-HEMBA1006067
C-HEMBA1006081
C-HEMBA1006091
C-HEMBA1006100
C-HEMBA1006108//"Homo sapiens mRNA for KIAA0943 protein, partial cds."//4.8E-245//764bp//99%//AB023160
C-HEMBA1006121
C-HEMBA1006130//SEL-10 PROTEIN.//0.000000043//219aa//25%//Q93794
C-HEMBA1006155
C-HEMBA1006158//"Homo sapiens transcription factor forkhead-like 7 (FKHL7) gene, complete cds."//0//1551bp//99%//AF048693
C-HEMBA1006182
C-HEMBA1006198//PROLINE-RICH PROTEIN MP-2 PRECURSOR.//1.9E-19//215aa//39%//P05142
C-HEMBA1006235//Homo sapiens clone 24422 mRNA sequence.//0//1615bp//99%//AF070557
C-HEMBA1006253//DNA-DAMAGE-REPAIR/TOLERATION PROTEIN DRT111 PRECURSOR.//0.00000002//62aa//53%//P42698
C-HEMBA1006259
C-HEMBA1006272//RETROVIRUS-RELATED PROTEASE (EC 3.4.23.-).//1.3E-123//200aa//73%//P10265
C-HEMBA1006278//POLY(A) POLYMERASE (EC 2.7.7.19) (PAP) (POLYNUCLEOTIDE ADENYLYLTRANSFERASE).//1E-210//490aa//77%//P25500
C-HEMBA1006283//NUCLEAR POLYADENYLATED RNA-BINDING PROTEIN NAB2.//0.000000012//176aa//30%//P32505
C-HEMBA1006284
C-HEMBA1006291//2-ARYLPROPIONYL-COA EPIMERASE (EC 5.-.-.-).//4.2E-12//215aa//23%//P70473
C-HEMBA1006293
C-HEMBA1006309//HYPOTHETICAL 54.2 KD PROTEIN IN ERP5-ORC6 INTERGENIC REGION.//1.4E-48//248aa//43%//P38821
C-HEMBA1006349
C-HEMBA1006364
C-HEMBA1006381
C-HEMBA1006398//"Human L1 element L1.6 putative p150 gene, complete cds."//2E-277//1729bp//85%//U93563
C-HEMBA1006445//"Homo sapiens putative tumor supressor NOEY2 mRNA, complete cds."//1.4E-270//1224bp//100%//U96750
C-HEMBA1006483
C-HEMBA1006492
C-HEMBA1006497
C-HEMBA1006502
C-HEMBA1006507//"Homo sapiens mRNA for KIAA0666 protein, partial cds."//0//2334bp//99%//AB014566
C-HEMBA1006535
C-HEMBA1006559//"Mus musculus PRAJA1 (Praja1) mRNA, complete cds."//2.8E-206//1107bp//83 %//U06944
C-HEMBA1006566
C-HEMBA1006579
C-HEMBA1006583
C-HEMBA1006612
C-HEMBA1006624//DNA/PANTOTHENATE METABOLISM FLAVOPROTEIN HOMOLOG.//0.00000069//109aa//38%//Q58323
C-HEMBA1006643
C-HEMBA1006674
C-HEMBA1006682
C-HEMBA1006708//HYPOTHETICAL 46.4 KD TRP-ASP REPEATS CONTAINING PROTEIN IN PMC1-TFG2 INTERGENIC REGION.//3.3E-22//241aa//31%//P53196
C-HEMBA1006717
C-HEMBA1006744
C-HEMBA1006754
C-HEMBA1006767
C-HEMBA1006789
C-HEMBA1006832
C-HEMBA1006885//"Homo sapiens gene for Proline synthetase associated, complete cds."//0//1467bp//96%//AB018566
C-HEMBA1006900
C-HEMBA1006926
C-HEMBA1006941//Homo sapiens mRNA for putative thioredoxin-like protein.//1.8E-226//1039bp//99%//AJ010841
C-HEMBA1006973//"Homo sapiens rab3-GAP regulatory domain mRNA, complete cds."//5.6E-143//740bp//94%//AF004828
C-HEMBA1006993
C-HEMBA1007002
C-HEMBA1007062
C-HEMBA1007080
C-HEMBA1007087//HYPOTHETICAL PROTEIN MJ0162.//2E-45//304aa//32%//Q57626
C-HEMBA1007112//Homo sapiens mRNA; cDNA DKFZp586C1817 (from clone DKFZp586C1817).//0//1619bp//99%//AL117450
C-HEMBA1007194//"Homo sapiens origin recognition complex subunit 6 (ORC6) mRNA, complete cds."//0//1588bp//99%//AF139658
C-HEMBA1007206
C-HEMBA1007256
C-HEMBA1007267
C-HEMBA1007281
C-HEMBA1007300//"Homo sapiens 3',5'-cyclic nucleotide phosphodiesterase 10A1 (PDE10A) mRNA, splice variant 1, complete cds."//0//1519bp//99%//AF127479
C-HEMBA1007301
C-HEMBA1007319
C-HEMBA1007320
C-HEMBA1007327
C-HEMBA1007347
C-HEMBB1000005
C-HEMBB1000030
C-HEMBB1000048
C-HEMBB1000099
C-HEMBB1000141
C-HEMBB1000198
C-HEMBB1000217//"Homo sapiens SUMO-1-activating enzyme E1 N subunit (SUA1) mRNA, complete cds."//0//1038bp//99%//AF090385
C-HEMBB1000218
C-HEMBB1000274
C-HEMBB1000312
C-HEMBB1000402
C-HEMBB1000420
C-HEMBB1000480
C-HEMBB1000530
C-HEMBB1000550
C-HEMBB10000556//"Homo sapiens mRNA for KIAA0750 protein, complete cds."//6.3E-74//1213bp//64%//AB018293
C-HEMBB1000586
C-HEMBB1000592
C-HEMBB1000593//"Homo sapiens transferrin receptor 2 alpha (TFR2) mRNA, complete cds."//1.3E-107//503bp//99%//AF067864
C-HEMBB1000649
C-HEMBB1000693//"Homo sapiens neuroan1 mRNA, complete cds."//0//2952bp//94%//AF040723
C-HEMBB1000822
C-HEMBB1000826
C-HEMBB1000890
C-HEMBB1000915//SUBTILISIN-LIKE PROTEASE PACE4 PRECURSOR (EC 3.4.21.-).//1.10E-08//129aa//31%//P29122
C-HEMBB1001008
C-HEMBB1001020//"Homo sapiens mRNA for KIAA0889 protein, complete cds."//0//1812bp//98%//AB020696
C-HEMBB1001051
C-HEMBB1001112//"Homo sapiens sec61 homolog mRNA, complete cds."//6E-145//961bp//83%//AF077032
C-HEMBB1001221
C-HEMBB1001234//65 KD YES-ASSOCIATED PROTEIN (YAP65).//5.4E-93//196aa//54%//P46938
C-HEMBB1001282//ANKYRIN R (ANKYRINS 2.1 AND 2.2) (ERYTHROCYTE ANKYRIN).//7E-43//394aa//34%//P16157
C-HEMBB1001302
C-HEMBB1001335
C-HEMBB1001337
C-HEMBB1001356
C-HEMBB1001364
C-HEMBB1001366
C-HEMBB1001367
C-HEMBB1001527
C-HEMBB1001537
C-HEMBB1002359
C-HEMBB1002415
C-HEMBB1002457
C-HEMBB1002492
C-HEMBB1002495
C-HEMBB1002502
C-HEMBB1002550//HYPOTHETICAL UOG-1 PROTEIN.//5E-28//266aa//33%//P27544
C-HEMBB1002600//"Homo sapiens tetraspan NET-5 mRNA, complete cds."//0//1417bp//99%//AF089749
C-HEMBB1002607//"Homo sapiens vitamin D3 receptor interacting protein (DRIP80) mRNA, complete cds."//2E-136//660bp//98%//AF105421
C-HEMBB1002684
C-HEMBB1002692
C-HEMBB1002697
C-HEMBB1002705//"Homo sapiens CGI-27 protein mRNA, complete cds."//7.80E-285//841bp//96%//AF132961
C-MAMMA1000019
C-MAMMA1000020//H.sapiens mRNA for flavin-containing monooxygenase 5 (FM05).//8.2E-198//868bp//99%//Z47553
C-MAMMA1000025
C-MAMMA1000055//TESTIN 2 (TES2) [CONTAINS: TESTIN 1 (TES1)].//1.5E-90//323aa//48%//P47226
C-MAMMA1000069
C-MAMMA1000084
C-MAMMA1000139
C-MAMMA1000163
C-MAMMA1000171
C-MAMMA1000173//"Homo sapiens src homology 3 domain-containing protein HIP-55 mRNA, complete cds."//2.6E-164//1044bp//87%//AF197060
C-MAMMA1000277
C-MAMMA1000278
C-MAMMA1000284//P.walti mRNA for mp associated protein 55.//2.2E-109//864bp//76%//X99836
C-MAMMA1000309
C-MAMMA1000312
C-MAMMA1000313
C-MAMMA1000361
C-MAMMA1000388//"Homo sapiens UKLF mRNA for ubiquitous Kruppel like factor, complete cds."//0//1466bp//99%//AB015132
C-MAMMA1000395
C-MAMMA1000410
C-MAMMA1000416//HYPOTHETICAL 32.0 KD PROTEIN C09F5.2 IN CHROMOSOME III.//2.00E-30//119aa//53%//Q09232
C-MAMMA1000421
C-MAMMA1000422
C-MAMMA1000468
C-MAMMA1000472
C-MAMMA1000490
C-MAMMA1000524
C-MAMMA1000567
C-MAMMA1000612//"Rattus norvegicus G beta-like protein GBL mRNA, complete cds."//1E-95//1115bp//72%//AF051155
C-MAMMA1000623
C-MAMMA1000625//GYP7 PROTEIN.//2.1E-41//198aa//40%//P48365
C-MAMMA1000664
C-MAMMA1000670
C-MAMMA1000672//VITELLOGENIC CARBOXYPEPTIDASE PRECURSOR (EC 3.4.16.-).//4.4E-33//250aa//33%//P42660
C-MAMMA1000713//L-RIBULOKINASE (EC 2.7.1.16).//7.70E-17//246aa//29%//P94524
C-MAMMA1000731//CHROMODOMAIN-HELICASE-DNA-BINDING PROTEIN 1 (CHD-1).//1E-77//395aa//45%//014646
C-MAMMA1000734//Homo sapiens mRNA for SEC63 protein.//0//1587bp//99%//AJ011779
C-MAMMA1000738//HYPOTHETICAL 116.5 KD PROTEIN C20G8.09C IN CHROMOSOME I.//9E-299//1033aa//55%//P87115
C-MAMMA1000746
C-MAMMA1000775
C-MAMMA1000824//ACTIN.//6.2E-20//284aa//28%//P53500
C-MAMMA1000831
C-MAMMA1000841//PUTATIVE AMIDASE (EC 3.5.1.4).//7.8E-40//101aa//54%//027540
C-MAMMA1000842
C-MAMMA1000843
C-MAMMA1000856
C-MAMMA1000865
C-MAMMA1000875
C-MAMMA1000906
C-MAMMA1000908
C-MAMMA1000914
C-MAMMA1000956//Homo sapiens CLDN8 gene for claudin-8.//0//1767bp//99%//AJ250711
C-MAMMA1000968
C-MAMMA1000979
C-MAMMA1001008//"Homo sapiens aspartic-like protease mRNA, complete cds."//2.50E-276//1263bp//99%//AF117892
C-MAMMA1001021
C-MAMMA1001041//"SPECTRIN BETA CHAIN, BRAIN (SPECTRIN, NON-ERYTHROID BETA CHAIN) (FODRIN BETA CHAIN) (SPTBN1)."//1.6E-16//113aa//41%//Q01082
C-MAMMA1001059//Homo sapiens mRNA for DEAD Box Protein 5.//0//1440bp//99%//AJ237946
C-MAMMA1001075//"Homo sapiens CGI-72 protein mRNA, complete cds."//1.3E-181//397bp//98%//AF151830
C-MAMMA1001078
C-MAMMA1001091
C-MAMMA1001105//OVO PROTEIN (SHAVEN BABY PROTEIN).//4E-49//125aa//68%//P51521
C-MAMMA1001110
C-MAMMA1001126
C-MAMMA1001139//SRE-2 PROTEIN.//5.80E-35//239aa//38%//Q09273
C-MAMMA1001143
C-MAMMA1001154
C-MAMMA1001181//ABC1 PROTEIN HOMOLOG PRECURSOR.//1.30E-07//81aa//45%//Q92338
C-MAMMA1001215
C-MAMMA1001244
C-MAMMA1001259//"Mus musculus F-box protein FBX18 mRNA, partial cds."//2.3E-271//1414bp//89%//AF184275
C-MAMMA1001260//HYPOTHETICAL 97.1 KD PROTEIN R05D3.4 IN CHROMOSOME III.//2.1E-52//630aa//30%//P34537
C-MAMMA1001343
C-MAMMA1001411//Homo sapiens mRNA; cDNA DKFZp56400823 (from clone DKFZp56400823).//0//2131bp//99%//AL080121
C-MAMMA1001419
C-MAMMA1001476//URIDINE KINASE (EC 2.7.1.48) (URIDINE MONOPHOSPHOKINASE) (FRAGMENT).//6.5E-129//260aa//92%//P52623
C-MAMMA1001510
C-MAMMA1001522
C-MAMMA1001576//"Human gamma-tubulin mRNA, complete cds."//7.5E-276//1561bp//90%//M61764
C-MAMMA1001604
C-MAMMA1001620
C-MAMMA1001635
C-MAMMA1001649
C-MAMMA1001686
C-MAMMA1001692
C-MAMMA1001743//Y BOX BINDING PROTEIN-1 (Y-BOX TRANSCRIPTION FACTOR).//8.5E-32//171aa//36%//P21573
C-MAMMA1001754//"Homo sapiens CGI-11 protein mRNA, complete cds."//0//1837bp//98%//AF132945
C-MAMMA1001757
C-MAMMA1001764
C-MAMMA1001768//CELL DIVISION CYCLE PROTEIN 48 HOMOLOG MJ1156.//3.8E-45//351aa//38%//Q58556
C-MAMMA1001771//M.musculus mRNA for semaphorin B.//2.60E-200//1272bp//79%//X85991
C-MAMMA1001790
C-MAMMA1001837//ZINC FINGER PROTEIN 29 (ZFP-29).//2.6E-77//507aa//38%//Q07230
C-MAMMA1001858
C-MAMMA1001868//TRICHOHYALIN.//2.7E-19//359aa//25%//P22793
C-MAMMA1001970
C-MAMMA1002042
C-MAMMA1002068
C-MAMMA1002153
C-MAMMA1002156
C-MAMMA1002170//40S RIBOSOMAL PROTEIN S2 (S4) (LLREP3 PROTEIN).//6E-66//157aa//70%//P15880
C-MAMMA1002174
C-MAMMA1002209
C-MAMMA1002219//"Homo sapiens mRNA for KIAA1067 protein, partial cds."//1.1E-181//861bp//98%//AB028990
C-MAMMA1002236//TRANSLATION INITIATION FACTOR EIF-2B GAMMA SUBUNIT (EIF-2B GDP-GTP EXCHANGE FACTOR).//8.8E-217//310aa//86%//P70541
C-MAMMA1002243
C-MAMMA1002268//"Mus musculus sphingosine kinase (SPHKIa) mRNA, partial cds."//1E-190//1624bp//76%//AF068748
C-MAMMA1002269
C-MAMMA1002292
C-MAMMA1002294
C-MAMMA1002297//Homo sapiens mRNA for Rab6 GTPase activating protein.//1.1E-214//881bp//97%//AJ011679
C-MAMMA1002312
C-MAMMA1002329//M.musculus mRNA for semaphorin B.//3.80E-45//332bp//84%//X85991
C-MAMMA1002333
C-MAMMA1002351//FERRIPYOCHELIN BINDING PROTEIN.//0.000078//127aa//26%//P40882
C-MAMMA1002353
C-MAMMA1002355
C-MAMMA1002356
C-MAMMA1002362
C-MAMMA1002380
C-MAMMA1002384
C-MAMMA1002427
C-MAMMA1002470//PROBABLE NH(3)-DEPENDENT NAD(⁺) SYNTHETASE (EC 6.3.5.1).//1E-11//128aa//36%//P47623
C-MAMMA1002485//"Homo sapiens stanniocalcin-related protein mRNA, complete cds."//0//1822bp//99%//AF098462
C-MAMMA1002494
C-MAMMA1002524//HYPOTHETICAL 117.8 KD PROTEIN IN STE2-FRS2 INTERGENIC REGION.//1.2E-34//337aa//31%//P43571
C-MAMMA1002530//"Homo sapiens cytosolic phospholipase A2 gamma (cPLA2 gamma) mRNA, complete cds."//0//1910bp//99%//AF065214
C-MAMMA1002554
C-MAMMA1002585//"Homo sapiens mRNA for KIAA0860 protein, complete cds."//0//1405bp//99%//AB020667
C-MAMMA1002598
C-MAMMA1002619//PROBABLE UBIQUITIN CARBOXYL-TERMINAL HYDROLASE K02C4.3 (EC 3.1.2.15) (UBIQUITIN THIOLESTERASE) (UBIQUITIN-SPECIFIC PROCESSING PROTEASE) (DEUBIQUITINATING ENZYME).//9.5E-16//159aa//37%//Q09931
C-MAMMA1002655//"Homo sapiens mRNA for ganglioside sialidase, complete cds."//0//1515bp//99%//AB008185
C-MAMMA1002671//ACETYL-COENZYME A SYNTHETASE (EC 6.2.1.1) (ACETATE--COA LIGASE) (ACYL- ACTIVATING ENZYME).//1.1E-45//618aa//26%//P27550
C-MAMMA1002673
C-MAMMA1002684//"Homo sapiens mRNA for KIAA0214 protein, complete cds."//0//3174bp//99%//D86987
C-MAMMA1002711
C-MAMMA1002769//"Homo sapiens cell cycle progression restoration 8 protein (CPR8) mRNA, complete cds."//2.2E-25//330bp//77%//AF011794
C-MAMMA1002775
C-MAMMA1002782
C-MAMMA1002796
C-MAMMA1002807
C-MAMMA1002838
C-MAMMA1002842//"Mus musculus c-Cb1 associated protein CAP mRNA, complete cds."//2.6E-58//373bp//81%//U58883
C-MAMMA1002869//PINCH PROTEIN (PARTICULARY INTERESTING NEW CYS-HIS PROTEIN).//1.4E-160//305aa//85%//P48059
C-MAMMA1002881//GLIOMA PATHOGENESIS-RELATED PROTEIN (RTVP-1 PROTEIN).//5.7E-30//214aa//35%//P48060
C-MAMMA1002886
C-MAMMA1002890
C-MAMMA1002938//"Homo sapiens mRNA for KIAA0698 protein, complete cds."//8.4E-252//1139bp//100%//AB014598
C-MAMMA1002964
C-MAMMA1003011//HESTONE MACRO-H2A.1.//2.7E-123//370aa//66%//Q02874
C-MAMMA1003013//DNA POLYMERASE BETA (EC 2.7.7.7).//7.4E-46//332aa//36%//P06746
C-MAMMA1003015
C-MAMMA1003019
C-MAMMA1003035//RIBOSOMAL LARGE SUBUNIT PSEUDOURIDINE SYNTHASE C (EC 4.2.1.70) (PSEUDOURIDYLATE SYNTHASE) (URACIL HYDROLYASE).//1.9E-13//108aa//33%//P23851
C-MAMMA1003039
C-MAMMA1003044
C-MAMMA1003049
C-MAMMA1003056
C-MAMMA1003057//MD6 PROTEIN.//3.1E-225//419aa//97%//Q60584
C-MAMMA1003066
C-MAMMA1003099
C-MAMMA1003104
C-MAMMA1003113//"Mus musculus COP9 complex subunit 7a (COPS7a) mRNA, complete cds."//1.1E-234//1178bp//86%//AF071316
C-MAMMA1003127//MYOSIN I ALPHA (MMI-ALPHA).//2.2E-105//217aa//89%//P46735
C-MAMMA1003135
C-MAMMA1003146//Homo sapiens mRNA for GalT3 protein.//4.3E-218//996bp//99%//Y15062
C-MAMMA1003150//"Homo sapiens mRNA for KIAA1096 protein, partial cds."/0//1342bp//99%//AB029019
C-MAMMA1003166//"Homo sapiens MLL septin-like fusion protein (MSF) mRNA, complete cds."//3.10E-158//592bp//97%//AF123052
C-NT2RM1000032
C-NT2RM1000035//"Human mRNA for KIAA0199 gene, partial cds."//0//2948bp//99%//D83782
C-NT2RM1000039//HYPOTHETICAL 41.4 KD PROTEIN IN SRLQ-HYPF INTERGENIC REGION (EC 1.18.1.-) (ORF4) (ORF2).//2.90E-14//299aa//25%//P37596
C-NT2RM1000055//"Homo sapiens mRNA for KIAA0829 protein, partial cds."//0//3111bp//99%//AB020636
C-NT2RM1000059
C-NT2RM1000062
C-NT2RM1000118//CALCINEURIN B SUBUNIT (PROTEIN PHOSPHATASE 2B REGULATORY SUBUNIT) (CALCINEURIN REGULATORY SUBUNIT).//1.2E-10//150aa//28%//P87072
C-NT2RM1000119
C-NT2RM1000127
C-NT2RM1000131//"Homo sapiens mRNA for KIAA0792 protein, complete cds."//0//2980bp//99%//AB018335
C-NT2RM1000132//"Homo sapiens NADH:ubiquinone oxidoreductas NDUFS6 subunit mRNA, nuclear gene encoding mitochondrial protein, complete cds."//7.8E-110//516bp//99%//AF044959
C-NT2RM1000153//CYTOSOLIC PURINE 5'-NUCLEOTIDASE (EC 3.1.3.5).//3.3E-3 8//469aa//27%//P49902
C-NT2RM1000186//CALCINEURIN B SUBUNIT (PROTEIN PHOSPHATASE 2B REGULATORY SUBUNIT) (CALCINEURIN REGULATORY SUBUNIT).//1.2E-10//150aa//28%//P87072
C-NT2RM1000187//PUTATIVE PRE-MRNA SPLICING FACTOR ATP-DEPENDENT RNA HELICASE SPAC10F6.02C.//1.1E-10//94aa//47%//042643
C-NT2RM1000199//Homo sapiens mRNA for type I transmembrane receptor (psk-1 gene).//0//2476bp//99%//AJ245820
C-NT2RM1000244//"Homo sapiens TRAF4 associated factor 1 mRNA, partial cds."//2E-126//592bp//99%//U81002
C-NT2RM1000252//H.sapiens E-MAP-115 mRNA.//9.7E-35//569bp//64%//X73882
C-NT2RM1000256//"Homo sapiens mRNA for Glutamine:fructose-6-phosphate amidotransferase, complete cds."//0//3012bp//99%//AB016789
C-NT2RM1000260//"Human mRNA for KIAA0130 gene, complete cds."//0//3139bp//98%//D50920
C-NT2RM1000271
C-NT2RM1000300
C-NT2RM1000314//"Human mRNA for KIAA0159 gene, complete cds."//0//4349bp//99%//D63880
C-NT2RM1000354//"Xenopus laevis chromosome condensation protein XCAP-G mRNA, complete cds."//7.4E-245//2101bp//68%//AF111423
C-NT2RM1000355//"Homo sapiens transmembrane protein BRI (BRI) mRNA, complete cds."//0//1599bp//99%//AF152462
C-NT2RM1000365
C-NT2RM1000377//"Homo sapiens dual specificity phosphatase MKP5 (MKP5) mRNA, complete cds."//3.2E-196//1016bp//94%//AF179212
C-NT2RM1000388//HYPOTHETICAL 27.7 KD PROTEIN IN CPT1-SPC98 INTERGENIC REGION.//0.000000019//67aa//31%//P53915
C-NT2RM1000399
C-NT2RM1000430//"Homo sapiens erythroblast macrophage protein EMP mRNA, complete cds."//1.4E-185//1486bp//81%//AF084928
C-NT2RM1000555//"Homo sapiens mRNA for KIAA0885 protein, complete cds."//0//2885bp//99%//AB020692
C-NT2RM1000563//TRANSMISSION-BLOCKING TARGET ANTIGEN S230 PRECURSOR.//0.0000068//199aa//30%//Q08372
C-NT2RM1000648//GLYCOSYLTRANSFERASE ALG2 (EC 2.4.1.-).//8.5E-75//301aa//39%//P43636
C-NT2RM1000661//"Homo sapiens translation initiation factor 4e mRNA, complete cds."//4.3E-210//960bp//99%//AF038957
C-NT2RM1000666//DNA-BINDING PROTEIN A.//2.2E-09//165aa//34%//P16989
C-NT2RM1000672
C-NT2RM1000691//Homo sapiens mRNA for PLU-1 protein.//0//3104bp//99%//AJ132440
C-NT2RM1000699
C-NT2RM1000741//"Homo sapiens mRNA for KIAA0567 protein, partial cds."//1.1E-295//1338bp//99%//AB011139
C-NT2RM1000742//"Homo sapiens AC133 antigen mRNA, complete cds."//0//3524bp//99%//AF027208
C-NT2RM1000746//"Homo sapiens polyamine modulated factor-1 (PMF1) mRNA, complete cds."//6.70E-227//1043bp//99%//AF141310
C-NT2RM1000770//DXS6673E PROTEIN.//1.4E-39//194aa//48%//Q14202
C-NT2RM1000772//VEGETATTOLE INCOMPATIBILITY PROTEIN HET-E-1.//7.3E-15//280aa//27%//Q00808
C-NT2RM1000780
C-NT2RM1000800//Mus musculus partial mRNA for B-IND1 protein (B-indl gene).//1.1E-98//571bp//89%//Z97207
C-NT2RM1000802
C-NT2RM1000811//"Homo sapiens AC133 antigen mRNA, complete cds."//0//3524bp//99%//AF027208
C-NT2RM1000826//"Homo sapiens mRNA for KIAA0885 protein, complete cds."//0//2885bp//99%//AB020692
C-NT2RM1000829
C-NT2RM1000850//ANKYRIN R (ANKYRINS 2.1 AND 2.2) (ERYTHROCYTE ANKYRIN).//9.7E-42//333aa//36%//P16157
C-NT2RM1000852//"Homo sapiens putative ATP-dependent RNA helicase ROK1 mRNA, complete cds."//0//2206bp//99%//AF077033
C-NT2RM1000857//"Homo sapiens mRNA for KIAA0962 protein, partial cds."//0//3716bp//99%//AB023179
C-NT2RM1000874//"Homo sapiens death effector domain-containing testicular molecule mRNA, complete cds."//1.4E-244//1113bp//99%//AF043733
C-NT2RM1000882//"Homo sapiens delta-6 fatty acid desaturase mRNA, complete cds."//4.30E-122//1394bp//69%//AF126799
C-NT2RM1000885//HYPOTHETICAL 97.1 KD PROTEIN R05D3.4 IN CHROMOSOME III.//1.8E-56//630aa//30%//P34537
C-NT2RM1000894//DNA-DIRECTED RNA POLYMERASE 1135 KD POLYPEPTIDE (EC 2.7.7.6) (RNA POLYMERASE I SUBUNIT 2) (RPA135).//0//1020aa//89%//P70700
C-NT2RM1000898//"ACTIN, CYTOPLASMIC (ACTIN, MICRONUCLEAR)."//8.9E-26//229aa//29%//P02583
C-NT2RM1000905//"Homo sapiens HSPC021 mRNA, complete cds."//0//1480bp//99%//AF077207
C-NT2RM1000924//HYPOTHETICAL 39.7 KD PROTEIN C34E10.2 IN CHROMOSOME III.//1E-15//266aa//26%//P46577
C-NT2RM1000927
C-NT2RM1000962
C-NT2RM1000978
C-NT2RM1001003//"Homo sapiens alpha-catenin-like protein (CTNNAL1) mRNA, complete cds."//0//2230bp//99%//AF030233
C-NT2RM1001043
C-NT2RM1001066
C-NT2RM1001072//"1-PHOSPHATIDYLINOSITOL-4,5-BISPHOSPHATE PHOSPHODIESTERASE GAMMA 1 (EC 3.1.4.11) (PLC-GAMMA-1) (PHOSPHOLIPASE C-GAMMA-1) (PLC-II) (PLC-148)."//8.3E-47//259aa//35%//P08487
C-NT2RM1001085//"Rattus norvegicus brain specific cortactin-binding protein CBP90 mRNA, partial cds."//3.7E-32//460bp//64%//AF053768
C-NT2RM1001102//"Human HEM45 mRNA, complete cds."//2.3E-27//482bp//63%//U88964
C-NT2RM1001105
C-NT2RM1001139//Homo sapiens mRNA; cDNA DKFZp564F0522 (from clone DKFZp564F0522).//0//1756bp//99%//AL049943
C-NT2RM2000420
C-NT2RM2000566//"Homo sapiens integrin alpha-7 mRNA, complete cds."//0//2519bp//96%//AF032108
C-NT2RM2000609
C-NT2RM2000612//"Rattus norvegicus ADP-ribosylation factor-directed GTPase activating protein mRNA, complete cds."//2.6E-106//1069bp//74%//U35776
C-NT2RM2000735//ZINC FINGER PROTEIN 43 (ZINC PROTEIN HTF6).//2.9E-103//249aa//73%//P28160
C-NT2RM2001588
C-NT2RM2001605//Homo sapiens mRNA for PLU-1 protein.//0//3114bp//99%//AJ132440
C-NT2RM2001613//"Homo sapiens sec61 homolog mRNA, complete cds."//0//2601bp//99%//AF084458
C-NT2RM2001632//KES 1 PROTEIN.//1.40E-31//342aa//34%//P35844
C-NT2RM2001648//"Homo sapiens sec61 homolog mRNA, complete cds."//0//2421bp//99%//AF084458
C-NT2RM2001652//"Homo sapiens guanine nucleotide exchange factor mRNA, complete cds."//0//2608bp//99%//AF111162
C-NT2RM2001659//ZINC/CADMIUM RESISTANCE PROTEIN.//3.4E-39//161aa//34%//P20107
C-NT2RM2001664//"Homo sapiens IkappaB kinase complex associated protein (IKAP) mRNA, complete cds."//0//2471bp//99%//AF044195
C-NT2RM2001668//"Homo sapiens putative WHSC1 protein (WHSC1) mRNA, alternative splice product ending in intron 11, complete cds."//6.2E-16//464bp//62%//AF083391
C-NT2RM2001671//"Oryctolagus cuniculus sarcolemmal associated protein (SLAP1) mRNA, complete cds."//0//1843bp//94%//U21155
C-NT2RM2001675
C-NT2RM2001681
C-NT2RM2001688//HYPOTHETICAL 33.8 KD PROTEIN C5H10.01 IN CHROMOSOME I.//4.60E-20//253aa//30%//Q09674
C-NT2RM2001695//Homo sapiens clone H63 unknown mRNA.//0//2016bp//99%//AF103804
C-NT2RM2001696
C-NT2RM2001698//"Homo sapiens XGalT-1 mRNA for galactosyltransferase I, complete cds."//6.2E-253//1170bp//99%//AB028600
C-NT2RM2001700//"ACYL-COA DEHYDROGENASE, VERY-LONG-CHAIN SPECIFIC (EC 1.3.99.-) (VLCAD) (FRAGMENT)."//5.7E-130//536aa//49%//P50544
C-NT2RM2001716
C-NT2RM2001723
C-NT2RM2001730//PROBABLE UBIQUITIN CARBOXYL-TERMINAL HYDROLASE K02C4.3 (EC 3.1.2.15) (UBIQUITIN THIOLESTERASE) (UBIQUITIN-SPECIFIC PROCESSING PROTEASE) (DEUBIQUITINATING ENZYME).//7.2E-16//381aa//27%//Q09931
C-NT2RM2001743//"Homo sapiens cell cycle progression 2 protein (CPR2) mRNA, complete cds."//0//1498bp//99%//AF011792
C-NT2RM2001753//HYPOTHETICAL PROTEIN KIAA0210.//8.8E-11//119aa//36%//Q92609
C-NT2RM2001760//"Homo sapiens sec61 homolog mRNA, complete cds."//0//2379bp//99%//AF084458
C-NT2RM2001768
C-NT2RM2001771//ZINC FINGER PROTEIN 135.//6.4E-154//394aa//64%//P52742
C-NT2RM2001782//"Homo sapiens GDP-mannose pyrophosphorylase A (GMPPA) mRNA, complete cds."//0//1470bp//99%//AF135422
C-NT2RM2001784
C-NT2RM2001785//Homo sapiens mRNA; cDNA DKFZp586C201 (from clone DKFZp586C201).//0//2146bp//99%//AL050118
C-NT2RM2001813
C-NT2RM2001823//CHD1 PROTEIN.//1.8E-106//631aa//39%//P32657
C-NT2RM2001839//"Homo sapiens calumein (Calu) mRNA, complete cds."//0//2415bp//97%//AF013759
C-NT2RM2001840
C-NT2RM2001855
C-NT2RM2001867//"Homo sapiens mRNA for KIAA0943 protein, partial cds."//0//967bp//99%//AB023160
C-NT2RM2001879
C-NT2RM2001983//"Homo sapiens RGS-GAIP interacting protein GIPC mRNA, complete cds."//0//1658bp//98%//AF089816
C-NT2RM2002145//"Homo sapiens erythroblast macrophage protein EMP mRNA, complete cds."//8.5E-191//1524bp//81%//AF084928
C-NT2RM4000027
C-NT2RM4000030//LAS1 PROTEIN.//5.6E-12//184aa//32%//P36146
C-NT2RM4000046//GOLIATH PROTEIN (G1 PROTEIN).//0.000008//112aa//31%//Q06003
C-NT2RM4000155//"THREONYL-TRNA SYNTHETASE, CYTOPLASMIC (EC 6.1.1.3) (THREONINE--TRNA LIGASE) (THRRS)."//1.2E-157//321aa//6l%//P26639
C-NT2RM4000156//H.sapiens HPBRII-7 gene.//3.6E-21//785bp//60%//X67336
C-NT2RM4000167//"Homo sapiens kinesin superfamily motor KIF4 mRNA, complete cds."//0//1946bp//99%//AF071592
C-NT2RM4000199
C-NT2RM4000200
C-NT2RM4000202//ZINC FINGER PROTEIN MOK-2 (HOK-2).//4.9E-32//170aa//41%//Q16600
C-NT2RM4000233//"Mus musculus semaphorin VIa mRNA, complete cds."//3.4E-231//1395bp//86%//AF030430
C-NT2RM4000244
C-NT2RM4000251
C-NT2RM4000265
C-NT2RM4000324
C-NT2RM4000327
C-NT2RM4000356//RAS-RELATED PROTEIN RAB-17.//5.9E-80//213aa//75%//P35292
C-NT2RM4000425
C-NT2RM4000433//"Mus musculus retinoic acid-responsive protein (Stra6) mRNA, complete cds."//4.1E-271//2085bp//77%//AF062476
C-NT2RM4000514
C-NT2RM4000531//ZINC FINGER PROTEIN 29 (ZFP-29).//2.4E-89//389aa//43%//007230
C-NT2RM4000532
C-NT2RM4000534
C-NT2RM4000603
C-NT2RM4000611//VEGETATIBLE INCOMPATIBILITY PROTEIN HET-E-1.//2.9E-09//108aa//31%//Q00808
C-NT2RM4000616//ACETYL-COENZYME A SYNTHETASE (EC 6.2.1.1) (ACETATE--COA LIGASE) (ACYL- ACTIVATING ENZYME).//2.7E-146//420aa//60%//P27550
C-NT2RM4000674//HYPOTHETICAL SYMPORTER SLL1374.//1.2E-28//180aa//30%//P74168
C-NT2RM4000689
C-NT2RM4000698
C-NT2RM4000700
C-NT2RM4000712//"Homo sapiens ubiquitin hydrolyzing enzyme I (UBH1) mRNA, partial cds."//1E-136//1104bp//77%//AF022789
C-NT2RM4000717
C-NT2RM4000733//TRANSCRIPTION TERMINATION FACTOR RHO.//0.00000041//207aa//29%//P52154
C-NT2RM4000734//"Homo sapiens mRNA for KIAA0760 protein, partial cds."//0//2273bp//99%//AB018303
C-NT2RM4000741//"Homo sapiens hSGT1 mRNA for hSgt1p, complete cds."//0//2184bp//99%//D88208
C-NT2RM4000751//ZINC FINGER PROTEIN 184 (FRAGMENT).//3.9E-125//301aa//53%//Q99676
C-NT2RM4000764
C-NT2RM4000778
C-NT2RM4000787
C-NT2RM4000790
C-NT2RM4000795//"Homo sapiens mRNA for KIAA0951 protein, complete cds."//0//1847bp//96%//AB023168
C-NT2RM4000796
C-NT2RM4000798//"Homo sapiens brefeldin A-inhibited guanine nucleotide-exchange protein 2 mRNA, complete cds."//0//2603bp//99%//AF084521
C-NT2RM4000813
C-NT2RM4000820//VACUOLAR ATP SYNTHASE SUBUNIT AC45 PRECURSOR (EC 3.6.1.34) (V-ATPASE AC45 SUBUNIT).//1.10E-24//138aa//44%//P40682
C-NT2RM4000833
C-NT2RM4000848
C-NT2RM4000852
C-NT2RM4000855
C-NT2RM4000887
C-NT2RM4000895
C-NT2RM4000950
C-NT2RM4000979
C-NT2RM4001002//Homo sapiens mRNA; cDNA DKFZp586G0518 (from clone DKFZp586G0518).//0//2259bp//100%//AL050092
C-NT2RM4001032
C-NT2RM4001047//M025 PROTEIN.//8E-140//333aa//80%//Q06138
C-NT2RM4001054//"Homo sapiens sec61 homolog mRNA, complete cds."//3.1E-190//1315bp//81%//AF077032
C-NT2RM4001084//HYPOTHETICAL 105.6 KD PROTEIN C16C9.06C IN CHROMOSOME I.//0.000000032//165aa//33%//Q09820
C-NT2RM4001116//HYPOTHETICAL 216.3 KD PROTEIN R06F6.8 IN CHROMOSOME II.//5.9E-86//292aa//48%//Q09417
C-NT2RM4001140//HOMEOBOX PROTEIN MSH-D.//1E-11//103aa//38%//Q01704
C-NT2RM4001151
C-NT2RM4001155//ADRENAL MEDULLA 50 KD PROTEIN.//4.1E-197//445aa//78%//Q27969
C-NT2RM4001160
C-NT2RM4001187
C-NT2RM4001191//"Homo sapiens clone 24963 mRNA sequence, complete cds."//0//1950bp//99%//AF131737
C-NT2RM4001200//ZINC FINGER PROTEIN 135.//9.5E-135//375aa//60%//P52742
C-NT2RM4001203//"Homo sapiens mRNA for KIAA0839 protein, partial cds."//0//3047bp//99%//AB020646
C-NT2RM4001204//"Homo sapiens mRNA for KIAA1089 protein, partial cds."//0//2349bp//99%//AB029012
C-NT2RM4001217//"Homo sapiens nuclear matrix protein NRP/B (NRPB) mRNA, complete cds."//7.3E-148//1409bp//72%//AF059611
C-NT2RM4001256//"Xenopus laevis putative Zic3 binding protein mRNA, complete cds."//4.30E-55//289bp//77%//AF129131
C-NT2RM4001258
C-NT2RM4001309
C-NT2RM4001313//PHOSPHATIDYLINOSITOL 3-KINASE VPS34-LIKE (EC 2.7.1.137) (PI3-KINASE) (PTDINS-3-KINASE) (PI3K).//3.50E-35//124aa//65%//P54676
C-NT2RM4001316//"ACYL-COA DEHYDROGENASE, MEDIUM-CHAIN SPECIFIC PRECURSOR (EC 1.3.99.3) (MCAD)."//2.3E-31//334aa//30%//P08503
C-NT2RM4001320//"Homo sapiens mRNA for Neuroblastoma, complete cds."//1.8E-39//728bp//64%//D89016
C-NT2RM4001340//UTR4 PROTEIN (UNKNOWN TRANSCRIPT 4 PROTEIN).//1E-28//171aa//37%//P32626
C-NT2RM4001344//HYPOTHETICAL GTP-BINDING PROTEIN IN POP2-HOL1 INTERGENIC REGION.//8.1E-30//265aa//33%//P53742
C-NT2RM4001347//"Homo sapiens NY-REN-25 antigen mRNA, partial cds."//0//2300bp//99%//AF155103
C-NT2RM4001371//"Homo sapiens IDN3 mRNA, partial cds."//0//2524bp//99%//AB019494
C-NT2RM4001382//"Homo sapiens RanBP7/importin 7 mRNA, complete cds."//2.2E-237//1079bp//99%//AF098799
C-NT2RM4001384
C-NT2RM4001410
C-NT2RM4001411//"Mus musculus Pro-rich, PH, SH2 domain-containing signaling mediator (PSM) mRNA, complete cds."//0//1962bp//87%//AF020526
C-NT2RM4001412//"Homo sapiens nGAP mRNA, complete cds."//0//1918bp//99%//AF047711
C-NT2RM4001414
C-NT2RM4001437
C-NT2RM4001444//ISOLEUCYL-TRNA SYNTHETASE (EC 6.1.1.5) (ISOLEUCINE-TRNA LIGASE) (ILERS).//1.4E-118//444aa//46%//P73505
C-NT2RM4001454
C-NT2RM4001455
C-NT2RM4001483//ZINC FINGER PROTEIN 136.//5.1E-106//357aa//55%//P52737
C-NT2RM4001489//"Homo sapiens mRNA for KIAA0685 protein, complete cds."//0//1810bp//99%//AB014585
C-NT2RM4001522
C-NT2RM4001557//"Homo sapiens mRNA for KIAA1040 protein, partial cds."//0//1547bp//97%//AB028963
C-NT2RM4001565
C-NT2RM4001566//"Homo sapiens mRNA for KIAA1114 protein, complete cds."//0//1900bp//99%//AB029037
C-NT2RM4001582//"Mus musculus COP9 complex subunit 7b (COPS7b) mRNA, complete cds."//1.5E-284//1082bp//90%//AF071317
C-NT2RM4001592//"Homo sapiens mRNA for KIAA1122 protein, partial cds."//0//2170bp//99%//AB032948
C-NT2RM4001594
C-NT2RM4001597//M.musculus red-1 gene.//2.1E-171//1414bp//78%//X92750
C-NT2RM4001611//SIS2 PROTEIN (HALOTOLERANCE PROTEIN HAL3).//2.6E-32//203aa//39%//Q12600
C-NT2RM4001629//"MAGUK P55 SUBFAMILY MEMBER 3 (MPP3 PROTEIN) (DISCS, LARGE HOMOLOG 3)."//1.5E-93//278aa//38%//Q13368
C-NT2RM4001650
C-NT2RM4001662
C-NT2RM4001666//HYPOTHETICAL 48.6 KD PROTEIN IN ALPA-GABP INTERGENIC REGION.//2.7E-84//410aa//42%//P37339
C-NT2RM4001682
C-NT2RM4001710
C-NT2RM4001714//SEPTIN 2 HOMOLOG (FRAGMENT).//8.9E-141//354aa//72%//Q14141
C-NT2RM4001715
C-NT2RM4001731//"Homo sapiens mRNA for KIAA1004 protein, partial cds."//0//1922bp//100%//AB023221
C-NT2RM4001746
C-NT2RM4001754
C-NT2RM4001758//PUTATIVE SERINE/THREONINE-PROTEIN KINASE EMK (EC 2.7.1.-).//4.1E-186//639aa//58%//Q05512
C-NT2RM4001783//ZINC FINGER PROTEIN HRX (ALL-1).//7.9E-66//311aa//35%//Q03164
C-NT2RM4001810//"Homo sapiens mRNA for KIAA0863 protein, complete cds."//0//2377bp//99%//AB020670
C-NT2RM4001813//LECTIN BRA-2.//0.00000048//114aa//30%//P17346
C-NT2RM4001823//ZINC FINGER PROTEIN 43 (ZINC PROTEIN HTF6).///2.9E-55//325aa//37%//P28160
C-NT2RM4001828//ZINC FINGER PROTEIN 84 (ZINC FINGER PROTEIN HPF2).//5.9E-161//481aa//56%//P51523
C-NT2RM4001836
C-NT2RM4001841//"Homo sapiens mRNA for KIAA0920 protein, complete cds."//0//1861bp//98%//AB023137
C-NT2RM4001842
C-NT2RM4001856
C-NT2RM4001858//T-BOX CONTAINING PROTEIN TBX6L (FRAGMENT).//6.5E-22//126aa//46%//P79779
C-NT2RM4001865//Homo sapiens mRNA for atopy related autoantigen CALC.//4.3E-244//1248bp//94%//Y17711
C-NT2RM4001876//RAS SUPPRESSOR PROTEIN 1 (RSU-1) (RSP-1 PROTEIN) (RSP-1).//6.5E-23//184aa//36%//Q15404
C-NT2RM4001880//PUTATIVE DNA HELICASE II HOMOLOG (EC 3.6.1.-).//5.9E-09//268aa//26%//P47486
C-NT2RM4001922//"Homo-sapiens mRNA for KIAA0957 protein, complete cds."//0//2165bp//99%//AB023174
C-NT2RM4001930//"Homo sapiens dolichyl-P-Glc:Man9GlcNAc2-PP-dolichyl glucosyltransferase (ALG6) mRNA, complete cds."//0//1930bp//99%//AF102851
C-NT2RM4001940//"Homo sapiens timeless homolog mRNA, complete cds."//0//2087bp//99%//AF098162
C-NT2RM4001953
C-NT2RM4001965
C-NT2RM4001969//R.norvegicus mRNA for IP63 protein.//2.6E-261//1563bp//84%//X99330
C-NT2RM4001979//ZINC FINGER PROTEIN 84 (ZINC FINGER PROTEIN HPF2).//1.8E-112//457aa//47%//P51523
C-NT2RM4001984
C-NT2RM4001987//"NEURAL CELL ADHESION MOLECULE 1, LARGE ISOFORM PRECURSOR (N-CAM 180) [CONTAINS: N-CAM 140]."//3.2E-17//281aa//30%//P16170
C-NT2RM4002013//HYPOTHETICAL 89.4 KD TRP-ASP REPEATS CONTAINING PROTEIN IN PMT6-PCT1 INTERGENIC REGION.//6.9E-94//589aa//35%//P42935
C-NT2RM4002018
C-NT2RM4002034//"Homo sapiens hiwi mRNA, partial cds."//1.9E-53//1585bp//60%//AF104260
C-NT2RM4002044
C-NT2RM4002054
C-NT2RM4002063//"Oryctolagus cuniculus sarcosine oxidase (SOX) mRNA, complete cds."//0//1865bp//99%//U82267
C-NT2RM4002066//"Homo sapiens thyroid hormone receptor-associated protein complex component TRAP230 mRNA, complete cds."//1.50E-211//1123bp//71%//AF117755
C-NT2RM4002075//RING CANAL PROTEIN (KELCH PROTEIN).//2.8E-105//556aa//41%//Q04652
C-NT2RM4002128
C-NT2RM4002140
C-NT2RM4002145//SLIT PROTEIN PRECURSOR.//1.40E-09//127aa//33%//P24014
C-NT2RM4002161//"Homo sapiens laforin (EPM2A) mRNA, complete cds."//0//2671bp//99%//AF084535
C-NT2RM4002174//MRP PROTEIN.//9.1E-68//264aa//51%//P21590
C-NT2RM4002189//"GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE)."//6.2E-33//688aa//27%//P08640
C-NT2RM4002205//"ELONGATION FACTOR G, MITOCHONDRIAL PRECURSOR (MEF-G)."//3E-37//122aa//72%//Q07803
C-NT2RM4002213//"Homo sapiens protein phosphatase methylesterase-1 (PME-1) mRNA, complete cds."//0//2452bp//100%//AF157028
C-NT2RM4002226//GTPASE ACTIVATING PROTEIN ROTUND.//3.7E-19//147aa//41%//P40809
C-NT2RM4002251//"ALPHA-1,3-MANNOSYL-GLYCOPROTEIN BETA-1,2-N-ACETYLGLUCOSAMINYLTRANSFERASE (EC 2.4.1.101) (N-GLYCOSYL-OLIGOSACCHARIDE-GLYCOPROTEIN N-ACETYLGLUCOSAMINYLTRANSFERASE I) (GNT-I) (GLCNAC-TI)."//2.2E-36//320aa//38%//P27808
C-NT2RM4002256
C-NT2RM4002266
C-NT2RM4002281
C-NT2RM4002287
C-NT2RM4002294
C-NT2RM4002301
C-NT2RM4002323//ANTIGEN GOR (FRAGMENT).//0.000000001//154aa//33 %//P48778
C-NT2RM4002339
C-NT2RM4002344
C-NT2RM4002373//"Homo sapiens mRNA for KIAA0649 protein, complete cds."//0//2666bp//99%//AB014549
C-NT2RM4002374
C-NT2RM4002383
C-NT2RM4002409//ACETYL-COENZYME A SYNTHETASE (EC 6.2.1.1) (ACETATE--COA LIGASE) (ACYL- ACTIVATING ENZYME).//1.3E-29//275aa//30%//P27095
C-NT2RM4002438//"Xenopus laevis putative Zic3 binding protein mRNA, complete cds."//1.1E-49//611bp//70%//AF129131
C-NT2RM4002446
C-NT2RM4002452
C-NT2RM4002457
C-NT2RM4002460//"ENV POLYPROTEIN (COAT POLYPROTEIN) [CONTAINS: COAT PROTEINS GP70, GP20]."//0.0000016//226aa//24%//P51515
C-NT2RM4002493
C-NT2RM4002527//VEGETATIBLE INCOMPATIBILITY PROTEIN HET-E-1.//8.9E-15//366aa//27%//Q00808
C-NT2RM4002532//PROTEIN HOM1.//2E-16//276aa//28%//P55137
C-NT2RM4002558//"Homo sapiens fatty acid transport protein (FATP) mRNA, complete cds."//0//1797bp//99%//AF055899
C-NT2RM4002567
C-NT2RM4002593
C-NT2RM4002594//MSP1 PROTEIN HOMOLOG.//2.7E-68//236aa//58%//P54815
C-NT2RM4002623//ASPARTYL-TRNA SYNTHETASE (EC 6.1.1.12) (ASPARTATE--TRNA LIGASE) (ASPRS).//2.3E-101//488aa//45%//032038
C-NT2RP1000324
C-NT2RP1000363//"Homo sapiens mRNA for KIAA0638 protein, partial cds."//0//1345bp//99%//AB014538
C-NT2RP1000418
C-NT2RP1000513//"Human NifU-like protein (hNifU) mRNA, partial cds."//6.50E-171//516bp//99%//U47101
C-NT2RP1000721
C-NT2RP1000730
C-NT2RP1000767
C-NT2RP1000836
C-NT2RP1000902//HYPOTHETICAL 127.4 KD PROTEIN F07F6.4 IN CHROMOSOME III.//5.2E-20//306aa//33%//Q09531
C-NT2RP1000943
C-NT2RP1001033//"Homo sapiens delta-tubulin mRNA, complete cds."//2.10E-285//1290bp//100%//AF201333
C-NT2RP1001073//"Homo sapiens U6 snRNA-associated Sm-like protein LSm5 mRNA, complete cds."//8.1E-107//504bp//99%//AF182291
C-NT2RP1001199
C-NT2RP1001248
C-NT2RP1001253//"Homo sapiens oscillin (hLn) mRNA, complete cds."//0//2020bp//99%//AF029914
C-NT2RP1001286
C-NT2RP1001294//MICROTUBULE-ASSOCIATED PROTEIN YTM1.//1.80E-38//258aa//32%//Q12024
C-NT2RP1001302//MICROTUBULE-ASSOCIATED PROTEIN YTM1.//1.80E-38//258aa//32%//Q12024
C-NT2RP1001310//"Homo sapiens mitochondrial carrier homolog 1 isoform a mRNA, partial cds; nuclear gene for mitochondrial product."//0//1732bp//99%//AF176006
C-NT2RP1001361//"Homo sapiens NADH-ubiquinone oxidoreductase subunit B14.5B homolog mRNA, complete cds."//6.5E-116//541bp//100%//AF070652
C-NT2RP1001385//HYPOTHETICAL 48.8 KD PROTEIN IN SSU81-SCS2 INTERGENIC REGION.//2.7E-22//284aa//25%//P40074
C-NT2RP1001432
C-NT2RP2000040//"Homo sapiens mRNA for KIAA0747 protein, partial cds."//0//2648bp//99%//AB013290
C-NT2RP2000076//Homo sapiens partial mRNA for polyhomeotic 2 protein (PH2 gene).//7.9E-20//265bp//73%//AJ242730
C-NT2RP2000098
C-NT2RP2000108
C-NT2RP2000257//PUTATIVE MITOCHONDRIAL CARRIER YIL006W.//9.7E-41//278aa//36%//P40556
C-NT2RP2000258//ACTIVATOR 1 140 KD SUBUNIT (REPLICATION FACTOR C LARGE SUBUNIT) (A1 140 KD SUBUNIT) (RF-C 140 KD SUBUNIT) (ACTIVATOR 1 LARGE SUBUNIT) (DNA-BINDING PROTEIN PO-GA).//7.1E-12//213aa//23%//P35251
C-NT2RP2000289
C-NT2RP2000327
C-NT2RP2000337
C-NT2RP2000420//ZINC FINGER PROTEIN 165.//8.5E-33//155aa//52%//P49910
C-NT2RP2000459
C-NT2RP2000498
C-NT2RP2000758
C-NT2RP2001137
C-NT2RP2001149
C-NT2RP2001168//VERPROLIN.//1.5E-09//143aa//33%//P37370
C-NT2RP2001173//"Homo sapiens mRNA for KIAA0480 protein, complete cds."//0//1780bp//99%//AB007949
C-NT2RP2001174//GASTRULA ZINC FINGER PROTEIN XLCGF46.1 (FRAGMENT).//6E-10//88aa//38%//P18722
C-NT2RP2001196
C-NT2RP2001226
C-NT2RP2001268//"Homo sapiens mRNA for KIAA0810 protein, partial cds."//0//3301bp//98%//AB018353
C-NT2RP2001290//BETA-SOLUBLE NSF ATTACHMENT PROTEIN (SNAP-BETA) (SNAP-ALPHA HOMOLOG) (BRAIN PROTEIN 147) (FRAGMENT).//4.4E-91//179aa//99%//P28663
C-NT2RP2001295//ZINC/CADMIUM RESISTANCE PROTEIN.//8.3E-39//161aa//34%//P20107
C-NT2RP2001312
C-NT2RP2001327//"TUMOR NECROSIS FACTOR, ALPHA-INDUCED PROTEIN 1, ENDOTHELIAL (B12 PROTEIN)."//5.5E-116//311aa//71%//Q13829
C-NT2RP2001328
C-NT2RP2001366
C-NT2RP2001378//MUCIN 2 PRECURSOR (INTESTINAL MUCIN 2).//2E-11//403aa//25%//Q02817
C-NT2RP2001392//MITOCHONDRIAL LON PROTEASE HOMOLOG 1 PRECURSOR (EC 3.4.21.-).//8.4E-192//581aa//54%//P93647
C-NT2RP2001394//Homo sapiens mRNA for SCML2 protein.//0//2068bp//99%//Y18004
C-NT2RP2001420//"Mus musculus nuclear protein NIP45 mRNA, complete cds."//9E-112//742bp//82%//U76759
C-NT2RP2001450
C-NT2RP2001467
C-NT2RP2001506
C-NT2RP2001511//"Homo sapiens putative RNA-binding protein Q99 mRNA, complete cds."//3.2E-297//2206bp//75 %//AF093097
C-NT2RP2001520//Homo sapiens mRNA for mitochondrial carrier protein ARALAR1.//0//2502bp//99%//Y14494
C-NT2RP2001536//"Homo sapiens X-ray repair cross-complementing protein 3 (XRCC3) mRNA, complete cds."//0//2326bp//99%//AF035586
C-NT2RP2001560//VAV2 PROTEIN.//0.00000015//219aa//27%//Q60992
C-NT2RP2001576//HYPOTHETICAL 62.2 KD PROTEIN C4G8.12C IN CHROMOSOME I.//8.2E-29//294aa//31%//Q09837
C-NT2RP2001581
C-NT2RP2001597//"RYANODINE RECEPTOR, CARDIAC MUSCLE."//0.000000036//127aa//36%//P30957
C-NT2RP2001628
C-NT2RP2001663//ENOLASE (EC 4.2.1.11) (2-PHOSPHOGLYCERATE DEHYDRATASE) (2-PHOSPHO-D- GLYCERATE HYDRO-LYASE) (FRAGMENT).//1.1E-47//126aa//53%//P42897
C-NT2RP2001748//FARNESYL PYROPHOSPHATE SYNTHETASE (FPP SYNTHETASE) (FPS) (FARNESYL DIPHOSPHATE SYNTHETASE) (DIMETHYLALLYLTRANSFERASE (EC 2.5.1.1) / GERANYLTRANSTRANSFERASE (EC 2.5.1.10)) (KIAA0032).//5.40E-47//96aa//97%//P14324
C-NT2RP2001813
C-NT2RP2001883//"Homo sapiens CGI-01 protein mRNA, complete cds."//0//2306bp//99%//AF132936
C-NT2RP2001900//ACTIN-LIKE PROTEIN ARP5.//2.3E-38//395aa//30%//P53946
C-NT2RP2001947
C-NT2RP2001985//"Homo sapiens high-risk human papilloma viruses E6 oncoproteins targeted protein E6TP1 alpha mRNA, complete cds."//2.00E-38//435bp//67%//AF090989
C-NT2RP2001991//SODIUM- AND CHLORIDE-DEPENDENT TRANSPORTER NTT73.//6.5E-129//279aa//85%//Q08469
C-NT2RP2002025//NG-CAM RELATED CELL ADHESION MOLECULE PRECURSOR (NR-CAM) (BRAVO).//1.7E-47//247aa//52%//P35331
C-NT2RP2002058//"Homo sapiens WD repeat protein WDR3 (WDR3) mRNA, complete cds."//0//2510bp//99%//AF083217
C-NT2RP2002076//Homo sapiens clone 24804 mRNA sequence.7/1.5E-294//1334bp//99%//AF052183
C-NT2RP2002078//PECANEX PROTEIN.//1.8E-09//195aa//32%//P18490
C-NT2RP2002079//"HISTONE H1, GONADAL."//4.4E-11//214aa//34%//P02256
C-NT2RP2002099//Homo sapiens mRNA for E1B-55kDa-associated protein.//0//3389bp//99%//AJ007509
C-NT2RP2002185//"Homo sapiens ubiquilin mRNA, complete cds."//0//1789bp//99%//AF176069
C-NT2RP2002193//"Homo sapiens PIAS3 mRNA for protein inhibitor of activatied STAT3, complete cds."//0//2809bp//99%//AB021868
C-NT2RP2002231
C-NT2RP2002235
C-NT2RP2002252//"Mus musculus (clone pVZmSin3A9) mSin3A9 mRNA, complete cds."//0//3118bp//91%//L38621
C-NT2RP2002292
C-NT2RP2002408
C-NT2RP2002442//HESA PROTEIN.//2.8E-14//163aa//30%//P46037
C-NT2RP2002464//DNA CROSS-LINK REPAIR PROTEIN PS02/SNM1.//6.50E-07//171aa//27%//P30620
C-NT2RP2002498
C-NT2RP2002503//ZINC FINGER PROTEIN 45 (BRC1744).//4.6E-144//537aa//49%//Q02386
C-NT2RP2002520//"Homo sapiens transcription factor RFX-B (RFXB) mRNA, complete cds."//3.70E-34//668bp//61%//AF105427
C-NT2RP2002549
C-NT2RP2002609//2-HYDROXYMUCONIC SEMIALDEHYDE HYDROLASE (EC 3.1.1.-) (HMSH).//2.80E-08//109aa//37%//P19076
C-NT2RP2002706
C-NT2RP2002710//SH3-BINDING PROTEIN 3BP-1.//4.9E-85//489aa//43%//P55194
C-NT2RP2002800
C-NT2RP2002880//GLUCOSE REPRESSION MEDIATOR PROTEIN.//0.000039//206aa//23%//P14922
C-NT2RP2002891
C-NT2RP2002929//HYPOTHETICAL 46.2 KD TRP-ASP REPEATS CONTAINING PROTEIN D2013.2 IN CHROMOSOME II.//4.1E-87//395aa//40%//Q18964
C-NT2RP2002939//ZINC FINGER PROTEIN 136.//5.4E-70//282aa//42%//P52737
C-NT2RP2002993//DNA-DIRECTED RNA POLYMERASE I 135 KD POLYPEPTIDE (EC 2.7.7.6) (RNA POLYMERASE I SUBUNIT 2) (RPA135).//0//716aa//91%//P70700
C-NT2RP2003034
C-NT2RP2003099
C-NT2RP2003137//UBIQUITIN.//0.000026//70aa//30%//P13117
C-NT2RP2003157//"Homo sapiens CGI-74 protein mRNA, complete cds."//0//2037bp//99%//AF151832
C-NT2RP2003158//"Homo sapiens mRNA for proteasome subunit p58, complete cds."//0//2091bp//99%//D67025
C-NT2RP2003165
C-NT2RP2003243//Homo sapiens partial mRNA for putative p621 protein which interacts with transcription factor Sp1.//0//1544bp//99%//AJ242978
C-NT2RP2003277//"Homo sapiens mRNA for KIAA0625 protein, partial cds."//0//3788bp//99%//AB014525
C-NT2RP2003286//PROBABLE RNA 3'-TERMINAL PHOSPHATE CYCLASE (EC 6.5.1.4) (RNA-3'- PHOSPHATE CYCLASE) (RNA CYCLASE).//4.1E-88//374aa//47%//Q23400
C-NT2RP2003297
C-NT2RP2003307//KINESIN LIGHT CHAIN (KLC).//2.2E-199//550aa//70%//Q07866
C-NT2RP2003308//CROOKED NECK PROTEIN.//5.4E-244//622aa//67%//P17886
C-NT2RP2003347//BREAST CANCER TYPE 1 SUSCEPTIBILITY PROTEIN HOMOLOG.//0.000022//261aa//24%//P48754
C-NT2RP2003391//Homo sapiens mRNA for nuclear transport receptor.//0//1509bp//99%//AJ133769
C-NT2RP2003393
C-NT2RP2003445
C-NT2RP2003466//"Homo sapiens delta-6 fatty acid desaturase mRNA, complete cds."7/0//2194bp//99%//AF126799
C-NT2RP2003480//"Homo sapiens zinc finger DNA binding protein 99 (ZNF281) mRNA, complete cds."//0//3012bp//99%//AF125158
C-NT2RP2003506//NADPH-CYTOCHROME P450 REDUCTASE (EC 1.6.2.4) (CPR).//5.4E-14//106aa//46%//P04175
C-NT2RP2003511
C-NT2RP2003513//"Human mRNA for KIAA0270 gene, partial cds."//0//2137bp//97%//D87460
C-NT2RP2003567//"Homo sapiens mRNA for KIAA0462 protein, partial cds."//0//2343bp//99%//AB007931
C-NT2RP2003604//"Homo sapiens alpha-catenin-like protein (CTNNAL1) mRNA, complete cds."//0//2442bp//99%//AF030233
C-NT2RP2003691
C-NT2RP2003713//"Homo sapiens ubiquitin-specific protease 3 (USP3) mRNA, complete cds."//0//2018bp//99%//AF073344
C-NT2RP2003760//COATOMER GAMMA SUBUNIT (GAMMA-COAT PROTEIN) (GAMMA-COP).//0//869aa//80%//P53620
C-NT2RP2003764
C-NT2RP2003769
C-NT2RP2003777
C-NT2RP2003840//HYPOTHETICAL 48.1 KD PROTEIN B0403.2 IN CHROMOSOME X.//3.7E-21//137aa//43%//Q11076
C-NT2RP2003857//MYOTROPHIN (V-1 PROTEIN) (GRANULE CELL DIFFERENTIATION PROTEIN).//0.00000016//117aa//29%//Q91955
C-NT2RP2003981//"Homo sapiens mRNA for KIAA0804 protein, partial cds."//0//3046bp//99%//AB018347
C-NT2RP2003984//Homo sapiens mRNA; cDNA DKFZp564A026 (from clone DKFZp564A026).//0//2514bp//99%//AL050367
C-NT2RP2004041//SYNAPSINS IA AND IB.//0.00000074//159aa//32%//P17599
C-NT2RP2004066//"Human DNA sequence from clone 134019 on chromosome 1p36.11-36.33, complete sequence."//0//2410bp//99%//AL034555
C-NT2RP2004081
C-NT2RP2004124
C-NT2RP2004152
C-NT2RP2004165
C-NT2RP2004187//ZINC FINGER PROTEIN 38 (ZFP-38) (CTFIN51) (TRANSCRIPTION FACTOR RU49).//5.6E-31//424aa//28%//007231
C-NT2RP2004239//"Homo sapiens lok mRNA for protein kinase, complete cds."//0//3044bp//99%//AB015718
C-NT2RP2004245
C-NT2RP2004364
C-NT2RP2004365
C-NT2RP2004366//"Homo sapiens mRNA for KIAA0986 protein, partial cds."//0//2790bp//97%//AB023203
C-NT2RP2004373
C-NT2RP2004476//"Homo sapiens cyclin L ania-6a mRNA, complete cds."//0//2075bp//99%//AF180920
C-NT2RP2004551
C-NT2RP2004568//PUTATIVE ATP-DEPENDENT RNA HELICASE C30D11.03.//3E-117//625aa//40%//Q09903
C-NT2RP2004600
C-NT2RP2004664//"Homo sapiens mRNA for KIAA0460 protein, partial cds."//0//2368bp//99%//AB007929
C-NT2RP2004743
C-NT2RP2004768//SERINE/THREONINE-PROTEIN KINASE NRK1 (EC 2.7.1.-) (N-RICH KINASE 1).//1.3E-26//190aa//41-%//P38692
C-NT2RP2004816//"Homo sapiens H beta 58 homolog mRNA, complete cds."//0//2144bp//96%//AF054179
C-NT2RP2004861
C-NT2RP2004897
C-NT2RP2004933//"Homo sapiens mRNA for ZIP-kinase, complete cds."//0//2103bp//99%//AB007144
C-NT2RP2004978//ACTIN-LIKE PROTEIN ARP8.//3.3E-47//353aa//30%//Q12386
C-NT2RP2005038//DNA NUCLEOTIDYLEXOTRANSFERASE (EC 2.7.7.31) (TERMINAL ADDITION ENZYME) (TERMINAL DEOXYNUCLEOTIDYLTRANSFERASE) (TERMINAL TRANSFERASE).//4E-91//218aa//44%//Q92089
C-NT2RP2005162//"Homo sapiens aspartyl aminopeptidase mRNA, complete cds."//0//1615bp//99%//AF005050
C-NT2RP2005204//"Homo sapiens SUMO-1-activating enzyme E1 N subunit (SUA1) mRNA, complete cds."//0//1262bp//99%//AF090385
C-NT2RP2005227
C-NT2RP2005287
C-NT2RP2005288//"Homo sapiens RCC1-like G exchanging factor RLG mRNA, complete cds."//0//2992bp//99%//AF060219
C-NT2RP2005490//"Mus musculus D3Mm3e (D3Mm3e) mRNA, complete cds."//1.8E-175//1102bp//83%//AF053628
C-NT2RP2005539//"Homo sapiens mRNA for KIAA0850 protein, complete cds."//0//1560bp//99%//AB020657
C-NT2RP2005605//QUEUINE TRNA-RIBOSYLTRANSFERASE (EC 2.4.2.29) (TRNA-GUANINE TRANSGLYCOSYLASE) (GUANINE INSERTION ENZYME).//8.2E-23//164aa//28%//032053
C-NT2RP2005722//"Homo sapiens ZK1 mRNA for Kruppel-type zinc finger protein, complete cds."//0//2545bp//99%//AB011414
C-NT2RP2005732
C-NT2RP2005784//"Homo sapiens ubiquitin-conjugating enzyme variant Kua (UBE2V) mRNA, complete cds."//0//2191bp//92%//AF155120
C-NT2RP2005812//HYPOTHETICAL 39.3 KD PROTEIN IN GCN4-WBP1 INTERGENIC REGION.//2.3E-39//318aa//31%//P40004
C-NT2RP2005859//"Homo sapiens mRNA for KIAA0863 protein, complete cds."//0//1649bp//99%//AB020670
C-NT2RP2006023
C-NT2RP2006334//Homo sapiens mRNA; cDNA DKFZp434J154 (from clone DKFZp434J154).//0//2318bp//99%//AL080155
C-NT2RP2006441
C-NT2RP3000002
C-NT2RP3000050//ZINC FINGER PROTEIN 91 (ZINC FINGER PROTEIN HTF10) (HPF7).//2.2E-150//490aa//53%//Q05481
C-NT2RP3000055
C-NT2RP3000068
C-NT2RP3000080
C-NT2RP3000085//ACETYL-/PROPIONYL-COENZYME A CARBOXYLASE ALPHA CHAIN [CONTAINS: BIOTIN CARBOXYLASE (EC 6.3.4.14); BIOTIN CARBOXYL CARRIER PROTEIN (BCCP)].//1.9E-123//436aa//50%//P46401
C-NT2RP3000092
C-NT2RP3000109//P54 PROTEIN PRECURSOR.//0.0000065//358aa//22%//P13692
C-NT2RP3000134
C-NT2RP3000149
C-NT2RP3000197
C-NT2RP3000207//"GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE)."//2.9E-11//721aa//23%//P08640
C-NT2RP3000233//"Human DNA sequence from clone 22D12 on chromosome Xq21.1-21.33. Contains a novel protein similar to Drosophila Kelch (Ring Canal protein, KEL) and a heterogenous set of other types of proteins. Contains ESTs and GSSs, complete sequence."//0//1462bp//99%//AL035424
C-NT2RP3000235
C-NT2RP3000247
C-NT2RP3000267
C-NT2RP3000299//"Rattus norvegicus mRNA for Crk-associated substrate, p130, complete cds."//0//2730bp//82%//D29766
C-NT2RP3000324
C-NT2RP3000341//"Homo sapiens mitochondrial inner membrane preprotein translocase Timl7a mRNA, nuclear gene encoding mitochondrial protein, complete cds."//1.5E-246//1124bp//99%//AF106622
C-NT2RP3000393//"Rattus norvegicus DNA-binding protein PREB (Preb) mRNA, complete cds."//5.8E-266//1373bp//86%//AF061817
C-NT2RP3000441//"Homo sapiens squamous cell carcinoma antigen recognized by T cell (SART-2) mRNA, complete cds."//3.40E-42//645bp//67%//AF098066
C-NT2RP3000449
C-NT2RP3000451
C-NT2RP3000456
C-NT2RP3000542
C-NT2RP3000561
C-NT2RP3000562//"Homo sapiens putative RNA-binding protein Q99 mRNA, complete cds."//0//2165bp//99%//AF093097
C-NT2RP3000578//HES1 PROTEIN.//1-3E-22//229aa//27%//P35843
C-NT2RP3000590//UVS-2 PROTEIN.//1.3E-22//458aa//24%//P33288
C-NT2RP3000592
C-NT2RP3000622
C-NT2RP3000624
C-NT2RP3000685
C-NT2RP3000736//HYPOTHETICAL PROTEIN KIAA0140.//1.2E-166//305aa//99%//014153
C-NT2RP3000742//"1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III) (FRAGMENT)."//4.1E-165//371aa//49%//P10895
C-NT2RP3000753
C-NT2RP3000826
C-NT2RP3000865
C-NT2RP3000875//MEVALONATE KINASE (EC 2.7.1.36) (MK).//7.7E-87//175aa//98%//Q03426
C-NT2RP3001007
C-NT2RP3001055
C-NT2RP300111//"Homo sapiens TRF-proximal protein mRNA, complete cds."//1.50E-149//731bp//97%//AF097725
C-NT2RP3001120//ZINC FINGER PROTEIN 136.//7.8E-170//512aa//58%//P52737
C-NT2RP3001126
C-NT2RP3001150//TRANSCRIPTION TERMINATION FACTOR RHO.//0.00000031//207aa//29%//P52154
C-NT2RP3001232
C-NT2RP3001268//"Homo sapiens zinc finger protein ZNF228 (ZNF228) mRNA, complete cds."//0//3606bp//99%//AF198358
C-NT2RP3001272//Mus musculus mRNA for macrophage actin-associated-tyrosine-phosphorylated protein.//1.3E-99//669bp//83%//Y18101
C-NT2RP3001274//"Homo sapiens mRNA for KIAA1037 protein, partial cds."//0//2254bp//99%//AB028960
C-NT2RP3001281
C-NT2RP3001297
C-NT2RP3001318
C-NT2RP3001338//ZINC FINGER PROTEIN 81 (FRAGMENT).//2.4E-16//175aa//28%//P51508
C-NT2RP3001355//TRICARBOXYLATE TRANSPORT PROTEIN PRECURSOR (CITRATE TRANSPORT PROTEIN) (CTP) (TRICARBOXYLATE CARRIER PROTEIN).//3.6E-25//129aa//34%//P32089
C-NT2RP3001374
C-NT2RP3001428//NUCLEOPROTEIN TPR.//1.4E-128//152aa//99%//P12270
C-NT2RP3001432
C-NT2RP3001447
C-NT2RP3001449//"Human DNA sequence from clone 283E3 on chromosome 1p36.21-36.33. Contains the alternatively spliced gene for Matrix Metalloproteinase in the Female Reproductive tract MIFR1, -2, MMP21/22A, -B and -C, a novel gene, the alternatively spliced CDC2L2 gene for Cell Division Cycle 2-Like 2 (PITSLRE, p58/GTA, Galactosyltransferase Associated Protein Kinase) beta 1, beta 2-1, beta 2-2 and alpha 2-4, a 40S Ribosomal Protein S7 pseudogene, part of the KIAA0447 gene, a novel alternatively spliced gene similar to many (archae)bacterial, worm and yeast hypothetical genes, and the GNB1 gene for Guanine Nucleotide Binding Protein (G protein), Beta polypeptide 1 (Transducin Beta chain 1). Contains putative CpG islands, ESTs, STSs and GSSs, complete sequence."//0//1827bp//99%//AL031282
C-NT2RP3001453//ANTIGEN PEPTIDE TRANSPORTER 2 (APT2) (HISTOCOMPATIBILITY ANTIGEN MODIFIER 2).//3.2E-90//157aa//59%//P36371
C-NT2RP3001459
C-NT2RP3001527//"Human Spl40 protein (Spl40) mRNA, complete cds."//4.3E-290//793bp//93%//U63420
C-NT2RP3001538//HYPOTHETICAL 39.0 KD PROTEIN T2.8D9.3 IN CHROMOSOME II.//9.10E-10//158aa//31%//Q10022
C-NT2RP3001580//"Mus musculus strain C57BL/J germ cell-less protein (Gcl) mRNA, complete cds."//0//1730bp//85%//AF163665
C-NT2RP3001587//"Human anthracycline-associated resistance ARX mRNA, complete cds."//0//2617bp//99%//U35832
C-NT2RP3001589
C-NT2RP3001607
C-NT2RP3001608
C-NT2RP3001671//"Homo sapiens mRNA for KIAA0850 protein, complete cds."//0//2310bp//99%//AB020657
C-NT2RP3001672//"Homo sapiens Sex comb on midleg homolog 1 isoform 2 (SCMH1) mRNA, complete cds."//0//2836bp//99%//AF149046
C-NT2RP3001678
C-NT2RP3001688//"Homo sapiens glucocorticoid modulatory element binding protein-1 (GMEB1) mRNA, complete cds."//0//1695bp//99%//AF099013
C-NT2RP3001690//INTRACELLULAR PROTEIN TRANSPORT PROTEIN USO1.//0.00000024//481aa//21%//P25386
C-NT2RP3001698
C-NT2RP3001708//TWISTED GASTRULATION PROTEIN PRECURSOR.//3.4E-33//161aa//32%//P54356
C-NT2RP3001716
C-NT2RP3001752
C-NT2RP3001792//HETEROGENEOUS NUCLEAR RIBONUCLEOPROTEIN M (HNRNP M).//1.8E-117//462aa//55%//P52272
C-NT2RP3001844
C-NT2RP3001854//Homo sapiens mRNA; cDNA DKFZp564G013 (from clone DKFZp564G013).//0//1528bp//99%//AL050011
C-NT2RP3001855//HOMEOBOX PROTEIN PKNOX1 (HOMEOBOX PROTEIN PREP-1).//8.1E-125//302aa//60%//P55347
C-NT2RP3001898//"Homo sapiens mRNA for UDP-N-acetylglucosamine: alpha-1,3-D-mannoside beta-1,4-N-acetylglucosaminyltransferase IV, complete cds."//0//1587bp//100%//AB000624
C-NT2RP3001931
C-NT2RP3001969//TRICHOHYALIN.//2.7E-11//442aa//23%//P37709
C-NT2RP3002002
C-NT2RP3002004//H.sapiens mRNA for FAST kinase.//1.50E-19211475bp//94%//X86779
C-NT2RP3002007//SAP1 PROTEIN.//1.1E-68//474aa//32%//P39955
C-NT2RP3002014//HYPOTHETICAL 32.0 KD PROTEIN C09F5.2 IN CHROMOSOME III.//5.30E-25//139aa//48%//Q09232
C-NT2RP3002045//"Homo sapiens mRNA for KIAA0899 protein, partial cds."//0//33 85bp//99%//AB020706
C-NT2RP3002056//"Homo sapiens Rb binding protein homolog mRNA, partial cds."//0//2374bp//99%//AF083249
C-NT2RP3002062//"Homo sapiens mRNA for KIAA0873 protein, partial cds."//0//3764bp//99%//AB020680
C-NT2RP3002081//"Xenopus laevis chromosome condensation protein XCAP-G mRNA, complete cds."//4.1E-233//1896bp//69%//AF111423
C-NT2RP3002097
C-NT2RP3002108//DEC1 PROTEIN (MDM20 PROTEIN).//7.90E-09//181aa//22%//Q12387
C-NT2RP3002142
C-NT2RP3002146
C-NT2RP3002151//G1 TO S PHASE TRANSITION PROTEIN 1 HOMOLOG (GTP-BINDING PROTEIN GST1-HS).//2.8E-253//474aa//93%//P15170
C-NT2RP3002165//TRANSCRIPTIONAL REGULATOR PROTEIN HCNGP7/1.9E-151//223aa//91%//Q02614
C-NT2RP3002166
C-NT2RP3002181
C-NT2RP3002244
C-NT2RP3002248
C-NT2RP3002273//SCD6 PROTEIN.//1.30E-09//295aa//28%//P45978
C-NT2RP3002276
C-NT2RP3002304
C-NT2RP3002501//THREONINE DEHYDRATASE CATABOLIC (EC 4.2.1.16) (THREONINE DEAMINASE).//3.70E-43//318aa//37%//P05792
C-NT2RP3002529//Homo sapiens mRNA for leucocyte vacuolar protein sorting.//0//2276bp//99%//AJ133421
C-NT2RP3002566
C-NT2RP3002587
C-NT2RP3002590
C-NT2RP3002631
C-NT2RP3002650//"Mus musculus growth suppressor 1L (Gros1) mRNA, complete cds."//0//2109bp//87%//AF165163
C-NT2RP3002663//"Homo sapiens putative glycolipid transfer protein mRNA, complete cds."//8.10E-263//1243bp//97%//AF103731
C-NT2RP3002671//ELONGATION FACTOR 2 (EF-2).//2.50E-73//179aa//36%//P13060
C-NT2RP3002763
C-NT2RP3002861
C-NT2RP3002911
C-NT2RP3002948//RING CANAL PROTEIN (KELCH PROTEIN).//2E-111//551aa//42%//Q04652
C-NT2RP3002953//"Homo sapiens protocadherin beta 5 (PCDH-beta5) mRNA, complete cds."//0//2388bp//99%//AF152498
C-NT2RP3002988//"Homo sapiens IkB kinase-b (IKK-beta) mRNA, complete cds."//1.8E-292//1325bp//99%//AF080158
C-NT2RP3003008
C-NT2RP3003101//"Mouse mRNA for tetracycline transporter-like protein, complete cds."//3.6E-83//807bp//72%//D88315
C-NT2RP3003204
C-NT2RP3003278
C-NT2RP3003282//"Homo sapiens dynamin (DNM) mRNA, complete cds."//0//2596bp//98%//L36983
C-NT2RP3003290//"Mus musculus mRNA for Ndr1 related protein Ndr3, complete cds."//1.5e-310//1468bp//82%//AB033922
C-NT2RP3003302
C-NT2RP3003313//"Homo sapiens thyroid hormone receptor-associated protein complex component TRAP80 mRNA, complete cds."//0//2476bp//99%//AF117657
C-NT2RP3003327//52 KD RO PROTEIN (SJOGREN SYNDROME TYPE A ANTIGEN (SS-A)) (RO(SS-A)) (R052).//1.3E-35//178aa//44%//Q62191
C-NT2RP3003344
C-NT2RP3003353//HYPOTHETICAL 26.2 KD PROTEIN IN GDI1-COX15 INTERGENIC REGION.//2.80E-07//161aa//28%//P40084
C-NT2RP3003377
C-NT2RP3003385//"Mus musculus SKD3 mRNA, complete cds."//0//2133bp//85%//U09874
C-NT2RP3003433
C-NT2RP3003490//"Homo sapiens mRNA for KIAA0725 protein, partial cds."//0//2437bp//99%//AB018268
C-NT2RP3003491//"Drosophila melanogaster Pelle associated protein Pellino (Pli) mRNA, complete cds."//5.6E-36//842bp//62%//AF091624
C-NT2RP3004206//CROOKED NECK PROTEIN.//1.4E-220//567aa//67%//P17886
C-NT2RP3004207//Homo sapiens mRNA for type I transmembrane receptor (psk-1 gene).//0//2445bp//100%//AJ245820
C-NT2RP3004209//"Homo sapiens ubiquitin processing protease (Ubp-M) mRNA, complete cds."//0//2320bp//99%//AF126736
C-NT2RP3004242//PUTATIVE RHO/RAC GUANINE NUCLEOTIDE EXCHANGE FACTOR (RHO/RAC GEF) (FACIOGENITAL DYSPLASIA PROTEIN HOMOLOG).//4.7E-13//118aa//33%//P52734
C-NT2RP3004246
C-NT2RP3004258//"Homo sapiens ZIS1 mRNA, complete cds."//0//1861bp//99%//AF065391
C-NT2RP3004262//"Homo sapiens heat shock protein hsp40-3 mRNA, complete cds."//2.4E-248//1126bp//100%//AF088982
C-NT2RP3004341
C-NT2RP3004378
C-NT2RP3004424//Homo sapiens mRNA for stromal antigen 3 (STAG3 gene).//1E-66//364bp//93%//AJ007798
C-NT2RP3004428
C-NT2RP3004451
C-NT2RP3004454//"Homo sapiens mRNA for KIAA0448 protein, complete cds."//0//2875bp//99%//AB007917
C-NT2RP3004472//GERM CELL-LESS PROTEIN.//1.6E-61//170aa//40%//Q01820
C-NT2RP3004498//"Mus musculus ROSA 26 transcription AS ROSA26AS mRNA, complete cds."//2E-249//1777bp//80%//U83176
C-NT2RP3004504//M.musculus mRNA for CPEB protein.//1.9E-295//893bp//92%//Y08260
C-NT2RP3004507//MOB1 PROTEIN (MPS1 BINDER 1).//3.7E-37//190aa//39%//P40484
C-NT2RP3004534//"Mouse oncogene (ect2) mRNA, complete cds."//0//2075bp//87%//L11316
C-NT2RP4000528//NPL4 PROTEIN.//9.8E-86//515aa//37%//P33755
C-NT2RP4000907//"Mouse NLRR-1 mRNA for leucine-rich-repeat protein, complete cds."//0//2127bp//86%//D45913
C-NT2RP4001029//"Mus domesticus nuclear binding factor NF2d9 mRNA, complete cds."//0//1711bp//90%//U20086
C-NT2RP4001336//GUANINE NUCLEOTIDE-BINDING PROTEIN BETA SUBUNIT-LIKE PROTEIN.//0.000016//186aa//29%//024076
C-NT2RP4001389//KES1 PROTEIN.//1.70E-31//342aa//34%//P35844
C-NT2RP4001442
C-NT2RP4001529//"Mus domesticus nuclear binding factor NF2d9 mRNA, complete cds."/1.70E-255//1148bp//90%//U20086
C-NT2RP4001656//VACUOLAR BIOGENESIS PROTEIN END1 (PEP5 PROTEIN).//1.10E-45//310aa//27%//P12868
C-OVARC1000106//"TROPOMYOSIN 1, FUSION PROTEIN 33."//0.000032//165aa//27%//P49455
C-OVARC1000198
C-OVARC1000682//"PROCESSING ALPHA-1,2-MANNOSIDASE (EC 3.2.1.-) (ALPHA-1,2-MANNOSIDASE 1B)."//1.1E-209//293aa//95%//P39098
C-OVARC1000703
C-OVARC1000722//"Homo sapiens chromosome 1q21-1q23 beta-1,4-galactosyltransferase mRNA, complete cds."//0//759bp//98%//AF038661
C-OVARC1000730
C-OVARC1000746//MATERNAL EFFECT PROTEIN STAUFEN.//0.000000017//78aa//48%//P25159
C-OVARC1000781
C-OVARC1000787
C-OVARC10008347/Homo sapiens mRNA for atopy related autoantigen CALCJ/2.8E-258//1183bp//99%//Y17711
C-OVARC1000846//NUCLEOLIN (PROTEIN C23).//0.0000097//109aa//30%//P08199
C-OVARC1000850//"Homo sapiens PB39 mRNA, complete cds."//0//2095bp//99%//AF045584
C-OVARC1000862//M.musculus mRNA for FT1.//5.9E-226//1498bp//81%//Z67963
C-OVARC1000876//MOB1 PROTEIN (MPS1 BINDER 1).//2.2E-50//206aa//52%//P40484
C-OVA-RC1000883
C-OVARC1000886
C-OVARC1000912
C-OVARC1000915//"Homo sapiens histone deacetylase 5 mRNA, complete cds."//1.60E-121//591bp//97%//AF132608
C-OVARC1000924
C-OVARC1000964
C-OVARC1000984
C-OVARC1001004
C-OVARC1001010
C-OVARC1001011
C-OVARC1001032
C-OVARC1001044
C-OVARC1001055//PRE-B CELL ENHANCING FACTOR PRECURSOR.//1.9E-35//76aa//98%//P43490
C-OVARC1001068//"Homo sapiens Era GTPase A protein (HERA-A) mRNA, partial cds."//0//1819bp//99%//AF082657
C-OVARC1001074
C-OVARC1001092//"Homo sapiens mRNA for JM5 protein, complete CDS (clone IMAGE 53337, LLNLc110F185707 (RZPD Berlin) and LLNLc110G0913Q7 (RZPD Berlin))."//2E-214//769bp//97%//AJ005897
C-OVARC1001107//"Homo sapiens protein methyltransferase (JBP1) mRNA, complete cds."//6.1E-276//594bp//98%//AF167572
C-OVARC1001154//"Homo sapiens clone 24720 epithelin 1 and 2 mRNA, complete cds."//2.3E-296//1561bp//93%//AF055008
C-OVARC1001161
C-OVARC1001167
C-OVARC1001170
C-OVARC1001171//"Homo sapiens translation initiation factor 3 47 kDa subunit mRNA, complete cds."//5.7E-151//436bp//92%//U94855
C-OVARC1001173
C-OVARC1001176
C-OVARC1001180//UBIQUITIN-LIKE PROTEIN DSK2.//1.1E-11//221aa//25%//P48510
C-OVARC1001188
C-OVARC1001232//"CLEAVAGE AND POLYADENYLATION SPECIFICITY FACTOR, 100 KD SUBUNIT (CPSF 100 KD SUBUNIT)."//5.10E-22//83aa//37%//Q10568
C-OVARC1001270
C-OVARC1001271//NUCLEOLAR TRANSCRIPTION FACTOR 1 (UPSTREAM BINDING FACTOR 1) (UBF-1).//0.0000014//224aa//26%//P25976
C-OVARC1001306//N-MYC PROTO-ONCOGENE PROTEIN.//0.00000073//247aa//27%//P18444
C-OVARC1001344
C-OVARC1001369
C-OVARC1001372//"Homo sapiens mRNA for KIAA0897 protein, partial cds."//0//840bp//97%//AB020704
C-OVARC1001391
C-OVARC1001399
C-OVARC1001417//"Homo sapiens thyroid hormone receptor-associated protein complex component TRAP170 mRNA, complete cds."//0//1715bp//99%//AF135802
C-OVARC1001419//"Homo sapiens GOK (STIM1) mRNA, complete cds."//4.9E-48//586bp//69%//U52426
C-OVARC1001436//ENL PROTEIN.//0.00000009//81aa//39%//Q03111
C-OVARC1001453
C-OVARC1001476//"Mus musculus YGR163w mRNA homologue, complete cds."//1.80E-187//510bp//89%//AB017616
C-OVARC1001480
C-OVARC1001489
C-OVARC1001506//POLYCYSTIN PRECURSOR (AUTOSOMAL DOMINANT POLYCYSTIC KIDNEY DISEASE PROTEIN 1).//0//777aa//91%//P98161
C-OVARC1001525
C-OVARC1001555//NGG1-INTERACTING FACTOR 3.//4.4E-19//130aa//40%//P53081
C-OVARC1001577//Homo sapiens SRp46 splicing factor transcribed retropseudogene.//0//1167bp//100%//AF031165
C-OVARC1001600
C-OVARC1001610//"Homo sapiens choline/ethanolaminephosphotransferase (CEPT1) mRNA, complete cds."//0//1870bp//99%//AF068302
C-OVARC1001702
C-OVARC1001703//"Mus musculus ARL-6 interacting protein-2 (Aip-2) mRNA, complete cds."//3.5E-16//399bp//61%//AF133670
C-OVARC1001711//CORNIFIN B (SMALL PROLINE-RICH PROTEIN 1B) (SPR1B) (SPR1 B).//2.80E-10//106aa//38%//Q62267
C-OVARC1001713//ENDOZEPINE-RELATED PROTEIN PRECURSOR (MEMBRANE-ASSOCIATED DIAZEPAM BINDING INHIBITOR) (MA-DBI).//4.4E-40//195aa//41%//P07106
C-OVARC1001726//APICAL-LIKE PROTEIN (APXL PROTEIN).//4.3E-16//116aa//43%//Q13796
C-OVARC1001731//"TROPOMYOSIN ALPHA CHAIN, FIBROBLAST ISOFORM F2."//4E-122//282aa//85%//P08942
C-OVARC1001745
C-OVARC1001762//"N-TERMINAL ACETYLTRANSFERASE 1 (EC 2.3.1.88) (AMINO-TERMINAL, ALPHA- AMINO, ACETYLTRANSFERASE 1)."//6.4E-85//514aa//34%//P12945
C-OVARC1001766//"Homo sapiens eukaryotic translation initiation factor eIF3, p35 subunit mRNA, complete cds."//0//963bp//99%//U97670
C-OVARC1001767//"Homo sapiens mRNA for KIAA0675 protein, complete cds."//0//2083bp//99%//AB014575
C-OVARC1001768
C-OVARC1001791
C-OVARC1001795
C-OVARC1001802
C-OVARC1001809//"Mus musculus sphingosine kinase (SPHK1a) mRNA, partial cds."//2.7E-190//1624bp//76%//AF068748
C-OVARC1001828
C-OVARC1001846
C-OVARC1001861
C-OVARC1001879
C-OVARC1001880
C-OVARC1001883
C-OVARC1001916
C-OVARC1001928
C-OVARC1001942//"N-TERMINAL ACETYLTRANSFERASE 1 (EC 2.3.1.88) (AMINO-TERMINAL, ALPHA- AMINO, ACETYLTRANSFERASE 1)."//3.1E-81//497aa//35%//P12945
C-OVARC1001943//"Mus musculus DEBT-91 mRNA, complete cds."//0//2035bp//87%//AF143859
C-OVARC1001950
C-OVARC1001987//"Rattus norvegicus DNA-binding protein PREB (Preb) mRNA, complete cds."//2.3E-220//652bp//84%//AF061817
C-OVARC1002050//"Homo sapiens mRNA for actin binding protein ABP620, complete cds."//0//1019bp//99%//AB029290
C-OVARC1002082
C-OVARC1002107
C-OVARC1002127//SODIUM-INDEPENDENT ORGANIC ANION TRANSPORTER 2 (BRAIN DIGOXIN CARRIER PROTEIN) (BRAIN-SPECIFIC ORGANIC ANION TRANSPORTER) (OATP-B1).//5.4E-52//306aa//35%//035913
C-OVARC1002138//SAP1 PROTEIN.//7.6E-60//128aa//59%//P39955
C-OVARC1002156
C-OVARC1002158
C-PLACE1000004//"Homo sapiens IDN3-B mRNA, complete cds."//0//2365bp//99%//AB019602
C-PLACE1000040//TRANSFORMING PROTEIN P21/K-RAS 2B.//1.4E-17//185aa//32%//P08643
C-PLACE1000048
C-PLACE1000050
C-PLACE1000061//Human ribosomal protein L37a mRNA sequence.//7.9E-54//190bp//94%//L22154
C-PLACE1000081//"Human SEC7 homolog Tic (TIC) mRNA, complete cds."//0//2077bp//99%//U63127
C-PLACE1000094
C-PLACE1000133//TRANSCRIPTION FACTOR BTF3 (RNA POLYMERASE B TRANSCRIPTION FACTOR 3).//1.8E-62//158aa//81%//P20290
C-PLACE1000214
C-PLACE1000236
C-PLACE1000246
C-PLACE1000292
C-PLACE1000308
C-PLACE1000332
C-PLACE1000453
C-PLACE1000583//ZINC FINGER PROTEIN 83 (ZINC FINGER PROTEIN HPF1).//1.60E-47//207aa//46%//P51522
C-PLACE1000599
C-PLACE1000610//MSN5 PROTEIN.//0.0000026//136aa//26%//P52918
C-PLACE1000653//"Homo sapiens N-acetylglucosamine-phosphate mutase mRNA, complete cds."//0//1992bp//99%//AF180371
C-PLACE1000656//"Homo sapiens mRNA for JM4 protein, complete CDS (clone IMAGE 546750 and LLNLc110F1857Q7 (RZPD Berlin))."//2.1E-277//1260bp//99%//AJ005896
C-PLACE1000706//"Homo sapiens transcriptional intermediary factor 1 gamma mRNA, complete cds."//0//1366bp//99%//AF119043
C-PLACE1000712
C-PLACE1000749
C-PLACE1000769//"Homo sapiens CGI-18 protein mRNA, complete cds."//0//1985bp//98%//AF132952
C-PLACE1000786//PUTATIVE RHO/RAC GUANINE NUCLEOTIDE EXCHANGE FACTOR (RHO/RAC GEF) (FACIOGENITAL DYSPLASIA PROTEIN HOMOLOG).//7.10E-09//59aa//47%//P52734
C-PLACE1000849
C-PLACE1000856//"Homo sapiens mRNA for KIAA0974 protein, partial cds."//0//1310bp//100%//AB023191
C-PLACE1000931
C-PLACE1000987//"Homo sapiens mRNA for KIAA0724 protein, complete cds."//0//1749bp//99%//AB018267
C-PLACE1001010
C-PLACE1001015
C-PLACE1001024
C-PLACE1001062//"Homo sapiens PAC clone DJ1049N15 from 7q31.2-7q32, complete sequence."//2.7E-32//470bp//71%//AC006020
C-PLACE1001104
C-PLACE1001168
C-PLACE1001171//MYOTUBULARIN.//7.1E-84//198aa//73%//Q13496
C-PLACE1001185//"Homo sapiens mRNA for KIAA0943 protein, partial cds."//0//1668bp//99%//AB023160
C-PLACE1001238//"Mouse mRNA for RNA polymerase I associated factor (PAF53), complete cds."//2E-202//1333bp//80%//D14336
C-PLACE1001280
C-PLACE1001294//M.musculus GEG-154 mRNA.//4.3E-221//1057bp//78%//X7l642
C-PLACE1001304//"Homo sapiens zinc finger protein dp mRNA, complete cds."//0//2421bp//99%//AF153201
C-PLACE1001311
C-PLACE1001323
C-PLACE1001351
C-PLACE1001414
C-PLACE1001440
C-PLACE1001456
C-PLACE1001517//"Homo sapiens gene for glycosylphosphatidylinositol anchor attachment 1 (GPAA1), complete cds."//4.60E-112//392bp//87%//AB002137
C-PLACE1001602//CCR4-ASSOCIATED FACTOR 1 (CAF1).//5.7E-130//244aa//99%//Q60809
C-PLACE1001632//ZINC FINGER PROTEIN 84 (ZINC FINGER PROTEIN HPF2).//1.4E-118//429aa//48%//P51523
C-PLACE1001634
C-PLACE1001640
C-PLACE1001672//PROBABLE AMINOTRANSFERASE T01B11.2 (EC 2.6.1.-).//4.3E-66//174aa//45%//P91408
C-PLACE1001705
C-PLACE1001716
C-PLACE1001720
C-PLACE1001745
C-PLACE1001748//"Homo sapiens metalloprotease 1 (MP1) mRNA, complete cds."//0//2602bp//99%//AF061243
C-PLACE1001771//Homo sapiens mRNA for transient receptor potential protein
TRP6.//0//2900bp//99%//AJ006276
C-PLACE1001799
C-PLACE1001845//"Mus musculus cyclin ania-6a mRNA, complete cds."//3.30E-31//925bp//62%//AF159159
C-PLACE1001897
C-PLACE1002090//SIGNAL RECOGNITION PARTICLE 72 KD PROTEIN (SRP72).//6.5E-58//112aa//100%//076094
C-PLACE1002157
C-PLACE1002171//TRANSCRIPTION REGULATORY PROTEIN SWI3 (SWI/SNF COMPLEX COMPONENT SWI3) (TRANSCRIPTION FACTOR TYE2).//0.00005//179aa//23%//P32591
C-PLACE1002227
C-PLACE1002259
C-PLACE1002319
C-PLACE1002395//"Mus musculus mRNA for UBE-1c1, UBE-1c2, UBE-1c3, complete cds."//7.9E-100//966bp//75%//AB030505
C-PLACE1002477
C-PLACE1002493//"Homo sapiens signal transducing adaptor molecule 2A (STAM2) mRNA, complete cds."//1.7E-113//545bp//98%//AF042273
C-PLACE1002500
C-PLACE1002514
C-PLACE1002532//HOMEOBOX PROTEIN DLX-5.//1.2E-152//289aa//96%//P70396
C-PLACE1002537
C-PLACE1002571//ACTIN-LIKE PROTEIN 13E.//5E-99//386aa//48%//P45890
C-PLACE10025 83//"GLUTAMATE RECEPTOR, IONOTROPIC KAINATE 2 PRECURSOR (GLUTAMATE RECEPTOR 6) (GLUR-6) (GLUTAMATE RECEPTOR BETA-2) (GLUR BETA-2) (FRAGMENT)."//5.6E-34//76aa//98%//P39087
C-PLACE1002598//OLIGORIBONUCLEASE (EC 3.1.-.-).//5.5E-17//76aa//56%//P45340
C-PLACE1002625
C-PLACE1002655//ADSEVERIN (SCINDERIN)(SC).//2.5E-278//543aa//92%//Q28046
C-PLACE1002768
C-PLACE1002782//"Rattus norvegicus zinc transporter (ZnT-2) mRNA, complete cds."//3.8E-43//385bp//77%//U50927
C-PLACE1002816//HISTONE DEACETYLASE HDA1.//2.20E-48//217aa//46%//P53973
C-PLACE1002853
C-PLACE1002908//"Homo sapiens XGalT-1 mRNA for galactosyltransferase I, complete cds."//0//1654bp//99%//AB028600
C-PLACE1002962
C-PLACE1002968
C-PLACE1002991//PUTATIVE AMIDASE (EC 3.5.1.4).//1.4E-78//496aa//37%//Q49091
C-PLACE1003025
C-PLACE1003027//"Homo sapiens mRNA for KIAA0516 protein, partial cds."//2.1e-314//1417bp//100%//AB011088
C-PLACE1003044//"Homo sapiens mRNA for KIAA0829 protein, partial cds."//0//1382bp//96%//AB020636
C-PLACE1003176
C-PLACE1003238//PROBABLE G PROTEIN-COUPLED RECEPTOR KIAA0001.//4.9E-76//309aa//47%//Q15391
C-PLACE1003256
C-PLACE1003258//EARLY EMBRYOGENESIS ZYG-11 PROTEIN.//7.9E-22//70aa7/47%//P21541
C-PLACE1003343
C-PLACE1003361
C-PLACE1003366//"Homo sapiens otoferlin (OTOF) mRNA, complete cds."//1.4E-78//542bp//67%//AF107403
C-PLACE1003373
C-PLACE1003375
C-PLACE1003394//"Sprague-Dawley (clone LRB13) RAB14 mRNA, complete cds."//2.30E-150//774bp//94%//M83680
C-PLACE1003420//PUTATIVE MITOCHONDRIAL CARRIER YIL006W.//1.3E-40//278aa//36%//P40556
C-PLACE1003454
C-PLACE1003478
C-PLACE1003516
C-PLACE1003519//H.sapiens hnRNP-E2 mRNA.//5.1E-218//905bp//99%//X78136
C-PLACE1003521//HYPOTHETICAL HELICASE C28H8.3 IN CHROMOSOME III.//0.0000011//101aa//32%//Q09475
C-PLACE1003528
C-PLACE1003537//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//7.7E-68//404aa//33%//P32802
C-PLACE1003566
C-PLACE1003584
C-PLACE1003593
C-PLACE1003605//HAP5 TRANSCRIPTIONAL
ACTIVATOR.//0.00000023//82aa//35%//Q02516
C-PLACE1003618
C-PLACE1003638
C-PLACE1003738//ZINC FINGER PROTEIN 135.//9.6E-118//350aa//46%//P52742
C-PLACE1003760//"Homo sapiens tetraspanin TM4-A mRNA, complete cds."//5.2E-289//1313bp//97%//AF133423
C-PLACE1003768
C-PLACE1003795
C-PLACE1003886
C-PLACE1003888//"Homo sapiens mRNA for KIAA1092 protein, partial cds."//0//2057bp//99%//AB029015
C-PLACE1003903//CTP SYNTHASE (EC 6.3.4.2) (UTP--AMMONIA LIGASE) (CTP SYNTHETASE).//1.4E-243//584aa//74%//P17812
C-PLACE1003915//"PROBABLE ARGINYL-TRNA SYNTHETASE, CYTOPLASMIC (EC 6.1.1.19) (ARGININE- -TRNA LIGASE) (ARGRS)."//2.4E-108//581aa//40%//Q05506
C-PLACE1004118
C-PLACE1004256//"Mus musculus short coiled coil protein SCOCO (Scoc) mRNA, complete cds."//2E-93//960bp//76%//AF115778
C-PLACE1004274
C-PLACE1004284
C-PLACE1005331
C-PLACE1005739//Homo sapiens mRNA; cDNA DKFZp564A032 (from clone DKFZp564A032).//0//2190bp//99%//AL050267
C-PLACE1005828
C-PLACE1005876//"CLEAVAGE AND POLYADENYLATION SPECIFICITY FACTOR, 100 KD SUBUNIT (CPSF 100 KD SUBUNIT)."//0//730aa//99%//Q10568
C-PLACE1005890//BEM46 PROTEIN (FRAGMENT).//9.9E-42//224aa//43%//P54069
C-PLACE1006157//E-SELECTIN PRECURSOR (ENDOTHELIAL LEUKOCYTE ADHESION MOLECULE 1) (ELAM-1) (LEUKOCYTE-ENDOTHELIAL CELL ADHESION MOLECULE 2) (LECAM2) (CD62E).//2E-28//236aa//30%//P98110
C-PLACE1007053
C-PLACE1007068
C-PLACE1008368//RING CANAL PROTEIN (KELCH PROTEIN).//5.3E-26//309aa//30%//Q04652
C-PLACE1009921
C-PLACE1010401
C-PLACE1010856
C-PLACE1010857
C-PLACE1010917
C-PLACE1010925
C-PLACE1010926//"Homo sapiens mRNA for KIAA0554 protein, partial cds."//0//1160bp//100%//AB011126
C-PLACE1010942//"Homo sapiens intersectin long isoform (ITSN) mRNA, complete cds."//0//1440bp//99%//AF114487
C-PLACE1010944
C-PLACE1010954
C-PLACE1010960//ACTIN-LIKE PROTEIN 13E.//5.3E-98//297aa//48%//P45890
C-PLACE1011026
C-PLACE1011046//"1-PHOSPHATIDYLINOSITOL-4,5-BISPHOSPHATE PHOSPHODIESTERASE BETA 1 (EC 3.1.4.11) (PLC-BETA-1) (PHOSPHOLIPASE C-BETA-1) (PLC-I) (PLC-154)."//0//646aa//97%//P10894
C-PLACE1011054
C-PLACE1011057
C-PLACE1011109//"ELONGATION FACTOR G, MITOCHONDRIAL PRECURSOR (MEF-G)."//1.50E-22//63aa//88%//Q07803
C-PLACE1011114//PROBABLE ATP-DEPENDENT RNA HELICASE HAS1.//2.9E-71//190aa//44%//Q03532
C-PLACE1011133
C-PLACE1011143
C-PLACE1011165
C-PLACE1011185//INSERTION ELEMENT IS1 PROTEIN INSB.//1.3E-89//167aa//100%//P03830
C-PLACE1011219//PROBABLEOXIDOREDUCTASE (EC 1.-.-.-).//3.2E-12//212aa//29%//Q03326
C-PLACE1011221
C-PLACE1011263//Homo sapiens mRNA; cDNA DKFZp5640043 (from clone DKFZp564O043).//0//2487bp//99%//AL050390
C-PLACE1011325
C-PLACE1011332//"Homo sapiens N-acetylglucosamine-phosphate mutase mRNA, complete cds."//7.2E-151//697bp//99%//AF102265
C-PLACE1011340//"Homo sapiens IDN3-B mRNA, complete cds."//1.20E-74//380bp//97%//AB019602
C-PLACE1011399//"Homo sapiens CGI-72 protein mRNA, complete cds."//3.2E-90//427bp//99%//AF151830
C-PLACE1011433//"Homo sapiens mRNA for KIAA0530 protein, partial cds."//0//1946bp//99%//AB011102
C-PLACE1011452
C-PLACE1011465
C-PLACE1011472//"Homo sapiens mRNA for KIAA0712 protein, complete cds."//0//2022bp//99%//AB018255
C-PLACE1011477//"Homo sapiens sorting nexin 2 (SNX2) mRNA, complete cds."//0//2040bp//99%//AF065482
C-PLACE1011492//NON-GREEN PLASTID TRIOSE PHOSPHATE TRANSLOCATOR PRECURSOR (CTPT).//4.90E-11//147aa//32%//P52178
C-PLACE1011520
C-PLACE1011563
C-PLACE1011567
C-PLACE1011576//"Human Kruppel related zinc finger protein (HTF10) mRNA, complete cds."//0//1791bp//82%//L11672
C-PLACE1011586
C-PLACE1011643
C-PLACE1011649
C-PLACE1011664//CROOKED NECK PROTEIN.//1.6E-187//505aa//64%//P17886
C-PLACE1011682
C-PLACE1011719
C-PLACE1011729
C-PLACE1011858//Homo sapiens mRNA; cDNA DKFZp586C021 (from clone DKFZp586C021).//0//1490bp//99%//AL050287
C-PLACE1011874
C-PLACE1011875//"Homo sapiens mRNA for KIAA0580 protein, partial cds."//4.1E-112//524bp//100%//AB011152
C-PLACE1011923//"Homo sapiens serum-inducible kinase mRNA, complete cds."//0//2782bp//99%//AF059617
C-PLACE1011982
C-PLACE2000014//HYPOTHETICAL HELICASE C28H8.3 IN CHROMOSOME III.//2.6E-42//104aa//49%//Q09475
C-PLACE2000015//EPIDERMAL GROWTH FACTOR RECEPTOR SUBSTRATE SUBSTRATE 15 (PROTEIN EPS15) (AF-1P PROTEIN).//1.1E-116//364aa//45%//P42566
C-PLACE2000017
C-PLACE2000021//"Homo sapiens TRF1-interacting ankyrin-related ADP-ribose polymerase mRNA, complete cds."//2.7E-107//981bp//74%//AF082556
C-PLACE2000047
C-PLACE2000062//"Homo sapiens mRNA for type II membrane protein similar to HIV gp120-binding C-type lectin, complete cds, clone:HP01347."//6.3E-166//656bp//94%//AB015629
C-PLACE2000100
C-PLACE2000111
C-PLACE2000172
C-PLACE2000187
C-PLACE2000216//"Dog nonerythroid beta-spectrin mRNA, 3' end.&quot,//3.2E-253//1799bp//83%//L02897
C-PLACE2000246//"Homo sapiens mRNA for KIAA0795 protein, partial cds."//4.60E-172//796bp//99%//AB018338
C-PLACE2000317
C-PLACE2000341//"Homo sapiens sodium-dependent multivitamin transporter (SMVT) mRNA, complete cds."/0//1554bp//99%//AF069307
C-PLACE2000366
C-PLACE2000373//F-SPONDIN PRECURSOR.//8.6E-16//371aa//28%//P35446
C-PLACE2000394
C-PLACE2000398//LAR PROTEIN PRECURSOR (LEUKOCYTE ANTIGEN RELATED) (EC 3.1.3.48).//6.3E-37//90aa//98%//P10586
C-PLACE2000411//"Homo sapiens mRNA for KIAA1037 protein, partial cds."//0//2515bp//99%//AB028960
C-PLACE2000425
C-PLACE2000427//PROBABLE HELICASE MOT1.//1.2E-26//200aa//27%//P32333
C-PLACE2000433
C-PLACE2000438//"POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N-ACETYLGALACTOSAMINYLTRANSFERASE)(GALNAC-T1)."//2.1E-86//348aa//41%//Q10472
C-PLACE2000458//CADHERIN-RELATED TUMOR SUPPRESSOR PRECURSOR (FAT PROTEIN).//2.5E-25//165aa//40%//P33450
C-PLACE2000477//"Homo sapiens putative secreted protein (ZSIG11) mRNA, complete cds."//6.7E-127//671bp//94%//AF072733
C-PLACE3000009
C-PLACE3000020//"Homo sapiens type III adenylyl cyclase (AC-III) mRNA, complete cds."//0//2253bp//99%//AF033861
C-PLACE3000103
C-PLACE3000142
C-PLACE3000145//TENSIN.//1E-108//277aa//75%//Q04205
C-PLACE3000156
C-PLACE3000157
C-PLACE3000197
C-PLACE3000208
C-PLACE3000226//"Homo sapiens mRNA for KIAA0962 protein, partial cds."//0//4805bp//99%//AB023179
C-PLACE3000242//"Homo sapiens mRNA for KIAA1114 protein, complete cds."//0//2786bp//96%//AB029037
C-PLACE3000363
C-PLACE3000405
C-PLACE3000416//"Homo sapiens mRNA for actin binding protein ABP620, complete cds."//1.80E-141//565bp//98%//AB029290
C-PLACE3000477
C-PLACE4000106//"Homo sapiens mRNA for KIAA0462 protein, partial cds."//0//6702bp//99%//AB007931
C-PLACE4000323
C-PLACE4000326//NAM7 PROTEIN (NONSENSE-MEDIATED MRNA DECAY PROTEIN 1) (UP-FRAMESHIFT SUPPRESSOR 1).//8.10E-24//319aa//31%//P30771
C-PLACE4000369//"Homo sapiens mRNA for KIAA1025 protein, partial cds."//0//4830bp//99%//AB028948
C-PLACE4000445//Homo sapiens mRNA; cDNA DKFZp434C212 (from clone DKFZp434C212).//0//2565bp//99%//AL080196
C-PLACE4000558//"Homo sapiens mRNA for KIAA0729 protein, partial cds."//0//1051bp//97%//AB018272
C-PLACE4000581//FIBROPELLIN I PRECURSOR (EPIDERMAL GROWTH FACTOR-RELATED PROTEIN 1) (UEGF-1).//9.3E-70//226aa//52%//P10079
C-PLACE4000593
C-PLACE4000612//POL POLYPROTEIN [CONTAINS: PROTEASE (EC 3.4.23.-); REVERSE TRANSCRIPTASE (EC 2.7.7.49); ENDONUCLEASE].//7.1E-154//340aa//40%//P21414
C-PLACE4000670
C-THYRO1000026
C-THYRO1000085//"PAIRED BOX PROTEIN PAX-8, ISOFORMS 8A/8B."//2E-72//155aa//92%//Q06710
C-THYRO1000107
C-THYRO1000111
C-THYRO1000132//"Homo sapiens echinoderm microtubule-associated protein homolog HuEMAP mRNA, complete cds."//1.1E-159//824bp//95%//U97018
C-THYRO1000156
C-THYRO1000173//"Homo sapiens AP-mu chain family member mu1B (HSMU1B) mRNA, complete cds."//0//1713bp//99%//AF020797
C-THYRO1000186
C-THYRO1000187
C-THYRO1000241
C-THYRO1000279
C-THYRO1000327//"Homo sapiens autocrine motility factor receptor (AMFR) mRNA, complete cds."//0//1567bp//99%//AF124145
C-THYRO1000452
C-THYRO1000471
C-THYRO1000484
C-THYRO1000502
C-THYRO1000505
C-THYRO1000585//"Homo sapiens protein associated with Myc mRNA, complete cds."//0//1901bp//99%//AF075587
C-THYRO1000596
C-THYRO1000662//"Homo sapiens XPV mRNA for DNA polymerase eta, complete cds."//0//2341 bp//99%//AB024313
C-THYRO1000666//Mus musculus mRNA for kinesin like protein 9.//0//2001bp//86%//AJ132889
C-THYRO1000715
C-THYRO1000734
C-THYRO1000748//RHO-GAP HEMATOPOIETIC PROTEIN C1 (P115) (KIAA0131).//3.30E-96//335aa//52%//P98171
C-THYRO1000756//"ALPHA-N-ACETYLGALACTOSAMINIDE ALPHA-2,6-SIALYLTRANSFERASE (EC 2.4.99.-) (ST6GALNACIII) (STY)."//1.8E-55//243aa//42%//Q64686
C-THYRO1000777
C-THYRO1000783//"Xenopus laevis tail-specific thyroid hormone up-regulated (gene 5) mRNA, complete cds."//2.4E-157//1656bp//70%//U37373
C-THYRO1000787
C-THYRO1000793
C-THYRO1000796
C-THYRO1000843
C-THYRO1000852//"Human branched chain aminotransferase precursor (BCATm) mRNA, nuclear gene encoding mitochondrial protein, complete cds."//3.3E-147//790bp//93%//U68418
C-THYRO1000865
C-THYRO1000895
C-THYRO1000926//"Homo sapiens cAMP-specific phosphodiesterase 8B (PDE8B) mRNA, partial cds."//0//2387bp//99%//AF079529
C-THYRO1000951//DIHYDROXYACETONE KINASE 2 (EC 2.7.1.29) (GLYCERONE KINASE).//5E-83//566aa//37%//P43550
C-THYRO1000952
C-THYRO1000983//UBIQUITIN-CONJUGATING ENZYME E2-17 KD 9 (EC 6.3.2.19) (UBIQUITIN-PROTEIN LIGASE 9) (UBIQUITIN CARRIER PROTEIN 9) (UBCAT4B).//6.30E-17//143aa//39%//P35132
C-THYRO1001003//UBIQUITIN-CONJUGATING ENZYME E2-21.2 KD (EC 6.3.2.19) (UBIQUITIN-PROTEIN LIGASE) (UBIQUITIN CARRIER PROTEIN).//5.90E-14//84aa//41%//P52491
C-THYRO1001031
C-THYRO1001062
C-THYRO1001100//ZINC FINGER X-LINKED PROTEIN ZXDA (FRAGMENT).//1.2E-67//245aa//62%//P98168
C-THYRO1001133
C-THYRO1001134//"Homo sapiens CGI-78 protein mRNA, complete cds."//0//1898bp//99%//AF151835
C-THYRO1001173
C-THYRO1001213
C-THYRO1001321
C-THYRO1001322
C-THYRO1001365
C-THYRO1001401
C-THYRO1001411
C-THYRO1001434
C-THYRO1001534
C-THYRO1001541
C-THYRO1001559
C-THYRO1001570
C-THYRO1001595
C-THYRO1001605
C-THYRO1001617//Homo sapiens cDNA for dihydroxyacetone phosphate acyltransferase (DAP-AT).//0//1784bp//99%//AJ002190
C-THYRO1001656//"Homo sapiens Leman coiled-coil protein (LCCP) mRNA, complete cds."//4.1E-273//1947bp//82%//AF175968
C-THYRO1001671//Homo sapiens mRNA for 2'-5' oligoadenylate synthetase 59 kDa isoform.//0//1820bp//99%//AJ225089
C-THYRO1001673
C-THYRO1001703//NIFR3-LIKE PROTEIN.//2.90E-32//282aa//32%//P45672
C-THYRO1001706
C-THYRO1001738//TUBULIN-TYROSINE LIGASE (EC 6.3.2.25) (TTL).//2.4E-20//217aa//30%//P38584
C-THYRO1001745
C-THYRO1001793
C-THYRO1001809//MYOCYTE NUCLEAR FACTOR (MNF).//1.4E-74//158aa//89%//P42128
C-THYRO1001895
C-THYRO1001907
C-VESEN1000122
C-Y79AA1000037//DNA-BINDING PROTEIN BMI-1.//2.4E-30//80aa//60%//P25916
C-Y79AA1000059//"Homo sapiens immunophilin homolog ARA9 mRNA, complete cds."//2.9E-70//1040bp//65%//U78521
C-Y79AA1000065
C-Y79AA1000131
C-Y79AA1000181//"Homo sapiens CGI-01 protein mRNA, complete cds."//0//1858bp//99%//AF132936
C-Y79AA1000202
C-Y79AA1000214//"Homo sapiens histone H2A.F/Z variant (H2AV) mRNA, complete cds."//7.1E-71//345bp//100%//AF081192
C-Y79AA1000230
C-Y79AA1000258
C-Y79AA1000268//"Mus musculus Nip21 mRNA, complete cds."//2.10E-50//648bp//64%//AF035207
C-Y79AA1000313//CALPHOTIN.//0.000011//336aa//23%//Q02910
C-Y79AA1000328//SEL-10 PROTEIN.//0.000000067//219aa//25 %//Q93794
C-Y79AA1000355
C-Y79AA1000368//REDUCED VIABILTTY UPON STARVATION PROTEIN 161.//4E-20//261 aa//27%//P25343
C-Y79AA1000420
C-Y79AA1000469//"Mus musculus ancient ubiquitous 46 kDa protein AUP1 precursor (Aup1) mRNA, complete cds."//8.30E-252//1207bp//85%//U41736
C-Y79AA1000480
C-Y79AA1000540
C-Y79AA1000560//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT).//0//652aa//98%//P17427
C-Y79AA1000574//Homo sapiens clone H17 unknown mRNA.//0//1932bp//99%//AF103801
C-Y79AA1000627//"Homo sapiens zinc finger protein (ZF5128) mRNA, complete cds."//2E-287//203 lbp//82%//AF060503
C-Y79AA1000705//M.musculus mRNA of enhancer-trap-locus 1.//5.80E-254//1477bp//84%//X69942
C-Y79AA1000734//"Homo sapiens peroxisomal biogenesis factor (PEX11b) mRNA, complete cds."//0//1594bp//99%//AF093670
C-Y79AA1000748//"Homo sapiens CGI-05 protein mRNA, complete cds."//1.9E-239//1367bp//91%//AF152097
C-Y79AA1000752//PUTATIVE HETEROGENEOUS NUCLEAR RIBONUCLEOPROTEIN X (HNRNP X) (CBP).//4.9E-91//200aa//64%//Q61990
C-Y79AA1000774
C-Y79AA1000782//CYTOSOLIC PURINE 5'-NUCLEOTEDASE (EC 3.1.3.5).//3E-37//469aa//27%//P49902
C-Y79AA1000784//"Homo sapiens RanBP7/importin 7 mRNA, complete cds."//1.10E-236//1076bp//99%//AF098799
C-Y79AA1000794//"Homo sapiens actin-associated protein 2E4/kaptin (2E4) mRNA, 2E4-1 allele, complete cds."//0//1610bp//99%//AF105369
C-Y79AA1000800//"Homo sapiens putative secreted protein (ZSIG11) mRNA, complete cds."//1.6E-284//1288bp//99%//AF072733
C-Y79AA1000805
C-Y79AA1000824
C-Y79AA1000833//TUBULIN ALPHA-1 CHAIN.//5E-173//220aa//79%//P05209
C-Y79AA1000850
C-Y79AA1000962//"MYOSIN HEAVY CHAIN, NON-MUSCLE (ZIPPER PROTEIN) (MYOSIN II)."//4.2E-17//430aa//27%//Q99323
C-Y79AA1000968//"Rattus norvegicus initiation factor eIF-2B gamma subunit (eIF-2B gamma) mRNA, complete cds."//3.9E-248//1468bp//87%//U38253
C-Y79AA1000976
C-Y79AA1001023
C-Y79AA1001041
C-Y79AA1001048//"ACYL-COA DEHYDROGENASE, VERY-LONG-CHAIN SPECIFIC PRECURSOR (EC 1.3.99.-) (VLCAD)."//3.1E-138//583aa//47%//P45953
C-Y79AA1001077
C-Y79AA1001078
C-Y79AA1001145
C-Y79AA1001177
C-Y79AA1001185
C-Y79AA1001211//"Homo sapiens origin recognition complex subunit 6 (ORC6) mRNA, complete cds."//0//1435bp//99%//AF139658
C-Y79AA1001228
C-Y79AA1001233//ESTRADIOL 17 BETA-DEHYDROGENASE 1 (EC 1.1.1.62) (17-BETA-HSD 1) (17-BETA-HYDROXYSTEROID DEHYDROGENASE 1).//7.7E-50//228aa//42%//P51657
C-Y79AA1001236//"Homo sapiens mRNA for JM23 protein, complete coding sequence (clone IMAGE 34581 and IMAGE 45355 and LLNLc110I133Q7 (RZPD Berlin))."//0//1653bp//99%//AJ005892
C-Y79AA1001281
C-Y79AA1001312//ZINC FINGER PROTEIN MLZ-4 (ZINC FINGER PROTEIN 46).//0.000000023//193aa//30%//Q03309
C-Y79AA1001323//"Mus musculus mRNA for GSG1, complete cds."//3.3E-172//1171bp//83%//D87325
C-Y79AA1001391//HOMEOBOX PROTEIN HOX-A13 (HOX-1J).//1.2E-58//178aa//66%//P31271
C-Y79AA1001394//CELL DIVISION PROTEIN FTSH HOMOLOG (EC 3.4.24.-).//1.2E-13//230aa//32%//O83746
C-Y79AA1001402//"Homo sapiens paraneoplastic cancer-testis-brain antigen (MA4) mRNA, partial cds."//8.50E-65//784bp//62%//AF083115
C-Y79AA1001493//UBIQUITIN-CONJUGATING ENZYME E2-17 KD 9 (EC 6.3.2.19) (UBIQUITIN-PROTEIN LIGASE 9) (UBIQUITIN CARRIER PROTEIN 9) (UBCAT4B).//3.80E-18//151aa//38%//P35132
C-Y79AA1001533//"Mouse mRNA for RNA polymerase I associated factor (PAF53), complete cds."//4.5E-193//1333bp//80%//D14336
C-Y79AA1001541
C-Y79AA1001548//PHOSPHATIDYLINOSITOL 4-KINASE ALPHA (EC 2.7.1.67) (PI4-KINASE) (PTDINS-4-KINASE) (PI4K-ALPHA).//7.5E-76//85aa//90%//P42356
C-Y79AA1001555
C-Y79AA1001581//ACETYL-COENZYME A SYNTHETASE (EC 6.2.1.1) (ACETATE--COA LIGASE) (ACYL- ACTIVATING ENZYME).//1.9E-40//482aa//27%//P27550
C-Y79AA1001585
C-Y79AA1001603//"POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N-ACETYLGALACTOSAMINYLTRANSFERASE) (GALNAC-T1)."//1.7E-84//313aa//48%//Q07537
C-Y79AA1001613//ZINC FINGER PROTEIN 132.//3.8E-91//209aa//41%//P52740
C-Y79AA1001665
C-Y79AA1001679//"Homo sapiens lambda-crystallin mRNA, complete cds."//3.4e-310//1430bp//98%//AF077049
C-Y79AA1001696//"Homo sapiens mRNA for KIAA1109 protein, partial cds."//0//1669bp//100%//AB029032
C-Y79AA1001705//"Homo sapiens p53 regulated PA26-T2 nuclear protein (PA26) mRNA, complete cds."//3.4E-47//626bp//68%//AF033120
C-Y79AA1001711//"Human 60-kdal ribonucleoprotein (Ro) mRNA, complete cds."//1.2E-258//1185bp//99%//J04137
C-Y79AA1001781
C-Y79AA1001805
C-Y79AA1001827//"Homo sapiens mammalian inositol hexakisphosphate kinase 2 (IP6K2) mRNA, complete cds."//0//1689bp//98%//AF177145
C-Y79AA1001846
C-Y79AA1001923
C-Y79AA1001963//PUTATIVE PRE-MRNA SPLICING FACTOR ATP-DEPENDENT RNA HELICASE SPAC10F6.02C.//1E-10//94aa//47%//O42643
C-Y79AA1002027//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//9.9E-39//143aa//52%//P42743
C-Y79AA1002083//H.sapiens mRNA for MUF1 protein.//5E-163//752bp//99%//X86018
C-Y79AA1002089
C-Y79AA1002115
C-Y79AA1002125
C-Y79AA1002204
C-Y79AA1002208//ANKYRIN.//8.1E-34//188aa//38%//Q02357
C-Y79AA1002209//"Homo sapiens CGI-04 protein mRNA, complete cds."//0//1617bp//99%//AF132939
C-Y79AA1002229//DNA CROSS-LINK REPAIR PROTEIN PSO2/SNM1.//7.10E-17//213aa//31%//P30620
C-Y79AA1002246//SYNAPTOTAGMIN V.//1.6E-28//286aa//32%//000445
C-Y79AA1002298
C-Y79AA1002307//"Homo sapiens astrotactin2 (ASTN2) mRNA, complete cds."//0//1209bp//99%//AF116574
C-Y79AA1002311//R.norvegicus mRNA for cytosolic resiniferatoxin-binding protein.//2.9E-186//1130bp//82%//X67877
C-Y79AA1002351
C-Y79AA1002407
C-Y79AA1002433//"Homo sapiens chromatin-specific transcription elongation factor FACT 140 kDa subunit mRNA, complete cds."//0//1545bp//96%//AF152961
C-Y79AA1002472//ZINC FINGER PROTEIN 91 (ZINC FINGER PROTEIN HTF10) (HPF7).//1.5E-136//472aa//49%//Q05481

### Homology Search Result Data 13.

Data obtained by the homology search for full-length nucleotide sequences and deduced amino acid sequences. Each data includes Clone name, Definition in hit data, P value, Length of sequence to be compared, Homology, and Accession number (No.) of hit data. These items are shown in this order and separated by a double-slash mark, //.
C-HEMBA1000042
C-HEMBA1000141//Homo sapiens SUMO-1-specific protease (SSP1) mRNA, complete cds.//0//1135bp//100%//AF196304
C-HEMBA1000150//H.sapiens gene for U5 snRNP-specific 200kD protein.//2.50E-153//525bp//91%//Z70200
C-HEMBA1000213
C-HEMBA1000243
C-HEMBA1000244
C-HEMBA1000251
C-HEMBA1000338
C-HEMBA1000357
C-HEMBA1000376
C-HEMBA1000428
C-HEMBA1000469
C-HEMBA1000497
C-HEMBA1000561//ZINC FINGER PROTEIN 91 (ZINC FINGER PROTEIN HTF10) (HPF7).//3.40E-37//674aa//25%//Q05481
C-HEMBA1000569//GPI-ANCHORED PROTEIN P137.//6.50E-19//265aa//32%//Q60865
C-HEMBA1000575
C-HEMBA1000591//PTB-ASSOCIATED SPLICING FACTOR (PSF).//2.20E-17//198aa//40%//P23246
C-HEMBA1000673
C-HEMBA1000702
C-HEMBA1000722
C-HEMBA1000726
C-HEMBA1000876
C-HEMBA1000942
C-HEMBA1000943
C-HEMBA1000960
C-HEMBA1000985
C-HEMBA1001019//CELL DIVISION CONTROL PROTEIN 2 HOMOLOG (EC 2.7.1.-) (P34 PROTEIN KINASE) (CYCLIN-DEPENDENT KINASE 1) (CDK1).//3.10E-10//70aa//58%//P06493
C-HEMBA1001020
C-HEMBA1001024
C-HEMBA1001026
C-HEMBA1001051
C-HEMBA1001060
C-HEMBA1001071//PROCOLLAGEN ALPHA 1(III) CHAIN PRECURSORS.//1.50E-92//82aa//100%//P02461
C-HEMBA1001077//Homo sapiens transcriptional intermediary factor 1 gamma mRNA, complete cds.//2.00E-80//432bp//94%//AF119043
C-HEMBA1001099
C-HEMBA1001121
C-HEMBA1001123
C-HEMBA1001208
C-HEMBA1001213
C-HEMBA1001226
C-HEMBA1001247
C-HEMBA1001299
C-HEMBA1001319
C-HEMBA1001323
C-HEMBA1001327
C-HEMBA1001361
C-HEMBA1001375
C-HEMBA1001377
C-HEMBA1001383
C-HEMBA1001391
C-HEMBA1001411
C-HEMBA1001432
C-HEMBA1001433
C-HEMBA1001435
C-HEMBA1001442
C-HEMBA1001463
C-HEMBA1001515
C-HEMBA1001522
C-HEMBA1001557
C-HEMBA1001566
C-HEMBA1001589
C-HEMBA1001608
C-HEMBA1001636
C-HEMBA1001647
C-HEMBA1001651
C-HEMBA1001658
C-HEMBA1001675//VACUOLAR PROTEIN SORTING-ASSOCIATED PROTEIN VPS9.//5.40E-09//101aa//35%//P54787
C-HEMBA1001712
C-HEMBA1001734//CADHERIN-11 PRECURSOR (OSTEOBLAST-CADHERIN) (OB-CADHERIN) (OSF-4).//1.10E-38//87aa//96%//P55288
C-HEMBA1001745
C-HEMBA1001750
C-HEMBA1001784
C-HEMBA1001791
C-HEMBA1001803
C-HEMBA1001820
C-HEMBA1001835
C-HEMBA1001888
C-HEMBA1001912
C-HEMBA1001915
C-HEMBA1001918
C-HEMBA1001940
C-HEMBA1001942
C-HEMBA1001964
C-HEMBA1002022
C-HEMBA1002039
C-HEMBA1002100
C-HEMBA1002113
C-HEMBA1002119
C-HEMBA1002139//LIM AND SH3 DOMAIN PROTEIN LASP-1 (MLN 50).//7.10E-05//51aa//49%//Q14847
C-HEMBA1002160
C-HEMBA1002162
C-HEMBA1002166
C-HEMBA1002185
C-HEMBA1002204
C-HEMBA1002328
C-HEMBA1002337
C-HEMBA1002348
C-HEMBA1002381
C-HEMBA1002486
C-HEMBA1002498
C-HEMBA1002538
C-HEMBA1002552
C-HEMBA1002555//Homo sapiens mSin3A associated polypeptide p30 mRNA, complete cds.//5.30E-51//768bp//68%//AF055993
C-HEMBA1002558
C-HEMBA1002621
C-HEMBA1002629
C-HEMBA1002645
C-HEMBA1002659
C-HEMBA1002661
C-HEMBA1002666
C-HEMBA1002678
C-HEMBA1002679
C-HEMBA1002712
C-HEMBA1002716
C-HEMBA1002742
C-HEMBA1002746//DNA POLYMERASE BETA (EC 2.7.7.7).//5.00E-37//268aa//34%//P06746
C-HEMBA1002748
C-HEMBA1002780
C-HEMBA1002801
C-HEMBA1002826
C-HEMBA1002833
C-HEMBA1002921
C-HEMBA1002934
C-HEMBA1002944
C-HEMBA1002968
C-HEMBA1003034
C-HEMBA1003037
C-HEMBA1003071//INSULIN-LIKE GROWTH FACTOR BINDING PROTEIN COMPLEX ACID LABILE CHAIN PRECURSOR (ALS).//1.30E-09//121aa//40%//P35858
C-HEMBA1003078
C-HEMBA1003083
C-HEMBA1003086
C-HEMBA1003098//Homo sapiens NY-REN-6 antigen mRNA, partial cds.//6.20E-273//1253bp//99%//AF155096
C-HEMBA1003133
C-HEMBA1003142
C-HEMBA1003166
C-HEMBA1003197
C-HEMBA1003202
C-HEMBA1003220
C-HEMBA1003229
C-HEMBA1003276
C-HEMBA1003278
C-HEMBA1003328
C-HEMBA1003373
C-HEMBA1003597
C-HEMBA1003598
C-HEMBA1003656
C-HEMBA1003680//PUTATIVE AMINOPEPTIDASE ZK353.6 IN CHROMOSOME III (EC 3.4.11.-).//2.40E-92//423aa//47%//P34629
C-HEMBA1003733
C-HEMBA1003742
C-HEMBA1003760//HYPOXIA-INDUCIBLE FACTOR 1 ALPHA (HIF-1 ALPHA) (ARNT INTERACTING PROTEIN) (MEMBER OF PAS PROTEIN 1) (MOP1) (HIF1 ALPHA).//3.70E-124//347aa//55%//Q16665
C-HEMBA1003803
C-HEMBA1003854
C-HEMBA1003926
C-HEMBA1003939
C-HEMBA1003987
C-HEMBA1004012
C-HEMBA1004015
C-HEMBA1004193
C-HEMBA1004225
C-HEMBA1004241
C-HEMBA1004267
C-HEMBA1004295//Homo sapiens NY-REN-25 antigen mRNA, partial cds.//9.40E-31//381bp//65%//AF155103
C-HEMBA1004354//CHL1 PROTEIN.//9.90E-26//130aa//42%//P22516
C-HEMBA1004356//H.sapiens MSSP-2 mRNA.//3.00E-243//573bp//98%//X77494
C-HEMBA1004396
C-HEMBA1004405
C-HEMBA1004433
C-HEMBA1004538
C-HEMBA1004542
C-HEMBA1004573
C-HEMBA1004577
C-HEMBA1004604//Homo sapiens COP9 complex subunit 7a mRNA, complete cds.//0//1612bp//99%//AF193844
C-HEMBA1004617
C-HEMBA1004631
C-HEMBA1004705
C-HEMBA1004733
C-HEMBA1004748
C-HEMBA1004778
C-HEMBA1004803
C-HEMBA1004807
C-HEMBA1004820
C-HEMBA1004865
C-HEMBA1004880
C-HEMBA1004900
C-HEMBA1004909
C-HEMBA1004960
C-HEMBA1004978
C-HEMBA1004980
C-HEMBA1004983
C-HEMBA1004995
C-HEMBA1005019//Homo sapiens mRNA for KIAA0648 protein, partial cds.//0//2212bp//99%//AB014548
C-HEMBA1005029//Homo sapiens CGI-13 protein mRNA, complete cds.//0//1487bp//99%//AF132947
C-HEMBA1005035
C-HEMBA1005039
C-HEMBA1005047//RAS-RELATED PROTEIN RAB-24 (RAB-16).//3.40E-101//106aa//98%//P35290
C-HEMBA1005050
C-HEMBA1005062
C-HEMBA1005066
C-HEMBA1005075
C-HEMBA1005079
C-HEMBA1005101//Homo sapiens SYT interacting protein SIP mRNA, complete cds.//0//2762bp//99%//AF080561
C-HEMBA1005123
C-HEMBA1005149
C-HEMBA1005152
C-HEMBA1005201//Homo sapiens CGI-07 protein mRNA, complete cds.//0//1608bp//99%//AF132941
C-HEMBA1005202//SIGNAL RECOGNITION PARTICLE 68 KD PROTEIN (SRP68).//1.90E-179//361aa//95%//Q00004
C-HEMBA1005223
C-HEMBA1005232
C-HEMBA1005241
C-HEMBA1005275
C-HEMBA1005293
C-HEMBA1005311
C-HEMBA1005338//Homo sapiens mRNA for matrilin-4, partial.//3.90E-241//1095bp//99%//AJ007581
C-HEMBA1005359//ZINC FINGER PROTEIN 137.//3.90E-85//206aa//69%//P52743
C-HEMBA1005367//Homo sapiens melastatin 1 (MLSN1) mRNA, complete cds.//9.00E-77//620bp//74%//AF071787
C-HEMBA1005374
C-HEMBA1005382
C-HEMBA1005411
C-HEMBA1005426
C-HEMBA1005443
C-HEMBA1005447
C-HEMBA1005497
C-HEMBA1005500
C-HEMBA1005506
C-HEMBA1005508
C-HEMBA1005526
C-HEMBA1005530//Homo sapiens anaphase-promoting complex subunit 7 (APC7) mRNA, complete cds.//0//1578bp//98%//AF191340
C-HEMBA1005548//Homo sapiens MAFB/Kreisler basic region/leucine zipper transcription factor (MAFB) mRNA, complete cds.//1.00E-220//1014bp//99%//AF134157
C-HEMBA1005552
C-HEMBA1005568
C-HEMBA1005588
C-HEMBA1005593
C-HEMBA1005606
C-HEMBA1005616
C-HEMBA1005627
C-HEMBA1005670
C-HEMBA1005679
C-HEMBA1005699
C-HEMBA1005705
C-HEMBA1005732//Human mRNA for KIAA1293 gene, complete cds.//5.50E-102//317bp//98%//D14697
C-HEMBA1005815//CALPAIN, LARGE [CATALYTIC] SUBUNIT (EC 3.4.22.17) (CALCIUM ACTIVATED NEUTRAL PROTEINASE) (CANP) (MU/M-TYPE).//2.00E-36//342aa//33%//P00789
C-HEMBA1005852
C-HEMBA1005894
C-HEMBA1005921
C-HEMBA1006035
C-HEMBA1006036
C-HEMBA1006090
C-HEMBA1006138
C-HEMBA1006173
C-HEMBA1006252
C-HEMBA1006268//Homo sapiens HQ0024c mRNA, complete cds.//3.50E-157//845bp//92%//AF073836
C-HEMBA1006347//MALES-ABSENT ON THE FIRST PROTEIN (EC 2.3.1.-).//1.60E-130//332aa//62%//002193
C-HEMBA1006359//ZINC FINGER PROTEIN 43 (ZINC PROTEIN HTF6).//3.50E-105//381aa//54%//P28160
C-HEMBA1006380
C-HEMBA1006416
C-HEMBA1006421
C-HEMBA1006424
C-HEMBA1006426
C-HEMBA1006446
C-HEMBA1006485//PUROMYCIN-SENSITIVE AMINOPEPTIDASE (EC 3.4.11.-) (PSA).//1.90E-81//153aa//97%//P55786
C-HEMBA1006486
C-HEMBA1006494
C-HEMBA1006546
C-HEMBA1006562
C-HEMBA1006595
C-HEMBA1006597
C-HEMBA1006631
C-HEMBA1006639
C-HEMBA1006652//60S RIBOSOMAL PROTEIN L7.//2.40E-44//206aa//47%//P14148
C-HEMBA1006659
C-HEMBA1006665
C-HEMBA1006676
C-HEMBA1006695
C-HEMBA1006709
C-HEMBA1006758//Homo sapiens protocadherin beta 13 (PCDH-beta13) mRNA, complete cds.//0//1832bp//91%//AF152492
C-HEMBA1006780
C-HEMBA1006807//Homo sapiens mRNA for SPOP.//5.70E-125//1109bp//75%//AJ000644
C-HEMBA1006824
C-HEMBA1006865
C-HEMBA1006921
C-HEMBA1006949
C-HEMBA1006976//H.sapiens mRNA for Gal-beta(1-3/1-4)GlcNAc alpha-2.3-sialyltransferase.//1.90E-80//447bp//89%//X74570
C-HEMBA1007051
C-HEMBA1007052
C-HEMBA1007066
C-HEMBA1007073
C-HEMBA1007078
C-HEMBA1007085
C-HEMBA1007113
C-HEMBA1007121//Homo sapiens bisphosphate 3'-nucleotidase mRNA, complete cds.//1.70E-252//1118bp//92%//AF125042
C-HEMBA1007129
C-HEMBA1007147
C-HEMBA1007151//Homo sapiens synphilin 1 mRNA, complete cds.//0//1900bp//99%//AF076929
C-HEMBA1007178
C-HEMBA1007203//Homo sapiens mRNA for KIAA0214 protein, complete cds.//0//1212bp//98%//D86987
C-HEMBA1007224//Homo sapiens SUMO-1-specific protease (SSP1) mRNA, complete cds.//0//1590bp//99%//AF196304
C-HEMBA1007243//Chinese hamster hprt mRNA, complete cds.//2.00E-58//650bp//70%//J00060
C-HEMBA1007251
C-HEMBA1007288
C-HEMBA1007322
C-HEMBA1007341
C-HEMBB1000050
C-HEMBB1000054
C-HEMBB1000059
C-HEMBB1000089
C-HEMBB1000113
C-HEMBB1000144//GUANYLATE CYCLASE ACTIVATING PROTEIN 2 (GCAP 2) (RETINAL GUANYLYL CYCLASE ACTIVATOR PROTEIN P24).//1.40E-24//71aa//77%//P51177
C-HEMBB1000173
C-HEMBB1000175
C-HEMBB1000272
C-HEMBB1000317//FIBULIN-1, ISOFORM D PRECURSOR.//7.10E-62//458aa//35%//P37888
C-HEMBB1000318
C-HEMBB1000336
C-HEMBB1000341
C-HEMBB1000343
C-HEMBB1000354
C-HEMBB1000374
C-HEMBB1000434
C-HEMBB1000441
C-HEMBB1000491
C-HEMBB1000493
C-HEMBB1000510
C-HEMBB1000652
C-HEMBB1000672
C-HEMBB1000684
C-HEMBB1000709
C-HEMBB1000726
C-HEMBB1000770
C-HEMBB1000827
C-HEMBB1000831
C-HEMBB1000883
C-HEMBB1000888
C-HEMBB1000893
C-HEMBB1000913
C-HEMBB1000996
C-HEMBB1001004
C-HEMBB1001047
C-HEMBB1001060
C-HEMBB1001114
C-HEMBB1001119
C-HEMBB1001133
C-HEMBB1001142
C-HEMBB1001177
C-HEMBB1001208
C-HEMBB1001209
C-HEMBB1001249
C-HEMBB1001253
C-HEMBB1001254
C-HEMBB1001271
C-HEMBB1001304
C-HEMBB1001317
C-HEMBB1001348
C-HEMBB1001394
C-HEMBB1001410
C-HEMBB1001424
C-HEMBB1001426
C-HEMBB1001429//Homo sapiens leucine aminopeptidase mRNA, complete cds.//0//1933bp//99%//AF061738
C-HEMBB1001436
C-HEMBB10014437/Rattus norvegicus pyruvate dehydrogenase phosphatase isoenzyme 1 mRNA, complete cds.//3.00E-130//553bp//86%//AF062740
C-HEMBB1001449
C-HEMBB1001458
C-HEMBB1001521
C-HEMBB1001531
C-HEMBB1001535
C-HEMBB1001536
C-HEMBB1001564
C-HEMBB1001565
C-HEMBB1001585
C-HEMBB1001588
C-HEMBB1001603
C-HEMBB1001618
C-HEMBB1001635
C-HEMBB1001653
C-HEMBB1001668
C-HEMBB1001673//Homo sapiens mRNA for KIAA0646 protein, complete cds.//0//2035bp//99%//AB014546
C-HEMBB1001685
C-HEMBB1001695
C-HEMBB1001707
C-HEMBB1001735
C-HEMBB1001736//EUKARYOTIC TRANSLATION INITIATION FACTOR 3 SUBUNIT 9 (EIF3 P116) (EIF3 P110).//4.60E-15//391aa//25%//P55884
C-HEMBB1001747
C-HEMBB1001749//TRANSCRIPTIONAL ACTIVATOR GCN5.//1.70E-16//84aa//47%//Q03330
C-HEMBB1001753
C-HEMBB1001756
C-HEMBB1001760
C-HEMBB1001785
C-HEMBB1001797
C-HEMBB1001802//Human desmin mRNA, complete cds.//0//1523bp//98%//U59167
C-HEMBB1001816
C-HEMBB1001831//Homo sapiens PAM COOH-terminal interactor protein 1 (PCIP1) mRNA complete cds.//0//1514bp//99%//AF056209
C-HEMBB1001839//GASTRULA ZINC FINGER PROTEIN XLCGF42.1 (FRAGMENT).//6.90E-11//87aa//35%//P18720
C-HEMBB1001850
C-HEMBB1001863
C-HEMBB1001868
C-HEMBB1001874
C-HEMBB1001880
C-HEMBB1001899
C-HEMBB1001906
C-HEMBB1001910
C-HEMBB1001911
C-HEMBB1001921
C-HEMBB1001922
C-HEMBB1001930
C-HEMBB1001944
C-HEMBB1001945
C-HEMBB1001947
C-HEMBB1001950//PROBABLE OXYGEN-INDEPENDENT COPROPORPHYRINOGEN III OXIDASE (EC 1.-.-.-) (COPROPORPHYRINOGENASE) (COPROGEN OXIDASE).//1.60E-41//370aa//31%//P54304
C-HEMBB1001952
C-HEMBB1001957
C-HEMBB1001962
C-HEMBB1001983
C-HEMBB1001990
C-HEMBB1001996
C-HEMBB1002002
C-HEMBB1002005
C-HEMBB1002042//CYTOCHROME P450 4C1 (EC 1.14.14.1) (CYPIVC1).//2.70E-49//139aa//55%//P29981
C-HEMBB1002043
C-HEMBB1002045
C-HEMBB1002049
C-HEMBB1002050
C-HEMBB1002068
C-HEMBB1002092
C-HEMBB1002139
C-HEMBB1002142
C-HEMBB1002190
C-HEMBB1002193
C-HEMBB1002217//ZINC FINGER PROTEIN 91 (ZINC FINGER PROTEIN HTF10) (HPF7).//2.10E-132//399aa//44%//Q05481
C-HEMBB1002218
C-HEMBB1002232
C-HEMBB1002247
C-HEMBB1002249
C-HEMBB1002266//NEURONAL PROTEIN.//2.10E-46//121aa//76%//P41737
C-HEMBB1002327
C-HEMBB1002329
C-HEMBB1002342//Homo sapiens mRNA for putative thioredoxin-like protein.//1.10E-274//1249bp//99%//AJ010841
C-HEMBB1002358
C-HEMBB1002371
C-HEMBB1002387
C-HEMBB1002409
C-HEMBB1002425
C-HEMBB1002442//LIN-10 PROTEIN.//9.70E-14//121aa//31%//P34692
C-HEMBB1002453
C-HEMBB1002458
C-HEMBB1002477//Human Grb2-associated binder-1 mRNA, complete cds.//7.70E-258//774bp//99%//U43885
C-HEMBB1002489
C-HEMBB1002510//GYP7 PROTEIN.//3.10E-50//192aa//42%//P48365
C-HEMBB1002520
C-HEMBB1002522
C-HEMBB1002545
C-HEMBB1002579
C-HEMBB1002582
C-HEMBB1002596
C-HEMBB1002603
C-HEMBB1002610
C-HEMBB1002613
C-HEMBB1002617
C-HEMBB1002623
C-HEMBB1002635
C-HEMBB1002677
C-HEMBB1002683
C-HEMBB1002699
C-HEMBB1002702
C-MAMMA1000009
C-MAMMA1000043
C-MAMMA1000045//ENV POLYPROTEIN [CONTAINS: SURFACE PROTEIN GP85; MEMBRANE PROTEIN GP37].//1.90E-07//249aa//27%//P03396
C-MAMMA1000057
C-MAMMA1000085//PUTATIVE CYSTEINYL-TRNA SYNTHETASE C29E6.06C (EC 6.1.1.16) (CYSTEINE-- TRNA LIGASE) (CYSRS).//2.10E-90//427aa//39%//Q09860
C-MAMMA1000092
C-MAMMA1000103
C-MAMMA1000117
C-MAMMA1000129
C-MAMMA1000133
C-MAMMA1000155
C-MAMMA1000175
C-MAMMA1000198
C-MAMMA1000241
C-MAMMA1000251
C-MAMMA1000254
C-MAMMA1000287
C-MAMMA1000307
C-MAMMA1000331
C-MAMMA1000339
C-MAMMA1000340
C-MAMMA1000348
C-MAMMA1000356
C-MAMMA1000360
C-MAMMA1000402
C-MAMMA1000414
C-MAMMA1000431
C-MAMMA1000444
C-MAMMA1000458
C-MAMMA1000500
C-MAMMA1000522
C-MAMMA1000576
C-MAMMA1000583
C-MAMMA1000594
C-MAMMA1000605
C-MAMMA1000616
C-MAMMA1000643
C-MAMMA1000684//Homo sapiens 7-60 mRNA, complete cds.//0//2402bp//99%//AF109134
C-MAMMA1000696
C-MAMMA1000707
C-MAMMA1000714
C-MAMMA1000720
C-MAMMA1000744
C-MAMMA1000761
C-MAMMA1000776
C-MAMMA1000798
C-MAMMA1000839
C-MAMMA1000851
C-MAMMA1000863
C-MAMMA1000867
C-MAMMA1000876
C-MAMMA1000880
C-MAMMA1000883
C-MAMMA1000921
C-MAMMA1000931
C-MAMMA1000941
C-MAMMA1000957
C-MAMMA1000962
C-MAMMA1000975
C-MAMMA1000987
C-MAMMA1001003
C-MAMMA1001030//LUTROPIN-CHORIOGONADOTROPIC HORMONE RECEPTOR (LH/CG-R) (LSH-R) (LUTEINIZING HOROMINE RECEPTOR) (FRAGMENT).//1.20E-26//276aa//28%//Q90674
C-MAMMA1001038//MYOSIN LIGHT CHAIN KINASE, SMOOTH MUSCLE AND NON-MUSCLE ISOZYMES (EC 2.7.1.117) (MLCK) [CONTAINS: TELOKIN].//2.60E-107//190aa//95%//Q15746
C-MAMMA1001082
C-MAMMA1001162
C-MAMMA1001186
C-MAMMA1001191
C-MAMMA1001206
C-MAMMA1001220
C-MAMMA1001243
C-MAMMA1001249
C-MAMMA1001256
C-MAMMA1001268
C-MAMMA1001271
C-MAMMA1001274
C-MAMMA1001292
C-MAMMA1001305//RHO-GTPASE-ACTIVATING PROTEIN 1 (GTPASE-ACTIVATING PROTEIN RHOGAP) (RHO-RELATED SMALL GTPASE PROTEIN ACTIVATOR) (CDC42 GTPASE-ACTIVATING PROTEIN) (P50-RHOGAP).//2.20E-98//283aa//63%//Q07960
C-MAMMA1001324
C-MAMMA1001341
C-MAMMA1001388//LEUCINE-RICH ALPHA-2-GLYCOPROTEIN (LRG).//1.40E-165//312aa//99%//P02750
C-MAMMA1001397
C-MAMMA1001408
C-MAMMA1001420
C-MAMMA1001442
C-MAMMA1001452
C-MAMMA1001465
C-MAMMA1001487
C-MAMMA1001501//CALPAIN 1, LARGE [CATALYTIC] SUBUNIT (EC 3.4.22.17) (CALCIUM-ACTIVATED NEUTRAL PROTEINASE) (CANP) (MU-TYPE).//5.70E-55//86aa//97%//P07384
C-MAMMA1001547
C-MAMMA1001551
C-MAMMA1001575
C-MAMMA1001590
C-MAMMA1001600
C-MAMMA1001606
C-MAMMA1001627//Homo sapiens mRNA for transcription factor TBX6.//5.20E-189//871bp//99%//AJ007989
C-MAMMA1001663
C-MAMMA1001670
C-MAMMA1001671
C-MAMMA1001679//F-ACTIN CAPPING PROTEIN BETA SUBUNIT (CAPZ).//0.00000058//29aa//100%//P47756
C-MAMMA1001711
C-MAMMA1001735//TUBULIN BETA-5 CHAIN (BETA-TUBULIN CLASS-V).//5.90E-240//445aa//97%//P09653
C-MAMMA1001744
C-MAMMA1001745
C-MAMMA1001751//Homo sapiens tandem pore domain potassium channel TWIK-2 (KCNK6) mRNA, complete cds.//0//2332bp//99%//AF117708
C-MAMMA1001783
C-MAMMA1001788
C-MAMMA1001806
C-MAMMA1001812
C-MAMMA1001815
C-MAMMA1001817
C-MAMMA1001818
C-MAMMA1001820//Rattus norvegicus mRNA for PAG608 gene.//1.30E-198//1157bp//80%//Y13148
C-MAMMA1001824
C-MAMMA1001851
C-MAMMA1001854
C-MAMMA1001864
C-MAMMA1001878
C-MAMMA1001890
C-MAMMA1001907
C-MAMMA1001908
C-MAMMA1001931
C-MAMMA1001969
C-MAMMA1002011
C-MAMMA1002032
C-MAMMA1002041
C-MAMMA1002047
C-MAMMA1002056
C-MAMMA1002058
C-MAMMA1002078
C-MAMMA1002082
C-MAMMA1002084
C-MAMMA1002093
C-MAMMA1002094
C-MAMMA1002118
C-MAMMA1002125
C-MAMMA1002132
C-MAMMA1002140
C-MAMMA1002143//Homo sapiens Cdc42 effector protein 4 mRNA, complete cds7/1.70E-252//1170bp//99%//AF099664
C-MAMMA1002145
C-MAMMA1002198//THIOREDOXIN PEROXIDASE 1 (THIOREDOXIN-DEPENDENT PEROXIDE REDUCTASE 1) (THIOL-SPECIFIC ANTIOXIDANT PROTEIN) (TSA) (PRP) (NATURAL KILLER CELL ENHANCING FACTOR B) (NKEF-B).//5.20E-61//60aa//90%//P32119
C-MAMMA1002230
C-MAMMA1002250
C-MAMMA1002282
C-MAMMA1002293
C-MAMMA1002298
C-MAMMA1002299
C-MAMMA1002308
C-MAMMA1002310
C-MAMMA1002311
C-MAMMA1002322
C-MAMMA1002339
C-MAMMA1002352
C-MAMMA1002359
C-MAMMA1002360
C-MAMMA1002392
C-MAMMA1002411
C-MAMMA1002413
C-MAMMA1002417
C-MAMMA1002428//LYSOSOME MEMBRANE PROTEIN II (LIMP II) (85 KD LYSOSOMAL MEMBRANE SIALOGLYCOPROTEIN) (LGP85) (CD36 ANTIGEN-LIKE 2).//1.10E-24//96aa//68%//Q14108
C-MAMMA1002434
C-MAMMA1002446
C-MAMMA1002454
C-MAMMA1002461
C-MAMMA1002475
C-MAMMA1002556
C-MAMMA1002566
C-MAMMA1002612
C-MAMMA1002622//VILLIN.//7.20E-35//53aa//64%//P02640
C-MAMMA1002637//KINESIN LIGHT CHAIN (KLC).//1.30E-198//550aa//70%//Q07866
C-MAMMA1002650//Mus musculus ODA-8S protein mRNA, complete cds.//5.40E-57//480bp//68%//AF194030
C-MAMMA1002699//Rattus norvegicus EH domain binding protein Epsin mRNA, complete cds.//4.3e-317//1942bp//85%//AF018261
C-MAMMA1002727
C-MAMMA1002748
C-MAMMA1002758
C-MAMMA1002780
C-MAMMA1002820
C-MAMMA1002833
C-MAMMA1002843
C-MAMMA1002895
C-MAMMA1002937//ZINC FINGER PROTEIN 135.//8.30E-99//393aa//43%//P52742
C-MAMMA1003004
C-MAMMA1003047//Homo sapiens protein inhibitor of activated STAT protein PIASy mRNA, complete cds.//0//1533bp//99%//AF077952
C-NT2RM1000001//D.melanogaster sap47-2 mRNA.//1.50E-10//417bp//62%//X80110
C-NT2RM1000018//Human mRNA for KIAA0066 gene, partial cds.//0//3376bp//99%//D31886
C-NT2RM1000037//Homo sapiens mRNA for KIAA0690 protein, partial cds.//0//3551bp//99%//AB014590
C-NT2RM1000086//Homo sapiens mRNA for KIAA0661 protein, complete cds.//0//3035bp//96%//AB014561
C-NT2RM1000421//RIBONUCLEASE INHIBITOR.//4.40E-21//372aa//30%//P10775 C-NT2RM1000499
C-NT2RM1001059//NUCLEAR POLYADENYLATED RNA-BINDING PROTEIN NAB4.//3.60E-11//180aa//28%//Q99383
C-NT2RM1001092//ZINC FINGER PROTEIN 91 (ZINC FINGER PROTEIN HTF10) (HPF7).//3.60E-115//332aa//52%//Q05481
C-NT2RM2001592//Homo sapiens mRNA for KIAA1067 protein, partial cds.//0//3471bp//99%//AB028990
C-NT2RM2001635//Homo sapiens mRNA for KIAA0618 protein, complete cds.//0//1632bp//99%//AB014518
C-NT2RM2001637
C-NT2RM2001641
C-NT2RM2001670//ZINC FINGER PROTEIN 29 (ZFP-29).//6.50E-104//407aa//43%//Q07230
C-NT2RM2001699
C-NT2RM2001706
C-NT2RM2001718
C-NT2RM2001727//Homo sapiens mRNA for KIAA0462 protein, partial cds.//0//2892bp//99%//AB007931
C-NT2RM2001805
C-NT2RM4000086
C-NT2RM4000215//MAK16 PROTEIN.//1.30E-68//295aa//49%//P10962
C-NT2RM4000414
C-NT2RM4000590//RING CANAL PROTEIN (KELCH PROTEIN).//1.00E-59//595aa//28%//Q04652
C-NT2RM4000634
C-NT2RM4000657//Homo sapiens mRNA for KIAA1069 protein, partial cds.//0//1412bp//100%//AB028992
C-NT2RM4000783
C-NT2RM4000857//LEUCINE-RICH ALPHA-2-GLYCOPROTEIN (LRG).//6.70E-22//250aa//29%//P02750
C-NT2RM4000971
C-NT2RM4000996//ZINC FINGER PROTEIN 91 (ZINC FINGER PROTEIN HTF10) (HPF7).//8.00E-211//738aa//50%//Q05481
C-NT2RM4001092//ZINC FINGER PROTEIN GLO3.//3.10E-24//265aa//33%//P38682
C-NT2RM4001178//PROBABLE ATP-DEPENDENT RNA HELICASE HAS1.//1.10E-48//218aa//43%//Q03532
C-NT2RM4001569
C-NT2RM4001819//Human p58/GTA (galactosyltransferase associated protein kinase) mRNA, complete cds.//8.10E-300//1395bp//98%//M37712
C-NT2RM4001905
C-NT2RM4001938//Homo sapiens mRNA for KIAA0898 protein, partial cds.//0//2234bp//99%//AB020705
C-NT2RM4002062//ASPARTYL-TRNA SYNTHETASE (EC 6.1.1.12) (ASPARTATE--TRNA LIGASE) (ASPRS).//1.90E-31//80aa//52%//P36419
C-NT2RM4002073//Mus musculus fatty acid transport protein 3 mRNA, partial cds.//9.30E-293//1751bp//83%//AF072758
C-NT2RM4002093//Homo sapiens neural polypyrimidine tract binding protein (PTB) mRNA, complete cds.//0//2550bp//99%//AF176085
C-NT2RM4002109//Homo sapiens kinesin superfamily motor KIF4 mRNA, complete cds.//0//2572bp//99%//AF071592
C-NT2RM4002146//Homo sapiens MAGOH mRNA, complete cds.//6.90E-70//454bp//85%//AF035940
C-NT2RM4002194//Mus musculus semaphorin VIa mRNA, complete cds.//5.20E-297//1753bp//87%//AF030430
C-NT2RM4002390
C-NT2RM4002398
C-NT2RM4002420
C-NT2RM4002534
C-NT2RM4002565//Mus musculus Sec8 mRNA, complete cds.//0//1915bp//87%//AF022962
C-NT2RM4002571//H.sapiens mRNA for UDP-GalNAc:polypeptide N-acetylgalactosaminyltransferase (T2).//4.60E-78//921bp//69%//X85019
C-NT2RP1000358//Homo sapiens mRNA; cDNA DKFZp564C186 (from clone DKFZp564C186).//0//1938bp//88%//AL050019
C-NT2RP1000522//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//8.20E-83//345aa//47%//Q61068
C-NT2RP1000609//Homo sapiens mRNA; cDNA DKFZp586C201 (from clone DKFZp586C201).//0//2165bp//99%//AL050118
C-NT2RP1000677//SODIUM-INDEPENDENT ORGANIC ANION TRANSPORTER (ORGANIC ANION TRANSPORTING POLYPEPTIDE).//1.20E-78//483aa//31%//P46721
C-NT2RP1000701//Homo sapiens phospholipase A2 activating protein (PLA2P) mRNA, complete cds.//0//1687bp//99%//AF145020
C-NT2RP1000834//Homo sapiens alpha-methylacyl-CoA racemase mRNA, complete cds.//1.80E-176//829bp//98%//AF047020
C-NT2RP1000860//Homo sapiens KL04P mRNA, complete cds.//0//1555bp//99%//AF064094
C-NT2RP1000916
C-NT2RP1000944
C-NT2RP1001079//Oryctolagus cuniculus sarcosine oxidase (SOX) mRNA, complete cds.//0//2085bp//99%//U82267
C-NT2RP1001080//PROBABLE ATP-DEPENDENT RNA HELICASE DBP9.//2.30E-116//319aa//46%//Q06218
C-NT2RP1001113
C-NT2RP1001173//Homo sapiens mRNA; cDNA DKFZp566D1146 (from clone DKFZp566D1146).//0//2333bp//99%//AL080222
C-NT2RP1001177//Rattus norvegicus histone macroH2A1.2 mRNA, complete cds.//5.20E-108//1278bp//69%//U79139
C-NT2RP1001185//Human isovaleryl-coA dehydrogenase (IVD) mRNA, complete cds.//1.90E-158//729bp//99%//M34192
C-NT2RP1001247//Homo sapiens TGF-beta type secreted signaling protein LEFTYA mRNA, complete cds.//0//2006bp//100%//AF081513
C-NT2RP1001311
C-NT2RP1001313//Homo sapiens delta-6 fatty acid desaturase mRNA, complete cds.//7.50E-121//1394bp//69%//AF126799
C-NT2RP2000001//Homo sapiens mRNA for KIAA1111 protein, partial cds.//0//3188bp//99%//AB029034
C-NT2RP2000027
C-NT2RP2000183//DIHYDROPYRIMIDINASE RELATED PROTEIN-2 (DRP-2) (NEURAL SPECIFIC PROTEIN NSP60).//3.30E-16//114aa//44%//002675
C-NT2RP2000198
C-NT2RP2000523//APOLIPOPROTEIN B MRNA EDITING PROTEIN (HEPR) (APOBEC-1).//6.00E-16//124aa//34%//P41238
C-NT2RP2000551
C-NT2RP2000644
C-NT2RP2000660//SAP1 PROTEIN.//5.20E-68//474aa//32%//P39955
C-NT2RP2000678
C-NT2RP2000715
C-NT2RP2000842//Human lysophosphatidic acid receptor homolog mRNA, complete cds.//0//1562bp//99%//U80811
C-NT2RP2000970
C-NT2RP2001347
C-NT2RP2001460//TRICHOHYALIN.//1.00E-14//521aa//24%//P37709
C-NT2RP2001613//MITOCHONDRIAL IMPORT RECEPTOR SUBUNIT TOM40 (MOM38 PROTEIN) (TRANSLOCASE OF OUTER MEMBRANE 40 KD SUBUNTD.//6.10E-12//184aa//31%//P24391
C-NT2RP2001634//Homo sapiens alpha-catenin-like protein mRNA, complete cds.//0//2445bp//99%//U97067
C-NT2RP2001660//Homo sapiens putative 13 S Golgi transport complex 90kD subunit brain-specific isoform mRNA, complete cds.//0//1287bp//99%//AF058718
C-NT2RP2001677
C-NT2RP2001678
C-NT2RP2001720
C-NT2RP2001740//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//7.90E-52//220aa//44%//Q61068
C-NT2RP2001756//ZINC FINGER PROTEIN 84 (ZINC FINGER PROTEIN HPF2).//1.70E-49//411aa//32%//P51523
C-NT2RP2001839//SCY1 PROTEIN.//5.40E-32//621aa//24%//P53009
C-NT2RP2001861
C-NT2RP2001869//ZINC FINGER PROTEIN 191.//7.10E-26//126aa//52%//014754
C-NT2RP2001876//ALLOGRAFT INFLAMMATORY FACTOR-1 (AIF-1) (IONIZED CALCIUM BINDING ADAPTER MOLECULE 1).//1.20E-45//141aa//65%//P55008
C-NT2RP2001898//Human inositol polyphosphate 5-phosphatase (5ptase) mRNA, 3' end.//0//2518bp//98%//M74161
C-NT2RP2001936
C-NT2RP2001943
C-NT2RP2001946
C-NT2RP2002032
C-NT2RP2002033
C-NT2RP2002041
C-NT2RP2002047
C-NT2RP2002066//Rattus norvegicus transmembrane receptor Unc5H2 mRNA, complete cds.//1.60E-226//1301bp//88%//U87306
C-NT2RP2002124//Homo sapiens mRNA for KIAA1097 protein, partial cds.//0//1772bp//95%//AB029020
C-NT2RP2002172
C-NT2RP2002219
C-NT2RP2002256//Homo sapiens retinoic acid hydroxylase mRNA, complete cds.//0//1528bp//98%//AF005418
C-NT2RP2002316
C-NT2RP2002373
C-NT2RP2002439
C-NT2RP2002475
C-NT2RP2002546
C-NT2RP2002591//ZINC FINGER PROTEIN 84 (ZINC FINGER PROTEIN HPF2).//1.20E-155//562aa//50%//P51523
C-NT2RP2002606//Rattus norvegicus Rabin3 mRNA, complete cds.//9.20E-147//874bp//87%//U19181
C-NT2RP2002643
C-NT2RP2002727//Rattus norvegicus tulip 2 mRNA, complete cds.//3.50E-74//727bp//72%//AF041107
C-NT2RP2002736
C-NT2RP2002740
C-NT2RP2002741//Homo sapiens mRNA for Neuroblastoma, complete cds.//9.90E-54//964bp//64%//D89016
C-NT2RP2002752
C-NT2RP2002753
C-NT2RP2002857
C-NT2RP2003000//TUMOR NECROSIS FACTOR, ALPHA-INDUCED PROTEIN 1, ENDOTHELIAL (B12 PROTEIN).//1.90E-11//132aa//38%//Q13829
C-NT2RP2003073
C-NT2RP2003164//Homo sapiens mRNA for protein kinase.//0//2313bp//99%//AJ132545
C-NT2RP2003206
C-NT2RP2003228//H.sapiens P1-Cdc21 mRNA.//0//2870bp//98%//X74794
C-NT2RP2003230//Rattus norvegicus endo-alpha-D-mannosidase (Enman) mRNA, complete cds.//2.60E-186//1551bp//77%//AF023657
C-NT2RP2003237
C-NT2RP2003272//Homo sapiens ubiquilin mRNA, complete cds.//0//1789bp//99%//AF176069
C-NT2RP2003280
C-NT2RP2003293
C-NT2RP2003394//INTRACELLULAR PROTEIN TRANSPORT PROTEIN USO1.//5.50E-13//302aa//26%//P25386
C-NT2RP2003401//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//9.60E-78//346aa//43%//061068
C-NT2RP2003456
C-NT2RP2003517//Human c-sis/platelet-derived growth factor 2 (SIS/PDGF2) mRNA, complete cds.//0//1746bp//95%//M12783
C-NT2RP2003522//Homo sapiens zinc finger DNA binding protein 99 (ZNF281) mRNA, complete cds.//0//1764bp//99%//AF125158
C-NT2RP2003559
C-NT2RP2003564//52 KD RO PROTEIN (SJOGREN SYNDROME TYPE A ANTIGEN (SS-A)) (RO(SS-A)).//2.10E-59//270aa//46%//P19474
C-NT2RP2003581
C-NT2RP2003643//Mus musculus mRNA for CMP-N-acetylneuraminic acid synthetase.//9.40E-243//1624bp//82%//AJ006215
C-NT2RP2003702//Homo sapiens 17 beta-hydroxysteroid dehydrogenase type VII (HSD17B7) mRNA, complete cds.//2.1e-313//978bp//99%//AF098786
C-NT2RP2003704//Homo sapiens mRNA for ATP-dependent metalloprotease YME1L.//1.80E-72//350bp//100%//AJ132637
C-NT2RP2003727
C-NT2RP2003751
C-NT2RP2003781//HYPOTHETICAL 36.7 KD PROTEIN AH6.2 IN CHROMOSOME II.//5.50E-63//253aa//50%//Q09201
C-NT2RP2003825
C-NT2RP2003871
C-NT2RP2003885
C-NT2RP2003912//SERINE/THREONINE-PROTEIN KINASE NEK1 (EC 2.7.1.-) (NIMA-RELATED PROTEIN KINASE 1).//6.10E-183//387aa//87%//P51954
C-NT2RP2003976//Homo sapiens mRNA for KIAA0447 protein, complete cds.//0//2866bp//98%//AB007916
C-NT2RP2003988
C-NT2RP2004013//TRANSCRIPTION FACTOR BTF3 (RNA POLYMERASE B TRANSCRIPTION FACTOR 3).//2.30E-53//141aa//78%//P20290
C-NT2RP2004098//ADENYLATE CYCLASE (EC 4.6.1.1) (ATP PYROPHOSPHATE-LYASE) (ADENYLYL CYCLASE).//5.40E-30//319aa//31%//Q01513
C-NT2RP2004142
C-NT2RP2004170//Homo sapiens mRNA for transducin (beta) like 1 protein.//1.10E-138//1236bp//74%//Y12781
C-NT2RP2004194//Rattus norvegicus Golgi SNARE GS15 mRNA, complete cds.//3.80E-52//397bp//82%//AF003998
C-NT2RP2004207
C-NT2RP2004226
C-NT2RP2004232//Homo sapiens EPK2 mRNA for serine/threonine kinase, complete cds.//0//2272bp//99%//AB015982
C-NT2RP2004242//NEUROFILAMENT TRIPLET H PROTEIN (200 KD NEUROFILAMENT PROTEIN) (NF-H).//9.90E-12//427aa//26%//P19246
C-NT2RP2004270//PROTEIN PTM1 PRECURSOR.//1.40E-16//334aa//24%//P32857
C-NT2RP2004321
C-NT2RP2004339
C-NT2RP2004347
C-NT2RP2004396//Homo sapiens mRNA for activator of S phase Kinase, complete cds.//5.40E-243//1108bp//99%//AB028069
C-NT2RP2004399
C-NT2RP2004400
C-NT2RP2004412
C-NT2RP2004425//Mus musculus axotrophin mRNA, complete cds.//0//2321bp//86%//AF155739
C-NT2RP2004490
C-NT2RP2004523
C-NT2RP2004538//Mus musculus kinesin-like protein KIF1B (Kif1b) mRNA, complete cds.//0//1387bp//86%//AF090190
C-NT2RP2004580
C-NT2RP2004587//Homo sapiens mRNA for KIAA0888 protein, partial cds.//0//2886bp//100%//AB020695
C-NT2RP2004594
C-NT2RP2004681
C-NT2RP2004709
C-NT2RP2004710//Homo sapiens mRNA for KIAA1014 protein, partial cds.//0//2587bp//100%//AB023231
C-NT2RP2004732//Homo sapiens mRNA for KIAA0884 protein, partial cds.//0//1774bp//99%//AB020691
C-NT2RP2004767
C-NT2RP2004775
C-NT2RP2004961//Rattus norvegicus KRAB/zinc finger suppressor protein 1 (KS1) mRNA, complete cds.//1.00E-228//1666bp//75%//U56732
C-NT2RP2004962
C-NT2RP2004982
C-NT2RP2005003//52 KD RO PROTEIN (SJOGREN SYNDROME TYPE A ANTIGEN (SS-A)) (RO(SS-A)).//1.80E-99//376aa//43%//P19474
C-NT2RP2005018
C-NT2RP2005020
C-NT2RP2005022
C-NT2RP2005031
C-NT2RP2005116//Homo sapiens mRNA for KIAA0664 protein, partial cds.//0//4069bp//99%//AB014564
C-NT2RP2005139//2-5A-DEPENDENT RIBONUCLEASE (EC 3.1.26.-) (2-5A-DEPENDENT RNAASE) (RNASE L) (RIBONUCLEASE 4) (FRAGMENT).//0.000000022//139aa//35%//Q05921
C-NT2RP2005168//Homo sapiens mRNA for E1B-55kDa-associated protein.//0//2769bp//98%//AJ007509
C-NT2RP2005254
C-NT2RP2005325//Homo sapiens LIM-homeodomain protein HLHX2 (LHX2) mRNA, complete cds.//0//1643bp//99%//AF124735
C-NT2RP2005336//TRICHOHYALIN.//5.40E-10//545aa//22%//P37709
C-NT2RP2005344//PROBABLE CALCIUM-TRANSPORTING ATPASE 5 (EC 3.6.1.38).//2.10E-124//636aa//38%//P32660
C-NT2RP2005360
C-NT2RP2005407//OXYSTEROL-BINDING PROTEIN.//5.30E-63//410aa//40%//P22059
C-NT2RP2005454
C-NT2RP2005457//Homo sapiens NADH-ubiquinone oxidoreductase subunit B14.5B homolog mRNA, complete cds.//1.20E-130//608bp//99%//AF070652
C-NT2RP2005476//Human p190-B (pl90-B) mRNA, complete cds.//3.40E-108//668bp//88%//U17032
C-NT2RP2005491//PARAMYOSIN (PMY) (ANTIGEN B).//0.00000015//279aa//26%//P35418
C-NT2RP2005496//ZINC FINGER PROTEIN 135.//2.90E-146//398aa//59%//P52742
C-NT2RP2005501
C-NT2RP2005531//PROTEIN 4.1 (BAND 4.1) (P4.1).//5.50E-70//393aa//39%//P11171
C-NT2RP2005600//Homo sapiens mRNA for KIAA1020 protein, partial cds.//0//2554bp//99%//AB028943
C-NT2RP2005645
C-NT2RP2005694//X-LINKED RETINITIS PIGMENTOSA GTPASE REGULATOR.//2.60E-10//175aa//27%//Q92834
C-NT2RP2005701//ZINC-FINGER PROTEIN RFP (RET FINGER PROTEIN).//3.00E-63//323aa//39%//Q62158
C-NT2RP2005741
C-NT2RP2005806
C-NT2RP2005815
C-NT2RP2005841
C-NT2RP2005882
C-NT2RP2005942//POLY(A) POLYMERASE (EC 2.7.7.19) (PAP) (POLYNUCLEOTIDE ADENYLYLTRANSFERASE).//1.50E-67//388aa//44%//P25500
C-NT2RP2006103
C-NT2RP2006166
C-NT2RP2006258
C-NT2RP2006261
C-NT2RP2006321
C-NT2RP2006454
C-NT2RP2006598//Homo sapiens retinoid x receptor interacting protein mRNA, complete cds.//3.10E-295//1193bp//99%//AF113538
C-NT2RP3000046//MITOCHONDRIAL GTPASE MSS1 PRECURSOR.//4.60E-78//421aa//37%//P32559
C-NT2RP3000047//NPL4 PROTEIN.//1.10E-85//526aa//36%//P33755
C-NT2RP3000418
C-NT2RP3000439//HYPOTHETICAL 46.4 KD PROTEIN IN FFH-GRPE INTERGENIC REGION.//2.90E-1511319aa//26%//P37908
C-NT2RP3000487
C-NT2RP3000512//Human HOX2G mRNA from the Hox2 locus.//0//1934bp//99%//X16667
C-NT2RP3000526
C-NT2RP3000603//NEUROGENIC DIFFERENTIATION FACTOR 1.//3.70E-11//90aa//42%//Q13562
C-NT2RP3000605//Mus musculus mRNA for wizL, complete cds.//0//2232bp//82%//AB012265
C-NT2RP3000628
C-NT2RP3000739//ATROPHIN-1 (DENTATORUBRAL-PALLIDOLUYSIAN ATROPHY PROTEIN).//1.40E-24//155aa//37%//Q10149
C-NT2RP3000845//PUTATIVE SERINE/THREONINE-PROTEIN KINASE P78 (EC 2.7.1.).//8.30E-108//331aa//50%//P27448
C-NT2RP3000968//40S RIBOSOMAL PROTEIN S15A.//1.90E-46//73aa//98%//P39027
C-NT2RP3001057//ZINC FINGER PROTEIN 91 (ZINC FINGER PROTEIN HTF10) (HPF7).//9.00E-201//584aa//54%//Q05481
C-NT2RP3001113//INTRACELLULAR PROTEIN TRANSPORT PROTEIN USO1.//2.90E-11//631aa//23%//P25386
C-NT2RP3001245//Homo sapiens mRNA for KIAA0923 protein, complete cds.//0//2659bp//99%//AB023140
C-NT2RP3001253//NUF1 PROTEIN (SPINDLE POLY BODY SPACER PROTEIN SPC110).//1.70E-10//540aa//23%//P32380
C-NT2RP3001356
C-NT2RP3001383
C-NT2RP3001399//SSU72 PROTEIN.//1.30E-16//84aa//52%//P53538
C-NT2RP3001554//MICROTUBULE-ASSOCIATED PROTEIN 1B [CONTAINS: LIGHT CHAIN LC1].//1.40E-76//388aa//32%//P46821
C-NT2RP3001712//Homo sapiens HP1-BP74 protein mRNA, complete cds.//0//1788bp//99%//AF113534
C-NT2RP3001724//Homo sapiens chromodomain-helicase-DNA-binding protein mRNA, complete cds.//1.10E-240//902bp//99%//AF054177
C-NT2RP3001727//Rattus norvegicus implantation-associated protein (IAG2) mRNA, partial cds.//6.90E-132//774bp//88%//AF008554
C-NT2RP3001730//SEPTIN 2 HOMOLOG (FRAGMENT).//7.10E-132//294aa//84%//Q14141
C-NT2RP3001739
C-NT2RP3001777
C-NT2RP3001857//VEGETATIBLE INCOMPATIBILITY PROTEIN HET-E-1.//1.20E-14//242aa//24%//Q00808
C-NT2RP3001943//Homo sapiens mRNA for KIAA0675 protein, complete cds.//0//3747bp//99%//AB014575
C-NT2RP3001944
C-NT2RP3002033
C-NT2RP3002054
C-NT2RP3002063//Homo sapiens mRNA for KIAA1033 protein, partial cds.//0//2830bp//99%//AB028956
C-NT2RP3002099
C-NT2RP3002102
C-NT2RP3002147
C-NT2RP3002163
C-NT2RP3002173
C-NT2RP3002255
C-NT2RP3002303//PROBABLE UNDECAPRENYL PYROPHOSPHATE SYNTHETASE (EC 2.5.1.31) (UPP SYNTHETASE) (DI-TRANS-POLY-CIS-DECAPRENYLCISTRANSFERASE).//8.60E-49//243aa//43%//Q58767
C-NT2RP3002343
C-NT2RP3002351//Human mRNA for NAD-dependent methylene tetrahydrofolate dehydrogenase cyclohydrolase (EC 1.5.1.15).//4.20E-70//590bp//76%//X16396
C-NT2RP3002399//DNA REPLICATION LICENSING FACTOR MCM4 (CDC21 HOMOLOG)(P1-CDC21).//8.60E-79//416aa//34%//P33991
C-NT2RP3002455//Homo sapiens mRNA for KIAA0678 protein, partial cds.//0//3811bp//99%//AB014578
C-NT2RP3002545//Homo sapiens mRNA; cDNA DKFZp586G0518 (from clone DKFZp586G0518).//0//2499bp//99%//AL050092
C-NT2RP3002549//HYPOTHETICAL 26.6 KD PROTEIN T19C3.4 IN CHROMOSOME III.//5.80E-40//161aa//52%//Q10010
C-NT2RP3002602//PROBABLE PROTEIN DISULFIDE ISOMERASE ER-60 PRECURSOR (EC 5.3.4.1) (ERP60) (58 KD MICROSOMAL PROTEIN) (P58) (HIP-70) (Q-2).//2.90E-19/173aa//28%//P11598
C-NT2RP3002603
C-NT2RP3002628//PROBABLE PROTEIN DISULFIDE ISOMERASE P5 PRECURSOR (EC 5.3.4.1).//2.50E-26//90aa//42%//P38660
C-NT2RP3002659
C-NT2RP3002660
C-NT2RP3002682//Homo sapiens CGI-145 protein mRNA, complete cds.//0//1596bp//98%//AF151903
C-NT2RP3002687
C-NT2RP3002688//Mouse mRNA for kinesin-like protein (Kifib), complete cds.//1.10E-93//1205bp//69%//D17577
C-NT2RP3002701
C-NT2RP3002785//LETHAL(2)DENTICLELESS PROTEIN (DTL83 PROTEIN).//2.50E-55//187aa//39%//Q24371
C-NT2RP3002869//Mus musculus semaphorin VIa mRNA, complete cds.//2.50E-232//1282bp//85%//AF030430
C-NT2RP3002876
C-NT2RP3002877
C-NT2RP3002909//Homo sapiens mRNA for KIAA0771 protein, partial cds.//0//2085bp//94%//AB018314
C-NT2RP3002969//Homo sapiens mRNA for Acyl-CoA synthetase 3, complete cds.//0//2722bp//99%//D89053
C-NT2RP3002972//Halocynthia roretzi mRNA for HrPET-1, complete cds.//3.90E-52//899bp//64%//AB029333
C-NT2RP3003032//Homo sapiens okadaic acid-inducible and cAMP-regulated phosphoprotein 19 (ARPP-19) mRNA, complete cds.//0//2656bp//99%//AF084555
C-NT2RP3003061//ANKYRIN.//1.40E-20//200aa//37%//Q02357
C-NT2RP3003071//NEUROGENIC PROTEIN BIG BRAIN.//1.10E-05//258aa//24%//P23645
C-NT2RP3003078
C-NT2RP3003139
C-NT2RP3003145//Mus musculus metallocarboxypeptidase CPX-1 mRNA, complete cds.//0//2251bp//81%//AF07773 8
C-NT2RP3003150
C-NT2RP3003197//HYPOTHETICAL 33.8 KD PROTEIN C5H10.01 IN CHROMOSOME I.//5.70E-09//169aa//31%//Q09674
C-NT2RP3003203//Rattus norvegicus golgi stacking protein homolog GRASP55 mRNA, complete cds.//2.00E-210//1851bp//76%//AF110267
C-NT2RP3003210
C-NT2RP3003212//Rattus norvegicus lamina associated polypeptide 1C (LAP1C) mRNA, complete cds.//4.30E-187//1750bp//75%//U20286
C-NT2RP3003230//Homo sapiens mRNA for hCRNN4, complete cds.//0//2350bp//99%//AB030656
C-NT2RP3003242//Homo sapiens stanniocalcin-related protein mRNA, complete cds.//0//2366bp//99%//AF098462
C-NT2RP3003251//52 KD RO PROTEIN (SJOGREN SYNDROME TYPE A ANTIGEN (SS-A))(RO(SS-A)).//4.20E-86//366aa//48%//P19474
C-NT2RP3003301//MITOCHONDRIAL LON PROTEASE HOMOLOG 1 PRECURSOR (EC 3.4.21.-).//1.10E-170//585aa//54%//064948
C-NT2RP3003311
C-NT2RP3003409//Human DHHC-domain-containing cysteine-rich protein mRNA, complete cds.//9.20E-45//782bp//65%//U90653
C-NT2RP3003427
C-NT2RP3003543
C-NT2RP3003552
C-NT2RP3003555//HYPOTHETICAL 32.6 KD PROTEIN IN MET30-PIG2 INTERGENIC REGION.//4.50E-30//191aa//40%//P40529
C-NT2RP3003564
C-NT2RP3003589//Homo sapiens ras-related GTP-binding protein mRNA, complete cds.//0//3131bp//94%//AF106681
C-NT2RP3003621
C-NT2RP3003625
C-NT2RP3003656
C-NT2RP3003659//HES1 PROTEIN.//5.90E-22//229aa//27%//P35843
C-NT2RP3003686
C-NT2RP3003701//F-SPONDIN PRECURSOR.//1.80E-17//324aa//26%//P35446
C-NT2RP3003716//SLIT PROTEIN PRECURSOR.//6.60E-10//150aa//34%//P24014
C-NT2RP3003726//Homo sapiens spermatogenesis associated PD1 mRNA, complete cds.//0//2568bp//99%//U28164
C-NT2RP3003795
C-NT2RP3003805
C-NT2RP3003809//SAV PROTEIN.//1.10E-131//576aa//41%//Q07590
C-NT2RP3003819
C-NT2RP3003825//PHOSPHATIDYLCHOLINE TRANSFER PROTEIN (PC-TP).//9.60E-19//174aa//31%//P02720
C-NT2RP3003831//Homo sapiens ENDOGL-1 (alias ENGL-a) mRNA for endonuclease G-like protein-1, complete cds.//2.2e-316//1436bp//99%//AB020523
C-NT2RP3003833
C-NT2RP3003842
C-NT2RP3003846//Homo sapiens mRNA for putative phospholipase, complete cds.//4.80E-277//1255bp//99%//AB019435
C-NT2RP3003870//Homo sapiens mRNA for KIAA0800 protein, complete cds.//0//2557bp//99%//AB018343
C-NT2RP3003876
C-NT2RP3003914//UDP-GLUCOSE:GLYCOPROTEIN GLUCOSYLTRANSFERASE PRECURSOR (EC 2.4.1.-) (DUGT).//2.20E-20//76aa//64%//Q09332
C-NT2RP3003918//Homo sapiens VAMP-associated protein B (VAP-B) mRNA, complete cds.//0//2191bp//99%//AF086628
C-NT2RP3003989
C-NT2RP3004016//TRANSCRIPTION INTERMEDIARY FACTOR 1-BETA (NUCLEAR COREPRESSOR KAP-1) (KRAB-ASSOCIATED PROTEIN 1).//1.50E-17//226aa//26%//Q13263
C-NT2RP3004070
C-NT2RP3004145
C-NT2RP3004215
C-NT2RP3004253
C-NT2RP3004282//Homo sapiens torsinA (DYT1) mRNA, complete cds.//5.10E-24//597bp//61 %//AF007871
C-NT2RP3004348//R.norvegicus mRNA for cytosolic resiniferatoxin-binding protein.//1.10E-185//1130bp//82%//X67877
C-NT2RP3004490//Homo sapiens PAC clone 166H1 from 12q, complete sequence.//0//1778bp//99%//AC003982
C-NT2RP3004503
C-NT2RP3004566//ZINC FINGER PROTEIN 84 (ZINC FINGER PROTEIN HPF2).//1.20E-95//434aa//43%//P51523
C-NT2RP3004670//Homo sapiens GN6ST mRNA for N-acetylglucosamine-6-O-sulfotransferas e (GlcNAc6ST), complete cds.//0//2393bp//99%//AB014679
C-NT2RP4000023
C-NT2RP4000218
C-NT2RP4000424
C-NT2RP4001213//ZINC FINGER PROTEIN 184 (FRAGMENT).//5.70E-141//511aa//43%//Q99676
C-NT2RP4001447
C-NT2RP4001841
C-NT2RP4001849//SH3-BINDING PROTEIN 3BP-1.//1.40E-85//489aa//43%//P55194
C-NT2RP4002047//GTP-BINDING PROTEIN LEPA.//1.50E-168//601aa//52%//067618
C-NT2RP4002075
C-NT2RP4002083
C-OVARC1000001/Homo sapiens mRNA for actin binding protein ABP620, complete cds.//7.00E-217//683bp//99%//AB029290
C-OVARC1000008
C-OVARC1000017
C-OVARC1000058
C-OVARC1000068
C-OVARC1000071//Homo sapiens NTF2-related export protein NXT1 (NXT1) mRNA, complete cds.//1.50E-47//727bp//67%//AF156957
C-OVARC1000085//Human mRNA for proteasome subunit HC5.//1.00E-151//699bp//100%//D00761
C-OVARC1000109
C-OVARC1000114
C-OVARC1000145
C-OVARC1000240
C-OVARC1000302
C-OVARC1000408
C-OVARC1000414
C-OVARC1000440
C-OVARC1000442
C-OVARC1000496
C-OVARC1000556//RIBOSOMAL PROTEIN S6 KINASE II ALPHA 2 (EC 2.7.1.-) (S6KII-ALPHA 2) (P90-RSK 2) (RIBOSOMAL S6 KINASE 3) (RSK3) (PP90RSK3).//3.30E-67//132aa//95%//015349
C-OVARC1000557
C-OVARC1000578
C-OVARC1000622
C-OVARC1000679//Homo sapiens myosin-IXa mRNA, complete cds.//0//808bp//99%//AF117888
C-OVARC1000681
C-OVARC1000700
C-OVARC1000724
C-OVARC1000751//PROBABLE PROTEIN PHOSPHATASE 2C T23F11.1 (EC 3.1.3.16) (PP2C).//5.60E-11//74aa//37%//P49596
C-OVARC1000800//MITOCHONDRIAL STRESS-70 PROTEIN PRECURSOR (75 KD GLUCOSE REGULATED PROTEIN) (GRP 75).//3.90E-46//78aa//98%//035501
C-OVARC1000885//OXIDOREDUCTASE UCPA (EC 1.-.-.-).//1.30E-32//170aa//34%//P37440
C-OVARC1000936//COAT PROTEIN GP37 (ENV PROTEIN GP37).//0.0000054//135aa//28%//P03398
C-OVARC1000937//S-PHASE ENTRY CYCLIN 6.//4.90E-10//61aaaa//49%//P32943
C-OVARC1000960
C-OVARC1000971
C-OVARC1000999//ANKYRIN HOMOLOG PRECURSOR.//4.10E-11//189aa//32%//Q06527
C-OVARC1001000
C-OVARC1001029
C-OVARC1001040
C-OVARC1001051//EPIDERMAL GROWTH FACTOR RECEPTOR SUBSTRATE SUBSTRATE 15 (PROTEIN EPS15) (AF-1P PROTEIN).//1.10E-08//216aa//23%//P42566
C-OVARC1001113//Homo sapiens diaphanous 1 (HDIA1) mRNA, complete cds.//5.1e-310//1588bp//93%//AF051782
C-OVARC1001118
C-OVARC1001129
C-OVARC1001169
C-OVARC1001240
C-OVARC1001261
C-OVARC1001339
C-OVARC1001342//40S RIBOSOMAL PROTEIN S8.//1.40E-110//207aa//99%//P09058
C-OVARC1001357
C-OVARC1001442
C-OVARC1001611
C-OVARC1001813
C-OVARC1002112//Homo sapiens histone macroH2A1.2 mRNA, complete cds.//0//1760bp//99%//AF054174
C-OVARC1002143
C-OVARC1002165//3-OXO-5-ALPHA-STEROID 4-DEHYDROGENASE 2 (EC 1.3.99.5) (STEROID 5-ALPHA-REDUCTASE 2) (SR TYPE 2).//7.60E-08//114aa//37%//P31213
C-OVARC1002182//BETA-TRCP (BETA-TRANSDUCIN REPEAT-CONTAINING PROTEIN) (BTRCP).//1.70E-09//207aa//30%//Q91854
C-PLACE1000014
C-PLACE1000078
C-PLACE1000492//Rat vacuolar protein sorting homolog r-vps33b mRNA, complete cds.//0//2041bp//87%//U35245
C-PLACE1000793//NEUROGENIC PROTEIN BIG BRAIN.//1.70E-07//251aa//24%//P23645
C-PLACE1000814
C-PLACE1000979//ZINC FINGER PROTEIN 135.//2.50E-153//326aa//64%//P52742
C-PLACE1001007
C-PLACE1001054//Homo sapiens mRNA for RuvB-like DNA helicase TIP49b, complete cds.//4.00E-300//1355bp//100%//AB024301
C-PLACE1001088
C-PLACE1001136
C-PLACE1001241
C-PLACE1001377//Homo sapiens ADAM10 (ADAM10) mRNA, complete cds.//5.90E-228//827bp//99%//AF009615
C-PLACE1001395
C-PLACE1001740
C-PLACE1001746
C-PLACE1001983//HYPOTHETICAL 46.4 KD PROTEIN IN FFH-GRPE INTERGENIC REGION.//7.50E-16//319aa//26%//P37908
C-PLACE1002066
C-PLACE1002115
C-PLACE1002213
C-PLACE1002342//Homo sapiens mRNA for KIAA0728 protein, partial cds.//0//1657bp//98%//AB018271
C-PLACE1002450//Human zinc finger protein mRNA, complete cds.//0//2565bp//99%//U69274
C-PLACE1002474//Mus musculus matrilin-2 precursor mRNA, complete cds.//0//2092bp//84%//U69262
C-PLACE1002499
C-PLACE1002578
C-PLACE1002714
C-PLACE1002772
C-PLACE1002775//PEREGRIN (BR140 PROTEIN).//3.80E-13//272aa//28%//P55201
C-PLACE1002834//ZINC FINGER PROTEIN 83 (ZINC FINGER PROTEIN HPF1).//5.50E-203//396aa//86%//P51522
C-PLACE1002993
C-PLACE1003030//Homo sapiens snRNA activating protein complex 190kD subunit (SNAP190) mRNA, complete cds.//8.50E-44//225bp//100%//AF032387
C-PLACE1003205
C-PLACE1003249
C-PLACE1003493//ENDOTHELIAL CELL MULTIMERIN PRECURSORS.//1.70E-23//594aa//33%//P28481
C-PLACE1003553
C-PLACE1003592
C-PLACE1003596//OLIGOSACCHARYL TRANSFERASE STT3 SUBUNIT HOMOLOG.//2.60E-93//270aa//66%//P46975
C-PLACE1003669//TRICHOHYALIN.//5.60E-09//219aa//30%//P22793
C-PLACE1003709//Homo sapiens mitotic checkpoint kinase Bub1 (BUB1) mRNA, complete cds.//6.20E-282//1316bp//98%//AF053305
C-PLACE1003870
C-PLACE1003885//POLY(A) POLYMERASE (EC 2.7.7.19) (PAP) (POLYNUCLEOTIDE ADENYLYLTRANSFERASE).//3.70E-222//651aa//66%//P25500
C-PLACE1003892
C-PLACE1003900
C-PLACE1004336
C-PLACE1004384
C-PLACE1004425
C-PLACE1004471//ZINC FINGER PROTEIN 83 (ZINC FINGER PROTEIN HPF1).//2.90E-56//276aa//41%//P51522
C-PLACE1004506//Homo sapiens carboxyl terminal LIM domain protein (CLIM1) mRNA, complete cds.//2.10E-16//402bp//62%//U90878
C-PLACE1004518
C-PLACE1004550//Homo sapiens CGI-20 protein mRNA, complete cds.//3.50E-274//1305bp//97%//AF132954
C-PLACE1004681
C-PLACE1004693
C-PLACE1004716//Homo sapiens HSPC038 protein mRNA, complete cds.//2.70E-103//586bp//91%//AF125099
C-PLACE1004815
C-PLACE1004836
C-PLACE1004838
C-PLACE1004840
C-PLACE1004900
C-PLACE1004985
C-PLACE1005085
C-PLACE1005086
C-PLACE1005108
C-PLACE1005146
C-PLACE1005409
C-PLACE1005453
C-PLACE1005477
C-PLACE1005557//60S RIBOSOMAL PROTEIN L27.//1.90E-11//60aa//48%//P46288
C-PLACE1005595
C-PLACE1005603
C-PLACE1005639
C-PLACE1005727//Homo sapiens STRIN protein (STRIN) mRNA, complete cds.//2.00E-118//378bp//98%//AF162680
C-PLACE1005799
C-PLACE1005813//Homo sapiens sorting nexin 2 (SNX2) mRNA, complete cds.//0//2040bp//99%//AF065482
C-PLACE1005884
C-PLACE1005968
C-PLACE1006002
C-PLACE1006003//Homo sapiens CGI-94 protein mRNA, complete cds.//2.40E-177//829bp//99%//AF151852
C-PLACE1006017
C-PLACE1006037
C-PLACE1006076
C-PLACE1006143
C-PLACE1006248//Homo sapiens mRNA for KIAA0648 protein, partial cds.//0//1489bp//100%//AB014548
C-PLACE1006288//VOLTAGE-DEPENDENT ANION-SELECTIVE CHANNEL PROTEIN 1 (VDAC1) (PLASMALEMMAL PORIN) (OUTER MITOCHONDRIAL MEMBRANE PROTEIN PORIN) (PORIN 31HL) (PORIN 31HM).//4.60E-117//147aa//80%//P21796
C-PLACE1006318//Mus musculus skm-BOP2 (Bop) mRNA, complete cds.//3.00E-07//376bp//59%//U76374
C-PLACE1006368//HYALURONAN-MEDIATED MOTILITY RECEPTOR (HYALURONIC ACID RECEPTOR).//1.30E-18//460aa//24%//Q00547
C-PLACE1006371
C-PLACE1006469//ACETYL-COENZYME A SYNTHETASE (EC 6.2.1.1) (ACETATE--COA LIGASE) (ACYL- ACTIVATING ENZYME).//1.20E-83//313aa//49%//P27550
C-PLACE1006506//Homo sapiens anaphase-promoting complex subunit 4 (APC4) mRNA, complete cds.//0//2170bp//99%//AF191338
C-PLACE1006521
C-PLACE1006534//Homo sapiens mRNA; cDNA DKFZp564G1964 (from clone DKFZp564G1964).//1.70E-192//883bp//99%//AL110144
C-PLACE1006617
C-PLACE1006640
C-PLACE1006754//BILIARY GLYCOPROTEIN 1 PRECURSOR (BGP-1) (ANTIGEN CD66) (CD66A ANTIGEN).//6.20E-63//191aa//43%//P13688
C-PLACE1006760
C-PLACE1006779
C-PLACE1006805
C-PLACE1006815
C-PLACE1006867
C-PLACE1007045
C-PLACE1007097
C-PLACE1007111
C-PLACE1007112
C-PLACE1007140//Homo sapiens mRNA for KIAA1009 protein, complete cds.//0//3492bp//99%//AB023226
C-PLACE1007218
C-PLACE1007454
C-PLACE1007478
C-PLACE1007677
C-PLACE10077057/Mus musculus mRNA for Ndr1 related protein Ndr3, complete cds.//1.10E-184//1096bp//82%//AB033922
C-PLACE1007737
C-PLACE1007743
C-PLACE1007852//Homo sapiens mRNA for KIAA0878 protein, complete cds.//1.00E-232//1174bp//94%//AB020685
C-PLACE1007877
C-PLACE1008045
C-PLACE1008080//Homo sapiens mRNA for HEXIM1 protein, complete cds.//0//2152bp//99%//AB021179
C-PLACE1008111//PROBABLE OXIDOREDUCTASE (EC 1.-.-.-).//3.00E-25//208aa//37%//Q03326
C-PLACE1008201//Rattus rattus zinc finger protein, complete cds.//0//2265bp//83%//L23077
C-PLACE1008231
C-PLACE1008244//VEGETATIBLE INCOMPATIBILITY PROTEIN HET-E-1.//9.50E-21//148aa//38%//Q00808
C-PLACE1008330//EOSINOPHIL LYSOPHOSPHOLIPASE (EC 3.1.1.5) (CHARCOT-LEYDEN CRYSTAL PROTEIN) (LYSOLECITHIN ACYLHYDROLASE) (CLC) (GALACTIN- 10).//2.20E-23//94aa//47%//Q05315
C-PLACE1008331
C-PLACE1008369
C-PLACE1008392
C-PLACE1008405
C-PLACE1008424
C-PLACE1008584
C-PLACE1008625
C-PLACE1008630
C-PLACE1008643//INTER-ALPHA-TRYPSIN INHIBITOR HEAVY CHAIN H2 PRECURSOR (ITI HEAVY CHAIN H2).//5.20E-90//483aa//38%//002668
C-PLACE1008715
C-PLACE1008748
C-PLACE1008757
C-PLACE1008798
C-PLACE1008851
C-PLACE1008947
C-PLACE1009039
C-PLACE1009048
C-PLACE1009050
C-PLACE10091137/Homo sapiens X-ray repair cross-complementing protein 3 (XRCC3) mRNA, complete cds.//0//2529bp//99%//AF035586
C-PLACE1009150
C-PLACE1009200
C-PLACE1009246//POLLEN SPECIFIC PROTEIN SF3.//4.40E-16//82aa//43%//P29675
C-PLACE1009298//Homo sapiens vacuolar sorting protein 35 (VPS35) mRNA, complete cds.//0//2262bp//99%//AF191298
C-PLACE1009308//GLUCOSE REPRESSION MEDIATOR PROTEIN.//4.00E-06//439aa//23%//P14922
C-PLACE1009398//ZINC FINGER PROTEIN 135.//6.20E-97//361aa//51%//P52742
C-PLACE1009410
C-PLACE1009477//Homo sapiens mRNA for KIAA0684 protein, partial cds.//6.50E-148//592bp//99%//AB014584
C-PLACE1009493
C-PLACE1009539
C-PLACE1009595
C-PLACE1009637
C-PLACE1009639
C-PLACE1009798//RLR1 PROTEIN.//1.60E-18//270aa//23%//P53552
C-PLACE1009861//CATHEPSIN B-LIKE CYSTEINE PROTEINASE 6 PRECURSOR (EC 3.4.22.-).//6.50E-28//209aa//38%//P43510
C-PLACE1009888
C-PLACE1009925//Homo sapiens RNA helicase (RIG-I) mRNA, complete cds.//0//1730bp//99%//AF038963
C-PLACE1009947
C-PLACE1010069
C-PLACE1010089//Homo sapiens mRNA for KIAA1097 protein, partial cds.//0//1554bp//100%//AB029020
C-PLACE1010231//CELL SURFACE GLYCOPROTEIN EMR1 PRECURSOR (EMR1 HORMONE RECEPTOR).//5.10E-27//371aa//28%//Q14246
C-PLACE1010270
C-PLACE1010562
C-PLACE1010579//Homo sapiens PTB domain adaptor protein CED-6 mRNA, complete cds.//9.30E-299//1362bp//99%//AF200715
C-PLACE1010624
C-PLACE1010628//Homo sapiens S164 gene, partial cds; PS1 and hypothetical protein genes, complete cds; and S171 gene, partial cds.//7.50E-08//324bp//64%//AF109907
C-PLACE1010662//UDP-GLUCOSE:GLYCOPROTEIN GLUCOSYLTRANSFERASE PRECURSOR (EC 2.4.1.-) (DUGT).//1.80E-222//808aa//52%//Q09332
C-PLAC.E1010702//ZINC FINGER PROTEIN 43 (ZINC PROTEIN HTF6).//5.20E-151//427aa//55%//P28160
C-PLACE1010761
C-PLACE1010802 C-PLACE1010833//CALTRACTIN (CENTRIN).//0.0000001//154aa//28%//P41209
C-PLACE1010896//NUF1 PROTEIN (SPINDLE POLY BODY SPACER PROTEIN SPC110).//1.50E-25//583aa//23%//P35580
C-PLACE1010916
C-PLACE1010947
C-PLACE1010965
C-PLACE1011032
C-PLACE1011041//Homo sapiens mRNA for BAP2-alpha protein, complete cds.//0//1701bp//97%//AB015019
C-PLACE1011056//HISTONE H1, GONADAL.//6.80E-13//154aa//37%//P02256
C-PLACE1011090//Homo sapiens mRNA; cDNA DKFZp586A0522 (from clone DKFZp586A0522).//0//880bp//99%//AL050159
C-PLACE1011160//Homo sapiens HFB30 mRNA, complete cds.//0//1691bp//99%//AB022663
C-PLACE1011214
C-PLACE1011229//Homo sapiens ubiquitin-specific protease homolog (UPH) mRNA, complete cds.//2.30E-152//701bp//99%//AF153604
C-PLACE1011273
C-PLACE1011291
C-PLACE1011310//MYOSIN HEAVY CHAIN, GIZZARD SMOOTH MUSCLE.//3.50E-20//496aa//25%//P10587
C-PLACE1011371//INTER-ALPHA-TRYPSIN INHIBITOR HEAVY CHAIN H2 PRECURSOR (ITI HEAVY CHAIN H2).//1.70E-78//383aa//39%//Q61703
C-PLACE1011503
C-PLACE1011635//Homo sapiens heparan sulfate D-glucosaminyl 3-O-sulfotransferase-3B (30ST3B1) mRNA, complete cds.//0//1559bp//99%//AF105377
C-PLACE1011646//Homo sapiens clone 25059 mRNA sequence.//5.00E-223//1035bp//99%//AF131752
C-PLACE1011650
C-PLACE1011675
C-PLACE1011725
C-PLACE1011749
C-PLACE1011922//MYOSIN HEAVY CHAIN, NONMUSCLE TYPE B (CELLULAR MYOSIN HEAVY CHAIN, TYPE B) (NMMHC-B).//1.30E-15//409aa//27%//P35580
C-PLACE1012031//Homo sapiens mRNA for KIAA0713 protein, partial cds.//0//1163bp//100%//AB018256
C-PLACE2000006
C-PLACE2000007//Homo sapiens mRNA for KIAA0913 protein, partial cds.//0//1968bp//97%//AB020720
C-PLACE2000034//LAR PROTEIN PRECURSOR (LEUKOCYTE ANTIGEN RELATED) (EC 3.1.3.48).//2.20E-29//212aa//35%//P10586
C-PLACE2000039//Rattus norvegicus cytoplasmic dynein heavy chain (MAP 1C), mRNA, complete cds.//4.60E-291//1167bp//89%//L08505
C-PLACE2000061
C-PLACE2000072//Homo sapiens ZNF202 beta (ZNF202) mRNA, complete cds.//0//3174bp//99%//AF027219
C-PLACE2000097
C-PLACE2000103
C-PLACE2000115
C-PLACE2000124
C-PLACE2000140
C-PLACE2000164//TIPD PROTEIN.//2.10E-59//481aa//33%//O15736
C-PLACE2000176
C-PLACE2000223
C-PLACE2000235
C-PLACE2000274//DYNEIN BETA CHAIN, CILIARY.//2.20E-167//880aa//37%//P23098
C-PLACE2000302
C-PLACE2000347
C-PLACE2000359
C-PLACE2000371//TENSIN.//2.90E-78//561aa//37%//Q04205
C-PLACE2000379
C-PLACE2000399//T-CELL SURFACE GLYCOPROTEIN E2 PRECURSOR (E2 ANTIGEN) (CD99) (MIC2 PROTEIN) (12E7).//1.60E-14//180aa//39%//P14209
C-PLACE2000404//PROBABLE LEUCYL-TRNA SYNTHETASE (EC 6.1.1.4) (LEUCINE--TRNA LIGASE) (LEURS).//9.90E-229//821aa//54%//Q09996
C-PLACE2000450
C-PLACE2000455
C-PLACE3000059//Mus musculus mRNA for ubiquitin conjugating enzyme.//0//1979bp//90%//Y17267
C-PLACE3000070
C-PLACE3000119
C-PLACE3000121//VESICULAR TRAFFIC CONTROL PROTEIN SEC15.//1.90E-08//281 aa//22%//P22224
C-PLACE3000136
C-PLACE3000147//Homo sapiens metalloproteinase with thrombospondin type 1 motifs ADAMTS1 (ADAMTS1) mRNA, complete cds.//0//2043bp//99%//AF170084
C-PLACE3000148
C-PLACE3000155//Homo sapiens mRNA for KIAA0672 protein, complete cds.//2.10E-75//382bp//99%//AB014572
C-PLACE3000160
C-PLACE3000169//ZINC FINGER PROTEIN 135.//2.50E-90//358aa//47%//P52742
C-PLACE3000194
C-PLACE3000199
C-PLACE3000218//Homo sapiens putative protein O-mannosyltransferase (POMT2) mRNA, complete cds.//0//1862bp//98%//AF105020
C-PLACE3000230
C-PLACE3000244//PROTEIN TSG24 (MEIOTIC CHECK POINT REGULATOR).//0//1435aay/92%//P53995
C-PLACE3000254//Homo sapiens transcriptional activator SRCAP (SRCAP) mRNA, complete cds.//0//4583bp//83%//AF143946
C-PLACE3000276
C-PLACE3000310
C-PLACE3000320
C-PLACE3000331
C-PLACE3000339//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//9.60E-08//359aa//23 %//P08640
C-PLACE3000352
C-PLACE3000353//Homo sapiens mRNA; cDNA DKFZp586H0623 (from clone DKFZp586H0623).//0//2456bp//99%//AL096739
C-PLACE3000362
C-PLACE3000365
C-PLACE3000388
C-PLACE3000413
C-PLACE3000425
C-PLACE4000009//MYOSIN HEAVY CHAIN, NONMUSCLE TYPE B (CELLULAR MYOSIN HEAVY CHAIN, TYPE B) (NMMHC-B).//2.90E-54//626aa//29%//P35580
C-PLACE4000014//X-LINKED HELICASE II (X-LINKED NUCLEAR PROTEIN) (XNP).//3.10E-111//348aa//41%//P46100
C-PLACE4000052//Homo sapiens ATP cassette binding transporter 1 (ABC1) mRNA, complete cds.//0//4661bp//99%//AF165281
C-PLACE4000089
C-PLACE4000128//Mus musculus putative transcription factor mRNA, complete cds.//1.60E-86//190aa//88%//AF091234
C-PLACE4000129
C-PLACE4000147
C-PLACE4000192//ZINC FINGER PROTEIN 142 (KIAA0236) (HA4654).//7.00E-22//369aa//25%//P52746
C-PLACE4000211//Homo sapiens TTF-I interacting peptide 5 mRNA, partial cds.//1.70E-262//1217bp//98%//AF000422
C-PLACE4000222
C-PLACE4000269//Homo sapiens mRNA for KIAA1067 protein, partial cds.//0//3787bp//99%//AB028990
C-PLACE4000270
C-PLACE4000300
C-PLACE4000387
C-PLACE4000392
C-PLACE4000431//H.sapiens gene for U5 snRNP-specific 200kD protein.//0//5142bp//90%//Z70200
C-PLACE4000450//Homo sapiens TTF-I interacting peptide 5 mRNA, partial cds.//2.70E-261//1217b.p//98%//AF000422
C-PLACE4000465
C-PLACE4000489//PROTEIN GRAINY-HEAD (DNA-BINDING PROTEIN ELF-1) (ELEMENT I-BINDING ACTIVITY) (TRANSCRIPTION FACTOR NTF-1).//5.70E-60//254aa//44%//P13002
C-PLACE4000654//Mus musculus mRNA for ubiquitin conjugating enzyme.//0//6340bp//87%//Y17267
C-SKNMC1000011//PUTATIVE IMPORTIN BETA-4 SUBUNIT (KARYOPHERIN BETA-4 SUBUNIT).//5.50E-35//431aa//29%//O60100
C-SKNMC1000046//Homo sapiens liprin-alpha3 mRNA, partial cds.//1.90E-162//749bp//99%//AF034800
C-SKNMC1000050//CALPAIN 2, LARGE [CATALYTIC] SUBUNIT (EC 3.4.22.17) (CALCIUM-ACTIVATED NEUTRAL PROTEINASE) (CANP) (M-TYPE).//3.20E-41//87aa//98%//P17655
C-THYRO1000034//TRICHOHYALIN.//9.40E-10//176aa//30%//P37709
C-THYRO1000070
C-THYRO1000072//MYOSIN LIGHT CHAIN KINASE, SMOOTH MUSCLE AND NON-MUSCLE ISOZYMES (EC 2.7.1.117) (MLCK) [CONTAINS: TELOKIN].//3.40E-16//201aa//29%//P11799
C-THYRO1000092
C-THYRO1000121//Homo sapiens mRNA for KIAA1116 protein, complete cds.//0//2159bp//99%//AB029039
C-THYRO1000124
C-THYRO1000197//Homo sapiens mRNA for poly(A)-specific ribonuclease.//0//2362bp//99%//AJ005698
C-THYRO1000199//Homo sapiens mRNA for KIAA0652 protein, complete cds.//0//1409bp//98%//AB014552
C-THYRO1000206
C-THYRO1000242//ZINC FINGER PROTEIN 84 (ZINC FINGER PROTEIN HPF2).//5.00E-118//239aa//66%//P51523
C-THYRO1000253
C-THYRO1000270
C-THYRO1000288//Homo sapiens mRNA for Hs Ste24p, complete cds.//0//2161bp//99%//AB016068
C-THYRO1000320
C-THYRO1000358//SELENIUM-BINDING LIVER PROTEIN.//2.30E-229//237aa//79%//P17563
C-THYRO1000368
C-THYRO1000381
C-THYRO1000387
C-THYRO1000394//Homo sapiens peroxisomal membrane protein PMP 24 mRNA, complete cds.//1.20E-299//1325bp//99%//AF072864
C-THYRO10003957/Homo sapiens actin-binding protein (IPP) mRNA, complete cds.//0//2092bp//99%//AF156857
C-THYRO1000401
C-THYRO1000488//Homo sapiens HFB30 mRNA, complete cds.//0//2254bp//100%//AB022663
C-THYRO1000501//52 KD RO PROTEIN (SJOGREN SYNDROME TYPE A ANTIGEN (SS-A))(RO(SS-A)).//4.20E-98//408aa//42%//P19474
C-THYRO1000558
C-THYRO1000570
C-THYRO1000605//Homo sapiens histone acetyltransferase (HBOa) mRNA, complete cds.//0//3080bp//99%//AF140360
C-THYRO1000625
C-THYRO1000637
C-THYRO1000676
C-THYRO1000684//Homo sapiens mRNA for KIAA0872 protein, complete cds.//0//2131bp//99%//AB020679
C-THYRO1000712
C-THYRO1000805
C-THYRO1000815
C-THYRO1000855
C-THYRO1000934//PYRROLINE-5-CARBOXYLATE REDUCTASE (EC 1.5.1.2) (P5CR) (P5C REDUCTASE).//7.50E-57//315aa//43%//P32322
C-THYRO1000988
C-THYRO1001033//TRANSFORMATION-SENSITIVE PROTEIN IEF SSP 3521.//8.40E-12//167aa//29%//P31948
C-THYRO1001120//Mus musculus FX-induced thymoma transcript (FXI-T1) mRNA, complete cds.//1.90E-92//1479bp//66%//U38252
C-THYRO1001204//Homo sapiens cathepsin Z precursor (CTSZ) gene, exons 4, 5, and 6 and complete cds; and TH1 gene partial sequence.//3.80E-100//478bp//99%//AF136276
C-THYRO1001262
C-THYRO1001271
C-THYRO1001287//MANNOSYL-OLIGOSACCHARIDE ALPHA-1,2-MANNOSIDASE (EC 3.2.1.113) (MAN(9)-ALPHA-MANNOSIDASE) (FRAGMENT).//3.40E-51//429aa//33%//P45701
C-THYRO1001313//Homo sapiens sorting nexin 11 (SNX11) mRNA, complete cds.//0//2330bp//94%//AF121861
C-THYRO1001347
C-THYRO1001363//Homo sapiens mRNA; cDNA DKFZp56400423 (from clone DKFZp56400423).//0//2173bp//99%//AL080120
C-THYRO1001374//Homo sapiens mRNA forKIAA0707 protein, partial cds.//0//1700bp//99%//AB014607
C-THYRO1001403
C-THYRO1001405//PLECTIN.//6.90E-19//450aa//27%//P30427
C-THYRO1001406//Homo sapiens steroid dehydrogenase homolog mRNA, complete cds.//0//1676bp//98%//AF078850
C-THYRO1001426
C-THYRO1001458//MYOSIN HEAVY CHAIN, NONMUSCLE TYPE B (CELLULAR MYOSIN HEAVY CHAIN, TYPE B) (NMMHC-B).//2.70E-171//559aa//59%//P35580
C-THYRO1001480
C-THYRO1001487
C-THYRO1001584
C-THYRO1001661
C-THYRO1001746
C-THYRO1001772
C-THYRO1001854
C-Y79AA1000013//Mus musculus RING finger protein A07 mRNA, complete cds.//8.90E-205//1435bp//81 %//AF171060
C-Y79AA1000033//Homo sapiens CARD4 mRNA, complete cds.//0//2929bp//96%//AF126484
C-Y79AA1000231//Homo sapiens nucleolar protein NOP5/NOP58 mRNA, complete cds.//0//1515bp//99%//AF123534
C-Y79AA1000342//Homo sapiens Ciz1 mRNA, complete cds.//0//2644bp//81%//AB030835
C-Y79AA1000349//M.musculus Spnr mRNA for RNA binding protein.//0//2048bp//93%//X84692
C-Y79AA1000410
C-Y79AA1000539
C-Y79AA1000589//Homo sapiens clone 614 unknown mRNA, complete sequence.//1.00E-302//1375bp//99%//AF091080
C-Y79AA1000802
C-Y79AA1000827
C-Y79AA1000966//Homo sapiens COP9 complex subunit 4 mRNA, complete cds.//0//1586bp//99%//AF100757
C-Y79AA1000969
C-Y79AA1000985//Human centrosomal protein kendrin mRNA, complete cds.//4.70E-151//985bp//87%//U52962
C-Y79AA1001061
C-Y79AA1001068
C-Y79AA1001216
C-Y79AA1001299//Homo sapiens mRNA for integrase interactor 1b protein (INI1B).//0//996bp//99%//AJ011738
C-Y79AA1001511
C-Y79AA1001594//HYALURONAN-MEDIATED MOTILITY RECEPTOR (HYALURONIC ACID RECEPTOR).//2.50E-14//410aa//24%//Q00547
C-Y79AA1001692//Mus musculus strain C57BL/J germ cell-less protein (Gcl) mRNA, complete cds.//1.40E-78//227aa//40%//Q01820
C-Y79AA1001866//Homo sapiens zinc finger protein ZNF180 (ZNF180) mRNA, complete cds.//0//2927bp//97%//AF192913
C-Y79AA1001874//OX40L RECEPTOR PRECURSOR (ACT35 ANTIGEN) (TAX-TRANSCRIPTIONALLY ACTIVATED GLYCOPROTEIN 1 RECEPTOR) (CD134 ANTIGEN).//4.50E-08//135aa//31%//P43489
C-Y79AA1002139//DNAJ PROTEIN HOMOLOG 1 (DROJ1).//9.00E-17//120aa//45%//Q24133
C-Y79AA1002210/YTUMOR NECROSIS FACTOR, ALPHA-INDUCED PROTEIN 1, ENDOTHELIAL (B12 PROTEIN).//0.0000018//140aa7/25%//Q13829
C-Y79AA1002211//PHOSPHATIDYLETHANOLAMINE-BINDING PROTEIN HOMOLOG F40A3.3.//1.70E-17//146aa//35%//016264
C-Y79AA1002220
C-Y79AA1002234//Homo sapiens mRNA for KIAA0692 protein, partial cds.//0//3168bp//99%//AB014592
C-Y79AA1002258//Homo sapiens mRNA for HIP1R, complete cds.//0//2106bp//99%//AB013384
C-Y79AA1002361//Rattus norvegicus mRNA for protein phosphatase 1 (GL-subunit).//6.90E-140//966bp//82%//Y18208
C-Y79AA1002399//Homo sapiens mRNA for sperm protein.//0//1163bp//95%//X91879
C-Y79AA1002416//Mus musculus CTP synthetase homolog (CTPsH) mRNA, complete cds.//3.9e-317//1902bp//86%//U49385
C-Y79AA1002431//TRANSDUCIN-LIKE ENHANCER PROTEIN 2 (ESG2).//9.80E-62//318aa//35%//Q04725
C-Y79AA1002482//ZINC FINGER PROTEIN 91 (ZINC FINGER PROTEIN HTF10) (HPF7).//2.70E-137//340aa//51%//Q05481
C-Y79AA1002487//Homo sapiens chromosome 5 F-box protein Fbx4 (FBX4) mRNA, complete cds.//7.3e-310//1444bp//98%//AF129534
C-HEMBA1000290
C-HEMBA1000459
C-HEMBA1000505
C-HEMBA1001196//Human DNA topoisomerase II (top2) mRNA, complete cds.//1.60E-268//1213bp//100%//J04088
C-HEMBA1002503
C-HEMBA1002508
C-HEMBA1002513//Homo sapiens mRNA for histone deacetylase-like protein (JM21).//0//2432bp//99%//AJ011972
C-HEMBA1003480
C-HEMBA1003538//COMPLEMENT C1R COMPONENT PRECURSOR (EC 3.4.21.41).//2.40E-110//242aa//58%//P00736
C-HEMBA10036451/TTPD PROTEIN.//2.40E-10//289aa//23%//015736
C-HEMBA1003646//Homo sapiens mRNA for KIAA1013 protein, partial cds.//0//3049bp//99%//AB023230
C-HEMBA1003667
C-HEMBA1003679//SIALIDASE (EC 3.2.1.18) (NEURAMINIDASE) (NA) (MAJOR SURFACE ANTIGEN).//1.00E-09//611aa//22%//P23253
C-HEMBA1003827
C-HEMBA1003838
C-HEMBA1004055
C-HEMBA1004056
C-HEMBA1004086
C-HEMBA1004335
C-HEMBA1004353//C-MYC BINDING PROTEIN MM-1.//3.00E-71//89aa//96%//Q99471
C-HEMBA1004479//HYPOXIA-INDUCIBLE FACTOR 1 ALPHA (HIF-1 ALPHA) (ARNT INTERACTING PROTEIN).//3.10E-51//152aa//40%//Q61221
C-HEMBA1004499//Homo sapiens delta-tubulin mRNA, complete cds.//3.40E-92//483bp//95%//AF201333
C-HEMBA1004507
C-HEMBA1004638
C-HEMBA1004669//SON PROTEIN (SON3).//7.30E-17//288aa//36%//P18583
C-HEMBA1004709
C-HEMBA1004860
C-HEMBA1005206//Drosophila simulans anon73B1 gene and Su(P) gene.//1.90E-11//376bp//63%//AJ250308
C-HEMBA1005472
C-HEMBA1005513//MALES-ABSENT ON THE FIRST PROTEIN (EC 2.3.1.-).//1.90E-129//332aa//61%//002193
C-HEMBA1005572
C-HEMBA1005780
C-HEMBA1005990//Homo sapiens I-1 receptor candidate protein mRNA, complete cds.//0//2371bp//100%//AF082516
C-HEMBA1006038//LAMININ ALPHA-5 CHAIN (FRAGMENT).//3.10E-33//81aa//64%//Q61001
C-HEMBA1006124
C-HEMBA1006461
C-HEMBA1006521//3-OXOACYL-[ACYL-CARRIER PROTEIN] REDUCTASE (EC 1.1.1.100) (3-KETOACYL- ACYL CARRIER PROTEIN REDUCTASE).//4.00E-33//177aa//42%//P25716
C-HEMBA1006617
C-HEMBA1006650//ARP2/3 COMPLEX 20 KD SUBUNIT (P20-ARC).//9.00E-40//113aa//82%//015509
C-HEMBA1006779
C-HEMBA1006796
C-HEMBA1006812
C-HEMBA1006914//Human anthracycline-associated resistance ARX mRNA, complete cds.//0//1837bp//99%//U35832
C-HEMBA1007174//Homo sapiens mRNA for KIAA1065 protein, complete cds.//0//1079bp//97%//AB028988
C-HEMBB1000240
C-HEMBB1000264//CHL1 PROTEIN.//9.50E-19//104aa//45%//P22516
C-HEMBB1000335
C-HEMBB1000337
C-HEMBB1000554
C-HEMBB1000573
C-HEMBB1000749
C-HEMBB1000774
C-HEMBB1000835
C-HEMBB1001197
C-HEMBB1001315
C-HEMBB1001482//ZINC FINGER PROTEIN 91 (ZINC FINGER PROTEIN HTF10) (HPF7).//2.10E-57//941aa//27%//Q05481
C-HEMBB1001500
C-HEMBB1001562//CYLICIN II (MULTIPLE-BAND POLYPEPTIDE II).//1.40E-06//373aa//21%//Q28092
C-HEMBB1001619
C-HEMBB1001630
C-HEMBB1001665
C-HEMBB1001684//Homo sapiens mRNA for KIAA1108 protein, partial cds.//0//2348bp//99%//AB029031
C-HEMBB1001812
C-HEMBB1001834
C-HEMBB1001869
C-HEMBB1001871//BONE/CARTILAGE PROTEOGLYCAN I PRECURSOR (BIGLYCAN) (PG-S 1).//5.40E-75//241aa//48%//P47853
C-HEMBB1001872//CELL SURFACE GLYCOPROTEIN EMR1 PRECURSOR (EMR1 HORMONE RECEPTOR) (CELL SURFACE GLYCOPROTEIN F4/80).//1.90E-22//210aa//27%//Q61549
C-HEMBB1001905//TRICHOHYALIN.//2.10E-10//268aa//27%//P37709
C-HEMBB1001908//Human monocytic leukaemia zinc finger protein (MOZ) mRNA, complete cds.//1.60E-131//874bp//86%//U47742
C-HEMBB1001915//UBIQUITIN CARBOXYL-TERMINAL HYDROLASE 64E (EC 3.1.2.15) (UBIQUITIN THIOLESTERASE 64E) (UBIQUITIN-SPECIFIC PROCESSING PROTEASE 64E) (DEUBIQUITINATING ENZYME 64E).//6.90E-132//561aa//50%//Q24574
C-HEMBB1001925
C-HEMBB1002044//Mus musculus mRNA for vascular cadherin-2.//0//3562bp//81%//Y08715
C-HEMBB1002134//ZINC-FINGER PROTEIN NEURO-D4.//8.10E-56//176aa//67%//P56163
C-HEMBB1002152
C-HEMBB1002300
C-HEMBB1002381
C-HEMBB1002383
C-HEMBB1002534
C-MAMMA1000143
C-MAMMA1000183//ZINC FINGER PROTEIN 84 (ZINC FINGER PROTEIN HPF2).//3.40E-134//359aa//63%//P51523
C-MAMMA1000227
C-MAMMA1000257
C-MAMMA1000264
C-MAMMA1000270
C-MAMMA1000279
C-MAMMA1000372
C-MAMMA1000559
C-MAMMA1000752
C-MAMMA1000760
C-MAMMA1000778
C-MAMMA1000855
C-MAMMA1000859
C-MAMMA1000897//INTER-ALPHA-TRYPSIN INHIBITOR HEAVY CHAIN H3 PRECURSOR (ITI HEAVY CHAIN H3) (SERUM-DERIVED HYALURONAN-ASSOCIATED PROTEIN) (SHAP).//1.00E-141//576aa//37%//Q06033
C-MAMMA1000940
C-MAMMA1001073
C-MAMMA1001080//Homo sapiens SNC73 protein (SNC73) mRNA, complete cds.//1.6e-312//1596bp//94%//AF067420
C-MAMMA10011987/Homo sapiens eps15RmRNA, partial cds.//0//2253bp//99%//AB015346
C-MAMMA1001202
C-MAMMA1001222//EBNA-2 NUCLEAR PROTEIN.//6.60E-09//255aa//29%//P12978
C-MAMMA1001252
C-MAMMA1001296
C-MAMMA1001502
C-MAMMA1001630
C-MAMMA1001633//ZINC FINGER PROTEIN 165.//6.30E-39//160aa//55%//P49910
C-MAMMA1001683
C-MAMMA1001715
C-MAMMA1001730//Homo sapiens brain and nasopharyngeal carcinoma susceptibility protein NSG-x mRNA, partial cds.//0//1603bp//99%//AF095687
C-MAMMA1001760
C-MAMMA1001769
C-MAMMA1001785
C-MAMMA1001848
C-MAMMA1001874
C-MAMMA1001956
C-MAMMA1002009
C-MAMMA1002033
C-MAMMA1002155
C-MAMMA1002498
C-MAMMA1002545
C-MAMMA1002571
C-MAMMA1002573//GLUCOAMYLASE S1/S2 PRECURSOR (EC 3.2.1.3)(GLUCAN 1,4-ALPHA- GLUCOSIDASE) (1,4-ALPHA-D-GLUCAN GLUCOHYDROLASE).//2.60E-19//666aa//23%//P08640
C-MAMMA1002590
C-MAMMA1002617//ZINC FINGER PROTEIN 135.//7.60E-89//252aa//57%//P52742
C-MAMMA1002618
C-MAMMA1002636
C-MAMMA1002646
C-MAMMA1002665
C-MAMMA1002708
C-MAMMA1002728
C-MAMMA1002744
C-MAMMA1002764
C-MAMMA1002765
C-MAMMA1002830
C-MAMMA1002844//TRIOSE PHOSPHATE/PHOSPHATE TRANSLOCATOR, NON-GREEN PLASTID PRECURSOR (CTPT).//4.90E-10//334aa//22%//P52178
C-MAMMA100285 8//Rat cMG1 mRNA.//3.70E-238//1147bp//92%//X52590
C-MAMMA1002880
C-MAMMA1002892
C-MAMMA1002909
C-MAMMA1002941
C-MAMMA1002947
C-MAMMA1002972//VACUOLAR PROTEIN SORTING-ASSOCIATED PROTEIN VPS27.//1.10E-05//69aa//42%//P40343
C-MAMMA1002973
C-MAMMA1002987
C-MAMMA1003003
C-MAMMA1003026//Homo sapiens HSPC057 mRNA, complete cds.//0//1773bp//98%//AF161542
C-MAMMA1003031
C-MAMMA1003089
C-NT2RM1000092//MULTIDRUG RESISTANCE PROTEIN 2 (MULTIDRUG-EFFLUX TRANSPORTER 2).//1.00E-07//362aa//23%//P39843
C-NT2RM1000272
C-NT2RM1000341
C-NT2RM1000539//Homo sapiens mRNA for Lsm5 protein.//3.00E-158//733bp//99%//AJ238097
C-NT2RM1000553//Homo sapiens putative glycolipid transfer protein mRNA, complete cds.//3.40E-177//814bp//99%//AF103731
C-NT2RM1000623//RIBONUCLEASE INHIBITOR.//4.40E-21//372aa//30%//P10775
C-NT2RM1000702//PUTATIVE SERINE/THREONINE-PROTEIN KINASE PKWA (EC 2.7.1.-).//5.60E-08//187aa//27%//P49695
C-NT2RM1000833//Homo sapiens sec61 homolog mRNA, complete cds.//0//3541bp//99%//AF084458
C-NT2RM1000883//Homo sapiens I-1 receptor candidate protein mRNA, complete cds.//0//5107bp//99%//AF082516
C-NT2RM1001082
C-NT2RM1001112
C-NT2RM2001105//Drosophila melanogaster eyelid (eld) mRNA, complete cds.//1.20E-28//805bp//61%//AF053091
C-NT2RM2001360//Homo sapiens clone C40 unknown mRNA.//1.00E-250//1136bp//100%//AF103798
C-NT2RM2001797//Homo sapiens mRNA; cDNA DKFZp572C163 (from clone DKFZp572C163); partial cds.//0//2300bp//100%//AL110217
C-NT2RM2001803//Homo sapiens IkappaB kinase cbmplex associated protein (IKAP) mRNA, complete cds.//0//2249bp//99%//AF044195
C-NT2RM4002504
C-NT2RP1000409
C-NT2RP1000460//Homo sapiens mRNA for KIAA1068 protein, partial cds.//0//3199bp//99%//AB028991
C-NT2RP1000746//Homo sapiens 60S acidic ribosomal protein PO mRNA, complete cds.//9.70E-196//901bp//99%//AF173378
C-NT2RP1000796
C-NT2RP1001013//ZINC FINGER PROTEIN 83 (ZINC FINGER PROTEIN HPF1).//7.70E-253//425aa//98%//P51522
C-NT2RP2001214
C-NT2RP2001233//ZINC FINGER PROTEIN 91 (ZINC FINGER PROTEIN HTF10) (HPF7).//2.00E-128//409aa//45%//Q05481
C-NT2RP2001440//Homo sapiens mRNA for 14-3-3gamma, complete cds.//0//3712bp//99%//AB024334
C-NT2RP2002056
C-NT2RP2002105//H.sapiens MSH-R gene for melanocyte stimulating hormone receptor.//0//1644bp//98%//X65634
C-NT2RP2002333
C-NT2RP2002677
C-NT2RP2002755
C-NT2RP2002843
C-NT2RP2003101
C-NT2RP2003668
C-NT2RP2003799
C-NT2RP2004095
C-NT2RP2004300
C-NT2RP2004675
C-NT2RP2004920//TRANSCRIPTIONAL REGULATOR ATRX (X-LINKED NUCLEAR PROTEIN) (HETEROCHROMATIN PROTEIN 2) (HP1 ALPHA-INTERACTING PROTEIN) (HP1-BP38 PROTEIN).//4.20E-09//804aa//22%//Q61687
C-NT2RP2005144//Homo sapiens tubby like protein 3 (TULP3) mRNA, complete cds.//2.10E-308//1437bp//98%//AF045583
C-NT2RP2005719//GPI-ANCHORED PROTEIN P137.//4.00E-14//99aa//43%//Q14444
C-NT2RP2005726
C-NT2RP2005776//POLY(A) POLYMERASE TYPE 2 (EC 2.7.7.19) (PAP) (POLYNUCLEOTIDE ADENYLYLTRANSFERASE).//4.40E-55//358aa//42%//P51005
C-NT2RP2005980
C-NT2RP2006184//Homo sapiens mRNA for KIAA0918 protein, partial cds.//0//4235bp//99%//AB020725
C-NT2RP2006534//5'-AMP-ACTIVATED PROTEIN KINASE, CATALYTIC ALPHA-1 CHAIN (EC 2.7.1.-) (AMPK ALPHA-1 CHAIN) (FRAGMENT).//3.20E-11//32aa//96%//Q13131
C-NT2RP2006554
C-NT2RP3000584
C-NT2RP3001115
C-NT2RP3001723//Homo sapiens cell recognition molecule Caspr2 (CASPR2) mRNA, complete cds.//1.40E-58//1138bp//63%//AF193613
C-NT2RP3001938//SPORULATION-SPECIFIC PROTEIN 1 (EC 2.7.1.-).//1.30E- 22//227aa//33%//P08458
C-NT2RP3002330//Homo sapiens eRFS mRNA, complete cds.//0//2443bp//99%//U87791
C-NT2RP3002402
C-NT2RP3002484//Homo sapiens mRNA for KIAA0998 protein, partial cds.//1.20E-124//597bp//98%//AB023215
C-NT2RP3002512
C-NT2RP3002713
C-NT2RP3002770//MYELOID DIFFERENTIATION PRIMARY RESPONSE PROTEIN MYD116.//1.00E-07//70aa//41%//P17564
C-NT2RP3002799
C-NT2RP3002810//HISTIDINE-RICH PROTEIN KE4.//2.20E-10//260aa//26%//Q31125
C-NT2RP3002818//INSERTION ELEMENT IS2A HYPOTHETICAL 48.2 KD PROTEIN.//5.70E-226//303aa//97%//P51026
C-NT2RP3002955
C-NT2RP3002985
C-NT2RP3003059//Rattus norvegicus potassium channel regulator 1 mRNA, complete cds.//3.80E-152//1007bp//82%//U78090
C-NT2RP3003121
C-NT2RP3003133//Homo sapiens ZK1 mRNA for Kruppel-type zinc fmger protein, complete cds.//0//1998bp//91%//AB011414
C-NT2RP3003138//Homo sapiens kinesin superfamily motor KIF4 mRNA, complete cds.//0//2159bp//98%//AF071592
C-NT2RP3003155
C-NT2RP3003157
C-NT2RP3003185//TROPOMYOSIN 1, FUSION PROTEIN 33.//2.80E-06//402aa//23%//P49455
C-NT2RP3003264
C-NT2RP3003346
C-NT2RP3003403
C-NT2RP3003411//Mus musculus COP9 complex subunit 7b (COPS7b) mRNA, complete cds.//6.30E-270//743bp//90%//AF071317
C-NT2RP3003500//SCY1 PROTEIN.//9.20E-27//601aa//23%//P53009
C-NT2RP3003572
C-NT2RP3003576
C-NT2RP3003665//Homo sapiens mRNA for beta-ureidopropionase, complete cds.//0//1690bp//99%//AB013885
C-NT2RP3003672//T-CELL SURFACE GLYCOPROTEIN E2 PRECURSOR (E2 ANTIGEN) (CD99) (MIC2 PROTEIN) (12E7).//2.20E-13//146aa//42%//P14209
C-NT2RP3003680//Homo sapiens mRNA; cDNA DKFZp434J154 (from clone DKFZp434J154); complete cds.//0//2047bp//95%//AL080155
C-NT2RP3003799//Rattus norvegicus Srg1 (Sytr1) mRNA, complete cds.//9.00E-238//1529bp//84%//U71294
C-NT2RP3003800//Rattus norvegicus tyrosine protein kinase pp60-c-src mRNA, complete cds.//1.90E-163//924bp//89%//AF130457
C-NT2RP3003828
C-NT2RP3003932
C-NT2RP3003992//Homo sapiens mRNA; cDNA DKFZp564C186 (from clone DKFZp564Cl 86).//0//2739bp//99%//AL050019
C-NT2RP3004013//M.musculus Spnr mRNA for RNA binding protein.//6.50E-240//1215bp//94%//X84692
C-NT2RP3004028
C-NT2RP3004041
C-NT2RP3004051
C-NT2RP3004078//H.sapiens HRFX2 mRNA.//0//1806bp//99%//X76091
C-NT2RP3004093
C-NT2RP3004095
C-NT2RP3004125//Mus musculus zinc finger protein splice variant FIZ1-B (Fiz1) mRNA, complete cds.//4.60E-229//1560bp//78%//AF126747
C-NT2RP3004148//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//7.90E-05//271aa//22%//P08640
C-NT2RP3004155//Homo sapiens COQ7 protein mRNA, complete cds.//1.10E-179//823bp//100%//AF098948
C-NT2RP3004189//VEGETATIBLE INCOMPATIBILITY PROTEIN HET-E-1*J*/1.30E-14//242aa//24%//Q00808
C-NT2RP3004332
C-NT2RP3004349
C-NT2RP3004470
C-NT2RP4000035
C-NT2RP4000049
C-NT2RP4000102
C-NT2RP4000167
C-NT2RP4000515
C-NT2RP4000517
C-NT2RP4000519
C-NT2RP5003512//Homo sapiens mRNA for KIAA1291 protein, partial cds.//0//1980bp//99%//AB033117
C-OVARC1000092
C-OVARC1000533
C-OVARC1000678
C-OVARC1000689//Homo sapiens mRNA; cDNA DKFZp434C1415 (from clone DKFZp434C1415); partial cds.//0//2032bp//99%//AL133014
C-OVARC1000802
C-OVARC1000890
C-OVARC1000891
C-OVARC1000945//Rattus norvegicus mRNA for atypical PKC specific binding protein, complete cds.//0//1961bp//82%//AB005549
C-OVARC1001072
C-OVARC1001117
C-OVARC1001200//Mus musculus mRNA for HS1 binding protein 3.//5.80E-88//658bp//80%//AJ132192
C-OVARC1001244//H.sapiens mRNA for Drosophila female sterile homeotic (FSH) homologue.//0//1467bp//99%//X62083
C-OVARC1001329
C-OVARC1001341
C-OVARC1001376
C-OVARC1001496//Homo sapiens C-terminal binding protein 2 mRNA, complete cds.//0//1876bp//98%//AF016507
C-OVARC1001873
C-PLACE1000007//PROBABLE UBIQUITIN CARBOXYL-TERMINAL HYDROLASE R10E11.3 (EC 3.1.2.15) (UBIQUITIN THIOLESTERASE) (UBIQUITIN-SPECIFIC PROCESSING PROTEASE) (DEUBIQUITINATING ENZYME).//1.60E-81//212aa//70%//P34547
C-PLACE1000547//Homo sapiens GDP-mannose pyrophosphorylase B (GMPPB) mRNA, complete cds.//3.70E-241//1124bp//98%//AF135421
C-PLACE1001036//Homo sapiens mRNA for KIAA1017 protein, complete cds.//0//2117bp//99%//AB023234
C-PLACE1001076
C-PLACE1001118//ZINC FINGER PROTEIN 135.//5.40E-147//443aa//57%//P52742
C-PLACE1001366
C-PLACE1001545
C-PLACE1001608
C-PLACE1002004
C-PLACE1002256
C-PLACE1002437//ATP-BINDING CASSETTE TRANSPORTER 1.//4.50E-76//180aa//83%//P41233
C-PLACE1002591//CORONIN-LIKE PROTEIN P57.//4.40E-70//208aa//66%//P31146
C-PLACE1002665//Mus musculus enhancer of polycomb (Epc1) mRNA, complete cds.//0//2462bp//89%//AF079765
C-PLACE1003383
C-PLACE1003864
C-PLACE1004793//RETROVIRUS-RELATED ENV POLYPROTEIN.//5.20E-47//577aa//25%//P10267
C-PLACE1004913
C-PLACE1004979
C-PLACE1005052//Homo sapiens CGI-16 protein mRNA, complete cds.//6.6e-313//1413bp//99%//AF132950
C-PLACE1005055//Homo sapiens mRNA for KIAA0576 protein, partial cds.//0//2431bp//99%//AB011148
C-PLACE1005128
C-PLACE1005162
C-PLACE1005176//Homo sapiens hypothalamus protein HT001 mRNA, complete cds.//3.90E-212//1040bp//96%//AF113539
C-PLACE1005467//PENICILLIN-BINDING PROTEIN 4* (PBP 4*) (PBP 4A).//1.10E-09//93aa//31%//P32959
C-PLACE1005549//Homo sapiens mRNA for Rho guanine nucleotide-exchange factor, splice variant NET1A.//7.60E-97//1287bp//67%//AJ010046
C-PLACE1005584//TRANS-ACTING TRANSCRIPTIONAL PROTEIN ICP0 (P135 PROTEIN) (IER 2.9/ER2.6).//6.80E-09//267aa//30%//P29128
C-PLACE1005611//Mus musculus mRNA for mDjlO, complete cds.//2.00E-33//379bp//66%//AB028860
C-PLACE1005802
C-PLACE1005850
C-PLACE1005898
C-PLACE1005932
C-PLACE1006129//Homo sapiens HSPC057 mRNA, complete cds.//0//2849bp//98%//AF161542
C-PLACE1006360
C-PLACE1006795
C-PLACE1006878//TRNA-SPUCING ENDONUCLEASE SUBUNIT SEN2 (EC 3.1.27.9) (TRNA-INTRON ENDONUCLEASE).//1.90E-08//122aa//36%//P16658
C-PLACE1007557
C-PLACE1007807
C-PLACE1008181
C-PLACE1008426//Homo sapiens mRNA for KIAA1288 protein, partial cds.//0//3311bp//99%//AB033114
C-PLACE1008455
C-PLACE1008941
C-PLACE1009935
C-PLACE1010310//SPIDROIN 2 (DRAGLINE SILK FIBROIN 2) (FRAGMENT).//1.20E-18//467aa//30%//P46804
C-PLACE1011891
C-PLACE10118967/Mus musculus Wnt10a mRNA, complete cds.//2.60E-287//1820bp//85%//U61969
C-PLACE2000003
C-PLACE2000132
C-PLACE2000170
C-PLACE2000335
C-PLACE3000124
C-PLACE3000158
C-PLACE3000207
C-PLACE3000221
C-PLACE3000271
C-PLACE3000304
C-PLACE3000322
C-PLACE3000341
C-PLACE3000373
C-PLACE3000399
C-PLACE3000401
C-PLACE3000402
C-PLACE3000406
C-PLACE3000475
C-PLACE4000063//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//1.70E-15//740aa//23%//P08640
C-PLACE4000093
C-PLACE4000100//Homo sapiens hydroxypyruvate reductase (GRHPR) gene, complete cds.//0//4199bp//97%//AF146689
C-PLACE4000131//Homo sapiens mRNA; cDNA DKFZp586J0917 (from clone DKFZp586J0917); partial cds.//0//1612bp//97%//AL117455
C-PLACE4000247
C-PLACE4000250
C-PLACE4000252
C-PLACE4000259//H.sapiens gene for U5 snRNP-specific 200kD protein.//0//5143bp//90%//Z70200
C-PLACE4000261//PEREGRIN (BR140 PROTEIN).//9.50E-10//128aa//34%//P55201
C-PLACE4000320
C-PLACE4000344
C-PLACE4000367
C-PLACE4000401//ACETYL-COENZYME A SYNTHETASE (EC 6.2.1.1) (ACETATE--COA LIGASE) (ACYL- ACTIVATING ENZYME).//7.20E-22//54aa//62%//Q01576
C-PLACE4000411//Homo sapiens mRNA; cDNA DKFZp586D0624 (from clone DKFZp586D0624); partial cds.//0//2159bp//98%//AL117654
C-PLACE4000487
C-PLACE4000494
C-PLACE4000521
C-PLACE4000548//Homo sapiens mRNA for KIAA0947 protein, partial cds.//0//4864bp//99%//AB023164
C-SKNMC1000013//Homo sapiens ATP-binding cassette protein M-ABC1 mRNA, nuclear gene encoding mitochondrial protein, complete cds.//0//2384bp//99%//AF047690
C-SKNMC1000091//Homo sapiens mRNA for leucine-zipper protein, complete cds.//6.10E-190//872bp//99%//AB021663
C-THYRO1000343//Homo sapiens mRNA for KIAA0790 protein, partial cds.//0//3711bp//99%//AB018333
C-THYRO1000569//Mus musculus hematopoietic zinc finger protein mRNA, complete cds.//0//1557bp//91%//AF118566
C-THYRO1001142
C-THYRO1001189//ZINC FINGER PROTEIN 91 (ZINC FINGER PROTEIN HTF10) (HPF7).//1.10E-200//546aa//62%//005481
C-THYRO1001320
C-THYRO1001537//Homo sapiens mRNA; cDNA DKFZp586A0522 (from clone DKFZp586A0522); partial cds.//0//1010bp//98%//AL050159
C-THYRO1001602
C-THYRO1001721//RING CANAL PROTEIN (KELCH PROTEIN).//9.30E-34//220aa//38%//Q04652
C-THYRO1001828
C-Y79AA1000346//Homo sapiens nonclathrin coat protein gamma2-COP mRNA, complete cds.//0//2520bp//99%//AF157833
C-Y79AA1001167
C-Y79AA1001384//Homo sapiens very large G-protein coupled receptor-1 (VLGR1) mRNA, complete cds.//0//4708bp//99%//AF055084
C-Y79AA1001875//RAS-RELATED PROTEIN RAB-7.//9.40E-12//34aa//97%//P51149
C-Y79AA1002103//ZINC FINGER PROTEIN ZFP-36 (FRAGMENT).//3.00E-257//549aa//76%//P16415
C-HEMBA1006092
C-HEMBA1006406
C-HEMBB1000790
C-HEMBB1000917
C-HEMBB1002280
C-MAMMA1000802
C-MAMMA1001322//B-CELL GROWTH FACTOR PRECURSOR (BCGF-12 KD).//0.000000017//46aa//60%//P20931
C-MAMMA1002597
C-MAMMA1002868
C-NT2RP2003161
C-NT2RP2003339
C-NT2RP3001282
C-PLACE1001761
C-PLACE1004491
C-PLACE1004686
C-PLACE1005574
C-PLACE1006382
C-PLACE1006792
C-PLACE3000455
C-PLACE4000230//Mus musculus semaphorin VIa mRNA, complete cds.//0//2567bp//88%//AF030430
C-THYRO1000916
C-HEMBA1000327
C-HEMBB1000637
C-HEMBB1001967
C-MAMMA1000266
C-NT2RP2002979
C-PLACE1007866
C-PLACE3000350//SERINE/THREONINE-PROTEIN KINASE PAK-GAMMA (EC 2.7.1.-) (GAMMA-PAK) (P21-ACTIVATED KINASE 2) (PAK-2) (PAK65) (S6/H4 KINASE).//9.80E-25//155aa//45%//Q13177
C-PLACE4000156//ZINC FINGER PROTEIN 132.//7.10E-151//476aa//46%//P52740
C-THYRO1001637
C-MAMMA1002215
C-MAMMA1002721
C-NT2RP2002070

### Homology search result 14.

Data obtained by the homology search for full-length nucleotide sequences and deduced amino acid sequences. In the result of the search shown below, both units, aa and bp, are used as length units for the sequences to be compared. Each data includes Clone name, Definition in matching data, P value, Length of sequence to be compared, Homology, and Accession number (No.) of matching data. These items are shown in this order, separated by a double-slash mark, //.
C-HEMBA1000005//DNAJ PROTEIN HOMOLOG MTJ1.//1.90E-250//554aa//85%//061712
C-HEMBA1000012//PROBABLE LEUCYL-TRNA SYNTHETASE (EC 6.1.1.4) (LEUCINE--TRNA LIGASE) (LEURS).//6.40E-99//457aa//45%//Q09996
C-HEMBA1000020//Homo sapiens beta 2 gene.//7.50E-264//1194bp//95%//X02344
C-HEMBA1000030//Homo sapiens ARF GTPase-activating protein GITl mRNA, complete cds.//0//1759bp//99%//AF124490
C-HEMBA1000129//HYPOTHETICAL HEUCASE C8A4.08C IN CHROMOSOME I.//3.80E-25//166aa//36%//Q09884
C-HEMBA1000141//Homo sapiens SUMO-1-specific protease (SSP1) mRNA, complete cds.//0/1135bp//100%//AF196304
C-HEMBA1000150//Homo sapiens putative RNA helicase mRNA, complete cds.//5.20E-213//525bp//99%//AF085356
C-HEMBA1000156//NEUROFILAMENT TRIPLET M PROTEIN (160 KD NEUROFILAMENT PROTEIN) (NF-M).//1.90E-12//368aa//24%//P08553
C-HEMBA1000158//HEPATOCYTE NUCLEAR FACTOR 3-GAMMA (HNF-3G).//5.00E-16//166aa//36%//P35584
C-HEMBA1000168//CYLICIN I (MULTIPLE-BAND POLYPEPTIDE D.//2.90E-14//303aa//25%//P35662
C-HEMBA1000185//RAS-RELATED PROTEIN RAL-A.//3.40E-12//125aa//31 %//P48555
C-HEMBA1000201//Homo sapiens mRNA for integrase interactor 1b protein (INI1B).//0//1612bp//99%//AJ011738
C-HEMBA1000216//HYPOXIA-INDUCIBLE FACTOR 1 ALPHA (HIF-1 ALPHA) (ARNT INTERACTING PROTEIN).//1.00E-86//146aa//56%//Q61221
C-HEMBA1000303//Mus musculus Plenty of SH3s (POSH) mRNA, complete cds.//7.10E-254//1440bp//87%//AF030131
C-HEMBA1000304//Rattus norvegicus Ca2⁺-dependent activator protein (CAPS) mRNA, complete cds.//5.10E-131//712bp//91%//U16802
C-HEMBA1000307//CARNITINE DEFICIENCY-ASSOCIATED PROTEIN EXPRESSED IN VENTRICLE 1//5.20E-49//107aa//91%//035594
C-HEMBA1000333//Homo sapiens F-box protein Fbx21 (FBX21) mRNA, complete cds.//0//1866bp//100%//AF174601
C-HEMBA1000369//Homo sapiens mRNA for PICK1, complete cds.//0//1949bp//98%//AB026491
C-HEMBA1000411//ANKYRIN.//5.70E-12//127aa//38%//Q02357
C-HEMBA1000488//RING CANAL PROTEIN (KELCH PROTEIN).//3.30E-45//481aa//29%//Q04652
C-HEMBA1000491//RAS-LIKE PROTEIN 2.//2.00E-22//188aa//31%//P22279
C-HEMBA1000518//PECANEX PROTEIN.//2.10E-19//227aa//38%//P18490
C-HEMBA1000523//TESTIS-SPECIFIC PROTEIN PBS13.//2.40E-44//292aa//36%//Q01755
C-HEMBA1000531//HEAT SHOCK 70 KD PROTEIN COGNATE 1 (HEAT SHOCK 70 KD PROTEIN 70C) (FRAGMENTS).//2.60E-12//73aa//41%//P02826
C-HEMBA1000542//Rattus norvegicus mRNA for dipeptidyl peptidase III, complete cds.//2.20E-194//663bp//83%//D89340
C-HEMBA1000555//Mus musculus Msx2 interacting nuclear target protein mRNA, complete cds.//7.90E-226//1501bp//83%//AF156529
C-HEMBA1000561//ZINC FINGER PROTEIN 91 (ZINC FINGER PROTEIN HTF10) (HPF7).//3.40E-37//674aa//25 %//Q05481
C-HEMBA1000569//GPI-ANCHORED PROTEIN P137.//6.50E-19//265aabp//32%//Q60865
C-HEMBA1000588//Mus musculus FLI-LRR associated protein-1 mRNA, complete cds.//2.10E-144//602bp//77%//AF045573
C-HEMBA1000591//PTB-ASSOCIATED SPLICING FACTOR (PSF).//2.20E-17//198aa//40%//P23246
C-HEMBA1000592//Homo sapiens sorting nexin 6 (SNX6) mRNA, complete cds.//0//1465bp//99%//AF121856
C-HEMBA1000608//HYPOTHETICAL PROTEIN KIAA0411 (FRAGMENT).//1.80E-55//179aa//61%//O43295
C-HEMBA1000657//Rattus norvegicus ADP-ribosylation factor-directed GTPase activating protein mRNA, complete cds.//7.20E-156//1366bp//76%//U35776
C-HEMBA1000851//Homo sapiens DNA binding protein p96PIF mRNA, complete cds.//0//1862bp//99%//AF173868
C-HEMBA1000852//ARYLSULFATASE D PRECURSOR (EC 3.1.6.-) (ASD).//1.00E-78//119aa//87%//P51689
C-HEMBA1000910//MELANOMA-ASSOCIATED ANTIGEN B1 (MAGE-B1 ANTIGEN) (MAGE-XP ANTIGEN)//1.60E-30//127aa//40%//P43366
C-HEMBA1000919//HYPOTHETICAL 65.5 KD TRP-ASP REPEATS CONTAINING PROTEIN F02E8.5 IN CHROMOSOME X.//1.00E-10//288aa//23%//Q19124
C-HEMBA1001019//CELL DIVISION CONTROL PROTEIN 2 HOMOLOG (EC 2.7.1.-) (P34 PROTEIN KINASE) (CYCLIN-DEPENDENT KINASE 1) (CDK1).//3.10E-10//70aa//58%//P06493
C-HEMBA1001043//ANKYRIN, BRAIN VARIANT 2 (ANKYRIN B) (ANKYRIN, NONERYTHROID)(FRAGMENT).//1.40E-12//131aa//38%//Q01485
C-HEMBA1001059//Human N-acetylgalactosamine 6-sulphatase (GALNS) gene, exon 14.//4.80E-169//786bp//99%//U06088
C-HEMBA1001071//PROCOLLAGEN ALPHA 1(III) CHAIN PRECURSOR.//1.50E-92//82aa//100%//P02461
C-HEMBA1001077//Homo sapiens transcriptional intermediary factor 1 gamma mRNA, complete cds.//2.00E-80//432bp//94%//AF119043
C-HEMBA1001088//PINCH PROTEIN (PARTICULARY INTERESTING NEW CYS-HIS PROTEIN).//3.50E-50//176aa//57%//P48059
C-HEMBA1001137//ZINC FINGER PROTEIN 33A (ZINC FINGER PROTEIN KOX31) (KIAA0065)(HA0946)(FRAGMENT).//1.50E-116//197aa//58%//Q06730
C-HEMBA1001174//ADP-RIBOSYLATION FACTOR-LIKE PROTEIN 5.//6.80E-79//179aa//80%//P51646
C-HEMBA1001197//Homo sapiens rap2 interacting protein x mRNA, complete cds.//0//1511bp//99%//AF112221
C-HEMBA1001257//Homo sapiens mRNA 2-methylacyl-CoA racemase.//0//1672bp//99%//AJ130733
C-HEMBA1001286//COMPLEMENT DECAY-ACCELERATING FACTOR PRECURSOR.//0.00000002//198aa//29%//Q60401
C-HEMBA1001302//Homo sapiens calcium binding protein precursor, mRNA, complete cds.//9.60E-258//682bp//94%//AF153686
C-HEMBA1001351//Homo sapiens VAMP-associated protein of 33 kDa (VAP-33) mRNA, complete cds.//1.40E-133//614bp//99%//AF057358
C-HEMBA1001387//GTP-BINDING PROTEIN TC10.//2.90E-64//104aa//82%//P17081
C-HEMBA1001405//Drosophila melanogaster eyelid (eld) mRNA, complete cds.//5.60E-25//863bp//60%//AF053091
C-HEMBA1001446//Homo sapiens rap2 interacting protein x mRNA, complete cds.//9.20E-55//719bp//68%//AF112221
C-HEMBA1001455//Mus musculus transposon-derived Buster2 transposase-like protein gene, partial cds.//4.20E-290//2008bp//81%//AF205599
C-HEMBA1001476//Human DNA topoisomerase II (top2) mRNA, complete cds.//1.60E-268//1213bp//100%//J04088
C-HEMBA1001510//CYCLIC-AMP-DEPENDENT TRANSCRIPTION FACTOR ATF-6 (FRAGMENT).//1.70E-16//63aa//61%//P18850
C-HEMBA1001526//PERIPLASMIC [FE] HYDROGENASE 1 (EC 1.18.99.1).//4.90E-37//399aa//29%//P29166
C-HEMBA1001569//SYNAPTOBREVIN 2 (VESICLE ASSOCIATED MEMBRANE PROTEIN 2) (VAMP-2).//2.30E-53//110aa//100%//P19065
C-HEMBA1001579//Homo sapiens mRNA for NS1-binding protein (NS1-BP).//0//808bp//97%//AJ012449
C-HEMBA1001595//SEPTIN 2 HOMOLOG (FRAGMENT).//4.90E-156//348aa//83%//Q14141
C-HEMBA1001620//MYO-INOSITOL-1-PHOSPHATE SYNTHASE (EC 5.5.1.4) (IPS).//1.60E-166//506aa//60%//P42803
C-HEMBA1001635//TESTIS SPECIFIC PROTEIN A (ZINC FINGER PROTEIN TSGA).//1.60E-10//155aa//28%//Q63679
C-HEMBA1001651//CYTADHERENCE HIGH MOLECULAR WEIGHT PROTEIN 1 (CYTADHERENCE ACCESSORY PROTEIN 1).//6.20E-07//362aa//24%//Q50365
C-HEMBA1001661//CADHERIN-RELATED TUMOR SUPPRESSOR PRECURSOR (FAT PROTEIN).//4.60E-36//365aa//33%//P33450
C-HEMBA1001672//Homo sapiens methyl-CpG binding domain-containing protein MBD3 (MBD3) mRNA, complete cds.//0//1707bp//98%//AF072247
C-HEMBA1001675//VACUOLAR PROTEIN SORTING-ASSOCIATED PROTEIN VPS9.//5.40E-09//101aa//35%//P54787
C-HEMBA1001714//Homo sapiens mRNA for ATPase inhibitor precursor, complete cds.//3.70E-78//200bp//100%//AB029042
C-HEMBA1001723//Homo sapiens G protein beta subunit mRNA, partial cds.//3.10E-267//1212bp//99%//AF195883
C-HEMBA1001734//CADHERIN-11 PRECURSOR (OSTEOBLAST-CADHERIN) (OBCADHERIN) (OSF-4).//1.10E-38//87aa//96%//P55288
C-HEMBA1001744//SCY1 PROTEIN.//9.90E-32//481aa//25%//P53009
C-HEMBA1001746//Homo sapiens squamous cell carcinoma antigen recognized by T cell (SART-2) mRNA, complete cds.//7.60E-59//998bp//64%//AF098066
C-HEMBA1001804//Homo sapiens zinc finger DNA binding protein 99 (ZNF281) mRNA, complete cds.//0//1637bp//99%//AF125158
C-HEMBA1001809//IMMEDIATE-EARLY PROTEIN IE180.//3.80E-11//206aa//36%//P11675
C-HEMBA1001819//ZINC FINGER PROTEIN 184 (FRAGMENT).//2.90E-135//459aa//52%//Q99676
C-HEMBA1001822//Mus musculus Ese2L protein mRNA, complete cds.//1.90E-235//1329bp//89%//AF132479
C-HEMBA1001824//Homo sapiens nuclear protein NP94 mRNA, complete cds.//1.40E-199//1180bp//89%//AF159025
C-HEMBA1001847//ZINC FINGER PROTEIN 29 (ZFP-29).//7.60E-64//221aa//55%//Q07230
C-HEMBA1001866//UDP-GLUCOSE:GLYCOPROTEIN GLUCOSYLTRANSFERASE PRECURSOR (EC 2.4.1.-) (DUGT).//5.70E-51//234aa//41%//Q09332
C-HEMBA1001869//TRITHORAX PROTEIN.//9.60E-05//166aa//27%//P20659
C-HEMBA1001896//DIMETHYLGLYCINE DEHYDROGENASE PRECURSOR (EC 1.5.99.2) (ME2GLYDH).//9.30E-36//395aa//26%//Q63342
C-HEMBA1001913//GCN20 PROTEIN.//2.30E-81//158aa//50%//P43535
C-HEMBA1001921//Homo sapiens germinal center kinase related protein kinase mRNA, complete cds.//0//1850bp//99%//AF000145
C-HEMBA1001967//Homo sapiens NY-REN-57 antigen mRNA, partial cds.//0//1721bp//99%//AF155114
C-HEMBA1002035//Homo sapiens BAZ1A mRNA for bromodomain adjacent to zinc finger domain 1A, complete cds.//0//2149bp//99%//AB032252
C-HEMBA1002092//Mus musculus Olf-1/EBF-like-3 transcription factor (O/E-3) mRNA, complete cds.//1.30E-271//1583bp//88%//U92703
C-HEMBA1002102//ANKYRIN.//4.40E-10//106aa//35%//Q02357
C-HEMBA1002139//LIM AND SH3 DOMAIN PROTEIN LASP-1 (MLN 50).//7.10E-05//51aa//49%//Q14847
C-HEMBA1002151//Rattus norvegicus p34 mRNA, complete cds.//1.10E-153//1059bp//82%//AF178669
C-HEMBA1002161//MYOSIN HEAVY CHAIN, CARDIAC MUSCLE BETA ISOFORM.//1.40E-51//180aa//56%//P79293
C-HEMBA1002177//TRANSCRIPTION FACTOR GATA-4 (GATA BINDING FACTOR-4).//6.00E-13//190aa//36%//P43694
C-HEMBA1002212//TYROSINE-PROTEIN KINASE-2 (EC 2.7.1.112) (FRAGMENT).//3.00E-17//267aa//29%//P18161
C-HEMBA1002215//TESTIN 2 (TES2) [CONTAINS: TESTIN 1 (TES1)].//2.20E-199//392aa//89%//P47226
C-HEMBA1002241//PROLIFERATING-CELL NUCLEOLAR ANTIGEN P120 (PROLIFERATION-ASSOCIATED NUCLEOLAR PROTEIN P120).//3.70E-06//95aa//33%//P46087
C-HEMBA1002267//Sus scrofa decorin mRNA, complete cds.//1.10E-46//302bp//90%//AF125537
C-HEMBA1002341//P53-BINDING PROTEIN 2 (53BP2) (FRAGMENT).//3.80E-55//109aa//96%//Q62415
C-HEMBA1002363//Homo sapiens chromosome-associated protein-E (hCAP-E) mRNA, complete cds.//0//1847bp//99%//AF092563
C-HEMBA1002417/mGHT JUNCTION PROTEIN ZO-1 (TIGHT JUNCTION PROTEIN 1).//1.00E-121//489aa//52%//P39447
C-HEMBA1002419//TRICHOHYALIN.//1.90E-09//299aa//24%//P22793
C-HEMBA1002458//OVARIAN GRANULOSA CELL 13.0 KD PROTEIN HGR74.//4.20E-24//109aa//55%//Q00994
C-HEMBA1002469//DXS8237E PROTEIN (FRAGMENT).//3.50E-50//199aa//61%//P98175
C-HEMBA1002475//SKIN SECRETORY PROTEIN XP2 PRECURSOR (APEG PROTEIN).//1.10E-12//285aa//31%//P17437
C-HEMBA1002495//LIGHT-MEDIATED DEVELOPMENT PROTEIN DET1.//6.80E-53//257aa//36%//P48732
C-HEMBA1002513//Homo sapiens mRNA for histone deacetylase-like protein (JM21).//0//2432bp//99%//AJ011972
C-HEMBA1002547//Homo sapiens agrin precursor mRNA, partial cds.//0//1605bp//97%//AF016903
C-HEMBA1002555//Homo sapiens mSin3A associated polypeptide p30 mRNA, complete cds.//5.30E-51//768bp//68%//AF055993
C-HEMBA1002569//Homo sapiens protein associated with Myc mRNA, complete cds.//6.80E-305//951bp//99%//AF075587
C-HEMBA1002746//DNA POLYMERASE BETA (EC 2.7.7.7).//5.00E-37//268aa//34%//P06746
C-HEMBA1002768//Mus musculus formin binding protein 17 mRNA, partial cds.//7.80E-237//1522bp//85%//AB011126
C-HEMBA1002770//Rattus norvegicus mRNA for TIP120, complete cds.//2.90E-176//1024bp//88%//D87671
C-HEMBA1002777//Fugu rubripes BAW (BAW) mRNA, complete cdsJ/3.40E-54//319bp//76%//AF153879
C-HEMBA1002810//Homo sapiens formin binding protein 21 mRNA, complete cds.//8.2e-314//1437bp//99%//AF071185
C-HEMBA1002818//Homo sapiens mRNA for fibulin-4.//2.00E-304//1383bp//99%//AJ132819
C-HEMBA1002876//HYPOTHETICAL 26.4 KD PROTEIN EEED8.8 IN CHROMOSOME n.//1.50E-44//188aa//52%//Q09297
C-HEMBA1002935//ZINC FINGER PROTEIN 91 (ZINC FINGER PROTEIN HTF10) (HPF7).//1.30E-15//371aa//25%//Q05481
C-HEMBA1002939//ANKYRIN R (ANKYRINS 2.1 AND 2.2) (ERYTHROCYTE ANKYRIN).//2.00E-34//300aa//34%//P16157
C-HEMBA1002951//NUF1 PROTEIN (SPINDLE POLY BODY SPACER PROTEIN SPC110).//4.40E-06//324aa//24%//P32380
C-HEMBA1002973//CAMP-DEPENDENT 3',5'-CYCLIC PHOSPHODIESTERASE 4B (EC 3.1.4.17) (DPDE4).//1.20E-27//63aa//100%//P14646
C-HEMBA1002997//CENTROMERIC PROTEIN E (CENP-E PROTEIN).//3.80E-25//534aa//24%//Q02224
C-HEMBA1002999//Rattus norvegicus lamina associated polypeptide 1C (LAP1C) mRNA, complete cds.//1.40E-171//1552bp//75%//U20286
C-HEMBA1003046//Homo sapiens mitochondrial processing peptidase beta-subunit mRNA,//0//1558bp//99%//AF054182
C-HEMBA1003071//INSULIN-LIKE GROWTH FACTOR BINDING PROTEIN COMPLEX ACID LABILE CHAIN PRECURSOR (ALS).//1.30E-09//121aa//40%//P35858
C-HEMBA1003077//SLIT PROTEIN PRECURSOR.//2.60E-15//199aa//31%//P24014
C-HEMBA1003096//Mouse 19.5 mRNA, complete cds.//5.60E-117//1139bp//72%//M32486
C-HEMBA1003098//Homo sapiens NY-REN-6 antigen mRNA, partial cds.//6.20E-273//1253bp//99%//AF155096
C-HEMBA1003136//MANNOSE-1-PHOSPHATE GUANYLTRANSFERASE (EC 2.7.7.13) (ATP-MANNOSE-1-PHOSPHATE GUANYLYLTRANSFERASE) (NDP-HEXOSE PYROPHOSPHORYLASE).//8.50E-51//221aa//33%//P41940
C-HEMBA1003148//Homo sapiens mRNA for dachshund protein.//0//1583bp//99%//AJ005670
C-HEMBA1003179//PROBABLE TRNA (5-METHYLAMINOMETHYL-2-THIOURIDYLATE)-METHYLTRANSFERASE (EC 2.1.1.61).//5.90E-74//134aa//53%//P44551
C-HEMBA1003199//Homo sapiens chromosome 5 F-box protein Fbx4 (FBX4) mRNA, complete cds.//8.50E-87//285bp//90%//AF129534
C-HEMBA1003235//TROPOMYOSIN.//2.30E-06//109aa//33%//Q02088
C-HEMBA1003250//PROTEIN KINASE APK1A (EC 2.7.1.-).//7.20E-41//245aa//42%//Q06548
C-HEMBA1003281//POLIOVIRUS RECEPTOR PRECURSOR.//6.00E-11//239aa//32%//P32506
C-HEMBA1003286//Homo sapiens mRNA for beta-1,4-galactosyltransferase IV, complete cds.//5.40E-229//1043bp//99%//AB024436
C-HEMBA1003291//SNF1-RELATED PROTEIN KINASE KIN10 (EC 2.7.1.-) (AKIN10)7/6.20E-28//126aa//51%//Q38997
C-HEMBA1003369//CENTROMERIC PROTEIN E (CENP-E PROTEIN).//2.00E-08//248aa//23%//Q02224
C-HEMBA1003408//DNA-DIRECTED RNA POLYMERASE II LARGEST SUBUNIT (EC 2.7.7.6) (VERSION 1).//7.80E-13//297aa//30%//P18616
C-HEMBA1003417//Homo sapiens BAG-family molecular chaperone regulator-2 mRNA, complete cds.//1.50E-255//1179bp//99%//AF095192
C-HEMBA1003418//TRICHOHYALIN.//8.70E-19//281aa//31%//P37709
C-HEMBA1003433//Homo sapiens gene for NBS1, complete cds.//0//511bp//94%//AB013139
C-HEMBA1003538//COMPLEMENT C1R COMPONENT PRECURSOR (EC 3.4.21.41).//2.40E-110//242aa//58%//P00736
C-HEMBA1003545//INSULIN GENE ENHANCER PROTEIN ISL-2 (TSLET-2).//8.80E-189//360aa//96%//P50480
C-HEMBA1003555//NUCLEOTIDE-BINDING PROTEIN (NBP).//2.10E-68//251aa//52%//P53384
C-HEMBA1003560//GUANINE NUCLEOTIDE-BINDING PROTEIN G(I)/G(S)/G(O) GAMMA-2 SUBUNIT (G GAMMA-I).//1.20E-31//71aa//100%//P16874
C-HEMBA1003568//52 KD RO PROTEIN (SJOGREN SYNDROME TYPE A ANTIGEN (SS-A))(RO(SS-A)).//7.90E-49//279aa//32%//P19474
C-HEMBA1003569//METASTASIS-ASSOCIATED PROTEIN MTA1.//6.90E-206//445aa//74%//Q13330
C-HEMBA1003581//TALIN.//4.40E-45//52aa//98%//P26039
C-HEMBA1003591//CHLOROPLAST 28 KD RIBONUCLEOPROTEIN PRECURSOR (28RNP).//4.40E-10//118aa//35%//P19682
C-HEMBA1003615//Homo sapiens ART-4 mRNA, complete cds.//0//1713bp//99%//AB026125
C-HEMBA1003617//Homo sapiens ubiquitin-like product Chap1/Dsk2 mRNA, complete cds.//6.90E-178//501bp//97%//AB015344
C-HEMBA1003645//TIPD PROTEIN.//2.40E-10//289aa//23%//O15736
C-HEMBA1003662//TBX2 PROTEIN (T-BOX PROTEIN 2).//1.20E-75//151aa//99%//Q13207
C-HEMBA1003679//SIALIDASE (EC 3.2.1.18) (NEURAMINIDASE) (NA) (MAJOR SURFACE ANTIGEN).//1.00E-09//611aa//22%//P23253
C-HEMBA1003680//PUTATIVE AMINOPEPTIDASE ZK353.6 IN CHROMOSOME III (EC 3.4.11.-).//2.40E-92//423aa//47%//P34629
C-HEMBA1003684//ZINC FINGER PROTEIN 151 (MIZ-1 PROTEIN).//2.00E-73//526aa//32%//Q13105
C-HEMBA1003690//HISTONE DEACETYLASE HDA1.//2.10E-59//249aa//47%//P53973
C-HEMBA1003742//Homo sapiens cleft lip and palate transmembrane protein 1 (CLPTM1) mRNA, complete cds.//1.70E-44//501bp//67%//AF037339
C-HEMBA1003760//HYPOXIA-INDUCIBLE FACTOR 1 ALPHA (HIF-1 ALPHA) (ARNT INTERACTING PROTEIN) (MEMBER OF PAS PROTEIN 1) (MOP1) (HIF1 ALPHA).//3.70E-124//347aa//55%//Q16665
C-HEMBA1003773//Mus musculus signal recognition particle receptor beta subunit mRNA, complete cds.//5.80E-81//511bp//86%//U17343
C-HEMBA1003783//Mus musculus bromodomain-containing protein BP75 mRNA, complete cds.//1.10E-190//1204bp//84%//AF084259
C-HEMBA1003805//Mus musculus KH domain RNA binding protein QKI-5A mRNA, complete cds.//0//988bp//95%//AF090402
C-HEMBA1003836//MOB1 PROTEIN (MPS1 BINDER 1).//8.10E-31//134aa//52%//P40484
C-HEMBA1003866//Mus musculus semaphorin VIa mRNA, complete cds.//1.20E-105//1192bp//70%//AF030430
C-HEMBA1003953//ZINC FINGER PROTEIN MFG-1 (ZINC FINGER PROTEIN 58) (FRAGMENT).//3.80E-16//89aa//46%//P16372
C-HEMBA1004097//Mus musculus putative transcription factor mRNA, complete cds.//8.50E-221//1188bp//78%//AF091234
C-HEMBA1004131//SEPTIN 2 HOMOLOG (FRAGMENT).//1.60E-166//416aa//72%//Q14141
C-HEMBA1004168//Homo sapiens geminin mRNA, complete cds.//3.90E-208//951 bp//99%//AF067855
C-HEMBA1004199//HYPOTHETICAL HELICASE K12H4.8 IN CHROMOSOME III.//8.40E-60//243aa//39%//P34529
C-HEMBA1004202//RAS-RELATED PROTEIN RAB-13.//6.20E-30//208aa//37%//P51153
C-HEMBA1004203//NUCLEOLAR PROTEIN NOP2.//1.50E-12//258aa//29%//P40991
C-HEMBA1004207//Homo sapiens leptin receptor short form (db) mRNA, complete cds.//0//1892bp//99%//U50748
C-HEMBA1004227//Rattus norvegicus protein phosphatase 2C mRNA, complete cds.//5.70E-217//1217bp//88%//AF095927
C-HEMBA1004248//INSULIN-INDUCED GROWTH RESPONSE PROTEIN CL-6 (IMMEDIATE-EARLY PROTEIN CL-6).//2.00E-43//98aa//84%//Q08755
C-HEMBA1004275//Homo sapiens PHD-finger protein (GRC5) mRNA, complete cds.//1.10E-152//1403bp//69%//AF043725
C-HEMBA1004276//Homo sapiens AP-4 adaptor complex beta4 subunit mRNA, complete cds.//4.80E-257//738bp//99%//AF092094
C-HEMBA1004286//Homo sapiens TGF beta receptor associated protein-1 mRNA, complete cds.//0//1982bp//99%//AF022795
C-HEMBA1004295//Homo sapiens NY-REN-25 antigen mRNA, partial cds.//9.40E-31//381bp//65%//AF155103
C-HEMBA1004321//ZINC FINGER PROTEIN 184 (FRAGMENT).//2.30E-93//357aa//42%//Q99676
C-HEMBA1004353//C-MYC BINDING PROTEIN MM-1.//3.00E-71//89aa//96%//Q99471
C-HEMBA1004354//CHL1 PROTEIN.//9.90E-26//130aa//42%//P22516
C-HEMBA1004356//H.sapiens MSSP-2 mRNA.//3.00E-243//573bp//98%//X77494
C-HEMBA1004389//Homo sapiens zinc finger DNA binding protein 99 (ZNF281) mRNA, complete cds.//0//1437bp//99%//AF125158
C-HEMBA1004408//PEPTIDYL-PROLYL CIS-TRANS ISOMERASE 10 (EC 5.2.1.8) (PPIASE) (ROTAMASE) (CYCLOPHILIN-10).//3.20E-32//148aa//52%//P52017
C-HEMBA1004479//HYPOXIA-INDUCIBLE FACTOR 1 ALPHA (HIF-1 ALPHA) (ARNT INTERACTING PROTEIN).//3.10E-51//152aa//40%//Q61221
C-HEMBA1004499//Homo sapiens delta-tubulin mRNA, complete cds.//3.40E-92//483bp//95%//AF201333
C-HEMBA1004509//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME ,4) (UBIQUITOUS NUCLEAR PROTEIN HOMOLOG).//2.70E-12//200aa//28%//Q13107
C-HEMBA1004534//Homo sapiens gamma-filamin (ABPL) mRNA, complete cds.//1.2e-316//1445bp//99%//AF089841
C-HEMBA1004573//Homo sapiens mRNA for HELG protein.//2.00E-59//483bp//68%//AJ277291
C-HEMBA1004604//Homo sapiens COP9 complex subunit 7a mRNA, complete cds.//0//1612bp//99%//AF193844
C-HEMBA1004669//SON PROTEIN (SON3).//7.30E-17//288aa//36%//P18583
C-HEMBA1004697//MYOSIN HEAVY CHAIN, SMOOTH MUSCLE ISOFORM (SMMHC) (FRAGMENT).//2.90E-05//303aa//21%//P35749
C-HEMBA1004734//UBIQUITIN-CONJUGATING ENZYME E2-18 KD (EC 6.3.2.19) (UBIQUITIN- PROTEIN LIGASE) (UBIQUTTIN CARRIER PROTEIN) (PM42).//9.90E-39//143aa//52%//P42743
C-HEMBA1004752//Homo sapiens mRNA for LAK-4p, complete cds.//4.60E-109//650bp//89%//AB002405
C-HEMBA1004756//Human transporter protein (g17) mRNA, complete cds.//9.10E-34//515bp//66%//U49082
C-HEMBA1004758//Homo sapiens transcription factor SL1 mRNA, complete cds.//2.60E-246//1249bp//94%//L39060
C-HEMBA1004768//LINE-1 REVERSE TRANSCRIPTASE HOMOLOG.//5.40E-111//314aa//58%//P08547
C-HEMBA1004795//CDC4-UKE PROTEIN (FRAGMENT).//3.80E-69//198aa//66%//P50851
C-HEMBA1004847//SIGNAL RECOGNITION PARTICLE 68 KD PROTEIN (SRP68).//8.20E-154//317aa//94%//Q00004
C-HEMBA1004889//Human C3f mRNA, complete cds.//6.70E-24//341aabp//26%//U72515
C-HEMBA1004929//INTRACELLULAR PROTEIN TRANSPORT PROTEIN USO1.//2.50E-05//148aa//24%//P25386
C-HEMBA1004930//26S PROTEASOME SUBUNIT S5B (KIAA0072) (HA1357).//3.30E-27//65aa//100%//Q16401
C-HEMBA1004972//NEUROFILAMENT TRIPLET H PROTEIN (200 KD NEURO FILAMENT PROTEIN) (NF-H).//0.00000096//286aa//23%//P12036
C-HEMBA1004973//ZINC-BINDING PROTEIN A337/4.10E-08//121aa//33%//Q02084
C-HEMBA1005009//Homo sapiens BAF53a (BAF53a) mRNA, complete cds.//0//1813bp//99%//AF041474
C-HEMBA1005029//Homo sapiens CGI-13 protein mRNA, complete cds.//0//1487bp//99%//AF132947
C-HEMBA1005047//RAS-RELATED PROTEIN RAB-24 (RAB-16).//3.40E-101//106aa//98%//P35290
C-HEMBA1005101//Homo sapiens SYT interacting protein SIP mRNA, complete cds.//0//2762bp//99%//AF080561
C-HEMBA1005201//Homo sapiens CGI-07 protein mRNA, complete cds.//0//1608bp//99%//AF132941
C-HEMBA1005202//SIGNAL RECOGNITION PARTICLE 68 KD PROTEIN (SRP68).//1.90E-179//361aa//95%//Q00004
C-HEMBA1005206//Drosophila simulans anon73Bl gene and Su(P) gene.//1.90E-11//376bp//63%//AJ250308
C-HEMBA1005219//NUCLEAR PROTEIN SNF7.//5.30E-10//189aa//25%//P39929
C-HEMBA1005338//Homo sapiens mRNA for matrilin-4, partial.//3.90E-241//1095bp//99%//AJ007581
C-HEMBA1005359//ZINC FINGER PROTEIN 137.//3.90E-85//206aa//69%//P52743
C-HEMBA1005367//Homo sapiens melastatin 1 (MLSN1) mRNA, complete cds.//9.00E-77//620bp//74%//AF071787
C-HEMBA1005394//Mus musculus pantothenate kinase 1 beta (panKlbeta) mRNA, complete cds.//3.90E-126//1097bp//75%//AF200357
C-HEMBA1005423//Homo sapiens cyclin-dependent kinase inhibitor (CDKN2C) mRNA, complete cds.//2.00E-213//537bp//99%//AF041248
C-HEMBA1005513//MALES-ABSENT ON THE FIRST PROTEIN (EC 2.3.1.-).//1.90E-129//332aa//61%//O02193
C-HEMBA1005528//CCR4-ASSOCIATED FACTOR 1 (CAF1).//3.10E-154//285aa//99%//Q60809
C-HEMBA1005530//Homo sapiens anaphase-promoting complex subunit 7 (APC7) mRNA, complete cds.//0//1578bp//98%//AF191340
C-HEMBA1005548//Homo sapiens MAFB/Kreisler basic region/leucine zipper transcription factor (MAFB) mRNA, complete cds.//1.00E-220//1014bp//99%//AF134157
C-HEMBA1005558//NUCLEAR PROTEIN SNF7.//6.40E-16//170aa//31%//P39929
C-HEMBA1005576//Mus musculus mRNA for plexin 2, complete cds.//1.20E-122//870bp//82%//D86949
C-HEMBA1005581//Homo sapiens SLIT2 (SLIL2) mRNA, complete cds.//0//1721bp//100%//AF133270
C-HEMBA1005582//TROPOMYOSIN 1, NON-MUSCLE ISOFORM (TROPOMYOSIN II) (CYTOSKELETAL TROPOMYOSIN).//0.00000009//213aa//27%//P09492
C-HEMBA1005595//DYNEIN HEAVY CHAIN, CYTOSOLIC (DYHC).//2.30E-54//562aa//29%//P34036
C-HEMBA1005621//Homo sapiens Mad2-like protein mRNA, complete cds.//8.00E-211//962bp//99%//AF072933
C-HEMBA1005666//Homo sapiens mRNA for DIPB protein.//8.60E-147//685bp//99%//AJ249128
C-HEMBA1005699//EPHRIN-B3 PRECURSOR (EPH-RELATED RECEPTOR TYROSINE KINASE LIGAND 8) (LERK-8) (EPH-RELATED RECEPTOR TRANSMEMBRANE LIGAND ELK-L3).//2.10E-37//98aa//81 %//Q15768
C-HEMBA1005737//CALCINEURIN B SUBUNIT (PROTEIN PHOSPHATASE 2B REGULATORY SUBUNIT).//4.40E-17//167aa//34%//P25296
C-HEMBA1005815//CALPAIN, LARGE [CATALYTIC] SUBUNIT (EC 3.4.22.17) (CALCIUM- ACTIVATED NEUTRAL PROTEINASE) (CANP) (MU/M-TYPE).//2.00E-36//342aa//33%//P00789
C-HEMBA1005931//ZINC FINGER PROTEIN 83 (ZINC FINGER PROTEIN HPF1).//5.60E-15//76aa//51%//P51522
C-HEMBA1005990//Homo sapiens I-1 receptor candidate protein mRNA, complete cds.//0//2371bp//100%//AF082516
C-HEMBA1006031//Homo sapiens mRNA for putative phospholipase, complete cds.//0//1413bp//99%//AB019435
C-HEMBA1006038//LAMININ ALPHA-5 CHAIN (FRAGMENT).//3.10E-33//81aa//64%//Q61001
C-HEMBA1006067//Homo sapiens squamous cell carcinoma antigen recognized by T cell (SART-2) mRNA, complete cds.//8.20E-12//297bp//64%//AF098066
C-HEMBA1006130//SEL-10 PROTEIN.//0.000000043//219aa//25 %//Q93794
C-HEMBA1006158//Homo sapiens transcription factor forkhead-like 7 (FKHL7) gene, complete cds.//0//155 lbp//99%//AF048693
C-HEMBA1006198//PROLINE-RICH PROTEIN MP-2 PRECURSOR.//1.90E-19//215aa//39%//P05142
C-HEMBA1006248//ZINC FINGER PROTEIN MFG-1 (ZINC FINGER PROTEIN 58) (FRAGMENT).//8.60E-23//151aa//37%//P16372
C-HEMBA1006253//DNA-DAMAGE-REPAIR/TOLERATION PROTEIN DRT111 PRECURSOR.//0.00000002//62aa//53%//P42698
C-HEMBA1006268//Homo sapiens HQ0024c mRNA, complete cds.//3.50E-157//845bp//92%//AF073836
C-HEMBA1006272//RETROVIRUS-RELATED PROTEASE (EC 3.4.23.-).//1.30E-123//200aa//73%//P10265
C-HEMBA1006278//POLY(A) POLYMERASE (EC 2.7.7.19) (PAP) (POLYNUCLEOTIDE ADENYLYLTRANSFERASE).//1.00E-210//490aa//77%//P25500
C-HEMBA1006283//NUCLEAR POLYADENYLATED RNA-BINDING PROTEIN NAB2.//0.000000012//176aa//30%//P32505
C-HEMBA1006291//2-ARYLPROPIONYL-COA EPIMERASE (EC 5.-.-.-).//4.20E-12//215aa//23%//P70473
C-HEMBA1006309//Homo sapiens aspartyl aminopeptidase mRNA, complete cds.//5.30E-169//774bp//100%//AF005050
C-HEMBA1006310//Rattus norvegicus cytosolic sorting protein PACS-1a (PACS-1) mRNA, complete cds.//3.70E-225//1189bp//88%//AF076183
C-HEMBA1006344//RADIXIN.//1.50E-31//333aa//28%//P26043
C-HEMBA1006347//MALES-ABSENT ON THE FIRST PROTEIN (EC 2.3.1.-).//1.60E-130//332aa//62%//O02193
C-HEMBA1006359//ZINC FINGER PROTEIN 43 (ZINC PROTEIN HTF6).//3.50E-105//381aa//54%//P28160
C-HEMBA1006398//Human L1 element L1.6 putative pi 50 gene, complete cds.//2.00E-277//1729bp//85%//U93563
C-HEMBA1006445//Homo sapiens putative tumor supressor NOEY2 mRNA, complete cds.//1.40E-270//1224bp//100%//U96750
C-HEMBA1006474//40 KD PROTEIN.//1.40E-39//292aa//34%//Q01552
C-HEMBA1006485//PUROMYCIN-SENSITIVE AMINOPEPTIDASE (EC 3.4.11.-) (PSA).//1.90E-81//153aa//97%//P55786
C-HEMBA1006507//DIAPHANOUS PROTEIN HOMOLOG 2.//1.40E-46//316aa//32%//O60879
C-HEMBA1006521//3-OXOACYL-[ACYL-CARRIER PROTEIN] REDUCTASE (EC 1.1.1.100) (3-KETOACYL- ACYL CARRIER PROTEIN REDUCTASE).//4.00E-33//177aa//42%//P25716
C-HEMBA1006559//Mus musculus PRAJA1 (Praja1) mRNA, complete cds.//2.80E-206//1107bp//83%//U06944
C-HEMBA1006583//Drosophila melanogaster Scribble (scrib) mRNA, complete cds.//1.70E-63//1002bp//65%//AF190774
C-HEMBA1006624//DNA/PANTOTHENATE METABOLISM FLAVOPROTEIN HOMOLOG.//0.00000069//109aa//38%//Q58323
C-HEMBA1006650//ARP2/3 COMPLEX 20 KD SUBUNIT (P20-ARC).//9.00E-40//113aa//82%//O15509
C-HEMBA1006652//60S RIBOSOMAL PROTEIN L7.//2.40E-44//206aa//47%//P14148
C-HEMBA1006708//HYPOTHETICAL 46.4 KD TRP-ASP REPEATS CONTAINING PROTEIN IN PMC1-TFG2 INTERGENIC REGION.//3.30E-22//241aa//31%//P53196
C-HEMBA1006737//ANKYRIN, BRAIN VARIANT 2 (ANKYRIN B) (ANKYRIN, NONERYTHROID) (FRAGMENT).//0.000000043//111aa//40%//Q01485
C-HEMBA1006758//Homo sapiens protocadherin beta 13 (PCDH-beta13) mRNA, complete cds.//0//1832bp//91%//AF152492
C-HEMBA1006807//Homo sapiens mRNA for SPOP.//5.70E-125//1109bp//75%//AJ000644
C-HEMBA1006877//OXYSTEROL-BINDINGPROTEIN.//2.00E-59//378aa//39%//P16258
C-HEMBA1006885//Homo sapiens gene for Proline synthetase associated, complete cds.//0//1467bp//96%//AB018566
C-HEMBA1006914//Human anthracycline-associated resistance ARX mRNA, complete cds.//0//1837bp//99%//U35832
C-HEMBA1006941//Homo sapiens PKCq-interacting protein PICOT (PICOT) mRNA, complete cds.//2.10E-271//1234bp//99%//AF118649
C-HEMBA1006973//Homo sapiens rab3-GAP regulatory domain mRNA, complete cds.//5.60E-143//740bp//94%//AF004828
C-HEMBA1006976//H.sapiens mRNA for Gal-beta(1-3/l-4)GlcNAc alpha-2.3-sialyltransferase.//1.90E-80//447bp//89%//X74570
C-HEMBA1007018//DYNEIN LIGHT INTERMEDIATE CHAIN 1, CYTOSOLIC (UC57/59) (DYNEIN LIGHT CHAIN A) (DLC-A).//2.40E-188//391aa//89%//Q90828
C-HEMBA1007087//CLEAVAGE AND POLYADENYLATION SPECIFICITY FACTOR, 100 KD SUBUNIT (CPSF 100 KD SUBUNIT).//8.30E-27//253aa//30%//Q10568
C-HEMBA1007121//Homo sapiens bisphosphate 3'-nucleotidase mRNA, complete cds.//1.70E-252//1118bp//92%//AF125042
C-HEMBA1007151//Homo sapiens synphilin 1 mRNA, complete cds.//0//1900bp//99%//AF076929
C-HEMBA1007174//Homo sapiens epsin 2b mRNA, complete cds.//3.80E-271//642bp//99%//AF062085
C-HEMBA1007194//Homo sapiens origin recognition complex subunit 6 (ORC6) mRNA, complete cds.//0//1588bp//99%//AF139658
C-HEMBA1007224//Homo sapiens SUMO-1-specific protease (SSP1) mRNA, complete cds.//0//1590bp//99%//AF196304
C-HEMBA1007243//Chinese hamster hprt mRNA, complete cds.//2.00E-58//650bp//70%//J00060
C-HEMBA1007251//Homo sapiens F-box protein FBX29 (FBX29) mRNA, partial cds.//5.00E-58//330bp//95%//AF176707
C-HEMBA1007300//Homo sapiens 3',5'-cyclic nucleotide phosphodiesterase 10A1 (PDE10A) mRNA, splice variant 1, complete cds.//0//1519bp//99%//AF127479
C-HEMBA1007301//COLLAGEN ALPHA 1(III) CHAIN (FRAGMENT).//6.20E-18//115aa//33%//P13941
C-HEMBB1000036//Homo sapiens CGI-51 protein mRNA, complete cds.//0//1665bp//99%//AF151809
C-HEMBB1000037//Homo sapiens erythroblast macrophage protein EMP mRNA, complete cds.//2.80E-187//1582bp//80%//AF084928
C-HEMBB1000083//MYOSIN LIGHT CHAIN KINASE, SMOOTH MUSCLE AND NON-MUSCLE ISOZYMES (EC 2.7.1.117) (MLCK) [CONTAINS: TELOKIN].//1.90E-22//426aa//25%//P11799
C-HEMBB1000119//Homo sapiens ASMTL gene.//0//1891bp//99%//Y15521
C-HEMBB1000144//GUANYLATE CYCLASE ACTIVATING PROTEIN 2 (GCAP 2) (RETINAL GUANYLYL CYCLASE ACTIVATOR PROTEIN P24).//1.40E-24//71aa//77%//P51177
C-HEMBB1000217//Homo sapiens SUMO-1-activating enzyme E1 N subunit (SUA1) mRNA, complete cds.//0//1038bp//99%//AF090385
C-HEMBB1000226//PUTATIVE PRE-MRNA SPLICING FACTOR ATP-DEPENDENT RNA HELICASE EEEDB.5.//2.70E-12//112aa//47%//Q09530
C-HEMBB1000264//CHL1 PROTEIN.//9.50E-19//104aa//45%//P22516
C-HEMBB1000266//HYPOTHETICAL 54.5 KD TRP-ASP REPEATS CONTAINING PROTEIN ZC302.2 IN CHROMOSOME V.//6.10E-09//242aa//26%//Q23256
C-HEMBB1000317//FIBULIN-1, ISOFORM D PRECURSOR.//7.10E-62//458aa//35%//P37888
C-HEMBB1000593//Homo sapiens transfemn receptor 2 alpha (TFR2) mRNA, complete cds.//1.30E-107//503bp//99%//AF067864
C-HEMBB1000631//LONGEVITY-ASSURANCE PROTEIN 1 (LONGEVITY ASSURANCE FACTOR 1).//4.10E-19//232aa//28%//P78970
C-HEMBB1000632//GUANINE NUCLEOTIDE RELEASING PROTEIN (GNRP).//2.20E-28//273aa//31%//P27671
C-HEMBB1000693//Homo sapiens neuroan1 mRNA, complete cds.//0//2952bp//94%//AF040723
C-HEMBB1000725//Rattus norvegicus GTPase Rab8b (Rab8b) mRNA, complete cds.//6.20E-130//692bp//93%//U53475
C-HEMBB1000763//Homo sapiens CGI-89 protein mRNA, complete cds.//0//1676bp//96%//AF151847
C-HEMBB1000781//Homo sapiens mitogen-activated protein kinase kinase kinase MEKK2 mRNA, complete cds.//1.20E-126//613bp//97%//AF111105
C-HEMBB1000789//PUTATIVE 90.2 KD ZINC FINGER PROTEIN IN CCA1-ADK2 INTERGENIC REGION.//5.10E-54//232aa//43%//P39956
C-HEMBB1000831//Homo sapiens breast cancer nuclear receptor-binding auxiliary protein (BRX) mRNA, complete cds.//5.80E-60//301bp//99%//AF126008
C-HEMBB1000915//SUBTILISIN-LIKE PROTEASE PACE4 PRECURSOR (EC 3.4.21.-).//1.10E-08//129aa//31%//P29122
C-HEMBB1000927//Homo sapiens A-type potassium channel modulatory protein 2 (KCHIP2) mRNA, complete cds.//1.30E-126//592bp//99%//AF199598
C-HEMBB1000947//Homo sapiens clone HAW100 putative ribonuclease III mRNA, complete cds.//0//2292bp//99%//AF116910
C-HEMBB1000973//Mus musculus schlafen3 (Slfn3) mRNA, complete cds.//3.40E-120//580bp//67%//AF099974
C-HEMBB1000985//MIPP PROTEIN (MURINE IAP-PROMOTED PLACENTA-EXPRESSED PROTEIN).//8.60E-18//178aa//30%//P28575
C-HEMBB1001011//ZINC FINGER PROTEIN 84 (ZINC FINGER PROTEIN HPF2).//1.40E-73//230aa//45%//P51523
C-HEMBB1001056//PROLIFERATING-CELL NUCLEOLAR ANTIGEN P120 (PROLIFERATION-ASSOCIATED NUCLEOLAR PROTEIN P120).//2.90E-19//264aa//34%//P46087
C-HEMBB1001058//Homo sapiens neuronal thread protein AD7c-NTP mRNA, complete cds//3.60E-52//331bp//80%//AF010144
C-HEMBB1001068//Homo sapiens liprin-beta2 mRNA, partial cds.//2.40E-307//1447bp//97%//AF034803
C-HEMBB1001112//Homo sapiens sec61 homolog mRNA, complete cds.//6.00E-145//961 bp//83 %//AF077032
C-HEMBB1001137//Homo sapiens mRNA for putative phospholipase, complete cds.//0//3069bp//99%//AB019435
C-HEMBB1001151//Rattus norvegicus golgi stacking protein homolog GRASP55 mRNA, complete cds.//4.20E-210//1835bp//76%//AF110267
C-HEMBB1001175//ANKYRIN.//7.00E-11//169aa//31%//Q02357
C-HEMBB1001234//65 KD YES-ASSOCIATED PROTEIN (YAP65).//5.40E-93//196aa//54%//P46938
C-HEMBB1001242//Homo sapiens topoisomerase-related function protein (TRF4-2) mRNA, partial cds.//1.80E-284//713bp//100%//AF089897
C-HEMBB1001282//ANKYRIN R (ANKYRINS 2.1 AND 2.2) (ERYTHROCYTE ANKYRIN).//7.00E-43//394aa//34%//P16157
C-HEMBB1001288//COPPER HOMEOSTASIS PROTEIN CUTC.//7.80E-46//163aa//51%//P46719
C-HEMBB1001294//GTP-BINDING PROTEIN TC10.//1.20E-79//196aa//80%//P17081
C-HEMBB1001314//Mus musculus Olf-1/EBF-like-3 transcription factor (O/E-3) mRNA, complete cds.//1.30E-129//724bp//86%//U92703
C-HEMBB1001331//Mus musculus mRNA for hepatoma-derived growth factor, complete cds, strain:BALB/c.//2.10E-65//458bp//79%//D63850
C-HEMBB1001339//DXS8237E PROTEIN (FRAGMENT).//4.60E-06//124aa//37%//P98175
C-HEMBB1001346//Homo sapiens phenylalanine-tRNA synthetase (FARS1) mRNA, nuclear gene encoding mitochondrial protein, complete cds.//1.10E-58//292bp//99%//AF097441
C-HEMBB1001384//Homo sapiens COP9 complex subunit 4 mRNA, complete cds.//0//1586bp//99%//AF100757
C-HEMBB1001429//Homo sapiens leucine aminopeptidase mRNA, complete cds.//0//1933bp//99%//AF061738
C-HEMBB1001443//Rattus norvegicus pyruvate dehydrogenase phosphatase isoenzyme 1 mRNA, complete cds.//3.00E-130//553bp//86%//AF062740
C-HEMBB1001482//ZINC FINGER PROTEIN 91 (ZINC FINGER PROTEIN HTF10) (HPF7).//2.10E-57//941aa//27%//Q05481
C-HEMBB1001562//CYLICIN II (MULTIPLE-BAND POLYPEPTIDE II).//1.40E-06//373aa//21%//Q28092
C-HEMBB1001564//VACUOLAR ATP SYNTHASE SUBUNIT H (EC 3.6.1.34) (V-ATPASE H SUBUNIT) (V-ATPASE M9.2 SUBUNIT) (9.2 KD MEMBRANE ACCESSORY PROTEIN).//9.60E-32//80aa//78%//O15342
C-HEMBB1001673//Homo sapiens gene for new zinc finger protein, complete cds.//0//1919bp//99%//AB012770
C-HEMBB1001736//EUKARYOTIC TRANSLATION INITIATION FACTOR 3 SUBUNIT 9 (EIF3 P116) (EIF3 P110).//4.60E-15//391aa//25%//P55884
C-HEMBB1001749//TRANSCRIPTIONAL ACTIVATOR GCN5.//1.70E-16//84aa//47%//Q03330
C-HEMBB1001802//Human desmin mRNA, complete cds.//0//1523bp//98%//U59167
C-HEMBB1001831//Homo sapiens PAM COOH-terminal interactor protein 1 (PCIP1) mRNA, complete cds.//0//1514bp//99%//AF056209
C-HEMBB1001839//GASTRULA ZINC FINGER PROTEIN XLCGF42.1 (FRAGMENT).//6.90E-11//87aa//35%//P18720
C-HEMBB1001871//BONE/CARTILAGE PROTEOGLYCAN I PRECURSOR (BIGLYCAN) (PG-S1).//5.40E-75//241aa//48%//P47853
C-HEMBB1001872//CELL SURFACE GLYCOPROTEIN EMR1 PRECURSOR (EMR1 HORMONE RECEPTOR) (CELL SURFACE GLYCOPROTEIN F4/80).//1.90E-22//210aa//27%//Q61549
C-HEMBB1001905//TRICHOHYALIN.//2.10E-10//268aa//27%//P37709
C-HEMBB1001908//Human monocytic leukaemia zinc finger protein (MOZ) mRNA, complete cds.//1.60E-131//874bp//86%//U47742
C-HEMBB1001915//UBIQUITIN CARBOXYL-TERMINAL HYDROLASE 64E (EC 3.1.2.15) (UBIQUITIN THIOLESTERASE 64E) (UBIQUITIN-SPECIFIC PROCESSING PROTEASE 64E) (DEUBIQUITINATING ENZYME 64E).//6.90E-132//561aa//50%//Q24574
C-HEMBB1001950//PROBABLE OXYGEN-INDEPENDENT COPROPORPHYRINOGEN III OXIDASE (EC 1.-.-.-) (COPROPORPHYRINOGENASE) (COPROGEN OXIDASE).//1.60E-41//370aa//31%//P54304
C-HEMBB1002042//CYTOCHROME P450 4C1 (EC 1.14.14.1) (CYPIVC1).//2.70E-49//139aa//55%//P29981
C-HEMBB1002044//Mus musculus mRNA for vascular cadherin-2.//0//3562bp//81%//Y08715
C-HEMBB1002134//ZINC-FINGER PROTEIN NEURO-D4.//8.10E-56//176aa//67%//P56163
C-HEMBB1002193//TYROSINE-PROTEIN KINASE RECEPTOR TYRO3 PRECURSOR (TYROSINE-PROTEIN KINASE RSE) (TYROSINE-PROTEIN KINASE DTK) (TK19-2).//8.70E-61//77aa//74%//P55144
C-HEMBB1002217//ZINC FINGER PROTEIN 91 (ZINC FINGER PROTEIN HTF10) (HPF7).//2.10E-132//399aa//44%//Q05481
C-HEMBB1002266//NEURONAL PROTEIN.//2.10E-46//121aa//76%//P41737
C-HEMBB1002342//Homo sapiens PKCq-interacting protein PICOT (PICOT) mRNA, complete cds.//1.50E-229//1045bp//99%//AF118649
C-HEMBB1002442//LIN-10 PROTEIN.//9.70E-14//121aa//31%//P34692
C-HEMBB1002477//Human Grb2-associated binder-1 mRNA, complete cds.//7.70E-258//774bp//99%//U43885
C-HEMBB1002510//GYP7 PROTEIN.//3.10E-50//192aa//42%//P48365
C-HEMBB1002550//HYPOTHETICAL UOG-1 PROTEIN.//5.00E-28//266aa//33%//P27544
C-HEMBB1002600//Homo sapiens tetraspan NET-5 mRNA, complete cds.//0//1417bp//99%//AF089749
C-HEMBB1002607//Homo sapiens vitamin D3 receptor interacting protein (DRIP80) mRNA, complete cds.//2.00E-136//660bp//98%//AF105421
C-HEMBB1002705//Homo sapiens CGI-27 protein mRNA, complete cds.//7.80E-285//841bp//96%//AF132961
C-MAMMA1000020//H.sapiens mRNA for flavin-containing monooxygenase 5 (FMO5).//8.20E-198//868bp//99%//Z47553
C-MAMMA1000045//ENV POLYPROTEIN [CONTAINS: SURFACE PROTEIN GP85; MEMBRANE PROTEIN GP37].//1.90E-07//249aa//27%//P03396
C-MAMMA1000055//TESTIN 2 (TES2) [CONTAINS: TESTIN 1 (TES1)].//1.50E-90//323aa//48%//P47226
C-MAMMA1000085//PUTATIVE CYSTEINYL-TRNA SYNTHETASE C29E6.06C (EC 6.1.1.16) (CYSTEINE-- TRNA LIGASE) (CYSRS).//2.10E-90//427aa//39%//Q09860
C-MAMMA1000173//Homo sapiens src homology 3 domain-containing protein HIP-55 mRNA, complete cds.//2.60E-164//1044bp//87%//AF197060
C-MAMMA1000183//ZINC FINGER PROTEIN 84 (ZINC FINGER PROTEIN HPF2).//3.40E-134//359aa//63%//P51523
C-MAMMA1000284//P.walti mRNA for rnp associated protein 55.//2.20E-109//864bp//76%//X99836
C-MAMMA1000388//Homo sapiens UKLF mRNA for ubiquitous Kruppel like factor, complete cds.//0//1466bp//99%//AB015132
C-MAMMA1000416//HYPOTHETICAL 32.0 KD PROTEIN C09F5.2 IN CHROMOSOME III.//2.00E-30//119aa//53%//Q09232
C-MAMMA1000612//Homo sapiens G protein beta subunit mRNA, partial cds.//8.30E-178//1992bp//84%//AF195883
C-MAMMA1000625//GYP7 PROTEIN.//2.10E-41//198aa//40%//P48365
C-MAMMA1000672//VITELLOGENIC CARBOXYPEPTIDASE PRECURSOR (EC 3.4.16.).//4.40E-33//250aa//33%//P42660
C-MAMMA1000684//Homo sapiens opioid growth factor receptor mRNA, complete cds.//0//2391bp//99%//AF172451
C-MAMMA1000713//L-RBULOKINASE (EC 2.7.1.16).//7.70E-17//246aa//29%//P94524
C-MAMMA1000731//CHROMODOMAIN-HELICASE-DNA-BINDING PROTEIN 1 (CHD-1).//1.00E-77//395aa//45%//O14646
C-MAMMA1000734//Homo sapiens mRNA for SEC63 protein.//0//1587bp//99%//AJ011779
C-MAMMA1000738//HYPOTHETICAL 116.5 KD PROTEIN C20G8.09C IN CHROMOSOME I.//9.00E-299//1033aa//55%//P87115
C-MAMMA1000824//ACTIN.//6.20E-20//284aa//28%//P53500
C-MAMMA1000841//PUTATIVE AMIDASE (EC 3.5.1.4).//7.80E-40//101aa//54%//O27540
C-MAMMA1000897//INTER-ALPHA-TRYPSIN INHIBITOR HEAVY CHAIN H3 PRECURSOR (TTI HEAVY CHAIN H3) (SERUM-DERIVED HYALURONAN-ASSOCIATED PROTEIN) (SHAP).//1.00E-141//576aa//37%//Q06033
C-MAMMA1000956//Homo sapiens CLDN8 gene for claudin-8.//0//1767bp//99%//AJ250711
C-MAMMA1001008//Homo sapiens aspartic-like protease mRNA, complete cds.//2.50E-276//1263bp//99%//AF117892
C-MAMMA1001030//LUTROPIN-CHORIOGONADOTROPIC HORMONE RECEPTOR (LH/CG-R) (LSH-R) (LUTEINIZING HOROMINE RECEPTOR) (FRAGMENT).//1.20E-26//276aa//28%//Q90674
C-MAMMA1001038//MYOSIN LIGHT CHAIN KINASE, SMOOTH MUSCLE AND NON-MUSCLE ISOZYMES (EC 2.7.1.117) (MLCK) [CONTAINS: TELOKIN]//2.60E-107//190aa//95%//Q15746
C-MAMMA1001041//SPECTRIN BETA CHAIN, BRAIN (SPECTRIN, NON-ERYTHROID BETA CHAIN) (FODRIN BETA CHAIN) (SPTBN1).//1.60E-16//113aa//41%//Q01082
C-MAMMA1001059//Homo sapiens mRNA for DEAD Box Protein 5.//0//1440bp//99%//AJ237946
C-MAMMA1001075//Homo sapiens CGI-72 protein mRNA, complete cds.//1.30E-181//397bp//98%//AF151830
C-MAMMA1001080//Homo sapiens SNC73 protein (SNC73) mRNA, complete cds.//1.6e-312//1596bp//94%//AF067420
C-MAMMA1001105//OVO PROTEIN (SHAVEN BABY PROTEIN).//4.00E-49//125aa//68%//P51521
C-MAMMA1001139//SRE-2 PROTEIN.//5.80E-35//239aa//38%//Q09273
C-MAMMA1001181//ABC1 PROTEIN HOMOLOG PRECURSOR.//1.30E-07//81aa//45%//Q92338
C-MAMMA1001198//Homo sapiens eps15R mRNA, partial cds.//0//2253bp//99%//AB015346
C-MAMMA1001222//EBNA-2 NUCLEAR PROTEIN.//6.60E-09//255aa//29%//P12978
C-MAMMA1001259//Mus musculus F-box protein FBX18 mRNA, partial cds.//2.30E-271//1414bp//89%//AF184275
C-MAMMA1001260//HYPOTHETICAL 97.1 KD PROTEIN R05D3.4 IN CHROMOSOME III.//2.10E-52//630aa//30%//P34537
C-MAMMA1001305//RHO-GTPASE-ACTIVATING PROTEIN 1 (GTPASE-ACTIVATING PROTEIN RHOGAP) (RHO-RELATED SMALL GTPASE PROTEIN ACTIVATOR) (CDC42 GTPASE-ACTIVATING PROTEIN) (P50-RHOGAP).//2.20E-98//283aa//63%//Q07960
C-MAMMA1001322//B-CELL GROWTH FACTOR PRECURSOR (BCGF-12 KD).//0.000000017//46aa//60%//P20931
C-MAMMA1001388//LEUCINE-RICH ALPHA-2-GLYCOPROTEIN (LRG).//1.40E-165//312aa//99%//P02750
C-MAMMA1001476//URIDINE KINASE (EC 2.7.1.48) (URIDINE MONOPHOSPHOKINASE) (FRAGMENT).//6.50E-129//260aa//92%//P52623
C-MAMMA1001501//CALPAIN 1, LARGE [CATALYTIC] SUBUNIT (EC 3.4.22.17) (CALCIUM-ACTIVATED NEUTRAL PROTEINASE) (CANP) (MU-TYPE).//5.70E-55//86aa//97%//P07384
C-MAMMA1001576//Human gamma-tubulin mRNA, complete cds.//7.50E-276//1561bp//90%//M61764
C-MAMMA1001627//Homo sapiens mRNA for transcription factor TBX6.//5.20E-189//871bp//99%//AJ007989
C-MAMMA1001633//ZINC FINGER PROTEIN 165.//6.30E-39//160aa//55%//P49910
C-MAMMA1001679//F-ACTIN CAPPING PROTEIN BETA SUBUNIT (CAPZ).//0.00000058//29aa//100%//P47756
C-MAMMA1001730//Homo sapiens brain and nasopharyngeal carcinoma susceptibility protein NSG-x mRNA, partial cds.//0//1603bp//99%//AF095687
C-MAMMA1001735/TUBULIN BETA-5 CHAIN (BETA-TUBULIN CLASS-V).//5.90E-240//445aa//97%//P09653
C-MAMMA1001743//Y BOX BINDING PROTEIN-1 (Y-BOX TRANSCRIPTION FACTOR).//8.50E-32//171aa//36%//P21573
C-MAMMA1001751//Homo sapiens tandem pore domain potassium channel TWIK-2 (KCNK6) mRNA, complete cds.//0//2332bp//99%//AF117708
C-MAMMA1001754//Homo sapiens Vacuolar proton pump subunit SFD alpha isoform mRNA complete cds.//0//1987bp//99%//AF112204
C-MAMMA1001768//CELL DIVISION CYCLE PROTEIN 48 HOMOLOG MJ1156.//3.80E-45//351aa//38%//Q58556
C-MAMMA1001771//M.musculus mRNA for semaphorin B.//2.60E-200//1272bp//79%//X85991
C-MAMMA1001820//Rattus norvegicus mRNA for PAG608 gene.//1.30E-198//1157bp//80%//Y13148
C-MAMMA1001837//ZINC FINGER PROTEIN 29 (ZFP-29).//2.60E-77//507aa//38%//Q07230
C-MAMMA1001868//TRICHOHYALIN.//2.70E-19//359aa//25%//P22793
C-MAMMA1002143//Homo sapiens Cdc42 effector protein 4 mRNA, complete cds.//1.70E-252//1170bp//99%//AF099664
C-MAMMA1002170//40S RIBOSOMAL PROTEIN S2 (S4) (LLREP3 PROTEIN).//6.00E-66//157aa//70%//P15880
C-MAMMA1002198//THIOREDOXIN PEROXIDASE 1 (THIOREDOXIN-DEPENDENT PEROXIDE REDUCTASE 1) (THIOL-SPECIFIC ANTIOXIDANT PROTEIN) (TSA) (PRP) (NATURAL KILLER CELL ENHANCING FACTOR B) (NKEF-B).//5.20E-61//60aa//90%//P32119
C-MAMMA1002219//Rattus norvegicus rexo70 mRNA, complete cds.//1.30E-181//861bp//98%//AF032667
C-MAMMA1002236//TRANSLATION INITIATION FACTOR EIF-2B GAMMA SUBUNIT (EIF-2B GDP-GTP EXCHANGE FACTOR).//8.80E-217//310aa//86%//PP70541
C-MAMMA1002268//Mus musculus sphingosine kinase (SPHKIa) mRNA, partial cds.//1.00E-190//1624bp//76%//AF068748
C-MAMMA1002297//Homo sapiens mRNA for Rab6 GTPase activating protein.//1.10E-214//881bp//97%//AJ011679
C-MAMMA1002329//M.musculus mRNA for semaphorin B.//3.80E-45//332bp//84%//X85991
C-MAMMA1002351//Mus musculus dynactin subunit p25 (p25) mRNA, complete cds.//4.30E-119//773bp//86%//AF190795
C-MAMMA1002385//RIBONUCLEOPROTEIN RB97D.//1.50E-07//206aa//29%//Q02926
C-MAMMA1002428//LYSOSOME MEMBRANE PROTEIN II (LIMP II) (85 KD LYSOSOMAL MEMBRANE SIALOGLYCOPROTEIN) (LGP85) (CD36 ANTIGEN-LIKE 2).//1.10E-24//96aa//68%//Q14108
C-MAMMA1002470//PROBABLE NH(3)-DEPENDENT NAD(⁺) SYNTHETASE (EC 6.3.5.1).//1.00E-11//128aa//36%//P47623
C-MAMMA1002485//Homo sapiens stanniocalcin-related protein mRNA, complete cds.//0//1822bp//99%//AF098462
C-MAMMA1002524//HYPOTHETICAL 117.8 KD PROTEIN IN STE2-FRS2 INTERGENIC REGION.//1.20E-34//337aa//31%//P43571
C-MAMMA1002530//Homo sapiens cytosolic phospholipase A2 gamma (cPLA2 gamma) mRNA, complete cds.//0//1910bp//99%//AF065214
C-MAMMA1002573//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//2.60E-19//666aa//23%//P08640
C-MAMMA1002617//ZINC FINGER PROTEIN 135.//7.60E-89//252aa7/57%//P52742
C-MAMMA1002619//PROBABLE UBIQUTTIN CARBOXYL-TERMINAL HYDROLASE K02C4.3 (EC 3.1.2.15) (UBIQUITIN THIOLESTERASE) (UBIQUTTIN-SPECIFIC PROCESSING PROTEASE) (DEUBIQUTTINATING ENZYME).//9.50E-16//159aa//37%//Q09931
C-MAMMA1002622//VILLIN.//7.20E-35//53aa//64%//P02640
C-MAMMA1002637//KINESIN LIGHT CHAIN (KLC).//1.30E-198//550aa//70%//Q07866
C-MAMMA1002650//Mus musculus ODA-8S protein mRNA, complete cds.//5.40E-57//480bp//68%//AF194030
C-MAMMA1002655//Homo sapiens mRNA for ganglioside sialidase, complete cds.//0//1515bp//99%//AB008185
C-MAMMA1002671//ACETYL-COENZYME A SYNTHETASE (EC 6.2.1.1) (ACETATE--COA LIGASE) (ACYL- ACTIVATING ENZYME).//1.10E-45//618aa//26%//P27550
C-MAMMA1002699//Rattus norvegicus EH domain binding protein Epsin mRNA, complete cds.//4.3e-317//1942bp//85%//AF018261
C-MAMMA1002769//Homo sapiens cell cycle progression restoration 8 protein (CPR8) mRNA, complete cds.//2.20E-25//330bp//77%//AF011794
C-MAMMA1002842//Mus musculus c-Cbl associated protein CAP mRNA, complete cds.//2.60E-58//373bp//81%//U58883
C-MAMMA1002844//TRIOSE PHOSPHATE/PHOSPHATE TRANSLOCATOR, NON-GREEN PLASTID PRECURSOR (CTPT).//4.90E-10//334aa//22%//P52178
C-MAMMA1002858//Rat cMG1 mRNA.//3.70E-238//1147bp//92%//X52590
C-MAMMA1002869//PINCH PROTEIN (PARTICULARY INTERESTING NEW CYS-HIS PROTEIN).//1.40E-160//305aa//85%//P48059
C-MAMMA1002881//GLIOMA PATHOGENESIS-RELATED PROTEIN (RTVP-1 PROTEIN).//5.70E-30//214aa//35%//P48060
C-MAMMA1002937//ZINC FINGER PROTEIN 135.//8.30E-99//393aa//43%//P52742
C-MAMMA1002972//VACUOLAR PROTEIN SORTING-ASSOCIATED PROTEIN VPS27.//1.10E-05//69aa//42%//P40343
C-MAMMA1003011//HISTONE MACRO-H2A.1.//2.70E-123//370aa//66%//Q02874
C-MAMMA1003013//DNA POLYMERASE BETA (EC 2.7.7.7).//7.40E-46//332aa//36%//P06746
C-MAMMA1003035//RIBOSOMAL LARGE SUBUNIT PSEUDOURIDINE SYNTHASE C (EC 4.2.1.70) (PSEUDOURIDYLATE SYNTHASE) (URACIL HYDROLYASE).//1.90E-13//108aa//33%//P23851
C-MAMMA1003047//Homo sapiens protein inhibitor of activated STAT protein PIASy mRNA, complete cds.//0//1533bp//99%//AF077952
C-MAMMA1003057//MD6 PROTEIN.//3.10E-225//419aa//97%//Q60584
C-MAMMA1003113//Mus musculus COP9 complex subunit 7a (COPS7a) mRNA, complete cds.//1.1OE-234//1178bp//86%//AF071316
C-MAMMA1003127//MYOSIN I ALPHA (MMI-ALPHA).//2.20E-105//217aa//89%//P46735
C-MAMMA1003146//Homo sapiens mRNA for GalT3 protein.//4.30E-218//996bp//99%//Y15062
C-MAMMA1003150//HYPOTHETICAL 118.4 KD PROTEIN IN BAT2-DAL5 INTERGENIC REGION PRECURSOR.//5.00E-13//592aa//24%//P47179
C-MAMMA1003166//Homo sapiens MLL septin-like fusion protein (MSF) mRNA, complete cds.//3.10E-158//592bp//97%//AF123052
C-NT2RM1000001//D.melanogaster sap47-2 mRNA.//1.50E-10//417bp//62%//X80110
C-NT2RM1000039//HYPOTHETICAL 41.4 KD PROTEIN IN SRLQ-HYPF INTERGENIC REGION (EC 1.18.1.-) (ORF4) (ORF2).//2.90E-14//299aa//25%//P37596
C-NT2RM1000055//Rattus norvegicus mRNA for TIP120, complete cds.//0//3106bp//89%//D87671
C-NT2RM1000080//UNC-1 PROTEIN.//5.90E-25//211aa//31%//Q21190
C-NT2RM1000086//HYPOTHETICAL 97.1 KD PROTEIN R05D3.4 IN CHROMOSOME III.//8.40E-52//364aa//32%//P34537
C-NT2RM1000092//MULTIDRUG RESISTANCE PROTEIN 2 (MULTIDRUG-EFFLUX TRANSPORTER 2).//1.00E-07//362aa//23%//P39843
C-NT2RM1000118//CALCINEURIN B SUBUNIT (PROTEIN PHOSPHATASE 2B REGULATORY SUBUNIT) (CALCINEURIN REGULATORY SUBUNIT).//1.20E-10//150aa//28%//P87072
C-NT2RM1000132//Homo sapiens NADH:ubiquinone oxidoreductase NDUFS6 subunit mRNA, nuclear gene encoding mitochondrial protein, complete cds.//7.80E-110//516bp//99%//AF044959
C-NT2RM1000153//CYTOSOLIC PURINE 5'-NUCLEOTIDASE (EC 3.1.3.5).//3.30E-38//469aa//27%//P49902
C-NT2RM1000186//CALCINEURIN B SUBUNIT (PROTEIN PHOSPHATASE 2B REGULATORY SUBUNIT) (CALCINEURIN REGULATORY SUBUNIT).//1.20E-10//150aa//28%//P87072
C-NT2RM1000187//PUTATIVE PRE-MRNA SPLICING FACTOR ATP-DEPENDENT RNA HELICASE SPAC10F6.02C.//1.10E-10//94aa//47%//O42643
C-NT2RM1000199//Homo sapiens mRNA for type I transmembrane receptor (psk-1 gene).//0//2476bp//99%//AJ245820
C-NT2RM1000244//Homo sapiens TRAF4 associated factor 1 mRNA, partial cds.//2.00E-126//592bp//99%//U81002
C-NT2RM1000252//H.sapiens E-MAP-115 mRNA.//9.70E-35//569bp//64%//X73882
C-NT2RM1000256//Homo sapiens mRNA for Glutamine:fructose-6-phosphate amidotransferase, complete cds.//0//3012bp//99%//AB016789
C-NT2RM1000257//MAGO NASHI PROTEIN.//7.90E-69//143aa//91%//P49028
C-NT2RM1000260//Homo sapiens thyroid hormone receptor-associated protein complex component TRAP100 mRNA, complete cds.//0//2766bp//99%//AF055995
C-NT2RM1000280//VACUOLAR ATP SYNTHASE SUBUNIT D (EC 3.6.1.34) (V-ATPASE D SUBUNIT) (V- ATPASE 28 KD ACCESSORY PROTEIN).//1.50E-106//118aa//97%//P39942
C-NT2RM1000354//Xenopus laevis chromosome condensation protein XCAP-G mRNA, complete cds.//7.40E-245//2101bp//68%//AF111423
C-NT2RM1000355//Homo sapiens transmembrane protein BRI (BRI) mRNA, complete cds.7/0//1599bp//99%//AF152462
C-NT2RM1000377//Homo sapiens dual specificity phosphatase MKP5 (MKP5) mRNA, complete cds.//3.20E-196//1016bp//94%//AF179212
C-NT2RM1000388//HYPOTHETICAL 27.7 KD PROTEIN IN CPT1-SPC98 INTERGENIC REGION.//0.000000019//67aa//31%//P53915
C-NT2RM1000421//RIBONUCLEASE INHIBITOR.//4.40E-21//372aa//30%//P10775
C-NT2RM1000430//Homo sapiens erythroblast macrophage protein EMP mRNA, complete cds.//1.40E-185//1486bp//81%//AF084928
C-NT2RM1000499//Caenorhabditis elegans mRNA for centaurin gamma 1A.//3.00E-17//927bp//58%//AJ132700
C-NT2RM1000539//Homo sapiens mRNA for Lsm5 protein.//3.00E-158//733bp//99%//AJ238097
C-NT2RM1000553//Homo sapiens putative glycolipid transfer protein mRNA, complete cds.//3.40E-177//814bp//99%//AF103731
C-NT2RM1000555//UNR PROTEIN.//0//678aa//98%//P18395
C-NT2RM1000563//TRANSMISSION-B LOCKING TARGET ANTIGEN S230 PRECURSOR.//0.0000068//199aa//30%//Q08372
C-NT2RM1000623//RIBONUCLEASE INHIBITOR.//4.40E-21//372aa//30%//P10775
C-NT2RM1000648//GLYCOSYLTRANSFERASE ALG2 (EC 2.4.1.-).//8.50E-75//301aa//39%//P43636
C-NT2RM1000661//Homo sapiens translation initiation factor 4e mRNA, complete cds.//5.70E-210//960bp//99%//AF038957
C-NT2RM1000666//DNA-BINDING PROTEIN A.//2.20E-09//165aa//34%//P16989
C-NT2RM1000691//Homo sapiens mRNA for PLU-1 protein.//0//3104bp//99%//AJ132440
C-NT2RM1000702//PUTATIVE SERINE/THREONINE-PROTEIN KINASE PKWA (EC 2.7.1.-).//5.60E-08//187aa//27%//P49695
C-NT2RM1000742//Homo sapiens AC133 antigen mRNA, complete cds.//0//3524bp//99%//AF027208
C-NT2RM1000746//Homo sapiens polyamine modulated factor-1 (PMF1) mRNA, complete cds.//6.70E-227//1043bp//99%//AF141310
C-NT2RM1000770//DXS6673E PROTEIN.//1.40E-39//194aa//48%//Q14202
C-NT2RM1000772//VEGETATABLE INCOMPATIBILITY PROTEIN HET-E-1.//7.30E-15//280aa//27%//Q00808
C-NT2RM1000800//Mus musculus partial mRNA for B-IND1 protein (B-indl gene).//1.10E-98//571bp//89%//Z97207
C-NT2RM1000811//Homo sapiens AC133 antigen mRNA, complete cds.//0//3524bp//99%//AF027208
C-NT2RM1000826//UNR PROTEIN.//0//678aa//98%//P18395
C-NT2RM1000833//Homo sapiens sec61 homolog mRNA, complete cds.//0//3541 bp//99%//AF08445 8
C-NT2RM1000850//ANKYRIN R (ANKYRINS 2.1 AND 2.2) (ERYTHROCYTE ANKYRIN).//9.70E-42//333aa//36%//P16157
C-NT2RM1000852//Homo sapiens putative ATP-dependent RNA helicase ROK1 mRNA, complete cds.//0//2206bp//99%//AF077033
C-NT2RM1000874//Homo sapiens death effector domain-containing testicular molecule mRNA, complete cds.//1.40E-244//1113bp//99%//AF043733
C-NT2RM1000882//Homo sapiens delta-6 fatty acid desaturase mRNA, complete cds.//4.30E-122//1394bp//69%//AF126799
C-NT2RM1000883//Homo sapiens I-1 receptor candidate protein mRNA, complete cds.//0//5107bp//99%//AF082516
C-NT2RM1000885//HYPOTHETICAL 97.1 KD PROTEIN R05D3.4 IN CHROMOSOME III.//1.80E-56//630aa//30%//P34537
C-NT2RM1000894//DNA-DIRECTED RNA POLYMERASE 1135 KD POLYPEPTIDE (EC 2.7.7.6) (RNA POLYMERASE I SUBUNIT 2) (RPA135).//0//1020aa//89%//P70700
C-NT2RM1000898//ACTIN, CYTOPLASMIC (ACTIN, MICRONUCLEAR).//8.90E-26//229aa//29%//P02583
C-NT2RM1000924//HYPOTHETICAL 39.7 KD PROTEIN C34E10.2 IN CHROMOSOME III.//1.00E-15//266aa//26%//P46577
C-NT2RM1001003//Homo sapiens alpha-catenin-like protein (CTNNAL1) mRNA, complete cds.//0//2230bp//99%//AF030233
C-NT2RM1001008//HYPOTHETICAL 72.5 KD PROTEIN C2F7.10 IN CHROMOSOME I.//1.60E-13//119aa//36%//Q09701
C-NT2RM1001059//NUCLEAR POLYADENYLATED RNA-BINDING PROTEIN NAB4.//3.60E-11//180aa//28%//Q99383
C-NT2RM1001072//1-PHOSPHATIDYLINOSITOL-4,5-BISPHOSPHATE PHOSPHODEESTERASE GAMMA 1 (EC 3.1.4.11) (PLC-GAMMA-1) (PHOSPHOLIPASE C-GAMMA-1) (PLC-II) (PLC-148).//8.30E-47//259aa//35%//P08487
C-NT2RM1001092//ZINC FINGER PROTEIN 91 (ZINC FINGER PROTEIN HTF10) (HPF7).//3.60E-115//332aa//52%//Q05481
C-NT2RM1001102//Human HEM45 mRNA, complete cds.//2.30E-27//482bp//63%//U88964
C-NT2RM1001115//ENDOCHITINASE 2 PRECURSOR (EC 3.2.1.14).//5.60E-06//239aa//27%//P54197
C-NT2RM2000013//DNA-DIRECTED RNA POLYMERASE III 128 KD POLYPEPTIDE (EC 2.7.7.6) (RNA POLYMERASE III SUBUNIT 2).//2.20E-144//362aa//71%//P25167
C-NT2RM2000030//DYNEIN INTERMEDIATE CHAIN, CYTOSOLIC (DH IC) (CYTOPLASMIC DYNEIN INTERMEDIATE CHAIN).//0.00000043//136aa//31%//P54703
C-NT2RM2000092//UBIQUITIN CARBOXYL-TERMINAL HYDROLASE 8 (EC 3.1.2.15) (UBIQUTTIN THIOLESTERASE 8) (UBIQUITIN-SPECIFIC PROCESSING PROTEASE 8) (DEUBIQUITINATING ENZYME 8).//1.30E-36//160aa//40%//P50102
C-NT2RM2000191//Homo sapiens cGMP phosphodiesterase A1 (PDE9A) mRNA, complete cds.//0//1574bp//99%//AF067223
C-NT2RM2000260//EXTENSIN PRECURSOR (PROLINE-RICH GLYCOPROTEIN).//3.60E-19//181aa//34%//P14918
C-NT2RM2000322//SPERMIDINE SYNTHASE (EC 2.5.1.16) (PUTRESCINE AMINOPROPYLTRANSFERASE) (AMINOPROPYLTRANSFERASE).//8.10E-06//167aa//29%//O48660
C-NT2RM2000363//BREAKPOINT CLUSTER REGION PROTEIN.//1.80E-14//245aa//29%//P11274
C-NT2RM2000368//Homo sapiens protein kinase C-binding protein RACK7 mRNA, partial cds.//0//1506bp//99%//U48251
C-NT2RM2000371//POLYRIBONUCLEOTIDE NUCLEOTIDYLTRANSFERASE (EC 2.7.7.8) (POLYNUCLEOTIDE//1.70E-68//419aa//36%//P50849
C-NT2RM2000402//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//1.60E-54//344aa//33 %//P32802
C-NT2RM2000407//Mus musculus semaphorin VIa mRNA, complete cds.//9.70E-201//826bp//84%//AF030430
C-NT2RM2000422//SODIUM- AND CHLORIDE-DEPENDENT TRANSPORTER NTT73.//1.00E-222//237aa//89%//Q08469
C-NT2RM2000452//HYPOTHETICAL 63.6 KD PROTEIN IN YPT52-GCN3 INTERGENIC REGION.//1.00E-07//157aa//28%//P36113
C-NT2RM2000469//NITROGEN PERMEASE REACTIVATOR PROTEIN (EC 2.7.1.-).//8.90E-06//377aa//24%//P22211
C-NT2RM2000490//SYNAPTOTAGMIN (P65).//1.80E-13//166aa//34%//P41823
C-NT2RM2000502//Rattus norvegicus W307 mRNA, complete cds.//1.70E-58//381bp//86%//U78304
C-NT2RM2000504//Homo sapiens metalloprotease 1 (MP1) mRNA, complete cds.//0//1673bp//99%//AF061243
C-NT2RM2000522//SKIN SECRETORY PROTEIN XP2 PRECURSOR (APEG PROTEIN).//1.30E-12//282aa//32%//P17437
C-NT2RM2000566//Homo sapiens integrin alpha-7 mRNA, complete cds.//0//2519bp//96%//AF032108
C-NT2RM2000577//ISOLEUCYL-TRNA SYNTHETASE (EC 6.1.1.5) (ISOLEUCINE--TRNA LIGASE) (ILERS).//1.70E-187//741aa//46%//P73505
C-NT2RM2000588//HISTONE DEACETYLASE HDA1.//2.80E-60//384aa//40%//P53973
C-NT2RM2000594//Homo sapiens DNA cytosine-5 methyltransferase 3 beta 3 (DNMT3B) mRNA, complete cds.//0//2712bp//99%//AF156487
C-NT2RM2000599//Homo sapiens F-box protein Lilina (LILINA) mRNA, complete cds.//4.90E-70//838bp//69%//AF179221
C-NT2RM2000609//Homo sapiens CTL1 gene.//0//1559bp//99%//AJ245620
C-NT2RM2000612//Rattus norvegicus ADP-ribosylation factor-directed GTPase activating protein mRNA, complete cds.//2.60E-106//1069bp//74%//U35776
C-NT2RM2000624//SPLICING FACTOR, ARGININE/SERINE-RICH 4 (PRE-MRNA SPLICING FACTOR SRP75).//4.40E-32//319aa//35%//Q08170
C-NT2RM2000691//ACTIN-LIKE PROTEIN 3 (ACTIN-2).//3.70E-142//285aa//90%//P32391
C-NT2RM2000714//RAS SUPPRESSOR PROTEIN 1 (RSU-1) (RSP-1 PROTEIN) (RSP-1).//3.80E-23//184aa//36%//Q15404
C-NT2RM2000718//Homo sapiens endocrine regulator mRNA, complete cds.//0//1731bp//99%//AF121141
C-NT2RM2000735//ZINC FINGER PROTEIN 43 (ZINC PROTEIN HTF6).//2.90E-103//249aa//73%//P28160
C-NT2RM2000740//POSSIBLE GLOBAL TRANSCRIPTION ACTIVATOR SNF2L.//5.70E-53//266aa//43%//P41877
C-NT2RM2000821//COATOMER BETA SUBUNIT (BETA-COAT PROTEIN) (BETA-COP).//9.50E-279//545aa//98%//P23514
C-NT2RM2000951//Homo sapiens XYLB mRNA for xylulokinase, complete cds.//1.70E-200//927bp//99%//AB015046
C-NT2RM2001035//CCR4-ASSOCIATED FACTOR 1 (CAF1).//8.20E-154//285aa//99%//Q60809
C-NT2RM2001065//Homo sapiens COP9 complex subunit 4 mRNA, complete cds.//0//1554bp//99%//AF100757
C-NT2RM2001100//HYPOTHETICAL 39.7 KD PROTEIN C34E10.2 IN CHROMOSOME III.//2.40E-15//266aa//26%//P46577
C-NT2RM2001105//Drosophila melanogaster eyelid (eld) mRNA, complete cds.//1.20E-28//805bp//61%//AF053091
C-NT2RM2001196//PROLINE-RICH PROTEIN MP-3 (FRAGMENT).//1.30E-20//267aa//35%//P05143
C-NT2RM2001201//EUKARYOTIC TRANSLATION INITIATION FACTOR 5 (EIF-5).//1.50E-07//95aa//35%//P48724
C-NT2RM2001221//KALIRIN (PAM COOH-TERMINAL INTERACTOR PROTEIN 10) (P-CIP10).//3.60E-10//177aa//32%//P97924
C-NT2RM2001238//GLUTAMINASE, KIDNEY ISOFORM PRECURSOR (EC 3.5.1.2) (GLS) (L-GLUTAMINE AMIDOHYDROLASE).//1.30E-180//328aa//99%//P13264
C-NT2RM2001256//PROTEIN TSG24 (MEIOTIC CHECK POINT REGULATOR).//1.60E-166//312aa//98%//P53995
C-NT2RM2001324//ZYXIN.//6.80E-55//200aa//41%//Q04584
C-NT2RM2001345//VEGETATIBLE INCOMPATIBILITY PROTEIN HET-E-1.//2.90E-08//334aa//22%//Q00808
C-NT2RM2001424//Homo sapiens mRNA for ElB-55kDa-associated protein.//0//1621bp//99%//AJ007509
C-NT2RM2001499//LOW-AFFINITY CATIONIC AMINO ACID TRANSPORTER-2 (CAT-2) (CAT2).//7.40E-121//437aa//57%//P52569
C-NT2RM2001547//PROBABLE PROTEIN DISULFIDE ISOMERASE P5 PRECURSOR (EC 5.3.4.1).//6.90E-27//90aa//42%//P38660
C-NT2RM2001575//52 KD RO PROTEIN (SJOGREN SYNDROME TYPE A ANTIGEN (SS-A)) (RO(SS-A)).//4.30E-61//312aa//44%//P19474
C-NT2RM2001592//Rattus norvegicus rexo70 mRNA, complete cds.//3.10E-156//909bp//88%//AF032667
C-NT2RM2001605//Homo sapiens mRNA for PLU-1 protein.//0//3114bp//99%//AJ132440
C-NT2RM2001613//Homo sapiens sec61 homolog mRNA, complete cds.//0//2601 bp//99%//AF084458
C-NT2RM2001632//KES1 PROTEIN.//1.40E-31//342aa//34%//P35844
C-NT2RM2001635//NUCLEAR ENVELOPE PORE MEMBRANE PROTEIN POM 121 (PORE MEMBRANE PROTEIN OF 121 KD) (P145).//1.20E-142//566aa//56%//P52591
C-NT2RM2001648//Homo sapiens sec61 homolog mRNA, complete cds.//0//2421 bp//99%//AF084458
C-NT2RM2001652//Homo sapiens guanine nucleotide exchange factor mRNA, complete cds.//0//2608bp//99%//AF111162
C-NT2RM2001659//ZINC/CADMIUM RESISTANCE PROTEIN.//3.40E-39//161aa//34%//P20107
C-NT2RM2001664//Homo sapiens IkappaB kinase complex associated protein (IKAP) mRNA complete cds.//0//2471bp//99%//AF044195
C-NT2RM2001668//Homo sapiens putative WHSC1 protein (WHSC1) mRNA, alternative splice product ending in intron 11, complete cds.//6.20E-16//464bp//62%//AFQ83391
C-NT2RM2001670//ZINC FINGER PROTEIN 29 (ZFP-29).//6.50E-104//407aa//43%//Q07230
C-NT2RM2001671//Oryctolagus cuniculus sarcolemmal associated protein (SLAP1) mRNA, complete cds.//0//1843bp//94%//U21155
C-NT2RM2001688//HYPOTHETICAL 33.8 KD PROTEIN C5H10.01 IN CHROMOSOME I.//4.60E-20//253aa//30%//Q09674
C-NT2RM2001698//Homo sapiens XGalT-1 mRNA for galactosyltransferase I, complete cds.//6.20E-253//1170bp//99%//AB028600
C-NT2RM2001700//ACYL-COA DEHYDROGENASE, VERY-LONG-CHAIN SPECIFIC (EC 1.3.99.-) (VLCAD) (FRAGMENT).//5.70E-130//536aa//49%//P50544
C-NT2RM2001716//Homo sapiens BPTF mRNA for bromodomain PHD finger transcription factor, complete cds.//0//1774bp//98%//AB032251
C-NT2RM2001730//PROBABLE UBIQUITIN CARBOXYL-TERMINAL HYDROLASE K02C4.3 (EC 3.1.2.15) (UBIQUITIN THIOLESTERASE) (UBIQUITIN-SPECIFIC PROCESSING PROTEASE) (DEUBIQUITINATING ENZYME).//7.20E-16//381aa//27%//Q09931
C-NT2RM2001743//Homo sapiens cell cycle progression 2 protein (CPR2) mRNA, complete cds.//0//1498bp//99%//AF011792
C-NT2RM2001753//HYPOTHETICAL PROTEIN KIAA0210.//8.80E-11//119aa//36%//Q92609
C-NT2RM2001760//Homo sapiens sec61 homolog mRNA, complete cds.//0//2379bp//99%//AF084458
C-NT2RM2001771//ZINC FINGER PROTEIN 135.//6.40E-154//394aa//64%//P52742
C-NT2RM2001782//Homo sapiens GDP-mannose pyrophosphorylase A (GMPPA) mRNA, complete cds.//0//1470bp//99%//AF135422
C-NT2RM2001785//Homo sapiens delta-6 fatty acid desaturase mRNA, complete cds.//0//2150bp//99%//AF126799
C-NT2RM2001803//Homo sapiens IkappaB kinase complex associated protein (IKAP) mRNA, complete cds.//0//2249bp//99%//AF044195
C-NT2RM2001823//CHD1 PROTEIN.//1.80E-106//631aa7/39%//P32657
C-NT2RM2001839//Homo sapiens calumein (Calu) mRNA, complete cds.//0//2415bp//97%//AF013759
C-NT2RM2001886//PAB-DEPENDENT POLY(A)-SPECIFIC RIBONUCLEASE SUBUNIT PAN2 (EC 3.1.13.4) (PAB1P-DEPENDENT POLY(A)-NUCLEASE).//3.00E-54//337aa//39%//P53010
C-NT2RM2001896//CELL DIVISION PROTEIN FTSJ.//5.10E-26//204aa//34%//P28692
C-NT2RM2001930//M.musculus mRNA for semaphorin G.//5.20E-135//894bp//83%//X97818
C-NT2RM2001935//Homo sapiens single-strand selective monofunctional uracil DNA glycosylase mRNA, complete cds.//0//1454bp//99%//AF125182
C-NT2RM2001936//32.3 KD PROTEIN IN CWP1-MBR1 INTERGENIC REGION.//2.70E-27//216aa//34%//P28320
C-NT2RM2001950//HYPOTHETICAL 105.9 KD PROTEIN IN AAC3-RFC5 INTERGENIC REGION.//0.0000001//212aa//23%//P38250
C-NT2RM2001983//Homo sapiens RGS-GAIP interacting protein GIPC mRNA, complete cds.//0//1658bp//98%//AF089816
C-NT2RM2001989//NUCLEOLAR PROTEIN NOP4 (NUCLEOLAR PROTEIN NOP77).//1.90E-39//253aa//35%//P37838
C-NT2RM2001997//PROTEIN DISULFIDE ISOMERASE PRECURSOR (PDI) (EC 5.3.4.1).//1.30E-10//232aa//28%//Q12730
C-NT2RM2001998//HYPOTHETICAL 85.7 KD PROTEIN C13G6.03 IN CHROMOSOME L//3.10E-12//206aa//30%//Q09782
C-NT2RM2002004//LA PROTEIN HOMOLOG (LA RIBONUCLEOPROTEIN) (LA AUTOANTIGEN HOMOLOG).//2.90E-08//83aa//44%//P40796
C-NT2RM2002014//HYPOTHETICAL 81.4 KD PROTEIN IN GREB-FEOA INTERGENIC REGION.//1.10E-89//425aa//41%//P46837
C-NT2RM2002030//Homo sapiens mRNA for Glutamine:fructose-6-phosphate amidotransferase, complete cds.//0//1959bp//99%//AB016789
C-NT2RM2002055//VACUOLAR PROTEIN SORTING-ASSOCIATED PROTEIN VPS13.//0.00000099//338aa//24%//Q07878
C-NT2RM2002088//PUTATIVE HETEROGENEOUS NUCLEAR RIBONUCLEOPROTEIN X (HNRNP X) (CBP).//5.00E-62//104aa//57%//Q61990
C-NT2RM2002091//Drosophila melanogaster eyelid (eld) mRNA, complete cds.//7.10E-29//805bp//61 %//AF053091
C-NT2RM2002100//Homo sapiens mRNA for ATP-dependent RNA helicase, partial.//0//1807bp//99%//AJ010840
C-NT2RM2002109//Homo sapiens glioma amplified on chromosome 1 protein (GAC1) mRNA, complete cds.//0//1868bp//99%//AF030435
C-NT2RM2002128//PUTATIVE SERINE/THREONINE-PROTEIN KINASE PKWA (EC 2.7.1.-).//4.90E-13//487aa7/26%//P49695
C-NT2RM2002142//GASTRULATION SPECIFIC PROTEIN G12.//8.00E-31//105aa//47%//P47805
C-NT2RM2002145//Homo sapiens erythroblast macrophage protein EMP mRNA, complete cds.//8.50E-191//1524bp//81%//AF084928
C-NT2RM4000024//DNA-DIRECTED RNA POLYMERASE III 128 KD POLYPEPTIDE (EC 2.7.7.6) (RNA POLYMERASE III SUBUNIT 2).//7.10E-155//381aa//72%//P25167
C-NT2RM4000030//LAS1 PROTEIN.//5.60E-12//184aa//32%//P36146
C-NT2RM4000046//GOLIATH PROTEIN (G1 PROTEIN).//0.000008//112aa//31%//Q06003
C-NT2RM4000104//ZINC FINGER PROTEIN 135.//1.50E-81//251aa//53%//P52742
C-NT2RM4000139//R.norvegicus trg mRNA.//2.30E-114//1161bp//72%//X68101
C-NT2RM4000155//THREONYL-TRNA SYNTHETASE, CYTOPLASMIC (EC 6.1.1.3) (THREONINE--TRNA LIGASE) (THRRS).//1.20E-157//321aa//61%//P26639
C-NT2RM4000156//H.sapiens HPBRII-7 gene.//3.60E-21//785bp//60%//X67336
C-NT2RM4000167//Homo sapiens mRNA for Chromokinesin (KIF 4 gene).//0//1946bp//99%//AJ271784
C-NT2RM4000169//INTRACELLULAR PROTEIN TRANSPORT PROTEIN USO1.//4.80E-13//686aa//23%//P25386
C-NT2RM4000191//PUTATIVE ATP-DEPENDENT RNA HELICASE PL10.//9.20E-75//439aa//41%//P16381
C-NT2RM4000202//ZINC FINGER PROTEIN MOK-2 (HOK-2).//4.90E-32//170aa//41%//Q16600
C-NT2RM4000215//MAK16 PROTEIN.//1.30E-68//295aa//49%//P10962
C-NT2RM4000229//Gallus gallus actin filament-associated protein (AFAP-110) mRNA, complete cds.//1.10E-27//633bp//64%//L20303
C-NT2RM4000233//Mus musculus semaphorin Via mRNA, complete cds.//3.40E-231//1395bp//86%//AF030430
C-NT2RM4000290//Human transducin-like enhancer protein (TLE3) mRNA, complete cds.//2.20E-276//1124bp//97%//M99438
C-NT2RM4000344//Homo sapiens mRNA for ATP-dependent metalloprotease YME1L.//0//2030bp//99%//AJ132637
C-NT2RM4000354//LETHAL(2)DENTICLELESS PROTEIN (DTL83 PROTEIN).//1.50E-21//208aa//35%//Q24371
C-NT2RM4000356//RAS-RELATED PROTEIN RAB-17.//5.90E-80//213aa//75%//P35292
C-NT2RM4000386//Mus musculus ODZ3 (Odz3) mRNA, partial cds.//0//2156bp//87%//AF195418
C-NT2RM4000421//Homo sapiens mRNA for nuclear transport receptor.//0//1730bp//99%//AJ133769
C-NT2RM4000433//Mus musculus retinoic acid-responsive protein (Stra6) mRNA, complete cds.//4.10E-271//2085bp//77%//AF062476
C-NT2RM4000457//HYPOTHETICAL 111.9 KD PROTEIN C22H10.03C IN CHROMOSOME I.//8.00E-20//393aa//24%//Q10297
C-NT2RM4000471//Homo sapiens cysteine desulfurase (nifS) mRNA, complete cds.//0//2092bp//99%//AF097025
C-NT2RM4000486//SALIVARY PROLINE-RICH PROTEIN PRECURSOR (CLONES CP3, CP4 AND CP5) [CONTAINS: BASIC PEPTIDE IB-6; PEPTIDE P-H].//4.80E-11//242aa//31%//P04280
C-NT2RM4000496//SAP1 PROTEIN.//8.30E-53//434aa//29%//P39955
C-NT2RM4000515//NEUROFILAMENT TRIPLET H PROTEIN (200 KD NEUROFILAMENT PROTEIN) (NF-H) (FRAGMENT).//1.10E-11//394aa//24%//P16884
C-NT2RM4000531//ZINC FINGER PROTEIN 29 (ZFP-29).//2.40E-89//389aa//43%//Q07230
C-NT2RM4000590//RING CANAL PROTEIN (KELCH PROTEIN).//1.00E-59//595aa//28%//Q04652
C-NT2RM4000595//PUTATIVE ADENYLATE CYCLASE REGULATORY PROTEIN.//8.70E-15//403aa//30%//P26337
C-NT2RM4000611//VEGETATIBLE INCOMPATIBILITY PROTEIN HET-E-1.//2.90E-09//108aa//31%//Q00808
C-NT2RM4000616//ACETYL-COENZYME A SYNTHETASE (EC 6.2.1.1) (ACETATE--COA LIGASE) (ACYL- ACTIVATING ENZYME).//2.70E-146//420aa//60%//P27550
C-NT2RM4000657//1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III).//3.00E-68//297aa//40%//P51178
C-NT2RM4000674//HYPOTHETICAL SYMPORTER SLL13747/1.20E-28//180aa//30%//P74168
C-NT2RM4000712//Homo sapiens ubiquitin hydrolyzing enzyme I (UBH1) mRNA, partial cds.//1.00E-136//1104bp//77%//AF022789
C-NT2RM4000733//TRANSCRIPTION TERMINATION FACTOR RHO.//0.00000041//207aa//29%//P52154
C-NT2RM4000734//Homo sapiens Smad- and Olf-interacting zinc finger protein mRNA, partial cds.//0//2071bp//99%//AF221712
C-NT2RM4000741//Homo sapiens hSGT1 mRNA for hSgt1p, complete cds.//0//2184bp//99%//D88208
C-NT2RM4000751//ZINC FINGER PROTEIN 184 (FRAGMENT).//3.90E-125//301aa//53%//Q99676
C-NT2RM4000798//Homo sapiens brefeldin A-inhibited guanine nucleotide-exchange protein 2 mRNA, complete cds.//0//2603bp//99%//AF084521
C-NT2RM4000820//VACUOLAR ATP SYNTHASE SUBUNIT AC45 PRECURSOR (EC 3.6.1.34) (V-ATPASE AC45 SUBUNIT).//1.10E-24//138aa//44%//P40682
C-NT2RM4000857//LEUCINE-RICH ALPHA-2-GLYCOPROTEIN (LRG).//6.70E-22//250aa//29%//P02750
C-NT2RM4000996//ZINC FINGER PROTEIN 91 (ZINC FINGER PROTEIN HTF10) (HPF7).//8.00E-211//738aa//50%//Q05481
C-NT2RM4001047//MO25 PROTEIN.//8.00E-140//333aa//80%//Q06138
C-NT2RM4001054//Homo sapiens sec61 homolog mRNA, complete cds.//3.10E-190//1315bp//81%//AF077032
C-NT2RM4001084//HYPOTHETICAL 105.6 KD PROTEIN C16C9.06C IN CHROMOSOME I.//0.000000032//165aa//33%//Q09820
C-NT2RM4001092//ZINC FINGER PROTEIN GLO37/3.10E-24//265aa//33%//P38682
C-NT2RM4001116//HYPOTHETICAL 216.3 KD PROTEIN R06F6.8 IN CHROMOSOME II.//5.90E-86//292aa//48%//Q09417
C-NT2RM4001140//HOMEOBOX PROTEIN MSH-D.//1.00E-11//103aa//38%//Q01704
C-NT2RM4001155//ADRENAL MEDULLA 50 KD PROTEIN.//4.10E-197//445aa//78%//Q27969
C-NT2RM4001178//PROBABLE ATP-DEPENDENT RNA HELICASE HAS1.//1.10E-48//218aa//43%//Q03532
C-NT2RM4001200//ZINC FINGER PROTEIN 135.//9.50E-135//375aa//60%//P52742
C-NT2RM4001203//Homo sapiens rab3-GAP regulatory domain mRNA, complete cds.//0//2310bp//99%//AF004828
C-NT2RM4001217//Mus musculus actin-binding protein (ENC-1) mRNA, complete cds.//3.10E-148//1445bp//72%//U65079
C-NT2RM4001256//Xenopus laevis putative Zic3 binding protein mRNA, complete cds.//4.30E-55//289bp//77%//AF129131
C-NT2RM4001313//PHOSPHATIDYLINOSITOL 3-KINASE VPS34-UKE (EC 2.7.1.137) (PI3-KINASE) (PTDINS-3-KINASE) (PI3K).//3.50E-35//124aa//65%//P54676
C-NT2RM4001316//ACYL-COA DEHYDROGENASE, MEDIUM-CHAIN SPECIFIC PRECURSOR (EC 1.3.99.3) (MCAD).//2.30E-31//334aa//30%//P08503
C-NT2RM4001320//Homo sapiens mRNA for Neuroblastoma, complete cds.//1.80E-39//728bp//64%//D89016
C-NT2RM4001340//UTR4 PROTEIN (UNKNOWN TRANSCRIPT 4 PROTEIN).//1.00E-28//171aa//37%//P32626
C-NT2RM4001344//HYPOTHETICAL GTP-BINDING PROTEIN IN POP2-HOL1 INTERGENIC REGION.//8.10E-30//265aa//33%//P53742
C-NT2RM4001347//Homo sapiens NY-REN-25 antigen mRNA, partial cds.//0//2300bp//99%//AF155103
C-NT2RM4001371//Homo sapiens IDN3 mRNA, partial cds.//0//2524bp//99%//AB019494
C-NT2RM4001382//Homo sapiens RanBP7/importin 7 mRNA, complete cds.//2.20E-237//1079bp//99%//AF098799
C-NT2RM4001411//Mus musculus Pro-rich, PH, SH2 domain-containing signaling mediator (PSM) mRNA, complete cds.//0//1962bp//87%//AF020526
C-NT2RM4001412//Homo sapiens nGAP mRNA, complete cds.//0//1918bp//99%//AF047711
C-NT2RM4001444//ISOLEUCYL-TRNA SYNTHETASE (EC 6.1.1.5) (ISOLEUCINE--TRNA LIGASE) (ILERS).//1.40E-118//444aa//46%//P73505
C-NT2RM4001483//ZINC FINGER PROTEIN 136.//5.10E-106//357aa//55%//P52737
C-NT2RM4001566//NECDIN.//9.80E-44//227aa//41%//P25233
C-NT2RM4001582//Mus musculus COP9 complex subunit 7b (COPS7b) mRNA, complete cds.//1.50E-284//1082bp//90%//AF071317
C-NT2RM4001592//HYPOTHETICAL 128.5 KD HELICASE IN ATS1-TPD3 INTERGENIC REGION.//7.60E-56//213aa//49%//P31380
C-NT2RM4001597//M.musculus red-1 gene./12.10E-171//1414bp//78%//X92750
C-NT2RM4001611//SIS2 PROTEIN (HALOTOLERANCE PROTEIN HAL3)J/2.60E-32//203aa//39%//Q12600
C-NT2RM4001629//MAGUK P55 SUBFAMILY MEMBER 3 (MPP3 PROTEIN) (DISCS, LARGE HOMOLOG 3).//1.50E-93//278aa//38%//Q13368
C-NT2RM4001666//HYPOTHETICAL 48.6 KD PROTEIN IN ALPA-GABP INTERGENIC REGION.//2.70E-84//410aa//42%//P37339
C-NT2RM4001714//SEPTIN 2 HOMOLOG (FRAGMENT).//8.90E-141//354aa//72%//Q14141
C-NT2RM4001731//Homo sapiens F-box protein Lilina (LILINA) mRNA, complete cds.//0//1922bp//100%//AF179221
C-NT2RM4001758//PUTATIVE SERINE/THREONINE-PROTEIN KINASE EMK (EC 2.7.).//4.10E-186//639aa//58%//Q05512
C-NT2RM4001783//ZINC FINGER PROTEIN HRX (ALL-1).//7.90E-66//311aa//35%//Q03164
C-NT2RM4001810//AGGRECAN CORE PROTEIN PRECURSOR (CARTILAGE-SPECIFIC PROTEOGLYCAN CORE PROTEIN) (CSPCP) (CHONDROITIN SULFATE PROTEOGLYCAN CORE PROTEIN 1).//5.10E-07//263aa//30%//P16112
C-NT2RM4001813//LECTIN BRA-2.//0.00000048//114aa//30%//P17346
C-NT2RM4001819//Human p58/GTA (galactosyltransferase associated protein kinase) mRNA, complete cds.//8.10E-300//1395bp//98%//M37712
C-NT2RM4001823//ZINC FINGER PROTEIN 43 (ZINC PROTEIN HTF6)7/2.90E-55//325aa//37%//P28160
C-NT2RM4001828//ZINC FINGER PROTEIN 84 (ZINC FINGER PROTEIN HPF2).//5.90E-161//481aa//56%//P51523
C-NT2RM4001858//T-BOX CONTAINING PROTEIN TBX6L (FRAGMENT).//6.50E-22//126aa//46%//P79779
C-NT2RM4001865//Homo sapiens mRNA for atopy related autoantigen CALC.//4.30E-244//1248bp//94%//Y17711
C-NT2RM4001876//RAS SUPPRESSOR PROTEIN 1 (RSU-1) (RSP-1 PROTEIN) (RSP-1).//6.50E-23//184aa//36%//Q15404
C-NT2RM4001880//PUTATIVE DNA HELICASE II HOMOLOG (EC 3.6.1.-).//5.90E-09//268aa//26%//P47486
C-NT2RM4001930//Homo sapiens dolichyl-P-Glc:Man9GlcNAc2-PP-dolichyl glucosyltransferase (ALG6) mRNA, complete cds.//0//1930bp//99%//AF102851
C-NT2RM4001940//Homo sapiens timeless homolog mRNA, complete cds.//0//2087bp//99%//AF098162
C-NT2RM4001969//R.norvegicus mRNA for IP63 protein.//2.60E-261//1563bp//84%//X99330
C-NT2RM4001979//ZINC FINGER PROTEIN 84 (ZINC FINGER PROTEIN HPF2).//1.80E-112//457aa//47%//P51523
C-NT2RM4001987//NEURAL CELL ADHESION MOLECULE 1, LARGE ISOFORM PRECURSOR (N-CAM 180) [CONTAINS: N-CAM 140].//3.20E-17//281aa//30%//P16170
C-NT2RM4002013//HYPOTHETICAL 89.4 KD TRP-ASP REPEATS CONTAINING PROTEIN IN PMT6-PCT1 INTERGENIC REGION.//6.90E-94//589aa//35%//P42935
C-NT2RM4002034//Homo sapiens hiwi mRNA, partial cds.//1.90E-53//1585bp//60%//AF104260
C-NT2RM4002062//ASPARTYL-TRNA SYNTHETASE (EC 6.1.1.12) (ASPARTATE--TRNA LIGASE) (ASPRS).//1.90E-31//80aa//52%//P36419
C-NT2RM4002063//Oryctolagus cuniculus sarcosine oxidase (SOX) mRNA, complete cds.//0//1865bp//99%//U82267
C-NT2RM4002066//Homo sapiens thyroid hormone receptor-associated protein complex component TRAP230 mRNA, complete cds.//1.50E-211//1123bp//71 %//AF117755
C-NT2RM4002073//Mus musculus fatty acid transport protein 3 mRNA, partial cds.//9.30E-293//1751bp//83%//AF072758
C-NT2RM4002075//RING CANAL PROTEIN (KELCH PROTEIN).//2.80E-105//556aa//41 %//Q04652
C-NT2RM4002093//Homo sapiens neural polypyrimidine tract binding protein (PTB) mRNA, complete cds.//0//2550bp//99%//AF176085
C-NT2RM4002109//Homo sapiens mRNA for Chromokinesin (KIF 4 gene).//0//2572bp//99%//AJ271784
C-NT2RM4002145//SLIT PROTEIN PRECURSOR.//1.40E-09//127aa//33%//P24014
C-NT2RM4002146//Homo sapiens MAGOH mRNA, complete cds.//6.90E-70//454bp//85%//AF035940
C-NT2RM4002161//Homo sapiens laforin (EPM2A) mRNA, complete cds.//0//2671bp//99%//AF084535
C-NT2RM4002174//MRPPROTEIN.//9.10E-68//264aa//51%//P21590
C-NT2RM4002189//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//6.20E-33//688aa//27%//P08640
C-NT2RM4002194//Mus musculus semaphorin VIa mRNA, complete cds.//5.20E-297//1753bp//87%//AF030430
C-NT2RM4002205//ELONGATION FACTOR G, MITOCHONDRIAL PRECURSOR (MEF-G).//3.00E-37//122aa//72%//Q07803
C-NT2RM4002213//Homo sapiens protein phosphatase methylesterase-1 (PME-1) mRNA, complete cds.//0//2452bp//100%//AF157028
C-NT2RM4002226//GTPASE ACTIVATING PROTEIN ROTUND.//3.70E-19//147aa//41%//P40809
C-NT2RM4002251//ALPHA-1,3-MANNOSYL-GLYCOPROTEIN BETA-1,2-N-ACETYLGLUCOSAMINYLTRANSFERASE (EC 2.4.1.101) (N-GLYCOSYLOLIGOSACCHARIDE-GLYCOPROTEIN N-ACETYLGLUCOSAMINYLTRANSFERASE I) (GNT- I) (GLCNAC-T I).//2.20E-36//320aa//38%//P27808
C-NT2RM4002323//ANTIGEN GOR (FRAGMENT).//0.000000001//154aa//33%//P48778
C-NT2RM4002409//ACETYL-COENZYME A SYNTHETASE (EC 6.2.1.1) (ACETATE--COA LIGASE) (ACYL- ACTIVATING ENZYME).//1.30E-29//275aa//30%//P27095
C-NT2RM4002438//Xenopus laevis putative Zic3 binding protein mRNA, complete cds.//1.10E-49//611 bp//70%//AF129131
C-NT2RM4002460//ENV POLYPROTEIN (COAT POLYPROTEIN) [CONTAINS: COAT PROTEINS GP70, GP20].//0.0000016//226aa//24%//P51515
C-NT2RM4002527//VEGETATIBLE INCOMPATIBILITY PROTEIN HET-E-1.//8.90E-15//366aa//27%//Q00808
C-NT2RM4002532//PROTEIN HOM1.//2.00E-16//276aa//28%//P55137
C-NT2RM4002558//Homo sapiens fatty acid transport protein (FATP) mRNA, complete cds.//0//1797bp//99%//AF055899
C-NT2RM4002565//Mus musculus Sec8 mRNA, complete cds.//0//1915bp//87%//AF022962
C-NT2RM4002571//H.sapiens mRNA for UDP-GalNAc:polypeptide N-acetylgalactosaminyltransferase (T2).//4.60E-78//921bp//69%//X85019
C-NT2RM4002594//MSP1 PROTEIN HOMOLOG.//2.70E-68//236aa//58%//P54815
C-NT2RM4002623//ASPARTYL-TRNA SYNTHETASE (EC 6.1.1.12) (ASPARTATE--TRNA UGASE) (ASPRS).//2.30E-101//488aa//45%//O32038
C-NT2RP1000018//Homo sapiens mRNA for NIK, partial cds.//0//1747bp//99%//AB013385
C-NT2RP1000035//Homo sapiens mRNA for NS1-binding protein (NS1-BP).//0//1652bp//99%//AJ012449
C-NT2RP1000040//Mus musculus donson protein (Donson) mRNA, partial cds.//5.90E-150//1025bp//82%//AF193608
C-NT2RP1000086//H.sapiens mRNA for zinc finger protein, Hsa12.//0//1162bp//99%//X98834
C-NT2RP1000111//COP1 REGULATORY PROTEIN.//4.00E-116//296aa//51%//P93471
C-NT2RP1000130//HEPATOMA-DERIVED GROWTH FACTOR (HDGF).//4.50E-50//181aa//60%//P51859
C-NT2RP1000163//Homo sapiens cell cycle progression 2 protein (CPR2) mRNA, complete cds.//3.40E-270//951bp//98%//AF011792
C-NT2RP1000202//ANKYRIN.//1.00E-25//302aa//34%//Q02357
C-NT2RP1000272//Mus musculus mRNA for neural specific sr protein NSSR 2, complete cds.//1.40E-267//1155bp//87%//AB015895
C-NT2RP1000326//Homo sapiens metaxin 2 (MTX2) mRNA, nuclear gene encoding mitochondrial protein, complete cds.//1.30E-275//1249bp//99%//AF053551
C-NT2RP1000333//ANTI-SILENCING PROTEIN 1.//8.70E-47//155aa//58%//P32447
C-NT2RP1000348//REDUCED VIABILITY UPON STARVATION PROTEIN 161.//1.70E-15//162aa//30%//P25343
C-NT2RP1000363//R.norvegicus LL5 mRNA7/7.90E-262//1175bp//83%//X74226
C-NT2RP1000376//Homo sapiens Ca2⁺-independent phospholipase A2 long isoform (iPLA2) mRNA, complete cds.//0//2252bp//96%//AF102989
C-NT2RP1000413//MEMBRANE-ASSOCIATED PROTEIN HEM-2 (NAP1 PROTEIN).//1.90E-153//230aa//99%//P55161
C-NT2RP1000439//Xenopus laevis chromosome condensation protein XCAP-G mRNA, complete cds.//1.80E-94//1019bp//63%//AF111423
C-NT2RP1000443//QUINONE OXIDOREDUCTASE (EC 1.6.5.5) (NADPH:QUINONE REDUCTASE) (ZETA- CRYSTALLIN).//2.40E-10//227aa//25%//Q08257
C-NT2RP1000460//NUCLEAR MOVEMENT PROTEIN NUDC.//3.80E-19//149aa//36%//P17624
C-NT2RP1000470//PUTATIVE ATP-DEPENDENT RNA HELICASE T26G10.1 IN CHROMOSOME III.//2.60E-94//254aa//47%//P34580
C-NT2RP1000478//TUBULIN BETA-5 CHAIN (CLASS-V).//4.50E-240//445aa//97%//P09653
C-NT2RP1000481//Homo sapiens antigen NY-CO-3 (NY-CO-3) mRNA, partial cds.//7.5e-315//1445bp//99%//AF039688
C-NT2RP1000493//POSSIBLE DNA-REPAIR PROTEIN XP-E (POSSIBLE XERODERMA PIGMENTOSUM GROUP E PROTEIN) (UV-DAMAGED DNA-BINDING PROTEIN) (UV-DDB).//3.60E-30//534aa//23%//P33194
C-NT2RP1000513//Human NifU-like protein (hNifU) mRNA, partial cds.//6.50E-171//516bp//99%//U47101
C-NT2RP1000522//UBIQUTIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//8.20E-83//345aa//47%//Q61068
C-NT2RP1000547//COP-COATED VESICLE MEMBRANE PROTEIN P24 PRECURSOR (FRAGMENT).//1.10E-27//193aa//35%//P49020
C-NT2RP1000574//HOMEOBOX PROTEIN MEIS2 (MEIS1-RELATED PROTEIN 1).//3.50E-75//151aa//94%//P97367
C-NT2RP1000630//NECDIN.//2.40E-44//227aa//41%//P25233
C-NT2RP1000677//SODIUM-INDEPENDENT ORGANIC ANION TRANSPORTER (ORGANIC ANION TRANSPORTING POLYPEPTIDE).//1.20E-78//483aa//31%//P46721
C-NT2RP1000701//Homo sapiens phospholipase A2 activating protein (PLA2P) mRNA, complete cds.//0//1687bp//99%//AF145020
C-NT2RP1000733//Human mRNA for GSPT1-TK protein,complete cds.//0//2057bp//99%//E14379
C-NT2RP1000738//Homo sapiens Wolf-Hirschhorn syndrome candidate 2 protein (WHSC2) mRNA, complete cds.//0//2186bp//99%//AF101434
C-NT2RP1000746//Homo sapiens 60S acidic ribosomal protein PO mRNA, complete cds.//9.70E-196//901bp//99%//AF173378
C-NT2RP1000782//PLATELET-ENDOTHELIAL TETRASPAN ANTIGEN 3 (PETA-3) (GP27) (MEMBRANE GLYCOPROTEIN SFA-1) (CD151 ANTIGEN).//1.20E-30//232aa//30%//O35566
C-NT2RP1000825//GTPASE-ACTIVATING PROTEIN RHOGAP (RHO-RELATED SMALL GTPASE PROTEIN ACTIVATOR) (CDC42 GTPASE-ACTIVATING PROTEIN) (P50-RHOGAP).//8.20E-83//334aa//50%//Q07960
C-NT2RP1000833//Homo sapiens cGMP phosphodiesterase Al (PDE9A) mRNA, complete cds.//0//1494bp//99%//AF067223
C-NT2RP1000834//Homo sapiens alpha-methylacyl-CoA racemase mRNA, complete cds.//1.80E-176//829bp//98%//AF047020
C-NT2RP1000856//PLATELET-ENDOTHELIAL TETRASPAN ANTIGEN 3 (PETA-3) (GP27) (MEMBRANE GLYCOPROTEIN SFA-1) (CD151 ANTIGEN).//1.20E-30//232aa//30%//O35566
C-NT2RP1000860//Homo sapiens KL04P mRNA, complete cds.//0//1555bp//99%//AF064094
C-NT2RP1000902//HYPOTHETICAL 127.4 KD PROTEIN F07F6.4 IN CHROMOSOME III.//5.20E-20//306aa//33%//Q09531
C-NT2RP1000915//AUTOANTIGEN NGP-1.//1.70E-19//343aa//25%//Q13823
C-NT2RP1000947//Human E2 ubiquitin conjugating enzyme UbcH5B (UBCH5B) mRNA, complete cds.//4.60E-105//504bp//99%//U39317
C-NT2RP1000954//RING CANAL PROTEIN (KELCH PROTEIN).//1.40E-23//370aa//28%//Q04652
C-NT2RP1000958//AUTOANTIGEN NGP-1.//1.40E-19//343aa//25%//Q13823
C-NT2RP1000959//Human acidic ribosomal phosphoprotein P0 mRNA, complete cds.//2.50E-236//966bp//99%//M17885
C-NT2RP1000966//NUCLEOLIN (PROTEIN C23).//8.90E-299//554aa//99%//P19338
C-NT2RP1001011//Drosophila melanogaster putative 43 kDa protein (TH1) mRNA, complete cds.//2.20E-78//1529bp//61%//L01790
C-NT2RP1001013//ZINC FINGER PROTEIN 83 (ZINC FINGER PROTEIN HPF1).//7.70E-253//425aa//98%//P51522
C-NT2RP1001033//Homo sapiens delta-tubulin mRNA, complete cds.//2.10E-285//1290bp//100%//AF201333
C-NT2RP1001073//Homo sapiens U6 snRNA-associated Sm-like protein LSm5 mRNA, complete cds.//8.10E-107//504bp//99%//AF182291
C-NT2RP1001079//Oryctolagus cuniculus sarcosine oxidase (SOX) mRNA, complete cds.//0//2085bp//99%//U82267
C-NT2RP1001080//PROBABLE ATP-DEPENDENT RNA HELICASE DBP9.//2.30E-116//319aa//46%//Q06218
C-NT2RP1001113//Homo sapiens CTL2 gene.//0//2790bp//98%//AJ245621
C-NT2RP1001177//Rattus norvegicus histone macroH2A1.2 mRNA, complete cds.//5.20E-108//1278bp//69%//U79139
C-NT2RP1001185//Human isovaleryl-coA dehydrogenase (IVD) mRNA, complete cds.//1.90E-158//729bp//99%//M34192
C-NT2RP1001247//Homo sapiens TGF-beta type secreted signaling protein LEFTYA mRNA, complete cds.//0//2006bp//100%//AF081513
C-NT2RP1001253//Homo sapiens oscillin (hLn) mRNA, complete cds.//0//2020bp//99%//AF029914
C-NT2RP1001294//MICROTUBULE-ASSOCIATED PROTEIN YTM1.//1.80E-38//258aa//32%//Q12024
C-NT2RP1001302//MICROTUBULE-ASSOCIATED PROTEIN YTM1.//1.80E-38//258aa//32%//Q12024
C-NT2RP1001310//Homo sapiens mitochondrial carrier homolog 1 isoform a mRNA, partial cds; nuclear gene for mitochondrial product.//0//1732bp//99%//AF176006
C-NT2RP1001313//Homo sapiens delta-6 fatty acid desaturase mRNA, complete cds.//7.50E-121//1394bp//69%//AF126799
C-NT2RP1001361//Homo sapiens NADH-ubiquinone oxidoreductase subunit B14.5B homolog mRNA, complete cds.//6.50E-116//541bp//100%//AF070652
C-NT2RP1001385//HYPOTHETICAL 48.8 KD PROTEIN IN SSU81-SCS2 INTERGENIC REGION.//2.70E-22//284aa//25%//P40074
C-NT2RP1001395//Homo sapiens COP9 complex subunit 7a mRNA, complete cds.//0//1782bp//99%//AF210052
C-NT2RP1001410//PUTATIVE GTP-BINDING PROTEIN W08E3.3.//8.90E-141//396aa//67%//P91917
C-NT2RP1001449//Mus musculus Gng31g mRNA, complete cds.//7.20E-165//800bp//87%//AF069954
C-NT2RP1001457//Homo sapiens partial mRNA for beta-transducin family protein (putative).//1.20E-137//629bp//100%//AJ005257
C-NT2RP1001482//Mouse oncogene (ect2) mRNA, complete cds.//2.10E-158//755bp//86%//L11316
C-NT2RP1001494//MALE STERILITY PROTEIN 2.//7.20E-40//261aa//27%//Q08891
C-NT2RP1001543//MYO-INOSITOL-1-PHOSPHATE SYNTHASE (EC 5.5.1.4) (IPS).//1.60E-166//506aa//60%//P42803
C-NT2RP1001546//PLATELET-ENDOTHELIAL TETRASPAN ANTIGEN 3 (PETA-3) (GP27) (MEMBRANE GLYCOPROTEIN SFA-1) (CD151 ANTIGEN).//1.60E-30//232aa//30%//O35566
C-NT2RP1001569//SIGNAL RECOGNITION PARTICLE RECEPTOR BETA SUBUNIT (SR-BETA).//5.80E-121//271aa//89%//P47758
C-NT2RP1001665//CALMODUUN.//0.00000051//83aa//30%//P02594
C-NT2RP2000006//DNAJ PROTEIN (40 KD HEAT SHOCK CHAPERONE PROTEIN) (HSP40).//9.80E-17//79aa//55%//O34136
C-NT2RP2000008//ZINC FINGER PROTEIN 84 (ZINC FINGER PROTEIN HPF2).//2.40E-177//726aa//47%//P51523
C-NT2RP2000032//RAS SUPPRESSOR PROTEIN 1 (RSU-1) (RSP-1 PROTEIN) (RSP-1)7/1.80E-22//184aa//34%//Q01730
C-NT2RP2000045//Homo sapiens tumorous imaginal discs protein Tid56 homolog (TID1) mRNA, complete cds.//0//1390bp//98%//AF061749
C-NT2RP2000054//Homo sapiens putative ring zinc finger protein NY-REN-43 antigen mRNA, complete cds.//0//2245bp//99%//AF155109
C-NT2RP2000056//PROTEIN-TYROSINE PHOSPHATASE EPSILON PRECURSOR (EC 3.1.3.48) (R-PTP- EPSILON).//9.40E-16//45aa//100%//P49446
C-NT2RP2000067//Mus musculus ODZ3 (Odz3) mRNA, partial cds.//0//3546bp//99%//AF195418
C-NT2RP2000070//CADHERIN-RELATED TUMOR SUPPRESSOR PRECURSOR (FAT PROTEIN).//3.40E-51//383aa//32%//P33450
C-NT2RP2000076//Homo sapiens partial mRNA for polyhomeotic 2 protein (PH2 gene).//7.90E-20//265bp//73%//AJ242730
C-NT2RP2000114//Homo sapiens mRNA for GM3 synthase, complete cds.//0//2244bp//99%//AB018356
C-NT2RP2000126//POSSIBLE GLOBAL TRANSCRIPTION ACTIVATOR SNF2L.//2.50E-117//541aa//42%//P41877
C-NT2RP2000133//Homo sapiens Leman coiled-coil protein (LCCP) mRNA, complete cds.//0//1490bp//99%//AF175966
C-NT2RP2000147//CLATHRIN COAT ASSEMBLY PROTEIN AP47 (CLATHRIN COAT ASSOCIATED PROTEIN AP47) (GOLGI ADAPTOR AP-1 47 KD PROTEIN) (HA1 47 KD SUBUNIT) (CLATHRIN ASSEMBLY PROTEIN ASSEMBLY PROTEIN COMPLEX 1 MEDIUM CHAIN).//4.40E-226//423aa//99%//P35585
C-NT2RP2000153//GAR2 PROTEIN.//9.80E-23//311aa//28%//P41891
C-NT2RP2000157//MLO2 PROTEIN.//2.60E-11//62aa//40%//Q09329
C-NT2RP2000161//DIS3 PROTEIN HOMOLOG.//4.10E-35//184aa//44%//Q17632
C-NT2RP2000183//DIHYDROPYRIMIDINASE RELATED PROTEIN-2 (DRP-2) (NEURAL SPECIFIC PROTEIN NSP60).//3.30E-16//114aa//44%//O02675
C-NT2RP2000195//Homo sapiens androgen induced protein (AIG-1) mRNA, complete cds.//7.80E-152//704bp//99%//AF153605
C-NT2RP2000224//INSULIN RECEPTOR SUBSTRATE-1 (IRS-1).//0.000043//103aa//28%//P35568
C-NT2RP2000248//UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).//3.40E-21//210aa//33%//P56558
C-NT2RP2000257//PUTATIVE MITOCHONDRIAL CARRIER YIL006W.//9.70E-41//278aa//36%//P40556
C-NT2RP2000258//ACTIVATOR 1 140 KD SUBUNIT (REPLICATION FACTOR C LARGE SUBUNIT) (Al 140 KD SUBUNIT) (RF-C 140 KD SUBUNIT) (ACTIVATOR 1 LARGE SUBUNIT) (DNA-BINDING PROTEIN PO-GA).//7.10E-12//213aa//23%//P35251
C-NT2RP2000270//Human putative G-protein coupled receptor (SH120) mRNA, complete cds.//1.30E-242//1043bp//99%//U78723
C-NT2RP2000288//HYPOTHETICAL 111.9 KD PROTEIN C22H10.03C IN CHROMOSOME I.//1.60E-27//576aa//25%//Q10297
C-NT2RP2000297//ZINC FINGER PROTEIN 184 (FRAGMENT).//3.30E-186//256aa//60%//Q99676
C-NT2RP2000310//Human proline dehydrogenase/proline oxidase (PRODH) mRNA, complete cds.//4.30E-279//1193bp//99%//U82381
C-NT2RP2000329//GTP:AMP PHOSPHOTRANSFERASE MITOCHONDRIAL (EC 2.7.4.10) (AK3).//2.00E-111//226aa//92%//P08760
C-NT2RP2000346//Homo sapiens apoptosis associated protein (GADD34) mRNA, complete cds.//0//2331bp//99%//U83981
C-NT2RP2000414//Homo sapiens HnRNP F protein mRNA, complete cds.//0//1886bp//99%//L28010
C-NT2RP2000420//ZINC FINGER PROTEIN 165.//8.50E-33//155aa//52%//P49910
C-NT2RP2000422//Homo sapiens N-acetylglucosamine-phosphate mutase mRNA, complete cds.//0//1757bp//99%//AF102265
C-NT2RP2000448//KES1 PROTEIN.//8.70E-54//392aa//38%//P35844
C-NT2RP2000523//APOLIPOPROTEIN B MRNA EDITING PROTEIN (HEPR) (APOBEC-1).//6.00E-16//124aa//34%//P41238
C-NT2RP2000660//SAP1 PROTEIN.//5.20E-68//474aa//32%//P39955
C-NT2RP2000668//SERINE/THREONINE PROTEIN KINASE PKPA (EC 2.7.1.-).//1.30E-27//349aa//32%//Q01577
C-NT2RP2000710//ASPARTYL-TRNA SYNTHETASE (EC 6.1.1.12) (ASPARTATE--TRNA LIGASE)//2.70E-100//488aa//44%//O32038
C-NT2RP2000764//NIFS PROTEIN.//6.60E-36//252aa//42%//P12623
C-NT2RP2000809//Homo sapiens BAG-family molecular chaperone regulator-5 mRNA, complete cds.//0//3347bp//99%//AF095195
C-NT2RP2000812//DILUTE MYOSIN HEAVY CHAIN, NON-MUSCLE (MYOSIN 5A).//5-.60E-08//179aa//29%//Q99104
C-NT2RP2000814//GELATION FACTOR (ACTIN BINDING PROTEIN 120) (ABP-120).//1.10E-07//96aa//29%//P13466
C-NT2RP2000816//MAGNESIUM-CHELATASE 30 KD SUBUNIT.//7.90E-08//172aa//28%//P26174
C-NT2RP2000842//Human lysophosphatidic acid receptor homolog mRNA, complete cds.//0//1562bp//99%//U80811
C-NT2RP2000880//PROBABLE TRANSLATION INITIATION FACTOR IF-2.//0//694aa//99%//O60841
C-NT2RP2000892//Rattus norvegicus db83 mRNA, complete cds.//2.90E-191//1094bp//85%//AB006135
C-NT2RP2000931//MATRIN 3.//2.40E-289//467aa//95%//P43244
C-NT2RP2000943//Homo sapiens sec24D protein mRNA, complete cds.//0//2767bp//99%//AF130464
C-NT2RP2000965//Homo sapiens mRNA for fls353, complete cds.//0//1989bp//96%//AB024704
C-NT2RP2001070//PUTATIVE PYRIDOXAMINE 5'-PHOSPHATE OXIDASE (EC 1.4.3.5) (PNP/PMP OXIDASE).//5.80E-46//222aa//45%//Q20939
C-NT2RP2001081//SYNAPTOTAGMIN IV.//4.20E-118//430aa//54%//P50232
C-NT2RP2001127//Homo sapiens mRNA for PLU-1 protein.//0//2514bp//99%//AJ132440
C-NT2RP2001168//VERPROLIN.//1.50E-09//143aa//33%//P37370
C-NT2RP2001174//GASTRULA ZINC FINGER PROTEIN XLCGF46.1 (FRAGMENT).//6.00E-10//88aa//38%//P18722
C-NT2RP2001233//ZINC FINGER PROTEIN 91 (ZINC FINGER PROTEIN HTF10) (HPF7).//2.00E-128//409aa//45%//Q05481
C-NT2RP2001245//MYOSIN HEAVY CHAIN, NONMUSCLE (CELLULAR MYOSIN HEAVY CHAIN) (NMMHC).//2.20E-10//366aa//28%//P14105
C-NT2RP2001290//BETA-SOLUBLE NSF ATTACHMENT PROTEIN (SNAP-BETA) (SNAP-ALPHA HOMOLOG) (BRAIN PROTEIN 147) (FRAGMENT).//4.40E-91//179aa//99%//P28663
C-NT2RP2001295//ZINC/CADMIUM RESISTANCE PROTEIN.//8.30E-39//161aa//34%//P20107
C-NT2RP2001327//TUMOR NECROSIS FACTOR, ALPHA-INDUCED PROTEIN 1, ENDOTHELIAL (B12 PROTEIN).//5.50E-116//311aa//71%//Q13829
C-NT2RP2001378//MUCIN 2 PRECURSOR (INTESTINAL MUCIN 2).//2.00E-11//403aa//25%//Q02817
C-NT2RP2001392//MTTOCHONDRIAL LON PROTEASE HOMOLOG 1 PRECURSOR (EC 3.4.21.-).//8.40E-192//581aa//54%//P93647
C-NT2RP2001394//Homo sapiens mRNA for SCML2 protein.//0//2068bp//99%//Y18004
C-NT2RP2001397//Homo sapiens mRNA for cyclin B2, complete cds.//1.9e-316//1428bp//100%//AB020981
C-NT2RP2001420//Mus musculus nuclear protein NIP45 mRNA, complete cds.//9.00E-112//742bp//82%//U76759
C-NT2RP2001440//Homo sapiens mRNA for 14-3-3gamma, complete cds.//0//3712bp//99%//AB024334
C-NT2RP2001460//TRICHOHYAUN.//1.00E-14//521aa//24%//P37709
C-NT2RP2001511//Homo sapiens putative RNA-binding protein Q99 mRNA, complete cds.//3.20E-297//2206bp//75%//AF093097
C-NT2RP2001520//Homo sapiens mRNA for mitochondrial carrier protein ARALAR1.//0//2502bp//99%//Y14494
C-NT2RP2001536//Homo sapiens X-ray repair cross-complementing protein 3 (XRCC3) mRNA, complete cds.//0//2326bp//99%//AF035586
C-NT2RP2001560//VAV2 PROTEIN.//0.00000015//219aa//27%//Q60992
C-NT2RP2001576//HYPOTHETICAL 62.2 KD PROTEIN C4G8.12C IN CHROMOSOME I.//8.20E-29//294aa//31%//Q09837
C-NT2RP2001597//RYANODINE RECEPTOR, CARDIAC MUSCLE.//0.000000036//127aa//36%//P30957
C-NT2RP2001601//Homo sapiens SUMO-1-specific protease (SSP1) mRNA, complete cds.//0//1748bp//99%//AF196304
C-NT2RP2001613//MITOCHONDRIAL IMPORT RECEPTOR SUBUNIT TOM40 (MOM38 PROTEIN) (TRANSLOCASE OF OUTER MEMBRANE 40 KD SUBUNIT).//6.10E-12//184aa//31%//P24391
C-NT2RP2001634//Homo sapiens alpha-catenin-like protein mRNA, complete cds.//0//2445bp//99%//U97067
C-NT2RP2001660//Homo sapiens putative 13 S Golgi transport complex 90kD subunit brain-specific isoform mRNA, complete cds.//0//1287bp//99%//AF058718
C-NT2RP2001663//ENOLASE (EC 4.2.1.11) (2-PHOSPHOGLYCERATE DEHYDRATASE), (2-PHOSPHO-D- GLYCERATE HYDRO-LYASE) (FRAGMENT).//1.10E-47//126aa//53%//P42897
C-NT2RP2001740//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//7.90E-52//220aa//44%//Q61068
C-NT2RP2001748//FARNESYL PYROPHOSPHATE SYNTHETASE (FPP SYNTHETASE) (FPS) (FARNESYL DIPHOSPHATE SYNTHETASE) (DIMETHYLALLYLTRANSFERASE (EC 2.5.1.1) / GERANYLTRANSTRANSFERASE (EC 2.5.1.10)) (KIAA0032).//5.40E-47//96aa/797%//P14324
C-NT2RP2001756//ZINC FINGER PROTEIN 84 (ZINC FINGER PROTEIN HPF2).//1.70E-49//411aa//32%//P51523
C-NT2RP2001839//SCY1 PROTEIN.//5.40E-32//621aa//24%//P53009
C-NT2RP2001869//ZINC FINGER PROTEIN 191.//7.10E-26//126aa//52%//O14754
C-NT2RP2001876//ALLOGRAFT INFLAMMATORY FACTOR-1 (AIF-1) (IONIZED CALCIUM BINDING ADAPTER MOLECULE 1).//1.20E-45//141aa//65%//P55008
C-NT2RP2001883//Homo sapiens CGI-01- protein mRNA, complete cds.//0//2306bp//99%//AF132936
C-NT2RP2001898//Human inositol polyphosphate 5-phosphatase (5ptase) mRNA, 3' end.//0//2518bp//98%//M74161
C-NT2RP2001900//ACTIN-LIKE PROTEIN ARP5.//2.30E-38//395aa//30%//P53946
C-NT2RP2001976//Mus musculus calmodulin-binding protein SHA1 (Sha1) mRNA, complete cds.//4.70E-177//1538bp//74%//AF062378
C-NT2RP2001985//Homo sapiens high-risk human papilloma viruses E6 oncoproteins targeted protein E6TP1 alpha mRNA, complete cds.//2.00E-38//435bp//67%//AF090989
C-NT2RP2001991//SODIUM- AND CHLORIDE-DEPENDENT TRANSPORTER NTT73.//6.50E-129//279aa//85%//Q08469
C-NT2RP2002025//NG-CAM RELATED CELL ADHESION MOLECULE PRECURSOR (NR-CAM) (BRAVO).//1.70E-47//247aa//52%//P35331
C-NT2RP2002046//Homo sapiens mRNA for transcription factor.//0//1664bp//99%//AJ130894
C-NT2RP2002058//Homo sapiens WD repeat protein WDR3 (WDR3) mRNA, complete cds.//0//2510bp//99%//AF083217
C-NT2RP2002066//Rattus norvegicus transmembrane receptor Unc5H2 mRNA, complete cds.//1.60E-226//1301bp//88%//U87306
C-NT2RP2002078//PECANEX PROTEIN.//1.80E-09//195aa//32%//P18490
C-NT2RP2002079//HISTONE HI, GONADAL.//4.40E-11//214aa//34%//P02256
C-NT2RP2002099//Homo sapiens mRNA for E1B-55kDa-associated protein.//0//33 89bp//99%//AJ007509
C-NT2RP2002105//H.sapiens MSH-R gene for melanocyte stimulating hormone receptor.//0//1644bp//98%//X65634
C-NT2RP2002124//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN HOMOLOG).//4.30E-44//155aa//37%//Q13107
C-NT2RP2002185//Homo sapiens ubiquilin mRNA, complete cds.//0//1789bp//99%//AF176069
C-NT2RP2002193//Homo sapiens PIAS3 mRNA for protein inhibitor of activatied STAT3, complete cds.//0//2809bp//99%//AB021868
C-NT2RP2002252//Mus musculus (clone pVZmSin3A9) mSin3A9 mRNA, complete cds.//0//3118bp//91%//L38621
C-NT2RP2002256//Homo sapiens retinoic acid hydroxylase mRNA, complete cds.//0//1528bp//98%//AF005418
C-NT2RP2002270//AF-9 PROTEIN.//1.20E-07//74aa//36%//P42568
C-NT2RP2002312//Homo sapiens mRNA for CDS2 protein.//0//2333bp//99%//Y16521
C-NT2RP2002325//Homo sapiens mRNA for Pex11p, complete cds.//8.40E-254//1158bp//99%//AB015594
C-NT2RP2002385//Homo sapiens synaptic glycoprotein SC2 spliced variant mRNA, complete cds.//4.30E-240//1105bp//99%//AF038958
C-NT2RP2002408//Homo sapiens mRNA for TOLLIP protein.//3.20E-210//1136bp//93%//AJ242972
C-NT2RP2002442//HESA PROTEIN.//2.80E-14//163aa//30%//P46037
C-NT2RP2002464//DNA CROSS-LINK REPAIR PROTEIN PSO2/SNM1.//6.50E-07//171aa//27%//P30620
C-NT2RP2002479//Homo sapiens mRNA for ABC transporter 7 protein, complete cds.//0//2180bp//99%//AB005289
C-NT2RP2002503//ZINC FINGER PROTEIN 45 (BRC1744).//4.60E-144//537aa//49%//Q02386
C-NT2RP2002520//Homo sapiens transcription factor RFX-B (RFXB) mRNA, complete cds.//3.70E-34//668bp//61%//AF105427
C-NT2RP2002537//HYPOTHETICAL 55.1 KD PROTEIN B0416.5 IN CHROMOSOME X.//6.20E-19//288aa//26%//Q11073
C-NT2RP2002591//ZINC FINGER PROTEIN 84 (ZINC FINGER PROTEIN HPF2).//1.20E-155//562aa//50%//P51523
C-NT2RP2002595//PROBABLE CALCIUM-BINDING PROTEIN ALG-2 (PMP41) (ALG-257).//7.50E-35//181aa//42%//P12815
C-NT2RP2002606//Rattus norvegicus Rabin3 mRNA, complete cds.//9.20E-147//874bp//87%//U19181
C-NT2RP2002609//2-HYDROXYMUCONIC SEMIALDEHYDE HYDROLASE (EC 3.1.1.-) (HMSH).//2.80E-08//109aa//37%//P19076
C-NT2RP2002618//PROTEIN ARGININE N-METHYLTRANSFERASE 2 (EC 2.1.1.).//1.70E-51//326aa//38%//P55345
C-NT2RP2002701//HYPOTHETICAL 38.1 KD PROTEIN C2F12.15C IN CHROMOSOME II.//1.90E-14//210aa//30%//O14345
C-NT2RP2002710//SH3-BINDING PROTEIN 3BP-1.//4.90E-85//489aa//43%//P55194
C-NT2RP2002727//Rattus norvegicus tulip 2 mRNA, complete cds.//3.50E-74//727bp//72%//AF041107
C-NT2RP2002741//Homo sapiens mRNA for Neuroblastoma, complete cds.//9.90E-54//964bp//64%//D89016
C-NT2RP2002862//60S ACIDIC RIBOSOMAL PROTEIN P0 (LIGHT-INDUCED 34 KD PROTEIN).//8.80E-10//203aa//27%//P29764
C-NT2RP2002880//GLUCOSE REPRESSION MEDIATOR PROTEIN.//0.000039//206aa//23%//P14922
C-NT2RP2002928//Homo sapiens pre-mRNA splicing factor (PRP17) mRNA, complete cds.//1.90E-136//623bp//100%//AF038392
C-NT2RP2002929//HYPOTHETICAL 46.2 KD TRP-ASP REPEATS CONTAINING PROTEIN D2013.2 IN CHROMOSOME II.//4.10E-87//395aa//40%//Q18964
C-NT2RP2002939//ZINC FINGER PROTEIN 136.//5.40E-70//282aa//42%//P52737
C-NT2RP2002959//UBIQUITIN-CONJUGATING ENZYME E2-17 KD 2 (EC 6.3.2.19) (UBIQUITIN- PROTEIN LIGASE) (UBIQUITIN CARRIER PROTEIN) (E2(17)KB 2).//4.60E-80//147aa//100%//P51669
C-NT2RP2002980//30S RIBOSOMAL PROTEIN S10.//1.00E-08//98aa//36%//P10129
C-NT2RP2002986//Homo sapiens mRNA for Kelch motif containing protein, complete cds.//0//2209bp//99%//AB026190
C-NT2RP2002993//DNA-DIRECTED RNA POLYMERASE 1135 KD POLYPEPTIDE (EC 2.7.7.6) (RNA POLYMERASE I SUBUNIT 2) (RPA135).//0//716aa//91%//P70700
C-NT2RP2003000//TUMOR NECROSIS FACTOR, ALPHA-INDUCED PROTEIN 1, ENDOTHELIAL (B12 PROTEIN).//L90E-11//132aa//38%//Q13829
C-NT2RP2003121//Mus musculus enhancer of polycbmb (Epc1) mRNA, complete cds.//2.30E-82//642bp//68%//AF079765
C-NT2RP2003125//RING CANAL PROTEIN (KELCH PROTEIN).//2.40E-38//539aa//25%//Q04652
C-NT2RP2003137//UBIQUITlN.//0.000026//70aa//30%//P13117
C-NT2RP2003157//SPLICING FACTOR, ARGININE/SERINE-RICH 4 (PRE-MRNA SPLICING FACTOR SRP75).//1.30E-13//185aa//38%//Q08170
C-NT2RP2003158//Homo sapiens mRNA for proteasome subunit p58, complete cds.//0//2091bp//99%//D67025
C-NT2RP2003164//Homo sapiens mRNA for protein kinase.//0//2313bp//99%//AJ132545
C-NT2RP2003177//Homo sapiens recombination and sister chromatid cohesion protein homolog (hrec8) mRNA, partial cds.//0//1641bp//99%//AF006264
C-NT2RP2003228//H.sapiens Pl-Cdc21 mRNA.//0//2870bp//98%//X74794
C-NT2RP2003230//Rattus norvegicus endo-alpha-D-mannosidase (Enman) mRNA, complete cds.//2.60E-186//1551bp//77%//AF023657
C-NT2RP2003243//Homo sapiens partial mRNA for putative p621 protein which interacts with transcription factor Sp1.//0//1544bp//99%//AJ242978
C-NT2RP2003265//Homo sapiens CGI-53 protein mRNA, complete cds.//0//1580bp//99%//AF151811
C-NT2RP2003272//Homo sapiens ubiquilin mRNA, complete cds.//0//1789bp//99%//AF176069
C-NT2RP2003277//NAM7 PROTEIN (NONSENSE-MEDIATED MRNA DECAY PROTEIN 1) (UP-FRAMESHIFT SUPPRESSOR 1).//1.90E-16//145aa//43%//P30771
C-NT2RP2003286//PROBABLE RNA 3'-TERMINAL PHOSPHATE CYCLASE (EC 6.5.1.4) (RNA-3'- PHOSPHATE CYCLASE) (RNA CYCLASE).//4.20E-88//374aa//47%//Q23400
C-NT2RP2003295//Homo sapiens RMP mRNA for RPB5 meidating protein, complete cds.//0//1526bp//99%//AB006572
C-NT2RP2003307//KINESIN LIGHT CHAIN (KLC).//2.20E-199//550aa//70%//Q07866
C-NT2RP2003308//CROOKED NECK PROTEIN.//5.40E-244//622aa//67%//P17886
C-NT2RP2003329//PUTATIVE ADENYLATE CYCLASE REGULATORY PROTEIN.//3.60E-14//332aa//32%//P26337
C-NT2RP2003347//BREAST CANCER TYPE 1 SUSCEPTIBILITY PROTEIN HOMOLOG.//0.000022//261aa//24%//P48754
C-NT2RP2003391//Homo sapiens mRNA for nuclear transport receptor.//0//1509bp//99%//AJ133769
C-NT2RP2003394//INTRACELLULAR PROTEIN TRANSPORT PROTEIN USO1.//5.50E-13//302aa//26%//P25386
C-NT2RP2003401//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//9.60E-78//346aa//43%//Q61068
C-NT2RP2003433//PROTEIN TRANSPORT PROTEIN SEC61 ALPHA SUBUNIT.//5.00E-131//269aa//91%//P38378
C-NT2RP2003466//Homo sapiens delta-6 fatty acid desaturase mRNA, complete cds.//0//2194bp//99%//AF126799
C-NT2RP2003480//Homo sapiens zinc finger DNA binding protein 99 (ZNF281) mRNA, complete cds.//0//3012bp//99%//AF125158
C-NT2RP2003506//NADPH-CYTQCHROME P450 REDUCTASE (EC 1.6.2.4) (CPR).//5.40E-14//106aa//46%//P04175
C-NT2RP2003513//Homo sapiens mRNA for paralemmin.//0//2137bp//97%//Y14770
C-NT2RP2003517//Human c-sis/platelet-derived growth factor 2 (SIS/PDGF2) mRNA, complete cds.//0//1746bp//95%//M12783
C-NT2RP2003522//Homo sapiens zinc finger DNA binding protein 99 (ZNF281) mRNA, complete cds.//0//1764bp//99%//AF125158
C-NT2RP2003543//HYPOTHETICAL TRNA/RRNA METHYLTRANSFERASE SLR1673 (EC 2.1.1.-).//1.70E-17//148aa//34%//P74261
C-NT2RP2003564//52 KD RO PROTEIN (SJOGREN SYNDROME TYPE A ANTIGEN (SS-A))(RO(SS-A)).//2.10E-59//270aa//46%//P19474
C-NT2RP2003596//Mus musculus Fas-apoptosis inhibitory molecule (Faim) mRNA, complete cds.//4.80E-82//530bp//85%//AF130367
C-NT2RP2003604//Homo sapiens alpha-catenin-like protein (CTNNAL1) mRNA, complete cds.//0//2442bp//99%//AF030233
C-NT2RP2003643//Mus musculus mRNA for CMP-N-acetylneuraminic acid synthetase.//9.40E-243//1624bp//82%//AJ006215
C-NT2RP2003702//Homo sapiens 17 beta-hydroxysteroid dehydrogenase type VII (HSD17B7) mRNA, complete cds.//2.1e-313//978bp//99%//AF098786
C-NT2RP2003704//Homo sapiens mRNA for ATP-dependent metalloprotease YME1L.//1.80E-72//350bp//100%//AJ132637
C-NT2RP2003713//Homo sapiens ubiquitin-specific protease 3 (USP3) mRNA, complete cds.//0//2018bp//99%//AF073344
C-NT2RP2003714//ZINC FINGER PROTEIN 91 (ZINC FINGER PROTEIN HTF10) (HPF7).//5.40E-29//85aa//72%//Q05481
C-NT2RP2003737//UBIQUITIN-CONJUGATING ENZYME E2-17 KD 2 (EC 6.3.2.19) (UBIQUITIN-PROTEIN LIGASE) (UBIQUITIN CARRIER PROTEIN) (E2(17)KB 2).//1.70E-75//147aa//93%//P51669
C-NT2RP2003760//COATOMER GAMMA SUBUNIT (GAMMA-COAT PROTEIN) (GAMMA-COP).//0//869aa//80%//P53620
C-NT2RP2003781//HYPOTHETICAL 36.7 KD PROTEIN AH6.2 IN CHROMOSOME II.//5.50E-63//253aa//50%//Q09201
C-NT2RP2003840//HYPOTHETICAL 48.1 KD PROTEIN B0403.2 IN CHROMOSOME X.//3.70E-21//137aa//43%//Q11076
C-NT2RP2003857//MYOTROPHIN (V-1 PROTEIN) (GRANULE CELL DIFFERENTIATION PROTEIN).//0.00000016//117aa//29%//Q91955
C-NT2RP2003871//Homo sapiens transposon-derived Buster1 transposase-like protein gene, complete cds.//0//2807bp//99%//AF205601
C-NT2RP2003912//SERINE/THREONINE-PROTEIN KINASE NEK1 (EC 2.7.1.-) (NIMA-RELATED PROTEIN KINASE 1).//6.10E-183//387aa//87%//P51954
C-NT2RP2003952//AMINOPEPTIDASE B (EC 3.4.11.6) (ARGINYL AMINOPEPTIDASE) (ARGININE AMINOPEPTIDASE) (CYTOSOL AMINOPEPTIDASE IV) (AP-B).//1.50E-23//200aa//30%//O09175
C-NT2RP2003981//VACUOLAR PROTEIN SORTING-ASSOCIATED PROTEIN VPS8.//1.40E-16//664aa7/20%//P39702
C-NT2RP2004013//TRANSCRIPTION FACTOR BTF3 (RNA POLYMERASE B TRANSCRIPTION FACTOR 3).//2.30E-53//141aa//78%//P20290
C-NT2RP2004041//SYNAPSINS IA AND B.//0.00000074//159aa//32%//P17599
C-NT2RP2004066//Mus musculus Msx2 interacting nuclear target protein mRNA, complete cds.//2.70E-288//1994bp//81%//AF156529
C-NT2RP2004098//ADENYLATE CYCLASE (EC 4.6.1.1) (ATP PYROPHOSPHATE-LYASE) (ADENYLYL CYCLASE).//5.40E-30//319aa//31%//Q01513
C-NT2RP2004170//Homo sapiens mRNA for transducin (beta) like 1 protein.//1.10E-138//1236bp//74%//Y12781
C-NT2RP2004187//ZINC FINGER PROTEIN 38 (ZFP-38) (CTFIN51) (TRANSCRIPTION FACTOR RU49).//5.60E-31//424aa//28%//Q07231
C-NT2RP2004194//Rattus norvegicus Golgi SNARE GS15 mRNA, complete cds.//3.80E-52//397bp//82%//AF003998
C-NT2RP2004232//Homo sapiens EPK2 mRNA for serine/threonine kinase, complete cds.//0//2272bp//99%//AB015982
C-NT2RP2004239//Homo sapiens lok mRNA for protein kinase, complete cds.//0//3044bp//99%//AB015718
C-NT2RP2004242//NEUROFILAMENT TRIPLET H PROTEIN (200 KD NEUROFILAMENT PROTEIN) (NF-H).//9.90E-12//427aa//26%//P19246
C-NT2RP2004245//Mus musculus pantothenate kinase 1 beta (panKlbeta) mRNA, complete cds.//6.40E-117//1122bp//72%//AF200357
C-NT2RP2004270//PROTEIN PTM1 PRECURSOR.//1.40E-16//334aa//24%//P32857
C-NT2RP2004366//VACUOLAR PROTEIN SORTING-ASSOCIATED PROTEIN VPS 13.//1.30E-51//505aa//29%//Q07878
C-NT2RP2004389//PROBABLE MITOCHONDRIAL 40S RIBOSOMAL PROTEIN S9 PRECURSOR.//9.30E-15//126aa//39%//P38120
C-NT2RP2004392//MNN4 PROTEIN7/1.40E-11//143aa//27%//P36044
C-NT2RP2004396//Homo sapiens mRNA for activator of S phase Kinase, complete cds.//5.40E-243//1108bp//99%//AB028069
C-NT2RP2004425//Mus musculus axotrophin mRNA, complete cds.//0//2321bp//86%//AF155739
C-NT2RP2004476//Homo sapiens cyclin L ania-6a mRNA, complete cds.//0//2075bp//99%//AF180920
C-NT2RP2004538//Mus musculus kinesin-like protein KIF1B (Kif1b) mRNA, complete cds.//0//1387bp//86%//AF090190
C-NT2RP2004568//PUTATIVE ATP-DEPENDENT RNA HELICASE C30D11.03.//3.00E-117//625aa//40%//Q09903
C-NT2RP2004587//NEUROFILAMENT TRIPLET M PROTEIN (160 KD NEUROFILAMENT PROTEIN) (NF-M).//7.30E-07//352aa//23%//P07197
C-NT2RP2004655//Homo sapiens mRNA for leucine rich protein.//8.50E-233//1061bp//99%//AJ006291
C-NT2RP2004681//NEUROFILAMENT TRIPLET H PROTEIN (200 KD NEUROFILAMENT PROTEIN) (NF-H).//2.60E-07//426aa//23%//P19246
C-NT2RP2004689//HYPOTHETICAL 192.5 KD PROTEIN C6G9.10C IN CHROMOSOME I.//5.60E-64//616aa//33%//Q92355
C-NT2RP2004710//Mus musculus formin binding protein 30 mRNA, complete cds.//1.50E-280//1464bp//85%//U40750
C-NT2RP2004732//NEUROFILAMENT TRIPLET M PROTEIN (160 KD NEUROFILAMENT PROTEIN) (NF-M).//7.30E-07//352aa//23%//P07197
C-NT2RP2004768//SERINE/THREONINE-PROTEIN KINASE NRK1 (EC 2.7.1.-) (N-RICH KINASE 1).//1.30E-26//190aa//41%//P38692
C-NT2RP2004791//PUTATIVE LEUCYL-TRNA SYNTHETASE, CYTOPLASMIC (EC 6.1.1.4) (LEUCINE-- TRNA LIGASE) (LEURS).//9.50E-73//153aa//59%//Q10490
C-NT2RP2004799//PROBABLE SUCCINYL-COA LIGASE [GDP-FORMING], BETA-CHAIN PRECURSOR (EC 6.2.1.4) (SUCCINYL-COA SYNTHETASE, BETA CHAIN) (SCS-BETA).//3.70E-135//414aa//62%//P53588
C-NT2RP2004816//H58 PROTEIN.//9.00E-173//327aa//98%//P40336
C-NT2RP2004920//TRANSCRIPTIONAL REGULATOR ATRX (X-LINKED NUCLEAR PROTEIN) (HETEROCHROMATIN PROTEIN 2) (HP1 ALPHA-INTERACTING PROTEIN) (HP1-BP38 PROTEIN).//4.20E-09//804aa//22%//Q61687
C-NT2RP2004933//Homo sapiens mRNA for ZIP-kinase, complete cds.//0//2103bp//99%//AB007144
C-NT2RP2004959//P54 PROTEIN PRECURSOR.//0.00000095//297aa//20%//P13692
C-NT2RP2004961//Rattus norvegicus KRAB/zinc finger suppressor protein 1 (KS1) mRNA, complete cds.//1.00E-228//1666bp//75%//U56732
C-NT2RP2004978//ACTIN-LIKE PROTEIN ARP8.//3.30E-47//353aa//30%//Q12386
C-NT2RP2005003//52 KD RO PROTEIN (SJOGREN SYNDROME TYPE A ANTIGEN (SS-A)) (RO(SS-A)).//1.80E-99//376aa//43%//P19474
C-NT2RP2005012//Homo sapiens mRNA for SEC63 protein.//0//1693bp//99%//AJ011779
C-NT2RP2005037//ANTI-SILENCING PROTEIN 1.//3.30E-47//155aa//59%//P32447
C-NT2RP2005038//DNA NUCLEOTIDYLEXOTRANSFERASE (EC 2.7.7.31) (TERMINAL ADDITION ENZYME) (TERMINAL DEOXYNUCLEOTIDYLTRANSFERASE) (TERMINAL TRANSFERASE).//4.00E-91//218aa//44%//Q92089
C-NT2RP2005116//PUTATIVE EUKARYOTIC TRANSLATION INITIATION FACTOR 3 ALPHA SUBUNIT (EIF-3 ALPHA).//2.00E-173//273aa//57%//P34466
C-NT2RP2005126//H.sapiens mRNA for RNA helicase (Myc-regulated dead box protein).//0//2388bp//98%//X98743
C-NT2RP2005139//2-5A-DEPENDENT RIBONUCLEASE (EC 3.1.26.-) (2-5A-DEPENDENT RNAASE) (RNASE L) (RIBONUCLEASE 4) (FRAGMENT).//0.000000022//139aa//35%//Q05921
C-NT2RP2005144//Homo sapiens tubby like protein 3 (TULP3) mRNA, complete cds.//0.00E-01//1437bp//98%//AF045583
C-NT2RP2005162//Homo sapiens aspartyl aminopeptidase mRNA, complete cds.//0//1615bp//99%//AF005050
C-NT2RP2005168//Homo sapiens mRNA for E1B-55kDa-associated protein.//0//2769bp//98%//AJ007509
C-NT2RP2005204//Homo sapiens SUMO-1-activating enzyme E1N subunit (SUA1) mRNA, complete cds.//0//1262bp//99%//AF090385
C-NT2RP2005239//Homo sapiens cysteine desulfurase (nifS) mRNA, complete cds.//0//2087bp//99%//AF097025
C-NT2RP2005276//Homo sapiens mRNA for Acyl-CoA synthetase 3, complete cds.//0/2122bp//99%//D89053
C-NT2RP2005288//Homo sapiens RCC1-like G exchanging factor RLG mRNA, complete cds.//0//2992bp//99%//AF060219
C-NT2RP2005315//Homo sapiens meningioma-expressed antigen 5 (MEA5) mRNA, partial cds.//1.90E-170//780bp//100%//AF036144
C-NT2RP2005325//Homo sapiens LIM-homeodomain protein HLHX2 (LHX2) mRNA, complete cds.//0//1643bp//99%//AF124735
C-NT2RP2005336//TRICHOHYALIN.//5.40E-10//545aa//22%//P37709
C-NT2RP2005344//PROBABLE CALCIUM-TRANSPORTING ATPASE 5 (EC 3.6.1.38).//2.10E-124//636aa//38%//P32660
C-NT2RP2005358//Homo sapiens methyl-CpG binding domain-containing protein MBD3 (MBD3) mRNA, complete cds.//0//2199bp//99%//AF072247
C-NT2RP2005360//Homo sapiens sentrin/SUMO-specific protease (SENP1) mRNA, complete cds.//1.30E-52//753bp//67%//AF149770
C-NT2RP2005393//AUTOANTIGEN NGP-1.//7.20E-39//224aa//35%//Q13823
C-NT2RP2005407//OXYSTEROL-BINDING PROTEIN.//5.30E-63//410aa//40%//P22059
C-NT2RP2005436//SPLICING FACTOR, ARGININE/SERINE-RICH 4 (PRE-MRNA SPLICING FACTOR SRP75).//1.20E-13//185aa//38%//Q08170
C-NT2RP2005441//Homo sapiens hypothalamus protein HT002 mRNA, complete cds.//4.10E-202//962bp//98%//AF113540
C-NT2RP2005457//Homo sapiens NADH-ubiquinone oxidoreductase subunit B14.5B homolog mRNA, complete cds.//1.20E-13 0//608bp//99%//AF070652
C-NT2RP2005465//MITOCHONDRIAL CARRIER PROTEIN RIM2.//3.00E-44//252aa//41%//P38127
C-NT2RP2005476//Human pl90-B (pl90-B) mRNA, complete cds.//3.40E-108//668bp//88%//U17032
C-NT2RP2005490//Mus musculus D3Mm3e (D3Mm3e) mRNA, complete cds.//1.80E-175//1102bp//83%//AF053628
C-NT2RP2005491//PARAMYOSIN (PMY) (ANTIGEN B).//0.00000015//279aa//26%//P35418
C-NT2RP2005496//ZINC FINGER PROTEIN 135.//2.90E-146//398aa//59%//P52742
C-NT2RP2005498//PROTEIN PHOSPHATASE PP2A, 55 KD REGULATORY SUBUNIT, ALPHA ISOFORM (PROTEIN PHOSPHATASE PP2A B SUBUNIT ALPHA ISOFORM) (ALPHA-PR55).//5.20E-81//166aa//88%//P36876
C-NT2RP2005509//Homo sapiens CGI-45 protein mRNA, complete cds.//0//1825bp//99%//AF151803
C-NT2RP2005520//Homo sapiens chromosome-associated protein-E (hCAP-E) mRNA, complete cds.//0//3994bp//99%//AF092563
C-NT2RP2005525//Mus musculus kanadaptin mRNA, complete cds.//2.40E-304//1687bp//85%//AF035526
C-NT2RP2005531//PROTEIN 4.1 (BAND 4.1) (P4.1).//5.50E-70//393aa//39%//P11171
C-NT2RP2005539//Homo sapiens mRNA for NS1-binding protein (NS1-BP).//0//1560bp//98%//AJ012449
C-NT2RP2005549//PUTATIVE LACTOYLGLUTATHIONE LYASE (EC 4.4.1.5) (METHYLGLYOXALASE) (ALDOKETOMUTASE) (GLYOXALASE I) (GLX I) (KETONE-ALDEHYDE MUTASE) (S-D-LACTOYLGLUTATHIONE METHYLGLYOXAL LYASE).//2.00E-20//181aa//36%//Q39366
C-NT2RP2005557//Homo sapiens clone 486790 diphosphoinositol polyphosphate phosphohydrolase mRNA, complete cds.//1.00E-46//576bp//70%//AF062529
C-NT2RP2005605//QUEUINE TRNA-RIBOSYLTRANSFERASE (EC 2.4.2.29) (TRNA-GUANINE TRANSGLYCOSYLASE) (GUANINE INSERTION ENZYME).//8.20E-23//164aa//28%//O32053
C-NT2RP2005620//Homo sapiens epsin 2a mRNA, complete cds.//8.9e-313//1455bp//98%//AF062085
C-NT2RP2005635//PROBABLE NH(3)-DEPENDENT NAD(⁺) SYNTHETASE (EC 6.3.5.1).//1.00E-11//128aa//36%//P47623
C-NT2RP2005654//CYSTEINE STRING PROTEIN (CCCS1).//1.20E-13//74aa//45%//P56101
C-NT2RP2005669//Homo sapiens death effector domain-containing testicular molecule mRNA, complete cds.//1.60E-248//1129bp//99%//AF043733
C-NT2RP2005675//Homo sapiens growth suppressor related (DOC-1R) mRNA, complete cds.//4.40E-200//908bp//99%//AF089814
C-NT2RP2005694//X-LINKED RETINITIS PIGMENTOSA GTPASE REGULATOR.//2.60E-10//175aa//27%//Q92834
C-NT2RP2005701//ZINC-FINGER PROTEIN RFP (RET FINGER PROTEIN).//3.00E-63//323aa//39%//Q62158
C-NT2RP2005712//Homo sapiens myosin X (MYO10) mRNA, partial cds.//0//2681 bp//99%//AF132022
C-NT2RP2005719//GPI-ANCHORED PROTEIN P137.//4.00E-14//99aa//43%//Q14444
C-NT2RP2005722//Homo sapiens ZK1 mRNA for Kruppel-type zinc finger protein, complete cds.//0//2545bp//99%//AB011414
C-NT2RP2005723//HNRNP ARGININE N-METHYLTRANSFERASE (EC 2.1.1.-) (ODP1 PROTEIN).//3.00E-09//169aa//28%//P38074
C-NT2RP2005752//Homo sapiens TNFR-related death receptor-6 (DR6) mRNA, complete cds.//0//1968bp//99%//AF068868
C-NT2RP2005753//Homo sapiens I-1 receptor candidate protein mRNA, complete cds.//0//1966bp//99%//AF082516
C-NT2RP2005763//EUKARYOTIC INITIATION FACTOR 4A (EIF-4A).//1.70E-61//374aa//38%//P47943
C-NT2RP2005767//G.gallus PB1 gene.//5.00E-163//1158bp//81%//X90849
C-NT2RP2005773//Homo sapiens pyrroline 5-carboxylate reductase isoform (P5CR2) mRNA, complete cds.//2.70E-180//656bp//99%//AF151351
C-NT2RP2005775//NEUROLYSIN PRECURSOR (EC 3.4.24.16) (NEUROTENSIN ENDOPEPTIDASE) (MITOCHONDRIAL OLIGOPEPTIDASE M) (MICROSOMAL ENDOPEPTIDASE) (MEP) (SOLUBLE ANGIOTENSIN-BINDING PROTEIN) (SABP).//2.10E-213//249aa//85%//Q02038
C-NT2RP2005776//POLY(A) POLYMERASE TYPE 2 (EC 2.7.7.19) (PAP) (POLYNUCLEOTIDE ADENYLYLTRANSFERASE).//4.40E-55//358aa//42%//P51005
C-NT2RP2005784//Homo sapiens ubiquitin-conjugating enzyme variant Kua (UBE2V) mRNA, complete cds.//0//2191bp//92%//AF155120
C-NT2RP2005812//HYPOTHETICAL 39.3 KD PROTEIN IN GCN4-WBP1 INTERGENIC REGION.//2.30E-39//318aa//31%//P40004
C-NT2RP2005835//SHP1 PROTEIN.//1.80E-28//208aa//32%//P34223
C-NT2RP2005841//Homo sapiens mRNA for ALEX3, complete cds.//3.50E-52//1091bp//59%//AB039669
C-NT2RP2005933//NUCLEOPORIN NUP57 (NUCLEAR PORE PROTEIN NUP57).//5.00E-11//155aa//34%//P48837
C-NT2RP2005942//POLY(A) POLYMERASE (EC 2.7.7.19) (PAP) (POLYNUCLEOTIDE ADENYLYLTRANSFERASE).//1.50E-67//388aa//44%//P25500
C-NT2RP2006043//SPLICING FACTOR, ARGININE/SERINE-RICH 4 (PRE-MRNA SPLICING FACTOR SRP75).//1.50E-13//185aa//38%//Q08170
C-NT2RP2006071//Homo sapiens adaptor protein APPL mRNA, complete cds.//5.80E-120//1257bp//64%//AF169797
C-NT2RP2006219//H.sapiens mRNA for DGCR6 protein.//1.10E-214//1026bp//97%//X96484
C-NT2RP2006238//Rattus norvegicus CTD-binding SR-like protein rA8 mRNA, complete cds.//0//1669bp//88%//U49055
C-NT2RP2006275//MICROTUBULE-ASSOCIATED PROTEIN 1B [CONTAINS: LIGHT CHAIN LC1].//2.00E-59//388aa//32%//P46821
C-NT2RP2006312//Homo sapiens BAF57 (BAF57) gene, complete cds.//2.80E-274//1236bp//99%//AF035262
C-NT2RP2006436//ANTERIOR-RESTRICTED HOMEOBOX PROTEIN (RATHKE POUCH HOMEO BOX).//3.40E-07//50aa//50%//Q61658
C-NT2RP2006456//Homo sapiens leucine-rich glioma-inactivated protein precursor (LGI1) mRNA, complete cds.//1.30E-37//484bp//65%//AF055636
C-NT2RP2006464//Homo sapiens mRNA for AND-1 protein.//0//2181bp//99%//AJ006266
C-NT2RP2006534//5'-AMP-ACTIVATED PROTEIN KINASE, CATALYTIC ALPHA-1 CHAIN (EC 2.7.1.-) (AMPK ALPHA-1 CHAIN) (FRAGMENT).//3.20E-11//32aa//96%//Q13131
C-NT2RP2006565//Homo sapiens secretory carrier-associated membrane protein (SCAMP) mRNA, complete cds.//3.10E-272//1220bp//95%//AF038966
C-NT2RP2006571//CYTOCHROME P450 2G1 (EC 1.14.14.1) (CYPIIG1) (P450-NMB) (OLFACTIVE).//4.20E-134//486aa//50%//P24461
C-NT2RP2006573//2',3'-CYCLIC NUCLEOTIDE 3'-PHOSPHODIESTERASE (EC 3.1.4.37) (CNP).//0.0000055//169aa//25%//P09543
C-NT2RP2006598//Homo sapiens retinoid x receptor interacting protein mRNA, complete cds.//3.10E-295//1193bp//99%//AF113538
C-NT2RP3000031//HISTONE DEACETYLASE HDA1.//1.10E-71//350aa//42%//P53973
C-NT2RP3000046//MITOCHONDRIAL GTPASE MSS1 PRECURSOR.//4.60E-78//421aa//37%//P32559
C-NT2RP3000047//NPL4 PROTEIN.//1.10E-85//526aa//36%//P33755
C-NT2RP3000050//ZINC FINGER PROTEIN 91 (ZINC FINGER PROTEIN HTF10) (HPF7).//2.20E-150//490aa//53%//Q05481
C-NT2RP3000068//SON OF SEVENLESS PROTEIN HOMOLOG 1 (SOS-1) (MSOS-1 ).//2.20E-06//165aa//27%//Q62245
C-NT2RP3000085//ACETYL-/PROPIONYL-COENZYME A CARBOXYLASE ALPHA CHAIN [CONTAINS: BIOTIN CARBOXYLASE (EC 6.3.4.14); BIOTIN CARBOXYL CARRIER PROTEIN (BCCP)].//1.90E-123//436aa//50%//P46401
C-NT2RP3000109//P54 PROTEIN PRECURSOR.//0.0000065//358aa//22%//P13692
C-NT2RP3000207//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//2.90E-11//721aa//23%//P08640
C-NT2RP3000233//RING CANAL PROTEIN (KELCH PROTEIN).//9.30E-84//453aa//42%//Q04652
C-NT2RP3000252//Homo sapiens GTP-binding protein NGB mRNA, complete cds.//0//2388bp//99%//AF120334
C-NT2RP3000299//Rattus norvegicus mRNA for Crk-associated substrate, pi 30, complete cds.//0//2730bp//82%//D29766
C-NT2RP3000320//Homo sapiens partial mRNA for putative p621 protein which interacts with transcription factor Sp1.//0//1544bp//100%//AJ242978
C-NT2RP3000333//Rattus norvegicus db83 mRNA, complete cds.//2.90E-191//1094bp//85%//AB006135
C-NT2RP3000341//Homo sapiens mitochondrial inner membrane preprotein translocase Tim17a mRNA, nuclear gene encoding mitochondrial protein, complete cds.//1.50E-246//1124bp//99%//AF106622
C-NT2RP3000350//Homo sapiens GTP-binding protein NGB mRNA, complete cds.//0//2392bp//99%//AF120334
C-NT2RP3000359//GTP:AMP PHOSPHOTRANSFERASE MITOCHONDRIAL (EC 2.7.4.10) (AK3).//2.00E-111//226aa//92%//P08760
C-NT2RP3000361//Homo sapiens mRNA, complete cds, similar to yeast pre-mRNA splicing factors, Prp1/Zer1 and Prp6.//0//2072bp//98%//AB019219
C-NT2RP3000366//RAS-RELATED PROTEIN RAB-18.//2.10E-107//206aa//99%//P35293
C-NT2RP3000393//Rattus norvegicus DNA-binding protein PREB (Preb) mRNA, complete cds.//5.80E-266//1373bp//86%//AF061817
C-NT2RP3000397//PUTATIVE PRE-MRNA SPLICING FACTOR RNA HELICASE (DEAH BOX PROTEIN 13)//1.70E-139//679aa//41%//O43143
C-NT2RP3000403//Homo sapiens formin binding protein 21 mRNA, complete cds.//0//2364bp//99%//AF071185
C-NT2RP3000439//HYPOTHETICAL 46.4 KD PROTEIN IN FFH-GRPE INTERGENIC REGION.//2.90E-15//319aa//26%//P37908
C-NT2RP3000441//Homo sapiens squamous cell carcinoma antigen recognized by T cell (SART-2) mRNA, complete cds.//3.40E-42//645bp//67%//AF098066
C-NT2RP3000512//Human HOX2G mRNA from the Hox2 locus.//0//1934bp//99%//X16667
C-NT2RP3000527//ZINC FINGER PROTEIN 43 (ZINC PROTEIN HTF6).//4.80E-28//536aa//27%//P28160
C-NT2RP3000531//POLIOVIRUS RECEPTOR PRECURSOR (CD155 ANTIGEN).//1.90E-12//192aa//30%//P15151
C-NT2RP3000562//Homo sapiens putative RNA-binding protein Q99 mRNA, complete cds.//0//2165bp//99%//AF093097
C-NT2RP3000578//HES1 PROTEIN.//1.30E-22//229aa//27%//P35843
C-NT2RP3000590//UVS-2 PROTEIN.//1.30E-22//458aa//24%//P33288
C-NT2RP3000596//TRICHOHYALIN.//2.50E-17//304aa//28%//Q07283
C-NT2RP3000603//NEUROGENIC DIFFERENTIATION FACTOR 1.//3.70E-11//90aa//42%//Q13562
C-NT2RP3000605//Mus musculus mRNA for wizL, complete cds.//0//2232bp//82%//AB012265
C-NT2RP3000624//Rattus norvegicus mRNA for SECIS binding protein 2 (sbp2 gene).//5.80E-234//1562bp//81%//AJ251245
C-NT2RP3000632//ZINC FINGER PROTEIN 84 (ZINC FINGER PROTEIN HPF2).//3.00E-140//499aa//46%//P51523
C-NT2RP3000739//ATROPHIN-1 (DENTATORUBRAL-PALLIDOLUYSIAN ATROPHY PROTEIN).//1.40E-24//155aa//37%//Q10149
C-NT2RP3000742//1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III) (FRAGMENT).//4.10E-165//371aa//49%//P10895
C-NT2RP3000753//NEUROFILAMENT TRIPLET H PROTEIN (200 KD NEUROFILAMENT PROTEIN) (NF-H).//2.00E-10//565aa//24%//P12036
C-NT2RP3000759//ADP-RIBOSYLATION FACTOR.//7.00E-28//176aa//34%//Q94650
C-NT2RP3000825//NEUROGENIC LOCUS NOTCH 3 PROTEIN.//2.50E-36//417aa//31%//Q61982
C-NT2RP3000826//Homo sapiens mRNA for seven transmembrane protein TM7SF3, complete cds.//0//2522bp//99%//AB032470
C-NT2RP3000845//PUTATIVE SERINE/THREONINE-PROTEIN KINASE P78 (EC 2.7.1.-).//8.30E-108//331aa//50%//P27448
C-NT2RP3000868//Human ovarian cancer downregulated myosin heavy chain homolog (Doc1) mRNA, complete cds.//6.90E-69//1611bp//61%//U53445
C-NT2RP3000869//Drosophila melanogaster AAA family protein Bor (bor) mRNA, complete cds.//2.60E-138//1673bp//67%//AF227209
C-NT2RP3000875//MEVALONATE KINASE (EC 2.7.1.36) (MK).//7.70E-87//175aa//98%//Q03426
C-NT2RP3000917//DHP1 PROTEIN.//1.00E-193//428aa//55%//P40848
C-NT2RP3000919//Rattus norvegicus golgi peripheral membrane protein p65 (GRASP65) mRNA, complete cds.//2.70E-185//585bp//88%//AF015264
C-NT2RP3000968//40S RIBOSOMAL PROTEIN S15A.//1.90E-46//73aa//98%//P39027
C-NT2RP3000994//MATERNAL EFFECT PROTEIN STAUFEN.//0.00000006//78aa//48%//P25159
C-NT2RP3001055//Drosophila melanogaster separation anxiety protein (san) mRNA, complete cds.//3.80E-38//462bp//70%//AF225902
C-NT2RP3001057//ZINC FINGER PROTEIN 91 (ZINC FINGER PROTEIN HTF10) (HPF7).//9.00E-201//584aa//54%//Q05481
C-NT2RP3001081//Homo sapiens RCC1-like G exchanging factor RLG mRNA, complete cds.//7.10E-47//537bp//74%//AF060219
C-NT2RP3001096//Rattus norvegicus leprecan (lepre1) mRNA, complete cds.//1.70E-94//787bp//66%//AF087433
C-NT2RP3001107//PEREGRIN (BR140 PROTEIN).//3.00E-44//260aa//40%//P55201
C-NT2RP3001111//Homo sapiens TRF-proximal protein mRNA, complete cds.//1.50E-149//731bp//97%//AF097725
C-NT2RP3001113//INTRACELLULAR PROTEIN TRANSPORT PROTEIN USO1.//2.90E-11//631aa//23%//P25386
C-NT2RP3001120//ZINC FINGER, PROTEIN 136.//7.80E-170//512aa//58%//P52737
C-NT2RP3001140//F-SPONDIN PRECURSOR.//9.90E-238//419aa//96%//P35446
C-NT2RP3001150//TRANSCRIPTION TERMINATION FACTOR RHO.//0.00000031//207aa//29%//P52154
C-NT2RP3001155//Homo sapiens mRNA for AND-1 protein.//0//2732bp//99%//AJ006266
C-NT2RP3001176//HYPOTHETICAL 65.3 KD PROTEIN IN MAD1-SCY1 INTERGENIC REGION.//1.70E-10//196aa//27%//P53154
C-NT2RP3001216//CYLICIN I (MULTIPLE-BAND POLYPEPTIDE I) (FRAGMENT).//0.0000023//137aa//33%//P35663
C-NT2RP3001221//GAMMA-BUTYROBETAINE,2-OXOGLUTARATE DIOXYGENASE (EC 1.14.11.1) (GAMMA-BUTYROBETAINE HYDROXYLASE).//1.90E-31//353aa//30%//P80193
C-NT2RP3001239//MICROTUBULE-ASSOCIATED PROTEIN 1B (MAP1.2) (MAP1(X)) [CONTAINS: LIGHT CHAIN LC1].//1.20E-166//395aa//51%//P14873
C-NT2RP3001253//NUF1 PROTEIN (SPINDLE POLY BODY SPACER PROTEIN SPC110).//1.70E-10//540aa//23%//P32380
C-NT2RP3001268//Homo sapiens zinc finger protein ZNF228 (ZNF228) mRNA, complete cds.//0//3606bp//99%//AF198358
C-NT2RP3001272//Mus musculus mRNA for macrophage actin-associated-tyrosine-phosphorylated protein.//1.30E-99//669bp//83 %//Y18101
C-NT2RP3001307//Gallus gallus RPE65 mRNA, complete cds.//4.20E-29//530bp//63%//AB017594
C-NT2RP3001338//ZINC FINGER PROTEIN 81 (FRAGMENT).//2.40E-16//175aa//28%//P51508
C-NT2RP3001355//TRICARBOXYLATE TRANSPORT PROTEIN PRECURSOR (CITRATE TRANSPORT PROTEIN) (CTP) (TRICARBOXYLATE CARRIER PROTEIN).//3.60E-25//129aa//34%//P32089
C-NT2RP3001383//Mus musculus ARL-6 interacting protein-6 (Aip-6) mRNA, partial cds.//3.40E-40//355bp//79%//AF133913
C-NT2RP3001384//Homo sapiens mRNA for LA95 protein.//0//1214bp//99%//AJ243467
C-NT2RP3001398//TRANSCRIPTIONAL REPRESSOR CTCF.//1.30E-61//374aa//36%//P49711
C-NT2RP3001399//SSU72 PROTEIN.//1.30E-16//84aa//52%//P53538
C-NT2RP3001407//SCY1 PROTEIN.//0.00000033//143aa//25%//P53009
C-NT2RP3001426//DNAJ PROTEIN (FRAGMENT).//1.00E-16//77aa//46%//O33529
C-NT2RP3001427//WERNER SYNDROME HEUCASE HOMOLOG.//2.70E-10//159aa//33%//O09053
C-NT2RP3001428//NUCLEOPROTEIN TPR.//1.40E-128//152aa//99%//P 12270
C-NT2RP3001453//ANTIGEN PEPTIDE TRANSPORTER 2 (APT2) (HISTOCOMPATIBILITY ANTIGEN MODIFIER 2).//3.20E-90//157aa//59%//P36371
C-NT2RP3001457//Drosophila melanogaster Melted (melt) mRNA, partial cds.//4.60E-20//792bp//59%//AF205831
C-NT2RP3001472//NONHISTONE CHROMOSOMAL PROTEIN 6A.//9.10E-13//87aa//43%//P11632
C-NT2RP3001495//Human oxidoreductase (HHCMA56) mRNA, complete cds.//0//1475bp//99%//U13395
C-NT2RP3001497//Homo sapiens multiple membrane spanning receptor TRC8 (TRC8) mRNA, complete cds.//0//2295bp//99%//AF064801
C-NT2RP3001527//Human Sp140 protein (Sp140) mRNA, complete cds.//4.30E-290//793bp//93%//U63420
C-NT2RP3001529//SPO0B-ASSOCIATED GTP-BINDING PROTEIN.//1.00E-61//345aa//42%//P20964
C-NT2RP3001538//HYPOTHETICAL 39.0 KD PROTEIN T28D9.3 IN CHROMOSOME II.//9.10E-10//158aa//31%//Q10022
C-NT2RP3001554//MICROTUBULE-ASSOCIATED PROTEIN 1B [CONTAINS: LIGHT CHAIN LC1].//1.40E-76//388aa//32%//P46821
C-NT2RP3001580//Mus musculus strain C57BL/J germ cell-less protein (Gcl) mRNA, complete cds.//0//1730bp//85%//AF163665
C-NT2RP3001587//Human anthracycline-associated resistance ARX mRNA, complete cds.//0//2617bp//99%//U35832
C-NT2RP3001642//HYPOTHETICAL PROTEIN KIAA0210.//6.80E-18//91aa//38%//Q92609
C-NT2RP3001646//WD-40 REPEAT PROTEIN MSI2.//8.80E-09//132aa//31%//O22468
C-NT2RP3001671//Homo sapiens mRNA for NS1-binding protein (NS1-BP).//0//1557bp//98%//AJ012449
C-NT2RP3001672//Homo sapiens Sex comb on midleg homolog 1 isoform 2 (SCMH1) mRNA, complete cds.//0//2836bp//99%//AF149046
C-NT2RP3001679//Homo sapiens rec mRNA, complete cds.//0//2495bp//99%//AB023584
C-NT2RP3001688//Homo sapiens DNA binding protein p96PIF mRNA, complete cds.//0//1869bp//99%//AF173868
C-NT2RP3001690//INTRACELLULAR PROTEIN TRANSPORT PROTEIN USO1.//0.00000024//481aa//21%//P25386
C-NT2RP3001708//TWISTED GASTRULATION PROTEIN PRECURSOR.//3.40E-33//161aa//32%//P54356
C-NT2RP3001712//Homo sapiens HP1-BP74 protein mRNA, complete cds.//0//1788bp//99%//AF113534
C-NT2RP3001723//Homo sapiens cell recognition molecule Caspr2 (CASPR2) mRNA, complete cds.//1.40E-58//1138bp//63%//AF193613
C-NT2RP3001724//Homo sapiens chromodomain-helicase-DNA-binding protein mRNA, complete cds.//1.10E-240//902bp//99%//AF054177
C-NT2RP3001727//Rattus norvegicus implantation-associated protein (IAG2) mRNA, partial cds.//6.90E-132//774bp//88%//AF008554
C-NT2RP3001730//SEPTIN 2 HOMOLOG (FRAGMENT).//7.10E-132//294aa//84%//Q14141
C-NT2RP3001739//HYPOTHETICAL 72.5 KD PROTEIN C2F7.10 IN CHROMOSOME I.//1.40E-15//190aa//32%//Q09701
C-NT2RP3001792//HETEROGENEOUS NUCLEAR RIBONUCLEOPROTEIN M (HNRNP M).//1.80E-117//462aa//55%//P52272
C-NT2RP3001799//MYOSIN HEAVY CHAIN, STRIATED MUSCLE.//1.60E-11//348aa//27%//P24733
C-NT2RP3001819//RING CANAL PROTEIN (KELCH PROTEIN).//7.40E-18//249aa//30%//Q04652
C-NT2RP3001854//Homo sapiens novel retinal pigment epithelial cell protein (NORPEG) mRNA, complete cds.//0//2742bp//99%//AF155135
C-NT2RP3001855//HOMEOBOX PROTEIN PKNOX1 (HOMEOBOX PROTEIN PREP-1).//8.10E-125//302aa//60%//P55347
C-NT2RP3001857//VEGETATIBLE INCOMPATIBILITY PROTEIN HET-E-1.//1.20E-14//242aa//24%//Q00808
C-NT2RP3001898//Homo sapiens mRNA for UDP-N-acetylglucosamine: alpha-1,3-D-mannoside beta-1,4-N-acetylglucosaminyltransferase IV, complete cds.//0//1587bp//100%//AB000624
C-NT2RP3001931//Rattus norvegicus clone C48 CDK5 activator-binding protein mRNA, complete cds.//4.30E-91//656bp//81%//AF177478
C-NT2RP3001938//SPORULATION-SPECIFIC PROTEIN 1 (EC 2.7.1.-).//1.30E-22//227aa//33%//P08458
C-NT2RP3001944//HYPOTHETICAL 47.6 KD PROTEIN C16C10.5 IN CHROMOSOME III.//3.10E-92//314aa//51 %//Q09251
C-NT2RP3001969//TRICHOHYALIN.//2.70E-11//442aa//23%//P37709
C-NT2RP3002004//H.sapiens mRNA for FAST kinase.//1.50E-192//475bp//94%//X86779
C-NT2RP3002007//SAP1 PROTEIN.//1.1 OE-68//474aa//32%//P39955
C-NT2RP3002014//HYPOTHETICAL 32.0 KD PROTEIN C09F5.2 IN CHROMOSOME III.//5.30E-25//139aa//48%//Q09232
C-NT2RP3002045//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT.//1.00E-299//397aa//94%//P18484
C-NT2RP3002056//RETINOBLASTOMA BINDING PROTEIN 1 (RBBP-1).//2.00E-48//475aa//35%//P29374
C-NT2RP3002062//Homo sapiens BAG-family molecular chaperone regulator-5 mRNA, complete cds.//0//3764bp//99%//AF095195
C-NT2RP3002081//Xenopus laevis chromosome condensation protein XCAP-G mRNA, complete cds.//4.10E-233//1896bp//69%//AF111423
C-NT2RP3002108//DEC1 PROTEIN (MDM20 PROTEIN).//7.90E-09//181aa//22%//Q12387
C-NT2RP3002151//G1 TO S PHASE TRANSITION PROTEIN 1 HOMOLOG (GTP-BINDING PROTEIN GST1-HS).//2.80E-253//474aa//93%//P15170
C-NT2RP3002165//TRANSCRIPTIONAL REGULATOR PROTEIN HCNGP.//1.90E-151//223aa//91%//Q02614
C-NT2RP3002273//SCD6 PROTEIN.//1.30E-09//295aa//28%//P45978
C-NT2RP3002303//PROBABLE UNDECAPRENYL PYROPHOSPHATE SYNTHETASE (EC 2.5.1.31) (UPP SYNTHETASE) (DI-TRANS-POLY-CIS-DECAPRENYLCISTRANSFERASE).//8.60E-49//243aa//43%//Q58767
C-NT2RP3002330//Homo sapiens eRFS mRNA, complete cds.//0//2443bp//99%//U87791
C-NT2RP3002351//Human mRNA for NAD-dependent methylene tetrahydrofolate dehydrogenase cyclohydrolase (EC 1.5.1.15).//4.20E-70//590bp//76%//X16396
C-NT2RP3002399//DNA REPLICATION LICENSING FACTOR MCM4 (CDC21 HOMOLOG) (P1-CDC21).//8.60E-79//416aa//34%//P33991
C-NT2RP3002501//THREONINE DEHYDRATASE CATABOLIC (EC 4.2.1.16) (THREONINE DEAMINASE).//3.70E-43//318aa//37%//P05792
C-NT2RP3002529//VACUOLAR PROTEIN SORTING-ASSOCIATED PROTEIN VPS45.//8.90E-95//542aa//38%//P38932
C-NT2RP3002549//HYPOTHETICAL 26.6 KD PROTEIN T19C3.4 IN CHROMOSOME III.//5.80E-40//161aa//52%//Q10010
C-NT2RP3002602//PROBABLE PROTEIN DISULFIDE ISOMERASE ER-60 PRECURSOR (EC 5.3.4.1) (ERP60) (58 KD MICROSOMAL PROTEIN) (P58) (HIP-70) (Q-2).//2.90E-19//173aa//28%//P11598
C-NT2RP3002628//PROBABLE PROTEIN DISULFIDE ISOMERASE P5 PRECURSOR (EC 5.3.4.1).//2.50E-26//90aa//42%//P38660
C-NT2RP3002631//Homo sapiens Ran binding protein 11 mRNA, complete cds.//0//1703bp//99%//AF111109
C-NT2RP3002650//Mus musculus growth suppressor 1L (Gros1) mRNA, complete cds.//0//2109bp//87%//AF165163
C-NT2RP3002663//Homo sapiens putative glycolipid transfer protein mRNA, complete cds.//8.10E-263//1243bp//97%//AF103731
C-NT2RP3002671//ELONGATION FACTOR 2 (EF-2).//2.50E-73//179aa//36%//P13060
C-NT2RP3002682//Homo sapiens CGI-145 protein mRNA, complete cds.//0//1596bp//98%//AF151903
C-NT2RP3002688//Mouse mRNA for kinesin-like protein (Kif1b), complete cds.//1.10E-93//1205bp//69%//D17577
C-NT2RP3002770//MYELOID DIFFERENTIATION PRIMARY RESPONSE PROTEIN MYD116.//1.00E-07//70aa//41%//P17564
C-NT2RP3002785//LETHAL(2)DENTICLELESS PROTEIN (DTL83 PROTEIN).//2.50E-55//187aa//39%//Q24371
C-NT2RP3002810//HISTIDINE-RICH PROTEIN KE4.//2.20E-10//260aa//26%//Q31125
C-NT2RP3002818//INSERTION ELEMENT IS2A HYPOTHETICAL 48.2 KD PROTEIN.//5.70E-226//303aa//97%//P51026
C-NT2RP3002869//Mus musculus semaphorin VIa mRNA, complete cds.//2.50E-232//1282bp//85%//AF030430
C-NT2RP3002876//Drosophila melanogaster eyelid (eld) mRNA, complete cds.//1.30E-29//805bp//61%//AF053091
C-NT2RP3002909//P53-BINDING PROTEIN 2 (53BP2) (BCL2-BINDING PROTEIN) (BBP).//1.50E-125//512aa//47%//Q13625
C-NT2RP3002948//RING CANAL PROTEIN (KELCH PROTEIN).//2.00E-111//551aa//42%//Q04652
C-NT2RP3002953//Homo sapiens protocadherin beta 5 (PCDH-beta5) mRNA, complete cds.//0//2388bp//99%//AF152498
C-NT2RP3002969//Homo sapiens mRNA for Acyl-CoA synthetase 3, complete cds.//0//2722bp//99%//D89053
C-NT2RP3002972//Halocynthia roretzi mRNA for HrPET-1, complete cds.//3.90E-52//899bp//64%//AB029333
C-NT2RP3002988//Homo sapiens IkB kinase-b (IKK-beta) mRNA, complete cds.//1.80E-292//1325bp//99%//AF080158
C-NT2RP3003032//Homo sapiens okadaic acid-inducible and cAMP-regulated phosphoprotein 19 (ARPP-19) mRNA, complete cds.//0//2656bp//99%//AF084555
C-NT2RP3003059//Rattus norvegicus potassium channel regulator 1 mRNA, complete cds.//3.80E-152//1007bp//82%//U78090
C-NT2RP3003061//ANKYRIN.//1.40E-20//200aa//37%//Q02357
C-NT2RP3003071//NEUROGENIC PROTEIN BIG BRAIN.//1.10E-05//258aa//24%//P23645
C-NT2RP3003078//Rattus norvegicus mRNA for ischemia related factor NYW-1, complete cds.//2.60E-112//633bp//88%//AB027149
C-NT2RP3003101//Mouse mRNA for tetracycline transporter-like protein, complete cds.//3.60E-83//807bp//72%//D88315
C-NT2RP3003133//Homo sapiens ZK1 mRNA for Kruppel-type zinc finger protein, complete cds.//0//1998bp//91%//AB011414
C-NT2RP3003138//Homo sapiens kinesin superfamily motor KIF4 mRNA, complete cds.//0//2159bp//98%//AF071592
C-NT2RP3003145//Mus musculus metallocarboxypeptidase CPX-1 mRNA, complete cds.//0//2251bp//81%//AF077738
C-NT2RP3003185//TROPOMYOSIN1, FUSION PROTEIN 33.//2.80E-06//402aa//23%//P49455
C-NT2RP3003193//ZINC FINGER PROTEIN 135.//7.30E-98//269aa//62%//P52742
C-NT2RP3003197//HYPOTHETICAL 33.8 KD PROTEIN C5H10.01 IN CHROMOSOME I.//5.70E-09//169aa//31%//Q09674
C-NT2RP3003203//Rattus norvegicus golgi stacking protein homolog GRASP55 mRNA, complete cds.//2.00E-210//1851 bp//76%//AF110267
C-NT2RP3003212//Rattus norvegicus lamina associated polypeptide 1C (LAP1C) mRNA, complete cds.//4.30E-187//1750bp//75%//U20286
C-NT2RP3003230//Homo sapiens mRNA for hCRNN4, complete cds.//0//2350bp//99%//AB030656
C-NT2RP3003242//Homo sapiens stanniocalcin-related protein mRNA, complete cds.//0//2366bp//99%//AF098462
C-NT2RP3003251//52 KD RO PROTEIN (SJOGREN SYNDROME TYPE A ANTIGEN (SS-A)) (RO(SS-A)).//4.20E-86//366aa//48%//P19474
C-NT2RP3003282//Homo sapiens dynamin (DNM) mRNA, complete cds.//0//2596bp//98%//L36983
C-NT2RP3003290//Mus musculus mRNA for Ndr1 related protein Ndr3, complete cds.//1.5e-310//1468bp//82%//AB033922
C-NT2RP3003301//MITOCHONDRIAL LON PROTEASE HOMOLOG 1 PRECURSOR (EC 3.4.21.-).//1.10E-170//585aa//54%//O64948
C-NT2RP3003313//Homo sapiens thyroid hormone receptor-associated protein complex component TRAP80 mRNA, complete cds.//0//2476bp//99%//AF117657
C-NT2RP3003327//52 KD RO PROTEIN (SJOGREN SYNDROME TYPE A ANTIGEN (SS-A)) (RO(SS-A)) (RO52).//1.30E-35//178aa//44%//Q62191
C-NT2RP3003353//HYPOTHETICAL 26.2 KD PROTEIN IN GDI1-COX15 INTERGENIC REGION.//2.80E-07//161aa//28%//P40084
C-NT2RP3003385//Mus musculus SKD3 mRNA, complete cds.//0//2133bp//85%//U09874
C-NT2RP3003409//Human DHHC-domain-containing cysteine-rich protein mRNA, complete cds.//9.20E-45//782bp//65%//U90653
C-NT2RP3003411//Mus musculus COP9 complex subunit 7b (COPS7b) mRNA, complete cds.//6.30E-270//743bp//90%//AF071317
C-NT2RP3003490//Homo sapiens mRNA for putative phospholipase, complete cds.//4.50E-81//649bp//67%//AB019435
C-NT2RP3003491//Drosophila melanogaster Pelle associated protein Pellino (Pli) mRNA, complete cds.//5.60E-36//842bp//62%//AF091624
C-NT2RP3003500//SCY1 PROTEIN.//9.20E-27//601aa//23%//P53009
C-NT2RP3003555//HYPOTHETICAL 32.6 KD PROTEIN IN MET30-PIG2 INTERGENIC REGION.//4.50E-30//191aa//40%//P40529
C-NT2RP3003589//Homo sapiens ras-related GTP-binding protein mRNA, complete cds.//0//3131bp//94%//AF106681
C-NT2RP3003659//HES1 PROTEIN.//5.90E-22//229aa//27%//P35843
C-NT2RP3003665//Homo sapiens mRNA for beta-ureidopropionase, complete cds.//0//1690bp//99%//AB013885
C-NT2RP3003672//T-CELL SURFACE GLYCOPROTEIN E2 PRECURSOR (E2 ANTIGEN) (CD99) (MIC2 PROTEIN) (12E7).//2.20E-13//146aa//42%//P14209
C-NT2RP3003701//F-SPONDIN PRECURSOR.//1.80E-17//324aa//26%//P35446
C-NT2RP3003716//SLIT PROTEIN PRECURSOR.//6.60E-10//150aa//34%//P24014
C-NT2RP3003726//Homo sapiens spermatogenesis associated PD1 mRNA, complete cds.//0//2568bp//99%//U28164
C-NT2RP3003799//Rattus norvegicus Srg1 (Sytr1) mRNA, complete cds.//9.00E-238//1529bp//84%//U71294
C-NT2RP3003800//Rattus norvegicus tyrosine protein kinase pp60-c-src mRNA, complete cds.//1.90E-163//924bp//89%//AF130457
C-NT2RP3003809//SAV PROTEIN.//1.10E-131//576aa//41%//Q07590
C-NT2RP3003825//PHOSPHATIDYLCHOLINE TRANSFER PROTEIN (PC-TP).//9.60E-19//174aa//31%//P02720
C-NT2RP3003831//Homo sapiens ENDOGL-1 (alias ENGL-a) mRNA for endonuclease G-like protein-1, complete cds.//2.2e-316//1436bp//99%//AB020523
C-NT2RP3003846//Homo sapiens mRNA for putative phospholipase, complete cds.//4.80E-277//1255bp//99%//AB019435
C-NT2RP3003876//Rattus norvegicus Rabin3 mRNA, complete cds.//4.50E-147//874bp//87%//U19181
C-NT2RP3003914//UDP-GLUCOSE:GLYCOPROTEIN GLUCOSYLTRANSFERASE PRECURSOR (EC 2.4.1.-) (DUGT).//2.20E-20//76aa//64%//Q09332
C-NT2RP3003918//Homo sapiens VAMP-associated protein B (VAP-B) mRNA, complete cds.//0//2191bp//99%//AF086628
C-NT2RP3004013//M.musculus Spnr mRNA for RNA binding protein.//6.50E-240//1215bp//94%//X84692
C-NT2RP3004016//TRANSCRIPTION INTERMEDIARY FACTOR 1-BETA (NUCLEAR COREPRESSOR KAP-1) (KRAB-ASSOCIATED PROTEIN 1).//1.50E-17//226aa//26%//Q13263
C-NT2RP3004078//H.sapiens HRFX2 mRNA.//0//1806bp//99%//X76091
C-NT2RP3004125//Mus musculus zinc finger protein splice variant FIZ1-B (Fiz1) mRNA, complete cds.//4.60E-229//1560bp//78%//AF126747
C-NT2RP3004148//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//7.90E-05//271aa//22%//P08640
C-NT2RP3004155//Homo sapiens COQ7 protein mRNA, complete cds.//1.10E-179//823bp//100%//AF098948
C-NT2RP3004189//VEGETATIBLE INCOMPATIBILITY PROTEIN HET-E-1.//1.30E-14//242aa//24%//Q00808
C-NT2RP3004206//CROOKED NECK PROTEIN.//1.40E-220//567aa//67%//P17886
C-NT2RP3004207//Homo sapiens mRNA for type I transmembrane receptor (psk-1 gene).//0//2445bp//100%//AJ245820
C-NT2RP3004209//Homo sapiens ubiquitin processing protease (Ubp-M) mRNA, complete cds.//0//2320bp//99%//AF126736
C-NT2RP3004242//PUTATIVE RHO/RAC GUANINE NUCLEOTIDE EXCHANGE FACTOR (RHO/RAC GEF) (FACIOGENITAL DYSPLASIA PROTEIN HOMOLOG).//4.70E-13//118aa//33%//P52734
C-NT2RP3004258//Homo sapiens ZIS1 mRNA, complete cds.//0//1861bp//99%//AF065391
C-NT2RP3004262//Homo sapiens heat shock protein hsp40-3 mRNA, complete cds.//2.40E-248//1126bp//100%//AF088982
C-NT2RP3004282//Homo sapiens torsinA (DYT1) mRNA, complete cds.//5.10E-24//597bp//61%//AF007871
C-NT2RP3004348//R.norvegicus mRNA for cytosolic resiniferatoxin-binding protein.//1.10E-185//1130bp//82%//X67877
C-NT2RP3004378//Drosophila melanogaster separation anxiety protein (san) mRNA, complete cds.//3.90E-38//462bp//70%//AF225902
C-NT2RP3004424//Homo sapiens mRNA for stromal antigen 3 (STAG3 gene).//1.00E-66//364bp//93%//AJ007798
C-NT2RP3004428//CHROMODOMAIN HELICASE-DNA-BINDING PROTEIN 4 (CHD-4) (MI-2 AUTOANTIGEN 218 KD PROTEIN) (MI2-BETA).//5.20E-09//212aa//25%//Q14839
C-NT2RP3004472//GERM CELL-LESS PROTEIN.//1.60E-61//170aa//40%//Q01820
C-NT2RP3004480//VACUOLAR PROTEIN SORTING-ASSOCIATED PROTEIN VPS35.//3.30E-113//466aa//42%//P34110
C-NT2RP3004490//Homo sapiens mRNA for Musashi, complete cds./4.00E-303//1385bp//99%//AB012851
C-NT2RP3004498//Mus musculus ROSA 26 transcription AS ROSA26AS mRNA, complete cds.//2.00E-249//1777bp//80%//U83176
C-NT2RP3004504//M.musculus mRNA for CPEB protein.//1.90E-295//893bp//92%//Y08260
C-NT2RP3004507//MOB1 PROTEIN (MPS1 BINDER 1).//3.70E-37//190aa//39%//P40484
C-NT2RP3004534//Mouse oncogene (ect2) mRNA, complete cds.//0//2075bp//87%//L11316
C-NT2RP3004544//THYROID RECEPTOR INTERACTING PROTEIN 10 (TRIP10) (FRAGMENT).//1.00E-22//1.3aa//53%//Q15642
C-NT2RP3004566//ZINC FINGER PROTEIN 84 (ZINC FINGER PROTEIN HPF2).//1.20E-95//434aa//43%//P51523
C-NT2RP3004569//ANKYRIN, BRAIN VARIANT 1 (ANKYRIN B) (ANKYRIN, NONERYTHROID).//3.80E-08//150aa//28%.//Q01484
C-NT2RP3004572//Homo sapiens TATA binding protein associated factor (TAFII150) mRNA, complete cds.//0//1853bp//99%//AF040701
C-NT2RP3004578//MYOSIN HEAVY CHAIN, CLONE 203 (FRAGMENT).//5.50E-12//396aa//23%//P39922
C-NT2RP3004594//Homo sapiens mRNA for AND-1 protein.//0//1807bp//99%//AJ006266
C-NT2RP3004617//ZINC-BINDING PROTEIN A33.//7.20E-75//464aa//35%//Q02084
C-NT2RP3004618//Homo sapiens putative RNA-binding protein Q99 mRNA, complete cds.//0//3972bp//98%//AF093097
C-NT2RP3004669//ETHANOLAMINE KINASE (EC 2.7.1.82) (EASILY SHOCKED PROTEIN).//1.70E-72//254aa//45%//P54352
C-NT2RP3004670//Homo sapiens GN6ST mRNA for N-acetylglucosamine-6-O-sulfotransferas e (GlcNAc6ST), complete cds.//0//2393bp//99%//AB014679
C-NT2RP4000008//CHLORINE CHANNEL PROTEIN P64.//2.60E-98//239aa//64%//P35526
C-NT2RP4000051//SYNAPTONEMAL COMPLEX PROTEIN SC65.//4.90E-51//335aa//37%//Q64375
C-NT2RP4000078//Homo sapiens mRNA for NS1-binding protein (NS1-BP).//0//2160bp//99%//AJ012449
C-NT2RP4000109//Homo sapiens mRNA for MEGF5, partial cds.//0//2161bp//99%//AB011538
C-NT2RP4000111//CLEAVAGE AND POLYADENYLATION SPECIFICITY FACTOR, 100 KD SUBUNIT (CPSF 100 KD SUBUNIT).//0//728aa//99%//Q10568
C-NT2RP4000129//Xenopus laevis F-box protein 28 (Fbx28) mRNA, partial cds.//1.40E-28//296bp//75%//AF176667
C-NT2RP4000147//Rattus norvegicus ADP-ribosylation factor-directed GTPase activating protein mRNA, complete cds.//4.30E-188//1543bp//78%//U35776
C-NT2RP4000210//PAIRED AMPHIPATHIC HELIX PROTEIN.//1.00E-71//396aa//36%//P22579
C-NT2RP4000212//ATRIAL GLAND-SPECIFIC ANTIGEN PRECURSOR (AGSA).//5.90E-15//104aa//40%//P15287
C-NT2RP4000243//Homo sapiens mRNA for cartilage-associated protein (CASP).//0//1932bp//99%//AJ006470
C-NT2RP4000246//NPC DERIVED PROLINE RICH PROTEIN 1 (NDPP-1).//2.70E-84//208aa//76%//Q03173
C-NT2RP4000259//GLUTATHIONE PEROXIDASE.2 (EC 1.11.1.9).//5.50E-29//153aa//43%//O23968
C-NT2RP4000290//HYPOTHETICAL 116.5 KD PROTEIN C20G8.09C IN CHROMOSOME I.//3.50E-297//1024aa//55%//P87115
C-NT2RP4000312//ADENYLATECYCLASE (EC 4.6.1.1) (ATP PYROPHOSPHATE-LYASE) (ADENYLYL CYCLASE).//1.50E-26//237aa//28%//Q01631
C-NT2RP4000323//KERATIN, ULTRA HIGH-SULFUR MATRIX PROTEIN (UHS KERATIN).//3.00E-07//101aa//32%//P26372
C-NT2RP4000367//Homo sapiens IkappaB kinase complex associated protein (IKAP) mRNA, complete cds.//0//4782bp//99%//AF044195
C-NT2RP4000370//MITOCHONDRIAL PEPTIDE CHAIN RELEASE FACTOR 1 PRECURSOR (MRF-1).//2.60E-77//262aa//54%//O75570
C-NT2RP4000376//Homo sapiens mRNA for phospholipase A2 activating protein.//0//2412bp//99%//AJ238243
C-NT2RP4000398//ZINC FINGER PROTEIN 140.//2.90E-110//435aa//50%//P52738
C-NT2RP4000415//Drosophila melanogaster fumble (fumble) mRNA, complete cds.//6.20E-19//902bp//57%//AF221546
C-NT2RP4000417//MANNOSYL-OLIGOSACCHARIDE ALPHA-1,2-MANNOSIDASE (EC 3.2.1.113)(MAN(9)-ALPHA-MANNOSIDASE)(FRAGMENT).//2.60E-51//438aa//33%//P45701
C-NT2RP4000449//Homo sapiens sirtuin type 1 (SIRT1) mRNA, complete cds.//0//3143bp//99%//AF083106
C-NT2RP4000455//TRANS-ACTING TRANSCRIPTIONAL PROTEIN ICP0.//3.00E-07//175aa//27%//P09309
C-NT2RP4000457//UBIQUITIN CARBOXYL-TERMINAL HYDROLASE 15 (EC 3.1.2.15) (UBIQUITIN THIOLESTERASE 15) (UBIQUITIN-SPECIFIC PROCESSING PROTEASE 15)(DEUBIQUITINATING ENZYME 15).//2.50E-37//291aa//38%//P50101
C-NT2RP4000481//ATP-DEPENDENT RNA HELICASE DOB1 (MRNA TRANSPORT REGULATOR MTR4).//1.90E-67//721aa//29%//Q09475
C-NT2RP4000498//MOB1 PROTEIN (MPS1 BINDER 1).//8.80E-50//214aa//50%//P40484
C-NT2RP4000518//ATP-DEPENDENT RNA HELICASE ROK1.//1.50E-106//495aa//45%//P45818
C-NT2RP4000524//Mus musculus Sec8 mRNA, complete cds.//0//3131bp//87%//AF022962
C-NT2RP4000528//NPL4 PROTEIN.//9.80E-86//515aa//37%//P33755
C-NT2RP4000556//SUR4 PROTEIN (SRE1 PROTEIN).//7.40E-14//233aa//31%//P40319
C-NT2RP4000614//Homo sapiens TLS-associated protein TASR-2 mRNA, complete cds.//2.90E-188//863bp//99%//AF067730
C-NT2RP4000648//TRANS-ACTING TRANSCRIPTIONAL PROTEIN ICP0.//3.70E-07//175aa//27%//P09309
C-NT2RP4000657//SPORE COAT POLYSACCHARIDE BIOSYNTHESIS PROTEIN SPSE.//1.10E-32//350aa//30%//P39625
C-NT2RP4000713//HYPOTHETICAL 55.1 KD PROTEIN B0416.5 IN CHROMOSOME X.//1.10E-13//295aa//27%//Q11073
C-NT2RP4000724//RETROVIRUS-RELATED ENV POLYPROTEIN.//3.20E-191//199aa//78%//P10267
C-NT2RP4000737//Mus musculus F-box protein FBL10 mRNA, partial cds.//4.60E-250//1462bp//84%//AF176524
C-NT2RP4000781//HYPOTHETICAL 27.7 KD PROTEIN IN CPT1-SPC98 INTERGENIC REGION.//0.000000032//67aa//31%//P53915
C-NT2RP4000817//GLUCOAMYLASE S1/S2 PRECURSOR (EC 3.2.1.3) (GLUCAN1,4-ALPHA- GLUCOSIDASE) (1,4-ALPHA-D-GLUCAN GLUCOHYDROLASE).//9.80E-11//503aa//23%//P08640
C-NT2RP4000837//Homo sapiens mRNA for zinc finger protein SALL1.//4.30E-94//810bp//65%//Y18265
C-NT2RP4000839//VEGETATIBLE INCOMPATIBILITY PROTEIN HET-E-1.//8.50E-21//271aa//28%//Q00808
C-NT2RP4000855//AMINOPEPTIDASE B (EC 3.4.11.6) (ARGINYL AMINOPEPTIDASE)(ARGININE AMINOPEPTIDASE) (CYTOSOL AMINOPEPTIDASE IV) (AP-B).//5.70E-82//324aa//48%//O09175
C-NT2RP4000865//ZINC FINGER PROTEIN ZFP-36 (FRAGMENT).//4.10E-85//174aa//55%//P16415
C-NT2RP4000878//MYELOID UPREGULATED PROTEIN.//6.20E-91//173aa//87%//O35682
C-NT2RP4000879//UBIQUITIN-ACTIVATING ENZYME El (A1S9 PROTEIN).//9.60E-96//513aa//42%//P22314
C-NT2RP4000907//Mouse NLRR-1 mRNA for leucine-rich-repeat protein, complete cds.//0//2127bp//86%//D45913
C-NT2RP4000925//FIBROMODULIN PRECURSOR (FM) (COLLAGEN-BINDING 59 KD PROTEIN).//2.60E-26//227aa//36%//Q06828
C-NT2RP4000927//UBIQUITIN CARBOXYL-TERMINAL HYDROLASE DUB-1 (EC 3.1.2.15) (UBIQUITIN THIOLESTERASE DUB-1) (UBIQUITIN-SPECIFIC PROCESSING PROTEASE DUB-1) (DEUBIQUITINATING ENZYME 1).//1.50E-76//346aa//43%//Q61068
C-NT2RP4000928//Homo sapiens mRNA for CDS2 protein.//0//2487bp//99%//Y16521
C-NT2RP4000929//PUTATIVE ATP-DEPENDENT RNA HEUCASE MJ1505.//1.40E-07//185aa//25%//Q58900
C-NT2RP4000973//PROBABLE PROTEIN DISULFIDE ISOMERASE P5 PRECURSOR (EC 5.3.4.1).//1.40E-26//90aa//42%//P38660
C-NT2RP4000979//Homo sapiens putative HIV-1 infection related protein mRNA, partial cds.//2.30E-81//389bp//100%//AF094583
C-NT2RP4000989//UNC-47 PROTEIN.//8.20E-06//173aa//25%//P34579
C-NT2RP4000997//DNA-DIRECTED RNA POLYMERASE 1135 KD POLYPEPTIDE (EC 2.7.7.6) (RNA POLYMERASE I SUBUNIT 2) (RPA135).//0//838aa//87%//P70700
C-NT2RP4001004//VACUOLAR PROTEIN 8.//3.70E-16//401aa//26%//P39968
C-NT2RP4001010//Rattus norvegicus PSD-95/SAP90-associated protein-4 mRNA, complete cds.//3.50E-257//1377bp//91%//U67140
C-NT2RP4001029//Homo sapiens transcription factor LBP-1b (LBP-1) mRNA, complete cds.//0//2002bp//98%//AF198487
C-NT2RP4001041//PROBABLE LEUCYL-TRNA SYNTHETASE (EC 6.1.1.4) (LEUCINE--TRNA LIGASE)//1.50E-92//443aa//44%//Q09996
C-NT2RP4001064//SYNAPTONEMAL COMPLEX PROTEIN SC65.//6.70E-51//335aa//37%//Q64375
C-NT2RP4001079//CALCIUM-TRANSPORTING ATPASE 1 (EC 3.6.1.38) (GOLGI CA2⁺-ATPASE).//1.30E-123//563aa//46%//P13586
C-NT2RP4001080//Homo sapiens mRNA for Rod1, complete cds.//0//1439bp//99%//AB023967
C-NT2RP4001086//NEUROFILAMENT TRIPLET H PROTEIN (200 KD NEUROFILAMENT PROTEIN) (NF-H).//2.30E-07//474aa//22%//P12036
C-NT2RP4001095//DOUBLE-STRANDED RNA-SPECIFIC EDITASE 1 (EC 3.5.-.-) (DSRNA ADENOSINE DEAMINASE) (RNA EDITING ENZYME 1).//2.60E-17//121aa//36%//P51400
C-NT2RP4001117//PROTEIN TRANSPORT PROTEIN SEC61 ALPHA SUBUNIT.//1.90E-115//224aa//100%//P38378
C-NT2RP4001122/mPD PROTEIN.//1.40E-65//253aa7/41%//O15736
C-NT2RP4001126/TRICHOHYALIN.//2.90E-18//380aa//26%//Q07283
C-NT2RP4001143//SUCCINYL-DIAMINOPIMELATE DESUCCINYLASE (EC 3.5.1.18) (SDAP).//2.10E-07//93aa//33%//P44514
C-NT2RP4001148//SOF1 PROTEIN.//1.30E-104//236aa//52%//P33750
C-NT2RP4001149//Homo sapiens cleft lip and palate transmembrane protein 1 (CLPTM1) mRNA, complete cds.//4.40E-187//731bp//100%//AF037339
C-NT2RP4001150//NG-CAM RELATED CELL ADHESION MOLECULE PRECURSOR (NR-CAM) (BRAVO).//3.40E-29//385aa//29%//P35331
C-NT2RP4001174//NON-GREEN PLASTID TRIOSE PHOSPHATE TRANSLOCATOR PRECURSOR (CTPT).//4.70E-29//227aa//35%//P52178
C-NT2RP4001206//Drosophila melanogaster strawberry notch (sno) mRNA, complete cds.//4.40E-104//1460bp//65%//U95760
C-NT2RP4001207//Homo sapiens Ran binding protein 11 mRNA, complete cds.//0//2940bp//99%//AF111109
C-NT2RP4001213//ZINC FINGER PROTEIN 184 (FRAGMENT).//5.70E-141//511aa//43%//Q99676
C-NT2RP4001219//PROBABLE PROTEIN DISULFIDE ISOMERASE P5 PRECURSOR (EC 5.3.4.1).//6.20E-27//90aa//42%//P38660
C-NT2RP4001228//RING CANAL PROTEIN (KELCH PROTEIN).//1.80E-103//508aa//43%//Q04652
C-NT2RP4001256//Homo sapiens mRNA for gamma tubulin ring complex protein (76p gene).//0//2006bp//100%//AJ249677
C-NT2RP4001260//Homo sapiens F-box protein Fbx21 (FBX21) mRNA, complete cds.//0//1866bp//100%//AF174601
C-NT2RP4001274//Human transporter protein (g17) mRNA, complete cds.//4.40E-58//1196bp//61%//U49082
C-NT2RP4001276//TRICHOHYALIN.//7.90E-09//126aa//32%//Q07283
C-NT2RP4001313//MITOCHONDRIAL IMPORT RECEPTOR SUBUNIT TOM40 (MOM38 PROTEIN) (TRANSLOCASE OF OUTER MEMBRANE 40 KD SUBUNIT).//5.90E-17//296aa//29%//P24391
C-NT2RP4001315//Bos taurus mRNA for Rab5 GDP/GTP exchange factor, Rabex5.//8.50E-213//1129bp//92%//AJ001119
C-NT2RP4001336//GUANINE NUCLEOTIDE-BINDING PROTEIN BETA SUBUNIT-LIKE PROTEIN.//0.000016//186aa//29%//O24076
C-NT2RP4001339//Homo sapiens mRNA for AMMERC1 protein.//9.20E-160//736bp//99%//AJ007014
C-NT2RP4001345//Homo sapiens mRNA for LCAT-like lysophospholipase (LLPL), complete cds.//2.7e-310//1400bp//100%//AB017494
C-NT2RP4001351//Human ovarian cancer downregulated myosin heavy chain homolog (Doc1) mRNA, complete cds.//1.40E-58//2425bp//59%//U53445
C-NT2RP4001372//IRREGULAR CHIASM C-ROUGHEST PROTEIN PRECURSOR (IRREC PROTEIN).//1.60E-19//222aa//30%//Q08180
C-NT2RP4001375//NON-RECEPTOR TYROSINE KINASE SPORE LYSIS A (EC 2.7.1.112) (TYROSINE- PROTEIN KINASE 1).//9.20E-17//146aa//35%//P18160
C-NT2RP4001379//HYPOTHETICAL 49.1 KD PROTEIN C11D3.06 IN CHROMOSOME I.//2.00E-53//436aa//30%//Q10085
C-NT2RP4001389//KESlPROTEIN.//1.70E-31//342aa//34%//P35844
C-NT2RP4001407//TRICHOHYALIN.//1.90E-05//298aa//21%//P22793
C-NT2RP4001414//SEPTIN 2 HOMOLOG (FRAGMENT).//7.70E-190//422aa//82%//Q14141
C-NT2RP4001433//ZINC FINGER PROTEIN 184 (FRAGMENT).//1.20E-138//419aa//54%//Q99676
C-NT2RP4001474//Xenopus laevis putative Zic3 binding protein mRNA, complete cdsJ/2.70E-66//738bp//71%//AF129131
C-NT2RP4001483//2-OXOGLUTARATE DEHYDROGENASE El COMPONENT PRECURSOR (EC 1.2.4.2) (ALPHA-KETOGLUTARATE DEHYDROGENASE).//0//962aa//78%//Q02218
C-NT2RP4001498//ANKYRIN REPEAT-CONTAINING PROTEIN AKR1.//1.00E-27//374aa//29%//P39010
C-NT2RP4001529//Homo sapiens transcription factor LBP-1b (LBP-1) mRNA, complete cds.//0//2002bp//98%//AF198487
C-NT2RP4001547//HYPOTHETICAL 45.0 KD PROTEIN IN NOT1/CDC39-HMR INTERGENIC REGION.//5.70E-54//242aa//38%//P25656
C-NT2RP4001551//Homo sapiens chromatin-specific transcription elongation factor FACT 140 kDa subunit mRNA, complete cds.//0//3202bp//99%//AF152961
C-NT2RP4001555//PUTATIVE ENDONUCLEASE VIII (EC 3.2.-.-).//4.70E-09//216aa//24%//P96902
C-NT2RP4001567//ARMADILLO SEGMENT POLARITY PROTEIN.//5.40E-07//213aa//26%//Q02453
C-NT2RP4001568//ZINC FINGER PROTEIN GCS1.//1.80E-10//109aa//36%//P35197
C-NT2RP4001574//COATOMER GAMMA SUBUNIT (GAMMA-COAT PROTEIN) (GAMMA-COP).//0//874aa//96%//P53620
C-NT2RP4001575//Rattus norvegiqus mRNA for ARE1 protein.//0//1087bp//87%//AJ223830
C-NT2RP4001592//ISOLEUCYL-TRNA SYNTHETASE (EC 6.1.1.5) (ISOLEUCINE--TRNA LIGASE) (ILERS).//1.70E-141//373aa7/47%//P73505
C-NT2RP4001634//CENTROMERIC PROTEIN E (CENP-E PROTEIN).//2.80E-14//652aa//22%//Q02224
C-NT2RP4001638//DNA REPAIR/TRANSCRIPTION PROTEIN MET18/MMS19.//5.10E-46//234aa//32%//P40469
C-NT2RP4001644//MYOSIN UGHT CHAIN KINASE (EC 2.7.1.117) (MLCK).//6.40E--19//111aa//45%//P25323
C-NT2RP4001656//VACUOLAR BIOGENESIS PROTEIN END1 (PEP5 PROTEIN).//1.10E-45//310aa//27%//P12868
C-NT2RP4001696//CLEAVAGE AND POLYADENYLATION SPECIFICITY FACTOR, 100 KD SUBUNIT (CPSF 100 KD SUBUNIT).//4.00E-10//243aa//25%//Q10568
C-NT2RP4001725//GUANINE NUCLEOTIDE-BINDING PROTEIN BETA SUBUNIT.//3.00E-10//128aa//32%//Q10282
C-NT2RP4001730//UDP-GLUCOSE:GLYCOPROTEIN GLUCOSYLTRANSFERASE PRECURSOR (EC 2.4.1.-) (DUGT).//6.40E-170//1168aa//33%//Q09332
C-NT2RP4001753//ZINC FINGER PROTEIN 84 (ZINC FINGER PROTEIN HPF2).//3.90E-236//665aa//58%//P51523
C-NT2RP4001760//PUTATIVE RHO/RAC GUANINE NUCLEOTIDE EXCHANGE FACTOR (RHO/RAC GEF) (FACIOGENITAL DYSPLASIA PROTEIN).//4.10E-16//263aa//27%//P98174
C-NT2RP4001790//Homo sapiens zinc finger protein ZFP-95 (ZFP95) mRNA, alternatively spliced, complete cds.//0//3053bp//99%//AF170025
C-NT2RP4001822//PLATELET-ENDOTHELIAL TETRASPAN ANTIGEN 3 (PETA-3) (GP27) (MEMBRANE GLYCOPROTEIN SFA-1) (CD151 ANTIGEN).//1.20E-30//241aa//30%//O35566
C-NT2RP4001823//MICROFIBRIL-ASSOCIATED GLYCOPROTEIN 4.//1.10E-19//77aa//54%//P55083
C-NT2RP4001838//Homo sapiens CoREST protein (COREST) mRNA, complete cds.//6.30E-99//555bp//73%//AF155595
C-NT2RP4001849//SH3-BINDING PROTEIN 3BP-1.//1.40E-85//489aa//43%//P55194
C-NT2RP4001861/HTUCHOHYALIN.//1.00E-35//307aa//34%//P37709
C-NT2RP4001896//VEGETATIBLE INCOMPATIBILITY PROTEIN HET-E-1.//1.40E-08//345aa7/25%//Q00808
C-NT2RP4001927//MICROTUBULE-ASSOCIATED PROTEIN YTM1.//1.30E-38//258aa//32%//Q12024
C-NT2RP4001938//TRANSCRIPTIONAL REPRESSOR CTCF.//9.80E-60//303aa//38%//P49711
C-NT2RP4001946//PROTEIN-L-ISOASPARTATE O-METHYLTRANSFERASE (EC 2.1.1.77) (PROTEIN- BETA-ASPARTATE METHYLTRANSFERASE) (PIMT) (PROTEIN L-ISOASPARTYL METHYLTRANSFERASE) (L-ISOASPARTYL PROTEIN CARBOXYL METHYLTRANSFERASE).//1.50E-13//211aa//28%//Q43209
C-NT2RP4001950//GLUTAMIC ACID-RICH PROTEIN PRECURSOR.//1.20E-13//356aa//27%//P13816
C-NT2RP4001966//Mus musculus ODZ3 (Odz3) mRNA, partial cds.//0//3203bp//87%//AF195418
C-NT2RP4001975//Homo sapiens golgi membrane protein GP73 mRNA, complete cds.//0//3024bp//99%//AF236056
C-NT2RP4002018//RING CANAL PROTEIN (KELCH PROTEIN).//6.90E-24//370aa//27%//Q04652
C-NT2RP4002047//GTP-BINDING PROTEIN LEPA.//1.50E-168//601aa//52%//O67618
C-NT2RP4002058//PUTATIVE PRE-MRNA SPLICING FACTOR RNA HELICASE (DEAH BOX PROTEIN 13)//1.00E-137//679aa//40%//O43143
C-NT2RP4002078//ZINC FINGER PROTEIN 91 (ZINC FINGER PROTEIN HTF10) (HPF7).//3.00E-150//722aa//39%//Q05481
C-NT2RP4002081//TRANSCRIPTION INITIATION FACTOR IIA ALPHA AND BETA CHAINS (TFIIA P35 AND PI 9 SUBUNITS) (TFIIA-42) (TFIIAL).//6.70E-06//250aa//31%//P52655
C-NT2RP4002408//PROTEIN KINASE CEK1 (EC 2.7.1.-).//1.50E-63//159aa//53%//P38938
C-NT2RP4002791//NUCLEOPROTEIN TPR.//6.50E-05//659aa//23%//P12270
C-NT2RP5003461//RLR1 PROTEIN.//9.70E-22//177aa//27%//P53552
C-NT2RP5003477//VEGETATIBLE INCOMPATIBILITY PROTEIN HET-E-1.//5.50E-15//280aa//27%//Q00808
C-NT2RP5003500//Mus musculus mRNA for heparan sulfate 6-sulfotransferase 2, complete cds.//1.30E-237//820bp//87%//AB024565
C-NT2RP5003506//Homo sapiens putative G protein-coupled receptor (RAIG1) mRNA, complete cds.//0//2289bp//99%//AF095448
C-NT2RP5003522//NADPH-CYTOCHROME P450 REDUCTASE (EC 1.6.2.4) (CPR).//3.30E-23//219aa//40%//P37116
C-OVARC1000001//Homo sapiens mRNA for actin binding protein ABP620, complete cds.//7.00E-217//683bp//99%//AB029290
C-OVARC1000006//HISTONE H2A.1.//1.10E-55//117aa//99%//P02262
C-OVARC1000013//APOPTOTIC PROTEASE ACTIVATING FACTOR 1 (APAF-1).//4.20E-06//102aa//32%//O14727
C-OVARC1000014//Homo sapiens GLE1 (GLE1) mRNA, complete cdsJ/2.60E-295//1393bp//97%//AF058922
C-OVARC1000060//EXTRACELLULAR RIBONUCLEASE LE PRECURSOR (EC 3.1.27.1) (RNASE LE).//3.20E-07//60aa//45 %//P80022
C-OVARC1000071//Homo sapiens NTF2-related export protein NXT1 (NXT1) mRNA, complete cds.//1.50E-47//727bp//67%//AF156957
C-OVARC1000085//Human mRNA for proteasome subunit HC5.//1.00E-151//699bp//100%//D00761
C-OVARC1000087//HISTONE MACRO-H2A.1.//1.60E-12//174aa//26%//Q02874
C-OVARC1000091//HOST CELL FACTOR C1 (HCF) (VP16 ACCESSORY PROTEIN) (HFC1) (VCAF) (CFF).//8.40E-14//259aa//30%//P51610
C-OVARC1000106//TROPOMYOSIN 1, FUSION PROTEIN 33.//0.000032//165aa//27%//P49455
C-OVARC1000139//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN HOMOLOG).//2.70E-12//120aa//32%//Q13107
C-OVARC1000151//Homo sapiens partial mRNA for putative protein p38 interacting with transcription factor Spl.//2.50E-95//461bp//98%//AJ242975
C-OVARC1000209//Oryza sativa submergence induced protein 2A mRNA, complete cds.//1.80E-32//511bp//65%//AF068332
C-OVARC1000241//HYPOXIA-INDUCIBLE FACTOR 1 ALPHA (HIF-1 ALPHA) (ARNT INTERACTING PROTEIN) (MEMBER OF PAS PROTEIN 1) (MOP1) (HIF1 ALPHA).//8.20E-120//351aa//54%//Q16665
C-OVARC1000288//VACUOLAR AMINOPEPTIDASE I PRECURSOR (EC 3.4.11.22) (POLYPEPTIDASE)(LEUCINE AMINOPEPTIDASE IV) (LAPIV) (AMINOPEPTIDASE III)(AMINOPEPTIDASE YSCI).//5.40E-53//384aa//30%//P14904
C-OVARC1000304//PROTEIN MOV-10.//1.10E-249//519aa//87%//P23249
C-OVARC1000309//THREONINE SYNTHASE (EC 4.2.99.2).//2.70E-40//154aa//38%//P29363
C-OVARC1000326//Rattus norvegicus lamina-associated polypeptide 1C (LAP1C) mRNA, complete cds.//9.20E-148//787bp//76%//U19614
C-OVARC1000335//HYPOTHETICAL 39.3 KD PROTEIN IN GCN4-WBP1 INTERGENIC REGION.//5.90E-14//200aa//27%//P40004
C-OVARC1000437//TENSIN.//7.90E-181//340aa//84%//Q04205
C-OVARC1000465//PROTEIN TRANSPORT PROTEIN SEC7.//1.20E-25//227aa//25%//P11075
C-OVARC1000473//DUAL SPECIFICITY PROTEIN PHOSPHATASE 3 (EC 3.1.3.48) (EC 3.1.3.16) (DUAL SPECIFICITY PROTEIN PHOSPHATASE VHR).//3.10E-10//125aa//35%//P51452
C-OVARC1000479//Rattus norvegicus mRNA for TIP120, complete cds.//0//1872bp//89%//D87671
C-OVARC1000520//Homo sapiens supervillin mRNA, complete cds.//2.20E-157//892bp//91%//AF051850
C-OVARC1000556//RIBOSOMAL PROTEIN S6 KINASE II ALPHA 2 (EC 2.7.1.-) (S6KII-ALPHA 2) (P90-RSK 2) (RIBOSOMAL S6 KINASE 3) (RSK3) (PP90RSK3).//3.30E-67//132aa//95%//Q15349
C-OVARC1000564//Homo sapiens sorting nexin 5 (SNX5) mRNA, complete cds.//1.0e-310//1440bp//98%//AF121855
C-OVARC1000649//Human squamous cell carcinama of esophagus mRNA for GRB-7 SH2 domain protein, complete cds.//0//1812bp//98%//D43772
C-OVARC1000679//Homo sapiens myosin-IXa mRNA, complete cds.//0//808bp//99%//AF117888
C-OVARC1000682//PROCESSING ALPHA-1,2-MANNOSIDASE (EC 3.2.1.-) (ALPHA-1,2-MANNOSIDASE 1B).//1.10E-209//293aa//95%//P39098
C-OVARC1000722//Homo sapiens chromosome 1q21-1q23 beta-1,4-galactosyltransferase mRNA, complete cds.//0//759bp//98%//AF038661
C-OVARC1000746//MATERNAL EFFECT PROTEIN STAUFEN.//0.000000017//78aa//48%//P25159
C-OVARC1000751//PROBABLE PROTEIN PHOSPHATASE 2C T23F11.1 (EC 3.1.3.16) (PP2C).//5.60E-11//74aa//37%//P49596
C-OVARC1000771//RAS-RELATED PROTEIN RAB-2.//1.10E-46//121aa//79%//P08886
C-OVARC1000800//MITOCHONDRIAL STRESS-70 PROTEIN PRECURSOR (75 KD GLUCOSE REGULATED PROTEIN) (GRP 75).//3.90E-46//78aa//98%//O35501
C-OVARC1000834//Homo sapiens mRNA for atopy related autoantigen CALC.//2.80E-258//1183bp//99%//Y17711
C-OVARC1000846//NUCLEOLIN (PROTEIN C23).//0.0000097//109aa//30%//P08199
C-OVARC1000850//Homo sapiens PB39 mRNA, complete cds.//0//2095bp//99%//AF045584
C-OVARC1000862//M.musculus mRNA for FT1.//5.90E-226//1498bp//81%//Z67963
C-OVARC1000876//MOB1 PROTEIN (MPS1 BINDER 1).//2.20E-50//206aa//52%//P40484
C-OVARC1000885//OXIDOREDUCTASE UCPA (EC 1.-.-.-).//1.30E-32//170aa//34%//P37440
C-OVARC1000915//Homo sapiens histone deacetylase 5 mRNA, complete cds.//1.60E-121//591bp//97%//AF132608
C-OVARC1000936//COAT PROTEIN GP37 (ENV PROTEIN GP37).//0.0000054//135aa//28%//P03398
C-OVARC1000937//S-PHASE ENTRY CYCLIN 6.//4.90E-10//61aabp//49%//P32943
C-OVARC1000945//Rattus norvegicus mRNA for atypical PKC specific binding protein, complete cds.//0//1961bp//82%//AB005549
C-OVARC1000959//HYPOTHETICAL PROTEIN MJ0933.//1.20E-17//127aa//33%//Q58343
C-OVARC1000999//ANKYRIN HOMOLOG PRECURSOR.//4.10E-11//189aa//32%//Q06527
C-OVARC1001034//Mus musculus Fn54 mRNA, partial cds.//1.50E-178//1113bp//86%//AF001533
C-OVARC1001038//Homo sapiens mRNA for Ariadne-2 protein.//0//1172bp//97%//AJ130978
C-OVARC1001051//EPIDERMAL GROWTH FACTOR RECEPTOR SUBSTRATE SUBSTRATE 15 (PROTEIN EPS15) (AF-1P PROTEIN).//1.10E-08//216aa//23%//P42566
C-OVARC1001055//PRE-B CELL ENHANCING FACTOR PRECURSORS.//1.90E-35//76aa//98%//P43490
C-OVARC1001065//Homo sapiens CGI-12 protein mRNA, complete cds.//1.00E-215//1027bp//98%//AF132946
C-OVARC1001068//Homo sapiens Era GTPase A protein (HERA-A) mRNA, partial cds.//0//1819bp//99%//AF082657
C-OVARC1001092//Homo sapiens mRNA for JM5 protein, complete CDS (clone IMAGE 53337, LLNLc110F1857Q7 (RZPD Berlin) and LLNLc110G0913Q7 (RZPD Berlin))-//2.00E-214//769bp//97%//AJ005897
C-OVARC1001107//Homo sapiens protein methyltransferase (JBP1) mRNA, complete cds.//6.10E-276//594bp//98%//AF167572
C-OVARC1001113//Homo sapiens diaphanous 1 (HDIA1) mRNA, complete cds.//5.1e-310//1588bp//93%//AF051782
C-OVARC1001154//Homo sapiens clone 24720 epithelin 1 and 2 mRNA, complete cds.//2.30E-296//1561bp//93%//AF055008
C-OVARC1001171//Homo sapiens translation initiation factor 3 47 kDa subunit mRNA, complete cds.//5.70E-151//436bp//92%//U94855
C-OVARC1001180//UBIQUITIN-LIKE PROTEIN DSK2.//1.10E-11//221aa//25%//P48510
C-OVARC1001200//Mus musculus mRNA for HS1 binding protein 3.//5.80E-88//658bp//80%//AJ132192
C-OVARC1001232//CLEAVAGE AND POLYADENYLATION SPECIFICITY FACTOR, 100 KD SUBUNIT (CPSF 100 KD SUBUNIT).//5.10E-22//83aa//37%//Q10568
C-OVARC1001244//H.sapiens mRNA for Drosophila female sterile homeotic (FSH) homologue.//0//1467bp//99%//X62083
C-OVARC1001271//NUCLEOLAR TRANSCRIPTION FACTOR 1 (UPSTREAM BINDING FACTOR 1) (UBF-1).//0.0000014//224aa//26%//P25976
C-OVARC1001306//N-MYC PROTO-ONCOGENE PROTEIN.//0.00000073//247aa//27%//P18444
C-OVARC1001342//40S RIBOSOMAL PROTEIN S8.//1.40E-110//207aa//99%//P09058
C-OVARC1001372//Homo sapiens liprin-alpha4 mRNA, partial cds.//2.00E-252//1146bp//99%//AF034801
C-OVARC1001381//Homo sapiens mRNA for candidate tumor suppressor involved in B-CLL.//6.00E-148//683bp//99%//AJ224819
C-OVARC1001417//Homo sapiens thyroid hormone receptor-associated protein complex component TRAP170 mRNA, complete cds.//0//1715bp//99%//AF135802
C-OVARC1001419//Homo sapiens GOK (STIM1) mRNA, complete cds.//4.90E-48//586bp//69%//U52426
C-OVARC1001436//ENL PROTEIN.//0.00000009//81aa//39%//Q03111
C-OVARC1001476//Mus musculus YGR163w mRNA homologue, complete cds.//1.80E-187//510bp//89%//AB017616
C-OVARC1001496//Homo sapiens C-terminal binding protein 2 mRNA, complete cds.//0//1876bp//98%//AF016507
C-OVARC1001506//POLYCYSTIN PRECURSOR (AUTOSOMAL DOMINANT POLYCYSTIC KIDNEY DISEASE PROTEIN 1).//0//777aa//91%//P98161
C-OVARC1001555//NGG1-INTERACTING FACTOR 3.//4.40E-19//130aa//40%//P53081
C-OVARC1001577//Homo sapiens SRp46 splicing factor transcribed retropseudogene.//0//1167bp//100%//AF031165
C-OVARC1001610//Homo sapiens choline/ethanolaminephosphotransferase (CEPT1) mRNA, complete cds.//0//1870bp//99%//AF068302
C-OVARC1001703//Mus musculus ARL-6 interacting protein-2 (Aip-2) mRNA, complete cds.//3.50E-16//399bp//61%//AF133670
C-OVARC1001711//CORNIFIN B (SMALL PROLINE-RICH PROTEIN 1B) (SPR1B) (SPR1 B).//2.80E-10//106aa//38%//Q62267
C-OVARC1001713//ENDOZEPINE-RELATED PROTEIN PRECURSOR (MEMBRANE-ASSOCIATED DIAZEPAM BINDING INHIBITOR) (MA-DBI).//4.40E-40//195aa//41%//P07106
C-OVARC1001726//APICAL-LIKE PROTEIN (APXL PROTEIN).//4.30E-16//116aa//43%//Q13796
C-OVARC1001731//TROPOMYOSIN ALPHA CHAIN, FIBROBLAST ISOFORM F2.//4.00E-122//282aa//85%//P08942
C-OVARC1001762//N-TERMINAL ACETYLTRANSFERASE 1 (EC 2.3.1.88) (AMINO-TERMINAL, ALPHA- AMINO, ACETYLTRANSFERASE 1).//6.40E-85//514aa//34%//P12945
C-OVARC1001766//Homo sapiens eukaryotic translation initiation factor eIF3, p35 subunit mRNA, complete cds.//0//963bp//99%//U97670
C-OVARC1001809//Mus musculus sphingosine kinase (SPHKIa) mRNA, partial cds.//2.70E-190//1624bp//76%//AF068748
C-OVARC1001942//N-TERMINAL ACETYLTRANSFERASE 1 (EC 2.3.1.88) (AMINO-TERMINAL, ALPHA- AMINO, ACETYLTRANSFERASE 1).//3.10E-81//497aa//35%//P12945
C-OVARC1001943//Mus musculus DEBT-91 mRNA, complete cds.//0//2035bp//87%//AF143859
C-OVARC1001987//Homo sapiens prolactin regulatory element-binding protein (PREB) mRNA, complete cds.//0//1083bp//99%//AF203687
C-OVARC1002050//Homo sapiens mRNA for actin binding protein ABP620, complete cds.//0//1019bp//99%//AB029290
C-OVARC1002112//HISTONE MACRO-H2A.1.//3.00E-174//371aa//90%//Q02874
C-OVARC1002127//SODIUM-INDEPENDENT ORGANIC ANION TRANSPORTER 2 (BRAIN DIGOXIN CARRIER PROTEIN) (BRAIN-SPECIFIC ORGANIC ANION TRANSPORTER) (OATP-B1).//5.40E-52//306aa//35%//O35913
C-OVARC100213 8//SAP1 PROTEIN.//7.60E-60//128aa//59%//P39955
C-OVARC1002156//Danio rerio uridine kinase mRNA, complete cds.//6.00E-16//262bp//64%//AF195851
C-OVARC1002165//3-OXO-5-ALPHA-STEROID 4-DEHYDROGENASE 2 (EC 1.3.99.5) (STEROID 5-ALPHA-REDUCTASE 2) (SR TYPE 2).//7.60E-08//114aa//37%//P31213
C-OVARC1002182//BETA-TRCP (BETA-TRANSDUCIN REPEAT-CONTAINING PROTEIN) (BTRCP).//1.70E-09//207aa//30%//Q91854
C-PLACE1000004//Homo sapiens IDN3-B mRNA, complete cds.//0//2365bp//99%//AB019602
C-PLACE1000007//PROBABLE UBIQUITIN CARBOXYL-TERMINAL HYDROLASE R10E11.3 (EC 3.1.2.15) (UBIQUITIN THIOLESTERASE) (UBIQUITIN-SPECIFIC PROCESSING PROTEASE) (DEUBIQUITINATING ENZYME).//1.60E-81//212aa//70%//P34547
C-PLACE1000040//TRANSFORMING PROTEIN P21/K-RAS 2B.//1.40E-17//185aa//32%//P08643
C-PLACE1000061//Human ribosomal protein L37a mRNA sequence.//7.90E-54//190bp//94%//L22154
C-PLACE1000066//SSU72 PROTEIN.//1.10E-39//206aa//43%//P53538
C-PLACE1000081//Human SEC7 homolog Tic (TIC) mRNA, complete cds.//0//2077bp//99%//U63127
C-PLACE1000133/TRANSCRIPTION FACTOR BTF3 (RNA POLYMERASE B TRANSCRIPTION FACTOR 3).//1.80E-62//158aa//81%//P20290
C-PLACE1000142//3-HYDROXYBUTYRYL-COA DEHYDRATASE (EC 4.2.1.55) (CROTONASE).//2.80E-29//134aa//43%//P52046
C-PLACE1000184//Homo sapiens estrogen-related receptor gamma mRNA, complete cds.//1.30E-305//1417bp//98%//AF058291
C-PLACE1000185//Homo sapiens mRNA for N-Acetylglucosamine kinase.//4.90E-258//1183bp//99%//AJ242910
C-PLACE1000213//GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).//4.50E-05//197aa//26%//P08640
C-PLACE1000383//Homo sapiens mRNA for MTMR1 protein.//0//753bp//99%//AJ224979
C-PLACE1000401//POLIOVIRUS RECEPTOR PRECURSOR (CD155 ANTIGEN).//2.70E-30//352aa//31%//P15151
C-PLACE1000406//PTB-ASSOCIATED SPLICING FACTOR (PSF).//1.20E-132//334aa//72%//P23246
C-PLACE1000420//7,8-DIHYDRO-8-OXOGUANINE TRIPHOSPHATASE (EC 3.1.6.-) (8-OXO-DGTPASE).//2.80E-06//134aa//29%//P53368
C-PLACE1000492//Rat vacuolar protein sorting homolog r-vps33b mRNA, complete cds.//0//2041bp//87%//U35245
C-PLACE1000547//Homo sapiens GDP-mannose pyrophosphorylase B (GMPPB) mRNA, complete cds.//3.70E-241//1124bp//98%//AF135421
C-PLACE1000583//ZINC FINGER PROTEIN 83 (ZINC FINGER PROTEIN HPF1).//1.60E-47//207aa//46%//P51522
C-PLACE1000588//INTERFERON-INDUCED GUANYLATE-BINDING PROTEIN 1 (GUANINE NUCLEOTIDE- BINDING PROTEIN 1).//1.60E-270//437aa//86%//P32455
C-PLACE1000596//Homo sapiens mRNA for NS1-binding protein (NS1-BP).//0//1540bp//99%//AJ012449
C-PLACE1000610//MSN5 PROTEIN.//0.0000026//136aa//26%//P52918
C-PLACE1000611//Rattus norvegicus neural membrane protein 35 mRNA, complete cds.//2.00E-55//779bp//67%//AF044201
C-PLACE1000636//MALE STERILITY PROTEIN 2.//1.20E-39//261aa//27%//Q08891
C-PLACE1000653//Homo sapiens N-acetylglucosamine-phosphate mutase mRNA, complete cds.//0//1992bp//99%//AF180371
C-PLACE1000656//Homo sapiens mRNA for JM4 protein, complete CDS (clone IMAGE 546750 and LLNLC110F1857Q7 (RZPD Berlin)).//2.10E-277//1260bp//99%//AJ005896
C-PLACE1000706//Homo sapiens transcriptional intermediary factor 1 gamma mRNA, complete cds.//0//1366bp//99%//AF119043
C-PLACE1000755//Homo sapiens mRNA for Helicase-MOI, complete cds.//4.60E-250//1189bp//97%//AB028449
C-PLACE1000769//Homo sapiens CGI-18 protein mRNA, complete cds.//0//1985bp//98%//AF132952
C-PLACE1000786//PUTATIVE RHO/RAC GUANINE NUCLEOTIDE EXCHANGE FACTOR (RHO/RAC GEF) (FACIOGENITAL DYSPLASIA PROTEIN HOMOLOG).//7.10E-09//59aa//47%//P52734
C-PLACE1000793//NEUROGENIC PROTEIN BIG BRAIN.//1.70E-07//251aa//24%//P23645
C-PLACE1000863//PUTATIVE MITOCHONDRIAL 40S RIBOSOMAL PROTEIN YHR148W.//2.50E-49//181aa//54%//P32899
C-PLACE1000909//ANKYRIN REPEAT-CONTAINING PROTEIN AKR1.//2.60E-19//404aa//26%//P39010
C-PLACE1000977//BETA-CHIMAERIN (BETA-CHIMERIN).//4.40E-22//129aa//35%//Q03070
C-PLACE1000979//ZINC FINGER PROTEIN 135.//2.50E-153//326aa//64%//P52742
C-PLACE1000987//Rattus norvegicus late gestation lung 2 protein (Lgl2) mRNA, complete cds.//5.90E-278//1476bp//92%//AF110195
C-PLACE1001036//Homo sapiens mRNA for alpha integrin binding protein 63, partial.//0//1988bp//99%//AJ131721
C-PLACE1001054//Homo sapiens mRNA for RuvB-like DNA helicase TIP49b, complete cds.//4.00E-300//1355bp//100%//AB024301
C-PLACE1001062//Homo sapiens mRNA for lysine-ketoglutarate reductase/saccharopine dehydrogenase, partial CDS.//1.60E-207//742bp//99%//AJ007714
C-PLACE1001092//Homo sapiens sorting nexin 4 mRNA, complete cds.//0//1500bp//99%//AF065485
C-PLACE1001104//MYOSIN HEAVY CHAIN, NON-MUSCLE (ZIPPER PROTEIN) (MYOSIN II).//6.80E-18//529aa//23%//Q99323
C-PLACE1001118//ZINC FINGER PROTEIN 135.//5.40E-147//443aa//57%//P52742
C-PLACE1001171//MYOTUBULARIN.//7.10E-84//198aa//73%//Q13496
C-PLACE1001238//Mouse mRNA for RNA polymerase I associated factor (PAF53), complete cds.//2.00E-202//1333bp//80%//D14336
C-PLACE1001257//RING CANAL PROTEIN (KELCH PROTEIN).//4.30E-54//257aa//46%//Q04652
C-PLACE1001294//Mus musculus XY body protein (Xybp) mRNA, complete cds.//6.20E-223//1092bp//78%//AF120207
C-PLACE1001304//Homo sapiens C2H2 (Kruppel-type) zinc finger protein mRNA, complete cds.//0//2145bp//99%//AF159567
C-PLACE1001377//Homo sapiens ADAM10 (ADAM10) mRNA, complete cds.//5.90E-228//827bp//99%//AF009615
C-PLACE1001383//ZINC-FINGER PROTEIN UBI-D4 (APOPTOSIS RESPONSE ZINC FINGER PROTEIN REQUIEM).//3.00E-33//138aa//42%//Q61103
C-PLACE1001387//EPIDERMAL GROWTH FACTOR RECEPTOR KINASE SUBSTRATE EPS8.//2.30E-61//132aa//46%//Q12929
C-PLACE1001517//Homo sapiens gene for glycosylphosphatidylinositol anchor attachment 1 (GPAA1), complete cds.//4.60E-112//392bp//87%//AB002137
C-PLACE1001602//CCR4-ASSOCIATED FACTOR 1 (CAF1).//5.70E-130//244aa//99%//Q60809
C-PLACE1001632//ZINC FINGER PROTEIN 84 (ZINC FINGER PROTEIN HPF2).//1.40E-118//429aa//48%//P51523
C-PLACE1001672//PROBABLE AMINOTRANSFERASE T01B11.2 (EC 2.6.1.-).//4.30E-66//174aa//45%//P91408
C-PLACE1001692//S-ACYL FATTY ACID SYNTHASE THIOESTERASE, MEDIUM CHAIN (EC 3.1.2.14) (THIOESTERASE II).//4.00E-81//263aa//56%//P08635
C-PLACE1001739//PUTATIVE ATP-DEPENDENT RNA HEUCASE PL10.//3.50E-75//439aa//41%//P16381
C-PLACE1001748//Homo sapiens metalloprotease 1 (MP1) mRNA, complete cds.//0//2602bp//99%//AF061243
C-PLACE1001771//Homo sapiens mRNA for transient receptor potential protein TRP6.//0//2900bp//99%//AJ006276
C-PLACE1001781//PROBABLE PHOSPHOMANNOMUTASE (EC 5.4.2.8) (PMM).//5.40E-63//427aa//35%//Q57290
C-PLACE1001817//Homo sapiens ATP-specific succinyl-CoA synthetase beta subunit (SCS) mRNA, partial cds.//0//1995bp//99%//AF058953
C-PLACE1001845//Mus musculus cyclin ania-6a mRNA, complete cds.//3.30E-31//925bp//62%//AF159159
C-PLACE1001869//L-RIBULOKINASE (EC 2.7.1.16).//2.00E-27//270aa//31%//P94524
C-PLACE1001920//Homo sapiens MDC-3.13 isoform 2 mRNA, complete cds.//0//1729bp//99%//AF099935
C-PLACE1001983//HYPOTHETICAL 46.4 KD PROTEIN IN FFH-GRPE INTERGENIC REGION.//7.50E-16//319aa//26%//P37908
C-PLACE1001989//PUTATIVE AMIDASE (EC 3.5.1.4).//1.40E-78//496aa//37%//Q49091
C-PLACE1002046//UGATIN (FRAGMENT).//1.70E-240//560aa//80%//Q61211
C-PLACE1002073//ADENYLATE CYCLASE (EC 4.6.1.1) (ATP PYROPHOSPHATE-LYASE) (ADENYLYL CYCLASE).//5.30E-07//188aa//29%//P49606
C-PLACE1002090//SIGNAL RECOGNITION PARTICLE 72 KD PROTEIN (SRP72).//6.50E-58//112aa//100%//O76094
C-PLACE1002140//Rattus norvegicus apelin mRNA, complete cds.//1.40E-43//425bp//74%//AF179679
C-PLACE1002171//TRANSCRIPTION REGULATORY PROTEIN SWI3 (SWI/SNF COMPLEX COMPONENT SWI3) (TRANSCRIPTION FACTOR TYE2).//0.00005//179aa//23%//P32591
C-PLACE1002395//Mus musculus mRNA for UBE-1c1, UBE-1c2, UBE-1c3, complete cds.//7.90E-100//966bp//75%//AB030505
C-PLACE1002433//CHROMOSOME ASSEMBLY PROTEIN XCAP-E.//5.10E-05//278aa//24%//P50533
C-PLACE1002437//ATP-BINDING CASSETTE TRANSPORTER 1.//4.50E-76//180aa//83%//P41233
C-PLACE1002438//ZINC FINGER PROTEIN 151 (MIZ-1 PROTEIN).//4.20E-06//133aa//29%//Q13105
C-PLACE1002450//Human zinc finger protein mRNA, complete cds.//0//2565bp//99%//U69274
C-PLACE1002474//Mus musculus matrilin-2 precursor mRNA, complete cds.//0//2092bp//84%//U69262
C-PLACE1002493//Homo sapiens signal transducing adaptor molecule 2A (STAM2) mRNA, complete cds.//1.70E-113//545bp//98%//AF042273
C-PLACE1002500//Rattus norvegicus zinc transporter (ZnT-2) mRNA, complete cds.//2.90E-58//465bp//80%//U50927
C-PLACE1002532//HOMEOBOX PROTEIN DLX-5.//1.20E-152//289aa//96%//P70396
C-PLACE1002571//ACTIN-LIKE PROTEIN 13E.//5.00E-99//386aa//48%//P45890
C-PLACE1002583//GLUTAMATE RECEPTOR, IONOTROPIC KAINATE 2 PRECURSOR (GLUTAMATE RECEPTOR 6) (GLUR-6) (GLUTAMATE RECEPTOR BETA-2) (GLUR BETA-2) (FRAGMENT).//5.60E-34//76aa//98%//P39087
C-PLACE1002591//CORONIN-UKE PROTEIN P57.//4.40E-70//208aa//66%//P31146
C-PLACE1002598//OLIGORIBONUCLEASE (EC 3.1.-.-).//5.50E-17//76aa//56%//P45340
C-PLACE1002655//ADSEVERIN (SCINDERIN) (SC).//2.50E-278//543aa//92%//Q28046
C-PLACE1002665//Mus musculus enhancer of polycomb (Epc1) mRNA, complete cds.//0//2462bp//89%//AF079765
C-PLACE1002685//Homo sapiens B cell linker protein BLNK mRNA, alternatively spliced, complete cds.//0//1750bp//99%//AF068180
C-PLACE1002714//MYOSIN HEAVY CHAIN, NON-MUSCLE (ZIPPER PROTEIN) (MYOSIN II).//9.40E-13//500aa//21%//Q99323
C-PLACE1002722//PROBABLE G PROTEIN-COUPLED RECEPTOR KIAA0001.//9.00E-45//305aa//33%//Q15391
C-PLACE1002775//PEREGRIN (BR140 PROTEIN).//3.80E-13//272aa//28%//P55201
C-PLACE1002782//Rattus norvegicus zinc transporter (ZnT-2) mRNA, complete cds.//3.80E-43//385bp//77%//U50927
C-PLACE1002816//HISTONE DEACETYLASE HDA1.//2.20E-48//217aa//46%//P53973
C-PLACE1002834//ZINC FINGER PROTEIN 83 (ZINC FINGER PROTEIN HPF1).//5.50E-203//396aa//86%//P51522
C-PLACE1002908//Homo sapiens XGalT-1 mRNA for galactosyltransferase I, complete cds.//0//1654bp//99%//AB028600
C-PLACE1002991//PUTATIVE AMIDASE (EC 3.5.1.4).//1.40E-78//496aa//37%//Q49091
C-PLACE1003030//Homo sapiens snRNA activating protein complex 190kD subunit (SNAP190) mRNA, complete cds.//8.50E-44//225bp//100%//AF032387
C-PLACE1003045//POLYCYSTIN 2 (AUTOSOMAL DOMINANT POLYCYSTIC KIDNEY DISEASE TYPE II PROTEIN) (POLYCYSTWIN) (R48321).//1.70E-05//150aa//24%//Q13563
C-PLACE1003100//HEP27 PROTEIN (PROTEIN D).//2.60E-79//253aa//60%//Q13268
C-PLACE1003174//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//3.80E-37//143aa//51%//P42743
C-PLACE1003176//Homo sapiens clone pHN1868 tyrosyl-DNA phosphodiesterase protein (TDP1) mRNA, partial cds.//1.70E-148//687bp//99%//AF182003
C-PLACE1003190//SOF1 PROTEIN.//1.90E-110//325aa//48%//P33750
C-PLACE1003238//PROBABLE G PROTEIN-COUPLED RECEPTOR KIAA0001.//4.90E-76//309aa//47%//Q15391
C-PLACE1003258//EARLY EMBRYOGENESIS ZYG-11 PROTEIN.//7.90E-22//70aa//47%//P21541
C-PLACE1003302//ZINC FINGER PROTEIN 83 (ZINC FINGER PROTEIN HPF1).//6.90E-206//396aa//86%//P51522
C-PLACE10033537/Homo sapiens breast cancer antiestrogen resistance 3 protein (BCAR3) mRNA, complete cds.//0//2435bp//99%//U92715
C-PLACE1003366//Homo sapiens otoferlin (OTOF) mRNA, complete cds.//1.40E-78//542bp//67%//AF107403
C-PLACE1003394//Homo sapiens RAB14 protein (RAB14) mRNA, complete cds.//2.60E-139//648bp//99%//AF152463
C-PLACE1003420//PUTATIVE MITOCHONDRIAL CARRIER YIL006W.//1.30E-40//278aa//36%//P40556
C-PLACE1003493//ENDOTHELIAL CELL MULTIMERIN PRECURSOR.//1.70E-23//322aa//26%//Q13201
C-PLACE1003519//H.sapiens hnRNP-E2 mRNA.//5.10E-218//905bp//99%//X78136
C-PLACE1003521//HYPOTHETICAL HELICASE C28H8.3 IN CHROMOSOME III.//0.0000011//101aa//32%//Q09475
C-PLACE1003537//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//7.70E-68//404aa//33%//P32802
C-PLACE1003596//OLIGOSACCHARYL TRANSFERASE STT3 SUBUNIT HOMOLOG.//2.60E-93//270aa//66%//P46975
C-PLACE1003602//Homo sapiens mRNA expressed in placenta.//5.90E-278//1275bp//99%//D83200
C-PLACE1003605//HAP5 TRANSCRIPTIONAL ACTIVATOR.//0.00000023//82aa//35%//Q02516
C-PLACE1003611//Homo sapiens anaphase-promoting complex subunit 4 (APC4) mRNA, complete cds.//6.20E-169//683bp//99%//AF191338
C-PLACE1003625//ARMADILLO SEGMENT POLARITY PROTEIN.//3.20E-10//380aa//25%//P18824
C-PLACE1003669//TRICHOHYALIN.//5.60E-09//219aa//30%//P22793
C-PLACE1003704//SPLICING FACTOR, ARGININE/SERINE-RICH 4 (PRE-MRNA SPLICING FACTOR SRP75).//8.00E-19//209aa//34%//Q08170
C-PLACE1003709//Homo sapiens mitotic checkpoint kinase Bub1 (BUB1) mRNA complete cds.//6.20E-282//1316bp//98%//AF053305
C-PLACE1003738//ZINC FINGER PROTEIN 135.//9.60E-118//350aa//46%//P52742
C-PLACE1003760//Homo sapiens tetraspanin TM4-A mRNA, complete cds.//5.20E-289//1313bp//97%//AF133423
C-PLACE1003885//POLY(A) POLYMERASE (EC 2.7.7.19) (PAP) (POLYNUCLEOTIDE ADENYLYLTRANSFERASE).//3.70E-222//651aa//66%//P25500
C-PLACE1003888//1-PHOSPHATIDYLINOSITOL-4,5-BISPHOSPHATE PHOSPHODIESTERASE DELTA 1 (EC 3.1.4.11) (PLC-DELTA-1) (PHOSPHOLIPASE C-DELTA-1) (PLC-III) (FRAGMENT).//6.70E-113//501aa//46%//P10895
C-PLACE1003903//CTP SYNTHASE (EC 6.3.4.2) (UTP--AMMONIA LIGASE) (CTP SYNTHETASE).//1.40E-243//584aa//74%//P17812
C-PLACE1003915//PROBABLE ARGINYL-TRNA SYNTHETASE, CYTOPLASMIC (EC 6.1.1.19) (ARGININE- -TRNA UGASE) (ARGRS).//2.40E-108//581aa//40%//Q05506
C-PLACE1003923//Homo sapiens p53 regulated PA26-T2 nuclear protein (PA26) mRNA, complete cds.//0//1670bp//99%//AF033120
C-PLACE1003968//5'-AMP-ACTIVATED PROTEIN KINASE, GAMMA-1 SUBUNIT (AMPK GAMMA-1 CHAIN).//2.40E-124//326aa//73%//P80385
C-PLACE1004104//Rattus norvegicus rsec5 mRNA, complete cds.//0//2384bp//86%//AF032666
C-PLACE1004128//GUANINE NUCLEOTIDE-BINDING PROTEIN BETA SUBUNIT 4 (TRANSDUCIN BETA CHAIN 4).//6.10E-181//340aa//96%//P29387
C-PLACE1004149//Rattus norvegicus GERp95 mRNA, complete cds.//3.30E-41//452bp//65%//AF195534
C-PLACE1004183//Homo sapiens for TOM1-like protein.//0//1279bp//97%//AJ010071
C-PLACE1004197//BUTYROPHILIN PRECURSOR (BT).//4.50E-10//208aa//27%//Q62556
C-PLACE1004203//Homo sapiens GPI-anchored membrane protein CDw108 precursor, mRNA, complete cds.//0//1882bp//99%//AF069493
C-PLACE1004256//Mus musculus short coiled coil protein SCOCO (Scoc) mRNA, complete cds.//2.00E-93//960bp//76%//AF115778
C-PLACE1004258//Homo sapiens vanilloid receptor-like protein 1 (VRL-1) mRNA//0//1144bp//98%//AF129112
C-PLACE1004270//TRANSMEMBRANE PROTEASE, SERINE 2 (EC 3.4.21.-).//9.70E-36//389aa//31%//O15393
C-PLACE1004277//Homo sapiens two pore domain K⁺ channel (TASK-2) mRNA, complete cds.//0//1498bp//99%//AF084830
C-PLACE1004302//SOF1 PROTEIN.//1.90E-110//325aa//48%//P33750
C-PLACE1004316//H.sapiens mRNA for apoptosis specific protein.//0//1767bp//99%//Y11588
C-PLACE1004358//Homo sapiens connector enhancer of KSR-like protein CNK1 mRNA, complete cds.//0//2512bp//99%//AF100153
C-PLACE1004428//PRISTANOYL-COA OXIDASE (EC 1.3.3.-).//1.20E-39//385aa//33%//Q63448
C-PLACE1004437//Human NAD⁺-specific isocitrate dehydrogenase beta subunit precursor, mRNA, nuclear gene encoding mitochondrial protein, complete cds.//0//985bp//99%//U49283
C-PLACE1004460//MATERNAL TUDOR PROTEIN.//0.0000002//218aa//23%//P25823
C-PLACE1004471//ZINC FINGER PROTEIN 83 (ZINC FINGER PROTEIN HPF1)7/2.90E-56//276aa//41%//P51522
C-PLACE1004506//Homo sapiens carboxyl terminal LIM domain protein (CLIM1) mRNA, complete cds.//2.10E-16//402bp//62%//U90878
C-PLACE1004510//Homo sapiens TATA binding protein associated factor (TAFII150) mRNA, complete cds.//3.40E-227//1037bp//99%//AF040701
C-PLACE1004550//Homo sapiens CGI-20 protein mRNA, complete cds.//3.50E-274//1305bp//97%//AF132954
C-PLACE1004564//CLEAVAGE AND POLYADENYLATION SPECIFICITY FACTOR, 100 KD SUBUNIT (CPSF 100 KD SUBUNIT).//0//525aa//99%//Q10568
C-PLACE1004629//PROTEIN OS-9 PRECURSOR.//7.70E-18//264aa//32%//Q13438
C-PLACE1004646//B.taurus mRNA for retinal pigment epithelial membrane receptor p63.//4.40E-42//985bp//59%//X66277
C-PLACE1004674//Homo sapiens calcium binding protein (ALG-2) mRNA, complete cds.//1.30E-195//982bp//96%//AF035606
C-PLACE1004743//PROBABLE N-END-RECOGNIZING PROTEIN (UBIQUITIN-PROTEIN LIGASE E3 COMPONENT) (N- RECOGNIN).//4.40E-35//578aa//27%//O60152
C-PLACE1004751//Homo sapiens mRNA for alpha2,3-sialyltransferase ST3Gal VI, complete cds.//7.10E-224//790bp//98%//AB022918
C-PLACE1004777//N-CHIMAERIN (NC) (N-CHIMERIN) (ALPHA CHIMERIN) (A-CHIMAERIN).//1.90E-32//259aa//32%//P30337
C-PLACE1004793//RETROVIRUS-RELATED ENV POLYPROTEIN.//5.20E-47//577aa//25%//P10267
C-PLACE1004804//ADENYLATE CYCLASE (EC 4.6.1.1) (ATP PYROPHOSPHATE-LYASE) (ADENYLYL CYCLASE).//4.70E-65//695aa//29%//Q01631
C-PLACE1004814//SPLICING FACTOR, ARGININE/SERINE-RICH 4 (PRE-MRNA SPLICING FACTOR SRP75).//5.90E-19//196aa//36%//Q08170
C-PLACE1004868//MALE STERILITY PROTEIN 27/3.90E-39//261aa//27%//Q08891
C-PLACE1004902//PUTATIVE PRE-MRNA SPLICING FACTOR ATP-DEPENDENT RNA HELICASE SPAC10F6.02C.//9.30E-11//94aa//47%//O42643
C-PLACE1004918//L-LACTATE DEHYDROGENASE M CHAIN (EC 1.1.1.27) (LDH-A).//4.90E-48//198aa//44%//P06151
C-PLACE1004930//Homo sapiens MDC-3.13 isoform 2 mRNA, complete cds//0//1853bp//98%//AF099936
C-PLACE1004937//SEL-10 PROTEIN.//6.30E-125//357aa//58%//Q93794
C-PLACE1004969//HYPOTHETICAL 55.1 KD PROTEIN B0416.5 IN CHROMOSOME X.//2.00E-14//205aa//26%//Q11073
C-PLACE1005052//Homo sapiens CGI-16 protein mRNA, complete cds.//6.6e-313//1413bp//99%//AF132950
C-PLACE1005102//RING CANAL PROTEIN (KELCH PROTEIN).//2.60E-56//565aa//30%//Q04652
C-PLACE1005176//Homo sapiens hypothalamus protein HT001 mRNA, complete cds.//3.90E-212//1040bp//96%//AF113539
C-PLACE1005187//APAG PROTEIN.//3.80E-13//122aa//36%//P05636
C-PLACE1005243//SERINE/THREONINE PROTEIN KINASE PKPA (EC 2.7.1.-).//1.30E-27//349aa//32%//Q01577
C-PLACE1005287//INNER CENTROMERE PROTEIN (INCENP).//2.30E-13//269aa//28%//P53352
C-PLACE1005305//GTP:AMP PHOSPHOTRANSFERASE MITOCHONDRIAL (EC 2.7.4.10) (AK3).//2.00E-111//226aa//92%//P08760
C-PLACE1005331//Homo sapiens 7h3 protein mRNA, partial cds.//1.20E-226//748bp//95%//AF209931
C-PLACE1005373//TRNA PSEUDOURIDINE SYNTHASE B (EC 4.2.1.70) (TRNA PSEUDOURIDINE 55 SYNTHASE) (PSI55 SYNTHASE) (PSEUDOURIDYLATE SYNTHASE) (URACIL HYDROLYASE).//8.60E-09//194aa//27%//O33335
C-PLACE1005467//PENICILLIN-BINDING PROTEIN 4* (PBP 4*) (PBP 4A).//1.10E-09//93aa//31%//P32959
C-PLACE1005494//Homo sapiens mRNA for transient receptor potential protein TRP6.//0//1649bp//99%//AJ006276
C-PLACE1005530//HYPOTHETICAL 47.6 KD PROTEIN C16C10.5 IN CHROMOSOME III.//5.60E-52//173aa//57%//Q09251
C-PLACE1005549//Homo sapiens mRNA for Rho guanine nucleotide-exchange factor, splice variant NET1A.//7.60E-97//1287bp//67%//AJ010046
C-PLACE1005557//60S RIBOSOMAL PROTEIN L27.//1.90E-11//60aa//48%//P46288
C-PLACE1005584//TRANS-ACTING TRANSCRIPTIONAL PROTEIN ICPQ (P135 PROTEIN) (IER 2.9/ER2.6).//6.80E-09//267aa//30%//P29128
C-PLACE1005611//Mus musculus mRNA for mDj10, complete cds.//2.00E-33//379bp//66%//AB028860
C-PLACE1005646//Homo sapiens RNA helicase-related protein mRNA, complete cds.//0//2130bp//99%//AF083255
C-PLACE1005656//RIBONUCLEOSIDE-DIPHOSPHATE REDUCTASE M2 CHAIN (EC 1.17.4.1) (RIBONUCLEOTIDE REDUCTASE).//2.10E-148//321aa//83%//P31350
C-PLACE10057277/Homo sapiens STRIN protein (STRIN) mRNA, complete cds.//2.00E-118//378bp//98%//AF162680
C-PLACE1005739//INTERFERON-GAMMA INDUCIBLE PROTEIN MG11///1.30E-237//585aa//72%//Q60710
C-PLACE1005763//S-ACYL FATTY ACID SYNTHASE THIOESTERASE, MEDIUM CHAIN (EC 3.1.2.14) (THIOESTERASE II).//2.50E-79//209aa//53%//P08635
C-PLACE1005803//Homo sapiens mRNA for transcription factor (SMIF gene).//0//1985bp//99%//AJ275986
C-PLACE1005804//Homo sapiens alpha 1,2-mannosidase IB mRNA, complete cds.//1.10E-217//994bp//99%//AF027156
C-PLACE1005813//Homo sapiens sorting nexin 2 (SNX2) mRNA, complete cds.//0//2040bp//99%//AF065482
C-PLACE1005876//CLEAVAGE AND POLYADENYLATION SPECIFICITY FACTOR, 100 KD SUBUNIT (CPSF 100 KD SUBUNIT).//0//730aa//99%//Q10568
C-PLACE1005890//BEM46 PROTEIN (FRAGMENT).//9.90E-42//224aa//43%//P54069
C-PLACE1005921//AIG1 PROTEIN.//3.00E-31//284aa//31%//P54120
C-PLACE1005951//Homo sapiens prolactin regulatory element-binding protein (PREB) mRNA, complete cds.//1.10E-264//661bp//99%//AF203687
C-PLACE1005953//GLYCOSYLTRANSFERASE ALG2 (EC 2.4.1.-).//6.70E-30//198aa//37%//P43636
C-PLACE1005955/VACUOLAR AMINOPEPTIDASE I PRECURSOR (EC 3.4.11.22) (POLYPEPTIDASE)//5.40E-54//455aa//32%//P14904
C-PLACE1005966//TRANSCRIPTION INITIATION FACTOR TFIID 90 KD SUBUNIT (TAFII-90)7/1.40E-07//254aa//25%//P38129
C-PLACE1006003//Homo sapiens CGI-94 protein mRNA, complete cds.//2.40E-177//829bp//99%//AF151852
C-PLACE1006011//Homo sapiens mRNA for poly(ADP-ribose) polymerase-2.//0//1564bp//99%//AJ236876
C-PLACE1006040//Homo sapiens mRNA for alpha endosulfine.//4.70E-161//744bp//99%//X99906
C-PLACE1006119//Homo sapiens Ran-GTP binding protein mRNA, partial cds.//1.50E-148//681bp//99%//AF039023
C-PLACE1006157//E-SELECTIN PRECURSOR (ENDOTHELIAL LEUKOCYTE ADHESION MOLECULE 1) (ELAM-1) (LEUKOCYTE-ENDOTHELIAL CELL ADHESION MOLECULE 2) (LECAM2) (CD62E).//2.00E-28//236aa//30%//P98110
C-PLACE1006167//PAF1 PROTEIN.//7.30E-15//437aa//24%//P38351
C-PLACE1006170//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT).//1.70E-169//373aa//88%//P17427
C-PLACE1006196//PUTATIVE ATP-DEPENDENT RNA HELICASE C12C2.06.//2.70E-116//496aa//48%//Q09747
C-PLACE1006239//BONE PROTEOGLYCAN II PRECURSOR (PG-S2) (DECORIN).//2.00E-16//244aa//31%//P28675
C-PLACE1006288//VOLTAGE-DEPENDENT ANION-SELECTIVE CHANNEL PROTEIN 1 (VDAC1) (PLASMALEMMAL PORIN) (OUTER MITOCHONDRIAL MEMBRANE PROTEIN PORIN) (PORIN 31HL) (PORIN 31HM).//4.60E-117//147aa//80%//P21796
C-PLACE1006318//Mus musculus skm-BOP2 (Bop) mRNA, complete cds.//3.00E-07//376bp//59%//U76374
C-PLACE1006335//Homo sapiens NY-REN-50 antigen mRNA, partial cds.//0//1649bp//99%//AF155112
C-PLACE1006368//HYALURONAN-MEDIATED MOTILITY RECEPTOR (HYALURONIC ACID RECEPTOR).//1.30E-18//460aa//24%//Q00547
C-PLACE1006385//Homo sapiens epsin 2a mRNA, complete cds.//0//1168bp//99%//AF062085
C-PLACE1006438//ZINC FINGER PROTEIN 165.//2.50E-45//122aa//43%//P49910
C-PLACE1006469//ACETYL-COENZYME A SYNTHETASE (EC 6.2.1.1) (ACETATE--COA LIGASE) (ACYL- ACTIVATING ENZYME).//1.20E-83//313aa//49%//P27550
C-PLACE1006482//TRANSCRIPTION FACTOR MAFF.//7.70E-55//142aa//85%//Q90595
C-PLACE1006488//SIGNAL RECOGNITION PARTICLE 68 KD PROTEIN (SRP68).//1.10E-229//367aa//96%//Q00004
C-PLACE1006492//Homo sapiens transmembrane protein 2 (TMEM2) mRNA, complete cds.//0//2618bp//99%//AF137030
C-PLACE1006506//Homo sapiens anaphase-promoting complex subunit 4 (APC4) mRNA, complete cds.//0//2170bp//99%//AF191338
C-PLACE1006531//Homo sapiens putative RNA-binding protein Q99 mRNA, complete cds.//0//1967bp//99%//AF093097
C-PLACE1006534//POLYPEPTIDE N-ACETYLGALACTOSAMINYLTRANSFERASE (EC 2.4.1.41)(PROTEIN-UDP ACETYLGALACTOSAMINYLTRANSFERASE)(UDP-GALNAC:POLYPEPTIDE, N-ACETYLGALACTOSAMINYLTRANSFERASE) (GALNAC-T1).//8.30E-08//100aa//41%//Q10472
C-PLACE1006552//MYOSIN HEAVY CHAIN, CLONE 203 (FRAGMENT).//1.20E-09//426aa//21%//P39922
C-PLACE1006615//Homo sapiens eukaryotic translation initiation factor eIF3, p35 subunit mRNA, complete cds.//0//1464bp//99%//U97670
C-PLACE1006626//Homo sapiens mRNA for Helicase-MOI, complete cds.//0//1760bp//99%//AB028449
C-PLACE1006678//Homo sapiens mRNA for type II membrane protein, complete cds, clone:HP10328.//5.80E-24//734bp//62%//AB015630
C-PLACE1006731//RIBOFLAVIN KINASE (EC 2.7.1.26) (FLAVOKINASE) / FMN ADENYLYLTRANSFERASE (EC 2.7.7.2) (FAD PYROPHOSPHORYLASE) (FAD SYNTHETASE).//6.90E-13//177aa//33%//Q59263
C-PLACE1006754//BILIARY GLYCOPROTEIN 1 PRECURSOR (BGP-1) (ANTIGEN CD66) (CD66A ANTIGEN).//6.20E-63//191aa//43%//P13688
C-PLACE1006819//UNE-1 REVERSE TRANSCRIPTASE HOMOLOG.//9.80E-213//232aa//80%//P08547
C-PLACE1006829//UBIQUITIN CARBOXYL-TERMINAL HYDROLASE 4 (EC 3.1.2.15) (UBIQUITIN THIOLESTERASE 4) (UBIQUITIN-SPECIFIC PROCESSING PROTEASE 4) (DEUBIQUITINATING ENZYME 4) (UBIQUITOUS NUCLEAR PROTEIN).//2.00E-15//188aa//29%//P35123
C-PLACE1006878//TRNA-SPLICING ENDONUCLEASE SUBUNIT SEN2 (EC 3.1.27.9) (TRNA-INTRON ENDONUCLEASE).//1.90E-08//122aa//36%//P16658
C-PLACE1006917//HSH49 PROTEIN.//5.50E-12//97aa//35%//Q99181
C-PLACE1006935//HYPOTHETICAL 95.2 KD PROTEIN R144.6 IN CHROMOSOME III.//6.70E-48//278aa//41%//Q10000
C-PLACE1006956//ATP-DEPENDENT PERMEASE MDL1.//1.30E-86//522aa//36%//P97998
C-PLACE1006958//Homo sapiens mRNA for heat shock protein apg-1, complete cds.//0//1770bp//99%//AB023421
C-PLACE1007014//36 KD NUCLEOLAR PROTEIN HNP36 (DELAYED-EARLY RESPONSE PROTEIN 12) (DER12).//3.20E-35//180aa//33%//Q14542
C-PLACE1007105//Homo sapiens muskelin (MKLN1) mRNA, complete cds.//0//2449bp//98%//AF047489
C-PLACE1007140//TRICHOHYALIN.//1.30E-25//816aa//22%//P37709
C-PLACE1007226//PROBABLE OXYGEN-INDEPENDENT COPROPORPHYRINOGEN III OXIDASE (EC 1.-.-.-) (COPROPORPHYRINOGENASE) (COPROGEN OXIDASE).//1.00E-42//370aa//31%//P54304
C-PLACE1007239//Homo sapiens mRNA for transcription elongation factor S-II, hS-II-T1, complete cds.//6.50E-216//1068bp//96%//D50495
C-PLACE1007243//UNC-47 PROTEIN.//1.70E-07//211aa//27%//P34579
C-PLACE1007257//Homo sapiens mRNA for dia-12c protein.//0//2052bp//99%//Y15908
C-PLACE1007317//Drosophila melanogaster Adrift (adrift) mRNA, complete cds.//4.10E-17//1037bp//56%//AF117649
C-PLACE1007346//Homo sapiens estrogen-responsive B box protein (EBBP) mRNA, complete cds.//0//2366bp//99%//AF096870
C-PLACE1007375//PHORBOL ESTER/DIACYLGLYCEROL-BINDING PROTEIN UNC-13.//0.00000044//127aa//30%//P27715
C-PLACE1007409//WHTTE PROTEIN.//1.10E-64//428aa//32%//Q17320
C-PLACE1007416//DIPEPTIDYL PEPTIDASE IV (EC 3.4.14.5) (DPP IV) (T-CELL ACTIVATION ANTIGEN CD26) (TP103) (ADENOSINE DEAMINASE COMPLEXING PROTEIN-2) (ADABP).//8.80E-25//140aa//35%//P27487
C-PLACE1007488//PUTATIVE RHO/RAC GUANINE NUCLEOTIDE EXCHANGE FACTOR (RHO/RAC GEF) (FACIOGENITAL DYSPLASIA PROTEIN HOMOLOG).//5.40E-53//426aa//33%//P52734
C-PLACE1007511//KERATIN, TYPE I CYTOSKELETAL 19 (CYTOKERATIN 19) (K19) (CK 19).//1.40E-85//385aa//45%//P08728
C-PLACE1007537//Homo sapiens ankyrin repeat-containing protein ASB-2 mRNA, complete cds.//8.9e-316//1485bp//98%//AF159164
C-PLACE1007547//HYPOTHETICAL 97.1 KD PROTEIN R05D3.4 IN CHROMOSOME III.//1.00E-49//361aa//36%//P34537
C-PLACE1007598//ZINC FINGER PROTEIN 184 (FRAGMENT).//1.60E-143//666aa//44%//Q99676
C-PLACE1007632//POLIOVIRUS RECEPTOR PRECURSOR.//1.00E-07//228aa//31%//P32506
C-PLACE1007649//GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).//4.50E-05//197aa//26%//P08640
C-PLACE1007688//LA PROTEIN HOMOLOG (LA RIBONUCLEOPROTEIN) (LA AUTOANTIGEN HOMOLOG).//8.70E-09//279aa//28%//Q26457
C-PLACE1007697//GCN20 PROTEIN.//7.60E-119//717aa//38%//P43535
C-PLACE1007705//Mus musculus mRNA for Ndr1 related protein Ndr3, complete cds.//1.10E-184//1096bp//82%//AB033922
C-PLACE1007706//Homo sapiens metalloprotease 1 (MP1) mRNA, complete cds.//0//3431bp//99%//AF061243
C-PLACE1007729//RETROVIRUS-RELATED PROTEASE (EC 3.4.23.-).//1.50E-44//231aa//42%//P10265
C-PLACE1007791//Homo sapiens IDN3-B mRNA, complete cds.//0//1836bp//99%//AB019602
C-PLACE1007897//Homo sapiens FLASH mRNA, complete cds.//0//2145bp//99%//AF154415
C-PLACE1007946//MYOSIN HEAVY CHAIN, NON-MUSCLE (ZIPPER PROTEIN) (MYOSIN II).//2.60E-14//370aa//25%//Q99323
C-PLACE1007954//HYPOTHETICAL 45.5 KD PROTEIN IN FIG1-GIP1 INTERGENIC REGION.//6.70E-13//168aa//31%//P38226
C-PLACE1007955//Homo sapiens cyclin-D binding Myb-like protein mRNA, complete cds.//0//2252bp//99%//AF084530
C-PLACE1007958//Homo sapiens cAMP-specific phosphodiesterase 8B (PDE8B) mRNA, partial cds.//0//2300bp//99%//AF079529
C-PLACE1007969//HETEROGENEOUS NUCLEAR RIBONUCLEOPROTEIN M (HNRNP M).//1.10E-36//202aa//48%//P52272
C-PLACE1008000//CHANNEL ASSOCIATED PROTEIN OF SYNAPSE-110 (CHAPSYN-110) (SYNAPTIC DENSITY PROTEIN PSD-93).//6.10E-14//128aa//39%//Q63622
C-PLACE1008044//NUCLEAR PORE COMPLEX PROTEIN NUP107 (NUCLEOPORIN NUP107) (107 KD NUCLEOPORIN) (P105).//4.6e-318//613aa//94%//P52590
C-PLACE1008080//Homo sapiens mRNA for HEXIM1 protein, complete cds.//0//2152bp//99%//AB021179
C-PLACE1008111//PROBABLE OXIDOREDUCTASE (EC 1.-.-.-).//3.00E-25//208aa//37%//Q03326
C-PLACE1008132//HYPOTHETICAL 127.4 KD PROTEIN F07F6.4 IN CHROMOSOME III.//1.30E-24//395aa//31%//Q09531
C-PLACE1008177//TRICHOHYALIN.//2.30E-29//487aa//26%//P37709
C-PLACE1008201//Rattus rattus zinc finger protein, complete cds.//0//2265bp//83%//L23077
C-PLACE1008244//VEGETATIBLE INCOMPATIBILITY PROTEIN HET-E-1.//9.50E-21//148aa//38%//Q00808
C-PLACE1008273//COATOMER GAMMA SUBUNIT (GAMMA-COAT PROTEIN) (GAMMA-COP).//1.30E-283//671aa//77%//P53620
C-PLACE1008275//DNA REPAIR PROTEIN REV1 (EC 2.7.7.-).//2.30E-18//162aa//37%//P12689
C-PLACE1008309//Rattus norvegicus putative four repeat ion channel mRNA, complete cds.//5.20E-137//672bp//77%//AF078779
C-PLACE1008330//EOSINOPHIL LYSOPHOSPHOLIPASE (EC 3.1.1.5) (CHARCOT-LEYDEN CRYSTAL PROTEIN) (LYSOLECITHIN ACYLHYDROLASE) (CLC) (GALACTIN-10).//2.20E-23//94aa//47%//Q05315
C-PLACE1008356//Homo sapiens meningioma-expressed antigen 5 (MEA5) mRNA, partial cds.7/1.90E-170//780bp//100%//AF036144
C-PLACE1008368//RING CANAL PROTEIN (KELCH PROTEIN).//5.30E-26//309aa//30%//Q04652
C-PLACE1008398//GENE 33 POLYPEPTIDE.//7.30E-114//243aa//87%//P05432
C-PLACE1008402//GENERAL VESICULAR TRANSPORT FACTOR P115 (TRAN-SCYTOSIS ASSOCIATED PROTEIN) (TAP).//0//698aa//95%//P41541
C-PLACE1008426//RESTIN (CYTOPLASMIC LINKER PROTEIN-170) (CLIP-170).//1.80E-11//365aa//25%//O42184
C-PLACE1008429//ANKYRIN HOMOLOG PRECURSOR.//3.10E-11//189aa//32%//Q06527
C-PLACE1008465//Homo sapiens mRNA for rapa-1 (rapa gene).//6.60E-243//1102bp//99%//AJ277275
C-PLACE1008533//101 KD MALARIA ANTIGEN (P101) (ACIDIC BASIC REPEAT ANTIGEN).//1.10E-09//62aa//48%//P22620
C-PLACE1008603//NUCLEAR PORE COMPLEX PROTEIN NUP155 (NUCLEOPORIN NUP155) (155 KD NUCLEOPORIN) (P140).//7.80E-236//453aa//96%//P37199
C-PLACE1008627//Homo sapiens mRNA for cysteine-rich protein.//0//1850bp//99%//AJ006591
C-PLACE1008643//INTER-ALPHA-TRYPSIN INHIBITOR HEAVY CHAIN H2 PRECURSOR (III HEAVY CHAIN H2).//5.20E-90//483aa//38%//O02668
C-PLACE1008650//PRL1/PRL2-LIKE PROTEIN.//2.00E-127//354aa//62%//O13615
C-PLACE1008696//Homo sapiens NADH dehydrogenase-ubiquinone Fe-S protein 8 23 kDa subunit (NDUFS8) gene, nuclear gene encoding mitochondrial protein, complete cds.//0//3002bp//99%//AF03 8406
C-PLACE1008790//Homo sapiens importin alpha 7 subunit mRNA, complete cds.//0//1670bp//99%//AF060543
C-PLACE1008808//Homo sapiens mRNA for cell cycle checkpoint protein radlA.//2.30E-269//1225bp//99%//AJ004974
C-PLACE1008813//Rattus norvegicus rsec15 mRNA, complete cds.//8.80E-268//1171bp//90%//AF032668
C-PLACE1009020//NIFS PROTEIN.//3.90E-55//279aa//41%//P12623
C-PLACE1009027//Homo sapiens mRNA for doublecortin.//0//1919bp//99%//AJ003112
C-PLACE1009060//BRO1 PROTEIN.//6.70E-19//567aa//24%//P48582
C-PLACE1009094//FURIN-LIKE PROTEASE 2 PRECURSOR (EC 3.4.21.75) (FURIN 2).//1.90E-44//480aa//30%//P30432
C-PLACE1009099//ZINC FINGER PROTEIN 41 (FRAGMENT).//1.10E-179//452aa//67%//P51814
C-PLACE1009113//Homo sapiens X-ray repair cross-complementing protein 3 (XRCC3) mRNA, complete cds.//0//2529bp//99%//AF035586
C-PLACE1009130/UBIQUITIN-PROTEIN LIGASE E3A (EC 6.3.2.-) (ONCOGENIC PROTEIN-ASSOCIATED PROTEIN E6-AP).//2.00E-68//181aa//43%//Q05086
C-PLACE1009158//Mus musculus mRNA for death inducer-obliterator-1 (Dio-1).//5.40E-200//1790bp//75%//AJ238332
C-PLACE1009186//Homo sapiens small zinc finger-like protein (TIM9b) mRNA, complete cds.//9.60E-255//1179bp//98%//AF150105
C-PLACE1009246//POLLEN SPECIFIC PROTEIN SF3.//4.40E-16//82aa//43%//P29675
C-PLACE1009298//VACUOLAR PROTEIN SORTING-ASSOCIATED PROTEIN VPS35.//2.00E-78//262aa//43%//P34110
C-PLACE1009308//GLUCOSE REPRESSION MEDIATOR PROTEIN.//4.00E-06//439aa//23%//P14922
C-PLACE1009319//Rattus norvegicus outer membrane protein (OMP25) mRNA, complete cds; nuclear gene for mitochondrial product.//2.10E-132//1229bp//75%//AF107295
C-PLACE1009368//METAL HOMEOSTASIS FACTOR ATX27/2.50E-10//151aa//29%//Q12067
C-PLACE1009398//ZINC FINGER PROTEIN 135.//6.20E-97//361aa//51%//P52742
C-PLACE1009404//HYPOTHETICAL 105.6 KD PROTEIN C16C9.06C IN CHROMOSOME I.//4.70E-08//165aa//33%//Q09820
C-PLACE1009443//Mus musculus F-box protein FBL8 mRNA, complete cds.//1.00E-173//1367bp//77%//AF176523
C-PLACE1009444//PHOSPHATIDYLINOSITOL 4-KINASE ALPHA (EC 2.7.1.67) (PI4-KINASE) (PTDINS-4-KINASE) (PI4K-ALPHA).//7.80E-71//82aa//89%//P42356
C-PLACE1009468//PHOSPHOLIPASE A-2-ACTIVATING PROTEIN (PLAP).//3.10E-289//550aa//93%//P54319
C-PLACE1009476//PUTATIVE ATP-DEPENDENT RNA HELICASE T26G10.1 IN CHROMOSOME III.//3.90E-40//179aa//37%//P34580
C-PLACE1009477//Homo sapiens ubiquitin-fusion degradation protein 2 (UFD2) mRNA, complete cds.//6.60E-147//592bp//99%//AF043117
C-PLACE1009524//ARF NUCLEOTIDE-BINDING SITE OPENER (ARNO PROTEIN) (ARF EXCHANGE FACTOR)7/8.10E-99//228aa//75%//Q99418
C-PLACE1009571//Homo sapiens PTD002 mRNA, complete cds.//5.90E-185//857bp//99%//AF078857
C-PLACE1009596//VEGETATIBLE INCOMPATIBILITY PROTEIN HET-E-1.//5.10E-54//291aa//40%//Q00808
C-PLACE1009622//MATERNAL EFFECT PROTEIN STAUFEN.//1.30E-60//209aa//41%//P25159
C-PLACE1009659//MEMBRANE-ASSOCIATED PROTEIN HEM-2 (NAP1 PROTEIN).//1.50E-285//538aa//99%//P55161
C-PLACE1009670//Homo sapiens genethonin 1 mRNA, complete cds.//0//1854bp//100%//AF062534
C-PLACE1009708//HYPOTHETICAL 143.3 KD TRP-ASP REPEATS CONTAINING PROTEIN C12G12.13C IN CHROMOSOME I.//7.00E-33//166aa//43%//Q09876
C-PLACE1009721//MSF1 PROTEIN.//1.70E-22//176aa//33%//P35200
C-PLACE1009731//AIG1 PROTEIN.//1.60E-22//274aa//28%//P54120
C-PLACE1009763//Homo sapiens mRNA for Nedd8-activating enzyme hUba3, complete cds.//4.30E-294//1329bp//100%//AB012190
C-PLACE1009798//RLR1 PROTEIN.//1.60E-18//270aa//23%//P53552
C-PLACE1009845//WEB1 PROTEIN (PROTEIN TRANSPORT PROTEIN SEC31).//2.30E-59//405aa//33%//P38968
C-PLACE1009861//CATHEPSIN B-LIKE CYSTEINE PROTEINASE 6 PRECURSOR (EC 3.4.22.-).//6.50E-28//209aa//38%//P43510
C-PLACE1009908//HYPOTHETICAL GTP-BINDING PROTEIN IN SEH1-PRP20 INTERGENIC REGION.//1.90E-108//277aa//43%//P53145
C-PLACE1009925//Homo sapiens RNA helicase (RIG-I) mRNA, complete cds.//0//1730bp//99%//AF038963
C-PLACE1009992//LIMULUS CLOTTING FACTOR C PRECURSOR (EC 3.4.21.84).//4.60E-59//450aa//34%//P28175
C-PLACE1009997//Rattus norvegicus A-kinase anchoring protein AKAP 220 mRNA, complete cds.//5.20E-70//736bp//73 %//U48288
C-PLACE1010053//M.musculus Spnr mRNA for RNA binding protein.//6.00E-279//1402bp//94%//X84692
C-PLACE1010074//Homo sapiens sorting nexin 2 (SNX2) mRNA, complete cds.//0//2019bp//99%//AF065482
C-PLACE1010096//100 KD PROTEIN (EC 6.3.2.-).//1.40E-268//506aa//98%//Q62671
C-PLACE1010105//RING CANAL PROTEIN (KELCH PROTEIN).//7.30E-114//537aa//44%//Q04652
C-PLACE1010134//TRANSCRIPTION REGULATORY PROTEIN SNF2 (SWI/SNF COMPLEX COMPONENT SNF2) (REGULATORY PROTEIN SWI2) (REGULATORY PROTEIN GAM1) (TRANSCRIPTION FACTOR TYE3).//1.70E-20//156aa//42%//P22082
C-PLACE1010148//CYUCIN I (MULTIPLE-BAND POLYPEPTIDE I).//4.60E-07//431aa//23%//P35662
C-PLACE1010194//SPLICING FACTOR, ARGININE/SERINE-RICH 2 (SPLICING FACTOR SC35) (SC-35) (SPUCING COMPONENT, 35 KD) (PR264 PROTEIN).//9.80E-11//95aa//49%//Q01130
C-PLACE1010231//CELL SURFACE GLYCOPROTEIN EMR1 PRECURSOR (EMR1 HORMONE RECEPTOR).//5.1 OE-27//371aa//28%//Q14246
C-PLACE1010261//SEGREGATION DISTORTER PROTEIN.//1.60E-77//214aa//62%//P25722
C-PLACE1010310//SPIDROIN 2 (DRAGLINE SILK FIBROIN 2) (FRAGMENT).//1.20E-18//467aa//30%//P46804
C-PLACE1010321//NON-GREEN PLASTID TRIOSE PHOSPHATE TRANSLOCATOR PRECURSOR (CTPT).//1.10E-09//350aa//22%//P52178
C-PLACE1010362//1-PHOSPHATIDYLINOSITOL PHOSPHODIESTERASE PRECURSOR (EC 3.1.4.10) (PHOSPHATIDYLINOSITOL-SPECIFIC PHOSPHOLIPASE C) (PI-PLC).//2.00E-09//126aa//29%//P34024
C-PLACE1010481//Bos taurus C5-glucuronyl epimerase mRNA, partial cds//0//2082bp//91%//AF003927
C-PLACE1010522//Homo sapiens mRNA for DEPP (decidual protein induced by progesterone), complete cds.//0//1981 bp//99%//AB022718
C-PLACE1010529//Homo sapiens TANK binding kinase TBK1 (TBK1) mRNA, complete cds.//0//1750bp//99%//AF191838
C-PLACE1010547//INTRACELLULAR PROTEIN TRANSPORT PROTEIN USO1.//1.20E-07//616aa//24%//P25386
C-PLACE1010579//Homo sapiens CED-6 protein (CED-6) mRNA, complete cds.//8.80E-300//1359bp//99%//AF191771
C-PLACE1010599//Homo sapiens Pex14 mRNA for peroxisomal membrane anchor protein, complete cds.//0//1904bp//99%//AB017546
C-PLACE1010622//TROPONIN T, CARDIAC MUSCLE ISOFORMS (TNTC).//0.00000016//120aa//28%//P02642
C-PLACE1010628//Homo sapiens S164 gene, partial cds; PS1 and hypothetical protein genes, complete cds; and S171 gene, partial cds.//7.50E-08//324bp//64%//AF109907
C-PLACE1010661//TESTIS-SPECIFIC PROTEIN PBS 13.//5.70E-75//423aa//39%//Q01755
C-PLACE1010662//UDP-GLUCOSE:GLYCOPROTEIN GLUCOSYLTRANSFERASE PRECURSOR (EC 2.4.1.-) (DUGT).//1.80E-222//808aa//52%//Q09332
C-PLACE1010702//ZINC FINGER PROTEIN 43 (ZINC PROTEIN HTF6).//5.20E-151//427aa//55%//P28160
C-PLACE1010720//Homo sapiens mRNA for chromosome-associated polypeptide-C, complete cds.//4.00E-299//1091bp//99%//AB019987
C-PLACE1010743//Homo sapiens myosin-IXb splice variant (Myo9b) mRNA, partial cds.//8.90E-91//668bp//82%//AF020267
C-PLACE1010761//Homo sapiens mRNA for cisplatin resistance-associated overexpressed protein, complete cds.//0//1448bp//99%//AB034205
C-PLACE1010771//M.musculus HCNGP mRNA.//7.40E-168//966bp//89%//X68061
C-PLACE1010811//Rattus norvegicus mRNA for protein encoded by bdeight gene, partial.//1.60E-217//858bp//87%//AJ010392
C-PLACE1010833//CALTRACTIN(CENTRIN).//0.0000001//154aa//28%//P41209
C-PLACE1010870//ZINC FINGER PROTEIN 91 (ZINC FINGER PROTEIN HTF10) (HPF7).//1.30E-143//407aa//58%//Q05481
C-PLACE1010896//NUF1 PROTEIN (SPINDLE POLY BODY SPACER PROTEIN SPC110).//1.50E-25//583aa//23%//P35580
C-PLACE1010926//HYPOTHETICAL 72.2 KD PROTEIN C12C2.05C IN CHROMOSOME II.//7.60E-23//103aa//53%//Q09746
C-PLACE1010942//Homo sapiens intersectin long isoform (ITSN) mRNA, complete cds.//0//1440bp//99%//AF114487
C-PLACE1010960//ACTIN-LIKE PROTEIN 13E.//5.30E-98//297aa//48%//P45890
C-PLACE1011041//Homo sapiens mRNA for BAP2-alpha protein, complete cds.//0//1701bp//97%//AB015019
C-PLACE1011046//1-PHOSPHATIDYLINOSITOL-4,5-BISPHOSPHATE PHOSPHODIESTERASE BETA 1 (EC 3.1.4.11) (PLC-BETA-1) (PHOSPHOLIPASE C-BETA-1) (PLC-I) (PLC-154).//0//646aa//97%//P10894
C-PLACE1011056//HISTONE HI, GONADAL.//6.80E-13//154aa//37%//P02256
C-PLACE1011109//ELONGATION FACTOR G, MITOCHONDRIAL PRECURSOR (MEFG).//1.50E-22//63aa//88%//Q07803
C-PLACE1011114//PROBABLE ATP-DEPENDENT RNA HELICASE HAS1.//2.90E-71//190aa//44%//Q03532
C-PLACE1011160//Homo sapiens HFB30 mRNA, complete cds.//0//1691bp//99%//AB022663
C-PLACE1011185//INSERTION ELEMENT IS1 PROTEIN INSB.//1.30E-89//167aa//100%//P03830
C-PLACE1011219//PROBABLE OXIDOREDUCTASE (EC 1.-.-.-).//3.20E-12//212aa//29%//Q03326
C-PLACE1011229//Homo sapiens ubiquitin-specific protease homolog (UPH) mRNA, complete cds.//2.30E-152//701bp//99%//AF153604
C-PLACE1011310//MYOSIN HEAVY CHAIN, GIZZARD SMOOTH MUSCLE.//3.50E-20//496aa//25%//P10587
C-PLACE1011332//Homo sapiens N-acetylglucosamine-phosphate mutase mRNA, complete cds.//7.20E-151//697bp//99%//AF102265
C-PLACE1011340//Homo sapiens IDN3-B mRNA, complete cds.//1.20E-74//380bp//97%//AB019602
C-PLACE1011371//INTER-ALPHA-TRYPSIN INHIBITOR HEAVY CHAIN H2 PRECURSOR (ITI HEAVY CHAIN H2).//1.70E-78//383aa//39%//Q61703
C-PLACE1011399//Homo sapiens CGI-72 protein mRNA, complete cds.//3.20E-90//427bp//99%//AF151830
C-PLACE1011433//TRANSCRIPTION FACTOR IIIA (FACTOR A) (TFIIIA).//3.00E-10//236aa//25%//P34695
C-PLACE1011477//Homo sapiens sorting nexin 2 (SNX2) mRNA, complete cds.//0//2040bp//99%//AF065482
C-PLACE1011492//NON-GREEN PLASTID TRIOSE PHOSPHATE TRANSLOCATOR PRECURSOR (CTPT).//4.90E-11//147aa//32%//P52178
C-PLACE1011576//Human Kruppel related zinc finger protein (HTF10) mRNA, complete cds.//0//1791bp//82%//L11672
C-PLACE1011586//Rattus norvegicus clone C53 CDK5 activator-binding protein mRNA, complete cds7/4.10E-259//1538bp//87%//AF177476
C-PLACE1011635//Homo sapiens heparan sulfate D-glucosaminyl 3-O-sulfotransferase-3B (3OST3B1) mRNA, complete cds.//0//1559bp//99%//AF105377
C-PLACE1011664//CROOKED NECK PROTEIN.//1.60E-187//505aa//64%//P17886
C-PLACE1011858//Homo sapiens BAG-family molecular chaperone regulator-2 mRNA, complete cds.//1.30E-255//1179bp//99%//AF095192
C-PLACE1011896//Mus musculus Wnt10a mRNA, complete cds.//2.60E-287//1820bp//85%//U61969
C-PLACE1011922//MYOSIN HEAVY CHAIN, NONMUSCLE TYPE B (CELLULAR MYOSIN HEAVY CHAIN, TYPE B) (NMMHC-B).//1.30E-15//409aa//27%//P35580
C-PLACE1011923//Homo sapiens serum-inducible kinase mRNA, complete cds.//0//2782bp//99%//AF059617
C-PLACE101-2031//Homo sapiens sorting nexin 13 (SNX13) mRNA, partial cds.//0//1701bp//100%//AF121862
C-PLACE2000014//HYPOTHETICAL HELICASE C28H8.3 IN CHROMOSOME III.//2.60E-42//104aa//49%//Q09475
C-PLACE2000015//EPIDERMAL GROWTH FACTOR RECEPTOR SUBSTRATE SUBSTRATE 15 (PROTEIN EPS15) (AF-1P PROTEIN).//1.10E-116//364aa//45%//P42566
C-PLACE2000021//Homo sapiens TRF1-interacting ankyrin-related ADP-ribose polymerase mRNA, complete cds.//2.70E-107//981bp//74%//AF082556
C-PLACE2000034//LAR PROTEIN PRECURSOR (LEUKOCYTE ANTIGEN RELATED) (EC 3.1.3.48).//2.20E-29//212aa//35%//P10586
C-PLACE2000039//DYNEIN HEAVY CHAIN, CYTOSOLIC (DYHC) (MAP 1C).//6.10E-293//388aa//99%//P38650
C-PLACE2000062//Homo sapiens mRNA for type II membrane protein similar to HIV gp120-binding C-type lectin, complete cds, clone:HP01347.//6.30E-166//656bp//94%//AB015629
C-PLACE2000072//Homo sapiens ZNF202 beta (ZNF202) mRNA, complete cds.//0//3174bp//99%//AF027219
C-PLACE2000164//TIPD PROTEIN.//2.10E-59//481aa//33%//O15736
C-PLACE2000216//SPECTRIN BETA CHAIN, BRAIN (SPECTRIN, NON-ERYTHROID BETA CHAIN) (FODRIN BETA CHAIN) (SPTBN1).//6.60E-115//226aa//99%//Q01082
C-PLACE2000246//RING CANAL PROTEIN (KELCH PROTEIN).//6.00E-57//239aa//34%//Q04652
C-PLACE2000274//DYNEIN BETA CHAIN, CILIARY.//2.20E-167//880aa//37%//P23098
C-PLACE2000341//Homo sapiens sodium-dependent multivitamin transporter (SMVT) mRNA, complete cds.//0//1554bp//99%//AF069307
C-PLACE2000371/TENSIN.//2.90E-78//561aa//37%//Q04205
C-PLACE2000373//F-SPONDIN PRECURSOR.//8.60E-16//371aa//28%//P35446
C-PLACE2000398//LAR PROTEIN PRECURSOR (LEUKOCYTE ANTIGEN RELATED) (EC 3.1.3.48).//6.30E-37//90aa//98%//P10586
C-PLACE2000399//T-CELL SURFACE GLYCOPROTEIN E2 PRECURSOR (E2 ANTIGEN) (CD99) (MIC2 PROTEIN) (12E7).//1.60E-14//180aa//39%//P14209
C-PLACE2000404//PROBABLE LEUCYL-TRNA SYNTHETASE (EC 6.1.1.4) (LEUCINE--TRNA LIGASE) (LEURS).//9.90E-229//821aa//54%//Q09996
C-PLACE2000411//Homo sapiens epsin 2b mRNA, complete cds.//3.80E-271//642bp//99%//AF062085
C-PLACE2000427//PROBABLE HELICASE MOT1.//1.20E-26//200aa//27%//P32333
C-PLACE2000438//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N- ACETYLGALACTOSAMINYLTRANSFERASE) (GALNAC-T1).//2.10E-86//348aa//41%//Q10472
C-PLACE2000458//CADHERIN-RELATED TUMOR SUPPRESSOR PRECURSOR (FAT PROTEIN).//2.50E-25//165aa//40%//P33450
C-PLACE2000477//Homo sapiens putative secreted protein (ZSIG11) mRNA, complete cds.//6.70E-127//671bp//94%//AF072733
C-PLACE3000009//DNA-DIRECTED RNA POLYMERASE II LARGEST SUBUNIT (EC 2.7.7.6) (RPB1)(FRAGMENT).//3.50E-30//400aa//30%//P11414
C-PLACE3000020//Homo sapiens type III adenylyl cyclase (AC-III) mRNA, complete cds.//0//2253bp//99%//AF033861
C-PLACE3000059//Mus musculus mRNA for ubiquitin conjugating enzyme.//0//1979bp//90%//Y17267
C-PLACE3000121//VESICULAR TRAFFIC CONTROL PROTEIN SEC157/1.90E-08//281aa//22%//P22224
C-PLACE3000145//TENSIN.//1.00E-108//277aa//75%//Q04205
C-PLACE3000147//Homo sapiens metalloproteinase with thrombospondin type 1 motifs ADAMTS1 (ADAMTS1) mRNA, complete cds.//0//2043bp//99%//AF170084
C-PLACE3000169//ZINC FINGER PROTEIN 135.//2.50E-90//358aa//47%//P52742
C-PLACE3000218//Homo sapiens putative protein O-mannosyltransferase (POMT2) mRNA, complete cds.//0//1862bp//98%//AF105020
C-PLACE3000242//Human trophinin mRNA, complete cds.//0//2290bp//99%//U04811
C-PLACE3000244//PROTEIN TSG24 (MEIOTIC CHECK POINT REGULATOR).//0//1435aa//92%//P53995
C-PLACE3000254//Homo sapiens transcriptional activator SRCAP (SRCAP) mRNA, complete cds.//0//4583bp//83%//AF143946
C-PLACE3000339//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//9.60E-08//359aa//23%//P08640
C-PLACE3000350//SERINE/THREONINE-PROTEIN KINASE SULU (EC 2.7.1.-).//1.00E-54//418aa//38%//P46549
C-PLACE3000416//Homo sapiens mRNA for actin binding protein ABP620, complete cds.//1.80E-141//565bp//98%//AB029290
C-PLACE3000477//Homo sapiens phosphoprotein pp75 mRNA, partial cds.//0//3012bp//98%//AF153085
C-PLACE4000009//MYOSIN HEAVY CHAIN, NONMUSCLE TYPE B (CELLULAR MYOSIN HEAVY CHAIN, TYPE B) (NMMHC-B).//2.90E-54//626aa//29%//P35580
C-PLACE4000014//X-LINKED HEUCASE II (X-LINKED NUCLEAR PROTEIN) (XNP).//3.10E-111//348aa//41%//P46100
C-PLACE4000052//Homo sapiens ATP cassette binding transporter 1 (ABC1) mRNA, complete cds.//0//4661bp//99%//AF165281
C-PLACE4000063//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//1.70E-15//740aa//23%//P08640
C-PLACE4000100//Homo sapiens hydroxypyruvate reductase (GRHPR) gene, complete cds.//0//4199bp//97%//AF146689
C-PLACE4000128//Mus musculus putative transcription factor mRNA, complete cds.//1.60E-86//190aabp//88%//AF091234
C-PLACE4000156//ZINC FINGER PROTEIN 91 (ZINC FINGER PROTEIN HTF10) (HPF7).//1.40E-235//516aa//51%//Q05481
C-PLACE4000192//ZINC FINGER PROTEIN 142 (KIAA0236) (HA4654).//7.00E-22//369aa//25%//P52746
C-PLACE4000211//Homo sapiens BAZ2A mRNA for bromodomain adjacent to zinc finger domain 2A, complete cds.//0//5709bp//96%//AB032254
C-PLACE4000230//Mus musculus semaphorin VIa mRNA, complete cds.//0//2567bp//88%//AF030430
C-PLACE4000259//H.sapiens gene for U5 snRNP-specific 200kD protem.//0//5143bp//90%//Z70200
C-PLACE4000261//PEREGRIN (BR140 PROTEIN).//9.50E-10//128aa//34%//P55201
C-PLACE4000269//Rattus norvegicus rexo70 mRNA, complete cds.//0//2034bp//89%//AF032667
C-PLACE4000326//NAM7 PROTEIN (NONSENSE-MEDIATED MRNA DECAY PROTEIN 1) (UP-FRAMESHIFT SUPPRESSOR 1).//8.10E-24//319aa//31%//P30771
C-PLACE4000369//Homo sapiens thyroid hormone receptor-associated protein complex component TRAP240 mRNA, complete cds.//1.40E-185//1135bp//67%//AF117754
C-PLACE4000401//ACETYL-COENZYME A SYNTHETASE (EC 6.2.1.1) (ACETATE--COA LIGASE) (ACYL- ACTIVATING ENZYME).//7.20E-22//54aa//62%//Q01576
C-PLACE4000431//H.sapiens gene for U5 snRNP-specific 200kD protein.//0//5142bp//90%//Z70200
C-PLACE4000450//Homo sapiens BAZ2A mRNA for bromodomain adjacent to zinc finger domain 2A, complete cds.//0//5709bp//96%//AB032254
C-PLACE4000489//PROTEIN GRAINY-HEAD (DNA-BINDING PROTEIN ELF-1) (ELEMENT I-BINDING ACTIVITY) (TRANSCRIPTION FACTOR NTF-1)7/5.70E-60//254aa//44%//P13002
C-PLACE4000522//NEUROGENIC LOCUS NOTCH PROTEIN HOMOLOG PRECURSOR (XOTCH PROTEIN).//2.40E-191//828aa//48%//P21783
C-PLACE4000548//GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).//8.70E-13//784aa//21%//P08640
C-PLACE4000558//PROBABLE UBIQUITIN CARBOXYL-TERMINAL HYDROLASE FAF (EC 3.1.2.15) (UBIQUITIN THIOLESTERASE FAF) (UBIQUITIN-SPECIFIC PROCESSING PROTEASE FAF) (DEUBIQUITINATING ENZYME FAF) (FAT FACETS PROTEIN).//1.50E-26//252aa//35%//P55824
C-PLACE4000581//FIBROPELLIN I PRECURSOR (EPIDERMAL GROWTH FACTOR-RELATED PROTEIN 1) (UEGF-1).//9.30E-70//226aa//52%//P10079
C-PLACE4000650//TUBERIN (TUBEROUS SCLEROSIS 2 HOMOLOG PROTEIN).//7.90E-17//201aa//34%//P49816
C-PLACE4000654//Mus musculus mRNA for ubiquitin conjugating enzyme.//0//6340bp//87%//Y17267
C-SKNMC1000011//PUTATIVE IMPORTIN BETA-4 SUBUNIT (KARYOPHERIN BETA-4 SUBUNIT).//5.50E-35//431aa//29%//O60100
C-SKNMC1000013//Homo sapiens ATP-binding cassette protein M-ABC1 mRNA, nuclear gene encoding mitochondrial protein, complete cds7/0//2384bp//99%//AF047690
C-SKNMC1000046//Homo sapiens liprin-alpha3 mRNA, partial cds.//1.90E-162//749bp//99%//AF034800
C-SKNMC1000050//CALPAIN 2, LARGE [CATALYTIC] SUBUNIT (EC 3.4.22.17) (CALCIUM-ACTIVATED NEUTRAL PROTEINASE) (CANP) (M-TYPE).//3.20E-41//87aa//98%//P17655
C-SKNMC1000091//Homo sapiens mRNA for leucine-zipper protein, complete cds.//6.10E-190//872bp//99%//AB021663
C-THYRO1000034/TRICHOHYALIN.//9.40E-10//176aa//30%//P37709
C-THYRO1000072//MYOSIN UGHT CHAIN KINASE, SMOOTH MUSCLE AND NON-MUSCLE ISOZYMES (EC 2.7.1.117) (MLCK) [CONTAINS: TELOKIN].//3.40E-16//201aa//29%//P11799
C-THYRO1000085//PAIRED BOX PROTEIN PAX-8, ISOFORMS 8A/8B.//2.00E-72//155aa//92%//Q06710
C-THYRO1000121//Rattus norvegicus CTD-binding SR-like protein rA8 mRNA, complete cds.//0//1737bp//87%//U49055
C-THYRO1000132//Homo sapiens echinoderm microtubule-associated protein homolog HuEMAP mRNA, complete cds.//1.10E-159//824bp//95%//U97018
C-THYRO1000173//Homo sapiens AP-mu chain family member mulB (HSMU1B) mRNA, complete cds.//0//1713bp//99%//AF020797
C-THYRO1000197//Homo sapiens mRNA for poly(A)-specific ribonuclease.//0//2362bp//99%//AJ005698
C-THYRO1000242//ZINC FINGER PROTEIN 84 (ZINC FINGER PROTEIN HPF2).//5.00E-118//239aa//66%//P51523
C-THYRO1000288//Homo sapiens mRNA for Hs Ste24p, complete cds.//0//2161bp//99%//AB016068
C-THY-RO1000327//Homo sapiens autocrine motility factor receptor (AMFR) mRNA, complete cds.//0//1567bp//99%//AF124145
C-THYRO1000343//ATROPHIN-1 (DENTATORUBRAL-PALUDOLUYSIAN ATROPHY PROTEIN).//4.90E-06//280aa//31%//P54259
C-THYRO1000358//SELENIUM-BINDING LIVER PROTEIN.//2.30E-229//237aa//79%//P17563
C-THYRO1000394//Homo sapiens peroxisomal membrane protein PMP 24 mRNA, complete cds.//1.20E-299//1325bp//99%//AF072864
C-THYRO1000395//Homo sapiens actin-binding protein (IPP) mRNA, complete cds.//0//2092bp//99%//AF156857
C-THYRO1000401//Human TcD37 homolog (HTcD37) mRNA, partial cds.//1.10E-90//430bp//99%//U67085
C-THYRO1000488//Homo sapiens HFB30 mRNA, complete cds.//0//2254bp//100%//AB022663
C-THYRO1000501//52 KD RO PROTEIN (SJOGREN SYNDROME TYPE A ANTIGEN (SS-A))(RO(SS-A)).//4.20E-98//408aa//42%//P19474
C-THYRO1000569//Mus musculus hematopoietic zinc finger protein mRNA, complete cds.//0//1557bp//91%//AF118566
C-THYRO1000585//Homo sapiens protein associated with Myc mRNA, complete cds.//0//1901bp//99%//AF075587
C-THYRO1000605//Homo sapiens histone acetyltransferase (HBOa) mRNA, complete cds.//0//3080bp//99%//AF140360
C-THYRO1000662//Homo sapiens XPV mRNA for DNA polymerase eta, complete cds.//0//2341 bp//99%//AB024313
C-THYRO1000666//Mus musculus mRNA for kinesin like protein 9.//0//2001bp//86%//AJ132889
C-THYRO1000684//Homo sapiens BAG-family molecular chaperone regulator-5 mRNA, complete cds.//0//3347bp//99%//AF095195
C-THYRO1000748//RHO-GAP HEMATOPOIETIC PROTEIN C1 (P115) (KIAA0131).//3.30E-96//335aa//52%//P98171
C-THYRO1000756//ALPHA-N-ACETYLGALACTOSAMINIDE ALPHA-2,6-SIALYLTRANSFERASE (EC 2.4.99.-) (ST6GALNACIII)(STY).//1.80E-55//243aa//42%//Q64686
C-THYRO1000783//Xenopus laevis tail-specific thyroid hormone up-regulated (gene 5) mRNA, complete cds.//2.40E-157//1656bp//70%//U37373
C-THYRO1000852//Human branched-chain amino acid aminotransferase (ECA40) mRNA, complete cds.//1.40E-137//689bp//96%//U62739
C-THYRO1000926//Homo sapiens cAMP-specific phosphodiesterase 8B (PDE8B) mRNA, partial cds.//0//2387bp//99%//AF079529
C-THYRO11000934//PYRROLINE-5-CARBOXYLATE REDUCTASE (EC 1.5.1.2) (P5CR) (P5C REDUCTASE).//7.50E-57//315aa//43%//P32322
C-THYRO1000951//DIHYDROXYACETONE KINASE 2 (EC 2.7.1.29) (GLYCERONE KINASE).//5.00E-83//566aa//37%//P43550
C-THYRO1000983//UBIQUITIN-CONJUGATING ENZYME E2-17 KD 9 (EC 6.3.2.19) (UBIQUITIN-PROTEIN LIGASE 9) (UBIQUITIN CARRIER PROTEIN 9) (UBCAT4B).//6.30E-17//143aa//39%//P35132
C-THYRO1001003//UBIQUITIN-CONJUGATING ENZYME E2-21.2 KD (EC 6.3.2.19) (UBIQUITIN-PROTEIN UGASE) (UBIQUITIN CARRIER PROTEIN).//5.90E-14//84aa//41%//P52491
C-THYRO1001033//TRANSFORMATION-SENSITIVE PROTEIN IEF SSP 3521.//8.40E-12//167aa//29%//P31948
C-THYRO1001100//ZINC FINGER X-UNKED PROTEIN ZXDA (FRAGMENT).//1.20E-67//245aa//62%//P98168
C-THYRO1001120//Homo sapiens deltex (Dx) mRNA, complete cds.//1.30E-110//1947bp//65%//AF053700
C-THYRO1001134//Homo sapiens CGI-78 protein mRNA, complete cds.//0//1898bp//99%//AF151835
C-THYRO1001189//ZINC FINGER PROTEIN 91 (ZINC FINGER PROTEIN HTF10) (HPF7).//1.10E-200//546aa//62%//Q05481
C-THYRO1001204//Homo sapiens cathepsin Z precursor (CTSZ) gene, exons 4, 5, and 6 and complete cds; and TH1 gene partial sequence.//3.80E-100//478bp//99%//AF136276
C-THYRO1001287//MANNOSYL-OLIGOSACCHARIDE ALPHA-1,2-MANNOSIDASE (EC 3.2.1.113) (MAN(9)-ALPHA-MANNOSIDASE) (FRAGMENT).//3.40E-51//429aa//33%//P45701
C-THYRO1001313//Homo sapiens sorting nexin 11 (SNX11) mRNA, complete cds.//0//2330bp//94%//AF121861
C-THYRO1001347//Homo sapiens RAN binding protein 16 mRNA, complete cds.//2.00E-263//3101bp//68%//AF064729
C-THYRO1001374//CYTOSOLIC ACYL COENZYME A THIOESTER HYDROLASE (EC 3.1.2.2) (LONG CHAIN ACYL-COA THIOESTER HYDROLASE) (CTE-II).//1.80E-13//361aa//22%//O00154
C-THYRO1001405//PLECTIN.//6.90E-19//450aa//27%//P30427
C-THYRO1001406//PUTATIVE STEROID DEHYDROGENASE KIK-I (EC 1.1.1.-).//1.10E-131//219aa//81%//O70503
C-THYRO1001458//MYOSIN HEAVY CHAIN, NONMUSCLE TYPE B (CELLULAR MYOSIN HEAVY CHAIN, TYPE B) (NMMHC-B).//2.70E-171//559aa//59%//P35580
C-THYRO1001617//Homo sapiens cDNA for dihydroxyacetone phosphate acyltransferase (DAP-AT).//0//1784bp//99%//AJ002190
C-THYRO1001656//Homo sapiens Leman coiled-coil protein (LCCP) mRNA, complete cds.//4.10E-273//1947bp//82%//AF175968
C-THYRO1001671//Homo sapiens mRNA for 2'-5' oligoadenylate synthetase 59 kDa isoform.//0//1820bp//99%//AJ225089
C-THYRO1001703//NIFR3-LIKEPROTEIN.//2.90E-32//282aa//32%//P45672
C-THYRO1001721//RING CANAL PROTEIN (KELCH PROTEIN).//9.30E-34//220aa//38%//Q04652
C-THYRO1001738//TUBULIN--TYROSINE LIGASE (EC 6.3.2.25) (TTL).//2.40E-20//217aa//30%//P38584
C-THYRO1001809//MYOCYTE NUCLEAR FACTOR (MNF).//1.40E-74//158aa//89%//P42128
C-Y79AA1000013//Mus musculus RING finger protein A07 mRNA, complete cds.//8.90E-205//1435bp//81%//AF171060
C-Y79AA1000033//Homo sapiens CARD4 mRNA, complete cds.//0//2929bp//96%//AF126484
C-Y79AA1000037//DNA-BINDING PROTEIN BMI-1.//2.40E-30//80aa//60%//P25916
C-Y79AA1000059//Homo sapiens aryl-hydrocarbon interacting protein-like 1 (AIPL1) gene, complete cds.//0//980bp//96%//AF180472
C-Y79AA1000181//Homo sapiens CGI-01 protein mRNA, complete cds.//0//1858bp//99%//AF132936
C-Y79AA1000214//Homo sapiens histone H2A.F/Z variant (H2AV) mRNA, complete cds.//7.10E-71//345bp//100%//AF081192
C-Y79AA1000231//Homo sapiens nucleolar protein NOP5/NOP58 mRNA, complete cds.//0//1515bp//99%//AF123534
C-Y79AA1000268//Mus musculus Nip21 mRNA, complete cds.//2.10E-50//648bp//64%//AF035207
C-Y79AA1000313//CALPHOTIN.//0.000011//336aa//23%//Q02910
C-Y79AA1000328//SEL-10 PROTEIN.//0.000000067//219aa//25%//Q93794
C-Y79AA1000342//Homo sapiens Ciz1 mRNA, complete cds.//0//2644bp//81%//AB030835
C-Y79AA1000346//Homo sapiens nonclathrin coat protein gamma2-COP mRNA, complete cds.//0//2520bp//99%//AF157833
C-Y79AA1000349//M.musculus Spnr mRNA for RNA binding protein.//0//2048bp//93%//X84692
C-Y79AA1000368//REDUCED VIABILITY UPON STARVATION PROTEIN 161.//4.00E-20//261aa//27%//P25343
C-Y79AA1000469//Mus musculus ancient ubiquitous 46 kDa protein AUP1 precursor (Aup1) mRNA, complete cds.//8.30E-252//1207bp//85%//U41736
C-Y79AA1000540//CELL POLARITY PROTEIN TEA1.//2.10E-12//211aa//33%//P87061
C-Y79AA1000560//ALPHA-ADAPTIN C (CLATHRIN ASSEMBLY PROTEIN COMPLEX 2 ALPHA-C LARGE CHAIN) (100 KD COATED VESICLE PROTEIN C) (PLASMA MEMBRANE ADAPTOR HA2/AP2 ADAPTIN ALPHA C SUBUNIT)7/0//652aa//98%//P17427
C-Y79AA1000589//32.3 KD PROTEIN IN CWP1-MBR1 INTERGENIC REGION.//2.40E-27//216aa//34%//P28320
C-Y79AA1000627//Homo sapiens zinc finger protein (ZF5128) mRNA, complete cds.//2.00E-287//2031bp//82%//AF060503
C-Y79AA1000705//M.musculus mRNA of enhancer-trap-locus 1.//5.80E-254//1477bp//84%//X69942
C-Y79AA1000734//Homo sapiens peroxisomal biogenesis factor (PEX11b) mRNA, complete cds.//0//1594bp//99%//AF093670
C-Y79AA1000748//Rattus norvegicus clone C42 CDK5 activator-binding protein mRNA, complete cds.//6.60E-286//1832bp//84%//AF177477
C-Y79AA1000752//PUTATIVE HETEROGENEOUS NUCLEAR RIBONUCLEOPROTEIN X (HNRNP X) (CBP).//4.90E-91//200aa//64%//Q61990
C-Y79AA1000782//CYTOSOLIC PURINE 5'-NUCLEOTIDASE (EC 3.1.3.5).//3.00E-37//469aa//27%//P49902
C-Y79AA1000784//Homo sapiens RanBP7/importin 7 mRNA, complete cds.//1.10E-236//1076bp//99%//AF098799
C-Y79AA1000794//Homo sapiens actin-associated protein 2E4/kaptin (2E4) mRNA, 2E4-1 allele, complete cds.//0//1610bp//99%//AF105369
C-Y79AA1000800//Homo sapiens putative secreted protein (ZSIG11) mRNA, complete cds.//1.60E-284//1288bp//99%//AF072733
C-Y79AA1000833//TUBULIN ALPHA-1 CHAIN.//5.00E-173//220aa//79%//P05209
C-Y79AA1000962//MYOSIN HEAVY CHAIN, NON-MUSCLE (ZIPPER PROTEIN) (MYOSIN II)7/4.20E-17//430aa//27%//Q99323
C-Y79AA1000966//Homo sapiens COP9 complex subunit 4 mRNA, complete cds.//0//1586bp//99%//AF100757
C-Y79AA1000968//Rattus norvegicus initiation factor eIF-2B gamma subunit (eIF-2B gamma) mRNA, complete cds.//3.90E-248//1468bp//87%//U38253
C-Y79AA1000985//Human centrosomal protein kendrin mRNA, complete cds.//4.70E-151//985bp//87%//U52962
C-Y79AA1001048//ACYL-COA DEHYDROGENASE, VERY-LONG-CHAIN SPECIFIC PRECURSOR (EC 1.3.99.-) (VLCAD).//3.10E-138//583aa//47%//P45953
C-Y79AA1001211//Homo sapiens origin recognition complex subunit 6 (ORC6) mRNA, complete cds.//0//1435bp//99%//AF139658
C-Y79AA1001233//ESTRADIOL 17 BETA-DEHYDROGENASE 1 (EC 1.1.1.62) (17-BETA-HSD 1) (17-BETA-HYDROXYSTEROID DEHYDROGENASE 1).//7.70E-50//228aa//42%//P51657
C-Y79AA1001236//Homo sapiens cell division protein mRNA, complete cds.//0//1612bp//99%//AF063015
C-Y79AA1001299//Homo sapiens mRNA for integrase interactor 1b protein (INI1B).//0//996bp//99%//AJ011738
C-Y79AA1001312//ZINC FINGER PROTEIN MLZ-4 (ZINC FINGER PROTEIN 46).//0.000000023//193aa//30%//Q03309
C-Y79AA1001323//Mus musculus mRNA for GSG1, complete cds.//3.30E-172//1171bp//83%//D87325
C-Y79AA1001384//Homo sapiens very large G-protein coupled receptor-1 (VLGR1) mRNA, complete cds.//0//4708bp//99%//AF055084
C-Y79AA1001391//HOMEOBOX PROTEIN HOX-A13 (HOX-1J).//1.20E-58//178aa//66%//P31271
C-Y79AA1001394//CELL DIVISION PROTEIN FTSH HOMOLOG (EC 3.4.24.-).//1.20E-13//230aa//32%//O83746
C-Y79AA1001402//Homo sapiens paraneoplastic cancer-testis-brain antigen (MA4) mRNA, partial cds.//8.50E-65//784bp//62%//AF083115
C-Y79AA1001493//UBIQUITIN-CONJUGATING ENZYME E2-17 KD 9 (EC 6.3.2.19) (UBIQUITIN-PROTEIN LIGASE 9) (UBIQUITIN CARRIER PROTEIN 9) (UBCAT4B).//3.80E-18//151aa//38%//P35132
C-Y79AA1001533//Mouse mRNA for RNA polymerase I associated factor (PAF53), complete cds.//4.50E-193//1333bp//80%//D14336
C-Y79AA1001548//PHOSPHATIDYLINOSITOL 4-KINASE ALPHA (EC 2.7.1.67) (PI4-KINASE) (PTDINS-4-KINASE) (PI4K-ALPHA).//7.50E-76//85aa//90%//P42356
C-Y79AA1001581//ACETYL-COENZYME A SYNTHETASE (EC 6.2.1.1) (ACETATE--COA LIGASE) (ACYL- ACTIVATING ENZYME).//1.90E-40//482aa//27%//P27550
C-Y79AA1001594//HYALURONAN-MEDIATED MOTILITY RECEPTOR (HYALURONIC ACID RECEPTOR).//2.50E-14//410aa//24%//Q00547
C-Y79AA1001603//POLYPEPTIDE N-ACETYLGALACTOSAMINYLTRANSFERASE (EC 2.4.1.41) (PROTEIN-UDP ACETYLGALACTOSAMINYLTRANSFERASE) (UDP-GALNAC:POLYPEPTIDE, N- ACETYLGALACTOSAMINYLTRANSFERASE) (GALNAC-T1).//1.70E-84//313aa//48%//Q07537
C-Y79AA1001613//ZINC FINGER PROTEIN 132.//3.80E-91//209aa//41%//P52740
C-Y79AA1001679//Homo sapiens lambda-crystallin mRNA, complete cds.//3.4e-310//1430bp//98%//AF077049
C-Y79AA1001692//Mus musculus strain C57BL/J germ cell-less protein (Gc1) mRNA, complete cds.//1.40E-78//227aa//40%//Q01820
C-Y79AA1001705//Homo sapiens p53 regulated PA26-T2 nuclear protein (PA26) mRNA, complete cds.//3.40E-47//626bp//68%//AF033120
C-Y79AA1001711//Human 60-kdal ribonucleoprotein (Ro) mRNA, complete cds.//1.20E-258//1185bp//99%//J04137
C-Y79AA1001827//Homo sapiens mammalian inositol hexakisphosphate kinase 2 (IP6K2) mRNA, complete cds.//0//1689bp//98%//AF177145
C-Y79AA1001866//Homo sapiens zinc finger protein ZNF180 (ZNF180) mRNA, complete cds.//0//2927bp//97%//AF192913
C-Y79AA1001874//OX40L RECEPTOR PRECURSOR (ACT35 ANTIGEN) (TAX-TRANSCRIPTIONALLY ACTIVATED GLYCOPROTEIN 1 RECEPTOR) (CD134 ANTIGEN).//4.50E-08//135aa//31%//P43489
C-Y79AA1001875//RAS-RELATED PROTEIN RAB-7.//9.40E-12//34aa//97%//P51149
C-Y79AA1001923//Homo sapiens F-box protein Fbx22 (FBX22) gene, partial cds.//7.10E-52//279bp//97%//AF174602
C-Y79AA1001963//PUTATIVE PRE-MRNA SPLICING FACTOR ATP-DEPENDENT RNA HELICASE SPAC10F6.02C.//1.00E-10//94aa//47%//O42643
C-Y79AA1002027//UBIQUITIN-CONJUGATING ENZYME E2-18 KD (EC 6.3.2.19) (UBIQUITIN- PROTEIN LIGASE) (UBIQUITIN CARRIER PROTEIN) (PM42).//9.90E-39//143aa//52%//P42743
C-Y79AA1002083//H.sapiens mRNA for MUF1 protein.//5.00E-163//752bp//99%//X86018
C-Y79AA1002103//ZINC FINGER PROTEIN ZFP-36 (FRAGMENT).//3.00E-257//549aa//76%//P16415
C-Y79AA1002139//DNAJ PROTEIN HOMOLOG 1 (DROJ1).//9.00E-17//120aa//45%//Q24133
C-Y79AA1002204//COMPLEXIN 2 (SYNAPHIN 1) (921-L).//7.50E-09//131aa//35%//Q13329
C-Y79AA1002208//ANKYRIN.//8.10E-34//188aa//38%//Q02357
C-Y79AA1002209/TYROSYL-TRNA SYNTHETASE (EC 6.1.1.1) (TYROSINE--TRNA LIGASE) (TYRRS).//1.60E-72//437aa//39%//P00952
C-Y79AA1002210//TUMOR NECROSIS FACTOR, ALPHA-INDUCED PROTEIN 1, ENDOTHELIAL (B12 PROTEIN).//0.0000018//140aa//25%//Q13829
C-Y79AA1002211//PHOSPHATIDYLETHANOLAMINE-BINDING PROTEIN HOMOLOG F40A3.3.//1.70E-17//146aa//35%//O16264
C-Y79AA1002229//DNA CROSS-LINK REPAIR PROTEIN PSO2/SNM1.//7.10E-17//213aa//31%//P30620
C-Y79AA1002246//SYNAPTOTAGMIN V.//1.60E-28//286aa//32%//O00445
C-Y79AA1002258//Homo sapiens mRNA for HIP1R, complete cds.//0//2106bp//99%//AB013384
C-Y79AA1002307//Homo sapiens astrotactin2 (ASTN2) mRNA, complete cds.//0//1209bp//99%//AF116574
C-Y79AA1002311//R.norvegicus mRNA for cytosolic resiniferatoxin-binding protein.//2.90E-186//1130bp//82%//X67877
C-Y79AA1002361//Rattus norvegicus mRNA for protein phosphatase 1 (GL-subunit).//6.90E-140//966bp//82%//Y18208
C-Y79AA1002399//Homo sapiens mRNA for sperm protein.//0//1163bp//95%//X91879
C-Y79AA1002416//Mus musculus CTP synthetase homolog (CTPsH) mRNA, complete cds.//3.9e-317//1902bp//86%//U49385
C-Y79AA1002431//TRANSDUCIN-LIKE ENHANCER PROTEIN 2 (ESG2).//9.80E-62//318aa//35%//Q04725
C-Y79AA1002433//Homo sapiens chromatin- specific transcription elongation factor FACT 140 kDa subunit mRNA, complete cds.//0//1545bp//96%//AF152961
C-Y79AA1002472//ZINC FINGER PROTEIN 91 (ZINC FINGER PROTEIN HTF10) (HPF7).//1.50E-136//472aa//49%//Q05481
C-Y79AA1002482//ZINC FINGER PROTEIN 91 (ZINC FINGER PROTEIN HTF10) (HPF7).//2.70E-137//340aa//51%//Q05481
C-Y79AA1002487//Homo sapiens chromosome 5 F-box protein Fbx4 (FBX4) mRNA, complete cds.//7.3e-311//1444bp//98%//AF129534

## Claims

1. Use of an oligonucleotide as a primer for synthesizing the polynucleotide comprising the nucleotide sequence set forth in any one of SEQ ID NOs: 1-5547 and SEQ ID NOs: 16111-16164, or the complementary strand thereof, wherein said oligonucleotide is complementary to said polynucleotide or the complementary strand thereof and comprises at least 15 nucleotides.

2. A primer set for synthesizing polynucleotides, the primer set comprising an oligo-dT primer and an oligonucleotide complementary to the complementary strand of the polynucleotide comprising the nucleotide sequence set forth in any one of SEQ ID NOs: 1-5547 and SEQ ID NOs: 16111-16164, wherein said oligonucleotide comprises at least 15 nucleotides.

3. A primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence 3'-end nucleotide sequence is selected from the group consisting of:

SEQ ID NO: 1 / SEQ ID NO: 5548, SEQ ID NO: 4 / SEQ ID NO: 5549, SEQ ID NO: 5 / SEQ
ID NO: 5550, SEQ ID NO: 6 / SEQ ID NO: 5551, SEQ ID NO: 7 / SEQ ID NO: 5552, SEQ ID
NO: 8 / SEQ ID NO: 5553, SEQ ID NO: 9 / SEQ ID NO: 5554, SEQ ID NO: 10 / SEQ ID NO:
5555, SEQ ID NO: 11 / SEQ ID NO: 5556, SEQ ID NO: 12 / SEQ ID NO: 5557, SEQ ID NO:
13 / SEQ ID NO: 5558, SEQ ID NO: 14 / SEQ ID NO: 5559, SEQ ID NO: 15 / SEQ ID NO:
5560, SEQ ID NO: 16 / SEQ ID NO: 5561, SEQ ID NO: 17 / SEQ ID NO: 5562, SEQ ID NO:
18 / SEQ ID NO: 5563, SEQ ID NO: 19 / SEQ ID NO: 5564, SEQ ID NO: 20 / SEQ ID NO:
5565, SEQ ID NO: 21 / SEQ ID NO: 5566, SEQ ID NO: 22 / SEQ ID NO: 5567, SEQ ID NO:
23 / SEQ ID NO: 5568, SEQ ID NO: 24 / SEQ ID NO: 5569, SEQ ID NO: 25 / SEQ ID NO:
5570, SEQ ID NO: 26 / SEQ ID NO: 5571, SEQ ID NO: 27 / SEQ ID NO: 5572, SEQ ID NO:
28 / SEQ ID NO: 5573, SEQ ID NO: 29 / SEQ ID NO: 5574, SEQ ID NO: 30 / SEQ ID NO:
5575, SEQ ID NO: 31 / SEQ ID NO: 5576, SEQ ID NO: 32 / SEQ ID NO: 5577, SEQ ID NO:
33 / SEQ ID NO: 5578, SEQ ID NO: 34 / SEQ ID NO: 5579, SEQ ID NO: 35 / SEQ ID NO:
5580, SEQ ID NO: 37 / SEQ ID NO: 5581, SEQ ID NO: 38 / SEQ ID NO: 5582, SEQ ID NO:
39 / SEQ ID NO: 5583, SEQ ID NO: 40 / SEQ ID NO: 5584, SEQ ID NO: 42 / SEQ ID NO:
5585, SEQ ID NO: 43 / SEQ ID NO: 5586, SEQ ID NO: 44 / SEQ ID NO: 5587, SEQ ID NO:
45 / SEQ ID NO: 5588, SEQ ID NO: 46 / SEQ ID NO: 5589, SEQ ID NO: 47 / SEQ ID NO:
5590, SEQ ID NO: 48 / SEQ ID NO: 5591, SEQ ID NO: 49 / SEQ ID NO: 5592, SEQ ID NO:
50 / SEQ ID NO: 5593, SEQ ID NO: 51 / SEQ ID NO: 5594, SEQ ID NO: 52 / SEQ ID NO:
5595, SEQ ID NO: 53 / SEQ ID NO: 5596, SEQ ID NO: 54 / SEQ ID NO: 5597, SEQ ID NO:
55 / SEQ ID NO: 5598, SEQ ID NO: 56 / SEQ ID NO: 5599, SEQ ID NO: 57 / SEQ ID NO:
5600, SEQ ID NO: 58 / SEQ ID NO: 5601, SEQ ID NO: 59 / SEQ ID NO: 5602, SEQ ID NO:
60 / SEQ ID NO: 5603, SEQ ID NO: 61 / SEQ ID NO: 5604, SEQ ID NO: 62 / SEQ ID NO:
5605, SEQ ID NO: 63 / SEQ ID NO: 5606, SEQ ID NO: 65 / SEQ ID NO: 5607, SEQ ID NO:
66 / SEQ ID NO: 5608, SEQ ID NO: 67 / SEQ ID NO: 5609, SEQ ID NO: 68 / SEQ ID NO:
5610, SEQ ID NO: 69 / SEQ ID NO: 5611, SEQ ID NO: 70 / SEQ ID NO: 5612, SEQ ID NO:
71 / SEQ ID NO: 5613, SEQ ID NO: 72 / SEQ ID NO: 5614, SEQ ID NO: 74 / SEQ ID NO:
5615, SEQ ID NO: 76 / SEQ ID NO: 5616, SEQ ID NO: 77 / SEQ ID NO: 5617, SEQ ID NO:
78 / SEQ ID NO: 5618, SEQ ID NO: 79 / SEQ ID NO: 5619, SEQ ID NO: 80 / SEQ ID NO:
5620, SEQ ID NO: 81 / SEQ ID NO: 5621, SEQ ID NO: 82 / SEQ ID NO: 5622, SEQ ID NO:
83 / SEQ ID NO: 5623, SEQ ID NO: 84 / SEQ ID NO: 5624, SEQ ID NO: 85 / SEQ ID NO:
5625, SEQ ID NO: 86 / SEQ ID NO: 5626, SEQ ID NO: 87 / SEQ ID NO: 5627, SEQ ID NO:
88 / SEQ ID NO: 5628, SEQ ID NO: 89 / SEQ ID NO: 5629, SEQ ID NO: 90 / SEQ ID NO:
5630, SEQ ID NO: 91 / SEQ ID NO: 5631, SEQ ID NO: 92 / SEQ ID NO: 5632, SEQ ID NO:

93 / SEQ ID NO: 5633, SEQ ID NO: 94 / SEQ ID NO: 5634, SEQ ID NO: 95 / SEQ ID NO:
5635, SEQ ID NO: 96 / SEQ ID NO: 5636, SEQ ID NO: 97 / SEQ ID NO: 5637, SEQ ID NO:
98 / SEQ ID NO: 5638, SEQ ID NO: 99 / SEQ ID NO: 5639, SEQ ID NO: 100 / SEQ ID NO:
5640, SEQ ID NO: 101 / SEQ ID NO: 5641, SEQ ID NO: 102 / SEQ ID NO: 5642, SEQ ID
NO: 103 / SEQ ID NO: 5643, SEQ ID NO: 104 / SEQ ID NO: 5644, SEQ ID NO: 105 / SEQ ID
NO: 5645, SEQ ID NO: 106 / SEQ ID NO: 5646, SEQ ID NO: 107 / SEQ ID NO: 5647,
SEQ ID NO: 108 / SEQ ID NO: 5648, SEQ ID NO: 109 / SEQ ID NO: 5649, SEQ ID NO: 110 /
SEQ ID NO: 5650, SEQ ID NO: 111 / SEQ ID NO: 5651, SEQ ID NO: 112 / SEQ ID NO: 5652,
SEQ ID NO: 113 / SEQ ID NO: 5653, SEQ ID NO: 114 / SEQ ID NO: 5654, SEQ ID NO: 115 /
SEQ ID NO: 5655, SEQ ID NO: 116 / SEQ ID NO: 5656, SEQ ID NO: 117 / SEQ ID NO: 5657,
SEQ ID NO: 118 / SEQ ID NO: 5658, SEQ ID NO: 119 / SEQ ID NO: 5659, SEQ ID NO: 120 /
SEQ ID NO: 5660, SEQ ID NO: 121 / SEQ ID NO: 5661, SEQ ID NO: 122 / SEQ ID NO: 5662,
SEQ ID NO: 123 / SEQ ID NO: 5663, SEQ ID NO: 124 / SEQ ID NO: 5664, SEQ ID NO: 125 /
SEQ ID NO: 5665, SEQ ID NO: 126 / SEQ ID NO: 5666, SEQ ID NO: 127 / SEQ ID NO: 5667,
SEQ ID NO: 128 / SEQ ID NO: 5668, SEQ ID NO: 129 / SEQ ID NO: 5669, SEQ ID NO: 130 /
SEQ ID NO: 5670, SEQ ID NO: 131 / SEQ ID NO: 5671, SEQ ID NO: 132 / SEQ ID NO: 5672,
SEQ ID NO: 133 / SEQ ID NO: 5673, SEQ ID NO: 134 / SEQ ID NO: 5674, SEQ ID NO: 135 /
SEQ ID NO: 5675, SEQ ID NO: 136 / SEQ ID NO: 5676, SEQ ID NO: 137 / SEQ ID NO: 5677,
SEQ ID NO: 138 / SEQ ID NO: 5678, SEQ ID NO: 139 / SEQ ID NO: 5679, SEQ ID NO: 140 /
SEQ ID NO: 5680, SEQ ID NO: 141 / SEQ ID NO: 5681, SEQ ID NO: 142 / SEQ ID NO: 5682,
SEQ ID NO: 143 / SEQ ID NO: 5683, SEQ ID NO: 144 / SEQ ID NO: 5684, SEQ ID NO: 145 /
SEQ ID NO: 5685, SEQ ID NO: 146 / SEQ ID NO: 5686, SEQ ID NO: 147 / SEQ ID NO: 5687,
SEQ ID NO: 148 / SEQ ID NO: 5688, SEQ ID NO: 149 / SEQ ID NO: 5689, SEQ ID NO: 150 /
SEQ ID NO: 5690, SEQ ID NO: 151 / SEQ ID NO: 5691, SEQ ID NO: 153 / SEQ ID NO: 5692,
SEQ ID NO: 154 / SEQ ID NO: 5693, SEQ ID NO: 155 / SEQ ID NO: 5694, SEQ ID NO: 156 /
SEQ ID NO: 5695, SEQ ID NO: 157 / SEQ ID NO: 5696, SEQ ID NO: 158 / SEQ ID NO: 5697,
SEQ ID NO: 159 / SEQ ID NO: 5698, SEQ ID NO: 160 / SEQ ID NO: 5699, SEQ ID NO: 161 /
SEQ ID NO: 5700, SEQ ID NO: 162 / SEQ ID NO: 5701, SEQ ID NO: 163 / SEQ ID NO: 5702,
SEQ ID NO: 164 / SEQ ID NO: 5703, SEQ ID NO: 165 / SEQ ID NO: 5704, SEQ ID NO: 166 /
SEQ ID NO: 5705, SEQ ID NO: 167 / SEQ ID NO: 5706, SEQ ID NO: 168 / SEQ ID NO: 5707,
SEQ ID NO: 169 / SEQ ID NO: 5708, SEQ ID NO: 170 / SEQ ID NO: 5709, SEQ ID NO: 171 /
SEQ ID NO: 5710, SEQ ID NO: 173 / SEQ ID NO: 5711, SEQ ID NO: 174 / SEQ ID NO: 5712,
SEQ ID NO: 175 / SEQ ID NO: 5713, SEQ ID NO: 176 / SEQ ID NO: 5714, SEQ ID NO: 177 /
SEQ ID NO: 5715, SEQ ID NO: 178 / SEQ ID NO: 5716, SEQ ID NO: 179 / SEQ ID NO: 5717,
SEQ ID NO: 180 / SEQ ID NO: 5718, SEQ ID NO: 181 / SEQ ID NO: 5719, SEQ ID NO: 182 /
SEQ ID NO: 5720, SEQ ID NO: 183 / SEQ ID NO: 5721, SEQ ID NO: 184 / SEQ ID NO: 5722,
SEQ ID NO: 185 / SEQ ID NO: 5723, SEQ ID NO: 186 / SEQ ID NO: 5724, SEQ ID NO: 187 /
SEQ ID NO: 5725, SEQ ID NO: 188 / SEQ ID NO: 5726, SEQ ID NO: 189 / SEQ ID NO: 5727,
SEQ ID NO: 190 / SEQ ID NO: 5728, SEQ ID NO: 191 / SEQ ID NO: 5729, SEQ ID NO: 192 /
SEQ ID NO: 5730, SEQ ID NO: 193 / SEQ ID NO: 5731, SEQ ID NO: 194 / SEQ ID NO: 5732,
SEQ ID NO: 195 / SEQ ID NO: 5733, SEQ ID NO: 196 / SEQ ID NO: 5734, SEQ ID NO: 197 /
SEQ ID NO: 5735, SEQ ID NO: 198 / SEQ ID NO: 5736, SEQ ID NO: 199 / SEQ ID NO: 5737,
SEQ ID NO: 200 / SEQ ID NO: 5738, SEQ ID NO: 201 / SEQ ID NO: 5739, SEQ ID NO: 202 /
SEQ ID NO: 5740, SEQ ID NO: 203 / SEQ ID NO: 5741, SEQ ID NO: 204 / SEQ ID NO: 5742,
SEQ ID NO: 205 / SEQ ID NO: 5743, SEQ ID NO: 206 / SEQ ID NO: 5744, SEQ ID NO: 207 /
SEQ ID NO: 5745, SEQ ID NO: 208 / SEQ ID NO: 5746, SEQ ID NO: 209 / SEQ ID NO: 5747,
SEQ ID NO: 210 / SEQ ID NO: 5748, SEQ ID NO: 211 / SEQ ID NO: 5749, SEQ ID NO: 212 /
SEQ ID NO: 5750, SEQ ID NO: 213 / SEQ ID NO: 5751, SEQ ID NO: 214 / SEQ ID NO: 5752,
SEQ ID NO: 215 / SEQ ID NO: 5753, SEQ ID NO: 216 / SEQ ID NO: 5754, SEQ ID NO: 217 /
SEQ ID NO: 5755, SEQ ID NO: 218 / SEQ ID NO: 5756, SEQ ID NO: 219 / SEQ ID NO: 5757,
SEQ ID NO: 220 / SEQ ID NO: 5758, SEQ ID NO: 221 / SEQ ID NO: 5759, SEQ ID NO: 222 /
SEQ ID NO: 5760, SEQ ID NO: 223 / SEQ ID NO: 5761, SEQ ID NO: 224 / SEQ ID NO: 5762,

SEQ ID NO: 225 / SEQ ID NO: 5763, SEQ ID NO: 226 / SEQ ID NO: 5764, SEQ ID NO: 227 /
SEQ ID NO: 5765, SEQ ID NO: 228 / SEQ ID NO: 5766, SEQ ID NO: 229 / SEQ ID NO: 5767,
SEQ ID NO: 230 / SEQ ID NO: 5768, SEQ ID NO: 231 / SEQ ID NO: 5769, SEQ ID NO: 232 /
SEQ ID NO: 5770, SEQ ID NO: 233 / SEQ ID NO: 5771, SEQ ID NO: 234 / SEQ ID NO: 5772,
SEQ ID NO: 235 / SEQ ID NO: 5773, SEQ ID NO: 236 / SEQ ID NO: 5774, SEQ ID NO: 238 /
SEQ ID NO: 5775, SEQ ID NO: 239 / SEQ ID NO: 5776, SEQ ID NO: 240 / SEQ ID NO: 5777,
SEQ ID NO: 241 / SEQ ID NO: 5778, SEQ ID NO: 242 / SEQ ID NO: 5779, SEQ ID NO: 243 /
SEQ ID NO: 5780, SEQ ID NO: 244 / SEQ ID NO: 5781, SEQ ID NO: 245 / SEQ ID NO: 5782,
SEQ ID NO: 246 / SEQ ID NO: 5783, SEQ ID NO: 247 / SEQ ID NO: 5784, SEQ ID NO: 248 /
SEQ ID NO: 5785, SEQ ID NO: 249 / SEQ ID NO: 5786, SEQ ID NO: 250 / SEQ ID NO: 5787,
SEQ ID NO: 251 / SEQ ID NO: 5788, SEQ ID NO: 252 / SEQ ID NO: 5789, SEQ ID NO: 253 /
SEQ ID NO: 5790, SEQ ID NO: 254 / SEQ ID NO: 5791, SEQ ID NO: 255 / SEQ ID NO: 5792,
SEQ ID NO: 256 / SEQ ID NO: 5793, SEQ ID NO: 257 / SEQ ID NO: 5794, SEQ ID NO: 258 /
SEQ ID NO: 5795, SEQ ID NO: 259 / SEQ ID NO: 5796, SEQ ID NO: 260 / SEQ ID NO: 5797,
SEQ ID NO: 261 / SEQ ID NO: 5798, SEQ ID NO: 262 / SEQ ID NO: 5799, SEQ ID NO: 263 /
SEQ ID NO: 5800, SEQ ID NO: 264 / SEQ ID NO: 5801, SEQ ID NO: 265 / SEQ ID NO: 5802,
SEQ ID NO: 266 / SEQ ID NO: 5803, SEQ ID NO: 267 / SEQ ID NO: 5804, SEQ ID NO: 268 /
SEQ ID NO: 5805, SEQ ID NO: 269 / SEQ ID NO: 5806, SEQ ID NO: 270 / SEQ ID NO: 5807,
SEQ ID NO: 271 / SEQ ID NO: 5808, SEQ ID NO: 272 / SEQ ID NO: 5809, SEQ ID NO: 273 /
SEQ ID NO: 5810, SEQ ID NO: 274 / SEQ ID NO: 5811, SEQ ID NO: 275 / SEQ ID NO: 5812,
SEQ ID NO: 276 / SEQ ID NO: 5813, SEQ ID NO: 277 / SEQ ID NO: 5814, SEQ ID NO: 278 /
SEQ ID NO: 5815, SEQ ID NO: 279 / SEQ ID NO: 5816, SEQ ID NO: 280 / SEQ ID NO: 5817,
SEQ ID NO: 281 / SEQ ID NO: 5818, SEQ ID NO: 282 / SEQ ID NO: 5819, SEQ ID NO: 283 /
SEQ ID NO: 5820, SEQ ID NO: 284 / SEQ ID NO: 5821, SEQ ID NO: 285 / SEQ ID NO: 5822,
SEQ ID NO: 286 / SEQ ID NO: 5823, SEQ ID NO: 287 / SEQ ID NO: 5824, SEQ ID NO: 288 /
SEQ ID NO: 5825, SEQ ID NO: 289 / SEQ ID NO: 5826, SEQ ID NO: 290 / SEQ ID NO: 5827,
SEQ ID NO: 291 / SEQ ID NO: 5828, SEQ ID NO: 292 / SEQ ID NO: 5829, SEQ ID NO: 293 /
SEQ ID NO: 5830, SEQ ID NO: 294 / SEQ ID NO: 5831, SEQ ID NO: 295 / SEQ ID NO: 5832,
SEQ ID NO: 296 / SEQ ID NO: 5833, SEQ ID NO: 297 / SEQ ID NO: 5834, SEQ ID NO: 298 /
SEQ ID NO: 5835, SEQ ID NO: 299 / SEQ ID NO: 5836, SEQ ID NO: 300 / SEQ ID NO: 5837,
SEQ ID NO: 301 / SEQ ID NO: 5838, SEQ ID NO: 302 / SEQ ID NO: 5839, SEQ ID NO: 303 /
SEQ ID NO: 5840, SEQ ID NO: 304 / SEQ ID NO: 5841, SEQ ID NO: 305 / SEQ ID NO: 5842,
SEQ ID NO: 306 / SEQ ID NO: 5843, SEQ ID NO: 307 / SEQ ID NO: 5844, SEQ ID NO: 308 /
SEQ ID NO: 5845, SEQ ID NO: 309 / SEQ ID NO: 5846, SEQ ID NO: 310 / SEQ ID NO: 5847,
SEQ ID NO: 311 / SEQ ID NO: 5848, SEQ ID NO: 312 / SEQ ID NO: 5849, SEQ ID NO: 313 /
SEQ ID NO: 5850, SEQ ID NO: 314 / SEQ ID NO: 5851, SEQ ID NO: 315 / SEQ ID NO: 5852,
SEQ ID NO: 316 / SEQ ID NO: 5853, SEQ ID NO: 317 / SEQ ID NO: 5854, SEQ ID NO: 318 /
SEQ ID NO: 5855, SEQ ID NO: 319 / SEQ ID NO: 5856, SEQ ID NO: 320 / SEQ ID NO: 5857,
SEQ ID NO: 321 / SEQ ID NO: 5858, SEQ ID NO: 322 / SEQ ID NO: 5859, SEQ ID NO: 323 /
SEQ ID NO: 5860, SEQ ID NO: 324 / SEQ ID NO: 5861, SEQ ID NO: 325 / SEQ ID NO: 5862,
SEQ ID NO: 326 / SEQ ID NO: 5863, SEQ ID NO: 327 / SEQ ID NO: 5864, SEQ ID NO: 328 /
SEQ ID NO: 5865, SEQ ID NO: 329 / SEQ ID NO: 5866, SEQ ID NO: 330 / SEQ ID NO: 5867,
SEQ ID NO: 331 / SEQ ID NO: 5868, SEQ ID NO: 332 / SEQ ID NO: 5869, SEQ ID NO: 334 /
SEQ ID NO: 5870, SEQ ID NO: 335 / SEQ ID NO: 5871, SEQ ID NO: 337 / SEQ ID NO: 5872,
SEQ ID NO: 338 / SEQ ID NO: 5873, SEQ ID NO: 339 / SEQ ID NO: 5874, SEQ ID NO: 340 /
SEQ ID NO: 5875, SEQ ID NO: 341 / SEQ ID NO: 5876, SEQ ID NO: 342 / SEQ ID NO: 5877,
SEQ ID NO: 343 / SEQ ID NO: 5878, SEQ ID NO: 344 / SEQ ID NO: 5879, SEQ ID NO: 345 /
SEQ ID NO: 5880, SEQ ID NO: 346 / SEQ ID NO: 5881, SEQ ID NO: 347 / SEQ ID NO: 5882,
SEQ ID NO: 348 / SEQ ID NO: 5883, SEQ ID NO: 349 / SEQ ID NO: 5884, SEQ ID NO: 350 /
SEQ ID NO: 5885, SEQ ID NO: 351 / SEQ ID NO: 5886, SEQ ID NO: 352 / SEQ ID NO: 5887,
SEQ ID NO: 353 / SEQ ID NO: 5888, SEQ ID NO: 354 / SEQ ID NO: 5889, SEQ ID NO: 356 /
SEQ ID NO: 5890, SEQ ID NO: 357 / SEQ ID NO: 5891, SEQ ID NO: 358 / SEQ ID NO: 5892,

SEQ ID NO: 359 / SEQ ID NO: 5893, SEQ ID NO: 360 / SEQ ID NO: 5894, SEQ ID NO: 361 /
SEQ ID NO: 5895, SEQ ID NO: 362 / SEQ ID NO: 5896, SEQ ID NO: 363 / SEQ ID NO: 5897,
SEQ ID NO: 364 / SEQ ID NO: 5898, SEQ ID NO: 365 / SEQ ID NO: 5899, SEQ ID NO: 366 /
SEQ ID NO: 5900, SEQ ID NO: 367 / SEQ ID NO: 5901, SEQ ID NO: 368 / SEQ ID NO: 5902,
SEQ ID NO: 369 / SEQ ID NO: 5903, SEQ ID NO: 370 / SEQ ID NO: 5904, SEQ ID NO: 371 /
SEQ ID NO: 5905, SEQ ID NO: 372 / SEQ ID NO: 5906, SEQ ID NO: 373 / SEQ ID NO: 5907,
SEQ ID NO: 374 / SEQ ID NO: 5908, SEQ ID NO: 375 / SEQ ID NO: 5909, SEQ ID NO: 377 /
SEQ ID NO: 5910, SEQ ID NO: 378 / SEQ ID NO: 5911, SEQ ID NO: 379 / SEQ ID NO: 5912,
SEQ ID NO: 380 / SEQ ID NO: 5913, SEQ ID NO: 381 / SEQ ID NO: 5914, SEQ ID NO: 382 /
SEQ ID NO: 5915, SEQ ID NO: 383 / SEQ ID NO: 5916, SEQ ID NO: 384 / SEQ ID NO: 5917,
SEQ ID NO: 385 / SEQ ID NO: 5918, SEQ ID NO: 386 / SEQ ID NO: 5919, SEQ ID NO: 387 /
SEQ ID NO: 5920, SEQ ID NO: 388 / SEQ ID NO: 5921, SEQ ID NO: 389 / SEQ ID NO: 5922,
SEQ ID NO: 391 / SEQ ID NO: 5923, SEQ ID NO: 392 / SEQ ID NO: 5924, SEQ ID NO: 393 /
SEQ ID NO: 5925, SEQ ID NO: 394 / SEQ ID NO: 5926, SEQ ID NO: 395 / SEQ ID NO: 5927,
SEQ ID NO: 396 / SEQ ID NO: 5928, SEQ ID NO: 397 / SEQ ID NO: 5929, SEQ ID NO: 398 /
SEQ ID NO: 5930, SEQ ID NO: 399 / SEQ ID NO: 5931, SEQ ID NO: 400 / SEQ ID NO: 5932,
SEQ ID NO: 401 / SEQ ID NO: 5933, SEQ ID NO: 402 / SEQ ID NO: 5934, SEQ ID NO: 403 /
SEQ ID NO: 5935, SEQ ID NO: 404 / SEQ ID NO: 5936, SEQ ID NO: 405 / SEQ ID NO: 5937,
SEQ ID NO: 406 / SEQ ID NO: 5938, SEQ ID NO: 407 / SEQ ID NO: 5939, SEQ ID NO: 409 /
SEQ ID NO: 5940, SEQ ID NO: 410 / SEQ ID NO: 5941, SEQ ID NO: 411 / SEQ ID NO: 5942,
SEQ ID NO: 412 / SEQ ID NO: 5943, SEQ ID NO: 413 / SEQ ID NO: 5944, SEQ ID NO: 414 /
SEQ ID NO: 5945, SEQ ID NO: 415 / SEQ ID NO: 5946, SEQ ID NO: 416 / SEQ ID NO: 5947,
SEQ ID NO: 417 / SEQ ID NO: 5948, SEQ ID NO: 418 / SEQ ID NO: 5949, SEQ ID NO: 419 /
SEQ ID NO: 5950, SEQ ID NO: 420 / SEQ ID NO: 5951, SEQ ID NO: 421 / SEQ ID NO: 5952,
SEQ ID NO: 422 / SEQ ID NO: 5953, SEQ ID NO: 423 / SEQ ID NO: 5954, SEQ ID NO: 424 /
SEQ ID NO: 5955, SEQ ID NO: 425 / SEQ ID NO: 5956, SEQ ID NO: 426 / SEQ ID NO: 5957,
SEQ ID NO: 427 / SEQ ID NO: 5958, SEQ ID NO: 428 / SEQ ID NO: 5959, SEQ ID NO: 429 /
SEQ ID NO: 5960, SEQ ID NO: 430 / SEQ ID NO: 5961, SEQ ID NO: 431 / SEQ ID NO: 5962,
SEQ ID NO: 432 / SEQ ID NO: 5963, SEQ ID NO: 433 / SEQ ID NO: 5964, SEQ ID NO: 434 /
SEQ ID NO: 5965, SEQ ID NO: 435 / SEQ ID NO: 5966, SEQ ID NO: 436 / SEQ ID NO: 5967,
SEQ ID NO: 437 / SEQ ID NO: 5968, SEQ ID NO: 438 / SEQ ID NO: 5969, SEQ ID NO: 439 /
SEQ ID NO: 5970, SEQ ID NO: 440 / SEQ ID NO: 5971, SEQ ID NO: 441 / SEQ ID NO: 5972,
SEQ ID NO: 442 / SEQ ID NO: 5973, SEQ ID NO: 443 / SEQ ID NO: 5974, SEQ ID NO: 444 /
SEQ ID NO: 5975, SEQ ID NO: 445 / SEQ ID NO: 5976, SEQ ID NO: 446 / SEQ ID NO: 5977,
SEQ ID NO: 447 / SEQ ID NO: 5978, SEQ ID NO: 448 / SEQ ID NO: 5979, SEQ ID NO: 449 /
SEQ ID NO: 5980, SEQ ID NO: 450 / SEQ ID NO: 5981, SEQ ID NO: 451 / SEQ ID NO: 5982,
SEQ ID NO: 452 / SEQ ID NO: 5983, SEQ ID NO: 453 / SEQ ID NO: 5984, SEQ ID NO: 454 /
SEQ ID NO: 5985, SEQ ID NO: 455 / SEQ ID NO: 5986, SEQ ID NO: 457 / SEQ ID NO: 5987,
SEQ ID NO: 458 / SEQ ID NO: 5988, SEQ ID NO: 459 / SEQ ID NO: 5989, SEQ ID NO: 460 /
SEQ ID NO: 5990, SEQ ID NO: 461 / SEQ ID NO: 5991, SEQ ID NO: 462 / SEQ ID NO: 5992,
SEQ ID NO: 463 / SEQ ID NO: 5993, SEQ ID NO: 464 / SEQ ID NO: 5994, SEQ ID NO: 465 /
SEQ ID NO: 5995, SEQ ID NO: 466 / SEQ ID NO: 5996, SEQ ID NO: 467 / SEQ ID NO: 5997,
SEQ ID NO: 468 / SEQ ID NO: 5998, SEQ ID NO: 469 / SEQ ID NO: 5999, SEQ ID NO: 470 /
SEQ ID NO: 6000, SEQ ID NO: 471 / SEQ ID NO: 6001, SEQ ID NO: 473 / SEQ ID NO: 6002,
SEQ ID NO: 474 / SEQ ID NO: 6003, SEQ ID NO: 475 / SEQ ID NO: 6004, SEQ ID NO: 476 /
SEQ ID NO: 6005, SEQ ID NO: 477 / SEQ ID NO: 6006, SEQ ID NO: 478 / SEQ ID NO: 6007,
SEQ ID NO: 479 / SEQ ID NO: 6008, SEQ ID NO: 480 / SEQ ID NO: 6009, SEQ ID NO: 481 /
SEQ ID NO: 6010, SEQ ID NO: 482 / SEQ ID NO: 6011, SEQ ID NO: 483 / SEQ ID NO: 6012,
SEQ ID NO: 484 / SEQ ID NO: 6013, SEQ ID NO: 485 / SEQ ID NO: 6014, SEQ ID NO: 486 /
SEQ ID NO: 6015, SEQ ID NO: 487 / SEQ ID NO: 6016, SEQ ID NO: 488 / SEQ ID NO: 6017,
SEQ ID NO: 489 / SEQ ID NO: 6018, SEQ ID NO: 490 / SEQ ID NO: 6019, SEQ ID NO: 492 /
SEQ ID NO: 6020, SEQ ID NO: 493 / SEQ ID NO: 6021, SEQ ID NO: 494 / SEQ ID NO: 6022,

SEQ ID NO: 495 / SEQ ID NO: 6023, SEQ ID NO: 497 / SEQ ID NO: 6024, SEQ ID NO: 498 /
SEQ ID NO: 6025, SEQ ID NO: 499 / SEQ ID NO: 6026, SEQ ID NO: 500 / SEQ ID NO: 6027,
SEQ ID NO: 501 / SEQ ID NO: 6028, SEQ ID NO: 502 / SEQ ID NO: 6029, SEQ ID NO: 503 /
SEQ ID NO: 6030, SEQ ID NO: 504 / SEQ ID NO: 6031, SEQ ID NO: 505 / SEQ ID NO: 6032,
SEQ ID NO: 506 / SEQ ID NO: 6033, SEQ ID NO: 507 / SEQ ID NO: 6034, SEQ ID NO: 508 /
SEQ ID NO: 6035, SEQ ID NO: 509 / SEQ ID NO: 6036, SEQ ID NO: 510 / SEQ ID NO: 6037,
SEQ ID NO: 511 / SEQ ID NO: 6038, SEQ ID NO: 512 / SEQ ID NO: 6039, SEQ ID NO: 513 /
SEQ ID NO: 6040, SEQ ID NO: 514 / SEQ ID NO: 6041, SEQ ID NO: 515 / SEQ ID NO: 6042,
SEQ ID NO: 516 / SEQ ID NO: 6043, SEQ ID NO: 517 / SEQ ID NO: 6044, SEQ ID NO: 518 /
SEQ ID NO: 6045, SEQ ID NO: 519 / SEQ ID NO: 6046, SEQ ID NO: 520 / SEQ ID NO: 6047,
SEQ ID NO: 521 / SEQ ID NO: 6048, SEQ ID NO: 522 / SEQ ID NO: 6049, SEQ ID NO: 523 /
SEQ ID NO: 6050, SEQ ID NO: 524 / SEQ ID NO: 6051, SEQ ID NO: 525 / SEQ ID NO: 6052,
SEQ ID NO: 526 / SEQ ID NO: 6053, SEQ ID NO: 527 / SEQ ID NO: 6054, SEQ ID NO: 528 /
SEQ ID NO: 6055, SEQ ID NO: 529 / SEQ ID NO: 6056, SEQ ID NO: 530 / SEQ ID NO: 6057,
SEQ ID NO: 531 / SEQ ID NO: 6058, SEQ ID NO: 532 / SEQ ID NO: 6059, SEQ ID NO: 533 /
SEQ ID NO: 6060, SEQ ID NO: 534 / SEQ ID NO: 6061, SEQ ID NO: 535 / SEQ ID NO: 6062,
SEQ ID NO: 536 / SEQ ID NO: 6063, SEQ ID NO: 537 / SEQ ID NO: 6064, SEQ ID NO: 538 /
SEQ ID NO: 6065, SEQ ID NO: 539 / SEQ ID NO: 6066, SEQ ID NO: 540 / SEQ ID NO: 6067,
SEQ ID NO: 541 / SEQ ID NO: 6068, SEQ ID NO: 542 / SEQ ID NO: 6069, SEQ ID NO: 543 /
SEQ ID NO: 6070, SEQ ID NO: 544 / SEQ ID NO: 6071, SEQ ID NO: 545 / SEQ ID NO: 6072,
SEQ ID NO: 546 / SEQ ID NO: 6073, SEQ ID NO: 547 / SEQ ID NO: 6074, SEQ ID NO: 548 /
SEQ ID NO: 6075, SEQ ID NO: 549 / SEQ ID NO: 6076, SEQ ID NO: 550 / SEQ ID NO: 6077,
SEQ ID NO: 551 / SEQ ID NO: 6078, SEQ ID NO: 553 / SEQ ID NO: 6079, SEQ ID NO: 554 /
SEQ ID NO: 6080, SEQ ID NO: 555 / SEQ ID NO: 6081, SEQ ID NO: 556 / SEQ ID NO: 6082,
SEQ ID NO: 557 / SEQ ID NO: 6083, SEQ ID NO: 558 / SEQ ID NO: 6084, SEQ ID NO: 559 /
SEQ ID NO: 6085, SEQ ID NO: 560 / SEQ ID NO: 6086, SEQ ID NO: 561 / SEQ ID NO: 6087,
SEQ ID NO: 562 / SEQ ID NO: 6088, SEQ ID NO: 563 / SEQ ID NO: 6089, SEQ ID NO: 564 /
SEQ ID NO: 6090, SEQ ID NO: 565 / SEQ ID NO: 6091, SEQ ID NO: 566 / SEQ ID NO: 6092,
SEQ ID NO: 567 / SEQ ID NO: 6093, SEQ ID NO: 568 / SEQ ID NO: 6094, SEQ ID NO: 569 /
SEQ ID NO: 6095, SEQ ID NO: 570 / SEQ ID NO: 6096, SEQ ID NO: 571 / SEQ ID NO: 6097,
SEQ ID NO: 572 / SEQ ID NO: 6098, SEQ ID NO: 573 / SEQ ID NO: 6099, SEQ ID NO: 574 /
SEQ ID NO: 6100, SEQ ID NO: 575 / SEQ ID NO: 6101, SEQ ID NO: 576 / SEQ ID NO: 6102,
SEQ ID NO: 577 / SEQ ID NO: 6103, SEQ ID NO: 578 / SEQ ID NO: 6104, SEQ ID NO: 579 /
SEQ ID NO: 6105, SEQ ID NO: 580 / SEQ ID NO: 6106, SEQ ID NO: 581 / SEQ ID NO: 6107,
SEQ ID NO: 582 / SEQ ID NO: 6108, SEQ ID NO: 583 / SEQ ID NO: 6109, SEQ ID NO: 584 /
SEQ ID NO: 6110, SEQ ID NO: 585 / SEQ ID NO: 6111, SEQ ID NO: 586 / SEQ ID NO: 6112,
SEQ ID NO: 587 / SEQ ID NO: 6113, SEQ ID NO: 588 / SEQ ID NO: 6114, SEQ ID NO: 589 /
SEQ ID NO: 6115, SEQ ID NO: 590 / SEQ ID NO: 6116, SEQ ID NO: 591 / SEQ ID NO: 6117,
SEQ ID NO: 593 / SEQ ID NO: 6118, SEQ ID NO: 594 / SEQ ID NO: 6119, SEQ ID NO: 595 /
SEQ ID NO: 6120, SEQ ID NO: 596 / SEQ ID NO: 6121, SEQ ID NO: 597 / SEQ ID NO: 6122,
SEQ ID NO: 598 / SEQ ID NO: 6123, SEQ ID NO: 599 / SEQ ID NO: 6124, SEQ ID NO: 600 /
SEQ ID NO: 6125, SEQ ID NO: 601 / SEQ ID NO: 6126, SEQ ID NO: 602 / SEQ ID NO: 6127,
SEQ ID NO: 603 / SEQ ID NO: 6128, SEQ ID NO: 604 / SEQ ID NO: 6129, SEQ ID NO: 605 /
SEQ ID NO: 6130, SEQ ID NO: 606 / SEQ ID NO: 6131, SEQ ID NO: 607 / SEQ ID NO: 6132,
SEQ ID NO: 608 / SEQ ID NO: 6133, SEQ ID NO: 609 / SEQ ID NO: 6134, SEQ ID NO: 610 /
SEQ ID NO: 6135, SEQ ID NO: 611 / SEQ ID NO: 6136, SEQ ID NO: 612 / SEQ ID NO: 6137,
SEQ ID NO: 613 / SEQ ID NO: 6138, SEQ ID NO: 614 / SEQ ID NO: 6139, SEQ ID NO: 615 /
SEQ ID NO: 6140, SEQ ID NO: 617 / SEQ ID NO: 6141, SEQ ID NO: 618 / SEQ ID NO: 6142,
SEQ ID NO: 619 / SEQ ID NO: 6143, SEQ ID NO: 620 / SEQ ID NO: 6144, SEQ ID NO: 621 /
SEQ ID NO: 6145, SEQ ID NO: 622 / SEQ ID NO: 6146, SEQ ID NO: 623 / SEQ ID NO: 6147,
SEQ ID NO: 624 / SEQ ID NO: 6148, SEQ ID NO: 625 / SEQ ID NO: 6149, SEQ ID NO: 626 /
SEQ ID NO: 6150, SEQ ID NO: 627 / SEQ ID NO: 6151, SEQ ID NO: 628 / SEQ ID NO: 6152,

SEQ ID NO: 629 / SEQ ID NO: 6153, SEQ ID NO: 630 / SEQ ID NO: 6154, SEQ ID NO: 631 /
SEQ ID NO: 6155, SEQ ID NO: 632 / SEQ ID NO: 6156, SEQ ID NO: 633 / SEQ ID NO: 6157,
SEQ ID NO: 634 / SEQ ID NO: 6158, SEQ ID NO: 635 / SEQ ID NO: 6159, SEQ ID NO: 636 /
SEQ ID NO: 6160, SEQ ID NO: 637 / SEQ ID NO: 6161, SEQ ID NO: 638 / SEQ ID NO: 6162,
SEQ ID NO: 639 / SEQ ID NO: 6163, SEQ ID NO: 640 / SEQ ID NO: 6164, SEQ ID NO: 641 /
SEQ ID NO: 6165, SEQ ID NO: 642 / SEQ ID NO: 6166, SEQ ID NO: 643 / SEQ ID NO: 6167,
SEQ ID NO: 644 / SEQ ID NO: 6168, SEQ ID NO: 645 / SEQ ID NO: 6169, SEQ ID NO: 646 /
SEQ ID NO: 6170, SEQ ID NO: 648 / SEQ ID NO: 6171, SEQ ID NO: 649 / SEQ ID NO: 6172,
SEQ ID NO: 650 / SEQ ID NO: 6173, SEQ ID NO: 651 / SEQ ID NO: 6174, SEQ ID NO: 652 /
SEQ ID NO: 6175, SEQ ID NO: 653 / SEQ ID NO: 6176, SEQ ID NO: 655 / SEQ ID NO: 6177,
SEQ ID NO: 656 / SEQ ID NO: 6178, SEQ ID NO: 657 / SEQ ID NO: 6179, SEQ ID NO: 658 /
SEQ ID NO: 6180, SEQ ID NO: 659 / SEQ ID NO: 6181, SEQ ID NO: 660 / SEQ ID NO: 6182,
SEQ ID NO: 662 / SEQ ID NO: 6183, SEQ ID NO: 663 / SEQ ID NO: 6184, SEQ ID NO: 664 /
SEQ ID NO: 6185, SEQ ID NO: 665 / SEQ ID NO: 6186, SEQ ID NO: 666 / SEQ ID NO: 6187,
SEQ ID NO: 667 / SEQ ID NO: 6188, SEQ ID NO: 668 / SEQ ID NO: 6189, SEQ ID NO: 669 /
SEQ ID NO: 6190, SEQ ID NO: 670 / SEQ ID NO: 6191, SEQ ID NO: 671 / SEQ ID NO: 6192,
SEQ ID NO: 672 / SEQ ID NO: 6193, SEQ ID NO: 673 / SEQ ID NO: 6194, SEQ ID NO: 674 /
SEQ ID NO: 6195, SEQ ID NO: 675 / SEQ ID NO: 6196, SEQ ID NO: 676 / SEQ ID NO: 6197,
SEQ ID NO: 677 / SEQ ID NO: 6198, SEQ ID NO: 678 / SEQ ID NO: 6199, SEQ ID NO: 679 /
SEQ ID NO: 6200, SEQ ID NO: 680 / SEQ ID NO: 6201, SEQ ID NO: 681 / SEQ ID NO: 6202,
SEQ ID NO: 682 / SEQ ID NO: 6203, SEQ ID NO: 683 / SEQ ID NO: 6204, SEQ ID NO: 684 /
SEQ ID NO: 6205, SEQ ID NO: 685 / SEQ ID NO: 6206, SEQ ID NO: 686 / SEQ ID NO: 6207,
SEQ ID NO: 687 / SEQ ID NO: 6208, SEQ ID NO: 688 / SEQ ID NO: 6209, SEQ ID NO: 689 /
SEQ ID NO: 6210, SEQ ID NO: 690 / SEQ ID NO: 6211, SEQ ID NO: 691 / SEQ ID NO: 6212,
SEQ ID NO: 692 / SEQ ID NO: 6213, SEQ ID NO: 693 / SEQ ID NO: 6214, SEQ ID NO: 694 /
SEQ ID NO: 6215, SEQ ID NO: 695 / SEQ ID NO: 6216, SEQ ID NO: 696 / SEQ ID NO: 6217,
SEQ ID NO: 697 / SEQ ID NO: 6218, SEQ ID NO: 698 / SEQ ID NO: 6219, SEQ ID NO: 699 /
SEQ ID NO: 6220, SEQ ID NO: 700 / SEQ ID NO: 6221, SEQ ID NO: 701 / SEQ ID NO: 6222,
SEQ ID NO: 702 / SEQ ID NO: 6223, SEQ ID NO: 703 / SEQ ID NO: 6224, SEQ ID NO: 704 /
SEQ ID NO: 6225, SEQ ID NO: 705 / SEQ ID NO: 6226, SEQ ID NO: 706 / SEQ ID NO: 6227,
SEQ ID NO: 707 / SEQ ID NO: 6228, SEQ ID NO: 708 / SEQ ID NO: 6229, SEQ ID NO: 709 /
SEQ ID NO: 6230, SEQ ID NO: 710 / SEQ ID NO: 6231, SEQ ID NO: 711 / SEQ ID NO: 6232,
SEQ ID NO: 712 / SEQ ID NO: 6233, SEQ ID NO: 713 / SEQ ID NO: 6234, SEQ ID NO: 714 /
SEQ ID NO: 6235, SEQ ID NO: 715 / SEQ ID NO: 6236, SEQ ID NO: 716 / SEQ ID NO: 6237,
SEQ ID NO: 717 / SEQ ID NO: 6238, SEQ ID NO: 718 / SEQ ID NO: 6239, SEQ ID NO: 719 /
SEQ ID NO: 6240, SEQ ID NO: 720 / SEQ ID NO: 6241, SEQ ID NO: 721 / SEQ ID NO: 6242,
SEQ ID NO: 722 / SEQ ID NO: 6243, SEQ ID NO: 723 / SEQ ID NO: 6244, SEQ ID NO: 724 /
SEQ ID NO: 6245, SEQ ID NO: 725 / SEQ ID NO: 6246, SEQ ID NO: 726 / SEQ ID NO: 6247,
SEQ ID NO: 727 / SEQ ID NO: 6248, SEQ ID NO: 728 / SEQ ID NO: 6249, SEQ ID NO: 729 /
SEQ ID NO: 6250, SEQ ID NO: 730 / SEQ ID NO: 6251, SEQ ID NO: 731 / SEQ ID NO: 6252,
SEQ ID NO: 732 / SEQ ID NO: 6253, SEQ ID NO: 733 / SEQ ID NO: 6254, SEQ ID NO: 734 /
SEQ ID NO: 6255, SEQ ID NO: 735 / SEQ ID NO: 6256, SEQ ID NO: 736 / SEQ ID NO: 6257,
SEQ ID NO: 737 / SEQ ID NO: 6258, SEQ ID NO: 738 / SEQ ID NO: 6259, SEQ ID NO: 739 /
SEQ ID NO: 6260, SEQ ID NO: 740 / SEQ ID NO: 6261, SEQ ID NO: 741 / SEQ ID NO: 6262,
SEQ ID NO: 742 / SEQ ID NO: 6263, SEQ ID NO: 743 / SEQ ID NO: 6264, SEQ ID NO: 744 /
SEQ ID NO: 6265, SEQ ID NO: 745 / SEQ ID NO: 6266, SEQ ID NO: 746 / SEQ ID NO: 6267,
SEQ ID NO: 747 / SEQ ID NO: 6268, SEQ ID NO: 748 / SEQ ID NO: 6269, SEQ ID NO: 749 /
SEQ ID NO: 6270, SEQ ID NO: 751 / SEQ ID NO: 6271, SEQ ID NO: 752 / SEQ ID NO: 6272,
SEQ ID NO: 753 / SEQ ID NO: 6273, SEQ ID NO: 754 / SEQ ID NO: 6274, SEQ ID NO: 755 /
SEQ ID NO: 6275, SEQ ID NO: 756 / SEQ ID NO: 6276, SEQ ID NO: 757 / SEQ ID NO: 6277,
SEQ ID NO: 758 / SEQ ID NO: 6278, SEQ ID NO: 759 / SEQ ID NO: 6279, SEQ ID NO: 760 /
SEQ ID NO: 6280, SEQ ID NO: 761 / SEQ ID NO: 6281, SEQ ID NO: 762 / SEQ ID NO: 6282,

SEQ ID NO: 763 / SEQ ID NO: 6283, SEQ ID NO: 764 / SEQ ID NO: 6284, SEQ ID NO: 765 /
SEQ ID NO: 6285, SEQ ID NO: 766 / SEQ ID NO: 6286, SEQ ID NO: 767 / SEQ ID NO: 6287,
SEQ ID NO: 769 / SEQ ID NO: 6288, SEQ ID NO: 770 / SEQ ID NO: 6289, SEQ ID NO: 771 /
SEQ ID NO: 6290, SEQ ID NO: 772 / SEQ ID NO: 6291, SEQ ID NO: 773 / SEQ ID NO: 6292,
SEQ ID NO: 775 / SEQ ID NO: 6293, SEQ ID NO: 776 / SEQ ID NO: 6294, SEQ ID NO: 777 /
SEQ ID NO: 6295, SEQ ID NO: 778 / SEQ ID NO: 6296, SEQ ID NO: 779 / SEQ ID NO: 6297,
SEQ ID NO: 780 / SEQ ID NO: 6298, SEQ ID NO: 781 / SEQ ID NO: 6299, SEQ ID NO: 782 /
SEQ ID NO: 6300, SEQ ID NO: 783 / SEQ ID NO: 6301, SEQ ID NO: 784 / SEQ ID NO: 6302,
SEQ ID NO: 785 / SEQ ID NO: 6303, SEQ ID NO: 786 / SEQ ID NO: 6304, SEQ ID NO: 787 /
SEQ ID NO: 6305, SEQ ID NO: 789 / SEQ ID NO: 6306, SEQ ID NO: 790 / SEQ ID NO: 6307,
SEQ ID NO: 791 / SEQ ID NO: 6308, SEQ ID NO: 792 / SEQ ID NO: 6309, SEQ ID NO: 793 /
SEQ ID NO: 6310, SEQ ID NO: 794 / SEQ ID NO: 6311, SEQ ID NO: 796 / SEQ ID NO: 6312,
SEQ ID NO: 797 / SEQ ID NO: 6313, SEQ ID NO: 798 / SEQ ID NO: 6314, SEQ ID NO: 799 /
SEQ ID NO: 6315, SEQ ID NO: 800 / SEQ ID NO: 6316, SEQ ID NO: 801 / SEQ ID NO: 6317,
SEQ ID NO: 802 / SEQ ID NO: 6318, SEQ ID NO: 803 / SEQ ID NO: 6319, SEQ ID NO: 804 /
SEQ ID NO: 6320, SEQ ID NO: 805 / SEQ ID NO: 6321, SEQ ID NO: 806 / SEQ ID NO: 6322,
SEQ ID NO: 807 / SEQ ID NO: 6323, SEQ ID NO: 808 / SEQ ID NO: 6324, SEQ ID NO: 809 /
SEQ ID NO: 6325, SEQ ID NO: 810 / SEQ ID NO: 6326, SEQ ID NO: 811 / SEQ ID NO: 6327,
SEQ ID NO: 812 / SEQ ID NO: 6328, SEQ ID NO: 813 / SEQ ID NO: 6329, SEQ ID NO: 814 /
SEQ ID NO: 6330, SEQ ID NO: 815 / SEQ ID NO: 6331, SEQ ID NO: 816 / SEQ ID NO: 6332,
SEQ ID NO: 817 / SEQ ID NO: 6333, SEQ ID NO: 818 / SEQ ID NO: 6334, SEQ ID NO: 819 /
SEQ ID NO: 6335, SEQ ID NO: 820 / SEQ ID NO: 6336, SEQ ID NO: 821 / SEQ ID NO: 6337,
SEQ ID NO: 822 / SEQ ID NO: 6338, SEQ ID NO: 823 / SEQ ID NO: 6339, SEQ ID NO: 824 /
SEQ ID NO: 6340, SEQ ID NO: 825 / SEQ ID NO: 6341, SEQ ID NO: 826 / SEQ ID NO: 6342,
SEQ ID NO: 827 / SEQ ID NO: 6343, SEQ ID NO: 828 / SEQ ID NO: 6344, SEQ ID NO: 829 /
SEQ ID NO: 6345, SEQ ID NO: 830 / SEQ ID NO: 6346, SEQ ID NO: 831 / SEQ ID NO: 6347,
SEQ ID NO: 832 / SEQ ID NO: 6348, SEQ ID NO: 833 / SEQ ID NO: 6349, SEQ ID NO: 834 /
SEQ ID NO: 6350, SEQ ID NO: 835 / SEQ ID NO: 6351, SEQ ID NO: 836 / SEQ ID NO: 6352,
SEQ ID NO: 837 / SEQ ID NO: 6353, SEQ ID NO: 838 / SEQ ID NO: 6354, SEQ ID NO: 839 /
SEQ ID NO: 6355, SEQ ID NO: 840 / SEQ ID NO: 6356, SEQ ID NO: 841 / SEQ ID NO: 6357,
SEQ ID NO: 842 / SEQ ID NO: 6358, SEQ ID NO: 843 / SEQ ID NO: 6359, SEQ ID NO: 844 /
SEQ ID NO: 6360, SEQ ID NO: 845 / SEQ ID NO: 6361, SEQ ID NO: 846 / SEQ ID NO: 6362,
SEQ ID NO: 847 / SEQ ID NO: 6363, SEQ ID NO: 848 / SEQ ID NO: 6364, SEQ ID NO: 849 /
SEQ ID NO: 6365, SEQ ID NO: 850 / SEQ ID NO: 6366, SEQ ID NO: 851 / SEQ ID NO: 6367,
SEQ ID NO: 852 / SEQ ID NO: 6368, SEQ ID NO: 853 / SEQ ID NO: 6369, SEQ ID NO: 854 /
SEQ ID NO: 6370, SEQ ID NO: 855 / SEQ ID NO: 6371, SEQ ID NO: 856 / SEQ ID NO: 6372,
SEQ ID NO: 857 / SEQ ID NO: 6373, SEQ ID NO: 858 / SEQ ID NO: 6374, SEQ ID NO: 859 /
SEQ ID NO: 6375, SEQ ID NO: 860 / SEQ ID NO: 6376, SEQ ID NO: 861 / SEQ ID NO: 6377,
SEQ ID NO: 862 / SEQ ID NO: 6378, SEQ ID NO: 863 / SEQ ID NO: 6379, SEQ ID NO: 864 /
SEQ ID NO: 6380, SEQ ID NO: 865 / SEQ ID NO: 6381, SEQ ID NO: 866 / SEQ ID NO: 6382,
SEQ ID NO: 867 / SEQ ID NO: 6383, SEQ ID NO: 868 / SEQ ID NO: 6384, SEQ ID NO: 869 /
SEQ ID NO: 6385, SEQ ID NO: 870 / SEQ ID NO: 6386, SEQ ID NO: 871 / SEQ ID NO: 6387,
SEQ ID NO: 872 / SEQ ID NO: 6388, SEQ ID NO: 873 / SEQ ID NO: 6389, SEQ ID NO: 874 /
SEQ ID NO: 6390, SEQ ID NO: 875 / SEQ ID NO: 6391, SEQ ID NO: 876 / SEQ ID NO: 6392,
SEQ ID NO: 877 / SEQ ID NO: 6393, SEQ ID NO: 878 / SEQ ID NO: 6394, SEQ ID NO: 879 /
SEQ ID NO: 6395, SEQ ID NO: 880 / SEQ ID NO: 6396, SEQ ID NO: 881 / SEQ ID NO: 6397,
SEQ ID NO: 882 / SEQ ID NO: 6398, SEQ ID NO: 883 / SEQ ID NO: 6399, SEQ ID NO: 884 /
SEQ ID NO: 6400, SEQ ID NO: 885 / SEQ ID NO: 6401, SEQ ID NO: 886 / SEQ ID NO: 6402,
SEQ ID NO: 887 / SEQ ID NO: 6403, SEQ ID NO: 888 / SEQ ID NO: 6404, SEQ ID NO: 889 /
SEQ ID NO: 6405, SEQ ID NO: 890 / SEQ ID NO: 6406, SEQ ID NO: 891 / SEQ ID NO: 6407,
SEQ ID NO: 892 / SEQ ID NO: 6408, SEQ ID NO: 893 / SEQ ID NO: 6409, SEQ ID NO: 894 /
SEQ ID NO: 6410, SEQ ID NO: 895 / SEQ ID NO: 6411, SEQ ID NO: 896 / SEQ ID NO: 6412,

SEQ ID NO: 897 / SEQ ID NO: 6413, SEQ ID NO: 898 / SEQ ID NO: 6414, SEQ ID NO: 899 /
SEQ ID NO: 6415, SEQ ID NO: 900 / SEQ ID NO: 6416, SEQ ID NO: 901 / SEQ ID NO: 6417,
SEQ ID NO: 902 / SEQ ID NO: 6418, SEQ ID NO: 903 / SEQ ID NO: 6419, SEQ ID NO: 904 /
SEQ ID NO: 6420, SEQ ID NO: 905 / SEQ ID NO: 6421, SEQ ID NO: 906 / SEQ ID NO: 6422,
SEQ ID NO: 907 / SEQ ID NO: 6423, SEQ ID NO: 908 / SEQ ID NO: 6424, SEQ ID NO: 909 /
SEQ ID NO: 6425, SEQ ID NO: 910 / SEQ ID NO: 6426, SEQ ID NO: 911 / SEQ ID NO: 6427,
SEQ ID NO: 912 / SEQ ID NO: 6428, SEQ ID NO: 913 / SEQ ID NO: 6429, SEQ ID NO: 914 /
SEQ ID NO: 6430, SEQ ID NO: 915 / SEQ ID NO: 6431, SEQ ID NO: 916 / SEQ ID NO: 6432,
SEQ ID NO: 917 / SEQ ID NO: 6433, SEQ ID NO: 918 / SEQ ID NO: 6434, SEQ ID NO: 919 /
SEQ ID NO: 6435, SEQ ID NO: 920 / SEQ ID NO: 6436, SEQ ID NO: 921 / SEQ ID NO: 6437,
SEQ ID NO: 922 / SEQ ID NO: 6438, SEQ ID NO: 923 / SEQ ID NO: 6439, SEQ ID NO: 924 /
SEQ ID NO: 6440, SEQ ID NO: 925 / SEQ ID NO: 6441, SEQ ID NO: 926 / SEQ ID NO: 6442,
SEQ ID NO: 927 / SEQ ID NO: 6443, SEQ ID NO: 928 / SEQ ID NO: 6444, SEQ ID NO: 929 /
SEQ ID NO: 6445, SEQ ID NO: 930 / SEQ ID NO: 6446, SEQ ID NO: 931 / SEQ ID NO: 6447,
SEQ ID NO: 932 / SEQ ID NO: 6448, SEQ ID NO: 933 / SEQ ID NO: 6449, SEQ ID NO: 934 /
SEQ ID NO: 6450, SEQ ID NO: 935 / SEQ ID NO: 6451, SEQ ID NO: 936 / SEQ ID NO: 6452,
SEQ ID NO: 937 / SEQ ID NO: 6453, SEQ ID NO: 938 / SEQ ID NO: 6454, SEQ ID NO: 939 /
SEQ ID NO: 6455, SEQ ID NO: 940 / SEQ ID NO: 6456, SEQ ID NO: 941 / SEQ ID NO: 6457,
SEQ ID NO: 942 / SEQ ID NO: 6458, SEQ ID NO: 943 / SEQ ID NO: 6459, SEQ ID NO: 944 /
SEQ ID NO: 6460, SEQ ID NO: 945 / SEQ ID NO: 6461, SEQ ID NO: 946 / SEQ ID NO: 6462,
SEQ ID NO: 947 / SEQ ID NO: 6463, SEQ ID NO: 949 / SEQ ID NO: 6464, SEQ ID NO: 950 /
SEQ ID NO: 6465, SEQ ID NO: 951 / SEQ ID NO: 6466, SEQ ID NO: 952 / SEQ ID NO: 6467,
SEQ ID NO: 953 / SEQ ID NO: 6468, SEQ ID NO: 954 / SEQ ID NO: 6469, SEQ ID NO: 955 /
SEQ ID NO: 6470, SEQ ID NO: 956 / SEQ ID NO: 6471, SEQ ID NO: 957 / SEQ ID NO: 6472,
SEQ ID NO: 958 / SEQ ID NO: 6473, SEQ ID NO: 959 / SEQ ID NO: 6474, SEQ ID NO: 960 /
SEQ ID NO: 6475, SEQ ID NO: 961 / SEQ ID NO: 6476, SEQ ID NO: 962 / SEQ ID NO: 6477,
SEQ ID NO: 963 / SEQ ID NO: 6478, SEQ ID NO: 964 / SEQ ID NO: 6479, SEQ ID NO: 965 /
SEQ ID NO: 6480, SEQ ID NO: 966 / SEQ ID NO: 6481, SEQ ID NO: 967 / SEQ ID NO: 6482,
SEQ ID NO: 968 / SEQ ID NO: 6483, SEQ ID NO: 969 / SEQ ID NO: 6484, SEQ ID NO: 970 /
SEQ ID NO: 6485, SEQ ID NO: 971 / SEQ ID NO: 6486, SEQ ID NO: 972 / SEQ ID NO: 6487,
SEQ ID NO: 973 / SEQ ID NO: 6488, SEQ ID NO: 974 / SEQ ID NO: 6489, SEQ ID NO: 975 /
SEQ ID NO: 6490, SEQ ID NO: 976 / SEQ ID NO: 6491, SEQ ID NO: 978 / SEQ ID NO: 6492,
SEQ ID NO: 979 / SEQ ID NO: 6493, SEQ ID NO: 980 / SEQ ID NO: 6494, SEQ ID NO: 981 /
SEQ ID NO: 6495, SEQ ID NO: 982 / SEQ ID NO: 6496, SEQ ID NO: 983 / SEQ ID NO: 6497,
SEQ ID NO: 984 / SEQ ID NO: 6498, SEQ ID NO: 985 / SEQ ID NO: 6499, SEQ ID NO: 986 /
SEQ ID NO: 6500, SEQ ID NO: 987 / SEQ ID NO: 6501, SEQ ID NO: 988 / SEQ ID NO: 6502,
SEQ ID NO: 989 / SEQ ID NO: 6503, SEQ ID NO: 990 / SEQ ID NO: 6504, SEQ ID NO: 991 /
SEQ ID NO: 6505, SEQ ID NO: 992 / SEQ ID NO: 6506, SEQ ID NO: 993 / SEQ ID NO: 6507,
SEQ ID NO: 994 / SEQ ID NO: 6508, SEQ ID NO: 995 / SEQ ID NO: 6509, SEQ ID NO: 996 /
SEQ ID NO: 6510, SEQ ID NO: 997 / SEQ ID NO: 6511, SEQ ID NO: 998 / SEQ ID NO: 6512,
SEQ ID NO: 1000 / SEQ ID NO: 6513, SEQ ID NO: 1001 / SEQ ID NO: 6514, SEQ ID NO:
1002 / SEQ ID NO: 6515, SEQ ID NO: 1003 / SEQ ID NO: 6516, SEQ ID NO: 1005 / SEQ ID
NO: 6517, SEQ ID NO: 1006 / SEQ ID NO: 6518, SEQ ID NO: 1007 / SEQ ID NO: 6519, SEQ
ID NO: 1008 / SEQ ID NO: 6520, SEQ ID NO: 1009 / SEQ ID NO: 6521, SEQ ID NO: 1010 /
SEQ ID NO: 6522, SEQ ID NO: 1011 / SEQ ID NO: 6523, SEQ ID NO: 1012 / SEQ ID NO:
6524, SEQ ID NO: 1013 / SEQ ID NO: 6525, SEQ ID NO: 1014 / SEQ ID NO: 6526, SEQ ID
NO: 1016 / SEQ ID NO: 6527, SEQ ID NO: 1017 / SEQ ID NO: 6528, SEQ ID NO: 1018 /
SEQ ID NO: 6529, SEQ ID NO: 1019 / SEQ ID NO: 6530, SEQ ID NO: 1020 / SEQ ID NO:
6531, SEQ ID NO: 1021 / SEQ ID NO: 6532, SEQ ID NO: 1022 / SEQ ID NO: 6533, SEQ ID
NO: 1023 / SEQ ID NO: 6534, SEQ ID NO: 1024 / SEQ ID NO: 6535, SEQ ID NO: 1025 /
SEQ ID NO: 6536, SEQ ID NO: 1026 / SEQ ID NO: 6537, SEQ ID NO: 1027 / SEQ ID NO:
6538, SEQ ID NO: 1028 / SEQ ID NO: 6539, SEQ ID NO: 1029 / SEQ ID NO: 6540, SEQ ID

NO: 1030 / SEQ ID NO: 6541, SEQ ID NO: 1031 / SEQ ID NO: 6542, SEQ ID NO: 1032 /
SEQ ID NO: 6543, SEQ ID NO: 1033 / SEQ ID NO: 6544, SEQ ID NO: 1034 / SEQ ID NO:
6545, SEQ ID NO: 1035 / SEQ ID NO: 6546, SEQ ID NO: 1036 / SEQ ID NO: 6547,
SEQ ID NO: 1037 / SEQ ID NO: 6548, SEQ ID NO: 1038 / SEQ ID NO: 6549, SEQ ID NO:
1039 / SEQ ID NO: 6550, SEQ ID NO: 1040 / SEQ ID NO: 6551, SEQ ID NO: 1041 / SEQ ID
NO: 6552, SEQ ID NO: 1042 / SEQ ID NO: 6553, SEQ ID NO: 1043 / SEQ ID NO: 6554, SEQ
ID NO: 1044 / SEQ ID NO: 6555, SEQ ID NO: 1045 / SEQ ID NO: 6556, SEQ ID NO: 1046 /
SEQ ID NO: 6557, SEQ ID NO: 1047 / SEQ ID NO: 6558, SEQ ID NO: 1048 / SEQ ID NO:
6559, SEQ ID NO: 1049 / SEQ ID NO: 6560, SEQ ID NO: 1050 / SEQ ID NO: 6561, SEQ ID
NO: 1051 / SEQ ID NO: 6562, SEQ ID NO: 1052 / SEQ ID NO: 6563, SEQ ID NO: 1053 /
SEQ ID NO: 6564, SEQ ID NO: 1054 / SEQ ID NO: 6565, SEQ ID NO: 1055 / SEQ ID NO:
6566, SEQ ID NO: 1056 / SEQ ID NO: 6567, SEQ ID NO: 1057 / SEQ ID NO: 6568, SEQ ID
NO: 1058 / SEQ ID NO: 6569, SEQ ID NO: 1059 / SEQ ID NO: 6570, SEQ ID NO: 1060 /
SEQ ID NO: 6571, SEQ ID NO: 1061 / SEQ ID NO: 6572, SEQ ID NO: 1062 / SEQ ID NO:
6573, SEQ ID NO: 1063 / SEQ ID NO: 6574, SEQ ID NO: 1064 / SEQ ID NO: 6575, SEQ ID
NO: 1065 / SEQ ID NO: 6576, SEQ ID NO: 1066 / SEQ ID NO: 6577, SEQ ID NO: 1067 /
SEQ ID NO: 6578, SEQ ID NO: 1068 / SEQ ID NO: 6579, SEQ ID NO: 1069 / SEQ ID NO:
6580, SEQ ID NO: 1070 / SEQ ID NO: 6581, SEQ ID NO: 1071 / SEQ ID NO: 6582, SEQ ID
NO: 1072 / SEQ ID NO: 6583, SEQ ID NO: 1073 / SEQ ID NO: 6584, SEQ ID NO: 1074 /
SEQ ID NO: 6585, SEQ ID NO: 1075 / SEQ ID NO: 6586, SEQ ID NO: 1076 / SEQ ID NO:
6587, SEQ ID NO: 1077 / SEQ ID NO: 6588, SEQ ID NO: 1078 / SEQ ID NO: 6589, SEQ ID
NO: 1079 / SEQ ID NO: 6590, SEQ ID NO: 1080 / SEQ ID NO: 6591, SEQ ID NO: 1081 /
SEQ ID NO: 6592, SEQ ID NO: 1082 / SEQ ID NO: 6593, SEQ ID NO: 1083 / SEQ ID NO:
6594, SEQ ID NO: 1084 / SEQ ID NO: 6595, SEQ ID NO: 1085 / SEQ ID NO: 6596, SEQ ID
NO: 1086 / SEQ ID NO: 6597, SEQ ID NO: 1087 / SEQ ID NO: 6598, SEQ ID NO: 1088 /
SEQ ID NO: 6599, SEQ ID NO: 1089 / SEQ ID NO: 6600, SEQ ID NO: 1090 / SEQ ID NO:
6601, SEQ ID NO: 1091 / SEQ ID NO: 6602, SEQ ID NO: 1092 / SEQ ID NO: 6603, SEQ ID
NO: 1093 / SEQ ID NO: 6604, SEQ ID NO: 1094 / SEQ ID NO: 6605, SEQ ID NO: 1095 /
SEQ ID NO: 6606, SEQ ID NO: 1096 / SEQ ID NO: 6607, SEQ ID NO: 1097 / SEQ ID NO:
6608, SEQ ID NO: 1098 / SEQ ID NO: 6609, SEQ ID NO: 1099 / SEQ ID NO: 6610, SEQ ID
NO: 1100 / SEQ ID NO: 6611, SEQ ID NO: 1101 / SEQ ID NO: 6612, SEQ ID NO: 1102 /
SEQ ID NO: 6613, SEQ ID NO: 1103 / SEQ ID NO: 6614, SEQ ID NO: 1104 / SEQ ID NO:
6615, SEQ ID NO: 1105 / SEQ ID NO: 6616, SEQ ID NO: 1106 / SEQ ID NO: 6617, SEQ ID
NO: 1107 / SEQ ID NO: 6618, SEQ ID NO: 1108 / SEQ ID NO: 6619, SEQ ID NO: 1109 /
SEQ ID NO: 6620, SEQ ID NO: 1110 / SEQ ID NO: 6621, SEQ ID NO: 1111 / SEQ ID NO:
6622, SEQ ID NO: 1112 / SEQ ID NO: 6623, SEQ ID NO: 1113 / SEQ ID NO: 6624, SEQ ID
NO: 1114 / SEQ ID NO: 6625, SEQ ID NO: 1115 / SEQ ID NO: 6626, SEQ ID NO: 1116 /
SEQ ID NO: 6627, SEQ ID NO: 1118 / SEQ ID NO: 6628, SEQ ID NO: 1119 / SEQ ID NO:
6629, SEQ ID NO: 1120 / SEQ ID NO: 6630, SEQ ID NO: 1121 / SEQ ID NO: 6631, SEQ ID
NO: 1122 / SEQ ID NO: 6632, SEQ ID NO: 1123 / SEQ ID NO: 6633, SEQ ID NO: 1124 /
SEQ ID NO: 6634, SEQ ID NO: 1125 / SEQ ID NO: 6635, SEQ ID NO: 1126 / SEQ ID NO:
6636, SEQ ID NO: 1127 / SEQ ID NO: 6637, SEQ ID NO: 1128 / SEQ ID NO: 6638, SEQ ID
NO: 1129 / SEQ ID NO: 6639, SEQ ID NO: 1130 / SEQ ID NO: 6640, SEQ ID NO: 1131 /
SEQ ID NO: 6641, SEQ ID NO: 1132 / SEQ ID NO: 6642, SEQ ID NO: 1133 / SEQ ID NO:
6643, SEQ ID NO: 1134 / SEQ ID NO: 6644, SEQ ID NO: 1135 / SEQ ID NO: 6645, SEQ ID
NO: 1136 / SEQ ID NO: 6646, SEQ ID NO: 1137 / SEQ ID NO: 6647,
SEQ ID NO: 1138 / SEQ ID NO: 6648, SEQ ID NO: 1139 / SEQ ID NO: 6649, SEQ ID NO:
1140 / SEQ ID NO: 6650, SEQ ID NO: 1141 / SEQ ID NO: 6651, SEQ ID NO: 1143 / SEQ ID
NO: 6652, SEQ ID NO: 1144 / SEQ ID NO: 6653, SEQ ID NO: 1145 / SEQ ID NO: 6654, SEQ
ID NO: 1146 / SEQ ID NO: 6655, SEQ ID NO: 1147 / SEQ ID NO: 6656, SEQ ID NO: 1148 /
SEQ ID NO: 6657, SEQ ID NO: 1149 / SEQ ID NO: 6658, SEQ ID NO: 1150 / SEQ ID NO:
6659, SEQ ID NO: 1151 / SEQ ID NO: 6660, SEQ ID NO: 1152 / SEQ ID NO: 6661, SEQ ID

NO: 1153 / SEQ ID NO: 6662, SEQ ID NO: 1154 / SEQ ID NO: 6663, SEQ ID NO: 1155 /
SEQ ID NO: 6664, SEQ ID NO: 1156 / SEQ ID NO: 6665, SEQ ID NO: 1157 / SEQ ID NO:
6666, SEQ ID NO: 1158 / SEQ ID NO: 6667, SEQ ID NO: 1159 / SEQ ID NO: 6668, SEQ ID
NO: 1160 / SEQ ID NO: 6669, SEQ ID NO: 1161 / SEQ ID NO: 6670, SEQ ID NO: 1162 /
SEQ ID NO: 6671, SEQ ID NO: 1163 / SEQ ID NO: 6672, SEQ ID NO: 1164 / SEQ ID NO:
6673, SEQ ID NO: 1165 / SEQ ID NO: 6674, SEQ ID NO: 1166 / SEQ ID NO: 6675, SEQ ID
NO: 1167 / SEQ ID NO: 6676, SEQ ID NO: 1168 / SEQ ID NO: 6677, SEQ ID NO: 1169 /
SEQ ID NO: 6678, SEQ ID NO: 1170 / SEQ ID NO: 6679, SEQ ID NO: 1171 / SEQ ID NO:
6680, SEQ ID NO: 1172 / SEQ ID NO: 6681, SEQ ID NO: 1173 / SEQ ID NO: 6682, SEQ ID
NO: 1174 / SEQ ID NO: 6683, SEQ ID NO: 1175 / SEQ ID NO: 6684, SEQ ID NO: 1176 /
SEQ ID NO: 6685, SEQ ID NO: 1177 / SEQ ID NO: 6686, SEQ ID NO: 1178 / SEQ ID NO:
6687, SEQ ID NO: 1179 / SEQ ID NO: 6688, SEQ ID NO: 1180 / SEQ ID NO: 6689, SEQ ID
NO: 1181 / SEQ ID NO: 6690, SEQ ID NO: 1182 / SEQ ID NO: 6691, SEQ ID NO: 1183 /
SEQ ID NO: 6692, SEQ ID NO: 1184 / SEQ ID NO: 6693, SEQ ID NO: 1185 / SEQ ID NO:
6694, SEQ ID NO: 1186 / SEQ ID NO: 6695, SEQ ID NO: 1187 / SEQ ID NO: 6696, SEQ ID
NO: 1188 / SEQ ID NO: 6697, SEQ ID NO: 1189 / SEQ ID NO: 6698, SEQ ID NO: 1190 /
SEQ ID NO: 6699, SEQ ID NO: 1191 / SEQ ID NO: 6700, SEQ ID NO: 1192 / SEQ ID NO:
6701, SEQ ID NO: 1193 / SEQ ID NO: 6702, SEQ ID NO: 1194 / SEQ ID NO: 6703, SEQ ID
NO: 1195 / SEQ ID NO: 6704, SEQ ID NO: 1197 / SEQ ID NO: 6705, SEQ ID NO: 1198 /
SEQ ID NO: 6706, SEQ ID NO: 1199 / SEQ ID NO: 6707, SEQ ID NO: 1200 / SEQ ID NO:
6708, SEQ ID NO: 1201 / SEQ ID NO: 6709, SEQ ID NO: 1202 / SEQ ID NO: 6710, SEQ ID
NO: 1203 / SEQ ID NO: 6711, SEQ ID NO: 1204 / SEQ ID NO: 6712, SEQ ID NO: 1205 /
SEQ ID NO: 6713, SEQ ID NO: 1206 / SEQ ID NO: 6714, SEQ ID NO: 1207 / SEQ ID NO:
6715, SEQ ID NO: 1208 / SEQ ID NO: 6716, SEQ ID NO: 1209 / SEQ ID NO: 6717, SEQ ID
NO: 1210 / SEQ ID NO: 6718, SEQ ID NO: 1211 / SEQ ID NO: 6719, SEQ ID NO: 1212 /
SEQ ID NO: 6720, SEQ ID NO: 1213 / SEQ ID NO: 6721, SEQ ID NO: 1214 / SEQ ID NO:
6722, SEQ ID NO: 1215 / SEQ ID NO: 6723, SEQ ID NO: 1216 / SEQ ID NO: 6724, SEQ ID
NO: 1217 / SEQ ID NO: 6725, SEQ ID NO: 1218 / SEQ ID NO: 6726, SEQ ID NO: 1219 /
SEQ ID NO: 6727, SEQ ID NO: 1220 / SEQ ID NO: 6728, SEQ ID NO: 1221 / SEQ ID NO:
6729, SEQ ID NO: 1222 / SEQ ID NO: 6730, SEQ ID NO: 1223 / SEQ ID NO: 6731, SEQ ID
NO: 1224 / SEQ ID NO: 6732, SEQ ID NO: 1225 / SEQ ID NO: 6733, SEQ ID NO: 1226 /
SEQ ID NO: 6734, SEQ ID NO: 1227 / SEQ ID NO: 6735, SEQ ID NO: 1228 / SEQ ID NO:
6736, SEQ ID NO: 1229 / SEQ ID NO: 6737, SEQ ID NO: 1230 / SEQ ID NO: 6738, SEQ ID
NO: 1231 / SEQ ID NO: 6739, SEQ ID NO: 1232 / SEQ ID NO: 6740, SEQ ID NO: 1233 /
SEQ ID NO: 6741, SEQ ID NO: 1234 / SEQ ID NO: 6742, SEQ ID NO: 1235 / SEQ ID NO:
6743, SEQ ID NO: 1236 / SEQ ID NO: 6744, SEQ ID NO: 1237 / SEQ ID NO: 6745, SEQ ID
NO: 1238 / SEQ ID NO: 6746, SEQ ID NO: 1239 / SEQ ID NO: 6747,
SEQ ID NO: 1240 / SEQ ID NO: 6748, SEQ ID NO: 1241 / SEQ ID NO: 6749, SEQ ID NO:
1242 / SEQ ID NO: 6750, SEQ ID NO: 1243 / SEQ ID NO: 6751, SEQ ID NO: 1244 / SEQ ID
NO: 6752, SEQ ID NO: 1245 / SEQ ID NO: 6753, SEQ ID NO: 1246 / SEQ ID NO: 6754, SEQ
ID NO: 1247 / SEQ ID NO: 6755, SEQ ID NO: 1248 / SEQ ID NO: 6756, SEQ ID NO: 1249 /
SEQ ID NO: 6757, SEQ ID NO: 1250 / SEQ ID NO: 6758, SEQ ID NO: 1251 / SEQ ID NO:
6759, SEQ ID NO: 1252 / SEQ ID NO: 6760, SEQ ID NO: 1253 / SEQ ID NO: 6761, SEQ ID
NO: 1254 / SEQ ID NO: 6762, SEQ ID NO: 1255 / SEQ ID NO: 6763, SEQ ID NO: 1256 /
SEQ ID NO: 6764, SEQ ID NO: 1257 / SEQ ID NO: 6765, SEQ ID NO: 1258 / SEQ ID NO:
6766, SEQ ID NO: 1259 / SEQ ID NO: 6767, SEQ ID NO: 1260 / SEQ ID NO: 6768, SEQ ID
NO: 1261 / SEQ ID NO: 6769, SEQ ID NO: 1262 / SEQ ID NO: 6770, SEQ ID NO: 1263 /
SEQ ID NO: 6771, SEQ ID NO: 1264 / SEQ ID NO: 6772, SEQ ID NO: 1265 / SEQ ID NO:
6773, SEQ ID NO: 1266 / SEQ ID NO: 6774, SEQ ID NO: 1267 / SEQ ID NO: 6775, SEQ ID
NO: 1268 / SEQ ID NO: 6776, SEQ ID NO: 1269 / SEQ ID NO: 6777, SEQ ID NO: 1270 /
SEQ ID NO: 6778, SEQ ID NO: 1271 / SEQ ID NO: 6779, SEQ ID NO: 1272 / SEQ ID NO:
6780, SEQ ID NO: 1273 / SEQ ID NO: 6781, SEQ ID NO: 1274 / SEQ ID NO: 6782, SEQ ID

NO: 1275 / SEQ ID NO: 6783, SEQ ID NO: 1276 / SEQ ID NO: 6784, SEQ ID NO: 1277 /
SEQ ID NO: 6785, SEQ ID NO: 1278 / SEQ ID NO: 6786, SEQ ID NO: 1279 / SEQ ID NO:
6787, SEQ ID NO: 1280 / SEQ ID NO: 6788, SEQ ID NO: 1281 / SEQ ID NO: 6789, SEQ ID
NO: 1282 / SEQ ID NO: 6790, SEQ ID NO: 1283 / SEQ ID NO: 6791, SEQ ID NO: 1284 /
SEQ ID NO: 6792, SEQ ID NO: 1285 / SEQ ID NO: 6793, SEQ ID NO: 1286 / SEQ ID NO:
6794, SEQ ID NO: 1287 / SEQ ID NO: 6795, SEQ ID NO: 1288 / SEQ ID NO: 6796, SEQ ID
NO: 1289 / SEQ ID NO: 6797, SEQ ID NO: 1290 / SEQ ID NO: 6798, SEQ ID NO: 1291 /
SEQ ID NO: 6799, SEQ ID NO: 1292 / SEQ ID NO: 6800, SEQ ID NO: 1293 / SEQ ID NO:
6801, SEQ ID NO: 1294 / SEQ ID NO: 6802, SEQ ID NO: 1295 / SEQ ID NO: 6803, SEQ ID
NO: 1296 / SEQ ID NO: 6804, SEQ ID NO: 1297 / SEQ ID NO: 6805, SEQ ID NO: 1298 /
SEQ ID NO: 6806, SEQ ID NO: 1299 / SEQ ID NO: 6807, SEQ ID NO: 1300 / SEQ ID NO:
6808, SEQ ID NO: 1301 / SEQ ID NO: 6809, SEQ ID NO: 1302 / SEQ ID NO: 6810, SEQ ID
NO: 1303 / SEQ ID NO: 6811, SEQ ID NO: 1304 / SEQ ID NO: 6812, SEQ ID NO: 1305 /
SEQ ID NO: 6813, SEQ ID NO: 1307 / SEQ ID NO: 6814, SEQ ID NO: 1308 / SEQ ID NO:
6815, SEQ ID NO: 1309 / SEQ ID NO: 6816, SEQ ID NO: 1310 / SEQ ID NO: 6817, SEQ ID
NO: 1311 / SEQ ID NO: 6818, SEQ ID NO: 1312 / SEQ ID NO: 6819, SEQ ID NO: 1313 /
SEQ ID NO: 6820, SEQ ID NO: 1314 / SEQ ID NO: 6821, SEQ ID NO: 1315 / SEQ ID NO:
6822, SEQ ID NO: 1316 / SEQ ID NO: 6823, SEQ ID NO: 1317 / SEQ ID NO: 6824, SEQ ID
NO: 1318 / SEQ ID NO: 6825, SEQ ID NO: 1319 / SEQ ID NO: 6826, SEQ ID NO: 1320 /
SEQ ID NO: 6827, SEQ ID NO: 1321 / SEQ ID NO: 6828, SEQ ID NO: 1322 / SEQ ID NO:
6829, SEQ ID NO: 1323 / SEQ ID NO: 6830, SEQ ID NO: 1324 / SEQ ID NO: 6831, SEQ ID
NO: 1325 / SEQ ID NO: 6832, SEQ ID NO: 1326 / SEQ ID NO: 6833, SEQ ID NO: 1327 /
SEQ ID NO: 6834, SEQ ID NO: 1328 / SEQ ID NO: 6835, SEQ ID NO: 1329 / SEQ ID NO:
6836, SEQ ID NO: 1330 / SEQ ID NO: 6837, SEQ ID NO: 1331 / SEQ ID NO: 6838, SEQ ID
NO: 1332 / SEQ ID NO: 6839, SEQ ID NO: 1333 / SEQ ID NO: 6840, SEQ ID NO: 1334 /
SEQ ID NO: 6841, SEQ ID NO: 1335 / SEQ ID NO: 6842, SEQ ID NO: 1336 / SEQ ID NO:
6843, SEQ ID NO: 1337 / SEQ ID NO: 6844, SEQ ID NO: 1338 / SEQ ID NO: 6845, SEQ ID
NO: 1339 / SEQ ID NO: 6846, SEQ ID NO: 1340 / SEQ ID NO: 6847,
SEQ ID NO: 1341 / SEQ ID NO: 6848, SEQ ID NO: 1342 / SEQ ID NO: 6849, SEQ ID NO:
1343 / SEQ ID NO: 6850, SEQ ID NO: 1344 / SEQ ID NO: 6851, SEQ ID NO: 1345 / SEQ ID
NO: 6852, SEQ ID NO: 1346 / SEQ ID NO: 6853, SEQ ID NO: 1347 / SEQ ID NO: 6854, SEQ
ID NO: 1348 / SEQ ID NO: 6855, SEQ ID NO: 1349 / SEQ ID NO: 6856, SEQ ID NO: 1350 /
SEQ ID NO: 6857, SEQ ID NO: 1351 / SEQ ID NO: 6858, SEQ ID NO: 1352 / SEQ ID NO:
6859, SEQ ID NO: 1353 / SEQ ID NO: 6860, SEQ ID NO: 1354 / SEQ ID NO: 6861, SEQ ID
NO: 1355 / SEQ ID NO: 6862, SEQ ID NO: 1356 / SEQ ID NO: 6863, SEQ ID NO: 1357 /
SEQ ID NO: 6864, SEQ ID NO: 1358 / SEQ ID NO: 6865, SEQ ID NO: 1360 / SEQ ID NO:
6866, SEQ ID NO: 1361 / SEQ ID NO: 6867, SEQ ID NO: 1362 / SEQ ID NO: 6868, SEQ ID
NO: 1363 / SEQ ID NO: 6869, SEQ ID NO: 1364 / SEQ ID NO: 6870, SEQ ID NO: 1365 /
SEQ ID NO: 6871, SEQ ID NO: 1366 / SEQ ID NO: 6872, SEQ ID NO: 1367 / SEQ ID NO:
6873, SEQ ID NO: 1368 / SEQ ID NO: 6874, SEQ ID NO: 1369 / SEQ ID NO: 6875, SEQ ID
NO: 1370 / SEQ ID NO: 6876, SEQ ID NO: 1371 / SEQ ID NO: 6877, SEQ ID NO: 1372 /
SEQ ID NO: 6878, SEQ ID NO: 1373 / SEQ ID NO: 6879, SEQ ID NO: 1374 / SEQ ID NO:
6880, SEQ ID NO: 1375 / SEQ ID NO: 6881, SEQ ID NO: 1376 / SEQ ID NO: 6882, SEQ ID
NO: 1377 / SEQ ID NO: 6883, SEQ ID NO: 1378 / SEQ ID NO: 6884, SEQ ID NO: 1379 /
SEQ ID NO: 6885, SEQ ID NO: 1380 / SEQ ID NO: 6886, SEQ ID NO: 1381 / SEQ ID NO:
6887, SEQ ID NO: 1382 / SEQ ID NO: 6888, SEQ ID NO: 1383 / SEQ ID NO: 6889, SEQ ID
NO: 1384 / SEQ ID NO: 6890, SEQ ID NO: 1385 / SEQ ID NO: 6891, SEQ ID NO: 1386 /
SEQ ID NO: 6892, SEQ ID NO: 1387 / SEQ ID NO: 6893, SEQ ID NO: 1389 / SEQ ID NO:
6894, SEQ ID NO: 1390 / SEQ ID NO: 6895, SEQ ID NO: 1391 / SEQ ID NO: 6896, SEQ ID
NO: 1392 / SEQ ID NO: 6897, SEQ ID NO: 1393 / SEQ ID NO: 6898, SEQ ID NO: 1394 /
SEQ ID NO: 6899, SEQ ID NO: 1395 / SEQ ID NO: 6900, SEQ ID NO: 1396 / SEQ ID NO:
6901, SEQ ID NO: 1397 / SEQ ID NO: 6902, SEQ ID NO: 1398 / SEQ ID NO: 6903, SEQ ID

NO: 1399 / SEQ ID NO: 6904, SEQ ID NO: 1400 / SEQ ID NO: 6905, SEQ ID NO: 1401 /
SEQ ID NO: 6906, SEQ ID NO: 1402 / SEQ ID NO: 6907, SEQ ID NO: 1403 / SEQ ID NO:
6908, SEQ ID NO: 1404 / SEQ ID NO: 6909, SEQ ID NO: 1405 / SEQ ID NO: 6910, SEQ ID
NO: 1406 / SEQ ID NO: 6911, SEQ ID NO: 1407 / SEQ ID NO: 6912, SEQ ID NO: 1408 /
SEQ ID NO: 6913, SEQ ID NO: 1409 / SEQ ID NO: 6914, SEQ ID NO: 1410 / SEQ ID NO:
6915, SEQ ID NO: 1411 / SEQ ID NO: 6916, SEQ ID NO: 1412 / SEQ ID NO: 6917, SEQ ID
NO: 1413 / SEQ ID NO: 6918, SEQ ID NO: 1414 / SEQ ID NO: 6919, SEQ ID NO: 1415 /
SEQ ID NO: 6920, SEQ ID NO: 1416 / SEQ ID NO: 6921, SEQ ID NO: 1417 / SEQ ID NO:
6922, SEQ ID NO: 1418 / SEQ ID NO: 6923, SEQ ID NO: 1419 / SEQ ID NO: 6924, SEQ ID
NO: 1420 / SEQ ID NO: 6925, SEQ ID NO: 1421 / SEQ ID NO: 6926, SEQ ID NO: 1422 /
SEQ ID NO: 6927, SEQ ID NO: 1423 / SEQ ID NO: 6928, SEQ ID NO: 1424 / SEQ ID NO:
6929, SEQ ID NO: 1425 / SEQ ID NO: 6930, SEQ ID NO: 1426 / SEQ ID NO: 6931, SEQ ID
NO: 1427 / SEQ ID NO: 6932, SEQ ID NO: 1428 / SEQ ID NO: 6933, SEQ ID NO: 1429 /
SEQ ID NO: 6934, SEQ ID NO: 1430 / SEQ ID NO: 6935, SEQ ID NO: 1431 / SEQ ID NO:
6936, SEQ ID NO: 1432 / SEQ ID NO: 6937, SEQ ID NO: 1433 / SEQ ID NO: 6938, SEQ ID
NO: 1434 / SEQ ID NO: 6939, SEQ ID NO: 1435 / SEQ ID NO: 6940, SEQ ID NO: 1436 /
SEQ ID NO: 6941, SEQ ID NO: 1437 / SEQ ID NO: 6942, SEQ ID NO: 1438 / SEQ ID NO:
6943, SEQ ID NO: 1439 / SEQ ID NO: 6944, SEQ ID NO: 1440 / SEQ ID NO: 6945, SEQ ID
NO: 1441 / SEQ ID NO: 6946, SEQ ID NO: 1442 / SEQ ID NO: 6947,
SEQ ID NO: 1443 / SEQ ID NO: 6948, SEQ ID NO: 1444 / SEQ ID NO: 6949, SEQ ID NO:
1445 / SEQ ID NO: 6950, SEQ ID NO: 1446 / SEQ ID NO: 6951, SEQ ID NO: 1447 / SEQ ID
NO: 6952, SEQ ID NO: 1448 / SEQ ID NO: 6953, SEQ ID NO: 1449 / SEQ ID NO: 6954, SEQ
ID NO: 1450 / SEQ ID NO: 6955, SEQ ID NO: 1451 / SEQ ID NO: 6956, SEQ ID NO: 1452 /
SEQ ID NO: 6957, SEQ ID NO: 1453 / SEQ ID NO: 6958, SEQ ID NO: 1454 / SEQ ID NO:
6959, SEQ ID NO: 1455 / SEQ ID NO: 6960, SEQ ID NO: 1456 / SEQ ID NO: 6961, SEQ ID
NO: 1457 / SEQ ID NO: 6962, SEQ ID NO: 1458 / SEQ ID NO: 6963, SEQ ID NO: 1459 /
SEQ ID NO: 6964, SEQ ID NO: 1460 / SEQ ID NO: 6965, SEQ ID NO: 1461 / SEQ ID NO:
6966, SEQ ID NO: 1462 / SEQ ID NO: 6967, SEQ ID NO: 1463 / SEQ ID NO: 6968, SEQ ID
NO: 1464 / SEQ ID NO: 6969, SEQ ID NO: 1465 / SEQ ID NO: 6970, SEQ ID NO: 1466 /
SEQ ID NO: 6971, SEQ ID NO: 1467 / SEQ ID NO: 6972, SEQ ID NO: 1468 / SEQ ID NO:
6973, SEQ ID NO: 1469 / SEQ ID NO: 6974, SEQ ID NO: 1470 / SEQ ID NO: 6975, SEQ ID
NO: 1471 / SEQ ID NO: 6976, SEQ ID NO: 1472 / SEQ ID NO: 6977, SEQ ID NO: 1473 /
SEQ ID NO: 6978, SEQ ID NO: 1474 / SEQ ID NO: 6979, SEQ ID NO: 1475 / SEQ ID NO:
6980, SEQ ID NO: 1476 / SEQ ID NO: 6981, SEQ ID NO: 1477 / SEQ ID NO: 6982, SEQ ID
NO: 1478 / SEQ ID NO: 6983, SEQ ID NO: 1479 / SEQ ID NO: 6984, SEQ ID NO: 1480 /
SEQ ID NO: 6985, SEQ ID NO: 1481 / SEQ ID NO: 6986, SEQ ID NO: 1482 / SEQ ID NO:
6987, SEQ ID NO: 1483 / SEQ ID NO: 6988, SEQ ID NO: 1484 / SEQ ID NO: 6989, SEQ ID
NO: 1485 / SEQ ID NO: 6990, SEQ ID NO: 1486 / SEQ ID NO: 6991, SEQ ID NO: 1487 /
SEQ ID NO: 6992, SEQ ID NO: 1488 / SEQ ID NO: 6993, SEQ ID NO: 1489 / SEQ ID NO:
6994, SEQ ID NO: 1490 / SEQ ID NO: 6995, SEQ ID NO: 1491 / SEQ ID NO: 6996, SEQ ID
NO: 1492 / SEQ ID NO: 6997, SEQ ID NO: 1493 / SEQ ID NO: 6998, SEQ ID NO: 1494 /
SEQ ID NO: 6999, SEQ ID NO: 1495 / SEQ ID NO: 7000, SEQ ID NO: 1496 / SEQ ID NO:
7001, SEQ ID NO: 1497 / SEQ ID NO: 7002, SEQ ID NO: 1498 / SEQ ID NO: 7003, SEQ ID
NO: 1499 / SEQ ID NO: 7004, SEQ ID NO: 1500 / SEQ ID NO: 7005, SEQ ID NO: 1501 /
SEQ ID NO: 7006, SEQ ID NO: 1502 / SEQ ID NO: 7007, SEQ ID NO: 1503 / SEQ ID NO:
7008, SEQ ID NO: 1504 / SEQ ID NO: 7009, SEQ ID NO: 1505 / SEQ ID NO: 7010, SEQ ID
NO: 1506 / SEQ ID NO: 7011, SEQ ID NO: 1507 / SEQ ID NO: 7012, SEQ ID NO: 1508 /
SEQ ID NO: 7013, SEQ ID NO: 1509 / SEQ ID NO: 7014, SEQ ID NO: 1510 / SEQ ID NO:
7015, SEQ ID NO: 1511 / SEQ ID NO: 7016, SEQ ID NO: 1512 / SEQ ID NO: 7017, SEQ ID
NO: 1514 / SEQ ID NO: 7018, SEQ ID NO: 1515 / SEQ ID NO: 7019, SEQ ID NO: 1516 /
SEQ ID NO: 7020, SEQ ID NO: 1517 / SEQ ID NO: 7021, SEQ ID NO: 1518 / SEQ ID NO:
7022, SEQ ID NO: 1519 / SEQ ID NO: 7023, SEQ ID NO: 1520 / SEQ ID NO: 7024, SEQ ID

NO: 1521 / SEQ ID NO: 7025, SEQ ID NO: 1522 / SEQ ID NO: 7026, SEQ ID NO: 1523 /
SEQ ID NO: 7027, SEQ ID NO: 1524 / SEQ ID NO: 7028, SEQ ID NO: 1525 / SEQ ID NO:
7029, SEQ ID NO: 1526 / SEQ ID NO: 7030, SEQ ID NO: 1527 / SEQ ID NO: 7031, SEQ ID
NO: 1528 / SEQ ID NO: 7032, SEQ ID NO: 1529 / SEQ ID NO: 7033, SEQ ID NO: 1530 /
SEQ ID NO: 7034, SEQ ID NO: 1531 / SEQ ID NO: 7035, SEQ ID NO: 1532 / SEQ ID NO:
7036, SEQ ID NO: 1533 / SEQ ID NO: 7037, SEQ ID NO: 1534 / SEQ ID NO: 7038, SEQ ID
NO: 1535 / SEQ ID NO: 7039, SEQ ID NO: 1536 / SEQ ID NO: 7040, SEQ ID NO: 1537 /
SEQ ID NO: 7041, SEQ ID NO: 1538 / SEQ ID NO: 7042, SEQ ID NO: 1539 / SEQ ID NO:
7043, SEQ ID NO: 1540 / SEQ ID NO: 7044, SEQ ID NO: 1541 / SEQ ID NO: 7045, SEQ ID
NO: 1542 / SEQ ID NO: 7046, SEQ ID NO: 1543 / SEQ ID NO: 7047,
SEQ ID NO: 1544 / SEQ ID NO: 7048, SEQ ID NO: 1545 / SEQ ID NO: 7049, SEQ ID NO:
1546 / SEQ ID NO: 7050, SEQ ID NO: 1547 / SEQ ID NO: 7051, SEQ ID NO: 1548 / SEQ ID
NO: 7052, SEQ ID NO: 1549 / SEQ ID NO: 7053, SEQ ID NO: 1550 / SEQ ID NO: 7054, SEQ
ID NO: 1551 / SEQ ID NO: 7055, SEQ ID NO: 1552 / SEQ ID NO: 7056, SEQ ID NO: 1553 /
SEQ ID NO: 7057, SEQ ID NO: 1554 / SEQ ID NO: 7058, SEQ ID NO: 1555 / SEQ ID NO:
7059, SEQ ID NO: 1556 / SEQ ID NO: 7060, SEQ ID NO: 1558 / SEQ ID NO: 7061, SEQ ID
NO: 1559 / SEQ ID NO: 7062, SEQ ID NO: 1560 / SEQ ID NO: 7063, SEQ ID NO: 1561 /
SEQ ID NO: 7064, SEQ ID NO: 1562 / SEQ ID NO: 7065, SEQ ID NO: 1563 / SEQ ID NO:
7066, SEQ ID NO: 1564 / SEQ ID NO: 7067, SEQ ID NO: 1565 / SEQ ID NO: 7068, SEQ ID
NO: 1566 / SEQ ID NO: 7069, SEQ ID NO: 1568 / SEQ ID NO: 7070, SEQ ID NO: 1569 /
SEQ ID NO: 7071, SEQ ID NO: 1570 / SEQ ID NO: 7072, SEQ ID NO: 1571 / SEQ ID NO:
7073, SEQ ID NO: 1572 / SEQ ID NO: 7074, SEQ ID NO: 1573 / SEQ ID NO: 7075, SEQ ID
NO: 1574 / SEQ ID NO: 7076, SEQ ID NO: 1575 / SEQ ID NO: 7077, SEQ ID NO: 1576 /
SEQ ID NO: 7078, SEQ ID NO: 1577 / SEQ ID NO: 7079, SEQ ID NO: 1578 / SEQ ID NO:
7080, SEQ ID NO: 1579 / SEQ ID NO: 7081, SEQ ID NO: 1580 / SEQ ID NO: 7082, SEQ ID
NO: 1581 / SEQ ID NO: 7083, SEQ ID NO: 1582 / SEQ ID NO: 7084, SEQ ID NO: 1583 /
SEQ ID NO: 7085, SEQ ID NO: 1584 / SEQ ID NO: 7086, SEQ ID NO: 1585 / SEQ ID NO:
7087, SEQ ID NO: 1586 / SEQ ID NO: 7088, SEQ ID NO: 1587 / SEQ ID NO: 7089, SEQ ID
NO: 1588 / SEQ ID NO: 7090, SEQ ID NO: 1589 / SEQ ID NO: 7091, SEQ ID NO: 1590 /
SEQ ID NO: 7092, SEQ ID NO: 1591 / SEQ ID NO: 7093, SEQ ID NO: 1592 / SEQ ID NO:
7094, SEQ ID NO: 1593 / SEQ ID NO: 7095, SEQ ID NO: 1594 / SEQ ID NO: 7096, SEQ ID
NO: 1595 / SEQ ID NO: 7097, SEQ ID NO: 1596 / SEQ ID NO: 7098, SEQ ID NO: 1597 /
SEQ ID NO: 7099, SEQ ID NO: 1598 / SEQ ID NO: 7100, SEQ ID NO: 1599 / SEQ ID NO:
7101, SEQ ID NO: 1600 / SEQ ID NO: 7102, SEQ ID NO: 1601 / SEQ ID NO: 7103, SEQ ID
NO: 1602 / SEQ ID NO: 7104, SEQ ID NO: 1603 / SEQ ID NO: 7105, SEQ ID NO: 1604 /
SEQ ID NO: 7106, SEQ ID NO: 1605 / SEQ ID NO: 7107, SEQ ID NO: 1606 / SEQ ID NO:
7108, SEQ ID NO: 1607 / SEQ ID NO: 7109, SEQ ID NO: 1608 / SEQ ID NO: 7110, SEQ ID
NO: 1609 / SEQ ID NO: 7111, SEQ ID NO: 1610 / SEQ ID NO: 7112, SEQ ID NO: 1612 /
SEQ ID NO: 7113, SEQ ID NO: 1613 / SEQ ID NO: 7114, SEQ ID NO: 1614 / SEQ ID NO:
7115, SEQ ID NO: 1615 / SEQ ID NO: 7116, SEQ ID NO: 1616 / SEQ ID NO: 7117, SEQ ID
NO: 1617 / SEQ ID NO: 7118, SEQ ID NO: 1618 / SEQ ID NO: 7119, SEQ ID NO: 1619 /
SEQ ID NO: 7120, SEQ ID NO: 1620 / SEQ ID NO: 7121, SEQ ID NO: 1621 / SEQ ID NO:
7122, SEQ ID NO: 1622 / SEQ ID NO: 7123, SEQ ID NO: 1623 / SEQ ID NO: 7124, SEQ ID
NO: 1624 / SEQ ID NO: 7125, SEQ ID NO: 1625 / SEQ ID NO: 7126, SEQ ID NO: 1626 /
SEQ ID NO: 7127, SEQ ID NO: 1627 / SEQ ID NO: 7128, SEQ ID NO: 1628 / SEQ ID NO:
7129, SEQ ID NO: 1629 / SEQ ID NO: 7130, SEQ ID NO: 1630 / SEQ ID NO: 7131, SEQ ID
NO: 1631 / SEQ ID NO: 7132, SEQ ID NO: 1632 / SEQ ID NO: 7133, SEQ ID NO: 1633 /
SEQ ID NO: 7134, SEQ ID NO: 1634 / SEQ ID NO: 7135, SEQ ID NO: 1635 / SEQ ID NO:
7136, SEQ ID NO: 1636 / SEQ ID NO: 7137, SEQ ID NO: 1637 / SEQ ID NO: 7138, SEQ ID
NO: 1638 / SEQ ID NO: 7139, SEQ ID NO: 1639 / SEQ ID NO: 7140, SEQ ID NO: 1640 /
SEQ ID NO: 7141, SEQ ID NO: 1641 / SEQ ID NO: 7142, SEQ ID NO: 1642 / SEQ ID NO:

7143, SEQ ID NO:.1643 / SEQ ID NO: 7144, SEQ ID NO: 1644 / SEQ ID NO: 7145, SEQ ID
NO: 1645 / SEQ ID NO: 7146, SEQ ID NO: 1646 / SEQ ID NO: 7147,
SEQ ID NO: 1647 / SEQ ID NO: 7148, SEQ ID NO: 1648 / SEQ ID NO: 7149, SEQ ID NO:
1649 / SEQ ID NO: 7150, SEQ ID NO: 1650 / SEQ ID NO: 7151, SEQ ID NO: 1651 / SEQ ID
NO: 7152, SEQ ID NO: 1652 / SEQ ID NO: 7153, SEQ ID NO: 1653 / SEQ ID NO: 7154, SEQ
ID NO: 1654 / SEQ ID NO: 7155, SEQ ID NO: 1655 / SEQ ID NO: 7156, SEQ ID NO: 1656 /
SEQ ID NO: 7157, SEQ ID NO: 1657 / SEQ ID NO: 7158, SEQ ID NO: 1658 / SEQ ID NO:
7159, SEQ ID NO: 1659 / SEQ ID NO: 7160, SEQ ID NO: 1660 / SEQ ID NO: 7161, SEQ ID
NO: 1661 / SEQ ID NO: 7162, SEQ ID NO: 1662 / SEQ ID NO: 7163, SEQ ID NO: 1663 /
SEQ ID NO: 7164, SEQ ID NO: 1664 / SEQ ID NO: 7165, SEQ ID NO: 1665 / SEQ ID NO:
7166, SEQ ID NO: 1666 / SEQ ID NO: 7167, SEQ ID NO: 1667 / SEQ ID NO: 7168, SEQ ID
NO: 1668 / SEQ ID NO: 7169, SEQ ID NO: 1669 / SEQ ID NO: 7170, SEQ ID NO: 1670 /
SEQ ID NO: 7171, SEQ ID NO: 1671 / SEQ ID NO: 7172, SEQ ID NO: 1672 / SEQ ID NO:
7173, SEQ ID NO: 1673 / SEQ ID NO: 7174, SEQ ID NO: 1674 / SEQ ID NO: 7175, SEQ ID
NO: 1675 / SEQ ID NO: 7176, SEQ ID NO: 1676 / SEQ ID NO: 7177, SEQ ID NO: 1677 /
SEQ ID NO: 7178, SEQ ID NO: 1678 / SEQ ID NO: 7179, SEQ ID NO: 1679 / SEQ ID NO:
7180, SEQ ID NO: 1680 / SEQ ID NO: 7181, SEQ ID NO: 1681 / SEQ ID NO: 7182, SEQ ID
NO: 1682 / SEQ ID NO: 7183, SEQ ID NO: 1683 / SEQ ID NO: 7184, SEQ ID NO: 1684 /
SEQ ID NO: 7185, SEQ ID NO: 1685 / SEQ ID NO: 7186, SEQ ID NO: 1686 / SEQ ID NO:
7187, SEQ ID NO: 1688 / SEQ ID NO: 7188, SEQ ID NO: 1689 / SEQ ID NO: 7189, SEQ ID
NO: 1690 / SEQ ID NO: 7190, SEQ ID NO: 1691 / SEQ ID NO: 7191, SEQ ID NO: 1692 /
SEQ ID NO: 7192, SEQ ID NO: 1693 / SEQ ID NO: 7193, SEQ ID NO: 1694 / SEQ ID NO:
7194, SEQ ID NO: 1695 / SEQ ID NO: 7195, SEQ ID NO: 1696 / SEQ ID NO: 7196, SEQ ID
NO: 1697 / SEQ ID NO: 7197, SEQ ID NO: 1698 / SEQ ID NO: 7198, SEQ ID NO: 1699 /
SEQ ID NO: 7199, SEQ ID NO: 1700 / SEQ ID NO: 7200, SEQ ID NO: 1701 / SEQ ID NO:
7201, SEQ ID NO: 1702 / SEQ ID NO: 7202, SEQ ID NO: 1703 / SEQ ID NO: 7203, SEQ ID
NO: 1704 / SEQ ID NO: 7204, SEQ ID NO: 1705 / SEQ ID NO: 7205, SEQ ID NO: 1706 /
SEQ ID NO: 7206, SEQ ID NO: 1707 / SEQ ID NO: 7207, SEQ ID NO: 1708 / SEQ ID NO:
7208, SEQ ID NO: 1709 / SEQ ID NO: 7209, SEQ ID NO: 1710 / SEQ ID NO: 7210, SEQ ID
NO: 1711 / SEQ ID NO: 7211, SEQ ID NO: 1712 / SEQ ID NO: 7212, SEQ ID NO: 1713 /
SEQ ID NO: 7213, SEQ ID NO: 1714 / SEQ ID NO: 7214, SEQ ID NO: 1715 / SEQ ID NO:
7215, SEQ ID NO: 1716 / SEQ ID NO: 7216, SEQ ID NO: 1717 / SEQ ID NO: 7217, SEQ ID
NO: 1718 / SEQ ID NO: 7218, SEQ ID NO: 1719 / SEQ ID NO: 7219, SEQ ID NO: 1720 /
SEQ ID NO: 7220, SEQ ID NO: 1721 / SEQ ID NO: 7221, SEQ ID NO: 1722 / SEQ ID NO:
7222, SEQ ID NO: 1723 / SEQ ID NO: 7223, SEQ ID NO: 1724 / SEQ ID NO: 7224, SEQ ID
NO: 1725 / SEQ ID NO: 7225, SEQ ID NO: 1726 / SEQ ID NO: 7226, SEQ ID NO: 1727 /
SEQ ID NO: 7227, SEQ ID NO: 1728 / SEQ ID NO: 7228, SEQ ID NO: 1729 / SEQ ID NO:
7229, SEQ ID NO: 1730 / SEQ ID NO: 7230, SEQ ID NO: 1731 / SEQ ID NO: 7231, SEQ ID
NO: 1732 / SEQ ID NO: 7232, SEQ ID NO: 1733 / SEQ ID NO: 7233, SEQ ID NO: 1734 /
SEQ ID NO: 7234, SEQ ID NO: 1735 / SEQ ID NO: 7235, SEQ ID NO: 1736 / SEQ ID NO:
7236, SEQ ID NO: 1737 / SEQ ID NO: 7237, SEQ ID NO: 1738 / SEQ ID NO: 7238, SEQ ID
NO: 1739 / SEQ ID NO: 7239, SEQ ID NO: 1740 / SEQ ID NO: 7240, SEQ ID NO: 1741 /
SEQ ID NO: 7241, SEQ ID NO: 1742 / SEQ ID NO: 7242, SEQ ID NO: 1743 / SEQ ID NO:
7243, SEQ ID NO: 1744 / SEQ ID NO: 7244, SEQ ID NO: 1745 / SEQ ID NO: 7245, SEQ ID
NO: 1746 / SEQ ID NO: 7246, SEQ ID NO: 1747 / SEQ ID NO: 7247,
SEQ ID NO: 1748 / SEQ ID NO: 7248, SEQ ID NO: 1749 / SEQ ID NO: 7249, SEQ ID NO:
1750 / SEQ ID NO: 7250, SEQ ID NO: 1751 / SEQ ID NO: 7251, SEQ ID NO: 1752 / SEQ ID
NO: 7252, SEQ ID NO: 1753 / SEQ ID NO: 7253, SEQ ID NO: 1754 / SEQ ID NO: 7254, SEQ
ID NO: 1755 / SEQ ID NO: 7255, SEQ ID NO: 1756 / SEQ ID NO: 7256, SEQ ID NO: 1757 /
SEQ ID NO: 7257, SEQ ID NO: 1758 / SEQ ID NO: 7258, SEQ ID NO: 1759 / SEQ ID NO:
7259, SEQ ID NO: 1760 / SEQ ID NO: 7260, SEQ ID NO: 1761 / SEQ ID NO: 7261, SEQ ID
NO: 1762 / SEQ ID NO: 7262, SEQ ID NO: 1763 / SEQ ID NO: 7263, SEQ ID NO: 1764 /

SEQ ID NO: 7264, SEQ ID NO: 1765 / SEQ ID NO: 7265, SEQ ID NO: 1766 / SEQ ID NO:
7266, SEQ ID NO: 1767 / SEQ ID NO: 7267, SEQ ID NO: 1768 / SEQ ID NO: 7268, SEQ ID
NO: 1769 / SEQ ID NO: 7269, SEQ ID NO: 1770 / SEQ ID NO: 7270, SEQ ID NO: 1771 /
SEQ ID NO: 7271, SEQ ID NO: 1772 / SEQ ID NO: 7272, SEQ ID NO: 1773 / SEQ ID NO:
7273, SEQ ID NO: 1774 / SEQ ID NO: 7274, SEQ ID NO: 1775 / SEQ ID NO: 7275, SEQ ID
NO: 1776 / SEQ ID NO: 7276, SEQ ID NO: 1777 / SEQ ID NO: 7277, SEQ ID NO: 1778 /
SEQ ID NO: 7278, SEQ ID NO: 1779 / SEQ ID NO: 7279, SEQ ID NO: 1780 / SEQ ID NO:
7280, SEQ ID NO: 1781 / SEQ ID NO: 7281, SEQ ID NO: 1782 / SEQ ID NO: 7282, SEQ ID
NO: 1783 / SEQ ID NO: 7283, SEQ ID NO: 1784 / SEQ ID NO: 7284, SEQ ID NO: 1785 /
SEQ ID NO: 7285, SEQ ID NO: 1786 / SEQ ID NO: 7286, SEQ ID NO: 1787 / SEQ ID NO:
7287, SEQ ID NO: 1788 / SEQ ID NO: 7288, SEQ ID NO: 1789 / SEQ ID NO: 7289, SEQ ID
NO: 1790 / SEQ ID NO: 7290, SEQ ID NO: 1791 / SEQ ID NO: 7291, SEQ ID NO: 1792 /
SEQ ID NO: 7292, SEQ ID NO: 1793 / SEQ ID NO: 7293, SEQ ID NO: 1794 / SEQ ID NO:
7294, SEQ ID NO: 1795 / SEQ ID NO: 7295, SEQ ID NO: 1796 / SEQ ID NO: 7296, SEQ ID
NO: 1797 / SEQ ID NO: 7297, SEQ ID NO: 1798 / SEQ ID NO: 7298, SEQ ID NO: 1799 /
SEQ ID NO: 7299, SEQ ID NO: 1800 / SEQ ID NO: 7300, SEQ ID NO: 1801 / SEQ ID NO:
7301, SEQ ID NO: 1802 / SEQ ID NO: 7302, SEQ ID NO: 1803 / SEQ ID NO: 7303, SEQ ID
NO: 1804 / SEQ ID NO: 7304, SEQ ID NO: 1805 / SEQ ID NO: 7305, SEQ ID NO: 1806 /
SEQ ID NO: 7306, SEQ ID NO: 1807 / SEQ ID NO: 7307, SEQ ID NO: 1808 / SEQ ID NO:
7308, SEQ ID NO: 1809 / SEQ ID NO: 7309, SEQ ID NO: 1810 / SEQ ID NO: 7310, SEQ ID
NO: 1811 / SEQ ID NO: 7311, SEQ ID NO: 1812 / SEQ ID NO: 7312, SEQ ID NO: 1813 /
SEQ ID NO: 7313, SEQ ID NO: 1814 / SEQ ID NO: 7314, SEQ ID NO: 1815 / SEQ ID NO:
7315, SEQ ID NO: 1816 / SEQ ID NO: 7316, SEQ ID NO: 1817 / SEQ ID NO: 7317, SEQ ID
NO: 1818 / SEQ ID NO: 7318, SEQ ID NO: 1819 / SEQ ID NO: 7319, SEQ ID NO: 1820 /
SEQ ID NO: 7320, SEQ ID NO: 1821 / SEQ ID NO: 7321, SEQ ID NO: 1822 / SEQ ID NO:
7322, SEQ ID NO: 1823 / SEQ ID NO: 7323, SEQ ID NO: 1824 / SEQ ID NO: 7324, SEQ ID
NO: 1825 / SEQ ID NO: 7325, SEQ ID NO: 1826 / SEQ ID NO: 7326, SEQ ID NO: 1827 /
SEQ ID NO: 7327, SEQ ID NO: 1828 / SEQ ID NO: 7328, SEQ ID NO: 1829 / SEQ ID NO:
7329, SEQ ID NO: 1830 / SEQ ID NO: 7330, SEQ ID NO: 1831 / SEQ ID NO: 7331, SEQ ID
NO: 1832 / SEQ ID NO: 7332, SEQ ID NO: 1833 / SEQ ID NO: 7333, SEQ ID NO: 1834 /
SEQ ID NO: 7334, SEQ ID NO: 1835 / SEQ ID NO: 7335, SEQ ID NO: 1836 / SEQ ID NO:
7336, SEQ ID NO: 1837 / SEQ ID NO: 7337, SEQ ID NO: 1838 / SEQ ID NO: 7338, SEQ ID
NO: 1839 / SEQ ID NO: 7339, SEQ ID NO: 1840 / SEQ ID NO: 7340, SEQ ID NO: 1841 /
SEQ ID NO: 7341, SEQ ID NO: 1842 / SEQ ID NO: 7342, SEQ ID NO: 1843 / SEQ ID NO:
7343, SEQ ID NO: 1844 / SEQ ID NO: 7344, SEQ ID NO: 1845 / SEQ ID NO: 7345, SEQ ID
NO: 1846 / SEQ ID NO: 7346, SEQ ID NO: 1847 / SEQ ID NO: 7347,
SEQ ID NO: 1848 / SEQ ID NO: 7348, SEQ ID NO: 1849 / SEQ ID NO: 7349, SEQ ID NO:
1850 / SEQ ID NO: 7350, SEQ ID NO: 1851 / SEQ ID NO: 7351, SEQ ID NO: 1852 / SEQ ID
NO: 7352, SEQ ID NO: 1853 / SEQ ID NO: 7353, SEQ ID NO: 1854 / SEQ ID NO: 7354, SEQ
ID NO: 1855 / SEQ ID NO: 7355, SEQ ID NO: 1856 / SEQ ID NO: 7356, SEQ ID NO: 1857 /
SEQ ID NO: 7357, SEQ ID NO: 1858 / SEQ ID NO: 7358, SEQ ID NO: 1859 / SEQ ID NO:
7359, SEQ ID NO: 1860 / SEQ ID NO: 7360, SEQ ID NO: 1861 / SEQ ID NO: 7361, SEQ ID
NO: 1862 / SEQ ID NO: 7362, SEQ ID NO: 1863 / SEQ ID NO: 7363, SEQ ID NO: 1864 /
SEQ ID NO: 7364, SEQ ID NO: 1865 / SEQ ID NO: 7365, SEQ ID NO: 1866 / SEQ ID NO:
7366, SEQ ID NO: 1867 / SEQ ID NO: 7367, SEQ ID NO: 1868 / SEQ ID NO: 7368, SEQ ID
NO: 1869 / SEQ ID NO: 7369, SEQ ID NO: 1870 / SEQ ID NO: 7370, SEQ ID NO: 1871 /
SEQ ID NO: 7371, SEQ ID NO: 1872 / SEQ ID NO: 7372, SEQ ID NO: 1873 / SEQ ID NO:
7373, SEQ ID NO: 1874 / SEQ ID NO: 7374, SEQ ID NO: 1876 / SEQ ID NO: 7375, SEQ ID
NO: 1877 / SEQ ID NO: 7376, SEQ ID NO: 1878 / SEQ ID NO: 7377, SEQ ID NO: 1879 /
SEQ ID NO: 7378, SEQ ID NO: 1880 / SEQ ID NO: 7379, SEQ ID NO: 1881 / SEQ ID NO:
7380, SEQ ID NO: 1882 / SEQ ID NO: 7381, SEQ ID NO: 1883 / SEQ ID NO: 7382, SEQ ID
NO: 1884 / SEQ ID NO: 7383, SEQ ID NO: 1885 / SEQ ID NO: 7384, SEQ ID NO: 1886 /

SEQ ID NO: 7385, SEQ ID NO: 1887 / SEQ ID NO: 7386, SEQ ID NO: 1888 / SEQ ID NO:
7387, SEQ ID NO: 1889 / SEQ ID NO: 7388, SEQ ID NO: 1891 / SEQ ID NO: 7389, SEQ ID
NO: 1892 / SEQ ID NO: 7390, SEQ ID NO: 1893 / SEQ ID NO: 7391, SEQ ID NO: 1894 /
SEQ ID NO: 7392, SEQ ID NO: 1895 / SEQ ID NO: 7393, SEQ ID NO: 1896 / SEQ ID NO:
7394, SEQ ID NO: 1897 / SEQ ID NO: 7395, SEQ ID NO: 1898 / SEQ ID NO: 7396, SEQ ID
NO: 1899 / SEQ ID NO: 7397, SEQ ID NO: 1900 / SEQ ID NO: 7398, SEQ ID NO: 1901 /
SEQ ID NO: 7399, SEQ ID NO: 1903 / SEQ ID NO: 7400, SEQ ID NO: 1904 / SEQ ID NO:
7401, SEQ ID NO: 1905 / SEQ ID NO: 7402, SEQ ID NO: 1906 / SEQ ID NO: 7403, SEQ ID
NO: 1907 / SEQ ID NO: 7404, SEQ ID NO: 1908 / SEQ ID NO: 7405, SEQ ID NO: 1909 /
SEQ ID NO: 7406, SEQ ID NO: 1910 / SEQ ID NO: 7407, SEQ ID NO: 1911 / SEQ ID NO:
7408, SEQ ID NO: 1912 / SEQ ID NO: 7409, SEQ ID NO: 1913 / SEQ ID NO: 7410, SEQ ID
NO: 1914 / SEQ ID NO: 7411, SEQ ID NO: 1915 / SEQ ID NO: 7412, SEQ ID NO: 1917 /
SEQ ID NO: 7413, SEQ ID NO: 1918 / SEQ ID NO: 7414, SEQ ID NO: 1919 / SEQ ID NO:
7415, SEQ ID NO: 1920 / SEQ ID NO: 7416, SEQ ID NO: 1921 / SEQ ID NO: 7417, SEQ ID
NO: 1922 / SEQ ID NO: 7418, SEQ ID NO: 1923 / SEQ ID NO: 7419, SEQ ID NO: 1924 /
SEQ ID NO: 7420, SEQ ID NO: 1925 / SEQ ID NO: 7421, SEQ ID NO: 1926 / SEQ ID NO:
7422, SEQ ID NO: 1927 / SEQ ID NO: 7423, SEQ ID NO: 1928 / SEQ ID NO: 7424, SEQ ID
NO: 1929 / SEQ ID NO: 7425, SEQ ID NO: 1930 / SEQ ID NO: 7426, SEQ ID NO: 1931 /
SEQ ID NO: 7427, SEQ ID NO: 1932 / SEQ ID NO: 7428, SEQ ID NO: 1933 / SEQ ID NO:
7429, SEQ ID NO: 1934 / SEQ ID NO: 7430, SEQ ID NO: 1935 / SEQ ID NO: 7431, SEQ ID
NO: 1936 / SEQ ID NO: 7432, SEQ ID NO: 1937 / SEQ ID NO: 7433, SEQ ID NO: 1938 /
SEQ ID NO: 7434, SEQ ID NO: 1939 / SEQ ID NO: 7435, SEQ ID NO: 1940 / SEQ ID NO:
7436, SEQ ID NO: 2215 / SEQ ID NO: 7437, SEQ ID NO: 2216 / SEQ ID NO: 7438, SEQ ID
NO: 2217 / SEQ ID NO: 7439, SEQ ID NO: 2218 / SEQ ID NO: 7440, SEQ ID NO: 2219 /
SEQ ID NO: 7441, SEQ ID NO: 2220 / SEQ ID NO: 7442, SEQ ID NO: 2221 / SEQ ID NO:
7443, SEQ ID NO: 2222 / SEQ ID NO: 7444, SEQ ID NO: 2223 / SEQ ID NO: 7445, SEQ ID
NO: 2224 / SEQ ID NO: 7446, SEQ ID NO: 2225 / SEQ ID NO: 7447,
SEQ ID NO: 2226 / SEQ ID NO: 7448, SEQ ID NO: 2227 / SEQ ID NO: 7449, SEQ ID NO:
2228 / SEQ ID NO: 7450, SEQ ID NO: 2229 / SEQ ID NO: 7451, SEQ ID NO: 2230 / SEQ ID
NO: 7452, SEQ ID NO: 2231 / SEQ ID NO: 7453, SEQ ID NO: 2232 / SEQ ID NO: 7454, SEQ
ID NO: 2233 / SEQ ID NO: 7455, SEQ ID NO: 2234 / SEQ ID NO: 7456, SEQ ID NO: 2235 /
SEQ ID NO: 7457, SEQ ID NO: 2236 / SEQ ID NO: 7458, SEQ ID NO: 2237 / SEQ ID NO:
7459, SEQ ID NO: 2238 / SEQ ID NO: 7460, SEQ ID NO: 2239 / SEQ ID NO: 7461, SEQ ID
NO: 2240 / SEQ ID NO: 7462, SEQ ID NO: 2241 / SEQ ID NO: 7463, SEQ ID NO: 2242 /
SEQ ID NO: 7464, SEQ ID NO: 2243 / SEQ ID NO: 7465, SEQ ID NO: 2244 / SEQ ID NO:
7466, SEQ ID NO: 2245 / SEQ ID NO: 7467, SEQ ID NO: 2246 / SEQ ID NO: 7468, SEQ ID
NO: 2247 / SEQ ID NO: 7469, SEQ ID NO: 2248 / SEQ ID NO: 7470, SEQ ID NO: 2249 /
SEQ ID NO: 7471, SEQ ID NO: 2250 / SEQ ID NO: 7472, SEQ ID NO: 2251 / SEQ ID NO:
7473, SEQ ID NO: 2252 / SEQ ID NO: 7474, SEQ ID NO: 2253 / SEQ ID NO: 7475, SEQ ID
NO: 2254 / SEQ ID NO: 7476, SEQ ID NO: 2255 / SEQ ID NO: 7477, SEQ ID NO: 2256 /
SEQ ID NO: 7478, SEQ ID NO: 2257 / SEQ ID NO: 7479, SEQ ID NO: 2258 / SEQ ID NO:
7480, SEQ ID NO: 2259 / SEQ ID NO: 7481, SEQ ID NO: 2260 / SEQ ID NO: 7482, SEQ ID
NO: 2261 / SEQ ID NO: 7483, SEQ ID NO: 2262 / SEQ ID NO: 7484, SEQ ID NO: 2263 /
SEQ ID NO: 7485, SEQ ID NO: 2264 / SEQ ID NO: 7486, SEQ ID NO: 2265 / SEQ ID NO:
7487, SEQ ID NO: 2266 / SEQ ID NO: 7488, SEQ ID NO: 2267 / SEQ ID NO: 7489, SEQ ID
NO: 2268 / SEQ ID NO: 7490, SEQ ID NO: 2269 / SEQ ID NO: 7491, SEQ ID NO: 2270 /
SEQ ID NO: 7492, SEQ ID NO: 2271 / SEQ ID NO: 7493, SEQ ID NO: 2272 / SEQ ID NO:
7494, SEQ ID NO: 2273 / SEQ ID NO: 7495, SEQ ID NO: 2274 / SEQ ID NO: 7496, SEQ ID
NO: 2275 / SEQ ID NO: 7497, SEQ ID NO: 2276 / SEQ ID NO: 7498, SEQ ID NO: 2277 /
SEQ ID NO: 7499, SEQ ID NO: 2278 / SEQ ID NO: 7500, SEQ ID NO: 2279 / SEQ ID NO:
7501, SEQ ID NO: 2280 / SEQ ID NO: 7502, SEQ ID NO: 2281 / SEQ ID NO: 7503, SEQ ID
NO: 2282 / SEQ ID NO: 7504, SEQ ID NO: 2283 / SEQ ID NO: 7505, SEQ ID NO: 2284 /

SEQ ID NO: 7506, SEQ ID NO: 2285 / SEQ ID NO: 7507, SEQ ID NO: 2286 / SEQ ID NO:
7508, SEQ ID NO: 2287 / SEQ ID NO: 7509, SEQ ID NO: 2288 / SEQ ID NO: 7510, SEQ ID
NO: 2289 / SEQ ID NO: 7511, SEQ ID NO: 2290 / SEQ ID NO: 7512, SEQ ID NO: 2291 /
SEQ ID NO: 7513, SEQ ID NO: 2292 / SEQ ID NO: 7514, SEQ ID NO: 2293 / SEQ ID NO:
7515, SEQ ID NO: 2294 / SEQ ID NO: 7516, SEQ ID NO: 2295 / SEQ ID NO: 7517, SEQ ID
NO: 2296 / SEQ ID NO: 7518, SEQ ID NO: 2297 / SEQ ID NO: 7519, SEQ ID NO: 2298 /
SEQ ID NO: 7520, SEQ ID NO: 2299 / SEQ ID NO: 7521, SEQ ID NO: 2300 / SEQ ID NO:
7522, SEQ ID NO: 2301 / SEQ ID NO: 7523, SEQ ID NO: 2302 / SEQ ID NO: 7524, SEQ ID
NO: 2303 / SEQ ID NO: 7525, SEQ ID NO: 2304 / SEQ ID NO: 7526, SEQ ID NO: 2305 /
SEQ ID NO: 7527, SEQ ID NO: 2306 / SEQ ID NO: 7528, SEQ ID NO: 2307 / SEQ ID NO:
7529, SEQ ID NO: 2308 / SEQ ID NO: 7530, SEQ ID NO: 2309 / SEQ ID NO: 7531, SEQ ID
NO: 2310 / SEQ ID NO: 7532, SEQ ID NO: 2311 / SEQ ID NO: 7533, SEQ ID NO: 2312 /
SEQ ID NO: 7534, SEQ ID NO: 2313 / SEQ ID NO: 7535, SEQ ID NO: 2314 / SEQ ID NO:
7536, SEQ ID NO: 2315 / SEQ ID NO: 7537, SEQ ID NO: 2316 / SEQ ID NO: 7538, SEQ ID
NO: 2317 / SEQ ID NO: 7539, SEQ ID NO: 2318 / SEQ ID NO: 7540, SEQ ID NO: 2319 /
SEQ ID NO: 7541, SEQ ID NO: 2320 / SEQ ID NO: 7542, SEQ ID NO: 2321 / SEQ ID NO:
7543, SEQ ID NO: 2322 / SEQ ID NO: 7544, SEQ ID NO: 2323 / SEQ ID NO: 7545, SEQ ID
NO: 2324 / SEQ ID NO: 7546, SEQ ID NO: 2325 / SEQ ID NO: 7547,
SEQ ID NO: 2326 / SEQ ID NO: 7548, SEQ ID NO: 2327 / SEQ ID NO: 7549, SEQ ID NO:
2328 / SEQ ID NO: 7550, SEQ ID NO: 2329 / SEQ ID NO: 7551, SEQ ID NO: 2330 / SEQ ID
NO: 7552, SEQ ID NO: 2331 / SEQ ID NO: 7553, SEQ ID NO: 2332 / SEQ ID NO: 7554, SEQ
ID NO: 2333 / SEQ ID NO: 7555, SEQ ID NO: 2334 / SEQ ID NO: 7556, SEQ ID NO: 2335 /
SEQ ID NO: 7557, SEQ ID NO: 2336 / SEQ ID NO: 7558, SEQ ID NO: 2337 / SEQ ID NO:
7559, SEQ ID NO: 2338 / SEQ ID NO: 7560, SEQ ID NO: 2339 / SEQ ID NO: 7561, SEQ ID
NO: 2340 / SEQ ID NO: 7562, SEQ ID NO: 2341 / SEQ ID NO: 7563, SEQ ID NO: 2342 /
SEQ ID NO: 7564, SEQ ID NO: 2343 / SEQ ID NO: 7565, SEQ ID NO: 2344 / SEQ ID NO:
7566, SEQ ID NO: 2345 / SEQ ID NO: 7567, SEQ ID NO: 2346 / SEQ ID NO: 7568, SEQ ID
NO: 2347 / SEQ ID NO: 7569, SEQ ID NO: 2348 / SEQ ID NO: 7570, SEQ ID NO: 2349 /
SEQ ID NO: 7571, SEQ ID NO: 2350 / SEQ ID NO: 7572, SEQ ID NO: 2351 / SEQ ID NO:
7573, SEQ ID NO: 2352 / SEQ ID NO: 7574, SEQ ID NO: 2353 / SEQ ID NO: 7575, SEQ ID
NO: 2354 / SEQ ID NO: 7576, SEQ ID NO: 2355 / SEQ ID NO: 7577, SEQ ID NO: 2356 /
SEQ ID NO: 7578, SEQ ID NO: 2357 / SEQ ID NO: 7579, SEQ ID NO: 2358 / SEQ ID NO:
7580, SEQ ID NO: 2359 / SEQ ID NO: 7581, SEQ ID NO: 2360 / SEQ ID NO: 7582, SEQ ID
NO: 2361 / SEQ ID NO: 7583, SEQ ID NO: 2362 / SEQ ID NO: 7584, SEQ ID NO: 2363 /
SEQ ID NO: 7585, SEQ ID NO: 2364 / SEQ ID NO: 7586, SEQ ID NO: 2365 / SEQ ID NO:
7587, SEQ ID NO: 2366 / SEQ ID NO: 7588, SEQ ID NO: 2367 / SEQ ID NO: 7589, SEQ ID
NO: 2368 / SEQ ID NO: 7590, SEQ ID NO: 2369 / SEQ ID NO: 7591, SEQ ID NO: 2370 /
SEQ ID NO: 7592, SEQ ID NO: 2371 / SEQ ID NO: 7593, SEQ ID NO: 2373 / SEQ ID NO:
7594, SEQ ID NO: 2374 / SEQ ID NO: 7595, SEQ ID NO: 2375 / SEQ ID NO: 7596, SEQ ID
NO: 2376 / SEQ ID NO: 7597, SEQ ID NO: 2377 / SEQ ID NO: 7598, SEQ ID NO: 2378 /
SEQ ID NO: 7599, SEQ ID NO: 2379 / SEQ ID NO: 7600, SEQ ID NO: 2380 / SEQ ID NO:
7601, SEQ ID NO: 2381 / SEQ ID NO: 7602, SEQ ID NO: 2382 / SEQ ID NO: 7603, SEQ ID
NO: 2383 / SEQ ID NO: 7604, SEQ ID NO: 2384 / SEQ ID NO: 7605, SEQ ID NO: 2385 /
SEQ ID NO: 7606, SEQ ID NO: 2386 / SEQ ID NO: 7607, SEQ ID NO: 2387 / SEQ ID NO:
7608, SEQ ID NO: 2388 / SEQ ID NO: 7609, SEQ ID NO: 2389 / SEQ ID NO: 7610, SEQ ID
NO: 2390 / SEQ ID NO: 7611, SEQ ID NO: 2391 / SEQ ID NO: 7612, SEQ ID NO: 2392 /
SEQ ID NO: 7613, SEQ ID NO: 2393 / SEQ ID NO: 7614, SEQ ID NO: 2394 / SEQ ID NO:
7615, SEQ ID NO: 2395 / SEQ ID NO: 7616, SEQ ID NO: 2396 / SEQ ID NO: 7617, SEQ ID
NO: 2397 / SEQ ID NO: 7618, SEQ ID NO: 2398 / SEQ ID NO: 7619, SEQ ID NO: 2400 /
SEQ ID NO: 7620, SEQ ID NO: 2401 / SEQ ID NO: 7621, SEQ ID NO: 2402 / SEQ ID NO:
7622, SEQ ID NO: 2403 / SEQ ID NO: 7623, SEQ ID NO: 2404 / SEQ ID NO: 7624, SEQ ID
NO: 2405 / SEQ ID NO: 7625, SEQ ID NO: 2406 / SEQ ID NO: 7626, SEQ ID NO: 2407 /

SEQ ID NO: 7627, SEQ ID NO: 2408 / SEQ ID NO: 7628, SEQ ID NO: 2409 / SEQ ID NO:
7629, SEQ ID NO: 2410 / SEQ ID NO: 7630, SEQ ID NO: 2411 / SEQ ID NO: 7631, SEQ ID
NO: 2412 / SEQ ID NO: 7632, SEQ ID NO: 2413 / SEQ ID NO: 7633, SEQ ID NO: 2414 /
SEQ ID NO: 7634, SEQ ID NO: 2415 / SEQ ID NO: 7635, SEQ ID NO: 2416 / SEQ ID NO:
7636, SEQ ID NO: 2417 / SEQ ID NO: 7637, SEQ ID NO: 2418 / SEQ ID NO: 7638, SEQ ID
NO: 2419 / SEQ ID NO: 7639, SEQ ID NO: 2420 / SEQ ID NO: 7640, SEQ ID NO: 2421 /
SEQ ID NO: 7641, SEQ ID NO: 2422 / SEQ ID NO: 7642, SEQ ID NO: 2423 / SEQ ID NO:
7643, SEQ ID NO: 2424 / SEQ ID NO: 7644, SEQ ID NO: 2425 / SEQ ID NO: 7645, SEQ ID
NO: 2426 / SEQ ID NO: 7646, SEQ ID NO: 2427 / SEQ ID NO: 7647,
SEQ ID NO: 2428 / SEQ ID NO: 7648, SEQ ID NO: 2429 / SEQ ID NO: 7649, SEQ ID NO:
2430 / SEQ ID NO: 7650, SEQ ID NO: 2431 / SEQ ID NO: 7651, SEQ ID NO: 2432 / SEQ ID
NO: 7652, SEQ ID NO: 2433 / SEQ ID NO: 7653, SEQ ID NO: 2435 / SEQ ID NO: 7654, SEQ
ID NO: 2436 / SEQ ID NO: 7655, SEQ ID NO: 2437 / SEQ ID NO: 7656, SEQ ID NO: 2438 /
SEQ ID NO: 7657, SEQ ID NO: 2439 / SEQ ID NO: 7658, SEQ ID NO: 2440 / SEQ ID NO:
7659, SEQ ID NO: 2441 / SEQ ID NO: 7660, SEQ ID NO: 2442 / SEQ ID NO: 7661, SEQ ID
NO: 2443 / SEQ ID NO: 7662, SEQ ID NO: 2444 / SEQ ID NO: 7663, SEQ ID NO: 2445 /
SEQ ID NO: 7664, SEQ ID NO: 2446 / SEQ ID NO: 7665, SEQ ID NO: 2447 / SEQ ID NO:
7666, SEQ ID NO: 2448 / SEQ ID NO: 7667, SEQ ID NO: 2451 / SEQ ID NO: 7668, SEQ ID
NO: 2452 / SEQ ID NO: 7669, SEQ ID NO: 2453 / SEQ ID NO: 7670, SEQ ID NO: 2455 /
SEQ ID NO: 7671, SEQ ID NO: 2572 / SEQ ID NO: 7672, SEQ ID NO: 2573 / SEQ ID NO:
7673, SEQ ID NO: 2575 / SEQ ID NO: 7674, SEQ ID NO: 2576 / SEQ ID NO: 7675, SEQ ID
NO: 2578 / SEQ ID NO: 7676, SEQ ID NO: 2579 / SEQ ID NO: 7677, SEQ ID NO: 2580 /
SEQ ID NO: 7678, SEQ ID NO: 2581 / SEQ ID NO: 7679, SEQ ID NO: 2582 / SEQ ID NO:
7680, SEQ ID NO: 2583 / SEQ ID NO: 7681, SEQ ID NO: 2584 / SEQ ID NO: 7682, SEQ ID
NO: 2585 / SEQ ID NO: 7683, SEQ ID NO: 2586 / SEQ ID NO: 7684, SEQ ID NO: 2587 /
SEQ ID NO: 7685, SEQ ID NO: 2588 / SEQ ID NO: 7686, SEQ ID NO: 2589 / SEQ ID NO:
7687, SEQ ID NO: 2590 / SEQ ID NO: 7688, SEQ ID NO: 2591 / SEQ ID NO: 7689, SEQ ID
NO: 2592 / SEQ ID NO: 7690, SEQ ID NO: 2593 / SEQ ID NO: 7691, SEQ ID NO: 2594 /
SEQ ID NO: 7692, SEQ ID NO: 2595 / SEQ ID NO: 7693, SEQ ID NO: 2596 / SEQ ID NO:
7694, SEQ ID NO: 2597 / SEQ ID NO: 7695, SEQ ID NO: 2598 / SEQ ID NO: 7696, SEQ ID
NO: 2599 / SEQ ID NO: 7697, SEQ ID NO: 2601 / SEQ ID NO: 7698, SEQ ID NO: 2602 /
SEQ ID NO: 7699, SEQ ID NO: 2603 / SEQ ID NO: 7700, SEQ ID NO: 2604 / SEQ ID NO:
7701, SEQ ID NO: 2606 / SEQ ID NO: 7702, SEQ ID NO: 2607 / SEQ ID NO: 7703, SEQ ID
NO: 2608 / SEQ ID NO: 7704, SEQ ID NO: 2609 / SEQ ID NO: 7705, SEQ ID NO: 2610 /
SEQ ID NO: 7706, SEQ ID NO: 2611 / SEQ ID NO: 7707, SEQ ID NO: 2612 / SEQ ID NO:
7708, SEQ ID NO: 2613 / SEQ ID NO: 7709, SEQ ID NO: 2614 / SEQ ID NO: 7710, SEQ ID
NO: 2616 / SEQ ID NO: 7711, SEQ ID NO: 2617 / SEQ ID NO: 7712, SEQ ID NO: 2618 /
SEQ ID NO: 7713, SEQ ID NO: 2619 / SEQ ID NO: 7714, SEQ ID NO: 2620 / SEQ ID NO:
7715, SEQ ID NO: 2621 / SEQ ID NO: 7716, SEQ ID NO: 2623 / SEQ ID NO: 7717, SEQ ID
NO: 2624 / SEQ ID NO: 7718, SEQ ID NO: 2625 / SEQ ID NO: 7719, SEQ ID NO: 2626 /
SEQ ID NO: 7720, SEQ ID NO: 2628 / SEQ ID NO: 7721, SEQ ID NO: 2629 / SEQ ID NO:
7722, SEQ ID NO: 2630 / SEQ ID NO: 7723, SEQ ID NO: 2631 / SEQ ID NO: 7724, SEQ ID
NO: 2632 / SEQ ID NO: 7725, SEQ ID NO: 2633 / SEQ ID NO: 7726, SEQ ID NO: 2634 /
SEQ ID NO: 7727, SEQ ID NO: 2635 / SEQ ID NO: 7728, SEQ ID NO: 2636 / SEQ ID NO:
7729, SEQ ID NO: 2637 / SEQ ID NO: 7730, SEQ ID NO: 2638 / SEQ ID NO: 7731, SEQ ID
NO: 2639 / SEQ ID NO: 7732, SEQ ID NO: 2640 / SEQ ID NO: 7733, SEQ ID NO: 2641 /
SEQ ID NO: 7734, SEQ ID NO: 2642 / SEQ ID NO: 7735, SEQ ID NO: 2643 / SEQ ID NO:
7736, SEQ ID NO: 2644 / SEQ ID NO: 7737, SEQ ID NO: 2645 / SEQ ID NO: 7738, SEQ ID
NO: 2646 / SEQ ID NO: 7739, SEQ ID NO: 2648 / SEQ ID NO: 7740, SEQ ID NO: 2649 /
SEQ ID NO: 7741, SEQ ID NO: 2650 / SEQ ID NO: 7742, SEQ ID NO: 2651 / SEQ ID NO:
7743, SEQ ID NO: 2652 / SEQ ID NO: 7744, SEQ ID NO: 2653 / SEQ ID NO: 7745, SEQ ID
NO: 2654 / SEQ ID NO: 7746, SEQ ID NO: 2655 / SEQ ID NO: 7747,

SEQ ID NO: 2656 / SEQ ID NO: 7748, SEQ ID NO: 2657 / SEQ ID NO: 7749, SEQ ID NO:
2658 / SEQ ID NO: 7750, SEQ ID NO: 2659 / SEQ ID NO: 7751, SEQ ID NO: 2660 / SEQ ID
NO: 7752, SEQ ID NO: 2661 / SEQ ID NO: 7753, SEQ ID NO: 2662 / SEQ ID NO: 7754, SEQ
ID NO: 2663 / SEQ ID NO: 7755, SEQ ID NO: 2664 / SEQ ID NO: 7756, SEQ ID NO: 2666 /
SEQ ID NO: 7757, SEQ ID NO: 2667 / SEQ ID NO: 7758, SEQ ID NO: 2668 / SEQ ID NO:
7759, SEQ ID NO: 2669 / SEQ ID NO: 7760, SEQ ID NO: 2670 / SEQ ID NO: 7761, SEQ ID
NO: 2671 / SEQ ID NO: 7762, SEQ ID NO: 2672 / SEQ ID NO: 7763, SEQ ID NO: 2673 /
SEQ ID NO: 7764, SEQ ID NO: 2675 / SEQ ID NO: 7765, SEQ ID NO: 2676 / SEQ ID NO:
7766, SEQ ID NO: 2678 / SEQ ID NO: 7767, SEQ ID NO: 2679 / SEQ ID NO: 7768, SEQ ID
NO: 2680 / SEQ ID NO: 7769, SEQ ID NO: 2681 / SEQ ID NO: 7770, SEQ ID NO: 2682 /
SEQ ID NO: 7771, SEQ ID NO: 2683 / SEQ ID NO: 7772, SEQ ID NO: 2684 / SEQ ID NO:
7773, SEQ ID NO: 2685 / SEQ ID NO: 7774, SEQ ID NO: 2686 / SEQ ID NO: 7775, SEQ ID
NO: 2687 / SEQ ID NO: 7776, SEQ ID NO: 2688 / SEQ ID NO: 7777, SEQ ID NO: 2689 /
SEQ ID NO: 7778, SEQ ID NO: 2690 / SEQ ID NO: 7779, SEQ ID NO: 2691 / SEQ ID NO:
7780, SEQ ID NO: 2692 / SEQ ID NO: 7781, SEQ ID NO: 2693 / SEQ ID NO: 7782, SEQ ID
NO: 2694 / SEQ ID NO: 7783, SEQ ID NO: 2695 / SEQ ID NO: 7784, SEQ ID NO: 2696 /
SEQ ID NO: 7785, SEQ ID NO: 2697 / SEQ ID NO: 7786, SEQ ID NO: 2698 / SEQ ID NO:
7787, SEQ ID NO: 2700 / SEQ ID NO: 7788, SEQ ID NO: 2701 / SEQ ID NO: 7789, SEQ ID
NO: 2702 / SEQ ID NO: 7790, SEQ ID NO: 2703 / SEQ ID NO: 7791, SEQ ID NO: 2704 /
SEQ ID NO: 7792, SEQ ID NO: 2705 / SEQ ID NO: 7793, SEQ ID NO: 2706 / SEQ ID NO:
7794, SEQ ID NO: 2707 / SEQ ID NO: 7795, SEQ ID NO: 2708 / SEQ ID NO: 7796, SEQ ID
NO: 2709 / SEQ ID NO: 7797, SEQ ID NO: 2710 / SEQ ID NO: 7798, SEQ ID NO: 2711 /
SEQ ID NO: 7799, SEQ ID NO: 2712 / SEQ ID NO: 7800, SEQ ID NO: 2713 / SEQ ID NO:
7801, SEQ ID NO: 2714 / SEQ ID NO: 7802, SEQ ID NO: 2715 / SEQ ID NO: 7803, SEQ ID
NO: 2716 / SEQ ID NO: 7804, SEQ ID NO: 2717 / SEQ ID NO: 7805, SEQ ID NO: 2718 /
SEQ ID NO: 7806, SEQ ID NO: 2719 / SEQ ID NO: 7807, SEQ ID NO: 2720 / SEQ ID NO:
7808, SEQ ID NO: 2721 / SEQ ID NO: 7809, SEQ ID NO: 2722 / SEQ ID NO: 7810, SEQ ID
NO: 2723 / SEQ ID NO: 7811, SEQ ID NO: 2724 / SEQ ID NO: 7812, SEQ ID NO: 2725 /
SEQ ID NO: 7813, SEQ ID NO: 2726 / SEQ ID NO: 7814, SEQ ID NO: 2729 / SEQ ID NO:
7815, SEQ ID NO: 2731 / SEQ ID NO: 7816, SEQ ID NO: 2732 / SEQ ID NO: 7817, SEQ ID
NO: 2733 / SEQ ID NO: 7818, SEQ ID NO: 2734 / SEQ ID NO: 7819, SEQ ID NO: 2735 /
SEQ ID NO: 7820, SEQ ID NO: 2736 / SEQ ID NO: 7821, SEQ ID NO: 2737 / SEQ ID NO:
7822, SEQ ID NO: 2738 / SEQ ID NO: 7823, SEQ ID NO: 2739 / SEQ ID NO: 7824, SEQ ID
NO: 2741 / SEQ ID NO: 7825, SEQ ID NO: 2742 / SEQ ID NO: 7826, SEQ ID NO: 2743 /
SEQ ID NO: 7827, SEQ ID NO: 2744 / SEQ ID NO: 7828, SEQ ID NO: 2746 / SEQ ID NO:
7829, SEQ ID NO: 2747 / SEQ ID NO: 7830, SEQ ID NO: 2748 / SEQ ID NO: 7831, SEQ ID
NO: 2749 / SEQ ID NO: 7832, SEQ ID NO: 2750 / SEQ ID NO: 7833, SEQ ID NO: 2751 /
SEQ ID NO: 7834, SEQ ID NO: 2752 / SEQ ID NO: 7835, SEQ ID NO: 2753 / SEQ ID NO:
7836, SEQ ID NO: 2754 / SEQ ID NO: 7837, SEQ ID NO: 2755 / SEQ ID NO: 7838, SEQ ID
NO: 2757 / SEQ ID NO: 7839, SEQ ID NO: 2758 / SEQ ID NO: 7840, SEQ ID NO: 2759 /
SEQ ID NO: 7841, SEQ ID NO: 2760 / SEQ ID NO: 7842, SEQ ID NO: 2761 / SEQ ID NO:
7843, SEQ ID NO: 2762 / SEQ ID NO: 7844, SEQ ID NO: 2763 / SEQ ID NO: 7845, SEQ ID
NO: 2764 / SEQ ID NO: 7846, SEQ ID NO: 2765 / SEQ ID NO: 7847,
SEQ ID NO: 2766 / SEQ ID NO: 7848, SEQ ID NO: 2768 / SEQ ID NO: 7849, SEQ ID NO:
2769 / SEQ ID NO: 7850, SEQ ID NO: 2770 / SEQ ID NO: 7851, SEQ ID NO: 2771 / SEQ ID
NO: 7852, SEQ ID NO: 2772 / SEQ ID NO: 7853, SEQ ID NO: 2773 / SEQ ID NO: 7854, SEQ
ID NO: 2774 / SEQ ID NO: 7855, SEQ ID NO: 2775 / SEQ ID NO: 7856, SEQ ID NO: 2776 /
SEQ ID NO: 7857, SEQ ID NO: 2777 / SEQ ID NO: 7858, SEQ ID NO: 2778 / SEQ ID NO:
7859, SEQ ID NO: 2779 / SEQ ID NO: 7860, SEQ ID NO: 2780 / SEQ ID NO: 7861, SEQ ID
NO: 2782 / SEQ ID NO: 7862, SEQ ID NO: 2783 / SEQ ID NO: 7863, SEQ ID NO: 2784 /
SEQ ID NO: 7864, SEQ ID NO: 2785 / SEQ ID NO: 7865, SEQ ID NO: 2786 / SEQ ID NO:
7866, SEQ ID NO: 2787 / SEQ ID NO: 7867, SEQ ID NO: 2788 / SEQ ID NO: 7868, SEQ ID

NO: 2789 / SEQ ID NO: 7869, SEQ ID NO: 2790 / SEQ ID NO: 7870, SEQ ID NO: 2792 /
SEQ ID NO: 7871, SEQ ID NO: 2793 / SEQ ID NO: 7872, SEQ ID NO: 2794 / SEQ ID NO:
7873, SEQ ID NO: 2795 / SEQ ID NO: 7874, SEQ ID NO: 2796 / SEQ ID NO: 7875, SEQ ID
NO: 2798 / SEQ ID NO: 7876, SEQ ID NO: 2799 / SEQ ID NO: 7877, SEQ ID NO: 2800 /
SEQ ID NO: 7878, SEQ ID NO: 2801 / SEQ ID NO: 7879, SEQ ID NO: 2802 / SEQ ID NO:
7880, SEQ ID NO: 2803 / SEQ ID NO: 7881, SEQ ID NO: 2804 / SEQ ID NO: 7882, SEQ ID
NO: 2805 / SEQ ID NO: 7883, SEQ ID NO: 2806 / SEQ ID NO: 7884, SEQ ID NO: 2807 /
SEQ ID NO: 7885, SEQ ID NO: 2808 / SEQ ID NO: 7886, SEQ ID NO: 2809 / SEQ ID NO:
7887, SEQ ID NO: 2810 / SEQ ID NO: 7888, SEQ ID NO: 2811 / SEQ ID NO: 7889, SEQ ID
NO: 2812 / SEQ ID NO: 7890, SEQ ID NO: 2813 / SEQ ID NO: 7891, SEQ ID NO: 2814 /
SEQ ID NO: 7892, SEQ ID NO: 2815 / SEQ ID NO: 7893, SEQ ID NO: 2816 / SEQ ID NO:
7894, SEQ ID NO: 2817 / SEQ ID NO: 7895, SEQ ID NO: 2818 / SEQ ID NO: 7896, SEQ ID
NO: 2819 / SEQ ID NO: 7897, SEQ ID NO: 2820 / SEQ ID NO: 7898, SEQ ID NO: 2821 /
SEQ ID NO: 7899, SEQ ID NO: 2822 / SEQ ID NO: 7900, SEQ ID NO: 2823 / SEQ ID NO:
7901, SEQ ID NO: 2824 / SEQ ID NO: 7902, SEQ ID NO: 2825 / SEQ ID NO: 7903, SEQ ID
NO: 2826 / SEQ ID NO: 7904, SEQ ID NO: 2827 / SEQ ID NO: 7905, SEQ ID NO: 2828 /
SEQ ID NO: 7906, SEQ ID NO: 2829 / SEQ ID NO: 7907, SEQ ID NO: 2830 / SEQ ID NO:
7908, SEQ ID NO: 2831 / SEQ ID NO: 7909, SEQ ID NO: 2832 / SEQ ID NO: 7910, SEQ ID
NO: 2833 / SEQ ID NO: 7911, SEQ ID NO: 2834 / SEQ ID NO: 7912, SEQ ID NO: 2835 /
SEQ ID NO: 7913, SEQ ID NO: 2836 / SEQ ID NO: 7914, SEQ ID NO: 2837 / SEQ ID NO:
7915, SEQ ID NO: 2839 / SEQ ID NO: 7916, SEQ ID NO: 2840 / SEQ ID NO: 7917, SEQ ID
NO: 2841 / SEQ ID NO: 7918, SEQ ID NO: 2842 / SEQ ID NO: 7919, SEQ ID NO: 2843 /
SEQ ID NO: 7920, SEQ ID NO: 2844 / SEQ ID NO: 7921, SEQ ID NO: 2845 / SEQ ID NO:
7922, SEQ ID NO: 2846 / SEQ ID NO: 7923, SEQ ID NO: 2847 / SEQ ID NO: 7924, SEQ ID
NO: 2848 / SEQ ID NO: 7925, SEQ ID NO: 2849 / SEQ ID NO: 7926, SEQ ID NO: 2850 /
SEQ ID NO: 7927, SEQ ID NO: 2851 / SEQ ID NO: 7928, SEQ ID NO: 2852 / SEQ ID NO:
7929, SEQ ID NO: 2853 / SEQ ID NO: 7930, SEQ ID NO: 2854 / SEQ ID NO: 7931, SEQ ID
NO: 2855 / SEQ ID NO: 7932, SEQ ID NO: 2858 / SEQ ID NO: 7933, SEQ ID NO: 2859 /
SEQ ID NO: 7934, SEQ ID NO: 2860 / SEQ ID NO: 7935, SEQ ID NO: 2861 / SEQ ID NO:
7936, SEQ ID NO: 2862 / SEQ ID NO: 7937, SEQ ID NO: 2863 / SEQ ID NO: 7938, SEQ ID
NO: 2865 / SEQ ID NO: 7939, SEQ ID NO: 2866 / SEQ ID NO: 7940, SEQ ID NO: 2867 /
SEQ ID NO: 7941, SEQ ID NO: 2868 / SEQ ID NO: 7942, SEQ ID NO: 2869 / SEQ ID NO:
7943, SEQ ID NO: 2870 / SEQ ID NO: 7944, SEQ ID NO: 2871 / SEQ ID NO: 7945, SEQ ID
NO: 2872 / SEQ ID NO: 7946, SEQ ID NO: 2873 / SEQ ID NO: 7947,
SEQ ID NO: 2874 / SEQ ID NO: 7948, SEQ ID NO: 2875 / SEQ ID NO: 7949, SEQ ID NO:
2877 / SEQ ID NO: 7950, SEQ ID NO: 2878 / SEQ ID NO: 7951, SEQ ID NO: 2879 / SEQ ID
NO: 7952, SEQ ID NO: 2880 / SEQ ID NO: 7953, SEQ ID NO: 2881 / SEQ ID NO: 7954, SEQ
ID NO: 2882 / SEQ ID NO: 7955, SEQ ID NO: 2883 / SEQ ID NO: 7956, SEQ ID NO: 2884 /
SEQ ID NO: 7957, SEQ ID NO: 2885 / SEQ ID NO: 7958, SEQ ID NO: 2886 / SEQ ID NO:
7959, SEQ ID NO: 2887 / SEQ ID NO: 7960, SEQ ID NO: 2888 / SEQ ID NO: 7961, SEQ ID
NO: 2889 / SEQ ID NO: 7962, SEQ ID NO: 2891 / SEQ ID NO: 7963, SEQ ID NO: 2892 /
SEQ ID NO: 7964, SEQ ID NO: 2893 / SEQ ID NO: 7965, SEQ ID NO: 2894 / SEQ ID NO:
7966, SEQ ID NO: 2895 / SEQ ID NO: 7967, SEQ ID NO: 2896 / SEQ ID NO: 7968, SEQ ID
NO: 2897 / SEQ ID NO: 7969, SEQ ID NO: 2898 / SEQ ID NO: 7970, SEQ ID NO: 2899 /
SEQ ID NO: 7971, SEQ ID NO: 2900 / SEQ ID NO: 7972, SEQ ID NO: 2901 / SEQ ID NO:
7973, SEQ ID NO: 2903 / SEQ ID NO: 7974, SEQ ID NO: 2904 / SEQ ID NO: 7975, SEQ ID
NO: 2905 / SEQ ID NO: 7976, SEQ ID NO: 2906 / SEQ ID NO: 7977, SEQ ID NO: 2907 /
SEQ ID NO: 7978, SEQ ID NO: 2908 / SEQ ID NO: 7979, SEQ ID NO: 2909 / SEQ ID NO:
7980, SEQ ID NO: 2910 / SEQ ID NO: 7981, SEQ ID NO: 2911 / SEQ ID NO: 7982, SEQ ID
NO: 2912 / SEQ ID NO: 7983, SEQ ID NO: 2913 / SEQ ID NO: 7984, SEQ ID NO: 2914 /
SEQ ID NO: 7985, SEQ ID NO: 2915 / SEQ ID NO: 7986, SEQ ID NO: 2916 / SEQ ID NO:
7987, SEQ ID NO: 2917 / SEQ ID NO: 7988, SEQ ID NO: 2918 / SEQ ID NO: 7989, SEQ ID

NO: 2919 / SEQ ID NO: 7990, SEQ ID NO: 2920 / SEQ ID NO: 7991, SEQ ID NO: 2921 /
SEQ ID NO: 7992, SEQ ID NO: 2922 / SEQ ID NO: 7993, SEQ ID NO: 2923 / SEQ ID NO:
7994, SEQ ID NO: 2924 / SEQ ID NO: 7995, SEQ ID NO: 2925 / SEQ ID NO: 7996, SEQ ID
NO: 2927 / SEQ ID NO: 7997, SEQ ID NO: 2928 / SEQ ID NO: 7998, SEQ ID NO: 2929 /
SEQ ID NO: 7999, SEQ ID NO: 2930 / SEQ ID NO: 8000, SEQ ID NO: 2931 / SEQ ID NO:
8001, SEQ ID NO: 2932 / SEQ ID NO: 8002, SEQ ID NO: 2933 / SEQ ID NO: 8003, SEQ ID
NO: 2934 / SEQ ID NO: 8004, SEQ ID NO: 2935 / SEQ ID NO: 8005, SEQ ID NO: 2936 /
SEQ ID NO: 8006, SEQ ID NO: 2937 / SEQ ID NO: 8007, SEQ ID NO: 2938 / SEQ ID NO:
8008, SEQ ID NO: 2939 / SEQ ID NO: 8009, SEQ ID NO: 2941 / SEQ ID NO: 8010, SEQ ID
NO: 2942 / SEQ ID NO: 8011, SEQ ID NO: 2943 / SEQ ID NO: 8012, SEQ ID NO: 2944 /
SEQ ID NO: 8013, SEQ ID NO: 2945 / SEQ ID NO: 8014, SEQ ID NO: 2946 / SEQ ID NO:
8015, SEQ ID NO: 2947 / SEQ ID NO: 8016, SEQ ID NO: 2948 / SEQ ID NO: 8017, SEQ ID
NO: 2949 / SEQ ID NO: 8018, SEQ ID NO: 2950 / SEQ ID NO: 8019, SEQ ID NO: 2951 /
SEQ ID NO: 8020, SEQ ID NO: 2952 / SEQ ID NO: 8021, SEQ ID NO: 2953 / SEQ ID NO:
8022, SEQ ID NO: 2954 / SEQ ID NO: 8023, SEQ ID NO: 2955 / SEQ ID NO: 8024, SEQ ID
NO: 2956 / SEQ ID NO: 8025, SEQ ID NO: 2957 / SEQ ID NO: 8026, SEQ ID NO: 2958 /
SEQ ID NO: 8027, SEQ ID NO: 2959 / SEQ ID NO: 8028, SEQ ID NO: 2960 / SEQ ID NO:
8029, SEQ ID NO: 2961 / SEQ ID NO: 8030, SEQ ID NO: 2962 / SEQ ID NO: 8031, SEQ ID
NO: 2963 / SEQ ID NO: 8032, SEQ ID NO: 2964 / SEQ ID NO: 8033, SEQ ID NO: 2965 /
SEQ ID NO: 8034, SEQ ID NO: 2966 / SEQ ID NO: 8035, SEQ ID NO: 2967 / SEQ ID NO:
8036, SEQ ID NO: 2968 / SEQ ID NO: 8037, SEQ ID NO: 2969 / SEQ ID NO: 8038, SEQ ID
NO: 2970 / SEQ ID NO: 8039, SEQ ID NO: 2971 / SEQ ID NO: 8040, SEQ ID NO: 2972 /
SEQ ID NO: 8041, SEQ ID NO: 2973 / SEQ ID NO: 8042, SEQ ID NO: 2974 / SEQ ID NO:
8043, SEQ ID NO: 2975 / SEQ ID NO: 8044, SEQ ID NO: 2976 / SEQ ID NO: 8045, SEQ ID
NO: 2977 / SEQ ID NO: 8046, SEQ ID NO: 2978 / SEQ ID NO: 8047,
SEQ ID NO: 2979 / SEQ ID NO: 8048, SEQ ID NO: 2981 / SEQ ID NO: 8049, SEQ ID NO:
2982 / SEQ ID NO: 8050, SEQ ID NO: 2983 / SEQ ID NO: 8051, SEQ ID NO: 2984 / SEQ ID
NO: 8052, SEQ ID NO: 2985 / SEQ ID NO: 8053, SEQ ID NO: 2986 / SEQ ID NO: 8054, SEQ
ID NO: 2987 / SEQ ID NO: 8055, SEQ ID NO: 2988 / SEQ ID NO: 8056, SEQ ID NO: 2989 /
SEQ ID NO: 8057, SEQ ID NO: 2990 / SEQ ID NO: 8058, SEQ ID NO: 2991 / SEQ ID NO:
8059, SEQ ID NO: 2992 / SEQ ID NO: 8060, SEQ ID NO: 2993 / SEQ ID NO: 8061, SEQ ID
NO: 2994 / SEQ ID NO: 8062, SEQ ID NO: 2995 / SEQ ID NO: 8063, SEQ ID NO: 2996 /
SEQ ID NO: 8064, SEQ ID NO: 2997 / SEQ ID NO: 8065, SEQ ID NO: 2998 / SEQ ID NO:
8066, SEQ ID NO: 2999 / SEQ ID NO: 8067, SEQ ID NO: 3000 / SEQ ID NO: 8068, SEQ ID
NO: 3001 / SEQ ID NO: 8069, SEQ ID NO: 3002 / SEQ ID NO: 8070, SEQ ID NO: 3003 /
SEQ ID NO: 8071, SEQ ID NO: 3005 / SEQ ID NO: 8072, SEQ ID NO: 3006 / SEQ ID NO:
8073, SEQ ID NO: 3007 / SEQ ID NO: 8074, SEQ ID NO: 3008 / SEQ ID NO: 8075, SEQ ID
NO: 3009 / SEQ ID NO: 8076, SEQ ID NO: 3010 / SEQ ID NO: 8077, SEQ ID NO: 3011 /
SEQ ID NO: 8078, SEQ ID NO: 3012 / SEQ ID NO: 8079, SEQ ID NO: 3014 / SEQ ID NO:
8080, SEQ ID NO: 3015 / SEQ ID NO: 8081, SEQ ID NO: 3016 / SEQ ID NO: 8082, SEQ ID
NO: 3017 / SEQ ID NO: 8083, SEQ ID NO: 3018 / SEQ ID NO: 8084, SEQ ID NO: 3019 /
SEQ ID NO: 8085, SEQ ID NO: 3020 / SEQ ID NO: 8086, SEQ ID NO: 3021 / SEQ ID NO:
8087, SEQ ID NO: 3022 / SEQ ID NO: 8088, SEQ ID NO: 3023 / SEQ ID NO: 8089, SEQ ID
NO: 3024 / SEQ ID NO: 8090, SEQ ID NO: 3025 / SEQ ID NO: 8091, SEQ ID NO: 3026 /
SEQ ID NO: 8092, SEQ ID NO: 3027 / SEQ ID NO: 8093, SEQ ID NO: 3028 / SEQ ID NO:
8094, SEQ ID NO: 3030 / SEQ ID NO: 8095, SEQ ID NO: 3031 / SEQ ID NO: 8096, SEQ ID
NO: 3032 / SEQ ID NO: 8097, SEQ ID NO: 3033 / SEQ ID NO: 8098, SEQ ID NO: 3034 /
SEQ ID NO: 8099, SEQ ID NO: 3035 / SEQ ID NO: 8100, SEQ ID NO: 3036 / SEQ ID NO:
8101, SEQ ID NO: 3037 / SEQ ID NO: 8102, SEQ ID NO: 3038 / SEQ ID NO: 8103, SEQ ID
NO: 3039 / SEQ ID NO: 8104, SEQ ID NO: 3040 / SEQ ID NO: 8105, SEQ ID NO: 3041 /
SEQ ID NO: 8106, SEQ ID NO: 3042 / SEQ ID NO: 8107, SEQ ID NO: 3043 / SEQ ID NO:
8108, SEQ ID NO: 3044 / SEQ ID NO: 8109, SEQ ID NO: 3045 / SEQ ID NO: 8110, SEQ ID

NO: 3046 / SEQ ID NO: 8111, SEQ ID NO: 3047 / SEQ ID NO: 8112, SEQ ID NO: 3048 /
SEQ ID NO: 8113, SEQ ID NO: 3049 / SEQ ID NO: 8114, SEQ ID NO: 3050 / SEQ ID NO:
8115, SEQ ID NO: 3051 / SEQ ID NO: 8116, SEQ ID NO: 3052 / SEQ ID NO: 8117, SEQ ID
NO: 3053 / SEQ ID NO: 8118, SEQ ID NO: 3054 / SEQ ID NO: 8119, SEQ ID NO: 3055 /
SEQ ID NO: 8120, SEQ ID NO: 3056 / SEQ ID NO: 8121, SEQ ID NO: 3057 / SEQ ID NO:
8122, SEQ ID NO: 3059 / SEQ ID NO: 8123, SEQ ID NO: 3060 / SEQ ID NO: 8124, SEQ ID
NO: 3061 / SEQ ID NO: 8125, SEQ ID NO: 3062 / SEQ ID NO: 8126, SEQ ID NO: 3063 /
SEQ ID NO: 8127, SEQ ID NO: 3064 / SEQ ID NO: 8128, SEQ ID NO: 3065 / SEQ ID NO:
8129, SEQ ID NO: 3066 / SEQ ID NO: 8130, SEQ ID NO: 3067 / SEQ ID NO: 8131, SEQ ID
NO: 3068 / SEQ ID NO: 8132, SEQ ID NO: 3069 / SEQ ID NO: 8133, SEQ ID NO: 3070 /
SEQ ID NO: 8134, SEQ ID NO: 3071 / SEQ ID NO: 8135, SEQ ID NO: 3072 / SEQ ID NO:
8136, SEQ ID NO: 3073 / SEQ ID NO: 8137, SEQ ID NO: 3074 / SEQ ID NO: 8138, SEQ ID
NO: 3075 / SEQ ID NO: 8139, SEQ ID NO: 3076 / SEQ ID NO: 8140, SEQ ID NO: 3077 /
SEQ ID NO: 8141, SEQ ID NO: 3078 / SEQ ID NO: 8142, SEQ ID NO: 3079 / SEQ ID NO:
8143, SEQ ID NO: 3080 / SEQ ID NO: 8144, SEQ ID NO: 3081 / SEQ ID NO: 8145, SEQ ID
NO: 3082 / SEQ ID NO: 8146, SEQ ID NO: 3083 / SEQ ID NO: 8147,
SEQ ID NO: 3084 / SEQ ID NO: 8148, SEQ ID NO: 3085 / SEQ ID NO: 8149, SEQ ID NO:
3086 / SEQ ID NO: 8150, SEQ ID NO: 3087 / SEQ ID NO: 8151, SEQ ID NO: 3088 / SEQ ID
NO: 8152, SEQ ID NO: 3089 / SEQ ID NO: 8153, SEQ ID NO: 3091 / SEQ ID NO: 8154, SEQ
ID NO: 3092 / SEQ ID NO: 8155, SEQ ID NO: 3093 / SEQ ID NO: 8156, SEQ ID NO: 3094 /
SEQ ID NO: 8157, SEQ ID NO: 3095 / SEQ ID NO: 8158, SEQ ID NO: 3096 / SEQ ID NO:
8159, SEQ ID NO: 3097 / SEQ ID NO: 8160, SEQ ID NO: 3098 / SEQ ID NO: 8161, SEQ ID
NO: 3099 / SEQ ID NO: 8162, SEQ ID NO: 3100 / SEQ ID NO: 8163, SEQ ID NO: 3101 /
SEQ ID NO: 8164, SEQ ID NO: 3102 / SEQ ID NO: 8165, SEQ ID NO: 3103 / SEQ ID NO:
8166, SEQ ID NO: 3104 / SEQ ID NO: 8167, SEQ ID NO: 3105 / SEQ ID NO: 8168, SEQ ID
NO: 3106 / SEQ ID NO: 8169, SEQ ID NO: 3108 / SEQ ID NO: 8170, SEQ ID NO: 3109 /
SEQ ID NO: 8171, SEQ ID NO: 3110 / SEQ ID NO: 8172, SEQ ID NO: 3111 / SEQ ID NO:
8173, SEQ ID NO: 3112 / SEQ ID NO: 8174, SEQ ID NO: 3113 / SEQ ID NO: 8175, SEQ ID
NO: 3114 / SEQ ID NO: 8176, SEQ ID NO: 3115 / SEQ ID NO: 8177, SEQ ID NO: 3116 /
SEQ ID NO: 8178, SEQ ID NO: 3117 / SEQ ID NO: 8179, SEQ ID NO: 3118 / SEQ ID NO:
8180, SEQ ID NO: 3119 / SEQ ID NO: 8181, SEQ ID NO: 3120 / SEQ ID NO: 8182, SEQ ID
NO: 3121 / SEQ ID NO: 8183, SEQ ID NO: 3122 / SEQ ID NO: 8184, SEQ ID NO: 3123 /
SEQ ID NO: 8185, SEQ ID NO: 3124 / SEQ ID NO: 8186, SEQ ID NO: 3125 / SEQ ID NO:
8187, SEQ ID NO: 3126 / SEQ ID NO: 8188, SEQ ID NO: 3128 / SEQ ID NO: 8189, SEQ ID
NO: 3129 / SEQ ID NO: 8190, SEQ ID NO: 3130 / SEQ ID NO: 8191, SEQ ID NO: 3131 /
SEQ ID NO: 8192, SEQ ID NO: 3132 / SEQ ID NO: 8193, SEQ ID NO: 3133 / SEQ ID NO:
8194, SEQ ID NO: 3134 / SEQ ID NO: 8195, SEQ ID NO: 3135 / SEQ ID NO: 8196, SEQ ID
NO: 3136 / SEQ ID NO: 8197, SEQ ID NO: 3137 / SEQ ID NO: 8198, SEQ ID NO: 3138 /
SEQ ID NO: 8199, SEQ ID NO: 3139 / SEQ ID NO: 8200, SEQ ID NO: 3140 / SEQ ID NO:
8201, SEQ ID NO: 3141 / SEQ ID NO: 8202, SEQ ID NO: 3142 / SEQ ID NO: 8203, SEQ ID
NO: 3144 / SEQ ID NO: 8204, SEQ ID NO: 3145 / SEQ ID NO: 8205, SEQ ID NO: 3146 /
SEQ ID NO: 8206, SEQ ID NO: 3147 / SEQ ID NO: 8207, SEQ ID NO: 3148 / SEQ ID NO:
8208, SEQ ID NO: 3149 / SEQ ID NO: 8209, SEQ ID NO: 3150 / SEQ ID NO: 8210, SEQ ID
NO: 3151 / SEQ ID NO: 8211, SEQ ID NO: 3152 / SEQ ID NO: 8212, SEQ ID NO: 3153 /
SEQ ID NO: 8213, SEQ ID NO: 3154 / SEQ ID NO: 8214, SEQ ID NO: 3156 / SEQ ID NO:
8215, SEQ ID NO: 3157 / SEQ ID NO: 8216, SEQ ID NO: 3158 / SEQ ID NO: 8217, SEQ ID
NO: 3159 / SEQ ID NO: 8218, SEQ ID NO: 3160 / SEQ ID NO: 8219, SEQ ID NO: 3161 /
SEQ ID NO: 8220, SEQ ID NO: 3162 / SEQ ID NO: 8221, SEQ ID NO: 3163 / SEQ ID NO:
8222, SEQ ID NO: 3164 / SEQ ID NO: 8223, SEQ ID NO: 3165 / SEQ ID NO: 8224, SEQ ID
NO: 3166 / SEQ ID NO: 8225, SEQ ID NO: 3167 / SEQ ID NO: 8226, SEQ ID NO: 3168 /
SEQ ID NO: 8227, SEQ ID NO: 3169 / SEQ ID NO: 8228, SEQ ID NO: 3170 / SEQ ID NO:
8229, SEQ ID NO: 3171 / SEQ ID NO: 8230, SEQ ID NO: 3172 / SEQ ID NO: 8231, SEQ ID

NO: 3173 / SEQ ID NO: 8232, SEQ ID NO: 3174 / SEQ ID NO: 8233, SEQ ID NO: 3175 /
SEQ ID NO: 8234, SEQ ID NO: 3176 / SEQ ID NO: 8235, SEQ ID NO: 3177 / SEQ ID NO:
8236, SEQ ID NO: 3178 / SEQ ID NO: 8237, SEQ ID NO: 3179 / SEQ ID NO: 8238, SEQ ID
NO: 3180 / SEQ ID NO: 8239, SEQ ID NO: 3181 / SEQ ID NO: 8240, SEQ ID NO: 3182 /
SEQ ID NO: 8241, SEQ ID NO: 3184 / SEQ ID NO: 8242, SEQ ID NO: 3185 / SEQ ID NO:
8243, SEQ ID NO: 3186 / SEQ ID NO: 8244, SEQ ID NO: 3188 / SEQ ID NO: 8245, SEQ ID
NO: 3189 / SEQ ID NO: 8246, SEQ ID NO: 3190 / SEQ ID NO: 8247,
SEQ ID NO: 3191 / SEQ ID NO: 8248, SEQ ID NO: 3192 / SEQ ID NO: 8249, SEQ ID NO:
3193 / SEQ ID NO: 8250, SEQ ID NO: 3194 / SEQ ID NO: 8251, SEQ ID NO: 3195 / SEQ ID
NO: 8252, SEQ ID NO: 3196 / SEQ ID NO: 8253, SEQ ID NO: 3197 / SEQ ID NO: 8254, SEQ
ID NO: 3198 / SEQ ID NO: 8255, SEQ ID NO: 3199 / SEQ ID NO: 8256, SEQ ID NO: 3200 /
SEQ ID NO: 8257, SEQ ID NO: 3201 / SEQ ID NO: 8258, SEQ ID NO: 3202 / SEQ ID NO:
8259, SEQ ID NO: 3203 / SEQ ID NO: 8260, SEQ ID NO: 3204 / SEQ ID NO: 8261, SEQ ID
NO: 3205 / SEQ ID NO: 8262, SEQ ID NO: 3206 / SEQ ID NO: 8263, SEQ ID NO: 3207 /
SEQ ID NO: 8264, SEQ ID NO: 3208 / SEQ ID NO: 8265, SEQ ID NO: 3209 / SEQ ID NO:
8266, SEQ ID NO: 3210 / SEQ ID NO: 8267, SEQ ID NO: 3211 / SEQ ID NO: 8268, SEQ ID
NO: 3212 / SEQ ID NO: 8269, SEQ ID NO: 3213 / SEQ ID NO: 8270, SEQ ID NO: 3215 /
SEQ ID NO: 8271, SEQ ID NO: 3216 / SEQ ID NO: 8272, SEQ ID NO: 3217 / SEQ ID NO:
8273, SEQ ID NO: 3218 / SEQ ID NO: 8274, SEQ ID NO: 3219 / SEQ ID NO: 8275, SEQ ID
NO: 3220 / SEQ ID NO: 8276, SEQ ID NO: 3221 / SEQ ID NO: 8277, SEQ ID NO: 3222 /
SEQ ID NO: 8278, SEQ ID NO: 3223 / SEQ ID NO: 8279, SEQ ID NO: 3224 / SEQ ID NO:
8280, SEQ ID NO: 3225 / SEQ ID NO: 8281, SEQ ID NO: 3226 / SEQ ID NO: 8282, SEQ ID
NO: 3227 / SEQ ID NO: 8283, SEQ ID NO: 3228 / SEQ ID NO: 8284, SEQ ID NO: 3229 /
SEQ ID NO: 8285, SEQ ID NO: 3230 / SEQ ID NO: 8286, SEQ ID NO: 3231 / SEQ ID NO:
8287, SEQ ID NO: 3232 / SEQ ID NO: 8288, SEQ ID NO: 3233 / SEQ ID NO: 8289, SEQ ID
NO: 3234 / SEQ ID NO: 8290, SEQ ID NO: 3235 / SEQ ID NO: 8291, SEQ ID NO: 3236 /
SEQ ID NO: 8292, SEQ ID NO: 3237 / SEQ ID NO: 8293, SEQ ID NO: 3238 / SEQ ID NO:
8294, SEQ ID NO: 3239 / SEQ ID NO: 8295, SEQ ID NO: 3241 / SEQ ID NO: 8296, SEQ ID
NO: 3242 / SEQ ID NO: 8297, SEQ ID NO: 3243 / SEQ ID NO: 8298, SEQ ID NO: 3244 /
SEQ ID NO: 8299, SEQ ID NO: 3245 / SEQ ID NO: 8300, SEQ ID NO: 3246 / SEQ ID NO:
8301, SEQ ID NO: 3247 / SEQ ID NO: 8302, SEQ ID NO: 3248 / SEQ ID NO: 8303, SEQ ID
NO: 3249 / SEQ ID NO: 8304, SEQ ID NO: 3250 / SEQ ID NO: 8305, SEQ ID NO: 3251 /
SEQ ID NO: 8306, SEQ ID NO: 3253 / SEQ ID NO: 8307, SEQ ID NO: 3254 / SEQ ID NO:
8308, SEQ ID NO: 3255 / SEQ ID NO: 8309, SEQ ID NO: 3256 / SEQ ID NO: 8310, SEQ ID
NO: 3257 / SEQ ID NO: 8311, SEQ ID NO: 3258 / SEQ ID NO: 8312, SEQ ID NO: 3259 /
SEQ ID NO: 8313, SEQ ID NO: 3260 / SEQ ID NO: 8314, SEQ ID NO: 3261 / SEQ ID NO:
8315, SEQ ID NO: 3262 / SEQ ID NO: 8316, SEQ ID NO: 3263 / SEQ ID NO: 8317, SEQ ID
NO: 3264 / SEQ ID NO: 8318, SEQ ID NO: 3265 / SEQ ID NO: 8319, SEQ ID NO: 3266 /
SEQ ID NO: 8320, SEQ ID NO: 3267 / SEQ ID NO: 8321, SEQ ID NO: 3268 / SEQ ID NO:
8322, SEQ ID NO: 3269 / SEQ ID NO: 8323, SEQ ID NO: 3270 / SEQ ID NO: 8324, SEQ ID
NO: 3271 / SEQ ID NO: 8325, SEQ ID NO: 3272 / SEQ ID NO: 8326, SEQ ID NO: 3273 /
SEQ ID NO: 8327, SEQ ID NO: 3274 / SEQ ID NO: 8328, SEQ ID NO: 3275 / SEQ ID NO:
8329, SEQ ID NO: 3276 / SEQ ID NO: 8330, SEQ ID NO: 3277 / SEQ ID NO: 8331, SEQ ID
NO: 3278 / SEQ ID NO: 8332, SEQ ID NO: 3279 / SEQ ID NO: 8333, SEQ ID NO: 3280 /
SEQ ID NO: 8334, SEQ ID NO: 3281 / SEQ ID NO: 8335, SEQ ID NO: 3282 / SEQ ID NO:
8336, SEQ ID NO: 3283 / SEQ ID NO: 8337, SEQ ID NO: 3284 / SEQ ID NO: 8338, SEQ ID
NO: 3285 / SEQ ID NO: 8339, SEQ ID NO: 3286 / SEQ ID NO: 8340, SEQ ID NO: 3287 /
SEQ ID NO: 8341, SEQ ID NO: 3288 / SEQ ID NO: 8342, SEQ ID NO: 3289 / SEQ ID NO:
8343, SEQ ID NO: 3290 / SEQ ID NO: 8344, SEQ ID NO: 3291 / SEQ ID NO: 8345, SEQ ID
NO: 3292 / SEQ ID NO: 8346, SEQ ID NO: 3293 / SEQ ID NO: 8347,
SEQ ID NO: 3294 / SEQ ID NO: 8348, SEQ ID NO: 3295 / SEQ ID NO: 8349, SEQ ID NO:
3296 / SEQ ID NO: 8350, SEQ ID NO: 3297 / SEQ ID NO: 8351, SEQ ID NO: 3298 / SEQ ID

NO: 8352, SEQ ID NO: 3299 / SEQ ID NO: 8353, SEQ ID NO: 3300 / SEQ ID NO: 8354, SEQ
ID NO: 3301 / SEQ ID NO: 8355, SEQ ID NO: 3302 / SEQ ID NO: 8356, SEQ ID NO: 3303 /
SEQ ID NO: 8357, SEQ ID NO: 3304 / SEQ ID NO: 8358, SEQ ID NO: 3305 / SEQ ID NO:
8359, SEQ ID NO: 3306 / SEQ ID NO: 8360, SEQ ID NO: 3307 / SEQ ID NO: 8361, SEQ ID
NO: 3308 / SEQ ID NO: 8362, SEQ ID NO: 3309 / SEQ ID NO: 8363, SEQ ID NO: 3310 /
SEQ ID NO: 8364, SEQ ID NO: 3311 / SEQ ID NO: 8365, SEQ ID NO: 3313 / SEQ ID NO:
8366, SEQ ID NO: 3314 / SEQ ID NO: 8367, SEQ ID NO: 3315 / SEQ ID NO: 8368, SEQ ID
NO: 3316 / SEQ ID NO: 8369, SEQ ID NO: 3317 / SEQ ID NO: 8370, SEQ ID NO: 3318 /
SEQ ID NO: 8371, SEQ ID NO: 3319 / SEQ ID NO: 8372, SEQ ID NO: 3321 / SEQ ID NO:
8373, SEQ ID NO: 3322 / SEQ ID NO: 8374, SEQ ID NO: 3323 / SEQ ID NO: 8375, SEQ ID
NO: 3324 / SEQ ID NO: 8376, SEQ ID NO: 3325 / SEQ ID NO: 8377, SEQ ID NO: 3326 /
SEQ ID NO: 8378, SEQ ID NO: 3327 / SEQ ID NO: 8379, SEQ ID NO: 3328 / SEQ ID NO:
8380, SEQ ID NO: 3329 / SEQ ID NO: 8381, SEQ ID NO: 3330 / SEQ ID NO: 8382, SEQ ID
NO: 3331 / SEQ ID NO: 8383, SEQ ID NO: 3332 / SEQ ID NO: 8384, SEQ ID NO: 3333 /
SEQ ID NO: 8385, SEQ ID NO: 3334 / SEQ ID NO: 8386, SEQ ID NO: 3335 / SEQ ID NO:
8387, SEQ ID NO: 3336 / SEQ ID NO: 8388, SEQ ID NO: 3337 / SEQ ID NO: 8389, SEQ ID
NO: 3338 / SEQ ID NO: 8390, SEQ ID NO: 3339 / SEQ ID NO: 8391, SEQ ID NO: 3340 /
SEQ ID NO: 8392, SEQ ID NO: 3341 / SEQ ID NO: 8393, SEQ ID NO: 3342 / SEQ ID NO:
8394, SEQ ID NO: 3343 / SEQ ID NO: 8395, SEQ ID NO: 3344 / SEQ ID NO: 8396, SEQ ID
NO: 3345 / SEQ ID NO: 8397, SEQ ID NO: 3346 / SEQ ID NO: 8398, SEQ ID NO: 3347 /
SEQ ID NO: 8399, SEQ ID NO: 3348 / SEQ ID NO: 8400, SEQ ID NO: 3349 / SEQ ID NO:
8401, SEQ ID NO: 3350 / SEQ ID NO: 8402, SEQ ID NO: 3351 / SEQ ID NO: 8403, SEQ ID
NO: 3352 / SEQ ID NO: 8404, SEQ ID NO: 3353 / SEQ ID NO: 8405, SEQ ID NO: 3354 /
SEQ ID NO: 8406, SEQ ID NO: 3355 / SEQ ID NO: 8407, SEQ ID NO: 3356 / SEQ ID NO:
8408, SEQ ID NO: 3357 / SEQ ID NO: 8409, SEQ ID NO: 3358 / SEQ ID NO: 8410, SEQ ID
NO: 3359 / SEQ ID NO: 8411, SEQ ID NO: 3360 / SEQ ID NO: 8412, SEQ ID NO: 3361 /
SEQ ID NO: 8413, SEQ ID NO: 3362 / SEQ ID NO: 8414, SEQ ID NO: 3363 / SEQ ID NO:
8415, SEQ ID NO: 3365 / SEQ ID NO: 8416, SEQ ID NO: 3366 / SEQ ID NO: 8417, SEQ ID
NO: 3367 / SEQ ID NO: 8418, SEQ ID NO: 3368 / SEQ ID NO: 8419, SEQ ID NO: 3370 /
SEQ ID NO: 8420, SEQ ID NO: 3371 / SEQ ID NO: 8421, SEQ ID NO: 3372 / SEQ ID NO:
8422, SEQ ID NO: 3373 / SEQ ID NO: 8423, SEQ ID NO: 3374 / SEQ ID NO: 8424, SEQ ID
NO: 3375 / SEQ ID NO: 8425, SEQ ID NO: 3376 / SEQ ID NO: 8426, SEQ ID NO: 3377 /
SEQ ID NO: 8427, SEQ ID NO: 3378 / SEQ ID NO: 8428, SEQ ID NO: 3379 / SEQ ID NO:
8429, SEQ ID NO: 3380 / SEQ ID NO: 8430, SEQ ID NO: 3381 / SEQ ID NO: 8431, SEQ ID
NO: 3382 / SEQ ID NO: 8432, SEQ ID NO: 3384 / SEQ ID NO: 8433, SEQ ID NO: 3385 /
SEQ ID NO: 8434, SEQ ID NO: 3386 / SEQ ID NO: 8435, SEQ ID NO: 3387 / SEQ ID NO:
8436, SEQ ID NO: 3388 / SEQ ID NO: 8437, SEQ ID NO: 3389 / SEQ ID NO: 8438, SEQ ID
NO: 3390 / SEQ ID NO: 8439, SEQ ID NO: 3391 / SEQ ID NO: 8440, SEQ ID NO: 3392 /
SEQ ID NO: 8441, SEQ ID NO: 3393 / SEQ ID NO: 8442, SEQ ID NO: 3394 / SEQ ID NO:
8443, SEQ ID NO: 3395 / SEQ ID NO: 8444, SEQ ID NO: 3396 / SEQ ID NO: 8445, SEQ ID
NO: 3397 / SEQ ID NO: 8446, SEQ ID NO: 3398 / SEQ ID NO: 8447,
SEQ ID NO: 3399 / SEQ ID NO: 8448, SEQ ID NO: 3400 / SEQ ID NO: 8449, SEQ ID NO:
3401 / SEQ ID NO: 8450, SEQ ID NO: 3402 / SEQ ID NO: 8451, SEQ ID NO: 3403 / SEQ ID
NO: 8452, SEQ ID NO: 3404 / SEQ ID NO: 8453, SEQ ID NO: 3405 / SEQ ID NO: 8454, SEQ
ID NO: 3406 / SEQ ID NO: 8455, SEQ ID NO: 3407 / SEQ ID NO: 8456, SEQ ID NO: 3408 /
SEQ ID NO: 8457, SEQ ID NO: 3409 / SEQ ID NO: 8458, SEQ ID NO: 3411 / SEQ ID NO:
8459, SEQ ID NO: 3412 / SEQ ID NO: 8460, SEQ ID NO: 3413 / SEQ ID NO: 8461, SEQ ID
NO: 3414 / SEQ ID NO: 8462, SEQ ID NO: 3415 / SEQ ID NO: 8463, SEQ ID NO: 3416 /
SEQ ID NO: 8464, SEQ ID NO: 3417 / SEQ ID NO: 8465, SEQ ID NO: 3418 / SEQ ID NO:
8466, SEQ ID NO: 3419 / SEQ ID NO: 8467, SEQ ID NO: 3420 / SEQ ID NO: 8468, SEQ ID
NO: 3421 / SEQ ID NO: 8469, SEQ ID NO: 3422 / SEQ ID NO: 8470, SEQ ID NO: 3423 /
SEQ ID NO: 8471, SEQ ID NO: 3424 / SEQ ID NO: 8472, SEQ ID NO: 3425 / SEQ ID NO:

8473, SEQ ID NO: 3426 / SEQ ID NO: 8474, SEQ ID NO: 3427 / SEQ ID NO: 8475, SEQ ID
NO: 3428 / SEQ ID NO: 8476, SEQ ID NO: 3429 / SEQ ID NO: 8477, SEQ ID NO: 3433 /
SEQ ID NO: 8478, SEQ ID NO: 3434 / SEQ ID NO: 8479, SEQ ID NO: 3435 / SEQ ID NO:
8480, SEQ ID NO: 3436 / SEQ ID NO: 8481, SEQ ID NO: 3437 / SEQ ID NO: 8482, SEQ ID
NO: 3438 / SEQ ID NO: 8483, SEQ ID NO: 3439 / SEQ ID NO: 8484, SEQ ID NO: 3440 /
SEQ ID NO: 8485, SEQ ID NO: 3441 / SEQ ID NO: 8486, SEQ ID NO: 3442 / SEQ ID NO:
8487, SEQ ID NO: 3443 / SEQ ID NO: 8488, SEQ ID NO: 3444 / SEQ ID NO: 8489, SEQ ID
NO: 3446 / SEQ ID NO: 8490, SEQ ID NO: 3448 / SEQ ID NO: 8491, SEQ ID NO: 3449 /
SEQ ID NO: 8492, SEQ ID NO: 3450 / SEQ ID NO: 8493, SEQ ID NO: 3451 / SEQ ID NO:
8494, SEQ ID NO: 3452 / SEQ ID NO: 8495, SEQ ID NO: 3453 / SEQ ID NO: 8496, SEQ ID
NO: 3454 / SEQ ID NO: 8497, SEQ ID NO: 3455 / SEQ ID NO: 8498, SEQ ID NO: 3456 /
SEQ ID NO: 8499, SEQ ID NO: 3457 / SEQ ID NO: 8500, SEQ ID NO: 3458 / SEQ ID NO:
8501, SEQ ID NO: 3459 / SEQ ID NO: 8502, SEQ ID NO: 3460 / SEQ ID NO: 8503, SEQ ID
NO: 3461 / SEQ ID NO: 8504, SEQ ID NO: 3462 / SEQ ID NO: 8505, SEQ ID NO: 3463 /
SEQ ID NO: 8506, SEQ ID NO: 3464 / SEQ ID NO: 8507, SEQ ID NO: 3465 / SEQ ID NO:
8508, SEQ ID NO: 3466 / SEQ ID NO: 8509, SEQ ID NO: 3467 / SEQ ID NO: 8510, SEQ ID
NO: 3468 / SEQ ID NO: 8511, SEQ ID NO: 3469 / SEQ ID NO: 8512, SEQ ID NO: 3470 /
SEQ ID NO: 8513, SEQ ID NO: 3471 / SEQ ID NO: 8514, SEQ ID NO: 3472 / SEQ ID NO:
8515, SEQ ID NO: 3473 / SEQ ID NO: 8516, SEQ ID NO: 3474 / SEQ ID NO: 8517, SEQ ID
NO: 3476 / SEQ ID NO: 8518, SEQ ID NO: 3477 / SEQ ID NO: 8519, SEQ ID NO: 3478 /
SEQ ID NO: 8520, SEQ ID NO: 3479 / SEQ ID NO: 8521, SEQ ID NO: 3480 / SEQ ID NO:
8522, SEQ ID NO: 3481 / SEQ ID NO: 8523, SEQ ID NO: 3482 / SEQ ID NO: 8524, SEQ ID
NO: 3483 / SEQ ID NO: 8525, SEQ ID NO: 3484 / SEQ ID NO: 8526, SEQ ID NO: 3485 /
SEQ ID NO: 8527, SEQ ID NO: 3486 / SEQ ID NO: 8528, SEQ ID NO: 3487 / SEQ ID NO:
8529, SEQ ID NO: 3488 / SEQ ID NO: 8530, SEQ ID NO: 3490 / SEQ ID NO: 8531, SEQ ID
NO: 3491 / SEQ ID NO: 8532, SEQ ID NO: 3492 / SEQ ID NO: 8533, SEQ ID NO: 3493 /
SEQ ID NO: 8534, SEQ ID NO: 3494 / SEQ ID NO: 8535, SEQ ID NO: 3495 / SEQ ID NO:
8536, SEQ ID NO: 3496 / SEQ ID NO: 8537, SEQ ID NO: 3498 / SEQ ID NO: 8538, SEQ ID
NO: 3499 / SEQ ID NO: 8539, SEQ ID NO: 3500 / SEQ ID NO: 8540, SEQ ID NO: 3501 /
SEQ ID NO: 8541, SEQ ID NO: 3502 / SEQ ID NO: 8542, SEQ ID NO: 3503 / SEQ ID NO:
8543, SEQ ID NO: 3504 / SEQ ID NO: 8544, SEQ ID NO: 3505 / SEQ ID NO: 8545, SEQ ID
NO: 3506 / SEQ ID NO: 8546, SEQ ID NO: 3507 / SEQ ID NO: 8547,
SEQ ID NO: 3508 / SEQ ID NO: 8548, SEQ ID NO: 3509 / SEQ ID NO: 8549, SEQ ID NO:
3510 / SEQ ID NO: 8550, SEQ ID NO: 3511 / SEQ ID NO: 8551, SEQ ID NO: 3512 / SEQ ID
NO: 8552, SEQ ID NO: 3513 / SEQ ID NO: 8553, SEQ ID NO: 3514 / SEQ ID NO: 8554, SEQ
ID NO: 3515 / SEQ ID NO: 8555, SEQ ID NO: 3516 / SEQ ID NO: 8556, SEQ ID NO: 3517 /
SEQ ID NO: 8557, SEQ ID NO: 3518 / SEQ ID NO: 8558, SEQ ID NO: 3519 / SEQ ID NO:
8559, SEQ ID NO: 3520 / SEQ ID NO: 8560, SEQ ID NO: 3521 / SEQ ID NO: 8561, SEQ ID
NO: 3522 / SEQ ID NO: 8562, SEQ ID NO: 3523 / SEQ ID NO: 8563, SEQ ID NO: 3524 /
SEQ ID NO: 8564, SEQ ID NO: 3525 / SEQ ID NO: 8565, SEQ ID NO: 3526 / SEQ ID NO:
8566, SEQ ID NO: 3527 / SEQ ID NO: 8567, SEQ ID NO: 3528 / SEQ ID NO: 8568, SEQ ID
NO: 3529 / SEQ ID NO: 8569, SEQ ID NO: 3530 / SEQ ID NO: 8570, SEQ ID NO: 3531 /
SEQ ID NO: 8571, SEQ ID NO: 3532 / SEQ ID NO: 8572, SEQ ID NO: 3533 / SEQ ID NO:
8573, SEQ ID NO: 3535 / SEQ ID NO: 8574, SEQ ID NO: 3536 / SEQ ID NO: 8575, SEQ ID
NO: 3537 / SEQ ID NO: 8576, SEQ ID NO: 3538 / SEQ ID NO: 8577, SEQ ID NO: 3539 /
SEQ ID NO: 8578, SEQ ID NO: 3541 / SEQ ID NO: 8579, SEQ ID NO: 3542 / SEQ ID NO:
8580, SEQ ID NO: 3543 / SEQ ID NO: 8581, SEQ ID NO: 3544 / SEQ ID NO: 8582, SEQ ID
NO: 3545 / SEQ ID NO: 8583, SEQ ID NO: 3546 / SEQ ID NO: 8584, SEQ ID NO: 3547 /
SEQ ID NO: 8585, SEQ ID NO: 3548 / SEQ ID NO: 8586, SEQ ID NO: 3549 / SEQ ID NO:
8587, SEQ ID NO: 3550 / SEQ ID NO: 8588, SEQ ID NO: 3551 / SEQ ID NO: 8589, SEQ ID
NO: 3552 / SEQ ID NO: 8590, SEQ ID NO: 3553 / SEQ ID NO: 8591, SEQ ID NO: 3554 /
SEQ ID NO: 8592, SEQ ID NO: 3555 / SEQ ID NO: 8593, SEQ ID NO: 3556 / SEQ ID NO:

8594, SEQ ID NO: 3557 / SEQ ID NO: 8595, SEQ ID NO: 3558 / SEQ ID NO: 8596, SEQ ID
NO: 3559 / SEQ ID NO: 8597, SEQ ID NO: 3560 / SEQ ID NO: 8598, SEQ ID NO: 3561 /
SEQ ID NO: 8599, SEQ ID NO: 3562 / SEQ ID NO: 8600, SEQ ID NO: 3563 / SEQ ID NO:
8601, SEQ ID NO: 3564 / SEQ ID NO: 8602, SEQ ID NO: 3565 / SEQ ID NO: 8603, SEQ ID
NO: 3566 / SEQ ID NO: 8604, SEQ ID NO: 3567 / SEQ ID NO: 8605, SEQ ID NO: 3568 /
SEQ ID NO: 8606, SEQ ID NO: 3569 / SEQ ID NO: 8607, SEQ ID NO: 3570 / SEQ ID NO:
8608, SEQ ID NO: 3571 / SEQ ID NO: 8609, SEQ ID NO: 3572 / SEQ ID NO: 8610, SEQ ID
NO: 3573 / SEQ ID NO: 8611, SEQ ID NO: 3574 / SEQ ID NO: 8612, SEQ ID NO: 3575 /
SEQ ID NO: 8613, SEQ ID NO: 3576 / SEQ ID NO: 8614, SEQ ID NO: 3577 / SEQ ID NO:
8615, SEQ ID NO: 3579 / SEQ ID NO: 8616, SEQ ID NO: 3580 / SEQ ID NO: 8617, SEQ ID
NO: 3581 / SEQ ID NO: 8618, SEQ ID NO: 3582 / SEQ ID NO: 8619, SEQ ID NO: 3583 /
SEQ ID NO: 8620, SEQ ID NO: 3584 / SEQ ID NO: 8621, SEQ ID NO: 3585 / SEQ ID NO:
8622, SEQ ID NO: 3586 / SEQ ID NO: 8623, SEQ ID NO: 3587 / SEQ ID NO: 8624, SEQ ID
NO: 3590 / SEQ ID NO: 8625, SEQ ID NO: 3591 / SEQ ID NO: 8626, SEQ ID NO: 3592 /
SEQ ID NO: 8627, SEQ ID NO: 3593 / SEQ ID NO: 8628, SEQ ID NO: 3594 / SEQ ID NO:
8629, SEQ ID NO: 3595 / SEQ ID NO: 8630, SEQ ID NO: 3596 / SEQ ID NO: 8631, SEQ ID
NO: 3597 / SEQ ID NO: 8632, SEQ ID NO: 3598 / SEQ ID NO: 8633, SEQ ID NO: 3599 /
SEQ ID NO: 8634, SEQ ID NO: 3600 / SEQ ID NO: 8635, SEQ ID NO: 3601 / SEQ ID NO:
8636, SEQ ID NO: 3602 / SEQ ID NO: 8637, SEQ ID NO: 3603 / SEQ ID NO: 8638, SEQ ID
NO: 3604 / SEQ ID NO: 8639, SEQ ID NO: 3605 / SEQ ID NO: 8640, SEQ ID NO: 3606 /
SEQ ID NO: 8641, SEQ ID NO: 3607 / SEQ ID NO: 8642, SEQ ID NO: 3608 / SEQ ID NO:
8643, SEQ ID NO: 3609 / SEQ ID NO: 8644, SEQ ID NO: 3610 / SEQ ID NO: 8645, SEQ ID
NO: 3611 / SEQ ID NO: 8646, SEQ ID NO: 3613 / SEQ ID NO: 8647,
SEQ ID NO: 3614 / SEQ ID NO: 8648, SEQ ID NO: 3615 / SEQ ID NO: 8649, SEQ ID NO:
3616 / SEQ ID NO: 8650, SEQ ID NO: 3617 / SEQ ID NO: 8651, SEQ ID NO: 3618 / SEQ ID
NO: 8652, SEQ ID NO: 3619 / SEQ ID NO: 8653, SEQ ID NO: 3620 / SEQ ID NO: 8654, SEQ
ID NO: 3622 / SEQ ID NO: 8655, SEQ ID NO: 3623 / SEQ ID NO: 8656, SEQ ID NO: 3624 /
SEQ ID NO: 8657, SEQ ID NO: 3625 / SEQ ID NO: 8658, SEQ ID NO: 3626 / SEQ ID NO:
8659, SEQ ID NO: 3627 / SEQ ID NO: 8660, SEQ ID NO: 3628 / SEQ ID NO: 8661, SEQ ID
NO: 3629 / SEQ ID NO: 8662, SEQ ID NO: 3630 / SEQ ID NO: 8663, SEQ ID NO: 3632 /
SEQ ID NO: 8664, SEQ ID NO: 3633 / SEQ ID NO: 8665, SEQ ID NO: 3634 / SEQ ID NO:
8666, SEQ ID NO: 3635 / SEQ ID NO: 8667, SEQ ID NO: 3636 / SEQ ID NO: 8668, SEQ ID
NO: 3637 / SEQ ID NO: 8669, SEQ ID NO: 3638 / SEQ ID NO: 8670, SEQ ID NO: 3639 /
SEQ ID NO: 8671, SEQ ID NO: 3640 / SEQ ID NO: 8672, SEQ ID NO: 3641 / SEQ ID NO:
8673, SEQ ID NO: 3642 / SEQ ID NO: 8674, SEQ ID NO: 3643 / SEQ ID NO: 8675, SEQ ID
NO: 3644 / SEQ ID NO: 8676, SEQ ID NO: 3645 / SEQ ID NO: 8677, SEQ ID NO: 3646 /
SEQ ID NO: 8678, SEQ ID NO: 3647 / SEQ ID NO: 8679, SEQ ID NO: 3648 / SEQ ID NO:
8680, SEQ ID NO: 3649 / SEQ ID NO: 8681, SEQ ID NO: 3650 / SEQ ID NO: 8682, SEQ ID
NO: 3651 / SEQ ID NO: 8683, SEQ ID NO: 3652 / SEQ ID NO: 8684, SEQ ID NO: 3653 /
SEQ ID NO: 8685, SEQ ID NO: 3654 / SEQ ID NO: 8686, SEQ ID NO: 3655 / SEQ ID NO:
8687, SEQ ID NO: 3656 / SEQ ID NO: 8688, SEQ ID NO: 3658 / SEQ ID NO: 8689, SEQ ID
NO: 3659 / SEQ ID NO: 8690, SEQ ID NO: 3660 / SEQ ID NO: 8691, SEQ ID NO: 3661 /
SEQ ID NO: 8692, SEQ ID NO: 3662 / SEQ ID NO: 8693, SEQ ID NO: 3663 / SEQ ID NO:
8694, SEQ ID NO: 3664 / SEQ ID NO: 8695, SEQ ID NO: 3665 / SEQ ID NO: 8696, SEQ ID
NO: 3666 / SEQ ID NO: 8697, SEQ ID NO: 3668 / SEQ ID NO: 8698, SEQ ID NO: 3669 /
SEQ ID NO: 8699, SEQ ID NO: 3670 / SEQ ID NO: 8700, SEQ ID NO: 3671 / SEQ ID NO:
8701, SEQ ID NO: 3672 / SEQ ID NO: 8702, SEQ ID NO: 3673 / SEQ ID NO: 8703, SEQ ID
NO: 3674 / SEQ ID NO: 8704, SEQ ID NO: 3675 / SEQ ID NO: 8705, SEQ ID NO: 3676 /
SEQ ID NO: 8706, SEQ ID NO: 3678 / SEQ ID NO: 8707, SEQ ID NO: 3679 / SEQ ID NO:
8708, SEQ ID NO: 3680 / SEQ ID NO: 8709, SEQ ID NO: 3681 / SEQ ID NO: 8710, SEQ ID
NO: 3682 / SEQ ID NO: 8711, SEQ ID NO: 3683 / SEQ ID NO: 8712, SEQ ID NO: 3685 /
SEQ ID NO: 8713, SEQ ID NO: 3686 / SEQ ID NO: 8714, SEQ ID NO: 3688 / SEQ ID NO:

8715, SEQ ID NO: 3689 / SEQ ID NO: 8716, SEQ ID NO: 3691 / SEQ ID NO: 8717, SEQ ID
NO: 3692 / SEQ ID NO: 8718, SEQ ID NO: 3693 / SEQ ID NO: 8719, SEQ ID NO: 3695 /
SEQ ID NO: 8720, SEQ ID NO: 3696 / SEQ ID NO: 8721, SEQ ID NO: 3697 / SEQ ID NO:
8722, SEQ ID NO: 3698 / SEQ ID NO: 8723, SEQ ID NO: 3699 / SEQ ID NO: 8724, SEQ ID
NO: 3700 / SEQ ID NO: 8725, SEQ ID NO: 3702 / SEQ ID NO: 8726, SEQ ID NO: 3703 /
SEQ ID NO: 8727, SEQ ID NO: 3704 / SEQ ID NO: 8728, SEQ ID NO: 3705 / SEQ ID NO:
8729, SEQ ID NO: 3706 / SEQ ID NO: 8730, SEQ ID NO: 3707 / SEQ ID NO: 8731, SEQ ID
NO: 3708 / SEQ ID NO: 8732, SEQ ID NO: 3709 / SEQ ID NO: 8733, SEQ ID NO: 3710 /
SEQ ID NO: 8734, SEQ ID NO: 3711 / SEQ ID NO: 8735, SEQ ID NO: 3712 / SEQ ID NO:
8736, SEQ ID NO: 3713 / SEQ ID NO: 8737, SEQ ID NO: 3714 / SEQ ID NO: 8738, SEQ ID
NO: 3715 / SEQ ID NO: 8739, SEQ ID NO: 3716 / SEQ ID NO: 8740, SEQ ID NO: 3717 /
SEQ ID NO: 8741, SEQ ID NO: 3718 / SEQ ID NO: 8742, SEQ ID NO: 3719 / SEQ ID NO:
8743, SEQ ID NO: 3720 / SEQ ID NO: 8744, SEQ ID NO: 3721 / SEQ ID NO: 8745, SEQ ID
NO: 3722 / SEQ ID NO: 8746, SEQ ID NO: 3723 / SEQ ID NO: 8747,
SEQ ID NO: 3724 / SEQ ID NO: 8748, SEQ ID NO: 3725 / SEQ ID NO: 8749, SEQ ID NO:
3726 / SEQ ID NO: 8750, SEQ ID NO: 3727 / SEQ ID NO: 8751, SEQ ID NO: 3728 / SEQ ID
NO: 8752, SEQ ID NO: 3729 / SEQ ID NO: 8753, SEQ ID NO: 3730 / SEQ ID NO: 8754, SEQ
ID NO: 3731 / SEQ ID NO: 8755, SEQ ID NO: 3732 / SEQ ID NO: 8756, SEQ ID NO: 3733 /
SEQ ID NO: 8757, SEQ ID NO: 3734 / SEQ ID NO: 8758, SEQ ID NO: 3735 / SEQ ID NO:
8759, SEQ ID NO: 3736 / SEQ ID NO: 8760, SEQ ID NO: 3737 / SEQ ID NO: 8761, SEQ ID
NO: 3738 / SEQ ID NO: 8762, SEQ ID NO: 3739 / SEQ ID NO: 8763, SEQ ID NO: 3740 /
SEQ ID NO: 8764, SEQ ID NO: 3741 / SEQ ID NO: 8765, SEQ ID NO: 3742 / SEQ ID NO:
8766, SEQ ID NO: 3743 / SEQ ID NO: 8767, SEQ ID NO: 3744 / SEQ ID NO: 8768, SEQ ID
NO: 3745 / SEQ ID NO: 8769, SEQ ID NO: 3746 / SEQ ID NO: 8770, SEQ ID NO: 3747 /
SEQ ID NO: 8771, SEQ ID NO: 3748 / SEQ ID NO: 8772, SEQ ID NO: 3749 / SEQ ID NO:
8773, SEQ ID NO: 3751 / SEQ ID NO: 8774, SEQ ID NO: 3753 / SEQ ID NO: 8775, SEQ ID
NO: 3754 / SEQ ID NO: 8776, SEQ ID NO: 3755 / SEQ ID NO: 8777, SEQ ID NO: 3756 /
SEQ ID NO: 8778, SEQ ID NO: 3757 / SEQ ID NO: 8779, SEQ ID NO: 3758 / SEQ ID NO:
8780, SEQ ID NO: 3759 / SEQ ID NO: 8781, SEQ ID NO: 3760 / SEQ ID NO: 8782, SEQ ID
NO: 3761 / SEQ ID NO: 8783, SEQ ID NO: 3762 / SEQ ID NO: 8784, SEQ ID NO: 3763 /
SEQ ID NO: 8785, SEQ ID NO: 3764 / SEQ ID NO: 8786, SEQ ID NO: 3765 / SEQ ID NO:
8787, SEQ ID NO: 3766 / SEQ ID NO: 8788, SEQ ID NO: 3767 / SEQ ID NO: 8789, SEQ ID
NO: 3768 / SEQ ID NO: 8790, SEQ ID NO: 3769 / SEQ ID NO: 8791, SEQ ID NO: 3770 /
SEQ ID NO: 8792, SEQ ID NO: 3771 / SEQ ID NO: 8793, SEQ ID NO: 3772 / SEQ ID NO:
8794, SEQ ID NO: 3773 / SEQ ID NO: 8795, SEQ ID NO: 3774 / SEQ ID NO: 8796, SEQ ID
NO: 3775 / SEQ ID NO: 8797, SEQ ID NO: 3776 / SEQ ID NO: 8798, SEQ ID NO: 3777 /
SEQ ID NO: 8799, SEQ ID NO: 3778 / SEQ ID NO: 8800, SEQ ID NO: 3779 / SEQ ID NO:
8801, SEQ ID NO: 3780 / SEQ ID NO: 8802, SEQ ID NO: 3781 / SEQ ID NO: 8803, SEQ ID
NO: 3782 / SEQ ID NO: 8804, SEQ ID NO: 3783 / SEQ ID NO: 8805, SEQ ID NO: 3784 /
SEQ ID NO: 8806, SEQ ID NO: 3785 / SEQ ID NO: 8807, SEQ ID NO: 3786 / SEQ ID NO:
8808, SEQ ID NO: 3787 / SEQ ID NO: 8809, SEQ ID NO: 3788 / SEQ ID NO: 8810, SEQ ID
NO: 3790 / SEQ ID NO: 8811, SEQ ID NO: 3791 / SEQ ID NO: 8812, SEQ ID NO: 3792 /
SEQ ID NO: 8813, SEQ ID NO: 3793 / SEQ ID NO: 8814, SEQ ID NO: 3794 / SEQ ID NO:
8815, SEQ ID NO: 3795 / SEQ ID NO: 8816, SEQ ID NO: 3796 / SEQ ID NO: 8817, SEQ ID
NO: 3797 / SEQ ID NO: 8818, SEQ ID NO: 3798 / SEQ ID NO: 8819, SEQ ID NO: 3799 /
SEQ ID NO: 8820, SEQ ID NO: 3800 / SEQ ID NO: 8821, SEQ ID NO: 3801 / SEQ ID NO:
8822, SEQ ID NO: 3803 / SEQ ID NO: 8823, SEQ ID NO: 3805 / SEQ ID NO: 8824, SEQ ID
NO: 3806 / SEQ ID NO: 8825, SEQ ID NO: 3807 / SEQ ID NO: 8826, SEQ ID NO: 3808 /
SEQ ID NO: 8827, SEQ ID NO: 3809 / SEQ ID NO: 8828, SEQ ID NO: 3810 / SEQ ID NO:
8829, SEQ ID NO: 3811 / SEQ ID NO: 8830, SEQ ID NO: 3812 / SEQ ID NO: 8831, SEQ ID
NO: 3813 / SEQ ID NO: 8832, SEQ ID NO: 3814 / SEQ ID NO: 8833, SEQ ID NO: 3815 /
SEQ ID NO: 8834, SEQ ID NO: 3816 / SEQ ID NO: 8835, SEQ ID NO: 3817 / SEQ ID NO:

8836, SEQ ID NO: 3818 / SEQ ID NO: 8837, SEQ ID NO: 3819 / SEQ ID NO: 8838, SEQ ID
NO: 3820 / SEQ ID NO: 8839, SEQ ID NO: 3821 / SEQ ID NO: 8840, SEQ ID NO: 3822 /
SEQ ID NO: 8841, SEQ ID NO: 3823 / SEQ ID NO: 8842, SEQ ID NO: 3824 / SEQ ID NO:
8843, SEQ ID NO: 3825 / SEQ ID NO: 8844, SEQ ID NO: 3826 / SEQ ID NO: 8845, SEQ ID
NO: 3827 / SEQ ID NO: 8846, SEQ ID NO: 3828 / SEQ ID NO: 8847,
SEQ ID NO: 3839 / SEQ ID NO: 8848, SEQ ID NO: 3840 / SEQ ID NO: 8849, SEQ ID NO:
3841 / SEQ ID NO: 8850, SEQ ID NO: 3842 / SEQ ID NO: 8851, SEQ ID NO: 3843 / SEQ ID
NO: 8852, SEQ ID NO: 3844 / SEQ ID NO: 8853, SEQ ID NO: 3845 / SEQ ID NO: 8854, SEQ
ID NO: 3846 / SEQ ID NO: 8855, SEQ ID NO: 3847 / SEQ ID NO: 8856, SEQ ID NO: 3848 /
SEQ ID NO: 8857, SEQ ID NO: 3849 / SEQ ID NO: 8858, SEQ ID NO: 3850 / SEQ ID NO:
8859, SEQ ID NO: 3851 / SEQ ID NO: 8860, SEQ ID NO: 3852 / SEQ ID NO: 8861, SEQ ID
NO: 3853 / SEQ ID NO: 8862, SEQ ID NO: 3854 / SEQ ID NO: 8863, SEQ ID NO: 3856 /
SEQ ID NO: 8864, SEQ ID NO: 3857 / SEQ ID NO: 8865, SEQ ID NO: 3858 / SEQ ID NO:
8866, SEQ ID NO: 3860 / SEQ ID NO: 8867, SEQ ID NO: 3861 / SEQ ID NO: 8868, SEQ ID
NO: 3862 / SEQ ID NO: 8869, SEQ ID NO: 3863 / SEQ ID NO: 8870, SEQ ID NO: 3864 /
SEQ ID NO: 8871, SEQ ID NO: 3865 / SEQ ID NO: 8872, SEQ ID NO: 3866 / SEQ ID NO:
8873, SEQ ID NO: 3867 / SEQ ID NO: 8874, SEQ ID NO: 3868 / SEQ ID NO: 8875, SEQ ID
NO: 3869 / SEQ ID NO: 8876, SEQ ID NO: 3870 / SEQ ID NO: 8877, SEQ ID NO: 3871 /
SEQ ID NO: 8878, SEQ ID NO: 3872 / SEQ ID NO: 8879, SEQ ID NO: 3873 / SEQ ID NO:
8880, SEQ ID NO: 3874 / SEQ ID NO: 8881, SEQ ID NO: 3875 / SEQ ID NO: 8882, SEQ ID
NO: 3876 / SEQ ID NO: 8883, SEQ ID NO: 3877 / SEQ ID NO: 8884, SEQ ID NO: 3878 /
SEQ ID NO: 8885, SEQ ID NO: 3879 / SEQ ID NO: 8886, SEQ ID NO: 3880 / SEQ ID NO:
8887, SEQ ID NO: 3881 / SEQ ID NO: 8888, SEQ ID NO: 3882 / SEQ ID NO: 8889, SEQ ID
NO: 3883 / SEQ ID NO: 8890, SEQ ID NO: 3884 / SEQ ID NO: 8891, SEQ ID NO: 3885 /
SEQ ID NO: 8892, SEQ ID NO: 3886 / SEQ ID NO: 8893, SEQ ID NO: 3887 / SEQ ID NO:
8894, SEQ ID NO: 3888 / SEQ ID NO: 8895, SEQ ID NO: 3889 / SEQ ID NO: 8896, SEQ ID
NO: 3890 / SEQ ID NO: 8897, SEQ ID NO: 3891 / SEQ ID NO: 8898, SEQ ID NO: 3892 /
SEQ ID NO: 8899, SEQ ID NO: 3893 / SEQ ID NO: 8900, SEQ ID NO: 3894 / SEQ ID NO:
8901, SEQ ID NO: 3895 / SEQ ID NO: 8902, SEQ ID NO: 3896 / SEQ ID NO: 8903, SEQ ID
NO: 3897 / SEQ ID NO: 8904, SEQ ID NO: 3898 / SEQ ID NO: 8905, SEQ ID NO: 3899 /
SEQ ID NO: 8906, SEQ ID NO: 3900 / SEQ ID NO: 8907, SEQ ID NO: 3901 / SEQ ID NO:
8908, SEQ ID NO: 3902 / SEQ ID NO: 8909, SEQ ID NO: 3903 / SEQ ID NO: 8910, SEQ ID
NO: 3905 / SEQ ID NO: 8911, SEQ ID NO: 3906 / SEQ ID NO: 8912, SEQ ID NO: 3907 /
SEQ ID NO: 8913, SEQ ID NO: 3908 / SEQ ID NO: 8914, SEQ ID NO: 3909 / SEQ ID NO:
8915, SEQ ID NO: 3911 / SEQ ID NO: 8916, SEQ ID NO: 3912 / SEQ ID NO: 8917, SEQ ID
NO: 3913 / SEQ ID NO: 8918, SEQ ID NO: 3914 / SEQ ID NO: 8919, SEQ ID NO: 3916 /
SEQ ID NO: 8920, SEQ ID NO: 3917 / SEQ ID NO: 8921, SEQ ID NO: 3918 / SEQ ID NO:
8922, SEQ ID NO: 3920 / SEQ ID NO: 8923, SEQ ID NO: 3921 / SEQ ID NO: 8924, SEQ ID
NO: 3922 / SEQ ID NO: 8925, SEQ ID NO: 3923 / SEQ ID NO: 8926, SEQ ID NO: 3924 /
SEQ ID NO: 8927, SEQ ID NO: 3925 / SEQ ID NO: 8928, SEQ ID NO: 3926 / SEQ ID NO:
8929, SEQ ID NO: 3927 / SEQ ID NO: 8930, SEQ ID NO: 3928 / SEQ ID NO: 8931, SEQ ID
NO: 3929 / SEQ ID NO: 8932, SEQ ID NO: 3930 / SEQ ID NO: 8933, SEQ ID NO: 3931 /
SEQ ID NO: 8934, SEQ ID NO: 3932 / SEQ ID NO: 8935, SEQ ID NO: 3933 / SEQ ID NO:
8936, SEQ ID NO: 3934 / SEQ ID NO: 8937, SEQ ID NO: 3935 / SEQ ID NO: 8938, SEQ ID
NO: 3937 / SEQ ID NO: 8939, SEQ ID NO: 3938 / SEQ ID NO: 8940, SEQ ID NO: 3939 /
SEQ ID NO: 8941, SEQ ID NO: 3940 / SEQ ID NO: 8942, SEQ ID NO: 3941 / SEQ ID NO:
8943, SEQ ID NO: 3942 / SEQ ID NO: 8944, SEQ ID NO: 3943 / SEQ ID NO: 8945, SEQ ID
NO: 3944 / SEQ ID NO: 8946, SEQ ID NO: 3945 / SEQ ID NO: 8947,
SEQ ID NO: 3946 / SEQ ID NO: 8948, SEQ ID NO: 3947 / SEQ ID NO: 8949, SEQ ID NO:
3949 / SEQ ID NO: 8950, SEQ ID NO: 3950 / SEQ ID NO: 8951, SEQ ID NO: 3951 / SEQ ID
NO: 8952, SEQ ID NO: 3952 / SEQ ID NO: 8953, SEQ ID NO: 3953 / SEQ ID NO: 8954, SEQ
ID NO: 3954 / SEQ ID NO: 8955, SEQ ID NO: 3955 / SEQ ID NO: 8956, SEQ ID NO: 3956 /

SEQ ID NO: 8957, SEQ ID NO: 3957 / SEQ ID NO: 8958, SEQ ID NO: 3958 / SEQ ID NO:
8959, SEQ ID NO: 3959 / SEQ ID NO: 8960, SEQ ID NO: 3960 / SEQ ID NO: 8961, SEQ ID
NO: 3961 / SEQ ID NO: 8962, SEQ ID NO: 3962 / SEQ ID NO: 8963, SEQ ID NO: 3963 /
SEQ ID NO: 8964, SEQ ID NO: 3964 / SEQ ID NO: 8965, SEQ ID NO: 3966 / SEQ ID NO:
8966, SEQ ID NO: 3967 / SEQ ID NO: 8967, SEQ ID NO: 3968 / SEQ ID NO: 8968, SEQ ID
NO: 3969 / SEQ ID NO: 8969, SEQ ID NO: 3970 / SEQ ID NO: 8970, SEQ ID NO: 3972 /
SEQ ID NO: 8971, SEQ ID NO: 3973 / SEQ ID NO: 8972, SEQ ID NO: 3974 / SEQ ID NO:
8973, SEQ ID NO: 3975 / SEQ ID NO: 8974, SEQ ID NO: 3977 / SEQ ID NO: 8975, SEQ ID
NO: 3978 / SEQ ID NO: 8976, SEQ ID NO: 3979 / SEQ ID NO: 8977, SEQ ID NO: 3980 /
SEQ ID NO: 8978, SEQ ID NO: 3981 / SEQ ID NO: 8979, SEQ ID NO: 3983 / SEQ ID NO:
8980, SEQ ID NO: 3985 / SEQ ID NO: 8981, SEQ ID NO: 3986 / SEQ ID NO: 8982, SEQ ID
NO: 3987 / SEQ ID NO: 8983, SEQ ID NO: 3988 / SEQ ID NO: 8984, SEQ ID NO: 3989 /
SEQ ID NO: 8985, SEQ ID NO: 3990 / SEQ ID NO: 8986, SEQ ID NO: 3992 / SEQ ID NO:
8987, SEQ ID NO: 3993 / SEQ ID NO: 8988, SEQ ID NO: 3994 / SEQ ID NO: 8989, SEQ ID
NO: 3995 / SEQ ID NO: 8990, SEQ ID NO: 3996 / SEQ ID NO: 8991, SEQ ID NO: 3997 /
SEQ ID NO: 8992, SEQ ID NO: 3998 / SEQ ID NO: 8993, SEQ ID NO: 3999 / SEQ ID NO:
8994, SEQ ID NO: 4000 / SEQ ID NO: 8995, SEQ ID NO: 4001 / SEQ ID NO: 8996, SEQ ID
NO: 4002 / SEQ ID NO: 8997, SEQ ID NO: 4003 / SEQ ID NO: 8998, SEQ ID NO: 4004 /
SEQ ID NO: 8999, SEQ ID NO: 4005 / SEQ ID NO: 9000, SEQ ID NO: 4006 / SEQ ID NO:
9001, SEQ ID NO: 4007 / SEQ ID NO: 9002, SEQ ID NO: 4008 / SEQ ID NO: 9003, SEQ ID
NO: 4009 / SEQ ID NO: 9004, SEQ ID NO: 4010 / SEQ ID NO: 9005, SEQ ID NO: 4011 /
SEQ ID NO: 9006, SEQ ID NO: 4012 / SEQ ID NO: 9007, SEQ ID NO: 4013 / SEQ ID NO:
9008, SEQ ID NO: 4014 / SEQ ID NO: 9009, SEQ ID NO: 4015 / SEQ ID NO: 9010, SEQ ID
NO: 4016 / SEQ ID NO: 9011, SEQ ID NO: 4017 / SEQ ID NO: 9012, SEQ ID NO: 4018 /
SEQ ID NO: 9013, SEQ ID NO: 4019 / SEQ ID NO: 9014, SEQ ID NO: 4020 / SEQ ID NO:
9015, SEQ ID NO: 4021 / SEQ ID NO: 9016, SEQ ID NO: 4022 / SEQ ID NO: 9017, SEQ ID
NO: 4023 / SEQ ID NO: 9018, SEQ ID NO: 4024 / SEQ ID NO: 9019, SEQ ID NO: 4025 /
SEQ ID NO: 9020, SEQ ID NO: 4026 / SEQ ID NO: 9021, SEQ ID NO: 4028 / SEQ ID NO:
9022, SEQ ID NO: 4029 / SEQ ID NO: 9023, SEQ ID NO: 4030 / SEQ ID NO: 9024, SEQ ID
NO: 4031 / SEQ ID NO: 9025, SEQ ID NO: 4032 / SEQ ID NO: 9026, SEQ ID NO: 4033 /
SEQ ID NO: 9027, SEQ ID NO: 4034 / SEQ ID NO: 9028, SEQ ID NO: 4035 / SEQ ID NO:
9029, SEQ ID NO: 4036 / SEQ ID NO: 9030, SEQ ID NO: 4038 / SEQ ID NO: 9031, SEQ ID
NO: 4039 / SEQ ID NO: 9032, SEQ ID NO: 4040 / SEQ ID NO: 9033, SEQ ID NO: 4041 /
SEQ ID NO: 9034, SEQ ID NO: 4042 / SEQ ID NO: 9035, SEQ ID NO: 4043 / SEQ ID NO:
9036, SEQ ID NO: 4044 / SEQ ID NO: 9037, SEQ ID NO: 4045 / SEQ ID NO: 9038, SEQ ID
NO: 4046 / SEQ ID NO: 9039, SEQ ID NO: 4047 / SEQ ID NO: 9040, SEQ ID NO: 4048 /
SEQ ID NO: 9041, SEQ ID NO: 4050 / SEQ ID NO: 9042, SEQ ID NO: 4051 / SEQ ID NO:
9043, SEQ ID NO: 4052 / SEQ ID NO: 9044, SEQ ID NO: 4053 / SEQ ID NO: 9045, SEQ ID
NO: 4054 / SEQ ID NO: 9046, SEQ ID NO: 4055 / SEQ ID NO: 9047,
SEQ ID NO: 4056 / SEQ ID NO: 9048, SEQ ID NO: 4057 / SEQ ID NO: 9049, SEQ ID NO:
4058 / SEQ ID NO: 9050, SEQ ID NO: 4059 / SEQ ID NO: 9051, SEQ ID NO: 4060 / SEQ ID
NO: 9052, SEQ ID NO: 4061 / SEQ ID NO: 9053, SEQ ID NO: 4062 / SEQ ID NO: 9054, SEQ
ID NO: 4063 / SEQ ID NO: 9055, SEQ ID NO: 4064 / SEQ ID NO: 9056, SEQ ID NO: 4065 /
SEQ ID NO: 9057, SEQ ID NO: 4066 / SEQ ID NO: 9058, SEQ ID NO: 4067 / SEQ ID NO:
9059, SEQ ID NO: 4068 / SEQ ID NO: 9060, SEQ ID NO: 4069 / SEQ ID NO: 9061, SEQ ID
NO: 4070 / SEQ ID NO: 9062, SEQ ID NO: 4071 / SEQ ID NO: 9063, SEQ ID NO: 4072 /
SEQ ID NO: 9064, SEQ ID NO: 4073 / SEQ ID NO: 9065, SEQ ID NO: 4074 / SEQ ID NO:
9066, SEQ ID NO: 4075 / SEQ ID NO: 9067, SEQ ID NO: 4077 / SEQ ID NO: 9068, SEQ ID
NO: 4078 / SEQ ID NO: 9069, SEQ ID NO: 4079 / SEQ ID NO: 9070, SEQ ID NO: 4080 /
SEQ ID NO: 9071, SEQ ID NO: 4081 / SEQ ID NO: 9072, SEQ ID NO: 4082 / SEQ ID NO:
9073, SEQ ID NO: 4083 / SEQ ID NO: 9074, SEQ ID NO: 4084 / SEQ ID NO: 9075, SEQ ID
NO: 4085 / SEQ ID NO: 9076, SEQ ID NO: 4086 / SEQ ID NO: 9077, SEQ ID NO: 4087 /

SEQ ID NO: 9078, SEQ ID NO: 4088 / SEQ ID NO: 9079, SEQ ID NO: 4089 / SEQ ID NO:
9080, SEQ ID NO: 4090 / SEQ ID NO: 9081, SEQ ID NO: 4091 / SEQ ID NO: 9082, SEQ ID
NO: 4092 / SEQ ID NO: 9083, SEQ ID NO: 4093 / SEQ ID NO: 9084, SEQ ID NO: 4094 /
SEQ ID NO: 9085, SEQ ID NO: 4095 / SEQ ID NO: 9086, SEQ ID NO: 4096 / SEQ ID NO:
9087, SEQ ID NO: 4097 / SEQ ID NO: 9088, SEQ ID NO: 4098 / SEQ ID NO: 9089, SEQ ID
NO: 4099 / SEQ ID NO: 9090, SEQ ID NO: 4100 / SEQ ID NO: 9091, SEQ ID NO: 4101 /
SEQ ID NO: 9092, SEQ ID NO: 4102 / SEQ ID NO: 9093, SEQ ID NO: 4103 / SEQ ID NO:
9094, SEQ ID NO: 4104 / SEQ ID NO: 9095, SEQ ID NO: 4105 / SEQ ID NO: 9096, SEQ ID
NO: 4107 / SEQ ID NO: 9097, SEQ ID NO: 4108 / SEQ ID NO: 9098, SEQ ID NO: 4109 /
SEQ ID NO: 9099, SEQ ID NO: 4110 / SEQ ID NO: 9100, SEQ ID NO: 4111 / SEQ ID NO:
9101, SEQ ID NO: 4112 / SEQ ID NO: 9102, SEQ ID NO: 4113 / SEQ ID NO: 9103, SEQ ID
NO: 4114 / SEQ ID NO: 9104, SEQ ID NO: 4115 / SEQ ID NO: 9105, SEQ ID NO: 4116 /
SEQ ID NO: 9106, SEQ ID NO: 4117 / SEQ ID NO: 9107, SEQ ID NO: 4118 / SEQ ID NO:
9108, SEQ ID NO: 4119 / SEQ ID NO: 9109, SEQ ID NO: 4120 / SEQ ID NO: 9110, SEQ ID
NO: 4121 / SEQ ID NO: 9111, SEQ ID NO: 4122 / SEQ ID NO: 9112, SEQ ID NO: 4123 /
SEQ ID NO: 9113, SEQ ID NO: 4124 / SEQ ID NO: 9114, SEQ ID NO: 4125 / SEQ ID NO:
9115, SEQ ID NO: 4126 / SEQ ID NO: 9116, SEQ ID NO: 4127 / SEQ ID NO: 9117, SEQ ID
NO: 4128 / SEQ ID NO: 9118, SEQ ID NO: 4129 / SEQ ID NO: 9119, SEQ ID NO: 4130 /
SEQ ID NO: 9120, SEQ ID NO: 4132 / SEQ ID NO: 9121, SEQ ID NO: 4133 / SEQ ID NO:
9122, SEQ ID NO: 4134 / SEQ ID NO: 9123, SEQ ID NO: 4135 / SEQ ID NO: 9124, SEQ ID
NO: 4136 / SEQ ID NO: 9125, SEQ ID NO: 4137 / SEQ ID NO: 9126, SEQ ID NO: 4138 /
SEQ ID NO: 9127, SEQ ID NO: 4139 / SEQ ID NO: 9128, SEQ ID NO: 4140 / SEQ ID NO:
9129, SEQ ID NO: 4141 / SEQ ID NO: 9130, SEQ ID NO: 4142 / SEQ ID NO: 9131, SEQ ID
NO: 4143 / SEQ ID NO: 9132, SEQ ID NO: 4144 / SEQ ID NO: 9133, SEQ ID NO: 4145 /
SEQ ID NO: 9134, SEQ ID NO: 4146 / SEQ ID NO: 9135, SEQ ID NO: 4147 / SEQ ID NO:
9136, SEQ ID NO: 4148 / SEQ ID NO: 9137, SEQ ID NO: 4149 / SEQ ID NO: 9138, SEQ ID
NO: 4150 / SEQ ID NO: 9139, SEQ ID NO: 4151 / SEQ ID NO: 9140, SEQ ID NO: 4152 /
SEQ ID NO: 9141, SEQ ID NO: 4153 / SEQ ID NO: 9142, SEQ ID NO: 4154 / SEQ ID NO:
9143, SEQ ID NO: 4155 / SEQ ID NO: 9144, SEQ ID NO: 4157 / SEQ ID NO: 9145, SEQ ID
NO: 4158 / SEQ ID NO: 9146, SEQ ID NO: 4159 / SEQ ID NO: 9147,
SEQ ID NO: 4160 / SEQ ID NO: 9148, SEQ ID NO: 4161 / SEQ ID NO: 9149, SEQ ID NO:
4162 / SEQ ID NO: 9150, SEQ ID NO: 4164 / SEQ ID NO: 9151, SEQ ID NO: 4165 / SEQ ID
NO: 9152, SEQ ID NO: 4166 / SEQ ID NO: 9153, SEQ ID NO: 4167 / SEQ ID NO: 9154, SEQ
ID NO: 4168 / SEQ ID NO: 9155, SEQ ID NO: 4169 / SEQ ID NO: 9156, SEQ ID NO: 4170 /
SEQ ID NO: 9157, SEQ ID NO: 4171 / SEQ ID NO: 9158, SEQ ID NO: 4172 / SEQ ID NO:
9159, SEQ ID NO: 4173 / SEQ ID NO: 9160, SEQ ID NO: 4174 / SEQ ID NO: 9161, SEQ ID
NO: 4175 / SEQ ID NO: 9162, SEQ ID NO: 4176 / SEQ ID NO: 9163, SEQ ID NO: 4177 /
SEQ ID NO: 9164, SEQ ID NO: 4178 / SEQ ID NO: 9165, SEQ ID NO: 4179 / SEQ ID NO:
9166, SEQ ID NO: 4180 / SEQ ID NO: 9167, SEQ ID NO: 4181 / SEQ ID NO: 9168, SEQ ID
NO: 4182 / SEQ ID NO: 9169, SEQ ID NO: 4183 / SEQ ID NO: 9170, SEQ ID NO: 4184 /
SEQ ID NO: 9171, SEQ ID NO: 4185 / SEQ ID NO: 9172, SEQ ID NO: 4186 / SEQ ID NO:
9173, SEQ ID NO: 4187 / SEQ ID NO: 9174, SEQ ID NO: 4188 / SEQ ID NO: 9175, SEQ ID
NO: 4189 / SEQ ID NO: 9176, SEQ ID NO: 4190 / SEQ ID NO: 9177, SEQ ID NO: 4191 /
SEQ ID NO: 9178, SEQ ID NO: 4192 / SEQ ID NO: 9179, SEQ ID NO: 4193 / SEQ ID NO:
9180, SEQ ID NO: 4194 / SEQ ID NO: 9181, SEQ ID NO: 4195 / SEQ ID NO: 9182, SEQ ID
NO: 4196 / SEQ ID NO: 9183, SEQ ID NO: 4197 / SEQ ID NO: 9184, SEQ ID NO: 4198 /
SEQ ID NO: 9185, SEQ ID NO: 4199 / SEQ ID NO: 9186, SEQ ID NO: 4200 / SEQ ID NO:
9187, SEQ ID NO: 4201 / SEQ ID NO: 9188, SEQ ID NO: 4202 / SEQ ID NO: 9189, SEQ ID
NO: 4203 / SEQ ID NO: 9190, SEQ ID NO: 4204 / SEQ ID NO: 9191, SEQ ID NO: 4205 /
SEQ ID NO: 9192, SEQ ID NO: 4206 / SEQ ID NO: 9193, SEQ ID NO: 4208 / SEQ ID NO:
9194, SEQ ID NO: 4209 / SEQ ID NO: 9195, SEQ ID NO: 4210 / SEQ ID NO: 9196, SEQ ID
NO: 4211 / SEQ ID NO: 9197, SEQ ID NO: 4212 / SEQ ID NO: 9198, SEQ ID NO: 4213 /

SEQ ID NO: 9199, SEQ ID NO: 4214 / SEQ ID NO: 9200, SEQ ID NO: 4215 / SEQ ID NO:
9201, SEQ ID NO: 4216 / SEQ ID NO: 9202, SEQ ID NO: 4217 / SEQ ID NO: 9203, SEQ ID
NO: 4218 / SEQ ID NO: 9204, SEQ ID NO: 4219 / SEQ ID NO: 9205, SEQ ID NO: 4220 /
SEQ ID NO: 9206, SEQ ID NO: 4221 / SEQ ID NO: 9207, SEQ ID NO: 4222 / SEQ ID NO:
9208, SEQ ID NO: 4223 / SEQ ID NO: 9209, SEQ ID NO: 4224 / SEQ ID NO: 9210, SEQ ID
NO: 4225 / SEQ ID NO: 9211, SEQ ID NO: 4226 / SEQ ID NO: 9212, SEQ ID NO: 4227 /
SEQ ID NO: 9213, SEQ ID NO: 4228 / SEQ ID NO: 9214, SEQ ID NO: 4229 / SEQ ID NO:
9215, SEQ ID NO: 4231 / SEQ ID NO: 9216, SEQ ID NO: 4232 / SEQ ID NO: 9217, SEQ ID
NO: 4234 / SEQ ID NO: 9218, SEQ ID NO: 4235 / SEQ ID NO: 9219, SEQ ID NO: 4236 /
SEQ ID NO: 9220, SEQ ID NO: 4237 / SEQ ID NO: 9221, SEQ ID NO: 4238 / SEQ ID NO:
9222, SEQ ID NO: 4239 / SEQ ID NO: 9223, SEQ ID NO: 4240 / SEQ ID NO: 9224, SEQ ID
NO: 4241 / SEQ ID NO: 9225, SEQ ID NO: 4243 / SEQ ID NO: 9226, SEQ ID NO: 4244 /
SEQ ID NO: 9227, SEQ ID NO: 4245 / SEQ ID NO: 9228, SEQ ID NO: 4246 / SEQ ID NO:
9229, SEQ ID NO: 4247 / SEQ ID NO: 9230, SEQ ID NO: 4248 / SEQ ID NO: 9231, SEQ ID
NO: 4249 / SEQ ID NO: 9232, SEQ ID NO: 4250 / SEQ ID NO: 9233, SEQ ID NO: 4251 /
SEQ ID NO: 9234, SEQ ID NO: 4252 / SEQ ID NO: 9235, SEQ ID NO: 4253 / SEQ ID NO:
9236, SEQ ID NO: 4254 / SEQ ID NO: 9237, SEQ ID NO: 4256 / SEQ ID NO: 9238, SEQ ID
NO: 4257 / SEQ ID NO: 9239, SEQ ID NO: 4258 / SEQ ID NO: 9240, SEQ ID NO: 4259 /
SEQ ID NO: 9241, SEQ ID NO: 4260 / SEQ ID NO: 9242, SEQ ID NO: 4261 / SEQ ID NO:
9243, SEQ ID NO: 4262 / SEQ ID NO: 9244, SEQ ID NO: 4263 / SEQ ID NO: 9245, SEQ ID
NO: 4264 / SEQ ID NO: 9246, SEQ ID NO: 4265 / SEQ ID NO: 9247,
SEQ ID NO: 4266 / SEQ ID NO: 9248, SEQ ID NO: 4267 / SEQ ID NO: 9249, SEQ ID NO:
4268 / SEQ ID NO: 9250, SEQ ID NO: 4269 / SEQ ID NO: 9251, SEQ ID NO: 4270 / SEQ ID
NO: 9252, SEQ ID NO: 4271 / SEQ ID NO: 9253, SEQ ID NO: 4272 / SEQ ID NO: 9254, SEQ
ID NO: 4273 / SEQ ID NO: 9255, SEQ ID NO: 4274 / SEQ ID NO: 9256, SEQ ID NO: 4275 /
SEQ ID NO: 9257, SEQ ID NO: 4276 / SEQ ID NO: 9258, SEQ ID NO: 4277 / SEQ ID NO:
9259, SEQ ID NO: 4278 / SEQ ID NO: 9260, SEQ ID NO: 4279 / SEQ ID NO: 9261, SEQ ID
NO: 4280 / SEQ ID NO: 9262, SEQ ID NO: 4281 / SEQ ID NO: 9263, SEQ ID NO: 4282 /
SEQ ID NO: 9264, SEQ ID NO: 4283 / SEQ ID NO: 9265, SEQ ID NO: 4284 / SEQ ID NO:
9266, SEQ ID NO: 4285 / SEQ ID NO: 9267, SEQ ID NO: 4286 / SEQ ID NO: 9268, SEQ ID
NO: 4288 / SEQ ID NO: 9269, SEQ ID NO: 4289 / SEQ ID NO: 9270, SEQ ID NO: 4290 /
SEQ ID NO: 9271, SEQ ID NO: 4291 / SEQ ID NO: 9272, SEQ ID NO: 4292 / SEQ ID NO:
9273, SEQ ID NO: 4293 / SEQ ID NO: 9274, SEQ ID NO: 4295 / SEQ ID NO: 9275, SEQ ID
NO: 4296 / SEQ ID NO: 9276, SEQ ID NO: 4297 / SEQ ID NO: 9277, SEQ ID NO: 4298 /
SEQ ID NO: 9278, SEQ ID NO: 4299 / SEQ ID NO: 9279, SEQ ID NO: 4300 / SEQ ID NO:
9280, SEQ ID NO: 4301 / SEQ ID NO: 9281, SEQ ID NO: 4302 / SEQ ID NO: 9282, SEQ ID
NO: 4303 / SEQ ID NO: 9283, SEQ ID NO: 4304 / SEQ ID NO: 9284, SEQ ID NO: 4305 /
SEQ ID NO: 9285, SEQ ID NO: 4306 / SEQ ID NO: 9286, SEQ ID NO: 4307 / SEQ ID NO:
9287, SEQ ID NO: 4308 / SEQ ID NO: 9288, SEQ ID NO: 4309 / SEQ ID NO: 9289, SEQ ID
NO: 4310 / SEQ ID NO: 9290, SEQ ID NO: 4311 / SEQ ID NO: 9291, SEQ ID NO: 4312 /
SEQ ID NO: 9292, SEQ ID NO: 4313 / SEQ ID NO: 9293, SEQ ID NO: 4314 / SEQ ID NO:
9294, SEQ ID NO: 4316 / SEQ ID NO: 9295, SEQ ID NO: 4317 / SEQ ID NO: 9296, SEQ ID
NO: 4318 / SEQ ID NO: 9297, SEQ ID NO: 4319 / SEQ ID NO: 9298, SEQ ID NO: 4320 /
SEQ ID NO: 9299, SEQ ID NO: 4321 / SEQ ID NO: 9300, SEQ ID NO: 4322 / SEQ ID NO:
9301, SEQ ID NO: 4323 / SEQ ID NO: 9302, SEQ ID NO: 4324 / SEQ ID NO: 9303, SEQ ID
NO: 4325 / SEQ ID NO: 9304, SEQ ID NO: 4326 / SEQ ID NO: 9305, SEQ ID NO: 4327 /
SEQ ID NO: 9306, SEQ ID NO: 4328 / SEQ ID NO: 9307, SEQ ID NO: 4329 / SEQ ID NO:
9308, SEQ ID NO: 4330 / SEQ ID NO: 9309, SEQ ID NO: 4331 / SEQ ID NO: 9310, SEQ ID
NO: 4332 / SEQ ID NO: 9311, SEQ ID NO: 4333 / SEQ ID NO: 9312, SEQ ID NO: 4334 /
SEQ ID NO: 9313, SEQ ID NO: 4335 / SEQ ID NO: 9314, SEQ ID NO: 4336 / SEQ ID NO:
9315, SEQ ID NO: 4337 / SEQ ID NO: 9316, SEQ ID NO: 4338 / SEQ ID NO: 9317, SEQ ID
NO: 4339 / SEQ ID NO: 9318, SEQ ID NO: 4340 / SEQ ID NO: 9319, SEQ ID NO: 4341 /

SEQ ID NO: 9320, SEQ ID NO: 4342 / SEQ ID NO: 9321, SEQ ID NO: 4344 / SEQ ID NO:
9322, SEQ ID NO: 4345 / SEQ ID NO: 9323, SEQ ID NO: 4346 / SEQ ID NO: 9324, SEQ ID
NO: 4347 / SEQ ID NO: 9325, SEQ ID NO: 4348 / SEQ ID NO: 9326, SEQ ID NO: 4349 /
SEQ ID NO: 9327, SEQ ID NO: 4350 / SEQ ID NO: 9328, SEQ ID NO: 4351 / SEQ ID NO:
9329, SEQ ID NO: 4352 / SEQ ID NO: 9330, SEQ ID NO: 4353 / SEQ ID NO: 9331, SEQ ID
NO: 4354 / SEQ ID NO: 9332, SEQ ID NO: 4355 / SEQ ID NO: 9333, SEQ ID NO: 4356 /
SEQ ID NO: 9334, SEQ ID NO: 4357 / SEQ ID NO: 9335, SEQ ID NO: 4358 / SEQ ID NO:
9336, SEQ ID NO: 4359 / SEQ ID NO: 9337, SEQ ID NO: 4360 / SEQ ID NO: 9338, SEQ ID
NO: 4361 / SEQ ID NO: 9339, SEQ ID NO: 4362 / SEQ ID NO: 9340, SEQ ID NO: 4363 /
SEQ ID NO: 9341, SEQ ID NO: 4364 / SEQ ID NO: 9342, SEQ ID NO: 4365 / SEQ ID NO:
9343, SEQ ID NO: 4366 / SEQ ID NO: 9344, SEQ ID NO: 4367 / SEQ ID NO: 9345, SEQ ID
NO: 4368 / SEQ ID NO: 9346, SEQ ID NO: 4369 / SEQ ID NO: 9347,
SEQ ID NO: 4370 / SEQ ID NO: 9348, SEQ ID NO: 4371 / SEQ ID NO: 9349, SEQ ID NO:
4372 / SEQ ID NO: 9350, SEQ ID NO: 4373 / SEQ ID NO: 9351, SEQ ID NO: 4374 / SEQ ID
NO: 9352, SEQ ID NO: 4375 / SEQ ID NO: 9353, SEQ ID NO: 4376 / SEQ ID NO: 9354, SEQ
ID NO: 4377 / SEQ ID NO: 9355, SEQ ID NO: 4378 / SEQ ID NO: 9356, SEQ ID NO: 4379 /
SEQ ID NO: 9357, SEQ ID NO: 4380 / SEQ ID NO: 9358, SEQ ID NO: 4381 / SEQ ID NO:
9359, SEQ ID NO: 4382 / SEQ ID NO: 9360, SEQ ID NO: 4383 / SEQ ID NO: 9361, SEQ ID
NO: 4384 / SEQ ID NO: 9362, SEQ ID NO: 4385 / SEQ ID NO: 9363, SEQ ID NO: 4386 /
SEQ ID NO: 9364, SEQ ID NO: 4387 / SEQ ID NO: 9365, SEQ ID NO: 4388 / SEQ ID NO:
9366, SEQ ID NO: 4390 / SEQ ID NO: 9367, SEQ ID NO: 4391 / SEQ ID NO: 9368, SEQ ID
NO: 4392 / SEQ ID NO: 9369, SEQ ID NO: 4393 / SEQ ID NO: 9370, SEQ ID NO: 4394 /
SEQ ID NO: 9371, SEQ ID NO: 4395 / SEQ ID NO: 9372, SEQ ID NO: 4396 / SEQ ID NO:
9373, SEQ ID NO: 4398 / SEQ ID NO: 9374, SEQ ID NO: 4399 / SEQ ID NO: 9375, SEQ ID
NO: 4400 / SEQ ID NO: 9376, SEQ ID NO: 4401 / SEQ ID NO: 9377, SEQ ID NO: 4402 /
SEQ ID NO: 9378, SEQ ID NO: 4403 / SEQ ID NO: 9379, SEQ ID NO: 4404 / SEQ ID NO:
9380, SEQ ID NO: 4405 / SEQ ID NO: 9381, SEQ ID NO: 4406 / SEQ ID NO: 9382, SEQ ID
NO: 4407 / SEQ ID NO: 9383, SEQ ID NO: 4408 / SEQ ID NO: 9384, SEQ ID NO: 4409 /
SEQ ID NO: 9385, SEQ ID NO: 4410 / SEQ ID NO: 9386, SEQ ID NO: 4411 / SEQ ID NO:
9387, SEQ ID NO: 4412 / SEQ ID NO: 9388, SEQ ID NO: 4413 / SEQ ID NO: 9389, SEQ ID
NO: 4414 / SEQ ID NO: 9390, SEQ ID NO: 4415 / SEQ ID NO: 9391, SEQ ID NO: 4416 /
SEQ ID NO: 9392, SEQ ID NO: 4417 / SEQ ID NO: 9393, SEQ ID NO: 4418 / SEQ ID NO:
9394, SEQ ID NO: 4419 / SEQ ID NO: 9395, SEQ ID NO: 4420 / SEQ ID NO: 9396, SEQ ID
NO: 4421 / SEQ ID NO: 9397, SEQ ID NO: 4422 / SEQ ID NO: 9398, SEQ ID NO: 4423 /
SEQ ID NO: 9399, SEQ ID NO: 4424 / SEQ ID NO: 9400, SEQ ID NO: 4425 / SEQ ID NO:
9401, SEQ ID NO: 4426 / SEQ ID NO: 9402, SEQ ID NO: 4427 / SEQ ID NO: 9403, SEQ ID
NO: 4428 / SEQ ID NO: 9404, SEQ ID NO: 4429 / SEQ ID NO: 9405, SEQ ID NO: 4430 /
SEQ ID NO: 9406, SEQ ID NO: 4431 / SEQ ID NO: 9407, SEQ ID NO: 4432 / SEQ ID NO:
9408, SEQ ID NO: 4433 / SEQ ID NO: 9409, SEQ ID NO: 4434 / SEQ ID NO: 9410, SEQ ID
NO: 4435 / SEQ ID NO: 9411, SEQ ID NO: 4436 / SEQ ID NO: 9412, SEQ ID NO: 4437 /
SEQ ID NO: 9413, SEQ ID NO: 4438 / SEQ ID NO: 9414, SEQ ID NO: 4439 / SEQ ID NO:
9415, SEQ ID NO: 4440 / SEQ ID NO: 9416, SEQ ID NO: 4441 / SEQ ID NO: 9417, SEQ ID
NO: 4442 / SEQ ID NO: 9418, SEQ ID NO: 4443 / SEQ ID NO: 9419, SEQ ID NO: 4444 /
SEQ ID NO: 9420, SEQ ID NO: 4445 / SEQ ID NO: 9421, SEQ ID NO: 4446 / SEQ ID NO:
9422, SEQ ID NO: 4447 / SEQ ID NO: 9423, SEQ ID NO: 4448 / SEQ ID NO: 9424, SEQ ID
NO: 4449 / SEQ ID NO: 9425, SEQ ID NO: 4450 / SEQ ID NO: 9426, SEQ ID NO: 4451 /
SEQ ID NO: 9427, SEQ ID NO: 4452 / SEQ ID NO: 9428, SEQ ID NO: 4453 / SEQ ID NO:
9429, SEQ ID NO: 4454 / SEQ ID NO: 9430, SEQ ID NO: 4455 / SEQ ID NO: 9431, SEQ ID
NO: 4456 / SEQ ID NO: 9432, SEQ ID NO: 4457 / SEQ ID NO: 9433, SEQ ID NO: 4458 /
SEQ ID NO: 9434, SEQ ID NO: 4459 / SEQ ID NO: 9435, SEQ ID NO: 4460 / SEQ ID NO:
9436, SEQ ID NO: 4461 / SEQ ID NO: 9437, SEQ ID NO: 4462 / SEQ ID NO: 9438, SEQ ID
NO: 4463 / SEQ ID NO: 9439, SEQ ID NO: 4464 / SEQ ID NO: 9440, SEQ ID NO: 4465 /

SEQ ID NO: 9441, SEQ ID NO: 4466 / SEQ ID NO: 9442, SEQ ID NO: 4467 / SEQ ID NO:
9443, SEQ ID NO: 4468 / SEQ ID NO: 9444, SEQ ID NO: 4469 / SEQ ID NO: 9445, SEQ ID
NO: 4470 / SEQ ID NO: 9446, SEQ ID NO: 4471 / SEQ ID NO: 9447,
SEQ ID NO: 4472 / SEQ ID NO: 9448, SEQ ID NO: 4473 / SEQ ID NO: 9449, SEQ ID NO:
4474 / SEQ ID NO: 9450, SEQ ID NO: 4475 / SEQ ID NO: 9451, SEQ ID NO: 4476 / SEQ ID
NO: 9452, SEQ ID NO: 4477 / SEQ ID NO: 9453, SEQ ID NO: 4478 / SEQ ID NO: 9454, SEQ
ID NO: 4479 / SEQ ID NO: 9455, SEQ ID NO: 4480 / SEQ ID NO: 9456, SEQ ID NO: 4481 /
SEQ ID NO: 9457, SEQ ID NO: 4482 / SEQ ID NO: 9458, SEQ ID NO: 4483 / SEQ ID NO:
9459, SEQ ID NO: 4484 / SEQ ID NO: 9460, SEQ ID NO: 4485 / SEQ ID NO: 9461, SEQ ID
NO: 4486 / SEQ ID NO: 9462, SEQ ID NO: 4487 / SEQ ID NO: 9463, SEQ ID NO: 4489 /
SEQ ID NO: 9464, SEQ ID NO: 4490 / SEQ ID NO: 9465, SEQ ID NO: 4491 / SEQ ID NO:
9466, SEQ ID NO: 4492 / SEQ ID NO: 9467, SEQ ID NO: 4493 / SEQ ID NO: 9468, SEQ ID
NO: 4494 / SEQ ID NO: 9469, SEQ ID NO: 4495 / SEQ ID NO: 9470, SEQ ID NO: 4496 /
SEQ ID NO: 9471, SEQ ID NO: 4497 / SEQ ID NO: 9472, SEQ ID NO: 4498 / SEQ ID NO:
9473, SEQ ID NO: 4499 / SEQ ID NO: 9474, SEQ ID NO: 4500 / SEQ ID NO: 9475, SEQ ID
NO: 4501 / SEQ ID NO: 9476, SEQ ID NO: 4502 / SEQ ID NO: 9477, SEQ ID NO: 4503 /
SEQ ID NO: 9478, SEQ ID NO: 4504 / SEQ ID NO: 9479, SEQ ID NO: 4505 / SEQ ID NO:
9480, SEQ ID NO: 4506 / SEQ ID NO: 9481, SEQ ID NO: 4507 / SEQ ID NO: 9482, SEQ ID
NO: 4508 / SEQ ID NO: 9483, SEQ ID NO: 4509 / SEQ ID NO: 9484, SEQ ID NO: 4510 /
SEQ ID NO: 9485, SEQ ID NO: 4511 / SEQ ID NO: 9486, SEQ ID NO: 4512 / SEQ ID NO:
9487, SEQ ID NO: 4513 / SEQ ID NO: 9488, SEQ ID NO: 4514 / SEQ ID NO: 9489, SEQ ID
NO: 4515 / SEQ ID NO: 9490, SEQ ID NO: 4516 / SEQ ID NO: 9491, SEQ ID NO: 4517 /
SEQ ID NO: 9492, SEQ ID NO: 4518 / SEQ ID NO: 9493, SEQ ID NO: 4519 / SEQ ID NO:
9494, SEQ ID NO: 4520 / SEQ ID NO: 9495, SEQ ID NO: 4521 / SEQ ID NO: 9496, SEQ ID
NO: 4522 / SEQ ID NO: 9497, SEQ ID NO: 4523 / SEQ ID NO: 9498, SEQ ID NO: 4524 /
SEQ ID NO: 9499, SEQ ID NO: 4525 / SEQ ID NO: 9500, SEQ ID NO: 4526 / SEQ ID NO:
9501, SEQ ID NO: 4527 / SEQ ID NO: 9502, SEQ ID NO: 4528 / SEQ ID NO: 9503, SEQ ID
NO: 4529 / SEQ ID NO: 9504, SEQ ID NO: 4530 / SEQ ID NO: 9505, SEQ ID NO: 4531 /
SEQ ID NO: 9506, SEQ ID NO: 4532 / SEQ ID NO: 9507, SEQ ID NO: 4533 / SEQ ID NO:
9508, SEQ ID NO: 4534 / SEQ ID NO: 9509, SEQ ID NO: 4535 / SEQ ID NO: 9510, SEQ ID
NO: 4536 / SEQ ID NO: 9511, SEQ ID NO: 4537 / SEQ ID NO: 9512, SEQ ID NO: 4538 /
SEQ ID NO: 9513, SEQ ID NO: 4539 / SEQ ID NO: 9514, SEQ ID NO: 4540 / SEQ ID NO:
9515, SEQ ID NO: 4541 / SEQ ID NO: 9516, SEQ ID NO: 4542 / SEQ ID NO: 9517, SEQ ID
NO: 4543 / SEQ ID NO: 9518, SEQ ID NO: 4544 / SEQ ID NO: 9519, SEQ ID NO: 4545 /
SEQ ID NO: 9520, SEQ ID NO: 4546 / SEQ ID NO: 9521, SEQ ID NO: 4547 / SEQ ID NO:
9522, SEQ ID NO: 4548 / SEQ ID NO: 9523, SEQ ID NO: 4549 / SEQ ID NO: 9524, SEQ ID
NO: 4550 / SEQ ID NO: 9525, SEQ ID NO: 4551 / SEQ ID NO: 9526, SEQ ID NO: 4552 /
SEQ ID NO: 9527, SEQ ID NO: 4553 / SEQ ID NO: 9528, SEQ ID NO: 4555 / SEQ ID NO:
9529, SEQ ID NO: 4556 / SEQ ID NO: 9530, SEQ ID NO: 4557 / SEQ ID NO: 9531, SEQ ID
NO: 4558 / SEQ ID NO: 9532, SEQ ID NO: 4559 / SEQ ID NO: 9533, SEQ ID NO: 4560 /
SEQ ID NO: 9534, SEQ ID NO: 4561 / SEQ ID NO: 9535, SEQ ID NO: 4563 / SEQ ID NO:
9536, SEQ ID NO: 4564 / SEQ ID NO: 9537, SEQ ID NO: 4565 / SEQ ID NO: 9538, SEQ ID
NO: 4566 / SEQ ID NO: 9539, SEQ ID NO: 4567 / SEQ ID NO: 9540, SEQ ID NO: 4568 /
SEQ ID NO: 9541, SEQ ID NO: 4569 / SEQ ID NO: 9542, SEQ ID NO: 4570 / SEQ ID NO:
9543, SEQ ID NO: 4571 / SEQ ID NO: 9544, SEQ ID NO: 4572 / SEQ ID NO: 9545, SEQ ID
NO: 4573 / SEQ ID NO: 9546, SEQ ID NO: 4574 / SEQ ID NO: 9547,
SEQ ID NO: 4575 / SEQ ID NO: 9548, SEQ ID NO: 4576 / SEQ ID NO: 9549, SEQ ID NO:
4577 / SEQ ID NO: 9550, SEQ ID NO: 4578 / SEQ ID NO: 9551, SEQ ID NO: 4579 / SEQ ID
NO: 9552, SEQ ID NO: 4580 / SEQ ID NO: 9553, SEQ ID NO: 4581 / SEQ ID NO: 9554, SEQ
ID NO: 4582 / SEQ ID NO: 9555, SEQ ID NO: 4583 / SEQ ID NO: 9556, SEQ ID NO: 4584 /
SEQ ID NO: 9557, SEQ ID NO: 4585 / SEQ ID NO: 9558, SEQ ID NO: 4586 / SEQ ID NO:
9559, SEQ ID NO: 4587 / SEQ ID NO: 9560, SEQ ID NO: 4588 / SEQ ID NO: 9561, SEQ ID

NO: 4589 / SEQ ID NO: 9562, SEQ ID NO: 4590 / SEQ ID NO: 9563, SEQ ID NO: 4591 /
SEQ ID NO: 9564, SEQ ID NO: 4592 / SEQ ID NO: 9565, SEQ ID NO: 4593 / SEQ ID NO:
9566, SEQ ID NO: 4594 / SEQ ID NO: 9567, SEQ ID NO: 4595 / SEQ ID NO: 9568, SEQ ID
NO: 4596 / SEQ ID NO: 9569, SEQ ID NO: 4597 / SEQ ID NO: 9570, SEQ ID NO: 4598 /
SEQ ID NO: 9571, SEQ ID NO: 4599 / SEQ ID NO: 9572, SEQ ID NO: 4600 / SEQ ID NO:
9573, SEQ ID NO: 4601 / SEQ ID NO: 9574, SEQ ID NO: 4602 / SEQ ID NO: 9575, SEQ ID
NO: 4603 / SEQ ID NO: 9576, SEQ ID NO: 4604 / SEQ ID NO: 9577, SEQ ID NO: 4605 /
SEQ ID NO: 9578, SEQ ID NO: 4606 / SEQ ID NO: 9579, SEQ ID NO: 4607 / SEQ ID NO:
9580, SEQ ID NO: 4608 / SEQ ID NO: 9581, SEQ ID NO: 4609 / SEQ ID NO: 9582, SEQ ID
NO: 4610 / SEQ ID NO: 9583, SEQ ID NO: 4611 / SEQ ID NO: 9584, SEQ ID NO: 4612 /
SEQ ID NO: 9585, SEQ ID NO: 4613 / SEQ ID NO: 9586, SEQ ID NO: 4614 / SEQ ID NO:
9587, SEQ ID NO: 4615 / SEQ ID NO: 9588, SEQ ID NO: 4616 / SEQ ID NO: 9589, SEQ ID
NO: 4617 / SEQ ID NO: 9590, SEQ ID NO: 4618 / SEQ ID NO: 9591, SEQ ID NO: 4619 /
SEQ ID NO: 9592, SEQ ID NO: 4621 / SEQ ID NO: 9593, SEQ ID NO: 4622 / SEQ ID NO:
9594, SEQ ID NO: 4623 / SEQ ID NO: 9595, SEQ ID NO: 4624 / SEQ ID NO: 9596, SEQ ID
NO: 4625 / SEQ ID NO: 9597, SEQ ID NO: 4626 / SEQ ID NO: 9598, SEQ ID NO: 4627 /
SEQ ID NO: 9599, SEQ ID NO: 4628 / SEQ ID NO: 9600, SEQ ID NO: 4629 / SEQ ID NO:
9601, SEQ ID NO: 4630 / SEQ ID NO: 9602, SEQ ID NO: 4631 / SEQ ID NO: 9603, SEQ ID
NO: 4632 / SEQ ID NO: 9604, SEQ ID NO: 4633 / SEQ ID NO: 9605, SEQ ID NO: 4634 /
SEQ ID NO: 9606, SEQ ID NO: 4635 / SEQ ID NO: 9607, SEQ ID NO: 4636 / SEQ ID NO:
9608, SEQ ID NO: 4637 / SEQ ID NO: 9609, SEQ ID NO: 4638 / SEQ ID NO: 9610, SEQ ID
NO: 4639 / SEQ ID NO: 9611, SEQ ID NO: 4640 / SEQ ID NO: 9612, SEQ ID NO: 4641 /
SEQ ID NO: 9613, SEQ ID NO: 4642 / SEQ ID NO: 9614, SEQ ID NO: 4643 / SEQ ID NO:
9615, SEQ ID NO: 4644 / SEQ ID NO: 9616, SEQ ID NO: 4645 / SEQ ID NO: 9617, SEQ ID
NO: 4646 / SEQ ID NO: 9618, SEQ ID NO: 4647 / SEQ ID NO: 9619, SEQ ID NO: 4648 /
SEQ ID NO: 9620, SEQ ID NO: 4649 / SEQ ID NO: 9621, SEQ ID NO: 4650 / SEQ ID NO:
9622, SEQ ID NO: 4651 / SEQ ID NO: 9623, SEQ ID NO: 4652 / SEQ ID NO: 9624, SEQ ID
NO: 4653 / SEQ ID NO: 9625, SEQ ID NO: 4654 / SEQ ID NO: 9626, SEQ ID NO: 4655 /
SEQ ID NO: 9627, SEQ ID NO: 4656 / SEQ ID NO: 9628, SEQ ID NO: 4657 / SEQ ID NO:
9629, SEQ ID NO: 4659 / SEQ ID NO: 9630, SEQ ID NO: 4660 / SEQ ID NO: 9631, SEQ ID
NO: 4661 / SEQ ID NO: 9632, SEQ ID NO: 4662 / SEQ ID NO: 9633, SEQ ID NO: 4663 /
SEQ ID NO: 9634, SEQ ID NO: 4664 / SEQ ID NO: 9635, SEQ ID NO: 4665 / SEQ ID NO:
9636, SEQ ID NO: 4666 / SEQ ID NO: 9637, SEQ ID NO: 4667 / SEQ ID NO: 9638, SEQ ID
NO: 4669 / SEQ ID NO: 9639, SEQ ID NO: 4670 / SEQ ID NO: 9640, SEQ ID NO: 4671 /
SEQ ID NO: 9641, SEQ ID NO: 4672 / SEQ ID NO: 9642, SEQ ID NO: 4673 / SEQ ID NO:
9643, SEQ ID NO: 4674 / SEQ ID NO: 9644, SEQ ID NO: 4675 / SEQ ID NO: 9645, SEQ ID
NO: 4676 / SEQ ID NO: 9646, SEQ ID NO: 4677 / SEQ ID NO: 9647,
SEQ ID NO: 4678 / SEQ ID NO: 9648, SEQ ID NO: 4679 / SEQ ID NO: 9649, SEQ ID NO:
4680 / SEQ ID NO: 9650, SEQ ID NO: 4681 / SEQ ID NO: 9651, SEQ ID NO: 4682 / SEQ ID
NO: 9652, SEQ ID NO: 4683 / SEQ ID NO: 9653, SEQ ID NO: 4684 / SEQ ID NO: 9654, SEQ
ID NO: 4685 / SEQ ID NO: 9655, SEQ ID NO: 4686 / SEQ ID NO: 9656, SEQ ID NO: 4687 /
SEQ ID NO: 9657, SEQ ID NO: 4688 / SEQ ID NO: 9658, SEQ ID NO: 4689 / SEQ ID NO:
9659, SEQ ID NO: 4690 / SEQ ID NO: 9660, SEQ ID NO: 4691 / SEQ ID NO: 9661, SEQ ID
NO: 4692 / SEQ ID NO: 9662, SEQ ID NO: 4693 / SEQ ID NO: 9663, SEQ ID NO: 4694 /
SEQ ID NO: 9664, SEQ ID NO: 4695 / SEQ ID NO: 9665, SEQ ID NO: 4696 / SEQ ID NO:
9666, SEQ ID NO: 4697 / SEQ ID NO: 9667, SEQ ID NO: 4699 / SEQ ID NO: 9668, SEQ ID
NO: 4700 / SEQ ID NO: 9669, SEQ ID NO: 4701 / SEQ ID NO: 9670, SEQ ID NO: 4702 /
SEQ ID NO: 9671, SEQ ID NO: 4703 / SEQ ID NO: 9672, SEQ ID NO: 4704 / SEQ ID NO:
9673, SEQ ID NO: 4705 / SEQ ID NO: 9674, SEQ ID NO: 4706 / SEQ ID NO: 9675, SEQ ID
NO: 4708 / SEQ ID NO: 9676, SEQ ID NO: 4709 / SEQ ID NO: 9677, SEQ ID NO: 4710 /
SEQ ID NO: 9678, SEQ ID NO: 4711 / SEQ ID NO: 9679, SEQ ID NO: 4712 / SEQ ID NO:
9680, SEQ ID NO: 4713 / SEQ ID NO: 9681, SEQ ID NO: 4714 / SEQ ID NO: 9682, SEQ ID

NO: 4715 / SEQ ID NO: 9683, SEQ ID NO: 4716 / SEQ ID NO: 9684, SEQ ID NO: 4717 /
SEQ ID NO: 9685, SEQ ID NO: 4718 / SEQ ID NO: 9686, SEQ ID NO: 4719 / SEQ ID NO:
9687, SEQ ID NO: 4720 / SEQ ID NO: 9688, SEQ ID NO: 4721 / SEQ ID NO: 9689, SEQ ID
NO: 4722 / SEQ ID NO: 9690, SEQ ID NO: 4723 / SEQ ID NO: 9691, SEQ ID NO: 4724 /
SEQ ID NO: 9692, SEQ ID NO: 4725 / SEQ ID NO: 9693, SEQ ID NO: 4726 / SEQ ID NO:
9694, SEQ ID NO: 4727 / SEQ ID NO: 9695, SEQ ID NO: 4728 / SEQ ID NO: 9696, SEQ ID
NO: 4729 / SEQ ID NO: 9697, SEQ ID NO: 4730 / SEQ ID NO: 9698, SEQ ID NO: 4731 /
SEQ ID NO: 9699, SEQ ID NO: 4732 / SEQ ID NO: 9700, SEQ ID NO: 4733 / SEQ ID NO:
9701, SEQ ID NO: 4734 / SEQ ID NO: 9702, SEQ ID NO: 4735 / SEQ ID NO: 9703, SEQ ID
NO: 4736 / SEQ ID NO: 9704, SEQ ID NO: 4737 / SEQ ID NO: 9705, SEQ ID NO: 4738 /
SEQ ID NO: 9706, SEQ ID NO: 4739 / SEQ ID NO: 9707, SEQ ID NO: 4740 / SEQ ID NO:
9708, SEQ ID NO: 4741 / SEQ ID NO: 9709, SEQ ID NO: 4742 / SEQ ID NO: 9710, SEQ ID
NO: 4743 / SEQ ID NO: 9711, SEQ ID NO: 4744 / SEQ ID NO: 9712, SEQ ID NO: 4745 /
SEQ ID NO: 9713, SEQ ID NO: 4746 / SEQ ID NO: 9714, SEQ ID NO: 4747 / SEQ ID NO:
9715, SEQ ID NO: 4748 / SEQ ID NO: 9716, SEQ ID NO: 4749 / SEQ ID NO: 9717, SEQ ID
NO: 4750 / SEQ ID NO: 9718, SEQ ID NO: 4751 / SEQ ID NO: 9719, SEQ ID NO: 4752 /
SEQ ID NO: 9720, SEQ ID NO: 4753 / SEQ ID NO: 9721, SEQ ID NO: 4754 / SEQ ID NO:
9722, SEQ ID NO: 4755 / SEQ ID NO: 9723, SEQ ID NO: 4756 / SEQ ID NO: 9724, SEQ ID
NO: 4757 / SEQ ID NO: 9725, SEQ ID NO: 4758 / SEQ ID NO: 9726, SEQ ID NO: 4759 /
SEQ ID NO: 9727, SEQ ID NO: 4760 / SEQ ID NO: 9728, SEQ ID NO: 4761 / SEQ ID NO:
9729, SEQ ID NO: 4762 / SEQ ID NO: 9730, SEQ ID NO: 4763 / SEQ ID NO: 9731, SEQ ID
NO: 4764 / SEQ ID NO: 9732, SEQ ID NO: 4765 / SEQ ID NO: 9733, SEQ ID NO: 4766 /
SEQ ID NO: 9734, SEQ ID NO: 4767 / SEQ ID NO: 9735, SEQ ID NO: 4768 / SEQ ID NO:
9736, SEQ ID NO: 4769 / SEQ ID NO: 9737, SEQ ID NO: 4770 / SEQ ID NO: 9738, SEQ ID
NO: 4771 / SEQ ID NO: 9739, SEQ ID NO: 4772 / SEQ ID NO: 9740, SEQ ID NO: 4773 /
SEQ ID NO: 9741, SEQ ID NO: 4774 / SEQ ID NO: 9742, SEQ ID NO: 4775 / SEQ ID NO:
9743, SEQ ID NO: 4776 / SEQ ID NO: 9744, SEQ ID NO: 4777 / SEQ ID NO: 9745, SEQ ID
NO: 4778 / SEQ ID NO: 9746, SEQ ID NO: 4779 / SEQ ID NO: 9747,
SEQ ID NO: 4780 / SEQ ID NO: 9748, SEQ ID NO: 4781 / SEQ ID NO: 9749, SEQ ID NO:
4782 / SEQ ID NO: 9750, SEQ ID NO: 4783 / SEQ ID NO: 9751, SEQ ID NO: 4784 / SEQ ID
NO: 9752, SEQ ID NO: 4785 / SEQ ID NO: 9753, SEQ ID NO: 4786 / SEQ ID NO: 9754, SEQ
ID NO: 4787 / SEQ ID NO: 9755, SEQ ID NO: 4788 / SEQ ID NO: 9756, SEQ ID NO: 4789 /
SEQ ID NO: 9757, SEQ ID NO: 4790 / SEQ ID NO: 9758, SEQ ID NO: 4791 / SEQ ID NO:
9759, SEQ ID NO: 4792 / SEQ ID NO: 9760, SEQ ID NO: 4793 / SEQ ID NO: 9761, SEQ ID
NO: 4795 / SEQ ID NO: 9762, SEQ ID NO: 4796 / SEQ ID NO: 9763, SEQ ID NO: 4797 /
SEQ ID NO: 9764, SEQ ID NO: 4798 / SEQ ID NO: 9765, SEQ ID NO: 4799 / SEQ ID NO:
9766, SEQ ID NO: 4800 / SEQ ID NO: 9767, SEQ ID NO: 4801 / SEQ ID NO: 9768, SEQ ID
NO: 4802 / SEQ ID NO: 9769, SEQ ID NO: 4803 / SEQ ID NO: 9770, SEQ ID NO: 4804 /
SEQ ID NO: 9771, SEQ ID NO: 4805 / SEQ ID NO: 9772, SEQ ID NO: 4806 / SEQ ID NO:
9773, SEQ ID NO: 4807 / SEQ ID NO: 9774, SEQ ID NO: 4808 / SEQ ID NO: 9775, SEQ ID
NO: 4809 / SEQ ID NO: 9776, SEQ ID NO: 4810 / SEQ ID NO: 9777, SEQ ID NO: 4811 /
SEQ ID NO: 9778, SEQ ID NO: 4812 / SEQ ID NO: 9779, SEQ ID NO: 4813 / SEQ ID NO:
9780, SEQ ID NO: 4814 / SEQ ID NO: 9781, SEQ ID NO: 4815 / SEQ ID NO: 9782, SEQ ID
NO: 4816 / SEQ ID NO: 9783, SEQ ID NO: 4817 / SEQ ID NO: 9784, SEQ ID NO: 4818 /
SEQ ID NO: 9785, SEQ ID NO: 4819 / SEQ ID NO: 9786, SEQ ID NO: 4820 / SEQ ID NO:
9787, SEQ ID NO: 4821 / SEQ ID NO: 9788, SEQ ID NO: 4822 / SEQ ID NO: 9789, SEQ ID
NO: 4823 / SEQ ID NO: 9790, SEQ ID NO: 4824 / SEQ ID NO: 9791, SEQ ID NO: 4825 /
SEQ ID NO: 9792, SEQ ID NO: 4826 / SEQ ID NO: 9793, SEQ ID NO: 4827 / SEQ ID NO:
9794, SEQ ID NO: 4828 / SEQ ID NO: 9795, SEQ ID NO: 4829 / SEQ ID NO: 9796, SEQ ID
NO: 4830 / SEQ ID NO: 9797, SEQ ID NO: 4832 / SEQ ID NO: 9798, SEQ ID NO: 4833 /
SEQ ID NO: 9799, SEQ ID NO: 4834 / SEQ ID NO: 9800, SEQ ID NO: 4835 / SEQ ID NO:
9801, SEQ ID NO: 4837 / SEQ ID NO: 9802, SEQ ID NO: 4838 / SEQ ID NO: 9803, SEQ ID

NO: 4839 / SEQ ID NO: 9804, SEQ ID NO: 4840 / SEQ ID NO: 9805, SEQ ID NO: 4841 /
SEQ ID NO: 9806, SEQ ID NO: 4842 / SEQ ID NO: 9807, SEQ ID NO: 4843 / SEQ ID NO:
9808, SEQ ID NO: 4844 / SEQ ID NO: 9809, SEQ ID NO: 4845 / SEQ ID NO: 9810, SEQ ID
NO: 4846 / SEQ ID NO: 9811, SEQ ID NO: 4847 / SEQ ID NO: 9812, SEQ ID NO: 4848 /
SEQ ID NO: 9813, SEQ ID NO: 4849 / SEQ ID NO: 9814, SEQ ID NO: 4851 / SEQ ID NO:
9815, SEQ ID NO: 4852 / SEQ ID NO: 9816, SEQ ID NO: 4853 / SEQ ID NO: 9817, SEQ ID
NO: 4854 / SEQ ID NO: 9818, SEQ ID NO: 4855 / SEQ ID NO: 9819, SEQ ID NO: 4856 /
SEQ ID NO: 9820, SEQ ID NO: 4857 / SEQ ID NO: 9821, SEQ ID NO: 4858 / SEQ ID NO:
9822, SEQ ID NO: 4859 / SEQ ID NO: 9823, SEQ ID NO: 4860 / SEQ ID NO: 9824, SEQ ID
NO: 4861 / SEQ ID NO: 9825, SEQ ID NO: 4862 / SEQ ID NO: 9826, SEQ ID NO: 4863 /
SEQ ID NO: 9827, SEQ ID NO: 4864 / SEQ ID NO: 9828, SEQ ID NO: 4865 / SEQ ID NO:
9829, SEQ ID NO: 4867 / SEQ ID NO: 9830, SEQ ID NO: 4868 / SEQ ID NO: 9831, SEQ ID
NO: 4869 / SEQ ID NO: 9832, SEQ ID NO: 4870 / SEQ ID NO: 9833, SEQ ID NO: 4871 /
SEQ ID NO: 9834, SEQ ID NO: 4872 / SEQ ID NO: 9835, SEQ ID NO: 4873 / SEQ ID NO:
9836, SEQ ID NO: 4874 / SEQ ID NO: 9837, SEQ ID NO: 4875 / SEQ ID NO: 9838, SEQ ID
NO: 4876 / SEQ ID NO: 9839, SEQ ID NO: 4877 / SEQ ID NO: 9840, SEQ ID NO: 4878 /
SEQ ID NO: 9841, SEQ ID NO: 4879 / SEQ ID NO: 9842, SEQ ID NO: 4880 / SEQ ID NO:
9843, SEQ ID NO: 4881 / SEQ ID NO: 9844, SEQ ID NO: 4882 / SEQ ID NO: 9845, SEQ ID
NO: 4883 / SEQ ID NO: 9846, SEQ ID NO: 4884 / SEQ ID NO: 9847,
SEQ ID NO: 4885 / SEQ ID NO: 9848, SEQ ID NO: 4886 / SEQ ID NO: 9849, SEQ ID NO:
4887 / SEQ ID NO: 9850, SEQ ID NO: 4888 / SEQ ID NO: 9851, SEQ ID NO: 4889 / SEQ ID
NO: 9852, SEQ ID NO: 4890 / SEQ ID NO: 9853, SEQ ID NO: 4891 / SEQ ID NO: 9854, SEQ
ID NO: 4892 / SEQ ID NO: 9855, SEQ ID NO: 4894 / SEQ ID NO: 9856, SEQ ID NO: 4895 /
SEQ ID NO: 9857, SEQ ID NO: 4896 / SEQ ID NO: 9858, SEQ ID NO: 4897 / SEQ ID NO:
9859, SEQ ID NO: 4898 / SEQ ID NO: 9860, SEQ ID NO: 4899 / SEQ ID NO: 9861, SEQ ID
NO: 4900 / SEQ ID NO: 9862, SEQ ID NO: 4901 / SEQ ID NO: 9863, SEQ ID NO: 4902 /
SEQ ID NO: 9864, SEQ ID NO: 4903 / SEQ ID NO: 9865, SEQ ID NO: 4904 / SEQ ID NO:
9866, SEQ ID NO: 4905 / SEQ ID NO: 9867, SEQ ID NO: 4906 / SEQ ID NO: 9868, SEQ ID
NO: 4907 / SEQ ID NO: 9869, SEQ ID NO: 4908 / SEQ ID NO: 9870, SEQ ID NO: 4909 /
SEQ ID NO: 9871, SEQ ID NO: 4910 / SEQ ID NO: 9872, SEQ ID NO: 4911 / SEQ ID NO:
9873, SEQ ID NO: 4912 / SEQ ID NO: 9874, SEQ ID NO: 4913 / SEQ ID NO: 9875, SEQ ID
NO: 4914 / SEQ ID NO: 9876, SEQ ID NO: 4915 / SEQ ID NO: 9877, SEQ ID NO: 4916 /
SEQ ID NO: 9878, SEQ ID NO: 4917 / SEQ ID NO: 9879, SEQ ID NO: 4918 / SEQ ID NO:
9880, SEQ ID NO: 4919 / SEQ ID NO: 9881, SEQ ID NO: 4920 / SEQ ID NO: 9882, SEQ ID
NO: 4921 / SEQ ID NO: 9883, SEQ ID NO: 4922 / SEQ ID NO: 9884, SEQ ID NO: 4923 /
SEQ ID NO: 9885, SEQ ID NO: 4924 / SEQ ID NO: 9886, SEQ ID NO: 4925 / SEQ ID NO:
9887, SEQ ID NO: 4926 / SEQ ID NO: 9888, SEQ ID NO: 4927 / SEQ ID NO: 9889, SEQ ID
NO: 4928 / SEQ ID NO: 9890, SEQ ID NO: 4929 / SEQ ID NO: 9891, SEQ ID NO: 4931 /
SEQ ID NO: 9892, SEQ ID NO: 4932 / SEQ ID NO: 9893, SEQ ID NO: 4933 / SEQ ID NO:
9894, SEQ ID NO: 4934 / SEQ ID NO: 9895, SEQ ID NO: 4935 / SEQ ID NO: 9896, SEQ ID
NO: 4936 / SEQ ID NO: 9897, SEQ ID NO: 4937 / SEQ ID NO: 9898, SEQ ID NO: 4938 /
SEQ ID NO: 9899, SEQ ID NO: 4939 / SEQ ID NO: 9900, SEQ ID NO: 4940 / SEQ ID NO:
9901, SEQ ID NO: 4941 / SEQ ID NO: 9902, SEQ ID NO: 4942 / SEQ ID NO: 9903, SEQ ID
NO: 4944 / SEQ ID NO: 9904, SEQ ID NO: 4945 / SEQ ID NO: 9905, SEQ ID NO: 4946 /
SEQ ID NO: 9906, SEQ ID NO: 4947 / SEQ ID NO: 9907, SEQ ID NO: 4948 / SEQ ID NO:
9908, SEQ ID NO: 4949 / SEQ ID NO: 9909, SEQ ID NO: 4950 / SEQ ID NO: 9910, SEQ ID
NO: 4951 / SEQ ID NO: 9911, SEQ ID NO: 4952 / SEQ ID NO: 9912, SEQ ID NO: 4953 /
SEQ ID NO: 9913, SEQ ID NO: 4954 / SEQ ID NO: 9914, SEQ ID NO: 4955 / SEQ ID NO:
9915, SEQ ID NO: 4956 / SEQ ID NO: 9916, SEQ ID NO: 4957 / SEQ ID NO: 9917, SEQ ID
NO: 4958 / SEQ ID NO: 9918, SEQ ID NO: 4959 / SEQ ID NO: 9919, SEQ ID NO: 4960 /
SEQ ID NO: 9920, SEQ ID NO: 4961 / SEQ ID NO: 9921, SEQ ID NO: 4962 / SEQ ID NO:
9922, SEQ ID NO: 4963 / SEQ ID NO: 9923, SEQ ID NO: 4964 / SEQ ID NO: 9924, SEQ ID

NO: 4965 / SEQ ID NO: 9925, SEQ ID NO: 4966 / SEQ ID NO: 9926, SEQ ID NO: 4967 /
SEQ ID NO: 9927, SEQ ID NO: 4968 / SEQ ID NO: 9928, SEQ ID NO: 4969 / SEQ ID NO:
9929, SEQ ID NO: 4970 / SEQ ID NO: 9930, SEQ ID NO: 4971 / SEQ ID NO: 9931, SEQ ID
NO: 4972 / SEQ ID NO: 9932, SEQ ID NO: 4973 / SEQ ID NO: 9933, SEQ ID NO: 4974 /
SEQ ID NO: 9934, SEQ ID NO: 4975 / SEQ ID NO: 9935, SEQ ID NO: 4976 / SEQ ID NO:
9936, SEQ ID NO: 4977 / SEQ ID NO: 9937, SEQ ID NO: 4978 / SEQ ID NO: 9938, SEQ ID
NO: 4979 / SEQ ID NO: 9939, SEQ ID NO: 4980 / SEQ ID NO: 9940, SEQ ID NO: 4981 /
SEQ ID NO: 9941, SEQ ID NO: 4982 / SEQ ID NO: 9942, SEQ ID NO: 4983 / SEQ ID NO:
9943, SEQ ID NO: 4984 / SEQ ID NO: 9944, SEQ ID NO: 4985 / SEQ ID NO: 9945, SEQ ID
NO: 4986 / SEQ ID NO: 9946, SEQ ID NO: 4987 / SEQ ID NO: 9947,
SEQ ID NO: 4988 / SEQ ID NO: 9948, SEQ ID NO: 4989 / SEQ ID NO: 9949, SEQ ID NO:
4990 / SEQ ID NO: 9950, SEQ ID NO: 4991 / SEQ ID NO: 9951, SEQ ID NO: 4992 / SEQ ID
NO: 9952, SEQ ID NO: 4993 / SEQ ID NO: 9953, SEQ ID NO: 4994 / SEQ ID NO: 9954, SEQ
ID NO: 4995 / SEQ ID NO: 9955, SEQ ID NO: 4996 / SEQ ID NO: 9956, SEQ ID NO: 4997 /
SEQ ID NO: 9957, SEQ ID NO: 4998 / SEQ ID NO: 9958, SEQ ID NO: 4999 / SEQ ID NO:
9959, SEQ ID NO: 5000 / SEQ ID NO: 9960, SEQ ID NO: 5001 / SEQ ID NO: 9961, SEQ ID
NO: 5002 / SEQ ID NO: 9962, SEQ ID NO: 5004 / SEQ ID NO: 9963, SEQ ID NO: 5005 /
SEQ ID NO: 9964, SEQ ID NO: 5006 / SEQ ID NO: 9965, SEQ ID NO: 5007 / SEQ ID NO:
9966, SEQ ID NO: 5008 / SEQ ID NO: 9967, SEQ ID NO: 5009 / SEQ ID NO: 9968, SEQ ID
NO: 5010 / SEQ ID NO: 9969, SEQ ID NO: 5011 / SEQ ID NO: 9970, SEQ ID NO: 5012 /
SEQ ID NO: 9971, SEQ ID NO: 5013 / SEQ ID NO: 9972, SEQ ID NO: 5014 / SEQ ID NO:
9973, SEQ ID NO: 5016 / SEQ ID NO: 9974, SEQ ID NO: 5017 / SEQ ID NO: 9975, SEQ ID
NO: 5018 / SEQ ID NO: 9976, SEQ ID NO: 5019 / SEQ ID NO: 9977, SEQ ID NO: 5020 /
SEQ ID NO: 9978, SEQ ID NO: 5021 / SEQ ID NO: 9979, SEQ ID NO: 5022 / SEQ ID NO:
9980, SEQ ID NO: 5024 / SEQ ID NO: 9981, SEQ ID NO: 5025 / SEQ ID NO: 9982, SEQ ID
NO: 5026 / SEQ ID NO: 9983, SEQ ID NO: 5027 / SEQ ID NO: 9984, SEQ ID NO: 5028 /
SEQ ID NO: 9985, SEQ ID NO: 5029 / SEQ ID NO: 9986, SEQ ID NO: 5030 / SEQ ID NO:
9987, SEQ ID NO: 5031 / SEQ ID NO: 9988, SEQ ID NO: 5032 / SEQ ID NO: 9989, SEQ ID
NO: 5033 / SEQ ID NO: 9990, SEQ ID NO: 5035 / SEQ ID NO: 9991, SEQ ID NO: 5036 /
SEQ ID NO: 9992, SEQ ID NO: 5037 / SEQ ID NO: 9993, SEQ ID NO: 5038 / SEQ ID NO:
9994, SEQ ID NO: 5039 / SEQ ID NO: 9995, SEQ ID NO: 5040 / SEQ ID NO: 9996, SEQ ID
NO: 5041 / SEQ ID NO: 9997, SEQ ID NO: 5042 / SEQ ID NO: 9998, SEQ ID NO: 5043 /
SEQ ID NO: 9999, SEQ ID NO: 5044 / SEQ ID NO: 10000, SEQ ID NO: 5045 / SEQ ID NO:
10001, SEQ ID NO: 5046 / SEQ ID NO: 10002, SEQ ID NO: 5047 / SEQ ID NO: 10003, SEQ
ID NO: 5048 / SEQ ID NO: 10004, SEQ ID NO: 5049 / SEQ ID NO: 10005, SEQ ID NO: 5050
/ SEQ ID NO: 10006, SEQ ID NO: 5051 / SEQ ID NO: 10007, SEQ ID NO: 5052 / SEQ ID
NO: 10008, SEQ ID NO: 5053 / SEQ ID NO: 10009, SEQ ID NO: 5054 / SEQ ID NO: 10010,
SEQ ID NO: 5055 / SEQ ID NO: 10011, SEQ ID NO: 5056 / SEQ ID NO: 10012, SEQ ID NO:
5057 / SEQ ID NO: 10013, SEQ ID NO: 5058 / SEQ ID NO: 10014, SEQ ID NO: 5059 / SEQ
ID NO: 10015, SEQ ID NO: 5061 / SEQ ID NO: 10016, SEQ ID NO: 5062 / SEQ ID NO:
10017, SEQ ID NO: 5064 / SEQ ID NO: 10018, SEQ ID NO: 5065 / SEQ ID NO: 10019, SEQ
ID NO: 5066 / SEQ ID NO: 10020, SEQ ID NO: 5068 / SEQ ID NO: 10021, SEQ ID NO: 5069
/ SEQ ID NO: 10022, SEQ ID NO: 5070 / SEQ ID NO: 10023, SEQ ID NO: 5071 / SEQ ID
NO: 10024, SEQ ID NO: 5072 / SEQ ID NO: 10025, SEQ ID NO: 5073 / SEQ ID NO: 10026,
SEQ ID NO: 5074 / SEQ ID NO: 10027, SEQ ID NO: 5075 / SEQ ID NO: 10028, SEQ ID NO:
5076 / SEQ ID NO: 10029, SEQ ID NO: 5077 / SEQ ID NO: 10030, SEQ ID NO: 5078 / SEQ
ID NO: 10031, SEQ ID NO: 5079 / SEQ ID NO: 10032, SEQ ID NO: 5080 / SEQ ID NO:
10033, SEQ ID NO: 5082 / SEQ ID NO: 10034, SEQ ID NO: 5083 / SEQ ID NO: 10035, SEQ
ID NO: 5084 / SEQ ID NO: 10036, SEQ ID NO: 5085 / SEQ ID NO: 10037, SEQ ID NO: 5086
/ SEQ ID NO: 10038, SEQ ID NO: 5087 / SEQ ID NO: 10039, SEQ ID NO: 5088 / SEQ ID
NO: 10040, SEQ ID NO: 5089 / SEQ ID NO: 10041, SEQ ID NO: 5090 / SEQ ID NO: 10042,
SEQ ID NO: 5091 / SEQ ID NO: 10043, SEQ ID NO: 5092 / SEQ ID NO: 10044, SEQ ID NO:

5093 / SEQ ID NO: 10045, SEQ ID NO: 5094 / SEQ ID NO: 10046, SEQ ID NO: 5095 / SEQ
ID NO: 10047,
SEQ ID NO: 5096 / SEQ ID NO: 10048, SEQ ID NO: 5097 / SEQ ID NO: 10049, SEQ ID NO:
5098 / SEQ ID NO: 10050, SEQ ID NO: 5099 / SEQ ID NO: 10051, SEQ ID NO: 5101 / SEQ
ID NO: 10052, SEQ ID NO: 5102 / SEQ ID NO: 10053, SEQ ID NO: 5103 / SEQ ID NO:
10054, SEQ ID NO: 5104 / SEQ ID NO: 10055, SEQ ID NO: 5105 / SEQ ID NO: 10056, SEQ
ID NO: 5106 / SEQ ID NO: 10057, SEQ ID NO: 5107 / SEQ ID NO: 10058, SEQ ID NO: 5108
/ SEQ ID NO: 10059, SEQ ID NO: 5109 / SEQ ID NO: 10060, SEQ ID NO: 5110 / SEQ ID
NO: 10061, SEQ ID NO: 5111 / SEQ ID NO: 10062, SEQ ID NO: 5112 / SEQ ID NO: 10063,
SEQ ID NO: 5113 / SEQ ID NO: 10064, SEQ ID NO: 5114 / SEQ ID NO: 10065, SEQ ID NO:
5115 / SEQ ID NO: 10066, SEQ ID NO: 5116 / SEQ ID NO: 10067, SEQ ID NO: 5117 / SEQ
ID NO: 10068, SEQ ID NO: 5118 / SEQ ID NO: 10069, SEQ ID NO: 5119 / SEQ ID NO:
10070, SEQ ID NO: 5120 / SEQ ID NO: 10071, SEQ ID NO: 5121 / SEQ ID NO: 10072, SEQ
ID NO: 5122 / SEQ ID NO: 10073, SEQ ID NO: 5123 / SEQ ID NO: 10074, SEQ ID NO: 5124
/ SEQ ID NO: 10075, SEQ ID NO: 5127 / SEQ ID NO: 10076, SEQ ID NO: 5128 / SEQ ID
NO: 10077, SEQ ID NO: 5129 / SEQ ID NO: 10078, SEQ ID NO: 5130 / SEQ ID NO: 10079,
SEQ ID NO: 5131 / SEQ ID NO: 10080, SEQ ID NO: 5133 / SEQ ID NO: 10081, SEQ ID NO:
5134 / SEQ ID NO: 10082, SEQ ID NO: 5135 / SEQ ID NO: 10083, SEQ ID NO: 5137 / SEQ
ID NO: 10084, SEQ ID NO: 5138 / SEQ ID NO: 10085, SEQ ID NO: 5139 / SEQ ID NO:
10086, SEQ ID NO: 5140 / SEQ ID NO: 10087, SEQ ID NO: 5141 / SEQ ID NO: 10088, SEQ
ID NO: 5142 / SEQ ID NO: 10089, SEQ ID NO: 5143 / SEQ ID NO: 10090, SEQ ID NO: 5144
/ SEQ ID NO: 10091, SEQ ID NO: 5145 / SEQ ID NO: 10092, SEQ ID NO: 5146 / SEQ ID
NO: 10093, SEQ ID NO: 5147 / SEQ ID NO: 10094, SEQ ID NO: 5148 / SEQ ID NO: 10095,
SEQ ID NO: 5149 / SEQ ID NO: 10096, SEQ ID NO: 5150 / SEQ ID NO: 10097, SEQ ID NO:
5151 / SEQ ID NO: 10098, SEQ ID NO: 5153 / SEQ ID NO: 10099, SEQ ID NO: 5154 / SEQ
ID NO: 10100, SEQ ID NO: 5155 / SEQ ID NO: 10101, SEQ ID NO: 5156 / SEQ ID NO:
10102, SEQ ID NO: 5157 / SEQ ID NO: 10103, SEQ ID NO: 5158 / SEQ ID NO: 10104, SEQ
ID NO: 5159 / SEQ ID NO: 10105, SEQ ID NO: 5160 / SEQ ID NO: 10106, SEQ ID NO: 5161
/ SEQ ID NO: 10107, SEQ ID NO: 5162 / SEQ ID NO: 10108, SEQ ID NO: 5163 / SEQ ID
NO: 10109, SEQ ID NO: 5164 / SEQ ID NO: 10110, SEQ ID NO: 5165 / SEQ ID NO: 10111,
SEQ ID NO: 5166 / SEQ ID NO: 10112, SEQ ID NO: 5167 / SEQ ID NO: 10113, SEQ ID NO:
5168 / SEQ ID NO: 10114, SEQ ID NO: 5169 / SEQ ID NO: 10115, SEQ ID NO: 5170 / SEQ
ID NO: 10116, SEQ ID NO: 5171 / SEQ ID NO: 10117, SEQ ID NO: 5172 / SEQ ID NO:
10118, SEQ ID NO: 5173 / SEQ ID NO: 10119, SEQ ID NO: 5174 / SEQ ID NO: 10120, SEQ
ID NO: 5175 / SEQ ID NO: 10121, SEQ ID NO: 5176 / SEQ ID NO: 10122, SEQ ID NO: 5177
/ SEQ ID NO: 10123, SEQ ID NO: 5178 / SEQ ID NO: 10124, SEQ ID NO: 5179 / SEQ ID
NO: 10125, SEQ ID NO: 5180 / SEQ ID NO: 10126, SEQ ID NO: 5181 / SEQ ID NO: 10127,
SEQ ID NO: 5182 / SEQ ID NO: 10128, SEQ ID NO: 5183 / SEQ ID NO: 10129, SEQ ID NO:
5184 / SEQ ID NO: 10130, SEQ ID NO: 5185 / SEQ ID NO: 10131, SEQ ID NO: 5186 / SEQ
ID NO: 10132, SEQ ID NO: 5187 / SEQ ID NO: 10133, SEQ ID NO: 5188 / SEQ ID NO:
10134, SEQ ID NO: 5189 / SEQ ID NO: 10135, SEQ ID NO: 5190 / SEQ ID NO: 10136, SEQ
ID NO: 5191 / SEQ ID NO: 10137, SEQ ID NO: 5192 / SEQ ID NO: 10138, SEQ ID NO: 5193
/ SEQ ID NO: 10139, SEQ ID NO: 5194 / SEQ ID NO: 10140, SEQ ID NO: 5195 / SEQ ID
NO: 10141, SEQ ID NO: 5196 / SEQ ID NO: 10142, SEQ ID NO: 5197 / SEQ ID NO: 10143,
SEQ ID NO: 5198 / SEQ ID NO: 10144, SEQ ID NO: 5200 / SEQ ID NO: 10145, SEQ ID NO:
5201 / SEQ ID NO: 10146, SEQ ID NO: 5202 / SEQ ID NO: 10147,
SEQ ID NO: 5204 / SEQ ID NO: 10148, SEQ ID NO: 5206 / SEQ ID NO: 10149, SEQ ID NO:
5207 / SEQ ID NO: 10150, SEQ ID NO: 5208 / SEQ ID NO: 10151, SEQ ID NO: 5209 / SEQ
ID NO: 10152, SEQ ID NO: 5211 / SEQ ID NO: 10153, SEQ ID NO: 5212 / SEQ ID NO:
10154, SEQ ID NO: 5213 / SEQ ID NO: 10155, SEQ ID NO: 5214 / SEQ ID NO: 10156, SEQ
ID NO: 5215 / SEQ ID NO: 10157, SEQ ID NO: 5216 / SEQ ID NO: 10158, SEQ ID NO: 5217
/ SEQ ID NO: 10159, SEQ ID NO: 5218 / SEQ ID NO: 10160, SEQ ID NO: 5219 / SEQ ID

NO: 10161, SEQ ID NO: 5220 / SEQ ID NO: 10162, SEQ ID NO: 5221 / SEQ ID NO: 10163,
SEQ ID NO: 5222 / SEQ ID NO: 10164, SEQ ID NO: 5223 / SEQ ID NO: 10165, SEQ ID NO:
5224 / SEQ ID NO: 10166, SEQ ID NO: 5225 / SEQ ID NO: 10167, SEQ ID NO: 5227 / SEQ
ID NO: 10168, SEQ ID NO: 5229 / SEQ ID NO: 10169, SEQ ID NO: 5231 / SEQ ID NO:
10170, SEQ ID NO: 5232 / SEQ ID NO: 10171, SEQ ID NO: 5234 / SEQ ID NO: 10172, SEQ
ID NO: 5235 / SEQ ID NO: 10173, SEQ ID NO: 5236 / SEQ ID NO: 10174, SEQ ID NO: 5251
/ SEQ ID NO: 10175, SEQ ID NO: 5252 / SEQ ID NO: 10176, SEQ ID NO: 5253 / SEQ ID
NO: 10177, SEQ ID NO: 5254 / SEQ ID NO: 10178, SEQ ID NO: 5255 / SEQ ID NO: 10179,
SEQ ID NO: 5256 / SEQ ID NO: 10180, SEQ ID NO: 5257 / SEQ ID NO: 10181, SEQ ID NO:
5258 / SEQ ID NO: 10182, SEQ ID NO: 5259 / SEQ ID NO: 10183, SEQ ID NO: 5260 / SEQ
ID NO: 10184, SEQ ID NO: 5261 / SEQ ID NO: 10185, SEQ ID NO: 5262 / SEQ ID NO:
10186, SEQ ID NO: 5263 / SEQ ID NO: 10187, SEQ ID NO: 5264 / SEQ ID NO: 10188, SEQ
ID NO: 5265 / SEQ ID NO: 10189, SEQ ID NO: 5266 / SEQ ID NO: 10190, SEQ ID NO: 5267
/ SEQ ID NO: 10191, SEQ ID NO: 5268 / SEQ ID NO: 10192, SEQ ID NO: 5269 / SEQ ID
NO: 10193, SEQ ID NO: 5270 / SEQ ID NO: 10194, SEQ ID NO: 5271 / SEQ ID NO: 10195,
SEQ ID NO: 5272 / SEQ ID NO: 10196, SEQ ID NO: 5273 / SEQ ID NO: 10197, SEQ ID NO:
5274 / SEQ ID NO: 10198, SEQ ID NO: 5275 / SEQ ID NO: 10199, SEQ ID NO: 5276 / SEQ
ID NO: 10200, SEQ ID NO: 5277 / SEQ ID NO: 10201, SEQ ID NO: 5278 / SEQ ID NO:
10202, SEQ ID NO: 5279 / SEQ ID NO: 10203, SEQ ID NO: 5280 / SEQ ID NO: 10204, SEQ
ID NO: 5281 / SEQ ID NO: 10205, SEQ ID NO: 5282 / SEQ ID NO: 10206, SEQ ID NO: 5283
/ SEQ ID NO: 10207, SEQ ID NO: 5284 / SEQ ID NO: 10208, SEQ ID NO: 5285 / SEQ ID
NO: 10209, SEQ ID NO: 5286 / SEQ ID NO: 10210, SEQ ID NO: 5287 / SEQ ID NO: 10211,
SEQ ID NO: 5288 / SEQ ID NO: 10212, SEQ ID NO: 5289 / SEQ ID NO: 10213, SEQ ID NO:
5290 / SEQ ID NO: 10214, SEQ ID NO: 5291 / SEQ ID NO: 10215, SEQ ID NO: 5292 / SEQ
ID NO: 10216, SEQ ID NO: 5293 / SEQ ID NO: 10217, SEQ ID NO: 5294 / SEQ ID NO:
10218, SEQ ID NO: 5295 / SEQ ID NO: 10219, SEQ ID NO: 5296 / SEQ ID NO: 10220, SEQ
ID NO: 5297 / SEQ ID NO: 10221, SEQ ID NO: 5298 / SEQ ID NO: 10222, SEQ ID NO: 5299
/ SEQ ID NO: 10223, SEQ ID NO: 5300 / SEQ ID NO: 10224, SEQ ID NO: 5301 / SEQ ID
NO: 10225, SEQ ID NO: 5302 / SEQ ID NO: 10226, SEQ ID NO: 5303 / SEQ ID NO: 10227,
SEQ ID NO: 5304 / SEQ ID NO: 10228, SEQ ID NO: 5305 / SEQ ID NO: 10229, SEQ ID NO:
5306 / SEQ ID NO: 10230, SEQ ID NO: 5307 / SEQ ID NO: 10231, SEQ ID NO: 5308 / SEQ
ID NO: 10232, SEQ ID NO: 5309 / SEQ ID NO: 10233, SEQ ID NO: 5310 / SEQ ID NO:
10234, SEQ ID NO: 5311 / SEQ ID NO: 10235, SEQ ID NO: 5312 / SEQ ID NO: 10236, SEQ
ID NO: 5313 / SEQ ID NO: 10237, SEQ ID NO: 5314 / SEQ ID NO: 10238, SEQ ID NO: 5315
/ SEQ ID NO: 10239, SEQ ID NO: 5317 / SEQ ID NO: 10240, SEQ ID NO: 5318 / SEQ ID
NO: 10241, SEQ ID NO: 5319 / SEQ ID NO: 10242, SEQ ID NO: 5320 / SEQ ID NO: 10243,
SEQ ID NO: 5321 / SEQ ID NO: 10244, SEQ ID NO: 5322 / SEQ ID NO: 10245, SEQ ID NO:
5323 / SEQ ID NO: 10246, SEQ ID NO: 5324 / SEQ ID NO: 10247,
SEQ ID NO: 5325 / SEQ ID NO: 10248, SEQ ID NO: 5326 / SEQ ID NO: 10249, SEQ ID NO:
5327 / SEQ ID NO: 10250, SEQ ID NO: 5328 / SEQ ID NO: 10251, SEQ ID NO: 5329 / SEQ
ID NO: 10252, SEQ ID NO: 5330 / SEQ ID NO: 10253, SEQ ID NO: 5331 / SEQ ID NO:
10254, SEQ ID NO: 5332 / SEQ ID NO: 10255, SEQ ID NO: 5333 / SEQ ID NO: 10256, SEQ
ID NO: 5334 / SEQ ID NO: 10257, SEQ ID NO: 5335 / SEQ ID NO: 10258, SEQ ID NO: 5336
/ SEQ ID NO: 10259, SEQ ID NO: 5337 / SEQ ID NO: 10260, SEQ ID NO: 5338 / SEQ ID
NO: 10261, SEQ ID NO: 5339 / SEQ ID NO: 10262, SEQ ID NO: 5340 / SEQ ID NO: 10263,
SEQ ID NO: 5341 / SEQ ID NO: 10264, SEQ ID NO: 5342 / SEQ ID NO: 10265, SEQ ID NO:
5343 / SEQ ID NO: 10266, SEQ ID NO: 5344 / SEQ ID NO: 10267, SEQ ID NO: 5345 / SEQ
ID NO: 10268, SEQ ID NO: 5346 / SEQ ID NO: 10269, SEQ ID NO: 5347 / SEQ ID NO:
10270, SEQ ID NO: 5348 / SEQ ID NO: 10271, SEQ ID NO: 5349 / SEQ ID NO: 10272, SEQ
ID NO: 5350 / SEQ ID NO: 10273, SEQ ID NO: 5351 / SEQ ID NO: 10274, SEQ ID NO: 5352
/ SEQ ID NO: 10275, SEQ ID NO: 5353 / SEQ ID NO: 10276, SEQ ID NO: 5354 / SEQ ID
NO: 10277, SEQ ID NO: 5355 / SEQ ID NO: 10278, SEQ ID NO: 5356 / SEQ ID NO: 10279,

SEQ ID NO: 5357 / SEQ ID NO: 10280, SEQ ID NO: 5358 / SEQ ID NO: 10281, SEQ ID NO:
5359 / SEQ ID NO: 10282, SEQ ID NO: 5360 / SEQ ID NO: 10283, SEQ ID NO: 5362 / SEQ
ID NO: 10284, SEQ ID NO: 5363 / SEQ ID NO: 10285, SEQ ID NO: 5364 / SEQ ID NO:
10286, SEQ ID NO: 5365 / SEQ ID NO: 10287, SEQ ID NO: 5366 / SEQ ID NO: 10288, SEQ
ID NO: 5367 / SEQ ID NO: 10289, SEQ ID NO: 5368 / SEQ ID NO: 10290, SEQ ID NO: 5369
/ SEQ ID NO: 10291, SEQ ID NO: 5370 / SEQ ID NO: 10292, SEQ ID NO: 5371 / SEQ ID
NO: 10293, SEQ ID NO: 5372 / SEQ ID NO: 10294, SEQ ID NO: 5373 / SEQ ID NO: 10295,
SEQ ID NO: 5374 / SEQ ID NO: 10296, SEQ ID NO: 5375 / SEQ ID NO: 10297, SEQ ID NO:
5376 / SEQ ID NO: 10298, SEQ ID NO: 5377 / SEQ ID NO: 10299, SEQ ID NO: 5378 / SEQ
ID NO: 10300, SEQ ID NO: 5379 / SEQ ID NO: 10301, SEQ ID NO: 5380 / SEQ ID NO:
10302, SEQ ID NO: 5381 / SEQ ID NO: 10303, SEQ ID NO: 5382 / SEQ ID NO: 10304, SEQ
ID NO: 5383 / SEQ ID NO: 10305, SEQ ID NO: 5384 / SEQ ID NO: 10306, SEQ ID NO: 5385
/ SEQ ID NO: 10307, SEQ ID NO: 5386 / SEQ ID NO: 10308, SEQ ID NO: 5387 / SEQ ID
NO: 10309, SEQ ID NO: 5388 / SEQ ID NO: 10310, SEQ ID NO: 5389 / SEQ ID NO: 10311,
SEQ ID NO: 5390 / SEQ ID NO: 10312, SEQ ID NO: 5391 / SEQ ID NO: 10313, SEQ ID NO:
5392 / SEQ ID NO: 10314, SEQ ID NO: 5393 / SEQ ID NO: 10315, SEQ ID NO: 5394 / SEQ
ID NO: 10316, SEQ ID NO: 5395 / SEQ ID NO: 10317, SEQ ID NO: 5396 / SEQ ID NO:
10318, SEQ ID NO: 5397 / SEQ ID NO: 10319, SEQ ID NO: 5398 / SEQ ID NO: 10320, SEQ
ID NO: 5399 / SEQ ID NO: 10321, SEQ ID NO: 5400 / SEQ ID NO: 10322, SEQ ID NO: 5401
/ SEQ ID NO: 10323, SEQ ID NO: 5402 / SEQ ID NO: 10324, SEQ ID NO: 5403 / SEQ ID
NO: 10325, SEQ ID NO: 5404 / SEQ ID NO: 10326, SEQ ID NO: 5405 / SEQ ID NO: 10327,
SEQ ID NO: 5407 / SEQ ID NO: 10328, SEQ ID NO: 5408 / SEQ ID NO: 10329, SEQ ID NO:
5409 / SEQ ID NO: 10330, SEQ ID NO: 5410 / SEQ ID NO: 10331, SEQ ID NO: 5411 / SEQ
ID NO: 10332, SEQ ID NO: 5412 / SEQ ID NO: 10333, SEQ ID NO: 5413 / SEQ ID NO:
10334, SEQ ID NO: 5414 / SEQ ID NO: 10335, SEQ ID NO: 5415 / SEQ ID NO: 10336, SEQ
ID NO: 5416 / SEQ ID NO: 10337, SEQ ID NO: 5417 / SEQ ID NO: 10338, SEQ ID NO: 5418
/ SEQ ID NO: 10339, SEQ ID NO: 5419 / SEQ ID NO: 10340, SEQ ID NO: 5420 / SEQ ID
NO: 10341, SEQ ID NO: 5421 / SEQ ID NO: 10342, SEQ ID NO: 5422 / SEQ ID NO: 10343,
SEQ ID NO: 5423 / SEQ ID NO: 10344, SEQ ID NO: 5424 / SEQ ID NO: 10345, SEQ ID NO:
5425 / SEQ ID NO: 10346, SEQ ID NO: 5426 / SEQ ID NO: 10347,
SEQ ID NO: 5427 / SEQ ID NO: 10348, SEQ ID NO: 5428 / SEQ ID NO: 10349, SEQ ID NO:
5429 / SEQ ID NO: 10350, SEQ ID NO: 5430 / SEQ ID NO: 10351, SEQ ID NO: 5431 / SEQ
ID NO: 10352, SEQ ID NO: 5432 / SEQ ID NO: 10353, SEQ ID NO: 5433 / SEQ ID NO:
10354, SEQ ID NO: 5434 / SEQ ID NO: 10355, SEQ ID NO: 5435 / SEQ ID NO: 10356, SEQ
ID NO: 5436 / SEQ ID NO: 10357, SEQ ID NO: 5437 / SEQ ID NO: 10358, SEQ ID NO: 5438
/ SEQ ID NO: 10359, SEQ ID NO: 5439 / SEQ ID NO: 10360, SEQ ID NO: 5440 / SEQ ID
NO: 10361, SEQ ID NO: 5442 / SEQ ID NO: 10362, SEQ ID NO: 5443 / SEQ ID NO: 10363,
SEQ ID NO: 5444 / SEQ ID NO: 10364, SEQ ID NO: 5445 / SEQ ID NO: 10365, SEQ ID NO:
5446 / SEQ ID NO: 10366, SEQ ID NO: 5447 / SEQ ID NO: 10367, SEQ ID NO: 5448 / SEQ
ID NO: 10368, SEQ ID NO: 5449 / SEQ ID NO: 10369, SEQ ID NO: 5450 / SEQ ID NO:
10370, SEQ ID NO: 5451 / SEQ ID NO: 10371, SEQ ID NO: 5452 / SEQ ID NO: 10372, SEQ
ID NO: 5453 / SEQ ID NO: 10373, SEQ ID NO: 5454 / SEQ ID NO: 10374, SEQ ID NO: 5455
/ SEQ ID NO: 10375, SEQ ID NO: 5457 / SEQ ID NO: 10376, SEQ ID NO: 5458 / SEQ ID
NO: 10377, SEQ ID NO: 5460 / SEQ ID NO: 10378, SEQ ID NO: 5461 / SEQ ID NO: 10379,
SEQ ID NO: 5462 / SEQ ID NO: 10380, SEQ ID NO: 5463 / SEQ ID NO: 10381, SEQ ID NO:
5464 / SEQ ID NO: 10382, SEQ ID NO: 5465 / SEQ ID NO: 10383, SEQ ID NO: 5466 / SEQ
ID NO: 10384, SEQ ID NO: 5467 / SEQ ID NO: 10385, SEQ ID NO: 5468 / SEQ ID NO:
10386, SEQ ID NO: 5469 / SEQ ID NO: 10387, SEQ ID NO: 5470 / SEQ ID NO: 10388, SEQ
ID NO: 5471 / SEQ ID NO: 10389, SEQ ID NO: 5472 / SEQ ID NO: 10390, SEQ ID NO: 5473
/ SEQ ID NO: 10391, SEQ ID NO: 5474 / SEQ ID NO: 10392, SEQ ID NO: 5475 / SEQ ID
NO: 10393, SEQ ID NO: 5476 / SEQ ID NO: 10394, SEQ ID NO: 5477 / SEQ ID NO: 10395,
SEQ ID NO: 5478 / SEQ ID NO: 10396, SEQ ID NO: 5479 / SEQ ID NO: 10397, SEQ ID NO:

5480 / SEQ ID NO: 10398, SEQ ID NO: 5481 / SEQ ID NO: 10399, SEQ ID NO: 5482 / SEQ
ID NO: 10400, SEQ ID NO: 5483 / SEQ ID NO: 10401, SEQ ID NO: 5484 / SEQ ID NO:
10402, SEQ ID NO: 5485 / SEQ ID NO: 10403, SEQ ID NO: 5486 / SEQ ID NO: 10404, SEQ
ID NO: 5487 / SEQ ID NO: 10405, SEQ ID NO: 5488 / SEQ ID NO: 10406, SEQ ID NO: 5489
/ SEQ ID NO: 10407, SEQ ID NO: 5490 / SEQ ID NO: 10408, SEQ ID NO: 5491 / SEQ ID
NO: 10409, SEQ ID NO: 5492 / SEQ ID NO: 10410, SEQ ID NO: 5493 / SEQ ID NO: 10411,
SEQ ID NO: 5494 / SEQ ID NO: 10412, SEQ ID NO: 5496 / SEQ ID NO: 10413, SEQ ID NO:
5497 / SEQ ID NO: 10414, SEQ ID NO: 5498 / SEQ ID NO: 10415, SEQ ID NO: 5499 / SEQ
ID NO: 10416, SEQ ID NO: 5500 / SEQ ID NO: 10417, SEQ ID NO: 5501 / SEQ ID NO:
10418, SEQ ID NO: 5502 / SEQ ID NO: 10419, SEQ ID NO: 5503 / SEQ ID NO: 10420, SEQ
ID NO: 5504 / SEQ ID NO: 10421, SEQ ID NO: 5505 / SEQ ID NO: 10422, SEQ ID NO: 5506
/ SEQ ID NO: 10423, SEQ ID NO: 5507 / SEQ ID NO: 10424, SEQ ID NO: 5508 / SEQ ID
NO: 10425, SEQ ID NO: 5509 / SEQ ID NO: 10426, SEQ ID NO: 5510 / SEQ ID NO: 10427,
SEQ ID NO: 5511 / SEQ ID NO: 10428, SEQ ID NO: 5512 / SEQ ID NO: 10429, SEQ ID NO:
5513 / SEQ ID NO: 10430, SEQ ID NO: 5514 / SEQ ID NO: 10431, SEQ ID NO: 5515 / SEQ
ID NO: 10432, SEQ ID NO: 5517 / SEQ ID NO: 10433, SEQ ID NO: 5518 / SEQ ID NO:
10434, SEQ ID NO: 5519 / SEQ ID NO: 10435, SEQ ID NO: 5520 / SEQ ID NO: 10436, SEQ
ID NO: 5521 / SEQ ID NO: 10437, SEQ ID NO: 5522 / SEQ ID NO: 10438, SEQ ID NO: 5523
/ SEQ ID NO: 10439, SEQ ID NO: 5524 / SEQ ID NO: 10440, SEQ ID NO: 5525 / SEQ ID
NO: 10441, SEQ ID NO: 5526 / SEQ ID NO: 10442, SEQ ID NO: 5527 / SEQ ID NO: 10443,
SEQ ID NO: 5528 / SEQ ID NO: 10444, SEQ ID NO: 5529 / SEQ ID NO: 10445, SEQ ID NO:
5530 / SEQ ID NO: 10446, SEQ ID NO: 5531 / SEQ ID NO: 10447,
SEQ ID NO: 5532 / SEQ ID NO: 10448, SEQ ID NO: 5533 / SEQ ID NO: 10449, SEQ ID NO:
5534 / SEQ ID NO: 10450, SEQ ID NO: 5535 / SEQ ID NO: 10451, SEQ ID NO: 5536 / SEQ
ID NO: 10452, SEQ ID NO: 5537 / SEQ ID NO: 10453, SEQ ID NO: 5538 / SEQ ID NO:
10454, SEQ ID NO: 5539 / SEQ ID NO: 10455, SEQ ID NO: 5540 / SEQ ID NO: 10456, SEQ
ID NO: 5541 / SEQ ID NO: 10457, SEQ ID NO: 5542 / SEQ ID NO: 10458, SEQ ID NO: 5543
/ SEQ ID NO: 10459, SEQ ID NO: 5544 / SEQ ID NO: 10460, SEQ ID NO: 5545 / SEQ ID
NO: 10461, SEQ ID NO: 5546 / SEQ ID NO: 10462, SEQ ID NO: 5547 / SEQ ID NO: 10463
SEQ ID NO: 16111 / SEQ ID NO: 16165, SEQ ID NO: 16112 / SEQ ID NO: 16166, SEQ ID
NO: 16113 / SEQ ID NO: 16167, SEQ ID NO: 16114 / SEQ ID NO: 16168, SEQ ID NO: 16115
/ SEQ ID NO: 16169, SEQ ID NO: 16116 / SEQ ID NO: 16170, SEQ ID NO: 16117 / SEQ ID
NO: 16171, SEQ ID NO: 16118 / SEQ ID NO: 16172, SEQ ID NO: 16119 / SEQ ID NO: 16173,
SEQ ID NO: 16120 / SEQ ID NO: 16174, SEQ ID NO: 16121 / SEQ ID NO: 16175, SEQ ID
NO: 16122 / SEQ ID NO: 16176, SEQ ID NO: 16123 / SEQ ID NO: 16177, SEQ ID NO: 16124
/ SEQ ID NO: 16178, SEQ ID NO: 16125 / SEQ ID NO: 16179, SEQ ID NO: 16126 / SEQ ID
NO: 16180, SEQ ID NO: 16127 / SEQ ID NO: 16181, SEQ ID NO: 16128 / SEQ ID NO: 16182,
SEQ ID NO: 16129 / SEQ ID NO: 16183, SEQ ID NO: 16130 / SEQ ID NO: 16184, SEQ ID
NO: 16131 / SEQ ID NO: 16185, SEQ ID NO: 16132 / SEQ ID NO: 16186, SEQ ID NO: 16133
/ SEQ ID NO: 16187, SEQ ID NO: 16134 / SEQ ID NO: 16188, SEQ ID NO: 16135 / SEQ ID
NO: 16189, SEQ ID NO: 16136 / SEQ ID NO: 16190, SEQ ID NO: 16137 / SEQ ID NO: 16191,
SEQ ID NO: 16138 / SEQ ID NO: 16192, SEQ ID NO: 16139 / SEQ ID NO: 16193, SEQ ID
NO: 16140 / SEQ ID NO: 16194, SEQ ID NO: 16141 / SEQ ID NO: 16195, SEQ ID NO: 16142
/ SEQ ID NO: 16196, SEQ ID NO: 16143 / SEQ ID NO: 16197, SEQ ID NO: 16144 / SEQ ID
NO: 16198, SEQ ID NO: 16145 / SEQ ID NO: 16199, SEQ ID NO: 16146 / SEQ ID NO: 16200,
SEQ ID NO: 16147 / SEQ ID NO: 16201, SEQ ID NO: 16148 / SEQ ID NO: 16202, SEQ ID
NO: 16149 / SEQ ID NO: 16203, SEQ ID NO: 16150 / SEQ ID NO: 16204, SEQ ID NO: 16151
/ SEQ ID NO: 16205, SEQ ID NO: 16152 / SEQ ID NO: 16206, SEQ ID NO: 16153 / SEQ ID
NO: 16207, SEQ ID NO: 16154 / SEQ ID NO: 16208, SEQ ID NO: 16155 / SEQ ID NO: 16209,
SEQ ID NO: 16156 / SEQ ID NO: 16210, SEQ ID NO: 16157 / SEQ ID NO: 16211, SEQ ID
NO: 16158 / SEQ ID NO: 16212, SEQ ID NO: 16159 / SEQ ID NO: 16213, SEQ ID NO: 16160
/ SEQ ID NO: 16214, SEQ ID NO: 16161 / SEQ ID NO: 16215, SEQ ID NO: 16162 / SEQ ID

NO: 16216, SEQ ID NO: 16163 / SEQ ID NO: 16217, and SEQ ID NO: 16164 / SEQ ID NO:
16218

4. A polynucleotide which can be synthesized with the primer set of claim 2 or 3.

5. A polynucleotide comprising a coding region in the polynucleotide of claim 4.

6. A substantially pure protein encoded by polynucleotide of claim 4.

7. A partial peptide of the protein of claim 6.

8. An isolated polynucleotide selected from the group consisting of
(a) a polynucleotide comprising a coding region of the nucleotide sequence set forth in any one of the following SEQ ID NOs: 
SEQ ID NO: 10468, SEQ ID NO: 10470, SEQ ID NO: 10471, SEQ ID NO: 10472, SEQ ID
NO: 10473, SEQ ID NO: 10475, SEQ ID NO: 10477, SEQ ID NO: 10479, SEQ ID NO: 10481,
SEQ ID NO: 10483, SEQ ID NO: 10485, SEQ ID NO: 10487, SEQ ID NO: 10488, SEQ ID
NO: 10489, SEQ ID NO: 10491, SEQ ID NO: 10493, SEQ ID NO: 10495, SEQ ID NO: 10496,
SEQ ID NO: 10497, SEQ ID NO: 10498, SEQ ID NO: 10500, SEQ ID NO: 10502, SEQ ID
NO: 10503, SEQ ID NO: 10504, SEQ ID NO: 10505, SEQ ID NO: 10507, SEQ ID NO: 10508,
SEQ ID NO: 10510, SEQ ID NO: 10511, SEQ ID NO: 10512, SEQ ID NO: 10514, SEQ ID
NO: 10516, SEQ ID NO: 10517, SEQ ID NO: 10519, SEQ ID NO: 10521, SEQ ID NO: 10523,
SEQ ID NO: 10524, SEQ ID NO: 10526, SEQ ID NO: 10528, SEQ ID NO: 10529, SEQ ID
NO: 10530, SEQ ID NO: 10532, SEQ ID NO: 10534, SEQ ID NO: 10535, SEQ ID NO: 10537,
SEQ ID NO: 10539, SEQ ID NO: 10540, SEQ ID NO: 10542, SEQ ID NO: 10543, SEQ ID
NO: 10545, SEQ ID NO: 10546, SEQ ID NO: 10548, SEQ ID NO: 10550, SEQ ID NO: 10551,
SEQ ID NO: 10553, SEQ ID NO: 10555, SEQ ID NO: 10556, SEQ ID NO: 10557, SEQ ID
NO: 10558, SEQ ID NO: 10560, SEQ ID NO: 10562, SEQ ID NO: 10564, SEQ ID NO: 10566,
SEQ ID NO: 10567, SEQ ID NO: 10569, SEQ ID NO: 10571, SEQ ID NO: 10573, SEQ ID
NO: 10574, SEQ ID NO: 10576, SEQ ID NO: 10578, SEQ ID NO: 10580, SEQ ID NO: 10582,
SEQ ID NO: 10584, SEQ ID NO: 10586, SEQ ID NO: 10588, SEQ ID NO: 10590, SEQ ID
NO: 10592, SEQ ID NO: 10594, SEQ ID NO: 10596, SEQ ID NO: 10597, SEQ ID NO: 10599,
SEQ ID NO: 10601, SEQ ID NO: 10603, SEQ ID NO: 10604, SEQ ID NO: 10606, SEQ ID
NO: 10607, SEQ ID NO: 10609, SEQ ID NO: 10611, SEQ ID NO: 10613, SEQ ID NO: 10614,
SEQ ID NO: 10615, SEQ ID NO: 10616, SEQ ID NO: 10618, SEQ ID NO: 10619, SEQ ID
NO: 10620, SEQ ID NO: 10622, SEQ ID NO: 10624, SEQ ID NO: 10625, SEQ ID NO: 10627,
SEQ ID NO: 10629,
SEQ ID NO: 10630, SEQ ID NO: 10632, SEQ ID NO: 10633, SEQ ID NO: 10635, SEQ ID
NO: 10637, SEQ ID NO: 10639, SEQ ID NO: 10641, SEQ ID NO: 10642, SEQ ID NO: 10644,
SEQ ID NO: 10646, SEQ ID NO: 10647, SEQ ID NO: 10648, SEQ ID NO: 10649, SEQ ID
NO: 10650, SEQ ID NO: 10652, SEQ ID NO: 10654, SEQ ID NO: 10655, SEQ ID NO: 10656,
SEQ ID NO: 10658, SEQ ID NO: 10659, SEQ ID NO: 10661, SEQ ID NO: 10663, SEQ ID
NO: 10665, SEQ ID NO: 10667, SEQ ID NO: 10669, SEQ ID NO: 10670, SEQ ID NO: 10671,
SEQ ID NO: 10673, SEQ ID NO: 10674, SEQ ID NO: 10676, SEQ ID NO: 10678, SEQ ID
NO: 10680, SEQ ID NO: 10682, SEQ ID NO: 10683, SEQ ID NO: 10685, SEQ ID NO: 10687,
SEQ ID NO: 10689, SEQ ID NO: 10691, SEQ ID NO: 10693, SEQ ID NO: 10695, SEQ ID
NO: 10696, SEQ ID NO: 10698, SEQ ID NO: 10700, SEQ ID NO: 10702, SEQ ID NO: 10704,
SEQ ID NO: 10706, SEQ ID NO: 10708, SEQ ID NO: 10710, SEQ ID NO: 10711, SEQ ID
NO: 10713, SEQ ID NO: 10715, SEQ ID NO: 10717, SEQ ID NO: 10718, SEQ ID NO: 10720,
SEQ ID NO: 10722, SEQ ID NO: 10723, SEQ ID NO: 10725, SEQ ID NO: 10727, SEQ ID
NO: 10728, SEQ ID NO: 10730, SEQ ID NO: 10732, SEQ ID NO: 10734, SEQ ID NO: 10736,
SEQ ID NO: 10738, SEQ ID NO: 10740, SEQ ID NO: 10742, SEQ ID NO: 10744, SEQ ID
NO: 10746, SEQ ID NO: 10748, SEQ ID NO: 10750, SEQ ID NO: 10752, SEQ ID NO: 10753,
SEQ ID NO: 10754, SEQ ID NO: 10756, SEQ ID NO: 10757, SEQ ID NO: 10758, SEQ ID
NO: 10760, SEQ ID NO: 10761, SEQ ID NO: 10763, SEQ ID NO: 10765, SEQ ID NO: 10767,
SEQ ID NO: 10769, SEQ ID NO: 10771, SEQ ID NO: 10773, SEQ ID NO: 10774, SEQ ID
NO: 10776, SEQ ID NO: 10778, SEQ ID NO: 10780, SEQ ID NO: 10781, SEQ ID NO: 10783,
 
SEQ ID NO: 10785, SEQ ID NO: 10786, SEQ ID NO: 10788, SEQ ID NO: 10790, SEQ ID
NO: 10792, SEQ ID NO: 10793, SEQ ID NO: 10795, SEQ ID NO: 10797, SEQ ID NO: 10799,
SEQ ID NO: 10800,
SEQ ID NO: 10802, SEQ ID NO: 10804, SEQ ID NO: 10805, SEQ ID NO: 10807, SEQ ID
NO: 10808, SEQ ID NO: 10810, SEQ ID NO: 10812, SEQ ID NO: 10814, SEQ ID NO: 10815,
SEQ ID NO: 10817, SEQ ID NO: 10818, SEQ ID NO: 10820, SEQ ID NO: 10822, SEQ ID
NO: 10824, SEQ ID NO: 10826, SEQ ID NO: 10828, SEQ ID NO: 10830, SEQ ID NO: 10831,
SEQ ID NO: 10832, SEQ ID NO: 10834, SEQ ID NO: 10836, SEQ ID NO: 10838, SEQ ID
NO: 10840, SEQ ID NO: 10841, SEQ ID NO: 10842, SEQ ID NO: 10844, SEQ ID NO: 10845,
SEQ ID NO: 10847, SEQ ID NO: 10849, SEQ ID NO: 10850, SEQ ID NO: 10852, SEQ ID
NO: 10854, SEQ ID NO: 10856, SEQ ID NO: 10858, SEQ ID NO: 10859, SEQ ID NO: 10861,
SEQ ID NO: 10862, SEQ ID NO: 10864, SEQ ID NO: 10866, SEQ ID NO: 10868, SEQ ID
NO: 10870, SEQ ID NO: 10872, SEQ ID NO: 10873, SEQ ID NO: 10875, SEQ ID NO: 10876,
SEQ ID NO: 10878, SEQ ID NO: 10879, SEQ ID NO: 10881, SEQ ID NO: 10883, SEQ ID
NO: 10885, SEQ ID NO: 10886, SEQ ID NO: 10888, SEQ ID NO: 10889, SEQ ID NO: 10890,
SEQ ID NO: 10891, SEQ ID NO: 10893, SEQ ID NO: 10895, SEQ ID NO: 10897, SEQ ID
NO: 10898, SEQ ID NO: 10900, SEQ ID NO: 10901, SEQ ID NO: 10902, SEQ ID NO: 10904,
SEQ ID NO: 10906, SEQ ID NO: 10908, SEQ ID NO: 10909, SEQ ID NO: 10910, SEQ ID
NO: 10912, SEQ ID NO: 10913, SEQ ID NO: 10914, SEQ ID NO: 10915, SEQ ID NO: 10916,
SEQ ID NO: 10918, SEQ ID NO: 10920, SEQ ID NO: 10922, SEQ ID NO: 10924, SEQ ID
NO: 10925, SEQ ID NO: 10927, SEQ ID NO: 10928, SEQ ID NO: 10929, SEQ ID NO: 10931,
SEQ ID NO: 10933, SEQ ID NO: 10935, SEQ ID NO: 10937, SEQ ID NO: 10939, SEQ ID
NO: 10940, SEQ ID NO: 10942, SEQ ID NO: 10943, SEQ ID NO: 10944, SEQ ID NO: 10945,
SEQ ID NO: 10946, SEQ ID NO: 10948, SEQ ID NO: 10950, SEQ ID NO: 10952, SEQ ID
NO: 10954, SEQ ID NO: 10956, SEQ ID NO: 10957, SEQ ID NO: 10958, SEQ ID NO: 10959,
SEQ ID NO: 10960,
SEQ ID NO: 10962, SEQ ID NO: 10964, SEQ ID NO: 10966, SEQ ID NO: 10967, SEQ ID
NO: 10968, SEQ ID NO: 10969, SEQ ID NO: 10971, SEQ ID NO: 10973, SEQ ID NO: 10975,
SEQ ID NO: 10977, SEQ ID NO: 10978, SEQ ID NO: 10980, SEQ ID NO: 10982, SEQ ID
NO: 10983, SEQ ID NO: 10984, SEQ ID NO: 10986, SEQ ID NO: 10988, SEQ ID NO: 10990,
SEQ ID NO: 10992, SEQ ID NO: 10994, SEQ ID NO: 10996, SEQ ID NO: 10998, SEQ ID
NO: 11000, SEQ ID NO: 11001, SEQ ID NO: 11003, SEQ ID NO: 11005, SEQ ID NO: 11006,
SEQ ID NO: 11007, SEQ ID NO: 11009, SEQ ID NO: 11011, SEQ ID NO: 11012, SEQ ID
NO: 11014, SEQ ID NO: 11016, SEQ ID NO: 11018, SEQ ID NO: 11019, SEQ ID NO: 11021,
SEQ ID NO: 11023, SEQ ID NO: 11025, SEQ ID NO: 11027, SEQ ID NO: 11029, SEQ ID
NO: 11030, SEQ ID NO: 11032, SEQ ID NO: 11034, SEQ ID NO: 11035, SEQ ID NO: 11036,
SEQ ID NO: 11038, SEQ ID NO: 11040, SEQ ID NO: 11042, SEQ ID NO: 11044, SEQ ID
NO: 11046, SEQ ID NO: 11048, SEQ ID NO: 11050, SEQ ID NO: 11052, SEQ ID NO: 11054,
SEQ ID NO: 11056, SEQ ID NO: 11058, SEQ ID NO: 11060, SEQ ID NO: 11062, SEQ ID
NO: 11064, SEQ ID NO: 11066, SEQ ID NO: 11068, SEQ ID NO: 11070, SEQ ID NO: 11072,
SEQ ID NO: 11074, SEQ ID NO: 11076, SEQ ID NO: 11078, SEQ ID NO: 11080, SEQ ID
NO: 11082, SEQ ID NO: 11084, SEQ ID NO: 11086, SEQ ID NO: 11088, SEQ ID NO: 11090,
SEQ ID NO: 11092, SEQ ID NO: 11093, SEQ ID NO: 11095, SEQ ID NO: 11097, SEQ ID
NO: 11099, SEQ ID NO: 11101, SEQ ID NO: 11103, SEQ ID NO: 11105, SEQ ID NO: 11107,
SEQ ID NO: 11108, SEQ ID NO: 11110, SEQ ID NO: 11112, SEQ ID NO: 11114, SEQ ID
NO: 11116, SEQ ID NO: 11118, SEQ ID NO: 11120, SEQ ID NO: 11122, SEQ ID NO: 11123,
SEQ ID NO: 11125, SEQ ID NO: 11127, SEQ ID NO: 11129, SEQ ID NO: 11131, SEQ ID
NO: 11133, SEQ ID NO: 11135, SEQ ID NO: 11137, SEQ ID NO: 11139, SEQ ID NO: 11141,
SEQ ID NO: 11143,
SEQ ID NO: 11145, SEQ ID NO: 11147, SEQ ID NO: 11148, SEQ ID NO: 11150, SEQ ID
NO: 11152, SEQ ID NO: 11154, SEQ ID NO: 11156, SEQ ID NO: 11158, SEQ ID NO: 11160,
SEQ ID NO: 11162, SEQ ID NO: 11164, SEQ ID NO: 11165, SEQ ID NO: 11167, SEQ ID
 
NO: 11168, SEQ ID NO: 11170, SEQ ID NO: 11172, SEQ ID NO: 11174, SEQ ID NO: 11176,
SEQ ID NO: 11178, SEQ ID NO: 11179, SEQ ID NO: 11181, SEQ ID NO: 11183, SEQ ID
NO: 11185, SEQ ID NO: 11187, SEQ ID NO: 11189, SEQ ID NO: 11191, SEQ ID NO: 11193,
SEQ ID NO: 11195, SEQ ID NO: 11197, SEQ ID NO: 11199, SEQ ID NO: 11201, SEQ ID
NO: 11203, SEQ ID NO: 11205, SEQ ID NO: 11207, SEQ ID NO: 11209, SEQ ID NO: 11211,
SEQ ID NO: 11213, SEQ ID NO: 11215, SEQ ID NO: 11217, SEQ ID NO: 11219, SEQ ID
NO: 11221, SEQ ID NO: 11223, SEQ ID NO: 11225, SEQ ID NO: 11227, SEQ ID NO: 11229,
SEQ ID NO: 11231, SEQ ID NO: 11233, SEQ ID NO: 11235, SEQ ID NO: 11237, SEQ ID
NO: 11239, SEQ ID NO: 11241, SEQ ID NO: 11243, SEQ ID NO: 11244, SEQ ID NO: 11246,
SEQ ID NO: 11248, SEQ ID NO: 11250, SEQ ID NO: 11252, SEQ ID NO: 11253, SEQ ID
NO: 11255, SEQ ID NO: 11257, SEQ ID NO: 11259, SEQ ID NO: 11261, SEQ ID NO: 11263,
SEQ ID NO: 11265, SEQ ID NO: 11267, SEQ ID NO: 11269, SEQ ID NO: 11271, SEQ ID
NO: 11273, SEQ ID NO: 11275, SEQ ID NO: 11277, SEQ ID NO: 11279, SEQ ID NO: 11281,
SEQ ID NO: 11283, SEQ ID NO: 11285, SEQ ID NO: 11287, SEQ ID NO: 11289, SEQ ID
NO: 11291, SEQ ID NO: 11293, SEQ ID NO: 11295, SEQ ID NO: 11297, SEQ ID NO: 11299,
SEQ ID NO: 11301, SEQ ID NO: 11303, SEQ ID NO: 11304, SEQ ID NO: 11306, SEQ ID
NO: 11308, SEQ ID NO: 11310, SEQ ID NO: 11312, SEQ ID NO: 11314, SEQ ID NO: 11316,
SEQ ID NO: 11318, SEQ ID NO: 11319, SEQ ID NO: 11321, SEQ ID NO: 11323, SEQ ID
NO: 11325, SEQ ID NO: 11327, SEQ ID NO: 11329, SEQ ID NO: 11331, SEQ ID NO: 11333,
SEQ ID NO: 11335,
SEQ ID NO: 11337, SEQ ID NO: 11339, SEQ ID NO: 11341, SEQ ID NO: 11343, SEQ ID
NO: 11345, SEQ ID NO: 11347, SEQ ID NO: 11349; SEQ ID NO: 11351, SEQ ID NO: 11352,
SEQ ID NO: 11354, SEQ ID NO: 11356, SEQ ID NO: 11358, SEQ ID NO: 11360, SEQ ID
NO: 11362, SEQ ID NO: 11364, SEQ ID NO: 11366, SEQ ID NO: 11368, SEQ ID NO: 11369,
SEQ ID NO: 11370, SEQ ID NO: 11372, SEQ ID NO: 11374, SEQ ID NO: 11376, SEQ ID
NO: 11378, SEQ ID NO: 11380, SEQ ID NO: 11382, SEQ ID NO: 11384, SEQ ID NO: 11386,
SEQ ID NO: 11388, SEQ ID NO: 11390, SEQ ID NO: 11392, SEQ ID NO: 11394, SEQ ID
NO: 11396, SEQ ID NO: 11397, SEQ ID NO: 11399, SEQ ID NO: 11401, SEQ ID NO: 11403,
SEQ ID NO: 11405, SEQ ID NO: 11407, SEQ ID NO: 11409, SEQ ID NO: 11411, SEQ ID
NO: 11413, SEQ ID NO: 11415, SEQ ID NO: 11416, SEQ ID NO: 11418, SEQ ID NO: 11419,
SEQ ID NO: 11421, SEQ ID NO: 11423, SEQ ID NO: 11425, SEQ ID NO: 11427, SEQ ID
NO: 11429, SEQ ID NO: 11430, SEQ ID NO: 11432, SEQ ID NO: 11434, SEQ ID NO: 11436,
SEQ ID NO: 11437, SEQ ID NO: 11439, SEQ ID NO: 11441, SEQ ID NO: 11443, SEQ ID
NO: 11445, SEQ ID NO: 11447, SEQ ID NO: 11449, SEQ ID NO: 11451, SEQ ID NO: 11453,
SEQ ID NO: 11455, SEQ ID NO: 11457, SEQ ID NO: 11459, SEQ ID NO: 11461, SEQ ID
NO: 11463, SEQ ID NO: 11465, SEQ ID NO: 11467, SEQ ID NO: 11469, SEQ ID NO: 11470,
SEQ ID NO: 11472, SEQ ID NO: 11474, SEQ ID NO: 11476, SEQ ID NO: 11478, SEQ ID
NO: 11480, SEQ ID NO: 11482, SEQ ID NO: 11484, SEQ ID NO: 11485, SEQ ID NO: 11487,
SEQ ID NO: 11489, SEQ ID NO: 11491, SEQ ID NO: 11493, SEQ ID NO: 11495, SEQ ID
NO: 11496, SEQ ID NO: 11498, SEQ ID NO: 11500, SEQ ID NO: 11502, SEQ ID NO: 11504,
SEQ ID NO: 11506, SEQ ID NO: 11507, SEQ ID NO: 11509, SEQ ID NO: 11511, SEQ ID
NO: 11513, SEQ ID NO: 11515, SEQ ID NO: 11517, SEQ ID NO: 11519, SEQ ID NO: 11521,
SEQ ID NO: 11523,
SEQ ID NO: 11524, SEQ ID NO: 11526, SEQ ID NO: 11528, SEQ ID NO: 11530, SEQ ID
NO: 11532, SEQ ID NO: 11534, SEQ ID NO: 11536, SEQ ID NO: 11538, SEQ ID NO: 11540,
SEQ ID NO: 11541, SEQ ID NO: 11543, SEQ ID NO: 11545, SEQ ID NO: 11547, SEQ ID
NO: 11549, SEQ ID NO: 11551, SEQ ID NO: 11553, SEQ ID NO: 11555, SEQ ID NO: 11557,
SEQ ID NO: 11559, SEQ ID NO: 11560, SEQ ID NO: 11561, SEQ ID NO: 11562, SEQ ID
NO: 11564, SEQ ID NO: 11566, SEQ ID NO: 11568, SEQ ID NO: 11569, SEQ ID NO: 11571,
SEQ ID NO: 11573, SEQ ID NO: 11575, SEQ ID NO: 11577, SEQ ID NO: 11579, SEQ ID
NO: 11581, SEQ ID NO: 11582, SEQ ID NO: 11584, SEQ ID NO: 11586, SEQ ID NO: 11588,
SEQ ID NO: 11590, SEQ ID NO: 11592, SEQ ID NO: 11594, SEQ ID NO: 11596, SEQ ID
 
NO: 11598, SEQ ID NO: 11600, SEQ ID NO: 11602, SEQ ID NO: 11604, SEQ ID NO: 11606,
SEQ ID NO: 11608, SEQ ID NO: 11609, SEQ ID NO: 11611, SEQ ID NO: 11613, SEQ ID
NO: 11615, SEQ ID NO: 11617, SEQ ID NO: 11619, SEQ ID NO: 11620, SEQ ID NO: 11622,
SEQ ID NO: 11624, SEQ ID NO: 11626, SEQ ID NO: 11628, SEQ ID NO: 11630, SEQ ID
NO: 11632, SEQ ID NO: 11634, SEQ ID NO: 11636, SEQ ID NO: 11638, SEQ ID NO: 11639,
SEQ ID NO: 11641, SEQ ID NO: 11643, SEQ ID NO: 11645, SEQ ID NO: 11647, SEQ ID
NO: 11649, SEQ ID NO: 11651, SEQ ID NO: 11653, SEQ ID NO: 11654, SEQ ID NO: 11656,
SEQ ID NO: 11658, SEQ ID NO: 11659, SEQ ID NO: 11661, SEQ ID NO: 11663, SEQ ID
NO: 11665, SEQ ID NO: 11666, SEQ ID NO: 11668, SEQ ID NO: 11670, SEQ ID NO: 11672,
SEQ ID NO: 11674, SEQ ID NO: 11675, SEQ ID NO: 11677, SEQ ID NO: 11678, SEQ ID
NO: 11680, SEQ ID NO: 11682, SEQ ID NO: 11684, SEQ ID NO: 11685, SEQ ID NO: 11687,
SEQ ID NO: 11689, SEQ ID NO: 11691, SEQ ID NO: 11693, SEQ ID NO: 11695, SEQ ID
NO: 11697, SEQ ID NO: 11699, SEQ ID NO: 11701, SEQ ID NO: 11703, SEQ ID NO: 11705,
SEQ ID NO: 11707,
SEQ ID NO: 11709, SEQ ID NO: 11711, SEQ ID NO: 11713, SEQ ID NO: 11715, SEQ ID
NO: 11717, SEQ ID NO: 11718, SEQ ID NO: 11720, SEQ ID NO: 11722, SEQ ID NO: 11724,
SEQ ID NO: 11726, SEQ ID NO: 11728, SEQ ID NO: 11730, SEQ ID NO: 11732, SEQ ID
NO: 11734, SEQ ID NO: 11736, SEQ ID NO: 11738, SEQ ID NO: 11739, SEQ ID NO: 11740,
SEQ ID NO: 11742, SEQ ID NO: 11744, SEQ ID NO: 11746, SEQ ID NO: 11748, SEQ ID
NO: 11750, SEQ ID NO: 11752, SEQ ID NO: 11754, SEQ ID NO: 11756, SEQ ID NO: 11758,
SEQ ID NO: 11759, SEQ ID NO: 11761, SEQ ID NO: 11763, SEQ ID NO: 11765, SEQ ID
NO: 11767, SEQ ID NO: 11768, SEQ ID NO: 11770, SEQ ID NO: 11772, SEQ ID NO: 11774,
SEQ ID NO: 11776, SEQ ID NO: 11778, SEQ ID NO: 11780, SEQ ID NO: 11782, SEQ ID
NO: 11784, SEQ ID NO: 11786, SEQ ID NO: 11788, SEQ ID NO: 11790, SEQ ID NO: 11792,
SEQ ID NO: 11794, SEQ ID NO: 11796, SEQ ID NO: 11798, SEQ ID NO: 11800, SEQ ID
NO: 11802, SEQ ID NO: 11804, SEQ ID NO: 11806, SEQ ID NO: 11808, SEQ ID NO: 11810,
SEQ ID NO: 11811, SEQ ID NO: 11813, SEQ ID NO: 11815, SEQ ID NO: 11817, SEQ ID
NO: 11819, SEQ ID NO: 11820, SEQ ID NO: 11822, SEQ ID NO: 11824, SEQ ID NO: 11826,
SEQ ID NO: 11828, SEQ ID NO: 11830, SEQ ID NO: 11832, SEQ ID NO: 11833, SEQ ID
NO: 11835, SEQ ID NO: 11837, SEQ ID NO: 11839, SEQ ID NO: 11841, SEQ ID NO: 11843,
SEQ ID NO: 11845, SEQ ID NO: 11847, SEQ ID NO: 11849, SEQ ID NO: 11850, SEQ ID
NO: 11851, SEQ ID NO: 11853, SEQ ID NO: 11855, SEQ ID NO: 11856, SEQ ID NO: 11858,
SEQ ID NO: 11859, SEQ ID NO: 11861, SEQ ID NO: 11863, SEQ ID NO: 11865, SEQ ID
NO: 11867, SEQ ID NO: 11869, SEQ ID NO: 11870, SEQ ID NO: 11871, SEQ ID NO: 11873,
SEQ ID NO: 11875, SEQ ID NO: 11877, SEQ ID NO: 11879, SEQ ID NO: 11880, SEQ ID
NO: 11882, SEQ ID NO: 11884, SEQ ID NO: 11886, SEQ ID NO: 11888, SEQ ID NO: 11890,
SEQ ID NO: 11892,
SEQ ID NO: 11893, SEQ ID NO: 11895, SEQ ID NO: 11897, SEQ ID NO: 11899, SEQ ID
NO: 11901, SEQ ID NO: 11903, SEQ ID NO: 11905, SEQ ID NO: 11907, SEQ ID NO: 11909,
SEQ ID NO: 11911, SEQ ID NO: 11913, SEQ ID NO: 11915, SEQ ID NO: 11916, SEQ ID
NO: 11918, SEQ ID NO: 11919, SEQ ID NO: 11921, SEQ ID NO: 11923, SEQ ID NO: 11925,
SEQ ID NO: 11927, SEQ ID NO: 11928, SEQ ID NO: 11930, SEQ ID NO: 11932, SEQ ID
NO: 11934, SEQ ID NO: 11936, SEQ ID NO: 11938, SEQ ID NO: 11940, SEQ ID NO: 11942,
SEQ ID NO: 11944, SEQ ID NO: 11946, SEQ ID NO: 11948, SEQ ID NO: 11949, SEQ ID
NO: 11951, SEQ ID NO: 11953, SEQ ID NO: 11955, SEQ ID NO: 11957, SEQ ID NO: 11959,
SEQ ID NO: 11961, SEQ ID NO: 11963, SEQ ID NO: 11965, SEQ ID NO: 11967, SEQ ID
NO: 11969, SEQ ID NO: 11971, SEQ ID NO: 11973, SEQ ID NO: 11975, SEQ ID NO: 11977,
SEQ ID NO: 11978, SEQ ID NO: 11980, SEQ ID NO: 11982, SEQ ID NO: 11984, SEQ ID
NO: 11986, SEQ ID NO: 11988, SEQ ID NO: 11990, SEQ ID NO: 11992, SEQ ID NO: 11994,
SEQ ID NO: 11996, SEQ ID NO: 11998, SEQ ID NO: 12000, SEQ ID NO: 12002, SEQ ID
NO: 12003, SEQ ID NO: 12005, SEQ ID NO: 12007, SEQ ID NO: 12008, SEQ ID NO: 12010,
SEQ ID NO: 12012, SEQ ID NO: 12014, SEQ ID NO: 12016, SEQ ID NO: 12018, SEQ ID
 
NO: 12019, SEQ ID NO: 12020, SEQ ID NO: 12022, SEQ ID NO: 12024, SEQ ID NO: 12026,
SEQ ID NO: 12028, SEQ ID NO: 12030, SEQ ID NO: 12032, SEQ ID NO: 12034, SEQ ID
NO: 12036, SEQ ID NO: 12038, SEQ ID NO: 12040, SEQ ID NO: 12042, SEQ ID NO: 12044,
SEQ ID NO: 12046, SEQ ID NO: 12048, SEQ ID NO: 12049, SEQ ID NO: 12051, SEQ ID
NO: 12053, SEQ ID NO: 12055, SEQ ID NO: 12056, SEQ ID NO: 12058, SEQ ID NO: 12060,
SEQ ID NO: 12062, SEQ ID NO: 12064, SEQ ID NO: 12066, SEQ ID NO: 12068, SEQ ID
NO: 12070, SEQ ID NO: 12072, SEQ ID NO: 12074, SEQ ID NO: 12076, SEQ ID NO: 12078,
SEQ ID NO: 12080,
SEQ ID NO: 12082, SEQ ID NO: 12084, SEQ ID NO: 12086, SEQ ID NO: 12088, SEQ ID
NO: 12090, SEQ ID NO: 12092, SEQ ID NO: 12094, SEQ ID NO: 12096, SEQ ID NO: 12097,
SEQ ID NO: 12099, SEQ ID NO: 12101, SEQ ID NO: 12103, SEQ ID NO: 12105, SEQ ID
NO: 12107, SEQ ID NO: 12109, SEQ ID NO: 12111, SEQ ID NO: 12113, SEQ ID NO: 12115,
SEQ ID NO: 12117, SEQ ID NO: 12119, SEQ ID NO: 12121, SEQ ID NO: 12123, SEQ ID
NO: 12125, SEQ ID NO: 12127, SEQ ID NO: 12129, SEQ ID NO: 12131, SEQ ID NO: 12133,
SEQ ID NO: 12135, SEQ ID NO: 12137, SEQ ID NO: 12139, SEQ ID NO: 12141, SEQ ID
NO: 12143, SEQ ID NO: 12145, SEQ ID NO: 12147, SEQ ID NO: 12149, SEQ ID NO: 12151,
SEQ ID NO: 12153, SEQ ID NO: 12155, SEQ ID NO: 12157, SEQ ID NO: 12159, SEQ ID
NO: 12161, SEQ ID NO: 12163, SEQ ID NO: 12165, SEQ ID NO: 12167, SEQ ID NO: 12169,
SEQ ID NO: 12171, SEQ ID NO: 12173, SEQ ID NO: 12175, SEQ ID NO: 12177, SEQ ID
NO: 12179, SEQ ID NO: 12181, SEQ ID NO: 12183, SEQ ID NO: 12185, SEQ ID NO: 12187,
SEQ ID NO: 12189, SEQ ID NO: 12191, SEQ ID NO: 12193, SEQ ID NO: 12195, SEQ ID
NO: 12197, SEQ ID NO: 12199, SEQ ID NO: 12201, SEQ ID NO: 12203, SEQ ID NO: 12205,
SEQ ID NO: 12207, SEQ ID NO: 12209, SEQ ID NO: 12211, SEQ ID NO: 12213, SEQ ID
NO: 12215, SEQ ID NO: 12216, SEQ ID NO: 12218, SEQ ID NO: 12220, SEQ ID NO: 12222,
SEQ ID NO: 12224, SEQ ID NO: 12226, SEQ ID NO: 12228, SEQ ID NO: 12230, SEQ ID
NO: 12232, SEQ ID NO: 12234, SEQ ID NO: 12236, SEQ ID NO: 12238, SEQ ID NO: 12240,
SEQ ID NO: 12242, SEQ ID NO: 12244, SEQ ID NO: 12246, SEQ ID NO: 12248, SEQ ID
NO: 12250, SEQ ID NO: 12252, SEQ ID NO: 12254, SEQ ID NO: 12256, SEQ ID NO: 12258,
SEQ ID NO: 12260, SEQ ID NO: 12262, SEQ ID NO: 12264, SEQ ID NO: 12266, SEQ ID
NO: 12268, SEQ ID NO: 12270, SEQ ID NO: 12272, SEQ ID NO: 12274, SEQ ID NO: 12276,
SEQ ID NO: 12278,
SEQ ID NO: 12279, SEQ ID NO: 12281, SEQ ID NO: 12283, SEQ ID NO: 12285, SEQ ID
NO: 12287, SEQ ID NO: 12289, SEQ ID NO: 12291, SEQ ID NO: 12293, SEQ ID NO: 12295,
SEQ ID NO: 12297, SEQ ID NO: 12299, SEQ ID NO: 12301, SEQ ID NO: 12303, SEQ ID
NO: 12305, SEQ ID NO: 12307, SEQ ID NO: 12309, SEQ ID NO: 12311, SEQ ID NO: 12313,
SEQ ID NO: 12315, SEQ ID NO: 12317, SEQ ID NO: 12319, SEQ ID NO: 12321, SEQ ID
NO: 12323, SEQ ID NO: 12325, SEQ ID NO: 12327, SEQ ID NO: 12329, SEQ ID NO: 12331,
SEQ ID NO: 12333, SEQ ID NO: 12335, SEQ ID NO: 12337, SEQ ID NO: 12339, SEQ ID
NO: 12341, SEQ ID NO: 12343, SEQ ID NO: 12345, SEQ ID NO: 12347, SEQ ID NO: 12349,
SEQ ID NO: 12351, SEQ ID NO: 12353, SEQ ID NO: 12354, SEQ ID NO: 12356, SEQ ID
NO: 12358, SEQ ID NO: 12360, SEQ ID NO: 12362, SEQ ID NO: 12364, SEQ ID NO: 12366,
SEQ ID NO: 12368, SEQ ID NO: 12370, SEQ ID NO: 12372, SEQ ID NO: 12374, SEQ ID
NO: 12376, SEQ ID NO: 12378, SEQ ID NO: 12380, SEQ ID NO: 12382, SEQ ID NO: 12384,
SEQ ID NO: 12386, SEQ ID NO: 12388, SEQ ID NO: 12390, SEQ ID NO: 12392, SEQ ID
NO: 12394, SEQ ID NO: 12396, SEQ ID NO: 12398, SEQ ID NO: 12400, SEQ ID NO: 12402,
SEQ ID NO: 12403, SEQ ID NO: 12405, SEQ ID NO: 12407, SEQ ID NO: 12409, SEQ ID
NO: 12410, SEQ ID NO: 12412, SEQ ID NO: 12414, SEQ ID NO: 12416, SEQ ID NO: 12417,
SEQ ID NO: 12419, SEQ ID NO: 12421, SEQ ID NO: 12423, SEQ ID NO: 12425, SEQ ID
NO: 12427, SEQ ID NO: 12429, SEQ ID NO: 12431, SEQ ID NO: 12433, SEQ ID NO: 12435,
SEQ ID NO: 12436, SEQ ID NO: 12438, SEQ ID NO: 12440, SEQ ID NO: 12442, SEQ ID
NO: 12444, SEQ ID NO: 12446, SEQ ID NO: 12448, SEQ ID NO: 12450, SEQ ID NO: 12452,
SEQ ID NO: 12454, SEQ ID NO: 12456, SEQ ID NO: 12458, SEQ ID NO: 12460, SEQ ID
 
NO: 12461, SEQ ID NO: 12463, SEQ ID NO: 12465, SEQ ID NO: 12467, SEQ ID NO: 12469,
SEQ ID NO: 12471,
SEQ ID NO: 12473, SEQ ID NO: 12475, SEQ ID NO: 12477, SEQ ID NO: 12479, SEQ ID
NO: 12481, SEQ ID NO: 12482, SEQ ID NO: 12483, SEQ ID NO: 12485, SEQ ID NO: 12486,
SEQ ID NO: 12488, SEQ ID NO: 12490, SEQ ID NO: 12491, SEQ ID NO: 12493, SEQ ID
NO: 12495, SEQ ID NO: 12496, SEQ ID NO: 12498, SEQ ID NO: 12500, SEQ ID NO: 12502,
SEQ ID NO: 12503, SEQ ID NO: 12505, SEQ ID NO: 12507, SEQ ID NO: 12509, SEQ ID
NO: 12511, SEQ ID NO: 12513, SEQ ID NO: 12514, SEQ ID NO: 12516, SEQ ID NO: 12518,
SEQ ID NO: 12520, SEQ ID NO: 12522, SEQ ID NO: 12524, SEQ ID NO: 12526, SEQ ID
NO: 12528, SEQ ID NO: 12529, SEQ ID NO: 12531, SEQ ID NO: 12533, SEQ ID NO: 12534,
SEQ ID NO: 12535, SEQ ID NO: 12537, SEQ ID NO: 12539, SEQ ID NO: 12541, SEQ ID
NO: 12543, SEQ ID NO: 12545, SEQ ID NO: 12546, SEQ ID NO: 12548, SEQ ID NO: 12550,
SEQ ID NO: 12552, SEQ ID NO: 12554, SEQ ID NO: 12555, SEQ ID NO: 12556, SEQ ID
NO: 12557, SEQ ID NO: 12559, SEQ ID NO: 12561, SEQ ID NO: 12563, SEQ ID NO: 12565,
SEQ ID NO: 12567, SEQ ID NO: 12569, SEQ ID NO: 12571, SEQ ID NO: 12572, SEQ ID
NO: 12574, SEQ ID NO: 12576, SEQ ID NO: 12578, SEQ ID NO: 12580, SEQ ID NO: 12581,
SEQ ID NO: 12583, SEQ ID NO: 12585, SEQ ID NO: 12587, SEQ ID NO: 12589, SEQ ID
NO: 12591, SEQ ID NO: 12592, SEQ ID NO: 12593, SEQ ID NO: 12595, SEQ ID NO: 12597,
SEQ ID NO: 12599, SEQ ID NO: 12601, SEQ ID NO: 12603, SEQ ID NO: 12605, SEQ ID
NO: 12607, SEQ ID NO: 12608, SEQ ID NO: 12609, SEQ ID NO: 12611, SEQ ID NO: 12613,
SEQ ID NO: 12615, SEQ ID NO: 12617, SEQ ID NO: 12619, SEQ ID NO: 12621, SEQ ID
NO: 12622, SEQ ID NO: 12623, SEQ ID NO: 12625, SEQ ID NO: 12627, SEQ ID NO: 12628,
SEQ ID NO: 12630, SEQ ID NO: 12632, SEQ ID NO: 12634, SEQ ID NO: 12636, SEQ ID
NO: 12638, SEQ ID NO: 12640, SEQ ID NO: 12642, SEQ ID NO: 12644, SEQ ID NO: 12646,
SEQ ID NO: 12648,
SEQ ID NO: 12649, SEQ ID NO: 12651, SEQ ID NO: 12653, SEQ ID NO: 12655, SEQ ID
NO: 12657, SEQ ID NO: 12659, SEQ ID NO: 12660, SEQ ID NO: 12662, SEQ ID NO: 12664,
SEQ ID NO: 12665, SEQ ID NO: 12667, SEQ ID NO: 12669, SEQ ID NO: 12671, SEQ ID
NO: 12673, SEQ ID NO: 12675, SEQ ID NO: 12677, SEQ ID NO: 12679, SEQ ID NO: 12681,
SEQ ID NO: 12683, SEQ ID NO: 12685, SEQ ID NO: 12687, SEQ ID NO: 12689, SEQ ID
NO: 12691, SEQ ID NO: 12693, SEQ ID NO: 12695, SEQ ID NO: 12697, SEQ ID NO: 12698,
SEQ ID NO: 12700, SEQ ID NO: 12702, SEQ ID NO: 12703, SEQ ID NO: 12705, SEQ ID
NO: 12707, SEQ ID NO: 12709, SEQ ID NO: 12710, SEQ ID NO: 12711, SEQ ID NO: 12713,
SEQ ID NO: 12715, SEQ ID NO: 12717, SEQ ID NO: 12719, SEQ ID NO: 12721, SEQ ID
NO: 12723, SEQ ID NO: 12725, SEQ ID NO: 12726, SEQ ID NO: 12728, SEQ ID NO: 12730,
SEQ ID NO: 12731, SEQ ID NO: 12733, SEQ ID NO: 12735, SEQ ID NO: 12737, SEQ ID
NO: 12739, SEQ ID NO: 12741, SEQ ID NO: 12743, SEQ ID NO: 12745, SEQ ID NO: 12747,
SEQ ID NO: 12749, SEQ ID NO: 12751, SEQ ID NO: 12753, SEQ ID NO: 12754, SEQ ID
NO: 12756, SEQ ID NO: 12758, SEQ ID NO: 12760, SEQ ID NO: 12762, SEQ ID NO: 12763,
SEQ ID NO: 12764, SEQ ID NO: 12765, SEQ ID NO: 12767, SEQ ID NO: 12769, SEQ ID
NO: 12770, SEQ ID NO: 12771, SEQ ID NO: 12773, SEQ ID NO: 12775, SEQ ID NO: 12776,
SEQ ID NO: 12778, SEQ ID NO: 12780, SEQ ID NO: 12781, SEQ ID NO: 12783, SEQ ID
NO: 12785, SEQ ID NO: 12787, SEQ ID NO: 12789, SEQ ID NO: 12791, SEQ ID NO: 12793,
SEQ ID NO: 12795, SEQ ID NO: 12797, SEQ ID NO: 12798, SEQ ID NO: 12799, SEQ ID
NO: 12800, SEQ ID NO: 12801, SEQ ID NO: 12803, SEQ ID NO: 12805, SEQ ID NO: 12806,
SEQ ID NO: 12808, SEQ ID NO: 12810, SEQ ID NO: 12812, SEQ ID NO: 12813, SEQ ID
NO: 12815, SEQ ID NO: 12817, SEQ ID NO: 12819, SEQ ID NO: 12821, SEQ ID NO: 12823,
SEQ ID NO: 12825,
SEQ ID NO: 12827, SEQ ID NO: 12829, SEQ ID NO: 12831, SEQ ID NO: 12833, SEQ ID
NO: 12835, SEQ ID NO: 12837, SEQ ID NO: 12839, SEQ ID NO: 12841, SEQ ID NO: 12843,
SEQ ID NO: 12845, SEQ ID NO: 12847, SEQ ID NO: 12848, SEQ ID NO: 12850, SEQ ID
NO: 12852, SEQ ID NO: 12854, SEQ ID NO: 12856, SEQ ID NO: 12858, SEQ ID NO: 12860,
 
SEQ ID NO: 12862, SEQ ID NO: 12864, SEQ ID NO: 12866, SEQ ID NO: 12867, SEQ ID
NO: 12868, SEQ ID NO: 12870, SEQ ID NO: 12871, SEQ ID NO: 12873, SEQ ID NO: 12874,
SEQ ID NO: 12876, SEQ ID NO: 12878, SEQ ID NO: 12880, SEQ ID NO: 12881, SEQ ID
NO: 12883, SEQ ID NO: 12885, SEQ ID NO: 12887, SEQ ID NO: 12889, SEQ ID NO: 12891,
SEQ ID NO: 12892, SEQ ID NO: 12894, SEQ ID NO: 12896, SEQ ID NO: 12897, SEQ ID
NO: 12899, SEQ ID NO: 12901, SEQ ID NO: 12903, SEQ ID NO: 12905, SEQ ID NO: 12907,
SEQ ID NO: 12909, SEQ ID NO: 12911, SEQ ID NO: 12913, SEQ ID NO: 12915, SEQ ID
NO: 12916, SEQ ID NO: 12918, SEQ ID NO: 12920, SEQ ID NO: 12922, SEQ ID NO: 12923,
SEQ ID NO: 12925, SEQ ID NO: 12927, SEQ ID NO: 12929, SEQ ID NO: 12931, SEQ ID
NO: 12933, SEQ ID NO: 12934, SEQ ID NO: 12936, SEQ ID NO: 12938, SEQ ID NO: 12940,
SEQ ID NO: 12941, SEQ ID NO: 12943, SEQ ID NO: 12945, SEQ ID NO: 12947, SEQ ID
NO: 12949, SEQ ID NO: 12950, SEQ ID NO: 12952, SEQ ID NO: 12953, SEQ ID NO: 12955,
SEQ ID NO: 12956, SEQ ID NO: 12958, SEQ ID NO: 12960, SEQ ID NO: 12961, SEQ ID
NO: 12963, SEQ ID NO: 12965, SEQ ID NO: 12966, SEQ ID NO: 12967, SEQ ID NO: 12969,
SEQ ID NO: 12971, SEQ ID NO: 12973, SEQ ID NO: 12975, SEQ ID NO: 12977, SEQ ID
NO: 12979, SEQ ID NO: 12981, SEQ ID NO: 12983, SEQ ID NO: 12985, SEQ ID NO: 12987,
SEQ ID NO: 12989, SEQ ID NO: 12991, SEQ ID NO: 12993, SEQ ID NO: 12995, SEQ ID
NO: 12997, SEQ ID NO: 12999, SEQ ID NO: 13001, SEQ ID NO: 13003, SEQ ID NO: 13005,
SEQ ID NO: 13007,
SEQ ID NO: 13009, SEQ ID NO: 13011, SEQ ID NO: 13013, SEQ ID NO: 13015, SEQ ID
NO: 13017, SEQ ID NO: 13018, SEQ ID NO: 13020, SEQ ID NO: 13021, SEQ ID NO: 13023,
SEQ ID NO: 13025, SEQ ID NO: 13027, SEQ ID NO: 13029, SEQ ID NO: 13031, SEQ ID
NO: 13033, SEQ ID NO: 13035, SEQ ID NO: 13037, SEQ ID NO: 13039, SEQ ID NO: 13040,
SEQ ID NO: 13042, SEQ ID NO: 13044, SEQ ID NO: 13046, SEQ ID NO: 13048, SEQ ID
NO: 13050, SEQ ID NO: 13052, SEQ ID NO: 13053, SEQ ID NO: 13055, SEQ ID NO: 13057,
SEQ ID NO: 13058, SEQ ID NO: 13060, SEQ ID NO: 13061, SEQ ID NO: 13063, SEQ ID
NO: 13065, SEQ ID NO: 13067, SEQ ID NO: 13069, SEQ ID NO: 13071, SEQ ID NO: 13072,
SEQ ID NO: 13074, SEQ ID NO: 13076, SEQ ID NO: 13077, SEQ ID NO: 13079, SEQ ID
NO: 13081, SEQ ID NO: 13083, SEQ ID NO: 13085, SEQ ID NO: 13087, SEQ ID NO: 13088,
SEQ ID NO: 13089, SEQ ID NO: 13091, SEQ ID NO: 13092, SEQ ID NO: 13094, SEQ ID
NO: 13096, SEQ ID NO: 13097, SEQ ID NO: 13099, SEQ ID NO: 13101, SEQ ID NO: 13103,
SEQ ID NO: 13105, SEQ ID NO: 13107, SEQ ID NO: 13108, SEQ ID NO: 13109, SEQ ID
NO: 13110, SEQ ID NO: 13112, SEQ ID NO: 13114, SEQ ID NO: 13116, SEQ ID NO: 13118,
SEQ ID NO: 13119, SEQ ID NO: 13120, SEQ ID NO: 13122, SEQ ID NO: 13124, SEQ ID
NO: 13125, SEQ ID NO: 13127, SEQ ID NO: 13129, SEQ ID NO: 13131, SEQ ID NO: 13133,
SEQ ID NO: 13135, SEQ ID NO: 13137, SEQ ID NO: 13139, SEQ ID NO: 13141, SEQ ID
NO: 13143, SEQ ID NO: 13144, SEQ ID NO: 13145, SEQ ID NO: 13147, SEQ ID NO: 13149,
SEQ ID NO: 13151, SEQ ID NO: 13152, SEQ ID NO: 13154, SEQ ID NO: 13156, SEQ ID
NO: 13158, SEQ ID NO: 13160, SEQ ID NO: 13162, SEQ ID NO: 13164, SEQ ID NO: 13166,
SEQ ID NO: 13168, SEQ ID NO: 13170, SEQ ID NO: 13172, SEQ ID NO: 13174, SEQ ID
NO: 13176, SEQ ID NO: 13177, SEQ ID NO: 13178, SEQ ID NO: 13180, SEQ ID NO: 13181,
SEQ ID NO: 13183,
SEQ ID NO: 13185, SEQ ID NO: 13187, SEQ ID NO: 13189, SEQ ID NO: 13191, SEQ ID
NO: 13193, SEQ ID NO: 13195, SEQ ID NO: 13197, SEQ ID NO: 13198, SEQ ID NO: 13200,
SEQ ID NO: 13201, SEQ ID NO: 13203, SEQ ID NO: 13205, SEQ ID NO: 13207, SEQ ID
NO: 13209, SEQ ID NO: 13211, SEQ ID NO: 13213, SEQ ID NO: 13215, SEQ ID NO: 13216,
SEQ ID NO: 13218, SEQ ID NO: 13219, SEQ ID NO: 13221, SEQ ID NO: 13222, SEQ ID
NO: 13224, SEQ ID NO: 13226, SEQ ID NO: 13228, SEQ ID NO: 13230, SEQ ID NO: 13232,
SEQ ID NO: 13234, SEQ ID NO: 13235, SEQ ID NO: 13237, SEQ ID NO: 13238, SEQ ID
NO: 13239, SEQ ID NO: 13241, SEQ ID NO: 13242, SEQ ID NO: 13243, SEQ ID NO: 13245,
SEQ ID NO: 13246, SEQ ID NO: 13248, SEQ ID NO: 13250, SEQ ID NO: 13252, SEQ ID
NO: 13254, SEQ ID NO: 13256, SEQ ID NO: 13257, SEQ ID NO: 13259, SEQ ID NO: 13260,
 
SEQ ID NO: 13261, SEQ ID NO: 13263, SEQ ID NO: 13264, SEQ ID NO: 13266, SEQ ID
NO: 13268, SEQ ID NO: 13270, SEQ ID NO: 13271, SEQ ID NO: 13273, SEQ ID NO: 13275,
SEQ ID NO: 13276, SEQ ID NO: 13278, SEQ ID NO: 13279, SEQ ID NO: 13280, SEQ ID
NO: 13282, SEQ ID NO: 13284, SEQ ID NO: 13286, SEQ ID NO: 13288
SEQ ID NO: 13294, SEQ ID NO: 13296, SEQ ID NO: 13298, SEQ ID NO: 13300, SEQ ID
NO: 13302, SEQ ID NO: 13304, SEQ ID NO: 13306, SEQ ID NO: 13307, SEQ ID NO: 13309,
SEQ ID NO: 13310, SEQ ID NO: 13312, SEQ ID NO: 13314, SEQ ID NO: 13316, SEQ ID
NO: 13318, SEQ ID NO: 13319, SEQ ID NO: 13321, SEQ ID NO: 13322, SEQ ID NO: 13324,
SEQ ID NO: 13325, SEQ ID NO: 13327, SEQ ID NO: 13328, SEQ ID NO: 13330, SEQ ID
NO: 13332, SEQ ID NO: 13333, SEQ ID NO: 13334, SEQ ID NO: 13336, SEQ ID NO: 13337,
SEQ ID NO: 13338, SEQ ID NO: 13339, SEQ ID NO: 13341, SEQ ID NO: 13342, SEQ ID
NO: 13344, SEQ ID NO: 13345, SEQ ID NO: 13346, SEQ ID NO: 13347, SEQ ID NO: 13348,
SEQ ID NO: 13350, SEQ ID NO: 13351, SEQ ID NO: 13352, SEQ ID NO: 13354, SEQ ID
NO: 13356, SEQ ID NO: 13357, SEQ ID NO: 13359, SEQ ID NO: 13360, SEQ ID NO: 13362,
SEQ ID NO: 13363, SEQ ID NO: 13365, SEQ ID NO: 13366, SEQ ID NO: 13368, SEQ ID
NO: 13369, SEQ ID NO: 13370, SEQ ID NO: 13371, SEQ ID NO: 13372, SEQ ID NO: 13374,
SEQ ID NO: 13375, SEQ ID NO: 13376, SEQ ID NO: 13378, SEQ ID NO: 13379, SEQ ID
NO: 13381, SEQ ID NO: 13383, SEQ ID NO: 13385, SEQ ID NO: 13387, SEQ ID NO: 13389,
SEQ ID NO: 13391, SEQ ID NO: 13393, SEQ ID NO: 13394, SEQ ID NO: 13396, SEQ ID
NO: 13398, SEQ ID NO: 13400, SEQ ID NO: 13402, SEQ ID NO: 13404, SEQ ID NO: 13406,
SEQ ID NO: 13408, SEQ ID NO: 13410, SEQ ID NO: 13412, SEQ ID NO: 13414, SEQ ID
NO: 13415, SEQ ID NO: 13417, SEQ ID NO: 13418, SEQ ID NO: 13420, SEQ ID NO: 13421,
SEQ ID NO: 13423, SEQ ID NO: 13425, SEQ ID NO: 13427, SEQ ID NO: 13428, SEQ ID
NO: 13430, SEQ ID NO: 13431, SEQ ID NO: 13433, SEQ ID NO: 13434, SEQ ID NO: 13436,
SEQ ID NO: 13438, SEQ ID NO: 13439, SEQ ID NO: 13440, SEQ ID NO: 13441, SEQ ID
NO: 13442, SEQ ID NO: 13443, SEQ ID NO: 13444, SEQ ID NO: 13445, SEQ ID NO: 13447,
SEQ ID NO: 13448,
SEQ ID NO: 13449, SEQ ID NO: 13451, SEQ ID NO: 13453, SEQ ID NO: 13454, SEQ ID
NO: 13455, SEQ ID NO: 13457, SEQ ID NO: 13458, SEQ ID NO: 13460, SEQ ID NO: 13462,
SEQ ID NO: 13463, SEQ ID NO: 13465, SEQ ID NO: 13467, SEQ ID NO: 13469, SEQ ID
NO: 13470, SEQ ID NO: 13472, SEQ ID NO: 13473, SEQ ID NO: 13474, SEQ ID NO: 13476,
SEQ ID NO: 13477, SEQ ID NO: 13478, SEQ ID NO: 13479, SEQ ID NO: 13480, SEQ ID
NO: 13482, SEQ ID NO: 13483, SEQ ID NO: 13484, SEQ ID NO: 13485, SEQ ID NO: 13486,
SEQ ID NO: 13488, SEQ ID NO: 13489, SEQ ID NO: 13491, SEQ ID NO: 13492, SEQ ID
NO: 13494, SEQ ID NO: 13495, SEQ ID NO: 13497, SEQ ID NO: 13498, SEQ ID NO: 13500,
SEQ ID NO: 13501, SEQ ID NO: 13503, SEQ ID NO: 13505, SEQ ID NO: 13506, SEQ ID
NO: 13507, SEQ ID NO: 13508, SEQ ID NO: 13510, SEQ ID NO: 13512, SEQ ID NO: 13514,
SEQ ID NO: 13515, SEQ ID NO: 13517, SEQ ID NO: 13518, SEQ ID NO: 13519, SEQ ID
NO: 13520, SEQ ID NO: 13522, SEQ ID NO: 13523, SEQ ID NO: 13524, SEQ ID NO: 13526,
SEQ ID NO: 13527, SEQ ID NO: 13529, SEQ ID NO: 13531, SEQ ID NO: 13532, SEQ ID
NO: 13534, SEQ ID NO: 13535, SEQ ID NO: 13536, SEQ ID NO: 13538, SEQ ID NO: 13539,
SEQ ID NO: 13540, SEQ ID NO: 13542, SEQ ID NO: 13544, SEQ ID NO: 13546, SEQ ID
NO: 13547, SEQ ID NO: 13549, SEQ ID NO: 13551, SEQ ID NO: 13553, SEQ ID NO: 13554,
SEQ ID NO: 13556, SEQ ID NO: 13557, SEQ ID NO: 13559, SEQ ID NO: 13561, SEQ ID
NO: 13562, SEQ ID NO: 13563, SEQ ID NO: 13565, SEQ ID NO: 13566, SEQ ID NO: 13568,
SEQ ID NO: 13570, SEQ ID NO: 13571, SEQ ID NO: 13572, SEQ ID NO: 13574, SEQ ID
NO: 13576, SEQ ID NO: 13578, SEQ ID NO: 13580, SEQ ID NO: 13581, SEQ ID NO: 13582,
SEQ ID NO: 13584, SEQ ID NO: 13586, SEQ ID NO: 13588, SEQ ID NO: 13590, SEQ ID
NO: 13591, SEQ ID NO: 13592, SEQ ID NO: 13594, SEQ ID NO: 13595, SEQ ID NO: 13597,
SEQ ID NO: 13599,
SEQ ID NO: 13601, SEQ ID NO: 13603, SEQ ID NO: 13605, SEQ ID NO: 13607, SEQ ID
NO: 13608, SEQ ID NO: 13610, SEQ ID NO: 13612, SEQ ID NO: 13613, SEQ ID NO: 13615,
 
SEQ ID NO: 13617, SEQ ID NO: 13619, SEQ ID NO: 13620, SEQ ID NO: 13621, SEQ ID
NO: 13622, SEQ ID NO: 13623, SEQ ID NO: 13624, SEQ ID NO: 13625, SEQ ID NO: 13626,
SEQ ID NO: 13627, SEQ ID NO: 13629, SEQ ID NO: 13631, SEQ ID NO: 13633, SEQ ID
NO: 13635, SEQ ID NO: 13637, SEQ ID NO: 13639, SEQ ID NO: 13640, SEQ ID NO: 13642,
SEQ ID NO: 13643, SEQ ID NO: 13645, SEQ ID NO: 13647, SEQ ID NO: 13649, SEQ ID
NO: 13651, SEQ ID NO: 13652, SEQ ID NO: 13653, SEQ ID NO: 13655, SEQ ID NO: 13656,
SEQ ID NO: 13657, SEQ ID NO: 13658, SEQ ID NO: 13660, SEQ ID NO: 13662, SEQ ID
NO: 13664, SEQ ID NO: 13666, SEQ ID NO: 13668, SEQ ID NO: 13670, SEQ ID NO: 13672,
SEQ ID NO: 13674, SEQ ID NO: 13675, SEQ ID NO: 13676, SEQ ID NO: 13678, SEQ ID
NO: 13680, SEQ ID NO: 13682, SEQ ID NO: 13684, SEQ ID NO: 13685, SEQ ID NO: 13687,
SEQ ID NO: 13689, SEQ ID NO: 13690, SEQ ID NO: 13692, SEQ ID NO: 13693, SEQ ID
NO: 13694, SEQ ID NO: 13696, SEQ ID NO: 13698, SEQ ID NO: 13700, SEQ ID NO: 13701,
SEQ ID NO: 13703, SEQ ID NO: 13704, SEQ ID NO: 13706, SEQ ID NO: 13708, SEQ ID
NO: 13709, SEQ ID NO: 13710, SEQ ID NO: 13711, SEQ ID NO: 13713, SEQ ID NO: 13715,
SEQ ID NO: 13717, SEQ ID NO: 13718, SEQ ID NO: 13720, SEQ ID NO: 13722, SEQ ID
NO: 13724, SEQ ID NO: 13725, SEQ ID NO: 13727, SEQ ID NO: 13728, SEQ ID NO: 13729,
SEQ ID NO: 13731, SEQ ID NO: 13732, SEQ ID NO: 13734, SEQ ID NO: 13735, SEQ ID
NO: 13736, SEQ ID NO: 13738, SEQ ID NO: 13740, SEQ ID NO: 13742, SEQ ID NO: 13744,
SEQ ID NO: 13746, SEQ ID NO: 13748, SEQ ID NO: 13749, SEQ ID NO: 13750, SEQ ID
NO: 13751, SEQ ID NO: 13753, SEQ ID NO: 13754, SEQ ID NO: 13755, SEQ ID NO: 13756,
SEQ ID NO: 13757,
SEQ ID NO: 13759, SEQ ID NO: 13760, SEQ ID NO: 13761, SEQ ID NO: 13762, SEQ ID
NO: 13763, SEQ ID NO: 13764, SEQ ID NO: 13765, SEQ ID NO: 13766, SEQ ID NO: 13767,
SEQ ID NO: 13769, SEQ ID NO: 13770, SEQ ID NO: 13772, SEQ ID NO: 13774, SEQ ID
NO: 13775, SEQ ID NO: 13777, SEQ ID NO: 13778, SEQ ID NO: 13779, SEQ ID NO: 13780,
SEQ ID NO: 13782, SEQ ID NO: 13783, SEQ ID NO: 13785, SEQ ID NO: 13786, SEQ ID
NO: 13788, SEQ ID NO: 13789, SEQ ID NO: 13791, SEQ ID NO: 13793, SEQ ID NO: 13795,
SEQ ID NO: 13796, SEQ ID NO: 13797, SEQ ID NO: 13798, SEQ ID NO: 13799, SEQ ID
NO: 13800, SEQ ID NO: 13802, SEQ ID NO: 13803, SEQ ID NO: 13804, SEQ ID NO: 13805,
SEQ ID NO: 13806, SEQ ID NO: 13808, SEQ ID NO: 13809, SEQ ID NO: 13810, SEQ ID
NO: 13812, SEQ ID NO: 13814, SEQ ID NO: 13816, SEQ ID NO: 13818, SEQ ID NO: 13819,
SEQ ID NO: 13821, SEQ ID NO: 13823, SEQ ID NO: 13824, SEQ ID NO: 13826, SEQ ID
NO: 13828, SEQ ID NO: 13829, SEQ ID NO: 13831, SEQ ID NO: 13833, SEQ ID NO: 13834,
SEQ ID NO: 13836, SEQ ID NO: 13838, SEQ ID NO: 13839, SEQ ID NO: 13841, SEQ ID
NO: 13843, SEQ ID NO: 13845, SEQ ID NO: 13847, SEQ ID NO: 13849, SEQ ID NO: 13850,
SEQ ID NO: 13851, SEQ ID NO: 13852, SEQ ID NO: 13854, SEQ ID NO: 13856, SEQ ID
NO: 13858, SEQ ID NO: 13860, SEQ ID NO: 13861, SEQ ID NO: 13863, SEQ ID NO: 13865,
SEQ ID NO: 13867, SEQ ID NO: 13869, SEQ ID NO: 13870, SEQ ID NO: 13872, SEQ ID
NO: 13874, SEQ ID NO: 13876, SEQ ID NO: 13878, SEQ ID NO: 13879, SEQ ID NO: 13881,
SEQ ID NO: 13883, SEQ ID NO: 13885, SEQ ID NO: 13887, SEQ ID NO: 13889, SEQ ID
NO: 13891, SEQ ID NO: 13892, SEQ ID NO: 13893, SEQ ID NO: 13895, SEQ ID NO: 13896,
SEQ ID NO: 13898, SEQ ID NO: 13900, SEQ ID NO: 13901, SEQ ID NO: 13903, SEQ ID
NO: 13904, SEQ ID NO: 13905, SEQ ID NO: 13906, SEQ ID NO: 13908, SEQ ID NO: 13909,
SEQ ID NO: 13911,
SEQ ID NO: 13912, SEQ ID NO: 13914, SEQ ID NO: 13916, SEQ ID NO: 13918, SEQ ID
NO: 13920, SEQ ID NO: 13922, SEQ ID NO: 13924, SEQ ID NO: 13926, SEQ ID NO: 13928,
SEQ ID NO: 13930, SEQ ID NO: 13932, SEQ ID NO: 13933, SEQ ID NO: 13935, SEQ ID
NO: 13936, SEQ ID NO: 13938, SEQ ID NO: 13940, SEQ ID NO: 13941, SEQ ID NO: 13942,
SEQ ID NO: 13944, SEQ ID NO: 13946, SEQ ID NO: 13948, SEQ ID NO: 13950, SEQ ID
NO: 13951, SEQ ID NO: 13953, SEQ ID NO: 13954, SEQ ID NO: 13956, SEQ ID NO: 13958,
SEQ ID NO: 13959, SEQ ID NO: 13961, SEQ ID NO: 13962, SEQ ID NO: 13964, SEQ ID
NO: 13965, SEQ ID NO: 13966, SEQ ID NO: 13967, SEQ ID NO: 13969, SEQ ID NO: 13970,
 
SEQ ID NO: 13971, SEQ ID NO: 13973, SEQ ID NO: 13975, SEQ ID NO: 13976, SEQ ID
NO: 13978, SEQ ID NO: 13979, SEQ ID NO: 13981, SEQ ID NO: 13982, SEQ ID NO: 13983,
SEQ ID NO: 13984, SEQ ID NO: 13985, SEQ ID NO: 13986, SEQ ID NO: 13988, SEQ ID
NO: 13989, SEQ ID NO: 13990, SEQ ID NO: 13992, SEQ ID NO: 13994, SEQ ID NO: 13996,
SEQ ID NO: 13998, SEQ ID NO: 13999, SEQ ID NO: 14001, SEQ ID NO: 14003, SEQ ID
NO: 14005, SEQ ID NO: 14006, SEQ ID NO: 14008, SEQ ID NO: 14010, SEQ ID NO: 14012,
SEQ ID NO: 14013, SEQ ID NO: 14014, SEQ ID NO: 14015, SEQ ID NO: 14016, SEQ ID
NO: 14018, SEQ ID NO: 14019, SEQ ID NO: 14021, SEQ ID NO: 14023, SEQ ID NO: 14025,
SEQ ID NO: 14026, SEQ ID NO: 14028, SEQ ID NO: 14030, SEQ ID NO: 14031, SEQ ID
NO: 14033, SEQ ID NO: 14035, SEQ ID NO: 14037, SEQ ID NO: 14039, SEQ ID NO: 14041,
SEQ ID NO: 14042, SEQ ID NO: 14044, SEQ ID NO: 14045, SEQ ID NO: 14047, SEQ ID
NO: 14049, SEQ ID NO: 14050, SEQ ID NO: 14052, SEQ ID NO: 14053, SEQ ID NO: 14054,
SEQ ID NO: 14056, SEQ ID NO: 14058, SEQ ID NO: 14060, SEQ ID NO: 14062, SEQ ID
NO: 14064, SEQ ID NO: 14066, SEQ ID NO: 14067, SEQ ID NO: 14069, SEQ ID NO: 14071,
SEQ ID NO: 14072,
SEQ ID NO: 14073, SEQ ID NO: 14075, SEQ ID NO: 14076, SEQ ID NO: 14077, SEQ ID
NO: 14079, SEQ ID NO: 14081, SEQ ID NO: 14083, SEQ ID NO: 14084, SEQ ID NO: 14085,
SEQ ID NO: 14086, SEQ ID NO: 14088, SEQ ID NO: 14090, SEQ ID NO: 14092, SEQ ID
NO: 14094, SEQ ID NO: 14096, SEQ ID NO: 14098, SEQ ID NO: 14100, SEQ ID NO: 14102,
SEQ ID NO: 14104, SEQ ID NO: 14106, SEQ ID NO: 14108, SEQ ID NO: 14110, SEQ ID
NO: 14112, SEQ ID NO: 14114, SEQ ID NO: 14116, SEQ ID NO: 14118, SEQ ID NO: 14120,
SEQ ID NO: 14122, SEQ ID NO: 14124, SEQ ID NO: 14126, SEQ ID NO: 14128, SEQ ID
NO: 14129, SEQ ID NO: 14131, SEQ ID NO: 14133, SEQ ID NO: 14135, SEQ ID NO: 14137,
SEQ ID NO: 14139, SEQ ID NO: 14141, SEQ ID NO: 14143, SEQ ID NO: 14145, SEQ ID
NO: 14146, SEQ ID NO: 14148, SEQ ID NO: 14150, SEQ ID NO: 14152, SEQ ID NO: 14154,
SEQ ID NO: 14156, SEQ ID NO: 14158, SEQ ID NO: 14160, SEQ ID NO: 14162, SEQ ID
NO: 14164, SEQ ID NO: 14165, SEQ ID NO: 14167, SEQ ID NO: 14168, SEQ ID NO: 14169,
SEQ ID NO: 14171, SEQ ID NO: 14173, SEQ ID NO: 14175, SEQ ID NO: 14177, SEQ ID
NO: 14179, SEQ ID NO: 14181, SEQ ID NO: 14183, SEQ ID NO: 14185, SEQ ID NO: 14187,
SEQ ID NO: 14188, SEQ ID NO: 14190, SEQ ID NO: 14192, SEQ ID NO: 14194, SEQ ID
NO: 14196, SEQ ID NO: 14198, SEQ ID NO: 14200, SEQ ID NO: 14202, SEQ ID NO: 14204,
SEQ ID NO: 14206, SEQ ID NO: 14208, SEQ ID NO: 14210, SEQ ID NO: 14212, SEQ ID
NO: 14214, SEQ ID NO: 14216, SEQ ID NO: 14217, SEQ ID NO: 14219, SEQ ID NO: 14221,
SEQ ID NO: 14223, SEQ ID NO: 14225, SEQ ID NO: 14227, SEQ ID NO: 14229, SEQ ID
NO: 14231, SEQ ID NO: 14233, SEQ ID NO: 14235, SEQ ID NO: 14237, SEQ ID NO: 14239,
SEQ ID NO: 14241, SEQ ID NO: 14243, SEQ ID NO: 14245, SEQ ID NO: 14247, SEQ ID
NO: 14249, SEQ ID NO: 14251, SEQ ID NO: 14253, SEQ ID NO: 14255, SEQ ID NO: 14257,
SEQ ID NO: 14259,
SEQ ID NO: 14261, SEQ ID NO: 14263, SEQ ID NO: 14265, SEQ ID NO: 14266, SEQ ID
NO: 14268, SEQ ID NO: 14270, SEQ ID NO: 14272, SEQ ID NO: 14274, SEQ ID NO: 14276,
SEQ ID NO: 14278, SEQ ID NO: 14280, SEQ ID NO: 14282, SEQ ID NO: 14284, SEQ ID
NO: 14286, SEQ ID NO: 14287, SEQ ID NO: 14289, SEQ ID NO: 14291, SEQ ID NO: 14293,
SEQ ID NO: 14295, SEQ ID NO: 14297, SEQ ID NO: 14299, SEQ ID NO: 14300, SEQ ID
NO: 14302, SEQ ID NO: 14304, SEQ ID NO: 14305, SEQ ID NO: 14307, SEQ ID NO: 14309,
SEQ ID NO: 14311, SEQ ID NO: 14313, SEQ ID NO: 14315, SEQ ID NO: 14317, SEQ ID
NO: 14319, SEQ ID NO: 14321, SEQ ID NO: 14323, SEQ ID NO: 14325, SEQ ID NO: 14327,
SEQ ID NO: 14329, SEQ ID NO: 14330, SEQ ID NO: 14332, SEQ ID NO: 14333, SEQ ID
NO: 14335, SEQ ID NO: 14336, SEQ ID NO: 14338, SEQ ID NO: 14339, SEQ ID NO: 14341,
SEQ ID NO: 14343, SEQ ID NO: 14344, SEQ ID NO: 14345, SEQ ID NO: 14347, SEQ ID
NO: 14349, SEQ ID NO: 14351, SEQ ID NO: 14353, SEQ ID NO: 14355, SEQ ID NO: 14357,
SEQ ID NO: 14359, SEQ ID NO: 14361, SEQ ID NO: 14363, SEQ ID NO: 14365, SEQ ID
NO: 14367, SEQ ID NO: 14369, SEQ ID NO: 14371, SEQ ID NO: 14373, SEQ ID NO: 14375,
 
SEQ ID NO: 14377, SEQ ID NO: 14378, SEQ ID NO: 14380, SEQ ID NO: 14382, SEQ ID
NO: 14384, SEQ ID NO: 14386, SEQ ID NO: 14388, SEQ ID NO: 14390, SEQ ID NO: 14392,
SEQ ID NO: 14394, SEQ ID NO: 14395, SEQ ID NO: 14397, SEQ ID NO: 14399, SEQ ID
NO: 14400, SEQ ID NO: 14402, SEQ ID NO: 14403, SEQ ID NO: 14405, SEQ ID NO: 14407,
SEQ ID NO: 14409, SEQ ID NO: 14411, SEQ ID NO: 14413, SEQ ID NO: 14415, SEQ ID
NO: 14417, SEQ ID NO: 14419, SEQ ID NO: 14421, SEQ ID NO: 14422, SEQ ID NO: 14424,
SEQ ID NO: 14426, SEQ ID NO: 14428, SEQ ID NO: 14430, SEQ ID NO: 14432, SEQ ID
NO: 14434, SEQ ID NO: 14436, SEQ ID NO: 14438, SEQ ID NO: 14439, SEQ ID NO: 14441,
SEQ ID NO: 14443,
SEQ ID NO: 14445, SEQ ID NO: 14447, SEQ ID NO: 14449, SEQ ID NO: 14451, SEQ ID
NO: 14453, SEQ ID NO: 14455, SEQ ID NO: 14457, SEQ ID NO: 14459, SEQ ID NO: 14461,
SEQ ID NO: 14463, SEQ ID NO: 14464, SEQ ID NO: 14465, SEQ ID NO: 14467, SEQ ID
NO: 14469, SEQ ID NO: 14471, SEQ ID NO: 14473, SEQ ID NO: 14475, SEQ ID NO: 14477,
SEQ ID NO: 14479, SEQ ID NO: 14481, SEQ ID NO: 14483, SEQ ID NO: 14485, SEQ ID
NO: 14487, SEQ ID NO: 14489, SEQ ID NO: 14491, SEQ ID NO: 14493, SEQ ID NO: 14495,
SEQ ID NO: 14497, SEQ ID NO: 14499, SEQ ID NO: 14501, SEQ ID NO: 14503, SEQ ID
NO: 14504, SEQ ID NO: 14506, SEQ ID NO: 14508, SEQ ID NO: 14510, SEQ ID NO: 14512,
SEQ ID NO: 14513, SEQ ID NO: 14515, SEQ ID NO: 14517, SEQ ID NO: 14519, SEQ ID
NO: 14521, SEQ ID NO: 14523, SEQ ID NO: 14525, SEQ ID NO: 14527, SEQ ID NO: 14528,
SEQ ID NO: 14529, SEQ ID NO: 14531, SEQ ID NO: 14533, SEQ ID NO: 14535, SEQ ID
NO: 14537, SEQ ID NO: 14539, SEQ ID NO: 14540, SEQ ID NO: 14542, SEQ ID NO: 14544,
SEQ ID NO: 14545, SEQ ID NO: 14547, SEQ ID NO: 14549, SEQ ID NO: 14551, SEQ ID
NO: 14552, SEQ ID NO: 14554, SEQ ID NO: 14556, SEQ ID NO: 14558, SEQ ID NO: 14560,
SEQ ID NO: 14562, SEQ ID NO: 14564, SEQ ID NO: 14566, SEQ ID NO: 14568, SEQ ID
NO: 14570, SEQ ID NO: 14572, SEQ ID NO: 14574, SEQ ID NO: 14576, SEQ ID NO: 14578,
SEQ ID NO: 14580, SEQ ID NO: 14582, SEQ ID NO: 14584, SEQ ID NO: 14586, SEQ ID
NO: 14588, SEQ ID NO: 14590, SEQ ID NO: 14592, SEQ ID NO: 14594, SEQ ID NO: 14595,
SEQ ID NO: 14597, SEQ ID NO: 14599, SEQ ID NO: 14601, SEQ ID NO: 14603, SEQ ID
NO: 14605, SEQ ID NO: 14607, SEQ ID NO: 14609, SEQ ID NO: 14610, SEQ ID NO: 14611,
SEQ ID NO: 14613, SEQ ID NO: 14615, SEQ ID NO: 14617, SEQ ID NO: 14619, SEQ ID
NO: 14620, SEQ ID NO: 14622, SEQ ID NO: 14624, SEQ ID NO: 14626, SEQ ID NO: 14628,
SEQ ID NO: 14630,
SEQ ID NO: 14632, SEQ ID NO: 14633, SEQ ID NO: 14635, SEQ ID NO: 14637, SEQ ID
NO: 14639, SEQ ID NO: 14641, SEQ ID NO: 14643, SEQ ID NO: 14644, SEQ ID NO: 14646,
SEQ ID NO: 14648, SEQ ID NO: 14650, SEQ ID NO: 14652, SEQ ID NO: 14654, SEQ ID
NO: 14656, SEQ ID NO: 14658, SEQ ID NO: 14660, SEQ ID NO: 14662, SEQ ID NO: 14664,
SEQ ID NO: 14666, SEQ ID NO: 14668, SEQ ID NO: 14670, SEQ ID NO: 14672, SEQ ID
NO: 14674, SEQ ID NO: 14676, SEQ ID NO: 14678, SEQ ID NO: 14679, SEQ ID NO: 14681,
SEQ ID NO: 14683, SEQ ID NO: 14685, SEQ ID NO: 14686, SEQ ID NO: 14688, SEQ ID
NO: 14690, SEQ ID NO: 14692, SEQ ID NO: 14693, SEQ ID NO: 14695, SEQ ID NO: 14697,
SEQ ID NO: 14699, SEQ ID NO: 14700, SEQ ID NO: 14702, SEQ ID NO: 14704, SEQ ID
NO: 14706, SEQ ID NO: 14708, SEQ ID NO: 14710, SEQ ID NO: 14712, SEQ ID NO: 14713,
SEQ ID NO: 14715, SEQ ID NO: 14717, SEQ ID NO: 14719, SEQ ID NO: 14721, SEQ ID
NO: 14722, SEQ ID NO: 14724, SEQ ID NO: 14725, SEQ ID NO: 14727, SEQ ID NO: 14729,
SEQ ID NO: 14731, SEQ ID NO: 14732, SEQ ID NO: 14734, SEQ ID NO: 14735, SEQ ID
NO: 14737, SEQ ID NO: 14738, SEQ ID NO: 14740, SEQ ID NO: 14742, SEQ ID NO: 14744,
SEQ ID NO: 14746, SEQ ID NO: 14748, SEQ ID NO: 14750, SEQ ID NO: 14752, SEQ ID
NO: 14754, SEQ ID NO: 14755, SEQ ID NO: 14757, SEQ ID NO: 14758, SEQ ID NO: 14760,
SEQ ID NO: 14762, SEQ ID NO: 14764, SEQ ID NO: 14766, SEQ ID NO: 14768, SEQ ID
NO: 14770, SEQ ID NO: 14772, SEQ ID NO: 14774, SEQ ID NO: 14776, SEQ ID NO: 14778,
SEQ ID NO: 14780, SEQ ID NO: 14782, SEQ ID NO: 14784, SEQ ID NO: 14786, SEQ ID
NO: 14788, SEQ ID NO: 14790, SEQ ID NO: 14792, SEQ ID NO: 14794, SEQ ID NO: 14796,
 
SEQ ID NO: 14798, SEQ ID NO: 14800, SEQ ID NO: 14802, SEQ ID NO: 14804, SEQ ID
NO: 14806, SEQ ID NO: 14808, SEQ ID NO: 14809, SEQ ID NO: 14811, SEQ ID NO: 14812,
SEQ ID NO: 14814,
SEQ ID NO: 14816, SEQ ID NO: 14818, SEQ ID NO: 14820, SEQ ID NO: 14822, SEQ ID
NO: 14823, SEQ ID NO: 14824, SEQ ID NO: 14826, SEQ ID NO: 14828, SEQ ID NO: 14830,
SEQ ID NO: 14831, SEQ ID NO: 14833, SEQ ID NO: 14835, SEQ ID NO: 14837, SEQ ID
NO: 14839, SEQ ID NO: 14841, SEQ ID NO: 14843, SEQ ID NO: 14845, SEQ ID NO: 14847,
SEQ ID NO: 14849, SEQ ID NO: 14850, SEQ ID NO: 14852, SEQ ID NO: 14854, SEQ ID
NO: 14856, SEQ ID NO: 14858, SEQ ID NO: 14860, SEQ ID NO: 14862, SEQ ID NO: 14864,
SEQ ID NO: 14865, SEQ ID NO: 14867, SEQ ID NO: 14869, SEQ ID NO: 14871, SEQ ID
NO: 14872, SEQ ID NO: 14874, SEQ ID NO: 14876, SEQ ID NO: 14878, SEQ ID NO: 14880,
SEQ ID NO: 14882, SEQ ID NO: 14884, SEQ ID NO: 14886, SEQ ID NO: 14888, SEQ ID
NO: 14890, SEQ ID NO: 14892, SEQ ID NO: 14894, SEQ ID NO: 14896, SEQ ID NO: 14898,
SEQ ID NO: 14900, SEQ ID NO: 14902, SEQ ID NO: 14904, SEQ ID NO: 14906, SEQ ID
NO: 14908, SEQ ID NO: 14910, SEQ ID NO: 14912, SEQ ID NO: 14914, SEQ ID NO: 14916,
SEQ ID NO: 14918, SEQ ID NO: 14919, SEQ ID NO: 14921, SEQ ID NO: 14923, SEQ ID
NO: 14924, SEQ ID NO: 14925, SEQ ID NO: 14927, SEQ ID NO: 14929, SEQ ID NO: 14931,
SEQ ID NO: 14933, SEQ ID NO: 14935, SEQ ID NO: 14937, SEQ ID NO: 14938, SEQ ID
NO: 14940, SEQ ID NO: 14942, SEQ ID NO: 14944, SEQ ID NO: 14946, SEQ ID NO: 14948,
SEQ ID NO: 14950, SEQ ID NO: 14952, SEQ ID NO: 14954, SEQ ID NO: 14956, SEQ ID
NO: 14958, SEQ ID NO: 14960, SEQ ID NO: 14961, SEQ ID NO: 14963, SEQ ID NO: 14965,
SEQ ID NO: 14967, SEQ ID NO: 14969, SEQ ID NO: 14971, SEQ ID NO: 14973, SEQ ID
NO: 14975, SEQ ID NO: 14977, SEQ ID NO: 14979, SEQ ID NO: 14981, SEQ ID NO: 14982,
SEQ ID NO: 14984, SEQ ID NO: 14986, SEQ ID NO: 14987, SEQ ID NO: 14988, SEQ ID
NO: 14990, SEQ ID NO: 14992, SEQ ID NO: 14993, SEQ ID NO: 14994, SEQ ID NO: 14996,
SEQ ID NO: 14998,
SEQ ID NO: 15000, SEQ ID NO: 15002, SEQ ID NO: 15004, SEQ ID NO: 15005, SEQ ID
NO: 15006, SEQ ID NO: 15007, SEQ ID NO: 15009, SEQ ID NO: 15011, SEQ ID NO: 15012,
SEQ ID NO: 15013, SEQ ID NO: 15015, SEQ ID NO: 15017, SEQ ID NO: 15019, SEQ ID
NO: 15021, SEQ ID NO: 15023, SEQ ID NO: 15025, SEQ ID NO: 15026, SEQ ID NO: 15028,
SEQ ID NO: 15029, SEQ ID NO: 15031, SEQ ID NO: 15033, SEQ ID NO: 15035, SEQ ID
NO: 15037, SEQ ID NO: 15039, SEQ ID NO: 15040, SEQ ID NO: 15042, SEQ ID NO: 15044,
SEQ ID NO: 15046, SEQ ID NO: 15048, SEQ ID NO: 15050, SEQ ID NO: 15051, SEQ ID
NO: 15053, SEQ ID NO: 15055, SEQ ID NO: 15057, SEQ ID NO: 15059, SEQ ID NO: 15060,
SEQ ID NO: 15062, SEQ ID NO: 15064, SEQ ID NO: 15066, SEQ ID NO: 15067, SEQ ID
NO: 15069, SEQ ID NO: 15071, SEQ ID NO: 15073, SEQ ID NO: 15075, SEQ ID NO: 15077,
SEQ ID NO: 15078, SEQ ID NO: 15080, SEQ ID NO: 15082, SEQ ID NO: 15084, SEQ ID
NO: 15086, SEQ ID NO: 15088, SEQ ID NO: 15090, SEQ ID NO: 15091, SEQ ID NO: 15093,
SEQ ID NO: 15095, SEQ ID NO: 15096, SEQ ID NO: 15098, SEQ ID NO: 15099, SEQ ID
NO: 15101, SEQ ID NO: 15103, SEQ ID NO: 15105, SEQ ID NO: 15107, SEQ ID NO: 15109,
SEQ ID NO: 15111, SEQ ID NO: 15113, SEQ ID NO: 15115, SEQ ID NO: 15117, SEQ ID
NO: 15119, SEQ ID NO: 15121, SEQ ID NO: 15123, SEQ ID NO: 15125, SEQ ID NO: 15127,
SEQ ID NO: 15129, SEQ ID NO: 15130, SEQ ID NO: 15132, SEQ ID NO: 15134, SEQ ID
NO: 15136, SEQ ID NO: 15138, SEQ ID NO: 15140, SEQ ID NO: 15142, SEQ ID NO: 15144,
SEQ ID NO: 15146, SEQ ID NO: 15147, SEQ ID NO: 15148, SEQ ID NO: 15150, SEQ ID
NO: 15152, SEQ ID NO: 15154, SEQ ID NO: 15155, SEQ ID NO: 15157, SEQ ID NO: 15159,
SEQ ID NO: 15161, SEQ ID NO: 15163, SEQ ID NO: 15165, SEQ ID NO: 15167, SEQ ID
NO: 15168, SEQ ID NO: 15169, SEQ ID NO: 15171, SEQ ID NO: 15173, SEQ ID NO: 15175,
SEQ ID NO: 15177,
SEQ ID NO: 15179, SEQ ID NO: 15181, SEQ ID NO: 15183, SEQ ID NO: 15185, SEQ ID
NO: 15187, SEQ ID NO: 15189, SEQ ID NO: 15191, SEQ ID NO: 15192, SEQ ID NO: 15194,
SEQ ID NO: 15196, SEQ ID NO: 15198, SEQ ID NO: 15200, SEQ ID NO: 15201, SEQ ID
 
NO: 15203, SEQ ID NO: 15205, SEQ ID NO: 15207, SEQ ID NO: 15209, SEQ ID NO: 15211,
SEQ ID NO: 15213, SEQ ID NO: 15215, SEQ ID NO: 15217, SEQ ID NO: 15219, SEQ ID
NO: 15220, SEQ ID NO: 15222, SEQ ID NO: 15224, SEQ ID NO: 15226, SEQ ID NO: 15228,
SEQ ID NO: 15230, SEQ ID NO: 15232, SEQ ID NO: 15234, SEQ ID NO: 15236, SEQ ID
NO: 15238, SEQ ID NO: 15240, SEQ ID NO: 15242, SEQ ID NO: 15244, SEQ ID NO: 15246,
SEQ ID NO: 15248, SEQ ID NO: 15250, SEQ ID NO: 15252, SEQ ID NO: 15254, SEQ ID
NO: 15256, SEQ ID NO: 15258, SEQ ID NO: 15259, SEQ ID NO: 15261, SEQ ID NO: 15263,
SEQ ID NO: 15265, SEQ ID NO: 15267, SEQ ID NO: 15269, SEQ ID NO: 15271, SEQ ID
NO: 15272, SEQ ID NO: 15274, SEQ ID NO: 15276, SEQ ID NO: 15278, SEQ ID NO: 15280,
SEQ ID NO: 15282, SEQ ID NO: 15283, SEQ ID NO: 15285, SEQ ID NO: 15287, SEQ ID
NO: 15289, SEQ ID NO: 15291, SEQ ID NO: 15292, SEQ ID NO: 15294, SEQ ID NO: 15295,
SEQ ID NO: 15297, SEQ ID NO: 15298, SEQ ID NO: 15300, SEQ ID NO: 15301, SEQ ID
NO: 15302, SEQ ID NO: 15304, SEQ ID NO: 15305, SEQ ID NO: 15307, SEQ ID NO: 15309,
SEQ ID NO: 15311, SEQ ID NO: 15313, SEQ ID NO: 15315, SEQ ID NO: 15316, SEQ ID
NO: 15318, SEQ ID NO: 15319, SEQ ID NO: 15321, SEQ ID NO: 15323, SEQ ID NO: 15325,
SEQ ID NO: 15327, SEQ ID NO: 15329, SEQ ID NO: 15331, SEQ ID NO: 15333, SEQ ID
NO: 15334, SEQ ID NO: 15336, SEQ ID NO: 15338, SEQ ID NO: 15340, SEQ ID NO: 15342,
SEQ ID NO: 15344, SEQ ID NO: 15346, SEQ ID NO: 15348, SEQ ID NO: 15350, SEQ ID
NO: 15352, SEQ ID NO: 15354, SEQ ID NO: 15355, SEQ ID NO: 15357, SEQ ID NO: 15359,
SEQ ID NO: 15361,
SEQ ID NO: 15363, SEQ ID NO: 15364, SEQ ID NO: 15366, SEQ ID NO: 15368, SEQ ID
NO: 15370, SEQ ID NO: 15372, SEQ ID NO: 15374, SEQ ID NO: 15376, SEQ ID NO: 15378,
SEQ ID NO: 15380, SEQ ID NO: 15382, SEQ ID NO: 15384, SEQ ID NO: 15386, SEQ ID
NO: 15388, SEQ ID NO: 15390, SEQ ID NO: 15391, SEQ ID NO: 15393, SEQ ID NO: 15395,
SEQ ID NO: 15396, SEQ ID NO: 15397, SEQ ID NO: 15399, SEQ ID NO: 15401, SEQ ID
NO: 15403, SEQ ID NO: 15404, SEQ ID NO: 15406, SEQ ID NO: 15407, SEQ ID NO: 15409,
SEQ ID NO: 15411, SEQ ID NO: 15412, SEQ ID NO: 15414, SEQ ID NO: 15416, SEQ ID
NO: 15417, SEQ ID NO: 15419, SEQ ID NO: 15421, SEQ ID NO: 15423, SEQ ID NO: 15425,
SEQ ID NO: 15427, SEQ ID NO: 15429, SEQ ID NO: 15431, SEQ ID NO: 15433, SEQ ID
NO: 15435, SEQ ID NO: 15436, SEQ ID NO: 15438, SEQ ID NO: 15439, SEQ ID NO: 15441,
SEQ ID NO: 15442, SEQ ID NO: 15443, SEQ ID NO: 15445, SEQ ID NO: 15446, SEQ ID
NO: 15447, SEQ ID NO: 15448, SEQ ID NO: 15449, SEQ ID NO: 15450, SEQ ID NO: 15451,
SEQ ID NO: 15453, SEQ ID NO: 15455, SEQ ID NO: 15457, SEQ ID NO: 15459, SEQ ID
NO: 15461, SEQ ID NO: 15463, SEQ ID NO: 15465, SEQ ID NO: 15467, SEQ ID NO: 15469,
SEQ ID NO: 15471, SEQ ID NO: 15473, SEQ ID NO: 15475, SEQ ID NO: 15477, SEQ ID
NO: 15478, SEQ ID NO: 15480, SEQ ID NO: 15482, SEQ ID NO: 15484, SEQ ID NO: 15485,
SEQ ID NO: 15487, SEQ ID NO: 15489, SEQ ID NO: 15491, SEQ ID NO: 15492, SEQ ID
NO: 15494, SEQ ID NO: 15496, SEQ ID NO: 15497, SEQ ID NO: 15498, SEQ ID NO: 15500,
SEQ ID NO: 15501, SEQ ID NO: 15502, SEQ ID NO: 15503, SEQ ID NO: 15505, SEQ ID
NO: 15507, SEQ ID NO: 15509, SEQ ID NO: 15510, SEQ ID NO: 15511, SEQ ID NO: 15513,
SEQ ID NO: 15514, SEQ ID NO: 15516, SEQ ID NO: 15518, SEQ ID NO: 15520, SEQ ID
NO: 15522, SEQ ID NO: 15524, SEQ ID NO: 15525, SEQ ID NO: 15527, SEQ ID NO: 15528,
SEQ ID NO: 15529,
SEQ ID NO: 15531, SEQ ID NO: 15533, SEQ ID NO: 15534, SEQ ID NO: 15535, SEQ ID
NO: 15537, SEQ ID NO: 15539, SEQ ID NO: 15540, SEQ ID NO: 15542, SEQ ID NO: 15544,
SEQ ID NO: 15545, SEQ ID NO: 15547, SEQ ID NO: 15548, SEQ ID NO: 15550, SEQ ID
NO: 15551, SEQ ID NO: 15552, SEQ ID NO: 15554, SEQ ID NO: 15556, SEQ ID NO: 15557,
SEQ ID NO: 15558, SEQ ID NO: 15560, SEQ ID NO: 15561, SEQ ID NO: 15563, SEQ ID
NO: 15564, SEQ ID NO: 15565, SEQ ID NO: 15567, SEQ ID NO: 15569, SEQ ID NO: 15571,
SEQ ID NO: 15573, SEQ ID NO: 15575, SEQ ID NO: 15577, SEQ ID NO: 15578, SEQ ID
NO: 15580, SEQ ID NO: 15581, SEQ ID NO: 15582, SEQ ID NO: 15584, SEQ ID NO: 15585,
SEQ ID NO: 15587, SEQ ID NO: 15589, SEQ ID NO: 15591, SEQ ID NO: 15592, SEQ ID
 
NO: 15593, SEQ ID NO: 15594, SEQ ID NO: 15596, SEQ ID NO: 15598, SEQ ID NO: 15599,
SEQ ID NO: 15601, SEQ ID NO: 15602, SEQ ID NO: 15603, SEQ ID NO: 15604, SEQ ID
NO: 15606, SEQ ID NO: 15607, SEQ ID NO: 15608, SEQ ID NO: 15610, SEQ ID NO: 15611,
SEQ ID NO: 15613, SEQ ID NO: 15615, SEQ ID NO: 15617, SEQ ID NO: 15619, SEQ ID
NO: 15621, SEQ ID NO: 15623, SEQ ID NO: 15625, SEQ ID NO: 15627, SEQ ID NO: 15629,
SEQ ID NO: 15630, SEQ ID NO: 15632, SEQ ID NO: 15634, SEQ ID NO: 15636, SEQ ID
NO: 15638, SEQ ID NO: 15640, SEQ ID NO: 15642, SEQ ID NO: 15644, SEQ ID NO: 15646,
SEQ ID NO: 15648, SEQ ID NO: 15649, SEQ ID NO: 15650, SEQ ID NO: 15651, SEQ ID
NO: 15653, SEQ ID NO: 15655, SEQ ID NO: 15656, SEQ ID NO: 15658, SEQ ID NO: 15660,
SEQ ID NO: 15661, SEQ ID NO: 15663, SEQ ID NO: 15665, SEQ ID NO: 15667, SEQ ID
NO: 15669, SEQ ID NO: 15670, SEQ ID NO: 15671, SEQ ID NO: 15672, SEQ ID NO: 15674,
SEQ ID NO: 15675, SEQ ID NO: 15676, SEQ ID NO: 15677, SEQ ID NO: 15679, SEQ ID
NO: 15681, SEQ ID NO: 15683, SEQ ID NO: 15685, SEQ ID NO: 15687, SEQ ID NO: 15689,
SEQ ID NO: 15690,
SEQ ID NO: 15692, SEQ ID NO: 15694, SEQ ID NO: 15695, SEQ ID NO: 15697, SEQ ID
NO: 15699, SEQ ID NO: 15701, SEQ ID NO: 15703, SEQ ID NO: 15704, SEQ ID NO: 15706,
SEQ ID NO: 15707, SEQ ID NO: 15709, SEQ ID NO: 15711, SEQ ID NO: 15713, SEQ ID
NO: 15715, SEQ ID NO: 15717, SEQ ID NO: 15718, SEQ ID NO: 15719, SEQ ID NO: 15720,
SEQ ID NO: 15722, SEQ ID NO: 15723, SEQ ID NO: 15725, SEQ ID NO: 15727, SEQ ID
NO: 15728, SEQ ID NO: 15729, SEQ ID NO: 15731, SEQ ID NO: 15732, SEQ ID NO: 15734,
SEQ ID NO: 15735, SEQ ID NO: 15737, SEQ ID NO: 15738, SEQ ID NO: 15740, SEQ ID
NO: 15742, SEQ ID NO: 15744, SEQ ID NO: 15746, SEQ ID NO: 15748, SEQ ID NO: 15749,
SEQ ID NO: 15751, SEQ ID NO: 15752, SEQ ID NO: 15754, SEQ ID NO: 15756, SEQ ID
NO: 15758, SEQ ID NO: 15760, SEQ ID NO: 15762, SEQ ID NO: 15764, SEQ ID NO: 15765,
SEQ ID NO: 15767, SEQ ID NO: 15769, SEQ ID NO: 15771, SEQ ID NO: 15773, SEQ ID
NO: 15775, SEQ ID NO: 15777, SEQ ID NO: 15778, SEQ ID NO: 15780, SEQ ID NO: 15781,
SEQ ID NO: 15783, SEQ ID NO: 15785, SEQ ID NO: 15787, SEQ ID NO: 15789, SEQ ID
NO: 15791, SEQ ID NO: 15793, SEQ ID NO: 15795, SEQ ID NO: 15797, SEQ ID NO: 15799,
SEQ ID NO: 15801, SEQ ID NO: 15802, SEQ ID NO: 15804, SEQ ID NO: 15806, SEQ ID
NO: 15808, SEQ ID NO: 15810, SEQ ID NO: 15812, SEQ ID NO: 15814, SEQ ID NO: 15816,
SEQ ID NO: 15818, SEQ ID NO: 15819, SEQ ID NO: 15820, SEQ ID NO: 15821, SEQ ID
NO: 15823, SEQ ID NO: 15825, SEQ ID NO: 15827, SEQ ID NO: 15829, SEQ ID NO: 15830,
SEQ ID NO: 15831, SEQ ID NO: 15832, SEQ ID NO: 15833, SEQ ID NO: 15835, SEQ ID
NO: 15836, SEQ ID NO: 15837, SEQ ID NO: 15839, SEQ ID NO: 15841, SEQ ID NO: 15843,
SEQ ID NO: 15845, SEQ ID NO: 15846, SEQ ID NO: 15848, SEQ ID NO: 15850, SEQ ID
NO: 15852, SEQ ID NO: 15853, SEQ ID NO: 15855, SEQ ID NO: 15857, SEQ ID NO: 15859,
SEQ ID NO: 15861,
SEQ ID NO: 15863, SEQ ID NO: 15865, SEQ ID NO: 15866, SEQ ID NO: 15867, SEQ ID
NO: 15869, SEQ ID NO: 15870, SEQ ID NO: 15872, SEQ ID NO: 15874, SEQ ID NO: 15876,
SEQ ID NO: 15878, SEQ ID NO: 15880, SEQ ID NO: 15881, SEQ ID NO: 15882, SEQ ID
NO: 15883, SEQ ID NO: 15885, SEQ ID NO: 15887, SEQ ID NO: 15889, SEQ ID NO: 15891,
SEQ ID NO: 15892, SEQ ID NO: 15893, SEQ ID NO: 15895, SEQ ID NO: 15897, SEQ ID
NO: 15899, SEQ ID NO: 15900, SEQ ID NO: 15901, SEQ ID NO: 15903, SEQ ID NO: 15905,
SEQ ID NO: 15906, SEQ ID NO: 15908, SEQ ID NO: 15909, SEQ ID NO: 15910, SEQ ID
NO: 15912, SEQ ID NO: 15914, SEQ ID NO: 15916, SEQ ID NO: 15917, SEQ ID NO: 15919,
SEQ ID NO: 15920, SEQ ID NO: 15922, SEQ ID NO: 15924, SEQ ID NO: 15926, SEQ ID
NO: 15928, SEQ ID NO: 15929, SEQ ID NO: 15930, SEQ ID NO: 15932, SEQ ID NO: 15934,
SEQ ID NO: 15936, SEQ ID NO: 15938, SEQ ID NO: 15940, SEQ ID NO: 15942, SEQ ID
NO: 15944, SEQ ID NO: 15946, SEQ ID NO: 15948, SEQ ID NO: 15950, SEQ ID NO: 15952,
SEQ ID NO: 15954, SEQ ID NO: 15956, SEQ ID NO: 15958, SEQ ID NO: 15960, SEQ ID
NO: 15962, SEQ ID NO: 15964, SEQ ID NO: 15966, SEQ ID NO: 15968, SEQ ID NO: 15970,
SEQ ID NO: 15972, SEQ ID NO: 15974, SEQ ID NO: 15976, SEQ ID NO: 15978, SEQ ID
 
NO: 15980, SEQ ID NO: 15982, SEQ ID NO: 15984, SEQ ID NO: 15986, SEQ ID NO: 15988,
SEQ ID NO: 15990, SEQ ID NO: 15992, SEQ ID NO: 15993, SEQ ID NO: 15995, SEQ ID
NO: 15997, SEQ ID NO: 15999, SEQ ID NO: 16001, SEQ ID NO: 16003, SEQ ID NO: 16005,
SEQ ID NO: 16007, SEQ ID NO: 16009, SEQ ID NO: 16011, SEQ ID NO: 16013, SEQ ID
NO: 16015, SEQ ID NO: 16017, SEQ ID NO: 16019, SEQ ID NO: 16021, SEQ ID NO: 16023,
SEQ ID NO: 16025, SEQ ID NO: 16027, SEQ ID NO: 16029, SEQ ID NO: 16031, SEQ ID
NO: 16033, SEQ ID NO: 16035, SEQ ID NO: 16037, SEQ ID NO: 16039, SEQ ID NO: 16041,
SEQ ID NO: 16043,
SEQ ID NO: 16045, SEQ ID NO: 16047, SEQ ID NO: 16048, SEQ ID NO: 16049, SEQ ID
NO: 16051, SEQ ID NO: 16052, SEQ ID NO: 16054, SEQ ID NO: 16056, SEQ ID NO: 16057,
SEQ ID NO: 16059, SEQ ID NO: 16061, SEQ ID NO: 16063, SEQ ID NO: 16065, SEQ ID
NO: 16067, SEQ ID NO: 16069, SEQ ID NO: 16071, SEQ ID NO: 16073, SEQ ID NO: 16075,
SEQ ID NO: 16077, SEQ ID NO: 16079, SEQ ID NO: 16081, SEQ ID NO: 16083, SEQ ID
NO: 16085, SEQ ID NO: 16087, SEQ ID NO: 16089, SEQ ID NO: 16091, SEQ ID NO: 16093,
SEQ ID NO: 16095, SEQ ID NO: 16097, SEQ ID NO: 16099, SEQ ID NO: 16101, SEQ ID
NO: 16103, SEQ ID NO: 16105, SEQ ID NO: 16107, SEQ ID NO: 16109,
SEQ ID NO: 16219, SEQ ID NO: 16220, SEQ ID NO: 16222, SEQ ID NO: 16224, SEQ ID
NO: 16225, SEQ ID NO: 16226, SEQ ID NO: 16228, SEQ ID NO: 16229, SEQ ID NO: 16230,
SEQ ID NO: 16232, SEQ ID NO: 16233, SEQ ID NO: 16235, SEQ ID NO: 16236, SEQ ID
NO: 16238, SEQ ID NO: 16240, SEQ ID NO: 16242, SEQ ID NO: 16243, SEQ ID NO: 16245,
SEQ ID NO: 16246, SEQ ID NO: 16247, SEQ ID NO: 16248, SEQ ID NO: 16249, SEQ ID
NO: 16250, SEQ ID NO: 16251, SEQ ID NO: 16252, SEQ ID NO: 16253, SEQ ID NO: 16254,
SEQ ID NO: 16255, SEQ ID NO: 16257, SEQ ID NO: 16258, SEQ ID NO: 16259, SEQ ID
NO: 16260, SEQ ID NO: 16261, SEQ ID NO: 16262, SEQ ID NO: 16264, SEQ ID NO: 16265,
SEQ ID NO: 16266, SEQ ID NO: 16267, SEQ ID NO: 16268, SEQ ID NO: 16269, SEQ ID
NO: 16270, SEQ ID NO: 16272, SEQ ID NO: 16273, SEQ ID NO: 16274, SEQ ID NO: 16275,
SEQ ID NO: 16276, SEQ ID NO: 16277, SEQ ID NO: 16278, SEQ ID NO: 16279, SEQ ID
NO: 16280, SEQ ID NO: 16281, SEQ ID NO: 16282, SEQ ID NO: 16283, SEQ ID NO: 16284,
SEQ ID NO: 16285, SEQ ID NO: 16286, SEQ ID NO: 16287, SEQ ID NO: 16289, SEQ ID
NO: 16291, SEQ ID NO: 16293, SEQ ID NO: 16294, SEQ ID NO: 16295, SEQ ID NO: 16297,
SEQ ID NO: 16299, SEQ ID NO: 16301, SEQ ID NO: 16303, SEQ ID NO: 16304, SEQ ID
NO: 16306, SEQ ID NO: 16307, SEQ ID NO: 16308, SEQ ID NO: 16309, SEQ ID NO: 16310,
SEQ ID NO: 16312, SEQ ID NO: 16314, SEQ ID NO: 16315, SEQ ID NO: 16316, SEQ ID
NO: 16317, SEQ ID NO: 16318, SEQ ID NO: 16319, SEQ ID NO: 16320, SEQ ID NO: 16321,
SEQ ID NO: 16322, SEQ ID NO: 16323, SEQ ID NO: 16324, SEQ ID NO: 16325, SEQ ID
NO: 16327, SEQ ID NO: 16328, SEQ ID NO: 16330, SEQ ID NO: 16331, SEQ ID NO: 16332,
SEQ ID NO: 16333, SEQ ID NO: 16334, SEQ ID NO: 16336, SEQ ID NO: 16337, SEQ ID
NO: 16338, SEQ ID NO: 16339, SEQ ID NO: 16340, SEQ ID NO: 16341, SEQ ID NO: 16343,
SEQ ID NO: 16345,
SEQ ID NO: 16346, SEQ ID NO: 16347, SEQ ID NO: 16348, SEQ ID NO: 16350, SEQ ID
NO: 16351, SEQ ID NO: 16352, SEQ ID NO: 16353, SEQ ID NO: 16354, SEQ ID NO: 16356,
SEQ ID NO: 16358, SEQ ID NO: 16359, SEQ ID NO: 16360, SEQ ID NO: 16362, SEQ ID
NO: 16363, SEQ ID NO: 16364, SEQ ID NO: 16365, SEQ ID NO: 16367, SEQ ID NO: 16369,
SEQ ID NO: 16371, SEQ ID NO: 16372, SEQ ID NO: 16373, SEQ ID NO: 16374, SEQ ID
NO: 16376, SEQ ID NO: 16377, SEQ ID NO: 16378, SEQ ID NO: 16379, SEQ ID NO: 16380,
SEQ ID NO: 16381, SEQ ID NO: 16383, SEQ ID NO: 16384, SEQ ID NO: 16385, SEQ ID
NO: 16387, SEQ ID NO: 16389, SEQ ID NO: 16391, SEQ ID NO: 16393, SEQ ID NO: 16394,
SEQ ID NO: 16395, SEQ ID NO: 16396, SEQ ID NO: 16398, SEQ ID NO: 16400, SEQ ID
NO: 16401, SEQ ID NO: 16402, SEQ ID NO: 16403, SEQ ID NO: 16404, SEQ ID NO: 16406,
SEQ ID NO: 16408, SEQ ID NO: 16409, SEQ ID NO: 16411, SEQ ID NO: 16412, SEQ ID
NO: 16414, SEQ ID NO: 16416, SEQ ID NO: 16418, SEQ ID NO: 16420, SEQ ID NO: 16421,
SEQ ID NO: 16422, SEQ ID NO: 16423, SEQ ID NO: 16424, SEQ ID NO: 16425, SEQ ID
 
NO: 16426, SEQ ID NO: 16427, SEQ ID NO: 16428, SEQ ID NO: 16429, SEQ ID NO: 16431,
SEQ ID NO: 16433, SEQ ID NO: 16435, SEQ ID NO: 16436, SEQ ID NO: 16437, SEQ ID
NO: 16438, SEQ ID NO: 16440, SEQ ID NO: 16441, SEQ ID NO: 16443, SEQ ID NO: 16445,
SEQ ID NO: 16447, SEQ ID NO: 16448, SEQ ID NO: 16449, SEQ ID NO: 16450, SEQ ID
NO: 16451, SEQ ID NO: 16452, SEQ ID NO: 16453, SEQ ID NO: 16454, SEQ ID NO: 16455,
SEQ ID NO: 16456, SEQ ID NO: 16457, SEQ ID NO: 16458, SEQ ID NO: 16459, SEQ ID
NO: 16460, SEQ ID NO: 16461, SEQ ID NO: 16462, SEQ ID NO: 16464, SEQ ID NO: 16465,
SEQ ID NO: 16467, SEQ ID NO: 16469, SEQ ID NO: 16471, SEQ ID NO: 16473, SEQ ID
NO: 16474, SEQ ID NO: 16476, SEQ ID NO: 16478, SEQ ID NO: 16479, SEQ ID NO: 16481,
SEQ ID NO: 16483,
SEQ ID NO: 16484, SEQ ID NO: 16486, SEQ ID NO: 16487, SEQ ID NO: 16489, SEQ ID
NO: 16490, SEQ ID NO: 16492, SEQ ID NO: 16494, SEQ ID NO: 16496, SEQ ID NO: 16497,
SEQ ID NO: 16499, SEQ ID NO: 16500, SEQ ID NO: 16501, SEQ ID NO: 16503, SEQ ID
NO: 16505, SEQ ID NO: 16506, SEQ ID NO: 16508, SEQ ID NO: 16509, SEQ ID NO: 16511,
SEQ ID NO: 16512, SEQ ID NO: 16513, SEQ ID NO: 16514, SEQ ID NO: 16515, SEQ ID
NO: 16516, SEQ ID NO: 16517, SEQ ID NO: 16518, SEQ ID NO: 16519, SEQ ID NO: 16520,
SEQ ID NO: 16522, SEQ ID NO: 16524, SEQ ID NO: 16526, SEQ ID NO: 16527, SEQ ID
NO: 16529, SEQ ID NO: 16531, SEQ ID NO: 16532, SEQ ID NO: 16534, SEQ ID NO: 16535,
SEQ ID NO: 16536, SEQ ID NO: 16537, SEQ ID NO: 16539, SEQ ID NO: 16540, SEQ ID
NO: 16542, SEQ ID NO: 16544, SEQ ID NO: 16546, SEQ ID NO: 16547, SEQ ID NO: 16548,
SEQ ID NO: 16550, SEQ ID NO: 16552, SEQ ID NO: 16553, SEQ ID NO: 16555, SEQ ID
NO: 16557, SEQ ID NO: 16559, SEQ ID NO: 16561, SEQ ID NO: 16563, SEQ ID NO: 16564,
SEQ ID NO: 16565, SEQ ID NO: 16567, SEQ ID NO: 16568, SEQ ID NO: 16569, SEQ ID
NO: 16570, SEQ ID NO: 16571, SEQ ID NO: 16573, SEQ ID NO: 16574, SEQ ID NO: 16575,
SEQ ID NO: 16576, SEQ ID NO: 16577, SEQ ID NO: 16578, SEQ ID NO: 16579, SEQ ID
NO: 16580, SEQ ID NO: 16581, SEQ ID NO: 16583, SEQ ID NO: 16585, SEQ ID NO: 16586,
SEQ ID NO: 16588, SEQ ID NO: 16589, SEQ ID NO: 16591, SEQ ID NO: 16593, SEQ ID
NO: 16594, SEQ ID NO: 16596, SEQ ID NO: 16597, SEQ ID NO: 16598, SEQ ID NO: 16600,
SEQ ID NO: 16601, SEQ ID NO: 16603, SEQ ID NO: 16604, SEQ ID NO: 16606, SEQ ID
NO: 16607, SEQ ID NO: 16608, SEQ ID NO: 16609, SEQ ID NO: 16611, SEQ ID NO: 16612,
SEQ ID NO: 16613, SEQ ID NO: 16614, SEQ ID NO: 16615, SEQ ID NO: 16617, SEQ ID
NO: 16618, SEQ ID NO: 16620, SEQ ID NO: 16622, SEQ ID NO: 16624, SEQ ID NO: 16626,
SEQ ID NO: 16627,
SEQ ID NO: 16628, SEQ ID NO: 16629, SEQ ID NO: 16631, SEQ ID NO: 16633, SEQ ID
NO: 16635, SEQ ID NO: 16636, SEQ ID NO: 16637, SEQ ID NO: 16639, SEQ ID NO: 16640,
SEQ ID NO: 16642, SEQ ID NO: 16643, SEQ ID NO: 16645, SEQ ID NO: 16646, SEQ ID
NO: 16647, SEQ ID NO: 16648, SEQ ID NO: 16649, SEQ ID NO: 16650, SEQ ID NO: 16651,
SEQ ID NO: 16652, SEQ ID NO: 16653, SEQ ID NO: 16654, SEQ ID NO: 16655, SEQ ID
NO: 16656, SEQ ID NO: 16657, SEQ ID NO: 16658, SEQ ID NO: 16659, SEQ ID NO: 16660,
SEQ ID NO: 16661, SEQ ID NO: 16662, SEQ ID NO: 16664, SEQ ID NO: 16666, SEQ ID
NO: 16667, SEQ ID NO: 16668, SEQ ID NO: 16669, SEQ ID NO: 16670, SEQ ID NO: 16671,
SEQ ID NO: 16672, SEQ ID NO: 16673, SEQ ID NO: 16675, SEQ ID NO: 16676, SEQ ID
NO: 16677, SEQ ID NO: 16678, SEQ ID NO: 16679, SEQ ID NO: 16681, SEQ ID NO: 16682,
SEQ ID NO: 16683, SEQ ID NO: 16684, SEQ ID NO: 16686, SEQ ID NO: 16687, SEQ ID
NO: 16689, SEQ ID NO: 16691, SEQ ID NO: 16692, SEQ ID NO: 16694, SEQ ID NO: 16695,
SEQ ID NO: 16696, SEQ ID NO: 16697, SEQ ID NO: 16698, SEQ ID NO: 16700, SEQ ID
NO: 16701, SEQ ID NO: 16703, SEQ ID NO: 16705, SEQ ID NO: 16706, SEQ ID NO: 16707,
SEQ ID NO: 16709, SEQ ID NO: 16710, SEQ ID NO: 16711, SEQ ID NO: 16713, SEQ ID
NO: 16715, SEQ ID NO: 16716, SEQ ID NO: 16717, SEQ ID NO: 16718, SEQ ID NO: 16719,
SEQ ID NO: 16720, SEQ ID NO: 16721, SEQ ID NO: 16722, SEQ ID NO: 16724, SEQ ID
NO: 16725, SEQ ID NO: 16727, SEQ ID NO: 16729, SEQ ID NO: 16731, SEQ ID NO: 16733,
SEQ ID NO: 16734, SEQ ID NO: 16735, SEQ ID NO: 16736, SEQ ID NO: 16737, SEQ ID
 
NO: 16738, SEQ ID NO: 16739, SEQ ID NO: 16740, SEQ ID NO: 16742, SEQ ID NO: 16744,
SEQ ID NO: 16746, SEQ ID NO: 16748, SEQ ID NO: 16749, SEQ ID NO: 16750, SEQ ID
NO: 16751, SEQ ID NO: 16753, SEQ ID NO: 16754, SEQ ID NO: 16755, SEQ ID NO: 16756,
SEQ ID NO: 16757,
SEQ ID NO: 16758, SEQ ID NO: 16759, SEQ ID NO: 16760, SEQ ID NO: 16761, SEQ ID
NO: 16762, SEQ ID NO: 16764, SEQ ID NO: 16766, SEQ ID NO: 16768, SEQ ID NO: 16769,
SEQ ID NO: 16770, SEQ ID NO: 16772, SEQ ID NO: 16773, SEQ ID NO: 16774, SEQ ID
NO: 16775, SEQ ID NO: 16777, SEQ ID NO: 16778, SEQ ID NO: 16779, SEQ ID NO: 16780,
SEQ ID NO: 16782, SEQ ID NO: 16783, SEQ ID NO: 16785, SEQ ID NO: 16787, SEQ ID
NO: 16789, SEQ ID NO: 16790, SEQ ID NO: 16792, SEQ ID NO: 16794, SEQ ID NO: 16796,
SEQ ID NO: 16798, SEQ ID NO: 16799, SEQ ID NO: 16800, SEQ ID NO: 16802, SEQ ID
NO: 16803, SEQ ID NO: 16805, SEQ ID NO: 16806, SEQ ID NO: 16808, SEQ ID NO: 16810,
SEQ ID NO: 16811, SEQ ID NO: 16812, SEQ ID NO: 16814, SEQ ID NO: 16816, SEQ ID
NO: 16817, SEQ ID NO: 16819, SEQ ID NO: 16820, SEQ ID NO: 16821, SEQ ID NO: 16823,
SEQ ID NO: 16824, SEQ ID NO: 16826, SEQ ID NO: 16827, SEQ ID NO: 16828, SEQ ID
NO: 16829, SEQ ID NO: 16830, SEQ ID NO: 16831, SEQ ID NO: 16832, SEQ ID NO: 16833,
SEQ ID NO: 16834, SEQ ID NO: 16835, SEQ ID NO: 16836, SEQ ID NO: 16837, SEQ ID
NO: 16838, SEQ ID NO: 16839, SEQ ID NO: 16840, SEQ ID NO: 16841, SEQ ID NO: 16842,
SEQ ID NO: 16843, SEQ ID NO: 16844, SEQ ID NO: 16846, SEQ ID NO: 16848, SEQ ID
NO: 16850, SEQ ID NO: 16851, SEQ ID NO: 16852, SEQ ID NO: 16854, SEQ ID NO: 16856,
SEQ ID NO: 16858, SEQ ID NO: 16859, SEQ ID NO: 16860, SEQ ID NO: 16861, SEQ ID
NO: 16862, SEQ ID NO: 16864, SEQ ID NO: 16866, SEQ ID NO: 16867, SEQ ID NO: 16868,
SEQ ID NO: 16869, SEQ ID NO: 16871, SEQ ID NO: 16872, SEQ ID NO: 16873, SEQ ID
NO: 16874, SEQ ID NO: 16875, SEQ ID NO: 16877, SEQ ID NO: 16878, SEQ ID NO: 16880,
SEQ ID NO: 16881, SEQ ID NO: 16882, SEQ ID NO: 16883, SEQ ID NO: 16885, SEQ ID
NO: 16886, SEQ ID NO: 16887, SEQ ID NO: 16888, SEQ ID NO: 16889, SEQ ID NO: 16890,
SEQ ID NO: 16891,
SEQ ID NO: 16893, SEQ ID NO: 16894, SEQ ID NO: 16895, SEQ ID NO: 16897, SEQ ID
NO: 16898, SEQ ID NO: 16899, SEQ ID NO: 16900, SEQ ID NO: 16902, SEQ ID NO: 16904,
SEQ ID NO: 16905, SEQ ID NO: 16906, SEQ ID NO: 16907, SEQ ID NO: 16908, SEQ ID
NO: 16910, SEQ ID NO: 16911, SEQ ID NO: 16912, SEQ ID NO: 16913, SEQ ID NO: 16914,
SEQ ID NO: 16915, SEQ ID NO: 16916, SEQ ID NO: 16917, SEQ ID NO: 16918, SEQ ID
NO: 16919, SEQ ID NO: 16920, SEQ ID NO: 16922, SEQ ID NO: 16923, SEQ ID NO: 16924,
SEQ ID NO: 16926, SEQ ID NO: 16927, SEQ ID NO: 16929, SEQ ID NO: 16930, SEQ ID
NO: 16931, SEQ ID NO: 16932, SEQ ID NO: 16933, SEQ ID NO: 16934, SEQ ID NO: 16936,
SEQ ID NO: 16938, SEQ ID NO: 16940, SEQ ID NO: 16942, SEQ ID NO: 16944, SEQ ID
NO: 16945, SEQ ID NO: 16946, SEQ ID NO: 16948, SEQ ID NO: 16949, SEQ ID NO: 16950,
SEQ ID NO: 16951, SEQ ID NO: 16952, SEQ ID NO: 16953, SEQ ID NO: 16955, SEQ ID
NO: 16957, SEQ ID NO: 16958, SEQ ID NO: 16959, SEQ ID NO: 16961, SEQ ID NO: 16962,
SEQ ID NO: 16964, SEQ ID NO: 16966, SEQ ID NO: 16967, SEQ ID NO: 16969, SEQ ID
NO: 16970, SEQ ID NO: 16972, SEQ ID NO: 16973, SEQ ID NO: 16974, SEQ ID NO: 16975,
SEQ ID NO: 16977, SEQ ID NO: 16978, SEQ ID NO: 16979, SEQ ID NO: 16981, SEQ ID
NO: 16982, SEQ ID NO: 16983, SEQ ID NO: 16984, SEQ ID NO: 16985, SEQ ID NO: 16986,
SEQ ID NO: 16988, SEQ ID NO: 16989, SEQ ID NO: 16991, SEQ ID NO: 16992, SEQ ID
NO: 16993, SEQ ID NO: 16994, SEQ ID NO: 16995, SEQ ID NO: 16996, SEQ ID NO: 16997,
SEQ ID NO: 16998, SEQ ID NO: 17000, SEQ ID NO: 17001, SEQ ID NO: 17002, SEQ ID
NO: 17004, SEQ ID NO: 17005, SEQ ID NO: 17006, SEQ ID NO: 17007, SEQ ID NO: 17008,
SEQ ID NO: 17009, SEQ ID NO: 17010, SEQ ID NO: 17011, SEQ ID NO: 17012, SEQ ID
NO: 17013, SEQ ID NO: 17014, SEQ ID NO: 17015, SEQ ID NO: 17016, SEQ ID NO: 17017,
SEQ ID NO: 17018,
SEQ ID NO: 17020, SEQ ID NO: 17021, SEQ ID NO: 17022, SEQ ID NO: 17023, SEQ ID
NO: 17024, SEQ ID NO: 17026, SEQ ID NO: 17027, SEQ ID NO: 17029, SEQ ID NO: 17030,
 
SEQ ID NO: 17031, SEQ ID NO: 17033, SEQ ID NO: 17034, SEQ ID NO: 17035, SEQ ID
NO: 17036, SEQ ID NO: 17037, SEQ ID NO: 17039, SEQ ID NO: 17041, SEQ ID NO: 17043,
SEQ ID NO: 17044, SEQ ID NO: 17045, SEQ ID NO: 17046, SEQ ID NO: 17047, SEQ ID
NO: 17048, SEQ ID NO: 17049, SEQ ID NO: 17050, SEQ ID NO: 17052, SEQ ID NO: 17054,
SEQ ID NO: 17055, SEQ ID NO: 17056, SEQ ID NO: 17057, SEQ ID NO: 17059, SEQ ID
NO: 17061, SEQ ID NO: 17063, SEQ ID NO: 17065, SEQ ID NO: 17066, SEQ ID NO: 17067,
SEQ ID NO: 17068, SEQ ID NO: 17069, SEQ ID NO: 17070, SEQ ID NO: 17071, SEQ ID
NO: 17073, SEQ ID NO: 17075, SEQ ID NO: 17077, SEQ ID NO: 17078, SEQ ID NO: 17080,
SEQ ID NO: 17082, SEQ ID NO: 17084, SEQ ID NO: 17086, SEQ ID NO: 17088, SEQ ID
NO: 17090, SEQ ID NO: 17092, SEQ ID NO: 17094, SEQ ID NO: 17096, SEQ ID NO: 17098,
SEQ ID NO: 17100, SEQ ID NO: 17102, SEQ ID NO: 17103, SEQ ID NO: 17105, SEQ ID
NO: 17107, SEQ ID NO: 17108, SEQ ID NO: 17110, SEQ ID NO: 17112, SEQ ID NO: 17114,
SEQ ID NO: 17116, SEQ ID NO: 17118, SEQ ID NO: 17119, SEQ ID NO: 17121, SEQ ID
NO: 17123, SEQ ID NO: 17125, SEQ ID NO: 17126, SEQ ID NO: 17128, SEQ ID NO: 17130,
SEQ ID NO: 17132, SEQ ID NO: 17134, SEQ ID NO: 17136, SEQ ID NO: 17138, SEQ ID
NO: 17139, SEQ ID NO: 17141, SEQ ID NO: 17143, SEQ ID NO: 17145, SEQ ID NO: 17147,
SEQ ID NO: 17149, SEQ ID NO: 17151, SEQ ID NO: 17153, SEQ ID NO: 17155, SEQ ID
NO: 17157, SEQ ID NO: 17159, SEQ ID NO: 17160, SEQ ID NO: 17162, SEQ ID NO: 17164,
SEQ ID NO: 17166, SEQ ID NO: 17168, SEQ ID NO: 17170, SEQ ID NO: 17172, SEQ ID
NO: 17174, SEQ ID NO: 17176, SEQ ID NO: 17178, SEQ ID NO: 17180, SEQ ID NO: 17182,
SEQ ID NO: 17183,
SEQ ID NO: 17185, SEQ ID NO: 17187, SEQ ID NO: 17189, SEQ ID NO: 17190, SEQ ID
NO: 17192, SEQ ID NO: 17193, SEQ ID NO: 17195, SEQ ID NO: 17197, SEQ ID NO: 17199,
SEQ ID NO: 17201, SEQ ID NO: 17202, SEQ ID NO: 17204, SEQ ID NO: 17206, SEQ ID
NO: 17208, SEQ ID NO: 17209, SEQ ID NO: 17210, SEQ ID NO: 17212, SEQ ID NO: 17213,
SEQ ID NO: 17214, SEQ ID NO: 17216, SEQ ID NO: 17218, SEQ ID NO: 17220, SEQ ID
NO: 17222, SEQ ID NO: 17224, SEQ ID NO: 17226, SEQ ID NO: 17228, SEQ ID NO: 17230,
SEQ ID NO: 17232, SEQ ID NO: 17234, SEQ ID NO: 17236, SEQ ID NO: 17238, SEQ ID
NO: 17240, SEQ ID NO: 17242, SEQ ID NO: 17244, SEQ ID NO: 17246, SEQ ID NO: 17248,
SEQ ID NO: 17249, SEQ ID NO: 17251, SEQ ID NO: 17253, SEQ ID NO: 17255, SEQ ID
NO: 17256, SEQ ID NO: 17258, SEQ ID NO: 17259, SEQ ID NO: 17261, SEQ ID NO: 17263,
SEQ ID NO: 17264, SEQ ID NO: 17265, SEQ ID NO: 17267, SEQ ID NO: 17268, SEQ ID
NO: 17270, SEQ ID NO: 17272, SEQ ID NO: 17274, SEQ ID NO: 17275, SEQ ID NO: 17277,
SEQ ID NO: 17279, SEQ ID NO: 17281, SEQ ID NO: 17283, SEQ ID NO: 17285, SEQ ID
NO: 17287, SEQ ID NO: 17289, SEQ ID NO: 17290, SEQ ID NO: 17292, SEQ ID NO: 17294,
SEQ ID NO: 17296, SEQ ID NO: 17297, SEQ ID NO: 17299, SEQ ID NO: 17300, SEQ ID
NO: 17302, SEQ ID NO: 17304, SEQ ID NO: 17305, SEQ ID NO: 17307, SEQ ID NO: 17309,
SEQ ID NO: 17310, SEQ ID NO: 17312, SEQ ID NO: 17314, SEQ ID NO: 17315, SEQ ID
NO: 17316, SEQ ID NO: 17318, SEQ ID NO: 17320, SEQ ID NO: 17322, SEQ ID NO: 17324,
SEQ ID NO: 17326, SEQ ID NO: 17328, SEQ ID NO: 17330, SEQ ID NO: 17332, SEQ ID
NO: 17334, SEQ ID NO: 17336, SEQ ID NO: 17338, SEQ ID NO: 17340, SEQ ID NO: 17341,
SEQ ID NO: 17343, SEQ ID NO: 17344, SEQ ID NO: 17345, SEQ ID NO: 17347, SEQ ID
NO: 17349, SEQ ID NO: 17351, SEQ ID NO: 17353, SEQ ID NO: 17355, SEQ ID NO: 17356,
SEQ ID NO: 17358,
SEQ ID NO: 17360, SEQ ID NO: 17362, SEQ ID NO: 17364, SEQ ID NO: 17365, SEQ ID
NO: 17366, SEQ ID NO: 17368, SEQ ID NO: 17370, SEQ ID NO: 17371, SEQ ID NO: 17372,
SEQ ID NO: 17374, SEQ ID NO: 17376, SEQ ID NO: 17377, SEQ ID NO: 17378, SEQ ID
NO: 17380, SEQ ID NO: 17381, SEQ ID NO: 17383, SEQ ID NO: 17385, SEQ ID NO: 17387,
SEQ ID NO: 17388, SEQ ID NO: 17390, SEQ ID NO: 17392, SEQ ID NO: 17393, SEQ ID
NO: 17395, SEQ ID NO: 17397, SEQ ID NO: 17398, SEQ ID NO: 17399, SEQ ID NO: 17401,
SEQ ID NO: 17402, SEQ ID NO: 17404, SEQ ID NO: 17406, SEQ ID NO: 17408, SEQ ID
NO: 17410, SEQ ID NO: 17412, SEQ ID NO: 17414, SEQ ID NO: 17416, SEQ ID NO: 17418,
 
SEQ ID NO: 17420, SEQ ID NO: 17422, SEQ ID NO: 17424, SEQ ID NO: 17426, SEQ ID
NO: 17427, SEQ ID NO: 17429, SEQ ID NO: 17430, SEQ ID NO: 17432, SEQ ID NO: 17433,
SEQ ID NO: 17434, SEQ ID NO: 17436, SEQ ID NO: 17437, SEQ ID NO: 17438, SEQ ID
NO: 17440, SEQ ID NO: 17442, SEQ ID NO: 17443, SEQ ID NO: 17445, SEQ ID NO: 17447,
SEQ ID NO: 17449, SEQ ID NO: 17450, SEQ ID NO: 17452, SEQ ID NO: 17454, SEQ ID
NO: 17456, SEQ ID NO: 17457, SEQ ID NO: 17459, SEQ ID NO: 17460, SEQ ID NO: 17461,
SEQ ID NO: 17463, SEQ ID NO: 17465, SEQ ID NO: 17467, SEQ ID NO: 17468, SEQ ID
NO: 17470, SEQ ID NO: 17472, SEQ ID NO: 17474, SEQ ID NO: 17475, SEQ ID NO: 17477,
SEQ ID NO: 17479, SEQ ID NO: 17480, SEQ ID NO: 17482, SEQ ID NO: 17484, SEQ ID
NO: 17485, SEQ ID NO: 17487, SEQ ID NO: 17489, SEQ ID NO: 17490, SEQ ID NO: 17492,
SEQ ID NO: 17494, SEQ ID NO: 17496, SEQ ID NO: 17498, SEQ ID NO: 17500, SEQ ID
NO: 17502, SEQ ID NO: 17504, SEQ ID NO: 17506, SEQ ID NO: 17508, SEQ ID NO: 17510,
SEQ ID NO: 17512, SEQ ID NO: 17514, SEQ ID NO: 17516, SEQ ID NO: 17518, SEQ ID
NO: 17520, SEQ ID NO: 17522, SEQ ID NO: 17524, SEQ ID NO: 17526, SEQ ID NO: 17527,
SEQ ID NO: 17529,
SEQ ID NO: 17531, SEQ ID NO: 17532, SEQ ID NO: 17533, SEQ ID NO: 17535, SEQ ID
NO: 17537, SEQ ID NO: 17539, SEQ ID NO: 17541, SEQ ID NO: 17543, SEQ ID NO: 17545,
SEQ ID NO: 17547, SEQ ID NO: 17549, SEQ ID NO: 17551, SEQ ID NO: 17553, SEQ ID
NO: 17555, SEQ ID NO: 17557, SEQ ID NO: 17559, SEQ ID NO: 17561, SEQ ID NO: 17563,
SEQ ID NO: 17565, SEQ ID NO: 17567, SEQ ID NO: 17569, SEQ ID NO: 17571, SEQ ID
NO: 17572, SEQ ID NO: 17574, SEQ ID NO: 17576, SEQ ID NO: 17578, SEQ ID NO: 17579,
SEQ ID NO: 17581, SEQ ID NO: 17583, SEQ ID NO: 17585, SEQ ID NO: 17586, SEQ ID
NO: 17588, SEQ ID NO: 17589, SEQ ID NO: 17591, SEQ ID NO: 17593, SEQ ID NO: 17594,
SEQ ID NO: 17596, SEQ ID NO: 17598, SEQ ID NO: 17600, SEQ ID NO: 17602, SEQ ID
NO: 17604, SEQ ID NO: 17606, SEQ ID NO: 17608, SEQ ID NO: 17610, SEQ ID NO: 17612,
SEQ ID NO: 17614, SEQ ID NO: 17616, SEQ ID NO: 17618, SEQ ID NO: 17620, SEQ ID
NO: 17622, SEQ ID NO: 17624, SEQ ID NO: 17626, SEQ ID NO: 17628, SEQ ID NO: 17630,
SEQ ID NO: 17632, SEQ ID NO: 17634, SEQ ID NO: 17636, SEQ ID NO: 17638, SEQ ID
NO: 17640, SEQ ID NO: 17642, SEQ ID NO: 17644, SEQ ID NO: 17646, SEQ ID NO: 17648,
SEQ ID NO: 17649, SEQ ID NO: 17650, SEQ ID NO: 17652, SEQ ID NO: 17654, SEQ ID
NO: 17656, SEQ ID NO: 17657, SEQ ID NO: 17659, SEQ ID NO: 17660, SEQ ID NO: 17662,
SEQ ID NO: 17663, SEQ ID NO: 17665, SEQ ID NO: 17666, SEQ ID NO: 17668, SEQ ID
NO: 17670, SEQ ID NO: 17672, SEQ ID NO: 17674, SEQ ID NO: 17676, SEQ ID NO: 17678,
SEQ ID NO: 17680, SEQ ID NO: 17681, SEQ ID NO: 17682, SEQ ID NO: 17683, SEQ ID
NO: 17685, SEQ ID NO: 17687, SEQ ID NO: 17689, SEQ ID NO: 17691, SEQ ID NO: 17693,
SEQ ID NO: 17695, SEQ ID NO: 17697, SEQ ID NO: 17699, SEQ ID NO: 17701, SEQ ID
NO: 17703, SEQ ID NO: 17705, SEQ ID NO: 17706, SEQ ID NO: 17708, SEQ ID NO: 17710,
SEQ ID NO: 17712,
SEQ ID NO: 17714, SEQ ID NO: 17716, SEQ ID NO: 17717, SEQ ID NO: 17718, SEQ ID
NO: 17720, SEQ ID NO: 17721, SEQ ID NO: 17722, SEQ ID NO: 17723, SEQ ID NO: 17724,
SEQ ID NO: 17726, SEQ ID NO: 17728, SEQ ID NO: 17729, SEQ ID NO: 17730, SEQ ID
NO: 17732, SEQ ID NO: 17733, SEQ ID NO: 17735, SEQ ID NO: 17737, SEQ ID NO: 17739,
SEQ ID NO: 17740, SEQ ID NO: 17742, SEQ ID NO: 17744, SEQ ID NO: 17746, SEQ ID
NO: 17747, SEQ ID NO: 17748, SEQ ID NO: 17750, SEQ ID NO: 17751, SEQ ID NO: 17753,
SEQ ID NO: 17754, SEQ ID NO: 17756, SEQ ID NO: 17758, SEQ ID NO: 17759, SEQ ID
NO: 17761, SEQ ID NO: 17762, SEQ ID NO: 17763, SEQ ID NO: 17764, SEQ ID NO: 17765,
SEQ ID NO: 17767, SEQ ID NO: 17768, SEQ ID NO: 17769, SEQ ID NO: 17770, SEQ ID
NO: 17771, SEQ ID NO: 17773, SEQ ID NO: 17774, SEQ ID NO: 17776, SEQ ID NO: 17778,
SEQ ID NO: 17780, SEQ ID NO: 17781, SEQ ID NO: 17783, SEQ ID NO: 17785, SEQ ID
NO: 17787, SEQ ID NO: 17789, SEQ ID NO: 17790, SEQ ID NO: 17792, SEQ ID NO: 17794,
SEQ ID NO: 17795, SEQ ID NO: 17797, SEQ ID NO: 17799, SEQ ID NO: 17801, SEQ ID
NO: 17802, SEQ ID NO: 17803, SEQ ID NO: 17805, SEQ ID NO: 17806, SEQ ID NO: 17807,
 
SEQ ID NO: 17809, SEQ ID NO: 17811, SEQ ID NO: 17813, SEQ ID NO: 17815, SEQ ID
NO: 17817, SEQ ID NO: 17818, SEQ ID NO: 17820, SEQ ID NO: 17822, SEQ ID NO: 17824,
SEQ ID NO: 17826, SEQ ID NO: 17828, SEQ ID NO: 17830, SEQ ID NO: 17831, SEQ ID
NO: 17832, SEQ ID NO: 17834, SEQ ID NO: 17836, SEQ ID NO: 17837, SEQ ID NO: 17839,
SEQ ID NO: 17841, SEQ ID NO: 17843, SEQ ID NO: 17845, SEQ ID NO: 17847, SEQ ID
NO: 17848, SEQ ID NO: 17849, SEQ ID NO: 17850, SEQ ID NO: 17851, SEQ ID NO: 17852,
SEQ ID NO: 17853, SEQ ID NO: 17855, SEQ ID NO: 17856, SEQ ID NO: 17858, SEQ ID
NO: 17859, SEQ ID NO: 17860, SEQ ID NO: 17861, SEQ ID NO: 17863, SEQ ID NO: 17865,
SEQ ID NO: 17867,
SEQ ID NO: 17869, SEQ ID NO: 17870, SEQ ID NO: 17871, SEQ ID NO: 17872, SEQ ID
NO: 17874, SEQ ID NO: 17875, SEQ ID NO: 17876, SEQ ID NO: 17877, SEQ ID NO: 17878,
SEQ ID NO: 17880, SEQ ID NO: 17881, SEQ ID NO: 17883, SEQ ID NO: 17885, SEQ ID
NO: 17886, SEQ ID NO: 17888, SEQ ID NO: 17890, SEQ ID NO: 17892, SEQ ID NO: 17894,
SEQ ID NO: 17895, SEQ ID NO: 17896, SEQ ID NO: 17898, SEQ ID NO: 17900, SEQ ID
NO: 17902, SEQ ID NO: 17903, SEQ ID NO: 17904, SEQ ID NO: 17906, SEQ ID NO: 17907,
SEQ ID NO: 17909, SEQ ID NO: 17911, SEQ ID NO: 17912, SEQ ID NO: 17914, SEQ ID
NO: 17916, SEQ ID NO: 17917, SEQ ID NO: 17918, SEQ ID NO: 17919, SEQ ID NO: 17920,
SEQ ID NO: 17922, SEQ ID NO: 17924, SEQ ID NO: 17925, SEQ ID NO: 17926, SEQ ID
NO: 17928, SEQ ID NO: 17929, SEQ ID NO: 17931, SEQ ID NO: 17932, SEQ ID NO: 17934,
SEQ ID NO: 17936, SEQ ID NO: 17938, SEQ ID NO: 17940, SEQ ID NO: 17941, SEQ ID
NO: 17942, SEQ ID NO: 17943, SEQ ID NO: 17945, SEQ ID NO: 17946, SEQ ID NO: 17947,
SEQ ID NO: 17949, SEQ ID NO: 17951, SEQ ID NO: 17952, SEQ ID NO: 17954, SEQ ID
NO: 17956, SEQ ID NO: 17958, SEQ ID NO: 17959, SEQ ID NO: 17961, SEQ ID NO: 17962,
SEQ ID NO: 17964, SEQ ID NO: 17965, SEQ ID NO: 17966, SEQ ID NO: 17967, SEQ ID
NO: 17969, SEQ ID NO: 17971, SEQ ID NO: 17973, SEQ ID NO: 17974, SEQ ID NO: 17976,
SEQ ID NO: 17978, SEQ ID NO: 17979, SEQ ID NO: 17980, SEQ ID NO: 17981, SEQ ID
NO: 17982, SEQ ID NO: 17984, SEQ ID NO: 17985, SEQ ID NO: 17986, SEQ ID NO: 17987,
SEQ ID NO: 17989, SEQ ID NO: 17990, SEQ ID NO: 17991, SEQ ID NO: 17993, SEQ ID
NO: 17995, SEQ ID NO: 17997, SEQ ID NO: 17999, SEQ ID NO: 18000, SEQ ID NO: 18001,
SEQ ID NO: 18003, SEQ ID NO: 18004, SEQ ID NO: 18005, SEQ ID NO: 18006, SEQ ID
NO: 18008, SEQ ID NO: 18010, SEQ ID NO: 18012, SEQ ID NO: 18013, SEQ ID NO: 18015,
SEQ ID NO: 18016,
SEQ ID NO: 18017, SEQ ID NO: 18019, SEQ ID NO: 18021, SEQ ID NO: 18022, SEQ ID
NO: 18024, SEQ ID NO: 18026, SEQ ID NO: 18028, SEQ ID NO: 18030, SEQ ID NO: 18032,
SEQ ID NO: 18034, SEQ ID NO: 18036, SEQ ID NO: 18038, SEQ ID NO: 18040, SEQ ID
NO: 18042, SEQ ID NO: 18043, SEQ ID NO: 18044, SEQ ID NO: 18045, SEQ ID NO: 18046,
SEQ ID NO: 18048, SEQ ID NO: 18050, SEQ ID NO: 18052, SEQ ID NO: 18054, SEQ ID
NO: 18056, SEQ ID NO: 18058, SEQ ID NO: 18059, SEQ ID NO: 18061, SEQ ID NO: 18063,
SEQ ID NO: 18065, SEQ ID NO: 18066, SEQ ID NO: 18068, SEQ ID NO: 18070, SEQ ID
NO: 18071, SEQ ID NO: 18072, SEQ ID NO: 18074, SEQ ID NO: 18076, SEQ ID NO: 18078,
SEQ ID NO: 18080, SEQ ID NO: 18082, SEQ ID NO: 18084, SEQ ID NO: 18086, SEQ ID
NO: 18088, SEQ ID NO: 18090, SEQ ID NO: 18092, SEQ ID NO: 18094, SEQ ID NO: 18095,
SEQ ID NO: 18096, SEQ ID NO: 18098, SEQ ID NO: 18099, SEQ ID NO: 18101, SEQ ID
NO: 18103, SEQ ID NO: 18104, SEQ ID NO: 18105, SEQ ID NO: 18107, SEQ ID NO: 18109,
SEQ ID NO: 18110, SEQ ID NO: 18112, SEQ ID NO: 18114, SEQ ID NO: 18116, SEQ ID
NO: 18117, SEQ ID NO: 18119, SEQ ID NO: 18120, SEQ ID NO: 18121, SEQ ID NO: 18123,
SEQ ID NO: 18124, SEQ ID NO: 18125, SEQ ID NO: 18127, SEQ ID NO: 18129, SEQ ID
NO: 18131, SEQ ID NO: 18133, SEQ ID NO: 18135, SEQ ID NO: 18137, SEQ ID NO: 18139,
SEQ ID NO: 18140, SEQ ID NO: 18142, SEQ ID NO: 18144, SEQ ID NO: 18146, SEQ ID
NO: 18148, SEQ ID NO: 18150, SEQ ID NO: 18151, SEQ ID NO: 18152, SEQ ID NO: 18153,
SEQ ID NO: 18154, SEQ ID NO: 18156, SEQ ID NO: 18157, SEQ ID NO: 18159, SEQ ID
NO: 18161, SEQ ID NO: 18162, SEQ ID NO: 18163, SEQ ID NO: 18165, SEQ ID NO: 18166,
 
SEQ ID NO: 18168, SEQ ID NO: 18170, SEQ ID NO: 18172, SEQ ID NO: 18174, SEQ ID
NO: 18176, SEQ ID NO: 18178, SEQ ID NO: 18180, SEQ ID NO: 18182, SEQ ID NO: 18184,
SEQ ID NO: 18186,
SEQ ID NO: 18188, SEQ ID NO: 18190, SEQ ID NO: 18192, SEQ ID NO: 18194, SEQ ID
NO: 18196, SEQ ID NO: 18198, SEQ ID NO: 18200, SEQ ID NO: 18201, SEQ ID NO: 18203,
SEQ ID NO: 18205, SEQ ID NO: 18207, SEQ ID NO: 18209, SEQ ID NO: 18210, SEQ ID
NO: 18212, SEQ ID NO: 18214, SEQ ID NO: 18216, SEQ ID NO: 18218, SEQ ID NO: 18220,
SEQ ID NO: 18222, SEQ ID NO: 18224, SEQ ID NO: 18226, SEQ ID NO: 18227, SEQ ID
NO: 18229, SEQ ID NO: 18231, SEQ ID NO: 18233, SEQ ID NO: 18235, SEQ ID NO: 18236,
SEQ ID NO: 18238, SEQ ID NO: 18240, SEQ ID NO: 18242, SEQ ID NO: 18243, SEQ ID
NO: 18245, SEQ ID NO: 18247, SEQ ID NO: 18249, SEQ ID NO: 18251, SEQ ID NO: 18253,
SEQ ID NO: 18254, SEQ ID NO: 18256, SEQ ID NO: 18258, SEQ ID NO: 18259, SEQ ID
NO: 18260, SEQ ID NO: 18261, SEQ ID NO: 18263, SEQ ID NO: 18264, SEQ ID NO: 18265,
SEQ ID NO: 18266, SEQ ID NO: 18268, SEQ ID NO: 18270, SEQ ID NO: 18272, SEQ ID
NO: 18274, SEQ ID NO: 18276, SEQ ID NO: 18278, SEQ ID NO: 18280, SEQ ID NO: 18282,
SEQ ID NO: 18284, SEQ ID NO: 18286, SEQ ID NO: 18288, SEQ ID NO: 18289, SEQ ID
NO: 18291, SEQ ID NO: 18293, SEQ ID NO: 18295, SEQ ID NO: 18297, SEQ ID NO: 18298,
SEQ ID NO: 18300, SEQ ID NO: 18302, SEQ ID NO: 18303, SEQ ID NO: 18305, SEQ ID
NO: 18307, SEQ ID NO: 18308, SEQ ID NO: 18309, SEQ ID NO: 18310, SEQ ID NO: 18312,
SEQ ID NO: 18314, SEQ ID NO: 18316, SEQ ID NO: 18318, SEQ ID NO: 18320, SEQ ID
NO: 18321, SEQ ID NO: 18323, SEQ ID NO: 18325, SEQ ID NO: 18327, SEQ ID NO: 18329,
SEQ ID NO: 18331, SEQ ID NO: 18332, SEQ ID NO: 18334, SEQ ID NO: 18335, SEQ ID
NO: 18337, SEQ ID NO: 18339, SEQ ID NO: 18341, SEQ ID NO: 18343, SEQ ID NO: 18345,
SEQ ID NO: 18347, SEQ ID NO: 18349, SEQ ID NO: 18351, SEQ ID NO: 18353, SEQ ID
NO: 18355, SEQ ID NO: 18357, SEQ ID NO: 18358, SEQ ID NO: 18360, SEQ ID NO: 18362,
SEQ ID NO: 18364,
SEQ ID NO: 18366, SEQ ID NO: 18368, SEQ ID NO: 18370, SEQ ID NO: 18372
SEQ ID NO: 18374, SEQ ID NO: 18375, SEQ ID NO: 18377, SEQ ID NO: 18378, SEQ ID
NO: 18379, SEQ ID NO: 18381, SEQ ID NO: 18383, SEQ ID NO: 18385, SEQ ID NO: 18387,
SEQ ID NO: 18389, SEQ ID NO: 18390, SEQ ID NO: 18392, SEQ ID NO: 18394, SEQ ID
NO: 18395, SEQ ID NO: 18397, SEQ ID NO: 18398, SEQ ID NO: 18399, SEQ ID NO: 18401,
SEQ ID NO: 18402, SEQ ID NO: 18404, SEQ ID NO: 18406, SEQ ID NO: 18408, SEQ ID
NO: 18410, SEQ ID NO: 18412, SEQ ID NO: 18413, SEQ ID NO: 18414, SEQ ID NO: 18416,
SEQ ID NO: 18418, SEQ ID NO: 18420, SEQ ID NO: 18421, SEQ ID NO: 18422, SEQ ID
NO: 18424, SEQ ID NO: 18426, SEQ ID NO: 18427, SEQ ID NO: 18428, SEQ ID NO: 18430,
SEQ ID NO: 18432, SEQ ID NO: 18433, SEQ ID NO: 18435, SEQ ID NO: 18437, SEQ ID
NO: 18439, SEQ ID NO: 18441, SEQ ID NO: 18443, SEQ ID NO: 18444, SEQ ID NO: 18446,
SEQ ID NO: 18447, SEQ ID NO: 18449, SEQ ID NO: 18451, SEQ ID NO: 18453, SEQ ID
NO: 18454, SEQ ID NO: 18456, SEQ ID NO: 18458, SEQ ID NO: 18460, SEQ ID NO: 18462,
SEQ ID NO: 18464, SEQ ID NO: 18465, SEQ ID NO: 18467, SEQ ID NO: 18468, SEQ ID
NO: 18470, SEQ ID NO: 18472, SEQ ID NO: 18474, SEQ ID NO: 18476, SEQ ID NO: 18478,
SEQ ID NO: 18480, SEQ ID NO: 18482, SEQ ID NO: 18484, SEQ ID NO: 18486, SEQ ID
NO: 18488, SEQ ID NO: 18489, SEQ ID NO: 18491, SEQ ID NO: 18493, SEQ ID NO: 18495,
SEQ ID NO: 18496, SEQ ID NO: 18497, SEQ ID NO: 18499, SEQ ID NO: 18500, SEQ ID
NO: 18501, SEQ ID NO: 18502, SEQ ID NO: 18503, SEQ ID NO: 18504, SEQ ID NO: 18505,
SEQ ID NO: 18507, SEQ ID NO: 18509, SEQ ID NO: 18511, SEQ ID NO: 18513, SEQ ID
NO: 18515, SEQ ID NO: 18517, SEQ ID NO: 18519, SEQ ID NO: 18521, SEQ ID NO: 18523,
SEQ ID NO: 18525, SEQ ID NO: 18527, SEQ ID NO: 18528, SEQ ID NO: 18530, SEQ ID
NO: 18531, SEQ ID NO: 18533, SEQ ID NO: 18534, SEQ ID NO: 18535, SEQ ID NO: 18537,
SEQ ID NO: 18539,
SEQ ID NO: 18540, SEQ ID NO: 18542, SEQ ID NO: 18543, SEQ ID NO: 18544, SEQ ID
NO: 18545, SEQ ID NO: 18547, SEQ ID NO: 18548, SEQ ID NO: 18549, SEQ ID NO: 18551,
 
SEQ ID NO: 18553, SEQ ID NO: 18555, SEQ ID NO: 18557, SEQ ID NO: 18559, SEQ ID
NO: 18560, SEQ ID NO: 18561, SEQ ID NO: 18563, SEQ ID NO: 18564, SEQ ID NO: 18565,
SEQ ID NO: 18566, SEQ ID NO: 18567, SEQ ID NO: 18569, SEQ ID NO: 18570, SEQ ID
NO: 18571, SEQ ID NO: 18573, SEQ ID NO: 18575, SEQ ID NO: 18577, SEQ ID NO: 18578,
SEQ ID NO: 18579, SEQ ID NO: 18581, SEQ ID NO: 18583, SEQ ID NO: 18584, SEQ ID
NO: 18585, SEQ ID NO: 18586, SEQ ID NO: 18588, SEQ ID NO: 18589, SEQ ID NO: 18591,
SEQ ID NO: 18593, SEQ ID NO: 18595, SEQ ID NO: 18597, SEQ ID NO: 18599, SEQ ID
NO: 18601, SEQ ID NO: 18603, SEQ ID NO: 18605, SEQ ID NO: 18607, SEQ ID NO: 18609,
SEQ ID NO: 18610, SEQ ID NO: 18612, SEQ ID NO: 18614, SEQ ID NO: 18616, SEQ ID
NO: 18618, SEQ ID NO: 18620, SEQ ID NO: 18621, SEQ ID NO: 18622, SEQ ID NO: 18624,
SEQ ID NO: 18626, SEQ ID NO: 18628, SEQ ID NO: 18630, SEQ ID NO: 18632, SEQ ID
NO: 18634, SEQ ID NO: 18636, SEQ ID NO: 18638, SEQ ID NO: 18640, SEQ ID NO: 18641,
SEQ ID NO: 18642, SEQ ID NO: 18643, SEQ ID NO: 18645, SEQ ID NO: 18647, SEQ ID
NO: 18648, SEQ ID NO: 18650, SEQ ID NO: 18652, SEQ ID NO: 18654, SEQ ID NO: 18656,
SEQ ID NO: 18658, SEQ ID NO: 18659, SEQ ID NO: 18661, SEQ ID NO: 18663, SEQ ID
NO: 18664, SEQ ID NO: 18666, SEQ ID NO: 18667, SEQ ID NO: 18669, SEQ ID NO: 18670,
SEQ ID NO: 18671, SEQ ID NO: 18673, SEQ ID NO: 18675, SEQ ID NO: 18677, SEQ ID
NO: 18679, SEQ ID NO: 18681, SEQ ID NO: 18683, SEQ ID NO: 18684, SEQ ID NO: 18685,
SEQ ID NO: 18687, SEQ ID NO: 18689, SEQ ID NO: 18690, SEQ ID NO: 18692, SEQ ID
NO: 18694, SEQ ID NO: 18696, SEQ ID NO: 18698, SEQ ID NO: 18700, SEQ ID NO: 18702,
SEQ ID NO: 18704,
SEQ ID NO: 18706, SEQ ID NO: 18707, SEQ ID NO: 18708, SEQ ID NO: 18710, SEQ ID
NO: 18712, SEQ ID NO: 18714, SEQ ID NO: 18716, SEQ ID NO: 18718, SEQ ID NO: 18719,
SEQ ID NO: 18721, SEQ ID NO: 18723, SEQ ID NO: 18725, SEQ ID NO: 18727, SEQ ID
NO: 18728, SEQ ID NO: 18730, SEQ ID NO: 18732, SEQ ID NO: 18734, SEQ ID NO: 18736,
SEQ ID NO: 18738, SEQ ID NO: 18740, SEQ ID NO: 18742, SEQ ID NO: 18744, SEQ ID
NO: 18746, SEQ ID NO: 18748, SEQ ID NO: 18750, SEQ ID NO: 18752, SEQ ID NO: 18754,
SEQ ID NO: 18755, SEQ ID NO: 18756, SEQ ID NO: 18757, SEQ ID NO: 18758, SEQ ID
NO: 18760, SEQ ID NO: 18761, SEQ ID NO: 18762, SEQ ID NO: 18764, SEQ ID NO: 18766,
SEQ ID NO: 18767, SEQ ID NO: 18768, SEQ ID NO: 18769, SEQ ID NO: 18771, SEQ ID
NO: 18772, SEQ ID NO: 18774, SEQ ID NO: 18776, SEQ ID NO: 18778, SEQ ID NO: 18780,
SEQ ID NO: 18782, SEQ ID NO: 18784, SEQ ID NO: 18786, SEQ ID NO: 18788, SEQ ID
NO: 18789, SEQ ID NO: 18791, SEQ ID NO: 18792, SEQ ID NO: 18794, SEQ ID NO: 18796,
SEQ ID NO: 18798, SEQ ID NO: 18799, SEQ ID NO: 18801, SEQ ID NO: 18803, SEQ ID
NO: 18805, SEQ ID NO: 18807, SEQ ID NO: 18808, SEQ ID NO: 18810, SEQ ID NO: 18812,
SEQ ID NO: 18814, SEQ ID NO: 18816, SEQ ID NO: 18818, SEQ ID NO: 18820, SEQ ID
NO: 18821, SEQ ID NO: 18823, SEQ ID NO: 18825, SEQ ID NO: 18827, SEQ ID NO: 18829,
SEQ ID NO: 18831, SEQ ID NO: 18833, SEQ ID NO: 18835, SEQ ID NO: 18837, SEQ ID
NO: 18838, SEQ ID NO: 18839, SEQ ID NO: 18841, SEQ ID NO: 18842, SEQ ID NO: 18844,
SEQ ID NO: 18845, SEQ ID NO: 18846, SEQ ID NO: 18848, SEQ ID NO: 18849, SEQ ID
NO: 18851, SEQ ID NO: 18852, SEQ ID NO: 18854, SEQ ID NO: 18856, SEQ ID NO: 18858,
SEQ ID NO: 18860, SEQ ID NO: 18861, SEQ ID NO: 18863, SEQ ID NO: 18864, SEQ ID
NO: 18866, SEQ ID NO: 18867, SEQ ID NO: 18869, SEQ ID NO: 18871, SEQ ID NO: 18872,
SEQ ID NO: 18873,
SEQ ID NO: 18875, SEQ ID NO: 18877, SEQ ID NO: 18878, SEQ ID NO: 18880, SEQ ID
NO: 18881, SEQ ID NO: 18883, SEQ ID NO: 18885, SEQ ID NO: 18886, SEQ ID NO: 18887,
SEQ ID NO: 18889, SEQ ID NO: 18890, SEQ ID NO: 18892, SEQ ID NO: 18893, SEQ ID
NO: 18895, SEQ ID NO: 18897, SEQ ID NO: 18899, SEQ ID NO: 18901, SEQ ID NO: 18903,
SEQ ID NO: 18904, SEQ ID NO: 18905, SEQ ID NO: 18907, SEQ ID NO: 18909, SEQ ID
NO: 18911, SEQ ID NO: 18913, SEQ ID NO: 18915, SEQ ID NO: 18917, SEQ ID NO: 18919,
SEQ ID NO: 18921, SEQ ID NO: 18923, SEQ ID NO: 18925, SEQ ID NO: 18927, SEQ ID
NO: 18928, SEQ ID NO: 18930, SEQ ID NO: 18932, SEQ ID NO: 18934, SEQ ID NO: 18936,
 
SEQ ID NO: 18938, SEQ ID NO: 18940, SEQ ID NO: 18941, SEQ ID NO:-18943, SEQ ID
NO: 18944, SEQ ID NO: 18946, SEQ ID NO: 18947, SEQ ID NO: 18949, SEQ ID NO: 18951,
SEQ ID NO: 18953, SEQ ID NO: 18955, SEQ ID NO: 18956, SEQ ID NO: 18957, SEQ ID
NO: 18958, SEQ ID NO: 18959, SEQ ID NO: 18960, SEQ ID NO: 18962, SEQ ID NO: 18964,
SEQ ID NO: 18966, SEQ ID NO: 18968, SEQ ID NO: 18969, SEQ ID NO: 18970, SEQ ID
NO: 18972, SEQ ID NO: 18973, SEQ ID NO: 18975, SEQ ID NO: 18976, SEQ ID NO: 18978,
SEQ ID NO: 18980,
SEQ ID NO: 18981, SEQ ID NO: 18982, SEQ ID NO: 18983, SEQ ID NO: 18984, SEQ ID
NO: 18985, SEQ ID NO: 18986, SEQ ID NO: 18987, SEQ ID NO: 18988, SEQ ID NO: 18989,
SEQ ID NO: 18990, SEQ ID NO: 18992, SEQ ID NO: 18993, SEQ ID NO: 18995, SEQ ID
NO: 18997, SEQ ID NO: 18998, SEQ ID NO: 18999, SEQ ID NO: 19000, SEQ ID NO: 19001,
SEQ ID NO: 19002, SEQ ID NO: 19004, SEQ ID NO: 19006
SEQ ID NO: 19007, SEQ ID NO: 19009, SEQ ID NO: 19011, SEQ ID NO: 19012, SEQ ID
NO: 19013, SEQ ID NO: 19014, SEQ ID NO: 19016, SEQ ID NO: 19018, SEQ ID NO: 19020,
SEQ ID NO: 19022, SEQ ID NO: 19024, and SEQ ID NO: 19025
(b) a polynucleotide comprising a nucleotide sequence encoding a protein comprising the amino acid sequence set forth in any one of the following SEQ ID NOs: 
SEQ ID NO:10469, SEQ ID NO:10474, SEQ ID NO:10476, SEQ ID NO:10478, SEQ ID
NO:10480, SEQ ID NO:10482, SEQ ID NO:10484, SEQ ID NO:10486, SEQ ID NO:10490,
SEQ ID NO:10492, SEQ ID NO:10494, SEQ ID NO:10499, SEQ ID NO:10501, SEQ ID
NO:10506, SEQ ID NO:10509, SEQ ID NO:10513, SEQ ID NO:10515, SEQ ID NO:10518,
SEQ ID NO:10520, SEQ ID NO:10522, SEQ ID NO:10525, SEQ ID NO:10527, SEQ ID
NO:10531, SEQ ID NO:10533, SEQ ID NO:10536, SEQ ID NO:10538, SEQ ID NO:10541,
SEQ ID NO:10544, SEQ ID NO:10547, SEQ ID NO:10549, SEQ ID NO:10552, SEQ ID
NO:10554, SEQ ID NO:10559, SEQ ID NO:10561, SEQ ID NO:10563, SEQ ID NO:10565,
SEQ ID NO:10568, SEQ ID NO:10570, SEQ ID NO:10572, SEQ ID NO:10575, SEQ ID
NO:10577, SEQ ID NO:10579, SEQ ID NO:10581, SEQ ID NO:10583, SEQ ID NO:10585,
SEQ ID NO:10587, SEQ ID NO:10589, SEQ ID NO:10591, SEQ ID NO:10593, SEQ ID
NO:10595, SEQ ID NO:10598, SEQ ID NO:10600, SEQ ID NO:10602, SEQ ID NO:10605,
SEQ ID NO:10608, SEQ ID NO:10610, SEQ ID NO:10612, SEQ ID NO:10617, SEQ ID
NO:10621, SEQ ID NO:10623, SEQ ID NO:10626, SEQ ID NO:10628, SEQ ID NO:10631,
SEQ ID NO:10634, SEQ ID NO:10636, SEQ ID NO:10638, SEQ ID NO:10640, SEQ ID
NO:10643, SEQ ID NO:10645, SEQ ID NO:10651, SEQ ID NO:10653, SEQ ID NO:10657,
SEQ ID NO:10660, SEQ ID NO:10662, SEQ ID NO:10664, SEQ ID NO:10666, SEQ ID
NO:10668, SEQ ID NO:10672, SEQ ID NO:10675, SEQ ID NO:10677, SEQ ID NO:10679,
SEQ ID NO:10681, SEQ ID NO:10684, SEQ ID NO:10686, SEQ ID NO:10688, SEQ ID
NO:10690, SEQ ID NO:10692, SEQ ID NO:10694, SEQ ID NO:10697, SEQ ID NO:10699,
SEQ ID NO:10701, SEQ ID NO:10703, SEQ ID NO:10705, SEQ ID NO:10707, SEQ ID
NO:10709, SEQ ID NO:10712, SEQ ID NO:10714, SEQ ID NO:10716, SEQ ID NO:10719,
SEQ ID NO:10721,
SEQ ID NO:10724, SEQ ID NO:10726, SEQ ID NO:10729, SEQ ID NO:10731, SEQ ID
NO:10733, SEQ ID NO:10735, SEQ ID NO:10737, SEQ ID NO:10739, SEQ ID NO:10741,
SEQ ID NO:10743, SEQ ID NO:10745, SEQ ID NO:10747, SEQ ID NO:10749, SEQ ID
NO:10751, SEQ ID NO:10755, SEQ ID NO:10759, SEQ ID NO:10762, SEQ ID NO:10764,
SEQ ID NO:10766, SEQ ID NO:10768, SEQ ID NO:10770, SEQ ID NO:10772, SEQ ID
NO:10775, SEQ ID NO:10777, SEQ ID NO:10779, SEQ ID NO:10782, SEQ ID NO:10784,
SEQ ID NO:10787, SEQ ID NO:10789, SEQ ID NO:10791, SEQ ID NO:10794, SEQ ID
NO:10796, SEQ ID NO:10798, SEQ ID NO:10801, SEQ ID NO:10803, SEQ ID NO:10806,
SEQ ID NO:10809, SEQ ID NO:10811, SEQ ID NO:10813, SEQ ID NO:10816, SEQ ID
NO:10819, SEQ ID NO:10821, SEQ ID NO:10823, SEQ ID NO:10825, SEQ ID NO:10827,
SEQ ID NO:10829, SEQ ID NO:10833, SEQ ID NO:10835, SEQ ID NO:10837, SEQ ID
NO:10839, SEQ ID NO:10843, SEQ ID NO:10846, SEQ ID NO:10848, SEQ ID NO:10851,
 
SEQ ID NO:10853, SEQ ID NO:10855, SEQ ID NO:10857, SEQ ID NO:10860, SEQ ID
NO:10863, SEQ ID NO:10865, SEQ ID NO:10867, SEQ ID NO:10869, SEQ ID NO:10871,
SEQ ID NO:10874, SEQ ID NO:10877, SEQ ID NO:10880, SEQ ID NO:10882, SEQ ID
NO:10884, SEQ ID NO:10887, SEQ ID NO:10892, SEQ ID NO:10894, SEQ ID NO:10896,
SEQ ID NO:10899, SEQ ID NO:10903, SEQ ID NO:10905, SEQ ID NO:10907, SEQ ID
NO:10911, SEQ ID NO:10917, SEQ ID NO:10919, SEQ ID NO:10921, SEQ ID NO:10923,
SEQ ID NO:10926, SEQ ID NO:10930, SEQ ID NO:10932, SEQ ID NO:10934, SEQ ID
NO:10936, SEQ ID NO:10938, SEQ ID NO:10941, SEQ ID NO:10947, SEQ ID NO:10949,
SEQ ID NO:10951, SEQ ID NO:10953, SEQ ID NO:10955, SEQ ID NO:10961, SEQ ID
NO:10963, SEQ ID NO:10965, SEQ ID NO:10970, SEQ ID NO:10972, SEQ ID NO:10974,
SEQ ID NO:10976,
SEQ ID NO:10979, SEQ ID NO:10981, SEQ ID NO:10985, SEQ ID NO:10987, SEQ ID
NO:10989, SEQ ID NO:10991, SEQ ID NO:10993, SEQ ID NO:10995, SEQ ID NO:10997,
SEQ ID NO:10999, SEQ ID NO:11002, SEQ ID NO:11004, SEQ ID NO:11008, SEQ ID
NO:11010, SEQ ID NO:11013, SEQ ID NO:11015, SEQ ID NO:11017, SEQ ID NO:11020,
SEQ ID NO:11022, SEQ ID NO:11024, SEQ ID NO:11026, SEQ ID NO:11028, SEQ ID
NO:11031, SEQ ID NO:11033, SEQ ID NO:11037, SEQ ID NO:11039, SEQ ID NO:11041,
SEQ ID NO:11043, SEQ ID NO:11045, SEQ ID NO:11047, SEQ ID NO:11049, SEQ ID
NO:11051, SEQ ID NO:11053, SEQ ID NO:11055, SEQ ID NO:11057, SEQ ID NO:11059,
SEQ ID NO:11061, SEQ ID NO:11063, SEQ ID NO:11065, SEQ ID NO:11067, SEQ ID
NO:11069, SEQ ID NO:11071, SEQ ID NO:11073, SEQ ID NO:11075, SEQ ID NO:11077,
SEQ ID NO:11079, SEQ ID NO:11081, SEQ ID NO:11083, SEQ ID NO:11085, SEQ ID
NO:11087, SEQ ID NO:11089, SEQ ID NO:11091, SEQ ID NO:11094, SEQ ID NO:11096,
SEQ ID NO:11098, SEQ ID NO:11100, SEQ ID NO:11102, SEQ ID NO:11104, SEQ ID
NO:11106, SEQ ID NO:11109, SEQ ID NO:11111, SEQ ID NO:11113, SEQ ID NO:11115,
SEQ ID NO:11117, SEQ ID NO:11119, SEQ ID NO:11121, SEQ ID NO:11124, SEQ ID
NO:11126, SEQ ID NO:11128, SEQ ID NO:11130, SEQ ID NO:11132, SEQ ID NO:11134,
SEQ ID NO:11136, SEQ ID NO:11138, SEQ ID NO:11140, SEQ ID NO:11142, SEQ ID
NO:11144, SEQ ID NO:11146, SEQ ID NO:11149, SEQ ID NO:11151, SEQ ID NO:11153,
SEQ ID NO:11155, SEQ ID NO:11157, SEQ ID NO:11159, SEQ ID NO:11161, SEQ ID
NO:11163, SEQ ID NO:11166, SEQ ID NO:11169, SEQ ID NO:11171, SEQ ID NO:11173,
SEQ ID NO:11175, SEQ ID NO:11177, SEQ ID NO:11180, SEQ ID NO:11182, SEQ ID
NO:11184, SEQ ID NO:11186, SEQ ID NO:11188, SEQ ID NO:11190, SEQ ID NO:11192,
SEQ ID NO:11194,
SEQ ID NO:11196, SEQ ID NO:11198, SEQ ID NO:11200, SEQ ID NO:11202, SEQ ID
NO:11204, SEQ ID NO:11206, SEQ ID NO:11208, SEQ ID NO:11210, SEQ ID NO:11212,
SEQ ID NO:11214, SEQ ID NO:11216, SEQ ID NO:11218, SEQ ID NO:11220, SEQ ID
NO:11222, SEQ ID NO:11224, SEQ ID NO:11226, SEQ ID NO:11228, SEQ ID NO:11230,
SEQ ID NO:11232, SEQ ID NO:11234, SEQ ID NO:11236, SEQ ID NO:11238, SEQ ID
NO:11240, SEQ ID NO:11242, SEQ ID NO:11245, SEQ ID NO:11247, SEQ ID NO:11249,
SEQ ID NO:11251, SEQ ID NO:11254, SEQ ID NO:11256, SEQ ID NO:11258, SEQ ID
NO:11260, SEQ ID NO:11262, SEQ ID NO:11264, SEQ ID NO:11266, SEQ ID NO:11268,
SEQ ID NO:11270, SEQ ID NO:11272, SEQ ID NO:11274, SEQ ID NO:11276, SEQ ID
NO:11278, SEQ ID NO:11280, SEQ ID NO:11282, SEQ ID NO:11284, SEQ ID NO:11286,
SEQ ID NO:11288, SEQ ID NO:11290, SEQ ID NO:11292, SEQ ID NO:11294, SEQ ID
NO:11296, SEQ ID NO:11298, SEQ ID NO:11300, SEQ ID NO:11302, SEQ ID NO:11305,
SEQ ID NO:11307, SEQ ID NO:11309, SEQ ID NO:11311, SEQ ID NO:11313, SEQ ID
NO:11315, SEQ ID NO:11317, SEQ ID NO:11320, SEQ ID NO:11322, SEQ ID NO:11324,
SEQ ID NO:11326, SEQ ID NO:11328, SEQ ID NO:11330, SEQ ID NO:11332, SEQ ID
NO:11334, SEQ ID NO:11336, SEQ ID NO:11338, SEQ ID NO:11340, SEQ ID NO:11342,
SEQ ID NO:11344, SEQ ID NO:11346, SEQ ID NO:11348, SEQ ID NO:11350, SEQ ID
NO:11353, SEQ ID NO:11355, SEQ ID NO:11357, SEQ ID NO:11359, SEQ ID NO:11361,
 
SEQ ID NO:11363, SEQ ID NO:11365, SEQ ID NO:11367, SEQ ID NO:11371, SEQ ID
NO:11373, SEQ ID NO:11375, SEQ ID NO:11377, SEQ ID NO:11379, SEQ ID NO:11381,
SEQ ID NO:11383, SEQ ID NO:11385, SEQ ID NO:11387, SEQ ID NO:11389, SEQ ID
NO:11391, SEQ ID NO:11393, SEQ ID NO:11395, SEQ ID NO:11398, SEQ ID NO:11400,
SEQ ID NO:11402,
SEQ ID NO:11404, SEQ ID NO:11406, SEQ ID NO:11408, SEQ ID NO:11410, SEQ ID
NO:11412, SEQ ID NO:11414, SEQ ID NO:11417, SEQ ID NO:11420, SEQ ID NO:11422,
SEQ ID NO:11424, SEQ ID NO:11426, SEQ ID NO:11428, SEQ ID NO:11431, SEQ ID
NO:11433, SEQ ID NO:11435, SEQ ID NO:11438, SEQ ID NO:11440, SEQ ID NO:11442,
SEQ ID NO:11444, SEQ ID NO:11446, SEQ ID NO:11448, SEQ ID NO:11450, SEQ ID
NO:11452, SEQ ID NO:11454, SEQ ID NO:11456, SEQ ID NO:11458, SEQ ID NO:11460,
SEQ ID NO:11462, SEQ ID NO:11464, SEQ ID NO:11466, SEQ ID NO:11468, SEQ ID
NO:11471, SEQ ID NO:11473, SEQ ID NO:11475, SEQ ID NO:11477, SEQ ID NO:11479,
SEQ ID NO:11481, SEQ ID NO:11483, SEQ ID NO:11486, SEQ ID NO:11488, SEQ ID
NO:11490, SEQ ID NO:11492, SEQ ID NO:11494, SEQ ID NO:11497, SEQ ID NO:11499,
SEQ ID NO:11501, SEQ ID NO:11503, SEQ ID NO:11505, SEQ ID NO:11508, SEQ ID
NO:11510, SEQ ID NO:11512, SEQ ID NO:11514, SEQ ID NO:11516, SEQ ID NO:11518,
SEQ ID NO:11520, SEQ ID NO:11522, SEQ ID NO:11525, SEQ ID NO:11527, SEQ ID
NO:11529, SEQ ID NO:11531, SEQ ID NO:11533, SEQ ID NO:11535, SEQ ID NO:11537,
SEQ ID NO:11539, SEQ ID NO:11542, SEQ ID NO:11544, SEQ ID NO:11546, SEQ ID
NO:11548, SEQ ID NO:11550, SEQ ID NO:11552, SEQ ID NO:11554, SEQ ID NO:11556,
SEQ ID NO:11558, SEQ ID NO:11563, SEQ ID NO:11565, SEQ ID NO:11567, SEQ ID
NO:11570, SEQ ID NO:11572, SEQ ID NO:11574, SEQ ID NO:11576, SEQ ID NO:11578,
SEQ ID NO:11580, SEQ ID NO:11583, SEQ ID NO:11585, SEQ ID NO:11587, SEQ ID
NO:11589, SEQ ID NO:11591, SEQ ID NO:11593, SEQ ID NO:11595, SEQ ID NO:11597,
SEQ ID NO:11599, SEQ ID NO:11601, SEQ ID NO:11603, SEQ ID NO:11605, SEQ ID
NO:11607, SEQ ID NO:11610, SEQ ID NO:11612, SEQ ID NO:11614, SEQ ID NO:11616,
SEQ ID NO:11618,
SEQ ID NO:11621, SEQ ID NO:11623, SEQ ID NO:11625, SEQ ID NO:11627, SEQ ID
NO:11629, SEQ ID NO:11631, SEQ ID NO:11633, SEQ ID NO:11635, SEQ ID NO:11637,
SEQ ID NO:11640, SEQ ID NO:11642, SEQ ID NO:11644, SEQ ID NO:11646, SEQ ID
NO:11648, SEQ ID NO:11650, SEQ ID NO:11652, SEQ ID NO:11655, SEQ ID NO:11657,
SEQ ID NO:11660, SEQ ID NO:11662, SEQ ID NO:11664, SEQ ID NO:11667, SEQ ID
NO:11669, SEQ ID NO:11671, SEQ ID NO:11673, SEQ ID NO:11676, SEQ ID NO:11679,
SEQ ID NO:11681, SEQ ID NO:11683, SEQ ID NO:11686, SEQ ID NO:11688, SEQ ID
NO:11690, SEQ ID NO:11692, SEQ ID NO:11694, SEQ ID NO:11696, SEQ ID NO:11698,
SEQ ID NO:11700, SEQ ID NO:11702, SEQ ID NO:11704, SEQ ID NO:11706, SEQ ID
NO:11708, SEQ ID NO:11710, SEQ ID NO:11712, SEQ ID NO:11714, SEQ ID NO:11716,
SEQ ID NO:11719, SEQ ID NO:11721, SEQ ID NO:11723, SEQ ID NO:11725, SEQ ID
NO:11727, SEQ ID NO:11729, SEQ ID NO:11731, SEQ ID NO:11733, SEQ ID NO:11735,
SEQ ID NO:11737, SEQ ID NO:11741, SEQ ID NO:11743, SEQ ID NO:11745, SEQ ID
NO:11747, SEQ ID NO:11749, SEQ ID NO:11751, SEQ ID NO:11753, SEQ ID NO:11755,
SEQ ID NO:11757, SEQ ID NO:11760, SEQ ID NO:11762, SEQ ID NO:11764, SEQ ID
NO:11766, SEQ ID NO:11769, SEQ ID NO:11771, SEQ ID NO:11773, SEQ ID NO:11775,
SEQ ID NO:11777, SEQ ID NO:11779, SEQ ID NO:11781, SEQ ID NO:11783, SEQ ID
NO:11785, SEQ ID NO:11787, SEQ ID NO:11789, SEQ ID NO:11791, SEQ ID NO:11793,
SEQ ID NO:11795, SEQ ID NO:11797, SEQ ID NO:11799, SEQ ID NO:11801, SEQ ID
NO:11803, SEQ ID NO:11805, SEQ ID NO:11807, SEQ ID NO:11809, SEQ ID NO:11812,
SEQ ID NO:11814, SEQ ID NO:11816, SEQ ID NO:11818, SEQ ID NO:11821, SEQ ID
NO:11823, SEQ ID NO:11825, SEQ ID NO:11827, SEQ ID NO:11829, SEQ ID NO:11831,
SEQ ID NO:11834,
 
SEQ ID NO:11836, SEQ ID NO:11838, SEQ ID NO:11840, SEQ ID NO:11842, SEQ ID
NO:11844, SEQ ID NO:11846, SEQ ID NO:11848, SEQ ID NO:11852, SEQ ID NO:11854,
SEQ ID NO:11857, SEQ ID NO:11860, SEQ ID NO:11862, SEQ ID NO:11864, SEQ ID
NO:11866, SEQ ID NO:11868, SEQ ID NO:11872, SEQ ID NO:11874, SEQ ID NO:11876,
SEQ ID NO:11878, SEQ ID NO:11881, SEQ ID NO:11883, SEQ ID NO:11885, SEQ ID
NO:11887, SEQ ID NO:11889, SEQ ID NO:11891, SEQ ID NO:11894, SEQ ID NO:11896,
SEQ ID NO:11898, SEQ ID NO:11900, SEQ ID NO:11902, SEQ ID NO:11904, SEQ ID
NO:11906, SEQ ID NO:11908, SEQ ID NO:11910, SEQ ID NO:11912, SEQ ID NO:11914,
SEQ ID NO:11917, SEQ ID NO:11920, SEQ ID NO:11922, SEQ ID NO:11924, SEQ ID
NO:11926, SEQ ID NO:11929, SEQ ID NO:11931, SEQ ID NO:11933, SEQ ID NO:11935,
SEQ ID NO:11937, SEQ ID NO:11939, SEQ ID NO:11941, SEQ ID NO:11943, SEQ ID
NO:11945, SEQ ID NO:11947, SEQ ID NO:11950, SEQ ID NO:11952, SEQ ID NO:11954,
SEQ ID NO:11956, SEQ ID NO:11958, SEQ ID NO:11960, SEQ ID NO:11962, SEQ ID
NO:11964, SEQ ID NO:11966, SEQ ID NO:11968, SEQ ID NO:11970, SEQ ID NO:11972,
SEQ ID NO:11974, SEQ ID NO:11976, SEQ ID NO:11979, SEQ ID NO:11981, SEQ ID
NO:11983, SEQ ID NO:11985, SEQ ID NO:11987, SEQ ID NO:11989, SEQ ID NO:11991,
SEQ ID NO:11993, SEQ ID NO:11995, SEQ ID NO:11997, SEQ ID NO:11999, SEQ ID
NO:12001, SEQ ID NO:12004, SEQ ID NO:12006, SEQ ID NO:12009, SEQ ID NO:12011,
SEQ ID NO:12013, SEQ ID NO:12015, SEQ ID NO:12017, SEQ ID NO:12021, SEQ ID
NO:12023, SEQ ID NO:12025, SEQ ID NO:12027, SEQ ID NO:12029, SEQ ID NO:12031,
SEQ ID NO:12033, SEQ ID NO:12035, SEQ ID NO:12037, SEQ ID NO:12039, SEQ ID
NO:12041, SEQ ID NO:12043, SEQ ID NO:12045, SEQ ID NO:12047, SEQ ID NO:12050,
SEQ ID NO:12052,
SEQ ID NO:12054, SEQ ID NO:12057, SEQ ID NO:12059, SEQ ID NO:12061, SEQ ID
NO:12063, SEQ ID NO:12065, SEQ ID NO:12067, SEQ ID NO:12069, SEQ ID NO:12071,
SEQ ID NO:12073, SEQ ID NO:12075, SEQ ID NO:12077, SEQ ID NO:12079, SEQ ID
NO:12081, SEQ ID NO:12083, SEQ ID NO:12085, SEQ ID NO:12087, SEQ ID NO:12089,
SEQ ID NO:12091, SEQ ID NO:12093, SEQ ID NO:12095, SEQ ID NO:12098, SEQ ID
NO:12100, SEQ ID NO:12102, SEQ ID NO:12104, SEQ ID NO:12106, SEQ ID NO:12108,
SEQ ID NO:12110, SEQ ID NO:12112, SEQ ID NO:12114, SEQ ID NO:12116, SEQ ID
NO:12118, SEQ ID NO:12120, SEQ ID NO:12122, SEQ ID NO:12124, SEQ ID NO:12126,
SEQ ID NO:12128, SEQ ID NO:12130, SEQ ID NO:12132, SEQ ID NO:12134, SEQ ID
NO:12136, SEQ ID NO:12138, SEQ ID NO:12140, SEQ ID NO:12142, SEQ ID NO:12144,
SEQ ID NO:12146, SEQ ID NO:12148, SEQ ID NO:12150, SEQ ID NO:12152, SEQ ID
NO:12154, SEQ ID NO:12156, SEQ ID NO:12158, SEQ ID NO:12160, SEQ ID NO:12162,
SEQ ID NO:12164, SEQ ID NO:12166, SEQ ID NO:12168, SEQ ID NO:12170, SEQ ID
NO:12172, SEQ ID NO:12174, SEQ ID NO:12176, SEQ ID NO:12178, SEQ ID NO:12180,
SEQ ID NO:12182, SEQ ID NO:12184, SEQ ID NO:12186, SEQ ID NO:12188, SEQ ID
NO:12190, SEQ ID NO:12192, SEQ ID NO:12194, SEQ ID NO:12196, SEQ ID NO:12198,
SEQ ID NO:12200, SEQ ID NO:12202, SEQ ID NO:12204, SEQ ID NO:12206, SEQ ID
NO:12208, SEQ ID NO:12210, SEQ ID NO:12212, SEQ ID NO:12214, SEQ ID NO:12217,
SEQ ID NO:12219, SEQ ID NO:12221, SEQ ID NO:12223, SEQ ID NO:12225, SEQ ID
NO:12227, SEQ ID NO:12229, SEQ ID NO:12231, SEQ ID NO:12233, SEQ ID NO:12235,
SEQ ID NO:12237, SEQ ID NO:12239, SEQ ID NO:12241, SEQ ID NO:12243, SEQ ID
NO:12245, SEQ ID NO:12247, SEQ ID NO:12249, SEQ ID NO:12251, SEQ ID NO:12253,
SEQ ID NO:12255,
SEQ ID NO:12257, SEQ ID NO:12259, SEQ ID NO:12261, SEQ ID NO:12263, SEQ ID
NO:12265, SEQ ID NO:12267, SEQ ID NO:12269, SEQ ID NO:12271, SEQ ID NO:12273,
SEQ ID NO:12275, SEQ ID NO:12277, SEQ ID NO:12280, SEQ ID NO:12282, SEQ ID
NO:12284, SEQ ID NO:12286, SEQ ID NO:12288, SEQ ID NO:12290, SEQ ID NO:12292,
SEQ ID NO:12294, SEQ ID NO:12296, SEQ ID NO:12298, SEQ ID NO:12300, SEQ ID
NO:12302, SEQ ID NO:12304, SEQ ID NO:12306, SEQ ID NO:12308, SEQ ID NO:12310,
 
SEQ ID NO:12312, SEQ ID NO:12314, SEQ ID NO:12316, SEQ ID NO:12318, SEQ ID
NO:12320, SEQ ID NO:12322, SEQ ID NO:12324, SEQ ID NO:12326, SEQ ID NO:12328,
SEQ ID NO:12330, SEQ ID NO:12332, SEQ ID NO:12334, SEQ ID NO:12336, SEQ ID
NO:12338, SEQ ID NO:12340, SEQ ID NO:12342, SEQ ID NO:12344, SEQ ID NO:12346,
SEQ ID NO:12348, SEQ ID NO:12350, SEQ ID NO:12352, SEQ ID NO:12355, SEQ ID
NO:12357, SEQ ID NO:12359, SEQ ID NO:12361, SEQ ID NO:12363, SEQ ID NO:12365,
SEQ ID NO:12367, SEQ ID NO:12369, SEQ ID NO:12371, SEQ ID NO:12373, SEQ ID
NO:12375, SEQ ID NO:12377, SEQ ID NO:12379, SEQ ID NO:12381, SEQ ID NO:12383,
SEQ ID NO:12385, SEQ ID NO:12387, SEQ ID NO:12389, SEQ ID NO:12391, SEQ ID
NO:12393, SEQ ID NO:12395, SEQ ID NO:12397, SEQ ID NO:12399, SEQ ID NO:12401,
SEQ ID NO:12404, SEQ ID NO:12406, SEQ ID NO:12408, SEQ ID NO:12411, SEQ ID
NO:12413, SEQ ID NO:12415, SEQ ID NO:12418, SEQ ID NO:12420, SEQ ID NO:12422,
SEQ ID NO:12424, SEQ ID NO:12426, SEQ ID NO:12428, SEQ ID NO:12430, SEQ ID
NO:12432, SEQ ID NO:12434, SEQ ID NO:12437, SEQ ID NO:12439, SEQ ID NO:12441,
SEQ ID NO:12443, SEQ ID NO:12445, SEQ ID NO:12447, SEQ ID NO:12449, SEQ ID
NO:12451, SEQ ID NO:12453, SEQ ID NO:12455, SEQ ID NO:12457, SEQ ID NO:12459,
SEQ ID NO:12462,
SEQ ID NO:12464, SEQ ID NO:12466, SEQ ID NO:12468, SEQ ID NO:12470, SEQ ID
NO:12472, SEQ ID NO:12474, SEQ ID NO:12476, SEQ ID NO:12478, SEQ ID NO:12480,
SEQ ID NO:12484, SEQ ID NO:12487, SEQ ID NO:12489, SEQ ID NO:12492, SEQ ID
NO:12494, SEQ ID NO:12497, SEQ ID NO:12499, SEQ ID NO:12501, SEQ ID NO:12504,
SEQ ID NO:12506, SEQ ID NO:12508, SEQ ID NO:12510, SEQ ID NO:12512, SEQ ID
NO:12515, SEQ ID NO:12517, SEQ ID NO:12519, SEQ ID NO:12521, SEQ ID NO:12523,
SEQ ID NO:12525, SEQ ID NO:12527, SEQ ID NO:12530, SEQ ID NO:12532, SEQ ID
NO:12536, SEQ ID NO:12538, SEQ ID NO:12540, SEQ ID NO:12542, SEQ ID NO:12544,
SEQ ID NO:12547, SEQ ID NO:12549, SEQ ID NO:12551, SEQ ID NO:12553, SEQ ID
NO:12558, SEQ ID NO:12560, SEQ ID NO:12562, SEQ ID NO:12564, SEQ ID NO:12566,
SEQ ID NO:12568, SEQ ID NO:12570, SEQ ID NO:12573, SEQ ID NO:12575, SEQ ID
NO:12577, SEQ ID NO:12579, SEQ ID NO:12582, SEQ ID NO:12584, SEQ ID NO:12586,
SEQ ID NO:12588, SEQ ID NO:12590, SEQ ID NO:12594, SEQ ID NO:12596, SEQ ID
NO:12598, SEQ ID NO:12600, SEQ ID NO:12602, SEQ ID NO:12604, SEQ ID NO:12606,
SEQ ID NO:12610, SEQ ID NO:12612, SEQ ID NO:12614, SEQ ID NO:12616, SEQ ID
NO:12618, SEQ ID NO:12620, SEQ ID NO:12624, SEQ ID NO:12626, SEQ ID NO:12629,
SEQ ID NO:12631, SEQ ID NO:12633, SEQ ID NO:12635, SEQ ID NO:12637, SEQ ID
NO:12639, SEQ ID NO:12641, SEQ ID NO:12643, SEQ ID NO:12645, SEQ ID NO:12647,
SEQ ID NO:12650, SEQ ID NO:12652, SEQ ID NO:12654, SEQ ID NO:12656, SEQ ID
NO:12658, SEQ ID NO:12661, SEQ ID NO:12663, SEQ ID NO:12666, SEQ ID NO:12668,
SEQ ID NO:12670, SEQ ID NO:12672, SEQ ID NO:12674, SEQ ID NO:12676, SEQ ID
NO:12678, SEQ ID NO:12680, SEQ ID NO:12682, SEQ ID NO:12684, SEQ ID NO:12686,
SEQ ID NO:12688,
SEQ ID NO:12690, SEQ ID NO:12692, SEQ ID NO:12694, SEQ ID NO:12696, SEQ ID
NO:12699, SEQ ID NO:12701, SEQ ID NO:12704, SEQ ID NO:12706, SEQ ID NO:12708,
SEQ ID NO:12712, SEQ ID NO:12714, SEQ ID NO:12716, SEQ ID NO:12718, SEQ ID
NO:12720, SEQ ID NO:12722, SEQ ID NO:12724, SEQ ID NO:12727, SEQ ID NO:12729,
SEQ ID NO:12732, SEQ ID NO:12734, SEQ ID NO:12736, SEQ ID NO:12738, SEQ ID
NO:12740, SEQ ID NO:12742, SEQ ID NO:12744, SEQ ID NO:12746, SEQ ID NO:12748,
SEQ ID NO:12750, SEQ ID NO:12752, SEQ ID NO:12755, SEQ ID NO:12757, SEQ ID
NO:12759, SEQ ID NO:12761, SEQ ID NO:12766, SEQ ID NO:12768, SEQ ID NO:12772,
SEQ ID NO:12774, SEQ ID NO:12777, SEQ ID NO:12779, SEQ ID NO:12782, SEQ ID
NO:12784, SEQ ID NO:12786, SEQ ID NO:12788, SEQ ID NO:12790, SEQ ID NO:12792,
SEQ ID NO:12794, SEQ ID NO:12796, SEQ ID NO:12802, SEQ ID NO:12804, SEQ ID
NO:12807, SEQ ID NO:12809, SEQ ID NO:12811, SEQ ID NO:12814, SEQ ID NO:12816,
 
SEQ ID NO:12818, SEQ ID NO:12820, SEQ ID NO:12822, SEQ ID NO:12824, SEQ ID
NO:12826, SEQ ID NO:12828, SEQ ID NO:12830, SEQ ID NO:12832, SEQ ID NO:12834,
SEQ ID NO:12836, SEQ ID NO:12838, SEQ ID NO:12840, SEQ ID NO:12842, SEQ ID
NO:12844, SEQ ID NO:12846, SEQ ID NO:12849, SEQ ID NO:12851, SEQ ID NO:12853,
SEQ ID NO:12855, SEQ ID NO:12857, SEQ ID NO:12859, SEQ ID NO:12861, SEQ ID
NO:12863, SEQ ID NO:12865, SEQ ID NO:12869, SEQ ID NO:12872, SEQ ID NO:12875,
SEQ ID NO:12877, SEQ ID NO:12879, SEQ ID NO:12882, SEQ ID NO:12884, SEQ ID
NO:12886, SEQ ID NO:12888, SEQ ID NO:12890, SEQ ID NO:12893, SEQ ID NO:12895,
SEQ ID NO:12898, SEQ ID NO:12900, SEQ ID NO:12902, SEQ ID NO:12904, SEQ ID
NO:12906, SEQ ID NO:12908, SEQ ID NO:12910, SEQ ID NO:12912, SEQ ID NO:12914,
SEQ ID NO:12917,
SEQ ID NO:12919, SEQ ID NO:12921, SEQ ID NO:12924, SEQ ID NO:12926, SEQ ID
NO:12928, SEQ ID NO:12930, SEQ ID NO:12932, SEQ ID NO:12935, SEQ ID NO:12937,
SEQ ID NO:12939, SEQ ID NO:12942, SEQ ID NO:12944, SEQ ID NO:12946, SEQ ID
NO:12948, SEQ ID NO:12951, SEQ ID NO:12954, SEQ ID NO:12957, SEQ ID NO:12959,
SEQ ID NO:12962, SEQ ID NO:12964, SEQ ID NO:12968, SEQ ID NO:12970, SEQ ID
NO:12972, SEQ ID NO:12974, SEQ ID NO:12976, SEQ ID NO:12978, SEQ ID NO:12980,
SEQ ID NO:12982, SEQ ID NO:12984, SEQ ID NO:12986, SEQ ID NO:12988, SEQ ID
NO:12990, SEQ ID NO:12992, SEQ ID NO:12994, SEQ ID NO:12996, SEQ ID NO:12998,
SEQ ID NO:13000, SEQ ID NO:13002, SEQ ID NO:13004, SEQ ID NO:13006, SEQ ID
NO:13008, SEQ ID NO:13010, SEQ ID NO:13012, SEQ ID NO:13014, SEQ ID NO:13016,
SEQ ID NO:13019, SEQ ID NO:13022, SEQ ID NO:13024, SEQ ID NO:13026, SEQ ID
NO:13028, SEQ ID NO:13030, SEQ ID NO:13032, SEQ ID NO:13034, SEQ ID NO:13036,
SEQ ID NO:13038, SEQ ID NO:13041, SEQ ID NO:13043, SEQ ID NO:13045, SEQ ID
NO:13047, SEQ ID NO:13049, SEQ ID NO:13051, SEQ ID NO:13054, SEQ ID NO:13056,
SEQ ID NO:13059, SEQ ID NO:13062, SEQ ID NO:13064, SEQ ID NO:13066, SEQ ID
NO:13068, SEQ ID NO:13070, SEQ ID NO:13073, SEQ ID NO:13075, SEQ ID NO:13078,
SEQ ID NO:13080, SEQ ID NO:13082, SEQ ID NO:13084, SEQ ID NO:13086, SEQ ID
NO:13090, SEQ ID NO:13093, SEQ ID NO:13095, SEQ ID NO:13098, SEQ ID NO:13100,
SEQ ID NO:13102, SEQ ID NO:13104, SEQ ID NO:13106, SEQ ID NO:13111, SEQ ID
NO:13113, SEQ ID NO:13115, SEQ ID NO:13117, SEQ ID NO:13121, SEQ ID NO:13123,
SEQ ID NO:13126, SEQ ID NO:13128, SEQ ID NO:13130, SEQ ID NO:13132, SEQ ID
NO:13134, SEQ ID NO:13136, SEQ ID NO:13138, SEQ ID NO:13140, SEQ ID NO:13142,
SEQ ID NO:13146,
SEQ ID NO:13148, SEQ ID NO:13150, SEQ ID NO:13153, SEQ ID NO:13155, SEQ ID
NO:13157, SEQ ID NO:13159, SEQ ID NO:13161, SEQ ID NO:13163, SEQ ID NO:13165,
SEQ ID NO:13167, SEQ ID NO:13169, SEQ ID NO:13171, SEQ ID NO:13173, SEQ ID
NO:13175, SEQ ID NO:13179, SEQ ID NO:13182, SEQ ID NO:13184, SEQ ID NO:13186,
SEQ ID NO:13188, SEQ ID NO:13190, SEQ ID NO:13192, SEQ ID NO:13194, SEQ ID
NO:13196, SEQ ID NO:13199, SEQ ID NO:13202, SEQ ID NO:13204, SEQ ID NO:13206,
SEQ ID NO:13208, SEQ ID NO:13210, SEQ ID NO:13212, SEQ ID NO:13214, SEQ ID
NO:13217, SEQ ID NO:13220, SEQ ID NO:13223, SEQ ID NO:13225, SEQ ID NO:13227,
SEQ ID NO:13229, SEQ ID NO:13231, SEQ ID NO:13233, SEQ ID NO:13236, SEQ ID
NO:13240, SEQ ID NO:13244, SEQ ID NO:13247, SEQ ID NO:13249, SEQ ID NO:13251,
SEQ ID NO:13253, SEQ ID NO:13255, SEQ ID NO:13258, SEQ ID NO:13262, SEQ ID
NO:13265, SEQ ID NO:13267, SEQ ID NO:13269, SEQ ID NO:13272, SEQ ID NO:13274,
SEQ ID NO:13277, SEQ ID NO:13281, SEQ ID NO:13283, SEQ ID NO:13285, SEQ ID
NO:13287, SEQ ID NO:13289,
SEQ ID NO:13295, SEQ ID NO:13297, SEQ ID NO:13299, SEQ ID NO:13301, SEQ ID
NO:13303, SEQ ID NO:13305, SEQ ID NO:13308, SEQ ID NO:13311, SEQ ID NO:13313,
SEQ ID NO:13315, SEQ ID NO:13317, SEQ ID NO:13320, SEQ ID NO:13323, SEQ ID
NO:13326, SEQ ID NO:13329, SEQ ID NO:13331, SEQ ID NO:13335, SEQ ID NO:13340,
 
SEQ ID NO:13343, SEQ ID NO:13349, SEQ ID NO:13353, SEQ ID NO:13355, SEQ ID
NO:13358, SEQ ID NO:13361, SEQ ID NO:13364, SEQ ID NO:13367, SEQ ID NO:13373,
SEQ ID NO:13377, SEQ ID NO:13380, SEQ ID NO:13382, SEQ ID NO:13384, SEQ ID
NO:13386, SEQ ID NO:13388, SEQ ID NO:13390, SEQ ID NO:13392, SEQ ID NO:13395,
SEQ ID NO:13397, SEQ ID NO:13399, SEQ ID NO:13401, SEQ ID NO:13403, SEQ ID
NO:13405, SEQ ID NO:13407, SEQ ID NO:13409, SEQ ID NO:13411, SEQ ID NO:13413,
SEQ ID NO:13416, SEQ ID NO:13419, SEQ ID NO:13422, SEQ ID NO:13424, SEQ ID
NO:13426, SEQ ID NO:13429, SEQ ID NO:13432, SEQ ID NO:13435, SEQ ID NO:13437,
SEQ ID NO:13446, SEQ ID NO:13450, SEQ ID NO:13452, SEQ ID NO:13456, SEQ ID
NO:13459, SEQ ID NO:13461, SEQ ID NO:13464, SEQ ID NO:13466, SEQ ID NO:13468,
SEQ ID NO:13471, SEQ ID NO:13475, SEQ ID NO:13481, SEQ ID NO:13487, SEQ ID
NO:13490, SEQ ID NO:13493, SEQ ID NO:13496, SEQ ID NO:13499, SEQ ID NO:13502,
SEQ ID NO:13504, SEQ ID NO:13509, SEQ ID NO:13511, SEQ ID NO:13513, SEQ ID
NO:13516, SEQ ID NO:13521, SEQ ID NO:13525, SEQ ID NO:13528, SEQ ID NO:13530,
SEQ ID NO:13533, SEQ ID NO:13537, SEQ ID NO:13541, SEQ ID NO:13543, SEQ ID
NO:13545, SEQ ID NO:13548, SEQ ID NO:13550, SEQ ID NO:13552, SEQ ID NO:13555,
SEQ ID NO:13558, SEQ ID NO:13560, SEQ ID NO:13564, SEQ ID NO:13567, SEQ ID
NO:13569, SEQ ID NO:13573, SEQ ID NO:13575, SEQ ID NO:13577, SEQ ID NO:13579,
SEQ ID NO:13583,
SEQ ID NO:13585, SEQ ID NO:13587, SEQ ID NO:13589, SEQ ID NO:13593, SEQ ID
NO:13596, SEQ ID NO:13598, SEQ ID NO:13600, SEQ ID NO:13602, SEQ ID NO:13604,
SEQ ID NO:13606, SEQ ID NO:13609, SEQ ID NO:13611, SEQ ID NO:13614, SEQ ID
NO:13616, SEQ ID NO:13618, SEQ ID NO:13628, SEQ ID NO:13630, SEQ ID NO:13632,
SEQ ID NO:13634, SEQ ID NO:13636, SEQ ID NO:13638, SEQ ID NO:13641, SEQ ID
NO:13644, SEQ ID NO:13646, SEQ ID NO:13648, SEQ ID NO:13650, SEQ ID NO:13654,
SEQ ID NO:13659, SEQ ID NO:13661, SEQ ID NO:13663, SEQ ID NO:13665, SEQ ID
NO:13667, SEQ ID NO:13669, SEQ ID NO:13671, SEQ ID NO:13673, SEQ ID NO:13677,
SEQ ID NO:13679, SEQ ID NO:13681, SEQ ID NO:13683, SEQ ID NO:13686, SEQ ID
NO:13688, SEQ ID NO:13691, SEQ ID NO:13695, SEQ ID NO:13697, SEQ ID NO:13699,
SEQ ID NO:13702, SEQ ID NO:13705, SEQ ID NO:13707, SEQ ID NO:13712, SEQ ID
NO:13714, SEQ ID NO:13716, SEQ ID NO:13719, SEQ ID NO:13721, SEQ ID NO:13723,
SEQ ID NO:13726, SEQ ID NO:13730, SEQ ID NO:13733, SEQ ID NO:13737, SEQ ID
NO:13739, SEQ ID NO:13741, SEQ ID NO:13743, SEQ ID NO:13745, SEQ ID NO:13747,
SEQ ID NO:13752, SEQ ID NO:13758, SEQ ID NO:13768, SEQ ID NO:13771, SEQ ID
NO:13773, SEQ ID NO:13776, SEQ ID NO:13781, SEQ ID NO:13784, SEQ ID NO:13787,
SEQ ID NO:13790, SEQ ID NO:13792, SEQ ID NO:13794, SEQ ID NO:13801, SEQ ID
NO:13807, SEQ ID NO:13811, SEQ ID NO:13813, SEQ ID NO:13815, SEQ ID NO:13817,
SEQ ID NO:13820, SEQ ID NO:13822, SEQ ID NO:13825, SEQ ID NO:13827, SEQ ID
NO:13830, SEQ ID NO:13832, SEQ ID NO:13835, SEQ ID NO:13837, SEQ ID NO:13840,
SEQ ID NO:13842, SEQ ID NO:13844, SEQ ID NO:13846, SEQ ID NO:13848, SEQ ID
NO:13853, SEQ ID NO:13855, SEQ ID NO:13857, SEQ ID NO:13859, SEQ ID NO:13862,
SEQ ID NO:13864,
SEQ ID NO:13866, SEQ ID NO:13868, SEQ ID NO:13871, SEQ ID NO:13873, SEQ ID
NO:13875, SEQ ID NO:13877, SEQ ID NO:13880, SEQ ID NO:13882, SEQ ID NO:13884,
SEQ ID NO:13886, SEQ ID NO:13888, SEQ ID NO:13890, SEQ ID NO:13894, SEQ ID
NO:13897, SEQ ID NO:13899, SEQ ID NO:13902, SEQ ID NO:13907, SEQ ID NO:13910,
SEQ ID NO:13913, SEQ ID NO:13915, SEQ ID NO:13917, SEQ ID NO:13919, SEQ ID
NO:13921, SEQ ID NO:13923, SEQ ID NO:13925, SEQ ID NO:13927, SEQ ID NO:13929,
SEQ ID NO:13931, SEQ ID NO:13934, SEQ ID NO:13937, SEQ ID NO:13939, SEQ ID
NO:13943, SEQ ID NO:13945, SEQ ID NO:13947, SEQ ID NO:13949, SEQ ID NO:13952,
SEQ ID NO:13955, SEQ ID NO:13957, SEQ ID NO:13960, SEQ ID NO:13963, SEQ ID
NO:13968, SEQ ID NO:13972, SEQ ID NO:13974, SEQ ID NO:13977, SEQ ID NO:13980,
 
SEQ ID NO:13987, SEQ ID NO:13991, SEQ ID NO:13993, SEQ ID NO:13995, SEQ ID
NO:13997, SEQ ID NO:14000, SEQ ID NO:14002, SEQ ID NO:14004, SEQ ID NO:14007,
SEQ ID NO:14009, SEQ ID NO:14011, SEQ ID NO:14017, SEQ ID NO:14020, SEQ ID
NO:14022, SEQ ID NO:14024, SEQ ID NO:14027, SEQ ID NO:14029, SEQ ID NO:14032,
SEQ ID NO:14034, SEQ ID NO:14036, SEQ ID NO:14038, SEQ ID NO:14040, SEQ ID
NO:14043, SEQ ID NO:14046, SEQ ID NO:14048, SEQ ID NO:14051, SEQ ID NO:14055,
SEQ ID NO:14057, SEQ ID NO:14059, SEQ ID NO:14061, SEQ ID NO:14063, SEQ ID
NO:14065, SEQ ID NO:14068, SEQ ID NO:14070, SEQ ID NO:14074, SEQ ID NO:14078,
SEQ ID NO:14080, SEQ ID NO:14082, SEQ ID NO:14087, SEQ ID NO:14089, SEQ ID
NO:14091, SEQ ID NO:14093, SEQ ID NO:14095, SEQ ID NO:14097, SEQ ID NO:14099,
SEQ ID NO:14101, SEQ ID NO:14103, SEQ ID NO:14105, SEQ ID NO:14107, SEQ ID
NO:14109, SEQ ID NO:14111, SEQ ID NO:14113, SEQ ID NO:14115, SEQ ID NO:14117,
SEQ ID NO:14119,
SEQ ID NO:14121, SEQ ID NO:14123, SEQ ID NO:14125, SEQ ID NO:14127, SEQ ID
NO:14130, SEQ ID NO:14132, SEQ ID NO:14134, SEQ ID NO:14136, SEQ ID NO:14138,
SEQ ID NO:14140, SEQ ID NO:14142, SEQ ID NO:14144, SEQ ID NO:14147, SEQ ID
NO:14149, SEQ ID NO:14151, SEQ ID NO:14153, SEQ ID NO:14155, SEQ ID NO:14157,
SEQ ID NO:14159, SEQ ID NO:14161, SEQ ID NO:14163, SEQ ID NO:14166, SEQ ID
NO:14170, SEQ ID NO:14172, SEQ ID NO:14174, SEQ ID NO:14176, SEQ ID NO:14178,
SEQ ID NO:14180, SEQ ID NO:14182, SEQ ID NO:14184, SEQ ID NO:14186, SEQ ID
NO:14189, SEQ ID NO:14191, SEQ ID NO:14193, SEQ ID NO:14195, SEQ ID NO:14197,
SEQ ID NO:14199, SEQ ID NO:14201, SEQ ID NO:14203, SEQ ID NO:14205, SEQ ID
NO:14207, SEQ ID NO:14209, SEQ ID NO:14211, SEQ ID NO:14213, SEQ ID NO:14215,
SEQ ID NO:14218, SEQ ID NO:14220, SEQ ID NO:14222, SEQ ID NO:14224, SEQ ID
NO:14226, SEQ ID NO:14228, SEQ ID NO:14230, SEQ ID NO:14232, SEQ ID NO:14234,
SEQ ID NO:14236, SEQ ID NO:14238, SEQ ID NO:14240, SEQ ID NO:14242, SEQ ID
NO:14244, SEQ ID NO:14246, SEQ ID NO:14248, SEQ ID NO:14250, SEQ ID NO:14252,
SEQ ID NO:14254, SEQ ID NO:14256, SEQ ID NO:14258, SEQ ID NO:14260, SEQ ID
NO:14262, SEQ ID NO:14264, SEQ ID NO:14267, SEQ ID NO:14269, SEQ ID NO:14271,
SEQ ID NO:14273, SEQ ID NO:14275, SEQ ID NO:14277, SEQ ID NO:14279, SEQ ID
NO:14281, SEQ ID NO:14283, SEQ ID NO:14285, SEQ ID NO:14288, SEQ ID NO:14290,
SEQ ID NO:14292, SEQ ID NO:14294, SEQ ID NO:14296, SEQ ID NO:14298, SEQ ID
NO:14301, SEQ ID NO:14303, SEQ ID NO:14306, SEQ ID NO:14308, SEQ ID NO:14310,
SEQ ID NO:14312, SEQ ID NO:14314, SEQ ID NO:14316, SEQ ID NO:14318, SEQ ID
NO:14320, SEQ ID NO:14322, SEQ ID NO:14324, SEQ ID NO:14326, SEQ ID NO:14328,
SEQ ID NO:14331,
SEQ ID NO:14334, SEQ ID NO:14337, SEQ ID NO:14340, SEQ ID NO:14342, SEQ ID
NO:14346, SEQ ID NO:14348, SEQ ID NO:14350, SEQ ID NO:14352, SEQ ID NO:14354,
SEQ ID NO:14356, SEQ ID NO:14358, SEQ ID NO:14360, SEQ ID NO:14362, SEQ ID
NO:14364, SEQ ID NO:14366, SEQ ID NO:14368, SEQ ID NO:14370, SEQ ID NO:14372,
SEQ ID NO:14374, SEQ ID NO:14376, SEQ ID NO:14379, SEQ ID NO:14381, SEQ ID
NO:14383, SEQ ID NO:14385, SEQ ID NO:14387, SEQ ID NO:14389, SEQ ID NO:14391,
SEQ ID NO:14393, SEQ ID NO:14396, SEQ ID NO:14398, SEQ ID NO:14401, SEQ ID
NO:14404, SEQ ID NO:14406, SEQ ID NO:14408, SEQ ID NO:14410, SEQ ID NO:14412,
SEQ ID NO:14414, SEQ ID NO:14416, SEQ ID NO:14418, SEQ ID NO:14420, SEQ ID
NO:14423, SEQ ID NO:14425, SEQ ID NO:14427, SEQ ID NO:14429, SEQ ID NO:14431,
SEQ ID NO:14433, SEQ ID NO:14435, SEQ ID NO:14437, SEQ ID NO:14440, SEQ ID
NO:14442, SEQ ID NO:14444, SEQ ID NO:14446, SEQ ID NO:14448, SEQ ID NO:14450,
SEQ ID NO:14452, SEQ ID NO:14454, SEQ ID NO:14456, SEQ ID NO:14458, SEQ ID
NO:14460, SEQ ID NO:14462, SEQ ID NO:14466, SEQ ID NO:14468, SEQ ID NO:14470,
SEQ ID NO:14472, SEQ ID NO:14474, SEQ ID NO:14476, SEQ ID NO:14478, SEQ ID
NO:14480, SEQ ID NO:14482, SEQ ID NO:14484, SEQ ID NO:14486, SEQ ID NO:14488,
 
SEQ ID NO:14490, SEQ ID NO:14492, SEQ ID NO:14494, SEQ ID NO:14496, SEQ ID
NO:14498, SEQ ID NO:14500, SEQ ID NO:14502, SEQ ID NO:14505, SEQ ID NO:14507,
SEQ ID NO:14509, SEQ ID NO:14511, SEQ ID NO:14514, SEQ ID NO:14516, SEQ ID
NO:14518, SEQ ID NO:14520, SEQ ID NO:14522, SEQ ID NO:14524, SEQ ID NO:14526,
SEQ ID NO:14530, SEQ ID NO:14532, SEQ ID NO:14534, SEQ ID NO:14536, SEQ ID
NO:14538, SEQ ID NO:14541, SEQ ID NO:14543, SEQ ID NO:14546, SEQ ID NO:14548,
SEQ ID NO:14550,
SEQ ID NO:14553, SEQ ID NO:14555, SEQ ID NO:14557, SEQ ID NO:14559, SEQ ID
NO:14561, SEQ ID NO:14563, SEQ ID NO:14565, SEQ ID NO:14567, SEQ ID NO:14569,
SEQ ID NO:14571, SEQ ID NO:14573, SEQ ID NO:14575, SEQ ID NO:14577, SEQ ID
NO:14579, SEQ ID NO:14581, SEQ ID NO:14583, SEQ ID NO:14585, SEQ ID NO:14587,
SEQ ID NO:14589, SEQ ID NO:14591, SEQ ID NO:14593, SEQ ID NO:14596, SEQ ID
NO:14598, SEQ ID NO:14600, SEQ ID NO:14602, SEQ ID NO:14604, SEQ ID NO:14606,
SEQ ID NO:14608, SEQ ID NO:14612, SEQ ID NO:14614, SEQ ID NO:14616, SEQ ID
NO:14618, SEQ ID NO:14621, SEQ ID NO:14623, SEQ ID NO:14625, SEQ ID NO:14627,
SEQ ID NO:14629, SEQ ID NO:14631, SEQ ID NO:14634, SEQ ID NO:14636, SEQ ID
NO:14638, SEQ ID NO:14640, SEQ ID NO:14642, SEQ ID NO:14645, SEQ ID NO:14647,
SEQ ID NO:14649, SEQ ID NO:14651, SEQ ID NO:14653, SEQ ID NO:14655, SEQ ID
NO:14657, SEQ ID NO:14659, SEQ ID NO:14661, SEQ ID NO:14663, SEQ ID NO:14665,
SEQ ID NO:14667, SEQ ID NO:14669, SEQ ID NO:14671, SEQ ID NO:14673, SEQ ID
NO:14675, SEQ ID NO:14677, SEQ ID NO:14680, SEQ ID NO:14682, SEQ ID NO:14684,
SEQ ID NO:14687, SEQ ID NO:14689, SEQ ID NO:14691, SEQ ID NO:14694, SEQ ID
NO:14696, SEQ ID NO:14698, SEQ ID NO:14701, SEQ ID NO:14703, SEQ ID NO:14705,
SEQ ID NO:14707, SEQ ID NO:14709, SEQ ID NO:14711, SEQ ID NO:14714, SEQ ID
NO:14716, SEQ ID NO:14718, SEQ ID NO:14720, SEQ ID NO:14723, SEQ ID NO:14726,
SEQ ID NO:14728, SEQ ID NO:14730, SEQ ID NO:14733, SEQ ID NO:14736, SEQ ID
NO:14739, SEQ ID NO:14741, SEQ ID NO:14743, SEQ ID NO:14745, SEQ ID NO:14747,
SEQ ID NO:14749, SEQ ID NO:14751, SEQ ID NO:14753, SEQ ID NO:14756, SEQ ID
NO:14759, SEQ ID NO:14761, SEQ ID NO:14763, SEQ ID NO:14765, SEQ ID NO:14767,
SEQ ID NO:14769,
SEQ ID NO:14771, SEQ ID NO:14773, SEQ ID NO:14775, SEQ ID NO:14777, SEQ ID
NO:14779, SEQ ID NO:14781, SEQ ID NO:14783, SEQ ID NO:14785, SEQ ID NO:14787,
SEQ ID NO:14789, SEQ ID NO:14791, SEQ ID NO:14793, SEQ ID NO:14795, SEQ ID
NO:14797, SEQ ID NO:14799, SEQ ID NO:14801, SEQ ID NO:14803, SEQ ID NO:14805,
SEQ ID NO:14807, SEQ ID NO:14810, SEQ ID NO:14813, SEQ ID NO:14815, SEQ ID
NO:14817, SEQ ID NO:14819, SEQ ID NO:14821, SEQ ID NO:14825, SEQ ID NO:14827,
SEQ ID NO:14829, SEQ ID NO:14832, SEQ ID NO:14834, SEQ ID NO:14836, SEQ ID
NO:14838, SEQ ID NO:14840, SEQ ID NO:14842, SEQ ID NO:14844, SEQ ID NO:14846,
SEQ ID NO:14848, SEQ ID NO:14851, SEQ ID NO:14853, SEQ ID NO:14855, SEQ ID
NO:14857, SEQ ID NO:14859, SEQ ID NO:14861, SEQ ID NO:14863, SEQ ID NO:14866,
SEQ ID NO:14868, SEQ ID NO:14870, SEQ ID NO:14873, SEQ ID NO:14875, SEQ ID
NO:14877, SEQ ID NO:14879, SEQ ID NO:14881, SEQ ID NO:14883, SEQ ID NO:14885,
SEQ ID NO:14887, SEQ ID NO:14889, SEQ ID NO:14891, SEQ ID NO:14893, SEQ ID
NO:14895, SEQ ID NO:14897, SEQ ID NO:14899, SEQ ID NO:14901, SEQ ID NO:14903,
SEQ ID NO:14905, SEQ ID NO:14907, SEQ ID NO:14909, SEQ ID NO:14911, SEQ ID
NO:14913, SEQ ID NO:14915, SEQ ID NO:14917, SEQ ID NO:14920, SEQ ID NO:14922,
SEQ ID NO:14926, SEQ ID NO:14928, SEQ ID NO:14930, SEQ ID NO:14932, SEQ ID
NO:14934, SEQ ID NO:14936, SEQ ID NO:14939, SEQ ID NO:14941, SEQ ID NO:14943,
SEQ ID NO:14945, SEQ ID NO:14947, SEQ ID NO:14949, SEQ ID NO:14951, SEQ ID
NO:14953, SEQ ID NO:14955, SEQ ID NO:14957, SEQ ID NO:14959, SEQ ID NO:14962,
SEQ ID NO:14964, SEQ ID NO:14966, SEQ ID NO:14968, SEQ ID NO:14970, SEQ ID
 
NO:14972, SEQ ID NO:14974, SEQ ID NO:14976, SEQ ID NO:14978, SEQ ID NO:14980,
SEQ ID NO:14983,
SEQ ID NO:14985, SEQ ID NO:14989, SEQ ID NO:14991, SEQ ID NO:14995, SEQ ID
NO:14997, SEQ ID NO:14999, SEQ ID NO:15001, SEQ ID NO:15003, SEQ ID NO:15008,
SEQ ID NO:15010, SEQ ID NO:15014, SEQ ID NO:15016, SEQ ID NO:15018, SEQ ID
NO:15020, SEQ ID NO:15022, SEQ ID NO:15024, SEQ ID NO:15027, SEQ ID NO:15030,
SEQ ID NO:15032, SEQ ID NO:15034, SEQ ID NO:15036, SEQ ID NO:15038, SEQ ID
NO:15041, SEQ ID NO:15043, SEQ ID NO:15045, SEQ ID NO:15047, SEQ ID NO:15049,
SEQ ID NO:15052, SEQ ID NO:15054, SEQ ID NO:15056, SEQ ID NO:15058, SEQ ID
NO:15061, SEQ ID NO:15063, SEQ ID NO:15065, SEQ ID NO:15068, SEQ ID NO:15070,
SEQ ID NO:15072, SEQ ID NO:15074, SEQ ID NO:15076, SEQ ID NO:15079, SEQ ID
NO:15081, SEQ ID NO:15083, SEQ ID NO:15085, SEQ ID NO:15087, SEQ ID NO:15089,
SEQ ID NO:15092, SEQ ID NO:15094, SEQ ID NO:15097, SEQ ID NO:15100, SEQ ID
NO:15102, SEQ ID NO:15104, SEQ ID NO:15106, SEQ ID NO:15108, SEQ ID NO:15110,
SEQ ID NO:15112, SEQ ID NO:15114, SEQ ID NO:15116, SEQ ID NO:15118, SEQ ID
NO:15120, SEQ ID NO:15122, SEQ ID NO:15124, SEQ ID NO:15126, SEQ ID NO:15128,
SEQ ID NO:15131, SEQ ID NO:15133, SEQ ID NO:15135, SEQ ID NO:15137, SEQ ID
NO:15139, SEQ ID NO:15141, SEQ ID NO:15143, SEQ ID NO:15145, SEQ ID NO:15149,
SEQ ID NO:15151, SEQ ID NO:15153, SEQ ID NO:15156, SEQ ID NO:15158, SEQ ID
NO:15160, SEQ ID NO:15162, SEQ ID NO:15164, SEQ ID NO:15166, SEQ ID NO:15170,
SEQ ID NO:15172, SEQ ID NO:15174, SEQ ID NO:15176, SEQ ID NO:15178, SEQ ID
NO:15180, SEQ ID NO:15182, SEQ ID NO:15184, SEQ ID NO:15186, SEQ ID NO:15188,
SEQ ID NO:15190, SEQ ID NO:15193, SEQ ID NO:15195, SEQ ID NO:15197, SEQ ID
NO:15199, SEQ ID NO:15202, SEQ ID NO:15204, SEQ ID NO:15206, SEQ ID NO:15208,
SEQ ID NO:15210,
SEQ ID NO:15212, SEQ ID NO:15214, SEQ ID NO:15216, SEQ ID NO:15218, SEQ ID
NO:15221, SEQ ID NO:15223, SEQ ID NO:15225, SEQ ID NO:15227, SEQ ID NO:15229,
SEQ ID NO:15231, SEQ ID NO:15233, SEQ ID NO:15235, SEQ ID NO:15237, SEQ ID
NO:15239, SEQ ID NO:15241, SEQ ID NO:15243, SEQ ID NO:15245, SEQ ID NO:15247,
SEQ ID NO:15249, SEQ ID NO:15251, SEQ ID NO:15253, SEQ ID NO:15255, SEQ ID
NO:15257, SEQ ID NO:15260, SEQ ID NO:15262, SEQ ID NO:15264, SEQ ID NO:15266,
SEQ ID NO:15268, SEQ ID NO:15270, SEQ ID NO:15273, SEQ ID NO:15275, SEQ ID
NO:15277, SEQ ID NO:15279, SEQ ID NO:15281, SEQ ID NO:15284, SEQ ID NO:15286,
SEQ ID NO:15288, SEQ ID NO:15290, SEQ ID NO:15293, SEQ ID NO:15296, SEQ ID
NO:15299, SEQ ID NO:15303, SEQ ID NO:15306, SEQ ID NO:15308, SEQ ID NO:15310,
SEQ ID NO:15312, SEQ ID NO:15314, SEQ ID NO:15317, SEQ ID NO:15320, SEQ ID
NO:15322, SEQ ID NO:15324, SEQ ID NO:15326, SEQ ID NO:15328, SEQ ID NO:15330,
SEQ ID NO:15332, SEQ ID NO:15335, SEQ ID NO:15337, SEQ ID NO:15339, SEQ ID
NO:15341, SEQ ID NO:15343, SEQ ID NO:15345, SEQ ID NO:15347, SEQ ID NO:15349,
SEQ ID NO:15351, SEQ ID NO:15353, SEQ ID NO:15356, SEQ ID NO:15358, SEQ ID
NO:15360, SEQ ID NO:15362, SEQ ID NO:15365, SEQ ID NO:15367, SEQ ID NO:15369,
SEQ ID NO:15371, SEQ ID NO:15373, SEQ ID NO:15375, SEQ ID NO:15377, SEQ ID
NO:15379, SEQ ID NO:15381, SEQ ID NO:15383, SEQ ID NO:15385, SEQ ID NO:15387,
SEQ ID NO:15389, SEQ ID NO:15392, SEQ ID NO:15394, SEQ ID NO:15398, SEQ ID
NO:15400, SEQ ID NO:15402, SEQ ID NO:15405, SEQ ID NO:15408, SEQ ID NO:15410,
SEQ ID NO:15413, SEQ ID NO:15415, SEQ ID NO:15418, SEQ ID NO:15420, SEQ ID
NO:15422, SEQ ID NO:15424, SEQ ID NO:15426, SEQ ID NO:15428, SEQ ID NO:15430,
SEQ ID NO:15432,
SEQ ID NO:15434, SEQ ID NO:15437, SEQ ID NO:15440, SEQ ID NO:15444, SEQ ID
NO:15452, SEQ ID NO:15454, SEQ ID NO:15456, SEQ ID NO:15458, SEQ ID NO:15460,
SEQ ID NO:15462, SEQ ID NO:15464, SEQ ID NO:15466, SEQ ID NO:15468, SEQ ID
NO:15470, SEQ ID NO:15472, SEQ ID NO:15474, SEQ ID NO:15476, SEQ ID NO:15479,
 
SEQ ID NO:15481, SEQ ID NO:15483, SEQ ID NO:15486, SEQ ID NO:15488, SEQ ID
NO:15490, SEQ ID NO:15493, SEQ ID NO:15495, SEQ ID NO:15499, SEQ ID NO:15504,
SEQ ID NO:15506, SEQ ID NO:15508, SEQ ID NO:15512, SEQ ID NO:15515, SEQ ID
NO:15517, SEQ ID NO:15519, SEQ ID NO:15521, SEQ ID NO:15523, SEQ ID NO:15526,
SEQ ID NO:15530, SEQ ID NO:15532, SEQ ID NO:15536, SEQ ID NO:15538, SEQ ID
NO:15541, SEQ ID NO:15543, SEQ ID NO:15546, SEQ ID NO:15549, SEQ ID NO:15553,
SEQ ID NO:15555, SEQ ID NO:15559, SEQ ID NO:15562, SEQ ID NO:15566, SEQ ID
NO:15568, SEQ ID NO:15570, SEQ ID NO:15572, SEQ ID NO:15574, SEQ ID NO:15576,
SEQ ID NO:15579, SEQ ID NO:15583, SEQ ID NO:15586, SEQ ID NO:15588, SEQ ID
NO:15590, SEQ ID NO:15595, SEQ ID NO:15597, SEQ ID NO:15600, SEQ ID NO:15605,
SEQ ID NO:15609, SEQ ID NO:15612, SEQ ID NO:15614, SEQ ID NO:15616, SEQ ID
NO:15618, SEQ ID NO:15620, SEQ ID NO:15622, SEQ ID NO:15624, SEQ ID NO:15626,
SEQ ID NO:15628, SEQ ID NO:15631, SEQ ID NO:15633, SEQ ID NO:15635, SEQ ID
NO:15637, SEQ ID NO:15639, SEQ ID NO:15641, SEQ ID NO:15643, SEQ ID NO:15645,
SEQ ID NO:15647, SEQ ID NO:15652, SEQ ID NO:15654, SEQ ID NO:15657, SEQ ID
NO:15659, SEQ ID NO:15662, SEQ ID NO:15664, SEQ ID NO:15666, SEQ ID NO:15668,
SEQ ID NO:15673, SEQ ID NO:15678, SEQ ID NO:15680, SEQ ID NO:15682, SEQ ID
NO:15684, SEQ ID NO:15686, SEQ ID NO:15688, SEQ ID NO:15691, SEQ ID NO:15693,
SEQ ID NO:15696,
SEQ ID NO:15698, SEQ ID NO:15700, SEQ ID NO:15702, SEQ ID NO:15705, SEQ ID
NO:15708, SEQ ID NO:15710, SEQ ID NO:15712, SEQ ID NO:15714, SEQ ID NO:15716,
SEQ ID NO:15721, SEQ ID NO:15724, SEQ ID NO:15726, SEQ ID NO:15730, SEQ ID
NO:15733, SEQ ID NO:15736, SEQ ID NO:15739, SEQ ID NO:15741, SEQ ID NO:15743,
SEQ ID NO:15745, SEQ ID NO:15747, SEQ ID NO:15750, SEQ ID NO:15753, SEQ ID
NO:15755, SEQ ID NO:15757, SEQ ID NO:15759, SEQ ID NO:15761, SEQ ID NO:15763,
SEQ ID NO:15766, SEQ ID NO:15768, SEQ ID NO:15770, SEQ ID NO:15772, SEQ ID
NO:15774, SEQ ID NO:15776, SEQ ID NO:15779, SEQ ID NO:15782, SEQ ID NO:15784,
SEQ ID NO:15786, SEQ ID NO:15788, SEQ ID NO:15790, SEQ ID NO:15792, SEQ ID
NO:15794, SEQ ID NO:15796, SEQ ID NO:15798, SEQ ID NO:15800, SEQ ID NO:15803,
SEQ ID NO:15805, SEQ ID NO:15807, SEQ ID NO:15809, SEQ ID NO:15811, SEQ ID
NO:15813, SEQ ID NO:15815, SEQ ID NO:15817, SEQ ID NO:15822, SEQ ID NO:15824,
SEQ ID NO:15826, SEQ ID NO:15828, SEQ ID NO:15834, SEQ ID NO:15838, SEQ ID
NO:15840, SEQ ID NO:15842, SEQ ID NO:15844, SEQ ID NO:15847, SEQ ID NO:15849,
SEQ ID NO:15851, SEQ ID NO:15854, SEQ ID NO:15856, SEQ ID NO:15858, SEQ ID
NO:15860, SEQ ID NO:15862, SEQ ID NO:15864, SEQ ID NO:15868, SEQ ID NO:15871,
SEQ ID NO:15873, SEQ ID NO:15875, SEQ ID NO:15877, SEQ ID NO:15879, SEQ ID
NO:15884, SEQ ID NO:15886, SEQ ID NO:15888, SEQ ID NO:15890, SEQ ID NO:15894,
SEQ ID NO:15896, SEQ ID NO:15898, SEQ ID NO:15902, SEQ ID NO:15904, SEQ ID
NO:15907, SEQ ID NO:15911, SEQ ID NO:15913, SEQ ID NO:15915, SEQ ID NO:15918,
SEQ ID NO:15921, SEQ ID NO:15923, SEQ ID NO:15925, SEQ ID NO:15927, SEQ ID
NO:15931, SEQ ID NO:15933, SEQ ID NO:15935, SEQ ID NO:15937, SEQ ID NO:15939,
SEQ ID NO:15941,
SEQ ID NO:15943, SEQ ID NO:15945, SEQ ID NO:15947, SEQ ID NO:15949, SEQ ID
NO:15951, SEQ ID NO:15953, SEQ ID NO:15955, SEQ ID NO:15957, SEQ ID NO:15959,
SEQ ID NO:15961, SEQ ID NO:15963, SEQ ID NO:15965, SEQ ID NO:15967, SEQ ID
NO:15969, SEQ ID NO:15971, SEQ ID NO:15973, SEQ ID NO:15975, SEQ ID NO:15977,
SEQ ID NO:15979, SEQ ID NO:15981, SEQ ID NO:15983, SEQ ID NO:15985, SEQ ID
NO:15987, SEQ ID NO:15989, SEQ ID NO:15991, SEQ ID NO:15994, SEQ ID NO:15996,
SEQ ID NO:15998, SEQ ID NO:16000, SEQ ID NO:16002, SEQ ID NO:16004, SEQ ID
NO:16006, SEQ ID NO:16008, SEQ ID NO:16010, SEQ ID NO:16012, SEQ ID NO:16014,
SEQ ID NO:16016, SEQ ID NO:16018, SEQ ID NO:16020, SEQ ID NO:16022, SEQ ID
NO:16024, SEQ ID NO:16026, SEQ ID NO:16028, SEQ ID NO:16030, SEQ ID NO:16032,
 
SEQ ID NO:16034, SEQ ID NO:16036, SEQ ID NO:16038, SEQ ID NO:16040, SEQ ID .
NO:16042, SEQ ID NO:16044, SEQ ID NO:16046, SEQ ID NO:16050, SEQ ID NO:16053,
SEQ ID NO:16055, SEQ ID NO:16058, SEQ ID NO:16060, SEQ ID NO:16062, SEQ ID
NO:16064, SEQ ID NO:16066, SEQ ID NO:16068, SEQ ID NO:16070, SEQ ID NO:16072,
SEQ ID NO:16074, SEQ ID NO:16076, SEQ ID NO:16078, SEQ ID NO:16080, SEQ ID
NO:16082, SEQ ID NO:16084, SEQ ID NO:16086, SEQ ID NO:16088, SEQ ID NO:16090,
SEQ ID NO:16092, SEQ ID NO:16094, SEQ ID NO:16096, SEQ ID NO:16098, SEQ ID
NO:16100, SEQ ID NO:16102, SEQ ID NO:16104, SEQ ID NO:16106, SEQ ID NO:16108,
SEQ ID NO:16110,
SEQ ID NO:16221, SEQ ID NO:16223, SEQ ID NO:16227, SEQ ID NO:16231, SEQ ID
NO:16234, SEQ ID NO:16237, SEQ ID NO:16239, SEQ ID NO:16241, SEQ ID NO:16244,
SEQ ID NO:16256, SEQ ID NO:16263, SEQ ID NO:16271, SEQ ID NO:16288, SEQ ID
NO:16290, SEQ ID NO:16292, SEQ ID NO:16296, SEQ ID NO:16298, SEQ ID NO:16300,
SEQ ID NO:16302, SEQ ID NO:16305, SEQ ID NO:16311, SEQ ID NO:16313, SEQ ID
NO:16326, SEQ ID NO:16329, SEQ ID NO:16335, SEQ ID NO:16342, SEQ ID NO:16344,
SEQ ID NO:16349, SEQ ID NO:16355, SEQ ID NO:16357, SEQ ID NO:16361, SEQ ID
NO:16366, SEQ ID NO:16368, SEQ ID NO:16370, SEQ ID NO:16375, SEQ ID NO:16382,
SEQ ID NO:16386, SEQ ID NO:16388, SEQ ID NO:16390, SEQ ID NO:16392, SEQ ID
NO:16397, SEQ ID NO:16399, SEQ ID NO:16405, SEQ ID NO:16407, SEQ ID NO:16410,
SEQ ID NO:16413, SEQ ID NO:16415, SEQ ID NO:16417, SEQ ID NO:16419, SEQ ID
NO:16430, SEQ ID NO:16432, SEQ ID NO:16434, SEQ ID NO:16439, SEQ ID NO:16442,
SEQ ID NO:16444, SEQ ID NO:16446, SEQ ID NO:16463, SEQ ID NO:16466, SEQ ID
NO:16468, SEQ ID NO:16470, SEQ ID NO:16472, SEQ ID NO:16475, SEQ ID NO:16477,-
SEQ ID NO:16480, SEQ ID NO:16482, SEQ ID NO:16485,.SEQ ID NO:16488, SEQ ID
NO:16491, SEQ ID NO:16493, SEQ ID NO:16495, SEQ ID NO:16498, SEQ ID NO:16502,
SEQ ID NO:16504, SEQ ID NO:16507, SEQ ID NO:16510, SEQ ID NO:16521, SEQ ID
NO:16523, SEQ ID NO:16525, SEQ ID NO:16528, SEQ ID NO:16530, SEQ ID NO:16533,
SEQ ID NO:16538, SEQ ID NO:16541, SEQ ID NO:16543, SEQ ID NO:16545, SEQ ID
NO:16549, SEQ ID NO:16551, SEQ ID NO:16554, SEQ ID NO:16556, SEQ ID NO:16558,
SEQ ID NO:16560, SEQ ID NO:16562, SEQ ID NO:16566, SEQ ID NO:16572, SEQ ID
NO:16582, SEQ ID NO:16584, SEQ ID NO:16587, SEQ ID NO:16590, SEQ ID NO:16592,
SEQ ID NO:16595,
SEQ ID NO:16599, SEQ ID NO:16602, SEQ ID NO:16605, SEQ ID NO:16610, SEQ ID
NO:16616, SEQ ID NO:16619, SEQ ID NO:16621, SEQ ID NO:16623, SEQ ID NO:16625,
SEQ ID NO:16630, SEQ ID NO:16632, SEQ ID NO:16634, SEQ ID NO:16638, SEQ ID
NO:16641, SEQ ID NO:16644, SEQ ID NO:16663, SEQ ID NO:16665, SEQ ID NO:16674,
SEQ ID NO:16680, SEQ ID NO:16685, SEQ ID NO:16688, SEQ ID NO:16690, SEQ ID
NO:16693, SEQ ID NO:16699, SEQ ID NO:16702, SEQ ID NO:16704, SEQ ID NO:16708,
SEQ ID NO:16712, SEQ ID NO:16714, SEQ ID NO:16723, SEQ ID NO:16726, SEQ ID
NO:16728, SEQ ID NO:16730, SEQ ID NO:16732, SEQ ID NO:16741, SEQ ID NO:16743,
SEQ ID NO:16745, SEQ ID NO:16747, SEQ ID NO:16752, SEQ ID NO:16763, SEQ ID
NO:16765, SEQ ID NO:16767, SEQ ID NO:16771, SEQ ID NO:16776, SEQ ID NO:16781,
SEQ ID NO:16784, SEQ ID NO:16786, SEQ ID NO:16788, SEQ ID NO:16791, SEQ ID
NO:16793, SEQ ID NO:16795, SEQ ID NO:16797, SEQ ID NO:16801, SEQ ID NO:16804,
SEQ ID NO:16807, SEQ ID NO:16809, SEQ ID NO:16813, SEQ ID NO:16815, SEQ ID
NO:16818, SEQ ID NO:16822, SEQ ID NO:16825, SEQ ID NO:16845, SEQ ID NO:16847,
SEQ ID NO:16849, SEQ ID NO:16853, SEQ ID NO:16855, SEQ ID NO:16857, SEQ ID
NO:16863, SEQ ID NO:16865, SEQ ID NO:16870, SEQ ID NO:16876, SEQ ID NO:16879,
SEQ ID NO:16884, SEQ ID NO:16892, SEQ ID NO:16896, SEQ ID NO:16901, SEQ ID
NO:16903, SEQ ID NO:16909, SEQ ID NO:16921, SEQ ID NO:16925, SEQ ID NO:16928,
SEQ ID NO:16935, SEQ ID NO:16937, SEQ ID NO:16939, SEQ ID NO:16941, SEQ ID
NO:16943, SEQ ID NO:16947, SEQ ID NO:16954, SEQ ID NO:16956, SEQ ID NO:16960,
 
SEQ ID NO:16963, SEQ ID NO:16965, SEQ ID NO:16968, SEQ ID NO:16971, SEQ ID
NO:16976, SEQ ID NO:16980, SEQ ID NO:16987, SEQ ID NO:16990, SEQ ID NO:16999,
SEQ ID NO:17003,
SEQ ID NO:17019, SEQ ID NO:17025, SEQ ID NO:17028, SEQ ID NO:17032, SEQ ID
NO:17038, SEQ ID NO:17040, SEQ ID NO:17042, SEQ ID NO:17051, SEQ ID NO:17053,
SEQ ID NO:17058, SEQ ID NO:17060, SEQ ID NO:17062, SEQ ID NO:17064, SEQ ID
NO:17072, SEQ ID NO:17074, SEQ ID NO:17076, SEQ ID NO:17079, SEQ ID NO:17081,
SEQ ID NO:17083, SEQ ID NO:17085, SEQ ID NO:17087, SEQ ID NO:17089, SEQ ID
NO:17091, SEQ ID NO:17093, SEQ ID NO:17095, SEQ ID NO:17097, SEQ ID NO:17099,
SEQ ID NO:17101, SEQ ID NO:17104, SEQ ID NO:17106, SEQ ID NO:17109, SEQ ID
NO:17111, SEQ ID NO:17113, SEQ ID NO:17115, SEQ ID NO:17117, SEQ ID NO:17120,
SEQ ID NO:17122, SEQ ID NO:17124, SEQ ID NO:17127, SEQ ID NO:17129, SEQ ID
NO:17131, SEQ ID NO:17133, SEQ ID NO:17135, SEQ ID NO:17137, SEQ ID NO:17140,
SEQ ID NO:17142, SEQ ID NO:17144, SEQ ID NO:17146, SEQ ID NO:17148, SEQ ID
NO:17150, SEQ ID NO:17152, SEQ ID NO:17154, SEQ ID NO:17156, SEQ ID NO:17158,
SEQ ID NO:17161, SEQ ID NO:17163, SEQ ID NO:17165, SEQ ID NO:17167, SEQ ID
NO:17169, SEQ ID NO:17171, SEQ ID NO:17173, SEQ ID NO:17175, SEQ ID NO:17177,
SEQ ID NO:17179, SEQ ID NO:17181, SEQ ID NO:17184, SEQ ID NO:17186, SEQ ID
NO:17188, SEQ ID NO:17191, SEQ ID NO:17194, SEQ ID NO:17196, SEQ ID NO:17198,
SEQ ID NO:17200, SEQ ID NO:17203, SEQ ID NO:17205, SEQ ID NO:17207, SEQ ID
NO:17211, SEQ ID NO:17215, SEQ ID NO:17217, SEQ ID NO:17219, SEQ ID NO:17221,
SEQ ID NO:17223, SEQ ID NO:17225, SEQ ID NO:17227, SEQ ID NO:17229, SEQ ID
NO:17231, SEQ ID NO:17233, SEQ ID NO:17235, SEQ ID NO:17237, SEQ ID NO:17239,
SEQ ID NO:17241, SEQ ID NO:17243, SEQ ID NO:17245, SEQ ID NO:17247, SEQ ID
NO:17250, SEQ ID NO:17252, SEQ ID NO:17254, SEQ ID NO:17257, SEQ ID NO:17260,
SEQ ID NO:17262,
SEQ ID NO:17266, SEQ ID NO:17269, SEQ ID NO:17271, SEQ ID NO:17273, SEQ ID
NO:17276, SEQ ID NO:17278, SEQ ID NO:17280, SEQ ID NO:17282, SEQ ID NO:17284,
SEQ ID NO:17286, SEQ ID NO:17288, SEQ ID NO:17291, SEQ ID NO:17293, SEQ ID
NO:17295, SEQ ID NO:17298, SEQ ID NO:17301, SEQ ID NO:17303, SEQ ID NO:17306,
SEQ ID NO:17308, SEQ ID NO:17311, SEQ ID NO:17313, SEQ ID NO:17317, SEQ ID
NO:17319, SEQ ID NO:17321, SEQ ID NO:17323, SEQ ID NO:17325, SEQ ID NO:17327,
SEQ ID NO:17329, SEQ ID NO:17331, SEQ ID NO:17333, SEQ ID NO:17335, SEQ ID
NO:17337, SEQ ID NO:17339, SEQ ID NO:17342, SEQ ID NO:17346, SEQ ID NO:17348,
SEQ ID NO:17350, SEQ ID NO:17352, SEQ ID NO:17354, SEQ ID NO:17357, SEQ ID
NO:17359, SEQ ID NO:17361, SEQ ID NO:17363, SEQ ID NO:17367, SEQ ID NO:17369,
SEQ ID NO:17373, SEQ ID NO:17375, SEQ ID NO:17379, SEQ ID NO:17382, SEQ ID
NO:17384, SEQ ID NO:17386, SEQ ID NO:17389, SEQ ID NO:17391, SEQ ID NO:17394,
SEQ ID NO:17396, SEQ ID NO:17400, SEQ ID NO:17403, SEQ ID NO:17405, SEQ ID
NO:17407, SEQ ID NO:17409, SEQ ID NO:17411, SEQ ID NO:17413, SEQ ID NO:17415,
SEQ ID NO:17417, SEQ ID NO:17419, SEQ ID NO:17421, SEQ ID NO:17423, SEQ ID
NO:17425, SEQ ID NO:17428, SEQ ID NO:17431, SEQ ID NO:17435, SEQ ID NO:17439,
SEQ ID NO:17441, SEQ ID NO:17444, SEQ ID NO:17446, SEQ ID NO:17448, SEQ ID
NO:17451, SEQ ID NO:17453, SEQ ID NO:17455, SEQ ID NO:17458, SEQ ID NO:17462,
SEQ ID NO:17464, SEQ ID NO:17466, SEQ ID NO:17469, SEQ ID NO:17471, SEQ ID
NO:17473, SEQ ID NO:17476, SEQ ID NO:17478, SEQ ID NO:17481, SEQ ID NO:17483,
SEQ ID NO:17486, SEQ ID NO:17488, SEQ ID NO:17491, SEQ ID NO:17493, SEQ ID
NO:17495, SEQ ID NO:17497, SEQ ID NO:17499, SEQ ID NO:17501, SEQ ID NO:17503,
SEQ ID NO:17505,
SEQ ID NO:17507, SEQ ID NO:17509, SEQ ID NO:17511, SEQ ID NO:17513, SEQ ID
NO:17515, SEQ ID NO:17517, SEQ ID NO:17519, SEQ ID NO:17521, SEQ ID NO:17523,
SEQ ID NO:17525, SEQ ID NO:17528, SEQ ID NO:17530, SEQ ID NO:17534, SEQ ID
 
NO:17536, SEQ ID NO:17538, SEQ ID NO:17540, SEQ ID NO:17542, SEQ ID NO:17544,
SEQ ID NO:17546, SEQ ID NO:17548, SEQ ID NO:17550, SEQ ID NO:17552, SEQ ID
NO:17554, SEQ ID NO:17556, SEQ ID NO:17558, SEQ ID NO:17560, SEQ ID NO:17562,
SEQ ID NO:17564, SEQ ID NO:17566, SEQ ID NO:17568, SEQ ID NO:17570, SEQ ID
NO:17573, SEQ ID NO:17575, SEQ ID NO:17577, SEQ ID NO:17580, SEQ ID NO:17582,
SEQ ID NO:17584, SEQ ID NO:17587, SEQ ID NO:17590, SEQ ID NO:17592, SEQ ID
NO:17595, SEQ ID NO:17597, SEQ ID NO:17599, SEQ ID NO:17601, SEQ ID NO:17603,
SEQ ID NO:17605, SEQ ID NO:17607, SEQ ID NO:17609, SEQ ID NO:17611, SEQ ID
NO:17613, SEQ ID NO:17615, SEQ ID NO:17617, SEQ ID NO:17619, SEQ ID NO:17621,
SEQ ID NO:17623, SEQ ID NO:17625, SEQ ID NO:17627, SEQ ID NO:17629, SEQ ID
NO:17631, SEQ ID NO:17633, SEQ ID NO:17635, SEQ ID NO:17637, SEQ ID NO:17639,
SEQ ID NO:17641, SEQ ID NO:17643, SEQ ID NO:17645, SEQ ID NO:17647, SEQ ID
NO:17651, SEQ ID NO:17653, SEQ ID NO:17655, SEQ ID NO:17658, SEQ ID NO:17661,
SEQ ID NO:17664, SEQ ID NO:17667, SEQ ID NO:17669, SEQ ID NO:17671, SEQ ID
NO:17673, SEQ ID NO:17675, SEQ ID NO:17677, SEQ ID NO:17679, SEQ ID NO:17684,
SEQ ID NO:17686, SEQ ID NO:17688, SEQ ID NO:17690, SEQ ID NO:17692, SEQ ID
NO:17694, SEQ ID NO:17696, SEQ ID NO:17698, SEQ ID NO:17700, SEQ ID NO:17702,
SEQ ID NO:17704, SEQ ID NO:17707, SEQ ID NO:17709, SEQ ID NO:17711, SEQ ID
NO:17713, SEQ ID NO:17715, SEQ ID NO:17719, SEQ ID NO:17725, SEQ ID NO:17727,
SEQ ID NO:17731,
SEQ ID NO:17734, SEQ ID NO:17736, SEQ ID NO:17738, SEQ ID NO:17741, SEQ ID
NO:17743, SEQ ID NO:17745, SEQ ID NO:17749, SEQ ID NO:17752, SEQ ID NO:17755,
SEQ ID NO:17757, SEQ ID NO:17760, SEQ ID NO:17766, SEQ ID NO:17772, SEQ ID
NO:17775, SEQ ID NO:17777, SEQ ID NO:17779, SEQ ID NO:17782, SEQ ID NO:17784,
SEQ ID NO:17786, SEQ ID NO:17788, SEQ ID NO:17791, SEQ ID NO:17793, SEQ ID
NO:17796, SEQ ID NO:17798, SEQ ID NO:17800, SEQ ID NO:17804, SEQ ID NO:17808,
SEQ ID NO:17810, SEQ ID NO:17812, SEQ ID NO:17814, SEQ ID NO:17816, SEQ ID
NO:17819, SEQ ID NO:17821, SEQ ID NO:17823, SEQ ID NO:17825, SEQ ID NO:17827,
SEQ ID NO:17829, SEQ ID NO:17833, SEQ ID NO:17835, SEQ ID NO:17838, SEQ ID
NO:17840, SEQ ID NO:17842, SEQ ID NO:17844, SEQ ID NO:17846, SEQ ID NO:17854,
SEQ ID NO:17857, SEQ ID NO:17862, SEQ ID NO:17864, SEQ ID NO:17866, SEQ ID
NO:17868, SEQ ID NO:17873, SEQ ID NO:17879, SEQ ID NO:17882, SEQ ID NO:17884,
SEQ ID NO:17887, SEQ ID NO:17889, SEQ ID NO:17891, SEQ ID NO:17893, SEQ ID
NO:17897, SEQ ID NO:17899, SEQ ID NO:17901, SEQ ID NO:17905, SEQ ID NO:17908,
SEQ ID NO:17910, SEQ ID NO:17913, SEQ ID NO:17915, SEQ ID NO:17921, SEQ ID
NO:17923, SEQ ID NO:17927, SEQ ID NO:17930, SEQ ID NO:17933, SEQ ID NO:17935,
SEQ ID NO:17937, SEQ ID NO:17939, SEQ ID NO:17944, SEQ ID NO:17948, SEQ ID
NO:17950, SEQ ID NO:17953, SEQ ID NO:17955, SEQ ID NO:17957, SEQ ID NO:17960,
SEQ ID NO:17963, SEQ ID NO:17968, SEQ ID NO:17970, SEQ ID NO:17972, SEQ ID
NO:17975, SEQ ID NO:17977, SEQ ID NO:17983, SEQ ID NO:17988, SEQ ID NO:17992,
SEQ ID NO:17994, SEQ ID NO:17996, SEQ ID NO:17998, SEQ ID NO:18002, SEQ ID
NO:18007, SEQ ID NO:18009, SEQ ID NO:18011, SEQ ID NO:18014, SEQ ID NO:18018,
SEQ ID NO:18020,
SEQ ID NO:18023, SEQ ID NO:18025, SEQ ID NO:18027, SEQ ID NO:18029, SEQ ID
NO:18031, SEQ ID NO:18033, SEQ ID NO:18035, SEQ ID NO:18037, SEQ ID NO:18039,
SEQ ID NO:18041, SEQ ID NO:18047, SEQ ID NO:18049, SEQ ID NO:18051, SEQ ID
NO:18053, SEQ ID NO:18055, SEQ ID NO:18057, SEQ ID NO:18060, SEQ ID NO:18062,
SEQ ID NO:18064, SEQ ID NO:18067, SEQ ID NO:18069, SEQ ID NO:18073, SEQ ID
NO:18075, SEQ ID NO:18077, SEQ ID NO:18079, SEQ ID NO:18081, SEQ ID NO:18083,
SEQ ID NO:18085, SEQ ID NO:18087, SEQ ID NO:18089, SEQ ID NO:18091, SEQ ID
NO:18093, SEQ ID NO:18097, SEQ ID NO:18100, SEQ ID NO:18102, SEQ ID NO:18106,
SEQ ID NO:18108, SEQ ID NO:18111, SEQ ID NO:18113, SEQ ID NO:18115, SEQ ID
 
NO:18118, SEQ ID NO:18122, SEQ ID NO:18126, SEQ ID NO:18128, SEQ ID NO:18130,
SEQ ID NO:18132, SEQ ID NO:18134, SEQ ID NO:18136, SEQ ID NO:18138, SEQ ID
NO:18141, SEQ ID NO:18143, SEQ ID NO:18145, SEQ ID NO:18147, SEQ ID NO:18149,
SEQ ID NO:18155, SEQ ID NO:18158, SEQ ID NO:18160, SEQ ID NO:18164, SEQ ID
NO:18167, SEQ ID NO:18169, SEQ ID NO:18171, SEQ ID NO:18173, SEQ ID NO:18175,
SEQ ID NO:18177, SEQ ID NO:18179, SEQ ID NO:18181, SEQ ID NO:18183, SEQ ID
NO:18185, SEQ ID NO:18187, SEQ ID NO:18189, SEQ ID NO:18191, SEQ ID NO:18193,
SEQ ID NO:18195, SEQ ID NO:18197, SEQ ID NO:18199, SEQ ID NO:18202, SEQ ID
NO:18204, SEQ ID NO:18206, SEQ ID NO:18208, SEQ ID NO:18211, SEQ ID NO:18213,
SEQ ID NO:18215, SEQ ID NO:18217, SEQ ID NO:18219, SEQ ID NO:18221, SEQ ID
NO:18223, SEQ ID NO:18225, SEQ ID NO:18228, SEQ ID NO:18230, SEQ ID NO:18232,
SEQ ID NO:18234, SEQ ID NO:18237, SEQ ID NO:18239, SEQ ID NO:18241, SEQ ID
NO:18244, SEQ ID NO:18246, SEQ ID NO:18248, SEQ ID NO:18250, SEQ ID NO:18252,
SEQ ID NO:18255,
SEQ ID NO:18257, SEQ ID NO:18262, SEQ ID NO:18267, SEQ ID NO:18269, SEQ ID
NO:18271, SEQ ID NO:18273, SEQ ID NO:18275, SEQ ID NO:18277, SEQ ID NO:18279,
SEQ ID NO:18281, SEQ ID NO:18283, SEQ ID NO:18285, SEQ ID NO:18287, SEQ ID
NO:18290, SEQ ID NO:18292, SEQ ID NO:18294, SEQ ID NO:18296, SEQ ID NO:18299,
SEQ ID NO:18301, SEQ ID NO:18304, SEQ ID NO:18306, SEQ ID NO:18311, SEQ ID
NO:18313, SEQ ID NO:18315, SEQ ID NO:18317, SEQ ID NO:18319, SEQ ID NO:18322,
SEQ ID NO:18324, SEQ ID NO:18326, SEQ ID NO:18328, SEQ ID NO:18330, SEQ ID
NO:18333, SEQ ID NO:18336, SEQ ID NO:18338, SEQ ID NO:18340, SEQ ID NO:18342,
SEQ ID NO:18344, SEQ ID NO:18346, SEQ ID NO:18348, SEQ ID NO:18350, SEQ ID
NO:18352, SEQ ID NO:18354, SEQ ID NO:18356, SEQ ID NO:18359, SEQ ID NO:18361,
SEQ ID NO:18363, SEQ ID NO:18365, SEQ ID NO:18367, SEQ ID NO:18369, SEQ ID
NO:18371, SEQ ID NO:18373
SEQ ID NO:18376, SEQ ID NO:18380, SEQ ID NO:18382, SEQ ID NO:18384, SEQ ID
NO:18386, SEQ ID NO:18388, SEQ ID NO:18391, SEQ ID NO:18393, SEQ ID NO:18396,
SEQ ID NO:18400, SEQ ID NO:18403, SEQ ID NO:18405, SEQ ID NO:18407, SEQ ID
NO:18409, SEQ ID NO:18411, SEQ ID NO:18415, SEQ ID NO:18417, SEQ ID NO:18419,
SEQ ID NO:18423, SEQ ID NO:18425, SEQ ID NO:18429, SEQ ID NO:18431, SEQ ID
NO:18434, SEQ ID NO:18436, SEQ ID NO:18438, SEQ ID NO:18440, SEQ ID NO:18442,
SEQ ID NO:18445, SEQ ID NO:18448, SEQ ID NO:18450, SEQ ID NO:18452, SEQ ID
NO:18455, SEQ ID NO:18457, SEQ ID NO:18459, SEQ ID NO:18461, SEQ ID NO:18463,
SEQ ID NO:18466, SEQ ID NO:18469, SEQ ID NO:18471, SEQ ID NO:18473, SEQ ID
NO:18475, SEQ ID NO:18477, SEQ ID NO:18479, SEQ ID NO:18481, SEQ ID NO:18483,
SEQ ID NO:18485, SEQ ID NO:18487, SEQ ID NO:18490, SEQ ID NO:18492, SEQ ID
NO:18494, SEQ ID NO:18498, SEQ ID NO:18506, SEQ ID NO:18508, SEQ ID NO:18510,
SEQ ID NO:18512, SEQ ID NO:18514, SEQ ID NO:18516, SEQ ID NO:18518, SEQ ID
NO:18520, SEQ ID NO:18522, SEQ ID NO:18524, SEQ ID NO:18526, SEQ ID NO:18529,
SEQ ID NO:18532, SEQ ID NO:18536, SEQ ID NO:18538, SEQ ID NO:18541, SEQ ID
NO:18546, SEQ ID NO:18550, SEQ ID NO:18552, SEQ ID NO:18554, SEQ ID NO:18556,
SEQ ID NO:18558, SEQ ID NO:18562, SEQ ID NO:18568, SEQ ID NO:18572, SEQ ID
NO:18574, SEQ ID NO:18576, SEQ ID NO:18580, SEQ ID NO:18582, SEQ ID NO:18587,
SEQ ID NO:18590, SEQ ID NO:18592, SEQ ID NO:18594, SEQ ID NO:18596, SEQ ID
NO:18598, SEQ ID NO:18600, SEQ ID NO:18602, SEQ ID NO:18604, SEQ ID NO:18606,
SEQ ID NO:18608, SEQ ID NO:18611, SEQ ID NO:18613, SEQ ID NO:18615, SEQ ID
NO:18617, SEQ ID NO:18619, SEQ ID NO:18623, SEQ ID NO:18625, SEQ ID NO:18627,
SEQ ID NO:18629,
SEQ ID NO:18631, SEQ ID NO:18633, SEQ ID NO:18635, SEQ ID NO:18637, SEQ ID
NO:18639, SEQ ID NO:18644, SEQ ID NO:18646, SEQ ID NO:18649, SEQ ID NO:18651,
SEQ ID NO:18653, SEQ ID NO:18655, SEQ ID NO:18657, SEQ ID NO:18660, SEQ ID
 
NO:18662, SEQ ID NO:18665, SEQ ID NO:18668, SEQ ID NO:18672, SEQ ID NO:18674,
SEQ ID NO:18676, SEQ ID NO:18678, SEQ ID NO:18680, SEQ ID NO:18682, SEQ ID
NO:18686, SEQ ID NO:18688, SEQ ID NO:18691, SEQ ID NO:18693, SEQ ID NO:18695,
SEQ ID NO:18697, SEQ ID NO:18699, SEQ ID NO:18701, SEQ ID NO:18703, SEQ ID
NO:18705, SEQ ID NO:18709, SEQ ID NO:18711, SEQ ID NO:18713, SEQ ID NO:18715,
SEQ ID NO:18717, SEQ ID NO:18720, SEQ ID NO:18722, SEQ ID NO:18724, SEQ ID
NO:18726, SEQ ID NO:18729, SEQ ID NO:18731, SEQ ID NO:18733, SEQ ID NO:18735,
SEQ ID NO:18737, SEQ ID NO:18739, SEQ ID NO:18741, SEQ ID NO:18743, SEQ ID
NO:18745, SEQ ID NO:18747, SEQ ID NO:18749, SEQ ID NO:18751, SEQ ID NO:18753,
SEQ ID NO:18759, SEQ ID NO:18763, SEQ ID NO:18765, SEQ ID NO:18770, SEQ ID
NO:18773, SEQ ID NO:18775, SEQ ID NO:18777, SEQ ID NO:18779, SEQ ID NO:18781,
SEQ ID NO:18783, SEQ ID NO:18785, SEQ ID NO:18787, SEQ ID NO:18790, SEQ ID
NO:18793, SEQ ID NO:18795, SEQ ID NO:18797, SEQ ID NO:18800, SEQ ID NO:18802,
SEQ ID NO:18804, SEQ ID NO:18806, SEQ ID NO:18809, SEQ ID NO:18811, SEQ ID
NO:18813, SEQ ID NO:18815, SEQ ID NO:18817, SEQ ID NO:18819, SEQ ID NO:18822,
SEQ ID NO:18824, SEQ ID NO:18826, SEQ ID NO:18828, SEQ ID NO:18830, SEQ ID
NO:18832, SEQ ID NO:18834, SEQ ID NO:18836, SEQ ID NO:18840, SEQ ID NO:18843,
SEQ ID NO:18847, SEQ ID NO:18850, SEQ ID NO:18853, SEQ ID NO:18855, SEQ ID
NO:18857, SEQ ID NO:18859, SEQ ID NO:18862, SEQ ID NO:18865, SEQ ID NO:18868,
SEQ ID NO:18870,
SEQ ID NO:18874, SEQ ID NO:18876, SEQ ID NO:18879, SEQ ID NO:18882, SEQ ID
NO:18884, SEQ ID NO:18888, SEQ ID NO:18891, SEQ ID NO:18894, SEQ ID NO:18896,
SEQ ID NO:18898, SEQ ID NO:18900, SEQ ID NO:18902, SEQ ID NO:18906, SEQ ID
NO:18908, SEQ ID NO:18910, SEQ ID NO:18912, SEQ ID NO:18914, SEQ ID NO:18916,
SEQ ID NO:18918, SEQ ID NO:18920, SEQ ID NO:18922, SEQ ID NO:18924, SEQ ID
NO:18926, SEQ ID NO:18929, SEQ ID NO:18931, SEQ ID NO:18933, SEQ ID NO:18935,
SEQ ID NO:18937, SEQ ID NO:18939, SEQ ID NO:18942, SEQ ID NO:18945, SEQ ID
NO:18948, SEQ ID NO:18950, SEQ ID NO:18952, SEQ ID NO:18954, SEQ ID NO:18961,
SEQ ID NO:18963, SEQ ID NO:18965, SEQ ID NO:18967, SEQ ID NO:18971, SEQ ID
NO:18974, SEQ ID NO:18977, SEQ ID NO:18979,
SEQ ID NO:18991, SEQ ID NO:18994, SEQ ID NO:18996, SEQ ID NO:19003, SEQ ID
NO:19005
SEQ ID NO:19008, SEQ ID NO:19010, SEQ ID NO:19015, SEQ ID NO:19017, SEQ ID
NO:19019, SEQ ID NO:19021, and SEQ ID NO:19023
(c) a polynucleotide comprising a nucleotide sequence encoding a protein comprising an amino acid sequence selected from the amino acid sequences of (b), in which one or more amino acids are substituted, deleted, inserted, and/or added, wherein said protein is functionally equivalent to the protein comprising said amino acid sequence selected from the amino acid sequences of (b);
(d) a polynucleotide that hybridizes with a polynucleotide comprising a nucleotide sequence selected from the nucleotide sequences of (a), and that comprises a nucleotide sequence encoding a protein functionally equivalent to the protein encoded by the nucleotide sequence selected from the nucleotide sequences of (a);
(e) a polynucleotide comprising a nucleotide sequence encoding a partial amino acid sequence of a protein encoded by the polynucleotide of (a) to (d);
(f) a polynucleotide comprising a nucleotide sequence with at least 70% identity to the nucleotide sequence of (a).

9. A substantially pure protein encoded by the polynucleotide of claim 8.

10. An antibody against the protein or peptide of any one of claims 6, 7, and 9.

11. A vector comprising the polynucleotide of claim 5 or 8.

12. A transformant carrying the polynucleotide of claim 5 or 8, or the vector of claim 11.

13. A transformant expressively carrying the polynucleotide of claim 5 or 8, or the vector of claim 11.

14. A method for producing the protein or peptide of any one of claims 6, 7, and 9, comprising culturing the transformant of claim 13 and recovering the expression product.

15. An oligonucleotide comprising the nucleotide sequence of claim 8 (a) or the nucleotide sequence complementary to the complementary strand thereof, wherein said oligonucleotide comprises 15 nucleotides or more.

16. Use of the oligonucleotide of claim 15 as a primer for synthesizing a polynucleotide.

17. Use of the oligonucleotide of claim 15 as a probe for detecting a gene.

18. An antisense polynucleotide against the polynucleotide of claim 8, or the portion thereof.

19. A method for synthesizing a polynucleotide, the method comprising:
a) synthesizing a complementary strand using a cDNA library as a template, and using the primer set of claim 2 or 3, or the primer of claim 16; and
b) recovering the synthesized product.

20. The method of claim 19, wherein the cDNA library is obtainable by oligo-capping method.

21. The method of claim 19, wherein the complementary strand is obtainable by PCR.

22. A method for detecting the polynucleotide of claim 8, the method comprising:
a) incubating a target polynucleotide with the oligonucleotide of claim 15 under the conditions where hybridization occurs, and
b) detecting the hybridization of the target polynucleotide with the oligonucleotide of claim 15.

23. A database of polynucleotides and/or proteins, the database comprising information on at least one sequence selected from the nucleotide sequences of claim 8 (a) and/or the amino acid sequences of claim 8 (b), or a medium on which the database is stored.
